(19) Europäisches Patentamt
European Patent Office
Office européen des brevets

(11) Publication number: 0 679 716 A1

(12) **EUROPEAN PATENT APPLICATION**
**published in accordance with Art. 158(3) EPC**

(21) Application number: **95900295.7**

(22) Date of filing: **11.11.94**

(86) International application number:
**PCT/JP94/01916**

(87) International publication number:
**WO 95/14772 (01.06.95 95/23)**

(51) Int. Cl.[6]: **C12N 15/11**, C12Q 1/68, //G01N33/566

(30) Priority: **12.11.93 JP 355504/93**

(43) Date of publication of application:
**02.11.95 Bulletin 95/44**

(84) Designated Contracting States:
**AT BE CH DE DK ES FR GB GR IE IT LI LU MC NL PT SE**

(71) Applicant: **Matsubara, Kenichi**
**Room 804, 18-1, Yamadahi-gashi 3-chome**
**Suita-shi,**
**Osaka 565 (JP)**
Applicant: **Okubo, Kousaku**
**11-26, Segawa 2-chome**
**Minoo-shi,**
**Osaka 562 (JP)**

(72) Inventor: **Matsubara, Kenichi**
**Room 804, 18-1, Yamadahi-gashi 3-chome**
**Suita-shi,**
**Osaka 565 (JP)**
Inventor: **Okubo, Kousaku**
**11-26, Segawa 2-chome**
**Minoo-shi,**
**Osaka 562 (JP)**

(74) Representative: **Vossius, Tilman et al**
**Dr. Volker Vossius,**
**Patent- und Rechtsanwaltskanzlei,**
**Holbeinstrasse 5**
**D-81679 München (DE)**

(54) **GENE SIGNATURE.**

(57) A 3'-directed cDNA library which accurately reflects the abundance ration of mRNA in a cell has been prepared from various human tissues, and sequencing of the cDNAs contained in the library has be conducted to examine the incidence of each cDNA in each tissue. As each cDNA has expression information with each tissue corresponding to the mRNA concentration, these cDNAs are usable as a probe or primer for detecting cell anomoly or discriminating cells. The cloned gene can produce porteins utilizable as a medicine or the like.

EP 0 679 716 A1

## Fields of the Invention

The present invention relates to purified single-stranded DNA molecules, purified single-stranded DNA molecules complementary thereto or purified double-stranded DNA molecules consisting of said single-stranded DNA molecules, which can specifically hybridize to human genomic DNA, human cDNA or human mRNA at particular sites. The DNA molecules of the present invention can be used for detecting the overall or individual expression status of mRNAs coding for the corresponding cellular proteins, detecting and diagnosing cellular abnormalities due to disease and viral infection, or distinguishing and identifying the cell type, and efficiently cloning genes expressed in a tissue-specific manner. The present invention further includes cloned DNA molecules which can be used to produce proteins useful as pharmaceutical products or the like.

## Related Arts

Recognizing the importance of the most fundamental attribute of mRNA, that is, "the nature of the cell is determined by the expression pattern of genes as reflected in the population of mRNA", the inventors of the present invention have proposed "body mapping" as a unique approach to their objective. This is an entirely novel attempt to prepare "the information on gene expression" for presumably about 200 different kinds of cells and tissues present in the human body and elucidate when, where and to what extent a certain gene is expressed, and map genes to the respective organ or cell type in which they are expressed.

While a variety of cells in the living body express various proteins depending on their respective biological functions, the intracellular concentrations of these proteins vary according to the cell type, stage of development and differentiation, environment, etc.

In general, genes are classified into "genes encoding proteins essential for the life of the cell" and "genes encoding proteins responsible for functions specific to the cell". Of these two, "genes encoding proteins essential for the life of the cell" are expressed constantly in all types of cells and also called "housekeeping genes", while "genes encoding proteins responsible for functions specific to the cell" are often expressed specifically in a particular type of cells or a particular group of cells, and also may be specifically expressed at a particular stage of cellular development and differentiation. Furthermore, they are often "inducible genes" and the amount of their expression varies depending upon the environment to which cells are exposed. In other words, cells may grow as a result of the expression of "genes encoding proteins essential for the life of the cell" and display their specific functions as a result of the expression of "genes encoding proteins responsible for functions specific to the cell".

However, under abnormal cellular conditions due to disease or infection, the expression of genes within individual cells is altered as compared with that under the normal conditions. Especially, during viral infection, RNAs encoding virus-specific proteins are synthesized in large amounts within the cell, leading to the production of said protein in large amounts. In other words, the alteration in the expression level of genes within the cell, especially as reflected in the concentration of intracellular mRNA, can lead to such abnormal cellular conditions as seen in diseases.

Thus, the function of each cell in the living body is closely related to the expression status of genes within the cell. Accordingly, in order to elucidate the function of each cell at molecular level or to investigate the pathogenesis of a disease at molecular level, it becomes necessary to comprehend the expression status of cellular genes, especially the intracellular concentration of each mRNA.

A theoretically possible approach to this objective is the extraction and analysis of all cellular proteins for determination of expression status. However, although it may be possible to isolate a specific protein, in most cases it is almost impossible to completely isolate all of these proteins, because a great variety of proteins are expressed within the cell.

Another approach is to directly estimate the concentrations of cellular mRNAs corresponding to all intracellular proteins. However, although it may be possible to isolate a specific mRNA, it is practically impossible to completely isolate all of these mRNAs and directly estimate their amounts, because a great variety of mRNAs are synthesized simultaneously within the cell and furthermore they may be unstable and susceptible to enzymatic degradation during their extraction.

This invention aims to provide DNA molecules which can be used as probes or primers required for detecting the overall or individual expression status of mRNAs coding for the corresponding cellular proteins, detecting or diagnosing cellular abnormalities due to disease or virus infection, recognizing and identifying various cell types, and efficiently cloning genes expressed in a tissue-specific manner. Moreover, the present invention aims to provide cloned DNA molecules which can be used to produce proteins useful as pharmaceutical products.

Summary of the invention

In general, the genetic information flows in order from DNA to mRNA and to protein (F. H. C. Crick, 1958). That is, "the information for the amino acid sequence of a protein" is first transcribed into mRNA and then translated into protein.

To explain this in further detail mammalian genes commonly comprise a region encoding a protein and a region regulating the expression of said gene. The regions of a gene encoding protein (called "exons") are often separated by intervening sequences (called "introns"). When a gene is transcribed into RNA, the introns of the precursor RNA (pre-mRNA) are excised and exons are connected in tandem to form a contiguous structure coding for a particular protein (this process is called "splicing"). On the other hand, the region regulating the expression of gene comprises, in addition to the regions directly regulating transcription such as a promoter and operator which are present upstream of the transcription region, untranslated regions are located both upstream (5') and downstream (3') of the coding region. In particular, 3' untranslated region (3' UTR) is important for regulating expression, since it contributes to the transport and stability of mRNA. During the processing of pre-mRNA, a methylated cap is added at its 5' end, the 3' untranslated region is cleaved at a specific site, a poly(A) tail is attached by adding 100 - 200 adenylate residues to the cleaved end, and the coding regions are spliced together to form mRNA. The protein is then synthesized after attachment of ribosomes to the mRNA.

The inventors of the present invention have elucidated that, in general, when the intracellular level of a particular mRNA is high, the expressed amount of the corresponding protein is also elevated, and also that it is possible to estimate the relative concentration of each intracellular protein by estimating relative intracellular concentration of the corresponding mRNA [DNA sequence 2, 137-144 (1991); Nature genetics, 2, 173-179 (1992)].

Basically in the present invention, mRNA is extracted from a particular cell and cDNA is synthesized by conventional methods using reverse transcriptase. However, in the present invention, cDNA is synthesized using a method developed by the inventors of the present invention so as to reflect the relative intracellular concentration of mRNA. A cDNA library is constructed and a group of cDNAs representing the population of total mRNA are cloned and sequenced.

An approach which appears to be similar to the one used by the inventors of the present invention but is entirely different, is the method of cloning of a cDNA library constructed by the random priming by Venter et al.

Venter's group randomly cloned cDNAs from commercially available cDNA libraries derived from brain cells (catalog No. 936206, 936205 or 935, Stratagene, California) and determined their base sequences [Science 252, 1651-1656 (1991); Nature 355, 632-634 (1992)].

While the method used by Venter et al. involves sequencing of cDNAs obtained by random priming, this method has the following drawbacks:

1) Since random cloning of various regions of a single-stranded mRNA may often lead to the formation of many cDNA fragments without any mutual overlapping portions, it is difficult to determine whether these cDNA fragments are derived from the same mRNA or a different one,

2) The longer a mRNA strand, the higher the chance for said mRNA to be reverse-transcribed into cDNA, and

3) Since the availability of each primer to be used among random primers differs depending on their base sequences, the relative frequency of cDNA synthesis is variable.

From aforementioned reasons, the relative frequency of appearance of cDNA does not reflect the relative concentration of cellular mRNA. Consequently, it is impossible to determine the relative concentration of each mRNA and the actual population of intracellular proteins by using the method of Venter et al.

However, with the method developed by the inventor of the present invention, it is possible to construct a cDNA library which precisely reflects the relative concentration of mRNA without any of the aforementioned complications. Since, in the present invention, cDNA is synthesized using only "poly-T" as the primer, the 3' ends of the cDNA have "a poly A tail". Therefore, the synthesis of cDNA with "poly-T" as the sole primer is initiated from the 3' end resulting in the formation of 3'-oriented cDNA. Since the 3' untranslated sequence is unique to a particular mRNA species and not present in other mRNA species [Birnsteil, M. L., et al., Cell 41, 349-359 (1985)], almost all the 3' end-oriented cDNAs hybridize with specific mRNAs. Digestion of the resulting cDNA with a restriction enzyme MboI which recognizes the specific four-base sequence GATC results in the formation of cDNA extending from the 3'-terminus to the first MboI restriction site. In the present invention, each cDNA thus cloned and included in "a cDNA library faithfully reflecting the relative intracellular concentration of mRNA" is called a "gene signature" (abbreviated as GS hereinafter). A GS includes not only the double-stranded DNA but also each single-stranded DNA thereof.

The present invention relates to a purified single-stranded DNA, purified single-stranded DNA complementary thereto, or a purified double-stranded DNA consisting of said single strands, containing all or a portion of a single-stranded DNA (or a single-stranded DNA complementary thereto) comprising any of the base sequences listed under the sequence identification number (SEQ ID NO) 1 - 7837 and hybridizing specifically to a particular site of human genomic DNA, human cDNA or human mRNA. The present invention also relates to probes and primers consisting of said single-stranded DNA. The present invention also relates to a purified single-stranded DNA, a purified single-stranded DNA complementary thereto, or a purified double-stranded DNA consisting of said single strands, containing all or a portion of a single-stranded DNA (or a single-stranded DNA complementary thereto) which is complementary to a human mRNA containing any of the base sequences listed under SEQ ID NO 1 - 7837 ( wherein T is read as U ) or any portion thereof at its 3' region and hybridizing specifically to a particular site of human genomic DNA, human cDNA or human mRNA. The present invention also relates to probes and primers consisting of said single-stranded DNA.

The present invention is explained further in detail as follows.

The DNA of the present invention not only includes a single-stranded DNA (or a single-stranded DNA complementary thereto) comprising any of the base sequences listed under SEQ ID NO 1 - 7837 but also includes a single-stranded DNA containing a portion of said single-stranded DNA (or said single-stranded DNA complementary thereto) if it hybridizes to human genomic DNA, human cDNA or human mRNA.

Furthermore, the DNA of the present invention not only includes a single-stranded DNA (or a single-stranded DNA complementary thereto) which is complementary to a mRNA containing any of the base sequences listed under SEQ ID NO 1- 7837 (wherein T is read as U) or any portion thereof at its 3' region but also includes a single-stranded DNA (or a single-stranded DNA complementary thereto) containing a portion of said single-stranded DNA (or said single-stranded DNA complementary thereto) if it hybridizes to human genomic DNA, human cDNA or human mRNA.

In addition, the DNA of the present invention not only includes a single-stranded DNA or a single-stranded DNA complementary thereto but also includes a double-stranded DNA consisting of said single strands.

Obviously, the term "contain" as used herein does not necessarily mean that the DNA of the present invention contains at a single site without interruption (1) "a single-stranded DNA (or a single-stranded DNA complementary thereto) comprising any of the base sequences listed under SEQ ID NO 1-7837 or a portion thereof" or (2) "a single-stranded DNA (or a single-stranded DNA complementary thereto) which is complementary to a mRNA containing any or any portion of the base sequences listed under SEQ ID NO 1 - 7837 (wherein T is read as U) at its 3' region or a portion of said single-stranded DNA." In other words, the term "contain" is applicable also to the case where one or more exogenous bases are inserted in the base sequence of the DNA (1) or (2).

The hybridization to a particular site of human genomic DNA, human cDNA or human mRNA can be achieved under standard conditions (see e.g., ,Molecular Cloning: A Laboratory Manual, Sambrook, J., et al., Cold Spring Harbor Laboratory Press, 1989). In the following preferred embodiment, there will be described methods for constructing a cDNA library which reflects precisely the relative intracellular concentration of mRNA, cloning cDNA groups which correspond to total mRNA, and determining the base sequence of each cDNA.

First, cells from specific tissues, for example, cells from organs, for example, cells derived from human liver (HepG2) are grown, and the total mRNA is extracted by standard procedures. mRNA thus obtained is attached to a vector to construct a cDNA library.

For example, mRNA is attached to the vector plasmid pUC19, which has the M13 sequences flanking the cloning site, as follows.

pUC19 is cleaved by HincII and PstI and poly-T of 20 bp - 30 bp is added to the PstI-digested end to which the 3'-end poly-A tail of the mRNA is hybridized (Fig. 1a). After the DNA strand is extended with conventional methods using reverse transcriptase, a double stranded DNA is formed with DNA polymerase (Fig. 1b). The double stranded DNA thus obtained is cleaved with the restriction enzyme MboI which recognizes a specific four base sequence (Fig. 1c).

MboI, which recognizes a four base sequence (GATC), cleaves the DNA within a few hundred bases from the poly-A tail. Since MboI is found to digest, without exception, about 300 human cDNAs which were randomly selected from the GenBank data base by the inventor of the present invention, this enzyme cleaves the cDNA to be cloned at a specific site. In addition, as pUC19 is prepared in $dam^+$ E. coli, e.g., E. coli JM109 and since its adenine at the MboI recognition site is methylated ($G^mATC$), it is not cleaved by MboI.

Subsequently, in order to prepare a vector containing the double-stranded DNA which has previously been attached to pUC19 and has the MboI-cleaved end, the pUC19 DNA is digested with BamHI to make termini cohesive with the MboI-cleaved end. Since the recognition sequence of BamHI (GGATCC) contains that of MboI (GATC), the extended portion of the double-stranded DNA is not cleaved with BamHI.

The resulting double-stranded DNA is then circularized by standard ligation methods, and the recombinant vector plasmid thus prepared is introduced into E. coli, e.g., E. coli DH5 in order to make a cDNA library.

With this method, only a clone containing the base sequence upstream of the poly-A tail of the mRNA is obtained.

Since the average size of the inserted cDNA fragment is relatively small, 270 bp, it is free from biased cloning resulting from variations in the efficiency of cDNA synthesis and transformation that occur in the case of larger sized DNAs. Furthermore, because instability due to repeated base sequences and the like is eliminated, the cDNA library of the present invention faithfully represents the relative concentration of mRNA in the cell.

Furthermore, when the cDNA inserted into the vector is relatively short, it is possible to accurately amplify the cDNA fragment using the sequence of the vector flanking it as a primer. It is also possible to determine the base sequence from the 5' end directly by the PCR without interference from the 3' poly-A tail which will reduce the accuracy of sequence determination.

Amplification of the GS, i.e., the cDNA fragment inserted into the vector, is performed as follows.

The E. coli cells in which the cDNA library is introduced are grown using standard methods and lysed. Debris contained in the bacterial lysate are removed by centrifugation and the supernatant containing the vector DNA is recovered. The vector DNA thus obtained is used as the DNA template for amplification by the PCR (Fig. 1d, amplification with PCR primers 1 and 2).

Base sequences flanking both ends of the GS is properly selected for use as primers and the PCR is performed under standard conditions. PCR products thus obtained are subjected to the elongation reaction using fluorescence primers complementary to the vector sequence flanking the 5' end of the GS, and the sequence is determined with an autosequencer (Fig. 1d, sequence determination with dye primer).

Based on the results of the sequence determination of each GS, the species and the frequency of appearance of the GS in each tissue or cell type are analyzed.

As to each cell type not only normal cells but also cells under pathogenic conditions (such as tumor cells, virus infected cells, etc.) can be used without any restriction. For example, liver cells (from fetus, neonate or adult), various hematopoietic cells (granulocytic, monocytic, etc.), lung cells, adipocytes, endothelial cells, osteoblasts, colon mucosa cells, retinal cells and hepatoma cells (HepG2, etc.), and promyelocytic leukemia cells (HL60, etc.) will be used. The appearance frequency for each GS is described for each cell type in Tables 1 through 219. There, patent number represents "SEQ ID NO for each GS", size represents the "length of each GS", and F represents the "sum of appearance frequencies in the cells studied". In addition, hepG2 stands for "hepG2 (a liver cancer cell line)", HL60 stands for "HL60 promyelocytic leukemia cell line", granulo stands for "granulocytoid, HL60 stimulated by DMSO", mono stands for "monocytoids, HL60 stimulated by TPA", 40 w liver stands for "40 w neonatal liver", 19 w liver stands for "liver of a 19 weeks old fetus, adult liver is "adult liver ", lung stands for "adult lung", adipose stands for "subcutaneous adipose tissue", endothel stands for "primary cultured aortic endothelium", osteoblast stands for "primary cultured osteoblast", colon mucosa is "colon mucosa", small cell carci stands for "small cell carcinoma of lung", retina is "retina", cerebral cortex is "cerebral cortex", adenocarci (lung) stands for "adenocarcinoma of lung", squamous cell ca (lung) stands for "squamous cell carcinoma of lung", keratinocyte stands for "primary cultured keratinocyte", fibroblast stands for "primary cultured fibroblast", Alzheimer stands for "Alzheimer temporal lobe", cerebellum stands for "cerebellum", visceral fat is "visceral fat", corneal epithelium is "corneal epithelium", peripheral granulocyte is "peripheral granulocyte", neuroblastoma is "neuroblastoma" and taste bud of tongue is "taste bud of tongue".

"Accession number of target mRNA" represents the accession number of the entry in GenBank Release 79 whose base sequence has homology with that of each GS, "match %" represents the percent homology of the GS sequence relative to that of said homologous sequence, "match starts at (GS)" represents the base position counted from the 5'-end of the GS at which the region for homology calculation starts, "match starts at (GenBank)" represents the base position counted from the 5'-end of the GenBank sequence at which the region for homology calculation starts, and "GenBank target size" represents the whole length of the GenBank sequence corresponding to the GS. The columns in Tables 1 - 219 represent the same items as in Table 1.

Based on the data in Tables 1 - 219, each GS can be classified into several groups. A GS, which is expressed at high frequency in a specific cell or groups of cells with similar property, for example, promyelocytic leukemia cell, granulocyte and monocyte and not expressed entirely or expressed very little in other cells (groups), is a likely GS corresponding to the gene encoding "the protein responsible for functions specific to the cell" (e.g., GS0001553, GS0002047, GS004895, etc.). On the other hand, a GS, which is expressed commonly in every kind of cell, most likely corresponds to the gene encoding "the protein essential for the life of the cell" (e.g., GS0000019, GS0000155, GS000861, etc.). In addition, some GSs are expressed at low frequency (e.g., GS0000013, GS0002399, GS0003155, etc.).

Since the GS with the sequence determined as described above will reflect the population of mRNA expressed in a particular cell, it must be possible to find the relative concentration of mRNA in each cell by determining the appearance frequency for each GS in a cDNA library derived from that cell. Therefore, to confirm the correlation between the appearance frequency for each GS in a cDNA library and the relative concentration of cellular mRNA, the GS thus obtained was labeled with $^{32}P$ by standard methods and used as the probe in the following hybridization test. mRNA isolated from a specific cell is hybridized to said $^{32}P$-labeled probe under standard conditions. The results of this Northern hybridization test were such that, when a GS found with high appearance frequency in a cDNA library was used as a probe, a dense band was formed, confirming the correlation of the frequency of appearance of the GS with the relative concentration of mRNA in the cell (see Example 5).

Similarly, the colony hybridization test of the cDNA library constructed as described above with a $^{32}P$-labeled probe prepared as described above showed a close correlation between the frequency of appearance of the GS and the number of colonies hybridized with said GS (see Example 6), confirming the correspondence of the frequency of appearance of the GS and relative concentration of the GS in a cDNA library.

From the above results, by determining the appearance frequency of each GS in a cDNA library derived from a variety of cells, it has become possible to determine the expression status of the gene (or mRNA) corresponding to each GS. This fact implies conversely that each GS may be useful for industrial purposes as a specific probe or primer encoding information about the expression status of its corresponding gene (or mRNA) for each cell. For example, when it is proven that "a certain GS appears at high frequency only in a cDNA library derived from tissue A, that is, the gene corresponding to said GS is specifically expressed only in tissue A", by conventional cloning of the corresponding full-length cDNA using said GS as a probe or primer, it is possible to clone a full-length gene which is expressed in a tissue-specific manner.

Furthermore, for example, when it is proven that "the frequency of appearance of a certain GS is low in a cDNA library derived from tissue B, that is, the appearance frequency of the gene corresponding to said GS is low in tissue B", by examining the expression frequency of the gene corresponding to said GS in a test sample of tissue B from a patient using said GS as a probe or primer, it may be possible to identify the pathogenic gene, wherein an unusually high expression frequency of said gene being a strong indication that said GS may be the gene involved in the pathogenesis. Furthermore, by conventional methods for cloning said full-length cDNA using said GS as a probe or primer, it is possible to isolate said pathogenic gene and elucidate its characteristics.

In practice, the DNA of the present invention may be used as a probe or primer for detecting and diagnosing disease, cloning a pathogenic gene or related gene, cloning a viral gene, identifying and recognizing cell types, cloning a species-specific promoter and gene mapping.

One GS corresponds to one mRNA. It is therefore obvious that any portion of cDNA complementary to each mRNA carry the same "information for expression" as the GS. Accordingly, the DNA of the present invention is not restricted to "the DNA comprising the GS itself or portion thereof", but also includes the DNA comprising, for example, "a full-length cDNA complementary to each mRNA" and "the non-GS region of the cDNA complementary to each mRNA or a portion thereof". They can be used as a probe or primer comprising the same "expression information" as that of the GS and can be used as a probe or primer in a similar manner as a GS. For example, by using a GS or a portion thereof as a probe or primer, it is obviously possible for those skilled in the art to readily isolate "a full-length cDNA corresponding to each mRNA" or "the non-GS region of the cDNA complementary to each mRNA or a portion thereof". For example, as described hereinafter, conventional techniques such as "5' RACE", "nesting" and "inverse PCR" can be used.

An example of the method for detecting disease using the GS of the present invention will be described. As shown in Tables 1 - 219, with the method described above it is possible to detect a GS present specifically in a cDNA library constructed from each tissue by detecting and comparing the frequency of appearance of GS in each tissue. It is also possible to identify a GS corresponding to a protein which is expressed commonly in various tissues or which is expressed at low frequency. These GSs are denatured and then fixed on an appropriate filter, for example, nylon filter or nitrocellulose filter. It is convenient to use a single filter with many GSs fixed on it. Usage of a single filter on which many denatured DNAs are fixed is well known. An example may be "the Escherichia coli Gene Mapping Membrane" (Takarashuzo, code No. 9035). It is a single nylon filter on which the cosmid contigs of genomic DNA of E. coli are fixed. It is possible to prepare a filter comprising a group of specific GSs corresponding to proteins expressed in a particular tissue, a filter comprising a group of GSs corresponding to proteins commonly expressed in various tissues, or a filter comprising a group of GSs corresponding to proteins expressed at low frequency. The single-stranded GSs fixed on these filters are then hybridized to labeled complementary DNA fragments synthesized using "random primers" prepared from template mRNA extracted from a test tissue, using four labeled nucleotides and reverse transcriptase (labeled mRNA can also be hybridized to the filters). Similarly, labeled complementary fragments synthesized using mRNA extracted from normal tissue as the template are hybridized (labeled mRNA can also be hybridized to the filters). If the profile of hybridization to a group of GSs has been categorized beforehand by comparing the hybridization profile of various pathogenic tissues to that of corresponding normal tissues, it is possible to diagnose the pathogenic condition of a particular test tissue by comparing the hybridization profile of the test tissue with that of the corresponding normal tissue and assigning that profile to a certain category. Virus infection can be detected in the same manner as in the case of other diseases.

Next, an example of the method for cloning pathogenic genes or their related genes using the GS of the present invention is described. As described above, using the filter on which denatured GSs are fixed, the GS-hybridization profile of various pathogenic tissues and that of corresponding normal tissues are compared. A considerable difference in the hybridization intensity between normal and pathogenic tissues will be an indication that the particular GS corresponds to a pathogenic gene. If a filter comprising only GSs specific for a particular tissue is applied to a sample from that particular tissue, the probability for detecting the GS with a great difference in hybridization intensity is elevated. Also a filter comprising GSs corresponding to proteins whose expression is low will facilitate the identification of the GS corresponding to the pathogenic gene by detecting an intense signal, because the hybridization signal for these GSs is usually weak. Once a GS corresponding to a pathogenic gene is found, said pathogenic gene can be cloned by established methods such as genomic Southern hybridization using said GS as a probe and/or a primer.

Furthermore, a method for cloning a full-length gene using a GS as a probe or primer is described in detail. Cloned genes isolated in the present invention are also appropriate for use in the production of proteins useful as pharmaceutical products. mRNA is extracted from tissues by conventional methods and cDNA libraries are then prepared (See Molecular Cloning, 2nd ed. Vol. 2, Section 8 New York; Cold Spring Harbor Laboratory). In this case, it is desirable to extract mRNA from tissues in which the target gene is highly expressed. One method to detect a specific gene in libraries thus prepared is, for example, to select positive clones via hybridization using a whole or partial GS as a probe. In general, since a GS is specific for a particular mRNA, hybridization can be carried out under certain stringent conditions. Probes used are at least more than 25 bases long, preferably more than 50 bases long, and more preferably more than 100 bases long.

Furthermore, if cDNA libraries, in which the cDNA for a specific gene is concentrated, are prepared, they will be preferable for selecting said specific gene. One method useful for this purpose is carried out as follows: 1) preparation of an affinity chromatographic column of resin on which the denatured GS corresponding to the specific gene is fixed; 2) application of mRNA extracted from a tissue to said column and retention of the mRNA species corresponding to the specific gene on said column; 3) elution and concentration of said retained mRNA; and finally 4) preparation of cDNA libraries using said concentrated mRNA species as the template. Another method is the selective amplification of cDNA corresponding to the specific gene by the PCR. Selective amplification of a specific gene is carried out as follows: using a partial sequence of a GS localized toward the 3' end of the specific gene as primer, cDNA is synthesized from mRNA with reverse transcriptase and 4 NTPs. To the 3' end of a single-stranded cDNA thus obtained a homopolymer such as poly-T is attached by the action of "terminal deoxyribonucleotide transferase (TdT)". In addition, using "a primer complementary to the homopolymer" and "a primer used in said reverse transcriptase reaction, or a primer whose sequence is included in the same GS but is located proximal to the 5' end", cDNA corresponding to the specific gene may be selectively amplified by the PCR [see 5'RACE (5' Rapid Amplification of cDNA ends): PNAS, Vol. 85, pp. 8998 - 9002 (1988); Nucleic Acids Res., Vol. 17, pp. 2919-2932 (1989)]. In addition, instead of the attachment of a homopolymer, there is another method comprising the following steps: 1) a single stranded anchor DNA is linked to the 3' end of a single stranded cDNA using "T4 DNA ligase"; and 2) said cDNA is amplified by the PCR using a primer complementary to said anchor DNA [Nucleic Acids Res., Vol. 19, pp. 5227-5232 (1991)]. Said primer is desirably more than 13 bases long, preferably more than 15 bases long, and more preferably more than 18 bases long. Furthermore, in order to enhance the efficiency of heat denaturation in the cycling reaction, said primer is preferably less than 50 bases long and more preferably less than 30 bases long. By linking said amplified DNA to a vector, a cDNA library concentrated with respect to the target gene is prepared.

In addition, it may be also possible to isolate a cDNA clone corresponding to the specific gene directly from the PCR products. Specifically, the PCR products are first separated by gel electrophoresis, subjected to Southern blotting analysis using the denatured GS as a probe, and examined for the presence of a band which specifically hybridizes to said GS. If a GS-hybridized band is detected, it is highly possible to isolate the cDNA clone corresponding to the specific gene by excising said band from the gel and subjecting it to direct cloning.

As described above, in order to further amplify the specific gene previously amplified by the PCR, it may be possible to perform the second PCR of the primary PCR products by replacing either or both primers previously used with a primer having the base sequence internal to said two primers (nesting) (Journal of Virology, Vol. 64, p. 864 (1990)). Nesting may be performed directly upon the products of the primary PCR. Alternatively, if a band which specifically hybridizes to the GS is detected by the Southern blotting analysis of the primary PCR products, nesting may be performed for the DNA obtained by excision of the band followed by extraction. In the case where a band which specifically hybridizes to the GS is detected by the Southern blotting analysis of nested products using the denatured GS as a probe, it is highly possible to successfully isolate the cDNA clone corresponding to the target gene by excising said band from the gel and subjecting it to direct cloning.

The isolated cDNA clone corresponding to the target gene may often correspond to the full-length mRNA, but it may be a cDNA with the 5' end deleted. In the case where the 5' end is deleted it is possible to isolate the full-length cDNA clone by conventional methods. For example, by screening a cDNA library using a probe comprising the base sequence in the 5' end region of the cloned cDNA, since the target position of said probe is shifted further toward the 5' end of the full-length cDNA than in the case of using a GS as a probe, it is possible to isolate only longer cDNA clones as the positive clone. Also by synthesizing cDNA using "a primer comprising the base sequence in the 5' end region of the cloned cDNA" with mRNA as the template followed by PCR amplification of "a single stranded cDNA having a homopolymer or anchor DNA sequence at the 5' end" and using" the primer used for previous cDNA synthesis or a primer having the sequence internal to that of said primer" and "a homopolymer or a primer complementary to anchor primer" as described above for the 5' RACE method, only the sequence toward the 5' side of the cDNA may be selectively amplified since the position of said primer is shifted further toward the 5' side of the full-length cDNA. Even if the cDNA thus obtained has a deletion at the 5' end, the population of cDNA fragments covering the full-length of the long cDNA may be obtained by repeating this procedure. It may be easy for those skilled in the art to obtain a full-length cDNA by suitably linking said cDNA fragments having overlap segments together.

Alternatively, by performing the inverse PCR (Inverse PCR: Genetics, Vol. 120, p. 621 (1988); Molecular Cloning, 2nd ed., Vol. 2, 14.12-14.13 (New York; Cold Spring Harbor Laboratory)), it may be possible to isolate a cDNA clone extending externally from the GS, that is, in the genomic DNA region. Specifically, the target DNA (genomic DNA or cDNA) is digested with restriction enzymes into fragments of about 2-3 kb and then circularized by ligating the cleaved ends. By performing the PCR for said DNA using "a set of primers which are complementary to the cDNA clone isolated using the GS or the GS as a probe or primer, and thereby making the direction of DNA synthesis mutually opposite (outward), it may be possible to amplify the DNA region extending externally from the GS. There is known a method to isolate a full-length genomic DNA of a specific gene by repeating this procedure (Nucleic Acids Res., Vol. 16, p. 8186 (1988)).

In addition, although "Taq polymerase" is conventionally used in the PCR described above, the cloning procedure may be more efficiently performed using the "LAPCR (long and accurate PCR" technique (Nature Genet., Vol. 7, p. 350-351 (1994), Nature., Vol.369, p.684-685(1994)).

Furthermore, needless to say that by linking said full-length gene thus obtained to a suitable expression vector followed by its expression in an appropriate host, it is possible to obtain the corresponding gene product (Molecular Cloning, 2nd ed.).

Next, there will be described an example of the method for identifying and recognizing cell types using the GS of the present invention. As shown in Tables 1 - 219, based on the appearance frequency of GS in each tissue and its comparison among tissues, it is possible to identify those GSs specifically present in a cDNA library constructed for each tissue. These "tissue-specific GSs" are fixed on a filter. It will be more convenient if GSs specific to each tissue are collected and fixed on a filter as a whole (e.g., a GS block specific for hepatocytes or cerebral cortex cells). As described above, to this filter are hybridized labeled complementary fragments synthesized using "random primers" prepared from mRNA extracted from test tissues or cells, "nucleotide containing 4 labeled nucleotides", and "reverse transcriptase". (Directly labeled mRNA can also be hybridized to the filters.) Depending on the type of tissues or cells, intense hybridization signals will be observed with the GS groups specific to said tissue or cell. Furthermore, a tissue-specific promoter can be cloned by structure analysis of the 5' upstream sequence through the cloning of the corresponding gene using established methods such as genomic Southern hybridization with the "tissue-specific GS" as the probe and/or primer.

These tissue-specific promoters thus obtained are useful for gene therapy in the future.

Gene therapy in a narrow sense aims to supplement the defective protein of patients using gene technology, and in this case it is necessary to express the exogenous gene in a desired tissue in a desired quantity. For this purpose, a promoter which is known to be expressed in a specific tissue in a desired quantity (in most cases a large quantity is desired) is highly useful. Although, at present, a virus promoter is often used, it can be inactivated by endogenous modification such as methylation. Promoters provided by tissue-specific GSs will be ideal substitutes for viral promoters.

There will be described the method for chromosomal assignment of DNA corresponding to the GS of the present invention using the probe derived from the GS obtained as described above.

First, the Southern blotting method will be described.

According to this method, for example, chromosomes are isolated from a lymphoblast cell line of human normal karyotype (e.g., GM0130b), and then a monochromosomal hybrid cell is prepared by introducing each human chromosome into non-human cells, such as rodent cells, and cultured on a large scale by standard methods. Then the DNAs extracted from said hybrid cells are digested with various restriction enzymes and subjected to agarose gel electrophoresis. Then, the electrophoresed DNAs are hybridized to $^{32}$P-labeled GS prepared as described above and used as the probe. By identifying the hybrid cell the DNA of which is hybridized to said probe, it is possible to identify the chromosome in which the DNA corresponding to the GS of the present invention is present. Southern hybridization test of the total human genomic DNA using each labeled GS as a probe formed a single band corresponding to the GS, indicating that the DNA of the present invention can be used as a desirable probe for human genomic DNA. It is obvious that a desirable probe for human genomic DNA can be used also as a desirable probe for human cDNA and human mRNA.

A method similarly using the PCR to determine chromosomal localization of the GS of the present invention will be described.

To prepare most appropriate primers, base sequences are selected from the sequence of the GS in question by conventional methods, for example, by using the computer software OLIGO4.0 (National Biosciences) and the oligonucleotides (20-24mer) having the selected sequences are synthesized. The preferred size of the sequence to be amplified by the PCR is from 50mer to 100mer.

Using the primers thus synthesized and the chromosomal DNA extracted from the monochromosomal hybrid cell as such as the template, amplification by the PCR is performed in a conventional manner. Resulting PCR products are subjected to non-denatured acrylamide gel electrophoresis and stained with ethidium bromide for fluorescent detection. The sizes of these PCR products are then determined.

Chromosomal assignment is confirmed when the presence of a PCR product of correct size is confirmed.

It is evident that a chromosome or chromosomes in which the DNA corresponding to a GS is localized can be identified by using these procedures. It has also become evident that the DNA of the present invention can be used as desirable primers for human genomic DNA since a single band has resulted from amplification of the total human genomic DNA by the PCR using primers designed based on each tested GS. Obviously, a desirable primer for human genomic DNA is also a desirable primer for human cDNA and human mRNA.

Brief Description of Figures

Fig. 1 shows the preparation of 3' MboI cDNA library.

Fig. 2 shows the results of tests of primers. A shows the location of primers on the vector; and B shows the electrophoretic patterns of DNA fragments amplified using the primers (A). Primers used are as follows: lane 1, FW (-40)/RV (-14); lane 2, FW (-40)/RV (-36); lane 3, FW (-40)/RV (-71); lane 4: FW (-40)/RV (-29); and lane 5, FW (-47)/RV (-48). Artifacts are indicted by arrows.

Fig. 3 shows the electrophoretic pattern of PCR products using FW(-40) and RV(-14) as primers. The lane at the right end shows the electrophoretic pattern of size markers and the other lanes show the PCR products using FW (-40)/RV (-14) as primers.

Fig. 4 shows the mRNA concentration reflecting the frequency of appearance of each GS in the cDNA library: especially, Fig.s 4A - 4D; experimental results; Fig. 4E, photographs of colonies; and Fig. 4F, summary.

Fig. 5 shows the appearance frequencies for various cDNAs in the 3'-directed HepG2 cDNA library.

Fig. 6 shows the genetic mapping of each GS (gs) using PCR.

Fig. 7 shows the genetic mapping of each GS (gs) using PCR.

Fig. 8 shows the genetic mapping of each GS (gs) using PCR.

Fig. 9 shows the genetic mapping of each GS (gs) using PCR.

Fig. 10 shows the genetic mapping of each GS (gs) using PCR.

Fig. 11 shows the chromosomal mapping of GS001418 (gs001418) using PCR.

Fig. 12 shows the chromosomal mapping of GS001457 (gs001457) using PCR.

Fig. 13 shows Southern blotting of human total chromosomes using the GS as a probe.

Fig. 14 shows Southern blotting of human total chromosomes using the GS as a probe.

Fig. 15 summarizes the characteristics of hybrid cells used for Southern hybridization.

Fig. 16 shows Southern blotting of chromosomal DNA from the hybrid cells using GS000152 (clone s14g02) as a probe.

Fig. 17 shows Southern blotting of chromosomal DNA from the hybrid cells using GS000041 (clone s650) as a probe.

Fig. 18 shows Southern blotting of chromosomal DNA from the hybrid cells using GS000181 (clone hm01e01) as a probe.

Fig. 19 shows Southern blotting of chromosomal DNA from the hybrid cells using GS000055 (clone c13a18) as a probe.

Fig. 20 shows Southern blotting of chromosomal DNA from the hybrid cells using GS000180 (clone s479) as a probe.

Fig. 21 shows Southern blotting of chromosomal DNA from the hybrid cells using GS000094 (clone s173) as a probe.

Fig. 22 shows Southern blotting of chromosomal DNA from the hybrid cells using junk (clone hm01g02) as a probe.

Fig. 23 shows the chromosomal mapping of each GS by Southern blotting. E stands for EcoRI, Ba stands for BamHI, Bg stands for BglII and E/B stands for double cleavage with EcoRI and BamHI.

Fig. 24 shows the chromosomal mapping of each GS by Southern-blotting. E stands for EcoRI, Ba stands for BamHI, Bg stands for BglII and E/B stands for double digestion with EcoRI and BamHI.

Fig. 25 shows the chromosomal mapping of each GS by Southern blotting. E stands for EcoRI, Ba stands for BamHI, Bg stands for BglII and E/B stands for double digestion with EcoRI and BamHI.

Fig. 26 shows the chromosomal mapping of each GS by Southern blotting. E stands for EcoRI, Ba stands for BamHI, Bg stands for BglII and E/B stands for double digestion with EcoRI and BamHI.

## Preferred embodiments of the invention

In the following section, there will be explained preferred embodiments of the present invention. However, the present invention will not be restricted to these preferred embodiments.

## [Example 1]

### Preparation of mRNA

Cytoplasmic RNA was extracted from a liver cancer cell line HepG2 (Aden., et al., Nature 282, 615-617, 1979) using standard procedures [Sambrook, J., et al., Molecular Cloning, 2nd ed. (New York: Cold Spring Harbor Laboratory), vol. 1, pp. 7.3-7.36, 1989]. Briefly, HepG2 cells grown in Dulbecco's modified Eagle medium supplemented with 10% FCS were lysed in RNA extraction buffer [0.14 M NaCl, 1.5 mM $MgCl_2$, 10 mM Tris-HCl (pH 8.6), 0.5% NP-40, 1 mM DTT, 1000 units/ml RNase inhibitor (Pharmacia)] by using a Vortex mixer for 30 sec and then left standing on ice for 5 min. Nuclei and other cell debris were precipitated by centrifuging at 12,000 g for 90 sec, and the supernatant was deproteinized with Proteinase K followed by phenol extraction. RNA was precipitated by isopropanol and rinsed with 70% ethanol. Finally, the poly $A^+$ fraction was collected by oligo dT column fractionation (Aviv., et al., Proc. Natl. Acad. Sci. USA 69, 1408-1412, 1972).

[Example 2]

Preparation of vector primer DNA and construction of cDNA libraries

To prepare a vector primer, pUC19 DNA amplified in JM109 cells (Yanisch-Perron, C., et al., Gene 33, 103-119, 1985) was digested with PstI to completion and a poly T-tail was added with terminal transferase (Pharmacia) to a mean length of 26. This process was monitored by the incorporation of $^{3}$H-deoxythymidine triphosphate [Okayama, H., et al., Methods in Enzymology (San Diego: Academic Press), vol. 154, pp. 3-28, 1987]. The product was digested by HincII, and the resulting short fragments were eliminated by chromatography with Sepharose S-300. Then the T-tailed plasmid was purified by an oligo dA column and stored in 50% ethanol at a concentration of 1 µg/µl.

Fig. 1 shows the outline of the construction of the cDNA library. Two micrograms each of the cytoplasmic Poly $A^+$ RNA and the vector primer DNA were co-precipitated in 70% ethanol containing 0.3 M Na-acetate and the pellet was dissolved in 12 µl of distilled water. For the first strand synthesis, after heat denaturation at 76 °C for 10 min, 4 µl of 5 x reaction buffer [250 mM Tris-HCl (pH 8.3), 375 mM KCl, 15 mM $MgCl_2$], 2 µl of 0.1 M DTT and 1 µl of 10 mM each of dATP, dCTP, dGTP and dTTP were added to the sample at 37 °C. The reaction was initiated by the addition of 200 units of reverse transcriptase MMLV-H-RT (BRL), and after incubation at 37 °C for 30 min, stopped by transferring the reaction tube onto ice. For the second strand synthesis, to the aforementioned reaction mixture the following was added: 92 µl of distilled water, 32 µl of 5 x E. coli reaction buffer [100 mM Tris-HCl (pH 7.5), 20 mM $MgCl_2$, 50 mM $(NH_4)_2SO_4$, 500 mM KCl, 250 µg/ml of BSA, 750 µM βNAD], 3 µl of 10 mM each of dATP, dCTP, dGTP and dTTP, 15 units of E. coli ligase (Pharmacia), 40 units of E. coli polymerase (Pharmacia), and 1.5 units of E. Coli RNase H (Pharmacia). The reaction mixture was then incubated at 16 °C for 2 h and heated to 65 °C for 15 min. Then 20 units each of BamHI and MboI were added, and the reaction mixture was incubated at 37 °C for 1 h and heated again at 65 °C for 30 min. Finally, the sample was diluted up to 1 ml with 1 x E. coli reaction buffer, and 100 units of E. coli ligase were added. The resulting mixture was incubated at 16 °C overnight. An aliquot of this mixture was used to transform competent E. coli DH5 cells (Toyobo). Transformants were selected by ampicillin resistance. The product was named "3' MboI cDNA library".

[Example 3]

Amplification of cDNA insert by PCR

The plasmid-carrier E. coli colonies were picked into 96-well plates containing 125 µl of LB medium (Davis, R. W., et al., Advanced Bacterial Genetics. New York: Cold Spring Harbor Laboratory, 1980) in each well and incubated in a moist chamber at 37 °C for 24 h. A replica culture was made for every plate using a 96-pinned replica device (Sigma) and the master plates were stored at -80 °C for future use. After overnight incubation at 37 °C, 50 µl of the culture from each well of these replicas were transferred to polycarbonate 96-well plates (Techne). Bacteria were collected by centrifugation in an Omnispin H4211 rotor (Sorvall) at 1500 rpm for 5 min, resuspended in 50 µl of water, covered with a layer of mineral oil and lysed at 95 °C for 30 min in a metal bath. Debris were removed by centrifugation at 3600 rpm for 30 min in the same rotor.

Five microliters of the supernatant were added to 20 µl of distilled water and kept at 95 °C for 10 min under a layer of mineral oil. Then the denatured lysate was subjected to PCR by adding 25 µl of 2 x reaction mixture [40 mM Tris-HCl (pH 8.9 at 23 °C), 3 mM $MgCl_2$, 50 mM KCl, 200 µg gelatin/ml] containing 5 pmol each of primers, 5 nmol each of dATP, dCTP, dGTP, dTTP and 1.25 units of Taq DNA polymerase (Cetus) at 70 °C. Temperature cycling reactions were carried out immediately after addition of the reaction mixtures using a thermal cycler either for microfuge tubes (PJ1000, Perkin Elmer Cetus) or for a 96-well plate (PHC-3, Techne); 35 repeated cycles of 30 sec at 96 °C, 1 min at 55 °C, and 2 min at 72 °C without a final extension step were performed.

For this method, the correct choice of primers for the PCR reaction is crucial. Therefore, preliminary tests were performed using the following primers with a predicted Tm of above 60 °C.

The primers tested were a pair of primers, FW(-47) and RV(-48), which are identical to the commercially available 24 mer primers, a second pair of primers, [FW(-40) and RV(-29)], which are a longer version (21 mer) of the well-tested sequencing primers, and the primers RV(-71) and RV(-14), which have a triplet sequence at the 3' terminus identical with that in FW(-40) but is in the opposite orientation (Fig. 2A).

In most of the cases where various combinations of primers were tested, short PCR artifacts appeared, besides the expected major products (Fig. 2B, arrows indicate the PCR artifacts.). These artifacts could be reduced by raising the annealing temperature, lowering the primer concentration or lowering the substrate concentration but in all cases the yield of the products was not high enough to serve as a template for the sequencing reaction without concentration thereof.

However, since one pair of primers [SW(-40) and RV(-14)] did not yield artifacts (Fig. 3), this pair was selected for further tests, and was found to give reproducible results. Similar results were obtained with randomly selected cDNA clones. Therefore, only this pair of primers SW(-40) and RV(-14) was used as the primers of the present embodiment.

[Example 4]

DNA sequencing

The PCR products were drop-dialyzed against TE [10 mM Tris-HCl (pH 8.0), 1 mM EDTA] on millipore filter (VS 0.025 µm) for 90 min while stirring. Forty-eight samples are easily applied on a single filter of 150 mm diameter. Without further purification the samples were subjected to the Cycle Sequencing protocol (Applied Biosystems, 1991) using dye labeled primers with minor modifications. For dideoxycytidine sequencing reaction, 2 µl of the dialyzed PCR reaction product (about 0.2 pmol of template DNA) were added to 3 µl of a reaction mixture containing 0.4 pmol of FAM M13 (-21) Primer (Applied Biosystems) in 160 mM Tris-HCl (pH 8.9), 40 mM $(NH_4)_2SO_4$, 10 mM $MgCl_2$, 50 µM dATP, 12.5µM dCTP, 75 µM 7-deaza-dGTP (Boehringer Mannheim Biochemicals), and 50 µM dTTP, 25µMddCTP, 0.8 unit of Taq Polymerase (Perkin Elmer Cetus), and subjected to 15 plus 15 cycles of the reaction (95° C 30 sec, 60° C 1 sec, 70° C 1 min and 95° C 30 sec, 70° C 1 min) according to the manufacturer's recommendation in a 96-well plate using a thermal cycler (PHC-3, Techne). The three other sequencing reactions for dideoxyguanosine, dideoxyadenosine, and dideoxythymidine were performed in parallel (with TMRA, JOE, and ROX primers respectively, supplied by Applied Biosystems) in an identical fashion, except that twice the volume of all the ingredients was added to the dideoxyguanosine and dideoxythymidine reactions. Each sample, from a set of four was cooled to 4° C, pooled, precipitated with ethanol, resuspended in 6 µl of a solution of formamide/50 mM EDTA (5/1 by v/v), loaded onto sequencing gel and analyzed by a DNA autosequencer (Model 373A Ver 1.0.1, Applied Biosystems).

[Example 5]

The frequency of appearance of each GS of the cDNA library reflects mRNA population.

To confirm that our 3'-directed regional cDNA library was a non-biased representation of the mRNA population in HepG2 cells, the inserts of four cDNA clones (EF-1α, α-1-antitrypsin, hnRNP core protein A1 and inter-α-trypsin inhibitor) from the clones redundantly obtained by random selection of cDNA were radiolabeled and used as probes in a Northern analysis of poly $A^+$ mRNA from the HepG2 cells. (The results are shown in Fig. 4A-D, and summarized in Fig. 4F.) The relative band intensity of the four mRNA species demonstrated that their relative ratios were 52, 24, 1 and 1.2, respectively (lane iii in Fig.4F). Then the same set of probes was used for measuring the number of colonies hybridizing with each probe in the same cDNA library of 8,800 clones (Fig. 4E).

The clonal frequencies were 307, 128, 7 and 9, or in ratio, 44, 17, 1 and 1.3, respectively (lane iv in Fig. 4F). These two estimates agreed, showing that the cDNA library used is a non-biased representation of the mRNA population. The ratio was practically unchanged when different preparations of mRNA from the same cell were tested.

Fig. 4 shows the proportionality of the composition of the 3'-directed cDNA library and of the mRNA. Fig.4A, 2 µg of poly $A^+$ RNA from HepG2 cells was electrophoresed in lanes 1-4 of a formamide agarose gel containing ethidium bromide (5 µg/ml) and then exposed to UV. Lane 5 is the RNA ladder (BRL) used as size markers (kb). In Fig. 4B, the filter was northern blotted using the following $^{32}P$-labeled 3'-specific cDNA probes: Elongation factor-1α (lane 1), α1-antitrypsin (lane 2), HnRNP core protein A1 (lane 3), inter-α-trypsin inhibitor (lane 4). In Fig. 4C, one pmol each of the non-labeled cDNA fragments [EF-1α (lane 1), α1-antitrypsin (lane 2), HnRNP core A1 (lane 3), inter-α-trypsin inhibitor (lane 4), were electrophoresed in a 2% agarose gel, then photographed. Fig. 4D is a Southern analysis of the blotted filer from Fig. 4C, using the same set of radioactive probes. Lane 5 shows the migration pattern of the reference 1 kb ladder (BRL). Hard copies of these screen images were taken at 8 h for b, and 1 h for d. The radioactivity in each band was counted directly in a scinti-scanner (β-603; Betagen) and registered in (i) and (ii) in Fig. 4F. The observed band intensities were corrected based on the band intensities in Fig. 4D (ii in Fig. 4F), and normalized relative to the value of probe 3 (HnRNP core A1, lane iii in Fig. 4F) as 1 (iii in Fig. 4F). These values represent the relative content of each mRNA species in the original mRNA preparation. Fig. 4E shows the results of colony hybridization of the membranes carrying 8,800 colonies of the 3'-directed cDNA library using the same set of the four radioactive probes. Positive colonies were counted and registered (iv in Fig. 4F), then normalized with the value of HnRNP core protein A1 as 1. The numbers in B, D and E in Fig. 4 represent the probe No. in Fig. 4F. Fig. 4F shows a remarkable agreement between the values of lanes (iii) and (v).

[Example 6]

Population study of the cDNA library

To analyze further the composition of the cDNA library, 7 and 10 clones were selected from the redundant (group I) and solitary (group II) sequence groups, respectively, and these inserts were used as radiolabeled probes for colony hybridization (Fig. 6). The frequencies of the colonies that hybridized with group I probes were roughly identical to those that were randomly picked and sequenced. These frequencies were about 3.5%-0.1%. Nearly 52% of the cDNA library population consisted of the redundant sequence group containing 173 species. When 8 probes from group II were tested, 18 positive colonies were identified among 26,400 colonies screened, giving an average frequency of 0.007%. Two probes did not hybridize with any of the 26,400 colonies, resulting in the average frequency of <0.004%. Thus, the average frequency of the 10 probes in group II was several orders of magnitude less than the lowest of group I.

The results are summarized in Fig. 5, showing the appearance frequencies of various DNA species in the 3'-directed HepG2 cDNA library. In Fig. 5, seven cDNA probes (a15 through tb042) were selected from the 162 identified genes in the redundant group (group I), and ten (s155 through s632) were randomly chosen from the solitary group (group II). In columns A, B and C, each one of the insert DNAs was radiolabeled and used as a probe for colony hybridization tests of 982 (A), 8,800 (B) or 26,400 colonies (C). NT indicates "not tested". The DDBJ entry names of the 17 clones listed in this table are HUM000A15, HUM000C321, HUM00TB038, HUMHM01B02, HUM0C13A04, HUMHM02D02, HUM00TB042, HUM000S155, HUM000S159, HUM000S639, HUM000S635, HUM000S170, HUM000S154, HUM000S167, HUM000S645, HUM000S647, and HUM000S632.

[Example 7]

Analyses of sequencing errors

All the sequence data presented in this specification were obtained by repeated cycles of enzymatic amplification of the plasmid inserts, followed by cycle sequencing with Taq polymerase. Sequences of 60 clones that showed data bank matches were examined for discrepancies from the data bank entries. It was found that the accuracy in the region 1-100 bp distant from the cloning site was 98.7%, indicating that the primers or probes designed with the sequence in this region could be obtained practically without any erroneous sequences or even if they contain any errors, they are functionally without problems.

[Example 8]

Mapping of GS by PCR

⟨cDNA sequence⟩

cDNA library was constructed from mRNA of DMSO treated HL60 cells. The methods for construction of the 3'-directed cDNA library and for sequence analysis of the library components are the same as described in Examples 1-4.

⟨PCR primer⟩

Primer design was performed by using the computer software OLIGO 4.0 (National Biosciences) which eliminates possible formation of inter- or intra-molecular secondary structures. In addition to the primer design, transfer of oligonucleotide sequences to the local database and synthesizer were semiautomated using a Macintosh computer linked with a network. DNA oligomers were synthesized on an automated DNA synthesizer (Model 394, Applied Biosystems) on a 40 nmol scale. The synthesized oligomers were used as PCR primers without further purification.

〈Preparation of Genomic DNA〉

The human genomic DNA was extracted from the normal karyotype lymphoblastoid cell line GM0130b.

Mouse and Chinese hamster genomic DNAs were purchased from Clontech. Monochromosomal hybrid cells utilized for mapping panel were commonly used ones which have been described previously. Briefly, chromosomes 3, 4, 9, 11, 12, 13, 15, 22 and Y were carried in human-Chinese hamster monochromosomal hybrid cells, and chromosomes 1, 2, 5, 6, 7, 8, 10, 11, 12, 14, 15, 16, 17, 18, 19, 20, 21 and X were carried in the human-mouse monochromosomal hybrid cells A9 series. The integrity of the hybrid cells were monitored by *in situ* hybridization.

〈Amplification by Polymerase Chain Reaction〉

PCR was performed according to standard protocols (Saiki, R. K., et al., Science 230, 1350-1354, 1985), using 10 pmol of each primer on a whole 20 µl scale reaction, with 35 thermal cycles of 30 sec at 94 °C, 60 sec at an annealing temperature, and 90 sec at 72 °C, using a Perkin-Elmer 9600 thermal cycler. Annealing temperature was determined according to the "optional annealing temperature" estimated by the Program OLIGO.

〈Analysis of the PCR Products〉

The PCR products were run on an 8% polyacrylamide non-denatured gel (Acrylamide:Bis-acrylamide = 19:1, 1 mm thick) at 300 V for 1 h, followed by staining in 90 mM Tris-borate, 2 mM EDTA buffer solution containing 0.25 µg/ml ethidium bromide for 15 min. The size of the amplification products were determined relative to the 10 bp DNA ladder (BRL). Detection of fluorescence was performed by using a laser fluorescent image analyzer (FM-BIO, Hitachi Software Engineering). The image data were transferred to a computer for analysis.

〈Results of Analysis of the PCR Products〉

Among various species of 3'-directed cDNA-GSs obtained from granulocytoid cells, 195 novel GSs which did not match the sequences deposited in Genbank Release 76 were selected and used for designing primers for the PCR. The PCR was performed with these primers using the total human genomic DNA as the template.

Among the 195 primer pairs, 191 (98%) yielded products whose size matched those expected within 5 nt. The results are summarized in Figs. 6 - 10 whose figure legends are as follows: GS, gene signature; CN, clone name; Chromosomal position, chromosome numbers to which GSs were mapped; Sequence of primers, DNA sequences of primers (Sense, sense strand; anti-sense, anti-sense strand); AT, annealing temperature; HO, Observed size of PCR products with total human genomic DNA (nt); HE, Expected size of PCR products with total human genomic DNA (nt); MO, Observed size of PCR products with mouse genomic DNA (nt); CO, Observed size of PCR products with Chinese hamster genomic DNA (nt); G, Number of "hits" of GS in the granulocytoid (DMSO treated HL60) cDNA library after analyzing altogether 1000 clones; T, Total number of "hits" of the GS after analyzing altogether 3000 clones from the three cDNA libraries of HL60 with and without induction by DMSO or TPA. Question marks ("?") indicate that the PCR products did not yield a clear band.

"M" indicates that the PCR products yielded a band which was indistinguishable from the band observed after the reaction using mouse DNA as the template. Similarly, "C" indicates that the PCR products yielded a band which was indistinguishable from the band after the reaction using Chinese hamster DNA as the template.

The overall rate of success of the PCR was 191/195 (98%), although GSs were randomly selected from the cDNA sequences, indicating that the quality of the cDNA library used in this work was reliable, and that the sequence analyses and primer designs were performed appropriately. Thus, the possible chances of failure of the PCR caused by presence of an intron(s) in the relevant cDNA sequences is negligible in working with the GS, as introns virtually do not lie in the poly A proximal 3'-region of vertebrate genes (Wilcox et al., Nucleic Acids Res. 19, 1837-1843, 1991). This is a big advantage compared to the use of partial fragmented cDNA sequences obtained from randomly primed cDNA libraries (Adams et al., Science 252, 1651-1656, 1991) or from 5'-directed cDNA libraries.

⟨Chromosomal assignments of GS⟩

The 191 primer pairs that yielded PCR products from total human DNA were used for chromosomal assignments of the GSs with the monochromosomal hybrid cell panel. At least 119 GSs were assigned to a single chromosome. As an example, GS001418, shown in Fig. 11, was assigned to chromosome number 3. With some clones, extra products were obtained, some of which were assigned to the same chromosome, whereas others to different chromosomes. An example, GS001457, is shown in Fig. 12. Sixty-two (33%) clones yielded the expected PCR products with two or more different chromosomes. Thirty-five cases (18%) yielded PCR products whose size were indistinguishable from background rodent genomic DNA. Among these, 21 GSs produced products indistinguishable from mouse and Chinese hamster DNA. Ten GSs yielded no expected PCR products with the monochromosomal cell panel DNA although the expected PCR products from total human genomic DNA were observed. The 10 cases probably arose from a small deletion in the hybrid cells. Five clones obtained from HepG2 cDNA library have been analyzed also by Southern blot analysis. Four out of the 5 GSs (GS000053, GS000120, GS000271 and GS000279) gave consistent results with those obtained by the PCR. One GS (GS000228), which was uncertainly assigned to chromosome Y because of the weak signal detected by the Southern blot method, was assigned to chromosome 11 by PCR.

[Example 9]

Mapping of GS by Southern blot method

⟨Cell lines⟩

Total human genomic DNA was isolated from the human normal karyotype lymphoblastoid cell line GM0130b. Monochromosomal hybrid cells used as the mapping panel are shown in Fig. 15. Hybrid A9(neo-x)-y cells as described by Koi, et al. (Jpn. J. Cancer Res. 80, 413-418, 1989) were donated by Dr. M. Oshimura, Faculty of Medicine, Tottori University, passaged 3 times and frozen for storage. The loss or rearrangements of chromosomes could have occurred during this period. The GM series was obtained from the Mutant Cell Repository, National Institute of General Medical Science (NIGMS) (Camden, NJ). To confirm that human chromosomes remained intact in the hybrid cells after storage in liquid nitrogen, metaphase spreads of the hybrid cells were monitored by chromosome staining based on *in situ* hybridization using biotinylated total human DNA as the probe (Durnam, D. M., et al., Somatic cell Mol. Geneta. 11, 571-577, 1985) Intact, as well as translocated or fragmented human chromosomes were easily detected by this means. In a hybrid cell mapping panel, chromosomes 11, 12 and 15 were represented by the hybrid cell lines A9(neo-11)-1, A9(neo-12)-4 and A9(neo-15)-2, respectively, and in another panel, they were represented by the hybrid cell lines GM10927A, GM10868 and GM11418, respectively.

⟨Isolation of genomic DNA and Southern blotting⟩

High molecular weight DNA was extracted from cells using sodium dodecyl sulfate (SDS) and Proteinase K, followed by phenol-chloroform extraction and ethanol precipitation. DNAs were digested overnight with a combination of two restriction enzymes including EcoRI, BamHI and BglII. About 5 $\mu$g of each digest was electrophoresed in an 0.8% agarose gel, then transferred to Hybond $N^+$ membrane (Amersham) with 0.4 N NaOH. The membrane was rinsed in 2 x SSC and stored at 4°C for subsequent use.

Clones containing a novel sequence and having more than 150 bp were selected as probes. The cDNA inserts of the clones were amplified by the PCR. The PCR products were isolated by electrophoresis through a 2% low-melting temperature agarose gel (Nusieve : SeaPlaque, 3 : 1), followed by excision. The gel was removed by melting at 65°C and digesting with $\beta$-Agarose I (Bio Labs) at 40°C for 1 h. The probes were labeled with [$\alpha$-$^{32}$P]dCTP by random priming using a commercial kit (Amersham). Hybridization proceeded at 65°C in a high salt buffer containing 6xSSC, 1x Denhardt's solution and 0.5% SDS, in the presence of 0.1 mg/ml of sonicated, denatured salmon sperm DNA. The membranes were washed in 2xSSC, 0.1% SDS at 65°C for 30 min, then twice for 30 min in 0.1xSSC, 0.1% SDS at 65°C, and analyzed using a Fuji BAS-2000 imaging analyzer.

〈Analyses with Genomic DNA〉

Among the HepG2 3'-directed cDNA libraries described in Examples 1 and 2, 160 novel clones were selected and used as probes for Southern blots.

Total human genomic DNA was isolated from a cell line GM0130b that has a normal karyotype, and digested with the restriction enzymes, EcoRI, BamHI and BglII alone or in combination. The GS clones used as probes were the 3'-directed cDNAs. Each of these cDNAs covers a region between the poly(A) site and the nearest MboI site (GATC) (Okubo, K., et al., Nature Genetics 2, 173-179, 1992) and thus do not have restriction sites for BamHI or BglII. In addition, because the average size of GS is 270 bp, the chances of having an EcoRI site in the cDNA moiety were not high. In fact, only 7 clones out of the 160 analyzed had an EcoRI restriction site.

Membranes blotted with digested human genomic DNA were hybridized with radio-labeled GS probes and washed at high stringency. Since the 3'-terminal region of cDNA has, in general, a unique sequence which differs from that of protein encoding regions which tend to have conserved motifs, cross hybridization with unrelated cDNA sequences will not occur under such stringency. Examples of the results of hybridization are shown in Figs. 13 and 14. Clones s503 and s632 (Figs. 13a and 13b; junk) respectively represent unique single band producers. As shown below, 67 clones belonged to this class. The positions of the GS sequence relative to the restriction sites were inferred from the band patterns. Clone s311 (Fig. 13c; GS000092) showed a single band with EcoRI -as well as (EcoRI + BamHI)-digested DNA, but two bands of different sizes in other double digests. The double digestion thus helped resolve multiple GSs. Similar results were obtained with clone c13a08 (Fig. 13d; GS000055), in which there were 2 bands with EcoRI- or (EcoRI + BamHI)-digested DNAs, and 4 when digested with (EcoRI + BglII) or (BamHI + BglII). On the other hand, 4 hybridization bands appeared with clone s479 with EcoRI alone, but the number of bands decreased with (EcoRI + BglII) and (BamHI + BglII) (Fig. 14e; GS000180). These results indicate that genomic DNAs should be digested in various ways to reveal the maximum number of hybridizing fragments. The results of the analysis showed that 41, 10, 7 and 19 clones contained 2, 3, 4 and 5 or more bands, respectively. Clones s14f01 and tw1-46 (Figs. 14f and 14g; GS000407 and junk, respectively) contained at least 10 bands in each lane. Since the EcoRI restriction site is not present in the two GS sequences, the multiplicity of bands is likely to represent the multiple copy number of these genes. Clone kmb07 moved as a smear (Fig. 14h; junk), even after intensive high stringency washes, suggesting that this probe has a repetitious sequence which has not been hitherto identified.

〈Chromosomal assignments〉

A set of monochromosomal hybrid cells carrying a single human chromosome in a background of rodent chromosome was collected (Fig. 15). Thirteen cell lines were microcell hybrids established by Koi et al. (Koi, M., et al., Jpn. J. Cancer Res. 80, 413-418, 1989) and the others were obtained from NIGMS. The results of monitoring the human chromosomes in these cell lines by *in situ* hybridization using biotinylated total human DNA are also presented in Fig. 15.

The GSs were assigned to chromosomes using hybrid cell mapping panels. Three types of membranes were prepared, each having DNAs prepared from hybrid cells, and digested with EcoRI, (EcoRI + BamHI), or (BamHI + BglII). Among these three types of membranes, the one which should have yielded the maximum number of bands was used for each GS probe, according to the results of total genomic Southern blots. Examples of hybridization results are shown in Figs. 16 - 22. The numeral on each lane represents the human chromosome numbers which is contained in the hybrid cell, and H stands for the total human chromosomes. Clone s14g02 (GS000152; Fig. 16) that showed a single hybridization band with the total human DNA digested with EcoRI (lane H), showed the corresponding band only with the hybrid cell line containing human chromosome 4. Thus, this GS lies in chromosome 4.

The clone s650 (GS000041; Fig. 17) was assigned to chromosome 12 which showed a characteristic 7.5kb band in the presence of an (EcoRI + BamHI)-digested membrane. However, with an EcoRI digested DNA, the clone could not be assigned, as the human-specific and the cross-reacting rodent DNA fragments overlapped. The single, but shorter fragment band (1.3kb) which appeared in lanes 3, 4, 9, 13 and 22 represents the homologous DNA sequence in Chinese hamster, and the 3.3kb band in other lanes represents the homologous DNA in the mouse.

Clone hm01e01 (GS000181; Fig. 18) exhibited two fragments when hybridized to total human DNA treated with EcoRI alone, and these corresponding bands appeared in lanes 1 and 2. Thus, the two members of this gene family are located on two chromosomes.

Fig. 19 shows that clone c13a08 (GS000055) exhibited 4 bands when hybridized to (BamHI + BglII)- or (EcoRI + BglII)-digested total human DNA, although only 2 bands appeared with EcoRI- or (EcoRI + BamHI)-digested human DNA. Therefore, the (BamHI + BglII)-digested DNA panel was used for this clone. Two bands (12.3kb and 7.5kb) appeared in lane 7, a 5.2kb band in lane 2, and a 3.2kb band in lane 17. Two bands (6.0kb and 3.8kb) that cross-reacted with Chinese hamster DNA appeared in lanes 3, 4, 9, 13 and 22, and a single band (3.5kb) that cross-reacted with mouse DNA appeared in other lanes.

Clone s479 (GS000180; Fig. 20) showed 4 EcoRI fragments with total human DNA. The hybridization to an EcoRI-digested DNA panel yielded in bands of 10.5kb in lanes 7 and 19, 8.5kb in lane 8, 7.8kb in lanes 11 and 12, and 3.5kb in lane 11. Thus, the human specific genes are dispersed among chromosomes 7, 8, 11, 12 and 19, among which the 10.5 and 7.8kb bands in the total DNA both consist of two overlapping fragments. As shown in lane H, the intensity of these overlapping fragments was higher than normal. The 3.5kb band in lane H, as well as in lane 11 was also intense, suggesting that it also represents overlapping fragments.

Clone s173 (GS000094) exhibited 5 bands in EcoRI-cleaved total DNA (Fig. 21). Four corresponding fragments included a 4.5kb fragment in lane 1. Another 4.5kb band was observed in lane 4, indicating that the corresponding band in lane H overlapped. In addition, an intense 3.1kb band was observed in lane 17.

Clone hm01g02 (junk; Fig. 22) exhibited many bands with total DNA, and with those from monochromosomal hybrids. This clone must represent a multiple and closely related family of genes. It also contains a sequence conserved in homologous rodent genes which also give rise to multiple bands. Since most of the human specific and rodent bands overlapped, the chromosomes could not be assigned. Other combinations of restriction enzymes did not resolve the overlap.

The results of the total genomic DNA analyses and the chromosome assignments of 160 GSs are summarized in Figs. 23 - 26. Through total genomic DNA analyses using 4 differently digested human DNAs, 67 clones were categorized into a single band group, 41 in a two band group, 10 in a three band group, 7 in a four band group and 19 in a group that yielded five or more bands. Nine clones did not show any hybridization band under fixed conditions.

Assignment of two band clones showed that the two genes lie in different chromosomes in 15 of them, whereas the gene represented by clone s317 originated from the same chromosome. The three band clones s308 (GS000412) and s401 (GS000224) showed that two of the fragments lie on the same chromosome, and clone hm05g02 (GS000209) and s17a10 (GS000294) showed bands in different chromosomes. Clones displaying four or more bands showed a relatively dispersed distribution among chromosomes. "junk" in Example 9 is the DNA segment cloned by the same method used for GS but is not numbered.

[Example 10 Cloning of gene using GS]

[10A. Cloning of a full length cDNA encoding a human ribosomal protein, homologue of yeast S28. Cloning of the full length cDNA by PCR using a primer comprising a partial sequence of a GS(1)]

Using a primer ('5-TGAAAATTTATTACTACAGTGTTTTCACCA-3' (SEQ ID NO:7839)) that is a partial sequence of a DNA which is substantially the same as the complementary strand of HUMGS00500 and a primer (5'-TAATACGACTCACTATAGGG-3' (SEQ ID NO: 7840)) complementary to the vector (pSPORT) sequence that is located external to the 5' end of the cDNA, HepG2 cDNA library was amplified by the PCR and a full length cDNA clone encoding a human ribosomal protein, a homologue of yeast ribosomal protein S28 was isolated. (Hori et al., Nucl. Acids Res. 21: 4394, 1993).

[10B. A human ribosomal protein homologous to rat L9 ribosomal protein-Cloning of the full length cDNA by PCR using a primer comprising a partial sequence of a GS(2)]

Using a primer 5'-CTTCTTTCTGTAGCCAGGTAACTCT-3' (SEQ ID NO: 7841) that is a partial sequence of a DNA which is substantially the same as the complementary strand of HUMGS00418 and a primer (SEQ ID NO: 7840) complementary to the vector (pSPORT) sequence that is located external to the 5' end of the cDNA, a full length cDNA clone encoding a human ribosomal protein homologous to rat L9 was isolated (Hori et al., Nucl. Acids Res. 21:4395, 1993).

[10C. A human protein homologous to bovine phosphatidylethanolamine-binding protein. Cloning of the full length cDNA by hybridization using a probe comprising a partial sequence of a GS]

By hybridization with the probe,

5'-GATCGTTCTTCATGGGGGTAAGAAAAGCTGGTCTGGAGTTGCTGAATG

TTGCATTAATTGTCCTGTTTGCTTGTAGTTGAATAAAAATAGAAACCTGAAT

GAAGGAAA-3' (SEQ ID NO:7838),

that comprises a partial sequence of HUMGS00421, a full length cDNA clone encoding a human protein homologous to bovine phosphatidylethanolamine-binding protein was isolated (Hori et al., Gene 140:293, 1994).

[10D. Human mpl-ligand. Cloning of a cDNA coding for the human mpl-ligand using a GS]

This embodiment employs the 5' SLIC (single ligation to single stranded cDNA) method which is an improved version of the 5'RACE (rapid amplification of cDNA ends) method, and is described in Nucleic Acids Res., 19, 5227-5232 (1991).

① Reverse transcription of cDNA and attachment of anchor

The template was prepared using the reagents of the 5'-Amplifinder™ Kit (Toyobo, Inc.) in accordance with the protocol included therewith. Specifically, 2μg of human fetal liver poly $A^+$RNA (Clontech Laboratories, Inc.) and 10 pmol of the primer PA-6, a primer corresponding to the 3' end of the gene signature (GS) sequence HUMGS02342 and consisting of the sequence 5'-TTTTCGGCGCTCCCATTTATTCCTT-3' (SEQ ID NO: 7842), were mixed together and then denatured by heating the mixture at 65°C for 5 min. The cDNA was synthesized by combining the denatured sample with AMW reverse transcriptase, RNase inhibitor, dNTPs, and a reaction buffer, and then heating the resultant mixture at 52°C for 30 min. EDTA was then added to the mixture to stop the reaction. Thereafter, the RNA was hydrolyzed by adding NaOH to the reaction mixture and heating the resultant mixture at 65°C for 30 min. The mixture was then neutralized with acetic acid. A suspension of glass beads (GENO-BIND™) and NaI were added to the neutralized solution and the cDNA was adsorbed onto the beads. The cDNA, adsorbed onto the beads, was washed with an aqueous solution of 80% EtOH, and then eluted in 50 μl of distilled water. Glycogen was added to the solution of purified cDNA, and the cDNA was precipitated with EtOH and resuspended in 6 μl of distilled water. The resultant suspension (2.5 μl) was added to a solution containing 4 pmol of AmpliFINDER Anchor (5'-CACGAATTCACTATCGATTCTGGAACCTTCAGAGG $NH_2$-3') (SEQ ID NO: 7843) provided with the Kit, T4 RNA ligase, and a ligation (reaction) buffer. The reaction mixture was incubated at room temperature overnight, and the AmpliFINDER Anchor primer in the reaction mixture was thereby ligated to the 3' end of the cDNA. The ligated product was then used as a template for the subsequent PCR.

② Amplification by PCR

The primary PCR was carried out using the template produced in the procedure described above (①), the Anchor primer, 5'-CTGGTTCGGCCCACCTCTGAAGGTTCCAGAATCGATAG-3' (SEQ ID NO: 7846) and the PA-5 primer consisting of the sequence 5'-CTCGCTCGCCCATCCTTATACAGGCTCAGTTTTGTCT-3' (SEQ ID NO: 7844). Specifically, 1 μl of the template was mixed with Taq DNA polymerase (Takara Shuzo Inc., Code No. R001A), dNTPs, a PCR buffer, and 10 pmol each of the PA-5 primer and Anchor primer. The resultant reaction mixture was diluted with distilled water to a final volume of 50 μl and the PCR was performed in a DNA Thermal Cycler 480 (Perkin Elmer Cetus Corp.). The reaction mixture was subjected to 40 cycles of the PCR, wherein each cycle consisted of incubating the sample in sequence at 94°C for 1 min, 63°C for 1 min, and 72°C for 3 min and, in the last PCR cycle, at 72°C for an additional 8 min. The products of the PCR were resolved by electrophoresis in a 1% agarose gel and a broad band of approximately 800 bp in length, representing a product of the PCR, was detected. The detected band was excised from the agarose gel and the DNA contained therein was recovered using a Sephaglas Bandprep Kit™ (Pharmacia Corp.) in accordance with the protocol included therewith. Specifically, the gel was dissolved in a solution of NaI and the resultant mixture was heated at 60 °C for 10 min. Sephaglas™ BP was added to the gel mixture and the DNA was adsorbed onto the glass beads contained therein. The glass beads, containing the adsorbed DNA, were then washed three times with a Wash Buffer provided with the Kit and eluted in 30 µl of TE buffer (10 mM Tris-HCl pH 8.0, 1mM EDTA).

One µl of the eluted DNA was used as a template in a secondary PCR. In order to enhance the specificity of the secondary PCR, the reaction was performed with PA-4 primer which consisted of the sequence 5'-CTCGCTCGCCCATGTATAGGGACAGCATTTCTGAGAG-3' (SEQ ID NO: 7845) and was positioned within the template sequence internal to the PA-5 primer and the Anchor primer. Specifically, 1 µl of the template was mixed with 2.5 units of Taq DNA polymerase (Takara Shuzo Inc., Code No. R001A), dNTPs, a PCR buffer, and 10 pmol each of the PA-4 primer and Anchor primer. The resultant reaction mixture was diluted with distilled water to a final volume of 50 µl preheated at 94 °C for 6 min, and the secondary PCR was then performed under the same conditions described above (①) for the primary PCR. The products of the secondary PCR were resolved by electrophoresis in a 1% agarose gel and a broad band of approximately 800 bp in length, representing a product of the PCR, was detected . The detected band was excised from the agarose gel and the DNA contained therein was recovered and purified under the same conditions as described above (①) for the primary PCR.

③ Subcloning into plasmid vector

The purified DNA product of the secondary PCR was subcloned into the plasmid vector pUC18 (pharmacia Corp.), using a SureClone™ Ligation Kit (Pharmacia Corp.) in accordance with the protocol included therewith. Specifically, the purified DNA was added to a solution containing Klenow polymerase, polynucleotide kinase and a reaction buffer, mixed and heated at 37 °C for 30 min in order to create blunt-ended termini and to phosphorylate the 5' terminus of the DNA molecules contained in the reaction mixture. The blunt-ended and phosphorylated DNA was combined with a solution containing 50 ng of a dephosphorylated and Sma I-cleaved pUC18 vector provided with the Ligation Kit, T4 DNA ligase, DTT and a ligation reaction buffer, and the resultant mixture was warmed at 16 °C for 3 hr. One sixth volume of the reaction solution was employed to transform E. coil competent cells using standard methods. Specifically frozen E. coli competent cells (Wako Pure Chemical Industries, Ltd.) were thawed and mixed with the ligated DNA. The resultant mixture was incubated on ice for 20 min, heat-treated at 42 °C for 45 sec, and then incubated on ice for 2 min. A medium [Hi-Competence Broth (Wako Pure Chemical Industries, Ltd.)] was added to the mixture containing the transformed E. coli cells. The mixture was incubated for 37 °C for 1 hr and then spread onto agar plates containing 100 µg/ml Ampicillin, 40 µg/ml X-Gal (6-bromo-4-chloro-3-indolyl-$\beta$-D-galactoside), 0.1 mM IPTG (isopropyl-$\beta$-D-thiogalactopyranoside) and cultured overnight at 37 °C. White colonies were selected from the colonies which consequently appeared on the agar plates and analyzed by the PCR to determine the presence or absence of the DNA insert. Specifically, a sample of a selected colony was picked with a sterilized toothpick and used to inoculate a 50 µl reaction solution containing 1 unit of Taq DNA polymerase, dNTPs, PCR buffer, 200 µM each of the M13 P4-22 primer consisting of the sequence 5'-CCAGGGTTTTCCCAGTCACGAC-3' (SEQ ID No: 7847) and M13 P5-22 primer consisting of the sequence 5'-TCACACAGGAAACAGCTATGAC-3' (SEQ ID No: 7848), wherein both primers are comprised of sequences complementary to the pUC18 vector. The resultant mixture was heated at 94 °C for 6 min and then subjected to 30 cycles of the PCR wherein each cycle consisted of incubating the sample in sequence, at 94 °C for 1 min, 55 °C for 1 min, and 72 °C for 2 min. The amplified insert was detected by electrophoresis of the PCR products on an agarose gel and thereby the clone pR02342-2, containing an insert, was selected.

④ Sequencing of cDNA

The plasmid DNA was prepared using the QIAPrep-Spin Kit (Funakoshi, Ltd.) in accordance with the standard alkali-SDS protocol included therewith. Specifically, E. coli cells transformed with the DNA of clone pR02342-2 were cultured overnight in Luria Broth medium containing 100 µg/ml Ampicillin. The cultured cells were then pelleted by centrifugation and resuspended in P1 solution provided in the Kit. The resultant cell suspension was mixed with the P2 alkali solution of the Kit, incubated at room temperature for 5 min, neutralized with N3 solution of the Kit, incubated on ice for an additional 5 min and then centrifuged. The supernatant obtained from the centrifuged solution was applied to a QIAPrep-Spin column. The Spin column was then washed in sequence with PB and then PE solution of the Kit and the DNA was eluted from the column with TE buffer. Sequencing of the eluted DNA was then carried out using the sequencing kit PRISM™ Terminator Mix (Applied Biosystem Corp). Approximately 1 μg of the purified DNA was mixed with a solution containing 3.3 pmol of either the M13 P4-22 primer or M13 P5-22 primer and 9.5 μl of PRISM™ Terminator Mix. The M13 P4-22 and M13 P5-22 primer were used to sequence both strands of the DNA insert of clone pR02342-2. The resultant mixture was diluted to a final volume of 20 μl with distilled water and subjected to 25 cycles of the PCR wherein each cycle consisted of incubating the sample in sequence at 96°C for 30 sec, 50 °C for 15 sec, and 60 °C for 4 min. The excess primers and fluorescent dye present in the reaction mixture were removed by gel filtration using a MicroSpin™ S-200 HR column (Pharmacia Corp.) and the DNA products of the sequencing reaction were precipitated with EtOH. The precipitated DNA was resuspended, sequenced using an automated sequencer, "Model 373A" (Applied Biosystem Corp.), and thereafter analyzed to determine the nucleotide sequence.

The analysis of the nucleotide sequence revealed that the insert of clone pR02342-2, including the PA-4 primer, was 608 bp in length. The sequence of this insert was subjected to a search for homologous sequences entered in the Gen Bank data base, and a 100% match was found to a sequence in the cDNA which encodes the human mpl-ligand (Accession No. L 33410, Nature 369, 533-538, 1994). Further comparison of the insert of clone pR02342-2 with the cDNA sequence of the human mpl-ligand revealed that the cloned insert contained 81 bp of the 3' coding region of open reading frame. In addition, the insert of clone pR02342-2 contained an additional sequence extending beyond the 3' end of the human mpl-ligand cDNA sequence registered under Gen Bank Accession No. L 33410. These findings suggest that, using the GS HUMGS02342, the inventors of the present invention succeeded in cloning a cDNA clone pR02342-2, which could possibly have a different and more desirable property for expression than the human mpl-ligand cDNA represented by the sequence registered under Gen Bank Accession No. L 33410.

⑤ Cloning of the full-length cDNA encoding the human mpl-ligand

In order to find an optimal PCR primer, an appropriate computer program is used to search the sequence downstream of the coding region of the human mpl-ligand (clone pR02342-2) and thereby a primer PA-7 is designed and synthesized. A PCR similar to that described above in ② is performed using the template produced by the procedure described above in ①, the Anchor primer, and the PA-7 primer. Specifically, 1 μl of the template is mixed with 2.5 units of Taq DNA polymerase (Takara Shuzo Inc., Code No. R001A), dNTPs, a PCR buffer, and 10 pmol each of the PA-7 primer and Anchor primer. The resultant reaction mixture is diluted with distilled water to a final volume of 50 μl and the PCR is performed in a DNA Thermal Cycler 480 (Perkin Elmer Cetus Corp.) under conditions similar to that described above in ②. The products of the PCR are then resolved by electrophoresis on a 1% agarose gel and a band greater than 1300 bp in length, representing a product of the PCR, is recovered and cloned into a suitable vector in a manner similar to that described in ③. The cloned DNA is sequenced in a manner similar to that described in ④. The sequence is then compared to that of the human mpl-ligand cDNA registered under Gen Bank Accession No. L 33410 to confirm the presence of the full-length open reading frame.

Alternatively, using the Takara La PCR Kit (Takara Shuzo Inc., Code No. RR011) in accordance with the protocol included therewith, performing the 5'RACE procedure using primers similar to those described above in ②, a cDNA of approximately 2 Kb in length, corresponding to the human mpl-ligand, was isolated.

The tables of appearance frequencies for all GSs related to the present invention are followed by "Sequence Listing" for these GSs, wherein HUMGS numbers after the heading 'clone' represent GS numbers. In the sequence table, N in the base sequence stands for "A or C or G or T or U". However, since nucleic acids in the Sequence Listing are DNAs, "T or U" stands for T in this case.

By the present invention, it has become possible to provide DNA molecules which carry "the information for expression" in various cells and can be used for detecting and diagnosing the cellular abnormalities, recognizing and identifying cells and further efficiently cloning genes which are expressed in a tissue-specific manner, and furthermore cloned DNA molecules which can be used for the production of proteins useful as pharmaceutical products.

| | A | B | C | E | G | I | K | M | O | Q | S | U | W | Y | AA | AC | AE | AG | AI | AK | AM | AO | AQ | AS | AU | AW | AY | BA | BC | BE | BF | BG | BH | BI | BK |
|---|---|---|---|---|---|---|---|---|---|---|---|---|---|---|---|---|---|---|---|---|---|---|---|---|---|---|---|---|---|---|---|---|---|---|---|
| 1 | GS | Sequence No. | F | HepG2 | mmHL60 | pmHL60+DMSO | mpHL60+TPA | 40w liver | 19w liver | adult liver | lung | subcutaneous adipose | aortic endothel | osteoblast | colon mucosa | small cell carcinoma of lung | retina | cerebral cortex | adenocarcinoma of lung | squamous cell carcinoma of lung | keratinocyte | fibroblast | Alzheimer disease | cerebellum | visceral fat | corneal epithelium | peripheral granulocyte | neuroblastoma | taste bud of tongue | Accession number of target mRNA | match % | match size | match starts at (GS) | match starts at (GenBank) | GenBank target size |
| 2 | 00001 | 00001 | 10 | 1 | 0 | 0 | 0 | 0 | 0 | 0 | 0 | 0 | 0 | 0 | 0 | 0 | 0 | 0 | 2 | 0 | 0 | 2 | 0 | 0 | 0 | 5 | 0 | 0 | 0 | M77349 | 95.2 | 461 | 1 | 2003 | 2691 |
| 3 | 00002 | 00002 | 1 | 1 | 0 | 0 | 0 | 0 | 0 | 0 | 0 | 0 | 0 | 0 | 0 | 0 | 0 | 0 | 0 | 0 | 0 | 0 | 0 | 0 | 0 | 0 | 0 | 0 | 0 | | | | | | |
| 4 | 00003 | 00003 | 3 | 1 | 0 | 0 | 0 | 0 | 0 | 0 | 1 | 0 | 0 | 0 | 0 | 0 | 0 | 0 | 0 | 0 | 0 | 0 | 0 | 0 | 0 | 0 | 0 | 1 | 0 | J05249 | 94.8 | 620 | 1 | 894 | 1512 |
| 5 | 00004 | 00004 | 5 | 2 | 0 | 0 | 0 | 0 | 0 | 0 | 0 | 0 | 0 | 0 | 0 | 0 | 0 | 0 | 0 | 0 | 0 | 0 | 0 | 0 | 0 | 0 | 0 | 3 | 0 | | | | | | |
| 6 | 00005 | 00005 | 1 | 1 | 0 | 0 | 0 | 0 | 0 | 0 | 0 | 0 | 0 | 0 | 0 | 0 | 0 | 0 | 0 | 0 | 0 | 0 | 0 | 0 | 0 | 0 | 0 | 0 | 0 | X72875 | 91 | 613 | 1 | 1737 | 2345 |
| 7 | 00006 | 00006 | 2 | 1 | 0 | 0 | 0 | 0 | 0 | 0 | 0 | 0 | 0 | 0 | 0 | 0 | 0 | 1 | 0 | 0 | 0 | 0 | 0 | 0 | 0 | 0 | 0 | 0 | 0 | | | | | | |
| 8 | 00007 | 00007 | 2 | 1 | 0 | 0 | 0 | 0 | 0 | 0 | 0 | 0 | 0 | 1 | 0 | 0 | 0 | 0 | 0 | 0 | 0 | 0 | 0 | 0 | 0 | 0 | 0 | 0 | 0 | | | | | | |
| 9 | 00008 | 00008 | 3 | 1 | 0 | 0 | 0 | 0 | 0 | 0 | 0 | 0 | 0 | 0 | 0 | 0 | 0 | 0 | 0 | 0 | 0 | 0 | 1 | 1 | 0 | 0 | 0 | 0 | 0 | | | | | | |
| 10 | 00009 | 00009 | 2 | 1 | 0 | 0 | 0 | 0 | 0 | 0 | 0 | 0 | 0 | 0 | 0 | 0 | 1 | 0 | 0 | 0 | 0 | 0 | 0 | 0 | 0 | 0 | 0 | 0 | 0 | | | | | | |
| 11 | 00010 | 00010 | 1 | 1 | 0 | 0 | 0 | 0 | 0 | 0 | 0 | 0 | 0 | 0 | 0 | 0 | 0 | 0 | 0 | 0 | 0 | 0 | 0 | 0 | 0 | 0 | 0 | 0 | 0 | Y00856 | 90.3 | 607 | 1 | 784 | 1397 |
| 12 | 00011 | 00011 | 3 | 2 | 0 | 0 | 0 | 0 | 0 | 0 | 0 | 0 | 0 | 0 | 0 | 0 | 0 | 0 | 0 | 0 | 0 | 0 | 0 | 0 | 0 | 0 | 0 | 1 | 0 | | | | | | |
| 13 | 00012 | 00012 | 2 | 2 | 0 | 0 | 0 | 0 | 0 | 0 | 0 | 0 | 0 | 0 | 0 | 0 | 0 | 0 | 0 | 0 | 0 | 0 | 0 | 0 | 0 | 0 | 0 | 0 | 0 | | | | | | |
| 14 | 00014 | 00013 | 3 | 2 | 0 | 0 | 0 | 0 | 0 | 0 | 0 | 0 | 0 | 0 | 0 | 0 | 0 | 0 | 0 | 0 | 0 | 0 | 0 | 0 | 0 | 1 | 0 | 0 | 0 | | | | | | |
| 15 | 00015 | 00014 | 1 | 1 | 0 | 0 | 0 | 0 | 0 | 0 | 0 | 0 | 0 | 0 | 0 | 0 | 0 | 0 | 0 | 0 | 0 | 0 | 0 | 0 | 0 | 0 | 0 | 0 | 0 | | | | | | |
| 16 | 00016 | 00015 | 1 | 1 | 0 | 0 | 0 | 0 | 0 | 0 | 0 | 0 | 0 | 0 | 0 | 0 | 0 | 0 | 0 | 0 | 0 | 0 | 0 | 0 | 0 | 0 | 0 | 0 | 0 | | | | | | |
| 17 | 00017 | 00016 | 1 | 1 | 0 | 0 | 0 | 0 | 0 | 0 | 0 | 0 | 0 | 0 | 0 | 0 | 0 | 0 | 0 | 0 | 0 | 0 | 0 | 0 | 0 | 0 | 0 | 0 | 0 | | | | | | |
| 18 | 00018 | 00017 | 25 | 3 | 2 | 1 | 1 | 0 | 1 | 0 | 0 | 1 | 2 | 0 | 0 | 2 | 1 | 1 | 0 | 2 | 0 | 0 | 0 | 0 | 0 | 0 | 0 | 4 | 4 | M84711 | 93.1 | 536 | 1 | 346 | 874 |
| 19 | 00019 | 00018 | 128 | 17 | 8 | 2 | 26 | 0 | 0 | 1 | 1 | 3 | 1 | 3 | 4 | 2 | 4 | 3 | 6 | 4 | 5 | 5 | 4 | 1 | 1 | 5 | 0 | 19 | 3 | X16869 | 94.6 | 479 | 1 | 1153 | 1696 |
| 20 | 00020 | 00019 | 1 | 1 | 0 | 0 | 0 | 0 | 0 | 0 | 0 | 0 | 0 | 0 | 0 | 0 | 0 | 0 | 0 | 0 | 0 | 0 | 0 | 0 | 0 | 0 | 0 | 0 | 0 | | | | | | |
| 21 | 00021 | 00020 | 3 | 2 | 0 | 0 | 0 | 0 | 0 | 0 | 0 | 0 | 0 | 0 | 0 | 0 | 0 | 0 | 0 | 0 | 0 | 0 | 0 | 0 | 0 | 0 | 0 | 1 | 0 | M31469 | 97.9 | 334 | 1 | 319 | 651 |

Table 1

| | A | B | C | E | G | I | K | M | O | Q | S | U | W | Y | AA | AC | AE | AG | AI | AK | AM | AO | AQ | AS | AU | AW | AY | BA | BC | BE | BF | BG | BH | BI | BK |
|---|---|---|---|---|---|---|---|---|---|---|---|---|---|---|---|---|---|---|---|---|---|---|---|---|---|---|---|---|---|---|---|---|---|---|---|
| 22 | 00023 | 00021 | 1 | 1 | 0 | 0 | 0 | 0 | 0 | 0 | 0 | 0 | 0 | 0 | 0 | 0 | 0 | 0 | 0 | 0 | 0 | 0 | 0 | 0 | 0 | 0 | 0 | 0 | 0 | | | | | | |
| 23 | 00026 | 00022 | 2 | 1 | 0 | 0 | 0 | 0 | 0 | 0 | 0 | 0 | 0 | 0 | 0 | 0 | 0 | 0 | 0 | 1 | 0 | 0 | 0 | 0 | 0 | 0 | 0 | 0 | 0 | | | | | | |
| 24 | 00028 | 00023 | 1 | 1 | 0 | 0 | 0 | 0 | 0 | 0 | 0 | 0 | 0 | 0 | 0 | 0 | 0 | 0 | 0 | 0 | 0 | 0 | 0 | 0 | 0 | 0 | 0 | 0 | 0 | J03225 | 90.8 | 534 | 1 | 520 | 1431 |
| 25 | 00029 | 00024 | 5 | 4 | 0 | 1 | 0 | 0 | 0 | 0 | 0 | 0 | 0 | 0 | 0 | 0 | 0 | 0 | 0 | 0 | 0 | 0 | 0 | 0 | 0 | 0 | 0 | 0 | 0 | | | | | | |
| 26 | 00030 | 00025 | 2 | 2 | 0 | 0 | 0 | 0 | 0 | 0 | 0 | 0 | 0 | 0 | 0 | 0 | 0 | 0 | 0 | 0 | 0 | 0 | 0 | 0 | 0 | 0 | 0 | 0 | 0 | | | | | | |
| 27 | 00031 | 00026 | 1 | 1 | 0 | 0 | 0 | 0 | 0 | 0 | 0 | 0 | 0 | 0 | 0 | 0 | 0 | 0 | 0 | 0 | 0 | 0 | 0 | 0 | 0 | 0 | 0 | 0 | 0 | | | | | | |
| 28 | 00033 | 00027 | 1 | 1 | 0 | 0 | 0 | 0 | 0 | 0 | 0 | 0 | 0 | 0 | 0 | 0 | 0 | 0 | 0 | 0 | 0 | 0 | 0 | 0 | 0 | 0 | 0 | 0 | 0 | | | | | | |
| 29 | 00034 | 00028 | 8 | 1 | 0 | 0 | 0 | 0 | 0 | 0 | 0 | 0 | 0 | 0 | 0 | 0 | 1 | 1 | 0 | 1 | 1 | 0 | 1 | 1 | 1 | 0 | 0 | 0 | 0 | | | | | | |
| 30 | 00036 | 00029 | 1 | 1 | 0 | 0 | 0 | 0 | 0 | 0 | 0 | 0 | 0 | 0 | 0 | 0 | 0 | 0 | 0 | 0 | 0 | 0 | 0 | 0 | 0 | 0 | 0 | 0 | 0 | | | | | | |
| 31 | 00037 | 00030 | 1 | 1 | 0 | 0 | 0 | 0 | 0 | 0 | 0 | 0 | 0 | 0 | 0 | 0 | 0 | 0 | 0 | 0 | 0 | 0 | 0 | 0 | 0 | 0 | 0 | 0 | 0 | | | | | | |
| 32 | 00038 | 00031 | 1 | 1 | 0 | 0 | 0 | 0 | 0 | 0 | 0 | 0 | 0 | 0 | 0 | 0 | 0 | 0 | 0 | 0 | 0 | 0 | 0 | 0 | 0 | 0 | 0 | 0 | 0 | | | | | | |
| 33 | 00039 | 00032 | 7 | 1 | 0 | 0 | 0 | 0 | 0 | 0 | 0 | 0 | 0 | 0 | 0 | 0 | 1 | 0 | 0 | 1 | 0 | 0 | 0 | 0 | 0 | 0 | 0 | 2 | 2 | M77233 | 95.4 | 346 | 1 | 16 | 600 |
| 34 | 00040 | 00033 | 5 | 1 | 0 | 0 | 0 | 0 | 0 | 0 | 0 | 0 | 0 | 0 | 0 | 0 | 0 | 0 | 0 | 0 | 0 | 0 | 0 | 0 | 0 | 0 | 1 | 2 | 1 | | | | | | |
| 35 | 00041 | 00034 | 2 | 2 | 0 | 0 | 0 | 0 | 0 | 0 | 0 | 0 | 0 | 0 | 0 | 0 | 0 | 0 | 0 | 0 | 0 | 0 | 0 | 0 | 0 | 0 | 0 | 0 | 0 | | | | | | |
| 36 | 00042 | 00035 | 9 | 1 | 0 | 0 | 0 | 0 | 0 | 0 | 0 | 0 | 0 | 0 | 0 | 0 | 0 | 1 | 0 | 0 | 0 | 0 | 5 | 2 | 0 | 0 | 0 | 0 | 0 | | | | | | |
| 37 | 00043 | 00036 | 9 | 2 | 0 | 0 | 1 | 0 | 0 | 0 | 0 | 1 | 0 | 0 | 0 | 1 | 0 | 0 | 0 | 0 | 0 | 0 | 0 | 1 | 0 | 0 | 0 | 3 | 0 | | | | | | |
| 38 | 00044 | 00037 | 1 | 1 | 0 | 0 | 0 | 0 | 0 | 0 | 0 | 0 | 0 | 0 | 0 | 0 | 0 | 0 | 0 | 0 | 0 | 0 | 0 | 0 | 0 | 0 | 0 | 0 | 0 | | | | | | |
| 39 | 00045 | 00038 | 5 | 3 | 1 | 0 | 0 | 0 | 0 | 0 | 0 | 0 | 0 | 0 | 0 | 0 | 0 | 0 | 0 | 0 | 0 | 0 | 0 | 1 | 0 | 0 | 0 | 0 | 0 | | | | | | |
| 40 | 00046 | 00039 | 3 | 1 | 0 | 0 | 0 | 0 | 0 | 0 | 0 | 1 | 0 | 0 | 0 | 0 | 0 | 0 | 0 | 0 | 0 | 0 | 0 | 0 | 0 | 0 | 0 | 1 | 0 | | | | | | |
| 41 | 00047 | 00040 | 3 | 1 | 0 | 0 | 0 | 0 | 0 | 0 | 1 | 0 | 0 | 0 | 0 | 0 | 0 | 0 | 0 | 0 | 0 | 0 | 0 | 0 | 0 | 1 | 0 | 0 | 0 | | | | | | |
| 42 | 00048 | 00041 | 10 | 2 | 0 | 0 | 0 | 0 | 0 | 0 | 1 | 0 | 0 | 0 | 0 | 0 | 0 | 0 | 1 | 2 | 0 | 2 | 0 | 1 | 0 | 0 | 0 | 1 | 0 | X67951 | 94.4 | 322 | 1 | 483 | 937 |
| 43 | 00049 | 00042 | 1 | 1 | 0 | 0 | 0 | 0 | 0 | 0 | 0 | 0 | 0 | 0 | 0 | 0 | 0 | 0 | 0 | 0 | 0 | 0 | 0 | 0 | 0 | 0 | 0 | 0 | 0 | X57847 | 93.6 | 392 | 1 | 348 | 1182 |
| 44 | 00050 | 00043 | 16 | 1 | 0 | 2 | 0 | 0 | 1 | 0 | 1 | 0 | 1 | 0 | 0 | 1 | 3 | 0 | 3 | 0 | 1 | 0 | 0 | 0 | 0 | 0 | 0 | 2 | 0 | M24070 | 96 | 421 | 1 | 1039 | 1452 |
| 45 | 00051 | 00044 | 1 | 1 | 0 | 0 | 0 | 0 | 0 | 0 | 0 | 0 | 0 | 0 | 0 | 0 | 0 | 0 | 0 | 0 | 0 | 0 | 0 | 0 | 0 | 0 | 0 | 0 | 0 | | | | | | |
| 46 | 00053 | 00045 | 4 | 1 | 0 | 2 | 1 | 0 | 0 | 0 | 0 | 0 | 0 | 0 | 0 | 0 | 0 | 0 | 0 | 0 | 0 | 0 | 0 | 0 | 0 | 0 | 0 | 0 | 0 | | | | | | |
| 47 | 00055 | 00046 | 1 | 1 | 0 | 0 | 0 | 0 | 0 | 0 | 0 | 0 | 0 | 0 | 0 | 0 | 0 | 0 | 0 | 0 | 0 | 0 | 0 | 0 | 0 | 0 | 0 | 0 | 0 | L13388 | 93.4 | 317 | 75 | 2 | 359 |
| 48 | 00056 | 00047 | 1 | 1 | 0 | 0 | 0 | 0 | 0 | 0 | 0 | 0 | 0 | 0 | 0 | 0 | 0 | 0 | 0 | 0 | 0 | 0 | 0 | 0 | 0 | 0 | 0 | 0 | 0 | | | | | | |
| 49 | 00057 | 00048 | 1 | 1 | 0 | 0 | 0 | 0 | 0 | 0 | 0 | 0 | 0 | 0 | 0 | 0 | 0 | 0 | 0 | 0 | 0 | 0 | 0 | 0 | 0 | 0 | 0 | 0 | 0 | | | | | | |
| 50 | 00060 | 00049 | 3 | 1 | 0 | 0 | 0 | 0 | 1 | 0 | 0 | 0 | 0 | 0 | 0 | 0 | 0 | 0 | 0 | 0 | 1 | 0 | 0 | 0 | 0 | 0 | 0 | 0 | 0 | | | | | | |
| 51 | 00061 | 00050 | 2 | 2 | 0 | 0 | 0 | 0 | 0 | 0 | 0 | 0 | 0 | 0 | 0 | 0 | 0 | 0 | 0 | 0 | 0 | 0 | 0 | 0 | 0 | 0 | 0 | 0 | 0 | | | | | | |
| 52 | 00062 | 00051 | 1 | 1 | 0 | 0 | 0 | 0 | 0 | 0 | 0 | 0 | 0 | 0 | 0 | 0 | 0 | 0 | 0 | 0 | 0 | 0 | 0 | 0 | 0 | 0 | 0 | 0 | 0 | | | | | | |
| 53 | 00064 | 00052 | 2 | 1 | 0 | 0 | 0 | 0 | 0 | 0 | 0 | 0 | 1 | 0 | 0 | 0 | 0 | 0 | 0 | 0 | 0 | 0 | 0 | 0 | 0 | 0 | 0 | 0 | 0 | | | | | | |
| 54 | 00065 | 00053 | 1 | 1 | 0 | 0 | 0 | 0 | 0 | 0 | 0 | 0 | 0 | 0 | 0 | 0 | 0 | 0 | 0 | 0 | 0 | 0 | 0 | 0 | 0 | 0 | 0 | 0 | 0 | | | | | | |
| 55 | 00066 | 00054 | 1 | 1 | 0 | 0 | 0 | 0 | 0 | 0 | 0 | 0 | 0 | 0 | 0 | 0 | 0 | 0 | 0 | 0 | 0 | 0 | 0 | 0 | 0 | 0 | 0 | 0 | 0 | | | | | | |
| 56 | 00067 | 00055 | 2 | 1 | 0 | 0 | 0 | 0 | 0 | 0 | 0 | 0 | 0 | 0 | 0 | 0 | 0 | 0 | 1 | 0 | 0 | 0 | 0 | 0 | 0 | 0 | 0 | 0 | 0 | | | | | | |
| 57 | 00068 | 00056 | 4 | 1 | 0 | 0 | 0 | 0 | 0 | 0 | 0 | 0 | 0 | 2 | 0 | 0 | 0 | 0 | 0 | 1 | 0 | 0 | 0 | 0 | 0 | 0 | 0 | 0 | 0 | | | | | | |

Table 2

| | A | B | C | E | G | I | K | M | O | Q | S | U | W | Y | AA | AC | AE | AG | AI | AK | AM | AO | AQ | AS | AU | AW | AY | BA | BC | BE | BF | BG | BH | BI | BK |
|---|---|---|---|---|---|---|---|---|---|---|---|---|---|---|---|---|---|---|---|---|---|---|---|---|---|---|---|---|---|---|---|---|---|---|---|
| 58 | 00069 | 00057 | 4 | 1 | 0 | 0 | 0 | 0 | 0 | 0 | 0 | 0 | 0 | 0 | 0 | 0 | 0 | 0 | 1 | 0 | 0 | 0 | 0 | 1 | 0 | 0 | 0 | 1 | 0 | M34664 | 92.4 | 344 | 1 | 1484 | 2202 |
| 59 | 00070 | 00058 | 1 | 1 | 0 | 0 | 0 | 0 | 0 | 0 | 0 | 0 | 0 | 0 | 0 | 0 | 0 | 0 | 0 | 0 | 0 | 0 | 0 | 0 | 0 | 0 | 0 | 0 | 0 | | | | | | |
| 60 | 00071 | 00059 | 7 | 1 | 0 | 3 | 0 | 0 | 0 | 0 | 0 | 0 | 1 | 0 | 0 | 0 | 0 | 0 | 0 | 0 | 0 | 0 | 0 | 0 | 0 | 0 | 0 | 2 | 0 | | | | | | |
| 61 | 00072 | 00060 | 1 | 1 | 0 | 0 | 0 | 0 | 0 | 0 | 0 | 0 | 0 | 0 | 0 | 0 | 0 | 0 | 0 | 0 | 0 | 0 | 0 | 0 | 0 | 0 | 0 | 0 | 0 | M16961 | 93.4 | 376 | 1 | 1131 | 1538 |
| 62 | 00073 | 00061 | 1 | 1 | 0 | 0 | 0 | 0 | 0 | 0 | 0 | 0 | 0 | 0 | 0 | 0 | 0 | 0 | 0 | 0 | 0 | 0 | 0 | 0 | 0 | 0 | 0 | 0 | 0 | | | | | | |
| 63 | 00074 | 00062 | 1 | 1 | 0 | 0 | 0 | 0 | 0 | 0 | 0 | 0 | 0 | 0 | 0 | 0 | 0 | 0 | 0 | 0 | 0 | 0 | 0 | 0 | 0 | 0 | 0 | 0 | 0 | | | | | | |
| 64 | 00075 | 00063 | 1 | 1 | 0 | 0 | 0 | 0 | 0 | 0 | 0 | 0 | 0 | 0 | 0 | 0 | 0 | 0 | 0 | 0 | 0 | 0 | 0 | 0 | 0 | 0 | 0 | 0 | 0 | | | | | | |
| 65 | 00076 | 00064 | 2 | 1 | 0 | 0 | 0 | 0 | 0 | 0 | 0 | 0 | 1 | 0 | 0 | 0 | 0 | 0 | 0 | 0 | 0 | 0 | 0 | 0 | 0 | 0 | 0 | 0 | 0 | | | | | | |
| 66 | 00077 | 00065 | 34 | 1 | 3 | 2 | 5 | 1 | 0 | 0 | 2 | 2 | 0 | 1 | 1 | 1 | 1 | 0 | 1 | 0 | 4 | 0 | 0 | 0 | 1 | 3 | 0 | 5 | 0 | D23660 | 97.9 | 439 | 1 | 980 | 1418 |
| 67 | 00078 | 00066 | 12 | 1 | 0 | 1 | 1 | 0 | 0 | 0 | 1 | 0 | 0 | 0 | 0 | 0 | 0 | 1 | 1 | 0 | 2 | 2 | 0 | 0 | 1 | 0 | 0 | 1 | 0 | X07979 | 90.9 | 572 | 1 | 3054 | 3614 |
| 68 | 00079 | 00067 | 2 | 1 | 0 | 0 | 0 | 0 | 0 | 0 | 1 | 0 | 0 | 0 | 0 | 0 | 0 | 0 | 0 | 0 | 0 | 0 | 0 | 0 | 0 | 0 | 0 | 0 | 0 | U04953 | 94.4 | 533 | 1 | 3741 | 4508 |
| 69 | 00080 | 00068 | 4 | 1 | 0 | 0 | 0 | 0 | 0 | 0 | 0 | 0 | 0 | 0 | 0 | 0 | 0 | 0 | 0 | 0 | 0 | 0 | 0 | 1 | 0 | 0 | 2 | 0 | 0 | L08246 | 93 | 415 | 4 | 3501 | 3934 |
| 70 | 00081 | 00069 | 1 | 1 | 0 | 0 | 0 | 0 | 0 | 0 | 0 | 0 | 0 | 0 | 0 | 0 | 0 | 0 | 0 | 0 | 0 | 0 | 0 | 0 | 0 | 0 | 0 | 0 | 0 | X70218 | 91.6 | 250 | 1 | 837 | 1382 |
| 71 | 00082 | 00070 | 1 | 1 | 0 | 0 | 0 | 0 | 0 | 0 | 0 | 0 | 0 | 0 | 0 | 0 | 0 | 0 | 0 | 0 | 0 | 0 | 0 | 0 | 0 | 0 | 0 | 0 | 0 | | | | | | |
| 72 | 00083 | 00071 | 1 | 1 | 0 | 0 | 0 | 0 | 0 | 0 | 0 | 0 | 0 | 0 | 0 | 0 | 0 | 0 | 0 | 0 | 0 | 0 | 0 | 0 | 0 | 0 | 0 | 0 | 0 | | | | | | |
| 73 | 00084 | 00072 | 5 | 1 | 0 | 0 | 0 | 0 | 0 | 0 | 0 | 0 | 0 | 0 | 0 | 0 | 0 | 0 | 1 | 1 | 0 | 0 | 0 | 0 | 0 | 2 | 0 | 0 | 0 | K03195 | 99.3 | 410 | 1 | 2134 | 2856 |
| 74 | 00085 | 00073 | 26 | 5 | 3 | 1 | 0 | 0 | 1 | 0 | 2 | 0 | 0 | 2 | 0 | 3 | 0 | 0 | 1 | 0 | 2 | 0 | 0 | 0 | 1 | 3 | 0 | 2 | 0 | M84334 | 94.6 | 406 | 1 | 1356 | 1760 |
| 75 | 00086 | 00074 | 4 | 1 | 0 | 0 | 0 | 0 | 1 | 0 | 1 | 0 | 0 | 0 | 0 | 0 | 0 | 0 | 0 | 0 | 0 | 1 | 0 | 0 | 0 | 0 | 0 | 0 | 0 | | | | | | |
| 76 | 00087 | 00075 | 1 | 1 | 0 | 0 | 0 | 0 | 0 | 0 | 0 | 0 | 0 | 0 | 0 | 0 | 0 | 0 | 0 | 0 | 0 | 0 | 0 | 0 | 0 | 0 | 0 | 0 | 0 | | | | | | |
| 77 | 00088 | 00076 | 2 | 1 | 0 | 0 | 0 | 0 | 0 | 0 | 0 | 0 | 0 | 0 | 1 | 0 | 0 | 0 | 0 | 0 | 0 | 0 | 0 | 0 | 0 | 0 | 0 | 0 | 0 | | | | | | |
| 78 | 00089 | 00077 | 33 | 1 | 3 | 2 | 2 | 0 | 1 | 0 | 1 | 0 | 5 | 0 | 0 | 2 | 0 | 0 | 1 | 4 | 7 | 0 | 0 | 0 | 1 | 1 | 0 | 2 | 0 | X17206 | 94.5 | 397 | 1 | 543 | 934 |
| 79 | 00090 | 00078 | 1 | 1 | 0 | 0 | 0 | 0 | 0 | 0 | 0 | 0 | 0 | 0 | 0 | 0 | 0 | 0 | 0 | 0 | 0 | 0 | 0 | 0 | 0 | 0 | 0 | 0 | 0 | | | | | | |
| 80 | 00091 | 00079 | 4 | 1 | 0 | 0 | 0 | 0 | 0 | 0 | 0 | 0 | 0 | 0 | 0 | 0 | 0 | 0 | 2 | 0 | 0 | 1 | 0 | 0 | 0 | 0 | 0 | 0 | 0 | M84326 | 95.1 | 487 | 1 | 1204 | 1815 |
| 81 | 00092 | 00080 | 2 | 2 | 0 | 0 | 0 | 0 | 0 | 0 | 0 | 0 | 0 | 0 | 0 | 0 | 0 | 0 | 0 | 0 | 0 | 0 | 0 | 0 | 0 | 0 | 0 | 0 | 0 | | | | | | |
| 82 | 00093 | 00081 | 2 | 2 | 0 | 0 | 0 | 0 | 0 | 0 | 0 | 0 | 0 | 0 | 0 | 0 | 0 | 0 | 0 | 0 | 0 | 0 | 0 | 0 | 0 | 0 | 0 | 0 | 0 | | | | | | |
| 83 | 00094 | 00082 | 9 | 1 | 0 | 1 | 1 | 0 | 0 | 0 | 0 | 1 | 2 | 0 | 0 | 0 | 0 | 0 | 0 | 1 | 1 | 0 | 0 | 0 | 0 | 0 | 0 | 1 | 0 | | | | | | |
| 84 | 00095 | 00083 | 2 | 1 | 0 | 0 | 0 | 0 | 0 | 0 | 0 | 0 | 0 | 0 | 0 | 0 | 0 | 0 | 0 | 0 | 1 | 0 | 0 | 0 | 0 | 0 | 0 | 0 | 0 | | | | | | |
| 85 | 00096 | 00084 | 62 | 6 | 5 | 4 | 2 | 0 | 0 | 1 | 2 | 6 | 5 | 1 | 3 | 1 | 1 | 5 | 3 | 3 | 3 | 0 | 1 | 1 | 2 | 0 | 0 | 6 | 1 | | | | | | |
| 86 | 00097 | 00085 | 2 | 2 | 0 | 0 | 0 | 0 | 0 | 0 | 0 | 0 | 0 | 0 | 0 | 0 | 0 | 0 | 0 | 0 | 0 | 0 | 0 | 0 | 0 | 0 | 0 | 0 | 0 | | | | | | |
| 87 | 00098 | 00086 | 7 | 1 | 0 | 0 | 0 | 2 | 3 | 1 | 0 | 0 | 0 | 0 | 0 | 0 | 0 | 0 | 0 | 0 | 0 | 0 | 0 | 0 | 0 | 0 | 0 | 0 | 0 | D14446 | 98.2 | 382 | 1 | 832 | 1215 |
| 88 | 00099 | 00087 | 9 | 2 | 0 | 0 | 0 | 0 | 0 | 2 | 0 | 1 | 0 | 0 | 0 | 1 | 0 | 0 | 2 | 0 | 0 | 0 | 0 | 0 | 1 | 0 | 0 | 0 | 0 | Z11793 | 96.1 | 385 | 1 | 1637 | 2038 |
| 89 | 00100 | 00088 | 3 | 1 | 0 | 0 | 0 | 0 | 0 | 0 | 0 | 0 | 1 | 0 | 0 | 0 | 0 | 0 | 0 | 1 | 0 | 0 | 0 | 0 | 0 | 0 | 0 | 0 | 0 | S65756 | 98.5 | 392 | 1 | 294 | 696 |
| 90 | 00101 | 00089 | 2 | 1 | 0 | 0 | 0 | 0 | 0 | 0 | 0 | 0 | 0 | 0 | 0 | 0 | 0 | 0 | 0 | 0 | 0 | 1 | 0 | 0 | 0 | 0 | 0 | 0 | 0 | | | | | | |
| 91 | 00102 | 00090 | 44 | 2 | 4 | 0 | 3 | 0 | 1 | 1 | 1 | 6 | 2 | 1 | 2 | 8 | 0 | 1 | 1 | 1 | 4 | 0 | 0 | 0 | 1 | 3 | 0 | 2 | 0 | X64707 | 93.3 | 387 | 1 | 346 | 942 |
| 92 | 00103 | 00091 | 4 | 2 | 0 | 0 | 0 | 0 | 2 | 0 | 0 | 0 | 0 | 0 | 0 | 0 | 0 | 0 | 0 | 0 | 0 | 0 | 0 | 0 | 0 | 0 | 0 | 0 | 0 | J00077 | 99.7 | 380 | 1 | 1653 | 2032 |
| 93 | 00104 | 00092 | 2 | 1 | 0 | 0 | 0 | 0 | 0 | 0 | 0 | 0 | 0 | 0 | 0 | 0 | 1 | 0 | 0 | 0 | 0 | 0 | 0 | 0 | 0 | 0 | 0 | 0 | 0 | | | | | | |

Table 3

| | A | B | C | E | G | I | K | M | O | Q | S | U | W | Y | AA | AC | AE | AG | AI | AK | AM | AO | AQ | AS | AU | AW | AY | BA | BC | BE | BF | BG | BH | BI | BK |
|---|---|---|---|---|---|---|---|---|---|---|---|---|---|---|---|---|---|---|---|---|---|---|---|---|---|---|---|---|---|---|---|---|---|---|---|
| 94 | 00105 | 00093 | 1 | 1 | 0 | 0 | 0 | 0 | 0 | 0 | 0 | 0 | 0 | 0 | 0 | 0 | 0 | 0 | 0 | 0 | 0 | 0 | 0 | 0 | 0 | 0 | 0 | 0 | 0 | | | | | | |
| 95 | 00106 | 00094 | 8 | 2 | 1 | 1 | 0 | 0 | 0 | 0 | 0 | 0 | 0 | 1 | 2 | 0 | 0 | 0 | 0 | 0 | 0 | 0 | 1 | 0 | 0 | 0 | 0 | 0 | 0 | | | | | | |
| 96 | 00107 | 00095 | 2 | 1 | 0 | 0 | 1 | 0 | 0 | 0 | 0 | 0 | 0 | 0 | 0 | 0 | 0 | 0 | 0 | 0 | 0 | 0 | 0 | 0 | 0 | 0 | 0 | 0 | 0 | | | | | | |
| 97 | 00108 | 00096 | 31 | 2 | 3 | 0 | 0 | 1 | 0 | 2 | 0 | 1 | 0 | 3 | 0 | 0 | 0 | 0 | 3 | 6 | 2 | 1 | 1 | 0 | 1 | 2 | 0 | 0 | 3 | X63237 | 94.8 | 384 | 1 | 154 | 532 |
| 98 | 00109 | 00097 | 5 | 1 | 0 | 0 | 2 | 0 | 0 | 0 | 0 | 0 | 0 | 0 | 0 | 0 | 0 | 0 | 0 | 0 | 1 | 1 | 0 | 0 | 0 | 0 | 0 | 0 | 0 | | | | | | |
| 99 | 00110 | 00098 | 2 | 1 | 0 | 0 | 1 | 0 | 0 | 0 | 0 | 0 | 0 | 0 | 0 | 0 | 0 | 0 | 0 | 0 | 0 | 0 | 0 | 0 | 0 | 0 | 0 | 0 | 0 | | | | | | |
| 100 | 00111 | 00099 | 38 | 7 | 0 | 0 | 1 | 9 | 13 | 6 | 0 | 0 | 0 | 0 | 0 | 0 | 0 | 0 | 1 | 0 | 0 | 0 | 0 | 0 | 0 | 0 | 1 | 0 | 0 | K01396 | 92 | 373 | 1 | 983 | 1352 |
| 101 | 00113 | 00100 | 1 | 1 | 0 | 0 | 0 | 0 | 0 | 0 | 0 | 0 | 0 | 0 | 0 | 0 | 0 | 0 | 0 | 0 | 0 | 0 | 0 | 0 | 0 | 0 | 0 | 0 | 0 | | | | | | |
| 102 | 00114 | 00101 | 38 | 1 | 1 | 5 | 0 | 1 | 0 | 0 | 3 | 0 | 1 | 3 | 1 | 1 | 0 | 4 | 3 | 1 | 0 | 9 | 0 | 1 | 1 | 0 | 0 | 2 | 0 | X04098 | 92.2 | 383 | 1 | 1551 | 1918 |
| 103 | 00115 | 00102 | 2 | 2 | 0 | 0 | 0 | 0 | 0 | 0 | 0 | 0 | 0 | 0 | 0 | 0 | 0 | 0 | 0 | 0 | 0 | 0 | 0 | 0 | 0 | 0 | 0 | 0 | 0 | | | | | | |
| 104 | 00116 | 00103 | 17 | 2 | 1 | 1 | 1 | 0 | 0 | 0 | 0 | 0 | 0 | 0 | 0 | 4 | 0 | 0 | 0 | 1 | 0 | 0 | 0 | 0 | 0 | 0 | 0 | 6 | 1 | M14630 | 95.6 | 362 | 1 | 840 | 1200 |
| 105 | 00117 | 00104 | 3 | 3 | 0 | 0 | 0 | 0 | 0 | 0 | 0 | 0 | 0 | 0 | 0 | 0 | 0 | 0 | 0 | 0 | 0 | 0 | 0 | 0 | 0 | 0 | 0 | 0 | 0 | | | | | | |
| 106 | 00118 | 00105 | 7 | 4 | 0 | 0 | 0 | 0 | 0 | 0 | 0 | 0 | 1 | 0 | 0 | 0 | 0 | 0 | 1 | 1 | 0 | 0 | 0 | 0 | 0 | 0 | 0 | 0 | 0 | | | | | | |
| 107 | 00119 | 00106 | 2 | 2 | 0 | 0 | 0 | 0 | 0 | 0 | 0 | 0 | 0 | 0 | 0 | 0 | 0 | 0 | 0 | 0 | 0 | 0 | 0 | 0 | 0 | 0 | 0 | 0 | 0 | | | | | | |
| 108 | 00120 | 00107 | 5 | 2 | 0 | 1 | 0 | 0 | 0 | 0 | 0 | 0 | 0 | 0 | 0 | 0 | 0 | 0 | 0 | 0 | 0 | 0 | 0 | 0 | 0 | 0 | 1 | 1 | 0 | | | | | | |
| 109 | 00121 | 00108 | 4 | 1 | 2 | 0 | 0 | 0 | 0 | 0 | 0 | 0 | 0 | 0 | 0 | 0 | 0 | 0 | 0 | 0 | 0 | 0 | 0 | 1 | 0 | 0 | 0 | 0 | 0 | | | | | | |
| 110 | 00122 | 00109 | 4 | 1 | 0 | 0 | 0 | 0 | 0 | 0 | 0 | 0 | 0 | 0 | 2 | 0 | 0 | 0 | 0 | 0 | 0 | 0 | 0 | 0 | 0 | 0 | 0 | 0 | 1 | | | | | | |
| 111 | 00123 | 00110 | 14 | 2 | 0 | 0 | 2 | 0 | 0 | 0 | 0 | 0 | 0 | 0 | 1 | 1 | 1 | 0 | 1 | 2 | 0 | 2 | 0 | 1 | 0 | 0 | 0 | 1 | 0 | X55954 | 98.8 | 336 | 1 | 144 | 479 |
| 112 | 00124 | 00111 | 6 | 2 | 1 | 0 | 0 | 0 | 0 | 0 | 0 | 0 | 0 | 0 | 0 | 0 | 0 | 0 | 0 | 0 | 0 | 0 | 0 | 0 | 1 | 1 | 1 | 0 | 0 | | | | | | |
| 113 | 00125 | 00112 | 1 | 1 | 0 | 0 | 0 | 0 | 0 | 0 | 0 | 0 | 0 | 0 | 0 | 0 | 0 | 0 | 0 | 0 | 0 | 0 | 0 | 0 | 0 | 0 | 0 | 0 | 0 | | | | | | |
| 114 | 00127 | 00113 | 1 | 1 | 0 | 0 | 0 | 0 | 0 | 0 | 0 | 0 | 0 | 0 | 0 | 0 | 0 | 0 | 0 | 0 | 0 | 0 | 0 | 0 | 0 | 0 | 0 | 0 | 0 | | | | | | |
| 115 | 00128 | 00114 | 6 | 2 | 0 | 0 | 0 | 0 | 0 | 0 | 1 | 0 | 0 | 1 | 0 | 0 | 0 | 0 | 1 | 0 | 0 | 0 | 0 | 0 | 1 | 0 | 0 | 0 | 0 | X54326 | 98 | 348 | 1 | 4238 | 4586 |
| 116 | 00129 | 00115 | 11 | 1 | 0 | 0 | 1 | 0 | 0 | 0 | 1 | 0 | 2 | 0 | 0 | 0 | 0 | 0 | 1 | 0 | 0 | 0 | 0 | 0 | 1 | 2 | 1 | 1 | 0 | X65923 | 96.6 | 348 | 1 | 168 | 518 |
| 117 | 00130 | 00116 | 1 | 1 | 0 | 0 | 0 | 0 | 0 | 0 | 0 | 0 | 0 | 0 | 0 | 0 | 0 | 0 | 0 | 0 | 0 | 0 | 0 | 0 | 0 | 0 | 0 | 0 | 0 | | | | | | |
| 118 | 00131 | 00117 | 6 | 1 | 0 | 0 | 0 | 0 | 0 | 0 | 2 | 0 | 0 | 0 | 0 | 0 | 0 | 0 | 0 | 0 | 1 | 0 | 0 | 0 | 0 | 1 | 0 | 0 | 1 | | | | | | |
| 119 | 00132 | 00118 | 1 | 1 | 0 | 0 | 0 | 0 | 0 | 0 | 0 | 0 | 0 | 0 | 0 | 0 | 0 | 0 | 0 | 0 | 0 | 0 | 0 | 0 | 0 | 0 | 0 | 0 | 0 | | | | | | |
| 120 | 00133 | 00119 | 2 | 1 | 0 | 0 | 0 | 0 | 0 | 0 | 0 | 0 | 0 | 0 | 0 | 0 | 0 | 0 | 0 | 1 | 0 | 0 | 0 | 0 | 0 | 0 | 0 | 0 | 0 | | | | | | |
| 121 | 00134 | 00120 | 24 | 1 | 0 | 0 | 1 | 0 | 1 | 1 | 1 | 0 | 1 | 2 | 0 | 4 | 1 | 1 | 0 | 0 | 3 | 1 | 1 | 1 | 3 | 0 | 0 | 1 | 0 | M16660 | 99.1 | 339 | 1 | 2205 | 2543 |
| 122 | 00135 | 00121 | 81 | 5 | 4 | 0 | 14 | 2 | 4 | 2 | 0 | 2 | 0 | 4 | 1 | 1 | 0 | 3 | 5 | 14 | 1 | 12 | 1 | 2 | 4 | 0 | 0 | 0 | 0 | M10119 | 99.7 | 307 | 1 | 416 | 723 |
| 123 | 00136 | 00122 | 2 | 1 | 0 | 0 | 0 | 0 | 0 | 0 | 0 | 0 | 0 | 0 | 0 | 0 | 0 | 0 | 0 | 0 | 0 | 0 | 0 | 0 | 0 | 1 | 0 | 0 | 0 | | | | | | |
| 124 | 00137 | 00123 | 10 | 2 | 1 | 0 | 0 | 0 | 0 | 1 | 0 | 1 | 1 | 0 | 0 | 1 | 0 | 0 | 0 | 1 | 0 | 0 | 0 | 0 | 0 | 0 | 0 | 2 | 0 | D00763 | 98.4 | 313 | 1 | 767 | 1078 |
| 125 | 00138 | 00124 | 1 | 1 | 0 | 0 | 0 | 0 | 0 | 0 | 0 | 0 | 0 | 0 | 0 | 0 | 0 | 0 | 0 | 0 | 0 | 0 | 0 | 0 | 0 | 0 | 0 | 0 | 0 | | | | | | |
| 126 | 00139 | 00125 | 8 | 1 | 1 | 0 | 1 | 0 | 0 | 0 | 0 | 0 | 1 | 2 | 2 | 0 | 0 | 0 | 0 | 0 | 0 | 0 | 0 | 0 | 0 | 0 | 0 | 0 | 0 | X03445 | 93.4 | 335 | 1 | 1698 | 2029 |
| 127 | 00140 | 00126 | 13 | 1 | 0 | 1 | 2 | 0 | 0 | 0 | 0 | 0 | 0 | 0 | 0 | 1 | 0 | 1 | 0 | 0 | 1 | 1 | 2 | 0 | 0 | 3 | 0 | 0 | 0 | | | | | | |
| 128 | 00141 | 00127 | 1 | 1 | 0 | 0 | 0 | 0 | 0 | 0 | 0 | 0 | 0 | 0 | 0 | 0 | 0 | 0 | 0 | 0 | 0 | 0 | 0 | 0 | 0 | 0 | 0 | 0 | 0 | | | | | | |
| 129 | 00142 | 00128 | 9 | 1 | 0 | 0 | 0 | 0 | 0 | 0 | 0 | 0 | 0 | 1 | 1 | 2 | 1 | 0 | 0 | 0 | 0 | 0 | 0 | 0 | 0 | 0 | 0 | 3 | 0 | | | | | | |

Table 4

| | A | B | C | E | G | I | K | M | O | Q | S | U | W | Y | AA | AC | AE | AG | AI | AK | AM | AO | AQ | AS | AU | AW | AY | BA | BC | BE | BF | BG | BH | BI | BK |
|---|---|---|---|---|---|---|---|---|---|---|---|---|---|---|---|---|---|---|---|---|---|---|---|---|---|---|---|---|---|---|---|---|---|---|---|
| 130 | 00143 | 00129 | 5 | 2 | 0 | 0 | 0 | 0 | 0 | 1 | 0 | 0 | 0 | 0 | 0 | 0 | 0 | 0 | 0 | 2 | 0 | 0 | 0 | 0 | 0 | 0 | 0 | 0 | 0 | | | | | | |
| 131 | 00144 | 00130 | 1 | 1 | 0 | 0 | 0 | 0 | 0 | 0 | 0 | 0 | 0 | 0 | 0 | 0 | 0 | 0 | 0 | 0 | 0 | 0 | 0 | 0 | 0 | 0 | 0 | 0 | 0 | | | | | | |
| 132 | 00145 | 00131 | 2 | 1 | 0 | 0 | 0 | 0 | 0 | 0 | 0 | 0 | 0 | 0 | 1 | 0 | 0 | 0 | 0 | 0 | 0 | 0 | 0 | 0 | 0 | 0 | 0 | 0 | 0 | | | | | | |
| 133 | 00146 | 00132 | 8 | 1 | 2 | 2 | 0 | 0 | 0 | 0 | 1 | 0 | 0 | 0 | 1 | 0 | 0 | 0 | 0 | 0 | 1 | 0 | 0 | 0 | 0 | 0 | 0 | 0 | 0 | | | | | | |
| 134 | 00147 | 00133 | 1 | 1 | 0 | 0 | 0 | 0 | 0 | 0 | 0 | 0 | 0 | 0 | 0 | 0 | 0 | 0 | 0 | 0 | 0 | 0 | 0 | 0 | 0 | 0 | 0 | 0 | 0 | J05682 | 98.8 | 329 | 1 | 1245 | 1618 |
| 135 | 00148 | 00134 | 1 | 1 | 0 | 0 | 0 | 0 | 0 | 0 | 0 | 0 | 0 | 0 | 0 | 0 | 0 | 0 | 0 | 0 | 0 | 0 | 0 | 0 | 0 | 0 | 0 | 0 | 0 | | | | | | |
| 136 | 00149 | 00135 | 1 | 1 | 0 | 0 | 0 | 0 | 0 | 0 | 0 | 0 | 0 | 0 | 0 | 0 | 0 | 0 | 0 | 0 | 0 | 0 | 0 | 0 | 0 | 0 | 0 | 0 | 0 | | | | | | |
| 137 | 00150 | 00136 | 17 | 2 | 0 | 0 | 0 | 0 | 0 | 0 | 1 | 0 | 0 | 0 | 0 | 1 | 0 | 4 | 0 | 0 | 1 | 0 | 3 | 1 | 4 | 0 | 0 | 0 | 0 | X05607 | 98.4 | 320 | 1 | 452 | 771 |
| 138 | 00151 | 00137 | 1 | 1 | 0 | 0 | 0 | 0 | 0 | 0 | 0 | 0 | 0 | 0 | 0 | 0 | 0 | 0 | 0 | 0 | 0 | 0 | 0 | 0 | 0 | 0 | 0 | 0 | 0 | | | | | | |
| 139 | 00152 | 00138 | 1 | 1 | 0 | 0 | 0 | 0 | 0 | 0 | 0 | 0 | 0 | 0 | 0 | 0 | 0 | 0 | 0 | 0 | 0 | 0 | 0 | 0 | 0 | 0 | 0 | 0 | 0 | | | | | | |
| 140 | 00153 | 00139 | 1 | 1 | 0 | 0 | 0 | 0 | 0 | 0 | 0 | 0 | 0 | 0 | 0 | 0 | 0 | 0 | 0 | 0 | 0 | 0 | 0 | 0 | 0 | 0 | 0 | 0 | 0 | | | | | | |
| 141 | 00154 | 00140 | 5 | 1 | 0 | 0 | 0 | 0 | 0 | 0 | 0 | 0 | 1 | 0 | 0 | 0 | 0 | 0 | 1 | 0 | 0 | 0 | 0 | 0 | 0 | 1 | 1 | 0 | 0 | | | | | | |
| 142 | 00155 | 00141 | 81 | 1 | 0 | 3 | 8 | 0 | 3 | 0 | 2 | 2 | 3 | 4 | 5 | 1 | 0 | 5 | 15 | 4 | 1 | 2 | 0 | 0 | 0 | 4 | 7 | 11 | 0 | M11948 | 93.8 | 321 | 1 | 110 | 425 |
| 143 | 00156 | 00142 | 20 | 2 | 1 | 0 | 1 | 0 | 0 | 0 | 0 | 0 | 0 | 0 | 1 | 1 | 1 | 0 | 1 | 0 | 0 | 3 | 0 | 0 | 0 | 1 | 0 | 8 | 0 | M23613 | 98.6 | 348 | 1 | 949 | 1296 |
| 144 | 00157 | 00143 | 11 | 1 | 0 | 0 | 0 | 8 | 2 | 0 | 0 | 0 | 0 | 0 | 0 | 0 | 0 | 0 | 0 | 0 | 0 | 0 | 0 | 0 | 0 | 0 | 0 | 0 | 0 | M64982 | 96.9 | 323 | 1 | 5109 | 5943 |
| 145 | 00158 | 00144 | 2 | 2 | 0 | 0 | 0 | 0 | 0 | 0 | 0 | 0 | 0 | 0 | 0 | 0 | 0 | 0 | 0 | 0 | 0 | 0 | 0 | 0 | 0 | 0 | 0 | 0 | 0 | | | | | | |
| 146 | 00159 | 00145 | 4 | 1 | 0 | 1 | 1 | 0 | 0 | 0 | 0 | 0 | 1 | 0 | 0 | 0 | 0 | 0 | 0 | 0 | 0 | 0 | 0 | 0 | 0 | 0 | 0 | 0 | 0 | M30496 | 98.6 | 283 | 1 | 519 | 802 |
| 147 | 00160 | 00146 | 9 | 1 | 1 | 0 | 0 | 0 | 0 | 0 | 1 | 0 | 0 | 0 | 1 | 0 | 1 | 0 | 0 | 0 | 1 | 1 | 0 | 0 | 1 | 0 | 0 | 1 | 0 | | | | | | |
| 148 | 00161 | 00147 | 1 | 1 | 0 | 0 | 0 | 0 | 0 | 0 | 0 | 0 | 0 | 0 | 0 | 0 | 0 | 0 | 0 | 0 | 0 | 0 | 0 | 0 | 0 | 0 | 0 | 0 | 0 | | | | | | |
| 149 | 00162 | 00148 | 47 | 1 | 4 | 3 | 2 | 1 | 0 | 1 | 2 | 5 | 2 | 4 | 4 | 2 | 0 | 0 | 2 | 2 | 1 | 7 | 1 | 0 | 2 | 1 | 0 | 0 | 0 | M17887 | 98.7 | 312 | 1 | 149 | 460 |
| 150 | 00163 | 00149 | 30 | 1 | 0 | 0 | 1 | 0 | 1 | 0 | 1 | 0 | 0 | 1 | 2 | 0 | 1 | 0 | 1 | 4 | 0 | 4 | 2 | 1 | 2 | 7 | 0 | 1 | 0 | M17886 | 95.7 | 305 | 1 | 210 | 512 |
| 151 | 00164 | 00150 | 3 | 2 | 0 | 0 | 0 | 0 | 0 | 0 | 0 | 0 | 0 | 0 | 0 | 0 | 0 | 0 | 0 | 0 | 0 | 0 | 0 | 0 | 0 | 1 | 0 | 0 | 0 | | | | | | |
| 152 | 00165 | 00151 | 3 | 3 | 0 | 0 | 0 | 0 | 0 | 0 | 0 | 0 | 0 | 0 | 0 | 0 | 0 | 0 | 0 | 0 | 0 | 0 | 0 | 0 | 0 | 0 | 0 | 0 | 0 | | | | | | |
| 153 | 00166 | 00152 | 3 | 1 | 0 | 0 | 1 | 0 | 0 | 0 | 0 | 0 | 0 | 0 | 0 | 0 | 0 | 0 | 0 | 0 | 1 | 0 | 0 | 0 | 0 | 0 | 0 | 0 | 0 | | | | | | |
| 154 | 00167 | 00153 | 1 | 1 | 0 | 0 | 0 | 0 | 0 | 0 | 0 | 0 | 0 | 0 | 0 | 0 | 0 | 0 | 0 | 0 | 0 | 0 | 0 | 0 | 0 | 0 | 0 | 0 | 0 | | | | | | |
| 155 | 00168 | 00154 | 6 | 1 | 0 | 0 | 1 | 0 | 0 | 0 | 0 | 0 | 1 | 0 | 0 | 0 | 0 | 0 | 2 | 0 | 0 | 0 | 0 | 0 | 0 | 0 | 0 | 1 | 0 | | | | | | |
| 156 | 00169 | 00155 | 1 | 1 | 0 | 0 | 0 | 0 | 0 | 0 | 0 | 0 | 0 | 0 | 0 | 0 | 0 | 0 | 0 | 0 | 0 | 0 | 0 | 0 | 0 | 0 | 0 | 0 | 0 | | | | | | |
| 157 | 00170 | 00156 | 2 | 2 | 0 | 0 | 0 | 0 | 0 | 0 | 0 | 0 | 0 | 0 | 0 | 0 | 0 | 0 | 0 | 0 | 0 | 0 | 0 | 0 | 0 | 0 | 0 | 0 | 0 | | | | | | |
| 158 | 00171 | 00157 | 2 | 1 | 0 | 0 | 0 | 0 | 0 | 0 | 0 | 0 | 0 | 0 | 0 | 0 | 0 | 0 | 0 | 1 | 0 | 0 | 0 | 0 | 0 | 0 | 0 | 0 | 0 | | | | | | |
| 159 | 00172 | 00158 | 14 | 1 | 0 | 0 | 0 | 1 | 1 | 0 | 0 | 0 | 0 | 0 | 1 | 5 | 3 | 0 | 1 | 0 | 0 | 0 | 0 | 1 | 0 | 0 | 0 | 0 | 0 | M12623 | 98 | 299 | 1 | 879 | 1187 |
| 160 | 00173 | 00159 | 3 | 1 | 0 | 0 | 0 | 0 | 0 | 0 | 0 | 0 | 0 | 0 | 1 | 0 | 0 | 0 | 0 | 0 | 0 | 1 | 0 | 0 | 0 | 0 | 0 | 0 | 0 | M26658 | 98.7 | 305 | 1 | 31 | 2490 |
| 161 | 00174 | 00160 | 3 | 1 | 0 | 0 | 0 | 0 | 0 | 0 | 0 | 0 | 0 | 0 | 0 | 0 | 0 | 0 | 0 | 0 | 0 | 0 | 1 | 0 | 0 | 0 | 0 | 1 | 0 | | | | | | |
| 162 | 00175 | 00161 | 3 | 1 | 0 | 0 | 0 | 0 | 0 | 0 | 0 | 0 | 0 | 1 | 0 | 0 | 0 | 0 | 0 | 0 | 0 | 0 | 0 | 0 | 0 | 1 | 0 | 0 | 0 | | | | | | |
| 163 | 00176 | 00162 | 7 | 1 | 0 | 0 | 0 | 0 | 0 | 0 | 0 | 0 | 0 | 0 | 0 | 0 | 0 | 0 | 0 | 3 | 0 | 0 | 1 | 0 | 0 | 2 | 0 | 0 | 0 | K03000 | 99.7 | 291 | 1 | 1269 | 1560 |
| 164 | 00177 | 00163 | 18 | 2 | 2 | 0 | 1 | 0 | 2 | 1 | 0 | 1 | 0 | 0 | 0 | 0 | 1 | 0 | 0 | 1 | 0 | 1 | 0 | 1 | 0 | 5 | 0 | 0 | 0 | L19739 | 98 | 293 | 1 | 36 | 329 |
| 165 | 00178 | 00164 | 1 | 1 | 0 | 0 | 0 | 0 | 0 | 0 | 0 | 0 | 0 | 0 | 0 | 0 | 0 | 0 | 0 | 0 | 0 | 0 | 0 | 0 | 0 | 0 | 0 | 0 | 0 | X06323 | 100 | 295 | 1 | 1001 | 1634 |

Table 5

| | A | B | C | E | G | I | K | M | O | Q | S | U | W | Y | AA | AC | AE | AG | AI | AK | AM | AO | AQ | AS | AU | AW | AY | BA | BC | BE | BF | BG | BH | BI | BK |
|---|---|---|---|---|---|---|---|---|---|---|---|---|---|---|---|---|---|---|---|---|---|---|---|---|---|---|---|---|---|---|---|---|---|---|---|
| 166 | 00179 | 00165 | 2 | 1 | 0 | 0 | 0 | 0 | 0 | 0 | 0 | 0 | 0 | 0 | 0 | 0 | 1 | 0 | 0 | 0 | 0 | 0 | 0 | 0 | 0 | 0 | 0 | 0 | 0 | | | | | | |
| 167 | 00180 | 00166 | 7 | 2 | 0 | 0 | 1 | 0 | 0 | 1 | 0 | 0 | 0 | 0 | 1 | 0 | 0 | 0 | 1 | 0 | 0 | 0 | 0 | 0 | 0 | 1 | 0 | 0 | 0 | | | | | | |
| 168 | 00181 | 00167 | 2 | 1 | 0 | 0 | 0 | 0 | 0 | 0 | 0 | 0 | 0 | 0 | 1 | 0 | 0 | 0 | 0 | 0 | 0 | 0 | 0 | 0 | 0 | 0 | 0 | 0 | 0 | | | | | | |
| 169 | 00182 | 00168 | 7 | 1 | 0 | 0 | 0 | 0 | 0 | 0 | 1 | 0 | 1 | 0 | 0 | 0 | 0 | 0 | 0 | 0 | 1 | 1 | 0 | 0 | 2 | 0 | 0 | 0 | 0 | M34671 | 99 | 97 | 1 | 1575 | 1671 |
| 170 | 00183 | 00169 | 2 | 1 | 0 | 0 | 0 | 0 | 0 | 0 | 0 | 0 | 0 | 0 | 0 | 0 | 0 | 0 | 0 | 0 | 0 | 0 | 0 | 0 | 0 | 0 | 0 | 1 | 0 | | | | | | |
| 171 | 00184 | 00170 | 4 | 1 | 1 | 0 | 0 | 0 | 0 | 0 | 1 | 0 | 0 | 0 | 0 | 0 | 0 | 0 | 0 | 1 | 0 | 0 | 0 | 0 | 0 | 0 | 0 | 0 | 0 | | | | | | |
| 172 | 00185 | 00171 | 13 | 2 | 1 | 2 | 1 | 0 | 0 | 0 | 0 | 0 | 0 | 0 | 1 | 0 | 0 | 0 | 0 | 1 | 2 | 1 | 0 | 0 | 0 | 0 | 1 | 1 | 0 | | | | | | |
| 173 | 00186 | 00172 | 1 | 1 | 0 | 0 | 0 | 0 | 0 | 0 | 0 | 0 | 0 | 0 | 0 | 0 | 0 | 0 | 0 | 0 | 0 | 0 | 0 | 0 | 0 | 0 | 0 | 0 | 0 | | | | | | |
| 174 | 00187 | 00173 | 1 | 1 | 0 | 0 | 0 | 0 | 0 | 0 | 0 | 0 | 0 | 0 | 0 | 0 | 0 | 0 | 0 | 0 | 0 | 0 | 0 | 0 | 0 | 0 | 0 | 0 | 0 | | | | | | |
| 175 | 00188 | 00174 | 11 | 1 | 0 | 1 | 0 | 0 | 0 | 0 | 0 | 0 | 0 | 2 | 0 | 0 | 0 | 0 | 0 | 2 | 1 | 0 | 1 | 1 | 0 | 0 | 0 | 2 | 0 | | | | | | |
| 176 | 00189 | 00175 | 1 | 1 | 0 | 0 | 0 | 0 | 0 | 0 | 0 | 0 | 0 | 0 | 0 | 0 | 0 | 0 | 0 | 0 | 0 | 0 | 0 | 0 | 0 | 0 | 0 | 0 | 0 | | | | | | |
| 177 | 00190 | 00176 | 1 | 1 | 0 | 0 | 0 | 0 | 0 | 0 | 0 | 0 | 0 | 0 | 0 | 0 | 0 | 0 | 0 | 0 | 0 | 0 | 0 | 0 | 0 | 0 | 0 | 0 | 0 | J02645 | 98.9 | 281 | 1 | 1114 | 1393 |
| 178 | 00191 | 00177 | 3 | 1 | 0 | 1 | 0 | 0 | 0 | 0 | 0 | 0 | 0 | 0 | 0 | 0 | 0 | 0 | 0 | 0 | 0 | 0 | 0 | 0 | 0 | 1 | 0 | 0 | 0 | | | | | | |
| 179 | 00192 | 00178 | 2 | 1 | 1 | 0 | 0 | 0 | 0 | 0 | 0 | 0 | 0 | 0 | 0 | 0 | 0 | 0 | 0 | 0 | 0 | 0 | 0 | 0 | 0 | 0 | 0 | 0 | 0 | | | | | | |
| 180 | 00193 | 00179 | 1 | 1 | 0 | 0 | 0 | 0 | 0 | 0 | 0 | 0 | 0 | 0 | 0 | 0 | 0 | 0 | 0 | 0 | 0 | 0 | 0 | 0 | 0 | 0 | 0 | 0 | 0 | | | | | | |
| 181 | 00194 | 00180 | 34 | 3 | 0 | 0 | 0 | 21 | 8 | 2 | 0 | 0 | 0 | 0 | 0 | 0 | 0 | 0 | 0 | 0 | 0 | 0 | 0 | 0 | 0 | 0 | 0 | 0 | 0 | M16961 | 98.9 | 275 | 1 | 1265 | 1538 |
| 182 | 00195 | 00181 | 4 | 2 | 0 | 0 | 0 | 0 | 0 | 0 | 0 | 0 | 0 | 0 | 0 | 0 | 1 | 0 | 0 | 0 | 0 | 0 | 0 | 0 | 0 | 0 | 1 | 0 | 0 | | | | | | |
| 183 | 00196 | 00182 | 67 | 1 | 0 | 0 | 0 | 6 | 11 | 9 | 0 | 0 | 0 | 0 | 40 | 0 | 0 | 0 | 0 | 0 | 0 | 0 | 0 | 0 | 0 | 0 | 0 | 0 | 0 | M10617 | 94.9 | 274 | 4 | 193 | 462 |
| 184 | 00197 | 00183 | 1 | 1 | 0 | 0 | 0 | 0 | 0 | 0 | 0 | 0 | 0 | 0 | 0 | 0 | 0 | 0 | 0 | 0 | 0 | 0 | 0 | 0 | 0 | 0 | 0 | 0 | 0 | X12646 | 93.8 | 273 | 1 | 687 | 2036 |
| 185 | 00198 | 00184 | 1 | 1 | 0 | 0 | 0 | 0 | 0 | 0 | 0 | 0 | 0 | 0 | 0 | 0 | 0 | 0 | 0 | 0 | 0 | 0 | 0 | 0 | 0 | 0 | 0 | 0 | 0 | | | | | | |
| 186 | 00199 | 00185 | 2 | 2 | 0 | 0 | 0 | 0 | 0 | 0 | 0 | 0 | 0 | 0 | 0 | 0 | 0 | 0 | 0 | 0 | 0 | 0 | 0 | 0 | 0 | 0 | 0 | 0 | 0 | | | | | | |
| 187 | 00200 | 00186 | 4 | 1 | 0 | 0 | 0 | 0 | 0 | 0 | 0 | 0 | 1 | 0 | 1 | 0 | 0 | 1 | 0 | 0 | 0 | 0 | 0 | 0 | 0 | 0 | 0 | 0 | 0 | L13385 | 96 | 253 | 1 | 4989 | 5243 |
| 188 | 00201 | 00187 | 1 | 1 | 0 | 0 | 0 | 0 | 0 | 0 | 0 | 0 | 0 | 0 | 0 | 0 | 0 | 0 | 0 | 0 | 0 | 0 | 0 | 0 | 0 | 0 | 0 | 0 | 0 | | | | | | |
| 189 | 00202 | 00188 | 3 | 1 | 0 | 0 | 0 | 0 | 0 | 0 | 1 | 0 | 0 | 0 | 0 | 0 | 1 | 0 | 0 | 0 | 0 | 0 | 0 | 0 | 0 | 0 | 0 | 0 | 0 | | | | | | |
| 190 | 00203 | 00189 | 3 | 1 | 0 | 0 | 0 | 0 | 0 | 1 | 0 | 0 | 0 | 0 | 0 | 0 | 0 | 0 | 0 | 1 | 0 | 0 | 0 | 0 | 0 | 0 | 0 | 0 | 0 | | | | | | |
| 191 | 00204 | 00190 | 1 | 1 | 0 | 0 | 0 | 0 | 0 | 0 | 0 | 0 | 0 | 0 | 0 | 0 | 0 | 0 | 0 | 0 | 0 | 0 | 0 | 0 | 0 | 0 | 0 | 0 | 0 | | | | | | |
| 192 | 00205 | 00191 | 5 | 2 | 0 | 1 | 1 | 0 | 0 | 0 | 0 | 0 | 0 | 1 | 0 | 0 | 0 | 0 | 0 | 0 | 0 | 0 | 0 | 0 | 0 | 0 | 0 | 0 | 0 | | | | | | |
| 193 | 00206 | 00192 | 7 | 2 | 0 | 0 | 0 | 0 | 1 | 0 | 0 | 1 | 0 | 0 | 0 | 0 | 0 | 0 | 1 | 0 | 0 | 0 | 0 | 1 | 0 | 0 | 0 | 1 | 0 | | | | | | |
| 194 | 00207 | 00193 | 5 | 1 | 0 | 0 | 0 | 0 | 0 | 0 | 0 | 0 | 2 | 0 | 0 | 0 | 0 | 0 | 0 | 0 | 1 | 0 | 0 | 0 | 0 | 0 | 0 | 1 | 0 | | | | | | |
| 195 | 00209 | 00194 | 1 | 1 | 0 | 0 | 0 | 0 | 0 | 0 | 0 | 0 | 0 | 0 | 0 | 0 | 0 | 0 | 0 | 0 | 0 | 0 | 0 | 0 | 0 | 0 | 0 | 0 | 0 | | | | | | |
| 196 | 00210 | 00195 | 14 | 1 | 0 | 1 | 0 | 0 | 0 | 0 | 0 | 0 | 3 | 1 | 0 | 0 | 0 | 1 | 1 | 1 | 1 | 0 | 0 | 0 | 0 | 0 | 0 | 4 | 0 | M36341 | 98.4 | 384 | 1 | 1135 | 1529 |
| 197 | 00211 | 00196 | 128 | 8 | 24 | 2 | 10 | 0 | 4 | 2 | 5 | 16 | 9 | 1 | 5 | 0 | 3 | 0 | 12 | 5 | 1 | 0 | 4 | 2 | 0 | 4 | 0 | 7 | 4 | | | | | | |
| 198 | 00212 | 00197 | 16 | 1 | 1 | 0 | 0 | 0 | 1 | 0 | 0 | 0 | 1 | 0 | 1 | 0 | 2 | 1 | 0 | 4 | 0 | 0 | 0 | 1 | 0 | 0 | 0 | 2 | 1 | L10612 | 97.8 | 272 | 1 | 275 | 557 |
| 199 | 00213 | 00198 | 8 | | 0 | 1 | 1 | 0 | 0 | 0 | 0 | 0 | 1 | 0 | 0 | 0 | 0 | 0 | 0 | 1 | 0 | 0 | 0 | 1 | 0 | 2 | 1 | 0 | 0 | | | | | | |
| 200 | 00215 | 00199 | 2 | 1 | 0 | 0 | 0 | 0 | 0 | 0 | 0 | 0 | 0 | 1 | 0 | 0 | 0 | 0 | 0 | 0 | 0 | 0 | 0 | 0 | 0 | 0 | 0 | 0 | 0 | | | | | | |
| 201 | 00216 | 00200 | 2 | 1 | 0 | 0 | 0 | 0 | 0 | 0 | 0 | 0 | 0 | 0 | 0 | 0 | 0 | 0 | 0 | 0 | 0 | 0 | 0 | 1 | 0 | 0 | 0 | 0 | 0 | | | | | | |

Table 6

| | A | B | C | E | G | I | K | M | O | Q | S | U | W | Y | AA | AC | AE | AG | AI | AK | AM | AO | AQ | AS | AU | AW | AY | BA | BC | BE | BF | BG | BH | BI | BK |
|---|---|---|---|---|---|---|---|---|---|---|---|---|---|---|---|---|---|---|---|---|---|---|---|---|---|---|---|---|---|---|---|---|---|---|---|
| 202 | 00217 | 00201 | 5 | 1 | 0 | 0 | 0 | 0 | 0 | 1 | 1 | 0 | 0 | 0 | 0 | 0 | 2 | 0 | 0 | 0 | 0 | 0 | 0 | 0 | 0 | 0 | 0 | 0 | 0 | | | | | | |
| 203 | 00218 | 00202 | 1 | 1 | 0 | 0 | 0 | 0 | 0 | 0 | 0 | 0 | 0 | 0 | 0 | 0 | 0 | 0 | 0 | 0 | 0 | 0 | 0 | 0 | 0 | 0 | 0 | 0 | 0 | | | | | | |
| 204 | 00219 | 00203 | 13 | 2 | 1 | 0 | 0 | 0 | 0 | 0 | 0 | 1 | 0 | 0 | 2 | 0 | 0 | 0 | 0 | 0 | 0 | 1 | 0 | 1 | 0 | 1 | 0 | 2 | 2 | D13388 | 99.5 | 220 | 1 | 1216 | 1435 |
| 205 | 00220 | 00204 | 1 | 1 | 0 | 0 | 0 | 0 | 0 | 0 | 0 | 0 | 0 | 0 | 0 | 0 | 0 | 0 | 0 | 0 | 0 | 0 | 0 | 0 | 0 | 0 | 0 | 0 | 0 | | | | | | |
| 206 | 00221 | 00205 | 4 | 1 | 0 | 0 | 1 | 0 | 0 | 0 | 0 | 0 | 0 | 0 | 0 | 0 | 1 | 0 | 0 | 0 | 0 | 0 | 1 | 0 | 0 | 0 | 0 | 0 | 0 | | | | | | |
| 207 | 00222 | 00206 | 1 | 1 | 0 | 0 | 0 | 0 | 0 | 0 | 0 | 0 | 0 | 0 | 0 | 0 | 0 | 0 | 0 | 0 | 0 | 0 | 0 | 0 | 0 | 0 | 0 | 0 | 0 | | | | | | |
| 208 | 00223 | 00207 | 1 | 1 | 0 | 0 | 0 | 0 | 0 | 0 | 0 | 0 | 0 | 0 | 0 | 0 | 0 | 0 | 0 | 0 | 0 | 0 | 0 | 0 | 0 | 0 | 0 | 0 | 0 | | | | | | |
| 209 | 00224 | 00208 | 8 | 1 | 1 | 0 | 0 | 0 | 0 | 1 | 0 | 2 | 0 | 0 | 0 | 0 | 2 | 0 | 0 | 0 | 0 | 0 | 0 | 0 | 0 | 0 | 0 | 1 | 0 | | | | | | |
| 210 | 00225 | 00209 | 1 | 1 | 0 | 0 | 0 | 0 | 0 | 0 | 0 | 0 | 0 | 0 | 0 | 0 | 0 | 0 | 0 | 0 | 0 | 0 | 0 | 0 | 0 | 0 | 0 | 0 | 0 | | | | | | |
| 211 | 00226 | 00210 | 1 | 1 | 0 | 0 | 0 | 0 | 0 | 0 | 0 | 0 | 0 | 0 | 0 | 0 | 0 | 0 | 0 | 0 | 0 | 0 | 0 | 0 | 0 | 0 | 0 | 0 | 0 | | | | | | |
| 212 | 00227 | 00211 | 1 | 1 | 0 | 0 | 0 | 0 | 0 | 0 | 0 | 0 | 0 | 0 | 0 | 0 | 0 | 0 | 0 | 0 | 0 | 0 | 0 | 0 | 0 | 0 | 0 | 0 | 0 | | | | | | |
| 213 | 00228 | 00212 | 9 | 2 | 0 | 2 | 0 | 0 | 0 | 0 | 0 | 0 | 1 | 0 | 0 | 0 | 0 | 0 | 1 | 0 | 2 | 0 | 0 | 0 | 0 | 0 | 1 | 0 | 0 | | | | | | |
| 214 | 00230 | 00213 | 1 | 1 | 0 | 0 | 0 | 0 | 0 | 0 | 0 | 0 | 0 | 0 | 0 | 0 | 0 | 0 | 0 | 0 | 0 | 0 | 0 | 0 | 0 | 0 | 0 | 0 | 0 | X17206 | 98.5 | 202 | 31 | 733 | 934 |
| 215 | 00231 | 00214 | 6 | 1 | 0 | 0 | 0 | 0 | 0 | 0 | 0 | 0 | 0 | 0 | 1 | 0 | 0 | 0 | 0 | 1 | 0 | 0 | 0 | 1 | 1 | 1 | 0 | 0 | 0 | | | | | | |
| 216 | 00232 | 00215 | 3 | 1 | 0 | 0 | 1 | 1 | 0 | 0 | 0 | 0 | 0 | 0 | 0 | 0 | 0 | 0 | 0 | 0 | 0 | 0 | 0 | 0 | 0 | 0 | 0 | 0 | 0 | | | | | | |
| 217 | 00233 | 00216 | 1 | 1 | 0 | 0 | 0 | 0 | 0 | 0 | 0 | 0 | 0 | 0 | 0 | 0 | 0 | 0 | 0 | 0 | 0 | 0 | 0 | 0 | 0 | 0 | 0 | 0 | 0 | | | | | | |
| 218 | 00234 | 00217 | 1 | 1 | 0 | 0 | 0 | 0 | 0 | 0 | 0 | 0 | 0 | 0 | 0 | 0 | 0 | 0 | 0 | 0 | 0 | 0 | 0 | 0 | 0 | 0 | 0 | 0 | 0 | | | | | | |
| 219 | 00235 | 00218 | 2 | 1 | 0 | 0 | 0 | 0 | 0 | 0 | 0 | 0 | 0 | 0 | 0 | 0 | 0 | 0 | 0 | 0 | 1 | 0 | 0 | 0 | 0 | 0 | 0 | 0 | 0 | | | | | | |
| 220 | 00236 | 00219 | 11 | 1 | 1 | 0 | 0 | 0 | 1 | 1 | 1 | 1 | 1 | 0 | 1 | 0 | 0 | 0 | 0 | 2 | 0 | 0 | 0 | 0 | 0 | 0 | 0 | 0 | 1 | | | | | | |
| 221 | 00237 | 00220 | 11 | 2 | 0 | 0 | 0 | 0 | 1 | 0 | 0 | 1 | 1 | 1 | 0 | 0 | 0 | 1 | 0 | 0 | 0 | 0 | 0 | 0 | 4 | 0 | 0 | 0 | 0 | | | | | | |
| 222 | 00238 | 00221 | 2 | 1 | 0 | 0 | 0 | 1 | 0 | 0 | 0 | 0 | 0 | 0 | 0 | 0 | 0 | 0 | 0 | 0 | 0 | 0 | 0 | 0 | 0 | 0 | 0 | 0 | 0 | J02888 | 99.6 | 234 | 1 | 739 | 976 |
| 223 | 00239 | 00222 | 7 | 1 | 0 | 1 | 0 | 0 | 0 | 0 | 0 | 0 | 0 | 0 | 0 | 1 | 0 | 0 | 0 | 1 | 0 | 0 | 0 | 0 | 0 | 0 | 3 | 0 | 0 | | | | | | |
| 224 | 00240 | 00223 | 1 | 1 | 0 | 0 | 0 | 0 | 0 | 0 | 0 | 0 | 0 | 0 | 0 | 0 | 0 | 0 | 0 | 0 | 0 | 0 | 0 | 0 | 0 | 0 | 0 | 0 | 0 | | | | | | |
| 225 | 00241 | 00224 | 12 | 1 | 0 | 0 | 2 | 0 | 0 | 0 | 0 | 1 | 0 | 1 | 0 | 0 | 0 | 0 | 0 | 1 | 1 | 0 | 0 | 0 | 0 | 1 | 1 | 3 | 0 | X59357 | 99.1 | 348 | 1 | 258 | 602 |
| 226 | 00242 | 00225 | 5 | 2 | 0 | 0 | 0 | 0 | 0 | 3 | 0 | 0 | 0 | 0 | 0 | 0 | 0 | 0 | 0 | 0 | 0 | 0 | 0 | 0 | 0 | 0 | 0 | 0 | 0 | | | | | | |
| 227 | 00243 | 00226 | 7 | 1 | 0 | 0 | 0 | 1 | 0 | 0 | 1 | 0 | 0 | 0 | 1 | 0 | 0 | 0 | 1 | 1 | 1 | 0 | 0 | 0 | 0 | 0 | 0 | 0 | 0 | M26326 | 100 | 225 | 1 | 1179 | 1412 |
| 228 | 00244 | 00227 | 65 | 2 | 0 | 14 | 1 | 0 | 0 | 0 | 0 | 0 | 1 | 2 | 3 | 0 | 1 | 3 | 7 | 2 | 4 | 11 | 3 | 0 | 1 | 1 | 4 | 4 | 1 | X00351 | 99.5 | 184 | 1 | 1109 | 1761 |
| 229 | 00245 | 00228 | 3 | 1 | 0 | 0 | 0 | 0 | 0 | 0 | 0 | 0 | 0 | 0 | 0 | 0 | 0 | 0 | 1 | 0 | 0 | 0 | 0 | 1 | 0 | 0 | 0 | 0 | 0 | | | | | | |
| 230 | 00246 | 00229 | 26 | 3 | 5 | 0 | 1 | 0 | 1 | 1 | 0 | 3 | 3 | 1 | 0 | 1 | 0 | 0 | 2 | 2 | 1 | 1 | 0 | 0 | 0 | 1 | 0 | 0 | 0 | | | | | | |
| 231 | 00247 | 00230 | 2 | 1 | 0 | 0 | 0 | 0 | 0 | 1 | 0 | 0 | 0 | 0 | 0 | 0 | 0 | 0 | 0 | 0 | 0 | 0 | 0 | 0 | 0 | 0 | 0 | 0 | 0 | | | | | | |
| 232 | 00248 | 00231 | 7 | 1 | 0 | 0 | 0 | 1 | 1 | 1 | 1 | 0 | 0 | 0 | 0 | 1 | 0 | 0 | 0 | 0 | 1 | 0 | 0 | 0 | 0 | 0 | 0 | 0 | 0 | | | | | | |
| 233 | 00249 | 00232 | 9 | 1 | 0 | 5 | 0 | 0 | 0 | 0 | 0 | 1 | 0 | 0 | 0 | 0 | 0 | 0 | 0 | 0 | 1 | 0 | 0 | 0 | 1 | 0 | 0 | 0 | 0 | M14636 | 99.1 | 222 | 1 | 2607 | 2828 |
| 234 | 00250 | 00233 | 2 | 1 | 0 | 0 | 0 | 0 | 0 | 0 | 0 | 0 | 0 | 0 | 0 | 0 | 0 | 0 | 0 | 0 | 1 | 0 | 0 | 0 | 0 | 0 | 0 | 0 | 0 | | | | | | |
| 235 | 00251 | 00234 | 2 | 1 | 0 | 0 | 0 | 0 | 0 | 0 | 0 | 0 | 0 | 0 | 0 | 0 | 0 | 0 | 0 | 0 | 0 | 0 | 0 | 1 | 0 | 0 | 0 | 0 | 0 | | | | | | |
| 236 | 00252 | 00235 | 5 | 1 | 0 | 3 | 0 | 0 | 0 | 0 | 0 | 0 | 0 | 0 | 0 | 0 | 0 | 0 | 0 | 0 | 0 | 0 | 0 | 0 | 1 | 0 | 0 | 0 | 0 | | | | | | |
| 237 | 00253 | 00236 | 16 | 2 | 1 | 2 | 0 | 0 | 1 | 0 | 0 | 0 | 0 | 0 | 2 | 2 | 0 | 0 | 2 | 1 | 0 | 1 | 0 | 0 | 0 | 0 | 1 | 1 | 0 | | | | | | |

Table 7

| | A | B | C | E | G | I | K | M | O | Q | S | U | W | Y | AA | AC | AE | AG | AI | AK | AM | AO | AQ | AS | AU | AW | AY | BA | BC | BE | BF | BG | BH | BI | BK |
|---|---|---|---|---|---|---|---|---|---|---|---|---|---|---|---|---|---|---|---|---|---|---|---|---|---|---|---|---|---|---|---|---|---|---|---|
| 238 | 00254 | 00237 | 38 | 1 | 0 | 0 | 2 | 0 | 0 | 1 | 2 | 1 | 0 | 1 | 0 | 2 | 0 | 1 | 3 | 0 | 0 | 0 | 0 | 0 | 1 | 3 | 20 | 0 | 0 | M24096 | 98.1 | 214 | 1 | 1152 | 1365 |
| 239 | 00255 | 00238 | 1 | 1 | 0 | 0 | 0 | 0 | 0 | 0 | 0 | 0 | 0 | 0 | 0 | 0 | 0 | 0 | 0 | 0 | 0 | 0 | 0 | 0 | 0 | 0 | 0 | 0 | 0 | | | | | | |
| 240 | 00256 | 00239 | 1 | 1 | 0 | 0 | 0 | 0 | 0 | 0 | 0 | 0 | 0 | 0 | 0 | 0 | 0 | 0 | 0 | 0 | 0 | 0 | 0 | 0 | 0 | 0 | 0 | 0 | 0 | | | | | | |
| 241 | 00257 | 00240 | 1 | 1 | 0 | 0 | 0 | 0 | 0 | 0 | 0 | 0 | 0 | 0 | 0 | 0 | 0 | 0 | 0 | 0 | 0 | 0 | 0 | 0 | 0 | 0 | 0 | 0 | 0 | | | | | | |
| 242 | 00258 | 00241 | 6 | 1 | 0 | 0 | 0 | 0 | 0 | 1 | 0 | 0 | 0 | 0 | 1 | 0 | 0 | 0 | 0 | 0 | 1 | 0 | 0 | 0 | 1 | 0 | 1 | 0 | 0 | X04588 | 95.4 | 217 | 3 | 1857 | 2077 |
| 243 | 00259 | 00242 | 2 | 1 | 0 | 0 | 0 | 0 | 0 | 0 | 0 | 0 | 0 | 0 | 0 | 0 | 1 | 0 | 0 | 0 | 0 | 0 | 0 | 0 | 0 | 0 | 0 | 0 | 0 | | | | | | |
| 244 | 00260 | 00243 | 4 | 1 | 0 | 0 | 0 | 0 | 0 | 0 | 0 | 0 | 0 | 1 | 0 | 0 | 0 | 0 | 1 | 0 | 0 | 0 | 0 | 0 | 0 | 0 | 1 | 0 | 0 | | | | | | |
| 245 | 00261 | 00244 | 17 | 2 | 1 | 0 | 1 | 0 | 1 | 0 | 0 | 0 | 0 | 1 | 0 | 1 | 0 | 1 | 1 | 1 | 0 | 0 | 0 | 3 | 0 | 2 | 0 | 2 | 0 | X65460 | 99 | 204 | 1 | 1607 | 1809 |
| 246 | 00262 | 00245 | 50 | 2 | 7 | 2 | 0 | 1 | 1 | 2 | 0 | 3 | 6 | 0 | 1 | 3 | 0 | 0 | 3 | 0 | 1 | 2 | 0 | 0 | 4 | 4 | 0 | 6 | 2 | M17885 | 98.6 | 211 | 1 | 888 | 1097 |
| 247 | 00263 | 00246 | 2 | 1 | 0 | 0 | 0 | 0 | 0 | 1 | 0 | 0 | 0 | 0 | 0 | 0 | 0 | 0 | 0 | 0 | 0 | 0 | 0 | 0 | 0 | 0 | 0 | 0 | 0 | | | | | | |
| 248 | 00264 | 00247 | 1 | 1 | 0 | 0 | 0 | 0 | 0 | 0 | 0 | 0 | 0 | 0 | 0 | 0 | 0 | 0 | 0 | 0 | 0 | 0 | 0 | 0 | 0 | 0 | 0 | 0 | 0 | | | | | | |
| 249 | 00265 | 00248 | 1 | 1 | 0 | 0 | 0 | 0 | 0 | 0 | 0 | 0 | 0 | 0 | 0 | 0 | 0 | 0 | 0 | 0 | 0 | 0 | 0 | 0 | 0 | 0 | 0 | 0 | 0 | | | | | | |
| 250 | 00266 | 00249 | 3 | 1 | 0 | 0 | 0 | 0 | 0 | 0 | 0 | 0 | 0 | 0 | 1 | 0 | 0 | 0 | 0 | 0 | 0 | 1 | 0 | 0 | 0 | 0 | 0 | 0 | 0 | | | | | | |
| 251 | 00267 | 00250 | 6 | 1 | 0 | 0 | 0 | 0 | 0 | 0 | 0 | 0 | 0 | 0 | 0 | 0 | 0 | 0 | 0 | 2 | 0 | 0 | 0 | 0 | 0 | 0 | 0 | 3 | 0 | X55330 | 99.1 | 107 | 83 | 1 | 2150 |
| 252 | 00268 | 00251 | 5 | 2 | 0 | 0 | 0 | 0 | 0 | 0 | 0 | 2 | 0 | 0 | 0 | 0 | 0 | 0 | 0 | 0 | 1 | 0 | 0 | 0 | 0 | 0 | 0 | 0 | 0 | | | | | | |
| 253 | 00269 | 00252 | 1 | 1 | 0 | 0 | 0 | 0 | 0 | 0 | 0 | 0 | 0 | 0 | 0 | 0 | 0 | 0 | 0 | 0 | 0 | 0 | 0 | 0 | 0 | 0 | 0 | 0 | 0 | X63679 | 98.3 | 116 | 1 | 1152 | 1267 |
| 254 | 00270 | 00253 | 9 | 1 | 0 | 1 | 0 | 0 | 0 | 0 | 0 | 0 | 1 | 0 | 1 | 0 | 3 | 0 | 1 | 0 | 0 | 0 | 1 | 0 | 0 | 0 | 0 | 0 | 0 | X04526 | 95.2 | 209 | 1 | 2887 | 3088 |
| 255 | 00271 | 00254 | 24 | 2 | 0 | 1 | 2 | 0 | 1 | 0 | 0 | 2 | 0 | 0 | 0 | 5 | 0 | 1 | 2 | 0 | 1 | 1 | 0 | 0 | 0 | 2 | 0 | 4 | 0 | X75683 | 99 | 210 | 1 | 92 | 301 |
| 256 | 00272 | 00255 | 9 | 1 | 0 | 0 | 0 | 0 | 0 | 1 | 2 | 0 | 0 | 0 | 1 | 0 | 0 | 0 | 1 | 0 | 1 | 0 | 0 | 0 | 0 | 1 | 0 | 1 | 0 | J05262 | 100 | 197 | 1 | 931 | 1148 |
| 257 | 00273 | 00256 | 119 | 9 | 6 | 4 | 13 | 2 | 11 | 2 | 1 | 12 | 14 | 0 | 6 | 0 | 6 | 0 | 3 | 1 | 3 | 0 | 0 | 0 | 2 | 3 | 3 | 15 | 3 | X64899 | 94.8 | 192 | 1 | 626 | 819 |
| 258 | 00274 | 00257 | 2 | 2 | 0 | 0 | 0 | 0 | 0 | 0 | 0 | 0 | 0 | 0 | 0 | 0 | 0 | 0 | 0 | 0 | 0 | 0 | 0 | 0 | 0 | 0 | 0 | 0 | 0 | | | | | | |
| 259 | 00275 | 00258 | 1 | 1 | 0 | 0 | 0 | 0 | 0 | 0 | 0 | 0 | 0 | 0 | 0 | 0 | 0 | 0 | 0 | 0 | 0 | 0 | 0 | 0 | 0 | 0 | 0 | 0 | 0 | | | | | | |
| 260 | 00276 | 00259 | 5 | 1 | 0 | 0 | 0 | 0 | 0 | 0 | 0 | 0 | 0 | 0 | 0 | 0 | 1 | 0 | 0 | 0 | 1 | 1 | 0 | 0 | 1 | 0 | 0 | 0 | 0 | | | | | | |
| 261 | 00277 | 00260 | 36 | 3 | 0 | 0 | 0 | 8 | 14 | 11 | 0 | 0 | 0 | 0 | 0 | 0 | 0 | 0 | 0 | 0 | 0 | 0 | 0 | 0 | 0 | 0 | 0 | 0 | 0 | M29882 | 100 | 191 | 1 | 234 | 424 |
| 262 | 00278 | 00261 | 1 | 1 | 0 | 0 | 0 | 0 | 0 | 0 | 0 | 0 | 0 | 0 | 0 | 0 | 0 | 0 | 0 | 0 | 0 | 0 | 0 | 0 | 0 | 0 | 0 | 0 | 0 | | | | | | |
| 263 | 00279 | 00262 | 10 | 2 | 0 | 1 | 1 | 0 | 0 | 0 | 0 | 0 | 2 | 3 | 0 | 0 | 0 | 0 | 0 | 1 | 0 | 0 | 0 | 0 | 0 | 0 | 0 | 0 | 0 | | | | | | |
| 264 | 00280 | 00263 | 3 | 1 | 0 | 1 | 0 | 0 | 0 | 0 | 1 | 0 | 0 | 0 | 0 | 0 | 0 | 0 | 0 | 0 | 0 | 0 | 0 | 0 | 0 | 0 | 0 | 0 | 0 | X64228 | 99.5 | 196 | 1 | 6389 | 6597 |
| 265 | 00281 | 00264 | 8 | 3 | 0 | 0 | 1 | 0 | 0 | 0 | 0 | 0 | 0 | 0 | 0 | 0 | 1 | 0 | 0 | 1 | 0 | 0 | 0 | 1 | 0 | 0 | 0 | 1 | 0 | L07395 | 97.9 | 190 | 1 | 2036 | 2226 |
| 266 | 00282 | 00265 | 1 | 1 | 0 | 0 | 0 | 0 | 0 | 0 | 0 | 0 | 0 | 0 | 0 | 0 | 0 | 0 | 0 | 0 | 0 | 0 | 0 | 0 | 0 | 0 | 0 | 0 | 0 | | | | | | |
| 267 | 00283 | 00266 | 1 | 1 | 0 | 0 | 0 | 0 | 0 | 0 | 0 | 0 | 0 | 0 | 0 | 0 | 0 | 0 | 0 | 0 | 0 | 0 | 0 | 0 | 0 | 0 | 0 | 0 | 0 | | | | | | |
| 268 | 00284 | 00267 | 4 | 1 | 0 | 0 | 0 | 0 | 0 | 0 | 0 | 0 | 0 | 1 | 0 | 0 | 0 | 0 | 1 | 0 | 0 | 0 | 0 | 1 | 0 | 0 | 0 | 0 | 0 | | | | | | |
| 269 | 00285 | 00268 | 89 | 6 | 14 | 1 | 8 | 0 | 3 | 4 | 1 | 4 | 4 | 3 | 5 | 3 | 5 | 0 | 2 | 5 | 1 | 1 | 4 | 1 | 3 | 2 | 0 | 8 | 1 | | | | | | |
| 270 | 00286 | 00269 | 4 | 2 | 0 | 0 | 0 | 0 | 0 | 0 | 0 | 0 | 0 | 0 | 0 | 0 | 0 | 0 | 0 | 1 | 0 | 0 | 0 | 0 | 0 | 0 | 1 | 0 | 0 | L11284 | 96.3 | 189 | 1 | 1999 | 2222 |
| 271 | 00287 | 00270 | 2 | 1 | 0 | 0 | 0 | 0 | 0 | 0 | 0 | 0 | 0 | 0 | 0 | 0 | 0 | 1 | 0 | 0 | 0 | 0 | 0 | 0 | 0 | 0 | 0 | 0 | 0 | | | | | | |
| 272 | 00288 | 00271 | 1 | 1 | 0 | 0 | 0 | 0 | 0 | 0 | 0 | 0 | 0 | 0 | 0 | 0 | 0 | 0 | 0 | 0 | 0 | 0 | 0 | 0 | 0 | 0 | 0 | 0 | 0 | | | | | | |
| 273 | 00289 | 00272 | 4 | 2 | 0 | 0 | 0 | 0 | 0 | 0 | 0 | 0 | 0 | 0 | 0 | 0 | 0 | 0 | 0 | 0 | 0 | 1 | 0 | 0 | 0 | 0 | 0 | 1 | 0 | | | | | | |

Table 8

| | A | B | C | E | G | I | K | M | O | Q | S | U | W | Y | AA | AC | AE | AG | AI | AK | AM | AO | AQ | AS | AU | AW | AY | BA | BC | BE | BF | BG | BH | BI | BK |
|---|---|---|---|---|---|---|---|---|---|---|---|---|---|---|---|---|---|---|---|---|---|---|---|---|---|---|---|---|---|---|---|---|---|---|---|
| 274 | 00290 | 00273 | 85 | 5 | 9 | 3 | 8 | 4 | 2 | 1 | 1 | 1 | 7 | 4 | 1 | 4 | 5 | 0 | 2 | 6 | 5 | 3 | 6 | 1 | 0 | 2 | 0 | 5 | 0 | X66699 | 99.5 | 184 | 1 | 163 | 349 |
| 275 | 00291 | 00274 | 8 | 1 | 0 | 0 | 0 | 0 | 0 | 0 | 0 | 1 | 2 | 0 | 0 | 0 | 0 | 0 | 0 | 1 | 0 | 1 | 0 | 0 | 0 | 0 | 1 | 1 | 0 | | | | | | |
| 276 | 00292 | 00275 | 49 | 4 | 12 | 1 | 0 | 2 | 1 | 1 | 4 | 6 | 4 | 0 | 0 | 2 | 0 | 0 | 5 | 4 | 0 | 1 | 0 | 1 | 0 | 1 | 0 | 0 | 0 | M60854 | 100 | 181 | 1 | 358 | 538 |
| 277 | 00293 | 00276 | 74 | 6 | 13 | 1 | 6 | 2 | 4 | 3 | 3 | 2 | 5 | 0 | 1 | 0 | 4 | 0 | 0 | 2 | 2 | 0 | 0 | 0 | 1 | 5 | 0 | 11 | 3 | | | | | | |
| 278 | 00294 | 00277 | 5 | 1 | 0 | 0 | 0 | 0 | 0 | 0 | 1 | 0 | 1 | 0 | 0 | 0 | 0 | 0 | 0 | 0 | 1 | 0 | 0 | 1 | 0 | 0 | 0 | 0 | 0 | | | | | | |
| 279 | 00295 | 00278 | 3 | 1 | 0 | 0 | 0 | 0 | 0 | 0 | 0 | 0 | 0 | 0 | 0 | 0 | 0 | 1 | 0 | 0 | 0 | 0 | 0 | 0 | 0 | 0 | 1 | 0 | 0 | | | | | | |
| 280 | 00296 | 00279 | 8 | 1 | 4 | 0 | 0 | 0 | 0 | 0 | 1 | 0 | 0 | 0 | 0 | 0 | 0 | 0 | 0 | 0 | 1 | 0 | 1 | 0 | 0 | 0 | 0 | 0 | 0 | M86737 | 100 | 183 | 1 | 2641 | 2839 |
| 281 | 00297 | 00280 | 1 | 1 | 0 | 0 | 0 | 0 | 0 | 0 | 0 | 0 | 0 | 0 | 0 | 0 | 0 | 0 | 0 | 0 | 0 | 0 | 0 | 0 | 0 | 0 | 0 | 0 | 0 | | | | | | |
| 282 | 00298 | 00281 | 2 | 2 | 0 | 0 | 0 | 0 | 0 | 0 | 0 | 0 | 0 | 0 | 0 | 0 | 0 | 0 | 0 | 0 | 0 | 0 | 0 | 0 | 0 | 0 | 0 | 0 | 0 | | | | | | |
| 283 | 00299 | 00282 | 1 | 1 | 0 | 0 | 0 | 0 | 0 | 0 | 0 | 0 | 0 | 0 | 0 | 0 | 0 | 0 | 0 | 0 | 0 | 0 | 0 | 0 | 0 | 0 | 0 | 0 | 0 | | | | | | |
| 284 | 00300 | 00283 | 24 | 2 | 1 | 0 | 2 | 0 | 1 | 0 | 0 | 1 | 1 | 2 | 0 | 3 | 1 | 4 | 2 | 0 | 1 | 1 | 0 | 0 | 1 | 1 | 0 | 0 | 0 | M37104 | 98.3 | 179 | 1 | 294 | 471 |
| 285 | 00301 | 00284 | 1 | 1 | 0 | 0 | 0 | 0 | 0 | 0 | 0 | 0 | 0 | 0 | 0 | 0 | 0 | 0 | 0 | 0 | 0 | 0 | 0 | 0 | 0 | 0 | 0 | 0 | 0 | | | | | | |
| 286 | 00302 | 00285 | 4 | 1 | 0 | 1 | 0 | 0 | 1 | 1 | 0 | 0 | 0 | 0 | 0 | 0 | 0 | 0 | 0 | 0 | 0 | 0 | 0 | 0 | 0 | 0 | 0 | 0 | 0 | | | | | | |
| 287 | 00303 | 00286 | 17 | 2 | 2 | 0 | 0 | 0 | 0 | 1 | 0 | 0 | 2 | 0 | 0 | 0 | 0 | 1 | 2 | 0 | 1 | 0 | 0 | 0 | 0 | 0 | 2 | 2 | 2 | L09260 | 96 | 176 | 1 | 1141 | 1322 |
| 288 | 00304 | 00287 | 48 | 2 | 1 | 2 | 2 | 1 | 0 | 1 | 1 | 3 | 2 | 2 | 3 | 1 | 0 | 4 | 2 | 6 | 0 | 3 | 0 | 1 | 4 | 3 | 3 | 1 | 0 | M26880 | 99.4 | 175 | 1 | 2088 | 2309 |
| 289 | 00305 | 00288 | 46 | 1 | 6 | 0 | 2 | 2 | 3 | 1 | 2 | 1 | 4 | 4 | 2 | 2 | 0 | 3 | 0 | 1 | 4 | 1 | 0 | 2 | 2 | 1 | 2 | 0 | 0 | | | | | | |
| 290 | 00306 | 00289 | 4 | 1 | 1 | 0 | 0 | 0 | 0 | 0 | 0 | 0 | 0 | 0 | 0 | 0 | 0 | 0 | 0 | 0 | 1 | 0 | 0 | 0 | 0 | 1 | 0 | 0 | 0 | | | | | | |
| 291 | 00307 | 00290 | 4 | 4 | 0 | 0 | 0 | 0 | 0 | 0 | 0 | 0 | 0 | 0 | 0 | 0 | 0 | 0 | 0 | 0 | 0 | 0 | 0 | 0 | 0 | 0 | 0 | 0 | 0 | | | | | | |
| 292 | 00308 | 00291 | 10 | 1 | 0 | 0 | 0 | 0 | 1 | 0 | 0 | 0 | 1 | 0 | 0 | 0 | 0 | 0 | 0 | 1 | 3 | 1 | 0 | 2 | 0 | 0 | 0 | 0 | 0 | Y00052 | 96.7 | 183 | 1 | 542 | 723 |
| 293 | 00309 | 00292 | 1 | 1 | 0 | 0 | 0 | 0 | 0 | 0 | 0 | 0 | 0 | 0 | 0 | 0 | 0 | 0 | 0 | 0 | 0 | 0 | 0 | 0 | 0 | 0 | 0 | 0 | 0 | | | | | | |
| 294 | 00310 | 00293 | 6 | 1 | 0 | 2 | 0 | 0 | 0 | 0 | 0 | 0 | 1 | 0 | 0 | 0 | 0 | 0 | 0 | 0 | 0 | 0 | 0 | 0 | 0 | 0 | 0 | 1 | 1 | M72709 | 98.8 | 85 | 1 | 1634 | 1717 |
| 295 | 00311 | 00294 | 2 | 1 | 0 | 0 | 0 | 0 | 0 | 0 | 0 | 0 | 0 | 0 | 0 | 0 | 0 | 0 | 0 | 0 | 0 | 0 | 0 | 0 | 0 | 1 | 0 | 0 | 0 | | | | | | |
| 296 | 00312 | 00295 | 2 | 1 | 0 | 0 | 0 | 0 | 0 | 0 | 0 | 0 | 0 | 0 | 0 | 0 | 0 | 0 | 0 | 0 | 0 | 0 | 0 | 0 | 0 | 0 | 0 | 1 | 0 | | | | | | |
| 297 | 00313 | 00296 | 17 | 1 | 0 | 1 | 0 | 1 | 0 | 0 | 1 | 0 | 0 | 0 | 0 | 1 | 0 | 2 | 2 | 1 | 0 | 3 | 2 | 0 | 0 | 0 | 2 | 0 | 0 | Y00472 | 96.8 | 63 | 1 | 767 | 829 |
| 298 | 00314 | 00297 | 45 | 1 | 3 | 1 | 3 | 0 | 1 | 1 | 5 | 1 | 1 | 0 | 0 | 7 | 2 | 0 | 3 | 2 | 0 | 3 | 0 | 0 | 4 | 3 | 2 | 0 | 2 | X06617 | 96.4 | 168 | 1 | 378 | 543 |
| 299 | 00315 | 00298 | 21 | 3 | 4 | 1 | 0 | 0 | 1 | 0 | 1 | 3 | 2 | 0 | 0 | 0 | 1 | 0 | 0 | 3 | 0 | 0 | 0 | 0 | 0 | 0 | 0 | 2 | 0 | L06498 | 99.4 | 161 | 1 | 345 | 505 |
| 300 | 00316 | 00299 | 1 | 1 | 0 | 0 | 0 | 0 | 0 | 0 | 0 | 0 | 0 | 0 | 0 | 0 | 0 | 0 | 0 | 0 | 0 | 0 | 0 | 0 | 0 | 0 | 0 | 0 | 0 | S63912 | 96.8 | 63 | 1 | 1126 | 3043 |
| 301 | 00317 | 00300 | 10 | 1 | 0 | 0 | 0 | 3 | 0 | 2 | 0 | 0 | 0 | 1 | 0 | 0 | 0 | 0 | 1 | 0 | 1 | 1 | 0 | 0 | 0 | 0 | 0 | 0 | 0 | | | | | | |
| 302 | 00320 | 00301 | 1 | 1 | 0 | 0 | 0 | 0 | 0 | 0 | 0 | 0 | 0 | 0 | 0 | 0 | 0 | 0 | 0 | 0 | 0 | 0 | 0 | 0 | 0 | 0 | 0 | 0 | 0 | | | | | | |
| 303 | 00322 | 00302 | 2 | 1 | 0 | 0 | 0 | 0 | 0 | 0 | 0 | 0 | 0 | 0 | 0 | 0 | 0 | 0 | 0 | 0 | 0 | 0 | 0 | 0 | 0 | 1 | 0 | 0 | 0 | | | | | | |
| 304 | 00323 | 00303 | 2 | 1 | 0 | 0 | 0 | 0 | 0 | 0 | 0 | 0 | 0 | 0 | 0 | 0 | 0 | 0 | 0 | 0 | 0 | 0 | 0 | 0 | 0 | 0 | 0 | 1 | 0 | | | | | | |
| 305 | 00325 | 00304 | 1 | 1 | 0 | 0 | 0 | 0 | 0 | 0 | 0 | 0 | 0 | 0 | 0 | 0 | 0 | 0 | 0 | 0 | 0 | 0 | 0 | 0 | 0 | 0 | 0 | 0 | 0 | | | | | | |
| 306 | 00327 | 00305 | 20 | 1 | 3 | 1 | 0 | 0 | 1 | 2 | 0 | 0 | 0 | 0 | 0 | 2 | 1 | 0 | 0 | 0 | 2 | 3 | 0 | 0 | 3 | 0 | 0 | 1 | 0 | X03342 | 96.4 | 169 | 1 | 328 | 505 |
| 307 | 00328 | 00306 | 2 | 1 | 0 | 0 | 0 | 0 | 0 | 0 | 0 | 0 | 0 | 0 | 0 | 0 | 0 | 0 | 1 | 0 | 0 | 0 | 0 | 0 | 0 | 0 | 0 | 0 | 0 | | | | | | |
| 308 | 00329 | 00307 | 5 | 1 | 0 | 0 | 1 | 0 | 0 | 0 | 0 | 0 | 0 | 0 | 1 | 0 | 0 | 0 | 0 | 0 | 0 | 0 | 0 | 0 | 0 | 1 | 0 | 1 | 0 | | | | | | |
| 309 | 00330 | 00308 | 8 | 1 | 0 | 2 | 3 | 0 | 0 | 0 | 0 | 0 | 1 | 0 | 0 | 0 | 0 | 0 | 0 | 1 | 0 | 0 | 0 | 0 | 0 | 0 | 0 | 0 | 0 | | | | | | |

Table 9

| | A | B | C | E | G | I | K | M | O | Q | S | U | W | Y | AA | AC | AE | AG | AI | AK | AM | AO | AQ | AS | AU | AW | AY | BA | BC | BE | BF | BG | BH | BI | BK |
|---|---|---|---|---|---|---|---|---|---|---|---|---|---|---|---|---|---|---|---|---|---|---|---|---|---|---|---|---|---|---|---|---|---|---|---|
| 310 | 00331 | 00309 | 6 | 4 | 0 | 0 | 0 | 0 | 0 | 0 | 0 | 0 | 0 | 0 | 0 | 0 | 0 | 0 | 1 | 0 | 0 | 0 | 0 | 0 | 0 | 0 | 0 | 1 | 0 | | | | | | |
| 311 | 00332 | 00310 | 2 | 1 | 0 | 0 | 0 | 0 | 0 | 0 | 0 | 0 | 0 | 0 | 1 | 0 | 0 | 0 | 0 | 0 | 0 | 0 | 0 | 0 | 0 | 0 | 0 | 0 | 0 | X12517 | 98.8 | 165 | 1 | 554 | 733 |
| 312 | 00333 | 00311 | 16 | 1 | 0 | 0 | 3 | 0 | 0 | 0 | 1 | 1 | 4 | 0 | 0 | 0 | 0 | 0 | 2 | 2 | 0 | 1 | 0 | 0 | 0 | 1 | 0 | 0 | 0 | M34539 | 98.1 | 157 | 1 | 1375 | 1532 |
| 313 | 00334 | 00312 | 3 | 1 | 0 | 1 | 0 | 0 | 0 | 0 | 0 | 0 | 0 | 0 | 0 | 0 | 0 | 0 | 0 | 0 | 0 | 0 | 0 | 0 | 0 | 0 | 0 | 1 | 0 | | | | | | |
| 314 | 00335 | 00313 | 86 | 1 | 16 | 4 | 3 | 1 | 1 | 0 | 3 | 7 | 3 | 1 | 6 | 6 | 1 | 3 | 2 | 1 | 1 | 1 | 1 | 4 | 2 | 5 | 0 | 10 | 3 | M36072 | 100 | 165 | 1 | 727 | 891 |
| 315 | 00336 | 00314 | 47 | 1 | 2 | 2 | 2 | 0 | 1 | 0 | 0 | 5 | 6 | 0 | 1 | 2 | 0 | 3 | 4 | 3 | 1 | 1 | 1 | 0 | 1 | 8 | 0 | 3 | 0 | Z12962 | 96.3 | 162 | 1 | 308 | 478 |
| 316 | 00337 | 00315 | 5 | 1 | 0 | 0 | 0 | 0 | 0 | 0 | 0 | 0 | 0 | 0 | 0 | 0 | 0 | 1 | 0 | 0 | 0 | 0 | 2 | 1 | 0 | 0 | 0 | 0 | 0 | | | | | | |
| 317 | 00338 | 00316 | 2 | 1 | 0 | 0 | 0 | 0 | 0 | 0 | 0 | 0 | 0 | 0 | 0 | 0 | 0 | 0 | 1 | 0 | 0 | 0 | 0 | 0 | 0 | 0 | 0 | 0 | 0 | | | | | | |
| 318 | 00339 | 00317 | 13 | 6 | 0 | 0 | 0 | 0 | 0 | 0 | 0 | 0 | 0 | 0 | 0 | 0 | 0 | 0 | 1 | 0 | 0 | 0 | 0 | 0 | 0 | 6 | 0 | 0 | 0 | J03934 | 96.8 | 155 | 1 | 857 | 2447 |
| 319 | 00340 | 00318 | 2 | 1 | 0 | 0 | 0 | 0 | 0 | 0 | 0 | 0 | 0 | 0 | 0 | 0 | 1 | 0 | 0 | 0 | 0 | 0 | 0 | 0 | 0 | 0 | 0 | 0 | 0 | | | | | | |
| 320 | 00341 | 00319 | 2 | 1 | 0 | 0 | 0 | 0 | 0 | 0 | 0 | 0 | 0 | 0 | 0 | 0 | 0 | 0 | 0 | 0 | 0 | 0 | 0 | 0 | 0 | 0 | 0 | 0 | 1 | | | | | | |
| 321 | 00342 | 00320 | 5 | 1 | 0 | 0 | 0 | 0 | 0 | 1 | 0 | 0 | 0 | 0 | 0 | 0 | 0 | 1 | 0 | 0 | 0 | 0 | 0 | 2 | 0 | 0 | 0 | 0 | 0 | | | | | | |
| 322 | 00343 | 00321 | 12 | 2 | 0 | 0 | 1 | 0 | 0 | 2 | 0 | 1 | 0 | 0 | 0 | 3 | 0 | 0 | 1 | 0 | 2 | 0 | 0 | 0 | 0 | 0 | 0 | 0 | 0 | L07633 | 97.4 | 153 | 1 | 833 | 985 |
| 323 | 00344 | 00322 | 1 | 1 | 0 | 0 | 0 | 0 | 0 | 0 | 0 | 0 | 0 | 0 | 0 | 0 | 0 | 0 | 0 | 0 | 0 | 0 | 0 | 0 | 0 | 0 | 0 | 0 | 0 | | | | | | |
| 324 | 00346 | 00323 | 9 | 1 | 0 | 0 | 0 | 0 | 0 | 1 | 1 | 0 | 0 | 1 | 0 | 0 | 1 | 0 | 0 | 2 | 0 | 0 | 1 | 1 | 0 | 0 | 0 | 0 | 0 | S42658 | 99.6 | 250 | 1 | 569 | 826 |
| 325 | 00347 | 00324 | 1 | 1 | 0 | 0 | 0 | 0 | 0 | 0 | 0 | 0 | 0 | 0 | 0 | 0 | 0 | 0 | 0 | 0 | 0 | 0 | 0 | 0 | 0 | 0 | 0 | 0 | 0 | | | | | | |
| 326 | 00348 | 00325 | 10 | 1 | 1 | 0 | 0 | 0 | 0 | 0 | 1 | 0 | 0 | 0 | 0 | 0 | 0 | 0 | 0 | 1 | 0 | 1 | 1 | 3 | 1 | 0 | 0 | 0 | 0 | | | | | | |
| 327 | 00349 | 00326 | 29 | 2 | 2 | 0 | 0 | 1 | 0 | 0 | 2 | 1 | 2 | 0 | 1 | 0 | 0 | 1 | 1 | 3 | 1 | 1 | 1 | 0 | 1 | 5 | 0 | 4 | 0 | X74070 | 93.7 | 159 | 1 | 699 | 857 |
| 328 | 00350 | 00327 | 6 | 1 | 0 | 0 | 0 | 0 | 0 | 0 | 0 | 0 | 0 | 0 | 0 | 0 | 0 | 0 | 0 | 0 | 0 | 0 | 0 | 3 | 0 | 1 | 1 | 0 | 0 | | | | | | |
| 329 | 00351 | 00328 | 8 | 1 | 0 | 0 | 0 | 0 | 0 | 0 | 1 | 0 | 0 | 0 | 1 | 0 | 2 | 0 | 0 | 1 | 2 | 0 | 0 | 0 | 0 | 0 | 0 | 0 | 0 | | | | | | |
| 330 | 00352 | 00329 | 7 | 1 | 0 | 0 | 0 | 0 | 0 | 0 | 0 | 0 | 2 | 0 | 0 | 0 | 1 | 0 | 0 | 0 | 1 | 1 | 0 | 0 | 0 | 0 | 0 | 1 | 0 | | | | | | |
| 331 | 00353 | 00330 | 3 | 1 | 0 | 0 | 0 | 0 | 0 | 0 | 0 | 0 | 0 | 0 | 0 | 0 | 0 | 0 | 0 | 1 | 0 | 0 | 0 | 1 | 0 | 0 | 0 | 0 | 0 | | | | | | |
| 332 | 00354 | 00331 | 6 | 1 | 1 | 0 | 0 | 0 | 0 | 0 | 0 | 0 | 1 | 1 | 1 | 0 | 0 | 0 | 0 | 0 | 0 | 0 | 1 | 0 | 0 | 0 | 0 | 0 | 0 | | | | | | |
| 333 | 00355 | 00332 | 1 | 1 | 0 | 0 | 0 | 0 | 0 | 0 | 0 | 0 | 0 | 0 | 0 | 0 | 0 | 0 | 0 | 0 | 0 | 0 | 0 | 0 | 0 | 0 | 0 | 0 | 0 | | | | | | |
| 334 | 00356 | 00333 | 43 | 1 | 17 | 0 | 2 | 2 | 1 | 3 | 0 | 0 | 5 | 0 | 0 | 1 | 0 | 0 | 0 | 1 | 1 | 1 | 0 | 0 | 1 | 0 | 1 | 4 | 2 | X53777 | 96 | 151 | 1 | 582 | 770 |
| 335 | 00357 | 00334 | 3 | 1 | 0 | 0 | 0 | 0 | 0 | 0 | 0 | 0 | 0 | 0 | 0 | 0 | 1 | 0 | 0 | 0 | 0 | 0 | 0 | 0 | 0 | 0 | 0 | 1 | 0 | | | | | | |
| 336 | 00358 | 00335 | 2 | 2 | 0 | 0 | 0 | 0 | 0 | 0 | 0 | 0 | 0 | 0 | 0 | 0 | 0 | 0 | 0 | 0 | 0 | 0 | 0 | 0 | 0 | 0 | 0 | 0 | 0 | | | | | | |
| 337 | 00359 | 00336 | 2 | 1 | 0 | 0 | 0 | 0 | 0 | 0 | 0 | 0 | 0 | 0 | 0 | 0 | 0 | 1 | 0 | 0 | 0 | 0 | 0 | 0 | 0 | 0 | 0 | 0 | 0 | | | | | | |
| 338 | 00360 | 00337 | 19 | 2 | 2 | 1 | 0 | 0 | 0 | 0 | 1 | 1 | 1 | 1 | 0 | 1 | 0 | 0 | 2 | 1 | 0 | 2 | 0 | 0 | 0 | 1 | 1 | 2 | 0 | X60036 | 97.2 | 145 | 1 | 1171 | 1330 |
| 339 | 00361 | 00338 | 11 | 1 | 0 | 1 | 0 | 0 | 0 | 0 | 0 | 2 | 0 | 0 | 1 | 0 | 1 | 1 | 0 | 0 | 3 | 0 | 0 | 0 | 1 | 0 | 0 | 0 | 0 | | | | | | |
| 340 | 00362 | 00339 | 19 | 1 | 0 | 0 | 0 | 0 | 0 | 0 | 2 | 0 | 12 | 2 | 1 | 0 | 0 | 0 | 1 | 0 | 0 | 0 | 0 | 0 | 0 | 0 | 0 | 0 | 0 | | | | | | |
| 341 | 00363 | 00340 | 29 | 1 | 0 | 0 | 0 | 1 | 0 | 1 | 1 | 0 | 5 | 0 | 2 | 0 | 1 | 1 | 3 | 2 | 2 | 1 | 3 | 1 | 1 | 1 | 1 | 1 | 0 | D14696 | 98.6 | 141 | 1 | 1262 | 1402 |
| 342 | 00364 | 00341 | 632 | 17 | 0 | 0 | 0 | 227 | 109 | ## | 0 | 0 | 0 | 0 | 0 | 0 | 0 | 0 | 0 | 0 | 0 | 0 | 0 | 0 | 0 | 0 | 0 | 0 | 0 | L00132 | 98.6 | 290 | 1 | 1961 | 2251 |
| 343 | 00365 | 00342 | 2 | 1 | 0 | 0 | 0 | 0 | 0 | 0 | 0 | 0 | 0 | 0 | 0 | 0 | 0 | 0 | 0 | 0 | 0 | 0 | 0 | 0 | 0 | 0 | 0 | 1 | 0 | | | | | | |
| 344 | 00366 | 00343 | 4 | 1 | 0 | 0 | 0 | 1 | 0 | 0 | 0 | 0 | 0 | 0 | 0 | 0 | 0 | 0 | 0 | 0 | 0 | 2 | 0 | 0 | 0 | 0 | 0 | 0 | 0 | | | | | | |
| 345 | 00367 | 00344 | 1 | 1 | 0 | 0 | 0 | 0 | 0 | 0 | 0 | 0 | 0 | 0 | 0 | 0 | 0 | 0 | 0 | 0 | 0 | 0 | 0 | 0 | 0 | 0 | 0 | 0 | 0 | | | | | | |

Table 10

| | A | B | C | E | G | I | K | M | O | Q | S | U | W | Y | AA | AC | AE | AG | AI | AK | AM | AO | AQ | AS | AU | AW | AY | BA | BC | BE | BF | BG | BH | BI | BK |
|---|---|---|---|---|---|---|---|---|---|---|---|---|---|---|---|---|---|---|---|---|---|---|---|---|---|---|---|---|---|---|---|---|---|---|---|
| 346 | 00368 | 00345 | 11 | 1 | 0 | 0 | 0 | 2 | 0 | 0 | 2 | 0 | 0 | 0 | 0 | 0 | 0 | 0 | 3 | 0 | 0 | 1 | 0 | 1 | 0 | 0 | 0 | 0 | 1 | M31627 | 98.6 | 141 | 1 | 1657 | 1818 |
| 347 | 00369 | 00346 | 3 | 1 | 1 | 0 | 0 | 0 | 0 | 0 | 0 | 0 | 0 | 0 | 0 | 0 | 0 | 0 | 0 | 0 | 0 | 0 | 0 | 1 | 0 | 0 | 0 | 0 | 0 | L10379 | 98.6 | 142 | 1 | 1581 | 1725 |
| 348 | 00370 | 00347 | 7 | 1 | 0 | 0 | 0 | 0 | 0 | 0 | 0 | 0 | 0 | 0 | 0 | 2 | 0 | 1 | 0 | 0 | 0 | 0 | 0 | 0 | 0 | 0 | 3 | 0 | 0 | | | | | | |
| 349 | 00372 | 00348 | 1 | 1 | 0 | 0 | 0 | 0 | 0 | 0 | 0 | 0 | 0 | 0 | 0 | 0 | 0 | 0 | 0 | 0 | 0 | 0 | 0 | 0 | 0 | 0 | 0 | 0 | 0 | | | | | | |
| 350 | 00373 | 00349 | 2 | 1 | 0 | 0 | 0 | 0 | 0 | 0 | 0 | 0 | 0 | 0 | 0 | 0 | 0 | 0 | 0 | 1 | 0 | 0 | 0 | 0 | 0 | 0 | 0 | 0 | 0 | | | | | | |
| 351 | 00374 | 00350 | 10 | 1 | 1 | 0 | 0 | 0 | 0 | 1 | 2 | 0 | 1 | 0 | 0 | 1 | 0 | 0 | 0 | 2 | 0 | 0 | 0 | 0 | 0 | 1 | 0 | 0 | 0 | | | | | | |
| 352 | 00375 | 00351 | 36 | 2 | 3 | 0 | 3 | 1 | 2 | 0 | 0 | 3 | 5 | 0 | 0 | 1 | 0 | 0 | 2 | 2 | 0 | 0 | 2 | 0 | 2 | 1 | 0 | 1 | 6 | | | | | | |
| 353 | 00376 | 00352 | 21 | 2 | 0 | 0 | 0 | 1 | 0 | 0 | 0 | 1 | 1 | 1 | 1 | 0 | 0 | 0 | 1 | 4 | 1 | 3 | 1 | 0 | 2 | 1 | 0 | 1 | 0 | | | | | | |
| 354 | 00377 | 00353 | 2 | 1 | 0 | 0 | 0 | 0 | 0 | 0 | 0 | 0 | 0 | 0 | 0 | 0 | 0 | 0 | 0 | 0 | 0 | 0 | 1 | 0 | 0 | 0 | 0 | 0 | 0 | | | | | | |
| 355 | 00378 | 00354 | 3 | 2 | 0 | 0 | 0 | 0 | 0 | 0 | 0 | 0 | 0 | 0 | 0 | 0 | 0 | 0 | 0 | 0 | 0 | 0 | 0 | 0 | 0 | 0 | 0 | 1 | 0 | | | | | | |
| 356 | 00379 | 00355 | 1 | 1 | 0 | 0 | 0 | 0 | 0 | 0 | 0 | 0 | 0 | 0 | 0 | 0 | 0 | 0 | 0 | 0 | 0 | 0 | 0 | 0 | 0 | 0 | 0 | 0 | 0 | | | | | | |
| 357 | 00380 | 00356 | 47 | 1 | 12 | 0 | 1 | 0 | 1 | 3 | 0 | 2 | 5 | 2 | 0 | 1 | 3 | 1 | 3 | 1 | 1 | 1 | 1 | 1 | 1 | 2 | 0 | 3 | 1 | | | | | | |
| 358 | 00381 | 00357 | 22 | 1 | 0 | 2 | 6 | 0 | 0 | 0 | 0 | 0 | 0 | 0 | 6 | 0 | 1 | 0 | 0 | 0 | 1 | 1 | 0 | 0 | 1 | 1 | 1 | 0 | 1 | | | | | | |
| 359 | 00382 | 00358 | 4 | 3 | 0 | 0 | 0 | 0 | 0 | 0 | 0 | 0 | 0 | 0 | 0 | 0 | 0 | 1 | 0 | 0 | 0 | 0 | 0 | 0 | 0 | 0 | 0 | 0 | 0 | | | | | | |
| 360 | 00383 | 00359 | 28 | 3 | 1 | 3 | 3 | 0 | 1 | 0 | 0 | 2 | 0 | 0 | 1 | 3 | 0 | 0 | 1 | 3 | 2 | 1 | 0 | 0 | 0 | 0 | 1 | 3 | 0 | | | | | | |
| 361 | 00384 | 00360 | 10 | 1 | 0 | 0 | 0 | 0 | 0 | 2 | 0 | 1 | 0 | 1 | 0 | 0 | 0 | 0 | 2 | 0 | 0 | 0 | 0 | 0 | 0 | 0 | 0 | 3 | 0 | J04031 | 97.8 | 136 | 1 | 2977 | 3112 |
| 362 | 00385 | 00361 | 4 | 3 | 0 | 0 | 0 | 1 | 0 | 0 | 0 | 0 | 0 | 0 | 0 | 0 | 0 | 0 | 0 | 0 | 0 | 0 | 0 | 0 | 0 | 0 | 0 | 0 | 0 | | | | | | |
| 363 | 00386 | 00362 | 6 | 2 | 0 | 0 | 0 | 0 | 0 | 0 | 0 | 0 | 0 | 1 | 0 | 0 | 0 | 0 | 2 | 0 | 0 | 0 | 0 | 1 | 0 | 0 | 0 | 0 | 0 | | | | | | |
| 364 | 00387 | 00363 | 5 | 1 | 0 | 0 | 0 | 0 | 0 | 2 | 0 | 0 | 0 | 0 | 0 | 0 | 0 | 0 | 0 | 0 | 0 | 0 | 0 | 1 | 0 | 0 | 0 | 0 | 1 | L11932 | 100 | 126 | 1 | 1667 | 1792 |
| 365 | 00388 | 00364 | 2 | 1 | 0 | 0 | 0 | 0 | 0 | 0 | 0 | 0 | 0 | 0 | 0 | 0 | 0 | 0 | 0 | 0 | 0 | 0 | 0 | 0 | 1 | 0 | 0 | 0 | 0 | | | | | | |
| 366 | 00389 | 00365 | 4 | 3 | 0 | 0 | 1 | 0 | 0 | 0 | 0 | 0 | 0 | 0 | 0 | 0 | 0 | 0 | 0 | 0 | 0 | 0 | 0 | 0 | 0 | 0 | 0 | 0 | 0 | | | | | | |
| 367 | 00390 | 00366 | 1 | 1 | 0 | 0 | 0 | 0 | 0 | 0 | 0 | 0 | 0 | 0 | 0 | 0 | 0 | 0 | 0 | 0 | 0 | 0 | 0 | 0 | 0 | 0 | 0 | 0 | 0 | | | | | | |
| 368 | 00391 | 00367 | 2 | 1 | 0 | 0 | 0 | 0 | 0 | 0 | 0 | 0 | 0 | 0 | 0 | 0 | 0 | 0 | 0 | 0 | 0 | 0 | 0 | 0 | 0 | 1 | 0 | 0 | 0 | | | | | | |
| 369 | 00392 | 00368 | 3 | 1 | 0 | 0 | 0 | 0 | 0 | 0 | 0 | 0 | 0 | 0 | 0 | 0 | 0 | 2 | 0 | 0 | 0 | 0 | 0 | 0 | 0 | 0 | 0 | 0 | 0 | | | | | | |
| 370 | 00393 | 00369 | 2 | 1 | 0 | 0 | 0 | 0 | 1 | 0 | 0 | 0 | 0 | 0 | 0 | 0 | 0 | 0 | 0 | 0 | 0 | 0 | 0 | 0 | 0 | 0 | 0 | 0 | 0 | | | | | | |
| 371 | 00394 | 00370 | 2 | 2 | 0 | 0 | 0 | 0 | 0 | 0 | 0 | 0 | 0 | 0 | 0 | 0 | 0 | 0 | 0 | 0 | 0 | 0 | 0 | 0 | 0 | 0 | 0 | 0 | 0 | | | | | | |
| 372 | 00395 | 00371 | 1 | 1 | 0 | 0 | 0 | 0 | 0 | 0 | 0 | 0 | 0 | 0 | 0 | 0 | 0 | 0 | 0 | 0 | 0 | 0 | 0 | 0 | 0 | 0 | 0 | 0 | 0 | | | | | | |
| 373 | 00396 | 00372 | 1 | 1 | 0 | 0 | 0 | 0 | 0 | 0 | 0 | 0 | 0 | 0 | 0 | 0 | 0 | 0 | 0 | 0 | 0 | 0 | 0 | 0 | 0 | 0 | 0 | 0 | 0 | | | | | | |
| 374 | 00397 | 00373 | 2 | 2 | 0 | 0 | 0 | 0 | 0 | 0 | 0 | 0 | 0 | 0 | 0 | 0 | 0 | 0 | 0 | 0 | 0 | 0 | 0 | 0 | 0 | 0 | 0 | 0 | 0 | | | | | | |
| 375 | 00398 | 00374 | 1 | 1 | 0 | 0 | 0 | 0 | 0 | 0 | 0 | 0 | 0 | 0 | 0 | 0 | 0 | 0 | 0 | 0 | 0 | 0 | 0 | 0 | 0 | 0 | 0 | 0 | 0 | | | | | | |
| 376 | 00399 | 00375 | 1 | 1 | 0 | 0 | 0 | 0 | 0 | 0 | 0 | 0 | 0 | 0 | 0 | 0 | 0 | 0 | 0 | 0 | 0 | 0 | 0 | 0 | 0 | 0 | 0 | 0 | 0 | | | | | | |
| 377 | 00400 | 00376 | 1 | 1 | 0 | 0 | 0 | 0 | 0 | 0 | 0 | 0 | 0 | 0 | 0 | 0 | 0 | 0 | 0 | 0 | 0 | 0 | 0 | 0 | 0 | 0 | 0 | 0 | 0 | M22490 | 94.9 | 118 | 1 | 1622 | 1751 |
| 378 | 00401 | 00377 | 16 | 1 | 0 | 0 | 0 | 2 | 0 | 0 | 0 | 0 | 0 | 0 | 2 | 2 | 0 | 0 | 0 | 2 | 1 | 1 | 0 | 1 | 0 | 1 | 0 | 3 | 0 | L24521 | 94.5 | 220 | 1 | 1024 | 1240 |
| 379 | 00402 | 00378 | 39 | 1 | 0 | 0 | 2 | 2 | 0 | 0 | 1 | 0 | 2 | 0 | 0 | 0 | 0 | 0 | 0 | 4 | 1 | 0 | 0 | 0 | 1 | 3 | 22 | 0 | 0 | M77693 | 97.6 | 125 | 1 | 917 | 1060 |
| 380 | 00403 | 00379 | 21 | 7 | 0 | 1 | 0 | 0 | 0 | 0 | 0 | 0 | 1 | 1 | 0 | 1 | 3 | 0 | 1 | 0 | 2 | 0 | 2 | 0 | 0 | 0 | 0 | 2 | 0 | | | | | | |
| 381 | 00404 | 00380 | 2 | 1 | 0 | 0 | 0 | 0 | 0 | 0 | 0 | 0 | 0 | 0 | 0 | 0 | 0 | 0 | 0 | 0 | 0 | 0 | 0 | 0 | 0 | 0 | 1 | 0 | 0 | | | | | | |

Table 11

| | A | B | C | E | G | I | K | M | O | Q | S | U | W | Y | AA | AC | AE | AG | AI | AK | AM | AO | AQ | AS | AU | AW | AY | BA | BC | BE | BF | BG | BH | BI | BK |
|---|---|---|---|---|---|---|---|---|---|---|---|---|---|---|---|---|---|---|---|---|---|---|---|---|---|---|---|---|---|---|---|---|---|---|---|
| 382 | 00405 | 00381 | 3 | 1 | 0 | 0 | 0 | 0 | 0 | 0 | 0 | 0 | 0 | 0 | 0 | 0 | 0 | 0 | 0 | 0 | 1 | 0 | 0 | 0 | 0 | 1 | 0 | 0 | 0 | | | | | | |
| 383 | 00406 | 00382 | 1 | 1 | 0 | 0 | 0 | 0 | 0 | 0 | 0 | 0 | 0 | 0 | 0 | 0 | 0 | 0 | 0 | 0 | 0 | 0 | 0 | 0 | 0 | 0 | 0 | 0 | 0 | | | | | | |
| 384 | 00407 | 00383 | 1 | 1 | 0 | 0 | 0 | 0 | 0 | 0 | 0 | 0 | 0 | 0 | 0 | 0 | 0 | 0 | 0 | 0 | 0 | 0 | 0 | 0 | 0 | 0 | 0 | 0 | 0 | S85655 | 93.9 | 114 | 1 | 755 | 1043 |
| 385 | 00408 | 00384 | 1 | 1 | 0 | 0 | 0 | 0 | 0 | 0 | 0 | 0 | 0 | 0 | 0 | 0 | 0 | 0 | 0 | 0 | 0 | 0 | 0 | 0 | 0 | 0 | 0 | 0 | 0 | | | | | | |
| 386 | 00409 | 00385 | 2 | 1 | 0 | 0 | 0 | 0 | 0 | 0 | 0 | 0 | 0 | 0 | 0 | 0 | 0 | 0 | 0 | 0 | 0 | 0 | 0 | 0 | 0 | 1 | 0 | 0 | 0 | | | | | | |
| 387 | 00410 | 00386 | 3 | 1 | 0 | 0 | 0 | 0 | 0 | 0 | 1 | 0 | 0 | 0 | 0 | 0 | 1 | 0 | 0 | 0 | 0 | 0 | 0 | 0 | 0 | 0 | 0 | 0 | 0 | J04046 | 100 | 117 | 1 | 589 | 2175 |
| 388 | 00411 | 00387 | 2 | 1 | 0 | 0 | 0 | 0 | 0 | 0 | 0 | 0 | 0 | 0 | 0 | 0 | 0 | 0 | 0 | 0 | 0 | 0 | 0 | 1 | 0 | 0 | 0 | 0 | 0 | | | | | | |
| 389 | 00412 | 00388 | 1 | 1 | 0 | 0 | 0 | 0 | 0 | 0 | 0 | 0 | 0 | 0 | 0 | 0 | 0 | 0 | 0 | 0 | 0 | 0 | 0 | 0 | 0 | 0 | 0 | 0 | 0 | | | | | | |
| 390 | 00413 | 00389 | 1 | 1 | 0 | 0 | 0 | 0 | 0 | 0 | 0 | 0 | 0 | 0 | 0 | 0 | 0 | 0 | 0 | 0 | 0 | 0 | 0 | 0 | 0 | 0 | 0 | 0 | 0 | | | | | | |
| 391 | 00414 | 00390 | 4 | 1 | 0 | 0 | 0 | 0 | 0 | 1 | 0 | 0 | 0 | 1 | 0 | 0 | 0 | 0 | 0 | 0 | 0 | 0 | 0 | 1 | 0 | 0 | 0 | 0 | 0 | | | | | | |
| 392 | 00415 | 00391 | 1 | 1 | 0 | 0 | 0 | 0 | 0 | 0 | 0 | 0 | 0 | 0 | 0 | 0 | 0 | 0 | 0 | 0 | 0 | 0 | 0 | 0 | 0 | 0 | 0 | 0 | 0 | | | | | | |
| 393 | 00416 | 00392 | 3 | 2 | 0 | 0 | 0 | 0 | 0 | 0 | 0 | 0 | 1 | 0 | 0 | 0 | 0 | 0 | 0 | 0 | 0 | 0 | 0 | 0 | 0 | 0 | 0 | 0 | 0 | | | | | | |
| 394 | 00417 | 00393 | 4 | 1 | 0 | 0 | 0 | 0 | 0 | 0 | 0 | 0 | 0 | 1 | 0 | 1 | 0 | 0 | 0 | 0 | 0 | 0 | 1 | 0 | 0 | 0 | 0 | 0 | 0 | D21260 | 97.2 | 109 | 1 | 6003 | 6111 |
| 395 | 00418 | 00394 | 116 | 5 | 22 | 1 | 13 | 1 | 5 | 1 | 0 | 10 | 10 | 0 | 0 | 0 | 9 | 0 | 1 | 6 | 0 | 0 | 1 | 2 | 1 | 7 | 1 | 16 | 4 | D14531 | 97.2 | 107 | 1 | 586 | 689 |
| 396 | 00419 | 00395 | 2 | 1 | 1 | 0 | 0 | 0 | 0 | 0 | 0 | 0 | 0 | 0 | 0 | 0 | 0 | 0 | 0 | 0 | 0 | 0 | 0 | 0 | 0 | 0 | 0 | 0 | 0 | | | | | | |
| 397 | 00420 | 00396 | 4 | 1 | 0 | 0 | 0 | 0 | 0 | 0 | 0 | 0 | 0 | 0 | 0 | 2 | 0 | 0 | 1 | 0 | 0 | 0 | 0 | 0 | 0 | 0 | 0 | 0 | 0 | | | | | | |
| 398 | 00421 | 00397 | 13 | 1 | 0 | 0 | 0 | 0 | 0 | 0 | 0 | 0 | 0 | 0 | 0 | 0 | 1 | 4 | 1 | 1 | 0 | 0 | 3 | 2 | 0 | 0 | 0 | 0 | 0 | D16111 | 100 | 101 | 1 | 1334 | 1434 |
| 399 | 00422 | 00398 | 16 | 1 | 3 | 0 | 0 | 1 | 0 | 0 | 0 | 0 | 1 | 0 | 2 | 1 | 2 | 1 | 1 | 0 | 0 | 1 | 1 | 0 | 1 | 0 | 0 | 0 | 0 | M10036 | 91.4 | 105 | 1 | 1462 | 1835 |
| 400 | 00423 | 00399 | 1 | 1 | 0 | 0 | 0 | 0 | 0 | 0 | 0 | 0 | 0 | 0 | 0 | 0 | 0 | 0 | 0 | 0 | 0 | 0 | 0 | 0 | 0 | 0 | 0 | 0 | 0 | | | | | | |
| 401 | 00424 | 00400 | 1 | 1 | 0 | 0 | 0 | 0 | 0 | 0 | 0 | 0 | 0 | 0 | 0 | 0 | 0 | 0 | 0 | 0 | 0 | 0 | 0 | 0 | 0 | 0 | 0 | 0 | 0 | | | | | | |
| 402 | 00425 | 00401 | 1 | 1 | 0 | 0 | 0 | 0 | 0 | 0 | 0 | 0 | 0 | 0 | 0 | 0 | 0 | 0 | 0 | 0 | 0 | 0 | 0 | 0 | 0 | 0 | 0 | 0 | 0 | | | | | | |
| 403 | 00426 | 00402 | 13 | 1 | 0 | 0 | 0 | 0 | 0 | 2 | 0 | 0 | 0 | 2 | 1 | 0 | 0 | 0 | 0 | 0 | 1 | 4 | 1 | 0 | 0 | 0 | 0 | 1 | 0 | | | | | | |
| 404 | 00427 | 00403 | 2 | 1 | 0 | 0 | 0 | 0 | 0 | 1 | 0 | 0 | 0 | 0 | 0 | 0 | 0 | 0 | 0 | 0 | 0 | 0 | 0 | 0 | 0 | 0 | 0 | 0 | 0 | | | | | | |
| 405 | 00428 | 00404 | 3 | 1 | 0 | 0 | 2 | 0 | 0 | 0 | 0 | 0 | 0 | 0 | 0 | 0 | 0 | 0 | 0 | 0 | 0 | 0 | 0 | 0 | 0 | 0 | 0 | 0 | 0 | X61970 | 99.4 | 157 | 1 | 756 | 959 |
| 406 | 00429 | 00405 | 1 | 1 | 0 | 0 | 0 | 0 | 0 | 0 | 0 | 0 | 0 | 0 | 0 | 0 | 0 | 0 | 0 | 0 | 0 | 0 | 0 | 0 | 0 | 0 | 0 | 0 | 0 | | | | | | |
| 407 | 00430 | 00406 | 3 | 2 | 0 | 0 | 0 | 0 | 0 | 0 | 0 | 0 | 0 | 0 | 0 | 0 | 0 | 0 | 0 | 0 | 0 | 0 | 1 | 0 | 0 | 0 | 0 | 0 | 0 | | | | | | |
| 408 | 00431 | 00407 | 4 | 1 | 0 | 0 | 0 | 0 | 0 | 0 | 0 | 0 | 0 | 0 | 0 | 0 | 0 | 0 | 1 | 2 | 0 | 0 | 0 | 0 | 0 | 0 | 0 | 0 | 0 | X75684 | 100 | 104 | 1 | 46 | 157 |
| 409 | 00433 | 00408 | 2 | 1 | 0 | 0 | 0 | 0 | 0 | 0 | 0 | 0 | 0 | 0 | 0 | 0 | 0 | 0 | 0 | 0 | 1 | 0 | 0 | 0 | 0 | 0 | 0 | 0 | 0 | | | | | | |
| 410 | 00434 | 00409 | 13 | 1 | 0 | 0 | 2 | 1 | 0 | 0 | 0 | 0 | 2 | 0 | 1 | 1 | 0 | 0 | 0 | 1 | 1 | 0 | 0 | 1 | 0 | 1 | 1 | 0 | 0 | | | | | | |
| 411 | 00435 | 00410 | 1 | 1 | 0 | 0 | 0 | 0 | 0 | 0 | 0 | 0 | 0 | 0 | 0 | 0 | 0 | 0 | 0 | 0 | 0 | 0 | 0 | 0 | 0 | 0 | 0 | 0 | 0 | | | | | | |
| 412 | 00436 | 00411 | 1 | 1 | 0 | 0 | 0 | 0 | 0 | 0 | 0 | 0 | 0 | 0 | 0 | 0 | 0 | 0 | 0 | 0 | 0 | 0 | 0 | 0 | 0 | 0 | 0 | 0 | 0 | | | | | | |
| 413 | 00437 | 00412 | 34 | 5 | 4 | 2 | 2 | 0 | 0 | 2 | 1 | 0 | 2 | 1 | 0 | 1 | 2 | 0 | 0 | 0 | 0 | 0 | 1 | 0 | 3 | 5 | 0 | 3 | 0 | X57959 | 98 | 101 | 1 | 727 | 827 |
| 414 | 00438 | 00413 | 3 | 1 | 0 | 0 | 0 | 0 | 0 | 0 | 0 | 0 | 0 | 0 | 0 | 0 | 0 | 0 | 0 | 0 | 0 | 0 | 0 | 0 | 0 | 0 | 0 | 2 | 0 | | | | | | |
| 415 | 00439 | 00414 | 9 | 1 | 1 | 0 | 3 | 0 | 0 | 0 | 0 | 0 | 0 | 0 | 0 | 0 | 2 | 0 | 0 | 1 | 0 | 0 | 0 | 1 | 0 | 0 | 0 | 0 | 0 | | | | | | |
| 416 | 00440 | 00415 | 1 | 1 | 0 | 0 | 0 | 0 | 0 | 0 | 0 | 0 | 0 | 0 | 0 | 0 | 0 | 0 | 0 | 0 | 0 | 0 | 0 | 0 | 0 | 0 | 0 | 0 | 0 | | | | | | |
| 417 | 00441 | 00416 | 4 | 2 | 0 | 0 | 1 | 0 | 0 | 0 | 0 | 0 | 0 | 0 | 0 | 0 | 0 | 0 | 0 | 0 | 0 | 0 | 0 | 1 | 0 | 0 | 0 | 0 | 0 | | | | | | |

Table 12

| | A | B | C | E | G | I | K | M | O | Q | S | U | W | Y | AA | AC | AE | AG | AI | AK | AM | AO | AQ | AS | AU | AW | AY | BA | BC | BE | BF | BG | BH | BI | BK |
|---|---|---|---|---|---|---|---|---|---|---|---|---|---|---|---|---|---|---|---|---|---|---|---|---|---|---|---|---|---|---|---|---|---|---|---|
| 418 | 00442 | 00417 | 1 | 1 | 0 | 0 | 0 | 0 | 0 | 0 | 0 | 0 | 0 | 0 | 0 | 0 | 0 | 0 | 0 | 0 | 0 | 0 | 0 | 0 | 0 | 0 | 0 | 0 | 0 | | | | | | |
| 419 | 00443 | 00418 | 2 | 1 | 0 | 0 | 0 | 0 | 0 | 0 | 0 | 0 | 0 | 0 | 0 | 0 | 0 | 0 | 0 | 0 | 0 | 0 | 0 | 0 | 0 | 1 | 0 | 0 | 0 | | | | | | |
| 420 | 00444 | 00419 | 71 | 6 | 10 | 1 | 4 | 2 | 4 | 1 | 1 | 0 | 3 | 1 | 0 | 5 | 0 | 0 | 1 | 4 | 8 | 5 | 5 | 0 | 3 | 5 | 0 | 2 | 0 | M81757 | 98.9 | 90 | 1 | 410 | 510 |
| 421 | 00445 | 00420 | 3 | 2 | 0 | 0 | 0 | 0 | 0 | 0 | 0 | 0 | 0 | 0 | 1 | 0 | 0 | 0 | 0 | 0 | 0 | 0 | 0 | 0 | 0 | 0 | 0 | 0 | 0 | | | | | | |
| 422 | 00446 | 00421 | 4 | 2 | 0 | 0 | 0 | 2 | 0 | 0 | 0 | 0 | 0 | 0 | 0 | 0 | 0 | 0 | 0 | 0 | 0 | 0 | 0 | 0 | 0 | 0 | 0 | 0 | 0 | | | | | | |
| 423 | 00447 | 00422 | 38 | 2 | 6 | 1 | 1 | 0 | 3 | 2 | 0 | 2 | 6 | 2 | 0 | 1 | 0 | 0 | 3 | 1 | 1 | 1 | 0 | 1 | 1 | 1 | 0 | 3 | 0 | X69391 | 96.5 | 86 | 1 | 841 | 926 |
| 424 | 00448 | 00423 | 17 | 1 | 0 | 1 | 1 | 0 | 0 | 0 | 0 | 1 | 2 | 0 | 1 | 0 | 1 | 0 | 1 | 0 | 2 | 0 | 1 | 2 | 0 | 1 | 0 | 1 | 1 | | | | | | |
| 425 | 00450 | 00424 | 30 | 3 | 2 | 2 | 2 | 0 | 0 | 0 | 0 | 0 | 0 | 0 | 0 | 2 | 1 | 0 | 2 | 0 | 3 | 1 | 10 | 0 | 2 | 0 | 0 | 0 | 0 | | | | | | |
| 426 | 00451 | 00425 | 1 | 1 | 0 | 0 | 0 | 0 | 0 | 0 | 0 | 0 | 0 | 0 | 0 | 0 | 0 | 0 | 0 | 0 | 0 | 0 | 0 | 0 | 0 | 0 | 0 | 0 | 0 | | | | | | |
| 427 | 00453 | 00426 | 4 | 1 | 0 | 0 | 0 | 0 | 0 | 0 | 0 | 1 | 1 | 0 | 0 | 0 | 0 | 0 | 0 | 0 | 0 | 0 | 0 | 0 | 0 | 0 | 0 | 1 | 0 | | | | | | |
| 428 | 00454 | 00427 | 7 | 1 | 0 | 0 | 1 | 0 | 0 | 0 | 0 | 1 | 1 | 2 | 0 | 0 | 0 | 0 | 0 | 0 | 0 | 0 | 0 | 1 | 0 | 0 | 0 | 0 | 0 | | | | | | |
| 429 | 00455 | 00428 | 10 | 2 | 0 | 1 | 0 | 0 | 0 | 1 | 0 | 0 | 1 | 0 | 0 | 0 | 0 | 0 | 1 | 0 | 0 | 0 | 0 | 1 | 0 | 1 | 0 | 2 | 0 | | | | | | |
| 430 | 00456 | 00429 | 18 | 1 | 0 | 0 | 0 | 0 | 0 | 0 | 0 | 0 | 0 | 1 | 0 | 1 | 0 | 0 | 0 | 2 | 0 | 1 | 0 | 1 | 2 | 1 | 0 | 8 | 0 | | | | | | |
| 431 | 00457 | 00430 | 3 | 1 | 0 | 0 | 0 | 0 | 0 | 0 | 0 | 0 | 0 | 1 | 0 | 0 | 0 | 0 | 0 | 0 | 0 | 0 | 0 | 0 | 0 | 1 | 0 | 0 | 0 | | | | | | |
| 432 | 00458 | 00431 | 1 | 1 | 0 | 0 | 0 | 0 | 0 | 0 | 0 | 0 | 0 | 0 | 0 | 0 | 0 | 0 | 0 | 0 | 0 | 0 | 0 | 0 | 0 | 0 | 0 | 0 | 0 | | | | | | |
| 433 | 00459 | 00432 | 8 | 1 | 0 | 0 | 0 | 0 | 0 | 0 | 1 | 0 | 1 | 0 | 0 | 0 | 0 | 0 | 1 | 0 | 0 | 2 | 0 | 0 | 1 | 1 | 0 | 0 | 0 | | | | | | |
| 434 | 00460 | 00433 | 1 | 1 | 0 | 0 | 0 | 0 | 0 | 0 | 0 | 0 | 0 | 0 | 0 | 0 | 0 | 0 | 0 | 0 | 0 | 0 | 0 | 0 | 0 | 0 | 0 | 0 | 0 | L07493 | 97.5 | 79 | 1 | 395 | 692 |
| 435 | 00461 | 00434 | 3 | 1 | 0 | 0 | 0 | 2 | 0 | 0 | 0 | 0 | 0 | 0 | 0 | 0 | 0 | 0 | 0 | 0 | 0 | 0 | 0 | 0 | 0 | 0 | 0 | 0 | 0 | | | | | | |
| 436 | 00462 | 00435 | 2 | 1 | 0 | 0 | 0 | 0 | 0 | 0 | 0 | 0 | 0 | 0 | 0 | 0 | 0 | 0 | 0 | 0 | 0 | 0 | 0 | 0 | 0 | 0 | 0 | 1 | 0 | | | | | | |
| 437 | 00463 | 00436 | 26 | 2 | 2 | 0 | 1 | 1 | 0 | 0 | 0 | 0 | 0 | 1 | 0 | 0 | 2 | 0 | 2 | 2 | 2 | 3 | 1 | 1 | 2 | 4 | 0 | 0 | 0 | X02152 | 94.7 | 76 | 1 | 1587 | 1661 |
| 438 | 00464 | 00437 | 18 | 1 | 1 | 2 | 0 | 0 | 1 | 0 | 0 | 1 | 3 | 0 | 1 | 1 | 0 | 0 | 0 | 2 | 2 | 1 | 0 | 1 | 1 | 0 | 0 | 0 | 0 | D13630 | 98.6 | 74 | 1 | 2925 | 2998 |
| 439 | 00465 | 00438 | 2 | 1 | 0 | 0 | 0 | 0 | 0 | 0 | 0 | 0 | 0 | 0 | 0 | 0 | 0 | 0 | 0 | 1 | 0 | 0 | 0 | 0 | 0 | 0 | 0 | 0 | 0 | X17644 | 96 | 75 | 1 | 2513 | 2587 |
| 440 | 00466 | 00439 | 1 | 1 | 0 | 0 | 0 | 0 | 0 | 0 | 0 | 0 | 0 | 0 | 0 | 0 | 0 | 0 | 0 | 0 | 0 | 0 | 0 | 0 | 0 | 0 | 0 | 0 | 0 | | | | | | |
| 441 | 00467 | 00440 | 4 | 1 | 0 | 0 | 0 | 0 | 0 | 1 | 1 | 0 | 1 | 0 | 0 | 0 | 0 | 0 | 0 | 0 | 0 | 0 | 0 | 0 | 0 | 0 | 0 | 0 | 0 | | | | | | |
| 442 | 00468 | 00441 | 1 | 1 | 0 | 0 | 0 | 0 | 0 | 0 | 0 | 0 | 0 | 0 | 0 | 0 | 0 | 0 | 0 | 0 | 0 | 0 | 0 | 0 | 0 | 0 | 0 | 0 | 0 | | | | | | |
| 443 | 00469 | 00442 | 6 | 1 | 1 | 1 | 0 | 0 | 0 | 0 | 0 | 0 | 0 | 0 | 0 | 0 | 0 | 2 | 0 | 1 | 0 | 0 | 0 | 0 | 0 | 0 | 0 | 0 | 0 | | | | | | |
| 444 | 00470 | 00443 | 3 | 1 | 0 | 0 | 0 | 0 | 0 | 0 | 0 | 0 | 0 | 0 | 0 | 0 | 2 | 0 | 0 | 0 | 0 | 0 | 0 | 0 | 0 | 0 | 0 | 0 | 0 | | | | | | |
| 445 | 00471 | 00444 | 8 | 1 | 0 | 1 | 0 | 0 | 1 | 1 | 0 | 0 | 0 | 0 | 0 | 1 | 0 | 0 | 1 | 0 | 0 | 0 | 0 | 0 | 1 | 0 | 1 | 0 | 0 | | | | | | |
| 446 | 00473 | 00445 | 10 | 1 | 0 | 1 | 0 | 0 | 0 | 1 | 1 | 0 | 0 | 0 | 0 | 0 | 0 | 0 | 1 | 5 | 0 | 0 | 0 | 0 | 0 | 0 | 0 | 0 | 0 | | | | | | |
| 447 | 00474 | 00446 | 3 | 1 | 0 | 0 | 0 | 0 | 0 | 0 | 0 | 0 | 0 | 0 | 0 | 0 | 0 | 0 | 0 | 0 | 0 | 0 | 0 | 0 | 0 | 0 | 2 | 0 | 0 | X16354 | 100 | 71 | 1 | 3387 | 3464 |
| 448 | 00475 | 00447 | 2 | 2 | 0 | 0 | 0 | 0 | 0 | 0 | 0 | 0 | 0 | 0 | 0 | 0 | 0 | 0 | 0 | 0 | 0 | 0 | 0 | 0 | 0 | 0 | 0 | 0 | 0 | | | | | | |
| 449 | 00477 | 00448 | 1 | 1 | 0 | 0 | 0 | 0 | 0 | 0 | 0 | 0 | 0 | 0 | 0 | 0 | 0 | 0 | 0 | 0 | 0 | 0 | 0 | 0 | 0 | 0 | 0 | 0 | 0 | | | | | | |
| 450 | 00478 | 00449 | 3 | 1 | 0 | 0 | 0 | 0 | 0 | 0 | 0 | 0 | 0 | 0 | 0 | 0 | 0 | 1 | 0 | 0 | 0 | 0 | 0 | 0 | 0 | 0 | 0 | 1 | 0 | | | | | | |
| 451 | 00479 | 00450 | 2 | 1 | 0 | 0 | 0 | 0 | 0 | 0 | 0 | 0 | 0 | 0 | 0 | 0 | 0 | 0 | 0 | 1 | 0 | 0 | 0 | 0 | 0 | 0 | 0 | 0 | 0 | | | | | | |
| 452 | 00480 | 00451 | 1 | 1 | 0 | 0 | 0 | 0 | 0 | 0 | 0 | 0 | 0 | 0 | 0 | 0 | 0 | 0 | 0 | 0 | 0 | 0 | 0 | 0 | 0 | 0 | 0 | 0 | 0 | | | | | | |
| 453 | 00481 | 00452 | 24 | 1 | 1 | 0 | 0 | 1 | 0 | 3 | 0 | 0 | 2 | 0 | 0 | 1 | 3 | 0 | 0 | 3 | 0 | 1 | 3 | 2 | 0 | 1 | 0 | 2 | 0 | | | | | | |

Table 13

| | A | B | C | E | G | I | K | M | O | Q | S | U | W | Y | AA | AC | AE | AG | AI | AK | AM | AO | AQ | AS | AU | AW | AY | BA | BC | BE | BF | BG | BH | BI | BK |
|---|---|---|---|---|---|---|---|---|---|---|---|---|---|---|---|---|---|---|---|---|---|---|---|---|---|---|---|---|---|---|---|---|---|---|---|
| 454 | 00482 | 00453 | 7 | 2 | 0 | 0 | 0 | 0 | 0 | 0 | 0 | 0 | 0 | 0 | 0 | 0 | 0 | 0 | 1 | 2 | 1 | 0 | 0 | 0 | 0 | 0 | 0 | 1 | 0 | | | | | | |
| 455 | 00483 | 00454 | 5 | 1 | 0 | 0 | 0 | 0 | 0 | 0 | 0 | 0 | 1 | 0 | 0 | 0 | 0 | 0 | 0 | 1 | 0 | 0 | 0 | 0 | 0 | 0 | 2 | 0 | 0 | J05272 | 100 | 51 | 1 | 2808 | 2858 |
| 456 | 00484 | 00455 | 2 | 1 | 0 | 0 | 0 | 0 | 0 | 0 | 0 | 0 | 0 | 1 | 0 | 0 | 0 | 0 | 0 | 0 | 0 | 0 | 0 | 0 | 0 | 0 | 0 | 0 | 0 | | | | | | |
| 457 | 00485 | 00456 | 1 | 1 | 0 | 0 | 0 | 0 | 0 | 0 | 0 | 0 | 0 | 0 | 0 | 0 | 0 | 0 | 0 | 0 | 0 | 0 | 0 | 0 | 0 | 0 | 0 | 0 | 0 | | | | | | |
| 458 | 00486 | 00457 | 6 | 6 | 0 | 0 | 0 | 0 | 0 | 0 | 0 | 0 | 0 | 0 | 0 | 0 | 0 | 0 | 0 | 0 | 0 | 0 | 0 | 0 | 0 | 0 | 0 | 0 | 0 | | | | | | |
| 459 | 00487 | 00458 | 11 | 1 | 0 | 0 | 0 | 0 | 0 | 0 | 0 | 0 | 0 | 0 | 1 | 0 | 0 | 0 | 3 | 0 | 0 | 3 | 0 | 1 | 0 | 0 | 1 | 1 | 0 | | | | | | |
| 460 | 00488 | 00459 | 1 | 1 | 0 | 0 | 0 | 0 | 0 | 0 | 0 | 0 | 0 | 0 | 0 | 0 | 0 | 0 | 0 | 0 | 0 | 0 | 0 | 0 | 0 | 0 | 0 | 0 | 0 | U02493 | 90.2 | 61 | 1 | 1905 | 2593 |
| 461 | 00489 | 00460 | 2 | 1 | 0 | 0 | 0 | 0 | 0 | 0 | 0 | 0 | 0 | 0 | 0 | 0 | 0 | 0 | 0 | 0 | 0 | 0 | 1 | 0 | 0 | 0 | 0 | 0 | 0 | | | | | | |
| 462 | 00491 | 00461 | 1 | 1 | 0 | 0 | 0 | 0 | 0 | 0 | 0 | 0 | 0 | 0 | 0 | 0 | 0 | 0 | 0 | 0 | 0 | 0 | 0 | 0 | 0 | 0 | 0 | 0 | 0 | | | | | | |
| 463 | 00493 | 00462 | 4 | 1 | 0 | 0 | 0 | 1 | 0 | 0 | 0 | 0 | 0 | 0 | 0 | 0 | 1 | 0 | 0 | 0 | 1 | 0 | 0 | 0 | 0 | 0 | 0 | 0 | 0 | | | | | | |
| 464 | 00494 | 00463 | 4 | 1 | 0 | 0 | 0 | 0 | 0 | 0 | 0 | 0 | 0 | 0 | 0 | 0 | 1 | 0 | 0 | 1 | 0 | 0 | 0 | 0 | 0 | 0 | 0 | 1 | 0 | | | | | | |
| 465 | 00495 | 00464 | 12 | 1 | 2 | 1 | 0 | 0 | 1 | 1 | 0 | 1 | 1 | 0 | 0 | 0 | 0 | 0 | 0 | 1 | 0 | 0 | 0 | 0 | 0 | 2 | 0 | 1 | 0 | | | | | | |
| 466 | 00496 | 00465 | 1 | 1 | 0 | 0 | 0 | 0 | 0 | 0 | 0 | 0 | 0 | 0 | 0 | 0 | 0 | 0 | 0 | 0 | 0 | 0 | 0 | 0 | 0 | 0 | 0 | 0 | 0 | | | | | | |
| 467 | 00497 | 00466 | 11 | 1 | 1 | 1 | 3 | 1 | 0 | 0 | 0 | 0 | 1 | 0 | 0 | 1 | 0 | 0 | 0 | 0 | 0 | 1 | 0 | 0 | 0 | 0 | 0 | 1 | 0 | | | | | | |
| 468 | 00498 | 00467 | 1 | 1 | 0 | 0 | 0 | 0 | 0 | 0 | 0 | 0 | 0 | 0 | 0 | 0 | 0 | 0 | 0 | 0 | 0 | 0 | 0 | 0 | 0 | 0 | 0 | 0 | 0 | X60036 | 94 | 50 | 5 | 1266 | 1330 |
| 469 | 00500 | 00468 | 54 | 6 | 4 | 1 | 1 | 0 | 2 | 2 | 2 | 3 | 3 | 2 | 3 | 1 | 1 | 0 | 3 | 8 | 1 | 2 | 1 | 1 | 1 | 1 | 0 | 4 | 1 | D14530 | 94.1 | 51 | 1 | 438 | 488 |
| 470 | 00503 | 00469 | 8 | 1 | 2 | 0 | 0 | 0 | 0 | 0 | 1 | 0 | 1 | 0 | 1 | 1 | 0 | 0 | 0 | 0 | 0 | 0 | 0 | 0 | 0 | 0 | 0 | 1 | 0 | X57346 | 98.1 | 52 | 1 | 1131 | 1213 |
| 471 | 00504 | 00470 | 56 | 1 | 5 | 1 | 0 | 0 | 1 | 1 | 1 | 0 | 2 | 2 | 2 | 3 | 4 | 2 | 1 | 6 | 2 | 3 | 3 | 2 | 0 | 13 | 0 | 1 | 0 | M14328 | 98 | 51 | 1 | 1706 | 1755 |
| 472 | 00505 | 00471 | 7 | 2 | 1 | 0 | 0 | 0 | 0 | 0 | 0 | 0 | 2 | 0 | 0 | 0 | 0 | 0 | 1 | 0 | 1 | 0 | 0 | 0 | 0 | 0 | 0 | 0 | 0 | L16785 | 90 | 50 | 1 | 599 | 647 |
| 473 | 00506 | 00472 | 3 | 1 | 0 | 0 | 0 | 0 | 0 | 0 | 0 | 0 | 0 | 0 | 0 | 0 | 0 | 0 | 0 | 0 | 1 | 0 | 0 | 0 | 0 | 1 | 0 | 0 | 0 | | | | | | |
| 474 | 00507 | 00473 | 9 | 1 | 1 | 0 | 0 | 0 | 0 | 0 | 0 | 1 | 0 | 0 | 0 | 0 | 0 | 0 | 2 | 0 | 0 | 0 | 0 | 2 | 1 | 1 | 0 | 0 | 0 | | | | | | |
| 475 | 00510 | 00474 | 2 | 1 | 0 | 0 | 0 | 0 | 0 | 0 | 0 | 0 | 1 | 0 | 0 | 0 | 0 | 0 | 0 | 0 | 0 | 0 | 0 | 0 | 0 | 0 | 0 | 0 | 0 | | | | | | |
| 476 | 00511 | 00475 | 7 | 1 | 2 | 0 | 0 | 0 | 0 | 0 | 0 | 0 | 0 | 0 | 0 | 0 | 0 | 1 | 0 | 0 | 2 | 1 | 0 | 0 | 0 | 0 | 0 | 0 | 0 | | | | | | |
| 477 | 00532 | 00476 | 2 | | 1 | 0 | 0 | 0 | 0 | 0 | 0 | 0 | 0 | 1 | 0 | 0 | 0 | 0 | 0 | 0 | 0 | 0 | 0 | 0 | 0 | 0 | 0 | 0 | 0 | | | | | | |
| 478 | 00533 | 00477 | 44 | | 1 | 0 | 0 | 0 | 0 | 0 | 0 | 2 | 0 | 4 | 0 | 1 | 1 | 2 | 4 | 4 | 3 | 9 | 2 | 3 | 0 | 6 | 1 | 1 | 0 | M97164 | 92.1 | 644 | 1 | 201 | 1119 |
| 479 | 00534 | 00478 | 3 | | 1 | 0 | 0 | 0 | 0 | 0 | 0 | 0 | 0 | 0 | 0 | 0 | 0 | 0 | 0 | 1 | 0 | 0 | 0 | 0 | 1 | 0 | 0 | 0 | 0 | D21262 | 93.5 | 321 | 1 | 3091 | 3727 |
| 480 | 00535 | 00479 | 2 | | 1 | 0 | 0 | 0 | 0 | 0 | 0 | 0 | 0 | 0 | 1 | 0 | 0 | 0 | 0 | 0 | 0 | 0 | 0 | 0 | 0 | 0 | 0 | 0 | 0 | | | | | | |
| 481 | 00536 | 00480 | 4 | | 1 | 0 | 0 | 0 | 0 | 0 | 0 | 1 | 0 | 2 | 0 | 0 | 0 | 0 | 0 | 0 | 0 | 0 | 0 | 0 | 0 | 0 | 0 | 0 | 0 | | | | | | |
| 482 | 00537 | 00481 | 1 | | 1 | 0 | 0 | 0 | 0 | 0 | 0 | 0 | 0 | 0 | 0 | 0 | 0 | 0 | 0 | 0 | 0 | 0 | 0 | 0 | 0 | 0 | 0 | 0 | 0 | | | | | | |
| 483 | 00538 | 00482 | 1 | | 1 | 0 | 0 | 0 | 0 | 0 | 0 | 0 | 0 | 0 | 0 | 0 | 0 | 0 | 0 | 0 | 0 | 0 | 0 | 0 | 0 | 0 | 0 | 0 | 0 | | | | | | |
| 484 | 00539 | 00483 | 3 | | 1 | 0 | 0 | 0 | 0 | 0 | 0 | 0 | 0 | 0 | 0 | 1 | 0 | 1 | 0 | 0 | 0 | 0 | 0 | 0 | 0 | 0 | 0 | 0 | 0 | | | | | | |
| 485 | 00540 | 00484 | 4 | | 1 | 0 | 0 | 0 | 0 | 0 | 0 | 0 | 0 | 0 | 0 | 0 | 0 | 0 | 1 | 0 | 0 | 0 | 0 | 0 | 0 | 0 | 1 | 0 | 1 | | | | | | |
| 486 | 00541 | 00485 | 1 | | 1 | 0 | 0 | 0 | 0 | 0 | 0 | 0 | 0 | 0 | 0 | 0 | 0 | 0 | 0 | 0 | 0 | 0 | 0 | 0 | 0 | 0 | 0 | 0 | 0 | | | | | | |
| 487 | 00542 | 00486 | 2 | | 1 | 1 | 0 | 0 | 0 | 0 | 0 | 0 | 0 | 0 | 0 | 0 | 0 | 0 | 0 | 0 | 0 | 0 | 0 | 0 | 0 | 0 | 0 | 0 | 0 | D25304 | 94.8 | 503 | 1 | 4312 | 4804 |
| 488 | 00543 | 00487 | 65 | | 9 | 4 | 5 | 0 | 2 | 2 | 0 | 2 | 11 | 0 | 2 | 7 | 4 | 0 | 0 | 1 | 3 | 1 | 0 | 1 | 2 | 2 | 1 | 5 | 1 | X56932 | 98.8 | 161 | 1 | 512 | 672 |
| 489 | 00544 | 00488 | 5 | | 2 | 0 | 0 | 0 | 0 | 0 | 0 | 0 | 0 | 0 | 0 | 0 | 0 | 0 | 2 | 0 | 0 | 0 | 0 | 1 | 0 | 0 | 0 | 0 | 0 | | | | | | |

Table 14

| | A | B | C | E | G | I | K | M | O | Q | S | U | W | Y | AA | AC | AE | AG | AI | AK | AM | AO | AQ | AS | AU | AW | AY | BA | BC | BE | BF | BG | BH | BI | BK |
|---|---|---|---|---|---|---|---|---|---|---|---|---|---|---|---|---|---|---|---|---|---|---|---|---|---|---|---|---|---|---|---|---|---|---|---|
| 490 | 00545 | 00489 | 36 | | 3 | 0 | 2 | 0 | 0 | 2 | 1 | 2 | 3 | 0 | 1 | 3 | 1 | 1 | 0 | 2 | 1 | 1 | 0 | 1 | 1 | 3 | 0 | 7 | 1 | M94314 | 93.4 | 439 | 1 | 75 | 556 |
| 491 | 00546 | 00490 | 1 | | 1 | 0 | 0 | 0 | 0 | 0 | 0 | 0 | 0 | 0 | 0 | 0 | 0 | 0 | 0 | 0 | 0 | 0 | 0 | 0 | 0 | 0 | 0 | 0 | 0 | | | | | | |
| 492 | 00547 | 00491 | 3 | | 1 | 0 | 0 | 0 | 0 | 0 | 0 | 0 | 0 | 0 | 0 | 0 | 0 | 0 | 0 | 0 | 0 | 0 | 1 | 0 | 0 | 0 | 0 | 1 | 0 | | | | | | |
| 493 | 00548 | 00492 | 1 | | 1 | 0 | 0 | 0 | 0 | 0 | 0 | 0 | 0 | 0 | 0 | 0 | 0 | 0 | 0 | 0 | 0 | 0 | 0 | 0 | 0 | 0 | 0 | 0 | 0 | M64716 | 94.4 | 478 | 2 | 24 | 497 |
| 494 | 00549 | 00493 | 2 | | 1 | 0 | 1 | 0 | 0 | 0 | 0 | 0 | 0 | 0 | 0 | 0 | 0 | 0 | 0 | 0 | 0 | 0 | 0 | 0 | 0 | 0 | 0 | 0 | 0 | | | | | | |
| 495 | 00551 | 00494 | 1 | | 1 | 0 | 0 | 0 | 0 | 0 | 0 | 0 | 0 | 0 | 0 | 0 | 0 | 0 | 0 | 0 | 0 | 0 | 0 | 0 | 0 | 0 | 0 | 0 | 0 | | | | | | |
| 496 | 00552 | 00495 | 4 | | 1 | 1 | 1 | 0 | 0 | 0 | 0 | 1 | 0 | 0 | 0 | 0 | 0 | 0 | 0 | 0 | 0 | 0 | 0 | 0 | 0 | 0 | 0 | 0 | 0 | | | | | | |
| 497 | 00553 | 00496 | 1 | | 1 | 0 | 0 | 0 | 0 | 0 | 0 | 0 | 0 | 0 | 0 | 0 | 0 | 0 | 0 | 0 | 0 | 0 | 0 | 0 | 0 | 0 | 0 | 0 | 0 | | | | | | |
| 498 | 00554 | 00497 | 1 | | 1 | 0 | 0 | 0 | 0 | 0 | 0 | 0 | 0 | 0 | 0 | 0 | 0 | 0 | 0 | 0 | 0 | 0 | 0 | 0 | 0 | 0 | 0 | 0 | 0 | | | | | | |
| 499 | 00555 | 00498 | 4 | | 1 | 0 | 0 | 0 | 0 | 0 | 0 | 0 | 0 | 0 | 1 | 0 | 0 | 0 | 0 | 0 | 2 | 0 | 0 | 0 | 0 | 0 | 0 | 0 | 0 | D14662 | 97.5 | 481 | 1 | 395 | 1653 |
| 500 | 00556 | 00499 | 2 | | 1 | 0 | 0 | 0 | 0 | 0 | 0 | 0 | 0 | 0 | 0 | 0 | 0 | 0 | 0 | 0 | 0 | 0 | 0 | 0 | 0 | 0 | 0 | 1 | 0 | | | | | | |
| 501 | 00557 | 00500 | 2 | | 1 | 0 | 0 | 0 | 0 | 0 | 0 | 0 | 0 | 0 | 0 | 0 | 0 | 0 | 0 | 0 | 1 | 0 | 0 | 0 | 0 | 0 | 0 | 0 | 0 | | | | | | |
| 502 | 00558 | 00501 | 1 | | 1 | 0 | 0 | 0 | 0 | 0 | 0 | 0 | 0 | 0 | 0 | 0 | 0 | 0 | 0 | 0 | 0 | 0 | 0 | 0 | 0 | 0 | 0 | 0 | 0 | | | | | | |
| 503 | 00560 | 00502 | 1 | | 1 | 0 | 0 | 0 | 0 | 0 | 0 | 0 | 0 | 0 | 0 | 0 | 0 | 0 | 0 | 0 | 0 | 0 | 0 | 0 | 0 | 0 | 0 | 0 | 0 | | | | | | |
| 504 | 00561 | 00503 | 1 | | 1 | 0 | 0 | 0 | 0 | 0 | 0 | 0 | 0 | 0 | 0 | 0 | 0 | 0 | 0 | 0 | 0 | 0 | 0 | 0 | 0 | 0 | 0 | 0 | 0 | | | | | | |
| 505 | 00562 | 00504 | 1 | | 1 | 0 | 0 | 0 | 0 | 0 | 0 | 0 | 0 | 0 | 0 | 0 | 0 | 0 | 0 | 0 | 0 | 0 | 0 | 0 | 0 | 0 | 0 | 0 | 0 | | | | | | |
| 506 | 00564 | 00505 | 2 | | 1 | 0 | 0 | 0 | 0 | 0 | 0 | 0 | 0 | 0 | 0 | 1 | 0 | 0 | 0 | 0 | 0 | 0 | 0 | 0 | 0 | 0 | 0 | 0 | 0 | | | | | | |
| 507 | 00565 | 00506 | 70 | | 4 | 2 | 5 | 0 | 1 | 1 | 1 | 1 | 1 | 4 | 5 | 1 | 1 | 0 | 4 | 6 | 2 | 1 | 1 | 2 | 0 | 0 | 25 | 0 | 2 | M30684 | 96.4 | 415 | 1 | 519 | 929 |
| 508 | 00567 | 00507 | 1 | | 1 | 0 | 0 | 0 | 0 | 0 | 0 | 0 | 0 | 0 | 0 | 0 | 0 | 0 | 0 | 0 | 0 | 0 | 0 | 0 | 0 | 0 | 0 | 0 | 0 | L06432 | 95.9 | 98 | 162 | 332 | 549 |
| 509 | 00568 | 00508 | 3 | | 1 | 0 | 0 | 0 | 0 | 0 | 1 | 0 | 0 | 1 | 0 | 0 | 0 | 0 | 0 | 0 | 0 | 0 | 0 | 0 | 0 | 0 | 0 | 0 | 0 | | | | | | |
| 510 | 00569 | 00509 | 10 | | 1 | 0 | 1 | 0 | 0 | 0 | 1 | 0 | 0 | 1 | 1 | 0 | 0 | 0 | 1 | 1 | 0 | 0 | 0 | 0 | 0 | 2 | 0 | 1 | 0 | | | | | | |
| 511 | 00571 | 00510 | 1 | | 1 | 0 | 0 | 0 | 0 | 0 | 0 | 0 | 0 | 0 | 0 | 0 | 0 | 0 | 0 | 0 | 0 | 0 | 0 | 0 | 0 | 0 | 0 | 0 | 0 | | | | | | |
| 512 | 00572 | 00511 | 2 | | 1 | 0 | 0 | 0 | 0 | 0 | 0 | 0 | 0 | 0 | 0 | 0 | 0 | 0 | 0 | 0 | 0 | 0 | 0 | 0 | 0 | 0 | 0 | 1 | 0 | | | | | | |
| 513 | 00573 | 00512 | 2 | | 1 | 0 | 0 | 0 | 0 | 0 | 0 | 0 | 0 | 0 | 1 | 0 | 0 | 0 | 0 | 0 | 0 | 0 | 0 | 0 | 0 | 0 | 0 | 0 | 0 | | | | | | |
| 514 | 00574 | 00513 | 1 | | 1 | 0 | 0 | 0 | 0 | 0 | 0 | 0 | 0 | 0 | 0 | 0 | 0 | 0 | 0 | 0 | 0 | 0 | 0 | 0 | 0 | 0 | 0 | 0 | 0 | | | | | | |
| 515 | 00575 | 00514 | 2 | | 1 | 0 | 0 | 0 | 0 | 0 | 1 | 0 | 0 | 0 | 0 | 0 | 0 | 0 | 0 | 0 | 0 | 0 | 0 | 0 | 0 | 0 | 0 | 0 | 0 | J04564 | 93.5 | 370 | 4 | 736 | 1099 |
| 516 | 00576 | 00515 | 3 | | 1 | 0 | 0 | 0 | 0 | 0 | 0 | 0 | 2 | 0 | 0 | 0 | 0 | 0 | 0 | 0 | 0 | 0 | 0 | 0 | 0 | 0 | 0 | 0 | 0 | | | | | | |
| 517 | 00577 | 00516 | 1 | | 1 | 0 | 0 | 0 | 0 | 0 | 0 | 0 | 0 | 0 | 0 | 0 | 0 | 0 | 0 | 0 | 0 | 0 | 0 | 0 | 0 | 0 | 0 | 0 | 0 | | | | | | |
| 518 | 00578 | 00517 | 2 | | 1 | 0 | 0 | 0 | 0 | 0 | 0 | 0 | 0 | 0 | 0 | 0 | 0 | 0 | 0 | 0 | 0 | 0 | 0 | 1 | 0 | 0 | 0 | 0 | 0 | | | | | | |
| 519 | 00579 | 00518 | 3 | | 1 | 0 | 0 | 0 | 0 | 0 | 0 | 0 | 0 | 0 | 0 | 0 | 0 | 0 | 0 | 1 | 0 | 0 | 0 | 0 | 0 | 0 | 0 | 1 | 0 | X52317 | 96.5 | 342 | 1 | 40 | 869 |
| 520 | 00580 | 00519 | 2 | | 1 | 0 | 0 | 0 | 0 | 0 | 0 | 0 | 1 | 0 | 0 | 0 | 0 | 0 | 0 | 0 | 0 | 0 | 0 | 0 | 0 | 0 | 0 | 0 | 0 | | | | | | |
| 521 | 00581 | 00520 | 1 | | 1 | 0 | 0 | 0 | 0 | 0 | 0 | 0 | 0 | 0 | 0 | 0 | 0 | 0 | 0 | 0 | 0 | 0 | 0 | 0 | 0 | 0 | 0 | 0 | 0 | | | | | | |
| 522 | 00582 | 00521 | 2 | | 1 | 0 | 0 | 0 | 0 | 0 | 0 | 0 | 1 | 0 | 0 | 0 | 0 | 0 | 0 | 0 | 0 | 0 | 0 | 0 | 0 | 0 | 0 | 0 | 0 | | | | | | |
| 523 | 00583 | 00522 | 34 | | 2 | 5 | 3 | 0 | 0 | 1 | 3 | 1 | 3 | 0 | 2 | 1 | 4 | 0 | 0 | 2 | 1 | 2 | 0 | 1 | 2 | 0 | 0 | 1 | 0 | X73460 | 95.8 | 378 | 1 | 900 | 1272 |
| 524 | 00584 | 00523 | 1 | | 1 | 0 | 0 | 0 | 0 | 0 | 0 | 0 | 0 | 0 | 0 | 0 | 0 | 0 | 0 | 0 | 0 | 0 | 0 | 0 | 0 | 0 | 0 | 0 | 0 | | | | | | |
| 525 | 00585 | 00524 | 1 | | 1 | 0 | 0 | 0 | 0 | 0 | 0 | 0 | 0 | 0 | 0 | 0 | 0 | 0 | 0 | 0 | 0 | 0 | 0 | 0 | 0 | 0 | 0 | 0 | 0 | | | | | | |

Table 15

| | A | B | C | E | G | I | K | M | O | Q | S | U | W | Y | AA | AC | AE | AG | AI | AK | AM | AO | AQ | AS | AU | AW | AY | BA | BC | BE | BF | BG | BH | BI | BK |
|---|---|---|---|---|---|---|---|---|---|---|---|---|---|---|---|---|---|---|---|---|---|---|---|---|---|---|---|---|---|---|---|---|---|---|---|
| 526 | 00586 | 00525 | 6 | | 1 | 0 | 0 | 1 | 1 | 0 | 0 | 0 | 0 | 0 | 1 | 0 | 0 | 1 | 1 | 0 | 0 | 0 | 0 | 0 | 0 | 0 | 0 | 0 | 0 | X07674 | 95.5 | 396 | 1 | 2559 | 2970 |
| 527 | 00589 | 00526 | 1 | | 1 | 0 | 0 | 0 | 0 | 0 | 0 | 0 | 0 | 0 | 0 | 0 | 0 | 0 | 0 | 0 | 0 | 0 | 0 | 0 | 0 | 0 | 0 | 0 | 0 | | | | | | |
| 528 | 00590 | 00527 | 3 | | 1 | 0 | 0 | 0 | 0 | 0 | 0 | 0 | 0 | 0 | 0 | 0 | 0 | 0 | 1 | 1 | 0 | 0 | 0 | 0 | 0 | 0 | 0 | 0 | 0 | | | | | | |
| 529 | 00591 | 00528 | 1 | | 1 | 0 | 0 | 0 | 0 | 0 | 0 | 0 | 0 | 0 | 0 | 0 | 0 | 0 | 0 | 0 | 0 | 0 | 0 | 0 | 0 | 0 | 0 | 0 | 0 | M96954 | 97.2 | 351 | 1 | 1045 | 1401 |
| 530 | 00592 | 00529 | 15 | | 1 | 1 | 1 | 0 | 1 | 0 | 0 | 0 | 0 | 1 | 0 | 1 | 0 | 0 | 2 | 4 | 0 | 0 | 0 | 0 | 0 | 1 | 1 | 1 | 0 | M84643 | 96.7 | 331 | 1 | 174 | 506 |
| 531 | 00593 | 00530 | 6 | | 1 | 0 | 0 | 0 | 0 | 0 | 2 | 0 | 0 | 0 | 0 | 0 | 0 | 1 | 0 | 0 | 0 | 0 | 0 | 1 | 0 | 1 | 0 | 0 | 0 | | | | | | |
| 532 | 00594 | 00531 | 17 | | 3 | 1 | 0 | 0 | 0 | 0 | 1 | 1 | 1 | 0 | 0 | 1 | 0 | 0 | 0 | 1 | 1 | 1 | 0 | 0 | 0 | 4 | 0 | 2 | 0 | M14199 | 99.7 | 341 | 1 | 136 | 475 |
| 533 | 00595 | 00532 | 4 | | 1 | 1 | 0 | 0 | 0 | 0 | 1 | 0 | 0 | 0 | 0 | 0 | 0 | 0 | 0 | 0 | 0 | 0 | 0 | 0 | 0 | 0 | 0 | 1 | 0 | Y00282 | 98.5 | 344 | 1 | 1968 | 2509 |
| 534 | 00596 | 00533 | 1 | | 1 | 0 | 0 | 0 | 0 | 0 | 0 | 0 | 0 | 0 | 0 | 0 | 0 | 0 | 0 | 0 | 0 | 0 | 0 | 0 | 0 | 0 | 0 | 0 | 0 | | | | | | |
| 535 | 00597 | 00534 | 5 | | 1 | 1 | 0 | 0 | 0 | 0 | 0 | 0 | 0 | 0 | 0 | 1 | 0 | 0 | 0 | 0 | 0 | 0 | 0 | 1 | 0 | 1 | 0 | 0 | 0 | | | | | | |
| 536 | 00598 | 00535 | 3 | | 1 | 0 | 2 | 0 | 0 | 0 | 0 | 0 | 0 | 0 | 0 | 0 | 0 | 0 | 0 | 0 | 0 | 0 | 0 | 0 | 0 | 0 | 0 | 0 | 0 | | | | | | |
| 537 | 00599 | 00536 | 4 | | 2 | 0 | 0 | 0 | 1 | 0 | 0 | 0 | 0 | 0 | 0 | 0 | 0 | 1 | 0 | 0 | 0 | 0 | 0 | 0 | 0 | 0 | 0 | 0 | 0 | | | | | | |
| 538 | 00600 | 00537 | 12 | | 2 | 0 | 0 | 0 | 0 | 0 | 1 | 0 | 1 | 0 | 0 | 0 | 1 | 0 | 1 | 0 | 0 | 0 | 0 | 3 | 0 | 0 | 2 | 1 | 0 | | | | | | |
| 539 | 00601 | 00538 | 1 | | 1 | 0 | 0 | 0 | 0 | 0 | 0 | 0 | 0 | 0 | 0 | 0 | 0 | 0 | 0 | 0 | 0 | 0 | 0 | 0 | 0 | 0 | 0 | 0 | 0 | | | | | | |
| 540 | 00602 | 00539 | 2 | | 1 | 0 | 0 | 0 | 0 | 0 | 0 | 0 | 0 | 0 | 0 | 0 | 0 | 0 | 0 | 0 | 0 | 0 | 0 | 0 | 0 | 1 | 0 | 0 | 0 | | | | | | |
| 541 | 00603 | 00540 | 1 | | 1 | 0 | 0 | 0 | 0 | 0 | 0 | 0 | 0 | 0 | 0 | 0 | 0 | 0 | 0 | 0 | 0 | 0 | 0 | 0 | 0 | 0 | 0 | 0 | 0 | | | | | | |
| 542 | 00604 | 00541 | 4 | | 1 | 1 | 0 | 0 | 0 | 0 | 0 | 0 | 0 | 0 | 0 | 0 | 0 | 0 | 0 | 0 | 0 | 0 | 0 | 0 | 2 | 0 | 0 | 0 | 0 | | | | | | |
| 543 | 00606 | 00542 | 1 | | 1 | 0 | 0 | 0 | 0 | 0 | 0 | 0 | 0 | 0 | 0 | 0 | 0 | 0 | 0 | 0 | 0 | 0 | 0 | 0 | 0 | 0 | 0 | 0 | 0 | | | | | | |
| 544 | 00607 | 00543 | 3 | | 1 | 0 | 0 | 0 | 0 | 0 | 0 | 0 | 0 | 0 | 0 | 1 | 0 | 0 | 0 | 0 | 0 | 0 | 0 | 1 | 0 | 0 | 0 | 0 | 0 | U02619 | 93.3 | 193 | 1 | 6800 | 6996 |
| 545 | 00608 | 00544 | 22 | | 5 | 1 | 0 | 0 | 0 | 0 | 0 | 0 | 0 | 0 | 0 | 6 | 0 | 2 | 0 | 0 | 0 | 1 | 1 | 0 | 0 | 0 | 0 | 6 | 0 | Z11566 | 99.1 | 321 | 1 | 878 | 1640 |
| 546 | 00609 | 00545 | 1 | | 1 | 0 | 0 | 0 | 0 | 0 | 0 | 0 | 0 | 0 | 0 | 0 | 0 | 0 | 0 | 0 | 0 | 0 | 0 | 0 | 0 | 0 | 0 | 0 | 0 | | | | | | |
| 547 | 00610 | 00546 | 2 | | 1 | 0 | 0 | 0 | 0 | 0 | 0 | 0 | 0 | 0 | 0 | 0 | 0 | 0 | 0 | 0 | 0 | 0 | 0 | 0 | 0 | 0 | 1 | 0 | 0 | | | | | | |
| 548 | 00611 | 00547 | 1 | | 1 | 0 | 0 | 0 | 0 | 0 | 0 | 0 | 0 | 0 | 0 | 0 | 0 | 0 | 0 | 0 | 0 | 0 | 0 | 0 | 0 | 0 | 0 | 0 | 0 | | | | | | |
| 549 | 00612 | 00548 | 3 | | 1 | 0 | 0 | 0 | 0 | 0 | 1 | 0 | 0 | 0 | 0 | 0 | 1 | 0 | 0 | 0 | 0 | 0 | 0 | 0 | 0 | 0 | 0 | 0 | 0 | | | | | | |
| 550 | 00614 | 00549 | 2 | | 1 | 0 | 0 | 0 | 0 | 0 | 0 | 0 | 0 | 0 | 0 | 0 | 0 | 0 | 0 | 0 | 1 | 0 | 0 | 0 | 0 | 0 | 0 | 0 | 0 | | | | | | |
| 551 | 00615 | 00550 | 8 | | 1 | 0 | 0 | 0 | 1 | 0 | 0 | 1 | 0 | 0 | 0 | 0 | 0 | 0 | 0 | 0 | 1 | 0 | 0 | 1 | 1 | 2 | 0 | 0 | 0 | X67688 | 96.5 | 318 | 1 | 1735 | 2106 |
| 552 | 00616 | 00551 | 1 | | 1 | 0 | 0 | 0 | 0 | 0 | 0 | 0 | 0 | 0 | 0 | 0 | 0 | 0 | 0 | 0 | 0 | 0 | 0 | 0 | 0 | 0 | 0 | 0 | 0 | | | | | | |
| 553 | 00617 | 00552 | 1 | | 1 | 0 | 0 | 0 | 0 | 0 | 0 | 0 | 0 | 0 | 0 | 0 | 0 | 0 | 0 | 0 | 0 | 0 | 0 | 0 | 0 | 0 | 0 | 0 | 0 | | | | | | |
| 554 | 00618 | 00553 | 2 | | 1 | 0 | 0 | 0 | 0 | 0 | 0 | 0 | 0 | 0 | 0 | 0 | 0 | 0 | 0 | 0 | 0 | 0 | 0 | 0 | 0 | 0 | 0 | 1 | 0 | | | | | | |
| 555 | 00619 | 00554 | 4 | | 1 | 0 | 1 | 0 | 0 | 0 | 0 | 0 | 0 | 0 | 1 | 0 | 0 | 0 | 0 | 1 | 0 | 0 | 0 | 0 | 0 | 0 | 0 | 0 | 0 | | | | | | |
| 556 | 00620 | 00555 | 1 | | 1 | 0 | 0 | 0 | 0 | 0 | 0 | 0 | 0 | 0 | 0 | 0 | 0 | 0 | 0 | 0 | 0 | 0 | 0 | 0 | 0 | 0 | 0 | 0 | 0 | | | | | | |
| 557 | 00621 | 00556 | 1 | | 1 | 0 | 0 | 0 | 0 | 0 | 0 | 0 | 0 | 0 | 0 | 0 | 0 | 0 | 0 | 0 | 0 | 0 | 0 | 0 | 0 | 0 | 0 | 0 | 0 | | | | | | |
| 558 | 00622 | 00557 | 3 | | 2 | 0 | 0 | 0 | 0 | 0 | 0 | 0 | 0 | 0 | 0 | 0 | 0 | 0 | 0 | 0 | 0 | 0 | 0 | 0 | 0 | 0 | 0 | 1 | 0 | S43127 | 98 | 298 | 1 | 1145 | 1441 |
| 559 | 00623 | 00558 | 4 | | 1 | 0 | 1 | 0 | 0 | 0 | 0 | 0 | 0 | 0 | 1 | 1 | 0 | 0 | 0 | 0 | 0 | 0 | 0 | 0 | 0 | 0 | 0 | 0 | 0 | | | | | | |
| 560 | 00624 | 00559 | 3 | | 1 | 1 | 0 | 0 | 0 | 0 | 0 | 0 | 0 | 0 | 0 | 0 | 0 | 0 | 1 | 0 | 0 | 0 | 0 | 0 | 0 | 0 | 0 | 0 | 0 | | | | | | |
| 561 | 00625 | 00560 | 4 | | 1 | 0 | 0 | 0 | 0 | 1 | 0 | 0 | 0 | 0 | 0 | 0 | 0 | 0 | 1 | 0 | 0 | 0 | 0 | 0 | 0 | 0 | 0 | 1 | 0 | | | | | | |

Table 16

| | A | B | C | E | G | I | K | M | O | Q | S | U | W | Y | AA | AC | AE | AG | AI | AK | AM | AO | AQ | AS | AU | AW | AY | BA | BC | BE | BF | BG | BH | BI | BK |
|---|---|---|---|---|---|---|---|---|---|---|---|---|---|---|---|---|---|---|---|---|---|---|---|---|---|---|---|---|---|---|---|---|---|---|---|
| 562 | 00626 | 00561 | 4 | | 2 | 1 | 0 | 0 | 0 | 0 | 0 | 0 | 1 | 0 | 0 | 0 | 0 | 0 | 0 | 0 | 0 | 0 | 0 | 0 | 0 | 0 | 0 | 0 | 0 | | | | | | |
| 563 | 00627 | 00562 | 2 | | 1 | 0 | 0 | 0 | 0 | 0 | 0 | 0 | 0 | 0 | 0 | 0 | 0 | 0 | 0 | 0 | 0 | 0 | 0 | 0 | 0 | 0 | 1 | 0 | 0 | | | | | | |
| 564 | 00628 | 00563 | 4 | | 1 | 1 | 2 | 0 | 0 | 0 | 0 | 0 | 0 | 0 | 0 | 0 | 0 | 0 | 0 | 0 | 0 | 0 | 0 | 0 | 0 | 0 | 0 | 0 | 0 | | | | | | |
| 565 | 00629 | 00564 | 3 | | 1 | 0 | 0 | 0 | 0 | 0 | 0 | 0 | 0 | 0 | 1 | 0 | 0 | 1 | 0 | 0 | 0 | 0 | 0 | 0 | 0 | 0 | 0 | 0 | 0 | | | | | | |
| 566 | 00630 | 00565 | 3 | | 1 | 0 | 0 | 0 | 0 | 0 | 0 | 0 | 0 | 1 | 0 | 0 | 0 | 0 | 0 | 0 | 0 | 1 | 0 | 0 | 0 | 0 | 0 | 0 | 0 | | | | | | |
| 567 | 00631 | 00566 | 2 | | 2 | 0 | 0 | 0 | 0 | 0 | 0 | 0 | 0 | 0 | 0 | 0 | 0 | 0 | 0 | 0 | 0 | 0 | 0 | 0 | 0 | 0 | 0 | 0 | 0 | | | | | | |
| 568 | 00632 | 00567 | 5 | | 1 | 1 | 0 | 0 | 0 | 0 | 0 | 0 | 0 | 0 | 0 | 0 | 0 | 0 | 1 | 1 | 1 | 0 | 0 | 0 | 0 | 0 | 0 | 0 | 0 | | | | | | |
| 569 | 00633 | 00568 | 3 | | 1 | 0 | 0 | 0 | 0 | 0 | 0 | 0 | 0 | 0 | 0 | 0 | 0 | 0 | 0 | 0 | 0 | 0 | 0 | 0 | 0 | 1 | 0 | 1 | 0 | | | | | | |
| 570 | 00634 | 00569 | 2 | | 1 | 0 | 0 | 0 | 0 | 0 | 0 | 0 | 0 | 0 | 0 | 0 | 0 | 0 | 0 | 0 | 0 | 0 | 1 | 0 | 0 | 0 | 0 | 0 | 0 | M19961 | 93.6 | 283 | 1 | 171 | 494 |
| 571 | 00635 | 00570 | 2 | | 1 | 0 | 0 | 0 | 0 | 0 | 0 | 0 | 0 | 0 | 0 | 1 | 0 | 0 | 0 | 0 | 0 | 0 | 0 | 0 | 0 | 0 | 0 | 0 | 0 | | | | | | |
| 572 | 00636 | 00571 | 5 | | 1 | 1 | 0 | 0 | 0 | 0 | 0 | 0 | 0 | 0 | 0 | 0 | 0 | 0 | 1 | 0 | 1 | 0 | 0 | 0 | 0 | 0 | 0 | 1 | 0 | | | | | | |
| 573 | 00637 | 00572 | 3 | | 1 | 0 | 0 | 0 | 0 | 0 | 0 | 0 | 0 | 0 | 0 | 0 | 0 | 0 | 1 | 0 | 0 | 0 | 0 | 0 | 0 | 0 | 1 | 0 | 0 | | | | | | |
| 574 | 00638 | 00573 | 3 | | 1 | 1 | 0 | 0 | 0 | 0 | 0 | 0 | 0 | 0 | 0 | 0 | 0 | 0 | 0 | 0 | 0 | 0 | 0 | 1 | 0 | 0 | 0 | 0 | 0 | | | | | | |
| 575 | 00639 | 00574 | 1 | | 1 | 0 | 0 | 0 | 0 | 0 | 0 | 0 | 0 | 0 | 0 | 0 | 0 | 0 | 0 | 0 | 0 | 0 | 0 | 0 | 0 | 0 | 0 | 0 | 0 | | | | | | |
| 576 | 00640 | 00575 | 10 | | 2 | 0 | 0 | 0 | 0 | 0 | 0 | 0 | 0 | 0 | 0 | 1 | 1 | 1 | 0 | 2 | 0 | 2 | 0 | 0 | 0 | 0 | 0 | 1 | 0 | | | | | | |
| 577 | 00641 | 00576 | 8 | | 2 | 2 | 0 | 0 | 0 | 0 | 0 | 0 | 0 | 1 | 0 | 1 | 0 | 0 | 1 | 1 | 0 | 0 | 0 | 0 | 0 | 0 | 0 | 0 | 0 | M55621 | 98.3 | 289 | 1 | 2311 | 2602 |
| 578 | 00642 | 00577 | 1 | | 1 | 0 | 0 | 0 | 0 | 0 | 0 | 0 | 0 | 0 | 0 | 0 | 0 | 0 | 0 | 0 | 0 | 0 | 0 | 0 | 0 | 0 | 0 | 0 | 0 | | | | | | |
| 579 | 00643 | 00578 | 2 | | 1 | 0 | 0 | 0 | 0 | 0 | 0 | 0 | 0 | 0 | 0 | 0 | 0 | 0 | 0 | 0 | 0 | 0 | 0 | 0 | 0 | 1 | 0 | 0 | 0 | X13482 | 96 | 277 | 1 | 760 | 1054 |
| 580 | 00644 | 00579 | 24 | | 5 | 0 | 2 | 0 | 1 | 1 | 0 | 0 | 3 | 2 | 0 | 0 | 2 | 0 | 0 | 0 | 0 | 0 | 0 | 2 | 0 | 2 | 0 | 4 | 0 | | | | | | |
| 581 | 00645 | 00580 | 1 | | 1 | 0 | 0 | 0 | 0 | 0 | 0 | 0 | 0 | 0 | 0 | 0 | 0 | 0 | 0 | 0 | 0 | 0 | 0 | 0 | 0 | 0 | 0 | 0 | 0 | X71491 | 93 | 285 | 1 | 929 | 1234 |
| 582 | 00646 | 00581 | 1 | | 1 | 0 | 0 | 0 | 0 | 0 | 0 | 0 | 0 | 0 | 0 | 0 | 0 | 0 | 0 | 0 | 0 | 0 | 0 | 0 | 0 | 0 | 0 | 0 | 0 | | | | | | |
| 583 | 00647 | 00582 | 3 | | 2 | 0 | 0 | 0 | 0 | 0 | 0 | 0 | 0 | 0 | 0 | 0 | 0 | 0 | 1 | 0 | 0 | 0 | 0 | 0 | 0 | 0 | 0 | 0 | 0 | M31520 | 97.4 | 305 | 1 | 133 | 620 |
| 584 | 00648 | 00583 | 8 | | 1 | 0 | 0 | 0 | 0 | 0 | 0 | 0 | 2 | 0 | 0 | 0 | 1 | 0 | 0 | 0 | 2 | 0 | 0 | 1 | 0 | 0 | 1 | 0 | 0 | | | | | | |
| 585 | 00649 | 00584 | 3 | | 1 | 0 | 0 | 0 | 0 | 0 | 0 | 0 | 0 | 0 | 0 | 0 | 1 | 0 | 0 | 1 | 0 | 0 | 0 | 0 | 0 | 0 | 0 | 0 | 0 | | | | | | |
| 586 | 00650 | 00585 | 52 | | 14 | 2 | 6 | 0 | 2 | 0 | 1 | 1 | 2 | 1 | 6 | 3 | 2 | 0 | 0 | 0 | 1 | 2 | 0 | 0 | 2 | 2 | 1 | 4 | 0 | L06432 | 97.4 | 265 | 1 | 280 | 549 |
| 587 | 00651 | 00586 | 9 | | 4 | 1 | 0 | 0 | 0 | 0 | 0 | 0 | 1 | 0 | 0 | 0 | 0 | 0 | 0 | 0 | 1 | 0 | 0 | 0 | 0 | 0 | 2 | 0 | 0 | L20688 | 97.7 | 266 | 1 | 984 | 1249 |
| 588 | 00652 | 00587 | 2 | | 1 | 0 | 0 | 0 | 0 | 0 | 0 | 0 | 0 | 0 | 0 | 0 | 0 | 0 | 0 | 0 | 1 | 0 | 0 | 0 | 0 | 0 | 0 | 0 | 0 | | | | | | |
| 589 | 00653 | 00588 | 13 | | 1 | 0 | 0 | 0 | 0 | 0 | 0 | 1 | 0 | 0 | 0 | 1 | 0 | 4 | 0 | 0 | 1 | 0 | 1 | 4 | 0 | 0 | 0 | 0 | 0 | J04046 | 96.2 | 266 | 1 | 1910 | 2175 |
| 590 | 00654 | 00589 | 2 | | 1 | 0 | 0 | 0 | 0 | 0 | 0 | 0 | 0 | 0 | 0 | 0 | 0 | 0 | 0 | 0 | 0 | 0 | 0 | 0 | 0 | 1 | 0 | 0 | 0 | X54942 | 97.8 | 268 | 1 | 348 | 627 |
| 591 | 00656 | 00590 | 3 | | 2 | 0 | 0 | 0 | 0 | 0 | 0 | 0 | 0 | 0 | 0 | 0 | 0 | 0 | 1 | 0 | 0 | 0 | 0 | 0 | 0 | 0 | 0 | 0 | 0 | | | | | | |
| 592 | 00657 | 00591 | 3 | | 1 | 0 | 0 | 0 | 0 | 0 | 0 | 0 | 0 | 0 | 0 | 0 | 1 | 0 | 0 | 0 | 0 | 0 | 0 | 0 | 0 | 0 | 0 | 1 | 0 | | | | | | |
| 593 | 00658 | 00592 | 1 | | 1 | 0 | 0 | 0 | 0 | 0 | 0 | 0 | 0 | 0 | 0 | 0 | 0 | 0 | 0 | 0 | 0 | 0 | 0 | 0 | 0 | 0 | 0 | 0 | 0 | | | | | | |
| 594 | 00659 | 00593 | 6 | | 2 | 0 | 0 | 0 | 0 | 0 | 0 | 0 | 0 | 0 | 0 | 0 | 0 | 3 | 0 | 0 | 0 | 0 | 0 | 1 | 0 | 0 | 0 | 0 | 0 | | | | | | |
| 595 | 00661 | 00594 | 9 | | 2 | 0 | 0 | 0 | 0 | 0 | 1 | 1 | 1 | 0 | 0 | 0 | 0 | 0 | 0 | 0 | 0 | 1 | 1 | 0 | 0 | 1 | 0 | 1 | 0 | | | | | | |
| 596 | 00662 | 00595 | 2 | | 1 | 0 | 0 | 0 | 0 | 0 | 0 | 0 | 0 | 1 | 0 | 0 | 0 | 0 | 0 | 0 | 0 | 0 | 0 | 0 | 0 | 0 | 0 | 0 | 0 | | | | | | |
| 597 | 00663 | 00596 | 1 | | 1 | 0 | 0 | 0 | 0 | 0 | 0 | 0 | 0 | 0 | 0 | 0 | 0 | 0 | 0 | 0 | 0 | 0 | 0 | 0 | 0 | 0 | 0 | 0 | 0 | | | | | | |

Table 17

| | A | B | C | E | G | I | K | M | O | Q | S | U | W | Y | AA | AC | AE | AG | AI | AK | AM | AO | AQ | AS | AU | AW | AY | BA | BC | BE | BF | BG | BH | BI | BK |
|---|---|---|---|---|---|---|---|---|---|---|---|---|---|---|---|---|---|---|---|---|---|---|---|---|---|---|---|---|---|---|---|---|---|---|---|
| 598 | 00664 | 00597 | 3 | | 1 | 0 | 1 | 0 | 0 | 0 | 0 | 0 | 0 | 0 | 0 | 0 | 0 | 0 | 0 | 0 | 0 | 0 | 0 | 0 | 0 | 0 | 0 | 1 | 0 | | | | | | |
| 599 | 00665 | 00598 | 2 | | 1 | 0 | 0 | 1 | 0 | 0 | 0 | 0 | 0 | 0 | 0 | 0 | 0 | 0 | 0 | 0 | 0 | 0 | 0 | 0 | 0 | 0 | 0 | 0 | 0 | | | | | | |
| 600 | 00666 | 00599 | 5 | | 1 | 0 | 1 | 0 | 0 | 0 | 0 | 0 | 0 | 0 | 0 | 1 | 0 | 0 | 0 | 0 | 0 | 0 | 0 | 0 | 0 | 0 | 0 | 2 | 0 | X65867 | 98.4 | 248 | 1 | 1335 | 1692 |
| 601 | 00667 | 00600 | 1 | | 1 | 0 | 0 | 0 | 0 | 0 | 0 | 0 | 0 | 0 | 0 | 0 | 0 | 0 | 0 | 0 | 0 | 0 | 0 | 0 | 0 | 0 | 0 | 0 | 0 | | | | | | |
| 602 | 00668 | 00601 | 8 | | 2 | 0 | 1 | 0 | 0 | 0 | 1 | 0 | 3 | 1 | 0 | 0 | 0 | 0 | 0 | 0 | 0 | 0 | 0 | 0 | 0 | 0 | 0 | 0 | 0 | | | | | | |
| 603 | 00669 | 00602 | 2 | | 1 | 0 | 0 | 1 | 0 | 0 | 0 | 0 | 0 | 0 | 0 | 0 | 0 | 0 | 0 | 0 | 0 | 0 | 0 | 0 | 0 | 0 | 0 | 0 | 0 | | | | | | |
| 604 | 00670 | 00603 | 12 | | 1 | 0 | 0 | 0 | 0 | 2 | 0 | 0 | 2 | 1 | 0 | 0 | 0 | 0 | 1 | 1 | 1 | 0 | 0 | 0 | 2 | 0 | 0 | 1 | 0 | M60857 | 98.4 | 249 | 1 | 600 | 851 |
| 605 | 00671 | 00604 | 3 | | 2 | 0 | 0 | 0 | 0 | 0 | 0 | 0 | 0 | 0 | 0 | 0 | 0 | 0 | 0 | 0 | 0 | 0 | 0 | 0 | 0 | 1 | 0 | 0 | 0 | | | | | | |
| 606 | 00672 | 00605 | 7 | | 3 | 0 | 0 | 0 | 0 | 0 | 0 | 0 | 0 | 0 | 1 | 0 | 0 | 0 | 0 | 0 | 2 | 0 | 1 | 0 | 0 | 0 | 0 | 0 | 0 | X66975 | 97.9 | 241 | 1 | 2915 | 3319 |
| 607 | 00673 | 00606 | 3 | | 1 | 0 | 0 | 0 | 0 | 0 | 0 | 0 | 0 | 0 | 0 | 1 | 0 | 0 | 0 | 0 | 0 | 0 | 0 | 1 | 0 | 0 | 0 | 0 | 0 | X57500 | 96.2 | 240 | 1 | 2728 | 2969 |
| 608 | 00674 | 00607 | 5 | | 1 | 1 | 0 | 0 | 0 | 0 | 0 | 1 | 0 | 0 | 0 | 0 | 0 | 0 | 0 | 0 | 0 | 1 | 1 | 0 | 0 | 0 | 0 | 0 | 0 | | | | | | |
| 609 | 00675 | 00608 | 31 | | 4 | 1 | 4 | 0 | 1 | 0 | 2 | 1 | 1 | 0 | 0 | 2 | 5 | 0 | 0 | 1 | 0 | 2 | 0 | 0 | 0 | 1 | 0 | 6 | 0 | L01124 | 100 | 237 | 1 | 291 | 530 |
| 610 | 00676 | 00609 | 1 | | 1 | 0 | 0 | 0 | 0 | 0 | 0 | 0 | 0 | 0 | 0 | 0 | 0 | 0 | 0 | 0 | 0 | 0 | 0 | 0 | 0 | 0 | 0 | 0 | 0 | | | | | | |
| 611 | 00677 | 00610 | 1 | | 1 | 0 | 0 | 0 | 0 | 0 | 0 | 0 | 0 | 0 | 0 | 0 | 0 | 0 | 0 | 0 | 0 | 0 | 0 | 0 | 0 | 0 | 0 | 0 | 0 | | | | | | |
| 612 | 00678 | 00611 | 1 | | 1 | 0 | 0 | 0 | 0 | 0 | 0 | 0 | 0 | 0 | 0 | 0 | 0 | 0 | 0 | 0 | 0 | 0 | 0 | 0 | 0 | 0 | 0 | 0 | 0 | | | | | | |
| 613 | 00679 | 00612 | 15 | | 1 | 1 | 0 | 0 | 0 | 0 | 0 | 0 | 1 | 0 | 0 | 0 | 2 | 0 | 0 | 3 | 1 | 2 | 2 | 2 | 0 | 0 | 0 | 0 | 0 | | | | | | |
| 614 | 00680 | 00613 | 1 | | 1 | 0 | 0 | 0 | 0 | 0 | 0 | 0 | 0 | 0 | 0 | 0 | 0 | 0 | 0 | 0 | 0 | 0 | 0 | 0 | 0 | 0 | 0 | 0 | 0 | | | | | | |
| 615 | 00681 | 00614 | 7 | | 1 | 0 | 0 | 0 | 0 | 0 | 0 | 0 | 1 | 0 | 0 | 2 | 0 | 0 | 0 | 0 | 0 | 2 | 0 | 1 | 0 | 0 | 0 | 0 | 0 | L01457 | 100 | 238 | 1 | 2484 | 2739 |
| 616 | 00682 | 00615 | 3 | | 1 | 0 | 0 | 0 | 1 | 0 | 0 | 0 | 0 | 0 | 0 | 1 | 0 | 0 | 0 | 0 | 0 | 0 | 0 | 0 | 0 | 0 | 0 | 0 | 0 | S63912 | 99.2 | 254 | 1 | 1304 | 3043 |
| 617 | 00683 | 00616 | 20 | | 1 | 2 | 0 | 1 | 0 | 0 | 0 | 1 | 1 | 0 | 1 | 0 | 2 | 2 | 1 | 3 | 0 | 0 | 3 | 0 | 0 | 0 | 0 | 2 | 0 | X13238 | 96.1 | 230 | 1 | 194 | 422 |
| 618 | 00684 | 00617 | 4 | | 1 | 0 | 0 | 0 | 0 | 0 | 0 | 0 | 0 | 0 | 0 | 0 | 0 | 0 | 0 | 1 | 0 | 0 | 0 | 1 | 1 | 0 | 0 | 0 | 0 | D21065 | 95.8 | 237 | 1 | 1728 | 3755 |
| 619 | 00685 | 00618 | 18 | | 2 | 5 | 4 | 0 | 0 | 1 | 0 | 0 | 0 | 0 | 0 | 0 | 0 | 0 | 2 | 0 | 0 | 0 | 0 | 0 | 0 | 0 | 4 | 0 | 0 | | | | | | |
| 620 | 00686 | 00619 | 1 | | 1 | 0 | 0 | 0 | 0 | 0 | 0 | 0 | 0 | 0 | 0 | 0 | 0 | 0 | 0 | 0 | 0 | 0 | 0 | 0 | 0 | 0 | 0 | 0 | 0 | | | | | | |
| 621 | 00687 | 00620 | 16 | | 1 | 3 | 5 | 0 | 0 | 0 | 0 | 0 | 0 | 7 | 0 | 0 | 0 | 0 | 0 | 0 | 0 | 0 | 0 | 0 | 0 | 0 | 0 | 0 | 0 | M80927 | 98.4 | 246 | 1 | 1494 | 1741 |
| 622 | 00689 | 00621 | 40 | | 1 | 1 | 2 | 0 | 1 | 3 | 3 | 0 | 4 | 0 | 3 | 2 | 2 | 2 | 1 | 2 | 1 | 5 | 1 | 1 | 1 | 1 | 0 | 2 | 1 | | | | | | |
| 623 | 00690 | 00622 | 1 | | 1 | 0 | 0 | 0 | 0 | 0 | 0 | 0 | 0 | 0 | 0 | 0 | 0 | 0 | 0 | 0 | 0 | 0 | 0 | 0 | 0 | 0 | 0 | 0 | 0 | | | | | | |
| 624 | 00691 | 00623 | 3 | | 1 | 1 | 0 | 0 | 0 | 0 | 0 | 0 | 0 | 0 | 0 | 0 | 0 | 0 | 0 | 0 | 1 | 0 | 0 | 0 | 0 | 0 | 0 | 0 | 0 | | | | | | |
| 625 | 00692 | 00624 | 6 | | 2 | 0 | 0 | 0 | 0 | 0 | 0 | 0 | 2 | 0 | 0 | 0 | 0 | 0 | 0 | 0 | 0 | 0 | 0 | 0 | 0 | 1 | 0 | 1 | 0 | | | | | | |
| 626 | 00693 | 00625 | 4 | | 1 | 0 | 0 | 0 | 0 | 0 | 0 | 0 | 0 | 1 | 1 | 0 | 0 | 0 | 0 | 0 | 0 | 1 | 0 | 0 | 0 | 0 | 0 | 0 | 0 | | | | | | |
| 627 | 00694 | 00626 | 3 | | 2 | 0 | 1 | 0 | 0 | 0 | 0 | 0 | 0 | 0 | 0 | 0 | 0 | 0 | 0 | 0 | 0 | 0 | 0 | 0 | 0 | 0 | 0 | 0 | 0 | | | | | | |
| 628 | 00695 | 00627 | 1 | | 1 | 0 | 0 | 0 | 0 | 0 | 0 | 0 | 0 | 0 | 0 | 0 | 0 | 0 | 0 | 0 | 0 | 0 | 0 | 0 | 0 | 0 | 0 | 0 | 0 | | | | | | |
| 629 | 00696 | 00628 | 1 | | 1 | 0 | 0 | 0 | 0 | 0 | 0 | 0 | 0 | 0 | 0 | 0 | 0 | 0 | 0 | 0 | 0 | 0 | 0 | 0 | 0 | 0 | 0 | 0 | 0 | | | | | | |
| 630 | 00697 | 00629 | 7 | | 2 | 3 | 2 | 0 | 0 | 0 | 0 | 0 | 0 | 0 | 0 | 0 | 0 | 0 | 0 | 0 | 0 | 0 | 0 | 0 | 0 | 0 | 0 | 0 | 0 | M18082 | 96.9 | 226 | 1 | 1640 | 1880 |
| 631 | 00698 | 00630 | 3 | | 1 | 0 | 0 | 0 | 0 | 0 | 0 | 0 | 0 | 0 | 0 | 0 | 0 | 0 | 0 | 1 | 0 | 0 | 0 | 0 | 0 | 1 | 0 | 0 | 0 | | | | | | |
| 632 | 00699 | 00631 | 1 | | 1 | 0 | 0 | 0 | 0 | 0 | 0 | 0 | 0 | 0 | 0 | 0 | 0 | 0 | 0 | 0 | 0 | 0 | 0 | 0 | 0 | 0 | 0 | 0 | 0 | | | | | | |
| 633 | 00700 | 00632 | 1 | | 1 | 0 | 0 | 0 | 0 | 0 | 0 | 0 | 0 | 0 | 0 | 0 | 0 | 0 | 0 | 0 | 0 | 0 | 0 | 0 | 0 | 0 | 0 | 0 | 0 | | | | | | |

Table 18

| | A | B | C | E | G | I | K | M | O | Q | S | U | W | Y | AA | AC | AE | AG | AI | AK | AM | AO | AQ | AS | AU | AW | AY | BA | BC | BE | BF | BG | BH | BI | BK |
|---|---|---|---|---|---|---|---|---|---|---|---|---|---|---|---|---|---|---|---|---|---|---|---|---|---|---|---|---|---|---|---|---|---|---|---|
| 634 | 00701 | 00633 | 1 | | 1 | 0 | 0 | 0 | 0 | 0 | 0 | 0 | 0 | 0 | 0 | 0 | 0 | 0 | 0 | 0 | 0 | 0 | 0 | 0 | 0 | 0 | 0 | 0 | 0 | | | | | | |
| 635 | 00702 | 00634 | 2 | | 2 | 0 | 0 | 0 | 0 | 0 | 0 | 0 | 0 | 0 | 0 | 0 | 0 | 0 | 0 | 0 | 0 | 0 | 0 | 0 | 0 | 0 | 0 | 0 | 0 | | | | | | |
| 636 | 00703 | 00635 | 17 | | 4 | 0 | 0 | 0 | 0 | 0 | 0 | 0 | 0 | 2 | 0 | 0 | 0 | 0 | 0 | 2 | 1 | 1 | 2 | 0 | 1 | 2 | 0 | 2 | 0 | L25899 | 98.3 | 172 | 1 | 466 | 635 |
| 637 | 00704 | 00636 | 45 | | 4 | 1 | 0 | 0 | 1 | 0 | 1 | 1 | 6 | 1 | 2 | 3 | 2 | 0 | 0 | 0 | 3 | 8 | 0 | 0 | 7 | 3 | 0 | 1 | 1 | M73791 | 100 | 217 | 1 | 506 | 722 |
| 638 | 00705 | 00637 | 1 | | 1 | 0 | 0 | 0 | 0 | 0 | 0 | 0 | 0 | 0 | 0 | 0 | 0 | 0 | 0 | 0 | 0 | 0 | 0 | 0 | 0 | 0 | 0 | 0 | 0 | | | | | | |
| 639 | 00706 | 00638 | 2 | | 1 | 0 | 0 | 0 | 0 | 0 | 0 | 0 | 0 | 0 | 1 | 0 | 0 | 0 | 0 | 0 | 0 | 0 | 0 | 0 | 0 | 0 | 0 | 0 | 0 | | | | | | |
| 640 | 00707 | 00639 | 10 | | 1 | 0 | 0 | 0 | 0 | 0 | 1 | 0 | 0 | 3 | 0 | 0 | 0 | 0 | 1 | 0 | 2 | 1 | 0 | 0 | 1 | 0 | 0 | 0 | 0 | M24194 | 92.3 | 209 | 1 | 887 | 1093 |
| 641 | 00708 | 00640 | 32 | | 1 | 0 | 2 | 0 | 1 | 0 | 3 | 3 | 3 | 2 | 0 | 1 | 0 | 2 | 0 | 2 | 3 | 2 | 0 | 1 | 2 | 1 | 0 | 2 | 1 | | | | | | |
| 642 | 00710 | 00641 | 2 | | 1 | 0 | 0 | 0 | 0 | 0 | 0 | 0 | 0 | 1 | 0 | 0 | 0 | 0 | 0 | 0 | 0 | 0 | 0 | 0 | 0 | 0 | 0 | 0 | 0 | X75690 | 98.4 | 127 | 84 | 3 | 135 |
| 643 | 00711 | 00642 | 3 | | 1 | 0 | 0 | 0 | 0 | 0 | 1 | 0 | 0 | 0 | 0 | 0 | 0 | 0 | 0 | 0 | 0 | 0 | 0 | 1 | 0 | 0 | 0 | 0 | 0 | | | | | | |
| 644 | 00712 | 00643 | 1 | | 1 | 0 | 0 | 0 | 0 | 0 | 0 | 0 | 0 | 0 | 0 | 0 | 0 | 0 | 0 | 0 | 0 | 0 | 0 | 0 | 0 | 0 | 0 | 0 | 0 | | | | | | |
| 645 | 00713 | 00644 | 3 | | 1 | 0 | 0 | 0 | 0 | 0 | 0 | 0 | 0 | 0 | 1 | 0 | 0 | 0 | 0 | 1 | 0 | 0 | 0 | 0 | 0 | 0 | 0 | 0 | 0 | | | | | | |
| 646 | 00714 | 00645 | 13 | | 3 | 0 | 0 | 0 | 0 | 0 | 0 | 1 | 0 | 0 | 0 | 0 | 2 | 1 | 0 | 0 | 0 | 0 | 0 | 0 | 0 | 0 | 0 | 6 | 0 | X17620 | 99 | 202 | 1 | 531 | 732 |
| 647 | 00715 | 00646 | 1 | | 1 | 0 | 0 | 0 | 0 | 0 | 0 | 0 | 0 | 0 | 0 | 0 | 0 | 0 | 0 | 0 | 0 | 0 | 0 | 0 | 0 | 0 | 0 | 0 | 0 | | | | | | |
| 648 | 00716 | 00647 | 48 | | 5 | 3 | 3 | 0 | 0 | 0 | 2 | 1 | 3 | 0 | 1 | 3 | 1 | 0 | 2 | 9 | 2 | 2 | 2 | 2 | 3 | 1 | 0 | 2 | 1 | X56998 | 94 | 200 | 1 | 291 | 495 |
| 649 | 00717 | 00648 | 6 | | 1 | 1 | 0 | 0 | 0 | 0 | 0 | 0 | 3 | 0 | 0 | 1 | 0 | 0 | 0 | 0 | 0 | 0 | 0 | 0 | 0 | 0 | 0 | 0 | 0 | L19067 | 97.5 | 198 | 1 | 2231 | 2444 |
| 650 | 00718 | 00649 | 3 | | 1 | 0 | 0 | 0 | 0 | 0 | 0 | 0 | 0 | 0 | 0 | 0 | 0 | 2 | 0 | 0 | 0 | 0 | 0 | 0 | 0 | 0 | 0 | 0 | 0 | | | | | | |
| 651 | 00719 | 00650 | 2 | | 1 | 0 | 0 | 0 | 0 | 0 | 0 | 0 | 0 | 0 | 0 | 0 | 1 | 0 | 0 | 0 | 0 | 0 | 0 | 0 | 0 | 0 | 0 | 0 | 0 | | | | | | |
| 652 | 00720 | 00651 | 1 | | 1 | 0 | 0 | 0 | 0 | 0 | 0 | 0 | 0 | 0 | 0 | 0 | 0 | 0 | 0 | 0 | 0 | 0 | 0 | 0 | 0 | 0 | 0 | 0 | 0 | | | | | | |
| 653 | 00721 | 00652 | 1 | | 1 | 0 | 0 | 0 | 0 | 0 | 0 | 0 | 0 | 0 | 0 | 0 | 0 | 0 | 0 | 0 | 0 | 0 | 0 | 0 | 0 | 0 | 0 | 0 | 0 | | | | | | |
| 654 | 00722 | 00653 | 1 | | 1 | 0 | 0 | 0 | 0 | 0 | 0 | 0 | 0 | 0 | 0 | 0 | 0 | 0 | 0 | 0 | 0 | 0 | 0 | 0 | 0 | 0 | 0 | 0 | 0 | | | | | | |
| 655 | 00723 | 00654 | 11 | | 1 | 0 | 0 | 0 | 2 | 0 | 0 | 2 | 0 | 0 | 1 | 0 | 0 | 1 | 0 | 0 | 2 | 1 | 0 | 0 | 1 | 0 | 0 | 0 | 0 | L10376 | 97 | 202 | 1 | 509 | 753 |
| 656 | 00725 | 00655 | 10 | | 1 | 0 | 0 | 0 | 1 | 0 | 1 | 0 | 1 | 1 | 1 | 0 | 1 | 0 | 2 | 0 | 0 | 0 | 0 | 1 | 0 | 0 | 0 | 0 | 0 | | | | | | |
| 657 | 00726 | 00656 | 1 | | 1 | 0 | 0 | 0 | 0 | 0 | 0 | 0 | 0 | 0 | 0 | 0 | 0 | 0 | 0 | 0 | 0 | 0 | 0 | 0 | 0 | 0 | 0 | 0 | 0 | | | | | | |
| 658 | 00727 | 00657 | 3 | | 1 | 0 | 0 | 0 | 0 | 0 | 0 | 0 | 0 | 2 | 0 | 0 | 0 | 0 | 0 | 0 | 0 | 0 | 0 | 0 | 0 | 0 | 0 | 0 | 0 | | | | | | |
| 659 | 00728 | 00658 | 5 | | 1 | 0 | 0 | 0 | 1 | 0 | 0 | 0 | 0 | 0 | 1 | 0 | 0 | 0 | 1 | 0 | 0 | 0 | 0 | 0 | 0 | 0 | 0 | 0 | 1 | M77024 | 92.5 | 133 | 1 | 932 | 1064 |
| 660 | 00729 | 00659 | 7 | | 2 | 0 | 0 | 0 | 0 | 0 | 0 | 0 | 0 | 0 | 0 | 0 | 0 | 0 | 1 | 0 | 1 | 0 | 0 | 0 | 0 | 0 | 3 | 0 | 0 | | | | | | |
| 661 | 00730 | 00660 | 4 | | 1 | 0 | 0 | 0 | 0 | 0 | 0 | 0 | 0 | 0 | 0 | 0 | 0 | 2 | 0 | 0 | 1 | 0 | 0 | 0 | 0 | 0 | 0 | 0 | 0 | | | | | | |
| 662 | 00731 | 00661 | 2 | | 1 | 0 | 0 | 0 | 1 | 0 | 0 | 0 | 0 | 0 | 0 | 0 | 0 | 0 | 0 | 0 | 0 | 0 | 0 | 0 | 0 | 0 | 0 | 0 | 0 | | | | | | |
| 663 | 00732 | 00662 | 47 | | 6 | 3 | 4 | 0 | 1 | 5 | 1 | 1 | 1 | 0 | 1 | 6 | 2 | 0 | 2 | 7 | 0 | 4 | 3 | 0 | 0 | 0 | 0 | 0 | 0 | | | | | | |
| 664 | 00733 | 00663 | 1 | | 1 | 0 | 0 | 0 | 0 | 0 | 0 | 0 | 0 | 0 | 0 | 0 | 0 | 0 | 0 | 0 | 0 | 0 | 0 | 0 | 0 | 0 | 0 | 0 | 0 | | | | | | |
| 665 | 00734 | 00664 | 3 | | 1 | 0 | 0 | 0 | 0 | 0 | 0 | 0 | 0 | 0 | 0 | 1 | 0 | 0 | 0 | 0 | 1 | 0 | 0 | 0 | 0 | 0 | 0 | 0 | 0 | | | | | | |
| 666 | 00735 | 00665 | 11 | | 1 | 1 | 1 | 0 | 0 | 0 | 0 | 0 | 1 | 0 | 0 | 0 | 0 | 2 | 1 | 0 | 1 | 3 | 0 | 0 | 0 | 0 | 0 | 0 | 0 | | | | | | |
| 667 | 00736 | 00666 | 2 | | 1 | 0 | 0 | 0 | 0 | 0 | 0 | 0 | 0 | 0 | 0 | 0 | 0 | 0 | 0 | 0 | 0 | 0 | 0 | 0 | 0 | 0 | 0 | 0 | 1 | | | | | | |
| 668 | 00737 | 00667 | 3 | | 1 | 0 | 0 | 0 | 0 | 0 | 0 | 0 | 0 | 0 | 1 | 0 | 0 | 0 | 0 | 0 | 0 | 1 | 0 | 0 | 0 | 0 | 0 | 0 | 0 | | | | | | |
| 669 | 00738 | 00668 | 6 | | 1 | 0 | 1 | 0 | 0 | 0 | 0 | 0 | 0 | 0 | 0 | 1 | 0 | 0 | 0 | 0 | 0 | 1 | 0 | 1 | 0 | 0 | 0 | 1 | 0 | | | | | | |

Table 19

| | A | B | C | E | G | I | K | M | O | Q | S | U | W | Y | AA | AC | AE | AG | AI | AK | AM | AO | AQ | AS | AU | AW | AY | BA | BC | BE | BF | BG | BH | BI | BK |
|---|---|---|---|---|---|---|---|---|---|---|---|---|---|---|---|---|---|---|---|---|---|---|---|---|---|---|---|---|---|---|---|---|---|---|---|
| 670 | 00739 | 00669 | 1 | | 1 | 0 | 0 | 0 | 0 | 0 | 0 | 0 | 0 | 0 | 0 | 0 | 0 | 0 | 0 | 0 | 0 | 0 | 0 | 0 | 0 | 0 | 0 | 0 | 0 | | | | | | |
| 671 | 00740 | 00670 | 1 | | 1 | 0 | 0 | 0 | 0 | 0 | 0 | 0 | 0 | 0 | 0 | 0 | 0 | 0 | 0 | 0 | 0 | 0 | 0 | 0 | 0 | 0 | 0 | 0 | 0 | | | | | | |
| 672 | 00741 | 00671 | 2 | | 1 | 0 | 0 | 0 | 0 | 0 | 0 | 0 | 0 | 0 | 0 | 0 | 0 | 0 | 0 | 0 | 0 | 0 | 0 | 0 | 1 | 0 | 0 | 0 | 0 | | | | | | |
| 673 | 00742 | 00672 | 1 | | 1 | 0 | 0 | 0 | 0 | 0 | 0 | 0 | 0 | 0 | 0 | 0 | 0 | 0 | 0 | 0 | 0 | 0 | 0 | 0 | 0 | 0 | 0 | 0 | 0 | | | | | | |
| 674 | 00743 | 00673 | 54 | | 7 | 1 | 7 | 2 | 6 | 1 | 0 | 1 | 8 | 0 | 0 | 3 | 3 | 2 | 0 | 0 | 2 | 0 | 1 | 1 | 0 | 3 | 0 | 6 | 0 | | | | | | |
| 675 | 00745 | 00674 | 4 | | 1 | 0 | 0 | 0 | 1 | 1 | 0 | 0 | 0 | 0 | 0 | 0 | 0 | 0 | 0 | 0 | 0 | 0 | 0 | 0 | 0 | 0 | 0 | 1 | 0 | | | | | | |
| 676 | 00746 | 00675 | 13 | | 1 | 2 | 1 | 0 | 0 | 0 | 0 | 0 | 0 | 0 | 0 | 1 | 0 | 2 | 0 | 1 | 0 | 1 | 1 | 1 | 2 | 0 | 0 | 0 | 0 | | | | | | |
| 677 | 00747 | 00676 | 1 | | 1 | 0 | 0 | 0 | 0 | 0 | 0 | 0 | 0 | 0 | 0 | 0 | 0 | 0 | 0 | 0 | 0 | 0 | 0 | 0 | 0 | 0 | 0 | 0 | 0 | S62904 | 95.9 | 170 | 1 | 1589 | 2742 |
| 678 | 00748 | 00677 | 5 | | 1 | 0 | 0 | 0 | 0 | 0 | 0 | 0 | 1 | 0 | 1 | 0 | 0 | 0 | 0 | 0 | 0 | 0 | 0 | 0 | 0 | 0 | 0 | 2 | 0 | | | | | | |
| 679 | 00749 | 00678 | 1 | | 1 | 0 | 0 | 0 | 0 | 0 | 0 | 0 | 0 | 0 | 0 | 0 | 0 | 0 | 0 | 0 | 0 | 0 | 0 | 0 | 0 | 0 | 0 | 0 | 0 | | | | | | |
| 680 | 00750 | 00679 | 1 | | 1 | 0 | 0 | 0 | 0 | 0 | 0 | 0 | 0 | 0 | 0 | 0 | 0 | 0 | 0 | 0 | 0 | 0 | 0 | 0 | 0 | 0 | 0 | 0 | 0 | | | | | | |
| 681 | 00751 | 00680 | 5 | | 1 | 1 | 0 | 0 | 0 | 0 | 0 | 0 | 0 | 0 | 0 | 0 | 0 | 0 | 0 | 1 | 0 | 0 | 0 | 0 | 0 | 0 | 1 | 0 | 1 | | | | | | |
| 682 | 00752 | 00681 | 4 | | 3 | 0 | 0 | 0 | 0 | 0 | 0 | 0 | 0 | 1 | 0 | 0 | 0 | 0 | 0 | 0 | 0 | 0 | 0 | 0 | 0 | 0 | 0 | 0 | 0 | X75252 | 97.1 | 170 | 1 | 873 | 1444 |
| 683 | 00753 | 00682 | 6 | | 2 | 0 | 0 | 0 | 0 | 0 | 0 | 0 | 0 | 0 | 0 | 1 | 0 | 1 | 2 | 0 | 0 | 0 | 0 | 0 | 0 | 0 | 0 | 0 | 0 | | | | | | |
| 684 | 00754 | 00683 | 5 | | 1 | 0 | 0 | 0 | 0 | 0 | 0 | 0 | 0 | 0 | 0 | 1 | 0 | 0 | 0 | 0 | 0 | 0 | 1 | 0 | 0 | 1 | 0 | 1 | 0 | | | | | | |
| 685 | 00755 | 00684 | 2 | | 1 | 0 | 0 | 0 | 0 | 0 | 0 | 0 | 0 | 0 | 0 | 0 | 0 | 0 | 0 | 1 | 0 | 0 | 0 | 0 | 0 | 0 | 0 | 0 | 0 | D00723 | 97.6 | 168 | 1 | 989 | 1192 |
| 686 | 00757 | 00685 | 5 | | 1 | 0 | 1 | 0 | 0 | 0 | 0 | 0 | 1 | 0 | 1 | 0 | 0 | 0 | 0 | 0 | 0 | 0 | 1 | 0 | 0 | 0 | 0 | 0 | 0 | | | | | | |
| 687 | 00758 | 00686 | 5 | | 1 | 0 | 0 | 0 | 0 | 0 | 1 | 0 | 0 | 0 | 0 | 0 | 0 | 0 | 0 | 1 | 0 | 0 | 0 | 0 | 1 | 0 | 0 | 1 | 0 | D14663 | 97 | 164 | 1 | 1145 | 1308 |
| 688 | 00759 | 00687 | 25 | | 4 | 1 | 1 | 0 | 1 | 1 | 0 | 0 | 2 | 1 | 0 | 2 | 2 | 0 | 0 | 0 | 1 | 0 | 1 | 0 | 1 | 4 | 0 | 3 | 0 | M13932 | 98.7 | 159 | 1 | 320 | 477 |
| 689 | 00760 | 00688 | 1 | | 1 | 0 | 0 | 0 | 0 | 0 | 0 | 0 | 0 | 0 | 0 | 0 | 0 | 0 | 0 | 0 | 0 | 0 | 0 | 0 | 0 | 0 | 0 | 0 | 0 | | | | | | |
| 690 | 00761 | 00689 | 10 | | 1 | 2 | 0 | 0 | 0 | 0 | 1 | 1 | 0 | 1 | 0 | 0 | 0 | 1 | 0 | 1 | 0 | 0 | 0 | 1 | 1 | 0 | 0 | 0 | 0 | U02570 | 99.4 | 164 | 1 | 3182 | 3345 |
| 691 | 00762 | 00690 | 1 | | 1 | 0 | 0 | 0 | 0 | 0 | 0 | 0 | 0 | 0 | 0 | 0 | 0 | 0 | 0 | 0 | 0 | 0 | 0 | 0 | 0 | 0 | 0 | 0 | 0 | M34379 | 93.8 | 160 | 1 | 761 | 920 |
| 692 | 00763 | 00691 | 3 | | 1 | 0 | 0 | 0 | 0 | 0 | 0 | 0 | 0 | 0 | 0 | 0 | 0 | 0 | 0 | 0 | 0 | 0 | 0 | 0 | 0 | 0 | 0 | 0 | 2 | | | | | | |
| 693 | 00764 | 00692 | 6 | | 1 | 0 | 1 | 0 | 0 | 0 | 0 | 0 | 0 | 0 | 0 | 0 | 1 | 0 | 0 | 0 | 0 | 0 | 0 | 0 | 0 | 1 | 0 | 2 | 0 | D21853 | 98.8 | 168 | 1 | 1516 | 1682 |
| 694 | 00765 | 00693 | 1 | | 1 | 0 | 0 | 0 | 0 | 0 | 0 | 0 | 0 | 0 | 0 | 0 | 0 | 0 | 0 | 0 | 0 | 0 | 0 | 0 | 0 | 0 | 0 | 0 | 0 | X05875 | 93.2 | 161 | 1 | 761 | 920 |
| 695 | 00766 | 00694 | 2 | | 1 | 0 | 0 | 0 | 0 | 0 | 0 | 0 | 0 | 0 | 0 | 0 | 0 | 0 | 0 | 0 | 0 | 0 | 0 | 0 | 0 | 1 | 0 | 0 | 0 | | | | | | |
| 696 | 00768 | 00695 | 2 | | 1 | 0 | 0 | 0 | 0 | 0 | 0 | 0 | 0 | 0 | 0 | 0 | 0 | 0 | 0 | 0 | 0 | 0 | 0 | 0 | 0 | 0 | 0 | 0 | 1 | | | | | | |
| 697 | 00769 | 00696 | 8 | | 1 | 0 | 0 | 0 | 0 | 0 | 0 | 0 | 0 | 0 | 0 | 0 | 1 | 1 | 0 | 0 | 1 | 0 | 4 | 0 | 0 | 0 | 0 | 0 | 0 | L16842 | 100 | 159 | 1 | 1827 | 1985 |
| 698 | 00770 | 00697 | 4 | | 3 | 0 | 0 | 0 | 0 | 0 | 0 | 0 | 0 | 0 | 0 | 0 | 0 | 0 | 0 | 0 | 0 | 0 | 0 | 0 | 0 | 0 | 0 | 1 | 0 | | | | | | |
| 699 | 00771 | 00698 | 3 | | 1 | 0 | 0 | 1 | 0 | 0 | 0 | 0 | 0 | 0 | 0 | 0 | 0 | 0 | 0 | 0 | 0 | 0 | 0 | 0 | 0 | 1 | 0 | 0 | 0 | | | | | | |
| 700 | 00772 | 00699 | 6 | | 1 | 1 | 0 | 0 | 0 | 0 | 0 | 0 | 0 | 0 | 0 | 0 | 1 | 0 | 2 | 0 | 0 | 0 | 0 | 1 | 0 | 0 | 0 | 0 | 0 | | | | | | |
| 701 | 00773 | 00700 | 1 | | 1 | 0 | 0 | 0 | 0 | 0 | 0 | 0 | 0 | 0 | 0 | 0 | 0 | 0 | 0 | 0 | 0 | 0 | 0 | 0 | 0 | 0 | 0 | 0 | 0 | | | | | | |
| 702 | 00774 | 00701 | 5 | | 1 | 0 | 1 | 0 | 0 | 0 | 0 | 0 | 0 | 0 | 0 | 2 | 0 | 0 | 0 | 0 | 1 | 0 | 0 | 0 | 0 | 0 | 0 | 0 | 0 | | | | | | |
| 703 | 00775 | 00702 | 3 | | 1 | 0 | 0 | 0 | 0 | 0 | 0 | 0 | 0 | 0 | 0 | 0 | 0 | 0 | 0 | 0 | 0 | 0 | 0 | 1 | 0 | 0 | 0 | 1 | 0 | X63692 | 93.2 | 161 | 1 | 5018 | 5194 |
| 704 | 00777 | 00703 | 3 | | 1 | 0 | 0 | 0 | 0 | 0 | 0 | 0 | 0 | 0 | 0 | 0 | 0 | 0 | 0 | 0 | 2 | 0 | 0 | 0 | 0 | 0 | 0 | 0 | 0 | | | | | | |
| 705 | 00778 | 00704 | 2 | | 1 | 0 | 1 | 0 | 0 | 0 | 0 | 0 | 0 | 0 | 0 | 0 | 0 | 0 | 0 | 0 | 0 | 0 | 0 | 0 | 0 | 0 | 0 | 0 | 0 | | | | | | |

Table 20

| | A | B | C | E | G | I | K | M | O | Q | S | U | W | Y | AA | AC | AE | AG | AI | AK | AM | AO | AQ | AS | AU | AW | AY | BA | BC | BE | BF | BG | BH | BI | BK |
|---|---|---|---|---|---|---|---|---|---|---|---|---|---|---|---|---|---|---|---|---|---|---|---|---|---|---|---|---|---|---|---|---|---|---|---|
| 706 | 00779 | 00705 | 3 | | 3 | 0 | 0 | 0 | 0 | 0 | 0 | 0 | 0 | 0 | 0 | 0 | 0 | 0 | 0 | 0 | 0 | 0 | 0 | 0 | 0 | 0 | 0 | 0 | 0 | X55668 | 92.6 | 149 | 1 | 824 | 965 |
| 707 | 00780 | 00706 | 3 | | 1 | 0 | 0 | 0 | 0 | 0 | 0 | 0 | 0 | 0 | 0 | 0 | 0 | 0 | 0 | 0 | 2 | 0 | 0 | 0 | 0 | 0 | 0 | 0 | 0 | X70944 | 100 | 152 | 1 | 2916 | 3071 |
| 708 | 00781 | 00707 | 1 | | 1 | 0 | 0 | 0 | 0 | 0 | 0 | 0 | 0 | 0 | 0 | 0 | 0 | 0 | 0 | 0 | 0 | 0 | 0 | 0 | 0 | 0 | 0 | 0 | 0 | | | | | | |
| 709 | 00782 | 00708 | 8 | | 1 | 1 | 0 | 0 | 0 | 0 | 0 | 0 | 1 | 0 | 0 | 1 | 0 | 0 | 0 | 2 | 0 | 1 | 0 | 0 | 0 | 0 | 0 | 1 | 0 | | | | | | |
| 710 | 00783 | 00709 | 5 | | 1 | 3 | 0 | 0 | 0 | 0 | 0 | 0 | 0 | 0 | 0 | 0 | 0 | 0 | 0 | 0 | 0 | 0 | 0 | 0 | 0 | 0 | 1 | 0 | 0 | | | | | | |
| 711 | 00784 | 00710 | 1 | | 1 | 0 | 0 | 0 | 0 | 0 | 0 | 0 | 0 | 0 | 0 | 0 | 0 | 0 | 0 | 0 | 0 | 0 | 0 | 0 | 0 | 0 | 0 | 0 | 0 | | | | | | |
| 712 | 00785 | 00711 | 2 | | 1 | 0 | 0 | 0 | 0 | 0 | 0 | 0 | 0 | 0 | 0 | 0 | 0 | 0 | 0 | 1 | 0 | 0 | 0 | 0 | 0 | 0 | 0 | 0 | 0 | M37716 | 100 | 145 | 1 | 329 | 491 |
| 713 | 00786 | 00712 | 4 | | 1 | 0 | 0 | 0 | 1 | 0 | 0 | 0 | 0 | 0 | 0 | 1 | 0 | 0 | 0 | 0 | 0 | 0 | 0 | 0 | 0 | 1 | 0 | 0 | 0 | | | | | | |
| 714 | 00787 | 00713 | 8 | | 1 | 0 | 0 | 0 | 0 | 0 | 0 | 0 | 0 | 0 | 0 | 0 | 0 | 2 | 0 | 1 | 0 | 0 | 0 | 0 | 0 | 0 | 0 | 4 | 0 | | | | | | |
| 715 | 00788 | 00714 | 4 | | 1 | 1 | 0 | 0 | 1 | 0 | 0 | 0 | 0 | 0 | 1 | 0 | 0 | 0 | 0 | 0 | 0 | 0 | 0 | 0 | 0 | 0 | 0 | 0 | 0 | | | | | | |
| 716 | 00789 | 00715 | 3 | | 1 | 0 | 0 | 0 | 0 | 0 | 0 | 0 | 0 | 0 | 0 | 0 | 0 | 0 | 0 | 1 | 0 | 0 | 0 | 0 | 1 | 0 | 0 | 0 | 0 | M15502 | 96.5 | 141 | 1 | 1310 | 1447 |
| 717 | 00790 | 00716 | 5 | | 1 | 0 | 2 | 0 | 0 | 0 | 0 | 0 | 0 | 0 | 0 | 0 | 0 | 0 | 0 | 0 | 0 | 1 | 0 | 0 | 0 | 1 | 0 | 0 | 0 | X59417 | 96.6 | 149 | 1 | 824 | 979 |
| 718 | 00791 | 00717 | 6 | | 1 | 0 | 0 | 0 | 0 | 0 | 0 | 0 | 1 | 1 | 1 | 1 | 0 | 1 | 0 | 0 | 0 | 0 | 0 | 0 | 0 | 0 | 0 | 0 | 0 | | | | | | |
| 719 | 00792 | 00718 | 1 | | 1 | 0 | 0 | 0 | 0 | 0 | 0 | 0 | 0 | 0 | 0 | 0 | 0 | 0 | 0 | 0 | 0 | 0 | 0 | 0 | 0 | 0 | 0 | 0 | 0 | | | | | | |
| 720 | 00793 | 00719 | 3 | | 1 | 0 | 0 | 0 | 0 | 0 | 0 | 0 | 0 | 0 | 0 | 0 | 0 | 1 | 0 | 0 | 0 | 0 | 0 | 1 | 0 | 0 | 0 | 0 | 0 | | | | | | |
| 721 | 00794 | 00720 | 1 | | 1 | 0 | 0 | 0 | 0 | 0 | 0 | 0 | 0 | 0 | 0 | 0 | 0 | 0 | 0 | 0 | 0 | 0 | 0 | 0 | 0 | 0 | 0 | 0 | 0 | | | | | | |
| 722 | 00795 | 00721 | 42 | | 1 | 1 | 2 | 0 | 1 | 3 | 0 | 5 | 0 | 2 | 0 | 4 | 5 | 0 | 1 | 2 | 0 | 1 | 0 | 1 | 4 | 7 | 1 | 1 | 0 | | | | | | |
| 723 | 00796 | 00722 | 2 | | 1 | 0 | 0 | 0 | 0 | 0 | 0 | 0 | 0 | 1 | 0 | 0 | 0 | 0 | 0 | 0 | 0 | 0 | 0 | 0 | 0 | 0 | 0 | 0 | 0 | L14754 | 98.5 | 133 | 1 | 3761 | 3892 |
| 724 | 00797 | 00723 | 36 | | 4 | 0 | 1 | 0 | 2 | 1 | 0 | 1 | 1 | 2 | 1 | 5 | 2 | 1 | 0 | 4 | 1 | 3 | 0 | 0 | 3 | 3 | 0 | 1 | 0 | J02984 | 100 | 137 | 1 | 362 | 498 |
| 725 | 00798 | 00724 | 4 | | 2 | 0 | 0 | 0 | 0 | 0 | 0 | 0 | 0 | 0 | 0 | 0 | 0 | 0 | 0 | 1 | 0 | 1 | 0 | 0 | 0 | 0 | 0 | 0 | 0 | | | | | | |
| 726 | 00799 | 00725 | 13 | | 1 | 0 | 0 | 0 | 0 | 0 | 0 | 2 | 0 | 1 | 1 | 2 | 1 | 0 | 1 | 0 | 1 | 0 | 1 | 2 | 0 | 0 | 0 | 0 | 0 | X63423 | 94.3 | 141 | 1 | 545 | 691 |
| 727 | 00800 | 00726 | 6 | | 1 | 0 | 0 | 0 | 1 | 0 | 0 | 0 | 2 | 0 | 0 | 0 | 0 | 0 | 1 | 0 | 0 | 0 | 0 | 0 | 0 | 0 | 0 | 1 | 0 | | | | | | |
| 728 | 00801 | 00727 | 9 | | 1 | 0 | 0 | 1 | 0 | 0 | 0 | 0 | 0 | 0 | 1 | 0 | 0 | 0 | 1 | 1 | 1 | 0 | 0 | 1 | 0 | 1 | 0 | 1 | 0 | J04823 | 99.2 | 132 | 1 | 342 | 472 |
| 729 | 00802 | 00728 | 1 | | 1 | 0 | 0 | 0 | 0 | 0 | 0 | 0 | 0 | 0 | 0 | 0 | 0 | 0 | 0 | 0 | 0 | 0 | 0 | 0 | 0 | 0 | 0 | 0 | 0 | | | | | | |
| 730 | 00803 | 00729 | 7 | | 1 | 1 | 1 | 0 | 1 | 0 | 0 | 1 | 0 | 0 | 0 | 0 | 0 | 0 | 0 | 1 | 0 | 1 | 0 | 0 | 0 | 0 | 0 | 0 | 0 | | | | | | |
| 731 | 00804 | 00730 | 5 | | 1 | 0 | 0 | 0 | 0 | 0 | 0 | 0 | 0 | 0 | 0 | 1 | 0 | 0 | 0 | 0 | 0 | 0 | 0 | 0 | 0 | 0 | 0 | 3 | 0 | | | | | | |
| 732 | 00805 | 00731 | 6 | | 2 | 2 | 0 | 0 | 0 | 0 | 0 | 0 | 0 | 0 | 1 | 0 | 0 | 0 | 0 | 0 | 0 | 0 | 0 | 0 | 0 | 0 | 0 | 1 | 0 | | | | | | |
| 733 | 00806 | 00732 | 7 | | 1 | 2 | 0 | 0 | 0 | 0 | 0 | 0 | 1 | 0 | 0 | 1 | 0 | 0 | 0 | 0 | 0 | 0 | 1 | 0 | 0 | 0 | 1 | 0 | 0 | | | | | | |
| 734 | 00807 | 00733 | 23 | | 2 | 0 | 1 | 1 | 0 | 1 | 0 | 1 | 3 | 1 | 1 | 1 | 1 | 0 | 0 | 1 | 0 | 0 | 0 | 0 | 0 | 2 | 0 | 6 | 1 | X69392 | 98.5 | 134 | 1 | 357 | 773 |
| 735 | 00808 | 00734 | 8 | | 2 | 0 | 0 | 0 | 0 | 1 | 0 | 0 | 0 | 1 | 0 | 0 | 1 | 0 | 0 | 0 | 0 | 0 | 1 | 0 | 0 | 0 | 0 | 2 | 0 | | | | | | |
| 736 | 00809 | 00735 | 1 | | 1 | 0 | 0 | 0 | 0 | 0 | 0 | 0 | 0 | 0 | 0 | 0 | 0 | 0 | 0 | 0 | 0 | 0 | 0 | 0 | 0 | 0 | 0 | 0 | 0 | | | | | | |
| 737 | 00810 | 00736 | 1 | | 1 | 0 | 0 | 0 | 0 | 0 | 0 | 0 | 0 | 0 | 0 | 0 | 0 | 0 | 0 | 0 | 0 | 0 | 0 | 0 | 0 | 0 | 0 | 0 | 0 | V00568 | 95.4 | 130 | 1 | 1953 | 2121 |
| 738 | 00811 | 00737 | 4 | | 1 | 0 | 0 | 0 | 1 | 0 | 0 | 0 | 1 | 0 | 0 | 0 | 0 | 0 | 0 | 0 | 0 | 0 | 0 | 0 | 0 | 1 | 0 | 0 | 0 | | | | | | |
| 739 | 00813 | 00738 | 7 | | 1 | 0 | 0 | 0 | 0 | 0 | 0 | 0 | 0 | 0 | 0 | 1 | 3 | 0 | 0 | 0 | 0 | 1 | 1 | 0 | 0 | 0 | 0 | 0 | 0 | | | | | | |
| 740 | 00814 | 00739 | 8 | | 1 | 1 | 0 | 0 | 0 | 0 | 0 | 0 | 2 | 0 | 0 | 2 | 0 | 0 | 0 | 1 | 0 | 0 | 0 | 0 | 0 | 0 | 0 | 1 | 0 | | | | | | |
| 741 | 00815 | 00740 | 1 | | 1 | 0 | 0 | 0 | 0 | 0 | 0 | 0 | 0 | 0 | 0 | 0 | 0 | 0 | 0 | 0 | 0 | 0 | 0 | 0 | 0 | 0 | 0 | 0 | 0 | | | | | | |

Table 21

| | A | B | C | E | G | I | K | M | O | Q | S | U | W | Y | AA | AC | AE | AG | AI | AK | AM | AO | AQ | AS | AU | AW | AY | BA | BC | BE | BF | BG | BH | BI | BK |
|---|---|---|---|---|---|---|---|---|---|---|---|---|---|---|---|---|---|---|---|---|---|---|---|---|---|---|---|---|---|---|---|---|---|---|---|
| 742 | 00816 | 00741 | 14 | | 1 | 5 | 1 | 0 | 0 | 0 | 1 | 0 | 0 | 1 | 0 | 0 | 0 | 0 | 0 | 0 | 0 | 0 | 0 | 0 | 0 | 0 | 5 | 0 | 0 | M33552 | 100 | 125 | 1 | 1507 | 1631 |
| 743 | 00817 | 00742 | 2 | | 1 | 0 | 0 | 0 | 0 | 0 | 0 | 0 | 0 | 0 | 0 | 0 | 0 | 0 | 1 | 0 | 0 | 0 | 0 | 0 | 0 | 0 | 0 | 0 | 0 | | | | | | |
| 744 | 00818 | 00743 | 20 | | 4 | 1 | 1 | 0 | 2 | 1 | 0 | 0 | 0 | 0 | 4 | 0 | 2 | 0 | 0 | 0 | 0 | 0 | 1 | 0 | 0 | 0 | 1 | 3 | 0 | | | | | | |
| 745 | 00819 | 00744 | 1 | | 1 | 0 | 0 | 0 | 0 | 0 | 0 | 0 | 0 | 0 | 0 | 0 | 0 | 0 | 0 | 0 | 0 | 0 | 0 | 0 | 0 | 0 | 0 | 0 | 0 | | | | | | |
| 746 | 00820 | 00745 | 18 | | 1 | 0 | 0 | 0 | 2 | 0 | 1 | 0 | 0 | 1 | 0 | 2 | 0 | 1 | 2 | 1 | 0 | 0 | 0 | 2 | 1 | 1 | 2 | 1 | 0 | | | | | | |
| 747 | 00821 | 00746 | 1 | | 1 | 0 | 0 | 0 | 0 | 0 | 0 | 0 | 0 | 0 | 0 | 0 | 0 | 0 | 0 | 0 | 0 | 0 | 0 | 0 | 0 | 0 | 0 | 0 | 0 | | | | | | |
| 748 | 00822 | 00747 | 3 | | 1 | 0 | 1 | 0 | 0 | 0 | 1 | 0 | 0 | 0 | 0 | 0 | 0 | 0 | 0 | 0 | 0 | 0 | 0 | 0 | 0 | 0 | 0 | 0 | 0 | | | | | | |
| 749 | 00823 | 00748 | 1 | | 1 | 0 | 0 | 0 | 0 | 0 | 0 | 0 | 0 | 0 | 0 | 0 | 0 | 0 | 0 | 0 | 0 | 0 | 0 | 0 | 0 | 0 | 0 | 0 | 0 | | | | | | |
| 750 | 00824 | 00749 | 3 | | 1 | 0 | 0 | 0 | 0 | 0 | 0 | 0 | 0 | 0 | 0 | 0 | 0 | 0 | 1 | 1 | 0 | 0 | 0 | 0 | 0 | 0 | 0 | 0 | 0 | | | | | | |
| 751 | 00825 | 00750 | 1 | | 1 | 0 | 0 | 0 | 0 | 0 | 0 | 0 | 0 | 0 | 0 | 0 | 0 | 0 | 0 | 0 | 0 | 0 | 0 | 0 | 0 | 0 | 0 | 0 | 0 | | | | | | |
| 752 | 00826 | 00751 | 6 | | 1 | 0 | 1 | 0 | 0 | 0 | 0 | 0 | 0 | 1 | 0 | 1 | 0 | 0 | 0 | 1 | 0 | 0 | 0 | 0 | 0 | 0 | 0 | 1 | 0 | | | | | | |
| 753 | 00827 | 00752 | 10 | | 1 | 1 | 0 | 0 | 2 | 0 | 0 | 0 | 0 | 0 | 0 | 1 | 2 | 0 | 0 | 2 | 0 | 0 | 0 | 0 | 0 | 0 | 1 | 0 | 0 | M89913 | 99.2 | 123 | 1 | 716 | 837 |
| 754 | 00828 | 00753 | 3 | | 1 | 0 | 0 | 0 | 0 | 0 | 0 | 0 | 0 | 0 | 0 | 0 | 0 | 1 | 0 | 0 | 0 | 1 | 0 | 0 | 0 | 0 | 0 | 0 | 0 | | | | | | |
| 755 | 00829 | 00754 | 3 | | 2 | 0 | 0 | 0 | 0 | 0 | 0 | 0 | 0 | 0 | 0 | 0 | 0 | 0 | 0 | 0 | 0 | 0 | 1 | 0 | 0 | 0 | 0 | 0 | 0 | | | | | | |
| 756 | 00830 | 00755 | 1 | | 1 | 0 | 0 | 0 | 0 | 0 | 0 | 0 | 0 | 0 | 0 | 0 | 0 | 0 | 0 | 0 | 0 | 0 | 0 | 0 | 0 | 0 | 0 | 0 | 0 | | | | | | |
| 757 | 00831 | 00756 | 5 | | 1 | 0 | 0 | 0 | 0 | 1 | 0 | 0 | 0 | 0 | 0 | 0 | 0 | 0 | 2 | 0 | 0 | 0 | 0 | 0 | 0 | 0 | 0 | 1 | 0 | | | | | | |
| 758 | 00832 | 00757 | 6 | | 1 | 0 | 0 | 1 | 0 | 0 | 0 | 0 | 1 | 0 | 0 | 0 | 0 | 0 | 2 | 0 | 0 | 1 | 0 | 0 | 0 | 0 | 0 | 0 | 0 | | | | | | |
| 759 | 00833 | 00758 | 5 | | 1 | 0 | 0 | 0 | 0 | 0 | 0 | 0 | 0 | 0 | 0 | 2 | 0 | 0 | 0 | 0 | 1 | 0 | 0 | 0 | 0 | 0 | 1 | 0 | 0 | | | | | | |
| 760 | 00834 | 00759 | 13 | | 1 | 0 | 0 | 0 | 0 | 0 | 2 | 0 | 1 | 0 | 0 | 1 | 0 | 0 | 1 | 4 | 1 | 0 | 0 | 0 | 1 | 1 | 0 | 0 | 0 | | | | | | |
| 761 | 00835 | 00760 | 5 | | 1 | 0 | 0 | 1 | 0 | 0 | 0 | 1 | 0 | 0 | 1 | 0 | 1 | 0 | 0 | 0 | 0 | 0 | 0 | 0 | 0 | 0 | 0 | 0 | 0 | | | | | | |
| 762 | 00836 | 00761 | 8 | | 1 | 1 | 1 | 0 | 0 | 0 | 0 | 0 | 0 | 0 | 0 | 0 | 0 | 0 | 0 | 2 | 0 | 0 | 0 | 0 | 0 | 0 | 0 | 3 | 0 | M20372 | 94.6 | 370 | 1 | 880 | 1395 |
| 763 | 00837 | 00762 | 3 | | 1 | 0 | 0 | 0 | 0 | 0 | 0 | 0 | 0 | 0 | 0 | 0 | 0 | 0 | 0 | 0 | 0 | 0 | 0 | 0 | 0 | 1 | 0 | 1 | 0 | | | | | | |
| 764 | 00839 | 00763 | 7 | | 1 | 2 | 0 | 0 | 0 | 0 | 0 | 0 | 0 | 1 | 0 | 0 | 0 | 0 | 0 | 0 | 1 | 0 | 0 | 0 | 0 | 1 | 0 | 1 | 0 | L20216 | 94.8 | 116 | 1 | 4066 | 4181 |
| 765 | 00840 | 00764 | 8 | | 1 | 0 | 1 | 0 | 0 | 0 | 0 | 0 | 2 | 0 | 0 | 0 | 0 | 0 | 1 | 1 | 0 | 0 | 0 | 1 | 1 | 0 | 0 | 0 | 0 | | | | | | |
| 766 | 00841 | 00765 | 10 | | 5 | 1 | 1 | 1 | 0 | 0 | 0 | 0 | 0 | 0 | 0 | 0 | 0 | 0 | 0 | 1 | 0 | 1 | 0 | 0 | 0 | 0 | 0 | 0 | 0 | | | | | | |
| 767 | 00842 | 00766 | 1 | | 1 | 0 | 0 | 0 | 0 | 0 | 0 | 0 | 0 | 0 | 0 | 0 | 0 | 0 | 0 | 0 | 0 | 0 | 0 | 0 | 0 | 0 | 0 | 0 | 0 | | | | | | |
| 768 | 00843 | 00767 | 1 | | 1 | 0 | 0 | 0 | 0 | 0 | 0 | 0 | 0 | 0 | 0 | 0 | 0 | 0 | 0 | 0 | 0 | 0 | 0 | 0 | 0 | 0 | 0 | 0 | 0 | | | | | | |
| 769 | 00844 | 00768 | 5 | | 1 | 0 | 0 | 0 | 0 | 0 | 0 | 2 | 1 | 0 | 0 | 0 | 0 | 0 | 0 | 0 | 1 | 0 | 0 | 0 | 0 | 0 | 0 | 0 | 0 | | | | | | |
| 770 | 00845 | 00769 | 1 | | 1 | 0 | 0 | 0 | 0 | 0 | 0 | 0 | 0 | 0 | 0 | 0 | 0 | 0 | 0 | 0 | 0 | 0 | 0 | 0 | 0 | 0 | 0 | 0 | 0 | | | | | | |
| 771 | 00846 | 00770 | 1 | | 1 | 0 | 0 | 0 | 0 | 0 | 0 | 0 | 0 | 0 | 0 | 0 | 0 | 0 | 0 | 0 | 0 | 0 | 0 | 0 | 0 | 0 | 0 | 0 | 0 | | | | | | |
| 772 | 00847 | 00771 | 1 | | 1 | 0 | 0 | 0 | 0 | 0 | 0 | 0 | 0 | 0 | 0 | 0 | 0 | 0 | 0 | 0 | 0 | 0 | 0 | 0 | 0 | 0 | 0 | 0 | 0 | | | | | | |
| 773 | 00848 | 00772 | 12 | | 1 | 0 | 0 | 0 | 0 | 0 | 0 | 0 | 1 | 0 | 0 | 0 | 0 | 2 | 0 | 0 | 0 | 0 | 2 | 1 | 0 | 1 | 0 | 4 | 0 | Y00711 | 100 | 107 | 1 | 1154 | 1260 |
| 774 | 00849 | 00773 | 2 | | 1 | 0 | 0 | 0 | 0 | 0 | 0 | 0 | 0 | 0 | 0 | 1 | 0 | 0 | 0 | 0 | 0 | 0 | 0 | 0 | 0 | 0 | 0 | 0 | 0 | | | | | | |
| 775 | 00850 | 00774 | 12 | | 1 | 1 | 0 | 0 | 0 | 0 | 0 | 2 | 1 | 0 | 0 | 0 | 1 | 0 | 0 | 0 | 1 | 0 | 0 | 1 | 0 | 0 | 1 | 2 | 1 | | | | | | |
| 776 | 00851 | 00775 | 14 | | 5 | 1 | 0 | 1 | 0 | 0 | 0 | 0 | 0 | 0 | 1 | 2 | 0 | 0 | 3 | 0 | 0 | 0 | 1 | 0 | 0 | 0 | 0 | 0 | 0 | D00761 | 100 | 87 | 1 | 737 | 823 |
| 777 | 00852 | 00776 | 1 | | 1 | 0 | 0 | 0 | 0 | 0 | 0 | 0 | 0 | 0 | 0 | 0 | 0 | 0 | 0 | 0 | 0 | 0 | 0 | 0 | 0 | 0 | 0 | 0 | 0 | | | | | | |

Table 22

| | A | B | C | E | G | I | K | M | O | Q | S | U | W | Y | AA | AC | AE | AG | AI | AK | AM | AO | AQ | AS | AU | AW | AY | BA | BC | BE | BF | BG | BH | BI | BK |
|---|---|---|---|---|---|---|---|---|---|---|---|---|---|---|---|---|---|---|---|---|---|---|---|---|---|---|---|---|---|---|---|---|---|---|---|
| 778 | 00853 | 00777 | 1 | | 1 | 0 | 0 | 0 | 0 | 0 | 0 | 0 | 0 | 0 | 0 | 0 | 0 | 0 | 0 | 0 | 0 | 0 | 0 | 0 | 0 | 0 | 0 | 0 | 0 | M34082 | 90.5 | 105 | 1 | 591 | 702 |
| 779 | 00854 | 00778 | 8 | | 1 | 1 | 0 | 0 | 0 | 0 | 1 | 0 | 0 | 0 | 0 | 0 | 1 | 0 | 0 | 0 | 0 | 0 | 1 | 1 | 0 | 1 | 0 | 1 | 0 | | | | | | |
| 780 | 00855 | 00779 | 1 | | 1 | 0 | 0 | 0 | 0 | 0 | 0 | 0 | 0 | 0 | 0 | 0 | 0 | 0 | 0 | 0 | 0 | 0 | 0 | 0 | 0 | 0 | 0 | 0 | 0 | | | | | | |
| 781 | 00856 | 00780 | 4 | | 1 | 0 | 0 | 0 | 0 | 0 | 0 | 0 | 0 | 1 | 0 | 0 | 0 | 0 | 0 | 1 | 1 | 0 | 0 | 0 | 0 | 0 | 0 | 0 | 0 | | | | | | |
| 782 | 00857 | 00781 | 2 | | 1 | 0 | 0 | 0 | 0 | 0 | 0 | 0 | 0 | 0 | 0 | 0 | 0 | 0 | 0 | 0 | 0 | 0 | 0 | 0 | 0 | 0 | 0 | 0 | 1 | | | | | | |
| 783 | 00858 | 00782 | 6 | | 1 | 0 | 0 | 0 | 0 | 1 | 0 | 1 | 0 | 0 | 2 | 0 | 0 | 0 | 0 | 0 | 0 | 0 | 0 | 0 | 0 | 0 | 1 | 0 | 0 | | | | | | |
| 784 | 00859 | 00783 | 1 | | 1 | 0 | 0 | 0 | 0 | 0 | 0 | 0 | 0 | 0 | 0 | 0 | 0 | 0 | 0 | 0 | 0 | 0 | 0 | 0 | 0 | 0 | 0 | 0 | 0 | | | | | | |
| 785 | 00860 | 00784 | 3 | | 1 | 0 | 0 | 0 | 0 | 0 | 0 | 0 | 0 | 0 | 0 | 1 | 0 | 0 | 0 | 0 | 0 | 0 | 0 | 0 | 1 | 0 | 0 | 0 | 0 | | | | | | |
| 786 | 00861 | 00785 | 34 | | 6 | 0 | 2 | 0 | 1 | 0 | 0 | 1 | 4 | 1 | 0 | 2 | 3 | 0 | 1 | 1 | 0 | 1 | 1 | 1 | 0 | 3 | 0 | 6 | 0 | M31520 | 93.5 | 92 | 5 | 529 | 620 |
| 787 | 00862 | 00786 | 3 | | 1 | 0 | 0 | 0 | 0 | 0 | 0 | 0 | 0 | 0 | 0 | 0 | 0 | 0 | 0 | 1 | 0 | 0 | 0 | 0 | 1 | 0 | 0 | 0 | 0 | J03490 | 97.9 | 94 | 1 | 2002 | 2314 |
| 788 | 00863 | 00787 | 5 | | 1 | 0 | 0 | 0 | 0 | 0 | 0 | 0 | 0 | 0 | 0 | 1 | 0 | 0 | 0 | 0 | 0 | 0 | 0 | 3 | 0 | 0 | 0 | 0 | 0 | | | | | | |
| 789 | 00864 | 00788 | 5 | | 2 | 0 | 0 | 0 | 0 | 0 | 0 | 0 | 0 | 0 | 0 | 0 | 0 | 0 | 1 | 1 | 0 | 0 | 0 | 0 | 0 | 0 | 0 | 1 | 0 | | | | | | |
| 790 | 00865 | 00789 | 56 | | 9 | 2 | 1 | 0 | 0 | 0 | 2 | 2 | 8 | 2 | 1 | 4 | 1 | 0 | 3 | 5 | 4 | 0 | 0 | 0 | 9 | 2 | 0 | 1 | 0 | M55409 | 100 | 89 | 1 | 1067 | 1155 |
| 791 | 00866 | 00790 | 1 | | 1 | 0 | 0 | 0 | 0 | 0 | 0 | 0 | 0 | 0 | 0 | 0 | 0 | 0 | 0 | 0 | 0 | 0 | 0 | 0 | 0 | 0 | 0 | 0 | 0 | | | | | | |
| 792 | 00867 | 00791 | 8 | | 1 | 0 | 0 | 1 | 0 | 0 | 1 | 0 | 1 | 1 | 0 | 0 | 0 | 0 | 0 | 1 | 0 | 2 | 0 | 0 | 0 | 0 | 0 | 0 | 0 | | | | | | |
| 793 | 00868 | 00792 | 5 | | 1 | 0 | 0 | 0 | 0 | 0 | 1 | 0 | 0 | 0 | 0 | 0 | 2 | 0 | 0 | 0 | 0 | 0 | 0 | 0 | 0 | 0 | 1 | 0 | 0 | | | | | | |
| 794 | 00869 | 00793 | 5 | | 1 | 0 | 0 | 0 | 0 | 0 | 1 | 0 | 0 | 0 | 0 | 0 | 0 | 0 | 0 | 2 | 0 | 0 | 0 | 0 | 0 | 0 | 0 | 1 | 0 | | | | | | |
| 795 | 00870 | 00794 | 4 | | 1 | 0 | 2 | 0 | 0 | 0 | 0 | 0 | 0 | 0 | 0 | 0 | 0 | 0 | 0 | 0 | 0 | 1 | 0 | 0 | 0 | 0 | 0 | 0 | 0 | | | | | | |
| 796 | 00871 | 00795 | 5 | | 1 | 0 | 0 | 0 | 0 | 0 | 0 | 0 | 0 | 0 | 1 | 0 | 0 | 0 | 0 | 1 | 0 | 0 | 0 | 1 | 0 | 1 | 0 | 0 | 0 | | | | | | |
| 797 | 00872 | 00796 | 2 | | 1 | 0 | 0 | 0 | 0 | 0 | 0 | 0 | 0 | 0 | 0 | 0 | 0 | 0 | 0 | 0 | 0 | 0 | 0 | 0 | 1 | 0 | 0 | 0 | 0 | | | | | | |
| 798 | 00873 | 00797 | 2 | | 1 | 0 | 0 | 0 | 0 | 0 | 0 | 0 | 0 | 0 | 0 | 0 | 0 | 0 | 0 | 0 | 0 | 0 | 0 | 0 | 0 | 0 | 0 | 1 | 0 | | | | | | |
| 799 | 00874 | 00798 | 1 | | 1 | 0 | 0 | 0 | 0 | 0 | 0 | 0 | 0 | 0 | 0 | 0 | 0 | 0 | 0 | 0 | 0 | 0 | 0 | 0 | 0 | 0 | 0 | 0 | 0 | | | | | | |
| 800 | 00875 | 00799 | 21 | | 2 | 3 | 1 | 1 | 1 | 0 | 0 | 0 | 0 | 0 | 0 | 3 | 0 | 1 | 1 | 6 | 1 | 0 | 1 | 0 | 0 | 0 | 0 | 0 | 0 | | | | | | |
| 801 | 00876 | 00800 | 9 | | 1 | 0 | 0 | 0 | 1 | 0 | 0 | 0 | 1 | 1 | 0 | 1 | 1 | 0 | 1 | 0 | 0 | 0 | 0 | 0 | 0 | 0 | 2 | 0 | 0 | | | | | | |
| 802 | 00877 | 00801 | 6 | | 1 | 0 | 0 | 1 | 0 | 1 | 1 | 0 | 0 | 0 | 1 | 0 | 0 | 0 | 0 | 0 | 0 | 0 | 0 | 0 | 0 | 1 | 0 | 0 | 0 | | | | | | |
| 803 | 00878 | 00802 | 3 | | 1 | 0 | 0 | 0 | 0 | 1 | 1 | 0 | 0 | 0 | 0 | 0 | 0 | 0 | 0 | 0 | 0 | 0 | 0 | 0 | 0 | 0 | 0 | 0 | 0 | | | | | | |
| 804 | 00879 | 00803 | 5 | | 1 | 0 | 0 | 0 | 0 | 0 | 0 | 0 | 1 | 0 | 0 | 0 | 0 | 1 | 0 | 0 | 0 | 0 | 0 | 0 | 0 | 0 | 0 | 2 | 0 | | | | | | |
| 805 | 00880 | 00804 | 5 | | 2 | 0 | 1 | 0 | 0 | 0 | 0 | 0 | 0 | 0 | 0 | 0 | 0 | 0 | 0 | 0 | 1 | 0 | 0 | 0 | 0 | 1 | 0 | 0 | 0 | | | | | | |
| 806 | 00882 | 00805 | 20 | | 1 | 0 | 1 | 0 | 0 | 0 | 0 | 2 | 2 | 2 | 0 | 0 | 0 | 2 | 1 | 0 | 1 | 1 | 1 | 1 | 1 | 1 | 0 | 3 | 0 | | | | | | |
| 807 | 00883 | 00806 | 5 | | 1 | 1 | 1 | 0 | 0 | 0 | 0 | 0 | 0 | 0 | 0 | 0 | 0 | 0 | 1 | 0 | 0 | 0 | 0 | 0 | 0 | 1 | 0 | 0 | 0 | | | | | | |
| 808 | 00884 | 00807 | 3 | | 2 | 0 | 1 | 0 | 0 | 0 | 0 | 0 | 0 | 0 | 0 | 0 | 0 | 0 | 0 | 0 | 0 | 0 | 0 | 0 | 0 | 0 | 0 | 0 | 0 | | | | | | |
| 809 | 00885 | 00808 | 2 | | 2 | 0 | 0 | 0 | 0 | 0 | 0 | 0 | 0 | 0 | 0 | 0 | 0 | 0 | 0 | 0 | 0 | 0 | 0 | 0 | 0 | 0 | 0 | 0 | 0 | | | | | | |
| 810 | 00886 | 00809 | 3 | | 2 | 0 | 0 | 0 | 0 | 0 | 0 | 0 | 0 | 0 | 0 | 0 | 0 | 0 | 0 | 0 | 1 | 0 | 0 | 0 | 0 | 0 | 0 | 0 | 0 | | | | | | |
| 811 | 00887 | 00810 | 14 | | 2 | 1 | 1 | 0 | 1 | 1 | 0 | 0 | 0 | 0 | 0 | 0 | 0 | 0 | 1 | 1 | 4 | 1 | 0 | 0 | 0 | 1 | 0 | 0 | 0 | | | | | | |
| 812 | 00889 | 00811 | 17 | | 1 | 0 | 0 | 1 | 1 | 1 | 1 | 0 | 1 | 0 | 0 | 1 | 0 | 0 | 0 | 4 | 2 | 1 | 1 | 1 | 0 | 0 | 1 | 0 | 0 | | | | | | |
| 813 | 00890 | 00812 | 2 | | 1 | 0 | 0 | 0 | 0 | 1 | 0 | 0 | 0 | 0 | 0 | 0 | 0 | 0 | 0 | 0 | 0 | 0 | 0 | 0 | 0 | 0 | 0 | 0 | 0 | | | | | | |

Table 23

| | A | B | C | E | G | I | K | M | O | Q | S | U | W | Y | AA | AC | AE | AG | AI | AK | AM | AO | AQ | AS | AU | AW | AY | BA | BC | BE | BF | BG | BH | BI | BK |
|---|---|---|---|---|---|---|---|---|---|---|---|---|---|---|---|---|---|---|---|---|---|---|---|---|---|---|---|---|---|---|---|---|---|---|---|
| 814 | 00891 | 00813 | 5 | | 1 | 0 | 0 | 0 | 0 | 0 | 0 | 1 | 0 | 0 | 1 | 0 | 1 | 0 | 0 | 0 | 0 | 0 | 0 | 0 | 1 | 0 | 0 | 0 | 0 | | | | | | |
| 815 | 00892 | 00814 | 4 | | 1 | 0 | 0 | 0 | 0 | 0 | 0 | 0 | 0 | 0 | 0 | 0 | 0 | 2 | 0 | 0 | 0 | 1 | 0 | 0 | 0 | 0 | 0 | 0 | 0 | | | | | | |
| 816 | 00894 | 00815 | 1 | | 1 | 0 | 0 | 0 | 0 | 0 | 0 | 0 | 0 | 0 | 0 | 0 | 0 | 0 | 0 | 0 | 0 | 0 | 0 | 0 | 0 | 0 | 0 | 0 | 0 | | | | | | |
| 817 | 00895 | 00816 | 1 | | 1 | 0 | 0 | 0 | 0 | 0 | 0 | 0 | 0 | 0 | 0 | 0 | 0 | 0 | 0 | 0 | 0 | 0 | 0 | 0 | 0 | 0 | 0 | 0 | 0 | | | | | | |
| 818 | 00896 | 00817 | 1 | | 1 | 0 | 0 | 0 | 0 | 0 | 0 | 0 | 0 | 0 | 0 | 0 | 0 | 0 | 0 | 0 | 0 | 0 | 0 | 0 | 0 | 0 | 0 | 0 | 0 | | | | | | |
| 819 | 00897 | 00818 | 10 | | 2 | 1 | 0 | 0 | 0 | 2 | 0 | 1 | 1 | 0 | 0 | 1 | 0 | 0 | 0 | 1 | 0 | 0 | 0 | 0 | 0 | 0 | 1 | 0 | 0 | | | | | | |
| 820 | 00898 | 00819 | 3 | | 1 | 0 | 0 | 0 | 0 | 0 | 0 | 0 | 0 | 0 | 0 | 0 | 0 | 0 | 2 | 0 | 0 | 0 | 0 | 0 | 0 | 0 | 0 | 0 | 0 | | | | | | |
| 821 | 00900 | 00820 | 27 | | 1 | 0 | 2 | 0 | 0 | 0 | 1 | 1 | 0 | 0 | 0 | 6 | 1 | 1 | 3 | 0 | 2 | 4 | 2 | 2 | 0 | 0 | 0 | 1 | 0 | K00558 | 100 | 69 | 1 | 1528 | 1596 |
| 822 | 00903 | 00821 | 3 | | 1 | 0 | 0 | 0 | 0 | 0 | 1 | 0 | 0 | 0 | 0 | 0 | 0 | 0 | 0 | 0 | 1 | 0 | 0 | 0 | 0 | 0 | 0 | 0 | 0 | | | | | | |
| 823 | 00904 | 00822 | 8 | | 1 | 0 | 0 | 0 | 0 | 1 | 0 | 1 | 2 | 0 | 1 | 0 | 0 | 0 | 0 | 0 | 0 | 1 | 1 | 0 | 0 | 0 | 0 | 0 | 0 | | | | | | |
| 824 | 00905 | 00823 | 3 | | 1 | 0 | 0 | 0 | 0 | 0 | 0 | 0 | 0 | 0 | 0 | 0 | 0 | 0 | 0 | 0 | 0 | 1 | 0 | 0 | 0 | 1 | 0 | 0 | 0 | L16991 | 98.6 | 73 | 1 | 989 | 1250 |
| 825 | 00906 | 00824 | 2 | | 2 | 0 | 0 | 0 | 0 | 0 | 0 | 0 | 0 | 0 | 0 | 0 | 0 | 0 | 0 | 0 | 0 | 0 | 0 | 0 | 0 | 0 | 0 | 0 | 0 | | | | | | |
| 826 | 00908 | 00825 | 3 | | 1 | 0 | 0 | 0 | 0 | 0 | 0 | 0 | 0 | 0 | 0 | 0 | 0 | 0 | 0 | 1 | 0 | 0 | 1 | 0 | 0 | 0 | 0 | 0 | 0 | | | | | | |
| 827 | 00910 | 00826 | 2 | | 1 | 1 | 0 | 0 | 0 | 0 | 0 | 0 | 0 | 0 | 0 | 0 | 0 | 0 | 0 | 0 | 0 | 0 | 0 | 0 | 0 | 0 | 0 | 0 | 0 | | | | | | |
| 828 | 00911 | 00827 | 9 | | 1 | 0 | 0 | 0 | 0 | 1 | 0 | 0 | 0 | 0 | 0 | 1 | 0 | 2 | 2 | 0 | 0 | 0 | 1 | 1 | 0 | 0 | 0 | 0 | 0 | | | | | | |
| 829 | 00912 | 00828 | 1 | | 1 | 0 | 0 | 0 | 0 | 0 | 0 | 0 | 0 | 0 | 0 | 0 | 0 | 0 | 0 | 0 | 0 | 0 | 0 | 0 | 0 | 0 | 0 | 0 | 0 | | | | | | |
| 830 | 00913 | 00829 | 5 | | 1 | 0 | 0 | 0 | 0 | 0 | 0 | 0 | 0 | 0 | 0 | 0 | 0 | 0 | 0 | 0 | 0 | 0 | 1 | 1 | 0 | 2 | 0 | 0 | 0 | | | | | | |
| 831 | 00914 | 00830 | 2 | | 1 | 0 | 0 | 0 | 0 | 0 | 0 | 0 | 0 | 0 | 0 | 0 | 0 | 0 | 0 | 1 | 0 | 0 | 0 | 0 | 0 | 0 | 0 | 0 | 0 | | | | | | |
| 832 | 00915 | 00831 | 2 | | 1 | 0 | 0 | 0 | 0 | 0 | 0 | 0 | 0 | 0 | 0 | 0 | 1 | 0 | 0 | 0 | 0 | 0 | 0 | 0 | 0 | 0 | 0 | 0 | 0 | | | | | | |
| 833 | 00917 | 00832 | 43 | | 3 | 1 | 3 | 0 | 3 | 3 | 0 | 2 | 1 | 1 | 5 | 6 | 3 | 0 | 0 | 0 | 3 | 3 | 1 | 1 | 1 | 1 | 0 | 2 | 0 | | | | | | |
| 834 | 00918 | 00833 | 1 | | 1 | 0 | 0 | 0 | 0 | 0 | 0 | 0 | 0 | 0 | 0 | 0 | 0 | 0 | 0 | 0 | 0 | 0 | 0 | 0 | 0 | 0 | 0 | 0 | 0 | | | | | | |
| 835 | 00919 | 00834 | 7 | | 1 | 1 | 0 | 0 | 1 | 0 | 0 | 0 | 0 | 0 | 0 | 0 | 0 | 0 | 2 | 0 | 0 | 0 | 0 | 0 | 0 | 0 | 2 | 0 | 0 | L10911 | 97.2 | 109 | 1 | 1711 | 2488 |
| 836 | 00920 | 00835 | 4 | | 1 | 0 | 0 | 0 | 0 | 0 | 0 | 0 | 2 | 0 | 0 | 1 | 0 | 0 | 0 | 0 | 0 | 0 | 0 | 0 | 0 | 0 | 0 | 0 | 0 | | | | | | |
| 837 | 00922 | 00836 | 9 | | 2 | 0 | 0 | 0 | 0 | 0 | 0 | 0 | 0 | 0 | 0 | 0 | 0 | 0 | 4 | 2 | 1 | 0 | 0 | 0 | 0 | 0 | 0 | 0 | 0 | | | | | | |
| 838 | 00924 | 00837 | 1 | | 1 | 0 | 0 | 0 | 0 | 0 | 0 | 0 | 0 | 0 | 0 | 0 | 0 | 0 | 0 | 0 | 0 | 0 | 0 | 0 | 0 | 0 | 0 | 0 | 0 | | | | | | |
| 839 | 00925 | 00838 | 1 | | 1 | 0 | 0 | 0 | 0 | 0 | 0 | 0 | 0 | 0 | 0 | 0 | 0 | 0 | 0 | 0 | 0 | 0 | 0 | 0 | 0 | 0 | 0 | 0 | 0 | | | | | | |
| 840 | 00926 | 00839 | 3 | | 1 | 0 | 0 | 0 | 0 | 0 | 0 | 0 | 0 | 0 | 0 | 0 | 0 | 0 | 0 | 0 | 0 | 0 | 0 | 0 | 1 | 0 | 0 | 1 | 0 | | | | | | |
| 841 | 00929 | 00840 | 1 | | 1 | 0 | 0 | 0 | 0 | 0 | 0 | 0 | 0 | 0 | 0 | 0 | 0 | 0 | 0 | 0 | 0 | 0 | 0 | 0 | 0 | 0 | 0 | 0 | 0 | | | | | | |
| 842 | 00930 | 00841 | 4 | | 1 | 0 | 0 | 0 | 0 | 0 | 0 | 0 | 0 | 0 | 0 | 0 | 1 | 0 | 0 | 0 | 0 | 0 | 1 | 0 | 0 | 0 | 0 | 1 | 0 | | | | | | |
| 843 | 00931 | 00842 | 1 | | 1 | 0 | 0 | 0 | 0 | 0 | 0 | 0 | 0 | 0 | 0 | 0 | 0 | 0 | 0 | 0 | 0 | 0 | 0 | 0 | 0 | 0 | 0 | 0 | 0 | | | | | | |
| 844 | 00932 | 00843 | 16 | | 1 | 1 | 0 | 0 | 0 | 0 | 1 | 0 | 0 | 0 | 3 | 0 | 1 | 4 | 1 | 1 | 0 | 0 | 3 | 0 | 0 | 0 | 0 | 0 | 0 | | | | | | |
| 845 | 00934 | 00844 | 34 | | 3 | 1 | 1 | 0 | 1 | 5 | 0 | 2 | 3 | 0 | 0 | 3 | 2 | 3 | 0 | 0 | 0 | 0 | 2 | 0 | 6 | 0 | 1 | 1 | 0 | X13923 | 100 | 48 | 1 | 392 | 439 |
| 846 | 00935 | 00845 | 6 | | 1 | 2 | 0 | 0 | 0 | 0 | 0 | 0 | 0 | 0 | 0 | 0 | 0 | 0 | 1 | 0 | 0 | 0 | 0 | 1 | 0 | 0 | 1 | 0 | 0 | L13773 | 94.2 | 52 | 1 | 9332 | 9390 |
| 847 | 00936 | 00846 | 8 | | 2 | 1 | 0 | 0 | 0 | 0 | 1 | 0 | 0 | 0 | 0 | 1 | 0 | 0 | 0 | 0 | 0 | 0 | 0 | 0 | 2 | 0 | 1 | 0 | 0 | | | | | | |
| 848 | 00937 | 00847 | 62 | | 1 | 5 | 0 | 0 | 0 | 0 | 12 | 0 | 6 | 4 | 0 | 2 | 0 | 0 | 2 | 2 | 1 | 0 | 3 | 2 | 5 | 0 | 15 | 0 | 2 | | | | | | |
| 849 | 00938 | 00848 | 8 | | 1 | 0 | 2 | 0 | 0 | 0 | 1 | 0 | 0 | 0 | 0 | 0 | 0 | 2 | 0 | 0 | 0 | 0 | 0 | 0 | 0 | 1 | 0 | 1 | 0 | | | | | | |

Table 24

| | A | B | C | E | G | I | K | M | O | Q | S | U | W | Y | AA | AC | AE | AG | AI | AK | AM | AO | AQ | AS | AU | AW | AY | BA | BC | BE | BF | BG | BH | BI | BK |
|---|---|---|---|---|---|---|---|---|---|---|---|---|---|---|---|---|---|---|---|---|---|---|---|---|---|---|---|---|---|---|---|---|---|---|---|
| 850 | 00955 | 00849 | 6 | | 3 | 0 | 1 | 0 | 1 | 0 | 0 | 0 | 0 | 0 | 0 | 0 | 0 | 0 | 0 | 0 | 0 | 1 | 0 | 0 | 0 | 0 | 0 | 0 | 0 | | | | | | |
| 851 | 00972 | 00850 | 1 | | 0 | 1 | 0 | 0 | 0 | 0 | 0 | 0 | 0 | 0 | 0 | 0 | 0 | 0 | 0 | 0 | 0 | 0 | 0 | 0 | 0 | 0 | 0 | 0 | 0 | | | | | | |
| 852 | 00973 | 00851 | 2 | | 0 | 1 | 0 | 0 | 0 | 0 | 0 | 0 | 0 | 0 | 0 | 0 | 0 | 0 | 0 | 0 | 0 | 0 | 0 | 0 | 0 | 0 | 1 | 0 | 0 | | | | | | |
| 853 | 00974 | 00852 | 59 | | 0 | 2 | 0 | 1 | 0 | 0 | 0 | 0 | 0 | 0 | 1 | 0 | 5 | 6 | 0 | 5 | 16 | 9 | 5 | 1 | 6 | 2 | 0 | 0 | 0 | | | | | | |
| 854 | 00976 | 00853 | 1 | | 0 | 1 | 0 | 0 | 0 | 0 | 0 | 0 | 0 | 0 | 0 | 0 | 0 | 0 | 0 | 0 | 0 | 0 | 0 | 0 | 0 | 0 | 0 | 0 | 0 | | | | | | |
| 855 | 00977 | 00854 | 5 | | 0 | 1 | 1 | 0 | 0 | 0 | 0 | 0 | 1 | 0 | 0 | 0 | 0 | 1 | 1 | 0 | 0 | 0 | 0 | 0 | 0 | 0 | 0 | 0 | 0 | | | | | | |
| 856 | 00978 | 00855 | 1 | | 0 | 1 | 0 | 0 | 0 | 0 | 0 | 0 | 0 | 0 | 0 | 0 | 0 | 0 | 0 | 0 | 0 | 0 | 0 | 0 | 0 | 0 | 0 | 0 | 0 | | | | | | |
| 857 | 00979 | 00856 | 3 | | 0 | 1 | 0 | 0 | 0 | 0 | 0 | 0 | 1 | 0 | 0 | 0 | 0 | 0 | 1 | 0 | 0 | 0 | 0 | 0 | 0 | 0 | 0 | 0 | 0 | | | | | | |
| 858 | 00980 | 00857 | 12 | | 0 | 2 | 0 | 0 | 0 | 0 | 0 | 0 | 0 | 1 | 0 | 3 | 1 | 0 | 1 | 0 | 0 | 0 | 2 | 0 | 0 | 0 | 0 | 2 | 0 | | | | | | |
| 859 | 00981 | 00858 | 1 | | 0 | 1 | 0 | 0 | 0 | 0 | 0 | 0 | 0 | 0 | 0 | 0 | 0 | 0 | 0 | 0 | 0 | 0 | 0 | 0 | 0 | 0 | 0 | 0 | 0 | | | | | | |
| 860 | 00982 | 00859 | 1 | | 0 | 1 | 0 | 0 | 0 | 0 | 0 | 0 | 0 | 0 | 0 | 0 | 0 | 0 | 0 | 0 | 0 | 0 | 0 | 0 | 0 | 0 | 0 | 0 | 0 | | | | | | |
| 861 | 00983 | 00860 | 1 | | 0 | 1 | 0 | 0 | 0 | 0 | 0 | 0 | 0 | 0 | 0 | 0 | 0 | 0 | 0 | 0 | 0 | 0 | 0 | 0 | 0 | 0 | 0 | 0 | 0 | | | | | | |
| 862 | 00984 | 00861 | 1 | | 0 | 1 | 0 | 0 | 0 | 0 | 0 | 0 | 0 | 0 | 0 | 0 | 0 | 0 | 0 | 0 | 0 | 0 | 0 | 0 | 0 | 0 | 0 | 0 | 0 | J04739 | 100 | 136 | 1 | 1068 | 1813 |
| 863 | 00985 | 00862 | 2 | | 0 | 2 | 0 | 0 | 0 | 0 | 0 | 0 | 0 | 0 | 0 | 0 | 0 | 0 | 0 | 0 | 0 | 0 | 0 | 0 | 0 | 0 | 0 | 0 | 0 | | | | | | |
| 864 | 00986 | 00863 | 1 | | 0 | 1 | 0 | 0 | 0 | 0 | 0 | 0 | 0 | 0 | 0 | 0 | 0 | 0 | 0 | 0 | 0 | 0 | 0 | 0 | 0 | 0 | 0 | 0 | 0 | | | | | | |
| 865 | 00987 | 00864 | 2 | | 0 | 1 | 0 | 0 | 0 | 0 | 0 | 0 | 0 | 0 | 1 | 0 | 0 | 0 | 0 | 0 | 0 | 0 | 0 | 0 | 0 | 0 | 0 | 0 | 0 | | | | | | |
| 866 | 00988 | 00865 | 1 | | 0 | 1 | 0 | 0 | 0 | 0 | 0 | 0 | 0 | 0 | 0 | 0 | 0 | 0 | 0 | 0 | 0 | 0 | 0 | 0 | 0 | 0 | 0 | 0 | 0 | | | | | | |
| 867 | 00989 | 00866 | 1 | | 0 | 1 | 0 | 0 | 0 | 0 | 0 | 0 | 0 | 0 | 0 | 0 | 0 | 0 | 0 | 0 | 0 | 0 | 0 | 0 | 0 | 0 | 0 | 0 | 0 | | | | | | |
| 868 | 00990 | 00867 | 2 | | 0 | 1 | 0 | 0 | 0 | 0 | 0 | 0 | 0 | 0 | 0 | 0 | 0 | 0 | 0 | 0 | 0 | 0 | 0 | 0 | 0 | 0 | 1 | 0 | 0 | | | | | | |
| 869 | 00991 | 00868 | 1 | | 0 | 1 | 0 | 0 | 0 | 0 | 0 | 0 | 0 | 0 | 0 | 0 | 0 | 0 | 0 | 0 | 0 | 0 | 0 | 0 | 0 | 0 | 0 | 0 | 0 | | | | | | |
| 870 | 00992 | 00869 | 3 | | 0 | 1 | 0 | 0 | 0 | 0 | 0 | 1 | 0 | 0 | 0 | 0 | 0 | 0 | 0 | 0 | 0 | 1 | 0 | 0 | 0 | 0 | 0 | 0 | 0 | | | | | | |
| 871 | 00993 | 00870 | 2 | | 0 | 1 | 0 | 0 | 0 | 0 | 0 | 0 | 0 | 0 | 0 | 1 | 0 | 0 | 0 | 0 | 0 | 0 | 0 | 0 | 0 | 0 | 0 | 0 | 0 | | | | | | |
| 872 | 00994 | 00871 | 19 | | 0 | 2 | 1 | 0 | 0 | 0 | 0 | 1 | 0 | 0 | 0 | 1 | 1 | 0 | 1 | 0 | 0 | 0 | 5 | 1 | 0 | 1 | 5 | 0 | 0 | | | | | | |
| 873 | 00995 | 00872 | 23 | | 0 | 3 | 1 | 1 | 0 | 1 | 1 | 1 | 0 | 0 | 2 | 0 | 1 | 3 | 0 | 2 | 0 | 0 | 1 | 3 | 0 | 1 | 1 | 1 | 0 | | | | | | |
| 874 | 00996 | 00873 | 1 | | 0 | 1 | 0 | 0 | 0 | 0 | 0 | 0 | 0 | 0 | 0 | 0 | 0 | 0 | 0 | 0 | 0 | 0 | 0 | 0 | 0 | 0 | 0 | 0 | 0 | | | | | | |
| 875 | 00997 | 00874 | 2 | | 0 | 1 | 0 | 0 | 0 | 0 | 0 | 0 | 0 | 0 | 0 | 1 | 0 | 0 | 0 | 0 | 0 | 0 | 0 | 0 | 0 | 0 | 0 | 0 | 0 | | | | | | |
| 876 | 00998 | 00875 | 1 | | 0 | 1 | 0 | 0 | 0 | 0 | 0 | 0 | 0 | 0 | 0 | 0 | 0 | 0 | 0 | 0 | 0 | 0 | 0 | 0 | 0 | 0 | 0 | 0 | 0 | | | | | | |
| 877 | 00999 | 00876 | 1 | | 0 | 1 | 0 | 0 | 0 | 0 | 0 | 0 | 0 | 0 | 0 | 0 | 0 | 0 | 0 | 0 | 0 | 0 | 0 | 0 | 0 | 0 | 0 | 0 | 0 | | | | | | |
| 878 | 01000 | 00877 | 3 | | 0 | 2 | 0 | 0 | 0 | 0 | 0 | 0 | 0 | 0 | 0 | 0 | 0 | 0 | 0 | 0 | 0 | 0 | 0 | 0 | 0 | 0 | 1 | 0 | 0 | | | | | | |
| 879 | 01001 | 00878 | 1 | | 0 | 1 | 0 | 0 | 0 | 0 | 0 | 0 | 0 | 0 | 0 | 0 | 0 | 0 | 0 | 0 | 0 | 0 | 0 | 0 | 0 | 0 | 0 | 0 | 0 | | | | | | |
| 880 | 01002 | 00879 | 2 | | 0 | 1 | 0 | 0 | 0 | 0 | 0 | 0 | 0 | 1 | 0 | 0 | 0 | 0 | 0 | 0 | 0 | 0 | 0 | 0 | 0 | 0 | 0 | 0 | 0 | | | | | | |
| 881 | 01003 | 00880 | 1 | | 0 | 1 | 0 | 0 | 0 | 0 | 0 | 0 | 0 | 0 | 0 | 0 | 0 | 0 | 0 | 0 | 0 | 0 | 0 | 0 | 0 | 0 | 0 | 0 | 0 | | | | | | |
| 882 | 01004 | 00881 | 4 | | 0 | 1 | 0 | 0 | 0 | 0 | 0 | 0 | 0 | 0 | 2 | 0 | 1 | 0 | 0 | 0 | 0 | 0 | 0 | 0 | 0 | 0 | 0 | 0 | 0 | | | | | | |
| 883 | 01005 | 00882 | 2 | | 0 | 1 | 0 | 0 | 0 | 0 | 0 | 0 | 0 | 0 | 0 | 0 | 0 | 0 | 0 | 0 | 0 | 0 | 0 | 0 | 0 | 1 | 0 | 0 | 0 | | | | | | |
| 884 | 01006 | 00883 | 3 | | 0 | 1 | 0 | 0 | 0 | 0 | 0 | 0 | 0 | 0 | 1 | 0 | 0 | 0 | 0 | 1 | 0 | 0 | 0 | 0 | 0 | 0 | 0 | 0 | 0 | | | | | | |
| 885 | 01007 | 00884 | 33 | | 0 | 3 | 0 | 0 | 0 | 0 | 1 | 1 | 0 | 2 | 2 | 1 | 0 | 5 | 1 | 1 | 2 | 2 | 1 | 3 | 0 | 0 | 2 | 5 | 1 | | | | | | |

Table 25

| | A | B | C | E | G | I | K | M | O | Q | S | U | W | Y | AA | AC | AE | AG | AI | AK | AM | AO | AQ | AS | AU | AW | AY | BA | BC | BE | BF | BG | BH | BI | BK |
|---|---|---|---|---|---|---|---|---|---|---|---|---|---|---|---|---|---|---|---|---|---|---|---|---|---|---|---|---|---|---|---|---|---|---|---|
| 886 | 01009 | 00885 | 2 | | 0 | 2 | 0 | 0 | 0 | 0 | 0 | 0 | 0 | 0 | 0 | 0 | 0 | 0 | 0 | 0 | 0 | 0 | 0 | 0 | 0 | 0 | 0 | 0 | 0 | | | | | | |
| 887 | 01010 | 00886 | 1 | | 0 | 1 | 0 | 0 | 0 | 0 | 0 | 0 | 0 | 0 | 0 | 0 | 0 | 0 | 0 | 0 | 0 | 0 | 0 | 0 | 0 | 0 | 0 | 0 | 0 | J05021 | 96.1 | 438 | 1 | 2494 | 2930 |
| 888 | 01011 | 00887 | 3 | | 0 | 1 | 0 | 0 | 0 | 0 | 0 | 0 | 0 | 0 | 0 | 0 | 0 | 0 | 0 | 0 | 0 | 0 | 0 | 1 | 1 | 0 | 0 | 0 | 0 | | | | | | |
| 889 | 01012 | 00888 | 3 | | 0 | 2 | 0 | 0 | 0 | 0 | 0 | 0 | 0 | 0 | 0 | 0 | 0 | 0 | 0 | 0 | 0 | 0 | 0 | 0 | 0 | 0 | 0 | 0 | 1 | D13748 | 94.8 | 346 | 1 | 601 | 1383 |
| 890 | 01013 | 00889 | 5 | | 0 | 1 | 0 | 0 | 0 | 1 | 0 | 0 | 0 | 2 | 0 | 0 | 0 | 1 | 0 | 0 | 0 | 0 | 0 | 0 | 0 | 0 | 0 | 0 | 0 | | | | | | |
| 891 | 01015 | 00890 | 1 | | 0 | 1 | 0 | 0 | 0 | 0 | 0 | 0 | 0 | 0 | 0 | 0 | 0 | 0 | 0 | 0 | 0 | 0 | 0 | 0 | 0 | 0 | 0 | 0 | 0 | | | | | | |
| 892 | 01016 | 00891 | 1 | | 0 | 1 | 0 | 0 | 0 | 0 | 0 | 0 | 0 | 0 | 0 | 0 | 0 | 0 | 0 | 0 | 0 | 0 | 0 | 0 | 0 | 0 | 0 | 0 | 0 | | | | | | |
| 893 | 01017 | 00892 | 1 | | 0 | 1 | 0 | 0 | 0 | 0 | 0 | 0 | 0 | 0 | 0 | 0 | 0 | 0 | 0 | 0 | 0 | 0 | 0 | 0 | 0 | 0 | 0 | 0 | 0 | | | | | | |
| 894 | 01018 | 00893 | 6 | | 0 | 1 | 0 | 0 | 0 | 0 | 1 | 0 | 1 | 0 | 0 | 0 | 0 | 1 | 0 | 1 | 0 | 0 | 0 | 0 | 1 | 0 | 0 | 0 | 0 | | | | | | |
| 895 | 01019 | 00894 | 3 | | 0 | 1 | 0 | 0 | 0 | 0 | 0 | 0 | 0 | 1 | 0 | 0 | 0 | 0 | 0 | 1 | 0 | 0 | 0 | 0 | 0 | 0 | 0 | 0 | 0 | | | | | | |
| 896 | 01020 | 00895 | 8 | | 0 | 1 | 0 | 0 | 0 | 1 | 0 | 1 | 0 | 1 | 0 | 0 | 0 | 0 | 1 | 0 | 0 | 0 | 1 | 1 | 0 | 0 | 0 | 1 | 0 | D00022 | 98.7 | 449 | 1 | 1353 | 1807 |
| 897 | 01021 | 00896 | 9 | | 0 | 1 | 0 | 0 | 0 | 0 | 1 | 0 | 1 | 0 | 0 | 0 | 1 | 1 | 0 | 2 | 1 | 0 | 0 | 0 | 1 | 0 | 0 | 0 | 0 | D13635 | 93.5 | 459 | 1 | 4716 | 5160 |
| 898 | 01023 | 00897 | 4 | | 0 | 1 | 0 | 0 | 0 | 0 | 0 | 0 | 0 | 0 | 0 | 0 | 1 | 0 | 0 | 0 | 0 | 0 | 0 | 0 | 0 | 0 | 2 | 0 | 0 | | | | | | |
| 899 | 01024 | 00898 | 6 | | 0 | 4 | 2 | 0 | 0 | 0 | 0 | 0 | 0 | 0 | 0 | 0 | 0 | 0 | 0 | 0 | 0 | 0 | 0 | 0 | 0 | 0 | 0 | 0 | 0 | M15395 | 95.3 | 451 | 1 | 2250 | 2776 |
| 900 | 01025 | 00899 | 1 | | 0 | 1 | 0 | 0 | 0 | 0 | 0 | 0 | 0 | 0 | 0 | 0 | 0 | 0 | 0 | 0 | 0 | 0 | 0 | 0 | 0 | 0 | 0 | 0 | 0 | | | | | | |
| 901 | 01026 | 00900 | 3 | | 0 | 1 | 0 | 0 | 0 | 0 | 0 | 0 | 0 | 0 | 0 | 0 | 0 | 0 | 0 | 0 | 1 | 0 | 0 | 0 | 0 | 1 | 0 | 0 | 0 | | | | | | |
| 902 | 01027 | 00901 | 4 | | 0 | 2 | 0 | 0 | 0 | 0 | 0 | 0 | 0 | 0 | 0 | 0 | 0 | 0 | 0 | 0 | 0 | 0 | 0 | 0 | 0 | 0 | 1 | 1 | 0 | | | | | | |
| 903 | 01028 | 00902 | 1 | | 0 | 1 | 0 | 0 | 0 | 0 | 0 | 0 | 0 | 0 | 0 | 0 | 0 | 0 | 0 | 0 | 0 | 0 | 0 | 0 | 0 | 0 | 0 | 0 | 0 | | | | | | |
| 904 | 01029 | 00903 | 13 | | 0 | 2 | 1 | 0 | 0 | 0 | 0 | 0 | 1 | 0 | 0 | 4 | 0 | 1 | 0 | 0 | 0 | 1 | 1 | 2 | 0 | 0 | 0 | 0 | 0 | D00422 | 92.4 | 327 | 1 | 2006 | 2734 |
| 905 | 01030 | 00904 | 9 | | 0 | 2 | 1 | 0 | 0 | 0 | 1 | 2 | 0 | 1 | 0 | 0 | 0 | 0 | 0 | 1 | 0 | 0 | 0 | 0 | 0 | 0 | 1 | 0 | 0 | X54304 | 94.4 | 448 | 1 | 505 | 944 |
| 906 | 01032 | 00905 | 14 | | 0 | 2 | 0 | 1 | 0 | 0 | 0 | 0 | 0 | 1 | 0 | 0 | 0 | 0 | 0 | 0 | 4 | 0 | 0 | 0 | 1 | 2 | 1 | 2 | 0 | Y00345 | 92.6 | 443 | 1 | 2409 | 2848 |
| 907 | 01033 | 00906 | 3 | | 0 | 1 | 0 | 0 | 0 | 0 | 2 | 0 | 0 | 0 | 0 | 0 | 0 | 0 | 0 | 0 | 0 | 0 | 0 | 0 | 0 | 0 | 0 | 0 | 0 | | | | | | |
| 908 | 01034 | 00907 | 1 | | 0 | 1 | 0 | 0 | 0 | 0 | 0 | 0 | 0 | 0 | 0 | 0 | 0 | 0 | 0 | 0 | 0 | 0 | 0 | 0 | 0 | 0 | 0 | 0 | 0 | | | | | | |
| 909 | 01035 | 00908 | 1 | | 0 | 1 | 0 | 0 | 0 | 0 | 0 | 0 | 0 | 0 | 0 | 0 | 0 | 0 | 0 | 0 | 0 | 0 | 0 | 0 | 0 | 0 | 0 | 0 | 0 | | | | | | |
| 910 | 01036 | 00909 | 1 | | 0 | 1 | 0 | 0 | 0 | 0 | 0 | 0 | 0 | 0 | 0 | 0 | 0 | 0 | 0 | 0 | 0 | 0 | 0 | 0 | 0 | 0 | 0 | 0 | 0 | | | | | | |
| 911 | 01037 | 00910 | 1 | | 0 | 1 | 0 | 0 | 0 | 0 | 0 | 0 | 0 | 0 | 0 | 0 | 0 | 0 | 0 | 0 | 0 | 0 | 0 | 0 | 0 | 0 | 0 | 0 | 0 | | | | | | |
| 912 | 01038 | 00911 | 1 | | 0 | 1 | 0 | 0 | 0 | 0 | 0 | 0 | 0 | 0 | 0 | 0 | 0 | 0 | 0 | 0 | 0 | 0 | 0 | 0 | 0 | 0 | 0 | 0 | 0 | | | | | | |
| 913 | 01039 | 00912 | 3 | | 0 | 1 | 0 | 0 | 0 | 0 | 0 | 0 | 0 | 0 | 0 | 0 | 0 | 0 | 1 | 0 | 0 | 1 | 0 | 0 | 0 | 0 | 0 | 0 | 0 | | | | | | |
| 914 | 01040 | 00913 | 1 | | 0 | 1 | 0 | 0 | 0 | 0 | 0 | 0 | 0 | 0 | 0 | 0 | 0 | 0 | 0 | 0 | 0 | 0 | 0 | 0 | 0 | 0 | 0 | 0 | 0 | | | | | | |
| 915 | 01041 | 00914 | 3 | | 0 | 1 | 0 | 0 | 0 | 0 | 0 | 0 | 0 | 0 | 0 | 0 | 0 | 0 | 0 | 0 | 1 | 0 | 0 | 0 | 0 | 1 | 0 | 0 | 0 | | | | | | |
| 916 | 01042 | 00915 | 1 | | 0 | 1 | 0 | 0 | 0 | 0 | 0 | 0 | 0 | 0 | 0 | 0 | 0 | 0 | 0 | 0 | 0 | 0 | 0 | 0 | 0 | 0 | 0 | 0 | 0 | | | | | | |
| 917 | 01043 | 00916 | 1 | | 0 | 1 | 0 | 0 | 0 | 0 | 0 | 0 | 0 | 0 | 0 | 0 | 0 | 0 | 0 | 0 | 0 | 0 | 0 | 0 | 0 | 0 | 0 | 0 | 0 | | | | | | |
| 918 | 01044 | 00917 | 2 | | 0 | 1 | 0 | 0 | 0 | 0 | 0 | 0 | 0 | 0 | 1 | 0 | 0 | 0 | 0 | 0 | 0 | 0 | 0 | 0 | 0 | 0 | 0 | 0 | 0 | | | | | | |
| 919 | 01045 | 00918 | 4 | | 0 | 1 | 0 | 0 | 0 | 0 | 0 | 0 | 0 | 0 | 0 | 1 | 0 | 0 | 0 | 0 | 0 | 0 | 0 | 0 | 0 | 0 | 0 | 2 | 0 | X64229 | 97.5 | 355 | 1 | 2345 | 2699 |
| 920 | 01046 | 00919 | 2 | | 0 | 1 | 0 | 0 | 0 | 0 | 0 | 0 | 0 | 0 | 0 | 0 | 0 | 0 | 0 | 0 | 0 | 0 | 0 | 0 | 0 | 1 | 0 | 0 | 0 | M82809 | 95.4 | 410 | 1 | 1572 | 1976 |
| 921 | 01047 | 00920 | 1 | | 0 | 1 | 0 | 0 | 0 | 0 | 0 | 0 | 0 | 0 | 0 | 0 | 0 | 0 | 0 | 0 | 0 | 0 | 0 | 0 | 0 | 0 | 0 | 0 | 0 | | | | | | |

Table 26

| | A | B | C | E | G | I | K | M | O | Q | S | U | W | Y | AA | AC | AE | AG | AI | AK | AM | AO | AQ | AS | AU | AW | AY | BA | BC | BE | BF | BG | BH | BI | BK |
|---|---|---|---|---|---|---|---|---|---|---|---|---|---|---|---|---|---|---|---|---|---|---|---|---|---|---|---|---|---|---|---|---|---|---|---|
| 922 | 01048 | 00921 | 5 | | 0 | 1 | 0 | 0 | 0 | 0 | 0 | 0 | 0 | 0 | 0 | 0 | 0 | 1 | 1 | 1 | 0 | 0 | 0 | 0 | 0 | 0 | 1 | 0 | 0 | M84694 | 96.9 | 382 | 1 | 708 | 1089 |
| 923 | 01049 | 00922 | 2 | | 0 | 1 | 0 | 0 | 0 | 0 | 0 | 1 | 0 | 0 | 0 | 0 | 0 | 0 | 0 | 0 | 0 | 0 | 0 | 0 | 0 | 0 | 0 | 0 | 0 | | | | | | |
| 924 | 01051 | 00923 | 3 | | 0 | 1 | 0 | 0 | 0 | 0 | 0 | 0 | 0 | 0 | 0 | 0 | 0 | 0 | 0 | 0 | 1 | 0 | 0 | 0 | 0 | 0 | 0 | 1 | 0 | | | | | | |
| 925 | 01052 | 00924 | 3 | | 0 | 1 | 0 | 0 | 0 | 0 | 0 | 0 | 0 | 0 | 0 | 0 | 0 | 0 | 0 | 0 | 1 | 0 | 0 | 0 | 0 | 0 | 1 | 0 | 0 | | | | | | |
| 926 | 01053 | 00925 | 1 | | 0 | 1 | 0 | 0 | 0 | 0 | 0 | 0 | 0 | 0 | 0 | 0 | 0 | 0 | 0 | 0 | 0 | 0 | 0 | 0 | 0 | 0 | 0 | 0 | 0 | M92423 | 90.3 | 380 | 24 | 3711 | 4221 |
| 927 | 01054 | 00926 | 2 | | 0 | 1 | 0 | 0 | 0 | 0 | 0 | 0 | 0 | 1 | 0 | 0 | 0 | 0 | 0 | 0 | 0 | 0 | 0 | 0 | 0 | 0 | 0 | 0 | 0 | | | | | | |
| 928 | 01055 | 00927 | 1 | | 0 | 1 | 0 | 0 | 0 | 0 | 0 | 0 | 0 | 0 | 0 | 0 | 0 | 0 | 0 | 0 | 0 | 0 | 0 | 0 | 0 | 0 | 0 | 0 | 0 | | | | | | |
| 929 | 01057 | 00928 | 3 | | 0 | 1 | 0 | 0 | 0 | 0 | 0 | 0 | 0 | 0 | 0 | 0 | 0 | 0 | 0 | 0 | 0 | 0 | 0 | 0 | 0 | 1 | 1 | 0 | 0 | | | | | | |
| 930 | 01061 | 00929 | 1 | | 0 | 1 | 0 | 0 | 0 | 0 | 0 | 0 | 0 | 0 | 0 | 0 | 0 | 0 | 0 | 0 | 0 | 0 | 0 | 0 | 0 | 0 | 0 | 0 | 0 | M74782 | 98.3 | 347 | 1 | 1115 | 1460 |
| 931 | 01062 | 00930 | 8 | | 0 | 1 | 1 | 0 | 0 | 0 | 0 | 0 | 1 | 0 | 0 | 1 | 0 | 0 | 0 | 0 | 2 | 1 | 0 | 1 | 0 | 0 | 0 | 0 | 0 | V00599 | 97.2 | 394 | 1 | 1011 | 1441 |
| 932 | 01063 | 00931 | 1 | | 0 | 1 | 0 | 0 | 0 | 0 | 0 | 0 | 0 | 0 | 0 | 0 | 0 | 0 | 0 | 0 | 0 | 0 | 0 | 0 | 0 | 0 | 0 | 0 | 0 | | | | | | |
| 933 | 01064 | 00932 | 1 | | 0 | 1 | 0 | 0 | 0 | 0 | 0 | 0 | 0 | 0 | 0 | 0 | 0 | 0 | 0 | 0 | 0 | 0 | 0 | 0 | 0 | 0 | 0 | 0 | 0 | | | | | | |
| 934 | 01065 | 00933 | 1 | | 0 | 1 | 0 | 0 | 0 | 0 | 0 | 0 | 0 | 0 | 0 | 0 | 0 | 0 | 0 | 0 | 0 | 0 | 0 | 0 | 0 | 0 | 0 | 0 | 0 | | | | | | |
| 935 | 01066 | 00934 | 2 | | 0 | 1 | 0 | 0 | 0 | 0 | 0 | 0 | 1 | 0 | 0 | 0 | 0 | 0 | 0 | 0 | 0 | 0 | 0 | 0 | 0 | 0 | 0 | 0 | 0 | | | | | | |
| 936 | 01067 | 00935 | 1 | | 0 | 1 | 0 | 0 | 0 | 0 | 0 | 0 | 0 | 0 | 0 | 0 | 0 | 0 | 0 | 0 | 0 | 0 | 0 | 0 | 0 | 0 | 0 | 0 | 0 | | | | | | |
| 937 | 01068 | 00936 | 1 | | 0 | 1 | 0 | 0 | 0 | 0 | 0 | 0 | 0 | 0 | 0 | 0 | 0 | 0 | 0 | 0 | 0 | 0 | 0 | 0 | 0 | 0 | 0 | 0 | 0 | | | | | | |
| 938 | 01069 | 00937 | 2 | | 0 | 1 | 0 | 0 | 0 | 0 | 0 | 0 | 0 | 0 | 0 | 0 | 0 | 0 | 0 | 0 | 0 | 0 | 0 | 0 | 0 | 0 | 1 | 0 | 0 | | | | | | |
| 939 | 01070 | 00938 | 2 | | 0 | 1 | 0 | 0 | 0 | 0 | 0 | 0 | 0 | 0 | 0 | 0 | 0 | 0 | 0 | 0 | 0 | 0 | 0 | 0 | 0 | 0 | 1 | 0 | 0 | | | | | | |
| 940 | 01071 | 00939 | 2 | | 0 | 1 | 1 | 0 | 0 | 0 | 0 | 0 | 0 | 0 | 0 | 0 | 0 | 0 | 0 | 0 | 0 | 0 | 0 | 0 | 0 | 0 | 0 | 0 | 0 | | | | | | |
| 941 | 01072 | 00940 | 8 | | 0 | 2 | 0 | 0 | 0 | 0 | 0 | 0 | 0 | 2 | 0 | 1 | 0 | 0 | 0 | 0 | 0 | 0 | 0 | 1 | 0 | 0 | 1 | 1 | 0 | | | | | | |
| 942 | 01073 | 00941 | 3 | | 0 | 1 | 0 | 0 | 0 | 0 | 0 | 0 | 0 | 0 | 0 | 0 | 0 | 0 | 0 | 0 | 0 | 0 | 0 | 1 | 0 | 0 | 1 | 0 | 0 | | | | | | |
| 943 | 01074 | 00942 | 2 | | 0 | 1 | 0 | 0 | 0 | 0 | 0 | 0 | 0 | 0 | 0 | 0 | 0 | 0 | 0 | 1 | 0 | 0 | 0 | 0 | 0 | 0 | 0 | 0 | 0 | X63071 | 97.3 | 377 | 1 | 4591 | 4972 |
| 944 | 01075 | 00943 | 10 | | 0 | 4 | 0 | 1 | 0 | 0 | 0 | 1 | 0 | 0 | 0 | 0 | 0 | 0 | 0 | 0 | 0 | 1 | 2 | 0 | 0 | 0 | 1 | 0 | 0 | | | | | | |
| 945 | 01076 | 00944 | 2 | | 0 | 1 | 0 | 0 | 0 | 0 | 0 | 0 | 0 | 0 | 0 | 0 | 0 | 0 | 0 | 0 | 0 | 0 | 0 | 1 | 0 | 0 | 0 | 0 | 0 | | | | | | |
| 946 | 01077 | 00945 | 1 | | 0 | 1 | 0 | 0 | 0 | 0 | 0 | 0 | 0 | 0 | 0 | 0 | 0 | 0 | 0 | 0 | 0 | 0 | 0 | 0 | 0 | 0 | 0 | 0 | 0 | D13435 | 99 | 311 | 69 | 608 | 917 |
| 947 | 01078 | 00946 | 2 | | 0 | 1 | 0 | 0 | 0 | 0 | 0 | 0 | 1 | 0 | 0 | 0 | 0 | 0 | 0 | 0 | 0 | 0 | 0 | 0 | 0 | 0 | 0 | 0 | 0 | | | | | | |
| 948 | 01079 | 00947 | 1 | | 0 | 1 | 0 | 0 | 0 | 0 | 0 | 0 | 0 | 0 | 0 | 0 | 0 | 0 | 0 | 0 | 0 | 0 | 0 | 0 | 0 | 0 | 0 | 0 | 0 | | | | | | |
| 949 | 01080 | 00948 | 5 | | 0 | 1 | 0 | 0 | 0 | 0 | 1 | 0 | 0 | 1 | 0 | 0 | 0 | 0 | 2 | 0 | 0 | 0 | 0 | 0 | 0 | 0 | 0 | 0 | 0 | | | | | | |
| 950 | 01081 | 00949 | 2 | | 0 | 1 | 0 | 0 | 0 | 0 | 0 | 0 | 0 | 0 | 0 | 0 | 0 | 0 | 0 | 0 | 0 | 0 | 1 | 0 | 0 | 0 | 0 | 0 | 0 | | | | | | |
| 951 | 01082 | 00950 | 1 | | 0 | 1 | 0 | 0 | 0 | 0 | 0 | 0 | 0 | 0 | 0 | 0 | 0 | 0 | 0 | 0 | 0 | 0 | 0 | 0 | 0 | 0 | 0 | 0 | 0 | | | | | | |
| 952 | 01083 | 00951 | 1 | | 0 | 1 | 0 | 0 | 0 | 0 | 0 | 0 | 0 | 0 | 0 | 0 | 0 | 0 | 0 | 0 | 0 | 0 | 0 | 0 | 0 | 0 | 0 | 0 | 0 | | | | | | |
| 953 | 01084 | 00952 | 2 | | 0 | 2 | 0 | 0 | 0 | 0 | 0 | 0 | 0 | 0 | 0 | 0 | 0 | 0 | 0 | 0 | 0 | 0 | 0 | 0 | 0 | 0 | 0 | 0 | 0 | | | | | | |
| 954 | 01085 | 00953 | 6 | | 0 | 1 | 0 | 0 | 0 | 0 | 0 | 0 | 0 | 0 | 0 | 1 | 0 | 0 | 1 | 0 | 0 | 1 | 1 | 0 | 0 | 1 | 0 | 0 | 0 | | | | | | |
| 955 | 01086 | 00954 | 8 | | 0 | 2 | 1 | 0 | 0 | 0 | 0 | 1 | 0 | 0 | 1 | 0 | 0 | 0 | 0 | 2 | 0 | 0 | 0 | 0 | 0 | 0 | 0 | 0 | 1 | | | | | | |
| 956 | 01087 | 00955 | 18 | | 0 | 6 | 0 | 0 | 0 | 0 | 0 | 2 | 0 | 1 | 0 | 0 | 0 | 0 | 2 | 2 | 0 | 0 | 0 | 1 | 1 | 0 | 3 | 0 | 0 | | | | | | |
| 957 | 01088 | 00956 | 3 | | 0 | 1 | 0 | 0 | 0 | 0 | 0 | 0 | 0 | 0 | 0 | 0 | 0 | 0 | 0 | 0 | 1 | 0 | 0 | 0 | 0 | 1 | 0 | 0 | 0 | | | | | | |

Table 27

| | A | B | C | E | G | I | K | M | O | Q | S | U | W | Y | AA | AC | AE | AG | AI | AK | AM | AO | AQ | AS | AU | AW | AY | BA | BC | BE | BF | BG | BH | BI | BK |
|---|---|---|---|---|---|---|---|---|---|---|---|---|---|---|---|---|---|---|---|---|---|---|---|---|---|---|---|---|---|---|---|---|---|---|---|
| 958 | 01089 | 00957 | 3 | | 0 | 1 | 0 | 0 | 0 | 0 | 0 | 0 | 0 | 0 | 0 | 0 | 0 | 0 | 0 | 0 | 0 | 0 | 0 | 0 | 0 | 0 | 2 | 0 | 0 | M16592 | 91.8 | 367 | 1 | 1410 | 1926 |
| 959 | 01090 | 00958 | 2 | | 0 | 1 | 0 | 0 | 0 | 0 | 0 | 0 | 0 | 0 | 1 | 0 | 0 | 0 | 0 | 0 | 0 | 0 | 0 | 0 | 0 | 0 | 0 | 0 | 0 | | | | | | |
| 960 | 01091 | 00959 | 5 | | 0 | 3 | 0 | 0 | 0 | 0 | 0 | 1 | 0 | 0 | 1 | 0 | 0 | 0 | 0 | 0 | 0 | 0 | 0 | 0 | 0 | 0 | 0 | 0 | 0 | | | | | | |
| 961 | 01092 | 00960 | 1 | | 0 | 1 | 0 | 0 | 0 | 0 | 0 | 0 | 0 | 0 | 0 | 0 | 0 | 0 | 0 | 0 | 0 | 0 | 0 | 0 | 0 | 0 | 0 | 0 | 0 | D12902 | 93 | 343 | 16 | 1 | 1391 |
| 962 | 01093 | 00961 | 2 | | 0 | 1 | 0 | 0 | 0 | 0 | 0 | 1 | 0 | 0 | 0 | 0 | 0 | 0 | 0 | 0 | 0 | 0 | 0 | 0 | 0 | 0 | 0 | 0 | 0 | | | | | | |
| 963 | 01094 | 00962 | 2 | | 0 | 1 | 0 | 0 | 0 | 0 | 0 | 0 | 0 | 0 | 1 | 0 | 0 | 0 | 0 | 0 | 0 | 0 | 0 | 0 | 0 | 0 | 0 | 0 | 0 | | | | | | |
| 964 | 01095 | 00963 | 3 | | 0 | 1 | 0 | 0 | 0 | 0 | 0 | 0 | 0 | 0 | 0 | 0 | 0 | 0 | 0 | 0 | 0 | 1 | 0 | 0 | 0 | 0 | 0 | 1 | 0 | | | | | | |
| 965 | 01096 | 00964 | 9 | | 0 | 1 | 0 | 0 | 0 | 0 | 0 | 0 | 1 | 0 | 0 | 1 | 1 | 1 | 1 | 0 | 0 | 0 | 0 | 2 | 0 | 0 | 0 | 1 | 0 | X66899 | 96.9 | 193 | 1 | 1981 | 2371 |
| 966 | 01097 | 00965 | 2 | | 0 | 1 | 0 | 0 | 0 | 0 | 0 | 0 | 0 | 0 | 1 | 0 | 0 | 0 | 0 | 0 | 0 | 0 | 0 | 0 | 0 | 0 | 0 | 0 | 0 | | | | | | |
| 967 | 01098 | 00966 | 1 | | 0 | 1 | 0 | 0 | 0 | 0 | 0 | 0 | 0 | 0 | 0 | 0 | 0 | 0 | 0 | 0 | 0 | 0 | 0 | 0 | 0 | 0 | 0 | 0 | 0 | X52195 | 98.4 | 64 | 1 | 477 | 540 |
| 968 | 01099 | 00967 | 2 | | 0 | 1 | 0 | 0 | 0 | 0 | 0 | 0 | 0 | 0 | 0 | 0 | 1 | 0 | 0 | 0 | 0 | 0 | 0 | 0 | 0 | 0 | 0 | 0 | 0 | Z23064 | 96.9 | 354 | 3 | 1513 | 1894 |
| 969 | 01100 | 00968 | 3 | | 0 | 1 | 0 | 0 | 0 | 0 | 0 | 0 | 0 | 0 | 0 | 0 | 0 | 0 | 0 | 0 | 0 | 0 | 1 | 0 | 0 | 0 | 1 | 0 | 0 | | | | | | |
| 970 | 01101 | 00969 | 1 | | 0 | 1 | 0 | 0 | 0 | 0 | 0 | 0 | 0 | 0 | 0 | 0 | 0 | 0 | 0 | 0 | 0 | 0 | 0 | 0 | 0 | 0 | 0 | 0 | 0 | | | | | | |
| 971 | 01102 | 00970 | 1 | | 0 | 1 | 0 | 0 | 0 | 0 | 0 | 0 | 0 | 0 | 0 | 0 | 0 | 0 | 0 | 0 | 0 | 0 | 0 | 0 | 0 | 0 | 0 | 0 | 0 | | | | | | |
| 972 | 01103 | 00971 | 2 | | 0 | 1 | 0 | 0 | 0 | 0 | 0 | 0 | 1 | 0 | 0 | 0 | 0 | 0 | 0 | 0 | 0 | 0 | 0 | 0 | 0 | 0 | 0 | 0 | 0 | | | | | | |
| 973 | 01104 | 00972 | 3 | | 0 | 2 | 0 | 0 | 0 | 0 | 0 | 0 | 0 | 0 | 0 | 0 | 0 | 0 | 0 | 0 | 0 | 0 | 0 | 0 | 0 | 0 | 0 | 1 | 0 | M60091 | 98.6 | 351 | 1 | 945 | 1295 |
| 974 | 01105 | 00973 | 8 | | 0 | 1 | 2 | 0 | 0 | 0 | 0 | 0 | 0 | 0 | 0 | 0 | 0 | 0 | 1 | 1 | 0 | 3 | 0 | 0 | 0 | 0 | 0 | 0 | 0 | | | | | | |
| 975 | 01106 | 00974 | 16 | | 0 | 4 | 3 | 0 | 0 | 0 | 0 | 0 | 0 | 0 | 0 | 0 | 0 | 0 | 4 | 1 | 0 | 0 | 0 | 0 | 0 | 0 | 2 | 0 | 2 | J03801 | 98.3 | 348 | 1 | 1142 | 1487 |
| 976 | 01107 | 00975 | 11 | | 0 | 2 | 0 | 0 | 0 | 0 | 3 | 0 | 0 | 1 | 1 | 2 | 0 | 0 | 1 | 0 | 1 | 0 | 0 | 0 | 0 | 0 | 0 | 0 | 0 | M75883 | 97.7 | 347 | 1 | 2227 | 2572 |
| 977 | 01108 | 00976 | 5 | | 0 | 1 | 1 | 0 | 1 | 0 | 0 | 0 | 1 | 1 | 0 | 0 | 0 | 0 | 0 | 0 | 0 | 0 | 0 | 0 | 0 | 0 | 0 | 0 | 0 | | | | | | |
| 978 | 01109 | 00977 | 2 | | 0 | 2 | 0 | 0 | 0 | 0 | 0 | 0 | 0 | 0 | 0 | 0 | 0 | 0 | 0 | 0 | 0 | 0 | 0 | 0 | 0 | 0 | 0 | 0 | 0 | | | | | | |
| 979 | 01110 | 00978 | 2 | | 0 | 1 | 0 | 0 | 0 | 0 | 1 | 0 | 0 | 0 | 0 | 0 | 0 | 0 | 0 | 0 | 0 | 0 | 0 | 0 | 0 | 0 | 0 | 0 | 0 | | | | | | |
| 980 | 01111 | 00979 | 1 | | 0 | 1 | 0 | 0 | 0 | 0 | 0 | 0 | 0 | 0 | 0 | 0 | 0 | 0 | 0 | 0 | 0 | 0 | 0 | 0 | 0 | 0 | 0 | 0 | 0 | | | | | | |
| 981 | 01112 | 00980 | 2 | | 0 | 1 | 0 | 0 | 0 | 0 | 0 | 0 | 0 | 0 | 0 | 0 | 0 | 1 | 0 | 0 | 0 | 0 | 0 | 0 | 0 | 0 | 0 | 0 | 0 | L20010 | 99.4 | 308 | 1 | 7851 | 8240 |
| 982 | 01115 | 00981 | 2 | | 0 | 1 | 0 | 0 | 0 | 0 | 0 | 1 | 0 | 0 | 0 | 0 | 0 | 0 | 0 | 0 | 0 | 0 | 0 | 0 | 0 | 0 | 0 | 0 | 0 | | | | | | |
| 983 | 01116 | 00982 | 2 | | 0 | 1 | 0 | 0 | 0 | 0 | 0 | 0 | 0 | 0 | 0 | 0 | 0 | 0 | 0 | 0 | 0 | 0 | 0 | 0 | 0 | 0 | 0 | 0 | 1 | | | | | | |
| 984 | 01117 | 00983 | 1 | | 0 | 1 | 0 | 0 | 0 | 0 | 0 | 0 | 0 | 0 | 0 | 0 | 0 | 0 | 0 | 0 | 0 | 0 | 0 | 0 | 0 | 0 | 0 | 0 | 0 | | | | | | |
| 985 | 01118 | 00984 | 2 | | 0 | 1 | 0 | 0 | 0 | 0 | 0 | 0 | 0 | 0 | 0 | 0 | 0 | 0 | 0 | 0 | 0 | 0 | 1 | 0 | 0 | 0 | 0 | 0 | 0 | | | | | | |
| 986 | 01119 | 00985 | 2 | | 0 | 1 | 0 | 0 | 0 | 0 | 0 | 0 | 0 | 0 | 0 | 0 | 0 | 0 | 0 | 0 | 0 | 0 | 0 | 1 | 0 | 0 | 0 | 0 | 0 | | | | | | |
| 987 | 01120 | 00986 | 1 | | 0 | 1 | 0 | 0 | 0 | 0 | 0 | 0 | 0 | 0 | 0 | 0 | 0 | 0 | 0 | 0 | 0 | 0 | 0 | 0 | 0 | 0 | 0 | 0 | 0 | | | | | | |
| 988 | 01122 | 00987 | 1 | | 0 | 1 | 0 | 0 | 0 | 0 | 0 | 0 | 0 | 0 | 0 | 0 | 0 | 0 | 0 | 0 | 0 | 0 | 0 | 0 | 0 | 0 | 0 | 0 | 0 | | | | | | |
| 989 | 01123 | 00988 | 3 | | 0 | 1 | 0 | 0 | 0 | 0 | 0 | 0 | 0 | 0 | 0 | 0 | 0 | 0 | 0 | 1 | 0 | 0 | 0 | 0 | 0 | 0 | 1 | 0 | 0 | | | | | | |
| 990 | 01126 | 00989 | 1 | | 0 | 1 | 0 | 0 | 0 | 0 | 0 | 0 | 0 | 0 | 0 | 0 | 0 | 0 | 0 | 0 | 0 | 0 | 0 | 0 | 0 | 0 | 0 | 0 | 0 | | | | | | |
| 991 | 01127 | 00990 | 1 | | 0 | 1 | 0 | 0 | 0 | 0 | 0 | 0 | 0 | 0 | 0 | 0 | 0 | 0 | 0 | 0 | 0 | 0 | 0 | 0 | 0 | 0 | 0 | 0 | 0 | X70218 | 92.2 | 332 | 1 | 837 | 1382 |
| 992 | 01128 | 00991 | 2 | | 0 | 1 | 0 | 0 | 0 | 0 | 0 | 0 | 0 | 0 | 0 | 0 | 0 | 0 | 1 | 0 | 0 | 0 | 0 | 0 | 0 | 0 | 0 | 0 | 0 | | | | | | |
| 993 | 01129 | 00992 | 1 | | 0 | 1 | 0 | 0 | 0 | 0 | 0 | 0 | 0 | 0 | 0 | 0 | 0 | 0 | 0 | 0 | 0 | 0 | 0 | 0 | 0 | 0 | 0 | 0 | 0 | | | | | | |

Table 28

| | A | B | C | E | G | I | K | M | O | Q | S | U | W | Y | AA | AC | AE | AG | AI | AK | AM | AO | AQ | AS | AU | AW | AY | BA | BC | BE | BF | BG | BH | BI | BK |
|---|---|---|---|---|---|---|---|---|---|---|---|---|---|---|---|---|---|---|---|---|---|---|---|---|---|---|---|---|---|---|---|---|---|---|---|
| 994 | 01130 | 00993 | 4 | | 0 | 1 | 0 | 0 | 0 | 0 | 0 | 0 | 0 | 0 | 0 | 0 | 0 | 3 | 0 | 0 | 0 | 0 | 0 | 0 | 0 | 0 | 0 | 0 | 0 | | | | | | |
| 995 | 01131 | 00994 | 1 | | 0 | 1 | 0 | 0 | 0 | 0 | 0 | 0 | 0 | 0 | 0 | 0 | 0 | 0 | 0 | 0 | 0 | 0 | 0 | 0 | 0 | 0 | 0 | 0 | 0 | M60721 | 94.6 | 331 | 1 | 1900 | 2231 |
| 996 | 01132 | 00995 | 3 | | 0 | 1 | 0 | 0 | 0 | 0 | 0 | 0 | 0 | 0 | 0 | 1 | 0 | 0 | 0 | 0 | 0 | 0 | 1 | 0 | 0 | 0 | 0 | 0 | 0 | | | | | | |
| 997 | 01133 | 00996 | 1 | | 0 | 1 | 0 | 0 | 0 | 0 | 0 | 0 | 0 | 0 | 0 | 0 | 0 | 0 | 0 | 0 | 0 | 0 | 0 | 0 | 0 | 0 | 0 | 0 | 0 | | | | | | |
| 998 | 01134 | 00997 | 3 | | 0 | 1 | 0 | 0 | 0 | 0 | 0 | 0 | 0 | 1 | 0 | 0 | 0 | 0 | 0 | 0 | 0 | 0 | 0 | 0 | 0 | 0 | 1 | 0 | 0 | | | | | | |
| 999 | 01135 | 00998 | 1 | | 0 | 1 | 0 | 0 | 0 | 0 | 0 | 0 | 0 | 0 | 0 | 0 | 0 | 0 | 0 | 0 | 0 | 0 | 0 | 0 | 0 | 0 | 0 | 0 | 0 | | | | | | |
| 1000 | 01136 | 00999 | 1 | | 0 | 1 | 0 | 0 | 0 | 0 | 0 | 0 | 0 | 0 | 0 | 0 | 0 | 0 | 0 | 0 | 0 | 0 | 0 | 0 | 0 | 0 | 0 | 0 | 0 | | | | | | |
| 1001 | 01137 | 01000 | 1 | | 0 | 1 | 0 | 0 | 0 | 0 | 0 | 0 | 0 | 0 | 0 | 0 | 0 | 0 | 0 | 0 | 0 | 0 | 0 | 0 | 0 | 0 | 0 | 0 | 0 | | | | | | |
| 1002 | 01138 | 01001 | 6 | | 0 | 1 | 1 | 0 | 1 | 0 | 1 | 0 | 0 | 0 | 0 | 0 | 0 | 0 | 1 | 0 | 0 | 1 | 0 | 0 | 0 | 0 | 0 | 0 | 0 | | | | | | |
| 1003 | 01139 | 01002 | 3 | | 0 | 1 | 0 | 0 | 0 | 0 | 0 | 0 | 1 | 0 | 0 | 0 | 0 | 0 | 0 | 1 | 0 | 0 | 0 | 0 | 0 | 0 | 0 | 0 | 0 | L19711 | 97.8 | 318 | 1 | 5079 | 5493 |
| 1004 | 01140 | 01003 | 5 | | 0 | 1 | 0 | 0 | 0 | 0 | 1 | 0 | 0 | 0 | 0 | 0 | 0 | 0 | 0 | 0 | 0 | 0 | 0 | 0 | 0 | 2 | 0 | 1 | 0 | L11066 | 95.8 | 310 | 1 | 2517 | 2845 |
| 1005 | 01141 | 01004 | 18 | | 0 | 3 | 0 | 1 | 0 | 0 | 0 | 2 | 0 | 0 | 0 | 0 | 0 | 0 | 0 | 2 | 0 | 1 | 0 | 0 | 1 | 7 | 0 | 0 | 1 | | | | | | |
| 1006 | 01142 | 01005 | 1 | | 0 | 1 | 0 | 0 | 0 | 0 | 0 | 0 | 0 | 0 | 0 | 0 | 0 | 0 | 0 | 0 | 0 | 0 | 0 | 0 | 0 | 0 | 0 | 0 | 0 | | | | | | |
| 1007 | 01143 | 01006 | 3 | | 0 | 1 | 0 | 0 | 0 | 0 | 0 | 0 | 0 | 0 | 0 | 0 | 0 | 0 | 1 | 0 | 0 | 0 | 0 | 0 | 0 | 1 | 0 | 0 | 0 | | | | | | |
| 1008 | 01144 | 01007 | 1 | | 0 | 1 | 0 | 0 | 0 | 0 | 0 | 0 | 0 | 0 | 0 | 0 | 0 | 0 | 0 | 0 | 0 | 0 | 0 | 0 | 0 | 0 | 0 | 0 | 0 | | | | | | |
| 1009 | 01145 | 01008 | 7 | | 0 | 4 | 0 | 0 | 0 | 0 | 0 | 1 | 0 | 0 | 1 | 0 | 0 | 0 | 0 | 1 | 0 | 0 | 0 | 0 | 0 | 0 | 0 | 0 | 0 | | | | | | |
| 1010 | 01147 | 01009 | 1 | | 0 | 1 | 0 | 0 | 0 | 0 | 0 | 0 | 0 | 0 | 0 | 0 | 0 | 0 | 0 | 0 | 0 | 0 | 0 | 0 | 0 | 0 | 0 | 0 | 0 | | | | | | |
| 1011 | 01148 | 01010 | 1 | | 0 | 1 | 0 | 0 | 0 | 0 | 0 | 0 | 0 | 0 | 0 | 0 | 0 | 0 | 0 | 0 | 0 | 0 | 0 | 0 | 0 | 0 | 0 | 0 | 0 | | | | | | |
| 1012 | 01149 | 01011 | 2 | | 0 | 1 | 0 | 0 | 0 | 0 | 0 | 0 | 0 | 0 | 0 | 0 | 0 | 0 | 0 | 0 | 1 | 0 | 0 | 0 | 0 | 0 | 0 | 0 | 0 | | | | | | |
| 1013 | 01150 | 01012 | 6 | | 0 | 1 | 0 | 0 | 0 | 0 | 0 | 1 | 0 | 0 | 0 | 0 | 0 | 0 | 0 | 2 | 0 | 0 | 1 | 0 | 0 | 0 | 0 | 1 | 0 | M80613 | 93.5 | 308 | 1 | 3730 | 4053 |
| 1014 | 01151 | 01013 | 4 | | 0 | 1 | 0 | 0 | 0 | 0 | 0 | 0 | 0 | 0 | 0 | 0 | 0 | 0 | 0 | 0 | 0 | 1 | 1 | 1 | 0 | 0 | 0 | 0 | 0 | | | | | | |
| 1015 | 01152 | 01014 | 1 | | 0 | 1 | 0 | 0 | 0 | 0 | 0 | 0 | 0 | 0 | 0 | 0 | 0 | 0 | 0 | 0 | 0 | 0 | 0 | 0 | 0 | 0 | 0 | 0 | 0 | | | | | | |
| 1016 | 01153 | 01015 | 3 | | 0 | 2 | 0 | 0 | 0 | 0 | 0 | 1 | 0 | 0 | 0 | 0 | 0 | 0 | 0 | 0 | 0 | 0 | 0 | 0 | 0 | 0 | 0 | 0 | 0 | | | | | | |
| 1017 | 01154 | 01016 | 17 | | 0 | 2 | 1 | 1 | 0 | 1 | 0 | 0 | 0 | 1 | 2 | 0 | 0 | 0 | 0 | 2 | 1 | 2 | 0 | 1 | 0 | 0 | 0 | 3 | 0 | J03191 | 96.7 | 301 | 1 | 496 | 793 |
| 1018 | 01155 | 01017 | 3 | | 0 | 1 | 0 | 0 | 0 | 0 | 0 | 0 | 0 | 0 | 0 | 0 | 0 | 0 | 0 | 0 | 0 | 0 | 0 | 0 | 0 | 0 | 2 | 0 | 0 | | | | | | |
| 1019 | 01156 | 01018 | 2 | | 0 | 2 | 0 | 0 | 0 | 0 | 0 | 0 | 0 | 0 | 0 | 0 | 0 | 0 | 0 | 0 | 0 | 0 | 0 | 0 | 0 | 0 | 0 | 0 | 0 | | | | | | |
| 1020 | 01157 | 01019 | 9 | | 0 | 2 | 1 | 0 | 0 | 0 | 0 | 0 | 0 | 0 | 1 | 0 | 0 | 0 | 0 | 0 | 1 | 1 | 1 | 0 | 1 | 0 | 1 | 0 | 0 | | | | | | |
| 1021 | 01158 | 01020 | 2 | | 0 | 1 | 0 | 0 | 0 | 0 | 0 | 0 | 0 | 0 | 0 | 0 | 0 | 0 | 0 | 0 | 0 | 0 | 0 | 0 | 0 | 0 | 1 | 0 | 0 | | | | | | |
| 1022 | 01159 | 01021 | 1 | | 0 | 1 | 0 | 0 | 0 | 0 | 0 | 0 | 0 | 0 | 0 | 0 | 0 | 0 | 0 | 0 | 0 | 0 | 0 | 0 | 0 | 0 | 0 | 0 | 0 | | | | | | |
| 1023 | 01160 | 01022 | 1 | | 0 | 1 | 0 | 0 | 0 | 0 | 0 | 0 | 0 | 0 | 0 | 0 | 0 | 0 | 0 | 0 | 0 | 0 | 0 | 0 | 0 | 0 | 0 | 0 | 0 | | | | | | |
| 1024 | 01161 | 01023 | 2 | | 0 | 1 | 0 | 0 | 0 | 0 | 0 | 0 | 0 | 0 | 0 | 0 | 0 | 0 | 0 | 0 | 0 | 1 | 0 | 0 | 0 | 0 | 0 | 0 | 0 | | | | | | |
| 1025 | 01162 | 01024 | 1 | | 0 | 1 | 0 | 0 | 0 | 0 | 0 | 0 | 0 | 0 | 0 | 0 | 0 | 0 | 0 | 0 | 0 | 0 | 0 | 0 | 0 | 0 | 0 | 0 | 0 | | | | | | |
| 1026 | 01163 | 01025 | 16 | | 0 | 1 | 1 | 0 | 0 | 0 | 2 | 0 | 0 | 2 | 0 | 0 | 0 | 1 | 1 | 1 | 0 | 1 | 2 | 1 | 1 | 1 | 0 | 0 | 1 | | | | | | |
| 1027 | 01164 | 01026 | 2 | | 0 | 2 | 0 | 0 | 0 | 0 | 0 | 0 | 0 | 0 | 0 | 0 | 0 | 0 | 0 | 0 | 0 | 0 | 0 | 0 | 0 | 0 | 0 | 0 | 0 | M23197 | 99 | 297 | 1 | 1140 | 1437 |
| 1028 | 01165 | 01027 | 3 | | 0 | 1 | 0 | 0 | 0 | 0 | 0 | 0 | 0 | 0 | 0 | 1 | 0 | 0 | 0 | 0 | 1 | 0 | 0 | 0 | 0 | 0 | 0 | 0 | 0 | | | | | | |
| 1029 | 01167 | 01028 | 2 | | 0 | 1 | 0 | 0 | 0 | 0 | 0 | 0 | 0 | 0 | 0 | 0 | 0 | 0 | 0 | 0 | 0 | 0 | 1 | 0 | 0 | 0 | 0 | 0 | 0 | | | | | | |

Table 29

| | A | B | C | E | G | I | K | M | O | Q | S | U | W | Y | AA | AC | AE | AG | AI | AK | AM | AO | AQ | AS | AU | AW | AY | BA | BC | BE | BF | BG | BH | BI | BK |
|---|---|---|---|---|---|---|---|---|---|---|---|---|---|---|---|---|---|---|---|---|---|---|---|---|---|---|---|---|---|---|---|---|---|---|---|
| 1030 | 01168 | 01029 | 2 | | 0 | 1 | 0 | 0 | 0 | 0 | 0 | 0 | 0 | 0 | 0 | 0 | 0 | 0 | 0 | 0 | 0 | 0 | 0 | 0 | 0 | 0 | 0 | 1 | 0 | | | | | | |
| 1031 | 01169 | 01030 | 2 | | 0 | 1 | 0 | 0 | 0 | 0 | 0 | 0 | 0 | 0 | 0 | 0 | 0 | 0 | 0 | 0 | 0 | 0 | 0 | 0 | 0 | 0 | 0 | 1 | 0 | | | | | | |
| 1032 | 01170 | 01031 | 3 | | 0 | 1 | 0 | 0 | 0 | 0 | 0 | 0 | 0 | 0 | 0 | 0 | 0 | 0 | 0 | 0 | 1 | 0 | 0 | 0 | 0 | 1 | 0 | 0 | 0 | | | | | | |
| 1033 | 01171 | 01032 | 3 | | 0 | 1 | 0 | 0 | 0 | 0 | 0 | 0 | 0 | 0 | 0 | 0 | 0 | 0 | 0 | 0 | 0 | 0 | 0 | 0 | 0 | 1 | 0 | 1 | 0 | | | | | | |
| 1034 | 01172 | 01033 | 3 | | 0 | 1 | 0 | 0 | 0 | 0 | 0 | 0 | 0 | 0 | 0 | 0 | 0 | 0 | 1 | 0 | 0 | 1 | 0 | 0 | 0 | 0 | 0 | 0 | 0 | | | | | | |
| 1035 | 01173 | 01034 | 2 | | 0 | 1 | 0 | 0 | 0 | 0 | 0 | 0 | 0 | 1 | 0 | 0 | 0 | 0 | 0 | 0 | 0 | 0 | 0 | 0 | 0 | 0 | 0 | 0 | 0 | | | | | | |
| 1036 | 01174 | 01035 | 1 | | 0 | 1 | 0 | 0 | 0 | 0 | 0 | 0 | 0 | 0 | 0 | 0 | 0 | 0 | 0 | 0 | 0 | 0 | 0 | 0 | 0 | 0 | 0 | 0 | 0 | | | | | | |
| 1037 | 01175 | 01036 | 14 | | 0 | 1 | 2 | 1 | 4 | 1 | 0 | 0 | 0 | 0 | 0 | 0 | 1 | 0 | 0 | 0 | 0 | 0 | 1 | 0 | 0 | 3 | 0 | 0 | 0 | X76717 | 98.9 | 275 | 1 | 21 | 415 |
| 1038 | 01176 | 01037 | 2 | | 0 | 1 | 0 | 0 | 0 | 0 | 0 | 0 | 1 | 0 | 0 | 0 | 0 | 0 | 0 | 0 | 0 | 0 | 0 | 0 | 0 | 0 | 0 | 0 | 0 | | | | | | |
| 1039 | 01177 | 01038 | 2 | | 0 | 2 | 0 | 0 | 0 | 0 | 0 | 0 | 0 | 0 | 0 | 0 | 0 | 0 | 0 | 0 | 0 | 0 | 0 | 0 | 0 | 0 | 0 | 0 | 0 | | | | | | |
| 1040 | 01178 | 01039 | 2 | | 0 | 1 | 0 | 0 | 0 | 0 | 0 | 0 | 0 | 0 | 0 | 0 | 0 | 0 | 0 | 0 | 0 | 0 | 0 | 0 | 1 | 0 | 0 | 0 | 0 | | | | | | |
| 1041 | 01179 | 01040 | 1 | | 0 | 1 | 0 | 0 | 0 | 0 | 0 | 0 | 0 | 0 | 0 | 0 | 0 | 0 | 0 | 0 | 0 | 0 | 0 | 0 | 0 | 0 | 0 | 0 | 0 | | | | | | |
| 1042 | 01180 | 01041 | 3 | | 0 | 1 | 0 | 0 | 0 | 0 | 0 | 0 | 0 | 0 | 0 | 0 | 0 | 1 | 0 | 1 | 0 | 0 | 0 | 0 | 0 | 0 | 0 | 0 | 0 | | | | | | |
| 1043 | 01181 | 01042 | 1 | | 0 | 1 | 0 | 0 | 0 | 0 | 0 | 0 | 0 | 0 | 0 | 0 | 0 | 0 | 0 | 0 | 0 | 0 | 0 | 0 | 0 | 0 | 0 | 0 | 0 | | | | | | |
| 1044 | 01182 | 01043 | 1 | | 0 | 1 | 0 | 0 | 0 | 0 | 0 | 0 | 0 | 0 | 0 | 0 | 0 | 0 | 0 | 0 | 0 | 0 | 0 | 0 | 0 | 0 | 0 | 0 | 0 | | | | | | |
| 1045 | 01183 | 01044 | 1 | | 0 | 1 | 0 | 0 | 0 | 0 | 0 | 0 | 0 | 0 | 0 | 0 | 0 | 0 | 0 | 0 | 0 | 0 | 0 | 0 | 0 | 0 | 0 | 0 | 0 | | | | | | |
| 1046 | 01184 | 01045 | 7 | | 0 | 1 | 1 | 0 | 0 | 0 | 0 | 0 | 0 | 0 | 0 | 0 | 0 | 2 | 0 | 0 | 0 | 1 | 1 | 0 | 1 | 0 | 0 | 0 | 0 | | | | | | |
| 1047 | 01186 | 01046 | 1 | | 0 | 1 | 0 | 0 | 0 | 0 | 0 | 0 | 0 | 0 | 0 | 0 | 0 | 0 | 0 | 0 | 0 | 0 | 0 | 0 | 0 | 0 | 0 | 0 | 0 | | | | | | |
| 1048 | 01187 | 01047 | 8 | | 0 | 2 | 2 | 0 | 0 | 0 | 1 | 0 | 0 | 0 | 0 | 0 | 0 | 0 | 0 | 0 | 0 | 0 | 0 | 0 | 0 | 0 | 3 | 0 | 0 | M83246 | 97.9 | 285 | 1 | 1055 | 1349 |
| 1049 | 01188 | 01048 | 5 | | 0 | 1 | 1 | 0 | 0 | 0 | 0 | 0 | 1 | 0 | 0 | 0 | 0 | 1 | 0 | 0 | 0 | 1 | 0 | 0 | 0 | 0 | 0 | 0 | 0 | | | | | | |
| 1050 | 01189 | 01049 | 13 | | 0 | 1 | 0 | 0 | 0 | 1 | 3 | 0 | 1 | 0 | 0 | 4 | 0 | 2 | 0 | 1 | 0 | 0 | 0 | 0 | 0 | 0 | 0 | 0 | 0 | | | | | | |
| 1051 | 01191 | 01050 | 1 | | 0 | 1 | 0 | 0 | 0 | 0 | 0 | 0 | 0 | 0 | 0 | 0 | 0 | 0 | 0 | 0 | 0 | 0 | 0 | 0 | 0 | 0 | 0 | 0 | 0 | | | | | | |
| 1052 | 01192 | 01051 | 8 | | 0 | 1 | 0 | 0 | 0 | 0 | 1 | 0 | 2 | 0 | 0 | 0 | 0 | 0 | 1 | 0 | 0 | 2 | 0 | 0 | 1 | 0 | 0 | 0 | 0 | | | | | | |
| 1053 | 01193 | 01052 | 1 | | 0 | 1 | 0 | 0 | 0 | 0 | 0 | 0 | 0 | 0 | 0 | 0 | 0 | 0 | 0 | 0 | 0 | 0 | 0 | 0 | 0 | 0 | 0 | 0 | 0 | | | | | | |
| 1054 | 01194 | 01053 | 1 | | 0 | 1 | 0 | 0 | 0 | 0 | 0 | 0 | 0 | 0 | 0 | 0 | 0 | 0 | 0 | 0 | 0 | 0 | 0 | 0 | 0 | 0 | 0 | 0 | 0 | | | | | | |
| 1055 | 01195 | 01054 | 6 | | 0 | 1 | 0 | 0 | 0 | 0 | 0 | 0 | 0 | 0 | 0 | 0 | 1 | 1 | 0 | 0 | 0 | 1 | 0 | 1 | 1 | 0 | 0 | 0 | 0 | M94083 | 96 | 278 | 1 | 1374 | 1685 |
| 1056 | 01196 | 01055 | 3 | | 0 | 1 | 0 | 0 | 0 | 0 | 0 | 0 | 0 | 0 | 0 | 0 | 0 | 1 | 0 | 0 | 1 | 0 | 0 | 0 | 0 | 0 | 0 | 0 | 0 | | | | | | |
| 1057 | 01197 | 01056 | 2 | | 0 | 1 | 0 | 0 | 0 | 0 | 1 | 0 | 0 | 0 | 0 | 0 | 0 | 0 | 0 | 0 | 0 | 0 | 0 | 0 | 0 | 0 | 0 | 0 | 0 | | | | | | |
| 1058 | 01198 | 01057 | 10 | | 0 | 2 | 0 | 0 | 0 | 1 | 0 | 0 | 1 | 0 | 0 | 1 | 0 | 0 | 0 | 1 | 0 | 2 | 0 | 0 | 1 | 0 | 0 | 1 | 0 | Y00281 | 92.8 | 290 | 1 | 1934 | 2397 |
| 1059 | 01199 | 01058 | 1 | | 0 | 1 | 0 | 0 | 0 | 0 | 0 | 0 | 0 | 0 | 0 | 0 | 0 | 0 | 0 | 0 | 0 | 0 | 0 | 0 | 0 | 0 | 0 | 0 | 0 | | | | | | |
| 1060 | 01200 | 01059 | 3 | | 0 | 1 | 0 | 0 | 0 | 0 | 0 | 0 | 0 | 0 | 0 | 0 | 0 | 0 | 0 | 0 | 0 | 0 | 0 | 0 | 0 | 0 | 2 | 0 | 0 | | | | | | |
| 1061 | 01201 | 01060 | 4 | | 0 | 2 | 0 | 0 | 0 | 0 | 0 | 0 | 0 | 0 | 0 | 0 | 1 | 0 | 0 | 0 | 0 | 0 | 0 | 0 | 0 | 0 | 1 | 0 | 0 | | | | | | |
| 1062 | 01202 | 01061 | 3 | | 0 | 1 | 0 | 0 | 0 | 0 | 0 | 0 | 0 | 0 | 0 | 0 | 0 | 0 | 0 | 0 | 0 | 0 | 0 | 0 | 0 | 0 | 2 | 0 | 0 | M32011 | 94.6 | 276 | 1 | 1931 | 2206 |
| 1063 | 01203 | 01062 | 4 | | 0 | 1 | 0 | 0 | 0 | 0 | 0 | 0 | 0 | 0 | 0 | 0 | 0 | 0 | 0 | 0 | 0 | 0 | 0 | 1 | 0 | 0 | 0 | 2 | 0 | | | | | | |
| 1064 | 01204 | 01063 | 1 | | 0 | 1 | 0 | 0 | 0 | 0 | 0 | 0 | 0 | 0 | 0 | 0 | 0 | 0 | 0 | 0 | 0 | 0 | 0 | 0 | 0 | 0 | 0 | 0 | 0 | | | | | | |
| 1065 | 01205 | 01064 | 1 | | 0 | 1 | 0 | 0 | 0 | 0 | 0 | 0 | 0 | 0 | 0 | 0 | 0 | 0 | 0 | 0 | 0 | 0 | 0 | 0 | 0 | 0 | 0 | 0 | 0 | | | | | | |

Table 30

| | A | B | C | E | G | I | K | M | O | Q | S | U | W | Y | AA | AC | AE | AG | AI | AK | AM | AO | AQ | AS | AU | AW | AY | BA | BC | BE | BF | BG | BH | BI | BK |
|---|---|---|---|---|---|---|---|---|---|---|---|---|---|---|---|---|---|---|---|---|---|---|---|---|---|---|---|---|---|---|---|---|---|---|---|
| 1066 | 01207 | 01065 | 5 | | 0 | 1 | 0 | 0 | 0 | 0 | 1 | 0 | 0 | 0 | 0 | 0 | 0 | 0 | 0 | 1 | 0 | 0 | 0 | 0 | 1 | 0 | 0 | 1 | 0 | | | | | | |
| 1067 | 01208 | 01066 | 2 | | 0 | 1 | 0 | 0 | 0 | 0 | 0 | 0 | 0 | 0 | 0 | 0 | 0 | 0 | 0 | 0 | 0 | 0 | 0 | 1 | 0 | 0 | 0 | 0 | 0 | | | | | | |
| 1068 | 01209 | 01067 | 2 | | 0 | 1 | 0 | 0 | 0 | 0 | 0 | 0 | 0 | 0 | 0 | 0 | 0 | 0 | 0 | 0 | 1 | 0 | 0 | 0 | 0 | 0 | 0 | 0 | 0 | | | | | | |
| 1069 | 01210 | 01068 | 1 | | 0 | 1 | 0 | 0 | 0 | 0 | 0 | 0 | 0 | 0 | 0 | 0 | 0 | 0 | 0 | 0 | 0 | 0 | 0 | 0 | 0 | 0 | 0 | 0 | 0 | | | | | | |
| 1070 | 01211 | 01069 | 1 | | 0 | 1 | 0 | 0 | 0 | 0 | 0 | 0 | 0 | 0 | 0 | 0 | 0 | 0 | 0 | 0 | 0 | 0 | 0 | 0 | 0 | 0 | 0 | 0 | 0 | | | | | | |
| 1071 | 01212 | 01070 | 1 | | 0 | 1 | 0 | 0 | 0 | 0 | 0 | 0 | 0 | 0 | 0 | 0 | 0 | 0 | 0 | 0 | 0 | 0 | 0 | 0 | 0 | 0 | 0 | 0 | 0 | | | | | | |
| 1072 | 01213 | 01071 | 1 | | 0 | 1 | 0 | 0 | 0 | 0 | 0 | 0 | 0 | 0 | 0 | 0 | 0 | 0 | 0 | 0 | 0 | 0 | 0 | 0 | 0 | 0 | 0 | 0 | 0 | | | | | | |
| 1073 | 01214 | 01072 | 2 | | 0 | 1 | 0 | 0 | 0 | 0 | 0 | 0 | 0 | 0 | 0 | 0 | 0 | 0 | 0 | 0 | 0 | 0 | 0 | 1 | 0 | 0 | 0 | 0 | 0 | | | | | | |
| 1074 | 01215 | 01073 | 15 | | 0 | 1 | 0 | 0 | 0 | 0 | 0 | 1 | 0 | 0 | 0 | 1 | 1 | 0 | 0 | 0 | 4 | 0 | 0 | 0 | 6 | 1 | 0 | 0 | 0 | | | | | | |
| 1075 | 01216 | 01074 | 7 | | 0 | 1 | 1 | 0 | 0 | 0 | 1 | 0 | 0 | 0 | 0 | 1 | 0 | 0 | 0 | 1 | 0 | 0 | 0 | 0 | 0 | 0 | 0 | 2 | 0 | M93425 | 97.3 | 262 | 1 | 2900 | 3160 |
| 1076 | 01217 | 01075 | 7 | | 0 | 1 | 0 | 0 | 0 | 0 | 1 | 0 | 0 | 2 | 0 | 0 | 0 | 0 | 0 | 1 | 1 | 0 | 0 | 0 | 0 | 0 | 0 | 1 | 0 | | | | | | |
| 1077 | 01218 | 01076 | 1 | | 0 | 1 | 0 | 0 | 0 | 0 | 0 | 0 | 0 | 0 | 0 | 0 | 0 | 0 | 0 | 0 | 0 | 0 | 0 | 0 | 0 | 0 | 0 | 0 | 0 | | | | | | |
| 1078 | 01219 | 01077 | 2 | | 0 | 1 | 0 | 0 | 0 | 0 | 0 | 0 | 0 | 0 | 0 | 0 | 0 | 0 | 0 | 0 | 0 | 0 | 0 | 0 | 0 | 0 | 0 | 1 | 0 | | | | | | |
| 1079 | 01220 | 01078 | 1 | | 0 | 1 | 0 | 0 | 0 | 0 | 0 | 0 | 0 | 0 | 0 | 0 | 0 | 0 | 0 | 0 | 0 | 0 | 0 | 0 | 0 | 0 | 0 | 0 | 0 | | | | | | |
| 1080 | 01221 | 01079 | 1 | | 0 | 1 | 0 | 0 | 0 | 0 | 0 | 0 | 0 | 0 | 0 | 0 | 0 | 0 | 0 | 0 | 0 | 0 | 0 | 0 | 0 | 0 | 0 | 0 | 0 | | | | | | |
| 1081 | 01222 | 01080 | 3 | | 0 | 1 | 0 | 0 | 0 | 0 | 0 | 0 | 0 | 0 | 0 | 2 | 0 | 0 | 0 | 0 | 0 | 0 | 0 | 0 | 0 | 0 | 0 | 0 | 0 | | | | | | |
| 1082 | 01223 | 01081 | 8 | | 0 | 2 | 1 | 0 | 0 | 0 | 0 | 0 | 1 | 0 | 0 | 0 | 0 | 0 | 4 | 0 | 0 | 0 | 0 | 0 | 0 | 0 | 0 | 0 | 0 | K03199 | 95.4 | 65 | 261 | 8 | 1760 |
| 1083 | 01224 | 01082 | 10 | | 0 | 4 | 0 | 0 | 0 | 0 | 1 | 0 | 0 | 0 | 0 | 0 | 0 | 0 | 1 | 1 | 0 | 0 | 0 | 0 | 0 | 0 | 1 | 2 | 0 | | | | | | |
| 1084 | 01225 | 01083 | 1 | | 0 | 1 | 0 | 0 | 0 | 0 | 0 | 0 | 0 | 0 | 0 | 0 | 0 | 0 | 0 | 0 | 0 | 0 | 0 | 0 | 0 | 0 | 0 | 0 | 0 | | | | | | |
| 1085 | 01226 | 01084 | 3 | | 0 | 1 | 1 | 0 | 0 | 0 | 0 | 0 | 0 | 0 | 0 | 0 | 0 | 0 | 0 | 1 | 0 | 0 | 0 | 0 | 0 | 0 | 0 | 0 | 0 | | | | | | |
| 1086 | 01227 | 01085 | 15 | | 0 | 1 | 2 | 0 | 1 | 0 | 1 | 0 | 2 | 3 | 0 | 0 | 0 | 0 | 1 | 0 | 1 | 0 | 3 | 0 | 0 | 0 | 0 | 0 | 0 | | | | | | |
| 1087 | 01228 | 01086 | 12 | | 0 | 4 | 0 | 0 | 0 | 1 | 0 | 0 | 0 | 0 | 1 | 1 | 0 | 0 | 0 | 0 | 0 | 0 | 2 | 0 | 0 | 0 | 3 | 0 | 0 | | | | | | |
| 1088 | 01229 | 01087 | 2 | | 0 | 1 | 0 | 0 | 0 | 0 | 0 | 0 | 0 | 0 | 0 | 0 | 0 | 0 | 0 | 0 | 0 | 0 | 0 | 0 | 0 | 0 | 1 | 0 | 0 | X06234 | 96.1 | 230 | 25 | 182 | 418 |
| 1089 | 01230 | 01088 | 2 | | 0 | 1 | 0 | 0 | 0 | 0 | 0 | 0 | 0 | 0 | 0 | 0 | 0 | 0 | 0 | 0 | 0 | 0 | 0 | 1 | 0 | 0 | 0 | 0 | 0 | | | | | | |
| 1090 | 01231 | 01089 | 2 | | 0 | 1 | 0 | 0 | 0 | 0 | 0 | 0 | 0 | 0 | 0 | 0 | 0 | 0 | 0 | 1 | 0 | 0 | 0 | 0 | 0 | 0 | 0 | 0 | 0 | | | | | | |
| 1091 | 01232 | 01090 | 2 | | 0 | 1 | 0 | 0 | 0 | 0 | 0 | 0 | 0 | 0 | 0 | 0 | 0 | 0 | 1 | 0 | 0 | 0 | 0 | 0 | 0 | 0 | 0 | 0 | 0 | | | | | | |
| 1092 | 01233 | 01091 | 1 | | 0 | 1 | 0 | 0 | 0 | 0 | 0 | 0 | 0 | 0 | 0 | 0 | 0 | 0 | 0 | 0 | 0 | 0 | 0 | 0 | 0 | 0 | 0 | 0 | 0 | | | | | | |
| 1093 | 01235 | 01092 | 1 | | 0 | 1 | 0 | 0 | 0 | 0 | 0 | 0 | 0 | 0 | 0 | 0 | 0 | 0 | 0 | 0 | 0 | 0 | 0 | 0 | 0 | 0 | 0 | 0 | 0 | | | | | | |
| 1094 | 01236 | 01093 | 1 | | 0 | 1 | 0 | 0 | 0 | 0 | 0 | 0 | 0 | 0 | 0 | 0 | 0 | 0 | 0 | 0 | 0 | 0 | 0 | 0 | 0 | 0 | 0 | 0 | 0 | M63483 | 96.8 | 247 | 1 | 975 | 1328 |
| 1095 | 01237 | 01094 | 1 | | 0 | 1 | 0 | 0 | 0 | 0 | 0 | 0 | 0 | 0 | 0 | 0 | 0 | 0 | 0 | 0 | 0 | 0 | 0 | 0 | 0 | 0 | 0 | 0 | 0 | | | | | | |
| 1096 | 01238 | 01095 | 2 | | 0 | 2 | 0 | 0 | 0 | 0 | 0 | 0 | 0 | 0 | 0 | 0 | 0 | 0 | 0 | 0 | 0 | 0 | 0 | 0 | 0 | 0 | 0 | 0 | 0 | | | | | | |
| 1097 | 01239 | 01096 | 8 | | 0 | 2 | 1 | 0 | 1 | 0 | 0 | 0 | 0 | 1 | 0 | 0 | 0 | 0 | 0 | 0 | 0 | 0 | 0 | 1 | 0 | 2 | 0 | 0 | 0 | | | | | | |
| 1098 | 01240 | 01097 | 2 | | 0 | 1 | 0 | 0 | 0 | 0 | 0 | 0 | 0 | 1 | 0 | 0 | 0 | 0 | 0 | 0 | 0 | 0 | 0 | 0 | 0 | 0 | 0 | 0 | 0 | | | | | | |
| 1099 | 01241 | 01098 | 1 | | 0 | 1 | 0 | 0 | 0 | 0 | 0 | 0 | 0 | 0 | 0 | 0 | 0 | 0 | 0 | 0 | 0 | 0 | 0 | 0 | 0 | 0 | 0 | 0 | 0 | | | | | | |
| 1100 | 01242 | 01099 | 7 | | 0 | 2 | 0 | 0 | 0 | 0 | 0 | 1 | 0 | 0 | 0 | 2 | 0 | 0 | 0 | 0 | 0 | 0 | 0 | 0 | 0 | 1 | 0 | 1 | 0 | | | | | | |
| 1101 | 01243 | 01100 | 2 | | 0 | 1 | 1 | 0 | 0 | 0 | 0 | 0 | 0 | 0 | 0 | 0 | 0 | 0 | 0 | 0 | 0 | 0 | 0 | 0 | 0 | 0 | 0 | 0 | 0 | | | | | | |

Table 31

| | A | B | C | E | G | I | K | M | O | Q | S | U | W | Y | AA | AC | AE | AG | AI | AK | AM | AO | AQ | AS | AU | AW | AY | BA | BC | BE | BF | BG | BH | BI | BK |
|---|---|---|---|---|---|---|---|---|---|---|---|---|---|---|---|---|---|---|---|---|---|---|---|---|---|---|---|---|---|---|---|---|---|---|---|
| 1102 | 01244 | 01101 | 25 | | 0 | 3 | 1 | 0 | 0 | 0 | 0 | 2 | 2 | 0 | 3 | 2 | 2 | 0 | 0 | 2 | 1 | 0 | 4 | 0 | 2 | 1 | 0 | 0 | 0 | X13585 | 93.5 | 262 | 1 | 254 | 518 |
| 1103 | 01245 | 01102 | 2 | | 0 | 1 | 0 | 0 | 0 | 0 | 0 | 0 | 0 | 0 | 0 | 0 | 0 | 0 | 0 | 0 | 0 | 0 | 0 | 1 | 0 | 0 | 0 | 0 | 0 | | | | | | |
| 1104 | 01246 | 01103 | 2 | | 0 | 1 | 0 | 0 | 0 | 0 | 0 | 0 | 0 | 0 | 0 | 0 | 0 | 0 | 0 | 0 | 0 | 0 | 0 | 0 | 0 | 1 | 0 | 0 | 0 | | | | | | |
| 1105 | 01247 | 01104 | 3 | | 0 | 1 | 0 | 0 | 0 | 0 | 0 | 0 | 0 | 0 | 0 | 0 | 0 | 0 | 0 | 0 | 0 | 0 | 0 | 0 | 0 | 0 | 2 | 0 | 0 | | | | | | |
| 1106 | 01248 | 01105 | 1 | | 0 | 1 | 0 | 0 | 0 | 0 | 0 | 0 | 0 | 0 | 0 | 0 | 0 | 0 | 0 | 0 | 0 | 0 | 0 | 0 | 0 | 0 | 0 | 0 | 0 | | | | | | |
| 1107 | 01249 | 01106 | 1 | | 0 | 1 | 0 | 0 | 0 | 0 | 0 | 0 | 0 | 0 | 0 | 0 | 0 | 0 | 0 | 0 | 0 | 0 | 0 | 0 | 0 | 0 | 0 | 0 | 0 | | | | | | |
| 1108 | 01250 | 01107 | 2 | | 0 | 1 | 0 | 0 | 0 | 1 | 0 | 0 | 0 | 0 | 0 | 0 | 0 | 0 | 0 | 0 | 0 | 0 | 0 | 0 | 0 | 0 | 0 | 0 | 0 | | | | | | |
| 1109 | 01251 | 01108 | 1 | | 0 | 1 | 0 | 0 | 0 | 0 | 0 | 0 | 0 | 0 | 0 | 0 | 0 | 0 | 0 | 0 | 0 | 0 | 0 | 0 | 0 | 0 | 0 | 0 | 0 | | | | | | |
| 1110 | 01252 | 01109 | 1 | | 0 | 1 | 0 | 0 | 0 | 0 | 0 | 0 | 0 | 0 | 0 | 0 | 0 | 0 | 0 | 0 | 0 | 0 | 0 | 0 | 0 | 0 | 0 | 0 | 0 | | | | | | |
| 1111 | 01253 | 01110 | 1 | | 0 | 1 | 0 | 0 | 0 | 0 | 0 | 0 | 0 | 0 | 0 | 0 | 0 | 0 | 0 | 0 | 0 | 0 | 0 | 0 | 0 | 0 | 0 | 0 | 0 | | | | | | |
| 1112 | 01254 | 01111 | 1 | | 0 | 1 | 0 | 0 | 0 | 0 | 0 | 0 | 0 | 0 | 0 | 0 | 0 | 0 | 0 | 0 | 0 | 0 | 0 | 0 | 0 | 0 | 0 | 0 | 0 | | | | | | |
| 1113 | 01256 | 01112 | 5 | | 0 | 1 | 0 | 0 | 0 | 0 | 0 | 0 | 0 | 0 | 0 | 1 | 1 | 0 | 0 | 0 | 2 | 0 | 0 | 0 | 0 | 0 | 0 | 0 | 0 | J05016 | 92.5 | 267 | 1 | 1912 | 2865 |
| 1114 | 01257 | 01113 | 2 | | 0 | 1 | 0 | 0 | 0 | 0 | 0 | 0 | 0 | 1 | 0 | 0 | 0 | 0 | 0 | 0 | 0 | 0 | 0 | 0 | 0 | 0 | 0 | 0 | 0 | M37712 | 94.1 | 237 | 1 | 1486 | 3863 |
| 1115 | 01258 | 01114 | 1 | | 0 | 1 | 0 | 0 | 0 | 0 | 0 | 0 | 0 | 0 | 0 | 0 | 0 | 0 | 0 | 0 | 0 | 0 | 0 | 0 | 0 | 0 | 0 | 0 | 0 | | | | | | |
| 1116 | 01259 | 01115 | 3 | | 0 | 1 | 0 | 0 | 0 | 0 | 0 | 0 | 0 | 0 | 0 | 0 | 0 | 0 | 0 | 0 | 0 | 0 | 0 | 2 | 0 | 0 | 0 | 0 | 0 | | | | | | |
| 1117 | 01260 | 01116 | 2 | | 0 | 1 | 0 | 0 | 0 | 0 | 0 | 0 | 0 | 0 | 0 | 0 | 0 | 0 | 0 | 0 | 0 | 0 | 1 | 0 | 0 | 0 | 0 | 0 | 0 | | | | | | |
| 1118 | 01261 | 01117 | 1 | | 0 | 1 | 0 | 0 | 0 | 0 | 0 | 0 | 0 | 0 | 0 | 0 | 0 | 0 | 0 | 0 | 0 | 0 | 0 | 0 | 0 | 0 | 0 | 0 | 0 | D13638 | 94.5 | 235 | 1 | 3809 | 5167 |
| 1119 | 01262 | 01118 | 1 | | 0 | 1 | 0 | 0 | 0 | 0 | 0 | 0 | 0 | 0 | 0 | 0 | 0 | 0 | 0 | 0 | 0 | 0 | 0 | 0 | 0 | 0 | 0 | 0 | 0 | | | | | | |
| 1120 | 01263 | 01119 | 5 | | 0 | 2 | 0 | 0 | 0 | 0 | 0 | 0 | 1 | 0 | 0 | 0 | 0 | 1 | 0 | 0 | 0 | 0 | 0 | 0 | 0 | 0 | 1 | 0 | 0 | M60346 | 92.4 | 172 | 1 | 2288 | 2457 |
| 1121 | 01264 | 01120 | 4 | | 0 | 1 | 0 | 0 | 0 | 0 | 0 | 0 | 0 | 0 | 0 | 0 | 1 | 0 | 0 | 0 | 0 | 0 | 1 | 1 | 0 | 0 | 0 | 0 | 0 | | | | | | |
| 1122 | 01265 | 01121 | 2 | | 0 | 1 | 0 | 0 | 0 | 0 | 0 | 0 | 0 | 0 | 0 | 0 | 0 | 0 | 0 | 0 | 0 | 0 | 1 | 0 | 0 | 0 | 0 | 0 | 0 | D25253 | 99.6 | 234 | 1 | 115 | 360 |
| 1123 | 01266 | 01122 | 1 | | 0 | 1 | 0 | 0 | 0 | 0 | 0 | 0 | 0 | 0 | 0 | 0 | 0 | 0 | 0 | 0 | 0 | 0 | 0 | 0 | 0 | 0 | 0 | 0 | 0 | | | | | | |
| 1124 | 01267 | 01123 | 15 | | 0 | 1 | 0 | 0 | 0 | 0 | 0 | 0 | 0 | 1 | 0 | 0 | 0 | 0 | 1 | 1 | 0 | 2 | 0 | 0 | 0 | 0 | 8 | 0 | 1 | X02490 | 99.5 | 215 | 1 | 354 | 568 |
| 1125 | 01268 | 01124 | 4 | | 0 | 1 | 0 | 0 | 0 | 0 | 0 | 0 | 0 | 0 | 0 | 1 | 1 | 0 | 0 | 0 | 1 | 0 | 0 | 0 | 0 | 0 | 0 | 0 | 0 | | | | | | |
| 1126 | 01269 | 01125 | 1 | | 0 | 1 | 0 | 0 | 0 | 0 | 0 | 0 | 0 | 0 | 0 | 0 | 0 | 0 | 0 | 0 | 0 | 0 | 0 | 0 | 0 | 0 | 0 | 0 | 0 | L26953 | 97 | 231 | 1 | 350 | 2868 |
| 1127 | 01270 | 01126 | 1 | | 0 | 1 | 0 | 0 | 0 | 0 | 0 | 0 | 0 | 0 | 0 | 0 | 0 | 0 | 0 | 0 | 0 | 0 | 0 | 0 | 0 | 0 | 0 | 0 | 0 | | | | | | |
| 1128 | 01272 | 01127 | 1 | | 0 | 1 | 0 | 0 | 0 | 0 | 0 | 0 | 0 | 0 | 0 | 0 | 0 | 0 | 0 | 0 | 0 | 0 | 0 | 0 | 0 | 0 | 0 | 0 | 0 | | | | | | |
| 1129 | 01273 | 01128 | 4 | | 0 | 1 | 0 | 0 | 0 | 0 | 0 | 0 | 0 | 0 | 0 | 0 | 0 | 0 | 1 | 0 | 0 | 0 | 0 | 2 | 0 | 0 | 0 | 0 | 0 | D13286 | 97 | 230 | 1 | 2037 | 2272 |
| 1130 | 01274 | 01129 | 1 | | 0 | 1 | 0 | 0 | 0 | 0 | 0 | 0 | 0 | 0 | 0 | 0 | 0 | 0 | 0 | 0 | 0 | 0 | 0 | 0 | 0 | 0 | 0 | 0 | 0 | | | | | | |
| 1131 | 01276 | 01130 | 1 | | 0 | 1 | 0 | 0 | 0 | 0 | 0 | 0 | 0 | 0 | 0 | 0 | 0 | 0 | 0 | 0 | 0 | 0 | 0 | 0 | 0 | 0 | 0 | 0 | 0 | | | | | | |
| 1132 | 01277 | 01131 | 1 | | 0 | 1 | 0 | 0 | 0 | 0 | 0 | 0 | 0 | 0 | 0 | 0 | 0 | 0 | 0 | 0 | 0 | 0 | 0 | 0 | 0 | 0 | 0 | 0 | 0 | | | | | | |
| 1133 | 01278 | 01132 | 2 | | 0 | 1 | 0 | 0 | 0 | 0 | 0 | 0 | 0 | 0 | 1 | 0 | 0 | 0 | 0 | 0 | 0 | 0 | 0 | 0 | 0 | 0 | 0 | 0 | 0 | | | | | | |
| 1134 | 01279 | 01133 | 6 | | 0 | 3 | 0 | 0 | 0 | 0 | 0 | 0 | 0 | 0 | 0 | 1 | 0 | 0 | 0 | 1 | 0 | 0 | 0 | 0 | 0 | 0 | 0 | 1 | 0 | | | | | | |
| 1135 | 01280 | 01134 | 1 | | 0 | 1 | 0 | 0 | 0 | 0 | 0 | 0 | 0 | 0 | 0 | 0 | 0 | 0 | 0 | 0 | 0 | 0 | 0 | 0 | 0 | 0 | 0 | 0 | 0 | | | | | | |
| 1136 | 01281 | 01135 | 1 | | 0 | 1 | 0 | 0 | 0 | 0 | 0 | 0 | 0 | 0 | 0 | 0 | 0 | 0 | 0 | 0 | 0 | 0 | 0 | 0 | 0 | 0 | 0 | 0 | 0 | | | | | | |
| 1137 | 01282 | 01136 | 6 | | 0 | 1 | 1 | 0 | 0 | 0 | 0 | 0 | 0 | 0 | 0 | 1 | 0 | 0 | 1 | 0 | 0 | 0 | 0 | 0 | 1 | 1 | 0 | 0 | 0 | L09604 | 92 | 199 | 1 | 748 | 945 |

Table 32

| | A | B | C | E | G | I | K | M | O | Q | S | U | W | Y | AA | AC | AE | AG | AI | AK | AM | AO | AQ | AS | AU | AW | AY | BA | BC | BE | BF | BG | BH | BI | BK |
|---|---|---|---|---|---|---|---|---|---|---|---|---|---|---|---|---|---|---|---|---|---|---|---|---|---|---|---|---|---|---|---|---|---|---|---|
| 1138 | 01283 | 01137 | 9 | | 0 | 2 | 1 | 0 | 0 | 1 | 1 | 0 | 0 | 0 | 0 | 1 | 0 | 0 | 0 | 0 | 0 | 0 | 1 | 0 | 0 | 1 | 0 | 1 | 0 | M30448 | 98.6 | 222 | 1 | 669 | 2527 |
| 1139 | 01284 | 01138 | 2 | | 0 | 1 | 0 | 0 | 0 | 0 | 0 | 0 | 0 | 1 | 0 | 0 | 0 | 0 | 0 | 0 | 0 | 0 | 0 | 0 | 0 | 0 | 0 | 0 | 0 | | | | | | |
| 1140 | 01285 | 01139 | 6 | | 0 | 1 | 0 | 0 | 0 | 0 | 0 | 1 | 0 | 0 | 1 | 0 | 1 | 0 | 0 | 0 | 0 | 0 | 0 | 1 | 0 | 1 | 0 | 0 | 0 | | | | | | |
| 1141 | 01286 | 01140 | 2 | | 0 | 1 | 0 | 0 | 0 | 0 | 0 | 0 | 0 | 0 | 0 | 0 | 0 | 0 | 0 | 0 | 0 | 0 | 0 | 0 | 0 | 0 | 1 | 0 | 0 | | | | | | |
| 1142 | 01287 | 01141 | 2 | | 0 | 1 | 0 | 0 | 0 | 0 | 0 | 0 | 0 | 0 | 0 | 0 | 0 | 0 | 0 | 0 | 0 | 0 | 0 | 0 | 0 | 1 | 0 | 0 | 0 | | | | | | |
| 1143 | 01288 | 01142 | 4 | | 0 | 1 | 0 | 0 | 0 | 0 | 0 | 0 | 0 | 0 | 0 | 0 | 0 | 0 | 0 | 0 | 0 | 0 | 0 | 0 | 0 | 0 | 3 | 0 | 0 | | | | | | |
| 1144 | 01289 | 01143 | 1 | | 0 | 1 | 0 | 0 | 0 | 0 | 0 | 0 | 0 | 0 | 0 | 0 | 0 | 0 | 0 | 0 | 0 | 0 | 0 | 0 | 0 | 0 | 0 | 0 | 0 | | | | | | |
| 1145 | 01290 | 01144 | 2 | | 0 | 1 | 0 | 0 | 0 | 0 | 0 | 0 | 0 | 0 | 0 | 1 | 0 | 0 | 0 | 0 | 0 | 0 | 0 | 0 | 0 | 0 | 0 | 0 | 0 | | | | | | |
| 1146 | 01291 | 01145 | 2 | | 0 | 1 | 0 | 0 | 0 | 0 | 0 | 0 | 0 | 0 | 0 | 0 | 0 | 0 | 0 | 0 | 0 | 0 | 0 | 0 | 1 | 0 | 0 | 0 | 0 | | | | | | |
| 1147 | 01292 | 01146 | 2 | | 0 | 1 | 0 | 0 | 0 | 0 | 0 | 0 | 0 | 0 | 0 | 0 | 0 | 0 | 0 | 1 | 0 | 0 | 0 | 0 | 0 | 0 | 0 | 0 | 0 | L06797 | 100 | 218 | 1 | 1439 | 1670 |
| 1148 | 01293 | 01147 | 1 | | 0 | 1 | 0 | 0 | 0 | 0 | 0 | 0 | 0 | 0 | 0 | 0 | 0 | 0 | 0 | 0 | 0 | 0 | 0 | 0 | 0 | 0 | 0 | 0 | 0 | | | | | | |
| 1149 | 01294 | 01148 | 1 | | 0 | 1 | 0 | 0 | 0 | 0 | 0 | 0 | 0 | 0 | 0 | 0 | 0 | 0 | 0 | 0 | 0 | 0 | 0 | 0 | 0 | 0 | 0 | 0 | 0 | | | | | | |
| 1150 | 01295 | 01149 | 3 | | 0 | 1 | 1 | 0 | 0 | 0 | 0 | 0 | 0 | 0 | 0 | 0 | 0 | 0 | 0 | 0 | 1 | 0 | 0 | 0 | 0 | 0 | 0 | 0 | 0 | | | | | | |
| 1151 | 01296 | 01150 | 6 | | 0 | 1 | 0 | 3 | 0 | 0 | 0 | 0 | 0 | 0 | 0 | 0 | 2 | 0 | 0 | 0 | 0 | 0 | 0 | 0 | 0 | 0 | 0 | 0 | 0 | | | | | | |
| 1152 | 01297 | 01151 | 2 | | 0 | 1 | 0 | 0 | 0 | 0 | 0 | 0 | 0 | 0 | 0 | 0 | 0 | 0 | 0 | 0 | 0 | 1 | 0 | 0 | 0 | 0 | 0 | 0 | 0 | | | | | | |
| 1153 | 01298 | 01152 | 4 | | 0 | 1 | 0 | 0 | 0 | 0 | 0 | 0 | 0 | 0 | 0 | 1 | 0 | 0 | 1 | 0 | 0 | 0 | 0 | 0 | 0 | 0 | 0 | 1 | 0 | | | | | | |
| 1154 | 01299 | 01153 | 1 | | 0 | 1 | 0 | 0 | 0 | 0 | 0 | 0 | 0 | 0 | 0 | 0 | 0 | 0 | 0 | 0 | 0 | 0 | 0 | 0 | 0 | 0 | 0 | 0 | 0 | | | | | | |
| 1155 | 01300 | 01154 | 1 | | 0 | 1 | 0 | 0 | 0 | 0 | 0 | 0 | 0 | 0 | 0 | 0 | 0 | 0 | 0 | 0 | 0 | 0 | 0 | 0 | 0 | 0 | 0 | 0 | 0 | | | | | | |
| 1156 | 01301 | 01155 | 5 | | 0 | 1 | 0 | 0 | 0 | 0 | 0 | 0 | 1 | 0 | 2 | 0 | 0 | 0 | 0 | 0 | 0 | 0 | 0 | 0 | 0 | 0 | 1 | 0 | 0 | | | | | | |
| 1157 | 01302 | 01156 | 1 | | 0 | 1 | 0 | 0 | 0 | 0 | 0 | 0 | 0 | 0 | 0 | 0 | 0 | 0 | 0 | 0 | 0 | 0 | 0 | 0 | 0 | 0 | 0 | 0 | 0 | | | | | | |
| 1158 | 01303 | 01157 | 1 | | 0 | 1 | 0 | 0 | 0 | 0 | 0 | 0 | 0 | 0 | 0 | 0 | 0 | 0 | 0 | 0 | 0 | 0 | 0 | 0 | 0 | 0 | 0 | 0 | 0 | | | | | | |
| 1159 | 01304 | 01158 | 10 | | 0 | 1 | 3 | 0 | 0 | 0 | 0 | 0 | 0 | 0 | 0 | 0 | 0 | 0 | 1 | 0 | 0 | 0 | 0 | 0 | 1 | 0 | 4 | 0 | 0 | M37033 | 100 | 209 | 1 | 1272 | 1480 |
| 1160 | 01305 | 01159 | 28 | | 0 | 2 | 1 | 0 | 1 | 3 | 0 | 0 | 1 | 1 | 0 | 0 | 1 | 3 | 1 | 3 | 0 | 0 | 3 | 5 | 2 | 0 | 1 | 0 | 0 | | | | | | |
| 1161 | 01306 | 01160 | 3 | | 0 | 1 | 0 | 0 | 0 | 1 | 0 | 0 | 0 | 0 | 0 | 0 | 1 | 0 | 0 | 0 | 0 | 0 | 0 | 0 | 0 | 0 | 0 | 0 | 0 | | | | | | |
| 1162 | 01307 | 01161 | 2 | | 0 | 1 | 0 | 0 | 0 | 0 | 0 | 0 | 0 | 0 | 1 | 0 | 0 | 0 | 0 | 0 | 0 | 0 | 0 | 0 | 0 | 0 | 0 | 0 | 0 | | | | | | |
| 1163 | 01308 | 01162 | 1 | | 0 | 1 | 0 | 0 | 0 | 0 | 0 | 0 | 0 | 0 | 0 | 0 | 0 | 0 | 0 | 0 | 0 | 0 | 0 | 0 | 0 | 0 | 0 | 0 | 0 | | | | | | |
| 1164 | 01309 | 01163 | 1 | | 0 | 1 | 0 | 0 | 0 | 0 | 0 | 0 | 0 | 0 | 0 | 0 | 0 | 0 | 0 | 0 | 0 | 0 | 0 | 0 | 0 | 0 | 0 | 0 | 0 | | | | | | |
| 1165 | 01310 | 01164 | 1 | | 0 | 1 | 0 | 0 | 0 | 0 | 0 | 0 | 0 | 0 | 0 | 0 | 0 | 0 | 0 | 0 | 0 | 0 | 0 | 0 | 0 | 0 | 0 | 0 | 0 | | | | | | |
| 1166 | 01311 | 01165 | 6 | | 0 | 2 | 0 | 0 | 0 | 0 | 0 | 0 | 0 | 1 | 0 | 0 | 0 | 0 | 0 | 0 | 0 | 1 | 0 | 2 | 0 | 0 | 0 | 0 | 0 | | | | | | |
| 1167 | 01312 | 01166 | 1 | | 0 | 1 | 0 | 0 | 0 | 0 | 0 | 0 | 0 | 0 | 0 | 0 | 0 | 0 | 0 | 0 | 0 | 0 | 0 | 0 | 0 | 0 | 0 | 0 | 0 | | | | | | |
| 1168 | 01313 | 01167 | 1 | | 0 | 1 | 0 | 0 | 0 | 0 | 0 | 0 | 0 | 0 | 0 | 0 | 0 | 0 | 0 | 0 | 0 | 0 | 0 | 0 | 0 | 0 | 0 | 0 | 0 | | | | | | |
| 1169 | 01314 | 01168 | 3 | | 0 | 1 | 0 | 0 | 0 | 0 | 0 | 0 | 1 | 0 | 0 | 0 | 0 | 0 | 0 | 0 | 0 | 1 | 0 | 0 | 0 | 0 | 0 | 0 | 0 | | | | | | |
| 1170 | 01315 | 01169 | 8 | | 0 | 1 | 1 | 0 | 0 | 0 | 0 | 0 | 0 | 0 | 0 | 1 | 2 | 1 | 0 | 1 | 0 | 0 | 1 | 0 | 0 | 0 | 0 | 0 | 0 | | | | | | |
| 1171 | 01316 | 01170 | 5 | | 0 | 1 | 0 | 0 | 0 | 0 | 1 | 0 | 0 | 0 | 1 | 0 | 1 | 0 | 0 | 0 | 1 | 0 | 0 | 0 | 0 | 0 | 0 | 0 | 0 | | | | | | |
| 1172 | 01317 | 01171 | 1 | | 0 | 1 | 0 | 0 | 0 | 0 | 0 | 0 | 0 | 0 | 0 | 0 | 0 | 0 | 0 | 0 | 0 | 0 | 0 | 0 | 0 | 0 | 0 | 0 | 0 | | | | | | |
| 1173 | 01318 | 01172 | 3 | | 0 | 1 | 0 | 0 | 0 | 0 | 0 | 0 | 0 | 0 | 1 | 0 | 0 | 0 | 0 | 0 | 0 | 0 | 0 | 0 | 0 | 0 | 0 | 1 | 0 | | | | | | |

Table 33

| | A | B | C | E | G | I | K | M | O | Q | S | U | W | Y | AA | AC | AE | AG | AI | AK | AM | AO | AQ | AS | AU | AW | AY | BA | BC | BE | BF | BG | BH | BI | BK |
|---|---|---|---|---|---|---|---|---|---|---|---|---|---|---|---|---|---|---|---|---|---|---|---|---|---|---|---|---|---|---|---|---|---|---|---|
| 1174 | 01319 | 01173 | 1 | | 0 | 1 | 0 | 0 | 0 | 0 | 0 | 0 | 0 | 0 | 0 | 0 | 0 | 0 | 0 | 0 | 0 | 0 | 0 | 0 | 0 | 0 | 0 | 0 | 0 | | | | | | |
| 1175 | 01320 | 01174 | 1 | | 0 | 1 | 0 | 0 | 0 | 0 | 0 | 0 | 0 | 0 | 0 | 0 | 0 | 0 | 0 | 0 | 0 | 0 | 0 | 0 | 0 | 0 | 0 | 0 | 0 | | | | | | |
| 1176 | 01321 | 01175 | 19 | | 0 | 3 | 2 | 1 | 2 | 0 | 1 | 1 | 0 | 0 | 1 | 0 | 2 | 0 | 0 | 0 | 0 | 0 | 0 | 1 | 0 | 2 | 2 | 1 | 0 | | | | | | |
| 1177 | 01322 | 01176 | 3 | | 0 | 2 | 0 | 0 | 0 | 0 | 0 | 0 | 1 | 0 | 0 | 0 | 0 | 0 | 0 | 0 | 0 | 0 | 0 | 0 | 0 | 0 | 0 | 0 | 0 | | | | | | |
| 1178 | 01323 | 01177 | 1 | | 0 | 1 | 0 | 0 | 0 | 0 | 0 | 0 | 0 | 0 | 0 | 0 | 0 | 0 | 0 | 0 | 0 | 0 | 0 | 0 | 0 | 0 | 0 | 0 | 0 | | | | | | |
| 1179 | 01324 | 01178 | 1 | | 0 | 1 | 0 | 0 | 0 | 0 | 0 | 0 | 0 | 0 | 0 | 0 | 0 | 0 | 0 | 0 | 0 | 0 | 0 | 0 | 0 | 0 | 0 | 0 | 0 | | | | | | |
| 1180 | 01325 | 01179 | 17 | | 0 | 1 | 0 | 0 | 0 | 0 | 0 | 0 | 2 | 0 | 1 | 0 | 1 | 0 | 2 | 1 | 0 | 2 | 0 | 0 | 1 | 2 | 3 | 1 | 0 | L26247 | 98.5 | 203 | 1 | 455 | 660 |
| 1181 | 01326 | 01180 | 1 | | 0 | 1 | 0 | 0 | 0 | 0 | 0 | 0 | 0 | 0 | 0 | 0 | 0 | 0 | 0 | 0 | 0 | 0 | 0 | 0 | 0 | 0 | 0 | 0 | 0 | | | | | | |
| 1182 | 01327 | 01181 | 4 | | 0 | 2 | 0 | 0 | 1 | 0 | 0 | 0 | 1 | 0 | 0 | 0 | 0 | 0 | 0 | 0 | 0 | 0 | 0 | 0 | 0 | 0 | 0 | 0 | 0 | M74524 | 100 | 199 | 1 | 1543 | 1743 |
| 1183 | 01328 | 01182 | 2 | | 0 | 1 | 0 | 0 | 0 | 0 | 0 | 0 | 0 | 0 | 0 | 0 | 0 | 0 | 0 | 0 | 0 | 0 | 0 | 0 | 0 | 0 | 1 | 0 | 0 | D16217 | 93.2 | 251 | 1 | 2131 | 2493 |
| 1184 | 01329 | 01183 | 2 | | 0 | 1 | 0 | 0 | 0 | 0 | 0 | 0 | 0 | 0 | 0 | 0 | 0 | 0 | 0 | 0 | 0 | 0 | 0 | 1 | 0 | 0 | 0 | 0 | 0 | | | | | | |
| 1185 | 01330 | 01184 | 4 | | 0 | 1 | 0 | 0 | 0 | 0 | 0 | 0 | 0 | 1 | 0 | 0 | 0 | 0 | 0 | 0 | 0 | 0 | 0 | 0 | 1 | 0 | 0 | 1 | 0 | M13450 | 99.3 | 146 | 1 | 820 | 1069 |
| 1186 | 01331 | 01185 | 3 | | 0 | 1 | 0 | 0 | 0 | 0 | 0 | 1 | 1 | 0 | 0 | 0 | 0 | 0 | 0 | 0 | 0 | 0 | 0 | 0 | 0 | 0 | 0 | 0 | 0 | | | | | | |
| 1187 | 01332 | 01186 | 3 | | 0 | 1 | 0 | 0 | 0 | 0 | 0 | 0 | 0 | 0 | 0 | 0 | 0 | 1 | 0 | 0 | 0 | 0 | 0 | 1 | 0 | 0 | 0 | 0 | 0 | | | | | | |
| 1188 | 01333 | 01187 | 1 | | 0 | 1 | 0 | 0 | 0 | 0 | 0 | 0 | 0 | 0 | 0 | 0 | 0 | 0 | 0 | 0 | 0 | 0 | 0 | 0 | 0 | 0 | 0 | 0 | 0 | L16862 | 99.5 | 196 | 1 | 1867 | 2848 |
| 1189 | 01334 | 01188 | 7 | | 0 | 2 | 1 | 0 | 0 | 0 | 0 | 0 | 0 | 0 | 0 | 0 | 0 | 0 | 0 | 0 | 0 | 1 | 0 | 0 | 0 | 0 | 3 | 0 | 0 | | | | | | |
| 1190 | 01335 | 01189 | 1 | | 0 | 1 | 0 | 0 | 0 | 0 | 0 | 0 | 0 | 0 | 0 | 0 | 0 | 0 | 0 | 0 | 0 | 0 | 0 | 0 | 0 | 0 | 0 | 0 | 0 | | | | | | |
| 1191 | 01336 | 01190 | 4 | | 0 | 2 | 0 | 1 | 0 | 0 | 0 | 0 | 0 | 0 | 0 | 0 | 0 | 1 | 0 | 0 | 0 | 0 | 0 | 0 | 0 | 0 | 0 | 0 | 0 | M33195 | 98.9 | 186 | 1 | 400 | 591 |
| 1192 | 01337 | 01191 | 1 | | 0 | 1 | 0 | 0 | 0 | 0 | 0 | 0 | 0 | 0 | 0 | 0 | 0 | 0 | 0 | 0 | 0 | 0 | 0 | 0 | 0 | 0 | 0 | 0 | 0 | | | | | | |
| 1193 | 01338 | 01192 | 1 | | 0 | 1 | 0 | 0 | 0 | 0 | 0 | 0 | 0 | 0 | 0 | 0 | 0 | 0 | 0 | 0 | 0 | 0 | 0 | 0 | 0 | 0 | 0 | 0 | 0 | | | | | | |
| 1194 | 01339 | 01193 | 1 | | 0 | 1 | 0 | 0 | 0 | 0 | 0 | 0 | 0 | 0 | 0 | 0 | 0 | 0 | 0 | 0 | 0 | 0 | 0 | 0 | 0 | 0 | 0 | 0 | 0 | X69838 | 98.9 | 188 | 1 | 3190 | 3391 |
| 1195 | 01340 | 01194 | 1 | | 0 | 1 | 0 | 0 | 0 | 0 | 0 | 0 | 0 | 0 | 0 | 0 | 0 | 0 | 0 | 0 | 0 | 0 | 0 | 0 | 0 | 0 | 0 | 0 | 0 | | | | | | |
| 1196 | 01341 | 01195 | 1 | | 0 | 1 | 0 | 0 | 0 | 0 | 0 | 0 | 0 | 0 | 0 | 0 | 0 | 0 | 0 | 0 | 0 | 0 | 0 | 0 | 0 | 0 | 0 | 0 | 0 | | | | | | |
| 1197 | 01342 | 01196 | 1 | | 0 | 1 | 0 | 0 | 0 | 0 | 0 | 0 | 0 | 0 | 0 | 0 | 0 | 0 | 0 | 0 | 0 | 0 | 0 | 0 | 0 | 0 | 0 | 0 | 0 | | | | | | |
| 1198 | 01343 | 01197 | 1 | | 0 | 1 | 0 | 0 | 0 | 0 | 0 | 0 | 0 | 0 | 0 | 0 | 0 | 0 | 0 | 0 | 0 | 0 | 0 | 0 | 0 | 0 | 0 | 0 | 0 | | | | | | |
| 1199 | 01344 | 01198 | 2 | | 0 | 1 | 0 | 0 | 0 | 0 | 0 | 0 | 0 | 0 | 0 | 0 | 0 | 0 | 0 | 0 | 0 | 0 | 0 | 1 | 0 | 0 | 0 | 0 | 0 | | | | | | |
| 1200 | 01345 | 01199 | 4 | | 0 | 4 | 0 | 0 | 0 | 0 | 0 | 0 | 0 | 0 | 0 | 0 | 0 | 0 | 0 | 0 | 0 | 0 | 0 | 0 | 0 | 0 | 0 | 0 | 0 | J04739 | 98.3 | 180 | 1 | 1634 | 1813 |
| 1201 | 01346 | 01200 | 1 | | 0 | 1 | 0 | 0 | 0 | 0 | 0 | 0 | 0 | 0 | 0 | 0 | 0 | 0 | 0 | 0 | 0 | 0 | 0 | 0 | 0 | 0 | 0 | 0 | 0 | | | | | | |
| 1202 | 01347 | 01201 | 5 | | 0 | 1 | 0 | 0 | 0 | 0 | 0 | 0 | 1 | 0 | 0 | 0 | 0 | 0 | 0 | 0 | 0 | 1 | 0 | 0 | 0 | 0 | 0 | 0 | 2 | | | | | | |
| 1203 | 01348 | 01202 | 13 | | 0 | 2 | 0 | 0 | 0 | 1 | 1 | 0 | 0 | 1 | 2 | 4 | 0 | 0 | 0 | 0 | 0 | 0 | 0 | 0 | 0 | 1 | 0 | 1 | 0 | | | | | | |
| 1204 | 01349 | 01203 | 1 | | 0 | 1 | 0 | 0 | 0 | 0 | 0 | 0 | 0 | 0 | 0 | 0 | 0 | 0 | 0 | 0 | 0 | 0 | 0 | 0 | 0 | 0 | 0 | 0 | 0 | | | | | | |
| 1205 | 01350 | 01204 | 1 | | 0 | 1 | 0 | 0 | 0 | 0 | 0 | 0 | 0 | 0 | 0 | 0 | 0 | 0 | 0 | 0 | 0 | 0 | 0 | 0 | 0 | 0 | 0 | 0 | 0 | | | | | | |
| 1206 | 01351 | 01205 | 7 | | 0 | 1 | 0 | 0 | 1 | 0 | 0 | 0 | 0 | 0 | 2 | 0 | 0 | 0 | 0 | 0 | 0 | 0 | 0 | 2 | 0 | 0 | 0 | 1 | 0 | | | | | | |
| 1207 | 01352 | 01206 | 2 | | 0 | 2 | 0 | 0 | 0 | 0 | 0 | 0 | 0 | 0 | 0 | 0 | 0 | 0 | 0 | 0 | 0 | 0 | 0 | 0 | 0 | 0 | 0 | 0 | 0 | | | | | | |
| 1208 | 01353 | 01207 | 2 | | 0 | 1 | 0 | 0 | 0 | 0 | 0 | 0 | 0 | 0 | 0 | 1 | 0 | 0 | 0 | 0 | 0 | 0 | 0 | 0 | 0 | 0 | 0 | 0 | 0 | | | | | | |
| 1209 | 01354 | 01208 | 2 | | 0 | 1 | 0 | 0 | 0 | 0 | 0 | 0 | 0 | 0 | 0 | 0 | 0 | 0 | 0 | 0 | 0 | 0 | 0 | 1 | 0 | 0 | 0 | 0 | 0 | | | | | | |

Table 34

| | A | B | C | E | G | I | K | M | O | Q | S | U | W | Y | AA | AC | AE | AG | AI | AK | AM | AO | AQ | AS | AU | AW | AY | BA | BC | BE | BF | BG | BH | BI | BK |
|---|---|---|---|---|---|---|---|---|---|---|---|---|---|---|---|---|---|---|---|---|---|---|---|---|---|---|---|---|---|---|---|---|---|---|---|
| 1210 | 01355 | 01209 | 2 | | 0 | 1 | 0 | 0 | 0 | 0 | 0 | 0 | 0 | 0 | 0 | 0 | 0 | 0 | 0 | 0 | 0 | 0 | 0 | 0 | 1 | 0 | 0 | 0 | 0 | | | | | | |
| 1211 | 01356 | 01210 | 2 | | 0 | 1 | 0 | 0 | 0 | 0 | 0 | 0 | 0 | 0 | 0 | 0 | 0 | 0 | 0 | 0 | 0 | 0 | 0 | 0 | 0 | 0 | 1 | 0 | 0 | | | | | | |
| 1212 | 01357 | 01211 | 1 | | 0 | 1 | 0 | 0 | 0 | 0 | 0 | 0 | 0 | 0 | 0 | 0 | 0 | 0 | 0 | 0 | 0 | 0 | 0 | 0 | 0 | 0 | 0 | 0 | 0 | | | | | | |
| 1213 | 01358 | 01212 | 1 | | 0 | 1 | 0 | 0 | 0 | 0 | 0 | 0 | 0 | 0 | 0 | 0 | 0 | 0 | 0 | 0 | 0 | 0 | 0 | 0 | 0 | 0 | 0 | 0 | 0 | L22473 | 95.3 | 127 | 1 | 453 | 579 |
| 1214 | 01359 | 01213 | 2 | | 0 | 1 | 0 | 0 | 0 | 0 | 0 | 0 | 0 | 0 | 0 | 0 | 0 | 0 | 0 | 0 | 1 | 0 | 0 | 0 | 0 | 0 | 0 | 0 | 0 | | | | | | |
| 1215 | 01360 | 01214 | 1 | | 0 | 1 | 0 | 0 | 0 | 0 | 0 | 0 | 0 | 0 | 0 | 0 | 0 | 0 | 0 | 0 | 0 | 0 | 0 | 0 | 0 | 0 | 0 | 0 | 0 | | | | | | |
| 1216 | 01361 | 01215 | 1 | | 0 | 1 | 0 | 0 | 0 | 0 | 0 | 0 | 0 | 0 | 0 | 0 | 0 | 0 | 0 | 0 | 0 | 0 | 0 | 0 | 0 | 0 | 0 | 0 | 0 | | | | | | |
| 1217 | 01362 | 01216 | 1 | | 0 | 1 | 0 | 0 | 0 | 0 | 0 | 0 | 0 | 0 | 0 | 0 | 0 | 0 | 0 | 0 | 0 | 0 | 0 | 0 | 0 | 0 | 0 | 0 | 0 | | | | | | |
| 1218 | 01363 | 01217 | 1 | | 0 | 1 | 0 | 0 | 0 | 0 | 0 | 0 | 0 | 0 | 0 | 0 | 0 | 0 | 0 | 0 | 0 | 0 | 0 | 0 | 0 | 0 | 0 | 0 | 0 | | | | | | |
| 1219 | 01364 | 01218 | 1 | | 0 | 1 | 0 | 0 | 0 | 0 | 0 | 0 | 0 | 0 | 0 | 0 | 0 | 0 | 0 | 0 | 0 | 0 | 0 | 0 | 0 | 0 | 0 | 0 | 0 | | | | | | |
| 1220 | 01365 | 01219 | 4 | | 0 | 3 | 0 | 0 | 0 | 0 | 0 | 0 | 0 | 0 | 0 | 0 | 0 | 0 | 0 | 0 | 0 | 0 | 0 | 0 | 0 | 0 | 1 | 0 | 0 | | | | | | |
| 1221 | 01366 | 01220 | 1 | | 0 | 1 | 0 | 0 | 0 | 0 | 0 | 0 | 0 | 0 | 0 | 0 | 0 | 0 | 0 | 0 | 0 | 0 | 0 | 0 | 0 | 0 | 0 | 0 | 0 | | | | | | |
| 1222 | 01367 | 01221 | 21 | | 0 | 3 | 1 | 0 | 0 | 0 | 3 | 1 | 0 | 0 | 0 | 0 | 1 | 0 | 1 | 0 | 0 | 1 | 0 | 0 | 1 | 0 | 7 | 1 | 1 | Z29505 | 97.2 | 216 | 1 | 1324 | 1558 |
| 1223 | 01368 | 01222 | 1 | | 0 | 1 | 0 | 0 | 0 | 0 | 0 | 0 | 0 | 0 | 0 | 0 | 0 | 0 | 0 | 0 | 0 | 0 | 0 | 0 | 0 | 0 | 0 | 0 | 0 | | | | | | |
| 1224 | 01369 | 01223 | 5 | | 0 | 1 | 0 | 0 | 0 | 0 | 1 | 1 | 1 | 0 | 0 | 0 | 0 | 0 | 0 | 0 | 1 | 0 | 0 | 0 | 0 | 0 | 0 | 0 | 0 | | | | | | |
| 1225 | 01370 | 01224 | 1 | | 0 | 1 | 0 | 0 | 0 | 0 | 0 | 0 | 0 | 0 | 0 | 0 | 0 | 0 | 0 | 0 | 0 | 0 | 0 | 0 | 0 | 0 | 0 | 0 | 0 | | | | | | |
| 1226 | 01371 | 01225 | 5 | | 0 | 1 | 0 | 0 | 0 | 0 | 0 | 0 | 0 | 0 | 0 | 0 | 0 | 0 | 0 | 0 | 0 | 0 | 0 | 0 | 0 | 0 | 4 | 0 | 0 | | | | | | |
| 1227 | 01372 | 01226 | 7 | | 0 | 3 | 0 | 0 | 0 | 0 | 0 | 0 | 0 | 0 | 0 | 0 | 0 | 0 | 1 | 0 | 0 | 0 | 0 | 0 | 1 | 2 | 0 | 0 | 0 | X06233 | 91.4 | 58 | 1 | 405 | 462 |
| 1228 | 01373 | 01227 | 2 | | 0 | 1 | 0 | 0 | 0 | 0 | 0 | 0 | 0 | 0 | 0 | 0 | 0 | 0 | 1 | 0 | 0 | 0 | 0 | 0 | 0 | 0 | 0 | 0 | 0 | | | | | | |
| 1229 | 01374 | 01228 | 1 | | 0 | 1 | 0 | 0 | 0 | 0 | 0 | 0 | 0 | 0 | 0 | 0 | 0 | 0 | 0 | 0 | 0 | 0 | 0 | 0 | 0 | 0 | 0 | 0 | 0 | | | | | | |
| 1230 | 01375 | 01229 | 2 | | 0 | 1 | 0 | 0 | 0 | 0 | 0 | 0 | 0 | 0 | 0 | 0 | 0 | 0 | 0 | 0 | 0 | 0 | 0 | 0 | 0 | 0 | 1 | 0 | 0 | | | | | | |
| 1231 | 01377 | 01230 | 3 | | 0 | 1 | 0 | 0 | 0 | 0 | 0 | 0 | 0 | 0 | 1 | 0 | 0 | 0 | 0 | 0 | 0 | 0 | 0 | 0 | 1 | 0 | 0 | 0 | 0 | | | | | | |
| 1232 | 01378 | 01231 | 5 | | 0 | 1 | 0 | 0 | 0 | 0 | 0 | 0 | 1 | 0 | 0 | 0 | 0 | 0 | 0 | 0 | 0 | 1 | 0 | 0 | 0 | 0 | 0 | 2 | 0 | | | | | | |
| 1233 | 01379 | 01232 | 1 | | 0 | 1 | 0 | 0 | 0 | 0 | 0 | 0 | 0 | 0 | 0 | 0 | 0 | 0 | 0 | 0 | 0 | 0 | 0 | 0 | 0 | 0 | 0 | 0 | 0 | | | | | | |
| 1234 | 01380 | 01233 | 1 | | 0 | 1 | 0 | 0 | 0 | 0 | 0 | 0 | 0 | 0 | 0 | 0 | 0 | 0 | 0 | 0 | 0 | 0 | 0 | 0 | 0 | 0 | 0 | 0 | 0 | | | | | | |
| 1235 | 01381 | 01234 | 1 | | 0 | 1 | 0 | 0 | 0 | 0 | 0 | 0 | 0 | 0 | 0 | 0 | 0 | 0 | 0 | 0 | 0 | 0 | 0 | 0 | 0 | 0 | 0 | 0 | 0 | | | | | | |
| 1236 | 01382 | 01235 | 5 | | 0 | 1 | 0 | 0 | 0 | 0 | 3 | 0 | 0 | 0 | 0 | 0 | 0 | 0 | 0 | 0 | 0 | 0 | 0 | 0 | 0 | 0 | 1 | 0 | 0 | | | | | | |
| 1237 | 01383 | 01236 | 3 | | 0 | 2 | 0 | 0 | 0 | 0 | 0 | 0 | 0 | 0 | 0 | 0 | 0 | 0 | 0 | 1 | 0 | 0 | 0 | 0 | 0 | 0 | 0 | 0 | 0 | | | | | | |
| 1238 | 01384 | 01237 | 16 | | 0 | 1 | 1 | 0 | 0 | 0 | 2 | 2 | 0 | 1 | 0 | 0 | 1 | 1 | 2 | 0 | 1 | 0 | 0 | 1 | 1 | 0 | 2 | 0 | 0 | | | | | | |
| 1239 | 01385 | 01238 | 1 | | 0 | 1 | 0 | 0 | 0 | 0 | 0 | 0 | 0 | 0 | 0 | 0 | 0 | 0 | 0 | 0 | 0 | 0 | 0 | 0 | 0 | 0 | 0 | 0 | 0 | | | | | | |
| 1240 | 01386 | 01239 | 3 | | 0 | 1 | 0 | 0 | 0 | 1 | 0 | 0 | 0 | 0 | 0 | 0 | 0 | 0 | 0 | 0 | 0 | 1 | 0 | 0 | 0 | 0 | 0 | 0 | 0 | M86667 | 100 | 124 | 41 | 1287 | 1560 |
| 1241 | 01387 | 01240 | 1 | | 0 | 1 | 0 | 0 | 0 | 0 | 0 | 0 | 0 | 0 | 0 | 0 | 0 | 0 | 0 | 0 | 0 | 0 | 0 | 0 | 0 | 0 | 0 | 0 | 0 | | | | | | |
| 1242 | 01388 | 01241 | 3 | | 0 | 1 | 0 | 0 | 0 | 0 | 0 | 0 | 0 | 0 | 2 | 0 | 0 | 0 | 0 | 0 | 0 | 0 | 0 | 0 | 0 | 0 | 0 | 0 | 0 | L10342 | 99.4 | 163 | 1 | 2 | 2225 |
| 1243 | 01389 | 01242 | 1 | | 0 | 1 | 0 | 0 | 0 | 0 | 0 | 0 | 0 | 0 | 0 | 0 | 0 | 0 | 0 | 0 | 0 | 0 | 0 | 0 | 0 | 0 | 0 | 0 | 0 | | | | | | |
| 1244 | 01390 | 01243 | 5 | | 0 | 1 | 0 | 0 | 0 | 0 | 0 | 0 | 0 | 0 | 0 | 0 | 0 | 0 | 1 | 0 | 0 | 1 | 0 | 0 | 1 | 0 | 0 | 1 | 0 | | | | | | |
| 1245 | 01391 | 01244 | 2 | | 0 | 1 | 0 | 0 | 0 | 0 | 0 | 0 | 0 | 1 | 0 | 0 | 0 | 0 | 0 | 0 | 0 | 0 | 0 | 0 | 0 | 0 | 0 | 0 | 0 | | | | | | |

Table 35

| | A | B | C | E | G | I | K | M | O | Q | S | U | W | Y | AA | AC | AE | AG | AI | AK | AM | AO | AQ | AS | AU | AW | AY | BA | BC | BE | BF | BG | BH | BI | BK |
|---|---|---|---|---|---|---|---|---|---|---|---|---|---|---|---|---|---|---|---|---|---|---|---|---|---|---|---|---|---|---|---|---|---|---|---|
| 1246 | 01392 | 01245 | 1 | | 0 | 1 | 0 | 0 | 0 | 0 | 0 | 0 | 0 | 0 | 0 | 0 | 0 | 0 | 0 | 0 | 0 | 0 | 0 | 0 | 0 | 0 | 0 | 0 | 0 | X03342 | 90.7 | 161 | 1 | 67 | 505 |
| 1247 | 01393 | 01246 | 5 | | 0 | 1 | 0 | 0 | 0 | 1 | 0 | 0 | 0 | 0 | 0 | 1 | 0 | 0 | 0 | 0 | 0 | 0 | 0 | 1 | 0 | 0 | 1 | 0 | 0 | | | | | | |
| 1248 | 01394 | 01247 | 12 | | 0 | 4 | 0 | 0 | 0 | 0 | 0 | 0 | 1 | 2 | 0 | 1 | 1 | 0 | 0 | 0 | 0 | 1 | 0 | 0 | 0 | 0 | 2 | 0 | 0 | | | | | | |
| 1249 | 01395 | 01248 | 7 | | 0 | 1 | 1 | 2 | 0 | 0 | 0 | 0 | 0 | 0 | 1 | 0 | 0 | 0 | 1 | 0 | 0 | 0 | 0 | 0 | 0 | 0 | 1 | 0 | 0 | Z17227 | 100 | 152 | 1 | 1724 | 1875 |
| 1250 | 01396 | 01249 | 4 | | 0 | 1 | 0 | 0 | 0 | 0 | 0 | 0 | 0 | 0 | 1 | 0 | 0 | 0 | 0 | 0 | 0 | 0 | 0 | 0 | 0 | 0 | 0 | 2 | 0 | | | | | | |
| 1251 | 01397 | 01250 | 1 | | 0 | 1 | 0 | 0 | 0 | 0 | 0 | 0 | 0 | 0 | 0 | 0 | 0 | 0 | 0 | 0 | 0 | 0 | 0 | 0 | 0 | 0 | 0 | 0 | 0 | | | | | | |
| 1252 | 01398 | 01251 | 2 | | 0 | 2 | 0 | 0 | 0 | 0 | 0 | 0 | 0 | 0 | 0 | 0 | 0 | 0 | 0 | 0 | 0 | 0 | 0 | 0 | 0 | 0 | 0 | 0 | 0 | | | | | | |
| 1253 | 01400 | 01252 | 1 | | 0 | 1 | 0 | 0 | 0 | 0 | 0 | 0 | 0 | 0 | 0 | 0 | 0 | 0 | 0 | 0 | 0 | 0 | 0 | 0 | 0 | 0 | 0 | 0 | 0 | | | | | | |
| 1254 | 01401 | 01253 | 2 | | 0 | 1 | 0 | 0 | 0 | 0 | 0 | 0 | 0 | 0 | 0 | 0 | 0 | 0 | 0 | 1 | 0 | 0 | 0 | 0 | 0 | 0 | 0 | 0 | 0 | | | | | | |
| 1255 | 01402 | 01254 | 7 | | 0 | 2 | 0 | 0 | 0 | 0 | 0 | 0 | 0 | 0 | 0 | 0 | 4 | 0 | 0 | 1 | 0 | 0 | 0 | 0 | 0 | 0 | 0 | 0 | 0 | | | | | | |
| 1256 | 01403 | 01255 | 6 | | 0 | 2 | 0 | 0 | 0 | 0 | 0 | 0 | 0 | 0 | 1 | 1 | 1 | 0 | 0 | 0 | 0 | 0 | 1 | 0 | 0 | 0 | 0 | 0 | 0 | | | | | | |
| 1257 | 01404 | 01256 | 16 | | 0 | 2 | 0 | 0 | 0 | 1 | 0 | 0 | 0 | 0 | 3 | 0 | 0 | 1 | 2 | 2 | 3 | 0 | 0 | 0 | 0 | 1 | 0 | 1 | 0 | M93651 | 96.2 | 159 | 1 | 1551 | 2577 |
| 1258 | 01405 | 01257 | 4 | | 0 | 1 | 0 | 0 | 0 | 0 | 0 | 0 | 2 | 0 | 0 | 0 | 0 | 0 | 0 | 0 | 0 | 0 | 0 | 0 | 0 | 1 | 0 | 0 | 0 | | | | | | |
| 1259 | 01406 | 01258 | 7 | | 0 | 2 | 1 | 0 | 0 | 0 | 0 | 0 | 0 | 0 | 0 | 0 | 0 | 1 | 0 | 0 | 0 | 0 | 0 | 0 | 0 | 0 | 3 | 0 | 0 | | | | | | |
| 1260 | 01407 | 01259 | 3 | | 0 | 1 | 0 | 0 | 0 | 0 | 0 | 0 | 0 | 0 | 0 | 0 | 0 | 0 | 0 | 0 | 0 | 0 | 0 | 0 | 1 | 0 | 1 | 0 | 0 | | | | | | |
| 1261 | 01408 | 01260 | 3 | | 0 | 1 | 0 | 0 | 0 | 0 | 0 | 0 | 1 | 0 | 0 | 0 | 0 | 0 | 0 | 0 | 0 | 0 | 0 | 0 | 0 | 1 | 0 | 0 | 0 | | | | | | |
| 1262 | 01409 | 01261 | 5 | | 0 | 1 | 0 | 0 | 0 | 0 | 0 | 0 | 0 | 0 | 0 | 0 | 0 | 2 | 0 | 0 | 0 | 0 | 1 | 0 | 0 | 1 | 0 | 0 | 0 | | | | | | |
| 1263 | 01410 | 01262 | 2 | | 0 | 2 | 0 | 0 | 0 | 0 | 0 | 0 | 0 | 0 | 0 | 0 | 0 | 0 | 0 | 0 | 0 | 0 | 0 | 0 | 0 | 0 | 0 | 0 | 0 | | | | | | |
| 1264 | 01411 | 01263 | 2 | | 0 | 1 | 0 | 0 | 0 | 0 | 0 | 0 | 0 | 0 | 0 | 0 | 0 | 0 | 0 | 1 | 0 | 0 | 0 | 0 | 0 | 0 | 0 | 0 | 0 | | | | | | |
| 1265 | 01412 | 01264 | 1 | | 0 | 1 | 0 | 0 | 0 | 0 | 0 | 0 | 0 | 0 | 0 | 0 | 0 | 0 | 0 | 0 | 0 | 0 | 0 | 0 | 0 | 0 | 0 | 0 | 0 | | | | | | |
| 1266 | 01413 | 01265 | 1 | | 0 | 1 | 0 | 0 | 0 | 0 | 0 | 0 | 0 | 0 | 0 | 0 | 0 | 0 | 0 | 0 | 0 | 0 | 0 | 0 | 0 | 0 | 0 | 0 | 0 | X65463 | 99.2 | 119 | 1 | 1634 | 1752 |
| 1267 | 01414 | 01266 | 2 | | 0 | 1 | 0 | 0 | 0 | 0 | 0 | 0 | 0 | 0 | 0 | 0 | 0 | 0 | 0 | 0 | 0 | 0 | 1 | 0 | 0 | 0 | 0 | 0 | 0 | | | | | | |
| 1268 | 01415 | 01267 | 1 | | 0 | 1 | 0 | 0 | 0 | 0 | 0 | 0 | 0 | 0 | 0 | 0 | 0 | 0 | 0 | 0 | 0 | 0 | 0 | 0 | 0 | 0 | 0 | 0 | 0 | | | | | | |
| 1269 | 01416 | 01268 | 1 | | 0 | 1 | 0 | 0 | 0 | 0 | 0 | 0 | 0 | 0 | 0 | 0 | 0 | 0 | 0 | 0 | 0 | 0 | 0 | 0 | 0 | 0 | 0 | 0 | 0 | | | | | | |
| 1270 | 01417 | 01269 | 1 | | 0 | 1 | 0 | 0 | 0 | 0 | 0 | 0 | 0 | 0 | 0 | 0 | 0 | 0 | 0 | 0 | 0 | 0 | 0 | 0 | 0 | 0 | 0 | 0 | 0 | | | | | | |
| 1271 | 01418 | 01270 | 3 | | 0 | 1 | 0 | 0 | 0 | 0 | 0 | 0 | 0 | 0 | 0 | 0 | 0 | 1 | 0 | 0 | 0 | 0 | 0 | 0 | 0 | 0 | 1 | 0 | 0 | | | | | | |
| 1272 | 01419 | 01271 | 2 | | 0 | 1 | 0 | 0 | 0 | 0 | 0 | 0 | 0 | 0 | 0 | 0 | 0 | 0 | 1 | 0 | 0 | 0 | 0 | 0 | 0 | 0 | 0 | 0 | 0 | | | | | | |
| 1273 | 01420 | 01272 | 4 | | 0 | 1 | 0 | 0 | 0 | 0 | 0 | 0 | 0 | 0 | 0 | 0 | 0 | 0 | 0 | 0 | 0 | 0 | 0 | 1 | 0 | 1 | 1 | 0 | 0 | | | | | | |
| 1274 | 01421 | 01273 | 4 | | 0 | 2 | 0 | 0 | 0 | 0 | 1 | 0 | 0 | 0 | 0 | 0 | 0 | 0 | 0 | 0 | 0 | 0 | 0 | 0 | 1 | 0 | 0 | 0 | 0 | | | | | | |
| 1275 | 01422 | 01274 | 3 | | 0 | 1 | 1 | 0 | 0 | 0 | 0 | 0 | 0 | 0 | 0 | 0 | 0 | 0 | 0 | 0 | 0 | 0 | 0 | 1 | 0 | 0 | 0 | 0 | 0 | | | | | | |
| 1276 | 01423 | 01275 | 2 | | 0 | 1 | 0 | 0 | 0 | 0 | 0 | 0 | 0 | 0 | 0 | 0 | 0 | 0 | 0 | 1 | 0 | 0 | 0 | 0 | 0 | 0 | 0 | 0 | 0 | | | | | | |
| 1277 | 01424 | 01276 | 12 | | 0 | 2 | 5 | 0 | 0 | 0 | 1 | 0 | 0 | 0 | 1 | 1 | 0 | 0 | 0 | 0 | 0 | 0 | 0 | 1 | 0 | 0 | 1 | 0 | 0 | | | | | | |
| 1278 | 01426 | 01277 | 1 | | 0 | 1 | 0 | 0 | 0 | 0 | 0 | 0 | 0 | 0 | 0 | 0 | 0 | 0 | 0 | 0 | 0 | 0 | 0 | 0 | 0 | 0 | 0 | 0 | 0 | | | | | | |
| 1279 | 01427 | 01278 | 1 | | 0 | 1 | 0 | 0 | 0 | 0 | 0 | 0 | 0 | 0 | 0 | 0 | 0 | 0 | 0 | 0 | 0 | 0 | 0 | 0 | 0 | 0 | 0 | 0 | 0 | | | | | | |
| 1280 | 01428 | 01279 | 4 | | 0 | 2 | 0 | 0 | 0 | 0 | 0 | 0 | 0 | 0 | 1 | 0 | 0 | 0 | 0 | 0 | 0 | 0 | 1 | 0 | 0 | 0 | 0 | 0 | 0 | | | | | | |
| 1281 | 01429 | 01280 | 4 | | 0 | 3 | 0 | 0 | 0 | 0 | 0 | 0 | 0 | 0 | 0 | 0 | 0 | 0 | 0 | 0 | 0 | 0 | 0 | 0 | 0 | 0 | 0 | 1 | 0 | | | | | | |

Table 36

| | A | B | C | E | G | I | K | M | O | Q | S | U | W | Y | AA | AC | AE | AG | AI | AK | AM | AO | AQ | AS | AU | AW | AY | BA | BC | BE | BF | BG | BH | BI | BK |
|---|---|---|---|---|---|---|---|---|---|---|---|---|---|---|---|---|---|---|---|---|---|---|---|---|---|---|---|---|---|---|---|---|---|---|---|
| 1282 | 01430 | 01281 | 1 | | 0 | 1 | 0 | 0 | 0 | 0 | 0 | 0 | 0 | 0 | 0 | 0 | 0 | 0 | 0 | 0 | 0 | 0 | 0 | 0 | 0 | 0 | 0 | 0 | 0 | | | | | | |
| 1283 | 01431 | 01282 | 6 | | 0 | 1 | 0 | 0 | 0 | 0 | 0 | 0 | 0 | 0 | 0 | 0 | 0 | 0 | 0 | 0 | 0 | 0 | 0 | 0 | 3 | 0 | 2 | 0 | 0 | X51346 | 99.3 | 144 | 1 | 1470 | 1612 |
| 1284 | 01432 | 01283 | 1 | | 0 | 1 | 0 | 0 | 0 | 0 | 0 | 0 | 0 | 0 | 0 | 0 | 0 | 0 | 0 | 0 | 0 | 0 | 0 | 0 | 0 | 0 | 0 | 0 | 0 | | | | | | |
| 1285 | 01433 | 01284 | 1 | | 0 | 1 | 0 | 0 | 0 | 0 | 0 | 0 | 0 | 0 | 0 | 0 | 0 | 0 | 0 | 0 | 0 | 0 | 0 | 0 | 0 | 0 | 0 | 0 | 0 | | | | | | |
| 1286 | 01434 | 01285 | 2 | | 0 | 1 | 0 | 0 | 0 | 0 | 0 | 0 | 0 | 0 | 0 | 0 | 0 | 0 | 0 | 0 | 0 | 0 | 0 | 0 | 0 | 0 | 1 | 0 | 0 | | | | | | |
| 1287 | 01435 | 01286 | 7 | | 0 | 1 | 0 | 0 | 0 | 0 | 0 | 0 | 0 | 1 | 2 | 0 | 0 | 0 | 1 | 0 | 1 | 0 | 0 | 0 | 0 | 1 | 0 | 0 | 0 | | | | | | |
| 1288 | 01436 | 01287 | 1 | | 0 | 1 | 0 | 0 | 0 | 0 | 0 | 0 | 0 | 0 | 0 | 0 | 0 | 0 | 0 | 0 | 0 | 0 | 0 | 0 | 0 | 0 | 0 | 0 | 0 | | | | | | |
| 1289 | 01437 | 01288 | 2 | | 0 | 1 | 0 | 0 | 0 | 0 | 0 | 0 | 0 | 0 | 0 | 0 | 1 | 0 | 0 | 0 | 0 | 0 | 0 | 0 | 0 | 0 | 0 | 0 | 0 | | | | | | |
| 1290 | 01438 | 01289 | 3 | | 0 | 1 | 1 | 0 | 0 | 0 | 0 | 0 | 0 | 0 | 0 | 0 | 0 | 0 | 0 | 1 | 0 | 0 | 0 | 0 | 0 | 0 | 0 | 0 | 0 | | | | | | |
| 1291 | 01439 | 01290 | 1 | | 0 | 1 | 0 | 0 | 0 | 0 | 0 | 0 | 0 | 0 | 0 | 0 | 0 | 0 | 0 | 0 | 0 | 0 | 0 | 0 | 0 | 0 | 0 | 0 | 0 | M11948 | 90.5 | 84 | 59 | 342 | 425 |
| 1292 | 01440 | 01291 | 5 | | 0 | 1 | 0 | 0 | 0 | 0 | 0 | 0 | 0 | 0 | 0 | 0 | 0 | 0 | 1 | 0 | 0 | 1 | 0 | 0 | 1 | 0 | 1 | 0 | 0 | | | | | | |
| 1293 | 01441 | 01292 | 1 | | 0 | 1 | 0 | 0 | 0 | 0 | 0 | 0 | 0 | 0 | 0 | 0 | 0 | 0 | 0 | 0 | 0 | 0 | 0 | 0 | 0 | 0 | 0 | 0 | 0 | X55188 | 98.9 | 92 | 1 | 10 | 1560 |
| 1294 | 01442 | 01293 | 4 | | 0 | 1 | 1 | 0 | 0 | 0 | 0 | 0 | 0 | 0 | 0 | 0 | 0 | 0 | 0 | 0 | 0 | 0 | 0 | 0 | 0 | 0 | 1 | 1 | 0 | | | | | | |
| 1295 | 01443 | 01294 | 3 | | 0 | 1 | 0 | 0 | 0 | 0 | 0 | 0 | 0 | 0 | 0 | 0 | 0 | 0 | 1 | 0 | 0 | 0 | 0 | 1 | 0 | 0 | 0 | 0 | 0 | | | | | | |
| 1296 | 01444 | 01295 | 3 | | 0 | 1 | 1 | 0 | 0 | 0 | 0 | 0 | 0 | 0 | 0 | 0 | 0 | 0 | 1 | 0 | 0 | 0 | 0 | 0 | 0 | 0 | 0 | 0 | 0 | | | | | | |
| 1297 | 01445 | 01296 | 2 | | 0 | 2 | 0 | 0 | 0 | 0 | 0 | 0 | 0 | 0 | 0 | 0 | 0 | 0 | 0 | 0 | 0 | 0 | 0 | 0 | 0 | 0 | 0 | 0 | 0 | | | | | | |
| 1298 | 01446 | 01297 | 1 | | 0 | 1 | 0 | 0 | 0 | 0 | 0 | 0 | 0 | 0 | 0 | 0 | 0 | 0 | 0 | 0 | 0 | 0 | 0 | 0 | 0 | 0 | 0 | 0 | 0 | | | | | | |
| 1299 | 01447 | 01298 | 2 | | 0 | 2 | 0 | 0 | 0 | 0 | 0 | 0 | 0 | 0 | 0 | 0 | 0 | 0 | 0 | 0 | 0 | 0 | 0 | 0 | 0 | 0 | 0 | 0 | 0 | | | | | | |
| 1300 | 01448 | 01299 | 2 | | 0 | 1 | 0 | 0 | 0 | 0 | 0 | 0 | 0 | 0 | 0 | 0 | 0 | 0 | 0 | 0 | 0 | 0 | 0 | 1 | 0 | 0 | 0 | 0 | 0 | | | | | | |
| 1301 | 01449 | 01300 | 10 | | 0 | 2 | 1 | 1 | 0 | 0 | 0 | 0 | 0 | 0 | 0 | 1 | 0 | 0 | 0 | 0 | 0 | 0 | 2 | 0 | 1 | 0 | 0 | 2 | 0 | | | | | | |
| 1302 | 01450 | 01301 | 4 | | 0 | 2 | 0 | 0 | 0 | 0 | 0 | 0 | 0 | 0 | 0 | 0 | 0 | 0 | 0 | 0 | 0 | 0 | 0 | 0 | 0 | 0 | 0 | 1 | 1 | | | | | | |
| 1303 | 01451 | 01302 | 1 | | 0 | 1 | 0 | 0 | 0 | 0 | 0 | 0 | 0 | 0 | 0 | 0 | 0 | 0 | 0 | 0 | 0 | 0 | 0 | 0 | 0 | 0 | 0 | 0 | 0 | | | | | | |
| 1304 | 01452 | 01303 | 4 | | 0 | 1 | 0 | 0 | 0 | 0 | 0 | 0 | 0 | 0 | 0 | 1 | 0 | 0 | 0 | 0 | 0 | 0 | 2 | 0 | 0 | 0 | 0 | 0 | 0 | | | | | | |
| 1305 | 01453 | 01304 | 3 | | 0 | 1 | 0 | 0 | 1 | 0 | 0 | 0 | 0 | 0 | 0 | 0 | 0 | 0 | 1 | 0 | 0 | 0 | 0 | 0 | 0 | 0 | 0 | 0 | 0 | | | | | | |
| 1306 | 01454 | 01305 | 2 | | 0 | 1 | 0 | 0 | 0 | 0 | 0 | 0 | 0 | 0 | 0 | 0 | 0 | 0 | 0 | 0 | 0 | 0 | 0 | 1 | 0 | 0 | 0 | 0 | 0 | | | | | | |
| 1307 | 01455 | 01306 | 1 | | 0 | 1 | 0 | 0 | 0 | 0 | 0 | 0 | 0 | 0 | 0 | 0 | 0 | 0 | 0 | 0 | 0 | 0 | 0 | 0 | 0 | 0 | 0 | 0 | 0 | | | | | | |
| 1308 | 01456 | 01307 | 1 | | 0 | 1 | 0 | 0 | 0 | 0 | 0 | 0 | 0 | 0 | 0 | 0 | 0 | 0 | 0 | 0 | 0 | 0 | 0 | 0 | 0 | 0 | 0 | 0 | 0 | | | | | | |
| 1309 | 01457 | 01308 | 1 | | 0 | 1 | 0 | 0 | 0 | 0 | 0 | 0 | 0 | 0 | 0 | 0 | 0 | 0 | 0 | 0 | 0 | 0 | 0 | 0 | 0 | 0 | 0 | 0 | 0 | | | | | | |
| 1310 | 01458 | 01309 | 2 | | 0 | 2 | 0 | 0 | 0 | 0 | 0 | 0 | 0 | 0 | 0 | 0 | 0 | 0 | 0 | 0 | 0 | 0 | 0 | 0 | 0 | 0 | 0 | 0 | 0 | | | | | | |
| 1311 | 01459 | 01310 | 1 | | 0 | 1 | 0 | 0 | 0 | 0 | 0 | 0 | 0 | 0 | 0 | 0 | 0 | 0 | 0 | 0 | 0 | 0 | 0 | 0 | 0 | 0 | 0 | 0 | 0 | | | | | | |
| 1312 | 01460 | 01311 | 2 | | 0 | 1 | 1 | 0 | 0 | 0 | 0 | 0 | 0 | 0 | 0 | 0 | 0 | 0 | 0 | 0 | 0 | 0 | 0 | 0 | 0 | 0 | 0 | 0 | 0 | | | | | | |
| 1313 | 01461 | 01312 | 1 | | 0 | 1 | 0 | 0 | 0 | 0 | 0 | 0 | 0 | 0 | 0 | 0 | 0 | 0 | 0 | 0 | 0 | 0 | 0 | 0 | 0 | 0 | 0 | 0 | 0 | | | | | | |
| 1314 | 01462 | 01313 | 6 | | 0 | 1 | 1 | 0 | 0 | 0 | 0 | 1 | 0 | 0 | 0 | 0 | 0 | 0 | 1 | 0 | 0 | 1 | 0 | 0 | 0 | 0 | 0 | 1 | 0 | | | | | | |
| 1315 | 01463 | 01314 | 1 | | 0 | 1 | 0 | 0 | 0 | 0 | 0 | 0 | 0 | 0 | 0 | 0 | 0 | 0 | 0 | 0 | 0 | 0 | 0 | 0 | 0 | 0 | 0 | 0 | 0 | | | | | | |
| 1316 | 01464 | 01315 | 1 | | 0 | 1 | 0 | 0 | 0 | 0 | 0 | 0 | 0 | 0 | 0 | 0 | 0 | 0 | 0 | 0 | 0 | 0 | 0 | 0 | 0 | 0 | 0 | 0 | 0 | | | | | | |
| 1317 | 01466 | 01316 | 1 | | 0 | 1 | 0 | 0 | 0 | 0 | 0 | 0 | 0 | 0 | 0 | 0 | 0 | 0 | 0 | 0 | 0 | 0 | 0 | 0 | 0 | 0 | 0 | 0 | 0 | | | | | | |

Table 37

| | A | B | C | E | G | I | K | M | O | Q | S | U | W | Y | AA | AC | AE | AG | AI | AK | AM | AO | AQ | AS | AU | AW | AY | BA | BC | BE | BF | BG | BH | BI | BK |
|---|---|---|---|---|---|---|---|---|---|---|---|---|---|---|---|---|---|---|---|---|---|---|---|---|---|---|---|---|---|---|---|---|---|---|---|
| 1318 | 01467 | 01317 | 4 | | 0 | 2 | 0 | 0 | 0 | 0 | 1 | 0 | 0 | 0 | 0 | 0 | 0 | 0 | 0 | 1 | 0 | 0 | 0 | 0 | 0 | 0 | 0 | 0 | 0 | | | | | | |
| 1319 | 01468 | 01318 | 2 | | 0 | 2 | 0 | 0 | 0 | 0 | 0 | 0 | 0 | 0 | 0 | 0 | 0 | 0 | 0 | 0 | 0 | 0 | 0 | 0 | 0 | 0 | 0 | 0 | 0 | | | | | | |
| 1320 | 01469 | 01319 | 4 | | 0 | 1 | 0 | 0 | 0 | 0 | 0 | 0 | 0 | 0 | 0 | 0 | 0 | 0 | 0 | 0 | 0 | 1 | 0 | 0 | 0 | 1 | 0 | 1 | 0 | | | | | | |
| 1321 | 01470 | 01320 | 3 | | 0 | 1 | 0 | 0 | 0 | 0 | 0 | 0 | 0 | 0 | 2 | 0 | 0 | 0 | 0 | 0 | 0 | 0 | 0 | 0 | 0 | 0 | 0 | 0 | 0 | | | | | | |
| 1322 | 01471 | 01321 | 2 | | 0 | 1 | 0 | 0 | 0 | 1 | 0 | 0 | 0 | 0 | 0 | 0 | 0 | 0 | 0 | 0 | 0 | 0 | 0 | 0 | 0 | 0 | 0 | 0 | 0 | | | | | | |
| 1323 | 01472 | 01322 | 1 | | 0 | 1 | 0 | 0 | 0 | 0 | 0 | 0 | 0 | 0 | 0 | 0 | 0 | 0 | 0 | 0 | 0 | 0 | 0 | 0 | 0 | 0 | 0 | 0 | 0 | | | | | | |
| 1324 | 01473 | 01323 | 9 | | 0 | 2 | 2 | 0 | 0 | 0 | 1 | 0 | 1 | 0 | 0 | 0 | 0 | 0 | 0 | 0 | 0 | 0 | 0 | 0 | 0 | 0 | 3 | 0 | 0 | | | | | | |
| 1325 | 01474 | 01324 | 2 | | 0 | 1 | 1 | 0 | 0 | 0 | 0 | 0 | 0 | 0 | 0 | 0 | 0 | 0 | 0 | 0 | 0 | 0 | 0 | 0 | 0 | 0 | 0 | 0 | 0 | | | | | | |
| 1326 | 01475 | 01325 | 3 | | 0 | 1 | 0 | 0 | 0 | 0 | 0 | 0 | 0 | 0 | 0 | 0 | 0 | 0 | 0 | 0 | 0 | 0 | 1 | 0 | 0 | 1 | 0 | 0 | 0 | | | | | | |
| 1327 | 01476 | 01326 | 19 | | 0 | 4 | 2 | 0 | 0 | 0 | 0 | 1 | 0 | 0 | 0 | 0 | 0 | 0 | 0 | 0 | 0 | 1 | 0 | 0 | 1 | 0 | 10 | 0 | 0 | | | | | | |
| 1328 | 01477 | 01327 | 4 | | 0 | 1 | 0 | 0 | 0 | 0 | 0 | 0 | 1 | 0 | 0 | 1 | 0 | 0 | 0 | 0 | 0 | 1 | 0 | 0 | 0 | 0 | 0 | 0 | 0 | | | | | | |
| 1329 | 01478 | 01328 | 5 | | 0 | 3 | 0 | 0 | 0 | 0 | 0 | 0 | 0 | 0 | 0 | 0 | 0 | 0 | 0 | 0 | 0 | 0 | 0 | 0 | 0 | 2 | 0 | 0 | 0 | | | | | | |
| 1330 | 01479 | 01329 | 1 | | 0 | 1 | 0 | 0 | 0 | 0 | 0 | 0 | 0 | 0 | 0 | 0 | 0 | 0 | 0 | 0 | 0 | 0 | 0 | 0 | 0 | 0 | 0 | 0 | 0 | | | | | | |
| 1331 | 01481 | 01330 | 3 | | 0 | 2 | 1 | 0 | 0 | 0 | 0 | 0 | 0 | 0 | 0 | 0 | 0 | 0 | 0 | 0 | 0 | 0 | 0 | 0 | 0 | 0 | 0 | 0 | 0 | | | | | | |
| 1332 | 01482 | 01331 | 2 | | 0 | 1 | 1 | 0 | 0 | 0 | 0 | 0 | 0 | 0 | 0 | 0 | 0 | 0 | 0 | 0 | 0 | 0 | 0 | 0 | 0 | 0 | 0 | 0 | 0 | | | | | | |
| 1333 | 01483 | 01332 | 9 | | 0 | 2 | 2 | 0 | 0 | 0 | 0 | 1 | 0 | 0 | 0 | 1 | 0 | 1 | 1 | 0 | 0 | 0 | 0 | 1 | 0 | 0 | 0 | 0 | 0 | | | | | | |
| 1334 | 01484 | 01333 | 1 | | 0 | 1 | 0 | 0 | 0 | 0 | 0 | 0 | 0 | 0 | 0 | 0 | 0 | 0 | 0 | 0 | 0 | 0 | 0 | 0 | 0 | 0 | 0 | 0 | 0 | | | | | | |
| 1335 | 01485 | 01334 | 1 | | 0 | 1 | 0 | 0 | 0 | 0 | 0 | 0 | 0 | 0 | 0 | 0 | 0 | 0 | 0 | 0 | 0 | 0 | 0 | 0 | 0 | 0 | 0 | 0 | 0 | | | | | | |
| 1336 | 01486 | 01335 | 3 | | 0 | 2 | 0 | 0 | 0 | 1 | 0 | 0 | 0 | 0 | 0 | 0 | 0 | 0 | 0 | 0 | 0 | 0 | 0 | 0 | 0 | 0 | 0 | 0 | 0 | | | | | | |
| 1337 | 01487 | 01336 | 1 | | 0 | 1 | 0 | 0 | 0 | 0 | 0 | 0 | 0 | 0 | 0 | 0 | 0 | 0 | 0 | 0 | 0 | 0 | 0 | 0 | 0 | 0 | 0 | 0 | 0 | | | | | | |
| 1338 | 01488 | 01337 | 4 | | 0 | 1 | 0 | 0 | 0 | 0 | 0 | 0 | 1 | 0 | 0 | 0 | 0 | 0 | 2 | 0 | 0 | 0 | 0 | 0 | 0 | 0 | 0 | 0 | 0 | | | | | | |
| 1339 | 01489 | 01338 | 5 | | 0 | 1 | 0 | 0 | 0 | 1 | 0 | 0 | 0 | 0 | 0 | 0 | 0 | 0 | 0 | 1 | 0 | 0 | 0 | 0 | 0 | 0 | 0 | 2 | 0 | | | | | | |
| 1340 | 01490 | 01339 | 2 | | 0 | 1 | 0 | 0 | 0 | 0 | 0 | 0 | 0 | 0 | 0 | 0 | 0 | 0 | 1 | 0 | 0 | 0 | 0 | 0 | 0 | 0 | 0 | 0 | 0 | | | | | | |
| 1341 | 01491 | 01340 | 3 | | 0 | 1 | 0 | 0 | 0 | 0 | 0 | 0 | 0 | 0 | 0 | 0 | 0 | 0 | 0 | 0 | 0 | 0 | 0 | 0 | 0 | 0 | 2 | 0 | 0 | | | | | | |
| 1342 | 01492 | 01341 | 1 | | 0 | 1 | 0 | 0 | 0 | 0 | 0 | 0 | 0 | 0 | 0 | 0 | 0 | 0 | 0 | 0 | 0 | 0 | 0 | 0 | 0 | 0 | 0 | 0 | 0 | | | | | | |
| 1343 | 01493 | 01342 | 1 | | 0 | 1 | 0 | 0 | 0 | 0 | 0 | 0 | 0 | 0 | 0 | 0 | 0 | 0 | 0 | 0 | 0 | 0 | 0 | 0 | 0 | 0 | 0 | 0 | 0 | | | | | | |
| 1344 | 01494 | 01343 | 1 | | 0 | 1 | 0 | 0 | 0 | 0 | 0 | 0 | 0 | 0 | 0 | 0 | 0 | 0 | 0 | 0 | 0 | 0 | 0 | 0 | 0 | 0 | 0 | 0 | 0 | | | | | | |
| 1345 | 01495 | 01344 | 1 | | 0 | 1 | 0 | 0 | 0 | 0 | 0 | 0 | 0 | 0 | 0 | 0 | 0 | 0 | 0 | 0 | 0 | 0 | 0 | 0 | 0 | 0 | 0 | 0 | 0 | | | | | | |
| 1346 | 01496 | 01345 | 3 | | 0 | 1 | 0 | 0 | 0 | 0 | 0 | 0 | 0 | 0 | 0 | 0 | 0 | 0 | 1 | 0 | 0 | 0 | 0 | 0 | 1 | 0 | 0 | 0 | 0 | X54637 | 100 | 116 | 1 | 4058 | 4176 |
| 1347 | 01497 | 01346 | 1 | | 0 | 1 | 0 | 0 | 0 | 0 | 0 | 0 | 0 | 0 | 0 | 0 | 0 | 0 | 0 | 0 | 0 | 0 | 0 | 0 | 0 | 0 | 0 | 0 | 0 | | | | | | |
| 1348 | 01498 | 01347 | 1 | | 0 | 1 | 0 | 0 | 0 | 0 | 0 | 0 | 0 | 0 | 0 | 0 | 0 | 0 | 0 | 0 | 0 | 0 | 0 | 0 | 0 | 0 | 0 | 0 | 0 | | | | | | |
| 1349 | 01499 | 01348 | 2 | | 0 | 1 | 0 | 0 | 0 | 0 | 0 | 0 | 0 | 0 | 0 | 0 | 0 | 0 | 0 | 0 | 0 | 0 | 0 | 0 | 0 | 0 | 1 | 0 | 0 | | | | | | |
| 1350 | 01500 | 01349 | 3 | | 0 | 1 | 0 | 0 | 0 | 0 | 0 | 1 | 0 | 0 | 0 | 0 | 0 | 0 | 0 | 0 | 0 | 0 | 0 | 0 | 0 | 0 | 1 | 0 | 0 | | | | | | |
| 1351 | 01501 | 01350 | 3 | | 0 | 1 | 0 | 0 | 0 | 0 | 0 | 0 | 0 | 0 | 0 | 0 | 0 | 0 | 0 | 0 | 0 | 2 | 0 | 0 | 0 | 0 | 0 | 0 | 0 | | | | | | |
| 1352 | 01502 | 01351 | 1 | | 0 | 1 | 0 | 0 | 0 | 0 | 0 | 0 | 0 | 0 | 0 | 0 | 0 | 0 | 0 | 0 | 0 | 0 | 0 | 0 | 0 | 0 | 0 | 0 | 0 | | | | | | |
| 1353 | 01503 | 01352 | 9 | | 0 | 1 | 2 | 0 | 0 | 0 | 1 | 0 | 0 | 2 | 0 | 0 | 0 | 1 | 0 | 1 | 0 | .0 | 0 | 0 | 1 | 0 | 0 | 0 | | | | | | | |

Table 38

| | A | B | C | E | G | I | K | M | O | Q | S | U | W | Y | AA | AC | AE | AG | AI | AK | AM | AO | AQ | AS | AU | AW | AY | BA | BC | BE | BF | BG | BH | BI | BK |
|---|---|---|---|---|---|---|---|---|---|---|---|---|---|---|---|---|---|---|---|---|---|---|---|---|---|---|---|---|---|---|---|---|---|---|---|
| 1354 | 01505 | 01353 | 1 | | 0 | 1 | 0 | 0 | 0 | 0 | 0 | 0 | 0 | 0 | 0 | 0 | 0 | 0 | 0 | 0 | 0 | 0 | 0 | 0 | 0 | 0 | 0 | 0 | 0 | | | | | | |
| 1355 | 01506 | 01354 | 1 | | 0 | 1 | 0 | 0 | 0 | 0 | 0 | 0 | 0 | 0 | 0 | 0 | 0 | 0 | 0 | 0 | 0 | 0 | 0 | 0 | 0 | 0 | 0 | 0 | 0 | | | | | | |
| 1356 | 01507 | 01355 | 5 | | 0 | 1 | 0 | 0 | 0 | 0 | 0 | 0 | 2 | 1 | 0 | 0 | 0 | 0 | 0 | 0 | 0 | 0 | 0 | 0 | 0 | 0 | 1 | 0 | 0 | X55187 | 98.3 | 406 | 1 | 1923 | 2327 |
| 1357 | 01508 | 01356 | 13 | | 0 | 3 | 0 | 0 | 0 | 0 | 0 | 0 | 0 | 1 | 0 | 1 | 0 | 0 | 0 | 0 | 0 | 6 | 1 | 0 | 0 | 1 | 0 | 0 | 0 | M29064 | 99.1 | 112 | 1 | 1617 | 1760 |
| 1358 | 01509 | 01357 | 1 | | 0 | 1 | 0 | 0 | 0 | 0 | 0 | 0 | 0 | 0 | 0 | 0 | 0 | 0 | 0 | 0 | 0 | 0 | 0 | 0 | 0 | 0 | 0 | 0 | 0 | | | | | | |
| 1359 | 01510 | 01358 | 1 | | 0 | 1 | 0 | 0 | 0 | 0 | 0 | 0 | 0 | 0 | 0 | 0 | 0 | 0 | 0 | 0 | 0 | 0 | 0 | 0 | 0 | 0 | 0 | 0 | 0 | | | | | | |
| 1360 | 01511 | 01359 | 1 | | 0 | 1 | 0 | 0 | 0 | 0 | 0 | 0 | 0 | 0 | 0 | 0 | 0 | 0 | 0 | 0 | 0 | 0 | 0 | 0 | 0 | 0 | 0 | 0 | 0 | | | | | | |
| 1361 | 01512 | 01360 | 1 | | 0 | 1 | 0 | 0 | 0 | 0 | 0 | 0 | 0 | 0 | 0 | 0 | 0 | 0 | 0 | 0 | 0 | 0 | 0 | 0 | 0 | 0 | 0 | 0 | 0 | | | | | | |
| 1362 | 01513 | 01361 | 1 | | 0 | 1 | 0 | 0 | 0 | 0 | 0 | 0 | 0 | 0 | 0 | 0 | 0 | 0 | 0 | 0 | 0 | 0 | 0 | 0 | 0 | 0 | 0 | 0 | 0 | | | | | | |
| 1363 | 01514 | 01362 | 1 | | 0 | 1 | 0 | 0 | 0 | 0 | 0 | 0 | 0 | 0 | 0 | 0 | 0 | 0 | 0 | 0 | 0 | 0 | 0 | 0 | 0 | 0 | 0 | 0 | 0 | | | | | | |
| 1364 | 01515 | 01363 | 1 | | 0 | 1 | 0 | 0 | 0 | 0 | 0 | 0 | 0 | 0 | 0 | 0 | 0 | 0 | 0 | 0 | 0 | 0 | 0 | 0 | 0 | 0 | 0 | 0 | 0 | M73554 | 93.6 | 110 | 1 | 3448 | 4221 |
| 1365 | 01516 | 01364 | 7 | | 0 | 1 | 0 | 0 | 0 | 0 | 0 | 0 | 0 | 0 | 0 | 0 | 0 | 0 | 0 | 0 | 0 | 0 | 2 | 4 | 0 | 0 | 0 | 0 | 0 | | | | | | |
| 1366 | 01517 | 01365 | 1 | | 0 | 1 | 0 | 0 | 0 | 0 | 0 | 0 | 0 | 0 | 0 | 0 | 0 | 0 | 0 | 0 | 0 | 0 | 0 | 0 | 0 | 0 | 0 | 0 | 0 | | | | | | |
| 1367 | 01518 | 01366 | 1 | | 0 | 1 | 0 | 0 | 0 | 0 | 0 | 0 | 0 | 0 | 0 | 0 | 0 | 0 | 0 | 0 | 0 | 0 | 0 | 0 | 0 | 0 | 0 | 0 | 0 | | | | | | |
| 1368 | 01519 | 01367 | 3 | | 0 | 1 | 0 | 0 | 0 | 0 | 1 | 0 | 0 | 0 | 0 | 0 | 0 | 0 | 0 | 1 | 0 | 0 | 0 | 0 | 0 | 0 | 0 | 0 | 0 | | | | | | |
| 1369 | 01520 | 01368 | 1 | | 0 | 1 | 0 | 0 | 0 | 0 | 0 | 0 | 0 | 0 | 0 | 0 | 0 | 0 | 0 | 0 | 0 | 0 | 0 | 0 | 0 | 0 | 0 | 0 | 0 | | | | | | |
| 1370 | 01521 | 01369 | 1 | | 0 | 1 | 0 | 0 | 0 | 0 | 0 | 0 | 0 | 0 | 0 | 0 | 0 | 0 | 0 | 0 | 0 | 0 | 0 | 0 | 0 | 0 | 0 | 0 | 0 | | | | | | |
| 1371 | 01522 | 01370 | 1 | | 0 | 1 | 0 | 0 | 0 | 0 | 0 | 0 | 0 | 0 | 0 | 0 | 0 | 0 | 0 | 0 | 0 | 0 | 0 | 0 | 0 | 0 | 0 | 0 | 0 | | | | | | |
| 1372 | 01523 | 01371 | 1 | | 0 | 1 | 0 | 0 | 0 | 0 | 0 | 0 | 0 | 0 | 0 | 0 | 0 | 0 | 0 | 0 | 0 | 0 | 0 | 0 | 0 | 0 | 0 | 0 | 0 | | | | | | |
| 1373 | 01524 | 01372 | 7 | | 0 | 1 | 0 | 1 | 0 | 0 | 0 | 0 | 0 | 0 | 0 | 0 | 0 | 1 | 1 | 0 | 0 | 0 | 1 | 0 | 0 | 0 | 1 | 1 | 0 | S54711 | 96.5 | 231 | 62 | 1 | 229 |
| 1374 | 01525 | 01373 | 1 | | 0 | 1 | 0 | 0 | 0 | 0 | 0 | 0 | 0 | 0 | 0 | 0 | 0 | 0 | 0 | 0 | 0 | 0 | 0 | 0 | 0 | 0 | 0 | 0 | 0 | | | | | | |
| 1375 | 01526 | 01374 | 1 | | 0 | 1 | 0 | 0 | 0 | 0 | 0 | 0 | 0 | 0 | 0 | 0 | 0 | 0 | 0 | 0 | 0 | 0 | 0 | 0 | 0 | 0 | 0 | 0 | 0 | | | | | | |
| 1376 | 01527 | 01375 | 1 | | 0 | 1 | 0 | 0 | 0 | 0 | 0 | 0 | 0 | 0 | 0 | 0 | 0 | 0 | 0 | 0 | 0 | 0 | 0 | 0 | 0 | 0 | 0 | 0 | 0 | | | | | | |
| 1377 | 01528 | 01376 | 16 | | 0 | 2 | 3 | 0 | 0 | 0 | 0 | 0 | 0 | 1 | 0 | 2 | 0 | 0 | 1 | 0 | 0 | 1 | 0 | 3 | 1 | 0 | 1 | 1 | 0 | | | | | | |
| 1378 | 01529 | 01377 | 7 | | 0 | 2 | 2 | 0 | 0 | 0 | 0 | 0 | 0 | 0 | 2 | 0 | 0 | 0 | 0 | 0 | 0 | 0 | 0 | 0 | 0 | 0 | 0 | 1 | 0 | | | | | | |
| 1379 | 01530 | 01378 | 1 | | 0 | 1 | 0 | 0 | 0 | 0 | 0 | 0 | 0 | 0 | 0 | 0 | 0 | 0 | 0 | 0 | 0 | 0 | 0 | 0 | 0 | 0 | 0 | 0 | 0 | | | | | | |
| 1380 | 01531 | 01379 | 1 | | 0 | 1 | 0 | 0 | 0 | 0 | 0 | 0 | 0 | 0 | 0 | 0 | 0 | 0 | 0 | 0 | 0 | 0 | 0 | 0 | 0 | 0 | 0 | 0 | 0 | | | | | | |
| 1381 | 01532 | 01380 | 1 | | 0 | 1 | 0 | 0 | 0 | 0 | 0 | 0 | 0 | 0 | 0 | 0 | 0 | 0 | 0 | 0 | 0 | 0 | 0 | 0 | 0 | 0 | 0 | 0 | 0 | | | | | | |
| 1382 | 01533 | 01381 | 19 | | 0 | 1 | 2 | 0 | 0 | 0 | 1 | 0 | 2 | 2 | 1 | 1 | 1 | 0 | 1 | 0 | 0 | 2 | 1 | 0 | 2 | 1 | 1 | 0 | 0 | J04182 | 98.1 | 105 | 1 | 2333 | 2455 |
| 1383 | 01534 | 01382 | 8 | | 0 | 1 | 0 | 0 | 0 | 0 | 0 | 0 | 0 | 0 | 0 | 0 | 1 | 1 | 1 | 2 | 0 | 1 | 0 | 0 | 0 | 1 | 0 | 0 | 0 | | | | | | |
| 1384 | 01535 | 01383 | 1 | | 0 | 1 | 0 | 0 | 0 | 0 | 0 | 0 | 0 | 0 | 0 | 0 | 0 | 0 | 0 | 0 | 0 | 0 | 0 | 0 | 0 | 0 | 0 | 0 | 0 | | | | | | |
| 1385 | 01537 | 01384 | 2 | | 0 | 1 | 0 | 0 | 0 | 0 | 0 | 0 | 0 | 0 | 0 | 0 | 0 | 1 | 0 | 0 | 0 | 0 | 0 | 0 | 0 | 0 | 0 | 0 | 0 | | | | | | |
| 1386 | 01538 | 01385 | 1 | | 0 | 1 | 0 | 0 | 0 | 0 | 0 | 0 | 0 | 0 | 0 | 0 | 0 | 0 | 0 | 0 | 0 | 0 | 0 | 0 | 0 | 0 | 0 | 0 | 0 | | | | | | |
| 1387 | 01539 | 01386 | 12 | | 0 | 1 | 3 | 0 | 0 | 0 | 1 | 0 | 1 | 0 | 0 | 0 | 1 | 0 | 2 | 0 | 0 | 1 | 0 | 2 | 0 | 0 | 0 | 0 | 0 | | | | | | |
| 1388 | 01540 | 01387 | 1 | | 0 | 1 | 0 | 0 | 0 | 0 | 0 | 0 | 0 | 0 | 0 | 0 | 0 | 0 | 0 | 0 | 0 | 0 | 0 | 0 | 0 | 0 | 0 | 0 | 0 | | | | | | |
| 1389 | 01541 | 01388 | 6 | | 0 | 1 | 1 | 0 | 0 | 1 | 0 | 0 | 0 | 0 | 0 | 0 | 0 | 0 | 0 | 0 | 1 | 1 | 0 | 0 | 0 | 0 | 0 | 1 | 0 | | | | | | |

Table 39

| | A | B | C | E | G | I | K | M | O | Q | S | U | W | Y | AA | AC | AE | AG | AI | AK | AM | AO | AQ | AS | AU | AW | AY | BA | BC | BE | BF | BG | BH | BI | BK |
|---|---|---|---|---|---|---|---|---|---|---|---|---|---|---|---|---|---|---|---|---|---|---|---|---|---|---|---|---|---|---|---|---|---|---|---|
| 1390 | 01542 | 01389 | 1 | | 0 | 1 | 0 | 0 | 0 | 0 | 0 | 0 | 0 | 0 | 0 | 0 | 0 | 0 | 0 | 0 | 0 | 0 | 0 | 0 | 0 | 0 | 0 | 0 | 0 | X06814 | 90.1 | 101 | 1 | 552 | 702 |
| 1391 | 01543 | 01390 | 1 | | 0 | 1 | 0 | 0 | 0 | 0 | 0 | 0 | 0 | 0 | 0 | 0 | 0 | 0 | 0 | 0 | 0 | 0 | 0 | 0 | 0 | 0 | 0 | 0 | 0 | | | | | | |
| 1392 | 01544 | 01391 | 4 | | 0 | 2 | 1 | 0 | 0 | 0 | 0 | 0 | 0 | 0 | 0 | 0 | 0 | 0 | 0 | 0 | 0 | 1 | 0 | 0 | 0 | 0 | 0 | 0 | 0 | | | | | | |
| 1393 | 01545 | 01392 | 1 | | 0 | 1 | 0 | 0 | 0 | 0 | 0 | 0 | 0 | 0 | 0 | 0 | 0 | 0 | 0 | 0 | 0 | 0 | 0 | 0 | 0 | 0 | 0 | 0 | 0 | | | | | | |
| 1394 | 01546 | 01393 | 1 | | 0 | 1 | 0 | 0 | 0 | 0 | 0 | 0 | 0 | 0 | 0 | 0 | 0 | 0 | 0 | 0 | 0 | 0 | 0 | 0 | 0 | 0 | 0 | 0 | 0 | | | | | | |
| 1395 | 01547 | 01394 | 1 | | 0 | 1 | 0 | 0 | 0 | 0 | 0 | 0 | 0 | 0 | 0 | 0 | 0 | 0 | 0 | 0 | 0 | 0 | 0 | 0 | 0 | 0 | 0 | 0 | 0 | | | | | | |
| 1396 | 01548 | 01395 | 1 | | 0 | 1 | 0 | 0 | 0 | 0 | 0 | 0 | 0 | 0 | 0 | 0 | 0 | 0 | 0 | 0 | 0 | 0 | 0 | 0 | 0 | 0 | 0 | 0 | 0 | | | | | | |
| 1397 | 01549 | 01396 | 1 | | 0 | 1 | 0 | 0 | 0 | 0 | 0 | 0 | 0 | 0 | 0 | 0 | 0 | 0 | 0 | 0 | 0 | 0 | 0 | 0 | 0 | 0 | 0 | 0 | 0 | | | | | | |
| 1398 | 01550 | 01397 | 3 | | 0 | 1 | 0 | 0 | 0 | 0 | 0 | 0 | 0 | 1 | 0 | 0 | 0 | 0 | 1 | 0 | 0 | 0 | 0 | 0 | 0 | 0 | 0 | 0 | 0 | D25538 | 100 | 95 | 1 | 6102 | 6196 |
| 1399 | 01551 | 01398 | 2 | | 0 | 1 | 0 | 0 | 0 | 0 | 0 | 0 | 0 | 0 | 0 | 0 | 0 | 0 | 0 | 0 | 1 | 0 | 0 | 0 | 0 | 0 | 0 | 0 | 0 | | | | | | |
| 1400 | 01552 | 01399 | 10 | | 0 | 1 | 0 | 0 | 0 | 1 | 0 | 0 | 0 | 0 | 0 | 1 | 1 | 1 | 0 | 0 | 0 | 0 | 0 | 5 | 0 | 0 | 0 | 0 | 0 | | | | | | |
| 1401 | 01553 | 01400 | 5 | | 0 | 2 | 2 | 0 | 0 | 0 | 0 | 0 | 0 | 0 | 0 | 0 | 0 | 0 | 1 | 0 | 0 | 0 | 0 | 0 | 0 | 0 | 0 | 0 | 0 | | | | | | |
| 1402 | 01554 | 01401 | 2 | | 0 | 1 | 0 | 0 | 0 | 0 | 0 | 0 | 0 | 0 | 0 | 0 | 0 | 0 | 0 | 0 | 0 | 0 | 1 | 0 | 0 | 0 | 0 | 0 | 0 | | | | | | |
| 1403 | 01555 | 01402 | 1 | | 0 | 1 | 0 | 0 | 0 | 0 | 0 | 0 | 0 | 0 | 0 | 0 | 0 | 0 | 0 | 0 | 0 | 0 | 0 | 0 | 0 | 0 | 0 | 0 | 0 | M95767 | 97.8 | 89 | 1 | 1208 | 1618 |
| 1404 | 01556 | 01403 | 1 | | 0 | 1 | 0 | 0 | 0 | 0 | 0 | 0 | 0 | 0 | 0 | 0 | 0 | 0 | 0 | 0 | 0 | 0 | 0 | 0 | 0 | 0 | 0 | 0 | 0 | | | | | | |
| 1405 | 01557 | 01404 | 5 | | 0 | 1 | 0 | 0 | 0 | 0 | 0 | 0 | 0 | 0 | 0 | 0 | 0 | 0 | 0 | 0 | 0 | 0 | 0 | 1 | 1 | 0 | 2 | 0 | 0 | | | | | | |
| 1406 | 01558 | 01405 | 2 | | 0 | 1 | 0 | 0 | 0 | 0 | 0 | 0 | 0 | 0 | 0 | 0 | 0 | 0 | 0 | 0 | 0 | 0 | 0 | 0 | 0 | 0 | 1 | 0 | 0 | | | | | | |
| 1407 | 01560 | 01406 | 2 | | 0 | 1 | 0 | 0 | 0 | 0 | 0 | 0 | 0 | 0 | 0 | 0 | 0 | 0 | 0 | 0 | 0 | 0 | 1 | 0 | 0 | 0 | 0 | 0 | 0 | | | | | | |
| 1408 | 01561 | 01407 | 2 | | 0 | 2 | 0 | 0 | 0 | 0 | 0 | 0 | 0 | 0 | 0 | 0 | 0 | 0 | 0 | 0 | 0 | 0 | 0 | 0 | 0 | 0 | 0 | 0 | 0 | | | | | | |
| 1409 | 01562 | 01408 | 1 | | 0 | 1 | 0 | 0 | 0 | 0 | 0 | 0 | 0 | 0 | 0 | 0 | 0 | 0 | 0 | 0 | 0 | 0 | 0 | 0 | 0 | 0 | 0 | 0 | 0 | | | | | | |
| 1410 | 01563 | 01409 | 1 | | 0 | 1 | 0 | 0 | 0 | 0 | 0 | 0 | 0 | 0 | 0 | 0 | 0 | 0 | 0 | 0 | 0 | 0 | 0 | 0 | 0 | 0 | 0 | 0 | 0 | | | | | | |
| 1411 | 01564 | 01410 | 1 | | 0 | 1 | 0 | 0 | 0 | 0 | 0 | 0 | 0 | 0 | 0 | 0 | 0 | 0 | 0 | 0 | 0 | 0 | 0 | 0 | 0 | 0 | 0 | 0 | 0 | | | | | | |
| 1412 | 01565 | 01411 | 6 | | 0 | 2 | 0 | 2 | 0 | 0 | 1 | 0 | 0 | 0 | 0 | 0 | 0 | 0 | 0 | 1 | 0 | 0 | 0 | 0 | 0 | 0 | 0 | 0 | 0 | M79463 | 100 | 84 | 1 | 3492 | 3587 |
| 1413 | 01566 | 01412 | 9 | | 0 | 1 | 0 | 0 | 1 | 0 | 0 | 0 | 0 | 0 | 0 | 0 | 0 | 0 | 0 | 1 | 0 | 2 | 0 | 0 | 1 | 1 | 1 | 1 | 0 | X71428 | 94.2 | 86 | 1 | 1734 | 1822 |
| 1414 | 01567 | 01413 | 1 | | 0 | 1 | 0 | 0 | 0 | 0 | 0 | 0 | 0 | 0 | 0 | 0 | 0 | 0 | 0 | 0 | 0 | 0 | 0 | 0 | 0 | 0 | 0 | 0 | 0 | | | | | | |
| 1415 | 01568 | 01414 | 1 | | 0 | 1 | 0 | 0 | 0 | 0 | 0 | 0 | 0 | 0 | 0 | 0 | 0 | 0 | 0 | 0 | 0 | 0 | 0 | 0 | 0 | 0 | 0 | 0 | 0 | | | | | | |
| 1416 | 01569 | 01415 | 6 | | 0 | 1 | 0 | 1 | 0 | 0 | 0 | 0 | 0 | 0 | 0 | 0 | 0 | 0 | 0 | 0 | 0 | 3 | 0 | 0 | 1 | 0 | 0 | 0 | 0 | | | | | | |
| 1417 | 01570 | 01416 | 7 | | 0 | 3 | 0 | 0 | 0 | 0 | 0 | 0 | 0 | 0 | 0 | 0 | 0 | 0 | 1 | 1 | 0 | 0 | 0 | 0 | 1 | 1 | 0 | 0 | 0 | X65019 | 98.8 | 82 | 1 | 1263 | 1358 |
| 1418 | 01571 | 01417 | 10 | | 0 | 1 | 0 | 0 | 0 | 0 | 0 | 0 | 1 | 0 | 0 | 1 | 0 | 2 | 0 | 0 | 0 | 0 | 2 | 2 | 0 | 1 | 0 | 0 | 0 | J03805 | 96.3 | 81 | 1 | 1449 | 1529 |
| 1419 | 01572 | 01418 | 4 | | 0 | 4 | 0 | 0 | 0 | 0 | 0 | 0 | 0 | 0 | 0 | 0 | 0 | 0 | 0 | 0 | 0 | 0 | 0 | 0 | 0 | 0 | 0 | 0 | 0 | | | | | | |
| 1420 | 01573 | 01419 | 2 | | 0 | 1 | 1 | 0 | 0 | 0 | 0 | 0 | 0 | 0 | 0 | 0 | 0 | 0 | 0 | 0 | 0 | 0 | 0 | 0 | 0 | 0 | 0 | 0 | 0 | | | | | | |
| 1421 | 01574 | 01420 | 11 | | 0 | 2 | 0 | 1 | 0 | 0 | 1 | 0 | 0 | 0 | 0 | 0 | 2 | 1 | 0 | 0 | 1 | 0 | 0 | 1 | 0 | 1 | 1 | 0 | 0 | | | | | | |
| 1422 | 01575 | 01421 | 6 | | 0 | 2 | 0 | 0 | 0 | 0 | 0 | 0 | 0 | 0 | 0 | 0 | 0 | 0 | 0 | 0 | 0 | 0 | 0 | 0 | 0 | 1 | 0 | 0 | 3 | | | | | | |
| 1423 | 01576 | 01422 | 6 | | 0 | 1 | 0 | 0 | 0 | 0 | 0 | 0 | 0 | 1 | 0 | 0 | 1 | 0 | 0 | 1 | 0 | 0 | 1 | 1 | 0 | 0 | 0 | 0 | 0 | | | | | | |
| 1424 | 01577 | 01423 | 5 | | 0 | 2 | 1 | 0 | 0 | 0 | 0 | 0 | 0 | 0 | 0 | 0 | 0 | 0 | 0 | 0 | 0 | 1 | 0 | 0 | 0 | 1 | 0 | 0 | 0 | | | | | | |
| 1425 | 01578 | 01424 | 1 | | 0 | 1 | 0 | 0 | 0 | 0 | 0 | 0 | 0 | 0 | 0 | 0 | 0 | 0 | 0 | 0 | 0 | 0 | 0 | 0 | 0 | 0 | 0 | 0 | 0 | | | | | | |

Table 40

| | A | B | C | E | G | I | K | M | O | Q | S | U | W | Y | AA | AC | AE | AG | AI | AK | AM | AO | AQ | AS | AU | AW | AY | BA | BC | BE | BF | BG | BH | BI | BK |
|---|---|---|---|---|---|---|---|---|---|---|---|---|---|---|---|---|---|---|---|---|---|---|---|---|---|---|---|---|---|---|---|---|---|---|---|
| 1426 | 01579 | 01425 | 1 | | 0 | 1 | 0 | 0 | 0 | 0 | 0 | 0 | 0 | 0 | 0 | 0 | 0 | 0 | 0 | 0 | 0 | 0 | 0 | 0 | 0 | 0 | 0 | 0 | 0 | | | | | | |
| 1427 | 01580 | 01426 | 4 | | 0 | 1 | 0 | 1 | 0 | 0 | 0 | 1 | 0 | 0 | 0 | 0 | 0 | 1 | 0 | 0 | 0 | 0 | 0 | 0 | 0 | 0 | 0 | 0 | 0 | | | | | | |
| 1428 | 01581 | 01427 | 1 | | 0 | 1 | 0 | 0 | 0 | 0 | 0 | 0 | 0 | 0 | 0 | 0 | 0 | 0 | 0 | 0 | 0 | 0 | 0 | 0 | 0 | 0 | 0 | 0 | 0 | M81601 | 96.1 | 51 | 28 | 2573 | 2634 |
| 1429 | 01582 | 01428 | 3 | | 0 | 2 | 0 | 0 | 0 | 0 | 0 | 0 | 0 | 0 | 0 | 0 | 0 | 0 | 0 | 0 | 0 | 0 | 0 | 0 | 0 | 0 | 1 | 0 | 0 | | | | | | |
| 1430 | 01583 | 01429 | 5 | | 0 | 1 | 1 | 0 | 0 | 0 | 0 | 0 | 1 | 0 | 0 | 1 | 0 | 0 | 0 | 0 | 0 | 1 | 0 | 0 | 0 | 0 | 0 | 0 | 0 | U02389 | 100 | 76 | 1 | 3106 | 3193 |
| 1431 | 01584 | 01430 | 1 | | 0 | 1 | 0 | 0 | 0 | 0 | 0 | 0 | 0 | 0 | 0 | 0 | 0 | 0 | 0 | 0 | 0 | 0 | 0 | 0 | 0 | 0 | 0 | 0 | 0 | | | | | | |
| 1432 | 01585 | 01431 | 8 | | 0 | 2 | 3 | 0 | 0 | 0 | 0 | 0 | 0 | 0 | 0 | 0 | 0 | 0 | 0 | 0 | 0 | 0 | 0 | 0 | 0 | 0 | 3 | 0 | 0 | M32315 | 100 | 75 | 1 | 3604 | 3683 |
| 1433 | 01586 | 01432 | 2 | | 0 | 2 | 0 | 0 | 0 | 0 | 0 | 0 | 0 | 0 | 0 | 0 | 0 | 0 | 0 | 0 | 0 | 0 | 0 | 0 | 0 | 0 | 0 | 0 | 0 | | | | | | |
| 1434 | 01587 | 01433 | 2 | | 0 | 1 | 0 | 0 | 0 | 0 | 0 | 0 | 0 | 0 | 0 | 0 | 0 | 0 | 1 | 0 | 0 | 0 | 0 | 0 | 0 | 0 | 0 | 0 | 0 | | | | | | |
| 1435 | 01588 | 01434 | 1 | | 0 | 1 | 0 | 0 | 0 | 0 | 0 | 0 | 0 | 0 | 0 | 0 | 0 | 0 | 0 | 0 | 0 | 0 | 0 | 0 | 0 | 0 | 0 | 0 | 0 | | | | | | |
| 1436 | 01589 | 01435 | 10 | | 0 | 1 | 0 | 0 | 0 | 1 | 0 | 0 | 3 | 1 | 1 | 0 | 0 | 0 | 0 | 0 | 1 | 0 | 0 | 1 | 0 | 0 | 1 | 0 | 0 | | | | | | |
| 1437 | 01590 | 01436 | 1 | | 0 | 1 | 0 | 0 | 0 | 0 | 0 | 0 | 0 | 0 | 0 | 0 | 0 | 0 | 0 | 0 | 0 | 0 | 0 | 0 | 0 | 0 | 0 | 0 | 0 | | | | | | |
| 1438 | 01591 | 01437 | 1 | | 0 | 1 | 0 | 0 | 0 | 0 | 0 | 0 | 0 | 0 | 0 | 0 | 0 | 0 | 0 | 0 | 0 | 0 | 0 | 0 | 0 | 0 | 0 | 0 | 0 | | | | | | |
| 1439 | 01592 | 01438 | 4 | | 0 | 1 | 0 | 1 | 0 | 0 | 0 | 1 | 0 | 0 | 0 | 0 | 0 | 0 | 0 | 0 | 0 | 0 | 1 | 0 | 0 | 0 | 0 | 0 | 0 | | | | | | |
| 1440 | 01593 | 01439 | 1 | | 0 | 1 | 0 | 0 | 0 | 0 | 0 | 0 | 0 | 0 | 0 | 0 | 0 | 0 | 0 | 0 | 0 | 0 | 0 | 0 | 0 | 0 | 0 | 0 | 0 | | | | | | |
| 1441 | 01594 | 01440 | 4 | | 0 | 1 | 0 | 0 | 0 | 0 | 0 | 0 | 0 | 0 | 0 | 0 | 0 | 0 | 0 | 0 | 0 | 0 | 0 | 0 | 0 | 0 | 3 | 0 | 0 | Y00062 | 95.1 | 364 | 1 | 3982 | 4597 |
| 1442 | 01595 | 01441 | 7 | | 0 | 1 | 0 | 0 | 0 | 1 | 0 | 0 | 0 | 2 | 1 | 0 | 0 | 0 | 0 | 0 | 0 | 0 | 0 | 0 | 2 | 0 | 0 | 0 | 0 | | | | | | |
| 1443 | 01596 | 01442 | 1 | | 0 | 1 | 0 | 0 | 0 | 0 | 0 | 0 | 0 | 0 | 0 | 0 | 0 | 0 | 0 | 0 | 0 | 0 | 0 | 0 | 0 | 0 | 0 | 0 | 0 | | | | | | |
| 1444 | 01597 | 01443 | 2 | | 0 | 1 | 0 | 0 | 0 | 0 | 0 | 0 | 0 | 0 | 0 | 0 | 0 | 0 | 0 | 0 | 0 | 0 | 0 | 0 | 0 | 0 | 1 | 0 | 0 | | | | | | |
| 1445 | 01598 | 01444 | 1 | | 0 | 1 | 0 | 0 | 0 | 0 | 0 | 0 | 0 | 0 | 0 | 0 | 0 | 0 | 0 | 0 | 0 | 0 | 0 | 0 | 0 | 0 | 0 | 0 | 0 | | | | | | |
| 1446 | 01599 | 01445 | 1 | | 0 | 1 | 0 | 0 | 0 | 0 | 0 | 0 | 0 | 0 | 0 | 0 | 0 | 0 | 0 | 0 | 0 | 0 | 0 | 0 | 0 | 0 | 0 | 0 | 0 | | | | | | |
| 1447 | 01600 | 01446 | 2 | | 0 | 1 | 0 | 0 | 0 | 0 | 0 | 0 | 0 | 0 | 0 | 0 | 0 | 0 | 0 | 0 | 0 | 0 | 0 | 0 | 0 | 0 | 0 | 1 | 0 | | | | | | |
| 1448 | 01601 | 01447 | 1 | | 0 | 1 | 0 | 0 | 0 | 0 | 0 | 0 | 0 | 0 | 0 | 0 | 0 | 0 | 0 | 0 | 0 | 0 | 0 | 0 | 0 | 0 | 0 | 0 | 0 | | | | | | |
| 1449 | 01602 | 01448 | 4 | | 0 | 1 | 0 | 1 | 0 | 0 | 0 | 0 | 0 | 0 | 0 | 0 | 0 | 0 | 1 | 0 | 0 | 0 | 0 | 0 | 0 | 0 | 1 | 0 | 0 | | | | | | |
| 1450 | 01603 | 01449 | 5 | | 0 | 4 | 0 | 0 | 0 | 0 | 0 | 0 | 0 | 0 | 0 | 0 | 0 | 0 | 0 | 0 | 0 | 0 | 0 | 0 | 0 | 0 | 1 | 0 | 0 | | | | | | |
| 1451 | 01604 | 01450 | 1 | | 0 | 1 | 0 | 0 | 0 | 0 | 0 | 0 | 0 | 0 | 0 | 0 | 0 | 0 | 0 | 0 | 0 | 0 | 0 | 0 | 0 | 0 | 0 | 0 | 0 | | | | | | |
| 1452 | 01605 | 01451 | 2 | | 0 | 1 | 0 | 0 | 0 | 0 | 0 | 0 | 0 | 0 | 0 | 0 | 0 | 0 | 0 | 0 | 1 | 0 | 0 | 0 | 0 | 0 | 0 | 0 | 0 | | | | | | |
| 1453 | 01606 | 01452 | 1 | | 0 | 1 | 0 | 0 | 0 | 0 | 0 | 0 | 0 | 0 | 0 | 0 | 0 | 0 | 0 | 0 | 0 | 0 | 0 | 0 | 0 | 0 | 0 | 0 | 0 | | | | | | |
| 1454 | 01607 | 01453 | 4 | | 0 | 1 | 0 | 0 | 0 | 0 | 0 | 0 | 1 | 0 | 0 | 0 | 0 | 0 | 0 | 0 | 0 | 0 | 0 | 1 | 0 | 0 | 0 | 0 | 1 | | | | | | |
| 1455 | 01608 | 01454 | 23 | | 0 | 2 | 0 | 0 | 0 | 0 | 0 | 1 | 2 | 3 | 0 | 0 | 5 | 0 | 0 | 2 | 1 | 3 | 0 | 1 | 2 | 0 | 1 | 0 | 0 | S60099 | 100 | 54 | 1 | 3673 | 3727 |
| 1456 | 01609 | 01455 | 16 | | 0 | 4 | 0 | 0 | 0 | 1 | 2 | 0 | 0 | 0 | 1 | 1 | 0 | 0 | 2 | 4 | 0 | 0 | 0 | 0 | 0 | 0 | 1 | 0 | 0 | J03909 | 100 | 52 | 1 | 981 | 1032 |
| 1457 | 01610 | 01456 | 5 | | 0 | 1 | 0 | 0 | 0 | 0 | 0 | 0 | 0 | 0 | 1 | 3 | 0 | 0 | 0 | 0 | 0 | 0 | 0 | 0 | 0 | 0 | 0 | 0 | 0 | | | | | | |
| 1458 | 01611 | 01457 | 3 | | 0 | 2 | 0 | 0 | 0 | 0 | 0 | 0 | 0 | 0 | 0 | 0 | 0 | 0 | 0 | 0 | 1 | 0 | 0 | 0 | 0 | 0 | 0 | 0 | 0 | | | | | | |
| 1459 | 01612 | 01458 | 5 | | 0 | 1 | 0 | 1 | 1 | 0 | 0 | 0 | 0 | 0 | 0 | 1 | 0 | 0 | 0 | 0 | 0 | 0 | 0 | 1 | 0 | 0 | 0 | 0 | 0 | | | | | | |
| 1460 | 01614 | 01459 | 4 | | 0 | 1 | 0 | 1 | 0 | 0 | 0 | 0 | 0 | 0 | 1 | 0 | 0 | 1 | 0 | 0 | 0 | 0 | 0 | 0 | 0 | 0 | 0 | 0 | 0 | | | | | | |
| 1461 | 01615 | 01460 | 1 | | 0 | 1 | 0 | 0 | 0 | 0 | 0 | 0 | 0 | 0 | 0 | 0 | 0 | 0 | 0 | 0 | 0 | 0 | 0 | 0 | 0 | 0 | 0 | 0 | 0 | | | | | | |

Table 41

| | A | B | C | E | G | I | K | M | O | Q | S | U | W | Y | AA | AC | AE | AG | AI | AK | AM | AO | AQ | AS | AU | AW | AY | BA | BC | BE | BF | BG | BH | BI | BK |
|---|---|---|---|---|---|---|---|---|---|---|---|---|---|---|---|---|---|---|---|---|---|---|---|---|---|---|---|---|---|---|---|---|---|---|---|
| 1462 | 01616 | 01461 | 32 | | 0 | 1 | 3 | 1 | 0 | 0 | 0 | 0 | 0 | 3 | 0 | 0 | 1 | 0 | 5 | 11 | 0 | 0 | 2 | 1 | 0 | 0 | 1 | 0 | 3 | L03558 | 98.2 | 55 | 1 | 588 | 642 |
| 1463 | 01617 | 01462 | 1 | | 0 | 1 | 0 | 0 | 0 | 0 | 0 | 0 | 0 | 0 | 0 | 0 | 0 | 0 | 0 | 0 | 0 | 0 | 0 | 0 | 0 | 0 | 0 | 0 | 0 | | | | | | |
| 1464 | 01618 | 01463 | 1 | | 0 | 1 | 0 | 0 | 0 | 0 | 0 | 0 | 0 | 0 | 0 | 0 | 0 | 0 | 0 | 0 | 0 | 0 | 0 | 0 | 0 | 0 | 0 | 0 | 0 | | | | | | |
| 1465 | 01619 | 01464 | 1 | | 0 | 1 | 0 | 0 | 0 | 0 | 0 | 0 | 0 | 0 | 0 | 0 | 0 | 0 | 0 | 0 | 0 | 0 | 0 | 0 | 0 | 0 | 0 | 0 | 0 | | | | | | |
| 1466 | 01620 | 01465 | 4 | | 0 | 1 | 0 | 0 | 0 | 0 | 0 | 0 | 0 | 0 | 0 | 0 | 0 | 0 | 1 | 0 | 0 | 0 | 1 | 0 | 0 | 0 | 0 | 1 | 0 | | | | | | |
| 1467 | 01621 | 01466 | 1 | | 0 | 1 | 0 | 0 | 0 | 0 | 0 | 0 | 0 | 0 | 0 | 0 | 0 | 0 | 0 | 0 | 0 | 0 | 0 | 0 | 0 | 0 | 0 | 0 | 0 | | | | | | |
| 1468 | 01622 | 01467 | 2 | | 0 | 1 | 0 | 0 | 0 | 0 | 0 | 0 | 0 | 0 | 0 | 0 | 0 | 0 | 0 | 0 | 0 | 0 | 0 | 0 | 0 | 0 | 1 | 0 | 0 | | | | | | |
| 1469 | 01623 | 01468 | 5 | | 0 | 1 | 0 | 0 | 0 | 0 | 0 | 1 | 1 | 0 | 0 | 0 | 0 | 0 | 0 | 0 | 0 | 1 | 1 | 0 | 0 | 0 | 0 | 0 | 0 | | | | | | |
| 1470 | 01624 | 01469 | 8 | | 0 | 2 | 0 | 0 | 0 | 0 | 0 | 0 | 0 | 1 | 0 | 0 | 0 | 0 | 2 | 0 | 0 | 1 | 0 | 0 | 0 | 0 | 2 | 0 | 0 | M22324 | 100 | 54 | 1 | 3427 | 3494 |
| 1471 | 01625 | 01470 | 3 | | 0 | 1 | 0 | 0 | 0 | 0 | 0 | 0 | 0 | 0 | 0 | 0 | 0 | 0 | 0 | 0 | 0 | 0 | 0 | 0 | 2 | 0 | 0 | 0 | 0 | | | | | | |
| 1472 | 01626 | 01471 | 1 | | 0 | 1 | 0 | 0 | 0 | 0 | 0 | 0 | 0 | 0 | 0 | 0 | 0 | 0 | 0 | 0 | 0 | 0 | 0 | 0 | 0 | 0 | 0 | 0 | 0 | | | | | | |
| 1473 | 01627 | 01472 | 4 | | 0 | 1 | 0 | 0 | 0 | 0 | 0 | 0 | 0 | 0 | 0 | 1 | 0 | 0 | 0 | 0 | 0 | 0 | 0 | 0 | 0 | 0 | 2 | 0 | 0 | | | | | | |
| 1474 | 01628 | 01473 | 1 | | 0 | 1 | 0 | 0 | 0 | 0 | 0 | 0 | 0 | 0 | 0 | 0 | 0 | 0 | 0 | 0 | 0 | 0 | 0 | 0 | 0 | 0 | 0 | 0 | 0 | | | | | | |
| 1475 | 01629 | 01474 | 1 | | 0 | 1 | 0 | 0 | 0 | 0 | 0 | 0 | 0 | 0 | 0 | 0 | 0 | 0 | 0 | 0 | 0 | 0 | 0 | 0 | 0 | 0 | 0 | 0 | 0 | | | | | | |
| 1476 | 01630 | 01475 | 9 | | 0 | 1 | 0 | 0 | 0 | 0 | 0 | 0 | 0 | 0 | 3 | 0 | 0 | 0 | 0 | 0 | 0 | 2 | 1 | 0 | 1 | 1 | 0 | 0 | 0 | | | | | | |
| 1477 | 01632 | 01476 | 11 | | 0 | 3 | 0 | 0 | 0 | 0 | 1 | 0 | 0 | 0 | 0 | 0 | 1 | 2 | 0 | 1 | 0 | 0 | 0 | 1 | 0 | 0 | 0 | 2 | 0 | | | | | | |
| 1478 | 01633 | 01477 | 7 | | 0 | 1 | 0 | 1 | 0 | 1 | 0 | 0 | 0 | 1 | 0 | 0 | 0 | 0 | 0 | 1 | 0 | 0 | 0 | 0 | 0 | 0 | 2 | 0 | 0 | | | | | | |
| 1479 | 01642 | 01478 | 3 | | 0 | 1 | 0 | 0 | 1 | 0 | 0 | 0 | 0 | 0 | 0 | 0 | 0 | 0 | 0 | 1 | 0 | 0 | 0 | 0 | 0 | 0 | 0 | 0 | 0 | | | | | | |
| 1480 | 01671 | 01479 | 35 | | 0 | 2 | 0 | 0 | 0 | 0 | 5 | 7 | 3 | 2 | 0 | 0 | 1 | 6 | 0 | 0 | 0 | 3 | 2 | 0 | 2 | 1 | 0 | 1 | 0 | | | | | | |
| 1481 | 01673 | 01480 | 6 | | 0 | 1 | 0 | 0 | 0 | 0 | 1 | 0 | 0 | 0 | 0 | 1 | 0 | 1 | 0 | 2 | 0 | 0 | 0 | 0 | 0 | 0 | 0 | 0 | 0 | | | | | | |
| 1482 | 01686 | 01481 | 1 | | 0 | 0 | 1 | 0 | 0 | 0 | 0 | 0 | 0 | 0 | 0 | 0 | 0 | 0 | 0 | 0 | 0 | 0 | 0 | 0 | 0 | 0 | 0 | 0 | 0 | | | | | | |
| 1483 | 01687 | 01482 | 2 | | 0 | 0 | 1 | 0 | 0 | 0 | 0 | 0 | 0 | 0 | 0 | 0 | 0 | 0 | 0 | 0 | 0 | 0 | 0 | 0 | 0 | 0 | 1 | 0 | 0 | X06409 | 93.9 | 408 | 1 | 1579 | 2602 |
| 1484 | 01688 | 01483 | 6 | | 0 | 0 | 1 | 0 | 0 | 0 | 0 | 0 | 0 | 0 | 0 | 0 | 0 | 1 | 1 | 0 | 2 | 0 | 0 | 0 | 0 | 1 | 0 | 0 | 0 | X04098 | 98.2 | 325 | 1 | 1093 | 1918 |
| 1485 | 01689 | 01484 | 1 | | 0 | 0 | 1 | 0 | 0 | 0 | 0 | 0 | 0 | 0 | 0 | 0 | 0 | 0 | 0 | 0 | 0 | 0 | 0 | 0 | 0 | 0 | 0 | 0 | 0 | | | | | | |
| 1486 | 01691 | 01485 | 1 | | 0 | 0 | 1 | 0 | 0 | 0 | 0 | 0 | 0 | 0 | 0 | 0 | 0 | 0 | 0 | 0 | 0 | 0 | 0 | 0 | 0 | 0 | 0 | 0 | 0 | | | | | | |
| 1487 | 01692 | 01486 | 1 | | 0 | 0 | 1 | 0 | 0 | 0 | 0 | 0 | 0 | 0 | 0 | 0 | 0 | 0 | 0 | 0 | 0 | 0 | 0 | 0 | 0 | 0 | 0 | 0 | 0 | | | | | | |
| 1488 | 01693 | 01487 | 5 | | 0 | 0 | 1 | 0 | 0 | 0 | 0 | 0 | 0 | 0 | 0 | 0 | 0 | 0 | 0 | 0 | 0 | 0 | 0 | 2 | 0 | 0 | 0 | 2 | 0 | | | | | | |
| 1489 | 01694 | 01488 | 1 | | 0 | 0 | 1 | 0 | 0 | 0 | 0 | 0 | 0 | 0 | 0 | 0 | 0 | 0 | 0 | 0 | 0 | 0 | 0 | 0 | 0 | 0 | 0 | 0 | 0 | | | | | | |
| 1490 | 01695 | 01489 | 11 | | 0 | 0 | 2 | 0 | 0 | 0 | 0 | 0 | 0 | 0 | 2 | 0 | 0 | 3 | 0 | 1 | 1 | 0 | 0 | 1 | 0 | 0 | 0 | 1 | 0 | M34600 | 96.2 | 524 | 1 | 118 | 634 |
| 1491 | 01696 | 01490 | 1 | | 0 | 0 | 1 | 0 | 0 | 0 | 0 | 0 | 0 | 0 | 0 | 0 | 0 | 0 | 0 | 0 | 0 | 0 | 0 | 0 | 0 | 0 | 0 | 0 | 0 | | | | | | |
| 1492 | 01697 | 01491 | 4 | | 0 | 0 | 1 | 0 | 0 | 0 | 0 | 0 | 0 | 0 | 0 | 0 | 0 | 0 | 0 | 2 | 0 | 0 | 0 | 0 | 0 | 0 | 0 | 1 | 0 | X01060 | 95.6 | 317 | 4 | 4532 | 5010 |
| 1493 | 01698 | 01492 | 2 | | 0 | 0 | 1 | 0 | 0 | 0 | 0 | 0 | 0 | 0 | 0 | 0 | 0 | 0 | 0 | 0 | 0 | 0 | 0 | 0 | 1 | 0 | 0 | 0 | 0 | | | | | | |
| 1494 | 01699 | 01493 | 2 | | 0 | 0 | 1 | 0 | 0 | 0 | 0 | 0 | 0 | 0 | 0 | 0 | 0 | 0 | 0 | 0 | 0 | 0 | 0 | 0 | 0 | 0 | 0 | 1 | 0 | | | | | | |
| 1495 | 01700 | 01494 | 9 | | 0 | 0 | 4 | 1 | 1 | 1 | 0 | 0 | 0 | 0 | 0 | 0 | 1 | 0 | 0 | 0 | 0 | 0 | 1 | 0 | 0 | 0 | 0 | 0 | 0 | | | | | | |
| 1496 | 01701 | 01495 | 1 | | 0 | 0 | 1 | 0 | 0 | 0 | 0 | 0 | 0 | 0 | 0 | 0 | 0 | 0 | 0 | 0 | 0 | 0 | 0 | 0 | 0 | 0 | 0 | 0 | 0 | | | | | | |
| 1497 | 01702 | 01496 | 4 | | 0 | 0 | 1 | 0 | 0 | 0 | 0 | 0 | 0 | 0 | 0 | 0 | 0 | 0 | 1 | 0 | 0 | 0 | 0 | 1 | 0 | 0 | 1 | 0 | 0 | M22918 | 99.2 | 394 | 1 | 109 | 651 |

Table 42

| | A | B | C | E | G | I | K | M | O | Q | S | U | W | Y | AA | AC | AE | AG | AI | AK | AM | AO | AQ | AS | AU | AW | AY | BA | BC | BE | BF | BG | BH | BI | BK |
|---|---|---|---|---|---|---|---|---|---|---|---|---|---|---|---|---|---|---|---|---|---|---|---|---|---|---|---|---|---|---|---|---|---|---|---|
| 1498 | 01703 | 01497 | 1 | | 0 | 0 | 1 | 0 | 0 | 0 | 0 | 0 | 0 | 0 | 0 | 0 | 0 | 0 | 0 | 0 | 0 | 0 | 0 | 0 | 0 | 0 | 0 | 0 | 0 | | | | | | |
| 1499 | 01704 | 01498 | 1 | | 0 | 0 | 1 | 0 | 0 | 0 | 0 | 0 | 0 | 0 | 0 | 0 | 0 | 0 | 0 | 0 | 0 | 0 | 0 | 0 | 0 | 0 | 0 | 0 | 0 | | | | | | |
| 1500 | 01705 | 01499 | 1 | | 0 | 0 | 1 | 0 | 0 | 0 | 0 | 0 | 0 | 0 | 0 | 0 | 0 | 0 | 0 | 0 | 0 | 0 | 0 | 0 | 0 | 0 | 0 | 0 | 0 | | | | | | |
| 1501 | 01706 | 01500 | 12 | | 0 | 0 | 4 | 0 | 0 | 0 | 2 | 0 | 0 | 0 | 0 | 0 | 0 | 0 | 0 | 0 | 0 | 0 | 0 | 0 | 0 | 6 | 0 | 0 | 0 | M80563 | 93.1 | 421 | 1 | 120 | 580 |
| 1502 | 01707 | 01501 | 1 | | 0 | 0 | 1 | 0 | 0 | 0 | 0 | 0 | 0 | 0 | 0 | 0 | 0 | 0 | 0 | 0 | 0 | 0 | 0 | 0 | 0 | 0 | 0 | 0 | 0 | M86752 | 95.3 | 471 | 1 | 1652 | 2113 |
| 1503 | 01708 | 01502 | 5 | | 0 | 0 | 1 | 0 | 0 | 0 | 0 | 0 | 0 | 1 | 0 | 0 | 0 | 1 | 0 | 0 | 0 | 0 | 0 | 0 | 0 | 1 | 0 | 1 | 0 | | | | | | |
| 1504 | 01709 | 01503 | 1 | | 0 | 0 | 1 | 0 | 0 | 0 | 0 | 0 | 0 | 0 | 0 | 0 | 0 | 0 | 0 | 0 | 0 | 0 | 0 | 0 | 0 | 0 | 0 | 0 | 0 | | | | | | |
| 1505 | 01710 | 01504 | 2 | | 0 | 0 | 1 | 0 | 0 | 0 | 0 | 0 | 0 | 0 | 0 | 0 | 0 | 0 | 0 | 0 | 0 | 0 | 0 | 0 | 0 | 0 | 1 | 0 | 0 | | | | | | |
| 1506 | 01711 | 01505 | 1 | | 0 | 0 | 1 | 0 | 0 | 0 | 0 | 0 | 0 | 0 | 0 | 0 | 0 | 0 | 0 | 0 | 0 | 0 | 0 | 0 | 0 | 0 | 0 | 0 | 0 | | | | | | |
| 1507 | 01713 | 01506 | 1 | | 0 | 0 | 1 | 0 | 0 | 0 | 0 | 0 | 0 | 0 | 0 | 0 | 0 | 0 | 0 | 0 | 0 | 0 | 0 | 0 | 0 | 0 | 0 | 0 | 0 | | | | | | |
| 1508 | 01714 | 01507 | 3 | | 0 | 0 | 2 | 0 | 0 | 0 | 0 | 0 | 0 | 0 | 0 | 0 | 0 | 0 | 0 | 1 | 0 | 0 | 0 | 0 | 0 | 0 | 0 | 0 | 0 | M15330 | 96.4 | 448 | 1 | 1052 | 1497 |
| 1509 | 01715 | 01508 | 1 | | 0 | 0 | 1 | 0 | 0 | 0 | 0 | 0 | 0 | 0 | 0 | 0 | 0 | 0 | 0 | 0 | 0 | 0 | 0 | 0 | 0 | 0 | 0 | 0 | 0 | | | | | | |
| 1510 | 01718 | 01509 | 2 | | 0 | 0 | 1 | 0 | 0 | 0 | 0 | 0 | 0 | 0 | 0 | 0 | 0 | 0 | 0 | 0 | 0 | 0 | 0 | 0 | 0 | 0 | 1 | 0 | 0 | | | | | | |
| 1511 | 01719 | 01510 | 3 | | 0 | 0 | 1 | 0 | 0 | 0 | 0 | 0 | 0 | 0 | 0 | 0 | 0 | 0 | 0 | 0 | 0 | 0 | 0 | 0 | 0 | 0 | 2 | 0 | 0 | M81750 | 98.3 | 345 | 1 | 1239 | 1672 |
| 1512 | 01720 | 01511 | 43 | | 0 | 0 | 1 | 0 | 0 | 0 | 4 | 2 | 4 | 3 | 1 | 1 | 0 | 0 | 5 | 7 | 9 | 4 | 0 | 0 | 0 | 2 | 0 | 0 | 0 | M14043 | 94.8 | 461 | 1 | 913 | 1362 |
| 1513 | 01721 | 01512 | 2 | | 0 | 0 | 1 | 0 | 0 | 0 | 0 | 0 | 0 | 0 | 0 | 0 | 0 | 0 | 1 | 0 | 0 | 0 | 0 | 0 | 0 | 0 | 0 | 0 | 0 | | | | | | |
| 1514 | 01722 | 01513 | 4 | | 0 | 0 | 1 | 0 | 0 | 0 | 0 | 0 | 0 | 0 | 2 | 0 | 0 | 1 | 0 | 0 | 0 | 0 | 0 | 0 | 0 | 0 | 0 | 0 | 0 | | | | | | |
| 1515 | 01724 | 01514 | 9 | | 0 | 0 | 2 | 0 | 0 | 0 | 0 | 0 | 0 | 0 | 0 | 0 | 0 | 0 | 1 | 0 | 0 | 0 | 0 | 0 | 0 | 0 | 6 | 0 | 0 | X07743 | 96.2 | 426 | 1 | 2322 | 2745 |
| 1516 | 01726 | 01515 | 5 | | 0 | 0 | 2 | 0 | 0 | 0 | 0 | 0 | 0 | 0 | 0 | 1 | 0 | 0 | 0 | 0 | 0 | 0 | 0 | 0 | 1 | 0 | 1 | 0 | 0 | M96843 | 90.9 | 364 | 1 | 808 | 1167 |
| 1517 | 01727 | 01516 | 4 | | 0 | 0 | 1 | 0 | 0 | 0 | 0 | 0 | 0 | 0 | 0 | 0 | 0 | 0 | 0 | 0 | 0 | 2 | 0 | 0 | 1 | 0 | 0 | 0 | 0 | | | | | | |
| 1518 | 01728 | 01517 | 1 | | 0 | 0 | 1 | 0 | 0 | 0 | 0 | 0 | 0 | 0 | 0 | 0 | 0 | 0 | 0 | 0 | 0 | 0 | 0 | 0 | 0 | 0 | 0 | 0 | 0 | | | | | | |
| 1519 | 01729 | 01518 | 1 | | 0 | 0 | 1 | 0 | 0 | 0 | 0 | 0 | 0 | 0 | 0 | 0 | 0 | 0 | 0 | 0 | 0 | 0 | 0 | 0 | 0 | 0 | 0 | 0 | 0 | | | | | | |
| 1520 | 01730 | 01519 | 1 | | 0 | 0 | 1 | 0 | 0 | 0 | 0 | 0 | 0 | 0 | 0 | 0 | 0 | 0 | 0 | 0 | 0 | 0 | 0 | 0 | 0 | 0 | 0 | 0 | 0 | | | | | | |
| 1521 | 01731 | 01520 | 1 | | 0 | 0 | 1 | 0 | 0 | 0 | 0 | 0 | 0 | 0 | 0 | 0 | 0 | 0 | 0 | 0 | 0 | 0 | 0 | 0 | 0 | 0 | 0 | 0 | 0 | X12597 | 96.7 | 276 | 127 | 521 | 1009 |
| 1522 | 01732 | 01521 | 4 | | 0 | 0 | 1 | 1 | 0 | 0 | 0 | 0 | 0 | 1 | 1 | 0 | 0 | 0 | 0 | 0 | 0 | 0 | 0 | 0 | 0 | 0 | 0 | 0 | 0 | | | | | | |
| 1523 | 01733 | 01522 | 1 | | 0 | 0 | 1 | 0 | 0 | 0 | 0 | 0 | 0 | 0 | 0 | 0 | 0 | 0 | 0 | 0 | 0 | 0 | 0 | 0 | 0 | 0 | 0 | 0 | 0 | | | | | | |
| 1524 | 01734 | 01523 | 1 | | 0 | 0 | 1 | 0 | 0 | 0 | 0 | 0 | 0 | 0 | 0 | 0 | 0 | 0 | 0 | 0 | 0 | 0 | 0 | 0 | 0 | 0 | 0 | 0 | 0 | | | | | | |
| 1525 | 01735 | 01524 | 1 | | 0 | 0 | 1 | 0 | 0 | 0 | 0 | 0 | 0 | 0 | 0 | 0 | 0 | 0 | 0 | 0 | 0 | 0 | 0 | 0 | 0 | 0 | 0 | 0 | 0 | | | | | | |
| 1526 | 01736 | 01525 | 3 | | 0 | 0 | 1 | 0 | 0 | 0 | 0 | 0 | 0 | 0 | 0 | 0 | 0 | 0 | 0 | 0 | 0 | 0 | 0 | 0 | 2 | 0 | 0 | 0 | 0 | | | | | | |
| 1527 | 01737 | 01526 | 1 | | 0 | 0 | 1 | 0 | 0 | 0 | 0 | 0 | 0 | 0 | 0 | 0 | 0 | 0 | 0 | 0 | 0 | 0 | 0 | 0 | 0 | 0 | 0 | 0 | 0 | | | | | | |
| 1528 | 01738 | 01527 | 1 | | 0 | 0 | 1 | 0 | 0 | 0 | 0 | 0 | 0 | 0 | 0 | 0 | 0 | 0 | 0 | 0 | 0 | 0 | 0 | 0 | 0 | 0 | 0 | 0 | 0 | | | | | | |
| 1529 | 01739 | 01528 | 8 | | 0 | 0 | 1 | 0 | 0 | 0 | 0 | 0 | 0 | 0 | 0 | 1 | 0 | 3 | 0 | 0 | 0 | 0 | 2 | 1 | 0 | 0 | 0 | 0 | 0 | | | | | | |
| 1530 | 01740 | 01529 | 8 | | 0 | 0 | 3 | 0 | 1 | 0 | 0 | 0 | 0 | 0 | 0 | 1 | 0 | 0 | 0 | 0 | 0 | 0 | 0 | 0 | 2 | 0 | 1 | 0 | 0 | | | | | | |
| 1531 | 01741 | 01530 | 5 | | 0 | 0 | 2 | 0 | 0 | 0 | 0 | 0 | 0 | 0 | 0 | 0 | 0 | 0 | 0 | 0 | 0 | 0 | 0 | 0 | 0 | 1 | 2 | 0 | 0 | | | | | | |
| 1532 | 01742 | 01531 | 4 | | 0 | 0 | 1 | 0 | 0 | 0 | 0 | 0 | 0 | 0 | 0 | 0 | 0 | 2 | 1 | 0 | 0 | 0 | 0 | 0 | 0 | 0 | 0 | 0 | 0 | | | | | | |
| 1533 | 01745 | 01532 | 2 | | 0 | 0 | 1 | 0 | 0 | 0 | 0 | 0 | 0 | 0 | 1 | 0 | 0 | 0 | 0 | 0 | 0 | 0 | .0 | 0 | 0 | 0 | 0 | 0 | 0 | | | | | | |

Table 43

| | A | B | C | E | G | I | K | M | O | Q | S | U | W | Y | AA | AC | AE | AG | AI | AK | AM | AO | AQ | AS | AU | AW | AY | BA | BC | BE | BF | BG | BH | BI | BK |
|---|---|---|---|---|---|---|---|---|---|---|---|---|---|---|---|---|---|---|---|---|---|---|---|---|---|---|---|---|---|---|---|---|---|---|---|
| 1534 | 01746 | 01533 | 1 | | 0 | 0 | 1 | 0 | 0 | 0 | 0 | 0 | 0 | 0 | 0 | 0 | 0 | 0 | 0 | 0 | 0 | 0 | 0 | 0 | 0 | 0 | 0 | 0 | 0 | | | | | | |
| 1535 | 01747 | 01534 | 1 | | 0 | 0 | 1 | 0 | 0 | 0 | 0 | 0 | 0 | 0 | 0 | 0 | 0 | 0 | 0 | 0 | 0 | 0 | 0 | 0 | 0 | 0 | 0 | 0 | 0 | | | | | | |
| 1536 | 01748 | 01535 | 1 | | 0 | 0 | 1 | 0 | 0 | 0 | 0 | 0 | 0 | 0 | 0 | 0 | 0 | 0 | 0 | 0 | 0 | 0 | 0 | 0 | 0 | 0 | 0 | 0 | 0 | | | | | | |
| 1537 | 01749 | 01536 | 2 | | 0 | 0 | 1 | 0 | 0 | 0 | 0 | 0 | 0 | 0 | 0 | 0 | 0 | 0 | 0 | 0 | 0 | 0 | 0 | 0 | 0 | 0 | 0 | 1 | 0 | X73685 | 92.7 | 358 | 1 | 850 | 2177 |
| 1538 | 01750 | 01537 | 2 | | 0 | 0 | 2 | 0 | 0 | 0 | 0 | 0 | 0 | 0 | 0 | 0 | 0 | 0 | 0 | 0 | 0 | 0 | 0 | 0 | 0 | 0 | 0 | 0 | 0 | X16166 | 99.2 | 358 | 1 | 278 | 637 |
| 1539 | 01751 | 01538 | 1 | | 0 | 0 | 1 | 0 | 0 | 0 | 0 | 0 | 0 | 0 | 0 | 0 | 0 | 0 | 0 | 0 | 0 | 0 | 0 | 0 | 0 | 0 | 0 | 0 | 0 | | | | | | |
| 1540 | 01752 | 01539 | 4 | | 0 | 0 | 1 | 0 | 0 | 0 | 0 | 0 | 0 | 1 | 0 | 0 | 0 | 0 | 0 | 0 | 0 | 0 | 1 | 1 | 0 | 0 | 0 | 0 | 0 | | | | | | |
| 1541 | 01753 | 01540 | 1 | | 0 | 0 | 1 | 0 | 0 | 0 | 0 | 0 | 0 | 0 | 0 | 0 | 0 | 0 | 0 | 0 | 0 | 0 | 0 | 0 | 0 | 0 | 0 | 0 | 0 | | | | | | |
| 1542 | 01754 | 01541 | 2 | | 0 | 0 | 1 | 0 | 0 | 0 | 0 | 0 | 0 | 0 | 0 | 0 | 0 | 0 | 0 | 0 | 0 | 1 | 0 | 0 | 0 | 0 | 0 | 0 | 0 | | | | | | |
| 1543 | 01755 | 01542 | 2 | | 0 | 0 | 1 | 0 | 0 | 0 | 0 | 0 | 0 | 0 | 1 | 0 | 0 | 0 | 0 | 0 | 0 | 0 | 0 | 0 | 0 | 0 | 0 | 0 | 0 | | | | | | |
| 1544 | 01756 | 01543 | 3 | | 0 | 0 | 1 | 0 | 0 | 0 | 0 | 0 | 0 | 0 | 0 | 0 | 0 | 2 | 0 | 0 | 0 | 0 | 0 | 0 | 0 | 0 | 0 | 0 | 0 | | | | | | |
| 1545 | 01758 | 01544 | 1 | | 0 | 0 | 1 | 0 | 0 | 0 | 0 | 0 | 0 | 0 | 0 | 0 | 0 | 0 | 0 | 0 | 0 | 0 | 0 | 0 | 0 | 0 | 0 | 0 | 0 | | | | | | |
| 1546 | 01759 | 01545 | 7 | | 0 | 0 | 2 | 0 | 0 | 0 | 0 | 0 | 0 | 0 | 0 | 0 | 0 | 0 | 0 | 0 | 1 | 0 | 0 | 0 | 0 | 1 | 2 | 1 | 0 | | | | | | |
| 1547 | 01760 | 01546 | 3 | | 0 | 0 | 1 | 0 | 0 | 0 | 0 | 0 | 0 | 0 | 0 | 0 | 0 | 0 | 0 | 0 | 0 | 0 | 0 | 1 | 0 | 0 | 0 | 1 | 0 | | | | | | |
| 1548 | 01761 | 01547 | 1 | | 0 | 0 | 1 | 0 | 0 | 0 | 0 | 0 | 0 | 0 | 0 | 0 | 0 | 0 | 0 | 0 | 0 | 0 | 0 | 0 | 0 | 0 | 0 | 0 | 0 | | | | | | |
| 1549 | 01763 | 01548 | 1 | | 0 | 0 | 1 | 0 | 0 | 0 | 0 | 0 | 0 | 0 | 0 | 0 | 0 | 0 | 0 | 0 | 0 | 0 | 0 | 0 | 0 | 0 | 0 | 0 | 0 | | | | | | |
| 1550 | 01764 | 01549 | 1 | | 0 | 0 | 1 | 0 | 0 | 0 | 0 | 0 | 0 | 0 | 0 | 0 | 0 | 0 | 0 | 0 | 0 | 0 | 0 | 0 | 0 | 0 | 0 | 0 | 0 | | | | | | |
| 1551 | 01765 | 01550 | 7 | | 0 | 0 | 1 | 1 | 0 | 0 | 0 | 0 | 0 | 0 | 1 | 0 | 0 | 0 | 1 | 1 | 1 | 1 | 0 | 0 | 0 | 0 | 0 | 0 | 0 | M84739 | 94.7 | 337 | 1 | 1615 | 1958 |
| 1552 | 01766 | 01551 | 63 | | 0 | 0 | 6 | 15 | 2 | 4 | 2 | 0 | 4 | 0 | 5 | 1 | 1 | 0 | 6 | 1 | 10 | 3 | 0 | 0 | 1 | 0 | 1 | 0 | 1 | V00594 | 93.4 | 318 | 1 | 61 | 372 |
| 1553 | 01767 | 01552 | 3 | | 0 | 0 | 1 | 0 | 0 | 0 | 0 | 0 | 0 | 0 | 0 | 0 | 0 | 0 | 0 | 0 | 2 | 0 | 0 | 0 | 0 | 0 | 0 | 0 | 0 | | | | | | |
| 1554 | 01768 | 01553 | 3 | | 0 | 0 | 1 | 0 | 0 | 0 | 0 | 0 | 0 | 0 | 0 | 0 | 0 | 0 | 0 | 1 | 0 | 0 | 0 | 0 | 0 | 1 | 0 | 0 | 0 | | | | | | |
| 1555 | 01769 | 01554 | 1 | | 0 | 0 | 1 | 0 | 0 | 0 | 0 | 0 | 0 | 0 | 0 | 0 | 0 | 0 | 0 | 0 | 0 | 0 | 0 | 0 | 0 | 0 | 0 | 0 | 0 | | | | | | |
| 1556 | 01770 | 01555 | 5 | | 0 | 0 | 1 | 1 | 0 | 0 | 0 | 0 | 1 | 0 | 0 | 0 | 0 | 0 | 0 | 0 | 1 | 1 | 0 | 0 | 0 | 0 | 0 | 0 | 0 | M64098 | 97.9 | 385 | 1 | 3963 | 4354 |
| 1557 | 01771 | 01556 | 1 | | 0 | 0 | 1 | 0 | 0 | 0 | 0 | 0 | 0 | 0 | 0 | 0 | 0 | 0 | 0 | 0 | 0 | 0 | 0 | 0 | 0 | 0 | 0 | 0 | 0 | | | | | | |
| 1558 | 01772 | 01557 | 2 | | 0 | 0 | 1 | 0 | 0 | 0 | 0 | 0 | 0 | 0 | 0 | 0 | 0 | 0 | 0 | 0 | 0 | 0 | 0 | 0 | 0 | 0 | 1 | 0 | 0 | M63838 | 96.6 | 322 | 1 | 2348 | 2709 |
| 1559 | 01773 | 01558 | 2 | | 0 | 0 | 1 | 0 | 0 | 0 | 0 | 0 | 0 | 0 | 0 | 0 | 0 | 0 | 0 | 0 | 0 | 0 | 0 | 0 | 1 | 0 | 0 | 0 | 0 | M16985 | 97.4 | 309 | 1 | 2120 | 2428 |
| 1560 | 01774 | 01559 | 1 | | 0 | 0 | 1 | 0 | 0 | 0 | 0 | 0 | 0 | 0 | 0 | 0 | 0 | 0 | 0 | 0 | 0 | 0 | 0 | 0 | 0 | 0 | 0 | 0 | 0 | M29863 | 91.9 | 185 | 138 | 906 | 1098 |
| 1561 | 01775 | 01560 | 2 | | 0 | 0 | 1 | 0 | 0 | 0 | 0 | 0 | 0 | 0 | 1 | 0 | 0 | 0 | 0 | 0 | 0 | 0 | 0 | 0 | 0 | 0 | 0 | 0 | 0 | D11094 | 96.3 | 374 | 1 | 1105 | 1478 |
| 1562 | 01776 | 01561 | 2 | | 0 | 0 | 1 | 0 | 0 | 0 | 0 | 0 | 1 | 0 | 0 | 0 | 0 | 0 | 0 | 0 | 0 | 0 | 0 | 0 | 0 | 0 | 0 | 0 | 0 | | | | | | |
| 1563 | 01777 | 01562 | 3 | | 0 | 0 | 1 | 0 | 0 | 0 | 0 | 0 | 0 | 0 | 0 | 0 | 1 | 0 | 0 | 0 | 0 | 0 | 0 | 1 | 0 | 0 | 0 | 0 | 0 | | | | | | |
| 1564 | 01778 | 01563 | 2 | | 0 | 0 | 2 | 0 | 0 | 0 | 0 | 0 | 0 | 0 | 0 | 0 | 0 | 0 | 0 | 0 | 0 | 0 | 0 | 0 | 0 | 0 | 0 | 0 | 0 | | | | | | |
| 1565 | 01779 | 01564 | 1 | | 0 | 0 | 1 | 0 | 0 | 0 | 0 | 0 | 0 | 0 | 0 | 0 | 0 | 0 | 0 | 0 | 0 | 0 | 0 | 0 | 0 | 0 | 0 | 0 | 0 | | | | | | |
| 1566 | 01780 | 01565 | 1 | | 0 | 0 | 1 | 0 | 0 | 0 | 0 | 0 | 0 | 0 | 0 | 0 | 0 | 0 | 0 | 0 | 0 | 0 | 0 | 0 | 0 | 0 | 0 | 0 | 0 | | | | | | |
| 1567 | 01781 | 01566 | 1 | | 0 | 0 | 1 | 0 | 0 | 0 | 0 | 0 | 0 | 0 | 0 | 0 | 0 | 0 | 0 | 0 | 0 | 0 | 0 | 0 | 0 | 0 | 0 | 0 | 0 | | | | | | |
| 1568 | 01782 | 01567 | 1 | | 0 | 0 | 1 | 0 | 0 | 0 | 0 | 0 | 0 | 0 | 0 | 0 | 0 | 0 | 0 | 0 | 0 | 0 | 0 | 0 | 0 | 0 | 0 | 0 | 0 | | | | | | |
| 1569 | 01783 | 01568 | 3 | | 0 | 0 | 3 | 0 | 0 | 0 | 0 | 0 | 0 | 0 | 0 | 0 | 0 | 0 | 0 | 0 | 0 | 0 | 0 | 0 | 0 | 0 | 0 | 0 | 0 | | | | | | |

Table 44

| | A | B | C | E | G | I | K | M | O | Q | S | U | W | Y | AA | AC | AE | AG | AI | AK | AM | AO | AQ | AS | AU | AW | AY | BA | BC | BE | BF | BG | BH | BI | BK |
|---|---|---|---|---|---|---|---|---|---|---|---|---|---|---|---|---|---|---|---|---|---|---|---|---|---|---|---|---|---|---|---|---|---|---|---|
| 1570 | 01784 | 01569 | 2 | | 0 | 0 | 1 | 0 | 0 | 0 | 0 | 0 | 1 | 0 | 0 | 0 | 0 | 0 | 0 | 0 | 0 | 0 | 0 | 0 | 0 | 0 | 0 | 0 | 0 | | | | | | |
| 1571 | 01785 | 01570 | 1 | | 0 | 0 | 1 | 0 | 0 | 0 | 0 | 0 | 0 | 0 | 0 | 0 | 0 | 0 | 0 | 0 | 0 | 0 | 0 | 0 | 0 | 0 | 0 | 0 | 0 | L10320 | 99 | 301 | 1 | 1101 | 1401 |
| 1572 | 01786 | 01571 | 8 | | 0 | 0 | 2 | 0 | 0 | 0 | 3 | 0 | 0 | 0 | 0 | 0 | 1 | 1 | 0 | 1 | 0 | 0 | 0 | 0 | 0 | 0 | 0 | 0 | 0 | | | | | | |
| 1573 | 01787 | 01572 | 1 | | 0 | 0 | 1 | 0 | 0 | 0 | 0 | 0 | 0 | 0 | 0 | 0 | 0 | 0 | 0 | 0 | 0 | 0 | 0 | 0 | 0 | 0 | 0 | 0 | 0 | | | | | | |
| 1574 | 01788 | 01573 | 3 | | 0 | 0 | 1 | 0 | 0 | 1 | 0 | 0 | 0 | 0 | 0 | 0 | 0 | 0 | 0 | 0 | 0 | 0 | 0 | 1 | 0 | 0 | 0 | 0 | 0 | | | | | | |
| 1575 | 01789 | 01574 | 1 | | 0 | 0 | 1 | 0 | 0 | 0 | 0 | 0 | 0 | 0 | 0 | 0 | 0 | 0 | 0 | 0 | 0 | 0 | 0 | 0 | 0 | 0 | 0 | 0 | 0 | | | | | | |
| 1576 | 01790 | 01575 | 3 | | 0 | 0 | 1 | 0 | 0 | 0 | 0 | 0 | 0 | 0 | 0 | 0 | 0 | 1 | 0 | 0 | 0 | 0 | 0 | 0 | 0 | 1 | 0 | 0 | 0 | L03411 | 95.9 | 295 | 1 | 1011 | 1301 |
| 1577 | 01791 | 01576 | 34 | | 0 | 0 | 1 | 0 | 0 | 2 | 3 | 1 | 3 | 0 | 0 | 5 | 0 | 2 | 0 | 2 | 3 | 3 | 4 | 1 | 3 | 1 | 0 | 0 | 0 | | | | | | |
| 1578 | 01792 | 01577 | 1 | | 0 | 0 | 1 | 0 | 0 | 0 | 0 | 0 | 0 | 0 | 0 | 0 | 0 | 0 | 0 | 0 | 0 | 0 | 0 | 0 | 0 | 0 | 0 | 0 | 0 | | | | | | |
| 1579 | 01793 | 01578 | 6 | | 0 | 0 | 1 | 0 | 1 | 0 | 0 | 0 | 0 | 0 | 0 | 0 | 0 | 1 | 1 | 1 | 0 | 0 | 0 | 0 | 0 | 0 | 0 | 1 | 0 | | | | | | |
| 1580 | 01794 | 01579 | 6 | | 0 | 0 | 1 | 0 | 0 | 0 | 0 | 0 | 0 | 0 | 3 | 0 | 0 | 0 | 1 | 0 | 0 | 0 | 0 | 1 | 0 | 0 | 0 | 0 | 0 | | | | | | |
| 1581 | 01795 | 01580 | 1 | | 0 | 0 | 1 | 0 | 0 | 0 | 0 | 0 | 0 | 0 | 0 | 0 | 0 | 0 | 0 | 0 | 0 | 0 | 0 | 0 | 0 | 0 | 0 | 0 | 0 | | | | | | |
| 1582 | 01796 | 01581 | 7 | | 0 | 0 | 4 | 0 | 0 | 0 | 0 | 0 | 0 | 0 | 0 | 0 | 0 | 0 | 2 | 0 | 0 | 1 | 0 | 0 | 0 | 0 | 0 | 0 | 0 | L20859 | 99.3 | 285 | 1 | 2924 | 3220 |
| 1583 | 01797 | 01582 | 1 | | 0 | 0 | 1 | 0 | 0 | 0 | 0 | 0 | 0 | 0 | 0 | 0 | 0 | 0 | 0 | 0 | 0 | 0 | 0 | 0 | 0 | 0 | 0 | 0 | 0 | | | | | | |
| 1584 | 01798 | 01583 | 1 | | 0 | 0 | 1 | 0 | 0 | 0 | 0 | 0 | 0 | 0 | 0 | 0 | 0 | 0 | 0 | 0 | 0 | 0 | 0 | 0 | 0 | 0 | 0 | 0 | 0 | | | | | | |
| 1585 | 01799 | 01584 | 2 | | 0 | 0 | 1 | 0 | 0 | 0 | 0 | 0 | 0 | 0 | 0 | 0 | 0 | 0 | 0 | 0 | 0 | 0 | 0 | 0 | 0 | 0 | 0 | 1 | 0 | | | | | | |
| 1586 | 01800 | 01585 | 2 | | 0 | 0 | 1 | 0 | 0 | 0 | 0 | 0 | 0 | 0 | 0 | 0 | 0 | 0 | 0 | 0 | 0 | 0 | 1 | 0 | 0 | 0 | 0 | 0 | 0 | | | | | | |
| 1587 | 01801 | 01586 | 2 | | 0 | 0 | 1 | 0 | 0 | 0 | 0 | 0 | 0 | 0 | 0 | 0 | 0 | 0 | 0 | 0 | 0 | 0 | 0 | 1 | 0 | 0 | 0 | 0 | 0 | | | | | | |
| 1588 | 01802 | 01587 | 1 | | 0 | 0 | 1 | 0 | 0 | 0 | 0 | 0 | 0 | 0 | 0 | 0 | 0 | 0 | 0 | 0 | 0 | 0 | 0 | 0 | 0 | 0 | 0 | 0 | 0 | | | | | | |
| 1589 | 01803 | 01588 | 4 | | 0 | 0 | 1 | 0 | 0 | 0 | 0 | 0 | 0 | 0 | 0 | 0 | 0 | 0 | 0 | 2 | 0 | 0 | 0 | 0 | 0 | 0 | 1 | 0 | 0 | | | | | | |
| 1590 | 01804 | 01589 | 1 | | 0 | 0 | 1 | 0 | 0 | 0 | 0 | 0 | 0 | 0 | 0 | 0 | 0 | 0 | 0 | 0 | 0 | 0 | 0 | 0 | 0 | 0 | 0 | 0 | 0 | | | | | | |
| 1591 | 01805 | 01590 | 1 | | 0 | 0 | 1 | 0 | 0 | 0 | 0 | 0 | 0 | 0 | 0 | 0 | 0 | 0 | 0 | 0 | 0 | 0 | 0 | 0 | 0 | 0 | 0 | 0 | 0 | | | | | | |
| 1592 | 01806 | 01591 | 26 | | 0 | 0 | 7 | 0 | 0 | 0 | 0 | 1 | 2 | 1 | 0 | 0 | 0 | 0 | 1 | 2 | 0 | 1 | 0 | 0 | 0 | 0 | 0 | 0 | 11 | X05908 | 95.8 | 262 | 1 | 1142 | 1399 |
| 1593 | 01807 | 01592 | 1 | | 0 | 0 | 1 | 0 | 0 | 0 | 0 | 0 | 0 | 0 | 0 | 0 | 0 | 0 | 0 | 0 | 0 | 0 | 0 | 0 | 0 | 0 | 0 | 0 | 0 | | | | | | |
| 1594 | 01808 | 01593 | 47 | | 0 | 0 | 6 | 0 | 2 | 1 | 1 | 3 | 7 | 4 | 2 | 4 | 0 | 0 | 0 | 0 | 6 | 3 | 2 | 0 | 0 | 6 | 0 | 0 | 0 | S54005 | 98 | 253 | 1 | 201 | 453 |
| 1595 | 01809 | 01594 | 19 | | 0 | 0 | 3 | 0 | 0 | 0 | 0 | 0 | 1 | 2 | 6 | 0 | 0 | 0 | 0 | 0 | 0 | 0 | 0 | 0 | 0 | 1 | 5 | 1 | 0 | M58485 | 96.1 | 256 | 1 | 610 | 875 |
| 1596 | 01810 | 01595 | 2 | | 0 | 0 | 1 | 0 | 0 | 0 | 0 | 0 | 0 | 1 | 0 | 0 | 0 | 0 | 0 | 0 | 0 | 0 | 0 | 0 | 0 | 0 | 0 | 0 | 0 | | | | | | |
| 1597 | 01811 | 01596 | 39 | | 0 | 0 | 1 | 0 | 0 | 0 | 0 | 0 | 0 | 0 | 2 | 2 | 1 | 0 | 2 | 0 | 0 | 0 | 0 | 0 | 0 | 0 | 10 | 17 | 4 | M11353 | 98 | 204 | 1 | 618 | 1305 |
| 1598 | 01812 | 01597 | 2 | | 0 | 0 | 1 | 0 | 0 | 0 | 0 | 0 | 0 | 0 | 0 | 0 | 0 | 0 | 0 | 0 | 1 | 0 | 0 | 0 | 0 | 0 | 0 | 0 | 0 | | | | | | |
| 1599 | 01813 | 01598 | 3 | | 0 | 0 | 1 | 0 | 0 | 0 | 0 | 0 | 0 | 0 | 0 | 0 | 0 | 0 | 0 | 0 | 0 | 1 | 0 | 0 | 0 | 0 | 1 | 0 | 0 | | | | | | |
| 1600 | 01814 | 01599 | 1 | | 0 | 0 | 1 | 0 | 0 | 0 | 0 | 0 | 0 | 0 | 0 | 0 | 0 | 0 | 0 | 0 | 0 | 0 | 0 | 0 | 0 | 0 | 0 | 0 | 0 | M69238 | 96.8 | 219 | 1 | 2394 | 2616 |
| 1601 | 01815 | 01600 | 1 | | 0 | 0 | 1 | 0 | 0 | 0 | 0 | 0 | 0 | 0 | 0 | 0 | 0 | 0 | 0 | 0 | 0 | 0 | 0 | 0 | 0 | 0 | 0 | 0 | 0 | | | | | | |
| 1602 | 01816 | 01601 | 1 | | 0 | 0 | 1 | 0 | 0 | 0 | 0 | 0 | 0 | 0 | 0 | 0 | 0 | 0 | 0 | 0 | 0 | 0 | 0 | 0 | 0 | 0 | 0 | 0 | 0 | | | | | | |
| 1603 | 01817 | 01602 | 1 | | 0 | 0 | 1 | 0 | 0 | 0 | 0 | 0 | 0 | 0 | 0 | 0 | 0 | 0 | 0 | 0 | 0 | 0 | 0 | 0 | 0 | 0 | 0 | 0 | 0 | M77140 | 97 | 233 | 1 | 280 | 512 |
| 1604 | 01818 | 01603 | 3 | | 0 | 0 | 1 | 0 | 0 | 0 | 1 | 0 | 1 | 0 | 0 | 0 | 0 | 0 | 0 | 0 | 0 | 0 | 0 | 0 | 0 | 0 | 0 | 0 | 0 | M65212 | 96.9 | 322 | 1 | 741 | 1107 |
| 1605 | 01819 | 01604 | 6 | | 0 | 0 | 1 | 0 | 0 | 0 | 0 | 0 | 0 | 2 | 0 | 1 | 0 | 0 | 0 | 0 | 0 | 0 | 0 | 1 | 0 | 1 | 0 | 0 | 0 | | | | | | |

Table 45

| | A | B | C | E | G | I | K | M | O | Q | S | U | W | Y | AA | AC | AF | AG | AI | AK | AM | AO | AQ | AS | AU | AW | AY | BA | BC | BE | BF | BG | BH | BI | BK |
|---|---|---|---|---|---|---|---|---|---|---|---|---|---|---|---|---|---|---|---|---|---|---|---|---|---|---|---|---|---|---|---|---|---|---|---|
| 1606 | 01820 | 01605 | 9 | | 0 | 0 | 1 | 0 | 0 | 0 | 0 | 0 | 1 | 0 | 0 | 1 | 0 | 3 | 0 | 0 | 0 | 0 | 0 | 0 | 0 | 0 | 2 | 1 | 0 | | | | | | |
| 1607 | 01821 | 01606 | 1 | | 0 | 0 | 1 | 0 | 0 | 0 | 0 | 0 | 0 | 0 | 0 | 0 | 0 | 0 | 0 | 0 | 0 | 0 | 0 | 0 | 0 | 0 | 0 | 0 | 0 | | | | | | |
| 1608 | 01822 | 01607 | 7 | | 0 | 0 | 2 | 0 | 0 | 0 | 0 | 0 | 3 | 2 | 0 | 0 | 0 | 0 | 0 | 0 | 0 | 0 | 0 | 0 | 0 | 0 | 0 | 0 | 0 | X06256 | 100 | 234 | 1 | 3965 | 4204 |
| 1609 | 01823 | 01608 | 24 | | 0 | 0 | 2 | 0 | 0 | 0 | 0 | 0 | 0 | 0 | 0 | 0 | 0 | 0 | 1 | 1 | 0 | 0 | 0 | 0 | 0 | 0 | 20 | 0 | 0 | U02020 | 98.6 | 353 | 1 | 1805 | 2376 |
| 1610 | 01824 | 01609 | 1 | | 0 | 0 | 1 | 0 | 0 | 0 | 0 | 0 | 0 | 0 | 0 | 0 | 0 | 0 | 0 | 0 | 0 | 0 | 0 | 0 | 0 | 0 | 0 | 0 | 0 | M15841 | 99.6 | 228 | 1 | 782 | 1015 |
| 1611 | 01825 | 01610 | 3 | | 0 | 0 | 1 | 0 | 0 | 0 | 0 | 0 | 0 | 0 | 1 | 0 | 0 | 0 | 0 | 0 | 0 | 0 | 0 | 1 | 0 | 0 | 0 | 0 | 0 | | | | | | |
| 1612 | 01826 | 01611 | 6 | | 0 | 0 | 1 | 0 | 0 | 0 | 0 | 0 | 0 | 0 | 0 | 0 | 0 | 1 | 0 | 0 | 0 | 0 | 0 | 0 | 0 | 0 | 4 | 0 | 0 | | | | | | |
| 1613 | 01827 | 01612 | 1 | | 0 | 0 | 1 | 0 | 0 | 0 | 0 | 0 | 0 | 0 | 0 | 0 | 0 | 0 | 0 | 0 | 0 | 0 | 0 | 0 | 0 | 0 | 0 | 0 | 0 | | | | | | |
| 1614 | 01828 | 01613 | 1 | | 0 | 0 | 1 | 0 | 0 | 0 | 0 | 0 | 0 | 0 | 0 | 0 | 0 | 0 | 0 | 0 | 0 | 0 | 0 | 0 | 0 | 0 | 0 | 0 | 0 | | | | | | |
| 1615 | 01829 | 01614 | 1 | | 0 | 0 | 1 | 0 | 0 | 0 | 0 | 0 | 0 | 0 | 0 | 0 | 0 | 0 | 0 | 0 | 0 | 0 | 0 | 0 | 0 | 0 | 0 | 0 | 0 | | | | | | |
| 1616 | 01830 | 01615 | 3 | | 0 | 0 | 1 | 0 | 0 | 0 | 0 | 0 | 0 | 0 | 0 | 0 | 0 | 0 | 1 | 0 | 0 | 0 | 0 | 0 | 0 | 0 | 0 | 1 | 0 | X76302 | 98 | 202 | 1 | 1201 | 1402 |
| 1617 | 01831 | 01616 | 3 | | 0 | 0 | 1 | 0 | 0 | 0 | 0 | 0 | 1 | 0 | 0 | 0 | 0 | 0 | 0 | 0 | 1 | 0 | 0 | 0 | 0 | 0 | 0 | 0 | 0 | | | | | | |
| 1618 | 01832 | 01617 | 8 | | 0 | 0 | 1 | 0 | 0 | 0 | 2 | 0 | 0 | 0 | 1 | 0 | 0 | 0 | 1 | 0 | 1 | 0 | 0 | 0 | 1 | 0 | 1 | 0 | 0 | X51345 | 96.8 | 219 | 1 | 1580 | 1797 |
| 1619 | 01833 | 01618 | 1 | | 0 | 0 | 1 | 0 | 0 | 0 | 0 | 0 | 0 | 0 | 0 | 0 | 0 | 0 | 0 | 0 | 0 | 0 | 0 | 0 | 0 | 0 | 0 | 0 | 0 | X14813 | 93.4 | 213 | 1 | 1432 | 1642 |
| 1620 | 01834 | 01619 | 1 | | 0 | 0 | 1 | 0 | 0 | 0 | 0 | 0 | 0 | 0 | 0 | 0 | 0 | 0 | 0 | 0 | 0 | 0 | 0 | 0 | 0 | 0 | 0 | 0 | 0 | | | | | | |
| 1621 | 01835 | 01620 | 5 | | 0 | 0 | 2 | 0 | 0 | 0 | 0 | 0 | 0 | 0 | 0 | 0 | 0 | 2 | 0 | 0 | 0 | 0 | 0 | 0 | 0 | 0 | 0 | 1 | 0 | | | | | | |
| 1622 | 01836 | 01621 | 1 | | 0 | 0 | 1 | 0 | 0 | 0 | 0 | 0 | 0 | 0 | 0 | 0 | 0 | 0 | 0 | 0 | 0 | 0 | 0 | 0 | 0 | 0 | 0 | 0 | 0 | | | | | | |
| 1623 | 01837 | 01622 | 1 | | 0 | 0 | 1 | 0 | 0 | 0 | 0 | 0 | 0 | 0 | 0 | 0 | 0 | 0 | 0 | 0 | 0 | 0 | 0 | 0 | 0 | 0 | 0 | 0 | 0 | | | | | | |
| 1624 | 01838 | 01623 | 2 | | 0 | 0 | 1 | 0 | 0 | 0 | 0 | 0 | 0 | 0 | 0 | 1 | 0 | 0 | 0 | 0 | 0 | 0 | 0 | 0 | 0 | 0 | 0 | 0 | 0 | | | | | | |
| 1625 | 01839 | 01624 | 2 | | 0 | 0 | 2 | 0 | 0 | 0 | 0 | 0 | 0 | 0 | 0 | 0 | 0 | 0 | 0 | 0 | 0 | 0 | 0 | 0 | 0 | 0 | 0 | 0 | 0 | | | | | | |
| 1626 | 01840 | 01625 | 3 | | 0 | 0 | 1 | 0 | 0 | 0 | 0 | 0 | 0 | 1 | 0 | 0 | 0 | 0 | 0 | 0 | 0 | 1 | 0 | 0 | 0 | 0 | 0 | 0 | 0 | | | | | | |
| 1627 | 01841 | 01626 | 2 | | 0 | 0 | 1 | 0 | 0 | 0 | 0 | 0 | 0 | 0 | 0 | 1 | 0 | 0 | 0 | 0 | 0 | 0 | 0 | 0 | 0 | 0 | 0 | 0 | 0 | | | | | | |
| 1628 | 01842 | 01627 | 1 | | 0 | 0 | 1 | 0 | 0 | 0 | 0 | 0 | 0 | 0 | 0 | 0 | 0 | 0 | 0 | 0 | 0 | 0 | 0 | 0 | 0 | 0 | 0 | 0 | 0 | | | | | | |
| 1629 | 01843 | 01628 | 1 | | 0 | 0 | 1 | 0 | 0 | 0 | 0 | 0 | 0 | 0 | 0 | 0 | 0 | 0 | 0 | 0 | 0 | 0 | 0 | 0 | 0 | 0 | 0 | 0 | 0 | | | | | | |
| 1630 | 01844 | 01629 | 1 | | 0 | 0 | 1 | 0 | 0 | 0 | 0 | 0 | 0 | 0 | 0 | 0 | 0 | 0 | 0 | 0 | 0 | 0 | 0 | 0 | 0 | 0 | 0 | 0 | 0 | | | | | | |
| 1631 | 01845 | 01630 | 1 | | 0 | 0 | 1 | 0 | 0 | 0 | 0 | 0 | 0 | 0 | 0 | 0 | 0 | 0 | 0 | 0 | 0 | 0 | 0 | 0 | 0 | 0 | 0 | 0 | 0 | | | | | | |
| 1632 | 01846 | 01631 | 2 | | 0 | 0 | 1 | 0 | 0 | 0 | 1 | 0 | 0 | 0 | 0 | 0 | 0 | 0 | 0 | 0 | 0 | 0 | 0 | 0 | 0 | 0 | 0 | 0 | 0 | | | | | | |
| 1633 | 01847 | 01632 | 2 | | 0 | 0 | 1 | 0 | 0 | 0 | 0 | 0 | 0 | 0 | 0 | 0 | 0 | 0 | 0 | 0 | 1 | 0 | 0 | 0 | 0 | 0 | 0 | 0 | 0 | | | | | | |
| 1634 | 01848 | 01633 | 1 | | 0 | 0 | 1 | 0 | 0 | 0 | 0 | 0 | 0 | 0 | 0 | 0 | 0 | 0 | 0 | 0 | 0 | 0 | 0 | 0 | 0 | 0 | 0 | 0 | 0 | | | | | | |
| 1635 | 01849 | 01634 | 2 | | 0 | 0 | 1 | 0 | 0 | 0 | 0 | 0 | 0 | 0 | 0 | 1 | 0 | 0 | 0 | 0 | 0 | 0 | 0 | 0 | 0 | 0 | 0 | 0 | 0 | | | | | | |
| 1636 | 01850 | 01635 | 4 | | 0 | 0 | 1 | 0 | 0 | 0 | 0 | 0 | 1 | 0 | 0 | 1 | 1 | 0 | 0 | 0 | 0 | 0 | 0 | 0 | 0 | 0 | 0 | 0 | 0 | | | | | | |
| 1637 | 01851 | 01636 | 1 | | 0 | 0 | 1 | 0 | 0 | 0 | 0 | 0 | 0 | 0 | 0 | 0 | 0 | 0 | 0 | 0 | 0 | 0 | 0 | 0 | 0 | 0 | 0 | 0 | 0 | | | | | | |
| 1638 | 01852 | 01637 | 1 | | 0 | 0 | 1 | 0 | 0 | 0 | 0 | 0 | 0 | 0 | 0 | 0 | 0 | 0 | 0 | 0 | 0 | 0 | 0 | 0 | 0 | 0 | 0 | 0 | 0 | | | | | | |
| 1639 | 01853 | 01638 | 1 | | 0 | 0 | 1 | 0 | 0 | 0 | 0 | 0 | 0 | 0 | 0 | 0 | 0 | 0 | 0 | 0 | 0 | 0 | 0 | 0 | 0 | 0 | 0 | 0 | 0 | | | | | | |
| 1640 | 01854 | 01639 | 1 | | 0 | 0 | 1 | 0 | 0 | 0 | 0 | 0 | 0 | 0 | 0 | 0 | 0 | 0 | 0 | 0 | 0 | 0 | 0 | 0 | 0 | 0 | 0 | 0 | 0 | | | | | | |
| 1641 | 01855 | 01640 | 7 | | 0 | 0 | 1 | 0 | 0 | 0 | 2 | 0 | 0 | 1 | 0 | 0 | 1 | 0 | 2 | 0 | 0 | 0 | 0 | 0 | 0 | 0 | 0 | 0 | 0 | | | | | | |

Table 46

| | A | B | C | E | G | I | K | M | O | Q | S | U | W | Y | AA | AC | AE | AG | AI | AK | AM | AO | AQ | AS | AU | AW | AY | BA | BC | BE | BF | BG | BH | BI | BK |
|---|---|---|---|---|---|---|---|---|---|---|---|---|---|---|---|---|---|---|---|---|---|---|---|---|---|---|---|---|---|---|---|---|---|---|---|
| 1642 | 01856 | 01641 | 5 | | 0 | 0 | 1 | 0 | 2 | 0 | 0 | 0 | 0 | 0 | 0 | 0 | 0 | 0 | 0 | 1 | 0 | 0 | 0 | 0 | 0 | 0 | 1 | 0 | 0 | | | | | | |
| 1643 | 01857 | 01642 | 6 | | 0 | 0 | 1 | 0 | 0 | 0 | 0 | 0 | 0 | 0 | 0 | 0 | 0 | 1 | 0 | 0 | 0 | 0 | 2 | 0 | 1 | 1 | 0 | 0 | 0 | M33146 | 100 | 186 | 6 | 1612 | 1797 |
| 1644 | 01858 | 01643 | 1 | | 0 | 0 | 1 | 0 | 0 | 0 | 0 | 0 | 0 | 0 | 0 | 0 | 0 | 0 | 0 | 0 | 0 | 0 | 0 | 0 | 0 | 0 | 0 | 0 | 0 | | | | | | |
| 1645 | 01859 | 01644 | 2 | | 0 | 0 | 1 | 0 | 0 | 0 | 0 | 0 | 0 | 1 | 0 | 0 | 0 | 0 | 0 | 0 | 0 | 0 | 0 | 0 | 0 | 0 | 0 | 0 | 0 | | | | | | |
| 1646 | 01860 | 01645 | 4 | | 0 | 0 | 1 | 0 | 1 | 0 | 0 | 0 | 0 | 0 | 0 | 0 | 0 | 0 | 1 | 0 | 0 | 0 | 0 | 0 | 0 | 0 | 0 | 1 | 0 | | | | | | |
| 1647 | 01861 | 01646 | 18 | | 0 | 0 | 1 | 0 | 0 | 0 | 1 | 1 | 1 | 0 | 1 | 2 | 0 | 1 | 2 | 0 | 2 | 1 | 0 | 1 | 0 | 0 | 0 | 4 | 0 | | | | | | |
| 1648 | 01862 | 01647 | 1 | | 0 | 0 | 1 | 0 | 0 | 0 | 0 | 0 | 0 | 0 | 0 | 0 | 0 | 0 | 0 | 0 | 0 | 0 | 0 | 0 | 0 | 0 | 0 | 0 | 0 | | | | | | |
| 1649 | 01863 | 01648 | 18 | | 0 | 0 | 1 | 0 | 0 | 0 | 0 | 1 | 1 | 2 | 2 | 2 | 1 | 0 | 1 | 0 | 1 | 2 | 0 | 0 | 0 | 2 | 1 | 1 | 0 | | | | | | |
| 1650 | 01864 | 01649 | 2 | | 0 | 0 | 1 | 0 | 0 | 0 | 0 | 0 | 1 | 0 | 0 | 0 | 0 | 0 | 0 | 0 | 0 | 0 | 0 | 0 | 0 | 0 | 0 | 0 | 0 | | | | | | |
| 1651 | 01865 | 01650 | 2 | | 0 | 0 | 2 | 0 | 0 | 0 | 0 | 0 | 0 | 0 | 0 | 0 | 0 | 0 | 0 | 0 | 0 | 0 | 0 | 0 | 0 | 0 | 0 | 0 | 0 | | | | | | |
| 1652 | 01866 | 01651 | 2 | | 0 | 0 | 1 | 0 | 0 | 0 | 0 | 0 | 0 | 0 | 1 | 0 | 0 | 0 | 0 | 0 | 0 | 0 | 0 | 0 | 0 | 0 | 0 | 0 | 0 | | | | | | |
| 1653 | 01867 | 01652 | 7 | | 0 | 0 | 1 | 0 | 0 | 0 | 0 | 0 | 0 | 0 | 0 | 0 | 0 | 0 | 0 | 2 | 1 | 0 | 0 | 0 | 0 | 0 | 0 | 2 | 1 | | | | | | |
| 1654 | 01868 | 01653 | 1 | | 0 | 0 | 1 | 0 | 0 | 0 | 0 | 0 | 0 | 0 | 0 | 0 | 0 | 0 | 0 | 0 | 0 | 0 | 0 | 0 | 0 | 0 | 0 | 0 | 0 | | | | | | |
| 1655 | 01869 | 01654 | 1 | | 0 | 0 | 1 | 0 | 0 | 0 | 0 | 0 | 0 | 0 | 0 | 0 | 0 | 0 | 0 | 0 | 0 | 0 | 0 | 0 | 0 | 0 | 0 | 0 | 0 | | | | | | |
| 1656 | 01870 | 01655 | 2 | | 0 | 0 | 1 | 0 | 0 | 0 | 0 | 0 | 1 | 0 | 0 | 0 | 0 | 0 | 0 | 0 | 0 | 0 | 0 | 0 | 0 | 0 | 0 | 0 | 0 | | | | | | |
| 1657 | 01871 | 01656 | 6 | | 0 | 0 | 4 | 0 | 0 | 0 | 0 | 0 | 0 | 0 | 0 | 1 | 0 | 0 | 0 | 0 | 0 | 0 | 0 | 0 | 0 | 1 | 0 | 0 | 0 | | | | | | |
| 1658 | 01872 | 01657 | 1 | | 0 | 0 | 1 | 0 | 0 | 0 | 0 | 0 | 0 | 0 | 0 | 0 | 0 | 0 | 0 | 0 | 0 | 0 | 0 | 0 | 0 | 0 | 0 | 0 | 0 | M13932 | 94 | 167 | 1 | 136 | 477 |
| 1659 | 01873 | 01658 | 1 | | 0 | 0 | 1 | 0 | 0 | 0 | 0 | 0 | 0 | 0 | 0 | 0 | 0 | 0 | 0 | 0 | 0 | 0 | 0 | 0 | 0 | 0 | 0 | 0 | 0 | | | | | | |
| 1660 | 01874 | 01659 | 2 | | 0 | 0 | 1 | 0 | 0 | 0 | 0 | 0 | 0 | 0 | 0 | 0 | 0 | 0 | 0 | 0 | 0 | 0 | 0 | 1 | 0 | 0 | 0 | 0 | 0 | | | | | | |
| 1661 | 01875 | 01660 | 3 | | 0 | 0 | 1 | 0 | 0 | 0 | 0 | 0 | 0 | 0 | 0 | 0 | 0 | 0 | 1 | 0 | 0 | 0 | 0 | 0 | 1 | 0 | 0 | 0 | 0 | | | | | | |
| 1662 | 01876 | 01661 | 11 | | 0 | 0 | 1 | 0 | 0 | 0 | 1 | 0 | 0 | 0 | 0 | 1 | 0 | 1 | 0 | 1 | 2 | 1 | 0 | 1 | 0 | 0 | 1 | 0 | 1 | | | | | | |
| 1663 | 01877 | 01662 | 8 | | 0 | 0 | 1 | 0 | 0 | 0 | 1 | 0 | 1 | 0 | 0 | 0 | 0 | 0 | 1 | 0 | 1 | 0 | 0 | 0 | 0 | 0 | 3 | 0 | 0 | | | | | | |
| 1664 | 01878 | 01663 | 2 | | 0 | 0 | 1 | 0 | 0 | 0 | 0 | 0 | 0 | 0 | 1 | 0 | 0 | 0 | 0 | 0 | 0 | 0 | 0 | 0 | 0 | 0 | 0 | 0 | 0 | | | | | | |
| 1665 | 01880 | 01664 | 1 | | 0 | 0 | 1 | 0 | 0 | 0 | 0 | 0 | 0 | 0 | 0 | 0 | 0 | 0 | 0 | 0 | 0 | 0 | 0 | 0 | 0 | 0 | 0 | 0 | 0 | | | | | | |
| 1666 | 01881 | 01665 | 1 | | 0 | 0 | 1 | 0 | 0 | 0 | 0 | 0 | 0 | 0 | 0 | 0 | 0 | 0 | 0 | 0 | 0 | 0 | 0 | 0 | 0 | 0 | 0 | 0 | 0 | | | | | | |
| 1667 | 01882 | 01666 | 3 | | 0 | 0 | 2 | 0 | 0 | 0 | 0 | 0 | 0 | 0 | 0 | 0 | 0 | 0 | 0 | 0 | 0 | 0 | 0 | 0 | 0 | 1 | 0 | 0 | 0 | M15400 | 99.3 | 153 | 1 | 4588 | 4740 |
| 1668 | 01883 | 01667 | 1 | | 0 | 0 | 1 | 0 | 0 | 0 | 0 | 0 | 0 | 0 | 0 | 0 | 0 | 0 | 0 | 0 | 0 | 0 | 0 | 0 | 0 | 0 | 0 | 0 | 0 | | | | | | |
| 1669 | 01884 | 01668 | 3 | | 0 | 0 | 1 | 0 | 0 | 0 | 0 | 0 | 0 | 0 | 0 | 0 | 1 | 0 | 0 | 0 | 0 | 0 | 0 | 0 | 1 | 0 | 0 | 0 | 0 | | | | | | |
| 1670 | 01885 | 01669 | 2 | | 0 | 0 | 1 | 0 | 0 | 0 | 0 | 0 | 0 | 0 | 0 | 0 | 0 | 0 | 0 | 0 | 1 | 0 | 0 | 0 | 0 | 0 | 0 | 0 | 0 | | | | | | |
| 1671 | 01886 | 01670 | 1 | | 0 | 0 | 1 | 0 | 0 | 0 | 0 | 0 | 0 | 0 | 0 | 0 | 0 | 0 | 0 | 0 | 0 | 0 | 0 | 0 | 0 | 0 | 0 | 0 | 0 | | | | | | |
| 1672 | 01887 | 01671 | 4 | | 0 | 0 | 2 | 0 | 0 | 0 | 0 | 0 | 0 | 0 | 1 | 0 | 0 | 0 | 0 | 0 | 0 | 0 | 0 | 1 | 0 | 0 | 0 | 0 | 0 | | | | | | |
| 1673 | 01888 | 01672 | 1 | | 0 | 0 | 1 | 0 | 0 | 0 | 0 | 0 | 0 | 0 | 0 | 0 | 0 | 0 | 0 | 0 | 0 | 0 | 0 | 0 | 0 | 0 | 0 | 0 | 0 | | | | | | |
| 1674 | 01889 | 01673 | 2 | | 0 | 0 | 1 | 0 | 0 | 0 | 0 | 0 | 1 | 0 | 0 | 0 | 0 | 0 | 0 | 0 | 0 | 0 | 0 | 0 | 0 | 0 | 0 | 0 | 0 | | | | | | |
| 1675 | 01890 | 01674 | 2 | | 0 | 0 | 1 | 0 | 0 | 0 | 0 | 0 | 0 | 0 | 0 | 0 | 0 | 0 | 0 | 0 | 0 | 0 | 0 | 0 | 0 | 0 | 0 | 1 | 0 | | | | | | |
| 1676 | 01891 | 01675 | 9 | | 0 | 0 | 1 | 0 | 0 | 0 | 3 | 0 | 0 | 1 | 0 | 0 | 0 | 0 | 0 | 0 | 1 | 0 | 0 | 0 | 0 | 1 | 0 | 0 | 2 | | | | | | |
| 1677 | 01892 | 01676 | 1 | | 0 | 0 | 1 | 0 | 0 | 0 | 0 | 0 | 0 | 0 | 0 | 0 | 0 | 0 | 0 | 0 | 0 | 0 | 0 | 0 | 0 | 0 | 0 | 0 | 0 | | | | | | |

Table 47

| | A | B | C | E | G | I | K | M | O | Q | S | U | W | Y | AA | AC | AD | AG | AI | AK | AM | AO | AQ | AS | AU | AW | AY | BA | BC | BE | BF | BG | BH | BI | BK |
|---|---|---|---|---|---|---|---|---|---|---|---|---|---|---|---|---|---|---|---|---|---|---|---|---|---|---|---|---|---|---|---|---|---|---|---|
| 1678 | 01893 | 01677 | 1 | | 0 | 0 | 1 | 0 | 0 | 0 | 0 | 0 | 0 | 0 | 0 | 0 | 0 | 0 | 0 | 0 | 0 | 0 | 0 | 0 | 0 | 0 | 0 | 0 | 0 | | | | | | |
| 1679 | 01895 | 01678 | 7 | | 0 | 0 | 1 | 0 | 0 | 0 | 0 | 0 | 1 | 0 | 0 | 1 | 0 | 2 | 0 | 1 | 0 | 0 | 0 | 0 | 1 | 0 | 0 | 0 | 0 | | | | | | |
| 1680 | 01896 | 01679 | 2 | | 0 | 0 | 2 | 0 | 0 | 0 | 0 | 0 | 0 | 0 | 0 | 0 | 0 | 0 | 0 | 0 | 0 | 0 | 0 | 0 | 0 | 0 | 0 | 0 | 0 | | | | | | |
| 1681 | 01897 | 01680 | 1 | | 0 | 0 | 1 | 0 | 0 | 0 | 0 | 0 | 0 | 0 | 0 | 0 | 0 | 0 | 0 | 0 | 0 | 0 | 0 | 0 | 0 | 0 | 0 | 0 | 0 | | | | | | |
| 1682 | 01898 | 01681 | 1 | | 0 | 0 | 1 | 0 | 0 | 0 | 0 | 0 | 0 | 0 | 0 | 0 | 0 | 0 | 0 | 0 | 0 | 0 | 0 | 0 | 0 | 0 | 0 | 0 | 0 | | | | | | |
| 1683 | 01899 | 01682 | 14 | | 0 | 0 | 2 | 0 | 0 | 0 | 0 | 0 | 2 | 0 | 0 | 3 | 1 | 0 | 0 | 1 | 1 | 3 | 0 | 1 | 0 | 0 | 0 | 0 | 0 | K00557 | 95.2 | 145 | 1 | 1104 | 1246 |
| 1684 | 01900 | 01683 | 1 | | 0 | 0 | 1 | 0 | 0 | 0 | 0 | 0 | 0 | 0 | 0 | 0 | 0 | 0 | 0 | 0 | 0 | 0 | 0 | 0 | 0 | 0 | 0 | 0 | 0 | | | | | | |
| 1685 | 01901 | 01684 | 1 | | 0 | 0 | 1 | 0 | 0 | 0 | 0 | 0 | 0 | 0 | 0 | 0 | 0 | 0 | 0 | 0 | 0 | 0 | 0 | 0 | 0 | 0 | 0 | 0 | 0 | | | | | | |
| 1686 | 01902 | 01685 | 2 | | 0 | 0 | 1 | 0 | 0 | 0 | 0 | 0 | 0 | 0 | 0 | 0 | 0 | 0 | 1 | 0 | 0 | 0 | 0 | 0 | 0 | 0 | 0 | 0 | 0 | M98252 | 98.5 | 136 | 1 | 2804 | 2939 |
| 1687 | 01903 | 01686 | 2 | | 0 | 0 | 2 | 0 | 0 | 0 | 0 | 0 | 0 | 0 | 0 | 0 | 0 | 0 | 0 | 0 | 0 | 0 | 0 | 0 | 0 | 0 | 0 | 0 | 0 | | | | | | |
| 1688 | 01904 | 01687 | 3 | | 0 | 0 | 1 | 0 | 0 | 0 | 0 | 0 | 0 | 2 | 0 | 0 | 0 | 0 | 0 | 0 | 0 | 0 | 0 | 0 | 0 | 0 | 0 | 0 | 0 | | | | | | |
| 1689 | 01905 | 01688 | 3 | | 0 | 0 | 1 | 0 | 0 | 0 | 0 | 0 | 0 | 0 | 0 | 0 | 0 | 0 | 0 | 0 | 1 | 1 | 0 | 0 | 0 | 0 | 0 | 0 | 0 | | | | | | |
| 1690 | 01906 | 01689 | 3 | | 0 | 0 | 1 | 1 | 0 | 0 | 0 | 0 | 0 | 0 | 0 | 1 | 0 | 0 | 0 | 0 | 0 | 0 | 0 | 0 | 0 | 0 | 0 | 0 | 0 | | | | | | |
| 1691 | 01907 | 01690 | 1 | | 0 | 0 | 1 | 0 | 0 | 0 | 0 | 0 | 0 | 0 | 0 | 0 | 0 | 0 | 0 | 0 | 0 | 0 | 0 | 0 | 0 | 0 | 0 | 0 | 0 | | | | | | |
| 1692 | 01908 | 01691 | 21 | | 0 | 0 | 2 | 0 | 0 | 2 | 0 | 0 | 0 | 4 | 0 | 0 | 0 | 0 | 3 | 9 | 0 | 1 | 0 | 0 | 0 | 0 | 0 | 0 | 0 | L16510 | 99.2 | 126 | 1 | 1871 | 1996 |
| 1693 | 01909 | 01692 | 4 | | 0 | 0 | 1 | 0 | 0 | 0 | 0 | 0 | 0 | 0 | 0 | 0 | 0 | 0 | 0 | 2 | 0 | 0 | 0 | 0 | 1 | 0 | 0 | 0 | 0 | X71129 | 100 | 140 | 1 | 699 | 852 |
| 1694 | 01910 | 01693 | 1 | | 0 | 0 | 1 | 0 | 0 | 0 | 0 | 0 | 0 | 0 | 0 | 0 | 0 | 0 | 0 | 0 | 0 | 0 | 0 | 0 | 0 | 0 | 0 | 0 | 0 | | | | | | |
| 1695 | 01911 | 01694 | 10 | | 0 | 0 | 1 | 1 | 0 | 0 | 1 | 1 | 0 | 0 | 0 | 0 | 0 | 0 | 2 | 1 | 0 | 2 | 0 | 0 | 0 | 0 | 0 | 1 | 0 | | | | | | |
| 1696 | 01912 | 01695 | 5 | | 0 | 0 | 1 | 0 | 0 | 2 | 0 | 0 | 0 | 0 | 0 | 0 | 0 | 0 | 1 | 0 | 0 | 0 | 0 | 0 | 0 | 0 | 1 | 0 | 0 | Z22572 | 98.5 | 131 | 1 | 2593 | 3723 |
| 1697 | 01913 | 01696 | 2 | | 0 | 0 | 2 | 0 | 0 | 0 | 0 | 0 | 0 | 0 | 0 | 0 | 0 | 0 | 0 | 0 | 0 | 0 | 0 | 0 | 0 | 0 | 0 | 0 | 0 | | | | | | |
| 1698 | 01914 | 01697 | 3 | | 0 | 0 | 3 | 0 | 0 | 0 | 0 | 0 | 0 | 0 | 0 | 0 | 0 | 0 | 0 | 0 | 0 | 0 | 0 | 0 | 0 | 0 | 0 | 0 | 0 | | | | | | |
| 1699 | 01915 | 01698 | 1 | | 0 | 0 | 1 | 0 | 0 | 0 | 0 | 0 | 0 | 0 | 0 | 0 | 0 | 0 | 0 | 0 | 0 | 0 | 0 | 0 | 0 | 0 | 0 | 0 | 0 | | | | | | |
| 1700 | 01916 | 01699 | 3 | | 0 | 0 | 2 | 0 | 0 | 0 | 0 | 0 | 0 | 1 | 0 | 0 | 0 | 0 | 0 | 0 | 0 | 0 | 0 | 0 | 0 | 0 | 0 | 0 | 0 | | | | | | |
| 1701 | 01917 | 01700 | 1 | | 0 | 0 | 1 | 0 | 0 | 0 | 0 | 0 | 0 | 0 | 0 | 0 | 0 | 0 | 0 | 0 | 0 | 0 | 0 | 0 | 0 | 0 | 0 | 0 | 0 | | | | | | |
| 1702 | 01918 | 01701 | 2 | | 0 | 0 | 1 | 0 | 0 | 0 | 0 | 0 | 0 | 1 | 0 | 0 | 0 | 0 | 0 | 0 | 0 | 0 | 0 | 0 | 0 | 0 | 0 | 0 | 0 | | | | | | |
| 1703 | 01919 | 01702 | 111 | | 0 | 0 | 5 | 0 | 1 | 0 | 3 | 5 | 6 | 12 | 3 | 2 | 0 | 0 | 7 | 23 | 5 | 2 | 3 | 3 | 6 | 22 | 3 | 0 | 0 | | | | | | |
| 1704 | 01920 | 01703 | 1 | | 0 | 0 | 1 | 0 | 0 | 0 | 0 | 0 | 0 | 0 | 0 | 0 | 0 | 0 | 0 | 0 | 0 | 0 | 0 | 0 | 0 | 0 | 0 | 0 | 0 | | | | | | |
| 1705 | 01921 | 01704 | 1 | | 0 | 0 | 1 | 0 | 0 | 0 | 0 | 0 | 0 | 0 | 0 | 0 | 0 | 0 | 0 | 0 | 0 | 0 | 0 | 0 | 0 | 0 | 0 | 0 | 0 | | | | | | |
| 1706 | 01922 | 01705 | 3 | | 0 | 0 | 2 | 0 | 0 | 0 | 0 | 0 | 0 | 0 | 0 | 0 | 0 | 0 | 0 | 0 | 0 | 0 | 0 | 0 | 0 | 0 | 1 | 0 | 0 | | | | | | |
| 1707 | 01923 | 01706 | 2 | | 0 | 0 | 1 | 0 | 0 | 0 | 1 | 0 | 0 | 0 | 0 | 0 | 0 | 0 | 0 | 0 | 0 | 0 | 0 | 0 | 0 | 0 | 0 | 0 | 0 | | | | | | |
| 1708 | 01924 | 01707 | 3 | | 0 | 0 | 1 | 0 | 0 | 0 | 0 | 0 | 0 | 0 | 0 | 0 | 0 | 0 | 0 | 1 | 0 | 1 | 0 | 0 | 0 | 0 | 0 | 0 | 0 | | | | | | |
| 1709 | 01925 | 01708 | 1 | | 0 | 0 | 1 | 0 | 0 | 0 | 0 | 0 | 0 | 0 | 0 | 0 | 0 | 0 | 0 | 0 | 0 | 0 | 0 | 0 | 0 | 0 | 0 | 0 | 0 | | | | | | |
| 1710 | 01926 | 01709 | 24 | | 0 | 0 | 2 | 0 | 0 | 0 | 2 | 0 | 0 | 3 | 0 | 0 | 1 | 2 | 1 | 0 | 0 | 2 | 3 | 1 | 1 | 1 | 2 | 1 | 2 | M29870 | 100 | 64 | 1 | 516 | 579 |
| 1711 | 01927 | 01710 | 3 | | 0 | 0 | 1 | 0 | 1 | 0 | 0 | 0 | 0 | 0 | 0 | 0 | 0 | 0 | 1 | 0 | 0 | 0 | 0 | 0 | 0 | 0 | 0 | 0 | 0 | | | | | | |
| 1712 | 01928 | 01711 | 10 | | 0 | 0 | 4 | 0 | 0 | 0 | 0 | 0 | 0 | 2 | 0 | 0 | 0 | 0 | 1 | 0 | 3 | 0 | 0 | 0 | 0 | 0 | 0 | 0 | 0 | | | | | | |
| 1713 | 01929 | 01712 | 19 | | 0 | 0 | 1 | 0 | 1 | 0 | 4 | 0 | 0 | 0 | 0 | 0 | 2 | 1 | 1 | 3 | 1 | 0 | 0 | 0 | 0 | 1 | 1 | 0 | 3 | | | | | | |

Table 48

| | A | B | C | E | G | I | K | M | O | Q | S | U | W | Y | AA | AC | AF | AG | AI | AK | AM | AO | AQ | AS | AU | AW | AY | BA | BC | BE | BF | BG | BH | BI | BK |
|---|---|---|---|---|---|---|---|---|---|---|---|---|---|---|---|---|---|---|---|---|---|---|---|---|---|---|---|---|---|---|---|---|---|---|---|
| 1714 | 01930 | 01713 | 2 | | 0 | 0 | 1 | 0 | 0 | 0 | 0 | 0 | 0 | 0 | 0 | 0 | 0 | 0 | 0 | 0 | 0 | 0 | 0 | 0 | 0 | 0 | 0 | 1 | 0 | | | | | | |
| 1715 | 01931 | 01714 | 4 | | 0 | 0 | 2 | 1 | 0 | 0 | 0 | 0 | 0 | 0 | 0 | 0 | 1 | 0 | 0 | 0 | 0 | 0 | 0 | 0 | 0 | 0 | 0 | 0 | 0 | | | | | | |
| 1716 | 01932 | 01715 | 2 | | 0 | 0 | 1 | 0 | 0 | 0 | 0 | 0 | 0 | 0 | 0 | 0 | 0 | 0 | 0 | 0 | 0 | 0 | 0 | 0 | 0 | 0 | 0 | 0 | 1 | | | | | | |
| 1717 | 01933 | 01716 | 10 | | 0 | 0 | 1 | 0 | 0 | 0 | 0 | 0 | 1 | 0 | 1 | 0 | 0 | 0 | 0 | 4 | 1 | 0 | 0 | 0 | 0 | 2 | 0 | 0 | 0 | X60111 | 97.5 | 276 | 1 | 756 | 1120 |
| 1718 | 01934 | 01717 | 5 | | 0 | 0 | 1 | 1 | 0 | 0 | 0 | 1 | 2 | 0 | 0 | 0 | 0 | 0 | 0 | 0 | 0 | 0 | 0 | 0 | 0 | 0 | 0 | 0 | 0 | | | | | | |
| 1719 | 01935 | 01718 | 1 | | 0 | 0 | 1 | 0 | 0 | 0 | 0 | 0 | 0 | 0 | 0 | 0 | 0 | 0 | 0 | 0 | 0 | 0 | 0 | 0 | 0 | 0 | 0 | 0 | 0 | | | | | | |
| 1720 | 01936 | 01719 | 2 | | 0 | 0 | 1 | 0 | 0 | 1 | 0 | 0 | 0 | 0 | 0 | 0 | 0 | 0 | 0 | 0 | 0 | 0 | 0 | 0 | 0 | 0 | 0 | 0 | 0 | J03132 | 93.9 | 114 | 1 | 2834 | 2986 |
| 1721 | 01937 | 01720 | 1 | | 0 | 0 | 1 | 0 | 0 | 0 | 0 | 0 | 0 | 0 | 0 | 0 | 0 | 0 | 0 | 0 | 0 | 0 | 0 | 0 | 0 | 0 | 0 | 0 | 0 | | | | | | |
| 1722 | 01938 | 01721 | 3 | | 0 | 0 | 1 | 0 | 0 | 0 | 0 | 0 | 1 | 0 | 0 | 0 | 0 | 0 | 1 | 0 | 0 | 0 | 0 | 0 | 0 | 0 | 0 | 0 | 0 | | | | | | |
| 1723 | 01939 | 01722 | 3 | | 0 | 0 | 1 | 0 | 0 | 0 | 0 | 0 | 0 | 0 | 0 | 1 | 0 | 0 | 0 | 0 | 0 | 0 | 1 | 0 | 0 | 0 | 0 | 0 | 0 | | | | | | |
| 1724 | 01940 | 01723 | 1 | | 0 | 0 | 1 | 0 | 0 | 0 | 0 | 0 | 0 | 0 | 0 | 0 | 0 | 0 | 0 | 0 | 0 | 0 | 0 | 0 | 0 | 0 | 0 | 0 | 0 | | | | | | |
| 1725 | 01941 | 01724 | 1 | | 0 | 0 | 1 | 0 | 0 | 0 | 0 | 0 | 0 | 0 | 0 | 0 | 0 | 0 | 0 | 0 | 0 | 0 | 0 | 0 | 0 | 0 | 0 | 0 | 0 | | | | | | |
| 1726 | 01943 | 01725 | 1 | | 0 | 0 | 1 | 0 | 0 | 0 | 0 | 0 | 0 | 0 | 0 | 0 | 0 | 0 | 0 | 0 | 0 | 0 | 0 | 0 | 0 | 0 | 0 | 0 | 0 | | | | | | |
| 1727 | 01944 | 01726 | 2 | | 0 | 0 | 1 | 0 | 0 | 0 | 0 | 0 | 0 | 0 | 0 | 0 | 0 | 0 | 0 | 0 | 1 | 0 | 0 | 0 | 0 | 0 | 0 | 0 | 0 | | | | | | |
| 1728 | 01945 | 01727 | 7 | | 0 | 0 | 1 | 1 | 0 | 0 | 0 | 1 | 0 | 0 | 0 | 0 | 0 | 2 | 0 | 1 | 0 | 0 | 0 | 0 | 1 | 0 | 0 | 0 | 0 | | | | | | |
| 1729 | 01946 | 01728 | 3 | | 0 | 0 | 1 | 0 | 0 | 0 | 0 | 0 | 0 | 0 | 0 | 0 | 0 | 0 | 0 | 0 | 1 | 1 | 0 | 0 | 0 | 0 | 0 | 0 | 0 | Z28407 | 94.2 | 328 | 1 | 236 | 852 |
| 1730 | 01947 | 01729 | 6 | | 0 | 0 | 1 | 0 | 0 | 0 | 0 | 1 | 0 | 0 | 0 | 1 | 0 | 1 | 1 | 0 | 0 | 0 | 0 | 1 | 0 | 0 | 0 | 0 | 0 | | | | | | |
| 1731 | 01948 | 01730 | 4 | | 0 | 0 | 1 | 0 | 1 | 0 | 0 | 0 | 0 | 0 | 0 | 0 | 0 | 0 | 0 | 0 | 0 | 0 | 1 | 1 | 0 | 0 | 0 | 0 | 0 | | | | | | |
| 1732 | 01949 | 01731 | 1 | | 0 | 0 | 1 | 0 | 0 | 0 | 0 | 0 | 0 | 0 | 0 | 0 | 0 | 0 | 0 | 0 | 0 | 0 | 0 | 0 | 0 | 0 | 0 | 0 | 0 | | | | | | |
| 1733 | 01950 | 01732 | 1 | | 0 | 0 | 1 | 0 | 0 | 0 | 0 | 0 | 0 | 0 | 0 | 0 | 0 | 0 | 0 | 0 | 0 | 0 | 0 | 0 | 0 | 0 | 0 | 0 | 0 | | | | | | |
| 1734 | 01951 | 01733 | 1 | | 0 | 0 | 1 | 0 | 0 | 0 | 0 | 0 | 0 | 0 | 0 | 0 | 0 | 0 | 0 | 0 | 0 | 0 | 0 | 0 | 0 | 0 | 0 | 0 | 0 | | | | | | |
| 1735 | 01952 | 01734 | 1 | | 0 | 0 | 1 | 0 | 0 | 0 | 0 | 0 | 0 | 0 | 0 | 0 | 0 | 0 | 0 | 0 | 0 | 0 | 0 | 0 | 0 | 0 | 0 | 0 | 0 | | | | | | |
| 1736 | 01953 | 01735 | 17 | | 0 | 0 | 2 | 2 | 0 | 0 | 0 | 1 | 0 | 1 | 0 | 3 | 0 | 2 | 0 | 1 | 0 | 0 | 1 | 0 | 1 | 1 | 0 | 2 | 0 | | | | | | |
| 1737 | 01954 | 01736 | 1 | | 0 | 0 | 1 | 0 | 0 | 0 | 0 | 0 | 0 | 0 | 0 | 0 | 0 | 0 | 0 | 0 | 0 | 0 | 0 | 0 | 0 | 0 | 0 | 0 | 0 | | | | | | |
| 1738 | 01955 | 01737 | 1 | | 0 | 0 | 1 | 0 | 0 | 0 | 0 | 0 | 0 | 0 | 0 | 0 | 0 | 0 | 0 | 0 | 0 | 0 | 0 | 0 | 0 | 0 | 0 | 0 | 0 | | | | | | |
| 1739 | 01956 | 01738 | 1 | | 0 | 0 | 1 | 0 | 0 | 0 | 0 | 0 | 0 | 0 | 0 | 0 | 0 | 0 | 0 | 0 | 0 | 0 | 0 | 0 | 0 | 0 | 0 | 0 | 0 | | | | | | |
| 1740 | 01957 | 01739 | 4 | | 0 | 0 | 1 | 0 | 0 | 0 | 0 | 1 | 0 | 0 | 0 | 1 | 0 | 0 | 0 | 0 | 0 | 0 | 0 | 0 | 0 | 0 | 0 | 1 | 0 | | | | | | |
| 1741 | 01958 | 01740 | 6 | | 0 | 0 | 1 | 0 | 0 | 0 | 0 | 0 | 3 | 0 | 0 | 0 | 0 | 0 | 0 | 0 | 0 | 1 | 0 | 0 | 0 | 0 | 0 | 1 | 0 | | | | | | |
| 1742 | 01959 | 01741 | 5 | | 0 | 0 | 1 | 0 | 1 | 0 | 1 | 0 | 0 | 0 | 0 | 0 | 1 | 0 | 0 | 0 | 0 | 0 | 0 | 0 | 1 | 0 | 0 | 0 | 0 | | | | | | |
| 1743 | 01960 | 01742 | 6 | | 0 | 0 | 1 | 0 | 0 | 1 | 0 | 0 | 0 | 0 | 1 | 0 | 1 | 0 | 0 | 0 | 0 | 1 | 0 | 0 | 1 | 0 | 0 | 0 | 0 | M33519 | 98 | 100 | 1 | 3640 | 3740 |
| 1744 | 01961 | 01743 | 1 | | 0 | 0 | 1 | 0 | 0 | 0 | 0 | 0 | 0 | 0 | 0 | 0 | 0 | 0 | 0 | 0 | 0 | 0 | 0 | 0 | 0 | 0 | 0 | 0 | 0 | | | | | | |
| 1745 | 01962 | 01744 | 1 | | 0 | 0 | 1 | 0 | 0 | 0 | 0 | 0 | 0 | 0 | 0 | 0 | 0 | 0 | 0 | 0 | 0 | 0 | 0 | 0 | 0 | 0 | 0 | 0 | 0 | | | | | | |
| 1746 | 01963 | 01745 | 2 | | 0 | 0 | 2 | 0 | 0 | 0 | 0 | 0 | 0 | 0 | 0 | 0 | 0 | 0 | 0 | 0 | 0 | 0 | 0 | 0 | 0 | 0 | 0 | 0 | 0 | | | | | | |
| 1747 | 01964 | 01746 | 6 | | 0 | 0 | 2 | 0 | 0 | 0 | 0 | 2 | 0 | 0 | 0 | 0 | 0 | 1 | 0 | 0 | 0 | 0 | 0 | 0 | 0 | 0 | 0 | 1 | 0 | | | | | | |
| 1748 | 01965 | 01747 | 4 | | 0 | 0 | 1 | 0 | 0 | 0 | 0 | 0 | 0 | 0 | 0 | 0 | 0 | 0 | 0 | 2 | 0 | 0 | 0 | 0 | 0 | 0 | 1 | 0 | 0 | | | | | | |
| 1749 | 01966 | 01748 | 8 | | 0 | 0 | 2 | 0 | 0 | 0 | 0 | 0 | 0 | 0 | 1 | 0 | 2 | 0 | 1 | 0 | 0 | 0 | 0 | 1 | 1 | 0 | 0 | 0 | 0 | | | | | | |

Table 49

| | A | B | C | E | G | I | K | M | O | Q | S | U | W | Y | AA | AC | AE | AG | AI | AK | AM | AO | AQ | AS | AU | AW | AY | BA | BC | BE | BF | BG | BH | BI | BK |
|---|---|---|---|---|---|---|---|---|---|---|---|---|---|---|---|---|---|---|---|---|---|---|---|---|---|---|---|---|---|---|---|---|---|---|---|
| 1750 | 01967 | 01749 | 13 | | 0 | 0 | 1 | 1 | 0 | 0 | 1 | 0 | 0 | 0 | 1 | 2 | 1 | 0 | 0 | 2 | 1 | 2 | 0 | 0 | 1 | 0 | 0 | 0 | 0 | X14986 | 100 | 57 | 36 | 1 | 57 |
| 1751 | 01968 | 01750 | 1 | | 0 | 0 | 1 | 0 | 0 | 0 | 0 | 0 | 0 | 0 | 0 | 0 | 0 | 0 | 0 | 0 | 0 | 0 | 0 | 0 | 0 | 0 | 0 | 0 | 0 | | | | | | |
| 1752 | 01969 | 01751 | 1 | | 0 | 0 | 1 | 0 | 0 | 0 | 0 | 0 | 0 | 0 | 0 | 0 | 0 | 0 | 0 | 0 | 0 | 0 | 0 | 0 | 0 | 0 | 0 | 0 | 0 | | | | | | |
| 1753 | 01970 | 01752 | 1 | | 0 | 0 | 1 | 0 | 0 | 0 | 0 | 0 | 0 | 0 | 0 | 0 | 0 | 0 | 0 | 0 | 0 | 0 | 0 | 0 | 0 | 0 | 0 | 0 | 0 | | | | | | |
| 1754 | 01972 | 01753 | 3 | | 0 | 0 | 1 | 0 | 0 | 0 | 0 | 0 | 0 | 0 | 0 | 0 | 0 | 0 | 0 | 0 | 0 | 0 | 0 | 0 | 1 | 0 | 0 | 0 | 1 | | | | | | |
| 1755 | 01973 | 01754 | 1 | | 0 | 0 | 1 | 0 | 0 | 0 | 0 | 0 | 0 | 0 | 0 | 0 | 0 | 0 | 0 | 0 | 0 | 0 | 0 | 0 | 0 | 0 | 0 | 0 | 0 | | | | | | |
| 1756 | 01974 | 01755 | 3 | | 0 | 0 | 1 | 0 | 0 | 0 | 0 | 0 | 0 | 0 | 0 | 1 | 0 | 0 | 1 | 0 | 0 | 0 | 0 | 0 | 0 | 0 | 0 | 0 | 0 | | | | | | |
| 1757 | 01975 | 01756 | 6 | | 0 | 0 | 1 | 0 | 0 | 0 | 0 | 2 | 1 | 0 | 1 | 0 | 0 | 0 | 0 | 0 | 1 | 0 | 0 | 0 | 0 | 0 | 0 | 0 | 0 | | | | | | |
| 1758 | 01976 | 01757 | 5 | | 0 | 0 | 1 | 0 | 0 | 0 | 0 | 0 | 0 | 1 | 0 | 0 | 0 | 0 | 1 | 1 | 0 | 0 | 0 | 0 | 1 | 0 | 0 | 0 | 0 | | | | | | |
| 1759 | 01977 | 01758 | 1 | | 0 | 0 | 1 | 0 | 0 | 0 | 0 | 0 | 0 | 0 | 0 | 0 | 0 | 0 | 0 | 0 | 0 | 0 | 0 | 0 | 0 | 0 | 0 | 0 | 0 | | | | | | |
| 1760 | 01978 | 01759 | 2 | | 0 | 0 | 2 | 0 | 0 | 0 | 0 | 0 | 0 | 0 | 0 | 0 | 0 | 0 | 0 | 0 | 0 | 0 | 0 | 0 | 0 | 0 | 0 | 0 | 0 | | | | | | |
| 1761 | 01979 | 01760 | 2 | | 0 | 0 | 1 | 0 | 0 | 0 | 0 | 0 | 0 | 0 | 0 | 0 | 0 | 0 | 0 | 0 | 0 | 0 | 0 | 0 | 1 | 0 | 0 | 0 | 0 | | | | | | |
| 1762 | 01981 | 01761 | 1 | | 0 | 0 | 1 | 0 | 0 | 0 | 0 | 0 | 0 | 0 | 0 | 0 | 0 | 0 | 0 | 0 | 0 | 0 | 0 | 0 | 0 | 0 | 0 | 0 | 0 | | | | | | |
| 1763 | 01982 | 01762 | 1 | | 0 | 0 | 1 | 0 | 0 | 0 | 0 | 0 | 0 | 0 | 0 | 0 | 0 | 0 | 0 | 0 | 0 | 0 | 0 | 0 | 0 | 0 | 0 | 0 | 0 | | | | | | |
| 1764 | 01983 | 01763 | 16 | | 0 | 0 | 1 | 0 | 0 | 0 | 0 | 3 | 2 | 3 | 0 | 1 | 1 | 0 | 2 | 1 | 0 | 2 | 0 | 0 | 0 | 0 | 0 | 0 | 0 | | | | | | |
| 1765 | 01984 | 01764 | 3 | | 0 | 0 | 1 | 0 | 0 | 0 | 0 | 0 | 0 | 0 | 0 | 0 | 0 | 0 | 0 | 0 | 1 | 0 | 0 | 0 | 0 | 1 | 0 | 0 | 0 | | | | | | |
| 1766 | 01985 | 01765 | 3 | | 0 | 0 | 1 | 0 | 0 | 0 | 0 | 0 | 0 | 0 | 0 | 0 | 0 | 0 | 0 | 0 | 0 | 0 | 0 | 0 | 0 | 0 | 2 | 0 | 0 | | | | | | |
| 1767 | 01986 | 01766 | 1 | | 0 | 0 | 1 | 0 | 0 | 0 | 0 | 0 | 0 | 0 | 0 | 0 | 0 | 0 | 0 | 0 | 0 | 0 | 0 | 0 | 0 | 0 | 0 | 0 | 0 | | | | | | |
| 1768 | 01987 | 01767 | 2 | | 0 | 0 | 2 | 0 | 0 | 0 | 0 | 0 | 0 | 0 | 0 | 0 | 0 | 0 | 0 | 0 | 0 | 0 | 0 | 0 | 0 | 0 | 0 | 0 | 0 | | | | | | |
| 1769 | 01988 | 01768 | 2 | | 0 | 0 | 1 | 0 | 0 | 0 | 0 | 1 | 0 | 0 | 0 | 0 | 0 | 0 | 0 | 0 | 0 | 0 | 0 | 0 | 0 | 0 | 0 | 0 | 0 | | | | | | |
| 1770 | 01989 | 01769 | 1 | | 0 | 0 | 1 | 0 | 0 | 0 | 0 | 0 | 0 | 0 | 0 | 0 | 0 | 0 | 0 | 0 | 0 | 0 | 0 | 0 | 0 | 0 | 0 | 0 | 0 | | | | | | |
| 1771 | 01990 | 01770 | 4 | | 0 | 0 | 4 | 0 | 0 | 0 | 0 | 0 | 0 | 0 | 0 | 0 | 0 | 0 | 0 | 0 | 0 | 0 | 0 | 0 | 0 | 0 | 0 | 0 | 0 | M57502 | 98.6 | 71 | 1 | 449 | 520 |
| 1772 | 01991 | 01771 | 6 | | 0 | 0 | 1 | 0 | 0 | 0 | 0 | 0 | 0 | 2 | 1 | 0 | 0 | 0 | 0 | 0 | 0 | 0 | 1 | 0 | 0 | 1 | 0 | 0 | 0 | L06132 | 98.5 | 66 | 1 | 1742 | 1806 |
| 1773 | 01992 | 01772 | 2 | | 0 | 0 | 1 | 0 | 0 | 0 | 0 | 0 | 0 | 1 | 0 | 0 | 0 | 0 | 0 | 0 | 0 | 0 | 0 | 0 | 0 | 0 | 0 | 0 | 0 | | | | | | |
| 1774 | 01994 | 01773 | 1 | | 0 | 0 | 1 | 0 | 0 | 0 | 0 | 0 | 0 | 0 | 0 | 0 | 0 | 0 | 0 | 0 | 0 | 0 | 0 | 0 | 0 | 0 | 0 | 0 | 0 | | | | | | |
| 1775 | 01995 | 01774 | 18 | | 0 | 0 | 4 | 0 | 1 | 0 | 0 | 2 | 2 | 0 | 1 | 0 | 0 | 0 | 0 | 0 | 0 | 0 | 1 | 0 | 1 | 4 | 1 | 1 | 0 | X13710 | 93.8 | 65 | 1 | 1039 | 1100 |
| 1776 | 01996 | 01775 | 1 | | 0 | 0 | 1 | 0 | 0 | 0 | 0 | 0 | 0 | 0 | 0 | 0 | 0 | 0 | 0 | 0 | 0 | 0 | 0 | 0 | 0 | 0 | 0 | 0 | 0 | | | | | | |
| 1777 | 01997 | 01776 | 7 | | 0 | 0 | 2 | 0 | 0 | 0 | 1 | 0 | 0 | 2 | 0 | 0 | 0 | 0 | 0 | 0 | 1 | 0 | 0 | 0 | 0 | 1 | 0 | 0 | 0 | X13973 | 95.2 | 62 | 1 | 1860 | 1921 |
| 1778 | 01998 | 01777 | 2 | | 0 | 0 | 1 | 0 | 0 | 0 | 0 | 0 | 0 | 0 | 0 | 0 | 0 | 0 | 0 | 0 | 0 | 0 | 0 | 1 | 0 | 0 | 0 | 0 | 0 | | | | | | |
| 1779 | 01999 | 01778 | 1 | | 0 | 0 | 1 | 0 | 0 | 0 | 0 | 0 | 0 | 0 | 0 | 0 | 0 | 0 | 0 | 0 | 0 | 0 | 0 | 0 | 0 | 0 | 0 | 0 | 0 | | | | | | |
| 1780 | 02000 | 01779 | 2 | | 0 | 0 | 1 | 0 | 0 | 0 | 0 | 0 | 0 | 0 | 0 | 0 | 0 | 0 | 0 | 0 | 0 | 0 | 0 | 0 | 0 | 1 | 0 | 0 | 0 | | | | | | |
| 1781 | 02001 | 01780 | 4 | | 0 | 0 | 2 | 1 | 0 | 0 | 0 | 0 | 0 | 0 | 0 | 0 | 0 | 0 | 0 | 0 | 1 | 0 | 0 | 0 | 0 | 0 | 0 | 0 | 0 | | | | | | |
| 1782 | 02002 | 01781 | 4 | | 0 | 0 | 1 | 0 | 0 | 0 | 0 | 0 | 0 | 0 | 0 | 0 | 0 | 3 | 0 | 0 | 0 | 0 | 0 | 0 | 0 | 0 | 0 | 0 | 0 | | | | | | |
| 1783 | 02003 | 01782 | 2 | | 0 | 0 | 1 | 0 | 0 | 0 | 0 | 0 | 0 | 0 | 0 | 0 | 0 | 0 | 0 | 0 | 0 | 0 | 0 | 1 | 0 | 0 | 0 | 0 | 0 | | | | | | |
| 1784 | 02005 | 01783 | 3 | | 0 | 0 | 1 | 0 | 0 | 0 | 0 | 0 | 0 | 0 | 0 | 0 | 0 | 0 | 0 | 0 | 1 | 0 | 0 | 0 | 1 | 0 | 0 | 0 | 0 | | | | | | |
| 1785 | 02006 | 01784 | 4 | | 0 | 0 | 1 | 0 | 0 | 0 | 0 | 0 | 0 | 0 | 0 | 0 | 0 | 0 | 0 | 1 | 0 | 0 | 0 | 0 | 1 | 0 | 0 | 0 | 1 | M16750 | 96.6 | 58 | 1 | 2239 | 2297 |

Table 50

| | A | B | C | E | G | I | K | M | O | Q | S | U | W | Y | AA | AC | AE | AG | AI | AK | AM | AO | AQ | AS | AU | AW | AY | BA | BC | BE | BF | BG | BH | BI | BK |
|---|---|---|---|---|---|---|---|---|---|---|---|---|---|---|---|---|---|---|---|---|---|---|---|---|---|---|---|---|---|---|---|---|---|---|---|
| 1786 | 02007 | 01785 | 15 | | 0 | 0 | 1 | 0 | 0 | 0 | 0 | 1 | 0 | 2 | 0 | 0 | 2 | 0 | 1 | 4 | 0 | 0 | 1 | 0 | 0 | 1 | 2 | 0 | 0 | M69023 | 100 | 56 | 1 | 1064 | 1119 |
| 1787 | 02010 | 01786 | 7 | | 0 | 0 | 1 | 0 | 0 | 0 | 1 | 2 | 0 | 0 | 0 | 0 | 0 | 0 | 0 | 0 | 0 | 0 | 0 | 0 | 3 | 0 | 0 | 0 | 0 | | | | | | |
| 1788 | 02011 | 01787 | 3 | | 0 | 0 | 1 | 0 | 0 | 0 | 0 | 0 | 0 | 0 | 0 | 0 | 0 | 0 | 0 | 0 | 0 | 0 | 0 | 0 | 1 | 0 | 1 | 0 | 0 | | | | | | |
| 1789 | 02012 | 01788 | 16 | | 0 | 0 | 2 | 0 | 0 | 0 | 2 | 2 | 0 | 1 | 0 | 1 | 0 | 0 | 0 | 1 | 0 | 1 | 1 | 1 | 3 | 0 | 0 | 1 | 0 | | | | | | |
| 1790 | 02013 | 01789 | 12 | | 0 | 0 | 2 | 0 | 0 | 0 | 0 | 0 | 2 | 0 | 0 | 0 | 0 | 0 | 0 | 1 | 2 | 2 | 0 | 0 | 1 | 1 | 1 | 0 | 0 | | | | | | |
| 1791 | 02025 | 01790 | 8 | | 0 | 0 | 1 | 0 | 0 | 0 | 3 | 0 | 1 | 0 | 0 | 0 | 0 | 1 | 0 | 0 | 0 | 0 | 1 | 0 | 0 | 0 | 0 | 1 | 0 | | | | | | |
| 1792 | 02039 | 01791 | 1 | | 0 | 0 | 0 | 1 | 0 | 0 | 0 | 0 | 0 | 0 | 0 | 0 | 0 | 0 | 0 | 0 | 0 | 0 | 0 | 0 | 0 | 0 | 0 | 0 | 0 | | | | | | |
| 1793 | 02040 | 01792 | 1 | | 0 | 0 | 0 | 1 | 0 | 0 | 0 | 0 | 0 | 0 | 0 | 0 | 0 | 0 | 0 | 0 | 0 | 0 | 0 | 0 | 0 | 0 | 0 | 0 | 0 | | | | | | |
| 1794 | 02041 | 01793 | 1 | | 0 | 0 | 0 | 1 | 0 | 0 | 0 | 0 | 0 | 0 | 0 | 0 | 0 | 0 | 0 | 0 | 0 | 0 | 0 | 0 | 0 | 0 | 0 | 0 | 0 | M61108 | 90.5 | 505 | 1 | 4356 | 5000 |
| 1795 | 02042 | 01794 | 1 | | 0 | 0 | 0 | 1 | 0 | 0 | 0 | 0 | 0 | 0 | 0 | 0 | 0 | 0 | 0 | 0 | 0 | 0 | 0 | 0 | 0 | 0 | 0 | 0 | 0 | | | | | | |
| 1796 | 02043 | 01795 | 8 | | 0 | 0 | 0 | 7 | 0 | 0 | 0 | 0 | 0 | 0 | 0 | 0 | 1 | 0 | 0 | 0 | 0 | 0 | 0 | 0 | 0 | 0 | 0 | 0 | 0 | K02215 | 95.4 | 477 | 1 | 1435 | 2099 |
| 1797 | 02044 | 01796 | 5 | | 0 | 0 | 0 | 5 | 0 | 0 | 0 | 0 | 0 | 0 | 0 | 0 | 0 | 0 | 0 | 0 | 0 | 0 | 0 | 0 | 0 | 0 | 0 | 0 | 0 | | | | | | |
| 1798 | 02045 | 01797 | 2 | | 0 | 0 | 0 | 1 | 0 | 0 | 0 | 0 | 0 | 0 | 1 | 0 | 0 | 0 | 0 | 0 | 0 | 0 | 0 | 0 | 0 | 0 | 0 | 0 | 0 | | | | | | |
| 1799 | 02046 | 01798 | 1 | | 0 | 0 | 0 | 1 | 0 | 0 | 0 | 0 | 0 | 0 | 0 | 0 | 0 | 0 | 0 | 0 | 0 | 0 | 0 | 0 | 0 | 0 | 0 | 0 | 0 | | | | | | |
| 1800 | 02047 | 01799 | 13 | | 0 | 0 | 0 | 2 | 7 | 4 | 0 | 0 | 0 | 0 | 0 | 0 | 0 | 0 | 0 | 0 | 0 | 0 | 0 | 0 | 0 | 0 | 0 | 0 | 0 | X14174 | 99.3 | 140 | 100 | 7 | 2339 |
| 1801 | 02048 | 01800 | 5 | | 0 | 0 | 0 | 1 | 1 | 0 | 0 | 0 | 0 | 0 | 0 | 0 | 0 | 2 | 1 | 0 | 0 | 0 | 0 | 0 | 0 | 0 | 0 | 0 | 0 | | | | | | |
| 1802 | 02049 | 01801 | 20 | | 0 | 0 | 0 | 1 | 0 | 0 | 1 | 0 | 0 | 10 | 0 | 0 | 0 | 0 | 3 | 0 | 0 | 4 | 0 | 0 | 1 | 0 | 0 | 0 | 0 | K01228 | 92.4 | 432 | 1 | 2919 | 3347 |
| 1803 | 02050 | 01802 | 5 | | 0 | 0 | 0 | 2 | 0 | 0 | 0 | 0 | 0 | 0 | 0 | 0 | 0 | 0 | 0 | 0 | 0 | 0 | 0 | 0 | 0 | 1 | 2 | 0 | 0 | | | | | | |
| 1804 | 02051 | 01803 | 1 | | 0 | 0 | 0 | 1 | 0 | 0 | 0 | 0 | 0 | 0 | 0 | 0 | 0 | 0 | 0 | 0 | 0 | 0 | 0 | 0 | 0 | 0 | 0 | 0 | 0 | | | | | | |
| 1805 | 02052 | 01804 | 2 | | 0 | 0 | 0 | 1 | 0 | 0 | 0 | 0 | 0 | 0 | 0 | 1 | 0 | 0 | 0 | 0 | 0 | 0 | 0 | 0 | 0 | 0 | 0 | 0 | 0 | | | | | | |
| 1806 | 02053 | 01805 | 1 | | 0 | 0 | 0 | 1 | 0 | 0 | 0 | 0 | 0 | 0 | 0 | 0 | 0 | 0 | 0 | 0 | 0 | 0 | 0 | 0 | 0 | 0 | 0 | 0 | 0 | | | | | | |
| 1807 | 02054 | 01806 | 1 | | 0 | 0 | 0 | 1 | 0 | 0 | 0 | 0 | 0 | 0 | 0 | 0 | 0 | 0 | 0 | 0 | 0 | 0 | 0 | 0 | 0 | 0 | 0 | 0 | 0 | | | | | | |
| 1808 | 02055 | 01807 | 2 | | 0 | 0 | 0 | 1 | 0 | 1 | 0 | 0 | 0 | 0 | 0 | 0 | 0 | 0 | 0 | 0 | 0 | 0 | 0 | 0 | 0 | 0 | 0 | 0 | 0 | | | | | | |
| 1809 | 02056 | 01808 | 2 | | 0 | 0 | 0 | 1 | 1 | 0 | 0 | 0 | 0 | 0 | 0 | 0 | 0 | 0 | 0 | 0 | 0 | 0 | 0 | 0 | 0 | 0 | 0 | 0 | 0 | L19684 | 100 | 67 | 1 | 1218 | 1284 |
| 1810 | 02057 | 01809 | 5 | | 0 | 0 | 0 | 1 | 0 | 0 | 0 | 0 | 0 | 0 | 0 | 0 | 0 | 0 | 0 | 0 | 0 | 1 | 1 | 0 | 0 | 0 | 1 | 0 | 1 | | | | | | |
| 1811 | 02058 | 01810 | 5 | | 0 | 0 | 0 | 1 | 1 | 3 | 0 | 0 | 0 | 0 | 0 | 0 | 0 | 0 | 0 | 0 | 0 | 0 | 0 | 0 | 0 | 0 | 0 | 0 | 0 | | | | | | |
| 1812 | 02059 | 01811 | 6 | | 0 | 0 | 0 | 1 | 0 | 0 | 0 | 1 | 0 | 1 | 1 | 0 | 0 | 0 | 1 | 0 | 1 | 0 | 0 | 0 | 0 | 0 | 0 | 0 | 0 | X67698 | 97.4 | 346 | 1 | 448 | 808 |
| 1813 | 02060 | 01812 | 8 | | 0 | 0 | 0 | 1 | 1 | 0 | 0 | 0 | 0 | 5 | 0 | 0 | 0 | 0 | 0 | 0 | 0 | 0 | 0 | 0 | 1 | 0 | 0 | 0 | 0 | M31159 | 98.2 | 341 | 1 | 2135 | 2474 |
| 1814 | 02062 | 01813 | 1 | | 0 | 0 | 0 | 1 | 0 | 0 | 0 | 0 | 0 | 0 | 0 | 0 | 0 | 0 | 0 | 0 | 0 | 0 | 0 | 0 | 0 | 0 | 0 | 0 | 0 | | | | | | |
| 1815 | 02063 | 01814 | 1 | | 0 | 0 | 0 | 1 | 0 | 0 | 0 | 0 | 0 | 0 | 0 | 0 | 0 | 0 | 0 | 0 | 0 | 0 | 0 | 0 | 0 | 0 | 0 | 0 | 0 | | | | | | |
| 1816 | 02064 | 01815 | 1 | | 0 | 0 | 0 | 1 | 0 | 0 | 0 | 0 | 0 | 0 | 0 | 0 | 0 | 0 | 0 | 0 | 0 | 0 | 0 | 0 | 0 | 0 | 0 | 0 | 0 | | | | | | |
| 1817 | 02065 | 01816 | 2 | | 0 | 0 | 0 | 2 | 0 | 0 | 0 | 0 | 0 | 0 | 0 | 0 | 0 | 0 | 0 | 0 | 0 | 0 | 0 | 0 | 0 | 0 | 0 | 0 | 0 | X64877 | 99.7 | 335 | 1 | 690 | 1040 |
| 1818 | 02066 | 01817 | 4 | | 0 | 0 | 0 | 1 | 0 | 0 | 0 | 0 | 0 | 0 | 0 | 0 | 0 | 1 | 0 | 0 | 0 | 1 | 0 | 1 | 0 | 0 | 0 | 0 | 0 | | | | | | |
| 1819 | 02067 | 01818 | 1 | | 0 | 0 | 0 | 1 | 0 | 0 | 0 | 0 | 0 | 0 | 0 | 0 | 0 | 0 | 0 | 0 | 0 | 0 | 0 | 0 | 0 | 0 | 0 | 0 | 0 | | | | | | |
| 1820 | 02068 | 01819 | 3 | | 0 | 0 | 0 | 2 | 0 | 0 | 0 | 0 | 0 | 1 | 0 | 0 | 0 | 0 | 0 | 0 | 0 | 0 | 0 | 0 | 0 | 0 | 0 | 0 | 0 | | | | | | |
| 1821 | 02069 | 01820 | 7 | | 0 | 0 | 0 | 1 | 1 | 1 | 2 | 0 | 0 | 0 | 0 | 0 | 0 | 0 | 0 | 0 | 0 | 2 | 0 | 0 | 0 | 0 | 0 | 0 | 0 | | | | | | |

Table 51

| | A | B | C | E | G | I | K | M | O | Q | S | U | W | Y | AA | AC | AE | AG | AI | AK | AM | AO | AQ | AS | AU | AW | AY | BA | BC | BE | BF | BG | BH | BI | BK |
|---|---|---|---|---|---|---|---|---|---|---|---|---|---|---|---|---|---|---|---|---|---|---|---|---|---|---|---|---|---|---|---|---|---|---|---|
| 1822 | 02070 | 01821 | 2 | | 0 | 0 | 0 | 1 | 0 | 0 | 0 | 0 | 0 | 1 | 0 | 0 | 0 | 0 | 0 | 0 | 0 | 0 | 0 | 0 | 0 | 0 | 0 | 0 | 0 | | | | | | |
| 1823 | 02071 | 01822 | 1 | | 0 | 0 | 0 | 1 | 0 | 0 | 0 | 0 | 0 | 0 | 0 | 0 | 0 | 0 | 0 | 0 | 0 | 0 | 0 | 0 | 0 | 0 | 0 | 0 | 0 | | | | | | |
| 1824 | 02072 | 01823 | 1 | | 0 | 0 | 0 | 1 | 0 | 0 | 0 | 0 | 0 | 0 | 0 | 0 | 0 | 0 | 0 | 0 | 0 | 0 | 0 | 0 | 0 | 0 | 0 | 0 | 0 | | | | | | |
| 1825 | 02073 | 01824 | 16 | | 0 | 0 | 0 | 1 | 0 | 0 | 1 | 2 | 0 | 0 | 3 | 0 | 3 | 0 | 1 | 0 | 0 | 2 | 0 | 1 | 0 | 0 | 0 | 1 | 1 | | | | | | |
| 1826 | 02074 | 01825 | 7 | | 0 | 0 | 0 | 1 | 0 | 0 | 0 | 0 | 0 | 0 | 0 | 0 | 0 | 0 | 1 | 2 | 0 | 0 | 0 | 0 | 0 | 0 | 0 | 3 | 0 | | | | | | |
| 1827 | 02075 | 01826 | 7 | | 0 | 0 | 0 | 1 | 3 | 3 | 0 | 0 | 0 | 0 | 0 | 0 | 0 | 0 | 0 | 0 | 0 | 0 | 0 | 0 | 0 | 0 | 0 | 0 | 0 | X07173 | 96.4 | 308 | 1 | 2782 | 3089 |
| 1828 | 02076 | 01827 | 4 | | 0 | 0 | 0 | 2 | 0 | 0 | 0 | 0 | 0 | 0 | 0 | 0 | 0 | 0 | 0 | 0 | 0 | 1 | 1 | 0 | 0 | 0 | 0 | 0 | 0 | | | | | | |
| 1829 | 02077 | 01828 | 3 | | 0 | 0 | 0 | 1 | 0 | 0 | 0 | 0 | 0 | 1 | 0 | 0 | 0 | 0 | 0 | 0 | 1 | 0 | 0 | 0 | 0 | 0 | 0 | 0 | 0 | | | | | | |
| 1830 | 02078 | 01829 | 1 | | 0 | 0 | 0 | 1 | 0 | 0 | 0 | 0 | 0 | 0 | 0 | 0 | 0 | 0 | 0 | 0 | 0 | 0 | 0 | 0 | 0 | 0 | 0 | 0 | 0 | | | | | | |
| 1831 | 02079 | 01830 | 2 | | 0 | 0 | 0 | 1 | 0 | 0 | 0 | 0 | 0 | 0 | 0 | 0 | 0 | 0 | 0 | 0 | 0 | 0 | 0 | 0 | 0 | 0 | 1 | 0 | 0 | M64925 | 95.7 | 281 | 1 | 1708 | 1989 |
| 1832 | 02080 | 01831 | 11 | | 0 | 0 | 0 | 1 | 1 | 0 | 0 | 1 | 0 | 0 | 0 | 0 | 2 | 0 | 1 | 0 | 0 | 0 | 2 | 0 | 0 | 0 | 0 | 3 | 0 | | | | | | |
| 1833 | 02081 | 01832 | 3 | | 0 | 0 | 0 | 1 | 0 | 0 | 0 | 0 | 0 | 0 | 1 | 0 | 0 | 0 | 0 | 0 | 0 | 0 | 0 | 1 | 0 | 0 | 0 | 0 | 0 | | | | | | |
| 1834 | 02082 | 01833 | 7 | | 0 | 0 | 0 | 2 | 2 | 0 | 0 | 0 | 0 | 0 | 0 | 0 | 0 | 0 | 0 | 0 | 0 | 0 | 2 | 1 | 0 | 0 | 0 | 0 | 0 | M25113 | 98.2 | 279 | 1 | 298 | 576 |
| 1835 | 02084 | 01834 | 1 | | 0 | 0 | 0 | 1 | 0 | 0 | 0 | 0 | 0 | 0 | 0 | 0 | 0 | 0 | 0 | 0 | 0 | 0 | 0 | 0 | 0 | 0 | 0 | 0 | 0 | | | | | | |
| 1836 | 02085 | 01835 | 83 | | 0 | 0 | 0 | 16 | 26 | 40 | 0 | 0 | 0 | 0 | 0 | 0 | 0 | 0 | 0 | 0 | 0 | 0 | 0 | 0 | 1 | 0 | 0 | 0 | 0 | K00422 | 98.1 | 268 | 1 | 1131 | 1397 |
| 1837 | 02086 | 01836 | 4 | | 0 | 0 | 0 | 2 | 0 | 0 | 0 | 1 | 0 | 0 | 1 | 0 | 0 | 0 | 0 | 0 | 0 | 0 | 0 | 0 | 0 | 0 | 0 | 0 | 0 | | | | | | |
| 1838 | 02087 | 01837 | 2 | | 0 | 0 | 0 | 1 | 0 | 0 | 0 | 0 | 0 | 0 | 0 | 0 | 0 | 0 | 0 | 0 | 0 | 0 | 0 | 0 | 0 | 0 | 0 | 1 | 0 | | | | | | |
| 1839 | 02088 | 01838 | 2 | | 0 | 0 | 0 | 1 | 0 | 1 | 0 | 0 | 0 | 0 | 0 | 0 | 0 | 0 | 0 | 0 | 0 | 0 | 0 | 0 | 0 | 0 | 0 | 0 | 0 | | | | | | |
| 1840 | 02089 | 01839 | 2 | | 0 | 0 | 0 | 2 | 0 | 0 | 0 | 0 | 0 | 0 | 0 | 0 | 0 | 0 | 0 | 0 | 0 | 0 | 0 | 0 | 0 | 0 | 0 | 0 | 0 | | | | | | |
| 1841 | 02090 | 01840 | 1 | | 0 | 0 | 0 | 1 | 0 | 0 | 0 | 0 | 0 | 0 | 0 | 0 | 0 | 0 | 0 | 0 | 0 | 0 | 0 | 0 | 0 | 0 | 0 | 0 | 0 | | | | | | |
| 1842 | 02091 | 01841 | 9 | | 0 | 0 | 0 | 1 | 0 | 0 | 2 | 2 | 0 | 1 | 0 | 0 | 0 | 0 | 0 | 0 | 0 | 0 | 0 | 1 | 2 | 0 | 0 | 0 | 0 | M14036 | 97.6 | 210 | 1 | 878 | 1088 |
| 1843 | 02092 | 01842 | 11 | | 0 | 0 | 0 | 2 | 1 | 7 | 0 | 0 | 0 | 0 | 0 | 0 | 0 | 0 | 0 | 0 | 0 | 0 | 0 | 0 | 1 | 0 | 0 | 0 | 0 | X00129 | 98 | 254 | 1 | 629 | 882 |
| 1844 | 02093 | 01843 | 11 | | 0 | 0 | 0 | 2 | 3 | 6 | 0 | 0 | 0 | 0 | 0 | 0 | 0 | 0 | 0 | 0 | 0 | 0 | 0 | 0 | 0 | 0 | 0 | 0 | 0 | X02747 | 100 | 241 | 1 | 1412 | 1652 |
| 1845 | 02094 | 01844 | 6 | | 0 | 0 | 0 | 1 | 2 | 0 | 0 | 0 | 0 | 0 | 0 | 0 | 0 | 0 | 1 | 0 | 1 | 1 | 0 | 0 | 0 | 0 | 0 | 0 | 0 | | | | | | |
| 1846 | 02095 | 01845 | 2 | | 0 | 0 | 0 | 2 | 0 | 0 | 0 | 0 | 0 | 0 | 0 | 0 | 0 | 0 | 0 | 0 | 0 | 0 | 0 | 0 | 0 | 0 | 0 | 0 | 0 | S46963 | 99.6 | 240 | 1 | 1564 | 1814 |
| 1847 | 02096 | 01846 | 2 | | 0 | 0 | 0 | 1 | 1 | 0 | 0 | 0 | 0 | 0 | 0 | 0 | 0 | 0 | 0 | 0 | 0 | 0 | 0 | 0 | 0 | 0 | 0 | 0 | 0 | X17544 | 99.2 | 237 | 1 | 1308 | 1545 |
| 1848 | 02097 | 01847 | 1 | | 0 | 0 | 0 | 1 | 0 | 0 | 0 | 0 | 0 | 0 | 0 | 0 | 0 | 0 | 0 | 0 | 0 | 0 | 0 | 0 | 0 | 0 | 0 | 0 | 0 | X04481 | 99.6 | 229 | 1 | 2381 | 2609 |
| 1849 | 02098 | 01848 | 1 | | 0 | 0 | 0 | 1 | 0 | 0 | 0 | 0 | 0 | 0 | 0 | 0 | 0 | 0 | 0 | 0 | 0 | 0 | 0 | 0 | 0 | 0 | 0 | 0 | 0 | | | | | | |
| 1850 | 02099 | 01849 | 3 | | 0 | 0 | 0 | 2 | 0 | 0 | 0 | 0 | 0 | 0 | 0 | 0 | 0 | 0 | 0 | 0 | 0 | 0 | 0 | 0 | 0 | 1 | 0 | 0 | 0 | | | | | | |
| 1851 | 02100 | 01850 | 5 | | 0 | 0 | 0 | 1 | 0 | 0 | 0 | 0 | 0 | 0 | 0 | 1 | 0 | 0 | 1 | 0 | 1 | 1 | 0 | 0 | 0 | 0 | 0 | 0 | 0 | | | | | | |
| 1852 | 02101 | 01851 | 2 | | 0 | 0 | 0 | 1 | 0 | 0 | 1 | 0 | 0 | 0 | 0 | 0 | 0 | 0 | 0 | 0 | 0 | 0 | 0 | 0 | 0 | 0 | 0 | 0 | 0 | | | | | | |
| 1853 | 02102 | 01852 | 5 | | 0 | 0 | 0 | 1 | 0 | 0 | 0 | 1 | 0 | 0 | 0 | 0 | 0 | 0 | 2 | 0 | 0 | 0 | 0 | 0 | 0 | 0 | 0 | 1 | 0 | | | | | | |
| 1854 | 02103 | 01853 | 5 | | 0 | 0 | 0 | 1 | 1 | 0 | 0 | 0 | 0 | 0 | 1 | 0 | 0 | 0 | 1 | 0 | 1 | 0 | 0 | 0 | 0 | 0 | 0 | 0 | 0 | | | | | | |
| 1855 | 02104 | 01854 | 1 | | 0 | 0 | 0 | 1 | 0 | 0 | 0 | 0 | 0 | 0 | 0 | 0 | 0 | 0 | 0 | 0 | 0 | 0 | 0 | 0 | 0 | 0 | 0 | 0 | 0 | M25897 | 97.8 | 223 | 1 | 187 | 439 |
| 1856 | 02105 | 01855 | 109 | | 0 | 0 | 0 | 6 | 4 | 3 | 10 | 0 | 36 | 22 | 0 | 0 | 0 | 0 | 2 | 1 | 2 | 22 | 1 | 0 | 0 | 0 | 0 | 0 | 0 | X02761 | 99.1 | 219 | 1 | 7462 | 7680 |
| 1857 | 02106 | 01856 | 1 | | 0 | 0 | 0 | 1 | 0 | 0 | 0 | 0 | 0 | 0 | 0 | 0 | 0 | 0 | 0 | 0 | 0 | 0 | 0 | 0 | 0 | 0 | 0 | 0 | 0 | | | | | | |

Table 52

| | A | B | C | E | G | I | K | M | O | Q | S | U | W | Y | AA | AC | AF | AG | AI | AK | AM | AO | AQ | AS | AU | AW | AY | BA | BC | BE | BF | BG | BH | BI | BK |
|---|---|---|---|---|---|---|---|---|---|---|---|---|---|---|---|---|---|---|---|---|---|---|---|---|---|---|---|---|---|---|---|---|---|---|---|
| 1858 | 02107 | 01857 | 1 | | 0 | 0 | 0 | 1 | 0 | 0 | 0 | 0 | 0 | 0 | 0 | 0 | 0 | 0 | 0 | 0 | 0 | 0 | 0 | 0 | 0 | 0 | 0 | 0 | 0 | X65551 | 98.2 | 218 | 1 | 8718 | 11435 |
| 1859 | 02108 | 01858 | 1 | | 0 | 0 | 0 | 1 | 0 | 0 | 0 | 0 | 0 | 0 | 0 | 0 | 0 | 0 | 0 | 0 | 0 | 0 | 0 | 0 | 0 | 0 | 0 | 0 | 0 | X16609 | 98.2 | 219 | 1 | 5919 | 7252 |
| 1860 | 02110 | 01859 | 2 | | 0 | 0 | 0 | 1 | 0 | 0 | 0 | 0 | 0 | 0 | 0 | 0 | 0 | 0 | 0 | 0 | 0 | 0 | 0 | 0 | 0 | 0 | 0 | 1 | 0 | | | | | | |
| 1861 | 02111 | 01860 | 2 | | 0 | 0 | 0 | 1 | 0 | 0 | 0 | 0 | 0 | 0 | 0 | 0 | 0 | 0 | 0 | 0 | 0 | 0 | 1 | 0 | 0 | 0 | 0 | 0 | 0 | | | | | | |
| 1862 | 02112 | 01861 | 1 | | 0 | 0 | 0 | 1 | 0 | 0 | 0 | 0 | 0 | 0 | 0 | 0 | 0 | 0 | 0 | 0 | 0 | 0 | 0 | 0 | 0 | 0 | 0 | 0 | 0 | | | | | | |
| 1863 | 02113 | 01862 | 2 | | 0 | 0 | 0 | 1 | 0 | 0 | 0 | 0 | 0 | 0 | 0 | 0 | 0 | 0 | 0 | 1 | 0 | 0 | 0 | 0 | 0 | 0 | 0 | 0 | 0 | | | | | | |
| 1864 | 02115 | 01863 | 3 | | 0 | 0 | 0 | 1 | 0 | 0 | 0 | 0 | 0 | 0 | 0 | 0 | 0 | 0 | 0 | 0 | 0 | 0 | 0 | 0 | 0 | 0 | 0 | 2 | 0 | | | | | | |
| 1865 | 02116 | 01864 | 12 | | 0 | 0 | 0 | 3 | 2 | 5 | 0 | 1 | 0 | 0 | 0 | 0 | 0 | 0 | 0 | 1 | 0 | 0 | 0 | 0 | 0 | 0 | 0 | 0 | 0 | M12272 | 100 | 208 | 1 | 1262 | 1486 |
| 1866 | 02117 | 01865 | 1 | | 0 | 0 | 0 | 1 | 0 | 0 | 0 | 0 | 0 | 0 | 0 | 0 | 0 | 0 | 0 | 0 | 0 | 0 | 0 | 0 | 0 | 0 | 0 | 0 | 0 | M14057 | 98.6 | 70 | 1 | 1884 | 2171 |
| 1867 | 02118 | 01866 | 1 | | 0 | 0 | 0 | 1 | 0 | 0 | 0 | 0 | 0 | 0 | 0 | 0 | 0 | 0 | 0 | 0 | 0 | 0 | 0 | 0 | 0 | 0 | 0 | 0 | 0 | M64982 | 96.8 | 188 | 11 | 5234 | 5943 |
| 1868 | 02119 | 01867 | 8 | | 0 | 0 | 0 | 2 | 0 | 0 | 0 | 0 | 0 | 0 | 0 | 0 | 0 | 0 | 0 | 1 | 0 | 1 | 0 | 0 | 0 | 1 | 0 | 1 | 2 | | | | | | |
| 1869 | 02120 | 01868 | 19 | | 0 | 0 | 0 | 3 | 12 | 4 | 0 | 0 | 0 | 0 | 0 | 0 | 0 | 0 | 0 | 0 | 0 | 0 | 0 | 0 | 0 | 0 | 0 | 0 | 0 | M64983 | 99.5 | 197 | 1 | 8075 | 8878 |
| 1870 | 02121 | 01869 | 1 | | 0 | 0 | 0 | 1 | 0 | 0 | 0 | 0 | 0 | 0 | 0 | 0 | 0 | 0 | 0 | 0 | 0 | 0 | 0 | 0 | 0 | 0 | 0 | 0 | 0 | J02770 | 95.3 | 191 | 1 | 1773 | 1963 |
| 1871 | 02122 | 01870 | 1 | | 0 | 0 | 0 | 1 | 0 | 0 | 0 | 0 | 0 | 0 | 0 | 0 | 0 | 0 | 0 | 0 | 0 | 0 | 0 | 0 | 0 | 0 | 0 | 0 | 0 | | | | | | |
| 1872 | 02123 | 01871 | 28 | | 0 | 0 | 0 | 1 | 1 | 1 | 0 | 0 | 0 | 0 | 12 | 4 | 0 | 0 | 1 | 0 | 1 | 0 | 0 | 0 | 0 | 0 | 0 | 6 | 1 | | | | | | |
| 1873 | 02124 | 01872 | 1 | | 0 | 0 | 0 | 1 | 0 | 0 | 0 | 0 | 0 | 0 | 0 | 0 | 0 | 0 | 0 | 0 | 0 | 0 | 0 | 0 | 0 | 0 | 0 | 0 | 0 | | | | | | |
| 1874 | 02126 | 01873 | 7 | | 0 | 0 | 0 | 6 | 0 | 1 | 0 | 0 | 0 | 0 | 0 | 0 | 0 | 0 | 0 | 0 | 0 | 0 | 0 | 0 | 0 | 0 | 0 | 0 | 0 | | | | | | |
| 1875 | 02127 | 01874 | 1 | | 0 | 0 | 0 | 1 | 0 | 0 | 0 | 0 | 0 | 0 | 0 | 0 | 0 | 0 | 0 | 0 | 0 | 0 | 0 | 0 | 0 | 0 | 0 | 0 | 0 | | | | | | |
| 1876 | 02128 | 01875 | 3 | | 0 | 0 | 0 | 1 | 0 | 0 | 0 | 2 | 0 | 0 | 0 | 0 | 0 | 0 | 0 | 0 | 0 | 0 | 0 | 0 | 0 | 0 | 0 | 0 | 0 | | | | | | |
| 1877 | 02129 | 01876 | 4 | | 0 | 0 | 0 | 1 | 0 | 3 | 0 | 0 | 0 | 0 | 0 | 0 | 0 | 0 | 0 | 0 | 0 | 0 | 0 | 0 | 0 | 0 | 0 | 0 | 0 | M11723 | 98.9 | 175 | 1 | 1785 | 1959 |
| 1878 | 02130 | 01877 | 1 | | 0 | 0 | 0 | 1 | 0 | 0 | 0 | 0 | 0 | 0 | 0 | 0 | 0 | 0 | 0 | 0 | 0 | 0 | 0 | 0 | 0 | 0 | 0 | 0 | 0 | | | | | | |
| 1879 | 02131 | 01878 | 1 | | 0 | 0 | 0 | 1 | 0 | 0 | 0 | 0 | 0 | 0 | 0 | 0 | 0 | 0 | 0 | 0 | 0 | 0 | 0 | 0 | 0 | 0 | 0 | 0 | 0 | J03810 | 100 | 169 | 1 | 3000 | 3168 |
| 1880 | 02132 | 01879 | 2 | | 0 | 0 | 0 | 1 | 1 | 0 | 0 | 0 | 0 | 0 | 0 | 0 | 0 | 0 | 0 | 0 | 0 | 0 | 0 | 0 | 0 | 0 | 0 | 0 | 0 | | | | | | |
| 1881 | 02133 | 01880 | 2 | | 0 | 0 | 0 | 1 | 0 | 0 | 0 | 0 | 0 | 0 | 0 | 0 | 1 | 0 | 0 | 0 | 0 | 0 | 0 | 0 | 0 | 0 | 0 | 0 | 0 | | | | | | |
| 1882 | 02134 | 01881 | 1 | | 0 | 0 | 0 | 1 | 0 | 0 | 0 | 0 | 0 | 0 | 0 | 0 | 0 | 0 | 0 | 0 | 0 | 0 | 0 | 0 | 0 | 0 | 0 | 0 | 0 | | | | | | |
| 1883 | 02135 | 01882 | 6 | | 0 | 0 | 0 | 1 | 0 | 0 | 0 | 0 | 1 | 0 | 1 | 0 | 0 | 0 | 0 | 2 | 0 | 0 | 0 | 0 | 0 | 0 | 1 | 0 | 0 | | | | | | |
| 1884 | 02136 | 01883 | 1 | | 0 | 0 | 0 | 1 | 0 | 0 | 0 | 0 | 0 | 0 | 0 | 0 | 0 | 0 | 0 | 0 | 0 | 0 | 0 | 0 | 0 | 0 | 0 | 0 | 0 | | | | | | |
| 1885 | 02137 | 01884 | 4 | | 0 | 0 | 0 | 1 | 0 | 0 | 0 | 0 | 0 | 0 | 0 | 1 | 0 | 0 | 0 | 0 | 0 | 1 | 0 | 0 | 0 | 0 | 0 | 1 | 0 | | | | | | |
| 1886 | 02138 | 01885 | 2 | | 0 | 0 | 0 | 1 | 0 | 0 | 0 | 0 | 0 | 0 | 0 | 0 | 0 | 0 | 0 | 0 | 0 | 1 | 0 | 0 | 0 | 0 | 0 | 0 | 0 | | | | | | |
| 1887 | 02139 | 01886 | 1 | | 0 | 0 | 0 | 1 | 0 | 0 | 0 | 0 | 0 | 0 | 0 | 0 | 0 | 0 | 0 | 0 | 0 | 0 | 0 | 0 | 0 | 0 | 0 | 0 | 0 | | | | | | |
| 1888 | 02140 | 01887 | 2 | | 0 | 0 | 0 | 1 | 0 | 0 | 0 | 0 | 0 | 0 | 0 | 0 | 0 | 0 | 0 | 1 | 0 | 0 | 0 | 0 | 0 | 0 | 0 | 0 | 0 | | | | | | |
| 1889 | 02141 | 01888 | 8 | | 0 | 0 | 0 | 5 | 2 | 1 | 0 | 0 | 0 | 0 | 0 | 0 | 0 | 0 | 0 | 0 | 0 | 0 | 0 | 0 | 0 | 0 | 0 | 0 | 0 | M12654 | 100 | 148 | 1 | 1506 | 1653 |
| 1890 | 02142 | 01889 | 1 | | 0 | 0 | 0 | 1 | 0 | 0 | 0 | 0 | 0 | 0 | 0 | 0 | 0 | 0 | 0 | 0 | 0 | 0 | 0 | 0 | 0 | 0 | 0 | 0 | 0 | | | | | | |
| 1891 | 02143 | 01890 | 3 | | 0 | 0 | 0 | 2 | 0 | 0 | 0 | 0 | 0 | 0 | 0 | 0 | 1 | 0 | 0 | 0 | 0 | 0 | 0 | 0 | 0 | 0 | 0 | 0 | 0 | | | | | | |
| 1892 | 02144 | 01891 | 8 | | 0 | 0 | 0 | 1 | 0 | 2 | 0 | 0 | 0 | 0 | 0 | 0 | 0 | 0 | 0 | 0 | 0 | 0 | 1 | 3 | 0 | 0 | 1 | 0 | 0 | Z15108 | 95.7 | 140 | 1 | 1998 | 2146 |
| 1893 | 02145 | 01892 | 2 | | 0 | 0 | 0 | 1 | 0 | 0 | 0 | 0 | 0 | 0 | 0 | 0 | 0 | 0 | 0 | 0 | 0 | 0 | 0 | 0 | 1 | 0 | 0 | 0 | 0 | | | | | | |

Table 53

| | A | B | C | E | G | I | K | M | O | Q | S | U | W | Y | AA | AC | AE | AG | AI | AK | AM | AO | AQ | AS | AU | AW | AY | BA | BC | BE | BF | BG | BH | BI | BK |
|---|---|---|---|---|---|---|---|---|---|---|---|---|---|---|---|---|---|---|---|---|---|---|---|---|---|---|---|---|---|---|---|---|---|---|---|
| 1894 | 02146 | 01893 | 1 | | 0 | 0 | 0 | 1 | 0 | 0 | 0 | 0 | 0 | 0 | 0 | 0 | 0 | 0 | 0 | 0 | 0 | 0 | 0 | 0 | 0 | 0 | 0 | 0 | 0 | | | | | | |
| 1895 | 02147 | 01894 | 4 | | 0 | 0 | 0 | 1 | 0 | 0 | 0 | 0 | 0 | 0 | 0 | 0 | 0 | 0 | 0 | 0 | 0 | 0 | 0 | 0 | 2 | 0 | 1 | 0 | 0 | J05016 | 100 | 134 | 1 | 2732 | 2865 |
| 1896 | 02148 | 01895 | 21 | | 0 | 0 | 0 | 3 | 6 | 6 | 4 | 0 | 0 | 0 | 1 | 0 | 0 | 0 | 0 | 1 | 0 | 0 | 0 | 0 | 0 | 0 | 0 | 0 | 0 | M36501 | 100 | 127 | 1 | 1915 | 2041 |
| 1897 | 02149 | 01896 | 4 | | 0 | 0 | 0 | 1 | 0 | 2 | 0 | 0 | 0 | 0 | 1 | 0 | 0 | 0 | 0 | 0 | 0 | 0 | 0 | 0 | 0 | 0 | 0 | 0 | 0 | | | | | | |
| 1898 | 02150 | 01897 | 2 | | 0 | 0 | 0 | 0 | 0 | 1 | 0 | 0 | 0 | 0 | 0 | 0 | 0 | 0 | 0 | 1 | 0 | 0 | 0 | 0 | 0 | 0 | 0 | 0 | 0 | | | | | | |
| 1899 | 02151 | 01898 | 14 | | 0 | 0 | 0 | 9 | 3 | 2 | 0 | 0 | 0 | 0 | 0 | 0 | 0 | 0 | 0 | 0 | 0 | 0 | 0 | 0 | 0 | 0 | 0 | 0 | 0 | | | | | | |
| 1900 | 02152 | 01899 | 7 | | 0 | 0 | 0 | 1 | 0 | 4 | 1 | 0 | 0 | 0 | 0 | 0 | 0 | 0 | 1 | 0 | 0 | 0 | 0 | 0 | 0 | 0 | 0 | 0 | 0 | K02403 | 96.3 | 245 | 1 | 5155 | 5406 |
| 1901 | 02153 | 01900 | 6 | | 0 | 0 | 0 | 1 | 0 | 0 | 0 | 0 | 0 | 0 | 0 | 0 | 0 | 0 | 1 | 0 | 0 | 0 | 0 | 4 | 0 | 0 | 0 | 0 | 0 | | | | | | |
| 1902 | 02154 | 01901 | 1 | | 0 | 0 | 0 | 1 | 0 | 0 | 0 | 0 | 0 | 0 | 0 | 0 | 0 | 0 | 0 | 0 | 0 | 0 | 0 | 0 | 0 | 0 | 0 | 0 | 0 | | | | | | |
| 1903 | 02155 | 01902 | 28 | | 0 | 0 | 0 | 7 | 3 | 11 | 0 | 0 | 0 | 0 | 0 | 0 | 3 | 0 | 0 | 0 | 0 | 0 | 0 | 2 | 2 | 0 | 0 | 0 | 0 | M12530 | 100 | 124 | 1 | 2175 | 2298 |
| 1904 | 02156 | 01903 | 8 | | 0 | 0 | 0 | 1 | 0 | 0 | 2 | 0 | 0 | 2 | 0 | 0 | 0 | 0 | 1 | 1 | 0 | 0 | 0 | 0 | 0 | 0 | 1 | 0 | 0 | M86511 | 99.2 | 124 | 1 | 1204 | 1327 |
| 1905 | 02157 | 01904 | 35 | | 0 | 0 | 0 | 1 | 0 | 2 | 0 | 0 | 12 | 0 | 0 | 0 | 0 | 0 | 5 | 0 | 0 | 14 | 0 | 0 | 1 | 0 | 0 | 0 | 0 | M14083 | 100 | 89 | 1 | 2849 | 2937 |
| 1906 | 02158 | 01905 | 3 | | 0 | 0 | 0 | 1 | 0 | 0 | 0 | 0 | 0 | 0 | 1 | 0 | 0 | 0 | 0 | 0 | 0 | 1 | 0 | 0 | 0 | 0 | 0 | 0 | 0 | | | | | | |
| 1907 | 02159 | 01906 | 1 | | 0 | 0 | 0 | 1 | 0 | 0 | 0 | 0 | 0 | 0 | 0 | 0 | 0 | 0 | 0 | 0 | 0 | 0 | 0 | 0 | 0 | 0 | 0 | 0 | 0 | | | | | | |
| 1908 | 02160 | 01907 | 1 | | 0 | 0 | 0 | 1 | 0 | 0 | 0 | 0 | 0 | 0 | 0 | 0 | 0 | 0 | 0 | 0 | 0 | 0 | 0 | 0 | 0 | 0 | 0 | 0 | 0 | | | | | | |
| 1909 | 02161 | 01908 | 9 | | 0 | 0 | 0 | 1 | 0 | 0 | 0 | 0 | 1 | 0 | 0 | 0 | 0 | 1 | 0 | 0 | 0 | 0 | 1 | 1 | 0 | 0 | 4 | 0 | 0 | M91029 | 100 | 110 | 1 | 3250 | 3359 |
| 1910 | 02162 | 01909 | 1 | | 0 | 0 | 0 | 1 | 0 | 0 | 0 | 0 | 0 | 0 | 0 | 0 | 0 | 0 | 0 | 0 | 0 | 0 | 0 | 0 | 0 | 0 | 0 | 0 | 0 | | | | | | |
| 1911 | 02163 | 01910 | 4 | | 0 | 0 | 0 | 1 | 0 | 0 | 0 | 0 | 0 | 0 | 0 | 0 | 0 | 0 | 0 | 0 | 2 | 0 | 0 | 0 | 1 | 0 | 0 | 0 | 0 | | | | | | |
| 1912 | 02164 | 01911 | 5 | | 0 | 0 | 0 | 1 | 0 | 0 | 0 | 0 | 0 | 0 | 0 | 0 | 0 | 0 | 0 | 0 | 1 | 0 | 0 | 0 | 0 | 1 | 0 | 0 | 2 | | | | | | |
| 1913 | 02165 | 01912 | 1 | | 0 | 0 | 0 | 1 | 0 | 0 | 0 | 0 | 0 | 0 | 0 | 0 | 0 | 0 | 0 | 0 | 0 | 0 | 0 | 0 | 0 | 0 | 0 | 0 | 0 | | | | | | |
| 1914 | 02166 | 01913 | 4 | | 0 | 0 | 0 | 1 | 0 | 0 | 0 | 0 | 0 | 0 | 1 | 0 | 0 | 0 | 1 | 0 | 0 | 0 | 0 | 1 | 0 | 0 | 0 | 0 | 0 | | | | | | |
| 1915 | 02167 | 01914 | 1 | | 0 | 0 | 0 | 1 | 0 | 0 | 0 | 0 | 0 | 0 | 0 | 0 | 0 | 0 | 0 | 0 | 0 | 0 | 0 | 0 | 0 | 0 | 0 | 0 | 0 | X02761 | 99 | 101 | 6 | 7470 | 7680 |
| 1916 | 02168 | 01915 | 1 | | 0 | 0 | 0 | 1 | 0 | 0 | 0 | 0 | 0 | 0 | 0 | 0 | 0 | 0 | 0 | 0 | 0 | 0 | 0 | 0 | 0 | 0 | 0 | 0 | 0 | | | | | | |
| 1917 | 02169 | 01916 | 9 | | 0 | 0 | 0 | 2 | 0 | 2 | 0 | 1 | 0 | 0 | 0 | 0 | 0 | 0 | 0 | 0 | 0 | 0 | 0 | 0 | 4 | 0 | 0 | 0 | 0 | | | | | | |
| 1918 | 02170 | 01917 | 3 | | 0 | 0 | 0 | 1 | 0 | 0 | 0 | 0 | 0 | 0 | 0 | 0 | 0 | 0 | 0 | 2 | 0 | 0 | 0 | 0 | 0 | 0 | 0 | 0 | 0 | | | | | | |
| 1919 | 02171 | 01918 | 6 | | 0 | 0 | 0 | 1 | 0 | 0 | 0 | 0 | 1 | 0 | 0 | 0 | 0 | 0 | 0 | 0 | 0 | 0 | 1 | 1 | 0 | 1 | 0 | 1 | 0 | | | | | | |
| 1920 | 02172 | 01919 | 4 | | 0 | 0 | 0 | 2 | 0 | 0 | 0 | 0 | 0 | 0 | 0 | 0 | 0 | 0 | 0 | 0 | 0 | 0 | 0 | 1 | 1 | 0 | 0 | 0 | 0 | | | | | | |
| 1921 | 02173 | 01920 | 2 | | 0 | 0 | 0 | 1 | 0 | 0 | 0 | 0 | 0 | 0 | 0 | 1 | 0 | 0 | 0 | 0 | 0 | 0 | 0 | 0 | 0 | 0 | 0 | 0 | 0 | X75621 | 96.6 | 268 | 1 | 5208 | 5474 |
| 1922 | 02174 | 01921 | 25 | | 0 | 0 | 0 | 4 | 1 | 20 | 0 | 0 | 0 | 0 | 0 | 0 | 0 | 0 | 0 | 0 | 0 | 0 | 0 | 0 | 0 | 0 | 0 | 0 | 0 | M13692 | 100 | 101 | 1 | 637 | 737 |
| 1923 | 02175 | 01922 | 4 | | 0 | 0 | 0 | 1 | 0 | 0 | 0 | 2 | 0 | 0 | 0 | 0 | 0 | 0 | 0 | 0 | 0 | 0 | 0 | 0 | 1 | 0 | 0 | 0 | 0 | | | | | | |
| 1924 | 02176 | 01923 | 13 | | 0 | 0 | 0 | 3 | 1 | 9 | 0 | 0 | 0 | 0 | 0 | 0 | 0 | 0 | 0 | 0 | 0 | 0 | 0 | 0 | 0 | 0 | 0 | 0 | 0 | M29844 | 96.9 | 96 | 1 | 325 | 420 |
| 1925 | 02177 | 01924 | 7 | | 0 | 0 | 0 | 1 | 0 | 0 | 0 | 0 | 0 | 0 | 3 | 1 | 1 | 0 | 0 | 0 | 1 | 0 | 0 | 0 | 0 | 0 | 0 | 0 | 0 | | | | | | |
| 1926 | 02178 | 01925 | 1 | | 0 | 0 | 0 | 1 | 0 | 0 | 0 | 0 | 0 | 0 | 0 | 0 | 0 | 0 | 0 | 0 | 0 | 0 | 0 | 0 | 0 | 0 | 0 | 0 | 0 | | | | | | |
| 1927 | 02179 | 01926 | 2 | | 0 | 0 | 0 | 1 | 0 | 0 | 0 | 0 | 0 | 1 | 0 | 0 | 0 | 0 | 0 | 0 | 0 | 0 | 0 | 0 | 0 | 0 | 0 | 0 | 0 | | | | | | |
| 1928 | 02180 | 01927 | 1 | | 0 | 0 | 0 | 1 | 0 | 0 | 0 | 0 | 0 | 0 | 0 | 0 | 0 | 0 | 0 | 0 | 0 | 0 | 0 | 0 | 0 | 0 | 0 | 0 | 0 | | | | | | |
| 1929 | 02181 | 01928 | 14 | | 0 | 0 | 0 | 1 | 0 | 0 | 0 | 0 | 1 | 2 | 1 | 1 | 0 | 0 | 2 | 1 | 0 | 3 | 0 | 1 | 0 | 0 | 1 | 0 | 0 | | | | | | |

Table 54

| | A | B | C | E | G | I | K | M | O | Q | S | U | W | Y | AA | AC | AE | AG | AI | AK | AM | AO | AQ | AS | AU | AW | AY | BA | BC | BE | BF | BG | BH | BI | BK |
|---|---|---|---|---|---|---|---|---|---|---|---|---|---|---|---|---|---|---|---|---|---|---|---|---|---|---|---|---|---|---|---|---|---|---|---|
| 1930 | 02182 | 01929 | 3 | | 0 | 0 | 0 | 1 | 2 | 0 | 0 | 0 | 0 | 0 | 0 | 0 | 0 | 0 | 0 | 0 | 0 | 0 | 0 | 0 | 0 | 0 | 0 | 0 | 0 | | | | | | |
| 1931 | 02183 | 01930 | 3 | | 0 | 0 | 0 | 1 | 0 | 0 | 0 | 0 | 0 | 0 | 0 | 0 | 0 | 0 | 0 | 0 | 0 | 0 | 0 | 0 | 0 | 1 | 0 | 1 | 0 | | | | | | |
| 1932 | 02184 | 01931 | 1 | | 0 | 0 | 0 | 1 | 0 | 0 | 0 | 0 | 0 | 0 | 0 | 0 | 0 | 0 | 0 | 0 | 0 | 0 | 0 | 0 | 0 | 0 | 0 | 0 | 0 | | | | | | |
| 1933 | 02185 | 01932 | 3 | | 0 | 0 | 0 | 1 | 0 | 0 | 0 | 0 | 0 | 1 | 0 | 0 | 0 | 0 | 1 | 0 | 0 | 0 | 0 | 0 | 0 | 0 | 0 | 0 | 0 | | | | | | |
| 1934 | 02186 | 01933 | 68 | | 0 | 0 | 0 | 1 | 1 | 2 | 0 | 0 | 2 | 1 | 0 | 0 | 2 | 1 | 0 | 2 | 0 | 1 | 8 | 5 | 5 | 37 | 0 | 0 | 0 | X14723 | 96 | 99 | 1 | 1551 | 1676 |
| 1935 | 02187 | 01934 | 1 | | 0 | 0 | 0 | 1 | 0 | 0 | 0 | 0 | 0 | 0 | 0 | 0 | 0 | 0 | 0 | 0 | 0 | 0 | 0 | 0 | 0 | 0 | 0 | 0 | 0 | | | | | | |
| 1936 | 02188 | 01935 | 2 | | 0 | 0 | 0 | 1 | 0 | 0 | 0 | 0 | 0 | 0 | 0 | 0 | 0 | 0 | 0 | 0 | 0 | 0 | 0 | 0 | 0 | 0 | 1 | 0 | 0 | | | | | | |
| 1937 | 02189 | 01936 | 11 | | 0 | 0 | 0 | 1 | 0 | 0 | 0 | 0 | 0 | 0 | 0 | 1 | 0 | 2 | 0 | 1 | 0 | 0 | 1 | 1 | 4 | 0 | 0 | 0 | 0 | | | | | | |
| 1938 | 02191 | 01937 | 6 | | 0 | 0 | 0 | 4 | 0 | 2 | 0 | 0 | 0 | 0 | 0 | 0 | 0 | 0 | 0 | 0 | 0 | 0 | 0 | 0 | 0 | 0 | 0 | 0 | 0 | X04608 | 98.8 | 83 | 1 | 842 | 923 |
| 1939 | 02192 | 01938 | 2 | | 0 | 0 | 0 | 1 | 0 | 1 | 0 | 0 | 0 | 0 | 0 | 0 | 0 | 0 | 0 | 0 | 0 | 0 | 0 | 0 | 0 | 0 | 0 | 0 | 0 | | | | | | |
| 1940 | 02193 | 01939 | 3 | | 0 | 0 | 0 | 1 | 0 | 0 | 0 | 0 | 0 | 0 | 0 | 0 | 0 | 0 | 1 | 0 | 0 | 0 | 0 | 0 | 0 | 1 | 0 | 0 | 0 | U02493 | 97.8 | 91 | 1 | 1606 | 2593 |
| 1941 | 02194 | 01940 | 2 | | 0 | 0 | 0 | 2 | 0 | 0 | 0 | 0 | 0 | 0 | 0 | 0 | 0 | 0 | 0 | 0 | 0 | 0 | 0 | 0 | 0 | 0 | 0 | 0 | 0 | M86752 | 97.3 | 73 | 11 | 2040 | 2113 |
| 1942 | 02195 | 01941 | 8 | | 0 | 0 | 0 | 4 | 2 | 2 | 0 | 0 | 0 | 0 | 0 | 0 | 0 | 0 | 0 | 0 | 0 | 0 | 0 | 0 | 0 | 0 | 0 | 0 | 0 | | | | | | |
| 1943 | 02196 | 01942 | 1 | | 0 | 0 | 0 | 1 | 0 | 0 | 0 | 0 | 0 | 0 | 0 | 0 | 0 | 0 | 0 | 0 | 0 | 0 | 0 | 0 | 0 | 0 | 0 | 0 | 0 | | | | | | |
| 1944 | 02197 | 01943 | 1 | | 0 | 0 | 0 | 1 | 0 | 0 | 0 | 0 | 0 | 0 | 0 | 0 | 0 | 0 | 0 | 0 | 0 | 0 | 0 | 0 | 0 | 0 | 0 | 0 | 0 | | | | | | |
| 1945 | 02198 | 01944 | 2 | | 0 | 0 | 0 | 1 | 0 | 0 | 0 | 0 | 0 | 0 | 0 | 0 | 0 | 0 | 0 | 0 | 0 | 0 | 0 | 0 | 0 | 1 | 0 | 0 | 0 | | | | | | |
| 1946 | 02199 | 01945 | 5 | | 0 | 0 | 0 | 2 | 0 | 0 | 0 | 0 | 0 | 0 | 0 | 0 | 0 | 0 | 1 | 0 | 0 | 0 | 0 | 1 | 0 | 0 | 0 | 1 | 0 | L19501 | 100 | 76 | 1 | 2451 | 2544 |
| 1947 | 02200 | 01946 | 6 | | 0 | 0 | 0 | 1 | 0 | 0 | 0 | 0 | 0 | 0 | 0 | 0 | 0 | 0 | 0 | 3 | 0 | 0 | 0 | 0 | 0 | 1 | 1 | 0 | 0 | | | | | | |
| 1948 | 02202 | 01947 | 32 | | 0 | 0 | 0 | 15 | 12 | 5 | 0 | 0 | 0 | 0 | 0 | 0 | 0 | 0 | 0 | 0 | 0 | 0 | 0 | 0 | 0 | 0 | 0 | 0 | 0 | M64983 | 100 | 60 | 1 | 8480 | 8878 |
| 1949 | 02203 | 01948 | 1 | | 0 | 0 | 0 | 1 | 0 | 0 | 0 | 0 | 0 | 0 | 0 | 0 | 0 | 0 | 0 | 0 | 0 | 0 | 0 | 0 | 0 | 0 | 0 | 0 | 0 | | | | | | |
| 1950 | 02204 | 01949 | 3 | | 0 | 0 | 0 | 1 | 0 | 0 | 0 | 0 | 1 | 0 | 0 | 0 | 0 | 0 | 0 | 1 | 0 | 0 | 0 | 0 | 0 | 0 | 0 | 0 | 0 | | | | | | |
| 1951 | 02205 | 01950 | 1 | | 0 | 0 | 0 | 1 | 0 | 0 | 0 | 0 | 0 | 0 | 0 | 0 | 0 | 0 | 0 | 0 | 0 | 0 | 0 | 0 | 0 | 0 | 0 | 0 | 0 | | | | | | |
| 1952 | 02206 | 01951 | 4 | | 0 | 0 | 0 | 2 | 0 | 0 | 0 | 0 | 0 | 0 | 1 | 0 | 0 | 0 | 0 | 0 | 0 | 0 | 0 | 1 | 0 | 0 | 0 | 0 | 0 | | | | | | |
| 1953 | 02207 | 01952 | 1 | | 0 | 0 | 0 | 1 | 0 | 0 | 0 | 0 | 0 | 0 | 0 | 0 | 0 | 0 | 0 | 0 | 0 | 0 | 0 | 0 | 0 | 0 | 0 | 0 | 0 | | | | | | |
| 1954 | 02208 | 01953 | 2 | | 0 | 0 | 0 | 1 | 0 | 0 | 0 | 0 | 0 | 0 | 0 | 0 | 0 | 0 | 0 | 0 | 0 | 0 | 0 | 1 | 0 | 0 | 0 | 0 | 0 | | | | | | |
| 1955 | 02209 | 01954 | 1 | | 0 | 0 | 0 | 1 | 0 | 0 | 0 | 0 | 0 | 0 | 0 | 0 | 0 | 0 | 0 | 0 | 0 | 0 | 0 | 0 | 0 | 0 | 0 | 0 | 0 | | | | | | |
| 1956 | 02210 | 01955 | 3 | | 0 | 0 | 0 | 1 | 0 | 0 | 0 | 1 | 0 | 0 | 0 | 0 | 0 | 0 | 0 | 0 | 0 | 0 | 1 | 0 | 0 | 0 | 0 | 0 | 0 | | | | | | |
| 1957 | 02211 | 01956 | 1 | | 0 | 0 | 0 | 1 | 0 | 0 | 0 | 0 | 0 | 0 | 0 | 0 | 0 | 0 | 0 | 0 | 0 | 0 | 0 | 0 | 0 | 0 | 0 | 0 | 0 | | | | | | |
| 1958 | 02212 | 01957 | 2 | | 0 | 0 | 0 | 1 | 1 | 0 | 0 | 0 | 0 | 0 | 0 | 0 | 0 | 0 | 0 | 0 | 0 | 0 | 0 | 0 | 0 | 0 | 0 | 0 | 0 | M35433 | 98.2 | 57 | 1 | 143 | 282 |
| 1959 | 02213 | 01958 | 1 | | 0 | 0 | 0 | 1 | 0 | 0 | 0 | 0 | 0 | 0 | 0 | 0 | 0 | 0 | 0 | 0 | 0 | 0 | 0 | 0 | 0 | 0 | 0 | 0 | 0 | | | | | | |
| 1960 | 02214 | 01959 | 1 | | 0 | 0 | 0 | 1 | 0 | 0 | 0 | 0 | 0 | 0 | 0 | 0 | 0 | 0 | 0 | 0 | 0 | 0 | 0 | 0 | 0 | 0 | 0 | 0 | 0 | | | | | | |
| 1961 | 02215 | 01960 | 4 | | 0 | 0 | 0 | 1 | 0 | 0 | 0 | 1 | 0 | 0 | 0 | 0 | 0 | 0 | 1 | 0 | 1 | 0 | 0 | 0 | 0 | 0 | 0 | 0 | 0 | | | | | | |
| 1962 | 02216 | 01961 | 2 | | 0 | 0 | 0 | 1 | 1 | 0 | 0 | 0 | 0 | 0 | 0 | 0 | 0 | 0 | 0 | 0 | 0 | 0 | 0 | 0 | 0 | 0 | 0 | 0 | 0 | | | | | | |
| 1963 | 02237 | 01962 | 1 | | 0 | 0 | 0 | 0 | 1 | 0 | 0 | 0 | 0 | 0 | 0 | 0 | 0 | 0 | 0 | 0 | 0 | 0 | 0 | 0 | 0 | 0 | 0 | 0 | 0 | D90282 | 93.1 | 101 | 1 | 5116 | 5215 |
| 1964 | 02238 | 01963 | 1 | | 0 | 0 | 0 | 0 | 1 | 0 | 0 | 0 | 0 | 0 | 0 | 0 | 0 | 0 | 0 | 0 | 0 | 0 | 0 | 0 | 0 | 0 | 0 | 0 | 0 | | | | | | |
| 1965 | 02239 | 01964 | 6 | | 0 | 0 | 0 | 0 | 1 | 0 | 1 | 0 | 0 | 1 | 0 | 0 | 0 | 0 | 1 | 0 | 0 | 2 | 0 | 0 | 0 | 0 | 0 | 0 | 0 | X51745 | 97.3 | 447 | 1 | 964 | 1418 |

Table 55

| | A | B | C | E | G | I | K | M | O | Q | S | U | W | Y | AA | AC | AE | AG | AI | AK | AM | AO | AQ | AS | AU | AW | AY | BA | BC | BE | BF | BG | BH | BI | BK |
|---|---|---|---|---|---|---|---|---|---|---|---|---|---|---|---|---|---|---|---|---|---|---|---|---|---|---|---|---|---|---|---|---|---|---|---|
| 1966 | 02240 | 01965 | 5 | | 0 | 0 | 0 | 0 | 1 | 0 | 0 | 0 | 0 | 0 | 1 | 1 | 0 | 0 | 0 | 0 | 0 | 0 | 1 | 0 | 0 | 0 | 0 | 1 | 0 | | | | | | |
| 1967 | 02241 | 01966 | 1 | | 0 | 0 | 0 | 0 | 1 | 0 | 0 | 0 | 0 | 0 | 0 | 0 | 0 | 0 | 0 | 0 | 0 | 0 | 0 | 0 | 0 | 0 | 0 | 0 | 0 | | | | | | |
| 1968 | 02242 | 01967 | 1 | | 0 | 0 | 0 | 0 | 1 | 0 | 0 | 0 | 0 | 0 | 0 | 0 | 0 | 0 | 0 | 0 | 0 | 0 | 0 | 0 | 0 | 0 | 0 | 0 | 0 | X60364 | 98.1 | 416 | 1 | 1523 | 1941 |
| 1969 | 02243 | 01968 | 1 | | 0 | 0 | 0 | 0 | 1 | 0 | 0 | 0 | 0 | 0 | 0 | 0 | 0 | 0 | 0 | 0 | 0 | 0 | 0 | 0 | 0 | 0 | 0 | 0 | 0 | | | | | | |
| 1970 | 02244 | 01969 | 1 | | 0 | 0 | 0 | 0 | 1 | 0 | 0 | 0 | 0 | 0 | 0 | 0 | 0 | 0 | 0 | 0 | 0 | 0 | 0 | 0 | 0 | 0 | 0 | 0 | 0 | D26124 | 92.5 | 399 | 1 | 610 | 1162 |
| 1971 | 02245 | 01970 | 1 | | 0 | 0 | 0 | 0 | 1 | 0 | 0 | 0 | 0 | 0 | 0 | 0 | 0 | 0 | 0 | 0 | 0 | 0 | 0 | 0 | 0 | 0 | 0 | 0 | 0 | | | | | | |
| 1972 | 02246 | 01971 | 2 | | 0 | 0 | 0 | 0 | 1 | 0 | 0 | 0 | 0 | 0 | 0 | 0 | 0 | 1 | 0 | 0 | 0 | 0 | 0 | 0 | 0 | 0 | 0 | 0 | 0 | | | | | | |
| 1973 | 02247 | 01972 | 1 | | 0 | 0 | 0 | 0 | 1 | 0 | 0 | 0 | 0 | 0 | 0 | 0 | 0 | 0 | 0 | 0 | 0 | 0 | 0 | 0 | 0 | 0 | 0 | 0 | 0 | M14091 | 93.7 | 380 | 1 | 1496 | 1872 |
| 1974 | 02248 | 01973 | 1 | | 0 | 0 | 0 | 0 | 1 | 0 | 0 | 0 | 0 | 0 | 0 | 0 | 0 | 0 | 0 | 0 | 0 | 0 | 0 | 0 | 0 | 0 | 0 | 0 | 0 | | | | | | |
| 1975 | 02249 | 01974 | 1 | | 0 | 0 | 0 | 0 | 1 | 0 | 0 | 0 | 0 | 0 | 0 | 0 | 0 | 0 | 0 | 0 | 0 | 0 | 0 | 0 | 0 | 0 | 0 | 0 | 0 | X67055 | 92.8 | 374 | 1 | 2279 | 2807 |
| 1976 | 02250 | 01975 | 2 | | 0 | 0 | 0 | 0 | 1 | 0 | 0 | 0 | 0 | 0 | 0 | 0 | 0 | 0 | 0 | 0 | 0 | 1 | 0 | 0 | 0 | 0 | 0 | 0 | 0 | | | | | | |
| 1977 | 02252 | 01976 | 1 | | 0 | 0 | 0 | 0 | 1 | 0 | 0 | 0 | 0 | 0 | 0 | 0 | 0 | 0 | 0 | 0 | 0 | 0 | 0 | 0 | 0 | 0 | 0 | 0 | 0 | | | | | | |
| 1978 | 02254 | 01977 | 3 | | 0 | 0 | 0 | 0 | 2 | 0 | 0 | 0 | 0 | 0 | 0 | 0 | 0 | 0 | 0 | 0 | 0 | 0 | 0 | 1 | 0 | 0 | 0 | 0 | 0 | K00484 | 91.6 | 275 | 1 | 48 | 333 |
| 1979 | 02255 | 01978 | 2 | | 0 | 0 | 0 | 0 | 1 | 0 | 0 | 0 | 0 | 0 | 0 | 0 | 0 | 0 | 1 | 0 | 0 | 0 | 0 | 0 | 0 | 0 | 0 | 0 | 0 | | | | | | |
| 1980 | 02256 | 01979 | 2 | | 0 | 0 | 0 | 0 | 1 | 0 | 0 | 0 | 0 | 0 | 0 | 0 | 0 | 0 | 0 | 1 | 0 | 0 | 0 | 0 | 0 | 0 | 0 | 0 | 0 | M76766 | 99.1 | 339 | 1 | 930 | 1268 |
| 1981 | 02257 | 01980 | 1 | | 0 | 0 | 0 | 0 | 1 | 0 | 0 | 0 | 0 | 0 | 0 | 0 | 0 | 0 | 0 | 0 | 0 | 0 | 0 | 0 | 0 | 0 | 0 | 0 | 0 | X04225 | 97.6 | 210 | 1 | 306 | 1239 |
| 1982 | 02258 | 01981 | 1 | | 0 | 0 | 0 | 0 | 1 | 0 | 0 | 0 | 0 | 0 | 0 | 0 | 0 | 0 | 0 | 0 | 0 | 0 | 0 | 0 | 0 | 0 | 0 | 0 | 0 | | | | | | |
| 1983 | 02259 | 01982 | 10 | | 0 | 0 | 0 | 0 | 1 | 0 | 0 | 1 | 0 | 1 | 1 | 0 | 0 | 1 | 0 | 0 | 0 | 0 | 0 | 1 | 4 | 0 | 0 | 0 | 0 | Y00109 | 94 | 332 | 1 | 1651 | 1980 |
| 1984 | 02260 | 01983 | 1 | | 0 | 0 | 0 | 0 | 1 | 0 | 0 | 0 | 0 | 0 | 0 | 0 | 0 | 0 | 0 | 0 | 0 | 0 | 0 | 0 | 0 | 0 | 0 | 0 | 0 | | | | | | |
| 1985 | 02261 | 01984 | 2 | | 0 | 0 | 0 | 0 | 1 | 0 | 0 | 0 | 0 | 0 | 0 | 0 | 0 | 1 | 0 | 0 | 0 | 0 | 0 | 0 | 0 | 0 | 0 | 0 | 0 | | | | | | |
| 1986 | 02262 | 01985 | 1 | | 0 | 0 | 0 | 0 | 1 | 0 | 0 | 0 | 0 | 0 | 0 | 0 | 0 | 0 | 0 | 0 | 0 | 0 | 0 | 0 | 0 | 0 | 0 | 0 | 0 | M28614 | 99.6 | 262 | 64 | 83 | 519 |
| 1987 | 02263 | 01986 | 4 | | 0 | 0 | 0 | 0 | 1 | 0 | 0 | 0 | 0 | 0 | 0 | 0 | 1 | 0 | 0 | 0 | 1 | 0 | 0 | 0 | 0 | 0 | 0 | 1 | 0 | | | | | | |
| 1988 | 02264 | 01987 | 1 | | 0 | 0 | 0 | 0 | 1 | 0 | 0 | 0 | 0 | 0 | 0 | 0 | 0 | 0 | 0 | 0 | 0 | 0 | 0 | 0 | 0 | 0 | 0 | 0 | 0 | | | | | | |
| 1989 | 02266 | 01988 | 1 | | 0 | 0 | 0 | 0 | 1 | 0 | 0 | 0 | 0 | 0 | 0 | 0 | 0 | 0 | 0 | 0 | 0 | 0 | 0 | 0 | 0 | 0 | 0 | 0 | 0 | | | | | | |
| 1990 | 02268 | 01989 | 1 | | 0 | 0 | 0 | 0 | 1 | 0 | 0 | 0 | 0 | 0 | 0 | 0 | 0 | 0 | 0 | 0 | 0 | 0 | 0 | 0 | 0 | 0 | 0 | 0 | 0 | | | | | | |
| 1991 | 02269 | 01990 | 4 | | 0 | 0 | 0 | 0 | 1 | 1 | 1 | 0 | 0 | 0 | 0 | 0 | 0 | 0 | 1 | 0 | 0 | 0 | 0 | 0 | 0 | 0 | 0 | 0 | 0 | M19922 | 98.7 | 233 | 1 | 1149 | 1382 |
| 1992 | 02270 | 01991 | 3 | | 0 | 0 | 0 | 0 | 1 | 0 | 0 | 1 | 0 | 0 | 0 | 0 | 0 | 0 | 0 | 0 | 0 | 0 | 0 | 0 | 0 | 1 | 0 | 0 | 0 | | | | | | |
| 1993 | 02271 | 01992 | 12 | | 0 | 0 | 0 | 0 | 1 | 0 | 0 | 0 | 0 | 0 | 5 | 0 | 0 | 0 | 2 | 1 | 0 | 0 | 0 | 0 | 1 | 2 | 0 | 0 | 0 | M77025 | 95.7 | 303 | 1 | 1100 | 1415 |
| 1994 | 02272 | 01993 | 9 | | 0 | 0 | 0 | 0 | 2 | 0 | 2 | 0 | 0 | 0 | 0 | 2 | 0 | 0 | 3 | 0 | 0 | 0 | 0 | 0 | 0 | 0 | 0 | 0 | 0 | X00457 | 92.5 | 308 | 1 | 752 | 1048 |
| 1995 | 02274 | 01994 | 2 | | 0 | 0 | 0 | 0 | 1 | 0 | 0 | 0 | 0 | 0 | 0 | 1 | 0 | 0 | 0 | 0 | 0 | 0 | 0 | 0 | 0 | 0 | 0 | 0 | 0 | S46622 | 97 | 297 | 1 | 1821 | 2134 |
| 1996 | 02275 | 01995 | 2 | | 0 | 0 | 0 | 0 | 1 | 1 | 0 | 0 | 0 | 0 | 0 | 0 | 0 | 0 | 0 | 0 | 0 | 0 | 0 | 0 | 0 | 0 | 0 | 0 | 0 | | | | | | |
| 1997 | 02276 | 01996 | 1 | | 0 | 0 | 0 | 0 | 1 | 0 | 0 | 0 | 0 | 0 | 0 | 0 | 0 | 0 | 0 | 0 | 0 | 0 | 0 | 0 | 0 | 0 | 0 | 0 | 0 | M63175 | 97.4 | 192 | 1 | 655 | 1810 |
| 1998 | 02277 | 01997 | 1 | | 0 | 0 | 0 | 0 | 1 | 0 | 0 | 0 | 0 | 0 | 0 | 0 | 0 | 0 | 0 | 0 | 0 | 0 | 0 | 0 | 0 | 0 | 0 | 0 | 0 | | | | | | |
| 1999 | 02278 | 01998 | 1 | | 0 | 0 | 0 | 0 | 1 | 0 | 0 | 0 | 0 | 0 | 0 | 0 | 0 | 0 | 0 | 0 | 0 | 0 | 0 | 0 | 0 | 0 | 0 | 0 | 0 | | | | | | |
| 2000 | 02279 | 01999 | 1 | | 0 | 0 | 0 | 0 | 1 | 0 | 0 | 0 | 0 | 0 | 0 | 0 | 0 | 0 | 0 | 0 | 0 | 0 | 0 | 0 | 0 | 0 | 0 | 0 | 0 | J00127 | 93.6 | 188 | 83 | 1988 | 2182 |
| 2001 | 02280 | 02000 | 3 | | 0 | 0 | 0 | 0 | 1 | 0 | 1 | 0 | 0 | 0 | 0 | 0 | 0 | 0 | 0 | 1 | 0 | 0 | 0 | 0 | 0 | 0 | 0 | 0 | 0 | | | | | | |

Table 56

| | A | B | C | E | G | I | K | M | O | Q | S | U | W | Y | AA | AC | AE | AG | AI | AK | AM | AO | AQ | AS | AU | AW | AY | BA | BC | BE | BF | BG | BH | BI | BK |
|---|---|---|---|---|---|---|---|---|---|---|---|---|---|---|---|---|---|---|---|---|---|---|---|---|---|---|---|---|---|---|---|---|---|---|---|
| 2002 | 02281 | 02001 | 1 | | 0 | 0 | 0 | 0 | 1 | 0 | 0 | 0 | 0 | 0 | 0 | 0 | 0 | 0 | 0 | 0 | 0 | 0 | 0 | 0 | 0 | 0 | 0 | 0 | 0 | | | | | | |
| 2003 | 02282 | 02002 | 1 | | 0 | 0 | 0 | 0 | 1 | 0 | 0 | 0 | 0 | 0 | 0 | 0 | 0 | 0 | 0 | 0 | 0 | 0 | 0 | 0 | 0 | 0 | 0 | 0 | 0 | | | | | | |
| 2004 | 02283 | 02003 | 1 | | 0 | 0 | 0 | 0 | 1 | 0 | 0 | 0 | 0 | 0 | 0 | 0 | 0 | 0 | 0 | 0 | 0 | 0 | 0 | 0 | 0 | 0 | 0 | 0 | 0 | X58529 | 95.1 | 264 | 1 | 2068 | 2325 |
| 2005 | 02285 | 02004 | 10 | | 0 | 0 | 0 | 0 | 1 | 0 | 0 | 0 | 0 | 1 | 0 | 0 | 0 | 0 | 3 | 0 | 0 | 4 | 0 | 0 | 1 | 0 | 0 | 0 | 0 | J03464 | 94.8 | 404 | 1 | 4976 | 5416 |
| 2006 | 02287 | 02005 | 1 | | 0 | 0 | 0 | 0 | 1 | 0 | 0 | 0 | 0 | 0 | 0 | 0 | 0 | 0 | 0 | 0 | 0 | 0 | 0 | 0 | 0 | 0 | 0 | 0 | 0 | M61763 | 99.6 | 240 | 1 | 207 | 612 |
| 2007 | 02288 | 02006 | 1 | | 0 | 0 | 0 | 0 | 1 | 0 | 0 | 0 | 0 | 0 | 0 | 0 | 0 | 0 | 0 | 0 | 0 | 0 | 0 | 0 | 0 | 0 | 0 | 0 | 0 | | | | | | |
| 2008 | 02289 | 02007 | 1 | | 0 | 0 | 0 | 0 | 1 | 0 | 0 | 0 | 0 | 0 | 0 | 0 | 0 | 0 | 0 | 0 | 0 | 0 | 0 | 0 | 0 | 0 | 0 | 0 | 0 | | | | | | |
| 2009 | 02290 | 02008 | 16 | | 0 | 0 | 0 | 0 | 16 | 0 | 0 | 0 | 0 | 0 | 0 | 0 | 0 | 0 | 0 | 0 | 0 | 0 | 0 | 0 | 0 | 0 | 0 | 0 | 0 | M15386 | 94.3 | 230 | 1 | 315 | 545 |
| 2010 | 02291 | 02009 | 1 | | 0 | 0 | 0 | 0 | 1 | 0 | 0 | 0 | 0 | 0 | 0 | 0 | 0 | 0 | 0 | 0 | 0 | 0 | 0 | 0 | 0 | 0 | 0 | 0 | 0 | S74678 | 97.5 | 80 | 1 | 1406 | 2302 |
| 2011 | 02292 | 02010 | 1 | | 0 | 0 | 0 | 0 | 1 | 0 | 0 | 0 | 0 | 0 | 0 | 0 | 0 | 0 | 0 | 0 | 0 | 0 | 0 | 0 | 0 | 0 | 0 | 0 | 0 | M15386 | 93.1 | 231 | 1 | 315 | 545 |
| 2012 | 02293 | 02011 | 1 | | 0 | 0 | 0 | 0 | 1 | 0 | 0 | 0 | 0 | 0 | 0 | 0 | 0 | 0 | 0 | 0 | 0 | 0 | 0 | 0 | 0 | 0 | 0 | 0 | 0 | | | | | | |
| 2013 | 02294 | 02012 | 1 | | 0 | 0 | 0 | 0 | 1 | 0 | 0 | 0 | 0 | 0 | 0 | 0 | 0 | 0 | 0 | 0 | 0 | 0 | 0 | 0 | 0 | 0 | 0 | 0 | 0 | | | | | | |
| 2014 | 02295 | 02013 | 1 | | 0 | 0 | 0 | 0 | 1 | 0 | 0 | 0 | 0 | 0 | 0 | 0 | 0 | 0 | 0 | 0 | 0 | 0 | 0 | 0 | 0 | 0 | 0 | 0 | 0 | M17886 | 98.6 | 210 | 10 | 303 | 512 |
| 2015 | 02296 | 02014 | 2 | | 0 | 0 | 0 | 0 | 1 | 0 | 0 | 0 | 0 | 0 | 0 | 0 | 0 | 0 | 0 | 0 | 0 | 0 | 0 | 0 | 0 | 1 | 0 | 0 | 0 | | | | | | |
| 2016 | 02297 | 02015 | 2 | | 0 | 0 | 0 | 0 | 1 | 0 | 0 | 0 | 0 | 0 | 0 | 0 | 0 | 0 | 0 | 0 | 0 | 0 | 0 | 0 | 1 | 0 | 0 | 0 | 0 | | | | | | |
| 2017 | 02298 | 02016 | 1 | | 0 | 0 | 0 | 0 | 1 | 0 | 0 | 0 | 0 | 0 | 0 | 0 | 0 | 0 | 0 | 0 | 0 | 0 | 0 | 0 | 0 | 0 | 0 | 0 | 0 | | | | | | |
| 2018 | 02299 | 02017 | 4 | | 0 | 0 | 0 | 0 | 1 | 0 | 0 | 0 | 0 | 0 | 0 | 0 | 1 | 0 | 1 | 0 | 0 | 0 | 0 | 0 | 1 | 0 | 0 | 0 | 0 | | | | | | |
| 2019 | 02300 | 02018 | 3 | | 0 | 0 | 0 | 0 | 1 | 0 | 0 | 0 | 0 | 0 | 0 | 1 | 0 | 0 | 0 | 0 | 0 | 0 | 1 | 0 | 0 | 0 | 0 | 0 | 0 | | | | | | |
| 2020 | 02301 | 02019 | 1 | | 0 | 0 | 0 | 0 | 1 | 0 | 0 | 0 | 0 | 0 | 0 | 0 | 0 | 0 | 0 | 0 | 0 | 0 | 0 | 0 | 0 | 0 | 0 | 0 | 0 | M13928 | 98 | 202 | 1 | 949 | 1154 |
| 2021 | 02302 | 02020 | 5 | | 0 | 0 | 0 | 0 | 1 | 0 | 0 | 0 | 0 | 0 | 0 | 0 | 0 | 1 | 0 | 0 | 0 | 0 | 2 | 1 | 0 | 0 | 0 | 0 | 0 | | | | | | |
| 2022 | 02303 | 02021 | 1 | | 0 | 0 | 0 | 0 | 1 | 0 | 0 | 0 | 0 | 0 | 0 | 0 | 0 | 0 | 0 | 0 | 0 | 0 | 0 | 0 | 0 | 0 | 0 | 0 | 0 | | | | | | |
| 2023 | 02304 | 02022 | 1 | | 0 | 0 | 0 | 0 | 1 | 0 | 0 | 0 | 0 | 0 | 0 | 0 | 0 | 0 | 0 | 0 | 0 | 0 | 0 | 0 | 0 | 0 | 0 | 0 | 0 | | | | | | |
| 2024 | 02305 | 02023 | 2 | | 0 | 0 | 0 | 0 | 1 | 0 | 0 | 0 | 1 | 0 | 0 | 0 | 0 | 0 | 0 | 0 | 0 | 0 | 0 | 0 | 0 | 0 | 0 | 0 | 0 | | | | | | |
| 2025 | 02306 | 02024 | 1 | | 0 | 0 | 0 | 0 | 1 | 0 | 0 | 0 | 0 | 0 | 0 | 0 | 0 | 0 | 0 | 0 | 0 | 0 | 0 | 0 | 0 | 0 | 0 | 0 | 0 | | | | | | |
| 2026 | 02307 | 02025 | 1 | | 0 | 0 | 0 | 0 | 1 | 0 | 0 | 0 | 0 | 0 | 0 | 0 | 0 | 0 | 0 | 0 | 0 | 0 | 0 | 0 | 0 | 0 | 0 | 0 | 0 | | | | | | |
| 2027 | 02308 | 02026 | 3 | | 0 | 0 | 0 | 0 | 1 | 0 | 0 | 0 | 0 | 0 | 2 | 0 | 0 | 0 | 0 | 0 | 0 | 0 | 0 | 0 | 0 | 0 | 0 | 0 | 0 | | | | | | |
| 2028 | 02309 | 02027 | 18 | | 0 | 0 | 0 | 0 | 1 | 0 | 0 | 0 | 0 | 0 | 0 | 0 | 2 | 1 | 2 | 1 | 2 | 4 | 0 | 1 | 0 | 1 | 0 | 3 | 0 | X15183 | 96 | 174 | 1 | 2742 | 2912 |
| 2029 | 02310 | 02028 | 3 | | 0 | 0 | 0 | 0 | 2 | 1 | 0 | 0 | 0 | 0 | 0 | 0 | 0 | 0 | 0 | 0 | 0 | 0 | 0 | 0 | 0 | 0 | 0 | 0 | 0 | | | | | | |
| 2030 | 02311 | 02029 | 6 | | 0 | 0 | 0 | 0 | 1 | 0 | 1 | 1 | 0 | 0 | 1 | 1 | 0 | 0 | 0 | 0 | 0 | 0 | 0 | 0 | 1 | 0 | 0 | 0 | 0 | | | | | | |
| 2031 | 02312 | 02030 | 5 | | 0 | 0 | 0 | 0 | 2 | 2 | 0 | 0 | 0 | 0 | 0 | 0 | 0 | 0 | 0 | 0 | 1 | 0 | 0 | 0 | 0 | 0 | 0 | 0 | 0 | | | | | | |
| 2032 | 02313 | 02031 | 6 | | 0 | 0 | 0 | 0 | 1 | 0 | 0 | 0 | 0 | 0 | 0 | 0 | 0 | 2 | 0 | 0 | 2 | 1 | 0 | 0 | 0 | 0 | 0 | 0 | 0 | X61971 | 100 | 174 | 1 | 444 | 624 |
| 2033 | 02314 | 02032 | 2 | | 0 | 0 | 0 | 0 | 1 | 0 | 0 | 0 | 0 | 0 | 0 | 0 | 0 | 0 | 0 | 0 | 0 | 0 | 0 | 1 | 0 | 0 | 0 | 0 | 0 | | | | | | |
| 2034 | 02315 | 02033 | 1 | | 0 | 0 | 0 | 0 | 1 | 0 | 0 | 0 | 0 | 0 | 0 | 0 | 0 | 0 | 0 | 0 | 0 | 0 | 0 | 0 | 0 | 0 | 0 | 0 | 0 | | | | | | |
| 2035 | 02316 | 02034 | 12 | | 0 | 0 | 0 | 0 | 1 | 0 | 0 | 0 | 1 | 0 | 0 | 1 | 3 | 0 | 0 | 0 | 0 | 0 | 1 | 1 | 0 | 1 | 0 | 3 | 0 | M38188 | 99.4 | 164 | 1 | 728 | 891 |
| 2036 | 02317 | 02035 | 5 | | 0 | 0 | 0 | 0 | 2 | 1 | 1 | 0 | 0 | 0 | 0 | 0 | 0 | 0 | 0 | 0 | 0 | 0 | 0 | 0 | 1 | 0 | 0 | 0 | 0 | | | | | | |
| 2037 | 02318 | 02036 | 2 | | 0 | 0 | 0 | 0 | 1 | 0 | 0 | 0 | 0 | 0 | 0 | 0 | 0 | 0 | 0 | 0 | 0 | 1 | 0 | 0 | 0 | 0 | 0 | 0 | 0 | | | | | | |

Table 57

| | A | B | C | E | G | I | K | M | O | Q | S | U | W | Y | AA | AC | AF | AG | AI | AK | AM | AO | AQ | AS | AU | AW | AY | BA | BC | BE | BF | BG | BH | BI | BK |
|---|---|---|---|---|---|---|---|---|---|---|---|---|---|---|---|---|---|---|---|---|---|---|---|---|---|---|---|---|---|---|---|---|---|---|---|
| 2038 | 02319 | 02037 | 1 | | 0 | 0 | 0 | 0 | 1 | 0 | 0 | 0 | 0 | 0 | 0 | 0 | 0 | 0 | 0 | 0 | 0 | 0 | 0 | 0 | 0 | 0 | 0 | 0 | 0 | | | | | | |
| 2039 | 02321 | 02038 | 7 | | 0 | 0 | 0 | 0 | 1 | 0 | 0 | 0 | 0 | 0 | 1 | 0 | 2 | 0 | 0 | 0 | 2 | 0 | 0 | 0 | 0 | 0 | 0 | 0 | 1 | M15796 | 99.3 | 137 | 1 | 1095 | 1231 |
| 2040 | 02322 | 02039 | 1 | | 0 | 0 | 0 | 0 | 1 | 0 | 0 | 0 | 0 | 0 | 0 | 0 | 0 | 0 | 0 | 0 | 0 | 0 | 0 | 0 | 0 | 0 | 0 | 0 | 0 | M13656 | 96.1 | 129 | 10 | 1673 | 1801 |
| 2041 | 02323 | 02040 | 2 | | 0 | 0 | 0 | 0 | 1 | 0 | 0 | 0 | 0 | 0 | 0 | 1 | 0 | 0 | 0 | 0 | 0 | 0 | 0 | 0 | 0 | 0 | 0 | 0 | 0 | | | | | | |
| 2042 | 02324 | 02041 | 2 | | 0 | 0 | 0 | 0 | 1 | 1 | 0 | 0 | 0 | 0 | 0 | 0 | 0 | 0 | 0 | 0 | 0 | 0 | 0 | 0 | 0 | 0 | 0 | 0 | 0 | | | | | | |
| 2043 | 02325 | 02042 | 5 | | 0 | 0 | 0 | 0 | 1 | 1 | 0 | 0 | 1 | 0 | 1 | 0 | 0 | 0 | 0 | 0 | 1 | 0 | 0 | 0 | 0 | 0 | 0 | 0 | 0 | | | | | | |
| 2044 | 02326 | 02043 | 1 | | 0 | 0 | 0 | 0 | 1 | 0 | 0 | 0 | 0 | 0 | 0 | 0 | 0 | 0 | 0 | 0 | 0 | 0 | 0 | 0 | 0 | 0 | 0 | 0 | 0 | | | | | | |
| 2045 | 02327 | 02044 | 1 | | 0 | 0 | 0 | 0 | 1 | 0 | 0 | 0 | 0 | 0 | 0 | 0 | 0 | 0 | 0 | 0 | 0 | 0 | 0 | 0 | 0 | 0 | 0 | 0 | 0 | | | | | | |
| 2046 | 02328 | 02045 | 1 | | 0 | 0 | 0 | 0 | 1 | 0 | 0 | 0 | 0 | 0 | 0 | 0 | 0 | 0 | 0 | 0 | 0 | 0 | 0 | 0 | 0 | 0 | 0 | 0 | 0 | | | | | | |
| 2047 | 02329 | 02046 | 3 | | 0 | 0 | 0 | 0 | 1 | 0 | 0 | 0 | 0 | 0 | 0 | 0 | 0 | 0 | 0 | 0 | 0 | 1 | 1 | 0 | 0 | 0 | 0 | 0 | 0 | | | | | | |
| 2048 | 02330 | 02047 | 1 | | 0 | 0 | 0 | 0 | 1 | 0 | 0 | 0 | 0 | 0 | 0 | 0 | 0 | 0 | 0 | 0 | 0 | 0 | 0 | 0 | 0 | 0 | 0 | 0 | 0 | | | | | | |
| 2049 | 02331 | 02048 | 1 | | 0 | 0 | 0 | 0 | 1 | 0 | 0 | 0 | 0 | 0 | 0 | 0 | 0 | 0 | 0 | 0 | 0 | 0 | 0 | 0 | 0 | 0 | 0 | 0 | 0 | | | | | | |
| 2050 | 02332 | 02049 | 3 | | 0 | 0 | 0 | 0 | 1 | 0 | 0 | 0 | 0 | 0 | 0 | 0 | 0 | 0 | 0 | 0 | 0 | 0 | 0 | 0 | 0 | 1 | 0 | 1 | 0 | | | | | | |
| 2051 | 02333 | 02050 | 1 | | 0 | 0 | 0 | 0 | 1 | 0 | 0 | 0 | 0 | 0 | 0 | 0 | 0 | 0 | 0 | 0 | 0 | 0 | 0 | 0 | 0 | 0 | 0 | 0 | 0 | | | | | | |
| 2052 | 02334 | 02051 | 4 | | 0 | 0 | 0 | 0 | 1 | 0 | 0 | 0 | 0 | 0 | 0 | 0 | 0 | 0 | 0 | 0 | 0 | 0 | 2 | 1 | 0 | 0 | 0 | 0 | 0 | | | | | | |
| 2053 | 02336 | 02052 | 1 | | 0 | 0 | 0 | 0 | 0 | 0 | 0 | 0 | 0 | 1 | 0 | 0 | 0 | 0 | 0 | 0 | 0 | 0 | 0 | 0 | 0 | 0 | 0 | 0 | 0 | D11384 | 93.2 | 132 | 1 | 657 | 793 |
| 2054 | 02337 | 02053 | 2 | | 0 | 0 | 0 | 0 | 1 | 0 | 0 | 0 | 0 | 0 | 0 | 1 | 0 | 0 | 0 | 0 | 0 | 0 | 0 | 0 | 0 | 0 | 0 | 0 | 0 | | | | | | |
| 2055 | 02338 | 02054 | 3 | | 0 | 0 | 0 | 0 | 1 | 0 | 0 | 0 | 0 | 1 | 0 | 0 | 0 | 0 | 0 | 1 | 0 | 0 | 0 | 0 | 0 | 0 | 0 | 0 | 0 | | | | | | |
| 2056 | 02339 | 02055 | 1 | | 0 | 0 | 0 | 0 | 1 | 0 | 0 | 0 | 0 | 0 | 0 | 0 | 0 | 0 | 0 | 0 | 0 | 0 | 0 | 0 | 0 | 0 | 0 | 0 | 0 | | | | | | |
| 2057 | 02340 | 02056 | 1 | | 0 | 0 | 0 | 0 | 1 | 0 | 0 | 0 | 0 | 0 | 0 | 0 | 0 | 0 | 0 | 0 | 0 | 0 | 0 | 0 | 0 | 0 | 0 | 0 | 0 | J02943 | 98.3 | 121 | 1 | 1302 | 1422 |
| 2058 | 02341 | 02057 | 2 | | 0 | 0 | 0 | 0 | 1 | 0 | 0 | 0 | 0 | 0 | 0 | 0 | 0 | 0 | 0 | 0 | 0 | 0 | 0 | 0 | 0 | 1 | 0 | 0 | 0 | S50157 | 95.8 | 120 | 1 | 1017 | 1134 |
| 2059 | 02342 | 02058 | 1 | | 0 | 0 | 0 | 0 | 1 | 0 | 0 | 0 | 0 | 0 | 0 | 0 | 0 | 0 | 0 | 0 | 0 | 0 | 0 | 0 | 0 | 0 | 0 | 0 | 0 | | | | | | |
| 2060 | 02343 | 02059 | 1 | | 0 | 0 | 0 | 0 | 1 | 0 | 0 | 0 | 0 | 0 | 0 | 0 | 0 | 0 | 0 | 0 | 0 | 0 | 0 | 0 | 0 | 0 | 0 | 0 | 0 | X56352 | 94.1 | 118 | 1 | 861 | 1882 |
| 2061 | 02345 | 02060 | 5 | | 0 | 0 | 0 | 0 | 1 | 0 | 0 | 0 | 1 | 0 | 0 | 1 | 0 | 0 | 0 | 0 | 1 | 0 | 0 | 0 | 0 | 0 | 0 | 1 | 0 | M24398 | 95.9 | 122 | 1 | 991 | 1109 |
| 2062 | 02346 | 02061 | 1 | | 0 | 0 | 0 | 0 | 1 | 0 | 0 | 0 | 0 | 0 | 0 | 0 | 0 | 0 | 0 | 0 | 0 | 0 | 0 | 0 | 0 | 0 | 0 | 0 | 0 | | | | | | |
| 2063 | 02347 | 02062 | 6 | | 0 | 0 | 0 | 0 | 1 | 0 | 0 | 0 | 0 | 0 | 1 | 0 | 0 | 0 | 0 | 0 | 0 | 2 | 0 | 1 | 0 | 0 | 0 | 1 | 0 | | | | | | |
| 2064 | 02348 | 02063 | 1 | | 0 | 0 | 0 | 0 | 1 | 0 | 0 | 0 | 0 | 0 | 0 | 0 | 0 | 0 | 0 | 0 | 0 | 0 | 0 | 0 | 0 | 0 | 0 | 0 | 0 | | | | | | |
| 2065 | 02349 | 02064 | 1 | | 0 | 0 | 0 | 0 | 1 | 0 | 0 | 0 | 0 | 0 | 0 | 0 | 0 | 0 | 0 | 0 | 0 | 0 | 0 | 0 | 0 | 0 | 0 | 0 | 0 | | | | | | |
| 2066 | 02350 | 02065 | 1 | | 0 | 0 | 0 | 0 | 1 | 0 | 0 | 0 | 0 | 0 | 0 | 0 | 0 | 0 | 0 | 0 | 0 | 0 | 0 | 0 | 0 | 0 | 0 | 0 | 0 | | | | | | |
| 2067 | 02351 | 02066 | 1 | | 0 | 0 | 0 | 0 | 1 | 0 | 0 | 0 | 0 | 0 | 0 | 0 | 0 | 0 | 0 | 0 | 0 | 0 | 0 | 0 | 0 | 0 | 0 | 0 | 0 | X05607 | 90.7 | 108 | 1 | 452 | 771 |
| 2068 | 02352 | 02067 | 1 | | 0 | 0 | 0 | 0 | 1 | 0 | 0 | 0 | 0 | 0 | 0 | 0 | 0 | 0 | 0 | 0 | 0 | 0 | 0 | 0 | 0 | 0 | 0 | 0 | 0 | M16973 | 92.5 | 107 | 1 | 1610 | 1995 |
| 2069 | 02353 | 02068 | 2 | | 0 | 0 | 0 | 0 | 1 | 0 | 0 | 0 | 0 | 0 | 0 | 0 | 0 | 0 | 0 | 1 | 0 | 0 | 0 | 0 | 0 | 0 | 0 | 0 | 0 | | | | | | |
| 2070 | 02354 | 02069 | 1 | | 0 | 0 | 0 | 0 | 1 | 0 | 0 | 0 | 0 | 0 | 0 | 0 | 0 | 0 | 0 | 0 | 0 | 0 | 0 | 0 | 0 | 0 | 0 | 0 | 0 | | | | | | |
| 2071 | 02355 | 02070 | 4 | | 0 | 0 | 0 | 0 | 1 | 0 | 0 | 0 | 0 | 0 | 0 | 0 | 0 | 0 | 0 | 1 | 0 | 0 | 0 | 0 | 0 | 0 | 0 | 2 | 0 | D21063 | 96.9 | 385 | 1 | 3022 | 3406 |
| 2072 | 02356 | 02071 | 1 | | 0 | 0 | 0 | 0 | 1 | 0 | 0 | 0 | 0 | 0 | 0 | 0 | 0 | 0 | 0 | 0 | 0 | 0 | 0 | 0 | 0 | 0 | 0 | 0 | 0 | | | | | | |
| 2073 | 02357 | 02072 | 3 | | 0 | 0 | 0 | 0 | 1 | 0 | 0 | 0 | 0 | 1 | 0 | 0 | 0 | 0 | 0 | 0 | 0 | 0 | 0 | 1 | 0 | 0 | 0 | 0 | 0 | | | | | | |

Table 58

| | A | B | C | E | G | I | K | M | O | Q | S | U | W | Y | AA | AC | AE | AG | AI | AK | AM | AO | AQ | AS | AU | AW | AY | BA | BC | BE | BF | BG | BH | BI | BK |
|---|---|---|---|---|---|---|---|---|---|---|---|---|---|---|---|---|---|---|---|---|---|---|---|---|---|---|---|---|---|---|---|---|---|---|---|
| 2074 | 02358 | 02073 | 2 | | 0 | 0 | 0 | 0 | 2 | 0 | 0 | 0 | 0 | 0 | 0 | 0 | 0 | 0 | 0 | 0 | 0 | 0 | 0 | 0 | 0 | 0 | 0 | 0 | 0 | X69078 | 97.9 | 95 | 1 | 3127 | 3228 |
| 2075 | 02359 | 02074 | 2 | | 0 | 0 | 0 | 0 | 1 | 0 | 0 | 0 | 0 | 0 | 0 | 0 | 0 | 0 | 0 | 0 | 0 | 0 | 0 | 0 | 0 | 0 | 1 | 0 | 0 | | | | | | |
| 2076 | 02360 | 02075 | 3 | | 0 | 0 | 0 | 0 | 1 | 0 | 0 | 0 | 0 | 0 | 0 | 0 | 0 | 0 | 0 | 0 | 0 | 0 | 0 | 0 | 0 | 0 | 0 | 2 | 0 | | | | | | |
| 2077 | 02361 | 02076 | 1 | | 0 | 0 | 0 | 0 | 1 | 0 | 0 | 0 | 0 | 0 | 0 | 0 | 0 | 0 | 0 | 0 | 0 | 0 | 0 | 0 | 0 | 0 | 0 | 0 | 0 | | | | | | |
| 2078 | 02362 | 02077 | 4 | | 0 | 0 | 0 | 0 | 1 | 0 | 0 | 0 | 0 | 0 | 0 | 1 | 0 | 0 | 0 | 1 | 0 | 0 | 1 | 0 | 0 | 0 | 0 | 0 | 0 | | | | | | |
| 2079 | 02363 | 02078 | 2 | | 0 | 0 | 0 | 0 | 1 | 0 | 0 | 0 | 0 | 1 | 0 | 0 | 0 | 0 | 0 | 0 | 0 | 0 | 0 | 0 | 0 | 0 | 0 | 0 | 0 | | | | | | |
| 2080 | 02364 | 02079 | 1 | | 0 | 0 | 0 | 0 | 1 | 0 | 0 | 0 | 0 | 0 | 0 | 0 | 0 | 0 | 0 | 0 | 0 | 0 | 0 | 0 | 0 | 0 | 0 | 0 | 0 | | | | | | |
| 2081 | 02365 | 02080 | 1 | | 0 | 0 | 0 | 0 | 1 | 0 | 0 | 0 | 0 | 0 | 0 | 0 | 0 | 0 | 0 | 0 | 0 | 0 | 0 | 0 | 0 | 0 | 0 | 0 | 0 | | | | | | |
| 2082 | 02366 | 02081 | 1 | | 0 | 0 | 0 | 0 | 1 | 0 | 0 | 0 | 0 | 0 | 0 | 0 | 0 | 0 | 0 | 0 | 0 | 0 | 0 | 0 | 0 | 0 | 0 | 0 | 0 | | | | | | |
| 2083 | 02367 | 02082 | 2 | | 0 | 0 | 0 | 0 | 1 | 0 | 0 | 0 | 0 | 0 | 0 | 0 | 0 | 0 | 0 | 0 | 0 | 0 | 1 | 0 | 0 | 0 | 0 | 0 | 0 | | | | | | |
| 2084 | 02368 | 02083 | 1 | | 0 | 0 | 0 | 0 | 1 | 0 | 0 | 0 | 0 | 0 | 0 | 0 | 0 | 0 | 0 | 0 | 0 | 0 | 0 | 0 | 0 | 0 | 0 | 0 | 0 | | | | | | |
| 2085 | 02369 | 02084 | 1 | | 0 | 0 | 0 | 0 | 1 | 0 | 0 | 0 | 0 | 0 | 0 | 0 | 0 | 0 | 0 | 0 | 0 | 0 | 0 | 0 | 0 | 0 | 0 | 0 | 0 | Y00317 | 98.5 | 67 | 1 | 2027 | 2093 |
| 2086 | 02370 | 02085 | 2 | | 0 | 0 | 0 | 0 | 2 | 0 | 0 | 0 | 0 | 0 | 0 | 0 | 0 | 0 | 0 | 0 | 0 | 0 | 0 | 0 | 0 | 0 | 0 | 0 | 0 | | | | | | |
| 2087 | 02371 | 02086 | 1 | | 0 | 0 | 0 | 0 | 1 | 0 | 0 | 0 | 0 | 0 | 0 | 0 | 0 | 0 | 0 | 0 | 0 | 0 | 0 | 0 | 0 | 0 | 0 | 0 | 0 | | | | | | |
| 2088 | 02372 | 02087 | 6 | | 0 | 0 | 0 | 0 | 1 | 0 | 0 | 0 | 2 | 0 | 0 | 0 | 0 | 0 | 0 | 3 | 0 | 0 | 0 | 0 | 0 | 0 | 0 | 0 | 0 | | | | | | |
| 2089 | 02373 | 02088 | 1 | | 0 | 0 | 0 | 0 | 1 | 0 | 0 | 0 | 0 | 0 | 0 | 0 | 0 | 0 | 0 | 0 | 0 | 0 | 0 | 0 | 0 | 0 | 0 | 0 | 0 | | | | | | |
| 2090 | 02374 | 02089 | 38 | | 0 | 0 | 0 | 0 | 1 | 1 | 1 | 5 | 1 | 0 | 0 | 0 | 0 | 0 | 0 | 1 | 0 | 1 | 0 | 0 | 10 | 17 | 0 | 0 | 0 | | | | | | |
| 2091 | 02375 | 02090 | 1 | | 0 | 0 | 0 | 0 | 1 | 0 | 0 | 0 | 0 | 0 | 0 | 0 | 0 | 0 | 0 | 0 | 0 | 0 | 0 | 0 | 0 | 0 | 0 | 0 | 0 | | | | | | |
| 2092 | 02376 | 02091 | 4 | | 0 | 0 | 0 | 0 | 1 | 0 | 0 | 0 | 0 | 0 | 0 | 0 | 0 | 0 | 0 | 1 | 0 | 0 | 0 | 0 | 0 | 0 | 0 | 0 | 2 | | | | | | |
| 2093 | 02377 | 02092 | 1 | | 0 | 0 | 0 | 0 | 1 | 0 | 0 | 0 | 0 | 0 | 0 | 0 | 0 | 0 | 0 | 0 | 0 | 0 | 0 | 0 | 0 | 0 | 0 | 0 | 0 | X64594 | 100 | 56 | 1 | 1839 | 1927 |
| 2094 | 02378 | 02093 | 4 | | 0 | 0 | 0 | 0 | 1 | 0 | 0 | 0 | 0 | 0 | 0 | 0 | 0 | 0 | 2 | 0 | 0 | 1 | 0 | 0 | 0 | 0 | 0 | 0 | 0 | | | | | | |
| 2095 | 02379 | 02094 | 1 | | 0 | 0 | 0 | 0 | 1 | 0 | 0 | 0 | 0 | 0 | 0 | 0 | 0 | 0 | 0 | 0 | 0 | 0 | 0 | 0 | 0 | 0 | 0 | 0 | 0 | | | | | | |
| 2096 | 02380 | 02095 | 2 | | 0 | 0 | 0 | 0 | 1 | 0 | 0 | 0 | 0 | 0 | 0 | 0 | 0 | 1 | 0 | 0 | 0 | 0 | 0 | 0 | 0 | 0 | 0 | 0 | 0 | | | | | | |
| 2097 | 02382 | 02096 | 13 | | 0 | 0 | 0 | 0 | 1 | 0 | 0 | 0 | 2 | 1 | 1 | 2 | 0 | 0 | 0 | 2 | 0 | 0 | 0 | 1 | 0 | 0 | 2 | 1 | 0 | | | | | | |
| 2098 | 02399 | 02097 | 1 | | 0 | 0 | 0 | 0 | 0 | 1 | 0 | 0 | 0 | 0 | 0 | 0 | 0 | 0 | 0 | 0 | 0 | 0 | 0 | 0 | 0 | 0 | 0 | 0 | 0 | | | | | | |
| 2099 | 02400 | 02098 | 2 | | 0 | 0 | 0 | 0 | 0 | 1 | 0 | 0 | 0 | 0 | 0 | 1 | 0 | 0 | 0 | 0 | 0 | 0 | 0 | 0 | 0 | 0 | 0 | 0 | 0 | | | | | | |
| 2100 | 02401 | 02099 | 1 | | 0 | 0 | 0 | 0 | 0 | 1 | 0 | 0 | 0 | 0 | 0 | 0 | 0 | 0 | 0 | 0 | 0 | 0 | 0 | 0 | 0 | 0 | 0 | 0 | 0 | | | | | | |
| 2101 | 02402 | 02100 | 6 | | 0 | 0 | 0 | 0 | 0 | 1 | 0 | 0 | 1 | 1 | 0 | 0 | 0 | 0 | 0 | 0 | 1 | 1 | 0 | 0 | 1 | 0 | 0 | 0 | 0 | | | | | | |
| 2102 | 02403 | 02101 | 1 | | 0 | 0 | 0 | 0 | 0 | 1 | 0 | 0 | 0 | 0 | 0 | 0 | 0 | 0 | 0 | 0 | 0 | 0 | 0 | 0 | 0 | 0 | 0 | 0 | 0 | X03557 | 100 | 273 | 1 | 1370 | 1642 |
| 2103 | 02404 | 02102 | 1 | | 0 | 0 | 0 | 0 | 0 | 1 | 0 | 0 | 0 | 0 | 0 | 0 | 0 | 0 | 0 | 0 | 0 | 0 | 0 | 0 | 0 | 0 | 0 | 0 | 0 | | | | | | |
| 2104 | 02406 | 02103 | 3 | | 0 | 0 | 0 | 0 | 0 | 1 | 0 | 0 | 0 | 0 | 0 | 0 | 0 | 0 | 0 | 1 | 1 | 0 | 0 | 0 | 0 | 0 | 0 | 0 | 0 | | | | | | |
| 2105 | 02408 | 02104 | 1 | | 0 | 0 | 0 | 0 | 0 | 1 | 0 | 0 | 0 | 0 | 0 | 0 | 0 | 0 | 0 | 0 | 0 | 0 | 0 | 0 | 0 | 0 | 0 | 0 | 0 | | | | | | |
| 2106 | 02409 | 02105 | 2 | | 0 | 0 | 0 | 0 | 0 | 1 | 0 | 0 | 0 | 0 | 0 | 0 | 0 | 0 | 0 | 1 | 0 | 0 | 0 | 0 | 0 | 0 | 0 | 0 | 0 | J05037 | 94.8 | 305 | 1 | 1093 | 1393 |
| 2107 | 02411 | 02106 | 2 | | 0 | 0 | 0 | 0 | 0 | 2 | 0 | 0 | 0 | 0 | 0 | 0 | 0 | 0 | 0 | 0 | 0 | 0 | 0 | 0 | 0 | 0 | 0 | 0 | 0 | M62401 | 97.4 | 378 | 1 | 1500 | 1880 |
| 2108 | 02412 | 02107 | 2 | | 0 | 0 | 0 | 0 | 0 | 1 | 0 | 0 | 0 | 0 | 0 | 0 | 0 | 0 | 0 | 0 | 0 | 0 | 0 | 1 | 0 | 0 | 0 | 0 | 0 | | | | | | |
| 2109 | 02413 | 02108 | 3 | | 0 | 0 | 0 | 0 | 0 | 1 | 0 | 1 | 0 | 0 | 0 | 0 | 0 | 0 | 0 | 1 | 0 | 0 | 0 | 0 | 0 | 0 | 0 | 0 | 0 | | | | | | |

Table 59

| | A | B | C | E | G | I | K | M | O | Q | S | U | W | Y | AA | AC | AF | AG | AI | AK | AM | AO | AQ | AS | AU | AW | AY | BA | BC | BE | BF | BG | BH | BI | BK |
|---|---|---|---|---|---|---|---|---|---|---|---|---|---|---|---|---|---|---|---|---|---|---|---|---|---|---|---|---|---|---|---|---|---|---|---|
| 2110 | 02414 | 02109 | 2 | | 0 | 0 | 0 | 0 | 0 | 1 | 0 | 0 | 1 | 0 | 0 | 0 | 0 | 0 | 0 | 0 | 0 | 0 | 0 | 0 | 0 | 0 | 0 | 0 | 0 | | | | | | |
| 2111 | 02415 | 02110 | 1 | | 0 | 0 | 0 | 0 | 0 | 1 | 0 | 0 | 0 | 0 | 0 | 0 | 0 | 0 | 0 | 0 | 0 | 0 | 0 | 0 | 0 | 0 | 0 | 0 | 0 | M65131 | 97.9 | 340 | 1 | 1994 | 2798 |
| 2112 | 02416 | 02111 | 2 | | 0 | 0 | 0 | 0 | 0 | 2 | 0 | 0 | 0 | 0 | 0 | 0 | 0 | 0 | 0 | 0 | 0 | 0 | 0 | 0 | 0 | 0 | 0 | 0 | 0 | | | | | | |
| 2113 | 02417 | 02112 | 1 | | 0 | 0 | 0 | 0 | 0 | 1 | 0 | 0 | 0 | 0 | 0 | 0 | 0 | 0 | 0 | 0 | 0 | 0 | 0 | 0 | 0 | 0 | 0 | 0 | 0 | K01396 | 96.5 | 347 | 1 | 559 | 1352 |
| 2114 | 02418 | 02113 | 1 | | 0 | 0 | 0 | 0 | 0 | 1 | 0 | 0 | 0 | 0 | 0 | 0 | 0 | 0 | 0 | 0 | 0 | 0 | 0 | 0 | 0 | 0 | 0 | 0 | 0 | K01763 | 97.6 | 211 | 152 | 1022 | 1234 |
| 2115 | 02419 | 02114 | 1 | | 0 | 0 | 0 | 0 | 0 | 1 | 0 | 0 | 0 | 0 | 0 | 0 | 0 | 0 | 0 | 0 | 0 | 0 | 0 | 0 | 0 | 0 | 0 | 0 | 0 | | | | | | |
| 2116 | 02420 | 02115 | 1 | | 0 | 0 | 0 | 0 | 0 | 1 | 0 | 0 | 0 | 0 | 0 | 0 | 0 | 0 | 0 | 0 | 0 | 0 | 0 | 0 | 0 | 0 | 0 | 0 | 0 | | | | | | |
| 2117 | 02421 | 02116 | 1 | | 0 | 0 | 0 | 0 | 0 | 1 | 0 | 0 | 0 | 0 | 0 | 0 | 0 | 0 | 0 | 0 | 0 | 0 | 0 | 0 | 0 | 0 | 0 | 0 | 0 | | | | | | |
| 2118 | 02422 | 02117 | 7 | | 0 | 0 | 0 | 0 | 0 | 1 | 0 | 0 | 0 | 5 | 0 | 0 | 0 | 0 | 0 | 0 | 1 | 0 | 0 | 0 | 0 | 0 | 0 | 0 | 0 | | | | | | |
| 2119 | 02423 | 02118 | 3 | | 0 | 0 | 0 | 0 | 0 | 3 | 0 | 0 | 0 | 0 | 0 | 0 | 0 | 0 | 0 | 0 | 0 | 0 | 0 | 0 | 0 | 0 | 0 | 0 | 0 | | | | | | |
| 2120 | 02424 | 02119 | 1 | | 0 | 0 | 0 | 0 | 0 | 1 | 0 | 0 | 0 | 0 | 0 | 0 | 0 | 0 | 0 | 0 | 0 | 0 | 0 | 0 | 0 | 0 | 0 | 0 | 0 | | | | | | |
| 2121 | 02425 | 02120 | 1 | | 0 | 0 | 0 | 0 | 0 | 1 | 0 | 0 | 0 | 0 | 0 | 0 | 0 | 0 | 0 | 0 | 0 | 0 | 0 | 0 | 0 | 0 | 0 | 0 | 0 | S49975 | 99.4 | 178 | 1 | 410 | 790 |
| 2122 | 02426 | 02121 | 1 | | 0 | 0 | 0 | 0 | 0 | 1 | 0 | 0 | 0 | 0 | 0 | 0 | 0 | 0 | 0 | 0 | 0 | 0 | 0 | 0 | 0 | 0 | 0 | 0 | 0 | | | | | | |
| 2123 | 02427 | 02122 | 1 | | 0 | 0 | 0 | 0 | 0 | 1 | 0 | 0 | 0 | 0 | 0 | 0 | 0 | 0 | 0 | 0 | 0 | 0 | 0 | 0 | 0 | 0 | 0 | 0 | 0 | | | | | | |
| 2124 | 02428 | 02123 | 4 | | 0 | 0 | 0 | 0 | 0 | 2 | 0 | 0 | 0 | 0 | 0 | 0 | 1 | 0 | 0 | 0 | 1 | 0 | 0 | 0 | 0 | 0 | 0 | 0 | 0 | | | | | | |
| 2125 | 02429 | 02124 | 1 | | 0 | 0 | 0 | 0 | 0 | 1 | 0 | 0 | 0 | 0 | 0 | 0 | 0 | 0 | 0 | 0 | 0 | 0 | 0 | 0 | 0 | 0 | 0 | 0 | 0 | | | | | | |
| 2126 | 02430 | 02125 | 3 | | 0 | 0 | 0 | 0 | 0 | 1 | 0 | 1 | 0 | 0 | 0 | 0 | 0 | 0 | 0 | 0 | 0 | 0 | 0 | 0 | 0 | 1 | 0 | 0 | 0 | | | | | | |
| 2127 | 02431 | 02126 | 1 | | 0 | 0 | 0 | 0 | 0 | 1 | 0 | 0 | 0 | 0 | 0 | 0 | 0 | 0 | 0 | 0 | 0 | 0 | 0 | 0 | 0 | 0 | 0 | 0 | 0 | | | | | | |
| 2128 | 02432 | 02127 | 1 | | 0 | 0 | 0 | 0 | 0 | 1 | 0 | 0 | 0 | 0 | 0 | 0 | 0 | 0 | 0 | 0 | 0 | 0 | 0 | 0 | 0 | 0 | 0 | 0 | 0 | M81182 | 98.1 | 319 | 1 | 3042 | 3397 |
| 2129 | 02433 | 02128 | 2 | | 0 | 0 | 0 | 0 | 0 | 2 | 0 | 0 | 0 | 0 | 0 | 0 | 0 | 0 | 0 | 0 | 0 | 0 | 0 | 0 | 0 | 0 | 0 | 0 | 0 | | | | | | |
| 2130 | 02434 | 02129 | 1 | | 0 | 0 | 0 | 0 | 0 | 1 | 0 | 0 | 0 | 0 | 0 | 0 | 0 | 0 | 0 | 0 | 0 | 0 | 0 | 0 | 0 | 0 | 0 | 0 | 0 | K01500 | 96.4 | 309 | 1 | 1214 | 1520 |
| 2131 | 02435 | 02130 | 2 | | 0 | 0 | 0 | 0 | 0 | 2 | 0 | 0 | 0 | 0 | 0 | 0 | 0 | 0 | 0 | 0 | 0 | 0 | 0 | 0 | 0 | 0 | 0 | 0 | 0 | K00485 | 93 | 214 | 12 | 107 | 323 |
| 2132 | 02436 | 02131 | 1 | | 0 | 0 | 0 | 0 | 0 | 1 | 0 | 0 | 0 | 0 | 0 | 0 | 0 | 0 | 0 | 0 | 0 | 0 | 0 | 0 | 0 | 0 | 0 | 0 | 0 | | | | | | |
| 2133 | 02437 | 02132 | 1 | | 0 | 0 | 0 | 0 | 0 | 1 | 0 | 0 | 0 | 0 | 0 | 0 | 0 | 0 | 0 | 0 | 0 | 0 | 0 | 0 | 0 | 0 | 0 | 0 | 0 | | | | | | |
| 2134 | 02438 | 02133 | 2 | | 0 | 0 | 0 | 0 | 0 | 1 | 0 | 0 | 0 | 0 | 0 | 0 | 0 | 0 | 0 | 0 | 0 | 0 | 0 | 0 | 0 | 1 | 0 | 0 | 0 | | | | | | |
| 2135 | 02439 | 02134 | 4 | | 0 | 0 | 0 | 0 | 0 | 1 | 0 | 0 | 0 | 0 | 0 | 1 | 0 | 0 | 0 | 1 | 0 | 0 | 0 | 0 | 0 | 0 | 0 | 1 | 0 | | | | | | |
| 2136 | 02441 | 02135 | 5 | | 0 | 0 | 0 | 0 | 0 | 1 | 0 | 0 | 0 | 0 | 0 | 1 | 0 | 0 | 0 | 0 | 0 | 0 | 1 | 0 | 0 | 1 | 0 | 1 | 0 | | | | | | |
| 2137 | 02442 | 02136 | 3 | | 0 | 0 | 0 | 0 | 0 | 2 | 0 | 0 | 0 | 0 | 0 | 0 | 0 | 1 | 0 | 0 | 0 | 0 | 0 | 0 | 0 | 0 | 0 | 0 | 0 | J04794 | 96.7 | 301 | 1 | 834 | 1132 |
| 2138 | 02443 | 02137 | 8 | | 0 | 0 | 0 | 0 | 0 | 1 | 0 | 0 | 3 | 0 | 0 | 0 | 0 | 0 | 0 | 1 | 0 | 0 | 1 | 1 | 0 | 0 | 1 | 0 | 0 | | | | | | |
| 2139 | 02444 | 02138 | 1 | | 0 | 0 | 0 | 0 | 0 | 1 | 0 | 0 | 0 | 0 | 0 | 0 | 0 | 0 | 0 | 0 | 0 | 0 | 0 | 0 | 0 | 0 | 0 | 0 | 0 | L19297 | 100 | 294 | 1 | 790 | 1083 |
| 2140 | 02445 | 02139 | 2 | | 0 | 0 | 0 | 0 | 0 | 1 | 0 | 0 | 0 | 0 | 0 | 0 | 0 | 0 | 1 | 0 | 0 | 0 | 0 | 0 | 0 | 0 | 0 | 0 | 0 | | | | | | |
| 2141 | 02446 | 02140 | 3 | | 0 | 0 | 0 | 0 | 0 | 1 | 0 | 0 | 0 | 0 | 0 | 0 | 0 | 0 | 0 | 1 | 0 | 0 | 1 | 0 | 0 | 0 | 0 | 0 | 0 | | | | | | |
| 2142 | 02447 | 02141 | 1 | | 0 | 0 | 0 | 0 | 0 | 1 | 0 | 0 | 0 | 0 | 0 | 0 | 0 | 0 | 0 | 0 | 0 | 0 | 0 | 0 | 0 | 0 | 0 | 0 | 0 | | | | | | |
| 2143 | 02448 | 02142 | 3 | | 0 | 0 | 0 | 0 | 0 | 1 | 0 | 0 | 0 | 0 | 0 | 0 | 0 | 0 | 0 | 0 | 0 | 0 | 0 | 0 | 0 | 0 | 0 | 2 | 0 | | | | | | |
| 2144 | 02449 | 02143 | 1 | | 0 | 0 | 0 | 0 | 0 | 1 | 0 | 0 | 0 | 0 | 0 | 0 | 0 | 0 | 0 | 0 | 0 | 0 | 0 | 0 | 0 | 0 | 0 | 0 | 0 | | | | | | |
| 2145 | 02450 | 02144 | 2 | | 0 | 0 | 0 | 0 | 0 | 2 | 0 | 0 | 0 | 0 | 0 | 0 | 0 | 0 | 0 | 0 | 0 | 0 | 0 | 0 | 0 | 0 | 0 | 0 | 0 | | | | | | |

Table 60

| | A | B | C | E | G | I | K | M | O | Q | S | U | W | Y | AA | AC | AE | AG | AI | AK | AM | AO | AQ | AS | AU | AW | AY | BA | BC | BE | BF | BG | BH | BI | BK |
|---|---|---|---|---|---|---|---|---|---|---|---|---|---|---|---|---|---|---|---|---|---|---|---|---|---|---|---|---|---|---|---|---|---|---|---|
| 2146 | 02452 | 02145 | 2 | | 0 | 0 | 0 | 0 | 0 | 1 | 0 | 0 | 0 | 0 | 0 | 0 | 0 | 0 | 0 | 0 | 0 | 0 | 0 | 0 | 0 | 1 | 0 | 0 | 0 | | | | | | |
| 2147 | 02453 | 02146 | 1 | | 0 | 0 | 0 | 0 | 0 | 1 | 0 | 0 | 0 | 0 | 0 | 0 | 0 | 0 | 0 | 0 | 0 | 0 | 0 | 0 | 0 | 0 | 0 | 0 | 0 | | | | | | |
| 2148 | 02454 | 02147 | 1 | | 0 | 0 | 0 | 0 | 0 | 1 | 0 | 0 | 0 | 0 | 0 | 0 | 0 | 0 | 0 | 0 | 0 | 0 | 0 | 0 | 0 | 0 | 0 | 0 | 0 | | | | | | |
| 2149 | 02455 | 02148 | 1 | | 0 | 0 | 0 | 0 | 0 | 1 | 0 | 0 | 0 | 0 | 0 | 0 | 0 | 0 | 0 | 0 | 0 | 0 | 0 | 0 | 0 | 0 | 0 | 0 | 0 | | | | | | |
| 2150 | 02456 | 02149 | 3 | | 0 | 0 | 0 | 0 | 0 | 1 | 0 | 0 | 0 | 0 | 0 | 0 | 0 | 1 | 0 | 0 | 1 | 0 | 0 | 0 | 0 | 0 | 0 | 0 | 0 | | | | | | |
| 2151 | 02457 | 02150 | 2 | | 0 | 0 | 0 | 0 | 0 | 1 | 0 | 0 | 0 | 0 | 1 | 0 | 0 | 0 | 0 | 0 | 0 | 0 | 0 | 0 | 0 | 0 | 0 | 0 | 0 | | | | | | |
| 2152 | 02458 | 02151 | 1 | | 0 | 0 | 0 | 0 | 0 | 1 | 0 | 0 | 0 | 0 | 0 | 0 | 0 | 0 | 0 | 0 | 0 | 0 | 0 | 0 | 0 | 0 | 0 | 0 | 0 | | | | | | |
| 2153 | 02459 | 02152 | 2 | | 0 | 0 | 0 | 0 | 0 | 1 | 0 | 0 | 0 | 0 | 0 | 0 | 0 | 0 | 1 | 0 | 0 | 0 | 0 | 0 | 0 | 0 | 0 | 0 | 0 | | | | | | |
| 2154 | 02461 | 02153 | 1 | | 0 | 0 | 0 | 0 | 0 | 1 | 0 | 0 | 0 | 0 | 0 | 0 | 0 | 0 | 0 | 0 | 0 | 0 | 0 | 0 | 0 | 0 | 0 | 0 | 0 | M15943 | 97.9 | 236 | 1 | 1746 | 1981 |
| 2155 | 02462 | 02154 | 1 | | 0 | 0 | 0 | 0 | 0 | 1 | 0 | 0 | 0 | 0 | 0 | 0 | 0 | 0 | 0 | 0 | 0 | 0 | 0 | 0 | 0 | 0 | 0 | 0 | 0 | | | | | | |
| 2156 | 02463 | 02155 | 1 | | 0 | 0 | 0 | 0 | 0 | 1 | 0 | 0 | 0 | 0 | 0 | 0 | 0 | 0 | 0 | 0 | 0 | 0 | 0 | 0 | 0 | 0 | 0 | 0 | 0 | L11244 | 98.8 | 245 | 1 | 913 | 1157 |
| 2157 | 02464 | 02156 | 1 | | 0 | 0 | 0 | 0 | 0 | 1 | 0 | 0 | 0 | 0 | 0 | 0 | 0 | 0 | 0 | 0 | 0 | 0 | 0 | 0 | 0 | 0 | 0 | 0 | 0 | | | | | | |
| 2158 | 02465 | 02157 | 1 | | 0 | 0 | 0 | 0 | 0 | 1 | 0 | 0 | 0 | 0 | 0 | 0 | 0 | 0 | 0 | 0 | 0 | 0 | 0 | 0 | 0 | 0 | 0 | 0 | 0 | X07002 | 98.7 | 231 | 1 | 969 | 1199 |
| 2159 | 02466 | 02158 | 1 | | 0 | 0 | 0 | 0 | 0 | 1 | 0 | 0 | 0 | 0 | 0 | 0 | 0 | 0 | 0 | 0 | 0 | 0 | 0 | 0 | 0 | 0 | 0 | 0 | 0 | | | | | | |
| 2160 | 02467 | 02159 | 1 | | 0 | 0 | 0 | 0 | 0 | 1 | 0 | 0 | 0 | 0 | 0 | 0 | 0 | 0 | 0 | 0 | 0 | 0 | 0 | 0 | 0 | 0 | 0 | 0 | 0 | | | | | | |
| 2161 | 02468 | 02160 | 2 | | 0 | 0 | 0 | 0 | 0 | 1 | 0 | 0 | 0 | 0 | 0 | 0 | 0 | 0 | 0 | 1 | 0 | 0 | 0 | 0 | 0 | 0 | 0 | 0 | 0 | | | | | | |
| 2162 | 02469 | 02161 | 1 | | 0 | 0 | 0 | 0 | 0 | 1 | 0 | 0 | 0 | 0 | 0 | 0 | 0 | 0 | 0 | 0 | 0 | 0 | 0 | 0 | 0 | 0 | 0 | 0 | 0 | | | | | | |
| 2163 | 02471 | 02162 | 2 | | 0 | 0 | 0 | 0 | 0 | 2 | 0 | 0 | 0 | 0 | 0 | 0 | 0 | 0 | 0 | 0 | 0 | 0 | 0 | 0 | 0 | 0 | 0 | 0 | 0 | L11708 | 99.1 | 219 | 1 | 1124 | 1344 |
| 2164 | 02472 | 02163 | 1 | | 0 | 0 | 0 | 0 | 0 | 1 | 0 | 0 | 0 | 0 | 0 | 0 | 0 | 0 | 0 | 0 | 0 | 0 | 0 | 0 | 0 | 0 | 0 | 0 | 0 | | | | | | |
| 2165 | 02473 | 02164 | 1 | | 0 | 0 | 0 | 0 | 0 | 1 | 0 | 0 | 0 | 0 | 0 | 0 | 0 | 0 | 0 | 0 | 0 | 0 | 0 | 0 | 0 | 0 | 0 | 0 | 0 | | | | | | |
| 2166 | 02474 | 02165 | 2 | | 0 | 0 | 0 | 0 | 0 | 2 | 0 | 0 | 0 | 0 | 0 | 0 | 0 | 0 | 0 | 0 | 0 | 0 | 0 | 0 | 0 | 0 | 0 | 0 | 0 | | | | | | |
| 2167 | 02475 | 02166 | 1 | | 0 | 0 | 0 | 0 | 0 | 1 | 0 | 0 | 0 | 0 | 0 | 0 | 0 | 0 | 0 | 0 | 0 | 0 | 0 | 0 | 0 | 0 | 0 | 0 | 0 | | | | | | |
| 2168 | 02476 | 02167 | 1 | | 0 | 0 | 0 | 0 | 0 | 1 | 0 | 0 | 0 | 0 | 0 | 0 | 0 | 0 | 0 | 0 | 0 | 0 | 0 | 0 | 0 | 0 | 0 | 0 | 0 | | | | | | |
| 2169 | 02477 | 02168 | 4 | | 0 | 0 | 0 | 0 | 0 | 1 | 0 | 0 | 1 | 0 | 0 | 0 | 0 | 0 | 0 | 0 | 0 | 0 | 0 | 0 | 0 | 0 | 0 | 2 | 0 | | | | | | |
| 2170 | 02478 | 02169 | 1 | | 0 | 0 | 0 | 0 | 0 | 1 | 0 | 0 | 0 | 0 | 0 | 0 | 0 | 0 | 0 | 0 | 0 | 0 | 0 | 0 | 0 | 0 | 0 | 0 | 0 | | | | | | |
| 2171 | 02479 | 02170 | 6 | | 0 | 0 | 0 | 0 | 0 | 1 | 0 | 0 | 2 | 0 | 1 | 1 | 0 | 0 | 0 | 0 | 0 | 0 | 1 | 0 | 0 | 0 | 0 | 0 | 0 | | | | | | |
| 2172 | 02480 | 02171 | 3 | | 0 | 0 | 0 | 0 | 0 | 1 | 0 | 0 | 0 | 0 | 0 | 0 | 0 | 0 | 0 | 0 | 0 | 0 | 0 | 0 | 1 | 1 | 0 | 0 | 0 | X01630 | 94.4 | 197 | 1 | 1355 | 1560 |
| 2173 | 02481 | 02172 | 1 | | 0 | 0 | 0 | 0 | 0 | 1 | 0 | 0 | 0 | 0 | 0 | 0 | 0 | 0 | 0 | 0 | 0 | 0 | 0 | 0 | 0 | 0 | 0 | 0 | 0 | | | | | | |
| 2174 | 02482 | 02173 | 1 | | 0 | 0 | 0 | 0 | 0 | 1 | 0 | 0 | 0 | 0 | 0 | 0 | 0 | 0 | 0 | 0 | 0 | 0 | 0 | 0 | 0 | 0 | 0 | 0 | 0 | L22548 | 98.5 | 197 | 1 | 3184 | 3394 |
| 2175 | 02483 | 02174 | 1 | | 0 | 0 | 0 | 0 | 0 | 1 | 0 | 0 | 0 | 0 | 0 | 0 | 0 | 0 | 0 | 0 | 0 | 0 | 0 | 0 | 0 | 0 | 0 | 0 | 0 | M13149 | 100 | 192 | 1 | 1856 | 2051 |
| 2176 | 02484 | 02175 | 1 | | 0 | 0 | 0 | 0 | 0 | 1 | 0 | 0 | 0 | 0 | 0 | 0 | 0 | 0 | 0 | 0 | 0 | 0 | 0 | 0 | 0 | 0 | 0 | 0 | 0 | | | | | | |
| 2177 | 02486 | 02176 | 1 | | 0 | 0 | 0 | 0 | 0 | 1 | 0 | 0 | 0 | 0 | 0 | 0 | 0 | 0 | 0 | 0 | 0 | 0 | 0 | 0 | 0 | 0 | 0 | 0 | 0 | L13176 | 91.3 | 173 | 15 | 5142 | 5417 |
| 2178 | 02487 | 02177 | 1 | | 0 | 0 | 0 | 0 | 0 | 1 | 0 | 0 | 0 | 0 | 0 | 0 | 0 | 0 | 0 | 0 | 0 | 0 | 0 | 0 | 0 | 0 | 0 | 0 | 0 | | | | | | |
| 2179 | 02488 | 02178 | 2 | | 0 | 0 | 0 | 0 | 0 | 2 | 0 | 0 | 0 | 0 | 0 | 0 | 0 | 0 | 0 | 0 | 0 | 0 | 0 | 0 | 0 | 0 | 0 | 0 | 0 | Z28339 | 98.9 | 180 | 1 | 2513 | 2694 |
| 2180 | 02489 | 02179 | 1 | | 0 | 0 | 0 | 0 | 0 | 1 | 0 | 0 | 0 | 0 | 0 | 0 | 0 | 0 | 0 | 0 | 0 | 0 | 0 | 0 | 0 | 0 | 0 | 0 | 0 | M76707 | 97.7 | 177 | 1 | 988 | 1166 |
| 2181 | 02490 | 02180 | 16 | | 0 | 0 | 0 | 0 | 0 | 1 | 2 | 1 | 0 | 0 | 0 | 0 | 1 | 0 | 0 | 0 | 0 | 0 | 1 | 1 | 1 | 2 | 6 | 0 | 0 | | | | | | |

Table 61

| | A | B | C | E | G | I | K | M | O | Q | S | U | W | Y | AA | AC | AF | AG | AI | AK | AM | AO | AQ | AS | AU | AW | AY | BA | BC | BE | BF | BG | BH | BI | BK |
|---|---|---|---|---|---|---|---|---|---|---|---|---|---|---|---|---|---|---|---|---|---|---|---|---|---|---|---|---|---|---|---|---|---|---|---|
| 2182 | 02491 | 02181 | 4 | | 0 | 0 | 0 | 0 | 0 | 2 | 0 | 1 | 0 | 0 | 0 | 0 | 0 | 0 | 1 | 0 | 0 | 0 | 0 | 0 | 0 | 0 | 0 | 0 | 0 | | | | | | |
| 2183 | 02493 | 02182 | 1 | | 0 | 0 | 0 | 0 | 0 | 1 | 0 | 0 | 0 | 0 | 0 | 0 | 0 | 0 | 0 | 0 | 0 | 0 | 0 | 0 | 0 | 0 | 0 | 0 | 0 | | | | | | |
| 2184 | 02494 | 02183 | 2 | | 0 | 0 | 0 | 0 | 0 | 1 | 0 | 0 | 1 | 0 | 0 | 0 | 0 | 0 | 0 | 0 | 0 | 0 | 0 | 0 | 0 | 0 | 0 | 0 | 0 | | | | | | |
| 2185 | 02495 | 02184 | 1 | | 0 | 0 | 0 | 0 | 0 | 1 | 0 | 0 | 0 | 0 | 0 | 0 | 0 | 0 | 0 | 0 | 0 | 0 | 0 | 0 | 0 | 0 | 0 | 0 | 0 | | | | | | |
| 2186 | 02496 | 02185 | 2 | | 0 | 0 | 0 | 0 | 0 | 1 | 0 | 0 | 0 | 0 | 0 | 0 | 0 | 0 | 0 | 0 | 1 | 0 | 0 | 0 | 0 | 0 | 0 | 0 | 0 | | | | | | |
| 2187 | 02497 | 02186 | 1 | | 0 | 0 | 0 | 0 | 0 | 1 | 0 | 0 | 0 | 0 | 0 | 0 | 0 | 0 | 0 | 0 | 0 | 0 | 0 | 0 | 0 | 0 | 0 | 0 | 0 | | | | | | |
| 2188 | 02498 | 02187 | 1 | | 0 | 0 | 0 | 0 | 0 | 1 | 0 | 0 | 0 | 0 | 0 | 0 | 0 | 0 | 0 | 0 | 0 | 0 | 0 | 0 | 0 | 0 | 0 | 0 | 0 | | | | | | |
| 2189 | 02499 | 02188 | 2 | | 0 | 0 | 0 | 0 | 0 | 1 | 0 | 0 | 0 | 0 | 0 | 0 | 0 | 0 | 0 | 0 | 0 | 0 | 0 | 0 | 0 | 1 | 0 | 0 | 0 | | | | | | |
| 2190 | 02500 | 02189 | 3 | | 0 | 0 | 0 | 0 | 0 | 2 | 0 | 0 | 0 | 0 | 0 | 0 | 1 | 0 | 0 | 0 | 0 | 0 | 0 | 0 | 0 | 0 | 0 | 0 | 0 | | | | | | |
| 2191 | 02501 | 02190 | 1 | | 0 | 0 | 0 | 0 | 0 | 1 | 0 | 0 | 0 | 0 | 0 | 0 | 0 | 0 | 0 | 0 | 0 | 0 | 0 | 0 | 0 | 0 | 0 | 0 | 0 | | | | | | |
| 2192 | 02502 | 02191 | 1 | | 0 | 0 | 0 | 0 | 0 | 1 | 0 | 0 | 0 | 0 | 0 | 0 | 0 | 0 | 0 | 0 | 0 | 0 | 0 | 0 | 0 | 0 | 0 | 0 | 0 | | | | | | |
| 2193 | 02503 | 02192 | 1 | | 0 | 0 | 0 | 0 | 0 | 1 | 0 | 0 | 0 | 0 | 0 | 0 | 0 | 0 | 0 | 0 | 0 | 0 | 0 | 0 | 0 | 0 | 0 | 0 | 0 | | | | | | |
| 2194 | 02504 | 02193 | 3 | | 0 | 0 | 0 | 0 | 0 | 1 | 0 | 0 | 1 | 0 | 0 | 0 | 0 | 0 | 0 | 0 | 1 | 0 | 0 | 0 | 0 | 0 | 0 | 0 | 0 | | | | | | |
| 2195 | 02505 | 02194 | 5 | | 0 | 0 | 0 | 0 | 0 | 2 | 1 | 0 | 0 | 0 | 0 | 0 | 0 | 0 | 0 | 0 | 0 | 1 | 0 | 0 | 1 | 0 | 0 | 0 | 0 | X04701 | 97.3 | 147 | 1 | 2139 | 2386 |
| 2196 | 02506 | 02195 | 2 | | 0 | 0 | 0 | 0 | 0 | 2 | 0 | 0 | 0 | 0 | 0 | 0 | 0 | 0 | 0 | 0 | 0 | 0 | 0 | 0 | 0 | 0 | 0 | 0 | 0 | | | | | | |
| 2197 | 02507 | 02196 | 4 | | 0 | 0 | 0 | 0 | 0 | 1 | 0 | 0 | 0 | 0 | 0 | 0 | 0 | 1 | 0 | 0 | 0 | 0 | 1 | 0 | 0 | 1 | 0 | 0 | 0 | | | | | | |
| 2198 | 02508 | 02197 | 1 | | 0 | 0 | 0 | 0 | 0 | 1 | 0 | 0 | 0 | 0 | 0 | 0 | 0 | 0 | 0 | 0 | 0 | 0 | 0 | 0 | 0 | 0 | 0 | 0 | 0 | | | | | | |
| 2199 | 02509 | 02198 | 1 | | 0 | 0 | 0 | 0 | 0 | 1 | 0 | 0 | 0 | 0 | 0 | 0 | 0 | 0 | 0 | 0 | 0 | 0 | 0 | 0 | 0 | 0 | 0 | 0 | 0 | | | | | | |
| 2200 | 02510 | 02199 | 1 | | 0 | 0 | 0 | 0 | 0 | 1 | 0 | 0 | 0 | 0 | 0 | 0 | 0 | 0 | 0 | 0 | 0 | 0 | 0 | 0 | 0 | 0 | 0 | 0 | 0 | | | | | | |
| 2201 | 02511 | 02200 | 1 | | 0 | 0 | 0 | 0 | 0 | 1 | 0 | 0 | 0 | 0 | 0 | 0 | 0 | 0 | 0 | 0 | 0 | 0 | 0 | 0 | 0 | 0 | 0 | 0 | 0 | | | | | | |
| 2202 | 02512 | 02201 | 13 | | 0 | 0 | 0 | 0 | 0 | 1 | 0 | 1 | 8 | 1 | 0 | 0 | 0 | 0 | 1 | 0 | 0 | 1 | 0 | 0 | 0 | 0 | 0 | 0 | 0 | M85289 | 99.2 | 132 | 1 | #### | 14327 |
| 2203 | 02513 | 02202 | 3 | | 0 | 0 | 0 | 0 | 0 | 2 | 0 | 0 | 0 | 0 | 0 | 0 | 0 | 0 | 0 | 0 | 0 | 0 | 0 | 1 | 0 | 0 | 0 | 0 | 0 | | | | | | |
| 2204 | 02514 | 02203 | 8 | | 0 | 0 | 0 | 0 | 0 | 1 | 0 | 1 | 0 | 0 | 0 | 0 | 0 | 0 | 0 | 2 | 0 | 1 | 1 | 0 | 1 | 1 | 0 | 0 | 0 | | | | | | |
| 2205 | 02515 | 02204 | 2 | | 0 | 0 | 0 | 0 | 0 | 2 | 0 | 0 | 0 | 0 | 0 | 0 | 0 | 0 | 0 | 0 | 0 | 0 | 0 | 0 | 0 | 0 | 0 | 0 | 0 | | | | | | |
| 2206 | 02516 | 02205 | 5 | | 0 | 0 | 0 | 0 | 0 | 2 | 0 | 0 | 0 | 0 | 0 | 1 | 0 | 0 | 1 | 0 | 0 | 0 | 1 | 0 | 0 | 0 | 0 | 0 | 0 | X72889 | 98.9 | 176 | 1 | 5691 | 5959 |
| 2207 | 02517 | 02206 | 6 | | 0 | 0 | 0 | 0 | 0 | 1 | 0 | 2 | 0 | 0 | 0 | 0 | 0 | 0 | 0 | 2 | 0 | 1 | 0 | 0 | 0 | 0 | 0 | 0 | 0 | J03746 | 96.9 | 127 | 1 | 784 | 909 |
| 2208 | 02518 | 02207 | 1 | | 0 | 0 | 0 | 0 | 0 | 1 | 0 | 0 | 0 | 0 | 0 | 0 | 0 | 0 | 0 | 0 | 0 | 0 | 0 | 0 | 0 | 0 | 0 | 0 | 0 | | | | | | |
| 2209 | 02519 | 02208 | 4 | | 0 | 0 | 0 | 0 | 0 | 1 | 0 | 0 | 0 | 0 | 0 | 1 | 0 | 0 | 0 | 0 | 1 | 0 | 0 | 1 | 0 | 0 | 0 | 0 | 0 | X75694 | 93.6 | 220 | 4 | 3 | 230 |
| 2210 | 02520 | 02209 | 1 | | 0 | 0 | 0 | 0 | 0 | 1 | 0 | 0 | 0 | 0 | 0 | 0 | 0 | 0 | 0 | 0 | 0 | 0 | 0 | 0 | 0 | 0 | 0 | 0 | 0 | | | | | | |
| 2211 | 02521 | 02210 | 1 | | 0 | 0 | 0 | 0 | 0 | 1 | 0 | 0 | 0 | 0 | 0 | 0 | 0 | 0 | 0 | 0 | 0 | 0 | 0 | 0 | 0 | 0 | 0 | 0 | 0 | | | | | | |
| 2212 | 02522 | 02211 | 2 | | 0 | 0 | 0 | 0 | 0 | 2 | 0 | 0 | 0 | 0 | 0 | 0 | 0 | 0 | 0 | 0 | 0 | 0 | 0 | 0 | 0 | 0 | 0 | 0 | 0 | | | | | | |
| 2213 | 02523 | 02212 | 2 | | 0 | 0 | 0 | 0 | 0 | 1 | 0 | 0 | 0 | 1 | 0 | 0 | 0 | 0 | 0 | 0 | 0 | 0 | 0 | 0 | 0 | 0 | 0 | 0 | 0 | | | | | | |
| 2214 | 02524 | 02213 | 2 | | 0 | 0 | 0 | 0 | 0 | 1 | 0 | 0 | 0 | 0 | 0 | 0 | 0 | 0 | 1 | 0 | 0 | 0 | 0 | 0 | 0 | 0 | 0 | 0 | 0 | | | | | | |
| 2215 | 02525 | 02214 | 5 | | 0 | 0 | 0 | 0 | 0 | 5 | 0 | 0 | 0 | 0 | 0 | 0 | 0 | 0 | 0 | 0 | 0 | 0 | 0 | 0 | 0 | 0 | 0 | 0 | 0 | J00307 | 100 | 116 | 1 | 1831 | 1947 |
| 2216 | 02526 | 02215 | 3 | | 0 | 0 | 0 | 0 | 0 | 1 | 0 | 1 | 0 | 0 | 0 | 0 | 0 | 0 | 0 | 0 | 0 | 0 | 0 | 0 | 0 | 1 | 0 | 0 | 0 | | | | | | |
| 2217 | 02527 | 02216 | 2 | | 0 | 0 | 0 | 0 | 0 | 2 | 0 | 0 | 0 | 0 | 0 | 0 | 0 | 0 | 0 | 0 | 0 | 0 | 0 | 0 | 0 | 0 | 0 | 0 | 0 | | | | | | |

Table 62

| | A | B | C | E | G | I | K | M | O | Q | S | U | W | Y | AA | AC | AE | AG | AI | AK | AM | AO | AQ | AS | AU | AW | AY | BA | BC | BE | BF | BG | BH | BI | BK |
|---|---|---|---|---|---|---|---|---|---|---|---|---|---|---|---|---|---|---|---|---|---|---|---|---|---|---|---|---|---|---|---|---|---|---|---|
| 2218 | 02528 | 02217 | 2 | | 0 | 0 | 0 | 0 | 0 | 1 | 0 | 0 | 1 | 0 | 0 | 0 | 0 | 0 | 0 | 0 | 0 | 0 | 0 | 0 | 0 | 0 | 0 | 0 | 0 | | | | | | |
| 2219 | 02529 | 02218 | 1 | | 0 | 0 | 0 | 0 | 0 | 1 | 0 | 0 | 0 | 0 | 0 | 0 | 0 | 0 | 0 | 0 | 0 | 0 | 0 | 0 | 0 | 0 | 0 | 0 | 0 | | | | | | |
| 2220 | 02531 | 02219 | 3 | | 0 | 0 | 0 | 0 | 0 | 1 | 0 | 0 | 0 | 0 | 1 | 0 | 0 | 0 | 0 | 0 | 0 | 0 | 0 | 0 | 0 | 0 | 0 | 1 | 0 | | | | | | |
| 2221 | 02532 | 02220 | 3 | | 0 | 0 | 0 | 0 | 0 | 1 | 0 | 0 | 0 | 0 | 0 | 0 | 0 | 0 | 0 | 0 | 1 | 1 | 0 | 0 | 0 | 0 | 0 | 0 | 0 | | | | | | |
| 2222 | 02534 | 02221 | 5 | | 0 | 0 | 0 | 0 | 0 | 2 | 0 | 0 | 0 | 0 | 0 | 0 | 0 | 0 | 0 | 0 | 0 | 0 | 0 | 0 | 2 | 0 | 1 | 0 | 0 | | | | | | |
| 2223 | 02535 | 02222 | 1 | | 0 | 0 | 0 | 0 | 0 | 1 | 0 | 0 | 0 | 0 | 0 | 0 | 0 | 0 | 0 | 0 | 0 | 0 | 0 | 0 | 0 | 0 | 0 | 0 | 0 | | | | | | |
| 2224 | 02537 | 02223 | 1 | | 0 | 0 | 0 | 0 | 0 | 1 | 0 | 0 | 0 | 0 | 0 | 0 | 0 | 0 | 0 | 0 | 0 | 0 | 0 | 0 | 0 | 0 | 0 | 0 | 0 | | | | | | |
| 2225 | 02538 | 02224 | 2 | | 0 | 0 | 0 | 0 | 0 | 1 | 0 | 0 | 0 | 0 | 0 | 0 | 0 | 0 | 0 | 0 | 0 | 0 | 0 | 0 | 0 | 0 | 0 | 1 | 0 | | | | | | |
| 2226 | 02540 | 02225 | 1 | | 0 | 0 | 0 | 0 | 0 | 1 | 0 | 0 | 0 | 0 | 0 | 0 | 0 | 0 | 0 | 0 | 0 | 0 | 0 | 0 | 0 | 0 | 0 | 0 | 0 | | | | | | |
| 2227 | 02542 | 02226 | 33 | | 0 | 0 | 0 | 0 | 0 | 1 | 4 | 0 | 0 | 0 | 12 | 1 | 0 | 0 | 14 | 1 | 0 | 0 | 0 | 0 | 0 | 0 | 0 | 0 | 0 | M87790 | 97.9 | 96 | 1 | 782 | 877 |
| 2228 | 02543 | 02227 | 2 | | 0 | 0 | 0 | 0 | 0 | 1 | 0 | 0 | 0 | 0 | 0 | 0 | 0 | 0 | 0 | 0 | 0 | 0 | 0 | 0 | 1 | 0 | 0 | 0 | 0 | | | | | | |
| 2229 | 02544 | 02228 | 1 | | 0 | 0 | 0 | 0 | 0 | 1 | 0 | 0 | 0 | 0 | 0 | 0 | 0 | 0 | 0 | 0 | 0 | 0 | 0 | 0 | 0 | 0 | 0 | 0 | 0 | | | | | | |
| 2230 | 02545 | 02229 | 1 | | 0 | 0 | 0 | 0 | 0 | 1 | 0 | 0 | 0 | 0 | 0 | 0 | 0 | 0 | 0 | 0 | 0 | 0 | 0 | 0 | 0 | 0 | 0 | 0 | 0 | | | | | | |
| 2231 | 02546 | 02230 | 19 | | 0 | 0 | 0 | 0 | 0 | 1 | 0 | 0 | 0 | 0 | 18 | 0 | 0 | 0 | 0 | 0 | 0 | 0 | 0 | 0 | 0 | 0 | 0 | 0 | 0 | | | | | | |
| 2232 | 02547 | 02231 | 1 | | 0 | 0 | 0 | 0 | 0 | 1 | 0 | 0 | 0 | 0 | 0 | 0 | 0 | 0 | 0 | 0 | 0 | 0 | 0 | 0 | 0 | 0 | 0 | 0 | 0 | | | | | | |
| 2233 | 02548 | 02232 | 8 | | 0 | 0 | 0 | 0 | 0 | 1 | 0 | 1 | 0 | 1 | 0 | 0 | 0 | 0 | 1 | 1 | 0 | 0 | 0 | 2 | 1 | 0 | 0 | 0 | 0 | | | | | | |
| 2234 | 02549 | 02233 | 1 | | 0 | 0 | 0 | 0 | 0 | 1 | 0 | 0 | 0 | 0 | 0 | 0 | 0 | 0 | 0 | 0 | 0 | 0 | 0 | 0 | 0 | 0 | 0 | 0 | 0 | | | | | | |
| 2235 | 02551 | 02234 | 2 | | 0 | 0 | 0 | 0 | 0 | 1 | 0 | 0 | 0 | 0 | 0 | 0 | 0 | 0 | 1 | 0 | 0 | 0 | 0 | 0 | 0 | 0 | 0 | 0 | 0 | J03826 | 97.7 | 86 | 1 | 1745 | 1830 |
| 2236 | 02552 | 02235 | 1 | | 0 | 0 | 0 | 0 | 0 | 1 | 0 | 0 | 0 | 0 | 0 | 0 | 0 | 0 | 0 | 0 | 0 | 0 | 0 | 0 | 0 | 0 | 0 | 0 | 0 | | | | | | |
| 2237 | 02553 | 02236 | 1 | | 0 | 0 | 0 | 0 | 0 | 1 | 0 | 0 | 0 | 0 | 0 | 0 | 0 | 0 | 0 | 0 | 0 | 0 | 0 | 0 | 0 | 0 | 0 | 0 | 0 | | | | | | |
| 2238 | 02554 | 02237 | 1 | | 0 | 0 | 0 | 0 | 0 | 1 | 0 | 0 | 0 | 0 | 0 | 0 | 0 | 0 | 0 | 0 | 0 | 0 | 0 | 0 | 0 | 0 | 0 | 0 | 0 | | | | | | |
| 2239 | 02555 | 02238 | 8 | | 0 | 0 | 0 | 0 | 0 | 2 | 0 | 2 | 0 | 0 | 0 | 0 | 0 | 0 | 2 | 0 | 0 | 0 | 0 | 0 | 1 | 0 | 0 | 0 | 1 | X51442 | 100 | 86 | 1 | 182 | 269 |
| 2240 | 02556 | 02239 | 1 | | 0 | 0 | 0 | 0 | 0 | 1 | 0 | 0 | 0 | 0 | 0 | 0 | 0 | 0 | 0 | 0 | 0 | 0 | 0 | 0 | 0 | 0 | 0 | 0 | 0 | | | | | | |
| 2241 | 02557 | 02240 | 5 | | 0 | 0 | 0 | 0 | 0 | 1 | 0 | 0 | 1 | 2 | 0 | 0 | 0 | 0 | 0 | 0 | 0 | 1 | 0 | 0 | 0 | 0 | 0 | 0 | 0 | | | | | | |
| 2242 | 02559 | 02241 | 1 | | 0 | 0 | 0 | 0 | 0 | 1 | 0 | 0 | 0 | 0 | 0 | 0 | 0 | 0 | 0 | 0 | 0 | 0 | 0 | 0 | 0 | 0 | 0 | 0 | 0 | X02750 | 97.6 | 85 | 1 | 1696 | 1843 |
| 2243 | 02560 | 02242 | 1 | | 0 | 0 | 0 | 0 | 0 | 1 | 0 | 0 | 0 | 0 | 0 | 0 | 0 | 0 | 0 | 0 | 0 | 0 | 0 | 0 | 0 | 0 | 0 | 0 | 0 | | | | | | |
| 2244 | 02562 | 02243 | 1 | | 0 | 0 | 0 | 0 | 0 | 1 | 0 | 0 | 0 | 0 | 0 | 0 | 0 | 0 | 0 | 0 | 0 | 0 | 0 | 0 | 0 | 0 | 0 | 0 | 0 | | | | | | |
| 2245 | 02563 | 02244 | 5 | | 0 | 0 | 0 | 0 | 0 | 2 | 0 | 0 | 2 | 0 | 0 | 0 | 0 | 0 | 0 | 0 | 0 | 0 | 0 | 0 | 0 | 1 | 0 | 0 | 0 | | | | | | |
| 2246 | 02564 | 02245 | 5 | | 0 | 0 | 0 | 0 | 0 | 4 | 0 | 0 | 0 | 0 | 0 | 0 | 0 | 0 | 0 | 0 | 0 | 0 | 0 | 0 | 0 | 0 | 1 | 0 | 0 | | | | | | |
| 2247 | 02565 | 02246 | 4 | | 0 | 0 | 0 | 0 | 0 | 1 | 2 | 1 | 0 | 0 | 0 | 0 | 0 | 0 | 0 | 0 | 0 | 0 | 0 | 0 | 0 | 0 | 0 | 0 | 0 | | | | | | |
| 2248 | 02567 | 02247 | 2 | | 0 | 0 | 0 | 0 | 0 | 2 | 0 | 0 | 0 | 0 | 0 | 0 | 0 | 0 | 0 | 0 | 0 | 0 | 0 | 0 | 0 | 0 | 0 | 0 | 0 | X06465 | 93.8 | 64 | 1 | 795 | 857 |
| 2249 | 02568 | 02248 | 1 | | 0 | 0 | 0 | 0 | 0 | 1 | 0 | 0 | 0 | 0 | 0 | 0 | 0 | 0 | 0 | 0 | 0 | 0 | 0 | 0 | 0 | 0 | 0 | 0 | 0 | | | | | | |
| 2250 | 02569 | 02249 | 3 | | 0 | 0 | 0 | 0 | 0 | 3 | 0 | 0 | 0 | 0 | 0 | 0 | 0 | 0 | 0 | 0 | 0 | 0 | 0 | 0 | 0 | 0 | 0 | 0 | 0 | | | | | | |
| 2251 | 02570 | 02250 | 2 | | 0 | 0 | 0 | 0 | 0 | 1 | 0 | 0 | 0 | 0 | 0 | 0 | 0 | 0 | 0 | 0 | 0 | 0 | 1 | 0 | 0 | 0 | 0 | 0 | 0 | | | | | | |
| 2252 | 02571 | 02251 | 9 | | 0 | 0 | 0 | 0 | 0 | 1 | 1 | 0 | 0 | 0 | 1 | 0 | 0 | 2 | 0 | 0 | 1 | 0 | 0 | 3 | 0 | 0 | 0 | 0 | 0 | | | | | | |
| 2253 | 02572 | 02252 | 1 | | 0 | 0 | 0 | 0 | 0 | 1 | 0 | 0 | 0 | 0 | 0 | 0 | 0 | 0 | 0 | 0 | 0 | 0 | 0 | 0 | 0 | 0 | 0 | 0 | 0 | L05144 | 97.1 | 70 | 1 | 2291 | 2657 |

Table 63

| | A | B | C | E | G | I | K | M | O | Q | S | U | W | Y | AA | AC | AE | AG | AI | AK | AM | AO | AQ | AS | AU | AW | AY | BA | BC | BE | BF | BG | BH | BI | BK |
|---|---|---|---|---|---|---|---|---|---|---|---|---|---|---|---|---|---|---|---|---|---|---|---|---|---|---|---|---|---|---|---|---|---|---|---|
| 2254 | 02573 | 02253 | 1 | | 0 | 0 | 0 | 0 | 0 | 1 | 0 | 0 | 0 | 0 | 0 | 0 | 0 | 0 | 0 | 0 | 0 | 0 | 0 | 0 | 0 | 0 | 0 | 0 | 0 | | | | | | |
| 2255 | 02574 | 02254 | 2 | | 0 | 0 | 0 | 0 | 0 | 2 | 0 | 0 | 0 | 0 | 0 | 0 | 0 | 0 | 0 | 0 | 0 | 0 | 0 | 0 | 0 | 0 | 0 | 0 | 0 | | | | | | |
| 2256 | 02575 | 02255 | 3 | | 0 | 0 | 0 | 0 | 0 | 3 | 0 | 0 | 0 | 0 | 0 | 0 | 0 | 0 | 0 | 0 | 0 | 0 | 0 | 0 | 0 | 0 | 0 | 0 | 0 | | | | | | |
| 2257 | 02576 | 02256 | 2 | | 0 | 0 | 0 | 0 | 0 | 2 | 0 | 0 | 0 | 0 | 0 | 0 | 0 | 0 | 0 | 0 | 0 | 0 | 0 | 0 | 0 | 0 | 0 | 0 | 0 | | | | | | |
| 2258 | 02577 | 02257 | 3 | | 0 | 0 | 0 | 0 | 0 | 2 | 0 | 0 | 0 | 0 | 0 | 0 | 0 | 1 | 0 | 0 | 0 | 0 | 0 | 0 | 0 | 0 | 0 | 0 | 0 | | | | | | |
| 2259 | 02578 | 02258 | 1 | | 0 | 0 | 0 | 0 | 0 | 1 | 0 | 0 | 0 | 0 | 0 | 0 | 0 | 0 | 0 | 0 | 0 | 0 | 0 | 0 | 0 | 0 | 0 | 0 | 0 | | | | | | |
| 2260 | 02580 | 02259 | 1 | | 0 | 0 | 0 | 0 | 0 | 1 | 0 | 0 | 0 | 0 | 0 | 0 | 0 | 0 | 0 | 0 | 0 | 0 | 0 | 0 | 0 | 0 | 0 | 0 | 0 | | | | | | |
| 2261 | 02581 | 02260 | 4 | | 0 | 0 | 0 | 0 | 0 | 1 | 0 | 0 | 0 | 0 | 0 | 0 | 0 | 1 | 0 | 1 | 0 | 1 | 0 | 0 | 0 | 0 | 0 | 0 | 0 | | | | | | |
| 2262 | 02582 | 02261 | 5 | | 0 | 0 | 0 | 0 | 0 | 1 | 0 | 0 | 0 | 0 | 0 | 0 | 0 | 0 | 0 | 0 | 2 | 0 | 0 | 0 | 0 | 0 | 2 | 0 | 0 | | | | | | |
| 2263 | 02583 | 02262 | 2 | | 0 | 0 | 0 | 0 | 0 | 2 | 0 | 0 | 0 | 0 | 0 | 0 | 0 | 0 | 0 | 0 | 0 | 0 | 0 | 0 | 0 | 0 | 0 | 0 | 0 | | | | | | |
| 2264 | 02585 | 02263 | 3 | | 0 | 0 | 0 | 0 | 0 | 1 | 0 | 1 | 0 | 0 | 1 | 0 | 0 | 0 | 0 | 0 | 0 | 0 | 0 | 0 | 0 | 0 | 0 | 0 | 0 | | | | | | |
| 2265 | 02586 | 02264 | 5 | | 0 | 0 | 0 | 0 | 0 | 1 | 0 | 0 | 1 | 0 | 0 | 0 | 0 | 0 | 0 | 0 | 0 | 0 | 0 | 0 | 0 | 1 | 0 | 2 | 0 | X15822 | 100 | 55 | 1 | 354 | 408 |
| 2266 | 02588 | 02265 | 1 | | 0 | 0 | 0 | 0 | 0 | 1 | 0 | 0 | 0 | 0 | 0 | 0 | 0 | 0 | 0 | 0 | 0 | 0 | 0 | 0 | 0 | 0 | 0 | 0 | 0 | M27492 | 100 | 55 | 1 | 4856 | 4910 |
| 2267 | 02589 | 02266 | 2 | | 0 | 0 | 0 | 0 | 0 | 1 | 0 | 0 | 0 | 0 | 0 | 0 | 0 | 0 | 0 | 1 | 0 | 0 | 0 | 0 | 0 | 0 | 0 | 0 | 0 | X06596 | 100 | 51 | 1 | 2566 | 2616 |
| 2268 | 02590 | 02267 | 1 | | 0 | 0 | 0 | 0 | 0 | 1 | 0 | 0 | 0 | 0 | 0 | 0 | 0 | 0 | 0 | 0 | 0 | 0 | 0 | 0 | 0 | 0 | 0 | 0 | 0 | M21941 | 96 | 50 | 1 | 1745 | 1807 |
| 2269 | 02591 | 02268 | 2 | | 0 | 0 | 0 | 0 | 0 | 1 | 0 | 0 | 1 | 0 | 0 | 0 | 0 | 0 | 0 | 0 | 0 | 0 | 0 | 0 | 0 | 0 | 0 | 0 | 0 | | | | | | |
| 2270 | 02592 | 02269 | 1 | | 0 | 0 | 0 | 0 | 0 | 1 | 0 | 0 | 0 | 0 | 0 | 0 | 0 | 0 | 0 | 0 | 0 | 0 | 0 | 0 | 0 | 0 | 0 | 0 | 0 | | | | | | |
| 2271 | 02593 | 02270 | 2 | | 0 | 0 | 0 | 0 | 0 | 1 | 0 | 0 | 0 | 0 | 0 | 0 | 0 | 1 | 0 | 0 | 0 | 0 | 0 | 0 | 0 | 0 | 0 | 0 | 0 | | | | | | |
| 2272 | 02612 | 02271 | 3 | | 0 | 0 | 0 | 0 | 0 | 1 | 0 | 1 | 0 | 1 | 0 | 0 | 0 | 0 | 0 | 0 | 0 | 0 | 0 | 0 | 0 | 0 | 0 | 0 | 0 | | | | | | |
| 2273 | 02627 | 02272 | 1 | | 0 | 0 | 0 | 0 | 0 | 0 | 1 | 0 | 0 | 0 | 0 | 0 | 0 | 0 | 0 | 0 | 0 | 0 | 0 | 0 | 0 | 0 | 0 | 0 | 0 | | | | | | |
| 2274 | 02628 | 02273 | 1 | | 0 | 0 | 0 | 0 | 0 | 0 | 1 | 0 | 0 | 0 | 0 | 0 | 0 | 0 | 0 | 0 | 0 | 0 | 0 | 0 | 0 | 0 | 0 | 0 | 0 | L12136 | 90.8 | 524 | 1 | 959 | 1831 |
| 2275 | 02629 | 02274 | 1 | | 0 | 0 | 0 | 0 | 0 | 0 | 1 | 0 | 0 | 0 | 0 | 0 | 0 | 0 | 0 | 0 | 0 | 0 | 0 | 0 | 0 | 0 | 0 | 0 | 0 | M31158 | 93.6 | 456 | 1 | 2456 | 3259 |
| 2276 | 02630 | 02275 | 1 | | 0 | 0 | 0 | 0 | 0 | 0 | 1 | 0 | 0 | 0 | 0 | 0 | 0 | 0 | 0 | 0 | 0 | 0 | 0 | 0 | 0 | 0 | 0 | 0 | 0 | | | | | | |
| 2277 | 02631 | 02276 | 1 | | 0 | 0 | 0 | 0 | 0 | 0 | 1 | 0 | 0 | 0 | 0 | 0 | 0 | 0 | 0 | 0 | 0 | 0 | 0 | 0 | 0 | 0 | 0 | 0 | 0 | | | | | | |
| 2278 | 02632 | 02277 | 1 | | 0 | 0 | 0 | 0 | 0 | 0 | 1 | 0 | 0 | 0 | 0 | 0 | 0 | 0 | 0 | 0 | 0 | 0 | 0 | 0 | 0 | 0 | 0 | 0 | 0 | M31899 | 96.4 | 446 | 1 | 2302 | 2751 |
| 2279 | 02633 | 02278 | 1 | | 0 | 0 | 0 | 0 | 0 | 0 | 1 | 0 | 0 | 0 | 0 | 0 | 0 | 0 | 0 | 0 | 0 | 0 | 0 | 0 | 0 | 0 | 0 | 0 | 0 | | | | | | |
| 2280 | 02634 | 02279 | 1 | | 0 | 0 | 0 | 0 | 0 | 0 | 1 | 0 | 0 | 0 | 0 | 0 | 0 | 0 | 0 | 0 | 0 | 0 | 0 | 0 | 0 | 0 | 0 | 0 | 0 | | | | | | |
| 2281 | 02635 | 02280 | 5 | | 0 | 0 | 0 | 0 | 0 | 0 | 2 | 0 | 0 | 0 | 0 | 0 | 0 | 0 | 0 | 2 | 0 | 0 | 1 | 0 | 0 | 0 | 0 | 0 | 0 | | | | | | |
| 2282 | 02637 | 02281 | 1 | | 0 | 0 | 0 | 0 | 0 | 0 | 1 | 0 | 0 | 0 | 0 | 0 | 0 | 0 | 0 | 0 | 0 | 0 | 0 | 0 | 0 | 0 | 0 | 0 | 0 | | | | | | |
| 2283 | 02638 | 02282 | 3 | | 0 | 0 | 0 | 0 | 0 | 0 | 1 | 0 | 0 | 0 | 0 | 0 | 0 | 0 | 0 | 0 | 0 | 0 | 0 | 1 | 0 | 1 | 0 | 0 | 0 | | | | | | |
| 2284 | 02639 | 02283 | 1 | | 0 | 0 | 0 | 0 | 0 | 0 | 1 | 0 | 0 | 0 | 0 | 0 | 0 | 0 | 0 | 0 | 0 | 0 | 0 | 0 | 0 | 0 | 0 | 0 | 0 | | | | | | |
| 2285 | 02640 | 02284 | 9 | | 0 | 0 | 0 | 0 | 0 | 0 | 1 | 3 | 0 | 1 | 0 | 0 | 0 | 0 | 0 | 1 | 1 | 1 | 1 | 0 | 0 | 0 | 0 | 0 | 0 | | | | | | |
| 2286 | 02641 | 02285 | 4 | | 0 | 0 | 0 | 0 | 0 | 0 | 1 | 0 | 0 | 0 | 0 | 2 | 0 | 1 | 0 | 0 | 0 | 0 | 0 | 0 | 0 | 0 | 0 | 0 | 0 | | | | | | |
| 2287 | 02642 | 02286 | 3 | | 0 | 0 | 0 | 0 | 0 | 0 | 1 | 0 | 1 | 1 | 0 | 0 | 0 | 0 | 0 | 0 | 0 | 0 | 0 | 0 | 0 | 0 | 0 | 0 | 0 | | | | | | |
| 2288 | 02643 | 02287 | 4 | | 0 | 0 | 0 | 0 | 0 | 0 | 1 | 0 | 0 | 1 | 0 | 1 | 0 | 0 | 0 | 0 | 0 | 0 | 0 | 0 | 0 | 1 | 0 | 0 | 0 | | | | | | |
| 2289 | 02644 | 02288 | 1 | | 0 | 0 | 0 | 0 | 0 | 0 | 1 | 0 | 0 | 0 | 0 | 0 | 0 | 0 | 0 | 0 | 0 | 0 | 0 | 0 | 0 | 0 | 0 | 0 | 0 | | | | | | |

Table 64

| | A | B | C | E | G | I | K | M | O | Q | S | U | W | Y | AA | AC | AE | AG | AI | AK | AM | AO | AQ | AS | AU | AW | AY | BA | BC | BE | BF | BG | BH | BI | BK |
|---|---|---|---|---|---|---|---|---|---|---|---|---|---|---|---|---|---|---|---|---|---|---|---|---|---|---|---|---|---|---|---|---|---|---|---|
| 2290 | 02645 | 02289 | 1 | | 0 | 0 | 0 | 0 | 0 | 0 | 1 | 0 | 0 | 0 | 0 | 0 | 0 | 0 | 0 | 0 | 0 | 0 | 0 | 0 | 0 | 0 | 0 | 0 | 0 | | | | | | |
| 2291 | 02646 | 02290 | 1 | | 0 | 0 | 0 | 0 | 0 | 0 | 1 | 0 | 0 | 0 | 0 | 0 | 0 | 0 | 0 | 0 | 0 | 0 | 0 | 0 | 0 | 0 | 0 | 0 | 0 | | | | | | |
| 2292 | 02648 | 02291 | 1 | | 0 | 0 | 0 | 0 | 0 | 0 | 1 | 0 | 0 | 0 | 0 | 0 | 0 | 0 | 0 | 0 | 0 | 0 | 0 | 0 | 0 | 0 | 0 | 0 | 0 | | | | | | |
| 2293 | 02650 | 02292 | 1 | | 0 | 0 | 0 | 0 | 0 | 0 | 1 | 0 | 0 | 0 | 0 | 0 | 0 | 0 | 0 | 0 | 0 | 0 | 0 | 0 | 0 | 0 | 0 | 0 | 0 | | | | | | |
| 2294 | 02651 | 02293 | 1 | | 0 | 0 | 0 | 0 | 0 | 0 | 1 | 0 | 0 | 0 | 0 | 0 | 0 | 0 | 0 | 0 | 0 | 0 | 0 | 0 | 0 | 0 | 0 | 0 | 0 | | | | | | |
| 2295 | 02652 | 02294 | 1 | | 0 | 0 | 0 | 0 | 0 | 0 | 1 | 0 | 0 | 0 | 0 | 0 | 0 | 0 | 0 | 0 | 0 | 0 | 0 | 0 | 0 | 0 | 0 | 0 | 0 | L28010 | 97.3 | 331 | 1 | 1560 | 1902 |
| 2296 | 02653 | 02295 | 1 | | 0 | 0 | 0 | 0 | 0 | 0 | 1 | 0 | 0 | 0 | 0 | 0 | 0 | 0 | 0 | 0 | 0 | 0 | 0 | 0 | 0 | 0 | 0 | 0 | 0 | | | | | | |
| 2297 | 02654 | 02296 | 2 | | 0 | 0 | 0 | 0 | 0 | 0 | 1 | 0 | 0 | 0 | 0 | 0 | 1 | 0 | 0 | 0 | 0 | 0 | 0 | 0 | 0 | 0 | 0 | 0 | 0 | | | | | | |
| 2298 | 02656 | 02297 | 20 | | 0 | 0 | 0 | 0 | 0 | 0 | 3 | 0 | 0 | 1 | 2 | 2 | 0 | 2 | 3 | 2 | 0 | 0 | 0 | 3 | 1 | 1 | 0 | 0 | 0 | X00497 | 92.3 | 351 | 1 | 962 | 1304 |
| 2299 | 02657 | 02298 | 1 | | 0 | 0 | 0 | 0 | 0 | 0 | 1 | 0 | 0 | 0 | 0 | 0 | 0 | 0 | 0 | 0 | 0 | 0 | 0 | 0 | 0 | 0 | 0 | 0 | 0 | | | | | | |
| 2300 | 02658 | 02299 | 6 | | 0 | 0 | 0 | 0 | 0 | 0 | 1 | 0 | 0 | 0 | 1 | 0 | 0 | 0 | 1 | 0 | 0 | 0 | 0 | 0 | 1 | 0 | 0 | 2 | 0 | | | | | | |
| 2301 | 02659 | 02300 | 2 | | 0 | 0 | 0 | 0 | 0 | 0 | 1 | 0 | 0 | 0 | 0 | 0 | 0 | 0 | 0 | 0 | 0 | 0 | 0 | 1 | 0 | 0 | 0 | 0 | 0 | | | | | | |
| 2302 | 02660 | 02301 | 1 | | 0 | 0 | 0 | 0 | 0 | 0 | 1 | 0 | 0 | 0 | 0 | 0 | 0 | 0 | 0 | 0 | 0 | 0 | 0 | 0 | 0 | 0 | 0 | 0 | 0 | | | | | | |
| 2303 | 02661 | 02302 | 3 | | 0 | 0 | 0 | 0 | 0 | 0 | 1 | 0 | 1 | 0 | 0 | 0 | 0 | 0 | 0 | 0 | 0 | 0 | 0 | 0 | 0 | 0 | 1 | 0 | 0 | | | | | | |
| 2304 | 02662 | 02303 | 2 | | 0 | 0 | 0 | 0 | 0 | 0 | 1 | 0 | 0 | 0 | 0 | 0 | 0 | 0 | 0 | 0 | 0 | 0 | 0 | 0 | 0 | 0 | 1 | 0 | 0 | | | | | | |
| 2305 | 02663 | 02304 | 4 | | 0 | 0 | 0 | 0 | 0 | 0 | 1 | 0 | 0 | 1 | 0 | 0 | 0 | 0 | 0 | 0 | 0 | 2 | 0 | 0 | 0 | 0 | 0 | 0 | 0 | M14219 | 91.5 | 319 | 1 | 1004 | 1778 |
| 2306 | 02664 | 02305 | 5 | | 0 | 0 | 0 | 0 | 0 | 0 | 2 | 0 | 0 | 0 | 0 | 0 | 0 | 0 | 0 | 2 | 1 | 0 | 0 | 0 | 0 | 0 | 0 | 0 | 0 | Z15008 | 97.9 | 233 | 1 | 4204 | 5200 |
| 2307 | 02665 | 02306 | 1 | | 0 | 0 | 0 | 0 | 0 | 0 | 1 | 0 | 0 | 0 | 0 | 0 | 0 | 0 | 0 | 0 | 0 | 0 | 0 | 0 | 0 | 0 | 0 | 0 | 0 | | | | | | |
| 2308 | 02666 | 02307 | 2 | | 0 | 0 | 0 | 0 | 0 | 0 | 2 | 0 | 0 | 0 | 0 | 0 | 0 | 0 | 0 | 0 | 0 | 0 | 0 | 0 | 0 | 0 | 0 | 0 | 0 | | | | | | |
| 2309 | 02667 | 02308 | 1 | | 0 | 0 | 0 | 0 | 0 | 0 | 1 | 0 | 0 | 0 | 0 | 0 | 0 | 0 | 0 | 0 | 0 | 0 | 0 | 0 | 0 | 0 | 0 | 0 | 0 | | | | | | |
| 2310 | 02668 | 02309 | 1 | | 0 | 0 | 0 | 0 | 0 | 0 | 1 | 0 | 0 | 0 | 0 | 0 | 0 | 0 | 0 | 0 | 0 | 0 | 0 | 0 | 0 | 0 | 0 | 0 | 0 | | | | | | |
| 2311 | 02669 | 02310 | 1 | | 0 | 0 | 0 | 0 | 0 | 0 | 1 | 0 | 0 | 0 | 0 | 0 | 0 | 0 | 0 | 0 | 0 | 0 | 0 | 0 | 0 | 0 | 0 | 0 | 0 | | | | | | |
| 2312 | 02670 | 02311 | 1 | | 0 | 0 | 0 | 0 | 0 | 0 | 1 | 0 | 0 | 0 | 0 | 0 | 0 | 0 | 0 | 0 | 0 | 0 | 0 | 0 | 0 | 0 | 0 | 0 | 0 | | | | | | |
| 2313 | 02672 | 02312 | 5 | | 0 | 0 | 0 | 0 | 0 | 0 | 1 | 0 | 1 | 1 | 0 | 0 | 0 | 0 | 0 | 0 | 0 | 1 | 0 | 0 | 1 | 0 | 0 | 0 | 0 | | | | | | |
| 2314 | 02673 | 02313 | 5 | | 0 | 0 | 0 | 0 | 0 | 0 | 1 | 1 | 0 | 0 | 3 | 0 | 0 | 0 | 0 | 0 | 0 | 0 | 0 | 0 | 0 | 0 | 0 | 0 | 0 | L21936 | 98.8 | 320 | 2 | 1957 | 2277 |
| 2315 | 02675 | 02314 | 2 | | 0 | 0 | 0 | 0 | 0 | 0 | 2 | 0 | 0 | 0 | 0 | 0 | 0 | 0 | 0 | 0 | 0 | 0 | 0 | 0 | 0 | 0 | 0 | 0 | 0 | | | | | | |
| 2316 | 02676 | 02315 | 2 | | 0 | 0 | 0 | 0 | 0 | 0 | 1 | 0 | 0 | 0 | 0 | 0 | 0 | 0 | 1 | 0 | 0 | 0 | 0 | 0 | 0 | 0 | 0 | 0 | 0 | | | | | | |
| 2317 | 02677 | 02316 | 2 | | 0 | 0 | 0 | 0 | 0 | 0 | 1 | 0 | 0 | 0 | 0 | 0 | 0 | 0 | 1 | 0 | 0 | 0 | 0 | 0 | 0 | 0 | 0 | 0 | 0 | | | | | | |
| 2318 | 02678 | 02317 | 2 | | 0 | 0 | 0 | 0 | 0 | 0 | 1 | 0 | 0 | 0 | 0 | 1 | 0 | 0 | 0 | 0 | 0 | 0 | 0 | 0 | 0 | 0 | 0 | 0 | 0 | | | | | | |
| 2319 | 02679 | 02318 | 2 | | 0 | 0 | 0 | 0 | 0 | 0 | 1 | 0 | 0 | 0 | 0 | 0 | 0 | 0 | 0 | 0 | 0 | 1 | 0 | 0 | 0 | 0 | 0 | 0 | 0 | | | | | | |
| 2320 | 02680 | 02319 | 1 | | 0 | 0 | 0 | 0 | 0 | 0 | 1 | 0 | 0 | 0 | 0 | 0 | 0 | 0 | 0 | 0 | 0 | 0 | 0 | 0 | 0 | 0 | 0 | 0 | 0 | M18737 | 99 | 303 | 1 | 541 | 849 |
| 2321 | 02681 | 02320 | 1 | | 0 | 0 | 0 | 0 | 0 | 0 | 1 | 0 | 0 | 0 | 0 | 0 | 0 | 0 | 0 | 0 | 0 | 0 | 0 | 0 | 0 | 0 | 0 | 0 | 0 | | | | | | |
| 2322 | 02682 | 02321 | 1 | | 0 | 0 | 0 | 0 | 0 | 0 | 1 | 0 | 0 | 0 | 0 | 0 | 0 | 0 | 0 | 0 | 0 | 0 | 0 | 0 | 0 | 0 | 0 | 0 | 0 | M18216 | 97 | 296 | 1 | 2232 | 2527 |
| 2323 | 02684 | 02322 | 13 | | 0 | 0 | 0 | 0 | 0 | 0 | 1 | 0 | 5 | 3 | 0 | 0 | 0 | 0 | 0 | 1 | 1 | 2 | 0 | 0 | 0 | 0 | 0 | 0 | 0 | X14787 | 96.1 | 284 | 1 | 5182 | 5722 |
| 2324 | 02685 | 02323 | 1 | | 0 | 0 | 0 | 0 | 0 | 0 | 1 | 0 | 0 | 0 | 0 | 0 | 0 | 0 | 0 | 0 | 0 | 0 | 0 | 0 | 0 | 0 | 0 | 0 | 0 | | | | | | |
| 2325 | 02686 | 02324 | 1 | | 0 | 0 | 0 | 0 | 0 | 0 | 1 | 0 | 0 | 0 | 0 | 0 | 0 | 0 | 0 | 0 | 0 | 0 | 0 | 0 | 0 | 0 | 0 | 0 | 0 | | | | | | |

Table 65

| | A | B | C | E | G | I | K | M | O | Q | S | U | W | Y | AA | AC | AE | AG | AI | AK | AM | AO | AQ | AS | AU | AW | AY | BA | BC | BE | BF | BG | BH | BI | BK |
|---|---|---|---|---|---|---|---|---|---|---|---|---|---|---|---|---|---|---|---|---|---|---|---|---|---|---|---|---|---|---|---|---|---|---|---|
| 2326 | 02687 | 02325 | 2 | | 0 | 0 | 0 | 0 | 0 | 0 | 1 | 0 | 0 | 0 | 0 | 0 | 1 | 0 | 0 | 0 | 0 | 0 | 0 | 0 | 0 | 0 | 0 | 0 | 0 | | | | | | |
| 2327 | 02688 | 02326 | 2 | | 0 | 0 | 0 | 0 | 0 | 0 | 1 | 1 | 0 | 0 | 0 | 0 | 0 | 0 | 0 | 0 | 0 | 0 | 0 | 0 | 0 | 0 | 0 | 0 | 0 | | | | | | |
| 2328 | 02689 | 02327 | 4 | | 0 | 0 | 0 | 0 | 0 | 0 | 2 | 0 | 0 | 0 | 0 | 0 | 0 | 0 | 0 | 0 | 0 | 1 | 0 | 0 | 1 | 0 | 0 | 0 | 0 | | | | | | |
| 2329 | 02690 | 02328 | 4 | | 0 | 0 | 0 | 0 | 0 | 0 | 1 | 0 | 1 | 0 | 0 | 0 | 0 | 0 | 0 | 0 | 1 | 0 | 0 | 1 | 0 | 0 | 0 | 0 | 0 | | | | | | |
| 2330 | 02691 | 02329 | 1 | | 0 | 0 | 0 | 0 | 0 | 0 | 1 | 0 | 0 | 0 | 0 | 0 | 0 | 0 | 0 | 0 | 0 | 0 | 0 | 0 | 0 | 0 | 0 | 0 | 0 | M55542 | 97.6 | 289 | 1 | 2370 | 2881 |
| 2331 | 02692 | 02330 | 3 | | 0 | 0 | 0 | 0 | 0 | 0 | 1 | 0 | 0 | 0 | 0 | 1 | 0 | 0 | 0 | 0 | 0 | 0 | 0 | 0 | 0 | 1 | 0 | 0 | 0 | | | | | | |
| 2332 | 02693 | 02331 | 1 | | 0 | 0 | 0 | 0 | 0 | 0 | 1 | 0 | 0 | 0 | 0 | 0 | 0 | 0 | 0 | 0 | 0 | 0 | 0 | 0 | 0 | 0 | 0 | 0 | 0 | J03553 | 97.8 | 276 | 1 | 504 | 991 |
| 2333 | 02694 | 02332 | 1 | | 0 | 0 | 0 | 0 | 0 | 0 | 1 | 0 | 0 | 0 | 0 | 0 | 0 | 0 | 0 | 0 | 0 | 0 | 0 | 0 | 0 | 0 | 0 | 0 | 0 | | | | | | |
| 2334 | 02695 | 02333 | 5 | | 0 | 0 | 0 | 0 | 0 | 0 | 2 | 0 | 0 | 1 | 0 | 0 | 1 | 0 | 0 | 0 | 0 | 0 | 1 | 0 | 0 | 0 | 0 | 0 | 0 | X72964 | 100 | 268 | 1 | 813 | 1087 |
| 2335 | 02696 | 02334 | 1 | | 0 | 0 | 0 | 0 | 0 | 0 | 1 | 0 | 0 | 0 | 0 | 0 | 0 | 0 | 0 | 0 | 0 | 0 | 0 | 0 | 0 | 0 | 0 | 0 | 0 | | | | | | |
| 2336 | 02697 | 02335 | 2 | | 0 | 0 | 0 | 0 | 0 | 0 | 1 | 0 | 0 | 0 | 0 | 0 | 0 | 0 | 0 | 0 | 0 | 0 | 0 | 1 | 0 | 0 | 0 | 0 | 0 | | | | | | |
| 2337 | 02698 | 02336 | 1 | | 0 | 0 | 0 | 0 | 0 | 0 | 1 | 0 | 0 | 0 | 0 | 0 | 0 | 0 | 0 | 0 | 0 | 0 | 0 | 0 | 0 | 0 | 0 | 0 | 0 | | | | | | |
| 2338 | 02699 | 02337 | 2 | | 0 | 0 | 0 | 0 | 0 | 0 | 1 | 0 | 0 | 0 | 0 | 0 | 0 | 0 | 0 | 0 | 0 | 1 | 0 | 0 | 0 | 0 | 0 | 0 | 0 | | | | | | |
| 2339 | 02700 | 02338 | 1 | | 0 | 0 | 0 | 0 | 0 | 0 | 1 | 0 | 0 | 0 | 0 | 0 | 0 | 0 | 0 | 0 | 0 | 0 | 0 | 0 | 0 | 0 | 0 | 0 | 0 | | | | | | |
| 2340 | 02701 | 02339 | 2 | | 0 | 0 | 0 | 0 | 0 | 0 | 1 | 0 | 0 | 0 | 0 | 0 | 0 | 0 | 0 | 0 | 0 | 0 | 0 | 0 | 1 | 0 | 0 | 0 | 0 | | | | | | |
| 2341 | 02702 | 02340 | 5 | | 0 | 0 | 0 | 0 | 0 | 0 | 3 | 0 | 0 | 0 | 0 | 0 | 0 | 0 | 0 | 0 | 0 | 0 | 0 | 1 | 0 | 0 | 0 | 1 | 0 | | | | | | |
| 2342 | 02703 | 02341 | 2 | | 0 | 0 | 0 | 0 | 0 | 0 | 1 | 0 | 0 | 0 | 0 | 0 | 0 | 0 | 1 | 0 | 0 | 0 | 0 | 0 | 0 | 0 | 0 | 0 | 0 | | | | | | |
| 2343 | 02704 | 02342 | 1 | | 0 | 0 | 0 | 0 | 0 | 0 | 1 | 0 | 0 | 0 | 0 | 0 | 0 | 0 | 0 | 0 | 0 | 0 | 0 | 0 | 0 | 0 | 0 | 0 | 0 | | | | | | |
| 2344 | 02705 | 02343 | 1 | | 0 | 0 | 0 | 0 | 0 | 0 | 1 | 0 | 0 | 0 | 0 | 0 | 0 | 0 | 0 | 0 | 0 | 0 | 0 | 0 | 0 | 0 | 0 | 0 | 0 | | | | | | |
| 2345 | 02706 | 02344 | 13 | | 0 | 0 | 0 | 0 | 0 | 0 | 2 | 0 | 0 | 0 | 11 | 0 | 0 | 0 | 0 | 0 | 0 | 0 | 0 | 0 | 0 | 0 | 0 | 0 | 0 | | | | | | |
| 2346 | 02707 | 02345 | 2 | | 0 | 0 | 0 | 0 | 0 | 0 | 1 | 0 | 0 | 0 | 0 | 0 | 0 | 0 | 0 | 0 | 0 | 0 | 0 | 0 | 0 | 0 | 0 | 1 | 0 | | | | | | |
| 2347 | 02708 | 02346 | 8 | | 0 | 0 | 0 | 0 | 0 | 0 | 2 | 0 | 1 | 1 | 1 | 0 | 0 | 0 | 0 | 1 | 0 | 1 | 0 | 0 | 1 | 0 | 0 | 0 | 0 | L26165 | 95.2 | 353 | 1 | 1641 | 2098 |
| 2348 | 02709 | 02347 | 1 | | 0 | 0 | 0 | 0 | 0 | 0 | 1 | 0 | 0 | 0 | 0 | 0 | 0 | 0 | 0 | 0 | 0 | 0 | 0 | 0 | 0 | 0 | 0 | 0 | 0 | M29873 | 95.1 | 263 | 1 | 2644 | 2907 |
| 2349 | 02710 | 02348 | 1 | | 0 | 0 | 0 | 0 | 0 | 0 | 1 | 0 | 0 | 0 | 0 | 0 | 0 | 0 | 0 | 0 | 0 | 0 | 0 | 0 | 0 | 0 | 0 | 0 | 0 | | | | | | |
| 2350 | 02711 | 02349 | 2 | | 0 | 0 | 0 | 0 | 0 | 0 | 1 | 0 | 0 | 0 | 0 | 0 | 0 | 0 | 0 | 0 | 0 | 0 | 0 | 1 | 0 | 0 | 0 | 0 | 0 | | | | | | |
| 2351 | 02712 | 02350 | 8 | | 0 | 0 | 0 | 0 | 0 | 0 | 1 | 1 | 3 | 0 | 1 | 1 | 0 | 0 | 0 | 0 | 0 | 0 | 0 | 0 | 1 | 0 | 0 | 0 | 0 | X53331 | 94.1 | 392 | 1 | 217 | 603 |
| 2352 | 02713 | 02351 | 2 | | 0 | 0 | 0 | 0 | 0 | 0 | 1 | 0 | 0 | 0 | 0 | 0 | 0 | 0 | 0 | 0 | 0 | 0 | 0 | 0 | 0 | 0 | 1 | 0 | 0 | | | | | | |
| 2353 | 02714 | 02352 | 2 | | 0 | 0 | 0 | 0 | 0 | 0 | 1 | 0 | 0 | 0 | 0 | 0 | 0 | 0 | 0 | 1 | 0 | 0 | 0 | 0 | 0 | 0 | 0 | 0 | 0 | | | | | | |
| 2354 | 02715 | 02353 | 1 | | 0 | 0 | 0 | 0 | 0 | 0 | 1 | 0 | 0 | 0 | 0 | 0 | 0 | 0 | 0 | 0 | 0 | 0 | 0 | 0 | 0 | 0 | 0 | 0 | 0 | | | | | | |
| 2355 | 02716 | 02354 | 18 | | 0 | 0 | 0 | 0 | 0 | 0 | 2 | 8 | 0 | 1 | 1 | 0 | 0 | 0 | 2 | 0 | 0 | 0 | 0 | 0 | 4 | 0 | 0 | 0 | 0 | X04412 | 93.6 | 375 | 1 | 2229 | 2602 |
| 2356 | 02717 | 02355 | 1 | | 0 | 0 | 0 | 0 | 0 | 0 | 1 | 0 | 0 | 0 | 0 | 0 | 0 | 0 | 0 | 0 | 0 | 0 | 0 | 0 | 0 | 0 | 0 | 0 | 0 | | | | | | |
| 2357 | 02718 | 02356 | 1 | | 0 | 0 | 0 | 0 | 0 | 0 | 1 | 0 | 0 | 0 | 0 | 0 | 0 | 0 | 0 | 0 | 0 | 0 | 0 | 0 | 0 | 0 | 0 | 0 | 0 | | | | | | |
| 2358 | 02719 | 02357 | 2 | | 0 | 0 | 0 | 0 | 0 | 0 | 1 | 0 | 0 | 0 | 1 | 0 | 0 | 0 | 0 | 0 | 0 | 0 | 0 | 0 | 0 | 0 | 0 | 0 | 0 | | | | | | |
| 2359 | 02721 | 02358 | 1 | | 0 | 0 | 0 | 0 | 0 | 0 | 1 | 0 | 0 | 0 | 0 | 0 | 0 | 0 | 0 | 0 | 0 | 0 | 0 | 0 | 0 | 0 | 0 | 0 | 0 | | | | | | |
| 2360 | 02722 | 02359 | 1 | | 0 | 0 | 0 | 0 | 0 | 0 | 1 | 0 | 0 | 0 | 0 | 0 | 0 | 0 | 0 | 0 | 0 | 0 | 0 | 0 | 0 | 0 | 0 | 0 | 0 | M16552 | 98.4 | 252 | 1 | 3303 | 3653 |
| 2361 | 02723 | 02360 | 1 | | 0 | 0 | 0 | 0 | 0 | 0 | 1 | 0 | 0 | 0 | 0 | 0 | 0 | 0 | 0 | 0 | 0 | 0 | 0 | 0 | 0 | 0 | 0 | 0 | 0 | | | | | | |

Table 66

| | A | B | C | E | G | I | K | M | O | Q | S | U | W | Y | AA | AC | AE | AG | AI | AK | AM | AO | AQ | AS | AU | AW | AY | BA | BC | BE | BF | BG | BH | BI | BK |
|---|---|---|---|---|---|---|---|---|---|---|---|---|---|---|---|---|---|---|---|---|---|---|---|---|---|---|---|---|---|---|---|---|---|---|---|
| 2362 | 02724 | 02361 | 1 | | 0 | 0 | 0 | 0 | 0 | 0 | 1 | 0 | 0 | 0 | 0 | 0 | 0 | 0 | 0 | 0 | 0 | 0 | 0 | 0 | 0 | 0 | 0 | 0 | 0 | M83664 | 98.8 | 251 | 1 | 826 | 1501 |
| 2363 | 02725 | 02362 | 2 | | 0 | 0 | 0 | 0 | 0 | 0 | 1 | 0 | 0 | 1 | 0 | 0 | 0 | 0 | 0 | 0 | 0 | 0 | 0 | 0 | 0 | 0 | 0 | 0 | 0 | | | | | | |
| 2364 | 02727 | 02363 | 2 | | 0 | 0 | 0 | 0 | 0 | 0 | 1 | 0 | 1 | 0 | 0 | 0 | 0 | 0 | 0 | 0 | 0 | 0 | 0 | 0 | 0 | 0 | 0 | 0 | 0 | D13748 | 96.3 | 218 | 87 | 1090 | 1383 |
| 2365 | 02728 | 02364 | 6 | | 0 | 0 | 0 | 0 | 0 | 0 | 3 | 0 | 1 | 0 | 0 | 0 | 0 | 0 | 0 | 0 | 0 | 0 | 0 | 0 | 2 | 0 | 0 | 0 | 0 | D16366 | 98 | 245 | 1 | 1 | 244 |
| 2366 | 02729 | 02365 | 2 | | 0 | 0 | 0 | 0 | 0 | 0 | 1 | 0 | 0 | 0 | 0 | 0 | 0 | 0 | 1 | 0 | 0 | 0 | 0 | 0 | 0 | 0 | 0 | 0 | 0 | | | | | | |
| 2367 | 02730 | 02366 | 5 | | 0 | 0 | 0 | 0 | 0 | 0 | 2 | 0 | 0 | 0 | 0 | 0 | 0 | 0 | 0 | 0 | 0 | 0 | 0 | 0 | 0 | 2 | 1 | 0 | 0 | | | | | | |
| 2368 | 02731 | 02367 | 7 | | 0 | 0 | 0 | 0 | 0 | 0 | 1 | 0 | 0 | 0 | 1 | 1 | 0 | 0 | 2 | 1 | 0 | 0 | 0 | 0 | 0 | 0 | 1 | 0 | 0 | | | | | | |
| 2369 | 02732 | 02368 | 3 | | 0 | 0 | 0 | 0 | 0 | 0 | 2 | 0 | 0 | 0 | 1 | 0 | 0 | 0 | 0 | 0 | 0 | 0 | 0 | 0 | 0 | 0 | 0 | 0 | 0 | | | | | | |
| 2370 | 02733 | 02369 | 1 | | 0 | 0 | 0 | 0 | 0 | 0 | 1 | 0 | 0 | 0 | 0 | 0 | 0 | 0 | 0 | 0 | 0 | 0 | 0 | 0 | 0 | 0 | 0 | 0 | 0 | | | | | | |
| 2371 | 02734 | 02370 | 1 | | 0 | 0 | 0 | 0 | 0 | 0 | 1 | 0 | 0 | 0 | 0 | 0 | 0 | 0 | 0 | 0 | 0 | 0 | 0 | 0 | 0 | 0 | 0 | 0 | 0 | | | | | | |
| 2372 | 02735 | 02371 | 1 | | 0 | 0 | 0 | 0 | 0 | 0 | 1 | 0 | 0 | 0 | 0 | 0 | 0 | 0 | 0 | 0 | 0 | 0 | 0 | 0 | 0 | 0 | 0 | 0 | 0 | M26761 | 97 | 235 | 1 | 768 | 1023 |
| 2373 | 02736 | 02372 | 1 | | 0 | 0 | 0 | 0 | 0 | 0 | 1 | 0 | 0 | 0 | 0 | 0 | 0 | 0 | 0 | 0 | 0 | 0 | 0 | 0 | 0 | 0 | 0 | 0 | 0 | | | | | | |
| 2374 | 02737 | 02373 | 2 | | 0 | 0 | 0 | 0 | 0 | 0 | 1 | 0 | 1 | 0 | 0 | 0 | 0 | 0 | 0 | 0 | 0 | 0 | 0 | 0 | 0 | 0 | 0 | 0 | 0 | | | | | | |
| 2375 | 02738 | 02374 | 5 | | 0 | 0 | 0 | 0 | 0 | 0 | 2 | 1 | 0 | 0 | 0 | 0 | 0 | 0 | 1 | 0 | 0 | 0 | 0 | 0 | 1 | 0 | 0 | 0 | 0 | | | | | | |
| 2376 | 02739 | 02375 | 2 | | 0 | 0 | 0 | 0 | 0 | 0 | 1 | 0 | 0 | 0 | 0 | 0 | 0 | 0 | 0 | 0 | 0 | 1 | 0 | 0 | 0 | 0 | 0 | 0 | 0 | | | | | | |
| 2377 | 02740 | 02376 | 1 | | 0 | 0 | 0 | 0 | 0 | 0 | 1 | 0 | 0 | 0 | 0 | 0 | 0 | 0 | 0 | 0 | 0 | 0 | 0 | 0 | 0 | 0 | 0 | 0 | 0 | | | | | | |
| 2378 | 02741 | 02377 | 2 | | 0 | 0 | 0 | 0 | 0 | 0 | 1 | 0 | 0 | 0 | 0 | 0 | 0 | 0 | 0 | 0 | 0 | 0 | 0 | 1 | 0 | 0 | 0 | 0 | 0 | | | | | | |
| 2379 | 02742 | 02378 | 1 | | 0 | 0 | 0 | 0 | 0 | 0 | 1 | 0 | 0 | 0 | 0 | 0 | 0 | 0 | 0 | 0 | 0 | 0 | 0 | 0 | 0 | 0 | 0 | 0 | 0 | | | | | | |
| 2380 | 02743 | 02379 | 1 | | 0 | 0 | 0 | 0 | 0 | 0 | 1 | 0 | 0 | 0 | 0 | 0 | 0 | 0 | 0 | 0 | 0 | 0 | 0 | 0 | 0 | 0 | 0 | 0 | 0 | | | | | | |
| 2381 | 02744 | 02380 | 1 | | 0 | 0 | 0 | 0 | 0 | 0 | 1 | 0 | 0 | 0 | 0 | 0 | 0 | 0 | 0 | 0 | 0 | 0 | 0 | 0 | 0 | 0 | 0 | 0 | 0 | | | | | | |
| 2382 | 02745 | 02381 | 2 | | 0 | 0 | 0 | 0 | 0 | 0 | 1 | 0 | 0 | 0 | 0 | 0 | 0 | 0 | 0 | 1 | 0 | 0 | 0 | 0 | 0 | 0 | 0 | 0 | 0 | | | | | | |
| 2383 | 02746 | 02382 | 2 | | 0 | 0 | 0 | 0 | 0 | 0 | 1 | 0 | 0 | 0 | 0 | 0 | 0 | 1 | 0 | 0 | 0 | 0 | 0 | 0 | 0 | 0 | 0 | 0 | 0 | J03503 | 99.1 | 226 | 1 | 1221 | 1446 |
| 2384 | 02747 | 02383 | 2 | | 0 | 0 | 0 | 0 | 0 | 0 | 1 | 0 | 0 | 0 | 0 | 0 | 0 | 0 | 1 | 0 | 0 | 0 | 0 | 0 | 0 | 0 | 0 | 0 | 0 | | | | | | |
| 2385 | 02749 | 02384 | 3 | | 0 | 0 | 0 | 0 | 0 | 0 | 1 | 0 | 1 | 1 | 0 | 0 | 0 | 0 | 0 | 0 | 0 | 0 | 0 | 0 | 0 | 0 | 0 | 0 | 0 | | | | | | |
| 2386 | 02750 | 02385 | 1 | | 0 | 0 | 0 | 0 | 0 | 0 | 1 | 0 | 0 | 0 | 0 | 0 | 0 | 0 | 0 | 0 | 0 | 0 | 0 | 0 | 0 | 0 | 0 | 0 | 0 | | | | | | |
| 2387 | 02751 | 02386 | 1 | | 0 | 0 | 0 | 0 | 0 | 0 | 1 | 0 | 0 | 0 | 0 | 0 | 0 | 0 | 0 | 0 | 0 | 0 | 0 | 0 | 0 | 0 | 0 | 0 | 0 | | | | | | |
| 2388 | 02752 | 02387 | 1 | | 0 | 0 | 0 | 0 | 0 | 0 | 1 | 0 | 0 | 0 | 0 | 0 | 0 | 0 | 0 | 0 | 0 | 0 | 0 | 0 | 0 | 0 | 0 | 0 | 0 | | | | | | |
| 2389 | 02753 | 02388 | 2 | | 0 | 0 | 0 | 0 | 0 | 0 | 1 | 0 | 0 | 0 | 0 | 0 | 1 | 0 | 0 | 0 | 0 | 0 | 0 | 0 | 0 | 0 | 0 | 0 | 0 | | | | | | |
| 2390 | 02754 | 02389 | 1 | | 0 | 0 | 0 | 0 | 0 | 0 | 1 | 0 | 0 | 0 | 0 | 0 | 0 | 0 | 0 | 0 | 0 | 0 | 0 | 0 | 0 | 0 | 0 | 0 | 0 | | | | | | |
| 2391 | 02755 | 02390 | 1 | | 0 | 0 | 0 | 0 | 0 | 0 | 1 | 0 | 0 | 0 | 0 | 0 | 0 | 0 | 0 | 0 | 0 | 0 | 0 | 0 | 0 | 0 | 0 | 0 | 0 | | | | | | |
| 2392 | 02756 | 02391 | 4 | | 0 | 0 | 0 | 0 | 0 | 0 | 1 | 0 | 0 | 1 | 0 | 0 | 0 | 0 | 0 | 1 | 0 | 0 | 0 | 0 | 1 | 0 | 0 | 0 | 0 | X15977 | 99.5 | 216 | 1 | 392 | 608 |
| 2393 | 02758 | 02392 | 1 | | 0 | 0 | 0 | 0 | 0 | 0 | 1 | 0 | 0 | 0 | 0 | 0 | 0 | 0 | 0 | 0 | 0 | 0 | 0 | 0 | 0 | 0 | 0 | 0 | 0 | | | | | | |
| 2394 | 02760 | 02393 | 1 | | 0 | 0 | 0 | 0 | 0 | 0 | 1 | 0 | 0 | 0 | 0 | 0 | 0 | 0 | 0 | 0 | 0 | 0 | 0 | 0 | 0 | 0 | 0 | 0 | 0 | | | | | | |
| 2395 | 02761 | 02394 | 4 | | 0 | 0 | 0 | 0 | 0 | 0 | 1 | 1 | 0 | 0 | 1 | 0 | 0 | 0 | 1 | 0 | 0 | 0 | 0 | 0 | 0 | 0 | 0 | 0 | 0 | | | | | | |
| 2396 | 02762 | 02395 | 2 | | 0 | 0 | 0 | 0 | 0 | 0 | 2 | 0 | 0 | 0 | 0 | 0 | 0 | 0 | 0 | 0 | 0 | 0 | 0 | 0 | 0 | 0 | 0 | 0 | 0 | | | | | | |
| 2397 | 02764 | 02396 | 1 | | 0 | 0 | 0 | 0 | 0 | 0 | 1 | 0 | 0 | 0 | 0 | 0 | 0 | 0 | 0 | 0 | 0 | 0 | 0 | 0 | 0 | 0 | 0 | 0 | 0 | | | | | | |

Table 67

| | A | B | C | E | G | I | K | M | O | Q | S | U | W | Y | AA | AC | AE | AG | AI | AK | AM | AO | AQ | AS | AU | AW | AY | BA | BC | BE | BF | BG | BH | BI | BK |
|---|---|---|---|---|---|---|---|---|---|---|---|---|---|---|---|---|---|---|---|---|---|---|---|---|---|---|---|---|---|---|---|---|---|---|---|
| 2398 | 02765 | 02397 | 1 | | 0 | 0 | 0 | 0 | 0 | 0 | 1 | 0 | 0 | 0 | 0 | 0 | 0 | 0 | 0 | 0 | 0 | 0 | 0 | 0 | 0 | 0 | 0 | 0 | 0 | | | | | | |
| 2399 | 02767 | 02398 | 9 | | 0 | 0 | 0 | 0 | 0 | 0 | 1 | 0 | 1 | 0 | 0 | 1 | 1 | 0 | 1 | 1 | 0 | 0 | 0 | 0 | 1 | 2 | 0 | 0 | 0 | | | | | | |
| 2400 | 02768 | 02399 | 5 | | 0 | 0 | 0 | 0 | 0 | 0 | 1 | 0 | 0 | 0 | 0 | 0 | 0 | 0 | 0 | 1 | 1 | 0 | 0 | 1 | 0 | 1 | 0 | 0 | 0 | | | | | | |
| 2401 | 02769 | 02400 | 7 | | 0 | 0 | 0 | 0 | 0 | 0 | 1 | 2 | 0 | 0 | 0 | 0 | 0 | 0 | 0 | 0 | 0 | 3 | 0 | 0 | 1 | 0 | 0 | 0 | 0 | | | | | | |
| 2402 | 02770 | 02401 | 3 | | 0 | 0 | 0 | 0 | 0 | 0 | 1 | 0 | 0 | 0 | 0 | 0 | 1 | 0 | 0 | 0 | 0 | 0 | 0 | 0 | 1 | 0 | 0 | 0 | 0 | | | | | | |
| 2403 | 02771 | 02402 | 2 | | 0 | 0 | 0 | 0 | 0 | 0 | 1 | 0 | 0 | 0 | 1 | 0 | 0 | 0 | 0 | 0 | 0 | 0 | 0 | 0 | 0 | 0 | 0 | 0 | 0 | | | | | | |
| 2404 | 02772 | 02403 | 2 | | 0 | 0 | 0 | 0 | 0 | 0 | 1 | 0 | 0 | 0 | 0 | 0 | 0 | 0 | 0 | 0 | 0 | 0 | 0 | 1 | 0 | 0 | 0 | 0 | 0 | | | | | | |
| 2405 | 02773 | 02404 | 2 | | 0 | 0 | 0 | 0 | 0 | 0 | 1 | 0 | 0 | 0 | 0 | 0 | 0 | 0 | 0 | 0 | 0 | 0 | 0 | 1 | 0 | 0 | 0 | 0 | 0 | | | | | | |
| 2406 | 02774 | 02405 | 3 | | 0 | 0 | 0 | 0 | 0 | 0 | 2 | 0 | 0 | 0 | 0 | 0 | 0 | 0 | 0 | 0 | 0 | 0 | 0 | 0 | 0 | 0 | 0 | 1 | 0 | | | | | | |
| 2407 | 02775 | 02406 | 1 | | 0 | 0 | 0 | 0 | 0 | 0 | 1 | 0 | 0 | 0 | 0 | 0 | 0 | 0 | 0 | 0 | 0 | 0 | 0 | 0 | 0 | 0 | 0 | 0 | 0 | | | | | | |
| 2408 | 02776 | 02407 | 2 | | 0 | 0 | 0 | 0 | 0 | 0 | 1 | 0 | 0 | 0 | 0 | 0 | 1 | 0 | 0 | 0 | 0 | 0 | 0 | 0 | 0 | 0 | 0 | 0 | 0 | | | | | | |
| 2409 | 02777 | 02408 | 1 | | 0 | 0 | 0 | 0 | 0 | 0 | 1 | 0 | 0 | 0 | 0 | 0 | 0 | 0 | 0 | 0 | 0 | 0 | 0 | 0 | 0 | 0 | 0 | 0 | 0 | M90707 | 99.5 | 200 | 1 | 2077 | 2493 |
| 2410 | 02778 | 02409 | 1 | | 0 | 0 | 0 | 0 | 0 | 0 | 1 | 0 | 0 | 0 | 0 | 0 | 0 | 0 | 0 | 0 | 0 | 0 | 0 | 0 | 0 | 0 | 0 | 0 | 0 | | | | | | |
| 2411 | 02779 | 02410 | 1 | | 0 | 0 | 0 | 0 | 0 | 0 | 1 | 0 | 0 | 0 | 0 | 0 | 0 | 0 | 0 | 0 | 0 | 0 | 0 | 0 | 0 | 0 | 0 | 0 | 0 | | | | | | |
| 2412 | 02780 | 02411 | 1 | | 0 | 0 | 0 | 0 | 0 | 0 | 1 | 0 | 0 | 0 | 0 | 0 | 0 | 0 | 0 | 0 | 0 | 0 | 0 | 0 | 0 | 0 | 0 | 0 | 0 | | | | | | |
| 2413 | 02781 | 02412 | 1 | | 0 | 0 | 0 | 0 | 0 | 0 | 1 | 0 | 0 | 0 | 0 | 0 | 0 | 0 | 0 | 0 | 0 | 0 | 0 | 0 | 0 | 0 | 0 | 0 | 0 | | | | | | |
| 2414 | 02782 | 02413 | 3 | | 0 | 0 | 0 | 0 | 0 | 0 | 1 | 0 | 0 | 0 | 0 | 1 | 0 | 0 | 1 | 0 | 0 | 0 | 0 | 0 | 0 | 0 | 0 | 0 | 0 | | | | | | |
| 2415 | 02783 | 02414 | 5 | | 0 | 0 | 0 | 0 | 0 | 0 | 1 | 1 | 0 | 1 | 0 | 1 | 0 | 0 | 0 | 0 | 0 | 0 | 0 | 0 | 0 | 1 | 0 | 0 | 0 | | | | | | |
| 2416 | 02785 | 02415 | 2 | | 0 | 0 | 0 | 0 | 0 | 0 | 2 | 0 | 0 | 0 | 0 | 0 | 0 | 0 | 0 | 0 | 0 | 0 | 0 | 0 | 0 | 0 | 0 | 0 | 0 | M91211 | 96.9 | 163 | 1 | 1230 | 1391 |
| 2417 | 02786 | 02416 | 2 | | 0 | 0 | 0 | 0 | 0 | 0 | 1 | 0 | 0 | 0 | 0 | 0 | 0 | 0 | 0 | 0 | 0 | 0 | 0 | 0 | 0 | 1 | 0 | 0 | 0 | | | | | | |
| 2418 | 02787 | 02417 | 1 | | 0 | 0 | 0 | 0 | 0 | 0 | 1 | 0 | 0 | 0 | 0 | 0 | 0 | 0 | 0 | 0 | 0 | 0 | 0 | 0 | 0 | 0 | 0 | 0 | 0 | | | | | | |
| 2419 | 02788 | 02418 | 1 | | 0 | 0 | 0 | 0 | 0 | 0 | 1 | 0 | 0 | 0 | 0 | 0 | 0 | 0 | 0 | 0 | 0 | 0 | 0 | 0 | 0 | 0 | 0 | 0 | 0 | | | | | | |
| 2420 | 02789 | 02419 | 3 | | 0 | 0 | 0 | 0 | 0 | 0 | 1 | 2 | 0 | 0 | 0 | 0 | 0 | 0 | 0 | 0 | 0 | 0 | 0 | 0 | 0 | 0 | 0 | 0 | 0 | M86667 | 99 | 192 | 1 | 1219 | 1560 |
| 2421 | 02790 | 02420 | 1 | | 0 | 0 | 0 | 0 | 0 | 0 | 1 | 0 | 0 | 0 | 0 | 0 | 0 | 0 | 0 | 0 | 0 | 0 | 0 | 0 | 0 | 0 | 0 | 0 | 0 | M83738 | 97.9 | 192 | 1 | 3744 | 3956 |
| 2422 | 02791 | 02421 | 3 | | 0 | 0 | 0 | 0 | 0 | 0 | 1 | 0 | 0 | 0 | 0 | 0 | 0 | 0 | 0 | 0 | 0 | 0 | 1 | 1 | 0 | 0 | 0 | 0 | 0 | | | | | | |
| 2423 | 02792 | 02422 | 4 | | 0 | 0 | 0 | 0 | 0 | 0 | 1 | 0 | 0 | 0 | 0 | 0 | 0 | 0 | 0 | 0 | 0 | 0 | 0 | 1 | 0 | 0 | 0 | 2 | 0 | | | | | | |
| 2424 | 02793 | 02423 | 9 | | 0 | 0 | 0 | 0 | 0 | 0 | 1 | 0 | 0 | 0 | 0 | 0 | 0 | 0 | 2 | 2 | 0 | 0 | 0 | 1 | 0 | 0 | 0 | 3 | 0 | | | | | | |
| 2425 | 02794 | 02424 | 1 | | 0 | 0 | 0 | 0 | 0 | 0 | 1 | 0 | 0 | 0 | 0 | 0 | 0 | 0 | 0 | 0 | 0 | 0 | 0 | 0 | 0 | 0 | 0 | 0 | 0 | | | | | | |
| 2426 | 02795 | 02425 | 7 | | 0 | 0 | 0 | 0 | 0 | 0 | 1 | 0 | 0 | 0 | 0 | 0 | 0 | 0 | 0 | 0 | 1 | 1 | 3 | 0 | 0 | 1 | 0 | 0 | 0 | | | | | | |
| 2427 | 02796 | 02426 | 5 | | 0 | 0 | 0 | 0 | 0 | 0 | 1 | 1 | 0 | 0 | 0 | 0 | 0 | 0 | 0 | 0 | 0 | 0 | 0 | 2 | 0 | 0 | 1 | 0 | 0 | | | | | | |
| 2428 | 02797 | 02427 | 3 | | 0 | 0 | 0 | 0 | 0 | 0 | 2 | 0 | 0 | 1 | 0 | 0 | 0 | 0 | 0 | 0 | 0 | 0 | 0 | 0 | 0 | 0 | 0 | 0 | 0 | | | | | | |
| 2429 | 02798 | 02428 | 1 | | 0 | 0 | 0 | 0 | 0 | 0 | 1 | 0 | 0 | 0 | 0 | 0 | 0 | 0 | 0 | 0 | 0 | 0 | 0 | 0 | 0 | 0 | 0 | 0 | 0 | | | | | | |
| 2430 | 02799 | 02429 | 3 | | 0 | 0 | 0 | 0 | 0 | 0 | 1 | 0 | 0 | 0 | 1 | 0 | 0 | 0 | 0 | 0 | 0 | 0 | 0 | 0 | 0 | 1 | 0 | 0 | 0 | | | | | | |
| 2431 | 02800 | 02430 | 1 | | 0 | 0 | 0 | 0 | 0 | 0 | 1 | 0 | 0 | 0 | 0 | 0 | 0 | 0 | 0 | 0 | 0 | 0 | 0 | 0 | 0 | 0 | 0 | 0 | 0 | | | | | | |
| 2432 | 02801 | 02431 | 9 | | 0 | 0 | 0 | 0 | 0 | 0 | 2 | 0 | 0 | 0 | 3 | 0 | 0 | 0 | 0 | 1 | 0 | 0 | 0 | 0 | 0 | 3 | 0 | 0 | 0 | | | | | | |
| 2433 | 02802 | 02432 | 1 | | 0 | 0 | 0 | 0 | 0 | 0 | 1 | 0 | 0 | 0 | 0 | 0 | 0 | 0 | 0 | 0 | 0 | 0 | 0 | 0 | 0 | 0 | 0 | 0 | 0 | X65018 | 98.2 | 171 | 1 | 1230 | 1410 |

Table 68

| | A | B | C | E | G | I | K | M | O | Q | S | U | W | Y | AA | AC | AE | AG | AI | AK | AM | AO | AQ | AS | AU | AW | AY | BA | BC | BE | BF | BG | BH | BI | BK |
|---|---|---|---|---|---|---|---|---|---|---|---|---|---|---|---|---|---|---|---|---|---|---|---|---|---|---|---|---|---|---|---|---|---|---|---|
| 2434 | 02803 | 02433 | 2 | | 0 | 0 | 0 | 0 | 0 | 0 | 1 | 0 | 0 | 0 | 0 | 0 | 0 | 0 | 0 | 0 | 0 | 0 | 0 | 0 | 0 | 0 | 1 | 0 | 0 | | | | | | |
| 2435 | 02804 | 02434 | 5 | | 0 | 0 | 0 | 0 | 0 | 0 | 2 | 1 | 0 | 0 | 0 | 0 | 0 | 0 | 0 | 0 | 0 | 0 | 0 | 0 | 0 | 0 | 2 | 0 | 0 | | | | | | |
| 2436 | 02805 | 02435 | 4 | | 0 | 0 | 0 | 0 | 0 | 0 | 1 | 0 | 0 | 1 | 0 | 0 | 0 | 0 | 0 | 0 | 1 | 0 | 0 | 0 | 0 | 0 | 0 | 1 | 0 | | | | | | |
| 2437 | 02806 | 02436 | 1 | | 0 | 0 | 0 | 0 | 0 | 0 | 1 | 0 | 0 | 0 | 0 | 0 | 0 | 0 | 0 | 0 | 0 | 0 | 0 | 0 | 0 | 0 | 0 | 0 | 0 | | | | | | |
| 2438 | 02807 | 02437 | 2 | | 0 | 0 | 0 | 0 | 0 | 0 | 1 | 0 | 0 | 0 | 0 | 0 | 0 | 0 | 0 | 0 | 0 | 0 | 0 | 1 | 0 | 0 | 0 | 0 | 0 | | | | | | |
| 2439 | 02808 | 02438 | 1 | | 0 | 0 | 0 | 0 | 0 | 0 | 1 | 0 | 0 | 0 | 0 | 0 | 0 | 0 | 0 | 0 | 0 | 0 | 0 | 0 | 0 | 0 | 0 | 0 | 0 | X70991 | 95.4 | 174 | 1 | 2015 | 2192 |
| 2440 | 02809 | 02439 | 1 | | 0 | 0 | 0 | 0 | 0 | 0 | 1 | 0 | 0 | 0 | 0 | 0 | 0 | 0 | 0 | 0 | 0 | 0 | 0 | 0 | 0 | 0 | 0 | 0 | 0 | | | | | | |
| 2441 | 02810 | 02440 | 2 | | 0 | 0 | 0 | 0 | 0 | 0 | 1 | 0 | 1 | 0 | 0 | 0 | 0 | 0 | 0 | 0 | 0 | 0 | 0 | 0 | 0 | 0 | 0 | 0 | 0 | | | | | | |
| 2442 | 02811 | 02441 | 4 | | 0 | 0 | 0 | 0 | 0 | 0 | 1 | 0 | 1 | 0 | 0 | 0 | 0 | 0 | 0 | 0 | 0 | 0 | 0 | 0 | 1 | 1 | 0 | 0 | 0 | D14665 | 100 | 165 | 1 | 3377 | 3541 |
| 2443 | 02812 | 02442 | 12 | | 0 | 0 | 0 | 0 | 0 | 0 | 1 | 3 | 0 | 0 | 0 | 0 | 2 | 0 | 1 | 1 | 0 | 0 | 2 | 2 | 0 | 0 | 0 | 0 | 0 | | | | | | |
| 2444 | 02813 | 02443 | 5 | | 0 | 0 | 0 | 0 | 0 | 0 | 1 | 0 | 0 | 1 | 0 | 0 | 2 | 0 | 0 | 0 | 0 | 0 | 0 | 0 | 0 | 0 | 1 | 0 | 0 | | | | | | |
| 2445 | 02814 | 02444 | 15 | | 0 | 0 | 0 | 0 | 0 | 0 | 1 | 0 | 0 | 1 | 0 | 0 | 1 | 0 | 0 | 0 | 0 | 3 | 2 | 1 | 0 | 1 | 1 | 2 | 2 | | | | | | |
| 2446 | 02815 | 02445 | 4 | | 0 | 0 | 0 | 0 | 0 | 0 | 1 | 0 | 0 | 0 | 1 | 1 | 0 | 0 | 0 | 1 | 0 | 0 | 0 | 0 | 0 | 0 | 0 | 0 | 0 | | | | | | |
| 2447 | 02816 | 02446 | 2 | | 0 | 0 | 0 | 0 | 0 | 0 | 2 | 0 | 0 | 0 | 0 | 0 | 0 | 0 | 0 | 0 | 0 | 0 | 0 | 0 | 0 | 0 | 0 | 0 | 0 | | | | | | |
| 2448 | 02817 | 02447 | 2 | | 0 | 0 | 0 | 0 | 0 | 0 | 1 | 0 | 0 | 0 | 0 | 0 | 0 | 0 | 0 | 0 | 0 | 0 | 0 | 1 | 0 | 0 | 0 | 0 | 0 | | | | | | |
| 2449 | 02818 | 02448 | 5 | | 0 | 0 | 0 | 0 | 0 | 0 | 2 | 1 | 0 | 0 | 0 | 0 | 0 | 0 | 0 | 0 | 0 | 0 | 0 | 0 | 0 | 0 | 0 | 0 | 2 | | | | | | |
| 2450 | 02819 | 02449 | 1 | | 0 | 0 | 0 | 0 | 0 | 0 | 1 | 0 | 0 | 0 | 0 | 0 | 0 | 0 | 0 | 0 | 0 | 0 | 0 | 0 | 0 | 0 | 0 | 0 | 0 | | | | | | |
| 2451 | 02820 | 02450 | 29 | | 0 | 0 | 0 | 0 | 0 | 0 | 1 | 1 | 0 | 15 | 0 | 0 | 0 | 3 | 2 | 0 | 1 | 1 | 1 | 0 | 3 | 0 | 1 | 0 | 0 | X02490 | 91.9 | 135 | 14 | 435 | 568 |
| 2452 | 02821 | 02451 | 22 | | 0 | 0 | 0 | 0 | 0 | 0 | 2 | 0 | 0 | 0 | 2 | 0 | 0 | 0 | 0 | 0 | 0 | 0 | 0 | 0 | 1 | 1 | 16 | 0 | 0 | X68277 | 93.4 | 409 | 1 | 1601 | 2000 |
| 2453 | 02822 | 02452 | 1 | | 0 | 0 | 0 | 0 | 0 | 0 | 1 | 0 | 0 | 0 | 0 | 0 | 0 | 0 | 0 | 0 | 0 | 0 | 0 | 0 | 0 | 0 | 0 | 0 | 0 | | | | | | |
| 2454 | 02823 | 02453 | 5 | | 0 | 0 | 0 | 0 | 0 | 0 | 1 | 0 | 0 | 0 | 0 | 0 | 0 | 1 | 0 | 0 | 0 | 0 | 1 | 1 | 1 | 0 | 0 | 0 | 0 | | | | | | |
| 2455 | 02824 | 02454 | 2 | | 0 | 0 | 0 | 0 | 0 | 0 | 1 | 0 | 0 | 0 | 0 | 0 | 0 | 0 | 0 | 0 | 0 | 0 | 0 | 0 | 1 | 0 | 0 | 0 | 0 | | | | | | |
| 2456 | 02825 | 02455 | 1 | | 0 | 0 | 0 | 0 | 0 | 0 | 1 | 0 | 0 | 0 | 0 | 0 | 0 | 0 | 0 | 0 | 0 | 0 | 0 | 0 | 0 | 0 | 0 | 0 | 0 | | | | | | |
| 2457 | 02826 | 02456 | 1 | | 0 | 0 | 0 | 0 | 0 | 0 | 1 | 0 | 0 | 0 | 0 | 0 | 0 | 0 | 0 | 0 | 0 | 0 | 0 | 0 | 0 | 0 | 0 | 0 | 0 | | | | | | |
| 2458 | 02827 | 02457 | 1 | | 0 | 0 | 0 | 0 | 0 | 0 | 1 | 0 | 0 | 0 | 0 | 0 | 0 | 0 | 0 | 0 | 0 | 0 | 0 | 0 | 0 | 0 | 0 | 0 | 0 | | | | | | |
| 2459 | 02828 | 02458 | 1 | | 0 | 0 | 0 | 0 | 0 | 0 | 1 | 0 | 0 | 0 | 0 | 0 | 0 | 0 | 0 | 0 | 0 | 0 | 0 | 0 | 0 | 0 | 0 | 0 | 0 | D25539 | 98.7 | 158 | 1 | 4303 | 4460 |
| 2460 | 02829 | 02459 | 1 | | 0 | 0 | 0 | 0 | 0 | 0 | 1 | 0 | 0 | 0 | 0 | 0 | 0 | 0 | 0 | 0 | 0 | 0 | 0 | 0 | 0 | 0 | 0 | 0 | 0 | | | | | | |
| 2461 | 02830 | 02460 | 1 | | 0 | 0 | 0 | 0 | 0 | 0 | 1 | 0 | 0 | 0 | 0 | 0 | 0 | 0 | 0 | 0 | 0 | 0 | 0 | 0 | 0 | 0 | 0 | 0 | 0 | | | | | | |
| 2462 | 02831 | 02461 | 3 | | 0 | 0 | 0 | 0 | 0 | 0 | 1 | 0 | 0 | 0 | 0 | 0 | 0 | 0 | 1 | 0 | 0 | 0 | 0 | 1 | 0 | 0 | 0 | 0 | 0 | | | | | | |
| 2463 | 02832 | 02462 | 2 | | 0 | 0 | 0 | 0 | 0 | 0 | 1 | 0 | 0 | 0 | 0 | 0 | 0 | 0 | 0 | 0 | 0 | 0 | 0 | 0 | 0 | 0 | 0 | 1 | 0 | | | | | | |
| 2464 | 02833 | 02463 | 7 | | 0 | 0 | 0 | 0 | 0 | 0 | 1 | 1 | 0 | 1 | 0 | 0 | 0 | 0 | 1 | 0 | 1 | 1 | 0 | 0 | 0 | 0 | 1 | 0 | 0 | M31013 | 90.4 | 407 | 1 | 4603 | 5122 |
| 2465 | 02834 | 02464 | 1 | | 0 | 0 | 0 | 0 | 0 | 0 | 1 | 0 | 0 | 0 | 0 | 0 | 0 | 0 | 0 | 0 | 0 | 0 | 0 | 0 | 0 | 0 | 0 | 0 | 0 | | | | | | |
| 2466 | 02835 | 02465 | 4 | | 0 | 0 | 0 | 0 | 0 | 0 | 1 | 0 | 0 | 0 | 0 | 0 | 0 | 0 | 0 | 0 | 1 | 0 | 0 | 0 | 0 | 0 | 1 | 1 | 0 | | | | | | |
| 2467 | 02836 | 02466 | 3 | | 0 | 0 | 0 | 0 | 0 | 0 | 3 | 0 | 0 | 0 | 0 | 0 | 0 | 0 | 0 | 0 | 0 | 0 | 0 | 0 | 0 | 0 | 0 | 0 | 0 | M92449 | 90.7 | 150 | 1 | 1084 | 1233 |
| 2468 | 02837 | 02467 | 1 | | 0 | 0 | 0 | 0 | 0 | 0 | 1 | 0 | 0 | 0 | 0 | 0 | 0 | 0 | 0 | 0 | 0 | 0 | 0 | 0 | 0 | 0 | 0 | 0 | 0 | | | | | | |
| 2469 | 02838 | 02468 | 1 | | 0 | 0 | 0 | 0 | 0 | 0 | 1 | 0 | 0 | 0 | 0 | 0 | 0 | 0 | 0 | 0 | 0 | 0 | 0 | 0 | 0 | 0 | 0 | 0 | 0 | | | | | | |

Table 69

| | A | B | C | E | G | I | K | M | O | Q | S | U | W | Y | AA | AC | AE | AG | AI | AK | AM | AO | AQ | AS | AU | AW | AY | BA | BC | BE | BF | BG | BH | BI | BK |
|---|---|---|---|---|---|---|---|---|---|---|---|---|---|---|---|---|---|---|---|---|---|---|---|---|---|---|---|---|---|---|---|---|---|---|---|
| 2470 | 02839 | 02469 | 2 | | 0 | 0 | 0 | 0 | 0 | 0 | 1 | 0 | 0 | 0 | 0 | 0 | 0 | 0 | 0 | 0 | 0 | 0 | 0 | 0 | 0 | 1 | 0 | 0 | 0 | | | | | | |
| 2471 | 02840 | 02470 | 3 | | 0 | 0 | 0 | 0 | 0 | 0 | 1 | 0 | 0 | 0 | 0 | 0 | 1 | 0 | 0 | 0 | 0 | 0 | 0 | 1 | 0 | 0 | 0 | 0 | 0 | | | | | | |
| 2472 | 02843 | 02471 | 2 | | 0 | 0 | 0 | 0 | 0 | 0 | 1 | 0 | 0 | 0 | 0 | 0 | 0 | 1 | 0 | 0 | 0 | 0 | 0 | 0 | 0 | 0 | 0 | 0 | 0 | | | | | | |
| 2473 | 02844 | 02472 | 1 | | 0 | 0 | 0 | 0 | 0 | 0 | 1 | 0 | 0 | 0 | 0 | 0 | 0 | 0 | 0 | 0 | 0 | 0 | 0 | 0 | 0 | 0 | 0 | 0 | 0 | L24783 | 98.6 | 148 | 1 | 495 | 649 |
| 2474 | 02845 | 02473 | 1 | | 0 | 0 | 0 | 0 | 0 | 0 | 1 | 0 | 0 | 0 | 0 | 0 | 0 | 0 | 0 | 0 | 0 | 0 | 0 | 0 | 0 | 0 | 0 | 0 | 0 | | | | | | |
| 2475 | 02846 | 02474 | 1 | | 0 | 0 | 0 | 0 | 0 | 0 | 1 | 0 | 0 | 0 | 0 | 0 | 0 | 0 | 0 | 0 | 0 | 0 | 0 | 0 | 0 | 0 | 0 | 0 | 0 | | | | | | |
| 2476 | 02847 | 02475 | 11 | | 0 | 0 | 0 | 0 | 0 | 0 | 1 | 0 | 0 | 1 | 0 | 0 | 2 | 0 | 0 | 0 | 1 | 0 | 0 | 1 | 3 | 0 | 0 | 2 | 0 | D90209 | 100 | 145 | 1 | 1871 | 2015 |
| 2477 | 02848 | 02476 | 2 | | 0 | 0 | 0 | 0 | 0 | 0 | 2 | 0 | 0 | 0 | 0 | 0 | 0 | 0 | 0 | 0 | 0 | 0 | 0 | 0 | 0 | 0 | 0 | 0 | 0 | | | | | | |
| 2478 | 02849 | 02477 | 2 | | 0 | 0 | 0 | 0 | 0 | 0 | 1 | 0 | 0 | 0 | 0 | 0 | 0 | 0 | 0 | 0 | 0 | 1 | 0 | 0 | 0 | 0 | 0 | 0 | 0 | | | | | | |
| 2479 | 02850 | 02478 | 1 | | 0 | 0 | 0 | 0 | 0 | 0 | 1 | 0 | 0 | 0 | 0 | 0 | 0 | 0 | 0 | 0 | 0 | 0 | 0 | 0 | 0 | 0 | 0 | 0 | 0 | | | | | | |
| 2480 | 02851 | 02479 | 9 | | 0 | 0 | 0 | 0 | 0 | 0 | 1 | 0 | 0 | 0 | 0 | 0 | 1 | 2 | 1 | 0 | 0 | 0 | 2 | 2 | 0 | 0 | 0 | 0 | 0 | | | | | | |
| 2481 | 02852 | 02480 | 1 | | 0 | 0 | 0 | 0 | 0 | 0 | 1 | 0 | 0 | 0 | 0 | 0 | 0 | 0 | 0 | 0 | 0 | 0 | 0 | 0 | 0 | 0 | 0 | 0 | 0 | | | | | | |
| 2482 | 02853 | 02481 | 8 | | 0 | 0 | 0 | 0 | 0 | 0 | 2 | 0 | 1 | 0 | 0 | 0 | 0 | 0 | 1 | 0 | 1 | 3 | 0 | 0 | 0 | 0 | 0 | 0 | 0 | M18366 | 98.3 | 287 | 1 | 1158 | 1444 |
| 2483 | 02854 | 02482 | 4 | | 0 | 0 | 0 | 0 | 0 | 0 | 2 | 0 | 1 | 0 | 0 | 0 | 0 | 0 | 0 | 0 | 0 | 0 | 0 | 0 | 0 | 1 | 0 | 0 | 0 | | | | | | |
| 2484 | 02855 | 02483 | 2 | | 0 | 0 | 0 | 0 | 0 | 0 | 1 | 0 | 0 | 0 | 0 | 0 | 0 | 0 | 0 | 0 | 0 | 0 | 0 | 1 | 0 | 0 | 0 | 0 | 0 | J00271 | 93.9 | 264 | 5 | 1362 | 1703 |
| 2485 | 02856 | 02484 | 1 | | 0 | 0 | 0 | 0 | 0 | 0 | 1 | 0 | 0 | 0 | 0 | 0 | 0 | 0 | 0 | 0 | 0 | 0 | 0 | 0 | 0 | 0 | 0 | 0 | 0 | | | | | | |
| 2486 | 02857 | 02485 | 3 | | 0 | 0 | 0 | 0 | 0 | 0 | 3 | 0 | 0 | 0 | 0 | 0 | 0 | 0 | 0 | 0 | 0 | 0 | 0 | 0 | 0 | 0 | 0 | 0 | 0 | | | | | | |
| 2487 | 02858 | 02486 | 1 | | 0 | 0 | 0 | 0 | 0 | 0 | 1 | 0 | 0 | 0 | 0 | 0 | 0 | 0 | 0 | 0 | 0 | 0 | 0 | 0 | 0 | 0 | 0 | 0 | 0 | | | | | | |
| 2488 | 02859 | 02487 | 5 | | 0 | 0 | 0 | 0 | 0 | 0 | 1 | 0 | 0 | 2 | 0 | 0 | 0 | 0 | 0 | 0 | 0 | 2 | 0 | 0 | 0 | 0 | 0 | 0 | 0 | | | | | | |
| 2489 | 02860 | 02488 | 4 | | 0 | 0 | 0 | 0 | 0 | 0 | 1 | 0 | 0 | 0 | 0 | 0 | 0 | 0 | 0 | 0 | 0 | 0 | 2 | 1 | 0 | 0 | 0 | 0 | 0 | | | | | | |
| 2490 | 02861 | 02489 | 10 | | 0 | 0 | 0 | 0 | 0 | 0 | 1 | 0 | 0 | 1 | 0 | 1 | 1 | 3 | 0 | 1 | 0 | 0 | 1 | 0 | 0 | 0 | 1 | 0 | 0 | | | | | | |
| 2491 | 02862 | 02490 | 7 | | 0 | 0 | 0 | 0 | 0 | 0 | 1 | 0 | 0 | 0 | 0 | 0 | 1 | 0 | 0 | 1 | 0 | 0 | 2 | 0 | 0 | 1 | 0 | 1 | 0 | | | | | | |
| 2492 | 02863 | 02491 | 3 | | 0 | 0 | 0 | 0 | 0 | 0 | 1 | 0 | 0 | 0 | 0 | 0 | 0 | 1 | 0 | 0 | 0 | 0 | 1 | 0 | 0 | 0 | 0 | 0 | 0 | | | | | | |
| 2493 | 02864 | 02492 | 1 | | 0 | 0 | 0 | 0 | 0 | 0 | 1 | 0 | 0 | 0 | 0 | 0 | 0 | 0 | 0 | 0 | 0 | 0 | 0 | 0 | 0 | 0 | 0 | 0 | 0 | | | | | | |
| 2494 | 02865 | 02493 | 2 | | 0 | 0 | 0 | 0 | 0 | 0 | 1 | 0 | 0 | 0 | 0 | 0 | 0 | 0 | 1 | 0 | 0 | 0 | 0 | 0 | 0 | 0 | 0 | 0 | 0 | X57347 | 94.8 | 134 | 1 | 509 | 1874 |
| 2495 | 02866 | 02494 | 1 | | 0 | 0 | 0 | 0 | 0 | 0 | 1 | 0 | 0 | 0 | 0 | 0 | 0 | 0 | 0 | 0 | 0 | 0 | 0 | 0 | 0 | 0 | 0 | 0 | 0 | | | | | | |
| 2496 | 02867 | 02495 | 1 | | 0 | 0 | 0 | 0 | 0 | 0 | 1 | 0 | 0 | 0 | 0 | 0 | 0 | 0 | 0 | 0 | 0 | 0 | 0 | 0 | 0 | 0 | 0 | 0 | 0 | | | | | | |
| 2497 | 02868 | 02496 | 1 | | 0 | 0 | 0 | 0 | 0 | 0 | 1 | 0 | 0 | 0 | 0 | 0 | 0 | 0 | 0 | 0 | 0 | 0 | 0 | 0 | 0 | 0 | 0 | 0 | 0 | | | | | | |
| 2498 | 02869 | 02497 | 1 | | 0 | 0 | 0 | 0 | 0 | 0 | 1 | 0 | 0 | 0 | 0 | 0 | 0 | 0 | 0 | 0 | 0 | 0 | 0 | 0 | 0 | 0 | 0 | 0 | 0 | | | | | | |
| 2499 | 02870 | 02498 | 1 | | 0 | 0 | 0 | 0 | 0 | 0 | 1 | 0 | 0 | 0 | 0 | 0 | 0 | 0 | 0 | 0 | 0 | 0 | 0 | 0 | 0 | 0 | 0 | 0 | 0 | | | | | | |
| 2500 | 02872 | 02499 | 4 | | 0 | 0 | 0 | 0 | 0 | 0 | 1 | 1 | 0 | 0 | 0 | 1 | 0 | 0 | 0 | 0 | 0 | 0 | 0 | 0 | 0 | 0 | 0 | 1 | 0 | | | | | | |
| 2501 | 02873 | 02500 | 5 | | 0 | 0 | 0 | 0 | 0 | 0 | 1 | 0 | 1 | 0 | 0 | 0 | 0 | 0 | 0 | 0 | 1 | 0 | 1 | 1 | 0 | 0 | 0 | 0 | 0 | | | | | | |
| 2502 | 02874 | 02501 | 6 | | 0 | 0 | 0 | 0 | 0 | 0 | 2 | 0 | 0 | 0 | 0 | 1 | 0 | 0 | 0 | 0 | 0 | 1 | 0 | 0 | 0 | 0 | 2 | 0 | 0 | | | | | | |
| 2503 | 02875 | 02502 | 2 | | 0 | 0 | 0 | 0 | 0 | 0 | 1 | 0 | 0 | 0 | 0 | 0 | 0 | 0 | 0 | 0 | 0 | 0 | 0 | 1 | 0 | 0 | 0 | 0 | 0 | | | | | | |
| 2504 | 02876 | 02503 | 1 | | 0 | 0 | 0 | 0 | 0 | 0 | 1 | 0 | 0 | 0 | 0 | 0 | 0 | 0 | 0 | 0 | 0 | 0 | 0 | 0 | 0 | 0 | 0 | 0 | 0 | | | | | | |
| 2505 | 02877 | 02504 | 1 | | 0 | 0 | 0 | 0 | 0 | 0 | 1 | 0 | 0 | 0 | 0 | 0 | 0 | 0 | 0 | 0 | 0 | 0 | 0 | 0 | 0 | 0 | 0 | 0 | 0 | | | | | | |

Table 70

| | A | B | C | E | G | I | K | M | O | Q | S | U | W | Y | AA | AC | AE | AG | AI | AK | AM | AO | AQ | AS | AU | AW | AY | BA | BC | BE | BF | BG | BH | BI | BK |
|---|---|---|---|---|---|---|---|---|---|---|---|---|---|---|---|---|---|---|---|---|---|---|---|---|---|---|---|---|---|---|---|---|---|---|---|
| 2506 | 02878 | 02505 | 1 | | 0 | 0 | 0 | 0 | 0 | 0 | 1 | 0 | 0 | 0 | 0 | 0 | 0 | 0 | 0 | 0 | 0 | 0 | 0 | 0 | 0 | 0 | 0 | 0 | 0 | | | | | | |
| 2507 | 02879 | 02506 | 1 | | 0 | 0 | 0 | 0 | 0 | 0 | 1 | 0 | 0 | 0 | 0 | 0 | 0 | 0 | 0 | 0 | 0 | 0 | 0 | 0 | 0 | 0 | 0 | 0 | 0 | | | | | | |
| 2508 | 02880 | 02507 | 1 | | 0 | 0 | 0 | 0 | 0 | 0 | 1 | 0 | 0 | 0 | 0 | 0 | 0 | 0 | 0 | 0 | 0 | 0 | 0 | 0 | 0 | 0 | 0 | 0 | 0 | | | | | | |
| 2509 | 02881 | 02508 | 3 | | 0 | 0 | 0 | 0 | 0 | 0 | 1 | 0 | 0 | 0 | 0 | 0 | 0 | 0 | 0 | 0 | 0 | 1 | 0 | 0 | 1 | 0 | 0 | 0 | 0 | | | | | | |
| 2510 | 02882 | 02509 | 2 | | 0 | 0 | 0 | 0 | 0 | 0 | 1 | 0 | 0 | 0 | 0 | 0 | 0 | 0 | 0 | 0 | 0 | 0 | 0 | 0 | 1 | 0 | 0 | 0 | 0 | | | | | | |
| 2511 | 02883 | 02510 | 2 | | 0 | 0 | 0 | 0 | 0 | 0 | 1 | 0 | 0 | 0 | 0 | 0 | 0 | 0 | 0 | 1 | 0 | 0 | 0 | 0 | 0 | 0 | 0 | 0 | 0 | | | | | | |
| 2512 | 02884 | 02511 | 1 | | 0 | 0 | 0 | 0 | 0 | 0 | 1 | 0 | 0 | 0 | 0 | 0 | 0 | 0 | 0 | 0 | 0 | 0 | 0 | 0 | 0 | 0 | 0 | 0 | 0 | | | | | | |
| 2513 | 02885 | 02512 | 1 | | 0 | 0 | 0 | 0 | 0 | 0 | 1 | 0 | 0 | 0 | 0 | 0 | 0 | 0 | 0 | 0 | 0 | 0 | 0 | 0 | 0 | 0 | 0 | 0 | 0 | | | | | | |
| 2514 | 02886 | 02513 | 2 | | 0 | 0 | 0 | 0 | 0 | 0 | 1 | 0 | 0 | 0 | 0 | 0 | 0 | 0 | 0 | 0 | 0 | 0 | 0 | 0 | 0 | 0 | 0 | 1 | 0 | | | | | | |
| 2515 | 02887 | 02514 | 1 | | 0 | 0 | 0 | 0 | 0 | 0 | 1 | 0 | 0 | 0 | 0 | 0 | 0 | 0 | 0 | 0 | 0 | 0 | 0 | 0 | 0 | 0 | 0 | 0 | 0 | | | | | | |
| 2516 | 02888 | 02515 | 1 | | 0 | 0 | 0 | 0 | 0 | 0 | 1 | 0 | 0 | 0 | 0 | 0 | 0 | 0 | 0 | 0 | 0 | 0 | 0 | 0 | 0 | 0 | 0 | 0 | 0 | | | | | | |
| 2517 | 02889 | 02516 | 2 | | 0 | 0 | 0 | 0 | 0 | 0 | 1 | 0 | 0 | 0 | 0 | 0 | 0 | 0 | 0 | 0 | 0 | 0 | 0 | 0 | 0 | 1 | 0 | 0 | 0 | M95724 | 97.5 | 119 | 1 | 2991 | 3132 |
| 2518 | 02890 | 02517 | 6 | | 0 | 0 | 0 | 0 | 0 | 0 | 3 | 0 | 0 | 1 | 0 | 0 | 1 | 0 | 0 | 0 | 0 | 1 | 0 | 0 | 0 | 0 | 0 | 0 | 0 | | | | | | |
| 2519 | 02891 | 02518 | 1 | | 0 | 0 | 0 | 0 | 0 | 0 | 1 | 0 | 0 | 0 | 0 | 0 | 0 | 0 | 0 | 0 | 0 | 0 | 0 | 0 | 0 | 0 | 0 | 0 | 0 | | | | | | |
| 2520 | 02893 | 02519 | 1 | | 0 | 0 | 0 | 0 | 0 | 0 | 1 | 0 | 0 | 0 | 0 | 0 | 0 | 0 | 0 | 0 | 0 | 0 | 0 | 0 | 0 | 0 | 0 | 0 | 0 | | | | | | |
| 2521 | 02894 | 02520 | 89 | | 0 | 0 | 0 | 0 | 0 | 0 | 87 | 0 | 0 | 1 | 0 | 1 | 0 | 0 | 0 | 0 | 0 | 0 | 0 | 0 | 0 | 0 | 0 | 0 | 0 | | | | | | |
| 2522 | 02895 | 02521 | 1 | | 0 | 0 | 0 | 0 | 0 | 0 | 1 | 0 | 0 | 0 | 0 | 0 | 0 | 0 | 0 | 0 | 0 | 0 | 0 | 0 | 0 | 0 | 0 | 0 | 0 | | | | | | |
| 2523 | 02896 | 02522 | 1 | | 0 | 0 | 0 | 0 | 0 | 0 | 1 | 0 | 0 | 0 | 0 | 0 | 0 | 0 | 0 | 0 | 0 | 0 | 0 | 0 | 0 | 0 | 0 | 0 | 0 | | | | | | |
| 2524 | 02897 | 02523 | 2 | | 0 | 0 | 0 | 0 | 0 | 0 | 1 | 0 | 0 | 0 | 0 | 0 | 0 | 0 | 1 | 0 | 0 | 0 | 0 | 0 | 0 | 0 | 0 | 0 | 0 | | | | | | |
| 2525 | 02898 | 02524 | 1 | | 0 | 0 | 0 | 0 | 0 | 0 | 1 | 0 | 0 | 0 | 0 | 0 | 0 | 0 | 0 | 0 | 0 | 0 | 0 | 0 | 0 | 0 | 0 | 0 | 0 | | | | | | |
| 2526 | 02899 | 02525 | 1 | | 0 | 0 | 0 | 0 | 0 | 0 | 1 | 0 | 0 | 0 | 0 | 0 | 0 | 0 | 0 | 0 | 0 | 0 | 0 | 0 | 0 | 0 | 0 | 0 | 0 | | | | | | |
| 2527 | 02900 | 02526 | 4 | | 0 | 0 | 0 | 0 | 0 | 0 | 1 | 0 | 0 | 0 | 0 | 0 | 0 | 0 | 2 | 0 | 1 | 0 | 0 | 0 | 0 | 0 | 0 | 0 | 0 | X02751 | 98.3 | 117 | 1 | 333 | 2436 |
| 2528 | 02901 | 02527 | 3 | | 0 | 0 | 0 | 0 | 0 | 0 | 1 | 0 | 0 | 0 | 0 | 0 | 0 | 0 | 0 | 0 | 0 | 0 | 1 | 0 | 1 | 0 | 0 | 0 | 0 | | | | | | |
| 2529 | 02902 | 02528 | 1 | | 0 | 0 | 0 | 0 | 0 | 0 | 1 | 0 | 0 | 0 | 0 | 0 | 0 | 0 | 0 | 0 | 0 | 0 | 0 | 0 | 0 | 0 | 0 | 0 | 0 | | | | | | |
| 2530 | 02903 | 02529 | 2 | | 0 | 0 | 0 | 0 | 0 | 0 | 1 | 0 | 0 | 0 | 0 | 0 | 0 | 0 | 1 | 0 | 0 | 0 | 0 | 0 | 0 | 0 | 0 | 0 | 0 | | | | | | |
| 2531 | 02904 | 02530 | 1 | | 0 | 0 | 0 | 0 | 0 | 0 | 1 | 0 | 0 | 0 | 0 | 0 | 0 | 0 | 0 | 0 | 0 | 0 | 0 | 0 | 0 | 0 | 0 | 0 | 0 | | | | | | |
| 2532 | 02905 | 02531 | 8 | | 0 | 0 | 0 | 0 | 0 | 0 | 1 | 0 | 1 | 0 | 0 | 3 | 0 | 0 | 1 | 0 | 0 | 0 | 1 | 0 | 1 | 0 | 0 | 0 | 0 | | | | | | |
| 2533 | 02906 | 02532 | 1 | | 0 | 0 | 0 | 0 | 0 | 0 | 1 | 0 | 0 | 0 | 0 | 0 | 0 | 0 | 0 | 0 | 0 | 0 | 0 | 0 | 0 | 0 | 0 | 0 | 0 | | | | | | |
| 2534 | 02907 | 02533 | 7 | | 0 | 0 | 0 | 0 | 0 | 0 | 1 | 0 | 0 | 0 | 1 | 0 | 1 | 0 | 0 | 0 | 0 | 0 | 0 | 1 | 0 | 0 | 1 | 2 | 0 | | | | | | |
| 2535 | 02908 | 02534 | 1 | | 0 | 0 | 0 | 0 | 0 | 0 | 1 | 0 | 0 | 0 | 0 | 0 | 0 | 0 | 0 | 0 | 0 | 0 | 0 | 0 | 0 | 0 | 0 | 0 | 0 | | | | | | |
| 2536 | 02909 | 02535 | 1 | | 0 | 0 | 0 | 0 | 0 | 0 | 1 | 0 | 0 | 0 | 0 | 0 | 0 | 0 | 0 | 0 | 0 | 0 | 0 | 0 | 0 | 0 | 0 | 0 | 0 | | | | | | |
| 2537 | 02910 | 02536 | 2 | | 0 | 0 | 0 | 0 | 0 | 0 | 1 | 0 | 0 | 0 | 0 | 0 | 0 | 0 | 1 | 0 | 0 | 0 | 0 | 0 | 0 | 0 | 0 | 0 | 0 | | | | | | |
| 2538 | 02911 | 02537 | 1 | | 0 | 0 | 0 | 0 | 0 | 0 | 1 | 0 | 0 | 0 | 0 | 0 | 0 | 0 | 0 | 0 | 0 | 0 | 0 | 0 | 0 | 0 | 0 | 0 | 0 | | | | | | |
| 2539 | 02912 | 02538 | 3 | | 0 | 0 | 0 | 0 | 0 | 0 | 2 | 0 | 0 | 0 | 0 | 0 | 1 | 0 | 0 | 0 | 0 | 0 | 0 | 0 | 0 | 0 | 0 | 0 | 0 | | | | | | |
| 2540 | 02913 | 02539 | 3 | | 0 | 0 | 0 | 0 | 0 | 0 | 1 | 0 | 0 | 0 | 0 | 0 | 0 | 0 | 0 | 0 | 0 | 0 | 0 | 2 | 0 | 0 | 0 | 0 | 0 | | | | | | |
| 2541 | 02914 | 02540 | 1 | | 0 | 0 | 0 | 0 | 0 | 0 | 1 | 0 | 0 | 0 | 0 | 0 | 0 | 0 | 0 | 0 | 0 | 0 | 0 | 0 | 0 | 0 | 0 | 0 | 0 | | | | | | |

Table 71

| | A | B | C | E | G | I | K | M | O | Q | S | U | W | Y | AA | AC | AF | AG | AI | AK | AM | AO | AQ | AS | AU | AW | AY | BA | BC | BE | BF | BG | BH | BI | BK |
|---|---|---|---|---|---|---|---|---|---|---|---|---|---|---|---|---|---|---|---|---|---|---|---|---|---|---|---|---|---|---|---|---|---|---|---|
| 2542 | 02915 | 02541 | 4 | | 0 | 0 | 0 | 0 | 0 | 0 | 1 | 0 | 0 | 0 | 0 | 0 | 0 | 0 | 1 | 0 | 1 | 1 | 0 | 0 | 0 | 0 | 0 | 0 | 0 | | | | | | |
| 2543 | 02916 | 02542 | 8 | | 0 | 0 | 0 | 0 | 0 | 0 | 1 | 0 | 0 | 1 | 0 | 0 | 0 | 0 | 2 | 3 | 0 | 0 | 0 | 0 | 0 | 1 | 0 | 0 | 0 | | | | | | |
| 2544 | 02917 | 02543 | 3 | | 0 | 0 | 0 | 0 | 0 | 0 | 1 | 0 | 0 | 0 | 0 | 1 | 0 | 0 | 0 | 0 | 0 | 0 | 0 | 0 | 1 | 0 | 0 | 0 | 0 | | | | | | |
| 2545 | 02919 | 02544 | 1 | | 0 | 0 | 0 | 0 | 0 | 0 | 1 | 0 | 0 | 0 | 0 | 0 | 0 | 0 | 0 | 0 | 0 | 0 | 0 | 0 | 0 | 0 | 0 | 0 | 0 | | | | | | |
| 2546 | 02920 | 02545 | 3 | | 0 | 0 | 0 | 0 | 0 | 0 | 1 | 0 | 0 | 1 | 0 | 0 | 0 | 0 | 0 | 0 | 0 | 0 | 0 | 0 | 0 | 0 | 0 | 1 | 0 | | | | | | |
| 2547 | 02921 | 02546 | 1 | | 0 | 0 | 0 | 0 | 0 | 0 | 1 | 0 | 0 | 0 | 0 | 0 | 0 | 0 | 0 | 0 | 0 | 0 | 0 | 0 | 0 | 0 | 0 | 0 | 0 | | | | | | |
| 2548 | 02922 | 02547 | 3 | | 0 | 0 | 0 | 0 | 0 | 0 | 1 | 0 | 0 | 0 | 0 | 0 | 0 | 0 | 0 | 0 | 0 | 0 | 0 | 0 | 2 | 0 | 0 | 0 | 0 | | | | | | |
| 2549 | 02923 | 02548 | 2 | | 0 | 0 | 0 | 0 | 0 | 0 | 1 | 0 | 0 | 0 | 0 | 0 | 0 | 0 | 0 | 1 | 0 | 0 | 0 | 0 | 0 | 0 | 0 | 0 | 0 | | | | | | |
| 2550 | 02924 | 02549 | 3 | | 0 | 0 | 0 | 0 | 0 | 0 | 2 | 0 | 0 | 0 | 0 | 0 | 0 | 0 | 1 | 0 | 0 | 0 | 0 | 0 | 0 | 0 | 0 | 0 | 0 | M31606 | 95.9 | 97 | 5 | 1475 | 1571 |
| 2551 | 02925 | 02550 | 1 | | 0 | 0 | 0 | 0 | 0 | 0 | 1 | 0 | 0 | 0 | 0 | 0 | 0 | 0 | 0 | 0 | 0 | 0 | 0 | 0 | 0 | 0 | 0 | 0 | 0 | | | | | | |
| 2552 | 02926 | 02551 | 1 | | 0 | 0 | 0 | 0 | 0 | 0 | 1 | 0 | 0 | 0 | 0 | 0 | 0 | 0 | 0 | 0 | 0 | 0 | 0 | 0 | 0 | 0 | 0 | 0 | 0 | | | | | | |
| 2553 | 02927 | 02552 | 1 | | 0 | 0 | 0 | 0 | 0 | 0 | 1 | 0 | 0 | 0 | 0 | 0 | 0 | 0 | 0 | 0 | 0 | 0 | 0 | 0 | 0 | 0 | 0 | 0 | 0 | | | | | | |
| 2554 | 02928 | 02553 | 2 | | 0 | 0 | 0 | 0 | 0 | 0 | 1 | 0 | 0 | 0 | 0 | 0 | 0 | 0 | 0 | 0 | 0 | 1 | 0 | 0 | 0 | 0 | 0 | 0 | 0 | | | | | | |
| 2555 | 02929 | 02554 | 4 | | 0 | 0 | 0 | 0 | 0 | 0 | 1 | 0 | 0 | 1 | 0 | 2 | 0 | 0 | 0 | 0 | 0 | 0 | 0 | 0 | 0 | 0 | 0 | 0 | 0 | | | | | | |
| 2556 | 02930 | 02555 | 2 | | 0 | 0 | 0 | 0 | 0 | 0 | 1 | 0 | 0 | 1 | 0 | 0 | 0 | 0 | 0 | 0 | 0 | 0 | 0 | 0 | 0 | 0 | 0 | 0 | 0 | | | | | | |
| 2557 | 02931 | 02556 | 15 | | 0 | 0 | 0 | 0 | 0 | 0 | 1 | 1 | 0 | 0 | 1 | 0 | 0 | 6 | 0 | 1 | 0 | 0 | 2 | 2 | 0 | 0 | 0 | 1 | 0 | D10653 | 100 | 100 | 1 | 1644 | 1743 |
| 2558 | 02932 | 02557 | 4 | | 0 | 0 | 0 | 0 | 0 | 0 | 2 | 0 | 0 | 0 | 0 | 0 | 0 | 1 | 1 | 0 | 0 | 0 | 0 | 0 | 0 | 0 | 0 | 0 | 0 | | | | | | |
| 2559 | 02933 | 02558 | 2 | | 0 | 0 | 0 | 0 | 0 | 0 | 1 | 0 | 0 | 0 | 0 | 1 | 0 | 0 | 0 | 0 | 0 | 0 | 0 | 0 | 0 | 0 | 0 | 0 | 0 | | | | | | |
| 2560 | 02934 | 02559 | 1 | | 0 | 0 | 0 | 0 | 0 | 0 | 1 | 0 | 0 | 0 | 0 | 0 | 0 | 0 | 0 | 0 | 0 | 0 | 0 | 0 | 0 | 0 | 0 | 0 | 0 | | | | | | |
| 2561 | 02935 | 02560 | 4 | | 0 | 0 | 0 | 0 | 0 | 0 | 2 | 0 | 0 | 0 | 0 | 0 | 0 | 0 | 0 | 1 | 0 | 0 | 1 | 0 | 0 | 0 | 0 | 0 | 0 | | | | | | |
| 2562 | 02936 | 02561 | 1 | | 0 | 0 | 0 | 0 | 0 | 0 | 1 | 0 | 0 | 0 | 0 | 0 | 0 | 0 | 0 | 0 | 0 | 0 | 0 | 0 | 0 | 0 | 0 | 0 | 0 | | | | | | |
| 2563 | 02937 | 02562 | 1 | | 0 | 0 | 0 | 0 | 0 | 0 | 1 | 0 | 0 | 0 | 0 | 0 | 0 | 0 | 0 | 0 | 0 | 0 | 0 | 0 | 0 | 0 | 0 | 0 | 0 | | | | | | |
| 2564 | 02938 | 02563 | 4 | | 0 | 0 | 0 | 0 | 0 | 0 | 1 | 0 | 0 | 0 | 0 | 0 | 0 | 1 | 0 | 1 | 0 | 0 | 0 | 0 | 0 | 0 | 1 | 0 | 0 | | | | | | |
| 2565 | 02940 | 02564 | 1 | | 0 | 0 | 0 | 0 | 0 | 0 | 1 | 0 | 0 | 0 | 0 | 0 | 0 | 0 | 0 | 0 | 0 | 0 | 0 | 0 | 0 | 0 | 0 | 0 | 0 | | | | | | |
| 2566 | 02942 | 02565 | 1 | | 0 | 0 | 0 | 0 | 0 | 0 | 1 | 0 | 0 | 0 | 0 | 0 | 0 | 0 | 0 | 0 | 0 | 0 | 0 | 0 | 0 | 0 | 0 | 0 | 0 | | | | | | |
| 2567 | 02944 | 02566 | 1 | | 0 | 0 | 0 | 0 | 0 | 0 | 1 | 0 | 0 | 0 | 0 | 0 | 0 | 0 | 0 | 0 | 0 | 0 | 0 | 0 | 0 | 0 | 0 | 0 | 0 | | | | | | |
| 2568 | 02946 | 02567 | 2 | | 0 | 0 | 0 | 0 | 0 | 0 | 2 | 0 | 0 | 0 | 0 | 0 | 0 | 0 | 0 | 0 | 0 | 0 | 0 | 0 | 0 | 0 | 0 | 0 | 0 | | | | | | |
| 2569 | 02947 | 02568 | 2 | | 0 | 0 | 0 | 0 | 0 | 0 | 1 | 0 | 0 | 0 | 0 | 0 | 0 | 0 | 0 | 0 | 0 | 0 | 0 | 0 | 1 | 0 | 0 | 0 | 0 | | | | | | |
| 2570 | 02948 | 02569 | 1 | | 0 | 0 | 0 | 0 | 0 | 0 | 1 | 0 | 0 | 0 | 0 | 0 | 0 | 0 | 0 | 0 | 0 | 0 | 0 | 0 | 0 | 0 | 0 | 0 | 0 | | | | | | |
| 2571 | 02949 | 02570 | 4 | | 0 | 0 | 0 | 0 | 0 | 0 | 1 | 0 | 1 | 0 | 0 | 1 | 0 | 0 | 0 | 0 | 0 | 0 | 0 | 1 | 0 | 0 | 0 | 0 | 0 | | | | | | |
| 2572 | 02950 | 02571 | 1 | | 0 | 0 | 0 | 0 | 0 | 0 | 1 | 0 | 0 | 0 | 0 | 0 | 0 | 0 | 0 | 0 | 0 | 0 | 0 | 0 | 0 | 0 | 0 | 0 | 0 | | | | | | |
| 2573 | 02951 | 02572 | 1 | | 0 | 0 | 0 | 0 | 0 | 0 | 1 | 0 | 0 | 0 | 0 | 0 | 0 | 0 | 0 | 0 | 0 | 0 | 0 | 0 | 0 | 0 | 0 | 0 | 0 | | | | | | |
| 2574 | 02953 | 02573 | 1 | | 0 | 0 | 0 | 0 | 0 | 0 | 1 | 0 | 0 | 0 | 0 | 0 | 0 | 0 | 0 | 0 | 0 | 0 | 0 | 0 | 0 | 0 | 0 | 0 | 0 | | | | | | |
| 2575 | 02955 | 02574 | 4 | | 0 | 0 | 0 | 0 | 0 | 0 | 1 | 0 | 0 | 0 | 0 | 0 | 2 | 1 | 0 | 0 | 0 | 0 | 0 | 0 | 0 | 0 | 0 | 0 | 0 | | | | | | |
| 2576 | 02957 | 02575 | 1 | | 0 | 0 | 0 | 0 | 0 | 0 | 1 | 0 | 0 | 0 | 0 | 0 | 0 | 0 | 0 | 0 | 0 | 0 | 0 | 0 | 0 | 0 | 0 | 0 | 0 | | | | | | |
| 2577 | 02958 | 02576 | 2 | | 0 | 0 | 0 | 0 | 0 | 0 | 2 | 0 | 0 | 0 | 0 | 0 | 0 | 0 | 0 | 0 | 0 | 0 | 0 | 0 | 0 | 0 | 0 | 0 | 0 | S60088 | 92.7 | 82 | 1 | 2535 | 4121 |

Table 72

| | A | B | C | E | G | I | K | M | O | Q | S | U | W | Y | AA | AC | AE | AG | AI | AK | AM | AO | AQ | AS | AU | AW | AY | BA | BC | BE | BF | BG | BH | BI | BK |
|---|---|---|---|---|---|---|---|---|---|---|---|---|---|---|---|---|---|---|---|---|---|---|---|---|---|---|---|---|---|---|---|---|---|---|---|
| 2578 | 02959 | 02577 | 9 | | 0 | 0 | 0 | 0 | 0 | 0 | 2 | 0 | 0 | 0 | 1 | 0 | 0 | 0 | 1 | 0 | 0 | 1 | 0 | 1 | 2 | 0 | 1 | 0 | 0 | X02751 | 93.8 | 80 | 6 | 1 | 2436 |
| 2579 | 02960 | 02578 | 1 | | 0 | 0 | 0 | 0 | 0 | 0 | 1 | 0 | 0 | 0 | 0 | 0 | 0 | 0 | 0 | 0 | 0 | 0 | 0 | 0 | 0 | 0 | 0 | 0 | 0 | | | | | | |
| 2580 | 02962 | 02579 | 1 | | 0 | 0 | 0 | 0 | 0 | 0 | 1 | 0 | 0 | 0 | 0 | 0 | 0 | 0 | 0 | 0 | 0 | 0 | 0 | 0 | 0 | 0 | 0 | 0 | 0 | | | | | | |
| 2581 | 02963 | 02580 | 2 | | 0 | 0 | 0 | 0 | 0 | 0 | 1 | 0 | 0 | 1 | 0 | 0 | 0 | 0 | 0 | 0 | 0 | 0 | 0 | 0 | 0 | 0 | 0 | 0 | 0 | | | | | | |
| 2582 | 02964 | 02581 | 1 | | 0 | 0 | 0 | 0 | 0 | 0 | 1 | 0 | 0 | 0 | 0 | 0 | 0 | 0 | 0 | 0 | 0 | 0 | 0 | 0 | 0 | 0 | 0 | 0 | 0 | | | | | | |
| 2583 | 02965 | 02582 | 3 | | 0 | 0 | 0 | 0 | 0 | 0 | 1 | 0 | 0 | 0 | 0 | 0 | 2 | 0 | 0 | 0 | 0 | 0 | 0 | 0 | 0 | 0 | 0 | 0 | 0 | | | | | | |
| 2584 | 02966 | 02583 | 1 | | 0 | 0 | 0 | 0 | 0 | 0 | 1 | 0 | 0 | 0 | 0 | 0 | 0 | 0 | 0 | 0 | 0 | 0 | 0 | 0 | 0 | 0 | 0 | 0 | 0 | X62744 | 100 | 72 | 1 | 1011 | 1100 |
| 2585 | 02967 | 02584 | 1 | | 0 | 0 | 0 | 0 | 0 | 0 | 1 | 0 | 0 | 0 | 0 | 0 | 0 | 0 | 0 | 0 | 0 | 0 | 0 | 0 | 0 | 0 | 0 | 0 | 0 | X64002 | 98.6 | 71 | 1 | 1581 | 2343 |
| 2586 | 02968 | 02585 | 2 | | 0 | 0 | 0 | 0 | 0 | 0 | 1 | 0 | 0 | 0 | 0 | 0 | 0 | 0 | 1 | 0 | 0 | 0 | 0 | 0 | 0 | 0 | 0 | 0 | 0 | | | | | | |
| 2587 | 02969 | 02586 | 5 | | 0 | 0 | 0 | 0 | 0 | 0 | 1 | 0 | 0 | 0 | 0 | 0 | 0 | 0 | 0 | 1 | 0 | 0 | 0 | 1 | 0 | 0 | 0 | 0 | 2 | | | | | | |
| 2588 | 02970 | 02587 | 2 | | 0 | 0 | 0 | 0 | 0 | 0 | 1 | 0 | 0 | 0 | 0 | 0 | 0 | 0 | 0 | 0 | 0 | 0 | 0 | 1 | 0 | 0 | 0 | 0 | 0 | | | | | | |
| 2589 | 02971 | 02588 | 5 | | 0 | 0 | 0 | 0 | 0 | 0 | 1 | 0 | 0 | 0 | 2 | 0 | 0 | 0 | 1 | 0 | 0 | 0 | 0 | 1 | 0 | 0 | 0 | 0 | 0 | | | | | | |
| 2590 | 02972 | 02589 | 2 | | 0 | 0 | 0 | 0 | 0 | 0 | 1 | 0 | 0 | 0 | 0 | 0 | 0 | 0 | 0 | 0 | 0 | 0 | 0 | 0 | 0 | 0 | 0 | 1 | 0 | | | | | | |
| 2591 | 02973 | 02590 | 7 | | 0 | 0 | 0 | 0 | 0 | 0 | 1 | 0 | 0 | 0 | 0 | 1 | 0 | 2 | 0 | 2 | 0 | 0 | 0 | 0 | 0 | 0 | 0 | 1 | 0 | L13799 | 95.5 | 67 | 1 | 436 | 500 |
| 2592 | 02974 | 02591 | 8 | | 0 | 0 | 0 | 0 | 0 | 0 | 1 | 1 | 0 | 1 | 0 | 0 | 0 | 0 | 3 | 0 | 0 | 1 | 0 | 0 | 1 | 0 | 0 | 0 | 0 | U05291 | 100 | 65 | 1 | 1828 | 1892 |
| 2593 | 02975 | 02592 | 1 | | 0 | 0 | 0 | 0 | 0 | 0 | 1 | 0 | 0 | 0 | 0 | 0 | 0 | 0 | 0 | 0 | 0 | 0 | 0 | 0 | 0 | 0 | 0 | 0 | 0 | | | | | | |
| 2594 | 02976 | 02593 | 5 | | 0 | 0 | 0 | 0 | 0 | 0 | 2 | 0 | 0 | 0 | 0 | 0 | 0 | 0 | 0 | 1 | 0 | 0 | 0 | 1 | 1 | 0 | 0 | 0 | 0 | | | | | | |
| 2595 | 02978 | 02594 | 1 | | 0 | 0 | 0 | 0 | 0 | 0 | 1 | 0 | 0 | 0 | 0 | 0 | 0 | 0 | 0 | 0 | 0 | 0 | 0 | 0 | 0 | 0 | 0 | 0 | 0 | | | | | | |
| 2596 | 02980 | 02595 | 1 | | 0 | 0 | 0 | 0 | 0 | 0 | 1 | 0 | 0 | 0 | 0 | 0 | 0 | 0 | 0 | 0 | 0 | 0 | 0 | 0 | 0 | 0 | 0 | 0 | 0 | | | | | | |
| 2597 | 02981 | 02596 | 13 | | 0 | 0 | 0 | 0 | 0 | 0 | 1 | 3 | 0 | 0 | 0 | 0 | 0 | 0 | 0 | 0 | 0 | 0 | 0 | 0 | 9 | 0 | 0 | 0 | 0 | M84526 | 93.8 | 64 | 1 | 894 | 1071 |
| 2598 | 02982 | 02597 | 5 | | 0 | 0 | 0 | 0 | 0 | 0 | 2 | 2 | 0 | 0 | 0 | 0 | 0 | 0 | 0 | 0 | 0 | 0 | 0 | 0 | 0 | 0 | 0 | 1 | 0 | | | | | | |
| 2599 | 02983 | 02598 | 5 | | 0 | 0 | 0 | 0 | 0 | 0 | 2 | 0 | 0 | 0 | 0 | 0 | 1 | 0 | 0 | 0 | 0 | 0 | 0 | 2 | 0 | 0 | 0 | 0 | 0 | | | | | | |
| 2600 | 02984 | 02599 | 1 | | 0 | 0 | 0 | 0 | 0 | 0 | 1 | 0 | 0 | 0 | 0 | 0 | 0 | 0 | 0 | 0 | 0 | 0 | 0 | 0 | 0 | 0 | 0 | 0 | 0 | | | | | | |
| 2601 | 02985 | 02600 | 2 | | 0 | 0 | 0 | 0 | 0 | 0 | 2 | 0 | 0 | 0 | 0 | 0 | 0 | 0 | 0 | 0 | 0 | 0 | 0 | 0 | 0 | 0 | 0 | 0 | 0 | M31210 | 100 | 58 | 1 | 2699 | 2757 |
| 2602 | 02986 | 02601 | 1 | | 0 | 0 | 0 | 0 | 0 | 0 | 1 | 0 | 0 | 0 | 0 | 0 | 0 | 0 | 0 | 0 | 0 | 0 | 0 | 0 | 0 | 0 | 0 | 0 | 0 | | | | | | |
| 2603 | 02987 | 02602 | 3 | | 0 | 0 | 0 | 0 | 0 | 0 | 1 | 1 | 0 | 1 | 0 | 0 | 0 | 0 | 0 | 0 | 0 | 0 | 0 | 0 | 0 | 0 | 0 | 0 | 0 | | | | | | |
| 2604 | 02988 | 02603 | 6 | | 0 | 0 | 0 | 0 | 0 | 0 | 1 | 2 | 0 | 0 | 0 | 0 | 0 | 0 | 0 | 0 | 0 | 0 | 0 | 0 | 0 | 2 | 0 | 1 | 0 | S57501 | 96.6 | 58 | 1 | 1336 | 1400 |
| 2605 | 02989 | 02604 | 3 | | 0 | 0 | 0 | 0 | 0 | 0 | 1 | 0 | 0 | 0 | 0 | 0 | 0 | 0 | 0 | 1 | 0 | 0 | 1 | 0 | 0 | 0 | 0 | 0 | 0 | X16901 | 96.2 | 53 | 1 | 1357 | 1408 |
| 2606 | 02990 | 02605 | 1 | | 0 | 0 | 0 | 0 | 0 | 0 | 1 | 0 | 0 | 0 | 0 | 0 | 0 | 0 | 0 | 0 | 0 | 0 | 0 | 0 | 0 | 0 | 0 | 0 | 0 | | | | | | |
| 2607 | 02991 | 02606 | 9 | | 0 | 0 | 0 | 0 | 0 | 0 | 1 | 0 | 0 | 2 | 0 | 0 | 2 | 1 | 0 | 0 | 0 | 0 | 2 | 1 | 0 | 0 | 0 | 0 | 0 | | | | | | |
| 2608 | 02992 | 02607 | 9 | | 0 | 0 | 0 | 0 | 0 | 0 | 1 | 0 | 1 | 0 | 0 | 0 | 0 | 1 | 0 | 2 | 0 | 0 | 0 | 1 | 1 | 2 | 0 | 0 | 0 | X62585 | 100 | 54 | 1 | 2506 | 2563 |
| 2609 | 02993 | 02608 | 3 | | 0 | 0 | 0 | 0 | 0 | 0 | 2 | 0 | 0 | 0 | 0 | 0 | 0 | 0 | 0 | 1 | 0 | 0 | 0 | 0 | 0 | 0 | 0 | 0 | 0 | X03747 | 96.2 | 52 | 1 | 2154 | 2208 |
| 2610 | 02994 | 02609 | 2 | | 0 | 0 | 0 | 0 | 0 | 0 | 1 | 0 | 0 | 0 | 0 | 0 | 0 | 0 | 0 | 0 | 1 | 0 | 0 | 0 | 0 | 0 | 0 | 0 | 0 | | | | | | |
| 2611 | 02995 | 02610 | 1 | | 0 | 0 | 0 | 0 | 0 | 0 | 1 | 0 | 0 | 0 | 0 | 0 | 0 | 0 | 0 | 0 | 0 | 0 | 0 | 0 | 0 | 0 | 0 | 0 | 0 | | | | | | |
| 2612 | 02996 | 02611 | 1 | | 0 | 0 | 0 | 0 | 0 | 0 | 1 | 0 | 0 | 0 | 0 | 0 | 0 | 0 | 0 | 0 | 0 | 0 | 0 | 0 | 0 | 0 | 0 | 0 | 0 | | | | | | |
| 2613 | 02997 | 02612 | 1 | | 0 | 0 | 0 | 0 | 0 | 0 | 1 | 0 | 0 | 0 | 0 | 0 | 0 | 0 | 0 | 0 | 0 | 0 | 0 | 0 | 0 | 0 | 0 | 0 | 0 | J04621 | 98.1 | 52 | 1 | 2626 | 3414 |

Table 73

| | A | B | C | E | G | I | K | M | O | Q | S | U | W | Y | AA | AC | AE | AG | AI | AK | AM | AO | AQ | AS | AU | AW | AY | BA | BC | BE | BF | BG | BH | BI | BK |
|---|---|---|---|---|---|---|---|---|---|---|---|---|---|---|---|---|---|---|---|---|---|---|---|---|---|---|---|---|---|---|---|---|---|---|---|
| 2614 | 02999 | 02613 | 1 | | 0 | 0 | 0 | 0 | 0 | 0 | 1 | 0 | 0 | 0 | 0 | 0 | 0 | 0 | 0 | 0 | 0 | 0 | 0 | 0 | 0 | 0 | 0 | 0 | 0 | | | | | | |
| 2615 | 03002 | 02614 | 3 | | 0 | 0 | 0 | 0 | 0 | 0 | 1 | 0 | 0 | 0 | 0 | 1 | 0 | 0 | 1 | 0 | 0 | 0 | 0 | 0 | 0 | 0 | 0 | 0 | 0 | | | | | | |
| 2616 | 03005 | 02615 | 3 | | 0 | 0 | 0 | 0 | 0 | 0 | 1 | 0 | 0 | 0 | 0 | 0 | 0 | 0 | 0 | 2 | 0 | 0 | 0 | 0 | 0 | 0 | 0 | 0 | 0 | | | | | | |
| 2617 | 03028 | 02616 | 2 | | 0 | 0 | 0 | 0 | 0 | 0 | 1 | 0 | 0 | 0 | 0 | 1 | 0 | 0 | 0 | 0 | 0 | 0 | 0 | 0 | 0 | 0 | 0 | 0 | 0 | | | | | | |
| 2618 | 03030 | 02617 | 3 | | 0 | 0 | 0 | 0 | 0 | 0 | 1 | 0 | 0 | 0 | 0 | 2 | 0 | 0 | 0 | 0 | 0 | 0 | 0 | 0 | 0 | 0 | 0 | 0 | 0 | | | | | | |
| 2619 | 03031 | 02618 | 3 | | 0 | 0 | 0 | 0 | 0 | 0 | 1 | 1 | 0 | 0 | 0 | 1 | 0 | 0 | 0 | 0 | 0 | 0 | 0 | 0 | 0 | 0 | 0 | 0 | 0 | | | | | | |
| 2620 | 03053 | 02619 | 3 | | 0 | 0 | 0 | 0 | 0 | 0 | 0 | 1 | 0 | 0 | 0 | 0 | 0 | 0 | 0 | 0 | 0 | 0 | 0 | 0 | 0 | 1 | 1 | 0 | 0 | | | | | | |
| 2621 | 03055 | 02620 | 1 | | 0 | 0 | 0 | 0 | 0 | 0 | 0 | 1 | 0 | 0 | 0 | 0 | 0 | 0 | 0 | 0 | 0 | 0 | 0 | 0 | 0 | 0 | 0 | 0 | 0 | | | | | | |
| 2622 | 03056 | 02621 | 3 | | 0 | 0 | 0 | 0 | 0 | 0 | 0 | 2 | 0 | 0 | 0 | 0 | 0 | 0 | 0 | 0 | 0 | 0 | 0 | 0 | 1 | 0 | 0 | 0 | 0 | J02874 | 93.5 | 429 | 1 | 209 | 634 |
| 2623 | 03058 | 02622 | 3 | | 0 | 0 | 0 | 0 | 0 | 0 | 0 | 2 | 0 | 0 | 0 | 0 | 0 | 0 | 0 | 0 | 0 | 0 | 0 | 0 | 0 | 1 | 0 | 0 | 0 | | | | | | |
| 2624 | 03059 | 02623 | 1 | | 0 | 0 | 0 | 0 | 0 | 0 | 0 | 1 | 0 | 0 | 0 | 0 | 0 | 0 | 0 | 0 | 0 | 0 | 0 | 0 | 0 | 0 | 0 | 0 | 0 | | | | | | |
| 2625 | 03060 | 02624 | 2 | | 0 | 0 | 0 | 0 | 0 | 0 | 0 | 1 | 0 | 0 | 0 | 0 | 0 | 0 | 0 | 0 | 0 | 0 | 0 | 1 | 0 | 0 | 0 | 0 | 0 | | | | | | |
| 2626 | 03061 | 02625 | 2 | | 0 | 0 | 0 | 0 | 0 | 0 | 0 | 2 | 0 | 0 | 0 | 0 | 0 | 0 | 0 | 0 | 0 | 0 | 0 | 0 | 0 | 0 | 0 | 0 | 0 | D17793 | 96.5 | 397 | 1 | 737 | 1227 |
| 2627 | 03062 | 02626 | 6 | | 0 | 0 | 0 | 0 | 0 | 0 | 0 | 1 | 0 | 0 | 1 | 0 | 0 | 0 | 1 | 0 | 0 | 1 | 0 | 0 | 1 | 1 | 0 | 0 | 0 | D14662 | 97.1 | 375 | 1 | 1280 | 1653 |
| 2628 | 03063 | 02627 | 1 | | 0 | 0 | 0 | 0 | 0 | 0 | 0 | 1 | 0 | 0 | 0 | 0 | 0 | 0 | 0 | 0 | 0 | 0 | 0 | 0 | 0 | 0 | 0 | 0 | 0 | | | | | | |
| 2629 | 03064 | 02628 | 4 | | 0 | 0 | 0 | 0 | 0 | 0 | 0 | 1 | 0 | 0 | 0 | 2 | 0 | 0 | 0 | 0 | 0 | 0 | 0 | 0 | 0 | 0 | 1 | 0 | 0 | | | | | | |
| 2630 | 03065 | 02629 | 4 | | 0 | 0 | 0 | 0 | 0 | 0 | 0 | 2 | 0 | 0 | 0 | 0 | 0 | 0 | 0 | 0 | 0 | 0 | 0 | 0 | 2 | 0 | 0 | 0 | 0 | | | | | | |
| 2631 | 03066 | 02630 | 2 | | 0 | 0 | 0 | 0 | 0 | 0 | 0 | 1 | 0 | 0 | 0 | 0 | 0 | 0 | 0 | 0 | 1 | 0 | 0 | 0 | 0 | 0 | 0 | 0 | 0 | | | | | | |
| 2632 | 03067 | 02631 | 1 | | 0 | 0 | 0 | 0 | 0 | 0 | 0 | 1 | 0 | 0 | 0 | 0 | 0 | 0 | 0 | 0 | 0 | 0 | 0 | 0 | 0 | 0 | 0 | 0 | 0 | | | | | | |
| 2633 | 03068 | 02632 | 1 | | 0 | 0 | 0 | 0 | 0 | 0 | 0 | 1 | 0 | 0 | 0 | 0 | 0 | 0 | 0 | 0 | 0 | 0 | 0 | 0 | 0 | 0 | 0 | 0 | 0 | | | | | | |
| 2634 | 03069 | 02633 | 1 | | 0 | 0 | 0 | 0 | 0 | 0 | 0 | 1 | 0 | 0 | 0 | 0 | 0 | 0 | 0 | 0 | 0 | 0 | 0 | 0 | 0 | 0 | 0 | 0 | 0 | | | | | | |
| 2635 | 03070 | 02634 | 3 | | 0 | 0 | 0 | 0 | 0 | 0 | 0 | 1 | 0 | 0 | 1 | 0 | 0 | 0 | 0 | 1 | 0 | 0 | 0 | 0 | 0 | 0 | 0 | 0 | 0 | | | | | | |
| 2636 | 03071 | 02635 | 1 | | 0 | 0 | 0 | 0 | 0 | 0 | 0 | 1 | 0 | 0 | 0 | 0 | 0 | 0 | 0 | 0 | 0 | 0 | 0 | 0 | 0 | 0 | 0 | 0 | 0 | | | | | | |
| 2637 | 03072 | 02636 | 1 | | 0 | 0 | 0 | 0 | 0 | 0 | 0 | 1 | 0 | 0 | 0 | 0 | 0 | 0 | 0 | 0 | 0 | 0 | 0 | 0 | 0 | 0 | 0 | 0 | 0 | | | | | | |
| 2638 | 03073 | 02637 | 5 | | 0 | 0 | 0 | 0 | 0 | 0 | 0 | 1 | 0 | 0 | 0 | 0 | 0 | 0 | 0 | 0 | 0 | 0 | 0 | 0 | 0 | 2 | 0 | 2 | 0 | | | | | | |
| 2639 | 03074 | 02638 | 44 | | 0 | 0 | 0 | 0 | 0 | 0 | 0 | 3 | 0 | 18 | 0 | 0 | 0 | 0 | 20 | 1 | 0 | 2 | 0 | 0 | 0 | 0 | 0 | 0 | 0 | X14420 | 97.3 | 329 | 1 | 4459 | 5460 |
| 2640 | 03075 | 02639 | 5 | | 0 | 0 | 0 | 0 | 0 | 0 | 0 | 1 | 1 | 1 | 0 | 0 | 0 | 0 | 1 | 1 | 0 | 0 | 0 | 0 | 0 | 0 | 0 | 0 | 0 | X61598 | 96.4 | 304 | 1 | 1507 | 1936 |
| 2641 | 03077 | 02640 | 5 | | 0 | 0 | 0 | 0 | 0 | 0 | 0 | 1 | 0 | 1 | 0 | 0 | 0 | 0 | 1 | 1 | 0 | 0 | 1 | 0 | 0 | 0 | 0 | 0 | 0 | | | | | | |
| 2642 | 03078 | 02641 | 2 | | 0 | 0 | 0 | 0 | 0 | 0 | 0 | 1 | 0 | 0 | 1 | 0 | 0 | 0 | 0 | 0 | 0 | 0 | 0 | 0 | 0 | 0 | 0 | 0 | 0 | | | | | | |
| 2643 | 03079 | 02642 | 1 | | 0 | 0 | 0 | 0 | 0 | 0 | 0 | 1 | 0 | 0 | 0 | 0 | 0 | 0 | 0 | 0 | 0 | 0 | 0 | 0 | 0 | 0 | 0 | 0 | 0 | | | | | | |
| 2644 | 03080 | 02643 | 2 | | 0 | 0 | 0 | 0 | 0 | 0 | 0 | 1 | 0 | 0 | 0 | 1 | 0 | 0 | 0 | 0 | 0 | 0 | 0 | 0 | 0 | 0 | 0 | 0 | 0 | | | | | | |
| 2645 | 03081 | 02644 | 1 | | 0 | 0 | 0 | 0 | 0 | 0 | 0 | 1 | 0 | 0 | 0 | 0 | 0 | 0 | 0 | 0 | 0 | 0 | 0 | 0 | 0 | 0 | 0 | 0 | 0 | | | | | | |
| 2646 | 03082 | 02645 | 1 | | 0 | 0 | 0 | 0 | 0 | 0 | 0 | 1 | 0 | 0 | 0 | 0 | 0 | 0 | 0 | 0 | 0 | 0 | 0 | 0 | 0 | 0 | 0 | 0 | 0 | | | | | | |
| 2647 | 03083 | 02646 | 1 | | 0 | 0 | 0 | 0 | 0 | 0 | 0 | 1 | 0 | 0 | 0 | 0 | 0 | 0 | 0 | 0 | 0 | 0 | 0 | 0 | 0 | 0 | 0 | 0 | 0 | | | | | | |
| 2648 | 03084 | 02647 | 2 | | 0 | 0 | 0 | 0 | 0 | 0 | 0 | 1 | 0 | 0 | 0 | 0 | 0 | 1 | 0 | 0 | 0 | 0 | 0 | 0 | 0 | 0 | 0 | 0 | 0 | | | | | | |
| 2649 | 03085 | 02648 | 2 | | 0 | 0 | 0 | 0 | 0 | 0 | 0 | 1 | 0 | 0 | 0 | 0 | 0 | 0 | 1 | 0 | 0 | 0 | 0 | 0 | 0 | 0 | 0 | 0 | 0 | | | | | | |

Table 74

| | A | B | C | E | G | I | K | M | O | Q | S | U | W | Y | AA | AC | AE | AG | AI | AK | AM | AO | AQ | AS | AU | AW | AY | BA | BC | BE | BF | BG | BH | BI | BK |
|---|---|---|---|---|---|---|---|---|---|---|---|---|---|---|---|---|---|---|---|---|---|---|---|---|---|---|---|---|---|---|---|---|---|---|---|
| 2650 | 03086 | 02649 | 5 | | 0 | 0 | 0 | 0 | 0 | 0 | 0 | 1 | 0 | 3 | 0 | 0 | 0 | 0 | 0 | 0 | 0 | 0 | 0 | 0 | 1 | 0 | 0 | 0 | 0 | | | | | | |
| 2651 | 03087 | 02650 | 8 | | 0 | 0 | 0 | 0 | 0 | 0 | 0 | 2 | 0 | 0 | 0 | 0 | 0 | 1 | 0 | 0 | 0 | 1 | 1 | 2 | 0 | 0 | 0 | 0 | 1 | S45630 | 99.3 | 298 | 1 | 394 | 691 |
| 2652 | 03088 | 02651 | 1 | | 0 | 0 | 0 | 0 | 0 | 0 | 0 | 1 | 0 | 0 | 0 | 0 | 0 | 0 | 0 | 0 | 0 | 0 | 0 | 0 | 0 | 0 | 0 | 0 | 0 | | | | | | |
| 2653 | 03089 | 02652 | 1 | | 0 | 0 | 0 | 0 | 0 | 0 | 0 | 1 | 0 | 0 | 0 | 0 | 0 | 0 | 0 | 0 | 0 | 0 | 0 | 0 | 0 | 0 | 0 | 0 | 0 | | | | | | |
| 2654 | 03090 | 02653 | 8 | | 0 | 0 | 0 | 0 | 0 | 0 | 0 | 1 | 0 | 0 | 2 | 0 | 0 | 0 | 3 | 0 | 1 | 0 | 0 | 0 | 1 | 0 | 0 | 0 | 0 | | | | | | |
| 2655 | 03091 | 02654 | 1 | | 0 | 0 | 0 | 0 | 0 | 0 | 0 | 1 | 0 | 0 | 0 | 0 | 0 | 0 | 0 | 0 | 0 | 0 | 0 | 0 | 0 | 0 | 0 | 0 | 0 | | | | | | |
| 2656 | 03092 | 02655 | 4 | | 0 | 0 | 0 | 0 | 0 | 0 | 0 | 1 | 0 | 0 | 0 | 2 | 0 | 0 | 0 | 1 | 0 | 0 | 0 | 0 | 0 | 0 | 0 | 0 | 0 | | | | | | |
| 2657 | 03094 | 02656 | 2 | | 0 | 0 | 0 | 0 | 0 | 0 | 0 | 1 | 0 | 0 | 0 | 0 | 0 | 0 | 0 | 0 | 0 | 0 | 0 | 0 | 0 | 0 | 0 | 1 | 0 | | | | | | |
| 2658 | 03095 | 02657 | 1 | | 0 | 0 | 0 | 0 | 0 | 0 | 0 | 1 | 0 | 0 | 0 | 0 | 0 | 0 | 0 | 0 | 0 | 0 | 0 | 0 | 0 | 0 | 0 | 0 | 0 | M12623 | 97.1 | 279 | 1 | 159 | 1187 |
| 2659 | 03096 | 02658 | 1 | | 0 | 0 | 0 | 0 | 0 | 0 | 0 | 1 | 0 | 0 | 0 | 0 | 0 | 0 | 0 | 0 | 0 | 0 | 0 | 0 | 0 | 0 | 0 | 0 | 0 | | | | | | |
| 2660 | 03097 | 02659 | 2 | | 0 | 0 | 0 | 0 | 0 | 0 | 0 | 1 | 0 | 0 | 0 | 0 | 0 | 0 | 1 | 0 | 0 | 0 | 0 | 0 | 0 | 0 | 0 | 0 | 0 | | | | | | |
| 2661 | 03098 | 02660 | 1 | | 0 | 0 | 0 | 0 | 0 | 0 | 0 | 1 | 0 | 0 | 0 | 0 | 0 | 0 | 0 | 0 | 0 | 0 | 0 | 0 | 0 | 0 | 0 | 0 | 0 | | | | | | |
| 2662 | 03099 | 02661 | 1 | | 0 | 0 | 0 | 0 | 0 | 0 | 0 | 1 | 0 | 0 | 0 | 0 | 0 | 0 | 0 | 0 | 0 | 0 | 0 | 0 | 0 | 0 | 0 | 0 | 0 | X63547 | 91 | 266 | 7 | 7860 | 8201 |
| 2663 | 03100 | 02662 | 1 | | 0 | 0 | 0 | 0 | 0 | 0 | 0 | 1 | 0 | 0 | 0 | 0 | 0 | 0 | 0 | 0 | 0 | 0 | 0 | 0 | 0 | 0 | 0 | 0 | 0 | | | | | | |
| 2664 | 03101 | 02663 | 2 | | 0 | 0 | 0 | 0 | 0 | 0 | 0 | 1 | 0 | 1 | 0 | 0 | 0 | 0 | 0 | 0 | 0 | 0 | 0 | 0 | 0 | 0 | 0 | 0 | 0 | | | | | | |
| 2665 | 03102 | 02664 | 2 | | 0 | 0 | 0 | 0 | 0 | 0 | 0 | 1 | 0 | 0 | 0 | 0 | 0 | 0 | 0 | 0 | 0 | 0 | 1 | 0 | 0 | 0 | 0 | 0 | 0 | | | | | | |
| 2666 | 03103 | 02665 | 2 | | 0 | 0 | 0 | 0 | 0 | 0 | 0 | 1 | 0 | 0 | 0 | 0 | 0 | 0 | 0 | 0 | 0 | 0 | 0 | 0 | 0 | 1 | 0 | 0 | 0 | | | | | | |
| 2667 | 03104 | 02666 | 3 | | 0 | 0 | 0 | 0 | 0 | 0 | 0 | 1 | 0 | 2 | 0 | 0 | 0 | 0 | 0 | 0 | 0 | 0 | 0 | 0 | 0 | 0 | 0 | 0 | 0 | M27508 | 99.6 | 265 | 1 | 1741 | 2007 |
| 2668 | 03105 | 02667 | 15 | | 0 | 0 | 0 | 0 | 0 | 0 | 0 | 2 | 3 | 4 | 0 | 0 | 0 | 0 | 0 | 0 | 0 | 3 | 0 | 0 | 3 | 0 | 0 | 0 | 0 | | | | | | |
| 2669 | 03106 | 02668 | 9 | | 0 | 0 | 0 | 0 | 0 | 0 | 0 | 2 | 0 | 0 | 0 | 0 | 0 | 0 | 0 | 0 | 0 | 0 | 0 | 0 | 7 | 0 | 0 | 0 | 0 | X58295 | 99.2 | 256 | 1 | 1333 | 1606 |
| 2670 | 03108 | 02669 | 1 | | 0 | 0 | 0 | 0 | 0 | 0 | 0 | 1 | 0 | 0 | 0 | 0 | 0 | 0 | 0 | 0 | 0 | 0 | 0 | 0 | 0 | 0 | 0 | 0 | 0 | | | | | | |
| 2671 | 03109 | 02670 | 13 | | 0 | 0 | 0 | 0 | 0 | 0 | 0 | 13 | 0 | 0 | 0 | 0 | 0 | 0 | 0 | 0 | 0 | 0 | 0 | 0 | 0 | 0 | 0 | 0 | 0 | | | | | | |
| 2672 | 03110 | 02671 | 1 | | 0 | 0 | 0 | 0 | 0 | 0 | 0 | 1 | 0 | 0 | 0 | 0 | 0 | 0 | 0 | 0 | 0 | 0 | 0 | 0 | 0 | 0 | 0 | 0 | 0 | | | | | | |
| 2673 | 03111 | 02672 | 1 | | 0 | 0 | 0 | 0 | 0 | 0 | 0 | 1 | 0 | 0 | 0 | 0 | 0 | 0 | 0 | 0 | 0 | 0 | 0 | 0 | 0 | 0 | 0 | 0 | 0 | | | | | | |
| 2674 | 03112 | 02673 | 1 | | 0 | 0 | 0 | 0 | 0 | 0 | 0 | 1 | 0 | 0 | 0 | 0 | 0 | 0 | 0 | 0 | 0 | 0 | 0 | 0 | 0 | 0 | 0 | 0 | 0 | | | | | | |
| 2675 | 03115 | 02674 | 3 | | 0 | 0 | 0 | 0 | 0 | 0 | 0 | 1 | 0 | 0 | 2 | 0 | 0 | 0 | 0 | 0 | 0 | 0 | 0 | 0 | 0 | 0 | 0 | 0 | 0 | M36532 | 91.5 | 435 | 1 | 890 | 1363 |
| 2676 | 03116 | 02675 | 2 | | 0 | 0 | 0 | 0 | 0 | 0 | 0 | 1 | 0 | 0 | 0 | 0 | 0 | 0 | 1 | 0 | 0 | 0 | 0 | 0 | 0 | 0 | 0 | 0 | 0 | | | | | | |
| 2677 | 03117 | 02676 | 1 | | 0 | 0 | 0 | 0 | 0 | 0 | 0 | 1 | 0 | 0 | 0 | 0 | 0 | 0 | 0 | 0 | 0 | 0 | 0 | 0 | 0 | 0 | 0 | 0 | 0 | | | | | | |
| 2678 | 03118 | 02677 | 1 | | 0 | 0 | 0 | 0 | 0 | 0 | 0 | 1 | 0 | 0 | 0 | 0 | 0 | 0 | 0 | 0 | 0 | 0 | 0 | 0 | 0 | 0 | 0 | 0 | 0 | | | | | | |
| 2679 | 03119 | 02678 | 5 | | 0 | 0 | 0 | 0 | 0 | 0 | 0 | 1 | 0 | 0 | 0 | 0 | 0 | 0 | 1 | 0 | 0 | 0 | 1 | 0 | 1 | 0 | 0 | 1 | 0 | | | | | | |
| 2680 | 03120 | 02679 | 1 | | 0 | 0 | 0 | 0 | 0 | 0 | 0 | 1 | 0 | 0 | 0 | 0 | 0 | 0 | 0 | 0 | 0 | 0 | 0 | 0 | 0 | 0 | 0 | 0 | 0 | | | | | | |
| 2681 | 03121 | 02680 | 6 | | 0 | 0 | 0 | 0 | 0 | 0 | 0 | 1 | 0 | 0 | 2 | 0 | 0 | 0 | 0 | 0 | 1 | 1 | 0 | 0 | 0 | 1 | 0 | 0 | 0 | | | | | | |
| 2682 | 03122 | 02681 | 5 | | 0 | 0 | 0 | 0 | 0 | 0 | 0 | 1 | 0 | 0 | 1 | 0 | 0 | 0 | 0 | 1 | 1 | 0 | 1 | 0 | 0 | 0 | 0 | 0 | 0 | J03544 | 96.6 | 236 | 1 | 3586 | 3816 |
| 2683 | 03123 | 02682 | 1 | | 0 | 0 | 0 | 0 | 0 | 0 | 0 | 1 | 0 | 0 | 0 | 0 | 0 | 0 | 0 | 0 | 0 | 0 | 0 | 0 | 0 | 0 | 0 | 0 | 0 | | | | | | |
| 2684 | 03124 | 02683 | 2 | | 0 | 0 | 0 | 0 | 0 | 0 | 0 | 1 | 0 | 0 | 0 | 0 | 1 | 0 | 0 | 0 | 0 | 0 | 0 | 0 | 0 | 0 | 0 | 0 | 0 | | | | | | |
| 2685 | 03125 | 02684 | 1 | | 0 | 0 | 0 | 0 | 0 | 0 | 0 | 1 | 0 | 0 | 0 | 0 | 0 | 0 | 0 | 0 | 0 | 0 | 0 | 0 | 0 | 0 | 0 | 0 | 0 | | | | | | |

Table 75

| | A | B | C | E | G | I | K | M | O | Q | S | U | W | Y | AA | AC | AF | AG | AI | AK | AM | AO | AQ | AS | AU | AW | AY | BA | BC | BE | BF | BG | BH | BI | BK |
|---|---|---|---|---|---|---|---|---|---|---|---|---|---|---|---|---|---|---|---|---|---|---|---|---|---|---|---|---|---|---|---|---|---|---|---|
| 2686 | 03126 | 02685 | 2 | | 0 | 0 | 0 | 0 | 0 | 0 | 0 | 1 | 0 | 0 | 0 | 0 | 0 | 0 | 0 | 0 | 0 | 0 | 0 | 0 | 0 | 0 | 0 | 1 | 0 | | | | | | |
| 2687 | 03127 | 02686 | 1 | | 0 | 0 | 0 | 0 | 0 | 0 | 0 | 1 | 0 | 0 | 0 | 0 | 0 | 0 | 0 | 0 | 0 | 0 | 0 | 0 | 0 | 0 | 0 | 0 | 0 | | | | | | |
| 2688 | 03128 | 02687 | 19 | | 0 | 0 | 0 | 0 | 0 | 0 | 0 | 2 | 0 | 2 | 0 | 2 | 1 | 0 | 0 | 1 | 4 | 1 | 0 | 0 | 0 | 6 | 0 | 0 | 0 | X15480 | 96 | 224 | 1 | 500 | 725 |
| 2689 | 03129 | 02688 | 1 | | 0 | 0 | 0 | 0 | 0 | 0 | 0 | 1 | 0 | 0 | 0 | 0 | 0 | 0 | 0 | 0 | 0 | 0 | 0 | 0 | 0 | 0 | 0 | 0 | 0 | | | | | | |
| 2690 | 03131 | 02689 | 1 | | 0 | 0 | 0 | 0 | 0 | 0 | 0 | 1 | 0 | 0 | 0 | 0 | 0 | 0 | 0 | 0 | 0 | 0 | 0 | 0 | 0 | 0 | 0 | 0 | 0 | | | | | | |
| 2691 | 03132 | 02690 | 1 | | 0 | 0 | 0 | 0 | 0 | 0 | 0 | 1 | 0 | 0 | 0 | 0 | 0 | 0 | 0 | 0 | 0 | 0 | 0 | 0 | 0 | 0 | 0 | 0 | 0 | | | | | | |
| 2692 | 03133 | 02691 | 1 | | 0 | 0 | 0 | 0 | 0 | 0 | 0 | 1 | 0 | 0 | 0 | 0 | 0 | 0 | 0 | 0 | 0 | 0 | 0 | 0 | 0 | 0 | 0 | 0 | 0 | | | | | | |
| 2693 | 03134 | 02692 | 1 | | 0 | 0 | 0 | 0 | 0 | 0 | 0 | 1 | 0 | 0 | 0 | 0 | 0 | 0 | 0 | 0 | 0 | 0 | 0 | 0 | 0 | 0 | 0 | 0 | 0 | | | | | | |
| 2694 | 03135 | 02693 | 2 | | 0 | 0 | 0 | 0 | 0 | 0 | 0 | 1 | 0 | 0 | 0 | 0 | 0 | 0 | 0 | 0 | 0 | 0 | 1 | 0 | 0 | 0 | 0 | 0 | 0 | | | | | | |
| 2695 | 03136 | 02694 | 1 | | 0 | 0 | 0 | 0 | 0 | 0 | 0 | 1 | 0 | 0 | 0 | 0 | 0 | 0 | 0 | 0 | 0 | 0 | 0 | 0 | 0 | 0 | 0 | 0 | 0 | | | | | | |
| 2696 | 03138 | 02695 | 3 | | 0 | 0 | 0 | 0 | 0 | 0 | 0 | 1 | 0 | 0 | 0 | 0 | 0 | 0 | 0 | 0 | 0 | 0 | 0 | 0 | 2 | 0 | 0 | 0 | 0 | X74295 | 98.5 | 205 | 1 | 507 | 719 |
| 2697 | 03139 | 02696 | 1 | | 0 | 0 | 0 | 0 | 0 | 0 | 0 | 1 | 0 | 0 | 0 | 0 | 0 | 0 | 0 | 0 | 0 | 0 | 0 | 0 | 0 | 0 | 0 | 0 | 0 | | | | | | |
| 2698 | 03140 | 02697 | 2 | | 0 | 0 | 0 | 0 | 0 | 0 | 0 | 1 | 0 | 0 | 0 | 0 | 0 | 0 | 1 | 0 | 0 | 0 | 0 | 0 | 0 | 0 | 0 | 0 | 0 | | | | | | |
| 2699 | 03141 | 02698 | 1 | | 0 | 0 | 0 | 0 | 0 | 0 | 0 | 1 | 0 | 0 | 0 | 0 | 0 | 0 | 0 | 0 | 0 | 0 | 0 | 0 | 0 | 0 | 0 | 0 | 0 | | | | | | |
| 2700 | 03142 | 02699 | 9 | | 0 | 0 | 0 | 0 | 0 | 0 | 0 | 1 | 0 | 3 | 0 | 0 | 0 | 0 | 1 | 0 | 0 | 2 | 1 | 1 | 0 | 0 | 0 | 0 | 0 | X13839 | 99.5 | 210 | 1 | 1121 | 1330 |
| 2701 | 03145 | 02700 | 3 | | 0 | 0 | 0 | 0 | 0 | 0 | 0 | 1 | 0 | 0 | 0 | 0 | 0 | 0 | 0 | 0 | 0 | 2 | 0 | 0 | 0 | 0 | 0 | 0 | 0 | | | | | | |
| 2702 | 03146 | 02701 | 1 | | 0 | 0 | 0 | 0 | 0 | 0 | 0 | 1 | 0 | 0 | 0 | 0 | 0 | 0 | 0 | 0 | 0 | 0 | 0 | 0 | 0 | 0 | 0 | 0 | 0 | | | | | | |
| 2703 | 03147 | 02702 | 2 | | 0 | 0 | 0 | 0 | 0 | 0 | 0 | 1 | 0 | 0 | 0 | 0 | 0 | 0 | 0 | 0 | 0 | 0 | 1 | 0 | 0 | 0 | 0 | 0 | 0 | | | | | | |
| 2704 | 03148 | 02703 | 12 | | 0 | 0 | 0 | 0 | 0 | 0 | 0 | 1 | 0 | 0 | 0 | 1 | 1 | 2 | 0 | 0 | 0 | 0 | 0 | 3 | 3 | 0 | 0 | 1 | 0 | X51405 | 99.5 | 205 | 1 | 2216 | 2443 |
| 2705 | 03149 | 02704 | 1 | | 0 | 0 | 0 | 0 | 0 | 0 | 0 | 1 | 0 | 0 | 0 | 0 | 0 | 0 | 0 | 0 | 0 | 0 | 0 | 0 | 0 | 0 | 0 | 0 | 0 | | | | | | |
| 2706 | 03150 | 02705 | 1 | | 0 | 0 | 0 | 0 | 0 | 0 | 0 | 1 | 0 | 0 | 0 | 0 | 0 | 0 | 0 | 0 | 0 | 0 | 0 | 0 | 0 | 0 | 0 | 0 | 0 | | | | | | |
| 2707 | 03151 | 02706 | 1 | | 0 | 0 | 0 | 0 | 0 | 0 | 0 | 1 | 0 | 0 | 0 | 0 | 0 | 0 | 0 | 0 | 0 | 0 | 0 | 0 | 0 | 0 | 0 | 0 | 0 | Z11793 | 93.9 | 197 | 1 | 154 | 2038 |
| 2708 | 03152 | 02707 | 8 | | 0 | 0 | 0 | 0 | 0 | 0 | 0 | 1 | 1 | 1 | 0 | 0 | 0 | 0 | 0 | 0 | 1 | 1 | 0 | 1 | 0 | 0 | 0 | 1 | 1 | | | | | | |
| 2709 | 03153 | 02708 | 1 | | 0 | 0 | 0 | 0 | 0 | 0 | 0 | 1 | 0 | 0 | 0 | 0 | 0 | 0 | 0 | 0 | 0 | 0 | 0 | 0 | 0 | 0 | 0 | 0 | 0 | | | | | | |
| 2710 | 03154 | 02709 | 1 | | 0 | 0 | 0 | 0 | 0 | 0 | 0 | 1 | 0 | 0 | 0 | 0 | 0 | 0 | 0 | 0 | 0 | 0 | 0 | 0 | 0 | 0 | 0 | 0 | 0 | | | | | | |
| 2711 | 03155 | 02710 | 1 | | 0 | 0 | 0 | 0 | 0 | 0 | 0 | 1 | 0 | 0 | 0 | 0 | 0 | 0 | 0 | 0 | 0 | 0 | 0 | 0 | 0 | 0 | 0 | 0 | 0 | | | | | | |
| 2712 | 03156 | 02711 | 15 | | 0 | 0 | 0 | 0 | 0 | 0 | 0 | 2 | 0 | 1 | 0 | 0 | 0 | 0 | 2 | 1 | 0 | 6 | 0 | 1 | 2 | 0 | 0 | 0 | 0 | | | | | | |
| 2713 | 03157 | 02712 | 2 | | 0 | 0 | 0 | 0 | 0 | 0 | 0 | 1 | 0 | 0 | 0 | 0 | 0 | 0 | 0 | 0 | 0 | 0 | 0 | 1 | 0 | 0 | 0 | 0 | 0 | | | | | | |
| 2714 | 03159 | 02713 | 3 | | 0 | 0 | 0 | 0 | 0 | 0 | 0 | 2 | 0 | 0 | 0 | 0 | 0 | 0 | 0 | 0 | 0 | 1 | 0 | 0 | 0 | 0 | 0 | 0 | 0 | | | | | | |
| 2715 | 03160 | 02714 | 1 | | 0 | 0 | 0 | 0 | 0 | 0 | 0 | 1 | 0 | 0 | 0 | 0 | 0 | 0 | 0 | 0 | 0 | 0 | 0 | 0 | 0 | 0 | 0 | 0 | 0 | | | | | | |
| 2716 | 03162 | 02715 | 4 | | 0 | 0 | 0 | 0 | 0 | 0 | 0 | 3 | 0 | 0 | 0 | 0 | 0 | 0 | 0 | 1 | 0 | 0 | 0 | 0 | 0 | 0 | 0 | 0 | 0 | | | | | | |
| 2717 | 03163 | 02716 | 2 | | 0 | 0 | 0 | 0 | 0 | 0 | 0 | 1 | 0 | 0 | 0 | 0 | 0 | 0 | 0 | 0 | 0 | 0 | 0 | 0 | 1 | 0 | 0 | 0 | 0 | | | | | | |
| 2718 | 03164 | 02717 | 2 | | 0 | 0 | 0 | 0 | 0 | 0 | 0 | 1 | 0 | 0 | 0 | 0 | 0 | 0 | 0 | 0 | 0 | 0 | 0 | 0 | 0 | 0 | 0 | 1 | 0 | | | | | | |
| 2719 | 03166 | 02718 | 1 | | 0 | 0 | 0 | 0 | 0 | 0 | 0 | 1 | 0 | 0 | 0 | 0 | 0 | 0 | 0 | 0 | 0 | 0 | 0 | 0 | 0 | 0 | 0 | 0 | 0 | | | | | | |
| 2720 | 03167 | 02719 | 1 | | 0 | 0 | 0 | 0 | 0 | 0 | 0 | 1 | 0 | 0 | 0 | 0 | 0 | 0 | 0 | 0 | 0 | 0 | 0 | 0 | 0 | 0 | 0 | 0 | 0 | | | | | | |
| 2721 | 03168 | 02720 | 1 | | 0 | 0 | 0 | 0 | 0 | 0 | 0 | 1 | 0 | 0 | 0 | 0 | 0 | 0 | 0 | 0 | 0 | 0 | 0 | 0 | 0 | 0 | 0 | 0 | 0 | | | | | | |

Table 76

| | A | B | C | E | G | I | K | M | O | Q | S | U | W | Y | AA | AC | AE | AG | AI | AK | AM | AO | AQ | AS | AU | AW | AY | BA | BC | BE | BF | BG | BH | BI | BK |
|---|---|---|---|---|---|---|---|---|---|---|---|---|---|---|---|---|---|---|---|---|---|---|---|---|---|---|---|---|---|---|---|---|---|---|---|
| 2722 | 03169 | 02721 | 2 | | 0 | 0 | 0 | 0 | 0 | 0 | 0 | 1 | 0 | 0 | 0 | 0 | 0 | 0 | 0 | 0 | 0 | 0 | 0 | 1 | 0 | 0 | 0 | 0 | 0 | | | | | | |
| 2723 | 03170 | 02722 | 2 | | 0 | 0 | 0 | 0 | 0 | 0 | 0 | 1 | 0 | 0 | 0 | 0 | 0 | 0 | 0 | 0 | 0 | 0 | 1 | 0 | 0 | 0 | 0 | 0 | 0 | | | | | | |
| 2724 | 03171 | 02723 | 3 | | 0 | 0 | 0 | 0 | 0 | 0 | 0 | 1 | 0 | 0 | 0 | 0 | 0 | 0 | 0 | 0 | 0 | 0 | 0 | 0 | 0 | 0 | 2 | 0 | 0 | | | | | | |
| 2725 | 03172 | 02724 | 3 | | 0 | 0 | 0 | 0 | 0 | 0 | 0 | 1 | 0 | 0 | 0 | 0 | 0 | 0 | 0 | 0 | 0 | 0 | 2 | 0 | 0 | 0 | 0 | 0 | 0 | L22214 | 92.5 | 174 | 1 | 2728 | 2900 |
| 2726 | 03173 | 02725 | 4 | | 0 | 0 | 0 | 0 | 0 | 0 | 0 | 1 | 0 | 0 | 0 | 0 | 0 | 0 | 0 | 1 | 0 | 0 | 0 | 0 | 1 | 1 | 0 | 0 | 0 | | | | | | |
| 2727 | 03175 | 02726 | 2 | | 0 | 0 | 0 | 0 | 0 | 0 | 0 | 1 | 0 | 0 | 0 | 0 | 1 | 0 | 0 | 0 | 0 | 0 | 0 | 0 | 0 | 0 | 0 | 0 | 0 | | | | | | |
| 2728 | 03176 | 02727 | 1 | | 0 | 0 | 0 | 0 | 0 | 0 | 0 | 1 | 0 | 0 | 0 | 0 | 0 | 0 | 0 | 0 | 0 | 0 | 0 | 0 | 0 | 0 | 0 | 0 | 0 | | | | | | |
| 2729 | 03177 | 02728 | 1 | | 0 | 0 | 0 | 0 | 0 | 0 | 0 | 1 | 0 | 0 | 0 | 0 | 0 | 0 | 0 | 0 | 0 | 0 | 0 | 0 | 0 | 0 | 0 | 0 | 0 | | | | | | |
| 2730 | 03178 | 02729 | 1 | | 0 | 0 | 0 | 0 | 0 | 0 | 0 | 1 | 0 | 0 | 0 | 0 | 0 | 0 | 0 | 0 | 0 | 0 | 0 | 0 | 0 | 0 | 0 | 0 | 0 | | | | | | |
| 2731 | 03179 | 02730 | 2 | | 0 | 0 | 0 | 0 | 0 | 0 | 0 | 1 | 0 | 0 | 0 | 0 | 0 | 0 | 0 | 0 | 0 | 0 | 0 | 0 | 0 | 1 | 0 | 0 | 0 | | | | | | |
| 2732 | 03180 | 02731 | 2 | | 0 | 0 | 0 | 0 | 0 | 0 | 0 | 1 | 0 | 0 | 0 | 0 | 0 | 0 | 0 | 1 | 0 | 0 | 0 | 0 | 0 | 0 | 0 | 0 | 0 | | | | | | |
| 2733 | 03181 | 02732 | 1 | | 0 | 0 | 0 | 0 | 0 | 0 | 0 | 1 | 0 | 0 | 0 | 0 | 0 | 0 | 0 | 0 | 0 | 0 | 0 | 0 | 0 | 0 | 0 | 0 | 0 | | | | | | |
| 2734 | 03182 | 02733 | 2 | | 0 | 0 | 0 | 0 | 0 | 0 | 0 | 2 | 0 | 0 | 0 | 0 | 0 | 0 | 0 | 0 | 0 | 0 | 0 | 0 | 0 | 0 | 0 | 0 | 0 | | | | | | |
| 2735 | 03183 | 02734 | 5 | | 0 | 0 | 0 | 0 | 0 | 0 | 0 | 1 | 0 | 0 | 0 | 0 | 0 | 0 | 0 | 0 | 0 | 0 | 1 | 1 | 1 | 0 | 0 | 1 | 0 | | | | | | |
| 2736 | 03185 | 02735 | 1 | | 0 | 0 | 0 | 0 | 0 | 0 | 0 | 1 | 0 | 0 | 0 | 0 | 0 | 0 | 0 | 0 | 0 | 0 | 0 | 0 | 0 | 0 | 0 | 0 | 0 | | | | | | |
| 2737 | 03186 | 02736 | 1 | | 0 | 0 | 0 | 0 | 0 | 0 | 0 | 1 | 0 | 0 | 0 | 0 | 0 | 0 | 0 | 0 | 0 | 0 | 0 | 0 | 0 | 0 | 0 | 0 | 0 | | | | | | |
| 2738 | 03187 | 02737 | 1 | | 0 | 0 | 0 | 0 | 0 | 0 | 0 | 1 | 0 | 0 | 0 | 0 | 0 | 0 | 0 | 0 | 0 | 0 | 0 | 0 | 0 | 0 | 0 | 0 | 0 | | | | | | |
| 2739 | 03188 | 02738 | 2 | | 0 | 0 | 0 | 0 | 0 | 0 | 0 | 1 | 0 | 0 | 0 | 0 | 0 | 0 | 1 | 0 | 0 | 0 | 0 | 0 | 0 | 0 | 0 | 0 | 0 | | | | | | |
| 2740 | 03189 | 02739 | 1 | | 0 | 0 | 0 | 0 | 0 | 0 | 0 | 1 | 0 | 0 | 0 | 0 | 0 | 0 | 0 | 0 | 0 | 0 | 0 | 0 | 0 | 0 | 0 | 0 | 0 | | | | | | |
| 2741 | 03190 | 02740 | 3 | | 0 | 0 | 0 | 0 | 0 | 0 | 0 | 2 | 0 | 0 | 0 | 0 | 0 | 0 | 0 | 0 | 1 | 0 | 0 | 0 | 0 | 0 | 0 | 0 | 0 | | | | | | |
| 2742 | 03192 | 02741 | 1 | | 0 | 0 | 0 | 0 | 0 | 0 | 0 | 1 | 0 | 0 | 0 | 0 | 0 | 0 | 0 | 0 | 0 | 0 | 0 | 0 | 0 | 0 | 0 | 0 | 0 | M98833 | 97.8 | 137 | 1 | 2703 | 2954 |
| 2743 | 03193 | 02742 | 1 | | 0 | 0 | 0 | 0 | 0 | 0 | 0 | 1 | 0 | 0 | 0 | 0 | 0 | 0 | 0 | 0 | 0 | 0 | 0 | 0 | 0 | 0 | 0 | 0 | 0 | | | | | | |
| 2744 | 03194 | 02743 | 1 | | 0 | 0 | 0 | 0 | 0 | 0 | 0 | 1 | 0 | 0 | 0 | 0 | 0 | 0 | 0 | 0 | 0 | 0 | 0 | 0 | 0 | 0 | 0 | 0 | 0 | | | | | | |
| 2745 | 03195 | 02744 | 2 | | 0 | 0 | 0 | 0 | 0 | 0 | 0 | 1 | 0 | 0 | 0 | 0 | 0 | 0 | 0 | 0 | 0 | 0 | 0 | 0 | 0 | 0 | 0 | 0 | 1 | | | | | | |
| 2746 | 03196 | 02745 | 8 | | 0 | 0 | 0 | 0 | 0 | 0 | 0 | 1 | 0 | 1 | 0 | 0 | 0 | 1 | 0 | 0 | 1 | 1 | 1 | 0 | 1 | 0 | 0 | 1 | 0 | | | | | | |
| 2747 | 03197 | 02746 | 1 | | 0 | 0 | 0 | 0 | 0 | 0 | 0 | 1 | 0 | 0 | 0 | 0 | 0 | 0 | 0 | 0 | 0 | 0 | 0 | 0 | 0 | 0 | 0 | 0 | 0 | | | | | | |
| 2748 | 03198 | 02747 | 2 | | 0 | 0 | 0 | 0 | 0 | 0 | 0 | 1 | 0 | 0 | 0 | 1 | 0 | 0 | 0 | 0 | 0 | 0 | 0 | 0 | 0 | 0 | 0 | 0 | 0 | | | | | | |
| 2749 | 03200 | 02748 | 52 | | 0 | 0 | 0 | 0 | 0 | 0 | 0 | 4 | 4 | 20 | 0 | 0 | 0 | 0 | 9 | 2 | 0 | 7 | 1 | 1 | 3 | 1 | 0 | 0 | 0 | J03040 | 97.9 | 142 | 1 | 1968 | 2133 |
| 2750 | 03201 | 02749 | 1 | | 0 | 0 | 0 | 0 | 0 | 0 | 0 | 1 | 0 | 0 | 0 | 0 | 0 | 0 | 0 | 0 | 0 | 0 | 0 | 0 | 0 | 0 | 0 | 0 | 0 | | | | | | |
| 2751 | 03202 | 02750 | 2 | | 0 | 0 | 0 | 0 | 0 | 0 | 0 | 2 | 0 | 0 | 0 | 0 | 0 | 0 | 0 | 0 | 0 | 0 | 0 | 0 | 0 | 0 | 0 | 0 | 0 | | | | | | |
| 2752 | 03204 | 02751 | 1 | | 0 | 0 | 0 | 0 | 0 | 0 | 0 | 1 | 0 | 0 | 0 | 0 | 0 | 0 | 0 | 0 | 0 | 0 | 0 | 0 | 0 | 0 | 0 | 0 | 0 | | | | | | |
| 2753 | 03205 | 02752 | 1 | | 0 | 0 | 0 | 0 | 0 | 0 | 0 | 1 | 0 | 0 | 0 | 0 | 0 | 0 | 0 | 0 | 0 | 0 | 0 | 0 | 0 | 0 | 0 | 0 | 0 | | | | | | |
| 2754 | 03206 | 02753 | 1 | | 0 | 0 | 0 | 0 | 0 | 0 | 0 | 1 | 0 | 0 | 0 | 0 | 0 | 0 | 0 | 0 | 0 | 0 | 0 | 0 | 0 | 0 | 0 | 0 | 0 | | | | | | |
| 2755 | 03207 | 02754 | 9 | | 0 | 0 | 0 | 0 | 0 | 0 | 0 | 1 | 0 | 0 | 0 | 2 | 0 | 0 | 1 | 0 | 1 | 0 | 0 | 0 | 3 | 1 | 0 | 0 | 0 | | | | | | |
| 2756 | 03208 | 02755 | 1 | | 0 | 0 | 0 | 0 | 0 | 0 | 0 | 1 | 0 | 0 | 0 | 0 | 0 | 0 | 0 | 0 | 0 | 0 | 0 | 0 | 0 | 0 | 0 | 0 | 0 | | | | | | |
| 2757 | 03212 | 02756 | 1 | | 0 | 0 | 0 | 0 | 0 | 0 | 0 | 1 | 0 | 0 | 0 | 0 | 0 | 0 | 0 | 0 | 0 | 0 | 0 | 0 | 0 | 0 | 0 | 0 | 0 | | | | | | |

Table 77

| | A | B | C | E | G | I | K | M | O | Q | S | U | W | Y | AA | AC | AE | AG | AI | AK | AM | AO | AQ | AS | AU | AW | AY | BA | BC | BE | BF | BG | BH | BI | BK |
|---|---|---|---|---|---|---|---|---|---|---|---|---|---|---|---|---|---|---|---|---|---|---|---|---|---|---|---|---|---|---|---|---|---|---|---|
| 2758 | 03213 | 02757 | 3 | | 0 | 0 | 0 | 0 | 0 | 0 | 0 | 1 | 0 | 0 | 1 | 0 | 0 | 0 | 0 | 0 | 0 | 0 | 0 | 0 | 1 | 0 | 0 | 0 | 0 | | | | | | |
| 2759 | 03214 | 02758 | 1 | | 0 | 0 | 0 | 0 | 0 | 0 | 0 | 1 | 0 | 0 | 0 | 0 | 0 | 0 | 0 | 0 | 0 | 0 | 0 | 0 | 0 | 0 | 0 | 0 | 0 | | | | | | |
| 2760 | 03215 | 02759 | 1 | | 0 | 0 | 0 | 0 | 0 | 0 | 0 | 1 | 0 | 0 | 0 | 0 | 0 | 0 | 0 | 0 | 0 | 0 | 0 | 0 | 0 | 0 | 0 | 0 | 0 | | | | | | |
| 2761 | 03216 | 02760 | 4 | | 0 | 0 | 0 | 0 | 0 | 0 | 0 | 1 | 0 | 0 | 0 | 0 | 0 | 0 | 0 | 0 | 0 | 0 | 0 | 0 | 0 | 0 | 3 | 0 | 0 | D10040 | 94.3 | 406 | 1 | 3055 | 3634 |
| 2762 | 03217 | 02761 | 1 | | 0 | 0 | 0 | 0 | 0 | 0 | 0 | 1 | 0 | 0 | 0 | 0 | 0 | 0 | 0 | 0 | 0 | 0 | 0 | 0 | 0 | 0 | 0 | 0 | 0 | | | | | | |
| 2763 | 03218 | 02762 | 2 | | 0 | 0 | 0 | 0 | 0 | 0 | 0 | 1 | 0 | 0 | 0 | 0 | 0 | 0 | 0 | 0 | 0 | 0 | 0 | 1 | 0 | 0 | 0 | 0 | 0 | | | | | | |
| 2764 | 03219 | 02763 | 3 | | 0 | 0 | 0 | 0 | 0 | 0 | 0 | 1 | 0 | 0 | 1 | 0 | 1 | 0 | 0 | 0 | 0 | 0 | 0 | 0 | 0 | 0 | 0 | 0 | 0 | | | | | | |
| 2765 | 03220 | 02764 | 2 | | 0 | 0 | 0 | 0 | 0 | 0 | 0 | 1 | 0 | 0 | 0 | 0 | 0 | 0 | 0 | 1 | 0 | 0 | 0 | 0 | 0 | 0 | 0 | 0 | 0 | | | | | | |
| 2766 | 03221 | 02765 | 2 | | 0 | 0 | 0 | 0 | 0 | 0 | 0 | 1 | 0 | 0 | 0 | 0 | 0 | 0 | 0 | 0 | 0 | 0 | 0 | 0 | 1 | 0 | 0 | 0 | 0 | | | | | | |
| 2767 | 03222 | 02766 | 1 | | 0 | 0 | 0 | 0 | 0 | 0 | 0 | 1 | 0 | 0 | 0 | 0 | 0 | 0 | 0 | 0 | 0 | 0 | 0 | 0 | 0 | 0 | 0 | 0 | 0 | | | | | | |
| 2768 | 03223 | 02767 | 1 | | 0 | 0 | 0 | 0 | 0 | 0 | 0 | 1 | 0 | 0 | 0 | 0 | 0 | 0 | 0 | 0 | 0 | 0 | 0 | 0 | 0 | 0 | 0 | 0 | 0 | S53910 | 98.4 | 124 | 1 | 2325 | 2463 |
| 2769 | 03224 | 02768 | 4 | | 0 | 0 | 0 | 0 | 0 | 0 | 0 | 1 | 0 | 1 | 0 | 0 | 0 | 0 | 1 | 0 | 0 | 0 | 0 | 0 | 1 | 0 | 0 | 0 | 0 | | | | | | |
| 2770 | 03225 | 02769 | 1 | | 0 | 0 | 0 | 0 | 0 | 0 | 0 | 1 | 0 | 0 | 0 | 0 | 0 | 0 | 0 | 0 | 0 | 0 | 0 | 0 | 0 | 0 | 0 | 0 | 0 | | | | | | |
| 2771 | 03227 | 02770 | 2 | | 0 | 0 | 0 | 0 | 0 | 0 | 0 | 1 | 0 | 0 | 1 | 0 | 0 | 0 | 0 | 0 | 0 | 0 | 0 | 0 | 0 | 0 | 0 | 0 | 0 | M13519 | 96.1 | 387 | 1 | 1250 | 1832 |
| 2772 | 03229 | 02771 | 2 | | 0 | 0 | 0 | 0 | 0 | 0 | 0 | 1 | 0 | 0 | 0 | 0 | 0 | 0 | 0 | 0 | 0 | 0 | 0 | 0 | 0 | 0 | 1 | 0 | 0 | | | | | | |
| 2773 | 03230 | 02772 | 1 | | 0 | 0 | 0 | 0 | 0 | 0 | 0 | 1 | 0 | 0 | 0 | 0 | 0 | 0 | 0 | 0 | 0 | 0 | 0 | 0 | 0 | 0 | 0 | 0 | 0 | | | | | | |
| 2774 | 03231 | 02773 | 1 | | 0 | 0 | 0 | 0 | 0 | 0 | 0 | 1 | 0 | 0 | 0 | 0 | 0 | 0 | 0 | 0 | 0 | 0 | 0 | 0 | 0 | 0 | 0 | 0 | 0 | | | | | | |
| 2775 | 03233 | 02774 | 2 | | 0 | 0 | 0 | 0 | 0 | 0 | 0 | 2 | 0 | 0 | 0 | 0 | 0 | 0 | 0 | 0 | 0 | 0 | 0 | 0 | 0 | 0 | 0 | 0 | 0 | | | | | | |
| 2776 | 03234 | 02775 | 2 | | 0 | 0 | 0 | 0 | 0 | 0 | 0 | 1 | 0 | 0 | 0 | 0 | 0 | 0 | 0 | 0 | 0 | 0 | 0 | 0 | 0 | 0 | 0 | 1 | 0 | | | | | | |
| 2777 | 03235 | 02776 | 1 | | 0 | 0 | 0 | 0 | 0 | 0 | 0 | 1 | 0 | 0 | 0 | 0 | 0 | 0 | 0 | 0 | 0 | 0 | 0 | 0 | 0 | 0 | 0 | 0 | 0 | | | | | | |
| 2778 | 03236 | 02777 | 2 | | 0 | 0 | 0 | 0 | 0 | 0 | 0 | 1 | 1 | 0 | 0 | 0 | 0 | 0 | 0 | 0 | 0 | 0 | 0 | 0 | 0 | 0 | 0 | 0 | 0 | | | | | | |
| 2779 | 03237 | 02778 | 1 | | 0 | 0 | 0 | 0 | 0 | 0 | 0 | 1 | 0 | 0 | 0 | 0 | 0 | 0 | 0 | 0 | 0 | 0 | 0 | 0 | 0 | 0 | 0 | 0 | 0 | | | | | | |
| 2780 | 03239 | 02779 | 1 | | 0 | 0 | 0 | 0 | 0 | 0 | 0 | 1 | 0 | 0 | 0 | 0 | 0 | 0 | 0 | 0 | 0 | 0 | 0 | 0 | 0 | 0 | 0 | 0 | 0 | | | | | | |
| 2781 | 03240 | 02780 | 4 | | 0 | 0 | 0 | 0 | 0 | 0 | 0 | 1 | 0 | 0 | 0 | 0 | 0 | 0 | 0 | 3 | 0 | 0 | 0 | 0 | 0 | 0 | 0 | 0 | 0 | | | | | | |
| 2782 | 03241 | 02781 | 2 | | 0 | 0 | 0 | 0 | 0 | 0 | 0 | 1 | 0 | 0 | 1 | 0 | 0 | 0 | 0 | 0 | 0 | 0 | 0 | 0 | 0 | 0 | 0 | 0 | 0 | | | | | | |
| 2783 | 03242 | 02782 | 2 | | 0 | 0 | 0 | 0 | 0 | 0 | 0 | 1 | 0 | 0 | 0 | 0 | 1 | 0 | 0 | 0 | 0 | 0 | 0 | 0 | 0 | 0 | 0 | 0 | 0 | | | | | | |
| 2784 | 03243 | 02783 | 2 | | 0 | 0 | 0 | 0 | 0 | 0 | 0 | 2 | 0 | 0 | 0 | 0 | 0 | 0 | 0 | 0 | 0 | 0 | 0 | 0 | 0 | 0 | 0 | 0 | 0 | | | | | | |
| 2785 | 03244 | 02784 | 3 | | 0 | 0 | 0 | 0 | 0 | 0 | 0 | 1 | 0 | 0 | 0 | 0 | 0 | 1 | 0 | 0 | 0 | 0 | 0 | 0 | 0 | 0 | 1 | 0 | 0 | | | | | | |
| 2786 | 03245 | 02785 | 1 | | 0 | 0 | 0 | 0 | 0 | 0 | 0 | 1 | 0 | 0 | 0 | 0 | 0 | 0 | 0 | 0 | 0 | 0 | 0 | 0 | 0 | 0 | 0 | 0 | 0 | | | | | | |
| 2787 | 03247 | 02786 | 2 | | 0 | 0 | 0 | 0 | 0 | 0 | 0 | 1 | 0 | 0 | 0 | 0 | 0 | 0 | 0 | 0 | 0 | 1 | 0 | 0 | 0 | 0 | 0 | 0 | 0 | | | | | | |
| 2788 | 03248 | 02787 | 1 | | 0 | 0 | 0 | 0 | 0 | 0 | 0 | 1 | 0 | 0 | 0 | 0 | 0 | 0 | 0 | 0 | 0 | 0 | 0 | 0 | 0 | 0 | 0 | 0 | 0 | | | | | | |
| 2789 | 03249 | 02788 | 6 | | 0 | 0 | 0 | 0 | 0 | 0 | 0 | 1 | 0 | 0 | 0 | 0 | 0 | 0 | 1 | 0 | 0 | 2 | 0 | 0 | 0 | 0 | 0 | 2 | 0 | | | | | | |
| 2790 | 03251 | 02789 | 2 | | 0 | 0 | 0 | 0 | 0 | 0 | 0 | 1 | 0 | 0 | 0 | 0 | 0 | 0 | 0 | 0 | 1 | 0 | 0 | 0 | 0 | 0 | 0 | 0 | 0 | | | | | | |
| 2791 | 03252 | 02790 | 1 | | 0 | 0 | 0 | 0 | 0 | 0 | 0 | 1 | 0 | 0 | 0 | 0 | 0 | 0 | 0 | 0 | 0 | 0 | 0 | 0 | 0 | 0 | 0 | 0 | 0 | | | | | | |
| 2792 | 03253 | 02791 | 1 | | 0 | 0 | 0 | 0 | 0 | 0 | 0 | 1 | 0 | 0 | 0 | 0 | 0 | 0 | 0 | 0 | 0 | 0 | 0 | 0 | 0 | 0 | 0 | 0 | 0 | | | | | | |
| 2793 | 03255 | 02792 | 5 | | 0 | 0 | 0 | 0 | 0 | 0 | 0 | 1 | 0 | 0 | 0 | 0 | 0 | 2 | 0 | 0 | 0 | 0 | 2 | 0 | 0 | 0 | 0 | 0 | 0 | | | | | | |

Table 78

| | A | B | C | E | G | I | K | M | O | Q | S | U | W | Y | AA | AC | AE | AG | AI | AK | AM | AO | AQ | AS | AU | AW | AY | BA | BC | BE | BF | BG | BH | BI | BK |
|---|---|---|---|---|---|---|---|---|---|---|---|---|---|---|---|---|---|---|---|---|---|---|---|---|---|---|---|---|---|---|---|---|---|---|---|
| 2794 | 03257 | 02793 | 1 | | 0 | 0 | 0 | 0 | 0 | 0 | 0 | 1 | 0 | 0 | 0 | 0 | 0 | 0 | 0 | 0 | 0 | 0 | 0 | 0 | 0 | 0 | 0 | 0 | 0 | | | | | | |
| 2795 | 03258 | 02794 | 2 | | 0 | 0 | 0 | 0 | 0 | 0 | 0 | 1 | 0 | 0 | 0 | 0 | 0 | 1 | 0 | 0 | 0 | 0 | 0 | 0 | 0 | 0 | 0 | 0 | 0 | | | | | | |
| 2796 | 03259 | 02795 | 10 | | 0 | 0 | 0 | 0 | 0 | 0 | 0 | 1 | 0 | 3 | 0 | 0 | 0 | 0 | 2 | 0 | 0 | 0 | 0 | 0 | 2 | 2 | 0 | 0 | 0 | L19182 | 95.9 | 98 | 1 | 1010 | 1115 |
| 2797 | 03261 | 02796 | 2 | | 0 | 0 | 0 | 0 | 0 | 0 | 0 | 1 | 0 | 0 | 0 | 0 | 1 | 0 | 0 | 0 | 0 | 0 | 0 | 0 | 0 | 0 | 0 | 0 | 0 | | | | | | |
| 2798 | 03263 | 02797 | 5 | | 0 | 0 | 0 | 0 | 0 | 0 | 0 | 2 | 0 | 0 | 0 | 0 | 0 | 0 | 0 | 0 | 0 | 0 | 0 | 0 | 3 | 0 | 0 | 0 | 0 | M15856 | 96.8 | 94 | 1 | 3055 | 3549 |
| 2799 | 03264 | 02798 | 1 | | 0 | 0 | 0 | 0 | 0 | 0 | 0 | 1 | 0 | 0 | 0 | 0 | 0 | 0 | 0 | 0 | 0 | 0 | 0 | 0 | 0 | 0 | 0 | 0 | 0 | M98398 | 96.9 | 96 | 1 | 1840 | 1942 |
| 2800 | 03267 | 02799 | 3 | | 0 | 0 | 0 | 0 | 0 | 0 | 0 | 1 | 0 | 0 | 0 | 0 | 0 | 0 | 0 | 0 | 0 | 0 | 0 | 0 | 0 | 1 | 1 | 0 | 0 | | | | | | |
| 2801 | 03268 | 02800 | 5 | | 0 | 0 | 0 | 0 | 0 | 0 | 0 | 3 | 0 | 0 | 0 | 0 | 0 | 1 | 0 | 1 | 0 | 0 | 0 | 0 | 0 | 0 | 0 | 0 | 0 | | | | | | |
| 2802 | 03271 | 02801 | 3 | | 0 | 0 | 0 | 0 | 0 | 0 | 0 | 2 | 0 | 0 | 0 | 0 | 0 | 0 | 0 | 0 | 0 | 0 | 0 | 1 | 0 | 0 | 0 | 0 | 0 | | | | | | |
| 2803 | 03274 | 02802 | 7 | | 0 | 0 | 0 | 0 | 0 | 0 | 0 | 1 | 0 | 0 | 0 | 0 | 0 | 0 | 1 | 0 | 0 | 0 | 0 | 0 | 5 | 0 | 0 | 0 | 0 | M84526 | 100 | 93 | 1 | 720 | 1071 |
| 2804 | 03279 | 02803 | 8 | | 0 | 0 | 0 | 0 | 0 | 0 | 0 | 1 | 0 | 0 | 1 | 2 | 0 | 0 | 0 | 0 | 0 | 0 | 2 | 2 | 0 | 0 | 0 | 0 | 0 | | | | | | |
| 2805 | 03281 | 02804 | 2 | | 0 | 0 | 0 | 0 | 0 | 0 | 0 | 1 | 0 | 0 | 0 | 0 | 0 | 0 | 1 | 0 | 0 | 0 | 0 | 0 | 0 | 0 | 0 | 0 | 0 | D13811 | 94.7 | 76 | 1 | 2028 | 2102 |
| 2806 | 03282 | 02805 | 2 | | 0 | 0 | 0 | 0 | 0 | 0 | 0 | 1 | 0 | 0 | 0 | 0 | 0 | 0 | 0 | 0 | 0 | 1 | 0 | 0 | 0 | 0 | 0 | 0 | 0 | | | | | | |
| 2807 | 03283 | 02806 | 1 | | 0 | 0 | 0 | 0 | 0 | 0 | 0 | 1 | 0 | 0 | 0 | 0 | 0 | 0 | 0 | 0 | 0 | 0 | 0 | 0 | 0 | 0 | 0 | 0 | 0 | | | | | | |
| 2808 | 03284 | 02807 | 2 | | 0 | 0 | 0 | 0 | 0 | 0 | 0 | 2 | 0 | 0 | 0 | 0 | 0 | 0 | 0 | 0 | 0 | 0 | 0 | 0 | 0 | 0 | 0 | 0 | 0 | | | | | | |
| 2809 | 03286 | 02808 | 1 | | 0 | 0 | 0 | 0 | 0 | 0 | 0 | 1 | 0 | 0 | 0 | 0 | 0 | 0 | 0 | 0 | 0 | 0 | 0 | 0 | 0 | 0 | 0 | 0 | 0 | | | | | | |
| 2810 | 03289 | 02809 | 1 | | 0 | 0 | 0 | 0 | 0 | 0 | 0 | 1 | 0 | 0 | 0 | 0 | 0 | 0 | 0 | 0 | 0 | 0 | 0 | 0 | 0 | 0 | 0 | 0 | 0 | L08647 | 100 | 71 | 1 | 432 | 502 |
| 2811 | 03294 | 02810 | 5 | | 0 | 0 | 0 | 0 | 0 | 0 | 0 | 1 | 0 | 0 | 0 | 0 | 0 | 0 | 0 | 0 | 0 | 0 | 3 | 1 | 0 | 0 | 0 | 0 | 0 | | | | | | |
| 2812 | 03296 | 02811 | 1 | | 0 | 0 | 0 | 0 | 0 | 0 | 0 | 1 | 0 | 0 | 0 | 0 | 0 | 0 | 0 | 0 | 0 | 0 | 0 | 0 | 0 | 0 | 0 | 0 | 0 | | | | | | |
| 2813 | 03297 | 02812 | 4 | | 0 | 0 | 0 | 0 | 0 | 0 | 0 | 2 | 0 | 0 | 0 | 0 | 0 | 0 | 0 | 0 | 0 | 0 | 0 | 0 | 2 | 0 | 0 | 0 | 0 | | | | | | |
| 2814 | 03299 | 02813 | 1 | | 0 | 0 | 0 | 0 | 0 | 0 | 0 | 1 | 0 | 0 | 0 | 0 | 0 | 0 | 0 | 0 | 0 | 0 | 0 | 0 | 0 | 0 | 0 | 0 | 0 | X15977 | 96.7 | 60 | 1 | 549 | 608 |
| 2815 | 03300 | 02814 | 1 | | 0 | 0 | 0 | 0 | 0 | 0 | 0 | 1 | 0 | 0 | 0 | 0 | 0 | 0 | 0 | 0 | 0 | 0 | 0 | 0 | 0 | 0 | 0 | 0 | 0 | | | | | | |
| 2816 | 03304 | 02815 | 1 | | 0 | 0 | 0 | 0 | 0 | 0 | 0 | 1 | 0 | 0 | 0 | 0 | 0 | 0 | 0 | 0 | 0 | 0 | 0 | 0 | 0 | 0 | 0 | 0 | 0 | X16940 | 94.7 | 57 | 1 | 1224 | 1279 |
| 2817 | 03305 | 02816 | 15 | | 0 | 0 | 0 | 0 | 0 | 0 | 0 | 1 | 0 | 1 | 0 | 1 | 1 | 2 | 2 | 2 | 0 | 2 | 0 | 0 | 3 | 0 | 0 | 0 | 0 | | | | | | |
| 2818 | 03306 | 02817 | 2 | | 0 | 0 | 0 | 0 | 0 | 0 | 0 | 1 | 0 | 0 | 0 | 0 | 0 | 1 | 0 | 0 | 0 | 0 | 0 | 0 | 0 | 0 | 0 | 0 | 0 | | | | | | |
| 2819 | 03307 | 02818 | 1 | | 0 | 0 | 0 | 0 | 0 | 0 | 0 | 1 | 0 | 0 | 0 | 0 | 0 | 0 | 0 | 0 | 0 | 0 | 0 | 0 | 0 | 0 | 0 | 0 | 0 | | | | | | |
| 2820 | 03308 | 02819 | 2 | | 0 | 0 | 0 | 0 | 0 | 0 | 0 | 2 | 0 | 0 | 0 | 0 | 0 | 0 | 0 | 0 | 0 | 0 | 0 | 0 | 0 | 0 | 0 | 0 | 0 | | | | | | |
| 2821 | 03310 | 02820 | 7 | | 0 | 0 | 0 | 0 | 0 | 0 | 0 | 1 | 1 | 1 | 1 | 0 | 0 | 0 | 0 | 0 | 0 | 0 | 1 | 1 | 0 | 0 | 1 | 0 | 0 | | | | | | |
| 2822 | 03311 | 02821 | 3 | | 0 | 0 | 0 | 0 | 0 | 0 | 0 | 1 | 0 | 0 | 0 | 1 | 0 | 0 | 0 | 1 | 0 | 0 | 0 | 0 | 0 | 0 | 0 | 0 | 0 | | | | | | |
| 2823 | 03312 | 02822 | 4 | | 0 | 0 | 0 | 0 | 0 | 0 | 0 | 2 | 0 | 0 | 0 | 0 | 0 | 0 | 0 | 0 | 0 | 0 | 1 | 0 | 1 | 0 | 0 | 0 | 0 | | | | | | |
| 2824 | 03313 | 02823 | 1 | | 0 | 0 | 0 | 0 | 0 | 0 | 0 | 1 | 0 | 0 | 0 | 0 | 0 | 0 | 0 | 0 | 0 | 0 | 0 | 0 | 0 | 0 | 0 | 0 | 0 | | | | | | |
| 2825 | 03315 | 02824 | 1 | | 0 | 0 | 0 | 0 | 0 | 0 | 0 | 1 | 0 | 0 | 0 | 0 | 0 | 0 | 0 | 0 | 0 | 0 | 0 | 0 | 0 | 0 | 0 | 0 | 0 | | | | | | |
| 2826 | 03318 | 02825 | 1 | | 0 | 0 | 0 | 0 | 0 | 0 | 0 | 1 | 0 | 0 | 0 | 0 | 0 | 0 | 0 | 0 | 0 | 0 | 0 | 0 | 0 | 0 | 0 | 0 | 0 | | | | | | |
| 2827 | 03319 | 02826 | 1 | | 0 | 0 | 0 | 0 | 0 | 0 | 0 | 1 | 0 | 0 | 0 | 0 | 0 | 0 | 0 | 0 | 0 | 0 | 0 | 0 | 0 | 0 | 0 | 0 | 0 | | | | | | |
| 2828 | 03324 | 02827 | 2 | | 0 | 0 | 0 | 0 | 0 | 0 | 0 | 1 | 0 | 0 | 0 | 0 | 0 | 0 | 0 | 0 | 0 | 0 | 0 | 0 | 0 | 0 | 0 | 0 | 1 | | | | | | |
| 2829 | 03325 | 02828 | 1 | | 0 | 0 | 0 | 0 | 0 | 0 | 0 | 1 | 0 | 0 | 0 | 0 | 0 | 0 | 0 | 0 | 0 | 0 | 0 | 0 | 0 | 0 | 0 | 0 | 0 | | | | | | |

Table 79

| | A | B | C | E | G | I | K | M | O | Q | S | U | W | Y | AA | AC | AE | AG | AI | AK | AM | AO | AQ | AS | AU | AW | AY | BA | BC | BE | BF | BG | BH | BI | BK |
|---|---|---|---|---|---|---|---|---|---|---|---|---|---|---|---|---|---|---|---|---|---|---|---|---|---|---|---|---|---|---|---|---|---|---|---|
| 2830 | 03326 | 02829 | 1 | | 0 | 0 | 0 | 0 | 0 | 0 | 0 | 1 | 0 | 0 | 0 | 0 | 0 | 0 | 0 | 0 | 0 | 0 | 0 | 0 | 0 | 0 | 0 | 0 | 0 | | | | | | |
| 2831 | 03331 | 02830 | 24 | | 0 | 0 | 0 | 0 | 0 | 0 | 0 | 1 | 1 | 10 | 1 | 0 | 0 | 0 | 6 | 2 | 0 | 3 | 0 | 0 | 0 | 0 | 0 | 0 | 0 | | | | | | |
| 2832 | 03332 | 02831 | 6 | | 0 | 0 | 0 | 0 | 0 | 0 | 0 | 1 | 0 | 1 | 0 | 0 | 0 | 0 | 0 | 0 | 0 | 1 | 1 | 1 | 0 | 1 | 0 | 0 | 0 | | | | | | |
| 2833 | 03356 | 02832 | 2 | | 0 | 0 | 0 | 0 | 0 | 0 | 0 | 1 | 0 | 0 | 1 | 0 | 0 | 0 | 0 | 0 | 0 | 0 | 0 | 0 | 0 | 0 | 0 | 0 | 0 | M58510 | 100 | 128 | 1 | 3228 | 3355 |
| 2834 | 03374 | 02833 | 1 | | 0 | 0 | 0 | 0 | 0 | 0 | 0 | 0 | 1 | 0 | 0 | 0 | 0 | 0 | 0 | 0 | 0 | 0 | 0 | 0 | 0 | 0 | 0 | 0 | 0 | D14658 | 96.5 | 429 | 1 | 889 | 1370 |
| 2835 | 03375 | 02834 | 1 | | 0 | 0 | 0 | 0 | 0 | 0 | 0 | 0 | 1 | 0 | 0 | 0 | 0 | 0 | 0 | 0 | 0 | 0 | 0 | 0 | 0 | 0 | 0 | 0 | 0 | | | | | | |
| 2836 | 03376 | 02835 | 2 | | 0 | 0 | 0 | 0 | 0 | 0 | 0 | 0 | 2 | 0 | 0 | 0 | 0 | 0 | 0 | 0 | 0 | 0 | 0 | 0 | 0 | 0 | 0 | 0 | 0 | | | | | | |
| 2837 | 03377 | 02836 | 4 | | 0 | 0 | 0 | 0 | 0 | 0 | 0 | 0 | 1 | 0 | 0 | 1 | 0 | 0 | 0 | 0 | 1 | 0 | 0 | 1 | 0 | 0 | 0 | 0 | 0 | | | | | | |
| 2838 | 03378 | 02837 | 1 | | 0 | 0 | 0 | 0 | 0 | 0 | 0 | 0 | 1 | 0 | 0 | 0 | 0 | 0 | 0 | 0 | 0 | 0 | 0 | 0 | 0 | 0 | 0 | 0 | 0 | | | | | | |
| 2839 | 03379 | 02838 | 3 | | 0 | 0 | 0 | 0 | 0 | 0 | 0 | 0 | 1 | 0 | 0 | 1 | 0 | 0 | 0 | 0 | 1 | 0 | 0 | 0 | 0 | 0 | 0 | 0 | 0 | M65028 | 98 | 394 | 1 | 1021 | 1537 |
| 2840 | 03380 | 02839 | 1 | | 0 | 0 | 0 | 0 | 0 | 0 | 0 | 0 | 1 | 0 | 0 | 0 | 0 | 0 | 0 | 0 | 0 | 0 | 0 | 0 | 0 | 0 | 0 | 0 | 0 | M24070 | 96.1 | 386 | 1 | 1063 | 1452 |
| 2841 | 03381 | 02840 | 3 | | 0 | 0 | 0 | 0 | 0 | 0 | 0 | 0 | 2 | 0 | 0 | 0 | 0 | 0 | 0 | 1 | 0 | 0 | 0 | 0 | 0 | 0 | 0 | 0 | 0 | | | | | | |
| 2842 | 03382 | 02841 | 3 | | 0 | 0 | 0 | 0 | 0 | 0 | 0 | 0 | 1 | 0 | 0 | 0 | 1 | 1 | 0 | 0 | 0 | 0 | 0 | 0 | 0 | 0 | 0 | 0 | 0 | | | | | | |
| 2843 | 03383 | 02842 | 1 | | 0 | 0 | 0 | 0 | 0 | 0 | 0 | 0 | 1 | 0 | 0 | 0 | 0 | 0 | 0 | 0 | 0 | 0 | 0 | 0 | 0 | 0 | 0 | 0 | 0 | | | | | | |
| 2844 | 03384 | 02843 | 3 | | 0 | 0 | 0 | 0 | 0 | 0 | 0 | 0 | 1 | 1 | 0 | 0 | 0 | 0 | 0 | 0 | 0 | 0 | 0 | 0 | 0 | 1 | 0 | 0 | 0 | | | | | | |
| 2845 | 03385 | 02844 | 1 | | 0 | 0 | 0 | 0 | 0 | 0 | 0 | 0 | 1 | 0 | 0 | 0 | 0 | 0 | 0 | 0 | 0 | 0 | 0 | 0 | 0 | 0 | 0 | 0 | 0 | | | | | | |
| 2846 | 03386 | 02845 | 2 | | 0 | 0 | 0 | 0 | 0 | 0 | 0 | 0 | 2 | 0 | 0 | 0 | 0 | 0 | 0 | 0 | 0 | 0 | 0 | 0 | 0 | 0 | 0 | 0 | 0 | | | | | | |
| 2847 | 03387 | 02846 | 1 | | 0 | 0 | 0 | 0 | 0 | 0 | 0 | 0 | 1 | 0 | 0 | 0 | 0 | 0 | 0 | 0 | 0 | 0 | 0 | 0 | 0 | 0 | 0 | 0 | 0 | | | | | | |
| 2848 | 03388 | 02847 | 3 | | 0 | 0 | 0 | 0 | 0 | 0 | 0 | 0 | 2 | 0 | 0 | 0 | 0 | 0 | 0 | 0 | 0 | 0 | 1 | 0 | 0 | 0 | 0 | 0 | 0 | | | | | | |
| 2849 | 03389 | 02848 | 1 | | 0 | 0 | 0 | 0 | 0 | 0 | 0 | 0 | 1 | 0 | 0 | 0 | 0 | 0 | 0 | 0 | 0 | 0 | 0 | 0 | 0 | 0 | 0 | 0 | 0 | | | | | | |
| 2850 | 03390 | 02849 | 1 | | 0 | 0 | 0 | 0 | 0 | 0 | 0 | 0 | 1 | 0 | 0 | 0 | 0 | 0 | 0 | 0 | 0 | 0 | 0 | 0 | 0 | 0 | 0 | 0 | 0 | | | | | | |
| 2851 | 03391 | 02850 | 2 | | 0 | 0 | 0 | 0 | 0 | 0 | 0 | 0 | 1 | 0 | 0 | 0 | 0 | 0 | 0 | 0 | 0 | 1 | 0 | 0 | 0 | 0 | 0 | 0 | 0 | M14083 | 93.2 | 325 | 1 | 1295 | 2937 |
| 2852 | 03392 | 02851 | 1 | | 0 | 0 | 0 | 0 | 0 | 0 | 0 | 0 | 1 | 0 | 0 | 0 | 0 | 0 | 0 | 0 | 0 | 0 | 0 | 0 | 0 | 0 | 0 | 0 | 0 | | | | | | |
| 2853 | 03393 | 02852 | 1 | | 0 | 0 | 0 | 0 | 0 | 0 | 0 | 0 | 1 | 0 | 0 | 0 | 0 | 0 | 0 | 0 | 0 | 0 | 0 | 0 | 0 | 0 | 0 | 0 | 0 | X15606 | 96.2 | 339 | 1 | 704 | 1041 |
| 2854 | 03394 | 02853 | 7 | | 0 | 0 | 0 | 0 | 0 | 0 | 0 | 0 | 1 | 1 | 0 | 0 | 0 | 0 | 1 | 0 | 1 | 0 | 0 | 0 | 0 | 0 | 1 | 2 | 0 | | | | | | |
| 2855 | 03395 | 02854 | 3 | | 0 | 0 | 0 | 0 | 0 | 0 | 0 | 0 | 1 | 0 | 0 | 0 | 0 | 0 | 1 | 0 | 0 | 0 | 0 | 0 | 1 | 0 | 0 | 0 | 0 | | | | | | |
| 2856 | 03396 | 02855 | 2 | | 0 | 0 | 0 | 0 | 0 | 0 | 0 | 0 | 1 | 0 | 0 | 0 | 0 | 0 | 0 | 0 | 0 | 0 | 0 | 0 | 0 | 0 | 0 | 1 | 0 | | | | | | |
| 2857 | 03397 | 02856 | 1 | | 0 | 0 | 0 | 0 | 0 | 0 | 0 | 0 | 1 | 0 | 0 | 0 | 0 | 0 | 0 | 0 | 0 | 0 | 0 | 0 | 0 | 0 | 0 | 0 | 0 | | | | | | |
| 2858 | 03398 | 02857 | 1 | | 0 | 0 | 0 | 0 | 0 | 0 | 0 | 0 | 1 | 0 | 0 | 0 | 0 | 0 | 0 | 0 | 0 | 0 | 0 | 0 | 0 | 0 | 0 | 0 | 0 | | | | | | |
| 2859 | 03399 | 02858 | 2 | | 0 | 0 | 0 | 0 | 0 | 0 | 0 | 0 | 1 | 0 | 0 | 0 | 0 | 0 | 0 | 0 | 0 | 1 | 0 | 0 | 0 | 0 | 0 | 0 | 0 | | | | | | |
| 2860 | 03400 | 02859 | 1 | | 0 | 0 | 0 | 0 | 0 | 0 | 0 | 0 | 1 | 0 | 0 | 0 | 0 | 0 | 0 | 0 | 0 | 0 | 0 | 0 | 0 | 0 | 0 | 0 | 0 | | | | | | |
| 2861 | 03401 | 02860 | 1 | | 0 | 0 | 0 | 0 | 0 | 0 | 0 | 0 | 1 | 0 | 0 | 0 | 0 | 0 | 0 | 0 | 0 | 0 | 0 | 0 | 0 | 0 | 0 | 0 | 0 | | | | | | |
| 2862 | 03402 | 02861 | 5 | | 0 | 0 | 0 | 0 | 0 | 0 | 0 | 0 | 1 | 0 | 0 | 0 | 0 | 0 | 0 | 2 | 1 | 0 | 0 | 0 | 0 | 0 | 0 | 1 | 0 | X72841 | 95.2 | 394 | 1 | 1556 | 1943 |
| 2863 | 03403 | 02862 | 4 | | 0 | 0 | 0 | 0 | 0 | 0 | 0 | 0 | 2 | 0 | 0 | 1 | 0 | 0 | 0 | 0 | 0 | 0 | 0 | 0 | 0 | 0 | 0 | 1 | 0 | | | | | | |
| 2864 | 03404 | 02863 | 2 | | 0 | 0 | 0 | 0 | 0 | 0 | 0 | 0 | 1 | 0 | 0 | 0 | 0 | 0 | 0 | 0 | 0 | 0 | 0 | 0 | 0 | 0 | 0 | 1 | 0 | | | | | | |
| 2865 | 03405 | 02864 | 1 | | 0 | 0 | 0 | 0 | 0 | 0 | 0 | 0 | 1 | 0 | 0 | 0 | 0 | 0 | 0 | 0 | 0 | 0 | 0 | 0 | 0 | 0 | 0 | 0 | 0 | L20773 | 95.4 | 328 | 1 | 1237 | 1560 |

Table 80

| | A | B | C | E | G | I | K | M | O | Q | S | U | W | Y | AA | AC | AE | AG | AI | AK | AM | AO | AQ | AS | AU | AW | AY | BA | BC | BE | BF | BG | BH | BI | BK |
|---|---|---|---|---|---|---|---|---|---|---|---|---|---|---|---|---|---|---|---|---|---|---|---|---|---|---|---|---|---|---|---|---|---|---|---|
| 2866 | 03406 | 02865 | 2 | | 0 | 0 | 0 | 0 | 0 | 0 | 0 | 0 | 2 | 0 | 0 | 0 | 0 | 0 | 0 | 0 | 0 | 0 | 0 | 0 | 0 | 0 | 0 | 0 | 0 | | | | | | |
| 2867 | 03407 | 02866 | 1 | | 0 | 0 | 0 | 0 | 0 | 0 | 0 | 0 | 1 | 0 | 0 | 0 | 0 | 0 | 0 | 0 | 0 | 0 | 0 | 0 | 0 | 0 | 0 | 0 | 0 | | | | | | |
| 2868 | 03408 | 02867 | 1 | | 0 | 0 | 0 | 0 | 0 | 0 | 0 | 0 | 1 | 0 | 0 | 0 | 0 | 0 | 0 | 0 | 0 | 0 | 0 | 0 | 0 | 0 | 0 | 0 | 0 | | | | | | |
| 2869 | 03409 | 02868 | 2 | | 0 | 0 | 0 | 0 | 0 | 0 | 0 | 0 | 2 | 0 | 0 | 0 | 0 | 0 | 0 | 0 | 0 | 0 | 0 | 0 | 0 | 0 | 0 | 0 | 0 | | | | | | |
| 2870 | 03410 | 02869 | 1 | | 0 | 0 | 0 | 0 | 0 | 0 | 0 | 0 | 1 | 0 | 0 | 0 | 0 | 0 | 0 | 0 | 0 | 0 | 0 | 0 | 0 | 0 | 0 | 0 | 0 | | | | | | |
| 2871 | 03411 | 02870 | 1 | | 0 | 0 | 0 | 0 | 0 | 0 | 0 | 0 | 1 | 0 | 0 | 0 | 0 | 0 | 0 | 0 | 0 | 0 | 0 | 0 | 0 | 0 | 0 | 0 | 0 | | | | | | |
| 2872 | 03412 | 02871 | 1 | | 0 | 0 | 0 | 0 | 0 | 0 | 0 | 0 | 1 | 0 | 0 | 0 | 0 | 0 | 0 | 0 | 0 | 0 | 0 | 0 | 0 | 0 | 0 | 0 | 0 | | | | | | |
| 2873 | 03413 | 02872 | 3 | | 0 | 0 | 0 | 0 | 0 | 0 | 0 | 0 | 2 | 0 | 0 | 0 | 0 | 0 | 0 | 0 | 0 | 0 | 0 | 0 | 0 | 0 | 1 | 0 | 0 | X66397 | 97.2 | 323 | 1 | 7170 | 7496 |
| 2874 | 03414 | 02873 | 1 | | 0 | 0 | 0 | 0 | 0 | 0 | 0 | 0 | 1 | 0 | 0 | 0 | 0 | 0 | 0 | 0 | 0 | 0 | 0 | 0 | 0 | 0 | 0 | 0 | 0 | | | | | | |
| 2875 | 03415 | 02874 | 2 | | 0 | 0 | 0 | 0 | 0 | 0 | 0 | 0 | 2 | 0 | 0 | 0 | 0 | 0 | 0 | 0 | 0 | 0 | 0 | 0 | 0 | 0 | 0 | 0 | 0 | | | | | | |
| 2876 | 03416 | 02875 | 4 | | 0 | 0 | 0 | 0 | 0 | 0 | 0 | 0 | 1 | 0 | 0 | 1 | 0 | 0 | 0 | 0 | 0 | 0 | 0 | 0 | 2 | 0 | 0 | 0 | 0 | | | | | | |
| 2877 | 03417 | 02876 | 1 | | 0 | 0 | 0 | 0 | 0 | 0 | 0 | 0 | 1 | 0 | 0 | 0 | 0 | 0 | 0 | 0 | 0 | 0 | 0 | 0 | 0 | 0 | 0 | 0 | 0 | | | | | | |
| 2878 | 03418 | 02877 | 1 | | 0 | 0 | 0 | 0 | 0 | 0 | 0 | 0 | 1 | 0 | 0 | 0 | 0 | 0 | 0 | 0 | 0 | 0 | 0 | 0 | 0 | 0 | 0 | 0 | 0 | | | | | | |
| 2879 | 03419 | 02878 | 7 | | 0 | 0 | 0 | 0 | 0 | 0 | 0 | 0 | 1 | 6 | 0 | 0 | 0 | 0 | 0 | 0 | 0 | 0 | 0 | 0 | 0 | 0 | 0 | 0 | 0 | D13666 | 96.1 | 306 | 1 | 2485 | 3213 |
| 2880 | 03420 | 02879 | 1 | | 0 | 0 | 0 | 0 | 0 | 0 | 0 | 0 | 1 | 0 | 0 | 0 | 0 | 0 | 0 | 0 | 0 | 0 | 0 | 0 | 0 | 0 | 0 | 0 | 0 | | | | | | |
| 2881 | 03421 | 02880 | 4 | | 0 | 0 | 0 | 0 | 0 | 0 | 0 | 0 | 2 | 1 | 0 | 0 | 0 | 0 | 0 | 1 | 0 | 0 | 0 | 0 | 0 | 0 | 0 | 0 | 0 | | | | | | |
| 2882 | 03422 | 02881 | 1 | | 0 | 0 | 0 | 0 | 0 | 0 | 0 | 0 | 1 | 0 | 0 | 0 | 0 | 0 | 0 | 0 | 0 | 0 | 0 | 0 | 0 | 0 | 0 | 0 | 0 | | | | | | |
| 2883 | 03423 | 02882 | 1 | | 0 | 0 | 0 | 0 | 0 | 0 | 0 | 0 | 1 | 0 | 0 | 0 | 0 | 0 | 0 | 0 | 0 | 0 | 0 | 0 | 0 | 0 | 0 | 0 | 0 | | | | | | |
| 2884 | 03424 | 02883 | 4 | | 0 | 0 | 0 | 0 | 0 | 0 | 0 | 0 | 1 | 0 | 0 | 0 | 0 | 0 | 0 | 0 | 1 | 0 | 0 | 0 | 0 | 0 | 1 | 1 | 0 | M29536 | 97.1 | 373 | 1 | 970 | 1416 |
| 2885 | 03426 | 02884 | 1 | | 0 | 0 | 0 | 0 | 0 | 0 | 0 | 0 | 1 | 0 | 0 | 0 | 0 | 0 | 0 | 0 | 0 | 0 | 0 | 0 | 0 | 0 | 0 | 0 | 0 | | | | | | |
| 2886 | 03427 | 02885 | 2 | | 0 | 0 | 0 | 0 | 0 | 0 | 0 | 0 | 1 | 0 | 0 | 0 | 1 | 0 | 0 | 0 | 0 | 0 | 0 | 0 | 0 | 0 | 0 | 0 | 0 | | | | | | |
| 2887 | 03428 | 02886 | 3 | | 0 | 0 | 0 | 0 | 0 | 0 | 0 | 0 | 2 | 0 | 0 | 0 | 0 | 0 | 0 | 0 | 0 | 0 | 0 | 0 | 1 | 0 | 0 | 0 | 0 | | | | | | |
| 2888 | 03429 | 02887 | 4 | | 0 | 0 | 0 | 0 | 0 | 0 | 0 | 0 | 1 | 0 | 0 | 0 | 0 | 0 | 2 | 1 | 0 | 0 | 0 | 0 | 0 | 0 | 0 | 0 | 0 | | | | | | |
| 2889 | 03430 | 02888 | 4 | | 0 | 0 | 0 | 0 | 0 | 0 | 0 | 0 | 1 | 0 | 1 | 0 | 0 | 0 | 0 | 0 | 0 | 0 | 0 | 0 | 0 | 1 | 0 | 1 | 0 | | | | | | |
| 2890 | 03431 | 02889 | 1 | | 0 | 0 | 0 | 0 | 0 | 0 | 0 | 0 | 1 | 0 | 0 | 0 | 0 | 0 | 0 | 0 | 0 | 0 | 0 | 0 | 0 | 0 | 0 | 0 | 0 | | | | | | |
| 2891 | 03432 | 02890 | 1 | | 0 | 0 | 0 | 0 | 0 | 0 | 0 | 0 | 1 | 0 | 0 | 0 | 0 | 0 | 0 | 0 | 0 | 0 | 0 | 0 | 0 | 0 | 0 | 0 | 0 | | | | | | |
| 2892 | 03433 | 02891 | 1 | | 0 | 0 | 0 | 0 | 0 | 0 | 0 | 0 | 1 | 0 | 0 | 0 | 0 | 0 | 0 | 0 | 0 | 0 | 0 | 0 | 0 | 0 | 0 | 0 | 0 | | | | | | |
| 2893 | 03434 | 02892 | 1 | | 0 | 0 | 0 | 0 | 0 | 0 | 0 | 0 | 1 | 0 | 0 | 0 | 0 | 0 | 0 | 0 | 0 | 0 | 0 | 0 | 0 | 0 | 0 | 0 | 0 | L22473 | 96.2 | 106 | 71 | 474 | 579 |
| 2894 | 03435 | 02893 | 1 | | 0 | 0 | 0 | 0 | 0 | 0 | 0 | 0 | 1 | 0 | 0 | 0 | 0 | 0 | 0 | 0 | 0 | 0 | 0 | 0 | 0 | 0 | 0 | 0 | 0 | | | | | | |
| 2895 | 03436 | 02894 | 3 | | 0 | 0 | 0 | 0 | 0 | 0 | 0 | 0 | 1 | 0 | 0 | 0 | 0 | 0 | 0 | 0 | 1 | 0 | 1 | 0 | 0 | 0 | 0 | 0 | 0 | | | | | | |
| 2896 | 03437 | 02895 | 1 | | 0 | 0 | 0 | 0 | 0 | 0 | 0 | 0 | 1 | 0 | 0 | 0 | 0 | 0 | 0 | 0 | 0 | 0 | 0 | 0 | 0 | 0 | 0 | 0 | 0 | | | | | | |
| 2897 | 03438 | 02896 | 1 | | 0 | 0 | 0 | 0 | 0 | 0 | 0 | 0 | 1 | 0 | 0 | 0 | 0 | 0 | 0 | 0 | 0 | 0 | 0 | 0 | 0 | 0 | 0 | 0 | 0 | | | | | | |
| 2898 | 03439 | 02897 | 6 | | 0 | 0 | 0 | 0 | 0 | 0 | 0 | 0 | 2 | 0 | 0 | 0 | 0 | 0 | 0 | 0 | 0 | 1 | 0 | 0 | 0 | 1 | 0 | 2 | 0 | X14958 | 95 | 282 | 1 | 1596 | 1875 |
| 2899 | 03440 | 02898 | 2 | | 0 | 0 | 0 | 0 | 0 | 0 | 0 | 0 | 1 | 0 | 0 | 0 | 0 | 0 | 0 | 0 | 0 | 0 | 0 | 0 | 0 | 1 | 0 | 0 | 0 | | | | | | |
| 2900 | 03441 | 02899 | 4 | | 0 | 0 | 0 | 0 | 0 | 0 | 0 | 0 | 1 | 0 | 0 | 0 | 0 | 0 | 1 | 0 | 0 | 1 | 0 | 0 | 0 | 0 | 0 | 1 | 0 | M86667 | 97.6 | 83 | 1 | 1085 | 1560 |
| 2901 | 03442 | 02900 | 2 | | 0 | 0 | 0 | 0 | 0 | 0 | 0 | 0 | 2 | 0 | 0 | 0 | 0 | 0 | 0 | 0 | 0 | 0 | 0 | 0 | 0 | 0 | 0 | 0 | 0 | | | | | | |

Table 81

| | A | B | C | E | G | I | K | M | O | Q | S | U | W | Y | AA | AC | AF | AG | AI | AK | AM | AO | AQ | AS | AU | AW | AY | BA | BC | BE | BF | BG | BH | BI | BK |
|---|---|---|---|---|---|---|---|---|---|---|---|---|---|---|---|---|---|---|---|---|---|---|---|---|---|---|---|---|---|---|---|---|---|---|---|
| 2902 | 03443 | 02901 | 14 | | 0 | 0 | 0 | 0 | 0 | 0 | 0 | 0 | 1 | 0 | 0 | 0 | 0 | 0 | 0 | 3 | 4 | 1 | 0 | 0 | 0 | 5 | 0 | 0 | 0 | | | | | | |
| 2903 | 03444 | 02902 | 1 | | 0 | 0 | 0 | 0 | 0 | 0 | 0 | 0 | 1 | 0 | 0 | 0 | 0 | 0 | 0 | 0 | 0 | 0 | 0 | 0 | 0 | 0 | 0 | 0 | 0 | | | | | | |
| 2904 | 03445 | 02903 | 1 | | 0 | 0 | 0 | 0 | 0 | 0 | 0 | 0 | 1 | 0 | 0 | 0 | 0 | 0 | 0 | 0 | 0 | 0 | 0 | 0 | 0 | 0 | 0 | 0 | 0 | | | | | | |
| 2905 | 03446 | 02904 | 1 | | 0 | 0 | 0 | 0 | 0 | 0 | 0 | 0 | 1 | 0 | 0 | 0 | 0 | 0 | 0 | 0 | 0 | 0 | 0 | 0 | 0 | 0 | 0 | 0 | 0 | | | | | | |
| 2906 | 03447 | 02905 | 5 | | 0 | 0 | 0 | 0 | 0 | 0 | 0 | 0 | 1 | 0 | 1 | 1 | 0 | 0 | 0 | 0 | 0 | 0 | 1 | 0 | 0 | 1 | 0 | 0 | 0 | S54769 | 96.2 | 264 | 1 | 161 | 429 |
| 2907 | 03448 | 02906 | 1 | | 0 | 0 | 0 | 0 | 0 | 0 | 0 | 0 | 1 | 0 | 0 | 0 | 0 | 0 | 0 | 0 | 0 | 0 | 0 | 0 | 0 | 0 | 0 | 0 | 0 | | | | | | |
| 2908 | 03449 | 02907 | 1 | | 0 | 0 | 0 | 0 | 0 | 0 | 0 | 0 | 1 | 0 | 0 | 0 | 0 | 0 | 0 | 0 | 0 | 0 | 0 | 0 | 0 | 0 | 0 | 0 | 0 | | | | | | |
| 2909 | 03450 | 02908 | 1 | | 0 | 0 | 0 | 0 | 0 | 0 | 0 | 0 | 1 | 0 | 0 | 0 | 0 | 0 | 0 | 0 | 0 | 0 | 0 | 0 | 0 | 0 | 0 | 0 | 0 | | | | | | |
| 2910 | 03451 | 02909 | 1 | | 0 | 0 | 0 | 0 | 0 | 0 | 0 | 0 | 1 | 0 | 0 | 0 | 0 | 0 | 0 | 0 | 0 | 0 | 0 | 0 | 0 | 0 | 0 | 0 | 0 | | | | | | |
| 2911 | 03452 | 02910 | 2 | | 0 | 0 | 0 | 0 | 0 | 0 | 0 | 0 | 1 | 0 | 0 | 0 | 0 | 0 | 0 | 1 | 0 | 0 | 0 | 0 | 0 | 0 | 0 | 0 | 0 | | | | | | |
| 2912 | 03453 | 02911 | 1 | | 0 | 0 | 0 | 0 | 0 | 0 | 0 | 0 | 1 | 0 | 0 | 0 | 0 | 0 | 0 | 0 | 0 | 0 | 0 | 0 | 0 | 0 | 0 | 0 | 0 | | | | | | |
| 2913 | 03454 | 02912 | 1 | | 0 | 0 | 0 | 0 | 0 | 0 | 0 | 0 | 1 | 0 | 0 | 0 | 0 | 0 | 0 | 0 | 0 | 0 | 0 | 0 | 0 | 0 | 0 | 0 | 0 | | | | | | |
| 2914 | 03455 | 02913 | 5 | | 0 | 0 | 0 | 0 | 0 | 0 | 0 | 0 | 1 | 0 | 0 | 0 | 0 | 0 | 0 | 1 | 0 | 3 | 0 | 0 | 0 | 0 | 0 | 0 | 0 | | | | | | |
| 2915 | 03456 | 02914 | 1 | | 0 | 0 | 0 | 0 | 0 | 0 | 0 | 0 | 1 | 0 | 0 | 0 | 0 | 0 | 0 | 0 | 0 | 0 | 0 | 0 | 0 | 0 | 0 | 0 | 0 | | | | | | |
| 2916 | 03457 | 02915 | 1 | | 0 | 0 | 0 | 0 | 0 | 0 | 0 | 0 | 1 | 0 | 0 | 0 | 0 | 0 | 0 | 0 | 0 | 0 | 0 | 0 | 0 | 0 | 0 | 0 | 0 | | | | | | |
| 2917 | 03458 | 02916 | 1 | | 0 | 0 | 0 | 0 | 0 | 0 | 0 | 0 | 1 | 0 | 0 | 0 | 0 | 0 | 0 | 0 | 0 | 0 | 0 | 0 | 0 | 0 | 0 | 0 | 0 | M98479 | 95.1 | 247 | 1 | 1311 | 1557 |
| 2918 | 03459 | 02917 | 7 | | 0 | 0 | 0 | 0 | 0 | 0 | 0 | 0 | 2 | 4 | 0 | 0 | 0 | 0 | 0 | 0 | 0 | 1 | 0 | 0 | 0 | 0 | 0 | 0 | 0 | | | | | | |
| 2919 | 03460 | 02918 | 8 | | 0 | 0 | 0 | 0 | 0 | 0 | 0 | 0 | 3 | 1 | 0 | 0 | 0 | 0 | 3 | 0 | 0 | 1 | 0 | 0 | 0 | 0 | 0 | 0 | 0 | J05481 | 95.5 | 242 | 1 | 2363 | 2620 |
| 2920 | 03461 | 02919 | 5 | | 0 | 0 | 0 | 0 | 0 | 0 | 0 | 0 | 1 | 1 | 1 | 1 | 0 | 0 | 0 | 0 | 0 | 0 | 0 | 0 | 1 | 0 | 0 | 0 | 0 | | | | | | |
| 2921 | 03462 | 02920 | 1 | | 0 | 0 | 0 | 0 | 0 | 0 | 0 | 0 | 1 | 0 | 0 | 0 | 0 | 0 | 0 | 0 | 0 | 0 | 0 | 0 | 0 | 0 | 0 | 0 | 0 | | | | | | |
| 2922 | 03463 | 02921 | 1 | | 0 | 0 | 0 | 0 | 0 | 0 | 0 | 0 | 1 | 0 | 0 | 0 | 0 | 0 | 0 | 0 | 0 | 0 | 0 | 0 | 0 | 0 | 0 | 0 | 0 | | | | | | |
| 2923 | 03464 | 02922 | 3 | | 0 | 0 | 0 | 0 | 0 | 0 | 0 | 0 | 1 | 0 | 0 | 0 | 0 | 0 | 0 | 0 | 0 | 0 | 0 | 0 | 0 | 0 | 0 | 0 | 2 | | | | | | |
| 2924 | 03465 | 02923 | 1 | | 0 | 0 | 0 | 0 | 0 | 0 | 0 | 0 | 1 | 0 | 0 | 0 | 0 | 0 | 0 | 0 | 0 | 0 | 0 | 0 | 0 | 0 | 0 | 0 | 0 | | | | | | |
| 2925 | 03466 | 02924 | 2 | | 0 | 0 | 0 | 0 | 0 | 0 | 0 | 0 | 1 | 0 | 0 | 0 | 0 | 0 | 1 | 0 | 0 | 0 | 0 | 0 | 0 | 0 | 0 | 0 | 0 | | | | | | |
| 2926 | 03467 | 02925 | 1 | | 0 | 0 | 0 | 0 | 0 | 0 | 0 | 0 | 1 | 0 | 0 | 0 | 0 | 0 | 0 | 0 | 0 | 0 | 0 | 0 | 0 | 0 | 0 | 0 | 0 | | | | | | |
| 2927 | 03468 | 02926 | 5 | | 0 | 0 | 0 | 0 | 0 | 0 | 0 | 0 | 1 | 1 | 0 | 0 | 0 | 0 | 0 | 0 | 0 | 3 | 0 | 0 | 0 | 0 | 0 | 0 | 0 | | | | | | |
| 2928 | 03469 | 02927 | 4 | | 0 | 0 | 0 | 0 | 0 | 0 | 0 | 0 | 1 | 0 | 0 | 1 | 0 | 0 | 0 | 0 | 0 | 0 | 0 | 0 | 0 | 0 | 0 | 2 | 0 | D14134 | 100 | 234 | 1 | 1436 | 2229 |
| 2929 | 03470 | 02928 | 2 | | 0 | 0 | 0 | 0 | 0 | 0 | 0 | 0 | 1 | 0 | 0 | 0 | 0 | 0 | 0 | 0 | 1 | 0 | 0 | 0 | 0 | 0 | 0 | 0 | 0 | | | | | | |
| 2930 | 03471 | 02929 | 6 | | 0 | 0 | 0 | 0 | 0 | 0 | 0 | 0 | 2 | 0 | 0 | 0 | 0 | 1 | 0 | 0 | 0 | 0 | 0 | 3 | 0 | 0 | 0 | 0 | 0 | | | | | | |
| 2931 | 03472 | 02930 | 2 | | 0 | 0 | 0 | 0 | 0 | 0 | 0 | 0 | 2 | 0 | 0 | 0 | 0 | 0 | 0 | 0 | 0 | 0 | 0 | 0 | 0 | 0 | 0 | 0 | 0 | | | | | | |
| 2932 | 03473 | 02931 | 3 | | 0 | 0 | 0 | 0 | 0 | 0 | 0 | 0 | 1 | 0 | 0 | 0 | 0 | 0 | 1 | 0 | 0 | 0 | 0 | 1 | 0 | 0 | 0 | 0 | 0 | | | | | | |
| 2933 | 03474 | 02932 | 1 | | 0 | 0 | 0 | 0 | 0 | 0 | 0 | 0 | 1 | 0 | 0 | 0 | 0 | 0 | 0 | 0 | 0 | 0 | 0 | 0 | 0 | 0 | 0 | 0 | 0 | | | | | | |
| 2934 | 03476 | 02933 | 2 | | 0 | 0 | 0 | 0 | 0 | 0 | 0 | 0 | 1 | 0 | 0 | 0 | 0 | 0 | 0 | 0 | 1 | 0 | 0 | 0 | 0 | 0 | 0 | 0 | 0 | L12535 | 98.2 | 327 | 1 | 1625 | 2194 |
| 2935 | 03477 | 02934 | 1 | | 0 | 0 | 0 | 0 | 0 | 0 | 0 | 0 | 1 | 0 | 0 | 0 | 0 | 0 | 0 | 0 | 0 | 0 | 0 | 0 | 0 | 0 | 0 | 0 | 0 | | | | | | |
| 2936 | 03478 | 02935 | 1 | | 0 | 0 | 0 | 0 | 0 | 0 | 0 | 0 | 1 | 0 | 0 | 0 | 0 | 0 | 0 | 0 | 0 | 0 | 0 | 0 | 0 | 0 | 0 | 0 | 0 | | | | | | |
| 2937 | 03479 | 02936 | 1 | | 0 | 0 | 0 | 0 | 0 | 0 | 0 | 0 | 1 | 0 | 0 | 0 | 0 | 0 | 0 | 0 | 0 | 0 | 0 | 0 | 0 | 0 | 0 | 0 | 0 | | | | | | |

Table 82

| | A | B | C | E | G | I | K | M | O | Q | S | U | W | Y | AA | AC | AF | AG | AI | AK | AM | AO | AQ | AS | AU | AW | AY | BA | BC | BE | BF | BG | BH | BI | BK |
|---|---|---|---|---|---|---|---|---|---|---|---|---|---|---|---|---|---|---|---|---|---|---|---|---|---|---|---|---|---|---|---|---|---|---|---|
| 2938 | 03480 | 02937 | 1 | | 0 | 0 | 0 | 0 | 0 | 0 | 0 | 0 | 1 | 0 | 0 | 0 | 0 | 0 | 0 | 0 | 0 | 0 | 0 | 0 | 0 | 0 | 0 | 0 | 0 | | | | | | |
| 2939 | 03481 | 02938 | 1 | | 0 | 0 | 0 | 0 | 0 | 0 | 0 | 0 | 1 | 0 | 0 | 0 | 0 | 0 | 0 | 0 | 0 | 0 | 0 | 0 | 0 | 0 | 0 | 0 | 0 | | | | | | |
| 2940 | 03482 | 02939 | 2 | | 0 | 0 | 0 | 0 | 0 | 0 | 0 | 0 | 1 | 0 | 0 | 0 | 0 | 0 | 0 | 0 | 0 | 0 | 0 | 0 | 0 | 0 | 0 | 1 | 0 | | | | | | |
| 2941 | 03483 | 02940 | 1 | | 0 | 0 | 0 | 0 | 0 | 0 | 0 | 0 | 1 | 0 | 0 | 0 | 0 | 0 | 0 | 0 | 0 | 0 | 0 | 0 | 0 | 0 | 0 | 0 | 0 | | | | | | |
| 2942 | 03484 | 02941 | 2 | | 0 | 0 | 0 | 0 | 0 | 0 | 0 | 0 | 1 | 0 | 0 | 0 | 0 | 0 | 0 | 0 | 0 | 0 | 0 | 0 | 0 | 0 | 0 | 1 | 0 | | | | | | |
| 2943 | 03485 | 02942 | 2 | | 0 | 0 | 0 | 0 | 0 | 0 | 0 | 0 | 1 | 0 | 0 | 0 | 0 | 0 | 0 | 0 | 0 | 0 | 0 | 1 | 0 | 0 | 0 | 0 | 0 | M29063 | 98.6 | 212 | 1 | 1175 | 1398 |
| 2944 | 03486 | 02943 | 7 | | 0 | 0 | 0 | 0 | 0 | 0 | 0 | 0 | 1 | 1 | 1 | 1 | 0 | 0 | 0 | 0 | 0 | 1 | 0 | 1 | 1 | 0 | 0 | 0 | 0 | | | | | | |
| 2945 | 03487 | 02944 | 1 | | 0 | 0 | 0 | 0 | 0 | 0 | 0 | 0 | 1 | 0 | 0 | 0 | 0 | 0 | 0 | 0 | 0 | 0 | 0 | 0 | 0 | 0 | 0 | 0 | 0 | | | | | | |
| 2946 | 03488 | 02945 | 2 | | 0 | 0 | 0 | 0 | 0 | 0 | 0 | 0 | 1 | 0 | 0 | 0 | 0 | 0 | 0 | 1 | 0 | 0 | 0 | 0 | 0 | 0 | 0 | 0 | 0 | | | | | | |
| 2947 | 03489 | 02946 | 1 | | 0 | 0 | 0 | 0 | 0 | 0 | 0 | 0 | 1 | 0 | 0 | 0 | 0 | 0 | 0 | 0 | 0 | 0 | 0 | 0 | 0 | 0 | 0 | 0 | 0 | | | | | | |
| 2948 | 03490 | 02947 | 3 | | 0 | 0 | 0 | 0 | 0 | 0 | 0 | 0 | 2 | 0 | 0 | 0 | 0 | 0 | 0 | 0 | 0 | 0 | 1 | 0 | 0 | 0 | 0 | 0 | 0 | | | | | | |
| 2949 | 03491 | 02948 | 1 | | 0 | 0 | 0 | 0 | 0 | 0 | 0 | 0 | 1 | 0 | 0 | 0 | 0 | 0 | 0 | 0 | 0 | 0 | 0 | 0 | 0 | 0 | 0 | 0 | 0 | | | | | | |
| 2950 | 03492 | 02949 | 2 | | 0 | 0 | 0 | 0 | 0 | 0 | 0 | 0 | 1 | 0 | 0 | 0 | 0 | 0 | 1 | 0 | 0 | 0 | 0 | 0 | 0 | 0 | 0 | 0 | 0 | | | | | | |
| 2951 | 03493 | 02950 | 1 | | 0 | 0 | 0 | 0 | 0 | 0 | 0 | 0 | 1 | 0 | 0 | 0 | 0 | 0 | 0 | 0 | 0 | 0 | 0 | 0 | 0 | 0 | 0 | 0 | 0 | | | | | | |
| 2952 | 03494 | 02951 | 1 | | 0 | 0 | 0 | 0 | 0 | 0 | 0 | 0 | 1 | 0 | 0 | 0 | 0 | 0 | 0 | 0 | 0 | 0 | 0 | 0 | 0 | 0 | 0 | 0 | 0 | | | | | | |
| 2953 | 03495 | 02952 | 2 | | 0 | 0 | 0 | 0 | 0 | 0 | 0 | 0 | 1 | 0 | 0 | 0 | 0 | 0 | 0 | 1 | 0 | 0 | 0 | 0 | 0 | 0 | 0 | 0 | 0 | | | | | | |
| 2954 | 03496 | 02953 | 2 | | 0 | 0 | 0 | 0 | 0 | 0 | 0 | 0 | 1 | 0 | 0 | 0 | 0 | 0 | 0 | 1 | 0 | 0 | 0 | 0 | 0 | 0 | 0 | 0 | 0 | | | | | | |
| 2955 | 03497 | 02954 | 3 | | 0 | 0 | 0 | 0 | 0 | 0 | 0 | 0 | 2 | 0 | 0 | 0 | 0 | 0 | 0 | 0 | 0 | 1 | 0 | 0 | 0 | 0 | 0 | 0 | 0 | M34671 | 100 | 191 | 1 | 917 | 1671 |
| 2956 | 03498 | 02955 | 1 | | 0 | 0 | 0 | 0 | 0 | 0 | 0 | 0 | 1 | 0 | 0 | 0 | 0 | 0 | 0 | 0 | 0 | 0 | 0 | 0 | 0 | 0 | 0 | 0 | 0 | | | | | | |
| 2957 | 03499 | 02956 | 5 | | 0 | 0 | 0 | 0 | 0 | 0 | 0 | 0 | 1 | 1 | 0 | 1 | 0 | 0 | 0 | 0 | 0 | 1 | 0 | 1 | 0 | 0 | 0 | 0 | 0 | | | | | | |
| 2958 | 03500 | 02957 | 4 | | 0 | 0 | 0 | 0 | 0 | 0 | 0 | 0 | 3 | 0 | 0 | 0 | 0 | 0 | 0 | 0 | 0 | 0 | 0 | 0 | 1 | 0 | 0 | 0 | 0 | | | | | | |
| 2959 | 03501 | 02958 | 1 | | 0 | 0 | 0 | 0 | 0 | 0 | 0 | 0 | 1 | 0 | 0 | 0 | 0 | 0 | 0 | 0 | 0 | 0 | 0 | 0 | 0 | 0 | 0 | 0 | 0 | | | | | | |
| 2960 | 03502 | 02959 | 6 | | 0 | 0 | 0 | 0 | 0 | 0 | 0 | 0 | 1 | 0 | 2 | 0 | 0 | 0 | 1 | 0 | 0 | 0 | 0 | 0 | 1 | 1 | 0 | 0 | 0 | | | | | | |
| 2961 | 03503 | 02960 | 4 | | 0 | 0 | 0 | 0 | 0 | 0 | 0 | 0 | 1 | 0 | 1 | 0 | 0 | 0 | 0 | 0 | 0 | 1 | 0 | 0 | 1 | 0 | 0 | 0 | 0 | L25081 | 96.6 | 175 | 1 | 887 | 1058 |
| 2962 | 03504 | 02961 | 2 | | 0 | 0 | 0 | 0 | 0 | 0 | 0 | 0 | 1 | 0 | 0 | 0 | 0 | 0 | 0 | 0 | 0 | 0 | 0 | 0 | 0 | 0 | 0 | 1 | 0 | | | | | | |
| 2963 | 03505 | 02962 | 2 | | 0 | 0 | 0 | 0 | 0 | 0 | 0 | 0 | 1 | 0 | 0 | 0 | 0 | 0 | 0 | 0 | 0 | 0 | 0 | 0 | 0 | 0 | 1 | 0 | 0 | | | | | | |
| 2964 | 03506 | 02963 | 1 | | 0 | 0 | 0 | 0 | 0 | 0 | 0 | 0 | 1 | 0 | 0 | 0 | 0 | 0 | 0 | 0 | 0 | 0 | 0 | 0 | 0 | 0 | 0 | 0 | 0 | | | | | | |
| 2965 | 03507 | 02964 | 1 | | 0 | 0 | 0 | 0 | 0 | 0 | 0 | 0 | 1 | 0 | 0 | 0 | 0 | 0 | 0 | 0 | 0 | 0 | 0 | 0 | 0 | 0 | 0 | 0 | 0 | | | | | | |
| 2966 | 03508 | 02965 | 3 | | 0 | 0 | 0 | 0 | 0 | 0 | 0 | 0 | 3 | 0 | 0 | 0 | 0 | 0 | 0 | 0 | 0 | 0 | 0 | 0 | 0 | 0 | 0 | 0 | 0 | | | | | | |
| 2967 | 03509 | 02966 | 3 | | 0 | 0 | 0 | 0 | 0 | 0 | 0 | 0 | 1 | 0 | 1 | 0 | 0 | 0 | 0 | 1 | 0 | 0 | 0 | 0 | 0 | 0 | 0 | 0 | 0 | | | | | | |
| 2968 | 03510 | 02967 | 1 | | 0 | 0 | 0 | 0 | 0 | 0 | 0 | 0 | 1 | 0 | 0 | 0 | 0 | 0 | 0 | 0 | 0 | 0 | 0 | 0 | 0 | 0 | 0 | 0 | 0 | | | | | | |
| 2969 | 03511 | 02968 | 4 | | 0 | 0 | 0 | 0 | 0 | 0 | 0 | 0 | 1 | 0 | 0 | 0 | 0 | 1 | 0 | 1 | 0 | 0 | 0 | 1 | 0 | 0 | 0 | 0 | 0 | | | | | | |
| 2970 | 03512 | 02969 | 3 | | 0 | 0 | 0 | 0 | 0 | 0 | 0 | 0 | 1 | 0 | 0 | 0 | 0 | 0 | 0 | 1 | 0 | 0 | 0 | 1 | 0 | 0 | 0 | 0 | 0 | | | | | | |
| 2971 | 03513 | 02970 | 2 | | 0 | 0 | 0 | 0 | 0 | 0 | 0 | 0 | 1 | 0 | 0 | 0 | 0 | 0 | 1 | 0 | 0 | 0 | 0 | 0 | 0 | 0 | 0 | 0 | 0 | | | | | | |
| 2972 | 03514 | 02971 | 1 | | 0 | 0 | 0 | 0 | 0 | 0 | 0 | 0 | 1 | 0 | 0 | 0 | 0 | 0 | 0 | 0 | 0 | 0 | 0 | 0 | 0 | 0 | 0 | 0 | 0 | | | | | | |
| 2973 | 03515 | 02972 | 1 | | 0 | 0 | 0 | 0 | 0 | 0 | 0 | 0 | 1 | 0 | 0 | 0 | 0 | 0 | 0 | 0 | 0 | 0 | 0 | 0 | 0 | 0 | 0 | 0 | 0 | | | | | | |

Table 83

| | A | B | C | E | G | I | K | M | O | Q | S | U | W | Y | AA | AC | AE | AG | AI | AK | AM | AO | AQ | AS | AU | AW | AY | BA | BC | BE | BF | BG | BH | BI | BK |
|---|---|---|---|---|---|---|---|---|---|---|---|---|---|---|---|---|---|---|---|---|---|---|---|---|---|---|---|---|---|---|---|---|---|---|---|
| 2974 | 03516 | 02973 | 3 | | 0 | 0 | 0 | 0 | 0 | 0 | 0 | 0 | 1 | 0 | 0 | 0 | 0 | 0 | 0 | 1 | 0 | 0 | 1 | 0 | 0 | 0 | 0 | 0 | 0 | | | | | | |
| 2975 | 03517 | 02974 | 10 | | 0 | 0 | 0 | 0 | 0 | 0 | 0 | 0 | 4 | 4 | 0 | 0 | 0 | 0 | 1 | 0 | 0 | 0 | 0 | 0 | 1 | 0 | 0 | 0 | 0 | | | | | | |
| 2976 | 03518 | 02975 | 2 | | 0 | 0 | 0 | 0 | 0 | 0 | 0 | 0 | 1 | 0 | 0 | 0 | 0 | 0 | 0 | 0 | 0 | 0 | 1 | 0 | 0 | 0 | 0 | 0 | 0 | M95929 | 100 | 96 | 1 | 1335 | 1433 |
| 2977 | 03519 | 02976 | 2 | | 0 | 0 | 0 | 0 | 0 | 0 | 0 | 0 | 1 | 0 | 0 | 0 | 0 | 0 | 0 | 0 | 0 | 1 | 0 | 0 | 0 | 0 | 0 | 0 | 0 | | | | | | |
| 2978 | 03520 | 02977 | 1 | | 0 | 0 | 0 | 0 | 0 | 0 | 0 | 0 | 1 | 0 | 0 | 0 | 0 | 0 | 0 | 0 | 0 | 0 | 0 | 0 | 0 | 0 | 0 | 0 | 0 | | | | | | |
| 2979 | 03521 | 02978 | 3 | | 0 | 0 | 0 | 0 | 0 | 0 | 0 | 0 | 1 | 0 | 0 | 0 | 0 | 0 | 0 | 0 | 0 | 0 | 0 | 0 | 0 | 2 | 0 | 0 | 0 | S60099 | 97.6 | 166 | 1 | 2428 | 3727 |
| 2980 | 03522 | 02979 | 1 | | 0 | 0 | 0 | 0 | 0 | 0 | 0 | 0 | 1 | 0 | 0 | 0 | 0 | 0 | 0 | 0 | 0 | 0 | 0 | 0 | 0 | 0 | 0 | 0 | 0 | | | | | | |
| 2981 | 03523 | 02980 | 1 | | 0 | 0 | 0 | 0 | 0 | 0 | 0 | 0 | 1 | 0 | 0 | 0 | 0 | 0 | 0 | 0 | 0 | 0 | 0 | 0 | 0 | 0 | 0 | 0 | 0 | | | | | | |
| 2982 | 03524 | 02981 | 3 | | 0 | 0 | 0 | 0 | 0 | 0 | 0 | 0 | 1 | 0 | 0 | 0 | 0 | 0 | 2 | 0 | 0 | 0 | 0 | 0 | 0 | 0 | 0 | 0 | 0 | | | | | | |
| 2983 | 03525 | 02982 | 1 | | 0 | 0 | 0 | 0 | 0 | 0 | 0 | 0 | 1 | 0 | 0 | 0 | 0 | 0 | 0 | 0 | 0 | 0 | 0 | 0 | 0 | 0 | 0 | 0 | 0 | | | | | | |
| 2984 | 03526 | 02983 | 1 | | 0 | 0 | 0 | 0 | 0 | 0 | 0 | 0 | 1 | 0 | 0 | 0 | 0 | 0 | 0 | 0 | 0 | 0 | 0 | 0 | 0 | 0 | 0 | 0 | 0 | M33308 | 100 | 161 | 1 | 4937 | 5102 |
| 2985 | 03527 | 02984 | 4 | | 0 | 0 | 0 | 0 | 0 | 0 | 0 | 0 | 1 | 0 | 0 | 1 | 0 | 0 | 0 | 0 | 0 | 0 | 0 | 0 | 0 | 2 | 0 | 0 | 0 | | | | | | |
| 2986 | 03528 | 02985 | 4 | | 0 | 0 | 0 | 0 | 0 | 0 | 0 | 0 | 1 | 0 | 1 | 0 | 0 | 0 | 0 | 0 | 0 | 1 | 0 | 0 | 0 | 0 | 1 | 0 | 0 | | | | | | |
| 2987 | 03529 | 02986 | 6 | | 0 | 0 | 0 | 0 | 0 | 0 | 0 | 0 | 1 | 0 | 0 | 0 | 1 | 0 | 0 | 2 | 0 | 1 | 1 | 0 | 0 | 0 | 0 | 0 | 0 | | | | | | |
| 2988 | 03530 | 02987 | 2 | | 0 | 0 | 0 | 0 | 0 | 0 | 0 | 0 | 1 | 0 | 0 | 0 | 0 | 0 | 1 | 0 | 0 | 0 | 0 | 0 | 0 | 0 | 0 | 0 | 0 | | | | | | |
| 2989 | 03531 | 02988 | 1 | | 0 | 0 | 0 | 0 | 0 | 0 | 0 | 0 | 1 | 0 | 0 | 0 | 0 | 0 | 0 | 0 | 0 | 0 | 0 | 0 | 0 | 0 | 0 | 0 | 0 | | | | | | |
| 2990 | 03532 | 02989 | 1 | | 0 | 0 | 0 | 0 | 0 | 0 | 0 | 0 | 1 | 0 | 0 | 0 | 0 | 0 | 0 | 0 | 0 | 0 | 0 | 0 | 0 | 0 | 0 | 0 | 0 | | | | | | |
| 2991 | 03533 | 02990 | 1 | | 0 | 0 | 0 | 0 | 0 | 0 | 0 | 0 | 1 | 0 | 0 | 0 | 0 | 0 | 0 | 0 | 0 | 0 | 0 | 0 | 0 | 0 | 0 | 0 | 0 | | | | | | |
| 2992 | 03534 | 02991 | 2 | | 0 | 0 | 0 | 0 | 0 | 0 | 0 | 0 | 1 | 0 | 0 | 0 | 0 | 0 | 0 | 0 | 0 | 0 | 0 | 0 | 0 | 0 | 0 | 1 | 0 | | | | | | |
| 2993 | 03535 | 02992 | 9 | | 0 | 0 | 0 | 0 | 0 | 0 | 0 | 0 | 2 | 1 | 1 | 0 | 1 | 0 | 0 | 0 | 0 | 1 | 0 | 0 | 0 | 1 | 2 | 0 | 0 | | | | | | |
| 2994 | 03536 | 02993 | 1 | | 0 | 0 | 0 | 0 | 0 | 0 | 0 | 0 | 1 | 0 | 0 | 0 | 0 | 0 | 0 | 0 | 0 | 0 | 0 | 0 | 0 | 0 | 0 | 0 | 0 | | | | | | |
| 2995 | 03537 | 02994 | 1 | | 0 | 0 | 0 | 0 | 0 | 0 | 0 | 0 | 1 | 0 | 0 | 0 | 0 | 0 | 0 | 0 | 0 | 0 | 0 | 0 | 0 | 0 | 0 | 0 | 0 | | | | | | |
| 2996 | 03538 | 02995 | 2 | | 0 | 0 | 0 | 0 | 0 | 0 | 0 | 0 | 1 | 0 | 0 | 0 | 1 | 0 | 0 | 0 | 0 | 0 | 0 | 0 | 0 | 0 | 0 | 0 | 0 | | | | | | |
| 2997 | 03539 | 02996 | 1 | | 0 | 0 | 0 | 0 | 0 | 0 | 0 | 0 | 1 | 0 | 0 | 0 | 0 | 0 | 0 | 0 | 0 | 0 | 0 | 0 | 0 | 0 | 0 | 0 | 0 | | | | | | |
| 2998 | 03540 | 02997 | 1 | | 0 | 0 | 0 | 0 | 0 | 0 | 0 | 0 | 1 | 0 | 0 | 0 | 0 | 0 | 0 | 0 | 0 | 0 | 0 | 0 | 0 | 0 | 0 | 0 | 0 | | | | | | |
| 2999 | 03541 | 02998 | 1 | | 0 | 0 | 0 | 0 | 0 | 0 | 0 | 0 | 1 | 0 | 0 | 0 | 0 | 0 | 0 | 0 | 0 | 0 | 0 | 0 | 0 | 0 | 0 | 0 | 0 | | | | | | |
| 3000 | 03542 | 02999 | 1 | | 0 | 0 | 0 | 0 | 0 | 0 | 0 | 0 | 1 | 0 | 0 | 0 | 0 | 0 | 0 | 0 | 0 | 0 | 0 | 0 | 0 | 0 | 0 | 0 | 0 | | | | | | |
| 3001 | 03543 | 03000 | 1 | | 0 | 0 | 0 | 0 | 0 | 0 | 0 | 0 | 1 | 0 | 0 | 0 | 0 | 0 | 0 | 0 | 0 | 0 | 0 | 0 | 0 | 0 | 0 | 0 | 0 | | | | | | |
| 3002 | 03544 | 03001 | 1 | | 0 | 0 | 0 | 0 | 0 | 0 | 0 | 0 | 1 | 0 | 0 | 0 | 0 | 0 | 0 | 0 | 0 | 0 | 0 | 0 | 0 | 0 | 0 | 0 | 0 | | | | | | |
| 3003 | 03545 | 03002 | 1 | | 0 | 0 | 0 | 0 | 0 | 0 | 0 | 0 | 1 | 0 | 0 | 0 | 0 | 0 | 0 | 0 | 0 | 0 | 0 | 0 | 0 | 0 | 0 | 0 | 0 | | | | | | |
| 3004 | 03546 | 03003 | 1 | | 0 | 0 | 0 | 0 | 0 | 0 | 0 | 0 | 1 | 0 | 0 | 0 | 0 | 0 | 0 | 0 | 0 | 0 | 0 | 0 | 0 | 0 | 0 | 0 | 0 | | | | | | |
| 3005 | 03547 | 03004 | 1 | | 0 | 0 | 0 | 0 | 0 | 0 | 0 | 0 | 1 | 0 | 0 | 0 | 0 | 0 | 0 | 0 | 0 | 0 | 0 | 0 | 0 | 0 | 0 | 0 | 0 | | | | | | |
| 3006 | 03548 | 03005 | 4 | | 0 | 0 | 0 | 0 | 0 | 0 | 0 | 0 | 1 | 0 | 0 | 0 | 0 | 0 | 0 | 0 | 1 | 1 | 0 | 0 | 0 | 1 | 0 | 0 | 0 | | | | | | |
| 3007 | 03549 | 03006 | 1 | | 0 | 0 | 0 | 0 | 0 | 0 | 0 | 0 | 1 | 0 | 0 | 0 | 0 | 0 | 0 | 0 | 0 | 0 | 0 | 0 | 0 | 0 | 0 | 0 | 0 | | | | | | |
| 3008 | 03550 | 03007 | 3 | | 0 | 0 | 0 | 0 | 0 | 0 | 0 | 0 | 1 | 0 | 0 | 0 | 0 | 0 | 0 | 1 | 0 | 0 | 0 | 0 | 0 | 1 | 0 | 0 | 0 | | | | | | |
| 3009 | 03551 | 03008 | 1 | | 0 | 0 | 0 | 0 | 0 | 0 | 0 | 0 | 1 | 0 | 0 | 0 | 0 | 0 | 0 | 0 | 0 | 0 | 0 | 0 | 0 | 0 | 0 | 0 | 0 | | | | | | |

Table 84

| | A | B | C | E | G | I | K | M | O | Q | S | U | W | Y | AA | AC | AE | AG | AI | AK | AM | AO | AQ | AS | AU | AW | AY | BA | BC | BE | BF | BG | BH | BI | BK |
|---|---|---|---|---|---|---|---|---|---|---|---|---|---|---|---|---|---|---|---|---|---|---|---|---|---|---|---|---|---|---|---|---|---|---|---|
| 3010 | 03552 | 03009 | 4 | | 0 | 0 | 0 | 0 | 0 | 0 | 0 | 0 | 1 | 0 | 0 | 1 | 0 | 0 | 0 | 0 | 0 | 0 | 0 | 0 | 0 | 0 | 1 | 1 | 0 | | | | | | |
| 3011 | 03553 | 03010 | 2 | | 0 | 0 | 0 | 0 | 0 | 0 | 0 | 0 | 1 | 0 | 0 | 0 | 0 | 0 | 0 | 1 | 0 | 0 | 0 | 0 | 0 | 0 | 0 | 0 | 0 | | | | | | |
| 3012 | 03554 | 03011 | 5 | | 0 | 0 | 0 | 0 | 0 | 0 | 0 | 0 | 1 | 0 | 0 | 0 | 0 | 0 | 0 | 0 | 0 | 0 | 0 | 0 | 0 | 0 | 4 | 0 | 0 | | | | | | |
| 3013 | 03555 | 03012 | 1 | | 0 | 0 | 0 | 0 | 0 | 0 | 0 | 0 | 1 | 0 | 0 | 0 | 0 | 0 | 0 | 0 | 0 | 0 | 0 | 0 | 0 | 0 | 0 | 0 | 0 | | | | | | |
| 3014 | 03556 | 03013 | 1 | | 0 | 0 | 0 | 0 | 0 | 0 | 0 | 0 | 1 | 0 | 0 | 0 | 0 | 0 | 0 | 0 | 0 | 0 | 0 | 0 | 0 | 0 | 0 | 0 | 0 | | | | | | |
| 3015 | 03557 | 03014 | 1 | | 0 | 0 | 0 | 0 | 0 | 0 | 0 | 0 | 1 | 0 | 0 | 0 | 0 | 0 | 0 | 0 | 0 | 0 | 0 | 0 | 0 | 0 | 0 | 0 | 0 | | | | | | |
| 3016 | 03558 | 03015 | 5 | | 0 | 0 | 0 | 0 | 0 | 0 | 0 | 0 | 1 | 0 | 1 | 0 | 0 | 1 | 0 | 0 | 1 | 0 | 0 | 0 | 0 | 0 | 0 | 1 | 0 | | | | | | |
| 3017 | 03559 | 03016 | 1 | | 0 | 0 | 0 | 0 | 0 | 0 | 0 | 0 | 1 | 0 | 0 | 0 | 0 | 0 | 0 | 0 | 0 | 0 | 0 | 0 | 0 | 0 | 0 | 0 | 0 | | | | | | |
| 3018 | 03560 | 03017 | 4 | | 0 | 0 | 0 | 0 | 0 | 0 | 0 | 0 | 1 | 0 | 0 | 0 | 0 | 0 | 0 | 1 | 1 | 1 | 0 | 0 | 0 | 0 | 0 | 0 | 0 | | | | | | |
| 3019 | 03561 | 03018 | 1 | | 0 | 0 | 0 | 0 | 0 | 0 | 0 | 0 | 1 | 0 | 0 | 0 | 0 | 0 | 0 | 0 | 0 | 0 | 0 | 0 | 0 | 0 | 0 | 0 | 0 | | | | | | |
| 3020 | 03562 | 03019 | 1 | | 0 | 0 | 0 | 0 | 0 | 0 | 0 | 0 | 1 | 0 | 0 | 0 | 0 | 0 | 0 | 0 | 0 | 0 | 0 | 0 | 0 | 0 | 0 | 0 | 0 | | | | | | |
| 3021 | 03563 | 03020 | 1 | | 0 | 0 | 0 | 0 | 0 | 0 | 0 | 0 | 1 | 0 | 0 | 0 | 0 | 0 | 0 | 0 | 0 | 0 | 0 | 0 | 0 | 0 | 0 | 0 | 0 | | | | | | |
| 3022 | 03564 | 03021 | 11 | | 0 | 0 | 0 | 0 | 0 | 0 | 0 | 0 | 1 | 0 | 2 | 0 | 0 | 0 | 0 | 0 | 1 | 0 | 2 | 0 | 1 | 1 | 0 | 2 | 1 | | | | | | |
| 3023 | 03565 | 03022 | 1 | | 0 | 0 | 0 | 0 | 0 | 0 | 0 | 0 | 1 | 0 | 0 | 0 | 0 | 0 | 0 | 0 | 0 | 0 | 0 | 0 | 0 | 0 | 0 | 0 | 0 | | | | | | |
| 3024 | 03566 | 03023 | 1 | | 0 | 0 | 0 | 0 | 0 | 0 | 0 | 0 | 1 | 0 | 0 | 0 | 0 | 0 | 0 | 0 | 0 | 0 | 0 | 0 | 0 | 0 | 0 | 0 | 0 | M22637 | 100 | 127 | 1 | 1222 | 1348 |
| 3025 | 03567 | 03024 | 3 | | 0 | 0 | 0 | 0 | 0 | 0 | 0 | 0 | 1 | 0 | 0 | 0 | 0 | 0 | 0 | 0 | 0 | 1 | 0 | 0 | 0 | 0 | 0 | 1 | 0 | | | | | | |
| 3026 | 03568 | 03025 | 2 | | 0 | 0 | 0 | 0 | 0 | 0 | 0 | 0 | 1 | 0 | 1 | 0 | 0 | 0 | 0 | 0 | 0 | 0 | 0 | 0 | 0 | 0 | 0 | 0 | 0 | | | | | | |
| 3027 | 03569 | 03026 | 1 | | 0 | 0 | 0 | 0 | 0 | 0 | 0 | 0 | 1 | 0 | 0 | 0 | 0 | 0 | 0 | 0 | 0 | 0 | 0 | 0 | 0 | 0 | 0 | 0 | 0 | | | | | | |
| 3028 | 03570 | 03027 | 4 | | 0 | 0 | 0 | 0 | 0 | 0 | 0 | 0 | 1 | 0 | 1 | 0 | 0 | 1 | 1 | 0 | 0 | 0 | 0 | 0 | 0 | 0 | 0 | 0 | 0 | D23662 | 98.4 | 123 | 1 | 483 | 605 |
| 3029 | 03571 | 03028 | 1 | | 0 | 0 | 0 | 0 | 0 | 0 | 0 | 0 | 1 | 0 | 0 | 0 | 0 | 0 | 0 | 0 | 0 | 0 | 0 | 0 | 0 | 0 | 0 | 0 | 0 | | | | | | |
| 3030 | 03572 | 03029 | 3 | | 0 | 0 | 0 | 0 | 0 | 0 | 0 | 0 | 2 | 0 | 0 | 0 | 1 | 0 | 0 | 0 | 0 | 0 | 0 | 0 | 0 | 0 | 0 | 0 | 0 | | | | | | |
| 3031 | 03573 | 03030 | 9 | | 0 | 0 | 0 | 0 | 0 | 0 | 0 | 0 | 1 | 0 | 0 | 0 | 0 | 3 | 0 | 0 | 0 | 0 | 4 | 1 | 0 | 0 | 0 | 0 | 0 | J04615 | 99.2 | 129 | 1 | 1198 | 1326 |
| 3032 | 03574 | 03031 | 1 | | 0 | 0 | 0 | 0 | 0 | 0 | 0 | 0 | 1 | 0 | 0 | 0 | 0 | 0 | 0 | 0 | 0 | 0 | 0 | 0 | 0 | 0 | 0 | 0 | 0 | | | | | | |
| 3033 | 03575 | 03032 | 17 | | 0 | 0 | 0 | 0 | 0 | 0 | 0 | 0 | 1 | 0 | 1 | 0 | 0 | 0 | 0 | 0 | 2 | 0 | 0 | 2 | 0 | 0 | 3 | 8 | 0 | | | | | | |
| 3034 | 03576 | 03033 | 3 | | 0 | 0 | 0 | 0 | 0 | 0 | 0 | 0 | 1 | 0 | 0 | 0 | 0 | 0 | 0 | 1 | 0 | 0 | 0 | 1 | 0 | 0 | 0 | 0 | 0 | | | | | | |
| 3035 | 03577 | 03034 | 5 | | 0 | 0 | 0 | 0 | 0 | 0 | 0 | 0 | 1 | 0 | 0 | 0 | 0 | 0 | 4 | 0 | 0 | 0 | 0 | 0 | 0 | 0 | 0 | 0 | 0 | | | | | | |
| 3036 | 03578 | 03035 | 3 | | 0 | 0 | 0 | 0 | 0 | 0 | 0 | 0 | 1 | 0 | 0 | 0 | 0 | 0 | 0 | 0 | 0 | 0 | 0 | 2 | 0 | 0 | 0 | 0 | 0 | | | | | | |
| 3037 | 03579 | 03036 | 1 | | 0 | 0 | 0 | 0 | 0 | 0 | 0 | 0 | 1 | 0 | 0 | 0 | 0 | 0 | 0 | 0 | 0 | 0 | 0 | 0 | 0 | 0 | 0 | 0 | 0 | M61764 | 94.4 | 108 | 1 | 1460 | 1568 |
| 3038 | 03580 | 03037 | 3 | | 0 | 0 | 0 | 0 | 0 | 0 | 0 | 0 | 2 | 0 | 0 | 0 | 0 | 0 | 0 | 0 | 1 | 0 | 0 | 0 | 0 | 0 | 0 | 0 | 0 | | | | | | |
| 3039 | 03581 | 03038 | 1 | | 0 | 0 | 0 | 0 | 0 | 0 | 0 | 0 | 1 | 0 | 0 | 0 | 0 | 0 | 0 | 0 | 0 | 0 | 0 | 0 | 0 | 0 | 0 | 0 | 0 | | | | | | |
| 3040 | 03582 | 03039 | 6 | | 0 | 0 | 0 | 0 | 0 | 0 | 0 | 0 | 1 | 0 | 0 | 0 | 0 | 0 | 0 | 0 | 0 | 0 | 0 | 0 | 0 | 5 | 0 | 0 | 0 | | | | | | |
| 3041 | 03583 | 03040 | 4 | | 0 | 0 | 0 | 0 | 0 | 0 | 0 | 0 | 2 | 0 | 0 | 0 | 0 | 0 | 1 | 1 | 0 | 0 | 0 | 0 | 0 | 0 | 0 | 0 | 0 | X04385 | 95.4 | 131 | 1 | 8070 | 8575 |
| 3042 | 03584 | 03041 | 1 | | 0 | 0 | 0 | 0 | 0 | 0 | 0 | 0 | 1 | 0 | 0 | 0 | 0 | 0 | 0 | 0 | 0 | 0 | 0 | 0 | 0 | 0 | 0 | 0 | 0 | J03171 | 100 | 115 | 1 | 2641 | 2755 |
| 3043 | 03585 | 03042 | 1 | | 0 | 0 | 0 | 0 | 0 | 0 | 0 | 0 | 1 | 0 | 0 | 0 | 0 | 0 | 0 | 0 | 0 | 0 | 0 | 0 | 0 | 0 | 0 | 0 | 0 | | | | | | |
| 3044 | 03586 | 03043 | 1 | | 0 | 0 | 0 | 0 | 0 | 0 | 0 | 0 | 1 | 0 | 0 | 0 | 0 | 0 | 0 | 0 | 0 | 0 | 0 | 0 | 0 | 0 | 0 | 0 | 0 | | | | | | |
| 3045 | 03587 | 03044 | 2 | | 0 | 0 | 0 | 0 | 0 | 0 | 0 | 0 | 1 | 0 | 0 | 0 | 0 | 0 | 0 | 1 | 0 | 0 | 0 | 0 | 0 | 0 | 0 | 0 | 0 | | | | | | |

Table 85

| | A | B | C | E | G | I | K | M | O | Q | S | U | W | Y | AA | AC | AE | AG | AI | AK | AM | AO | AQ | AS | AU | AW | AY | BA | BC | BE | BF | BG | BH | BI | BK |
|---|---|---|---|---|---|---|---|---|---|---|---|---|---|---|---|---|---|---|---|---|---|---|---|---|---|---|---|---|---|---|---|---|---|---|---|
| 3046 | 03588 | 03045 | 1 | | 0 | 0 | 0 | 0 | 0 | 0 | 0 | 0 | 1 | 0 | 0 | 0 | 0 | 0 | 0 | 0 | 0 | 0 | 0 | 0 | 0 | 0 | 0 | 0 | 0 | | | | | | |
| 3047 | 03589 | 03046 | 2 | | 0 | 0 | 0 | 0 | 0 | 0 | 0 | 0 | 1 | 0 | 0 | 0 | 0 | 0 | 1 | 0 | 0 | 0 | 0 | 0 | 0 | 0 | 0 | 0 | 0 | | | | | | |
| 3048 | 03590 | 03047 | 1 | | 0 | 0 | 0 | 0 | 0 | 0 | 0 | 0 | 1 | 0 | 0 | 0 | 0 | 0 | 0 | 0 | 0 | 0 | 0 | 0 | 0 | 0 | 0 | 0 | 0 | | | | | | |
| 3049 | 03591 | 03048 | 9 | | 0 | 0 | 0 | 0 | 0 | 0 | 0 | 0 | 2 | 0 | 0 | 0 | 1 | 1 | 0 | 2 | 0 | 2 | 0 | 0 | 0 | 0 | 0 | 1 | 0 | L06328 | 97.8 | 179 | 1 | 1061 | 1404 |
| 3050 | 03592 | 03049 | 2 | | 0 | 0 | 0 | 0 | 0 | 0 | 0 | 0 | 1 | 0 | 0 | 0 | 1 | 0 | 0 | 0 | 0 | 0 | 0 | 0 | 0 | 0 | 0 | 0 | 0 | X59543 | 97.1 | 238 | 1 | 2559 | 3075 |
| 3051 | 03593 | 03050 | 1 | | 0 | 0 | 0 | 0 | 0 | 0 | 0 | 0 | 1 | 0 | 0 | 0 | 0 | 0 | 0 | 0 | 0 | 0 | 0 | 0 | 0 | 0 | 0 | 0 | 0 | | | | | | |
| 3052 | 03594 | 03051 | 1 | | 0 | 0 | 0 | 0 | 0 | 0 | 0 | 0 | 1 | 0 | 0 | 0 | 0 | 0 | 0 | 0 | 0 | 0 | 0 | 0 | 0 | 0 | 0 | 0 | 0 | | | | | | |
| 3053 | 03595 | 03052 | 1 | | 0 | 0 | 0 | 0 | 0 | 0 | 0 | 0 | 1 | 0 | 0 | 0 | 0 | 0 | 0 | 0 | 0 | 0 | 0 | 0 | 0 | 0 | 0 | 0 | 0 | | | | | | |
| 3054 | 03596 | 03053 | 4 | | 0 | 0 | 0 | 0 | 0 | 0 | 0 | 0 | 1 | 0 | 0 | 0 | 0 | 0 | 1 | 0 | 0 | 0 | 0 | 0 | 0 | 0 | 2 | 0 | 0 | M77693 | 100 | 159 | 1 | 639 | 1060 |
| 3055 | 03597 | 03054 | 1 | | 0 | 0 | 0 | 0 | 0 | 0 | 0 | 0 | 1 | 0 | 0 | 0 | 0 | 0 | 0 | 0 | 0 | 0 | 0 | 0 | 0 | 0 | 0 | 0 | 0 | | | | | | |
| 3056 | 03598 | 03055 | 1 | | 0 | 0 | 0 | 0 | 0 | 0 | 0 | 0 | 1 | 0 | 0 | 0 | 0 | 0 | 0 | 0 | 0 | 0 | 0 | 0 | 0 | 0 | 0 | 0 | 0 | | | | | | |
| 3057 | 03599 | 03056 | 1 | | 0 | 0 | 0 | 0 | 0 | 0 | 0 | 0 | 1 | 0 | 0 | 0 | 0 | 0 | 0 | 0 | 0 | 0 | 0 | 0 | 0 | 0 | 0 | 0 | 0 | K03199 | 98.3 | 58 | 52 | 8 | 1760 |
| 3058 | 03600 | 03057 | 1 | | 0 | 0 | 0 | 0 | 0 | 0 | 0 | 0 | 1 | 0 | 0 | 0 | 0 | 0 | 0 | 0 | 0 | 0 | 0 | 0 | 0 | 0 | 0 | 0 | 0 | | | | | | |
| 3059 | 03602 | 03058 | 1 | | 0 | 0 | 0 | 0 | 0 | 0 | 0 | 0 | 1 | 0 | 0 | 0 | 0 | 0 | 0 | 0 | 0 | 0 | 0 | 0 | 0 | 0 | 0 | 0 | 0 | | | | | | |
| 3060 | 03603 | 03059 | 1 | | 0 | 0 | 0 | 0 | 0 | 0 | 0 | 0 | 1 | 0 | 0 | 0 | 0 | 0 | 0 | 0 | 0 | 0 | 0 | 0 | 0 | 0 | 0 | 0 | 0 | | | | | | |
| 3061 | 03604 | 03060 | 1 | | 0 | 0 | 0 | 0 | 0 | 0 | 0 | 0 | 1 | 0 | 0 | 0 | 0 | 0 | 0 | 0 | 0 | 0 | 0 | 0 | 0 | 0 | 0 | 0 | 0 | | | | | | |
| 3062 | 03605 | 03061 | 3 | | 0 | 0 | 0 | 0 | 0 | 0 | 0 | 0 | 1 | 0 | 0 | 0 | 0 | 0 | 0 | 1 | 0 | 0 | 0 | 0 | 0 | 0 | 0 | 1 | 0 | | | | | | |
| 3063 | 03606 | 03062 | 1 | | 0 | 0 | 0 | 0 | 0 | 0 | 0 | 0 | 1 | 0 | 0 | 0 | 0 | 0 | 0 | 0 | 0 | 0 | 0 | 0 | 0 | 0 | 0 | 0 | 0 | | | | | | |
| 3064 | 03607 | 03063 | 1 | | 0 | 0 | 0 | 0 | 0 | 0 | 0 | 0 | 1 | 0 | 0 | 0 | 0 | 0 | 0 | 0 | 0 | 0 | 0 | 0 | 0 | 0 | 0 | 0 | 0 | | | | | | |
| 3065 | 03608 | 03064 | 4 | | 0 | 0 | 0 | 0 | 0 | 0 | 0 | 0 | 1 | 0 | 0 | 0 | 0 | 0 | 2 | 1 | 0 | 0 | 0 | 0 | 0 | 0 | 0 | 0 | 0 | | | | | | |
| 3066 | 03609 | 03065 | 1 | | 0 | 0 | 0 | 0 | 0 | 0 | 0 | 0 | 1 | 0 | 0 | 0 | 0 | 0 | 0 | 0 | 0 | 0 | 0 | 0 | 0 | 0 | 0 | 0 | 0 | | | | | | |
| 3067 | 03610 | 03066 | 3 | | 0 | 0 | 0 | 0 | 0 | 0 | 0 | 0 | 1 | 0 | 0 | 0 | 0 | 0 | 1 | 1 | 0 | 0 | 0 | 0 | 0 | 0 | 0 | 0 | 0 | M12783 | 93.4 | 182 | 1 | 3284 | 3798 |
| 3068 | 03611 | 03067 | 2 | | 0 | 0 | 0 | 0 | 0 | 0 | 0 | 0 | 1 | 0 | 0 | 0 | 0 | 0 | 0 | 0 | 0 | 0 | 0 | 0 | 0 | 0 | 1 | 0 | 0 | | | | | | |
| 3069 | 03612 | 03068 | 1 | | 0 | 0 | 0 | 0 | 0 | 0 | 0 | 0 | 1 | 0 | 0 | 0 | 0 | 0 | 0 | 0 | 0 | 0 | 0 | 0 | 0 | 0 | 0 | 0 | 0 | | | | | | |
| 3070 | 03613 | 03069 | 1 | | 0 | 0 | 0 | 0 | 0 | 0 | 0 | 0 | 1 | 0 | 0 | 0 | 0 | 0 | 0 | 0 | 0 | 0 | 0 | 0 | 0 | 0 | 0 | 0 | 0 | | | | | | |
| 3071 | 03614 | 03070 | 2 | | 0 | 0 | 0 | 0 | 0 | 0 | 0 | 0 | 1 | 1 | 0 | 0 | 0 | 0 | 0 | 0 | 0 | 0 | 0 | 0 | 0 | 0 | 0 | 0 | 0 | | | | | | |
| 3072 | 03615 | 03071 | 2 | | 0 | 0 | 0 | 0 | 0 | 0 | 0 | 0 | 2 | 0 | 0 | 0 | 0 | 0 | 0 | 0 | 0 | 0 | 0 | 0 | 0 | 0 | 0 | 0 | 0 | | | | | | |
| 3073 | 03616 | 03072 | 4 | | 0 | 0 | 0 | 0 | 0 | 0 | 0 | 0 | 2 | 0 | 0 | 0 | 0 | 0 | 0 | 0 | 0 | 0 | 1 | 1 | 0 | 0 | 0 | 0 | 0 | | | | | | |
| 3074 | 03617 | 03073 | 1 | | 0 | 0 | 0 | 0 | 0 | 0 | 0 | 0 | 1 | 0 | 0 | 0 | 0 | 0 | 0 | 0 | 0 | 0 | 0 | 0 | 0 | 0 | 0 | 0 | 0 | | | | | | |
| 3075 | 03618 | 03074 | 2 | | 0 | 0 | 0 | 0 | 0 | 0 | 0 | 0 | 1 | 1 | 0 | 0 | 0 | 0 | 0 | 0 | 0 | 0 | 0 | 0 | 0 | 0 | 0 | 0 | 0 | | | | | | |
| 3076 | 03619 | 03075 | 3 | | 0 | 0 | 0 | 0 | 0 | 0 | 0 | 0 | 2 | 0 | 0 | 0 | 0 | 0 | 0 | 0 | 0 | 1 | 0 | 0 | 0 | 0 | 0 | 0 | 0 | | | | | | |
| 3077 | 03620 | 03076 | 1 | | 0 | 0 | 0 | 0 | 0 | 0 | 0 | 0 | 1 | 0 | 0 | 0 | 0 | 0 | 0 | 0 | 0 | 0 | 0 | 0 | 0 | 0 | 0 | 0 | 0 | | | | | | |
| 3078 | 03621 | 03077 | 2 | | 0 | 0 | 0 | 0 | 0 | 0 | 0 | 0 | 1 | 0 | 0 | 0 | 0 | 0 | 0 | 0 | 0 | 0 | 0 | 0 | 0 | 1 | 0 | 0 | 0 | | | | | | |
| 3079 | 03622 | 03078 | 2 | | 0 | 0 | 0 | 0 | 0 | 0 | 0 | 0 | 1 | 0 | 1 | 0 | 0 | 0 | 0 | 0 | 0 | 0 | 0 | 0 | 0 | 0 | 0 | 0 | 0 | | | | | | |
| 3080 | 03623 | 03079 | 5 | | 0 | 0 | 0 | 0 | 0 | 0 | 0 | 0 | 1 | 0 | 0 | 0 | 0 | 0 | 0 | 0 | 1 | 3 | 0 | 0 | 0 | 0 | 0 | 0 | 0 | M11718 | 95 | 238 | 1 | 920 | 1317 |
| 3081 | 03624 | 03080 | 2 | | 0 | 0 | 0 | 0 | 0 | 0 | 0 | 0 | 1 | 0 | 0 | 0 | 0 | 0 | 0 | 0 | 0 | 0 | 1 | 0 | 0 | 0 | 0 | 0 | 0 | | | | | | |

Table 86

| | A | B | C | E | G | I | K | M | O | Q | S | U | W | Y | AA | AC | AE | AG | AI | AK | AM | AO | AQ | AS | AU | AW | AY | BA | BC | BE | BF | BG | BH | BI | BK |
|---|---|---|---|---|---|---|---|---|---|---|---|---|---|---|---|---|---|---|---|---|---|---|---|---|---|---|---|---|---|---|---|---|---|---|---|
| 3082 | 03625 | 03081 | 2 | | 0 | 0 | 0 | 0 | 0 | 0 | 0 | 0 | 1 | 0 | 0 | 0 | 0 | 0 | 0 | 0 | 0 | 0 | 0 | 0 | 1 | 0 | 0 | 0 | 0 | | | | | | |
| 3083 | 03626 | 03082 | 2 | | 0 | 0 | 0 | 0 | 0 | 0 | 0 | 0 | 1 | 0 | 0 | 0 | 0 | 0 | 1 | 0 | 0 | 0 | 0 | 0 | 0 | 0 | 0 | 0 | 0 | | | | | | |
| 3084 | 03627 | 03083 | 5 | | 0 | 0 | 0 | 0 | 0 | 0 | 0 | 0 | 1 | 0 | 0 | 2 | 2 | 0 | 0 | 0 | 0 | 0 | 0 | 0 | 0 | 0 | 0 | 0 | 0 | X63564 | 100 | 86 | 1 | 6647 | 6732 |
| 3085 | 03628 | 03084 | 2 | | 0 | 0 | 0 | 0 | 0 | 0 | 0 | 0 | 2 | 0 | 0 | 0 | 0 | 0 | 0 | 0 | 0 | 0 | 0 | 0 | 0 | 0 | 0 | 0 | 0 | | | | | | |
| 3086 | 03629 | 03085 | 9 | | 0 | 0 | 0 | 0 | 0 | 0 | 0 | 0 | 1 | 1 | 1 | 0 | 0 | 1 | 2 | 0 | 1 | 0 | 0 | 1 | 0 | 0 | 1 | 0 | 0 | | | | | | |
| 3087 | 03631 | 03086 | 3 | | 0 | 0 | 0 | 0 | 0 | 0 | 0 | 0 | 1 | 0 | 0 | 0 | 0 | 1 | 0 | 0 | 0 | 0 | 1 | 0 | 0 | 0 | 0 | 0 | 0 | | | | | | |
| 3088 | 03632 | 03087 | 1 | | 0 | 0 | 0 | 0 | 0 | 0 | 0 | 0 | 1 | 0 | 0 | 0 | 0 | 0 | 0 | 0 | 0 | 0 | 0 | 0 | 0 | 0 | 0 | 0 | 0 | | | | | | |
| 3089 | 03633 | 03088 | 2 | | 0 | 0 | 0 | 0 | 0 | 0 | 0 | 0 | 1 | 0 | 0 | 0 | 0 | 0 | 0 | 0 | 0 | 0 | 0 | 0 | 1 | 0 | 0 | 0 | 0 | | | | | | |
| 3090 | 03634 | 03089 | 8 | | 0 | 0 | 0 | 0 | 0 | 0 | 0 | 0 | 1 | 2 | 0 | 0 | 0 | 0 | 1 | 0 | 0 | 1 | 3 | 0 | 0 | 0 | 0 | 0 | 0 | | | | | | |
| 3091 | 03635 | 03090 | 3 | | 0 | 0 | 0 | 0 | 0 | 0 | 0 | 0 | 2 | 0 | 0 | 0 | 0 | 0 | 0 | 0 | 0 | 1 | 0 | 0 | 0 | 0 | 0 | 0 | 0 | | | | | | |
| 3092 | 03636 | 03091 | 1 | | 0 | 0 | 0 | 0 | 0 | 0 | 0 | 0 | 1 | 0 | 0 | 0 | 0 | 0 | 0 | 0 | 0 | 0 | 0 | 0 | 0 | 0 | 0 | 0 | 0 | | | | | | |
| 3093 | 03637 | 03092 | 1 | | 0 | 0 | 0 | 0 | 0 | 0 | 0 | 0 | 1 | 0 | 0 | 0 | 0 | 0 | 0 | 0 | 0 | 0 | 0 | 0 | 0 | 0 | 0 | 0 | 0 | | | | | | |
| 3094 | 03638 | 03093 | 1 | | 0 | 0 | 0 | 0 | 0 | 0 | 0 | 0 | 1 | 0 | 0 | 0 | 0 | 0 | 0 | 0 | 0 | 0 | 0 | 0 | 0 | 0 | 0 | 0 | 0 | | | | | | |
| 3095 | 03639 | 03094 | 2 | | 0 | 0 | 0 | 0 | 0 | 0 | 0 | 0 | 1 | 0 | 0 | 0 | 0 | 0 | 0 | 0 | 0 | 0 | 0 | 0 | 0 | 1 | 0 | 0 | 0 | | | | | | |
| 3096 | 03640 | 03095 | 2 | | 0 | 0 | 0 | 0 | 0 | 0 | 0 | 0 | 1 | 0 | 0 | 0 | 0 | 0 | 0 | 0 | 0 | 0 | 1 | 0 | 0 | 0 | 0 | 0 | 0 | | | | | | |
| 3097 | 03641 | 03096 | 2 | | 0 | 0 | 0 | 0 | 0 | 0 | 0 | 0 | 1 | 0 | 0 | 0 | 0 | 0 | 0 | 0 | 0 | 0 | 0 | 0 | 0 | 1 | 0 | 0 | 0 | | | | | | |
| 3098 | 03642 | 03097 | 2 | | 0 | 0 | 0 | 0 | 0 | 0 | 0 | 0 | 1 | 0 | 0 | 0 | 0 | 0 | 0 | 0 | 0 | 0 | 0 | 1 | 0 | 0 | 0 | 0 | 0 | | | | | | |
| 3099 | 03643 | 03098 | 2 | | 0 | 0 | 0 | 0 | 0 | 0 | 0 | 0 | 1 | 0 | 0 | 0 | 0 | 0 | 0 | 0 | 0 | 0 | 0 | 1 | 0 | 0 | 0 | 0 | 0 | | | | | | |
| 3100 | 03644 | 03099 | 1 | | 0 | 0 | 0 | 0 | 0 | 0 | 0 | 0 | 1 | 0 | 0 | 0 | 0 | 0 | 0 | 0 | 0 | 0 | 0 | 0 | 0 | 0 | 0 | 0 | 0 | | | | | | |
| 3101 | 03645 | 03100 | 2 | | 0 | 0 | 0 | 0 | 0 | 0 | 0 | 0 | 1 | 0 | 0 | 0 | 0 | 0 | 0 | 0 | 0 | 0 | 1 | 0 | 0 | 0 | 0 | 0 | 0 | | | | | | |
| 3102 | 03646 | 03101 | 1 | | 0 | 0 | 0 | 0 | 0 | 0 | 0 | 0 | 1 | 0 | 0 | 0 | 0 | 0 | 0 | 0 | 0 | 0 | 0 | 0 | 0 | 0 | 0 | 0 | 0 | | | | | | |
| 3103 | 03647 | 03102 | 1 | | 0 | 0 | 0 | 0 | 0 | 0 | 0 | 0 | 1 | 0 | 0 | 0 | 0 | 0 | 0 | 0 | 0 | 0 | 0 | 0 | 0 | 0 | 0 | 0 | 0 | | | | | | |
| 3104 | 03648 | 03103 | 1 | | 0 | 0 | 0 | 0 | 0 | 0 | 0 | 0 | 1 | 0 | 0 | 0 | 0 | 0 | 0 | 0 | 0 | 0 | 0 | 0 | 0 | 0 | 0 | 0 | 0 | | | | | | |
| 3105 | 03649 | 03104 | 8 | | 0 | 0 | 0 | 0 | 0 | 0 | 0 | 0 | 2 | 2 | 0 | 1 | 0 | 0 | 1 | 0 | 0 | 1 | 0 | 0 | 1 | 0 | 0 | 0 | 0 | | | | | | |
| 3106 | 03650 | 03105 | 1 | | 0 | 0 | 0 | 0 | 0 | 0 | 0 | 0 | 1 | 0 | 0 | 0 | 0 | 0 | 0 | 0 | 0 | 0 | 0 | 0 | 0 | 0 | 0 | 0 | 0 | | | | | | |
| 3107 | 03651 | 03106 | 3 | | 0 | 0 | 0 | 0 | 0 | 0 | 0 | 0 | 1 | 0 | 0 | 0 | 2 | 0 | 0 | 0 | 0 | 0 | 0 | 0 | 0 | 0 | 0 | 0 | 0 | | | | | | |
| 3108 | 03652 | 03107 | 1 | | 0 | 0 | 0 | 0 | 0 | 0 | 0 | 0 | 1 | 0 | 0 | 0 | 0 | 0 | 0 | 0 | 0 | 0 | 0 | 0 | 0 | 0 | 0 | 0 | 0 | | | | | | |
| 3109 | 03653 | 03108 | 1 | | 0 | 0 | 0 | 0 | 0 | 0 | 0 | 0 | 1 | 0 | 0 | 0 | 0 | 0 | 0 | 0 | 0 | 0 | 0 | 0 | 0 | 0 | 0 | 0 | 0 | | | | | | |
| 3110 | 03654 | 03109 | 3 | | 0 | 0 | 0 | 0 | 0 | 0 | 0 | 0 | 1 | 1 | 0 | 0 | 0 | 0 | 0 | 0 | 0 | 0 | 0 | 0 | 1 | 0 | 0 | 0 | 0 | | | | | | |
| 3111 | 03655 | 03110 | 3 | | 0 | 0 | 0 | 0 | 0 | 0 | 0 | 0 | 1 | 0 | 0 | 0 | 0 | 0 | 0 | 1 | 0 | 0 | 0 | 0 | 0 | 1 | 0 | 0 | 0 | | | | | | |
| 3112 | 03659 | 03111 | 6 | | 0 | 0 | 0 | 0 | 0 | 0 | 0 | 0 | 1 | 0 | 1 | 0 | 0 | 0 | 0 | 0 | 0 | 0 | 1 | 0 | 1 | 0 | 2 | 0 | 0 | | | | | | |
| 3113 | 03661 | 03112 | 2 | | 0 | 0 | 0 | 0 | 0 | 0 | 0 | 0 | 1 | 0 | 0 | 0 | 0 | 0 | 0 | 0 | 1 | 0 | 0 | 0 | 0 | 0 | 0 | 0 | 0 | | | | | | |
| 3114 | 03679 | 03113 | 1 | | 0 | 0 | 0 | 0 | 0 | 0 | 0 | 0 | 0 | 1 | 0 | 0 | 0 | 0 | 0 | 0 | 0 | 0 | 0 | 0 | 0 | 0 | 0 | 0 | 0 | | | | | | |
| 3115 | 03680 | 03114 | 1 | | 0 | 0 | 0 | 0 | 0 | 0 | 0 | 0 | 0 | 1 | 0 | 0 | 0 | 0 | 0 | 0 | 0 | 0 | 0 | 0 | 0 | 0 | 0 | 0 | 0 | | | | | | |
| 3116 | 03681 | 03115 | 3 | | 0 | 0 | 0 | 0 | 0 | 0 | 0 | 0 | 0 | 2 | 0 | 0 | 0 | 0 | 0 | 0 | 0 | 0 | 0 | 0 | 0 | 1 | 0 | 0 | 0 | X62320 | 95.1 | 430 | 1 | 1700 | 2152 |
| 3117 | 03682 | 03116 | 1 | | 0 | 0 | 0 | 0 | 0 | 0 | 0 | 0 | 0 | 1 | 0 | 0 | 0 | 0 | 0 | 0 | 0 | 0 | 0 | 0 | 0 | 0 | 0 | 0 | 0 | | | | | | |

Table 87

| | A | B | C | E | G | I | K | M | O | Q | S | U | W | Y | AA | AC | AE | AG | AI | AK | AM | AO | AQ | AS | AU | AW | AY | BA | BC | BE | BF | BG | BH | BI | BK |
|---|---|---|---|---|---|---|---|---|---|---|---|---|---|---|---|---|---|---|---|---|---|---|---|---|---|---|---|---|---|---|---|---|---|---|---|
| 3118 | 03683 | 03117 | 1 | | 0 | 0 | 0 | 0 | 0 | 0 | 0 | 0 | 0 | 1 | 0 | 0 | 0 | 0 | 0 | 0 | 0 | 0 | 0 | 0 | 0 | 0 | 0 | 0 | 0 | | | | | | |
| 3119 | 03684 | 03118 | 2 | | 0 | 0 | 0 | 0 | 0 | 0 | 0 | 0 | 0 | 1 | 1 | 0 | 0 | 0 | 0 | 0 | 0 | 0 | 0 | 0 | 0 | 0 | 0 | 0 | 0 | | | | | | |
| 3120 | 03685 | 03119 | 1 | | 0 | 0 | 0 | 0 | 0 | 0 | 0 | 0 | 0 | 1 | 0 | 0 | 0 | 0 | 0 | 0 | 0 | 0 | 0 | 0 | 0 | 0 | 0 | 0 | 0 | | | | | | |
| 3121 | 03686 | 03120 | 1 | | 0 | 0 | 0 | 0 | 0 | 0 | 0 | 0 | 0 | 1 | 0 | 0 | 0 | 0 | 0 | 0 | 0 | 0 | 0 | 0 | 0 | 0 | 0 | 0 | 0 | | | | | | |
| 3122 | 03688 | 03121 | 1 | | 0 | 0 | 0 | 0 | 0 | 0 | 0 | 0 | 0 | 1 | 0 | 0 | 0 | 0 | 0 | 0 | 0 | 0 | 0 | 0 | 0 | 0 | 0 | 0 | 0 | | | | | | |
| 3123 | 03689 | 03122 | 5 | | 0 | 0 | 0 | 0 | 0 | 0 | 0 | 0 | 0 | 1 | 1 | 0 | 0 | 0 | 0 | 0 | 0 | 1 | 0 | 1 | 1 | 0 | 0 | 0 | 0 | | | | | | |
| 3124 | 03690 | 03123 | 4 | | 0 | 0 | 0 | 0 | 0 | 0 | 0 | 0 | 0 | 2 | 0 | 0 | 0 | 0 | 0 | 0 | 0 | 1 | 0 | 1 | 0 | 0 | 0 | 0 | 0 | J04164 | 97.3 | 300 | 1 | 388 | 853 |
| 3125 | 03691 | 03124 | 3 | | 0 | 0 | 0 | 0 | 0 | 0 | 0 | 0 | 0 | 2 | 0 | 0 | 0 | 0 | 1 | 0 | 0 | 0 | 0 | 0 | 0 | 0 | 0 | 0 | 0 | | | | | | |
| 3126 | 03693 | 03125 | 1 | | 0 | 0 | 0 | 0 | 0 | 0 | 0 | 0 | 0 | 1 | 0 | 0 | 0 | 0 | 0 | 0 | 0 | 0 | 0 | 0 | 0 | 0 | 0 | 0 | 0 | | | | | | |
| 3127 | 03694 | 03126 | 10 | | 0 | 0 | 0 | 0 | 0 | 0 | 0 | 0 | 0 | 1 | 0 | 1 | 0 | 1 | 0 | 0 | 0 | 0 | 1 | 1 | 4 | 0 | 0 | 1 | 0 | | | | | | |
| 3128 | 03695 | 03127 | 1 | | 0 | 0 | 0 | 0 | 0 | 0 | 0 | 0 | 0 | 1 | 0 | 0 | 0 | 0 | 0 | 0 | 0 | 0 | 0 | 0 | 0 | 0 | 0 | 0 | 0 | | | | | | |
| 3129 | 03696 | 03128 | 1 | | 0 | 0 | 0 | 0 | 0 | 0 | 0 | 0 | 0 | 1 | 0 | 0 | 0 | 0 | 0 | 0 | 0 | 0 | 0 | 0 | 0 | 0 | 0 | 0 | 0 | | | | | | |
| 3130 | 03697 | 03129 | 1 | | 0 | 0 | 0 | 0 | 0 | 0 | 0 | 0 | 0 | 1 | 0 | 0 | 0 | 0 | 0 | 0 | 0 | 0 | 0 | 0 | 0 | 0 | 0 | 0 | 0 | | | | | | |
| 3131 | 03698 | 03130 | 13 | | 0 | 0 | 0 | 0 | 0 | 0 | 0 | 0 | 0 | 1 | 2 | 0 | 0 | 0 | 2 | 1 | 4 | 1 | 1 | 0 | 0 | 1 | 0 | 0 | 0 | | | | | | |
| 3132 | 03699 | 03131 | 1 | | 0 | 0 | 0 | 0 | 0 | 0 | 0 | 0 | 0 | 1 | 0 | 0 | 0 | 0 | 0 | 0 | 0 | 0 | 0 | 0 | 0 | 0 | 0 | 0 | 0 | | | | | | |
| 3133 | 03701 | 03132 | 1 | | 0 | 0 | 0 | 0 | 0 | 0 | 0 | 0 | 0 | 1 | 0 | 0 | 0 | 0 | 0 | 0 | 0 | 0 | 0 | 0 | 0 | 0 | 0 | 0 | 0 | X71973 | 97.8 | 358 | 1 | 524 | 896 |
| 3134 | 03702 | 03133 | 1 | | 0 | 0 | 0 | 0 | 0 | 0 | 0 | 0 | 0 | 1 | 0 | 0 | 0 | 0 | 0 | 0 | 0 | 0 | 0 | 0 | 0 | 0 | 0 | 0 | 0 | | | | | | |
| 3135 | 03703 | 03134 | 2 | | 0 | 0 | 0 | 0 | 0 | 0 | 0 | 0 | 0 | 2 | 0 | 0 | 0 | 0 | 0 | 0 | 0 | 0 | 0 | 0 | 0 | 0 | 0 | 0 | 0 | | | | | | |
| 3136 | 03704 | 03135 | 3 | | 0 | 0 | 0 | 0 | 0 | 0 | 0 | 0 | 0 | 1 | 0 | 0 | 0 | 0 | 0 | 0 | 0 | 0 | 0 | 1 | 0 | 0 | 0 | 1 | 0 | | | | | | |
| 3137 | 03705 | 03136 | 3 | | 0 | 0 | 0 | 0 | 0 | 0 | 0 | 0 | 0 | 1 | 0 | 0 | 0 | 0 | 0 | 0 | 0 | 0 | 0 | 1 | 0 | 0 | 1 | 0 | 0 | | | | | | |
| 3138 | 03707 | 03137 | 3 | | 0 | 0 | 0 | 0 | 0 | 0 | 0 | 0 | 0 | 1 | 0 | 0 | 0 | 0 | 0 | 0 | 1 | 0 | 0 | 0 | 0 | 1 | 0 | 0 | 0 | M64571 | 92.1 | 353 | 1 | 4646 | 5022 |
| 3139 | 03708 | 03138 | 4 | | 0 | 0 | 0 | 0 | 0 | 0 | 0 | 0 | 0 | 2 | 0 | 0 | 0 | 0 | 0 | 0 | 0 | 0 | 0 | 0 | 0 | 0 | 0 | 2 | 0 | M94556 | 98.3 | 355 | 1 | 275 | 628 |
| 3140 | 03709 | 03139 | 2 | | 0 | 0 | 0 | 0 | 0 | 0 | 0 | 0 | 0 | 1 | 0 | 0 | 0 | 0 | 0 | 0 | 0 | 0 | 1 | 0 | 0 | 0 | 0 | 0 | 0 | | | | | | |
| 3141 | 03710 | 03140 | 4 | | 0 | 0 | 0 | 0 | 0 | 0 | 0 | 0 | 0 | 2 | 0 | 0 | 0 | 0 | 0 | 0 | 0 | 2 | 0 | 0 | 0 | 0 | 0 | 0 | 0 | | | | | | |
| 3142 | 03711 | 03141 | 1 | | 0 | 0 | 0 | 0 | 0 | 0 | 0 | 0 | 0 | 1 | 0 | 0 | 0 | 0 | 0 | 0 | 0 | 0 | 0 | 0 | 0 | 0 | 0 | 0 | 0 | | | | | | |
| 3143 | 03712 | 03142 | 3 | | 0 | 0 | 0 | 0 | 0 | 0 | 0 | 0 | 0 | 1 | 0 | 0 | 0 | 0 | 0 | 0 | 2 | 0 | 0 | 0 | 0 | 0 | 0 | 0 | 0 | | | | | | |
| 3144 | 03713 | 03143 | 1 | | 0 | 0 | 0 | 0 | 0 | 0 | 0 | 0 | 0 | 1 | 0 | 0 | 0 | 0 | 0 | 0 | 0 | 0 | 0 | 0 | 0 | 0 | 0 | 0 | 0 | | | | | | |
| 3145 | 03714 | 03144 | 2 | | 0 | 0 | 0 | 0 | 0 | 0 | 0 | 0 | 0 | 2 | 0 | 0 | 0 | 0 | 0 | 0 | 0 | 0 | 0 | 0 | 0 | 0 | 0 | 0 | 0 | | | | | | |
| 3146 | 03715 | 03145 | 3 | | 0 | 0 | 0 | 0 | 0 | 0 | 0 | 0 | 0 | 1 | 0 | 0 | 0 | 0 | 0 | 1 | 0 | 0 | 0 | 0 | 0 | 0 | 1 | 0 | 0 | | | | | | |
| 3147 | 03716 | 03146 | 4 | | 0 | 0 | 0 | 0 | 0 | 0 | 0 | 0 | 0 | 1 | 0 | 0 | 0 | 0 | 0 | 2 | 0 | 1 | 0 | 0 | 0 | 0 | 0 | 0 | 0 | | | | | | |
| 3148 | 03717 | 03147 | 5 | | 0 | 0 | 0 | 0 | 0 | 0 | 0 | 0 | 0 | 2 | 0 | 0 | 0 | 0 | 0 | 0 | 0 | 0 | 0 | 0 | 3 | 0 | 0 | 0 | 0 | M76979 | 95 | 343 | 1 | 1151 | 1490 |
| 3149 | 03718 | 03148 | 1 | | 0 | 0 | 0 | 0 | 0 | 0 | 0 | 0 | 0 | 1 | 0 | 0 | 0 | 0 | 0 | 0 | 0 | 0 | 0 | 0 | 0 | 0 | 0 | 0 | 0 | | | | | | |
| 3150 | 03719 | 03149 | 1 | | 0 | 0 | 0 | 0 | 0 | 0 | 0 | 0 | 0 | 1 | 0 | 0 | 0 | 0 | 0 | 0 | 0 | 0 | 0 | 0 | 0 | 0 | 0 | 0 | 0 | | | | | | |
| 3151 | 03720 | 03150 | 2 | | 0 | 0 | 0 | 0 | 0 | 0 | 0 | 0 | 0 | 1 | 0 | 1 | 0 | 0 | 0 | 0 | 0 | 0 | 0 | 0 | 0 | 0 | 0 | 0 | 0 | | | | | | |
| 3152 | 03721 | 03151 | 1 | | 0 | 0 | 0 | 0 | 0 | 0 | 0 | 0 | 0 | 1 | 0 | 0 | 0 | 0 | 0 | 0 | 0 | 0 | 0 | 0 | 0 | 0 | 0 | 0 | 0 | | | | | | |
| 3153 | 03722 | 03152 | 1 | | 0 | 0 | 0 | 0 | 0 | 0 | 0 | 0 | 0 | 1 | 0 | 0 | 0 | 0 | 0 | 0 | 0 | 0 | 0 | 0 | 0 | 0 | 0 | 0 | 0 | | | | | | |

Table 88

| | A | B | C | E | G | I | K | M | O | Q | S | U | W | Y | AA | AC | AE | AG | AI | AK | AM | AO | AQ | AS | AU | AW | AY | BA | BC | BE | BF | BG | BH | BI | BK |
|---|---|---|---|---|---|---|---|---|---|---|---|---|---|---|---|---|---|---|---|---|---|---|---|---|---|---|---|---|---|---|---|---|---|---|---|
| 3154 | 03723 | 03153 | 3 | | 0 | 0 | 0 | 0 | 0 | 0 | 0 | 0 | 0 | 2 | 0 | 0 | 0 | 0 | 0 | 0 | 0 | 0 | 0 | 0 | 0 | 0 | 1 | 0 | 0 | | | | | | |
| 3155 | 03724 | 03154 | 3 | | 0 | 0 | 0 | 0 | 0 | 0 | 0 | 0 | 0 | 1 | 0 | 0 | 0 | 0 | 2 | 0 | 0 | 0 | 0 | 0 | 0 | 0 | 0 | 0 | 0 | | | | | | |
| 3156 | 03725 | 03155 | 1 | | 0 | 0 | 0 | 0 | 0 | 0 | 0 | 0 | 0 | 1 | 0 | 0 | 0 | 0 | 0 | 0 | 0 | 0 | 0 | 0 | 0 | 0 | 0 | 0 | 0 | | | | | | |
| 3157 | 03726 | 03156 | 2 | | 0 | 0 | 0 | 0 | 0 | 0 | 0 | 0 | 0 | 1 | 0 | 0 | 0 | 0 | 0 | 0 | 0 | 0 | 0 | 0 | 0 | 0 | 0 | 0 | 1 | | | | | | |
| 3158 | 03727 | 03157 | 1 | | 0 | 0 | 0 | 0 | 0 | 0 | 0 | 0 | 0 | 1 | 0 | 0 | 0 | 0 | 0 | 0 | 0 | 0 | 0 | 0 | 0 | 0 | 0 | 0 | 0 | | | | | | |
| 3159 | 03728 | 03158 | 2 | | 0 | 0 | 0 | 0 | 0 | 0 | 0 | 0 | 0 | 1 | 0 | 0 | 0 | 0 | 0 | 0 | 0 | 0 | 1 | 0 | 0 | 0 | 0 | 0 | 0 | | | | | | |
| 3160 | 03729 | 03159 | 3 | | 0 | 0 | 0 | 0 | 0 | 0 | 0 | 0 | 0 | 1 | 2 | 0 | 0 | 0 | 0 | 0 | 0 | 0 | 0 | 0 | 0 | 0 | 0 | 0 | 0 | | | | | | |
| 3161 | 03730 | 03160 | 1 | | 0 | 0 | 0 | 0 | 0 | 0 | 0 | 0 | 0 | 1 | 0 | 0 | 0 | 0 | 0 | 0 | 0 | 0 | 0 | 0 | 0 | 0 | 0 | 0 | 0 | | | | | | |
| 3162 | 03731 | 03161 | 3 | | 0 | 0 | 0 | 0 | 0 | 0 | 0 | 0 | 0 | 1 | 0 | 0 | 0 | 0 | 1 | 1 | 0 | 0 | 0 | 0 | 0 | 0 | 0 | 0 | 0 | | | | | | |
| 3163 | 03732 | 03162 | 2 | | 0 | 0 | 0 | 0 | 0 | 0 | 0 | 0 | 0 | 1 | 0 | 0 | 0 | 0 | 0 | 0 | 0 | 0 | 0 | 0 | 0 | 1 | 0 | 0 | 0 | | | | | | |
| 3164 | 03733 | 03163 | 2 | | 0 | 0 | 0 | 0 | 0 | 0 | 0 | 0 | 0 | 2 | 0 | 0 | 0 | 0 | 0 | 0 | 0 | 0 | 0 | 0 | 0 | 0 | 0 | 0 | 0 | | | | | | |
| 3165 | 03734 | 03164 | 7 | | 0 | 0 | 0 | 0 | 0 | 0 | 0 | 0 | 0 | 1 | 1 | 1 | 0 | 0 | 1 | 0 | 0 | 0 | 0 | 0 | 0 | 0 | 0 | 3 | 0 | | | | | | |
| 3166 | 03736 | 03165 | 1 | | 0 | 0 | 0 | 0 | 0 | 0 | 0 | 0 | 0 | 1 | 0 | 0 | 0 | 0 | 0 | 0 | 0 | 0 | 0 | 0 | 0 | 0 | 0 | 0 | 0 | | | | | | |
| 3167 | 03737 | 03166 | 1 | | 0 | 0 | 0 | 0 | 0 | 0 | 0 | 0 | 0 | 1 | 0 | 0 | 0 | 0 | 0 | 0 | 0 | 0 | 0 | 0 | 0 | 0 | 0 | 0 | 0 | | | | | | |
| 3168 | 03738 | 03167 | 1 | | 0 | 0 | 0 | 0 | 0 | 0 | 0 | 0 | 0 | 1 | 0 | 0 | 0 | 0 | 0 | 0 | 0 | 0 | 0 | 0 | 0 | 0 | 0 | 0 | 0 | | | | | | |
| 3169 | 03739 | 03168 | 1 | | 0 | 0 | 0 | 0 | 0 | 0 | 0 | 0 | 0 | 1 | 0 | 0 | 0 | 0 | 0 | 0 | 0 | 0 | 0 | 0 | 0 | 0 | 0 | 0 | 0 | | | | | | |
| 3170 | 03740 | 03169 | 2 | | 0 | 0 | 0 | 0 | 0 | 0 | 0 | 0 | 0 | 1 | 0 | 1 | 0 | 0 | 0 | 0 | 0 | 0 | 0 | 0 | 0 | 0 | 0 | 0 | 0 | | | | | | |
| 3171 | 03741 | 03170 | 1 | | 0 | 0 | 0 | 0 | 0 | 0 | 0 | 0 | 0 | 1 | 0 | 0 | 0 | 0 | 0 | 0 | 0 | 0 | 0 | 0 | 0 | 0 | 0 | 0 | 0 | | | | | | |
| 3172 | 03742 | 03171 | 3 | | 0 | 0 | 0 | 0 | 0 | 0 | 0 | 0 | 0 | 2 | 1 | 0 | 0 | 0 | 0 | 0 | 0 | 0 | 0 | 0 | 0 | 0 | 0 | 0 | 0 | | | | | | |
| 3173 | 03743 | 03172 | 1 | | 0 | 0 | 0 | 0 | 0 | 0 | 0 | 0 | 0 | 1 | 0 | 0 | 0 | 0 | 0 | 0 | 0 | 0 | 0 | 0 | 0 | 0 | 0 | 0 | 0 | L21186 | 97.3 | 300 | 1 | 2029 | 2328 |
| 3174 | 03744 | 03173 | 4 | | 0 | 0 | 0 | 0 | 0 | 0 | 0 | 0 | 0 | 1 | 0 | 0 | 0 | 0 | 0 | 2 | 0 | 0 | 0 | 0 | 0 | 0 | 0 | 1 | 0 | | | | | | |
| 3175 | 03745 | 03174 | 1 | | 0 | 0 | 0 | 0 | 0 | 0 | 0 | 0 | 0 | 1 | 0 | 0 | 0 | 0 | 0 | 0 | 0 | 0 | 0 | 0 | 0 | 0 | 0 | 0 | 0 | | | | | | |
| 3176 | 03746 | 03175 | 1 | | 0 | 0 | 0 | 0 | 0 | 0 | 0 | 0 | 0 | 1 | 0 | 0 | 0 | 0 | 0 | 0 | 0 | 0 | 0 | 0 | 0 | 0 | 0 | 0 | 0 | | | | | | |
| 3177 | 03747 | 03176 | 2 | | 0 | 0 | 0 | 0 | 0 | 0 | 0 | 0 | 0 | 2 | 0 | 0 | 0 | 0 | 0 | 0 | 0 | 0 | 0 | 0 | 0 | 0 | 0 | 0 | 0 | | | | | | |
| 3178 | 03748 | 03177 | 1 | | 0 | 0 | 0 | 0 | 0 | 0 | 0 | 0 | 0 | 1 | 0 | 0 | 0 | 0 | 0 | 0 | 0 | 0 | 0 | 0 | 0 | 0 | 0 | 0 | 0 | | | | | | |
| 3179 | 03749 | 03178 | 1 | | 0 | 0 | 0 | 0 | 0 | 0 | 0 | 0 | 0 | 1 | 0 | 0 | 0 | 0 | 0 | 0 | 0 | 0 | 0 | 0 | 0 | 0 | 0 | 0 | 0 | | | | | | |
| 3180 | 03750 | 03179 | 1 | | 0 | 0 | 0 | 0 | 0 | 0 | 0 | 0 | 0 | 1 | 0 | 0 | 0 | 0 | 0 | 0 | 0 | 0 | 0 | 0 | 0 | 0 | 0 | 0 | 0 | | | | | | |
| 3181 | 03751 | 03180 | 2 | | 0 | 0 | 0 | 0 | 0 | 0 | 0 | 0 | 0 | 1 | 0 | 0 | 0 | 0 | 0 | 0 | 1 | 0 | 0 | 0 | 0 | 0 | 0 | 0 | 0 | | | | | | |
| 3182 | 03752 | 03181 | 8 | | 0 | 0 | 0 | 0 | 0 | 0 | 0 | 0 | 0 | 3 | 0 | 0 | 0 | 0 | 0 | 1 | 0 | 4 | 0 | 0 | 0 | 0 | 0 | 0 | 0 | | | | | | |
| 3183 | 03753 | 03182 | 2 | | 0 | 0 | 0 | 0 | 0 | 0 | 0 | 0 | 0 | 1 | 0 | 0 | 0 | 0 | 0 | 0 | 0 | 1 | 0 | 0 | 0 | 0 | 0 | 0 | 0 | | | | | | |
| 3184 | 03754 | 03183 | 1 | | 0 | 0 | 0 | 0 | 0 | 0 | 0 | 0 | 0 | 1 | 0 | 0 | 0 | 0 | 0 | 0 | 0 | 0 | 0 | 0 | 0 | 0 | 0 | 0 | 0 | | | | | | |
| 3185 | 03755 | 03184 | 6 | | 0 | 0 | 0 | 0 | 0 | 0 | 0 | 0 | 0 | 1 | 0 | 0 | 0 | 0 | 1 | 0 | 0 | 4 | 0 | 0 | 0 | 0 | 0 | 0 | 0 | X02598 | 97.7 | 393 | 1 | 227 | 760 |
| 3186 | 03756 | 03185 | 1 | | 0 | 0 | 0 | 0 | 0 | 0 | 0 | 0 | 0 | 1 | 0 | 0 | 0 | 0 | 0 | 0 | 0 | 0 | 0 | 0 | 0 | 0 | 0 | 0 | 0 | Y00285 | 96.6 | 58 | 1 | 6745 | 9090 |
| 3187 | 03757 | 03186 | 1 | | 0 | 0 | 0 | 0 | 0 | 0 | 0 | 0 | 0 | 1 | 0 | 0 | 0 | 0 | 0 | 0 | 0 | 0 | 0 | 0 | 0 | 0 | 0 | 0 | 0 | | | | | | |
| 3188 | 03758 | 03187 | 1 | | 0 | 0 | 0 | 0 | 0 | 0 | 0 | 0 | 0 | 1 | 0 | 0 | 0 | 0 | 0 | 0 | 0 | 0 | 0 | 0 | 0 | 0 | 0 | 0 | 0 | D90228 | 97.6 | 287 | 1 | 1184 | 1518 |
| 3189 | 03759 | 03188 | 1 | | 0 | 0 | 0 | 0 | 0 | 0 | 0 | 0 | 0 | 1 | 0 | 0 | 0 | 0 | 0 | 0 | 0 | 0 | 0 | 0 | 0 | 0 | 0 | 0 | 0 | | | | | | |

Table 89

| | A | B | C | E | G | I | K | M | O | Q | S | U | W | Y | AA | AC | AF | AG | AI | AK | AM | AO | AQ | AS | AU | AW | AY | BA | BC | BE | BF | BG | BH | BI | BK |
|---|---|---|---|---|---|---|---|---|---|---|---|---|---|---|---|---|---|---|---|---|---|---|---|---|---|---|---|---|---|---|---|---|---|---|---|
| 3190 | 03760 | 03189 | 1 | | 0 | 0 | 0 | 0 | 0 | 0 | 0 | 0 | 0 | 1 | 0 | 0 | 0 | 0 | 0 | 0 | 0 | 0 | 0 | 0 | 0 | 0 | 0 | 0 | 0 | | | | | | |
| 3191 | 03761 | 03190 | 4 | | 0 | 0 | 0 | 0 | 0 | 0 | 0 | 0 | 0 | 1 | 0 | 0 | 0 | 1 | 0 | 1 | 0 | 0 | 0 | 0 | 1 | 0 | 0 | 0 | 0 | | | | | | |
| 3192 | 03762 | 03191 | 2 | | 0 | 0 | 0 | 0 | 0 | 0 | 0 | 0 | 0 | 2 | 0 | 0 | 0 | 0 | 0 | 0 | 0 | 0 | 0 | 0 | 0 | 0 | 0 | 0 | 0 | | | | | | |
| 3193 | 03763 | 03192 | 2 | | 0 | 0 | 0 | 0 | 0 | 0 | 0 | 0 | 0 | 1 | 0 | 1 | 0 | 0 | 0 | 0 | 0 | 0 | 0 | 0 | 0 | 0 | 0 | 0 | 0 | | | | | | |
| 3194 | 03764 | 03193 | 1 | | 0 | 0 | 0 | 0 | 0 | 0 | 0 | 0 | 0 | 1 | 0 | 0 | 0 | 0 | 0 | 0 | 0 | 0 | 0 | 0 | 0 | 0 | 0 | 0 | 0 | X70904 | 92.4 | 276 | 1 | 1063 | 1405 |
| 3195 | 03765 | 03194 | 1 | | 0 | 0 | 0 | 0 | 0 | 0 | 0 | 0 | 0 | 1 | 0 | 0 | 0 | 0 | 0 | 0 | 0 | 0 | 0 | 0 | 0 | 0 | 0 | 0 | 0 | | | | | | |
| 3196 | 03766 | 03195 | 1 | | 0 | 0 | 0 | 0 | 0 | 0 | 0 | 0 | 0 | 1 | 0 | 0 | 0 | 0 | 0 | 0 | 0 | 0 | 0 | 0 | 0 | 0 | 0 | 0 | 0 | M36035 | 92.6 | 272 | 1 | 392 | 821 |
| 3197 | 03767 | 03196 | 1 | | 0 | 0 | 0 | 0 | 0 | 0 | 0 | 0 | 0 | 1 | 0 | 0 | 0 | 0 | 0 | 0 | 0 | 0 | 0 | 0 | 0 | 0 | 0 | 0 | 0 | | | | | | |
| 3198 | 03768 | 03197 | 3 | | 0 | 0 | 0 | 0 | 0 | 0 | 0 | 0 | 0 | 1 | 0 | 0 | 0 | 0 | 0 | 0 | 0 | 0 | 0 | 0 | 1 | 0 | 0 | 0 | 1 | | | | | | |
| 3199 | 03769 | 03198 | 1 | | 0 | 0 | 0 | 0 | 0 | 0 | 0 | 0 | 0 | 1 | 0 | 0 | 0 | 0 | 0 | 0 | 0 | 0 | 0 | 0 | 0 | 0 | 0 | 0 | 0 | X53698 | 96.7 | 241 | 1 | 211 | 451 |
| 3200 | 03770 | 03199 | 1 | | 0 | 0 | 0 | 0 | 0 | 0 | 0 | 0 | 0 | 1 | 0 | 0 | 0 | 0 | 0 | 0 | 0 | 0 | 0 | 0 | 0 | 0 | 0 | 0 | 0 | | | | | | |
| 3201 | 03771 | 03200 | 1 | | 0 | 0 | 0 | 0 | 0 | 0 | 0 | 0 | 0 | 1 | 0 | 0 | 0 | 0 | 0 | 0 | 0 | 0 | 0 | 0 | 0 | 0 | 0 | 0 | 0 | | | | | | |
| 3202 | 03772 | 03201 | 1 | | 0 | 0 | 0 | 0 | 0 | 0 | 0 | 0 | 0 | 1 | 0 | 0 | 0 | 0 | 0 | 0 | 0 | 0 | 0 | 0 | 0 | 0 | 0 | 0 | 0 | | | | | | |
| 3203 | 03773 | 03202 | 1 | | 0 | 0 | 0 | 0 | 0 | 0 | 0 | 0 | 0 | 1 | 0 | 0 | 0 | 0 | 0 | 0 | 0 | 0 | 0 | 0 | 0 | 0 | 0 | 0 | 0 | | | | | | |
| 3204 | 03774 | 03203 | 2 | | 0 | 0 | 0 | 0 | 0 | 0 | 0 | 0 | 0 | 1 | 0 | 0 | 0 | 0 | 0 | 0 | 0 | 1 | 0 | 0 | 0 | 0 | 0 | 0 | 0 | | | | | | |
| 3205 | 03775 | 03204 | 5 | | 0 | 0 | 0 | 0 | 0 | 0 | 0 | 0 | 0 | 1 | 0 | 1 | 0 | 0 | 0 | 0 | 0 | 1 | 0 | 2 | 0 | 0 | 0 | 0 | 0 | L11373 | 94.4 | 213 | 1 | 4478 | 4688 |
| 3206 | 03776 | 03205 | 1 | | 0 | 0 | 0 | 0 | 0 | 0 | 0 | 0 | 0 | 1 | 0 | 0 | 0 | 0 | 0 | 0 | 0 | 0 | 0 | 0 | 0 | 0 | 0 | 0 | 0 | | | | | | |
| 3207 | 03777 | 03206 | 2 | | 0 | 0 | 0 | 0 | 0 | 0 | 0 | 0 | 0 | 1 | 0 | 0 | 1 | 0 | 0 | 0 | 0 | 0 | 0 | 0 | 0 | 0 | 0 | 0 | 0 | | | | | | |
| 3208 | 03778 | 03207 | 1 | | 0 | 0 | 0 | 0 | 0 | 0 | 0 | 0 | 0 | 1 | 0 | 0 | 0 | 0 | 0 | 0 | 0 | 0 | 0 | 0 | 0 | 0 | 0 | 0 | 0 | | | | | | |
| 3209 | 03779 | 03208 | 1 | | 0 | 0 | 0 | 0 | 0 | 0 | 0 | 0 | 0 | 1 | 0 | 0 | 0 | 0 | 0 | 0 | 0 | 0 | 0 | 0 | 0 | 0 | 0 | 0 | 0 | | | | | | |
| 3210 | 03780 | 03209 | 3 | | 0 | 0 | 0 | 0 | 0 | 0 | 0 | 0 | 0 | 1 | 0 | 0 | 0 | 0 | 0 | 0 | 0 | 0 | 0 | 0 | 0 | 0 | 0 | 2 | 0 | J03459 | 99.6 | 246 | 1 | 1815 | 2060 |
| 3211 | 03781 | 03210 | 4 | | 0 | 0 | 0 | 0 | 0 | 0 | 0 | 0 | 0 | 1 | 0 | 0 | 0 | 0 | 0 | 0 | 0 | 0 | 0 | 0 | 0 | 0 | 0 | 3 | 0 | | | | | | |
| 3212 | 03782 | 03211 | 3 | | 0 | 0 | 0 | 0 | 0 | 0 | 0 | 0 | 0 | 1 | 0 | 2 | 0 | 0 | 0 | 0 | 0 | 0 | 0 | 0 | 0 | 0 | 0 | 0 | 0 | D13636 | 97.2 | 248 | 1 | 3347 | 3594 |
| 3213 | 03783 | 03212 | 1 | | 0 | 0 | 0 | 0 | 0 | 0 | 0 | 0 | 0 | 1 | 0 | 0 | 0 | 0 | 0 | 0 | 0 | 0 | 0 | 0 | 0 | 0 | 0 | 0 | 0 | | | | | | |
| 3214 | 03784 | 03213 | 3 | | 0 | 0 | 0 | 0 | 0 | 0 | 0 | 0 | 0 | 1 | 0 | 0 | 0 | 1 | 0 | 0 | 0 | 0 | 0 | 1 | 0 | 0 | 0 | 0 | 0 | | | | | | |
| 3215 | 03785 | 03214 | 1 | | 0 | 0 | 0 | 0 | 0 | 0 | 0 | 0 | 0 | 1 | 0 | 0 | 0 | 0 | 0 | 0 | 0 | 0 | 0 | 0 | 0 | 0 | 0 | 0 | 0 | | | | | | |
| 3216 | 03786 | 03215 | 2 | | 0 | 0 | 0 | 0 | 0 | 0 | 0 | 0 | 0 | 2 | 0 | 0 | 0 | 0 | 0 | 0 | 0 | 0 | 0 | 0 | 0 | 0 | 0 | 0 | 0 | | | | | | |
| 3217 | 03787 | 03216 | 4 | | 0 | 0 | 0 | 0 | 0 | 0 | 0 | 0 | 0 | 1 | 0 | 0 | 0 | 0 | 0 | 0 | 0 | 1 | 1 | 0 | 0 | 0 | 0 | 1 | 0 | | | | | | |
| 3218 | 03788 | 03217 | 3 | | 0 | 0 | 0 | 0 | 0 | 0 | 0 | 0 | 0 | 1 | 1 | 0 | 0 | 0 | 0 | 0 | 0 | 0 | 0 | 0 | 0 | 0 | 0 | 1 | 0 | | | | | | |
| 3219 | 03789 | 03218 | 6 | | 0 | 0 | 0 | 0 | 0 | 0 | 0 | 0 | 0 | 1 | 1 | 1 | 0 | 0 | 1 | 0 | 0 | 1 | 0 | 1 | 0 | 0 | 0 | 0 | 0 | | | | | | |
| 3220 | 03790 | 03219 | 2 | | 0 | 0 | 0 | 0 | 0 | 0 | 0 | 0 | 0 | 1 | 0 | 0 | 0 | 0 | 0 | 0 | 0 | 0 | 0 | 1 | 0 | 0 | 0 | 0 | 0 | | | | | | |
| 3221 | 03791 | 03220 | 1 | | 0 | 0 | 0 | 0 | 0 | 0 | 0 | 0 | 0 | 1 | 0 | 0 | 0 | 0 | 0 | 0 | 0 | 0 | 0 | 0 | 0 | 0 | 0 | 0 | 0 | | | | | | |
| 3222 | 03792 | 03221 | 1 | | 0 | 0 | 0 | 0 | 0 | 0 | 0 | 0 | 0 | 1 | 0 | 0 | 0 | 0 | 0 | 0 | 0 | 0 | 0 | 0 | 0 | 0 | 0 | 0 | 0 | | | | | | |
| 3223 | 03793 | 03222 | 1 | | 0 | 0 | 0 | 0 | 0 | 0 | 0 | 0 | 0 | 1 | 0 | 0 | 0 | 0 | 0 | 0 | 0 | 0 | 0 | 0 | 0 | 0 | 0 | 0 | 0 | | | | | | |
| 3224 | 03794 | 03223 | 5 | | 0 | 0 | 0 | 0 | 0 | 0 | 0 | 0 | 0 | 2 | 0 | 0 | 0 | 0 | 0 | 0 | 0 | 2 | 0 | 0 | 1 | 0 | 0 | 0 | 0 | | | | | | |
| 3225 | 03795 | 03224 | 2 | | 0 | 0 | 0 | 0 | 0 | 0 | 0 | 0 | 0 | 1 | 0 | 0 | 0 | 0 | 0 | 0 | 0 | 0 | 0 | 0 | 0 | 0 | 0 | 1 | 0 | | | | | | |

Table 90

| | A | B | C | E | G | I | K | M | O | Q | S | U | W | Y | AA | AC | AF | AG | AI | AK | AM | AO | AQ | AS | AU | AW | AY | BA | BC | BE | BF | BG | BH | BI | BK |
|---|---|---|---|---|---|---|---|---|---|---|---|---|---|---|---|---|---|---|---|---|---|---|---|---|---|---|---|---|---|---|---|---|---|---|---|
| 3226 | 03796 | 03225 | 1 | | 0 | 0 | 0 | 0 | 0 | 0 | 0 | 0 | 0 | 1 | 0 | 0 | 0 | 0 | 0 | 0 | 0 | 0 | 0 | 0 | 0 | 0 | 0 | 0 | 0 | | | | | | |
| 3227 | 03797 | 03226 | 2 | | 0 | 0 | 0 | 0 | 0 | 0 | 0 | 0 | 0 | 1 | 0 | 0 | 1 | 0 | 0 | 0 | 0 | 0 | 0 | 0 | 0 | 0 | 0 | 0 | 0 | | | | | | |
| 3228 | 03798 | 03227 | 1 | | 0 | 0 | 0 | 0 | 0 | 0 | 0 | 0 | 0 | 1 | 0 | 0 | 0 | 0 | 0 | 0 | 0 | 0 | 0 | 0 | 0 | 0 | 0 | 0 | 0 | | | | | | |
| 3229 | 03799 | 03228 | 1 | | 0 | 0 | 0 | 0 | 0 | 0 | 0 | 0 | 0 | 1 | 0 | 0 | 0 | 0 | 0 | 0 | 0 | 0 | 0 | 0 | 0 | 0 | 0 | 0 | 0 | | | | | | |
| 3230 | 03800 | 03229 | 1 | | 0 | 0 | 0 | 0 | 0 | 0 | 0 | 0 | 0 | 1 | 0 | 0 | 0 | 0 | 0 | 0 | 0 | 0 | 0 | 0 | 0 | 0 | 0 | 0 | 0 | | | | | | |
| 3231 | 03801 | 03230 | 3 | | 0 | 0 | 0 | 0 | 0 | 0 | 0 | 0 | 0 | 2 | 0 | 0 | 0 | 0 | 1 | 0 | 0 | 0 | 0 | 0 | 0 | 0 | 0 | 0 | 0 | | | | | | |
| 3232 | 03802 | 03231 | 2 | | 0 | 0 | 0 | 0 | 0 | 0 | 0 | 0 | 0 | 1 | 0 | 0 | 0 | 0 | 0 | 0 | 0 | 0 | 1 | 0 | 0 | 0 | 0 | 0 | 0 | D13639 | 100 | 224 | 1 | 6255 | 6478 |
| 3233 | 03803 | 03232 | 2 | | 0 | 0 | 0 | 0 | 0 | 0 | 0 | 0 | 0 | 1 | 0 | 0 | 0 | 0 | 0 | 0 | 0 | 0 | 1 | 0 | 0 | 0 | 0 | 0 | 0 | | | | | | |
| 3234 | 03804 | 03233 | 7 | | 0 | 0 | 0 | 0 | 0 | 0 | 0 | 0 | 0 | 1 | 0 | 0 | 0 | 1 | 1 | 0 | 0 | 1 | 0 | 0 | 1 | 1 | 1 | 0 | 0 | | | | | | |
| 3235 | 03805 | 03234 | 1 | | 0 | 0 | 0 | 0 | 0 | 0 | 0 | 0 | 0 | 1 | 0 | 0 | 0 | 0 | 0 | 0 | 0 | 0 | 0 | 0 | 0 | 0 | 0 | 0 | 0 | | | | | | |
| 3236 | 03806 | 03235 | 3 | | 0 | 0 | 0 | 0 | 0 | 0 | 0 | 0 | 0 | 2 | 0 | 0 | 0 | 0 | 0 | 1 | 0 | 0 | 0 | 0 | 0 | 0 | 0 | 0 | 0 | | | | | | |
| 3237 | 03807 | 03236 | 1 | | 0 | 0 | 0 | 0 | 0 | 0 | 0 | 0 | 0 | 1 | 0 | 0 | 0 | 0 | 0 | 0 | 0 | 0 | 0 | 0 | 0 | 0 | 0 | 0 | 0 | | | | | | |
| 3238 | 03808 | 03237 | 3 | | 0 | 0 | 0 | 0 | 0 | 0 | 0 | 0 | 0 | 1 | 0 | 0 | 0 | 0 | 0 | 0 | 0 | 0 | 0 | 0 | 0 | 0 | 0 | 2 | 0 | | | | | | |
| 3239 | 03809 | 03238 | 2 | | 0 | 0 | 0 | 0 | 0 | 0 | 0 | 0 | 0 | 2 | 0 | 0 | 0 | 0 | 0 | 0 | 0 | 0 | 0 | 0 | 0 | 0 | 0 | 0 | 0 | | | | | | |
| 3240 | 03810 | 03239 | 1 | | 0 | 0 | 0 | 0 | 0 | 0 | 0 | 0 | 0 | 1 | 0 | 0 | 0 | 0 | 0 | 0 | 0 | 0 | 0 | 0 | 0 | 0 | 0 | 0 | 0 | | | | | | |
| 3241 | 03811 | 03240 | 1 | | 0 | 0 | 0 | 0 | 0 | 0 | 0 | 0 | 0 | 1 | 0 | 0 | 0 | 0 | 0 | 0 | 0 | 0 | 0 | 0 | 0 | 0 | 0 | 0 | 0 | | | | | | |
| 3242 | 03812 | 03241 | 1 | | 0 | 0 | 0 | 0 | 0 | 0 | 0 | 0 | 0 | 1 | 0 | 0 | 0 | 0 | 0 | 0 | 0 | 0 | 0 | 0 | 0 | 0 | 0 | 0 | 0 | | | | | | |
| 3243 | 03813 | 03242 | 1 | | 0 | 0 | 0 | 0 | 0 | 0 | 0 | 0 | 0 | 1 | 0 | 0 | 0 | 0 | 0 | 0 | 0 | 0 | 0 | 0 | 0 | 0 | 0 | 0 | 0 | L16896 | 96.2 | 213 | 1 | 2003 | 2288 |
| 3244 | 03814 | 03243 | 6 | | 0 | 0 | 0 | 0 | 0 | 0 | 0 | 0 | 0 | 1 | 0 | 2 | 0 | 0 | 2 | 0 | 0 | 0 | 0 | 1 | 0 | 0 | 0 | 0 | 0 | M63959 | 96.2 | 209 | 1 | 1286 | 1493 |
| 3245 | 03815 | 03244 | 1 | | 0 | 0 | 0 | 0 | 0 | 0 | 0 | 0 | 0 | 1 | 0 | 0 | 0 | 0 | 0 | 0 | 0 | 0 | 0 | 0 | 0 | 0 | 0 | 0 | 0 | M17219 | 99 | 208 | 1 | 1105 | 1344 |
| 3246 | 03816 | 03245 | 1 | | 0 | 0 | 0 | 0 | 0 | 0 | 0 | 0 | 0 | 1 | 0 | 0 | 0 | 0 | 0 | 0 | 0 | 0 | 0 | 0 | 0 | 0 | 0 | 0 | 0 | | | | | | |
| 3247 | 03817 | 03246 | 1 | | 0 | 0 | 0 | 0 | 0 | 0 | 0 | 0 | 0 | 1 | 0 | 0 | 0 | 0 | 0 | 0 | 0 | 0 | 0 | 0 | 0 | 0 | 0 | 0 | 0 | | | | | | |
| 3248 | 03818 | 03247 | 1 | | 0 | 0 | 0 | 0 | 0 | 0 | 0 | 0 | 0 | 1 | 0 | 0 | 0 | 0 | 0 | 0 | 0 | 0 | 0 | 0 | 0 | 0 | 0 | 0 | 0 | | | | | | |
| 3249 | 03819 | 03248 | 1 | | 0 | 0 | 0 | 0 | 0 | 0 | 0 | 0 | 0 | 1 | 0 | 0 | 0 | 0 | 0 | 0 | 0 | 0 | 0 | 0 | 0 | 0 | 0 | 0 | 0 | L08240 | 99.5 | 201 | 1 | 1992 | 2198 |
| 3250 | 03820 | 03249 | 3 | | 0 | 0 | 0 | 0 | 0 | 0 | 0 | 0 | 0 | 2 | 0 | 0 | 0 | 0 | 0 | 0 | 0 | 1 | 0 | 0 | 0 | 0 | 0 | 0 | 0 | M25785 | 98.1 | 209 | 1 | 70 | 874 |
| 3251 | 03821 | 03250 | 3 | | 0 | 0 | 0 | 0 | 0 | 0 | 0 | 0 | 0 | 1 | 0 | 0 | 0 | 0 | 0 | 0 | 0 | 2 | 0 | 0 | 0 | 0 | 0 | 0 | 0 | | | | | | |
| 3252 | 03822 | 03251 | 5 | | 0 | 0 | 0 | 0 | 0 | 0 | 0 | 0 | 0 | 1 | 0 | 0 | 0 | 1 | 0 | 1 | 0 | 0 | 1 | 1 | 0 | 0 | 0 | 0 | 0 | | | | | | |
| 3253 | 03823 | 03252 | 1 | | 0 | 0 | 0 | 0 | 0 | 0 | 0 | 0 | 0 | 1 | 0 | 0 | 0 | 0 | 0 | 0 | 0 | 0 | 0 | 0 | 0 | 0 | 0 | 0 | 0 | | | | | | |
| 3254 | 03824 | 03253 | 2 | | 0 | 0 | 0 | 0 | 0 | 0 | 0 | 0 | 0 | 1 | 0 | 0 | 0 | 0 | 1 | 0 | 0 | 0 | 0 | 0 | 0 | 0 | 0 | 0 | 0 | | | | | | |
| 3255 | 03825 | 03254 | 2 | | 0 | 0 | 0 | 0 | 0 | 0 | 0 | 0 | 0 | 1 | 0 | 0 | 0 | 0 | 0 | 0 | 0 | 0 | 0 | 0 | 0 | 1 | 0 | 0 | 0 | | | | | | |
| 3256 | 03826 | 03255 | 2 | | 0 | 0 | 0 | 0 | 0 | 0 | 0 | 0 | 0 | 1 | 0 | 0 | 0 | 0 | 1 | 0 | 0 | 0 | 0 | 0 | 0 | 0 | 0 | 0 | 0 | | | | | | |
| 3257 | 03827 | 03256 | 2 | | 0 | 0 | 0 | 0 | 0 | 0 | 0 | 0 | 0 | 1 | 0 | 0 | 0 | 0 | 0 | 1 | 0 | 0 | 0 | 0 | 0 | 0 | 0 | 0 | 0 | | | | | | |
| 3258 | 03828 | 03257 | 2 | | 0 | 0 | 0 | 0 | 0 | 0 | 0 | 0 | 0 | 1 | 1 | 0 | 0 | 0 | 0 | 0 | 0 | 0 | 0 | 0 | 0 | 0 | 0 | 0 | 0 | | | | | | |
| 3259 | 03829 | 03258 | 2 | | 0 | 0 | 0 | 0 | 0 | 0 | 0 | 0 | 0 | 1 | 0 | 0 | 0 | 0 | 0 | 1 | 0 | 0 | 0 | 0 | 0 | 0 | 0 | 0 | 0 | | | | | | |
| 3260 | 03830 | 03259 | 1 | | 0 | 0 | 0 | 0 | 0 | 0 | 0 | 0 | 0 | 1 | 0 | 0 | 0 | 0 | 0 | 0 | 0 | 0 | 0 | 0 | 0 | 0 | 0 | 0 | 0 | | | | | | |
| 3261 | 03831 | 03260 | 2 | | 0 | 0 | 0 | 0 | 0 | 0 | 0 | 0 | 0 | 1 | 1 | 0 | 0 | 0 | 0 | 0 | 0 | 0 | 0 | 0 | 0 | 0 | 0 | 0 | 0 | | | | | | |

Table 91

| | A | B | C | E | G | I | K | M | O | Q | S | U | W | Y | AA | AC | AE | AG | AI | AK | AM | AO | AQ | AS | AU | AW | AY | BA | BC | BE | BF | BG | BH | BI | BK |
|---|---|---|---|---|---|---|---|---|---|---|---|---|---|---|---|---|---|---|---|---|---|---|---|---|---|---|---|---|---|---|---|---|---|---|---|
| 3262 | 03832 | 03261 | 1 | | 0 | 0 | 0 | 0 | 0 | 0 | 0 | 0 | 0 | 1 | 0 | 0 | 0 | 0 | 0 | 0 | 0 | 0 | 0 | 0 | 0 | 0 | 0 | 0 | 0 | | | | | | |
| 3263 | 03833 | 03262 | 2 | | 0 | 0 | 0 | 0 | 0 | 0 | 0 | 0 | 0 | 2 | 0 | 0 | 0 | 0 | 0 | 0 | 0 | 0 | 0 | 0 | 0 | 0 | 0 | 0 | 0 | | | | | | |
| 3264 | 03835 | 03263 | 1 | | 0 | 0 | 0 | 0 | 0 | 0 | 0 | 0 | 0 | 1 | 0 | 0 | 0 | 0 | 0 | 0 | 0 | 0 | 0 | 0 | 0 | 0 | 0 | 0 | 0 | | | | | | |
| 3265 | 03836 | 03264 | 1 | | 0 | 0 | 0 | 0 | 0 | 0 | 0 | 0 | 0 | 1 | 0 | 0 | 0 | 0 | 0 | 0 | 0 | 0 | 0 | 0 | 0 | 0 | 0 | 0 | 0 | | | | | | |
| 3266 | 03837 | 03265 | 13 | | 0 | 0 | 0 | 0 | 0 | 0 | 0 | 0 | 0 | 5 | 0 | 0 | 0 | 0 | 7 | 1 | 0 | 0 | 0 | 0 | 0 | 0 | 0 | 0 | 0 | | | | | | |
| 3267 | 03838 | 03266 | 1 | | 0 | 0 | 0 | 0 | 0 | 0 | 0 | 0 | 0 | 1 | 0 | 0 | 0 | 0 | 0 | 0 | 0 | 0 | 0 | 0 | 0 | 0 | 0 | 0 | 0 | X04665 | 100 | 171 | 1 | 3674 | 4434 |
| 3268 | 03839 | 03267 | 2 | | 0 | 0 | 0 | 0 | 0 | 0 | 0 | 0 | 0 | 1 | 0 | 0 | 0 | 0 | 0 | 0 | 0 | 0 | 0 | 0 | 0 | 0 | 0 | 1 | 0 | X61123 | 96.7 | 337 | 1 | 1050 | 1783 |
| 3269 | 03840 | 03268 | 1 | | 0 | 0 | 0 | 0 | 0 | 0 | 0 | 0 | 0 | 1 | 0 | 0 | 0 | 0 | 0 | 0 | 0 | 0 | 0 | 0 | 0 | 0 | 0 | 0 | 0 | | | | | | |
| 3270 | 03841 | 03269 | 3 | | 0 | 0 | 0 | 0 | 0 | 0 | 0 | 0 | 0 | 3 | 0 | 0 | 0 | 0 | 0 | 0 | 0 | 0 | 0 | 0 | 0 | 0 | 0 | 0 | 0 | | | | | | |
| 3271 | 03842 | 03270 | 2 | | 0 | 0 | 0 | 0 | 0 | 0 | 0 | 0 | 0 | 1 | 0 | 0 | 0 | 0 | 0 | 0 | 0 | 0 | 0 | 0 | 1 | 0 | 0 | 0 | 0 | | | | | | |
| 3272 | 03843 | 03271 | 1 | | 0 | 0 | 0 | 0 | 0 | 0 | 0 | 0 | 0 | 1 | 0 | 0 | 0 | 0 | 0 | 0 | 0 | 0 | 0 | 0 | 0 | 0 | 0 | 0 | 0 | | | | | | |
| 3273 | 03844 | 03272 | 50 | | 0 | 0 | 0 | 0 | 0 | 0 | 0 | 0 | 0 | 25 | 0 | 0 | 0 | 0 | 8 | 3 | 0 | 9 | 0 | 0 | 5 | 0 | 0 | 0 | 0 | J03464 | 96.9 | 162 | 1 | 4723 | 5416 |
| 3274 | 03845 | 03273 | 1 | | 0 | 0 | 0 | 0 | 0 | 0 | 0 | 0 | 0 | 1 | 0 | 0 | 0 | 0 | 0 | 0 | 0 | 0 | 0 | 0 | 0 | 0 | 0 | 0 | 0 | | | | | | |
| 3275 | 03846 | 03274 | 1 | | 0 | 0 | 0 | 0 | 0 | 0 | 0 | 0 | 0 | 1 | 0 | 0 | 0 | 0 | 0 | 0 | 0 | 0 | 0 | 0 | 0 | 0 | 0 | 0 | 0 | | | | | | |
| 3276 | 03847 | 03275 | 2 | | 0 | 0 | 0 | 0 | 0 | 0 | 0 | 0 | 0 | 1 | 0 | 0 | 0 | 0 | 0 | 0 | 0 | 0 | 0 | 0 | 1 | 0 | 0 | 0 | 0 | L08246 | 97.5 | 163 | 1 | 2385 | 3934 |
| 3277 | 03848 | 03276 | 2 | | 0 | 0 | 0 | 0 | 0 | 0 | 0 | 0 | 0 | 1 | 0 | 0 | 0 | 0 | 0 | 1 | 0 | 0 | 0 | 0 | 0 | 0 | 0 | 0 | 0 | | | | | | |
| 3278 | 03849 | 03277 | 3 | | 0 | 0 | 0 | 0 | 0 | 0 | 0 | 0 | 0 | 1 | 0 | 0 | 0 | 0 | 0 | 1 | 0 | 0 | 0 | 0 | 1 | 0 | 0 | 0 | 0 | | | | | | |
| 3279 | 03850 | 03278 | 2 | | 0 | 0 | 0 | 0 | 0 | 0 | 0 | 0 | 0 | 2 | 0 | 0 | 0 | 0 | 0 | 0 | 0 | 0 | 0 | 0 | 0 | 0 | 0 | 0 | 0 | | | | | | |
| 3280 | 03851 | 03279 | 1 | | 0 | 0 | 0 | 0 | 0 | 0 | 0 | 0 | 0 | 1 | 0 | 0 | 0 | 0 | 0 | 0 | 0 | 0 | 0 | 0 | 0 | 0 | 0 | 0 | 0 | | | | | | |
| 3281 | 03852 | 03280 | 2 | | 0 | 0 | 0 | 0 | 0 | 0 | 0 | 0 | 0 | 1 | 0 | 0 | 0 | 0 | 0 | 0 | 0 | 0 | 0 | 1 | 0 | 0 | 0 | 0 | 0 | | | | | | |
| 3282 | 03853 | 03281 | 2 | | 0 | 0 | 0 | 0 | 0 | 0 | 0 | 0 | 0 | 1 | 0 | 0 | 1 | 0 | 0 | 0 | 0 | 0 | 0 | 0 | 0 | 0 | 0 | 0 | 0 | | | | | | |
| 3283 | 03854 | 03282 | 2 | | 0 | 0 | 0 | 0 | 0 | 0 | 0 | 0 | 0 | 2 | 0 | 0 | 0 | 0 | 0 | 0 | 0 | 0 | 0 | 0 | 0 | 0 | 0 | 0 | 0 | | | | | | |
| 3284 | 03855 | 03283 | 1 | | 0 | 0 | 0 | 0 | 0 | 0 | 0 | 0 | 0 | 1 | 0 | 0 | 0 | 0 | 0 | 0 | 0 | 0 | 0 | 0 | 0 | 0 | 0 | 0 | 0 | | | | | | |
| 3285 | 03856 | 03284 | 2 | | 0 | 0 | 0 | 0 | 0 | 0 | 0 | 0 | 0 | 1 | 0 | 0 | 0 | 0 | 1 | 0 | 0 | 0 | 0 | 0 | 0 | 0 | 0 | 0 | 0 | | | | | | |
| 3286 | 03857 | 03285 | 2 | | 0 | 0 | 0 | 0 | 0 | 0 | 0 | 0 | 0 | 1 | 0 | 0 | 0 | 0 | 1 | 0 | 0 | 0 | 0 | 0 | 0 | 0 | 0 | 0 | 0 | | | | | | |
| 3287 | 03859 | 03286 | 1 | | 0 | 0 | 0 | 0 | 0 | 0 | 0 | 0 | 0 | 1 | 0 | 0 | 0 | 0 | 0 | 0 | 0 | 0 | 0 | 0 | 0 | 0 | 0 | 0 | 0 | | | | | | |
| 3288 | 03860 | 03287 | 2 | | 0 | 0 | 0 | 0 | 0 | 0 | 0 | 0 | 0 | 1 | 0 | 0 | 0 | 0 | 0 | 0 | 1 | 0 | 0 | 0 | 0 | 0 | 0 | 0 | 0 | | | | | | |
| 3289 | 03861 | 03288 | 1 | | 0 | 0 | 0 | 0 | 0 | 0 | 0 | 0 | 0 | 1 | 0 | 0 | 0 | 0 | 0 | 0 | 0 | 0 | 0 | 0 | 0 | 0 | 0 | 0 | 0 | | | | | | |
| 3290 | 03862 | 03289 | 1 | | 0 | 0 | 0 | 0 | 0 | 0 | 0 | 0 | 0 | 1 | 0 | 0 | 0 | 0 | 0 | 0 | 0 | 0 | 0 | 0 | 0 | 0 | 0 | 0 | 0 | | | | | | |
| 3291 | 03863 | 03290 | 3 | | 0 | 0 | 0 | 0 | 0 | 0 | 0 | 0 | 0 | 2 | 0 | 0 | 0 | 0 | 0 | 0 | 0 | 0 | 1 | 0 | 0 | 0 | 0 | 0 | 0 | U02556 | 96.7 | 151 | 1 | 2007 | 2156 |
| 3292 | 03864 | 03291 | 6 | | 0 | 0 | 0 | 0 | 0 | 0 | 0 | 0 | 0 | 1 | 1 | 2 | 0 | 0 | 1 | 0 | 0 | 0 | 0 | 0 | 0 | 0 | 1 | 0 | 0 | L13803 | 97.1 | 278 | 1 | 35 | 312 |
| 3293 | 03865 | 03292 | 2 | | 0 | 0 | 0 | 0 | 0 | 0 | 0 | 0 | 0 | 1 | 0 | 0 | 0 | 0 | 0 | 0 | 0 | 0 | 0 | 0 | 1 | 0 | 0 | 0 | 0 | | | | | | |
| 3294 | 03866 | 03293 | 3 | | 0 | 0 | 0 | 0 | 0 | 0 | 0 | 0 | 0 | 1 | 0 | 0 | 0 | 0 | 0 | 0 | 0 | 0 | 0 | 2 | 0 | 0 | 0 | 0 | 0 | | | | | | |
| 3295 | 03867 | 03294 | 20 | | 0 | 0 | 0 | 0 | 0 | 0 | 0 | 0 | 0 | 2 | 1 | 0 | 0 | 0 | 5 | 3 | 3 | 1 | 0 | 0 | 0 | 5 | 0 | 0 | 0 | M38591 | 99.3 | 145 | 1 | 505 | 649 |
| 3296 | 03868 | 03295 | 1 | | 0 | 0 | 0 | 0 | 0 | 0 | 0 | 0 | 0 | 1 | 0 | 0 | 0 | 0 | 0 | 0 | 0 | 0 | 0 | 0 | 0 | 0 | 0 | 0 | 0 | | | | | | |
| 3297 | 03869 | 03296 | 7 | | 0 | 0 | 0 | 0 | 0 | 0 | 0 | 0 | 0 | 1 | 0 | 0 | 2 | 1 | 0 | 0 | 0 | 0 | 1 | 1 | 1 | 0 | 0 | 0 | 0 | | | | | | |

Table 92

| | A | B | C | E | G | I | K | M | O | Q | S | U | W | Y | AA | AC | AE | AG | AI | AK | AM | AO | AQ | AS | AU | AW | AY | BA | BC | BE | BF | BG | BH | BI | BK |
|---|---|---|---|---|---|---|---|---|---|---|---|---|---|---|---|---|---|---|---|---|---|---|---|---|---|---|---|---|---|---|---|---|---|---|---|
| 3298 | 03870 | 03297 | 1 | | 0 | 0 | 0 | 0 | 0 | 0 | 0 | 0 | 0 | 1 | 0 | 0 | 0 | 0 | 0 | 0 | 0 | 0 | 0 | 0 | 0 | 0 | 0 | 0 | 0 | | | | | | |
| 3299 | 03871 | 03298 | 3 | | 0 | 0 | 0 | 0 | 0 | 0 | 0 | 0 | 0 | 1 | 0 | 0 | 0 | 0 | 0 | 0 | 0 | 1 | 0 | 0 | 0 | 1 | 0 | 0 | 0 | | | | | | |
| 3300 | 03872 | 03299 | 1 | | 0 | 0 | 0 | 0 | 0 | 0 | 0 | 0 | 0 | 1 | 0 | 0 | 0 | 0 | 0 | 0 | 0 | 0 | 0 | 0 | 0 | 0 | 0 | 0 | 0 | | | | | | |
| 3301 | 03873 | 03300 | 1 | | 0 | 0 | 0 | 0 | 0 | 0 | 0 | 0 | 0 | 1 | 0 | 0 | 0 | 0 | 0 | 0 | 0 | 0 | 0 | 0 | 0 | 0 | 0 | 0 | 0 | | | | | | |
| 3302 | 03874 | 03301 | 1 | | 0 | 0 | 0 | 0 | 0 | 0 | 0 | 0 | 0 | 1 | 0 | 0 | 0 | 0 | 0 | 0 | 0 | 0 | 0 | 0 | 0 | 0 | 0 | 0 | 0 | | | | | | |
| 3303 | 03875 | 03302 | 2 | | 0 | 0 | 0 | 0 | 0 | 0 | 0 | 0 | 0 | 1 | 0 | 0 | 0 | 0 | 0 | 0 | 0 | 1 | 0 | 0 | 0 | 0 | 0 | 0 | 0 | | | | | | |
| 3304 | 03876 | 03303 | 5 | | 0 | 0 | 0 | 0 | 0 | 0 | 0 | 0 | 0 | 1 | 1 | 0 | 1 | 0 | 0 | 0 | 0 | 0 | 0 | 0 | 1 | 1 | 0 | 0 | 0 | | | | | | |
| 3305 | 03877 | 03304 | 2 | | 0 | 0 | 0 | 0 | 0 | 0 | 0 | 0 | 0 | 1 | 1 | 0 | 0 | 0 | 0 | 0 | 0 | 0 | 0 | 0 | 0 | 0 | 0 | 0 | 0 | | | | | | |
| 3306 | 03878 | 03305 | 1 | | 0 | 0 | 0 | 0 | 0 | 0 | 0 | 0 | 0 | 1 | 0 | 0 | 0 | 0 | 0 | 0 | 0 | 0 | 0 | 0 | 0 | 0 | 0 | 0 | 0 | | | | | | |
| 3307 | 03879 | 03306 | 7 | | 0 | 0 | 0 | 0 | 0 | 0 | 0 | 0 | 0 | 3 | 0 | 0 | 0 | 0 | 0 | 0 | 0 | 3 | 0 | 0 | 1 | 0 | 0 | 0 | 0 | | | | | | |
| 3308 | 03880 | 03307 | 1 | | 0 | 0 | 0 | 0 | 0 | 0 | 0 | 0 | 0 | 1 | 0 | 0 | 0 | 0 | 0 | 0 | 0 | 0 | 0 | 0 | 0 | 0 | 0 | 0 | 0 | | | | | | |
| 3309 | 03881 | 03308 | 2 | | 0 | 0 | 0 | 0 | 0 | 0 | 0 | 0 | 0 | 2 | 0 | 0 | 0 | 0 | 0 | 0 | 0 | 0 | 0 | 0 | 0 | 0 | 0 | 0 | 0 | | | | | | |
| 3310 | 03882 | 03309 | 1 | | 0 | 0 | 0 | 0 | 0 | 0 | 0 | 0 | 0 | 1 | 0 | 0 | 0 | 0 | 0 | 0 | 0 | 0 | 0 | 0 | 0 | 0 | 0 | 0 | 0 | | | | | | |
| 3311 | 03883 | 03310 | 1 | | 0 | 0 | 0 | 0 | 0 | 0 | 0 | 0 | 0 | 1 | 0 | 0 | 0 | 0 | 0 | 0 | 0 | 0 | 0 | 0 | 0 | 0 | 0 | 0 | 0 | | | | | | |
| 3312 | 03884 | 03311 | 2 | | 0 | 0 | 0 | 0 | 0 | 0 | 0 | 0 | 0 | 2 | 0 | 0 | 0 | 0 | 0 | 0 | 0 | 0 | 0 | 0 | 0 | 0 | 0 | 0 | 0 | | | | | | |
| 3313 | 03885 | 03312 | 1 | | 0 | 0 | 0 | 0 | 0 | 0 | 0 | 0 | 0 | 1 | 0 | 0 | 0 | 0 | 0 | 0 | 0 | 0 | 0 | 0 | 0 | 0 | 0 | 0 | 0 | | | | | | |
| 3314 | 03886 | 03313 | 1 | | 0 | 0 | 0 | 0 | 0 | 0 | 0 | 0 | 0 | 1 | 0 | 0 | 0 | 0 | 0 | 0 | 0 | 0 | 0 | 0 | 0 | 0 | 0 | 0 | 0 | | | | | | |
| 3315 | 03887 | 03314 | 2 | | 0 | 0 | 0 | 0 | 0 | 0 | 0 | 0 | 0 | 2 | 0 | 0 | 0 | 0 | 0 | 0 | 0 | 0 | 0 | 0 | 0 | 0 | 0 | 0 | 0 | | | | | | |
| 3316 | 03888 | 03315 | 5 | | 0 | 0 | 0 | 0 | 0 | 0 | 0 | 0 | 0 | 1 | 0 | 0 | 0 | 0 | 0 | 0 | 3 | 0 | 0 | 0 | 0 | 1 | 0 | 0 | 0 | | | | | | |
| 3317 | 03889 | 03316 | 4 | | 0 | 0 | 0 | 0 | 0 | 0 | 0 | 0 | 0 | 1 | 2 | 0 | 0 | 0 | 0 | 0 | 0 | 0 | 0 | 1 | 0 | 0 | 0 | 0 | 0 | | | | | | |
| 3318 | 03890 | 03317 | 3 | | 0 | 0 | 0 | 0 | 0 | 0 | 0 | 0 | 0 | 1 | 0 | 0 | 0 | 2 | 0 | 0 | 0 | 0 | 0 | 0 | 0 | 0 | 0 | 0 | 0 | | | | | | |
| 3319 | 03891 | 03318 | 1 | | 0 | 0 | 0 | 0 | 0 | 0 | 0 | 0 | 0 | 1 | 0 | 0 | 0 | 0 | 0 | 0 | 0 | 0 | 0 | 0 | 0 | 0 | 0 | 0 | 0 | | | | | | |
| 3320 | 03892 | 03319 | 1 | | 0 | 0 | 0 | 0 | 0 | 0 | 0 | 0 | 0 | 1 | 0 | 0 | 0 | 0 | 0 | 0 | 0 | 0 | 0 | 0 | 0 | 0 | 0 | 0 | 0 | | | | | | |
| 3321 | 03893 | 03320 | 1 | | 0 | 0 | 0 | 0 | 0 | 0 | 0 | 0 | 0 | 1 | 0 | 0 | 0 | 0 | 0 | 0 | 0 | 0 | 0 | 0 | 0 | 0 | 0 | 0 | 0 | | | | | | |
| 3322 | 03894 | 03321 | 5 | | 0 | 0 | 0 | 0 | 0 | 0 | 0 | 0 | 0 | 2 | 0 | 0 | 0 | 0 | 0 | 0 | 0 | 0 | 1 | 2 | 0 | 0 | 0 | 0 | 0 | | | | | | |
| 3323 | 03895 | 03322 | 1 | | 0 | 0 | 0 | 0 | 0 | 0 | 0 | 0 | 0 | 1 | 0 | 0 | 0 | 0 | 0 | 0 | 0 | 0 | 0 | 0 | 0 | 0 | 0 | 0 | 0 | | | | | | |
| 3324 | 03896 | 03323 | 7 | | 0 | 0 | 0 | 0 | 0 | 0 | 0 | 0 | 0 | 1 | 1 | 1 | 1 | 0 | 2 | 0 | 0 | 0 | 0 | 0 | 0 | 0 | 0 | 1 | 0 | D10522 | 96.1 | 407 | 1 | 1997 | 2589 |
| 3325 | 03897 | 03324 | 1 | | 0 | 0 | 0 | 0 | 0 | 0 | 0 | 0 | 0 | 1 | 0 | 0 | 0 | 0 | 0 | 0 | 0 | 0 | 0 | 0 | 0 | 0 | 0 | 0 | 0 | | | | | | |
| 3326 | 03898 | 03325 | 1 | | 0 | 0 | 0 | 0 | 0 | 0 | 0 | 0 | 0 | 1 | 0 | 0 | 0 | 0 | 0 | 0 | 0 | 0 | 0 | 0 | 0 | 0 | 0 | 0 | 0 | | | | | | |
| 3327 | 03899 | 03326 | 4 | | 0 | 0 | 0 | 0 | 0 | 0 | 0 | 0 | 0 | 1 | 0 | 0 | 0 | 0 | 0 | 0 | 0 | 0 | 1 | 1 | 1 | 0 | 0 | 0 | 0 | | | | | | |
| 3328 | 03900 | 03327 | 2 | | 0 | 0 | 0 | 0 | 0 | 0 | 0 | 0 | 0 | 2 | 0 | 0 | 0 | 0 | 0 | 0 | 0 | 0 | 0 | 0 | 0 | 0 | 0 | 0 | 0 | | | | | | |
| 3329 | 03901 | 03328 | 5 | | 0 | 0 | 0 | 0 | 0 | 0 | 0 | 0 | 0 | 1 | 0 | 1 | 2 | 0 | 0 | 0 | 0 | 0 | 1 | 0 | 0 | 0 | 0 | 0 | 0 | | | | | | |
| 3330 | 03902 | 03329 | 1 | | 0 | 0 | 0 | 0 | 0 | 0 | 0 | 0 | 0 | 1 | 0 | 0 | 0 | 0 | 0 | 0 | 0 | 0 | 0 | 0 | 0 | 0 | 0 | 0 | 0 | | | | | | |
| 3331 | 03903 | 03330 | 1 | | 0 | 0 | 0 | 0 | 0 | 0 | 0 | 0 | 0 | 1 | 0 | 0 | 0 | 0 | 0 | 0 | 0 | 0 | 0 | 0 | 0 | 0 | 0 | 0 | 0 | | | | | | |
| 3332 | 03904 | 03331 | 5 | | 0 | 0 | 0 | 0 | 0 | 0 | 0 | 0 | 0 | 1 | 0 | 0 | 0 | 0 | 0 | 0 | 0 | 0 | 1 | 3 | 0 | 0 | 0 | 0 | 0 | | | | | | |
| 3333 | 03905 | 03332 | 5 | | 0 | 0 | 0 | 0 | 0 | 0 | 0 | 0 | 0 | 2 | 0 | 0 | 0 | 1 | 0 | 0 | 0 | 0 | 2 | 0 | 0 | 0 | 0 | 0 | 0 | | | | | | |

Table 93

| | A | B | C | E | G | I | K | M | O | Q | S | U | W | Y | AA | AC | AE | AG | AI | AK | AM | AO | AQ | AS | AU | AW | AY | BA | BC | BE | BF | BG | BH | BI | BK |
|---|---|---|---|---|---|---|---|---|---|---|---|---|---|---|---|---|---|---|---|---|---|---|---|---|---|---|---|---|---|---|---|---|---|---|---|
| 3334 | 03906 | 03333 | 1 | | 0 | 0 | 0 | 0 | 0 | 0 | 0 | 0 | 0 | 1 | 0 | 0 | 0 | 0 | 0 | 0 | 0 | 0 | 0 | 0 | 0 | 0 | 0 | 0 | 0 | | | | | | |
| 3335 | 03907 | 03334 | 2 | | 0 | 0 | 0 | 0 | 0 | 0 | 0 | 0 | 0 | 1 | 0 | 0 | 0 | 0 | 0 | 0 | 0 | 1 | 0 | 0 | 0 | 0 | 0 | 0 | 0 | X63556 | 100 | 116 | 1 | 9804 | 9940 |
| 3336 | 03908 | 03335 | 2 | | 0 | 0 | 0 | 0 | 0 | 0 | 0 | 0 | 0 | 1 | 1 | 0 | 0 | 0 | 0 | 0 | 0 | 0 | 0 | 0 | 0 | 0 | 0 | 0 | 0 | | | | | | |
| 3337 | 03909 | 03336 | 2 | | 0 | 0 | 0 | 0 | 0 | 0 | 0 | 0 | 0 | 1 | 0 | 0 | 0 | 0 | 0 | 0 | 1 | 0 | 0 | 0 | 0 | 0 | 0 | 0 | 0 | M14676 | 94.5 | 273 | 1 | 2088 | 2435 |
| 3338 | 03910 | 03337 | 1 | | 0 | 0 | 0 | 0 | 0 | 0 | 0 | 0 | 0 | 1 | 0 | 0 | 0 | 0 | 0 | 0 | 0 | 0 | 0 | 0 | 0 | 0 | 0 | 0 | 0 | | | | | | |
| 3339 | 03911 | 03338 | 1 | | 0 | 0 | 0 | 0 | 0 | 0 | 0 | 0 | 0 | 1 | 0 | 0 | 0 | 0 | 0 | 0 | 0 | 0 | 0 | 0 | 0 | 0 | 0 | 0 | 0 | | | | | | |
| 3340 | 03912 | 03339 | 1 | | 0 | 0 | 0 | 0 | 0 | 0 | 0 | 0 | 0 | 1 | 0 | 0 | 0 | 0 | 0 | 0 | 0 | 0 | 0 | 0 | 0 | 0 | 0 | 0 | 0 | | | | | | |
| 3341 | 03913 | 03340 | 1 | | 0 | 0 | 0 | 0 | 0 | 0 | 0 | 0 | 0 | 1 | 0 | 0 | 0 | 0 | 0 | 0 | 0 | 0 | 0 | 0 | 0 | 0 | 0 | 0 | 0 | | | | | | |
| 3342 | 03914 | 03341 | 4 | | 0 | 0 | 0 | 0 | 0 | 0 | 0 | 0 | 0 | 1 | 0 | 0 | 0 | 0 | 0 | 0 | 2 | 0 | 1 | 0 | 0 | 0 | 0 | 0 | 0 | S56151 | 93.2 | 293 | 1 | 979 | 1270 |
| 3343 | 03915 | 03342 | 1 | | 0 | 0 | 0 | 0 | 0 | 0 | 0 | 0 | 0 | 1 | 0 | 0 | 0 | 0 | 0 | 0 | 0 | 0 | 0 | 0 | 0 | 0 | 0 | 0 | 0 | | | | | | |
| 3344 | 03916 | 03343 | 1 | | 0 | 0 | 0 | 0 | 0 | 0 | 0 | 0 | 0 | 1 | 0 | 0 | 0 | 0 | 0 | 0 | 0 | 0 | 0 | 0 | 0 | 0 | 0 | 0 | 0 | | | | | | |
| 3345 | 03917 | 03344 | 5 | | 0 | 0 | 0 | 0 | 0 | 0 | 0 | 0 | 0 | 1 | 0 | 0 | 1 | 0 | 0 | 0 | 0 | 0 | 0 | 3 | 0 | 0 | 0 | 0 | 0 | | | | | | |
| 3346 | 03918 | 03345 | 3 | | 0 | 0 | 0 | 0 | 0 | 0 | 0 | 0 | 0 | 1 | 0 | 0 | 0 | 0 | 0 | 0 | 0 | 2 | 0 | 0 | 0 | 0 | 0 | 0 | 0 | | | | | | |
| 3347 | 03919 | 03346 | 3 | | 0 | 0 | 0 | 0 | 0 | 0 | 0 | 0 | 0 | 2 | 1 | 0 | 0 | 0 | 0 | 0 | 0 | 0 | 0 | 0 | 0 | 0 | 0 | 0 | 0 | L12350 | 94.7 | 113 | 1 | 5395 | 5784 |
| 3348 | 03920 | 03347 | 1 | | 0 | 0 | 0 | 0 | 0 | 0 | 0 | 0 | 0 | 1 | 0 | 0 | 0 | 0 | 0 | 0 | 0 | 0 | 0 | 0 | 0 | 0 | 0 | 0 | 0 | | | | | | |
| 3349 | 03921 | 03348 | 1 | | 0 | 0 | 0 | 0 | 0 | 0 | 0 | 0 | 0 | 1 | 0 | 0 | 0 | 0 | 0 | 0 | 0 | 0 | 0 | 0 | 0 | 0 | 0 | 0 | 0 | | | | | | |
| 3350 | 03922 | 03349 | 1 | | 0 | 0 | 0 | 0 | 0 | 0 | 0 | 0 | 0 | 1 | 0 | 0 | 0 | 0 | 0 | 0 | 0 | 0 | 0 | 0 | 0 | 0 | 0 | 0 | 0 | | | | | | |
| 3351 | 03923 | 03350 | 2 | | 0 | 0 | 0 | 0 | 0 | 0 | 0 | 0 | 0 | 1 | 0 | 0 | 0 | 0 | 0 | 0 | 1 | 0 | 0 | 0 | 0 | 0 | 0 | 0 | 0 | | | | | | |
| 3352 | 03924 | 03351 | 1 | | 0 | 0 | 0 | 0 | 0 | 0 | 0 | 0 | 0 | 1 | 0 | 0 | 0 | 0 | 0 | 0 | 0 | 0 | 0 | 0 | 0 | 0 | 0 | 0 | 0 | | | | | | |
| 3353 | 03925 | 03352 | 4 | | 0 | 0 | 0 | 0 | 0 | 0 | 0 | 0 | 0 | 1 | 0 | 0 | 0 | 0 | 1 | 0 | 1 | 0 | 0 | 0 | 0 | 0 | 0 | 1 | 0 | | | | | | |
| 3354 | 03926 | 03353 | 1 | | 0 | 0 | 0 | 0 | 0 | 0 | 0 | 0 | 0 | 1 | 0 | 0 | 0 | 0 | 0 | 0 | 0 | 0 | 0 | 0 | 0 | 0 | 0 | 0 | 0 | | | | | | |
| 3355 | 03927 | 03354 | 1 | | 0 | 0 | 0 | 0 | 0 | 0 | 0 | 0 | 0 | 1 | 0 | 0 | 0 | 0 | 0 | 0 | 0 | 0 | 0 | 0 | 0 | 0 | 0 | 0 | 0 | | | | | | |
| 3356 | 03928 | 03355 | 1 | | 0 | 0 | 0 | 0 | 0 | 0 | 0 | 0 | 0 | 1 | 0 | 0 | 0 | 0 | 0 | 0 | 0 | 0 | 0 | 0 | 0 | 0 | 0 | 0 | 0 | | | | | | |
| 3357 | 03929 | 03356 | 1 | | 0 | 0 | 0 | 0 | 0 | 0 | 0 | 0 | 0 | 1 | 0 | 0 | 0 | 0 | 0 | 0 | 0 | 0 | 0 | 0 | 0 | 0 | 0 | 0 | 0 | | | | | | |
| 3358 | 03930 | 03357 | 2 | | 0 | 0 | 0 | 0 | 0 | 0 | 0 | 0 | 0 | 1 | 0 | 0 | 1 | 0 | 0 | 0 | 0 | 0 | 0 | 0 | 0 | 0 | 0 | 0 | 0 | | | | | | |
| 3359 | 03931 | 03358 | 1 | | 0 | 0 | 0 | 0 | 0 | 0 | 0 | 0 | 0 | 1 | 0 | 0 | 0 | 0 | 0 | 0 | 0 | 0 | 0 | 0 | 0 | 0 | 0 | 0 | 0 | X15880 | 93.6 | 110 | 1 | 1128 | 2291 |
| 3360 | 03932 | 03359 | 5 | | 0 | 0 | 0 | 0 | 0 | 0 | 0 | 0 | 0 | 1 | 0 | 0 | 0 | 0 | 2 | 0 | 0 | 0 | 0 | 1 | 0 | 1 | 0 | 0 | 0 | | | | | | |
| 3361 | 03933 | 03360 | 3 | | 0 | 0 | 0 | 0 | 0 | 0 | 0 | 0 | 0 | 1 | 0 | 1 | 0 | 0 | 0 | 0 | 0 | 0 | 0 | 0 | 0 | 1 | 0 | 0 | 0 | | | | | | |
| 3362 | 03934 | 03361 | 1 | | 0 | 0 | 0 | 0 | 0 | 0 | 0 | 0 | 0 | 1 | 0 | 0 | 0 | 0 | 0 | 0 | 0 | 0 | 0 | 0 | 0 | 0 | 0 | 0 | 0 | L13977 | 91.7 | 109 | 1 | 1481 | 2060 |
| 3363 | 03935 | 03362 | 1 | | 0 | 0 | 0 | 0 | 0 | 0 | 0 | 0 | 0 | 1 | 0 | 0 | 0 | 0 | 0 | 0 | 0 | 0 | 0 | 0 | 0 | 0 | 0 | 0 | 0 | | | | | | |
| 3364 | 03936 | 03363 | 2 | | 0 | 0 | 0 | 0 | 0 | 0 | 0 | 0 | 0 | 1 | 0 | 0 | 0 | 0 | 0 | 0 | 0 | 0 | 0 | 1 | 0 | 0 | 0 | 0 | 0 | | | | | | |
| 3365 | 03937 | 03364 | 1 | | 0 | 0 | 0 | 0 | 0 | 0 | 0 | 0 | 0 | 1 | 0 | 0 | 0 | 0 | 0 | 0 | 0 | 0 | 0 | 0 | 0 | 0 | 0 | 0 | 0 | | | | | | |
| 3366 | 03938 | 03365 | 1 | | 0 | 0 | 0 | 0 | 0 | 0 | 0 | 0 | 0 | 1 | 0 | 0 | 0 | 0 | 0 | 0 | 0 | 0 | 0 | 0 | 0 | 0 | 0 | 0 | 0 | | | | | | |
| 3367 | 03939 | 03366 | 1 | | 0 | 0 | 0 | 0 | 0 | 0 | 0 | 0 | 0 | 1 | 0 | 0 | 0 | 0 | 0 | 0 | 0 | 0 | 0 | 0 | 0 | 0 | 0 | 0 | 0 | | | | | | |
| 3368 | 03940 | 03367 | 1 | | 0 | 0 | 0 | 0 | 0 | 0 | 0 | 0 | 0 | 1 | 0 | 0 | 0 | 0 | 0 | 0 | 0 | 0 | 0 | 0 | 0 | 0 | 0 | 0 | 0 | X16434 | 95.8 | 96 | 1 | 647 | 742 |
| 3369 | 03941 | 03368 | 1 | | 0 | 0 | 0 | 0 | 0 | 0 | 0 | 0 | 0 | 1 | 0 | 0 | 0 | 0 | 0 | 0 | 0 | 0 | 0 | 0 | 0 | 0 | 0 | 0 | 0 | | | | | | |

Table 94

| | A | B | C | E | G | I | K | M | O | Q | S | U | W | Y | AA | AC | AE | AG | AI | AK | AM | AO | AQ | AS | AU | AW | AY | BA | BC | BE | BF | BG | BH | BI | BK |
|---|---|---|---|---|---|---|---|---|---|---|---|---|---|---|---|---|---|---|---|---|---|---|---|---|---|---|---|---|---|---|---|---|---|---|---|
| 3370 | 03942 | 03369 | 1 | | 0 | 0 | 0 | 0 | 0 | 0 | 0 | 0 | 0 | 1 | 0 | 0 | 0 | 0 | 0 | 0 | 0 | 0 | 0 | 0 | 0 | 0 | 0 | 0 | 0 | | | | | | |
| 3371 | 03943 | 03370 | 1 | | 0 | 0 | 0 | 0 | 0 | 0 | 0 | 0 | 0 | 1 | 0 | 0 | 0 | 0 | 0 | 0 | 0 | 0 | 0 | 0 | 0 | 0 | 0 | 0 | 0 | | | | | | |
| 3372 | 03944 | 03371 | 1 | | 0 | 0 | 0 | 0 | 0 | 0 | 0 | 0 | 0 | 1 | 0 | 0 | 0 | 0 | 0 | 0 | 0 | 0 | 0 | 0 | 0 | 0 | 0 | 0 | 0 | | | | | | |
| 3373 | 03945 | 03372 | 1 | | 0 | 0 | 0 | 0 | 0 | 0 | 0 | 0 | 0 | 1 | 0 | 0 | 0 | 0 | 0 | 0 | 0 | 0 | 0 | 0 | 0 | 0 | 0 | 0 | 0 | | | | | | |
| 3374 | 03946 | 03373 | 2 | | 0 | 0 | 0 | 0 | 0 | 0 | 0 | 0 | 0 | 1 | 0 | 0 | 0 | 0 | 0 | 0 | 0 | 0 | 0 | 0 | 0 | 0 | 1 | 0 | 0 | | | | | | |
| 3375 | 03947 | 03374 | 1 | | 0 | 0 | 0 | 0 | 0 | 0 | 0 | 0 | 0 | 1 | 0 | 0 | 0 | 0 | 0 | 0 | 0 | 0 | 0 | 0 | 0 | 0 | 0 | 0 | 0 | | | | | | |
| 3376 | 03948 | 03375 | 3 | | 0 | 0 | 0 | 0 | 0 | 0 | 0 | 0 | 0 | 1 | 0 | 1 | 0 | 0 | 0 | 0 | 0 | 0 | 1 | 0 | 0 | 0 | 0 | 0 | 0 | | | | | | |
| 3377 | 03949 | 03376 | 2 | | 0 | 0 | 0 | 0 | 0 | 0 | 0 | 0 | 0 | 1 | 0 | 0 | 0 | 0 | 0 | 0 | 0 | 1 | 0 | 0 | 0 | 0 | 0 | 0 | 0 | | | | | | |
| 3378 | 03951 | 03377 | 1 | | 0 | 0 | 0 | 0 | 0 | 0 | 0 | 0 | 0 | 1 | 0 | 0 | 0 | 0 | 0 | 0 | 0 | 0 | 0 | 0 | 0 | 0 | 0 | 0 | 0 | | | | | | |
| 3379 | 03952 | 03378 | 2 | | 0 | 0 | 0 | 0 | 0 | 0 | 0 | 0 | 0 | 1 | 0 | 0 | 0 | 0 | 0 | 1 | 0 | 0 | 0 | 0 | 0 | 0 | 0 | 0 | 0 | | | | | | |
| 3380 | 03954 | 03379 | 1 | | 0 | 0 | 0 | 0 | 0 | 0 | 0 | 0 | 0 | 1 | 0 | 0 | 0 | 0 | 0 | 0 | 0 | 0 | 0 | 0 | 0 | 0 | 0 | 0 | 0 | | | | | | |
| 3381 | 03955 | 03380 | 2 | | 0 | 0 | 0 | 0 | 0 | 0 | 0 | 0 | 0 | 2 | 0 | 0 | 0 | 0 | 0 | 0 | 0 | 0 | 0 | 0 | 0 | 0 | 0 | 0 | 0 | | | | | | |
| 3382 | 03956 | 03381 | 1 | | 0 | 0 | 0 | 0 | 0 | 0 | 0 | 0 | 0 | 1 | 0 | 0 | 0 | 0 | 0 | 0 | 0 | 0 | 0 | 0 | 0 | 0 | 0 | 0 | 0 | | | | | | |
| 3383 | 03958 | 03382 | 1 | | 0 | 0 | 0 | 0 | 0 | 0 | 0 | 0 | 0 | 1 | 0 | 0 | 0 | 0 | 0 | 0 | 0 | 0 | 0 | 0 | 0 | 0 | 0 | 0 | 0 | | | | | | |
| 3384 | 03959 | 03383 | 2 | | 0 | 0 | 0 | 0 | 0 | 0 | 0 | 0 | 0 | 2 | 0 | 0 | 0 | 0 | 0 | 0 | 0 | 0 | 0 | 0 | 0 | 0 | 0 | 0 | 0 | | | | | | |
| 3385 | 03960 | 03384 | 1 | | 0 | 0 | 0 | 0 | 0 | 0 | 0 | 0 | 0 | 1 | 0 | 0 | 0 | 0 | 0 | 0 | 0 | 0 | 0 | 0 | 0 | 0 | 0 | 0 | 0 | | | | | | |
| 3386 | 03961 | 03385 | 1 | | 0 | 0 | 0 | 0 | 0 | 0 | 0 | 0 | 0 | 1 | 0 | 0 | 0 | 0 | 0 | 0 | 0 | 0 | 0 | 0 | 0 | 0 | 0 | 0 | 0 | | | | | | |
| 3387 | 03962 | 03386 | 1 | | 0 | 0 | 0 | 0 | 0 | 0 | 0 | 0 | 0 | 1 | 0 | 0 | 0 | 0 | 0 | 0 | 0 | 0 | 0 | 0 | 0 | 0 | 0 | 0 | 0 | | | | | | |
| 3388 | 03963 | 03387 | 1 | | 0 | 0 | 0 | 0 | 0 | 0 | 0 | 0 | 0 | 1 | 0 | 0 | 0 | 0 | 0 | 0 | 0 | 0 | 0 | 0 | 0 | 0 | 0 | 0 | 0 | M76729 | 92.9 | 84 | 1 | 6245 | 7138 |
| 3389 | 03964 | 03388 | 1 | | 0 | 0 | 0 | 0 | 0 | 0 | 0 | 0 | 0 | 1 | 0 | 0 | 0 | 0 | 0 | 0 | 0 | 0 | 0 | 0 | 0 | 0 | 0 | 0 | 0 | | | | | | |
| 3390 | 03965 | 03389 | 1 | | 0 | 0 | 0 | 0 | 0 | 0 | 0 | 0 | 0 | 1 | 0 | 0 | 0 | 0 | 0 | 0 | 0 | 0 | 0 | 0 | 0 | 0 | 0 | 0 | 0 | | | | | | |
| 3391 | 03966 | 03390 | 15 | | 0 | 0 | 0 | 0 | 0 | 0 | 0 | 0 | 0 | 1 | 0 | 0 | 0 | 0 | 9 | 0 | 0 | 5 | 0 | 0 | 0 | 0 | 0 | 0 | 0 | M55618 | 95.5 | 88 | 1 | 7295 | 7390 |
| 3392 | 03967 | 03391 | 2 | | 0 | 0 | 0 | 0 | 0 | 0 | 0 | 0 | 0 | 1 | 0 | 0 | 0 | 0 | 0 | 0 | 0 | 0 | 0 | 0 | 0 | 1 | 0 | 0 | 0 | L13210 | 100 | 73 | 1 | 2185 | 2257 |
| 3393 | 03968 | 03392 | 3 | | 0 | 0 | 0 | 0 | 0 | 0 | 0 | 0 | 0 | 1 | 0 | 0 | 0 | 0 | 0 | 0 | 0 | 0 | 0 | 1 | 0 | 1 | 0 | 0 | 0 | | | | | | |
| 3394 | 03969 | 03393 | 1 | | 0 | 0 | 0 | 0 | 0 | 0 | 0 | 0 | 0 | 1 | 0 | 0 | 0 | 0 | 0 | 0 | 0 | 0 | 0 | 0 | 0 | 0 | 0 | 0 | 0 | | | | | | |
| 3395 | 03971 | 03394 | 8 | | 0 | 0 | 0 | 0 | 0 | 0 | 0 | 0 | 0 | 3 | 0 | 0 | 0 | 0 | 1 | 0 | 0 | 0 | 2 | 2 | 0 | 0 | 0 | 0 | 0 | D25274 | 95.7 | 69 | 1 | 1168 | 1279 |
| 3396 | 03972 | 03395 | 1 | | 0 | 0 | 0 | 0 | 0 | 0 | 0 | 0 | 0 | 1 | 0 | 0 | 0 | 0 | 0 | 0 | 0 | 0 | 0 | 0 | 0 | 0 | 0 | 0 | 0 | | | | | | |
| 3397 | 03973 | 03396 | 1 | | 0 | 0 | 0 | 0 | 0 | 0 | 0 | 0 | 0 | 1 | 0 | 0 | 0 | 0 | 0 | 0 | 0 | 0 | 0 | 0 | 0 | 0 | 0 | 0 | 0 | | | | | | |
| 3398 | 03974 | 03397 | 5 | | 0 | 0 | 0 | 0 | 0 | 0 | 0 | 0 | 0 | 1 | 1 | 2 | 0 | 0 | 0 | 0 | 0 | 0 | 0 | 1 | 0 | 0 | 0 | 0 | 0 | | | | | | |
| 3399 | 03975 | 03398 | 2 | | 0 | 0 | 0 | 0 | 0 | 0 | 0 | 0 | 0 | 1 | 0 | 0 | 0 | 0 | 1 | 0 | 0 | 0 | 0 | 0 | 0 | 0 | 0 | 0 | 0 | | | | | | |
| 3400 | 03976 | 03399 | 5 | | 0 | 0 | 0 | 0 | 0 | 0 | 0 | 0 | 0 | 3 | 0 | 0 | 0 | 0 | 1 | 0 | 0 | 0 | 0 | 0 | 1 | 0 | 0 | 0 | 0 | | | | | | |
| 3401 | 03977 | 03400 | 2 | | 0 | 0 | 0 | 0 | 0 | 0 | 0 | 0 | 0 | 1 | 0 | 1 | 0 | 0 | 0 | 0 | 0 | 0 | 0 | 0 | 0 | 0 | 0 | 0 | 0 | | | | | | |
| 3402 | 03978 | 03401 | 1 | | 0 | 0 | 0 | 0 | 0 | 0 | 0 | 0 | 0 | 1 | 0 | 0 | 0 | 0 | 0 | 0 | 0 | 0 | 0 | 0 | 0 | 0 | 0 | 0 | 0 | | | | | | |
| 3403 | 03979 | 03402 | 1 | | 0 | 0 | 0 | 0 | 0 | 0 | 0 | 0 | 0 | 1 | 0 | 0 | 0 | 0 | 0 | 0 | 0 | 0 | 0 | 0 | 0 | 0 | 0 | 0 | 0 | | | | | | |
| 3404 | 03980 | 03403 | 8 | | 0 | 0 | 0 | 0 | 0 | 0 | 0 | 0 | 0 | 3 | 0 | 0 | 0 | 1 | 0 | 0 | 0 | 1 | 0 | 0 | 0 | 3 | 0 | 0 | 0 | X52947 | 100 | 58 | 1 | 2981 | 3038 |
| 3405 | 03981 | 03404 | 1 | | 0 | 0 | 0 | 0 | 0 | 0 | 0 | 0 | 0 | 1 | 0 | 0 | 0 | 0 | 0 | 0 | 0 | 0 | 0 | 0 | 0 | 0 | 0 | 0 | 0 | | | | | | |

Table 95

| | A | B | C | E | G | I | K | M | O | Q | S | U | W | Y | AA | AC | AE | AG | AI | AK | AM | AO | AQ | AS | AU | AW | AY | BA | BC | BE | BF | BG | BH | BI | BK |
|---|---|---|---|---|---|---|---|---|---|---|---|---|---|---|---|---|---|---|---|---|---|---|---|---|---|---|---|---|---|---|---|---|---|---|---|
| 3406 | 03982 | 03405 | 1 | | 0 | 0 | 0 | 0 | 0 | 0 | 0 | 0 | 0 | 1 | 0 | 0 | 0 | 0 | 0 | 0 | 0 | 0 | 0 | 0 | 0 | 0 | 0 | 0 | 0 | | | | | | |
| 3407 | 03984 | 03406 | 2 | | 0 | 0 | 0 | 0 | 0 | 0 | 0 | 0 | 0 | 1 | 0 | 0 | 0 | 0 | 0 | 0 | 1 | 0 | 0 | 0 | 0 | 0 | 0 | 0 | 0 | | | | | | |
| 3408 | 03985 | 03407 | 5 | | 0 | 0 | 0 | 0 | 0 | 0 | 0 | 0 | 0 | 1 | 0 | 0 | 0 | 0 | 0 | 1 | 0 | 0 | 0 | 1 | 0 | 2 | 0 | 0 | 0 | | | | | | |
| 3409 | 03986 | 03408 | 1 | | 0 | 0 | 0 | 0 | 0 | 0 | 0 | 0 | 0 | 1 | 0 | 0 | 0 | 0 | 0 | 0 | 0 | 0 | 0 | 0 | 0 | 0 | 0 | 0 | 0 | | | | | | |
| 3410 | 03987 | 03409 | 2 | | 0 | 0 | 0 | 0 | 0 | 0 | 0 | 0 | 0 | 1 | 0 | 0 | 0 | 0 | 1 | 0 | 0 | 0 | 0 | 0 | 0 | 0 | 0 | 0 | 0 | | | | | | |
| 3411 | 03988 | 03410 | 1 | | 0 | 0 | 0 | 0 | 0 | 0 | 0 | 0 | 0 | 1 | 0 | 0 | 0 | 0 | 0 | 0 | 0 | 0 | 0 | 0 | 0 | 0 | 0 | 0 | 0 | | | | | | |
| 3412 | 03989 | 03411 | 5 | | 0 | 0 | 0 | 0 | 0 | 0 | 0 | 0 | 0 | 1 | 0 | 1 | 0 | 0 | 0 | 0 | 0 | 0 | 2 | 1 | 0 | 0 | 0 | 0 | 0 | | | | | | |
| 3413 | 03993 | 03412 | 5 | | 0 | 0 | 0 | 0 | 0 | 0 | 0 | 0 | 0 | 1 | 0 | 0 | 0 | 0 | 0 | 3 | 0 | 0 | 0 | 0 | 0 | 1 | 0 | 0 | 0 | | | | | | |
| 3414 | 04020 | 03413 | 2 | | 0 | 0 | 0 | 0 | 0 | 0 | 0 | 0 | 0 | 0 | 1 | 0 | 0 | 0 | 0 | 0 | 0 | 0 | 0 | 1 | 0 | 0 | 0 | 0 | 0 | | | | | | |
| 3415 | 04021 | 03414 | 4 | | 0 | 0 | 0 | 0 | 0 | 0 | 0 | 0 | 0 | 0 | 2 | 0 | 0 | 0 | 0 | 0 | 2 | 0 | 0 | 0 | 0 | 0 | 0 | 0 | 0 | | | | | | |
| 3416 | 04022 | 03415 | 1 | | 0 | 0 | 0 | 0 | 0 | 0 | 0 | 0 | 0 | 0 | 1 | 0 | 0 | 0 | 0 | 0 | 0 | 0 | 0 | 0 | 0 | 0 | 0 | 0 | 0 | D13641 | 93.5 | 431 | 1 | 2737 | 3259 |
| 3417 | 04023 | 03416 | 1 | | 0 | 0 | 0 | 0 | 0 | 0 | 0 | 0 | 0 | 0 | 1 | 0 | 0 | 0 | 0 | 0 | 0 | 0 | 0 | 0 | 0 | 0 | 0 | 0 | 0 | | | | | | |
| 3418 | 04024 | 03417 | 1 | | 0 | 0 | 0 | 0 | 0 | 0 | 0 | 0 | 0 | 0 | 1 | 0 | 0 | 0 | 0 | 0 | 0 | 0 | 0 | 0 | 0 | 0 | 0 | 0 | 0 | | | | | | |
| 3419 | 04025 | 03418 | 4 | | 0 | 0 | 0 | 0 | 0 | 0 | 0 | 0 | 0 | 0 | 1 | 0 | 0 | 0 | 0 | 0 | 0 | 1 | 0 | 1 | 0 | 1 | 0 | 0 | 0 | | | | | | |
| 3420 | 04026 | 03419 | 9 | | 0 | 0 | 0 | 0 | 0 | 0 | 0 | 0 | 0 | 0 | 2 | 0 | 0 | 1 | 0 | 1 | 2 | 2 | 1 | 0 | 0 | 0 | 0 | 0 | 0 | M11560 | 95 | 301 | 1 | 968 | 1464 |
| 3421 | 04028 | 03420 | 1 | | 0 | 0 | 0 | 0 | 0 | 0 | 0 | 0 | 0 | 0 | 1 | 0 | 0 | 0 | 0 | 0 | 0 | 0 | 0 | 0 | 0 | 0 | 0 | 0 | 0 | | | | | | |
| 3422 | 04029 | 03421 | 1 | | 0 | 0 | 0 | 0 | 0 | 0 | 0 | 0 | 0 | 0 | 1 | 0 | 0 | 0 | 0 | 0 | 0 | 0 | 0 | 0 | 0 | 0 | 0 | 0 | 0 | | | | | | |
| 3423 | 04030 | 03422 | 1 | | 0 | 0 | 0 | 0 | 0 | 0 | 0 | 0 | 0 | 0 | 1 | 0 | 0 | 0 | 0 | 0 | 0 | 0 | 0 | 0 | 0 | 0 | 0 | 0 | 0 | | | | | | |
| 3424 | 04031 | 03423 | 1 | | 0 | 0 | 0 | 0 | 0 | 0 | 0 | 0 | 0 | 0 | 1 | 0 | 0 | 0 | 0 | 0 | 0 | 0 | 0 | 0 | 0 | 0 | 0 | 0 | 0 | | | | | | |
| 3425 | 04032 | 03424 | 1 | | 0 | 0 | 0 | 0 | 0 | 0 | 0 | 0 | 0 | 0 | 1 | 0 | 0 | 0 | 0 | 0 | 0 | 0 | 0 | 0 | 0 | 0 | 0 | 0 | 0 | | | | | | |
| 3426 | 04033 | 03425 | 1 | | 0 | 0 | 0 | 0 | 0 | 0 | 0 | 0 | 0 | 0 | 1 | 0 | 0 | 0 | 0 | 0 | 0 | 0 | 0 | 0 | 0 | 0 | 0 | 0 | 0 | | | | | | |
| 3427 | 04034 | 03426 | 1 | | 0 | 0 | 0 | 0 | 0 | 0 | 0 | 0 | 0 | 0 | 1 | 0 | 0 | 0 | 0 | 0 | 0 | 0 | 0 | 0 | 0 | 0 | 0 | 0 | 0 | | | | | | |
| 3428 | 04035 | 03427 | 1 | | 0 | 0 | 0 | 0 | 0 | 0 | 0 | 0 | 0 | 0 | 1 | 0 | 0 | 0 | 0 | 0 | 0 | 0 | 0 | 0 | 0 | 0 | 0 | 0 | 0 | | | | | | |
| 3429 | 04036 | 03428 | 1 | | 0 | 0 | 0 | 0 | 0 | 0 | 0 | 0 | 0 | 0 | 1 | 0 | 0 | 0 | 0 | 0 | 0 | 0 | 0 | 0 | 0 | 0 | 0 | 0 | 0 | M80783 | 97.6 | 375 | 1 | 3139 | 3512 |
| 3430 | 04037 | 03429 | 1 | | 0 | 0 | 0 | 0 | 0 | 0 | 0 | 0 | 0 | 0 | 1 | 0 | 0 | 0 | 0 | 0 | 0 | 0 | 0 | 0 | 0 | 0 | 0 | 0 | 0 | | | | | | |
| 3431 | 04039 | 03430 | 1 | | 0 | 0 | 0 | 0 | 0 | 0 | 0 | 0 | 0 | 0 | 1 | 0 | 0 | 0 | 0 | 0 | 0 | 0 | 0 | 0 | 0 | 0 | 0 | 0 | 0 | D16494 | 95.6 | 367 | 1 | 2667 | 3155 |
| 3432 | 04040 | 03431 | 4 | | 0 | 0 | 0 | 0 | 0 | 0 | 0 | 0 | 0 | 0 | 2 | 0 | 0 | 0 | 2 | 0 | 0 | 0 | 0 | 0 | 0 | 0 | 0 | 0 | 0 | M83088 | 96.2 | 365 | 1 | 1957 | 2320 |
| 3433 | 04041 | 03432 | 3 | | 0 | 0 | 0 | 0 | 0 | 0 | 0 | 0 | 0 | 0 | 1 | 0 | 0 | 1 | 0 | 0 | 0 | 0 | 0 | 1 | 0 | 0 | 0 | 0 | 0 | | | | | | |
| 3434 | 04042 | 03433 | 1 | | 0 | 0 | 0 | 0 | 0 | 0 | 0 | 0 | 0 | 0 | 1 | 0 | 0 | 0 | 0 | 0 | 0 | 0 | 0 | 0 | 0 | 0 | 0 | 0 | 0 | | | | | | |
| 3435 | 04044 | 03434 | 2 | | 0 | 0 | 0 | 0 | 0 | 0 | 0 | 0 | 0 | 0 | 1 | 0 | 0 | 0 | 0 | 1 | 0 | 0 | 0 | 0 | 0 | 0 | 0 | 0 | 0 | | | | | | |
| 3436 | 04045 | 03435 | 2 | | 0 | 0 | 0 | 0 | 0 | 0 | 0 | 0 | 0 | 0 | 1 | 1 | 0 | 0 | 0 | 0 | 0 | 0 | 0 | 0 | 0 | 0 | 0 | 0 | 0 | | | | | | |
| 3437 | 04046 | 03436 | 1 | | 0 | 0 | 0 | 0 | 0 | 0 | 0 | 0 | 0 | 0 | 1 | 0 | 0 | 0 | 0 | 0 | 0 | 0 | 0 | 0 | 0 | 0 | 0 | 0 | 0 | | | | | | |
| 3438 | 04047 | 03437 | 3 | | 0 | 0 | 0 | 0 | 0 | 0 | 0 | 0 | 0 | 0 | 1 | 0 | 0 | 0 | 0 | 2 | 0 | 0 | 0 | 0 | 0 | 0 | 0 | 0 | 0 | | | | | | |
| 3439 | 04048 | 03438 | 1 | | 0 | 0 | 0 | 0 | 0 | 0 | 0 | 0 | 0 | 0 | 1 | 0 | 0 | 0 | 0 | 0 | 0 | 0 | 0 | 0 | 0 | 0 | 0 | 0 | 0 | | | | | | |
| 3440 | 04049 | 03439 | 1 | | 0 | 0 | 0 | 0 | 0 | 0 | 0 | 0 | 0 | 0 | 1 | 0 | 0 | 0 | 0 | 0 | 0 | 0 | 0 | 0 | 0 | 0 | 0 | 0 | 0 | S45936 | 95.7 | 350 | 1 | 2281 | 2687 |
| 3441 | 04050 | 03440 | 1 | | 0 | 0 | 0 | 0 | 0 | 0 | 0 | 0 | 0 | 0 | 1 | 0 | 0 | 0 | 0 | 0 | 0 | 0 | 0 | 0 | 0 | 0 | 0 | 0 | 0 | | | | | | |

Table 96

| | A | B | C | E | G | I | K | M | O | Q | S | U | W | Y | AA | AC | AE | AG | AI | AK | AM | AO | AQ | AS | AU | AW | AY | BA | BC | BE | BF | BG | BH | BI | BK |
|---|---|---|---|---|---|---|---|---|---|---|---|---|---|---|---|---|---|---|---|---|---|---|---|---|---|---|---|---|---|---|---|---|---|---|---|
| 3442 | 04051 | 03441 | 3 | | 0 | 0 | 0 | 0 | 0 | 0 | 0 | 0 | 0 | 0 | 1 | 0 | 0 | 0 | 1 | 1 | 0 | 0 | 0 | 0 | 0 | 0 | 0 | 0 | 0 | | | | | | |
| 3443 | 04052 | 03442 | 1 | | 0 | 0 | 0 | 0 | 0 | 0 | 0 | 0 | 0 | 0 | 1 | 0 | 0 | 0 | 0 | 0 | 0 | 0 | 0 | 0 | 0 | 0 | 0 | 0 | 0 | | | | | | |
| 3444 | 04053 | 03443 | 2 | | 0 | 0 | 0 | 0 | 0 | 0 | 0 | 0 | 0 | 0 | 1 | 0 | 0 | 0 | 0 | 0 | 0 | 0 | 0 | 0 | 0 | 1 | 0 | 0 | 0 | | | | | | |
| 3445 | 04054 | 03444 | 9 | | 0 | 0 | 0 | 0 | 0 | 0 | 0 | 0 | 0 | 0 | 1 | 0 | 1 | 4 | 0 | 0 | 0 | 0 | 3 | 0 | 0 | 0 | 0 | 0 | 0 | | | | | | |
| 3446 | 04055 | 03445 | 1 | | 0 | 0 | 0 | 0 | 0 | 0 | 0 | 0 | 0 | 0 | 1 | 0 | 0 | 0 | 0 | 0 | 0 | 0 | 0 | 0 | 0 | 0 | 0 | 0 | 0 | | | | | | |
| 3447 | 04056 | 03446 | 1 | | 0 | 0 | 0 | 0 | 0 | 0 | 0 | 0 | 0 | 0 | 1 | 0 | 0 | 0 | 0 | 0 | 0 | 0 | 0 | 0 | 0 | 0 | 0 | 0 | 0 | | | | | | |
| 3448 | 04057 | 03447 | 1 | | 0 | 0 | 0 | 0 | 0 | 0 | 0 | 0 | 0 | 0 | 1 | 0 | 0 | 0 | 0 | 0 | 0 | 0 | 0 | 0 | 0 | 0 | 0 | 0 | 0 | M10051 | 100 | 154 | 1 | 2017 | 4723 |
| 3449 | 04058 | 03448 | 1 | | 0 | 0 | 0 | 0 | 0 | 0 | 0 | 0 | 0 | 0 | 1 | 0 | 0 | 0 | 0 | 0 | 0 | 0 | 0 | 0 | 0 | 0 | 0 | 0 | 0 | | | | | | |
| 3450 | 04059 | 03449 | 1 | | 0 | 0 | 0 | 0 | 0 | 0 | 0 | 0 | 0 | 0 | 1 | 0 | 0 | 0 | 0 | 0 | 0 | 0 | 0 | 0 | 0 | 0 | 0 | 0 | 0 | | | | | | |
| 3451 | 04060 | 03450 | 4 | | 0 | 0 | 0 | 0 | 0 | 0 | 0 | 0 | 0 | 0 | 1 | 1 | 0 | 1 | 0 | 0 | 0 | 0 | 1 | 0 | 0 | 0 | 0 | 0 | 0 | | | | | | |
| 3452 | 04061 | 03451 | 1 | | 0 | 0 | 0 | 0 | 0 | 0 | 0 | 0 | 0 | 0 | 1 | 0 | 0 | 0 | 0 | 0 | 0 | 0 | 0 | 0 | 0 | 0 | 0 | 0 | 0 | M88458 | 94.2 | 325 | 1 | 536 | 1180 |
| 3453 | 04062 | 03452 | 2 | | 0 | 0 | 0 | 0 | 0 | 0 | 0 | 0 | 0 | 0 | 1 | 0 | 0 | 0 | 0 | 0 | 1 | 0 | 0 | 0 | 0 | 0 | 0 | 0 | 0 | | | | | | |
| 3454 | 04063 | 03453 | 4 | | 0 | 0 | 0 | 0 | 0 | 0 | 0 | 0 | 0 | 0 | 1 | 2 | 0 | 0 | 0 | 0 | 0 | 0 | 0 | 1 | 0 | 0 | 0 | 0 | 0 | | | | | | |
| 3455 | 04064 | 03454 | 1 | | 0 | 0 | 0 | 0 | 0 | 0 | 0 | 0 | 0 | 0 | 1 | 0 | 0 | 0 | 0 | 0 | 0 | 0 | 0 | 0 | 0 | 0 | 0 | 0 | 0 | | | | | | |
| 3456 | 04065 | 03455 | 1 | | 0 | 0 | 0 | 0 | 0 | 0 | 0 | 0 | 0 | 0 | 1 | 0 | 0 | 0 | 0 | 0 | 0 | 0 | 0 | 0 | 0 | 0 | 0 | 0 | 0 | | | | | | |
| 3457 | 04066 | 03456 | 1 | | 0 | 0 | 0 | 0 | 0 | 0 | 0 | 0 | 0 | 0 | 1 | 0 | 0 | 0 | 0 | 0 | 0 | 0 | 0 | 0 | 0 | 0 | 0 | 0 | 0 | | | | | | |
| 3458 | 04067 | 03457 | 2 | | 0 | 0 | 0 | 0 | 0 | 0 | 0 | 0 | 0 | 0 | 1 | 0 | 0 | 0 | 0 | 1 | 0 | 0 | 0 | 0 | 0 | 0 | 0 | 0 | 0 | | | | | | |
| 3459 | 04068 | 03458 | 1 | | 0 | 0 | 0 | 0 | 0 | 0 | 0 | 0 | 0 | 0 | 1 | 0 | 0 | 0 | 0 | 0 | 0 | 0 | 0 | 0 | 0 | 0 | 0 | 0 | 0 | | | | | | |
| 3460 | 04069 | 03459 | 1 | | 0 | 0 | 0 | 0 | 0 | 0 | 0 | 0 | 0 | 0 | 1 | 0 | 0 | 0 | 0 | 0 | 0 | 0 | 0 | 0 | 0 | 0 | 0 | 0 | 0 | | | | | | |
| 3461 | 04070 | 03460 | 3 | | 0 | 0 | 0 | 0 | 0 | 0 | 0 | 0 | 0 | 0 | 3 | 0 | 0 | 0 | 0 | 0 | 0 | 0 | 0 | 0 | 0 | 0 | 0 | 0 | 0 | M15042 | 94.3 | 317 | 1 | 2560 | 2929 |
| 3462 | 04071 | 03461 | 1 | | 0 | 0 | 0 | 0 | 0 | 0 | 0 | 0 | 0 | 0 | 1 | 0 | 0 | 0 | 0 | 0 | 0 | 0 | 0 | 0 | 0 | 0 | 0 | 0 | 0 | | | | | | |
| 3463 | 04072 | 03462 | 1 | | 0 | 0 | 0 | 0 | 0 | 0 | 0 | 0 | 0 | 0 | 1 | 0 | 0 | 0 | 0 | 0 | 0 | 0 | 0 | 0 | 0 | 0 | 0 | 0 | 0 | | | | | | |
| 3464 | 04073 | 03463 | 3 | | 0 | 0 | 0 | 0 | 0 | 0 | 0 | 0 | 0 | 0 | 1 | 0 | 0 | 0 | 0 | 0 | 0 | 1 | 0 | 0 | 0 | 0 | 0 | 1 | 0 | | | | | | |
| 3465 | 04074 | 03464 | 5 | | 0 | 0 | 0 | 0 | 0 | 0 | 0 | 0 | 0 | 0 | 1 | 0 | 0 | 0 | 0 | 0 | 0 | 0 | 0 | 0 | 0 | 1 | 3 | 0 | 0 | M62831 | 99.4 | 345 | 1 | 1467 | 1811 |
| 3466 | 04075 | 03465 | 1 | | 0 | 0 | 0 | 0 | 0 | 0 | 0 | 0 | 0 | 0 | 1 | 0 | 0 | 0 | 0 | 0 | 0 | 0 | 0 | 0 | 0 | 0 | 0 | 0 | 0 | | | | | | |
| 3467 | 04076 | 03466 | 2 | | 0 | 0 | 0 | 0 | 0 | 0 | 0 | 0 | 0 | 0 | 1 | 1 | 0 | 0 | 0 | 0 | 0 | 0 | 0 | 0 | 0 | 0 | 0 | 0 | 0 | X53143 | 98.2 | 273 | 1 | 379 | 651 |
| 3468 | 04077 | 03467 | 1 | | 0 | 0 | 0 | 0 | 0 | 0 | 0 | 0 | 0 | 0 | 1 | 0 | 0 | 0 | 0 | 0 | 0 | 0 | 0 | 0 | 0 | 0 | 0 | 0 | 0 | | | | | | |
| 3469 | 04078 | 03468 | 1 | | 0 | 0 | 0 | 0 | 0 | 0 | 0 | 0 | 0 | 0 | 1 | 0 | 0 | 0 | 0 | 0 | 0 | 0 | 0 | 0 | 0 | 0 | 0 | 0 | 0 | | | | | | |
| 3470 | 04079 | 03469 | 1 | | 0 | 0 | 0 | 0 | 0 | 0 | 0 | 0 | 0 | 0 | 1 | 0 | 0 | 0 | 0 | 0 | 0 | 0 | 0 | 0 | 0 | 0 | 0 | 0 | 0 | | | | | | |
| 3471 | 04080 | 03470 | 10 | | 0 | 0 | 0 | 0 | 0 | 0 | 0 | 0 | 0 | 0 | 1 | 0 | 0 | 1 | 2 | 0 | 1 | 2 | 0 | 0 | 0 | 0 | 2 | 1 | 0 | | | | | | |
| 3472 | 04081 | 03471 | 2 | | 0 | 0 | 0 | 0 | 0 | 0 | 0 | 0 | 0 | 0 | 2 | 0 | 0 | 0 | 0 | 0 | 0 | 0 | 0 | 0 | 0 | 0 | 0 | 0 | 0 | | | | | | |
| 3473 | 04082 | 03472 | 1 | | 0 | 0 | 0 | 0 | 0 | 0 | 0 | 0 | 0 | 0 | 1 | 0 | 0 | 0 | 0 | 0 | 0 | 0 | 0 | 0 | 0 | 0 | 0 | 0 | 0 | | | | | | |
| 3474 | 04083 | 03473 | 1 | | 0 | 0 | 0 | 0 | 0 | 0 | 0 | 0 | 0 | 0 | 1 | 0 | 0 | 0 | 0 | 0 | 0 | 0 | 0 | 0 | 0 | 0 | 0 | 0 | 0 | | | | | | |
| 3475 | 04084 | 03474 | 1 | | 0 | 0 | 0 | 0 | 0 | 0 | 0 | 0 | 0 | 0 | 1 | 0 | 0 | 0 | 0 | 0 | 0 | 0 | 0 | 0 | 0 | 0 | 0 | 0 | 0 | | | | | | |
| 3476 | 04086 | 03475 | 1 | | 0 | 0 | 0 | 0 | 0 | 0 | 0 | 0 | 0 | 0 | 1 | 0 | 0 | 0 | 0 | 0 | 0 | 0 | 0 | 0 | 0 | 0 | 0 | 0 | 0 | | | | | | |
| 3477 | 04087 | 03476 | 1 | | 0 | 0 | 0 | 0 | 0 | 0 | 0 | 0 | 0 | 0 | 1 | 0 | 0 | 0 | 0 | 0 | 0 | 0 | 0 | 0 | 0 | 0 | 0 | 0 | 0 | | | | | | |

Table 97

| | A | B | C | E | G | I | K | M | O | Q | S | U | W | Y | AA | AC | AE | AG | AI | AK | AM | AO | AQ | AS | AU | AW | AY | BA | BC | BE | BF | BG | BH | BI | BK |
|---|---|---|---|---|---|---|---|---|---|---|---|---|---|---|---|---|---|---|---|---|---|---|---|---|---|---|---|---|---|---|---|---|---|---|---|
| 3478 | 04088 | 03477 | 1 | | 0 | 0 | 0 | 0 | 0 | 0 | 0 | 0 | 0 | 0 | 1 | 0 | 0 | 0 | 0 | 0 | 0 | 0 | 0 | 0 | 0 | 0 | 0 | 0 | 0 | X74979 | 95.6 | 271 | 1 | 2937 | 3554 |
| 3479 | 04089 | 03478 | 2 | | 0 | 0 | 0 | 0 | 0 | 0 | 0 | 0 | 0 | 0 | 1 | 0 | 0 | 1 | 0 | 0 | 0 | 0 | 0 | 0 | 0 | 0 | 0 | 0 | 0 | X04297 | 96.7 | 270 | 1 | 3174 | 4108 |
| 3480 | 04090 | 03479 | 2 | | 0 | 0 | 0 | 0 | 0 | 0 | 0 | 0 | 0 | 0 | 1 | 0 | 0 | 0 | 1 | 0 | 0 | 0 | 0 | 0 | 0 | 0 | 0 | 0 | 0 | M58459 | 99.6 | 264 | 1 | 594 | 870 |
| 3481 | 04091 | 03480 | 2 | | 0 | 0 | 0 | 0 | 0 | 0 | 0 | 0 | 0 | 0 | 1 | 1 | 0 | 0 | 0 | 0 | 0 | 0 | 0 | 0 | 0 | 0 | 0 | 0 | 0 | L15203 | 99.2 | 260 | 1 | 221 | 480 |
| 3482 | 04092 | 03481 | 2 | | 0 | 0 | 0 | 0 | 0 | 0 | 0 | 0 | 0 | 0 | 2 | 0 | 0 | 0 | 0 | 0 | 0 | 0 | 0 | 0 | 0 | 0 | 0 | 0 | 0 | M35252 | 98.8 | 250 | 1 | 836 | 1104 |
| 3483 | 04093 | 03482 | 2 | | 0 | 0 | 0 | 0 | 0 | 0 | 0 | 0 | 0 | 0 | 1 | 1 | 0 | 0 | 0 | 0 | 0 | 0 | 0 | 0 | 0 | 0 | 0 | 0 | 0 | | | | | | |
| 3484 | 04094 | 03483 | 13 | | 0 | 0 | 0 | 0 | 0 | 0 | 0 | 0 | 0 | 0 | 13 | 0 | 0 | 0 | 0 | 0 | 0 | 0 | 0 | 0 | 0 | 0 | 0 | 0 | 0 | | | | | | |
| 3485 | 04095 | 03484 | 2 | | 0 | 0 | 0 | 0 | 0 | 0 | 0 | 0 | 0 | 0 | 1 | 0 | 0 | 0 | 0 | 0 | 0 | 0 | 0 | 0 | 0 | 0 | 0 | 1 | 0 | | | | | | |
| 3486 | 04096 | 03485 | 2 | | 0 | 0 | 0 | 0 | 0 | 0 | 0 | 0 | 0 | 0 | 1 | 0 | 0 | 0 | 0 | 0 | 0 | 0 | 0 | 0 | 0 | 0 | 0 | 1 | 0 | | | | | | |
| 3487 | 04097 | 03486 | 1 | | 0 | 0 | 0 | 0 | 0 | 0 | 0 | 0 | 0 | 0 | 1 | 0 | 0 | 0 | 0 | 0 | 0 | 0 | 0 | 0 | 0 | 0 | 0 | 0 | 0 | | | | | | |
| 3488 | 04098 | 03487 | 1 | | 0 | 0 | 0 | 0 | 0 | 0 | 0 | 0 | 0 | 0 | 1 | 0 | 0 | 0 | 0 | 0 | 0 | 0 | 0 | 0 | 0 | 0 | 0 | 0 | 0 | M65131 | 94.7 | 246 | 1 | 2377 | 2798 |
| 3489 | 04099 | 03488 | 2 | | 0 | 0 | 0 | 0 | 0 | 0 | 0 | 0 | 0 | 0 | 1 | 0 | 0 | 0 | 0 | 1 | 0 | 0 | 0 | 0 | 0 | 0 | 0 | 0 | 0 | | | | | | |
| 3490 | 04100 | 03489 | 2 | | 0 | 0 | 0 | 0 | 0 | 0 | 0 | 0 | 0 | 0 | 1 | 0 | 0 | 0 | 0 | 0 | 0 | 0 | 0 | 0 | 0 | 0 | 1 | 0 | 0 | D14658 | 97.5 | 244 | 1 | 452 | 1370 |
| 3491 | 04101 | 03490 | 1 | | 0 | 0 | 0 | 0 | 0 | 0 | 0 | 0 | 0 | 0 | 1 | 0 | 0 | 0 | 0 | 0 | 0 | 0 | 0 | 0 | 0 | 0 | 0 | 0 | 0 | X03363 | 99.2 | 236 | 1 | 4238 | 4473 |
| 3492 | 04102 | 03491 | 1 | | 0 | 0 | 0 | 0 | 0 | 0 | 0 | 0 | 0 | 0 | 1 | 0 | 0 | 0 | 0 | 0 | 0 | 0 | 0 | 0 | 0 | 0 | 0 | 0 | 0 | | | | | | |
| 3493 | 04103 | 03492 | 4 | | 0 | 0 | 0 | 0 | 0 | 0 | 0 | 0 | 0 | 0 | 1 | 0 | 0 | 0 | 0 | 0 | 0 | 0 | 0 | 0 | 0 | 0 | 3 | 0 | 0 | M24048 | 99.1 | 235 | 1 | 1113 | 1347 |
| 3494 | 04104 | 03493 | 1 | | 0 | 0 | 0 | 0 | 0 | 0 | 0 | 0 | 0 | 0 | 1 | 0 | 0 | 0 | 0 | 0 | 0 | 0 | 0 | 0 | 0 | 0 | 0 | 0 | 0 | | | | | | |
| 3495 | 04105 | 03494 | 1 | | 0 | 0 | 0 | 0 | 0 | 0 | 0 | 0 | 0 | 0 | 1 | 0 | 0 | 0 | 0 | 0 | 0 | 0 | 0 | 0 | 0 | 0 | 0 | 0 | 0 | X53578 | 97.4 | 233 | 1 | 1795 | 2043 |
| 3496 | 04106 | 03495 | 2 | | 0 | 0 | 0 | 0 | 0 | 0 | 0 | 0 | 0 | 0 | 1 | 0 | 0 | 0 | 0 | 0 | 0 | 0 | 1 | 0 | 0 | 0 | 0 | 0 | 0 | | | | | | |
| 3497 | 04107 | 03496 | 2 | | 0 | 0 | 0 | 0 | 0 | 0 | 0 | 0 | 0 | 0 | 1 | 0 | 0 | 0 | 0 | 0 | 0 | 0 | 0 | 0 | 1 | 0 | 0 | 0 | 0 | J04501 | 98.2 | 223 | 1 | 3309 | 3531 |
| 3498 | 04108 | 03497 | 1 | | 0 | 0 | 0 | 0 | 0 | 0 | 0 | 0 | 0 | 0 | 1 | 0 | 0 | 0 | 0 | 0 | 0 | 0 | 0 | 0 | 0 | 0 | 0 | 0 | 0 | | | | | | |
| 3499 | 04109 | 03498 | 1 | | 0 | 0 | 0 | 0 | 0 | 0 | 0 | 0 | 0 | 0 | 1 | 0 | 0 | 0 | 0 | 0 | 0 | 0 | 0 | 0 | 0 | 0 | 0 | 0 | 0 | | | | | | |
| 3500 | 04110 | 03499 | 2 | | 0 | 0 | 0 | 0 | 0 | 0 | 0 | 0 | 0 | 0 | 1 | 0 | 0 | 0 | 1 | 0 | 0 | 0 | 0 | 0 | 0 | 0 | 0 | 0 | 0 | | | | | | |
| 3501 | 04111 | 03500 | 1 | | 0 | 0 | 0 | 0 | 0 | 0 | 0 | 0 | 0 | 0 | 1 | 0 | 0 | 0 | 0 | 0 | 0 | 0 | 0 | 0 | 0 | 0 | 0 | 0 | 0 | M37197 | 93.2 | 219 | 1 | 2983 | 3216 |
| 3502 | 04112 | 03501 | 4 | | 0 | 0 | 0 | 0 | 0 | 0 | 0 | 0 | 0 | 0 | 1 | 0 | 0 | 0 | 1 | 2 | 0 | 0 | 0 | 0 | 0 | 0 | 0 | 0 | 0 | | | | | | |
| 3503 | 04113 | 03502 | 4 | | 0 | 0 | 0 | 0 | 0 | 0 | 0 | 0 | 0 | 0 | 2 | 0 | 0 | 0 | 0 | 0 | 0 | 0 | 0 | 0 | 0 | 2 | 0 | 0 | 0 | | | | | | |
| 3504 | 04114 | 03503 | 4 | | 0 | 0 | 0 | 0 | 0 | 0 | 0 | 0 | 0 | 0 | 1 | 0 | 0 | 0 | 1 | 0 | 0 | 0 | 0 | 0 | 0 | 0 | 0 | 2 | 0 | | | | | | |
| 3505 | 04115 | 03504 | 1 | | 0 | 0 | 0 | 0 | 0 | 0 | 0 | 0 | 0 | 0 | 1 | 0 | 0 | 0 | 0 | 0 | 0 | 0 | 0 | 0 | 0 | 0 | 0 | 0 | 0 | | | | | | |
| 3506 | 04116 | 03505 | 12 | | 0 | 0 | 0 | 0 | 0 | 0 | 0 | 0 | 0 | 0 | 10 | 0 | 0 | 0 | 0 | 2 | 0 | 0 | 0 | 0 | 0 | 0 | 0 | 0 | 0 | M93036 | 97.2 | 212 | 1 | 413 | 672 |
| 3507 | 04117 | 03506 | 1 | | 0 | 0 | 0 | 0 | 0 | 0 | 0 | 0 | 0 | 0 | 1 | 0 | 0 | 0 | 0 | 0 | 0 | 0 | 0 | 0 | 0 | 0 | 0 | 0 | 0 | | | | | | |
| 3508 | 04118 | 03507 | 1 | | 0 | 0 | 0 | 0 | 0 | 0 | 0 | 0 | 0 | 0 | 1 | 0 | 0 | 0 | 0 | 0 | 0 | 0 | 0 | 0 | 0 | 0 | 0 | 0 | 0 | | | | | | |
| 3509 | 04119 | 03508 | 1 | | 0 | 0 | 0 | 0 | 0 | 0 | 0 | 0 | 0 | 0 | 1 | 0 | 0 | 0 | 0 | 0 | 0 | 0 | 0 | 0 | 0 | 0 | 0 | 0 | 0 | | | | | | |
| 3510 | 04120 | 03509 | 2 | | 0 | 0 | 0 | 0 | 0 | 0 | 0 | 0 | 0 | 0 | 2 | 0 | 0 | 0 | 0 | 0 | 0 | 0 | 0 | 0 | 0 | 0 | 0 | 0 | 0 | | | | | | |
| 3511 | 04121 | 03510 | 1 | | 0 | 0 | 0 | 0 | 0 | 0 | 0 | 0 | 0 | 0 | 1 | 0 | 0 | 0 | 0 | 0 | 0 | 0 | 0 | 0 | 0 | 0 | 0 | 0 | 0 | | | | | | |
| 3512 | 04122 | 03511 | 1 | | 0 | 0 | 0 | 0 | 0 | 0 | 0 | 0 | 0 | 0 | 1 | 0 | 0 | 0 | 0 | 0 | 0 | 0 | 0 | 0 | 0 | 0 | 0 | 0 | 0 | | | | | | |
| 3513 | 04123 | 03512 | 1 | | 0 | 0 | 0 | 0 | 0 | 0 | 0 | 0 | 0 | 0 | 1 | 0 | 0 | 0 | 0 | 0 | 0 | 0 | 0 | 0 | 0 | 0 | 0 | 0 | 0 | | | | | | |

Table 98

| | A | B | C | E | G | I | K | M | O | Q | S | U | W | Y | AA | AC | AE | AG | AI | AK | AM | AO | AQ | AS | AU | AW | AY | BA | BC | BE | BF | BG | BH | BI | BK |
|---|---|---|---|---|---|---|---|---|---|---|---|---|---|---|---|---|---|---|---|---|---|---|---|---|---|---|---|---|---|---|---|---|---|---|---|
| 3514 | 04125 | 03513 | 1 | | 0 | 0 | 0 | 0 | 0 | 0 | 0 | 0 | 0 | 0 | 1 | 0 | 0 | 0 | 0 | 0 | 0 | 0 | 0 | 0 | 0 | 0 | 0 | 0 | 0 | | | | | | |
| 3515 | 04126 | 03514 | 2 | | 0 | 0 | 0 | 0 | 0 | 0 | 0 | 0 | 0 | 0 | 1 | 0 | 0 | 0 | 0 | 0 | 0 | 0 | 0 | 1 | 0 | 0 | 0 | 0 | 0 | | | | | | |
| 3516 | 04127 | 03515 | 4 | | 0 | 0 | 0 | 0 | 0 | 0 | 0 | 0 | 0 | 0 | 1 | 0 | 1 | 0 | 1 | 0 | 0 | 0 | 0 | 0 | 0 | 0 | 0 | 1 | 0 | | | | | | |
| 3517 | 04128 | 03516 | 1 | | 0 | 0 | 0 | 0 | 0 | 0 | 0 | 0 | 0 | 0 | 1 | 0 | 0 | 0 | 0 | 0 | 0 | 0 | 0 | 0 | 0 | 0 | 0 | 0 | 0 | | | | | | |
| 3518 | 04129 | 03517 | 4 | | 0 | 0 | 0 | 0 | 0 | 0 | 0 | 0 | 0 | 0 | 1 | 0 | 0 | 0 | 0 | 0 | 0 | 0 | 0 | 3 | 0 | 0 | 0 | 0 | 0 | | | | | | |
| 3519 | 04130 | 03518 | 1 | | 0 | 0 | 0 | 0 | 0 | 0 | 0 | 0 | 0 | 0 | 1 | 0 | 0 | 0 | 0 | 0 | 0 | 0 | 0 | 0 | 0 | 0 | 0 | 0 | 0 | D14705 | 97.4 | 190 | 1 | 2545 | 3433 |
| 3520 | 04131 | 03519 | 2 | | 0 | 0 | 0 | 0 | 0 | 0 | 0 | 0 | 0 | 0 | 2 | 0 | 0 | 0 | 0 | 0 | 0 | 0 | 0 | 0 | 0 | 0 | 0 | 0 | 0 | M75099 | 97.3 | 186 | 1 | 377 | 562 |
| 3521 | 04132 | 03520 | 1 | | 0 | 0 | 0 | 0 | 0 | 0 | 0 | 0 | 0 | 0 | 1 | 0 | 0 | 0 | 0 | 0 | 0 | 0 | 0 | 0 | 0 | 0 | 0 | 0 | 0 | | | | | | |
| 3522 | 04133 | 03521 | 7 | | 0 | 0 | 0 | 0 | 0 | 0 | 0 | 0 | 0 | 0 | 1 | 0 | 1 | 0 | 0 | 0 | 2 | 0 | 1 | 0 | 0 | 0 | 1 | 1 | 0 | | | | | | |
| 3523 | 04134 | 03522 | 3 | | 0 | 0 | 0 | 0 | 0 | 0 | 0 | 0 | 0 | 0 | 1 | 0 | 0 | 0 | 0 | 0 | 0 | 0 | 1 | 0 | 0 | 0 | 1 | 0 | 0 | M75099 | 95.9 | 219 | 1 | 345 | 562 |
| 3524 | 04135 | 03523 | 1 | | 0 | 0 | 0 | 0 | 0 | 0 | 0 | 0 | 0 | 0 | 1 | 0 | 0 | 0 | 0 | 0 | 0 | 0 | 0 | 0 | 0 | 0 | 0 | 0 | 0 | | | | | | |
| 3525 | 04136 | 03524 | 1 | | 0 | 0 | 0 | 0 | 0 | 0 | 0 | 0 | 0 | 0 | 1 | 0 | 0 | 0 | 0 | 0 | 0 | 0 | 0 | 0 | 0 | 0 | 0 | 0 | 0 | M34079 | 96.4 | 168 | 1 | 1172 | 1341 |
| 3526 | 04137 | 03525 | 2 | | 0 | 0 | 0 | 0 | 0 | 0 | 0 | 0 | 0 | 0 | 1 | 0 | 0 | 0 | 0 | 0 | 0 | 0 | 0 | 0 | 0 | 0 | 0 | 1 | 0 | | | | | | |
| 3527 | 04139 | 03526 | 2 | | 0 | 0 | 0 | 0 | 0 | 0 | 0 | 0 | 0 | 0 | 2 | 0 | 0 | 0 | 0 | 0 | 0 | 0 | 0 | 0 | 0 | 0 | 0 | 0 | 0 | | | | | | |
| 3528 | 04140 | 03527 | 1 | | 0 | 0 | 0 | 0 | 0 | 0 | 0 | 0 | 0 | 0 | 1 | 0 | 0 | 0 | 0 | 0 | 0 | 0 | 0 | 0 | 0 | 0 | 0 | 0 | 0 | X52897 | 93.3 | 164 | 1 | 2329 | 2670 |
| 3529 | 04141 | 03528 | 1 | | 0 | 0 | 0 | 0 | 0 | 0 | 0 | 0 | 0 | 0 | 1 | 0 | 0 | 0 | 0 | 0 | 0 | 0 | 0 | 0 | 0 | 0 | 0 | 0 | 0 | | | | | | |
| 3530 | 04142 | 03529 | 1 | | 0 | 0 | 0 | 0 | 0 | 0 | 0 | 0 | 0 | 0 | 1 | 0 | 0 | 0 | 0 | 0 | 0 | 0 | 0 | 0 | 0 | 0 | 0 | 0 | 0 | X52125 | 100 | 156 | 1 | 3147 | 3302 |
| 3531 | 04143 | 03530 | 1 | | 0 | 0 | 0 | 0 | 0 | 0 | 0 | 0 | 0 | 0 | 1 | 0 | 0 | 0 | 0 | 0 | 0 | 0 | 0 | 0 | 0 | 0 | 0 | 0 | 0 | | | | | | |
| 3532 | 04144 | 03531 | 11 | | 0 | 0 | 0 | 0 | 0 | 0 | 0 | 0 | 0 | 0 | 2 | 0 | 0 | 0 | 1 | 0 | 0 | 5 | 1 | 0 | 0 | 0 | 0 | 2 | 0 | X69654 | 98.7 | 156 | 1 | 283 | 438 |
| 3533 | 04145 | 03532 | 2 | | 0 | 0 | 0 | 0 | 0 | 0 | 0 | 0 | 0 | 0 | 1 | 0 | 0 | 0 | 0 | 0 | 0 | 0 | 0 | 0 | 0 | 0 | 1 | 0 | 0 | | | | | | |
| 3534 | 04146 | 03533 | 2 | | 0 | 0 | 0 | 0 | 0 | 0 | 0 | 0 | 0 | 0 | 1 | 0 | 0 | 0 | 0 | 0 | 1 | 0 | 0 | 0 | 0 | 0 | 0 | 0 | 0 | | | | | | |
| 3535 | 04147 | 03534 | 6 | | 0 | 0 | 0 | 0 | 0 | 0 | 0 | 0 | 0 | 0 | 1 | 0 | 0 | 0 | 0 | 0 | 0 | 0 | 0 | 0 | 0 | 5 | 0 | 0 | 0 | | | | | | |
| 3536 | 04148 | 03535 | 2 | | 0 | 0 | 0 | 0 | 0 | 0 | 0 | 0 | 0 | 0 | 1 | 0 | 0 | 0 | 0 | 0 | 0 | 0 | 0 | 0 | 0 | 0 | 0 | 1 | 0 | | | | | | |
| 3537 | 04149 | 03536 | 2 | | 0 | 0 | 0 | 0 | 0 | 0 | 0 | 0 | 0 | 0 | 2 | 0 | 0 | 0 | 0 | 0 | 0 | 0 | 0 | 0 | 0 | 0 | 0 | 0 | 0 | | | | | | |
| 3538 | 04150 | 03537 | 3 | | 0 | 0 | 0 | 0 | 0 | 0 | 0 | 0 | 0 | 0 | 1 | 0 | 0 | 0 | 0 | 0 | 0 | 0 | 0 | 0 | 0 | 0 | 0 | 2 | 0 | | | | | | |
| 3539 | 04151 | 03538 | 3 | | 0 | 0 | 0 | 0 | 0 | 0 | 0 | 0 | 0 | 0 | 1 | 0 | 0 | 0 | 0 | 0 | 0 | 0 | 0 | 0 | 0 | 0 | 2 | 0 | 0 | | | | | | |
| 3540 | 04152 | 03539 | 4 | | 0 | 0 | 0 | 0 | 0 | 0 | 0 | 0 | 0 | 0 | 1 | 0 | 0 | 1 | 0 | 0 | 0 | 0 | 1 | 0 | 0 | 1 | 0 | 0 | 0 | M73547 | 95.9 | 148 | 1 | 2918 | 3178 |
| 3541 | 04153 | 03540 | 2 | | 0 | 0 | 0 | 0 | 0 | 0 | 0 | 0 | 0 | 0 | 1 | 0 | 0 | 1 | 0 | 0 | 0 | 0 | 0 | 0 | 0 | 0 | 0 | 0 | 0 | | | | | | |
| 3542 | 04154 | 03541 | 1 | | 0 | 0 | 0 | 0 | 0 | 0 | 0 | 0 | 0 | 0 | 1 | 0 | 0 | 0 | 0 | 0 | 0 | 0 | 0 | 0 | 0 | 0 | 0 | 0 | 0 | | | | | | |
| 3543 | 04155 | 03542 | 1 | | 0 | 0 | 0 | 0 | 0 | 0 | 0 | 0 | 0 | 0 | 1 | 0 | 0 | 0 | 0 | 0 | 0 | 0 | 0 | 0 | 0 | 0 | 0 | 0 | 0 | | | | | | |
| 3544 | 04156 | 03543 | 1 | | 0 | 0 | 0 | 0 | 0 | 0 | 0 | 0 | 0 | 0 | 1 | 0 | 0 | 0 | 0 | 0 | 0 | 0 | 0 | 0 | 0 | 0 | 0 | 0 | 0 | | | | | | |
| 3545 | 04157 | 03544 | 1 | | 0 | 0 | 0 | 0 | 0 | 0 | 0 | 0 | 0 | 0 | 1 | 0 | 0 | 0 | 0 | 0 | 0 | 0 | 0 | 0 | 0 | 0 | 0 | 0 | 0 | | | | | | |
| 3546 | 04158 | 03545 | 1 | | 0 | 0 | 0 | 0 | 0 | 0 | 0 | 0 | 0 | 0 | 1 | 0 | 0 | 0 | 0 | 0 | 0 | 0 | 0 | 0 | 0 | 0 | 0 | 0 | 0 | | | | | | |
| 3547 | 04159 | 03546 | 2 | | 0 | 0 | 0 | 0 | 0 | 0 | 0 | 0 | 0 | 0 | 1 | 0 | 0 | 0 | 0 | 1 | 0 | 0 | 0 | 0 | 0 | 0 | 0 | 0 | 0 | | | | | | |
| 3548 | 04160 | 03547 | 1 | | 0 | 0 | 0 | 0 | 0 | 0 | 0 | 0 | 0 | 0 | 1 | 0 | 0 | 0 | 0 | 0 | 0 | 0 | 0 | 0 | 0 | 0 | 0 | 0 | 0 | | | | | | |
| 3549 | 04161 | 03548 | 2 | | 0 | 0 | 0 | 0 | 0 | 0 | 0 | 0 | 0 | 0 | 1 | 0 | 0 | 0 | 0 | 0 | 0 | 0 | 0 | 0 | 0 | 0 | 0 | 0 | 1 | | | | | | |

Table 99

| | A | B | C | E | G | I | K | M | O | Q | S | U | W | Y | AA | AC | AE | AG | AI | AK | AM | AO | AQ | AS | AU | AW | AY | BA | BC | BE | BF | BG | BH | BI | BK |
|---|---|---|---|---|---|---|---|---|---|---|---|---|---|---|---|---|---|---|---|---|---|---|---|---|---|---|---|---|---|---|---|---|---|---|---|
| 3550 | 04162 | 03549 | 3 | | 0 | 0 | 0 | 0 | 0 | 0 | 0 | 0 | 0 | 0 | 2 | 0 | 0 | 0 | 0 | 0 | 0 | 0 | 1 | 0 | 0 | 0 | 0 | 0 | 0 | | | | | | |
| 3551 | 04163 | 03550 | 1 | | 0 | 0 | 0 | 0 | 0 | 0 | 0 | 0 | 0 | 0 | 1 | 0 | 0 | 0 | 0 | 0 | 0 | 0 | 0 | 0 | 0 | 0 | 0 | 0 | 0 | | | | | | |
| 3552 | 04164 | 03551 | 2 | | 0 | 0 | 0 | 0 | 0 | 0 | 0 | 0 | 0 | 0 | 2 | 0 | 0 | 0 | 0 | 0 | 0 | 0 | 0 | 0 | 0 | 0 | 0 | 0 | 0 | | | | | | |
| 3553 | 04165 | 03552 | 6 | | 0 | 0 | 0 | 0 | 0 | 0 | 0 | 0 | 0 | 0 | 1 | 0 | 1 | 0 | 1 | 1 | 1 | 0 | 0 | 0 | 1 | 0 | 0 | 0 | 0 | J03592 | 96.1 | 127 | 1 | 990 | 1116 |
| 3554 | 04166 | 03553 | 3 | | 0 | 0 | 0 | 0 | 0 | 0 | 0 | 0 | 0 | 0 | 2 | 0 | 0 | 0 | 1 | 0 | 0 | 0 | 0 | 0 | 0 | 0 | 0 | 0 | 0 | S65738 | 96.2 | 132 | 1 | 533 | 1452 |
| 3555 | 04167 | 03554 | 5 | | 0 | 0 | 0 | 0 | 0 | 0 | 0 | 0 | 0 | 0 | 3 | 1 | 0 | 0 | 0 | 0 | 0 | 0 | 0 | 0 | 1 | 0 | 0 | 0 | 0 | | | | | | |
| 3556 | 04169 | 03555 | 1 | | 0 | 0 | 0 | 0 | 0 | 0 | 0 | 0 | 0 | 0 | 1 | 0 | 0 | 0 | 0 | 0 | 0 | 0 | 0 | 0 | 0 | 0 | 0 | 0 | 0 | | | | | | |
| 3557 | 04170 | 03556 | 1 | | 0 | 0 | 0 | 0 | 0 | 0 | 0 | 0 | 0 | 0 | 1 | 0 | 0 | 0 | 0 | 0 | 0 | 0 | 0 | 0 | 0 | 0 | 0 | 0 | 0 | | | | | | |
| 3558 | 04171 | 03557 | 1 | | 0 | 0 | 0 | 0 | 0 | 0 | 0 | 0 | 0 | 0 | 1 | 0 | 0 | 0 | 0 | 0 | 0 | 0 | 0 | 0 | 0 | 0 | 0 | 0 | 0 | | | | | | |
| 3559 | 04172 | 03558 | 1 | | 0 | 0 | 0 | 0 | 0 | 0 | 0 | 0 | 0 | 0 | 1 | 0 | 0 | 0 | 0 | 0 | 0 | 0 | 0 | 0 | 0 | 0 | 0 | 0 | 0 | | | | | | |
| 3560 | 04173 | 03559 | 1 | | 0 | 0 | 0 | 0 | 0 | 0 | 0 | 0 | 0 | 0 | 1 | 0 | 0 | 0 | 0 | 0 | 0 | 0 | 0 | 0 | 0 | 0 | 0 | 0 | 0 | | | | | | |
| 3561 | 04174 | 03560 | 4 | | 0 | 0 | 0 | 0 | 0 | 0 | 0 | 0 | 0 | 0 | 1 | 0 | 0 | 1 | 0 | 0 | 2 | 0 | 0 | 0 | 0 | 0 | 0 | 0 | 0 | | | | | | |
| 3562 | 04175 | 03561 | 2 | | 0 | 0 | 0 | 0 | 0 | 0 | 0 | 0 | 0 | 0 | 2 | 0 | 0 | 0 | 0 | 0 | 0 | 0 | 0 | 0 | 0 | 0 | 0 | 0 | 0 | | | | | | |
| 3563 | 04176 | 03562 | 1 | | 0 | 0 | 0 | 0 | 0 | 0 | 0 | 0 | 0 | 0 | 1 | 0 | 0 | 0 | 0 | 0 | 0 | 0 | 0 | 0 | 0 | 0 | 0 | 0 | 0 | | | | | | |
| 3564 | 04177 | 03563 | 1 | | 0 | 0 | 0 | 0 | 0 | 0 | 0 | 0 | 0 | 0 | 1 | 0 | 0 | 0 | 0 | 0 | 0 | 0 | 0 | 0 | 0 | 0 | 0 | 0 | 0 | X69141 | 93.2 | 117 | 1 | 1659 | 2051 |
| 3565 | 04179 | 03564 | 2 | | 0 | 0 | 0 | 0 | 0 | 0 | 0 | 0 | 0 | 0 | 1 | 0 | 0 | 0 | 0 | 0 | 1 | 0 | 0 | 0 | 0 | 0 | 0 | 0 | 0 | | | | | | |
| 3566 | 04180 | 03565 | 3 | | 0 | 0 | 0 | 0 | 0 | 0 | 0 | 0 | 0 | 0 | 2 | 0 | 0 | 0 | 0 | 0 | 0 | 0 | 0 | 0 | 0 | 0 | 0 | 1 | 0 | | | | | | |
| 3567 | 04181 | 03566 | 1 | | 0 | 0 | 0 | 0 | 0 | 0 | 0 | 0 | 0 | 0 | 1 | 0 | 0 | 0 | 0 | 0 | 0 | 0 | 0 | 0 | 0 | 0 | 0 | 0 | 0 | | | | | | |
| 3568 | 04182 | 03567 | 1 | | 0 | 0 | 0 | 0 | 0 | 0 | 0 | 0 | 0 | 0 | 1 | 0 | 0 | 0 | 0 | 0 | 0 | 0 | 0 | 0 | 0 | 0 | 0 | 0 | 0 | | | | | | |
| 3569 | 04183 | 03568 | 1 | | 0 | 0 | 0 | 0 | 0 | 0 | 0 | 0 | 0 | 0 | 1 | 0 | 0 | 0 | 0 | 0 | 0 | 0 | 0 | 0 | 0 | 0 | 0 | 0 | 0 | | | | | | |
| 3570 | 04184 | 03569 | 1 | | 0 | 0 | 0 | 0 | 0 | 0 | 0 | 0 | 0 | 0 | 1 | 0 | 0 | 0 | 0 | 0 | 0 | 0 | 0 | 0 | 0 | 0 | 0 | 0 | 0 | | | | | | |
| 3571 | 04186 | 03570 | 2 | | 0 | 0 | 0 | 0 | 0 | 0 | 0 | 0 | 0 | 0 | 1 | 0 | 0 | 0 | 0 | 1 | 0 | 0 | 0 | 0 | 0 | 0 | 0 | 0 | 0 | | | | | | |
| 3572 | 04187 | 03571 | 1 | | 0 | 0 | 0 | 0 | 0 | 0 | 0 | 0 | 0 | 0 | 1 | 0 | 0 | 0 | 0 | 0 | 0 | 0 | 0 | 0 | 0 | 0 | 0 | 0 | 0 | | | | | | |
| 3573 | 04188 | 03572 | 1 | | 0 | 0 | 0 | 0 | 0 | 0 | 0 | 0 | 0 | 0 | 1 | 0 | 0 | 0 | 0 | 0 | 0 | 0 | 0 | 0 | 0 | 0 | 0 | 0 | 0 | | | | | | |
| 3574 | 04189 | 03573 | 3 | | 0 | 0 | 0 | 0 | 0 | 0 | 0 | 0 | 0 | 0 | 1 | 0 | 0 | 0 | 0 | 0 | 0 | 0 | 0 | 0 | 0 | 2 | 0 | 0 | 0 | | | | | | |
| 3575 | 04190 | 03574 | 10 | | 0 | 0 | 0 | 0 | 0 | 0 | 0 | 0 | 0 | 0 | 1 | 0 | 0 | 0 | 0 | 1 | 0 | 2 | 0 | 0 | 0 | 5 | 1 | 0 | 0 | | | | | | |
| 3576 | 04191 | 03575 | 3 | | 0 | 0 | 0 | 0 | 0 | 0 | 0 | 0 | 0 | 0 | 1 | 0 | 0 | 0 | 0 | 0 | 0 | 1 | 0 | 0 | 0 | 1 | 0 | 0 | 0 | J03058 | 100 | 81 | 1 | 1422 | 1502 |
| 3577 | 04192 | 03576 | 1 | | 0 | 0 | 0 | 0 | 0 | 0 | 0 | 0 | 0 | 0 | 1 | 0 | 0 | 0 | 0 | 0 | 0 | 0 | 0 | 0 | 0 | 0 | 0 | 0 | 0 | | | | | | |
| 3578 | 04193 | 03577 | 1 | | 0 | 0 | 0 | 0 | 0 | 0 | 0 | 0 | 0 | 0 | 1 | 0 | 0 | 0 | 0 | 0 | 0 | 0 | 0 | 0 | 0 | 0 | 0 | 0 | 0 | M96684 | 98.8 | 83 | 1 | 1027 | 1144 |
| 3579 | 04194 | 03578 | 1 | | 0 | 0 | 0 | 0 | 0 | 0 | 0 | 0 | 0 | 0 | 1 | 0 | 0 | 0 | 0 | 0 | 0 | 0 | 0 | 0 | 0 | 0 | 0 | 0 | 0 | | | | | | |
| 3580 | 04195 | 03579 | 3 | | 0 | 0 | 0 | 0 | 0 | 0 | 0 | 0 | 0 | 0 | 2 | 0 | 0 | 0 | 0 | 0 | 0 | 0 | 0 | 0 | 0 | 0 | 0 | 0 | 1 | | | | | | |
| 3581 | 04196 | 03580 | 2 | | 0 | 0 | 0 | 0 | 0 | 0 | 0 | 0 | 0 | 0 | 1 | 0 | 0 | 0 | 0 | 0 | 0 | 1 | 0 | 0 | 0 | 0 | 0 | 0 | 0 | | | | | | |
| 3582 | 04197 | 03581 | 2 | | 0 | 0 | 0 | 0 | 0 | 0 | 0 | 0 | 0 | 0 | 1 | 0 | 0 | 0 | 1 | 0 | 0 | 0 | 0 | 0 | 0 | 0 | 0 | 0 | 0 | | | | | | |
| 3583 | 04198 | 03582 | 3 | | 0 | 0 | 0 | 0 | 0 | 0 | 0 | 0 | 0 | 0 | 1 | 0 | 0 | 0 | 0 | 0 | 0 | 1 | 0 | 0 | 0 | 1 | 0 | 0 | 0 | | | | | | |
| 3584 | 04199 | 03583 | 2 | | 0 | 0 | 0 | 0 | 0 | 0 | 0 | 0 | 0 | 0 | 1 | 0 | 0 | 0 | 0 | 0 | 0 | 0 | 0 | 0 | 0 | 1 | 0 | 0 | 0 | | | | | | |
| 3585 | 04200 | 03584 | 1 | | 0 | 0 | 0 | 0 | 0 | 0 | 0 | 0 | 0 | 0 | 1 | 0 | 0 | 0 | 0 | 0 | 0 | 0 | 0 | 0 | 0 | 0 | 0 | 0 | 0 | | | | | | |

Table 100

| | A | B | C | E | G | I | K | M | O | Q | S | U | W | Y | AA | AC | AE | AG | AI | AK | AM | AO | AQ | AS | AU | AW | AY | BA | BC | BE | BF | BG | BH | BI | BK |
|---|---|---|---|---|---|---|---|---|---|---|---|---|---|---|---|---|---|---|---|---|---|---|---|---|---|---|---|---|---|---|---|---|---|---|---|
| 3586 | 04201 | 03585 | 2 | | 0 | 0 | 0 | 0 | 0 | 0 | 0 | 0 | 0 | 0 | 1 | 0 | 0 | 0 | 0 | 0 | 0 | 0 | 0 | 0 | 0 | 0 | 1 | 0 | 0 | | | | | | |
| 3587 | 04202 | 03586 | 1 | | 0 | 0 | 0 | 0 | 0 | 0 | 0 | 0 | 0 | 0 | 1 | 0 | 0 | 0 | 0 | 0 | 0 | 0 | 0 | 0 | 0 | 0 | 0 | 0 | 0 | | | | | | |
| 3588 | 04203 | 03587 | 2 | | 0 | 0 | 0 | 0 | 0 | 0 | 0 | 0 | 0 | 0 | 1 | 0 | 0 | 0 | 0 | 0 | 0 | 0 | 0 | 0 | 0 | 0 | 0 | 1 | 0 | | | | | | |
| 3589 | 04204 | 03588 | 2 | | 0 | 0 | 0 | 0 | 0 | 0 | 0 | 0 | 0 | 0 | 1 | 0 | 0 | 1 | 0 | 0 | 0 | 0 | 0 | 0 | 0 | 0 | 0 | 0 | 0 | D13900 | 92.8 | 152 | 1 | 612 | 1277 |
| 3590 | 04205 | 03589 | 2 | | 0 | 0 | 0 | 0 | 0 | 0 | 0 | 0 | 0 | 0 | 2 | 0 | 0 | 0 | 0 | 0 | 0 | 0 | 0 | 0 | 0 | 0 | 0 | 0 | 0 | | | | | | |
| 3591 | 04206 | 03590 | 1 | | 0 | 0 | 0 | 0 | 0 | 0 | 0 | 0 | 0 | 0 | 1 | 0 | 0 | 0 | 0 | 0 | 0 | 0 | 0 | 0 | 0 | 0 | 0 | 0 | 0 | | | | | | |
| 3592 | 04207 | 03591 | 1 | | 0 | 0 | 0 | 0 | 0 | 0 | 0 | 0 | 0 | 0 | 1 | 0 | 0 | 0 | 0 | 0 | 0 | 0 | 0 | 0 | 0 | 0 | 0 | 0 | 0 | | | | | | |
| 3593 | 04210 | 03592 | 1 | | 0 | 0 | 0 | 0 | 0 | 0 | 0 | 0 | 0 | 0 | 1 | 0 | 0 | 0 | 0 | 0 | 0 | 0 | 0 | 0 | 0 | 0 | 0 | 0 | 0 | | | | | | |
| 3594 | 04211 | 03593 | 1 | | 0 | 0 | 0 | 0 | 0 | 0 | 0 | 0 | 0 | 0 | 1 | 0 | 0 | 0 | 0 | 0 | 0 | 0 | 0 | 0 | 0 | 0 | 0 | 0 | 0 | | | | | | |
| 3595 | 04213 | 03594 | 2 | | 0 | 0 | 0 | 0 | 0 | 0 | 0 | 0 | 0 | 0 | 1 | 0 | 0 | 1 | 0 | 0 | 0 | 0 | 0 | 0 | 0 | 0 | 0 | 0 | 0 | | | | | | |
| 3596 | 04214 | 03595 | 3 | | 0 | 0 | 0 | 0 | 0 | 0 | 0 | 0 | 0 | 0 | 1 | 0 | 0 | 2 | 0 | 0 | 0 | 0 | 0 | 0 | 0 | 0 | 0 | 0 | 0 | | | | | | |
| 3597 | 04215 | 03596 | 1 | | 0 | 0 | 0 | 0 | 0 | 0 | 0 | 0 | 0 | 0 | 1 | 0 | 0 | 0 | 0 | 0 | 0 | 0 | 0 | 0 | 0 | 0 | 0 | 0 | 0 | | | | | | |
| 3598 | 04216 | 03597 | 1 | | 0 | 0 | 0 | 0 | 0 | 0 | 0 | 0 | 0 | 0 | 1 | 0 | 0 | 0 | 0 | 0 | 0 | 0 | 0 | 0 | 0 | 0 | 0 | 0 | 0 | | | | | | |
| 3599 | 04218 | 03598 | 1 | | 0 | 0 | 0 | 0 | 0 | 0 | 0 | 0 | 0 | 0 | 1 | 0 | 0 | 0 | 0 | 0 | 0 | 0 | 0 | 0 | 0 | 0 | 0 | 0 | 0 | | | | | | |
| 3600 | 04219 | 03599 | 1 | | 0 | 0 | 0 | 0 | 0 | 0 | 0 | 0 | 0 | 0 | 1 | 0 | 0 | 0 | 0 | 0 | 0 | 0 | 0 | 0 | 0 | 0 | 0 | 0 | 0 | | | | | | |
| 3601 | 04220 | 03600 | 1 | | 0 | 0 | 0 | 0 | 0 | 0 | 0 | 0 | 0 | 0 | 1 | 0 | 0 | 0 | 0 | 0 | 0 | 0 | 0 | 0 | 0 | 0 | 0 | 0 | 0 | | | | | | |
| 3602 | 04247 | 03601 | 3 | | 0 | 0 | 0 | 0 | 0 | 0 | 0 | 0 | 0 | 0 | 0 | 2 | 0 | 0 | 0 | 0 | 0 | 0 | 1 | 0 | 0 | 0 | 0 | 0 | 0 | M94630 | 95.7 | 328 | 1 | 767 | 1154 |
| 3603 | 04248 | 03602 | 1 | | 0 | 0 | 0 | 0 | 0 | 0 | 0 | 0 | 0 | 0 | 0 | 1 | 0 | 0 | 0 | 0 | 0 | 0 | 0 | 0 | 0 | 0 | 0 | 0 | 0 | | | | | | |
| 3604 | 04249 | 03603 | 1 | | 0 | 0 | 0 | 0 | 0 | 0 | 0 | 0 | 0 | 0 | 0 | 1 | 0 | 0 | 0 | 0 | 0 | 0 | 0 | 0 | 0 | 0 | 0 | 0 | 0 | | | | | | |
| 3605 | 04250 | 03604 | 1 | | 0 | 0 | 0 | 0 | 0 | 0 | 0 | 0 | 0 | 0 | 0 | 1 | 0 | 0 | 0 | 0 | 0 | 0 | 0 | 0 | 0 | 0 | 0 | 0 | 0 | | | | | | |
| 3606 | 04252 | 03605 | 1 | | 0 | 0 | 0 | 0 | 0 | 0 | 0 | 0 | 0 | 0 | 0 | 1 | 0 | 0 | 0 | 0 | 0 | 0 | 0 | 0 | 0 | 0 | 0 | 0 | 0 | | | | | | |
| 3607 | 04253 | 03606 | 1 | | 0 | 0 | 0 | 0 | 0 | 0 | 0 | 0 | 0 | 0 | 0 | 1 | 0 | 0 | 0 | 0 | 0 | 0 | 0 | 0 | 0 | 0 | 0 | 0 | 0 | | | | | | |
| 3608 | 04256 | 03607 | 1 | | 0 | 0 | 0 | 0 | 0 | 0 | 0 | 0 | 0 | 0 | 0 | 1 | 0 | 0 | 0 | 0 | 0 | 0 | 0 | 0 | 0 | 0 | 0 | 0 | 0 | | | | | | |
| 3609 | 04257 | 03608 | 2 | | 0 | 0 | 0 | 0 | 0 | 0 | 0 | 0 | 0 | 0 | 0 | 1 | 0 | 0 | 0 | 0 | 0 | 0 | 0 | 0 | 0 | 0 | 0 | 1 | 0 | | | | | | |
| 3610 | 04258 | 03609 | 1 | | 0 | 0 | 0 | 0 | 0 | 0 | 0 | 0 | 0 | 0 | 0 | 1 | 0 | 0 | 0 | 0 | 0 | 0 | 0 | 0 | 0 | 0 | 0 | 0 | 0 | | | | | | |
| 3611 | 04259 | 03610 | 2 | | 0 | 0 | 0 | 0 | 0 | 0 | 0 | 0 | 0 | 0 | 0 | 1 | 0 | 0 | 0 | 0 | 0 | 0 | 0 | 1 | 0 | 0 | 0 | 0 | 0 | | | | | | |
| 3612 | 04261 | 03611 | 1 | | 0 | 0 | 0 | 0 | 0 | 0 | 0 | 0 | 0 | 0 | 0 | 1 | 0 | 0 | 0 | 0 | 0 | 0 | 0 | 0 | 0 | 0 | 0 | 0 | 0 | | | | | | |
| 3613 | 04264 | 03612 | 1 | | 0 | 0 | 0 | 0 | 0 | 0 | 0 | 0 | 0 | 0 | 0 | 1 | 0 | 0 | 0 | 0 | 0 | 0 | 0 | 0 | 0 | 0 | 0 | 0 | 0 | | | | | | |
| 3614 | 04265 | 03613 | 1 | | 0 | 0 | 0 | 0 | 0 | 0 | 0 | 0 | 0 | 0 | 0 | 1 | 0 | 0 | 0 | 0 | 0 | 0 | 0 | 0 | 0 | 0 | 0 | 0 | 0 | | | | | | |
| 3615 | 04266 | 03614 | 2 | | 0 | 0 | 0 | 0 | 0 | 0 | 0 | 0 | 0 | 0 | 0 | 2 | 0 | 0 | 0 | 0 | 0 | 0 | 0 | 0 | 0 | 0 | 0 | 0 | 0 | | | | | | |
| 3616 | 04267 | 03615 | 2 | | 0 | 0 | 0 | 0 | 0 | 0 | 0 | 0 | 0 | 0 | 0 | 2 | 0 | 0 | 0 | 0 | 0 | 0 | 0 | 0 | 0 | 0 | 0 | 0 | 0 | | | | | | |
| 3617 | 04268 | 03616 | 1 | | 0 | 0 | 0 | 0 | 0 | 0 | 0 | 0 | 0 | 0 | 0 | 1 | 0 | 0 | 0 | 0 | 0 | 0 | 0 | 0 | 0 | 0 | 0 | 0 | 0 | | | | | | |
| 3618 | 04269 | 03617 | 3 | | 0 | 0 | 0 | 0 | 0 | 0 | 0 | 0 | 0 | 0 | 0 | 2 | 0 | 0 | 0 | 0 | 0 | 0 | 0 | 0 | 1 | 0 | 0 | 0 | 0 | | | | | | |
| 3619 | 04270 | 03618 | 1 | | 0 | 0 | 0 | 0 | 0 | 0 | 0 | 0 | 0 | 0 | 0 | 1 | 0 | 0 | 0 | 0 | 0 | 0 | 0 | 0 | 0 | 0 | 0 | 0 | 0 | | | | | | |
| 3620 | 04271 | 03619 | 1 | | 0 | 0 | 0 | 0 | 0 | 0 | 0 | 0 | 0 | 0 | 0 | 1 | 0 | 0 | 0 | 0 | 0 | 0 | 0 | 0 | 0 | 0 | 0 | 0 | 0 | | | | | | |
| 3621 | 04272 | 03620 | 1 | | 0 | 0 | 0 | 0 | 0 | 0 | 0 | 0 | 0 | 0 | 0 | 1 | 0 | 0 | 0 | 0 | 0 | 0 | 0 | 0 | 0 | 0 | 0 | 0 | 0 | | | | | | |

Table 101

| | A | B | C | E | G | I | K | M | O | Q | S | U | W | Y | AA | AC | AE | AG | AI | AK | AM | AO | AQ | AS | AU | AW | AY | BA | BC | BE | BF | BG | BH | BI | BK |
|---|---|---|---|---|---|---|---|---|---|---|---|---|---|---|---|---|---|---|---|---|---|---|---|---|---|---|---|---|---|---|---|---|---|---|---|
| 3622 | 04273 | 03621 | 1 | | 0 | 0 | 0 | 0 | 0 | 0 | 0 | 0 | 0 | 0 | 0 | 1 | 0 | 0 | 0 | 0 | 0 | 0 | 0 | 0 | 0 | 0 | 0 | 0 | 0 | | | | | | |
| 3623 | 04274 | 03622 | 1 | | 0 | 0 | 0 | 0 | 0 | 0 | 0 | 0 | 0 | 0 | 0 | 1 | 0 | 0 | 0 | 0 | 0 | 0 | 0 | 0 | 0 | 0 | 0 | 0 | 0 | | | | | | |
| 3624 | 04275 | 03623 | 2 | | 0 | 0 | 0 | 0 | 0 | 0 | 0 | 0 | 0 | 0 | 0 | 1 | 0 | 0 | 0 | 0 | 0 | 0 | 0 | 0 | 0 | 0 | 1 | 0 | 0 | | | | | | |
| 3625 | 04276 | 03624 | 1 | | 0 | 0 | 0 | 0 | 0 | 0 | 0 | 0 | 0 | 0 | 0 | 1 | 0 | 0 | 0 | 0 | 0 | 0 | 0 | 0 | 0 | 0 | 0 | 0 | 0 | | | | | | |
| 3626 | 04278 | 03625 | 4 | | 0 | 0 | 0 | 0 | 0 | 0 | 0 | 0 | 0 | 0 | 0 | 2 | 0 | 0 | 1 | 0 | 0 | 0 | 1 | 0 | 0 | 0 | 0 | 0 | 0 | | | | | | |
| 3627 | 04279 | 03626 | 1 | | 0 | 0 | 0 | 0 | 0 | 0 | 0 | 0 | 0 | 0 | 0 | 1 | 0 | 0 | 0 | 0 | 0 | 0 | 0 | 0 | 0 | 0 | 0 | 0 | 0 | | | | | | |
| 3628 | 04280 | 03627 | 1 | | 0 | 0 | 0 | 0 | 0 | 0 | 0 | 0 | 0 | 0 | 0 | 1 | 0 | 0 | 0 | 0 | 0 | 0 | 0 | 0 | 0 | 0 | 0 | 0 | 0 | | | | | | |
| 3629 | 04281 | 03628 | 1 | | 0 | 0 | 0 | 0 | 0 | 0 | 0 | 0 | 0 | 0 | 0 | 1 | 0 | 0 | 0 | 0 | 0 | 0 | 0 | 0 | 0 | 0 | 0 | 0 | 0 | | | | | | |
| 3630 | 04282 | 03629 | 1 | | 0 | 0 | 0 | 0 | 0 | 0 | 0 | 0 | 0 | 0 | 0 | 1 | 0 | 0 | 0 | 0 | 0 | 0 | 0 | 0 | 0 | 0 | 0 | 0 | 0 | | | | | | |
| 3631 | 04284 | 03630 | 1 | | 0 | 0 | 0 | 0 | 0 | 0 | 0 | 0 | 0 | 0 | 0 | 1 | 0 | 0 | 0 | 0 | 0 | 0 | 0 | 0 | 0 | 0 | 0 | 0 | 0 | | | | | | |
| 3632 | 04285 | 03631 | 1 | | 0 | 0 | 0 | 0 | 0 | 0 | 0 | 0 | 0 | 0 | 0 | 1 | 0 | 0 | 0 | 0 | 0 | 0 | 0 | 0 | 0 | 0 | 0 | 0 | 0 | | | | | | |
| 3633 | 04286 | 03632 | 1 | | 0 | 0 | 0 | 0 | 0 | 0 | 0 | 0 | 0 | 0 | 0 | 1 | 0 | 0 | 0 | 0 | 0 | 0 | 0 | 0 | 0 | 0 | 0 | 0 | 0 | | | | | | |
| 3634 | 04287 | 03633 | 2 | | 0 | 0 | 0 | 0 | 0 | 0 | 0 | 0 | 0 | 0 | 0 | 1 | 0 | 0 | 0 | 0 | 0 | 0 | 0 | 1 | 0 | 0 | 0 | 0 | 0 | | | | | | |
| 3635 | 04288 | 03634 | 3 | | 0 | 0 | 0 | 0 | 0 | 0 | 0 | 0 | 0 | 0 | 0 | 1 | 0 | 0 | 0 | 0 | 0 | 0 | 1 | 1 | 0 | 0 | 0 | 0 | 0 | X58141 | 95.8 | 356 | 1 | 3557 | 3936 |
| 3636 | 04289 | 03635 | 2 | | 0 | 0 | 0 | 0 | 0 | 0 | 0 | 0 | 0 | 0 | 0 | 1 | 0 | 0 | 0 | 1 | 0 | 0 | 0 | 0 | 0 | 0 | 0 | 0 | 0 | | | | | | |
| 3637 | 04290 | 03636 | 8 | | 0 | 0 | 0 | 0 | 0 | 0 | 0 | 0 | 0 | 0 | 0 | 1 | 0 | 0 | 1 | 0 | 0 | 0 | 0 | 1 | 0 | 0 | 5 | 0 | 0 | | | | | | |
| 3638 | 04291 | 03637 | 1 | | 0 | 0 | 0 | 0 | 0 | 0 | 0 | 0 | 0 | 0 | 0 | 1 | 0 | 0 | 0 | 0 | 0 | 0 | 0 | 0 | 0 | 0 | 0 | 0 | 0 | | | | | | |
| 3639 | 04292 | 03638 | 1 | | 0 | 0 | 0 | 0 | 0 | 0 | 0 | 0 | 0 | 0 | 0 | 1 | 0 | 0 | 0 | 0 | 0 | 0 | 0 | 0 | 0 | 0 | 0 | 0 | 0 | | | | | | |
| 3640 | 04293 | 03639 | 2 | | 0 | 0 | 0 | 0 | 0 | 0 | 0 | 0 | 0 | 0 | 0 | 1 | 0 | 0 | 0 | 0 | 0 | 0 | 1 | 0 | 0 | 0 | 0 | 0 | 0 | | | | | | |
| 3641 | 04294 | 03640 | 6 | | 0 | 0 | 0 | 0 | 0 | 0 | 0 | 0 | 0 | 0 | 0 | 2 | 0 | 0 | 0 | 0 | 1 | 1 | 0 | 0 | 0 | 0 | 0 | 2 | 0 | | | | | | |
| 3642 | 04295 | 03641 | 2 | | 0 | 0 | 0 | 0 | 0 | 0 | 0 | 0 | 0 | 0 | 0 | 1 | 0 | 1 | 0 | 0 | 0 | 0 | 0 | 0 | 0 | 0 | 0 | 0 | 0 | | | | | | |
| 3643 | 04296 | 03642 | 2 | | 0 | 0 | 0 | 0 | 0 | 0 | 0 | 0 | 0 | 0 | 0 | 2 | 0 | 0 | 0 | 0 | 0 | 0 | 0 | 0 | 0 | 0 | 0 | 0 | 0 | | | | | | |
| 3644 | 04297 | 03643 | 1 | | 0 | 0 | 0 | 0 | 0 | 0 | 0 | 0 | 0 | 0 | 0 | 1 | 0 | 0 | 0 | 0 | 0 | 0 | 0 | 0 | 0 | 0 | 0 | 0 | 0 | | | | | | |
| 3645 | 04298 | 03644 | 1 | | 0 | 0 | 0 | 0 | 0 | 0 | 0 | 0 | 0 | 0 | 0 | 1 | 0 | 0 | 0 | 0 | 0 | 0 | 0 | 0 | 0 | 0 | 0 | 0 | 0 | | | | | | |
| 3646 | 04299 | 03645 | 1 | | 0 | 0 | 0 | 0 | 0 | 0 | 0 | 0 | 0 | 0 | 0 | 1 | 0 | 0 | 0 | 0 | 0 | 0 | 0 | 0 | 0 | 0 | 0 | 0 | 0 | | | | | | |
| 3647 | 04300 | 03646 | 1 | | 0 | 0 | 0 | 0 | 0 | 0 | 0 | 0 | 0 | 0 | 0 | 1 | 0 | 0 | 0 | 0 | 0 | 0 | 0 | 0 | 0 | 0 | 0 | 0 | 0 | | | | | | |
| 3648 | 04302 | 03647 | 2 | | 0 | 0 | 0 | 0 | 0 | 0 | 0 | 0 | 0 | 0 | 0 | 1 | 0 | 0 | 0 | 0 | 0 | 0 | 0 | 0 | 0 | 1 | 0 | 0 | 0 | | | | | | |
| 3649 | 04303 | 03648 | 1 | | 0 | 0 | 0 | 0 | 0 | 0 | 0 | 0 | 0 | 0 | 0 | 1 | 0 | 0 | 0 | 0 | 0 | 0 | 0 | 0 | 0 | 0 | 0 | 0 | 0 | | | | | | |
| 3650 | 04304 | 03649 | 1 | | 0 | 0 | 0 | 0 | 0 | 0 | 0 | 0 | 0 | 0 | 0 | 1 | 0 | 0 | 0 | 0 | 0 | 0 | 0 | 0 | 0 | 0 | 0 | 0 | 0 | | | | | | |
| 3651 | 04305 | 03650 | 3 | | 0 | 0 | 0 | 0 | 0 | 0 | 0 | 0 | 0 | 0 | 0 | 1 | 0 | 0 | 0 | 0 | 0 | 0 | 0 | 0 | 0 | 0 | 0 | 2 | 0 | | | | | | |
| 3652 | 04306 | 03651 | 1 | | 0 | 0 | 0 | 0 | 0 | 0 | 0 | 0 | 0 | 0 | 0 | 1 | 0 | 0 | 0 | 0 | 0 | 0 | 0 | 0 | 0 | 0 | 0 | 0 | 0 | | | | | | |
| 3653 | 04307 | 03652 | 2 | | 0 | 0 | 0 | 0 | 0 | 0 | 0 | 0 | 0 | 0 | 0 | 1 | 0 | 0 | 0 | 0 | 1 | 0 | 0 | 0 | 0 | 0 | 0 | 0 | 0 | | | | | | |
| 3654 | 04308 | 03653 | 1 | | 0 | 0 | 0 | 0 | 0 | 0 | 0 | 0 | 0 | 0 | 0 | 1 | 0 | 0 | 0 | 0 | 0 | 0 | 0 | 0 | 0 | 0 | 0 | 0 | 0 | | | | | | |
| 3655 | 04309 | 03654 | 2 | | 0 | 0 | 0 | 0 | 0 | 0 | 0 | 0 | 0 | 0 | 0 | 1 | 0 | 0 | 0 | 0 | 0 | 0 | 0 | 0 | 0 | 0 | 0 | 1 | 0 | | | | | | |
| 3656 | 04310 | 03655 | 1 | | 0 | 0 | 0 | 0 | 0 | 0 | 0 | 0 | 0 | 0 | 0 | 1 | 0 | 0 | 0 | 0 | 0 | 0 | 0 | 0 | 0 | 0 | 0 | 0 | 0 | | | | | | |
| 3657 | 04311 | 03656 | 3 | | 0 | 0 | 0 | 0 | 0 | 0 | 0 | 0 | 0 | 0 | 0 | 2 | 0 | 0 | 0 | 0 | 0 | 1 | 0 | 0 | 0 | 0 | 0 | 0 | 0 | | | | | | |

Table 102

| | A | B | C | E | G | I | K | M | O | Q | S | U | W | Y | AA | AC | AE | AG | AI | AK | AM | AO | AQ | AS | AU | AW | AY | BA | BC | BE | BF | BG | BH | BI | BK |
|---|---|---|---|---|---|---|---|---|---|---|---|---|---|---|---|---|---|---|---|---|---|---|---|---|---|---|---|---|---|---|---|---|---|---|---|
| 3658 | 04312 | 03657 | 3 | | 0 | 0 | 0 | 0 | 0 | 0 | 0 | 0 | 0 | 0 | 0 | 2 | 0 | 1 | 0 | 0 | 0 | 0 | 0 | 0 | 0 | 0 | 0 | 0 | 0 | | | | | | |
| 3659 | 04313 | 03658 | 1 | | 0 | 0 | 0 | 0 | 0 | 0 | 0 | 0 | 0 | 0 | 0 | 1 | 0 | 0 | 0 | 0 | 0 | 0 | 0 | 0 | 0 | 0 | 0 | 0 | 0 | | | | | | |
| 3660 | 04314 | 03659 | 1 | | 0 | 0 | 0 | 0 | 0 | 0 | 0 | 0 | 0 | 0 | 0 | 1 | 0 | 0 | 0 | 0 | 0 | 0 | 0 | 0 | 0 | 0 | 0 | 0 | 0 | | | | | | |
| 3661 | 04315 | 03660 | 3 | | 0 | 0 | 0 | 0 | 0 | 0 | 0 | 0 | 0 | 0 | 0 | 1 | 0 | 0 | 0 | 1 | 0 | 0 | 0 | 0 | 0 | 1 | 0 | 0 | 0 | | | | | | |
| 3662 | 04316 | 03661 | 1 | | 0 | 0 | 0 | 0 | 0 | 0 | 0 | 0 | 0 | 0 | 0 | 1 | 0 | 0 | 0 | 0 | 0 | 0 | 0 | 0 | 0 | 0 | 0 | 0 | 0 | | | | | | |
| 3663 | 04317 | 03662 | 1 | | 0 | 0 | 0 | 0 | 0 | 0 | 0 | 0 | 0 | 0 | 0 | 1 | 0 | 0 | 0 | 0 | 0 | 0 | 0 | 0 | 0 | 0 | 0 | 0 | 0 | | | | | | |
| 3664 | 04318 | 03663 | 1 | | 0 | 0 | 0 | 0 | 0 | 0 | 0 | 0 | 0 | 0 | 0 | 1 | 0 | 0 | 0 | 0 | 0 | 0 | 0 | 0 | 0 | 0 | 0 | 0 | 0 | | | | | | |
| 3665 | 04319 | 03664 | 1 | | 0 | 0 | 0 | 0 | 0 | 0 | 0 | 0 | 0 | 0 | 0 | 1 | 0 | 0 | 0 | 0 | 0 | 0 | 0 | 0 | 0 | 0 | 0 | 0 | 0 | | | | | | |
| 3666 | 04320 | 03665 | 3 | | 0 | 0 | 0 | 0 | 0 | 0 | 0 | 0 | 0 | 0 | 0 | 1 | 0 | 0 | 1 | 0 | 0 | 0 | 0 | 1 | 0 | 0 | 0 | 0 | 0 | | | | | | |
| 3667 | 04321 | 03666 | 3 | | 0 | 0 | 0 | 0 | 0 | 0 | 0 | 0 | 0 | 0 | 0 | 1 | 0 | 0 | 1 | 0 | 0 | 0 | 0 | 0 | 0 | 0 | 1 | 0 | 0 | | | | | | |
| 3668 | 04322 | 03667 | 12 | | 0 | 0 | 0 | 0 | 0 | 0 | 0 | 0 | 0 | 0 | 3 | 1 | 1 | 1 | 1 | 1 | 0 | 2 | 2 | 0 | 0 | 0 | 0 | 0 | 0 | M86400 | 99.8 | 415 | 1 | 2379 | 2834 |
| 3669 | 04323 | 03668 | 1 | | 0 | 0 | 0 | 0 | 0 | 0 | 0 | 0 | 0 | 0 | 0 | 1 | 0 | 0 | 0 | 0 | 0 | 0 | 0 | 0 | 0 | 0 | 0 | 0 | 0 | | | | | | |
| 3670 | 04324 | 03669 | 1 | | 0 | 0 | 0 | 0 | 0 | 0 | 0 | 0 | 0 | 0 | 0 | 1 | 0 | 0 | 0 | 0 | 0 | 0 | 0 | 0 | 0 | 0 | 0 | 0 | 0 | | | | | | |
| 3671 | 04325 | 03670 | 2 | | 0 | 0 | 0 | 0 | 0 | 0 | 0 | 0 | 0 | 0 | 0 | 2 | 0 | 0 | 0 | 0 | 0 | 0 | 0 | 0 | 0 | 0 | 0 | 0 | 0 | | | | | | |
| 3672 | 04326 | 03671 | 2 | | 0 | 0 | 0 | 0 | 0 | 0 | 0 | 0 | 0 | 0 | 0 | 1 | 0 | 0 | 0 | 0 | 0 | 0 | 0 | 1 | 0 | 0 | 0 | 0 | 0 | | | | | | |
| 3673 | 04327 | 03672 | 1 | | 0 | 0 | 0 | 0 | 0 | 0 | 0 | 0 | 0 | 0 | 0 | 1 | 0 | 0 | 0 | 0 | 0 | 0 | 0 | 0 | 0 | 0 | 0 | 0 | 0 | | | | | | |
| 3674 | 04328 | 03673 | 1 | | 0 | 0 | 0 | 0 | 0 | 0 | 0 | 0 | 0 | 0 | 0 | 1 | 0 | 0 | 0 | 0 | 0 | 0 | 0 | 0 | 0 | 0 | 0 | 0 | 0 | | | | | | |
| 3675 | 04329 | 03674 | 1 | | 0 | 0 | 0 | 0 | 0 | 0 | 0 | 0 | 0 | 0 | 0 | 1 | 0 | 0 | 0 | 0 | 0 | 0 | 0 | 0 | 0 | 0 | 0 | 0 | 0 | | | | | | |
| 3676 | 04330 | 03675 | 2 | | 0 | 0 | 0 | 0 | 0 | 0 | 0 | 0 | 0 | 0 | 0 | 2 | 0 | 0 | 0 | 0 | 0 | 0 | 0 | 0 | 0 | 0 | 0 | 0 | 0 | | | | | | |
| 3677 | 04331 | 03676 | 1 | | 0 | 0 | 0 | 0 | 0 | 0 | 0 | 0 | 0 | 0 | 0 | 1 | 0 | 0 | 0 | 0 | 0 | 0 | 0 | 0 | 0 | 0 | 0 | 0 | 0 | | | | | | |
| 3678 | 04332 | 03677 | 1 | | 0 | 0 | 0 | 0 | 0 | 0 | 0 | 0 | 0 | 0 | 0 | 1 | 0 | 0 | 0 | 0 | 0 | 0 | 0 | 0 | 0 | 0 | 0 | 0 | 0 | | | | | | |
| 3679 | 04333 | 03678 | 1 | | 0 | 0 | 0 | 0 | 0 | 0 | 0 | 0 | 0 | 0 | 0 | 1 | 0 | 0 | 0 | 0 | 0 | 0 | 0 | 0 | 0 | 0 | 0 | 0 | 0 | | | | | | |
| 3680 | 04334 | 03679 | 2 | | 0 | 0 | 0 | 0 | 0 | 0 | 0 | 0 | 0 | 0 | 0 | 1 | 0 | 1 | 0 | 0 | 0 | 0 | 0 | 0 | 0 | 0 | 0 | 0 | 0 | | | | | | |
| 3681 | 04335 | 03680 | 1 | | 0 | 0 | 0 | 0 | 0 | 0 | 0 | 0 | 0 | 0 | 0 | 1 | 0 | 0 | 0 | 0 | 0 | 0 | 0 | 0 | 0 | 0 | 0 | 0 | 0 | | | | | | |
| 3682 | 04336 | 03681 | 2 | | 0 | 0 | 0 | 0 | 0 | 0 | 0 | 0 | 0 | 0 | 0 | 1 | 0 | 0 | 0 | 0 | 0 | 0 | 0 | 0 | 0 | 1 | 0 | 0 | 0 | | | | | | |
| 3683 | 04337 | 03682 | 1 | | 0 | 0 | 0 | 0 | 0 | 0 | 0 | 0 | 0 | 0 | 0 | 1 | 0 | 0 | 0 | 0 | 0 | 0 | 0 | 0 | 0 | 0 | 0 | 0 | 0 | | | | | | |
| 3684 | 04339 | 03683 | 3 | | 0 | 0 | 0 | 0 | 0 | 0 | 0 | 0 | 0 | 0 | 0 | 1 | 0 | 0 | 2 | 0 | 0 | 0 | 0 | 0 | 0 | 0 | 0 | 0 | 0 | | | | | | |
| 3685 | 04340 | 03684 | 2 | | 0 | 0 | 0 | 0 | 0 | 0 | 0 | 0 | 0 | 0 | 0 | 1 | 0 | 0 | 0 | 1 | 0 | 0 | 0 | 0 | 0 | 0 | 0 | 0 | 0 | | | | | | |
| 3686 | 04341 | 03685 | 1 | | 0 | 0 | 0 | 0 | 0 | 0 | 0 | 0 | 0 | 0 | 0 | 1 | 0 | 0 | 0 | 0 | 0 | 0 | 0 | 0 | 0 | 0 | 0 | 0 | 0 | | | | | | |
| 3687 | 04342 | 03686 | 2 | | 0 | 0 | 0 | 0 | 0 | 0 | 0 | 0 | 0 | 0 | 0 | 1 | 0 | 0 | 1 | 0 | 0 | 0 | 0 | 0 | 0 | 0 | 0 | 0 | 0 | | | | | | |
| 3688 | 04343 | 03687 | 1 | | 0 | 0 | 0 | 0 | 0 | 0 | 0 | 0 | 0 | 0 | 0 | 1 | 0 | 0 | 0 | 0 | 0 | 0 | 0 | 0 | 0 | 0 | 0 | 0 | 0 | | | | | | |
| 3689 | 04344 | 03688 | 1 | | 0 | 0 | 0 | 0 | 0 | 0 | 0 | 0 | 0 | 0 | 0 | 1 | 0 | 0 | 0 | 0 | 0 | 0 | 0 | 0 | 0 | 0 | 0 | 0 | 0 | M92299 | 97.7 | 259 | 1 | 1779 | 2037 |
| 3690 | 04345 | 03689 | 8 | | 0 | 0 | 0 | 0 | 0 | 0 | 0 | 0 | 0 | 0 | 0 | 1 | 0 | 0 | 0 | 0 | 0 | 0 | 1 | 0 | 0 | 0 | 6 | 0 | 0 | | | | | | |
| 3691 | 04346 | 03690 | 1 | | 0 | 0 | 0 | 0 | 0 | 0 | 0 | 0 | 0 | 0 | 0 | 1 | 0 | 0 | 0 | 0 | 0 | 0 | 0 | 0 | 0 | 0 | 0 | 0 | 0 | | | | | | |
| 3692 | 04347 | 03691 | 1 | | 0 | 0 | 0 | 0 | 0 | 0 | 0 | 0 | 0 | 0 | 0 | 1 | 0 | 0 | 0 | 0 | 0 | 0 | 0 | 0 | 0 | 0 | 0 | 0 | 0 | | | | | | |
| 3693 | 04348 | 03692 | 1 | | 0 | 0 | 0 | 0 | 0 | 0 | 0 | 0 | 0 | 0 | 0 | 1 | 0 | 0 | 0 | 0 | 0 | 0 | 0 | 0 | 0 | 0 | 0 | 0 | 0 | | | | | | |

Table 103

| | A | B | C | E | G | I | K | M | O | Q | S | U | W | Y | AA | AC | AE | AG | AI | AK | AM | AO | AQ | AS | AU | AW | AY | BA | BC | BE | BF | BG | BH | BI | BK |
|---|---|---|---|---|---|---|---|---|---|---|---|---|---|---|---|---|---|---|---|---|---|---|---|---|---|---|---|---|---|---|---|---|---|---|---|
| 3694 | 04349 | 03693 | 1 | | 0 | 0 | 0 | 0 | 0 | 0 | 0 | 0 | 0 | 0 | 0 | 1 | 0 | 0 | 0 | 0 | 0 | 0 | 0 | 0 | 0 | 0 | 0 | 0 | 0 | | | | | | |
| 3695 | 04351 | 03694 | 1 | | 0 | 0 | 0 | 0 | 0 | 0 | 0 | 0 | 0 | 0 | 0 | 1 | 0 | 0 | 0 | 0 | 0 | 0 | 0 | 0 | 0 | 0 | 0 | 0 | 0 | | | | | | |
| 3696 | 04352 | 03695 | 1 | | 0 | 0 | 0 | 0 | 0 | 0 | 0 | 0 | 0 | 0 | 0 | 1 | 0 | 0 | 0 | 0 | 0 | 0 | 0 | 0 | 0 | 0 | 0 | 0 | 0 | | | | | | |
| 3697 | 04353 | 03696 | 1 | | 0 | 0 | 0 | 0 | 0 | 0 | 0 | 0 | 0 | 0 | 0 | 1 | 0 | 0 | 0 | 0 | 0 | 0 | 0 | 0 | 0 | 0 | 0 | 0 | 0 | | | | | | |
| 3698 | 04354 | 03697 | 4 | | 0 | 0 | 0 | 0 | 0 | 0 | 0 | 0 | 0 | 0 | 0 | 2 | 0 | 1 | 0 | 0 | 0 | 1 | 0 | 0 | 0 | 0 | 0 | 0 | 0 | M63167 | 98.2 | 217 | 1 | 2394 | 2610 |
| 3699 | 04355 | 03698 | 1 | | 0 | 0 | 0 | 0 | 0 | 0 | 0 | 0 | 0 | 0 | 0 | 1 | 0 | 0 | 0 | 0 | 0 | 0 | 0 | 0 | 0 | 0 | 0 | 0 | 0 | | | | | | |
| 3700 | 04356 | 03699 | 1 | | 0 | 0 | 0 | 0 | 0 | 0 | 0 | 0 | 0 | 0 | 0 | 1 | 0 | 0 | 0 | 0 | 0 | 0 | 0 | 0 | 0 | 0 | 0 | 0 | 0 | | | | | | |
| 3701 | 04357 | 03700 | 2 | | 0 | 0 | 0 | 0 | 0 | 0 | 0 | 0 | 0 | 0 | 0 | 1 | 0 | 0 | 0 | 0 | 0 | 0 | 0 | 0 | 0 | 0 | 0 | 1 | 0 | | | | | | |
| 3702 | 04358 | 03701 | 1 | | 0 | 0 | 0 | 0 | 0 | 0 | 0 | 0 | 0 | 0 | 0 | 1 | 0 | 0 | 0 | 0 | 0 | 0 | 0 | 0 | 0 | 0 | 0 | 0 | 0 | | | | | | |
| 3703 | 04359 | 03702 | 1 | | 0 | 0 | 0 | 0 | 0 | 0 | 0 | 0 | 0 | 0 | 0 | 1 | 0 | 0 | 0 | 0 | 0 | 0 | 0 | 0 | 0 | 0 | 0 | 0 | 0 | | | | | | |
| 3704 | 04360 | 03703 | 2 | | 0 | 0 | 0 | 0 | 0 | 0 | 0 | 0 | 0 | 0 | 0 | 1 | 0 | 0 | 0 | 1 | 0 | 0 | 0 | 0 | 0 | 0 | 0 | 0 | 0 | | | | | | |
| 3705 | 04361 | 03704 | 1 | | 0 | 0 | 0 | 0 | 0 | 0 | 0 | 0 | 0 | 0 | 0 | 1 | 0 | 0 | 0 | 0 | 0 | 0 | 0 | 0 | 0 | 0 | 0 | 0 | 0 | K02054 | 96.9 | 224 | 1 | 575 | 797 |
| 3706 | 04362 | 03705 | 2 | | 0 | 0 | 0 | 0 | 0 | 0 | 0 | 0 | 0 | 0 | 0 | 1 | 1 | 0 | 0 | 0 | 0 | 0 | 0 | 0 | 0 | 0 | 0 | 0 | 0 | | | | | | |
| 3707 | 04363 | 03706 | 2 | | 0 | 0 | 0 | 0 | 0 | 0 | 0 | 0 | 0 | 0 | 0 | 1 | 0 | 0 | 0 | 0 | 0 | 0 | 0 | 1 | 0 | 0 | 0 | 0 | 0 | | | | | | |
| 3708 | 04364 | 03707 | 2 | | 0 | 0 | 0 | 0 | 0 | 0 | 0 | 0 | 0 | 0 | 0 | 1 | 1 | 0 | 0 | 0 | 0 | 0 | 0 | 0 | 0 | 0 | 0 | 0 | 0 | | | | | | |
| 3709 | 04365 | 03708 | 1 | | 0 | 0 | 0 | 0 | 0 | 0 | 0 | 0 | 0 | 0 | 0 | 1 | 0 | 0 | 0 | 0 | 0 | 0 | 0 | 0 | 0 | 0 | 0 | 0 | 0 | | | | | | |
| 3710 | 04366 | 03709 | 1 | | 0 | 0 | 0 | 0 | 0 | 0 | 0 | 0 | 0 | 0 | 0 | 1 | 0 | 0 | 0 | 0 | 0 | 0 | 0 | 0 | 0 | 0 | 0 | 0 | 0 | | | | | | |
| 3711 | 04368 | 03710 | 1 | | 0 | 0 | 0 | 0 | 0 | 0 | 0 | 0 | 0 | 0 | 0 | 1 | 0 | 0 | 0 | 0 | 0 | 0 | 0 | 0 | 0 | 0 | 0 | 0 | 0 | | | | | | |
| 3712 | 04369 | 03711 | 1 | | 0 | 0 | 0 | 0 | 0 | 0 | 0 | 0 | 0 | 0 | 0 | 1 | 0 | 0 | 0 | 0 | 0 | 0 | 0 | 0 | 0 | 0 | 0 | 0 | 0 | | | | | | |
| 3713 | 04370 | 03712 | 2 | | 0 | 0 | 0 | 0 | 0 | 0 | 0 | 0 | 0 | 0 | 0 | 1 | 0 | 0 | 0 | 0 | 0 | 0 | 1 | 0 | 0 | 0 | 0 | 0 | 0 | | | | | | |
| 3714 | 04371 | 03713 | 1 | | 0 | 0 | 0 | 0 | 0 | 0 | 0 | 0 | 0 | 0 | 0 | 1 | 0 | 0 | 0 | 0 | 0 | 0 | 0 | 0 | 0 | 0 | 0 | 0 | 0 | | | | | | |
| 3715 | 04372 | 03714 | 3 | | 0 | 0 | 0 | 0 | 0 | 0 | 0 | 0 | 0 | 0 | 0 | 1 | 2 | 0 | 0 | 0 | 0 | 0 | 0 | 0 | 0 | 0 | 0 | 0 | 0 | | | | | | |
| 3716 | 04373 | 03715 | 3 | | 0 | 0 | 0 | 0 | 0 | 0 | 0 | 0 | 0 | 0 | 0 | 1 | 0 | 0 | 0 | 0 | 0 | 0 | 0 | 0 | 0 | 0 | 0 | 2 | 0 | X62534 | 96.4 | 332 | 1 | 550 | 1301 |
| 3717 | 04374 | 03716 | 2 | | 0 | 0 | 0 | 0 | 0 | 0 | 0 | 0 | 0 | 0 | 0 | 2 | 0 | 0 | 0 | 0 | 0 | 0 | 0 | 0 | 0 | 0 | 0 | 0 | 0 | | | | | | |
| 3718 | 04375 | 03717 | 2 | | 0 | 0 | 0 | 0 | 0 | 0 | 0 | 0 | 0 | 0 | 0 | 1 | 1 | 0 | 0 | 0 | 0 | 0 | 0 | 0 | 0 | 0 | 0 | 0 | 0 | | | | | | |
| 3719 | 04377 | 03718 | 2 | | 0 | 0 | 0 | 0 | 0 | 0 | 0 | 0 | 0 | 0 | 0 | 1 | 0 | 0 | 0 | 0 | 0 | 0 | 1 | 0 | 0 | 0 | 0 | 0 | 0 | | | | | | |
| 3720 | 04378 | 03719 | 1 | | 0 | 0 | 0 | 0 | 0 | 0 | 0 | 0 | 0 | 0 | 0 | 1 | 0 | 0 | 0 | 0 | 0 | 0 | 0 | 0 | 0 | 0 | 0 | 0 | 0 | | | | | | |
| 3721 | 04379 | 03720 | 1 | | 0 | 0 | 0 | 0 | 0 | 0 | 0 | 0 | 0 | 0 | 0 | 1 | 0 | 0 | 0 | 0 | 0 | 0 | 0 | 0 | 0 | 0 | 0 | 0 | 0 | | | | | | |
| 3722 | 04380 | 03721 | 1 | | 0 | 0 | 0 | 0 | 0 | 0 | 0 | 0 | 0 | 0 | 0 | 1 | 0 | 0 | 0 | 0 | 0 | 0 | 0 | 0 | 0 | 0 | 0 | 0 | 0 | | | | | | |
| 3723 | 04381 | 03722 | 1 | | 0 | 0 | 0 | 0 | 0 | 0 | 0 | 0 | 0 | 0 | 0 | 1 | 0 | 0 | 0 | 0 | 0 | 0 | 0 | 0 | 0 | 0 | 0 | 0 | 0 | | | | | | |
| 3724 | 04382 | 03723 | 1 | | 0 | 0 | 0 | 0 | 0 | 0 | 0 | 0 | 0 | 0 | 0 | 1 | 0 | 0 | 0 | 0 | 0 | 0 | 0 | 0 | 0 | 0 | 0 | 0 | 0 | | | | | | |
| 3725 | 04383 | 03724 | 1 | | 0 | 0 | 0 | 0 | 0 | 0 | 0 | 0 | 0 | 0 | 0 | 1 | 0 | 0 | 0 | 0 | 0 | 0 | 0 | 0 | 0 | 0 | 0 | 0 | 0 | | | | | | |
| 3726 | 04384 | 03725 | 1 | | 0 | 0 | 0 | 0 | 0 | 0 | 0 | 0 | 0 | 0 | 0 | 1 | 0 | 0 | 0 | 0 | 0 | 0 | 0 | 0 | 0 | 0 | 0 | 0 | 0 | | | | | | |
| 3727 | 04385 | 03726 | 1 | | 0 | 0 | 0 | 0 | 0 | 0 | 0 | 0 | 0 | 0 | 0 | 1 | 0 | 0 | 0 | 0 | 0 | 0 | 0 | 0 | 0 | 0 | 0 | 0 | 0 | | | | | | |
| 3728 | 04386 | 03727 | 3 | | 0 | 0 | 0 | 0 | 0 | 0 | 0 | 0 | 0 | 0 | 0 | 1 | 0 | 0 | 0 | 0 | 0 | 0 | 0 | 0 | 0 | 0 | 2 | 0 | 0 | | | | | | |
| 3729 | 04387 | 03728 | 2 | | 0 | 0 | 0 | 0 | 0 | 0 | 0 | 0 | 0 | 0 | 0 | 2 | 0 | 0 | 0 | 0 | 0 | 0 | 0 | 0 | 0 | 0 | 0 | 0 | 0 | | | | | | |

Table 104

| | A | B | C | E | G | I | K | M | O | Q | S | U | W | Y | AA | AC | AE | AG | AI | AK | AM | AO | AQ | AS | AU | AW | AY | BA | BC | BE | BF | BG | BH | BI | BK |
|---|---|---|---|---|---|---|---|---|---|---|---|---|---|---|---|---|---|---|---|---|---|---|---|---|---|---|---|---|---|---|---|---|---|---|---|
| 3730 | 04388 | 03729 | 1 | | 0 | 0 | 0 | 0 | 0 | 0 | 0 | 0 | 0 | 0 | 0 | 1 | 0 | 0 | 0 | 0 | 0 | 0 | 0 | 0 | 0 | 0 | 0 | 0 | 0 | | | | | | |
| 3731 | 04389 | 03730 | 3 | | 0 | 0 | 0 | 0 | 0 | 0 | 0 | 0 | 0 | 0 | 0 | 1 | 0 | 0 | 0 | 0 | 0 | 0 | 0 | 2 | 0 | 0 | 0 | 0 | 0 | | | | | | |
| 3732 | 04390 | 03731 | 2 | | 0 | 0 | 0 | 0 | 0 | 0 | 0 | 0 | 0 | 0 | 0 | 1 | 0 | 0 | 0 | 0 | 0 | 0 | 0 | 1 | 0 | 0 | 0 | 0 | 0 | | | | | | |
| 3733 | 04391 | 03732 | 3 | | 0 | 0 | 0 | 0 | 0 | 0 | 0 | 0 | 0 | 0 | 0 | 3 | 0 | 0 | 0 | 0 | 0 | 0 | 0 | 0 | 0 | 0 | 0 | 0 | 0 | | | | | | |
| 3734 | 04392 | 03733 | 1 | | 0 | 0 | 0 | 0 | 0 | 0 | 0 | 0 | 0 | 0 | 0 | 1 | 0 | 0 | 0 | 0 | 0 | 0 | 0 | 0 | 0 | 0 | 0 | 0 | 0 | | | | | | |
| 3735 | 04393 | 03734 | 3 | | 0 | 0 | 0 | 0 | 0 | 0 | 0 | 0 | 0 | 0 | 0 | 1 | 0 | 0 | 0 | 1 | 0 | 0 | 0 | 0 | 0 | 0 | 1 | 0 | 0 | | | | | | |
| 3736 | 04394 | 03735 | 1 | | 0 | 0 | 0 | 0 | 0 | 0 | 0 | 0 | 0 | 0 | 0 | 1 | 0 | 0 | 0 | 0 | 0 | 0 | 0 | 0 | 0 | 0 | 0 | 0 | 0 | | | | | | |
| 3737 | 04395 | 03736 | 2 | | 0 | 0 | 0 | 0 | 0 | 0 | 0 | 0 | 0 | 0 | 0 | 1 | 0 | 1 | 0 | 0 | 0 | 0 | 0 | 0 | 0 | 0 | 0 | 0 | 0 | | | | | | |
| 3738 | 04396 | 03737 | 1 | | 0 | 0 | 0 | 0 | 0 | 0 | 0 | 0 | 0 | 0 | 0 | 1 | 0 | 0 | 0 | 0 | 0 | 0 | 0 | 0 | 0 | 0 | 0 | 0 | 0 | | | | | | |
| 3739 | 04397 | 03738 | 1 | | 0 | 0 | 0 | 0 | 0 | 0 | 0 | 0 | 0 | 0 | 0 | 1 | 0 | 0 | 0 | 0 | 0 | 0 | 0 | 0 | 0 | 0 | 0 | 0 | 0 | | | | | | |
| 3740 | 04398 | 03739 | 1 | | 0 | 0 | 0 | 0 | 0 | 0 | 0 | 0 | 0 | 0 | 0 | 1 | 0 | 0 | 0 | 0 | 0 | 0 | 0 | 0 | 0 | 0 | 0 | 0 | 0 | | | | | | |
| 3741 | 04399 | 03740 | 1 | | 0 | 0 | 0 | 0 | 0 | 0 | 0 | 0 | 0 | 0 | 0 | 1 | 0 | 0 | 0 | 0 | 0 | 0 | 0 | 0 | 0 | 0 | 0 | 0 | 0 | | | | | | |
| 3742 | 04400 | 03741 | 1 | | 0 | 0 | 0 | 0 | 0 | 0 | 0 | 0 | 0 | 0 | 0 | 1 | 0 | 0 | 0 | 0 | 0 | 0 | 0 | 0 | 0 | 0 | 0 | 0 | 0 | | | | | | |
| 3743 | 04401 | 03742 | 1 | | 0 | 0 | 0 | 0 | 0 | 0 | 0 | 0 | 0 | 0 | 0 | 1 | 0 | 0 | 0 | 0 | 0 | 0 | 0 | 0 | 0 | 0 | 0 | 0 | 0 | | | | | | |
| 3744 | 04402 | 03743 | 7 | | 0 | 0 | 0 | 0 | 0 | 0 | 0 | 0 | 0 | 0 | 0 | 1 | 0 | 0 | 2 | 3 | 0 | 1 | 0 | 0 | 0 | 0 | 0 | 0 | 0 | D13629 | 97.2 | 177 | 1 | 4282 | 4457 |
| 3745 | 04403 | 03744 | 1 | | 0 | 0 | 0 | 0 | 0 | 0 | 0 | 0 | 0 | 0 | 0 | 1 | 0 | 0 | 0 | 0 | 0 | 0 | 0 | 0 | 0 | 0 | 0 | 0 | 0 | L25080 | 95 | 179 | 1 | 509 | 1777 |
| 3746 | 04404 | 03745 | 1 | | 0 | 0 | 0 | 0 | 0 | 0 | 0 | 0 | 0 | 0 | 0 | 1 | 0 | 0 | 0 | 0 | 0 | 0 | 0 | 0 | 0 | 0 | 0 | 0 | 0 | M31523 | 98.9 | 175 | 1 | 4222 | 4396 |
| 3747 | 04405 | 03746 | 2 | | 0 | 0 | 0 | 0 | 0 | 0 | 0 | 0 | 0 | 0 | 0 | 1 | 0 | 0 | 1 | 0 | 0 | 0 | 0 | 0 | 0 | 0 | 0 | 0 | 0 | | | | | | |
| 3748 | 04406 | 03747 | 1 | | 0 | 0 | 0 | 0 | 0 | 0 | 0 | 0 | 0 | 0 | 0 | 1 | 0 | 0 | 0 | 0 | 0 | 0 | 0 | 0 | 0 | 0 | 0 | 0 | 0 | | | | | | |
| 3749 | 04407 | 03748 | 3 | | 0 | 0 | 0 | 0 | 0 | 0 | 0 | 0 | 0 | 0 | 0 | 1 | 0 | 0 | 0 | 2 | 0 | 0 | 0 | 0 | 0 | 0 | 0 | 0 | 0 | | | | | | |
| 3750 | 04408 | 03749 | 1 | | 0 | 0 | 0 | 0 | 0 | 0 | 0 | 0 | 0 | 0 | 0 | 1 | 0 | 0 | 0 | 0 | 0 | 0 | 0 | 0 | 0 | 0 | 0 | 0 | 0 | | | | | | |
| 3751 | 04409 | 03750 | 1 | | 0 | 0 | 0 | 0 | 0 | 0 | 0 | 0 | 0 | 0 | 0 | 1 | 0 | 0 | 0 | 0 | 0 | 0 | 0 | 0 | 0 | 0 | 0 | 0 | 0 | | | | | | |
| 3752 | 04410 | 03751 | 1 | | 0 | 0 | 0 | 0 | 0 | 0 | 0 | 0 | 0 | 0 | 0 | 1 | 0 | 0 | 0 | 0 | 0 | 0 | 0 | 0 | 0 | 0 | 0 | 0 | 0 | | | | | | |
| 3753 | 04411 | 03752 | 2 | | 0 | 0 | 0 | 0 | 0 | 0 | 0 | 0 | 0 | 0 | 0 | 1 | 0 | 0 | 1 | 0 | 0 | 0 | 0 | 0 | 0 | 0 | 0 | 0 | 0 | | | | | | |
| 3754 | 04412 | 03753 | 2 | | 0 | 0 | 0 | 0 | 0 | 0 | 0 | 0 | 0 | 0 | 0 | 1 | 0 | 0 | 0 | 1 | 0 | 0 | 0 | 0 | 0 | 0 | 0 | 0 | 0 | | | | | | |
| 3755 | 04414 | 03754 | 5 | | 0 | 0 | 0 | 0 | 0 | 0 | 0 | 0 | 0 | 0 | 0 | 1 | 0 | 0 | 1 | 1 | 1 | 0 | 0 | 0 | 0 | 1 | 0 | 0 | 0 | | | | | | |
| 3756 | 04415 | 03755 | 3 | | 0 | 0 | 0 | 0 | 0 | 0 | 0 | 0 | 0 | 0 | 0 | 1 | 1 | 0 | 0 | 0 | 0 | 0 | 1 | 0 | 0 | 0 | 0 | 0 | 0 | | | | | | |
| 3757 | 04416 | 03756 | 1 | | 0 | 0 | 0 | 0 | 0 | 0 | 0 | 0 | 0 | 0 | 0 | 1 | 0 | 0 | 0 | 0 | 0 | 0 | 0 | 0 | 0 | 0 | 0 | 0 | 0 | | | | | | |
| 3758 | 04417 | 03757 | 2 | | 0 | 0 | 0 | 0 | 0 | 0 | 0 | 0 | 0 | 0 | 0 | 2 | 0 | 0 | 0 | 0 | 0 | 0 | 0 | 0 | 0 | 0 | 0 | 0 | 0 | | | | | | |
| 3759 | 04418 | 03758 | 2 | | 0 | 0 | 0 | 0 | 0 | 0 | 0 | 0 | 0 | 0 | 0 | 1 | 0 | 0 | 0 | 0 | 0 | 0 | 0 | 1 | 0 | 0 | 0 | 0 | 0 | | | | | | |
| 3760 | 04419 | 03759 | 1 | | 0 | 0 | 0 | 0 | 0 | 0 | 0 | 0 | 0 | 0 | 0 | 1 | 0 | 0 | 0 | 0 | 0 | 0 | 0 | 0 | 0 | 0 | 0 | 0 | 0 | | | | | | |
| 3761 | 04420 | 03760 | 1 | | 0 | 0 | 0 | 0 | 0 | 0 | 0 | 0 | 0 | 0 | 0 | 1 | 0 | 0 | 0 | 0 | 0 | 0 | 0 | 0 | 0 | 0 | 0 | 0 | 0 | | | | | | |
| 3762 | 04421 | 03761 | 2 | | 0 | 0 | 0 | 0 | 0 | 0 | 0 | 0 | 0 | 0 | 0 | 1 | 0 | 0 | 0 | 0 | 0 | 0 | 0 | 1 | 0 | 0 | 0 | 0 | 0 | | | | | | |
| 3763 | 04422 | 03762 | 1 | | 0 | 0 | 0 | 0 | 0 | 0 | 0 | 0 | 0 | 0 | 0 | 1 | 0 | 0 | 0 | 0 | 0 | 0 | 0 | 0 | 0 | 0 | 0 | 0 | 0 | | | | | | |
| 3764 | 04423 | 03763 | 1 | | 0 | 0 | 0 | 0 | 0 | 0 | 0 | 0 | 0 | 0 | 0 | 1 | 0 | 0 | 0 | 0 | 0 | 0 | 0 | 0 | 0 | 0 | 0 | 0 | 0 | | | | | | |
| 3765 | 04424 | 03764 | 1 | | 0 | 0 | 0 | 0 | 0 | 0 | 0 | 0 | 0 | 0 | 0 | 1 | 0 | 0 | 0 | 0 | 0 | 0 | 0 | 0 | 0 | 0 | 0 | 0 | 0 | | | | | | |

Table 105

| | A | B | C | E | G | I | K | M | O | Q | S | U | W | Y | AA | AC | AE | AG | AI | AK | AM | AO | AQ | AS | AU | AW | AY | BA | BC | BE | BF | BG | BH | BI | BK |
|---|---|---|---|---|---|---|---|---|---|---|---|---|---|---|---|---|---|---|---|---|---|---|---|---|---|---|---|---|---|---|---|---|---|---|---|
| 3766 | 04425 | 03765 | 5 | | 0 | 0 | 0 | 0 | 0 | 0 | 0 | 0 | 0 | 0 | 0 | 1 | 0 | 1 | 0 | 0 | 0 | 0 | 3 | 0 | 0 | 0 | 0 | 0 | 0 | | | | | | |
| 3767 | 04426 | 03766 | 2 | | 0 | 0 | 0 | 0 | 0 | 0 | 0 | 0 | 0 | 0 | 0 | 2 | 0 | 0 | 0 | 0 | 0 | 0 | 0 | 0 | 0 | 0 | 0 | 0 | 0 | | | | | | |
| 3768 | 04427 | 03767 | 1 | | 0 | 0 | 0 | 0 | 0 | 0 | 0 | 0 | 0 | 0 | 0 | 1 | 0 | 0 | 0 | 0 | 0 | 0 | 0 | 0 | 0 | 0 | 0 | 0 | 0 | | | | | | |
| 3769 | 04428 | 03768 | 1 | | 0 | 0 | 0 | 0 | 0 | 0 | 0 | 0 | 0 | 0 | 0 | 1 | 0 | 0 | 0 | 0 | 0 | 0 | 0 | 0 | 0 | 0 | 0 | 0 | 0 | | | | | | |
| 3770 | 04429 | 03769 | 1 | | 0 | 0 | 0 | 0 | 0 | 0 | 0 | 0 | 0 | 0 | 0 | 1 | 0 | 0 | 0 | 0 | 0 | 0 | 0 | 0 | 0 | 0 | 0 | 0 | 0 | | | | | | |
| 3771 | 04430 | 03770 | 1 | | 0 | 0 | 0 | 0 | 0 | 0 | 0 | 0 | 0 | 0 | 0 | 1 | 0 | 0 | 0 | 0 | 0 | 0 | 0 | 0 | 0 | 0 | 0 | 0 | 0 | | | | | | |
| 3772 | 04431 | 03771 | 3 | | 0 | 0 | 0 | 0 | 0 | 0 | 0 | 0 | 0 | 0 | 0 | 1 | 0 | 0 | 0 | 0 | 0 | 0 | 0 | 0 | 0 | 0 | 0 | 2 | 0 | | | | | | |
| 3773 | 04432 | 03772 | 1 | | 0 | 0 | 0 | 0 | 0 | 0 | 0 | 0 | 0 | 0 | 0 | 1 | 0 | 0 | 0 | 0 | 0 | 0 | 0 | 0 | 0 | 0 | 0 | 0 | 0 | | | | | | |
| 3774 | 04433 | 03773 | 1 | | 0 | 0 | 0 | 0 | 0 | 0 | 0 | 0 | 0 | 0 | 0 | 1 | 0 | 0 | 0 | 0 | 0 | 0 | 0 | 0 | 0 | 0 | 0 | 0 | 0 | | | | | | |
| 3775 | 04434 | 03774 | 1 | | 0 | 0 | 0 | 0 | 0 | 0 | 0 | 0 | 0 | 0 | 0 | 1 | 0 | 0 | 0 | 0 | 0 | 0 | 0 | 0 | 0 | 0 | 0 | 0 | 0 | | | | | | |
| 3776 | 04435 | 03775 | 1 | | 0 | 0 | 0 | 0 | 0 | 0 | 0 | 0 | 0 | 0 | 0 | 1 | 0 | 0 | 0 | 0 | 0 | 0 | 0 | 0 | 0 | 0 | 0 | 0 | 0 | | | | | | |
| 3777 | 04436 | 03776 | 2 | | 0 | 0 | 0 | 0 | 0 | 0 | 0 | 0 | 0 | 0 | 0 | 1 | 1 | 0 | 0 | 0 | 0 | 0 | 0 | 0 | 0 | 0 | 0 | 0 | 0 | | | | | | |
| 3778 | 04437 | 03777 | 1 | | 0 | 0 | 0 | 0 | 0 | 0 | 0 | 0 | 0 | 0 | 0 | 1 | 0 | 0 | 0 | 0 | 0 | 0 | 0 | 0 | 0 | 0 | 0 | 0 | 0 | | | | | | |
| 3779 | 04439 | 03778 | 2 | | 0 | 0 | 0 | 0 | 0 | 0 | 0 | 0 | 0 | 0 | 0 | 1 | 0 | 0 | 0 | 0 | 0 | 0 | 0 | 0 | 0 | 0 | 1 | 0 | 0 | | | | | | |
| 3780 | 04440 | 03779 | 3 | | 0 | 0 | 0 | 0 | 0 | 0 | 0 | 0 | 0 | 0 | 0 | 1 | 0 | 1 | 0 | 0 | 0 | 0 | 0 | 0 | 0 | 0 | 0 | 1 | 0 | | | | | | |
| 3781 | 04441 | 03780 | 1 | | 0 | 0 | 0 | 0 | 0 | 0 | 0 | 0 | 0 | 0 | 0 | 1 | 0 | 0 | 0 | 0 | 0 | 0 | 0 | 0 | 0 | 0 | 0 | 0 | 0 | J03824 | 97.9 | 141 | 1 | 1136 | 1279 |
| 3782 | 04442 | 03781 | 1 | | 0 | 0 | 0 | 0 | 0 | 0 | 0 | 0 | 0 | 0 | 0 | 1 | 0 | 0 | 0 | 0 | 0 | 0 | 0 | 0 | 0 | 0 | 0 | 0 | 0 | | | | | | |
| 3783 | 04443 | 03782 | 1 | | 0 | 0 | 0 | 0 | 0 | 0 | 0 | 0 | 0 | 0 | 0 | 1 | 0 | 0 | 0 | 0 | 0 | 0 | 0 | 0 | 0 | 0 | 0 | 0 | 0 | | | | | | |
| 3784 | 04445 | 03783 | 2 | | 0 | 0 | 0 | 0 | 0 | 0 | 0 | 0 | 0 | 0 | 0 | 1 | 0 | 0 | 0 | 1 | 0 | 0 | 0 | 0 | 0 | 0 | 0 | 0 | 0 | J05243 | 98.5 | 68 | 1 | 7712 | 7787 |
| 3785 | 04446 | 03784 | 1 | | 0 | 0 | 0 | 0 | 0 | 0 | 0 | 0 | 0 | 0 | 0 | 1 | 0 | 0 | 0 | 0 | 0 | 0 | 0 | 0 | 0 | 0 | 0 | 0 | 0 | | | | | | |
| 3786 | 04447 | 03785 | 1 | | 0 | 0 | 0 | 0 | 0 | 0 | 0 | 0 | 0 | 0 | 0 | 1 | 0 | 0 | 0 | 0 | 0 | 0 | 0 | 0 | 0 | 0 | 0 | 0 | 0 | | | | | | |
| 3787 | 04448 | 03786 | 1 | | 0 | 0 | 0 | 0 | 0 | 0 | 0 | 0 | 0 | 0 | 0 | 1 | 0 | 0 | 0 | 0 | 0 | 0 | 0 | 0 | 0 | 0 | 0 | 0 | 0 | | | | | | |
| 3788 | 04449 | 03787 | 1 | | 0 | 0 | 0 | 0 | 0 | 0 | 0 | 0 | 0 | 0 | 0 | 1 | 0 | 0 | 0 | 0 | 0 | 0 | 0 | 0 | 0 | 0 | 0 | 0 | 0 | | | | | | |
| 3789 | 04450 | 03788 | 1 | | 0 | 0 | 0 | 0 | 0 | 0 | 0 | 0 | 0 | 0 | 0 | 1 | 0 | 0 | 0 | 0 | 0 | 0 | 0 | 0 | 0 | 0 | 0 | 0 | 0 | | | | | | |
| 3790 | 04452 | 03789 | 3 | | 0 | 0 | 0 | 0 | 0 | 0 | 0 | 0 | 0 | 0 | 0 | 2 | 0 | 0 | 0 | 0 | 0 | 0 | 0 | 0 | 1 | 0 | 0 | 0 | 0 | | | | | | |
| 3791 | 04453 | 03790 | 1 | | 0 | 0 | 0 | 0 | 0 | 0 | 0 | 0 | 0 | 0 | 0 | 1 | 0 | 0 | 0 | 0 | 0 | 0 | 0 | 0 | 0 | 0 | 0 | 0 | 0 | | | | | | |
| 3792 | 04454 | 03791 | 3 | | 0 | 0 | 0 | 0 | 0 | 0 | 0 | 0 | 0 | 0 | 0 | 1 | 0 | 0 | 0 | 0 | 2 | 0 | 0 | 0 | 0 | 0 | 0 | 0 | 0 | | | | | | |
| 3793 | 04455 | 03792 | 1 | | 0 | 0 | 0 | 0 | 0 | 0 | 0 | 0 | 0 | 0 | 0 | 1 | 0 | 0 | 0 | 0 | 0 | 0 | 0 | 0 | 0 | 0 | 0 | 0 | 0 | | | | | | |
| 3794 | 04456 | 03793 | 1 | | 0 | 0 | 0 | 0 | 0 | 0 | 0 | 0 | 0 | 0 | 0 | 1 | 0 | 0 | 0 | 0 | 0 | 0 | 0 | 0 | 0 | 0 | 0 | 0 | 0 | | | | | | |
| 3795 | 04457 | 03794 | 1 | | 0 | 0 | 0 | 0 | 0 | 0 | 0 | 0 | 0 | 0 | 0 | 1 | 0 | 0 | 0 | 0 | 0 | 0 | 0 | 0 | 0 | 0 | 0 | 0 | 0 | | | | | | |
| 3796 | 04460 | 03795 | 1 | | 0 | 0 | 0 | 0 | 0 | 0 | 0 | 0 | 0 | 0 | 0 | 1 | 0 | 0 | 0 | 0 | 0 | 0 | 0 | 0 | 0 | 0 | 0 | 0 | 0 | | | | | | |
| 3797 | 04462 | 03796 | 3 | | 0 | 0 | 0 | 0 | 0 | 0 | 0 | 0 | 0 | 0 | 0 | 1 | 1 | 0 | 0 | 1 | 0 | 0 | 0 | 0 | 0 | 0 | 0 | 0 | 0 | X04526 | 100 | 125 | 1 | 1493 | 3088 |
| 3798 | 04463 | 03797 | 3 | | 0 | 0 | 0 | 0 | 0 | 0 | 0 | 0 | 0 | 0 | 0 | 2 | 0 | 0 | 0 | 0 | 0 | 0 | 0 | 1 | 0 | 0 | 0 | 0 | 0 | | | | | | |
| 3799 | 04464 | 03798 | 8 | | 0 | 0 | 0 | 0 | 0 | 0 | 0 | 0 | 0 | 0 | 0 | 1 | 3 | 1 | 0 | 0 | 0 | 0 | 1 | 2 | 0 | 0 | 0 | 0 | 0 | | | | | | |
| 3800 | 04465 | 03799 | 1 | | 0 | 0 | 0 | 0 | 0 | 0 | 0 | 0 | 0 | 0 | 0 | 1 | 0 | 0 | 0 | 0 | 0 | 0 | 0 | 0 | 0 | 0 | 0 | 0 | 0 | | | | | | |
| 3801 | 04466 | 03800 | 1 | | 0 | 0 | 0 | 0 | 0 | 0 | 0 | 0 | 0 | 0 | 0 | 1 | 0 | 0 | 0 | 0 | 0 | 0 | 0 | 0 | 0 | 0 | 0 | 0 | 0 | | | | | | |

Table 106

| | A | B | C | E | G | I | K | M | O | Q | S | U | W | Y | AA | AC | AE | AG | AI | AK | AM | AO | AQ | AS | AU | AW | AY | BA | BC | BE | BF | BG | BH | BI | BK |
|---|---|---|---|---|---|---|---|---|---|---|---|---|---|---|---|---|---|---|---|---|---|---|---|---|---|---|---|---|---|---|---|---|---|---|---|
| 3802 | 04467 | 03801 | 1 | | 0 | 0 | 0 | 0 | 0 | 0 | 0 | 0 | 0 | 0 | 0 | 1 | 0 | 0 | 0 | 0 | 0 | 0 | 0 | 0 | 0 | 0 | 0 | 0 | 0 | | | | | | |
| 3803 | 04468 | 03802 | 1 | | 0 | 0 | 0 | 0 | 0 | 0 | 0 | 0 | 0 | 0 | 0 | 1 | 0 | 0 | 0 | 0 | 0 | 0 | 0 | 0 | 0 | 0 | 0 | 0 | 0 | | | | | | |
| 3804 | 04469 | 03803 | 1 | | 0 | 0 | 0 | 0 | 0 | 0 | 0 | 0 | 0 | 0 | 0 | 1 | 0 | 0 | 0 | 0 | 0 | 0 | 0 | 0 | 0 | 0 | 0 | 0 | 0 | | | | | | |
| 3805 | 04470 | 03804 | 1 | | 0 | 0 | 0 | 0 | 0 | 0 | 0 | 0 | 0 | 0 | 0 | 1 | 0 | 0 | 0 | 0 | 0 | 0 | 0 | 0 | 0 | 0 | 0 | 0 | 0 | | | | | | |
| 3806 | 04471 | 03805 | 2 | | 0 | 0 | 0 | 0 | 0 | 0 | 0 | 0 | 0 | 0 | 0 | 2 | 0 | 0 | 0 | 0 | 0 | 0 | 0 | 0 | 0 | 0 | 0 | 0 | 0 | | | | | | |
| 3807 | 04472 | 03806 | 1 | | 0 | 0 | 0 | 0 | 0 | 0 | 0 | 0 | 0 | 0 | 0 | 1 | 0 | 0 | 0 | 0 | 0 | 0 | 0 | 0 | 0 | 0 | 0 | 0 | 0 | M94046 | 94.7 | 114 | 1 | 1932 | 2389 |
| 3808 | 04473 | 03807 | 1 | | 0 | 0 | 0 | 0 | 0 | 0 | 0 | 0 | 0 | 0 | 0 | 1 | 0 | 0 | 0 | 0 | 0 | 0 | 0 | 0 | 0 | 0 | 0 | 0 | 0 | | | | | | |
| 3809 | 04474 | 03808 | 1 | | 0 | 0 | 0 | 0 | 0 | 0 | 0 | 0 | 0 | 0 | 0 | 1 | 0 | 0 | 0 | 0 | 0 | 0 | 0 | 0 | 0 | 0 | 0 | 0 | 0 | | | | | | |
| 3810 | 04476 | 03809 | 1 | | 0 | 0 | 0 | 0 | 0 | 0 | 0 | 0 | 0 | 0 | 0 | 1 | 0 | 0 | 0 | 0 | 0 | 0 | 0 | 0 | 0 | 0 | 0 | 0 | 0 | | | | | | |
| 3811 | 04478 | 03810 | 1 | | 0 | 0 | 0 | 0 | 0 | 0 | 0 | 0 | 0 | 0 | 0 | 1 | 0 | 0 | 0 | 0 | 0 | 0 | 0 | 0 | 0 | 0 | 0 | 0 | 0 | | | | | | |
| 3812 | 04482 | 03811 | 2 | | 0 | 0 | 0 | 0 | 0 | 0 | 0 | 0 | 0 | 0 | 0 | 1 | 0 | 0 | 1 | 0 | 0 | 0 | 0 | 0 | 0 | 0 | 0 | 0 | 0 | | | | | | |
| 3813 | 04483 | 03812 | 6 | | 0 | 0 | 0 | 0 | 0 | 0 | 0 | 0 | 0 | 0 | 0 | 1 | 0 | 0 | 3 | 1 | 0 | 1 | 0 | 0 | 0 | 0 | 0 | 0 | 0 | | | | | | |
| 3814 | 04484 | 03813 | 1 | | 0 | 0 | 0 | 0 | 0 | 0 | 0 | 0 | 0 | 0 | 0 | 1 | 0 | 0 | 0 | 0 | 0 | 0 | 0 | 0 | 0 | 0 | 0 | 0 | 0 | | | | | | |
| 3815 | 04485 | 03814 | 1 | | 0 | 0 | 0 | 0 | 0 | 0 | 0 | 0 | 0 | 0 | 0 | 1 | 0 | 0 | 0 | 0 | 0 | 0 | 0 | 0 | 0 | 0 | 0 | 0 | 0 | | | | | | |
| 3816 | 04486 | 03815 | 1 | | 0 | 0 | 0 | 0 | 0 | 0 | 0 | 0 | 0 | 0 | 0 | 1 | 0 | 0 | 0 | 0 | 0 | 0 | 0 | 0 | 0 | 0 | 0 | 0 | 0 | | | | | | |
| 3817 | 04487 | 03816 | 1 | | 0 | 0 | 0 | 0 | 0 | 0 | 0 | 0 | 0 | 0 | 0 | 1 | 0 | 0 | 0 | 0 | 0 | 0 | 0 | 0 | 0 | 0 | 0 | 0 | 0 | | | | | | |
| 3818 | 04488 | 03817 | 1 | | 0 | 0 | 0 | 0 | 0 | 0 | 0 | 0 | 0 | 0 | 0 | 1 | 0 | 0 | 0 | 0 | 0 | 0 | 0 | 0 | 0 | 0 | 0 | 0 | 0 | X73459 | 97 | 99 | 1 | 217 | 721 |
| 3819 | 04489 | 03818 | 1 | | 0 | 0 | 0 | 0 | 0 | 0 | 0 | 0 | 0 | 0 | 0 | 1 | 0 | 0 | 0 | 0 | 0 | 0 | 0 | 0 | 0 | 0 | 0 | 0 | 0 | | | | | | |
| 3820 | 04491 | 03819 | 1 | | 0 | 0 | 0 | 0 | 0 | 0 | 0 | 0 | 0 | 0 | 0 | 1 | 0 | 0 | 0 | 0 | 0 | 0 | 0 | 0 | 0 | 0 | 0 | 0 | 0 | | | | | | |
| 3821 | 04492 | 03820 | 1 | | 0 | 0 | 0 | 0 | 0 | 0 | 0 | 0 | 0 | 0 | 0 | 1 | 0 | 0 | 0 | 0 | 0 | 0 | 0 | 0 | 0 | 0 | 0 | 0 | 0 | | | | | | |
| 3822 | 04493 | 03821 | 3 | | 0 | 0 | 0 | 0 | 0 | 0 | 0 | 0 | 0 | 0 | 0 | 1 | 0 | 0 | 0 | 0 | 0 | 0 | 0 | 0 | 0 | 0 | 0 | 2 | 0 | | | | | | |
| 3823 | 04494 | 03822 | 1 | | 0 | 0 | 0 | 0 | 0 | 0 | 0 | 0 | 0 | 0 | 0 | 1 | 0 | 0 | 0 | 0 | 0 | 0 | 0 | 0 | 0 | 0 | 0 | 0 | 0 | | | | | | |
| 3824 | 04495 | 03823 | 1 | | 0 | 0 | 0 | 0 | 0 | 0 | 0 | 0 | 0 | 0 | 0 | 1 | 0 | 0 | 0 | 0 | 0 | 0 | 0 | 0 | 0 | 0 | 0 | 0 | 0 | | | | | | |
| 3825 | 04496 | 03824 | 1 | | 0 | 0 | 0 | 0 | 0 | 0 | 0 | 0 | 0 | 0 | 0 | 1 | 0 | 0 | 0 | 0 | 0 | 0 | 0 | 0 | 0 | 0 | 0 | 0 | 0 | | | | | | |
| 3826 | 04497 | 03825 | 1 | | 0 | 0 | 0 | 0 | 0 | 0 | 0 | 0 | 0 | 0 | 0 | 1 | 0 | 0 | 0 | 0 | 0 | 0 | 0 | 0 | 0 | 0 | 0 | 0 | 0 | | | | | | |
| 3827 | 04498 | 03826 | 1 | | 0 | 0 | 0 | 0 | 0 | 0 | 0 | 0 | 0 | 0 | 0 | 1 | 0 | 0 | 0 | 0 | 0 | 0 | 0 | 0 | 0 | 0 | 0 | 0 | 0 | | | | | | |
| 3828 | 04499 | 03827 | 2 | | 0 | 0 | 0 | 0 | 0 | 0 | 0 | 0 | 0 | 0 | 1 | 1 | 0 | 0 | 0 | 0 | 0 | 0 | 0 | 0 | 0 | 0 | 0 | 0 | 0 | | | | | | |
| 3829 | 04500 | 03828 | 1 | | 0 | 0 | 0 | 0 | 0 | 0 | 0 | 0 | 0 | 0 | 0 | 1 | 0 | 0 | 0 | 0 | 0 | 0 | 0 | 0 | 0 | 0 | 0 | 0 | 0 | D00860 | 100 | 83 | 1 | 1974 | 2075 |
| 3830 | 04501 | 03829 | 1 | | 0 | 0 | 0 | 0 | 0 | 0 | 0 | 0 | 0 | 0 | 0 | 1 | 0 | 0 | 0 | 0 | 0 | 0 | 0 | 0 | 0 | 0 | 0 | 0 | 0 | | | | | | |
| 3831 | 04502 | 03830 | 3 | | 0 | 0 | 0 | 0 | 0 | 0 | 0 | 0 | 0 | 0 | 0 | 1 | 0 | 0 | 0 | 0 | 0 | 0 | 1 | 1 | 0 | 0 | 0 | 0 | 0 | | | | | | |
| 3832 | 04503 | 03831 | 1 | | 0 | 0 | 0 | 0 | 0 | 0 | 0 | 0 | 0 | 0 | 0 | 1 | 0 | 0 | 0 | 0 | 0 | 0 | 0 | 0 | 0 | 0 | 0 | 0 | 0 | | | | | | |
| 3833 | 04504 | 03832 | 1 | | 0 | 0 | 0 | 0 | 0 | 0 | 0 | 0 | 0 | 0 | 0 | 1 | 0 | 0 | 0 | 0 | 0 | 0 | 0 | 0 | 0 | 0 | 0 | 0 | 0 | | | | | | |
| 3834 | 04506 | 03833 | 5 | | 0 | 0 | 0 | 0 | 0 | 0 | 0 | 0 | 0 | 0 | 0 | 1 | 0 | 0 | 0 | 1 | 0 | 3 | 0 | 0 | 0 | 0 | 0 | 0 | 0 | | | | | | |
| 3835 | 04507 | 03834 | 1 | | 0 | 0 | 0 | 0 | 0 | 0 | 0 | 0 | 0 | 0 | 0 | 1 | 0 | 0 | 0 | 0 | 0 | 0 | 0 | 0 | 0 | 0 | 0 | 0 | 0 | | | | | | |
| 3836 | 04509 | 03835 | 1 | | 0 | 0 | 0 | 0 | 0 | 0 | 0 | 0 | 0 | 0 | 0 | 1 | 0 | 0 | 0 | 0 | 0 | 0 | 0 | 0 | 0 | 0 | 0 | 0 | 0 | | | | | | |
| 3837 | 04510 | 03836 | 1 | | 0 | 0 | 0 | 0 | 0 | 0 | 0 | 0 | 0 | 0 | 0 | 1 | 0 | 0 | 0 | 0 | 0 | 0 | 0 | 0 | 0 | 0 | 0 | 0 | 0 | | | | | | |

Table 107

| | A | B | C | E | G | I | K | M | O | Q | S | U | W | Y | AA | AC | AE | AG | AI | AK | AM | AO | AQ | AS | AU | AW | AY | BA | BC | BE | BF | BG | BH | BI | BK |
|---|---|---|---|---|---|---|---|---|---|---|---|---|---|---|---|---|---|---|---|---|---|---|---|---|---|---|---|---|---|---|---|---|---|---|---|
| 3838 | 04511 | 03837 | 2 | | 0 | 0 | 0 | 0 | 0 | 0 | 0 | 0 | 0 | 0 | 0 | 1 | 0 | 0 | 0 | 0 | 0 | 0 | 0 | 0 | 1 | 0 | 0 | 0 | 0 | | | | | | |
| 3839 | 04512 | 03838 | 1 | | 0 | 0 | 0 | 0 | 0 | 0 | 0 | 0 | 0 | 0 | 0 | 1 | 0 | 0 | 0 | 0 | 0 | 0 | 0 | 0 | 0 | 0 | 0 | 0 | 0 | | | | | | |
| 3840 | 04513 | 03839 | 2 | | 0 | 0 | 0 | 0 | 0 | 0 | 0 | 0 | 0 | 0 | 0 | 2 | 0 | 0 | 0 | 0 | 0 | 0 | 0 | 0 | 0 | 0 | 0 | 0 | 0 | | | | | | |
| 3841 | 04514 | 03840 | 3 | | 0 | 0 | 0 | 0 | 0 | 0 | 0 | 0 | 0 | 0 | 0 | 1 | 0 | 0 | 0 | 0 | 0 | 0 | 0 | 0 | 0 | 0 | 0 | 2 | 0 | | | | | | |
| 3842 | 04515 | 03841 | 1 | | 0 | 0 | 0 | 0 | 0 | 0 | 0 | 0 | 0 | 0 | 0 | 1 | 0 | 0 | 0 | 0 | 0 | 0 | 0 | 0 | 0 | 0 | 0 | 0 | 0 | | | | | | |
| 3843 | 04516 | 03842 | 1 | | 0 | 0 | 0 | 0 | 0 | 0 | 0 | 0 | 0 | 0 | 0 | 1 | 0 | 0 | 0 | 0 | 0 | 0 | 0 | 0 | 0 | 0 | 0 | 0 | 0 | | | | | | |
| 3844 | 04517 | 03843 | 1 | | 0 | 0 | 0 | 0 | 0 | 0 | 0 | 0 | 0 | 0 | 0 | 1 | 0 | 0 | 0 | 0 | 0 | 0 | 0 | 0 | 0 | 0 | 0 | 0 | 0 | | | | | | |
| 3845 | 04518 | 03844 | 3 | | 0 | 0 | 0 | 0 | 0 | 0 | 0 | 0 | 0 | 0 | 0 | 1 | 0 | 0 | 0 | 1 | 0 | 0 | 1 | 0 | 0 | 0 | 0 | 0 | 0 | | | | | | |
| 3846 | 04519 | 03845 | 1 | | 0 | 0 | 0 | 0 | 0 | 0 | 0 | 0 | 0 | 0 | 0 | 1 | 0 | 0 | 0 | 0 | 0 | 0 | 0 | 0 | 0 | 0 | 0 | 0 | 0 | | | | | | |
| 3847 | 04520 | 03846 | 2 | | 0 | 0 | 0 | 0 | 0 | 0 | 0 | 0 | 0 | 0 | 0 | 1 | 0 | 0 | 0 | 0 | 0 | 0 | 0 | 0 | 0 | 0 | 0 | 1 | 0 | | | | | | |
| 3848 | 04521 | 03847 | 2 | | 0 | 0 | 0 | 0 | 0 | 0 | 0 | 0 | 0 | 0 | 0 | 2 | 0 | 0 | 0 | 0 | 0 | 0 | 0 | 0 | 0 | 0 | 0 | 0 | 0 | | | | | | |
| 3849 | 04522 | 03848 | 1 | | 0 | 0 | 0 | 0 | 0 | 0 | 0 | 0 | 0 | 0 | 0 | 1 | 0 | 0 | 0 | 0 | 0 | 0 | 0 | 0 | 0 | 0 | 0 | 0 | 0 | | | | | | |
| 3850 | 04523 | 03849 | 1 | | 0 | 0 | 0 | 0 | 0 | 0 | 0 | 0 | 0 | 0 | 0 | 1 | 0 | 0 | 0 | 0 | 0 | 0 | 0 | 0 | 0 | 0 | 0 | 0 | 0 | L21181 | 95.7 | 69 | 1 | 1601 | 1672 |
| 3851 | 04525 | 03850 | 1 | | 0 | 0 | 0 | 0 | 0 | 0 | 0 | 0 | 0 | 0 | 0 | 1 | 0 | 0 | 0 | 0 | 0 | 0 | 0 | 0 | 0 | 0 | 0 | 0 | 0 | | | | | | |
| 3852 | 04527 | 03851 | 1 | | 0 | 0 | 0 | 0 | 0 | 0 | 0 | 0 | 0 | 0 | 0 | 1 | 0 | 0 | 0 | 0 | 0 | 0 | 0 | 0 | 0 | 0 | 0 | 0 | 0 | | | | | | |
| 3853 | 04528 | 03852 | 1 | | 0 | 0 | 0 | 0 | 0 | 0 | 0 | 0 | 0 | 0 | 0 | 1 | 0 | 0 | 0 | 0 | 0 | 0 | 0 | 0 | 0 | 0 | 0 | 0 | 0 | | | | | | |
| 3854 | 04529 | 03853 | 1 | | 0 | 0 | 0 | 0 | 0 | 0 | 0 | 0 | 0 | 0 | 0 | 1 | 0 | 0 | 0 | 0 | 0 | 0 | 0 | 0 | 0 | 0 | 0 | 0 | 0 | | | | | | |
| 3855 | 04530 | 03854 | 1 | | 0 | 0 | 0 | 0 | 0 | 0 | 0 | 0 | 0 | 0 | 0 | 1 | 0 | 0 | 0 | 0 | 0 | 0 | 0 | 0 | 0 | 0 | 0 | 0 | 0 | | | | | | |
| 3856 | 04531 | 03855 | 2 | | 0 | 0 | 0 | 0 | 0 | 0 | 0 | 0 | 0 | 0 | 0 | 1 | 1 | 0 | 0 | 0 | 0 | 0 | 0 | 0 | 0 | 0 | 0 | 0 | 0 | | | | | | |
| 3857 | 04532 | 03856 | 3 | | 0 | 0 | 0 | 0 | 0 | 0 | 0 | 0 | 0 | 0 | 0 | 1 | 0 | 0 | 1 | 0 | 0 | 0 | 0 | 1 | 0 | 0 | 0 | 0 | 0 | | | | | | |
| 3858 | 04533 | 03857 | 4 | | 0 | 0 | 0 | 0 | 0 | 0 | 0 | 0 | 0 | 0 | 0 | 1 | 0 | 0 | 0 | 0 | 0 | 0 | 0 | 0 | 0 | 0 | 0 | 2 | 1 | | | | | | |
| 3859 | 04534 | 03858 | 2 | | 0 | 0 | 0 | 0 | 0 | 0 | 0 | 0 | 0 | 0 | 0 | 1 | 0 | 0 | 0 | 0 | 0 | 0 | 0 | 1 | 0 | 0 | 0 | 0 | 0 | | | | | | |
| 3860 | 04535 | 03859 | 1 | | 0 | 0 | 0 | 0 | 0 | 0 | 0 | 0 | 0 | 0 | 0 | 1 | 0 | 0 | 0 | 0 | 0 | 0 | 0 | 0 | 0 | 0 | 0 | 0 | 0 | | | | | | |
| 3861 | 04536 | 03860 | 1 | | 0 | 0 | 0 | 0 | 0 | 0 | 0 | 0 | 0 | 0 | 0 | 1 | 0 | 0 | 0 | 0 | 0 | 0 | 0 | 0 | 0 | 0 | 0 | 0 | 0 | | | | | | |
| 3862 | 04537 | 03861 | 1 | | 0 | 0 | 0 | 0 | 0 | 0 | 0 | 0 | 0 | 0 | 0 | 1 | 0 | 0 | 0 | 0 | 0 | 0 | 0 | 0 | 0 | 0 | 0 | 0 | 0 | | | | | | |
| 3863 | 04538 | 03862 | 1 | | 0 | 0 | 0 | 0 | 0 | 0 | 0 | 0 | 0 | 0 | 0 | 1 | 0 | 0 | 0 | 0 | 0 | 0 | 0 | 0 | 0 | 0 | 0 | 0 | 0 | | | | | | |
| 3864 | 04539 | 03863 | 1 | | 0 | 0 | 0 | 0 | 0 | 0 | 0 | 0 | 0 | 0 | 0 | 1 | 0 | 0 | 0 | 0 | 0 | 0 | 0 | 0 | 0 | 0 | 0 | 0 | 0 | | | | | | |
| 3865 | 04541 | 03864 | 2 | | 0 | 0 | 0 | 0 | 0 | 0 | 0 | 0 | 0 | 0 | 0 | 1 | 0 | 0 | 0 | 0 | 0 | 0 | 1 | 0 | 0 | 0 | 0 | 0 | 0 | | | | | | |
| 3866 | 04542 | 03865 | 1 | | 0 | 0 | 0 | 0 | 0 | 0 | 0 | 0 | 0 | 0 | 0 | 1 | 0 | 0 | 0 | 0 | 0 | 0 | 0 | 0 | 0 | 0 | 0 | 0 | 0 | | | | | | |
| 3867 | 04543 | 03866 | 2 | | 0 | 0 | 0 | 0 | 0 | 0 | 0 | 0 | 0 | 0 | 0 | 1 | 0 | 0 | 0 | 0 | 0 | 0 | 1 | 0 | 0 | 0 | 0 | 0 | 0 | | | | | | |
| 3868 | 04546 | 03867 | 1 | | 0 | 0 | 0 | 0 | 0 | 0 | 0 | 0 | 0 | 0 | 0 | 1 | 0 | 0 | 0 | 0 | 0 | 0 | 0 | 0 | 0 | 0 | 0 | 0 | 0 | | | | | | |
| 3869 | 04547 | 03868 | 1 | | 0 | 0 | 0 | 0 | 0 | 0 | 0 | 0 | 0 | 0 | 0 | 1 | 0 | 0 | 0 | 0 | 0 | 0 | 0 | 0 | 0 | 0 | 0 | 0 | 0 | | | | | | |
| 3870 | 04548 | 03869 | 2 | | 0 | 0 | 0 | 0 | 0 | 0 | 0 | 0 | 0 | 0 | 0 | 1 | 1 | 0 | 0 | 0 | 0 | 0 | 0 | 0 | 0 | 0 | 0 | 0 | 0 | | | | | | |
| 3871 | 04552 | 03870 | 1 | | 0 | 0 | 0 | 0 | 0 | 0 | 0 | 0 | 0 | 0 | 0 | 1 | 0 | 0 | 0 | 0 | 0 | 0 | 0 | 0 | 0 | 0 | 0 | 0 | 0 | | | | | | |
| 3872 | 04556 | 03871 | 1 | | 0 | 0 | 0 | 0 | 0 | 0 | 0 | 0 | 0 | 0 | 0 | 1 | 0 | 0 | 0 | 0 | 0 | 0 | 0 | 0 | 0 | 0 | 0 | 0 | 0 | | | | | | |
| 3873 | 04557 | 03872 | 1 | | 0 | 0 | 0 | 0 | 0 | 0 | 0 | 0 | 0 | 0 | 0 | 1 | 0 | 0 | 0 | 0 | 0 | 0 | 0 | 0 | 0 | 0 | 0 | 0 | 0 | | | | | | |

Table 108

| | A | B | C | E | G | I | K | M | O | Q | S | U | W | Y | AA | AC | AE | AG | AI | AK | AM | AO | AQ | AS | AU | AW | AY | BA | BC | BE | BF | BG | BH | BI | BK |
|---|---|---|---|---|---|---|---|---|---|---|---|---|---|---|---|---|---|---|---|---|---|---|---|---|---|---|---|---|---|---|---|---|---|---|---|
| 3874 | 04597 | 03873 | 3 | | 0 | 0 | 0 | 0 | 0 | 0 | 0 | 0 | 0 | 0 | 0 | 0 | 3 | 0 | 0 | 0 | 0 | 0 | 0 | 0 | 0 | 0 | 0 | 0 | 0 | | | | | | |
| 3875 | 04598 | 03874 | 3 | | 0 | 0 | 0 | 0 | 0 | 0 | 0 | 0 | 0 | 0 | 0 | 0 | 1 | 0 | 1 | 0 | 0 | 0 | 0 | 0 | 0 | 0 | 0 | 1 | 0 | | | | | | |
| 3876 | 04599 | 03875 | 1 | | 0 | 0 | 0 | 0 | 0 | 0 | 0 | 0 | 0 | 0 | 0 | 0 | 1 | 0 | 0 | 0 | 0 | 0 | 0 | 0 | 0 | 0 | 0 | 0 | 0 | | | | | | |
| 3877 | 04600 | 03876 | 1 | | 0 | 0 | 0 | 0 | 0 | 0 | 0 | 0 | 0 | 0 | 0 | 0 | 1 | 0 | 0 | 0 | 0 | 0 | 0 | 0 | 0 | 0 | 0 | 0 | 0 | | | | | | |
| 3878 | 04601 | 03877 | 1 | | 0 | 0 | 0 | 0 | 0 | 0 | 0 | 0 | 0 | 0 | 0 | 0 | 1 | 0 | 0 | 0 | 0 | 0 | 0 | 0 | 0 | 0 | 0 | 0 | 0 | | | | | | |
| 3879 | 04602 | 03878 | 3 | | 0 | 0 | 0 | 0 | 0 | 0 | 0 | 0 | 0 | 0 | 0 | 0 | 1 | 0 | 0 | 0 | 0 | 0 | 0 | 0 | 0 | 0 | 0 | 2 | 0 | | | | | | |
| 3880 | 04603 | 03879 | 2 | | 0 | 0 | 0 | 0 | 0 | 0 | 0 | 0 | 0 | 0 | 0 | 0 | 2 | 0 | 0 | 0 | 0 | 0 | 0 | 0 | 0 | 0 | 0 | 0 | 0 | | | | | | |
| 3881 | 04604 | 03880 | 1 | | 0 | 0 | 0 | 0 | 0 | 0 | 0 | 0 | 0 | 0 | 0 | 0 | 1 | 0 | 0 | 0 | 0 | 0 | 0 | 0 | 0 | 0 | 0 | 0 | 0 | | | | | | |
| 3882 | 04605 | 03881 | 2 | | 0 | 0 | 0 | 0 | 0 | 0 | 0 | 0 | 0 | 0 | 0 | 0 | 2 | 0 | 0 | 0 | 0 | 0 | 0 | 0 | 0 | 0 | 0 | 0 | 0 | | | | | | |
| 3883 | 04606 | 03882 | 1 | | 0 | 0 | 0 | 0 | 0 | 0 | 0 | 0 | 0 | 0 | 0 | 0 | 1 | 0 | 0 | 0 | 0 | 0 | 0 | 0 | 0 | 0 | 0 | 0 | 0 | | | | | | |
| 3884 | 04607 | 03883 | 2 | | 0 | 0 | 0 | 0 | 0 | 0 | 0 | 0 | 0 | 0 | 0 | 0 | 1 | 0 | 0 | 0 | 0 | 1 | 0 | 0 | 0 | 0 | 0 | 0 | 0 | | | | | | |
| 3885 | 04608 | 03884 | 1 | | 0 | 0 | 0 | 0 | 0 | 0 | 0 | 0 | 0 | 0 | 0 | 0 | 1 | 0 | 0 | 0 | 0 | 0 | 0 | 0 | 0 | 0 | 0 | 0 | 0 | | | | | | |
| 3886 | 04609 | 03885 | 1 | | 0 | 0 | 0 | 0 | 0 | 0 | 0 | 0 | 0 | 0 | 0 | 0 | 1 | 0 | 0 | 0 | 0 | 0 | 0 | 0 | 0 | 0 | 0 | 0 | 0 | X12453 | 98.6 | 362 | 1 | 1217 | 1582 |
| 3887 | 04610 | 03886 | 2 | | 0 | 0 | 0 | 0 | 0 | 0 | 0 | 0 | 0 | 0 | 0 | 0 | 1 | 0 | 0 | 0 | 0 | 0 | 0 | 0 | 0 | 0 | 0 | 1 | 0 | | | | | | |
| 3888 | 04611 | 03887 | 2 | | 0 | 0 | 0 | 0 | 0 | 0 | 0 | 0 | 0 | 0 | 0 | 0 | 1 | 0 | 0 | 0 | 0 | 0 | 1 | 0 | 0 | 0 | 0 | 0 | 0 | | | | | | |
| 3889 | 04612 | 03888 | 1 | | 0 | 0 | 0 | 0 | 0 | 0 | 0 | 0 | 0 | 0 | 0 | 0 | 1 | 0 | 0 | 0 | 0 | 0 | 0 | 0 | 0 | 0 | 0 | 0 | 0 | | | | | | |
| 3890 | 04613 | 03889 | 3 | | 0 | 0 | 0 | 0 | 0 | 0 | 0 | 0 | 0 | 0 | 0 | 0 | 2 | 0 | 0 | 0 | 0 | 0 | 0 | 1 | 0 | 0 | 0 | 0 | 0 | | | | | | |
| 3891 | 04614 | 03890 | 1 | | 0 | 0 | 0 | 0 | 0 | 0 | 0 | 0 | 0 | 0 | 0 | 0 | 1 | 0 | 0 | 0 | 0 | 0 | 0 | 0 | 0 | 0 | 0 | 0 | 0 | | | | | | |
| 3892 | 04615 | 03891 | 1 | | 0 | 0 | 0 | 0 | 0 | 0 | 0 | 0 | 0 | 0 | 0 | 0 | 1 | 0 | 0 | 0 | 0 | 0 | 0 | 0 | 0 | 0 | 0 | 0 | 0 | | | | | | |
| 3893 | 04616 | 03892 | 2 | | 0 | 0 | 0 | 0 | 0 | 0 | 0 | 0 | 0 | 0 | 0 | 0 | 1 | 0 | 0 | 0 | 0 | 0 | 0 | 0 | 0 | 1 | 0 | 0 | 0 | | | | | | |
| 3894 | 04617 | 03893 | 1 | | 0 | 0 | 0 | 0 | 0 | 0 | 0 | 0 | 0 | 0 | 0 | 0 | 1 | 0 | 0 | 0 | 0 | 0 | 0 | 0 | 0 | 0 | 0 | 0 | 0 | | | | | | |
| 3895 | 04618 | 03894 | 1 | | 0 | 0 | 0 | 0 | 0 | 0 | 0 | 0 | 0 | 0 | 0 | 0 | 1 | 0 | 0 | 0 | 0 | 0 | 0 | 0 | 0 | 0 | 0 | 0 | 0 | | | | | | |
| 3896 | 04619 | 03895 | 1 | | 0 | 0 | 0 | 0 | 0 | 0 | 0 | 0 | 0 | 0 | 0 | 0 | 1 | 0 | 0 | 0 | 0 | 0 | 0 | 0 | 0 | 0 | 0 | 0 | 0 | | | | | | |
| 3897 | 04620 | 03896 | 1 | | 0 | 0 | 0 | 0 | 0 | 0 | 0 | 0 | 0 | 0 | 0 | 0 | 1 | 0 | 0 | 0 | 0 | 0 | 0 | 0 | 0 | 0 | 0 | 0 | 0 | | | | | | |
| 3898 | 04621 | 03897 | 1 | | 0 | 0 | 0 | 0 | 0 | 0 | 0 | 0 | 0 | 0 | 0 | 0 | 1 | 0 | 0 | 0 | 0 | 0 | 0 | 0 | 0 | 0 | 0 | 0 | 0 | | | | | | |
| 3899 | 04623 | 03898 | 1 | | 0 | 0 | 0 | 0 | 0 | 0 | 0 | 0 | 0 | 0 | 0 | 0 | 1 | 0 | 0 | 0 | 0 | 0 | 0 | 0 | 0 | 0 | 0 | 0 | 0 | | | | | | |
| 3900 | 04624 | 03899 | 6 | | 0 | 0 | 0 | 0 | 0 | 0 | 0 | 0 | 0 | 0 | 0 | 0 | 1 | 0 | 1 | 1 | 2 | 0 | 0 | 0 | 0 | 0 | 0 | 1 | 0 | | | | | | |
| 3901 | 04625 | 03900 | 1 | | 0 | 0 | 0 | 0 | 0 | 0 | 0 | 0 | 0 | 0 | 0 | 0 | 1 | 0 | 0 | 0 | 0 | 0 | 0 | 0 | 0 | 0 | 0 | 0 | 0 | | | | | | |
| 3902 | 04626 | 03901 | 4 | | 0 | 0 | 0 | 0 | 0 | 0 | 0 | 0 | 0 | 0 | 0 | 0 | 4 | 0 | 0 | 0 | 0 | 0 | 0 | 0 | 0 | 0 | 0 | 0 | 0 | S62027 | 97.4 | 229 | 1 | 159 | 408 |
| 3903 | 04627 | 03902 | 3 | | 0 | 0 | 0 | 0 | 0 | 0 | 0 | 0 | 0 | 0 | 0 | 0 | 1 | 0 | 0 | 0 | 0 | 0 | 2 | 0 | 0 | 0 | 0 | 0 | 0 | | | | | | |
| 3904 | 04628 | 03903 | 3 | | 0 | 0 | 0 | 0 | 0 | 0 | 0 | 0 | 0 | 0 | 0 | 0 | 1 | 0 | 0 | 0 | 0 | 0 | 0 | 1 | 0 | 1 | 0 | 0 | 0 | | | | | | |
| 3905 | 04629 | 03904 | 3 | | 0 | 0 | 0 | 0 | 0 | 0 | 0 | 0 | 0 | 0 | 0 | 0 | 3 | 0 | 0 | 0 | 0 | 0 | 0 | 0 | 0 | 0 | 0 | 0 | 0 | | | | | | |
| 3906 | 04630 | 03905 | 1 | | 0 | 0 | 0 | 0 | 0 | 0 | 0 | 0 | 0 | 0 | 0 | 0 | 1 | 0 | 0 | 0 | 0 | 0 | 0 | 0 | 0 | 0 | 0 | 0 | 0 | | | | | | |
| 3907 | 04631 | 03906 | 1 | | 0 | 0 | 0 | 0 | 0 | 0 | 0 | 0 | 0 | 0 | 0 | 0 | 1 | 0 | 0 | 0 | 0 | 0 | 0 | 0 | 0 | 0 | 0 | 0 | 0 | | | | | | |
| 3908 | 04632 | 03907 | 2 | | 0 | 0 | 0 | 0 | 0 | 0 | 0 | 0 | 0 | 0 | 0 | 0 | 1 | 0 | 0 | 0 | 0 | 0 | 1 | 0 | 0 | 0 | 0 | 0 | 0 | | | | | | |
| 3909 | 04633 | 03908 | 2 | | 0 | 0 | 0 | 0 | 0 | 0 | 0 | 0 | 0 | 0 | 0 | 0 | 1 | 0 | 0 | 0 | 0 | 0 | 1 | 0 | 0 | 0 | 0 | 0 | 0 | | | | | | |

Table 109

| | A | B | C | E | G | I | K | M | O | Q | S | U | W | Y | AA | AC | AE | AG | AI | AK | AM | AO | AQ | AS | AU | AW | AY | BA | BC | BE | BF | BG | BH | BI | BK |
|---|---|---|---|---|---|---|---|---|---|---|---|---|---|---|---|---|---|---|---|---|---|---|---|---|---|---|---|---|---|---|---|---|---|---|---|
| 3910 | 04634 | 03909 | 2 | | 0 | 0 | 0 | 0 | 0 | 0 | 0 | 0 | 0 | 0 | 0 | 0 | 1 | 0 | 0 | 0 | 0 | 0 | 0 | 0 | 0 | 0 | 0 | 1 | 0 | | | | | | |
| 3911 | 04635 | 03910 | 1 | | 0 | 0 | 0 | 0 | 0 | 0 | 0 | 0 | 0 | 0 | 0 | 0 | 1 | 0 | 0 | 0 | 0 | 0 | 0 | 0 | 0 | 0 | 0 | 0 | 0 | | | | | | |
| 3912 | 04636 | 03911 | 1 | | 0 | 0 | 0 | 0 | 0 | 0 | 0 | 0 | 0 | 0 | 0 | 0 | 1 | 0 | 0 | 0 | 0 | 0 | 0 | 0 | 0 | 0 | 0 | 0 | 0 | | | | | | |
| 3913 | 04637 | 03912 | 1 | | 0 | 0 | 0 | 0 | 0 | 0 | 0 | 0 | 0 | 0 | 0 | 0 | 1 | 0 | 0 | 0 | 0 | 0 | 0 | 0 | 0 | 0 | 0 | 0 | 0 | S62027 | 96.1 | 232 | 1 | 159 | 408 |
| 3914 | 04638 | 03913 | 1 | | 0 | 0 | 0 | 0 | 0 | 0 | 0 | 0 | 0 | 0 | 0 | 0 | 1 | 0 | 0 | 0 | 0 | 0 | 0 | 0 | 0 | 0 | 0 | 0 | 0 | | | | | | |
| 3915 | 04639 | 03914 | 2 | | 0 | 0 | 0 | 0 | 0 | 0 | 0 | 0 | 0 | 0 | 0 | 0 | 2 | 0 | 0 | 0 | 0 | 0 | 0 | 0 | 0 | 0 | 0 | 0 | 0 | | | | | | |
| 3916 | 04640 | 03915 | 1 | | 0 | 0 | 0 | 0 | 0 | 0 | 0 | 0 | 0 | 0 | 0 | 0 | 1 | 0 | 0 | 0 | 0 | 0 | 0 | 0 | 0 | 0 | 0 | 0 | 0 | | | | | | |
| 3917 | 04641 | 03916 | 2 | | 0 | 0 | 0 | 0 | 0 | 0 | 0 | 0 | 0 | 0 | 0 | 0 | 2 | 0 | 0 | 0 | 0 | 0 | 0 | 0 | 0 | 0 | 0 | 0 | 0 | | | | | | |
| 3918 | 04642 | 03917 | 3 | | 0 | 0 | 0 | 0 | 0 | 0 | 0 | 0 | 0 | 0 | 0 | 0 | 3 | 0 | 0 | 0 | 0 | 0 | 0 | 0 | 0 | 0 | 0 | 0 | 0 | | | | | | |
| 3919 | 04643 | 03918 | 1 | | 0 | 0 | 0 | 0 | 0 | 0 | 0 | 0 | 0 | 0 | 0 | 0 | 1 | 0 | 0 | 0 | 0 | 0 | 0 | 0 | 0 | 0 | 0 | 0 | 0 | | | | | | |
| 3920 | 04644 | 03919 | 1 | | 0 | 0 | 0 | 0 | 0 | 0 | 0 | 0 | 0 | 0 | 0 | 0 | 1 | 0 | 0 | 0 | 0 | 0 | 0 | 0 | 0 | 0 | 0 | 0 | 0 | | | | | | |
| 3921 | 04645 | 03920 | 1 | | 0 | 0 | 0 | 0 | 0 | 0 | 0 | 0 | 0 | 0 | 0 | 0 | 1 | 0 | 0 | 0 | 0 | 0 | 0 | 0 | 0 | 0 | 0 | 0 | 0 | | | | | | |
| 3922 | 04646 | 03921 | 2 | | 0 | 0 | 0 | 0 | 0 | 0 | 0 | 0 | 0 | 0 | 0 | 0 | 1 | 1 | 0 | 0 | 0 | 0 | 0 | 0 | 0 | 0 | 0 | 0 | 0 | | | | | | |
| 3923 | 04647 | 03922 | 1 | | 0 | 0 | 0 | 0 | 0 | 0 | 0 | 0 | 0 | 0 | 0 | 0 | 1 | 0 | 0 | 0 | 0 | 0 | 0 | 0 | 0 | 0 | 0 | 0 | 0 | | | | | | |
| 3924 | 04648 | 03923 | 1 | | 0 | 0 | 0 | 0 | 0 | 0 | 0 | 0 | 0 | 0 | 0 | 0 | 1 | 0 | 0 | 0 | 0 | 0 | 0 | 0 | 0 | 0 | 0 | 0 | 0 | | | | | | |
| 3925 | 04649 | 03924 | 1 | | 0 | 0 | 0 | 0 | 0 | 0 | 0 | 0 | 0 | 0 | 0 | 0 | 1 | 0 | 0 | 0 | 0 | 0 | 0 | 0 | 0 | 0 | 0 | 0 | 0 | | | | | | |
| 3926 | 04650 | 03925 | 1 | | 0 | 0 | 0 | 0 | 0 | 0 | 0 | 0 | 0 | 0 | 0 | 0 | 1 | 0 | 0 | 0 | 0 | 0 | 0 | 0 | 0 | 0 | 0 | 0 | 0 | | | | | | |
| 3927 | 04651 | 03926 | 1 | | 0 | 0 | 0 | 0 | 0 | 0 | 0 | 0 | 0 | 0 | 0 | 0 | 1 | 0 | 0 | 0 | 0 | 0 | 0 | 0 | 0 | 0 | 0 | 0 | 0 | | | | | | |
| 3928 | 04652 | 03927 | 1 | | 0 | 0 | 0 | 0 | 0 | 0 | 0 | 0 | 0 | 0 | 0 | 0 | 1 | 0 | 0 | 0 | 0 | 0 | 0 | 0 | 0 | 0 | 0 | 0 | 0 | | | | | | |
| 3929 | 04653 | 03928 | 1 | | 0 | 0 | 0 | 0 | 0 | 0 | 0 | 0 | 0 | 0 | 0 | 0 | 1 | 0 | 0 | 0 | 0 | 0 | 0 | 0 | 0 | 0 | 0 | 0 | 0 | | | | | | |
| 3930 | 04654 | 03929 | 2 | | 0 | 0 | 0 | 0 | 0 | 0 | 0 | 0 | 0 | 0 | 0 | 0 | 2 | 0 | 0 | 0 | 0 | 0 | 0 | 0 | 0 | 0 | 0 | 0 | 0 | | | | | | |
| 3931 | 04655 | 03930 | 5 | | 0 | 0 | 0 | 0 | 0 | 0 | 0 | 0 | 0 | 0 | 0 | 0 | 2 | 0 | 0 | 0 | 0 | 1 | 0 | 1 | 0 | 0 | 0 | 1 | 0 | M22349 | 97.9 | 285 | 1 | 1990 | 2273 |
| 3932 | 04656 | 03931 | 2 | | 0 | 0 | 0 | 0 | 0 | 0 | 0 | 0 | 0 | 0 | 0 | 0 | 1 | 0 | 0 | 0 | 0 | 1 | 0 | 0 | 0 | 0 | 0 | 0 | 0 | | | | | | |
| 3933 | 04657 | 03932 | 1 | | 0 | 0 | 0 | 0 | 0 | 0 | 0 | 0 | 0 | 0 | 0 | 0 | 1 | 0 | 0 | 0 | 0 | 0 | 0 | 0 | 0 | 0 | 0 | 0 | 0 | | | | | | |
| 3934 | 04658 | 03933 | 1 | | 0 | 0 | 0 | 0 | 0 | 0 | 0 | 0 | 0 | 0 | 0 | 0 | 1 | 0 | 0 | 0 | 0 | 0 | 0 | 0 | 0 | 0 | 0 | 0 | 0 | | | | | | |
| 3935 | 04659 | 03934 | 2 | | 0 | 0 | 0 | 0 | 0 | 0 | 0 | 0 | 0 | 0 | 0 | 0 | 2 | 0 | 0 | 0 | 0 | 0 | 0 | 0 | 0 | 0 | 0 | 0 | 0 | | | | | | |
| 3936 | 04660 | 03935 | 1 | | 0 | 0 | 0 | 0 | 0 | 0 | 0 | 0 | 0 | 0 | 0 | 0 | 1 | 0 | 0 | 0 | 0 | 0 | 0 | 0 | 0 | 0 | 0 | 0 | 0 | | | | | | |
| 3937 | 04661 | 03936 | 1 | | 0 | 0 | 0 | 0 | 0 | 0 | 0 | 0 | 0 | 0 | 0 | 0 | 1 | 0 | 0 | 0 | 0 | 0 | 0 | 0 | 0 | 0 | 0 | 0 | 0 | | | | | | |
| 3938 | 04662 | 03937 | 1 | | 0 | 0 | 0 | 0 | 0 | 0 | 0 | 0 | 0 | 0 | 0 | 0 | 1 | 0 | 0 | 0 | 0 | 0 | 0 | 0 | 0 | 0 | 0 | 0 | 0 | | | | | | |
| 3939 | 04663 | 03938 | 2 | | 0 | 0 | 0 | 0 | 0 | 0 | 0 | 0 | 0 | 0 | 0 | 0 | 1 | 0 | 1 | 0 | 0 | 0 | 0 | 0 | 0 | 0 | 0 | 0 | 0 | | | | | | |
| 3940 | 04664 | 03939 | 2 | | 0 | 0 | 0 | 0 | 0 | 0 | 0 | 0 | 0 | 0 | 0 | 0 | 2 | 0 | 0 | 0 | 0 | 0 | 0 | 0 | 0 | 0 | 0 | 0 | 0 | | | | | | |
| 3941 | 04665 | 03940 | 8 | | 0 | 0 | 0 | 0 | 0 | 0 | 0 | 0 | 0 | 0 | 0 | 0 | 2 | 0 | 0 | 0 | 0 | 0 | 1 | 2 | 0 | 1 | 0 | 2 | 0 | | | | | | |
| 3942 | 04666 | 03941 | 3 | | 0 | 0 | 0 | 0 | 0 | 0 | 0 | 0 | 0 | 0 | 0 | 0 | 1 | 0 | 0 | 0 | 0 | 0 | 1 | 0 | 0 | 0 | 0 | 1 | 0 | | | | | | |
| 3943 | 04667 | 03942 | 2 | | 0 | 0 | 0 | 0 | 0 | 0 | 0 | 0 | 0 | 0 | 0 | 0 | 2 | 0 | 0 | 0 | 0 | 0 | 0 | 0 | 0 | 0 | 0 | 0 | 0 | | | | | | |
| 3944 | 04668 | 03943 | 1 | | 0 | 0 | 0 | 0 | 0 | 0 | 0 | 0 | 0 | 0 | 0 | 0 | 1 | 0 | 0 | 0 | 0 | 0 | 0 | 0 | 0 | 0 | 0 | 0 | 0 | | | | | | |
| 3945 | 04669 | 03944 | 1 | | 0 | 0 | 0 | 0 | 0 | 0 | 0 | 0 | 0 | 0 | 0 | 0 | 1 | 0 | 0 | 0 | 0 | 0 | 0 | 0 | 0 | 0 | 0 | 0 | 0 | L13738 | 92.6 | 269 | 1 | 4278 | 4545 |

Table 110

| | A | B | C | E | G | I | K | M | O | Q | S | U | W | Y | AA | AC | AF | AG | AI | AK | AM | AO | AQ | AS | AU | AW | AY | BA | BC | BE | BF | BG | BH | BI | BK |
|---|---|---|---|---|---|---|---|---|---|---|---|---|---|---|---|---|---|---|---|---|---|---|---|---|---|---|---|---|---|---|---|---|---|---|---|
| 3946 | 04670 | 03945 | 1 | | 0 | 0 | 0 | 0 | 0 | 0 | 0 | 0 | 0 | 0 | 0 | 0 | 1 | 0 | 0 | 0 | 0 | 0 | 0 | 0 | 0 | 0 | 0 | 0 | 0 | | | | | | |
| 3947 | 04671 | 03946 | 1 | | 0 | 0 | 0 | 0 | 0 | 0 | 0 | 0 | 0 | 0 | 0 | 0 | 1 | 0 | 0 | 0 | 0 | 0 | 0 | 0 | 0 | 0 | 0 | 0 | 0 | | | | | | |
| 3948 | 04672 | 03947 | 1 | | 0 | 0 | 0 | 0 | 0 | 0 | 0 | 0 | 0 | 0 | 0 | 0 | 1 | 0 | 0 | 0 | 0 | 0 | 0 | 0 | 0 | 0 | 0 | 0 | 0 | | | | | | |
| 3949 | 04673 | 03948 | 1 | | 0 | 0 | 0 | 0 | 0 | 0 | 0 | 0 | 0 | 0 | 0 | 0 | 1 | 0 | 0 | 0 | 0 | 0 | 0 | 0 | 0 | 0 | 0 | 0 | 0 | | | | | | |
| 3950 | 04674 | 03949 | 2 | | 0 | 0 | 0 | 0 | 0 | 0 | 0 | 0 | 0 | 0 | 0 | 0 | 2 | 0 | 0 | 0 | 0 | 0 | 0 | 0 | 0 | 0 | 0 | 0 | 0 | | | | | | |
| 3951 | 04675 | 03950 | 1 | | 0 | 0 | 0 | 0 | 0 | 0 | 0 | 0 | 0 | 0 | 0 | 0 | 1 | 0 | 0 | 0 | 0 | 0 | 0 | 0 | 0 | 0 | 0 | 0 | 0 | | | | | | |
| 3952 | 04676 | 03951 | 2 | | 0 | 0 | 0 | 0 | 0 | 0 | 0 | 0 | 0 | 0 | 0 | 0 | 1 | 0 | 0 | 0 | 1 | 0 | 0 | 0 | 0 | 0 | 0 | 0 | 0 | | | | | | |
| 3953 | 04677 | 03952 | 1 | | 0 | 0 | 0 | 0 | 0 | 0 | 0 | 0 | 0 | 0 | 0 | 0 | 1 | 0 | 0 | 0 | 0 | 0 | 0 | 0 | 0 | 0 | 0 | 0 | 0 | | | | | | |
| 3954 | 04678 | 03953 | 1 | | 0 | 0 | 0 | 0 | 0 | 0 | 0 | 0 | 0 | 0 | 0 | 0 | 1 | 0 | 0 | 0 | 0 | 0 | 0 | 0 | 0 | 0 | 0 | 0 | 0 | | | | | | |
| 3955 | 04679 | 03954 | 2 | | 0 | 0 | 0 | 0 | 0 | 0 | 0 | 0 | 0 | 0 | 0 | 0 | 2 | 0 | 0 | 0 | 0 | 0 | 0 | 0 | 0 | 0 | 0 | 0 | 0 | | | | | | |
| 3956 | 04680 | 03955 | 1 | | 0 | 0 | 0 | 0 | 0 | 0 | 0 | 0 | 0 | 0 | 0 | 0 | 1 | 0 | 0 | 0 | 0 | 0 | 0 | 0 | 0 | 0 | 0 | 0 | 0 | | | | | | |
| 3957 | 04681 | 03956 | 1 | | 0 | 0 | 0 | 0 | 0 | 0 | 0 | 0 | 0 | 0 | 0 | 0 | 1 | 0 | 0 | 0 | 0 | 0 | 0 | 0 | 0 | 0 | 0 | 0 | 0 | | | | | | |
| 3958 | 04682 | 03957 | 1 | | 0 | 0 | 0 | 0 | 0 | 0 | 0 | 0 | 0 | 0 | 0 | 0 | 1 | 0 | 0 | 0 | 0 | 0 | 0 | 0 | 0 | 0 | 0 | 0 | 0 | | | | | | |
| 3959 | 04683 | 03958 | 1 | | 0 | 0 | 0 | 0 | 0 | 0 | 0 | 0 | 0 | 0 | 0 | 0 | 1 | 0 | 0 | 0 | 0 | 0 | 0 | 0 | 0 | 0 | 0 | 0 | 0 | | | | | | |
| 3960 | 04684 | 03959 | 1 | | 0 | 0 | 0 | 0 | 0 | 0 | 0 | 0 | 0 | 0 | 0 | 0 | 1 | 0 | 0 | 0 | 0 | 0 | 0 | 0 | 0 | 0 | 0 | 0 | 0 | | | | | | |
| 3961 | 04685 | 03960 | 1 | | 0 | 0 | 0 | 0 | 0 | 0 | 0 | 0 | 0 | 0 | 0 | 0 | 1 | 0 | 0 | 0 | 0 | 0 | 0 | 0 | 0 | 0 | 0 | 0 | 0 | | | | | | |
| 3962 | 04686 | 03961 | 1 | | 0 | 0 | 0 | 0 | 0 | 0 | 0 | 0 | 0 | 0 | 0 | 0 | 1 | 0 | 0 | 0 | 0 | 0 | 0 | 0 | 0 | 0 | 0 | 0 | 0 | | | | | | |
| 3963 | 04687 | 03962 | 2 | | 0 | 0 | 0 | 0 | 0 | 0 | 0 | 0 | 0 | 0 | 0 | 0 | 1 | 0 | 0 | 0 | 0 | 0 | 1 | 0 | 0 | 0 | 0 | 0 | 0 | | | | | | |
| 3964 | 04688 | 03963 | 1 | | 0 | 0 | 0 | 0 | 0 | 0 | 0 | 0 | 0 | 0 | 0 | 0 | 1 | 0 | 0 | 0 | 0 | 0 | 0 | 0 | 0 | 0 | 0 | 0 | 0 | | | | | | |
| 3965 | 04689 | 03964 | 2 | | 0 | 0 | 0 | 0 | 0 | 0 | 0 | 0 | 0 | 0 | 0 | 0 | 2 | 0 | 0 | 0 | 0 | 0 | 0 | 0 | 0 | 0 | 0 | 0 | 0 | | | | | | |
| 3966 | 04690 | 03965 | 1 | | 0 | 0 | 0 | 0 | 0 | 0 | 0 | 0 | 0 | 0 | 0 | 0 | 1 | 0 | 0 | 0 | 0 | 0 | 0 | 0 | 0 | 0 | 0 | 0 | 0 | | | | | | |
| 3967 | 04691 | 03966 | 2 | | 0 | 0 | 0 | 0 | 0 | 0 | 0 | 0 | 0 | 0 | 0 | 0 | 1 | 0 | 0 | 0 | 0 | 0 | 0 | 1 | 0 | 0 | 0 | 0 | 0 | | | | | | |
| 3968 | 04692 | 03967 | 1 | | 0 | 0 | 0 | 0 | 0 | 0 | 0 | 0 | 0 | 0 | 0 | 0 | 1 | 0 | 0 | 0 | 0 | 0 | 0 | 0 | 0 | 0 | 0 | 0 | 0 | | | | | | |
| 3969 | 04693 | 03968 | 2 | | 0 | 0 | 0 | 0 | 0 | 0 | 0 | 0 | 0 | 0 | 0 | 0 | 1 | 0 | 0 | 0 | 1 | 0 | 0 | 0 | 0 | 0 | 0 | 0 | 0 | | | | | | |
| 3970 | 04694 | 03969 | 1 | | 0 | 0 | 0 | 0 | 0 | 0 | 0 | 0 | 0 | 0 | 0 | 0 | 1 | 0 | 0 | 0 | 0 | 0 | 0 | 0 | 0 | 0 | 0 | 0 | 0 | | | | | | |
| 3971 | 04695 | 03970 | 1 | | 0 | 0 | 0 | 0 | 0 | 0 | 0 | 0 | 0 | 0 | 0 | 0 | 1 | 0 | 0 | 0 | 0 | 0 | 0 | 0 | 0 | 0 | 0 | 0 | 0 | | | | | | |
| 3972 | 04696 | 03971 | 1 | | 0 | 0 | 0 | 0 | 0 | 0 | 0 | 0 | 0 | 0 | 0 | 0 | 1 | 0 | 0 | 0 | 0 | 0 | 0 | 0 | 0 | 0 | 0 | 0 | 0 | | | | | | |
| 3973 | 04697 | 03972 | 1 | | 0 | 0 | 0 | 0 | 0 | 0 | 0 | 0 | 0 | 0 | 0 | 0 | 1 | 0 | 0 | 0 | 0 | 0 | 0 | 0 | 0 | 0 | 0 | 0 | 0 | | | | | | |
| 3974 | 04698 | 03973 | 1 | | 0 | 0 | 0 | 0 | 0 | 0 | 0 | 0 | 0 | 0 | 0 | 0 | 1 | 0 | 0 | 0 | 0 | 0 | 0 | 0 | 0 | 0 | 0 | 0 | 0 | | | | | | |
| 3975 | 04699 | 03974 | 1 | | 0 | 0 | 0 | 0 | 0 | 0 | 0 | 0 | 0 | 0 | 0 | 0 | 1 | 0 | 0 | 0 | 0 | 0 | 0 | 0 | 0 | 0 | 0 | 0 | 0 | | | | | | |
| 3976 | 04700 | 03975 | 1 | | 0 | 0 | 0 | 0 | 0 | 0 | 0 | 0 | 0 | 0 | 0 | 0 | 1 | 0 | 0 | 0 | 0 | 0 | 0 | 0 | 0 | 0 | 0 | 0 | 0 | | | | | | |
| 3977 | 04701 | 03976 | 1 | | 0 | 0 | 0 | 0 | 0 | 0 | 0 | 0 | 0 | 0 | 0 | 0 | 1 | 0 | 0 | 0 | 0 | 0 | 0 | 0 | 0 | 0 | 0 | 0 | 0 | | | | | | |
| 3978 | 04702 | 03977 | 1 | | 0 | 0 | 0 | 0 | 0 | 0 | 0 | 0 | 0 | 0 | 0 | 0 | 1 | 0 | 0 | 0 | 0 | 0 | 0 | 0 | 0 | 0 | 0 | 0 | 0 | | | | | | |
| 3979 | 04703 | 03978 | 2 | | 0 | 0 | 0 | 0 | 0 | 0 | 0 | 0 | 0 | 0 | 0 | 0 | 2 | 0 | 0 | 0 | 0 | 0 | 0 | 0 | 0 | 0 | 0 | 0 | 0 | | | | | | |
| 3980 | 04704 | 03979 | 1 | | 0 | 0 | 0 | 0 | 0 | 0 | 0 | 0 | 0 | 0 | 0 | 0 | 1 | 0 | 0 | 0 | 0 | 0 | 0 | 0 | 0 | 0 | 0 | 0 | 0 | | | | | | |
| 3981 | 04705 | 03980 | 2 | | 0 | 0 | 0 | 0 | 0 | 0 | 0 | 0 | 0 | 0 | 0 | 0 | 1 | 1 | 0 | 0 | 0 | 0 | 0 | 0 | 0 | 0 | 0 | 0 | 0 | | | | | | |

Table 111

| | A | B | C | E | G | I | K | M | O | Q | S | U | W | Y | AA | AC | AF | AG | AI | AK | AM | AO | AQ | AS | AU | AW | AY | BA | BC | BE | BF | BG | BH | BI | BK |
|---|---|---|---|---|---|---|---|---|---|---|---|---|---|---|---|---|---|---|---|---|---|---|---|---|---|---|---|---|---|---|---|---|---|---|---|
| 3982 | 04706 | 03981 | 3 | | 0 | 0 | 0 | 0 | 0 | 0 | 0 | 0 | 0 | 0 | 0 | 0 | 1 | 1 | 0 | 0 | 0 | 0 | 0 | 1 | 0 | 0 | 0 | 0 | 0 | | | | | | |
| 3983 | 04707 | 03982 | 1 | | 0 | 0 | 0 | 0 | 0 | 0 | 0 | 0 | 0 | 0 | 0 | 0 | 1 | 0 | 0 | 0 | 0 | 0 | 0 | 0 | 0 | 0 | 0 | 0 | 0 | X15088 | 94.3 | 230 | 1 | 1045 | 1292 |
| 3984 | 04708 | 03983 | 1 | | 0 | 0 | 0 | 0 | 0 | 0 | 0 | 0 | 0 | 0 | 0 | 0 | 1 | 0 | 0 | 0 | 0 | 0 | 0 | 0 | 0 | 0 | 0 | 0 | 0 | | | | | | |
| 3985 | 04709 | 03984 | 2 | | 0 | 0 | 0 | 0 | 0 | 0 | 0 | 0 | 0 | 0 | 0 | 0 | 2 | 0 | 0 | 0 | 0 | 0 | 0 | 0 | 0 | 0 | 0 | 0 | 0 | | | | | | |
| 3986 | 04710 | 03985 | 1 | | 0 | 0 | 0 | 0 | 0 | 0 | 0 | 0 | 0 | 0 | 0 | 0 | 1 | 0 | 0 | 0 | 0 | 0 | 0 | 0 | 0 | 0 | 0 | 0 | 0 | | | | | | |
| 3987 | 04711 | 03986 | 7 | | 0 | 0 | 0 | 0 | 0 | 0 | 0 | 0 | 0 | 0 | 0 | 0 | 4 | 0 | 0 | 0 | 0 | 0 | 0 | 2 | 0 | 0 | 1 | 0 | 0 | | | | | | |
| 3988 | 04712 | 03987 | 1 | | 0 | 0 | 0 | 0 | 0 | 0 | 0 | 0 | 0 | 0 | 0 | 0 | 1 | 0 | 0 | 0 | 0 | 0 | 0 | 0 | 0 | 0 | 0 | 0 | 0 | | | | | | |
| 3989 | 04713 | 03988 | 1 | | 0 | 0 | 0 | 0 | 0 | 0 | 0 | 0 | 0 | 0 | 0 | 0 | 1 | 0 | 0 | 0 | 0 | 0 | 0 | 0 | 0 | 0 | 0 | 0 | 0 | | | | | | |
| 3990 | 04714 | 03989 | 2 | | 0 | 0 | 0 | 0 | 0 | 0 | 0 | 0 | 0 | 0 | 0 | 0 | 2 | 0 | 0 | 0 | 0 | 0 | 0 | 0 | 0 | 0 | 0 | 0 | 0 | | | | | | |
| 3991 | 04715 | 03990 | 5 | | 0 | 0 | 0 | 0 | 0 | 0 | 0 | 0 | 0 | 0 | 0 | 0 | 2 | 1 | 1 | 0 | 0 | 0 | 1 | 0 | 0 | 0 | 0 | 0 | 0 | | | | | | |
| 3992 | 04716 | 03991 | 2 | | 0 | 0 | 0 | 0 | 0 | 0 | 0 | 0 | 0 | 0 | 0 | 0 | 1 | 0 | 0 | 0 | 0 | 0 | 0 | 0 | 1 | 0 | 0 | 0 | 0 | | | | | | |
| 3993 | 04717 | 03992 | 2 | | 0 | 0 | 0 | 0 | 0 | 0 | 0 | 0 | 0 | 0 | 0 | 0 | 2 | 0 | 0 | 0 | 0 | 0 | 0 | 0 | 0 | 0 | 0 | 0 | 0 | | | | | | |
| 3994 | 04718 | 03993 | 2 | | 0 | 0 | 0 | 0 | 0 | 0 | 0 | 0 | 0 | 0 | 0 | 0 | 2 | 0 | 0 | 0 | 0 | 0 | 0 | 0 | 0 | 0 | 0 | 0 | 0 | | | | | | |
| 3995 | 04719 | 03994 | 2 | | 0 | 0 | 0 | 0 | 0 | 0 | 0 | 0 | 0 | 0 | 0 | 0 | 1 | 0 | 0 | 0 | 0 | 0 | 0 | 0 | 0 | 1 | 0 | 0 | 0 | | | | | | |
| 3996 | 04720 | 03995 | 2 | | 0 | 0 | 0 | 0 | 0 | 0 | 0 | 0 | 0 | 0 | 0 | 0 | 1 | 0 | 0 | 0 | 0 | 0 | 0 | 0 | 0 | 1 | 0 | 0 | 0 | | | | | | |
| 3997 | 04721 | 03996 | 4 | | 0 | 0 | 0 | 0 | 0 | 0 | 0 | 0 | 0 | 0 | 0 | 0 | 3 | 0 | 0 | 0 | 0 | 0 | 0 | 0 | 0 | 0 | 1 | 0 | 0 | | | | | | |
| 3998 | 04722 | 03997 | 3 | | 0 | 0 | 0 | 0 | 0 | 0 | 0 | 0 | 0 | 0 | 0 | 0 | 1 | 0 | 0 | 0 | 0 | 0 | 0 | 0 | 0 | 1 | 1 | 0 | 0 | | | | | | |
| 3999 | 04723 | 03998 | 1 | | 0 | 0 | 0 | 0 | 0 | 0 | 0 | 0 | 0 | 0 | 0 | 0 | 1 | 0 | 0 | 0 | 0 | 0 | 0 | 0 | 0 | 0 | 0 | 0 | 0 | | | | | | |
| 4000 | 04724 | 03999 | 2 | | 0 | 0 | 0 | 0 | 0 | 0 | 0 | 0 | 0 | 0 | 0 | 0 | 2 | 0 | 0 | 0 | 0 | 0 | 0 | 0 | 0 | 0 | 0 | 0 | 0 | | | | | | |
| 4001 | 04725 | 04000 | 1 | | 0 | 0 | 0 | 0 | 0 | 0 | 0 | 0 | 0 | 0 | 0 | 0 | 1 | 0 | 0 | 0 | 0 | 0 | 0 | 0 | 0 | 0 | 0 | 0 | 0 | | | | | | |
| 4002 | 04728 | 04001 | 1 | | 0 | 0 | 0 | 0 | 0 | 0 | 0 | 0 | 0 | 0 | 0 | 0 | 1 | 0 | 0 | 0 | 0 | 0 | 0 | 0 | 0 | 0 | 0 | 0 | 0 | | | | | | |
| 4003 | 04729 | 04002 | 1 | | 0 | 0 | 0 | 0 | 0 | 0 | 0 | 0 | 0 | 0 | 0 | 0 | 1 | 0 | 0 | 0 | 0 | 0 | 0 | 0 | 0 | 0 | 0 | 0 | 0 | | | | | | |
| 4004 | 04730 | 04003 | 2 | | 0 | 0 | 0 | 0 | 0 | 0 | 0 | 0 | 0 | 0 | 0 | 0 | 1 | 0 | 0 | 0 | 0 | 0 | 0 | 0 | 0 | 0 | 1 | 0 | 0 | | | | | | |
| 4005 | 04732 | 04004 | 1 | | 0 | 0 | 0 | 0 | 0 | 0 | 0 | 0 | 0 | 0 | 0 | 0 | 1 | 0 | 0 | 0 | 0 | 0 | 0 | 0 | 0 | 0 | 0 | 0 | 0 | | | | | | |
| 4006 | 04733 | 04005 | 1 | | 0 | 0 | 0 | 0 | 0 | 0 | 0 | 0 | 0 | 0 | 0 | 0 | 1 | 0 | 0 | 0 | 0 | 0 | 0 | 0 | 0 | 0 | 0 | 0 | 0 | | | | | | |
| 4007 | 04734 | 04006 | 2 | | 0 | 0 | 0 | 0 | 0 | 0 | 0 | 0 | 0 | 0 | 0 | 0 | 1 | 0 | 0 | 0 | 0 | 0 | 0 | 0 | 0 | 1 | 0 | 0 | 0 | | | | | | |
| 4008 | 04735 | 04007 | 3 | | 0 | 0 | 0 | 0 | 0 | 0 | 0 | 0 | 0 | 0 | 0 | 0 | 2 | 0 | 0 | 0 | 0 | 0 | 0 | 1 | 0 | 0 | 0 | 0 | 0 | J02966 | 99.5 | 204 | 1 | 1097 | 1320 |
| 4009 | 04736 | 04008 | 1 | | 0 | 0 | 0 | 0 | 0 | 0 | 0 | 0 | 0 | 0 | 0 | 0 | 1 | 0 | 0 | 0 | 0 | 0 | 0 | 0 | 0 | 0 | 0 | 0 | 0 | | | | | | |
| 4010 | 04737 | 04009 | 2 | | 0 | 0 | 0 | 0 | 0 | 0 | 0 | 0 | 0 | 0 | 0 | 0 | 2 | 0 | 0 | 0 | 0 | 0 | 0 | 0 | 0 | 0 | 0 | 0 | 0 | | | | | | |
| 4011 | 04738 | 04010 | 1 | | 0 | 0 | 0 | 0 | 0 | 0 | 0 | 0 | 0 | 0 | 0 | 0 | 1 | 0 | 0 | 0 | 0 | 0 | 0 | 0 | 0 | 0 | 0 | 0 | 0 | | | | | | |
| 4012 | 04739 | 04011 | 1 | | 0 | 0 | 0 | 0 | 0 | 0 | 0 | 0 | 0 | 0 | 0 | 0 | 1 | 0 | 0 | 0 | 0 | 0 | 0 | 0 | 0 | 0 | 0 | 0 | 0 | | | | | | |
| 4013 | 04740 | 04012 | 1 | | 0 | 0 | 0 | 0 | 0 | 0 | 0 | 0 | 0 | 0 | 0 | 0 | 1 | 0 | 0 | 0 | 0 | 0 | 0 | 0 | 0 | 0 | 0 | 0 | 0 | | | | | | |
| 4014 | 04741 | 04013 | 1 | | 0 | 0 | 0 | 0 | 0 | 0 | 0 | 0 | 0 | 0 | 0 | 0 | 1 | 0 | 0 | 0 | 0 | 0 | 0 | 0 | 0 | 0 | 0 | 0 | 0 | | | | | | |
| 4015 | 04742 | 04014 | 1 | | 0 | 0 | 0 | 0 | 0 | 0 | 0 | 0 | 0 | 0 | 0 | 0 | 1 | 0 | 0 | 0 | 0 | 0 | 0 | 0 | 0 | 0 | 0 | 0 | 0 | | | | | | |
| 4016 | 04743 | 04015 | 1 | | 0 | 0 | 0 | 0 | 0 | 0 | 0 | 0 | 0 | 0 | 0 | 0 | 1 | 0 | 0 | 0 | 0 | 0 | 0 | 0 | 0 | 0 | 0 | 0 | 0 | S42457 | 98 | 198 | 1 | 2483 | 2857 |
| 4017 | 04744 | 04016 | 2 | | 0 | 0 | 0 | 0 | 0 | 0 | 0 | 0 | 0 | 0 | 0 | 0 | 1 | 0 | 0 | 0 | 1 | 0 | 0 | 0 | 0 | 0 | 0 | 0 | 0 | | | | | | |

Table 112

| | A | B | C | E | G | I | K | M | O | Q | S | U | W | Y | AA | AC | AE | AG | AI | AK | AM | AO | AQ | AS | AU | AW | AY | BA | BC | BE | BF | BG | BH | BI | BK |
|---|---|---|---|---|---|---|---|---|---|---|---|---|---|---|---|---|---|---|---|---|---|---|---|---|---|---|---|---|---|---|---|---|---|---|---|
| 4018 | 04745 | 04017 | 1 | | 0 | 0 | 0 | 0 | 0 | 0 | 0 | 0 | 0 | 0 | 0 | 0 | 1 | 0 | 0 | 0 | 0 | 0 | 0 | 0 | 0 | 0 | 0 | 0 | 0 | | | | | | |
| 4019 | 04746 | 04018 | 1 | | 0 | 0 | 0 | 0 | 0 | 0 | 0 | 0 | 0 | 0 | 0 | 0 | 1 | 0 | 0 | 0 | 0 | 0 | 0 | 0 | 0 | 0 | 0 | 0 | 0 | | | | | | |
| 4020 | 04747 | 04019 | 3 | | 0 | 0 | 0 | 0 | 0 | 0 | 0 | 0 | 0 | 0 | 0 | 0 | 1 | 0 | 1 | 0 | 0 | 0 | 0 | 0 | 1 | 0 | 0 | 0 | 0 | | | | | | |
| 4021 | 04748 | 04020 | 1 | | 0 | 0 | 0 | 0 | 0 | 0 | 0 | 0 | 0 | 0 | 0 | 0 | 1 | 0 | 0 | 0 | 0 | 0 | 0 | 0 | 0 | 0 | 0 | 0 | 0 | | | | | | |
| 4022 | 04750 | 04021 | 2 | | 0 | 0 | 0 | 0 | 0 | 0 | 0 | 0 | 0 | 0 | 0 | 0 | 1 | 1 | 0 | 0 | 0 | 0 | 0 | 0 | 0 | 0 | 0 | 0 | 0 | | | | | | |
| 4023 | 04751 | 04022 | 1 | | 0 | 0 | 0 | 0 | 0 | 0 | 0 | 0 | 0 | 0 | 0 | 0 | 1 | 0 | 0 | 0 | 0 | 0 | 0 | 0 | 0 | 0 | 0 | 0 | 0 | | | | | | |
| 4024 | 04752 | 04023 | 3 | | 0 | 0 | 0 | 0 | 0 | 0 | 0 | 0 | 0 | 0 | 0 | 0 | 2 | 0 | 0 | 0 | 0 | 0 | 1 | 0 | 0 | 0 | 0 | 0 | 0 | | | | | | |
| 4025 | 04753 | 04024 | 3 | | 0 | 0 | 0 | 0 | 0 | 0 | 0 | 0 | 0 | 0 | 0 | 0 | 1 | 0 | 0 | 0 | 0 | 0 | 0 | 2 | 0 | 0 | 0 | 0 | 0 | | | | | | |
| 4026 | 04754 | 04025 | 8 | | 0 | 0 | 0 | 0 | 0 | 0 | 0 | 0 | 0 | 0 | 0 | 0 | 8 | 0 | 0 | 0 | 0 | 0 | 0 | 0 | 0 | 0 | 0 | 0 | 0 | | | | | | |
| 4027 | 04755 | 04026 | 2 | | 0 | 0 | 0 | 0 | 0 | 0 | 0 | 0 | 0 | 0 | 0 | 0 | 1 | 0 | 1 | 0 | 0 | 0 | 0 | 0 | 0 | 0 | 0 | 0 | 0 | | | | | | |
| 4028 | 04756 | 04027 | 1 | | 0 | 0 | 0 | 0 | 0 | 0 | 0 | 0 | 0 | 0 | 0 | 0 | 1 | 0 | 0 | 0 | 0 | 0 | 0 | 0 | 0 | 0 | 0 | 0 | 0 | | | | | | |
| 4029 | 04757 | 04028 | 5 | | 0 | 0 | 0 | 0 | 0 | 0 | 0 | 0 | 0 | 0 | 0 | 0 | 1 | 2 | 0 | 0 | 0 | 0 | 0 | 2 | 0 | 0 | 0 | 0 | 0 | | | | | | |
| 4030 | 04758 | 04029 | 1 | | 0 | 0 | 0 | 0 | 0 | 0 | 0 | 0 | 0 | 0 | 0 | 0 | 1 | 0 | 0 | 0 | 0 | 0 | 0 | 0 | 0 | 0 | 0 | 0 | 0 | | | | | | |
| 4031 | 04759 | 04030 | 1 | | 0 | 0 | 0 | 0 | 0 | 0 | 0 | 0 | 0 | 0 | 0 | 0 | 1 | 0 | 0 | 0 | 0 | 0 | 0 | 0 | 0 | 0 | 0 | 0 | 0 | | | | | | |
| 4032 | 04760 | 04031 | 1 | | 0 | 0 | 0 | 0 | 0 | 0 | 0 | 0 | 0 | 0 | 0 | 0 | 1 | 0 | 0 | 0 | 0 | 0 | 0 | 0 | 0 | 0 | 0 | 0 | 0 | | | | | | |
| 4033 | 04761 | 04032 | 2 | | 0 | 0 | 0 | 0 | 0 | 0 | 0 | 0 | 0 | 0 | 0 | 0 | 2 | 0 | 0 | 0 | 0 | 0 | 0 | 0 | 0 | 0 | 0 | 0 | 0 | | | | | | |
| 4034 | 04762 | 04033 | 6 | | 0 | 0 | 0 | 0 | 0 | 0 | 0 | 0 | 0 | 0 | 0 | 0 | 2 | 0 | 0 | 3 | 0 | 1 | 0 | 0 | 0 | 0 | 0 | 0 | 0 | J04056 | 100 | 178 | 1 | 1032 | 1209 |
| 4035 | 04763 | 04034 | 4 | | 0 | 0 | 0 | 0 | 0 | 0 | 0 | 0 | 0 | 0 | 0 | 0 | 4 | 0 | 0 | 0 | 0 | 0 | 0 | 0 | 0 | 0 | 0 | 0 | 0 | | | | | | |
| 4036 | 04764 | 04035 | 2 | | 0 | 0 | 0 | 0 | 0 | 0 | 0 | 0 | 0 | 0 | 0 | 0 | 2 | 0 | 0 | 0 | 0 | 0 | 0 | 0 | 0 | 0 | 0 | 0 | 0 | | | | | | |
| 4037 | 04765 | 04036 | 1 | | 0 | 0 | 0 | 0 | 0 | 0 | 0 | 0 | 0 | 0 | 0 | 0 | 1 | 0 | 0 | 0 | 0 | 0 | 0 | 0 | 0 | 0 | 0 | 0 | 0 | | | | | | |
| 4038 | 04766 | 04037 | 1 | | 0 | 0 | 0 | 0 | 0 | 0 | 0 | 0 | 0 | 0 | 0 | 0 | 1 | 0 | 0 | 0 | 0 | 0 | 0 | 0 | 0 | 0 | 0 | 0 | 0 | | | | | | |
| 4039 | 04767 | 04038 | 2 | | 0 | 0 | 0 | 0 | 0 | 0 | 0 | 0 | 0 | 0 | 0 | 0 | 2 | 0 | 0 | 0 | 0 | 0 | 0 | 0 | 0 | 0 | 0 | 0 | 0 | | | | | | |
| 4040 | 04768 | 04039 | 1 | | 0 | 0 | 0 | 0 | 0 | 0 | 0 | 0 | 0 | 0 | 0 | 0 | 1 | 0 | 0 | 0 | 0 | 0 | 0 | 0 | 0 | 0 | 0 | 0 | 0 | | | | | | |
| 4041 | 04769 | 04040 | 1 | | 0 | 0 | 0 | 0 | 0 | 0 | 0 | 0 | 0 | 0 | 0 | 0 | 1 | 0 | 0 | 0 | 0 | 0 | 0 | 0 | 0 | 0 | 0 | 0 | 0 | | | | | | |
| 4042 | 04770 | 04041 | 2 | | 0 | 0 | 0 | 0 | 0 | 0 | 0 | 0 | 0 | 0 | 0 | 0 | 2 | 0 | 0 | 0 | 0 | 0 | 0 | 0 | 0 | 0 | 0 | 0 | 0 | | | | | | |
| 4043 | 04771 | 04042 | 1 | | 0 | 0 | 0 | 0 | 0 | 0 | 0 | 0 | 0 | 0 | 0 | 0 | 1 | 0 | 0 | 0 | 0 | 0 | 0 | 0 | 0 | 0 | 0 | 0 | 0 | | | | | | |
| 4044 | 04772 | 04043 | 4 | | 0 | 0 | 0 | 0 | 0 | 0 | 0 | 0 | 0 | 0 | 0 | 0 | 3 | 0 | 0 | 0 | 0 | 0 | 1 | 0 | 0 | 0 | 0 | 0 | 0 | | | | | | |
| 4045 | 04773 | 04044 | 2 | | 0 | 0 | 0 | 0 | 0 | 0 | 0 | 0 | 0 | 0 | 0 | 0 | 2 | 0 | 0 | 0 | 0 | 0 | 0 | 0 | 0 | 0 | 0 | 0 | 0 | | | | | | |
| 4046 | 04774 | 04045 | 1 | | 0 | 0 | 0 | 0 | 0 | 0 | 0 | 0 | 0 | 0 | 0 | 0 | 1 | 0 | 0 | 0 | 0 | 0 | 0 | 0 | 0 | 0 | 0 | 0 | 0 | | | | | | |
| 4047 | 04775 | 04046 | 1 | | 0 | 0 | 0 | 0 | 0 | 0 | 0 | 0 | 0 | 0 | 0 | 0 | 1 | 0 | 0 | 0 | 0 | 0 | 0 | 0 | 0 | 0 | 0 | 0 | 0 | M73531 | 97 | 166 | 1 | 2820 | 2985 |
| 4048 | 04776 | 04047 | 1 | | 0 | 0 | 0 | 0 | 0 | 0 | 0 | 0 | 0 | 0 | 0 | 0 | 1 | 0 | 0 | 0 | 0 | 0 | 0 | 0 | 0 | 0 | 0 | 0 | 0 | | | | | | |
| 4049 | 04777 | 04048 | 2 | | 0 | 0 | 0 | 0 | 0 | 0 | 0 | 0 | 0 | 0 | 0 | 0 | 1 | 0 | 0 | 0 | 0 | 0 | 0 | 0 | 0 | 1 | 0 | 0 | 0 | | | | | | |
| 4050 | 04778 | 04049 | 1 | | 0 | 0 | 0 | 0 | 0 | 0 | 0 | 0 | 0 | 0 | 0 | 0 | 1 | 0 | 0 | 0 | 0 | 0 | 0 | 0 | 0 | 0 | 0 | 0 | 0 | | | | | | |
| 4051 | 04779 | 04050 | 3 | | 0 | 0 | 0 | 0 | 0 | 0 | 0 | 0 | 0 | 0 | 1 | 0 | 1 | 0 | 1 | 0 | 0 | 0 | 0 | 0 | 0 | 0 | 0 | 0 | 0 | | | | | | |
| 4052 | 04780 | 04051 | 2 | | 0 | 0 | 0 | 0 | 0 | 0 | 0 | 0 | 0 | 0 | 0 | 0 | 1 | 0 | 0 | 0 | 0 | 0 | 0 | 1 | 0 | 0 | 0 | 0 | 0 | | | | | | |
| 4053 | 04781 | 04052 | 1 | | 0 | 0 | 0 | 0 | 0 | 0 | 0 | 0 | 0 | 0 | 0 | 0 | 1 | 0 | 0 | 0 | 0 | 0 | 0 | 0 | 0 | 0 | 0 | 0 | 0 | | | | | | |

Table 113

| | A | B | C | E | G | I | K | M | O | Q | S | U | W | Y | AA | AC | AE | AG | AI | AK | AM | AO | AQ | AS | AU | AW | AY | BA | BC | BE | BF | BG | BH | BI | BK |
|---|---|---|---|---|---|---|---|---|---|---|---|---|---|---|---|---|---|---|---|---|---|---|---|---|---|---|---|---|---|---|---|---|---|---|---|
| 4054 | 04782 | 04053 | 1 | | 0 | 0 | 0 | 0 | 0 | 0 | 0 | 0 | 0 | 0 | 0 | 0 | 1 | 0 | 0 | 0 | 0 | 0 | 0 | 0 | 0 | 0 | 0 | 0 | 0 | | | | | | |
| 4055 | 04783 | 04054 | 14 | | 0 | 0 | 0 | 0 | 0 | 0 | 0 | 0 | 0 | 0 | 0 | 0 | 14 | 0 | 0 | 0 | 0 | 0 | 0 | 0 | 0 | 0 | 0 | 0 | 0 | | | | | | |
| 4056 | 04784 | 04055 | 2 | | 0 | 0 | 0 | 0 | 0 | 0 | 0 | 0 | 0 | 0 | 0 | 0 | 1 | 0 | 0 | 0 | 0 | 0 | 0 | 1 | 0 | 0 | 0 | 0 | 0 | | | | | | |
| 4057 | 04785 | 04056 | 1 | | 0 | 0 | 0 | 0 | 0 | 0 | 0 | 0 | 0 | 0 | 0 | 0 | 1 | 0 | 0 | 0 | 0 | 0 | 0 | 0 | 0 | 0 | 0 | 0 | 0 | | | | | | |
| 4058 | 04786 | 04057 | 7 | | 0 | 0 | 0 | 0 | 0 | 0 | 0 | 0 | 0 | 0 | 0 | 0 | 4 | 0 | 0 | 0 | 0 | 0 | 0 | 3 | 0 | 0 | 0 | 0 | 0 | | | | | | |
| 4059 | 04787 | 04058 | 1 | | 0 | 0 | 0 | 0 | 0 | 0 | 0 | 0 | 0 | 0 | 0 | 0 | 1 | 0 | 0 | 0 | 0 | 0 | 0 | 0 | 0 | 0 | 0 | 0 | 0 | | | | | | |
| 4060 | 04788 | 04059 | 2 | | 0 | 0 | 0 | 0 | 0 | 0 | 0 | 0 | 0 | 0 | 0 | 0 | 1 | 0 | 0 | 0 | 0 | 0 | 0 | 0 | 0 | 0 | 0 | 1 | 0 | | | | | | |
| 4061 | 04789 | 04060 | 1 | | 0 | 0 | 0 | 0 | 0 | 0 | 0 | 0 | 0 | 0 | 0 | 0 | 1 | 0 | 0 | 0 | 0 | 0 | 0 | 0 | 0 | 0 | 0 | 0 | 0 | | | | | | |
| 4062 | 04790 | 04061 | 1 | | 0 | 0 | 0 | 0 | 0 | 0 | 0 | 0 | 0 | 0 | 0 | 0 | 1 | 0 | 0 | 0 | 0 | 0 | 0 | 0 | 0 | 0 | 0 | 0 | 0 | | | | | | |
| 4063 | 04791 | 04062 | 3 | | 0 | 0 | 0 | 0 | 0 | 0 | 0 | 0 | 0 | 0 | 0 | 0 | 3 | 0 | 0 | 0 | 0 | 0 | 0 | 0 | 0 | 0 | 0 | 0 | 0 | | | | | | |
| 4064 | 04792 | 04063 | 2 | | 0 | 0 | 0 | 0 | 0 | 0 | 0 | 0 | 0 | 0 | 0 | 0 | 2 | 0 | 0 | 0 | 0 | 0 | 0 | 0 | 0 | 0 | 0 | 0 | 0 | | | | | | |
| 4065 | 04793 | 04064 | 1 | | 0 | 0 | 0 | 0 | 0 | 0 | 0 | 0 | 0 | 0 | 0 | 0 | 1 | 0 | 0 | 0 | 0 | 0 | 0 | 0 | 0 | 0 | 0 | 0 | 0 | | | | | | |
| 4066 | 04794 | 04065 | 1 | | 0 | 0 | 0 | 0 | 0 | 0 | 0 | 0 | 0 | 0 | 0 | 0 | 1 | 0 | 0 | 0 | 0 | 0 | 0 | 0 | 0 | 0 | 0 | 0 | 0 | | | | | | |
| 4067 | 04795 | 04066 | 2 | | 0 | 0 | 0 | 0 | 0 | 0 | 0 | 0 | 0 | 0 | 1 | 0 | 1 | 0 | 0 | 0 | 0 | 0 | 0 | 0 | 0 | 0 | 0 | 0 | 0 | | | | | | |
| 4068 | 04796 | 04067 | 1 | | 0 | 0 | 0 | 0 | 0 | 0 | 0 | 0 | 0 | 0 | 0 | 0 | 1 | 0 | 0 | 0 | 0 | 0 | 0 | 0 | 0 | 0 | 0 | 0 | 0 | | | | | | |
| 4069 | 04797 | 04068 | 1 | | 0 | 0 | 0 | 0 | 0 | 0 | 0 | 0 | 0 | 0 | 0 | 0 | 1 | 0 | 0 | 0 | 0 | 0 | 0 | 0 | 0 | 0 | 0 | 0 | 0 | | | | | | |
| 4070 | 04798 | 04069 | 5 | | 0 | 0 | 0 | 0 | 0 | 0 | 0 | 0 | 0 | 0 | 0 | 0 | 1 | 1 | 1 | 0 | 0 | 0 | 0 | 0 | 2 | 0 | 0 | 0 | 0 | M27319 | 97.3 | 148 | 1 | 462 | 799 |
| 4071 | 04799 | 04070 | 2 | | 0 | 0 | 0 | 0 | 0 | 0 | 0 | 0 | 0 | 0 | 0 | 0 | 1 | 0 | 1 | 0 | 0 | 0 | 0 | 0 | 0 | 0 | 0 | 0 | 0 | | | | | | |
| 4072 | 04800 | 04071 | 1 | | 0 | 0 | 0 | 0 | 0 | 0 | 0 | 0 | 0 | 0 | 0 | 0 | 1 | 0 | 0 | 0 | 0 | 0 | 0 | 0 | 0 | 0 | 0 | 0 | 0 | | | | | | |
| 4073 | 04801 | 04072 | 1 | | 0 | 0 | 0 | 0 | 0 | 0 | 0 | 0 | 0 | 0 | 0 | 0 | 1 | 0 | 0 | 0 | 0 | 0 | 0 | 0 | 0 | 0 | 0 | 0 | 0 | | | | | | |
| 4074 | 04802 | 04073 | 2 | | 0 | 0 | 0 | 0 | 0 | 0 | 0 | 0 | 0 | 0 | 0 | 0 | 2 | 0 | 0 | 0 | 0 | 0 | 0 | 0 | 0 | 0 | 0 | 0 | 0 | | | | | | |
| 4075 | 04803 | 04074 | 1 | | 0 | 0 | 0 | 0 | 0 | 0 | 0 | 0 | 0 | 0 | 0 | 0 | 1 | 0 | 0 | 0 | 0 | 0 | 0 | 0 | 0 | 0 | 0 | 0 | 0 | | | | | | |
| 4076 | 04804 | 04075 | 2 | | 0 | 0 | 0 | 0 | 0 | 0 | 0 | 0 | 0 | 0 | 0 | 0 | 2 | 0 | 0 | 0 | 0 | 0 | 0 | 0 | 0 | 0 | 0 | 0 | 0 | | | | | | |
| 4077 | 04805 | 04076 | 2 | | 0 | 0 | 0 | 0 | 0 | 0 | 0 | 0 | 0 | 0 | 0 | 0 | 2 | 0 | 0 | 0 | 0 | 0 | 0 | 0 | 0 | 0 | 0 | 0 | 0 | | | | | | |
| 4078 | 04806 | 04077 | 1 | | 0 | 0 | 0 | 0 | 0 | 0 | 0 | 0 | 0 | 0 | 0 | 0 | 1 | 0 | 0 | 0 | 0 | 0 | 0 | 0 | 0 | 0 | 0 | 0 | 0 | | | | | | |
| 4079 | 04808 | 04078 | 1 | | 0 | 0 | 0 | 0 | 0 | 0 | 0 | 0 | 0 | 0 | 0 | 0 | 1 | 0 | 0 | 0 | 0 | 0 | 0 | 0 | 0 | 0 | 0 | 0 | 0 | | | | | | |
| 4080 | 04809 | 04079 | 1 | | 0 | 0 | 0 | 0 | 0 | 0 | 0 | 0 | 0 | 0 | 0 | 0 | 1 | 0 | 0 | 0 | 0 | 0 | 0 | 0 | 0 | 0 | 0 | 0 | 0 | | | | | | |
| 4081 | 04810 | 04080 | 2 | | 0 | 0 | 0 | 0 | 0 | 0 | 0 | 0 | 0 | 0 | 0 | 0 | 1 | 0 | 1 | 0 | 0 | 0 | 0 | 0 | 0 | 0 | 0 | 0 | 0 | | | | | | |
| 4082 | 04811 | 04081 | 1 | | 0 | 0 | 0 | 0 | 0 | 0 | 0 | 0 | 0 | 0 | 0 | 0 | 1 | 0 | 0 | 0 | 0 | 0 | 0 | 0 | 0 | 0 | 0 | 0 | 0 | S74445 | 96.4 | 140 | 1 | 596 | 735 |
| 4083 | 04812 | 04082 | 1 | | 0 | 0 | 0 | 0 | 0 | 0 | 0 | 0 | 0 | 0 | 0 | 0 | 1 | 0 | 0 | 0 | 0 | 0 | 0 | 0 | 0 | 0 | 0 | 0 | 0 | | | | | | |
| 4084 | 04813 | 04083 | 1 | | 0 | 0 | 0 | 0 | 0 | 0 | 0 | 0 | 0 | 0 | 0 | 0 | 1 | 0 | 0 | 0 | 0 | 0 | 0 | 0 | 0 | 0 | 0 | 0 | 0 | | | | | | |
| 4085 | 04814 | 04084 | 2 | | 0 | 0 | 0 | 0 | 0 | 0 | 0 | 0 | 0 | 0 | 0 | 0 | 1 | 0 | 0 | 0 | 0 | 0 | 0 | 0 | 0 | 0 | 0 | 1 | 0 | | | | | | |
| 4086 | 04815 | 04085 | 1 | | 0 | 0 | 0 | 0 | 0 | 0 | 0 | 0 | 0 | 0 | 0 | 0 | 1 | 0 | 0 | 0 | 0 | 0 | 0 | 0 | 0 | 0 | 0 | 0 | 0 | | | | | | |
| 4087 | 04816 | 04086 | 1 | | 0 | 0 | 0 | 0 | 0 | 0 | 0 | 0 | 0 | 0 | 0 | 0 | 1 | 0 | 0 | 0 | 0 | 0 | 0 | 0 | 0 | 0 | 0 | 0 | 0 | | | | | | |
| 4088 | 04817 | 04087 | 1 | | 0 | 0 | 0 | 0 | 0 | 0 | 0 | 0 | 0 | 0 | 0 | 0 | 1 | 0 | 0 | 0 | 0 | 0 | 0 | 0 | 0 | 0 | 0 | 0 | 0 | | | | | | |
| 4089 | 04818 | 04088 | 5 | | 0 | 0 | 0 | 0 | 0 | 0 | 0 | 0 | 0 | 0 | 0 | 0 | 2 | 0 | 0 | 0 | 0 | 1 | 1 | 0 | 1 | 0 | 0 | 0 | 0 | X72760 | 98.5 | 136 | 1 | 1830 | 1971 |

Table 114

| | A | B | C | E | G | I | K | M | O | Q | S | U | W | Y | AA | AC | AE | AG | AI | AK | AM | AO | AQ | AS | AU | AW | AY | BA | BC | BE | BF | BG | BH | BI | BK |
|---|---|---|---|---|---|---|---|---|---|---|---|---|---|---|---|---|---|---|---|---|---|---|---|---|---|---|---|---|---|---|---|---|---|---|---|
| 4090 | 04819 | 04089 | 2 | | 0 | 0 | 0 | 0 | 0 | 0 | 0 | 0 | 0 | 0 | 0 | 0 | 2 | 0 | 0 | 0 | 0 | 0 | 0 | 0 | 0 | 0 | 0 | 0 | 0 | | | | | | |
| 4091 | 04820 | 04090 | 3 | | 0 | 0 | 0 | 0 | 0 | 0 | 0 | 0 | 0 | 0 | 0 | 0 | 3 | 0 | 0 | 0 | 0 | 0 | 0 | 0 | 0 | 0 | 0 | 0 | 0 | | | | | | |
| 4092 | 04821 | 04091 | 1 | | 0 | 0 | 0 | 0 | 0 | 0 | 0 | 0 | 0 | 0 | 0 | 0 | 1 | 0 | 0 | 0 | 0 | 0 | 0 | 0 | 0 | 0 | 0 | 0 | 0 | | | | | | |
| 4093 | 04822 | 04092 | 3 | | 0 | 0 | 0 | 0 | 0 | 0 | 0 | 0 | 0 | 0 | 0 | 0 | 1 | 1 | 0 | 0 | 0 | 0 | 0 | 0 | 1 | 0 | 0 | 0 | 0 | | | | | | |
| 4094 | 04823 | 04093 | 3 | | 0 | 0 | 0 | 0 | 0 | 0 | 0 | 0 | 0 | 0 | 0 | 0 | 1 | 1 | 1 | 0 | 0 | 0 | 0 | 0 | 0 | 0 | 0 | 0 | 0 | M65217 | 100 | 101 | 1 | 2311 | 2411 |
| 4095 | 04824 | 04094 | 1 | | 0 | 0 | 0 | 0 | 0 | 0 | 0 | 0 | 0 | 0 | 0 | 0 | 1 | 0 | 0 | 0 | 0 | 0 | 0 | 0 | 0 | 0 | 0 | 0 | 0 | | | | | | |
| 4096 | 04826 | 04095 | 1 | | 0 | 0 | 0 | 0 | 0 | 0 | 0 | 0 | 0 | 0 | 0 | 0 | 1 | 0 | 0 | 0 | 0 | 0 | 0 | 0 | 0 | 0 | 0 | 0 | 0 | | | | | | |
| 4097 | 04827 | 04096 | 1 | | 0 | 0 | 0 | 0 | 0 | 0 | 0 | 0 | 0 | 0 | 0 | 0 | 1 | 0 | 0 | 0 | 0 | 0 | 0 | 0 | 0 | 0 | 0 | 0 | 0 | | | | | | |
| 4098 | 04828 | 04097 | 2 | | 0 | 0 | 0 | 0 | 0 | 0 | 0 | 0 | 0 | 0 | 0 | 0 | 1 | 0 | 0 | 0 | 0 | 0 | 0 | 0 | 0 | 0 | 1 | 0 | 0 | | | | | | |
| 4099 | 04829 | 04098 | 2 | | 0 | 0 | 0 | 0 | 0 | 0 | 0 | 0 | 0 | 0 | 0 | 0 | 2 | 0 | 0 | 0 | 0 | 0 | 0 | 0 | 0 | 0 | 0 | 0 | 0 | | | | | | |
| 4100 | 04830 | 04099 | 1 | | 0 | 0 | 0 | 0 | 0 | 0 | 0 | 0 | 0 | 0 | 0 | 0 | 1 | 0 | 0 | 0 | 0 | 0 | 0 | 0 | 0 | 0 | 0 | 0 | 0 | | | | | | |
| 4101 | 04831 | 04100 | 1 | | 0 | 0 | 0 | 0 | 0 | 0 | 0 | 0 | 0 | 0 | 0 | 0 | 1 | 0 | 0 | 0 | 0 | 0 | 0 | 0 | 0 | 0 | 0 | 0 | 0 | | | | | | |
| 4102 | 04832 | 04101 | 1 | | 0 | 0 | 0 | 0 | 0 | 0 | 0 | 0 | 0 | 0 | 0 | 0 | 1 | 0 | 0 | 0 | 0 | 0 | 0 | 0 | 0 | 0 | 0 | 0 | 0 | | | | | | |
| 4103 | 04833 | 04102 | 1 | | 0 | 0 | 0 | 0 | 0 | 0 | 0 | 0 | 0 | 0 | 0 | 0 | 1 | 0 | 0 | 0 | 0 | 0 | 0 | 0 | 0 | 0 | 0 | 0 | 0 | | | | | | |
| 4104 | 04834 | 04103 | 1 | | 0 | 0 | 0 | 0 | 0 | 0 | 0 | 0 | 0 | 0 | 0 | 0 | 1 | 0 | 0 | 0 | 0 | 0 | 0 | 0 | 0 | 0 | 0 | 0 | 0 | | | | | | |
| 4105 | 04835 | 04104 | 1 | | 0 | 0 | 0 | 0 | 0 | 0 | 0 | 0 | 0 | 0 | 0 | 0 | 1 | 0 | 0 | 0 | 0 | 0 | 0 | 0 | 0 | 0 | 0 | 0 | 0 | | | | | | |
| 4106 | 04836 | 04105 | 2 | | 0 | 0 | 0 | 0 | 0 | 0 | 0 | 0 | 0 | 0 | 0 | 0 | 2 | 0 | 0 | 0 | 0 | 0 | 0 | 0 | 0 | 0 | 0 | 0 | 0 | | | | | | |
| 4107 | 04837 | 04106 | 1 | | 0 | 0 | 0 | 0 | 0 | 0 | 0 | 0 | 0 | 0 | 0 | 0 | 1 | 0 | 0 | 0 | 0 | 0 | 0 | 0 | 0 | 0 | 0 | 0 | 0 | | | | | | |
| 4108 | 04838 | 04107 | 1 | | 0 | 0 | 0 | 0 | 0 | 0 | 0 | 0 | 0 | 0 | 0 | 0 | 1 | 0 | 0 | 0 | 0 | 0 | 0 | 0 | 0 | 0 | 0 | 0 | 0 | | | | | | |
| 4109 | 04839 | 04108 | 1 | | 0 | 0 | 0 | 0 | 0 | 0 | 0 | 0 | 0 | 0 | 0 | 0 | 1 | 0 | 0 | 0 | 0 | 0 | 0 | 0 | 0 | 0 | 0 | 0 | 0 | | | | | | |
| 4110 | 04840 | 04109 | 1 | | 0 | 0 | 0 | 0 | 0 | 0 | 0 | 0 | 0 | 0 | 0 | 0 | 1 | 0 | 0 | 0 | 0 | 0 | 0 | 0 | 0 | 0 | 0 | 0 | 0 | | | | | | |
| 4111 | 04841 | 04110 | 3 | | 0 | 0 | 0 | 0 | 0 | 0 | 0 | 0 | 0 | 0 | 0 | 0 | 2 | 0 | 0 | 1 | 0 | 0 | 0 | 0 | 0 | 0 | 0 | 0 | 0 | X02317 | 100 | 111 | 1 | 517 | 874 |
| 4112 | 04842 | 04111 | 1 | | 0 | 0 | 0 | 0 | 0 | 0 | 0 | 0 | 0 | 0 | 0 | 0 | 1 | 0 | 0 | 0 | 0 | 0 | 0 | 0 | 0 | 0 | 0 | 0 | 0 | | | | | | |
| 4113 | 04843 | 04112 | 1 | | 0 | 0 | 0 | 0 | 0 | 0 | 0 | 0 | 0 | 0 | 0 | 0 | 1 | 0 | 0 | 0 | 0 | 0 | 0 | 0 | 0 | 0 | 0 | 0 | 0 | | | | | | |
| 4114 | 04844 | 04113 | 1 | | 0 | 0 | 0 | 0 | 0 | 0 | 0 | 0 | 0 | 0 | 0 | 0 | 1 | 0 | 0 | 0 | 0 | 0 | 0 | 0 | 0 | 0 | 0 | 0 | 0 | | | | | | |
| 4115 | 04845 | 04114 | 1 | | 0 | 0 | 0 | 0 | 0 | 0 | 0 | 0 | 0 | 0 | 0 | 0 | 1 | 0 | 0 | 0 | 0 | 0 | 0 | 0 | 0 | 0 | 0 | 0 | 0 | | | | | | |
| 4116 | 04846 | 04115 | 1 | | 0 | 0 | 0 | 0 | 0 | 0 | 0 | 0 | 0 | 0 | 0 | 0 | 1 | 0 | 0 | 0 | 0 | 0 | 0 | 0 | 0 | 0 | 0 | 0 | 0 | | | | | | |
| 4117 | 04847 | 04116 | 2 | | 0 | 0 | 0 | 0 | 0 | 0 | 0 | 0 | 0 | 0 | 0 | 0 | 2 | 0 | 0 | 0 | 0 | 0 | 0 | 0 | 0 | 0 | 0 | 0 | 0 | | | | | | |
| 4118 | 04848 | 04117 | 1 | | 0 | 0 | 0 | 0 | 0 | 0 | 0 | 0 | 0 | 0 | 0 | 0 | 1 | 0 | 0 | 0 | 0 | 0 | 0 | 0 | 0 | 0 | 0 | 0 | 0 | | | | | | |
| 4119 | 04850 | 04118 | 5 | | 0 | 0 | 0 | 0 | 0 | 0 | 0 | 0 | 0 | 0 | 0 | 0 | 2 | 0 | 0 | 1 | 2 | 0 | 0 | 0 | 0 | 0 | 0 | 0 | 0 | | | | | | |
| 4120 | 04851 | 04119 | 1 | | 0 | 0 | 0 | 0 | 0 | 0 | 0 | 0 | 0 | 0 | 0 | 0 | 1 | 0 | 0 | 0 | 0 | 0 | 0 | 0 | 0 | 0 | 0 | 0 | 0 | | | | | | |
| 4121 | 04852 | 04120 | 4 | | 0 | 0 | 0 | 0 | 0 | 0 | 0 | 0 | 0 | 0 | 0 | 0 | 1 | 1 | 0 | 0 | 0 | 0 | 0 | 1 | 0 | 1 | 0 | 0 | 0 | | | | | | |
| 4122 | 04853 | 04121 | 2 | | 0 | 0 | 0 | 0 | 0 | 0 | 0 | 0 | 0 | 0 | 0 | 0 | 1 | 0 | 0 | 0 | 0 | 0 | 0 | 0 | 0 | 1 | 0 | 0 | 0 | | | | | | |
| 4123 | 04854 | 04122 | 1 | | 0 | 0 | 0 | 0 | 0 | 0 | 0 | 0 | 0 | 0 | 0 | 0 | 1 | 0 | 0 | 0 | 0 | 0 | 0 | 0 | 0 | 0 | 0 | 0 | 0 | | | | | | |
| 4124 | 04855 | 04123 | 1 | | 0 | 0 | 0 | 0 | 0 | 0 | 0 | 0 | 0 | 0 | 0 | 0 | 1 | 0 | 0 | 0 | 0 | 0 | 0 | 0 | 0 | 0 | 0 | 0 | 0 | | | | | | |
| 4125 | 04856 | 04124 | 1 | | 0 | 0 | 0 | 0 | 0 | 0 | 0 | 0 | 0 | 0 | 0 | 0 | 1 | 0 | 0 | 0 | 0 | 0 | 0 | 0 | 0 | 0 | 0 | 0 | 0 | | | | | | |

Table 115

| | A | B | C | E | G | I | K | M | O | Q | S | U | W | Y | AA | AC | AE | AG | AI | AK | AM | AO | AQ | AS | AU | AW | AY | BA | BC | BE | BF | BG | BH | BI | BK |
|---|---|---|---|---|---|---|---|---|---|---|---|---|---|---|---|---|---|---|---|---|---|---|---|---|---|---|---|---|---|---|---|---|---|---|---|
| 4126 | 04859 | 04125 | 3 | | 0 | 0 | 0 | 0 | 0 | 0 | 0 | 0 | 0 | 0 | 0 | 0 | 3 | 0 | 0 | 0 | 0 | 0 | 0 | 0 | 0 | 0 | 0 | 0 | 0 | | | | | | |
| 4127 | 04860 | 04126 | 1 | | 0 | 0 | 0 | 0 | 0 | 0 | 0 | 0 | 0 | 0 | 0 | 0 | 1 | 0 | 0 | 0 | 0 | 0 | 0 | 0 | 0 | 0 | 0 | 0 | 0 | | | | | | |
| 4128 | 04861 | 04127 | 1 | | 0 | 0 | 0 | 0 | 0 | 0 | 0 | 0 | 0 | 0 | 0 | 0 | 1 | 0 | 0 | 0 | 0 | 0 | 0 | 0 | 0 | 0 | 0 | 0 | 0 | | | | | | |
| 4129 | 04862 | 04128 | 2 | | 0 | 0 | 0 | 0 | 0 | 0 | 0 | 0 | 0 | 0 | 0 | 0 | 2 | 0 | 0 | 0 | 0 | 0 | 0 | 0 | 0 | 0 | 0 | 0 | 0 | | | | | | |
| 4130 | 04863 | 04129 | 1 | | 0 | 0 | 0 | 0 | 0 | 0 | 0 | 0 | 0 | 0 | 0 | 0 | 1 | 0 | 0 | 0 | 0 | 0 | 0 | 0 | 0 | 0 | 0 | 0 | 0 | | | | | | |
| 4131 | 04864 | 04130 | 1 | | 0 | 0 | 0 | 0 | 0 | 0 | 0 | 0 | 0 | 0 | 0 | 0 | 1 | 0 | 0 | 0 | 0 | 0 | 0 | 0 | 0 | 0 | 0 | 0 | 0 | | | | | | |
| 4132 | 04865 | 04131 | 1 | | 0 | 0 | 0 | 0 | 0 | 0 | 0 | 0 | 0 | 0 | 0 | 0 | 1 | 0 | 0 | 0 | 0 | 0 | 0 | 0 | 0 | 0 | 0 | 0 | 0 | | | | | | |
| 4133 | 04866 | 04132 | 1 | | 0 | 0 | 0 | 0 | 0 | 0 | 0 | 0 | 0 | 0 | 0 | 0 | 1 | 0 | 0 | 0 | 0 | 0 | 0 | 0 | 0 | 0 | 0 | 0 | 0 | | | | | | |
| 4134 | 04867 | 04133 | 2 | | 0 | 0 | 0 | 0 | 0 | 0 | 0 | 0 | 0 | 0 | 0 | 0 | 1 | 0 | 0 | 0 | 0 | 0 | 0 | 1 | 0 | 0 | 0 | 0 | 0 | | | | | | |
| 4135 | 04868 | 04134 | 1 | | 0 | 0 | 0 | 0 | 0 | 0 | 0 | 0 | 0 | 0 | 0 | 0 | 1 | 0 | 0 | 0 | 0 | 0 | 0 | 0 | 0 | 0 | 0 | 0 | 0 | | | | | | |
| 4136 | 04869 | 04135 | 1 | | 0 | 0 | 0 | 0 | 0 | 0 | 0 | 0 | 0 | 0 | 0 | 0 | 1 | 0 | 0 | 0 | 0 | 0 | 0 | 0 | 0 | 0 | 0 | 0 | 0 | | | | | | |
| 4137 | 04870 | 04136 | 1 | | 0 | 0 | 0 | 0 | 0 | 0 | 0 | 0 | 0 | 0 | 0 | 0 | 1 | 0 | 0 | 0 | 0 | 0 | 0 | 0 | 0 | 0 | 0 | 0 | 0 | | | | | | |
| 4138 | 04872 | 04137 | 3 | | 0 | 0 | 0 | 0 | 0 | 0 | 0 | 0 | 0 | 0 | 0 | 0 | 1 | 0 | 0 | 1 | 0 | 0 | 0 | 1 | 0 | 0 | 0 | 0 | 0 | | | | | | |
| 4139 | 04873 | 04138 | 4 | | 0 | 0 | 0 | 0 | 0 | 0 | 0 | 0 | 0 | 0 | 0 | 0 | 2 | 0 | 0 | 2 | 0 | 0 | 0 | 0 | 0 | 0 | 0 | 0 | 0 | | | | | | |
| 4140 | 04874 | 04139 | 4 | | 0 | 0 | 0 | 0 | 0 | 0 | 0 | 0 | 0 | 0 | 0 | 0 | 4 | 0 | 0 | 0 | 0 | 0 | 0 | 0 | 0 | 0 | 0 | 0 | 0 | | | | | | |
| 4141 | 04875 | 04140 | 5 | | 0 | 0 | 0 | 0 | 0 | 0 | 0 | 0 | 0 | 0 | 0 | 0 | 5 | 0 | 0 | 0 | 0 | 0 | 0 | 0 | 0 | 0 | 0 | 0 | 0 | | | | | | |
| 4142 | 04877 | 04141 | 1 | | 0 | 0 | 0 | 0 | 0 | 0 | 0 | 0 | 0 | 0 | 0 | 0 | 1 | 0 | 0 | 0 | 0 | 0 | 0 | 0 | 0 | 0 | 0 | 0 | 0 | | | | | | |
| 4143 | 04878 | 04142 | 4 | | 0 | 0 | 0 | 0 | 0 | 0 | 0 | 0 | 0 | 0 | 0 | 0 | 1 | 0 | 0 | 0 | 0 | 0 | 3 | 0 | 0 | 0 | 0 | 0 | 0 | | | | | | |
| 4144 | 04879 | 04143 | 2 | | 0 | 0 | 0 | 0 | 0 | 0 | 0 | 0 | 0 | 0 | 0 | 0 | 1 | 1 | 0 | 0 | 0 | 0 | 0 | 0 | 0 | 0 | 0 | 0 | 0 | | | | | | |
| 4145 | 04881 | 04144 | 5 | | 0 | 0 | 0 | 0 | 0 | 0 | 0 | 0 | 0 | 0 | 0 | 0 | 1 | 2 | 0 | 0 | 0 | 0 | 2 | 0 | 0 | 0 | 0 | 0 | 0 | | | | | | |
| 4146 | 04882 | 04145 | 1 | | 0 | 0 | 0 | 0 | 0 | 0 | 0 | 0 | 0 | 0 | 0 | 0 | 1 | 0 | 0 | 0 | 0 | 0 | 0 | 0 | 0 | 0 | 0 | 0 | 0 | | | | | | |
| 4147 | 04883 | 04146 | 1 | | 0 | 0 | 0 | 0 | 0 | 0 | 0 | 0 | 0 | 0 | 0 | 0 | 1 | 0 | 0 | 0 | 0 | 0 | 0 | 0 | 0 | 0 | 0 | 0 | 0 | | | | | | |
| 4148 | 04884 | 04147 | 2 | | 0 | 0 | 0 | 0 | 0 | 0 | 0 | 0 | 0 | 0 | 0 | 0 | 2 | 0 | 0 | 0 | 0 | 0 | 0 | 0 | 0 | 0 | 0 | 0 | 0 | | | | | | |
| 4149 | 04885 | 04148 | 2 | | 0 | 0 | 0 | 0 | 0 | 0 | 0 | 0 | 0 | 0 | 0 | 0 | 1 | 0 | 0 | 0 | 0 | 0 | 0 | 1 | 0 | 0 | 0 | 0 | 0 | | | | | | |
| 4150 | 04886 | 04149 | 2 | | 0 | 0 | 0 | 0 | 0 | 0 | 0 | 0 | 0 | 0 | 0 | 0 | 1 | 0 | 1 | 0 | 0 | 0 | 0 | 0 | 0 | 0 | 0 | 0 | 0 | | | | | | |
| 4151 | 04887 | 04150 | 2 | | 0 | 0 | 0 | 0 | 0 | 0 | 0 | 0 | 0 | 0 | 0 | 0 | 1 | 0 | 1 | 0 | 0 | 0 | 0 | 0 | 0 | 0 | 0 | 0 | 0 | | | | | | |
| 4152 | 04888 | 04151 | 25 | | 0 | 0 | 0 | 0 | 0 | 0 | 0 | 0 | 0 | 0 | 0 | 0 | 9 | 3 | 0 | 0 | 0 | 0 | 6 | 7 | 0 | 0 | 0 | 0 | 0 | | | | | | |
| 4153 | 04889 | 04152 | 6 | | 0 | 0 | 0 | 0 | 0 | 0 | 0 | 0 | 0 | 0 | 0 | 0 | 3 | 0 | 0 | 1 | 0 | 0 | 1 | 0 | 1 | 0 | 0 | 0 | 0 | | | | | | |
| 4154 | 04890 | 04153 | 4 | | 0 | 0 | 0 | 0 | 0 | 0 | 0 | 0 | 0 | 0 | 0 | 0 | 1 | 1 | 0 | 0 | 0 | 0 | 0 | 2 | 0 | 0 | 0 | 0 | 0 | | | | | | |
| 4155 | 04891 | 04154 | 1 | | 0 | 0 | 0 | 0 | 0 | 0 | 0 | 0 | 0 | 0 | 0 | 0 | 1 | 0 | 0 | 0 | 0 | 0 | 0 | 0 | 0 | 0 | 0 | 0 | 0 | | | | | | |
| 4156 | 04892 | 04155 | 3 | | 0 | 0 | 0 | 0 | 0 | 0 | 0 | 0 | 0 | 0 | 0 | 0 | 3 | 0 | 0 | 0 | 0 | 0 | 0 | 0 | 0 | 0 | 0 | 0 | 0 | | | | | | |
| 4157 | 04893 | 04156 | 1 | | 0 | 0 | 0 | 0 | 0 | 0 | 0 | 0 | 0 | 0 | 0 | 0 | 1 | 0 | 0 | 0 | 0 | 0 | 0 | 0 | 0 | 0 | 0 | 0 | 0 | | | | | | |
| 4158 | 04894 | 04157 | 3 | | 0 | 0 | 0 | 0 | 0 | 0 | 0 | 0 | 0 | 0 | 0 | 0 | 3 | 0 | 0 | 0 | 0 | 0 | 0 | 0 | 0 | 0 | 0 | 0 | 0 | | | | | | |
| 4159 | 04895 | 04158 | 14 | | 0 | 0 | 0 | 0 | 0 | 0 | 0 | 0 | 0 | 0 | 0 | 0 | 13 | 1 | 0 | 0 | 0 | 0 | 0 | 0 | 0 | 0 | 0 | 0 | 0 | | | | | | |
| 4160 | 04897 | 04159 | 1 | | 0 | 0 | 0 | 0 | 0 | 0 | 0 | 0 | 0 | 0 | 0 | 0 | 1 | 0 | 0 | 0 | 0 | 0 | 0 | 0 | 0 | 0 | 0 | 0 | 0 | | | | | | |
| 4161 | 04898 | 04160 | 2 | | 0 | 0 | 0 | 0 | 0 | 0 | 0 | 0 | 0 | 0 | 0 | 0 | 2 | 0 | 0 | 0 | 0 | 0 | 0 | 0 | 0 | 0 | 0 | 0 | 0 | | | | | | |

Table 116

| | A | B | C | E | G | I | K | M | O | Q | S | U | W | Y | AA | AC | AE | AG | AI | AK | AM | AO | AQ | AS | AU | AW | AY | BA | BC | BE | BF | BG | BH | BI | BK |
|---|---|---|---|---|---|---|---|---|---|---|---|---|---|---|---|---|---|---|---|---|---|---|---|---|---|---|---|---|---|---|---|---|---|---|---|
| 4162 | 04900 | 04161 | 1 | | 0 | 0 | 0 | 0 | 0 | 0 | 0 | 0 | 0 | 0 | 0 | 0 | 1 | 0 | 0 | 0 | 0 | 0 | 0 | 0 | 0 | 0 | 0 | 0 | 0 | | | | | | |
| 4163 | 04902 | 04162 | 1 | | 0 | 0 | 0 | 0 | 0 | 0 | 0 | 0 | 0 | 0 | 0 | 0 | 1 | 0 | 0 | 0 | 0 | 0 | 0 | 0 | 0 | 0 | 0 | 0 | 0 | | | | | | |
| 4164 | 04903 | 04163 | 1 | | 0 | 0 | 0 | 0 | 0 | 0 | 0 | 0 | 0 | 0 | 0 | 0 | 1 | 0 | 0 | 0 | 0 | 0 | 0 | 0 | 0 | 0 | 0 | 0 | 0 | | | | | | |
| 4165 | 04904 | 04164 | 5 | | 0 | 0 | 0 | 0 | 0 | 0 | 0 | 0 | 0 | 0 | 0 | 0 | 1 | 2 | 0 | 1 | 0 | 0 | 0 | 1 | 0 | 0 | 0 | 0 | 0 | | | | | | |
| 4166 | 04905 | 04165 | 5 | | 0 | 0 | 0 | 0 | 0 | 0 | 0 | 0 | 0 | 0 | 0 | 0 | 3 | 2 | 0 | 0 | 0 | 0 | 0 | 0 | 0 | 0 | 0 | 0 | 0 | | | | | | |
| 4167 | 04907 | 04166 | 2 | | 0 | 0 | 0 | 0 | 0 | 0 | 0 | 0 | 0 | 0 | 0 | 0 | 2 | 0 | 0 | 0 | 0 | 0 | 0 | 0 | 0 | 0 | 0 | 0 | 0 | | | | | | |
| 4168 | 04908 | 04167 | 2 | | 0 | 0 | 0 | 0 | 0 | 0 | 0 | 0 | 0 | 0 | 0 | 0 | 1 | 0 | 0 | 0 | 0 | 0 | 0 | 0 | 0 | 0 | 1 | 0 | 0 | | | | | | |
| 4169 | 04909 | 04168 | 1 | | 0 | 0 | 0 | 0 | 0 | 0 | 0 | 0 | 0 | 0 | 0 | 0 | 1 | 0 | 0 | 0 | 0 | 0 | 0 | 0 | 0 | 0 | 0 | 0 | 0 | | | | | | |
| 4170 | 04910 | 04169 | 1 | | 0 | 0 | 0 | 0 | 0 | 0 | 0 | 0 | 0 | 0 | 0 | 0 | 1 | 0 | 0 | 0 | 0 | 0 | 0 | 0 | 0 | 0 | 0 | 0 | 0 | L03785 | 100 | 65 | 1 | 597 | 661 |
| 4171 | 04912 | 04170 | 1 | | 0 | 0 | 0 | 0 | 0 | 0 | 0 | 0 | 0 | 0 | 0 | 0 | 1 | 0 | 0 | 0 | 0 | 0 | 0 | 0 | 0 | 0 | 0 | 0 | 0 | | | | | | |
| 4172 | 04913 | 04171 | 4 | | 0 | 0 | 0 | 0 | 0 | 0 | 0 | 0 | 0 | 0 | 0 | 0 | 2 | 0 | 1 | 0 | 0 | 0 | 1 | 0 | 0 | 0 | 0 | 0 | 0 | | | | | | |
| 4173 | 04914 | 04172 | 6 | | 0 | 0 | 0 | 0 | 0 | 0 | 0 | 0 | 0 | 0 | 0 | 0 | 2 | 0 | 0 | 1 | 0 | 0 | 0 | 0 | 0 | 1 | 0 | 1 | 1 | | | | | | |
| 4174 | 04915 | 04173 | 2 | | 0 | 0 | 0 | 0 | 0 | 0 | 0 | 0 | 0 | 0 | 0 | 0 | 2 | 0 | 0 | 0 | 0 | 0 | 0 | 0 | 0 | 0 | 0 | 0 | 0 | | | | | | |
| 4175 | 04916 | 04174 | 1 | | 0 | 0 | 0 | 0 | 0 | 0 | 0 | 0 | 0 | 0 | 0 | 0 | 1 | 0 | 0 | 0 | 0 | 0 | 0 | 0 | 0 | 0 | 0 | 0 | 0 | | | | | | |
| 4176 | 04917 | 04175 | 3 | | 0 | 0 | 0 | 0 | 0 | 0 | 0 | 0 | 0 | 0 | 0 | 0 | 3 | 0 | 0 | 0 | 0 | 0 | 0 | 0 | 0 | 0 | 0 | 0 | 0 | | | | | | |
| 4177 | 04918 | 04176 | 1 | | 0 | 0 | 0 | 0 | 0 | 0 | 0 | 0 | 0 | 0 | 0 | 0 | 1 | 0 | 0 | 0 | 0 | 0 | 0 | 0 | 0 | 0 | 0 | 0 | 0 | | | | | | |
| 4178 | 04919 | 04177 | 1 | | 0 | 0 | 0 | 0 | 0 | 0 | 0 | 0 | 0 | 0 | 0 | 0 | 1 | 0 | 0 | 0 | 0 | 0 | 0 | 0 | 0 | 0 | 0 | 0 | 0 | | | | | | |
| 4179 | 04920 | 04178 | 1 | | 0 | 0 | 0 | 0 | 0 | 0 | 0 | 0 | 0 | 0 | 0 | 0 | 1 | 0 | 0 | 0 | 0 | 0 | 0 | 0 | 0 | 0 | 0 | 0 | 0 | | | | | | |
| 4180 | 04921 | 04179 | 1 | | 0 | 0 | 0 | 0 | 0 | 0 | 0 | 0 | 0 | 0 | 0 | 0 | 1 | 0 | 0 | 0 | 0 | 0 | 0 | 0 | 0 | 0 | 0 | 0 | 0 | | | | | | |
| 4181 | 04922 | 04180 | 2 | | 0 | 0 | 0 | 0 | 0 | 0 | 0 | 0 | 0 | 0 | 0 | 0 | 1 | 0 | 0 | 0 | 0 | 0 | 0 | 0 | 0 | 1 | 0 | 0 | 0 | | | | | | |
| 4182 | 04923 | 04181 | 1 | | 0 | 0 | 0 | 0 | 0 | 0 | 0 | 0 | 0 | 0 | 0 | 0 | 1 | 0 | 0 | 0 | 0 | 0 | 0 | 0 | 0 | 0 | 0 | 0 | 0 | | | | | | |
| 4183 | 04924 | 04182 | 1 | | 0 | 0 | 0 | 0 | 0 | 0 | 0 | 0 | 0 | 0 | 0 | 0 | 1 | 0 | 0 | 0 | 0 | 0 | 0 | 0 | 0 | 0 | 0 | 0 | 0 | M74826 | 96.6 | 58 | 1 | 2396 | 2457 |
| 4184 | 04925 | 04183 | 1 | | 0 | 0 | 0 | 0 | 0 | 0 | 0 | 0 | 0 | 0 | 0 | 0 | 1 | 0 | 0 | 0 | 0 | 0 | 0 | 0 | 0 | 0 | 0 | 0 | 0 | | | | | | |
| 4185 | 04926 | 04184 | 1 | | 0 | 0 | 0 | 0 | 0 | 0 | 0 | 0 | 0 | 0 | 0 | 0 | 1 | 0 | 0 | 0 | 0 | 0 | 0 | 0 | 0 | 0 | 0 | 0 | 0 | | | | | | |
| 4186 | 04927 | 04185 | 1 | | 0 | 0 | 0 | 0 | 0 | 0 | 0 | 0 | 0 | 0 | 0 | 0 | 1 | 0 | 0 | 0 | 0 | 0 | 0 | 0 | 0 | 0 | 0 | 0 | 0 | | | | | | |
| 4187 | 04928 | 04186 | 3 | | 0 | 0 | 0 | 0 | 0 | 0 | 0 | 0 | 0 | 0 | 0 | 0 | 3 | 0 | 0 | 0 | 0 | 0 | 0 | 0 | 0 | 0 | 0 | 0 | 0 | | | | | | |
| 4188 | 04929 | 04187 | 1 | | 0 | 0 | 0 | 0 | 0 | 0 | 0 | 0 | 0 | 0 | 0 | 0 | 1 | 0 | 0 | 0 | 0 | 0 | 0 | 0 | 0 | 0 | 0 | 0 | 0 | | | | | | |
| 4189 | 04930 | 04188 | 1 | | 0 | 0 | 0 | 0 | 0 | 0 | 0 | 0 | 0 | 0 | 0 | 0 | 1 | 0 | 0 | 0 | 0 | 0 | 0 | 0 | 0 | 0 | 0 | 0 | 0 | | | | | | |
| 4190 | 04940 | 04189 | 1 | | 0 | 0 | 0 | 0 | 0 | 0 | 0 | 0 | 0 | 0 | 0 | 0 | 1 | 0 | 0 | 0 | 0 | 0 | 0 | 0 | 0 | 0 | 0 | 0 | 0 | | | | | | |
| 4191 | 04945 | 04190 | 2 | | 0 | 0 | 0 | 0 | 0 | 0 | 0 | 0 | 0 | 0 | 0 | 0 | 2 | 0 | 0 | 0 | 0 | 0 | 0 | 0 | 0 | 0 | 0 | 0 | 0 | | | | | | |
| 4192 | 04947 | 04191 | 1 | | 0 | 0 | 0 | 0 | 0 | 0 | 0 | 0 | 0 | 0 | 0 | 0 | 1 | 0 | 0 | 0 | 0 | 0 | 0 | 0 | 0 | 0 | 0 | 0 | 0 | | | | | | |
| 4193 | 04948 | 04192 | 3 | | 0 | 0 | 0 | 0 | 0 | 0 | 0 | 0 | 0 | 0 | 0 | 0 | 1 | 2 | 0 | 0 | 0 | 0 | 0 | 0 | 0 | 0 | 0 | 0 | 0 | | | | | | |
| 4194 | 04950 | 04193 | 1 | | 0 | 0 | 0 | 0 | 0 | 0 | 0 | 0 | 0 | 0 | 0 | 0 | 1 | 0 | 0 | 0 | 0 | 0 | 0 | 0 | 0 | 0 | 0 | 0 | 0 | | | | | | |
| 4195 | 04965 | 04194 | 3 | | 0 | 0 | 0 | 0 | 0 | 0 | 0 | 0 | 0 | 0 | 0 | 0 | 1 | 0 | 0 | 1 | 0 | 0 | 0 | 0 | 1 | 0 | 0 | 0 | 0 | | | | | | |
| 4196 | 04985 | 04195 | 2 | | 0 | 0 | 0 | 0 | 0 | 0 | 0 | 0 | 0 | 0 | 0 | 0 | 1 | 0 | 1 | 0 | 0 | 0 | 0 | 0 | 0 | 0 | 0 | 0 | 0 | | | | | | |
| 4197 | 04986 | 04196 | 2 | | 0 | 0 | 0 | 0 | 0 | 0 | 0 | 0 | 0 | 0 | 0 | 0 | 0 | 0 | 1 | 0 | 0 | 0 | 0 | 0 | 0 | 0 | 1 | 0 | 0 | | | | | | |

Table 117

| | A | B | C | E | G | I | K | M | O | Q | S | U | W | Y | AA | AC | AF | AG | AI | AK | AM | AO | AQ | AS | AU | AW | AY | BA | BC | BE | BF | BG | BH | BI | BK |
|---|---|---|---|---|---|---|---|---|---|---|---|---|---|---|---|---|---|---|---|---|---|---|---|---|---|---|---|---|---|---|---|---|---|---|---|
| 4198 | 04987 | 04197 | 3 | | 0 | 0 | 0 | 0 | 0 | 0 | 0 | 0 | 0 | 0 | 0 | 0 | 1 | 0 | 1 | 0 | 0 | 0 | 0 | 0 | 0 | 0 | 0 | 1 | 0 | | | | | | |
| 4199 | 04988 | 04198 | 2 | | 0 | 0 | 0 | 0 | 0 | 0 | 0 | 0 | 0 | 0 | 0 | 0 | 1 | 0 | 1 | 0 | 0 | 0 | 0 | 0 | 0 | 0 | 0 | 0 | 0 | | | | | | |
| 4200 | 04989 | 04199 | 1 | | 0 | 0 | 0 | 0 | 0 | 0 | 0 | 0 | 0 | 0 | 0 | 0 | 0 | 0 | 1 | 0 | 0 | 0 | 0 | 0 | 0 | 0 | 0 | 0 | 0 | | | | | | |
| 4201 | 04990 | 04200 | 1 | | 0 | 0 | 0 | 0 | 0 | 0 | 0 | 0 | 0 | 0 | 0 | 0 | 0 | 0 | 1 | 0 | 0 | 0 | 0 | 0 | 0 | 0 | 0 | 0 | 0 | | | | | | |
| 4202 | 04991 | 04201 | 1 | | 0 | 0 | 0 | 0 | 0 | 0 | 0 | 0 | 0 | 0 | 0 | 0 | 0 | 0 | 1 | 0 | 0 | 0 | 0 | 0 | 0 | 0 | 0 | 0 | 0 | | | | | | |
| 4203 | 04992 | 04202 | 1 | | 0 | 0 | 0 | 0 | 0 | 0 | 0 | 0 | 0 | 0 | 0 | 0 | 0 | 0 | 1 | 0 | 0 | 0 | 0 | 0 | 0 | 0 | 0 | 0 | 0 | | | | | | |
| 4204 | 04993 | 04203 | 1 | | 0 | 0 | 0 | 0 | 0 | 0 | 0 | 0 | 0 | 0 | 0 | 0 | 0 | 0 | 1 | 0 | 0 | 0 | 0 | 0 | 0 | 0 | 0 | 0 | 0 | | | | | | |
| 4205 | 04994 | 04204 | 1 | | 0 | 0 | 0 | 0 | 0 | 0 | 0 | 0 | 0 | 0 | 0 | 0 | 0 | 0 | 1 | 0 | 0 | 0 | 0 | 0 | 0 | 0 | 0 | 0 | 0 | | | | | | |
| 4206 | 04997 | 04205 | 1 | | 0 | 0 | 0 | 0 | 0 | 0 | 0 | 0 | 0 | 0 | 0 | 0 | 0 | 0 | 1 | 0 | 0 | 0 | 0 | 0 | 0 | 0 | 0 | 0 | 0 | | | | | | |
| 4207 | 04998 | 04206 | 2 | | 0 | 0 | 0 | 0 | 0 | 0 | 0 | 0 | 0 | 0 | 0 | 0 | 0 | 0 | 2 | 0 | 0 | 0 | 0 | 0 | 0 | 0 | 0 | 0 | 0 | | | | | | |
| 4208 | 04999 | 04207 | 2 | | 0 | 0 | 0 | 0 | 0 | 0 | 0 | 0 | 0 | 0 | 0 | 0 | 0 | 0 | 2 | 0 | 0 | 0 | 0 | 0 | 0 | 0 | 0 | 0 | 0 | X76488 | 93.3 | 270 | 1 | 2202 | 2626 |
| 4209 | 05001 | 04208 | 1 | | 0 | 0 | 0 | 0 | 0 | 0 | 0 | 0 | 0 | 0 | 0 | 0 | 0 | 0 | 1 | 0 | 0 | 0 | 0 | 0 | 0 | 0 | 0 | 0 | 0 | Y00839 | 98.5 | 341 | 1 | 3286 | 3624 |
| 4210 | 05002 | 04209 | 1 | | 0 | 0 | 0 | 0 | 0 | 0 | 0 | 0 | 0 | 0 | 0 | 0 | 0 | 0 | 1 | 0 | 0 | 0 | 0 | 0 | 0 | 0 | 0 | 0 | 0 | | | | | | |
| 4211 | 05003 | 04210 | 1 | | 0 | 0 | 0 | 0 | 0 | 0 | 0 | 0 | 0 | 0 | 0 | 0 | 0 | 0 | 1 | 0 | 0 | 0 | 0 | 0 | 0 | 0 | 0 | 0 | 0 | | | | | | |
| 4212 | 05004 | 04211 | 1 | | 0 | 0 | 0 | 0 | 0 | 0 | 0 | 0 | 0 | 0 | 0 | 0 | 0 | 0 | 1 | 0 | 0 | 0 | 0 | 0 | 0 | 0 | 0 | 0 | 0 | | | | | | |
| 4213 | 05005 | 04212 | 1 | | 0 | 0 | 0 | 0 | 0 | 0 | 0 | 0 | 0 | 0 | 0 | 0 | 0 | 0 | 1 | 0 | 0 | 0 | 0 | 0 | 0 | 0 | 0 | 0 | 0 | | | | | | |
| 4214 | 05006 | 04213 | 8 | | 0 | 0 | 0 | 0 | 0 | 0 | 0 | 0 | 0 | 0 | 1 | 0 | 0 | 3 | 3 | 0 | 0 | 0 | 1 | 0 | 0 | 0 | 0 | 0 | 0 | | | | | | |
| 4215 | 05007 | 04214 | 1 | | 0 | 0 | 0 | 0 | 0 | 0 | 0 | 0 | 0 | 0 | 0 | 0 | 0 | 0 | 1 | 0 | 0 | 0 | 0 | 0 | 0 | 0 | 0 | 0 | 0 | | | | | | |
| 4216 | 05008 | 04215 | 1 | | 0 | 0 | 0 | 0 | 0 | 0 | 0 | 0 | 0 | 0 | 0 | 0 | 0 | 0 | 1 | 0 | 0 | 0 | 0 | 0 | 0 | 0 | 0 | 0 | 0 | | | | | | |
| 4217 | 05009 | 04216 | 1 | | 0 | 0 | 0 | 0 | 0 | 0 | 0 | 0 | 0 | 0 | 0 | 0 | 0 | 0 | 1 | 0 | 0 | 0 | 0 | 0 | 0 | 0 | 0 | 0 | 0 | | | | | | |
| 4218 | 05010 | 04217 | 3 | | 0 | 0 | 0 | 0 | 0 | 0 | 0 | 0 | 0 | 0 | 0 | 0 | 0 | 0 | 3 | 0 | 0 | 0 | 0 | 0 | 0 | 0 | 0 | 0 | 0 | | | | | | |
| 4219 | 05011 | 04218 | 1 | | 0 | 0 | 0 | 0 | 0 | 0 | 0 | 0 | 0 | 0 | 0 | 0 | 0 | 0 | 1 | 0 | 0 | 0 | 0 | 0 | 0 | 0 | 0 | 0 | 0 | | | | | | |
| 4220 | 05013 | 04219 | 14 | | 0 | 0 | 0 | 0 | 0 | 0 | 0 | 0 | 0 | 0 | 0 | 0 | 0 | 0 | 6 | 7 | 1 | 0 | 0 | 0 | 0 | 0 | 0 | 0 | 0 | Y00503 | 96.3 | 164 | 1 | 1197 | 1360 |
| 4221 | 05014 | 04220 | 5 | | 0 | 0 | 0 | 0 | 0 | 0 | 0 | 0 | 0 | 0 | 0 | 0 | 0 | 0 | 1 | 0 | 0 | 0 | 0 | 0 | 0 | 0 | 4 | 0 | 0 | | | | | | |
| 4222 | 05016 | 04221 | 1 | | 0 | 0 | 0 | 0 | 0 | 0 | 0 | 0 | 0 | 0 | 0 | 0 | 0 | 0 | 1 | 0 | 0 | 0 | 0 | 0 | 0 | 0 | 0 | 0 | 0 | M16276 | 98 | 196 | 1 | 1121 | 1763 |
| 4223 | 05018 | 04222 | 1 | | 0 | 0 | 0 | 0 | 0 | 0 | 0 | 0 | 0 | 0 | 0 | 0 | 0 | 0 | 1 | 0 | 0 | 0 | 0 | 0 | 0 | 0 | 0 | 0 | 0 | | | | | | |
| 4224 | 05019 | 04223 | 1 | | 0 | 0 | 0 | 0 | 0 | 0 | 0 | 0 | 0 | 0 | 0 | 0 | 0 | 0 | 1 | 0 | 0 | 0 | 0 | 0 | 0 | 0 | 0 | 0 | 0 | | | | | | |
| 4225 | 05021 | 04224 | 1 | | 0 | 0 | 0 | 0 | 0 | 0 | 0 | 0 | 0 | 0 | 0 | 0 | 0 | 0 | 1 | 0 | 0 | 0 | 0 | 0 | 0 | 0 | 0 | 0 | 0 | L20493 | 97.8 | 269 | 1 | 761 | 1032 |
| 4226 | 05023 | 04225 | 1 | | 0 | 0 | 0 | 0 | 0 | 0 | 0 | 0 | 0 | 0 | 0 | 0 | 0 | 0 | 1 | 0 | 0 | 0 | 0 | 0 | 0 | 0 | 0 | 0 | 0 | | | | | | |
| 4227 | 05024 | 04226 | 4 | | 0 | 0 | 0 | 0 | 0 | 0 | 0 | 0 | 0 | 0 | 0 | 0 | 0 | 1 | 1 | 0 | 0 | 0 | 0 | 0 | 0 | 0 | 0 | 2 | 0 | | | | | | |
| 4228 | 05025 | 04227 | 1 | | 0 | 0 | 0 | 0 | 0 | 0 | 0 | 0 | 0 | 0 | 0 | 0 | 0 | 0 | 1 | 0 | 0 | 0 | 0 | 0 | 0 | 0 | 0 | 0 | 0 | | | | | | |
| 4229 | 05026 | 04228 | 1 | | 0 | 0 | 0 | 0 | 0 | 0 | 0 | 0 | 0 | 0 | 0 | 0 | 0 | 0 | 1 | 0 | 0 | 0 | 0 | 0 | 0 | 0 | 0 | 0 | 0 | | | | | | |
| 4230 | 05027 | 04229 | 1 | | 0 | 0 | 0 | 0 | 0 | 0 | 0 | 0 | 0 | 0 | 0 | 0 | 0 | 0 | 1 | 0 | 0 | 0 | 0 | 0 | 0 | 0 | 0 | 0 | 0 | | | | | | |
| 4231 | 05028 | 04230 | 2 | | 0 | 0 | 0 | 0 | 0 | 0 | 0 | 0 | 0 | 0 | 0 | 0 | 0 | 0 | 1 | 0 | 0 | 1 | 0 | 0 | 0 | 0 | 0 | 0 | 0 | | | | | | |
| 4232 | 05029 | 04231 | 1 | | 0 | 0 | 0 | 0 | 0 | 0 | 0 | 0 | 0 | 0 | 0 | 0 | 0 | 0 | 1 | 0 | 0 | 0 | 0 | 0 | 0 | 0 | 0 | 0 | 0 | | | | | | |
| 4233 | 05030 | 04232 | 2 | | 0 | 0 | 0 | 0 | 0 | 0 | 0 | 0 | 0 | 0 | 0 | 0 | 0 | 0 | 1 | 0 | 1 | 0 | 0 | 0 | 0 | 0 | 0 | 0 | 0 | | | | | | |

Table 118

| | A | B | C | E | G | I | K | M | O | Q | S | U | W | Y | AA | AC | AF | AG | AI | AK | AM | AO | AQ | AS | AU | AW | AY | BA | BC | BE | BF | BG | BH | BI | BK |
|---|---|---|---|---|---|---|---|---|---|---|---|---|---|---|---|---|---|---|---|---|---|---|---|---|---|---|---|---|---|---|---|---|---|---|---|
| 4234 | 05031 | 04233 | 2 | | 0 | 0 | 0 | 0 | 0 | 0 | 0 | 0 | 0 | 0 | 0 | 0 | 0 | 0 | 1 | 0 | 1 | 0 | 0 | 0 | 0 | 0 | 0 | 0 | 0 | | | | | | |
| 4235 | 05033 | 04234 | 1 | | 0 | 0 | 0 | 0 | 0 | 0 | 0 | 0 | 0 | 0 | 0 | 0 | 0 | 0 | 1 | 0 | 0 | 0 | 0 | 0 | 0 | 0 | 0 | 0 | 0 | | | | | | |
| 4236 | 05034 | 04235 | 1 | | 0 | 0 | 0 | 0 | 0 | 0 | 0 | 0 | 0 | 0 | 0 | 0 | 0 | 0 | 1 | 0 | 0 | 0 | 0 | 0 | 0 | 0 | 0 | 0 | 0 | | | | | | |
| 4237 | 05035 | 04236 | 3 | | 0 | 0 | 0 | 0 | 0 | 0 | 0 | 0 | 0 | 0 | 0 | 0 | 0 | 0 | 1 | 0 | 0 | 1 | 0 | 0 | 0 | 0 | 0 | 1 | 0 | M97935 | 98.7 | 312 | 1 | 3429 | 3970 |
| 4238 | 05037 | 04237 | 4 | | 0 | 0 | 0 | 0 | 0 | 0 | 0 | 0 | 0 | 0 | 0 | 0 | 0 | 0 | 4 | 0 | 0 | 0 | 0 | 0 | 0 | 0 | 0 | 0 | 0 | M11233 | 92.4 | 460 | 1 | 1540 | 2038 |
| 4239 | 05039 | 04238 | 1 | | 0 | 0 | 0 | 0 | 0 | 0 | 0 | 0 | 0 | 0 | 0 | 0 | 0 | 0 | 1 | 0 | 0 | 0 | 0 | 0 | 0 | 0 | 0 | 0 | 0 | | | | | | |
| 4240 | 05040 | 04239 | 1 | | 0 | 0 | 0 | 0 | 0 | 0 | 0 | 0 | 0 | 0 | 0 | 0 | 0 | 0 | 1 | 0 | 0 | 0 | 0 | 0 | 0 | 0 | 0 | 0 | 0 | | | | | | |
| 4241 | 05041 | 04240 | 1 | | 0 | 0 | 0 | 0 | 0 | 0 | 0 | 0 | 0 | 0 | 0 | 0 | 0 | 0 | 1 | 0 | 0 | 0 | 0 | 0 | 0 | 0 | 0 | 0 | 0 | | | | | | |
| 4242 | 05042 | 04241 | 1 | | 0 | 0 | 0 | 0 | 0 | 0 | 0 | 0 | 0 | 0 | 0 | 0 | 0 | 0 | 1 | 0 | 0 | 0 | 0 | 0 | 0 | 0 | 0 | 0 | 0 | | | | | | |
| 4243 | 05043 | 04242 | 1 | | 0 | 0 | 0 | 0 | 0 | 0 | 0 | 0 | 0 | 0 | 0 | 0 | 0 | 0 | 1 | 0 | 0 | 0 | 0 | 0 | 0 | 0 | 0 | 0 | 0 | | | | | | |
| 4244 | 05044 | 04243 | 1 | | 0 | 0 | 0 | 0 | 0 | 0 | 0 | 0 | 0 | 0 | 0 | 0 | 0 | 0 | 1 | 0 | 0 | 0 | 0 | 0 | 0 | 0 | 0 | 0 | 0 | X14618 | 98.1 | 321 | 1 | 1043 | 1359 |
| 4245 | 05045 | 04244 | 1 | | 0 | 0 | 0 | 0 | 0 | 0 | 0 | 0 | 0 | 0 | 0 | 0 | 0 | 0 | 1 | 0 | 0 | 0 | 0 | 0 | 0 | 0 | 0 | 0 | 0 | | | | | | |
| 4246 | 05046 | 04245 | 2 | | 0 | 0 | 0 | 0 | 0 | 0 | 0 | 0 | 0 | 0 | 0 | 0 | 0 | 0 | 1 | 0 | 0 | 0 | 0 | 1 | 0 | 0 | 0 | 0 | 0 | | | | | | |
| 4247 | 05047 | 04246 | 1 | | 0 | 0 | 0 | 0 | 0 | 0 | 0 | 0 | 0 | 0 | 0 | 0 | 0 | 0 | 1 | 0 | 0 | 0 | 0 | 0 | 0 | 0 | 0 | 0 | 0 | | | | | | |
| 4248 | 05051 | 04247 | 4 | | 0 | 0 | 0 | 0 | 0 | 0 | 0 | 0 | 0 | 0 | 0 | 0 | 0 | 0 | 1 | 0 | 2 | 0 | 0 | 0 | 0 | 1 | 0 | 0 | 0 | M59911 | 93.1 | 289 | 1 | 4163 | 4637 |
| 4249 | 05052 | 04248 | 4 | | 0 | 0 | 0 | 0 | 0 | 0 | 0 | 0 | 0 | 0 | 0 | 0 | 0 | 0 | 1 | 0 | 1 | 1 | 0 | 0 | 0 | 1 | 0 | 0 | 0 | | | | | | |
| 4250 | 05053 | 04249 | 1 | | 0 | 0 | 0 | 0 | 0 | 0 | 0 | 0 | 0 | 0 | 0 | 0 | 0 | 0 | 1 | 0 | 0 | 0 | 0 | 0 | 0 | 0 | 0 | 0 | 0 | | | | | | |
| 4251 | 05054 | 04250 | 2 | | 0 | 0 | 0 | 0 | 0 | 0 | 0 | 0 | 0 | 0 | 0 | 0 | 0 | 0 | 2 | 0 | 0 | 0 | 0 | 0 | 0 | 0 | 0 | 0 | 0 | | | | | | |
| 4252 | 05055 | 04251 | 1 | | 0 | 0 | 0 | 0 | 0 | 0 | 0 | 0 | 0 | 0 | 0 | 0 | 0 | 0 | 1 | 0 | 0 | 0 | 0 | 0 | 0 | 0 | 0 | 0 | 0 | | | | | | |
| 4253 | 05056 | 04252 | 1 | | 0 | 0 | 0 | 0 | 0 | 0 | 0 | 0 | 0 | 0 | 0 | 0 | 0 | 0 | 1 | 0 | 0 | 0 | 0 | 0 | 0 | 0 | 0 | 0 | 0 | | | | | | |
| 4254 | 05058 | 04253 | 3 | | 0 | 0 | 0 | 0 | 0 | 0 | 0 | 0 | 0 | 0 | 0 | 0 | 0 | 0 | 1 | 0 | 0 | 0 | 0 | 0 | 0 | 1 | 0 | 1 | 0 | | | | | | |
| 4255 | 05059 | 04254 | 3 | | 0 | 0 | 0 | 0 | 0 | 0 | 0 | 0 | 0 | 0 | 0 | 0 | 0 | 0 | 1 | 0 | 0 | 0 | 0 | 0 | 0 | 1 | 0 | 1 | 0 | L03426 | 96.9 | 261 | 1 | 2875 | 3233 |
| 4256 | 05060 | 04255 | 1 | | 0 | 0 | 0 | 0 | 0 | 0 | 0 | 0 | 0 | 0 | 0 | 0 | 0 | 0 | 1 | 0 | 0 | 0 | 0 | 0 | 0 | 0 | 0 | 0 | 0 | | | | | | |
| 4257 | 05061 | 04256 | 1 | | 0 | 0 | 0 | 0 | 0 | 0 | 0 | 0 | 0 | 0 | 0 | 0 | 0 | 0 | 1 | 0 | 0 | 0 | 0 | 0 | 0 | 0 | 0 | 0 | 0 | | | | | | |
| 4258 | 05062 | 04257 | 4 | | 0 | 0 | 0 | 0 | 0 | 0 | 0 | 0 | 0 | 0 | 0 | 0 | 0 | 0 | 2 | 0 | 0 | 0 | 0 | 0 | 0 | 1 | 1 | 0 | 0 | | | | | | |
| 4259 | 05063 | 04258 | 1 | | 0 | 0 | 0 | 0 | 0 | 0 | 0 | 0 | 0 | 0 | 0 | 0 | 0 | 0 | 1 | 0 | 0 | 0 | 0 | 0 | 0 | 0 | 0 | 0 | 0 | | | | | | |
| 4260 | 05064 | 04259 | 1 | | 0 | 0 | 0 | 0 | 0 | 0 | 0 | 0 | 0 | 0 | 0 | 0 | 0 | 0 | 1 | 0 | 0 | 0 | 0 | 0 | 0 | 0 | 0 | 0 | 0 | | | | | | |
| 4261 | 05065 | 04260 | 1 | | 0 | 0 | 0 | 0 | 0 | 0 | 0 | 0 | 0 | 0 | 0 | 0 | 0 | 0 | 1 | 0 | 0 | 0 | 0 | 0 | 0 | 0 | 0 | 0 | 0 | | | | | | |
| 4262 | 05066 | 04261 | 1 | | 0 | 0 | 0 | 0 | 0 | 0 | 0 | 0 | 0 | 0 | 0 | 0 | 0 | 0 | 1 | 0 | 0 | 0 | 0 | 0 | 0 | 0 | 0 | 0 | 0 | | | | | | |
| 4263 | 05068 | 04262 | 5 | | 0 | 0 | 0 | 0 | 0 | 0 | 0 | 0 | 0 | 0 | 0 | 0 | 0 | 0 | 3 | 1 | 0 | 0 | 0 | 0 | 1 | 0 | 0 | 0 | 0 | M87789 | 97.4 | 312 | 1 | 877 | 1599 |
| 4264 | 05069 | 04263 | 1 | | 0 | 0 | 0 | 0 | 0 | 0 | 0 | 0 | 0 | 0 | 0 | 0 | 0 | 0 | 1 | 0 | 0 | 0 | 0 | 0 | 0 | 0 | 0 | 0 | 0 | | | | | | |
| 4265 | 05070 | 04264 | 1 | | 0 | 0 | 0 | 0 | 0 | 0 | 0 | 0 | 0 | 0 | 0 | 0 | 0 | 0 | 1 | 0 | 0 | 0 | 0 | 0 | 0 | 0 | 0 | 0 | 0 | D01096 | 90.8 | 370 | 1 | 1993 | 2359 |
| 4266 | 05071 | 04265 | 4 | | 0 | 0 | 0 | 0 | 0 | 0 | 0 | 0 | 0 | 0 | 0 | 0 | 0 | 0 | 2 | 0 | 0 | 1 | 0 | 0 | 0 | 0 | 0 | 1 | 0 | | | | | | |
| 4267 | 05073 | 04266 | 1 | | 0 | 0 | 0 | 0 | 0 | 0 | 0 | 0 | 0 | 0 | 0 | 0 | 0 | 0 | 1 | 0 | 0 | 0 | 0 | 0 | 0 | 0 | 0 | 0 | 0 | | | | | | |
| 4268 | 05074 | 04267 | 1 | | 0 | 0 | 0 | 0 | 0 | 0 | 0 | 0 | 0 | 0 | 0 | 0 | 0 | 0 | 1 | 0 | 0 | 0 | 0 | 0 | 0 | 0 | 0 | 0 | 0 | | | | | | |
| 4269 | 05075 | 04268 | 1 | | 0 | 0 | 0 | 0 | 0 | 0 | 0 | 0 | 0 | 0 | 0 | 0 | 0 | 0 | 1 | 0 | 0 | 0 | 0 | 0 | 0 | 0 | 0 | 0 | 0 | | | | | | |

Table 119

| | A | B | C | E | G | I | K | M | O | Q | S | U | W | Y | AA | AC | AE | AG | AI | AK | AM | AO | AQ | AS | AU | AW | AY | BA | BC | BE | BF | BG | BH | BI | BK |
|---|---|---|---|---|---|---|---|---|---|---|---|---|---|---|---|---|---|---|---|---|---|---|---|---|---|---|---|---|---|---|---|---|---|---|---|
| 4270 | 05076 | 04269 | 2 | | 0 | 0 | 0 | 0 | 0 | 0 | 0 | 0 | 0 | 0 | 0 | 0 | 0 | 0 | 1 | 1 | 0 | 0 | 0 | 0 | 0 | 0 | 0 | 0 | 0 | | | | | | |
| 4271 | 05077 | 04270 | 1 | | 0 | 0 | 0 | 0 | 0 | 0 | 0 | 0 | 0 | 0 | 0 | 0 | 0 | 0 | 1 | 0 | 0 | 0 | 0 | 0 | 0 | 0 | 0 | 0 | 0 | | | | | | |
| 4272 | 05079 | 04271 | 1 | | 0 | 0 | 0 | 0 | 0 | 0 | 0 | 0 | 0 | 0 | 0 | 0 | 0 | 0 | 1 | 0 | 0 | 0 | 0 | 0 | 0 | 0 | 0 | 0 | 0 | | | | | | |
| 4273 | 05080 | 04272 | 1 | | 0 | 0 | 0 | 0 | 0 | 0 | 0 | 0 | 0 | 0 | 0 | 0 | 0 | 0 | 1 | 0 | 0 | 0 | 0 | 0 | 0 | 0 | 0 | 0 | 0 | | | | | | |
| 4274 | 05081 | 04273 | 2 | | 0 | 0 | 0 | 0 | 0 | 0 | 0 | 0 | 0 | 0 | 0 | 0 | 0 | 0 | 1 | 1 | 0 | 0 | 0 | 0 | 0 | 0 | 0 | 0 | 0 | | | | | | |
| 4275 | 05082 | 04274 | 2 | | 0 | 0 | 0 | 0 | 0 | 0 | 0 | 0 | 0 | 0 | 0 | 0 | 0 | 0 | 2 | 0 | 0 | 0 | 0 | 0 | 0 | 0 | 0 | 0 | 0 | X03067 | 95.1 | 246 | 1 | 815 | 1081 |
| 4276 | 05083 | 04275 | 1 | | 0 | 0 | 0 | 0 | 0 | 0 | 0 | 0 | 0 | 0 | 0 | 0 | 0 | 0 | 1 | 0 | 0 | 0 | 0 | 0 | 0 | 0 | 0 | 0 | 0 | | | | | | |
| 4277 | 05085 | 04276 | 4 | | 0 | 0 | 0 | 0 | 0 | 0 | 0 | 0 | 0 | 0 | 0 | 0 | 0 | 0 | 2 | 0 | 0 | 1 | 0 | 0 | 0 | 0 | 1 | 0 | 0 | | | | | | |
| 4278 | 05086 | 04277 | 2 | | 0 | 0 | 0 | 0 | 0 | 0 | 0 | 0 | 0 | 0 | 0 | 0 | 0 | 0 | 1 | 0 | 0 | 0 | 0 | 0 | 1 | 0 | 0 | 0 | 0 | | | | | | |
| 4279 | 05087 | 04278 | 1 | | 0 | 0 | 0 | 0 | 0 | 0 | 0 | 0 | 0 | 0 | 0 | 0 | 0 | 0 | 1 | 0 | 0 | 0 | 0 | 0 | 0 | 0 | 0 | 0 | 0 | | | | | | |
| 4280 | 05088 | 04279 | 1 | | 0 | 0 | 0 | 0 | 0 | 0 | 0 | 0 | 0 | 0 | 0 | 0 | 0 | 0 | 1 | 0 | 0 | 0 | 0 | 0 | 0 | 0 | 0 | 0 | 0 | | | | | | |
| 4281 | 05089 | 04280 | 1 | | 0 | 0 | 0 | 0 | 0 | 0 | 0 | 0 | 0 | 0 | 0 | 0 | 0 | 0 | 1 | 0 | 0 | 0 | 0 | 0 | 0 | 0 | 0 | 0 | 0 | | | | | | |
| 4282 | 05090 | 04281 | 2 | | 0 | 0 | 0 | 0 | 0 | 0 | 0 | 0 | 0 | 0 | 0 | 0 | 0 | 0 | 1 | 0 | 0 | 0 | 0 | 0 | 0 | 0 | 0 | 1 | 0 | | | | | | |
| 4283 | 05092 | 04282 | 1 | | 0 | 0 | 0 | 0 | 0 | 0 | 0 | 0 | 0 | 0 | 0 | 0 | 0 | 0 | 1 | 0 | 0 | 0 | 0 | 0 | 0 | 0 | 0 | 0 | 0 | | | | | | |
| 4284 | 05094 | 04283 | 1 | | 0 | 0 | 0 | 0 | 0 | 0 | 0 | 0 | 0 | 0 | 0 | 0 | 0 | 0 | 1 | 0 | 0 | 0 | 0 | 0 | 0 | 0 | 0 | 0 | 0 | | | | | | |
| 4285 | 05095 | 04284 | 1 | | 0 | 0 | 0 | 0 | 0 | 0 | 0 | 0 | 0 | 0 | 0 | 0 | 0 | 0 | 1 | 0 | 0 | 0 | 0 | 0 | 0 | 0 | 0 | 0 | 0 | | | | | | |
| 4286 | 05096 | 04285 | 1 | | 0 | 0 | 0 | 0 | 0 | 0 | 0 | 0 | 0 | 0 | 0 | 0 | 0 | 0 | 1 | 0 | 0 | 0 | 0 | 0 | 0 | 0 | 0 | 0 | 0 | J03241 | 97.9 | 290 | 1 | 2235 | 2529 |
| 4287 | 05099 | 04286 | 2 | | 0 | 0 | 0 | 0 | 0 | 0 | 0 | 0 | 0 | 0 | 0 | 0 | 0 | 1 | 1 | 0 | 0 | 0 | 0 | 0 | 0 | 0 | 0 | 0 | 0 | | | | | | |
| 4288 | 05100 | 04287 | 2 | | 0 | 0 | 0 | 0 | 0 | 0 | 0 | 0 | 0 | 0 | 0 | 0 | 0 | 0 | 1 | 0 | 1 | 0 | 0 | 0 | 0 | 0 | 0 | 0 | 0 | | | | | | |
| 4289 | 05102 | 04288 | 1 | | 0 | 0 | 0 | 0 | 0 | 0 | 0 | 0 | 0 | 0 | 0 | 0 | 0 | 0 | 1 | 0 | 0 | 0 | 0 | 0 | 0 | 0 | 0 | 0 | 0 | | | | | | |
| 4290 | 05103 | 04289 | 5 | | 0 | 0 | 0 | 0 | 0 | 0 | 0 | 0 | 0 | 0 | 0 | 0 | 0 | 0 | 2 | 0 | 0 | 0 | 0 | 0 | 0 | 0 | 3 | 0 | 0 | X69819 | 100 | 69 | 1 | 1666 | 1817 |
| 4291 | 05104 | 04290 | 1 | | 0 | 0 | 0 | 0 | 0 | 0 | 0 | 0 | 0 | 0 | 0 | 0 | 0 | 0 | 1 | 0 | 0 | 0 | 0 | 0 | 0 | 0 | 0 | 0 | 0 | | | | | | |
| 4292 | 05105 | 04291 | 3 | | 0 | 0 | 0 | 0 | 0 | 0 | 0 | 0 | 0 | 0 | 0 | 0 | 0 | 0 | 1 | 0 | 1 | 0 | 0 | 0 | 0 | 0 | 1 | 0 | 0 | | | | | | |
| 4293 | 05106 | 04292 | 3 | | 0 | 0 | 0 | 0 | 0 | 0 | 0 | 0 | 0 | 0 | 0 | 0 | 0 | 0 | 1 | 0 | 0 | 0 | 1 | 0 | 0 | 0 | 0 | 1 | 0 | | | | | | |
| 4294 | 05107 | 04293 | 2 | | 0 | 0 | 0 | 0 | 0 | 0 | 0 | 0 | 0 | 0 | 0 | 0 | 0 | 1 | 1 | 0 | 0 | 0 | 0 | 0 | 0 | 0 | 0 | 0 | 0 | | | | | | |
| 4295 | 05108 | 04294 | 12 | | 0 | 0 | 0 | 0 | 0 | 0 | 0 | 0 | 0 | 0 | 0 | 0 | 0 | 0 | 3 | 1 | 0 | 1 | 0 | 1 | 1 | 5 | 0 | 0 | 0 | Z23090 | 96.6 | 206 | 1 | 1025 | 1231 |
| 4296 | 05109 | 04295 | 1 | | 0 | 0 | 0 | 0 | 0 | 0 | 0 | 0 | 0 | 0 | 0 | 0 | 0 | 0 | 1 | 0 | 0 | 0 | 0 | 0 | 0 | 0 | 0 | 0 | 0 | X56510 | 90 | 331 | 1 | 83 | 426 |
| 4297 | 05110 | 04296 | 1 | | 0 | 0 | 0 | 0 | 0 | 0 | 0 | 0 | 0 | 0 | 0 | 0 | 0 | 0 | 1 | 0 | 0 | 0 | 0 | 0 | 0 | 0 | 0 | 0 | 0 | | | | | | |
| 4298 | 05111 | 04297 | 16 | | 0 | 0 | 0 | 0 | 0 | 0 | 0 | 0 | 0 | 0 | 0 | 0 | 0 | 0 | 13 | 3 | 0 | 0 | 0 | 0 | 0 | 0 | 0 | 0 | 0 | M63438 | 94.1 | 374 | 1 | 720 | 1244 |
| 4299 | 05112 | 04298 | 1 | | 0 | 0 | 0 | 0 | 0 | 0 | 0 | 0 | 0 | 0 | 0 | 0 | 0 | 0 | 1 | 0 | 0 | 0 | 0 | 0 | 0 | 0 | 0 | 0 | 0 | | | | | | |
| 4300 | 05113 | 04299 | 1 | | 0 | 0 | 0 | 0 | 0 | 0 | 0 | 0 | 0 | 0 | 0 | 0 | 0 | 0 | 1 | 0 | 0 | 0 | 0 | 0 | 0 | 0 | 0 | 0 | 0 | | | | | | |
| 4301 | 05114 | 04300 | 1 | | 0 | 0 | 0 | 0 | 0 | 0 | 0 | 0 | 0 | 0 | 0 | 0 | 0 | 0 | 1 | 0 | 0 | 0 | 0 | 0 | 0 | 0 | 0 | 0 | 0 | | | | | | |
| 4302 | 05115 | 04301 | 2 | | 0 | 0 | 0 | 0 | 0 | 0 | 0 | 0 | 0 | 0 | 0 | 0 | 0 | 0 | 2 | 0 | 0 | 0 | 0 | 0 | 0 | 0 | 0 | 0 | 0 | | | | | | |
| 4303 | 05116 | 04302 | 1 | | 0 | 0 | 0 | 0 | 0 | 0 | 0 | 0 | 0 | 0 | 0 | 0 | 0 | 0 | 1 | 0 | 0 | 0 | 0 | 0 | 0 | 0 | 0 | 0 | 0 | | | | | | |
| 4304 | 05117 | 04303 | 1 | | 0 | 0 | 0 | 0 | 0 | 0 | 0 | 0 | 0 | 0 | 0 | 0 | 0 | 0 | 1 | 0 | 0 | 0 | 0 | 0 | 0 | 0 | 0 | 0 | 0 | | | | | | |
| 4305 | 05118 | 04304 | 2 | | 0 | 0 | 0 | 0 | 0 | 0 | 0 | 0 | 0 | 0 | 0 | 0 | 0 | 0 | 2 | 0 | 0 | 0 | 0 | 0 | 0 | 0 | 0 | 0 | 0 | | | | | | |

Table 120

| | A | B | C | E | G | I | K | M | O | Q | S | U | W | Y | AA | AC | AE | AG | AI | AK | AM | AO | AQ | AS | AU | AW | AY | BA | BC | BE | BF | BG | BH | BI | BK |
|---|---|---|---|---|---|---|---|---|---|---|---|---|---|---|---|---|---|---|---|---|---|---|---|---|---|---|---|---|---|---|---|---|---|---|---|
| 4306 | 05119 | 04305 | 1 | | 0 | 0 | 0 | 0 | 0 | 0 | 0 | 0 | 0 | 0 | 0 | 0 | 0 | 0 | 1 | 0 | 0 | 0 | 0 | 0 | 0 | 0 | 0 | 0 | 0 | | | | | | |
| 4307 | 05120 | 04306 | 2 | | 0 | 0 | 0 | 0 | 0 | 0 | 0 | 0 | 0 | 0 | 0 | 0 | 0 | 0 | 1 | 0 | 0 | 0 | 0 | 1 | 0 | 0 | 0 | 0 | 0 | | | | | | |
| 4308 | 05121 | 04307 | 3 | | 0 | 0 | 0 | 0 | 0 | 0 | 0 | 0 | 0 | 0 | 0 | 0 | 0 | 0 | 2 | 0 | 0 | 0 | 0 | 1 | 0 | 0 | 0 | 0 | 0 | | | | | | |
| 4309 | 05122 | 04308 | 1 | | 0 | 0 | 0 | 0 | 0 | 0 | 0 | 0 | 0 | 0 | 0 | 0 | 0 | 0 | 1 | 0 | 0 | 0 | 0 | 0 | 0 | 0 | 0 | 0 | 0 | | | | | | |
| 4310 | 05123 | 04309 | 2 | | 0 | 0 | 0 | 0 | 0 | 0 | 0 | 0 | 0 | 0 | 0 | 0 | 0 | 0 | 1 | 0 | 0 | 0 | 0 | 0 | 0 | 1 | 0 | 0 | 0 | | | | | | |
| 4311 | 05124 | 04310 | 3 | | 0 | 0 | 0 | 0 | 0 | 0 | 0 | 0 | 0 | 0 | 0 | 0 | 0 | 0 | 1 | 0 | 0 | 0 | 1 | 0 | 0 | 0 | 1 | 0 | 0 | | | | | | |
| 4312 | 05127 | 04311 | 1 | | 0 | 0 | 0 | 0 | 0 | 0 | 0 | 0 | 0 | 0 | 0 | 0 | 0 | 0 | 1 | 0 | 0 | 0 | 0 | 0 | 0 | 0 | 0 | 0 | 0 | | | | | | |
| 4313 | 05128 | 04312 | 1 | | 0 | 0 | 0 | 0 | 0 | 0 | 0 | 0 | 0 | 0 | 0 | 0 | 0 | 0 | 1 | 0 | 0 | 0 | 0 | 0 | 0 | 0 | 0 | 0 | 0 | | | | | | |
| 4314 | 05129 | 04313 | 2 | | 0 | 0 | 0 | 0 | 0 | 0 | 0 | 0 | 0 | 0 | 0 | 0 | 0 | 0 | 1 | 0 | 0 | 1 | 0 | 0 | 0 | 0 | 0 | 0 | 0 | | | | | | |
| 4315 | 05130 | 04314 | 3 | | 0 | 0 | 0 | 0 | 0 | 0 | 0 | 0 | 0 | 0 | 0 | 0 | 0 | 0 | 1 | 0 | 1 | 0 | 0 | 1 | 0 | 0 | 0 | 0 | 0 | | | | | | |
| 4316 | 05132 | 04315 | 2 | | 0 | 0 | 0 | 0 | 0 | 0 | 0 | 0 | 0 | 0 | 0 | 0 | 0 | 0 | 1 | 0 | 0 | 0 | 0 | 0 | 1 | 0 | 0 | 0 | 0 | | | | | | |
| 4317 | 05133 | 04316 | 1 | | 0 | 0 | 0 | 0 | 0 | 0 | 0 | 0 | 0 | 0 | 0 | 0 | 0 | 0 | 1 | 0 | 0 | 0 | 0 | 0 | 0 | 0 | 0 | 0 | 0 | | | | | | |
| 4318 | 05134 | 04317 | 1 | | 0 | 0 | 0 | 0 | 0 | 0 | 0 | 0 | 0 | 0 | 0 | 0 | 0 | 0 | 1 | 0 | 0 | 0 | 0 | 0 | 0 | 0 | 0 | 0 | 0 | | | | | | |
| 4319 | 05135 | 04318 | 1 | | 0 | 0 | 0 | 0 | 0 | 0 | 0 | 0 | 0 | 0 | 0 | 0 | 0 | 0 | 1 | 0 | 0 | 0 | 0 | 0 | 0 | 0 | 0 | 0 | 0 | | | | | | |
| 4320 | 05136 | 04319 | 1 | | 0 | 0 | 0 | 0 | 0 | 0 | 0 | 0 | 0 | 0 | 0 | 0 | 0 | 0 | 1 | 0 | 0 | 0 | 0 | 0 | 0 | 0 | 0 | 0 | 0 | | | | | | |
| 4321 | 05137 | 04320 | 1 | | 0 | 0 | 0 | 0 | 0 | 0 | 0 | 0 | 0 | 0 | 0 | 0 | 0 | 0 | 1 | 0 | 0 | 0 | 0 | 0 | 0 | 0 | 0 | 0 | 0 | | | | | | |
| 4322 | 05138 | 04321 | 2 | | 0 | 0 | 0 | 0 | 0 | 0 | 0 | 0 | 0 | 0 | 0 | 0 | 0 | 0 | 2 | 0 | 0 | 0 | 0 | 0 | 0 | 0 | 0 | 0 | 0 | | | | | | |
| 4323 | 05139 | 04322 | 1 | | 0 | 0 | 0 | 0 | 0 | 0 | 0 | 0 | 0 | 0 | 0 | 0 | 0 | 0 | 1 | 0 | 0 | 0 | 0 | 0 | 0 | 0 | 0 | 0 | 0 | | | | | | |
| 4324 | 05140 | 04323 | 1 | | 0 | 0 | 0 | 0 | 0 | 0 | 0 | 0 | 0 | 0 | 0 | 0 | 0 | 0 | 1 | 0 | 0 | 0 | 0 | 0 | 0 | 0 | 0 | 0 | 0 | | | | | | |
| 4325 | 05141 | 04324 | 1 | | 0 | 0 | 0 | 0 | 0 | 0 | 0 | 0 | 0 | 0 | 0 | 0 | 0 | 0 | 1 | 0 | 0 | 0 | 0 | 0 | 0 | 0 | 0 | 0 | 0 | | | | | | |
| 4326 | 05142 | 04325 | 2 | | 0 | 0 | 0 | 0 | 0 | 0 | 0 | 0 | 0 | 0 | 0 | 0 | 0 | 0 | 2 | 0 | 0 | 0 | 0 | 0 | 0 | 0 | 0 | 0 | 0 | X59445 | 98.9 | 88 | 1 | 735 | 969 |
| 4327 | 05143 | 04326 | 2 | | 0 | 0 | 0 | 0 | 0 | 0 | 0 | 0 | 0 | 0 | 0 | 0 | 0 | 0 | 1 | 0 | 0 | 0 | 0 | 0 | 0 | 0 | 1 | 0 | 0 | | | | | | |
| 4328 | 05144 | 04327 | 3 | | 0 | 0 | 0 | 0 | 0 | 0 | 0 | 0 | 0 | 0 | 2 | 0 | 0 | 0 | 1 | 0 | 0 | 0 | 0 | 0 | 0 | 0 | 0 | 0 | 0 | | | | | | |
| 4329 | 05145 | 04328 | 2 | | 0 | 0 | 0 | 0 | 0 | 0 | 0 | 0 | 0 | 0 | 0 | 0 | 0 | 0 | 1 | 0 | 0 | 1 | 0 | 0 | 0 | 0 | 0 | 0 | 0 | | | | | | |
| 4330 | 05147 | 04329 | 2 | | 0 | 0 | 0 | 0 | 0 | 0 | 0 | 0 | 0 | 0 | 0 | 0 | 0 | 0 | 1 | 1 | 0 | 0 | 0 | 0 | 0 | 0 | 0 | 0 | 0 | | | | | | |
| 4331 | 05148 | 04330 | 1 | | 0 | 0 | 0 | 0 | 0 | 0 | 0 | 0 | 0 | 0 | 0 | 0 | 0 | 0 | 1 | 0 | 0 | 0 | 0 | 0 | 0 | 0 | 0 | 0 | 0 | | | | | | |
| 4332 | 05149 | 04331 | 1 | | 0 | 0 | 0 | 0 | 0 | 0 | 0 | 0 | 0 | 0 | 0 | 0 | 0 | 0 | 1 | 0 | 0 | 0 | 0 | 0 | 0 | 0 | 0 | 0 | 0 | | | | | | |
| 4333 | 05150 | 04332 | 1 | | 0 | 0 | 0 | 0 | 0 | 0 | 0 | 0 | 0 | 0 | 0 | 0 | 0 | 0 | 1 | 0 | 0 | 0 | 0 | 0 | 0 | 0 | 0 | 0 | 0 | | | | | | |
| 4334 | 05151 | 04333 | 1 | | 0 | 0 | 0 | 0 | 0 | 0 | 0 | 0 | 0 | 0 | 0 | 0 | 0 | 0 | 1 | 0 | 0 | 0 | 0 | 0 | 0 | 0 | 0 | 0 | 0 | L23808 | 99.2 | 250 | 1 | 1346 | 1778 |
| 4335 | 05152 | 04334 | 1 | | 0 | 0 | 0 | 0 | 0 | 0 | 0 | 0 | 0 | 0 | 0 | 0 | 0 | 0 | 1 | 0 | 0 | 0 | 0 | 0 | 0 | 0 | 0 | 0 | 0 | | | | | | |
| 4336 | 05153 | 04335 | 1 | | 0 | 0 | 0 | 0 | 0 | 0 | 0 | 0 | 0 | 0 | 0 | 0 | 0 | 0 | 1 | 0 | 0 | 0 | 0 | 0 | 0 | 0 | 0 | 0 | 0 | | | | | | |
| 4337 | 05155 | 04336 | 1 | | 0 | 0 | 0 | 0 | 0 | 0 | 0 | 0 | 0 | 0 | 0 | 0 | 0 | 0 | 1 | 0 | 0 | 0 | 0 | 0 | 0 | 0 | 0 | 0 | 0 | | | | | | |
| 4338 | 05157 | 04337 | 3 | | 0 | 0 | 0 | 0 | 0 | 0 | 0 | 0 | 0 | 0 | 0 | 0 | 0 | 0 | 1 | 0 | 0 | 0 | 0 | 0 | 0 | 0 | 2 | 0 | 0 | | | | | | |
| 4339 | 05158 | 04338 | 1 | | 0 | 0 | 0 | 0 | 0 | 0 | 0 | 0 | 0 | 0 | 0 | 0 | 0 | 0 | 1 | 0 | 0 | 0 | 0 | 0 | 0 | 0 | 0 | 0 | 0 | | | | | | |
| 4340 | 05159 | 04339 | 1 | | 0 | 0 | 0 | 0 | 0 | 0 | 0 | 0 | 0 | 0 | 0 | 0 | 0 | 0 | 1 | 0 | 0 | 0 | 0 | 0 | 0 | 0 | 0 | 0 | 0 | | | | | | |
| 4341 | 05160 | 04340 | 1 | | 0 | 0 | 0 | 0 | 0 | 0 | 0 | 0 | 0 | 0 | 0 | 0 | 0 | 0 | 1 | 0 | 0 | 0 | 0 | 0 | 0 | 0 | 0 | 0 | 0 | | | | | | |

Table 121

| | A | B | C | E | G | I | K | M | O | Q | S | U | W | Y | AA | AC | AE | AG | AI | AK | AM | AO | AQ | AS | AU | AW | AY | BA | BC | BE | BF | BG | BH | BI | BK |
|---|---|---|---|---|---|---|---|---|---|---|---|---|---|---|---|---|---|---|---|---|---|---|---|---|---|---|---|---|---|---|---|---|---|---|---|
| 4342 | 05161 | 04341 | 2 | | 0 | 0 | 0 | 0 | 0 | 0 | 0 | 0 | 0 | 0 | 0 | 0 | 0 | 0 | 2 | 0 | 0 | 0 | 0 | 0 | 0 | 0 | 0 | 0 | 0 | | | | | | |
| 4343 | 05162 | 04342 | 1 | | 0 | 0 | 0 | 0 | 0 | 0 | 0 | 0 | 0 | 0 | 0 | 0 | 0 | 0 | 1 | 0 | 0 | 0 | 0 | 0 | 0 | 0 | 0 | 0 | 0 | | | | | | |
| 4344 | 05163 | 04343 | 1 | | 0 | 0 | 0 | 0 | 0 | 0 | 0 | 0 | 0 | 0 | 0 | 0 | 0 | 0 | 1 | 0 | 0 | 0 | 0 | 0 | 0 | 0 | 0 | 0 | 0 | | | | | | |
| 4345 | 05164 | 04344 | 1 | | 0 | 0 | 0 | 0 | 0 | 0 | 0 | 0 | 0 | 0 | 0 | 0 | 0 | 0 | 1 | 0 | 0 | 0 | 0 | 0 | 0 | 0 | 0 | 0 | 0 | | | | | | |
| 4346 | 05165 | 04345 | 1 | | 0 | 0 | 0 | 0 | 0 | 0 | 0 | 0 | 0 | 0 | 0 | 0 | 0 | 0 | 1 | 0 | 0 | 0 | 0 | 0 | 0 | 0 | 0 | 0 | 0 | | | | | | |
| 4347 | 05166 | 04346 | 2 | | 0 | 0 | 0 | 0 | 0 | 0 | 0 | 0 | 0 | 0 | 0 | 0 | 0 | 0 | 1 | 0 | 0 | 0 | 0 | 1 | 0 | 0 | 0 | 0 | 0 | | | | | | |
| 4348 | 05168 | 04347 | 3 | | 0 | 0 | 0 | 0 | 0 | 0 | 0 | 0 | 0 | 0 | 0 | 0 | 0 | 2 | 1 | 0 | 0 | 0 | 0 | 0 | 0 | 0 | 0 | 0 | 0 | | | | | | |
| 4349 | 05169 | 04348 | 1 | | 0 | 0 | 0 | 0 | 0 | 0 | 0 | 0 | 0 | 0 | 0 | 0 | 0 | 0 | 1 | 0 | 0 | 0 | 0 | 0 | 0 | 0 | 0 | 0 | 0 | | | | | | |
| 4350 | 05170 | 04349 | 1 | | 0 | 0 | 0 | 0 | 0 | 0 | 0 | 0 | 0 | 0 | 0 | 0 | 0 | 0 | 1 | 0 | 0 | 0 | 0 | 0 | 0 | 0 | 0 | 0 | 0 | | | | | | |
| 4351 | 05172 | 04350 | 1 | | 0 | 0 | 0 | 0 | 0 | 0 | 0 | 0 | 0 | 0 | 0 | 0 | 0 | 0 | 1 | 0 | 0 | 0 | 0 | 0 | 0 | 0 | 0 | 0 | 0 | | | | | | |
| 4352 | 05173 | 04351 | 1 | | 0 | 0 | 0 | 0 | 0 | 0 | 0 | 0 | 0 | 0 | 0 | 0 | 0 | 0 | 1 | 0 | 0 | 0 | 0 | 0 | 0 | 0 | 0 | 0 | 0 | | | | | | |
| 4353 | 05174 | 04352 | 1 | | 0 | 0 | 0 | 0 | 0 | 0 | 0 | 0 | 0 | 0 | 0 | 0 | 0 | 0 | 1 | 0 | 0 | 0 | 0 | 0 | 0 | 0 | 0 | 0 | 0 | | | | | | |
| 4354 | 05176 | 04353 | 1 | | 0 | 0 | 0 | 0 | 0 | 0 | 0 | 0 | 0 | 0 | 0 | 0 | 0 | 0 | 1 | 0 | 0 | 0 | 0 | 0 | 0 | 0 | 0 | 0 | 0 | | | | | | |
| 4355 | 05177 | 04354 | 6 | | 0 | 0 | 0 | 0 | 0 | 0 | 0 | 0 | 0 | 0 | 0 | 0 | 0 | 0 | 1 | 2 | 0 | 1 | 0 | 0 | 0 | 1 | 1 | 0 | 0 | | | | | | |
| 4356 | 05178 | 04355 | 1 | | 0 | 0 | 0 | 0 | 0 | 0 | 0 | 0 | 0 | 0 | 0 | 0 | 0 | 0 | 1 | 0 | 0 | 0 | 0 | 0 | 0 | 0 | 0 | 0 | 0 | | | | | | |
| 4357 | 05180 | 04356 | 1 | | 0 | 0 | 0 | 0 | 0 | 0 | 0 | 0 | 0 | 0 | 0 | 0 | 0 | 0 | 1 | 0 | 0 | 0 | 0 | 0 | 0 | 0 | 0 | 0 | 0 | | | | | | |
| 4358 | 05181 | 04357 | 1 | | 0 | 0 | 0 | 0 | 0 | 0 | 0 | 0 | 0 | 0 | 0 | 0 | 0 | 0 | 1 | 0 | 0 | 0 | 0 | 0 | 0 | 0 | 0 | 0 | 0 | | | | | | |
| 4359 | 05182 | 04358 | 2 | | 0 | 0 | 0 | 0 | 0 | 0 | 0 | 0 | 0 | 0 | 0 | 0 | 0 | 0 | 1 | 0 | 0 | 0 | 1 | 0 | 0 | 0 | 0 | 0 | 0 | | | | | | |
| 4360 | 05183 | 04359 | 1 | | 0 | 0 | 0 | 0 | 0 | 0 | 0 | 0 | 0 | 0 | 0 | 0 | 0 | 0 | 1 | 0 | 0 | 0 | 0 | 0 | 0 | 0 | 0 | 0 | 0 | | | | | | |
| 4361 | 05184 | 04360 | 2 | | 0 | 0 | 0 | 0 | 0 | 0 | 0 | 0 | 0 | 0 | 0 | 0 | 0 | 0 | 1 | 1 | 0 | 0 | 0 | 0 | 0 | 0 | 0 | 0 | 0 | | | | | | |
| 4362 | 05185 | 04361 | 1 | | 0 | 0 | 0 | 0 | 0 | 0 | 0 | 0 | 0 | 0 | 0 | 0 | 0 | 0 | 1 | 0 | 0 | 0 | 0 | 0 | 0 | 0 | 0 | 0 | 0 | | | | | | |
| 4363 | 05186 | 04362 | 1 | | 0 | 0 | 0 | 0 | 0 | 0 | 0 | 0 | 0 | 0 | 0 | 0 | 0 | 0 | 1 | 0 | 0 | 0 | 0 | 0 | 0 | 0 | 0 | 0 | 0 | | | | | | |
| 4364 | 05187 | 04363 | 1 | | 0 | 0 | 0 | 0 | 0 | 0 | 0 | 0 | 0 | 0 | 0 | 0 | 0 | 0 | 1 | 0 | 0 | 0 | 0 | 0 | 0 | 0 | 0 | 0 | 0 | | | | | | |
| 4365 | 05188 | 04364 | 3 | | 0 | 0 | 0 | 0 | 0 | 0 | 0 | 0 | 0 | 0 | 0 | 0 | 0 | 0 | 1 | 0 | 0 | 2 | 0 | 0 | 0 | 0 | 0 | 0 | 0 | | | | | | |
| 4366 | 05190 | 04365 | 1 | | 0 | 0 | 0 | 0 | 0 | 0 | 0 | 0 | 0 | 0 | 0 | 0 | 0 | 0 | 1 | 0 | 0 | 0 | 0 | 0 | 0 | 0 | 0 | 0 | 0 | | | | | | |
| 4367 | 05191 | 04366 | 1 | | 0 | 0 | 0 | 0 | 0 | 0 | 0 | 0 | 0 | 0 | 0 | 0 | 0 | 0 | 1 | 0 | 0 | 0 | 0 | 0 | 0 | 0 | 0 | 0 | 0 | | | | | | |
| 4368 | 05192 | 04367 | 1 | | 0 | 0 | 0 | 0 | 0 | 0 | 0 | 0 | 0 | 0 | 0 | 0 | 0 | 0 | 1 | 0 | 0 | 0 | 0 | 0 | 0 | 0 | 0 | 0 | 0 | | | | | | |
| 4369 | 05194 | 04368 | 2 | | 0 | 0 | 0 | 0 | 0 | 0 | 0 | 0 | 0 | 0 | 0 | 0 | 0 | 0 | 1 | 0 | 0 | 0 | 1 | 0 | 0 | 0 | 0 | 0 | 0 | | | | | | |
| 4370 | 05195 | 04369 | 2 | | 0 | 0 | 0 | 0 | 0 | 0 | 0 | 0 | 0 | 0 | 0 | 0 | 0 | 0 | 2 | 0 | 0 | 0 | 0 | 0 | 0 | 0 | 0 | 0 | 0 | L19597 | 90.9 | 55 | 1 | 3459 | 3511 |
| 4371 | 05196 | 04370 | 1 | | 0 | 0 | 0 | 0 | 0 | 0 | 0 | 0 | 0 | 0 | 0 | 0 | 0 | 0 | 1 | 0 | 0 | 0 | 0 | 0 | 0 | 0 | 0 | 0 | 0 | | | | | | |
| 4372 | 05197 | 04371 | 1 | | 0 | 0 | 0 | 0 | 0 | 0 | 0 | 0 | 0 | 0 | 0 | 0 | 0 | 0 | 1 | 0 | 0 | 0 | 0 | 0 | 0 | 0 | 0 | 0 | 0 | | | | | | |
| 4373 | 05198 | 04372 | 2 | | 0 | 0 | 0 | 0 | 0 | 0 | 0 | 0 | 0 | 0 | 0 | 0 | 0 | 0 | 1 | 0 | 0 | 0 | 0 | 1 | 0 | 0 | 0 | 0 | 0 | | | | | | |
| 4374 | 05199 | 04373 | 1 | | 0 | 0 | 0 | 0 | 0 | 0 | 0 | 0 | 0 | 0 | 0 | 0 | 0 | 0 | 1 | 0 | 0 | 0 | 0 | 0 | 0 | 0 | 0 | 0 | 0 | L10284 | 100 | 51 | 1 | 3826 | 4117 |
| 4375 | 05200 | 04374 | 1 | | 0 | 0 | 0 | 0 | 0 | 0 | 0 | 0 | 0 | 0 | 0 | 0 | 0 | 0 | 1 | 0 | 0 | 0 | 0 | 0 | 0 | 0 | 0 | 0 | 0 | | | | | | |
| 4376 | 05201 | 04375 | 3 | | 0 | 0 | 0 | 0 | 0 | 0 | 0 | 0 | 0 | 0 | 0 | 0 | 0 | 0 | 1 | 0 | 0 | 0 | 0 | 0 | 0 | 2 | 0 | 0 | 0 | | | | | | |
| 4377 | 05203 | 04376 | 1 | | 0 | 0 | 0 | 0 | 0 | 0 | 0 | 0 | 0 | 0 | 0 | 0 | 0 | 0 | 1 | 0 | 0 | 0 | 0 | 0 | 0 | 0 | 0 | 0 | 0 | | | | | | |

Table 122

| | A | B | C | E | G | I | K | M | O | Q | S | U | W | Y | AA | AC | AE | AG | AI | AK | AM | AO | AQ | AS | AU | AW | AY | BA | BC | BE | BF | BG | BH | BI | BK |
|---|---|---|---|---|---|---|---|---|---|---|---|---|---|---|---|---|---|---|---|---|---|---|---|---|---|---|---|---|---|---|---|---|---|---|---|
| 4378 | 05204 | 04377 | 1 | | 0 | 0 | 0 | 0 | 0 | 0 | 0 | 0 | 0 | 0 | 0 | 0 | 0 | 0 | 1 | 0 | 0 | 0 | 0 | 0 | 0 | 0 | 0 | 0 | 0 | | | | | | |
| 4379 | 05205 | 04378 | 2 | | 0 | 0 | 0 | 0 | 0 | 0 | 0 | 0 | 0 | 0 | 0 | 0 | 0 | 1 | 1 | 0 | 0 | 0 | 0 | 0 | 0 | 0 | 0 | 0 | 0 | | | | | | |
| 4380 | 05206 | 04379 | 2 | | 0 | 0 | 0 | 0 | 0 | 0 | 0 | 0 | 0 | 0 | 0 | 0 | 0 | 0 | 1 | 0 | 0 | 0 | 0 | 0 | 0 | 0 | 0 | 1 | 0 | | | | | | |
| 4381 | 05207 | 04380 | 3 | | 0 | 0 | 0 | 0 | 0 | 0 | 0 | 0 | 0 | 0 | 0 | 0 | 0 | 0 | 1 | 1 | 1 | 0 | 0 | 0 | 0 | 0 | 0 | 0 | 0 | | | | | | |
| 4382 | 05208 | 04381 | 3 | | 0 | 0 | 0 | 0 | 0 | 0 | 0 | 0 | 0 | 0 | 0 | 0 | 0 | 0 | 1 | 0 | 0 | 2 | 0 | 0 | 0 | 0 | 0 | 0 | 0 | | | | | | |
| 4383 | 05209 | 04382 | 8 | | 0 | 0 | 0 | 0 | 0 | 0 | 0 | 0 | 0 | 0 | 0 | 0 | 0 | 0 | 1 | 0 | 0 | 0 | 0 | 1 | 0 | 0 | 6 | 0 | 0 | | | | | | |
| 4384 | 05210 | 04383 | 2 | | 0 | 0 | 0 | 0 | 0 | 0 | 0 | 0 | 0 | 0 | 0 | 0 | 0 | 0 | 1 | 0 | 0 | 0 | 0 | 0 | 0 | 0 | 1 | 0 | 0 | | | | | | |
| 4385 | 05211 | 04384 | 4 | | 0 | 0 | 0 | 0 | 0 | 0 | 0 | 0 | 0 | 0 | 0 | 0 | 0 | 0 | 1 | 0 | 0 | 1 | 0 | 0 | 2 | 0 | 0 | 0 | 0 | M12125 | 98.4 | 254 | 1 | 790 | 1044 |
| 4386 | 05212 | 04385 | 6 | | 0 | 0 | 0 | 0 | 0 | 0 | 0 | 0 | 0 | 0 | 0 | 0 | 0 | 0 | 1 | 1 | 0 | 0 | 0 | 0 | 0 | 2 | 0 | 0 | 2 | | | | | | |
| 4387 | 05213 | 04386 | 3 | | 0 | 0 | 0 | 0 | 0 | 0 | 0 | 0 | 0 | 0 | 2 | 0 | 0 | 0 | 1 | 0 | 0 | 0 | 0 | 0 | 0 | 0 | 0 | 0 | 0 | | | | | | |
| 4388 | 05214 | 04387 | 1 | | 0 | 0 | 0 | 0 | 0 | 0 | 0 | 0 | 0 | 0 | 0 | 0 | 0 | 0 | 1 | 0 | 0 | 0 | 0 | 0 | 0 | 0 | 0 | 0 | 0 | | | | | | |
| 4389 | 05215 | 04388 | 2 | | 0 | 0 | 0 | 0 | 0 | 0 | 0 | 0 | 0 | 0 | 0 | 0 | 0 | 0 | 1 | 0 | 0 | 0 | 0 | 0 | 0 | 0 | 1 | 0 | 0 | | | | | | |
| 4390 | 05216 | 04389 | 2 | | 0 | 0 | 0 | 0 | 0 | 0 | 0 | 0 | 0 | 0 | 0 | 0 | 0 | 0 | 1 | 0 | 0 | 0 | 0 | 1 | 0 | 0 | 0 | 0 | 0 | | | | | | |
| 4391 | 05217 | 04390 | 5 | | 0 | 0 | 0 | 0 | 0 | 0 | 0 | 0 | 0 | 0 | 0 | 0 | 0 | 1 | 1 | 0 | 0 | 0 | 0 | 3 | 0 | 0 | 0 | 0 | 0 | | | | | | |
| 4392 | 05218 | 04391 | 1 | | 0 | 0 | 0 | 0 | 0 | 0 | 0 | 0 | 0 | 0 | 0 | 0 | 0 | 0 | 1 | 0 | 0 | 0 | 0 | 0 | 0 | 0 | 0 | 0 | 0 | | | | | | |
| 4393 | 05219 | 04392 | 1 | | 0 | 0 | 0 | 0 | 0 | 0 | 0 | 0 | 0 | 0 | 0 | 0 | 0 | 0 | 1 | 0 | 0 | 0 | 0 | 0 | 0 | 0 | 0 | 0 | 0 | | | | | | |
| 4394 | 05220 | 04393 | 1 | | 0 | 0 | 0 | 0 | 0 | 0 | 0 | 0 | 0 | 0 | 0 | 0 | 0 | 0 | 1 | 0 | 0 | 0 | 0 | 0 | 0 | 0 | 0 | 0 | 0 | | | | | | |
| 4395 | 05221 | 04394 | 1 | | 0 | 0 | 0 | 0 | 0 | 0 | 0 | 0 | 0 | 0 | 0 | 0 | 0 | 0 | 1 | 0 | 0 | 0 | 0 | 0 | 0 | 0 | 0 | 0 | 0 | | | | | | |
| 4396 | 05223 | 04395 | 1 | | 0 | 0 | 0 | 0 | 0 | 0 | 0 | 0 | 0 | 0 | 0 | 0 | 0 | 0 | 1 | 0 | 0 | 0 | 0 | 0 | 0 | 0 | 0 | 0 | 0 | | | | | | |
| 4397 | 05224 | 04396 | 1 | | 0 | 0 | 0 | 0 | 0 | 0 | 0 | 0 | 0 | 0 | 0 | 0 | 0 | 0 | 1 | 0 | 0 | 0 | 0 | 0 | 0 | 0 | 0 | 0 | 0 | | | | | | |
| 4398 | 05227 | 04397 | 1 | | 0 | 0 | 0 | 0 | 0 | 0 | 0 | 0 | 0 | 0 | 0 | 0 | 0 | 0 | 1 | 0 | 0 | 0 | 0 | 0 | 0 | 0 | 0 | 0 | 0 | | | | | | |
| 4399 | 05228 | 04398 | 1 | | 0 | 0 | 0 | 0 | 0 | 0 | 0 | 0 | 0 | 0 | 0 | 0 | 0 | 0 | 1 | 0 | 0 | 0 | 0 | 0 | 0 | 0 | 0 | 0 | 0 | | | | | | |
| 4400 | 05229 | 04399 | 1 | | 0 | 0 | 0 | 0 | 0 | 0 | 0 | 0 | 0 | 0 | 0 | 0 | 0 | 0 | 1 | 0 | 0 | 0 | 0 | 0 | 0 | 0 | 0 | 0 | 0 | | | | | | |
| 4401 | 05230 | 04400 | 1 | | 0 | 0 | 0 | 0 | 0 | 0 | 0 | 0 | 0 | 0 | 0 | 0 | 0 | 0 | 1 | 0 | 0 | 0 | 0 | 0 | 0 | 0 | 0 | 0 | 0 | | | | | | |
| 4402 | 05231 | 04401 | 2 | | 0 | 0 | 0 | 0 | 0 | 0 | 0 | 0 | 0 | 0 | 0 | 0 | 0 | 0 | 1 | 0 | 0 | 0 | 0 | 1 | 0 | 0 | 0 | 0 | 0 | | | | | | |
| 4403 | 05232 | 04402 | 1 | | 0 | 0 | 0 | 0 | 0 | 0 | 0 | 0 | 0 | 0 | 0 | 0 | 0 | 0 | 1 | 0 | 0 | 0 | 0 | 0 | 0 | 0 | 0 | 0 | 0 | | | | | | |
| 4404 | 05233 | 04403 | 2 | | 0 | 0 | 0 | 0 | 0 | 0 | 0 | 0 | 0 | 0 | 0 | 0 | 0 | 0 | 1 | 0 | 0 | 0 | 0 | 0 | 0 | 0 | 0 | 1 | 0 | | | | | | |
| 4405 | 05234 | 04404 | 1 | | 0 | 0 | 0 | 0 | 0 | 0 | 0 | 0 | 0 | 0 | 0 | 0 | 0 | 0 | 1 | 0 | 0 | 0 | 0 | 0 | 0 | 0 | 0 | 0 | 0 | M57650 | 98.4 | 64 | 1 | 1236 | 1299 |
| 4406 | 05235 | 04405 | 1 | | 0 | 0 | 0 | 0 | 0 | 0 | 0 | 0 | 0 | 0 | 0 | 0 | 0 | 0 | 1 | 0 | 0 | 0 | 0 | 0 | 0 | 0 | 0 | 0 | 0 | | | | | | |
| 4407 | 05236 | 04406 | 1 | | 0 | 0 | 0 | 0 | 0 | 0 | 0 | 0 | 0 | 0 | 0 | 0 | 0 | 0 | 1 | 0 | 0 | 0 | 0 | 0 | 0 | 0 | 0 | 0 | 0 | M83554 | 98.9 | 261 | 1 | 2258 | 3630 |
| 4408 | 05237 | 04407 | 2 | | 0 | 0 | 0 | 0 | 0 | 0 | 0 | 0 | 0 | 0 | 1 | 0 | 0 | 0 | 1 | 0 | 0 | 0 | 0 | 0 | 0 | 0 | 0 | 0 | 0 | | | | | | |
| 4409 | 05238 | 04408 | 1 | | 0 | 0 | 0 | 0 | 0 | 0 | 0 | 0 | 0 | 0 | 0 | 0 | 0 | 0 | 1 | 0 | 0 | 0 | 0 | 0 | 0 | 0 | 0 | 0 | 0 | | | | | | |
| 4410 | 05239 | 04409 | 1 | | 0 | 0 | 0 | 0 | 0 | 0 | 0 | 0 | 0 | 0 | 0 | 0 | 0 | 0 | 1 | 0 | 0 | 0 | 0 | 0 | 0 | 0 | 0 | 0 | 0 | | | | | | |
| 4411 | 05240 | 04410 | 1 | | 0 | 0 | 0 | 0 | 0 | 0 | 0 | 0 | 0 | 0 | 0 | 0 | 0 | 0 | 1 | 0 | 0 | 0 | 0 | 0 | 0 | 0 | 0 | 0 | 0 | X64330 | 96.3 | 188 | 4 | 3910 | 4297 |
| 4412 | 05241 | 04411 | 5 | | 0 | 0 | 0 | 0 | 0 | 0 | 0 | 0 | 0 | 0 | 0 | 0 | 0 | 0 | 1 | 0 | 0 | 0 | 1 | 0 | 1 | 1 | 1 | 0 | 0 | | | | | | |
| 4413 | 05242 | 04412 | 5 | | 0 | 0 | 0 | 0 | 0 | 0 | 0 | 0 | 0 | 0 | 0 | 0 | 0 | 0 | 1 | 0 | 0 | 0 | 0 | 0 | 0 | 0 | 4 | 0 | 0 | | | | | | |

Table 123

| | A | B | C | E | G | I | K | M | O | Q | S | U | W | Y | AA | AC | AF | AG | AI | AK | AM | AO | AQ | AS | AU | AW | AY | BA | BC | BE | BF | BG | BH | BI | BK |
|---|---|---|---|---|---|---|---|---|---|---|---|---|---|---|---|---|---|---|---|---|---|---|---|---|---|---|---|---|---|---|---|---|---|---|---|
| 4414 | 05243 | 04413 | 1 | | 0 | 0 | 0 | 0 | 0 | 0 | 0 | 0 | 0 | 0 | 0 | 0 | 0 | 0 | 1 | 0 | 0 | 0 | 0 | 0 | 0 | 0 | 0 | 0 | 0 | | | | | | |
| 4415 | 05244 | 04414 | 4 | | 0 | 0 | 0 | 0 | 0 | 0 | 0 | 0 | 0 | 0 | 0 | 0 | 0 | 0 | 2 | 0 | 0 | 0 | 0 | 0 | 0 | 0 | 2 | 0 | 0 | | | | | | |
| 4416 | 05245 | 04415 | 1 | | 0 | 0 | 0 | 0 | 0 | 0 | 0 | 0 | 0 | 0 | 0 | 0 | 0 | 0 | 1 | 0 | 0 | 0 | 0 | 0 | 0 | 0 | 0 | 0 | 0 | | | | | | |
| 4417 | 05246 | 04416 | 1 | | 0 | 0 | 0 | 0 | 0 | 0 | 0 | 0 | 0 | 0 | 0 | 0 | 0 | 0 | 1 | 0 | 0 | 0 | 0 | 0 | 0 | 0 | 0 | 0 | 0 | | | | | | |
| 4418 | 05247 | 04417 | 1 | | 0 | 0 | 0 | 0 | 0 | 0 | 0 | 0 | 0 | 0 | 0 | 0 | 0 | 0 | 1 | 0 | 0 | 0 | 0 | 0 | 0 | 0 | 0 | 0 | 0 | | | | | | |
| 4419 | 05248 | 04418 | 2 | | 0 | 0 | 0 | 0 | 0 | 0 | 0 | 0 | 0 | 0 | 0 | 0 | 0 | 0 | 1 | 0 | 0 | 1 | 0 | 0 | 0 | 0 | 0 | 0 | 0 | | | | | | |
| 4420 | 05249 | 04419 | 1 | | 0 | 0 | 0 | 0 | 0 | 0 | 0 | 0 | 0 | 0 | 0 | 0 | 0 | 0 | 1 | 0 | 0 | 0 | 0 | 0 | 0 | 0 | 0 | 0 | 0 | | | | | | |
| 4421 | 05251 | 04420 | 2 | | 0 | 0 | 0 | 0 | 0 | 0 | 0 | 0 | 0 | 0 | 0 | 0 | 0 | 0 | 2 | 0 | 0 | 0 | 0 | 0 | 0 | 0 | 0 | 0 | 0 | | | | | | |
| 4422 | 05252 | 04421 | 1 | | 0 | 0 | 0 | 0 | 0 | 0 | 0 | 0 | 0 | 0 | 0 | 0 | 0 | 0 | 1 | 0 | 0 | 0 | 0 | 0 | 0 | 0 | 0 | 0 | 0 | | | | | | |
| 4423 | 05253 | 04422 | 1 | | 0 | 0 | 0 | 0 | 0 | 0 | 0 | 0 | 0 | 0 | 0 | 0 | 0 | 0 | 1 | 0 | 0 | 0 | 0 | 0 | 0 | 0 | 0 | 0 | 0 | | | | | | |
| 4424 | 05254 | 04423 | 1 | | 0 | 0 | 0 | 0 | 0 | 0 | 0 | 0 | 0 | 0 | 0 | 0 | 0 | 0 | 1 | 0 | 0 | 0 | 0 | 0 | 0 | 0 | 0 | 0 | 0 | | | | | | |
| 4425 | 05255 | 04424 | 1 | | 0 | 0 | 0 | 0 | 0 | 0 | 0 | 0 | 0 | 0 | 0 | 0 | 0 | 0 | 1 | 0 | 0 | 0 | 0 | 0 | 0 | 0 | 0 | 0 | 0 | | | | | | |
| 4426 | 05259 | 04425 | 1 | | 0 | 0 | 0 | 0 | 0 | 0 | 0 | 0 | 0 | 0 | 0 | 0 | 0 | 0 | 1 | 0 | 0 | 0 | 0 | 0 | 0 | 0 | 0 | 0 | 0 | | | | | | |
| 4427 | 05260 | 04426 | 2 | | 0 | 0 | 0 | 0 | 0 | 0 | 0 | 0 | 0 | 0 | 0 | 0 | 0 | 0 | 2 | 0 | 0 | 0 | 0 | 0 | 0 | 0 | 0 | 0 | 0 | | | | | | |
| 4428 | 05262 | 04427 | 1 | | 0 | 0 | 0 | 0 | 0 | 0 | 0 | 0 | 0 | 0 | 0 | 0 | 0 | 0 | 1 | 0 | 0 | 0 | 0 | 0 | 0 | 0 | 0 | 0 | 0 | | | | | | |
| 4429 | 05263 | 04428 | 2 | | 0 | 0 | 0 | 0 | 0 | 0 | 0 | 0 | 0 | 0 | 1 | 0 | 0 | 0 | 1 | 0 | 0 | 0 | 0 | 0 | 0 | 0 | 0 | 0 | 0 | | | | | | |
| 4430 | 05264 | 04429 | 2 | | 0 | 0 | 0 | 0 | 0 | 0 | 0 | 0 | 0 | 0 | 0 | 0 | 0 | 0 | 1 | 0 | 0 | 1 | 0 | 0 | 0 | 0 | 0 | 0 | 0 | | | | | | |
| 4431 | 05266 | 04430 | 1 | | 0 | 0 | 0 | 0 | 0 | 0 | 0 | 0 | 0 | 0 | 0 | 0 | 0 | 0 | 1 | 0 | 0 | 0 | 0 | 0 | 0 | 0 | 0 | 0 | 0 | M59040 | 95.2 | 84 | 1 | 1706 | 1794 |
| 4432 | 05267 | 04431 | 2 | | 0 | 0 | 0 | 0 | 0 | 0 | 0 | 0 | 0 | 0 | 0 | 0 | 0 | 0 | 1 | 0 | 0 | 0 | 0 | 1 | 0 | 0 | 0 | 0 | 0 | | | | | | |
| 4433 | 05269 | 04432 | 1 | | 0 | 0 | 0 | 0 | 0 | 0 | 0 | 0 | 0 | 0 | 0 | 0 | 0 | 0 | 1 | 0 | 0 | 0 | 0 | 0 | 0 | 0 | 0 | 0 | 0 | | | | | | |
| 4434 | 05270 | 04433 | 1 | | 0 | 0 | 0 | 0 | 0 | 0 | 0 | 0 | 0 | 0 | 0 | 0 | 0 | 0 | 1 | 0 | 0 | 0 | 0 | 0 | 0 | 0 | 0 | 0 | 0 | | | | | | |
| 4435 | 05271 | 04434 | 2 | | 0 | 0 | 0 | 0 | 0 | 0 | 0 | 0 | 0 | 0 | 0 | 0 | 0 | 0 | 1 | 1 | 0 | 0 | 0 | 0 | 0 | 0 | 0 | 0 | 0 | | | | | | |
| 4436 | 05272 | 04435 | 1 | | 0 | 0 | 0 | 0 | 0 | 0 | 0 | 0 | 0 | 0 | 0 | 0 | 0 | 0 | 1 | 0 | 0 | 0 | 0 | 0 | 0 | 0 | 0 | 0 | 0 | M80647 | 96.7 | 120 | 1 | 1761 | 1882 |
| 4437 | 05273 | 04436 | 3 | | 0 | 0 | 0 | 0 | 0 | 0 | 0 | 0 | 0 | 0 | 0 | 0 | 0 | 0 | 2 | 0 | 0 | 0 | 0 | 0 | 0 | 1 | 0 | 0 | 0 | | | | | | |
| 4438 | 05274 | 04437 | 1 | | 0 | 0 | 0 | 0 | 0 | 0 | 0 | 0 | 0 | 0 | 0 | 0 | 0 | 0 | 1 | 0 | 0 | 0 | 0 | 0 | 0 | 0 | 0 | 0 | 0 | J04621 | 95.5 | 332 | 1 | 1887 | 3414 |
| 4439 | 05275 | 04438 | 1 | | 0 | 0 | 0 | 0 | 0 | 0 | 0 | 0 | 0 | 0 | 0 | 0 | 0 | 0 | 1 | 0 | 0 | 0 | 0 | 0 | 0 | 0 | 0 | 0 | 0 | | | | | | |
| 4440 | 05276 | 04439 | 1 | | 0 | 0 | 0 | 0 | 0 | 0 | 0 | 0 | 0 | 0 | 0 | 0 | 0 | 0 | 1 | 0 | 0 | 0 | 0 | 0 | 0 | 0 | 0 | 0 | 0 | | | | | | |
| 4441 | 05278 | 04440 | 1 | | 0 | 0 | 0 | 0 | 0 | 0 | 0 | 0 | 0 | 0 | 0 | 0 | 0 | 0 | 1 | 0 | 0 | 0 | 0 | 0 | 0 | 0 | 0 | 0 | 0 | | | | | | |
| 4442 | 05279 | 04441 | 1 | | 0 | 0 | 0 | 0 | 0 | 0 | 0 | 0 | 0 | 0 | 0 | 0 | 0 | 0 | 1 | 0 | 0 | 0 | 0 | 0 | 0 | 0 | 0 | 0 | 0 | | | | | | |
| 4443 | 05280 | 04442 | 1 | | 0 | 0 | 0 | 0 | 0 | 0 | 0 | 0 | 0 | 0 | 0 | 0 | 0 | 0 | 1 | 0 | 0 | 0 | 0 | 0 | 0 | 0 | 0 | 0 | 0 | | | | | | |
| 4444 | 05281 | 04443 | 1 | | 0 | 0 | 0 | 0 | 0 | 0 | 0 | 0 | 0 | 0 | 0 | 0 | 0 | 0 | 1 | 0 | 0 | 0 | 0 | 0 | 0 | 0 | 0 | 0 | 0 | | | | | | |
| 4445 | 05282 | 04444 | 1 | | 0 | 0 | 0 | 0 | 0 | 0 | 0 | 0 | 0 | 0 | 0 | 0 | 0 | 0 | 1 | 0 | 0 | 0 | 0 | 0 | 0 | 0 | 0 | 0 | 0 | | | | | | |
| 4446 | 05283 | 04445 | 2 | | 0 | 0 | 0 | 0 | 0 | 0 | 0 | 0 | 0 | 0 | 0 | 0 | 0 | 0 | 1 | 0 | 0 | 0 | 0 | 0 | 0 | 1 | 0 | 0 | 0 | | | | | | |
| 4447 | 05284 | 04446 | 1 | | 0 | 0 | 0 | 0 | 0 | 0 | 0 | 0 | 0 | 0 | 0 | 0 | 0 | 0 | 1 | 0 | 0 | 0 | 0 | 0 | 0 | 0 | 0 | 0 | 0 | | | | | | |
| 4448 | 05285 | 04447 | 3 | | 0 | 0 | 0 | 0 | 0 | 0 | 0 | 0 | 0 | 0 | 0 | 0 | 0 | 0 | 1 | 1 | 0 | 0 | 0 | 0 | 0 | 0 | 0 | 0 | 1 | | | | | | |
| 4449 | 05286 | 04448 | 2 | | 0 | 0 | 0 | 0 | 0 | 0 | 0 | 0 | 0 | 0 | 0 | 0 | 0 | 0 | 1 | 0 | 0 | 1 | 0 | 0 | 0 | 0 | 0 | 0 | 0 | | | | | | |

Table 124

| | A | B | C | E | G | I | K | M | O | Q | S | U | W | Y | AA | AC | AE | AG | AI | AK | AM | AO | AQ | AS | AU | AW | AY | BA | BC | BE | BF | BG | BH | BI | BK |
|---|---|---|---|---|---|---|---|---|---|---|---|---|---|---|---|---|---|---|---|---|---|---|---|---|---|---|---|---|---|---|---|---|---|---|---|
| 4450 | 05287 | 04449 | 1 | | 0 | 0 | 0 | 0 | 0 | 0 | 0 | 0 | 0 | 0 | 0 | 0 | 0 | 0 | 1 | 0 | 0 | 0 | 0 | 0 | 0 | 0 | 0 | 0 | 0 | | | | | | |
| 4451 | 05288 | 04450 | 1 | | 0 | 0 | 0 | 0 | 0 | 0 | 0 | 0 | 0 | 0 | 0 | 0 | 0 | 0 | 1 | 0 | 0 | 0 | 0 | 0 | 0 | 0 | 0 | 0 | 0 | | | | | | |
| 4452 | 05289 | 04451 | 2 | | 0 | 0 | 0 | 0 | 0 | 0 | 0 | 0 | 0 | 0 | 0 | 0 | 0 | 0 | 1 | 1 | 0 | 0 | 0 | 0 | 0 | 0 | 0 | 0 | 0 | U03643 | 99.2 | 118 | 1 | 1263 | 1380 |
| 4453 | 05290 | 04452 | 1 | | 0 | 0 | 0 | 0 | 0 | 0 | 0 | 0 | 0 | 0 | 0 | 0 | 0 | 0 | 1 | 0 | 0 | 0 | 0 | 0 | 0 | 0 | 0 | 0 | 0 | X53799 | 100 | 290 | 1 | 792 | 1081 |
| 4454 | 05291 | 04453 | 2 | | 0 | 0 | 0 | 0 | 0 | 0 | 0 | 0 | 0 | 0 | 0 | 0 | 0 | 0 | 1 | 0 | 0 | 0 | 0 | 0 | 0 | 0 | 1 | 0 | 0 | | | | | | |
| 4455 | 05292 | 04454 | 1 | | 0 | 0 | 0 | 0 | 0 | 0 | 0 | 0 | 0 | 0 | 0 | 0 | 0 | 0 | 1 | 0 | 0 | 0 | 0 | 0 | 0 | 0 | 0 | 0 | 0 | | | | | | |
| 4456 | 05293 | 04455 | 3 | | 0 | 0 | 0 | 0 | 0 | 0 | 0 | 0 | 0 | 0 | 0 | 0 | 0 | 2 | 1 | 0 | 0 | 0 | 0 | 0 | 0 | 0 | 0 | 0 | 0 | | | | | | |
| 4457 | 05294 | 04456 | 1 | | 0 | 0 | 0 | 0 | 0 | 0 | 0 | 0 | 0 | 0 | 0 | 0 | 0 | 0 | 1 | 0 | 0 | 0 | 0 | 0 | 0 | 0 | 0 | 0 | 0 | | | | | | |
| 4458 | 05295 | 04457 | 1 | | 0 | 0 | 0 | 0 | 0 | 0 | 0 | 0 | 0 | 0 | 0 | 0 | 0 | 0 | 1 | 0 | 0 | 0 | 0 | 0 | 0 | 0 | 0 | 0 | 0 | | | | | | |
| 4459 | 05296 | 04458 | 1 | | 0 | 0 | 0 | 0 | 0 | 0 | 0 | 0 | 0 | 0 | 0 | 0 | 0 | 0 | 1 | 0 | 0 | 0 | 0 | 0 | 0 | 0 | 0 | 0 | 0 | | | | | | |
| 4460 | 05297 | 04459 | 1 | | 0 | 0 | 0 | 0 | 0 | 0 | 0 | 0 | 0 | 0 | 0 | 0 | 0 | 0 | 1 | 0 | 0 | 0 | 0 | 0 | 0 | 0 | 0 | 0 | 0 | | | | | | |
| 4461 | 05298 | 04460 | 1 | | 0 | 0 | 0 | 0 | 0 | 0 | 0 | 0 | 0 | 0 | 0 | 0 | 0 | 0 | 1 | 0 | 0 | 0 | 0 | 0 | 0 | 0 | 0 | 0 | 0 | | | | | | |
| 4462 | 05300 | 04461 | 1 | | 0 | 0 | 0 | 0 | 0 | 0 | 0 | 0 | 0 | 0 | 0 | 0 | 0 | 0 | 1 | 0 | 0 | 0 | 0 | 0 | 0 | 0 | 0 | 0 | 0 | Z22969 | 98.5 | 130 | 1 | 3405 | 3786 |
| 4463 | 05301 | 04462 | 1 | | 0 | 0 | 0 | 0 | 0 | 0 | 0 | 0 | 0 | 0 | 0 | 0 | 0 | 0 | 1 | 0 | 0 | 0 | 0 | 0 | 0 | 0 | 0 | 0 | 0 | | | | | | |
| 4464 | 05302 | 04463 | 1 | | 0 | 0 | 0 | 0 | 0 | 0 | 0 | 0 | 0 | 0 | 0 | 0 | 0 | 0 | 1 | 0 | 0 | 0 | 0 | 0 | 0 | 0 | 0 | 0 | 0 | | | | | | |
| 4465 | 05303 | 04464 | 2 | | 0 | 0 | 0 | 0 | 0 | 0 | 0 | 0 | 0 | 0 | 0 | 0 | 0 | 1 | 1 | 0 | 0 | 0 | 0 | 0 | 0 | 0 | 0 | 0 | 0 | | | | | | |
| 4466 | 05304 | 04465 | 1 | | 0 | 0 | 0 | 0 | 0 | 0 | 0 | 0 | 0 | 0 | 0 | 0 | 0 | 0 | 1 | 0 | 0 | 0 | 0 | 0 | 0 | 0 | 0 | 0 | 0 | | | | | | |
| 4467 | 05305 | 04466 | 1 | | 0 | 0 | 0 | 0 | 0 | 0 | 0 | 0 | 0 | 0 | 0 | 0 | 0 | 0 | 1 | 0 | 0 | 0 | 0 | 0 | 0 | 0 | 0 | 0 | 0 | | | | | | |
| 4468 | 05306 | 04467 | 1 | | 0 | 0 | 0 | 0 | 0 | 0 | 0 | 0 | 0 | 0 | 0 | 0 | 0 | 0 | 1 | 0 | 0 | 0 | 0 | 0 | 0 | 0 | 0 | 0 | 0 | | | | | | |
| 4469 | 05307 | 04468 | 1 | | 0 | 0 | 0 | 0 | 0 | 0 | 0 | 0 | 0 | 0 | 0 | 0 | 0 | 0 | 1 | 0 | 0 | 0 | 0 | 0 | 0 | 0 | 0 | 0 | 0 | | | | | | |
| 4470 | 05308 | 04469 | 4 | | 0 | 0 | 0 | 0 | 0 | 0 | 0 | 0 | 0 | 0 | 0 | 0 | 0 | 0 | 1 | 1 | 0 | 1 | 0 | 0 | 0 | 1 | 0 | 0 | 0 | X68148 | 98 | 354 | 1 | 2662 | 3031 |
| 4471 | 05309 | 04470 | 1 | | 0 | 0 | 0 | 0 | 0 | 0 | 0 | 0 | 0 | 0 | 0 | 0 | 0 | 0 | 1 | 0 | 0 | 0 | 0 | 0 | 0 | 0 | 0 | 0 | 0 | Y00971 | 96.9 | 64 | 1 | 2392 | 2457 |
| 4472 | 05310 | 04471 | 1 | | 0 | 0 | 0 | 0 | 0 | 0 | 0 | 0 | 0 | 0 | 0 | 0 | 0 | 0 | 1 | 0 | 0 | 0 | 0 | 0 | 0 | 0 | 0 | 0 | 0 | | | | | | |
| 4473 | 05311 | 04472 | 1 | | 0 | 0 | 0 | 0 | 0 | 0 | 0 | 0 | 0 | 0 | 0 | 0 | 0 | 0 | 1 | 0 | 0 | 0 | 0 | 0 | 0 | 0 | 0 | 0 | 0 | | | | | | |
| 4474 | 05312 | 04473 | 2 | | 0 | 0 | 0 | 0 | 0 | 0 | 0 | 0 | 0 | 0 | 0 | 0 | 0 | 0 | 2 | 0 | 0 | 0 | 0 | 0 | 0 | 0 | 0 | 0 | 0 | | | | | | |
| 4475 | 05313 | 04474 | 1 | | 0 | 0 | 0 | 0 | 0 | 0 | 0 | 0 | 0 | 0 | 0 | 0 | 0 | 0 | 1 | 0 | 0 | 0 | 0 | 0 | 0 | 0 | 0 | 0 | 0 | | | | | | |
| 4476 | 05314 | 04475 | 1 | | 0 | 0 | 0 | 0 | 0 | 0 | 0 | 0 | 0 | 0 | 0 | 0 | 0 | 0 | 1 | 0 | 0 | 0 | 0 | 0 | 0 | 0 | 0 | 0 | 0 | | | | | | |
| 4477 | 05315 | 04476 | 1 | | 0 | 0 | 0 | 0 | 0 | 0 | 0 | 0 | 0 | 0 | 0 | 0 | 0 | 0 | 1 | 0 | 0 | 0 | 0 | 0 | 0 | 0 | 0 | 0 | 0 | | | | | | |
| 4478 | 05316 | 04477 | 2 | | 0 | 0 | 0 | 0 | 0 | 0 | 0 | 0 | 0 | 0 | 0 | 0 | 0 | 0 | 2 | 0 | 0 | 0 | 0 | 0 | 0 | 0 | 0 | 0 | 0 | | | | | | |
| 4479 | 05318 | 04478 | 1 | | 0 | 0 | 0 | 0 | 0 | 0 | 0 | 0 | 0 | 0 | 0 | 0 | 0 | 0 | 1 | 0 | 0 | 0 | 0 | 0 | 0 | 0 | 0 | 0 | 0 | | | | | | |
| 4480 | 05319 | 04479 | 2 | | 0 | 0 | 0 | 0 | 0 | 0 | 0 | 0 | 0 | 0 | 0 | 0 | 0 | 0 | 2 | 0 | 0 | 0 | 0 | 0 | 0 | 0 | 0 | 0 | 0 | | | | | | |
| 4481 | 05320 | 04480 | 1 | | 0 | 0 | 0 | 0 | 0 | 0 | 0 | 0 | 0 | 0 | 0 | 0 | 0 | 0 | 1 | 0 | 0 | 0 | 0 | 0 | 0 | 0 | 0 | 0 | 0 | | | | | | |
| 4482 | 05321 | 04481 | 2 | | 0 | 0 | 0 | 0 | 0 | 0 | 0 | 0 | 0 | 0 | 0 | 0 | 0 | 0 | 1 | 0 | 0 | 0 | 0 | 0 | 0 | 0 | 1 | 0 | 0 | | | | | | |
| 4483 | 05322 | 04482 | 3 | | 0 | 0 | 0 | 0 | 0 | 0 | 0 | 0 | 0 | 0 | 0 | 0 | 0 | 0 | 1 | 0 | 0 | 0 | 0 | 0 | 0 | 0 | 2 | 0 | 0 | | | | | | |
| 4484 | 05323 | 04483 | 3 | | 0 | 0 | 0 | 0 | 0 | 0 | 0 | 0 | 0 | 0 | 0 | 0 | 0 | 1 | 1 | 0 | 0 | 0 | 0 | 0 | 0 | 0 | 1 | 0 | 0 | | | | | | |
| 4485 | 05325 | 04484 | 5 | | 0 | 0 | 0 | 0 | 0 | 0 | 0 | 0 | 0 | 0 | 0 | 0 | 0 | 0 | 1 | 1 | 0 | 2 | 0 | 0 | 1 | 0 | 0 | 0 | 0 | | | | | | |

Table 125

| | A | B | C | E | G | I | K | M | O | Q | S | U | W | Y | AA | AC | AE | AG | AI | AK | AM | AO | AQ | AS | AU | AW | AY | BA | BC | BE | BF | BG | BH | BI | BK |
|---|---|---|---|---|---|---|---|---|---|---|---|---|---|---|---|---|---|---|---|---|---|---|---|---|---|---|---|---|---|---|---|---|---|---|---|
| 4486 | 05326 | 04485 | 1 | | 0 | 0 | 0 | 0 | 0 | 0 | 0 | 0 | 0 | 0 | 0 | 0 | 0 | 0 | 1 | 0 | 0 | 0 | 0 | 0 | 0 | 0 | 0 | 0 | 0 | | | | | | |
| 4487 | 05327 | 04486 | 1 | | 0 | 0 | 0 | 0 | 0 | 0 | 0 | 0 | 0 | 0 | 0 | 0 | 0 | 0 | 1 | 0 | 0 | 0 | 0 | 0 | 0 | 0 | 0 | 0 | 0 | | | | | | |
| 4488 | 05328 | 04487 | 1 | | 0 | 0 | 0 | 0 | 0 | 0 | 0 | 0 | 0 | 0 | 0 | 0 | 0 | 0 | 1 | 0 | 0 | 0 | 0 | 0 | 0 | 0 | 0 | 0 | 0 | | | | | | |
| 4489 | 05329 | 04488 | 1 | | 0 | 0 | 0 | 0 | 0 | 0 | 0 | 0 | 0 | 0 | 0 | 0 | 0 | 0 | 1 | 0 | 0 | 0 | 0 | 0 | 0 | 0 | 0 | 0 | 0 | | | | | | |
| 4490 | 05331 | 04489 | 1 | | 0 | 0 | 0 | 0 | 0 | 0 | 0 | 0 | 0 | 0 | 0 | 0 | 0 | 0 | 1 | 0 | 0 | 0 | 0 | 0 | 0 | 0 | 0 | 0 | 0 | | | | | | |
| 4491 | 05332 | 04490 | 1 | | 0 | 0 | 0 | 0 | 0 | 0 | 0 | 0 | 0 | 0 | 0 | 0 | 0 | 0 | 1 | 0 | 0 | 0 | 0 | 0 | 0 | 0 | 0 | 0 | 0 | | | | | | |
| 4492 | 05333 | 04491 | 3 | | 0 | 0 | 0 | 0 | 0 | 0 | 0 | 0 | 0 | 0 | 0 | 0 | 0 | 0 | 1 | 0 | 0 | 0 | 0 | 0 | 0 | 1 | 0 | 0 | 1 | | | | | | |
| 4493 | 05334 | 04492 | 2 | | 0 | 0 | 0 | 0 | 0 | 0 | 0 | 0 | 0 | 0 | 0 | 0 | 0 | 0 | 1 | 0 | 0 | 0 | 0 | 1 | 0 | 0 | 0 | 0 | 0 | | | | | | |
| 4494 | 05335 | 04493 | 1 | | 0 | 0 | 0 | 0 | 0 | 0 | 0 | 0 | 0 | 0 | 0 | 0 | 0 | 0 | 1 | 0 | 0 | 0 | 0 | 0 | 0 | 0 | 0 | 0 | 0 | | | | | | |
| 4495 | 05336 | 04494 | 1 | | 0 | 0 | 0 | 0 | 0 | 0 | 0 | 0 | 0 | 0 | 0 | 0 | 0 | 0 | 1 | 0 | 0 | 0 | 0 | 0 | 0 | 0 | 0 | 0 | 0 | | | | | | |
| 4496 | 05337 | 04495 | 1 | | 0 | 0 | 0 | 0 | 0 | 0 | 0 | 0 | 0 | 0 | 0 | 0 | 0 | 0 | 1 | 0 | 0 | 0 | 0 | 0 | 0 | 0 | 0 | 0 | 0 | | | | | | |
| 4497 | 05339 | 04496 | 4 | | 0 | 0 | 0 | 0 | 0 | 0 | 0 | 0 | 0 | 0 | 0 | 0 | 0 | 0 | 1 | 1 | 0 | 0 | 0 | 0 | 1 | 0 | 0 | 1 | 0 | | | | | | |
| 4498 | 05340 | 04497 | 1 | | 0 | 0 | 0 | 0 | 0 | 0 | 0 | 0 | 0 | 0 | 0 | 0 | 0 | 0 | 1 | 0 | 0 | 0 | 0 | 0 | 0 | 0 | 0 | 0 | 0 | | | | | | |
| 4499 | 05341 | 04498 | 1 | | 0 | 0 | 0 | 0 | 0 | 0 | 0 | 0 | 0 | 0 | 0 | 0 | 0 | 0 | 1 | 0 | 0 | 0 | 0 | 0 | 0 | 0 | 0 | 0 | 0 | | | | | | |
| 4500 | 05342 | 04499 | 2 | | 0 | 0 | 0 | 0 | 0 | 0 | 0 | 0 | 0 | 0 | 0 | 0 | 0 | 0 | 2 | 0 | 0 | 0 | 0 | 0 | 0 | 0 | 0 | 0 | 0 | | | | | | |
| 4501 | 05343 | 04500 | 1 | | 0 | 0 | 0 | 0 | 0 | 0 | 0 | 0 | 0 | 0 | 0 | 0 | 0 | 0 | 1 | 0 | 0 | 0 | 0 | 0 | 0 | 0 | 0 | 0 | 0 | | | | | | |
| 4502 | 05344 | 04501 | 2 | | 0 | 0 | 0 | 0 | 0 | 0 | 0 | 0 | 0 | 0 | 0 | 0 | 0 | 0 | 1 | 0 | 0 | 0 | 0 | 0 | 0 | 1 | 0 | 0 | 0 | | | | | | |
| 4503 | 05345 | 04502 | 3 | | 0 | 0 | 0 | 0 | 0 | 0 | 0 | 0 | 0 | 0 | 0 | 0 | 0 | 1 | 1 | 0 | 0 | 1 | 0 | 0 | 0 | 0 | 0 | 0 | 0 | | | | | | |
| 4504 | 05346 | 04503 | 1 | | 0 | 0 | 0 | 0 | 0 | 0 | 0 | 0 | 0 | 0 | 0 | 0 | 0 | 0 | 1 | 0 | 0 | 0 | 0 | 0 | 0 | 0 | 0 | 0 | 0 | | | | | | |
| 4505 | 05347 | 04504 | 1 | | 0 | 0 | 0 | 0 | 0 | 0 | 0 | 0 | 0 | 0 | 0 | 0 | 0 | 0 | 1 | 0 | 0 | 0 | 0 | 0 | 0 | 0 | 0 | 0 | 0 | | | | | | |
| 4506 | 05349 | 04505 | 1 | | 0 | 0 | 0 | 0 | 0 | 0 | 0 | 0 | 0 | 0 | 0 | 0 | 0 | 0 | 1 | 0 | 0 | 0 | 0 | 0 | 0 | 0 | 0 | 0 | 0 | | | | | | |
| 4507 | 05350 | 04506 | 1 | | 0 | 0 | 0 | 0 | 0 | 0 | 0 | 0 | 0 | 0 | 0 | 0 | 0 | 0 | 1 | 0 | 0 | 0 | 0 | 0 | 0 | 0 | 0 | 0 | 0 | | | | | | |
| 4508 | 05351 | 04507 | 1 | | 0 | 0 | 0 | 0 | 0 | 0 | 0 | 0 | 0 | 0 | 0 | 0 | 0 | 0 | 1 | 0 | 0 | 0 | 0 | 0 | 0 | 0 | 0 | 0 | 0 | | | | | | |
| 4509 | 05352 | 04508 | 1 | | 0 | 0 | 0 | 0 | 0 | 0 | 0 | 0 | 0 | 0 | 0 | 0 | 0 | 0 | 1 | 0 | 0 | 0 | 0 | 0 | 0 | 0 | 0 | 0 | 0 | | | | | | |
| 4510 | 05353 | 04509 | 2 | | 0 | 0 | 0 | 0 | 0 | 0 | 0 | 0 | 0 | 0 | 0 | 0 | 0 | 0 | 1 | 0 | 0 | 0 | 0 | 0 | 0 | 0 | 1 | 0 | 0 | | | | | | |
| 4511 | 05354 | 04510 | 1 | | 0 | 0 | 0 | 0 | 0 | 0 | 0 | 0 | 0 | 0 | 0 | 0 | 0 | 0 | 1 | 0 | 0 | 0 | 0 | 0 | 0 | 0 | 0 | 0 | 0 | L01411 | 90.4 | 324 | 2 | 223 | 636 |
| 4512 | 05355 | 04511 | 4 | | 0 | 0 | 0 | 0 | 0 | 0 | 0 | 0 | 0 | 0 | 0 | 0 | 0 | 0 | 2 | 0 | 0 | 0 | 1 | 1 | 0 | 0 | 0 | 0 | 0 | | | | | | |
| 4513 | 05356 | 04512 | 1 | | 0 | 0 | 0 | 0 | 0 | 0 | 0 | 0 | 0 | 0 | 0 | 0 | 0 | 0 | 1 | 0 | 0 | 0 | 0 | 0 | 0 | 0 | 0 | 0 | 0 | | | | | | |
| 4514 | 05357 | 04513 | 1 | | 0 | 0 | 0 | 0 | 0 | 0 | 0 | 0 | 0 | 0 | 0 | 0 | 0 | 0 | 1 | 0 | 0 | 0 | 0 | 0 | 0 | 0 | 0 | 0 | 0 | | | | | | |
| 4515 | 05359 | 04514 | 1 | | 0 | 0 | 0 | 0 | 0 | 0 | 0 | 0 | 0 | 0 | 0 | 0 | 0 | 0 | 1 | 0 | 0 | 0 | 0 | 0 | 0 | 0 | 0 | 0 | 0 | | | | | | |
| 4516 | 05360 | 04515 | 1 | | 0 | 0 | 0 | 0 | 0 | 0 | 0 | 0 | 0 | 0 | 0 | 0 | 0 | 0 | 1 | 0 | 0 | 0 | 0 | 0 | 0 | 0 | 0 | 0 | 0 | | | | | | |
| 4517 | 05361 | 04516 | 1 | | 0 | 0 | 0 | 0 | 0 | 0 | 0 | 0 | 0 | 0 | 0 | 0 | 0 | 0 | 1 | 0 | 0 | 0 | 0 | 0 | 0 | 0 | 0 | 0 | 0 | | | | | | |
| 4518 | 05362 | 04517 | 1 | | 0 | 0 | 0 | 0 | 0 | 0 | 0 | 0 | 0 | 0 | 0 | 0 | 0 | 0 | 1 | 0 | 0 | 0 | 0 | 0 | 0 | 0 | 0 | 0 | 0 | | | | | | |
| 4519 | 05363 | 04518 | 1 | | 0 | 0 | 0 | 0 | 0 | 0 | 0 | 0 | 0 | 0 | 0 | 0 | 0 | 0 | 1 | 0 | 0 | 0 | 0 | 0 | 0 | 0 | 0 | 0 | 0 | | | | | | |
| 4520 | 05364 | 04519 | 1 | | 0 | 0 | 0 | 0 | 0 | 0 | 0 | 0 | 0 | 0 | 0 | 0 | 0 | 0 | 1 | 0 | 0 | 0 | 0 | 0 | 0 | 0 | 0 | 0 | 0 | | | | | | |
| 4521 | 05365 | 04520 | 1 | | 0 | 0 | 0 | 0 | 0 | 0 | 0 | 0 | 0 | 0 | 0 | 0 | 0 | 0 | 1 | 0 | 0 | 0 | 0 | 0 | 0 | 0 | 0 | 0 | 0 | | | | | | |

Table 126

| | A | B | C | E | G | I | K | M | O | Q | S | U | W | Y | AA | AC | AE | AG | AI | AK | AM | AO | AQ | AS | AU | AW | AY | BA | BC | BE | BF | BG | BH | BI | BK |
|---|---|---|---|---|---|---|---|---|---|---|---|---|---|---|---|---|---|---|---|---|---|---|---|---|---|---|---|---|---|---|---|---|---|---|---|
| 4522 | 05366 | 04521 | 1 | | 0 | 0 | 0 | 0 | 0 | 0 | 0 | 0 | 0 | 0 | 0 | 0 | 0 | 0 | 1 | 0 | 0 | 0 | 0 | 0 | 0 | 0 | 0 | 0 | 0 | | | | | | |
| 4523 | 05367 | 04522 | 2 | | 0 | 0 | 0 | 0 | 0 | 0 | 0 | 0 | 0 | 0 | 0 | 0 | 0 | 0 | 2 | 0 | 0 | 0 | 0 | 0 | 0 | 0 | 0 | 0 | 0 | M55169 | 100 | 79 | 1 | 4363 | 4446 |
| 4524 | 05368 | 04523 | 1 | | 0 | 0 | 0 | 0 | 0 | 0 | 0 | 0 | 0 | 0 | 0 | 0 | 0 | 0 | 1 | 0 | 0 | 0 | 0 | 0 | 0 | 0 | 0 | 0 | 0 | | | | | | |
| 4525 | 05370 | 04524 | 1 | | 0 | 0 | 0 | 0 | 0 | 0 | 0 | 0 | 0 | 0 | 0 | 0 | 0 | 0 | 1 | 0 | 0 | 0 | 0 | 0 | 0 | 0 | 0 | 0 | 0 | | | | | | |
| 4526 | 05371 | 04525 | 1 | | 0 | 0 | 0 | 0 | 0 | 0 | 0 | 0 | 0 | 0 | 0 | 0 | 0 | 0 | 1 | 0 | 0 | 0 | 0 | 0 | 0 | 0 | 0 | 0 | 0 | | | | | | |
| 4527 | 05372 | 04526 | 2 | | 0 | 0 | 0 | 0 | 0 | 0 | 0 | 0 | 0 | 0 | 0 | 0 | 0 | 0 | 1 | 0 | 0 | 0 | 0 | 1 | 0 | 0 | 0 | 0 | 0 | | | | | | |
| 4528 | 05373 | 04527 | 1 | | 0 | 0 | 0 | 0 | 0 | 0 | 0 | 0 | 0 | 0 | 0 | 0 | 0 | 0 | 1 | 0 | 0 | 0 | 0 | 0 | 0 | 0 | 0 | 0 | 0 | | | | | | |
| 4529 | 05374 | 04528 | 1 | | 0 | 0 | 0 | 0 | 0 | 0 | 0 | 0 | 0 | 0 | 0 | 0 | 0 | 0 | 1 | 0 | 0 | 0 | 0 | 0 | 0 | 0 | 0 | 0 | 0 | | | | | | |
| 4530 | 05375 | 04529 | 1 | | 0 | 0 | 0 | 0 | 0 | 0 | 0 | 0 | 0 | 0 | 0 | 0 | 0 | 0 | 1 | 0 | 0 | 0 | 0 | 0 | 0 | 0 | 0 | 0 | 0 | | | | | | |
| 4531 | 05376 | 04530 | 2 | | 0 | 0 | 0 | 0 | 0 | 0 | 0 | 0 | 0 | 0 | 0 | 0 | 0 | 1 | 1 | 0 | 0 | 0 | 0 | 0 | 0 | 0 | 0 | 0 | 0 | | | | | | |
| 4532 | 05377 | 04531 | 1 | | 0 | 0 | 0 | 0 | 0 | 0 | 0 | 0 | 0 | 0 | 0 | 0 | 0 | 0 | 1 | 0 | 0 | 0 | 0 | 0 | 0 | 0 | 0 | 0 | 0 | | | | | | |
| 4533 | 05378 | 04532 | 1 | | 0 | 0 | 0 | 0 | 0 | 0 | 0 | 0 | 0 | 0 | 0 | 0 | 0 | 0 | 1 | 0 | 0 | 0 | 0 | 0 | 0 | 0 | 0 | 0 | 0 | | | | | | |
| 4534 | 05379 | 04533 | 1 | | 0 | 0 | 0 | 0 | 0 | 0 | 0 | 0 | 0 | 0 | 0 | 0 | 0 | 0 | 1 | 0 | 0 | 0 | 0 | 0 | 0 | 0 | 0 | 0 | 0 | | | | | | |
| 4535 | 05381 | 04534 | 1 | | 0 | 0 | 0 | 0 | 0 | 0 | 0 | 0 | 0 | 0 | 0 | 0 | 0 | 0 | 1 | 0 | 0 | 0 | 0 | 0 | 0 | 0 | 0 | 0 | 0 | | | | | | |
| 4536 | 05382 | 04535 | 3 | | 0 | 0 | 0 | 0 | 0 | 0 | 0 | 0 | 0 | 0 | 0 | 0 | 0 | 0 | 1 | 1 | 0 | 1 | 0 | 0 | 0 | 0 | 0 | 0 | 0 | | | | | | |
| 4537 | 05383 | 04536 | 1 | | 0 | 0 | 0 | 0 | 0 | 0 | 0 | 0 | 0 | 0 | 0 | 0 | 0 | 0 | 1 | 0 | 0 | 0 | 0 | 0 | 0 | 0 | 0 | 0 | 0 | | | | | | |
| 4538 | 05384 | 04537 | 1 | | 0 | 0 | 0 | 0 | 0 | 0 | 0 | 0 | 0 | 0 | 0 | 0 | 0 | 0 | 1 | 0 | 0 | 0 | 0 | 0 | 0 | 0 | 0 | 0 | 0 | | | | | | |
| 4539 | 05387 | 04538 | 2 | | 0 | 0 | 0 | 0 | 0 | 0 | 0 | 0 | 0 | 0 | 0 | 0 | 0 | 2 | 0 | 0 | 0 | 0 | 0 | 0 | 0 | 0 | 0 | 0 | 0 | | | | | | |
| 4540 | 05388 | 04539 | 2 | | 0 | 0 | 0 | 0 | 0 | 0 | 0 | 0 | 0 | 0 | 0 | 0 | 0 | 2 | 0 | 0 | 0 | 0 | 0 | 0 | 0 | 0 | 0 | 0 | 0 | | | | | | |
| 4541 | 05389 | 04540 | 4 | | 0 | 0 | 0 | 0 | 0 | 0 | 0 | 0 | 0 | 0 | 0 | 0 | 0 | 2 | 0 | 0 | 0 | 0 | 1 | 0 | 0 | 0 | 0 | 1 | 0 | | | | | | |
| 4542 | 05390 | 04541 | 32 | | 0 | 0 | 0 | 0 | 0 | 0 | 0 | 0 | 0 | 0 | 0 | 0 | 0 | 13 | 0 | 0 | 0 | 0 | 14 | 5 | 0 | 0 | 0 | 0 | 0 | M13577 | 98 | 100 | 1 | 2041 | 2139 |
| 4543 | 05391 | 04542 | 2 | | 0 | 0 | 0 | 0 | 0 | 0 | 0 | 0 | 0 | 0 | 0 | 0 | 0 | 2 | 0 | 0 | 0 | 0 | 0 | 0 | 0 | 0 | 0 | 0 | 0 | J05459 | 99.1 | 216 | 1 | 630 | 1266 |
| 4544 | 05392 | 04543 | 2 | | 0 | 0 | 0 | 0 | 0 | 0 | 0 | 0 | 0 | 0 | 0 | 0 | 0 | 2 | 0 | 0 | 0 | 0 | 0 | 0 | 0 | 0 | 0 | 0 | 0 | | | | | | |
| 4545 | 05393 | 04544 | 1 | | 0 | 0 | 0 | 0 | 0 | 0 | 0 | 0 | 0 | 0 | 0 | 0 | 0 | 1 | 0 | 0 | 0 | 0 | 0 | 0 | 0 | 0 | 0 | 0 | 0 | | | | | | |
| 4546 | 05394 | 04545 | 1 | | 0 | 0 | 0 | 0 | 0 | 0 | 0 | 0 | 0 | 0 | 0 | 0 | 0 | 1 | 0 | 0 | 0 | 0 | 0 | 0 | 0 | 0 | 0 | 0 | 0 | | | | | | |
| 4547 | 05395 | 04546 | 3 | | 0 | 0 | 0 | 0 | 0 | 0 | 0 | 0 | 0 | 0 | 0 | 0 | 0 | 1 | 0 | 1 | 1 | 0 | 0 | 0 | 0 | 0 | 0 | 0 | 0 | | | | | | |
| 4548 | 05396 | 04547 | 9 | | 0 | 0 | 0 | 0 | 0 | 0 | 0 | 0 | 0 | 0 | 0 | 0 | 0 | 2 | 0 | 0 | 0 | 0 | 7 | 0 | 0 | 0 | 0 | 0 | 0 | | | | | | |
| 4549 | 05398 | 04548 | 1 | | 0 | 0 | 0 | 0 | 0 | 0 | 0 | 0 | 0 | 0 | 0 | 0 | 0 | 1 | 0 | 0 | 0 | 0 | 0 | 0 | 0 | 0 | 0 | 0 | 0 | | | | | | |
| 4550 | 05399 | 04549 | 1 | | 0 | 0 | 0 | 0 | 0 | 0 | 0 | 0 | 0 | 0 | 0 | 0 | 0 | 1 | 0 | 0 | 0 | 0 | 0 | 0 | 0 | 0 | 0 | 0 | 0 | | | | | | |
| 4551 | 05400 | 04550 | 2 | | 0 | 0 | 0 | 0 | 0 | 0 | 0 | 0 | 0 | 0 | 0 | 0 | 0 | 1 | 0 | 1 | 0 | 0 | 0 | 0 | 0 | 0 | 0 | 0 | 0 | | | | | | |
| 4552 | 05401 | 04551 | 2 | | 0 | 0 | 0 | 0 | 0 | 0 | 0 | 0 | 0 | 0 | 0 | 0 | 0 | 1 | 0 | 0 | 0 | 0 | 0 | 0 | 0 | 0 | 0 | 0 | 1 | M69054 | 95.8 | 265 | 1 | 645 | 918 |
| 4553 | 05402 | 04552 | 1 | | 0 | 0 | 0 | 0 | 0 | 0 | 0 | 0 | 0 | 0 | 0 | 0 | 0 | 1 | 0 | 0 | 0 | 0 | 0 | 0 | 0 | 0 | 0 | 0 | 0 | | | | | | |
| 4554 | 05404 | 04553 | 1 | | 0 | 0 | 0 | 0 | 0 | 0 | 0 | 0 | 0 | 0 | 0 | 0 | 0 | 1 | 0 | 0 | 0 | 0 | 0 | 0 | 0 | 0 | 0 | 0 | 0 | | | | | | |
| 4555 | 05406 | 04554 | 2 | | 0 | 0 | 0 | 0 | 0 | 0 | 0 | 0 | 0 | 0 | 0 | 0 | 0 | 1 | 0 | 0 | 0 | 0 | 0 | 0 | 0 | 0 | 0 | 0 | 1 | | | | | | |
| 4556 | 05407 | 04555 | 1 | | 0 | 0 | 0 | 0 | 0 | 0 | 0 | 0 | 0 | 0 | 0 | 0 | 0 | 1 | 0 | 0 | 0 | 0 | 0 | 0 | 0 | 0 | 0 | 0 | 0 | | | | | | |
| 4557 | 05408 | 04556 | 1 | | 0 | 0 | 0 | 0 | 0 | 0 | 0 | 0 | 0 | 0 | 0 | 0 | 0 | 1 | 0 | 0 | 0 | 0 | 0 | 0 | 0 | 0 | 0 | 0 | 0 | | | | | | |

Table 127

| | A | B | C | E | G | I | K | M | O | Q | S | U | W | Y | AA | AC | AE | AG | AI | AK | AM | AO | AQ | AS | AU | AW | AY | BA | BC | BE | BF | BG | BH | BI | BK |
|---|---|---|---|---|---|---|---|---|---|---|---|---|---|---|---|---|---|---|---|---|---|---|---|---|---|---|---|---|---|---|---|---|---|---|---|
| 4558 | 05409 | 04557 | 1 | | 0 | 0 | 0 | 0 | 0 | 0 | 0 | 0 | 0 | 0 | 0 | 0 | 0 | 1 | 0 | 0 | 0 | 0 | 0 | 0 | 0 | 0 | 0 | 0 | 0 | | | | | | |
| 4559 | 05410 | 04558 | 2 | | 0 | 0 | 0 | 0 | 0 | 0 | 0 | 0 | 0 | 0 | 0 | 0 | 0 | 1 | 0 | 0 | 0 | 0 | 0 | 0 | 1 | 0 | 0 | 0 | 0 | | | | | | |
| 4560 | 05411 | 04559 | 4 | | 0 | 0 | 0 | 0 | 0 | 0 | 0 | 0 | 0 | 0 | 0 | 0 | 0 | 1 | 0 | 0 | 0 | 0 | 0 | 1 | 1 | 1 | 0 | 0 | 0 | | | | | | |
| 4561 | 05412 | 04560 | 9 | | 0 | 0 | 0 | 0 | 0 | 0 | 0 | 0 | 0 | 0 | 0 | 0 | 0 | 7 | 0 | 0 | 2 | 0 | 0 | 0 | 0 | 0 | 0 | 0 | 0 | | | | | | |
| 4562 | 05413 | 04561 | 1 | | 0 | 0 | 0 | 0 | 0 | 0 | 0 | 0 | 0 | 0 | 0 | 0 | 0 | 1 | 0 | 0 | 0 | 0 | 0 | 0 | 0 | 0 | 0 | 0 | 0 | | | | | | |
| 4563 | 05414 | 04562 | 1 | | 0 | 0 | 0 | 0 | 0 | 0 | 0 | 0 | 0 | 0 | 0 | 0 | 0 | 1 | 0 | 0 | 0 | 0 | 0 | 0 | 0 | 0 | 0 | 0 | 0 | | | | | | |
| 4564 | 05415 | 04563 | 3 | | 0 | 0 | 0 | 0 | 0 | 0 | 0 | 0 | 0 | 0 | 0 | 0 | 0 | 1 | 0 | 0 | 0 | 0 | 0 | 2 | 0 | 0 | 0 | 0 | 0 | | | | | | |
| 4565 | 05416 | 04564 | 1 | | 0 | 0 | 0 | 0 | 0 | 0 | 0 | 0 | 0 | 0 | 0 | 0 | 0 | 1 | 0 | 0 | 0 | 0 | 0 | 0 | 0 | 0 | 0 | 0 | 0 | | | | | | |
| 4566 | 05417 | 04565 | 5 | | 0 | 0 | 0 | 0 | 0 | 0 | 0 | 0 | 0 | 0 | 0 | 0 | 0 | 3 | 0 | 0 | 0 | 0 | 0 | 1 | 1 | 0 | 0 | 0 | 0 | | | | | | |
| 4567 | 05418 | 04566 | 3 | | 0 | 0 | 0 | 0 | 0 | 0 | 0 | 0 | 0 | 0 | 1 | 0 | 0 | 1 | 0 | 0 | 0 | 0 | 1 | 0 | 0 | 0 | 0 | 0 | 0 | | | | | | |
| 4568 | 05419 | 04567 | 2 | | 0 | 0 | 0 | 0 | 0 | 0 | 0 | 0 | 0 | 0 | 0 | 0 | 0 | 1 | 0 | 0 | 0 | 0 | 0 | 0 | 0 | 0 | 0 | 1 | 0 | | | | | | |
| 4569 | 05420 | 04568 | 3 | | 0 | 0 | 0 | 0 | 0 | 0 | 0 | 0 | 0 | 0 | 0 | 0 | 0 | 3 | 0 | 0 | 0 | 0 | 0 | 0 | 0 | 0 | 0 | 0 | 0 | | | | | | |
| 4570 | 05421 | 04569 | 1 | | 0 | 0 | 0 | 0 | 0 | 0 | 0 | 0 | 0 | 0 | 0 | 0 | 0 | 1 | 0 | 0 | 0 | 0 | 0 | 0 | 0 | 0 | 0 | 0 | 0 | | | | | | |
| 4571 | 05422 | 04570 | 2 | | 0 | 0 | 0 | 0 | 0 | 0 | 0 | 0 | 0 | 0 | 0 | 0 | 0 | 1 | 0 | 0 | 0 | 0 | 0 | 1 | 0 | 0 | 0 | 0 | 0 | | | | | | |
| 4572 | 05423 | 04571 | 3 | | 0 | 0 | 0 | 0 | 0 | 0 | 0 | 0 | 0 | 0 | 0 | 0 | 0 | 1 | 0 | 0 | 0 | 0 | 1 | 1 | 0 | 0 | 0 | 0 | 0 | | | | | | |
| 4573 | 05424 | 04572 | 2 | | 0 | 0 | 0 | 0 | 0 | 0 | 0 | 0 | 0 | 0 | 0 | 0 | 0 | 1 | 0 | 1 | 0 | 0 | 0 | 0 | 0 | 0 | 0 | 0 | 0 | | | | | | |
| 4574 | 05425 | 04573 | 2 | | 0 | 0 | 0 | 0 | 0 | 0 | 0 | 0 | 0 | 0 | 0 | 0 | 0 | 1 | 0 | 0 | 0 | 0 | 0 | 1 | 0 | 0 | 0 | 0 | 0 | | | | | | |
| 4575 | 05427 | 04574 | 1 | | 0 | 0 | 0 | 0 | 0 | 0 | 0 | 0 | 0 | 0 | 0 | 0 | 0 | 1 | 0 | 0 | 0 | 0 | 0 | 0 | 0 | 0 | 0 | 0 | 0 | | | | | | |
| 4576 | 05428 | 04575 | 2 | | 0 | 0 | 0 | 0 | 0 | 0 | 0 | 0 | 0 | 0 | 0 | 0 | 0 | 2 | 0 | 0 | 0 | 0 | 0 | 0 | 0 | 0 | 0 | 0 | 0 | | | | | | |
| 4577 | 05429 | 04576 | 1 | | 0 | 0 | 0 | 0 | 0 | 0 | 0 | 0 | 0 | 0 | 0 | 0 | 0 | 1 | 0 | 0 | 0 | 0 | 0 | 0 | 0 | 0 | 0 | 0 | 0 | | | | | | |
| 4578 | 05430 | 04577 | 1 | | 0 | 0 | 0 | 0 | 0 | 0 | 0 | 0 | 0 | 0 | 0 | 0 | 0 | 1 | 0 | 0 | 0 | 0 | 0 | 0 | 0 | 0 | 0 | 0 | 0 | | | | | | |
| 4579 | 05431 | 04578 | 1 | | 0 | 0 | 0 | 0 | 0 | 0 | 0 | 0 | 0 | 0 | 0 | 0 | 0 | 1 | 0 | 0 | 0 | 0 | 0 | 0 | 0 | 0 | 0 | 0 | 0 | | | | | | |
| 4580 | 05432 | 04579 | 2 | | 0 | 0 | 0 | 0 | 0 | 0 | 0 | 0 | 0 | 0 | 0 | 0 | 0 | 1 | 0 | 0 | 0 | 0 | 1 | 0 | 0 | 0 | 0 | 0 | 0 | | | | | | |
| 4581 | 05433 | 04580 | 2 | | 0 | 0 | 0 | 0 | 0 | 0 | 0 | 0 | 0 | 0 | 0 | 0 | 0 | 2 | 0 | 0 | 0 | 0 | 0 | 0 | 0 | 0 | 0 | 0 | 0 | | | | | | |
| 4582 | 05434 | 04581 | 4 | | 0 | 0 | 0 | 0 | 0 | 0 | 0 | 0 | 0 | 0 | 0 | 0 | 0 | 1 | 0 | 0 | 0 | 0 | 2 | 1 | 0 | 0 | 0 | 0 | 0 | | | | | | |
| 4583 | 05435 | 04582 | 3 | | 0 | 0 | 0 | 0 | 0 | 0 | 0 | 0 | 0 | 0 | 0 | 0 | 0 | 1 | 0 | 0 | 0 | 0 | 0 | 0 | 2 | 0 | 0 | 0 | 0 | | | | | | |
| 4584 | 05436 | 04583 | 1 | | 0 | 0 | 0 | 0 | 0 | 0 | 0 | 0 | 0 | 0 | 0 | 0 | 0 | 1 | 0 | 0 | 0 | 0 | 0 | 0 | 0 | 0 | 0 | 0 | 0 | | | | | | |
| 4585 | 05437 | 04584 | 3 | | 0 | 0 | 0 | 0 | 0 | 0 | 0 | 0 | 0 | 0 | 0 | 0 | 0 | 2 | 0 | 0 | 0 | 0 | 0 | 1 | 0 | 0 | 0 | 0 | 0 | | | | | | |
| 4586 | 05438 | 04585 | 3 | | 0 | 0 | 0 | 0 | 0 | 0 | 0 | 0 | 0 | 0 | 0 | 0 | 0 | 2 | 0 | 0 | 0 | 0 | 1 | 0 | 0 | 0 | 0 | 0 | 0 | | | | | | |
| 4587 | 05439 | 04586 | 3 | | 0 | 0 | 0 | 0 | 0 | 0 | 0 | 0 | 0 | 0 | 0 | 0 | 0 | 2 | 0 | 0 | 0 | 0 | 0 | 1 | 0 | 0 | 0 | 0 | 0 | | | | | | |
| 4588 | 05440 | 04587 | 1 | | 0 | 0 | 0 | 0 | 0 | 0 | 0 | 0 | 0 | 0 | 0 | 0 | 0 | 1 | 0 | 0 | 0 | 0 | 0 | 0 | 0 | 0 | 0 | 0 | 0 | | | | | | |
| 4589 | 05441 | 04588 | 1 | | 0 | 0 | 0 | 0 | 0 | 0 | 0 | 0 | 0 | 0 | 0 | 0 | 0 | 1 | 0 | 0 | 0 | 0 | 0 | 0 | 0 | 0 | 0 | 0 | 0 | | | | | | |
| 4590 | 05442 | 04589 | 1 | | 0 | 0 | 0 | 0 | 0 | 0 | 0 | 0 | 0 | 0 | 0 | 0 | 0 | 1 | 0 | 0 | 0 | 0 | 0 | 0 | 0 | 0 | 0 | 0 | 0 | | | | | | |
| 4591 | 05443 | 04590 | 1 | | 0 | 0 | 0 | 0 | 0 | 0 | 0 | 0 | 0 | 0 | 0 | 0 | 0 | 1 | 0 | 0 | 0 | 0 | 0 | 0 | 0 | 0 | 0 | 0 | 0 | | | | | | |
| 4592 | 05444 | 04591 | 1 | | 0 | 0 | 0 | 0 | 0 | 0 | 0 | 0 | 0 | 0 | 0 | 0 | 0 | 1 | 0 | 0 | 0 | 0 | 0 | 0 | 0 | 0 | 0 | 0 | 0 | | | | | | |
| 4593 | 05445 | 04592 | 2 | | 0 | 0 | 0 | 0 | 0 | 0 | 0 | 0 | 0 | 0 | 0 | 0 | 0 | 1 | 0 | 0 | 0 | 0 | 1 | 0 | 0 | 0 | 0 | 0 | 0 | | | | | | |

Table 128

| | A | B | C | E | G | I | K | M | O | Q | S | U | W | Y | AA | AC | AE | AG | AI | AK | AM | AO | AQ | AS | AU | AW | AY | BA | BC | BE | BF | BG | BH | BI | BK |
|---|---|---|---|---|---|---|---|---|---|---|---|---|---|---|---|---|---|---|---|---|---|---|---|---|---|---|---|---|---|---|---|---|---|---|---|
| 4594 | 05446 | 04593 | 1 | | 0 | 0 | 0 | 0 | 0 | 0 | 0 | 0 | 0 | 0 | 0 | 0 | 0 | 1 | 0 | 0 | 0 | 0 | 0 | 0 | 0 | 0 | 0 | 0 | 0 | | | | | | |
| 4595 | 05447 | 04594 | 2 | | 0 | 0 | 0 | 0 | 0 | 0 | 0 | 0 | 0 | 0 | 0 | 0 | 0 | 2 | 0 | 0 | 0 | 0 | 0 | 0 | 0 | 0 | 0 | 0 | 0 | | | | | | |
| 4596 | 05448 | 04595 | 3 | | 0 | 0 | 0 | 0 | 0 | 0 | 0 | 0 | 0 | 0 | 0 | 0 | 0 | 2 | 0 | 0 | 0 | 0 | 0 | 0 | 0 | 1 | 0 | 0 | 0 | | | | | | |
| 4597 | 05451 | 04596 | 1 | | 0 | 0 | 0 | 0 | 0 | 0 | 0 | 0 | 0 | 0 | 0 | 0 | 0 | 1 | 0 | 0 | 0 | 0 | 0 | 0 | 0 | 0 | 0 | 0 | 0 | | | | | | |
| 4598 | 05452 | 04597 | 2 | | 0 | 0 | 0 | 0 | 0 | 0 | 0 | 0 | 0 | 0 | 0 | 0 | 0 | 1 | 0 | 0 | 0 | 0 | 0 | 1 | 0 | 0 | 0 | 0 | 0 | | | | | | |
| 4599 | 05453 | 04598 | 1 | | 0 | 0 | 0 | 0 | 0 | 0 | 0 | 0 | 0 | 0 | 0 | 0 | 0 | 1 | 0 | 0 | 0 | 0 | 0 | 0 | 0 | 0 | 0 | 0 | 0 | | | | | | |
| 4600 | 05454 | 04599 | 1 | | 0 | 0 | 0 | 0 | 0 | 0 | 0 | 0 | 0 | 0 | 0 | 0 | 0 | 1 | 0 | 0 | 0 | 0 | 0 | 0 | 0 | 0 | 0 | 0 | 0 | | | | | | |
| 4601 | 05455 | 04600 | 1 | | 0 | 0 | 0 | 0 | 0 | 0 | 0 | 0 | 0 | 0 | 0 | 0 | 0 | 1 | 0 | 0 | 0 | 0 | 0 | 0 | 0 | 0 | 0 | 0 | 0 | | | | | | |
| 4602 | 05456 | 04601 | 2 | | 0 | 0 | 0 | 0 | 0 | 0 | 0 | 0 | 0 | 0 | 0 | 0 | 0 | 2 | 0 | 0 | 0 | 0 | 0 | 0 | 0 | 0 | 0 | 0 | 0 | | | | | | |
| 4603 | 05458 | 04602 | 1 | | 0 | 0 | 0 | 0 | 0 | 0 | 0 | 0 | 0 | 0 | 0 | 0 | 0 | 1 | 0 | 0 | 0 | 0 | 0 | 0 | 0 | 0 | 0 | 0 | 0 | | | | | | |
| 4604 | 05459 | 04603 | 1 | | 0 | 0 | 0 | 0 | 0 | 0 | 0 | 0 | 0 | 0 | 0 | 0 | 0 | 1 | 0 | 0 | 0 | 0 | 0 | 0 | 0 | 0 | 0 | 0 | 0 | | | | | | |
| 4605 | 05460 | 04604 | 1 | | 0 | 0 | 0 | 0 | 0 | 0 | 0 | 0 | 0 | 0 | 0 | 0 | 0 | 1 | 0 | 0 | 0 | 0 | 0 | 0 | 0 | 0 | 0 | 0 | 0 | | | | | | |
| 4606 | 05461 | 04605 | 2 | | 0 | 0 | 0 | 0 | 0 | 0 | 0 | 0 | 0 | 0 | 0 | 0 | 0 | 1 | 0 | 0 | 0 | 0 | 1 | 0 | 0 | 0 | 0 | 0 | 0 | | | | | | |
| 4607 | 05462 | 04606 | 3 | | 0 | 0 | 0 | 0 | 0 | 0 | 0 | 0 | 0 | 0 | 0 | 0 | 0 | 1 | 0 | 0 | 0 | 0 | 0 | 2 | 0 | 0 | 0 | 0 | 0 | | | | | | |
| 4608 | 05464 | 04607 | 1 | | 0 | 0 | 0 | 0 | 0 | 0 | 0 | 0 | 0 | 0 | 0 | 0 | 0 | 1 | 0 | 0 | 0 | 0 | 0 | 0 | 0 | 0 | 0 | 0 | 0 | | | | | | |
| 4609 | 05465 | 04608 | 1 | | 0 | 0 | 0 | 0 | 0 | 0 | 0 | 0 | 0 | 0 | 0 | 0 | 0 | 1 | 0 | 0 | 0 | 0 | 0 | 0 | 0 | 0 | 0 | 0 | 0 | | | | | | |
| 4610 | 05466 | 04609 | 1 | | 0 | 0 | 0 | 0 | 0 | 0 | 0 | 0 | 0 | 0 | 0 | 0 | 0 | 1 | 0 | 0 | 0 | 0 | 0 | 0 | 0 | 0 | 0 | 0 | 0 | | | | | | |
| 4611 | 05467 | 04610 | 2 | | 0 | 0 | 0 | 0 | 0 | 0 | 0 | 0 | 0 | 0 | 0 | 0 | 0 | 2 | 0 | 0 | 0 | 0 | 0 | 0 | 0 | 0 | 0 | 0 | 0 | | | | | | |
| 4612 | 05468 | 04611 | 2 | | 0 | 0 | 0 | 0 | 0 | 0 | 0 | 0 | 0 | 0 | 0 | 0 | 0 | 1 | 0 | 0 | 0 | 0 | 0 | 1 | 0 | 0 | 0 | 0 | 0 | | | | | | |
| 4613 | 05469 | 04612 | 1 | | 0 | 0 | 0 | 0 | 0 | 0 | 0 | 0 | 0 | 0 | 0 | 0 | 0 | 1 | 0 | 0 | 0 | 0 | 0 | 0 | 0 | 0 | 0 | 0 | 0 | | | | | | |
| 4614 | 05470 | 04613 | 1 | | 0 | 0 | 0 | 0 | 0 | 0 | 0 | 0 | 0 | 0 | 0 | 0 | 0 | 1 | 0 | 0 | 0 | 0 | 0 | 0 | 0 | 0 | 0 | 0 | 0 | | | | | | |
| 4615 | 05471 | 04614 | 1 | | 0 | 0 | 0 | 0 | 0 | 0 | 0 | 0 | 0 | 0 | 0 | 0 | 0 | 1 | 0 | 0 | 0 | 0 | 0 | 0 | 0 | 0 | 0 | 0 | 0 | | | | | | |
| 4616 | 05472 | 04615 | 1 | | 0 | 0 | 0 | 0 | 0 | 0 | 0 | 0 | 0 | 0 | 0 | 0 | 0 | 1 | 0 | 0 | 0 | 0 | 0 | 0 | 0 | 0 | 0 | 0 | 0 | | | | | | |
| 4617 | 05474 | 04616 | 1 | | 0 | 0 | 0 | 0 | 0 | 0 | 0 | 0 | 0 | 0 | 0 | 0 | 0 | 1 | 0 | 0 | 0 | 0 | 0 | 0 | 0 | 0 | 0 | 0 | 0 | | | | | | |
| 4618 | 05475 | 04617 | 1 | | 0 | 0 | 0 | 0 | 0 | 0 | 0 | 0 | 0 | 0 | 0 | 0 | 0 | 1 | 0 | 0 | 0 | 0 | 0 | 0 | 0 | 0 | 0 | 0 | 0 | | | | | | |
| 4619 | 05476 | 04618 | 2 | | 0 | 0 | 0 | 0 | 0 | 0 | 0 | 0 | 0 | 0 | 0 | 0 | 0 | 1 | 0 | 0 | 0 | 0 | 0 | 0 | 0 | 1 | 0 | 0 | 0 | | | | | | |
| 4620 | 05477 | 04619 | 1 | | 0 | 0 | 0 | 0 | 0 | 0 | 0 | 0 | 0 | 0 | 0 | 0 | 0 | 1 | 0 | 0 | 0 | 0 | 0 | 0 | 0 | 0 | 0 | 0 | 0 | | | | | | |
| 4621 | 05478 | 04620 | 2 | | 0 | 0 | 0 | 0 | 0 | 0 | 0 | 0 | 0 | 0 | 0 | 0 | 0 | 1 | 0 | 0 | 0 | 0 | 0 | 1 | 0 | 0 | 0 | 0 | 0 | | | | | | |
| 4622 | 05479 | 04621 | 4 | | 0 | 0 | 0 | 0 | 0 | 0 | 0 | 0 | 0 | 0 | 0 | 0 | 0 | 1 | 0 | 0 | 0 | 0 | 2 | 1 | 0 | 0 | 0 | 0 | 0 | | | | | | |
| 4623 | 05480 | 04622 | 2 | | 0 | 0 | 0 | 0 | 0 | 0 | 0 | 0 | 0 | 0 | 0 | 0 | 0 | 1 | 0 | 0 | 0 | 0 | 0 | 1 | 0 | 0 | 0 | 0 | 0 | | | | | | |
| 4624 | 05481 | 04623 | 1 | | 0 | 0 | 0 | 0 | 0 | 0 | 0 | 0 | 0 | 0 | 0 | 0 | 0 | 1 | 0 | 0 | 0 | 0 | 0 | 0 | 0 | 0 | 0 | 0 | 0 | | | | | | |
| 4625 | 05482 | 04624 | 1 | | 0 | 0 | 0 | 0 | 0 | 0 | 0 | 0 | 0 | 0 | 0 | 0 | 0 | 1 | 0 | 0 | 0 | 0 | 0 | 0 | 0 | 0 | 0 | 0 | 0 | | | | | | |
| 4626 | 05483 | 04625 | 2 | | 0 | 0 | 0 | 0 | 0 | 0 | 0 | 0 | 0 | 0 | 0 | 0 | 0 | 2 | 0 | 0 | 0 | 0 | 0 | 0 | 0 | 0 | 0 | 0 | 0 | | | | | | |
| 4627 | 05484 | 04626 | 1 | | 0 | 0 | 0 | 0 | 0 | 0 | 0 | 0 | 0 | 0 | 0 | 0 | 0 | 1 | 0 | 0 | 0 | 0 | 0 | 0 | 0 | 0 | 0 | 0 | 0 | | | | | | |
| 4628 | 05485 | 04627 | 1 | | 0 | 0 | 0 | 0 | 0 | 0 | 0 | 0 | 0 | 0 | 0 | 0 | 0 | 1 | 0 | 0 | 0 | 0 | 0 | 0 | 0 | 0 | 0 | 0 | 0 | | | | | | |
| 4629 | 05486 | 04628 | 1 | | 0 | 0 | 0 | 0 | 0 | 0 | 0 | 0 | 0 | 0 | 0 | 0 | 0 | 1 | 0 | 0 | 0 | 0 | 0 | 0 | 0 | 0 | 0 | 0 | 0 | | | | | | |

Table 129

| | A | B | C | E | G | I | K | M | O | Q | S | U | W | Y | AA | AC | AE | AG | AI | AK | AM | AO | AQ | AS | AU | AW | AY | BA | BC | BE | BF | BG | BH | BI | BK |
|---|---|---|---|---|---|---|---|---|---|---|---|---|---|---|---|---|---|---|---|---|---|---|---|---|---|---|---|---|---|---|---|---|---|---|---|
| 4630 | 05488 | 04629 | 2 | | 0 | 0 | 0 | 0 | 0 | 0 | 0 | 0 | 0 | 0 | 0 | 0 | 0 | 2 | 0 | 0 | 0 | 0 | 0 | 0 | 0 | 0 | 0 | 0 | 0 | | | | | | |
| 4631 | 05490 | 04630 | 3 | | 0 | 0 | 0 | 0 | 0 | 0 | 0 | 0 | 0 | 0 | 0 | 0 | 0 | 2 | 0 | 1 | 0 | 0 | 0 | 0 | 0 | 0 | 0 | 0 | 0 | | | | | | |
| 4632 | 05491 | 04631 | 1 | | 0 | 0 | 0 | 0 | 0 | 0 | 0 | 0 | 0 | 0 | 0 | 0 | 0 | 0 | 0 | 0 | 1 | 0 | 0 | 0 | 0 | 0 | 0 | 0 | 0 | | | | | | |
| 4633 | 05492 | 04632 | 2 | | 0 | 0 | 0 | 0 | 0 | 0 | 0 | 0 | 0 | 0 | 0 | 0 | 0 | 2 | 0 | 0 | 0 | 0 | 0 | 0 | 0 | 0 | 0 | 0 | 0 | | | | | | |
| 4634 | 05493 | 04633 | 1 | | 0 | 0 | 0 | 0 | 0 | 0 | 0 | 0 | 0 | 0 | 0 | 0 | 0 | 1 | 0 | 0 | 0 | 0 | 0 | 0 | 0 | 0 | 0 | 0 | 0 | | | | | | |
| 4635 | 05494 | 04634 | 1 | | 0 | 0 | 0 | 0 | 0 | 0 | 0 | 0 | 0 | 0 | 0 | 0 | 0 | 1 | 0 | 0 | 0 | 0 | 0 | 0 | 0 | 0 | 0 | 0 | 0 | | | | | | |
| 4636 | 05495 | 04635 | 2 | | 0 | 0 | 0 | 0 | 0 | 0 | 0 | 0 | 0 | 0 | 0 | 0 | 0 | 1 | 0 | 0 | 0 | 0 | 0 | 1 | 0 | 0 | 0 | 0 | 0 | | | | | | |
| 4637 | 05496 | 04636 | 1 | | 0 | 0 | 0 | 0 | 0 | 0 | 0 | 0 | 0 | 0 | 0 | 0 | 0 | 1 | 0 | 0 | 0 | 0 | 0 | 0 | 0 | 0 | 0 | 0 | 0 | | | | | | |
| 4638 | 05497 | 04637 | 2 | | 0 | 0 | 0 | 0 | 0 | 0 | 0 | 0 | 0 | 0 | 0 | 0 | 0 | 2 | 0 | 0 | 0 | 0 | 0 | 0 | 0 | 0 | 0 | 0 | 0 | | | | | | |
| 4639 | 05498 | 04638 | 1 | | 0 | 0 | 0 | 0 | 0 | 0 | 0 | 0 | 0 | 0 | 0 | 0 | 0 | 1 | 0 | 0 | 0 | 0 | 0 | 0 | 0 | 0 | 0 | 0 | 0 | | | | | | |
| 4640 | 05502 | 04639 | 1 | | 0 | 0 | 0 | 0 | 0 | 0 | 0 | 0 | 0 | 0 | 0 | 0 | 0 | 1 | 0 | 0 | 0 | 0 | 0 | 0 | 0 | 0 | 0 | 0 | 0 | | | | | | |
| 4641 | 05503 | 04640 | 3 | | 0 | 0 | 0 | 0 | 0 | 0 | 0 | 0 | 0 | 0 | 0 | 0 | 0 | 2 | 0 | 0 | 0 | 0 | 1 | 0 | 0 | 0 | 0 | 0 | 0 | | | | | | |
| 4642 | 05504 | 04641 | 1 | | 0 | 0 | 0 | 0 | 0 | 0 | 0 | 0 | 0 | 0 | 0 | 0 | 0 | 1 | 0 | 0 | 0 | 0 | 0 | 0 | 0 | 0 | 0 | 0 | 0 | | | | | | |
| 4643 | 05505 | 04642 | 1 | | 0 | 0 | 0 | 0 | 0 | 0 | 0 | 0 | 0 | 0 | 0 | 0 | 0 | 1 | 0 | 0 | 0 | 0 | 0 | 0 | 0 | 0 | 0 | 0 | 0 | | | | | | |
| 4644 | 05506 | 04643 | 1 | | 0 | 0 | 0 | 0 | 0 | 0 | 0 | 0 | 0 | 0 | 0 | 0 | 0 | 1 | 0 | 0 | 0 | 0 | 0 | 0 | 0 | 0 | 0 | 0 | 0 | | | | | | |
| 4645 | 05507 | 04644 | 1 | | 0 | 0 | 0 | 0 | 0 | 0 | 0 | 0 | 0 | 0 | 0 | 0 | 0 | 1 | 0 | 0 | 0 | 0 | 0 | 0 | 0 | 0 | 0 | 0 | 0 | | | | | | |
| 4646 | 05508 | 04645 | 1 | | 0 | 0 | 0 | 0 | 0 | 0 | 0 | 0 | 0 | 0 | 0 | 0 | 0 | 1 | 0 | 0 | 0 | 0 | 0 | 0 | 0 | 0 | 0 | 0 | 0 | D26018 | 97.1 | 136 | 1 | 3296 | 3430 |
| 4647 | 05509 | 04646 | 1 | | 0 | 0 | 0 | 0 | 0 | 0 | 0 | 0 | 0 | 0 | 0 | 0 | 0 | 1 | 0 | 0 | 0 | 0 | 0 | 0 | 0 | 0 | 0 | 0 | 0 | | | | | | |
| 4648 | 05510 | 04647 | 1 | | 0 | 0 | 0 | 0 | 0 | 0 | 0 | 0 | 0 | 0 | 0 | 0 | 0 | 1 | 0 | 0 | 0 | 0 | 0 | 0 | 0 | 0 | 0 | 0 | 0 | | | | | | |
| 4649 | 05511 | 04648 | 1 | | 0 | 0 | 0 | 0 | 0 | 0 | 0 | 0 | 0 | 0 | 0 | 0 | 0 | 1 | 0 | 0 | 0 | 0 | 0 | 0 | 0 | 0 | 0 | 0 | 0 | | | | | | |
| 4650 | 05513 | 04649 | 2 | | 0 | 0 | 0 | 0 | 0 | 0 | 0 | 0 | 0 | 0 | 0 | 0 | 0 | 2 | 0 | 0 | 0 | 0 | 0 | 0 | 0 | 0 | 0 | 0 | 0 | | | | | | |
| 4651 | 05514 | 04650 | 1 | | 0 | 0 | 0 | 0 | 0 | 0 | 0 | 0 | 0 | 0 | 0 | 0 | 0 | 1 | 0 | 0 | 0 | 0 | 0 | 0 | 0 | 0 | 0 | 0 | 0 | | | | | | |
| 4652 | 05515 | 04651 | 1 | | 0 | 0 | 0 | 0 | 0 | 0 | 0 | 0 | 0 | 0 | 0 | 0 | 0 | 1 | 0 | 0 | 0 | 0 | 0 | 0 | 0 | 0 | 0 | 0 | 0 | | | | | | |
| 4653 | 05516 | 04652 | 1 | | 0 | 0 | 0 | 0 | 0 | 0 | 0 | 0 | 0 | 0 | 0 | 0 | 0 | 1 | 0 | 0 | 0 | 0 | 0 | 0 | 0 | 0 | 0 | 0 | 0 | | | | | | |
| 4654 | 05518 | 04653 | 1 | | 0 | 0 | 0 | 0 | 0 | 0 | 0 | 0 | 0 | 0 | 0 | 0 | 0 | 1 | 0 | 0 | 0 | 0 | 0 | 0 | 0 | 0 | 0 | 0 | 0 | | | | | | |
| 4655 | 05519 | 04654 | 4 | | 0 | 0 | 0 | 0 | 0 | 0 | 0 | 0 | 0 | 0 | 0 | 0 | 0 | 1 | 0 | 0 | 0 | 0 | 0 | 1 | 0 | 2 | 0 | 0 | 0 | | | | | | |
| 4656 | 05520 | 04655 | 1 | | 0 | 0 | 0 | 0 | 0 | 0 | 0 | 0 | 0 | 0 | 0 | 0 | 0 | 1 | 0 | 0 | 0 | 0 | 0 | 0 | 0 | 0 | 0 | 0 | 0 | | | | | | |
| 4657 | 05521 | 04656 | 1 | | 0 | 0 | 0 | 0 | 0 | 0 | 0 | 0 | 0 | 0 | 0 | 0 | 0 | 1 | 0 | 0 | 0 | 0 | 0 | 0 | 0 | 0 | 0 | 0 | 0 | | | | | | |
| 4658 | 05522 | 04657 | 2 | | 0 | 0 | 0 | 0 | 0 | 0 | 0 | 0 | 0 | 0 | 0 | 0 | 0 | 1 | 0 | 0 | 0 | 0 | 0 | 0 | 0 | 0 | 0 | 0 | 1 | | | | | | |
| 4659 | 05523 | 04658 | 1 | | 0 | 0 | 0 | 0 | 0 | 0 | 0 | 0 | 0 | 0 | 0 | 0 | 0 | 1 | 0 | 0 | 0 | 0 | 0 | 0 | 0 | 0 | 0 | 0 | 0 | | | | | | |
| 4660 | 05524 | 04659 | 5 | | 0 | 0 | 0 | 0 | 0 | 0 | 0 | 0 | 0 | 0 | 1 | 0 | 0 | 3 | 0 | 0 | 0 | 0 | 0 | 0 | 0 | 0 | 0 | 1 | 0 | | | | | | |
| 4661 | 05525 | 04660 | 2 | | 0 | 0 | 0 | 0 | 0 | 0 | 0 | 0 | 0 | 0 | 0 | 0 | 0 | 1 | 0 | 0 | 0 | 0 | 0 | 0 | 0 | 1 | 0 | 0 | 0 | | | | | | |
| 4662 | 05526 | 04661 | 1 | | 0 | 0 | 0 | 0 | 0 | 0 | 0 | 0 | 0 | 0 | 0 | 0 | 0 | 1 | 0 | 0 | 0 | 0 | 0 | 0 | 0 | 0 | 0 | 0 | 0 | | | | | | |
| 4663 | 05528 | 04662 | 1 | | 0 | 0 | 0 | 0 | 0 | 0 | 0 | 0 | 0 | 0 | 0 | 0 | 0 | 1 | 0 | 0 | 0 | 0 | 0 | 0 | 0 | 0 | 0 | 0 | 0 | | | | | | |
| 4664 | 05529 | 04663 | 1 | | 0 | 0 | 0 | 0 | 0 | 0 | 0 | 0 | 0 | 0 | 0 | 0 | 0 | 1 | 0 | 0 | 0 | 0 | 0 | 0 | 0 | 0 | 0 | 0 | 0 | | | | | | |
| 4665 | 05531 | 04664 | 1 | | 0 | 0 | 0 | 0 | 0 | 0 | 0 | 0 | 0 | 0 | 0 | 0 | 0 | 1 | 0 | 0 | 0 | 0 | 0 | 0 | 0 | 0 | 0 | 0 | 0 | X76104 | 95 | 121 | 1 | 5667 | 5886 |

Table 130

| | A | B | C | E | G | I | K | M | O | Q | S | U | W | Y | AA | AC | AE | AG | AI | AK | AM | AO | AQ | AS | AU | AW | AY | BA | BC | BE | BF | BG | BH | BI | BK |
|---|---|---|---|---|---|---|---|---|---|---|---|---|---|---|---|---|---|---|---|---|---|---|---|---|---|---|---|---|---|---|---|---|---|---|---|
| 4666 | 05532 | 04665 | 1 | | 0 | 0 | 0 | 0 | 0 | 0 | 0 | 0 | 0 | 0 | 0 | 0 | 0 | 1 | 0 | 0 | 0 | 0 | 0 | 0 | 0 | 0 | 0 | 0 | 0 | | | | | | |
| 4667 | 05533 | 04666 | 1 | | 0 | 0 | 0 | 0 | 0 | 0 | 0 | 0 | 0 | 0 | 0 | 0 | 0 | 1 | 0 | 0 | 0 | 0 | 0 | 0 | 0 | 0 | 0 | 0 | 0 | | | | | | |
| 4668 | 05534 | 04667 | 1 | | 0 | 0 | 0 | 0 | 0 | 0 | 0 | 0 | 0 | 0 | 0 | 0 | 0 | 1 | 0 | 0 | 0 | 0 | 0 | 0 | 0 | 0 | 0 | 0 | 0 | | | | | | |
| 4669 | 05535 | 04668 | 3 | | 0 | 0 | 0 | 0 | 0 | 0 | 0 | 0 | 0 | 0 | 0 | 0 | 0 | 3 | 0 | 0 | 0 | 0 | 0 | 0 | 0 | 0 | 0 | 0 | 0 | | | | | | |
| 4670 | 05536 | 04669 | 1 | | 0 | 0 | 0 | 0 | 0 | 0 | 0 | 0 | 0 | 0 | 0 | 0 | 0 | 1 | 0 | 0 | 0 | 0 | 0 | 0 | 0 | 0 | 0 | 0 | 0 | | | | | | |
| 4671 | 05538 | 04670 | 3 | | 0 | 0 | 0 | 0 | 0 | 0 | 0 | 0 | 0 | 0 | 0 | 0 | 0 | 3 | 0 | 0 | 0 | 0 | 0 | 0 | 0 | 0 | 0 | 0 | 0 | | | | | | |
| 4672 | 05539 | 04671 | 1 | | 0 | 0 | 0 | 0 | 0 | 0 | 0 | 0 | 0 | 0 | 0 | 0 | 0 | 1 | 0 | 0 | 0 | 0 | 0 | 0 | 0 | 0 | 0 | 0 | 0 | | | | | | |
| 4673 | 05540 | 04672 | 1 | | 0 | 0 | 0 | 0 | 0 | 0 | 0 | 0 | 0 | 0 | 0 | 0 | 0 | 1 | 0 | 0 | 0 | 0 | 0 | 0 | 0 | 0 | 0 | 0 | 0 | | | | | | |
| 4674 | 05541 | 04673 | 1 | | 0 | 0 | 0 | 0 | 0 | 0 | 0 | 0 | 0 | 0 | 0 | 0 | 0 | 1 | 0 | 0 | 0 | 0 | 0 | 0 | 0 | 0 | 0 | 0 | 0 | J04027 | 100 | 116 | 1 | 4286 | 4401 |
| 4675 | 05544 | 04674 | 1 | | 0 | 0 | 0 | 0 | 0 | 0 | 0 | 0 | 0 | 0 | 0 | 0 | 0 | 1 | 0 | 0 | 0 | 0 | 0 | 0 | 0 | 0 | 0 | 0 | 0 | | | | | | |
| 4676 | 05545 | 04675 | 2 | | 0 | 0 | 0 | 0 | 0 | 0 | 0 | 0 | 0 | 0 | 0 | 0 | 0 | 1 | 0 | 0 | 0 | 0 | 1 | 0 | 0 | 0 | 0 | 0 | 0 | | | | | | |
| 4677 | 05547 | 04676 | 2 | | 0 | 0 | 0 | 0 | 0 | 0 | 0 | 0 | 0 | 0 | 0 | 0 | 0 | 2 | 0 | 0 | 0 | 0 | 0 | 0 | 0 | 0 | 0 | 0 | 0 | Y00757 | 98.8 | 256 | 1 | 896 | 1152 |
| 4678 | 05548 | 04677 | 1 | | 0 | 0 | 0 | 0 | 0 | 0 | 0 | 0 | 0 | 0 | 0 | 0 | 0 | 1 | 0 | 0 | 0 | 0 | 0 | 0 | 0 | 0 | 0 | 0 | 0 | | | | | | |
| 4679 | 05551 | 04678 | 4 | | 0 | 0 | 0 | 0 | 0 | 0 | 0 | 0 | 0 | 0 | 0 | 0 | 0 | 1 | 0 | 0 | 0 | 0 | 1 | 2 | 0 | 0 | 0 | 0 | 0 | | | | | | |
| 4680 | 05552 | 04679 | 2 | | 0 | 0 | 0 | 0 | 0 | 0 | 0 | 0 | 0 | 0 | 0 | 0 | 0 | 1 | 0 | 0 | 0 | 1 | 0 | 0 | 0 | 0 | 0 | 0 | 0 | | | | | | |
| 4681 | 05553 | 04680 | 3 | | 0 | 0 | 0 | 0 | 0 | 0 | 0 | 0 | 0 | 0 | 0 | 0 | 0 | 2 | 0 | 0 | 0 | 0 | 1 | 0 | 0 | 0 | 0 | 0 | 0 | | | | | | |
| 4682 | 05555 | 04681 | 1 | | 0 | 0 | 0 | 0 | 0 | 0 | 0 | 0 | 0 | 0 | 0 | 0 | 0 | 1 | 0 | 0 | 0 | 0 | 0 | 0 | 0 | 0 | 0 | 0 | 0 | | | | | | |
| 4683 | 05556 | 04682 | 1 | | 0 | 0 | 0 | 0 | 0 | 0 | 0 | 0 | 0 | 0 | 0 | 0 | 0 | 1 | 0 | 0 | 0 | 0 | 0 | 0 | 0 | 0 | 0 | 0 | 0 | | | | | | |
| 4684 | 05558 | 04683 | 1 | | 0 | 0 | 0 | 0 | 0 | 0 | 0 | 0 | 0 | 0 | 0 | 0 | 0 | 1 | 0 | 0 | 0 | 0 | 0 | 0 | 0 | 0 | 0 | 0 | 0 | | | | | | |
| 4685 | 05559 | 04684 | 1 | | 0 | 0 | 0 | 0 | 0 | 0 | 0 | 0 | 0 | 0 | 0 | 0 | 0 | 1 | 0 | 0 | 0 | 0 | 0 | 0 | 0 | 0 | 0 | 0 | 0 | | | | | | |
| 4686 | 05560 | 04685 | 3 | | 0 | 0 | 0 | 0 | 0 | 0 | 0 | 0 | 0 | 0 | 0 | 0 | 0 | 3 | 0 | 0 | 0 | 0 | 0 | 0 | 0 | 0 | 0 | 0 | 0 | | | | | | |
| 4687 | 05561 | 04686 | 1 | | 0 | 0 | 0 | 0 | 0 | 0 | 0 | 0 | 0 | 0 | 0 | 0 | 0 | 1 | 0 | 0 | 0 | 0 | 0 | 0 | 0 | 0 | 0 | 0 | 0 | | | | | | |
| 4688 | 05562 | 04687 | 1 | | 0 | 0 | 0 | 0 | 0 | 0 | 0 | 0 | 0 | 0 | 0 | 0 | 0 | 1 | 0 | 0 | 0 | 0 | 0 | 0 | 0 | 0 | 0 | 0 | 0 | | | | | | |
| 4689 | 05563 | 04688 | 1 | | 0 | 0 | 0 | 0 | 0 | 0 | 0 | 0 | 0 | 0 | 0 | 0 | 0 | 1 | 0 | 0 | 0 | 0 | 0 | 0 | 0 | 0 | 0 | 0 | 0 | | | | | | |
| 4690 | 05564 | 04689 | 1 | | 0 | 0 | 0 | 0 | 0 | 0 | 0 | 0 | 0 | 0 | 0 | 0 | 0 | 1 | 0 | 0 | 0 | 0 | 0 | 0 | 0 | 0 | 0 | 0 | 0 | | | | | | |
| 4691 | 05565 | 04690 | 4 | | 0 | 0 | 0 | 0 | 0 | 0 | 0 | 0 | 0 | 0 | 0 | 0 | 0 | 1 | 0 | 1 | 0 | 0 | 1 | 0 | 0 | 1 | 0 | 0 | 0 | | | | | | |
| 4692 | 05566 | 04691 | 1 | | 0 | 0 | 0 | 0 | 0 | 0 | 0 | 0 | 0 | 0 | 0 | 0 | 0 | 1 | 0 | 0 | 0 | 0 | 0 | 0 | 0 | 0 | 0 | 0 | 0 | | | | | | |
| 4693 | 05567 | 04692 | 1 | | 0 | 0 | 0 | 0 | 0 | 0 | 0 | 0 | 0 | 0 | 0 | 0 | 0 | 1 | 0 | 0 | 0 | 0 | 0 | 0 | 0 | 0 | 0 | 0 | 0 | | | | | | |
| 4694 | 05568 | 04693 | 2 | | 0 | 0 | 0 | 0 | 0 | 0 | 0 | 0 | 0 | 0 | 0 | 0 | 0 | 1 | 0 | 0 | 0 | 0 | 1 | 0 | 0 | 0 | 0 | 0 | 0 | | | | | | |
| 4695 | 05570 | 04694 | 2 | | 0 | 0 | 0 | 0 | 0 | 0 | 0 | 0 | 0 | 0 | 0 | 0 | 0 | 2 | 0 | 0 | 0 | 0 | 0 | 0 | 0 | 0 | 0 | 0 | 0 | | | | | | |
| 4696 | 05571 | 04695 | 1 | | 0 | 0 | 0 | 0 | 0 | 0 | 0 | 0 | 0 | 0 | 0 | 0 | 0 | 1 | 0 | 0 | 0 | 0 | 0 | 0 | 0 | 0 | 0 | 0 | 0 | L06328 | 97.1 | 103 | 1 | 1302 | 1404 |
| 4697 | 05572 | 04696 | 1 | | 0 | 0 | 0 | 0 | 0 | 0 | 0 | 0 | 0 | 0 | 0 | 0 | 0 | 1 | 0 | 0 | 0 | 0 | 0 | 0 | 0 | 0 | 0 | 0 | 0 | | | | | | |
| 4698 | 05573 | 04697 | 4 | | 0 | 0 | 0 | 0 | 0 | 0 | 0 | 0 | 0 | 0 | 1 | 0 | 0 | 1 | 0 | 1 | 0 | 0 | 0 | 0 | 0 | 0 | 0 | 1 | 0 | | | | | | |
| 4699 | 05574 | 04698 | 1 | | 0 | 0 | 0 | 0 | 0 | 0 | 0 | 0 | 0 | 0 | 0 | 0 | 0 | 1 | 0 | 0 | 0 | 0 | 0 | 0 | 0 | 0 | 0 | 0 | 0 | | | | | | |
| 4700 | 05575 | 04699 | 1 | | 0 | 0 | 0 | 0 | 0 | 0 | 0 | 0 | 0 | 0 | 0 | 0 | 0 | 1 | 0 | 0 | 0 | 0 | 0 | 0 | 0 | 0 | 0 | 0 | 0 | X67734 | 98.8 | 81 | 1 | 4419 | 4548 |
| 4701 | 05576 | 04700 | 1 | | 0 | 0 | 0 | 0 | 0 | 0 | 0 | 0 | 0 | 0 | 0 | 0 | 0 | 1 | 0 | 0 | 0 | 0 | 0 | 0 | 0 | 0 | 0 | 0 | 0 | | | | | | |

Table 131

| | A | B | C | E | G | I | K | M | O | Q | S | U | W | Y | AA | AC | AE | AG | AI | AK | AM | AO | AQ | AS | AU | AW | AY | BA | BC | BE | BF | BG | BH | BI | BK |
|---|---|---|---|---|---|---|---|---|---|---|---|---|---|---|---|---|---|---|---|---|---|---|---|---|---|---|---|---|---|---|---|---|---|---|---|
| 4702 | 05577 | 04701 | 4 | | 0 | 0 | 0 | 0 | 0 | 0 | 0 | 0 | 0 | 0 | 0 | 0 | 0 | 2 | 0 | 0 | 0 | 0 | 1 | 0 | 1 | 0 | 0 | 0 | 0 | J02611 | 94.5 | 308 | 1 | 502 | 809 |
| 4703 | 05578 | 04702 | 1 | | 0 | 0 | 0 | 0 | 0 | 0 | 0 | 0 | 0 | 0 | 0 | 0 | 0 | 1 | 0 | 0 | 0 | 0 | 0 | 0 | 0 | 0 | 0 | 0 | 0 | | | | | | |
| 4704 | 05579 | 04703 | 2 | | 0 | 0 | 0 | 0 | 0 | 0 | 0 | 0 | 0 | 0 | 0 | 0 | 0 | 2 | 0 | 0 | 0 | 0 | 0 | 0 | 0 | 0 | 0 | 0 | 0 | | | | | | |
| 4705 | 05580 | 04704 | 2 | | 0 | 0 | 0 | 0 | 0 | 0 | 0 | 0 | 0 | 0 | 0 | 0 | 0 | 2 | 0 | 0 | 0 | 0 | 0 | 0 | 0 | 0 | 0 | 0 | 0 | | | | | | |
| 4706 | 05581 | 04705 | 4 | | 0 | 0 | 0 | 0 | 0 | 0 | 0 | 0 | 0 | 0 | 0 | 0 | 0 | 2 | 0 | 0 | 0 | 0 | 1 | 1 | 0 | 0 | 0 | 0 | 0 | | | | | | |
| 4707 | 05582 | 04706 | 1 | | 0 | 0 | 0 | 0 | 0 | 0 | 0 | 0 | 0 | 0 | 0 | 0 | 0 | 1 | 0 | 0 | 0 | 0 | 0 | 0 | 0 | 0 | 0 | 0 | 0 | | | | | | |
| 4708 | 05583 | 04707 | 4 | | 0 | 0 | 0 | 0 | 0 | 0 | 0 | 0 | 0 | 0 | 0 | 0 | 0 | 1 | 0 | 0 | 0 | 1 | 1 | 1 | 0 | 0 | 0 | 0 | 0 | | | | | | |
| 4709 | 05585 | 04708 | 1 | | 0 | 0 | 0 | 0 | 0 | 0 | 0 | 0 | 0 | 0 | 0 | 0 | 0 | 1 | 0 | 0 | 0 | 0 | 0 | 0 | 0 | 0 | 0 | 0 | 0 | | | | | | |
| 4710 | 05586 | 04709 | 1 | | 0 | 0 | 0 | 0 | 0 | 0 | 0 | 0 | 0 | 0 | 0 | 0 | 0 | 1 | 0 | 0 | 0 | 0 | 0 | 0 | 0 | 0 | 0 | 0 | 0 | | | | | | |
| 4711 | 05587 | 04710 | 4 | | 0 | 0 | 0 | 0 | 0 | 0 | 0 | 0 | 0 | 0 | 0 | 0 | 0 | 2 | 0 | 0 | 0 | 0 | 0 | 0 | 0 | 0 | 0 | 2 | 0 | | | | | | |
| 4712 | 05588 | 04711 | 2 | | 0 | 0 | 0 | 0 | 0 | 0 | 0 | 0 | 0 | 0 | 0 | 0 | 0 | 1 | 0 | 1 | 0 | 0 | 0 | 0 | 0 | 0 | 0 | 0 | 0 | | | | | | |
| 4713 | 05589 | 04712 | 1 | | 0 | 0 | 0 | 0 | 0 | 0 | 0 | 0 | 0 | 0 | 0 | 0 | 0 | 1 | 0 | 0 | 0 | 0 | 0 | 0 | 0 | 0 | 0 | 0 | 0 | | | | | | |
| 4714 | 05590 | 04713 | 3 | | 0 | 0 | 0 | 0 | 0 | 0 | 0 | 0 | 0 | 0 | 0 | 0 | 0 | 1 | 0 | 0 | 0 | 0 | 2 | 0 | 0 | 0 | 0 | 0 | 0 | | | | | | |
| 4715 | 05591 | 04714 | 1 | | 0 | 0 | 0 | 0 | 0 | 0 | 0 | 0 | 0 | 0 | 0 | 0 | 0 | 1 | 0 | 0 | 0 | 0 | 0 | 0 | 0 | 0 | 0 | 0 | 0 | | | | | | |
| 4716 | 05593 | 04715 | 3 | | 0 | 0 | 0 | 0 | 0 | 0 | 0 | 0 | 0 | 0 | 0 | 0 | 0 | 1 | 0 | 0 | 0 | 0 | 1 | 1 | 0 | 0 | 0 | 0 | 0 | M25667 | 99.1 | 114 | 1 | 1118 | 1231 |
| 4717 | 05595 | 04716 | 2 | | 0 | 0 | 0 | 0 | 0 | 0 | 0 | 0 | 0 | 0 | 0 | 0 | 0 | 1 | 0 | 0 | 0 | 1 | 0 | 0 | 0 | 0 | 0 | 0 | 0 | | | | | | |
| 4718 | 05598 | 04717 | 1 | | 0 | 0 | 0 | 0 | 0 | 0 | 0 | 0 | 0 | 0 | 0 | 0 | 0 | 1 | 0 | 0 | 0 | 0 | 0 | 0 | 0 | 0 | 0 | 0 | 0 | | | | | | |
| 4719 | 05599 | 04718 | 1 | | 0 | 0 | 0 | 0 | 0 | 0 | 0 | 0 | 0 | 0 | 0 | 0 | 0 | 1 | 0 | 0 | 0 | 0 | 0 | 0 | 0 | 0 | 0 | 0 | 0 | | | | | | |
| 4720 | 05600 | 04719 | 2 | | 0 | 0 | 0 | 0 | 0 | 0 | 0 | 0 | 0 | 0 | 0 | 0 | 0 | 1 | 0 | 0 | 0 | 0 | 1 | 0 | 0 | 0 | 0 | 0 | 0 | M23115 | 97.5 | 80 | 1 | 3702 | 3781 |
| 4721 | 05601 | 04720 | 1 | | 0 | 0 | 0 | 0 | 0 | 0 | 0 | 0 | 0 | 0 | 0 | 0 | 0 | 1 | 0 | 0 | 0 | 0 | 0 | 0 | 0 | 0 | 0 | 0 | 0 | | | | | | |
| 4722 | 05602 | 04721 | 1 | | 0 | 0 | 0 | 0 | 0 | 0 | 0 | 0 | 0 | 0 | 0 | 0 | 0 | 1 | 0 | 0 | 0 | 0 | 0 | 0 | 0 | 0 | 0 | 0 | 0 | | | | | | |
| 4723 | 05603 | 04722 | 1 | | 0 | 0 | 0 | 0 | 0 | 0 | 0 | 0 | 0 | 0 | 0 | 0 | 0 | 1 | 0 | 0 | 0 | 0 | 0 | 0 | 0 | 0 | 0 | 0 | 0 | | | | | | |
| 4724 | 05604 | 04723 | 1 | | 0 | 0 | 0 | 0 | 0 | 0 | 0 | 0 | 0 | 0 | 0 | 0 | 0 | 1 | 0 | 0 | 0 | 0 | 0 | 0 | 0 | 0 | 0 | 0 | 0 | | | | | | |
| 4725 | 05606 | 04724 | 1 | | 0 | 0 | 0 | 0 | 0 | 0 | 0 | 0 | 0 | 0 | 0 | 0 | 0 | 1 | 0 | 0 | 0 | 0 | 0 | 0 | 0 | 0 | 0 | 0 | 0 | | | | | | |
| 4726 | 05607 | 04725 | 1 | | 0 | 0 | 0 | 0 | 0 | 0 | 0 | 0 | 0 | 0 | 0 | 0 | 0 | 1 | 0 | 0 | 0 | 0 | 0 | 0 | 0 | 0 | 0 | 0 | 0 | | | | | | |
| 4727 | 05608 | 04726 | 1 | | 0 | 0 | 0 | 0 | 0 | 0 | 0 | 0 | 0 | 0 | 0 | 0 | 0 | 1 | 0 | 0 | 0 | 0 | 0 | 0 | 0 | 0 | 0 | 0 | 0 | | | | | | |
| 4728 | 05610 | 04727 | 1 | | 0 | 0 | 0 | 0 | 0 | 0 | 0 | 0 | 0 | 0 | 0 | 0 | 0 | 1 | 0 | 0 | 0 | 0 | 0 | 0 | 0 | 0 | 0 | 0 | 0 | | | | | | |
| 4729 | 05612 | 04728 | 2 | | 0 | 0 | 0 | 0 | 0 | 0 | 0 | 0 | 0 | 0 | 0 | 0 | 0 | 1 | 0 | 0 | 0 | 0 | 0 | 0 | 0 | 0 | 1 | 0 | 0 | | | | | | |
| 4730 | 05613 | 04729 | 1 | | 0 | 0 | 0 | 0 | 0 | 0 | 0 | 0 | 0 | 0 | 0 | 0 | 0 | 1 | 0 | 0 | 0 | 0 | 0 | 0 | 0 | 0 | 0 | 0 | 0 | | | | | | |
| 4731 | 05614 | 04730 | 2 | | 0 | 0 | 0 | 0 | 0 | 0 | 0 | 0 | 0 | 0 | 0 | 0 | 0 | 2 | 0 | 0 | 0 | 0 | 0 | 0 | 0 | 0 | 0 | 0 | 0 | | | | | | |
| 4732 | 05615 | 04731 | 1 | | 0 | 0 | 0 | 0 | 0 | 0 | 0 | 0 | 0 | 0 | 0 | 0 | 0 | 1 | 0 | 0 | 0 | 0 | 0 | 0 | 0 | 0 | 0 | 0 | 0 | | | | | | |
| 4733 | 05616 | 04732 | 1 | | 0 | 0 | 0 | 0 | 0 | 0 | 0 | 0 | 0 | 0 | 0 | 0 | 0 | 1 | 0 | 0 | 0 | 0 | 0 | 0 | 0 | 0 | 0 | 0 | 0 | | | | | | |
| 4734 | 05618 | 04733 | 1 | | 0 | 0 | 0 | 0 | 0 | 0 | 0 | 0 | 0 | 0 | 0 | 0 | 0 | 1 | 0 | 0 | 0 | 0 | 0 | 0 | 0 | 0 | 0 | 0 | 0 | M74491 | 96.9 | 294 | 1 | 725 | 3595 |
| 4735 | 05619 | 04734 | 1 | | 0 | 0 | 0 | 0 | 0 | 0 | 0 | 0 | 0 | 0 | 0 | 0 | 0 | 1 | 0 | 0 | 0 | 0 | 0 | 0 | 0 | 0 | 0 | 0 | 0 | M27436 | 95.5 | 292 | 1 | 1590 | 2127 |
| 4736 | 05621 | 04735 | 1 | | 0 | 0 | 0 | 0 | 0 | 0 | 0 | 0 | 0 | 0 | 0 | 0 | 0 | 1 | 0 | 0 | 0 | 0 | 0 | 0 | 0 | 0 | 0 | 0 | 0 | | | | | | |
| 4737 | 05622 | 04736 | 1 | | 0 | 0 | 0 | 0 | 0 | 0 | 0 | 0 | 0 | 0 | 0 | 0 | 0 | 1 | 0 | 0 | 0 | 0 | 0 | 0 | 0 | 0 | 0 | 0 | 0 | | | | | | |

Table 132

| | A | B | C | E | G | I | K | M | O | Q | S | U | W | Y | AA | AC | AE | AG | AI | AK | AM | AO | AQ | AS | AU | AW | AY | BA | BC | BE | BF | BG | BH | BI | BK |
|---|---|---|---|---|---|---|---|---|---|---|---|---|---|---|---|---|---|---|---|---|---|---|---|---|---|---|---|---|---|---|---|---|---|---|---|
| 4738 | 05623 | 04737 | 1 | | 0 | 0 | 0 | 0 | 0 | 0 | 0 | 0 | 0 | 0 | 0 | 0 | 0 | 1 | 0 | 0 | 0 | 0 | 0 | 0 | 0 | 0 | 0 | 0 | 0 | | | | | | |
| 4739 | 05624 | 04738 | 3 | | 0 | 0 | 0 | 0 | 0 | 0 | 0 | 0 | 0 | 0 | 0 | 0 | 0 | 1 | 0 | 0 | 0 | 2 | 0 | 0 | 0 | 0 | 0 | 0 | 0 | | | | | | |
| 4740 | 05625 | 04739 | 3 | | 0 | 0 | 0 | 0 | 0 | 0 | 0 | 0 | 0 | 0 | 0 | 0 | 0 | 2 | 0 | 0 | 0 | 0 | 1 | 0 | 0 | 0 | 0 | 0 | 0 | | | | | | |
| 4741 | 05627 | 04740 | 1 | | 0 | 0 | 0 | 0 | 0 | 0 | 0 | 0 | 0 | 0 | 0 | 0 | 0 | 1 | 0 | 0 | 0 | 0 | 0 | 0 | 0 | 0 | 0 | 0 | 0 | | | | | | |
| 4742 | 05628 | 04741 | 1 | | 0 | 0 | 0 | 0 | 0 | 0 | 0 | 0 | 0 | 0 | 0 | 0 | 0 | 1 | 0 | 0 | 0 | 0 | 0 | 0 | 0 | 0 | 0 | 0 | 0 | | | | | | |
| 4743 | 05629 | 04742 | 2 | | 0 | 0 | 0 | 0 | 0 | 0 | 0 | 0 | 0 | 0 | 0 | 0 | 0 | 1 | 0 | 0 | 0 | 0 | 1 | 0 | 0 | 0 | 0 | 0 | 0 | | | | | | |
| 4744 | 05630 | 04743 | 1 | | 0 | 0 | 0 | 0 | 0 | 0 | 0 | 0 | 0 | 0 | 0 | 0 | 0 | 1 | 0 | 0 | 0 | 0 | 0 | 0 | 0 | 0 | 0 | 0 | 0 | | | | | | |
| 4745 | 05631 | 04744 | 2 | | 0 | 0 | 0 | 0 | 0 | 0 | 0 | 0 | 0 | 0 | 0 | 0 | 0 | 2 | 0 | 0 | 0 | 0 | 0 | 0 | 0 | 0 | 0 | 0 | 0 | | | | | | |
| 4746 | 05632 | 04745 | 4 | | 0 | 0 | 0 | 0 | 0 | 0 | 0 | 0 | 0 | 0 | 0 | 0 | 0 | 2 | 0 | 1 | 0 | 0 | 0 | 0 | 0 | 1 | 0 | 0 | 0 | | | | | | |
| 4747 | 05634 | 04746 | 1 | | 0 | 0 | 0 | 0 | 0 | 0 | 0 | 0 | 0 | 0 | 0 | 0 | 0 | 1 | 0 | 0 | 0 | 0 | 0 | 0 | 0 | 0 | 0 | 0 | 0 | | | | | | |
| 4748 | 05635 | 04747 | 1 | | 0 | 0 | 0 | 0 | 0 | 0 | 0 | 0 | 0 | 0 | 0 | 0 | 0 | 1 | 0 | 0 | 0 | 0 | 0 | 0 | 0 | 0 | 0 | 0 | 0 | | | | | | |
| 4749 | 05636 | 04748 | 2 | | 0 | 0 | 0 | 0 | 0 | 0 | 0 | 0 | 0 | 0 | 0 | 0 | 0 | 2 | 0 | 0 | 0 | 0 | 0 | 0 | 0 | 0 | 0 | 0 | 0 | | | | | | |
| 4750 | 05637 | 04749 | 1 | | 0 | 0 | 0 | 0 | 0 | 0 | 0 | 0 | 0 | 0 | 0 | 0 | 0 | 1 | 0 | 0 | 0 | 0 | 0 | 0 | 0 | 0 | 0 | 0 | 0 | | | | | | |
| 4751 | 05639 | 04750 | 4 | | 0 | 0 | 0 | 0 | 0 | 0 | 0 | 0 | 0 | 0 | 0 | 0 | 0 | 1 | 0 | 0 | 0 | 0 | 2 | 1 | 0 | 0 | 0 | 0 | 0 | | | | | | |
| 4752 | 05640 | 04751 | 1 | | 0 | 0 | 0 | 0 | 0 | 0 | 0 | 0 | 0 | 0 | 0 | 0 | 0 | 1 | 0 | 0 | 0 | 0 | 0 | 0 | 0 | 0 | 0 | 0 | 0 | | | | | | |
| 4753 | 05642 | 04752 | 2 | | 0 | 0 | 0 | 0 | 0 | 0 | 0 | 0 | 0 | 0 | 0 | 0 | 0 | 2 | 0 | 0 | 0 | 0 | 0 | 0 | 0 | 0 | 0 | 0 | 0 | | | | | | |
| 4754 | 05643 | 04753 | 2 | | 0 | 0 | 0 | 0 | 0 | 0 | 0 | 0 | 0 | 0 | 0 | 0 | 0 | 1 | 0 | 0 | 0 | 0 | 0 | 1 | 0 | 0 | 0 | 0 | 0 | | | | | | |
| 4755 | 05644 | 04754 | 4 | | 0 | 0 | 0 | 0 | 0 | 0 | 0 | 0 | 0 | 0 | 0 | 0 | 0 | 2 | 0 | 0 | 0 | 1 | 0 | 0 | 0 | 0 | 0 | 1 | 0 | | | | | | |
| 4756 | 05645 | 04755 | 2 | | 0 | 0 | 0 | 0 | 0 | 0 | 0 | 0 | 0 | 0 | 0 | 0 | 0 | 1 | 0 | 0 | 0 | 0 | 0 | 0 | 0 | 1 | 0 | 0 | 0 | | | | | | |
| 4757 | 05646 | 04756 | 1 | | 0 | 0 | 0 | 0 | 0 | 0 | 0 | 0 | 0 | 0 | 0 | 0 | 0 | 1 | 0 | 0 | 0 | 0 | 0 | 0 | 0 | 0 | 0 | 0 | 0 | | | | | | |
| 4758 | 05647 | 04757 | 5 | | 0 | 0 | 0 | 0 | 0 | 0 | 0 | 0 | 0 | 0 | 0 | 0 | 0 | 1 | 0 | 1 | 1 | 0 | 0 | 1 | 0 | 0 | 0 | 1 | 0 | | | | | | |
| 4759 | 05648 | 04758 | 2 | | 0 | 0 | 0 | 0 | 0 | 0 | 0 | 0 | 0 | 0 | 0 | 0 | 0 | 1 | 0 | 0 | 0 | 1 | 0 | 0 | 0 | 0 | 0 | 0 | 0 | X04741 | 99.7 | 310 | 1 | 330 | 1014 |
| 4760 | 05649 | 04759 | 1 | | 0 | 0 | 0 | 0 | 0 | 0 | 0 | 0 | 0 | 0 | 0 | 0 | 0 | 1 | 0 | 0 | 0 | 0 | 0 | 0 | 0 | 0 | 0 | 0 | 0 | | | | | | |
| 4761 | 05650 | 04760 | 4 | | 0 | 0 | 0 | 0 | 0 | 0 | 0 | 0 | 0 | 0 | 0 | 0 | 0 | 1 | 0 | 0 | 0 | 0 | 2 | 1 | 0 | 0 | 0 | 0 | 0 | | | | | | |
| 4762 | 05651 | 04761 | 1 | | 0 | 0 | 0 | 0 | 0 | 0 | 0 | 0 | 0 | 0 | 0 | 0 | 0 | 1 | 0 | 0 | 0 | 0 | 0 | 0 | 0 | 0 | 0 | 0 | 0 | L12136 | 99 | 307 | 1 | 94 | 1831 |
| 4763 | 05652 | 04762 | 3 | | 0 | 0 | 0 | 0 | 0 | 0 | 0 | 0 | 0 | 0 | 0 | 0 | 0 | 1 | 0 | 1 | 0 | 0 | 0 | 0 | 0 | 1 | 0 | 0 | 0 | | | | | | |
| 4764 | 05653 | 04763 | 1 | | 0 | 0 | 0 | 0 | 0 | 0 | 0 | 0 | 0 | 0 | 0 | 0 | 0 | 1 | 0 | 0 | 0 | 0 | 0 | 0 | 0 | 0 | 0 | 0 | 0 | M36661 | 96.9 | 257 | 1 | 1338 | 1598 |
| 4765 | 05654 | 04764 | 1 | | 0 | 0 | 0 | 0 | 0 | 0 | 0 | 0 | 0 | 0 | 0 | 0 | 0 | 1 | 0 | 0 | 0 | 0 | 0 | 0 | 0 | 0 | 0 | 0 | 0 | | | | | | |
| 4766 | 05655 | 04765 | 2 | | 0 | 0 | 0 | 0 | 0 | 0 | 0 | 0 | 0 | 0 | 0 | 0 | 0 | 1 | 0 | 0 | 0 | 0 | 0 | 0 | 0 | 0 | 0 | 1 | 0 | | | | | | |
| 4767 | 05656 | 04766 | 1 | | 0 | 0 | 0 | 0 | 0 | 0 | 0 | 0 | 0 | 0 | 0 | 0 | 0 | 1 | 0 | 0 | 0 | 0 | 0 | 0 | 0 | 0 | 0 | 0 | 0 | | | | | | |
| 4768 | 05657 | 04767 | 1 | | 0 | 0 | 0 | 0 | 0 | 0 | 0 | 0 | 0 | 0 | 0 | 0 | 0 | 1 | 0 | 0 | 0 | 0 | 0 | 0 | 0 | 0 | 0 | 0 | 0 | | | | | | |
| 4769 | 05658 | 04768 | 1 | | 0 | 0 | 0 | 0 | 0 | 0 | 0 | 0 | 0 | 0 | 0 | 0 | 0 | 1 | 0 | 0 | 0 | 0 | 0 | 0 | 0 | 0 | 0 | 0 | 0 | X01410 | 96.3 | 299 | 1 | 506 | 801 |
| 4770 | 05659 | 04769 | 1 | | 0 | 0 | 0 | 0 | 0 | 0 | 0 | 0 | 0 | 0 | 0 | 0 | 0 | 1 | 0 | 0 | 0 | 0 | 0 | 0 | 0 | 0 | 0 | 0 | 0 | | | | | | |
| 4771 | 05660 | 04770 | 1 | | 0 | 0 | 0 | 0 | 0 | 0 | 0 | 0 | 0 | 0 | 0 | 0 | 0 | 1 | 0 | 0 | 0 | 0 | 0 | 0 | 0 | 0 | 0 | 0 | 0 | | | | | | |
| 4772 | 05661 | 04771 | 1 | | 0 | 0 | 0 | 0 | 0 | 0 | 0 | 0 | 0 | 0 | 0 | 0 | 0 | 1 | 0 | 0 | 0 | 0 | 0 | 0 | 0 | 0 | 0 | 0 | 0 | L08112 | 94.6 | 111 | 1 | 1011 | 1344 |
| 4773 | 05662 | 04772 | 1 | | 0 | 0 | 0 | 0 | 0 | 0 | 0 | 0 | 0 | 0 | 0 | 0 | 0 | 1 | 0 | 0 | 0 | 0 | 0 | 0 | 0 | 0 | 0 | 0 | 0 | | | | | | |

Table 133

| | A | B | C | E | G | I | K | M | O | Q | S | U | W | Y | AA | AC | AF | AG | AI | AK | AM | AO | AQ | AS | AU | AW | AY | BA | BC | BE | BF | BG | BH | BI | BK |
|---|---|---|---|---|---|---|---|---|---|---|---|---|---|---|---|---|---|---|---|---|---|---|---|---|---|---|---|---|---|---|---|---|---|---|---|
| 4774 | 05665 | 04773 | 1 | | 0 | 0 | 0 | 0 | 0 | 0 | 0 | 0 | 0 | 0 | 0 | 0 | 0 | 1 | 0 | 0 | 0 | 0 | 0 | 0 | 0 | 0 | 0 | 0 | 0 | | | | | | |
| 4775 | 05667 | 04774 | 2 | | 0 | 0 | 0 | 0 | 0 | 0 | 0 | 0 | 0 | 0 | 0 | 0 | 0 | 1 | 0 | 0 | 0 | 0 | 0 | 0 | 0 | 0 | 1 | 0 | 0 | | | | | | |
| 4776 | 05668 | 04775 | 4 | | 0 | 0 | 0 | 0 | 0 | 0 | 0 | 0 | 0 | 0 | 0 | 0 | 0 | 1 | 0 | 0 | 2 | 0 | 1 | 0 | 0 | 0 | 0 | 0 | 0 | | | | | | |
| 4777 | 05669 | 04776 | 1 | | 0 | 0 | 0 | 0 | 0 | 0 | 0 | 0 | 0 | 0 | 0 | 0 | 0 | 1 | 0 | 0 | 0 | 0 | 0 | 0 | 0 | 0 | 0 | 0 | 0 | | | | | | |
| 4778 | 05671 | 04777 | 1 | | 0 | 0 | 0 | 0 | 0 | 0 | 0 | 0 | 0 | 0 | 0 | 0 | 0 | 1 | 0 | 0 | 0 | 0 | 0 | 0 | 0 | 0 | 0 | 0 | 0 | | | | | | |
| 4779 | 05673 | 04778 | 1 | | 0 | 0 | 0 | 0 | 0 | 0 | 0 | 0 | 0 | 0 | 0 | 0 | 0 | 1 | 0 | 0 | 0 | 0 | 0 | 0 | 0 | 0 | 0 | 0 | 0 | | | | | | |
| 4780 | 05674 | 04779 | 1 | | 0 | 0 | 0 | 0 | 0 | 0 | 0 | 0 | 0 | 0 | 0 | 0 | 0 | 1 | 0 | 0 | 0 | 0 | 0 | 0 | 0 | 0 | 0 | 0 | 0 | | | | | | |
| 4781 | 05676 | 04780 | 2 | | 0 | 0 | 0 | 0 | 0 | 0 | 0 | 0 | 0 | 0 | 0 | 0 | 0 | 1 | 0 | 1 | 0 | 0 | 0 | 0 | 0 | 0 | 0 | 0 | 0 | | | | | | |
| 4782 | 05679 | 04781 | 1 | | 0 | 0 | 0 | 0 | 0 | 0 | 0 | 0 | 0 | 0 | 0 | 0 | 0 | 1 | 0 | 0 | 0 | 0 | 0 | 0 | 0 | 0 | 0 | 0 | 0 | | | | | | |
| 4783 | 05680 | 04782 | 2 | | 0 | 0 | 0 | 0 | 0 | 0 | 0 | 0 | 0 | 0 | 0 | 0 | 0 | 1 | 0 | 0 | 0 | 0 | 1 | 0 | 0 | 0 | 0 | 0 | 0 | | | | | | |
| 4784 | 05681 | 04783 | 1 | | 0 | 0 | 0 | 0 | 0 | 0 | 0 | 0 | 0 | 0 | 0 | 0 | 0 | 1 | 0 | 0 | 0 | 0 | 0 | 0 | 0 | 0 | 0 | 0 | 0 | | | | | | |
| 4785 | 05682 | 04784 | 3 | | 0 | 0 | 0 | 0 | 0 | 0 | 0 | 0 | 0 | 0 | 0 | 0 | 0 | 1 | 0 | 0 | 0 | 0 | 0 | 0 | 0 | 0 | 0 | 2 | 0 | | | | | | |
| 4786 | 05683 | 04785 | 1 | | 0 | 0 | 0 | 0 | 0 | 0 | 0 | 0 | 0 | 0 | 0 | 0 | 0 | 1 | 0 | 0 | 0 | 0 | 0 | 0 | 0 | 0 | 0 | 0 | 0 | | | | | | |
| 4787 | 05684 | 04786 | 1 | | 0 | 0 | 0 | 0 | 0 | 0 | 0 | 0 | 0 | 0 | 0 | 0 | 0 | 1 | 0 | 0 | 0 | 0 | 0 | 0 | 0 | 0 | 0 | 0 | 0 | | | | | | |
| 4788 | 05685 | 04787 | 3 | | 0 | 0 | 0 | 0 | 0 | 0 | 0 | 0 | 0 | 0 | 0 | 0 | 0 | 2 | 0 | 0 | 0 | 0 | 0 | 1 | 0 | 0 | 0 | 0 | 0 | | | | | | |
| 4789 | 05686 | 04788 | 1 | | 0 | 0 | 0 | 0 | 0 | 0 | 0 | 0 | 0 | 0 | 0 | 0 | 0 | 1 | 0 | 0 | 0 | 0 | 0 | 0 | 0 | 0 | 0 | 0 | 0 | | | | | | |
| 4790 | 05687 | 04789 | 3 | | 0 | 0 | 0 | 0 | 0 | 0 | 0 | 0 | 0 | 0 | 0 | 0 | 0 | 1 | 0 | 0 | 0 | 0 | 0 | 2 | 0 | 0 | 0 | 0 | 0 | | | | | | |
| 4791 | 05688 | 04790 | 3 | | 0 | 0 | 0 | 0 | 0 | 0 | 0 | 0 | 0 | 0 | 0 | 0 | 0 | 1 | 0 | 0 | 0 | 1 | 0 | 0 | 0 | 0 | 0 | 1 | 0 | X62078 | 99.2 | 248 | 1 | 2189 | 2436 |
| 4792 | 05689 | 04791 | 1 | | 0 | 0 | 0 | 0 | 0 | 0 | 0 | 0 | 0 | 0 | 0 | 0 | 0 | 1 | 0 | 0 | 0 | 0 | 0 | 0 | 0 | 0 | 0 | 0 | 0 | | | | | | |
| 4793 | 05690 | 04792 | 2 | | 0 | 0 | 0 | 0 | 0 | 0 | 0 | 0 | 0 | 0 | 0 | 0 | 0 | 1 | 0 | 0 | 0 | 0 | 0 | 0 | 0 | 1 | 0 | 0 | 0 | | | | | | |
| 4794 | 05692 | 04793 | 1 | | 0 | 0 | 0 | 0 | 0 | 0 | 0 | 0 | 0 | 0 | 0 | 0 | 0 | 1 | 0 | 0 | 0 | 0 | 0 | 0 | 0 | 0 | 0 | 0 | 0 | | | | | | |
| 4795 | 05693 | 04794 | 3 | | 0 | 0 | 0 | 0 | 0 | 0 | 0 | 0 | 0 | 0 | 0 | 0 | 0 | 1 | 0 | 0 | 0 | 1 | 0 | 1 | 0 | 0 | 0 | 0 | 0 | J05474 | 99.1 | 222 | 1 | 1133 | 1354 |
| 4796 | 05694 | 04795 | 1 | | 0 | 0 | 0 | 0 | 0 | 0 | 0 | 0 | 0 | 0 | 0 | 0 | 0 | 1 | 0 | 0 | 0 | 0 | 0 | 0 | 0 | 0 | 0 | 0 | 0 | M93119 | 94.3 | 229 | 1 | 2593 | 2838 |
| 4797 | 05695 | 04796 | 1 | | 0 | 0 | 0 | 0 | 0 | 0 | 0 | 0 | 0 | 0 | 0 | 0 | 0 | 1 | 0 | 0 | 0 | 0 | 0 | 0 | 0 | 0 | 0 | 0 | 0 | | | | | | |
| 4798 | 05696 | 04797 | 2 | | 0 | 0 | 0 | 0 | 0 | 0 | 0 | 0 | 0 | 0 | 0 | 0 | 0 | 1 | 0 | 0 | 0 | 0 | 0 | 0 | 1 | 0 | 0 | 0 | 0 | | | | | | |
| 4799 | 05697 | 04798 | 2 | | 0 | 0 | 0 | 0 | 0 | 0 | 0 | 0 | 0 | 0 | 0 | 0 | 0 | 0 | 0 | 0 | 1 | 0 | 0 | 0 | 0 | 0 | 0 | 1 | 0 | | | | | | |
| 4800 | 05701 | 04799 | 1 | | 0 | 0 | 0 | 0 | 0 | 0 | 0 | 0 | 0 | 0 | 0 | 0 | 0 | 0 | 0 | 0 | 1 | 0 | 0 | 0 | 0 | 0 | 0 | 0 | 0 | M28650 | 98.6 | 145 | 1 | 950 | 1098 |
| 4801 | 05704 | 04800 | 1 | | 0 | 0 | 0 | 0 | 0 | 0 | 0 | 0 | 0 | 0 | 0 | 0 | 0 | 0 | 0 | 0 | 1 | 0 | 0 | 0 | 0 | 0 | 0 | 0 | 0 | | | | | | |
| 4802 | 05707 | 04801 | 2 | | 0 | 0 | 0 | 0 | 0 | 0 | 0 | 0 | 0 | 0 | 0 | 0 | 0 | 0 | 0 | 0 | 2 | 0 | 0 | 0 | 0 | 0 | 0 | 0 | 0 | | | | | | |
| 4803 | 05708 | 04802 | 1 | | 0 | 0 | 0 | 0 | 0 | 0 | 0 | 0 | 0 | 0 | 0 | 0 | 0 | 0 | 0 | 0 | 1 | 0 | 0 | 0 | 0 | 0 | 0 | 0 | 0 | | | | | | |
| 4804 | 05709 | 04803 | 1 | | 0 | 0 | 0 | 0 | 0 | 0 | 0 | 0 | 0 | 0 | 0 | 0 | 0 | 0 | 0 | 0 | 1 | 0 | 0 | 0 | 0 | 0 | 0 | 0 | 0 | | | | | | |
| 4805 | 05710 | 04804 | 5 | | 0 | 0 | 0 | 0 | 0 | 0 | 0 | 0 | 0 | 0 | 1 | 0 | 0 | 0 | 0 | 0 | 3 | 0 | 0 | 0 | 0 | 1 | 0 | 0 | 0 | | | | | | |
| 4806 | 05711 | 04805 | 1 | | 0 | 0 | 0 | 0 | 0 | 0 | 0 | 0 | 0 | 0 | 0 | 0 | 0 | 0 | 0 | 0 | 1 | 0 | 0 | 0 | 0 | 0 | 0 | 0 | 0 | M63256 | 96.6 | 117 | 1 | 2439 | 2570 |
| 4807 | 05714 | 04806 | 1 | | 0 | 0 | 0 | 0 | 0 | 0 | 0 | 0 | 0 | 0 | 0 | 0 | 0 | 0 | 0 | 0 | 1 | 0 | 0 | 0 | 0 | 0 | 0 | 0 | 0 | | | | | | |
| 4808 | 05715 | 04807 | 1 | | 0 | 0 | 0 | 0 | 0 | 0 | 0 | 0 | 0 | 0 | 0 | 0 | 0 | 0 | 0 | 0 | 1 | 0 | 0 | 0 | 0 | 0 | 0 | 0 | 0 | | | | | | |
| 4809 | 05716 | 04808 | 1 | | 0 | 0 | 0 | 0 | 0 | 0 | 0 | 0 | 0 | 0 | 0 | 0 | 0 | 0 | 0 | 0 | 1 | 0 | 0 | 0 | 0 | 0 | 0 | 0 | 0 | | | | | | |

Table 134

| | A | B | C | E | G | I | K | M | O | Q | S | U | W | Y | AA | AC | AE | AG | AI | AK | AM | AO | AQ | AS | AU | AW | AY | BA | BC | BE | BF | BG | BH | BI | BK |
|---|---|---|---|---|---|---|---|---|---|---|---|---|---|---|---|---|---|---|---|---|---|---|---|---|---|---|---|---|---|---|---|---|---|---|---|
| 4810 | 05718 | 04809 | 1 | | 0 | 0 | 0 | 0 | 0 | 0 | 0 | 0 | 0 | 0 | 0 | 0 | 0 | 0 | 0 | 0 | 1 | 0 | 0 | 0 | 0 | 0 | 0 | 0 | 0 | J05392 | 98.2 | 284 | 1 | 2120 | 2402 |
| 4811 | 05719 | 04810 | 1 | | 0 | 0 | 0 | 0 | 0 | 0 | 0 | 0 | 0 | 0 | 0 | 0 | 0 | 0 | 0 | 0 | 1 | 0 | 0 | 0 | 0 | 0 | 0 | 0 | 0 | | | | | | |
| 4812 | 05720 | 04811 | 1 | | 0 | 0 | 0 | 0 | 0 | 0 | 0 | 0 | 0 | 0 | 0 | 0 | 0 | 0 | 0 | 0 | 1 | 0 | 0 | 0 | 0 | 0 | 0 | 0 | 0 | | | | | | |
| 4813 | 05721 | 04812 | 2 | | 0 | 0 | 0 | 0 | 0 | 0 | 0 | 0 | 0 | 0 | 0 | 0 | 0 | 0 | 0 | 0 | 1 | 0 | 0 | 0 | 1 | 0 | 0 | 0 | 0 | K03002 | 95.4 | 306 | 1 | 327 | 632 |
| 4814 | 05722 | 04813 | 1 | | 0 | 0 | 0 | 0 | 0 | 0 | 0 | 0 | 0 | 0 | 0 | 0 | 0 | 0 | 0 | 0 | 1 | 0 | 0 | 0 | 0 | 0 | 0 | 0 | 0 | M86667 | 98.4 | 63 | 1 | 802 | 1560 |
| 4815 | 05723 | 04814 | 3 | | 0 | 0 | 0 | 0 | 0 | 0 | 0 | 0 | 0 | 0 | 0 | 0 | 0 | 0 | 0 | 0 | 2 | 0 | 0 | 0 | 0 | 1 | 0 | 0 | 0 | | | | | | |
| 4816 | 05724 | 04815 | 2 | | 0 | 0 | 0 | 0 | 0 | 0 | 0 | 0 | 0 | 0 | 0 | 0 | 0 | 0 | 0 | 0 | 1 | 0 | 0 | 0 | 0 | 1 | 0 | 0 | 0 | | | | | | |
| 4817 | 05725 | 04816 | 1 | | 0 | 0 | 0 | 0 | 0 | 0 | 0 | 0 | 0 | 0 | 0 | 0 | 0 | 0 | 0 | 0 | 1 | 0 | 0 | 0 | 0 | 0 | 0 | 0 | 0 | | | | | | |
| 4818 | 05726 | 04817 | 1 | | 0 | 0 | 0 | 0 | 0 | 0 | 0 | 0 | 0 | 0 | 0 | 0 | 0 | 0 | 0 | 0 | 1 | 0 | 0 | 0 | 0 | 0 | 0 | 0 | 0 | | | | | | |
| 4819 | 05727 | 04818 | 2 | | 0 | 0 | 0 | 0 | 0 | 0 | 0 | 0 | 0 | 0 | 0 | 0 | 0 | 0 | 0 | 0 | 1 | 0 | 1 | 0 | 0 | 0 | 0 | 0 | 0 | L06105 | 100 | 60 | 1 | 1297 | 1649 |
| 4820 | 05729 | 04819 | 1 | | 0 | 0 | 0 | 0 | 0 | 0 | 0 | 0 | 0 | 0 | 0 | 0 | 0 | 0 | 0 | 0 | 1 | 0 | 0 | 0 | 0 | 0 | 0 | 0 | 0 | | | | | | |
| 4821 | 05730 | 04820 | 1 | | 0 | 0 | 0 | 0 | 0 | 0 | 0 | 0 | 0 | 0 | 0 | 0 | 0 | 0 | 0 | 0 | 1 | 0 | 0 | 0 | 0 | 0 | 0 | 0 | 0 | | | | | | |
| 4822 | 05731 | 04821 | 1 | | 0 | 0 | 0 | 0 | 0 | 0 | 0 | 0 | 0 | 0 | 0 | 0 | 0 | 0 | 0 | 0 | 1 | 0 | 0 | 0 | 0 | 0 | 0 | 0 | 0 | | | | | | |
| 4823 | 05732 | 04822 | 1 | | 0 | 0 | 0 | 0 | 0 | 0 | 0 | 0 | 0 | 0 | 0 | 0 | 0 | 0 | 0 | 0 | 1 | 0 | 0 | 0 | 0 | 0 | 0 | 0 | 0 | | | | | | |
| 4824 | 05733 | 04823 | 1 | | 0 | 0 | 0 | 0 | 0 | 0 | 0 | 0 | 0 | 0 | 0 | 0 | 0 | 0 | 0 | 0 | 1 | 0 | 0 | 0 | 0 | 0 | 0 | 0 | 0 | | | | | | |
| 4825 | 05734 | 04824 | 1 | | 0 | 0 | 0 | 0 | 0 | 0 | 0 | 0 | 0 | 0 | 0 | 0 | 0 | 0 | 0 | 0 | 1 | 0 | 0 | 0 | 0 | 0 | 0 | 0 | 0 | | | | | | |
| 4826 | 05735 | 04825 | 1 | | 0 | 0 | 0 | 0 | 0 | 0 | 0 | 0 | 0 | 0 | 0 | 0 | 0 | 0 | 0 | 0 | 1 | 0 | 0 | 0 | 0 | 0 | 0 | 0 | 0 | | | | | | |
| 4827 | 05736 | 04826 | 1 | | 0 | 0 | 0 | 0 | 0 | 0 | 0 | 0 | 0 | 0 | 0 | 0 | 0 | 0 | 0 | 0 | 1 | 0 | 0 | 0 | 0 | 0 | 0 | 0 | 0 | | | | | | |
| 4828 | 05738 | 04827 | 17 | | 0 | 0 | 0 | 0 | 0 | 0 | 0 | 0 | 0 | 0 | 0 | 0 | 0 | 0 | 0 | 0 | 15 | 0 | 0 | 0 | 0 | 1 | 0 | 0 | 1 | | | | | | |
| 4829 | 05741 | 04828 | 3 | | 0 | 0 | 0 | 0 | 0 | 0 | 0 | 0 | 0 | 0 | 0 | 0 | 0 | 0 | 0 | 0 | 2 | 0 | 0 | 0 | 0 | 0 | 1 | 0 | 0 | | | | | | |
| 4830 | 05742 | 04829 | 7 | | 0 | 0 | 0 | 0 | 0 | 0 | 0 | 0 | 0 | 0 | 0 | 0 | 0 | 0 | 0 | 2 | 1 | 0 | 0 | 0 | 0 | 4 | 0 | 0 | 0 | | | | | | |
| 4831 | 05743 | 04830 | 1 | | 0 | 0 | 0 | 0 | 0 | 0 | 0 | 0 | 0 | 0 | 0 | 0 | 0 | 0 | 0 | 0 | 1 | 0 | 0 | 0 | 0 | 0 | 0 | 0 | 0 | | | | | | |
| 4832 | 05747 | 04831 | 1 | | 0 | 0 | 0 | 0 | 0 | 0 | 0 | 0 | 0 | 0 | 0 | 0 | 0 | 0 | 0 | 0 | 1 | 0 | 0 | 0 | 0 | 0 | 0 | 0 | 0 | | | | | | |
| 4833 | 05748 | 04832 | 1 | | 0 | 0 | 0 | 0 | 0 | 0 | 0 | 0 | 0 | 0 | 0 | 0 | 0 | 0 | 0 | 0 | 1 | 0 | 0 | 0 | 0 | 0 | 0 | 0 | 0 | M80244 | 95.4 | 324 | 1 | 3373 | 3984 |
| 4834 | 05749 | 04833 | 1 | | 0 | 0 | 0 | 0 | 0 | 0 | 0 | 0 | 0 | 0 | 0 | 0 | 0 | 0 | 0 | 0 | 1 | 0 | 0 | 0 | 0 | 0 | 0 | 0 | 0 | | | | | | |
| 4835 | 05751 | 04834 | 1 | | 0 | 0 | 0 | 0 | 0 | 0 | 0 | 0 | 0 | 0 | 0 | 0 | 0 | 0 | 0 | 0 | 1 | 0 | 0 | 0 | 0 | 0 | 0 | 0 | 0 | | | | | | |
| 4836 | 05752 | 04835 | 3 | | 0 | 0 | 0 | 0 | 0 | 0 | 0 | 0 | 0 | 0 | 0 | 0 | 0 | 0 | 0 | 1 | 2 | 0 | 0 | 0 | 0 | 0 | 0 | 0 | 0 | | | | | | |
| 4837 | 05754 | 04836 | 2 | | 0 | 0 | 0 | 0 | 0 | 0 | 0 | 0 | 0 | 0 | 0 | 0 | 0 | 0 | 0 | 1 | 1 | 0 | 0 | 0 | 0 | 0 | 0 | 0 | 0 | | | | | | |
| 4838 | 05755 | 04837 | 1 | | 0 | 0 | 0 | 0 | 0 | 0 | 0 | 0 | 0 | 0 | 0 | 0 | 0 | 0 | 0 | 0 | 1 | 0 | 0 | 0 | 0 | 0 | 0 | 0 | 0 | | | | | | |
| 4839 | 05756 | 04838 | 1 | | 0 | 0 | 0 | 0 | 0 | 0 | 0 | 0 | 0 | 0 | 0 | 0 | 0 | 0 | 0 | 0 | 1 | 0 | 0 | 0 | 0 | 0 | 0 | 0 | 0 | | | | | | |
| 4840 | 05757 | 04839 | 1 | | 0 | 0 | 0 | 0 | 0 | 0 | 0 | 0 | 0 | 0 | 0 | 0 | 0 | 0 | 0 | 0 | 1 | 0 | 0 | 0 | 0 | 0 | 0 | 0 | 0 | | | | | | |
| 4841 | 05758 | 04840 | 1 | | 0 | 0 | 0 | 0 | 0 | 0 | 0 | 0 | 0 | 0 | 0 | 0 | 0 | 0 | 0 | 0 | 1 | 0 | 0 | 0 | 0 | 0 | 0 | 0 | 0 | | | | | | |
| 4842 | 05759 | 04841 | 1 | | 0 | 0 | 0 | 0 | 0 | 0 | 0 | 0 | 0 | 0 | 0 | 0 | 0 | 0 | 0 | 0 | 1 | 0 | 0 | 0 | 0 | 0 | 0 | 0 | 0 | | | | | | |
| 4843 | 05763 | 04842 | 2 | | 0 | 0 | 0 | 0 | 0 | 0 | 0 | 0 | 0 | 0 | 0 | 0 | 0 | 0 | 0 | 0 | 1 | 0 | 0 | 0 | 0 | 0 | 0 | 1 | 0 | | | | | | |
| 4844 | 05765 | 04843 | 1 | | 0 | 0 | 0 | 0 | 0 | 0 | 0 | 0 | 0 | 0 | 0 | 0 | 0 | 0 | 0 | 0 | 1 | 0 | 0 | 0 | 0 | 0 | 0 | 0 | 0 | | | | | | |
| 4845 | 05767 | 04844 | 2 | | 0 | 0 | 0 | 0 | 0 | 0 | 0 | 0 | 0 | 0 | 0 | 0 | 0 | 0 | 0 | 0 | 1 | 0 | 0 | 0 | 0 | 0 | 1 | 0 | 0 | | | | | | |

Table 135

| | A | B | C | E | G | I | K | M | O | Q | S | U | W | Y | AA | AC | AF | AG | AI | AK | AM | AO | AQ | AS | AU | AW | AY | BA | BC | BE | BF | BG | BH | BI | BK |
|---|---|---|---|---|---|---|---|---|---|---|---|---|---|---|---|---|---|---|---|---|---|---|---|---|---|---|---|---|---|---|---|---|---|---|---|
| 4846 | 05768 | 04845 | 1 | | 0 | 0 | 0 | 0 | 0 | 0 | 0 | 0 | 0 | 0 | 0 | 0 | 0 | 0 | 0 | 0 | 1 | 0 | 0 | 0 | 0 | 0 | 0 | 0 | 0 | X62571 | 97.3 | 73 | 130 | 1430 | 1512 |
| 4847 | 05769 | 04846 | 1 | | 0 | 0 | 0 | 0 | 0 | 0 | 0 | 0 | 0 | 0 | 0 | 0 | 0 | 0 | 0 | 0 | 1 | 0 | 0 | 0 | 0 | 0 | 0 | 0 | 0 | | | | | | |
| 4848 | 05770 | 04847 | 1 | | 0 | 0 | 0 | 0 | 0 | 0 | 0 | 0 | 0 | 0 | 0 | 0 | 0 | 0 | 0 | 0 | 1 | 0 | 0 | 0 | 0 | 0 | 0 | 0 | 0 | | | | | | |
| 4849 | 05772 | 04848 | 1 | | 0 | 0 | 0 | 0 | 0 | 0 | 0 | 0 | 0 | 0 | 0 | 0 | 0 | 0 | 0 | 0 | 1 | 0 | 0 | 0 | 0 | 0 | 0 | 0 | 0 | | | | | | |
| 4850 | 05773 | 04849 | 1 | | 0 | 0 | 0 | 0 | 0 | 0 | 0 | 0 | 0 | 0 | 0 | 0 | 0 | 0 | 0 | 0 | 1 | 0 | 0 | 0 | 0 | 0 | 0 | 0 | 0 | | | | | | |
| 4851 | 05774 | 04850 | 1 | | 0 | 0 | 0 | 0 | 0 | 0 | 0 | 0 | 0 | 0 | 0 | 0 | 0 | 0 | 0 | 0 | 1 | 0 | 0 | 0 | 0 | 0 | 0 | 0 | 0 | | | | | | |
| 4852 | 05775 | 04851 | 1 | | 0 | 0 | 0 | 0 | 0 | 0 | 0 | 0 | 0 | 0 | 0 | 0 | 0 | 0 | 0 | 0 | 1 | 0 | 0 | 0 | 0 | 0 | 0 | 0 | 0 | D13643 | 95 | 343 | 1 | 3150 | 4186 |
| 4853 | 05776 | 04852 | 1 | | 0 | 0 | 0 | 0 | 0 | 0 | 0 | 0 | 0 | 0 | 0 | 0 | 0 | 0 | 0 | 0 | 1 | 0 | 0 | 0 | 0 | 0 | 0 | 0 | 0 | | | | | | |
| 4854 | 05777 | 04853 | 2 | | 0 | 0 | 0 | 0 | 0 | 0 | 0 | 0 | 0 | 0 | 0 | 0 | 0 | 0 | 0 | 0 | 2 | 0 | 0 | 0 | 0 | 0 | 0 | 0 | 0 | | | | | | |
| 4855 | 05778 | 04854 | 1 | | 0 | 0 | 0 | 0 | 0 | 0 | 0 | 0 | 0 | 0 | 0 | 0 | 0 | 0 | 0 | 0 | 1 | 0 | 0 | 0 | 0 | 0 | 0 | 0 | 0 | | | | | | |
| 4856 | 05779 | 04855 | 1 | | 0 | 0 | 0 | 0 | 0 | 0 | 0 | 0 | 0 | 0 | 0 | 0 | 0 | 0 | 0 | 0 | 1 | 0 | 0 | 0 | 0 | 0 | 0 | 0 | 0 | | | | | | |
| 4857 | 05780 | 04856 | 7 | | 0 | 0 | 0 | 0 | 0 | 0 | 0 | 0 | 0 | 0 | 0 | 0 | 0 | 0 | 0 | 4 | 3 | 0 | 0 | 0 | 0 | 0 | 0 | 0 | 0 | J00269 | 90.5 | 359 | 1 | 1268 | 1617 |
| 4858 | 05781 | 04857 | 2 | | 0 | 0 | 0 | 0 | 0 | 0 | 0 | 0 | 0 | 0 | 0 | 0 | 0 | 0 | 0 | 0 | 1 | 0 | 1 | 0 | 0 | 0 | 0 | 0 | 0 | | | | | | |
| 4859 | 05782 | 04858 | 2 | | 0 | 0 | 0 | 0 | 0 | 0 | 0 | 0 | 0 | 0 | 0 | 0 | 0 | 0 | 0 | 0 | 1 | 0 | 0 | 0 | 0 | 0 | 0 | 0 | 1 | | | | | | |
| 4860 | 05783 | 04859 | 7 | | 0 | 0 | 0 | 0 | 0 | 0 | 0 | 0 | 0 | 0 | 0 | 0 | 0 | 0 | 0 | 0 | 1 | 2 | 1 | 1 | 0 | 0 | 0 | 2 | 0 | | | | | | |
| 4861 | 05784 | 04860 | 1 | | 0 | 0 | 0 | 0 | 0 | 0 | 0 | 0 | 0 | 0 | 0 | 0 | 0 | 0 | 0 | 0 | 1 | 0 | 0 | 0 | 0 | 0 | 0 | 0 | 0 | | | | | | |
| 4862 | 05785 | 04861 | 1 | | 0 | 0 | 0 | 0 | 0 | 0 | 0 | 0 | 0 | 0 | 0 | 0 | 0 | 0 | 0 | 0 | 1 | 0 | 0 | 0 | 0 | 0 | 0 | 0 | 0 | | | | | | |
| 4863 | 05787 | 04862 | 1 | | 0 | 0 | 0 | 0 | 0 | 0 | 0 | 0 | 0 | 0 | 0 | 0 | 0 | 0 | 0 | 0 | 1 | 0 | 0 | 0 | 0 | 0 | 0 | 0 | 0 | | | | | | |
| 4864 | 05789 | 04863 | 1 | | 0 | 0 | 0 | 0 | 0 | 0 | 0 | 0 | 0 | 0 | 0 | 0 | 0 | 0 | 0 | 0 | 1 | 0 | 0 | 0 | 0 | 0 | 0 | 0 | 0 | | | | | | |
| 4865 | 05790 | 04864 | 1 | | 0 | 0 | 0 | 0 | 0 | 0 | 0 | 0 | 0 | 0 | 0 | 0 | 0 | 0 | 0 | 0 | 1 | 0 | 0 | 0 | 0 | 0 | 0 | 0 | 0 | | | | | | |
| 4866 | 05792 | 04865 | 1 | | 0 | 0 | 0 | 0 | 0 | 0 | 0 | 0 | 0 | 0 | 0 | 0 | 0 | 0 | 0 | 0 | 1 | 0 | 0 | 0 | 0 | 0 | 0 | 0 | 0 | | | | | | |
| 4867 | 05793 | 04866 | 9 | | 0 | 0 | 0 | 0 | 0 | 0 | 0 | 0 | 0 | 0 | 0 | 0 | 0 | 0 | 0 | 1 | 3 | 0 | 0 | 0 | 0 | 2 | 0 | 0 | 3 | M87068 | 98.5 | 67 | 1 | 361 | 427 |
| 4868 | 05794 | 04867 | 1 | | 0 | 0 | 0 | 0 | 0 | 0 | 0 | 0 | 0 | 0 | 0 | 0 | 0 | 0 | 0 | 0 | 1 | 0 | 0 | 0 | 0 | 0 | 0 | 0 | 0 | | | | | | |
| 4869 | 05795 | 04868 | 3 | | 0 | 0 | 0 | 0 | 0 | 0 | 0 | 0 | 0 | 0 | 0 | 0 | 0 | 0 | 0 | 0 | 1 | 1 | 0 | 1 | 0 | 0 | 0 | 0 | 0 | | | | | | |
| 4870 | 05796 | 04869 | 1 | | 0 | 0 | 0 | 0 | 0 | 0 | 0 | 0 | 0 | 0 | 0 | 0 | 0 | 0 | 0 | 0 | 1 | 0 | 0 | 0 | 0 | 0 | 0 | 0 | 0 | | | | | | |
| 4871 | 05797 | 04870 | 1 | | 0 | 0 | 0 | 0 | 0 | 0 | 0 | 0 | 0 | 0 | 0 | 0 | 0 | 0 | 0 | 0 | 1 | 0 | 0 | 0 | 0 | 0 | 0 | 0 | 0 | L12350 | 99.5 | 204 | 1 | 5181 | 5784 |
| 4872 | 05798 | 04871 | 3 | | 0 | 0 | 0 | 0 | 0 | 0 | 0 | 0 | 0 | 0 | 0 | 0 | 0 | 0 | 0 | 0 | 1 | 0 | 2 | 0 | 0 | 0 | 0 | 0 | 0 | | | | | | |
| 4873 | 05799 | 04872 | 2 | | 0 | 0 | 0 | 0 | 0 | 0 | 0 | 0 | 0 | 0 | 0 | 0 | 0 | 0 | 0 | 0 | 2 | 0 | 0 | 0 | 0 | 0 | 0 | 0 | 0 | X02851 | 100 | 84 | 1 | 1936 | 2027 |
| 4874 | 05801 | 04873 | 2 | | 0 | 0 | 0 | 0 | 0 | 0 | 0 | 0 | 0 | 0 | 0 | 0 | 0 | 0 | 0 | 0 | 1 | 0 | 0 | 0 | 0 | 1 | 0 | 0 | 0 | | | | | | |
| 4875 | 05804 | 04874 | 2 | | 0 | 0 | 0 | 0 | 0 | 0 | 0 | 0 | 0 | 0 | 0 | 0 | 0 | 0 | 0 | 0 | 2 | 0 | 0 | 0 | 0 | 0 | 0 | 0 | 0 | X05803 | 90.7 | 323 | 1 | 525 | 956 |
| 4876 | 05807 | 04875 | 1 | | 0 | 0 | 0 | 0 | 0 | 0 | 0 | 0 | 0 | 0 | 0 | 0 | 0 | 0 | 0 | 0 | 1 | 0 | 0 | 0 | 0 | 0 | 0 | 0 | 0 | | | | | | |
| 4877 | 05808 | 04876 | 6 | | 0 | 0 | 0 | 0 | 0 | 0 | 0 | 0 | 0 | 0 | 0 | 0 | 0 | 0 | 0 | 0 | 3 | 0 | 1 | 2 | 0 | 0 | 0 | 0 | 0 | | | | | | |
| 4878 | 05810 | 04877 | 1 | | 0 | 0 | 0 | 0 | 0 | 0 | 0 | 0 | 0 | 0 | 0 | 0 | 0 | 0 | 0 | 0 | 1 | 0 | 0 | 0 | 0 | 0 | 0 | 0 | 0 | | | | | | |
| 4879 | 05813 | 04878 | 1 | | 0 | 0 | 0 | 0 | 0 | 0 | 0 | 0 | 0 | 0 | 0 | 0 | 0 | 0 | 0 | 0 | 1 | 0 | 0 | 0 | 0 | 0 | 0 | 0 | 0 | | | | | | |
| 4880 | 05814 | 04879 | 2 | | 0 | 0 | 0 | 0 | 0 | 0 | 0 | 0 | 0 | 0 | 0 | 0 | 0 | 0 | 0 | 0 | 1 | 1 | 0 | 0 | 0 | 0 | 0 | 0 | 0 | | | | | | |
| 4881 | 05815 | 04880 | 1 | | 0 | 0 | 0 | 0 | 0 | 0 | 0 | 0 | 0 | 0 | 0 | 0 | 0 | 0 | 0 | 0 | 1 | 0 | 0 | 0 | 0 | 0 | 0 | 0 | 0 | | | | | | |

Table 136

| | A | B | C | E | G | I | K | M | O | Q | S | U | W | Y | AA | AC | AE | AG | AI | AK | AM | AO | AQ | AS | AU | AW | AY | BA | BC | BE | BF | BG | BH | BI | BK |
|---|---|---|---|---|---|---|---|---|---|---|---|---|---|---|---|---|---|---|---|---|---|---|---|---|---|---|---|---|---|---|---|---|---|---|---|
| 4882 | 05816 | 04881 | 1 | | 0 | 0 | 0 | 0 | 0 | 0 | 0 | 0 | 0 | 0 | 0 | 0 | 0 | 0 | 0 | 0 | 1 | 0 | 0 | 0 | 0 | 0 | 0 | 0 | 0 | | | | | | |
| 4883 | 05817 | 04882 | 1 | | 0 | 0 | 0 | 0 | 0 | 0 | 0 | 0 | 0 | 0 | 0 | 0 | 0 | 0 | 0 | 0 | 1 | 0 | 0 | 0 | 0 | 0 | 0 | 0 | 0 | | | | | | |
| 4884 | 05818 | 04883 | 1 | | 0 | 0 | 0 | 0 | 0 | 0 | 0 | 0 | 0 | 0 | 0 | 0 | 0 | 0 | 0 | 0 | 1 | 0 | 0 | 0 | 0 | 0 | 0 | 0 | 0 | | | | | | |
| 4885 | 05819 | 04884 | 1 | | 0 | 0 | 0 | 0 | 0 | 0 | 0 | 0 | 0 | 0 | 0 | 0 | 0 | 0 | 0 | 0 | 1 | 0 | 0 | 0 | 0 | 0 | 0 | 0 | 0 | | | | | | |
| 4886 | 05820 | 04885 | 1 | | 0 | 0 | 0 | 0 | 0 | 0 | 0 | 0 | 0 | 0 | 0 | 0 | 0 | 0 | 0 | 0 | 1 | 0 | 0 | 0 | 0 | 0 | 0 | 0 | 0 | | | | | | |
| 4887 | 05821 | 04886 | 1 | | 0 | 0 | 0 | 0 | 0 | 0 | 0 | 0 | 0 | 0 | 0 | 0 | 0 | 0 | 0 | 0 | 1 | 0 | 0 | 0 | 0 | 0 | 0 | 0 | 0 | | | | | | |
| 4888 | 05823 | 04887 | 1 | | 0 | 0 | 0 | 0 | 0 | 0 | 0 | 0 | 0 | 0 | 0 | 0 | 0 | 0 | 0 | 0 | 1 | 0 | 0 | 0 | 0 | 0 | 0 | 0 | 0 | | | | | | |
| 4889 | 05824 | 04888 | 1 | | 0 | 0 | 0 | 0 | 0 | 0 | 0 | 0 | 0 | 0 | 0 | 0 | 0 | 0 | 0 | 0 | 1 | 0 | 0 | 0 | 0 | 0 | 0 | 0 | 0 | | | | | | |
| 4890 | 05825 | 04889 | 1 | | 0 | 0 | 0 | 0 | 0 | 0 | 0 | 0 | 0 | 0 | 0 | 0 | 0 | 0 | 0 | 0 | 1 | 0 | 0 | 0 | 0 | 0 | 0 | 0 | 0 | X15183 | 97.8 | 321 | 1 | 2133 | 2912 |
| 4891 | 05826 | 04890 | 1 | | 0 | 0 | 0 | 0 | 0 | 0 | 0 | 0 | 0 | 0 | 0 | 0 | 0 | 0 | 0 | 0 | 1 | 0 | 0 | 0 | 0 | 0 | 0 | 0 | 0 | | | | | | |
| 4892 | 05827 | 04891 | 1 | | 0 | 0 | 0 | 0 | 0 | 0 | 0 | 0 | 0 | 0 | 0 | 0 | 0 | 0 | 0 | 0 | 1 | 0 | 0 | 0 | 0 | 0 | 0 | 0 | 0 | | | | | | |
| 4893 | 05829 | 04892 | 2 | | 0 | 0 | 0 | 0 | 0 | 0 | 0 | 0 | 0 | 0 | 0 | 0 | 0 | 0 | 0 | 0 | 2 | 0 | 0 | 0 | 0 | 0 | 0 | 0 | 0 | M19723 | 93.6 | 343 | 1 | 1152 | 1869 |
| 4894 | 05830 | 04893 | 1 | | 0 | 0 | 0 | 0 | 0 | 0 | 0 | 0 | 0 | 0 | 0 | 0 | 0 | 0 | 0 | 0 | 1 | 0 | 0 | 0 | 0 | 0 | 0 | 0 | 0 | | | | | | |
| 4895 | 05831 | 04894 | 1 | | 0 | 0 | 0 | 0 | 0 | 0 | 0 | 0 | 0 | 0 | 0 | 0 | 0 | 0 | 0 | 0 | 1 | 0 | 0 | 0 | 0 | 0 | 0 | 0 | 0 | | | | | | |
| 4896 | 05832 | 04895 | 1 | | 0 | 0 | 0 | 0 | 0 | 0 | 0 | 0 | 0 | 0 | 0 | 0 | 0 | 0 | 0 | 0 | 1 | 0 | 0 | 0 | 0 | 0 | 0 | 0 | 0 | | | | | | |
| 4897 | 05833 | 04896 | 2 | | 0 | 0 | 0 | 0 | 0 | 0 | 0 | 0 | 0 | 0 | 0 | 0 | 0 | 0 | 0 | 0 | 1 | 0 | 0 | 1 | 0 | 0 | 0 | 0 | 0 | | | | | | |
| 4898 | 05834 | 04897 | 2 | | 0 | 0 | 0 | 0 | 0 | 0 | 0 | 0 | 0 | 0 | 0 | 0 | 0 | 0 | 0 | 1 | 1 | 0 | 0 | 0 | 0 | 0 | 0 | 0 | 0 | | | | | | |
| 4899 | 05835 | 04898 | 2 | | 0 | 0 | 0 | 0 | 0 | 0 | 0 | 0 | 0 | 0 | 0 | 0 | 0 | 0 | 0 | 0 | 1 | 0 | 1 | 0 | 0 | 0 | 0 | 0 | 0 | X54232 | 96.5 | 313 | 1 | 3380 | 3692 |
| 4900 | 05836 | 04899 | 2 | | 0 | 0 | 0 | 0 | 0 | 0 | 0 | 0 | 0 | 0 | 0 | 0 | 0 | 0 | 0 | 0 | 1 | 1 | 0 | 0 | 0 | 0 | 0 | 0 | 0 | | | | | | |
| 4901 | 05837 | 04900 | 2 | | 0 | 0 | 0 | 0 | 0 | 0 | 0 | 0 | 0 | 0 | 0 | 0 | 0 | 0 | 0 | 0 | 1 | 1 | 0 | 0 | 0 | 0 | 0 | 0 | 0 | | | | | | |
| 4902 | 05838 | 04901 | 1 | | 0 | 0 | 0 | 0 | 0 | 0 | 0 | 0 | 0 | 0 | 0 | 0 | 0 | 0 | 0 | 0 | 1 | 0 | 0 | 0 | 0 | 0 | 0 | 0 | 0 | | | | | | |
| 4903 | 05839 | 04902 | 1 | | 0 | 0 | 0 | 0 | 0 | 0 | 0 | 0 | 0 | 0 | 0 | 0 | 0 | 0 | 0 | 0 | 1 | 0 | 0 | 0 | 0 | 0 | 0 | 0 | 0 | | | | | | |
| 4904 | 05840 | 04903 | 3 | | 0 | 0 | 0 | 0 | 0 | 0 | 0 | 0 | 0 | 0 | 0 | 0 | 0 | 1 | 0 | 0 | 1 | 0 | 0 | 0 | 0 | 0 | 0 | 1 | 0 | | | | | | |
| 4905 | 05841 | 04904 | 1 | | 0 | 0 | 0 | 0 | 0 | 0 | 0 | 0 | 0 | 0 | 0 | 0 | 0 | 0 | 0 | 0 | 1 | 0 | 0 | 0 | 0 | 0 | 0 | 0 | 0 | D26067 | 90.7 | 54 | 1 | 1718 | 3269 |
| 4906 | 05842 | 04905 | 2 | | 0 | 0 | 0 | 0 | 0 | 0 | 0 | 0 | 0 | 0 | 0 | 0 | 0 | 0 | 0 | 0 | 1 | 0 | 1 | 0 | 0 | 0 | 0 | 0 | 0 | | | | | | |
| 4907 | 05843 | 04906 | 1 | | 0 | 0 | 0 | 0 | 0 | 0 | 0 | 0 | 0 | 0 | 0 | 0 | 0 | 0 | 0 | 0 | 1 | 0 | 0 | 0 | 0 | 0 | 0 | 0 | 0 | | | | | | |
| 4908 | 05844 | 04907 | 1 | | 0 | 0 | 0 | 0 | 0 | 0 | 0 | 0 | 0 | 0 | 0 | 0 | 0 | 0 | 0 | 0 | 1 | 0 | 0 | 0 | 0 | 0 | 0 | 0 | 0 | | | | | | |
| 4909 | 05845 | 04908 | 1 | | 0 | 0 | 0 | 0 | 0 | 0 | 0 | 0 | 0 | 0 | 0 | 0 | 0 | 0 | 0 | 0 | 1 | 0 | 0 | 0 | 0 | 0 | 0 | 0 | 0 | | | | | | |
| 4910 | 05846 | 04909 | 7 | | 0 | 0 | 0 | 0 | 0 | 0 | 0 | 0 | 0 | 0 | 0 | 0 | 0 | 0 | 0 | 5 | 1 | 0 | 0 | 0 | 0 | 0 | 0 | 1 | 0 | X74801 | 100 | 135 | 1 | 941 | 1108 |
| 4911 | 05847 | 04910 | 1 | | 0 | 0 | 0 | 0 | 0 | 0 | 0 | 0 | 0 | 0 | 0 | 0 | 0 | 0 | 0 | 0 | 1 | 0 | 0 | 0 | 0 | 0 | 0 | 0 | 0 | L10844 | 92.8 | 223 | 3 | 125 | 1855 |
| 4912 | 05848 | 04911 | 1 | | 0 | 0 | 0 | 0 | 0 | 0 | 0 | 0 | 0 | 0 | 0 | 0 | 0 | 0 | 0 | 0 | 1 | 0 | 0 | 0 | 0 | 0 | 0 | 0 | 0 | | | | | | |
| 4913 | 05849 | 04912 | 1 | | 0 | 0 | 0 | 0 | 0 | 0 | 0 | 0 | 0 | 0 | 0 | 0 | 0 | 0 | 0 | 0 | 1 | 0 | 0 | 0 | 0 | 0 | 0 | 0 | 0 | | | | | | |
| 4914 | 05850 | 04913 | 1 | | 0 | 0 | 0 | 0 | 0 | 0 | 0 | 0 | 0 | 0 | 0 | 0 | 0 | 0 | 0 | 0 | 1 | 0 | 0 | 0 | 0 | 0 | 0 | 0 | 0 | | | | | | |
| 4915 | 05851 | 04914 | 1 | | 0 | 0 | 0 | 0 | 0 | 0 | 0 | 0 | 0 | 0 | 0 | 0 | 0 | 0 | 0 | 0 | 1 | 0 | 0 | 0 | 0 | 0 | 0 | 0 | 0 | M87770 | 100 | 124 | 1 | 4132 | 4268 |
| 4916 | 05852 | 04915 | 1 | | 0 | 0 | 0 | 0 | 0 | 0 | 0 | 0 | 0 | 0 | 0 | 0 | 0 | 0 | 0 | 0 | 1 | 0 | 0 | 0 | 0 | 0 | 0 | 0 | 0 | | | | | | |
| 4917 | 05854 | 04916 | 1 | | 0 | 0 | 0 | 0 | 0 | 0 | 0 | 0 | 0 | 0 | 0 | 0 | 0 | 0 | 0 | 0 | 1 | 0 | 0 | 0 | 0 | 0 | 0 | 0 | 0 | | | | | | |

Table 137

| | A | B | C | E | G | I | K | M | O | Q | S | U | W | Y | AA | AC | AE | AG | AI | AK | AM | AO | AQ | AS | AU | AW | AY | BA | BC | BE | BF | BG | BH | BI | BK |
|---|---|---|---|---|---|---|---|---|---|---|---|---|---|---|---|---|---|---|---|---|---|---|---|---|---|---|---|---|---|---|---|---|---|---|---|
| 4918 | 05856 | 04917 | 4 | | 0 | 0 | 0 | 0 | 0 | 0 | 0 | 0 | 0 | 0 | 3 | 0 | 0 | 0 | 0 | 0 | 1 | 0 | 0 | 0 | 0 | 0 | 0 | 0 | 0 | | | | | | |
| 4919 | 05857 | 04918 | 4 | | 0 | 0 | 0 | 0 | 0 | 0 | 0 | 0 | 0 | 0 | 0 | 0 | 0 | 0 | 0 | 0 | 1 | 1 | 0 | 0 | 0 | 1 | 0 | 1 | 0 | | | | | | |
| 4920 | 05858 | 04919 | 1 | | 0 | 0 | 0 | 0 | 0 | 0 | 0 | 0 | 0 | 0 | 0 | 0 | 0 | 0 | 0 | 0 | 1 | 0 | 0 | 0 | 0 | 0 | 0 | 0 | 0 | M86667 | 100 | 168 | 1 | 376 | 1560 |
| 4921 | 05859 | 04920 | 2 | | 0 | 0 | 0 | 0 | 0 | 0 | 0 | 0 | 0 | 0 | 0 | 0 | 0 | 0 | 0 | 0 | 1 | 0 | 0 | 0 | 0 | 1 | 0 | 0 | 0 | M18391 | 93.2 | 118 | 1 | 3253 | 3370 |
| 4922 | 05860 | 04921 | 1 | | 0 | 0 | 0 | 0 | 0 | 0 | 0 | 0 | 0 | 0 | 0 | 0 | 0 | 0 | 0 | 0 | 1 | 0 | 0 | 0 | 0 | 0 | 0 | 0 | 0 | | | | | | |
| 4923 | 05862 | 04922 | 1 | | 0 | 0 | 0 | 0 | 0 | 0 | 0 | 0 | 0 | 0 | 0 | 0 | 0 | 0 | 0 | 0 | 1 | 0 | 0 | 0 | 0 | 0 | 0 | 0 | 0 | | | | | | |
| 4924 | 05863 | 04923 | 3 | | 0 | 0 | 0 | 0 | 0 | 0 | 0 | 0 | 0 | 0 | 1 | 0 | 0 | 0 | 0 | 1 | 1 | 0 | 0 | 0 | 0 | 0 | 0 | 0 | 0 | | | | | | |
| 4925 | 05864 | 04924 | 10 | | 0 | 0 | 0 | 0 | 0 | 0 | 0 | 0 | 0 | 0 | 0 | 0 | 0 | 0 | 0 | 2 | 2 | 5 | 0 | 0 | 0 | 1 | 0 | 0 | 0 | M26252 | 93 | 327 | 1 | 1817 | 2306 |
| 4926 | 05865 | 04925 | 2 | | 0 | 0 | 0 | 0 | 0 | 0 | 0 | 0 | 0 | 0 | 0 | 0 | 0 | 0 | 0 | 0 | 1 | 0 | 1 | 0 | 0 | 0 | 0 | 0 | 0 | | | | | | |
| 4927 | 05866 | 04926 | 1 | | 0 | 0 | 0 | 0 | 0 | 0 | 0 | 0 | 0 | 0 | 0 | 0 | 0 | 0 | 0 | 0 | 1 | 0 | 0 | 0 | 0 | 0 | 0 | 0 | 0 | | | | | | |
| 4928 | 05867 | 04927 | 2 | | 0 | 0 | 0 | 0 | 0 | 0 | 0 | 0 | 0 | 0 | 0 | 0 | 0 | 0 | 0 | 0 | 1 | 0 | 0 | 0 | 0 | 0 | 1 | 0 | 0 | | | | | | |
| 4929 | 05868 | 04928 | 1 | | 0 | 0 | 0 | 0 | 0 | 0 | 0 | 0 | 0 | 0 | 0 | 0 | 0 | 0 | 0 | 0 | 1 | 0 | 0 | 0 | 0 | 0 | 0 | 0 | 0 | | | | | | |
| 4930 | 05869 | 04929 | 2 | | 0 | 0 | 0 | 0 | 0 | 0 | 0 | 0 | 0 | 0 | 0 | 0 | 0 | 0 | 0 | 0 | 1 | 0 | 0 | 0 | 0 | 0 | 0 | 0 | 1 | | | | | | |
| 4931 | 05870 | 04930 | 1 | | 0 | 0 | 0 | 0 | 0 | 0 | 0 | 0 | 0 | 0 | 0 | 0 | 0 | 0 | 0 | 0 | 1 | 0 | 0 | 0 | 0 | 0 | 0 | 0 | 0 | | | | | | |
| 4932 | 05871 | 04931 | 1 | | 0 | 0 | 0 | 0 | 0 | 0 | 0 | 0 | 0 | 0 | 0 | 0 | 0 | 0 | 0 | 0 | 1 | 0 | 0 | 0 | 0 | 0 | 0 | 0 | 0 | | | | | | |
| 4933 | 05872 | 04932 | 1 | | 0 | 0 | 0 | 0 | 0 | 0 | 0 | 0 | 0 | 0 | 0 | 0 | 0 | 0 | 0 | 0 | 1 | 0 | 0 | 0 | 0 | 0 | 0 | 0 | 0 | | | | | | |
| 4934 | 05874 | 04933 | 1 | | 0 | 0 | 0 | 0 | 0 | 0 | 0 | 0 | 0 | 0 | 0 | 0 | 0 | 0 | 0 | 0 | 1 | 0 | 0 | 0 | 0 | 0 | 0 | 0 | 0 | | | | | | |
| 4935 | 05875 | 04934 | 1 | | 0 | 0 | 0 | 0 | 0 | 0 | 0 | 0 | 0 | 0 | 0 | 0 | 0 | 0 | 0 | 0 | 1 | 0 | 0 | 0 | 0 | 0 | 0 | 0 | 0 | | | | | | |
| 4936 | 05876 | 04935 | 1 | | 0 | 0 | 0 | 0 | 0 | 0 | 0 | 0 | 0 | 0 | 0 | 0 | 0 | 0 | 0 | 0 | 1 | 0 | 0 | 0 | 0 | 0 | 0 | 0 | 0 | | | | | | |
| 4937 | 05877 | 04936 | 1 | | 0 | 0 | 0 | 0 | 0 | 0 | 0 | 0 | 0 | 0 | 0 | 0 | 0 | 0 | 0 | 0 | 1 | 0 | 0 | 0 | 0 | 0 | 0 | 0 | 0 | | | | | | |
| 4938 | 05879 | 04937 | 2 | | 0 | 0 | 0 | 0 | 0 | 0 | 0 | 0 | 0 | 0 | 0 | 0 | 0 | 0 | 0 | 0 | 2 | 0 | 0 | 0 | 0 | 0 | 0 | 0 | 0 | | | | | | |
| 4939 | 05880 | 04938 | 1 | | 0 | 0 | 0 | 0 | 0 | 0 | 0 | 0 | 0 | 0 | 0 | 0 | 0 | 0 | 0 | 0 | 1 | 0 | 0 | 0 | 0 | 0 | 0 | 0 | 0 | | | | | | |
| 4940 | 05882 | 04939 | 1 | | 0 | 0 | 0 | 0 | 0 | 0 | 0 | 0 | 0 | 0 | 0 | 0 | 0 | 0 | 0 | 0 | 1 | 0 | 0 | 0 | 0 | 0 | 0 | 0 | 0 | | | | | | |
| 4941 | 05885 | 04940 | 1 | | 0 | 0 | 0 | 0 | 0 | 0 | 0 | 0 | 0 | 0 | 0 | 0 | 0 | 0 | 0 | 0 | 1 | 0 | 0 | 0 | 0 | 0 | 0 | 0 | 0 | | | | | | |
| 4942 | 05886 | 04941 | 1 | | 0 | 0 | 0 | 0 | 0 | 0 | 0 | 0 | 0 | 0 | 0 | 0 | 0 | 0 | 0 | 0 | 1 | 0 | 0 | 0 | 0 | 0 | 0 | 0 | 0 | | | | | | |
| 4943 | 05887 | 04942 | 1 | | 0 | 0 | 0 | 0 | 0 | 0 | 0 | 0 | 0 | 0 | 0 | 0 | 0 | 0 | 0 | 0 | 1 | 0 | 0 | 0 | 0 | 0 | 0 | 0 | 0 | | | | | | |
| 4944 | 05888 | 04943 | 2 | | 0 | 0 | 0 | 0 | 0 | 0 | 0 | 0 | 0 | 0 | 0 | 0 | 0 | 0 | 0 | 1 | 1 | 0 | 0 | 0 | 0 | 0 | 0 | 0 | 0 | | | | | | |
| 4945 | 05889 | 04944 | 1 | | 0 | 0 | 0 | 0 | 0 | 0 | 0 | 0 | 0 | 0 | 0 | 0 | 0 | 0 | 0 | 0 | 1 | 0 | 0 | 0 | 0 | 0 | 0 | 0 | 0 | | | | | | |
| 4946 | 05890 | 04945 | 1 | | 0 | 0 | 0 | 0 | 0 | 0 | 0 | 0 | 0 | 0 | 0 | 0 | 0 | 0 | 0 | 0 | 1 | 0 | 0 | 0 | 0 | 0 | 0 | 0 | 0 | | | | | | |
| 4947 | 05892 | 04946 | 1 | | 0 | 0 | 0 | 0 | 0 | 0 | 0 | 0 | 0 | 0 | 0 | 0 | 0 | 0 | 0 | 0 | 1 | 0 | 0 | 0 | 0 | 0 | 0 | 0 | 0 | | | | | | |
| 4948 | 05893 | 04947 | 1 | | 0 | 0 | 0 | 0 | 0 | 0 | 0 | 0 | 0 | 0 | 0 | 0 | 0 | 0 | 0 | 0 | 1 | 0 | 0 | 0 | 0 | 0 | 0 | 0 | 0 | | | | | | |
| 4949 | 05894 | 04948 | 2 | | 0 | 0 | 0 | 0 | 0 | 0 | 0 | 0 | 0 | 0 | 0 | 0 | 0 | 0 | 0 | 0 | 1 | 1 | 0 | 0 | 0 | 0 | 0 | 0 | 0 | M17783 | 98 | 101 | 1 | 1091 | 1191 |
| 4950 | 05895 | 04949 | 1 | | 0 | 0 | 0 | 0 | 0 | 0 | 0 | 0 | 0 | 0 | 0 | 0 | 0 | 0 | 0 | 0 | 1 | 0 | 0 | 0 | 0 | 0 | 0 | 0 | 0 | M21551 | 96.1 | 330 | 1 | 295 | 640 |
| 4951 | 05896 | 04950 | 1 | | 0 | 0 | 0 | 0 | 0 | 0 | 0 | 0 | 0 | 0 | 0 | 0 | 0 | 0 | 0 | 0 | 1 | 0 | 0 | 0 | 0 | 0 | 0 | 0 | 0 | | | | | | |
| 4952 | 05897 | 04951 | 2 | | 0 | 0 | 0 | 0 | 0 | 0 | 0 | 0 | 0 | 0 | 0 | 0 | 0 | 0 | 0 | 0 | 2 | 0 | 0 | 0 | 0 | 0 | 0 | 0 | 0 | | | | | | |
| 4953 | 05898 | 04952 | 1 | | 0 | 0 | 0 | 0 | 0 | 0 | 0 | 0 | 0 | 0 | 0 | 0 | 0 | 0 | 0 | 0 | 1 | 0 | 0 | 0 | 0 | 0 | 0 | 0 | 0 | | | | | | |

Table 138

| | A | B | C | E | G | I | K | M | O | Q | S | U | W | Y | AA | AC | AE | AG | AI | AK | AM | AO | AQ | AS | AU | AW | AY | BA | BC | BE | BF | BG | BH | BI | BK |
|---|---|---|---|---|---|---|---|---|---|---|---|---|---|---|---|---|---|---|---|---|---|---|---|---|---|---|---|---|---|---|---|---|---|---|---|
| 4954 | 05899 | 04953 | 1 | | 0 | 0 | 0 | 0 | 0 | 0 | 0 | 0 | 0 | 0 | 0 | 0 | 0 | 0 | 0 | 0 | 1 | 0 | 0 | 0 | 0 | 0 | 0 | 0 | 0 | | | | | | |
| 4955 | 05900 | 04954 | 2 | | 0 | 0 | 0 | 0 | 0 | 0 | 0 | 0 | 0 | 0 | 0 | 0 | 0 | 0 | 0 | 0 | 1 | 0 | 0 | 0 | 1 | 0 | 0 | 0 | 0 | | | | | | |
| 4956 | 05902 | 04955 | 1 | | 0 | 0 | 0 | 0 | 0 | 0 | 0 | 0 | 0 | 0 | 0 | 0 | 0 | 0 | 0 | 0 | 1 | 0 | 0 | 0 | 0 | 0 | 0 | 0 | 0 | | | | | | |
| 4957 | 05903 | 04956 | 1 | | 0 | 0 | 0 | 0 | 0 | 0 | 0 | 0 | 0 | 0 | 0 | 0 | 0 | 0 | 0 | 0 | 1 | 0 | 0 | 0 | 0 | 0 | 0 | 0 | 0 | M62994 | 95.2 | 315 | 1 | 1591 | 2007 |
| 4958 | 05907 | 04957 | 1 | | 0 | 0 | 0 | 0 | 0 | 0 | 0 | 0 | 0 | 0 | 0 | 0 | 0 | 0 | 0 | 0 | 1 | 0 | 0 | 0 | 0 | 0 | 0 | 0 | 0 | X05803 | 93.8 | 308 | 1 | 525 | 956 |
| 4959 | 05908 | 04958 | 1 | | 0 | 0 | 0 | 0 | 0 | 0 | 0 | 0 | 0 | 0 | 0 | 0 | 0 | 0 | 0 | 0 | 1 | 0 | 0 | 0 | 0 | 0 | 0 | 0 | 0 | | | | | | |
| 4960 | 05912 | 04959 | 2 | | 0 | 0 | 0 | 0 | 0 | 0 | 0 | 0 | 0 | 0 | 0 | 0 | 0 | 0 | 0 | 0 | 1 | 0 | 0 | 0 | 0 | 0 | 1 | 0 | 0 | | | | | | |
| 4961 | 05913 | 04960 | 1 | | 0 | 0 | 0 | 0 | 0 | 0 | 0 | 0 | 0 | 0 | 0 | 0 | 0 | 0 | 0 | 0 | 1 | 0 | 0 | 0 | 0 | 0 | 0 | 0 | 0 | | | | | | |
| 4962 | 05914 | 04961 | 1 | | 0 | 0 | 0 | 0 | 0 | 0 | 0 | 0 | 0 | 0 | 0 | 0 | 0 | 0 | 0 | 0 | 1 | 0 | 0 | 0 | 0 | 0 | 0 | 0 | 0 | | | | | | |
| 4963 | 05916 | 04962 | 1 | | 0 | 0 | 0 | 0 | 0 | 0 | 0 | 0 | 0 | 0 | 0 | 0 | 0 | 0 | 0 | 0 | 1 | 0 | 0 | 0 | 0 | 0 | 0 | 0 | 0 | | | | | | |
| 4964 | 05917 | 04963 | 1 | | 0 | 0 | 0 | 0 | 0 | 0 | 0 | 0 | 0 | 0 | 0 | 0 | 0 | 0 | 0 | 0 | 1 | 0 | 0 | 0 | 0 | 0 | 0 | 0 | 0 | | | | | | |
| 4965 | 05918 | 04964 | 1 | | 0 | 0 | 0 | 0 | 0 | 0 | 0 | 0 | 0 | 0 | 0 | 0 | 0 | 0 | 0 | 0 | 1 | 0 | 0 | 0 | 0 | 0 | 0 | 0 | 0 | | | | | | |
| 4966 | 05919 | 04965 | 1 | | 0 | 0 | 0 | 0 | 0 | 0 | 0 | 0 | 0 | 0 | 0 | 0 | 0 | 0 | 0 | 0 | 1 | 0 | 0 | 0 | 0 | 0 | 0 | 0 | 0 | | | | | | |
| 4967 | 05920 | 04966 | 1 | | 0 | 0 | 0 | 0 | 0 | 0 | 0 | 0 | 0 | 0 | 0 | 0 | 0 | 0 | 0 | 0 | 1 | 0 | 0 | 0 | 0 | 0 | 0 | 0 | 0 | | | | | | |
| 4968 | 05921 | 04967 | 1 | | 0 | 0 | 0 | 0 | 0 | 0 | 0 | 0 | 0 | 0 | 0 | 0 | 0 | 0 | 0 | 0 | 1 | 0 | 0 | 0 | 0 | 0 | 0 | 0 | 0 | X63629 | 99.2 | 126 | 1 | 3046 | 3171 |
| 4969 | 05923 | 04968 | 1 | | 0 | 0 | 0 | 0 | 0 | 0 | 0 | 0 | 0 | 0 | 0 | 0 | 0 | 0 | 0 | 0 | 1 | 0 | 0 | 0 | 0 | 0 | 0 | 0 | 0 | | | | | | |
| 4970 | 05925 | 04969 | 2 | | 0 | 0 | 0 | 0 | 0 | 0 | 0 | 0 | 0 | 0 | 1 | 0 | 0 | 0 | 0 | 0 | 1 | 0 | 0 | 0 | 0 | 0 | 0 | 0 | 0 | | | | | | |
| 4971 | 05926 | 04970 | 1 | | 0 | 0 | 0 | 0 | 0 | 0 | 0 | 0 | 0 | 0 | 0 | 0 | 0 | 0 | 0 | 0 | 1 | 0 | 0 | 0 | 0 | 0 | 0 | 0 | 0 | | | | | | |
| 4972 | 05927 | 04971 | 1 | | 0 | 0 | 0 | 0 | 0 | 0 | 0 | 0 | 0 | 0 | 0 | 0 | 0 | 0 | 0 | 0 | 1 | 0 | 0 | 0 | 0 | 0 | 0 | 0 | 0 | | | | | | |
| 4973 | 05928 | 04972 | 1 | | 0 | 0 | 0 | 0 | 0 | 0 | 0 | 0 | 0 | 0 | 0 | 0 | 0 | 0 | 0 | 0 | 1 | 0 | 0 | 0 | 0 | 0 | 0 | 0 | 0 | | | | | | |
| 4974 | 05932 | 04973 | 1 | | 0 | 0 | 0 | 0 | 0 | 0 | 0 | 0 | 0 | 0 | 0 | 0 | 0 | 0 | 0 | 0 | 1 | 0 | 0 | 0 | 0 | 0 | 0 | 0 | 0 | | | | | | |
| 4975 | 05933 | 04974 | 1 | | 0 | 0 | 0 | 0 | 0 | 0 | 0 | 0 | 0 | 0 | 0 | 0 | 0 | 0 | 0 | 0 | 1 | 0 | 0 | 0 | 0 | 0 | 0 | 0 | 0 | | | | | | |
| 4976 | 05934 | 04975 | 1 | | 0 | 0 | 0 | 0 | 0 | 0 | 0 | 0 | 0 | 0 | 0 | 0 | 0 | 0 | 0 | 0 | 1 | 0 | 0 | 0 | 0 | 0 | 0 | 0 | 0 | | | | | | |
| 4977 | 05937 | 04976 | 1 | | 0 | 0 | 0 | 0 | 0 | 0 | 0 | 0 | 0 | 0 | 0 | 0 | 0 | 0 | 0 | 0 | 1 | 0 | 0 | 0 | 0 | 0 | 0 | 0 | 0 | | | | | | |
| 4978 | 05938 | 04977 | 1 | | 0 | 0 | 0 | 0 | 0 | 0 | 0 | 0 | 0 | 0 | 0 | 0 | 0 | 0 | 0 | 0 | 1 | 0 | 0 | 0 | 0 | 0 | 0 | 0 | 0 | | | | | | |
| 4979 | 05939 | 04978 | 1 | | 0 | 0 | 0 | 0 | 0 | 0 | 0 | 0 | 0 | 0 | 0 | 0 | 0 | 0 | 0 | 0 | 1 | 0 | 0 | 0 | 0 | 0 | 0 | 0 | 0 | | | | | | |
| 4980 | 05940 | 04979 | 1 | | 0 | 0 | 0 | 0 | 0 | 0 | 0 | 0 | 0 | 0 | 0 | 0 | 0 | 0 | 0 | 0 | 1 | 0 | 0 | 0 | 0 | 0 | 0 | 0 | 0 | | | | | | |
| 4981 | 05941 | 04980 | 1 | | 0 | 0 | 0 | 0 | 0 | 0 | 0 | 0 | 0 | 0 | 0 | 0 | 0 | 0 | 0 | 0 | 1 | 0 | 0 | 0 | 0 | 0 | 0 | 0 | 0 | | | | | | |
| 4982 | 05942 | 04981 | 1 | | 0 | 0 | 0 | 0 | 0 | 0 | 0 | 0 | 0 | 0 | 0 | 0 | 0 | 0 | 0 | 0 | 1 | 0 | 0 | 0 | 0 | 0 | 0 | 0 | 0 | | | | | | |
| 4983 | 05943 | 04982 | 2 | | 0 | 0 | 0 | 0 | 0 | 0 | 0 | 0 | 0 | 0 | 0 | 0 | 0 | 0 | 0 | 0 | 1 | 0 | 0 | 0 | 0 | 1 | 0 | 0 | 0 | | | | | | |
| 4984 | 05946 | 04983 | 2 | | 0 | 0 | 0 | 0 | 0 | 0 | 0 | 0 | 0 | 0 | 0 | 0 | 0 | 0 | 0 | 0 | 2 | 0 | 0 | 0 | 0 | 0 | 0 | 0 | 0 | L19183 | 90.2 | 51 | 1 | 983 | 2002 |
| 4985 | 05947 | 04984 | 1 | | 0 | 0 | 0 | 0 | 0 | 0 | 0 | 0 | 0 | 0 | 0 | 0 | 0 | 0 | 0 | 0 | 1 | 0 | 0 | 0 | 0 | 0 | 0 | 0 | 0 | | | | | | |
| 4986 | 05948 | 04985 | 1 | | 0 | 0 | 0 | 0 | 0 | 0 | 0 | 0 | 0 | 0 | 0 | 0 | 0 | 0 | 0 | 0 | 1 | 0 | 0 | 0 | 0 | 0 | 0 | 0 | 0 | | | | | | |
| 4987 | 05949 | 04986 | 1 | | 0 | 0 | 0 | 0 | 0 | 0 | 0 | 0 | 0 | 0 | 0 | 0 | 0 | 0 | 0 | 0 | 1 | 0 | 0 | 0 | 0 | 0 | 0 | 0 | 0 | | | | | | |
| 4988 | 05951 | 04987 | 1 | | 0 | 0 | 0 | 0 | 0 | 0 | 0 | 0 | 0 | 0 | 0 | 0 | 0 | 0 | 0 | 0 | 1 | 0 | 0 | 0 | 0 | 0 | 0 | 0 | 0 | M65028 | 91.8 | 343 | 1 | 1021 | 1537 |
| 4989 | 05952 | 04988 | 1 | | 0 | 0 | 0 | 0 | 0 | 0 | 0 | 0 | 0 | 0 | 0 | 0 | 0 | 0 | 0 | 0 | 1 | 0 | 0 | 0 | 0 | 0 | 0 | 0 | 0 | | | | | | |

Table 139

| | A | B | C | E | G | I | K | M | O | Q | S | U | W | Y | AA | AC | AF | AG | AI | AK | AM | AO | AQ | AS | AU | AW | AY | BA | BC | BE | BF | BG | BH | BI | BK |
|---|---|---|---|---|---|---|---|---|---|---|---|---|---|---|---|---|---|---|---|---|---|---|---|---|---|---|---|---|---|---|---|---|---|---|---|
| 4990 | 05953 | 04989 | 1 | | 0 | 0 | 0 | 0 | 0 | 0 | 0 | 0 | 0 | 0 | 0 | 0 | 0 | 0 | 0 | 0 | 1 | 0 | 0 | 0 | 0 | 0 | 0 | 0 | 0 | M37712 | 99.3 | 147 | 1 | 3717 | 3863 |
| 4991 | 05954 | 04990 | 2 | | 0 | 0 | 0 | 0 | 0 | 0 | 0 | 0 | 0 | 0 | 0 | 0 | 0 | 0 | 0 | 0 | 1 | 0 | 0 | 0 | 1 | 0 | 0 | 0 | 0 | | | | | | |
| 4992 | 05955 | 04991 | 5 | | 0 | 0 | 0 | 0 | 0 | 0 | 0 | 0 | 0 | 0 | 2 | 0 | 0 | 0 | 0 | 0 | 1 | 0 | 0 | 0 | 0 | 2 | 0 | 0 | 0 | | | | | | |
| 4993 | 05956 | 04992 | 1 | | 0 | 0 | 0 | 0 | 0 | 0 | 0 | 0 | 0 | 0 | 0 | 0 | 0 | 0 | 0 | 0 | 1 | 0 | 0 | 0 | 0 | 0 | 0 | 0 | 0 | | | | | | |
| 4994 | 05957 | 04993 | 1 | | 0 | 0 | 0 | 0 | 0 | 0 | 0 | 0 | 0 | 0 | 0 | 0 | 0 | 0 | 0 | 0 | 1 | 0 | 0 | 0 | 0 | 0 | 0 | 0 | 0 | | | | | | |
| 4995 | 05959 | 04994 | 3 | | 0 | 0 | 0 | 0 | 0 | 0 | 0 | 0 | 0 | 0 | 0 | 0 | 0 | 0 | 0 | 1 | 1 | 0 | 0 | 0 | 1 | 0 | 0 | 0 | 0 | M27319 | 98.5 | 65 | 1 | 735 | 799 |
| 4996 | 05960 | 04995 | 1 | | 0 | 0 | 0 | 0 | 0 | 0 | 0 | 0 | 0 | 0 | 0 | 0 | 0 | 0 | 0 | 0 | 1 | 0 | 0 | 0 | 0 | 0 | 0 | 0 | 0 | | | | | | |
| 4997 | 05961 | 04996 | 1 | | 0 | 0 | 0 | 0 | 0 | 0 | 0 | 0 | 0 | 0 | 0 | 0 | 0 | 0 | 0 | 0 | 1 | 0 | 0 | 0 | 0 | 0 | 0 | 0 | 0 | | | | | | |
| 4998 | 05962 | 04997 | 1 | | 0 | 0 | 0 | 0 | 0 | 0 | 0 | 0 | 0 | 0 | 0 | 0 | 0 | 0 | 0 | 0 | 1 | 0 | 0 | 0 | 0 | 0 | 0 | 0 | 0 | | | | | | |
| 4999 | 05963 | 04998 | 1 | | 0 | 0 | 0 | 0 | 0 | 0 | 0 | 0 | 0 | 0 | 0 | 0 | 0 | 0 | 0 | 0 | 1 | 0 | 0 | 0 | 0 | 0 | 0 | 0 | 0 | | | | | | |
| 5000 | 05964 | 04999 | 1 | | 0 | 0 | 0 | 0 | 0 | 0 | 0 | 0 | 0 | 0 | 0 | 0 | 0 | 0 | 0 | 0 | 1 | 0 | 0 | 0 | 0 | 0 | 0 | 0 | 0 | | | | | | |
| 5001 | 05966 | 05000 | 1 | | 0 | 0 | 0 | 0 | 0 | 0 | 0 | 0 | 0 | 0 | 0 | 0 | 0 | 0 | 0 | 0 | 1 | 0 | 0 | 0 | 0 | 0 | 0 | 0 | 0 | | | | | | |
| 5002 | 05967 | 05001 | 1 | | 0 | 0 | 0 | 0 | 0 | 0 | 0 | 0 | 0 | 0 | 0 | 0 | 0 | 0 | 0 | 0 | 1 | 0 | 0 | 0 | 0 | 0 | 0 | 0 | 0 | | | | | | |
| 5003 | 05968 | 05002 | 1 | | 0 | 0 | 0 | 0 | 0 | 0 | 0 | 0 | 0 | 0 | 0 | 0 | 0 | 0 | 0 | 0 | 1 | 0 | 0 | 0 | 0 | 0 | 0 | 0 | 0 | | | | | | |
| 5004 | 05969 | 05003 | 1 | | 0 | 0 | 0 | 0 | 0 | 0 | 0 | 0 | 0 | 0 | 0 | 0 | 0 | 0 | 0 | 0 | 1 | 0 | 0 | 0 | 0 | 0 | 0 | 0 | 0 | | | | | | |
| 5005 | 05970 | 05004 | 1 | | 0 | 0 | 0 | 0 | 0 | 0 | 0 | 0 | 0 | 0 | 0 | 0 | 0 | 0 | 0 | 0 | 1 | 0 | 0 | 0 | 0 | 0 | 0 | 0 | 0 | | | | | | |
| 5006 | 05974 | 05005 | 1 | | 0 | 0 | 0 | 0 | 0 | 0 | 0 | 0 | 0 | 0 | 0 | 0 | 0 | 0 | 0 | 0 | 1 | 0 | 0 | 0 | 0 | 0 | 0 | 0 | 0 | | | | | | |
| 5007 | 05975 | 05006 | 1 | | 0 | 0 | 0 | 0 | 0 | 0 | 0 | 0 | 0 | 0 | 0 | 0 | 0 | 0 | 0 | 0 | 1 | 0 | 0 | 0 | 0 | 0 | 0 | 0 | 0 | | | | | | |
| 5008 | 05976 | 05007 | 1 | | 0 | 0 | 0 | 0 | 0 | 0 | 0 | 0 | 0 | 0 | 0 | 0 | 0 | 0 | 0 | 0 | 1 | 0 | 0 | 0 | 0 | 0 | 0 | 0 | 0 | | | | | | |
| 5009 | 05978 | 05008 | 6 | | 0 | 0 | 0 | 0 | 0 | 0 | 0 | 0 | 0 | 0 | 0 | 0 | 0 | 0 | 0 | 0 | 1 | 5 | 0 | 0 | 0 | 0 | 0 | 0 | 0 | M33197 | 94.8 | 305 | 1 | 495 | 1268 |
| 5010 | 05980 | 05009 | 1 | | 0 | 0 | 0 | 0 | 0 | 0 | 0 | 0 | 0 | 0 | 0 | 0 | 0 | 0 | 0 | 0 | 1 | 0 | 0 | 0 | 0 | 0 | 0 | 0 | 0 | | | | | | |
| 5011 | 05981 | 05010 | 1 | | 0 | 0 | 0 | 0 | 0 | 0 | 0 | 0 | 0 | 0 | 0 | 0 | 0 | 0 | 0 | 0 | 1 | 0 | 0 | 0 | 0 | 0 | 0 | 0 | 0 | M16447 | 91.2 | 330 | 1 | 1097 | 1550 |
| 5012 | 05982 | 05011 | 2 | | 0 | 0 | 0 | 0 | 0 | 0 | 0 | 0 | 0 | 0 | 0 | 0 | 0 | 0 | 0 | 1 | 1 | 0 | 0 | 0 | 0 | 0 | 0 | 0 | 0 | | | | | | |
| 5013 | 05983 | 05012 | 1 | | 0 | 0 | 0 | 0 | 0 | 0 | 0 | 0 | 0 | 0 | 0 | 0 | 0 | 0 | 0 | 0 | 1 | 0 | 0 | 0 | 0 | 0 | 0 | 0 | 0 | | | | | | |
| 5014 | 05984 | 05013 | 3 | | 0 | 0 | 0 | 0 | 0 | 0 | 0 | 0 | 0 | 0 | 0 | 0 | 0 | 0 | 0 | 0 | 1 | 0 | 0 | 0 | 0 | 1 | 0 | 0 | 1 | L24203 | 94.5 | 328 | 1 | 2207 | 3018 |
| 5015 | 05987 | 05014 | 2 | | 0 | 0 | 0 | 0 | 0 | 0 | 0 | 0 | 0 | 0 | 0 | 0 | 0 | 0 | 0 | 0 | 1 | 1 | 0 | 0 | 0 | 0 | 0 | 0 | 0 | | | | | | |
| 5016 | 05988 | 05015 | 1 | | 0 | 0 | 0 | 0 | 0 | 0 | 0 | 0 | 0 | 0 | 0 | 0 | 0 | 0 | 0 | 0 | 1 | 0 | 0 | 0 | 0 | 0 | 0 | 0 | 0 | | | | | | |
| 5017 | 05989 | 05016 | 1 | | 0 | 0 | 0 | 0 | 0 | 0 | 0 | 0 | 0 | 0 | 0 | 0 | 0 | 0 | 0 | 0 | 1 | 0 | 0 | 0 | 0 | 0 | 0 | 0 | 0 | | | | | | |
| 5018 | 05991 | 05017 | 1 | | 0 | 0 | 0 | 0 | 0 | 0 | 0 | 0 | 0 | 0 | 0 | 0 | 0 | 0 | 0 | 0 | 1 | 0 | 0 | 0 | 0 | 0 | 0 | 0 | 0 | | | | | | |
| 5019 | 05992 | 05018 | 1 | | 0 | 0 | 0 | 0 | 0 | 0 | 0 | 0 | 0 | 0 | 0 | 0 | 0 | 0 | 0 | 0 | 1 | 0 | 0 | 0 | 0 | 0 | 0 | 0 | 0 | | | | | | |
| 5020 | 05993 | 05019 | 1 | | 0 | 0 | 0 | 0 | 0 | 0 | 0 | 0 | 0 | 0 | 0 | 0 | 0 | 0 | 0 | 0 | 1 | 0 | 0 | 0 | 0 | 0 | 0 | 0 | 0 | | | | | | |
| 5021 | 05994 | 05020 | 1 | | 0 | 0 | 0 | 0 | 0 | 0 | 0 | 0 | 0 | 0 | 0 | 0 | 0 | 0 | 0 | 0 | 1 | 0 | 0 | 0 | 0 | 0 | 0 | 0 | 0 | | | | | | |
| 5022 | 05996 | 05021 | 1 | | 0 | 0 | 0 | 0 | 0 | 0 | 0 | 0 | 0 | 0 | 0 | 0 | 0 | 0 | 0 | 0 | 1 | 0 | 0 | 0 | 0 | 0 | 0 | 0 | 0 | | | | | | |
| 5023 | 05997 | 05022 | 1 | | 0 | 0 | 0 | 0 | 0 | 0 | 0 | 0 | 0 | 0 | 0 | 0 | 0 | 0 | 0 | 0 | 1 | 0 | 0 | 0 | 0 | 0 | 0 | 0 | 0 | | | | | | |
| 5024 | 05998 | 05023 | 1 | | 0 | 0 | 0 | 0 | 0 | 0 | 0 | 0 | 0 | 0 | 0 | 0 | 0 | 0 | 0 | 0 | 1 | 0 | 0 | 0 | 0 | 0 | 0 | 0 | 0 | | | | | | |
| 5025 | 05999 | 05024 | 1 | | 0 | 0 | 0 | 0 | 0 | 0 | 0 | 0 | 0 | 0 | 0 | 0 | 0 | 0 | 0 | 0 | 1 | 0 | 0 | 0 | 0 | 0 | 0 | 0 | 0 | | | | | | |

Table 140

| | A | B | C | E | G | I | K | M | O | Q | S | U | W | Y | AA | AC | AE | AG | AI | AK | AM | AO | AQ | AS | AU | AW | AY | BA | BC | BE | BF | BG | BH | BI | BK |
|---|---|---|---|---|---|---|---|---|---|---|---|---|---|---|---|---|---|---|---|---|---|---|---|---|---|---|---|---|---|---|---|---|---|---|---|
| 5026 | 06000 | 05025 | 1 | | 0 | 0 | 0 | 0 | 0 | 0 | 0 | 0 | 0 | 0 | 0 | 0 | 0 | 0 | 0 | 0 | 1 | 0 | 0 | 0 | 0 | 0 | 0 | 0 | 0 | | | | | | |
| 5027 | 06001 | 05026 | 1 | | 0 | 0 | 0 | 0 | 0 | 0 | 0 | 0 | 0 | 0 | 0 | 0 | 0 | 0 | 0 | 0 | 1 | 0 | 0 | 0 | 0 | 0 | 0 | 0 | 0 | | | | | | |
| 5028 | 06004 | 05027 | 1 | | 0 | 0 | 0 | 0 | 0 | 0 | 0 | 0 | 0 | 0 | 0 | 0 | 0 | 0 | 0 | 0 | 1 | 0 | 0 | 0 | 0 | 0 | 0 | 0 | 0 | | | | | | |
| 5029 | 06005 | 05028 | 1 | | 0 | 0 | 0 | 0 | 0 | 0 | 0 | 0 | 0 | 0 | 0 | 0 | 0 | 0 | 0 | 0 | 1 | 0 | 0 | 0 | 0 | 0 | 0 | 0 | 0 | | | | | | |
| 5030 | 06008 | 05029 | 1 | | 0 | 0 | 0 | 0 | 0 | 0 | 0 | 0 | 0 | 0 | 0 | 0 | 0 | 0 | 0 | 0 | 1 | 0 | 0 | 0 | 0 | 0 | 0 | 0 | 0 | | | | | | |
| 5031 | 06010 | 05030 | 1 | | 0 | 0 | 0 | 0 | 0 | 0 | 0 | 0 | 0 | 0 | 0 | 0 | 0 | 0 | 0 | 0 | 1 | 0 | 0 | 0 | 0 | 0 | 0 | 0 | 0 | | | | | | |
| 5032 | 06011 | 05031 | 1 | | 0 | 0 | 0 | 0 | 0 | 0 | 0 | 0 | 0 | 0 | 0 | 0 | 0 | 0 | 0 | 0 | 1 | 0 | 0 | 0 | 0 | 0 | 0 | 0 | 0 | | | | | | |
| 5033 | 06013 | 05032 | 3 | | 0 | 0 | 0 | 0 | 0 | 0 | 0 | 0 | 0 | 0 | 0 | 0 | 0 | 0 | 0 | 0 | 1 | 0 | 0 | 0 | 1 | 0 | 0 | 1 | 0 | | | | | | |
| 5034 | 06014 | 05033 | 1 | | 0 | 0 | 0 | 0 | 0 | 0 | 0 | 0 | 0 | 0 | 0 | 0 | 0 | 0 | 0 | 0 | 1 | 0 | 0 | 0 | 0 | 0 | 0 | 0 | 0 | | | | | | |
| 5035 | 06015 | 05034 | 1 | | 0 | 0 | 0 | 0 | 0 | 0 | 0 | 0 | 0 | 0 | 0 | 0 | 0 | 0 | 0 | 0 | 1 | 0 | 0 | 0 | 0 | 0 | 0 | 0 | 0 | | | | | | |
| 5036 | 06016 | 05035 | 1 | | 0 | 0 | 0 | 0 | 0 | 0 | 0 | 0 | 0 | 0 | 0 | 0 | 0 | 0 | 0 | 0 | 1 | 0 | 0 | 0 | 0 | 0 | 0 | 0 | 0 | | | | | | |
| 5037 | 06017 | 05036 | 2 | | 0 | 0 | 0 | 0 | 0 | 0 | 0 | 0 | 0 | 0 | 0 | 0 | 0 | 0 | 0 | 0 | 1 | 0 | 0 | 1 | 0 | 0 | 0 | 0 | 0 | | | | | | |
| 5038 | 06018 | 05037 | 1 | | 0 | 0 | 0 | 0 | 0 | 0 | 0 | 0 | 0 | 0 | 0 | 0 | 0 | 0 | 0 | 0 | 1 | 0 | 0 | 0 | 0 | 0 | 0 | 0 | 0 | | | | | | |
| 5039 | 06020 | 05038 | 1 | | 0 | 0 | 0 | 0 | 0 | 0 | 0 | 0 | 0 | 0 | 0 | 0 | 0 | 0 | 0 | 0 | 1 | 0 | 0 | 0 | 0 | 0 | 0 | 0 | 0 | | | | | | |
| 5040 | 06021 | 05039 | 1 | | 0 | 0 | 0 | 0 | 0 | 0 | 0 | 0 | 0 | 0 | 0 | 0 | 0 | 0 | 0 | 0 | 1 | 0 | 0 | 0 | 0 | 0 | 0 | 0 | 0 | | | | | | |
| 5041 | 06022 | 05040 | 1 | | 0 | 0 | 0 | 0 | 0 | 0 | 0 | 0 | 0 | 0 | 0 | 0 | 0 | 0 | 0 | 0 | 1 | 0 | 0 | 0 | 0 | 0 | 0 | 0 | 0 | | | | | | |
| 5042 | 06023 | 05041 | 1 | | 0 | 0 | 0 | 0 | 0 | 0 | 0 | 0 | 0 | 0 | 0 | 0 | 0 | 0 | 0 | 0 | 1 | 0 | 0 | 0 | 0 | 0 | 0 | 0 | 0 | | | | | | |
| 5043 | 06024 | 05042 | 2 | | 0 | 0 | 0 | 0 | 0 | 0 | 0 | 0 | 0 | 0 | 0 | 0 | 0 | 0 | 0 | 1 | 1 | 0 | 0 | 0 | 0 | 0 | 0 | 0 | 0 | M60801 | 97.8 | 90 | 1 | 1279 | 1368 |
| 5044 | 06025 | 05043 | 1 | | 0 | 0 | 0 | 0 | 0 | 0 | 0 | 0 | 0 | 0 | 0 | 0 | 0 | 0 | 0 | 0 | 1 | 0 | 0 | 0 | 0 | 0 | 0 | 0 | 0 | | | | | | |
| 5045 | 06026 | 05044 | 4 | | 0 | 0 | 0 | 0 | 0 | 0 | 0 | 0 | 0 | 0 | 0 | 0 | 0 | 2 | 0 | 1 | 0 | 0 | 0 | 0 | 0 | 1 | 0 | 0 | 0 | | | | | | |
| 5046 | 06027 | 05045 | 2 | | 0 | 0 | 0 | 0 | 0 | 0 | 0 | 0 | 0 | 0 | 0 | 0 | 0 | 0 | 0 | 2 | 0 | 0 | 0 | 0 | 0 | 0 | 0 | 0 | 0 | | | | | | |
| 5047 | 06028 | 05046 | 2 | | 0 | 0 | 0 | 0 | 0 | 0 | 0 | 0 | 0 | 0 | 0 | 0 | 0 | 0 | 0 | 2 | 0 | 0 | 0 | 0 | 0 | 0 | 0 | 0 | 0 | | | | | | |
| 5048 | 06030 | 05047 | 1 | | 0 | 0 | 0 | 0 | 0 | 0 | 0 | 0 | 0 | 0 | 0 | 0 | 0 | 0 | 0 | 1 | 0 | 0 | 0 | 0 | 0 | 0 | 0 | 0 | 0 | | | | | | |
| 5049 | 06031 | 05048 | 1 | | 0 | 0 | 0 | 0 | 0 | 0 | 0 | 0 | 0 | 0 | 0 | 0 | 0 | 0 | 0 | 1 | 0 | 0 | 0 | 0 | 0 | 0 | 0 | 0 | 0 | | | | | | |
| 5050 | 06032 | 05049 | 1 | | 0 | 0 | 0 | 0 | 0 | 0 | 0 | 0 | 0 | 0 | 0 | 0 | 0 | 0 | 0 | 1 | 0 | 0 | 0 | 0 | 0 | 0 | 0 | 0 | 0 | | | | | | |
| 5051 | 06034 | 05050 | 1 | | 0 | 0 | 0 | 0 | 0 | 0 | 0 | 0 | 0 | 0 | 0 | 0 | 0 | 0 | 0 | 1 | 0 | 0 | 0 | 0 | 0 | 0 | 0 | 0 | 0 | | | | | | |
| 5052 | 06035 | 05051 | 1 | | 0 | 0 | 0 | 0 | 0 | 0 | 0 | 0 | 0 | 0 | 0 | 0 | 0 | 0 | 0 | 1 | 0 | 0 | 0 | 0 | 0 | 0 | 0 | 0 | 0 | | | | | | |
| 5053 | 06036 | 05052 | 2 | | 0 | 0 | 0 | 0 | 0 | 0 | 0 | 0 | 0 | 0 | 0 | 0 | 0 | 0 | 0 | 1 | 0 | 0 | 0 | 1 | 0 | 0 | 0 | 0 | 0 | | | | | | |
| 5054 | 06038 | 05053 | 2 | | 0 | 0 | 0 | 0 | 0 | 0 | 0 | 0 | 0 | 0 | 0 | 0 | 0 | 0 | 0 | 1 | 0 | 0 | 0 | 0 | 0 | 1 | 0 | 0 | 0 | | | | | | |
| 5055 | 06039 | 05054 | 1 | | 0 | 0 | 0 | 0 | 0 | 0 | 0 | 0 | 0 | 0 | 0 | 0 | 0 | 0 | 0 | 1 | 0 | 0 | 0 | 0 | 0 | 0 | 0 | 0 | 0 | | | | | | |
| 5056 | 06040 | 05055 | 1 | | 0 | 0 | 0 | 0 | 0 | 0 | 0 | 0 | 0 | 0 | 0 | 0 | 0 | 0 | 0 | 1 | 0 | 0 | 0 | 0 | 0 | 0 | 0 | 0 | 0 | | | | | | |
| 5057 | 06041 | 05056 | 3 | | 0 | 0 | 0 | 0 | 0 | 0 | 0 | 0 | 0 | 0 | 0 | 0 | 0 | 0 | 0 | 3 | 0 | 0 | 0 | 0 | 0 | 0 | 0 | 0 | 0 | X68314 | 98.1 | 316 | 1 | 629 | 971 |
| 5058 | 06042 | 05057 | 1 | | 0 | 0 | 0 | 0 | 0 | 0 | 0 | 0 | 0 | 0 | 0 | 0 | 0 | 0 | 0 | 1 | 0 | 0 | 0 | 0 | 0 | 0 | 0 | 0 | 0 | | | | | | |
| 5059 | 06043 | 05058 | 1 | | 0 | 0 | 0 | 0 | 0 | 0 | 0 | 0 | 0 | 0 | 0 | 0 | 0 | 0 | 0 | 1 | 0 | 0 | 0 | 0 | 0 | 0 | 0 | 0 | 0 | | | | | | |
| 5060 | 06044 | 05059 | 1 | | 0 | 0 | 0 | 0 | 0 | 0 | 0 | 0 | 0 | 0 | 0 | 0 | 0 | 0 | 0 | 1 | 0 | 0 | 0 | 0 | 0 | 0 | 0 | 0 | 0 | | | | | | |
| 5061 | 06045 | 05060 | 1 | | 0 | 0 | 0 | 0 | 0 | 0 | 0 | 0 | 0 | 0 | 0 | 0 | 0 | 0 | 0 | 1 | 0 | 0 | 0 | 0 | 0 | 0 | 0 | 0 | 0 | | | | | | |

Table 141

| | A | B | C | E | G | I | K | M | O | Q | S | U | W | Y | AA | AC | AE | AG | AI | AK | AM | AO | AQ | AS | AU | AW | AY | BA | BC | BE | BF | BG | BH | BI | BK |
|---|---|---|---|---|---|---|---|---|---|---|---|---|---|---|---|---|---|---|---|---|---|---|---|---|---|---|---|---|---|---|---|---|---|---|---|
| 5062 | 06046 | 05061 | 1 | | 0 | 0 | 0 | 0 | 0 | 0 | 0 | 0 | 0 | 0 | 0 | 0 | 0 | 0 | 0 | 1 | 0 | 0 | 0 | 0 | 0 | 0 | 0 | 0 | 0 | | | | | | |
| 5063 | 06047 | 05062 | 2 | | 0 | 0 | 0 | 0 | 0 | 0 | 0 | 0 | 0 | 0 | 0 | 0 | 0 | 0 | 0 | 1 | 0 | 0 | 0 | 0 | 0 | 0 | 1 | 0 | 0 | | | | | | |
| 5064 | 06048 | 05063 | 2 | | 0 | 0 | 0 | 0 | 0 | 0 | 0 | 0 | 0 | 0 | 0 | 0 | 0 | 0 | 0 | 1 | 0 | 0 | 1 | 0 | 0 | 0 | 0 | 0 | 0 | | | | | | |
| 5065 | 06049 | 05064 | 1 | | 0 | 0 | 0 | 0 | 0 | 0 | 0 | 0 | 0 | 0 | 0 | 0 | 0 | 0 | 0 | 1 | 0 | 0 | 0 | 0 | 0 | 0 | 0 | 0 | 0 | | | | | | |
| 5066 | 06050 | 05065 | 1 | | 0 | 0 | 0 | 0 | 0 | 0 | 0 | 0 | 0 | 0 | 0 | 0 | 0 | 0 | 0 | 1 | 0 | 0 | 0 | 0 | 0 | 0 | 0 | 0 | 0 | | | | | | |
| 5067 | 06051 | 05066 | 1 | | 0 | 0 | 0 | 0 | 0 | 0 | 0 | 0 | 0 | 0 | 0 | 0 | 0 | 0 | 0 | 1 | 0 | 0 | 0 | 0 | 0 | 0 | 0 | 0 | 0 | | | | | | |
| 5068 | 06052 | 05067 | 1 | | 0 | 0 | 0 | 0 | 0 | 0 | 0 | 0 | 0 | 0 | 0 | 0 | 0 | 0 | 0 | 1 | 0 | 0 | 0 | 0 | 0 | 0 | 0 | 0 | 0 | | | | | | |
| 5069 | 06053 | 05068 | 3 | | 0 | 0 | 0 | 0 | 0 | 0 | 0 | 0 | 0 | 0 | 0 | 0 | 0 | 0 | 0 | 3 | 0 | 0 | 0 | 0 | 0 | 0 | 0 | 0 | 0 | | | | | | |
| 5070 | 06054 | 05069 | 1 | | 0 | 0 | 0 | 0 | 0 | 0 | 0 | 0 | 0 | 0 | 0 | 0 | 0 | 0 | 0 | 1 | 0 | 0 | 0 | 0 | 0 | 0 | 0 | 0 | 0 | | | | | | |
| 5071 | 06055 | 05070 | 2 | | 0 | 0 | 0 | 0 | 0 | 0 | 0 | 0 | 0 | 0 | 0 | 0 | 0 | 0 | 0 | 1 | 0 | 0 | 0 | 0 | 0 | 0 | 0 | 1 | 0 | Z22548 | 94.4 | 338 | 1 | 509 | 937 |
| 5072 | 06056 | 05071 | 1 | | 0 | 0 | 0 | 0 | 0 | 0 | 0 | 0 | 0 | 0 | 0 | 0 | 0 | 0 | 0 | 1 | 0 | 0 | 0 | 0 | 0 | 0 | 0 | 0 | 0 | | | | | | |
| 5073 | 06057 | 05072 | 3 | | 0 | 0 | 0 | 0 | 0 | 0 | 0 | 0 | 0 | 0 | 0 | 0 | 0 | 0 | 0 | 1 | 0 | 0 | 0 | 0 | 0 | 0 | 2 | 0 | 0 | | | | | | |
| 5074 | 06059 | 05073 | 1 | | 0 | 0 | 0 | 0 | 0 | 0 | 0 | 0 | 0 | 0 | 0 | 0 | 0 | 0 | 0 | 1 | 0 | 0 | 0 | 0 | 0 | 0 | 0 | 0 | 0 | | | | | | |
| 5075 | 06060 | 05074 | 1 | | 0 | 0 | 0 | 0 | 0 | 0 | 0 | 0 | 0 | 0 | 0 | 0 | 0 | 0 | 0 | 1 | 0 | 0 | 0 | 0 | 0 | 0 | 0 | 0 | 0 | | | | | | |
| 5076 | 06061 | 05075 | 1 | | 0 | 0 | 0 | 0 | 0 | 0 | 0 | 0 | 0 | 0 | 0 | 0 | 0 | 0 | 0 | 1 | 0 | 0 | 0 | 0 | 0 | 0 | 0 | 0 | 0 | | | | | | |
| 5077 | 06062 | 05076 | 1 | | 0 | 0 | 0 | 0 | 0 | 0 | 0 | 0 | 0 | 0 | 0 | 0 | 0 | 0 | 0 | 1 | 0 | 0 | 0 | 0 | 0 | 0 | 0 | 0 | 0 | | | | | | |
| 5078 | 06064 | 05077 | 1 | | 0 | 0 | 0 | 0 | 0 | 0 | 0 | 0 | 0 | 0 | 0 | 0 | 0 | 0 | 0 | 1 | 0 | 0 | 0 | 0 | 0 | 0 | 0 | 0 | 0 | | | | | | |
| 5079 | 06065 | 05078 | 1 | | 0 | 0 | 0 | 0 | 0 | 0 | 0 | 0 | 0 | 0 | 0 | 0 | 0 | 0 | 0 | 1 | 0 | 0 | 0 | 0 | 0 | 0 | 0 | 0 | 0 | | | | | | |
| 5080 | 06066 | 05079 | 1 | | 0 | 0 | 0 | 0 | 0 | 0 | 0 | 0 | 0 | 0 | 0 | 0 | 0 | 0 | 0 | 1 | 0 | 0 | 0 | 0 | 0 | 0 | 0 | 0 | 0 | | | | | | |
| 5081 | 06067 | 05080 | 1 | | 0 | 0 | 0 | 0 | 0 | 0 | 0 | 0 | 0 | 0 | 0 | 0 | 0 | 0 | 0 | 1 | 0 | 0 | 0 | 0 | 0 | 0 | 0 | 0 | 0 | | | | | | |
| 5082 | 06068 | 05081 | 3 | | 0 | 0 | 0 | 0 | 0 | 0 | 0 | 0 | 0 | 0 | 2 | 0 | 0 | 0 | 0 | 1 | 0 | 0 | 0 | 0 | 0 | 0 | 0 | 0 | 0 | | | | | | |
| 5083 | 06069 | 05082 | 4 | | 0 | 0 | 0 | 0 | 0 | 0 | 0 | 0 | 0 | 0 | 0 | 0 | 0 | 0 | 0 | 1 | 0 | 0 | 0 | 0 | 0 | 3 | 0 | 0 | 0 | M77477 | 96.4 | 281 | 1 | 1243 | 1625 |
| 5084 | 06070 | 05083 | 1 | | 0 | 0 | 0 | 0 | 0 | 0 | 0 | 0 | 0 | 0 | 0 | 0 | 0 | 0 | 0 | 1 | 0 | 0 | 0 | 0 | 0 | 0 | 0 | 0 | 0 | | | | | | |
| 5085 | 06071 | 05084 | 1 | | 0 | 0 | 0 | 0 | 0 | 0 | 0 | 0 | 0 | 0 | 0 | 0 | 0 | 0 | 0 | 1 | 0 | 0 | 0 | 0 | 0 | 0 | 0 | 0 | 0 | | | | | | |
| 5086 | 06072 | 05085 | 1 | | 0 | 0 | 0 | 0 | 0 | 0 | 0 | 0 | 0 | 0 | 0 | 0 | 0 | 0 | 0 | 1 | 0 | 0 | 0 | 0 | 0 | 0 | 0 | 0 | 0 | | | | | | |
| 5087 | 06074 | 05086 | 1 | | 0 | 0 | 0 | 0 | 0 | 0 | 0 | 0 | 0 | 0 | 0 | 0 | 0 | 0 | 0 | 1 | 0 | 0 | 0 | 0 | 0 | 0 | 0 | 0 | 0 | | | | | | |
| 5088 | 06075 | 05087 | 1 | | 0 | 0 | 0 | 0 | 0 | 0 | 0 | 0 | 0 | 0 | 0 | 0 | 0 | 0 | 0 | 1 | 0 | 0 | 0 | 0 | 0 | 0 | 0 | 0 | 0 | M10941 | 95.9 | 267 | 1 | 679 | 1296 |
| 5089 | 06076 | 05088 | 3 | | 0 | 0 | 0 | 0 | 0 | 0 | 0 | 0 | 0 | 0 | 0 | 0 | 0 | 0 | 0 | 1 | 0 | 0 | 0 | 2 | 0 | 0 | 0 | 0 | 0 | | | | | | |
| 5090 | 06077 | 05089 | 1 | | 0 | 0 | 0 | 0 | 0 | 0 | 0 | 0 | 0 | 0 | 0 | 0 | 0 | 0 | 0 | 1 | 0 | 0 | 0 | 0 | 0 | 0 | 0 | 0 | 0 | L10284 | 92.5 | 268 | 1 | 2191 | 4117 |
| 5091 | 06078 | 05090 | 2 | | 0 | 0 | 0 | 0 | 0 | 0 | 0 | 0 | 0 | 0 | 0 | 0 | 0 | 0 | 0 | 1 | 0 | 0 | 0 | 0 | 0 | 1 | 0 | 0 | 0 | | | | | | |
| 5092 | 06079 | 05091 | 2 | | 0 | 0 | 0 | 0 | 0 | 0 | 0 | 0 | 0 | 0 | 0 | 0 | 0 | 0 | 0 | 1 | 0 | 0 | 0 | 1 | 0 | 0 | 0 | 0 | 0 | | | | | | |
| 5093 | 06080 | 05092 | 1 | | 0 | 0 | 0 | 0 | 0 | 0 | 0 | 0 | 0 | 0 | 0 | 0 | 0 | 0 | 0 | 1 | 0 | 0 | 0 | 0 | 0 | 0 | 0 | 0 | 0 | | | | | | |
| 5094 | 06081 | 05093 | 1 | | 0 | 0 | 0 | 0 | 0 | 0 | 0 | 0 | 0 | 0 | 0 | 0 | 0 | 0 | 0 | 1 | 0 | 0 | 0 | 0 | 0 | 0 | 0 | 0 | 0 | | | | | | |
| 5095 | 06083 | 05094 | 2 | | 0 | 0 | 0 | 0 | 0 | 0 | 0 | 0 | 0 | 0 | 0 | 0 | 0 | 0 | 0 | 1 | 0 | 0 | 0 | 0 | 0 | 1 | 0 | 0 | 0 | | | | | | |
| 5096 | 06084 | 05095 | 1 | | 0 | 0 | 0 | 0 | 0 | 0 | 0 | 0 | 0 | 0 | 0 | 0 | 0 | 0 | 0 | 1 | 0 | 0 | 0 | 0 | 0 | 0 | 0 | 0 | 0 | X75042 | 98.2 | 55 | 1 | 1868 | 2337 |
| 5097 | 06085 | 05096 | 1 | | 0 | 0 | 0 | 0 | 0 | 0 | 0 | 0 | 0 | 0 | 0 | 0 | 0 | 0 | 0 | 1 | 0 | 0 | 0 | 0 | 0 | 0 | 0 | 0 | 0 | | | | | | |

Table 142

| | A | B | C | E | G | I | K | M | O | Q | S | U | W | Y | AA | AC | AE | AG | AI | AK | AM | AO | AQ | AS | AU | AW | AY | BA | BC | BE | BF | BG | BH | BI | BK |
|---|---|---|---|---|---|---|---|---|---|---|---|---|---|---|---|---|---|---|---|---|---|---|---|---|---|---|---|---|---|---|---|---|---|---|---|
| 5098 | 06086 | 05097 | 1 | | 0 | 0 | 0 | 0 | 0 | 0 | 0 | 0 | 0 | 0 | 0 | 0 | 0 | 0 | 0 | 1 | 0 | 0 | 0 | 0 | 0 | 0 | 0 | 0 | 0 | | | | | | |
| 5099 | 06087 | 05098 | 3 | | 0 | 0 | 0 | 0 | 0 | 0 | 0 | 0 | 0 | 0 | 0 | 0 | 0 | 0 | 0 | 1 | 0 | 0 | 0 | 0 | 0 | 1 | 0 | 1 | 0 | | | | | | |
| 5100 | 06089 | 05099 | 1 | | 0 | 0 | 0 | 0 | 0 | 0 | 0 | 0 | 0 | 0 | 0 | 0 | 0 | 0 | 0 | 1 | 0 | 0 | 0 | 0 | 0 | 0 | 0 | 0 | 0 | | | | | | |
| 5101 | 06090 | 05100 | 1 | | 0 | 0 | 0 | 0 | 0 | 0 | 0 | 0 | 0 | 0 | 0 | 0 | 0 | 0 | 0 | 1 | 0 | 0 | 0 | 0 | 0 | 0 | 0 | 0 | 0 | | | | | | |
| 5102 | 06091 | 05101 | 1 | | 0 | 0 | 0 | 0 | 0 | 0 | 0 | 0 | 0 | 0 | 0 | 0 | 0 | 0 | 0 | 1 | 0 | 0 | 0 | 0 | 0 | 0 | 0 | 0 | 0 | | | | | | |
| 5103 | 06092 | 05102 | 1 | | 0 | 0 | 0 | 0 | 0 | 0 | 0 | 0 | 0 | 0 | 0 | 0 | 0 | 0 | 0 | 1 | 0 | 0 | 0 | 0 | 0 | 0 | 0 | 0 | 0 | | | | | | |
| 5104 | 06093 | 05103 | 1 | | 0 | 0 | 0 | 0 | 0 | 0 | 0 | 0 | 0 | 0 | 0 | 0 | 0 | 0 | 0 | 1 | 0 | 0 | 0 | 0 | 0 | 0 | 0 | 0 | 0 | | | | | | |
| 5105 | 06094 | 05104 | 1 | | 0 | 0 | 0 | 0 | 0 | 0 | 0 | 0 | 0 | 0 | 0 | 0 | 0 | 0 | 0 | 1 | 0 | 0 | 0 | 0 | 0 | 0 | 0 | 0 | 0 | | | | | | |
| 5106 | 06095 | 05105 | 1 | | 0 | 0 | 0 | 0 | 0 | 0 | 0 | 0 | 0 | 0 | 0 | 0 | 0 | 0 | 0 | 1 | 0 | 0 | 0 | 0 | 0 | 0 | 0 | 0 | 0 | | | | | | |
| 5107 | 06097 | 05106 | 1 | | 0 | 0 | 0 | 0 | 0 | 0 | 0 | 0 | 0 | 0 | 0 | 0 | 0 | 0 | 0 | 1 | 0 | 0 | 0 | 0 | 0 | 0 | 0 | 0 | 0 | | | | | | |
| 5108 | 06098 | 05107 | 1 | | 0 | 0 | 0 | 0 | 0 | 0 | 0 | 0 | 0 | 0 | 0 | 0 | 0 | 0 | 0 | 1 | 0 | 0 | 0 | 0 | 0 | 0 | 0 | 0 | 0 | | | | | | |
| 5109 | 06100 | 05108 | 2 | | 0 | 0 | 0 | 0 | 0 | 0 | 0 | 0 | 0 | 0 | 0 | 0 | 0 | 0 | 0 | 1 | 0 | 0 | 0 | 1 | 0 | 0 | 0 | 0 | 0 | | | | | | |
| 5110 | 06101 | 05109 | 1 | | 0 | 0 | 0 | 0 | 0 | 0 | 0 | 0 | 0 | 0 | 0 | 0 | 0 | 0 | 0 | 1 | 0 | 0 | 0 | 0 | 0 | 0 | 0 | 0 | 0 | | | | | | |
| 5111 | 06102 | 05110 | 1 | | 0 | 0 | 0 | 0 | 0 | 0 | 0 | 0 | 0 | 0 | 0 | 0 | 0 | 0 | 0 | 1 | 0 | 0 | 0 | 0 | 0 | 0 | 0 | 0 | 0 | | | | | | |
| 5112 | 06103 | 05111 | 1 | | 0 | 0 | 0 | 0 | 0 | 0 | 0 | 0 | 0 | 0 | 0 | 0 | 0 | 0 | 0 | 1 | 0 | 0 | 0 | 0 | 0 | 0 | 0 | 0 | 0 | | | | | | |
| 5113 | 06104 | 05112 | 1 | | 0 | 0 | 0 | 0 | 0 | 0 | 0 | 0 | 0 | 0 | 0 | 0 | 0 | 0 | 0 | 1 | 0 | 0 | 0 | 0 | 0 | 0 | 0 | 0 | 0 | | | | | | |
| 5114 | 06105 | 05113 | 1 | | 0 | 0 | 0 | 0 | 0 | 0 | 0 | 0 | 0 | 0 | 0 | 0 | 0 | 0 | 0 | 1 | 0 | 0 | 0 | 0 | 0 | 0 | 0 | 0 | 0 | | | | | | |
| 5115 | 06107 | 05114 | 3 | | 0 | 0 | 0 | 0 | 0 | 0 | 0 | 0 | 0 | 0 | 0 | 0 | 0 | 0 | 0 | 2 | 0 | 0 | 0 | 1 | 0 | 0 | 0 | 0 | 0 | | | | | | |
| 5116 | 06108 | 05115 | 1 | | 0 | 0 | 0 | 0 | 0 | 0 | 0 | 0 | 0 | 0 | 0 | 0 | 0 | 0 | 0 | 1 | 0 | 0 | 0 | 0 | 0 | 0 | 0 | 0 | 0 | M58510 | 98.8 | 84 | 1 | 100 | 3355 |
| 5117 | 06109 | 05116 | 1 | | 0 | 0 | 0 | 0 | 0 | 0 | 0 | 0 | 0 | 0 | 0 | 0 | 0 | 0 | 0 | 1 | 0 | 0 | 0 | 0 | 0 | 0 | 0 | 0 | 0 | | | | | | |
| 5118 | 06110 | 05117 | 2 | | 0 | 0 | 0 | 0 | 0 | 0 | 0 | 0 | 0 | 0 | 0 | 0 | 0 | 0 | 0 | 1 | 0 | 1 | 0 | 0 | 0 | 0 | 0 | 0 | 0 | | | | | | |
| 5119 | 06111 | 05118 | 1 | | 0 | 0 | 0 | 0 | 0 | 0 | 0 | 0 | 0 | 0 | 0 | 0 | 0 | 0 | 0 | 1 | 0 | 0 | 0 | 0 | 0 | 0 | 0 | 0 | 0 | | | | | | |
| 5120 | 06112 | 05119 | 1 | | 0 | 0 | 0 | 0 | 0 | 0 | 0 | 0 | 0 | 0 | 0 | 0 | 0 | 0 | 0 | 1 | 0 | 0 | 0 | 0 | 0 | 0 | 0 | 0 | 0 | | | | | | |
| 5121 | 06113 | 05120 | 1 | | 0 | 0 | 0 | 0 | 0 | 0 | 0 | 0 | 0 | 0 | 0 | 0 | 0 | 0 | 0 | 1 | 0 | 0 | 0 | 0 | 0 | 0 | 0 | 0 | 0 | | | | | | |
| 5122 | 06114 | 05121 | 1 | | 0 | 0 | 0 | 0 | 0 | 0 | 0 | 0 | 0 | 0 | 0 | 0 | 0 | 0 | 0 | 1 | 0 | 0 | 0 | 0 | 0 | 0 | 0 | 0 | 0 | | | | | | |
| 5123 | 06115 | 05122 | 1 | | 0 | 0 | 0 | 0 | 0 | 0 | 0 | 0 | 0 | 0 | 0 | 0 | 0 | 0 | 0 | 1 | 0 | 0 | 0 | 0 | 0 | 0 | 0 | 0 | 0 | | | | | | |
| 5124 | 06116 | 05123 | 1 | | 0 | 0 | 0 | 0 | 0 | 0 | 0 | 0 | 0 | 0 | 0 | 0 | 0 | 0 | 0 | 1 | 0 | 0 | 0 | 0 | 0 | 0 | 0 | 0 | 0 | | | | | | |
| 5125 | 06117 | 05124 | 3 | | 0 | 0 | 0 | 0 | 0 | 0 | 0 | 0 | 0 | 0 | 0 | 0 | 0 | 0 | 0 | 2 | 0 | 0 | 0 | 0 | 1 | 0 | 0 | 0 | 0 | | | | | | |
| 5126 | 06118 | 05125 | 1 | | 0 | 0 | 0 | 0 | 0 | 0 | 0 | 0 | 0 | 0 | 0 | 0 | 0 | 0 | 0 | 1 | 0 | 0 | 0 | 0 | 0 | 0 | 0 | 0 | 0 | | | | | | |
| 5127 | 06119 | 05126 | 2 | | 0 | 0 | 0 | 0 | 0 | 0 | 0 | 0 | 0 | 0 | 0 | 0 | 0 | 0 | 0 | 1 | 0 | 0 | 1 | 0 | 0 | 0 | 0 | 0 | 0 | | | | | | |
| 5128 | 06122 | 05127 | 1 | | 0 | 0 | 0 | 0 | 0 | 0 | 0 | 0 | 0 | 0 | 0 | 0 | 0 | 0 | 0 | 1 | 0 | 0 | 0 | 0 | 0 | 0 | 0 | 0 | 0 | | | | | | |
| 5129 | 06123 | 05128 | 1 | | 0 | 0 | 0 | 0 | 0 | 0 | 0 | 0 | 0 | 0 | 0 | 0 | 0 | 0 | 0 | 1 | 0 | 0 | 0 | 0 | 0 | 0 | 0 | 0 | 0 | | | | | | |
| 5130 | 06125 | 05129 | 2 | | 0 | 0 | 0 | 0 | 0 | 0 | 0 | 0 | 0 | 0 | 0 | 0 | 0 | 0 | 0 | 1 | 0 | 0 | 0 | 1 | 0 | 0 | 0 | 0 | 0 | | | | | | |
| 5131 | 06126 | 05130 | 3 | | 0 | 0 | 0 | 0 | 0 | 0 | 0 | 0 | 0 | 0 | 0 | 0 | 0 | 0 | 0 | 2 | 0 | 0 | 0 | 0 | 1 | 0 | 0 | 0 | 0 | K03515 | 98.8 | 339 | 1 | 1506 | 1987 |
| 5132 | 06127 | 05131 | 1 | | 0 | 0 | 0 | 0 | 0 | 0 | 0 | 0 | 0 | 0 | 0 | 0 | 0 | 0 | 0 | 1 | 0 | 0 | 0 | 0 | 0 | 0 | 0 | 0 | 0 | | | | | | |
| 5133 | 06128 | 05132 | 1 | | 0 | 0 | 0 | 0 | 0 | 0 | 0 | 0 | 0 | 0 | 0 | 0 | 0 | 0 | 0 | 1 | 0 | 0 | 0 | 0 | 0 | 0 | 0 | 0 | 0 | | | | | | |

Table 143

| | A | B | C | E | G | I | K | M | O | Q | S | U | W | Y | AA | AC | AE | AG | AI | AK | AM | AO | AQ | AS | AU | AW | AY | BA | BC | BE | BF | BG | BH | BI | BK |
|---|---|---|---|---|---|---|---|---|---|---|---|---|---|---|---|---|---|---|---|---|---|---|---|---|---|---|---|---|---|---|---|---|---|---|---|
| 5134 | 06129 | 05133 | 1 | | 0 | 0 | 0 | 0 | 0 | 0 | 0 | 0 | 0 | 0 | 0 | 0 | 0 | 0 | 0 | 1 | 0 | 0 | 0 | 0 | 0 | 0 | 0 | 0 | 0 | | | | | | |
| 5135 | 06130 | 05134 | 1 | | 0 | 0 | 0 | 0 | 0 | 0 | 0 | 0 | 0 | 0 | 0 | 0 | 0 | 0 | 0 | 1 | 0 | 0 | 0 | 0 | 0 | 0 | 0 | 0 | 0 | | | | | | |
| 5136 | 06131 | 05135 | 1 | | 0 | 0 | 0 | 0 | 0 | 0 | 0 | 0 | 0 | 0 | 0 | 0 | 0 | 0 | 0 | 1 | 0 | 0 | 0 | 0 | 0 | 0 | 0 | 0 | 0 | | | | | | |
| 5137 | 06133 | 05136 | 1 | | 0 | 0 | 0 | 0 | 0 | 0 | 0 | 0 | 0 | 0 | 0 | 0 | 0 | 0 | 0 | 1 | 0 | 0 | 0 | 0 | 0 | 0 | 0 | 0 | 0 | | | | | | |
| 5138 | 06136 | 05137 | 1 | | 0 | 0 | 0 | 0 | 0 | 0 | 0 | 0 | 0 | 0 | 0 | 0 | 0 | 0 | 0 | 1 | 0 | 0 | 0 | 0 | 0 | 0 | 0 | 0 | 0 | | | | | | |
| 5139 | 06137 | 05138 | 2 | | 0 | 0 | 0 | 0 | 0 | 0 | 0 | 0 | 0 | 0 | 0 | 0 | 0 | 0 | 0 | 1 | 0 | 0 | 0 | 1 | 0 | 0 | 0 | 0 | 0 | | | | | | |
| 5140 | 06138 | 05139 | 2 | | 0 | 0 | 0 | 0 | 0 | 0 | 0 | 0 | 0 | 0 | 0 | 0 | 0 | 0 | 0 | 2 | 0 | 0 | 0 | 0 | 0 | 0 | 0 | 0 | 0 | | | | | | |
| 5141 | 06139 | 05140 | 3 | | 0 | 0 | 0 | 0 | 0 | 0 | 0 | 0 | 0 | 0 | 0 | 0 | 0 | 0 | 0 | 2 | 0 | 0 | 0 | 0 | 0 | 0 | 0 | 1 | 0 | | | | | | |
| 5142 | 06141 | 05141 | 1 | | 0 | 0 | 0 | 0 | 0 | 0 | 0 | 0 | 0 | 0 | 0 | 0 | 0 | 0 | 0 | 1 | 0 | 0 | 0 | 0 | 0 | 0 | 0 | 0 | 0 | | | | | | |
| 5143 | 06142 | 05142 | 1 | | 0 | 0 | 0 | 0 | 0 | 0 | 0 | 0 | 0 | 0 | 0 | 0 | 0 | 0 | 0 | 1 | 0 | 0 | 0 | 0 | 0 | 0 | 0 | 0 | 0 | | | | | | |
| 5144 | 06144 | 05143 | 1 | | 0 | 0 | 0 | 0 | 0 | 0 | 0 | 0 | 0 | 0 | 0 | 0 | 0 | 0 | 0 | 1 | 0 | 0 | 0 | 0 | 0 | 0 | 0 | 0 | 0 | | | | | | |
| 5145 | 06145 | 05144 | 1 | | 0 | 0 | 0 | 0 | 0 | 0 | 0 | 0 | 0 | 0 | 0 | 0 | 0 | 0 | 0 | 1 | 0 | 0 | 0 | 0 | 0 | 0 | 0 | 0 | 0 | | | | | | |
| 5146 | 06146 | 05145 | 1 | | 0 | 0 | 0 | 0 | 0 | 0 | 0 | 0 | 0 | 0 | 0 | 0 | 0 | 0 | 0 | 1 | 0 | 0 | 0 | 0 | 0 | 0 | 0 | 0 | 0 | | | | | | |
| 5147 | 06148 | 05146 | 1 | | 0 | 0 | 0 | 0 | 0 | 0 | 0 | 0 | 0 | 0 | 0 | 0 | 0 | 0 | 0 | 1 | 0 | 0 | 0 | 0 | 0 | 0 | 0 | 0 | 0 | 222534 | 96 | 321 | 1 | 1930 | 2715 |
| 5148 | 06150 | 05147 | 4 | | 0 | 0 | 0 | 0 | 0 | 0 | 0 | 0 | 0 | 0 | 0 | 0 | 0 | 1 | 0 | 1 | 0 | 0 | 0 | 0 | 1 | 0 | 1 | 0 | 0 | | | | | | |
| 5149 | 06151 | 05148 | 2 | | 0 | 0 | 0 | 0 | 0 | 0 | 0 | 0 | 0 | 0 | 0 | 0 | 0 | 0 | 0 | 1 | 0 | 1 | 0 | 0 | 0 | 0 | 0 | 0 | 0 | | | | | | |
| 5150 | 06152 | 05149 | 1 | | 0 | 0 | 0 | 0 | 0 | 0 | 0 | 0 | 0 | 0 | 0 | 0 | 0 | 0 | 0 | 1 | 0 | 0 | 0 | 0 | 0 | 0 | 0 | 0 | 0 | | | | | | |
| 5151 | 06153 | 05150 | 2 | | 0 | 0 | 0 | 0 | 0 | 0 | 0 | 0 | 0 | 0 | 0 | 0 | 0 | 0 | 0 | 1 | 0 | 0 | 0 | 0 | 0 | 0 | 0 | 1 | 0 | | | | | | |
| 5152 | 06154 | 05151 | 1 | | 0 | 0 | 0 | 0 | 0 | 0 | 0 | 0 | 0 | 0 | 0 | 0 | 0 | 0 | 0 | 1 | 0 | 0 | 0 | 0 | 0 | 0 | 0 | 0 | 0 | | | | | | |
| 5153 | 06155 | 05152 | 1 | | 0 | 0 | 0 | 0 | 0 | 0 | 0 | 0 | 0 | 0 | 0 | 0 | 0 | 0 | 0 | 1 | 0 | 0 | 0 | 0 | 0 | 0 | 0 | 0 | 0 | | | | | | |
| 5154 | 06156 | 05153 | 1 | | 0 | 0 | 0 | 0 | 0 | 0 | 0 | 0 | 0 | 0 | 0 | 0 | 0 | 0 | 0 | 1 | 0 | 0 | 0 | 0 | 0 | 0 | 0 | 0 | 0 | | | | | | |
| 5155 | 06157 | 05154 | 1 | | 0 | 0 | 0 | 0 | 0 | 0 | 0 | 0 | 0 | 0 | 0 | 0 | 0 | 0 | 0 | 1 | 0 | 0 | 0 | 0 | 0 | 0 | 0 | 0 | 0 | | | | | | |
| 5156 | 06158 | 05155 | 1 | | 0 | 0 | 0 | 0 | 0 | 0 | 0 | 0 | 0 | 0 | 0 | 0 | 0 | 0 | 0 | 1 | 0 | 0 | 0 | 0 | 0 | 0 | 0 | 0 | 0 | | | | | | |
| 5157 | 06159 | 05156 | 1 | | 0 | 0 | 0 | 0 | 0 | 0 | 0 | 0 | 0 | 0 | 0 | 0 | 0 | 0 | 0 | 1 | 0 | 0 | 0 | 0 | 0 | 0 | 0 | 0 | 0 | | | | | | |
| 5158 | 06160 | 05157 | 2 | | 0 | 0 | 0 | 0 | 0 | 0 | 0 | 0 | 0 | 0 | 1 | 0 | 0 | 0 | 0 | 1 | 0 | 0 | 0 | 0 | 0 | 0 | 0 | 0 | 0 | | | | | | |
| 5159 | 06161 | 05158 | 1 | | 0 | 0 | 0 | 0 | 0 | 0 | 0 | 0 | 0 | 0 | 0 | 0 | 0 | 0 | 0 | 1 | 0 | 0 | 0 | 0 | 0 | 0 | 0 | 0 | 0 | | | | | | |
| 5160 | 06163 | 05159 | 1 | | 0 | 0 | 0 | 0 | 0 | 0 | 0 | 0 | 0 | 0 | 0 | 0 | 0 | 0 | 0 | 1 | 0 | 0 | 0 | 0 | 0 | 0 | 0 | 0 | 0 | | | | | | |
| 5161 | 06164 | 05160 | 2 | | 0 | 0 | 0 | 0 | 0 | 0 | 0 | 0 | 0 | 0 | 0 | 0 | 0 | 0 | 0 | 2 | 0 | 0 | 0 | 0 | 0 | 0 | 0 | 0 | 0 | | | | | | |
| 5162 | 06165 | 05161 | 1 | | 0 | 0 | 0 | 0 | 0 | 0 | 0 | 0 | 0 | 0 | 0 | 0 | 0 | 0 | 0 | 1 | 0 | 0 | 0 | 0 | 0 | 0 | 0 | 0 | 0 | | | | | | |
| 5163 | 06166 | 05162 | 1 | | 0 | 0 | 0 | 0 | 0 | 0 | 0 | 0 | 0 | 0 | 0 | 0 | 0 | 0 | 0 | 1 | 0 | 0 | 0 | 0 | 0 | 0 | 0 | 0 | 0 | | | | | | |
| 5164 | 06167 | 05163 | 1 | | 0 | 0 | 0 | 0 | 0 | 0 | 0 | 0 | 0 | 0 | 0 | 0 | 0 | 0 | 0 | 1 | 0 | 0 | 0 | 0 | 0 | 0 | 0 | 0 | 0 | | | | | | |
| 5165 | 06168 | 05164 | 1 | | 0 | 0 | 0 | 0 | 0 | 0 | 0 | 0 | 0 | 0 | 0 | 0 | 0 | 0 | 0 | 1 | 0 | 0 | 0 | 0 | 0 | 0 | 0 | 0 | 0 | | | | | | |
| 5166 | 06169 | 05165 | 1 | | 0 | 0 | 0 | 0 | 0 | 0 | 0 | 0 | 0 | 0 | 0 | 0 | 0 | 0 | 0 | 1 | 0 | 0 | 0 | 0 | 0 | 0 | 0 | 0 | 0 | 213009 | 96.6 | 321 | 1 | 4457 | 4778 |
| 5167 | 06170 | 05166 | 2 | | 0 | 0 | 0 | 0 | 0 | 0 | 0 | 0 | 0 | 0 | 0 | 0 | 0 | 0 | 0 | 1 | 0 | 0 | 0 | 0 | 1 | 0 | 0 | 0 | 0 | | | | | | |
| 5168 | 06171 | 05167 | 1 | | 0 | 0 | 0 | 0 | 0 | 0 | 0 | 0 | 0 | 0 | 0 | 0 | 0 | 0 | 0 | 1 | 0 | 0 | 0 | 0 | 0 | 0 | 0 | 0 | 0 | | | | | | |
| 5169 | 06172 | 05168 | 2 | | 0 | 0 | 0 | 0 | 0 | 0 | 0 | 0 | 0 | 0 | 0 | 0 | 0 | 0 | 0 | 1 | 0 | 0 | 1 | 0 | 0 | 0 | 0 | 0 | 0 | | | | | | |

Table 144

| | A | B | C | E | G | I | K | M | O | Q | S | U | W | Y | AA | AC | AE | AG | AI | AK | AM | AO | AQ | AS | AU | AW | AY | BA | BC | BE | BF | BG | BH | BI | BK |
|---|---|---|---|---|---|---|---|---|---|---|---|---|---|---|---|---|---|---|---|---|---|---|---|---|---|---|---|---|---|---|---|---|---|---|---|
| 5170 | 06173 | 05169 | 1 | | 0 | 0 | 0 | 0 | 0 | 0 | 0 | 0 | 0 | 0 | 0 | 0 | 0 | 0 | 0 | 1 | 0 | 0 | 0 | 0 | 0 | 0 | 0 | 0 | 0 | | | | | | |
| 5171 | 06174 | 05170 | 1 | | 0 | 0 | 0 | 0 | 0 | 0 | 0 | 0 | 0 | 0 | 0 | 0 | 0 | 0 | 0 | 1 | 0 | 0 | 0 | 0 | 0 | 0 | 0 | 0 | 0 | | | | | | |
| 5172 | 06175 | 05171 | 1 | | 0 | 0 | 0 | 0 | 0 | 0 | 0 | 0 | 0 | 0 | 0 | 0 | 0 | 0 | 0 | 1 | 0 | 0 | 0 | 0 | 0 | 0 | 0 | 0 | 0 | | | | | | |
| 5173 | 06176 | 05172 | 3 | | 0 | 0 | 0 | 0 | 0 | 0 | 0 | 0 | 0 | 0 | 2 | 0 | 0 | 0 | 0 | 1 | 0 | 0 | 0 | 0 | 0 | 0 | 0 | 0 | 0 | | | | | | |
| 5174 | 06177 | 05173 | 1 | | 0 | 0 | 0 | 0 | 0 | 0 | 0 | 0 | 0 | 0 | 0 | 0 | 0 | 0 | 0 | 1 | 0 | 0 | 0 | 0 | 0 | 0 | 0 | 0 | 0 | | | | | | |
| 5175 | 06179 | 05174 | 1 | | 0 | 0 | 0 | 0 | 0 | 0 | 0 | 0 | 0 | 0 | 0 | 0 | 0 | 0 | 0 | 1 | 0 | 0 | 0 | 0 | 0 | 0 | 0 | 0 | 0 | | | | | | |
| 5176 | 06180 | 05175 | 1 | | 0 | 0 | 0 | 0 | 0 | 0 | 0 | 0 | 0 | 0 | 0 | 0 | 0 | 0 | 0 | 1 | 0 | 0 | 0 | 0 | 0 | 0 | 0 | 0 | 0 | | | | | | |
| 5177 | 06181 | 05176 | 2 | | 0 | 0 | 0 | 0 | 0 | 0 | 0 | 0 | 0 | 0 | 0 | 0 | 0 | 0 | 0 | 2 | 0 | 0 | 0 | 0 | 0 | 0 | 0 | 0 | 0 | | | | | | |
| 5178 | 06182 | 05177 | 1 | | 0 | 0 | 0 | 0 | 0 | 0 | 0 | 0 | 0 | 0 | 0 | 0 | 0 | 0 | 0 | 1 | 0 | 0 | 0 | 0 | 0 | 0 | 0 | 0 | 0 | | | | | | |
| 5179 | 06183 | 05178 | 1 | | 0 | 0 | 0 | 0 | 0 | 0 | 0 | 0 | 0 | 0 | 0 | 0 | 0 | 0 | 0 | 1 | 0 | 0 | 0 | 0 | 0 | 0 | 0 | 0 | 0 | | | | | | |
| 5180 | 06184 | 05179 | 1 | | 0 | 0 | 0 | 0 | 0 | 0 | 0 | 0 | 0 | 0 | 0 | 0 | 0 | 0 | 0 | 1 | 0 | 0 | 0 | 0 | 0 | 0 | 0 | 0 | 0 | | | | | | |
| 5181 | 06185 | 05180 | 1 | | 0 | 0 | 0 | 0 | 0 | 0 | 0 | 0 | 0 | 0 | 0 | 0 | 0 | 0 | 0 | 1 | 0 | 0 | 0 | 0 | 0 | 0 | 0 | 0 | 0 | | | | | | |
| 5182 | 06186 | 05181 | 4 | | 0 | 0 | 0 | 0 | 0 | 0 | 0 | 0 | 0 | 0 | 0 | 0 | 0 | 0 | 0 | 1 | 0 | 0 | 0 | 1 | 0 | 0 | 0 | 2 | 0 | | | | | | |
| 5183 | 06187 | 05182 | 1 | | 0 | 0 | 0 | 0 | 0 | 0 | 0 | 0 | 0 | 0 | 0 | 0 | 0 | 0 | 0 | 1 | 0 | 0 | 0 | 0 | 0 | 0 | 0 | 0 | 0 | | | | | | |
| 5184 | 06188 | 05183 | 1 | | 0 | 0 | 0 | 0 | 0 | 0 | 0 | 0 | 0 | 0 | 0 | 0 | 0 | 0 | 0 | 1 | 0 | 0 | 0 | 0 | 0 | 0 | 0 | 0 | 0 | | | | | | |
| 5185 | 06189 | 05184 | 1 | | 0 | 0 | 0 | 0 | 0 | 0 | 0 | 0 | 0 | 0 | 0 | 0 | 0 | 0 | 0 | 1 | 0 | 0 | 0 | 0 | 0 | 0 | 0 | 0 | 0 | | | | | | |
| 5186 | 06190 | 05185 | 1 | | 0 | 0 | 0 | 0 | 0 | 0 | 0 | 0 | 0 | 0 | 0 | 0 | 0 | 0 | 0 | 1 | 0 | 0 | 0 | 0 | 0 | 0 | 0 | 0 | 0 | | | | | | |
| 5187 | 06191 | 05186 | 1 | | 0 | 0 | 0 | 0 | 0 | 0 | 0 | 0 | 0 | 0 | 0 | 0 | 0 | 0 | 0 | 1 | 0 | 0 | 0 | 0 | 0 | 0 | 0 | 0 | 0 | | | | | | |
| 5188 | 06192 | 05187 | 1 | | 0 | 0 | 0 | 0 | 0 | 0 | 0 | 0 | 0 | 0 | 0 | 0 | 0 | 0 | 0 | 1 | 0 | 0 | 0 | 0 | 0 | 0 | 0 | 0 | 0 | | | | | | |
| 5189 | 06195 | 05188 | 1 | | 0 | 0 | 0 | 0 | 0 | 0 | 0 | 0 | 0 | 0 | 0 | 0 | 0 | 0 | 0 | 1 | 0 | 0 | 0 | 0 | 0 | 0 | 0 | 0 | 0 | Z23064 | 93.8 | 224 | 1 | 1152 | 1894 |
| 5190 | 06196 | 05189 | 1 | | 0 | 0 | 0 | 0 | 0 | 0 | 0 | 0 | 0 | 0 | 0 | 0 | 0 | 0 | 0 | 1 | 0 | 0 | 0 | 0 | 0 | 0 | 0 | 0 | 0 | | | | | | |
| 5191 | 06197 | 05190 | 2 | | 0 | 0 | 0 | 0 | 0 | 0 | 0 | 0 | 0 | 0 | 0 | 0 | 0 | 0 | 0 | 2 | 0 | 0 | 0 | 0 | 0 | 0 | 0 | 0 | 0 | | | | | | |
| 5192 | 06198 | 05191 | 1 | | 0 | 0 | 0 | 0 | 0 | 0 | 0 | 0 | 0 | 0 | 0 | 0 | 0 | 0 | 0 | 1 | 0 | 0 | 0 | 0 | 0 | 0 | 0 | 0 | 0 | | | | | | |
| 5193 | 06199 | 05192 | 1 | | 0 | 0 | 0 | 0 | 0 | 0 | 0 | 0 | 0 | 0 | 0 | 0 | 0 | 0 | 0 | 1 | 0 | 0 | 0 | 0 | 0 | 0 | 0 | 0 | 0 | | | | | | |
| 5194 | 06200 | 05193 | 1 | | 0 | 0 | 0 | 0 | 0 | 0 | 0 | 0 | 0 | 0 | 0 | 0 | 0 | 0 | 0 | 1 | 0 | 0 | 0 | 0 | 0 | 0 | 0 | 0 | 0 | | | | | | |
| 5195 | 06201 | 05194 | 1 | | 0 | 0 | 0 | 0 | 0 | 0 | 0 | 0 | 0 | 0 | 0 | 0 | 0 | 0 | 0 | 1 | 0 | 0 | 0 | 0 | 0 | 0 | 0 | 0 | 0 | | | | | | |
| 5196 | 06202 | 05195 | 1 | | 0 | 0 | 0 | 0 | 0 | 0 | 0 | 0 | 0 | 0 | 0 | 0 | 0 | 0 | 0 | 1 | 0 | 0 | 0 | 0 | 0 | 0 | 0 | 0 | 0 | | | | | | |
| 5197 | 06203 | 05196 | 3 | | 0 | 0 | 0 | 0 | 0 | 0 | 0 | 0 | 0 | 0 | 0 | 0 | 0 | 0 | 0 | 1 | 0 | 0 | 0 | 0 | 1 | 0 | 0 | 1 | 0 | | | | | | |
| 5198 | 06204 | 05197 | 3 | | 0 | 0 | 0 | 0 | 0 | 0 | 0 | 0 | 0 | 0 | 0 | 0 | 0 | 0 | 0 | 1 | 0 | 0 | 1 | 1 | 0 | 0 | 0 | 0 | 0 | | | | | | |
| 5199 | 06205 | 05198 | 3 | | 0 | 0 | 0 | 0 | 0 | 0 | 0 | 0 | 0 | 0 | 0 | 0 | 0 | 0 | 0 | 1 | 0 | 0 | 0 | 0 | 0 | 0 | 0 | 0 | 2 | | | | | | |
| 5200 | 06206 | 05199 | 1 | | 0 | 0 | 0 | 0 | 0 | 0 | 0 | 0 | 0 | 0 | 0 | 0 | 0 | 0 | 0 | 1 | 0 | 0 | 0 | 0 | 0 | 0 | 0 | 0 | 0 | | | | | | |
| 5201 | 06207 | 05200 | 1 | | 0 | 0 | 0 | 0 | 0 | 0 | 0 | 0 | 0 | 0 | 0 | 0 | 0 | 0 | 0 | 1 | 0 | 0 | 0 | 0 | 0 | 0 | 0 | 0 | 0 | | | | | | |
| 5202 | 06208 | 05201 | 1 | | 0 | 0 | 0 | 0 | 0 | 0 | 0 | 0 | 0 | 0 | 0 | 0 | 0 | 0 | 0 | 1 | 0 | 0 | 0 | 0 | 0 | 0 | 0 | 0 | 0 | | | | | | |
| 5203 | 06209 | 05202 | 2 | | 0 | 0 | 0 | 0 | 0 | 0 | 0 | 0 | 0 | 0 | 0 | 0 | 0 | 0 | 0 | 2 | 0 | 0 | 0 | 0 | 0 | 0 | 0 | 0 | 0 | | | | | | |
| 5204 | 06210 | 05203 | 1 | | 0 | 0 | 0 | 0 | 0 | 0 | 0 | 0 | 0 | 0 | 0 | 0 | 0 | 0 | 0 | 1 | 0 | 0 | 0 | 0 | 0 | 0 | 0 | 0 | 0 | | | | | | |
| 5205 | 06211 | 05204 | 1 | | 0 | 0 | 0 | 0 | 0 | 0 | 0 | 0 | 0 | 0 | 0 | 0 | 0 | 0 | 0 | 1 | 0 | 0 | 0 | 0 | 0 | 0 | 0 | 0 | 0 | | | | | | |

Table 145

| | A | B | C | E | G | I | K | M | O | Q | S | U | W | Y | AA | AC | AE | AG | AI | AK | AM | AO | AQ | AS | AU | AW | AY | BA | BC | BE | BF | BG | BH | BI | BK |
|---|---|---|---|---|---|---|---|---|---|---|---|---|---|---|---|---|---|---|---|---|---|---|---|---|---|---|---|---|---|---|---|---|---|---|---|
| 5206 | 06213 | 05205 | 1 | | 0 | 0 | 0 | 0 | 0 | 0 | 0 | 0 | 0 | 0 | 0 | 0 | 0 | 0 | 0 | 1 | 0 | 0 | 0 | 0 | 0 | 0 | 0 | 0 | 0 | | | | | | |
| 5207 | 06215 | 05206 | 1 | | 0 | 0 | 0 | 0 | 0 | 0 | 0 | 0 | 0 | 0 | 0 | 0 | 0 | 0 | 0 | 1 | 0 | 0 | 0 | 0 | 0 | 0 | 0 | 0 | 0 | | | | | | |
| 5208 | 06216 | 05207 | 1 | | 0 | 0 | 0 | 0 | 0 | 0 | 0 | 0 | 0 | 0 | 0 | 0 | 0 | 0 | 0 | 1 | 0 | 0 | 0 | 0 | 0 | 0 | 0 | 0 | 0 | | | | | | |
| 5209 | 06217 | 05208 | 1 | | 0 | 0 | 0 | 0 | 0 | 0 | 0 | 0 | 0 | 0 | 0 | 0 | 0 | 0 | 0 | 1 | 0 | 0 | 0 | 0 | 0 | 0 | 0 | 0 | 0 | | | | | | |
| 5210 | 06218 | 05209 | 1 | | 0 | 0 | 0 | 0 | 0 | 0 | 0 | 0 | 0 | 0 | 0 | 0 | 0 | 0 | 0 | 1 | 0 | 0 | 0 | 0 | 0 | 0 | 0 | 0 | 0 | | | | | | |
| 5211 | 06219 | 05210 | 1 | | 0 | 0 | 0 | 0 | 0 | 0 | 0 | 0 | 0 | 0 | 0 | 0 | 0 | 0 | 0 | 1 | 0 | 0 | 0 | 0 | 0 | 0 | 0 | 0 | 0 | | | | | | |
| 5212 | 06220 | 05211 | 2 | | 0 | 0 | 0 | 0 | 0 | 0 | 0 | 0 | 0 | 0 | 0 | 0 | 0 | 0 | 0 | 1 | 0 | 1 | 0 | 0 | 0 | 0 | 0 | 0 | 0 | | | | | | |
| 5213 | 06221 | 05212 | 1 | | 0 | 0 | 0 | 0 | 0 | 0 | 0 | 0 | 0 | 0 | 0 | 0 | 0 | 0 | 0 | 1 | 0 | 0 | 0 | 0 | 0 | 0 | 0 | 0 | 0 | | | | | | |
| 5214 | 06222 | 05213 | 2 | | 0 | 0 | 0 | 0 | 0 | 0 | 0 | 0 | 0 | 0 | 0 | 0 | 0 | 0 | 0 | 1 | 0 | 0 | 0 | 1 | 0 | 0 | 0 | 0 | 0 | | | | | | |
| 5215 | 06224 | 05214 | 2 | | 0 | 0 | 0 | 0 | 0 | 0 | 0 | 0 | 0 | 0 | 0 | 0 | 0 | 0 | 0 | 1 | 0 | 1 | 0 | 0 | 0 | 0 | 0 | 0 | 0 | | | | | | |
| 5216 | 06225 | 05215 | 1 | | 0 | 0 | 0 | 0 | 0 | 0 | 0 | 0 | 0 | 0 | 0 | 0 | 0 | 0 | 0 | 1 | 0 | 0 | 0 | 0 | 0 | 0 | 0 | 0 | 0 | | | | | | |
| 5217 | 06226 | 05216 | 1 | | 0 | 0 | 0 | 0 | 0 | 0 | 0 | 0 | 0 | 0 | 0 | 0 | 0 | 0 | 0 | 1 | 0 | 0 | 0 | 0 | 0 | 0 | 0 | 0 | 0 | | | | | | |
| 5218 | 06227 | 05217 | 1 | | 0 | 0 | 0 | 0 | 0 | 0 | 0 | 0 | 0 | 0 | 0 | 0 | 0 | 0 | 0 | 1 | 0 | 0 | 0 | 0 | 0 | 0 | 0 | 0 | 0 | | | | | | |
| 5219 | 06228 | 05218 | 1 | | 0 | 0 | 0 | 0 | 0 | 0 | 0 | 0 | 0 | 0 | 0 | 0 | 0 | 0 | 0 | 1 | 0 | 0 | 0 | 0 | 0 | 0 | 0 | 0 | 0 | | | | | | |
| 5220 | 06229 | 05219 | 1 | | 0 | 0 | 0 | 0 | 0 | 0 | 0 | 0 | 0 | 0 | 0 | 0 | 0 | 0 | 0 | 1 | 0 | 0 | 0 | 0 | 0 | 0 | 0 | 0 | 0 | | | | | | |
| 5221 | 06230 | 05220 | 1 | | 0 | 0 | 0 | 0 | 0 | 0 | 0 | 0 | 0 | 0 | 0 | 0 | 0 | 0 | 0 | 1 | 0 | 0 | 0 | 0 | 0 | 0 | 0 | 0 | 0 | | | | | | |
| 5222 | 06232 | 05221 | 1 | | 0 | 0 | 0 | 0 | 0 | 0 | 0 | 0 | 0 | 0 | 0 | 0 | 0 | 0 | 0 | 1 | 0 | 0 | 0 | 0 | 0 | 0 | 0 | 0 | 0 | | | | | | |
| 5223 | 06233 | 05222 | 1 | | 0 | 0 | 0 | 0 | 0 | 0 | 0 | 0 | 0 | 0 | 0 | 0 | 0 | 0 | 0 | 1 | 0 | 0 | 0 | 0 | 0 | 0 | 0 | 0 | 0 | | | | | | |
| 5224 | 06234 | 05223 | 1 | | 0 | 0 | 0 | 0 | 0 | 0 | 0 | 0 | 0 | 0 | 0 | 0 | 0 | 0 | 0 | 1 | 0 | 0 | 0 | 0 | 0 | 0 | 0 | 0 | 0 | | | | | | |
| 5225 | 06235 | 05224 | 1 | | 0 | 0 | 0 | 0 | 0 | 0 | 0 | 0 | 0 | 0 | 0 | 0 | 0 | 0 | 0 | 1 | 0 | 0 | 0 | 0 | 0 | 0 | 0 | 0 | 0 | | | | | | |
| 5226 | 06236 | 05225 | 1 | | 0 | 0 | 0 | 0 | 0 | 0 | 0 | 0 | 0 | 0 | 0 | 0 | 0 | 0 | 0 | 1 | 0 | 0 | 0 | 0 | 0 | 0 | 0 | 0 | 0 | | | | | | |
| 5227 | 06237 | 05226 | 1 | | 0 | 0 | 0 | 0 | 0 | 0 | 0 | 0 | 0 | 0 | 0 | 0 | 0 | 0 | 0 | 1 | 0 | 0 | 0 | 0 | 0 | 0 | 0 | 0 | 0 | | | | | | |
| 5228 | 06238 | 05227 | 3 | | 0 | 0 | 0 | 0 | 0 | 0 | 0 | 0 | 0 | 0 | 0 | 0 | 0 | 0 | 0 | 1 | 0 | 0 | 0 | 0 | 0 | 0 | 2 | 0 | 0 | | | | | | |
| 5229 | 06240 | 05228 | 1 | | 0 | 0 | 0 | 0 | 0 | 0 | 0 | 0 | 0 | 0 | 0 | 0 | 0 | 0 | 0 | 1 | 0 | 0 | 0 | 0 | 0 | 0 | 0 | 0 | 0 | | | | | | |
| 5230 | 06241 | 05229 | 1 | | 0 | 0 | 0 | 0 | 0 | 0 | 0 | 0 | 0 | 0 | 0 | 0 | 0 | 0 | 0 | 1 | 0 | 0 | 0 | 0 | 0 | 0 | 0 | 0 | 0 | | | | | | |
| 5231 | 06243 | 05230 | 1 | | 0 | 0 | 0 | 0 | 0 | 0 | 0 | 0 | 0 | 0 | 0 | 0 | 0 | 0 | 0 | 1 | 0 | 0 | 0 | 0 | 0 | 0 | 0 | 0 | 0 | | | | | | |
| 5232 | 06244 | 05231 | 1 | | 0 | 0 | 0 | 0 | 0 | 0 | 0 | 0 | 0 | 0 | 0 | 0 | 0 | 0 | 0 | 1 | 0 | 0 | 0 | 0 | 0 | 0 | 0 | 0 | 0 | | | | | | |
| 5233 | 06246 | 05232 | 2 | | 0 | 0 | 0 | 0 | 0 | 0 | 0 | 0 | 0 | 0 | 0 | 0 | 0 | 0 | 0 | 1 | 0 | 1 | 0 | 0 | 0 | 0 | 0 | 0 | 0 | | | | | | |
| 5234 | 06247 | 05233 | 2 | | 0 | 0 | 0 | 0 | 0 | 0 | 0 | 0 | 0 | 0 | 0 | 0 | 0 | 0 | 0 | 1 | 0 | 0 | 0 | 0 | 0 | 0 | 0 | 1 | 0 | | | | | | |
| 5235 | 06248 | 05234 | 1 | | 0 | 0 | 0 | 0 | 0 | 0 | 0 | 0 | 0 | 0 | 0 | 0 | 0 | 0 | 0 | 1 | 0 | 0 | 0 | 0 | 0 | 0 | 0 | 0 | 0 | | | | | | |
| 5236 | 06249 | 05235 | 2 | | 0 | 0 | 0 | 0 | 0 | 0 | 0 | 0 | 0 | 0 | 0 | 0 | 0 | 0 | 0 | 1 | 0 | 0 | 0 | 0 | 1 | 0 | 0 | 0 | 0 | | | | | | |
| 5237 | 06250 | 05236 | 1 | | 0 | 0 | 0 | 0 | 0 | 0 | 0 | 0 | 0 | 0 | 0 | 0 | 0 | 0 | 0 | 1 | 0 | 0 | 0 | 0 | 0 | 0 | 0 | 0 | 0 | | | | | | |
| 5238 | 06251 | 05237 | 1 | | 0 | 0 | 0 | 0 | 0 | 0 | 0 | 0 | 0 | 0 | 0 | 0 | 0 | 0 | 0 | 1 | 0 | 0 | 0 | 0 | 0 | 0 | 0 | 0 | 0 | | | | | | |
| 5239 | 06253 | 05238 | 1 | | 0 | 0 | 0 | 0 | 0 | 0 | 0 | 0 | 0 | 0 | 0 | 0 | 0 | 0 | 0 | 1 | 0 | 0 | 0 | 0 | 0 | 0 | 0 | 0 | 0 | | | | | | |
| 5240 | 06254 | 05239 | 1 | | 0 | 0 | 0 | 0 | 0 | 0 | 0 | 0 | 0 | 0 | 0 | 0 | 0 | 0 | 0 | 1 | 0 | 0 | 0 | 0 | 0 | 0 | 0 | 0 | 0 | | | | | | |
| 5241 | 06255 | 05240 | 1 | | 0 | 0 | 0 | 0 | 0 | 0 | 0 | 0 | 0 | 0 | 0 | 0 | 0 | 0 | 0 | 1 | 0 | 0 | 0 | 0 | 0 | 0 | 0 | 0 | 0 | | | | | | |

Table 146

| | A | B | C | E | G | I | K | M | O | Q | S | U | W | Y | AA | AC | AE | AG | AI | AK | AM | AO | AQ | AS | AU | AW | AY | BA | BC | BE | BF | BG | BH | BI | BK |
|---|---|---|---|---|---|---|---|---|---|---|---|---|---|---|---|---|---|---|---|---|---|---|---|---|---|---|---|---|---|---|---|---|---|---|---|
| 5242 | 06256 | 05241 | 1 | | 0 | 0 | 0 | 0 | 0 | 0 | 0 | 0 | 0 | 0 | 0 | 0 | 0 | 0 | 0 | 1 | 0 | 0 | 0 | 0 | 0 | 0 | 0 | 0 | 0 | | | | | | |
| 5243 | 06257 | 05242 | 5 | | 0 | 0 | 0 | 0 | 0 | 0 | 0 | 0 | 0 | 0 | 0 | 0 | 0 | 0 | 0 | 1 | 0 | 0 | 0 | 1 | 1 | 0 | 2 | 0 | 0 | | | | | | |
| 5244 | 06258 | 05243 | 1 | | 0 | 0 | 0 | 0 | 0 | 0 | 0 | 0 | 0 | 0 | 0 | 0 | 0 | 0 | 0 | 1 | 0 | 0 | 0 | 0 | 0 | 0 | 0 | 0 | 0 | | | | | | |
| 5245 | 06259 | 05244 | 3 | | 0 | 0 | 0 | 0 | 0 | 0 | 0 | 0 | 0 | 0 | 0 | 0 | 0 | 0 | 0 | 1 | 0 | 0 | 0 | 0 | 0 | 0 | 2 | 0 | 0 | X52056 | 98.5 | 68 | 1 | 1297 | 1364 |
| 5246 | 06260 | 05245 | 1 | | 0 | 0 | 0 | 0 | 0 | 0 | 0 | 0 | 0 | 0 | 0 | 0 | 0 | 0 | 0 | 1 | 0 | 0 | 0 | 0 | 0 | 0 | 0 | 0 | 0 | | | | | | |
| 5247 | 06261 | 05246 | 2 | | 0 | 0 | 0 | 0 | 0 | 0 | 0 | 0 | 0 | 0 | 0 | 0 | 0 | 0 | 0 | 1 | 0 | 0 | 0 | 1 | 0 | 0 | 0 | 0 | 0 | | | | | | |
| 5248 | 06262 | 05247 | 1 | | 0 | 0 | 0 | 0 | 0 | 0 | 0 | 0 | 0 | 0 | 0 | 0 | 0 | 0 | 0 | 1 | 0 | 0 | 0 | 0 | 0 | 0 | 0 | 0 | 0 | | | | | | |
| 5249 | 06264 | 05248 | 1 | | 0 | 0 | 0 | 0 | 0 | 0 | 0 | 0 | 0 | 0 | 0 | 0 | 0 | 0 | 0 | 1 | 0 | 0 | 0 | 0 | 0 | 0 | 0 | 0 | 0 | | | | | | |
| 5250 | 06267 | 05249 | 1 | | 0 | 0 | 0 | 0 | 0 | 0 | 0 | 0 | 0 | 0 | 0 | 0 | 0 | 0 | 0 | 1 | 0 | 0 | 0 | 0 | 0 | 0 | 0 | 0 | 0 | | | | | | |
| 5251 | 06268 | 05250 | 1 | | 0 | 0 | 0 | 0 | 0 | 0 | 0 | 0 | 0 | 0 | 0 | 0 | 0 | 0 | 0 | 1 | 0 | 0 | 0 | 0 | 0 | 0 | 0 | 0 | 0 | | | | | | |
| 5252 | 06269 | 05251 | 1 | | 0 | 0 | 0 | 0 | 0 | 0 | 0 | 0 | 0 | 0 | 0 | 0 | 0 | 0 | 0 | 1 | 0 | 0 | 0 | 0 | 0 | 0 | 0 | 0 | 0 | | | | | | |
| 5253 | 06270 | 05252 | 1 | | 0 | 0 | 0 | 0 | 0 | 0 | 0 | 0 | 0 | 0 | 0 | 0 | 0 | 0 | 0 | 1 | 0 | 0 | 0 | 0 | 0 | 0 | 0 | 0 | 0 | | | | | | |
| 5254 | 06271 | 05253 | 1 | | 0 | 0 | 0 | 0 | 0 | 0 | 0 | 0 | 0 | 0 | 0 | 0 | 0 | 0 | 0 | 1 | 0 | 0 | 0 | 0 | 0 | 0 | 0 | 0 | 0 | | | | | | |
| 5255 | 06272 | 05254 | 1 | | 0 | 0 | 0 | 0 | 0 | 0 | 0 | 0 | 0 | 0 | 0 | 0 | 0 | 0 | 0 | 1 | 0 | 0 | 0 | 0 | 0 | 0 | 0 | 0 | 0 | | | | | | |
| 5256 | 06273 | 05255 | 1 | | 0 | 0 | 0 | 0 | 0 | 0 | 0 | 0 | 0 | 0 | 0 | 0 | 0 | 0 | 0 | 1 | 0 | 0 | 0 | 0 | 0 | 0 | 0 | 0 | 0 | M80359 | 93.9 | 181 | 1 | 2062 | 2914 |
| 5257 | 06274 | 05256 | 1 | | 0 | 0 | 0 | 0 | 0 | 0 | 0 | 0 | 0 | 0 | 0 | 0 | 0 | 0 | 0 | 1 | 0 | 0 | 0 | 0 | 0 | 0 | 0 | 0 | 0 | | | | | | |
| 5258 | 06275 | 05257 | 5 | | 0 | 0 | 0 | 0 | 0 | 0 | 0 | 0 | 0 | 0 | 0 | 0 | 0 | 0 | 0 | 1 | 0 | 1 | 0 | 1 | 0 | 2 | 0 | 0 | 0 | M90104 | 94.2 | 173 | 1 | 1700 | 1879 |
| 5259 | 06277 | 05258 | 1 | | 0 | 0 | 0 | 0 | 0 | 0 | 0 | 0 | 0 | 0 | 0 | 0 | 0 | 0 | 0 | 1 | 0 | 0 | 0 | 0 | 0 | 0 | 0 | 0 | 0 | | | | | | |
| 5260 | 06278 | 05259 | 1 | | 0 | 0 | 0 | 0 | 0 | 0 | 0 | 0 | 0 | 0 | 0 | 0 | 0 | 0 | 0 | 1 | 0 | 0 | 0 | 0 | 0 | 0 | 0 | 0 | 0 | | | | | | |
| 5261 | 06279 | 05260 | 1 | | 0 | 0 | 0 | 0 | 0 | 0 | 0 | 0 | 0 | 0 | 0 | 0 | 0 | 0 | 0 | 1 | 0 | 0 | 0 | 0 | 0 | 0 | 0 | 0 | 0 | | | | | | |
| 5262 | 06280 | 05261 | 1 | | 0 | 0 | 0 | 0 | 0 | 0 | 0 | 0 | 0 | 0 | 0 | 0 | 0 | 0 | 0 | 1 | 0 | 0 | 0 | 0 | 0 | 0 | 0 | 0 | 0 | | | | | | |
| 5263 | 06281 | 05262 | 1 | | 0 | 0 | 0 | 0 | 0 | 0 | 0 | 0 | 0 | 0 | 0 | 0 | 0 | 0 | 0 | 1 | 0 | 0 | 0 | 0 | 0 | 0 | 0 | 0 | 0 | M64784 | 95.6 | 338 | 1 | 638 | 1138 |
| 5264 | 06282 | 05263 | 1 | | 0 | 0 | 0 | 0 | 0 | 0 | 0 | 0 | 0 | 0 | 0 | 0 | 0 | 0 | 0 | 1 | 0 | 0 | 0 | 0 | 0 | 0 | 0 | 0 | 0 | | | | | | |
| 5265 | 06283 | 05264 | 11 | | 0 | 0 | 0 | 0 | 0 | 0 | 0 | 0 | 0 | 0 | 0 | 0 | 0 | 0 | 0 | 1 | 0 | 0 | 0 | 0 | 0 | 1 | 0 | 0 | 9 | X52426 | 100 | 102 | 1 | 1574 | 1677 |
| 5266 | 06284 | 05265 | 2 | | 0 | 0 | 0 | 0 | 0 | 0 | 0 | 0 | 0 | 0 | 0 | 0 | 0 | 0 | 0 | 1 | 0 | 0 | 0 | 1 | 0 | 0 | 0 | 0 | 0 | | | | | | |
| 5267 | 06286 | 05266 | 1 | | 0 | 0 | 0 | 0 | 0 | 0 | 0 | 0 | 0 | 0 | 0 | 0 | 0 | 0 | 0 | 1 | 0 | 0 | 0 | 0 | 0 | 0 | 0 | 0 | 0 | | | | | | |
| 5268 | 06288 | 05267 | 2 | | 0 | 0 | 0 | 0 | 0 | 0 | 0 | 0 | 0 | 0 | 0 | 0 | 0 | 0 | 0 | 1 | 0 | 0 | 0 | 0 | 0 | 1 | 0 | 0 | 0 | | | | | | |
| 5269 | 06289 | 05268 | 1 | | 0 | 0 | 0 | 0 | 0 | 0 | 0 | 0 | 0 | 0 | 0 | 0 | 0 | 0 | 0 | 1 | 0 | 0 | 0 | 0 | 0 | 0 | 0 | 0 | 0 | | | | | | |
| 5270 | 06290 | 05269 | 2 | | 0 | 0 | 0 | 0 | 0 | 0 | 0 | 0 | 0 | 0 | 0 | 0 | 0 | 0 | 0 | 1 | 0 | 0 | 0 | 0 | 0 | 0 | 1 | 0 | 0 | | | | | | |
| 5271 | 06291 | 05270 | 1 | | 0 | 0 | 0 | 0 | 0 | 0 | 0 | 0 | 0 | 0 | 0 | 0 | 0 | 0 | 0 | 1 | 0 | 0 | 0 | 0 | 0 | 0 | 0 | 0 | 0 | | | | | | |
| 5272 | 06292 | 05271 | 1 | | 0 | 0 | 0 | 0 | 0 | 0 | 0 | 0 | 0 | 0 | 0 | 0 | 0 | 0 | 0 | 1 | 0 | 0 | 0 | 0 | 0 | 0 | 0 | 0 | 0 | | | | | | |
| 5273 | 06293 | 05272 | 1 | | 0 | 0 | 0 | 0 | 0 | 0 | 0 | 0 | 0 | 0 | 0 | 0 | 0 | 0 | 0 | 1 | 0 | 0 | 0 | 0 | 0 | 0 | 0 | 0 | 0 | | | | | | |
| 5274 | 06294 | 05273 | 1 | | 0 | 0 | 0 | 0 | 0 | 0 | 0 | 0 | 0 | 0 | 0 | 0 | 0 | 0 | 0 | 1 | 0 | 0 | 0 | 0 | 0 | 0 | 0 | 0 | 0 | | | | | | |
| 5275 | 06295 | 05274 | 1 | | 0 | 0 | 0 | 0 | 0 | 0 | 0 | 0 | 0 | 0 | 0 | 0 | 0 | 0 | 0 | 1 | 0 | 0 | 0 | 0 | 0 | 0 | 0 | 0 | 0 | | | | | | |
| 5276 | 06296 | 05275 | 1 | | 0 | 0 | 0 | 0 | 0 | 0 | 0 | 0 | 0 | 0 | 0 | 0 | 0 | 0 | 0 | 1 | 0 | 0 | 0 | 0 | 0 | 0 | 0 | 0 | 0 | | | | | | |
| 5277 | 06297 | 05276 | 1 | | 0 | 0 | 0 | 0 | 0 | 0 | 0 | 0 | 0 | 0 | 0 | 0 | 0 | 0 | 0 | 1 | 0 | 0 | 0 | 0 | 0 | 0 | 0 | 0 | 0 | M86400 | 92.4 | 197 | 1 | 1196 | 2834 |

Table 147

| | A | B | C | E | G | I | K | M | O | Q | S | U | W | Y | AA | AC | AF | AG | AI | AK | AM | AO | AQ | AS | AU | AW | AY | BA | BC | BE | BF | BG | BH | BI | BK |
|---|---|---|---|---|---|---|---|---|---|---|---|---|---|---|---|---|---|---|---|---|---|---|---|---|---|---|---|---|---|---|---|---|---|---|---|
| 5278 | 06299 | 05277 | 3 | | 0 | 0 | 0 | 0 | 0 | 0 | 0 | 0 | 0 | 0 | 0 | 0 | 0 | 0 | 0 | 1 | 0 | 0 | 0 | 1 | 0 | 0 | 1 | 0 | 0 | M74002 | 98.7 | 229 | 1 | 2508 | 2736 |
| 5279 | 06300 | 05278 | 1 | | 0 | 0 | 0 | 0 | 0 | 0 | 0 | 0 | 0 | 0 | 0 | 0 | 0 | 0 | 0 | 1 | 0 | 0 | 0 | 0 | 0 | 0 | 0 | 0 | 0 | J05448 | 98.9 | 176 | 1 | 1256 | 1766 |
| 5280 | 06301 | 05279 | 2 | | 0 | 0 | 0 | 0 | 0 | 0 | 0 | 0 | 0 | 0 | 0 | 0 | 0 | 0 | 0 | 2 | 0 | 0 | 0 | 0 | 0 | 0 | 0 | 0 | 0 | | | | | | |
| 5281 | 06302 | 05280 | 2 | | 0 | 0 | 0 | 0 | 0 | 0 | 0 | 0 | 0 | 0 | 0 | 0 | 0 | 0 | 0 | 1 | 0 | 1 | 0 | 0 | 0 | 0 | 0 | 0 | 0 | | | | | | |
| 5282 | 06303 | 05281 | 1 | | 0 | 0 | 0 | 0 | 0 | 0 | 0 | 0 | 0 | 0 | 0 | 0 | 0 | 0 | 0 | 1 | 0 | 0 | 0 | 0 | 0 | 0 | 0 | 0 | 0 | | | | | | |
| 5283 | 06304 | 05282 | 1 | | 0 | 0 | 0 | 0 | 0 | 0 | 0 | 0 | 0 | 0 | 0 | 0 | 0 | 0 | 0 | 1 | 0 | 0 | 0 | 0 | 0 | 0 | 0 | 0 | 0 | | | | | | |
| 5284 | 06305 | 05283 | 1 | | 0 | 0 | 0 | 0 | 0 | 0 | 0 | 0 | 0 | 0 | 0 | 0 | 0 | 0 | 0 | 1 | 0 | 0 | 0 | 0 | 0 | 0 | 0 | 0 | 0 | | | | | | |
| 5285 | 06306 | 05284 | 1 | | 0 | 0 | 0 | 0 | 0 | 0 | 0 | 0 | 0 | 0 | 0 | 0 | 0 | 0 | 0 | 1 | 0 | 0 | 0 | 0 | 0 | 0 | 0 | 0 | 0 | | | | | | |
| 5286 | 06307 | 05285 | 2 | | 0 | 0 | 0 | 0 | 0 | 0 | 0 | 0 | 0 | 0 | 0 | 0 | 0 | 0 | 0 | 1 | 0 | 0 | 0 | 0 | 0 | 1 | 0 | 0 | 0 | | | | | | |
| 5287 | 06308 | 05286 | 2 | | 0 | 0 | 0 | 0 | 0 | 0 | 0 | 0 | 0 | 0 | 0 | 0 | 0 | 0 | 0 | 1 | 0 | 0 | 1 | 0 | 0 | 0 | 0 | 0 | 0 | | | | | | |
| 5288 | 06309 | 05287 | 1 | | 0 | 0 | 0 | 0 | 0 | 0 | 0 | 0 | 0 | 0 | 0 | 0 | 0 | 0 | 0 | 1 | 0 | 0 | 0 | 0 | 0 | 0 | 0 | 0 | 0 | | | | | | |
| 5289 | 06310 | 05288 | 3 | | 0 | 0 | 0 | 0 | 0 | 0 | 0 | 0 | 0 | 0 | 0 | 0 | 0 | 0 | 0 | 1 | 0 | 1 | 1 | 0 | 0 | 0 | 0 | 0 | 0 | | | | | | |
| 5290 | 06311 | 05289 | 1 | | 0 | 0 | 0 | 0 | 0 | 0 | 0 | 0 | 0 | 0 | 0 | 0 | 0 | 0 | 0 | 1 | 0 | 0 | 0 | 0 | 0 | 0 | 0 | 0 | 0 | | | | | | |
| 5291 | 06313 | 05290 | 1 | | 0 | 0 | 0 | 0 | 0 | 0 | 0 | 0 | 0 | 0 | 0 | 0 | 0 | 0 | 0 | 1 | 0 | 0 | 0 | 0 | 0 | 0 | 0 | 0 | 0 | | | | | | |
| 5292 | 06314 | 05291 | 1 | | 0 | 0 | 0 | 0 | 0 | 0 | 0 | 0 | 0 | 0 | 0 | 0 | 0 | 0 | 0 | 1 | 0 | 0 | 0 | 0 | 0 | 0 | 0 | 0 | 0 | | | | | | |
| 5293 | 06315 | 05292 | 3 | | 0 | 0 | 0 | 0 | 0 | 0 | 0 | 0 | 0 | 0 | 0 | 0 | 0 | 0 | 0 | 1 | 0 | 0 | 0 | 0 | 0 | 2 | 0 | 0 | 0 | M69039 | 99 | 102 | 1 | 1130 | 1240 |
| 5294 | 06316 | 05293 | 1 | | 0 | 0 | 0 | 0 | 0 | 0 | 0 | 0 | 0 | 0 | 0 | 0 | 0 | 0 | 0 | 1 | 0 | 0 | 0 | 0 | 0 | 0 | 0 | 0 | 0 | | | | | | |
| 5295 | 06317 | 05294 | 1 | | 0 | 0 | 0 | 0 | 0 | 0 | 0 | 0 | 0 | 0 | 0 | 0 | 0 | 0 | 0 | 1 | 0 | 0 | 0 | 0 | 0 | 0 | 0 | 0 | 0 | | | | | | |
| 5296 | 06318 | 05295 | 2 | | 0 | 0 | 0 | 0 | 0 | 0 | 0 | 0 | 0 | 0 | 0 | 0 | 0 | 0 | 0 | 1 | 0 | 0 | 0 | 0 | 1 | 0 | 0 | 0 | 0 | | | | | | |
| 5297 | 06319 | 05296 | 5 | | 0 | 0 | 0 | 0 | 0 | 0 | 0 | 0 | 0 | 0 | 0 | 0 | 0 | 0 | 0 | 1 | 0 | 1 | 0 | 1 | 0 | 1 | 1 | 0 | 0 | M59287 | 99.5 | 198 | 1 | 1549 | 1750 |
| 5298 | 06320 | 05297 | 1 | | 0 | 0 | 0 | 0 | 0 | 0 | 0 | 0 | 0 | 0 | 0 | 0 | 0 | 0 | 0 | 1 | 0 | 0 | 0 | 0 | 0 | 0 | 0 | 0 | 0 | | | | | | |
| 5299 | 06321 | 05298 | 1 | | 0 | 0 | 0 | 0 | 0 | 0 | 0 | 0 | 0 | 0 | 0 | 0 | 0 | 0 | 0 | 1 | 0 | 0 | 0 | 0 | 0 | 0 | 0 | 0 | 0 | | | | | | |
| 5300 | 06322 | 05299 | 1 | | 0 | 0 | 0 | 0 | 0 | 0 | 0 | 0 | 0 | 0 | 0 | 0 | 0 | 0 | 0 | 1 | 0 | 0 | 0 | 0 | 0 | 0 | 0 | 0 | 0 | L22846 | 100 | 69 | 1 | 1698 | 1766 |
| 5301 | 06323 | 05300 | 1 | | 0 | 0 | 0 | 0 | 0 | 0 | 0 | 0 | 0 | 0 | 0 | 0 | 0 | 0 | 0 | 1 | 0 | 0 | 0 | 0 | 0 | 0 | 0 | 0 | 0 | | | | | | |
| 5302 | 06324 | 05301 | 1 | | 0 | 0 | 0 | 0 | 0 | 0 | 0 | 0 | 0 | 0 | 0 | 0 | 0 | 0 | 0 | 1 | 0 | 0 | 0 | 0 | 0 | 0 | 0 | 0 | 0 | | | | | | |
| 5303 | 06325 | 05302 | 1 | | 0 | 0 | 0 | 0 | 0 | 0 | 0 | 0 | 0 | 0 | 0 | 0 | 0 | 0 | 0 | 1 | 0 | 0 | 0 | 0 | 0 | 0 | 0 | 0 | 0 | | | | | | |
| 5304 | 06326 | 05303 | 1 | | 0 | 0 | 0 | 0 | 0 | 0 | 0 | 0 | 0 | 0 | 0 | 0 | 0 | 0 | 0 | 1 | 0 | 0 | 0 | 0 | 0 | 0 | 0 | 0 | 0 | | | | | | |
| 5305 | 06327 | 05304 | 1 | | 0 | 0 | 0 | 0 | 0 | 0 | 0 | 0 | 0 | 0 | 0 | 0 | 0 | 0 | 0 | 1 | 0 | 0 | 0 | 0 | 0 | 0 | 0 | 0 | 0 | | | | | | |
| 5306 | 06329 | 05305 | 1 | | 0 | 0 | 0 | 0 | 0 | 0 | 0 | 0 | 0 | 0 | 0 | 0 | 0 | 0 | 0 | 1 | 0 | 0 | 0 | 0 | 0 | 0 | 0 | 0 | 0 | | | | | | |
| 5307 | 06330 | 05306 | 2 | | 0 | 0 | 0 | 0 | 0 | 0 | 0 | 0 | 0 | 0 | 0 | 0 | 0 | 0 | 0 | 1 | 0 | 1 | 0 | 0 | 0 | 0 | 0 | 0 | 0 | | | | | | |
| 5308 | 06331 | 05307 | 1 | | 0 | 0 | 0 | 0 | 0 | 0 | 0 | 0 | 0 | 0 | 0 | 0 | 0 | 0 | 0 | 1 | 0 | 0 | 0 | 0 | 0 | 0 | 0 | 0 | 0 | | | | | | |
| 5309 | 06332 | 05308 | 1 | | 0 | 0 | 0 | 0 | 0 | 0 | 0 | 0 | 0 | 0 | 0 | 0 | 0 | 0 | 0 | 1 | 0 | 0 | 0 | 0 | 0 | 0 | 0 | 0 | 0 | | | | | | |
| 5310 | 06333 | 05309 | 1 | | 0 | 0 | 0 | 0 | 0 | 0 | 0 | 0 | 0 | 0 | 0 | 0 | 0 | 0 | 0 | 1 | 0 | 0 | 0 | 0 | 0 | 0 | 0 | 0 | 0 | | | | | | |
| 5311 | 06334 | 05310 | 1 | | 0 | 0 | 0 | 0 | 0 | 0 | 0 | 0 | 0 | 0 | 0 | 0 | 0 | 0 | 0 | 1 | 0 | 0 | 0 | 0 | 0 | 0 | 0 | 0 | 0 | | | | | | |
| 5312 | 06335 | 05311 | 1 | | 0 | 0 | 0 | 0 | 0 | 0 | 0 | 0 | 0 | 0 | 0 | 0 | 0 | 0 | 0 | 1 | 0 | 0 | 0 | 0 | 0 | 0 | 0 | 0 | 0 | D13638 | 96 | 124 | 1 | 1571 | 5167 |
| 5313 | 06337 | 05312 | 1 | | 0 | 0 | 0 | 0 | 0 | 0 | 0 | 0 | 0 | 0 | 0 | 0 | 0 | 0 | 0 | 1 | 0 | 0 | 0 | 0 | 0 | 0 | 0 | 0 | 0 | | | | | | |

Table 148

| | A | B | C | E | G | I | K | M | O | Q | S | U | W | Y | AA | AC | AE | AG | AI | AK | AM | AO | AQ | AS | AU | AW | AY | BA | BC | BE | BF | BG | BH | BI | BK |
|---|---|---|---|---|---|---|---|---|---|---|---|---|---|---|---|---|---|---|---|---|---|---|---|---|---|---|---|---|---|---|---|---|---|---|---|
| 5314 | 06338 | 05313 | 1 | | 0 | 0 | 0 | 0 | 0 | 0 | 0 | 0 | 0 | 0 | 0 | 0 | 0 | 0 | 0 | 1 | 0 | 0 | 0 | 0 | 0 | 0 | 0 | 0 | 0 | | | | | | |
| 5315 | 06339 | 05314 | 1 | | 0 | 0 | 0 | 0 | 0 | 0 | 0 | 0 | 0 | 0 | 0 | 0 | 0 | 0 | 0 | 1 | 0 | 0 | 0 | 0 | 0 | 0 | 0 | 0 | 0 | | | | | | |
| 5316 | 06341 | 05315 | 1 | | 0 | 0 | 0 | 0 | 0 | 0 | 0 | 0 | 0 | 0 | 0 | 0 | 0 | 0 | 0 | 1 | 0 | 0 | 0 | 0 | 0 | 0 | 0 | 0 | 0 | | | | | | |
| 5317 | 06343 | 05316 | 1 | | 0 | 0 | 0 | 0 | 0 | 0 | 0 | 0 | 0 | 0 | 0 | 0 | 0 | 0 | 0 | 1 | 0 | 0 | 0 | 0 | 0 | 0 | 0 | 0 | 0 | | | | | | |
| 5318 | 06344 | 05317 | 1 | | 0 | 0 | 0 | 0 | 0 | 0 | 0 | 0 | 0 | 0 | 0 | 0 | 0 | 0 | 0 | 1 | 0 | 0 | 0 | 0 | 0 | 0 | 0 | 0 | 0 | | | | | | |
| 5319 | 06345 | 05318 | 1 | | 0 | 0 | 0 | 0 | 0 | 0 | 0 | 0 | 0 | 0 | 0 | 0 | 0 | 0 | 0 | 1 | 0 | 0 | 0 | 0 | 0 | 0 | 0 | 0 | 0 | | | | | | |
| 5320 | 06346 | 05319 | 1 | | 0 | 0 | 0 | 0 | 0 | 0 | 0 | 0 | 0 | 0 | 0 | 0 | 0 | 0 | 0 | 1 | 0 | 0 | 0 | 0 | 0 | 0 | 0 | 0 | 0 | | | | | | |
| 5321 | 06347 | 05320 | 1 | | 0 | 0 | 0 | 0 | 0 | 0 | 0 | 0 | 0 | 0 | 0 | 0 | 0 | 0 | 0 | 1 | 0 | 0 | 0 | 0 | 0 | 0 | 0 | 0 | 0 | | | | | | |
| 5322 | 06348 | 05321 | 2 | | 0 | 0 | 0 | 0 | 0 | 0 | 0 | 0 | 0 | 0 | 0 | 0 | 0 | 0 | 0 | 1 | 0 | 0 | 0 | 0 | 0 | 0 | 0 | 1 | 0 | | | | | | |
| 5323 | 06350 | 05322 | 3 | | 0 | 0 | 0 | 0 | 0 | 0 | 0 | 0 | 0 | 0 | 0 | 0 | 0 | 0 | 0 | 1 | 0 | 0 | 0 | 0 | 0 | 0 | 0 | 2 | 0 | | | | | | |
| 5324 | 06351 | 05323 | 1 | | 0 | 0 | 0 | 0 | 0 | 0 | 0 | 0 | 0 | 0 | 0 | 0 | 0 | 0 | 0 | 1 | 0 | 0 | 0 | 0 | 0 | 0 | 0 | 0 | 0 | | | | | | |
| 5325 | 06353 | 05324 | 1 | | 0 | 0 | 0 | 0 | 0 | 0 | 0 | 0 | 0 | 0 | 0 | 0 | 0 | 0 | 0 | 1 | 0 | 0 | 0 | 0 | 0 | 0 | 0 | 0 | 0 | | | | | | |
| 5326 | 06354 | 05325 | 1 | | 0 | 0 | 0 | 0 | 0 | 0 | 0 | 0 | 0 | 0 | 0 | 0 | 0 | 0 | 0 | 1 | 0 | 0 | 0 | 0 | 0 | 0 | 0 | 0 | 0 | | | | | | |
| 5327 | 06355 | 05326 | 1 | | 0 | 0 | 0 | 0 | 0 | 0 | 0 | 0 | 0 | 0 | 0 | 0 | 0 | 0 | 0 | 1 | 0 | 0 | 0 | 0 | 0 | 0 | 0 | 0 | 0 | | | | | | |
| 5328 | 06356 | 05327 | 1 | | 0 | 0 | 0 | 0 | 0 | 0 | 0 | 0 | 0 | 0 | 0 | 0 | 0 | 0 | 0 | 1 | 0 | 0 | 0 | 0 | 0 | 0 | 0 | 0 | 0 | | | | | | |
| 5329 | 06357 | 05328 | 1 | | 0 | 0 | 0 | 0 | 0 | 0 | 0 | 0 | 0 | 0 | 0 | 0 | 0 | 0 | 0 | 1 | 0 | 0 | 0 | 0 | 0 | 0 | 0 | 0 | 0 | | | | | | |
| 5330 | 06358 | 05329 | 1 | | 0 | 0 | 0 | 0 | 0 | 0 | 0 | 0 | 0 | 0 | 0 | 0 | 0 | 0 | 0 | 1 | 0 | 0 | 0 | 0 | 0 | 0 | 0 | 0 | 0 | | | | | | |
| 5331 | 06359 | 05330 | 1 | | 0 | 0 | 0 | 0 | 0 | 0 | 0 | 0 | 0 | 0 | 0 | 0 | 0 | 0 | 0 | 1 | 0 | 0 | 0 | 0 | 0 | 0 | 0 | 0 | 0 | | | | | | |
| 5332 | 06360 | 05331 | 1 | | 0 | 0 | 0 | 0 | 0 | 0 | 0 | 0 | 0 | 0 | 0 | 0 | 0 | 0 | 0 | 1 | 0 | 0 | 0 | 0 | 0 | 0 | 0 | 0 | 0 | | | | | | |
| 5333 | 06361 | 05332 | 1 | | 0 | 0 | 0 | 0 | 0 | 0 | 0 | 0 | 0 | 0 | 0 | 0 | 0 | 0 | 0 | 1 | 0 | 0 | 0 | 0 | 0 | 0 | 0 | 0 | 0 | | | | | | |
| 5334 | 06362 | 05333 | 1 | | 0 | 0 | 0 | 0 | 0 | 0 | 0 | 0 | 0 | 0 | 0 | 0 | 0 | 0 | 0 | 1 | 0 | 0 | 0 | 0 | 0 | 0 | 0 | 0 | 0 | | | | | | |
| 5335 | 06363 | 05334 | 1 | | 0 | 0 | 0 | 0 | 0 | 0 | 0 | 0 | 0 | 0 | 0 | 0 | 0 | 0 | 0 | 1 | 0 | 0 | 0 | 0 | 0 | 0 | 0 | 0 | 0 | | | | | | |
| 5336 | 06364 | 05335 | 1 | | 0 | 0 | 0 | 0 | 0 | 0 | 0 | 0 | 0 | 0 | 0 | 0 | 0 | 0 | 0 | 1 | 0 | 0 | 0 | 0 | 0 | 0 | 0 | 0 | 0 | | | | | | |
| 5337 | 06365 | 05336 | 1 | | 0 | 0 | 0 | 0 | 0 | 0 | 0 | 0 | 0 | 0 | 0 | 0 | 0 | 0 | 0 | 1 | 0 | 0 | 0 | 0 | 0 | 0 | 0 | 0 | 0 | X02994 | 96.8 | 217 | 1 | 1282 | 1498 |
| 5338 | 06366 | 05337 | 1 | | 0 | 0 | 0 | 0 | 0 | 0 | 0 | 0 | 0 | 0 | 0 | 0 | 0 | 0 | 0 | 1 | 0 | 0 | 0 | 0 | 0 | 0 | 0 | 0 | 0 | | | | | | |
| 5339 | 06367 | 05338 | 5 | | 0 | 0 | 0 | 0 | 0 | 0 | 0 | 0 | 0 | 0 | 0 | 0 | 0 | 0 | 0 | 1 | 0 | 0 | 3 | 1 | 0 | 0 | 0 | 0 | 0 | | | | | | |
| 5340 | 06369 | 05339 | 1 | | 0 | 0 | 0 | 0 | 0 | 0 | 0 | 0 | 0 | 0 | 0 | 0 | 0 | 0 | 0 | 1 | 0 | 0 | 0 | 0 | 0 | 0 | 0 | 0 | 0 | | | | | | |
| 5341 | 06370 | 05340 | 1 | | 0 | 0 | 0 | 0 | 0 | 0 | 0 | 0 | 0 | 0 | 0 | 0 | 0 | 0 | 0 | 1 | 0 | 0 | 0 | 0 | 0 | 0 | 0 | 0 | 0 | | | | | | |
| 5342 | 06371 | 05341 | 1 | | 0 | 0 | 0 | 0 | 0 | 0 | 0 | 0 | 0 | 0 | 0 | 0 | 0 | 0 | 0 | 1 | 0 | 0 | 0 | 0 | 0 | 0 | 0 | 0 | 0 | | | | | | |
| 5343 | 06372 | 05342 | 1 | | 0 | 0 | 0 | 0 | 0 | 0 | 0 | 0 | 0 | 0 | 0 | 0 | 0 | 0 | 0 | 1 | 0 | 0 | 0 | 0 | 0 | 0 | 0 | 0 | 0 | | | | | | |
| 5344 | 06373 | 05343 | 1 | | 0 | 0 | 0 | 0 | 0 | 0 | 0 | 0 | 0 | 0 | 0 | 0 | 0 | 0 | 0 | 1 | 0 | 0 | 0 | 0 | 0 | 0 | 0 | 0 | 0 | | | | | | |
| 5345 | 06374 | 05344 | 1 | | 0 | 0 | 0 | 0 | 0 | 0 | 0 | 0 | 0 | 0 | 0 | 0 | 0 | 0 | 0 | 1 | 0 | 0 | 0 | 0 | 0 | 0 | 0 | 0 | 0 | | | | | | |
| 5346 | 06376 | 05345 | 1 | | 0 | 0 | 0 | 0 | 0 | 0 | 0 | 0 | 0 | 0 | 0 | 0 | 0 | 0 | 0 | 1 | 0 | 0 | 0 | 0 | 0 | 0 | 0 | 0 | 0 | | | | | | |
| 5347 | 06377 | 05346 | 1 | | 0 | 0 | 0 | 0 | 0 | 0 | 0 | 0 | 0 | 0 | 0 | 0 | 0 | 0 | 0 | 1 | 0 | 0 | 0 | 0 | 0 | 0 | 0 | 0 | 0 | | | | | | |
| 5348 | 06378 | 05347 | 1 | | 0 | 0 | 0 | 0 | 0 | 0 | 0 | 0 | 0 | 0 | 0 | 0 | 0 | 0 | 0 | 1 | 0 | 0 | 0 | 0 | 0 | 0 | 0 | 0 | 0 | | | | | | |
| 5349 | 06379 | 05348 | 4 | | 0 | 0 | 0 | 0 | 0 | 0 | 0 | 0 | 0 | 0 | 1 | 0 | 0 | 0 | 0 | 1 | 0 | 1 | 0 | 0 | 0 | 0 | 0 | 1 | 0 | M83751 | 99.1 | 323 | 1 | 660 | 1103 |

Table 149

| | A | B | C | E | G | I | K | M | O | Q | S | U | W | Y | AA | AC | AE | AG | AI | AK | AM | AO | AQ | AS | AU | AW | AY | BA | BC | BE | BF | BG | BH | BI | BK |
|---|---|---|---|---|---|---|---|---|---|---|---|---|---|---|---|---|---|---|---|---|---|---|---|---|---|---|---|---|---|---|---|---|---|---|---|
| 5350 | 06380 | 05349 | 1 | | 0 | 0 | 0 | 0 | 0 | 0 | 0 | 0 | 0 | 0 | 0 | 0 | 0 | 0 | 0 | 1 | 0 | 0 | 0 | 0 | 0 | 0 | 0 | 0 | 0 | | | | | | |
| 5351 | 06381 | 05350 | 2 | | 0 | 0 | 0 | 0 | 0 | 0 | 0 | 0 | 0 | 0 | 0 | 0 | 0 | 0 | 0 | 1 | 0 | 0 | 0 | 1 | 0 | 0 | 0 | 0 | 0 | | | | | | |
| 5352 | 06382 | 05351 | 1 | | 0 | 0 | 0 | 0 | 0 | 0 | 0 | 0 | 0 | 0 | 0 | 0 | 0 | 0 | 0 | 1 | 0 | 0 | 0 | 0 | 0 | 0 | 0 | 0 | 0 | | | | | | |
| 5353 | 06383 | 05352 | 1 | | 0 | 0 | 0 | 0 | 0 | 0 | 0 | 0 | 0 | 0 | 0 | 0 | 0 | 0 | 0 | 1 | 0 | 0 | 0 | 0 | 0 | 0 | 0 | 0 | 0 | | | | | | |
| 5354 | 06384 | 05353 | 1 | | 0 | 0 | 0 | 0 | 0 | 0 | 0 | 0 | 0 | 0 | 0 | 0 | 0 | 0 | 0 | 1 | 0 | 0 | 0 | 0 | 0 | 0 | 0 | 0 | 0 | | | | | | |
| 5355 | 06385 | 05354 | 2 | | 0 | 0 | 0 | 0 | 0 | 0 | 0 | 0 | 0 | 0 | 0 | 0 | 0 | 0 | 0 | 1 | 0 | 0 | 0 | 0 | 0 | 0 | 1 | 0 | 0 | | | | | | |
| 5356 | 06386 | 05355 | 1 | | 0 | 0 | 0 | 0 | 0 | 0 | 0 | 0 | 0 | 0 | 0 | 0 | 0 | 0 | 0 | 1 | 0 | 0 | 0 | 0 | 0 | 0 | 0 | 0 | 0 | | | | | | |
| 5357 | 06387 | 05356 | 1 | | 0 | 0 | 0 | 0 | 0 | 0 | 0 | 0 | 0 | 0 | 0 | 0 | 0 | 0 | 0 | 0 | 0 | 1 | 0 | 0 | 0 | 0 | 0 | 0 | 0 | X56134 | 94.9 | 59 | 1 | 430 | 1766 |
| 5358 | 06388 | 05357 | 1 | | 0 | 0 | 0 | 0 | 0 | 0 | 0 | 0 | 0 | 0 | 0 | 0 | 0 | 0 | 0 | 0 | 0 | 1 | 0 | 0 | 0 | 0 | 0 | 0 | 0 | | | | | | |
| 5359 | 06389 | 05358 | 1 | | 0 | 0 | 0 | 0 | 0 | 0 | 0 | 0 | 0 | 0 | 0 | 0 | 0 | 0 | 0 | 0 | 0 | 1 | 0 | 0 | 0 | 0 | 0 | 0 | 0 | | | | | | |
| 5360 | 06390 | 05359 | 1 | | 0 | 0 | 0 | 0 | 0 | 0 | 0 | 0 | 0 | 0 | 0 | 0 | 0 | 0 | 0 | 0 | 0 | 1 | 0 | 0 | 0 | 0 | 0 | 0 | 0 | | | | | | |
| 5361 | 06392 | 05360 | 26 | | 0 | 0 | 0 | 0 | 0 | 0 | 0 | 0 | 0 | 0 | 0 | 0 | 0 | 0 | 0 | 0 | 0 | 26 | 0 | 0 | 0 | 0 | 0 | 0 | 0 | X05232 | 99 | 103 | 1 | 1699 | 1801 |
| 5362 | 06393 | 05361 | 1 | | 0 | 0 | 0 | 0 | 0 | 0 | 0 | 0 | 0 | 0 | 0 | 0 | 0 | 0 | 0 | 0 | 0 | 1 | 0 | 0 | 0 | 0 | 0 | 0 | 0 | | | | | | |
| 5363 | 06394 | 05362 | 1 | | 0 | 0 | 0 | 0 | 0 | 0 | 0 | 0 | 0 | 0 | 0 | 0 | 0 | 0 | 0 | 0 | 0 | 1 | 0 | 0 | 0 | 0 | 0 | 0 | 0 | | | | | | |
| 5364 | 06395 | 05363 | 2 | | 0 | 0 | 0 | 0 | 0 | 0 | 0 | 0 | 0 | 0 | 0 | 0 | 0 | 0 | 0 | 0 | 0 | 1 | 1 | 0 | 0 | 0 | 0 | 0 | 0 | L08239 | 98.4 | 63 | 1 | 1559 | 1631 |
| 5365 | 06396 | 05364 | 2 | | 0 | 0 | 0 | 0 | 0 | 0 | 0 | 0 | 0 | 0 | 0 | 0 | 0 | 0 | 0 | 0 | 0 | 1 | 0 | 0 | 0 | 1 | 0 | 0 | 0 | M92287 | 99.2 | 238 | 1 | 1794 | 2042 |
| 5366 | 06397 | 05365 | 1 | | 0 | 0 | 0 | 0 | 0 | 0 | 0 | 0 | 0 | 0 | 0 | 0 | 0 | 0 | 0 | 0 | 0 | 1 | 0 | 0 | 0 | 0 | 0 | 0 | 0 | | | | | | |
| 5367 | 06398 | 05366 | 1 | | 0 | 0 | 0 | 0 | 0 | 0 | 0 | 0 | 0 | 0 | 0 | 0 | 0 | 0 | 0 | 0 | 0 | 1 | 0 | 0 | 0 | 0 | 0 | 0 | 0 | | | | | | |
| 5368 | 06399 | 05367 | 1 | | 0 | 0 | 0 | 0 | 0 | 0 | 0 | 0 | 0 | 0 | 0 | 0 | 0 | 0 | 0 | 0 | 0 | 1 | 0 | 0 | 0 | 0 | 0 | 0 | 0 | | | | | | |
| 5369 | 06400 | 05368 | 1 | | 0 | 0 | 0 | 0 | 0 | 0 | 0 | 0 | 0 | 0 | 0 | 0 | 0 | 0 | 0 | 0 | 0 | 1 | 0 | 0 | 0 | 0 | 0 | 0 | 0 | | | | | | |
| 5370 | 06401 | 05369 | 1 | | 0 | 0 | 0 | 0 | 0 | 0 | 0 | 0 | 0 | 0 | 0 | 0 | 0 | 0 | 0 | 0 | 0 | 1 | 0 | 0 | 0 | 0 | 0 | 0 | 0 | | | | | | |
| 5371 | 06402 | 05370 | 1 | | 0 | 0 | 0 | 0 | 0 | 0 | 0 | 0 | 0 | 0 | 0 | 0 | 0 | 0 | 0 | 0 | 0 | 1 | 0 | 0 | 0 | 0 | 0 | 0 | 0 | | | | | | |
| 5372 | 06403 | 05371 | 1 | | 0 | 0 | 0 | 0 | 0 | 0 | 0 | 0 | 0 | 0 | 0 | 0 | 0 | 0 | 0 | 0 | 0 | 1 | 0 | 0 | 0 | 0 | 0 | 0 | 0 | | | | | | |
| 5373 | 06404 | 05372 | 2 | | 0 | 0 | 0 | 0 | 0 | 0 | 0 | 0 | 0 | 0 | 0 | 0 | 0 | 0 | 0 | 0 | 0 | 1 | 1 | 0 | 0 | 0 | 0 | 0 | 0 | | | | | | |
| 5374 | 06405 | 05373 | 1 | | 0 | 0 | 0 | 0 | 0 | 0 | 0 | 0 | 0 | 0 | 0 | 0 | 0 | 0 | 0 | 0 | 0 | 1 | 0 | 0 | 0 | 0 | 0 | 0 | 0 | X56134 | 94.8 | 193 | 1 | 430 | 1766 |
| 5375 | 06407 | 05374 | 2 | | 0 | 0 | 0 | 0 | 0 | 0 | 0 | 0 | 0 | 0 | 0 | 0 | 0 | 0 | 0 | 0 | 0 | 2 | 0 | 0 | 0 | 0 | 0 | 0 | 0 | M62403 | 100 | 70 | 1 | 1157 | 1955 |
| 5376 | 06408 | 05375 | 1 | | 0 | 0 | 0 | 0 | 0 | 0 | 0 | 0 | 0 | 0 | 0 | 0 | 0 | 0 | 0 | 0 | 0 | 1 | 0 | 0 | 0 | 0 | 0 | 0 | 0 | | | | | | |
| 5377 | 06409 | 05376 | 1 | | 0 | 0 | 0 | 0 | 0 | 0 | 0 | 0 | 0 | 0 | 0 | 0 | 0 | 0 | 0 | 0 | 0 | 1 | 0 | 0 | 0 | 0 | 0 | 0 | 0 | | | | | | |
| 5378 | 06410 | 05377 | 1 | | 0 | 0 | 0 | 0 | 0 | 0 | 0 | 0 | 0 | 0 | 0 | 0 | 0 | 0 | 0 | 0 | 0 | 1 | 0 | 0 | 0 | 0 | 0 | 0 | 0 | | | | | | |
| 5379 | 06411 | 05378 | 15 | | 0 | 0 | 0 | 0 | 0 | 0 | 0 | 0 | 0 | 0 | 0 | 0 | 0 | 0 | 0 | 0 | 0 | 15 | 0 | 0 | 0 | 0 | 0 | 0 | 0 | M13509 | 97 | 203 | 1 | 1769 | 1970 |
| 5380 | 06412 | 05379 | 1 | | 0 | 0 | 0 | 0 | 0 | 0 | 0 | 0 | 0 | 0 | 0 | 0 | 0 | 0 | 0 | 0 | 0 | 1 | 0 | 0 | 0 | 0 | 0 | 0 | 0 | | | | | | |
| 5381 | 06413 | 05380 | 1 | | 0 | 0 | 0 | 0 | 0 | 0 | 0 | 0 | 0 | 0 | 0 | 0 | 0 | 0 | 0 | 0 | 0 | 1 | 0 | 0 | 0 | 0 | 0 | 0 | 0 | | | | | | |
| 5382 | 06414 | 05381 | 1 | | 0 | 0 | 0 | 0 | 0 | 0 | 0 | 0 | 0 | 0 | 0 | 0 | 0 | 0 | 0 | 0 | 0 | 1 | 0 | 0 | 0 | 0 | 0 | 0 | 0 | | | | | | |
| 5383 | 06415 | 05382 | 1 | | 0 | 0 | 0 | 0 | 0 | 0 | 0 | 0 | 0 | 0 | 0 | 0 | 0 | 0 | 0 | 0 | 0 | 1 | 0 | 0 | 0 | 0 | 0 | 0 | 0 | | | | | | |
| 5384 | 06416 | 05383 | 1 | | 0 | 0 | 0 | 0 | 0 | 0 | 0 | 0 | 0 | 0 | 0 | 0 | 0 | 0 | 0 | 0 | 0 | 1 | 0 | 0 | 0 | 0 | 0 | 0 | 0 | | | | | | |
| 5385 | 06417 | 05384 | 1 | | 0 | 0 | 0 | 0 | 0 | 0 | 0 | 0 | 0 | 0 | 0 | 0 | 0 | 0 | 0 | 0 | 0 | 1 | 0 | 0 | 0 | 0 | 0 | 0 | 0 | | | | | | |

Table 150

| | A | B | C | E | G | I | K | M | O | Q | S | U | W | Y | AA | AC | AE | AG | AI | AK | AM | AO | AQ | AS | AU | AW | AY | BA | BC | BE | BF | BG | BH | BI | BK |
|---|---|---|---|---|---|---|---|---|---|---|---|---|---|---|---|---|---|---|---|---|---|---|---|---|---|---|---|---|---|---|---|---|---|---|---|
| 5386 | 06418 | 05385 | 1 | | 0 | 0 | 0 | 0 | 0 | 0 | 0 | 0 | 0 | 0 | 0 | 0 | 0 | 0 | 0 | 0 | 0 | 1 | 0 | 0 | 0 | 0 | 0 | 0 | 0 | | | | | | |
| 5387 | 06419 | 05386 | 2 | | 0 | 0 | 0 | 0 | 0 | 0 | 0 | 0 | 0 | 0 | 0 | 0 | 0 | 0 | 0 | 0 | 0 | 1 | 1 | 0 | 0 | 0 | 0 | 0 | 0 | | | | | | |
| 5388 | 06420 | 05387 | 1 | | 0 | 0 | 0 | 0 | 0 | 0 | 0 | 0 | 0 | 0 | 0 | 0 | 0 | 0 | 0 | 0 | 0 | 1 | 0 | 0 | 0 | 0 | 0 | 0 | 0 | | | | | | |
| 5389 | 06421 | 05388 | 2 | | 0 | 0 | 0 | 0 | 0 | 0 | 0 | 0 | 0 | 0 | 0 | 0 | 0 | 0 | 0 | 0 | 0 | 1 | 0 | 0 | 0 | 1 | 0 | 0 | 0 | M59040 | 99.3 | 296 | 1 | 934 | 1794 |
| 5390 | 06422 | 05389 | 1 | | 0 | 0 | 0 | 0 | 0 | 0 | 0 | 0 | 0 | 0 | 0 | 0 | 0 | 0 | 0 | 0 | 0 | 1 | 0 | 0 | 0 | 0 | 0 | 0 | 0 | | | | | | |
| 5391 | 06423 | 05390 | 1 | | 0 | 0 | 0 | 0 | 0 | 0 | 0 | 0 | 0 | 0 | 0 | 0 | 0 | 0 | 0 | 0 | 0 | 1 | 0 | 0 | 0 | 0 | 0 | 0 | 0 | | | | | | |
| 5392 | 06424 | 05391 | 2 | | 0 | 0 | 0 | 0 | 0 | 0 | 0 | 0 | 0 | 0 | 0 | 0 | 0 | 0 | 0 | 0 | 0 | 1 | 0 | 0 | 0 | 0 | 0 | 0 | 1 | | | | | | |
| 5393 | 06425 | 05392 | 1 | | 0 | 0 | 0 | 0 | 0 | 0 | 0 | 0 | 0 | 0 | 0 | 0 | 0 | 0 | 0 | 0 | 0 | 1 | 0 | 0 | 0 | 0 | 0 | 0 | 0 | | | | | | |
| 5394 | 06426 | 05393 | 1 | | 0 | 0 | 0 | 0 | 0 | 0 | 0 | 0 | 0 | 0 | 0 | 0 | 0 | 0 | 0 | 0 | 0 | 1 | 0 | 0 | 0 | 0 | 0 | 0 | 0 | | | | | | |
| 5395 | 06427 | 05394 | 1 | | 0 | 0 | 0 | 0 | 0 | 0 | 0 | 0 | 0 | 0 | 0 | 0 | 0 | 0 | 0 | 0 | 0 | 1 | 0 | 0 | 0 | 0 | 0 | 0 | 0 | | | | | | |
| 5396 | 06428 | 05395 | 1 | | 0 | 0 | 0 | 0 | 0 | 0 | 0 | 0 | 0 | 0 | 0 | 0 | 0 | 0 | 0 | 0 | 0 | 1 | 0 | 0 | 0 | 0 | 0 | 0 | 0 | | | | | | |
| 5397 | 06429 | 05396 | 1 | | 0 | 0 | 0 | 0 | 0 | 0 | 0 | 0 | 0 | 0 | 0 | 0 | 0 | 0 | 0 | 0 | 0 | 1 | 0 | 0 | 0 | 0 | 0 | 0 | 0 | | | | | | |
| 5398 | 06430 | 05397 | 1 | | 0 | 0 | 0 | 0 | 0 | 0 | 0 | 0 | 0 | 0 | 0 | 0 | 0 | 0 | 0 | 0 | 0 | 1 | 0 | 0 | 0 | 0 | 0 | 0 | 0 | | | | | | |
| 5399 | 06431 | 05398 | 1 | | 0 | 0 | 0 | 0 | 0 | 0 | 0 | 0 | 0 | 0 | 0 | 0 | 0 | 0 | 0 | 0 | 0 | 1 | 0 | 0 | 0 | 0 | 0 | 0 | 0 | | | | | | |
| 5400 | 06432 | 05399 | 1 | | 0 | 0 | 0 | 0 | 0 | 0 | 0 | 0 | 0 | 0 | 0 | 0 | 0 | 0 | 0 | 0 | 0 | 1 | 0 | 0 | 0 | 0 | 0 | 0 | 0 | | | | | | |
| 5401 | 06433 | 05400 | 1 | | 0 | 0 | 0 | 0 | 0 | 0 | 0 | 0 | 0 | 0 | 0 | 0 | 0 | 0 | 0 | 0 | 0 | 1 | 0 | 0 | 0 | 0 | 0 | 0 | 0 | | | | | | |
| 5402 | 06434 | 05401 | 4 | | 0 | 0 | 0 | 0 | 0 | 0 | 0 | 0 | 0 | 0 | 0 | 0 | 0 | 0 | 0 | 0 | 0 | 2 | 0 | 0 | 1 | 0 | 0 | 1 | 0 | | | | | | |
| 5403 | 06435 | 05402 | 2 | | 0 | 0 | 0 | 0 | 0 | 0 | 0 | 0 | 0 | 0 | 0 | 0 | 0 | 0 | 0 | 0 | 0 | 1 | 0 | 0 | 0 | 0 | 0 | 1 | 0 | S59184 | 94.9 | 274 | 1 | 2791 | 3068 |
| 5404 | 06436 | 05403 | 1 | | 0 | 0 | 0 | 0 | 0 | 0 | 0 | 0 | 0 | 0 | 0 | 0 | 0 | 0 | 0 | 0 | 0 | 1 | 0 | 0 | 0 | 0 | 0 | 0 | 0 | | | | | | |
| 5405 | 06437 | 05404 | 1 | | 0 | 0 | 0 | 0 | 0 | 0 | 0 | 0 | 0 | 0 | 0 | 0 | 0 | 0 | 0 | 0 | 0 | 1 | 0 | 0 | 0 | 0 | 0 | 0 | 0 | | | | | | |
| 5406 | 06438 | 05405 | 1 | | 0 | 0 | 0 | 0 | 0 | 0 | 0 | 0 | 0 | 0 | 0 | 0 | 0 | 0 | 0 | 0 | 0 | 1 | 0 | 0 | 0 | 0 | 0 | 0 | 0 | | | | | | |
| 5407 | 06439 | 05406 | 3 | | 0 | 0 | 0 | 0 | 0 | 0 | 0 | 0 | 0 | 0 | 0 | 0 | 0 | 0 | 0 | 0 | 0 | 3 | 0 | 0 | 0 | 0 | 0 | 0 | 0 | | | | | | |
| 5408 | 06440 | 05407 | 1 | | 0 | 0 | 0 | 0 | 0 | 0 | 0 | 0 | 0 | 0 | 0 | 0 | 0 | 0 | 0 | 0 | 0 | 1 | 0 | 0 | 0 | 0 | 0 | 0 | 0 | | | | | | |
| 5409 | 06441 | 05408 | 1 | | 0 | 0 | 0 | 0 | 0 | 0 | 0 | 0 | 0 | 0 | 0 | 0 | 0 | 0 | 0 | 0 | 0 | 1 | 0 | 0 | 0 | 0 | 0 | 0 | 0 | | | | | | |
| 5410 | 06442 | 05409 | 2 | | 0 | 0 | 0 | 0 | 0 | 0 | 0 | 0 | 0 | 0 | 0 | 0 | 0 | 0 | 0 | 0 | 0 | 2 | 0 | 0 | 0 | 0 | 0 | 0 | 0 | | | | | | |
| 5411 | 06443 | 05410 | 2 | | 0 | 0 | 0 | 0 | 0 | 0 | 0 | 0 | 0 | 0 | 0 | 0 | 0 | 0 | 0 | 0 | 0 | 1 | 0 | 0 | 0 | 1 | 0 | 0 | 0 | | | | | | |
| 5412 | 06444 | 05411 | 1 | | 0 | 0 | 0 | 0 | 0 | 0 | 0 | 0 | 0 | 0 | 0 | 0 | 0 | 0 | 0 | 0 | 0 | 1 | 0 | 0 | 0 | 0 | 0 | 0 | 0 | | | | | | |
| 5413 | 06445 | 05412 | 1 | | 0 | 0 | 0 | 0 | 0 | 0 | 0 | 0 | 0 | 0 | 0 | 0 | 0 | 0 | 0 | 0 | 0 | 1 | 0 | 0 | 0 | 0 | 0 | 0 | 0 | | | | | | |
| 5414 | 06446 | 05413 | 1 | | 0 | 0 | 0 | 0 | 0 | 0 | 0 | 0 | 0 | 0 | 0 | 0 | 0 | 0 | 0 | 0 | 0 | 1 | 0 | 0 | 0 | 0 | 0 | 0 | 0 | | | | | | |
| 5415 | 06447 | 05414 | 3 | | 0 | 0 | 0 | 0 | 0 | 0 | 0 | 0 | 0 | 0 | 0 | 0 | 0 | 0 | 0 | 0 | 0 | 3 | 0 | 0 | 0 | 0 | 0 | 0 | 0 | M62994 | 96.9 | 196 | 1 | 426 | 2007 |
| 5416 | 06448 | 05415 | 3 | | 0 | 0 | 0 | 0 | 0 | 0 | 0 | 0 | 0 | 0 | 0 | 0 | 0 | 0 | 0 | 0 | 0 | 1 | 0 | 0 | 0 | 2 | 0 | 0 | 0 | | | | | | |
| 5417 | 06449 | 05416 | 4 | | 0 | 0 | 0 | 0 | 0 | 0 | 0 | 0 | 0 | 0 | 0 | 0 | 0 | 0 | 0 | 0 | 0 | 2 | 0 | 0 | 0 | 2 | 0 | 0 | 0 | | | | | | |
| 5418 | 06450 | 05417 | 1 | | 0 | 0 | 0 | 0 | 0 | 0 | 0 | 0 | 0 | 0 | 0 | 0 | 0 | 0 | 0 | 0 | 0 | 1 | 0 | 0 | 0 | 0 | 0 | 0 | 0 | | | | | | |
| 5419 | 06451 | 05418 | 1 | | 0 | 0 | 0 | 0 | 0 | 0 | 0 | 0 | 0 | 0 | 0 | 0 | 0 | 0 | 0 | 0 | 0 | 1 | 0 | 0 | 0 | 0 | 0 | 0 | 0 | | | | | | |
| 5420 | 06452 | 05419 | 1 | | 0 | 0 | 0 | 0 | 0 | 0 | 0 | 0 | 0 | 0 | 0 | 0 | 0 | 0 | 0 | 0 | 0 | 1 | 0 | 0 | 0 | 0 | 0 | 0 | 0 | | | | | | |
| 5421 | 06453 | 05420 | 1 | | 0 | 0 | 0 | 0 | 0 | 0 | 0 | 0 | 0 | 0 | 0 | 0 | 0 | 0 | 0 | 0 | 0 | 1 | 0 | 0 | 0 | 0 | 0 | 0 | 0 | | | | | | |

Table 151

| | A | B | C | E | G | I | K | M | O | Q | S | U | W | Y | AA | AC | AE | AG | AI | AK | AM | AO | AQ | AS | AU | AW | AY | BA | BC | BE | BF | BG | BH | BI | BK |
|---|---|---|---|---|---|---|---|---|---|---|---|---|---|---|---|---|---|---|---|---|---|---|---|---|---|---|---|---|---|---|---|---|---|---|---|
| 5422 | 06454 | 05421 | 1 | | 0 | 0 | 0 | 0 | 0 | 0 | 0 | 0 | 0 | 0 | 0 | 0 | 0 | 0 | 0 | 0 | 0 | 1 | 0 | 0 | 0 | 0 | 0 | 0 | 0 | L10910 | 95.1 | 205 | 1 | 2334 | 2595 |
| 5423 | 06455 | 05422 | 1 | | 0 | 0 | 0 | 0 | 0 | 0 | 0 | 0 | 0 | 0 | 0 | 0 | 0 | 0 | 0 | 0 | 0 | 1 | 0 | 0 | 0 | 0 | 0 | 0 | 0 | | | | | | |
| 5424 | 06456 | 05423 | 1 | | 0 | 0 | 0 | 0 | 0 | 0 | 0 | 0 | 0 | 0 | 0 | 0 | 0 | 0 | 0 | 0 | 0 | 1 | 0 | 0 | 0 | 0 | 0 | 0 | 0 | | | | | | |
| 5425 | 06457 | 05424 | 1 | | 0 | 0 | 0 | 0 | 0 | 0 | 0 | 0 | 0 | 0 | 0 | 0 | 0 | 0 | 0 | 0 | 0 | 1 | 0 | 0 | 0 | 0 | 0 | 0 | 0 | | | | | | |
| 5426 | 06458 | 05425 | 1 | | 0 | 0 | 0 | 0 | 0 | 0 | 0 | 0 | 0 | 0 | 0 | 0 | 0 | 0 | 0 | 0 | 0 | 1 | 0 | 0 | 0 | 0 | 0 | 0 | 0 | | | | | | |
| 5427 | 06459 | 05426 | 1 | | 0 | 0 | 0 | 0 | 0 | 0 | 0 | 0 | 0 | 0 | 0 | 0 | 0 | 0 | 0 | 0 | 0 | 1 | 0 | 0 | 0 | 0 | 0 | 0 | 0 | | | | | | |
| 5428 | 06460 | 05427 | 1 | | 0 | 0 | 0 | 0 | 0 | 0 | 0 | 0 | 0 | 0 | 0 | 0 | 0 | 0 | 0 | 0 | 0 | 1 | 0 | 0 | 0 | 0 | 0 | 0 | 0 | M59465 | 100 | 183 | 1 | 4244 | 4426 |
| 5429 | 06461 | 05428 | 1 | | 0 | 0 | 0 | 0 | 0 | 0 | 0 | 0 | 0 | 0 | 0 | 0 | 0 | 0 | 0 | 0 | 0 | 1 | 0 | 0 | 0 | 0 | 0 | 0 | 0 | | | | | | |
| 5430 | 06462 | 05429 | 1 | | 0 | 0 | 0 | 0 | 0 | 0 | 0 | 0 | 0 | 0 | 0 | 0 | 0 | 0 | 0 | 0 | 0 | 1 | 0 | 0 | 0 | 0 | 0 | 0 | 0 | | | | | | |
| 5431 | 06463 | 05430 | 2 | | 0 | 0 | 0 | 0 | 0 | 0 | 0 | 0 | 0 | 0 | 0 | 0 | 0 | 0 | 0 | 0 | 0 | 1 | 0 | 1 | 0 | 0 | 0 | 0 | 0 | | | | | | |
| 5432 | 06464 | 05431 | 1 | | 0 | 0 | 0 | 0 | 0 | 0 | 0 | 0 | 0 | 0 | 0 | 0 | 0 | 0 | 0 | 0 | 0 | 1 | 0 | 0 | 0 | 0 | 0 | 0 | 0 | | | | | | |
| 5433 | 06465 | 05432 | 1 | | 0 | 0 | 0 | 0 | 0 | 0 | 0 | 0 | 0 | 0 | 0 | 0 | 0 | 0 | 0 | 0 | 0 | 1 | 0 | 0 | 0 | 0 | 0 | 0 | 0 | | | | | | |
| 5434 | 06466 | 05433 | 1 | | 0 | 0 | 0 | 0 | 0 | 0 | 0 | 0 | 0 | 0 | 0 | 0 | 0 | 0 | 0 | 0 | 0 | 1 | 0 | 0 | 0 | 0 | 0 | 0 | 0 | | | | | | |
| 5435 | 06468 | 05434 | 1 | | 0 | 0 | 0 | 0 | 0 | 0 | 0 | 0 | 0 | 0 | 0 | 0 | 0 | 0 | 0 | 0 | 0 | 1 | 0 | 0 | 0 | 0 | 0 | 0 | 0 | | | | | | |
| 5436 | 06469 | 05435 | 1 | | 0 | 0 | 0 | 0 | 0 | 0 | 0 | 0 | 0 | 0 | 0 | 0 | 0 | 0 | 0 | 0 | 0 | 1 | 0 | 0 | 0 | 0 | 0 | 0 | 0 | | | | | | |
| 5437 | 06470 | 05436 | 1 | | 0 | 0 | 0 | 0 | 0 | 0 | 0 | 0 | 0 | 0 | 0 | 0 | 0 | 0 | 0 | 0 | 0 | 1 | 0 | 0 | 0 | 0 | 0 | 0 | 0 | | | | | | |
| 5438 | 06471 | 05437 | 1 | | 0 | 0 | 0 | 0 | 0 | 0 | 0 | 0 | 0 | 0 | 0 | 0 | 0 | 0 | 0 | 0 | 0 | 1 | 0 | 0 | 0 | 0 | 0 | 0 | 0 | | | | | | |
| 5439 | 06472 | 05438 | 1 | | 0 | 0 | 0 | 0 | 0 | 0 | 0 | 0 | 0 | 0 | 0 | 0 | 0 | 0 | 0 | 0 | 0 | 1 | 0 | 0 | 0 | 0 | 0 | 0 | 0 | | | | | | |
| 5440 | 06473 | 05439 | 3 | | 0 | 0 | 0 | 0 | 0 | 0 | 0 | 0 | 0 | 0 | 0 | 0 | 0 | 0 | 0 | 0 | 0 | 2 | 0 | 0 | 1 | 0 | 0 | 0 | 0 | | | | | | |
| 5441 | 06474 | 05440 | 1 | | 0 | 0 | 0 | 0 | 0 | 0 | 0 | 0 | 0 | 0 | 0 | 0 | 0 | 0 | 0 | 0 | 0 | 1 | 0 | 0 | 0 | 0 | 0 | 0 | 0 | | | | | | |
| 5442 | 06475 | 05441 | 2 | | 0 | 0 | 0 | 0 | 0 | 0 | 0 | 0 | 0 | 0 | 0 | 0 | 0 | 0 | 0 | 0 | 0 | 1 | 0 | 0 | 1 | 0 | 0 | 0 | 0 | | | | | | |
| 5443 | 06476 | 05442 | 1 | | 0 | 0 | 0 | 0 | 0 | 0 | 0 | 0 | 0 | 0 | 0 | 0 | 0 | 0 | 0 | 0 | 0 | 1 | 0 | 0 | 0 | 0 | 0 | 0 | 0 | | | | | | |
| 5444 | 06477 | 05443 | 2 | | 0 | 0 | 0 | 0 | 0 | 0 | 0 | 0 | 0 | 0 | 0 | 0 | 0 | 0 | 0 | 0 | 0 | 1 | 0 | 0 | 0 | 0 | 0 | 1 | 0 | X63657 | 96.8 | 313 | 1 | 1738 | 2272 |
| 5445 | 06478 | 05444 | 1 | | 0 | 0 | 0 | 0 | 0 | 0 | 0 | 0 | 0 | 0 | 0 | 0 | 0 | 0 | 0 | 0 | 0 | 1 | 0 | 0 | 0 | 0 | 0 | 0 | 0 | | | | | | |
| 5446 | 06481 | 05445 | 1 | | 0 | 0 | 0 | 0 | 0 | 0 | 0 | 0 | 0 | 0 | 0 | 0 | 0 | 0 | 0 | 0 | 0 | 1 | 0 | 0 | 0 | 0 | 0 | 0 | 0 | X12510 | 95.6 | 250 | 1 | 760 | 1041 |
| 5447 | 06482 | 05446 | 2 | | 0 | 0 | 0 | 0 | 0 | 0 | 0 | 0 | 0 | 0 | 0 | 0 | 0 | 0 | 0 | 0 | 0 | 1 | 0 | 0 | 0 | 0 | 1 | 0 | 0 | | | | | | |
| 5448 | 06483 | 05447 | 1 | | 0 | 0 | 0 | 0 | 0 | 0 | 0 | 0 | 0 | 0 | 0 | 0 | 0 | 0 | 0 | 0 | 0 | 1 | 0 | 0 | 0 | 0 | 0 | 0 | 0 | L25610 | 92.8 | 333 | 1 | 1641 | 2098 |
| 5449 | 06484 | 05448 | 1 | | 0 | 0 | 0 | 0 | 0 | 0 | 0 | 0 | 0 | 0 | 0 | 0 | 0 | 0 | 0 | 0 | 0 | 1 | 0 | 0 | 0 | 0 | 0 | 0 | 0 | | | | | | |
| 5450 | 06485 | 05449 | 1 | | 0 | 0 | 0 | 0 | 0 | 0 | 0 | 0 | 0 | 0 | 0 | 0 | 0 | 0 | 0 | 0 | 0 | 1 | 0 | 0 | 0 | 0 | 0 | 0 | 0 | | | | | | |
| 5451 | 06486 | 05450 | 1 | | 0 | 0 | 0 | 0 | 0 | 0 | 0 | 0 | 0 | 0 | 0 | 0 | 0 | 0 | 0 | 0 | 0 | 1 | 0 | 0 | 0 | 0 | 0 | 0 | 0 | | | | | | |
| 5452 | 06487 | 05451 | 2 | | 0 | 0 | 0 | 0 | 0 | 0 | 0 | 0 | 0 | 0 | 0 | 0 | 0 | 0 | 0 | 0 | 0 | 1 | 1 | 0 | 0 | 0 | 0 | 0 | 0 | | | | | | |
| 5453 | 06488 | 05452 | 2 | | 0 | 0 | 0 | 0 | 0 | 0 | 0 | 0 | 0 | 0 | 0 | 0 | 0 | 0 | 0 | 0 | 0 | 2 | 0 | 0 | 0 | 0 | 0 | 0 | 0 | | | | | | |
| 5454 | 06489 | 05453 | 2 | | 0 | 0 | 0 | 0 | 0 | 0 | 0 | 0 | 0 | 0 | 0 | 0 | 0 | 0 | 0 | 0 | 0 | 1 | 0 | 1 | 0 | 0 | 0 | 0 | 0 | | | | | | |
| 5455 | 06490 | 05454 | 1 | | 0 | 0 | 0 | 0 | 0 | 0 | 0 | 0 | 0 | 0 | 0 | 0 | 0 | 0 | 0 | 0 | 0 | 1 | 0 | 0 | 0 | 0 | 0 | 0 | 0 | | | | | | |
| 5456 | 06491 | 05455 | 1 | | 0 | 0 | 0 | 0 | 0 | 0 | 0 | 0 | 0 | 0 | 0 | 0 | 0 | 0 | 0 | 0 | 0 | 1 | 0 | 0 | 0 | 0 | 0 | 0 | 0 | | | | | | |
| 5457 | 06492 | 05456 | 2 | | 0 | 0 | 0 | 0 | 0 | 0 | 0 | 0 | 0 | 0 | 0 | 0 | 0 | 0 | 0 | 0 | 0 | 1 | 0 | 0 | 0 | 1 | 0 | 0 | 0 | | | | | | |

Table 152

| | A | B | C | E | G | I | K | M | O | Q | S | U | W | Y | AA | AC | AE | AG | AI | AK | AM | AO | AQ | AS | AU | AW | AY | BA | BC | BE | BF | BG | BH | BI | BK |
|---|---|---|---|---|---|---|---|---|---|---|---|---|---|---|---|---|---|---|---|---|---|---|---|---|---|---|---|---|---|---|---|---|---|---|---|
| 5458 | 06493 | 05457 | 1 | | 0 | 0 | 0 | 0 | 0 | 0 | 0 | 0 | 0 | 0 | 0 | 0 | 0 | 0 | 0 | 0 | 0 | 1 | 0 | 0 | 0 | 0 | 0 | 0 | 0 | | | | | | |
| 5459 | 06496 | 05458 | 2 | | 0 | 0 | 0 | 0 | 0 | 0 | 0 | 0 | 0 | 0 | 0 | 0 | 0 | 0 | 0 | 0 | 0 | 2 | 0 | 0 | 0 | 0 | 0 | 0 | 0 | | | | | | |
| 5460 | 06498 | 05459 | 1 | | 0 | 0 | 0 | 0 | 0 | 0 | 0 | 0 | 0 | 0 | 0 | 0 | 0 | 0 | 0 | 0 | 0 | 1 | 0 | 0 | 0 | 0 | 0 | 0 | 0 | | | | | | |
| 5461 | 06499 | 05460 | 1 | | 0 | 0 | 0 | 0 | 0 | 0 | 0 | 0 | 0 | 0 | 0 | 0 | 0 | 0 | 0 | 0 | 0 | 1 | 0 | 0 | 0 | 0 | 0 | 0 | 0 | | | | | | |
| 5462 | 06500 | 05461 | 2 | | 0 | 0 | 0 | 0 | 0 | 0 | 0 | 0 | 0 | 0 | 0 | 0 | 0 | 0 | 0 | 0 | 0 | 1 | 0 | 1 | 0 | 0 | 0 | 0 | 0 | L24559 | 97.1 | 68 | 1 | 2112 | 2187 |
| 5463 | 06501 | 05462 | 1 | | 0 | 0 | 0 | 0 | 0 | 0 | 0 | 0 | 0 | 0 | 0 | 0 | 0 | 0 | 0 | 0 | 0 | 1 | 0 | 0 | 0 | 0 | 0 | 0 | 0 | | | | | | |
| 5464 | 06502 | 05463 | 1 | | 0 | 0 | 0 | 0 | 0 | 0 | 0 | 0 | 0 | 0 | 0 | 0 | 0 | 0 | 0 | 0 | 0 | 1 | 0 | 0 | 0 | 0 | 0 | 0 | 0 | | | | | | |
| 5465 | 06503 | 05464 | 1 | | 0 | 0 | 0 | 0 | 0 | 0 | 0 | 0 | 0 | 0 | 0 | 0 | 0 | 0 | 0 | 0 | 0 | 1 | 0 | 0 | 0 | 0 | 0 | 0 | 0 | | | | | | |
| 5466 | 06504 | 05465 | 1 | | 0 | 0 | 0 | 0 | 0 | 0 | 0 | 0 | 0 | 0 | 0 | 0 | 0 | 0 | 0 | 0 | 0 | 1 | 0 | 0 | 0 | 0 | 0 | 0 | 0 | | | | | | |
| 5467 | 06505 | 05466 | 1 | | 0 | 0 | 0 | 0 | 0 | 0 | 0 | 0 | 0 | 0 | 0 | 0 | 0 | 0 | 0 | 0 | 0 | 1 | 0 | 0 | 0 | 0 | 0 | 0 | 0 | | | | | | |
| 5468 | 06506 | 05467 | 1 | | 0 | 0 | 0 | 0 | 0 | 0 | 0 | 0 | 0 | 0 | 0 | 0 | 0 | 0 | 0 | 0 | 0 | 1 | 0 | 0 | 0 | 0 | 0 | 0 | 0 | | | | | | |
| 5469 | 06507 | 05468 | 2 | | 0 | 0 | 0 | 0 | 0 | 0 | 0 | 0 | 0 | 0 | 0 | 0 | 0 | 0 | 0 | 0 | 0 | 1 | 0 | 0 | 0 | 0 | 0 | 1 | 0 | | | | | | |
| 5470 | 06508 | 05469 | 1 | | 0 | 0 | 0 | 0 | 0 | 0 | 0 | 0 | 0 | 0 | 0 | 0 | 0 | 0 | 0 | 0 | 0 | 1 | 0 | 0 | 0 | 0 | 0 | 0 | 0 | | | | | | |
| 5471 | 06509 | 05470 | 1 | | 0 | 0 | 0 | 0 | 0 | 0 | 0 | 0 | 0 | 0 | 0 | 0 | 0 | 0 | 0 | 0 | 0 | 1 | 0 | 0 | 0 | 0 | 0 | 0 | 0 | | | | | | |
| 5472 | 06510 | 05471 | 1 | | 0 | 0 | 0 | 0 | 0 | 0 | 0 | 0 | 0 | 0 | 0 | 0 | 0 | 0 | 0 | 0 | 0 | 1 | 0 | 0 | 0 | 0 | 0 | 0 | 0 | | | | | | |
| 5473 | 06511 | 05472 | 1 | | 0 | 0 | 0 | 0 | 0 | 0 | 0 | 0 | 0 | 0 | 0 | 0 | 0 | 0 | 0 | 0 | 0 | 1 | 0 | 0 | 0 | 0 | 0 | 0 | 0 | | | | | | |
| 5474 | 06512 | 05473 | 1 | | 0 | 0 | 0 | 0 | 0 | 0 | 0 | 0 | 0 | 0 | 0 | 0 | 0 | 0 | 0 | 0 | 0 | 1 | 0 | 0 | 0 | 0 | 0 | 0 | 0 | | | | | | |
| 5475 | 06513 | 05474 | 1 | | 0 | 0 | 0 | 0 | 0 | 0 | 0 | 0 | 0 | 0 | 0 | 0 | 0 | 0 | 0 | 0 | 0 | 1 | 0 | 0 | 0 | 0 | 0 | 0 | 0 | | | | | | |
| 5476 | 06514 | 05475 | 1 | | 0 | 0 | 0 | 0 | 0 | 0 | 0 | 0 | 0 | 0 | 0 | 0 | 0 | 0 | 0 | 0 | 0 | 1 | 0 | 0 | 0 | 0 | 0 | 0 | 0 | | | | | | |
| 5477 | 06515 | 05476 | 4 | | 0 | 0 | 0 | 0 | 0 | 0 | 0 | 0 | 0 | 0 | 0 | 0 | 0 | 0 | 0 | 0 | 0 | 2 | 1 | 0 | 1 | 0 | 0 | 0 | 0 | L16785 | 100 | 49 | 1 | 599 | 647 |
| 5478 | 06516 | 05477 | 2 | | 0 | 0 | 0 | 0 | 0 | 0 | 0 | 0 | 0 | 0 | 0 | 0 | 0 | 0 | 0 | 0 | 0 | 1 | 0 | 1 | 0 | 0 | 0 | 0 | 0 | | | | | | |
| 5479 | 06517 | 05478 | 1 | | 0 | 0 | 0 | 0 | 0 | 0 | 0 | 0 | 0 | 0 | 0 | 0 | 0 | 0 | 0 | 0 | 0 | 1 | 0 | 0 | 0 | 0 | 0 | 0 | 0 | | | | | | |
| 5480 | 06518 | 05479 | 1 | | 0 | 0 | 0 | 0 | 0 | 0 | 0 | 0 | 0 | 0 | 0 | 0 | 0 | 0 | 0 | 0 | 0 | 1 | 0 | 0 | 0 | 0 | 0 | 0 | 0 | | | | | | |
| 5481 | 06519 | 05480 | 1 | | 0 | 0 | 0 | 0 | 0 | 0 | 0 | 0 | 0 | 0 | 0 | 0 | 0 | 0 | 0 | 0 | 0 | 1 | 0 | 0 | 0 | 0 | 0 | 0 | 0 | | | | | | |
| 5482 | 06520 | 05481 | 1 | | 0 | 0 | 0 | 0 | 0 | 0 | 0 | 0 | 0 | 0 | 0 | 0 | 0 | 0 | 0 | 0 | 0 | 1 | 0 | 0 | 0 | 0 | 0 | 0 | 0 | | | | | | |
| 5483 | 06521 | 05482 | 1 | | 0 | 0 | 0 | 0 | 0 | 0 | 0 | 0 | 0 | 0 | 0 | 0 | 0 | 0 | 0 | 0 | 0 | 1 | 0 | 0 | 0 | 0 | 0 | 0 | 0 | | | | | | |
| 5484 | 06522 | 05483 | 1 | | 0 | 0 | 0 | 0 | 0 | 0 | 0 | 0 | 0 | 0 | 0 | 0 | 0 | 0 | 0 | 0 | 0 | 1 | 0 | 0 | 0 | 0 | 0 | 0 | 0 | | | | | | |
| 5485 | 06523 | 05484 | 1 | | 0 | 0 | 0 | 0 | 0 | 0 | 0 | 0 | 0 | 0 | 0 | 0 | 0 | 0 | 0 | 0 | 0 | 1 | 0 | 0 | 0 | 0 | 0 | 0 | 0 | | | | | | |
| 5486 | 06524 | 05485 | 1 | | 0 | 0 | 0 | 0 | 0 | 0 | 0 | 0 | 0 | 0 | 0 | 0 | 0 | 0 | 0 | 0 | 0 | 1 | 0 | 0 | 0 | 0 | 0 | 0 | 0 | | | | | | |
| 5487 | 06525 | 05486 | 1 | | 0 | 0 | 0 | 0 | 0 | 0 | 0 | 0 | 0 | 0 | 0 | 0 | 0 | 0 | 0 | 0 | 0 | 1 | 0 | 0 | 0 | 0 | 0 | 0 | 0 | | | | | | |
| 5488 | 06526 | 05487 | 1 | | 0 | 0 | 0 | 0 | 0 | 0 | 0 | 0 | 0 | 0 | 0 | 0 | 0 | 0 | 0 | 0 | 0 | 1 | 0 | 0 | 0 | 0 | 0 | 0 | 0 | | | | | | |
| 5489 | 06527 | 05488 | 1 | | 0 | 0 | 0 | 0 | 0 | 0 | 0 | 0 | 0 | 0 | 0 | 0 | 0 | 0 | 0 | 0 | 0 | 1 | 0 | 0 | 0 | 0 | 0 | 0 | 0 | | | | | | |
| 5490 | 06528 | 05489 | 1 | | 0 | 0 | 0 | 0 | 0 | 0 | 0 | 0 | 0 | 0 | 0 | 0 | 0 | 0 | 0 | 0 | 0 | 1 | 0 | 0 | 0 | 0 | 0 | 0 | 0 | | | | | | |
| 5491 | 06529 | 05490 | 1 | | 0 | 0 | 0 | 0 | 0 | 0 | 0 | 0 | 0 | 0 | 0 | 0 | 0 | 0 | 0 | 0 | 0 | 1 | 0 | 0 | 0 | 0 | 0 | 0 | 0 | | | | | | |
| 5492 | 06530 | 05491 | 1 | | 0 | 0 | 0 | 0 | 0 | 0 | 0 | 0 | 0 | 0 | 0 | 0 | 0 | 0 | 0 | 0 | 0 | 1 | 0 | 0 | 0 | 0 | 0 | 0 | 0 | | | | | | |
| 5493 | 06531 | 05492 | 1 | | 0 | 0 | 0 | 0 | 0 | 0 | 0 | 0 | 0 | 0 | 0 | 0 | 0 | 0 | 0 | 0 | 0 | 1 | 0 | 0 | 0 | 0 | 0 | 0 | 0 | | | | | | |

Table 153

| | A | B | C | E | G | I | K | M | O | Q | S | U | W | Y | AA | AC | AE | AG | AI | AK | AM | AO | AQ | AS | AU | AW | AY | BA | BC | BE | BF | BG | BH | BI | BK |
|---|---|---|---|---|---|---|---|---|---|---|---|---|---|---|---|---|---|---|---|---|---|---|---|---|---|---|---|---|---|---|---|---|---|---|---|
| 5494 | 06532 | 05493 | 1 | | 0 | 0 | 0 | 0 | 0 | 0 | 0 | 0 | 0 | 0 | 0 | 0 | 0 | 0 | 0 | 0 | 0 | 1 | 0 | 0 | 0 | 0 | 0 | 0 | 0 | | | | | | |
| 5495 | 06533 | 05494 | 1 | | 0 | 0 | 0 | 0 | 0 | 0 | 0 | 0 | 0 | 0 | 0 | 0 | 0 | 0 | 0 | 0 | 0 | 1 | 0 | 0 | 0 | 0 | 0 | 0 | 0 | | | | | | |
| 5496 | 06536 | 05495 | 1 | | 0 | 0 | 0 | 0 | 0 | 0 | 0 | 0 | 0 | 0 | 0 | 0 | 0 | 0 | 0 | 0 | 0 | 1 | 0 | 0 | 0 | 0 | 0 | 0 | 0 | | | | | | |
| 5497 | 06537 | 05496 | 1 | | 0 | 0 | 0 | 0 | 0 | 0 | 0 | 0 | 0 | 0 | 0 | 0 | 0 | 0 | 0 | 0 | 0 | 1 | 0 | 0 | 0 | 0 | 0 | 0 | 0 | | | | | | |
| 5498 | 06538 | 05497 | 3 | | 0 | 0 | 0 | 0 | 0 | 0 | 0 | 0 | 0 | 0 | 0 | 0 | 0 | 1 | 0 | 0 | 0 | 1 | 0 | 1 | 0 | 0 | 0 | 0 | 0 | | | | | | |
| 5499 | 06539 | 05498 | 1 | | 0 | 0 | 0 | 0 | 0 | 0 | 0 | 0 | 0 | 0 | 0 | 0 | 0 | 0 | 0 | 0 | 0 | 1 | 0 | 0 | 0 | 0 | 0 | 0 | 0 | | | | | | |
| 5500 | 06540 | 05499 | 1 | | 0 | 0 | 0 | 0 | 0 | 0 | 0 | 0 | 0 | 0 | 0 | 0 | 0 | 0 | 0 | 0 | 0 | 1 | 0 | 0 | 0 | 0 | 0 | 0 | 0 | | | | | | |
| 5501 | 06541 | 05500 | 1 | | 0 | 0 | 0 | 0 | 0 | 0 | 0 | 0 | 0 | 0 | 0 | 0 | 0 | 0 | 0 | 0 | 0 | 1 | 0 | 0 | 0 | 0 | 0 | 0 | 0 | X02761 | 94.9 | 313 | 1 | 4357 | 7680 |
| 5502 | 06542 | 05501 | 1 | | 0 | 0 | 0 | 0 | 0 | 0 | 0 | 0 | 0 | 0 | 0 | 0 | 0 | 0 | 0 | 0 | 0 | 1 | 0 | 0 | 0 | 0 | 0 | 0 | 0 | | | | | | |
| 5503 | 06544 | 05502 | 1 | | 0 | 0 | 0 | 0 | 0 | 0 | 0 | 0 | 0 | 0 | 0 | 0 | 0 | 0 | 0 | 0 | 0 | 1 | 0 | 0 | 0 | 0 | 0 | 0 | 0 | | | | | | |
| 5504 | 06545 | 05503 | 1 | | 0 | 0 | 0 | 0 | 0 | 0 | 0 | 0 | 0 | 0 | 0 | 0 | 0 | 0 | 0 | 0 | 0 | 1 | 0 | 0 | 0 | 0 | 0 | 0 | 0 | | | | | | |
| 5505 | 06546 | 05504 | 1 | | 0 | 0 | 0 | 0 | 0 | 0 | 0 | 0 | 0 | 0 | 0 | 0 | 0 | 0 | 0 | 0 | 0 | 1 | 0 | 0 | 0 | 0 | 0 | 0 | 0 | | | | | | |
| 5506 | 06547 | 05505 | 1 | | 0 | 0 | 0 | 0 | 0 | 0 | 0 | 0 | 0 | 0 | 0 | 0 | 0 | 0 | 0 | 0 | 0 | 1 | 0 | 0 | 0 | 0 | 0 | 0 | 0 | | | | | | |
| 5507 | 06548 | 05506 | 1 | | 0 | 0 | 0 | 0 | 0 | 0 | 0 | 0 | 0 | 0 | 0 | 0 | 0 | 0 | 0 | 0 | 0 | 1 | 0 | 0 | 0 | 0 | 0 | 0 | 0 | | | | | | |
| 5508 | 06549 | 05507 | 2 | | 0 | 0 | 0 | 0 | 0 | 0 | 0 | 0 | 0 | 0 | 0 | 0 | 0 | 0 | 0 | 0 | 0 | 1 | 0 | 0 | 0 | 0 | 1 | 0 | 0 | | | | | | |
| 5509 | 06550 | 05508 | 1 | | 0 | 0 | 0 | 0 | 0 | 0 | 0 | 0 | 0 | 0 | 0 | 0 | 0 | 0 | 0 | 0 | 0 | 1 | 0 | 0 | 0 | 0 | 0 | 0 | 0 | | | | | | |
| 5510 | 06551 | 05509 | 1 | | 0 | 0 | 0 | 0 | 0 | 0 | 0 | 0 | 0 | 0 | 0 | 0 | 0 | 0 | 0 | 0 | 0 | 1 | 0 | 0 | 0 | 0 | 0 | 0 | 0 | | | | | | |
| 5511 | 06552 | 05510 | 1 | | 0 | 0 | 0 | 0 | 0 | 0 | 0 | 0 | 0 | 0 | 0 | 0 | 0 | 0 | 0 | 0 | 0 | 1 | 0 | 0 | 0 | 0 | 0 | 0 | 0 | | | | | | |
| 5512 | 06553 | 05511 | 1 | | 0 | 0 | 0 | 0 | 0 | 0 | 0 | 0 | 0 | 0 | 0 | 0 | 0 | 0 | 0 | 0 | 0 | 1 | 0 | 0 | 0 | 0 | 0 | 0 | 0 | | | | | | |
| 5513 | 06555 | 05512 | 1 | | 0 | 0 | 0 | 0 | 0 | 0 | 0 | 0 | 0 | 0 | 0 | 0 | 0 | 0 | 0 | 0 | 0 | 1 | 0 | 0 | 0 | 0 | 0 | 0 | 0 | | | | | | |
| 5514 | 06556 | 05513 | 1 | | 0 | 0 | 0 | 0 | 0 | 0 | 0 | 0 | 0 | 0 | 0 | 0 | 0 | 0 | 0 | 0 | 0 | 1 | 0 | 0 | 0 | 0 | 0 | 0 | 0 | | | | | | |
| 5515 | 06557 | 05514 | 1 | | 0 | 0 | 0 | 0 | 0 | 0 | 0 | 0 | 0 | 0 | 0 | 0 | 0 | 0 | 0 | 0 | 0 | 1 | 0 | 0 | 0 | 0 | 0 | 0 | 0 | | | | | | |
| 5516 | 06558 | 05515 | 1 | | 0 | 0 | 0 | 0 | 0 | 0 | 0 | 0 | 0 | 0 | 0 | 0 | 0 | 0 | 0 | 0 | 0 | 1 | 0 | 0 | 0 | 0 | 0 | 0 | 0 | | | | | | |
| 5517 | 06559 | 05516 | 1 | | 0 | 0 | 0 | 0 | 0 | 0 | 0 | 0 | 0 | 0 | 0 | 0 | 0 | 0 | 0 | 0 | 0 | 1 | 0 | 0 | 0 | 0 | 0 | 0 | 0 | | | | | | |
| 5518 | 06560 | 05517 | 1 | | 0 | 0 | 0 | 0 | 0 | 0 | 0 | 0 | 0 | 0 | 0 | 0 | 0 | 0 | 0 | 0 | 0 | 1 | 0 | 0 | 0 | 0 | 0 | 0 | 0 | | | | | | |
| 5519 | 06561 | 05518 | 2 | | 0 | 0 | 0 | 0 | 0 | 0 | 0 | 0 | 0 | 0 | 0 | 0 | 0 | 0 | 0 | 0 | 0 | 1 | 0 | 0 | 0 | 0 | 0 | 1 | 0 | | | | | | |
| 5520 | 06563 | 05519 | 1 | | 0 | 0 | 0 | 0 | 0 | 0 | 0 | 0 | 0 | 0 | 0 | 0 | 0 | 0 | 0 | 0 | 0 | 1 | 0 | 0 | 0 | 0 | 0 | 0 | 0 | | | | | | |
| 5521 | 06564 | 05520 | 1 | | 0 | 0 | 0 | 0 | 0 | 0 | 0 | 0 | 0 | 0 | 0 | 0 | 0 | 0 | 0 | 0 | 0 | 1 | 0 | 0 | 0 | 0 | 0 | 0 | 0 | | | | | | |
| 5522 | 06565 | 05521 | 1 | | 0 | 0 | 0 | 0 | 0 | 0 | 0 | 0 | 0 | 0 | 0 | 0 | 0 | 0 | 0 | 0 | 0 | 1 | 0 | 0 | 0 | 0 | 0 | 0 | 0 | | | | | | |
| 5523 | 06566 | 05522 | 1 | | 0 | 0 | 0 | 0 | 0 | 0 | 0 | 0 | 0 | 0 | 0 | 0 | 0 | 0 | 0 | 0 | 0 | 1 | 0 | 0 | 0 | 0 | 0 | 0 | 0 | X07820 | 94.4 | 269 | 1 | 1374 | 1743 |
| 5524 | 06567 | 05523 | 1 | | 0 | 0 | 0 | 0 | 0 | 0 | 0 | 0 | 0 | 0 | 0 | 0 | 0 | 0 | 0 | 0 | 0 | 1 | 0 | 0 | 0 | 0 | 0 | 0 | 0 | | | | | | |
| 5525 | 06569 | 05524 | 1 | | 0 | 0 | 0 | 0 | 0 | 0 | 0 | 0 | 0 | 0 | 0 | 0 | 0 | 0 | 0 | 0 | 0 | 1 | 0 | 0 | 0 | 0 | 0 | 0 | 0 | | | | | | |
| 5526 | 06571 | 05525 | 1 | | 0 | 0 | 0 | 0 | 0 | 0 | 0 | 0 | 0 | 0 | 0 | 0 | 0 | 0 | 0 | 0 | 0 | 1 | 0 | 0 | 0 | 0 | 0 | 0 | 0 | | | | | | |
| 5527 | 06572 | 05526 | 1 | | 0 | 0 | 0 | 0 | 0 | 0 | 0 | 0 | 0 | 0 | 0 | 0 | 0 | 0 | 0 | 0 | 0 | 1 | 0 | 0 | 0 | 0 | 0 | 0 | 0 | | | | | | |
| 5528 | 06573 | 05527 | 1 | | 0 | 0 | 0 | 0 | 0 | 0 | 0 | 0 | 0 | 0 | 0 | 0 | 0 | 0 | 0 | 0 | 0 | 1 | 0 | 0 | 0 | 0 | 0 | 0 | 0 | | | | | | |
| 5529 | 06574 | 05528 | 1 | | 0 | 0 | 0 | 0 | 0 | 0 | 0 | 0 | 0 | 0 | 0 | 0 | 0 | 0 | 0 | 0 | 0 | 1 | 0 | 0 | 0 | 0 | 0 | 0 | 0 | | | | | | |

Table 154

| | A | B | C | E | G | I | K | M | O | Q | S | U | W | Y | AA | AC | AE | AG | AI | AK | AM | AO | AQ | AS | AU | AW | AY | BA | BC | BE | BF | BG | BH | BI | BK |
|---|---|---|---|---|---|---|---|---|---|---|---|---|---|---|---|---|---|---|---|---|---|---|---|---|---|---|---|---|---|---|---|---|---|---|---|
| 5530 | 06576 | 05529 | 1 | | 0 | 0 | 0 | 0 | 0 | 0 | 0 | 0 | 0 | 0 | 0 | 0 | 0 | 0 | 0 | 0 | 0 | 1 | 0 | 0 | 0 | 0 | 0 | 0 | 0 | | | | | | |
| 5531 | 06577 | 05530 | 1 | | 0 | 0 | 0 | 0 | 0 | 0 | 0 | 0 | 0 | 0 | 0 | 0 | 0 | 0 | 0 | 0 | 0 | 1 | 0 | 0 | 0 | 0 | 0 | 0 | 0 | | | | | | |
| 5532 | 06578 | 05531 | 1 | | 0 | 0 | 0 | 0 | 0 | 0 | 0 | 0 | 0 | 0 | 0 | 0 | 0 | 0 | 0 | 0 | 0 | 1 | 0 | 0 | 0 | 0 | 0 | 0 | 0 | | | | | | |
| 5533 | 06579 | 05532 | 2 | | 0 | 0 | 0 | 0 | 0 | 0 | 0 | 0 | 0 | 0 | 0 | 0 | 0 | 0 | 0 | 0 | 0 | 2 | 0 | 0 | 0 | 0 | 0 | 0 | 0 | X54925 | 98 | 203 | 1 | 1410 | 1970 |
| 5534 | 06580 | 05533 | 1 | | 0 | 0 | 0 | 0 | 0 | 0 | 0 | 0 | 0 | 0 | 0 | 0 | 0 | 0 | 0 | 0 | 0 | 1 | 0 | 0 | 0 | 0 | 0 | 0 | 0 | | | | | | |
| 5535 | 06581 | 05534 | 1 | | 0 | 0 | 0 | 0 | 0 | 0 | 0 | 0 | 0 | 0 | 0 | 0 | 0 | 0 | 0 | 0 | 0 | 1 | 0 | 0 | 0 | 0 | 0 | 0 | 0 | M58460 | 98.9 | 177 | 1 | 1364 | 1542 |
| 5536 | 06582 | 05535 | 2 | | 0 | 0 | 0 | 0 | 0 | 0 | 0 | 0 | 0 | 0 | 0 | 0 | 0 | 0 | 0 | 0 | 0 | 1 | 0 | 0 | 0 | 1 | 0 | 0 | 0 | | | | | | |
| 5537 | 06583 | 05536 | 3 | | 0 | 0 | 0 | 0 | 0 | 0 | 0 | 0 | 0 | 0 | 0 | 0 | 1 | 0 | 0 | 0 | 0 | 1 | 0 | 0 | 1 | 0 | 0 | 0 | 0 | | | | | | |
| 5538 | 06584 | 05537 | 1 | | 0 | 0 | 0 | 0 | 0 | 0 | 0 | 0 | 0 | 0 | 0 | 0 | 0 | 0 | 0 | 0 | 0 | 1 | 0 | 0 | 0 | 0 | 0 | 0 | 0 | | | | | | |
| 5539 | 06585 | 05538 | 1 | | 0 | 0 | 0 | 0 | 0 | 0 | 0 | 0 | 0 | 0 | 0 | 0 | 0 | 0 | 0 | 0 | 0 | 1 | 0 | 0 | 0 | 0 | 0 | 0 | 0 | | | | | | |
| 5540 | 06586 | 05539 | 1 | | 0 | 0 | 0 | 0 | 0 | 0 | 0 | 0 | 0 | 0 | 0 | 0 | 0 | 0 | 0 | 0 | 0 | 1 | 0 | 0 | 0 | 0 | 0 | 0 | 0 | | | | | | |
| 5541 | 06587 | 05540 | 1 | | 0 | 0 | 0 | 0 | 0 | 0 | 0 | 0 | 0 | 0 | 0 | 0 | 0 | 0 | 0 | 0 | 0 | 1 | 0 | 0 | 0 | 0 | 0 | 0 | 0 | | | | | | |
| 5542 | 06588 | 05541 | 1 | | 0 | 0 | 0 | 0 | 0 | 0 | 0 | 0 | 0 | 0 | 0 | 0 | 0 | 0 | 0 | 0 | 0 | 1 | 0 | 0 | 0 | 0 | 0 | 0 | 0 | | | | | | |
| 5543 | 06590 | 05542 | 3 | | 0 | 0 | 0 | 0 | 0 | 0 | 0 | 0 | 0 | 0 | 0 | 0 | 0 | 0 | 0 | 0 | 0 | 1 | 1 | 0 | 0 | 0 | 0 | 1 | 0 | | | | | | |
| 5544 | 06592 | 05543 | 2 | | 0 | 0 | 0 | 0 | 0 | 0 | 0 | 0 | 0 | 0 | 0 | 0 | 0 | 0 | 0 | 0 | 0 | 2 | 0 | 0 | 0 | 0 | 0 | 0 | 0 | M98479 | 92.4 | 250 | 1 | 1311 | 1557 |
| 5545 | 06593 | 05544 | 1 | | 0 | 0 | 0 | 0 | 0 | 0 | 0 | 0 | 0 | 0 | 0 | 0 | 0 | 0 | 0 | 0 | 0 | 1 | 0 | 0 | 0 | 0 | 0 | 0 | 0 | | | | | | |
| 5546 | 06594 | 05545 | 1 | | 0 | 0 | 0 | 0 | 0 | 0 | 0 | 0 | 0 | 0 | 0 | 0 | 0 | 0 | 0 | 0 | 0 | 1 | 0 | 0 | 0 | 0 | 0 | 0 | 0 | | | | | | |
| 5547 | 06595 | 05546 | 1 | | 0 | 0 | 0 | 0 | 0 | 0 | 0 | 0 | 0 | 0 | 0 | 0 | 0 | 0 | 0 | 0 | 0 | 1 | 0 | 0 | 0 | 0 | 0 | 0 | 0 | | | | | | |
| 5548 | 06596 | 05547 | 2 | | 0 | 0 | 0 | 0 | 0 | 0 | 0 | 0 | 0 | 0 | 0 | 0 | 0 | 0 | 0 | 0 | 0 | 1 | 0 | 0 | 0 | 1 | 0 | 0 | 0 | | | | | | |
| 5549 | 06597 | 05548 | 1 | | 0 | 0 | 0 | 0 | 0 | 0 | 0 | 0 | 0 | 0 | 0 | 0 | 0 | 0 | 0 | 0 | 0 | 1 | 0 | 0 | 0 | 0 | 0 | 0 | 0 | | | | | | |
| 5550 | 06599 | 05549 | 1 | | 0 | 0 | 0 | 0 | 0 | 0 | 0 | 0 | 0 | 0 | 0 | 0 | 0 | 0 | 0 | 0 | 0 | 1 | 0 | 0 | 0 | 0 | 0 | 0 | 0 | | | | | | |
| 5551 | 06600 | 05550 | 1 | | 0 | 0 | 0 | 0 | 0 | 0 | 0 | 0 | 0 | 0 | 0 | 0 | 0 | 0 | 0 | 0 | 0 | 1 | 0 | 0 | 0 | 0 | 0 | 0 | 0 | M14083 | 90.1 | 162 | 1 | 2218 | 2937 |
| 5552 | 06601 | 05551 | 1 | | 0 | 0 | 0 | 0 | 0 | 0 | 0 | 0 | 0 | 0 | 0 | 0 | 0 | 0 | 0 | 0 | 0 | 1 | 0 | 0 | 0 | 0 | 0 | 0 | 0 | | | | | | |
| 5553 | 06602 | 05552 | 1 | | 0 | 0 | 0 | 0 | 0 | 0 | 0 | 0 | 0 | 0 | 0 | 0 | 0 | 0 | 0 | 0 | 0 | 1 | 0 | 0 | 0 | 0 | 0 | 0 | 0 | L07590 | 95.1 | 102 | 1 | 4230 | 5217 |
| 5554 | 06603 | 05553 | 1 | | 0 | 0 | 0 | 0 | 0 | 0 | 0 | 0 | 0 | 0 | 0 | 0 | 0 | 0 | 0 | 0 | 0 | 1 | 0 | 0 | 0 | 0 | 0 | 0 | 0 | | | | | | |
| 5555 | 06604 | 05554 | 2 | | 0 | 0 | 0 | 0 | 0 | 0 | 0 | 0 | 0 | 0 | 0 | 0 | 0 | 0 | 0 | 0 | 0 | 1 | 1 | 0 | 0 | 0 | 0 | 0 | 0 | | | | | | |
| 5556 | 06605 | 05555 | 1 | | 0 | 0 | 0 | 0 | 0 | 0 | 0 | 0 | 0 | 0 | 0 | 0 | 0 | 0 | 0 | 0 | 0 | 1 | 0 | 0 | 0 | 0 | 0 | 0 | 0 | | | | | | |
| 5557 | 06606 | 05556 | 2 | | 0 | 0 | 0 | 0 | 0 | 0 | 0 | 0 | 0 | 0 | 0 | 0 | 0 | 0 | 0 | 0 | 0 | 1 | 1 | 0 | 0 | 0 | 0 | 0 | 0 | | | | | | |
| 5558 | 06607 | 05557 | 2 | | 0 | 0 | 0 | 0 | 0 | 0 | 0 | 0 | 0 | 0 | 0 | 0 | 0 | 0 | 0 | 0 | 0 | 2 | 0 | 0 | 0 | 0 | 0 | 0 | 0 | | | | | | |
| 5559 | 06608 | 05558 | 1 | | 0 | 0 | 0 | 0 | 0 | 0 | 0 | 0 | 0 | 0 | 0 | 0 | 0 | 0 | 0 | 0 | 0 | 1 | 0 | 0 | 0 | 0 | 0 | 0 | 0 | | | | | | |
| 5560 | 06609 | 05559 | 1 | | 0 | 0 | 0 | 0 | 0 | 0 | 0 | 0 | 0 | 0 | 0 | 0 | 0 | 0 | 0 | 0 | 0 | 1 | 0 | 0 | 0 | 0 | 0 | 0 | 0 | M10036 | 95 | 80 | 43 | 1483 | 1835 |
| 5561 | 06610 | 05560 | 2 | | 0 | 0 | 0 | 0 | 0 | 0 | 0 | 0 | 0 | 0 | 0 | 0 | 0 | 0 | 0 | 0 | 0 | 1 | 0 | 0 | 0 | 0 | 1 | 0 | 0 | | | | | | |
| 5562 | 06611 | 05561 | 1 | | 0 | 0 | 0 | 0 | 0 | 0 | 0 | 0 | 0 | 0 | 0 | 0 | 0 | 0 | 0 | 0 | 0 | 1 | 0 | 0 | 0 | 0 | 0 | 0 | 0 | L22569 | 91.5 | 130 | 1 | 971 | 2286 |
| 5563 | 06612 | 05562 | 1 | | 0 | 0 | 0 | 0 | 0 | 0 | 0 | 0 | 0 | 0 | 0 | 0 | 0 | 0 | 0 | 0 | 0 | 1 | 0 | 0 | 0 | 0 | 0 | 0 | 0 | | | | | | |
| 5564 | 06613 | 05563 | 2 | | 0 | 0 | 0 | 0 | 0 | 0 | 0 | 0 | 0 | 0 | 0 | 0 | 0 | 0 | 0 | 0 | 0 | 1 | 0 | 0 | 0 | 1 | 0 | 0 | 0 | | | | | | |
| 5565 | 06614 | 05564 | 1 | | 0 | 0 | 0 | 0 | 0 | 0 | 0 | 0 | 0 | 0 | 0 | 0 | 0 | 0 | 0 | 0 | 0 | 1 | 0 | 0 | 0 | 0 | 0 | 0 | 0 | | | | | | |

Table 155

| | A | B | C | E | G | I | K | M | O | Q | S | U | W | Y | AA | AC | AE | AG | AI | AK | AM | AO | AQ | AS | AU | AW | AY | BA | BC | BE | BF | BG | BH | BI | BK |
|---|---|---|---|---|---|---|---|---|---|---|---|---|---|---|---|---|---|---|---|---|---|---|---|---|---|---|---|---|---|---|---|---|---|---|---|
| 5566 | 06615 | 05565 | 1 | | 0 | 0 | 0 | 0 | 0 | 0 | 0 | 0 | 0 | 0 | 0 | 0 | 0 | 0 | 0 | 0 | 0 | 1 | 0 | 0 | 0 | 0 | 0 | 0 | 0 | | | | | | |
| 5567 | 06616 | 05566 | 1 | | 0 | 0 | 0 | 0 | 0 | 0 | 0 | 0 | 0 | 0 | 0 | 0 | 0 | 0 | 0 | 0 | 0 | 1 | 0 | 0 | 0 | 0 | 0 | 0 | 0 | | | | | | |
| 5568 | 06617 | 05567 | 1 | | 0 | 0 | 0 | 0 | 0 | 0 | 0 | 0 | 0 | 0 | 0 | 0 | 0 | 0 | 0 | 0 | 0 | 1 | 0 | 0 | 0 | 0 | 0 | 0 | 0 | | | | | | |
| 5569 | 06618 | 05568 | 2 | | 0 | 0 | 0 | 0 | 0 | 0 | 0 | 0 | 0 | 0 | 0 | 0 | 0 | 0 | 0 | 0 | 0 | 1 | 0 | 0 | 1 | 0 | 0 | 0 | 0 | L11005 | 98.7 | 76 | 1 | 4882 | 4957 |
| 5570 | 06619 | 05569 | 1 | | 0 | 0 | 0 | 0 | 0 | 0 | 0 | 0 | 0 | 0 | 0 | 0 | 0 | 0 | 0 | 0 | 0 | 1 | 0 | 0 | 0 | 0 | 0 | 0 | 0 | | | | | | |
| 5571 | 06620 | 05570 | 1 | | 0 | 0 | 0 | 0 | 0 | 0 | 0 | 0 | 0 | 0 | 0 | 0 | 0 | 0 | 0 | 0 | 0 | 1 | 0 | 0 | 0 | 0 | 0 | 0 | 0 | | | | | | |
| 5572 | 06621 | 05571 | 1 | | 0 | 0 | 0 | 0 | 0 | 0 | 0 | 0 | 0 | 0 | 0 | 0 | 0 | 0 | 0 | 0 | 0 | 1 | 0 | 0 | 0 | 0 | 0 | 0 | 0 | | | | | | |
| 5573 | 06623 | 05572 | 1 | | 0 | 0 | 0 | 0 | 0 | 0 | 0 | 0 | 0 | 0 | 0 | 0 | 0 | 0 | 0 | 0 | 0 | 1 | 0 | 0 | 0 | 0 | 0 | 0 | 0 | | | | | | |
| 5574 | 06624 | 05573 | 2 | | 0 | 0 | 0 | 0 | 0 | 0 | 0 | 0 | 0 | 0 | 0 | 0 | 0 | 0 | 0 | 0 | 0 | 1 | 0 | 0 | 1 | 0 | 0 | 0 | 0 | | | | | | |
| 5575 | 06625 | 05574 | 1 | | 0 | 0 | 0 | 0 | 0 | 0 | 0 | 0 | 0 | 0 | 0 | 0 | 0 | 0 | 0 | 0 | 0 | 1 | 0 | 0 | 0 | 0 | 0 | 0 | 0 | | | | | | |
| 5576 | 06626 | 05575 | 1 | | 0 | 0 | 0 | 0 | 0 | 0 | 0 | 0 | 0 | 0 | 0 | 0 | 0 | 0 | 0 | 0 | 0 | 1 | 0 | 0 | 0 | 0 | 0 | 0 | 0 | M20472 | 95.4 | 131 | 1 | 383 | 1023 |
| 5577 | 06627 | 05576 | 1 | | 0 | 0 | 0 | 0 | 0 | 0 | 0 | 0 | 0 | 0 | 0 | 0 | 0 | 0 | 0 | 0 | 0 | 1 | 0 | 0 | 0 | 0 | 0 | 0 | 0 | | | | | | |
| 5578 | 06628 | 05577 | 1 | | 0 | 0 | 0 | 0 | 0 | 0 | 0 | 0 | 0 | 0 | 0 | 0 | 0 | 0 | 0 | 0 | 0 | 1 | 0 | 0 | 0 | 0 | 0 | 0 | 0 | | | | | | |
| 5579 | 06630 | 05578 | 1 | | 0 | 0 | 0 | 0 | 0 | 0 | 0 | 0 | 0 | 0 | 0 | 0 | 0 | 0 | 0 | 0 | 0 | 1 | 0 | 0 | 0 | 0 | 0 | 0 | 0 | X66363 | 95.1 | 184 | 1 | 1479 | 1745 |
| 5580 | 06631 | 05579 | 1 | | 0 | 0 | 0 | 0 | 0 | 0 | 0 | 0 | 0 | 0 | 0 | 0 | 0 | 0 | 0 | 0 | 0 | 1 | 0 | 0 | 0 | 0 | 0 | 0 | 0 | | | | | | |
| 5581 | 06632 | 05580 | 1 | | 0 | 0 | 0 | 0 | 0 | 0 | 0 | 0 | 0 | 0 | 0 | 0 | 0 | 0 | 0 | 0 | 0 | 1 | 0 | 0 | 0 | 0 | 0 | 0 | 0 | | | | | | |
| 5582 | 06633 | 05581 | 1 | | 0 | 0 | 0 | 0 | 0 | 0 | 0 | 0 | 0 | 0 | 0 | 0 | 0 | 0 | 0 | 0 | 0 | 1 | 0 | 0 | 0 | 0 | 0 | 0 | 0 | | | | | | |
| 5583 | 06634 | 05582 | 1 | | 0 | 0 | 0 | 0 | 0 | 0 | 0 | 0 | 0 | 0 | 0 | 0 | 0 | 0 | 0 | 0 | 0 | 1 | 0 | 0 | 0 | 0 | 0 | 0 | 0 | | | | | | |
| 5584 | 06635 | 05583 | 2 | | 0 | 0 | 0 | 0 | 0 | 0 | 0 | 0 | 0 | 0 | 1 | 0 | 0 | 0 | 0 | 0 | 0 | 1 | 0 | 0 | 0 | 0 | 0 | 0 | 0 | | | | | | |
| 5585 | 06636 | 05584 | 2 | | 0 | 0 | 0 | 0 | 0 | 0 | 0 | 0 | 0 | 0 | 0 | 0 | 0 | 0 | 0 | 0 | 0 | 2 | 0 | 0 | 0 | 0 | 0 | 0 | 0 | X53743 | 97.6 | 167 | 1 | 1991 | 2156 |
| 5586 | 06637 | 05585 | 1 | | 0 | 0 | 0 | 0 | 0 | 0 | 0 | 0 | 0 | 0 | 0 | 0 | 0 | 0 | 0 | 0 | 0 | 1 | 0 | 0 | 0 | 0 | 0 | 0 | 0 | | | | | | |
| 5587 | 06638 | 05586 | 1 | | 0 | 0 | 0 | 0 | 0 | 0 | 0 | 0 | 0 | 0 | 0 | 0 | 0 | 0 | 0 | 0 | 0 | 1 | 0 | 0 | 0 | 0 | 0 | 0 | 0 | | | | | | |
| 5588 | 06639 | 05587 | 2 | | 0 | 0 | 0 | 0 | 0 | 0 | 0 | 0 | 0 | 0 | 0 | 0 | 0 | 0 | 0 | 0 | 0 | 2 | 0 | 0 | 0 | 0 | 0 | 0 | 0 | M38258 | 94.9 | 253 | 1 | 2320 | 2571 |
| 5589 | 06640 | 05588 | 1 | | 0 | 0 | 0 | 0 | 0 | 0 | 0 | 0 | 0 | 0 | 0 | 0 | 0 | 0 | 0 | 0 | 0 | 1 | 0 | 0 | 0 | 0 | 0 | 0 | 0 | | | | | | |
| 5590 | 06641 | 05589 | 1 | | 0 | 0 | 0 | 0 | 0 | 0 | 0 | 0 | 0 | 0 | 0 | 0 | 0 | 0 | 0 | 0 | 0 | 1 | 0 | 0 | 0 | 0 | 0 | 0 | 0 | | | | | | |
| 5591 | 06642 | 05590 | 1 | | 0 | 0 | 0 | 0 | 0 | 0 | 0 | 0 | 0 | 0 | 0 | 0 | 0 | 0 | 0 | 0 | 0 | 1 | 0 | 0 | 0 | 0 | 0 | 0 | 0 | | | | | | |
| 5592 | 06643 | 05591 | 1 | | 0 | 0 | 0 | 0 | 0 | 0 | 0 | 0 | 0 | 0 | 0 | 0 | 0 | 0 | 0 | 0 | 0 | 1 | 0 | 0 | 0 | 0 | 0 | 0 | 0 | | | | | | |
| 5593 | 06645 | 05592 | 1 | | 0 | 0 | 0 | 0 | 0 | 0 | 0 | 0 | 0 | 0 | 0 | 0 | 0 | 0 | 0 | 0 | 0 | 1 | 0 | 0 | 0 | 0 | 0 | 0 | 0 | | | | | | |
| 5594 | 06646 | 05593 | 1 | | 0 | 0 | 0 | 0 | 0 | 0 | 0 | 0 | 0 | 0 | 0 | 0 | 0 | 0 | 0 | 0 | 0 | 1 | 0 | 0 | 0 | 0 | 0 | 0 | 0 | X52678 | 95.8 | 96 | 1 | 1593 | 1879 |
| 5595 | 06647 | 05594 | 1 | | 0 | 0 | 0 | 0 | 0 | 0 | 0 | 0 | 0 | 0 | 0 | 0 | 0 | 0 | 0 | 0 | 0 | 1 | 0 | 0 | 0 | 0 | 0 | 0 | 0 | | | | | | |
| 5596 | 06649 | 05595 | 1 | | 0 | 0 | 0 | 0 | 0 | 0 | 0 | 0 | 0 | 0 | 0 | 0 | 0 | 0 | 0 | 0 | 0 | 1 | 0 | 0 | 0 | 0 | 0 | 0 | 0 | | | | | | |
| 5597 | 06650 | 05596 | 2 | | 0 | 0 | 0 | 0 | 0 | 0 | 0 | 0 | 0 | 0 | 0 | 0 | 0 | 0 | 0 | 0 | 0 | 1 | 0 | 1 | 0 | 0 | 0 | 0 | 0 | | | | | | |
| 5598 | 06651 | 05597 | 1 | | 0 | 0 | 0 | 0 | 0 | 0 | 0 | 0 | 0 | 0 | 0 | 0 | 0 | 0 | 0 | 0 | 0 | 1 | 0 | 0 | 0 | 0 | 0 | 0 | 0 | | | | | | |
| 5599 | 06652 | 05598 | 1 | | 0 | 0 | 0 | 0 | 0 | 0 | 0 | 0 | 0 | 0 | 0 | 0 | 0 | 0 | 0 | 0 | 0 | 1 | 0 | 0 | 0 | 0 | 0 | 0 | 0 | | | | | | |
| 5600 | 06653 | 05599 | 1 | | 0 | 0 | 0 | 0 | 0 | 0 | 0 | 0 | 0 | 0 | 0 | 0 | 0 | 0 | 0 | 0 | 0 | 1 | 0 | 0 | 0 | 0 | 0 | 0 | 0 | M81757 | 98.6 | 69 | 17 | 432 | 510 |
| 5601 | 06654 | 05600 | 1 | | 0 | 0 | 0 | 0 | 0 | 0 | 0 | 0 | 0 | 0 | 0 | 0 | 0 | 0 | 0 | 0 | 0 | 1 | 0 | 0 | 0 | 0 | 0 | 0 | 0 | | | | | | |

Table 156

| | A | B | C | E | G | I | K | M | O | Q | S | U | W | Y | AA | AC | AE | AG | AI | AK | AM | AO | AQ | AS | AU | AW | AY | BA | BC | BE | BF | BG | BH | BI | BK |
|---|---|---|---|---|---|---|---|---|---|---|---|---|---|---|---|---|---|---|---|---|---|---|---|---|---|---|---|---|---|---|---|---|---|---|---|
| 5602 | 06656 | 05601 | 1 | | 0 | 0 | 0 | 0 | 0 | 0 | 0 | 0 | 0 | 0 | 0 | 0 | 0 | 0 | 0 | 0 | 0 | 1 | 0 | 0 | 0 | 0 | 0 | 0 | 0 | | | | | | |
| 5603 | 06659 | 05602 | 2 | | 0 | 0 | 0 | 0 | 0 | 0 | 0 | 0 | 0 | 0 | 0 | 0 | 0 | 0 | 0 | 0 | 0 | 1 | 0 | 0 | 0 | 0 | 0 | 1 | 0 | | | | | | |
| 5604 | 06660 | 05603 | 1 | | 0 | 0 | 0 | 0 | 0 | 0 | 0 | 0 | 0 | 0 | 0 | 0 | 0 | 0 | 0 | 0 | 0 | 1 | 0 | 0 | 0 | 0 | 0 | 0 | 0 | | | | | | |
| 5605 | 06661 | 05604 | 2 | | 0 | 0 | 0 | 0 | 0 | 0 | 0 | 0 | 0 | 0 | 0 | 0 | 0 | 0 | 0 | 0 | 0 | 1 | 0 | 0 | 0 | 1 | 0 | 0 | 0 | | | | | | |
| 5606 | 06662 | 05605 | 1 | | 0 | 0 | 0 | 0 | 0 | 0 | 0 | 0 | 0 | 0 | 0 | 0 | 0 | 0 | 0 | 0 | 0 | 1 | 0 | 0 | 0 | 0 | 0 | 0 | 0 | | | | | | |
| 5607 | 06664 | 05606 | 3 | | 0 | 0 | 0 | 0 | 0 | 0 | 0 | 0 | 0 | 0 | 1 | 0 | 0 | 0 | 0 | 0 | 0 | 1 | 0 | 0 | 1 | 0 | 0 | 0 | 0 | | | | | | |
| 5608 | 06666 | 05607 | 2 | | 0 | 0 | 0 | 0 | 0 | 0 | 0 | 0 | 0 | 0 | 2 | 0 | 0 | 0 | 0 | 0 | 0 | 0 | 0 | 0 | 0 | 0 | 0 | 0 | 0 | J05016 | 91.6 | 215 | 1 | 1912 | 2865 |
| 5609 | 06667 | 05608 | 1 | | 0 | 0 | 0 | 0 | 0 | 0 | 0 | 0 | 0 | 0 | 1 | 0 | 0 | 0 | 0 | 0 | 0 | 0 | 0 | 0 | 0 | 0 | 0 | 0 | 0 | | | | | | |
| 5610 | 06668 | 05609 | 1 | | 0 | 0 | 0 | 0 | 0 | 0 | 0 | 0 | 0 | 0 | 1 | 0 | 0 | 0 | 0 | 0 | 0 | 0 | 0 | 0 | 0 | 0 | 0 | 0 | 0 | | | | | | |
| 5611 | 06669 | 05610 | 1 | | 0 | 0 | 0 | 0 | 0 | 0 | 0 | 0 | 0 | 0 | 1 | 0 | 0 | 0 | 0 | 0 | 0 | 0 | 0 | 0 | 0 | 0 | 0 | 0 | 0 | | | | | | |
| 5612 | 06670 | 05611 | 1 | | 0 | 0 | 0 | 0 | 0 | 0 | 0 | 0 | 0 | 0 | 1 | 0 | 0 | 0 | 0 | 0 | 0 | 0 | 0 | 0 | 0 | 0 | 0 | 0 | 0 | | | | | | |
| 5613 | 06672 | 05612 | 1 | | 0 | 0 | 0 | 0 | 0 | 0 | 0 | 0 | 0 | 0 | 1 | 0 | 0 | 0 | 0 | 0 | 0 | 0 | 0 | 0 | 0 | 0 | 0 | 0 | 0 | | | | | | |
| 5614 | 06673 | 05613 | 1 | | 0 | 0 | 0 | 0 | 0 | 0 | 0 | 0 | 0 | 0 | 1 | 0 | 0 | 0 | 0 | 0 | 0 | 0 | 0 | 0 | 0 | 0 | 0 | 0 | 0 | | | | | | |
| 5615 | 06674 | 05614 | 1 | | 0 | 0 | 0 | 0 | 0 | 0 | 0 | 0 | 0 | 0 | 1 | 0 | 0 | 0 | 0 | 0 | 0 | 0 | 0 | 0 | 0 | 0 | 0 | 0 | 0 | | | | | | |
| 5616 | 06675 | 05615 | 1 | | 0 | 0 | 0 | 0 | 0 | 0 | 0 | 0 | 0 | 0 | 1 | 0 | 0 | 0 | 0 | 0 | 0 | 0 | 0 | 0 | 0 | 0 | 0 | 0 | 0 | | | | | | |
| 5617 | 06676 | 05616 | 2 | | 0 | 0 | 0 | 0 | 0 | 0 | 0 | 0 | 0 | 0 | 2 | 0 | 0 | 0 | 0 | 0 | 0 | 0 | 0 | 0 | 0 | 0 | 0 | 0 | 0 | | | | | | |
| 5618 | 06677 | 05617 | 1 | | 0 | 0 | 0 | 0 | 0 | 0 | 0 | 0 | 0 | 0 | 1 | 0 | 0 | 0 | 0 | 0 | 0 | 0 | 0 | 0 | 0 | 0 | 0 | 0 | 0 | | | | | | |
| 5619 | 06678 | 05618 | 1 | | 0 | 0 | 0 | 0 | 0 | 0 | 0 | 0 | 0 | 0 | 1 | 0 | 0 | 0 | 0 | 0 | 0 | 0 | 0 | 0 | 0 | 0 | 0 | 0 | 0 | | | | | | |
| 5620 | 06679 | 05619 | 1 | | 0 | 0 | 0 | 0 | 0 | 0 | 0 | 0 | 0 | 0 | 1 | 0 | 0 | 0 | 0 | 0 | 0 | 0 | 0 | 0 | 0 | 0 | 0 | 0 | 0 | | | | | | |
| 5621 | 06680 | 05620 | 1 | | 0 | 0 | 0 | 0 | 0 | 0 | 0 | 0 | 0 | 0 | 1 | 0 | 0 | 0 | 0 | 0 | 0 | 0 | 0 | 0 | 0 | 0 | 0 | 0 | 0 | | | | | | |
| 5622 | 06681 | 05621 | 1 | | 0 | 0 | 0 | 0 | 0 | 0 | 0 | 0 | 0 | 0 | 1 | 0 | 0 | 0 | 0 | 0 | 0 | 0 | 0 | 0 | 0 | 0 | 0 | 0 | 0 | | | | | | |
| 5623 | 06682 | 05622 | 1 | | 0 | 0 | 0 | 0 | 0 | 0 | 0 | 0 | 0 | 0 | 1 | 0 | 0 | 0 | 0 | 0 | 0 | 0 | 0 | 0 | 0 | 0 | 0 | 0 | 0 | | | | | | |
| 5624 | 06683 | 05623 | 2 | | 0 | 0 | 0 | 0 | 0 | 0 | 0 | 0 | 0 | 0 | 2 | 0 | 0 | 0 | 0 | 0 | 0 | 0 | 0 | 0 | 0 | 0 | 0 | 0 | 0 | | | | | | |
| 5625 | 06684 | 05624 | 1 | | 0 | 0 | 0 | 0 | 0 | 0 | 0 | 0 | 0 | 0 | 1 | 0 | 0 | 0 | 0 | 0 | 0 | 0 | 0 | 0 | 0 | 0 | 0 | 0 | 0 | | | | | | |
| 5626 | 06685 | 05625 | 1 | | 0 | 0 | 0 | 0 | 0 | 0 | 0 | 0 | 0 | 0 | 1 | 0 | 0 | 0 | 0 | 0 | 0 | 0 | 0 | 0 | 0 | 0 | 0 | 0 | 0 | | | | | | |
| 5627 | 06686 | 05626 | 1 | | 0 | 0 | 0 | 0 | 0 | 0 | 0 | 0 | 0 | 0 | 1 | 0 | 0 | 0 | 0 | 0 | 0 | 0 | 0 | 0 | 0 | 0 | 0 | 0 | 0 | | | | | | |
| 5628 | 06687 | 05627 | 1 | | 0 | 0 | 0 | 0 | 0 | 0 | 0 | 0 | 0 | 0 | 1 | 0 | 0 | 0 | 0 | 0 | 0 | 0 | 0 | 0 | 0 | 0 | 0 | 0 | 0 | | | | | | |
| 5629 | 06688 | 05628 | 1 | | 0 | 0 | 0 | 0 | 0 | 0 | 0 | 0 | 0 | 0 | 1 | 0 | 0 | 0 | 0 | 0 | 0 | 0 | 0 | 0 | 0 | 0 | 0 | 0 | 0 | | | | | | |
| 5630 | 06689 | 05629 | 3 | | 0 | 0 | 0 | 0 | 0 | 0 | 0 | 0 | 0 | 0 | 1 | 0 | 0 | 0 | 0 | 0 | 0 | 0 | 0 | 0 | 0 | 0 | 2 | 0 | 0 | | | | | | |
| 5631 | 06690 | 05630 | 1 | | 0 | 0 | 0 | 0 | 0 | 0 | 0 | 0 | 0 | 0 | 1 | 0 | 0 | 0 | 0 | 0 | 0 | 0 | 0 | 0 | 0 | 0 | 0 | 0 | 0 | | | | | | |
| 5632 | 06691 | 05631 | 1 | | 0 | 0 | 0 | 0 | 0 | 0 | 0 | 0 | 0 | 0 | 1 | 0 | 0 | 0 | 0 | 0 | 0 | 0 | 0 | 0 | 0 | 0 | 0 | 0 | 0 | | | | | | |
| 5633 | 06692 | 05632 | 1 | | 0 | 0 | 0 | 0 | 0 | 0 | 0 | 0 | 0 | 0 | 1 | 0 | 0 | 0 | 0 | 0 | 0 | 0 | 0 | 0 | 0 | 0 | 0 | 0 | 0 | | | | | | |
| 5634 | 06693 | 05633 | 1 | | 0 | 0 | 0 | 0 | 0 | 0 | 0 | 0 | 0 | 0 | 1 | 0 | 0 | 0 | 0 | 0 | 0 | 0 | 0 | 0 | 0 | 0 | 0 | 0 | 0 | | | | | | |
| 5635 | 06694 | 05634 | 1 | | 0 | 0 | 0 | 0 | 0 | 0 | 0 | 0 | 0 | 0 | 1 | 0 | 0 | 0 | 0 | 0 | 0 | 0 | 0 | 0 | 0 | 0 | 0 | 0 | 0 | | | | | | |
| 5636 | 06696 | 05635 | 1 | | 0 | 0 | 0 | 0 | 0 | 0 | 0 | 0 | 0 | 0 | 1 | 0 | 0 | 0 | 0 | 0 | 0 | 0 | 0 | 0 | 0 | 0 | 0 | 0 | 0 | | | | | | |
| 5637 | 06697 | 05636 | 1 | | 0 | 0 | 0 | 0 | 0 | 0 | 0 | 0 | 0 | 0 | 1 | 0 | 0 | 0 | 0 | 0 | 0 | 0 | 0 | 0 | 0 | 0 | 0 | 0 | 0 | | | | | | |

Table 157

| | A | B | C | E | G | I | K | M | O | Q | S | U | W | Y | AA | AC | AE | AG | AI | AK | AM | AO | AQ | AS | AU | AW | AY | BA | BC | BE | BF | BG | BH | BI | BK |
|---|---|---|---|---|---|---|---|---|---|---|---|---|---|---|---|---|---|---|---|---|---|---|---|---|---|---|---|---|---|---|---|---|---|---|---|
| 5638 | 06698 | 05637 | 2 | | 0 | 0 | 0 | 0 | 0 | 0 | 0 | 0 | 0 | 0 | 1 | 0 | 0 | 0 | 0 | 0 | 0 | 0 | 0 | 0 | 1 | 0 | 0 | 0 | 0 | | | | | | |
| 5639 | 06699 | 05638 | 1 | | 0 | 0 | 0 | 0 | 0 | 0 | 0 | 0 | 0 | 0 | 1 | 0 | 0 | 0 | 0 | 0 | 0 | 0 | 0 | 0 | 0 | 0 | 0 | 0 | 0 | | | | | | |
| 5640 | 06700 | 05639 | 2 | | 0 | 0 | 0 | 0 | 0 | 0 | 0 | 0 | 0 | 0 | 1 | 0 | 0 | 0 | 0 | 0 | 0 | 0 | 0 | 0 | 0 | 0 | 1 | 0 | 0 | | | | | | |
| 5641 | 06701 | 05640 | 5 | | 0 | 0 | 0 | 0 | 0 | 0 | 0 | 0 | 0 | 0 | 1 | 0 | 0 | 0 | 0 | 0 | 0 | 0 | 0 | 0 | 0 | 0 | 3 | 1 | 0 | | | | | | |
| 5642 | 06702 | 05641 | 1 | | 0 | 0 | 0 | 0 | 0 | 0 | 0 | 0 | 0 | 0 | 1 | 0 | 0 | 0 | 0 | 0 | 0 | 0 | 0 | 0 | 0 | 0 | 0 | 0 | 0 | | | | | | |
| 5643 | 06703 | 05642 | 1 | | 0 | 0 | 0 | 0 | 0 | 0 | 0 | 0 | 0 | 0 | 1 | 0 | 0 | 0 | 0 | 0 | 0 | 0 | 0 | 0 | 0 | 0 | 0 | 0 | 0 | | | | | | |
| 5644 | 06704 | 05643 | 1 | | 0 | 0 | 0 | 0 | 0 | 0 | 0 | 0 | 0 | 0 | 1 | 0 | 0 | 0 | 0 | 0 | 0 | 0 | 0 | 0 | 0 | 0 | 0 | 0 | 0 | | | | | | |
| 5645 | 06705 | 05644 | 2 | | 0 | 0 | 0 | 0 | 0 | 0 | 0 | 0 | 0 | 0 | 1 | 0 | 0 | 0 | 0 | 0 | 0 | 0 | 0 | 0 | 1 | 0 | 0 | 0 | 0 | | | | | | |
| 5646 | 06706 | 05645 | 1 | | 0 | 0 | 0 | 0 | 0 | 0 | 0 | 0 | 0 | 0 | 1 | 0 | 0 | 0 | 0 | 0 | 0 | 0 | 0 | 0 | 0 | 0 | 0 | 0 | 0 | | | | | | |
| 5647 | 06707 | 05646 | 1 | | 0 | 0 | 0 | 0 | 0 | 0 | 0 | 0 | 0 | 0 | 1 | 0 | 0 | 0 | 0 | 0 | 0 | 0 | 0 | 0 | 0 | 0 | 0 | 0 | 0 | | | | | | |
| 5648 | 06709 | 05647 | 1 | | 0 | 0 | 0 | 0 | 0 | 0 | 0 | 0 | 0 | 0 | 1 | 0 | 0 | 0 | 0 | 0 | 0 | 0 | 0 | 0 | 0 | 0 | 0 | 0 | 0 | L15388 | 95.7 | 369 | 1 | 1826 | 2557 |
| 5649 | 06710 | 05648 | 2 | | 0 | 0 | 0 | 0 | 0 | 0 | 0 | 0 | 0 | 0 | 1 | 0 | 0 | 0 | 0 | 0 | 0 | 0 | 0 | 0 | 0 | 0 | 0 | 0 | 1 | | | | | | |
| 5650 | 06711 | 05649 | 1 | | 0 | 0 | 0 | 0 | 0 | 0 | 0 | 0 | 0 | 0 | 1 | 0 | 0 | 0 | 0 | 0 | 0 | 0 | 0 | 0 | 0 | 0 | 0 | 0 | 0 | | | | | | |
| 5651 | 06712 | 05650 | 1 | | 0 | 0 | 0 | 0 | 0 | 0 | 0 | 0 | 0 | 0 | 1 | 0 | 0 | 0 | 0 | 0 | 0 | 0 | 0 | 0 | 0 | 0 | 0 | 0 | 0 | | | | | | |
| 5652 | 06713 | 05651 | 1 | | 0 | 0 | 0 | 0 | 0 | 0 | 0 | 0 | 0 | 0 | 1 | 0 | 0 | 0 | 0 | 0 | 0 | 0 | 0 | 0 | 0 | 0 | 0 | 0 | 0 | | | | | | |
| 5653 | 06714 | 05652 | 1 | | 0 | 0 | 0 | 0 | 0 | 0 | 0 | 0 | 0 | 0 | 1 | 0 | 0 | 0 | 0 | 0 | 0 | 0 | 0 | 0 | 0 | 0 | 0 | 0 | 0 | X15187 | 97.8 | 231 | 1 | 2537 | 2780 |
| 5654 | 06715 | 05653 | 1 | | 0 | 0 | 0 | 0 | 0 | 0 | 0 | 0 | 0 | 0 | 1 | 0 | 0 | 0 | 0 | 0 | 0 | 0 | 0 | 0 | 0 | 0 | 0 | 0 | 0 | | | | | | |
| 5655 | 06716 | 05654 | 1 | | 0 | 0 | 0 | 0 | 0 | 0 | 0 | 0 | 0 | 0 | 1 | 0 | 0 | 0 | 0 | 0 | 0 | 0 | 0 | 0 | 0 | 0 | 0 | 0 | 0 | | | | | | |
| 5656 | 06717 | 05655 | 1 | | 0 | 0 | 0 | 0 | 0 | 0 | 0 | 0 | 0 | 0 | 1 | 0 | 0 | 0 | 0 | 0 | 0 | 0 | 0 | 0 | 0 | 0 | 0 | 0 | 0 | | | | | | |
| 5657 | 06718 | 05656 | 2 | | 0 | 0 | 0 | 0 | 0 | 0 | 0 | 0 | 0 | 0 | 2 | 0 | 0 | 0 | 0 | 0 | 0 | 0 | 0 | 0 | 0 | 0 | 0 | 0 | 0 | | | | | | |
| 5658 | 06719 | 05657 | 1 | | 0 | 0 | 0 | 0 | 0 | 0 | 0 | 0 | 0 | 0 | 1 | 0 | 0 | 0 | 0 | 0 | 0 | 0 | 0 | 0 | 0 | 0 | 0 | 0 | 0 | | | | | | |
| 5659 | 06720 | 05658 | 1 | | 0 | 0 | 0 | 0 | 0 | 0 | 0 | 0 | 0 | 0 | 1 | 0 | 0 | 0 | 0 | 0 | 0 | 0 | 0 | 0 | 0 | 0 | 0 | 0 | 0 | | | | | | |
| 5660 | 06721 | 05659 | 1 | | 0 | 0 | 0 | 0 | 0 | 0 | 0 | 0 | 0 | 0 | 1 | 0 | 0 | 0 | 0 | 0 | 0 | 0 | 0 | 0 | 0 | 0 | 0 | 0 | 0 | | | | | | |
| 5661 | 06724 | 05660 | 1 | | 0 | 0 | 0 | 0 | 0 | 0 | 0 | 0 | 0 | 0 | 1 | 0 | 0 | 0 | 0 | 0 | 0 | 0 | 0 | 0 | 0 | 0 | 0 | 0 | 0 | | | | | | |
| 5662 | 06725 | 05661 | 1 | | 0 | 0 | 0 | 0 | 0 | 0 | 0 | 0 | 0 | 0 | 1 | 0 | 0 | 0 | 0 | 0 | 0 | 0 | 0 | 0 | 0 | 0 | 0 | 0 | 0 | | | | | | |
| 5663 | 06726 | 05662 | 3 | | 0 | 0 | 0 | 0 | 0 | 0 | 0 | 0 | 0 | 0 | 2 | 0 | 0 | 0 | 0 | 0 | 0 | 0 | 0 | 1 | 0 | 0 | 0 | 0 | 0 | | | | | | |
| 5664 | 06727 | 05663 | 1 | | 0 | 0 | 0 | 0 | 0 | 0 | 0 | 0 | 0 | 0 | 1 | 0 | 0 | 0 | 0 | 0 | 0 | 0 | 0 | 0 | 0 | 0 | 0 | 0 | 0 | | | | | | |
| 5665 | 06728 | 05664 | 1 | | 0 | 0 | 0 | 0 | 0 | 0 | 0 | 0 | 0 | 0 | 1 | 0 | 0 | 0 | 0 | 0 | 0 | 0 | 0 | 0 | 0 | 0 | 0 | 0 | 0 | | | | | | |
| 5666 | 06730 | 05665 | 1 | | 0 | 0 | 0 | 0 | 0 | 0 | 0 | 0 | 0 | 0 | 1 | 0 | 0 | 0 | 0 | 0 | 0 | 0 | 0 | 0 | 0 | 0 | 0 | 0 | 0 | | | | | | |
| 5667 | 06731 | 05666 | 1 | | 0 | 0 | 0 | 0 | 0 | 0 | 0 | 0 | 0 | 0 | 1 | 0 | 0 | 0 | 0 | 0 | 0 | 0 | 0 | 0 | 0 | 0 | 0 | 0 | 0 | | | | | | |
| 5668 | 06732 | 05667 | 1 | | 0 | 0 | 0 | 0 | 0 | 0 | 0 | 0 | 0 | 0 | 1 | 0 | 0 | 0 | 0 | 0 | 0 | 0 | 0 | 0 | 0 | 0 | 0 | 0 | 0 | | | | | | |
| 5669 | 06733 | 05668 | 1 | | 0 | 0 | 0 | 0 | 0 | 0 | 0 | 0 | 0 | 0 | 1 | 0 | 0 | 0 | 0 | 0 | 0 | 0 | 0 | 0 | 0 | 0 | 0 | 0 | 0 | | | | | | |
| 5670 | 06734 | 05669 | 1 | | 0 | 0 | 0 | 0 | 0 | 0 | 0 | 0 | 0 | 0 | 1 | 0 | 0 | 0 | 0 | 0 | 0 | 0 | 0 | 0 | 0 | 0 | 0 | 0 | 0 | | | | | | |
| 5671 | 06735 | 05670 | 1 | | 0 | 0 | 0 | 0 | 0 | 0 | 0 | 0 | 0 | 0 | 1 | 0 | 0 | 0 | 0 | 0 | 0 | 0 | 0 | 0 | 0 | 0 | 0 | 0 | 0 | | | | | | |
| 5672 | 06736 | 05671 | 2 | | 0 | 0 | 0 | 0 | 0 | 0 | 0 | 0 | 0 | 0 | 1 | 0 | 0 | 0 | 0 | 0 | 0 | 0 | 0 | 0 | 0 | 0 | 1 | 0 | 0 | | | | | | |
| 5673 | 06737 | 05672 | 1 | | 0 | 0 | 0 | 0 | 0 | 0 | 0 | 0 | 0 | 0 | 1 | 0 | 0 | 0 | 0 | 0 | 0 | 0 | 0 | 0 | 0 | 0 | 0 | 0 | 0 | | | | | | |

Table 158

| | A | B | C | E | G | I | K | M | O | Q | S | U | W | Y | AA | AC | AE | AG | AI | AK | AM | AO | AQ | AS | AU | AW | AY | BA | BC | BE | BF | BG | BH | BI | BK |
|---|---|---|---|---|---|---|---|---|---|---|---|---|---|---|---|---|---|---|---|---|---|---|---|---|---|---|---|---|---|---|---|---|---|---|---|
| 5674 | 06739 | 05673 | 1 | | 0 | 0 | 0 | 0 | 0 | 0 | 0 | 0 | 0 | 0 | 1 | 0 | 0 | 0 | 0 | 0 | 0 | 0 | 0 | 0 | 0 | 0 | 0 | 0 | 0 | | | | | | |
| 5675 | 06740 | 05674 | 1 | | 0 | 0 | 0 | 0 | 0 | 0 | 0 | 0 | 0 | 0 | 1 | 0 | 0 | 0 | 0 | 0 | 0 | 0 | 0 | 0 | 0 | 0 | 0 | 0 | 0 | | | | | | |
| 5676 | 06742 | 05675 | 1 | | 0 | 0 | 0 | 0 | 0 | 0 | 0 | 0 | 0 | 0 | 1 | 0 | 0 | 0 | 0 | 0 | 0 | 0 | 0 | 0 | 0 | 0 | 0 | 0 | 0 | | | | | | |
| 5677 | 06743 | 05676 | 1 | | 0 | 0 | 0 | 0 | 0 | 0 | 0 | 0 | 0 | 0 | 1 | 0 | 0 | 0 | 0 | 0 | 0 | 0 | 0 | 0 | 0 | 0 | 0 | 0 | 0 | | | | | | |
| 5678 | 06744 | 05677 | 2 | | 0 | 0 | 0 | 0 | 0 | 0 | 0 | 0 | 0 | 0 | 2 | 0 | 0 | 0 | 0 | 0 | 0 | 0 | 0 | 0 | 0 | 0 | 0 | 0 | 0 | | | | | | |
| 5679 | 06745 | 05678 | 1 | | 0 | 0 | 0 | 0 | 0 | 0 | 0 | 0 | 0 | 0 | 1 | 0 | 0 | 0 | 0 | 0 | 0 | 0 | 0 | 0 | 0 | 0 | 0 | 0 | 0 | | | | | | |
| 5680 | 06746 | 05679 | 2 | | 0 | 0 | 0 | 0 | 0 | 0 | 0 | 0 | 0 | 0 | 1 | 0 | 0 | 0 | 0 | 0 | 0 | 0 | 0 | 0 | 0 | 1 | 0 | 0 | 0 | L08488 | 100 | 253 | 1 | 1453 | 1705 |
| 5681 | 06747 | 05680 | 1 | | 0 | 0 | 0 | 0 | 0 | 0 | 0 | 0 | 0 | 0 | 1 | 0 | 0 | 0 | 0 | 0 | 0 | 0 | 0 | 0 | 0 | 0 | 0 | 0 | 0 | Y00815 | 98.8 | 172 | 1 | 7531 | 7702 |
| 5682 | 06749 | 05681 | 1 | | 0 | 0 | 0 | 0 | 0 | 0 | 0 | 0 | 0 | 0 | 1 | 0 | 0 | 0 | 0 | 0 | 0 | 0 | 0 | 0 | 0 | 0 | 0 | 0 | 0 | | | | | | |
| 5683 | 06750 | 05682 | 2 | | 0 | 0 | 0 | 0 | 0 | 0 | 0 | 0 | 0 | 0 | 1 | 0 | 0 | 0 | 0 | 0 | 0 | 0 | 0 | 1 | 0 | 0 | 0 | 0 | 0 | | | | | | |
| 5684 | 06751 | 05683 | 1 | | 0 | 0 | 0 | 0 | 0 | 0 | 0 | 0 | 0 | 0 | 1 | 0 | 0 | 0 | 0 | 0 | 0 | 0 | 0 | 0 | 0 | 0 | 0 | 0 | 0 | | | | | | |
| 5685 | 06752 | 05684 | 1 | | 0 | 0 | 0 | 0 | 0 | 0 | 0 | 0 | 0 | 0 | 1 | 0 | 0 | 0 | 0 | 0 | 0 | 0 | 0 | 0 | 0 | 0 | 0 | 0 | 0 | | | | | | |
| 5686 | 06753 | 05685 | 1 | | 0 | 0 | 0 | 0 | 0 | 0 | 0 | 0 | 0 | 0 | 1 | 0 | 0 | 0 | 0 | 0 | 0 | 0 | 0 | 0 | 0 | 0 | 0 | 0 | 0 | | | | | | |
| 5687 | 06754 | 05686 | 1 | | 0 | 0 | 0 | 0 | 0 | 0 | 0 | 0 | 0 | 0 | 1 | 0 | 0 | 0 | 0 | 0 | 0 | 0 | 0 | 0 | 0 | 0 | 0 | 0 | 0 | | | | | | |
| 5688 | 06755 | 05687 | 1 | | 0 | 0 | 0 | 0 | 0 | 0 | 0 | 0 | 0 | 0 | 1 | 0 | 0 | 0 | 0 | 0 | 0 | 0 | 0 | 0 | 0 | 0 | 0 | 0 | 0 | J03548 | 94.6 | 129 | 1 | 1335 | 1463 |
| 5689 | 06756 | 05688 | 1 | | 0 | 0 | 0 | 0 | 0 | 0 | 0 | 0 | 0 | 0 | 1 | 0 | 0 | 0 | 0 | 0 | 0 | 0 | 0 | 0 | 0 | 0 | 0 | 0 | 0 | | | | | | |
| 5690 | 06757 | 05689 | 1 | | 0 | 0 | 0 | 0 | 0 | 0 | 0 | 0 | 0 | 0 | 1 | 0 | 0 | 0 | 0 | 0 | 0 | 0 | 0 | 0 | 0 | 0 | 0 | 0 | 0 | | | | | | |
| 5691 | 06758 | 05690 | 1 | | 0 | 0 | 0 | 0 | 0 | 0 | 0 | 0 | 0 | 0 | 1 | 0 | 0 | 0 | 0 | 0 | 0 | 0 | 0 | 0 | 0 | 0 | 0 | 0 | 0 | | | | | | |
| 5692 | 06759 | 05691 | 1 | | 0 | 0 | 0 | 0 | 0 | 0 | 0 | 0 | 0 | 0 | 1 | 0 | 0 | 0 | 0 | 0 | 0 | 0 | 0 | 0 | 0 | 0 | 0 | 0 | 0 | | | | | | |
| 5693 | 06760 | 05692 | 2 | | 0 | 0 | 0 | 0 | 0 | 0 | 0 | 0 | 0 | 0 | 1 | 0 | 0 | 0 | 0 | 0 | 0 | 0 | 0 | 0 | 0 | 0 | 0 | 1 | 0 | | | | | | |
| 5694 | 06762 | 05693 | 1 | | 0 | 0 | 0 | 0 | 0 | 0 | 0 | 0 | 0 | 0 | 1 | 0 | 0 | 0 | 0 | 0 | 0 | 0 | 0 | 0 | 0 | 0 | 0 | 0 | 0 | | | | | | |
| 5695 | 06763 | 05694 | 1 | | 0 | 0 | 0 | 0 | 0 | 0 | 0 | 0 | 0 | 0 | 1 | 0 | 0 | 0 | 0 | 0 | 0 | 0 | 0 | 0 | 0 | 0 | 0 | 0 | 0 | | | | | | |
| 5696 | 06764 | 05695 | 1 | | 0 | 0 | 0 | 0 | 0 | 0 | 0 | 0 | 0 | 0 | 1 | 0 | 0 | 0 | 0 | 0 | 0 | 0 | 0 | 0 | 0 | 0 | 0 | 0 | 0 | M18217 | 91.4 | 336 | 1 | 292 | 946 |
| 5697 | 06765 | 05696 | 1 | | 0 | 0 | 0 | 0 | 0 | 0 | 0 | 0 | 0 | 0 | 1 | 0 | 0 | 0 | 0 | 0 | 0 | 0 | 0 | 0 | 0 | 0 | 0 | 0 | 0 | | | | | | |
| 5698 | 06766 | 05697 | 1 | | 0 | 0 | 0 | 0 | 0 | 0 | 0 | 0 | 0 | 0 | 1 | 0 | 0 | 0 | 0 | 0 | 0 | 0 | 0 | 0 | 0 | 0 | 0 | 0 | 0 | | | | | | |
| 5699 | 06767 | 05698 | 1 | | 0 | 0 | 0 | 0 | 0 | 0 | 0 | 0 | 0 | 0 | 1 | 0 | 0 | 0 | 0 | 0 | 0 | 0 | 0 | 0 | 0 | 0 | 0 | 0 | 0 | | | | | | |
| 5700 | 06768 | 05699 | 1 | | 0 | 0 | 0 | 0 | 0 | 0 | 0 | 0 | 0 | 0 | 1 | 0 | 0 | 0 | 0 | 0 | 0 | 0 | 0 | 0 | 0 | 0 | 0 | 0 | 0 | | | | | | |
| 5701 | 06769 | 05700 | 1 | | 0 | 0 | 0 | 0 | 0 | 0 | 0 | 0 | 0 | 0 | 1 | 0 | 0 | 0 | 0 | 0 | 0 | 0 | 0 | 0 | 0 | 0 | 0 | 0 | 0 | | | | | | |
| 5702 | 06770 | 05701 | 1 | | 0 | 0 | 0 | 0 | 0 | 0 | 0 | 0 | 0 | 0 | 1 | 0 | 0 | 0 | 0 | 0 | 0 | 0 | 0 | 0 | 0 | 0 | 0 | 0 | 0 | L02785 | 93.6 | 373 | 1 | 2499 | 2881 |
| 5703 | 06771 | 05702 | 1 | | 0 | 0 | 0 | 0 | 0 | 0 | 0 | 0 | 0 | 0 | 1 | 0 | 0 | 0 | 0 | 0 | 0 | 0 | 0 | 0 | 0 | 0 | 0 | 0 | 0 | | | | | | |
| 5704 | 06772 | 05703 | 2 | | 0 | 0 | 0 | 0 | 0 | 0 | 0 | 0 | 0 | 0 | 1 | 0 | 0 | 0 | 0 | 0 | 0 | 0 | 0 | 0 | 0 | 0 | 0 | 0 | 1 | | | | | | |
| 5705 | 06773 | 05704 | 1 | | 0 | 0 | 0 | 0 | 0 | 0 | 0 | 0 | 0 | 0 | 1 | 0 | 0 | 0 | 0 | 0 | 0 | 0 | 0 | 0 | 0 | 0 | 0 | 0 | 0 | | | | | | |
| 5706 | 06774 | 05705 | 1 | | 0 | 0 | 0 | 0 | 0 | 0 | 0 | 0 | 0 | 0 | 1 | 0 | 0 | 0 | 0 | 0 | 0 | 0 | 0 | 0 | 0 | 0 | 0 | 0 | 0 | | | | | | |
| 5707 | 06775 | 05706 | 1 | | 0 | 0 | 0 | 0 | 0 | 0 | 0 | 0 | 0 | 0 | 1 | 0 | 0 | 0 | 0 | 0 | 0 | 0 | 0 | 0 | 0 | 0 | 0 | 0 | 0 | | | | | | |
| 5708 | 06776 | 05707 | 1 | | 0 | 0 | 0 | 0 | 0 | 0 | 0 | 0 | 0 | 0 | 1 | 0 | 0 | 0 | 0 | 0 | 0 | 0 | 0 | 0 | 0 | 0 | 0 | 0 | 0 | | | | | | |
| 5709 | 06777 | 05708 | 1 | | 0 | 0 | 0 | 0 | 0 | 0 | 0 | 0 | 0 | 0 | 1 | 0 | 0 | 0 | 0 | 0 | 0 | 0 | 0 | 0 | 0 | 0 | 0 | 0 | 0 | | | | | | |

Table 159

| | A | B | C | E | G | I | K | M | O | Q | S | U | W | Y | AA | AC | AF | AG | AI | AK | AM | AO | AQ | AS | AU | AW | AY | BA | BC | BE | BF | BG | BH | BI | BK |
|---|---|---|---|---|---|---|---|---|---|---|---|---|---|---|---|---|---|---|---|---|---|---|---|---|---|---|---|---|---|---|---|---|---|---|---|
| 5710 | 06778 | 05709 | 1 | | 0 | 0 | 0 | 0 | 0 | 0 | 0 | 0 | 0 | 0 | 1 | 0 | 0 | 0 | 0 | 0 | 0 | 0 | 0 | 0 | 0 | 0 | 0 | 0 | 0 | | | | | | |
| 5711 | 06780 | 05710 | 4 | | 0 | 0 | 0 | 0 | 0 | 0 | 0 | 0 | 0 | 0 | 0 | 0 | 0 | 0 | 0 | 0 | 0 | 2 | 1 | 0 | 1 | 0 | 0 | 0 | 0 | | | | | | |
| 5712 | 06781 | 05711 | 2 | | 0 | 0 | 0 | 0 | 0 | 0 | 0 | 0 | 0 | 0 | 0 | 0 | 0 | 0 | 0 | 0 | 0 | 1 | 0 | 0 | 1 | 0 | 0 | 0 | 0 | | | | | | |
| 5713 | 06783 | 05712 | 1 | | 0 | 0 | 0 | 0 | 0 | 0 | 0 | 0 | 0 | 0 | 0 | 0 | 0 | 0 | 0 | 0 | 0 | 1 | 0 | 0 | 0 | 0 | 0 | 0 | 0 | | | | | | |
| 5714 | 06784 | 05713 | 1 | | 0 | 0 | 0 | 0 | 0 | 0 | 0 | 0 | 0 | 0 | 0 | 0 | 0 | 0 | 0 | 0 | 0 | 1 | 0 | 0 | 0 | 0 | 0 | 0 | 0 | | | | | | |
| 5715 | 06785 | 05714 | 1 | | 0 | 0 | 0 | 0 | 0 | 0 | 0 | 0 | 0 | 0 | 0 | 0 | 0 | 0 | 0 | 0 | 0 | 1 | 0 | 0 | 0 | 0 | 0 | 0 | 0 | | | | | | |
| 5716 | 06786 | 05715 | 1 | | 0 | 0 | 0 | 0 | 0 | 0 | 0 | 0 | 0 | 0 | 0 | 0 | 0 | 0 | 0 | 0 | 0 | 1 | 0 | 0 | 0 | 0 | 0 | 0 | 0 | J05633 | 100 | 214 | 1 | 2887 | 3117 |
| 5717 | 06787 | 05716 | 3 | | 0 | 0 | 0 | 0 | 0 | 0 | 0 | 0 | 0 | 0 | 0 | 0 | 0 | 0 | 0 | 0 | 0 | 1 | 1 | 1 | 0 | 0 | 0 | 0 | 0 | | | | | | |
| 5718 | 06788 | 05717 | 1 | | 0 | 0 | 0 | 0 | 0 | 0 | 0 | 0 | 0 | 0 | 0 | 0 | 0 | 0 | 0 | 0 | 0 | 1 | 0 | 0 | 0 | 0 | 0 | 0 | 0 | | | | | | |
| 5719 | 06789 | 05718 | 1 | | 0 | 0 | 0 | 0 | 0 | 0 | 0 | 0 | 0 | 0 | 0 | 0 | 0 | 0 | 0 | 0 | 0 | 1 | 0 | 0 | 0 | 0 | 0 | 0 | 0 | | | | | | |
| 5720 | 06790 | 05719 | 1 | | 0 | 0 | 0 | 0 | 0 | 0 | 0 | 0 | 0 | 0 | 0 | 0 | 0 | 0 | 0 | 0 | 0 | 1 | 0 | 0 | 0 | 0 | 0 | 0 | 0 | | | | | | |
| 5721 | 06791 | 05720 | 1 | | 0 | 0 | 0 | 0 | 0 | 0 | 0 | 0 | 0 | 0 | 0 | 0 | 0 | 0 | 0 | 0 | 0 | 1 | 0 | 0 | 0 | 0 | 0 | 0 | 0 | | | | | | |
| 5722 | 06792 | 05721 | 2 | | 0 | 0 | 0 | 0 | 0 | 0 | 0 | 0 | 0 | 0 | 0 | 0 | 0 | 0 | 0 | 0 | 0 | 1 | 1 | 0 | 0 | 0 | 0 | 0 | 0 | | | | | | |
| 5723 | 06794 | 05722 | 1 | | 0 | 0 | 0 | 0 | 0 | 0 | 0 | 0 | 0 | 0 | 0 | 0 | 0 | 0 | 0 | 0 | 0 | 1 | 0 | 0 | 0 | 0 | 0 | 0 | 0 | D21163 | 98.3 | 363 | 1 | 2923 | 3784 |
| 5724 | 06795 | 05723 | 1 | | 0 | 0 | 0 | 0 | 0 | 0 | 0 | 0 | 0 | 0 | 0 | 0 | 0 | 0 | 0 | 0 | 0 | 1 | 0 | 0 | 0 | 0 | 0 | 0 | 0 | | | | | | |
| 5725 | 06796 | 05724 | 1 | | 0 | 0 | 0 | 0 | 0 | 0 | 0 | 0 | 0 | 0 | 0 | 0 | 0 | 0 | 0 | 0 | 0 | 1 | 0 | 0 | 0 | 0 | 0 | 0 | 0 | | | | | | |
| 5726 | 06797 | 05725 | 1 | | 0 | 0 | 0 | 0 | 0 | 0 | 0 | 0 | 0 | 0 | 0 | 0 | 0 | 0 | 0 | 0 | 0 | 1 | 0 | 0 | 0 | 0 | 0 | 0 | 0 | | | | | | |
| 5727 | 06798 | 05726 | 1 | | 0 | 0 | 0 | 0 | 0 | 0 | 0 | 0 | 0 | 0 | 0 | 0 | 0 | 0 | 0 | 0 | 0 | 1 | 0 | 0 | 0 | 0 | 0 | 0 | 0 | | | | | | |
| 5728 | 06800 | 05727 | 1 | | 0 | 0 | 0 | 0 | 0 | 0 | 0 | 0 | 0 | 0 | 0 | 0 | 0 | 0 | 0 | 0 | 0 | 1 | 0 | 0 | 0 | 0 | 0 | 0 | 0 | | | | | | |
| 5729 | 06801 | 05728 | 1 | | 0 | 0 | 0 | 0 | 0 | 0 | 0 | 0 | 0 | 0 | 0 | 0 | 0 | 0 | 0 | 0 | 0 | 1 | 0 | 0 | 0 | 0 | 0 | 0 | 0 | | | | | | |
| 5730 | 06802 | 05729 | 1 | | 0 | 0 | 0 | 0 | 0 | 0 | 0 | 0 | 0 | 0 | 0 | 0 | 0 | 0 | 0 | 0 | 0 | 1 | 0 | 0 | 0 | 0 | 0 | 0 | 0 | | | | | | |
| 5731 | 06803 | 05730 | 1 | | 0 | 0 | 0 | 0 | 0 | 0 | 0 | 0 | 0 | 0 | 0 | 0 | 0 | 0 | 0 | 0 | 0 | 1 | 0 | 0 | 0 | 0 | 0 | 0 | 0 | | | | | | |
| 5732 | 06804 | 05731 | 1 | | 0 | 0 | 0 | 0 | 0 | 0 | 0 | 0 | 0 | 0 | 0 | 0 | 0 | 0 | 0 | 0 | 0 | 1 | 0 | 0 | 0 | 0 | 0 | 0 | 0 | | | | | | |
| 5733 | 06805 | 05732 | 1 | | 0 | 0 | 0 | 0 | 0 | 0 | 0 | 0 | 0 | 0 | 0 | 0 | 0 | 0 | 0 | 0 | 0 | 1 | 0 | 0 | 0 | 0 | 0 | 0 | 0 | | | | | | |
| 5734 | 06806 | 05733 | 1 | | 0 | 0 | 0 | 0 | 0 | 0 | 0 | 0 | 0 | 0 | 0 | 0 | 0 | 0 | 0 | 0 | 0 | 1 | 0 | 0 | 0 | 0 | 0 | 0 | 0 | | | | | | |
| 5735 | 06807 | 05734 | 1 | | 0 | 0 | 0 | 0 | 0 | 0 | 0 | 0 | 0 | 0 | 0 | 0 | 0 | 0 | 0 | 0 | 0 | 1 | 0 | 0 | 0 | 0 | 0 | 0 | 0 | | | | | | |
| 5736 | 06808 | 05735 | 1 | | 0 | 0 | 0 | 0 | 0 | 0 | 0 | 0 | 0 | 0 | 0 | 0 | 0 | 0 | 0 | 0 | 0 | 1 | 0 | 0 | 0 | 0 | 0 | 0 | 0 | | | | | | |
| 5737 | 06809 | 05736 | 1 | | 0 | 0 | 0 | 0 | 0 | 0 | 0 | 0 | 0 | 0 | 0 | 0 | 0 | 0 | 0 | 0 | 0 | 1 | 0 | 0 | 0 | 0 | 0 | 0 | 0 | | | | | | |
| 5738 | 06810 | 05737 | 2 | | 0 | 0 | 0 | 0 | 0 | 0 | 0 | 0 | 0 | 0 | 0 | 0 | 0 | 0 | 0 | 0 | 0 | 1 | 0 | 0 | 0 | 1 | 0 | 0 | 0 | | | | | | |
| 5739 | 06811 | 05738 | 1 | | 0 | 0 | 0 | 0 | 0 | 0 | 0 | 0 | 0 | 0 | 0 | 0 | 0 | 0 | 0 | 0 | 0 | 1 | 0 | 0 | 0 | 0 | 0 | 0 | 0 | | | | | | |
| 5740 | 06812 | 05739 | 1 | | 0 | 0 | 0 | 0 | 0 | 0 | 0 | 0 | 0 | 0 | 0 | 0 | 0 | 0 | 0 | 0 | 0 | 1 | 0 | 0 | 0 | 0 | 0 | 0 | 0 | | | | | | |
| 5741 | 06813 | 05740 | 2 | | 0 | 0 | 0 | 0 | 0 | 0 | 0 | 0 | 0 | 0 | 0 | 0 | 0 | 0 | 0 | 0 | 0 | 2 | 0 | 0 | 0 | 0 | 0 | 0 | 0 | L07956 | 98.4 | 127 | 1 | 2780 | 2955 |
| 5742 | 06815 | 05741 | 2 | | 0 | 0 | 0 | 0 | 0 | 0 | 0 | 0 | 0 | 0 | 0 | 0 | 0 | 0 | 0 | 0 | 0 | 1 | 0 | 0 | 0 | 1 | 0 | 0 | 0 | | | | | | |
| 5743 | 06816 | 05742 | 1 | | 0 | 0 | 0 | 0 | 0 | 0 | 0 | 0 | 0 | 0 | 0 | 0 | 0 | 0 | 0 | 0 | 0 | 1 | 0 | 0 | 0 | 0 | 0 | 0 | 0 | X06985 | 93.3 | 195 | 1 | 1175 | 1550 |
| 5744 | 06817 | 05743 | 2 | | 0 | 0 | 0 | 0 | 0 | 0 | 0 | 0 | 0 | 0 | 0 | 0 | 0 | 0 | 0 | 0 | 0 | 1 | 0 | 0 | 0 | 1 | 0 | 0 | 0 | | | | | | |
| 5745 | 06818 | 05744 | 1 | | 0 | 0 | 0 | 0 | 0 | 0 | 0 | 0 | 0 | 0 | 0 | 0 | 0 | 0 | 0 | 0 | 0 | 1 | 0 | 0 | 0 | 0 | 0 | 0 | 0 | | | | | | |

Table 160

| | A | B | C | E | G | I | K | M | O | Q | S | U | W | Y | AA | AC | AE | AG | AI | AK | AM | AO | AQ | AS | AU | AW | AY | BA | BC | BE | BF | BG | BH | BI | BK |
|---|---|---|---|---|---|---|---|---|---|---|---|---|---|---|---|---|---|---|---|---|---|---|---|---|---|---|---|---|---|---|---|---|---|---|---|
| 5746 | 06819 | 05745 | 1 | | 0 | 0 | 0 | 0 | 0 | 0 | 0 | 0 | 0 | 0 | 0 | 0 | 0 | 0 | 0 | 0 | 0 | 1 | 0 | 0 | 0 | 0 | 0 | 0 | 0 | | | | | | |
| 5747 | 06820 | 05746 | 1 | | 0 | 0 | 0 | 0 | 0 | 0 | 0 | 0 | 0 | 0 | 0 | 0 | 0 | 0 | 0 | 0 | 0 | 1 | 0 | 0 | 0 | 0 | 0 | 0 | 0 | | | | | | |
| 5748 | 06821 | 05747 | 1 | | 0 | 0 | 0 | 0 | 0 | 0 | 0 | 0 | 0 | 0 | 0 | 0 | 0 | 0 | 0 | 0 | 0 | 1 | 0 | 0 | 0 | 0 | 0 | 0 | 0 | | | | | | |
| 5749 | 06823 | 05748 | 1 | | 0 | 0 | 0 | 0 | 0 | 0 | 0 | 0 | 0 | 0 | 0 | 0 | 0 | 0 | 0 | 0 | 0 | 1 | 0 | 0 | 0 | 0 | 0 | 0 | 0 | | | | | | |
| 5750 | 06824 | 05749 | 1 | | 0 | 0 | 0 | 0 | 0 | 0 | 0 | 0 | 0 | 0 | 0 | 0 | 0 | 0 | 0 | 0 | 0 | 1 | 0 | 0 | 0 | 0 | 0 | 0 | 0 | | | | | | |
| 5751 | 06825 | 05750 | 1 | | 0 | 0 | 0 | 0 | 0 | 0 | 0 | 0 | 0 | 0 | 0 | 0 | 0 | 0 | 0 | 0 | 0 | 1 | 0 | 0 | 0 | 0 | 0 | 0 | 0 | | | | | | |
| 5752 | 06826 | 05751 | 2 | | 0 | 0 | 0 | 0 | 0 | 0 | 0 | 0 | 0 | 0 | 0 | 0 | 0 | 0 | 0 | 0 | 0 | 1 | 0 | 0 | 1 | 0 | 0 | 0 | 0 | | | | | | |
| 5753 | 06827 | 05752 | 1 | | 0 | 0 | 0 | 0 | 0 | 0 | 0 | 0 | 0 | 0 | 0 | 0 | 0 | 0 | 0 | 0 | 0 | 1 | 0 | 0 | 0 | 0 | 0 | 0 | 0 | | | | | | |
| 5754 | 06828 | 05753 | 1 | | 0 | 0 | 0 | 0 | 0 | 0 | 0 | 0 | 0 | 0 | 0 | 0 | 0 | 0 | 0 | 0 | 0 | 1 | 0 | 0 | 0 | 0 | 0 | 0 | 0 | | | | | | |
| 5755 | 06829 | 05754 | 1 | | 0 | 0 | 0 | 0 | 0 | 0 | 0 | 0 | 0 | 0 | 0 | 0 | 0 | 0 | 0 | 0 | 0 | 1 | 0 | 0 | 0 | 0 | 0 | 0 | 0 | | | | | | |
| 5756 | 06830 | 05755 | 1 | | 0 | 0 | 0 | 0 | 0 | 0 | 0 | 0 | 0 | 0 | 0 | 0 | 0 | 0 | 0 | 0 | 0 | 1 | 0 | 0 | 0 | 0 | 0 | 0 | 0 | | | | | | |
| 5757 | 06831 | 05756 | 1 | | 0 | 0 | 0 | 0 | 0 | 0 | 0 | 0 | 0 | 0 | 0 | 0 | 0 | 0 | 0 | 0 | 0 | 1 | 0 | 0 | 0 | 0 | 0 | 0 | 0 | | | | | | |
| 5758 | 06832 | 05757 | 1 | | 0 | 0 | 0 | 0 | 0 | 0 | 0 | 0 | 0 | 0 | 0 | 0 | 0 | 0 | 0 | 0 | 0 | 1 | 0 | 0 | 0 | 0 | 0 | 0 | 0 | | | | | | |
| 5759 | 06833 | 05758 | 2 | | 0 | 0 | 0 | 0 | 0 | 0 | 0 | 0 | 0 | 0 | 0 | 0 | 0 | 0 | 0 | 0 | 0 | 1 | 0 | 0 | 0 | 0 | 0 | 1 | 0 | | | | | | |
| 5760 | 06834 | 05759 | 1 | | 0 | 0 | 0 | 0 | 0 | 0 | 0 | 0 | 0 | 0 | 0 | 0 | 0 | 0 | 0 | 0 | 0 | 1 | 0 | 0 | 0 | 0 | 0 | 0 | 0 | J05481 | 92.8 | 237 | 1 | 2363 | 2620 |
| 5761 | 06835 | 05760 | 1 | | 0 | 0 | 0 | 0 | 0 | 0 | 0 | 0 | 0 | 0 | 0 | 0 | 0 | 0 | 0 | 0 | 0 | 1 | 0 | 0 | 0 | 0 | 0 | 0 | 0 | | | | | | |
| 5762 | 06836 | 05761 | 2 | | 0 | 0 | 0 | 0 | 0 | 0 | 0 | 0 | 0 | 0 | 0 | 0 | 0 | 0 | 0 | 0 | 0 | 1 | 0 | 1 | 0 | 0 | 0 | 0 | 0 | | | | | | |
| 5763 | 06837 | 05762 | 2 | | 0 | 0 | 0 | 0 | 0 | 0 | 0 | 0 | 0 | 0 | 0 | 0 | 0 | 0 | 0 | 0 | 0 | 1 | 0 | 1 | 0 | 0 | 0 | 0 | 0 | | | | | | |
| 5764 | 06838 | 05763 | 1 | | 0 | 0 | 0 | 0 | 0 | 0 | 0 | 0 | 0 | 0 | 0 | 0 | 0 | 0 | 0 | 0 | 0 | 1 | 0 | 0 | 0 | 0 | 0 | 0 | 0 | J03202 | 95.3 | 299 | 1 | 5002 | 5306 |
| 5765 | 06839 | 05764 | 1 | | 0 | 0 | 0 | 0 | 0 | 0 | 0 | 0 | 0 | 0 | 0 | 0 | 0 | 0 | 0 | 0 | 0 | 1 | 0 | 0 | 0 | 0 | 0 | 0 | 0 | | | | | | |
| 5766 | 06840 | 05765 | 1 | | 0 | 0 | 0 | 0 | 0 | 0 | 0 | 0 | 0 | 0 | 0 | 0 | 0 | 0 | 0 | 0 | 0 | 1 | 0 | 0 | 0 | 0 | 0 | 0 | 0 | | | | | | |
| 5767 | 06841 | 05766 | 1 | | 0 | 0 | 0 | 0 | 0 | 0 | 0 | 0 | 0 | 0 | 0 | 0 | 0 | 0 | 0 | 0 | 0 | 1 | 0 | 0 | 0 | 0 | 0 | 0 | 0 | | | | | | |
| 5768 | 06842 | 05767 | 1 | | 0 | 0 | 0 | 0 | 0 | 0 | 0 | 0 | 0 | 0 | 0 | 0 | 0 | 0 | 0 | 0 | 0 | 1 | 0 | 0 | 0 | 0 | 0 | 0 | 0 | | | | | | |
| 5769 | 06843 | 05768 | 2 | | 0 | 0 | 0 | 0 | 0 | 0 | 0 | 0 | 0 | 0 | 0 | 0 | 0 | 0 | 0 | 0 | 0 | 1 | 0 | 0 | 0 | 1 | 0 | 0 | 0 | | | | | | |
| 5770 | 06844 | 05769 | 1 | | 0 | 0 | 0 | 0 | 0 | 0 | 0 | 0 | 0 | 0 | 0 | 0 | 0 | 0 | 0 | 0 | 0 | 0 | 1 | 0 | 0 | 0 | 0 | 0 | 0 | | | | | | |
| 5771 | 06845 | 05770 | 1 | | 0 | 0 | 0 | 0 | 0 | 0 | 0 | 0 | 0 | 0 | 0 | 0 | 0 | 0 | 0 | 0 | 0 | 0 | 1 | 0 | 0 | 0 | 0 | 0 | 0 | | | | | | |
| 5772 | 06846 | 05771 | 2 | | 0 | 0 | 0 | 0 | 0 | 0 | 0 | 0 | 0 | 0 | 0 | 0 | 0 | 0 | 0 | 0 | 0 | 0 | 2 | 0 | 0 | 0 | 0 | 0 | 0 | | | | | | |
| 5773 | 06848 | 05772 | 2 | | 0 | 0 | 0 | 0 | 0 | 0 | 0 | 0 | 0 | 0 | 0 | 0 | 0 | 0 | 0 | 0 | 0 | 0 | 2 | 0 | 0 | 0 | 0 | 0 | 0 | | | | | | |
| 5774 | 06850 | 05773 | 3 | | 0 | 0 | 0 | 0 | 0 | 0 | 0 | 0 | 0 | 0 | 0 | 0 | 0 | 0 | 0 | 0 | 0 | 0 | 1 | 0 | 2 | 0 | 0 | 0 | 0 | | | | | | |
| 5775 | 06851 | 05774 | 1 | | 0 | 0 | 0 | 0 | 0 | 0 | 0 | 0 | 0 | 0 | 0 | 0 | 0 | 0 | 0 | 0 | 0 | 0 | 1 | 0 | 0 | 0 | 0 | 0 | 0 | | | | | | |
| 5776 | 06852 | 05775 | 2 | | 0 | 0 | 0 | 0 | 0 | 0 | 0 | 0 | 0 | 0 | 0 | 0 | 0 | 0 | 0 | 0 | 0 | 0 | 2 | 0 | 0 | 0 | 0 | 0 | 0 | | | | | | |
| 5777 | 06854 | 05776 | 1 | | 0 | 0 | 0 | 0 | 0 | 0 | 0 | 0 | 0 | 0 | 0 | 0 | 0 | 0 | 0 | 0 | 0 | 0 | 1 | 0 | 0 | 0 | 0 | 0 | 0 | | | | | | |
| 5778 | 06855 | 05777 | 1 | | 0 | 0 | 0 | 0 | 0 | 0 | 0 | 0 | 0 | 0 | 0 | 0 | 0 | 0 | 0 | 0 | 0 | 0 | 1 | 0 | 0 | 0 | 0 | 0 | 0 | | | | | | |
| 5779 | 06856 | 05778 | 1 | | 0 | 0 | 0 | 0 | 0 | 0 | 0 | 0 | 0 | 0 | 0 | 0 | 0 | 0 | 0 | 0 | 0 | 0 | 1 | 0 | 0 | 0 | 0 | 0 | 0 | | | | | | |
| 5780 | 06857 | 05779 | 1 | | 0 | 0 | 0 | 0 | 0 | 0 | 0 | 0 | 0 | 0 | 0 | 0 | 0 | 0 | 0 | 0 | 0 | 0 | 1 | 0 | 0 | 0 | 0 | 0 | 0 | | | | | | |
| 5781 | 06858 | 05780 | 1 | | 0 | 0 | 0 | 0 | 0 | 0 | 0 | 0 | 0 | 0 | 0 | 0 | 0 | 0 | 0 | 0 | 0 | 0 | 1 | 0 | 0 | 0 | 0 | 0 | 0 | | | | | | |

Table 161

| | A | B | C | E | G | I | K | M | O | Q | S | U | W | Y | AA | AC | AE | AG | AI | AK | AM | AO | AQ | AS | AU | AW | AY | BA | BC | BE | BF | BG | BH | BI | BK |
|---|---|---|---|---|---|---|---|---|---|---|---|---|---|---|---|---|---|---|---|---|---|---|---|---|---|---|---|---|---|---|---|---|---|---|---|
| 5782 | 06859 | 05781 | 2 | | 0 | 0 | 0 | 0 | 0 | 0 | 0 | 0 | 0 | 0 | 0 | 0 | 0 | 0 | 0 | 0 | 0 | 0 | 2 | 0 | 0 | 0 | 0 | 0 | 0 | | | | | | |
| 5783 | 06863 | 05782 | 1 | | 0 | 0 | 0 | 0 | 0 | 0 | 0 | 0 | 0 | 0 | 0 | 0 | 0 | 0 | 0 | 0 | 0 | 0 | 1 | 0 | 0 | 0 | 0 | 0 | 0 | | | | | | |
| 5784 | 06864 | 05783 | 1 | | 0 | 0 | 0 | 0 | 0 | 0 | 0 | 0 | 0 | 0 | 0 | 0 | 0 | 0 | 0 | 0 | 0 | 0 | 1 | 0 | 0 | 0 | 0 | 0 | 0 | | | | | | |
| 5785 | 06865 | 05784 | 1 | | 0 | 0 | 0 | 0 | 0 | 0 | 0 | 0 | 0 | 0 | 0 | 0 | 0 | 0 | 0 | 0 | 0 | 0 | 1 | 0 | 0 | 0 | 0 | 0 | 0 | | | | | | |
| 5786 | 06866 | 05785 | 1 | | 0 | 0 | 0 | 0 | 0 | 0 | 0 | 0 | 0 | 0 | 0 | 0 | 0 | 0 | 0 | 0 | 0 | 0 | 1 | 0 | 0 | 0 | 0 | 0 | 0 | | | | | | |
| 5787 | 06869 | 05786 | 4 | | 0 | 0 | 0 | 0 | 0 | 0 | 0 | 0 | 0 | 0 | 0 | 0 | 0 | 0 | 0 | 0 | 0 | 0 | 1 | 0 | 0 | 0 | 0 | 3 | 0 | | | | | | |
| 5788 | 06870 | 05787 | 1 | | 0 | 0 | 0 | 0 | 0 | 0 | 0 | 0 | 0 | 0 | 0 | 0 | 0 | 0 | 0 | 0 | 0 | 0 | 1 | 0 | 0 | 0 | 0 | 0 | 0 | | | | | | |
| 5789 | 06871 | 05788 | 1 | | 0 | 0 | 0 | 0 | 0 | 0 | 0 | 0 | 0 | 0 | 0 | 0 | 0 | 0 | 0 | 0 | 0 | 0 | 1 | 0 | 0 | 0 | 0 | 0 | 0 | | | | | | |
| 5790 | 06872 | 05789 | 1 | | 0 | 0 | 0 | 0 | 0 | 0 | 0 | 0 | 0 | 0 | 0 | 0 | 0 | 0 | 0 | 0 | 0 | 0 | 1 | 0 | 0 | 0 | 0 | 0 | 0 | | | | | | |
| 5791 | 06873 | 05790 | 1 | | 0 | 0 | 0 | 0 | 0 | 0 | 0 | 0 | 0 | 0 | 0 | 0 | 0 | 0 | 0 | 0 | 0 | 0 | 1 | 0 | 0 | 0 | 0 | 0 | 0 | | | | | | |
| 5792 | 06874 | 05791 | 1 | | 0 | 0 | 0 | 0 | 0 | 0 | 0 | 0 | 0 | 0 | 0 | 0 | 0 | 0 | 0 | 0 | 0 | 0 | 1 | 0 | 0 | 0 | 0 | 0 | 0 | | | | | | |
| 5793 | 06875 | 05792 | 1 | | 0 | 0 | 0 | 0 | 0 | 0 | 0 | 0 | 0 | 0 | 0 | 0 | 0 | 0 | 0 | 0 | 0 | 0 | 1 | 0 | 0 | 0 | 0 | 0 | 0 | | | | | | |
| 5794 | 06876 | 05793 | 1 | | 0 | 0 | 0 | 0 | 0 | 0 | 0 | 0 | 0 | 0 | 0 | 0 | 0 | 0 | 0 | 0 | 0 | 0 | 1 | 0 | 0 | 0 | 0 | 0 | 0 | | | | | | |
| 5795 | 06878 | 05794 | 1 | | 0 | 0 | 0 | 0 | 0 | 0 | 0 | 0 | 0 | 0 | 0 | 0 | 0 | 0 | 0 | 0 | 0 | 0 | 1 | 0 | 0 | 0 | 0 | 0 | 0 | D21261 | 95.3 | 236 | 1 | 672 | 1360 |
| 5796 | 06879 | 05795 | 1 | | 0 | 0 | 0 | 0 | 0 | 0 | 0 | 0 | 0 | 0 | 0 | 0 | 0 | 0 | 0 | 0 | 0 | 0 | 1 | 0 | 0 | 0 | 0 | 0 | 0 | | | | | | |
| 5797 | 06880 | 05796 | 1 | | 0 | 0 | 0 | 0 | 0 | 0 | 0 | 0 | 0 | 0 | 0 | 0 | 0 | 0 | 0 | 0 | 0 | 0 | 1 | 0 | 0 | 0 | 0 | 0 | 0 | | | | | | |
| 5798 | 06882 | 05797 | 2 | | 0 | 0 | 0 | 0 | 0 | 0 | 0 | 0 | 0 | 0 | 0 | 0 | 0 | 0 | 0 | 0 | 0 | 0 | 2 | 0 | 0 | 0 | 0 | 0 | 0 | | | | | | |
| 5799 | 06883 | 05798 | 1 | | 0 | 0 | 0 | 0 | 0 | 0 | 0 | 0 | 0 | 0 | 0 | 0 | 0 | 0 | 0 | 0 | 0 | 0 | 1 | 0 | 0 | 0 | 0 | 0 | 0 | | | | | | |
| 5800 | 06884 | 05799 | 1 | | 0 | 0 | 0 | 0 | 0 | 0 | 0 | 0 | 0 | 0 | 0 | 0 | 0 | 0 | 0 | 0 | 0 | 0 | 1 | 0 | 0 | 0 | 0 | 0 | 0 | | | | | | |
| 5801 | 06885 | 05800 | 2 | | 0 | 0 | 0 | 0 | 0 | 0 | 0 | 0 | 0 | 0 | 0 | 0 | 0 | 0 | 0 | 0 | 0 | 0 | 1 | 0 | 1 | 0 | 0 | 0 | 0 | | | | | | |
| 5802 | 06886 | 05801 | 1 | | 0 | 0 | 0 | 0 | 0 | 0 | 0 | 0 | 0 | 0 | 0 | 0 | 0 | 0 | 0 | 0 | 0 | 0 | 1 | 0 | 0 | 0 | 0 | 0 | 0 | L22342 | 97.6 | 289 | 1 | 545 | 835 |
| 5803 | 06888 | 05802 | 1 | | 0 | 0 | 0 | 0 | 0 | 0 | 0 | 0 | 0 | 0 | 0 | 0 | 0 | 0 | 0 | 0 | 0 | 0 | 1 | 0 | 0 | 0 | 0 | 0 | 0 | | | | | | |
| 5804 | 06889 | 05803 | 1 | | 0 | 0 | 0 | 0 | 0 | 0 | 0 | 0 | 0 | 0 | 0 | 0 | 0 | 0 | 0 | 0 | 0 | 0 | 1 | 0 | 0 | 0 | 0 | 0 | 0 | | | | | | |
| 5805 | 06890 | 05804 | 1 | | 0 | 0 | 0 | 0 | 0 | 0 | 0 | 0 | 0 | 0 | 0 | 0 | 0 | 0 | 0 | 0 | 0 | 0 | 1 | 0 | 0 | 0 | 0 | 0 | 0 | | | | | | |
| 5806 | 06891 | 05805 | 1 | | 0 | 0 | 0 | 0 | 0 | 0 | 0 | 0 | 0 | 0 | 0 | 0 | 0 | 0 | 0 | 0 | 0 | 0 | 1 | 0 | 0 | 0 | 0 | 0 | 0 | | | | | | |
| 5807 | 06893 | 05806 | 1 | | 0 | 0 | 0 | 0 | 0 | 0 | 0 | 0 | 0 | 0 | 0 | 0 | 0 | 0 | 0 | 0 | 0 | 0 | 1 | 0 | 0 | 0 | 0 | 0 | 0 | | | | | | |
| 5808 | 06894 | 05807 | 1 | | 0 | 0 | 0 | 0 | 0 | 0 | 0 | 0 | 0 | 0 | 0 | 0 | 0 | 0 | 0 | 0 | 0 | 0 | 1 | 0 | 0 | 0 | 0 | 0 | 0 | | | | | | |
| 5809 | 06895 | 05808 | 1 | | 0 | 0 | 0 | 0 | 0 | 0 | 0 | 0 | 0 | 0 | 0 | 0 | 0 | 0 | 0 | 0 | 0 | 0 | 1 | 0 | 0 | 0 | 0 | 0 | 0 | | | | | | |
| 5810 | 06896 | 05809 | 1 | | 0 | 0 | 0 | 0 | 0 | 0 | 0 | 0 | 0 | 0 | 0 | 0 | 0 | 0 | 0 | 0 | 0 | 0 | 1 | 0 | 0 | 0 | 0 | 0 | 0 | | | | | | |
| 5811 | 06897 | 05810 | 1 | | 0 | 0 | 0 | 0 | 0 | 0 | 0 | 0 | 0 | 0 | 0 | 0 | 0 | 0 | 0 | 0 | 0 | 0 | 1 | 0 | 0 | 0 | 0 | 0 | 0 | | | | | | |
| 5812 | 06898 | 05811 | 1 | | 0 | 0 | 0 | 0 | 0 | 0 | 0 | 0 | 0 | 0 | 0 | 0 | 0 | 0 | 0 | 0 | 0 | 0 | 1 | 0 | 0 | 0 | 0 | 0 | 0 | | | | | | |
| 5813 | 06899 | 05812 | 1 | | 0 | 0 | 0 | 0 | 0 | 0 | 0 | 0 | 0 | 0 | 0 | 0 | 0 | 0 | 0 | 0 | 0 | 0 | 1 | 0 | 0 | 0 | 0 | 0 | 0 | | | | | | |
| 5814 | 06900 | 05813 | 1 | | 0 | 0 | 0 | 0 | 0 | 0 | 0 | 0 | 0 | 0 | 0 | 0 | 0 | 0 | 0 | 0 | 0 | 0 | 1 | 0 | 0 | 0 | 0 | 0 | 0 | | | | | | |
| 5815 | 06901 | 05814 | 1 | | 0 | 0 | 0 | 0 | 0 | 0 | 0 | 0 | 0 | 0 | 0 | 0 | 0 | 0 | 0 | 0 | 0 | 0 | 1 | 0 | 0 | 0 | 0 | 0 | 0 | M73778 | 94.8 | 269 | 1 | 1762 | 2155 |
| 5816 | 06902 | 05815 | 1 | | 0 | 0 | 0 | 0 | 0 | 0 | 0 | 0 | 0 | 0 | 0 | 0 | 0 | 0 | 0 | 0 | 0 | 0 | 1 | 0 | 0 | 0 | 0 | 0 | 0 | | | | | | |
| 5817 | 06903 | 05816 | 1 | | 0 | 0 | 0 | 0 | 0 | 0 | 0 | 0 | 0 | 0 | 0 | 0 | 0 | 0 | 0 | 0 | 0 | 0 | 1 | 0 | 0 | 0 | 0 | 0 | 0 | | | | | | |

Table 162

| | A | B | C | E | G | I | K | M | O | Q | S | U | W | Y | AA | AC | AE | AG | AI | AK | AM | AO | AQ | AS | AU | AW | AY | BA | BC | BE | BF | BG | BH | BI | BK |
|---|---|---|---|---|---|---|---|---|---|---|---|---|---|---|---|---|---|---|---|---|---|---|---|---|---|---|---|---|---|---|---|---|---|---|---|
| 5818 | 06904 | 05817 | 1 | | 0 | 0 | 0 | 0 | 0 | 0 | 0 | 0 | 0 | 0 | 0 | 0 | 0 | 0 | 0 | 0 | 0 | 0 | 1 | 0 | 0 | 0 | 0 | 0 | 0 | | | | | | |
| 5819 | 06906 | 05818 | 1 | | 0 | 0 | 0 | 0 | 0 | 0 | 0 | 0 | 0 | 0 | 0 | 0 | 0 | 0 | 0 | 0 | 0 | 0 | 1 | 0 | 0 | 0 | 0 | 0 | 0 | | | | | | |
| 5820 | 06909 | 05819 | 1 | | 0 | 0 | 0 | 0 | 0 | 0 | 0 | 0 | 0 | 0 | 0 | 0 | 0 | 0 | 0 | 0 | 0 | 0 | 1 | 0 | 0 | 0 | 0 | 0 | 0 | | | | | | |
| 5821 | 06910 | 05820 | 1 | | 0 | 0 | 0 | 0 | 0 | 0 | 0 | 0 | 0 | 0 | 0 | 0 | 0 | 0 | 0 | 0 | 0 | 0 | 1 | 0 | 0 | 0 | 0 | 0 | 0 | | | | | | |
| 5822 | 06911 | 05821 | 1 | | 0 | 0 | 0 | 0 | 0 | 0 | 0 | 0 | 0 | 0 | 0 | 0 | 0 | 0 | 0 | 0 | 0 | 0 | 1 | 0 | 0 | 0 | 0 | 0 | 0 | | | | | | |
| 5823 | 06912 | 05822 | 1 | | 0 | 0 | 0 | 0 | 0 | 0 | 0 | 0 | 0 | 0 | 0 | 0 | 0 | 0 | 0 | 0 | 0 | 0 | 1 | 0 | 0 | 0 | 0 | 0 | 0 | | | | | | |
| 5824 | 06913 | 05823 | 2 | | 0 | 0 | 0 | 0 | 0 | 0 | 0 | 0 | 0 | 0 | 0 | 0 | 0 | 0 | 0 | 0 | 0 | 0 | 1 | 1 | 0 | 0 | 0 | 0 | 0 | | | | | | |
| 5825 | 06915 | 05824 | 1 | | 0 | 0 | 0 | 0 | 0 | 0 | 0 | 0 | 0 | 0 | 0 | 0 | 0 | 0 | 0 | 0 | 0 | 0 | 1 | 0 | 0 | 0 | 0 | 0 | 0 | | | | | | |
| 5826 | 06916 | 05825 | 1 | | 0 | 0 | 0 | 0 | 0 | 0 | 0 | 0 | 0 | 0 | 0 | 0 | 0 | 0 | 0 | 0 | 0 | 0 | 1 | 0 | 0 | 0 | 0 | 0 | 0 | | | | | | |
| 5827 | 06917 | 05826 | 1 | | 0 | 0 | 0 | 0 | 0 | 0 | 0 | 0 | 0 | 0 | 0 | 0 | 0 | 0 | 0 | 0 | 0 | 0 | 1 | 0 | 0 | 0 | 0 | 0 | 0 | | | | | | |
| 5828 | 06919 | 05827 | 1 | | 0 | 0 | 0 | 0 | 0 | 0 | 0 | 0 | 0 | 0 | 0 | 0 | 0 | 0 | 0 | 0 | 0 | 0 | 1 | 0 | 0 | 0 | 0 | 0 | 0 | | | | | | |
| 5829 | 06920 | 05828 | 27 | | 0 | 0 | 0 | 0 | 0 | 0 | 0 | 0 | 0 | 0 | 0 | 0 | 0 | 0 | 0 | 0 | 0 | 0 | 5 | 22 | 0 | 0 | 0 | 0 | 0 | J04569 | 93.5 | 216 | 1 | 2807 | 3017 |
| 5830 | 06922 | 05829 | 1 | | 0 | 0 | 0 | 0 | 0 | 0 | 0 | 0 | 0 | 0 | 0 | 0 | 0 | 0 | 0 | 0 | 0 | 0 | 1 | 0 | 0 | 0 | 0 | 0 | 0 | | | | | | |
| 5831 | 06923 | 05830 | 3 | | 0 | 0 | 0 | 0 | 0 | 0 | 0 | 0 | 0 | 0 | 0 | 0 | 0 | 0 | 0 | 0 | 0 | 0 | 1 | 2 | 0 | 0 | 0 | 0 | 0 | | | | | | |
| 5832 | 06927 | 05831 | 1 | | 0 | 0 | 0 | 0 | 0 | 0 | 0 | 0 | 0 | 0 | 0 | 0 | 0 | 0 | 0 | 0 | 0 | 0 | 1 | 0 | 0 | 0 | 0 | 0 | 0 | X52203 | 92.3 | 91 | 1 | 816 | 906 |
| 5833 | 06928 | 05832 | 1 | | 0 | 0 | 0 | 0 | 0 | 0 | 0 | 0 | 0 | 0 | 0 | 0 | 0 | 0 | 0 | 0 | 0 | 0 | 1 | 0 | 0 | 0 | 0 | 0 | 0 | | | | | | |
| 5834 | 06929 | 05833 | 2 | | 0 | 0 | 0 | 0 | 0 | 0 | 0 | 0 | 0 | 0 | 0 | 0 | 0 | 0 | 0 | 0 | 0 | 0 | 2 | 0 | 0 | 0 | 0 | 0 | 0 | | | | | | |
| 5835 | 06930 | 05834 | 1 | | 0 | 0 | 0 | 0 | 0 | 0 | 0 | 0 | 0 | 0 | 0 | 0 | 0 | 0 | 0 | 0 | 0 | 0 | 1 | 0 | 0 | 0 | 0 | 0 | 0 | L20046 | 96.8 | 158 | 1 | 3545 | 3702 |
| 5836 | 06931 | 05835 | 1 | | 0 | 0 | 0 | 0 | 0 | 0 | 0 | 0 | 0 | 0 | 0 | 0 | 0 | 0 | 0 | 0 | 0 | 0 | 1 | 0 | 0 | 0 | 0 | 0 | 0 | | | | | | |
| 5837 | 06932 | 05836 | 3 | | 0 | 0 | 0 | 0 | 0 | 0 | 0 | 0 | 0 | 0 | 0 | 0 | 0 | 0 | 0 | 0 | 0 | 0 | 2 | 1 | 0 | 0 | 0 | 0 | 0 | M27110 | 97.1 | 137 | 1 | 2343 | 2777 |
| 5838 | 06933 | 05837 | 1 | | 0 | 0 | 0 | 0 | 0 | 0 | 0 | 0 | 0 | 0 | 0 | 0 | 0 | 0 | 0 | 0 | 0 | 0 | 1 | 0 | 0 | 0 | 0 | 0 | 0 | | | | | | |
| 5839 | 06934 | 05838 | 2 | | 0 | 0 | 0 | 0 | 0 | 0 | 0 | 0 | 0 | 0 | 0 | 0 | 0 | 0 | 0 | 0 | 0 | 0 | 1 | 1 | 0 | 0 | 0 | 0 | 0 | | | | | | |
| 5840 | 06935 | 05839 | 1 | | 0 | 0 | 0 | 0 | 0 | 0 | 0 | 0 | 0 | 0 | 0 | 0 | 0 | 0 | 0 | 0 | 0 | 0 | 1 | 0 | 0 | 0 | 0 | 0 | 0 | | | | | | |
| 5841 | 06936 | 05840 | 1 | | 0 | 0 | 0 | 0 | 0 | 0 | 0 | 0 | 0 | 0 | 0 | 0 | 0 | 0 | 0 | 0 | 0 | 0 | 1 | 0 | 0 | 0 | 0 | 0 | 0 | | | | | | |
| 5842 | 06937 | 05841 | 1 | | 0 | 0 | 0 | 0 | 0 | 0 | 0 | 0 | 0 | 0 | 0 | 0 | 0 | 0 | 0 | 0 | 0 | 0 | 1 | 0 | 0 | 0 | 0 | 0 | 0 | | | | | | |
| 5843 | 06938 | 05842 | 1 | | 0 | 0 | 0 | 0 | 0 | 0 | 0 | 0 | 0 | 0 | 0 | 0 | 0 | 0 | 0 | 0 | 0 | 0 | 1 | 0 | 0 | 0 | 0 | 0 | 0 | | | | | | |
| 5844 | 06939 | 05843 | 1 | | 0 | 0 | 0 | 0 | 0 | 0 | 0 | 0 | 0 | 0 | 0 | 0 | 0 | 0 | 0 | 0 | 0 | 0 | 1 | 0 | 0 | 0 | 0 | 0 | 0 | | | | | | |
| 5845 | 06941 | 05844 | 1 | | 0 | 0 | 0 | 0 | 0 | 0 | 0 | 0 | 0 | 0 | 0 | 0 | 0 | 0 | 0 | 0 | 0 | 0 | 1 | 0 | 0 | 0 | 0 | 0 | 0 | | | | | | |
| 5846 | 06942 | 05845 | 1 | | 0 | 0 | 0 | 0 | 0 | 0 | 0 | 0 | 0 | 0 | 0 | 0 | 0 | 0 | 0 | 0 | 0 | 0 | 1 | 0 | 0 | 0 | 0 | 0 | 0 | | | | | | |
| 5847 | 06943 | 05846 | 7 | | 0 | 0 | 0 | 0 | 0 | 0 | 0 | 0 | 0 | 0 | 0 | 0 | 0 | 0 | 0 | 0 | 0 | 0 | 1 | 0 | 0 | 0 | 0 | 0 | 6 | | | | | | |
| 5848 | 06945 | 05847 | 1 | | 0 | 0 | 0 | 0 | 0 | 0 | 0 | 0 | 0 | 0 | 0 | 0 | 0 | 0 | 0 | 0 | 0 | 0 | 1 | 0 | 0 | 0 | 0 | 0 | 0 | | | | | | |
| 5849 | 06946 | 05848 | 1 | | 0 | 0 | 0 | 0 | 0 | 0 | 0 | 0 | 0 | 0 | 0 | 0 | 0 | 0 | 0 | 0 | 0 | 0 | 1 | 0 | 0 | 0 | 0 | 0 | 0 | | | | | | |
| 5850 | 06947 | 05849 | 2 | | 0 | 0 | 0 | 0 | 0 | 0 | 0 | 0 | 0 | 0 | 0 | 0 | 0 | 0 | 0 | 0 | 0 | 0 | 2 | 0 | 0 | 0 | 0 | 0 | 0 | | | | | | |
| 5851 | 06948 | 05850 | 1 | | 0 | 0 | 0 | 0 | 0 | 0 | 0 | 0 | 0 | 0 | 0 | 0 | 0 | 0 | 0 | 0 | 0 | 0 | 1 | 0 | 0 | 0 | 0 | 0 | 0 | | | | | | |
| 5852 | 06949 | 05851 | 1 | | 0 | 0 | 0 | 0 | 0 | 0 | 0 | 0 | 0 | 0 | 0 | 0 | 0 | 0 | 0 | 0 | 0 | 0 | 1 | 0 | 0 | 0 | 0 | 0 | 0 | | | | | | |
| 5853 | 06951 | 05852 | 1 | | 0 | 0 | 0 | 0 | 0 | 0 | 0 | 0 | 0 | 0 | 0 | 0 | 0 | 0 | 0 | 0 | 0 | 0 | 1 | 0 | 0 | 0 | 0 | 0 | 0 | | | | | | |

Table 163

| | A | B | C | E | G | I | K | M | O | Q | S | U | W | Y | AA | AC | AE | AG | AI | AK | AM | AO | AQ | AS | AU | AW | AY | BA | BC | BE | BF | BG | BH | BI | BK |
|---|---|---|---|---|---|---|---|---|---|---|---|---|---|---|---|---|---|---|---|---|---|---|---|---|---|---|---|---|---|---|---|---|---|---|---|
| 5854 | 06952 | 05853 | 1 | | 0 | 0 | 0 | 0 | 0 | 0 | 0 | 0 | 0 | 0 | 0 | 0 | 0 | 0 | 0 | 0 | 0 | 0 | 1 | 0 | 0 | 0 | 0 | 0 | 0 | | | | | | |
| 5855 | 06953 | 05854 | 1 | | 0 | 0 | 0 | 0 | 0 | 0 | 0 | 0 | 0 | 0 | 0 | 0 | 0 | 0 | 0 | 0 | 0 | 0 | 1 | 0 | 0 | 0 | 0 | 0 | 0 | | | | | | |
| 5856 | 06954 | 05855 | 2 | | 0 | 0 | 0 | 0 | 0 | 0 | 0 | 0 | 0 | 0 | 0 | 0 | 0 | 0 | 0 | 0 | 0 | 0 | 1 | 0 | 0 | 0 | 0 | 1 | 0 | | | | | | |
| 5857 | 06955 | 05856 | 1 | | 0 | 0 | 0 | 0 | 0 | 0 | 0 | 0 | 0 | 0 | 0 | 0 | 0 | 0 | 0 | 0 | 0 | 0 | 1 | 0 | 0 | 0 | 0 | 0 | 0 | | | | | | |
| 5858 | 06956 | 05857 | 1 | | 0 | 0 | 0 | 0 | 0 | 0 | 0 | 0 | 0 | 0 | 0 | 0 | 0 | 0 | 0 | 0 | 0 | 0 | 1 | 0 | 0 | 0 | 0 | 0 | 0 | | | | | | |
| 5859 | 06957 | 05858 | 2 | | 0 | 0 | 0 | 0 | 0 | 0 | 0 | 0 | 0 | 0 | 0 | 0 | 0 | 0 | 0 | 0 | 0 | 0 | 1 | 1 | 0 | 0 | 0 | 0 | 0 | | | | | | |
| 5860 | 06959 | 05859 | 1 | | 0 | 0 | 0 | 0 | 0 | 0 | 0 | 0 | 0 | 0 | 0 | 0 | 0 | 0 | 0 | 0 | 0 | 0 | 1 | 0 | 0 | 0 | 0 | 0 | 0 | | | | | | |
| 5861 | 06960 | 05860 | 1 | | 0 | 0 | 0 | 0 | 0 | 0 | 0 | 0 | 0 | 0 | 0 | 0 | 0 | 0 | 0 | 0 | 0 | 0 | 1 | 0 | 0 | 0 | 0 | 0 | 0 | M72709 | 100 | 84 | 1 | 1634 | 1717 |
| 5862 | 06961 | 05861 | 1 | | 0 | 0 | 0 | 0 | 0 | 0 | 0 | 0 | 0 | 0 | 0 | 0 | 0 | 0 | 0 | 0 | 0 | 0 | 1 | 0 | 0 | 0 | 0 | 0 | 0 | | | | | | |
| 5863 | 06963 | 05862 | 1 | | 0 | 0 | 0 | 0 | 0 | 0 | 0 | 0 | 0 | 0 | 0 | 0 | 0 | 0 | 0 | 0 | 0 | 0 | 1 | 0 | 0 | 0 | 0 | 0 | 0 | | | | | | |
| 5864 | 06964 | 05863 | 1 | | 0 | 0 | 0 | 0 | 0 | 0 | 0 | 0 | 0 | 0 | 0 | 0 | 0 | 0 | 0 | 0 | 0 | 0 | 1 | 0 | 0 | 0 | 0 | 0 | 0 | | | | | | |
| 5865 | 06965 | 05864 | 2 | | 0 | 0 | 0 | 0 | 0 | 0 | 0 | 0 | 0 | 0 | 0 | 0 | 0 | 0 | 0 | 0 | 0 | 0 | 1 | 1 | 0 | 0 | 0 | 0 | 0 | | | | | | |
| 5866 | 06966 | 05865 | 1 | | 0 | 0 | 0 | 0 | 0 | 0 | 0 | 0 | 0 | 0 | 0 | 0 | 0 | 0 | 0 | 0 | 0 | 0 | 1 | 0 | 0 | 0 | 0 | 0 | 0 | | | | | | |
| 5867 | 06967 | 05866 | 2 | | 0 | 0 | 0 | 0 | 0 | 0 | 0 | 0 | 0 | 0 | 0 | 0 | 0 | 0 | 0 | 0 | 0 | 0 | 2 | 0 | 0 | 0 | 0 | 0 | 0 | | | | | | |
| 5868 | 06969 | 05867 | 1 | | 0 | 0 | 0 | 0 | 0 | 0 | 0 | 0 | 0 | 0 | 0 | 0 | 0 | 0 | 0 | 0 | 0 | 0 | 1 | 0 | 0 | 0 | 0 | 0 | 0 | | | | | | |
| 5869 | 06971 | 05868 | 3 | | 0 | 0 | 0 | 0 | 0 | 0 | 0 | 0 | 0 | 0 | 0 | 0 | 0 | 1 | 0 | 0 | 0 | 0 | 1 | 0 | 0 | 1 | 0 | 0 | 0 | Y00698 | 93.7 | 284 | 1 | 2394 | 2759 |
| 5870 | 06972 | 05869 | 2 | | 0 | 0 | 0 | 0 | 0 | 0 | 0 | 0 | 0 | 0 | 0 | 0 | 0 | 0 | 0 | 0 | 0 | 0 | 1 | 0 | 0 | 1 | 0 | 0 | 0 | Z21966 | 96.8 | 344 | 1 | 1731 | 2182 |
| 5871 | 06973 | 05870 | 1 | | 0 | 0 | 0 | 0 | 0 | 0 | 0 | 0 | 0 | 0 | 0 | 0 | 0 | 0 | 0 | 0 | 0 | 0 | 1 | 0 | 0 | 0 | 0 | 0 | 0 | | | | | | |
| 5872 | 06975 | 05871 | 1 | | 0 | 0 | 0 | 0 | 0 | 0 | 0 | 0 | 0 | 0 | 0 | 0 | 0 | 0 | 0 | 0 | 0 | 0 | 1 | 0 | 0 | 0 | 0 | 0 | 0 | | | | | | |
| 5873 | 06977 | 05872 | 1 | | 0 | 0 | 0 | 0 | 0 | 0 | 0 | 0 | 0 | 0 | 0 | 0 | 0 | 0 | 0 | 0 | 0 | 0 | 1 | 0 | 0 | 0 | 0 | 0 | 0 | | | | | | |
| 5874 | 06978 | 05873 | 2 | | 0 | 0 | 0 | 0 | 0 | 0 | 0 | 0 | 0 | 0 | 0 | 0 | 0 | 0 | 0 | 0 | 0 | 0 | 2 | 0 | 0 | 0 | 0 | 0 | 0 | | | | | | |
| 5875 | 06979 | 05874 | 1 | | 0 | 0 | 0 | 0 | 0 | 0 | 0 | 0 | 0 | 0 | 0 | 0 | 0 | 0 | 0 | 0 | 0 | 0 | 1 | 0 | 0 | 0 | 0 | 0 | 0 | | | | | | |
| 5876 | 06981 | 05875 | 1 | | 0 | 0 | 0 | 0 | 0 | 0 | 0 | 0 | 0 | 0 | 0 | 0 | 0 | 0 | 0 | 0 | 0 | 0 | 1 | 0 | 0 | 0 | 0 | 0 | 0 | | | | | | |
| 5877 | 06982 | 05876 | 2 | | 0 | 0 | 0 | 0 | 0 | 0 | 0 | 0 | 0 | 0 | 0 | 0 | 0 | 0 | 0 | 0 | 0 | 0 | 2 | 0 | 0 | 0 | 0 | 0 | 0 | | | | | | |
| 5878 | 06983 | 05877 | 1 | | 0 | 0 | 0 | 0 | 0 | 0 | 0 | 0 | 0 | 0 | 0 | 0 | 0 | 0 | 0 | 0 | 0 | 0 | 1 | 0 | 0 | 0 | 0 | 0 | 0 | | | | | | |
| 5879 | 06984 | 05878 | 1 | | 0 | 0 | 0 | 0 | 0 | 0 | 0 | 0 | 0 | 0 | 0 | 0 | 0 | 0 | 0 | 0 | 0 | 0 | 1 | 0 | 0 | 0 | 0 | 0 | 0 | | | | | | |
| 5880 | 06985 | 05879 | 2 | | 0 | 0 | 0 | 0 | 0 | 0 | 0 | 0 | 0 | 0 | 0 | 0 | 0 | 0 | 0 | 0 | 0 | 0 | 1 | 0 | 0 | 0 | 0 | 0 | 1 | | | | | | |
| 5881 | 06986 | 05880 | 1 | | 0 | 0 | 0 | 0 | 0 | 0 | 0 | 0 | 0 | 0 | 0 | 0 | 0 | 0 | 0 | 0 | 0 | 0 | 1 | 0 | 0 | 0 | 0 | 0 | 0 | | | | | | |
| 5882 | 06987 | 05881 | 1 | | 0 | 0 | 0 | 0 | 0 | 0 | 0 | 0 | 0 | 0 | 0 | 0 | 0 | 0 | 0 | 0 | 0 | 0 | 1 | 0 | 0 | 0 | 0 | 0 | 0 | | | | | | |
| 5883 | 06988 | 05882 | 1 | | 0 | 0 | 0 | 0 | 0 | 0 | 0 | 0 | 0 | 0 | 0 | 0 | 0 | 0 | 0 | 0 | 0 | 0 | 1 | 0 | 0 | 0 | 0 | 0 | 0 | | | | | | |
| 5884 | 06989 | 05883 | 1 | | 0 | 0 | 0 | 0 | 0 | 0 | 0 | 0 | 0 | 0 | 0 | 0 | 0 | 0 | 0 | 0 | 0 | 0 | 1 | 0 | 0 | 0 | 0 | 0 | 0 | | | | | | |
| 5885 | 06990 | 05884 | 1 | | 0 | 0 | 0 | 0 | 0 | 0 | 0 | 0 | 0 | 0 | 0 | 0 | 0 | 0 | 0 | 0 | 0 | 0 | 1 | 0 | 0 | 0 | 0 | 0 | 0 | | | | | | |
| 5886 | 06991 | 05885 | 1 | | 0 | 0 | 0 | 0 | 0 | 0 | 0 | 0 | 0 | 0 | 0 | 0 | 0 | 0 | 0 | 0 | 0 | 0 | 1 | 0 | 0 | 0 | 0 | 0 | 0 | | | | | | |
| 5887 | 06992 | 05886 | 1 | | 0 | 0 | 0 | 0 | 0 | 0 | 0 | 0 | 0 | 0 | 0 | 0 | 0 | 0 | 0 | 0 | 0 | 0 | 1 | 0 | 0 | 0 | 0 | 0 | 0 | | | | | | |
| 5888 | 06993 | 05887 | 1 | | 0 | 0 | 0 | 0 | 0 | 0 | 0 | 0 | 0 | 0 | 0 | 0 | 0 | 0 | 0 | 0 | 0 | 0 | 1 | 0 | 0 | 0 | 0 | 0 | 0 | | | | | | |
| 5889 | 06994 | 05888 | 5 | | 0 | 0 | 0 | 0 | 0 | 0 | 0 | 0 | 0 | 0 | 0 | 0 | 0 | 0 | 0 | 0 | 0 | 0 | 1 | 0 | 0 | 4 | 0 | 0 | 0 | | | | | | |

Table 164

| | A | B | C | E | G | I | K | M | O | Q | S | U | W | Y | AA | AC | AE | AG | AI | AK | AM | AO | AQ | AS | AU | AW | AY | BA | BC | BE | BF | BG | BH | BI | BK |
|---|---|---|---|---|---|---|---|---|---|---|---|---|---|---|---|---|---|---|---|---|---|---|---|---|---|---|---|---|---|---|---|---|---|---|---|
| 5890 | 06995 | 05889 | 1 | | 0 | 0 | 0 | 0 | 0 | 0 | 0 | 0 | 0 | 0 | 0 | 0 | 0 | 0 | 0 | 0 | 0 | 0 | 1 | 0 | 0 | 0 | 0 | 0 | 0 | | | | | | |
| 5891 | 06996 | 05890 | 1 | | 0 | 0 | 0 | 0 | 0 | 0 | 0 | 0 | 0 | 0 | 0 | 0 | 0 | 0 | 0 | 0 | 0 | 0 | 1 | 0 | 0 | 0 | 0 | 0 | 0 | | | | | | |
| 5892 | 06997 | 05891 | 1 | | 0 | 0 | 0 | 0 | 0 | 0 | 0 | 0 | 0 | 0 | 0 | 0 | 0 | 0 | 0 | 0 | 0 | 0 | 1 | 0 | 0 | 0 | 0 | 0 | 0 | | | | | | |
| 5893 | 06998 | 05892 | 1 | | 0 | 0 | 0 | 0 | 0 | 0 | 0 | 0 | 0 | 0 | 0 | 0 | 0 | 0 | 0 | 0 | 0 | 0 | 1 | 0 | 0 | 0 | 0 | 0 | 0 | | | | | | |
| 5894 | 06999 | 05893 | 1 | | 0 | 0 | 0 | 0 | 0 | 0 | 0 | 0 | 0 | 0 | 0 | 0 | 0 | 0 | 0 | 0 | 0 | 0 | 1 | 0 | 0 | 0 | 0 | 0 | 0 | | | | | | |
| 5895 | 07000 | 05894 | 1 | | 0 | 0 | 0 | 0 | 0 | 0 | 0 | 0 | 0 | 0 | 0 | 0 | 0 | 0 | 0 | 0 | 0 | 0 | 1 | 0 | 0 | 0 | 0 | 0 | 0 | | | | | | |
| 5896 | 07001 | 05895 | 1 | | 0 | 0 | 0 | 0 | 0 | 0 | 0 | 0 | 0 | 0 | 0 | 0 | 0 | 0 | 0 | 0 | 0 | 0 | 1 | 0 | 0 | 0 | 0 | 0 | 0 | | | | | | |
| 5897 | 07002 | 05896 | 1 | | 0 | 0 | 0 | 0 | 0 | 0 | 0 | 0 | 0 | 0 | 0 | 0 | 0 | 0 | 0 | 0 | 0 | 0 | 1 | 0 | 0 | 0 | 0 | 0 | 0 | | | | | | |
| 5898 | 07003 | 05897 | 2 | | 0 | 0 | 0 | 0 | 0 | 0 | 0 | 0 | 0 | 0 | 0 | 0 | 0 | 0 | 0 | 0 | 0 | 0 | 1 | 0 | 1 | 0 | 0 | 0 | 0 | | | | | | |
| 5899 | 07004 | 05898 | 2 | | 0 | 0 | 0 | 0 | 0 | 0 | 0 | 0 | 0 | 0 | 0 | 0 | 0 | 0 | 0 | 0 | 0 | 0 | 2 | 0 | 0 | 0 | 0 | 0 | 0 | | | | | | |
| 5900 | 07005 | 05899 | 1 | | 0 | 0 | 0 | 0 | 0 | 0 | 0 | 0 | 0 | 0 | 0 | 0 | 0 | 0 | 0 | 0 | 0 | 0 | 1 | 0 | 0 | 0 | 0 | 0 | 0 | | | | | | |
| 5901 | 07006 | 05900 | 1 | | 0 | 0 | 0 | 0 | 0 | 0 | 0 | 0 | 0 | 0 | 0 | 0 | 0 | 0 | 0 | 0 | 0 | 0 | 1 | 0 | 0 | 0 | 0 | 0 | 0 | | | | | | |
| 5902 | 07007 | 05901 | 4 | | 0 | 0 | 0 | 0 | 0 | 0 | 0 | 0 | 0 | 0 | 0 | 0 | 0 | 0 | 0 | 0 | 0 | 0 | 1 | 3 | 0 | 0 | 0 | 0 | 0 | | | | | | |
| 5903 | 07008 | 05902 | 1 | | 0 | 0 | 0 | 0 | 0 | 0 | 0 | 0 | 0 | 0 | 0 | 0 | 0 | 0 | 0 | 0 | 0 | 0 | 1 | 0 | 0 | 0 | 0 | 0 | 0 | | | | | | |
| 5904 | 07009 | 05903 | 1 | | 0 | 0 | 0 | 0 | 0 | 0 | 0 | 0 | 0 | 0 | 0 | 0 | 0 | 0 | 0 | 0 | 0 | 0 | 1 | 0 | 0 | 0 | 0 | 0 | 0 | | | | | | |
| 5905 | 07011 | 05904 | 1 | | 0 | 0 | 0 | 0 | 0 | 0 | 0 | 0 | 0 | 0 | 0 | 0 | 0 | 0 | 0 | 0 | 0 | 0 | 1 | 0 | 0 | 0 | 0 | 0 | 0 | | | | | | |
| 5906 | 07012 | 05905 | 2 | | 0 | 0 | 0 | 0 | 0 | 0 | 0 | 0 | 0 | 0 | 0 | 0 | 0 | 0 | 0 | 0 | 0 | 0 | 1 | 0 | 0 | 0 | 1 | 0 | 0 | | | | | | |
| 5907 | 07013 | 05906 | 1 | | 0 | 0 | 0 | 0 | 0 | 0 | 0 | 0 | 0 | 0 | 0 | 0 | 0 | 0 | 0 | 0 | 0 | 0 | 1 | 0 | 0 | 0 | 0 | 0 | 0 | | | | | | |
| 5908 | 07014 | 05907 | 1 | | 0 | 0 | 0 | 0 | 0 | 0 | 0 | 0 | 0 | 0 | 0 | 0 | 0 | 0 | 0 | 0 | 0 | 0 | 1 | 0 | 0 | 0 | 0 | 0 | 0 | | | | | | |
| 5909 | 07015 | 05908 | 1 | | 0 | 0 | 0 | 0 | 0 | 0 | 0 | 0 | 0 | 0 | 0 | 0 | 0 | 0 | 0 | 0 | 0 | 0 | 1 | 0 | 0 | 0 | 0 | 0 | 0 | | | | | | |
| 5910 | 07016 | 05909 | 1 | | 0 | 0 | 0 | 0 | 0 | 0 | 0 | 0 | 0 | 0 | 0 | 0 | 0 | 0 | 0 | 0 | 0 | 0 | 1 | 0 | 0 | 0 | 0 | 0 | 0 | | | | | | |
| 5911 | 07017 | 05910 | 1 | | 0 | 0 | 0 | 0 | 0 | 0 | 0 | 0 | 0 | 0 | 0 | 0 | 0 | 0 | 0 | 0 | 0 | 0 | 1 | 0 | 0 | 0 | 0 | 0 | 0 | | | | | | |
| 5912 | 07018 | 05911 | 2 | | 0 | 0 | 0 | 0 | 0 | 0 | 0 | 0 | 0 | 0 | 0 | 0 | 0 | 0 | 0 | 0 | 0 | 0 | 2 | 0 | 0 | 0 | 0 | 0 | 0 | | | | | | |
| 5913 | 07020 | 05912 | 3 | | 0 | 0 | 0 | 0 | 0 | 0 | 0 | 0 | 0 | 0 | 0 | 0 | 0 | 0 | 0 | 0 | 0 | 0 | 2 | 0 | 0 | 0 | 1 | 0 | 0 | | | | | | |
| 5914 | 07021 | 05913 | 1 | | 0 | 0 | 0 | 0 | 0 | 0 | 0 | 0 | 0 | 0 | 0 | 0 | 0 | 0 | 0 | 0 | 0 | 0 | 1 | 0 | 0 | 0 | 0 | 0 | 0 | | | | | | |
| 5915 | 07022 | 05914 | 1 | | 0 | 0 | 0 | 0 | 0 | 0 | 0 | 0 | 0 | 0 | 0 | 0 | 0 | 0 | 0 | 0 | 0 | 0 | 1 | 0 | 0 | 0 | 0 | 0 | 0 | | | | | | |
| 5916 | 07023 | 05915 | 1 | | 0 | 0 | 0 | 0 | 0 | 0 | 0 | 0 | 0 | 0 | 0 | 0 | 0 | 0 | 0 | 0 | 0 | 0 | 1 | 0 | 0 | 0 | 0 | 0 | 0 | | | | | | |
| 5917 | 07025 | 05916 | 1 | | 0 | 0 | 0 | 0 | 0 | 0 | 0 | 0 | 0 | 0 | 0 | 0 | 0 | 0 | 0 | 0 | 0 | 0 | 1 | 0 | 0 | 0 | 0 | 0 | 0 | | | | | | |
| 5918 | 07026 | 05917 | 1 | | 0 | 0 | 0 | 0 | 0 | 0 | 0 | 0 | 0 | 0 | 0 | 0 | 0 | 0 | 0 | 0 | 0 | 0 | 1 | 0 | 0 | 0 | 0 | 0 | 0 | | | | | | |
| 5919 | 07027 | 05918 | 3 | | 0 | 0 | 0 | 0 | 0 | 0 | 0 | 0 | 0 | 0 | 0 | 0 | 0 | 0 | 0 | 0 | 0 | 0 | 2 | 0 | 1 | 0 | 0 | 0 | 0 | | | | | | |
| 5920 | 07028 | 05919 | 2 | | 0 | 0 | 0 | 0 | 0 | 0 | 0 | 0 | 0 | 0 | 0 | 0 | 0 | 0 | 0 | 0 | 0 | 0 | 1 | 1 | 0 | 0 | 0 | 0 | 0 | | | | | | |
| 5921 | 07029 | 05920 | 1 | | 0 | 0 | 0 | 0 | 0 | 0 | 0 | 0 | 0 | 0 | 0 | 0 | 0 | 0 | 0 | 0 | 0 | 0 | 1 | 0 | 0 | 0 | 0 | 0 | 0 | | | | | | |
| 5922 | 07030 | 05921 | 1 | | 0 | 0 | 0 | 0 | 0 | 0 | 0 | 0 | 0 | 0 | 0 | 0 | 0 | 0 | 0 | 0 | 0 | 0 | 1 | 0 | 0 | 0 | 0 | 0 | 0 | | | | | | |
| 5923 | 07031 | 05922 | 1 | | 0 | 0 | 0 | 0 | 0 | 0 | 0 | 0 | 0 | 0 | 0 | 0 | 0 | 0 | 0 | 0 | 0 | 0 | 1 | 0 | 0 | 0 | 0 | 0 | 0 | | | | | | |
| 5924 | 07032 | 05923 | 1 | | 0 | 0 | 0 | 0 | 0 | 0 | 0 | 0 | 0 | 0 | 0 | 0 | 0 | 0 | 0 | 0 | 0 | 0 | 1 | 0 | 0 | 0 | 0 | 0 | 0 | | | | | | |
| 5925 | 07033 | 05924 | 1 | | 0 | 0 | 0 | 0 | 0 | 0 | 0 | 0 | 0 | 0 | 0 | 0 | 0 | 0 | 0 | 0 | 0 | 0 | 1 | 0 | 0 | 0 | 0 | 0 | 0 | | | | | | |

Table 165

| | A | B | C | E | G | I | K | M | O | Q | S | U | W | Y | AA | AC | AF | AG | AI | AK | AM | AO | AQ | AS | AU | AW | AY | BA | BC | BE | BF | BG | BH | BI | BK |
|---|---|---|---|---|---|---|---|---|---|---|---|---|---|---|---|---|---|---|---|---|---|---|---|---|---|---|---|---|---|---|---|---|---|---|---|
| 5926 | 07034 | 05925 | 1 | | 0 | 0 | 0 | 0 | 0 | 0 | 0 | 0 | 0 | 0 | 0 | 0 | 0 | 0 | 0 | 0 | 0 | 0 | 1 | 0 | 0 | 0 | 0 | 0 | 0 | | | | | | |
| 5927 | 07035 | 05926 | 1 | | 0 | 0 | 0 | 0 | 0 | 0 | 0 | 0 | 0 | 0 | 0 | 0 | 0 | 0 | 0 | 0 | 0 | 0 | 1 | 0 | 0 | 0 | 0 | 0 | 0 | | | | | | |
| 5928 | 07036 | 05927 | 1 | | 0 | 0 | 0 | 0 | 0 | 0 | 0 | 0 | 0 | 0 | 0 | 0 | 0 | 0 | 0 | 0 | 0 | 0 | 1 | 0 | 0 | 0 | 0 | 0 | 0 | | | | | | |
| 5929 | 07037 | 05928 | 1 | | 0 | 0 | 0 | 0 | 0 | 0 | 0 | 0 | 0 | 0 | 0 | 0 | 0 | 0 | 0 | 0 | 0 | 0 | 1 | 0 | 0 | 0 | 0 | 0 | 0 | | | | | | |
| 5930 | 07038 | 05929 | 1 | | 0 | 0 | 0 | 0 | 0 | 0 | 0 | 0 | 0 | 0 | 0 | 0 | 0 | 0 | 0 | 0 | 0 | 0 | 1 | 0 | 0 | 0 | 0 | 0 | 0 | | | | | | |
| 5931 | 07039 | 05930 | 1 | | 0 | 0 | 0 | 0 | 0 | 0 | 0 | 0 | 0 | 0 | 0 | 0 | 0 | 0 | 0 | 0 | 0 | 0 | 1 | 0 | 0 | 0 | 0 | 0 | 0 | | | | | | |
| 5932 | 07040 | 05931 | 1 | | 0 | 0 | 0 | 0 | 0 | 0 | 0 | 0 | 0 | 0 | 0 | 0 | 0 | 0 | 0 | 0 | 0 | 0 | 1 | 0 | 0 | 0 | 0 | 0 | 0 | | | | | | |
| 5933 | 07041 | 05932 | 1 | | 0 | 0 | 0 | 0 | 0 | 0 | 0 | 0 | 0 | 0 | 0 | 0 | 0 | 0 | 0 | 0 | 0 | 0 | 1 | 0 | 0 | 0 | 0 | 0 | 0 | | | | | | |
| 5934 | 07043 | 05933 | 1 | | 0 | 0 | 0 | 0 | 0 | 0 | 0 | 0 | 0 | 0 | 0 | 0 | 0 | 0 | 0 | 0 | 0 | 0 | 1 | 0 | 0 | 0 | 0 | 0 | 0 | | | | | | |
| 5935 | 07044 | 05934 | 1 | | 0 | 0 | 0 | 0 | 0 | 0 | 0 | 0 | 0 | 0 | 0 | 0 | 0 | 0 | 0 | 0 | 0 | 0 | 1 | 0 | 0 | 0 | 0 | 0 | 0 | | | | | | |
| 5936 | 07046 | 05935 | 1 | | 0 | 0 | 0 | 0 | 0 | 0 | 0 | 0 | 0 | 0 | 0 | 0 | 0 | 0 | 0 | 0 | 0 | 0 | 1 | 0 | 0 | 0 | 0 | 0 | 0 | | | | | | |
| 5937 | 07047 | 05936 | 1 | | 0 | 0 | 0 | 0 | 0 | 0 | 0 | 0 | 0 | 0 | 0 | 0 | 0 | 0 | 0 | 0 | 0 | 0 | 1 | 0 | 0 | 0 | 0 | 0 | 0 | | | | | | |
| 5938 | 07048 | 05937 | 1 | | 0 | 0 | 0 | 0 | 0 | 0 | 0 | 0 | 0 | 0 | 0 | 0 | 0 | 0 | 0 | 0 | 0 | 0 | 1 | 0 | 0 | 0 | 0 | 0 | 0 | | | | | | |
| 5939 | 07049 | 05938 | 1 | | 0 | 0 | 0 | 0 | 0 | 0 | 0 | 0 | 0 | 0 | 0 | 0 | 0 | 0 | 0 | 0 | 0 | 0 | 1 | 0 | 0 | 0 | 0 | 0 | 0 | | | | | | |
| 5940 | 07050 | 05939 | 1 | | 0 | 0 | 0 | 0 | 0 | 0 | 0 | 0 | 0 | 0 | 0 | 0 | 0 | 0 | 0 | 0 | 0 | 0 | 1 | 0 | 0 | 0 | 0 | 0 | 0 | | | | | | |
| 5941 | 07053 | 05940 | 2 | | 0 | 0 | 0 | 0 | 0 | 0 | 0 | 0 | 0 | 0 | 0 | 0 | 0 | 0 | 0 | 0 | 0 | 0 | 1 | 0 | 0 | 1 | 0 | 0 | 0 | | | | | | |
| 5942 | 07054 | 05941 | 2 | | 0 | 0 | 0 | 0 | 0 | 0 | 0 | 0 | 0 | 0 | 0 | 0 | 0 | 0 | 0 | 0 | 0 | 0 | 1 | 0 | 1 | 0 | 0 | 0 | 0 | | | | | | |
| 5943 | 07055 | 05942 | 1 | | 0 | 0 | 0 | 0 | 0 | 0 | 0 | 0 | 0 | 0 | 0 | 0 | 0 | 0 | 0 | 0 | 0 | 0 | 1 | 0 | 0 | 0 | 0 | 0 | 0 | | | | | | |
| 5944 | 07056 | 05943 | 1 | | 0 | 0 | 0 | 0 | 0 | 0 | 0 | 0 | 0 | 0 | 0 | 0 | 0 | 0 | 0 | 0 | 0 | 0 | 1 | 0 | 0 | 0 | 0 | 0 | 0 | | | | | | |
| 5945 | 07057 | 05944 | 1 | | 0 | 0 | 0 | 0 | 0 | 0 | 0 | 0 | 0 | 0 | 0 | 0 | 0 | 0 | 0 | 0 | 0 | 0 | 1 | 0 | 0 | 0 | 0 | 0 | 0 | | | | | | |
| 5946 | 07058 | 05945 | 1 | | 0 | 0 | 0 | 0 | 0 | 0 | 0 | 0 | 0 | 0 | 0 | 0 | 0 | 0 | 0 | 0 | 0 | 0 | 1 | 0 | 0 | 0 | 0 | 0 | 0 | | | | | | |
| 5947 | 07059 | 05946 | 1 | | 0 | 0 | 0 | 0 | 0 | 0 | 0 | 0 | 0 | 0 | 0 | 0 | 0 | 0 | 0 | 0 | 0 | 0 | 1 | 0 | 0 | 0 | 0 | 0 | 0 | | | | | | |
| 5948 | 07060 | 05947 | 1 | | 0 | 0 | 0 | 0 | 0 | 0 | 0 | 0 | 0 | 0 | 0 | 0 | 0 | 0 | 0 | 0 | 0 | 0 | 1 | 0 | 0 | 0 | 0 | 0 | 0 | | | | | | |
| 5949 | 07061 | 05948 | 1 | | 0 | 0 | 0 | 0 | 0 | 0 | 0 | 0 | 0 | 0 | 0 | 0 | 0 | 0 | 0 | 0 | 0 | 0 | 1 | 0 | 0 | 0 | 0 | 0 | 0 | | | | | | |
| 5950 | 07062 | 05949 | 2 | | 0 | 0 | 0 | 0 | 0 | 0 | 0 | 0 | 0 | 0 | 0 | 0 | 0 | 0 | 0 | 0 | 0 | 0 | 2 | 0 | 0 | 0 | 0 | 0 | 0 | | | | | | |
| 5951 | 07063 | 05950 | 1 | | 0 | 0 | 0 | 0 | 0 | 0 | 0 | 0 | 0 | 0 | 0 | 0 | 0 | 0 | 0 | 0 | 0 | 0 | 1 | 0 | 0 | 0 | 0 | 0 | 0 | | | | | | |
| 5952 | 07065 | 05951 | 1 | | 0 | 0 | 0 | 0 | 0 | 0 | 0 | 0 | 0 | 0 | 0 | 0 | 0 | 0 | 0 | 0 | 0 | 0 | 1 | 0 | 0 | 0 | 0 | 0 | 0 | | | | | | |
| 5953 | 07066 | 05952 | 2 | | 0 | 0 | 0 | 0 | 0 | 0 | 0 | 0 | 0 | 0 | 0 | 0 | 0 | 0 | 0 | 0 | 0 | 0 | 1 | 0 | 0 | 0 | 1 | 0 | 0 | | | | | | |
| 5954 | 07067 | 05953 | 1 | | 0 | 0 | 0 | 0 | 0 | 0 | 0 | 0 | 0 | 0 | 0 | 0 | 0 | 0 | 0 | 0 | 0 | 0 | 1 | 0 | 0 | 0 | 0 | 0 | 0 | | | | | | |
| 5955 | 07068 | 05954 | 1 | | 0 | 0 | 0 | 0 | 0 | 0 | 0 | 0 | 0 | 0 | 0 | 0 | 0 | 0 | 0 | 0 | 0 | 0 | 1 | 0 | 0 | 0 | 0 | 0 | 0 | M34671 | 94 | 248 | 1 | 1332 | 1671 |
| 5956 | 07069 | 05955 | 1 | | 0 | 0 | 0 | 0 | 0 | 0 | 0 | 0 | 0 | 0 | 0 | 0 | 0 | 0 | 0 | 0 | 0 | 0 | 1 | 0 | 0 | 0 | 0 | 0 | 0 | D25269 | 93.3 | 134 | 1 | 147 | 410 |
| 5957 | 07071 | 05956 | 2 | | 0 | 0 | 0 | 0 | 0 | 0 | 0 | 0 | 0 | 0 | 0 | 0 | 0 | 0 | 0 | 0 | 0 | 0 | 1 | 1 | 0 | 0 | 0 | 0 | 0 | | | | | | |
| 5958 | 07072 | 05957 | 1 | | 0 | 0 | 0 | 0 | 0 | 0 | 0 | 0 | 0 | 0 | 0 | 0 | 0 | 0 | 0 | 0 | 0 | 0 | 1 | 0 | 0 | 0 | 0 | 0 | 0 | | | | | | |
| 5959 | 07074 | 05958 | 1 | | 0 | 0 | 0 | 0 | 0 | 0 | 0 | 0 | 0 | 0 | 0 | 0 | 0 | 0 | 0 | 0 | 0 | 0 | 1 | 0 | 0 | 0 | 0 | 0 | 0 | | | | | | |
| 5960 | 07075 | 05959 | 1 | | 0 | 0 | 0 | 0 | 0 | 0 | 0 | 0 | 0 | 0 | 0 | 0 | 0 | 0 | 0 | 0 | 0 | 0 | 1 | 0 | 0 | 0 | 0 | 0 | 0 | | | | | | |
| 5961 | 07076 | 05960 | 1 | | 0 | 0 | 0 | 0 | 0 | 0 | 0 | 0 | 0 | 0 | 0 | 0 | 0 | 0 | 0 | 0 | 0 | 0 | 1 | 0 | 0 | 0 | 0 | 0 | 0 | | | | | | |

Table 166

| | A | B | C | E | G | I | K | M | O | Q | S | U | W | Y | AA | AC | AE | AG | AI | AK | AM | AO | AQ | AS | AU | AW | AY | BA | BC | BE | BF | BG | BH | BI | BK |
|---|---|---|---|---|---|---|---|---|---|---|---|---|---|---|---|---|---|---|---|---|---|---|---|---|---|---|---|---|---|---|---|---|---|---|---|
| 5962 | 07079 | 05961 | 1 | | 0 | 0 | 0 | 0 | 0 | 0 | 0 | 0 | 0 | 0 | 0 | 0 | 0 | 0 | 0 | 0 | 0 | 0 | 1 | 0 | 0 | 0 | 0 | 0 | 0 | | | | | | |
| 5963 | 07080 | 05962 | 2 | | 0 | 0 | 0 | 0 | 0 | 0 | 0 | 0 | 0 | 0 | 0 | 0 | 0 | 0 | 0 | 0 | 0 | 0 | 1 | 1 | 0 | 0 | 0 | 0 | 0 | | | | | | |
| 5964 | 07081 | 05963 | 1 | | 0 | 0 | 0 | 0 | 0 | 0 | 0 | 0 | 0 | 0 | 0 | 0 | 0 | 0 | 0 | 0 | 0 | 0 | 1 | 0 | 0 | 0 | 0 | 0 | 0 | | | | | | |
| 5965 | 07082 | 05964 | 1 | | 0 | 0 | 0 | 0 | 0 | 0 | 0 | 0 | 0 | 0 | 0 | 0 | 0 | 0 | 0 | 0 | 0 | 0 | 1 | 0 | 0 | 0 | 0 | 0 | 0 | | | | | | |
| 5966 | 07083 | 05965 | 3 | | 0 | 0 | 0 | 0 | 0 | 0 | 0 | 0 | 0 | 0 | 0 | 0 | 0 | 0 | 0 | 0 | 0 | 0 | 1 | 0 | 1 | 0 | 1 | 0 | 0 | | | | | | |
| 5967 | 07084 | 05966 | 1 | | 0 | 0 | 0 | 0 | 0 | 0 | 0 | 0 | 0 | 0 | 0 | 0 | 0 | 0 | 0 | 0 | 0 | 0 | 1 | 0 | 0 | 0 | 0 | 0 | 0 | | | | | | |
| 5968 | 07085 | 05967 | 1 | | 0 | 0 | 0 | 0 | 0 | 0 | 0 | 0 | 0 | 0 | 0 | 0 | 0 | 0 | 0 | 0 | 0 | 0 | 1 | 0 | 0 | 0 | 0 | 0 | 0 | | | | | | |
| 5969 | 07086 | 05968 | 1 | | 0 | 0 | 0 | 0 | 0 | 0 | 0 | 0 | 0 | 0 | 0 | 0 | 0 | 0 | 0 | 0 | 0 | 0 | 1 | 0 | 0 | 0 | 0 | 0 | 0 | | | | | | |
| 5970 | 07087 | 05969 | 1 | | 0 | 0 | 0 | 0 | 0 | 0 | 0 | 0 | 0 | 0 | 0 | 0 | 0 | 0 | 0 | 0 | 0 | 0 | 1 | 0 | 0 | 0 | 0 | 0 | 0 | | | | | | |
| 5971 | 07088 | 05970 | 1 | | 0 | 0 | 0 | 0 | 0 | 0 | 0 | 0 | 0 | 0 | 0 | 0 | 0 | 0 | 0 | 0 | 0 | 0 | 1 | 0 | 0 | 0 | 0 | 0 | 0 | | | | | | |
| 5972 | 07089 | 05971 | 1 | | 0 | 0 | 0 | 0 | 0 | 0 | 0 | 0 | 0 | 0 | 0 | 0 | 0 | 0 | 0 | 0 | 0 | 0 | 1 | 0 | 0 | 0 | 0 | 0 | 0 | | | | | | |
| 5973 | 07091 | 05972 | 1 | | 0 | 0 | 0 | 0 | 0 | 0 | 0 | 0 | 0 | 0 | 0 | 0 | 0 | 0 | 0 | 0 | 0 | 0 | 1 | 0 | 0 | 0 | 0 | 0 | 0 | | | | | | |
| 5974 | 07093 | 05973 | 1 | | 0 | 0 | 0 | 0 | 0 | 0 | 0 | 0 | 0 | 0 | 0 | 0 | 0 | 0 | 0 | 0 | 0 | 0 | 1 | 0 | 0 | 0 | 0 | 0 | 0 | | | | | | |
| 5975 | 07094 | 05974 | 1 | | 0 | 0 | 0 | 0 | 0 | 0 | 0 | 0 | 0 | 0 | 0 | 0 | 0 | 0 | 0 | 0 | 0 | 0 | 1 | 0 | 0 | 0 | 0 | 0 | 0 | | | | | | |
| 5976 | 07095 | 05975 | 2 | | 0 | 0 | 0 | 0 | 0 | 0 | 0 | 0 | 0 | 0 | 0 | 0 | 0 | 0 | 0 | 0 | 0 | 0 | 1 | 1 | 0 | 0 | 0 | 0 | 0 | | | | | | |
| 5977 | 07096 | 05976 | 1 | | 0 | 0 | 0 | 0 | 0 | 0 | 0 | 0 | 0 | 0 | 0 | 0 | 0 | 0 | 0 | 0 | 0 | 0 | 1 | 0 | 0 | 0 | 0 | 0 | 0 | | | | | | |
| 5978 | 07097 | 05977 | 1 | | 0 | 0 | 0 | 0 | 0 | 0 | 0 | 0 | 0 | 0 | 0 | 0 | 0 | 0 | 0 | 0 | 0 | 0 | 1 | 0 | 0 | 0 | 0 | 0 | 0 | | | | | | |
| 5979 | 07098 | 05978 | 1 | | 0 | 0 | 0 | 0 | 0 | 0 | 0 | 0 | 0 | 0 | 0 | 0 | 0 | 0 | 0 | 0 | 0 | 0 | 1 | 0 | 0 | 0 | 0 | 0 | 0 | | | | | | |
| 5980 | 07099 | 05979 | 1 | | 0 | 0 | 0 | 0 | 0 | 0 | 0 | 0 | 0 | 0 | 0 | 0 | 0 | 0 | 0 | 0 | 0 | 0 | 1 | 0 | 0 | 0 | 0 | 0 | 0 | | | | | | |
| 5981 | 07100 | 05980 | 1 | | 0 | 0 | 0 | 0 | 0 | 0 | 0 | 0 | 0 | 0 | 0 | 0 | 0 | 0 | 0 | 0 | 0 | 0 | 1 | 0 | 0 | 0 | 0 | 0 | 0 | | | | | | |
| 5982 | 07101 | 05981 | 1 | | 0 | 0 | 0 | 0 | 0 | 0 | 0 | 0 | 0 | 0 | 0 | 0 | 0 | 0 | 0 | 0 | 0 | 0 | 1 | 0 | 0 | 0 | 0 | 0 | 0 | | | | | | |
| 5983 | 07102 | 05982 | 1 | | 0 | 0 | 0 | 0 | 0 | 0 | 0 | 0 | 0 | 0 | 0 | 0 | 0 | 0 | 0 | 0 | 0 | 0 | 1 | 0 | 0 | 0 | 0 | 0 | 0 | | | | | | |
| 5984 | 07103 | 05983 | 1 | | 0 | 0 | 0 | 0 | 0 | 0 | 0 | 0 | 0 | 0 | 0 | 0 | 0 | 0 | 0 | 0 | 0 | 0 | 1 | 0 | 0 | 0 | 0 | 0 | 0 | | | | | | |
| 5985 | 07104 | 05984 | 1 | | 0 | 0 | 0 | 0 | 0 | 0 | 0 | 0 | 0 | 0 | 0 | 0 | 0 | 0 | 0 | 0 | 0 | 0 | 1 | 0 | 0 | 0 | 0 | 0 | 0 | | | | | | |
| 5986 | 07105 | 05985 | 2 | | 0 | 0 | 0 | 0 | 0 | 0 | 0 | 0 | 0 | 0 | 0 | 0 | 0 | 0 | 0 | 0 | 0 | 0 | 2 | 0 | 0 | 0 | 0 | 0 | 0 | | | | | | |
| 5987 | 07106 | 05986 | 3 | | 0 | 0 | 0 | 0 | 0 | 0 | 0 | 0 | 0 | 0 | 0 | 0 | 0 | 0 | 0 | 0 | 0 | 0 | 1 | 0 | 1 | 0 | 0 | 0 | 1 | | | | | | |
| 5988 | 07108 | 05987 | 1 | | 0 | 0 | 0 | 0 | 0 | 0 | 0 | 0 | 0 | 0 | 0 | 0 | 0 | 0 | 0 | 0 | 0 | 0 | 1 | 0 | 0 | 0 | 0 | 0 | 0 | | | | | | |
| 5989 | 07110 | 05988 | 1 | | 0 | 0 | 0 | 0 | 0 | 0 | 0 | 0 | 0 | 0 | 0 | 0 | 0 | 0 | 0 | 0 | 0 | 0 | 1 | 0 | 0 | 0 | 0 | 0 | 0 | | | | | | |
| 5990 | 07111 | 05989 | 1 | | 0 | 0 | 0 | 0 | 0 | 0 | 0 | 0 | 0 | 0 | 0 | 0 | 0 | 0 | 0 | 0 | 0 | 0 | 1 | 0 | 0 | 0 | 0 | 0 | 0 | | | | | | |
| 5991 | 07112 | 05990 | 1 | | 0 | 0 | 0 | 0 | 0 | 0 | 0 | 0 | 0 | 0 | 0 | 0 | 0 | 0 | 0 | 0 | 0 | 0 | 1 | 0 | 0 | 0 | 0 | 0 | 0 | | | | | | |
| 5992 | 07113 | 05991 | 1 | | 0 | 0 | 0 | 0 | 0 | 0 | 0 | 0 | 0 | 0 | 0 | 0 | 0 | 0 | 0 | 0 | 0 | 0 | 1 | 0 | 0 | 0 | 0 | 0 | 0 | X14767 | 96 | 124 | 1 | 1743 | 1866 |
| 5993 | 07114 | 05992 | 2 | | 0 | 0 | 0 | 0 | 0 | 0 | 0 | 0 | 0 | 0 | 0 | 0 | 0 | 0 | 0 | 0 | 0 | 0 | 1 | 0 | 0 | 0 | 0 | 1 | 0 | | | | | | |
| 5994 | 07115 | 05993 | 1 | | 0 | 0 | 0 | 0 | 0 | 0 | 0 | 0 | 0 | 0 | 0 | 0 | 0 | 0 | 0 | 0 | 0 | 0 | 1 | 0 | 0 | 0 | 0 | 0 | 0 | | | | | | |
| 5995 | 07116 | 05994 | 1 | | 0 | 0 | 0 | 0 | 0 | 0 | 0 | 0 | 0 | 0 | 0 | 0 | 0 | 0 | 0 | 0 | 0 | 0 | 1 | 0 | 0 | 0 | 0 | 0 | 0 | | | | | | |
| 5996 | 07117 | 05995 | 1 | | 0 | 0 | 0 | 0 | 0 | 0 | 0 | 0 | 0 | 0 | 0 | 0 | 0 | 0 | 0 | 0 | 0 | 0 | 1 | 0 | 0 | 0 | 0 | 0 | 0 | | | | | | |
| 5997 | 07118 | 05996 | 4 | | 0 | 0 | 0 | 0 | 0 | 0 | 0 | 0 | 0 | 0 | 0 | 0 | 0 | 0 | 0 | 0 | 0 | 0 | 2 | 0 | 1 | 1 | 0 | 0 | 0 | | | | | | |

Table 167

| | A | B | C | E | G | I | K | M | O | Q | S | U | W | Y | AA | AC | AE | AG | AI | AK | AM | AO | AQ | AS | AU | AW | AY | BA | BC | BE | BF | BG | BH | BI | BK |
|---|---|---|---|---|---|---|---|---|---|---|---|---|---|---|---|---|---|---|---|---|---|---|---|---|---|---|---|---|---|---|---|---|---|---|---|
| 5998 | 07119 | 05997 | 1 | | 0 | 0 | 0 | 0 | 0 | 0 | 0 | 0 | 0 | 0 | 0 | 0 | 0 | 0 | 0 | 0 | 0 | 0 | 1 | 0 | 0 | 0 | 0 | 0 | 0 | | | | | | |
| 5999 | 07120 | 05998 | 1 | | 0 | 0 | 0 | 0 | 0 | 0 | 0 | 0 | 0 | 0 | 0 | 0 | 0 | 0 | 0 | 0 | 0 | 0 | 1 | 0 | 0 | 0 | 0 | 0 | 0 | | | | | | |
| 6000 | 07121 | 05999 | 1 | | 0 | 0 | 0 | 0 | 0 | 0 | 0 | 0 | 0 | 0 | 0 | 0 | 0 | 0 | 0 | 0 | 0 | 0 | 1 | 0 | 0 | 0 | 0 | 0 | 0 | | | | | | |
| 6001 | 07122 | 06000 | 1 | | 0 | 0 | 0 | 0 | 0 | 0 | 0 | 0 | 0 | 0 | 0 | 0 | 0 | 0 | 0 | 0 | 0 | 0 | 1 | 0 | 0 | 0 | 0 | 0 | 0 | | | | | | |
| 6002 | 07123 | 06001 | 1 | | 0 | 0 | 0 | 0 | 0 | 0 | 0 | 0 | 0 | 0 | 0 | 0 | 0 | 0 | 0 | 0 | 0 | 0 | 1 | 0 | 0 | 0 | 0 | 0 | 0 | | | | | | |
| 6003 | 07124 | 06002 | 1 | | 0 | 0 | 0 | 0 | 0 | 0 | 0 | 0 | 0 | 0 | 0 | 0 | 0 | 0 | 0 | 0 | 0 | 0 | 1 | 0 | 0 | 0 | 0 | 0 | 0 | | | | | | |
| 6004 | 07125 | 06003 | 1 | | 0 | 0 | 0 | 0 | 0 | 0 | 0 | 0 | 0 | 0 | 0 | 0 | 0 | 0 | 0 | 0 | 0 | 0 | 1 | 0 | 0 | 0 | 0 | 0 | 0 | | | | | | |
| 6005 | 07126 | 06004 | 1 | | 0 | 0 | 0 | 0 | 0 | 0 | 0 | 0 | 0 | 0 | 0 | 0 | 0 | 0 | 0 | 0 | 0 | 0 | 1 | 0 | 0 | 0 | 0 | 0 | 0 | | | | | | |
| 6006 | 07127 | 06005 | 1 | | 0 | 0 | 0 | 0 | 0 | 0 | 0 | 0 | 0 | 0 | 0 | 0 | 0 | 0 | 0 | 0 | 0 | 0 | 1 | 0 | 0 | 0 | 0 | 0 | 0 | | | | | | |
| 6007 | 07128 | 06006 | 1 | | 0 | 0 | 0 | 0 | 0 | 0 | 0 | 0 | 0 | 0 | 0 | 0 | 0 | 0 | 0 | 0 | 0 | 0 | 1 | 0 | 0 | 0 | 0 | 0 | 0 | | | | | | |
| 6008 | 07129 | 06007 | 1 | | 0 | 0 | 0 | 0 | 0 | 0 | 0 | 0 | 0 | 0 | 0 | 0 | 0 | 0 | 0 | 0 | 0 | 0 | 1 | 0 | 0 | 0 | 0 | 0 | 0 | M16447 | 91.6 | 227 | 1 | 725 | 1550 |
| 6009 | 07130 | 06008 | 1 | | 0 | 0 | 0 | 0 | 0 | 0 | 0 | 0 | 0 | 0 | 0 | 0 | 0 | 0 | 0 | 0 | 0 | 0 | 1 | 0 | 0 | 0 | 0 | 0 | 0 | | | | | | |
| 6010 | 07131 | 06009 | 1 | | 0 | 0 | 0 | 0 | 0 | 0 | 0 | 0 | 0 | 0 | 0 | 0 | 0 | 0 | 0 | 0 | 0 | 0 | 1 | 0 | 0 | 0 | 0 | 0 | 0 | | | | | | |
| 6011 | 07132 | 06010 | 1 | | 0 | 0 | 0 | 0 | 0 | 0 | 0 | 0 | 0 | 0 | 0 | 0 | 0 | 0 | 0 | 0 | 0 | 0 | 1 | 0 | 0 | 0 | 0 | 0 | 0 | | | | | | |
| 6012 | 07133 | 06011 | 1 | | 0 | 0 | 0 | 0 | 0 | 0 | 0 | 0 | 0 | 0 | 0 | 0 | 0 | 0 | 0 | 0 | 0 | 0 | 1 | 0 | 0 | 0 | 0 | 0 | 0 | | | | | | |
| 6013 | 07134 | 06012 | 1 | | 0 | 0 | 0 | 0 | 0 | 0 | 0 | 0 | 0 | 0 | 0 | 0 | 0 | 0 | 0 | 0 | 0 | 0 | 1 | 0 | 0 | 0 | 0 | 0 | 0 | | | | | | |
| 6014 | 07135 | 06013 | 2 | | 0 | 0 | 0 | 0 | 0 | 0 | 0 | 0 | 0 | 0 | 0 | 0 | 0 | 0 | 0 | 0 | 0 | 0 | 1 | 1 | 0 | 0 | 0 | 0 | 0 | | | | | | |
| 6015 | 07136 | 06014 | 2 | | 0 | 0 | 0 | 0 | 0 | 0 | 0 | 0 | 0 | 0 | 0 | 0 | 0 | 0 | 0 | 0 | 0 | 0 | 2 | 0 | 0 | 0 | 0 | 0 | 0 | | | | | | |
| 6016 | 07137 | 06015 | 1 | | 0 | 0 | 0 | 0 | 0 | 0 | 0 | 0 | 0 | 0 | 0 | 0 | 0 | 0 | 0 | 0 | 0 | 0 | 1 | 0 | 0 | 0 | 0 | 0 | 0 | | | | | | |
| 6017 | 07138 | 06016 | 1 | | 0 | 0 | 0 | 0 | 0 | 0 | 0 | 0 | 0 | 0 | 0 | 0 | 0 | 0 | 0 | 0 | 0 | 0 | 1 | 0 | 0 | 0 | 0 | 0 | 0 | | | | | | |
| 6018 | 07139 | 06017 | 2 | | 0 | 0 | 0 | 0 | 0 | 0 | 0 | 0 | 0 | 0 | 0 | 0 | 0 | 0 | 0 | 0 | 0 | 0 | 2 | 0 | 0 | 0 | 0 | 0 | 0 | | | | | | |
| 6019 | 07140 | 06018 | 3 | | 0 | 0 | 0 | 0 | 0 | 0 | 0 | 0 | 0 | 0 | 0 | 0 | 0 | 0 | 0 | 0 | 0 | 0 | 1 | 2 | 0 | 0 | 0 | 0 | 0 | | | | | | |
| 6020 | 07141 | 06019 | 1 | | 0 | 0 | 0 | 0 | 0 | 0 | 0 | 0 | 0 | 0 | 0 | 0 | 0 | 0 | 0 | 0 | 0 | 0 | 1 | 0 | 0 | 0 | 0 | 0 | 0 | | | | | | |
| 6021 | 07142 | 06020 | 1 | | 0 | 0 | 0 | 0 | 0 | 0 | 0 | 0 | 0 | 0 | 0 | 0 | 0 | 0 | 0 | 0 | 0 | 0 | 1 | 0 | 0 | 0 | 0 | 0 | 0 | | | | | | |
| 6022 | 07143 | 06021 | 1 | | 0 | 0 | 0 | 0 | 0 | 0 | 0 | 0 | 0 | 0 | 0 | 0 | 0 | 0 | 0 | 0 | 0 | 0 | 1 | 0 | 0 | 0 | 0 | 0 | 0 | | | | | | |
| 6023 | 07144 | 06022 | 2 | | 0 | 0 | 0 | 0 | 0 | 0 | 0 | 0 | 0 | 0 | 0 | 0 | 0 | 0 | 0 | 0 | 0 | 0 | 2 | 0 | 0 | 0 | 0 | 0 | 0 | | | | | | |
| 6024 | 07145 | 06023 | 1 | | 0 | 0 | 0 | 0 | 0 | 0 | 0 | 0 | 0 | 0 | 0 | 0 | 0 | 0 | 0 | 0 | 0 | 0 | 1 | 0 | 0 | 0 | 0 | 0 | 0 | | | | | | |
| 6025 | 07146 | 06024 | 1 | | 0 | 0 | 0 | 0 | 0 | 0 | 0 | 0 | 0 | 0 | 0 | 0 | 0 | 0 | 0 | 0 | 0 | 0 | 1 | 0 | 0 | 0 | 0 | 0 | 0 | | | | | | |
| 6026 | 07147 | 06025 | 1 | | 0 | 0 | 0 | 0 | 0 | 0 | 0 | 0 | 0 | 0 | 0 | 0 | 0 | 0 | 0 | 0 | 0 | 0 | 1 | 0 | 0 | 0 | 0 | 0 | 0 | | | | | | |
| 6027 | 07150 | 06026 | 1 | | 0 | 0 | 0 | 0 | 0 | 0 | 0 | 0 | 0 | 0 | 0 | 0 | 0 | 0 | 0 | 0 | 0 | 0 | 1 | 0 | 0 | 0 | 0 | 0 | 0 | | | | | | |
| 6028 | 07151 | 06027 | 1 | | 0 | 0 | 0 | 0 | 0 | 0 | 0 | 0 | 0 | 0 | 0 | 0 | 0 | 0 | 0 | 0 | 0 | 0 | 1 | 0 | 0 | 0 | 0 | 0 | 0 | | | | | | |
| 6029 | 07152 | 06028 | 1 | | 0 | 0 | 0 | 0 | 0 | 0 | 0 | 0 | 0 | 0 | 0 | 0 | 0 | 0 | 0 | 0 | 0 | 0 | 1 | 0 | 0 | 0 | 0 | 0 | 0 | | | | | | |
| 6030 | 07153 | 06029 | 1 | | 0 | 0 | 0 | 0 | 0 | 0 | 0 | 0 | 0 | 0 | 0 | 0 | 0 | 0 | 0 | 0 | 0 | 0 | 1 | 0 | 0 | 0 | 0 | 0 | 0 | | | | | | |
| 6031 | 07154 | 06030 | 1 | | 0 | 0 | 0 | 0 | 0 | 0 | 0 | 0 | 0 | 0 | 0 | 0 | 0 | 0 | 0 | 0 | 0 | 0 | 1 | 0 | 0 | 0 | 0 | 0 | 0 | | | | | | |
| 6032 | 07156 | 06031 | 1 | | 0 | 0 | 0 | 0 | 0 | 0 | 0 | 0 | 0 | 0 | 0 | 0 | 0 | 0 | 0 | 0 | 0 | 0 | 1 | 0 | 0 | 0 | 0 | 0 | 0 | | | | | | |
| 6033 | 07157 | 06032 | 1 | | 0 | 0 | 0 | 0 | 0 | 0 | 0 | 0 | 0 | 0 | 0 | 0 | 0 | 0 | 0 | 0 | 0 | 0 | 1 | 0 | 0 | 0 | 0 | 0 | 0 | | | | | | |

Table 168

| | A | B | C | E | G | I | K | M | O | Q | S | U | W | Y | AA | AC | AE | AG | AI | AK | AM | AO | AQ | AS | AU | AW | AY | BA | BC | BE | BF | BG | BH | BI | BK |
|---|---|---|---|---|---|---|---|---|---|---|---|---|---|---|---|---|---|---|---|---|---|---|---|---|---|---|---|---|---|---|---|---|---|---|---|
| 6034 | 07158 | 06033 | 1 | | 0 | 0 | 0 | 0 | 0 | 0 | 0 | 0 | 0 | 0 | 0 | 0 | 0 | 0 | 0 | 0 | 0 | 0 | 1 | 0 | 0 | 0 | 0 | 0 | 0 | | | | | | |
| 6035 | 07160 | 06034 | 1 | | 0 | 0 | 0 | 0 | 0 | 0 | 0 | 0 | 0 | 0 | 0 | 0 | 0 | 0 | 0 | 0 | 0 | 0 | 1 | 0 | 0 | 0 | 0 | 0 | 0 | | | | | | |
| 6036 | 07161 | 06035 | 1 | | 0 | 0 | 0 | 0 | 0 | 0 | 0 | 0 | 0 | 0 | 0 | 0 | 0 | 0 | 0 | 0 | 0 | 0 | 1 | 0 | 0 | 0 | 0 | 0 | 0 | | | | | | |
| 6037 | 07162 | 06036 | 2 | | 0 | 0 | 0 | 0 | 0 | 0 | 0 | 0 | 0 | 0 | 0 | 0 | 0 | 0 | 0 | 0 | 0 | 0 | 1 | 0 | 0 | 1 | 0 | 0 | 0 | | | | | | |
| 6038 | 07163 | 06037 | 1 | | 0 | 0 | 0 | 0 | 0 | 0 | 0 | 0 | 0 | 0 | 0 | 0 | 0 | 0 | 0 | 0 | 0 | 0 | 1 | 0 | 0 | 0 | 0 | 0 | 0 | | | | | | |
| 6039 | 07164 | 06038 | 1 | | 0 | 0 | 0 | 0 | 0 | 0 | 0 | 0 | 0 | 0 | 0 | 0 | 0 | 0 | 0 | 0 | 0 | 0 | 1 | 0 | 0 | 0 | 0 | 0 | 0 | | | | | | |
| 6040 | 07165 | 06039 | 2 | | 0 | 0 | 0 | 0 | 0 | 0 | 0 | 0 | 0 | 0 | 0 | 0 | 0 | 0 | 0 | 0 | 0 | 0 | 1 | 0 | 0 | 0 | 0 | 1 | 0 | | | | | | |
| 6041 | 07166 | 06040 | 2 | | 0 | 0 | 0 | 0 | 0 | 0 | 0 | 0 | 0 | 0 | 0 | 0 | 0 | 0 | 0 | 0 | 0 | 0 | 1 | 0 | 1 | 0 | 0 | 0 | 0 | | | | | | |
| 6042 | 07167 | 06041 | 1 | | 0 | 0 | 0 | 0 | 0 | 0 | 0 | 0 | 0 | 0 | 0 | 0 | 0 | 0 | 0 | 0 | 0 | 0 | 1 | 0 | 0 | 0 | 0 | 0 | 0 | | | | | | |
| 6043 | 07169 | 06042 | 1 | | 0 | 0 | 0 | 0 | 0 | 0 | 0 | 0 | 0 | 0 | 0 | 0 | 0 | 0 | 0 | 0 | 0 | 0 | 1 | 0 | 0 | 0 | 0 | 0 | 0 | | | | | | |
| 6044 | 07170 | 06043 | 1 | | 0 | 0 | 0 | 0 | 0 | 0 | 0 | 0 | 0 | 0 | 0 | 0 | 0 | 0 | 0 | 0 | 0 | 0 | 1 | 0 | 0 | 0 | 0 | 0 | 0 | | | | | | |
| 6045 | 07171 | 06044 | 2 | | 0 | 0 | 0 | 0 | 0 | 0 | 0 | 0 | 0 | 0 | 0 | 0 | 0 | 0 | 0 | 0 | 0 | 0 | 1 | 1 | 0 | 0 | 0 | 0 | 0 | | | | | | |
| 6046 | 07173 | 06045 | 1 | | 0 | 0 | 0 | 0 | 0 | 0 | 0 | 0 | 0 | 0 | 0 | 0 | 0 | 0 | 0 | 0 | 0 | 0 | 1 | 0 | 0 | 0 | 0 | 0 | 0 | | | | | | |
| 6047 | 07174 | 06046 | 2 | | 0 | 0 | 0 | 0 | 0 | 0 | 0 | 0 | 0 | 0 | 0 | 0 | 0 | 0 | 0 | 0 | 0 | 0 | 1 | 0 | 0 | 0 | 0 | 0 | 1 | | | | | | |
| 6048 | 07175 | 06047 | 2 | | 0 | 0 | 0 | 0 | 0 | 0 | 0 | 0 | 0 | 0 | 0 | 0 | 0 | 0 | 0 | 0 | 0 | 0 | 1 | 0 | 0 | 0 | 0 | 0 | 1 | | | | | | |
| 6049 | 07178 | 06048 | 1 | | 0 | 0 | 0 | 0 | 0 | 0 | 0 | 0 | 0 | 0 | 0 | 0 | 0 | 0 | 0 | 0 | 0 | 0 | 1 | 0 | 0 | 0 | 0 | 0 | 0 | | | | | | |
| 6050 | 07179 | 06049 | 1 | | 0 | 0 | 0 | 0 | 0 | 0 | 0 | 0 | 0 | 0 | 0 | 0 | 0 | 0 | 0 | 0 | 0 | 0 | 1 | 0 | 0 | 0 | 0 | 0 | 0 | | | | | | |
| 6051 | 07180 | 06050 | 1 | | 0 | 0 | 0 | 0 | 0 | 0 | 0 | 0 | 0 | 0 | 0 | 0 | 0 | 0 | 0 | 0 | 0 | 0 | 1 | 0 | 0 | 0 | 0 | 0 | 0 | M32219 | 95.7 | 326 | 1 | 312 | 765 |
| 6052 | 07181 | 06051 | 1 | | 0 | 0 | 0 | 0 | 0 | 0 | 0 | 0 | 0 | 0 | 0 | 0 | 0 | 0 | 0 | 0 | 0 | 0 | 1 | 0 | 0 | 0 | 0 | 0 | 0 | | | | | | |
| 6053 | 07182 | 06052 | 1 | | 0 | 0 | 0 | 0 | 0 | 0 | 0 | 0 | 0 | 0 | 0 | 0 | 0 | 0 | 0 | 0 | 0 | 0 | 1 | 0 | 0 | 0 | 0 | 0 | 0 | | | | | | |
| 6054 | 07183 | 06053 | 1 | | 0 | 0 | 0 | 0 | 0 | 0 | 0 | 0 | 0 | 0 | 0 | 0 | 0 | 0 | 0 | 0 | 0 | 0 | 1 | 0 | 0 | 0 | 0 | 0 | 0 | | | | | | |
| 6055 | 07185 | 06054 | 2 | | 0 | 0 | 0 | 0 | 0 | 0 | 0 | 0 | 0 | 0 | 0 | 0 | 0 | 0 | 0 | 0 | 0 | 0 | 1 | 1 | 0 | 0 | 0 | 0 | 0 | L20860 | 96.8 | 94 | 1 | 639 | 2793 |
| 6056 | 07186 | 06055 | 2 | | 0 | 0 | 0 | 0 | 0 | 0 | 0 | 0 | 0 | 0 | 0 | 0 | 0 | 0 | 0 | 0 | 0 | 0 | 2 | 0 | 0 | 0 | 0 | 0 | 0 | | | | | | |
| 6057 | 07187 | 06056 | 2 | | 0 | 0 | 0 | 0 | 0 | 0 | 0 | 0 | 0 | 0 | 0 | 0 | 0 | 0 | 0 | 0 | 0 | 0 | 2 | 0 | 0 | 0 | 0 | 0 | 0 | | | | | | |
| 6058 | 07188 | 06057 | 1 | | 0 | 0 | 0 | 0 | 0 | 0 | 0 | 0 | 0 | 0 | 0 | 0 | 0 | 0 | 0 | 0 | 0 | 0 | 1 | 0 | 0 | 0 | 0 | 0 | 0 | | | | | | |
| 6059 | 07192 | 06058 | 1 | | 0 | 0 | 0 | 0 | 0 | 0 | 0 | 0 | 0 | 0 | 0 | 0 | 0 | 0 | 0 | 0 | 0 | 0 | 1 | 0 | 0 | 0 | 0 | 0 | 0 | | | | | | |
| 6060 | 07193 | 06059 | 1 | | 0 | 0 | 0 | 0 | 0 | 0 | 0 | 0 | 0 | 0 | 0 | 0 | 0 | 0 | 0 | 0 | 0 | 0 | 1 | 0 | 0 | 0 | 0 | 0 | 0 | | | | | | |
| 6061 | 07194 | 06060 | 1 | | 0 | 0 | 0 | 0 | 0 | 0 | 0 | 0 | 0 | 0 | 0 | 0 | 0 | 0 | 0 | 0 | 0 | 0 | 1 | 0 | 0 | 0 | 0 | 0 | 0 | | | | | | |
| 6062 | 07195 | 06061 | 1 | | 0 | 0 | 0 | 0 | 0 | 0 | 0 | 0 | 0 | 0 | 0 | 0 | 0 | 0 | 0 | 0 | 0 | 0 | 1 | 0 | 0 | 0 | 0 | 0 | 0 | | | | | | |
| 6063 | 07196 | 06062 | 1 | | 0 | 0 | 0 | 0 | 0 | 0 | 0 | 0 | 0 | 0 | 0 | 0 | 0 | 0 | 0 | 0 | 0 | 0 | 1 | 0 | 0 | 0 | 0 | 0 | 0 | | | | | | |
| 6064 | 07199 | 06063 | 1 | | 0 | 0 | 0 | 0 | 0 | 0 | 0 | 0 | 0 | 0 | 0 | 0 | 0 | 0 | 0 | 0 | 0 | 0 | 1 | 0 | 0 | 0 | 0 | 0 | 0 | | | | | | |
| 6065 | 07200 | 06064 | 1 | | 0 | 0 | 0 | 0 | 0 | 0 | 0 | 0 | 0 | 0 | 0 | 0 | 0 | 0 | 0 | 0 | 0 | 0 | 1 | 0 | 0 | 0 | 0 | 0 | 0 | | | | | | |
| 6066 | 07201 | 06065 | 1 | | 0 | 0 | 0 | 0 | 0 | 0 | 0 | 0 | 0 | 0 | 0 | 0 | 0 | 0 | 0 | 0 | 0 | 0 | 1 | 0 | 0 | 0 | 0 | 0 | 0 | | | | | | |
| 6067 | 07202 | 06066 | 1 | | 0 | 0 | 0 | 0 | 0 | 0 | 0 | 0 | 0 | 0 | 0 | 0 | 0 | 0 | 0 | 0 | 0 | 0 | 1 | 0 | 0 | 0 | 0 | 0 | 0 | | | | | | |
| 6068 | 07204 | 06067 | 1 | | 0 | 0 | 0 | 0 | 0 | 0 | 0 | 0 | 0 | 0 | 0 | 0 | 0 | 0 | 0 | 0 | 0 | 0 | 1 | 0 | 0 | 0 | 0 | 0 | 0 | | | | | | |
| 6069 | 07206 | 06068 | 1 | | 0 | 0 | 0 | 0 | 0 | 0 | 0 | 0 | 0 | 0 | 0 | 0 | 0 | 0 | 0 | 0 | 0 | 0 | 1 | 0 | 0 | 0 | 0 | 0 | 0 | | | | | | |

Table 169

| | A | B | C | E | G | I | K | M | O | Q | S | U | W | Y | AA | AC | AE | AG | AI | AK | AM | AO | AQ | AS | AU | AW | AY | BA | BC | BE | BF | BG | BH | BI | BK |
|---|---|---|---|---|---|---|---|---|---|---|---|---|---|---|---|---|---|---|---|---|---|---|---|---|---|---|---|---|---|---|---|---|---|---|---|
| 6070 | 07209 | 06069 | 1 | | 0 | 0 | 0 | 0 | 0 | 0 | 0 | 0 | 0 | 0 | 0 | 0 | 0 | 0 | 0 | 0 | 0 | 0 | 1 | 0 | 0 | 0 | 0 | 0 | 0 | | | | | | |
| 6071 | 07210 | 06070 | 2 | | 0 | 0 | 0 | 0 | 0 | 0 | 0 | 0 | 0 | 0 | 0 | 0 | 0 | 0 | 0 | 0 | 0 | 0 | 2 | 0 | 0 | 0 | 0 | 0 | 0 | | | | | | |
| 6072 | 07211 | 06071 | 1 | | 0 | 0 | 0 | 0 | 0 | 0 | 0 | 0 | 0 | 0 | 0 | 0 | 0 | 0 | 0 | 0 | 0 | 0 | 1 | 0 | 0 | 0 | 0 | 0 | 0 | L19713 | 94.2 | 171 | 1 | 2239 | 2660 |
| 6073 | 07212 | 06072 | 1 | | 0 | 0 | 0 | 0 | 0 | 0 | 0 | 0 | 0 | 0 | 0 | 0 | 0 | 0 | 0 | 0 | 0 | 0 | 1 | 0 | 0 | 0 | 0 | 0 | 0 | | | | | | |
| 6074 | 07213 | 06073 | 1 | | 0 | 0 | 0 | 0 | 0 | 0 | 0 | 0 | 0 | 0 | 0 | 0 | 0 | 0 | 0 | 0 | 0 | 0 | 1 | 0 | 0 | 0 | 0 | 0 | 0 | | | | | | |
| 6075 | 07214 | 06074 | 1 | | 0 | 0 | 0 | 0 | 0 | 0 | 0 | 0 | 0 | 0 | 0 | 0 | 0 | 0 | 0 | 0 | 0 | 0 | 1 | 0 | 0 | 0 | 0 | 0 | 0 | | | | | | |
| 6076 | 07215 | 06075 | 1 | | 0 | 0 | 0 | 0 | 0 | 0 | 0 | 0 | 0 | 0 | 0 | 0 | 0 | 0 | 0 | 0 | 0 | 0 | 1 | 0 | 0 | 0 | 0 | 0 | 0 | | | | | | |
| 6077 | 07216 | 06076 | 2 | | 0 | 0 | 0 | 0 | 0 | 0 | 0 | 0 | 0 | 0 | 0 | 0 | 0 | 0 | 0 | 0 | 0 | 0 | 2 | 0 | 0 | 0 | 0 | 0 | 0 | | | | | | |
| 6078 | 07217 | 06077 | 1 | | 0 | 0 | 0 | 0 | 0 | 0 | 0 | 0 | 0 | 0 | 0 | 0 | 0 | 0 | 0 | 0 | 0 | 0 | 1 | 0 | 0 | 0 | 0 | 0 | 0 | | | | | | |
| 6079 | 07218 | 06078 | 2 | | 0 | 0 | 0 | 0 | 0 | 0 | 0 | 0 | 0 | 0 | 0 | 0 | 0 | 0 | 0 | 0 | 0 | 0 | 2 | 0 | 0 | 0 | 0 | 0 | 0 | | | | | | |
| 6080 | 07219 | 06079 | 2 | | 0 | 0 | 0 | 0 | 0 | 0 | 0 | 0 | 0 | 0 | 0 | 0 | 0 | 0 | 0 | 0 | 0 | 0 | 2 | 0 | 0 | 0 | 0 | 0 | 0 | | | | | | |
| 6081 | 07220 | 06080 | 1 | | 0 | 0 | 0 | 0 | 0 | 0 | 0 | 0 | 0 | 0 | 0 | 0 | 0 | 0 | 0 | 0 | 0 | 0 | 1 | 0 | 0 | 0 | 0 | 0 | 0 | | | | | | |
| 6082 | 07221 | 06081 | 1 | | 0 | 0 | 0 | 0 | 0 | 0 | 0 | 0 | 0 | 0 | 0 | 0 | 0 | 0 | 0 | 0 | 0 | 0 | 1 | 0 | 0 | 0 | 0 | 0 | 0 | | | | | | |
| 6083 | 07222 | 06082 | 1 | | 0 | 0 | 0 | 0 | 0 | 0 | 0 | 0 | 0 | 0 | 0 | 0 | 0 | 0 | 0 | 0 | 0 | 0 | 1 | 0 | 0 | 0 | 0 | 0 | 0 | X68148 | 92.3 | 104 | 1 | 1502 | 3031 |
| 6084 | 07223 | 06083 | 1 | | 0 | 0 | 0 | 0 | 0 | 0 | 0 | 0 | 0 | 0 | 0 | 0 | 0 | 0 | 0 | 0 | 0 | 0 | 1 | 0 | 0 | 0 | 0 | 0 | 0 | X65024 | 93.6 | 172 | 1 | 3234 | 3455 |
| 6085 | 07224 | 06084 | 1 | | 0 | 0 | 0 | 0 | 0 | 0 | 0 | 0 | 0 | 0 | 0 | 0 | 0 | 0 | 0 | 0 | 0 | 0 | 1 | 0 | 0 | 0 | 0 | 0 | 0 | | | | | | |
| 6086 | 07225 | 06085 | 1 | | 0 | 0 | 0 | 0 | 0 | 0 | 0 | 0 | 0 | 0 | 0 | 0 | 0 | 0 | 0 | 0 | 0 | 0 | 1 | 0 | 0 | 0 | 0 | 0 | 0 | | | | | | |
| 6087 | 07226 | 06086 | 1 | | 0 | 0 | 0 | 0 | 0 | 0 | 0 | 0 | 0 | 0 | 0 | 0 | 0 | 0 | 0 | 0 | 0 | 0 | 1 | 0 | 0 | 0 | 0 | 0 | 0 | | | | | | |
| 6088 | 07227 | 06087 | 1 | | 0 | 0 | 0 | 0 | 0 | 0 | 0 | 0 | 0 | 0 | 0 | 0 | 0 | 0 | 0 | 0 | 0 | 0 | 1 | 0 | 0 | 0 | 0 | 0 | 0 | | | | | | |
| 6089 | 07228 | 06088 | 1 | | 0 | 0 | 0 | 0 | 0 | 0 | 0 | 0 | 0 | 0 | 0 | 0 | 0 | 0 | 0 | 0 | 0 | 0 | 1 | 0 | 0 | 0 | 0 | 0 | 0 | | | | | | |
| 6090 | 07229 | 06089 | 1 | | 0 | 0 | 0 | 0 | 0 | 0 | 0 | 0 | 0 | 0 | 0 | 0 | 0 | 0 | 0 | 0 | 0 | 0 | 1 | 0 | 0 | 0 | 0 | 0 | 0 | | | | | | |
| 6091 | 07230 | 06090 | 1 | | 0 | 0 | 0 | 0 | 0 | 0 | 0 | 0 | 0 | 0 | 0 | 0 | 0 | 0 | 0 | 0 | 0 | 0 | 1 | 0 | 0 | 0 | 0 | 0 | 0 | | | | | | |
| 6092 | 07231 | 06091 | 1 | | 0 | 0 | 0 | 0 | 0 | 0 | 0 | 0 | 0 | 0 | 0 | 0 | 0 | 0 | 0 | 0 | 0 | 0 | 1 | 0 | 0 | 0 | 0 | 0 | 0 | D21852 | 99.3 | 134 | 1 | 4139 | 4272 |
| 6093 | 07232 | 06092 | 1 | | 0 | 0 | 0 | 0 | 0 | 0 | 0 | 0 | 0 | 0 | 0 | 0 | 0 | 0 | 0 | 0 | 0 | 0 | 1 | 0 | 0 | 0 | 0 | 0 | 0 | | | | | | |
| 6094 | 07235 | 06093 | 1 | | 0 | 0 | 0 | 0 | 0 | 0 | 0 | 0 | 0 | 0 | 0 | 0 | 0 | 0 | 0 | 0 | 0 | 0 | 1 | 0 | 0 | 0 | 0 | 0 | 0 | | | | | | |
| 6095 | 07238 | 06094 | 1 | | 0 | 0 | 0 | 0 | 0 | 0 | 0 | 0 | 0 | 0 | 0 | 0 | 0 | 0 | 0 | 0 | 0 | 0 | 1 | 0 | 0 | 0 | 0 | 0 | 0 | | | | | | |
| 6096 | 07241 | 06095 | 1 | | 0 | 0 | 0 | 0 | 0 | 0 | 0 | 0 | 0 | 0 | 0 | 0 | 0 | 0 | 0 | 0 | 0 | 0 | 1 | 0 | 0 | 0 | 0 | 0 | 0 | | | | | | |
| 6097 | 07243 | 06096 | 1 | | 0 | 0 | 0 | 0 | 0 | 0 | 0 | 0 | 0 | 0 | 0 | 0 | 0 | 0 | 0 | 0 | 0 | 0 | 1 | 0 | 0 | 0 | 0 | 0 | 0 | | | | | | |
| 6098 | 07244 | 06097 | 1 | | 0 | 0 | 0 | 0 | 0 | 0 | 0 | 0 | 0 | 0 | 0 | 0 | 0 | 0 | 0 | 0 | 0 | 0 | 1 | 0 | 0 | 0 | 0 | 0 | 0 | J05032 | 98.8 | 81 | 1 | 1573 | 2271 |
| 6099 | 07245 | 06098 | 1 | | 0 | 0 | 0 | 0 | 0 | 0 | 0 | 0 | 0 | 0 | 0 | 0 | 0 | 0 | 0 | 0 | 0 | 0 | 1 | 0 | 0 | 0 | 0 | 0 | 0 | | | | | | |
| 6100 | 07246 | 06099 | 1 | | 0 | 0 | 0 | 0 | 0 | 0 | 0 | 0 | 0 | 0 | 0 | 0 | 0 | 1 | 0 | 0 | 0 | 0 | 0 | 0 | 0 | 0 | 0 | 0 | 0 | | | | | | |
| 6101 | 07249 | 06100 | 1 | | 0 | 0 | 0 | 0 | 0 | 0 | 0 | 0 | 0 | 0 | 0 | 0 | 0 | 1 | 0 | 0 | 0 | 0 | 0 | 0 | 0 | 0 | 0 | 0 | 0 | | | | | | |
| 6102 | 07250 | 06101 | 1 | | 0 | 0 | 0 | 0 | 0 | 0 | 0 | 0 | 0 | 0 | 0 | 0 | 0 | 1 | 0 | 0 | 0 | 0 | 0 | 0 | 0 | 0 | 0 | 0 | 0 | | | | | | |
| 6103 | 07251 | 06102 | 1 | | 0 | 0 | 0 | 0 | 0 | 0 | 0 | 0 | 0 | 0 | 0 | 0 | 0 | 1 | 0 | 0 | 0 | 0 | 0 | 0 | 0 | 0 | 0 | 0 | 0 | | | | | | |
| 6104 | 07253 | 06103 | 1 | | 0 | 0 | 0 | 0 | 0 | 0 | 0 | 0 | 0 | 0 | 0 | 0 | 0 | 1 | 0 | 0 | 0 | 0 | 0 | 0 | 0 | 0 | 0 | 0 | 0 | | | | | | |
| 6105 | 07257 | 06104 | 1 | | 0 | 0 | 0 | 0 | 0 | 0 | 0 | 0 | 0 | 0 | 0 | 0 | 0 | 1 | 0 | 0 | 0 | 0 | 0 | 0 | 0 | 0 | 0 | 0 | 0 | | | | | | |

Table 170

| | A | B | C | E | G | I | K | M | O | Q | S | U | W | Y | AA | AC | AE | AG | AI | AK | AM | AO | AQ | AS | AU | AW | AY | BA | BC | BE | BF | BG | BH | BI | BK |
|---|---|---|---|---|---|---|---|---|---|---|---|---|---|---|---|---|---|---|---|---|---|---|---|---|---|---|---|---|---|---|---|---|---|---|---|
| 6106 | 07259 | 06105 | 1 | | 0 | 0 | 0 | 0 | 0 | 0 | 0 | 0 | 0 | 0 | 0 | 0 | 0 | 1 | 0 | 0 | 0 | 0 | 0 | 0 | 0 | 0 | 0 | 0 | 0 | | | | | | |
| 6107 | 07261 | 06106 | 2 | | 0 | 0 | 0 | 0 | 0 | 0 | 0 | 0 | 0 | 0 | 0 | 0 | 0 | 1 | 0 | 0 | 0 | 0 | 0 | 0 | 0 | 0 | 0 | 1 | 0 | | | | | | |
| 6108 | 07263 | 06107 | 1 | | 0 | 0 | 0 | 0 | 0 | 0 | 0 | 0 | 0 | 0 | 0 | 0 | 0 | 1 | 0 | 0 | 0 | 0 | 0 | 0 | 0 | 0 | 0 | 0 | 0 | | | | | | |
| 6109 | 07264 | 06108 | 1 | | 0 | 0 | 0 | 0 | 0 | 0 | 0 | 0 | 0 | 0 | 0 | 0 | 0 | 1 | 0 | 0 | 0 | 0 | 0 | 0 | 0 | 0 | 0 | 0 | 0 | | | | | | |
| 6110 | 07265 | 06109 | 1 | | 0 | 0 | 0 | 0 | 0 | 0 | 0 | 0 | 0 | 0 | 0 | 0 | 0 | 1 | 0 | 0 | 0 | 0 | 0 | 0 | 0 | 0 | 0 | 0 | 0 | | | | | | |
| 6111 | 07266 | 06110 | 1 | | 0 | 0 | 0 | 0 | 0 | 0 | 0 | 0 | 0 | 0 | 0 | 0 | 0 | 1 | 0 | 0 | 0 | 0 | 0 | 0 | 0 | 0 | 0 | 0 | 0 | | | | | | |
| 6112 | 07267 | 06111 | 1 | | 0 | 0 | 0 | 0 | 0 | 0 | 0 | 0 | 0 | 0 | 0 | 0 | 0 | 1 | 0 | 0 | 0 | 0 | 0 | 0 | 0 | 0 | 0 | 0 | 0 | | | | | | |
| 6113 | 07268 | 06112 | 1 | | 0 | 0 | 0 | 0 | 0 | 0 | 0 | 0 | 0 | 0 | 0 | 0 | 0 | 1 | 0 | 0 | 0 | 0 | 0 | 0 | 0 | 0 | 0 | 0 | 0 | | | | | | |
| 6114 | 07269 | 06113 | 1 | | 0 | 0 | 0 | 0 | 0 | 0 | 0 | 0 | 0 | 0 | 0 | 0 | 0 | 1 | 0 | 0 | 0 | 0 | 0 | 0 | 0 | 0 | 0 | 0 | 0 | | | | | | |
| 6115 | 07270 | 06114 | 1 | | 0 | 0 | 0 | 0 | 0 | 0 | 0 | 0 | 0 | 0 | 0 | 0 | 0 | 1 | 0 | 0 | 0 | 0 | 0 | 0 | 0 | 0 | 0 | 0 | 0 | | | | | | |
| 6116 | 07272 | 06115 | 1 | | 0 | 0 | 0 | 0 | 0 | 0 | 0 | 0 | 0 | 0 | 0 | 0 | 0 | 1 | 0 | 0 | 0 | 0 | 0 | 0 | 0 | 0 | 0 | 0 | 0 | | | | | | |
| 6117 | 07274 | 06116 | 1 | | 0 | 0 | 0 | 0 | 0 | 0 | 0 | 0 | 0 | 0 | 0 | 0 | 0 | 1 | 0 | 0 | 0 | 0 | 0 | 0 | 0 | 0 | 0 | 0 | 0 | | | | | | |
| 6118 | 07275 | 06117 | 1 | | 0 | 0 | 0 | 0 | 0 | 0 | 0 | 0 | 0 | 0 | 0 | 0 | 0 | 1 | 0 | 0 | 0 | 0 | 0 | 0 | 0 | 0 | 0 | 0 | 0 | | | | | | |
| 6119 | 07276 | 06118 | 1 | | 0 | 0 | 0 | 0 | 0 | 0 | 0 | 0 | 0 | 0 | 0 | 0 | 0 | 1 | 0 | 0 | 0 | 0 | 0 | 0 | 0 | 0 | 0 | 0 | 0 | | | | | | |
| 6120 | 07277 | 06119 | 3 | | 0 | 0 | 0 | 0 | 0 | 0 | 0 | 0 | 0 | 0 | 0 | 0 | 0 | 1 | 0 | 0 | 0 | 0 | 0 | 1 | 0 | 0 | 0 | 0 | 1 | | | | | | |
| 6121 | 07278 | 06120 | 4 | | 0 | 0 | 0 | 0 | 0 | 0 | 0 | 0 | 0 | 0 | 0 | 0 | 0 | 2 | 0 | 0 | 0 | 0 | 0 | 2 | 0 | 0 | 0 | 0 | 0 | | | | | | |
| 6122 | 07279 | 06121 | 1 | | 0 | 0 | 0 | 0 | 0 | 0 | 0 | 0 | 0 | 0 | 0 | 0 | 0 | 1 | 0 | 0 | 0 | 0 | 0 | 0 | 0 | 0 | 0 | 0 | 0 | | | | | | |
| 6123 | 07280 | 06122 | 1 | | 0 | 0 | 0 | 0 | 0 | 0 | 0 | 0 | 0 | 0 | 0 | 0 | 0 | 1 | 0 | 0 | 0 | 0 | 0 | 0 | 0 | 0 | 0 | 0 | 0 | | | | | | |
| 6124 | 07281 | 06123 | 2 | | 0 | 0 | 0 | 0 | 0 | 0 | 0 | 0 | 0 | 0 | 0 | 0 | 0 | 1 | 0 | 0 | 0 | 0 | 0 | 0 | 0 | 0 | 0 | 1 | 0 | | | | | | |
| 6125 | 07282 | 06124 | 1 | | 0 | 0 | 0 | 0 | 0 | 0 | 0 | 0 | 0 | 0 | 0 | 0 | 0 | 1 | 0 | 0 | 0 | 0 | 0 | 0 | 0 | 0 | 0 | 0 | 0 | | | | | | |
| 6126 | 07283 | 06125 | 1 | | 0 | 0 | 0 | 0 | 0 | 0 | 0 | 0 | 0 | 0 | 0 | 0 | 0 | 1 | 0 | 0 | 0 | 0 | 0 | 0 | 0 | 0 | 0 | 0 | 0 | | | | | | |
| 6127 | 07284 | 06126 | 1 | | 0 | 0 | 0 | 0 | 0 | 0 | 0 | 0 | 0 | 0 | 0 | 0 | 0 | 1 | 0 | 0 | 0 | 0 | 0 | 0 | 0 | 0 | 0 | 0 | 0 | | | | | | |
| 6128 | 07285 | 06127 | 1 | | 0 | 0 | 0 | 0 | 0 | 0 | 0 | 0 | 0 | 0 | 0 | 0 | 0 | 1 | 0 | 0 | 0 | 0 | 0 | 0 | 0 | 0 | 0 | 0 | 0 | | | | | | |
| 6129 | 07286 | 06128 | 1 | | 0 | 0 | 0 | 0 | 0 | 0 | 0 | 0 | 0 | 0 | 0 | 0 | 0 | 1 | 0 | 0 | 0 | 0 | 0 | 0 | 0 | 0 | 0 | 0 | 0 | | | | | | |
| 6130 | 07287 | 06129 | 1 | | 0 | 0 | 0 | 0 | 0 | 0 | 0 | 0 | 0 | 0 | 0 | 0 | 0 | 0 | 0 | 0 | 0 | 0 | 0 | 1 | 0 | 0 | 0 | 0 | 0 | | | | | | |
| 6131 | 07288 | 06130 | 1 | | 0 | 0 | 0 | 0 | 0 | 0 | 0 | 0 | 0 | 0 | 0 | 0 | 0 | 0 | 0 | 0 | 0 | 0 | 0 | 1 | 0 | 0 | 0 | 0 | 0 | | | | | | |
| 6132 | 07289 | 06131 | 2 | | 0 | 0 | 0 | 0 | 0 | 0 | 0 | 0 | 0 | 0 | 0 | 0 | 0 | 0 | 0 | 0 | 0 | 0 | 0 | 2 | 0 | 0 | 0 | 0 | 0 | | | | | | |
| 6133 | 07290 | 06132 | 1 | | 0 | 0 | 0 | 0 | 0 | 0 | 0 | 0 | 0 | 0 | 0 | 0 | 0 | 0 | 0 | 0 | 0 | 0 | 0 | 1 | 0 | 0 | 0 | 0 | 0 | | | | | | |
| 6134 | 07291 | 06133 | 1 | | 0 | 0 | 0 | 0 | 0 | 0 | 0 | 0 | 0 | 0 | 0 | 0 | 0 | 0 | 0 | 0 | 0 | 0 | 0 | 1 | 0 | 0 | 0 | 0 | 0 | | | | | | |
| 6135 | 07292 | 06134 | 1 | | 0 | 0 | 0 | 0 | 0 | 0 | 0 | 0 | 0 | 0 | 0 | 0 | 0 | 0 | 0 | 0 | 0 | 0 | 0 | 1 | 0 | 0 | 0 | 0 | 0 | | | | | | |
| 6136 | 07293 | 06135 | 1 | | 0 | 0 | 0 | 0 | 0 | 0 | 0 | 0 | 0 | 0 | 0 | 0 | 0 | 0 | 0 | 0 | 0 | 0 | 0 | 1 | 0 | 0 | 0 | 0 | 0 | | | | | | |
| 6137 | 07294 | 06136 | 2 | | 0 | 0 | 0 | 0 | 0 | 0 | 0 | 0 | 0 | 0 | 0 | 0 | 0 | 0 | 0 | 0 | 0 | 0 | 0 | 1 | 0 | 0 | 0 | 1 | 0 | | | | | | |
| 6138 | 07295 | 06137 | 2 | | 0 | 0 | 0 | 0 | 0 | 0 | 0 | 0 | 0 | 0 | 0 | 0 | 0 | 0 | 0 | 0 | 0 | 0 | 0 | 2 | 0 | 0 | 0 | 0 | 0 | | | | | | |
| 6139 | 07296 | 06138 | 1 | | 0 | 0 | 0 | 0 | 0 | 0 | 0 | 0 | 0 | 0 | 0 | 0 | 0 | 0 | 0 | 0 | 0 | 0 | 0 | 1 | 0 | 0 | 0 | 0 | 0 | | | | | | |
| 6140 | 07297 | 06139 | 1 | | 0 | 0 | 0 | 0 | 0 | 0 | 0 | 0 | 0 | 0 | 0 | 0 | 0 | 0 | 0 | 0 | 0 | 0 | 0 | 1 | 0 | 0 | 0 | 0 | 0 | | | | | | |
| 6141 | 07298 | 06140 | 1 | | 0 | 0 | 0 | 0 | 0 | 0 | 0 | 0 | 0 | 0 | 0 | 0 | 0 | 0 | 0 | 0 | 0 | 0 | 0 | 1 | 0 | 0 | 0 | 0 | 0 | | | | | | |

Table 171

| | A | B | C | E | G | I | K | M | O | Q | S | U | W | Y | AA | AC | AE | AG | AI | AK | AM | AO | AQ | AS | AU | AW | AY | BA | BC | BE | BF | BG | BH | BI | BK |
|---|---|---|---|---|---|---|---|---|---|---|---|---|---|---|---|---|---|---|---|---|---|---|---|---|---|---|---|---|---|---|---|---|---|---|---|
| 6142 | 07299 | 06141 | 1 | | 0 | 0 | 0 | 0 | 0 | 0 | 0 | 0 | 0 | 0 | 0 | 0 | 0 | 0 | 0 | 0 | 0 | 0 | 0 | 1 | 0 | 0 | 0 | 0 | 0 | | | | | | |
| 6143 | 07300 | 06142 | 1 | | 0 | 0 | 0 | 0 | 0 | 0 | 0 | 0 | 0 | 0 | 0 | 0 | 0 | 0 | 0 | 0 | 0 | 0 | 0 | 1 | 0 | 0 | 0 | 0 | 0 | | | | | | |
| 6144 | 07301 | 06143 | 1 | | 0 | 0 | 0 | 0 | 0 | 0 | 0 | 0 | 0 | 0 | 0 | 0 | 0 | 0 | 0 | 0 | 0 | 0 | 0 | 1 | 0 | 0 | 0 | 0 | 0 | | | | | | |
| 6145 | 07302 | 06144 | 2 | | 0 | 0 | 0 | 0 | 0 | 0 | 0 | 0 | 0 | 0 | 0 | 0 | 0 | 0 | 0 | 0 | 0 | 0 | 0 | 2 | 0 | 0 | 0 | 0 | 0 | | | | | | |
| 6146 | 07303 | 06145 | 1 | | 0 | 0 | 0 | 0 | 0 | 0 | 0 | 0 | 0 | 0 | 0 | 0 | 0 | 0 | 0 | 0 | 0 | 0 | 0 | 1 | 0 | 0 | 0 | 0 | 0 | | | | | | |
| 6147 | 07304 | 06146 | 1 | | 0 | 0 | 0 | 0 | 0 | 0 | 0 | 0 | 0 | 0 | 0 | 0 | 0 | 0 | 0 | 0 | 0 | 0 | 0 | 1 | 0 | 0 | 0 | 0 | 0 | | | | | | |
| 6148 | 07305 | 06147 | 2 | | 0 | 0 | 0 | 0 | 0 | 0 | 0 | 0 | 0 | 0 | 0 | 0 | 0 | 0 | 0 | 0 | 0 | 0 | 0 | 1 | 0 | 1 | 0 | 0 | 0 | | | | | | |
| 6149 | 07306 | 06148 | 1 | | 0 | 0 | 0 | 0 | 0 | 0 | 0 | 0 | 0 | 0 | 0 | 0 | 0 | 0 | 0 | 0 | 0 | 0 | 0 | 1 | 0 | 0 | 0 | 0 | 0 | | | | | | |
| 6150 | 07307 | 06149 | 1 | | 0 | 0 | 0 | 0 | 0 | 0 | 0 | 0 | 0 | 0 | 0 | 0 | 0 | 0 | 0 | 0 | 0 | 0 | 0 | 1 | 0 | 0 | 0 | 0 | 0 | | | | | | |
| 6151 | 07308 | 06150 | 1 | | 0 | 0 | 0 | 0 | 0 | 0 | 0 | 0 | 0 | 0 | 0 | 0 | 0 | 0 | 0 | 0 | 0 | 0 | 0 | 1 | 0 | 0 | 0 | 0 | 0 | | | | | | |
| 6152 | 07309 | 06151 | 5 | | 0 | 0 | 0 | 0 | 0 | 0 | 0 | 0 | 0 | 0 | 0 | 0 | 0 | 0 | 0 | 0 | 0 | 0 | 0 | 5 | 0 | 0 | 0 | 0 | 0 | | | | | | |
| 6153 | 07311 | 06152 | 1 | | 0 | 0 | 0 | 0 | 0 | 0 | 0 | 0 | 0 | 0 | 0 | 0 | 0 | 0 | 0 | 0 | 0 | 0 | 0 | 1 | 0 | 0 | 0 | 0 | 0 | | | | | | |
| 6154 | 07312 | 06153 | 1 | | 0 | 0 | 0 | 0 | 0 | 0 | 0 | 0 | 0 | 0 | 0 | 0 | 0 | 0 | 0 | 0 | 0 | 0 | 0 | 1 | 0 | 0 | 0 | 0 | 0 | | | | | | |
| 6155 | 07313 | 06154 | 1 | | 0 | 0 | 0 | 0 | 0 | 0 | 0 | 0 | 0 | 0 | 0 | 0 | 0 | 0 | 0 | 0 | 0 | 0 | 0 | 1 | 0 | 0 | 0 | 0 | 0 | | | | | | |
| 6156 | 07315 | 06155 | 1 | | 0 | 0 | 0 | 0 | 0 | 0 | 0 | 0 | 0 | 0 | 0 | 0 | 0 | 0 | 0 | 0 | 0 | 0 | 0 | 1 | 0 | 0 | 0 | 0 | 0 | | | | | | |
| 6157 | 07316 | 06156 | 1 | | 0 | 0 | 0 | 0 | 0 | 0 | 0 | 0 | 0 | 0 | 0 | 0 | 0 | 0 | 0 | 0 | 0 | 0 | 0 | 1 | 0 | 0 | 0 | 0 | 0 | | | | | | |
| 6158 | 07317 | 06157 | 2 | | 0 | 0 | 0 | 0 | 0 | 0 | 0 | 0 | 0 | 0 | 0 | 0 | 0 | 0 | 0 | 0 | 0 | 0 | 0 | 2 | 0 | 0 | 0 | 0 | 0 | | | | | | |
| 6159 | 07318 | 06158 | 1 | | 0 | 0 | 0 | 0 | 0 | 0 | 0 | 0 | 0 | 0 | 0 | 0 | 0 | 0 | 0 | 0 | 0 | 0 | 0 | 1 | 0 | 0 | 0 | 0 | 0 | | | | | | |
| 6160 | 07319 | 06159 | 1 | | 0 | 0 | 0 | 0 | 0 | 0 | 0 | 0 | 0 | 0 | 0 | 0 | 0 | 0 | 0 | 0 | 0 | 0 | 0 | 1 | 0 | 0 | 0 | 0 | 0 | | | | | | |
| 6161 | 07320 | 06160 | 2 | | 0 | 0 | 0 | 0 | 0 | 0 | 0 | 0 | 0 | 0 | 0 | 0 | 0 | 0 | 0 | 0 | 0 | 0 | 0 | 2 | 0 | 0 | 0 | 0 | 0 | | | | | | |
| 6162 | 07321 | 06161 | 1 | | 0 | 0 | 0 | 0 | 0 | 0 | 0 | 0 | 0 | 0 | 0 | 0 | 0 | 0 | 0 | 0 | 0 | 0 | 0 | 1 | 0 | 0 | 0 | 0 | 0 | | | | | | |
| 6163 | 07322 | 06162 | 1 | | 0 | 0 | 0 | 0 | 0 | 0 | 0 | 0 | 0 | 0 | 0 | 0 | 0 | 0 | 0 | 0 | 0 | 0 | 0 | 1 | 0 | 0 | 0 | 0 | 0 | | | | | | |
| 6164 | 07323 | 06163 | 1 | | 0 | 0 | 0 | 0 | 0 | 0 | 0 | 0 | 0 | 0 | 0 | 0 | 0 | 0 | 0 | 0 | 0 | 0 | 0 | 1 | 0 | 0 | 0 | 0 | 0 | | | | | | |
| 6165 | 07324 | 06164 | 1 | | 0 | 0 | 0 | 0 | 0 | 0 | 0 | 0 | 0 | 0 | 0 | 0 | 0 | 0 | 0 | 0 | 0 | 0 | 0 | 1 | 0 | 0 | 0 | 0 | 0 | | | | | | |
| 6166 | 07325 | 06165 | 1 | | 0 | 0 | 0 | 0 | 0 | 0 | 0 | 0 | 0 | 0 | 0 | 0 | 0 | 0 | 0 | 0 | 0 | 0 | 0 | 1 | 0 | 0 | 0 | 0 | 0 | | | | | | |
| 6167 | 07326 | 06166 | 1 | | 0 | 0 | 0 | 0 | 0 | 0 | 0 | 0 | 0 | 0 | 0 | 0 | 0 | 0 | 0 | 0 | 0 | 0 | 0 | 1 | 0 | 0 | 0 | 0 | 0 | | | | | | |
| 6168 | 07327 | 06167 | 1 | | 0 | 0 | 0 | 0 | 0 | 0 | 0 | 0 | 0 | 0 | 0 | 0 | 0 | 0 | 0 | 0 | 0 | 0 | 0 | 1 | 0 | 0 | 0 | 0 | 0 | | | | | | |
| 6169 | 07328 | 06168 | 2 | | 0 | 0 | 0 | 0 | 0 | 0 | 0 | 0 | 0 | 0 | 0 | 0 | 0 | 0 | 0 | 0 | 0 | 0 | 0 | 2 | 0 | 0 | 0 | 0 | 0 | | | | | | |
| 6170 | 07329 | 06169 | 2 | | 0 | 0 | 0 | 0 | 0 | 0 | 0 | 0 | 0 | 0 | 0 | 0 | 0 | 0 | 0 | 0 | 0 | 0 | 0 | 2 | 0 | 0 | 0 | 0 | 0 | | | | | | |
| 6171 | 07330 | 06170 | 1 | | 0 | 0 | 0 | 0 | 0 | 0 | 0 | 0 | 0 | 0 | 0 | 0 | 0 | 0 | 0 | 0 | 0 | 0 | 0 | 1 | 0 | 0 | 0 | 0 | 0 | | | | | | |
| 6172 | 07331 | 06171 | 1 | | 0 | 0 | 0 | 0 | 0 | 0 | 0 | 0 | 0 | 0 | 0 | 0 | 0 | 0 | 0 | 0 | 0 | 0 | 0 | 1 | 0 | 0 | 0 | 0 | 0 | | | | | | |
| 6173 | 07332 | 06172 | 2 | | 0 | 0 | 0 | 0 | 0 | 0 | 0 | 0 | 0 | 0 | 0 | 0 | 0 | 0 | 0 | 0 | 0 | 0 | 0 | 2 | 0 | 0 | 0 | 0 | 0 | | | | | | |
| 6174 | 07333 | 06173 | 2 | | 0 | 0 | 0 | 0 | 0 | 0 | 0 | 0 | 0 | 0 | 0 | 0 | 0 | 0 | 0 | 0 | 0 | 0 | 0 | 2 | 0 | 0 | 0 | 0 | 0 | | | | | | |
| 6175 | 07334 | 06174 | 1 | | 0 | 0 | 0 | 0 | 0 | 0 | 0 | 0 | 0 | 0 | 0 | 0 | 0 | 0 | 0 | 0 | 0 | 0 | 0 | 1 | 0 | 0 | 0 | 0 | 0 | | | | | | |
| 6176 | 07335 | 06175 | 1 | | 0 | 0 | 0 | 0 | 0 | 0 | 0 | 0 | 0 | 0 | 0 | 0 | 0 | 0 | 0 | 0 | 0 | 0 | 0 | 1 | 0 | 0 | 0 | 0 | 0 | | | | | | |
| 6177 | 07336 | 06176 | 1 | | 0 | 0 | 0 | 0 | 0 | 0 | 0 | 0 | 0 | 0 | 0 | 0 | 0 | 0 | 0 | 0 | 0 | 0 | 0 | 1 | 0 | 0 | 0 | 0 | 0 | | | | | | |

Table 172

| | A | B | C | E | G | I | K | M | O | Q | S | U | W | Y | AA | AC | AE | AG | AI | AK | AM | AO | AQ | AS | AU | AW | AY | BA | BC | BE | BF | BG | BH | BI | BK |
|---|---|---|---|---|---|---|---|---|---|---|---|---|---|---|---|---|---|---|---|---|---|---|---|---|---|---|---|---|---|---|---|---|---|---|---|
| 6178 | 07337 | 06177 | 1 | | 0 | 0 | 0 | 0 | 0 | 0 | 0 | 0 | 0 | 0 | 0 | 0 | 0 | 0 | 0 | 0 | 0 | 0 | 0 | 1 | 0 | 0 | 0 | 0 | 0 | | | | | | |
| 6179 | 07338 | 06178 | 1 | | 0 | 0 | 0 | 0 | 0 | 0 | 0 | 0 | 0 | 0 | 0 | 0 | 0 | 0 | 0 | 0 | 0 | 0 | 0 | 1 | 0 | 0 | 0 | 0 | 0 | | | | | | |
| 6180 | 07339 | 06179 | 1 | | 0 | 0 | 0 | 0 | 0 | 0 | 0 | 0 | 0 | 0 | 0 | 0 | 0 | 0 | 0 | 0 | 0 | 0 | 0 | 1 | 0 | 0 | 0 | 0 | 0 | | | | | | |
| 6181 | 07340 | 06180 | 1 | | 0 | 0 | 0 | 0 | 0 | 0 | 0 | 0 | 0 | 0 | 0 | 0 | 0 | 0 | 0 | 0 | 0 | 0 | 0 | 1 | 0 | 0 | 0 | 0 | 0 | | | | | | |
| 6182 | 07341 | 06181 | 1 | | 0 | 0 | 0 | 0 | 0 | 0 | 0 | 0 | 0 | 0 | 0 | 0 | 0 | 0 | 0 | 0 | 0 | 0 | 0 | 1 | 0 | 0 | 0 | 0 | 0 | | | | | | |
| 6183 | 07342 | 06182 | 1 | | 0 | 0 | 0 | 0 | 0 | 0 | 0 | 0 | 0 | 0 | 0 | 0 | 0 | 0 | 0 | 0 | 0 | 0 | 0 | 1 | 0 | 0 | 0 | 0 | 0 | | | | | | |
| 6184 | 07343 | 06183 | 1 | | 0 | 0 | 0 | 0 | 0 | 0 | 0 | 0 | 0 | 0 | 0 | 0 | 0 | 0 | 0 | 0 | 0 | 0 | 0 | 1 | 0 | 0 | 0 | 0 | 0 | | | | | | |
| 6185 | 07344 | 06184 | 1 | | 0 | 0 | 0 | 0 | 0 | 0 | 0 | 0 | 0 | 0 | 0 | 0 | 0 | 0 | 0 | 0 | 0 | 0 | 0 | 1 | 0 | 0 | 0 | 0 | 0 | | | | | | |
| 6186 | 07345 | 06185 | 2 | | 0 | 0 | 0 | 0 | 0 | 0 | 0 | 0 | 0 | 0 | 0 | 0 | 0 | 0 | 0 | 0 | 0 | 0 | 0 | 1 | 0 | 0 | 1 | 0 | 0 | | | | | | |
| 6187 | 07346 | 06186 | 1 | | 0 | 0 | 0 | 0 | 0 | 0 | 0 | 0 | 0 | 0 | 0 | 0 | 0 | 0 | 0 | 0 | 0 | 0 | 0 | 1 | 0 | 0 | 0 | 0 | 0 | | | | | | |
| 6188 | 07348 | 06187 | 1 | | 0 | 0 | 0 | 0 | 0 | 0 | 0 | 0 | 0 | 0 | 0 | 0 | 0 | 0 | 0 | 0 | 0 | 0 | 0 | 1 | 0 | 0 | 0 | 0 | 0 | | | | | | |
| 6189 | 07349 | 06188 | 1 | | 0 | 0 | 0 | 0 | 0 | 0 | 0 | 0 | 0 | 0 | 0 | 0 | 0 | 0 | 0 | 0 | 0 | 0 | 0 | 1 | 0 | 0 | 0 | 0 | 0 | | | | | | |
| 6190 | 07350 | 06189 | 3 | | 0 | 0 | 0 | 0 | 0 | 0 | 0 | 0 | 0 | 0 | 0 | 0 | 0 | 0 | 0 | 0 | 0 | 0 | 0 | 2 | 0 | 0 | 0 | 1 | 0 | | | | | | |
| 6191 | 07351 | 06190 | 2 | | 0 | 0 | 0 | 0 | 0 | 0 | 0 | 0 | 0 | 0 | 0 | 0 | 0 | 0 | 0 | 0 | 0 | 0 | 0 | 2 | 0 | 0 | 0 | 0 | 0 | | | | | | |
| 6192 | 07352 | 06191 | 1 | | 0 | 0 | 0 | 0 | 0 | 0 | 0 | 0 | 0 | 0 | 0 | 0 | 0 | 0 | 0 | 0 | 0 | 0 | 0 | 1 | 0 | 0 | 0 | 0 | 0 | | | | | | |
| 6193 | 07353 | 06192 | 2 | | 0 | 0 | 0 | 0 | 0 | 0 | 0 | 0 | 0 | 0 | 0 | 0 | 0 | 0 | 0 | 0 | 0 | 0 | 0 | 1 | 1 | 0 | 0 | 0 | 0 | | | | | | |
| 6194 | 07354 | 06193 | 2 | | 0 | 0 | 0 | 0 | 0 | 0 | 0 | 0 | 0 | 0 | 0 | 0 | 0 | 0 | 0 | 0 | 0 | 0 | 0 | 1 | 0 | 0 | 0 | 1 | 0 | | | | | | |
| 6195 | 07355 | 06194 | 1 | | 0 | 0 | 0 | 0 | 0 | 0 | 0 | 0 | 0 | 0 | 0 | 0 | 0 | 0 | 0 | 0 | 0 | 0 | 0 | 1 | 0 | 0 | 0 | 0 | 0 | | | | | | |
| 6196 | 07356 | 06195 | 1 | | 0 | 0 | 0 | 0 | 0 | 0 | 0 | 0 | 0 | 0 | 0 | 0 | 0 | 0 | 0 | 0 | 0 | 0 | 0 | 1 | 0 | 0 | 0 | 0 | 0 | | | | | | |
| 6197 | 07357 | 06196 | 1 | | 0 | 0 | 0 | 0 | 0 | 0 | 0 | 0 | 0 | 0 | 0 | 0 | 0 | 0 | 0 | 0 | 0 | 0 | 0 | 1 | 0 | 0 | 0 | 0 | 0 | | | | | | |
| 6198 | 07358 | 06197 | 2 | | 0 | 0 | 0 | 0 | 0 | 0 | 0 | 0 | 0 | 0 | 0 | 0 | 0 | 0 | 0 | 0 | 0 | 0 | 0 | 1 | 0 | 1 | 0 | 0 | 0 | | | | | | |
| 6199 | 07359 | 06198 | 1 | | 0 | 0 | 0 | 0 | 0 | 0 | 0 | 0 | 0 | 0 | 0 | 0 | 0 | 0 | 0 | 0 | 0 | 0 | 0 | 1 | 0 | 0 | 0 | 0 | 0 | | | | | | |
| 6200 | 07360 | 06199 | 1 | | 0 | 0 | 0 | 0 | 0 | 0 | 0 | 0 | 0 | 0 | 0 | 0 | 0 | 0 | 0 | 0 | 0 | 0 | 0 | 1 | 0 | 0 | 0 | 0 | 0 | D14659 | 95.2 | 147 | 1 | 885 | 1219 |
| 6201 | 07361 | 06200 | 1 | | 0 | 0 | 0 | 0 | 0 | 0 | 0 | 0 | 0 | 0 | 0 | 0 | 0 | 0 | 0 | 0 | 0 | 0 | 0 | 1 | 0 | 0 | 0 | 0 | 0 | | | | | | |
| 6202 | 07362 | 06201 | 1 | | 0 | 0 | 0 | 0 | 0 | 0 | 0 | 0 | 0 | 0 | 0 | 0 | 0 | 0 | 0 | 0 | 0 | 0 | 0 | 1 | 0 | 0 | 0 | 0 | 0 | | | | | | |
| 6203 | 07363 | 06202 | 1 | | 0 | 0 | 0 | 0 | 0 | 0 | 0 | 0 | 0 | 0 | 0 | 0 | 0 | 0 | 0 | 0 | 0 | 0 | 0 | 1 | 0 | 0 | 0 | 0 | 0 | | | | | | |
| 6204 | 07364 | 06203 | 1 | | 0 | 0 | 0 | 0 | 0 | 0 | 0 | 0 | 0 | 0 | 0 | 0 | 0 | 0 | 0 | 0 | 0 | 0 | 0 | 1 | 0 | 0 | 0 | 0 | 0 | | | | | | |
| 6205 | 07365 | 06204 | 1 | | 0 | 0 | 0 | 0 | 0 | 0 | 0 | 0 | 0 | 0 | 0 | 0 | 0 | 0 | 0 | 0 | 0 | 0 | 0 | 1 | 0 | 0 | 0 | 0 | 0 | | | | | | |
| 6206 | 07366 | 06205 | 1 | | 0 | 0 | 0 | 0 | 0 | 0 | 0 | 0 | 0 | 0 | 0 | 0 | 0 | 0 | 0 | 0 | 0 | 0 | 0 | 1 | 0 | 0 | 0 | 0 | 0 | | | | | | |
| 6207 | 07367 | 06206 | 1 | | 0 | 0 | 0 | 0 | 0 | 0 | 0 | 0 | 0 | 0 | 0 | 0 | 0 | 0 | 0 | 0 | 0 | 0 | 0 | 1 | 0 | 0 | 0 | 0 | 0 | | | | | | |
| 6208 | 07368 | 06207 | 1 | | 0 | 0 | 0 | 0 | 0 | 0 | 0 | 0 | 0 | 0 | 0 | 0 | 0 | 0 | 0 | 0 | 0 | 0 | 0 | 1 | 0 | 0 | 0 | 0 | 0 | | | | | | |
| 6209 | 07369 | 06208 | 1 | | 0 | 0 | 0 | 0 | 0 | 0 | 0 | 0 | 0 | 0 | 0 | 0 | 0 | 0 | 0 | 0 | 0 | 0 | 0 | 1 | 0 | 0 | 0 | 0 | 0 | | | | | | |
| 6210 | 07370 | 06209 | 1 | | 0 | 0 | 0 | 0 | 0 | 0 | 0 | 0 | 0 | 0 | 0 | 0 | 0 | 0 | 0 | 0 | 0 | 0 | 0 | 1 | 0 | 0 | 0 | 0 | 0 | | | | | | |
| 6211 | 07371 | 06210 | 1 | | 0 | 0 | 0 | 0 | 0 | 0 | 0 | 0 | 0 | 0 | 0 | 0 | 0 | 0 | 0 | 0 | 0 | 0 | 0 | 1 | 0 | 0 | 0 | 0 | 0 | | | | | | |
| 6212 | 07372 | 06211 | 1 | | 0 | 0 | 0 | 0 | 0 | 0 | 0 | 0 | 0 | 0 | 0 | 0 | 0 | 0 | 0 | 0 | 0 | 0 | 0 | 1 | 0 | 0 | 0 | 0 | 0 | | | | | | |
| 6213 | 07373 | 06212 | 2 | | 0 | 0 | 0 | 0 | 0 | 0 | 0 | 0 | 0 | 0 | 0 | 0 | 0 | 0 | 0 | 0 | 0 | 0 | 0 | 2 | 0 | 0 | 0 | 0 | 0 | | | | | | |

Table 173

| | A | B | C | E | G | I | K | M | O | Q | S | U | W | Y | AA | AC | AE | AG | AI | AK | AM | AO | AQ | AS | AU | AW | AY | BA | BC | BE | BF | BG | BH | BI | BK |
|---|---|---|---|---|---|---|---|---|---|---|---|---|---|---|---|---|---|---|---|---|---|---|---|---|---|---|---|---|---|---|---|---|---|---|---|
| 6214 | 07374 | 06213 | 1 | | 0 | 0 | 0 | 0 | 0 | 0 | 0 | 0 | 0 | 0 | 0 | 0 | 0 | 0 | 0 | 0 | 0 | 0 | 0 | 1 | 0 | 0 | 0 | 0 | 0 | | | | | | |
| 6215 | 07375 | 06214 | 2 | | 0 | 0 | 0 | 0 | 0 | 0 | 0 | 0 | 0 | 0 | 0 | 0 | 0 | 0 | 0 | 0 | 0 | 0 | 0 | 2 | 0 | 0 | 0 | 0 | 0 | | | | | | |
| 6216 | 07376 | 06215 | 1 | | 0 | 0 | 0 | 0 | 0 | 0 | 0 | 0 | 0 | 0 | 0 | 0 | 0 | 0 | 0 | 0 | 0 | 0 | 0 | 1 | 0 | 0 | 0 | 0 | 0 | | | | | | |
| 6217 | 07377 | 06216 | 4 | | 0 | 0 | 0 | 0 | 0 | 0 | 0 | 0 | 0 | 0 | 0 | 0 | 0 | 0 | 0 | 0 | 0 | 0 | 0 | 1 | 1 | 0 | 2 | 0 | 0 | | | | | | |
| 6218 | 07378 | 06217 | 1 | | 0 | 0 | 0 | 0 | 0 | 0 | 0 | 0 | 0 | 0 | 0 | 0 | 0 | 0 | 0 | 0 | 0 | 0 | 0 | 1 | 0 | 0 | 0 | 0 | 0 | | | | | | |
| 6219 | 07379 | 06218 | 1 | | 0 | 0 | 0 | 0 | 0 | 0 | 0 | 0 | 0 | 0 | 0 | 0 | 0 | 0 | 0 | 0 | 0 | 0 | 0 | 1 | 0 | 0 | 0 | 0 | 0 | | | | | | |
| 6220 | 07380 | 06219 | 1 | | 0 | 0 | 0 | 0 | 0 | 0 | 0 | 0 | 0 | 0 | 0 | 0 | 0 | 0 | 0 | 0 | 0 | 0 | 0 | 1 | 0 | 0 | 0 | 0 | 0 | | | | | | |
| 6221 | 07381 | 06220 | 1 | | 0 | 0 | 0 | 0 | 0 | 0 | 0 | 0 | 0 | 0 | 0 | 0 | 0 | 0 | 0 | 0 | 0 | 0 | 0 | 1 | 0 | 0 | 0 | 0 | 0 | | | | | | |
| 6222 | 07382 | 06221 | 1 | | 0 | 0 | 0 | 0 | 0 | 0 | 0 | 0 | 0 | 0 | 0 | 0 | 0 | 0 | 0 | 0 | 0 | 0 | 0 | 1 | 0 | 0 | 0 | 0 | 0 | | | | | | |
| 6223 | 07383 | 06222 | 1 | | 0 | 0 | 0 | 0 | 0 | 0 | 0 | 0 | 0 | 0 | 0 | 0 | 0 | 0 | 0 | 0 | 0 | 0 | 0 | 1 | 0 | 0 | 0 | 0 | 0 | | | | | | |
| 6224 | 07384 | 06223 | 1 | | 0 | 0 | 0 | 0 | 0 | 0 | 0 | 0 | 0 | 0 | 0 | 0 | 0 | 0 | 0 | 0 | 0 | 0 | 0 | 1 | 0 | 0 | 0 | 0 | 0 | | | | | | |
| 6225 | 07385 | 06224 | 1 | | 0 | 0 | 0 | 0 | 0 | 0 | 0 | 0 | 0 | 0 | 0 | 0 | 0 | 0 | 0 | 0 | 0 | 0 | 0 | 1 | 0 | 0 | 0 | 0 | 0 | | | | | | |
| 6226 | 07386 | 06225 | 1 | | 0 | 0 | 0 | 0 | 0 | 0 | 0 | 0 | 0 | 0 | 0 | 0 | 0 | 0 | 0 | 0 | 0 | 0 | 0 | 1 | 0 | 0 | 0 | 0 | 0 | | | | | | |
| 6227 | 07387 | 06226 | 1 | | 0 | 0 | 0 | 0 | 0 | 0 | 0 | 0 | 0 | 0 | 0 | 0 | 0 | 0 | 0 | 0 | 0 | 0 | 0 | 1 | 0 | 0 | 0 | 0 | 0 | M77016 | 94 | 234 | 1 | 1369 | 2665 |
| 6228 | 07388 | 06227 | 3 | | 0 | 0 | 0 | 0 | 0 | 0 | 0 | 0 | 0 | 0 | 0 | 0 | 0 | 0 | 0 | 0 | 0 | 0 | 0 | 3 | 0 | 0 | 0 | 0 | 0 | | | | | | |
| 6229 | 07389 | 06228 | 1 | | 0 | 0 | 0 | 0 | 0 | 0 | 0 | 0 | 0 | 0 | 0 | 0 | 0 | 0 | 0 | 0 | 0 | 0 | 0 | 1 | 0 | 0 | 0 | 0 | 0 | | | | | | |
| 6230 | 07390 | 06229 | 1 | | 0 | 0 | 0 | 0 | 0 | 0 | 0 | 0 | 0 | 0 | 0 | 0 | 0 | 0 | 0 | 0 | 0 | 0 | 0 | 1 | 0 | 0 | 0 | 0 | 0 | | | | | | |
| 6231 | 07391 | 06230 | 1 | | 0 | 0 | 0 | 0 | 0 | 0 | 0 | 0 | 0 | 0 | 0 | 0 | 0 | 0 | 0 | 0 | 0 | 0 | 0 | 1 | 0 | 0 | 0 | 0 | 0 | | | | | | |
| 6232 | 07392 | 06231 | 2 | | 0 | 0 | 0 | 0 | 0 | 0 | 0 | 0 | 0 | 0 | 0 | 0 | 0 | 0 | 0 | 0 | 0 | 0 | 0 | 2 | 0 | 0 | 0 | 0 | 0 | | | | | | |
| 6233 | 07393 | 06232 | 1 | | 0 | 0 | 0 | 0 | 0 | 0 | 0 | 0 | 0 | 0 | 0 | 0 | 0 | 0 | 0 | 0 | 0 | 0 | 0 | 1 | 0 | 0 | 0 | 0 | 0 | | | | | | |
| 6234 | 07394 | 06233 | 1 | | 0 | 0 | 0 | 0 | 0 | 0 | 0 | 0 | 0 | 0 | 0 | 0 | 0 | 0 | 0 | 0 | 0 | 0 | 0 | 1 | 0 | 0 | 0 | 0 | 0 | J03068 | 92.9 | 141 | 1 | 2175 | 3317 |
| 6235 | 07395 | 06234 | 1 | | 0 | 0 | 0 | 0 | 0 | 0 | 0 | 0 | 0 | 0 | 0 | 0 | 0 | 0 | 0 | 0 | 0 | 0 | 0 | 1 | 0 | 0 | 0 | 0 | 0 | | | | | | |
| 6236 | 07396 | 06235 | 1 | | 0 | 0 | 0 | 0 | 0 | 0 | 0 | 0 | 0 | 0 | 0 | 0 | 0 | 0 | 0 | 0 | 0 | 0 | 0 | 1 | 0 | 0 | 0 | 0 | 0 | | | | | | |
| 6237 | 07397 | 06236 | 1 | | 0 | 0 | 0 | 0 | 0 | 0 | 0 | 0 | 0 | 0 | 0 | 0 | 0 | 0 | 0 | 0 | 0 | 0 | 0 | 1 | 0 | 0 | 0 | 0 | 0 | | | | | | |
| 6238 | 07398 | 06237 | 1 | | 0 | 0 | 0 | 0 | 0 | 0 | 0 | 0 | 0 | 0 | 0 | 0 | 0 | 0 | 0 | 0 | 0 | 0 | 0 | 1 | 0 | 0 | 0 | 0 | 0 | | | | | | |
| 6239 | 07399 | 06238 | 1 | | 0 | 0 | 0 | 0 | 0 | 0 | 0 | 0 | 0 | 0 | 0 | 0 | 0 | 0 | 0 | 0 | 0 | 0 | 0 | 1 | 0 | 0 | 0 | 0 | 0 | | | | | | |
| 6240 | 07400 | 06239 | 1 | | 0 | 0 | 0 | 0 | 0 | 0 | 0 | 0 | 0 | 0 | 0 | 0 | 0 | 0 | 0 | 0 | 0 | 0 | 0 | 1 | 0 | 0 | 0 | 0 | 0 | | | | | | |
| 6241 | 07401 | 06240 | 1 | | 0 | 0 | 0 | 0 | 0 | 0 | 0 | 0 | 0 | 0 | 0 | 0 | 0 | 0 | 0 | 0 | 0 | 0 | 0 | 1 | 0 | 0 | 0 | 0 | 0 | | | | | | |
| 6242 | 07402 | 06241 | 1 | | 0 | 0 | 0 | 0 | 0 | 0 | 0 | 0 | 0 | 0 | 0 | 0 | 0 | 0 | 0 | 0 | 0 | 0 | 0 | 1 | 0 | 0 | 0 | 0 | 0 | | | | | | |
| 6243 | 07403 | 06242 | 1 | | 0 | 0 | 0 | 0 | 0 | 0 | 0 | 0 | 0 | 0 | 0 | 0 | 0 | 0 | 0 | 0 | 0 | 0 | 0 | 1 | 0 | 0 | 0 | 0 | 0 | | | | | | |
| 6244 | 07404 | 06243 | 2 | | 0 | 0 | 0 | 0 | 0 | 0 | 0 | 0 | 0 | 0 | 0 | 0 | 0 | 0 | 0 | 0 | 0 | 0 | 0 | 1 | 0 | 0 | 1 | 0 | 0 | | | | | | |
| 6245 | 07405 | 06244 | 1 | | 0 | 0 | 0 | 0 | 0 | 0 | 0 | 0 | 0 | 0 | 0 | 0 | 0 | 0 | 0 | 0 | 0 | 0 | 0 | 1 | 0 | 0 | 0 | 0 | 0 | | | | | | |
| 6246 | 07406 | 06245 | 1 | | 0 | 0 | 0 | 0 | 0 | 0 | 0 | 0 | 0 | 0 | 0 | 0 | 0 | 0 | 0 | 0 | 0 | 0 | 0 | 1 | 0 | 0 | 0 | 0 | 0 | | | | | | |
| 6247 | 07407 | 06246 | 1 | | 0 | 0 | 0 | 0 | 0 | 0 | 0 | 0 | 0 | 0 | 0 | 0 | 0 | 0 | 0 | 0 | 0 | 0 | 0 | 1 | 0 | 0 | 0 | 0 | 0 | | | | | | |
| 6248 | 07408 | 06247 | 1 | | 0 | 0 | 0 | 0 | 0 | 0 | 0 | 0 | 0 | 0 | 0 | 0 | 0 | 0 | 0 | 0 | 0 | 0 | 0 | 1 | 0 | 0 | 0 | 0 | 0 | | | | | | |
| 6249 | 07409 | 06248 | 1 | | 0 | 0 | 0 | 0 | 0 | 0 | 0 | 0 | 0 | 0 | 0 | 0 | 0 | 0 | 0 | 0 | 0 | 0 | 0 | 1 | 0 | 0 | 0 | 0 | 0 | | | | | | |

Table 174

| | A | B | C | E | G | I | K | M | O | Q | S | U | W | Y | AA | AC | AE | AG | AI | AK | AM | AO | AQ | AS | AU | AW | AY | BA | BC | BE | BF | BG | BH | BI | BK |
|---|---|---|---|---|---|---|---|---|---|---|---|---|---|---|---|---|---|---|---|---|---|---|---|---|---|---|---|---|---|---|---|---|---|---|---|
| 6250 | 07410 | 06249 | 1 | | 0 | 0 | 0 | 0 | 0 | 0 | 0 | 0 | 0 | 0 | 0 | 0 | 0 | 0 | 0 | 0 | 0 | 0 | 0 | 1 | 0 | 0 | 0 | 0 | 0 | | | | | | |
| 6251 | 07411 | 06250 | 1 | | 0 | 0 | 0 | 0 | 0 | 0 | 0 | 0 | 0 | 0 | 0 | 0 | 0 | 0 | 0 | 0 | 0 | 0 | 0 | 1 | 0 | 0 | 0 | 0 | 0 | | | | | | |
| 6252 | 07412 | 06251 | 2 | | 0 | 0 | 0 | 0 | 0 | 0 | 0 | 0 | 0 | 0 | 0 | 0 | 0 | 0 | 0 | 0 | 0 | 0 | 0 | 2 | 0 | 0 | 0 | 0 | 0 | | | | | | |
| 6253 | 07413 | 06252 | 3 | | 0 | 0 | 0 | 0 | 0 | 0 | 0 | 0 | 0 | 0 | 0 | 0 | 0 | 0 | 0 | 0 | 0 | 0 | 0 | 3 | 0 | 0 | 0 | 0 | 0 | | | | | | |
| 6254 | 07414 | 06253 | 1 | | 0 | 0 | 0 | 0 | 0 | 0 | 0 | 0 | 0 | 0 | 0 | 0 | 0 | 0 | 0 | 0 | 0 | 0 | 0 | 1 | 0 | 0 | 0 | 0 | 0 | | | | | | |
| 6255 | 07415 | 06254 | 1 | | 0 | 0 | 0 | 0 | 0 | 0 | 0 | 0 | 0 | 0 | 0 | 0 | 0 | 0 | 0 | 0 | 0 | 0 | 0 | 1 | 0 | 0 | 0 | 0 | 0 | | | | | | |
| 6256 | 07416 | 06255 | 1 | | 0 | 0 | 0 | 0 | 0 | 0 | 0 | 0 | 0 | 0 | 0 | 0 | 0 | 0 | 0 | 0 | 0 | 0 | 0 | 1 | 0 | 0 | 0 | 0 | 0 | | | | | | |
| 6257 | 07417 | 06256 | 2 | | 0 | 0 | 0 | 0 | 0 | 0 | 0 | 0 | 0 | 0 | 0 | 0 | 0 | 0 | 0 | 0 | 0 | 0 | 0 | 2 | 0 | 0 | 0 | 0 | 0 | | | | | | |
| 6258 | 07418 | 06257 | 1 | | 0 | 0 | 0 | 0 | 0 | 0 | 0 | 0 | 0 | 0 | 0 | 0 | 0 | 0 | 0 | 0 | 0 | 0 | 0 | 1 | 0 | 0 | 0 | 0 | 0 | | | | | | |
| 6259 | 07419 | 06258 | 1 | | 0 | 0 | 0 | 0 | 0 | 0 | 0 | 0 | 0 | 0 | 0 | 0 | 0 | 0 | 0 | 0 | 0 | 0 | 0 | 1 | 0 | 0 | 0 | 0 | 0 | | | | | | |
| 6260 | 07420 | 06259 | 1 | | 0 | 0 | 0 | 0 | 0 | 0 | 0 | 0 | 0 | 0 | 0 | 0 | 0 | 0 | 0 | 0 | 0 | 0 | 0 | 1 | 0 | 0 | 0 | 0 | 0 | | | | | | |
| 6261 | 07421 | 06260 | 1 | | 0 | 0 | 0 | 0 | 0 | 0 | 0 | 0 | 0 | 0 | 0 | 0 | 0 | 0 | 0 | 0 | 0 | 0 | 0 | 1 | 0 | 0 | 0 | 0 | 0 | | | | | | |
| 6262 | 07422 | 06261 | 2 | | 0 | 0 | 0 | 0 | 0 | 0 | 0 | 0 | 0 | 0 | 0 | 0 | 0 | 0 | 0 | 0 | 0 | 0 | 0 | 1 | 0 | 1 | 0 | 0 | 0 | | | | | | |
| 6263 | 07423 | 06262 | 1 | | 0 | 0 | 0 | 0 | 0 | 0 | 0 | 0 | 0 | 0 | 0 | 0 | 0 | 0 | 0 | 0 | 0 | 0 | 0 | 1 | 0 | 0 | 0 | 0 | 0 | | | | | | |
| 6264 | 07424 | 06263 | 1 | | 0 | 0 | 0 | 0 | 0 | 0 | 0 | 0 | 0 | 0 | 0 | 0 | 0 | 0 | 0 | 0 | 0 | 0 | 0 | 1 | 0 | 0 | 0 | 0 | 0 | | | | | | |
| 6265 | 07425 | 06264 | 1 | | 0 | 0 | 0 | 0 | 0 | 0 | 0 | 0 | 0 | 0 | 0 | 0 | 0 | 0 | 0 | 0 | 0 | 0 | 0 | 1 | 0 | 0 | 0 | 0 | 0 | | | | | | |
| 6266 | 07426 | 06265 | 1 | | 0 | 0 | 0 | 0 | 0 | 0 | 0 | 0 | 0 | 0 | 0 | 0 | 0 | 0 | 0 | 0 | 0 | 0 | 0 | 1 | 0 | 0 | 0 | 0 | 0 | | | | | | |
| 6267 | 07427 | 06266 | 1 | | 0 | 0 | 0 | 0 | 0 | 0 | 0 | 0 | 0 | 0 | 0 | 0 | 0 | 0 | 0 | 0 | 0 | 0 | 0 | 1 | 0 | 0 | 0 | 0 | 0 | | | | | | |
| 6268 | 07429 | 06267 | 1 | | 0 | 0 | 0 | 0 | 0 | 0 | 0 | 0 | 0 | 0 | 0 | 0 | 0 | 0 | 0 | 0 | 0 | 0 | 0 | 1 | 0 | 0 | 0 | 0 | 0 | | | | | | |
| 6269 | 07430 | 06268 | 3 | | 0 | 0 | 0 | 0 | 0 | 0 | 0 | 0 | 0 | 0 | 0 | 0 | 0 | 0 | 0 | 0 | 0 | 0 | 0 | 2 | 0 | 0 | 1 | 0 | 0 | | | | | | |
| 6270 | 07431 | 06269 | 1 | | 0 | 0 | 0 | 0 | 0 | 0 | 0 | 0 | 0 | 0 | 0 | 0 | 0 | 0 | 0 | 0 | 0 | 0 | 0 | 1 | 0 | 0 | 0 | 0 | 0 | | | | | | |
| 6271 | 07432 | 06270 | 1 | | 0 | 0 | 0 | 0 | 0 | 0 | 0 | 0 | 0 | 0 | 0 | 0 | 0 | 0 | 0 | 0 | 0 | 0 | 0 | 1 | 0 | 0 | 0 | 0 | 0 | | | | | | |
| 6272 | 07433 | 06271 | 2 | | 0 | 0 | 0 | 0 | 0 | 0 | 0 | 0 | 0 | 0 | 0 | 0 | 0 | 0 | 0 | 0 | 0 | 0 | 0 | 1 | 0 | 0 | 0 | 1 | 0 | | | | | | |
| 6273 | 07434 | 06272 | 1 | | 0 | 0 | 0 | 0 | 0 | 0 | 0 | 0 | 0 | 0 | 0 | 0 | 0 | 0 | 0 | 0 | 0 | 0 | 0 | 1 | 0 | 0 | 0 | 0 | 0 | | | | | | |
| 6274 | 07435 | 06273 | 1 | | 0 | 0 | 0 | 0 | 0 | 0 | 0 | 0 | 0 | 0 | 0 | 0 | 0 | 0 | 0 | 0 | 0 | 0 | 0 | 1 | 0 | 0 | 0 | 0 | 0 | | | | | | |
| 6275 | 07436 | 06274 | 1 | | 0 | 0 | 0 | 0 | 0 | 0 | 0 | 0 | 0 | 0 | 0 | 0 | 0 | 0 | 0 | 0 | 0 | 0 | 0 | 1 | 0 | 0 | 0 | 0 | 0 | | | | | | |
| 6276 | 07437 | 06275 | 1 | | 0 | 0 | 0 | 0 | 0 | 0 | 0 | 0 | 0 | 0 | 0 | 0 | 0 | 0 | 0 | 0 | 0 | 0 | 0 | 1 | 0 | 0 | 0 | 0 | 0 | | | | | | |
| 6277 | 07438 | 06276 | 1 | | 0 | 0 | 0 | 0 | 0 | 0 | 0 | 0 | 0 | 0 | 0 | 0 | 0 | 0 | 0 | 0 | 0 | 0 | 0 | 1 | 0 | 0 | 0 | 0 | 0 | | | | | | |
| 6278 | 07439 | 06277 | 1 | | 0 | 0 | 0 | 0 | 0 | 0 | 0 | 0 | 0 | 0 | 0 | 0 | 0 | 0 | 0 | 0 | 0 | 0 | 0 | 1 | 0 | 0 | 0 | 0 | 0 | | | | | | |
| 6279 | 07440 | 06278 | 1 | | 0 | 0 | 0 | 0 | 0 | 0 | 0 | 0 | 0 | 0 | 0 | 0 | 0 | 0 | 0 | 0 | 0 | 0 | 0 | 1 | 0 | 0 | 0 | 0 | 0 | | | | | | |
| 6280 | 07441 | 06279 | 1 | | 0 | 0 | 0 | 0 | 0 | 0 | 0 | 0 | 0 | 0 | 0 | 0 | 0 | 0 | 0 | 0 | 0 | 0 | 0 | 1 | 0 | 0 | 0 | 0 | 0 | M99436 | 92.1 | 280 | 1 | 1990 | 2271 |
| 6281 | 07442 | 06280 | 1 | | 0 | 0 | 0 | 0 | 0 | 0 | 0 | 0 | 0 | 0 | 0 | 0 | 0 | 0 | 0 | 0 | 0 | 0 | 0 | 1 | 0 | 0 | 0 | 0 | 0 | | | | | | |
| 6282 | 07443 | 06281 | 2 | | 0 | 0 | 0 | 0 | 0 | 0 | 0 | 0 | 0 | 0 | 0 | 0 | 0 | 0 | 0 | 0 | 0 | 0 | 0 | 1 | 0 | 0 | 0 | 1 | 0 | Z25535 | 93.7 | 191 | 1 | 5299 | 5487 |
| 6283 | 07444 | 06282 | 1 | | 0 | 0 | 0 | 0 | 0 | 0 | 0 | 0 | 0 | 0 | 0 | 0 | 0 | 0 | 0 | 0 | 0 | 0 | 0 | 1 | 0 | 0 | 0 | 0 | 0 | | | | | | |
| 6284 | 07445 | 06283 | 1 | | 0 | 0 | 0 | 0 | 0 | 0 | 0 | 0 | 0 | 0 | 0 | 0 | 0 | 0 | 0 | 0 | 0 | 0 | 0 | 1 | 0 | 0 | 0 | 0 | 0 | | | | | | |
| 6285 | 07446 | 06284 | 1 | | 0 | 0 | 0 | 0 | 0 | 0 | 0 | 0 | 0 | 0 | 0 | 0 | 0 | 0 | 0 | 0 | 0 | 0 | 0 | 1 | 0 | 0 | 0 | 0 | 0 | | | | | | |

Table 175

| | A | B | C | E | G | I | K | M | O | Q | S | U | W | Y | AA | AC | AE | AG | AI | AK | AM | AO | AQ | AS | AU | AW | AY | BA | BC | BE | BF | BG | BH | BI | BK |
|---|---|---|---|---|---|---|---|---|---|---|---|---|---|---|---|---|---|---|---|---|---|---|---|---|---|---|---|---|---|---|---|---|---|---|---|
| 6286 | 07447 | 06285 | 1 | | 0 | 0 | 0 | 0 | 0 | 0 | 0 | 0 | 0 | 0 | 0 | 0 | 0 | 0 | 0 | 0 | 0 | 0 | 0 | 1 | 0 | 0 | 0 | 0 | 0 | | | | | | |
| 6287 | 07448 | 06286 | 1 | | 0 | 0 | 0 | 0 | 0 | 0 | 0 | 0 | 0 | 0 | 0 | 0 | 0 | 0 | 0 | 0 | 0 | 0 | 0 | 1 | 0 | 0 | 0 | 0 | 0 | | | | | | |
| 6288 | 07449 | 06287 | 2 | | 0 | 0 | 0 | 0 | 0 | 0 | 0 | 0 | 0 | 0 | 0 | 0 | 0 | 0 | 0 | 0 | 0 | 0 | 0 | 1 | 0 | 0 | 0 | 1 | 0 | | | | | | |
| 6289 | 07450 | 06288 | 1 | | 0 | 0 | 0 | 0 | 0 | 0 | 0 | 0 | 0 | 0 | 0 | 0 | 0 | 0 | 0 | 0 | 0 | 0 | 0 | 1 | 0 | 0 | 0 | 0 | 0 | | | | | | |
| 6290 | 07451 | 06289 | 1 | | 0 | 0 | 0 | 0 | 0 | 0 | 0 | 0 | 0 | 0 | 0 | 0 | 0 | 0 | 0 | 0 | 0 | 0 | 0 | 1 | 0 | 0 | 0 | 0 | 0 | | | | | | |
| 6291 | 07452 | 06290 | 1 | | 0 | 0 | 0 | 0 | 0 | 0 | 0 | 0 | 0 | 0 | 0 | 0 | 0 | 0 | 0 | 0 | 0 | 0 | 0 | 1 | 0 | 0 | 0 | 0 | 0 | | | | | | |
| 6292 | 07453 | 06291 | 1 | | 0 | 0 | 0 | 0 | 0 | 0 | 0 | 0 | 0 | 0 | 0 | 0 | 0 | 0 | 0 | 0 | 0 | 0 | 0 | 1 | 0 | 0 | 0 | 0 | 0 | | | | | | |
| 6293 | 07454 | 06292 | 1 | | 0 | 0 | 0 | 0 | 0 | 0 | 0 | 0 | 0 | 0 | 0 | 0 | 0 | 0 | 0 | 0 | 0 | 0 | 0 | 1 | 0 | 0 | 0 | 0 | 0 | | | | | | |
| 6294 | 07455 | 06293 | 1 | | 0 | 0 | 0 | 0 | 0 | 0 | 0 | 0 | 0 | 0 | 0 | 0 | 0 | 0 | 0 | 0 | 0 | 0 | 0 | 1 | 0 | 0 | 0 | 0 | 0 | | | | | | |
| 6295 | 07456 | 06294 | 1 | | 0 | 0 | 0 | 0 | 0 | 0 | 0 | 0 | 0 | 0 | 0 | 0 | 0 | 0 | 0 | 0 | 0 | 0 | 0 | 1 | 0 | 0 | 0 | 0 | 0 | | | | | | |
| 6296 | 07457 | 06295 | 1 | | 0 | 0 | 0 | 0 | 0 | 0 | 0 | 0 | 0 | 0 | 0 | 0 | 0 | 0 | 0 | 0 | 0 | 0 | 0 | 1 | 0 | 0 | 0 | 0 | 0 | | | | | | |
| 6297 | 07458 | 06296 | 1 | | 0 | 0 | 0 | 0 | 0 | 0 | 0 | 0 | 0 | 0 | 0 | 0 | 0 | 0 | 0 | 0 | 0 | 0 | 0 | 1 | 0 | 0 | 0 | 0 | 0 | | | | | | |
| 6298 | 07459 | 06297 | 1 | | 0 | 0 | 0 | 0 | 0 | 0 | 0 | 0 | 0 | 0 | 0 | 0 | 0 | 0 | 0 | 0 | 0 | 0 | 0 | 1 | 0 | 0 | 0 | 0 | 0 | | | | | | |
| 6299 | 07460 | 06298 | 1 | | 0 | 0 | 0 | 0 | 0 | 0 | 0 | 0 | 0 | 0 | 0 | 0 | 0 | 0 | 0 | 0 | 0 | 0 | 0 | 1 | 0 | 0 | 0 | 0 | 0 | | | | | | |
| 6300 | 07461 | 06299 | 1 | | 0 | 0 | 0 | 0 | 0 | 0 | 0 | 0 | 0 | 0 | 0 | 0 | 0 | 0 | 0 | 0 | 0 | 0 | 0 | 1 | 0 | 0 | 0 | 0 | 0 | | | | | | |
| 6301 | 07462 | 06300 | 1 | | 0 | 0 | 0 | 0 | 0 | 0 | 0 | 0 | 0 | 0 | 0 | 0 | 0 | 0 | 0 | 0 | 0 | 0 | 0 | 1 | 0 | 0 | 0 | 0 | 0 | | | | | | |
| 6302 | 07463 | 06301 | 1 | | 0 | 0 | 0 | 0 | 0 | 0 | 0 | 0 | 0 | 0 | 0 | 0 | 0 | 0 | 0 | 0 | 0 | 0 | 0 | 1 | 0 | 0 | 0 | 0 | 0 | | | | | | |
| 6303 | 07464 | 06302 | 1 | | 0 | 0 | 0 | 0 | 0 | 0 | 0 | 0 | 0 | 0 | 0 | 0 | 0 | 0 | 0 | 0 | 0 | 0 | 0 | 1 | 0 | 0 | 0 | 0 | 0 | | | | | | |
| 6304 | 07465 | 06303 | 1 | | 0 | 0 | 0 | 0 | 0 | 0 | 0 | 0 | 0 | 0 | 0 | 0 | 0 | 0 | 0 | 0 | 0 | 0 | 0 | 1 | 0 | 0 | 0 | 0 | 0 | | | | | | |
| 6305 | 07466 | 06304 | 1 | | 0 | 0 | 0 | 0 | 0 | 0 | 0 | 0 | 0 | 0 | 0 | 0 | 0 | 0 | 0 | 0 | 0 | 0 | 0 | 1 | 0 | 0 | 0 | 0 | 0 | | | | | | |
| 6306 | 07467 | 06305 | 1 | | 0 | 0 | 0 | 0 | 0 | 0 | 0 | 0 | 0 | 0 | 0 | 0 | 0 | 0 | 0 | 0 | 0 | 0 | 0 | 1 | 0 | 0 | 0 | 0 | 0 | | | | | | |
| 6307 | 07468 | 06306 | 1 | | 0 | 0 | 0 | 0 | 0 | 0 | 0 | 0 | 0 | 0 | 0 | 0 | 0 | 0 | 0 | 0 | 0 | 0 | 0 | 1 | 0 | 0 | 0 | 0 | 0 | | | | | | |
| 6308 | 07469 | 06307 | 1 | | 0 | 0 | 0 | 0 | 0 | 0 | 0 | 0 | 0 | 0 | 0 | 0 | 0 | 0 | 0 | 0 | 0 | 0 | 0 | 1 | 0 | 0 | 0 | 0 | 0 | | | | | | |
| 6309 | 07470 | 06308 | 1 | | 0 | 0 | 0 | 0 | 0 | 0 | 0 | 0 | 0 | 0 | 0 | 0 | 0 | 0 | 0 | 0 | 0 | 0 | 0 | 1 | 0 | 0 | 0 | 0 | 0 | | | | | | |
| 6310 | 07471 | 06309 | 2 | | 0 | 0 | 0 | 0 | 0 | 0 | 0 | 0 | 0 | 0 | 0 | 0 | 0 | 0 | 0 | 0 | 0 | 0 | 0 | 1 | 0 | 0 | 0 | 1 | 0 | | | | | | |
| 6311 | 07472 | 06310 | 1 | | 0 | 0 | 0 | 0 | 0 | 0 | 0 | 0 | 0 | 0 | 0 | 0 | 0 | 0 | 0 | 0 | 0 | 0 | 0 | 1 | 0 | 0 | 0 | 0 | 0 | | | | | | |
| 6312 | 07473 | 06311 | 1 | | 0 | 0 | 0 | 0 | 0 | 0 | 0 | 0 | 0 | 0 | 0 | 0 | 0 | 0 | 0 | 0 | 0 | 0 | 0 | 1 | 0 | 0 | 0 | 0 | 0 | | | | | | |
| 6313 | 07474 | 06312 | 1 | | 0 | 0 | 0 | 0 | 0 | 0 | 0 | 0 | 0 | 0 | 0 | 0 | 0 | 0 | 0 | 0 | 0 | 0 | 0 | 1 | 0 | 0 | 0 | 0 | 0 | | | | | | |
| 6314 | 07475 | 06313 | 2 | | 0 | 0 | 0 | 0 | 0 | 0 | 0 | 0 | 0 | 0 | 0 | 0 | 0 | 0 | 0 | 0 | 0 | 0 | 0 | 1 | 0 | 0 | 0 | 0 | 1 | | | | | | |
| 6315 | 07476 | 06314 | 1 | | 0 | 0 | 0 | 0 | 0 | 0 | 0 | 0 | 0 | 0 | 0 | 0 | 0 | 0 | 0 | 0 | 0 | 0 | 0 | 1 | 0 | 0 | 0 | 0 | 0 | | | | | | |
| 6316 | 07477 | 06315 | 1 | | 0 | 0 | 0 | 0 | 0 | 0 | 0 | 0 | 0 | 0 | 0 | 0 | 0 | 0 | 0 | 0 | 0 | 0 | 0 | 1 | 0 | 0 | 0 | 0 | 0 | | | | | | |
| 6317 | 07478 | 06316 | 1 | | 0 | 0 | 0 | 0 | 0 | 0 | 0 | 0 | 0 | 0 | 0 | 0 | 0 | 0 | 0 | 0 | 0 | 0 | 0 | 1 | 0 | 0 | 0 | 0 | 0 | | | | | | |
| 6318 | 07479 | 06317 | 1 | | 0 | 0 | 0 | 0 | 0 | 0 | 0 | 0 | 0 | 0 | 0 | 0 | 0 | 0 | 0 | 0 | 0 | 0 | 0 | 1 | 0 | 0 | 0 | 0 | 0 | | | | | | |
| 6319 | 07480 | 06318 | 1 | | 0 | 0 | 0 | 0 | 0 | 0 | 0 | 0 | 0 | 0 | 0 | 0 | 0 | 0 | 0 | 0 | 0 | 0 | 0 | 1 | 0 | 0 | 0 | 0 | 0 | | | | | | |
| 6320 | 07481 | 06319 | 1 | | 0 | 0 | 0 | 0 | 0 | 0 | 0 | 0 | 0 | 0 | 0 | 0 | 0 | 0 | 0 | 0 | 0 | 0 | 0 | 1 | 0 | 0 | 0 | 0 | 0 | | | | | | |
| 6321 | 07483 | 06320 | 1 | | 0 | 0 | 0 | 0 | 0 | 0 | 0 | 0 | 0 | 0 | 0 | 0 | 0 | 0 | 0 | 0 | 0 | 0 | 0 | 1 | 0 | 0 | 0 | 0 | 0 | | | | | | |

Table 176

| | A | B | C | E | G | I | K | M | O | Q | S | U | W | Y | AA | AC | AE | AG | AI | AK | AM | AO | AQ | AS | AU | AW | AY | BA | BC | BE | BF | BG | BH | BI | BK |
|---|---|---|---|---|---|---|---|---|---|---|---|---|---|---|---|---|---|---|---|---|---|---|---|---|---|---|---|---|---|---|---|---|---|---|---|
| 6322 | 07484 | 06321 | 1 | | 0 | 0 | 0 | 0 | 0 | 0 | 0 | 0 | 0 | 0 | 0 | 0 | 0 | 0 | 0 | 0 | 0 | 0 | 0 | 1 | 0 | 0 | 0 | 0 | 0 | M75126 | 94.2 | 330 | 1 | 3032 | 3580 |
| 6323 | 07485 | 06322 | 1 | | 0 | 0 | 0 | 0 | 0 | 0 | 0 | 0 | 0 | 0 | 0 | 0 | 0 | 0 | 0 | 0 | 0 | 0 | 0 | 1 | 0 | 0 | 0 | 0 | 0 | | | | | | |
| 6324 | 07486 | 06323 | 1 | | 0 | 0 | 0 | 0 | 0 | 0 | 0 | 0 | 0 | 0 | 0 | 0 | 0 | 0 | 0 | 0 | 0 | 0 | 0 | 1 | 0 | 0 | 0 | 0 | 0 | | | | | | |
| 6325 | 07487 | 06324 | 1 | | 0 | 0 | 0 | 0 | 0 | 0 | 0 | 0 | 0 | 0 | 0 | 0 | 0 | 0 | 0 | 0 | 0 | 0 | 0 | 1 | 0 | 0 | 0 | 0 | 0 | | | | | | |
| 6326 | 07488 | 06325 | 1 | | 0 | 0 | 0 | 0 | 0 | 0 | 0 | 0 | 0 | 0 | 0 | 0 | 0 | 0 | 0 | 0 | 0 | 0 | 0 | 1 | 0 | 0 | 0 | 0 | 0 | | | | | | |
| 6327 | 07489 | 06326 | 2 | | 0 | 0 | 0 | 0 | 0 | 0 | 0 | 0 | 0 | 0 | 0 | 0 | 0 | 0 | 0 | 0 | 0 | 0 | 0 | 2 | 0 | 0 | 0 | 0 | 0 | | | | | | |
| 6328 | 07490 | 06327 | 1 | | 0 | 0 | 0 | 0 | 0 | 0 | 0 | 0 | 0 | 0 | 0 | 0 | 0 | 0 | 0 | 0 | 0 | 0 | 0 | 1 | 0 | 0 | 0 | 0 | 0 | | | | | | |
| 6329 | 07491 | 06328 | 1 | | 0 | 0 | 0 | 0 | 0 | 0 | 0 | 0 | 0 | 0 | 0 | 0 | 0 | 0 | 0 | 0 | 0 | 0 | 0 | 1 | 0 | 0 | 0 | 0 | 0 | | | | | | |
| 6330 | 07492 | 06329 | 1 | | 0 | 0 | 0 | 0 | 0 | 0 | 0 | 0 | 0 | 0 | 0 | 0 | 0 | 0 | 0 | 0 | 0 | 0 | 0 | 1 | 0 | 0 | 0 | 0 | 0 | | | | | | |
| 6331 | 07493 | 06330 | 1 | | 0 | 0 | 0 | 0 | 0 | 0 | 0 | 0 | 0 | 0 | 0 | 0 | 0 | 0 | 0 | 0 | 0 | 0 | 0 | 1 | 0 | 0 | 0 | 0 | 0 | | | | | | |
| 6332 | 07494 | 06331 | 1 | | 0 | 0 | 0 | 0 | 0 | 0 | 0 | 0 | 0 | 0 | 0 | 0 | 0 | 0 | 0 | 0 | 0 | 0 | 0 | 1 | 0 | 0 | 0 | 0 | 0 | | | | | | |
| 6333 | 07495 | 06332 | 1 | | 0 | 0 | 0 | 0 | 0 | 0 | 0 | 0 | 0 | 0 | 0 | 0 | 0 | 0 | 0 | 0 | 0 | 0 | 0 | 1 | 0 | 0 | 0 | 0 | 0 | | | | | | |
| 6334 | 07496 | 06333 | 2 | | 0 | 0 | 0 | 0 | 0 | 0 | 0 | 0 | 0 | 0 | 0 | 0 | 0 | 0 | 0 | 0 | 0 | 0 | 0 | 1 | 0 | 0 | 0 | 1 | 0 | | | | | | |
| 6335 | 07497 | 06334 | 1 | | 0 | 0 | 0 | 0 | 0 | 0 | 0 | 0 | 0 | 0 | 0 | 0 | 0 | 0 | 0 | 0 | 0 | 0 | 0 | 1 | 0 | 0 | 0 | 0 | 0 | | | | | | |
| 6336 | 07498 | 06335 | 1 | | 0 | 0 | 0 | 0 | 0 | 0 | 0 | 0 | 0 | 0 | 0 | 0 | 0 | 0 | 0 | 0 | 0 | 0 | 0 | 1 | 0 | 0 | 0 | 0 | 0 | | | | | | |
| 6337 | 07499 | 06336 | 1 | | 0 | 0 | 0 | 0 | 0 | 0 | 0 | 0 | 0 | 0 | 0 | 0 | 0 | 0 | 0 | 0 | 0 | 0 | 0 | 1 | 0 | 0 | 0 | 0 | 0 | | | | | | |
| 6338 | 07500 | 06337 | 1 | | 0 | 0 | 0 | 0 | 0 | 0 | 0 | 0 | 0 | 0 | 0 | 0 | 0 | 0 | 0 | 0 | 0 | 0 | 0 | 1 | 0 | 0 | 0 | 0 | 0 | | | | | | |
| 6339 | 07501 | 06338 | 1 | | 0 | 0 | 0 | 0 | 0 | 0 | 0 | 0 | 0 | 0 | 0 | 0 | 0 | 0 | 0 | 0 | 0 | 0 | 0 | 1 | 0 | 0 | 0 | 0 | 0 | | | | | | |
| 6340 | 07502 | 06339 | 1 | | 0 | 0 | 0 | 0 | 0 | 0 | 0 | 0 | 0 | 0 | 0 | 0 | 0 | 0 | 0 | 0 | 0 | 0 | 0 | 1 | 0 | 0 | 0 | 0 | 0 | | | | | | |
| 6341 | 07503 | 06340 | 1 | | 0 | 0 | 0 | 0 | 0 | 0 | 0 | 0 | 0 | 0 | 0 | 0 | 0 | 0 | 0 | 0 | 0 | 0 | 0 | 1 | 0 | 0 | 0 | 0 | 0 | | | | | | |
| 6342 | 07504 | 06341 | 1 | | 0 | 0 | 0 | 0 | 0 | 0 | 0 | 0 | 0 | 0 | 0 | 0 | 0 | 0 | 0 | 0 | 0 | 0 | 0 | 1 | 0 | 0 | 0 | 0 | 0 | | | | | | |
| 6343 | 07505 | 06342 | 1 | | 0 | 0 | 0 | 0 | 0 | 0 | 0 | 0 | 0 | 0 | 0 | 0 | 0 | 0 | 0 | 0 | 0 | 0 | 0 | 1 | 0 | 0 | 0 | 0 | 0 | | | | | | |
| 6344 | 07506 | 06343 | 2 | | 0 | 0 | 0 | 0 | 0 | 0 | 0 | 0 | 0 | 0 | 0 | 0 | 0 | 0 | 0 | 0 | 0 | 0 | 0 | 1 | 0 | 0 | 0 | 1 | 0 | | | | | | |
| 6345 | 07507 | 06344 | 1 | | 0 | 0 | 0 | 0 | 0 | 0 | 0 | 0 | 0 | 0 | 0 | 0 | 0 | 0 | 0 | 0 | 0 | 0 | 0 | 1 | 0 | 0 | 0 | 0 | 0 | | | | | | |
| 6346 | 07508 | 06345 | 1 | | 0 | 0 | 0 | 0 | 0 | 0 | 0 | 0 | 0 | 0 | 0 | 0 | 0 | 0 | 0 | 0 | 0 | 0 | 0 | 1 | 0 | 0 | 0 | 0 | 0 | | | | | | |
| 6347 | 07509 | 06346 | 1 | | 0 | 0 | 0 | 0 | 0 | 0 | 0 | 0 | 0 | 0 | 0 | 0 | 0 | 0 | 0 | 0 | 0 | 0 | 0 | 1 | 0 | 0 | 0 | 0 | 0 | | | | | | |
| 6348 | 07510 | 06347 | 1 | | 0 | 0 | 0 | 0 | 0 | 0 | 0 | 0 | 0 | 0 | 0 | 0 | 0 | 0 | 0 | 0 | 0 | 0 | 0 | 1 | 0 | 0 | 0 | 0 | 0 | | | | | | |
| 6349 | 07511 | 06348 | 1 | | 0 | 0 | 0 | 0 | 0 | 0 | 0 | 0 | 0 | 0 | 0 | 0 | 0 | 0 | 0 | 0 | 0 | 0 | 0 | 1 | 0 | 0 | 0 | 0 | 0 | | | | | | |
| 6350 | 07512 | 06349 | 1 | | 0 | 0 | 0 | 0 | 0 | 0 | 0 | 0 | 0 | 0 | 0 | 0 | 0 | 0 | 0 | 0 | 0 | 0 | 0 | 1 | 0 | 0 | 0 | 0 | 0 | | | | | | |
| 6351 | 07513 | 06350 | 1 | | 0 | 0 | 0 | 0 | 0 | 0 | 0 | 0 | 0 | 0 | 0 | 0 | 0 | 0 | 0 | 0 | 0 | 0 | 0 | 1 | 0 | 0 | 0 | 0 | 0 | | | | | | |
| 6352 | 07514 | 06351 | 1 | | 0 | 0 | 0 | 0 | 0 | 0 | 0 | 0 | 0 | 0 | 0 | 0 | 0 | 0 | 0 | 0 | 0 | 0 | 0 | 1 | 0 | 0 | 0 | 0 | 0 | | | | | | |
| 6353 | 07515 | 06352 | 1 | | 0 | 0 | 0 | 0 | 0 | 0 | 0 | 0 | 0 | 0 | 0 | 0 | 0 | 0 | 0 | 0 | 0 | 0 | 0 | 1 | 0 | 0 | 0 | 0 | 0 | | | | | | |
| 6354 | 07516 | 06353 | 1 | | 0 | 0 | 0 | 0 | 0 | 0 | 0 | 0 | 0 | 0 | 0 | 0 | 0 | 0 | 0 | 0 | 0 | 0 | 0 | 1 | 0 | 0 | 0 | 0 | 0 | | | | | | |
| 6355 | 07517 | 06354 | 1 | | 0 | 0 | 0 | 0 | 0 | 0 | 0 | 0 | 0 | 0 | 0 | 0 | 0 | 0 | 0 | 0 | 0 | 0 | 0 | 1 | 0 | 0 | 0 | 0 | 0 | | | | | | |
| 6356 | 07518 | 06355 | 1 | | 0 | 0 | 0 | 0 | 0 | 0 | 0 | 0 | 0 | 0 | 0 | 0 | 0 | 0 | 0 | 0 | 0 | 0 | 0 | 1 | 0 | 0 | 0 | 0 | 0 | | | | | | |
| 6357 | 07520 | 06356 | 1 | | 0 | 0 | 0 | 0 | 0 | 0 | 0 | 0 | 0 | 0 | 0 | 0 | 0 | 0 | 0 | 0 | 0 | 0 | 0 | 1 | 0 | 0 | 0 | 0 | 0 | | | | | | |

Table 177

| | A | B | C | E | G | I | K | M | O | Q | S | U | W | Y | AA | AC | AE | AG | AI | AK | AM | AO | AQ | AS | AU | AW | AY | BA | BC | BE | BF | BG | BH | BI | BK |
|---|---|---|---|---|---|---|---|---|---|---|---|---|---|---|---|---|---|---|---|---|---|---|---|---|---|---|---|---|---|---|---|---|---|---|---|
| 6358 | 07521 | 06357 | 1 | | 0 | 0 | 0 | 0 | 0 | 0 | 0 | 0 | 0 | 0 | 0 | 0 | 0 | 0 | 0 | 0 | 0 | 0 | 0 | 1 | 0 | 0 | 0 | 0 | 0 | | | | | | |
| 6359 | 07522 | 06358 | 1 | | 0 | 0 | 0 | 0 | 0 | 0 | 0 | 0 | 0 | 0 | 0 | 0 | 0 | 0 | 0 | 0 | 0 | 0 | 0 | 1 | 0 | 0 | 0 | 0 | 0 | | | | | | |
| 6360 | 07523 | 06359 | 1 | | 0 | 0 | 0 | 0 | 0 | 0 | 0 | 0 | 0 | 0 | 0 | 0 | 0 | 0 | 0 | 0 | 0 | 0 | 0 | 1 | 0 | 0 | 0 | 0 | 0 | | | | | | |
| 6361 | 07524 | 06360 | 1 | | 0 | 0 | 0 | 0 | 0 | 0 | 0 | 0 | 0 | 0 | 0 | 0 | 0 | 0 | 0 | 0 | 0 | 0 | 0 | 1 | 0 | 0 | 0 | 0 | 0 | | | | | | |
| 6362 | 07525 | 06361 | 1 | | 0 | 0 | 0 | 0 | 0 | 0 | 0 | 0 | 0 | 0 | 0 | 0 | 0 | 0 | 0 | 0 | 0 | 0 | 0 | 1 | 0 | 0 | 0 | 0 | 0 | | | | | | |
| 6363 | 07526 | 06362 | 1 | | 0 | 0 | 0 | 0 | 0 | 0 | 0 | 0 | 0 | 0 | 0 | 0 | 0 | 0 | 0 | 0 | 0 | 0 | 0 | 1 | 0 | 0 | 0 | 0 | 0 | M38054 | 98 | 102 | 1 | 112 | 213 |
| 6364 | 07527 | 06363 | 1 | | 0 | 0 | 0 | 0 | 0 | 0 | 0 | 0 | 0 | 0 | 0 | 0 | 0 | 0 | 0 | 0 | 0 | 0 | 0 | 1 | 0 | 0 | 0 | 0 | 0 | | | | | | |
| 6365 | 07528 | 06364 | 1 | | 0 | 0 | 0 | 0 | 0 | 0 | 0 | 0 | 0 | 0 | 0 | 0 | 0 | 0 | 0 | 0 | 0 | 0 | 0 | 1 | 0 | 0 | 0 | 0 | 0 | | | | | | |
| 6366 | 07529 | 06365 | 1 | | 0 | 0 | 0 | 0 | 0 | 0 | 0 | 0 | 0 | 0 | 0 | 0 | 0 | 0 | 0 | 0 | 0 | 0 | 0 | 1 | 0 | 0 | 0 | 0 | 0 | | | | | | |
| 6367 | 07530 | 06366 | 1 | | 0 | 0 | 0 | 0 | 0 | 0 | 0 | 0 | 0 | 0 | 0 | 0 | 0 | 0 | 0 | 0 | 0 | 0 | 0 | 1 | 0 | 0 | 0 | 0 | 0 | | | | | | |
| 6368 | 07531 | 06367 | 1 | | 0 | 0 | 0 | 0 | 0 | 0 | 0 | 0 | 0 | 0 | 0 | 0 | 0 | 0 | 0 | 0 | 0 | 0 | 0 | 1 | 0 | 0 | 0 | 0 | 0 | | | | | | |
| 6369 | 07532 | 06368 | 1 | | 0 | 0 | 0 | 0 | 0 | 0 | 0 | 0 | 0 | 0 | 0 | 0 | 0 | 0 | 0 | 0 | 0 | 0 | 0 | 1 | 0 | 0 | 0 | 0 | 0 | | | | | | |
| 6370 | 07533 | 06369 | 1 | | 0 | 0 | 0 | 0 | 0 | 0 | 0 | 0 | 0 | 0 | 0 | 0 | 0 | 0 | 0 | 0 | 0 | 0 | 0 | 1 | 0 | 0 | 0 | 0 | 0 | | | | | | |
| 6371 | 07534 | 06370 | 1 | | 0 | 0 | 0 | 0 | 0 | 0 | 0 | 0 | 0 | 0 | 0 | 0 | 0 | 0 | 0 | 0 | 0 | 0 | 0 | 1 | 0 | 0 | 0 | 0 | 0 | | | | | | |
| 6372 | 07535 | 06371 | 1 | | 0 | 0 | 0 | 0 | 0 | 0 | 0 | 0 | 0 | 0 | 0 | 0 | 0 | 0 | 0 | 0 | 0 | 0 | 0 | 1 | 0 | 0 | 0 | 0 | 0 | | | | | | |
| 6373 | 07536 | 06372 | 1 | | 0 | 0 | 0 | 0 | 0 | 0 | 0 | 0 | 0 | 0 | 0 | 0 | 0 | 0 | 0 | 0 | 0 | 0 | 0 | 1 | 0 | 0 | 0 | 0 | 0 | | | | | | |
| 6374 | 07537 | 06373 | 1 | | 0 | 0 | 0 | 0 | 0 | 0 | 0 | 0 | 0 | 0 | 0 | 0 | 0 | 0 | 0 | 0 | 0 | 0 | 0 | 1 | 0 | 0 | 0 | 0 | 0 | | | | | | |
| 6375 | 07538 | 06374 | 1 | | 0 | 0 | 0 | 0 | 0 | 0 | 0 | 0 | 0 | 0 | 0 | 0 | 0 | 0 | 0 | 0 | 0 | 0 | 0 | 1 | 0 | 0 | 0 | 0 | 0 | | | | | | |
| 6376 | 07539 | 06375 | 1 | | 0 | 0 | 0 | 0 | 0 | 0 | 0 | 0 | 0 | 0 | 0 | 0 | 0 | 0 | 0 | 0 | 0 | 0 | 0 | 1 | 0 | 0 | 0 | 0 | 0 | | | | | | |
| 6377 | 07540 | 06376 | 1 | | 0 | 0 | 0 | 0 | 0 | 0 | 0 | 0 | 0 | 0 | 0 | 0 | 0 | 0 | 0 | 0 | 0 | 0 | 0 | 1 | 0 | 0 | 0 | 0 | 0 | | | | | | |
| 6378 | 07541 | 06377 | 1 | | 0 | 0 | 0 | 0 | 0 | 0 | 0 | 0 | 0 | 0 | 0 | 0 | 0 | 0 | 0 | 0 | 0 | 0 | 0 | 1 | 0 | 0 | 0 | 0 | 0 | | | | | | |
| 6379 | 07543 | 06378 | 1 | | 0 | 0 | 0 | 0 | 0 | 0 | 0 | 0 | 0 | 0 | 0 | 0 | 0 | 0 | 0 | 0 | 0 | 0 | 0 | 1 | 0 | 0 | 0 | 0 | 0 | | | | | | |
| 6380 | 07544 | 06379 | 1 | | 0 | 0 | 0 | 0 | 0 | 0 | 0 | 0 | 0 | 0 | 0 | 0 | 0 | 0 | 0 | 0 | 0 | 0 | 0 | 1 | 0 | 0 | 0 | 0 | 0 | | | | | | |
| 6381 | 07545 | 06380 | 1 | | 0 | 0 | 0 | 0 | 0 | 0 | 0 | 0 | 0 | 0 | 0 | 0 | 0 | 0 | 0 | 0 | 0 | 0 | 0 | 1 | 0 | 0 | 0 | 0 | 0 | | | | | | |
| 6382 | 07546 | 06381 | 2 | | 0 | 0 | 0 | 0 | 0 | 0 | 0 | 0 | 0 | 0 | 0 | 0 | 0 | 0 | 0 | 0 | 0 | 0 | 0 | 1 | 0 | 0 | 1 | 0 | 0 | | | | | | |
| 6383 | 07547 | 06382 | 1 | | 0 | 0 | 0 | 0 | 0 | 0 | 0 | 0 | 0 | 0 | 0 | 0 | 0 | 0 | 0 | 0 | 0 | 0 | 0 | 1 | 0 | 0 | 0 | 0 | 0 | | | | | | |
| 6384 | 07548 | 06383 | 1 | | 0 | 0 | 0 | 0 | 0 | 0 | 0 | 0 | 0 | 0 | 0 | 0 | 0 | 0 | 0 | 0 | 0 | 0 | 0 | 1 | 0 | 0 | 0 | 0 | 0 | | | | | | |
| 6385 | 07549 | 06384 | 1 | | 0 | 0 | 0 | 0 | 0 | 0 | 0 | 0 | 0 | 0 | 0 | 0 | 0 | 0 | 0 | 0 | 0 | 0 | 0 | 1 | 0 | 0 | 0 | 0 | 0 | | | | | | |
| 6386 | 07550 | 06385 | 1 | | 0 | 0 | 0 | 0 | 0 | 0 | 0 | 0 | 0 | 0 | 0 | 0 | 0 | 0 | 0 | 0 | 0 | 0 | 0 | 1 | 0 | 0 | 0 | 0 | 0 | | | | | | |
| 6387 | 07551 | 06386 | 1 | | 0 | 0 | 0 | 0 | 0 | 0 | 0 | 0 | 0 | 0 | 0 | 0 | 0 | 0 | 0 | 0 | 0 | 0 | 0 | 1 | 0 | 0 | 0 | 0 | 0 | | | | | | |
| 6388 | 07552 | 06387 | 2 | | 0 | 0 | 0 | 0 | 0 | 0 | 0 | 0 | 0 | 0 | 0 | 0 | 0 | 0 | 0 | 0 | 0 | 0 | 0 | 2 | 0 | 0 | 0 | 0 | 0 | | | | | | |
| 6389 | 07553 | 06388 | 1 | | 0 | 0 | 0 | 0 | 0 | 0 | 0 | 0 | 0 | 0 | 0 | 0 | 0 | 0 | 0 | 0 | 0 | 0 | 0 | 1 | 0 | 0 | 0 | 0 | 0 | | | | | | |
| 6390 | 07554 | 06389 | 1 | | 0 | 0 | 0 | 0 | 0 | 0 | 0 | 0 | 0 | 0 | 0 | 0 | 0 | 0 | 0 | 0 | 0 | 0 | 0 | 1 | 0 | 0 | 0 | 0 | 0 | | | | | | |
| 6391 | 07555 | 06390 | 1 | | 0 | 0 | 0 | 0 | 0 | 0 | 0 | 0 | 0 | 0 | 0 | 0 | 0 | 0 | 0 | 0 | 0 | 0 | 0 | 1 | 0 | 0 | 0 | 0 | 0 | | | | | | |
| 6392 | 07556 | 06391 | 3 | | 0 | 0 | 0 | 0 | 0 | 0 | 0 | 0 | 0 | 0 | 0 | 0 | 0 | 0 | 0 | 0 | 0 | 0 | 0 | 1 | 0 | 2 | 0 | 0 | 0 | | | | | | |
| 6393 | 07557 | 06392 | 2 | | 0 | 0 | 0 | 0 | 0 | 0 | 0 | 0 | 0 | 0 | 0 | 0 | 0 | 0 | 0 | 0 | 0 | 0 | 0 | 2 | 0 | 0 | 0 | 0 | 0 | | | | | | |

Table 178

| | A | B | C | E | G | I | K | M | O | Q | S | U | W | Y | AA | AC | AE | AG | AI | AK | AM | AO | AQ | AS | AU | AW | AY | BA | BC | BE | BF | BG | BH | BI | BK |
|---|---|---|---|---|---|---|---|---|---|---|---|---|---|---|---|---|---|---|---|---|---|---|---|---|---|---|---|---|---|---|---|---|---|---|---|
| 6394 | 07558 | 06393 | 1 | | 0 | 0 | 0 | 0 | 0 | 0 | 0 | 0 | 0 | 0 | 0 | 0 | 0 | 0 | 0 | 0 | 0 | 0 | 0 | 1 | 0 | 0 | 0 | 0 | 0 | | | | | | |
| 6395 | 07559 | 06394 | 1 | | 0 | 0 | 0 | 0 | 0 | 0 | 0 | 0 | 0 | 0 | 0 | 0 | 0 | 0 | 0 | 0 | 0 | 0 | 0 | 1 | 0 | 0 | 0 | 0 | 0 | M18172 | 97.5 | 161 | 1 | 674 | 834 |
| 6396 | 07560 | 06395 | 1 | | 0 | 0 | 0 | 0 | 0 | 0 | 0 | 0 | 0 | 0 | 0 | 0 | 0 | 0 | 0 | 0 | 0 | 0 | 0 | 1 | 0 | 0 | 0 | 0 | 0 | | | | | | |
| 6397 | 07561 | 06396 | 1 | | 0 | 0 | 0 | 0 | 0 | 0 | 0 | 0 | 0 | 0 | 0 | 0 | 0 | 0 | 0 | 0 | 0 | 0 | 0 | 1 | 0 | 0 | 0 | 0 | 0 | | | | | | |
| 6398 | 07562 | 06397 | 2 | | 0 | 0 | 0 | 0 | 0 | 0 | 0 | 0 | 0 | 0 | 0 | 0 | 0 | 0 | 0 | 0 | 0 | 0 | 0 | 1 | 0 | 0 | 0 | 1 | 0 | | | | | | |
| 6399 | 07563 | 06398 | 2 | | 0 | 0 | 0 | 0 | 0 | 0 | 0 | 0 | 0 | 0 | 0 | 0 | 0 | 0 | 0 | 0 | 0 | 0 | 0 | 1 | 0 | 0 | 0 | 1 | 0 | | | | | | |
| 6400 | 07564 | 06399 | 1 | | 0 | 0 | 0 | 0 | 0 | 0 | 0 | 0 | 0 | 0 | 0 | 0 | 0 | 0 | 0 | 0 | 0 | 0 | 0 | 1 | 0 | 0 | 0 | 0 | 0 | | | | | | |
| 6401 | 07565 | 06400 | 1 | | 0 | 0 | 0 | 0 | 0 | 0 | 0 | 0 | 0 | 0 | 0 | 0 | 0 | 0 | 0 | 0 | 0 | 0 | 0 | 1 | 0 | 0 | 0 | 0 | 0 | | | | | | |
| 6402 | 07567 | 06401 | 1 | | 0 | 0 | 0 | 0 | 0 | 0 | 0 | 0 | 0 | 0 | 0 | 0 | 0 | 0 | 0 | 0 | 0 | 0 | 0 | 1 | 0 | 0 | 0 | 0 | 0 | | | | | | |
| 6403 | 07568 | 06402 | 3 | | 0 | 0 | 0 | 0 | 0 | 0 | 0 | 0 | 0 | 0 | 0 | 0 | 0 | 0 | 0 | 0 | 0 | 0 | 0 | 1 | 0 | 0 | 0 | 2 | 0 | | | | | | |
| 6404 | 07569 | 06403 | 3 | | 0 | 0 | 0 | 0 | 0 | 0 | 0 | 0 | 0 | 0 | 0 | 0 | 0 | 0 | 0 | 0 | 0 | 0 | 0 | 1 | 2 | 0 | 0 | 0 | 0 | | | | | | |
| 6405 | 07570 | 06404 | 3 | | 0 | 0 | 0 | 0 | 0 | 0 | 0 | 0 | 0 | 0 | 0 | 0 | 0 | 0 | 0 | 0 | 0 | 0 | 0 | 1 | 0 | 0 | 2 | 0 | 0 | | | | | | |
| 6406 | 07571 | 06405 | 1 | | 0 | 0 | 0 | 0 | 0 | 0 | 0 | 0 | 0 | 0 | 0 | 0 | 0 | 0 | 0 | 0 | 0 | 0 | 0 | 1 | 0 | 0 | 0 | 0 | 0 | | | | | | |
| 6407 | 07572 | 06406 | 1 | | 0 | 0 | 0 | 0 | 0 | 0 | 0 | 0 | 0 | 0 | 0 | 0 | 0 | 0 | 0 | 0 | 0 | 0 | 0 | 1 | 0 | 0 | 0 | 0 | 0 | | | | | | |
| 6408 | 07573 | 06407 | 1 | | 0 | 0 | 0 | 0 | 0 | 0 | 0 | 0 | 0 | 0 | 0 | 0 | 0 | 0 | 0 | 0 | 0 | 0 | 0 | 1 | 0 | 0 | 0 | 0 | 0 | | | | | | |
| 6409 | 07574 | 06408 | 1 | | 0 | 0 | 0 | 0 | 0 | 0 | 0 | 0 | 0 | 0 | 0 | 0 | 0 | 0 | 0 | 0 | 0 | 0 | 0 | 1 | 0 | 0 | 0 | 0 | 0 | | | | | | |
| 6410 | 07575 | 06409 | 2 | | 0 | 0 | 0 | 0 | 0 | 0 | 0 | 0 | 0 | 0 | 0 | 0 | 0 | 0 | 0 | 0 | 0 | 0 | 0 | 2 | 0 | 0 | 0 | 0 | 0 | | | | | | |
| 6411 | 07576 | 06410 | 1 | | 0 | 0 | 0 | 0 | 0 | 0 | 0 | 0 | 0 | 0 | 0 | 0 | 0 | 0 | 0 | 0 | 0 | 0 | 0 | 1 | 0 | 0 | 0 | 0 | 0 | | | | | | |
| 6412 | 07577 | 06411 | 1 | | 0 | 0 | 0 | 0 | 0 | 0 | 0 | 0 | 0 | 0 | 0 | 0 | 0 | 0 | 0 | 0 | 0 | 0 | 0 | 1 | 0 | 0 | 0 | 0 | 0 | | | | | | |
| 6413 | 07578 | 06412 | 1 | | 0 | 0 | 0 | 0 | 0 | 0 | 0 | 0 | 0 | 0 | 0 | 0 | 0 | 0 | 0 | 0 | 0 | 0 | 0 | 1 | 0 | 0 | 0 | 0 | 0 | | | | | | |
| 6414 | 07579 | 06413 | 1 | | 0 | 0 | 0 | 0 | 0 | 0 | 0 | 0 | 0 | 0 | 0 | 0 | 0 | 0 | 0 | 0 | 0 | 0 | 0 | 1 | 0 | 0 | 0 | 0 | 0 | | | | | | |
| 6415 | 07580 | 06414 | 1 | | 0 | 0 | 0 | 0 | 0 | 0 | 0 | 0 | 0 | 0 | 0 | 0 | 0 | 0 | 0 | 0 | 0 | 0 | 0 | 1 | 0 | 0 | 0 | 0 | 0 | | | | | | |
| 6416 | 07581 | 06415 | 1 | | 0 | 0 | 0 | 0 | 0 | 0 | 0 | 0 | 0 | 0 | 0 | 0 | 0 | 0 | 0 | 0 | 0 | 0 | 0 | 1 | 0 | 0 | 0 | 0 | 0 | | | | | | |
| 6417 | 07582 | 06416 | 1 | | 0 | 0 | 0 | 0 | 0 | 0 | 0 | 0 | 0 | 0 | 0 | 0 | 0 | 0 | 0 | 0 | 0 | 0 | 0 | 1 | 0 | 0 | 0 | 0 | 0 | | | | | | |
| 6418 | 07583 | 06417 | 2 | | 0 | 0 | 0 | 0 | 0 | 0 | 0 | 0 | 0 | 0 | 0 | 0 | 0 | 0 | 0 | 0 | 0 | 0 | 0 | 2 | 0 | 0 | 0 | 0 | 0 | | | | | | |
| 6419 | 07584 | 06418 | 1 | | 0 | 0 | 0 | 0 | 0 | 0 | 0 | 0 | 0 | 0 | 0 | 0 | 0 | 0 | 0 | 0 | 0 | 0 | 0 | 1 | 0 | 0 | 0 | 0 | 0 | | | | | | |
| 6420 | 07585 | 06419 | 1 | | 0 | 0 | 0 | 0 | 0 | 0 | 0 | 0 | 0 | 0 | 0 | 0 | 0 | 0 | 0 | 0 | 0 | 0 | 0 | 1 | 0 | 0 | 0 | 0 | 0 | | | | | | |
| 6421 | 07586 | 06420 | 1 | | 0 | 0 | 0 | 0 | 0 | 0 | 0 | 0 | 0 | 0 | 0 | 0 | 0 | 0 | 0 | 0 | 0 | 0 | 0 | 1 | 0 | 0 | 0 | 0 | 0 | | | | | | |
| 6422 | 07587 | 06421 | 1 | | 0 | 0 | 0 | 0 | 0 | 0 | 0 | 0 | 0 | 0 | 0 | 0 | 0 | 0 | 0 | 0 | 0 | 0 | 0 | 1 | 0 | 0 | 0 | 0 | 0 | | | | | | |
| 6423 | 07588 | 06422 | 1 | | 0 | 0 | 0 | 0 | 0 | 0 | 0 | 0 | 0 | 0 | 0 | 0 | 0 | 0 | 0 | 0 | 0 | 0 | 0 | 1 | 0 | 0 | 0 | 0 | 0 | | | | | | |
| 6424 | 07589 | 06423 | 1 | | 0 | 0 | 0 | 0 | 0 | 0 | 0 | 0 | 0 | 0 | 0 | 0 | 0 | 0 | 0 | 0 | 0 | 0 | 0 | 1 | 0 | 0 | 0 | 0 | 0 | | | | | | |
| 6425 | 07590 | 06424 | 1 | | 0 | 0 | 0 | 0 | 0 | 0 | 0 | 0 | 0 | 0 | 0 | 0 | 0 | 0 | 0 | 0 | 0 | 0 | 0 | 1 | 0 | 0 | 0 | 0 | 0 | | | | | | |
| 6426 | 07591 | 06425 | 1 | | 0 | 0 | 0 | 0 | 0 | 0 | 0 | 0 | 0 | 0 | 0 | 0 | 0 | 0 | 0 | 0 | 0 | 0 | 0 | 1 | 0 | 0 | 0 | 0 | 0 | | | | | | |
| 6427 | 07592 | 06426 | 1 | | 0 | 0 | 0 | 0 | 0 | 0 | 0 | 0 | 0 | 0 | 0 | 0 | 0 | 0 | 0 | 0 | 0 | 0 | 0 | 1 | 0 | 0 | 0 | 0 | 0 | | | | | | |
| 6428 | 07593 | 06427 | 1 | | 0 | 0 | 0 | 0 | 0 | 0 | 0 | 0 | 0 | 0 | 0 | 0 | 0 | 0 | 0 | 0 | 0 | 0 | 0 | 1 | 0 | 0 | 0 | 0 | 0 | | | | | | |
| 6429 | 07594 | 06428 | 1 | | 0 | 0 | 0 | 0 | 0 | 0 | 0 | 0 | 0 | 0 | 0 | 0 | 0 | 0 | 0 | 0 | 0 | 0 | 0 | 1 | 0 | 0 | 0 | 0 | 0 | | | | | | |

Table 179

| | A | B | C | E | G | I | K | M | O | Q | S | U | W | Y | AA | AC | AE | AG | AI | AK | AM | AO | AQ | AS | AU | AW | AY | BA | BC | BE | BF | BG | BH | BI | BK |
|---|---|---|---|---|---|---|---|---|---|---|---|---|---|---|---|---|---|---|---|---|---|---|---|---|---|---|---|---|---|---|---|---|---|---|---|
| 6430 | 07595 | 06429 | 2 | | 0 | 0 | 0 | 0 | 0 | 0 | 0 | 0 | 0 | 0 | 0 | 0 | 0 | 0 | 0 | 0 | 0 | 0 | 0 | 1 | 1 | 0 | 0 | 0 | 0 | | | | | | |
| 6431 | 07596 | 06430 | 1 | | 0 | 0 | 0 | 0 | 0 | 0 | 0 | 0 | 0 | 0 | 0 | 0 | 0 | 0 | 0 | 0 | 0 | 0 | 0 | 1 | 0 | 0 | 0 | 0 | 0 | | | | | | |
| 6432 | 07597 | 06431 | 1 | | 0 | 0 | 0 | 0 | 0 | 0 | 0 | 0 | 0 | 0 | 0 | 0 | 0 | 0 | 0 | 0 | 0 | 0 | 0 | 1 | 0 | 0 | 0 | 0 | 0 | | | | | | |
| 6433 | 07598 | 06432 | 1 | | 0 | 0 | 0 | 0 | 0 | 0 | 0 | 0 | 0 | 0 | 0 | 0 | 0 | 0 | 0 | 0 | 0 | 0 | 0 | 1 | 0 | 0 | 0 | 0 | 0 | | | | | | |
| 6434 | 07599 | 06433 | 2 | | 0 | 0 | 0 | 0 | 0 | 0 | 0 | 0 | 0 | 0 | 0 | 0 | 0 | 0 | 0 | 0 | 0 | 0 | 0 | 2 | 0 | 0 | 0 | 0 | 0 | | | | | | |
| 6435 | 07600 | 06434 | 1 | | 0 | 0 | 0 | 0 | 0 | 0 | 0 | 0 | 0 | 0 | 0 | 0 | 0 | 0 | 0 | 0 | 0 | 0 | 0 | 1 | 0 | 0 | 0 | 0 | 0 | | | | | | |
| 6436 | 07601 | 06435 | 1 | | 0 | 0 | 0 | 0 | 0 | 0 | 0 | 0 | 0 | 0 | 0 | 0 | 0 | 0 | 0 | 0 | 0 | 0 | 0 | 1 | 0 | 0 | 0 | 0 | 0 | | | | | | |
| 6437 | 07602 | 06436 | 1 | | 0 | 0 | 0 | 0 | 0 | 0 | 0 | 0 | 0 | 0 | 0 | 0 | 0 | 0 | 0 | 0 | 0 | 0 | 0 | 1 | 0 | 0 | 0 | 0 | 0 | | | | | | |
| 6438 | 07603 | 06437 | 1 | | 0 | 0 | 0 | 0 | 0 | 0 | 0 | 0 | 0 | 0 | 0 | 0 | 0 | 0 | 0 | 0 | 0 | 0 | 0 | 1 | 0 | 0 | 0 | 0 | 0 | | | | | | |
| 6439 | 07604 | 06438 | 2 | | 0 | 0 | 0 | 0 | 0 | 0 | 0 | 0 | 0 | 0 | 0 | 0 | 0 | 0 | 0 | 0 | 0 | 0 | 0 | 1 | 0 | 1 | 0 | 0 | 0 | | | | | | |
| 6440 | 07605 | 06439 | 1 | | 0 | 0 | 0 | 0 | 0 | 0 | 0 | 0 | 0 | 0 | 0 | 0 | 0 | 0 | 0 | 0 | 0 | 0 | 0 | 1 | 0 | 0 | 0 | 0 | 0 | M81757 | 100 | 67 | 248 | 430 | 510 |
| 6441 | 07606 | 06440 | 1 | | 0 | 0 | 0 | 0 | 0 | 0 | 0 | 0 | 0 | 0 | 0 | 0 | 0 | 0 | 0 | 0 | 0 | 0 | 0 | 1 | 0 | 0 | 0 | 0 | 0 | | | | | | |
| 6442 | 07607 | 06441 | 1 | | 0 | 0 | 0 | 0 | 0 | 0 | 0 | 0 | 0 | 0 | 0 | 0 | 0 | 0 | 0 | 0 | 0 | 0 | 0 | 1 | 0 | 0 | 0 | 0 | 0 | | | | | | |
| 6443 | 07608 | 06442 | 1 | | 0 | 0 | 0 | 0 | 0 | 0 | 0 | 0 | 0 | 0 | 0 | 0 | 0 | 0 | 0 | 0 | 0 | 0 | 0 | 1 | 0 | 0 | 0 | 0 | 0 | | | | | | |
| 6444 | 07609 | 06443 | 1 | | 0 | 0 | 0 | 0 | 0 | 0 | 0 | 0 | 0 | 0 | 0 | 0 | 0 | 0 | 0 | 0 | 0 | 0 | 0 | 1 | 0 | 0 | 0 | 0 | 0 | | | | | | |
| 6445 | 07610 | 06444 | 1 | | 0 | 0 | 0 | 0 | 0 | 0 | 0 | 0 | 0 | 0 | 0 | 0 | 0 | 0 | 0 | 0 | 0 | 0 | 0 | 1 | 0 | 0 | 0 | 0 | 0 | | | | | | |
| 6446 | 07611 | 06445 | 1 | | 0 | 0 | 0 | 0 | 0 | 0 | 0 | 0 | 0 | 0 | 0 | 0 | 0 | 0 | 0 | 0 | 0 | 0 | 0 | 1 | 0 | 0 | 0 | 0 | 0 | | | | | | |
| 6447 | 07612 | 06446 | 1 | | 0 | 0 | 0 | 0 | 0 | 0 | 0 | 0 | 0 | 0 | 0 | 0 | 0 | 0 | 0 | 0 | 0 | 0 | 0 | 1 | 0 | 0 | 0 | 0 | 0 | | | | | | |
| 6448 | 07613 | 06447 | 2 | | 0 | 0 | 0 | 0 | 0 | 0 | 0 | 0 | 0 | 0 | 0 | 0 | 0 | 0 | 0 | 0 | 0 | 0 | 0 | 1 | 1 | 0 | 0 | 0 | 0 | | | | | | |
| 6449 | 07614 | 06448 | 3 | | 0 | 0 | 0 | 0 | 0 | 0 | 0 | 0 | 0 | 0 | 0 | 0 | 0 | 0 | 0 | 0 | 0 | 0 | 0 | 1 | 0 | 0 | 2 | 0 | 0 | | | | | | |
| 6450 | 07615 | 06449 | 1 | | 0 | 0 | 0 | 0 | 0 | 0 | 0 | 0 | 0 | 0 | 0 | 0 | 0 | 0 | 0 | 0 | 0 | 0 | 0 | 1 | 0 | 0 | 0 | 0 | 0 | | | | | | |
| 6451 | 07616 | 06450 | 1 | | 0 | 0 | 0 | 0 | 0 | 0 | 0 | 0 | 0 | 0 | 0 | 0 | 0 | 0 | 0 | 0 | 0 | 0 | 0 | 1 | 0 | 0 | 0 | 0 | 0 | | | | | | |
| 6452 | 07617 | 06451 | 1 | | 0 | 0 | 0 | 0 | 0 | 0 | 0 | 0 | 0 | 0 | 0 | 0 | 0 | 0 | 0 | 0 | 0 | 0 | 0 | 1 | 0 | 0 | 0 | 0 | 0 | | | | | | |
| 6453 | 07619 | 06452 | 1 | | 0 | 0 | 0 | 0 | 0 | 0 | 0 | 0 | 0 | 0 | 0 | 0 | 0 | 0 | 0 | 0 | 0 | 0 | 0 | 1 | 0 | 0 | 0 | 0 | 0 | | | | | | |
| 6454 | 07620 | 06453 | 2 | | 0 | 0 | 0 | 0 | 0 | 0 | 0 | 0 | 0 | 0 | 0 | 0 | 0 | 0 | 0 | 0 | 0 | 0 | 0 | 1 | 0 | 1 | 0 | 0 | 0 | | | | | | |
| 6455 | 07621 | 06454 | 1 | | 0 | 0 | 0 | 0 | 0 | 0 | 0 | 0 | 0 | 0 | 0 | 0 | 0 | 0 | 0 | 0 | 0 | 0 | 0 | 1 | 0 | 0 | 0 | 0 | 0 | | | | | | |
| 6456 | 07622 | 06455 | 1 | | 0 | 0 | 0 | 0 | 0 | 0 | 0 | 0 | 0 | 0 | 0 | 0 | 0 | 0 | 0 | 0 | 0 | 0 | 0 | 1 | 0 | 0 | 0 | 0 | 0 | | | | | | |
| 6457 | 07623 | 06456 | 1 | | 0 | 0 | 0 | 0 | 0 | 0 | 0 | 0 | 0 | 0 | 0 | 0 | 0 | 0 | 0 | 0 | 0 | 0 | 0 | 1 | 0 | 0 | 0 | 0 | 0 | | | | | | |
| 6458 | 07624 | 06457 | 1 | | 0 | 0 | 0 | 0 | 0 | 0 | 0 | 0 | 0 | 0 | 0 | 0 | 0 | 0 | 0 | 0 | 0 | 0 | 0 | 1 | 0 | 0 | 0 | 0 | 0 | | | | | | |
| 6459 | 07625 | 06458 | 1 | | 0 | 0 | 0 | 0 | 0 | 0 | 0 | 0 | 0 | 0 | 0 | 0 | 0 | 0 | 0 | 0 | 0 | 0 | 0 | 1 | 0 | 0 | 0 | 0 | 0 | | | | | | |
| 6460 | 07626 | 06459 | 1 | | 0 | 0 | 0 | 0 | 0 | 0 | 0 | 0 | 0 | 0 | 0 | 0 | 0 | 0 | 0 | 0 | 0 | 0 | 0 | 1 | 0 | 0 | 0 | 0 | 0 | | | | | | |
| 6461 | 07627 | 06460 | 1 | | 0 | 0 | 0 | 0 | 0 | 0 | 0 | 0 | 0 | 0 | 0 | 0 | 0 | 0 | 0 | 0 | 0 | 0 | 0 | 1 | 0 | 0 | 0 | 0 | 0 | U04847 | 90 | 100 | 1 | 1337 | 1857 |
| 6462 | 07628 | 06461 | 1 | | 0 | 0 | 0 | 0 | 0 | 0 | 0 | 0 | 0 | 0 | 0 | 0 | 0 | 0 | 0 | 0 | 0 | 0 | 0 | 1 | 0 | 0 | 0 | 0 | 0 | | | | | | |
| 6463 | 07629 | 06462 | 1 | | 0 | 0 | 0 | 0 | 0 | 0 | 0 | 0 | 0 | 0 | 0 | 0 | 0 | 0 | 0 | 0 | 0 | 0 | 0 | 1 | 0 | 0 | 0 | 0 | 0 | | | | | | |
| 6464 | 07630 | 06463 | 1 | | 0 | 0 | 0 | 0 | 0 | 0 | 0 | 0 | 0 | 0 | 0 | 0 | 0 | 0 | 0 | 0 | 0 | 0 | 0 | 1 | 0 | 0 | 0 | 0 | 0 | | | | | | |
| 6465 | 07631 | 06464 | 1 | | 0 | 0 | 0 | 0 | 0 | 0 | 0 | 0 | 0 | 0 | 0 | 0 | 0 | 0 | 0 | 0 | 0 | 0 | 0 | 1 | 0 | 0 | 0 | 0 | 0 | | | | | | |

Table 180

| | A | B | C | E | G | I | K | M | O | Q | S | U | W | Y | AA | AC | AE | AG | AI | AK | AM | AO | AQ | AS | AU | AW | AY | BA | BC | BE | BF | BG | BH | BI | BK |
|---|---|---|---|---|---|---|---|---|---|---|---|---|---|---|---|---|---|---|---|---|---|---|---|---|---|---|---|---|---|---|---|---|---|---|---|
| 6466 | 07632 | 06465 | 1 | | 0 | 0 | 0 | 0 | 0 | 0 | 0 | 0 | 0 | 0 | 0 | 0 | 0 | 0 | 0 | 0 | 0 | 0 | 0 | 1 | 0 | 0 | 0 | 0 | 0 | | | | | | |
| 6467 | 07633 | 06466 | 1 | | 0 | 0 | 0 | 0 | 0 | 0 | 0 | 0 | 0 | 0 | 0 | 0 | 0 | 0 | 0 | 0 | 0 | 0 | 0 | 1 | 0 | 0 | 0 | 0 | 0 | | | | | | |
| 6468 | 07634 | 06467 | 2 | | 0 | 0 | 0 | 0 | 0 | 0 | 0 | 0 | 0 | 0 | 0 | 0 | 0 | 0 | 0 | 0 | 0 | 0 | 0 | 1 | 0 | 0 | 0 | 1 | 0 | | | | | | |
| 6469 | 07635 | 06468 | 1 | | 0 | 0 | 0 | 0 | 0 | 0 | 0 | 0 | 0 | 0 | 0 | 0 | 0 | 0 | 0 | 0 | 0 | 0 | 0 | 1 | 0 | 0 | 0 | 0 | 0 | | | | | | |
| 6470 | 07636 | 06469 | 1 | | 0 | 0 | 0 | 0 | 0 | 0 | 0 | 0 | 0 | 0 | 0 | 0 | 0 | 0 | 0 | 0 | 0 | 0 | 0 | 1 | 0 | 0 | 0 | 0 | 0 | | | | | | |
| 6471 | 07637 | 06470 | 1 | | 0 | 0 | 0 | 0 | 0 | 0 | 0 | 0 | 0 | 0 | 0 | 0 | 0 | 0 | 0 | 0 | 0 | 0 | 0 | 1 | 0 | 0 | 0 | 0 | 0 | | | | | | |
| 6472 | 07638 | 06471 | 1 | | 0 | 0 | 0 | 0 | 0 | 0 | 0 | 0 | 0 | 0 | 0 | 0 | 0 | 0 | 0 | 0 | 0 | 0 | 0 | 1 | 0 | 0 | 0 | 0 | 0 | J04208 | 100 | 48 | 1 | 1598 | 1654 |
| 6473 | 07639 | 06472 | 1 | | 0 | 0 | 0 | 0 | 0 | 0 | 0 | 0 | 0 | 0 | 0 | 0 | 0 | 0 | 0 | 0 | 0 | 0 | 0 | 1 | 0 | 0 | 0 | 0 | 0 | | | | | | |
| 6474 | 07640 | 06473 | 1 | | 0 | 0 | 0 | 0 | 0 | 0 | 0 | 0 | 0 | 0 | 0 | 0 | 0 | 0 | 0 | 0 | 0 | 0 | 0 | 1 | 0 | 0 | 0 | 0 | 0 | | | | | | |
| 6475 | 07641 | 06474 | 2 | | 0 | 0 | 0 | 0 | 0 | 0 | 0 | 0 | 0 | 0 | 0 | 0 | 0 | 0 | 0 | 0 | 0 | 0 | 0 | 1 | 1 | 0 | 0 | 0 | 0 | | | | | | |
| 6476 | 07642 | 06475 | 1 | | 0 | 0 | 0 | 0 | 0 | 0 | 0 | 0 | 0 | 0 | 0 | 0 | 0 | 0 | 0 | 0 | 0 | 0 | 0 | 1 | 0 | 0 | 0 | 0 | 0 | | | | | | |
| 6477 | 07643 | 06476 | 1 | | 0 | 0 | 0 | 0 | 0 | 0 | 0 | 0 | 0 | 0 | 0 | 0 | 0 | 0 | 0 | 0 | 0 | 0 | 0 | 1 | 0 | 0 | 0 | 0 | 0 | | | | | | |
| 6478 | 07644 | 06477 | 1 | | 0 | 0 | 0 | 0 | 0 | 0 | 0 | 0 | 0 | 0 | 0 | 0 | 0 | 0 | 0 | 0 | 0 | 0 | 0 | 1 | 0 | 0 | 0 | 0 | 0 | | | | | | |
| 6479 | 07645 | 06478 | 1 | | 0 | 0 | 0 | 0 | 0 | 0 | 0 | 0 | 0 | 0 | 0 | 0 | 0 | 0 | 0 | 0 | 0 | 0 | 0 | 1 | 0 | 0 | 0 | 0 | 0 | | | | | | |
| 6480 | 07646 | 06479 | 1 | | 0 | 0 | 0 | 0 | 0 | 0 | 0 | 0 | 0 | 0 | 0 | 0 | 0 | 0 | 0 | 0 | 0 | 0 | 0 | 1 | 0 | 0 | 0 | 0 | 0 | | | | | | |
| 6481 | 07647 | 06480 | 1 | | 0 | 0 | 0 | 0 | 0 | 0 | 0 | 0 | 0 | 0 | 0 | 0 | 0 | 0 | 0 | 0 | 0 | 0 | 0 | 1 | 0 | 0 | 0 | 0 | 0 | D25248 | 95.5 | 157 | 1 | 4575 | 5109 |
| 6482 | 07648 | 06481 | 1 | | 0 | 0 | 0 | 0 | 0 | 0 | 0 | 0 | 0 | 0 | 0 | 0 | 0 | 0 | 0 | 0 | 0 | 0 | 0 | 1 | 0 | 0 | 0 | 0 | 0 | X69391 | 91.3 | 69 | 19 | 858 | 926 |
| 6483 | 07649 | 06482 | 1 | | 0 | 0 | 0 | 0 | 0 | 0 | 0 | 0 | 0 | 0 | 0 | 0 | 0 | 0 | 0 | 0 | 0 | 0 | 0 | 1 | 0 | 0 | 0 | 0 | 0 | | | | | | |
| 6484 | 07650 | 06483 | 1 | | 0 | 0 | 0 | 0 | 0 | 0 | 0 | 0 | 0 | 0 | 0 | 0 | 0 | 0 | 0 | 0 | 0 | 0 | 0 | 1 | 0 | 0 | 0 | 0 | 0 | | | | | | |
| 6485 | 07651 | 06484 | 1 | | 0 | 0 | 0 | 0 | 0 | 0 | 0 | 0 | 0 | 0 | 0 | 0 | 0 | 0 | 0 | 0 | 0 | 0 | 0 | 1 | 0 | 0 | 0 | 0 | 0 | | | | | | |
| 6486 | 07652 | 06485 | 1 | | 0 | 0 | 0 | 0 | 0 | 0 | 0 | 0 | 0 | 0 | 0 | 0 | 0 | 0 | 0 | 0 | 0 | 0 | 0 | 1 | 0 | 0 | 0 | 0 | 0 | | | | | | |
| 6487 | 07653 | 06486 | 1 | | 0 | 0 | 0 | 0 | 0 | 0 | 0 | 0 | 0 | 0 | 0 | 0 | 0 | 0 | 0 | 0 | 0 | 0 | 0 | 1 | 0 | 0 | 0 | 0 | 0 | | | | | | |
| 6488 | 07655 | 06487 | 1 | | 0 | 0 | 0 | 0 | 0 | 0 | 0 | 0 | 0 | 0 | 0 | 0 | 0 | 0 | 0 | 0 | 0 | 0 | 0 | 1 | 0 | 0 | 0 | 0 | 0 | | | | | | |
| 6489 | 07656 | 06488 | 3 | | 0 | 0 | 0 | 0 | 0 | 0 | 0 | 0 | 0 | 0 | 0 | 0 | 0 | 0 | 0 | 0 | 0 | 0 | 0 | 2 | 0 | 0 | 0 | 1 | 0 | | | | | | |
| 6490 | 07657 | 06489 | 1 | | 0 | 0 | 0 | 0 | 0 | 0 | 0 | 0 | 0 | 0 | 0 | 0 | 0 | 0 | 0 | 0 | 0 | 0 | 0 | 1 | 0 | 0 | 0 | 0 | 0 | | | | | | |
| 6491 | 07658 | 06490 | 1 | | 0 | 0 | 0 | 0 | 0 | 0 | 0 | 0 | 0 | 0 | 0 | 0 | 0 | 0 | 0 | 0 | 0 | 0 | 0 | 1 | 0 | 0 | 0 | 0 | 0 | | | | | | |
| 6492 | 07659 | 06491 | 1 | | 0 | 0 | 0 | 0 | 0 | 0 | 0 | 0 | 0 | 0 | 0 | 0 | 0 | 0 | 0 | 0 | 0 | 0 | 0 | 1 | 0 | 0 | 0 | 0 | 0 | | | | | | |
| 6493 | 07660 | 06492 | 1 | | 0 | 0 | 0 | 0 | 0 | 0 | 0 | 0 | 0 | 0 | 0 | 0 | 0 | 0 | 0 | 0 | 0 | 0 | 0 | 1 | 0 | 0 | 0 | 0 | 0 | L02950 | 99.2 | 119 | 1 | 974 | 1100 |
| 6494 | 07661 | 06493 | 1 | | 0 | 0 | 0 | 0 | 0 | 0 | 0 | 0 | 0 | 0 | 0 | 0 | 0 | 0 | 0 | 0 | 0 | 0 | 0 | 1 | 0 | 0 | 0 | 0 | 0 | | | | | | |
| 6495 | 07663 | 06494 | 1 | | 0 | 0 | 0 | 0 | 0 | 0 | 0 | 0 | 0 | 0 | 0 | 0 | 0 | 0 | 0 | 0 | 0 | 0 | 0 | 1 | 0 | 0 | 0 | 0 | 0 | U00968 | 97.2 | 327 | 1 | 3828 | 4154 |
| 6496 | 07664 | 06495 | 1 | | 0 | 0 | 0 | 0 | 0 | 0 | 0 | 0 | 0 | 0 | 0 | 0 | 0 | 0 | 0 | 0 | 0 | 0 | 0 | 1 | 0 | 0 | 0 | 0 | 0 | | | | | | |
| 6497 | 07665 | 06496 | 1 | | 0 | 0 | 0 | 0 | 0 | 0 | 0 | 0 | 0 | 0 | 0 | 0 | 0 | 0 | 0 | 0 | 0 | 0 | 0 | 1 | 0 | 0 | 0 | 0 | 0 | | | | | | |
| 6498 | 07666 | 06497 | 1 | | 0 | 0 | 0 | 0 | 0 | 0 | 0 | 0 | 0 | 0 | 0 | 0 | 0 | 0 | 0 | 0 | 0 | 0 | 0 | 1 | 0 | 0 | 0 | 0 | 0 | | | | | | |
| 6499 | 07667 | 06498 | 4 | | 0 | 0 | 0 | 0 | 0 | 0 | 0 | 0 | 0 | 0 | 0 | 0 | 0 | 0 | 0 | 0 | 0 | 0 | 0 | 1 | 0 | 1 | 2 | 0 | 0 | | | | | | |
| 6500 | 07668 | 06499 | 1 | | 0 | 0 | 0 | 0 | 0 | 0 | 0 | 0 | 0 | 0 | 0 | 0 | 0 | 0 | 0 | 0 | 0 | 0 | 0 | 1 | 0 | 0 | 0 | 0 | 0 | | | | | | |
| 6501 | 07669 | 06500 | 1 | | 0 | 0 | 0 | 0 | 0 | 0 | 0 | 0 | 0 | 0 | 0 | 0 | 0 | 0 | 0 | 0 | 0 | 0 | 0 | 1 | 0 | 0 | 0 | 0 | 0 | | | | | | |

Table 181

| | A | B | C | E | G | I | K | M | O | Q | S | U | W | Y | AA | AC | AE | AG | AI | AK | AM | AO | AQ | AS | AU | AW | AY | BA | BC | BE | BF | BG | BH | BI | BK |
|---|---|---|---|---|---|---|---|---|---|---|---|---|---|---|---|---|---|---|---|---|---|---|---|---|---|---|---|---|---|---|---|---|---|---|---|
| 6502 | 07670 | 06501 | 1 | | 0 | 0 | 0 | 0 | 0 | 0 | 0 | 0 | 0 | 0 | 0 | 0 | 0 | 0 | 0 | 0 | 0 | 0 | 0 | 1 | 0 | 0 | 0 | 0 | 0 | | | | | | |
| 6503 | 07671 | 06502 | 1 | | 0 | 0 | 0 | 0 | 0 | 0 | 0 | 0 | 0 | 0 | 0 | 0 | 0 | 0 | 0 | 0 | 0 | 0 | 0 | 1 | 0 | 0 | 0 | 0 | 0 | | | | | | |
| 6504 | 07672 | 06503 | 1 | | 0 | 0 | 0 | 0 | 0 | 0 | 0 | 0 | 0 | 0 | 0 | 0 | 0 | 0 | 0 | 0 | 0 | 0 | 0 | 1 | 0 | 0 | 0 | 0 | 0 | | | | | | |
| 6505 | 07673 | 06504 | 2 | | 0 | 0 | 0 | 0 | 0 | 0 | 0 | 0 | 0 | 0 | 0 | 0 | 0 | 0 | 0 | 0 | 0 | 0 | 0 | 2 | 0 | 0 | 0 | 0 | 0 | | | | | | |
| 6506 | 07674 | 06505 | 1 | | 0 | 0 | 0 | 0 | 0 | 0 | 0 | 0 | 0 | 0 | 0 | 0 | 0 | 0 | 0 | 0 | 0 | 0 | 0 | 1 | 0 | 0 | 0 | 0 | 0 | | | | | | |
| 6507 | 07675 | 06506 | 1 | | 0 | 0 | 0 | 0 | 0 | 0 | 0 | 0 | 0 | 0 | 0 | 0 | 0 | 0 | 0 | 0 | 0 | 0 | 0 | 1 | 0 | 0 | 0 | 0 | 0 | | | | | | |
| 6508 | 07676 | 06507 | 1 | | 0 | 0 | 0 | 0 | 0 | 0 | 0 | 0 | 0 | 0 | 0 | 0 | 0 | 0 | 0 | 0 | 0 | 0 | 0 | 1 | 0 | 0 | 0 | 0 | 0 | | | | | | |
| 6509 | 07677 | 06508 | 1 | | 0 | 0 | 0 | 0 | 0 | 0 | 0 | 0 | 0 | 0 | 0 | 0 | 0 | 0 | 0 | 0 | 0 | 0 | 0 | 1 | 0 | 0 | 0 | 0 | 0 | | | | | | |
| 6510 | 07678 | 06509 | 1 | | 0 | 0 | 0 | 0 | 0 | 0 | 0 | 0 | 0 | 0 | 0 | 0 | 0 | 0 | 0 | 0 | 0 | 0 | 0 | 1 | 0 | 0 | 0 | 0 | 0 | | | | | | |
| 6511 | 07679 | 06510 | 4 | | 0 | 0 | 0 | 0 | 0 | 0 | 0 | 0 | 0 | 0 | 0 | 0 | 0 | 0 | 0 | 0 | 0 | 0 | 0 | 1 | 0 | 0 | 3 | 0 | 0 | | | | | | |
| 6512 | 07680 | 06511 | 1 | | 0 | 0 | 0 | 0 | 0 | 0 | 0 | 0 | 0 | 0 | 0 | 0 | 0 | 0 | 0 | 0 | 0 | 0 | 0 | 1 | 0 | 0 | 0 | 0 | 0 | | | | | | |
| 6513 | 07681 | 06512 | 1 | | 0 | 0 | 0 | 0 | 0 | 0 | 0 | 0 | 0 | 0 | 0 | 0 | 0 | 0 | 0 | 0 | 0 | 0 | 0 | 1 | 0 | 0 | 0 | 0 | 0 | | | | | | |
| 6514 | 07682 | 06513 | 1 | | 0 | 0 | 0 | 0 | 0 | 0 | 0 | 0 | 0 | 0 | 0 | 0 | 0 | 0 | 0 | 0 | 0 | 0 | 0 | 1 | 0 | 0 | 0 | 0 | 0 | | | | | | |
| 6515 | 07683 | 06514 | 1 | | 0 | 0 | 0 | 0 | 0 | 0 | 0 | 0 | 0 | 0 | 0 | 0 | 0 | 0 | 0 | 0 | 0 | 0 | 0 | 1 | 0 | 0 | 0 | 0 | 0 | | | | | | |
| 6516 | 07684 | 06515 | 1 | | 0 | 0 | 0 | 0 | 0 | 0 | 0 | 0 | 0 | 0 | 0 | 0 | 0 | 0 | 0 | 0 | 0 | 0 | 0 | 1 | 0 | 0 | 0 | 0 | 0 | | | | | | |
| 6517 | 07686 | 06516 | 1 | | 0 | 0 | 0 | 0 | 0 | 0 | 0 | 0 | 0 | 0 | 0 | 0 | 0 | 0 | 0 | 0 | 0 | 0 | 0 | 1 | 0 | 0 | 0 | 0 | 0 | | | | | | |
| 6518 | 07687 | 06517 | 1 | | 0 | 0 | 0 | 0 | 0 | 0 | 0 | 0 | 0 | 0 | 0 | 0 | 0 | 0 | 0 | 0 | 0 | 0 | 0 | 1 | 0 | 0 | 0 | 0 | 0 | | | | | | |
| 6519 | 07688 | 06518 | 1 | | 0 | 0 | 0 | 0 | 0 | 0 | 0 | 0 | 0 | 0 | 0 | 0 | 0 | 0 | 0 | 0 | 0 | 0 | 0 | 1 | 0 | 0 | 0 | 0 | 0 | | | | | | |
| 6520 | 07689 | 06519 | 1 | | 0 | 0 | 0 | 0 | 0 | 0 | 0 | 0 | 0 | 0 | 0 | 0 | 0 | 0 | 0 | 0 | 0 | 0 | 0 | 1 | 0 | 0 | 0 | 0 | 0 | | | | | | |
| 6521 | 07690 | 06520 | 1 | | 0 | 0 | 0 | 0 | 0 | 0 | 0 | 0 | 0 | 0 | 0 | 0 | 0 | 0 | 0 | 0 | 0 | 0 | 0 | 1 | 0 | 0 | 0 | 0 | 0 | | | | | | |
| 6522 | 07691 | 06521 | 1 | | 0 | 0 | 0 | 0 | 0 | 0 | 0 | 0 | 0 | 0 | 0 | 0 | 0 | 0 | 0 | 0 | 0 | 0 | 0 | 1 | 0 | 0 | 0 | 0 | 0 | | | | | | |
| 6523 | 07692 | 06522 | 1 | | 0 | 0 | 0 | 0 | 0 | 0 | 0 | 0 | 0 | 0 | 0 | 0 | 0 | 0 | 0 | 0 | 0 | 0 | 0 | 1 | 0 | 0 | 0 | 0 | 0 | | | | | | |
| 6524 | 07693 | 06523 | 1 | | 0 | 0 | 0 | 0 | 0 | 0 | 0 | 0 | 0 | 0 | 0 | 0 | 0 | 0 | 0 | 0 | 0 | 0 | 0 | 1 | 0 | 0 | 0 | 0 | 0 | | | | | | |
| 6525 | 07694 | 06524 | 2 | | 0 | 0 | 0 | 0 | 0 | 0 | 0 | 0 | 0 | 0 | 0 | 0 | 0 | 0 | 0 | 0 | 0 | 0 | 0 | 1 | 1 | 0 | 0 | 0 | 0 | | | | | | |
| 6526 | 07695 | 06525 | 1 | | 0 | 0 | 0 | 0 | 0 | 0 | 0 | 0 | 0 | 0 | 0 | 0 | 0 | 0 | 0 | 0 | 0 | 0 | 0 | 1 | 0 | 0 | 0 | 0 | 0 | | | | | | |
| 6527 | 07696 | 06526 | 1 | | 0 | 0 | 0 | 0 | 0 | 0 | 0 | 0 | 0 | 0 | 0 | 0 | 0 | 0 | 0 | 0 | 0 | 0 | 0 | 1 | 0 | 0 | 0 | 0 | 0 | | | | | | |
| 6528 | 07697 | 06527 | 1 | | 0 | 0 | 0 | 0 | 0 | 0 | 0 | 0 | 0 | 0 | 0 | 0 | 0 | 0 | 0 | 0 | 0 | 0 | 0 | 1 | 0 | 0 | 0 | 0 | 0 | L20431 | 94.8 | 135 | 1 | 5392 | 5672 |
| 6529 | 07698 | 06528 | 1 | | 0 | 0 | 0 | 0 | 0 | 0 | 0 | 0 | 0 | 0 | 0 | 0 | 0 | 0 | 0 | 0 | 0 | 0 | 0 | 1 | 0 | 0 | 0 | 0 | 0 | | | | | | |
| 6530 | 07699 | 06529 | 1 | | 0 | 0 | 0 | 0 | 0 | 0 | 0 | 0 | 0 | 0 | 0 | 0 | 0 | 0 | 0 | 0 | 0 | 0 | 0 | 1 | 0 | 0 | 0 | 0 | 0 | | | | | | |
| 6531 | 07700 | 06530 | 1 | | 0 | 0 | 0 | 0 | 0 | 0 | 0 | 0 | 0 | 0 | 0 | 0 | 0 | 0 | 0 | 0 | 0 | 0 | 0 | 1 | 0 | 0 | 0 | 0 | 0 | | | | | | |
| 6532 | 07701 | 06531 | 1 | | 0 | 0 | 0 | 0 | 0 | 0 | 0 | 0 | 0 | 0 | 0 | 0 | 0 | 0 | 0 | 0 | 0 | 0 | 0 | 1 | 0 | 0 | 0 | 0 | 0 | | | | | | |
| 6533 | 07702 | 06532 | 1 | | 0 | 0 | 0 | 0 | 0 | 0 | 0 | 0 | 0 | 0 | 0 | 0 | 0 | 0 | 0 | 0 | 0 | 0 | 0 | 1 | 0 | 0 | 0 | 0 | 0 | Y00064 | 95.4 | 261 | 1 | 2191 | 2454 |
| 6534 | 07703 | 06533 | 1 | | 0 | 0 | 0 | 0 | 0 | 0 | 0 | 0 | 0 | 0 | 0 | 0 | 0 | 0 | 0 | 0 | 0 | 0 | 0 | 0 | 1 | 0 | 0 | 0 | 0 | | | | | | |
| 6535 | 07704 | 06534 | 2 | | 0 | 0 | 0 | 0 | 0 | 0 | 0 | 0 | 0 | 0 | 0 | 0 | 0 | 0 | 0 | 0 | 0 | 0 | 0 | 0 | 2 | 0 | 0 | 0 | 0 | | | | | | |
| 6536 | 07705 | 06535 | 1 | | 0 | 0 | 0 | 0 | 0 | 0 | 0 | 0 | 0 | 0 | 0 | 0 | 0 | 0 | 0 | 0 | 0 | 0 | 0 | 0 | 1 | 0 | 0 | 0 | 0 | | | | | | |
| 6537 | 07708 | 06536 | 1 | | 0 | 0 | 0 | 0 | 0 | 0 | 0 | 0 | 0 | 0 | 0 | 0 | 0 | 0 | 0 | 0 | 0 | 0 | 0 | 0 | 1 | 0 | 0 | 0 | 0 | | | | | | |

Table 182

| | A | B | C | E | G | I | K | M | O | Q | S | U | W | Y | AA | AC | AE | AG | AI | AK | AM | AO | AQ | AS | AU | AW | AY | BA | BC | BE | BF | BG | BH | BI | BK |
|---|---|---|---|---|---|---|---|---|---|---|---|---|---|---|---|---|---|---|---|---|---|---|---|---|---|---|---|---|---|---|---|---|---|---|---|
| 6538 | 07709 | 06537 | 1 | | 0 | 0 | 0 | 0 | 0 | 0 | 0 | 0 | 0 | 0 | 0 | 0 | 0 | 0 | 0 | 0 | 0 | 0 | 0 | 0 | 1 | 0 | 0 | 0 | 0 | | | | | | |
| 6539 | 07710 | 06538 | 1 | | 0 | 0 | 0 | 0 | 0 | 0 | 0 | 0 | 0 | 0 | 0 | 0 | 0 | 0 | 0 | 0 | 0 | 0 | 0 | 0 | 1 | 0 | 0 | 0 | 0 | | | | | | |
| 6540 | 07711 | 06539 | 2 | | 0 | 0 | 0 | 0 | 0 | 0 | 0 | 0 | 0 | 0 | 0 | 0 | 0 | 0 | 0 | 0 | 0 | 0 | 0 | 0 | 1 | 0 | 0 | 1 | 0 | | | | | | |
| 6541 | 07712 | 06540 | 1 | | 0 | 0 | 0 | 0 | 0 | 0 | 0 | 0 | 0 | 0 | 0 | 0 | 0 | 0 | 0 | 0 | 0 | 0 | 0 | 0 | 1 | 0 | 0 | 0 | 0 | | | | | | |
| 6542 | 07713 | 06541 | 1 | | 0 | 0 | 0 | 0 | 0 | 0 | 0 | 0 | 0 | 0 | 0 | 0 | 0 | 0 | 0 | 0 | 0 | 0 | 0 | 0 | 1 | 0 | 0 | 0 | 0 | | | | | | |
| 6543 | 07714 | 06542 | 1 | | 0 | 0 | 0 | 0 | 0 | 0 | 0 | 0 | 0 | 0 | 0 | 0 | 0 | 0 | 0 | 0 | 0 | 0 | 0 | 0 | 1 | 0 | 0 | 0 | 0 | | | | | | |
| 6544 | 07715 | 06543 | 1 | | 0 | 0 | 0 | 0 | 0 | 0 | 0 | 0 | 0 | 0 | 0 | 0 | 0 | 0 | 0 | 0 | 0 | 0 | 0 | 0 | 1 | 0 | 0 | 0 | 0 | | | | | | |
| 6545 | 07716 | 06544 | 1 | | 0 | 0 | 0 | 0 | 0 | 0 | 0 | 0 | 0 | 0 | 0 | 0 | 0 | 0 | 0 | 0 | 0 | 0 | 0 | 0 | 1 | 0 | 0 | 0 | 0 | | | | | | |
| 6546 | 07717 | 06545 | 2 | | 0 | 0 | 0 | 0 | 0 | 0 | 0 | 0 | 0 | 0 | 0 | 0 | 0 | 0 | 0 | 0 | 0 | 0 | 0 | 0 | 2 | 0 | 0 | 0 | 0 | | | | | | |
| 6547 | 07718 | 06546 | 2 | | 0 | 0 | 0 | 0 | 0 | 0 | 0 | 0 | 0 | 0 | 0 | 0 | 0 | 0 | 0 | 0 | 0 | 0 | 0 | 0 | 1 | 1 | 0 | 0 | 0 | M75715 | 92.6 | 229 | 1 | 1762 | 2261 |
| 6548 | 07719 | 06547 | 7 | | 0 | 0 | 0 | 0 | 0 | 0 | 0 | 0 | 0 | 0 | 0 | 0 | 0 | 0 | 0 | 0 | 0 | 0 | 0 | 0 | 7 | 0 | 0 | 0 | 0 | X14829 | 96.2 | 79 | 1 | 391 | 472 |
| 6549 | 07720 | 06548 | 1 | | 0 | 0 | 0 | 0 | 0 | 0 | 0 | 0 | 0 | 0 | 0 | 0 | 0 | 0 | 0 | 0 | 0 | 0 | 0 | 0 | 1 | 0 | 0 | 0 | 0 | | | | | | |
| 6550 | 07721 | 06549 | 1 | | 0 | 0 | 0 | 0 | 0 | 0 | 0 | 0 | 0 | 0 | 0 | 0 | 0 | 0 | 0 | 0 | 0 | 0 | 0 | 0 | 1 | 0 | 0 | 0 | 0 | | | | | | |
| 6551 | 07722 | 06550 | 1 | | 0 | 0 | 0 | 0 | 0 | 0 | 0 | 0 | 0 | 0 | 0 | 0 | 0 | 0 | 0 | 0 | 0 | 0 | 0 | 0 | 1 | 0 | 0 | 0 | 0 | | | | | | |
| 6552 | 07723 | 06551 | 1 | | 0 | 0 | 0 | 0 | 0 | 0 | 0 | 0 | 0 | 0 | 0 | 0 | 0 | 0 | 0 | 0 | 0 | 0 | 0 | 0 | 1 | 0 | 0 | 0 | 0 | | | | | | |
| 6553 | 07724 | 06552 | 1 | | 0 | 0 | 0 | 0 | 0 | 0 | 0 | 0 | 0 | 0 | 0 | 0 | 0 | 0 | 0 | 0 | 0 | 0 | 0 | 0 | 1 | 0 | 0 | 0 | 0 | | | | | | |
| 6554 | 07725 | 06553 | 1 | | 0 | 0 | 0 | 0 | 0 | 0 | 0 | 0 | 0 | 0 | 0 | 0 | 0 | 0 | 0 | 0 | 0 | 0 | 0 | 0 | 1 | 0 | 0 | 0 | 0 | | | | | | |
| 6555 | 07726 | 06554 | 2 | | 0 | 0 | 0 | 0 | 0 | 0 | 0 | 0 | 0 | 0 | 0 | 0 | 0 | 0 | 0 | 0 | 0 | 0 | 0 | 0 | 2 | 0 | 0 | 0 | 0 | | | | | | |
| 6556 | 07727 | 06555 | 1 | | 0 | 0 | 0 | 0 | 0 | 0 | 0 | 0 | 0 | 0 | 0 | 0 | 0 | 0 | 0 | 0 | 0 | 0 | 0 | 0 | 1 | 0 | 0 | 0 | 0 | | | | | | |
| 6557 | 07729 | 06556 | 1 | | 0 | 0 | 0 | 0 | 0 | 0 | 0 | 0 | 0 | 0 | 0 | 0 | 0 | 0 | 0 | 0 | 0 | 0 | 0 | 0 | 1 | 0 | 0 | 0 | 0 | | | | | | |
| 6558 | 07731 | 06557 | 1 | | 0 | 0 | 0 | 0 | 0 | 0 | 0 | 0 | 0 | 0 | 0 | 0 | 0 | 0 | 0 | 0 | 0 | 0 | 0 | 0 | 1 | 0 | 0 | 0 | 0 | | | | | | |
| 6559 | 07734 | 06558 | 1 | | 0 | 0 | 0 | 0 | 0 | 0 | 0 | 0 | 0 | 0 | 0 | 0 | 0 | 0 | 0 | 0 | 0 | 0 | 0 | 0 | 1 | 0 | 0 | 0 | 0 | | | | | | |
| 6560 | 07735 | 06559 | 1 | | 0 | 0 | 0 | 0 | 0 | 0 | 0 | 0 | 0 | 0 | 0 | 0 | 0 | 0 | 0 | 0 | 0 | 0 | 0 | 0 | 1 | 0 | 0 | 0 | 0 | | | | | | |
| 6561 | 07736 | 06560 | 1 | | 0 | 0 | 0 | 0 | 0 | 0 | 0 | 0 | 0 | 0 | 0 | 0 | 0 | 0 | 0 | 0 | 0 | 0 | 0 | 0 | 1 | 0 | 0 | 0 | 0 | | | | | | |
| 6562 | 07738 | 06561 | 1 | | 0 | 0 | 0 | 0 | 0 | 0 | 0 | 0 | 0 | 0 | 0 | 0 | 0 | 0 | 0 | 0 | 0 | 0 | 0 | 0 | 1 | 0 | 0 | 0 | 0 | | | | | | |
| 6563 | 07739 | 06562 | 1 | | 0 | 0 | 0 | 0 | 0 | 0 | 0 | 0 | 0 | 0 | 0 | 0 | 0 | 0 | 0 | 0 | 0 | 0 | 0 | 0 | 1 | 0 | 0 | 0 | 0 | | | | | | |
| 6564 | 07740 | 06563 | 3 | | 0 | 0 | 0 | 0 | 0 | 0 | 0 | 0 | 0 | 0 | 0 | 0 | 0 | 0 | 0 | 0 | 0 | 0 | 0 | 0 | 3 | 0 | 0 | 0 | 0 | | | | | | |
| 6565 | 07741 | 06564 | 1 | | 0 | 0 | 0 | 0 | 0 | 0 | 0 | 0 | 0 | 0 | 0 | 0 | 0 | 0 | 0 | 0 | 0 | 0 | 0 | 0 | 1 | 0 | 0 | 0 | 0 | | | | | | |
| 6566 | 07742 | 06565 | 1 | | 0 | 0 | 0 | 0 | 0 | 0 | 0 | 0 | 0 | 0 | 0 | 0 | 0 | 0 | 0 | 0 | 0 | 0 | 0 | 0 | 1 | 0 | 0 | 0 | 0 | | | | | | |
| 6567 | 07743 | 06566 | 2 | | 0 | 0 | 0 | 0 | 0 | 0 | 0 | 0 | 0 | 0 | 0 | 0 | 0 | 0 | 0 | 0 | 0 | 0 | 0 | 0 | 1 | 0 | 0 | 0 | 1 | | | | | | |
| 6568 | 07744 | 06567 | 1 | | 0 | 0 | 0 | 0 | 0 | 0 | 0 | 0 | 0 | 0 | 0 | 0 | 0 | 0 | 0 | 0 | 0 | 0 | 0 | 0 | 1 | 0 | 0 | 0 | 0 | | | | | | |
| 6569 | 07745 | 06568 | 1 | | 0 | 0 | 0 | 0 | 0 | 0 | 0 | 0 | 0 | 0 | 0 | 0 | 0 | 0 | 0 | 0 | 0 | 0 | 0 | 0 | 1 | 0 | 0 | 0 | 0 | | | | | | |
| 6570 | 07746 | 06569 | 1 | | 0 | 0 | 0 | 0 | 0 | 0 | 0 | 0 | 0 | 0 | 0 | 0 | 0 | 0 | 0 | 0 | 0 | 0 | 0 | 0 | 1 | 0 | 0 | 0 | 0 | M14016 | 100 | 34 | 1 | 1164 | 1197 |
| 6571 | 07748 | 06570 | 1 | | 0 | 0 | 0 | 0 | 0 | 0 | 0 | 0 | 0 | 0 | 0 | 0 | 0 | 0 | 0 | 0 | 0 | 0 | 0 | 0 | 1 | 0 | 0 | 0 | 0 | | | | | | |
| 6572 | 07749 | 06571 | 2 | | 0 | 0 | 0 | 0 | 0 | 0 | 0 | 0 | 0 | 0 | 0 | 0 | 0 | 0 | 0 | 0 | 0 | 0 | 0 | 0 | 1 | 0 | 0 | 1 | 0 | | | | | | |
| 6573 | 07750 | 06572 | 1 | | 0 | 0 | 0 | 0 | 0 | 0 | 0 | 0 | 0 | 0 | 0 | 0 | 0 | 0 | 0 | 0 | 0 | 0 | 0 | 0 | 1 | 0 | 0 | 0 | 0 | | | | | | |

Table 183

| | A | B | C | E | G | I | K | M | O | Q | S | U | W | Y | AA | AC | AE | AG | AI | AK | AM | AO | AQ | AS | AU | AW | AY | BA | BC | BE | BF | BG | BH | BI | BK |
|---|---|---|---|---|---|---|---|---|---|---|---|---|---|---|---|---|---|---|---|---|---|---|---|---|---|---|---|---|---|---|---|---|---|---|---|
| 6574 | 07751 | 06573 | 1 | | 0 | 0 | 0 | 0 | 0 | 0 | 0 | 0 | 0 | 0 | 0 | 0 | 0 | 0 | 0 | 0 | 0 | 0 | 0 | 0 | 1 | 0 | 0 | 0 | 0 | | | | | | |
| 6575 | 07752 | 06574 | 1 | | 0 | 0 | 0 | 0 | 0 | 0 | 0 | 0 | 0 | 0 | 0 | 0 | 0 | 0 | 0 | 0 | 0 | 0 | 0 | 0 | 1 | 0 | 0 | 0 | 0 | | | | | | |
| 6576 | 07753 | 06575 | 1 | | 0 | 0 | 0 | 0 | 0 | 0 | 0 | 0 | 0 | 0 | 0 | 0 | 0 | 0 | 0 | 0 | 0 | 0 | 0 | 0 | 1 | 0 | 0 | 0 | 0 | | | | | | |
| 6577 | 07754 | 06576 | 1 | | 0 | 0 | 0 | 0 | 0 | 0 | 0 | 0 | 0 | 0 | 0 | 0 | 0 | 0 | 0 | 0 | 0 | 0 | 0 | 0 | 1 | 0 | 0 | 0 | 0 | | | | | | |
| 6578 | 07755 | 06577 | 2 | | 0 | 0 | 0 | 0 | 0 | 0 | 0 | 0 | 0 | 0 | 0 | 0 | 0 | 0 | 0 | 0 | 0 | 0 | 0 | 0 | 1 | 0 | 0 | 1 | 0 | | | | | | |
| 6579 | 07756 | 06578 | 2 | | 0 | 0 | 0 | 0 | 0 | 0 | 0 | 0 | 0 | 0 | 0 | 0 | 0 | 0 | 0 | 0 | 0 | 0 | 0 | 0 | 1 | 0 | 0 | 1 | 0 | | | | | | |
| 6580 | 07757 | 06579 | 1 | | 0 | 0 | 0 | 0 | 0 | 0 | 0 | 0 | 0 | 0 | 0 | 0 | 0 | 0 | 0 | 0 | 0 | 0 | 0 | 0 | 1 | 0 | 0 | 0 | 0 | | | | | | |
| 6581 | 07758 | 06580 | 1 | | 0 | 0 | 0 | 0 | 0 | 0 | 0 | 0 | 0 | 0 | 0 | 0 | 0 | 0 | 0 | 0 | 0 | 0 | 0 | 0 | 1 | 0 | 0 | 0 | 0 | | | | | | |
| 6582 | 07759 | 06581 | 1 | | 0 | 0 | 0 | 0 | 0 | 0 | 0 | 0 | 0 | 0 | 0 | 0 | 0 | 0 | 0 | 0 | 0 | 0 | 0 | 0 | 1 | 0 | 0 | 0 | 0 | | | | | | |
| 6583 | 07760 | 06582 | 1 | | 0 | 0 | 0 | 0 | 0 | 0 | 0 | 0 | 0 | 0 | 0 | 0 | 0 | 0 | 0 | 0 | 0 | 0 | 0 | 0 | 1 | 0 | 0 | 0 | 0 | | | | | | |
| 6584 | 07761 | 06583 | 1 | | 0 | 0 | 0 | 0 | 0 | 0 | 0 | 0 | 0 | 0 | 0 | 0 | 0 | 0 | 0 | 0 | 0 | 0 | 0 | 0 | 1 | 0 | 0 | 0 | 0 | | | | | | |
| 6585 | 07762 | 06584 | 1 | | 0 | 0 | 0 | 0 | 0 | 0 | 0 | 0 | 0 | 0 | 0 | 0 | 0 | 0 | 0 | 0 | 0 | 0 | 0 | 0 | 1 | 0 | 0 | 0 | 0 | | | | | | |
| 6586 | 07763 | 06585 | 1 | | 0 | 0 | 0 | 0 | 0 | 0 | 0 | 0 | 0 | 0 | 0 | 0 | 0 | 0 | 0 | 0 | 0 | 0 | 0 | 0 | 1 | 0 | 0 | 0 | 0 | | | | | | |
| 6587 | 07764 | 06586 | 1 | | 0 | 0 | 0 | 0 | 0 | 0 | 0 | 0 | 0 | 0 | 0 | 0 | 0 | 0 | 0 | 0 | 0 | 0 | 0 | 0 | 1 | 0 | 0 | 0 | 0 | | | | | | |
| 6588 | 07765 | 06587 | 3 | | 0 | 0 | 0 | 0 | 0 | 0 | 0 | 0 | 0 | 0 | 0 | 0 | 0 | 0 | 0 | 0 | 0 | 0 | 0 | 0 | 1 | 2 | 0 | 0 | 0 | | | | | | |
| 6589 | 07766 | 06588 | 1 | | 0 | 0 | 0 | 0 | 0 | 0 | 0 | 0 | 0 | 0 | 0 | 0 | 0 | 0 | 0 | 0 | 0 | 0 | 0 | 0 | 1 | 0 | 0 | 0 | 0 | | | | | | |
| 6590 | 07768 | 06589 | 1 | | 0 | 0 | 0 | 0 | 0 | 0 | 0 | 0 | 0 | 0 | 0 | 0 | 0 | 0 | 0 | 0 | 0 | 0 | 0 | 0 | 1 | 0 | 0 | 0 | 0 | | | | | | |
| 6591 | 07769 | 06590 | 1 | | 0 | 0 | 0 | 0 | 0 | 0 | 0 | 0 | 0 | 0 | 0 | 0 | 0 | 0 | 0 | 0 | 0 | 0 | 0 | 0 | 1 | 0 | 0 | 0 | 0 | | | | | | |
| 6592 | 07770 | 06591 | 1 | | 0 | 0 | 0 | 0 | 0 | 0 | 0 | 0 | 0 | 0 | 0 | 0 | 0 | 0 | 0 | 0 | 0 | 0 | 0 | 0 | 1 | 0 | 0 | 0 | 0 | | | | | | |
| 6593 | 07771 | 06592 | 2 | | 0 | 0 | 0 | 0 | 0 | 0 | 0 | 0 | 0 | 0 | 0 | 0 | 0 | 0 | 0 | 0 | 0 | 0 | 0 | 0 | 1 | 0 | 0 | 1 | 0 | | | | | | |
| 6594 | 07773 | 06593 | 1 | | 0 | 0 | 0 | 0 | 0 | 0 | 0 | 0 | 0 | 0 | 0 | 0 | 0 | 0 | 0 | 0 | 0 | 0 | 0 | 0 | 1 | 0 | 0 | 0 | 0 | | | | | | |
| 6595 | 07774 | 06594 | 1 | | 0 | 0 | 0 | 0 | 0 | 0 | 0 | 0 | 0 | 0 | 0 | 0 | 0 | 0 | 0 | 0 | 0 | 0 | 0 | 0 | 1 | 0 | 0 | 0 | 0 | | | | | | |
| 6596 | 07775 | 06595 | 1 | | 0 | 0 | 0 | 0 | 0 | 0 | 0 | 0 | 0 | 0 | 0 | 0 | 0 | 0 | 0 | 0 | 0 | 0 | 0 | 0 | 1 | 0 | 0 | 0 | 0 | | | | | | |
| 6597 | 07776 | 06596 | 1 | | 0 | 0 | 0 | 0 | 0 | 0 | 0 | 0 | 0 | 0 | 0 | 0 | 0 | 0 | 0 | 0 | 0 | 0 | 0 | 0 | 1 | 0 | 0 | 0 | 0 | | | | | | |
| 6598 | 07777 | 06597 | 1 | | 0 | 0 | 0 | 0 | 0 | 0 | 0 | 0 | 0 | 0 | 0 | 0 | 0 | 0 | 0 | 0 | 0 | 0 | 0 | 0 | 1 | 0 | 0 | 0 | 0 | | | | | | |
| 6599 | 07778 | 06598 | 2 | | 0 | 0 | 0 | 0 | 0 | 0 | 0 | 0 | 0 | 0 | 0 | 0 | 0 | 0 | 0 | 0 | 0 | 0 | 0 | 0 | 2 | 0 | 0 | 0 | 0 | | | | | | |
| 6600 | 07779 | 06599 | 1 | | 0 | 0 | 0 | 0 | 0 | 0 | 0 | 0 | 0 | 0 | 0 | 0 | 0 | 0 | 0 | 0 | 0 | 0 | 0 | 0 | 1 | 0 | 0 | 0 | 0 | | | | | | |
| 6601 | 07780 | 06600 | 1 | | 0 | 0 | 0 | 0 | 0 | 0 | 0 | 0 | 0 | 0 | 0 | 0 | 0 | 0 | 0 | 0 | 0 | 0 | 0 | 0 | 1 | 0 | 0 | 0 | 0 | | | | | | |
| 6602 | 07781 | 06601 | 1 | | 0 | 0 | 0 | 0 | 0 | 0 | 0 | 0 | 0 | 0 | 0 | 0 | 0 | 0 | 0 | 0 | 0 | 0 | 0 | 0 | 1 | 0 | 0 | 0 | 0 | | | | | | |
| 6603 | 07782 | 06602 | 1 | | 0 | 0 | 0 | 0 | 0 | 0 | 0 | 0 | 0 | 0 | 0 | 0 | 0 | 0 | 0 | 0 | 0 | 0 | 0 | 0 | 1 | 0 | 0 | 0 | 0 | | | | | | |
| 6604 | 07783 | 06603 | 1 | | 0 | 0 | 0 | 0 | 0 | 0 | 0 | 0 | 0 | 0 | 0 | 0 | 0 | 0 | 0 | 0 | 0 | 0 | 0 | 0 | 1 | 0 | 0 | 0 | 0 | | | | | | |
| 6605 | 07785 | 06604 | 1 | | 0 | 0 | 0 | 0 | 0 | 0 | 0 | 0 | 0 | 0 | 0 | 0 | 0 | 0 | 0 | 0 | 0 | 0 | 0 | 0 | 1 | 0 | 0 | 0 | 0 | | | | | | |
| 6606 | 07786 | 06605 | 1 | | 0 | 0 | 0 | 0 | 0 | 0 | 0 | 0 | 0 | 0 | 0 | 0 | 0 | 0 | 0 | 0 | 0 | 0 | 0 | 0 | 1 | 0 | 0 | 0 | 0 | | | | | | |
| 6607 | 07787 | 06606 | 1 | | 0 | 0 | 0 | 0 | 0 | 0 | 0 | 0 | 0 | 0 | 0 | 0 | 0 | 0 | 0 | 0 | 0 | 0 | 0 | 0 | 1 | 0 | 0 | 0 | 0 | | | | | | |
| 6608 | 07788 | 06607 | 1 | | 0 | 0 | 0 | 0 | 0 | 0 | 0 | 0 | 0 | 0 | 0 | 0 | 0 | 0 | 0 | 0 | 0 | 0 | 0 | 0 | 1 | 0 | 0 | 0 | 0 | | | | | | |
| 6609 | 07789 | 06608 | 1 | | 0 | 0 | 0 | 0 | 0 | 0 | 0 | 0 | 0 | 0 | 0 | 0 | 0 | 0 | 0 | 0 | 0 | 0 | 0 | 0 | 1 | 0 | 0 | 0 | 0 | | | | | | |

Table 184

| | A | B | C | E | G | I | K | M | O | Q | S | U | W | Y | AA | AC | AE | AG | AI | AK | AM | AO | AQ | AS | AU | AW | AY | BA | BC | BE | BF | BG | BH | BI | BK |
|---|---|---|---|---|---|---|---|---|---|---|---|---|---|---|---|---|---|---|---|---|---|---|---|---|---|---|---|---|---|---|---|---|---|---|---|
| 6610 | 07790 | 06609 | 1 | | 0 | 0 | 0 | 0 | 0 | 0 | 0 | 0 | 0 | 0 | 0 | 0 | 0 | 0 | 0 | 0 | 0 | 0 | 0 | 0 | 1 | 0 | 0 | 0 | 0 | | | | | | |
| 6611 | 07792 | 06610 | 1 | | 0 | 0 | 0 | 0 | 0 | 0 | 0 | 0 | 0 | 0 | 0 | 0 | 0 | 0 | 0 | 0 | 0 | 0 | 0 | 0 | 1 | 0 | 0 | 0 | 0 | | | | | | |
| 6612 | 07793 | 06611 | 1 | | 0 | 0 | 0 | 0 | 0 | 0 | 0 | 0 | 0 | 0 | 0 | 0 | 0 | 0 | 0 | 0 | 0 | 0 | 0 | 0 | 1 | 0 | 0 | 0 | 0 | | | | | | |
| 6613 | 07794 | 06612 | 1 | | 0 | 0 | 0 | 0 | 0 | 0 | 0 | 0 | 0 | 0 | 0 | 0 | 0 | 0 | 0 | 0 | 0 | 0 | 0 | 0 | 1 | 0 | 0 | 0 | 0 | | | | | | |
| 6614 | 07795 | 06613 | 6 | | 0 | 0 | 0 | 0 | 0 | 0 | 0 | 0 | 0 | 0 | 0 | 0 | 0 | 0 | 0 | 0 | 0 | 0 | 0 | 0 | 1 | 0 | 5 | 0 | 0 | M31732 | 98.5 | 66 | 1 | 1748 | 1813 |
| 6615 | 07796 | 06614 | 1 | | 0 | 0 | 0 | 0 | 0 | 0 | 0 | 0 | 0 | 0 | 0 | 0 | 0 | 0 | 0 | 0 | 0 | 0 | 0 | 0 | 1 | 0 | 0 | 0 | 0 | | | | | | |
| 6616 | 07797 | 06615 | 1 | | 0 | 0 | 0 | 0 | 0 | 0 | 0 | 0 | 0 | 0 | 0 | 0 | 0 | 0 | 0 | 0 | 0 | 0 | 0 | 0 | 1 | 0 | 0 | 0 | 0 | | | | | | |
| 6617 | 07798 | 06616 | 1 | | 0 | 0 | 0 | 0 | 0 | 0 | 0 | 0 | 0 | 0 | 0 | 0 | 0 | 0 | 0 | 0 | 0 | 0 | 0 | 0 | 1 | 0 | 0 | 0 | 0 | | | | | | |
| 6618 | 07800 | 06617 | 1 | | 0 | 0 | 0 | 0 | 0 | 0 | 0 | 0 | 0 | 0 | 0 | 0 | 0 | 0 | 0 | 0 | 0 | 0 | 0 | 0 | 1 | 0 | 0 | 0 | 0 | | | | | | |
| 6619 | 07801 | 06618 | 1 | | 0 | 0 | 0 | 0 | 0 | 0 | 0 | 0 | 0 | 0 | 0 | 0 | 0 | 0 | 0 | 0 | 0 | 0 | 0 | 0 | 1 | 0 | 0 | 0 | 0 | | | | | | |
| 6620 | 07802 | 06619 | 3 | | 0 | 0 | 0 | 0 | 0 | 0 | 0 | 0 | 0 | 0 | 0 | 0 | 0 | 0 | 0 | 0 | 0 | 0 | 0 | 0 | 1 | 0 | 2 | 0 | 0 | | | | | | |
| 6621 | 07804 | 06620 | 1 | | 0 | 0 | 0 | 0 | 0 | 0 | 0 | 0 | 0 | 0 | 0 | 0 | 0 | 0 | 0 | 0 | 0 | 0 | 0 | 0 | 1 | 0 | 0 | 0 | 0 | | | | | | |
| 6622 | 07805 | 06621 | 1 | | 0 | 0 | 0 | 0 | 0 | 0 | 0 | 0 | 0 | 0 | 0 | 0 | 0 | 0 | 0 | 0 | 0 | 0 | 0 | 0 | 1 | 0 | 0 | 0 | 0 | | | | | | |
| 6623 | 07807 | 06622 | 1 | | 0 | 0 | 0 | 0 | 0 | 0 | 0 | 0 | 0 | 0 | 0 | 0 | 0 | 0 | 0 | 0 | 0 | 0 | 0 | 0 | 1 | 0 | 0 | 0 | 0 | | | | | | |
| 6624 | 07808 | 06623 | 2 | | 0 | 0 | 0 | 0 | 0 | 0 | 0 | 0 | 0 | 0 | 0 | 0 | 0 | 0 | 0 | 0 | 0 | 0 | 0 | 0 | 1 | 1 | 0 | 0 | 0 | | | | | | |
| 6625 | 07810 | 06624 | 1 | | 0 | 0 | 0 | 0 | 0 | 0 | 0 | 0 | 0 | 0 | 0 | 0 | 0 | 0 | 0 | 0 | 0 | 0 | 0 | 0 | 1 | 0 | 0 | 0 | 0 | | | | | | |
| 6626 | 07811 | 06625 | 1 | | 0 | 0 | 0 | 0 | 0 | 0 | 0 | 0 | 0 | 0 | 0 | 0 | 0 | 0 | 0 | 0 | 0 | 0 | 0 | 0 | 1 | 0 | 0 | 0 | 0 | | | | | | |
| 6627 | 07812 | 06626 | 1 | | 0 | 0 | 0 | 0 | 0 | 0 | 0 | 0 | 0 | 0 | 0 | 0 | 0 | 0 | 0 | 0 | 0 | 0 | 0 | 0 | 1 | 0 | 0 | 0 | 0 | | | | | | |
| 6628 | 07813 | 06627 | 1 | | 0 | 0 | 0 | 0 | 0 | 0 | 0 | 0 | 0 | 0 | 0 | 0 | 0 | 0 | 0 | 0 | 0 | 0 | 0 | 0 | 1 | 0 | 0 | 0 | 0 | | | | | | |
| 6629 | 07814 | 06628 | 1 | | 0 | 0 | 0 | 0 | 0 | 0 | 0 | 0 | 0 | 0 | 0 | 0 | 0 | 0 | 0 | 0 | 0 | 0 | 0 | 0 | 1 | 0 | 0 | 0 | 0 | | | | | | |
| 6630 | 07815 | 06629 | 1 | | 0 | 0 | 0 | 0 | 0 | 0 | 0 | 0 | 0 | 0 | 0 | 0 | 0 | 0 | 0 | 0 | 0 | 0 | 0 | 0 | 1 | 0 | 0 | 0 | 0 | | | | | | |
| 6631 | 07816 | 06630 | 2 | | 0 | 0 | 0 | 0 | 0 | 0 | 0 | 0 | 0 | 0 | 0 | 0 | 0 | 0 | 0 | 0 | 0 | 0 | 0 | 0 | 1 | 1 | 0 | 0 | 0 | J05448 | 97.8 | 91 | 1 | 1669 | 1766 |
| 6632 | 07817 | 06631 | 1 | | 0 | 0 | 0 | 0 | 0 | 0 | 0 | 0 | 0 | 0 | 0 | 0 | 0 | 0 | 0 | 0 | 0 | 0 | 0 | 0 | 1 | 0 | 0 | 0 | 0 | | | | | | |
| 6633 | 07818 | 06632 | 1 | | 0 | 0 | 0 | 0 | 0 | 0 | 0 | 0 | 0 | 0 | 0 | 0 | 0 | 0 | 0 | 0 | 0 | 0 | 0 | 0 | 1 | 0 | 0 | 0 | 0 | | | | | | |
| 6634 | 07819 | 06633 | 1 | | 0 | 0 | 0 | 0 | 0 | 0 | 0 | 0 | 0 | 0 | 0 | 0 | 0 | 0 | 0 | 0 | 0 | 0 | 0 | 0 | 1 | 0 | 0 | 0 | 0 | | | | | | |
| 6635 | 07820 | 06634 | 1 | | 0 | 0 | 0 | 0 | 0 | 0 | 0 | 0 | 0 | 0 | 0 | 0 | 0 | 0 | 0 | 0 | 0 | 0 | 0 | 0 | 1 | 0 | 0 | 0 | 0 | | | | | | |
| 6636 | 07821 | 06635 | 1 | | 0 | 0 | 0 | 0 | 0 | 0 | 0 | 0 | 0 | 0 | 0 | 0 | 0 | 0 | 0 | 0 | 0 | 0 | 0 | 0 | 1 | 0 | 0 | 0 | 0 | | | | | | |
| 6637 | 07822 | 06636 | 2 | | 0 | 0 | 0 | 0 | 0 | 0 | 0 | 0 | 0 | 0 | 0 | 0 | 0 | 0 | 0 | 0 | 0 | 0 | 0 | 0 | 1 | 0 | 1 | 0 | 0 | | | | | | |
| 6638 | 07823 | 06637 | 1 | | 0 | 0 | 0 | 0 | 0 | 0 | 0 | 0 | 0 | 0 | 0 | 0 | 0 | 0 | 0 | 0 | 0 | 0 | 0 | 0 | 1 | 0 | 0 | 0 | 0 | | | | | | |
| 6639 | 07824 | 06638 | 1 | | 0 | 0 | 0 | 0 | 0 | 0 | 0 | 0 | 0 | 0 | 0 | 0 | 0 | 0 | 0 | 0 | 0 | 0 | 0 | 0 | 1 | 0 | 0 | 0 | 0 | | | | | | |
| 6640 | 07826 | 06639 | 1 | | 0 | 0 | 0 | 0 | 0 | 0 | 0 | 0 | 0 | 0 | 0 | 0 | 0 | 0 | 0 | 0 | 0 | 0 | 0 | 0 | 1 | 0 | 0 | 0 | 0 | | | | | | |
| 6641 | 07827 | 06640 | 1 | | 0 | 0 | 0 | 0 | 0 | 0 | 0 | 0 | 0 | 0 | 0 | 0 | 0 | 0 | 0 | 0 | 0 | 0 | 0 | 0 | 1 | 0 | 0 | 0 | 0 | L07395 | 95.3 | 127 | 1 | 1265 | 2226 |
| 6642 | 07828 | 06641 | 2 | | 0 | 0 | 0 | 0 | 0 | 0 | 0 | 0 | 0 | 0 | 0 | 0 | 0 | 0 | 0 | 0 | 0 | 0 | 0 | 0 | 1 | 0 | 0 | 1 | 0 | | | | | | |
| 6643 | 07829 | 06642 | 1 | | 0 | 0 | 0 | 0 | 0 | 0 | 0 | 0 | 0 | 0 | 0 | 0 | 0 | 0 | 0 | 0 | 0 | 0 | 0 | 0 | 1 | 0 | 0 | 0 | 0 | | | | | | |
| 6644 | 07831 | 06643 | 1 | | 0 | 0 | 0 | 0 | 0 | 0 | 0 | 0 | 0 | 0 | 0 | 0 | 0 | 0 | 0 | 0 | 0 | 0 | 0 | 0 | 1 | 0 | 0 | 0 | 0 | | | | | | |
| 6645 | 07834 | 06644 | 1 | | 0 | 0 | 0 | 0 | 0 | 0 | 0 | 0 | 0 | 0 | 0 | 0 | 0 | 0 | 0 | 0 | 0 | 0 | 0 | 0 | 1 | 0 | 0 | 0 | 0 | | | | | | |

Table 185

| | A | B | C | E | G | I | K | M | O | Q | S | U | W | Y | AA | AC | AE | AG | AI | AK | AM | AO | AQ | AS | AU | AW | AY | BA | BC | BE | BF | BG | BH | BI | BK |
|---|---|---|---|---|---|---|---|---|---|---|---|---|---|---|---|---|---|---|---|---|---|---|---|---|---|---|---|---|---|---|---|---|---|---|---|
| 6646 | 07835 | 06645 | 1 | | 0 | 0 | 0 | 0 | 0 | 0 | 0 | 0 | 0 | 0 | 0 | 0 | 0 | 0 | 0 | 0 | 0 | 0 | 0 | 0 | 1 | 0 | 0 | 0 | 0 | | | | | | |
| 6647 | 07836 | 06646 | 2 | | 0 | 0 | 0 | 0 | 0 | 0 | 0 | 0 | 0 | 0 | 0 | 0 | 0 | 0 | 0 | 0 | 0 | 0 | 0 | 0 | 2 | 0 | 0 | 0 | 0 | | | | | | |
| 6648 | 07837 | 06647 | 1 | | 0 | 0 | 0 | 0 | 0 | 0 | 0 | 0 | 0 | 0 | 0 | 0 | 0 | 0 | 0 | 0 | 0 | 0 | 0 | 0 | 1 | 0 | 0 | 0 | 0 | | | | | | |
| 6649 | 07838 | 06648 | 1 | | 0 | 0 | 0 | 0 | 0 | 0 | 0 | 0 | 0 | 0 | 0 | 0 | 0 | 0 | 0 | 0 | 0 | 0 | 0 | 0 | 1 | 0 | 0 | 0 | 0 | | | | | | |
| 6650 | 07839 | 06649 | 2 | | 0 | 0 | 0 | 0 | 0 | 0 | 0 | 0 | 0 | 0 | 0 | 0 | 0 | 0 | 0 | 0 | 0 | 0 | 0 | 0 | 1 | 0 | 0 | 1 | 0 | | | | | | |
| 6651 | 07840 | 06650 | 1 | | 0 | 0 | 0 | 0 | 0 | 0 | 0 | 0 | 0 | 0 | 0 | 0 | 0 | 0 | 0 | 0 | 0 | 0 | 0 | 0 | 1 | 0 | 0 | 0 | 0 | | | | | | |
| 6652 | 07841 | 06651 | 3 | | 0 | 0 | 0 | 0 | 0 | 0 | 0 | 0 | 0 | 0 | 0 | 0 | 0 | 0 | 0 | 0 | 0 | 0 | 0 | 0 | 2 | 0 | 1 | 0 | 0 | | | | | | |
| 6653 | 07844 | 06652 | 1 | | 0 | 0 | 0 | 0 | 0 | 0 | 0 | 0 | 0 | 0 | 0 | 0 | 0 | 0 | 0 | 0 | 0 | 0 | 0 | 0 | 1 | 0 | 0 | 0 | 0 | | | | | | |
| 6654 | 07845 | 06653 | 1 | | 0 | 0 | 0 | 0 | 0 | 0 | 0 | 0 | 0 | 0 | 0 | 0 | 0 | 0 | 0 | 0 | 0 | 0 | 0 | 0 | 1 | 0 | 0 | 0 | 0 | | | | | | |
| 6655 | 07848 | 06654 | 7 | | 0 | 0 | 0 | 0 | 0 | 0 | 0 | 0 | 0 | 0 | 0 | 0 | 0 | 0 | 0 | 0 | 0 | 0 | 0 | 0 | 7 | 0 | 0 | 0 | 0 | | | | | | |
| 6656 | 07849 | 06655 | 1 | | 0 | 0 | 0 | 0 | 0 | 0 | 0 | 0 | 0 | 0 | 0 | 0 | 0 | 0 | 0 | 0 | 0 | 0 | 0 | 0 | 1 | 0 | 0 | 0 | 0 | | | | | | |
| 6657 | 07850 | 06656 | 1 | | 0 | 0 | 0 | 0 | 0 | 0 | 0 | 0 | 0 | 0 | 0 | 0 | 0 | 0 | 0 | 0 | 0 | 0 | 0 | 0 | 1 | 0 | 0 | 0 | 0 | | | | | | |
| 6658 | 07852 | 06657 | 1 | | 0 | 0 | 0 | 0 | 0 | 0 | 0 | 0 | 0 | 0 | 0 | 0 | 0 | 0 | 0 | 0 | 0 | 0 | 0 | 0 | 1 | 0 | 0 | 0 | 0 | | | | | | |
| 6659 | 07853 | 06658 | 1 | | 0 | 0 | 0 | 0 | 0 | 0 | 0 | 0 | 0 | 0 | 0 | 0 | 0 | 0 | 0 | 0 | 0 | 0 | 0 | 0 | 1 | 0 | 0 | 0 | 0 | | | | | | |
| 6660 | 07854 | 06659 | 2 | | 0 | 0 | 0 | 0 | 0 | 0 | 0 | 0 | 0 | 0 | 0 | 0 | 0 | 0 | 0 | 0 | 0 | 0 | 0 | 0 | 2 | 0 | 0 | 0 | 0 | | | | | | |
| 6661 | 07855 | 06660 | 1 | | 0 | 0 | 0 | 0 | 0 | 0 | 0 | 0 | 0 | 0 | 0 | 0 | 0 | 0 | 0 | 0 | 0 | 0 | 0 | 0 | 1 | 0 | 0 | 0 | 0 | | | | | | |
| 6662 | 07856 | 06661 | 1 | | 0 | 0 | 0 | 0 | 0 | 0 | 0 | 0 | 0 | 0 | 0 | 0 | 0 | 0 | 0 | 0 | 0 | 0 | 0 | 0 | 1 | 0 | 0 | 0 | 0 | Z22555 | 94.2 | 278 | 1 | 2228 | 2566 |
| 6663 | 07857 | 06662 | 1 | | 0 | 0 | 0 | 0 | 0 | 0 | 0 | 0 | 0 | 0 | 0 | 0 | 0 | 0 | 0 | 0 | 0 | 0 | 0 | 0 | 1 | 0 | 0 | 0 | 0 | | | | | | |
| 6664 | 07858 | 06663 | 1 | | 0 | 0 | 0 | 0 | 0 | 0 | 0 | 0 | 0 | 0 | 0 | 0 | 0 | 0 | 0 | 0 | 0 | 0 | 0 | 0 | 1 | 0 | 0 | 0 | 0 | | | | | | |
| 6665 | 07859 | 06664 | 1 | | 0 | 0 | 0 | 0 | 0 | 0 | 0 | 0 | 0 | 0 | 0 | 0 | 0 | 0 | 0 | 0 | 0 | 0 | 0 | 0 | 1 | 0 | 0 | 0 | 0 | S49355 | 94.4 | 284 | 1 | 1211 | 1558 |
| 6666 | 07860 | 06665 | 1 | | 0 | 0 | 0 | 0 | 0 | 0 | 0 | 0 | 0 | 0 | 0 | 0 | 0 | 0 | 0 | 0 | 0 | 0 | 0 | 0 | 1 | 0 | 0 | 0 | 0 | | | | | | |
| 6667 | 07861 | 06666 | 2 | | 0 | 0 | 0 | 0 | 0 | 0 | 0 | 0 | 0 | 0 | 0 | 0 | 0 | 0 | 0 | 0 | 0 | 0 | 0 | 0 | 1 | 0 | 1 | 0 | 0 | | | | | | |
| 6668 | 07862 | 06667 | 1 | | 0 | 0 | 0 | 0 | 0 | 0 | 0 | 0 | 0 | 0 | 0 | 0 | 0 | 0 | 0 | 0 | 0 | 0 | 0 | 0 | 1 | 0 | 0 | 0 | 0 | | | | | | |
| 6669 | 07863 | 06668 | 1 | | 0 | 0 | 0 | 0 | 0 | 0 | 0 | 0 | 0 | 0 | 0 | 0 | 0 | 0 | 0 | 0 | 0 | 0 | 0 | 0 | 1 | 0 | 0 | 0 | 0 | | | | | | |
| 6670 | 07864 | 06669 | 1 | | 0 | 0 | 0 | 0 | 0 | 0 | 0 | 0 | 0 | 0 | 0 | 0 | 0 | 0 | 0 | 0 | 0 | 0 | 0 | 0 | 1 | 0 | 0 | 0 | 0 | | | | | | |
| 6671 | 07865 | 06670 | 1 | | 0 | 0 | 0 | 0 | 0 | 0 | 0 | 0 | 0 | 0 | 0 | 0 | 0 | 0 | 0 | 0 | 0 | 0 | 0 | 0 | 1 | 0 | 0 | 0 | 0 | | | | | | |
| 6672 | 07866 | 06671 | 2 | | 0 | 0 | 0 | 0 | 0 | 0 | 0 | 0 | 0 | 0 | 0 | 0 | 0 | 0 | 0 | 0 | 0 | 0 | 0 | 0 | 1 | 1 | 0 | 0 | 0 | | | | | | |
| 6673 | 07868 | 06672 | 1 | | 0 | 0 | 0 | 0 | 0 | 0 | 0 | 0 | 0 | 0 | 0 | 0 | 0 | 0 | 0 | 0 | 0 | 0 | 0 | 0 | 1 | 0 | 0 | 0 | 0 | | | | | | |
| 6674 | 07869 | 06673 | 1 | | 0 | 0 | 0 | 0 | 0 | 0 | 0 | 0 | 0 | 0 | 0 | 0 | 0 | 0 | 0 | 0 | 0 | 0 | 0 | 0 | 1 | 0 | 0 | 0 | 0 | | | | | | |
| 6675 | 07870 | 06674 | 1 | | 0 | 0 | 0 | 0 | 0 | 0 | 0 | 0 | 0 | 0 | 0 | 0 | 0 | 0 | 0 | 0 | 0 | 0 | 0 | 0 | 1 | 0 | 0 | 0 | 0 | | | | | | |
| 6676 | 07871 | 06675 | 1 | | 0 | 0 | 0 | 0 | 0 | 0 | 0 | 0 | 0 | 0 | 0 | 0 | 0 | 0 | 0 | 0 | 0 | 0 | 0 | 0 | 1 | 0 | 0 | 0 | 0 | | | | | | |
| 6677 | 07872 | 06676 | 1 | | 0 | 0 | 0 | 0 | 0 | 0 | 0 | 0 | 0 | 0 | 0 | 0 | 0 | 0 | 0 | 0 | 0 | 0 | 0 | 0 | 1 | 0 | 0 | 0 | 0 | | | | | | |
| 6678 | 07875 | 06677 | 1 | | 0 | 0 | 0 | 0 | 0 | 0 | 0 | 0 | 0 | 0 | 0 | 0 | 0 | 0 | 0 | 0 | 0 | 0 | 0 | 0 | 1 | 0 | 0 | 0 | 0 | X63422 | 94.9 | 138 | 1 | 843 | 994 |
| 6679 | 07876 | 06678 | 1 | | 0 | 0 | 0 | 0 | 0 | 0 | 0 | 0 | 0 | 0 | 0 | 0 | 0 | 0 | 0 | 0 | 0 | 0 | 0 | 0 | 1 | 0 | 0 | 0 | 0 | L20868 | 94.5 | 163 | 45 | 1043 | 1379 |
| 6680 | 07878 | 06679 | 1 | | 0 | 0 | 0 | 0 | 0 | 0 | 0 | 0 | 0 | 0 | 0 | 0 | 0 | 0 | 0 | 0 | 0 | 0 | 0 | 0 | 1 | 0 | 0 | 0 | 0 | | | | | | |
| 6681 | 07879 | 06680 | 2 | | 0 | 0 | 0 | 0 | 0 | 0 | 0 | 0 | 0 | 0 | 0 | 0 | 0 | 0 | 0 | 0 | 0 | 0 | 0 | 0 | 2 | 0 | 0 | 0 | 0 | | | | | | |

Table 186

| | A | B | C | E | G | I | K | M | O | Q | S | U | W | Y | AA | AC | AE | AG | AI | AK | AM | AO | AQ | AS | AU | AW | AY | BA | BC | BE | BF | BG | BH | BI | BK |
|---|---|---|---|---|---|---|---|---|---|---|---|---|---|---|---|---|---|---|---|---|---|---|---|---|---|---|---|---|---|---|---|---|---|---|---|
| 6682 | 07880 | 06681 | 2 | | 0 | 0 | 0 | 0 | 0 | 0 | 0 | 0 | 0 | 0 | 0 | 0 | 0 | 0 | 0 | 0 | 0 | 0 | 0 | 0 | 2 | 0 | 0 | 0 | 0 | M60278 | 98.6 | 294 | 1 | 1700 | 2360 |
| 6683 | 07881 | 06682 | 1 | | 0 | 0 | 0 | 0 | 0 | 0 | 0 | 0 | 0 | 0 | 0 | 0 | 0 | 0 | 0 | 0 | 0 | 0 | 0 | 0 | 1 | 0 | 0 | 0 | 0 | | | | | | |
| 6684 | 07882 | 06683 | 1 | | 0 | 0 | 0 | 0 | 0 | 0 | 0 | 0 | 0 | 0 | 0 | 0 | 0 | 0 | 0 | 0 | 0 | 0 | 0 | 0 | 1 | 0 | 0 | 0 | 0 | | | | | | |
| 6685 | 07883 | 06684 | 2 | | 0 | 0 | 0 | 0 | 0 | 0 | 0 | 0 | 0 | 0 | 0 | 0 | 0 | 0 | 0 | 0 | 0 | 0 | 0 | 0 | 1 | 0 | 0 | 1 | 0 | | | | | | |
| 6686 | 07884 | 06685 | 1 | | 0 | 0 | 0 | 0 | 0 | 0 | 0 | 0 | 0 | 0 | 0 | 0 | 0 | 0 | 0 | 0 | 0 | 0 | 0 | 0 | 1 | 0 | 0 | 0 | 0 | | | | | | |
| 6687 | 07885 | 06686 | 1 | | 0 | 0 | 0 | 0 | 0 | 0 | 0 | 0 | 0 | 0 | 0 | 0 | 0 | 0 | 0 | 0 | 0 | 0 | 0 | 0 | 1 | 0 | 0 | 0 | 0 | | | | | | |
| 6688 | 07886 | 06687 | 1 | | 0 | 0 | 0 | 0 | 0 | 0 | 0 | 0 | 0 | 0 | 0 | 0 | 0 | 0 | 0 | 0 | 0 | 0 | 0 | 0 | 1 | 0 | 0 | 0 | 0 | | | | | | |
| 6689 | 07887 | 06688 | 1 | | 0 | 0 | 0 | 0 | 0 | 0 | 0 | 0 | 0 | 0 | 0 | 0 | 0 | 0 | 0 | 0 | 0 | 0 | 0 | 0 | 1 | 0 | 0 | 0 | 0 | | | | | | |
| 6690 | 07889 | 06689 | 1 | | 0 | 0 | 0 | 0 | 0 | 0 | 0 | 0 | 0 | 0 | 0 | 0 | 0 | 0 | 0 | 0 | 0 | 0 | 0 | 0 | 1 | 0 | 0 | 0 | 0 | | | | | | |
| 6691 | 07890 | 06690 | 1 | | 0 | 0 | 0 | 0 | 0 | 0 | 0 | 0 | 0 | 0 | 0 | 0 | 0 | 0 | 0 | 0 | 0 | 0 | 0 | 0 | 1 | 0 | 0 | 0 | 0 | | | | | | |
| 6692 | 07891 | 06691 | 1 | | 0 | 0 | 0 | 0 | 0 | 0 | 0 | 0 | 0 | 0 | 0 | 0 | 0 | 0 | 0 | 0 | 0 | 0 | 0 | 0 | 1 | 0 | 0 | 0 | 0 | | | | | | |
| 6693 | 07892 | 06692 | 1 | | 0 | 0 | 0 | 0 | 0 | 0 | 0 | 0 | 0 | 0 | 0 | 0 | 0 | 0 | 0 | 0 | 0 | 0 | 0 | 0 | 1 | 0 | 0 | 0 | 0 | | | | | | |
| 6694 | 07893 | 06693 | 1 | | 0 | 0 | 0 | 0 | 0 | 0 | 0 | 0 | 0 | 0 | 0 | 0 | 0 | 0 | 0 | 0 | 0 | 0 | 0 | 0 | 1 | 0 | 0 | 0 | 0 | | | | | | |
| 6695 | 07894 | 06694 | 1 | | 0 | 0 | 0 | 0 | 0 | 0 | 0 | 0 | 0 | 0 | 0 | 0 | 0 | 0 | 0 | 0 | 0 | 0 | 0 | 0 | 1 | 0 | 0 | 0 | 0 | | | | | | |
| 6696 | 07895 | 06695 | 1 | | 0 | 0 | 0 | 0 | 0 | 0 | 0 | 0 | 0 | 0 | 0 | 0 | 0 | 0 | 0 | 0 | 0 | 0 | 0 | 0 | 1 | 0 | 0 | 0 | 0 | | | | | | |
| 6697 | 07896 | 06696 | 1 | | 0 | 0 | 0 | 0 | 0 | 0 | 0 | 0 | 0 | 0 | 0 | 0 | 0 | 0 | 0 | 0 | 0 | 0 | 0 | 0 | 1 | 0 | 0 | 0 | 0 | | | | | | |
| 6698 | 07897 | 06697 | 1 | | 0 | 0 | 0 | 0 | 0 | 0 | 0 | 0 | 0 | 0 | 0 | 0 | 0 | 0 | 0 | 0 | 0 | 0 | 0 | 0 | 1 | 0 | 0 | 0 | 0 | | | | | | |
| 6699 | 07898 | 06698 | 1 | | 0 | 0 | 0 | 0 | 0 | 0 | 0 | 0 | 0 | 0 | 0 | 0 | 0 | 0 | 0 | 0 | 0 | 0 | 0 | 0 | 1 | 0 | 0 | 0 | 0 | | | | | | |
| 6700 | 07900 | 06699 | 1 | | 0 | 0 | 0 | 0 | 0 | 0 | 0 | 0 | 0 | 0 | 0 | 0 | 0 | 0 | 0 | 0 | 0 | 0 | 0 | 0 | 1 | 0 | 0 | 0 | 0 | | | | | | |
| 6701 | 07901 | 06700 | 1 | | 0 | 0 | 0 | 0 | 0 | 0 | 0 | 0 | 0 | 0 | 0 | 0 | 0 | 0 | 0 | 0 | 0 | 0 | 0 | 0 | 1 | 0 | 0 | 0 | 0 | | | | | | |
| 6702 | 07902 | 06701 | 2 | | 0 | 0 | 0 | 0 | 0 | 0 | 0 | 0 | 0 | 0 | 0 | 0 | 0 | 0 | 0 | 0 | 0 | 0 | 0 | 0 | 1 | 1 | 0 | 0 | 0 | | | | | | |
| 6703 | 07903 | 06702 | 1 | | 0 | 0 | 0 | 0 | 0 | 0 | 0 | 0 | 0 | 0 | 0 | 0 | 0 | 0 | 0 | 0 | 0 | 0 | 0 | 0 | 1 | 0 | 0 | 0 | 0 | | | | | | |
| 6704 | 07904 | 06703 | 1 | | 0 | 0 | 0 | 0 | 0 | 0 | 0 | 0 | 0 | 0 | 0 | 0 | 0 | 0 | 0 | 0 | 0 | 0 | 0 | 0 | 1 | 0 | 0 | 0 | 0 | | | | | | |
| 6705 | 07905 | 06704 | 1 | | 0 | 0 | 0 | 0 | 0 | 0 | 0 | 0 | 0 | 0 | 0 | 0 | 0 | 0 | 0 | 0 | 0 | 0 | 0 | 0 | 1 | 0 | 0 | 0 | 0 | | | | | | |
| 6706 | 07909 | 06705 | 1 | | 0 | 0 | 0 | 0 | 0 | 0 | 0 | 0 | 0 | 0 | 0 | 0 | 0 | 0 | 0 | 0 | 0 | 0 | 0 | 0 | 1 | 0 | 0 | 0 | 0 | | | | | | |
| 6707 | 07911 | 06706 | 1 | | 0 | 0 | 0 | 0 | 0 | 0 | 0 | 0 | 0 | 0 | 0 | 0 | 0 | 0 | 0 | 0 | 0 | 0 | 0 | 0 | 1 | 0 | 0 | 0 | 0 | | | | | | |
| 6708 | 07912 | 06707 | 1 | | 0 | 0 | 0 | 0 | 0 | 0 | 0 | 0 | 0 | 0 | 0 | 0 | 0 | 0 | 0 | 0 | 0 | 0 | 0 | 0 | 1 | 0 | 0 | 0 | 0 | | | | | | |
| 6709 | 07913 | 06708 | 1 | | 0 | 0 | 0 | 0 | 0 | 0 | 0 | 0 | 0 | 0 | 0 | 0 | 0 | 0 | 0 | 0 | 0 | 0 | 0 | 0 | 1 | 0 | 0 | 0 | 0 | | | | | | |
| 6710 | 07915 | 06709 | 1 | | 0 | 0 | 0 | 0 | 0 | 0 | 0 | 0 | 0 | 0 | 0 | 0 | 0 | 0 | 0 | 0 | 0 | 0 | 0 | 0 | 1 | 0 | 0 | 0 | 0 | | | | | | |
| 6711 | 07916 | 06710 | 1 | | 0 | 0 | 0 | 0 | 0 | 0 | 0 | 0 | 0 | 0 | 0 | 0 | 0 | 0 | 0 | 0 | 0 | 0 | 0 | 0 | 1 | 0 | 0 | 0 | 0 | | | | | | |
| 6712 | 07917 | 06711 | 1 | | 0 | 0 | 0 | 0 | 0 | 0 | 0 | 0 | 0 | 0 | 0 | 0 | 0 | 0 | 0 | 0 | 0 | 0 | 0 | 0 | 1 | 0 | 0 | 0 | 0 | | | | | | |
| 6713 | 07918 | 06712 | 1 | | 0 | 0 | 0 | 0 | 0 | 0 | 0 | 0 | 0 | 0 | 0 | 0 | 0 | 0 | 0 | 0 | 0 | 0 | 0 | 0 | 1 | 0 | 0 | 0 | 0 | | | | | | |
| 6714 | 07919 | 06713 | 1 | | 0 | 0 | 0 | 0 | 0 | 0 | 0 | 0 | 0 | 0 | 0 | 0 | 0 | 0 | 0 | 0 | 0 | 0 | 0 | 0 | 1 | 0 | 0 | 0 | 0 | | | | | | |
| 6715 | 07920 | 06714 | 1 | | 0 | 0 | 0 | 0 | 0 | 0 | 0 | 0 | 0 | 0 | 0 | 0 | 0 | 0 | 0 | 0 | 0 | 0 | 0 | 0 | 1 | 0 | 0 | 0 | 0 | | | | | | |
| 6716 | 07921 | 06715 | 1 | | 0 | 0 | 0 | 0 | 0 | 0 | 0 | 0 | 0 | 0 | 0 | 0 | 0 | 0 | 0 | 0 | 0 | 0 | 0 | 0 | 1 | 0 | 0 | 0 | 0 | | | | | | |
| 6717 | 07922 | 06716 | 2 | | 0 | 0 | 0 | 0 | 0 | 0 | 0 | 0 | 0 | 0 | 0 | 0 | 0 | 0 | 0 | 0 | 0 | 0 | 0 | 0 | 2 | 0 | 0 | 0 | 0 | | | | | | |

Table 187

| | A | B | C | E | G | I | K | M | O | Q | S | U | W | Y | AA | AC | AE | AG | AI | AK | AM | AO | AQ | AS | AU | AW | AY | BA | BC | BE | BF | BG | BH | BI | BK |
|---|---|---|---|---|---|---|---|---|---|---|---|---|---|---|---|---|---|---|---|---|---|---|---|---|---|---|---|---|---|---|---|---|---|---|---|
| 6718 | 07923 | 06717 | 3 | | 0 | 0 | 0 | 0 | 0 | 0 | 0 | 0 | 0 | 0 | 0 | 0 | 0 | 0 | 0 | 0 | 0 | 0 | 0 | 0 | 1 | 0 | 2 | 0 | 0 | | | | | | |
| 6719 | 07925 | 06718 | 1 | | 0 | 0 | 0 | 0 | 0 | 0 | 0 | 0 | 0 | 0 | 0 | 0 | 0 | 0 | 0 | 0 | 0 | 0 | 0 | 0 | 1 | 0 | 0 | 0 | 0 | | | | | | |
| 6720 | 07927 | 06719 | 1 | | 0 | 0 | 0 | 0 | 0 | 0 | 0 | 0 | 0 | 0 | 0 | 0 | 0 | 0 | 0 | 0 | 0 | 0 | 0 | 0 | 1 | 0 | 0 | 0 | 0 | | | | | | |
| 6721 | 07928 | 06720 | 1 | | 0 | 0 | 0 | 0 | 0 | 0 | 0 | 0 | 0 | 0 | 0 | 0 | 0 | 0 | 0 | 0 | 0 | 0 | 0 | 0 | 1 | 0 | 0 | 0 | 0 | | | | | | |
| 6722 | 07932 | 06721 | 1 | | 0 | 0 | 0 | 0 | 0 | 0 | 0 | 0 | 0 | 0 | 0 | 0 | 0 | 0 | 0 | 0 | 0 | 0 | 0 | 0 | 1 | 0 | 0 | 0 | 0 | | | | | | |
| 6723 | 07933 | 06722 | 1 | | 0 | 0 | 0 | 0 | 0 | 0 | 0 | 0 | 0 | 0 | 0 | 0 | 0 | 0 | 0 | 0 | 0 | 0 | 0 | 0 | 1 | 0 | 0 | 0 | 0 | | | | | | |
| 6724 | 07934 | 06723 | 1 | | 0 | 0 | 0 | 0 | 0 | 0 | 0 | 0 | 0 | 0 | 0 | 0 | 0 | 0 | 0 | 0 | 0 | 0 | 0 | 0 | 1 | 0 | 0 | 0 | 0 | | | | | | |
| 6725 | 07935 | 06724 | 2 | | 0 | 0 | 0 | 0 | 0 | 0 | 0 | 0 | 0 | 0 | 0 | 0 | 0 | 0 | 0 | 0 | 0 | 0 | 0 | 0 | 1 | 0 | 1 | 0 | 0 | L13852 | 91.9 | 62 | 1 | 3457 | 3520 |
| 6726 | 07936 | 06725 | 1 | | 0 | 0 | 0 | 0 | 0 | 0 | 0 | 0 | 0 | 0 | 0 | 0 | 0 | 0 | 0 | 0 | 0 | 0 | 0 | 0 | 1 | 0 | 0 | 0 | 0 | | | | | | |
| 6727 | 07937 | 06726 | 2 | | 0 | 0 | 0 | 0 | 0 | 0 | 0 | 0 | 0 | 0 | 0 | 0 | 0 | 0 | 0 | 0 | 0 | 0 | 0 | 0 | 1 | 0 | 0 | 1 | 0 | | | | | | |
| 6728 | 07938 | 06727 | 3 | | 0 | 0 | 0 | 0 | 0 | 0 | 0 | 0 | 0 | 0 | 0 | 0 | 0 | 0 | 0 | 0 | 0 | 0 | 0 | 0 | 3 | 0 | 0 | 0 | 0 | | | | | | |
| 6729 | 07939 | 06728 | 1 | | 0 | 0 | 0 | 0 | 0 | 0 | 0 | 0 | 0 | 0 | 0 | 0 | 0 | 0 | 0 | 0 | 0 | 0 | 0 | 0 | 1 | 0 | 0 | 0 | 0 | | | | | | |
| 6730 | 07941 | 06729 | 2 | | 0 | 0 | 0 | 0 | 0 | 0 | 0 | 0 | 0 | 0 | 0 | 0 | 0 | 0 | 0 | 0 | 0 | 0 | 0 | 0 | 1 | 0 | 0 | 1 | 0 | | | | | | |
| 6731 | 07942 | 06730 | 1 | | 0 | 0 | 0 | 0 | 0 | 0 | 0 | 0 | 0 | 0 | 0 | 0 | 0 | 0 | 0 | 0 | 0 | 0 | 0 | 0 | 1 | 0 | 0 | 0 | 0 | | | | | | |
| 6732 | 07944 | 06731 | 1 | | 0 | 0 | 0 | 0 | 0 | 0 | 0 | 0 | 0 | 0 | 0 | 0 | 0 | 0 | 0 | 0 | 0 | 0 | 0 | 0 | 1 | 0 | 0 | 0 | 0 | | | | | | |
| 6733 | 07945 | 06732 | 1 | | 0 | 0 | 0 | 0 | 0 | 0 | 0 | 0 | 0 | 0 | 0 | 0 | 0 | 0 | 0 | 0 | 0 | 0 | 0 | 0 | 1 | 0 | 0 | 0 | 0 | | | | | | |
| 6734 | 07946 | 06733 | 1 | | 0 | 0 | 0 | 0 | 0 | 0 | 0 | 0 | 0 | 0 | 0 | 0 | 0 | 0 | 0 | 0 | 0 | 0 | 0 | 0 | 1 | 0 | 0 | 0 | 0 | | | | | | |
| 6735 | 07948 | 06734 | 1 | | 0 | 0 | 0 | 0 | 0 | 0 | 0 | 0 | 0 | 0 | 0 | 0 | 0 | 0 | 0 | 0 | 0 | 0 | 0 | 0 | 1 | 0 | 0 | 0 | 0 | | | | | | |
| 6736 | 07949 | 06735 | 1 | | 0 | 0 | 0 | 0 | 0 | 0 | 0 | 0 | 0 | 0 | 0 | 0 | 0 | 0 | 0 | 0 | 0 | 0 | 0 | 0 | 1 | 0 | 0 | 0 | 0 | | | | | | |
| 6737 | 07951 | 06736 | 1 | | 0 | 0 | 0 | 0 | 0 | 0 | 0 | 0 | 0 | 0 | 0 | 0 | 0 | 0 | 0 | 0 | 0 | 0 | 0 | 0 | 1 | 0 | 0 | 0 | 0 | | | | | | |
| 6738 | 07952 | 06737 | 1 | | 0 | 0 | 0 | 0 | 0 | 0 | 0 | 0 | 0 | 0 | 0 | 0 | 0 | 0 | 0 | 0 | 0 | 0 | 0 | 0 | 1 | 0 | 0 | 0 | 0 | | | | | | |
| 6739 | 07953 | 06738 | 1 | | 0 | 0 | 0 | 0 | 0 | 0 | 0 | 0 | 0 | 0 | 0 | 0 | 0 | 0 | 0 | 0 | 0 | 0 | 0 | 0 | 1 | 0 | 0 | 0 | 0 | | | | | | |
| 6740 | 07954 | 06739 | 1 | | 0 | 0 | 0 | 0 | 0 | 0 | 0 | 0 | 0 | 0 | 0 | 0 | 0 | 0 | 0 | 0 | 0 | 0 | 0 | 0 | 1 | 0 | 0 | 0 | 0 | | | | | | |
| 6741 | 07955 | 06740 | 1 | | 0 | 0 | 0 | 0 | 0 | 0 | 0 | 0 | 0 | 0 | 0 | 0 | 0 | 0 | 0 | 0 | 0 | 0 | 0 | 0 | 1 | 0 | 0 | 0 | 0 | | | | | | |
| 6742 | 07956 | 06741 | 1 | | 0 | 0 | 0 | 0 | 0 | 0 | 0 | 0 | 0 | 0 | 0 | 0 | 0 | 0 | 0 | 0 | 0 | 0 | 0 | 0 | 1 | 0 | 0 | 0 | 0 | | | | | | |
| 6743 | 07957 | 06742 | 1 | | 0 | 0 | 0 | 0 | 0 | 0 | 0 | 0 | 0 | 0 | 0 | 0 | 0 | 0 | 0 | 0 | 0 | 0 | 0 | 0 | 1 | 0 | 0 | 0 | 0 | | | | | | |
| 6744 | 07958 | 06743 | 1 | | 0 | 0 | 0 | 0 | 0 | 0 | 0 | 0 | 0 | 0 | 0 | 0 | 0 | 0 | 0 | 0 | 0 | 0 | 0 | 0 | 1 | 0 | 0 | 0 | 0 | | | | | | |
| 6745 | 07959 | 06744 | 1 | | 0 | 0 | 0 | 0 | 0 | 0 | 0 | 0 | 0 | 0 | 0 | 0 | 0 | 0 | 0 | 0 | 0 | 0 | 0 | 0 | 1 | 0 | 0 | 0 | 0 | | | | | | |
| 6746 | 07960 | 06745 | 1 | | 0 | 0 | 0 | 0 | 0 | 0 | 0 | 0 | 0 | 0 | 0 | 0 | 0 | 0 | 0 | 0 | 0 | 0 | 0 | 0 | 1 | 0 | 0 | 0 | 0 | | | | | | |
| 6747 | 07961 | 06746 | 1 | | 0 | 0 | 0 | 0 | 0 | 0 | 0 | 0 | 0 | 0 | 0 | 0 | 0 | 0 | 0 | 0 | 0 | 0 | 0 | 0 | 1 | 0 | 0 | 0 | 0 | | | | | | |
| 6748 | 07963 | 06747 | 1 | | 0 | 0 | 0 | 0 | 0 | 0 | 0 | 0 | 0 | 0 | 0 | 0 | 0 | 0 | 0 | 0 | 0 | 0 | 0 | 0 | 1 | 0 | 0 | 0 | 0 | | | | | | |
| 6749 | 07964 | 06748 | 1 | | 0 | 0 | 0 | 0 | 0 | 0 | 0 | 0 | 0 | 0 | 0 | 0 | 0 | 0 | 0 | 0 | 0 | 0 | 0 | 0 | 1 | 0 | 0 | 0 | 0 | | | | | | |
| 6750 | 07965 | 06749 | 1 | | 0 | 0 | 0 | 0 | 0 | 0 | 0 | 0 | 0 | 0 | 0 | 0 | 0 | 0 | 0 | 0 | 0 | 0 | 0 | 0 | 1 | 0 | 0 | 0 | 0 | | | | | | |
| 6751 | 07966 | 06750 | 1 | | 0 | 0 | 0 | 0 | 0 | 0 | 0 | 0 | 0 | 0 | 0 | 0 | 0 | 0 | 0 | 0 | 0 | 0 | 0 | 0 | 1 | 0 | 0 | 0 | 0 | | | | | | |
| 6752 | 07967 | 06751 | 1 | | 0 | 0 | 0 | 0 | 0 | 0 | 0 | 0 | 0 | 0 | 0 | 0 | 0 | 0 | 0 | 0 | 0 | 0 | 0 | 0 | 1 | 0 | 0 | 0 | 0 | | | | | | |
| 6753 | 07969 | 06752 | 1 | | 0 | 0 | 0 | 0 | 0 | 0 | 0 | 0 | 0 | 0 | 0 | 0 | 0 | 0 | 0 | 0 | 0 | 0 | 0 | 0 | 1 | 0 | 0 | 0 | 0 | | | | | | |

Table 188

| | A | B | C | E | G | I | K | M | O | Q | S | U | W | Y | AA | AC | AE | AG | AI | AK | AM | AO | AQ | AS | AU | AW | AY | BA | BC | BE | BF | BG | BH | BI | BK |
|---|---|---|---|---|---|---|---|---|---|---|---|---|---|---|---|---|---|---|---|---|---|---|---|---|---|---|---|---|---|---|---|---|---|---|---|
| 6754 | 07971 | 06753 | 2 | | 0 | 0 | 0 | 0 | 0 | 0 | 0 | 0 | 0 | 0 | 0 | 0 | 0 | 0 | 0 | 0 | 0 | 0 | 0 | 0 | 2 | 0 | 0 | 0 | 0 | | | | | | |
| 6755 | 07973 | 06754 | 1 | | 0 | 0 | 0 | 0 | 0 | 0 | 0 | 0 | 0 | 0 | 0 | 0 | 0 | 0 | 0 | 0 | 0 | 0 | 0 | 0 | 1 | 0 | 0 | 0 | 0 | | | | | | |
| 6756 | 07974 | 06755 | 1 | | 0 | 0 | 0 | 0 | 0 | 0 | 0 | 0 | 0 | 0 | 0 | 0 | 0 | 0 | 0 | 0 | 0 | 0 | 0 | 0 | 1 | 0 | 0 | 0 | 0 | | | | | | |
| 6757 | 07975 | 06756 | 1 | | 0 | 0 | 0 | 0 | 0 | 0 | 0 | 0 | 0 | 0 | 0 | 0 | 0 | 0 | 0 | 0 | 0 | 0 | 0 | 0 | 1 | 0 | 0 | 0 | 0 | | | | | | |
| 6758 | 07976 | 06757 | 1 | | 0 | 0 | 0 | 0 | 0 | 0 | 0 | 0 | 0 | 0 | 0 | 0 | 0 | 0 | 0 | 0 | 0 | 0 | 0 | 0 | 1 | 0 | 0 | 0 | 0 | | | | | | |
| 6759 | 07977 | 06758 | 1 | | 0 | 0 | 0 | 0 | 0 | 0 | 0 | 0 | 0 | 0 | 0 | 0 | 0 | 0 | 0 | 0 | 0 | 0 | 0 | 0 | 1 | 0 | 0 | 0 | 0 | | | | | | |
| 6760 | 07981 | 06759 | 1 | | 0 | 0 | 0 | 0 | 0 | 0 | 0 | 0 | 0 | 0 | 0 | 0 | 0 | 0 | 0 | 0 | 0 | 0 | 0 | 0 | 1 | 0 | 0 | 0 | 0 | | | | | | |
| 6761 | 07986 | 06760 | 5 | | 0 | 0 | 0 | 0 | 0 | 0 | 0 | 0 | 0 | 0 | 0 | 0 | 0 | 0 | 0 | 0 | 0 | 0 | 0 | 0 | 1 | 1 | 3 | 0 | 0 | | | | | | |
| 6762 | 07987 | 06761 | 1 | | 0 | 0 | 0 | 0 | 0 | 0 | 0 | 0 | 0 | 0 | 0 | 0 | 0 | 0 | 0 | 0 | 0 | 0 | 0 | 0 | 1 | 0 | 0 | 0 | 0 | | | | | | |
| 6763 | 07988 | 06762 | 1 | | 0 | 0 | 0 | 0 | 0 | 0 | 0 | 0 | 0 | 0 | 0 | 0 | 0 | 0 | 0 | 0 | 0 | 0 | 0 | 0 | 1 | 0 | 0 | 0 | 0 | | | | | | |
| 6764 | 07989 | 06763 | 1 | | 0 | 0 | 0 | 0 | 0 | 0 | 0 | 0 | 0 | 0 | 0 | 0 | 0 | 0 | 0 | 0 | 0 | 0 | 0 | 0 | 1 | 0 | 0 | 0 | 0 | | | | | | |
| 6765 | 07990 | 06764 | 1 | | 0 | 0 | 0 | 0 | 0 | 0 | 0 | 0 | 0 | 0 | 0 | 0 | 0 | 0 | 0 | 0 | 0 | 0 | 0 | 0 | 1 | 0 | 0 | 0 | 0 | | | | | | |
| 6766 | 07991 | 06765 | 1 | | 0 | 0 | 0 | 0 | 0 | 0 | 0 | 0 | 0 | 0 | 0 | 0 | 0 | 0 | 0 | 0 | 0 | 0 | 0 | 0 | 1 | 0 | 0 | 0 | 0 | | | | | | |
| 6767 | 07992 | 06766 | 1 | | 0 | 0 | 0 | 0 | 0 | 0 | 0 | 0 | 0 | 0 | 0 | 0 | 0 | 0 | 0 | 0 | 0 | 0 | 0 | 0 | 1 | 0 | 0 | 0 | 0 | | | | | | |
| 6768 | 07994 | 06767 | 1 | | 0 | 0 | 0 | 0 | 0 | 0 | 0 | 0 | 0 | 0 | 0 | 0 | 0 | 0 | 0 | 0 | 0 | 0 | 0 | 0 | 1 | 0 | 0 | 0 | 0 | | | | | | |
| 6769 | 07995 | 06768 | 1 | | 0 | 0 | 0 | 0 | 0 | 0 | 0 | 0 | 0 | 0 | 0 | 0 | 0 | 0 | 0 | 0 | 0 | 0 | 0 | 0 | 1 | 0 | 0 | 0 | 0 | | | | | | |
| 6770 | 07997 | 06769 | 1 | | 0 | 0 | 0 | 0 | 0 | 0 | 0 | 0 | 0 | 0 | 0 | 0 | 0 | 0 | 0 | 0 | 0 | 0 | 0 | 0 | 1 | 0 | 0 | 0 | 0 | | | | | | |
| 6771 | 07998 | 06770 | 1 | | 0 | 0 | 0 | 0 | 0 | 0 | 0 | 0 | 0 | 0 | 0 | 0 | 0 | 0 | 0 | 0 | 0 | 0 | 0 | 0 | 1 | 0 | 0 | 0 | 0 | | | | | | |
| 6772 | 07999 | 06771 | 1 | | 0 | 0 | 0 | 0 | 0 | 0 | 0 | 0 | 0 | 0 | 0 | 0 | 0 | 0 | 0 | 0 | 0 | 0 | 0 | 0 | 1 | 0 | 0 | 0 | 0 | | | | | | |
| 6773 | 08000 | 06772 | 1 | | 0 | 0 | 0 | 0 | 0 | 0 | 0 | 0 | 0 | 0 | 0 | 0 | 0 | 0 | 0 | 0 | 0 | 0 | 0 | 0 | 0 | 1 | 0 | 0 | 0 | | | | | | |
| 6774 | 08001 | 06773 | 1 | | 0 | 0 | 0 | 0 | 0 | 0 | 0 | 0 | 0 | 0 | 0 | 0 | 0 | 0 | 0 | 0 | 0 | 0 | 0 | 0 | 0 | 1 | 0 | 0 | 0 | | | | | | |
| 6775 | 08002 | 06774 | 1 | | 0 | 0 | 0 | 0 | 0 | 0 | 0 | 0 | 0 | 0 | 0 | 0 | 0 | 0 | 0 | 0 | 0 | 0 | 0 | 0 | 0 | 1 | 0 | 0 | 0 | | | | | | |
| 6776 | 08003 | 06775 | 1 | | 0 | 0 | 0 | 0 | 0 | 0 | 0 | 0 | 0 | 0 | 0 | 0 | 0 | 0 | 0 | 0 | 0 | 0 | 0 | 0 | 0 | 1 | 0 | 0 | 0 | | | | | | |
| 6777 | 08004 | 06776 | 4 | | 0 | 0 | 0 | 0 | 0 | 0 | 0 | 0 | 0 | 0 | 0 | 0 | 0 | 0 | 0 | 0 | 0 | 0 | 0 | 0 | 0 | 4 | 0 | 0 | 0 | | | | | | |
| 6778 | 08005 | 06777 | 1 | | 0 | 0 | 0 | 0 | 0 | 0 | 0 | 0 | 0 | 0 | 0 | 0 | 0 | 0 | 0 | 0 | 0 | 0 | 0 | 0 | 0 | 1 | 0 | 0 | 0 | | | | | | |
| 6779 | 08006 | 06778 | 1 | | 0 | 0 | 0 | 0 | 0 | 0 | 0 | 0 | 0 | 0 | 0 | 0 | 0 | 0 | 0 | 0 | 0 | 0 | 0 | 0 | 0 | 1 | 0 | 0 | 0 | | | | | | |
| 6780 | 08007 | 06779 | 1 | | 0 | 0 | 0 | 0 | 0 | 0 | 0 | 0 | 0 | 0 | 0 | 0 | 0 | 0 | 0 | 0 | 0 | 0 | 0 | 0 | 0 | 1 | 0 | 0 | 0 | | | | | | |
| 6781 | 08008 | 06780 | 1 | | 0 | 0 | 0 | 0 | 0 | 0 | 0 | 0 | 0 | 0 | 0 | 0 | 0 | 0 | 0 | 0 | 0 | 0 | 0 | 0 | 0 | 1 | 0 | 0 | 0 | | | | | | |
| 6782 | 08009 | 06781 | 5 | | 0 | 0 | 0 | 0 | 0 | 0 | 0 | 0 | 0 | 0 | 0 | 0 | 0 | 0 | 0 | 0 | 0 | 0 | 0 | 0 | 0 | 5 | 0 | 0 | 0 | | | | | | |
| 6783 | 08010 | 06782 | 2 | | 0 | 0 | 0 | 0 | 0 | 0 | 0 | 0 | 0 | 0 | 0 | 0 | 0 | 0 | 0 | 0 | 0 | 0 | 0 | 0 | 0 | 2 | 0 | 0 | 0 | M85168 | 96.9 | 96 | 1 | 6865 | 6961 |
| 6784 | 08011 | 06783 | 2 | | 0 | 0 | 0 | 0 | 0 | 0 | 0 | 0 | 0 | 0 | 0 | 0 | 0 | 0 | 0 | 0 | 0 | 0 | 0 | 0 | 0 | 1 | 1 | 0 | 0 | U03891 | 100 | 195 | 1 | 491 | 685 |
| 6785 | 08012 | 06784 | 1 | | 0 | 0 | 0 | 0 | 0 | 0 | 0 | 0 | 0 | 0 | 0 | 0 | 0 | 0 | 0 | 0 | 0 | 0 | 0 | 0 | 0 | 1 | 0 | 0 | 0 | | | | | | |
| 6786 | 08013 | 06785 | 2 | | 0 | 0 | 0 | 0 | 0 | 0 | 0 | 0 | 0 | 0 | 0 | 0 | 0 | 0 | 0 | 0 | 0 | 0 | 0 | 0 | 0 | 1 | 1 | 0 | 0 | M63488 | 95.1 | 283 | 1 | 1995 | 2393 |
| 6787 | 08014 | 06786 | 1 | | 0 | 0 | 0 | 0 | 0 | 0 | 0 | 0 | 0 | 0 | 0 | 0 | 0 | 0 | 0 | 0 | 0 | 0 | 0 | 0 | 0 | 1 | 0 | 0 | 0 | | | | | | |
| 6788 | 08015 | 06787 | 1 | | 0 | 0 | 0 | 0 | 0 | 0 | 0 | 0 | 0 | 0 | 0 | 0 | 0 | 0 | 0 | 0 | 0 | 0 | 0 | 0 | 0 | 1 | 0 | 0 | 0 | | | | | | |
| 6789 | 08016 | 06788 | 1 | | 0 | 0 | 0 | 0 | 0 | 0 | 0 | 0 | 0 | 0 | 0 | 0 | 0 | 0 | 0 | 0 | 0 | 0 | 0 | 0 | 0 | 1 | 0 | 0 | 0 | | | | | | |

Table 189

| | A | B | C | E | G | I | K | M | O | Q | S | U | W | Y | AA | AC | AE | AG | AI | AK | AM | AO | AQ | AS | AU | AW | AY | BA | BC | BE | BF | BG | BH | BI | BK |
|---|---|---|---|---|---|---|---|---|---|---|---|---|---|---|---|---|---|---|---|---|---|---|---|---|---|---|---|---|---|---|---|---|---|---|---|
| 6790 | 08017 | 06789 | 3 | | 0 | 0 | 0 | 0 | 0 | 0 | 0 | 0 | 0 | 0 | 0 | 0 | 0 | 0 | 0 | 0 | 0 | 0 | 0 | 0 | 0 | 3 | 0 | 0 | 0 | Z28407 | 92.7 | 300 | 1 | 236 | 852 |
| 6791 | 08018 | 06790 | 1 | | 0 | 0 | 0 | 0 | 0 | 0 | 0 | 0 | 0 | 0 | 0 | 0 | 0 | 0 | 0 | 0 | 0 | 0 | 0 | 0 | 0 | 1 | 0 | 0 | 0 | | | | | | |
| 6792 | 08019 | 06791 | 1 | | 0 | 0 | 0 | 0 | 0 | 0 | 0 | 0 | 0 | 0 | 0 | 0 | 0 | 0 | 0 | 0 | 0 | 0 | 0 | 0 | 0 | 1 | 0 | 0 | 0 | | | | | | |
| 6793 | 08020 | 06792 | 1 | | 0 | 0 | 0 | 0 | 0 | 0 | 0 | 0 | 0 | 0 | 0 | 0 | 0 | 0 | 0 | 0 | 0 | 0 | 0 | 0 | 0 | 1 | 0 | 0 | 0 | | | | | | |
| 6794 | 08021 | 06793 | 1 | | 0 | 0 | 0 | 0 | 0 | 0 | 0 | 0 | 0 | 0 | 0 | 0 | 0 | 0 | 0 | 0 | 0 | 0 | 0 | 0 | 0 | 1 | 0 | 0 | 0 | | | | | | |
| 6795 | 08022 | 06794 | 1 | | 0 | 0 | 0 | 0 | 0 | 0 | 0 | 0 | 0 | 0 | 0 | 0 | 0 | 0 | 0 | 0 | 0 | 0 | 0 | 0 | 0 | 1 | 0 | 0 | 0 | | | | | | |
| 6796 | 08023 | 06795 | 1 | | 0 | 0 | 0 | 0 | 0 | 0 | 0 | 0 | 0 | 0 | 0 | 0 | 0 | 0 | 0 | 0 | 0 | 0 | 0 | 0 | 0 | 1 | 0 | 0 | 0 | | | | | | |
| 6797 | 08024 | 06796 | 1 | | 0 | 0 | 0 | 0 | 0 | 0 | 0 | 0 | 0 | 0 | 0 | 0 | 0 | 0 | 0 | 0 | 0 | 0 | 0 | 0 | 0 | 1 | 0 | 0 | 0 | | | | | | |
| 6798 | 08025 | 06797 | 27 | | 0 | 0 | 0 | 0 | 0 | 0 | 0 | 0 | 0 | 0 | 0 | 0 | 0 | 0 | 0 | 0 | 0 | 0 | 0 | 0 | 0 | 27 | 0 | 0 | 0 | | | | | | |
| 6799 | 08026 | 06798 | 1 | | 0 | 0 | 0 | 0 | 0 | 0 | 0 | 0 | 0 | 0 | 0 | 0 | 0 | 0 | 0 | 0 | 0 | 0 | 0 | 0 | 0 | 1 | 0 | 0 | 0 | | | | | | |
| 6800 | 08027 | 06799 | 2 | | 0 | 0 | 0 | 0 | 0 | 0 | 0 | 0 | 0 | 0 | 0 | 0 | 0 | 0 | 0 | 0 | 0 | 0 | 0 | 0 | 0 | 2 | 0 | 0 | 0 | X65024 | 98.2 | 112 | 1 | 3344 | 3455 |
| 6801 | 08028 | 06800 | 1 | | 0 | 0 | 0 | 0 | 0 | 0 | 0 | 0 | 0 | 0 | 0 | 0 | 0 | 0 | 0 | 0 | 0 | 0 | 0 | 0 | 0 | 1 | 0 | 0 | 0 | | | | | | |
| 6802 | 08029 | 06801 | 1 | | 0 | 0 | 0 | 0 | 0 | 0 | 0 | 0 | 0 | 0 | 0 | 0 | 0 | 0 | 0 | 0 | 0 | 0 | 0 | 0 | 0 | 1 | 0 | 0 | 0 | | | | | | |
| 6803 | 08030 | 06802 | 2 | | 0 | 0 | 0 | 0 | 0 | 0 | 0 | 0 | 0 | 0 | 0 | 0 | 0 | 0 | 0 | 0 | 0 | 0 | 0 | 0 | 0 | 2 | 0 | 0 | 0 | | | | | | |
| 6804 | 08031 | 06803 | 1 | | 0 | 0 | 0 | 0 | 0 | 0 | 0 | 0 | 0 | 0 | 0 | 0 | 0 | 0 | 0 | 0 | 0 | 0 | 0 | 0 | 0 | 1 | 0 | 0 | 0 | | | | | | |
| 6805 | 08032 | 06804 | 1 | | 0 | 0 | 0 | 0 | 0 | 0 | 0 | 0 | 0 | 0 | 0 | 0 | 0 | 0 | 0 | 0 | 0 | 0 | 0 | 0 | 0 | 1 | 0 | 0 | 0 | | | | | | |
| 6806 | 08033 | 06805 | 1 | | 0 | 0 | 0 | 0 | 0 | 0 | 0 | 0 | 0 | 0 | 0 | 0 | 0 | 0 | 0 | 0 | 0 | 0 | 0 | 0 | 0 | 1 | 0 | 0 | 0 | | | | | | |
| 6807 | 08034 | 06806 | 1 | | 0 | 0 | 0 | 0 | 0 | 0 | 0 | 0 | 0 | 0 | 0 | 0 | 0 | 0 | 0 | 0 | 0 | 0 | 0 | 0 | 0 | 1 | 0 | 0 | 0 | | | | | | |
| 6808 | 08036 | 06807 | 1 | | 0 | 0 | 0 | 0 | 0 | 0 | 0 | 0 | 0 | 0 | 0 | 0 | 0 | 0 | 0 | 0 | 0 | 0 | 0 | 0 | 0 | 1 | 0 | 0 | 0 | M14199 | 98.8 | 240 | 32 | 236 | 475 |
| 6809 | 08037 | 06808 | 1 | | 0 | 0 | 0 | 0 | 0 | 0 | 0 | 0 | 0 | 0 | 0 | 0 | 0 | 0 | 0 | 0 | 0 | 0 | 0 | 0 | 0 | 1 | 0 | 0 | 0 | | | | | | |
| 6810 | 08038 | 06809 | 1 | | 0 | 0 | 0 | 0 | 0 | 0 | 0 | 0 | 0 | 0 | 0 | 0 | 0 | 0 | 0 | 0 | 0 | 0 | 0 | 0 | 0 | 1 | 0 | 0 | 0 | | | | | | |
| 6811 | 08039 | 06810 | 1 | | 0 | 0 | 0 | 0 | 0 | 0 | 0 | 0 | 0 | 0 | 0 | 0 | 0 | 0 | 0 | 0 | 0 | 0 | 0 | 0 | 0 | 1 | 0 | 0 | 0 | | | | | | |
| 6812 | 08040 | 06811 | 2 | | 0 | 0 | 0 | 0 | 0 | 0 | 0 | 0 | 0 | 0 | 0 | 0 | 0 | 0 | 0 | 0 | 0 | 0 | 0 | 0 | 0 | 2 | 0 | 0 | 0 | M16453 | 94.4 | 143 | 1 | 172 | 369 |
| 6813 | 08041 | 06812 | 1 | | 0 | 0 | 0 | 0 | 0 | 0 | 0 | 0 | 0 | 0 | 0 | 0 | 0 | 0 | 0 | 0 | 0 | 0 | 0 | 0 | 0 | 1 | 0 | 0 | 0 | | | | | | |
| 6814 | 08042 | 06813 | 1 | | 0 | 0 | 0 | 0 | 0 | 0 | 0 | 0 | 0 | 0 | 0 | 0 | 0 | 0 | 0 | 0 | 0 | 0 | 0 | 0 | 0 | 1 | 0 | 0 | 0 | | | | | | |
| 6815 | 08043 | 06814 | 1 | | 0 | 0 | 0 | 0 | 0 | 0 | 0 | 0 | 0 | 0 | 0 | 0 | 0 | 0 | 0 | 0 | 0 | 0 | 0 | 0 | 0 | 1 | 0 | 0 | 0 | | | | | | |
| 6816 | 08044 | 06815 | 3 | | 0 | 0 | 0 | 0 | 0 | 0 | 0 | 0 | 0 | 0 | 0 | 0 | 0 | 0 | 0 | 0 | 0 | 0 | 0 | 0 | 0 | 1 | 2 | 0 | 0 | M63625 | 91 | 433 | 1 | 1019 | 1684 |
| 6817 | 08045 | 06816 | 1 | | 0 | 0 | 0 | 0 | 0 | 0 | 0 | 0 | 0 | 0 | 0 | 0 | 0 | 0 | 0 | 0 | 0 | 0 | 0 | 0 | 0 | 1 | 0 | 0 | 0 | M92439 | 96.5 | 229 | 1 | 3739 | 4782 |
| 6818 | 08046 | 06817 | 2 | | 0 | 0 | 0 | 0 | 0 | 0 | 0 | 0 | 0 | 0 | 0 | 0 | 0 | 0 | 0 | 0 | 0 | 0 | 0 | 0 | 0 | 2 | 0 | 0 | 0 | | | | | | |
| 6819 | 08047 | 06818 | 1 | | 0 | 0 | 0 | 0 | 0 | 0 | 0 | 0 | 0 | 0 | 0 | 0 | 0 | 0 | 0 | 0 | 0 | 0 | 0 | 0 | 0 | 1 | 0 | 0 | 0 | | | | | | |
| 6820 | 08048 | 06819 | 1 | | 0 | 0 | 0 | 0 | 0 | 0 | 0 | 0 | 0 | 0 | 0 | 0 | 0 | 0 | 0 | 0 | 0 | 0 | 0 | 0 | 0 | 1 | 0 | 0 | 0 | | | | | | |
| 6821 | 08049 | 06820 | 1 | | 0 | 0 | 0 | 0 | 0 | 0 | 0 | 0 | 0 | 0 | 0 | 0 | 0 | 0 | 0 | 0 | 0 | 0 | 0 | 0 | 0 | 1 | 0 | 0 | 0 | | | | | | |
| 6822 | 08050 | 06821 | 1 | | 0 | 0 | 0 | 0 | 0 | 0 | 0 | 0 | 0 | 0 | 0 | 0 | 0 | 0 | 0 | 0 | 0 | 0 | 0 | 0 | 0 | 1 | 0 | 0 | 0 | | | | | | |
| 6823 | 08051 | 06822 | 2 | | 0 | 0 | 0 | 0 | 0 | 0 | 0 | 0 | 0 | 0 | 0 | 0 | 0 | 0 | 0 | 0 | 0 | 0 | 0 | 0 | 0 | 2 | 0 | 0 | 0 | M86849 | 98.1 | 157 | 1 | 2087 | 2312 |
| 6824 | 08052 | 06823 | 1 | | 0 | 0 | 0 | 0 | 0 | 0 | 0 | 0 | 0 | 0 | 0 | 0 | 0 | 0 | 0 | 0 | 0 | 0 | 0 | 0 | 0 | 1 | 0 | 0 | 0 | | | | | | |
| 6825 | 08053 | 06824 | 1 | | 0 | 0 | 0 | 0 | 0 | 0 | 0 | 0 | 0 | 0 | 0 | 0 | 0 | 0 | 0 | 0 | 0 | 0 | 0 | 0 | 0 | 1 | 0 | 0 | 0 | | | | | | |

Table 190

| | A | B | C | E | G | I | K | M | O | Q | S | U | W | Y | AA | AC | AE | AG | AI | AK | AM | AO | AQ | AS | AU | AW | AY | BA | BC | BE | BF | BG | BH | BI | BK |
|---|---|---|---|---|---|---|---|---|---|---|---|---|---|---|---|---|---|---|---|---|---|---|---|---|---|---|---|---|---|---|---|---|---|---|---|
| 6826 | 08054 | 06825 | 1 | | 0 | 0 | 0 | 0 | 0 | 0 | 0 | 0 | 0 | 0 | 0 | 0 | 0 | 0 | 0 | 0 | 0 | 0 | 0 | 0 | 0 | 1 | 0 | 0 | 0 | | | | | | |
| 6827 | 08055 | 06826 | 7 | | 0 | 0 | 0 | 0 | 0 | 0 | 0 | 0 | 0 | 0 | 0 | 0 | 0 | 0 | 0 | 0 | 0 | 0 | 0 | 0 | 0 | 2 | 0 | 5 | 0 | | | | | | |
| 6828 | 08056 | 06827 | 4 | | 0 | 0 | 0 | 0 | 0 | 0 | 0 | 0 | 0 | 0 | 0 | 0 | 0 | 0 | 0 | 0 | 0 | 0 | 0 | 0 | 0 | 1 | 3 | 0 | 0 | L13463 | 99.5 | 207 | 1 | 1139 | 1345 |
| 6829 | 08057 | 06828 | 1 | | 0 | 0 | 0 | 0 | 0 | 0 | 0 | 0 | 0 | 0 | 0 | 0 | 0 | 0 | 0 | 0 | 0 | 0 | 0 | 0 | 0 | 1 | 0 | 0 | 0 | | | | | | |
| 6830 | 08058 | 06829 | 1 | | 0 | 0 | 0 | 0 | 0 | 0 | 0 | 0 | 0 | 0 | 0 | 0 | 0 | 0 | 0 | 0 | 0 | 0 | 0 | 0 | 0 | 1 | 0 | 0 | 0 | | | | | | |
| 6831 | 08059 | 06830 | 1 | | 0 | 0 | 0 | 0 | 0 | 0 | 0 | 0 | 0 | 0 | 0 | 0 | 0 | 0 | 0 | 0 | 0 | 0 | 0 | 0 | 0 | 1 | 0 | 0 | 0 | | | | | | |
| 6832 | 08060 | 06831 | 1 | | 0 | 0 | 0 | 0 | 0 | 0 | 0 | 0 | 0 | 0 | 0 | 0 | 0 | 0 | 0 | 0 | 0 | 0 | 0 | 0 | 0 | 1 | 0 | 0 | 0 | | | | | | |
| 6833 | 08061 | 06832 | 1 | | 0 | 0 | 0 | 0 | 0 | 0 | 0 | 0 | 0 | 0 | 0 | 0 | 0 | 0 | 0 | 0 | 0 | 0 | 0 | 0 | 0 | 1 | 0 | 0 | 0 | | | | | | |
| 6834 | 08062 | 06833 | 1 | | 0 | 0 | 0 | 0 | 0 | 0 | 0 | 0 | 0 | 0 | 0 | 0 | 0 | 0 | 0 | 0 | 0 | 0 | 0 | 0 | 0 | 1 | 0 | 0 | 0 | | | | | | |
| 6835 | 08063 | 06834 | 1 | | 0 | 0 | 0 | 0 | 0 | 0 | 0 | 0 | 0 | 0 | 0 | 0 | 0 | 0 | 0 | 0 | 0 | 0 | 0 | 0 | 0 | 1 | 0 | 0 | 0 | | | | | | |
| 6836 | 08064 | 06835 | 1 | | 0 | 0 | 0 | 0 | 0 | 0 | 0 | 0 | 0 | 0 | 0 | 0 | 0 | 0 | 0 | 0 | 0 | 0 | 0 | 0 | 0 | 1 | 0 | 0 | 0 | M10036 | 93.2 | 103 | 1 | 1462 | 1835 |
| 6837 | 08065 | 06836 | 1 | | 0 | 0 | 0 | 0 | 0 | 0 | 0 | 0 | 0 | 0 | 0 | 0 | 0 | 0 | 0 | 0 | 0 | 0 | 0 | 0 | 0 | 1 | 0 | 0 | 0 | | | | | | |
| 6838 | 08066 | 06837 | 1 | | 0 | 0 | 0 | 0 | 0 | 0 | 0 | 0 | 0 | 0 | 0 | 0 | 0 | 0 | 0 | 0 | 0 | 0 | 0 | 0 | 0 | 1 | 0 | 0 | 0 | | | | | | |
| 6839 | 08067 | 06838 | 9 | | 0 | 0 | 0 | 0 | 0 | 0 | 0 | 0 | 0 | 0 | 0 | 0 | 0 | 0 | 0 | 0 | 0 | 0 | 0 | 0 | 0 | 9 | 0 | 0 | 0 | | | | | | |
| 6840 | 08068 | 06839 | 1 | | 0 | 0 | 0 | 0 | 0 | 0 | 0 | 0 | 0 | 0 | 0 | 0 | 0 | 0 | 0 | 0 | 0 | 0 | 0 | 0 | 0 | 1 | 0 | 0 | 0 | | | | | | |
| 6841 | 08069 | 06840 | 1 | | 0 | 0 | 0 | 0 | 0 | 0 | 0 | 0 | 0 | 0 | 0 | 0 | 0 | 0 | 0 | 0 | 0 | 0 | 0 | 0 | 0 | 1 | 0 | 0 | 0 | | | | | | |
| 6842 | 08070 | 06841 | 1 | | 0 | 0 | 0 | 0 | 0 | 0 | 0 | 0 | 0 | 0 | 0 | 0 | 0 | 0 | 0 | 0 | 0 | 0 | 0 | 0 | 0 | 1 | 0 | 0 | 0 | | | | | | |
| 6843 | 08071 | 06842 | 1 | | 0 | 0 | 0 | 0 | 0 | 0 | 0 | 0 | 0 | 0 | 0 | 0 | 0 | 0 | 0 | 0 | 0 | 0 | 0 | 0 | 0 | 1 | 0 | 0 | 0 | | | | | | |
| 6844 | 08073 | 06843 | 1 | | 0 | 0 | 0 | 0 | 0 | 0 | 0 | 0 | 0 | 0 | 0 | 0 | 0 | 0 | 0 | 0 | 0 | 0 | 0 | 0 | 0 | 1 | 0 | 0 | 0 | | | | | | |
| 6845 | 08074 | 06844 | 2 | | 0 | 0 | 0 | 0 | 0 | 0 | 0 | 0 | 0 | 0 | 0 | 0 | 0 | 0 | 0 | 0 | 0 | 0 | 0 | 0 | 0 | 2 | 0 | 0 | 0 | | | | | | |
| 6846 | 08075 | 06845 | 1 | | 0 | 0 | 0 | 0 | 0 | 0 | 0 | 0 | 0 | 0 | 0 | 0 | 0 | 0 | 0 | 0 | 0 | 0 | 0 | 0 | 0 | 1 | 0 | 0 | 0 | | | | | | |
| 6847 | 08076 | 06846 | 1 | | 0 | 0 | 0 | 0 | 0 | 0 | 0 | 0 | 0 | 0 | 0 | 0 | 0 | 0 | 0 | 0 | 0 | 0 | 0 | 0 | 0 | 1 | 0 | 0 | 0 | | | | | | |
| 6848 | 08077 | 06847 | 1 | | 0 | 0 | 0 | 0 | 0 | 0 | 0 | 0 | 0 | 0 | 0 | 0 | 0 | 0 | 0 | 0 | 0 | 0 | 0 | 0 | 0 | 1 | 0 | 0 | 0 | | | | | | |
| 6849 | 08078 | 06848 | 1 | | 0 | 0 | 0 | 0 | 0 | 0 | 0 | 0 | 0 | 0 | 0 | 0 | 0 | 0 | 0 | 0 | 0 | 0 | 0 | 0 | 0 | 1 | 0 | 0 | 0 | | | | | | |
| 6850 | 08079 | 06849 | 1 | | 0 | 0 | 0 | 0 | 0 | 0 | 0 | 0 | 0 | 0 | 0 | 0 | 0 | 0 | 0 | 0 | 0 | 0 | 0 | 0 | 0 | 1 | 0 | 0 | 0 | | | | | | |
| 6851 | 08080 | 06850 | 1 | | 0 | 0 | 0 | 0 | 0 | 0 | 0 | 0 | 0 | 0 | 0 | 0 | 0 | 0 | 0 | 0 | 0 | 0 | 0 | 0 | 0 | 1 | 0 | 0 | 0 | | | | | | |
| 6852 | 08081 | 06851 | 1 | | 0 | 0 | 0 | 0 | 0 | 0 | 0 | 0 | 0 | 0 | 0 | 0 | 0 | 0 | 0 | 0 | 0 | 0 | 0 | 0 | 0 | 1 | 0 | 0 | 0 | | | | | | |
| 6853 | 08082 | 06852 | 1 | | 0 | 0 | 0 | 0 | 0 | 0 | 0 | 0 | 0 | 0 | 0 | 0 | 0 | 0 | 0 | 0 | 0 | 0 | 0 | 0 | 0 | 1 | 0 | 0 | 0 | | | | | | |
| 6854 | 08083 | 06853 | 2 | | 0 | 0 | 0 | 0 | 0 | 0 | 0 | 0 | 0 | 0 | 0 | 0 | 0 | 0 | 0 | 0 | 0 | 0 | 0 | 0 | 0 | 1 | 0 | 0 | 1 | | | | | | |
| 6855 | 08084 | 06854 | 1 | | 0 | 0 | 0 | 0 | 0 | 0 | 0 | 0 | 0 | 0 | 0 | 0 | 0 | 0 | 0 | 0 | 0 | 0 | 0 | 0 | 0 | 1 | 0 | 0 | 0 | | | | | | |
| 6856 | 08085 | 06855 | 2 | | 0 | 0 | 0 | 0 | 0 | 0 | 0 | 0 | 0 | 0 | 0 | 0 | 0 | 0 | 0 | 0 | 0 | 0 | 0 | 0 | 0 | 2 | 0 | 0 | 0 | | | | | | |
| 6857 | 08086 | 06856 | 1 | | 0 | 0 | 0 | 0 | 0 | 0 | 0 | 0 | 0 | 0 | 0 | 0 | 0 | 0 | 0 | 0 | 0 | 0 | 0 | 0 | 0 | 1 | 0 | 0 | 0 | | | | | | |
| 6858 | 08087 | 06857 | 1 | | 0 | 0 | 0 | 0 | 0 | 0 | 0 | 0 | 0 | 0 | 0 | 0 | 0 | 0 | 0 | 0 | 0 | 0 | 0 | 0 | 0 | 1 | 0 | 0 | 0 | | | | | | |
| 6859 | 08088 | 06858 | 1 | | 0 | 0 | 0 | 0 | 0 | 0 | 0 | 0 | 0 | 0 | 0 | 0 | 0 | 0 | 0 | 0 | 0 | 0 | 0 | 0 | 0 | 1 | 0 | 0 | 0 | | | | | | |
| 6860 | 08089 | 06859 | 1 | | 0 | 0 | 0 | 0 | 0 | 0 | 0 | 0 | 0 | 0 | 0 | 0 | 0 | 0 | 0 | 0 | 0 | 0 | 0 | 0 | 0 | 1 | 0 | 0 | 0 | X13425 | 94.4 | 250 | 1 | 1364 | 1793 |
| 6861 | 08090 | 06860 | 1 | | 0 | 0 | 0 | 0 | 0 | 0 | 0 | 0 | 0 | 0 | 0 | 0 | 0 | 0 | 0 | 0 | 0 | 0 | 0 | 0 | 0 | 1 | 0 | 0 | 0 | | | | | | |

Table 191

| | A | B | C | E | G | I | K | M | O | Q | S | U | W | Y | AA | AC | AE | AG | AI | AK | AM | AO | AQ | AS | AU | AW | AY | BA | BC | BE | BF | BG | BH | BI | BK |
|---|---|---|---|---|---|---|---|---|---|---|---|---|---|---|---|---|---|---|---|---|---|---|---|---|---|---|---|---|---|---|---|---|---|---|---|
| 6862 | 08091 | 06861 | 1 | | 0 | 0 | 0 | 0 | 0 | 0 | 0 | 0 | 0 | 0 | 0 | 0 | 0 | 0 | 0 | 0 | 0 | 0 | 0 | 0 | 0 | 1 | 0 | 0 | 0 | | | | | | |
| 6863 | 08092 | 06862 | 1 | | 0 | 0 | 0 | 0 | 0 | 0 | 0 | 0 | 0 | 0 | 0 | 0 | 0 | 0 | 0 | 0 | 0 | 0 | 0 | 0 | 0 | 1 | 0 | 0 | 0 | D13627 | 97.8 | 225 | 1 | 1597 | 1821 |
| 6864 | 08093 | 06863 | 1 | | 0 | 0 | 0 | 0 | 0 | 0 | 0 | 0 | 0 | 0 | 0 | 0 | 0 | 0 | 0 | 0 | 0 | 0 | 0 | 0 | 0 | 1 | 0 | 0 | 0 | | | | | | |
| 6865 | 08094 | 06864 | 1 | | 0 | 0 | 0 | 0 | 0 | 0 | 0 | 0 | 0 | 0 | 0 | 0 | 0 | 0 | 0 | 0 | 0 | 0 | 0 | 0 | 0 | 1 | 0 | 0 | 0 | | | | | | |
| 6866 | 08095 | 06865 | 4 | | 0 | 0 | 0 | 0 | 0 | 0 | 0 | 0 | 0 | 0 | 0 | 0 | 0 | 0 | 0 | 0 | 0 | 0 | 0 | 0 | 0 | 4 | 0 | 0 | 0 | | | | | | |
| 6867 | 08096 | 06866 | 1 | | 0 | 0 | 0 | 0 | 0 | 0 | 0 | 0 | 0 | 0 | 0 | 0 | 0 | 0 | 0 | 0 | 0 | 0 | 0 | 0 | 0 | 1 | 0 | 0 | 0 | | | | | | |
| 6868 | 08097 | 06867 | 1 | | 0 | 0 | 0 | 0 | 0 | 0 | 0 | 0 | 0 | 0 | 0 | 0 | 0 | 0 | 0 | 0 | 0 | 0 | 0 | 0 | 0 | 1 | 0 | 0 | 0 | | | | | | |
| 6869 | 08098 | 06868 | 1 | | 0 | 0 | 0 | 0 | 0 | 0 | 0 | 0 | 0 | 0 | 0 | 0 | 0 | 0 | 0 | 0 | 0 | 0 | 0 | 0 | 0 | 1 | 0 | 0 | 0 | | | | | | |
| 6870 | 08099 | 06869 | 1 | | 0 | 0 | 0 | 0 | 0 | 0 | 0 | 0 | 0 | 0 | 0 | 0 | 0 | 0 | 0 | 0 | 0 | 0 | 0 | 0 | 0 | 1 | 0 | 0 | 0 | | | | | | |
| 6871 | 08100 | 06870 | 1 | | 0 | 0 | 0 | 0 | 0 | 0 | 0 | 0 | 0 | 0 | 0 | 0 | 0 | 0 | 0 | 0 | 0 | 0 | 0 | 0 | 0 | 1 | 0 | 0 | 0 | | | | | | |
| 6872 | 08101 | 06871 | 1 | | 0 | 0 | 0 | 0 | 0 | 0 | 0 | 0 | 0 | 0 | 0 | 0 | 0 | 0 | 0 | 0 | 0 | 0 | 0 | 0 | 0 | 1 | 0 | 0 | 0 | | | | | | |
| 6873 | 08102 | 06872 | 1 | | 0 | 0 | 0 | 0 | 0 | 0 | 0 | 0 | 0 | 0 | 0 | 0 | 0 | 0 | 0 | 0 | 0 | 0 | 0 | 0 | 0 | 1 | 0 | 0 | 0 | | | | | | |
| 6874 | 08103 | 06873 | 2 | | 0 | 0 | 0 | 0 | 0 | 0 | 0 | 0 | 0 | 0 | 0 | 0 | 0 | 0 | 0 | 0 | 0 | 0 | 0 | 0 | 0 | 2 | 0 | 0 | 0 | | | | | | |
| 6875 | 08104 | 06874 | 1 | | 0 | 0 | 0 | 0 | 0 | 0 | 0 | 0 | 0 | 0 | 0 | 0 | 0 | 0 | 0 | 0 | 0 | 0 | 0 | 0 | 0 | 1 | 0 | 0 | 0 | | | | | | |
| 6876 | 08105 | 06875 | 2 | | 0 | 0 | 0 | 0 | 0 | 0 | 0 | 0 | 0 | 0 | 0 | 0 | 0 | 0 | 0 | 0 | 0 | 0 | 0 | 0 | 0 | 2 | 0 | 0 | 0 | X07695 | 100 | 165 | 1 | 1582 | 1746 |
| 6877 | 08107 | 06876 | 1 | | 0 | 0 | 0 | 0 | 0 | 0 | 0 | 0 | 0 | 0 | 0 | 0 | 0 | 0 | 0 | 0 | 0 | 0 | 0 | 0 | 0 | 1 | 0 | 0 | 0 | | | | | | |
| 6878 | 08108 | 06877 | 1 | | 0 | 0 | 0 | 0 | 0 | 0 | 0 | 0 | 0 | 0 | 0 | 0 | 0 | 0 | 0 | 0 | 0 | 0 | 0 | 0 | 0 | 1 | 0 | 0 | 0 | | | | | | |
| 6879 | 08109 | 06878 | 1 | | 0 | 0 | 0 | 0 | 0 | 0 | 0 | 0 | 0 | 0 | 0 | 0 | 0 | 0 | 0 | 0 | 0 | 0 | 0 | 0 | 0 | 1 | 0 | 0 | 0 | | | | | | |
| 6880 | 08110 | 06879 | 1 | | 0 | 0 | 0 | 0 | 0 | 0 | 0 | 0 | 0 | 0 | 0 | 0 | 0 | 0 | 0 | 0 | 0 | 0 | 0 | 0 | 0 | 1 | 0 | 0 | 0 | | | | | | |
| 6881 | 08111 | 06880 | 1 | | 0 | 0 | 0 | 0 | 0 | 0 | 0 | 0 | 0 | 0 | 0 | 0 | 0 | 0 | 0 | 0 | 0 | 0 | 0 | 0 | 0 | 1 | 0 | 0 | 0 | | | | | | |
| 6882 | 08112 | 06881 | 1 | | 0 | 0 | 0 | 0 | 0 | 0 | 0 | 0 | 0 | 0 | 0 | 0 | 0 | 0 | 0 | 0 | 0 | 0 | 0 | 0 | 0 | 1 | 0 | 0 | 0 | | | | | | |
| 6883 | 08113 | 06882 | 1 | | 0 | 0 | 0 | 0 | 0 | 0 | 0 | 0 | 0 | 0 | 0 | 0 | 0 | 0 | 0 | 0 | 0 | 0 | 0 | 0 | 0 | 1 | 0 | 0 | 0 | | | | | | |
| 6884 | 08114 | 06883 | 3 | | 0 | 0 | 0 | 0 | 0 | 0 | 0 | 0 | 0 | 0 | 0 | 0 | 0 | 0 | 0 | 0 | 0 | 0 | 0 | 0 | 0 | 3 | 0 | 0 | 0 | Y00503 | 92.8 | 166 | 1 | 1197 | 1360 |
| 6885 | 08115 | 06884 | 1 | | 0 | 0 | 0 | 0 | 0 | 0 | 0 | 0 | 0 | 0 | 0 | 0 | 0 | 0 | 0 | 0 | 0 | 0 | 0 | 0 | 0 | 1 | 0 | 0 | 0 | | | | | | |
| 6886 | 08116 | 06885 | 2 | | 0 | 0 | 0 | 0 | 0 | 0 | 0 | 0 | 0 | 0 | 0 | 0 | 0 | 0 | 0 | 0 | 0 | 0 | 0 | 0 | 0 | 1 | 0 | 1 | 0 | | | | | | |
| 6887 | 08117 | 06886 | 2 | | 0 | 0 | 0 | 0 | 0 | 0 | 0 | 0 | 0 | 0 | 0 | 0 | 0 | 0 | 0 | 0 | 0 | 0 | 0 | 0 | 0 | 1 | 1 | 0 | 0 | | | | | | |
| 6888 | 08118 | 06887 | 3 | | 0 | 0 | 0 | 0 | 0 | 0 | 0 | 0 | 0 | 0 | 0 | 0 | 0 | 0 | 0 | 0 | 0 | 0 | 0 | 0 | 0 | 3 | 0 | 0 | 0 | | | | | | |
| 6889 | 08119 | 06888 | 1 | | 0 | 0 | 0 | 0 | 0 | 0 | 0 | 0 | 0 | 0 | 0 | 0 | 0 | 0 | 0 | 0 | 0 | 0 | 0 | 0 | 0 | 1 | 0 | 0 | 0 | | | | | | |
| 6890 | 08120 | 06889 | 1 | | 0 | 0 | 0 | 0 | 0 | 0 | 0 | 0 | 0 | 0 | 0 | 0 | 0 | 0 | 0 | 0 | 0 | 0 | 0 | 0 | 0 | 1 | 0 | 0 | 0 | | | | | | |
| 6891 | 08121 | 06890 | 1 | | 0 | 0 | 0 | 0 | 0 | 0 | 0 | 0 | 0 | 0 | 0 | 0 | 0 | 0 | 0 | 0 | 0 | 0 | 0 | 0 | 0 | 1 | 0 | 0 | 0 | M80482 | 99.5 | 221 | 1 | 4183 | 4403 |
| 6892 | 08122 | 06891 | 5 | | 0 | 0 | 0 | 0 | 0 | 0 | 0 | 0 | 0 | 0 | 0 | 0 | 0 | 0 | 0 | 0 | 0 | 0 | 0 | 0 | 0 | 5 | 0 | 0 | 0 | | | | | | |
| 6893 | 08123 | 06892 | 1 | | 0 | 0 | 0 | 0 | 0 | 0 | 0 | 0 | 0 | 0 | 0 | 0 | 0 | 0 | 0 | 0 | 0 | 0 | 0 | 0 | 0 | 1 | 0 | 0 | 0 | | | | | | |
| 6894 | 08124 | 06893 | 2 | | 0 | 0 | 0 | 0 | 0 | 0 | 0 | 0 | 0 | 0 | 0 | 0 | 0 | 0 | 0 | 0 | 0 | 0 | 0 | 0 | 0 | 2 | 0 | 0 | 0 | | | | | | |
| 6895 | 08125 | 06894 | 1 | | 0 | 0 | 0 | 0 | 0 | 0 | 0 | 0 | 0 | 0 | 0 | 0 | 0 | 0 | 0 | 0 | 0 | 0 | 0 | 0 | 0 | 1 | 0 | 0 | 0 | | | | | | |
| 6896 | 08126 | 06895 | 1 | | 0 | 0 | 0 | 0 | 0 | 0 | 0 | 0 | 0 | 0 | 0 | 0 | 0 | 0 | 0 | 0 | 0 | 0 | 0 | 0 | 0 | 1 | 0 | 0 | 0 | | | | | | |
| 6897 | 08127 | 06896 | 1 | | 0 | 0 | 0 | 0 | 0 | 0 | 0 | 0 | 0 | 0 | 0 | 0 | 0 | 0 | 0 | 0 | 0 | 0 | 0 | 0 | 0 | 1 | 0 | 0 | 0 | | | | | | |

Table 192

| | A | B | C | E | G | I | K | M | O | Q | S | U | W | Y | AA | AC | AE | AG | AI | AK | AM | AO | AQ | AS | AU | AW | AY | BA | BC | BE | BF | BG | BH | BI | BK |
|---|---|---|---|---|---|---|---|---|---|---|---|---|---|---|---|---|---|---|---|---|---|---|---|---|---|---|---|---|---|---|---|---|---|---|---|
| 6898 | 08128 | 06897 | 1 | | 0 | 0 | 0 | 0 | 0 | 0 | 0 | 0 | 0 | 0 | 0 | 0 | 0 | 0 | 0 | 0 | 0 | 0 | 0 | 0 | 0 | 1 | 0 | 0 | 0 | | | | | | |
| 6899 | 08129 | 06898 | 1 | | 0 | 0 | 0 | 0 | 0 | 0 | 0 | 0 | 0 | 0 | 0 | 0 | 0 | 0 | 0 | 0 | 0 | 0 | 0 | 0 | 0 | 1 | 0 | 0 | 0 | | | | | | |
| 6900 | 08130 | 06899 | 1 | | 0 | 0 | 0 | 0 | 0 | 0 | 0 | 0 | 0 | 0 | 0 | 0 | 0 | 0 | 0 | 0 | 0 | 0 | 0 | 0 | 0 | 1 | 0 | 0 | 0 | | | | | | |
| 6901 | 08131 | 06900 | 1 | | 0 | 0 | 0 | 0 | 0 | 0 | 0 | 0 | 0 | 0 | 0 | 0 | 0 | 0 | 0 | 0 | 0 | 0 | 0 | 0 | 0 | 1 | 0 | 0 | 0 | | | | | | |
| 6902 | 08132 | 06901 | 1 | | 0 | 0 | 0 | 0 | 0 | 0 | 0 | 0 | 0 | 0 | 0 | 0 | 0 | 0 | 0 | 0 | 0 | 0 | 0 | 0 | 0 | 1 | 0 | 0 | 0 | | | | | | |
| 6903 | 08133 | 06902 | 1 | | 0 | 0 | 0 | 0 | 0 | 0 | 0 | 0 | 0 | 0 | 0 | 0 | 0 | 0 | 0 | 0 | 0 | 0 | 0 | 0 | 0 | 1 | 0 | 0 | 0 | | | | | | |
| 6904 | 08134 | 06903 | 1 | | 0 | 0 | 0 | 0 | 0 | 0 | 0 | 0 | 0 | 0 | 0 | 0 | 0 | 0 | 0 | 0 | 0 | 0 | 0 | 0 | 0 | 1 | 0 | 0 | 0 | X57500 | 98.9 | 90 | 1 | 2021 | 2969 |
| 6905 | 08135 | 06904 | 2 | | 0 | 0 | 0 | 0 | 0 | 0 | 0 | 0 | 0 | 0 | 0 | 0 | 0 | 0 | 0 | 0 | 0 | 0 | 0 | 0 | 0 | 2 | 0 | 0 | 0 | | | | | | |
| 6906 | 08136 | 06905 | 1 | | 0 | 0 | 0 | 0 | 0 | 0 | 0 | 0 | 0 | 0 | 0 | 0 | 0 | 0 | 0 | 0 | 0 | 0 | 0 | 0 | 0 | 1 | 0 | 0 | 0 | | | | | | |
| 6907 | 08137 | 06906 | 1 | | 0 | 0 | 0 | 0 | 0 | 0 | 0 | 0 | 0 | 0 | 0 | 0 | 0 | 0 | 0 | 0 | 0 | 0 | 0 | 0 | 0 | 1 | 0 | 0 | 0 | | | | | | |
| 6908 | 08138 | 06907 | 1 | | 0 | 0 | 0 | 0 | 0 | 0 | 0 | 0 | 0 | 0 | 0 | 0 | 0 | 0 | 0 | 0 | 0 | 0 | 0 | 0 | 0 | 1 | 0 | 0 | 0 | | | | | | |
| 6909 | 08139 | 06908 | 1 | | 0 | 0 | 0 | 0 | 0 | 0 | 0 | 0 | 0 | 0 | 0 | 0 | 0 | 0 | 0 | 0 | 0 | 0 | 0 | 0 | 0 | 1 | 0 | 0 | 0 | | | | | | |
| 6910 | 08140 | 06909 | 1 | | 0 | 0 | 0 | 0 | 0 | 0 | 0 | 0 | 0 | 0 | 0 | 0 | 0 | 0 | 0 | 0 | 0 | 0 | 0 | 0 | 0 | 1 | 0 | 0 | 0 | | | | | | |
| 6911 | 08141 | 06910 | 1 | | 0 | 0 | 0 | 0 | 0 | 0 | 0 | 0 | 0 | 0 | 0 | 0 | 0 | 0 | 0 | 0 | 0 | 0 | 0 | 0 | 0 | 1 | 0 | 0 | 0 | | | | | | |
| 6912 | 08142 | 06911 | 1 | | 0 | 0 | 0 | 0 | 0 | 0 | 0 | 0 | 0 | 0 | 0 | 0 | 0 | 0 | 0 | 0 | 0 | 0 | 0 | 0 | 0 | 1 | 0 | 0 | 0 | | | | | | |
| 6913 | 08143 | 06912 | 1 | | 0 | 0 | 0 | 0 | 0 | 0 | 0 | 0 | 0 | 0 | 0 | 0 | 0 | 0 | 0 | 0 | 0 | 0 | 0 | 0 | 0 | 1 | 0 | 0 | 0 | | | | | | |
| 6914 | 08144 | 06913 | 1 | | 0 | 0 | 0 | 0 | 0 | 0 | 0 | 0 | 0 | 0 | 0 | 0 | 0 | 0 | 0 | 0 | 0 | 0 | 0 | 0 | 0 | 1 | 0 | 0 | 0 | | | | | | |
| 6915 | 08145 | 06914 | 1 | | 0 | 0 | 0 | 0 | 0 | 0 | 0 | 0 | 0 | 0 | 0 | 0 | 0 | 0 | 0 | 0 | 0 | 0 | 0 | 0 | 0 | 1 | 0 | 0 | 0 | | | | | | |
| 6916 | 08146 | 06915 | 2 | | 0 | 0 | 0 | 0 | 0 | 0 | 0 | 0 | 0 | 0 | 0 | 0 | 0 | 0 | 0 | 0 | 0 | 0 | 0 | 0 | 0 | 2 | 0 | 0 | 0 | D10923 | 97.7 | 298 | 1 | 1454 | 2051 |
| 6917 | 08147 | 06916 | 1 | | 0 | 0 | 0 | 0 | 0 | 0 | 0 | 0 | 0 | 0 | 0 | 0 | 0 | 0 | 0 | 0 | 0 | 0 | 0 | 0 | 0 | 1 | 0 | 0 | 0 | X59303 | 98.5 | 68 | 1 | 4016 | 4107 |
| 6918 | 08148 | 06917 | 1 | | 0 | 0 | 0 | 0 | 0 | 0 | 0 | 0 | 0 | 0 | 0 | 0 | 0 | 0 | 0 | 0 | 0 | 0 | 0 | 0 | 0 | 1 | 0 | 0 | 0 | | | | | | |
| 6919 | 08149 | 06918 | 1 | | 0 | 0 | 0 | 0 | 0 | 0 | 0 | 0 | 0 | 0 | 0 | 0 | 0 | 0 | 0 | 0 | 0 | 0 | 0 | 0 | 0 | 1 | 0 | 0 | 0 | | | | | | |
| 6920 | 08150 | 06919 | 1 | | 0 | 0 | 0 | 0 | 0 | 0 | 0 | 0 | 0 | 0 | 0 | 0 | 0 | 0 | 0 | 0 | 0 | 0 | 0 | 0 | 0 | 1 | 0 | 0 | 0 | M81457 | 97.4 | 77 | 199 | 513 | 609 |
| 6921 | 08151 | 06920 | 1 | | 0 | 0 | 0 | 0 | 0 | 0 | 0 | 0 | 0 | 0 | 0 | 0 | 0 | 0 | 0 | 0 | 0 | 0 | 0 | 0 | 0 | 1 | 0 | 0 | 0 | | | | | | |
| 6922 | 08153 | 06921 | 1 | | 0 | 0 | 0 | 0 | 0 | 0 | 0 | 0 | 0 | 0 | 0 | 0 | 0 | 0 | 0 | 0 | 0 | 0 | 0 | 0 | 0 | 1 | 0 | 0 | 0 | | | | | | |
| 6923 | 08154 | 06922 | 2 | | 0 | 0 | 0 | 0 | 0 | 0 | 0 | 0 | 0 | 0 | 0 | 0 | 0 | 0 | 0 | 0 | 0 | 0 | 0 | 0 | 0 | 1 | 1 | 0 | 0 | | | | | | |
| 6924 | 08155 | 06923 | 1 | | 0 | 0 | 0 | 0 | 0 | 0 | 0 | 0 | 0 | 0 | 0 | 0 | 0 | 0 | 0 | 0 | 0 | 0 | 0 | 0 | 0 | 1 | 0 | 0 | 0 | | | | | | |
| 6925 | 08156 | 06924 | 1 | | 0 | 0 | 0 | 0 | 0 | 0 | 0 | 0 | 0 | 0 | 0 | 0 | 0 | 0 | 0 | 0 | 0 | 0 | 0 | 0 | 0 | 1 | 0 | 0 | 0 | | | | | | |
| 6926 | 08157 | 06925 | 2 | | 0 | 0 | 0 | 0 | 0 | 0 | 0 | 0 | 0 | 0 | 0 | 0 | 0 | 0 | 0 | 0 | 0 | 0 | 0 | 0 | 0 | 1 | 0 | 1 | 0 | | | | | | |
| 6927 | 08158 | 06926 | 1 | | 0 | 0 | 0 | 0 | 0 | 0 | 0 | 0 | 0 | 0 | 0 | 0 | 0 | 0 | 0 | 0 | 0 | 0 | 0 | 0 | 0 | 1 | 0 | 0 | 0 | | | | | | |
| 6928 | 08159 | 06927 | 1 | | 0 | 0 | 0 | 0 | 0 | 0 | 0 | 0 | 0 | 0 | 0 | 0 | 0 | 0 | 0 | 0 | 0 | 0 | 0 | 0 | 0 | 1 | 0 | 0 | 0 | | | | | | |
| 6929 | 08160 | 06928 | 1 | | 0 | 0 | 0 | 0 | 0 | 0 | 0 | 0 | 0 | 0 | 0 | 0 | 0 | 0 | 0 | 0 | 0 | 0 | 0 | 0 | 0 | 1 | 0 | 0 | 0 | | | | | | |
| 6930 | 08161 | 06929 | 1 | | 0 | 0 | 0 | 0 | 0 | 0 | 0 | 0 | 0 | 0 | 0 | 0 | 0 | 0 | 0 | 0 | 0 | 0 | 0 | 0 | 0 | 1 | 0 | 0 | 0 | | | | | | |
| 6931 | 08162 | 06930 | 2 | | 0 | 0 | 0 | 0 | 0 | 0 | 0 | 0 | 0 | 0 | 0 | 0 | 0 | 0 | 0 | 0 | 0 | 0 | 0 | 0 | 0 | 1 | 0 | 1 | 0 | | | | | | |
| 6932 | 08163 | 06931 | 1 | | 0 | 0 | 0 | 0 | 0 | 0 | 0 | 0 | 0 | 0 | 0 | 0 | 0 | 0 | 0 | 0 | 0 | 0 | 0 | 0 | 0 | 1 | 0 | 0 | 0 | | | | | | |
| 6933 | 08164 | 06932 | 1 | | 0 | 0 | 0 | 0 | 0 | 0 | 0 | 0 | 0 | 0 | 0 | 0 | 0 | 0 | 0 | 0 | 0 | 0 | 0 | 0 | 0 | 1 | 0 | 0 | 0 | | | | | | |

Table 193

| | A | B | C | E | G | I | K | M | O | Q | S | U | W | Y | AA | AC | AE | AG | AI | AK | AM | AO | AQ | AS | AU | AW | AY | BA | BC | BE | BF | BG | BH | BI | BK |
|---|---|---|---|---|---|---|---|---|---|---|---|---|---|---|---|---|---|---|---|---|---|---|---|---|---|---|---|---|---|---|---|---|---|---|---|
| 6934 | 08165 | 06933 | 1 | | 0 | 0 | 0 | 0 | 0 | 0 | 0 | 0 | 0 | 0 | 0 | 0 | 0 | 0 | 0 | 0 | 0 | 0 | 0 | 0 | 0 | 1 | 0 | 0 | 0 | M77349 | 94.2 | 103 | 7 | 2589 | 2691 |
| 6935 | 08166 | 06934 | 1 | | 0 | 0 | 0 | 0 | 0 | 0 | 0 | 0 | 0 | 0 | 0 | 0 | 0 | 0 | 0 | 0 | 0 | 0 | 0 | 0 | 0 | 1 | 0 | 0 | 0 | | | | | | |
| 6936 | 08167 | 06935 | 1 | | 0 | 0 | 0 | 0 | 0 | 0 | 0 | 0 | 0 | 0 | 0 | 0 | 0 | 0 | 0 | 0 | 0 | 0 | 0 | 0 | 0 | 1 | 0 | 0 | 0 | | | | | | |
| 6937 | 08168 | 06936 | 1 | | 0 | 0 | 0 | 0 | 0 | 0 | 0 | 0 | 0 | 0 | 0 | 0 | 0 | 0 | 0 | 0 | 0 | 0 | 0 | 0 | 0 | 1 | 0 | 0 | 0 | | | | | | |
| 6938 | 08169 | 06937 | 1 | | 0 | 0 | 0 | 0 | 0 | 0 | 0 | 0 | 0 | 0 | 0 | 0 | 0 | 0 | 0 | 0 | 0 | 0 | 0 | 0 | 0 | 1 | 0 | 0 | 0 | | | | | | |
| 6939 | 08170 | 06938 | 1 | | 0 | 0 | 0 | 0 | 0 | 0 | 0 | 0 | 0 | 0 | 0 | 0 | 0 | 0 | 0 | 0 | 0 | 0 | 0 | 0 | 0 | 1 | 0 | 0 | 0 | | | | | | |
| 6940 | 08172 | 06939 | 1 | | 0 | 0 | 0 | 0 | 0 | 0 | 0 | 0 | 0 | 0 | 0 | 0 | 0 | 0 | 0 | 0 | 0 | 0 | 0 | 0 | 0 | 1 | 0 | 0 | 0 | | | | | | |
| 6941 | 08173 | 06940 | 1 | | 0 | 0 | 0 | 0 | 0 | 0 | 0 | 0 | 0 | 0 | 0 | 0 | 0 | 0 | 0 | 0 | 0 | 0 | 0 | 0 | 0 | 1 | 0 | 0 | 0 | | | | | | |
| 6942 | 08174 | 06941 | 1 | | 0 | 0 | 0 | 0 | 0 | 0 | 0 | 0 | 0 | 0 | 0 | 0 | 0 | 0 | 0 | 0 | 0 | 0 | 0 | 0 | 0 | 1 | 0 | 0 | 0 | | | | | | |
| 6943 | 08175 | 06942 | 1 | | 0 | 0 | 0 | 0 | 0 | 0 | 0 | 0 | 0 | 0 | 0 | 0 | 0 | 0 | 0 | 0 | 0 | 0 | 0 | 0 | 0 | 1 | 0 | 0 | 0 | | | | | | |
| 6944 | 08176 | 06943 | 1 | | 0 | 0 | 0 | 0 | 0 | 0 | 0 | 0 | 0 | 0 | 0 | 0 | 0 | 0 | 0 | 0 | 0 | 0 | 0 | 0 | 0 | 1 | 0 | 0 | 0 | | | | | | |
| 6945 | 08177 | 06944 | 1 | | 0 | 0 | 0 | 0 | 0 | 0 | 0 | 0 | 0 | 0 | 0 | 0 | 0 | 0 | 0 | 0 | 0 | 0 | 0 | 0 | 0 | 1 | 0 | 0 | 0 | | | | | | |
| 6946 | 08178 | 06945 | 2 | | 0 | 0 | 0 | 0 | 0 | 0 | 0 | 0 | 0 | 0 | 0 | 0 | 0 | 0 | 0 | 0 | 0 | 0 | 0 | 0 | 0 | 2 | 0 | 0 | 0 | | | | | | |
| 6947 | 08179 | 06946 | 1 | | 0 | 0 | 0 | 0 | 0 | 0 | 0 | 0 | 0 | 0 | 0 | 0 | 0 | 0 | 0 | 0 | 0 | 0 | 0 | 0 | 0 | 1 | 0 | 0 | 0 | | | | | | |
| 6948 | 08180 | 06947 | 1 | | 0 | 0 | 0 | 0 | 0 | 0 | 0 | 0 | 0 | 0 | 0 | 0 | 0 | 0 | 0 | 0 | 0 | 0 | 0 | 0 | 0 | 1 | 0 | 0 | 0 | | | | | | |
| 6949 | 08181 | 06948 | 3 | | 0 | 0 | 0 | 0 | 0 | 0 | 0 | 0 | 0 | 0 | 0 | 0 | 0 | 0 | 0 | 0 | 0 | 0 | 0 | 0 | 0 | 3 | 0 | 0 | 0 | | | | | | |
| 6950 | 08182 | 06949 | 1 | | 0 | 0 | 0 | 0 | 0 | 0 | 0 | 0 | 0 | 0 | 0 | 0 | 0 | 0 | 0 | 0 | 0 | 0 | 0 | 0 | 0 | 1 | 0 | 0 | 0 | K00558 | 90.9 | 99 | 2 | 938 | 1596 |
| 6951 | 08184 | 06950 | 1 | | 0 | 0 | 0 | 0 | 0 | 0 | 0 | 0 | 0 | 0 | 0 | 0 | 0 | 0 | 0 | 0 | 0 | 0 | 0 | 0 | 0 | 1 | 0 | 0 | 0 | | | | | | |
| 6952 | 08185 | 06951 | 1 | | 0 | 0 | 0 | 0 | 0 | 0 | 0 | 0 | 0 | 0 | 0 | 0 | 0 | 0 | 0 | 0 | 0 | 0 | 0 | 0 | 0 | 1 | 0 | 0 | 0 | | | | | | |
| 6953 | 08186 | 06952 | 2 | | 0 | 0 | 0 | 0 | 0 | 0 | 0 | 0 | 0 | 0 | 0 | 0 | 0 | 0 | 0 | 0 | 0 | 0 | 0 | 0 | 0 | 2 | 0 | 0 | 0 | | | | | | |
| 6954 | 08188 | 06953 | 1 | | 0 | 0 | 0 | 0 | 0 | 0 | 0 | 0 | 0 | 0 | 0 | 0 | 0 | 0 | 0 | 0 | 0 | 0 | 0 | 0 | 0 | 1 | 0 | 0 | 0 | | | | | | |
| 6955 | 08189 | 06954 | 1 | | 0 | 0 | 0 | 0 | 0 | 0 | 0 | 0 | 0 | 0 | 0 | 0 | 0 | 0 | 0 | 0 | 0 | 0 | 0 | 0 | 0 | 1 | 0 | 0 | 0 | | | | | | |
| 6956 | 08190 | 06955 | 1 | | 0 | 0 | 0 | 0 | 0 | 0 | 0 | 0 | 0 | 0 | 0 | 0 | 0 | 0 | 0 | 0 | 0 | 0 | 0 | 0 | 0 | 1 | 0 | 0 | 0 | | | | | | |
| 6957 | 08191 | 06956 | 1 | | 0 | 0 | 0 | 0 | 0 | 0 | 0 | 0 | 0 | 0 | 0 | 0 | 0 | 0 | 0 | 0 | 0 | 0 | 0 | 0 | 0 | 1 | 0 | 0 | 0 | | | | | | |
| 6958 | 08192 | 06957 | 1 | | 0 | 0 | 0 | 0 | 0 | 0 | 0 | 0 | 0 | 0 | 0 | 0 | 0 | 0 | 0 | 0 | 0 | 0 | 0 | 0 | 0 | 1 | 0 | 0 | 0 | | | | | | |
| 6959 | 08193 | 06958 | 1 | | 0 | 0 | 0 | 0 | 0 | 0 | 0 | 0 | 0 | 0 | 0 | 0 | 0 | 0 | 0 | 0 | 0 | 0 | 0 | 0 | 0 | 1 | 0 | 0 | 0 | | | | | | |
| 6960 | 08194 | 06959 | 1 | | 0 | 0 | 0 | 0 | 0 | 0 | 0 | 0 | 0 | 0 | 0 | 0 | 0 | 0 | 0 | 0 | 0 | 0 | 0 | 0 | 0 | 1 | 0 | 0 | 0 | | | | | | |
| 6961 | 08195 | 06960 | 1 | | 0 | 0 | 0 | 0 | 0 | 0 | 0 | 0 | 0 | 0 | 0 | 0 | 0 | 0 | 0 | 0 | 0 | 0 | 0 | 0 | 0 | 1 | 0 | 0 | 0 | | | | | | |
| 6962 | 08196 | 06961 | 1 | | 0 | 0 | 0 | 0 | 0 | 0 | 0 | 0 | 0 | 0 | 0 | 0 | 0 | 0 | 0 | 0 | 0 | 0 | 0 | 0 | 0 | 1 | 0 | 0 | 0 | M24194 | 93.7 | 205 | 1 | 887 | 1093 |
| 6963 | 08197 | 06962 | 1 | | 0 | 0 | 0 | 0 | 0 | 0 | 0 | 0 | 0 | 0 | 0 | 0 | 0 | 0 | 0 | 0 | 0 | 0 | 0 | 0 | 0 | 1 | 0 | 0 | 0 | | | | | | |
| 6964 | 08198 | 06963 | 1 | | 0 | 0 | 0 | 0 | 0 | 0 | 0 | 0 | 0 | 0 | 0 | 0 | 0 | 0 | 0 | 0 | 0 | 0 | 0 | 0 | 0 | 1 | 0 | 0 | 0 | | | | | | |
| 6965 | 08199 | 06964 | 1 | | 0 | 0 | 0 | 0 | 0 | 0 | 0 | 0 | 0 | 0 | 0 | 0 | 0 | 0 | 0 | 0 | 0 | 0 | 0 | 0 | 0 | 1 | 0 | 0 | 0 | U04209 | 92.2 | 51 | 1 | 1905 | 1955 |
| 6966 | 08200 | 06965 | 1 | | 0 | 0 | 0 | 0 | 0 | 0 | 0 | 0 | 0 | 0 | 0 | 0 | 0 | 0 | 0 | 0 | 0 | 0 | 0 | 0 | 0 | 1 | 0 | 0 | 0 | | | | | | |
| 6967 | 08201 | 06966 | 1 | | 0 | 0 | 0 | 0 | 0 | 0 | 0 | 0 | 0 | 0 | 0 | 0 | 0 | 0 | 0 | 0 | 0 | 0 | 0 | 0 | 0 | 1 | 0 | 0 | 0 | | | | | | |
| 6968 | 08202 | 06967 | 2 | | 0 | 0 | 0 | 0 | 0 | 0 | 0 | 0 | 0 | 0 | 0 | 0 | 0 | 0 | 0 | 0 | 0 | 0 | 0 | 0 | 0 | 2 | 0 | 0 | 0 | | | | | | |
| 6969 | 08203 | 06968 | 1 | | 0 | 0 | 0 | 0 | 0 | 0 | 0 | 0 | 0 | 0 | 0 | 0 | 0 | 0 | 0 | 0 | 0 | 0 | 0 | 0 | 0 | 1 | 0 | 0 | 0 | L18960 | 100 | 38 | 1 | 702 | 1202 |

Table 194

| | A | B | C | E | G | I | K | M | O | Q | S | U | W | Y | AA | AC | AE | AG | AI | AK | AM | AO | AQ | AS | AU | AW | AY | BA | BC | BE | BF | BG | BH | BI | BK |
|---|---|---|---|---|---|---|---|---|---|---|---|---|---|---|---|---|---|---|---|---|---|---|---|---|---|---|---|---|---|---|---|---|---|---|---|
| 6970 | 08204 | 06969 | 1 | | 0 | 0 | 0 | 0 | 0 | 0 | 0 | 0 | 0 | 0 | 0 | 0 | 0 | 0 | 0 | 0 | 0 | 0 | 0 | 0 | 0 | 1 | 0 | 0 | 0 | | | | | | |
| 6971 | 08205 | 06970 | 1 | | 0 | 0 | 0 | 0 | 0 | 0 | 0 | 0 | 0 | 0 | 0 | 0 | 0 | 0 | 0 | 0 | 0 | 0 | 0 | 0 | 0 | 1 | 0 | 0 | 0 | | | | | | |
| 6972 | 08206 | 06971 | 1 | | 0 | 0 | 0 | 0 | 0 | 0 | 0 | 0 | 0 | 0 | 0 | 0 | 0 | 0 | 0 | 0 | 0 | 0 | 0 | 0 | 0 | 1 | 0 | 0 | 0 | | | | | | |
| 6973 | 08207 | 06972 | 1 | | 0 | 0 | 0 | 0 | 0 | 0 | 0 | 0 | 0 | 0 | 0 | 0 | 0 | 0 | 0 | 0 | 0 | 0 | 0 | 0 | 0 | 1 | 0 | 0 | 0 | | | | | | |
| 6974 | 08208 | 06973 | 1 | | 0 | 0 | 0 | 0 | 0 | 0 | 0 | 0 | 0 | 0 | 0 | 0 | 0 | 0 | 0 | 0 | 0 | 0 | 0 | 0 | 0 | 1 | 0 | 0 | 0 | | | | | | |
| 6975 | 08209 | 06974 | 1 | | 0 | 0 | 0 | 0 | 0 | 0 | 0 | 0 | 0 | 0 | 0 | 0 | 0 | 0 | 0 | 0 | 0 | 0 | 0 | 0 | 0 | 1 | 0 | 0 | 0 | | | | | | |
| 6976 | 08210 | 06975 | 1 | | 0 | 0 | 0 | 0 | 0 | 0 | 0 | 0 | 0 | 0 | 0 | 0 | 0 | 0 | 0 | 0 | 0 | 0 | 0 | 0 | 0 | 1 | 0 | 0 | 0 | | | | | | |
| 6977 | 08211 | 06976 | 1 | | 0 | 0 | 0 | 0 | 0 | 0 | 0 | 0 | 0 | 0 | 0 | 0 | 0 | 0 | 0 | 0 | 0 | 0 | 0 | 0 | 0 | 1 | 0 | 0 | 0 | | | | | | |
| 6978 | 08212 | 06977 | 1 | | 0 | 0 | 0 | 0 | 0 | 0 | 0 | 0 | 0 | 0 | 0 | 0 | 0 | 0 | 0 | 0 | 0 | 0 | 0 | 0 | 0 | 1 | 0 | 0 | 0 | | | | | | |
| 6979 | 08213 | 06978 | 1 | | 0 | 0 | 0 | 0 | 0 | 0 | 0 | 0 | 0 | 0 | 0 | 0 | 0 | 0 | 0 | 0 | 0 | 0 | 0 | 0 | 0 | 1 | 0 | 0 | 0 | | | | | | |
| 6980 | 08214 | 06979 | 1 | | 0 | 0 | 0 | 0 | 0 | 0 | 0 | 0 | 0 | 0 | 0 | 0 | 0 | 0 | 0 | 0 | 0 | 0 | 0 | 0 | 0 | 1 | 0 | 0 | 0 | | | | | | |
| 6981 | 08215 | 06980 | 1 | | 0 | 0 | 0 | 0 | 0 | 0 | 0 | 0 | 0 | 0 | 0 | 0 | 0 | 0 | 0 | 0 | 0 | 0 | 0 | 0 | 0 | 1 | 0 | 0 | 0 | | | | | | |
| 6982 | 08216 | 06981 | 1 | | 0 | 0 | 0 | 0 | 0 | 0 | 0 | 0 | 0 | 0 | 0 | 0 | 0 | 0 | 0 | 0 | 0 | 0 | 0 | 0 | 0 | 1 | 0 | 0 | 0 | M15800 | 98.6 | 69 | 1 | 983 | 1051 |
| 6983 | 08217 | 06982 | 1 | | 0 | 0 | 0 | 0 | 0 | 0 | 0 | 0 | 0 | 0 | 0 | 0 | 0 | 0 | 0 | 0 | 0 | 0 | 0 | 0 | 0 | 1 | 0 | 0 | 0 | | | | | | |
| 6984 | 08218 | 06983 | 1 | | 0 | 0 | 0 | 0 | 0 | 0 | 0 | 0 | 0 | 0 | 0 | 0 | 0 | 0 | 0 | 0 | 0 | 0 | 0 | 0 | 0 | 1 | 0 | 0 | 0 | | | | | | |
| 6985 | 08219 | 06984 | 1 | | 0 | 0 | 0 | 0 | 0 | 0 | 0 | 0 | 0 | 0 | 0 | 0 | 0 | 0 | 0 | 0 | 0 | 0 | 0 | 0 | 0 | 1 | 0 | 0 | 0 | | | | | | |
| 6986 | 08220 | 06985 | 1 | | 0 | 0 | 0 | 0 | 0 | 0 | 0 | 0 | 0 | 0 | 0 | 0 | 0 | 0 | 0 | 0 | 0 | 0 | 0 | 0 | 0 | 1 | 0 | 0 | 0 | | | | | | |
| 6987 | 08221 | 06986 | 1 | | 0 | 0 | 0 | 0 | 0 | 0 | 0 | 0 | 0 | 0 | 0 | 0 | 0 | 0 | 0 | 0 | 0 | 0 | 0 | 0 | 0 | 1 | 0 | 0 | 0 | | | | | | |
| 6988 | 08222 | 06987 | 1 | | 0 | 0 | 0 | 0 | 0 | 0 | 0 | 0 | 0 | 0 | 0 | 0 | 0 | 0 | 0 | 0 | 0 | 0 | 0 | 0 | 0 | 1 | 0 | 0 | 0 | | | | | | |
| 6989 | 08223 | 06988 | 1 | | 0 | 0 | 0 | 0 | 0 | 0 | 0 | 0 | 0 | 0 | 0 | 0 | 0 | 0 | 0 | 0 | 0 | 0 | 0 | 0 | 0 | 1 | 0 | 0 | 0 | | | | | | |
| 6990 | 08224 | 06989 | 2 | | 0 | 0 | 0 | 0 | 0 | 0 | 0 | 0 | 0 | 0 | 0 | 0 | 0 | 0 | 0 | 0 | 0 | 0 | 0 | 0 | 0 | 1 | 1 | 0 | 0 | | | | | | |
| 6991 | 08225 | 06990 | 1 | | 0 | 0 | 0 | 0 | 0 | 0 | 0 | 0 | 0 | 0 | 0 | 0 | 0 | 0 | 0 | 0 | 0 | 0 | 0 | 0 | 0 | 1 | 0 | 0 | 0 | | | | | | |
| 6992 | 08226 | 06991 | 1 | | 0 | 0 | 0 | 0 | 0 | 0 | 0 | 0 | 0 | 0 | 0 | 0 | 0 | 0 | 0 | 0 | 0 | 0 | 0 | 0 | 0 | 1 | 0 | 0 | 0 | | | | | | |
| 6993 | 08227 | 06992 | 1 | | 0 | 0 | 0 | 0 | 0 | 0 | 0 | 0 | 0 | 0 | 0 | 0 | 0 | 0 | 0 | 0 | 0 | 0 | 0 | 0 | 0 | 1 | 0 | 0 | 0 | | | | | | |
| 6994 | 08228 | 06993 | 1 | | 0 | 0 | 0 | 0 | 0 | 0 | 0 | 0 | 0 | 0 | 0 | 0 | 0 | 0 | 0 | 0 | 0 | 0 | 0 | 0 | 0 | 1 | 0 | 0 | 0 | | | | | | |
| 6995 | 08229 | 06994 | 1 | | 0 | 0 | 0 | 0 | 0 | 0 | 0 | 0 | 0 | 0 | 0 | 0 | 0 | 0 | 0 | 0 | 0 | 0 | 0 | 0 | 0 | 1 | 0 | 0 | 0 | | | | | | |
| 6996 | 08230 | 06995 | 1 | | 0 | 0 | 0 | 0 | 0 | 0 | 0 | 0 | 0 | 0 | 0 | 0 | 0 | 0 | 0 | 0 | 0 | 0 | 0 | 0 | 0 | 1 | 0 | 0 | 0 | | | | | | |
| 6997 | 08232 | 06996 | 1 | | 0 | 0 | 0 | 0 | 0 | 0 | 0 | 0 | 0 | 0 | 0 | 0 | 0 | 0 | 0 | 0 | 0 | 0 | 0 | 0 | 0 | 1 | 0 | 0 | 0 | | | | | | |
| 6998 | 08233 | 06997 | 1 | | 0 | 0 | 0 | 0 | 0 | 0 | 0 | 0 | 0 | 0 | 0 | 0 | 0 | 0 | 0 | 0 | 0 | 0 | 0 | 0 | 0 | 1 | 0 | 0 | 0 | | | | | | |
| 6999 | 08234 | 06998 | 1 | | 0 | 0 | 0 | 0 | 0 | 0 | 0 | 0 | 0 | 0 | 0 | 0 | 0 | 0 | 0 | 0 | 0 | 0 | 0 | 0 | 0 | 1 | 0 | 0 | 0 | | | | | | |
| 7000 | 08235 | 06999 | 3 | | 0 | 0 | 0 | 0 | 0 | 0 | 0 | 0 | 0 | 0 | 0 | 0 | 0 | 0 | 0 | 0 | 0 | 0 | 0 | 0 | 0 | 1 | 2 | 0 | 0 | | | | | | |
| 7001 | 08236 | 07000 | 1 | | 0 | 0 | 0 | 0 | 0 | 0 | 0 | 0 | 0 | 0 | 0 | 0 | 0 | 0 | 0 | 0 | 0 | 0 | 0 | 0 | 0 | 1 | 0 | 0 | 0 | | | | | | |
| 7002 | 08237 | 07001 | 2 | | 0 | 0 | 0 | 0 | 0 | 0 | 0 | 0 | 0 | 0 | 0 | 0 | 0 | 0 | 0 | 0 | 0 | 0 | 0 | 0 | 0 | 1 | 0 | 1 | 0 | | | | | | |
| 7003 | 08238 | 07002 | 1 | | 0 | 0 | 0 | 0 | 0 | 0 | 0 | 0 | 0 | 0 | 0 | 0 | 0 | 0 | 0 | 0 | 0 | 0 | 0 | 0 | 0 | 1 | 0 | 0 | 0 | | | | | | |
| 7004 | 08239 | 07003 | 1 | | 0 | 0 | 0 | 0 | 0 | 0 | 0 | 0 | 0 | 0 | 0 | 0 | 0 | 0 | 0 | 0 | 0 | 0 | 0 | 0 | 0 | 1 | 0 | 0 | 0 | | | | | | |
| 7005 | 08240 | 07004 | 1 | | 0 | 0 | 0 | 0 | 0 | 0 | 0 | 0 | 0 | 0 | 0 | 0 | 0 | 0 | 0 | 0 | 0 | 0 | 0 | 0 | 0 | 1 | 0 | 0 | 0 | | | | | | |

Table 195

| | A | B | C | E | G | I | K | M | O | Q | S | U | W | Y | AA | AC | AE | AG | AI | AK | AM | AO | AQ | AS | AU | AW | AY | BA | BC | BE | BF | BG | BH | BI | BK |
|---|---|---|---|---|---|---|---|---|---|---|---|---|---|---|---|---|---|---|---|---|---|---|---|---|---|---|---|---|---|---|---|---|---|---|---|
| 7006 | 08241 | 07005 | 1 | | 0 | 0 | 0 | 0 | 0 | 0 | 0 | 0 | 0 | 0 | 0 | 0 | 0 | 0 | 0 | 0 | 0 | 0 | 0 | 0 | 0 | 1 | 0 | 0 | 0 | | | | | | |
| 7007 | 08242 | 07006 | 1 | | 0 | 0 | 0 | 0 | 0 | 0 | 0 | 0 | 0 | 0 | 0 | 0 | 0 | 0 | 0 | 0 | 0 | 0 | 0 | 0 | 0 | 1 | 0 | 0 | 0 | L06133 | 98.2 | 57 | 1 | 8422 | 8478 |
| 7008 | 08243 | 07007 | 1 | | 0 | 0 | 0 | 0 | 0 | 0 | 0 | 0 | 0 | 0 | 0 | 0 | 0 | 0 | 0 | 0 | 0 | 0 | 0 | 0 | 0 | 1 | 0 | 0 | 0 | | | | | | |
| 7009 | 08244 | 07008 | 1 | | 0 | 0 | 0 | 0 | 0 | 0 | 0 | 0 | 0 | 0 | 0 | 0 | 0 | 0 | 0 | 0 | 0 | 0 | 0 | 0 | 0 | 1 | 0 | 0 | 0 | | | | | | |
| 7010 | 08245 | 07009 | 1 | | 0 | 0 | 0 | 0 | 0 | 0 | 0 | 0 | 0 | 0 | 0 | 0 | 0 | 0 | 0 | 0 | 0 | 0 | 0 | 0 | 0 | 1 | 0 | 0 | 0 | | | | | | |
| 7011 | 08246 | 07010 | 1 | | 0 | 0 | 0 | 0 | 0 | 0 | 0 | 0 | 0 | 0 | 0 | 0 | 0 | 0 | 0 | 0 | 0 | 0 | 0 | 0 | 0 | 1 | 0 | 0 | 0 | | | | | | |
| 7012 | 08247 | 07011 | 1 | | 0 | 0 | 0 | 0 | 0 | 0 | 0 | 0 | 0 | 0 | 0 | 0 | 0 | 0 | 0 | 0 | 0 | 0 | 0 | 0 | 0 | 1 | 0 | 0 | 0 | S55700 | 94.2 | 275 | 1 | 1297 | 1589 |
| 7013 | 08248 | 07012 | 1 | | 0 | 0 | 0 | 0 | 0 | 0 | 0 | 0 | 0 | 0 | 0 | 0 | 0 | 0 | 0 | 0 | 0 | 0 | 0 | 0 | 0 | 1 | 0 | 0 | 0 | | | | | | |
| 7014 | 08249 | 07013 | 1 | | 0 | 0 | 0 | 0 | 0 | 0 | 0 | 0 | 0 | 0 | 0 | 0 | 0 | 0 | 0 | 0 | 0 | 0 | 0 | 0 | 0 | 1 | 0 | 0 | 0 | L20298 | 94.9 | 214 | 1 | 2615 | 2851 |
| 7015 | 08250 | 07014 | 2 | | 0 | 0 | 0 | 0 | 0 | 0 | 0 | 0 | 0 | 0 | 0 | 0 | 0 | 0 | 0 | 0 | 0 | 0 | 0 | 0 | 0 | 1 | 0 | 1 | 0 | | | | | | |
| 7016 | 08251 | 07015 | 1 | | 0 | 0 | 0 | 0 | 0 | 0 | 0 | 0 | 0 | 0 | 0 | 0 | 0 | 0 | 0 | 0 | 0 | 0 | 0 | 0 | 0 | 1 | 0 | 0 | 0 | | | | | | |
| 7017 | 08252 | 07016 | 1 | | 0 | 0 | 0 | 0 | 0 | 0 | 0 | 0 | 0 | 0 | 0 | 0 | 0 | 0 | 0 | 0 | 0 | 0 | 0 | 0 | 0 | 1 | 0 | 0 | 0 | | | | | | |
| 7018 | 08253 | 07017 | 1 | | 0 | 0 | 0 | 0 | 0 | 0 | 0 | 0 | 0 | 0 | 0 | 0 | 0 | 0 | 0 | 0 | 0 | 0 | 0 | 0 | 0 | 1 | 0 | 0 | 0 | | | | | | |
| 7019 | 08254 | 07018 | 1 | | 0 | 0 | 0 | 0 | 0 | 0 | 0 | 0 | 0 | 0 | 0 | 0 | 0 | 0 | 0 | 0 | 0 | 0 | 0 | 0 | 0 | 1 | 0 | 0 | 0 | | | | | | |
| 7020 | 08255 | 07019 | 1 | | 0 | 0 | 0 | 0 | 0 | 0 | 0 | 0 | 0 | 0 | 0 | 0 | 0 | 0 | 0 | 0 | 0 | 0 | 0 | 0 | 0 | 1 | 0 | 0 | 0 | | | | | | |
| 7021 | 08256 | 07020 | 1 | | 0 | 0 | 0 | 0 | 0 | 0 | 0 | 0 | 0 | 0 | 0 | 0 | 0 | 0 | 0 | 0 | 0 | 0 | 0 | 0 | 0 | 1 | 0 | 0 | 0 | | | | | | |
| 7022 | 08257 | 07021 | 1 | | 0 | 0 | 0 | 0 | 0 | 0 | 0 | 0 | 0 | 0 | 0 | 0 | 0 | 0 | 0 | 0 | 0 | 0 | 0 | 0 | 0 | 1 | 0 | 0 | 0 | | | | | | |
| 7023 | 08258 | 07022 | 1 | | 0 | 0 | 0 | 0 | 0 | 0 | 0 | 0 | 0 | 0 | 0 | 0 | 0 | 0 | 0 | 0 | 0 | 0 | 0 | 0 | 0 | 1 | 0 | 0 | 0 | | | | | | |
| 7024 | 08259 | 07023 | 1 | | 0 | 0 | 0 | 0 | 0 | 0 | 0 | 0 | 0 | 0 | 0 | 0 | 0 | 0 | 0 | 0 | 0 | 0 | 0 | 0 | 0 | 1 | 0 | 0 | 0 | | | | | | |
| 7025 | 08260 | 07024 | 1 | | 0 | 0 | 0 | 0 | 0 | 0 | 0 | 0 | 0 | 0 | 0 | 0 | 0 | 0 | 0 | 0 | 0 | 0 | 0 | 0 | 0 | 1 | 0 | 0 | 0 | | | | | | |
| 7026 | 08261 | 07025 | 1 | | 0 | 0 | 0 | 0 | 0 | 0 | 0 | 0 | 0 | 0 | 0 | 0 | 0 | 0 | 0 | 0 | 0 | 0 | 0 | 0 | 0 | 1 | 0 | 0 | 0 | | | | | | |
| 7027 | 08262 | 07026 | 1 | | 0 | 0 | 0 | 0 | 0 | 0 | 0 | 0 | 0 | 0 | 0 | 0 | 0 | 0 | 0 | 0 | 0 | 0 | 0 | 0 | 0 | 1 | 0 | 0 | 0 | | | | | | |
| 7028 | 08263 | 07027 | 1 | | 0 | 0 | 0 | 0 | 0 | 0 | 0 | 0 | 0 | 0 | 0 | 0 | 0 | 0 | 0 | 0 | 0 | 0 | 0 | 0 | 0 | 1 | 0 | 0 | 0 | | | | | | |
| 7029 | 08264 | 07028 | 1 | | 0 | 0 | 0 | 0 | 0 | 0 | 0 | 0 | 0 | 0 | 0 | 0 | 0 | 0 | 0 | 0 | 0 | 0 | 0 | 0 | 0 | 1 | 0 | 0 | 0 | | | | | | |
| 7030 | 08265 | 07029 | 1 | | 0 | 0 | 0 | 0 | 0 | 0 | 0 | 0 | 0 | 0 | 0 | 0 | 0 | 0 | 0 | 0 | 0 | 0 | 0 | 0 | 0 | 1 | 0 | 0 | 0 | | | | | | |
| 7031 | 08266 | 07030 | 1 | | 0 | 0 | 0 | 0 | 0 | 0 | 0 | 0 | 0 | 0 | 0 | 0 | 0 | 0 | 0 | 0 | 0 | 0 | 0 | 0 | 0 | 1 | 0 | 0 | 0 | | | | | | |
| 7032 | 08267 | 07031 | 1 | | 0 | 0 | 0 | 0 | 0 | 0 | 0 | 0 | 0 | 0 | 0 | 0 | 0 | 0 | 0 | 0 | 0 | 0 | 0 | 0 | 0 | 1 | 0 | 0 | 0 | | | | | | |
| 7033 | 08268 | 07032 | 1 | | 0 | 0 | 0 | 0 | 0 | 0 | 0 | 0 | 0 | 0 | 0 | 0 | 0 | 0 | 0 | 0 | 0 | 0 | 0 | 0 | 0 | 1 | 0 | 0 | 0 | | | | | | |
| 7034 | 08269 | 07033 | 1 | | 0 | 0 | 0 | 0 | 0 | 0 | 0 | 0 | 0 | 0 | 0 | 0 | 0 | 0 | 0 | 0 | 0 | 0 | 0 | 0 | 0 | 1 | 0 | 0 | 0 | D00860 | 96 | 101 | 1 | 1974 | 2075 |
| 7035 | 08270 | 07034 | 1 | | 0 | 0 | 0 | 0 | 0 | 0 | 0 | 0 | 0 | 0 | 0 | 0 | 0 | 0 | 0 | 0 | 0 | 0 | 0 | 0 | 0 | 1 | 0 | 0 | 0 | | | | | | |
| 7036 | 08271 | 07035 | 1 | | 0 | 0 | 0 | 0 | 0 | 0 | 0 | 0 | 0 | 0 | 0 | 0 | 0 | 0 | 0 | 0 | 0 | 0 | 0 | 0 | 0 | 1 | 0 | 0 | 0 | | | | | | |
| 7037 | 08272 | 07036 | 1 | | 0 | 0 | 0 | 0 | 0 | 0 | 0 | 0 | 0 | 0 | 0 | 0 | 0 | 0 | 0 | 0 | 0 | 0 | 0 | 0 | 0 | 1 | 0 | 0 | 0 | | | | | | |
| 7038 | 08273 | 07037 | 1 | | 0 | 0 | 0 | 0 | 0 | 0 | 0 | 0 | 0 | 0 | 0 | 0 | 0 | 0 | 0 | 0 | 0 | 0 | 0 | 0 | 0 | 1 | 0 | 0 | 0 | | | | | | |
| 7039 | 08274 | 07038 | 1 | | 0 | 0 | 0 | 0 | 0 | 0 | 0 | 0 | 0 | 0 | 0 | 0 | 0 | 0 | 0 | 0 | 0 | 0 | 0 | 0 | 0 | 1 | 0 | 0 | 0 | | | | | | |
| 7040 | 08275 | 07039 | 1 | | 0 | 0 | 0 | 0 | 0 | 0 | 0 | 0 | 0 | 0 | 0 | 0 | 0 | 0 | 0 | 0 | 0 | 0 | 0 | 0 | 0 | 1 | 0 | 0 | 0 | | | | | | |
| 7041 | 08276 | 07040 | 1 | | 0 | 0 | 0 | 0 | 0 | 0 | 0 | 0 | 0 | 0 | 0 | 0 | 0 | 0 | 0 | 0 | 0 | 0 | 0 | 0 | 0 | 1 | 0 | 0 | 0 | | | | | | |

Table 196

| | A | B | C | E | G | I | K | M | O | Q | S | U | W | Y | AA | AC | AF | AG | AI | AK | AM | AO | AQ | AS | AU | AW | AY | BA | BC | BE | BF | BG | BH | BI | BK |
|---|---|---|---|---|---|---|---|---|---|---|---|---|---|---|---|---|---|---|---|---|---|---|---|---|---|---|---|---|---|---|---|---|---|---|---|
| 7042 | 08277 | 07041 | 1 | | 0 | 0 | 0 | 0 | 0 | 0 | 0 | 0 | 0 | 0 | 0 | 0 | 0 | 0 | 0 | 0 | 0 | 0 | 0 | 0 | 0 | 1 | 0 | 0 | 0 | | | | | | |
| 7043 | 08278 | 07042 | 2 | | 0 | 0 | 0 | 0 | 0 | 0 | 0 | 0 | 0 | 0 | 0 | 0 | 0 | 0 | 0 | 0 | 0 | 0 | 0 | 0 | 0 | 1 | 1 | 0 | 0 | | | | | | |
| 7044 | 08279 | 07043 | 4 | | 0 | 0 | 0 | 0 | 0 | 0 | 0 | 0 | 0 | 0 | 0 | 0 | 0 | 0 | 0 | 0 | 0 | 0 | 0 | 0 | 0 | 2 | 2 | 0 | 0 | | | | | | |
| 7045 | 08280 | 07044 | 1 | | 0 | 0 | 0 | 0 | 0 | 0 | 0 | 0 | 0 | 0 | 0 | 0 | 0 | 0 | 0 | 0 | 0 | 0 | 0 | 0 | 0 | 1 | 0 | 0 | 0 | | | | | | |
| 7046 | 08281 | 07045 | 1 | | 0 | 0 | 0 | 0 | 0 | 0 | 0 | 0 | 0 | 0 | 0 | 0 | 0 | 0 | 0 | 0 | 0 | 0 | 0 | 0 | 0 | 1 | 0 | 0 | 0 | M57892 | 97.1 | 172 | 1 | 1145 | 1316 |
| 7047 | 08282 | 07046 | 1 | | 0 | 0 | 0 | 0 | 0 | 0 | 0 | 0 | 0 | 0 | 0 | 0 | 0 | 0 | 0 | 0 | 0 | 0 | 0 | 0 | 0 | 1 | 0 | 0 | 0 | | | | | | |
| 7048 | 08283 | 07047 | 1 | | 0 | 0 | 0 | 0 | 0 | 0 | 0 | 0 | 0 | 0 | 0 | 0 | 0 | 0 | 0 | 0 | 0 | 0 | 0 | 0 | 0 | 1 | 0 | 0 | 0 | | | | | | |
| 7049 | 08284 | 07048 | 1 | | 0 | 0 | 0 | 0 | 0 | 0 | 0 | 0 | 0 | 0 | 0 | 0 | 0 | 0 | 0 | 0 | 0 | 0 | 0 | 0 | 0 | 1 | 0 | 0 | 0 | | | | | | |
| 7050 | 08285 | 07049 | 1 | | 0 | 0 | 0 | 0 | 0 | 0 | 0 | 0 | 0 | 0 | 0 | 0 | 0 | 0 | 0 | 0 | 0 | 0 | 0 | 0 | 0 | 1 | 0 | 0 | 0 | | | | | | |
| 7051 | 08286 | 07050 | 1 | | 0 | 0 | 0 | 0 | 0 | 0 | 0 | 0 | 0 | 0 | 0 | 0 | 0 | 0 | 0 | 0 | 0 | 0 | 0 | 0 | 0 | 1 | 0 | 0 | 0 | | | | | | |
| 7052 | 08287 | 07051 | 1 | | 0 | 0 | 0 | 0 | 0 | 0 | 0 | 0 | 0 | 0 | 0 | 0 | 0 | 0 | 0 | 0 | 0 | 0 | 0 | 0 | 0 | 1 | 0 | 0 | 0 | X03918 | 94.3 | 123 | 1 | 2268 | 2760 |
| 7053 | 08288 | 07052 | 1 | | 0 | 0 | 0 | 0 | 0 | 0 | 0 | 0 | 0 | 0 | 0 | 0 | 0 | 0 | 0 | 0 | 0 | 0 | 0 | 0 | 0 | 1 | 0 | 0 | 0 | | | | | | |
| 7054 | 08289 | 07053 | 1 | | 0 | 0 | 0 | 0 | 0 | 0 | 0 | 0 | 0 | 0 | 0 | 0 | 0 | 0 | 0 | 0 | 0 | 0 | 0 | 0 | 0 | 1 | 0 | 0 | 0 | | | | | | |
| 7055 | 08290 | 07054 | 1 | | 0 | 0 | 0 | 0 | 0 | 0 | 0 | 0 | 0 | 0 | 0 | 0 | 0 | 0 | 0 | 0 | 0 | 0 | 0 | 0 | 0 | 1 | 0 | 0 | 0 | | | | | | |
| 7056 | 08291 | 07055 | 1 | | 0 | 0 | 0 | 0 | 0 | 0 | 0 | 0 | 0 | 0 | 0 | 0 | 0 | 0 | 0 | 0 | 0 | 0 | 0 | 0 | 0 | 1 | 0 | 0 | 0 | | | | | | |
| 7057 | 08292 | 07056 | 1 | | 0 | 0 | 0 | 0 | 0 | 0 | 0 | 0 | 0 | 0 | 0 | 0 | 0 | 0 | 0 | 0 | 0 | 0 | 0 | 0 | 0 | 1 | 0 | 0 | 0 | | | | | | |
| 7058 | 08293 | 07057 | 1 | | 0 | 0 | 0 | 0 | 0 | 0 | 0 | 0 | 0 | 0 | 0 | 0 | 0 | 0 | 0 | 0 | 0 | 0 | 0 | 0 | 0 | 1 | 0 | 0 | 0 | K03195 | 95.4 | 153 | 1 | 2704 | 2856 |
| 7059 | 08294 | 07058 | 1 | | 0 | 0 | 0 | 0 | 0 | 0 | 0 | 0 | 0 | 0 | 0 | 0 | 0 | 0 | 0 | 0 | 0 | 0 | 0 | 0 | 0 | 1 | 0 | 0 | 0 | | | | | | |
| 7060 | 08295 | 07059 | 1 | | 0 | 0 | 0 | 0 | 0 | 0 | 0 | 0 | 0 | 0 | 0 | 0 | 0 | 0 | 0 | 0 | 0 | 0 | 0 | 0 | 0 | 1 | 0 | 0 | 0 | | | | | | |
| 7061 | 08296 | 07060 | 1 | | 0 | 0 | 0 | 0 | 0 | 0 | 0 | 0 | 0 | 0 | 0 | 0 | 0 | 0 | 0 | 0 | 0 | 0 | 0 | 0 | 0 | 1 | 0 | 0 | 0 | | | | | | |
| 7062 | 08297 | 07061 | 2 | | 0 | 0 | 0 | 0 | 0 | 0 | 0 | 0 | 0 | 0 | 0 | 0 | 0 | 0 | 0 | 0 | 0 | 0 | 0 | 0 | 0 | 2 | 0 | 0 | 0 | L19783 | 100 | 18 | 1 | 1152 | 1394 |
| 7063 | 08298 | 07062 | 1 | | 0 | 0 | 0 | 0 | 0 | 0 | 0 | 0 | 0 | 0 | 0 | 0 | 0 | 0 | 0 | 0 | 0 | 0 | 0 | 0 | 0 | 1 | 0 | 0 | 0 | | | | | | |
| 7064 | 08299 | 07063 | 1 | | 0 | 0 | 0 | 0 | 0 | 0 | 0 | 0 | 0 | 0 | 0 | 0 | 0 | 0 | 0 | 0 | 0 | 0 | 0 | 0 | 0 | 1 | 0 | 0 | 0 | | | | | | |
| 7065 | 08300 | 07064 | 1 | | 0 | 0 | 0 | 0 | 0 | 0 | 0 | 0 | 0 | 0 | 0 | 0 | 0 | 0 | 0 | 0 | 0 | 0 | 0 | 0 | 0 | 1 | 0 | 0 | 0 | | | | | | |
| 7066 | 08301 | 07065 | 1 | | 0 | 0 | 0 | 0 | 0 | 0 | 0 | 0 | 0 | 0 | 0 | 0 | 0 | 0 | 0 | 0 | 0 | 0 | 0 | 0 | 0 | 1 | 0 | 0 | 0 | | | | | | |
| 7067 | 08302 | 07066 | 1 | | 0 | 0 | 0 | 0 | 0 | 0 | 0 | 0 | 0 | 0 | 0 | 0 | 0 | 0 | 0 | 0 | 0 | 0 | 0 | 0 | 0 | 1 | 0 | 0 | 0 | | | | | | |
| 7068 | 08303 | 07067 | 1 | | 0 | 0 | 0 | 0 | 0 | 0 | 0 | 0 | 0 | 0 | 0 | 0 | 0 | 0 | 0 | 0 | 0 | 0 | 0 | 0 | 0 | 1 | 0 | 0 | 0 | | | | | | |
| 7069 | 08304 | 07068 | 1 | | 0 | 0 | 0 | 0 | 0 | 0 | 0 | 0 | 0 | 0 | 0 | 0 | 0 | 0 | 0 | 0 | 0 | 0 | 0 | 0 | 0 | 1 | 0 | 0 | 0 | | | | | | |
| 7070 | 08305 | 07069 | 1 | | 0 | 0 | 0 | 0 | 0 | 0 | 0 | 0 | 0 | 0 | 0 | 0 | 0 | 0 | 0 | 0 | 0 | 0 | 0 | 0 | 0 | 1 | 0 | 0 | 0 | | | | | | |
| 7071 | 08306 | 07070 | 1 | | 0 | 0 | 0 | 0 | 0 | 0 | 0 | 0 | 0 | 0 | 0 | 0 | 0 | 0 | 0 | 0 | 0 | 0 | 0 | 0 | 0 | 1 | 0 | 0 | 0 | | | | | | |
| 7072 | 08307 | 07071 | 1 | | 0 | 0 | 0 | 0 | 0 | 0 | 0 | 0 | 0 | 0 | 0 | 0 | 0 | 0 | 0 | 0 | 0 | 0 | 0 | 0 | 0 | 1 | 0 | 0 | 0 | | | | | | |
| 7073 | 08308 | 07072 | 1 | | 0 | 0 | 0 | 0 | 0 | 0 | 0 | 0 | 0 | 0 | 0 | 0 | 0 | 0 | 0 | 0 | 0 | 0 | 0 | 0 | 0 | 1 | 0 | 0 | 0 | | | | | | |
| 7074 | 08309 | 07073 | 1 | | 0 | 0 | 0 | 0 | 0 | 0 | 0 | 0 | 0 | 0 | 0 | 0 | 0 | 0 | 0 | 0 | 0 | 0 | 0 | 0 | 0 | 1 | 0 | 0 | 0 | | | | | | |
| 7075 | 08310 | 07074 | 1 | | 0 | 0 | 0 | 0 | 0 | 0 | 0 | 0 | 0 | 0 | 0 | 0 | 0 | 0 | 0 | 0 | 0 | 0 | 0 | 0 | 0 | 1 | 0 | 0 | 0 | | | | | | |
| 7076 | 08311 | 07075 | 1 | | 0 | 0 | 0 | 0 | 0 | 0 | 0 | 0 | 0 | 0 | 0 | 0 | 0 | 0 | 0 | 0 | 0 | 0 | 0 | 0 | 0 | 1 | 0 | 0 | 0 | | | | | | |
| 7077 | 08312 | 07076 | 1 | | 0 | 0 | 0 | 0 | 0 | 0 | 0 | 0 | 0 | 0 | 0 | 0 | 0 | 0 | 0 | 0 | 0 | 0 | 0 | 0 | 0 | 1 | 0 | 0 | 0 | | | | | | |

Table 197

| | A | B | C | E | G | I | K | M | O | Q | S | U | W | Y | AA | AC | AE | AG | AI | AK | AM | AO | AQ | AS | AU | AW | AY | BA | BC | BE | BF | BG | BH | BI | BK |
|---|---|---|---|---|---|---|---|---|---|---|---|---|---|---|---|---|---|---|---|---|---|---|---|---|---|---|---|---|---|---|---|---|---|---|---|
| 7078 | 08313 | 07077 | 1 | | 0 | 0 | 0 | 0 | 0 | 0 | 0 | 0 | 0 | 0 | 0 | 0 | 0 | 0 | 0 | 0 | 0 | 0 | 0 | 0 | 0 | 1 | 0 | 0 | 0 | | | | | | |
| 7079 | 08314 | 07078 | 1 | | 0 | 0 | 0 | 0 | 0 | 0 | 0 | 0 | 0 | 0 | 0 | 0 | 0 | 0 | 0 | 0 | 0 | 0 | 0 | 0 | 0 | 1 | 0 | 0 | 0 | M96067 | 94.8 | 328 | 1 | 2147 | 2472 |
| 7080 | 08315 | 07079 | 1 | | 0 | 0 | 0 | 0 | 0 | 0 | 0 | 0 | 0 | 0 | 0 | 0 | 0 | 0 | 0 | 0 | 0 | 0 | 0 | 0 | 0 | 1 | 0 | 0 | 0 | | | | | | |
| 7081 | 08316 | 07080 | 1 | | 0 | 0 | 0 | 0 | 0 | 0 | 0 | 0 | 0 | 0 | 0 | 0 | 0 | 0 | 0 | 0 | 0 | 0 | 0 | 0 | 0 | 1 | 0 | 0 | 0 | | | | | | |
| 7082 | 08317 | 07081 | 1 | | 0 | 0 | 0 | 0 | 0 | 0 | 0 | 0 | 0 | 0 | 0 | 0 | 0 | 0 | 0 | 0 | 0 | 0 | 0 | 0 | 0 | 1 | 0 | 0 | 0 | | | | | | |
| 7083 | 08318 | 07082 | 1 | | 0 | 0 | 0 | 0 | 0 | 0 | 0 | 0 | 0 | 0 | 0 | 0 | 0 | 0 | 0 | 0 | 0 | 0 | 0 | 0 | 0 | 0 | 1 | 0 | 0 | M60626 | 99.4 | 310 | 1 | 1290 | 1866 |
| 7084 | 08319 | 07083 | 2 | | 0 | 0 | 0 | 0 | 0 | 0 | 0 | 0 | 0 | 0 | 0 | 0 | 0 | 0 | 0 | 0 | 0 | 0 | 0 | 0 | 0 | 0 | 2 | 0 | 0 | | | | | | |
| 7085 | 08320 | 07084 | 2 | | 0 | 0 | 0 | 0 | 0 | 0 | 0 | 0 | 0 | 0 | 0 | 0 | 0 | 0 | 0 | 0 | 0 | 0 | 0 | 0 | 0 | 0 | 2 | 0 | 0 | | | | | | |
| 7086 | 08321 | 07085 | 1 | | 0 | 0 | 0 | 0 | 0 | 0 | 0 | 0 | 0 | 0 | 0 | 0 | 0 | 0 | 0 | 0 | 0 | 0 | 0 | 0 | 0 | 0 | 1 | 0 | 0 | | | | | | |
| 7087 | 08322 | 07086 | 1 | | 0 | 0 | 0 | 0 | 0 | 0 | 0 | 0 | 0 | 0 | 0 | 0 | 0 | 0 | 0 | 0 | 0 | 0 | 0 | 0 | 0 | 0 | 1 | 0 | 0 | | | | | | |
| 7088 | 08323 | 07087 | 1 | | 0 | 0 | 0 | 0 | 0 | 0 | 0 | 0 | 0 | 0 | 0 | 0 | 0 | 0 | 0 | 0 | 0 | 0 | 0 | 0 | 0 | 0 | 1 | 0 | 0 | | | | | | |
| 7089 | 08324 | 07088 | 1 | | 0 | 0 | 0 | 0 | 0 | 0 | 0 | 0 | 0 | 0 | 0 | 0 | 0 | 0 | 0 | 0 | 0 | 0 | 0 | 0 | 0 | 0 | 1 | 0 | 0 | | | | | | |
| 7090 | 08325 | 07089 | 4 | | 0 | 0 | 0 | 0 | 0 | 0 | 0 | 0 | 0 | 0 | 0 | 0 | 0 | 0 | 0 | 0 | 0 | 0 | 0 | 0 | 0 | 0 | 4 | 0 | 0 | | | | | | |
| 7091 | 08326 | 07090 | 1 | | 0 | 0 | 0 | 0 | 0 | 0 | 0 | 0 | 0 | 0 | 0 | 0 | 0 | 0 | 0 | 0 | 0 | 0 | 0 | 0 | 0 | 0 | 1 | 0 | 0 | | | | | | |
| 7092 | 08327 | 07091 | 1 | | 0 | 0 | 0 | 0 | 0 | 0 | 0 | 0 | 0 | 0 | 0 | 0 | 0 | 0 | 0 | 0 | 0 | 0 | 0 | 0 | 0 | 0 | 1 | 0 | 0 | | | | | | |
| 7093 | 08328 | 07092 | 1 | | 0 | 0 | 0 | 0 | 0 | 0 | 0 | 0 | 0 | 0 | 0 | 0 | 0 | 0 | 0 | 0 | 0 | 0 | 0 | 0 | 0 | 0 | 1 | 0 | 0 | | | | | | |
| 7094 | 08329 | 07093 | 1 | | 0 | 0 | 0 | 0 | 0 | 0 | 0 | 0 | 0 | 0 | 0 | 0 | 0 | 0 | 0 | 0 | 0 | 0 | 0 | 0 | 0 | 0 | 1 | 0 | 0 | | | | | | |
| 7095 | 08330 | 07094 | 1 | | 0 | 0 | 0 | 0 | 0 | 0 | 0 | 0 | 0 | 0 | 0 | 0 | 0 | 0 | 0 | 0 | 0 | 0 | 0 | 0 | 0 | 0 | 1 | 0 | 0 | | | | | | |
| 7096 | 08331 | 07095 | 1 | | 0 | 0 | 0 | 0 | 0 | 0 | 0 | 0 | 0 | 0 | 0 | 0 | 0 | 0 | 0 | 0 | 0 | 0 | 0 | 0 | 0 | 0 | 1 | 0 | 0 | | | | | | |
| 7097 | 08332 | 07096 | 1 | | 0 | 0 | 0 | 0 | 0 | 0 | 0 | 0 | 0 | 0 | 0 | 0 | 0 | 0 | 0 | 0 | 0 | 0 | 0 | 0 | 0 | 0 | 1 | 0 | 0 | | | | | | |
| 7098 | 08333 | 07097 | 1 | | 0 | 0 | 0 | 0 | 0 | 0 | 0 | 0 | 0 | 0 | 0 | 0 | 0 | 0 | 0 | 0 | 0 | 0 | 0 | 0 | 0 | 0 | 1 | 0 | 0 | | | | | | |
| 7099 | 08334 | 07098 | 1 | | 0 | 0 | 0 | 0 | 0 | 0 | 0 | 0 | 0 | 0 | 0 | 0 | 0 | 0 | 0 | 0 | 0 | 0 | 0 | 0 | 0 | 0 | 1 | 0 | 0 | | | | | | |
| 7100 | 08335 | 07099 | 1 | | 0 | 0 | 0 | 0 | 0 | 0 | 0 | 0 | 0 | 0 | 0 | 0 | 0 | 0 | 0 | 0 | 0 | 0 | 0 | 0 | 0 | 0 | 1 | 0 | 0 | | | | | | |
| 7101 | 08336 | 07100 | 3 | | 0 | 0 | 0 | 0 | 0 | 0 | 0 | 0 | 0 | 0 | 0 | 0 | 0 | 0 | 0 | 0 | 0 | 0 | 0 | 0 | 0 | 0 | 3 | 0 | 0 | X17042 | 97.3 | 296 | 1 | 344 | 1182 |
| 7102 | 08337 | 07101 | 2 | | 0 | 0 | 0 | 0 | 0 | 0 | 0 | 0 | 0 | 0 | 0 | 0 | 0 | 0 | 0 | 0 | 0 | 0 | 0 | 0 | 0 | 0 | 2 | 0 | 0 | M81637 | 93.9 | 295 | 1 | 1293 | 1652 |
| 7103 | 08338 | 07102 | 2 | | 0 | 0 | 0 | 0 | 0 | 0 | 0 | 0 | 0 | 0 | 0 | 0 | 0 | 0 | 0 | 0 | 0 | 0 | 0 | 0 | 0 | 0 | 1 | 1 | 0 | | | | | | |
| 7104 | 08339 | 07103 | 9 | | 0 | 0 | 0 | 0 | 0 | 0 | 0 | 0 | 0 | 0 | 0 | 0 | 0 | 0 | 0 | 0 | 0 | 0 | 0 | 0 | 0 | 0 | 9 | 0 | 0 | M17017 | 93.2 | 413 | 1 | 874 | 1639 |
| 7105 | 08340 | 07104 | 1 | | 0 | 0 | 0 | 0 | 0 | 0 | 0 | 0 | 0 | 0 | 0 | 0 | 0 | 0 | 0 | 0 | 0 | 0 | 0 | 0 | 0 | 0 | 1 | 0 | 0 | | | | | | |
| 7106 | 08341 | 07105 | 2 | | 0 | 0 | 0 | 0 | 0 | 0 | 0 | 0 | 0 | 0 | 0 | 0 | 0 | 0 | 0 | 0 | 0 | 0 | 0 | 0 | 0 | 0 | 2 | 0 | 0 | | | | | | |
| 7107 | 08342 | 07106 | 1 | | 0 | 0 | 0 | 0 | 0 | 0 | 0 | 0 | 0 | 0 | 0 | 0 | 0 | 0 | 0 | 0 | 0 | 0 | 0 | 0 | 0 | 0 | 1 | 0 | 0 | M21130 | 100 | 297 | 1 | 34 | 448 |
| 7108 | 08343 | 07107 | 1 | | 0 | 0 | 0 | 0 | 0 | 0 | 0 | 0 | 0 | 0 | 0 | 0 | 0 | 0 | 0 | 0 | 0 | 0 | 0 | 0 | 0 | 0 | 1 | 0 | 0 | | | | | | |
| 7109 | 08344 | 07108 | 1 | | 0 | 0 | 0 | 0 | 0 | 0 | 0 | 0 | 0 | 0 | 0 | 0 | 0 | 0 | 0 | 0 | 0 | 0 | 0 | 0 | 0 | 0 | 1 | 0 | 0 | | | | | | |
| 7110 | 08345 | 07109 | 1 | | 0 | 0 | 0 | 0 | 0 | 0 | 0 | 0 | 0 | 0 | 0 | 0 | 0 | 0 | 0 | 0 | 0 | 0 | 0 | 0 | 0 | 0 | 1 | 0 | 0 | | | | | | |
| 7111 | 08346 | 07110 | 1 | | 0 | 0 | 0 | 0 | 0 | 0 | 0 | 0 | 0 | 0 | 0 | 0 | 0 | 0 | 0 | 0 | 0 | 0 | 0 | 0 | 0 | 0 | 1 | 0 | 0 | | | | | | |
| 7112 | 08347 | 07111 | 8 | | 0 | 0 | 0 | 0 | 0 | 0 | 0 | 0 | 0 | 0 | 0 | 0 | 0 | 0 | 0 | 0 | 0 | 0 | 0 | 0 | 0 | 0 | 8 | 0 | 0 | | | | | | |
| 7113 | 08348 | 07112 | 1 | | 0 | 0 | 0 | 0 | 0 | 0 | 0 | 0 | 0 | 0 | 0 | 0 | 0 | 0 | 0 | 0 | 0 | 0 | 0 | 0 | 0 | 0 | 1 | 0 | 0 | | | | | | |

Table 198

| | A | B | C | E | G | I | K | M | O | Q | S | U | W | Y | AA | AC | AE | AG | AI | AK | AM | AO | AQ | AS | AU | AW | AY | BA | BC | BE | BF | BG | BH | BI | BK |
|---|---|---|---|---|---|---|---|---|---|---|---|---|---|---|---|---|---|---|---|---|---|---|---|---|---|---|---|---|---|---|---|---|---|---|---|
| 7114 | 08349 | 07113 | 1 | | 0 | 0 | 0 | 0 | 0 | 0 | 0 | 0 | 0 | 0 | 0 | 0 | 0 | 0 | 0 | 0 | 0 | 0 | 0 | 0 | 0 | 0 | 1 | 0 | 0 | X17519 | 98.4 | 306 | 1 | 1624 | 2323 |
| 7115 | 08350 | 07114 | 1 | | 0 | 0 | 0 | 0 | 0 | 0 | 0 | 0 | 0 | 0 | 0 | 0 | 0 | 0 | 0 | 0 | 0 | 0 | 0 | 0 | 0 | 0 | 1 | 0 | 0 | | | | | | |
| 7116 | 08351 | 07115 | 2 | | 0 | 0 | 0 | 0 | 0 | 0 | 0 | 0 | 0 | 0 | 0 | 0 | 0 | 0 | 0 | 0 | 0 | 0 | 0 | 0 | 0 | 0 | 2 | 0 | 0 | | | | | | |
| 7117 | 08352 | 07116 | 1 | | 0 | 0 | 0 | 0 | 0 | 0 | 0 | 0 | 0 | 0 | 0 | 0 | 0 | 0 | 0 | 0 | 0 | 0 | 0 | 0 | 0 | 0 | 1 | 0 | 0 | | | | | | |
| 7118 | 08353 | 07117 | 1 | | 0 | 0 | 0 | 0 | 0 | 0 | 0 | 0 | 0 | 0 | 0 | 0 | 0 | 0 | 0 | 0 | 0 | 0 | 0 | 0 | 0 | 0 | 1 | 0 | 0 | | | | | | |
| 7119 | 08354 | 07118 | 1 | | 0 | 0 | 0 | 0 | 0 | 0 | 0 | 0 | 0 | 0 | 0 | 0 | 0 | 0 | 0 | 0 | 0 | 0 | 0 | 0 | 0 | 0 | 1 | 0 | 0 | | | | | | |
| 7120 | 08355 | 07119 | 2 | | 0 | 0 | 0 | 0 | 0 | 0 | 0 | 0 | 0 | 0 | 0 | 0 | 0 | 0 | 0 | 0 | 0 | 0 | 0 | 0 | 0 | 0 | 2 | 0 | 0 | M62505 | 97.1 | 204 | 1 | 2110 | 2328 |
| 7121 | 08356 | 07120 | 1 | | 0 | 0 | 0 | 0 | 0 | 0 | 0 | 0 | 0 | 0 | 0 | 0 | 0 | 0 | 0 | 0 | 0 | 0 | 0 | 0 | 0 | 0 | 1 | 0 | 0 | | | | | | |
| 7122 | 08357 | 07121 | 1 | | 0 | 0 | 0 | 0 | 0 | 0 | 0 | 0 | 0 | 0 | 0 | 0 | 0 | 0 | 0 | 0 | 0 | 0 | 0 | 0 | 0 | 0 | 1 | 0 | 0 | | | | | | |
| 7123 | 08358 | 07122 | 1 | | 0 | 0 | 0 | 0 | 0 | 0 | 0 | 0 | 0 | 0 | 0 | 0 | 0 | 0 | 0 | 0 | 0 | 0 | 0 | 0 | 0 | 0 | 1 | 0 | 0 | | | | | | |
| 7124 | 08359 | 07123 | 1 | | 0 | 0 | 0 | 0 | 0 | 0 | 0 | 0 | 0 | 0 | 0 | 0 | 0 | 0 | 0 | 0 | 0 | 0 | 0 | 0 | 0 | 0 | 1 | 0 | 0 | | | | | | |
| 7125 | 08360 | 07124 | 1 | | 0 | 0 | 0 | 0 | 0 | 0 | 0 | 0 | 0 | 0 | 0 | 0 | 0 | 0 | 0 | 0 | 0 | 0 | 0 | 0 | 0 | 0 | 1 | 0 | 0 | | | | | | |
| 7126 | 08361 | 07125 | 1 | | 0 | 0 | 0 | 0 | 0 | 0 | 0 | 0 | 0 | 0 | 0 | 0 | 0 | 0 | 0 | 0 | 0 | 0 | 0 | 0 | 0 | 0 | 1 | 0 | 0 | | | | | | |
| 7127 | 08362 | 07126 | 16 | | 0 | 0 | 0 | 0 | 0 | 0 | 0 | 0 | 0 | 0 | 0 | 0 | 0 | 0 | 0 | 0 | 0 | 0 | 0 | 0 | 0 | 0 | 16 | 0 | 0 | X55720 | 100 | 85 | 1 | 2450 | 2546 |
| 7128 | 08363 | 07127 | 1 | | 0 | 0 | 0 | 0 | 0 | 0 | 0 | 0 | 0 | 0 | 0 | 0 | 0 | 0 | 0 | 0 | 0 | 0 | 0 | 0 | 0 | 0 | 1 | 0 | 0 | | | | | | |
| 7129 | 08364 | 07128 | 1 | | 0 | 0 | 0 | 0 | 0 | 0 | 0 | 0 | 0 | 0 | 0 | 0 | 0 | 0 | 0 | 0 | 0 | 0 | 0 | 0 | 0 | 0 | 1 | 0 | 0 | | | | | | |
| 7130 | 08365 | 07129 | 1 | | 0 | 0 | 0 | 0 | 0 | 0 | 0 | 0 | 0 | 0 | 0 | 0 | 0 | 0 | 0 | 0 | 0 | 0 | 0 | 0 | 0 | 0 | 1 | 0 | 0 | | | | | | |
| 7131 | 08366 | 07130 | 1 | | 0 | 0 | 0 | 0 | 0 | 0 | 0 | 0 | 0 | 0 | 0 | 0 | 0 | 0 | 0 | 0 | 0 | 0 | 0 | 0 | 0 | 0 | 1 | 0 | 0 | L14076 | 95.8 | 337 | 1 | 1507 | 2076 |
| 7132 | 08367 | 07131 | 1 | | 0 | 0 | 0 | 0 | 0 | 0 | 0 | 0 | 0 | 0 | 0 | 0 | 0 | 0 | 0 | 0 | 0 | 0 | 0 | 0 | 0 | 0 | 1 | 0 | 0 | | | | | | |
| 7133 | 08368 | 07132 | 1 | | 0 | 0 | 0 | 0 | 0 | 0 | 0 | 0 | 0 | 0 | 0 | 0 | 0 | 0 | 0 | 0 | 0 | 0 | 0 | 0 | 0 | 0 | 1 | 0 | 0 | | | | | | |
| 7134 | 08369 | 07133 | 1 | | 0 | 0 | 0 | 0 | 0 | 0 | 0 | 0 | 0 | 0 | 0 | 0 | 0 | 0 | 0 | 0 | 0 | 0 | 0 | 0 | 0 | 0 | 1 | 0 | 0 | | | | | | |
| 7135 | 08371 | 07134 | 1 | | 0 | 0 | 0 | 0 | 0 | 0 | 0 | 0 | 0 | 0 | 0 | 0 | 0 | 0 | 0 | 0 | 0 | 0 | 0 | 0 | 0 | 0 | 1 | 0 | 0 | | | | | | |
| 7136 | 08372 | 07135 | 1 | | 0 | 0 | 0 | 0 | 0 | 0 | 0 | 0 | 0 | 0 | 0 | 0 | 0 | 0 | 0 | 0 | 0 | 0 | 0 | 0 | 0 | 0 | 1 | 0 | 0 | | | | | | |
| 7137 | 08373 | 07136 | 1 | | 0 | 0 | 0 | 0 | 0 | 0 | 0 | 0 | 0 | 0 | 0 | 0 | 0 | 0 | 0 | 0 | 0 | 0 | 0 | 0 | 0 | 0 | 1 | 0 | 0 | | | | | | |
| 7138 | 08374 | 07137 | 1 | | 0 | 0 | 0 | 0 | 0 | 0 | 0 | 0 | 0 | 0 | 0 | 0 | 0 | 0 | 0 | 0 | 0 | 0 | 0 | 0 | 0 | 0 | 1 | 0 | 0 | | | | | | |
| 7139 | 08375 | 07138 | 3 | | 0 | 0 | 0 | 0 | 0 | 0 | 0 | 0 | 0 | 0 | 0 | 0 | 0 | 0 | 0 | 0 | 0 | 0 | 0 | 0 | 0 | 0 | 3 | 0 | 0 | M69043 | 99.3 | 301 | 1 | 1250 | 1550 |
| 7140 | 08376 | 07139 | 1 | | 0 | 0 | 0 | 0 | 0 | 0 | 0 | 0 | 0 | 0 | 0 | 0 | 0 | 0 | 0 | 0 | 0 | 0 | 0 | 0 | 0 | 0 | 1 | 0 | 0 | | | | | | |
| 7141 | 08377 | 07140 | 1 | | 0 | 0 | 0 | 0 | 0 | 0 | 0 | 0 | 0 | 0 | 0 | 0 | 0 | 0 | 0 | 0 | 0 | 0 | 0 | 0 | 0 | 0 | 1 | 0 | 0 | | | | | | |
| 7142 | 08378 | 07141 | 1 | | 0 | 0 | 0 | 0 | 0 | 0 | 0 | 0 | 0 | 0 | 0 | 0 | 0 | 0 | 0 | 0 | 0 | 0 | 0 | 0 | 0 | 0 | 1 | 0 | 0 | | | | | | |
| 7143 | 08379 | 07142 | 1 | | 0 | 0 | 0 | 0 | 0 | 0 | 0 | 0 | 0 | 0 | 0 | 0 | 0 | 0 | 0 | 0 | 0 | 0 | 0 | 0 | 0 | 0 | 1 | 0 | 0 | | | | | | |
| 7144 | 08380 | 07143 | 2 | | 0 | 0 | 0 | 0 | 0 | 0 | 0 | 0 | 0 | 0 | 0 | 0 | 0 | 0 | 0 | 0 | 0 | 0 | 0 | 0 | 0 | 0 | 2 | 0 | 0 | | | | | | |
| 7145 | 08381 | 07144 | 1 | | 0 | 0 | 0 | 0 | 0 | 0 | 0 | 0 | 0 | 0 | 0 | 0 | 0 | 0 | 0 | 0 | 0 | 0 | 0 | 0 | 0 | 0 | 1 | 0 | 0 | | | | | | |
| 7146 | 08382 | 07145 | 1 | | 0 | 0 | 0 | 0 | 0 | 0 | 0 | 0 | 0 | 0 | 0 | 0 | 0 | 0 | 0 | 0 | 0 | 0 | 0 | 0 | 0 | 0 | 1 | 0 | 0 | | | | | | |
| 7147 | 08383 | 07146 | 5 | | 0 | 0 | 0 | 0 | 0 | 0 | 0 | 0 | 0 | 0 | 0 | 0 | 0 | 0 | 0 | 0 | 0 | 0 | 0 | 0 | 0 | 0 | 5 | 0 | 0 | U00115 | 97.2 | 180 | 1 | 3034 | 3536 |
| 7148 | 08384 | 07147 | 2 | | 0 | 0 | 0 | 0 | 0 | 0 | 0 | 0 | 0 | 0 | 0 | 0 | 0 | 0 | 0 | 0 | 0 | 0 | 0 | 0 | 0 | 0 | 1 | 1 | 0 | | | | | | |
| 7149 | 08385 | 07148 | 1 | | 0 | 0 | 0 | 0 | 0 | 0 | 0 | 0 | 0 | 0 | 0 | 0 | 0 | 0 | 0 | 0 | 0 | 0 | 0 | 0 | 0 | 0 | 1 | 0 | 0 | | | | | | |

Table 199

| | A | B | C | E | G | I | K | M | O | Q | S | U | W | Y | AA | AC | AE | AG | AI | AK | AM | AO | AQ | AS | AU | AW | AY | BA | BC | BE | BF | BG | BH | BI | BK |
|---|---|---|---|---|---|---|---|---|---|---|---|---|---|---|---|---|---|---|---|---|---|---|---|---|---|---|---|---|---|---|---|---|---|---|---|
| 7150 | 08386 | 07149 | 1 | | 0 | 0 | 0 | 0 | 0 | 0 | 0 | 0 | 0 | 0 | 0 | 0 | 0 | 0 | 0 | 0 | 0 | 0 | 0 | 0 | 0 | 0 | 1 | 0 | 0 | | | | | | |
| 7151 | 08387 | 07150 | 2 | | 0 | 0 | 0 | 0 | 0 | 0 | 0 | 0 | 0 | 0 | 0 | 0 | 0 | 0 | 0 | 0 | 0 | 0 | 0 | 0 | 0 | 0 | 1 | 1 | 0 | | | | | | |
| 7152 | 08388 | 07151 | 1 | | 0 | 0 | 0 | 0 | 0 | 0 | 0 | 0 | 0 | 0 | 0 | 0 | 0 | 0 | 0 | 0 | 0 | 0 | 0 | 0 | 0 | 0 | 1 | 0 | 0 | | | | | | |
| 7153 | 08389 | 07152 | 4 | | 0 | 0 | 0 | 0 | 0 | 0 | 0 | 0 | 0 | 0 | 0 | 0 | 0 | 0 | 0 | 0 | 0 | 0 | 0 | 0 | 0 | 0 | 3 | 1 | 0 | | | | | | |
| 7154 | 08390 | 07153 | 1 | | 0 | 0 | 0 | 0 | 0 | 0 | 0 | 0 | 0 | 0 | 0 | 0 | 0 | 0 | 0 | 0 | 0 | 0 | 0 | 0 | 0 | 0 | 1 | 0 | 0 | | | | | | |
| 7155 | 08391 | 07154 | 2 | | 0 | 0 | 0 | 0 | 0 | 0 | 0 | 0 | 0 | 0 | 0 | 0 | 0 | 0 | 0 | 0 | 0 | 0 | 0 | 0 | 0 | 0 | 2 | 0 | 0 | | | | | | |
| 7156 | 08392 | 07155 | 1 | | 0 | 0 | 0 | 0 | 0 | 0 | 0 | 0 | 0 | 0 | 0 | 0 | 0 | 0 | 0 | 0 | 0 | 0 | 0 | 0 | 0 | 0 | 1 | 0 | 0 | | | | | | |
| 7157 | 08393 | 07156 | 1 | | 0 | 0 | 0 | 0 | 0 | 0 | 0 | 0 | 0 | 0 | 0 | 0 | 0 | 0 | 0 | 0 | 0 | 0 | 0 | 0 | 0 | 0 | 1 | 0 | 0 | X71427 | 95.5 | 292 | 1 | 934 | 1678 |
| 7158 | 08394 | 07157 | 1 | | 0 | 0 | 0 | 0 | 0 | 0 | 0 | 0 | 0 | 0 | 0 | 0 | 0 | 0 | 0 | 0 | 0 | 0 | 0 | 0 | 0 | 0 | 1 | 0 | 0 | | | | | | |
| 7159 | 08395 | 07158 | 4 | | 0 | 0 | 0 | 0 | 0 | 0 | 0 | 0 | 0 | 0 | 0 | 0 | 0 | 0 | 0 | 0 | 0 | 0 | 0 | 0 | 0 | 0 | 4 | 0 | 0 | | | | | | |
| 7160 | 08396 | 07159 | 2 | | 0 | 0 | 0 | 0 | 0 | 0 | 0 | 0 | 0 | 0 | 0 | 0 | 0 | 0 | 0 | 0 | 0 | 0 | 0 | 0 | 0 | 0 | 1 | 0 | 1 | X75304 | 98.9 | 90 | 1 | #### | 10300 |
| 7161 | 08397 | 07160 | 1 | | 0 | 0 | 0 | 0 | 0 | 0 | 0 | 0 | 0 | 0 | 0 | 0 | 0 | 0 | 0 | 0 | 0 | 0 | 0 | 0 | 0 | 0 | 1 | 0 | 0 | | | | | | |
| 7162 | 08398 | 07161 | 1 | | 0 | 0 | 0 | 0 | 0 | 0 | 0 | 0 | 0 | 0 | 0 | 0 | 0 | 0 | 0 | 0 | 0 | 0 | 0 | 0 | 0 | 0 | 1 | 0 | 0 | | | | | | |
| 7163 | 08399 | 07162 | 1 | | 0 | 0 | 0 | 0 | 0 | 0 | 0 | 0 | 0 | 0 | 0 | 0 | 0 | 0 | 0 | 0 | 0 | 0 | 0 | 0 | 0 | 0 | 1 | 0 | 0 | | | | | | |
| 7164 | 08400 | 07163 | 1 | | 0 | 0 | 0 | 0 | 0 | 0 | 0 | 0 | 0 | 0 | 0 | 0 | 0 | 0 | 0 | 0 | 0 | 0 | 0 | 0 | 0 | 0 | 1 | 0 | 0 | L08238 | 96.7 | 123 | 1 | 2681 | 2805 |
| 7165 | 08402 | 07164 | 1 | | 0 | 0 | 0 | 0 | 0 | 0 | 0 | 0 | 0 | 0 | 0 | 0 | 0 | 0 | 0 | 0 | 0 | 0 | 0 | 0 | 0 | 0 | 1 | 0 | 0 | | | | | | |
| 7166 | 08403 | 07165 | 1 | | 0 | 0 | 0 | 0 | 0 | 0 | 0 | 0 | 0 | 0 | 0 | 0 | 0 | 0 | 0 | 0 | 0 | 0 | 0 | 0 | 0 | 0 | 1 | 0 | 0 | | | | | | |
| 7167 | 08404 | 07166 | 2 | | 0 | 0 | 0 | 0 | 0 | 0 | 0 | 0 | 0 | 0 | 0 | 0 | 0 | 0 | 0 | 0 | 0 | 0 | 0 | 0 | 0 | 0 | 2 | 0 | 0 | | | | | | |
| 7168 | 08405 | 07167 | 1 | | 0 | 0 | 0 | 0 | 0 | 0 | 0 | 0 | 0 | 0 | 0 | 0 | 0 | 0 | 0 | 0 | 0 | 0 | 0 | 0 | 0 | 0 | 1 | 0 | 0 | | | | | | |
| 7169 | 08406 | 07168 | 1 | | 0 | 0 | 0 | 0 | 0 | 0 | 0 | 0 | 0 | 0 | 0 | 0 | 0 | 0 | 0 | 0 | 0 | 0 | 0 | 0 | 0 | 0 | 1 | 0 | 0 | | | | | | |
| 7170 | 08407 | 07169 | 1 | | 0 | 0 | 0 | 0 | 0 | 0 | 0 | 0 | 0 | 0 | 0 | 0 | 0 | 0 | 0 | 0 | 0 | 0 | 0 | 0 | 0 | 0 | 1 | 0 | 0 | | | | | | |
| 7171 | 08408 | 07170 | 1 | | 0 | 0 | 0 | 0 | 0 | 0 | 0 | 0 | 0 | 0 | 0 | 0 | 0 | 0 | 0 | 0 | 0 | 0 | 0 | 0 | 0 | 0 | 1 | 0 | 0 | | | | | | |
| 7172 | 08409 | 07171 | 1 | | 0 | 0 | 0 | 0 | 0 | 0 | 0 | 0 | 0 | 0 | 0 | 0 | 0 | 0 | 0 | 0 | 0 | 0 | 0 | 0 | 0 | 0 | 1 | 0 | 0 | | | | | | |
| 7173 | 08410 | 07172 | 1 | | 0 | 0 | 0 | 0 | 0 | 0 | 0 | 0 | 0 | 0 | 0 | 0 | 0 | 0 | 0 | 0 | 0 | 0 | 0 | 0 | 0 | 0 | 1 | 0 | 0 | | | | | | |
| 7174 | 08411 | 07173 | 1 | | 0 | 0 | 0 | 0 | 0 | 0 | 0 | 0 | 0 | 0 | 0 | 0 | 0 | 0 | 0 | 0 | 0 | 0 | 0 | 0 | 0 | 0 | 1 | 0 | 0 | | | | | | |
| 7175 | 08412 | 07174 | 2 | | 0 | 0 | 0 | 0 | 0 | 0 | 0 | 0 | 0 | 0 | 0 | 0 | 0 | 0 | 0 | 0 | 0 | 0 | 0 | 0 | 0 | 0 | 2 | 0 | 0 | | | | | | |
| 7176 | 08413 | 07175 | 1 | | 0 | 0 | 0 | 0 | 0 | 0 | 0 | 0 | 0 | 0 | 0 | 0 | 0 | 0 | 0 | 0 | 0 | 0 | 0 | 0 | 0 | 0 | 1 | 0 | 0 | | | | | | |
| 7177 | 08414 | 07176 | 1 | | 0 | 0 | 0 | 0 | 0 | 0 | 0 | 0 | 0 | 0 | 0 | 0 | 0 | 0 | 0 | 0 | 0 | 0 | 0 | 0 | 0 | 0 | 1 | 0 | 0 | | | | | | |
| 7178 | 08415 | 07177 | 1 | | 0 | 0 | 0 | 0 | 0 | 0 | 0 | 0 | 0 | 0 | 0 | 0 | 0 | 0 | 0 | 0 | 0 | 0 | 0 | 0 | 0 | 0 | 1 | 0 | 0 | | | | | | |
| 7179 | 08416 | 07178 | 1 | | 0 | 0 | 0 | 0 | 0 | 0 | 0 | 0 | 0 | 0 | 0 | 0 | 0 | 0 | 0 | 0 | 0 | 0 | 0 | 0 | 0 | 0 | 1 | 0 | 0 | | | | | | |
| 7180 | 08417 | 07179 | 1 | | 0 | 0 | 0 | 0 | 0 | 0 | 0 | 0 | 0 | 0 | 0 | 0 | 0 | 0 | 0 | 0 | 0 | 0 | 0 | 0 | 0 | 0 | 1 | 0 | 0 | | | | | | |
| 7181 | 08418 | 07180 | 1 | | 0 | 0 | 0 | 0 | 0 | 0 | 0 | 0 | 0 | 0 | 0 | 0 | 0 | 0 | 0 | 0 | 0 | 0 | 0 | 0 | 0 | 0 | 1 | 0 | 0 | M84721 | 99.4 | 345 | 1 | 3260 | 3680 |
| 7182 | 08419 | 07181 | 1 | | 0 | 0 | 0 | 0 | 0 | 0 | 0 | 0 | 0 | 0 | 0 | 0 | 0 | 0 | 0 | 0 | 0 | 0 | 0 | 0 | 0 | 0 | 1 | 0 | 0 | | | | | | |
| 7183 | 08420 | 07182 | 2 | | 0 | 0 | 0 | 0 | 0 | 0 | 0 | 0 | 0 | 0 | 0 | 0 | 0 | 0 | 0 | 0 | 0 | 0 | 0 | 0 | 0 | 0 | 2 | 0 | 0 | | | | | | |
| 7184 | 08421 | 07183 | 1 | | 0 | 0 | 0 | 0 | 0 | 0 | 0 | 0 | 0 | 0 | 0 | 0 | 0 | 0 | 0 | 0 | 0 | 0 | 0 | 0 | 0 | 0 | 1 | 0 | 0 | | | | | | |
| 7185 | 08422 | 07184 | 1 | | 0 | 0 | 0 | 0 | 0 | 0 | 0 | 0 | 0 | 0 | 0 | 0 | 0 | 0 | 0 | 0 | 0 | 0 | 0 | 0 | 0 | 0 | 1 | 0 | 0 | | | | | | |

Table 200

| | A | B | C | E | G | I | K | M | O | Q | S | U | W | Y | AA | AC | AE | AG | AI | AK | AM | AO | AQ | AS | AU | AW | AY | BA | BC | BE | BF | BG | BH | BI | BK |
|---|---|---|---|---|---|---|---|---|---|---|---|---|---|---|---|---|---|---|---|---|---|---|---|---|---|---|---|---|---|---|---|---|---|---|---|
| 7186 | 08423 | 07185 | 1 | | 0 | 0 | 0 | 0 | 0 | 0 | 0 | 0 | 0 | 0 | 0 | 0 | 0 | 0 | 0 | 0 | 0 | 0 | 0 | 0 | 0 | 0 | 1 | 0 | 0 | | | | | | |
| 7187 | 08424 | 07186 | 3 | | 0 | 0 | 0 | 0 | 0 | 0 | 0 | 0 | 0 | 0 | 0 | 0 | 0 | 0 | 0 | 0 | 0 | 0 | 0 | 0 | 0 | 0 | 3 | 0 | 0 | | | | | | |
| 7188 | 08425 | 07187 | 1 | | 0 | 0 | 0 | 0 | 0 | 0 | 0 | 0 | 0 | 0 | 0 | 0 | 0 | 0 | 0 | 0 | 0 | 0 | 0 | 0 | 0 | 0 | 1 | 0 | 0 | | | | | | |
| 7189 | 08426 | 07188 | 1 | | 0 | 0 | 0 | 0 | 0 | 0 | 0 | 0 | 0 | 0 | 0 | 0 | 0 | 0 | 0 | 0 | 0 | 0 | 0 | 0 | 0 | 0 | 1 | 0 | 0 | | | | | | |
| 7190 | 08427 | 07189 | 1 | | 0 | 0 | 0 | 0 | 0 | 0 | 0 | 0 | 0 | 0 | 0 | 0 | 0 | 0 | 0 | 0 | 0 | 0 | 0 | 0 | 0 | 0 | 1 | 0 | 0 | | | | | | |
| 7191 | 08428 | 07190 | 1 | | 0 | 0 | 0 | 0 | 0 | 0 | 0 | 0 | 0 | 0 | 0 | 0 | 0 | 0 | 0 | 0 | 0 | 0 | 0 | 0 | 0 | 0 | 1 | 0 | 0 | | | | | | |
| 7192 | 08429 | 07191 | 1 | | 0 | 0 | 0 | 0 | 0 | 0 | 0 | 0 | 0 | 0 | 0 | 0 | 0 | 0 | 0 | 0 | 0 | 0 | 0 | 0 | 0 | 0 | 1 | 0 | 0 | | | | | | |
| 7193 | 08430 | 07192 | 1 | | 0 | 0 | 0 | 0 | 0 | 0 | 0 | 0 | 0 | 0 | 0 | 0 | 0 | 0 | 0 | 0 | 0 | 0 | 0 | 0 | 0 | 0 | 1 | 0 | 0 | | | | | | |
| 7194 | 08431 | 07193 | 1 | | 0 | 0 | 0 | 0 | 0 | 0 | 0 | 0 | 0 | 0 | 0 | 0 | 0 | 0 | 0 | 0 | 0 | 0 | 0 | 0 | 0 | 0 | 1 | 0 | 0 | | | | | | |
| 7195 | 08432 | 07194 | 2 | | 0 | 0 | 0 | 0 | 0 | 0 | 0 | 0 | 0 | 0 | 0 | 0 | 0 | 0 | 0 | 0 | 0 | 0 | 0 | 0 | 0 | 0 | 2 | 0 | 0 | | | | | | |
| 7196 | 08434 | 07195 | 1 | | 0 | 0 | 0 | 0 | 0 | 0 | 0 | 0 | 0 | 0 | 0 | 0 | 0 | 0 | 0 | 0 | 0 | 0 | 0 | 0 | 0 | 0 | 1 | 0 | 0 | | | | | | |
| 7197 | 08435 | 07196 | 2 | | 0 | 0 | 0 | 0 | 0 | 0 | 0 | 0 | 0 | 0 | 0 | 0 | 0 | 0 | 0 | 0 | 0 | 0 | 0 | 0 | 0 | 0 | 2 | 0 | 0 | | | | | | |
| 7198 | 08436 | 07197 | 1 | | 0 | 0 | 0 | 0 | 0 | 0 | 0 | 0 | 0 | 0 | 0 | 0 | 0 | 0 | 0 | 0 | 0 | 0 | 0 | 0 | 0 | 0 | 1 | 0 | 0 | | | | | | |
| 7199 | 08437 | 07198 | 1 | | 0 | 0 | 0 | 0 | 0 | 0 | 0 | 0 | 0 | 0 | 0 | 0 | 0 | 0 | 0 | 0 | 0 | 0 | 0 | 0 | 0 | 0 | 1 | 0 | 0 | | | | | | |
| 7200 | 08438 | 07199 | 4 | | 0 | 0 | 0 | 0 | 0 | 0 | 0 | 0 | 0 | 0 | 0 | 0 | 0 | 0 | 0 | 0 | 0 | 0 | 0 | 0 | 0 | 0 | 4 | 0 | 0 | | | | | | |
| 7201 | 08439 | 07200 | 1 | | 0 | 0 | 0 | 0 | 0 | 0 | 0 | 0 | 0 | 0 | 0 | 0 | 0 | 0 | 0 | 0 | 0 | 0 | 0 | 0 | 0 | 0 | 1 | 0 | 0 | | | | | | |
| 7202 | 08440 | 07201 | 1 | | 0 | 0 | 0 | 0 | 0 | 0 | 0 | 0 | 0 | 0 | 0 | 0 | 0 | 0 | 0 | 0 | 0 | 0 | 0 | 0 | 0 | 0 | 1 | 0 | 0 | | | | | | |
| 7203 | 08441 | 07202 | 1 | | 0 | 0 | 0 | 0 | 0 | 0 | 0 | 0 | 0 | 0 | 0 | 0 | 0 | 0 | 0 | 0 | 0 | 0 | 0 | 0 | 0 | 0 | 1 | 0 | 0 | | | | | | |
| 7204 | 08442 | 07203 | 1 | | 0 | 0 | 0 | 0 | 0 | 0 | 0 | 0 | 0 | 0 | 0 | 0 | 0 | 0 | 0 | 0 | 0 | 0 | 0 | 0 | 0 | 0 | 1 | 0 | 0 | | | | | | |
| 7205 | 08443 | 07204 | 1 | | 0 | 0 | 0 | 0 | 0 | 0 | 0 | 0 | 0 | 0 | 0 | 0 | 0 | 0 | 0 | 0 | 0 | 0 | 0 | 0 | 0 | 0 | 1 | 0 | 0 | | | | | | |
| 7206 | 08444 | 07205 | 2 | | 0 | 0 | 0 | 0 | 0 | 0 | 0 | 0 | 0 | 0 | 0 | 0 | 0 | 0 | 0 | 0 | 0 | 0 | 0 | 0 | 0 | 0 | 2 | 0 | 0 | | | | | | |
| 7207 | 08445 | 07206 | 1 | | 0 | 0 | 0 | 0 | 0 | 0 | 0 | 0 | 0 | 0 | 0 | 0 | 0 | 0 | 0 | 0 | 0 | 0 | 0 | 0 | 0 | 0 | 1 | 0 | 0 | | | | | | |
| 7208 | 08446 | 07207 | 1 | | 0 | 0 | 0 | 0 | 0 | 0 | 0 | 0 | 0 | 0 | 0 | 0 | 0 | 0 | 0 | 0 | 0 | 0 | 0 | 0 | 0 | 0 | 1 | 0 | 0 | | | | | | |
| 7209 | 08447 | 07208 | 1 | | 0 | 0 | 0 | 0 | 0 | 0 | 0 | 0 | 0 | 0 | 0 | 0 | 0 | 0 | 0 | 0 | 0 | 0 | 0 | 0 | 0 | 0 | 1 | 0 | 0 | | | | | | |
| 7210 | 08448 | 07209 | 1 | | 0 | 0 | 0 | 0 | 0 | 0 | 0 | 0 | 0 | 0 | 0 | 0 | 0 | 0 | 0 | 0 | 0 | 0 | 0 | 0 | 0 | 0 | 1 | 0 | 0 | | | | | | |
| 7211 | 08449 | 07210 | 1 | | 0 | 0 | 0 | 0 | 0 | 0 | 0 | 0 | 0 | 0 | 0 | 0 | 0 | 0 | 0 | 0 | 0 | 0 | 0 | 0 | 0 | 0 | 1 | 0 | 0 | | | | | | |
| 7212 | 08450 | 07211 | 1 | | 0 | 0 | 0 | 0 | 0 | 0 | 0 | 0 | 0 | 0 | 0 | 0 | 0 | 0 | 0 | 0 | 0 | 0 | 0 | 0 | 0 | 0 | 1 | 0 | 0 | | | | | | |
| 7213 | 08451 | 07212 | 1 | | 0 | 0 | 0 | 0 | 0 | 0 | 0 | 0 | 0 | 0 | 0 | 0 | 0 | 0 | 0 | 0 | 0 | 0 | 0 | 0 | 0 | 0 | 1 | 0 | 0 | | | | | | |
| 7214 | 08452 | 07213 | 1 | | 0 | 0 | 0 | 0 | 0 | 0 | 0 | 0 | 0 | 0 | 0 | 0 | 0 | 0 | 0 | 0 | 0 | 0 | 0 | 0 | 0 | 0 | 1 | 0 | 0 | | | | | | |
| 7215 | 08453 | 07214 | 1 | | 0 | 0 | 0 | 0 | 0 | 0 | 0 | 0 | 0 | 0 | 0 | 0 | 0 | 0 | 0 | 0 | 0 | 0 | 0 | 0 | 0 | 0 | 1 | 0 | 0 | | | | | | |
| 7216 | 08454 | 07215 | 1 | | 0 | 0 | 0 | 0 | 0 | 0 | 0 | 0 | 0 | 0 | 0 | 0 | 0 | 0 | 0 | 0 | 0 | 0 | 0 | 0 | 0 | 0 | 1 | 0 | 0 | L24122 | 99.4 | 161 | 1 | 1342 | 1502 |
| 7217 | 08455 | 07216 | 1 | | 0 | 0 | 0 | 0 | 0 | 0 | 0 | 0 | 0 | 0 | 0 | 0 | 0 | 0 | 0 | 0 | 0 | 0 | 0 | 0 | 0 | 0 | 1 | 0 | 0 | | | | | | |
| 7218 | 08456 | 07217 | 1 | | 0 | 0 | 0 | 0 | 0 | 0 | 0 | 0 | 0 | 0 | 0 | 0 | 0 | 0 | 0 | 0 | 0 | 0 | 0 | 0 | 0 | 0 | 1 | 0 | 0 | | | | | | |
| 7219 | 08457 | 07218 | 1 | | 0 | 0 | 0 | 0 | 0 | 0 | 0 | 0 | 0 | 0 | 0 | 0 | 0 | 0 | 0 | 0 | 0 | 0 | 0 | 0 | 0 | 0 | 1 | 0 | 0 | | | | | | |
| 7220 | 08458 | 07219 | 2 | | 0 | 0 | 0 | 0 | 0 | 0 | 0 | 0 | 0 | 0 | 0 | 0 | 0 | 0 | 0 | 0 | 0 | 0 | 0 | 0 | 0 | 0 | 2 | 0 | 0 | | | | | | |
| 7221 | 08459 | 07220 | 1 | | 0 | 0 | 0 | 0 | 0 | 0 | 0 | 0 | 0 | 0 | 0 | 0 | 0 | 0 | 0 | 0 | 0 | 0 | 0 | 0 | 0 | 0 | 1 | 0 | 0 | | | | | | |

Table 201

| | A | B | C | E | G | I | K | M | O | Q | S | U | W | Y | AA | AC | AE | AG | AI | AK | AM | AO | AQ | AS | AU | AW | AY | BA | BC | BE | BF | BG | BH | BI | BK |
|---|---|---|---|---|---|---|---|---|---|---|---|---|---|---|---|---|---|---|---|---|---|---|---|---|---|---|---|---|---|---|---|---|---|---|---|
| 7222 | 08460 | 07221 | 2 | | 0 | 0 | 0 | 0 | 0 | 0 | 0 | 0 | 0 | 0 | 0 | 0 | 0 | 0 | 0 | 0 | 0 | 0 | 0 | 0 | 0 | 0 | 2 | 0 | 0 | | | | | | |
| 7223 | 08461 | 07222 | 1 | | 0 | 0 | 0 | 0 | 0 | 0 | 0 | 0 | 0 | 0 | 0 | 0 | 0 | 0 | 0 | 0 | 0 | 0 | 0 | 0 | 0 | 0 | 1 | 0 | 0 | | | | | | |
| 7224 | 08462 | 07223 | 1 | | 0 | 0 | 0 | 0 | 0 | 0 | 0 | 0 | 0 | 0 | 0 | 0 | 0 | 0 | 0 | 0 | 0 | 0 | 0 | 0 | 0 | 0 | 1 | 0 | 0 | | | | | | |
| 7225 | 08463 | 07224 | 1 | | 0 | 0 | 0 | 0 | 0 | 0 | 0 | 0 | 0 | 0 | 0 | 0 | 0 | 0 | 0 | 0 | 0 | 0 | 0 | 0 | 0 | 0 | 1 | 0 | 0 | | | | | | |
| 7226 | 08464 | 07225 | 1 | | 0 | 0 | 0 | 0 | 0 | 0 | 0 | 0 | 0 | 0 | 0 | 0 | 0 | 0 | 0 | 0 | 0 | 0 | 0 | 0 | 0 | 0 | 1 | 0 | 0 | | | | | | |
| 7227 | 08465 | 07226 | 1 | | 0 | 0 | 0 | 0 | 0 | 0 | 0 | 0 | 0 | 0 | 0 | 0 | 0 | 0 | 0 | 0 | 0 | 0 | 0 | 0 | 0 | 0 | 1 | 0 | 0 | | | | | | |
| 7228 | 08466 | 07227 | 1 | | 0 | 0 | 0 | 0 | 0 | 0 | 0 | 0 | 0 | 0 | 0 | 0 | 0 | 0 | 0 | 0 | 0 | 0 | 0 | 0 | 0 | 0 | 1 | 0 | 0 | | | | | | |
| 7229 | 08467 | 07228 | 1 | | 0 | 0 | 0 | 0 | 0 | 0 | 0 | 0 | 0 | 0 | 0 | 0 | 0 | 0 | 0 | 0 | 0 | 0 | 0 | 0 | 0 | 0 | 1 | 0 | 0 | | | | | | |
| 7230 | 08468 | 07229 | 1 | | 0 | 0 | 0 | 0 | 0 | 0 | 0 | 0 | 0 | 0 | 0 | 0 | 0 | 0 | 0 | 0 | 0 | 0 | 0 | 0 | 0 | 0 | 1 | 0 | 0 | | | | | | |
| 7231 | 08469 | 07230 | 1 | | 0 | 0 | 0 | 0 | 0 | 0 | 0 | 0 | 0 | 0 | 0 | 0 | 0 | 0 | 0 | 0 | 0 | 0 | 0 | 0 | 0 | 0 | 1 | 0 | 0 | | | | | | |
| 7232 | 08470 | 07231 | 2 | | 0 | 0 | 0 | 0 | 0 | 0 | 0 | 0 | 0 | 0 | 0 | 0 | 0 | 0 | 0 | 0 | 0 | 0 | 0 | 0 | 0 | 0 | 2 | 0 | 0 | | | | | | |
| 7233 | 08471 | 07232 | 1 | | 0 | 0 | 0 | 0 | 0 | 0 | 0 | 0 | 0 | 0 | 0 | 0 | 0 | 0 | 0 | 0 | 0 | 0 | 0 | 0 | 0 | 0 | 1 | 0 | 0 | | | | | | |
| 7234 | 08472 | 07233 | 1 | | 0 | 0 | 0 | 0 | 0 | 0 | 0 | 0 | 0 | 0 | 0 | 0 | 0 | 0 | 0 | 0 | 0 | 0 | 0 | 0 | 0 | 0 | 1 | 0 | 0 | | | | | | |
| 7235 | 08473 | 07234 | 1 | | 0 | 0 | 0 | 0 | 0 | 0 | 0 | 0 | 0 | 0 | 0 | 0 | 0 | 0 | 0 | 0 | 0 | 0 | 0 | 0 | 0 | 0 | 1 | 0 | 0 | | | | | | |
| 7236 | 08474 | 07235 | 1 | | 0 | 0 | 0 | 0 | 0 | 0 | 0 | 0 | 0 | 0 | 0 | 0 | 0 | 0 | 0 | 0 | 0 | 0 | 0 | 0 | 0 | 0 | 1 | 0 | 0 | | | | | | |
| 7237 | 08475 | 07236 | 1 | | 0 | 0 | 0 | 0 | 0 | 0 | 0 | 0 | 0 | 0 | 0 | 0 | 0 | 0 | 0 | 0 | 0 | 0 | 0 | 0 | 0 | 0 | 1 | 0 | 0 | | | | | | |
| 7238 | 08476 | 07237 | 1 | | 0 | 0 | 0 | 0 | 0 | 0 | 0 | 0 | 0 | 0 | 0 | 0 | 0 | 0 | 0 | 0 | 0 | 0 | 0 | 0 | 0 | 0 | 1 | 0 | 0 | | | | | | |
| 7239 | 08477 | 07238 | 2 | | 0 | 0 | 0 | 0 | 0 | 0 | 0 | 0 | 0 | 0 | 0 | 0 | 0 | 0 | 0 | 0 | 0 | 0 | 0 | 0 | 0 | 0 | 2 | 0 | 0 | | | | | | |
| 7240 | 08478 | 07239 | 1 | | 0 | 0 | 0 | 0 | 0 | 0 | 0 | 0 | 0 | 0 | 0 | 0 | 0 | 0 | 0 | 0 | 0 | 0 | 0 | 0 | 0 | 0 | 1 | 0 | 0 | | | | | | |
| 7241 | 08479 | 07240 | 1 | | 0 | 0 | 0 | 0 | 0 | 0 | 0 | 0 | 0 | 0 | 0 | 0 | 0 | 0 | 0 | 0 | 0 | 0 | 0 | 0 | 0 | 0 | 1 | 0 | 0 | | | | | | |
| 7242 | 08480 | 07241 | 1 | | 0 | 0 | 0 | 0 | 0 | 0 | 0 | 0 | 0 | 0 | 0 | 0 | 0 | 0 | 0 | 0 | 0 | 0 | 0 | 0 | 0 | 0 | 1 | 0 | 0 | | | | | | |
| 7243 | 08481 | 07242 | 1 | | 0 | 0 | 0 | 0 | 0 | 0 | 0 | 0 | 0 | 0 | 0 | 0 | 0 | 0 | 0 | 0 | 0 | 0 | 0 | 0 | 0 | 0 | 1 | 0 | 0 | | | | | | |
| 7244 | 08482 | 07243 | 1 | | 0 | 0 | 0 | 0 | 0 | 0 | 0 | 0 | 0 | 0 | 0 | 0 | 0 | 0 | 0 | 0 | 0 | 0 | 0 | 0 | 0 | 0 | 1 | 0 | 0 | | | | | | |
| 7245 | 08483 | 07244 | 1 | | 0 | 0 | 0 | 0 | 0 | 0 | 0 | 0 | 0 | 0 | 0 | 0 | 0 | 0 | 0 | 0 | 0 | 0 | 0 | 0 | 0 | 0 | 1 | 0 | 0 | | | | | | |
| 7246 | 08484 | 07245 | 1 | | 0 | 0 | 0 | 0 | 0 | 0 | 0 | 0 | 0 | 0 | 0 | 0 | 0 | 0 | 0 | 0 | 0 | 0 | 0 | 0 | 0 | 0 | 1 | 0 | 0 | | | | | | |
| 7247 | 08485 | 07246 | 1 | | 0 | 0 | 0 | 0 | 0 | 0 | 0 | 0 | 0 | 0 | 0 | 0 | 0 | 0 | 0 | 0 | 0 | 0 | 0 | 0 | 0 | 0 | 1 | 0 | 0 | | | | | | |
| 7248 | 08486 | 07247 | 1 | | 0 | 0 | 0 | 0 | 0 | 0 | 0 | 0 | 0 | 0 | 0 | 0 | 0 | 0 | 0 | 0 | 0 | 0 | 0 | 0 | 0 | 0 | 1 | 0 | 0 | | | | | | |
| 7249 | 08487 | 07248 | 1 | | 0 | 0 | 0 | 0 | 0 | 0 | 0 | 0 | 0 | 0 | 0 | 0 | 0 | 0 | 0 | 0 | 0 | 0 | 0 | 0 | 0 | 0 | 1 | 0 | 0 | | | | | | |
| 7250 | 08488 | 07249 | 1 | | 0 | 0 | 0 | 0 | 0 | 0 | 0 | 0 | 0 | 0 | 0 | 0 | 0 | 0 | 0 | 0 | 0 | 0 | 0 | 0 | 0 | 0 | 1 | 0 | 0 | | | | | | |
| 7251 | 08489 | 07250 | 1 | | 0 | 0 | 0 | 0 | 0 | 0 | 0 | 0 | 0 | 0 | 0 | 0 | 0 | 0 | 0 | 0 | 0 | 0 | 0 | 0 | 0 | 0 | 1 | 0 | 0 | | | | | | |
| 7252 | 08490 | 07251 | 1 | | 0 | 0 | 0 | 0 | 0 | 0 | 0 | 0 | 0 | 0 | 0 | 0 | 0 | 0 | 0 | 0 | 0 | 0 | 0 | 0 | 0 | 0 | 1 | 0 | 0 | | | | | | |
| 7253 | 08491 | 07252 | 1 | | 0 | 0 | 0 | 0 | 0 | 0 | 0 | 0 | 0 | 0 | 0 | 0 | 0 | 0 | 0 | 0 | 0 | 0 | 0 | 0 | 0 | 0 | 1 | 0 | 0 | | | | | | |
| 7254 | 08492 | 07253 | 1 | | 0 | 0 | 0 | 0 | 0 | 0 | 0 | 0 | 0 | 0 | 0 | 0 | 0 | 0 | 0 | 0 | 0 | 0 | 0 | 0 | 0 | 0 | 1 | 0 | 0 | | | | | | |
| 7255 | 08493 | 07254 | 1 | | 0 | 0 | 0 | 0 | 0 | 0 | 0 | 0 | 0 | 0 | 0 | 0 | 0 | 0 | 0 | 0 | 0 | 0 | 0 | 0 | 0 | 0 | 1 | 0 | 0 | | | | | | |
| 7256 | 08494 | 07255 | 1 | | 0 | 0 | 0 | 0 | 0 | 0 | 0 | 0 | 0 | 0 | 0 | 0 | 0 | 0 | 0 | 0 | 0 | 0 | 0 | 0 | 0 | 0 | 1 | 0 | 0 | | | | | | |
| 7257 | 08495 | 07256 | 1 | | 0 | 0 | 0 | 0 | 0 | 0 | 0 | 0 | 0 | 0 | 0 | 0 | 0 | 0 | 0 | 0 | 0 | 0 | 0 | 0 | 0 | 0 | 1 | 0 | 0 | | | | | | |

Table 202

| | A | B | C | E | G | I | K | M | O | Q | S | U | W | Y | AA | AC | AF | AG | AI | AK | AM | AO | AQ | AS | AU | AW | AY | BA | BC | BE | BF | BG | BH | BI | BK |
|---|---|---|---|---|---|---|---|---|---|---|---|---|---|---|---|---|---|---|---|---|---|---|---|---|---|---|---|---|---|---|---|---|---|---|---|
| 7258 | 08496 | 07257 | 1 | | 0 | 0 | 0 | 0 | 0 | 0 | 0 | 0 | 0 | 0 | 0 | 0 | 0 | 0 | 0 | 0 | 0 | 0 | 0 | 0 | 0 | 0 | 1 | 0 | 0 | | | | | | |
| 7259 | 08497 | 07258 | 1 | | 0 | 0 | 0 | 0 | 0 | 0 | 0 | 0 | 0 | 0 | 0 | 0 | 0 | 0 | 0 | 0 | 0 | 0 | 0 | 0 | 0 | 0 | 1 | 0 | 0 | | | | | | |
| 7260 | 08498 | 07259 | 1 | | 0 | 0 | 0 | 0 | 0 | 0 | 0 | 0 | 0 | 0 | 0 | 0 | 0 | 0 | 0 | 0 | 0 | 0 | 0 | 0 | 0 | 0 | 1 | 0 | 0 | | | | | | |
| 7261 | 08499 | 07260 | 2 | | 0 | 0 | 0 | 0 | 0 | 0 | 0 | 0 | 0 | 0 | 0 | 0 | 0 | 0 | 0 | 0 | 0 | 0 | 0 | 0 | 0 | 0 | 2 | 0 | 0 | | | | | | |
| 7262 | 08500 | 07261 | 1 | | 0 | 0 | 0 | 0 | 0 | 0 | 0 | 0 | 0 | 0 | 0 | 0 | 0 | 0 | 0 | 0 | 0 | 0 | 0 | 0 | 0 | 0 | 1 | 0 | 0 | | | | | | |
| 7263 | 08501 | 07262 | 1 | | 0 | 0 | 0 | 0 | 0 | 0 | 0 | 0 | 0 | 0 | 0 | 0 | 0 | 0 | 0 | 0 | 0 | 0 | 0 | 0 | 0 | 0 | 1 | 0 | 0 | S48568 | 99 | 302 | 1 | 753 | 1062 |
| 7264 | 08502 | 07263 | 1 | | 0 | 0 | 0 | 0 | 0 | 0 | 0 | 0 | 0 | 0 | 0 | 0 | 0 | 0 | 0 | 0 | 0 | 0 | 0 | 0 | 0 | 0 | 1 | 0 | 0 | | | | | | |
| 7265 | 08503 | 07264 | 1 | | 0 | 0 | 0 | 0 | 0 | 0 | 0 | 0 | 0 | 0 | 0 | 0 | 0 | 0 | 0 | 0 | 0 | 0 | 0 | 0 | 0 | 0 | 1 | 0 | 0 | | | | | | |
| 7266 | 08504 | 07265 | 1 | | 0 | 0 | 0 | 0 | 0 | 0 | 0 | 0 | 0 | 0 | 0 | 0 | 0 | 0 | 0 | 0 | 0 | 0 | 0 | 0 | 0 | 0 | 1 | 0 | 0 | M98252 | 96.2 | 445 | 1 | 2200 | 2939 |
| 7267 | 08505 | 07266 | 1 | | 0 | 0 | 0 | 0 | 0 | 0 | 0 | 0 | 0 | 0 | 0 | 0 | 0 | 0 | 0 | 0 | 0 | 0 | 0 | 0 | 0 | 0 | 1 | 0 | 0 | | | | | | |
| 7268 | 08506 | 07267 | 1 | | 0 | 0 | 0 | 0 | 0 | 0 | 0 | 0 | 0 | 0 | 0 | 0 | 0 | 0 | 0 | 0 | 0 | 0 | 0 | 0 | 0 | 0 | 1 | 0 | 0 | | | | | | |
| 7269 | 08507 | 07268 | 1 | | 0 | 0 | 0 | 0 | 0 | 0 | 0 | 0 | 0 | 0 | 0 | 0 | 0 | 0 | 0 | 0 | 0 | 0 | 0 | 0 | 0 | 0 | 1 | 0 | 0 | | | | | | |
| 7270 | 08508 | 07269 | 1 | | 0 | 0 | 0 | 0 | 0 | 0 | 0 | 0 | 0 | 0 | 0 | 0 | 0 | 0 | 0 | 0 | 0 | 0 | 0 | 0 | 0 | 0 | 1 | 0 | 0 | | | | | | |
| 7271 | 08509 | 07270 | 1 | | 0 | 0 | 0 | 0 | 0 | 0 | 0 | 0 | 0 | 0 | 0 | 0 | 0 | 0 | 0 | 0 | 0 | 0 | 0 | 0 | 0 | 0 | 1 | 0 | 0 | | | | | | |
| 7272 | 08510 | 07271 | 1 | | 0 | 0 | 0 | 0 | 0 | 0 | 0 | 0 | 0 | 0 | 0 | 0 | 0 | 0 | 0 | 0 | 0 | 0 | 0 | 0 | 0 | 0 | 1 | 0 | 0 | | | | | | |
| 7273 | 08511 | 07272 | 1 | | 0 | 0 | 0 | 0 | 0 | 0 | 0 | 0 | 0 | 0 | 0 | 0 | 0 | 0 | 0 | 0 | 0 | 0 | 0 | 0 | 0 | 0 | 1 | 0 | 0 | | | | | | |
| 7274 | 08512 | 07273 | 2 | | 0 | 0 | 0 | 0 | 0 | 0 | 0 | 0 | 0 | 0 | 0 | 0 | 0 | 0 | 0 | 0 | 0 | 0 | 0 | 0 | 0 | 0 | 2 | 0 | 0 | | | | | | |
| 7275 | 08513 | 07274 | 4 | | 0 | 0 | 0 | 0 | 0 | 0 | 0 | 0 | 0 | 0 | 0 | 0 | 0 | 0 | 0 | 0 | 0 | 0 | 0 | 0 | 0 | 0 | 2 | 2 | 0 | | | | | | |
| 7276 | 08514 | 07275 | 1 | | 0 | 0 | 0 | 0 | 0 | 0 | 0 | 0 | 0 | 0 | 0 | 0 | 0 | 0 | 0 | 0 | 0 | 0 | 0 | 0 | 0 | 0 | 1 | 0 | 0 | | | | | | |
| 7277 | 08515 | 07276 | 1 | | 0 | 0 | 0 | 0 | 0 | 0 | 0 | 0 | 0 | 0 | 0 | 0 | 0 | 0 | 0 | 0 | 0 | 0 | 0 | 0 | 0 | 0 | 1 | 0 | 0 | | | | | | |
| 7278 | 08516 | 07277 | 1 | | 0 | 0 | 0 | 0 | 0 | 0 | 0 | 0 | 0 | 0 | 0 | 0 | 0 | 0 | 0 | 0 | 0 | 0 | 0 | 0 | 0 | 0 | 1 | 0 | 0 | | | | | | |
| 7279 | 08517 | 07278 | 1 | | 0 | 0 | 0 | 0 | 0 | 0 | 0 | 0 | 0 | 0 | 0 | 0 | 0 | 0 | 0 | 0 | 0 | 0 | 0 | 0 | 0 | 0 | 1 | 0 | 0 | | | | | | |
| 7280 | 08518 | 07279 | 1 | | 0 | 0 | 0 | 0 | 0 | 0 | 0 | 0 | 0 | 0 | 0 | 0 | 0 | 0 | 0 | 0 | 0 | 0 | 0 | 0 | 0 | 0 | 1 | 0 | 0 | | | | | | |
| 7281 | 08519 | 07280 | 1 | | 0 | 0 | 0 | 0 | 0 | 0 | 0 | 0 | 0 | 0 | 0 | 0 | 0 | 0 | 0 | 0 | 0 | 0 | 0 | 0 | 0 | 0 | 1 | 0 | 0 | | | | | | |
| 7282 | 08520 | 07281 | 1 | | 0 | 0 | 0 | 0 | 0 | 0 | 0 | 0 | 0 | 0 | 0 | 0 | 0 | 0 | 0 | 0 | 0 | 0 | 0 | 0 | 0 | 0 | 1 | 0 | 0 | | | | | | |
| 7283 | 08521 | 07282 | 1 | | 0 | 0 | 0 | 0 | 0 | 0 | 0 | 0 | 0 | 0 | 0 | 0 | 0 | 0 | 0 | 0 | 0 | 0 | 0 | 0 | 0 | 0 | 1 | 0 | 0 | | | | | | |
| 7284 | 08522 | 07283 | 1 | | 0 | 0 | 0 | 0 | 0 | 0 | 0 | 0 | 0 | 0 | 0 | 0 | 0 | 0 | 0 | 0 | 0 | 0 | 0 | 0 | 0 | 0 | 1 | 0 | 0 | | | | | | |
| 7285 | 08523 | 07284 | 1 | | 0 | 0 | 0 | 0 | 0 | 0 | 0 | 0 | 0 | 0 | 0 | 0 | 0 | 0 | 0 | 0 | 0 | 0 | 0 | 0 | 0 | 0 | 1 | 0 | 0 | | | | | | |
| 7286 | 08524 | 07285 | 1 | | 0 | 0 | 0 | 0 | 0 | 0 | 0 | 0 | 0 | 0 | 0 | 0 | 0 | 0 | 0 | 0 | 0 | 0 | 0 | 0 | 0 | 0 | 1 | 0 | 0 | J03143 | 94.6 | 112 | 1 | 1595 | 2064 |
| 7287 | 08525 | 07286 | 1 | | 0 | 0 | 0 | 0 | 0 | 0 | 0 | 0 | 0 | 0 | 0 | 0 | 0 | 0 | 0 | 0 | 0 | 0 | 0 | 0 | 0 | 0 | 1 | 0 | 0 | | | | | | |
| 7288 | 08526 | 07287 | 1 | | 0 | 0 | 0 | 0 | 0 | 0 | 0 | 0 | 0 | 0 | 0 | 0 | 0 | 0 | 0 | 0 | 0 | 0 | 0 | 0 | 0 | 0 | 1 | 0 | 0 | | | | | | |
| 7289 | 08527 | 07288 | 1 | | 0 | 0 | 0 | 0 | 0 | 0 | 0 | 0 | 0 | 0 | 0 | 0 | 0 | 0 | 0 | 0 | 0 | 0 | 0 | 0 | 0 | 0 | 1 | 0 | 0 | D13638 | 91.8 | 61 | 1 | 3809 | 5167 |
| 7290 | 08528 | 07289 | 1 | | 0 | 0 | 0 | 0 | 0 | 0 | 0 | 0 | 0 | 0 | 0 | 0 | 0 | 0 | 0 | 0 | 0 | 0 | 0 | 0 | 0 | 0 | 1 | 0 | 0 | | | | | | |
| 7291 | 08529 | 07290 | 1 | | 0 | 0 | 0 | 0 | 0 | 0 | 0 | 0 | 0 | 0 | 0 | 0 | 0 | 0 | 0 | 0 | 0 | 0 | 0 | 0 | 0 | 0 | 1 | 0 | 0 | | | | | | |
| 7292 | 08530 | 07291 | 1 | | 0 | 0 | 0 | 0 | 0 | 0 | 0 | 0 | 0 | 0 | 0 | 0 | 0 | 0 | 0 | 0 | 0 | 0 | 0 | 0 | 0 | 0 | 1 | 0 | 0 | | | | | | |
| 7293 | 08531 | 07292 | 1 | | 0 | 0 | 0 | 0 | 0 | 0 | 0 | 0 | 0 | 0 | 0 | 0 | 0 | 0 | 0 | 0 | 0 | 0 | 0 | 0 | 0 | 0 | 1 | 0 | 0 | | | | | | |

Table 203

| | A | B | C | E | G | I | K | M | O | Q | S | U | W | Y | AA | AC | AE | AG | AI | AK | AM | AO | AQ | AS | AU | AW | AY | BA | BC | BE | BF | BG | BH | BI | BK |
|---|---|---|---|---|---|---|---|---|---|---|---|---|---|---|---|---|---|---|---|---|---|---|---|---|---|---|---|---|---|---|---|---|---|---|---|
| 7294 | 08532 | 07293 | 1 | | 0 | 0 | 0 | 0 | 0 | 0 | 0 | 0 | 0 | 0 | 0 | 0 | 0 | 0 | 0 | 0 | 0 | 0 | 0 | 0 | 0 | 0 | 1 | 0 | 0 | | | | | | |
| 7295 | 08533 | 07294 | 1 | | 0 | 0 | 0 | 0 | 0 | 0 | 0 | 0 | 0 | 0 | 0 | 0 | 0 | 0 | 0 | 0 | 0 | 0 | 0 | 0 | 0 | 0 | 1 | 0 | 0 | | | | | | |
| 7296 | 08534 | 07295 | 1 | | 0 | 0 | 0 | 0 | 0 | 0 | 0 | 0 | 0 | 0 | 0 | 0 | 0 | 0 | 0 | 0 | 0 | 0 | 0 | 0 | 0 | 0 | 1 | 0 | 0 | | | | | | |
| 7297 | 08535 | 07296 | 1 | | 0 | 0 | 0 | 0 | 0 | 0 | 0 | 0 | 0 | 0 | 0 | 0 | 0 | 0 | 0 | 0 | 0 | 0 | 0 | 0 | 0 | 0 | 1 | 0 | 0 | | | | | | |
| 7298 | 08536 | 07297 | 1 | | 0 | 0 | 0 | 0 | 0 | 0 | 0 | 0 | 0 | 0 | 0 | 0 | 0 | 0 | 0 | 0 | 0 | 0 | 0 | 0 | 0 | 0 | 1 | 0 | 0 | | | | | | |
| 7299 | 08537 | 07298 | 1 | | 0 | 0 | 0 | 0 | 0 | 0 | 0 | 0 | 0 | 0 | 0 | 0 | 0 | 0 | 0 | 0 | 0 | 0 | 0 | 0 | 0 | 0 | 1 | 0 | 0 | | | | | | |
| 7300 | 08538 | 07299 | 1 | | 0 | 0 | 0 | 0 | 0 | 0 | 0 | 0 | 0 | 0 | 0 | 0 | 0 | 0 | 0 | 0 | 0 | 0 | 0 | 0 | 0 | 0 | 1 | 0 | 0 | | | | | | |
| 7301 | 08539 | 07300 | 1 | | 0 | 0 | 0 | 0 | 0 | 0 | 0 | 0 | 0 | 0 | 0 | 0 | 0 | 0 | 0 | 0 | 0 | 0 | 0 | 0 | 0 | 0 | 1 | 0 | 0 | | | | | | |
| 7302 | 08540 | 07301 | 1 | | 0 | 0 | 0 | 0 | 0 | 0 | 0 | 0 | 0 | 0 | 0 | 0 | 0 | 0 | 0 | 0 | 0 | 0 | 0 | 0 | 0 | 0 | 1 | 0 | 0 | | | | | | |
| 7303 | 08541 | 07302 | 1 | | 0 | 0 | 0 | 0 | 0 | 0 | 0 | 0 | 0 | 0 | 0 | 0 | 0 | 0 | 0 | 0 | 0 | 0 | 0 | 0 | 0 | 0 | 1 | 0 | 0 | | | | | | |
| 7304 | 08542 | 07303 | 1 | | 0 | 0 | 0 | 0 | 0 | 0 | 0 | 0 | 0 | 0 | 0 | 0 | 0 | 0 | 0 | 0 | 0 | 0 | 0 | 0 | 0 | 0 | 1 | 0 | 0 | | | | | | |
| 7305 | 08543 | 07304 | 1 | | 0 | 0 | 0 | 0 | 0 | 0 | 0 | 0 | 0 | 0 | 0 | 0 | 0 | 0 | 0 | 0 | 0 | 0 | 0 | 0 | 0 | 0 | 1 | 0 | 0 | | | | | | |
| 7306 | 08544 | 07305 | 1 | | 0 | 0 | 0 | 0 | 0 | 0 | 0 | 0 | 0 | 0 | 0 | 0 | 0 | 0 | 0 | 0 | 0 | 0 | 0 | 0 | 0 | 0 | 1 | 0 | 0 | | | | | | |
| 7307 | 08545 | 07306 | 1 | | 0 | 0 | 0 | 0 | 0 | 0 | 0 | 0 | 0 | 0 | 0 | 0 | 0 | 0 | 0 | 0 | 0 | 0 | 0 | 0 | 0 | 0 | 1 | 0 | 0 | | | | | | |
| 7308 | 08546 | 07307 | 1 | | 0 | 0 | 0 | 0 | 0 | 0 | 0 | 0 | 0 | 0 | 0 | 0 | 0 | 0 | 0 | 0 | 0 | 0 | 0 | 0 | 0 | 0 | 1 | 0 | 0 | L06633 | 95.2 | 310 | 1 | 1124 | 1724 |
| 7309 | 08547 | 07308 | 1 | | 0 | 0 | 0 | 0 | 0 | 0 | 0 | 0 | 0 | 0 | 0 | 0 | 0 | 0 | 0 | 0 | 0 | 0 | 0 | 0 | 0 | 0 | 1 | 0 | 0 | | | | | | |
| 7310 | 08548 | 07309 | 3 | | 0 | 0 | 0 | 0 | 0 | 0 | 0 | 0 | 0 | 0 | 0 | 0 | 0 | 0 | 0 | 0 | 0 | 0 | 0 | 0 | 0 | 0 | 3 | 0 | 0 | | | | | | |
| 7311 | 08549 | 07310 | 1 | | 0 | 0 | 0 | 0 | 0 | 0 | 0 | 0 | 0 | 0 | 0 | 0 | 0 | 0 | 0 | 0 | 0 | 0 | 0 | 0 | 0 | 0 | 1 | 0 | 0 | | | | | | |
| 7312 | 08550 | 07311 | 1 | | 0 | 0 | 0 | 0 | 0 | 0 | 0 | 0 | 0 | 0 | 0 | 0 | 0 | 0 | 0 | 0 | 0 | 0 | 0 | 0 | 0 | 0 | 1 | 0 | 0 | | | | | | |
| 7313 | 08551 | 07312 | 2 | | 0 | 0 | 0 | 0 | 0 | 0 | 0 | 0 | 0 | 0 | 0 | 0 | 0 | 0 | 0 | 0 | 0 | 0 | 0 | 0 | 0 | 0 | 2 | 0 | 0 | | | | | | |
| 7314 | 08552 | 07313 | 1 | | 0 | 0 | 0 | 0 | 0 | 0 | 0 | 0 | 0 | 0 | 0 | 0 | 0 | 0 | 0 | 0 | 0 | 0 | 0 | 0 | 0 | 0 | 1 | 0 | 0 | | | | | | |
| 7315 | 08553 | 07314 | 1 | | 0 | 0 | 0 | 0 | 0 | 0 | 0 | 0 | 0 | 0 | 0 | 0 | 0 | 0 | 0 | 0 | 0 | 0 | 0 | 0 | 0 | 0 | 1 | 0 | 0 | M90696 | 99.5 | 208 | 1 | 1524 | 1784 |
| 7316 | 08554 | 07315 | 2 | | 0 | 0 | 0 | 0 | 0 | 0 | 0 | 0 | 0 | 0 | 0 | 0 | 0 | 0 | 0 | 0 | 0 | 0 | 0 | 0 | 0 | 0 | 2 | 0 | 0 | | | | | | |
| 7317 | 08555 | 07316 | 1 | | 0 | 0 | 0 | 0 | 0 | 0 | 0 | 0 | 0 | 0 | 0 | 0 | 0 | 0 | 0 | 0 | 0 | 0 | 0 | 0 | 0 | 0 | 1 | 0 | 0 | | | | | | |
| 7318 | 08556 | 07317 | 1 | | 0 | 0 | 0 | 0 | 0 | 0 | 0 | 0 | 0 | 0 | 0 | 0 | 0 | 0 | 0 | 0 | 0 | 0 | 0 | 0 | 0 | 0 | 1 | 0 | 0 | | | | | | |
| 7319 | 08557 | 07318 | 1 | | 0 | 0 | 0 | 0 | 0 | 0 | 0 | 0 | 0 | 0 | 0 | 0 | 0 | 0 | 0 | 0 | 0 | 0 | 0 | 0 | 0 | 0 | 1 | 0 | 0 | | | | | | |
| 7320 | 08558 | 07319 | 1 | | 0 | 0 | 0 | 0 | 0 | 0 | 0 | 0 | 0 | 0 | 0 | 0 | 0 | 0 | 0 | 0 | 0 | 0 | 0 | 0 | 0 | 0 | 1 | 0 | 0 | | | | | | |
| 7321 | 08559 | 07320 | 1 | | 0 | 0 | 0 | 0 | 0 | 0 | 0 | 0 | 0 | 0 | 0 | 0 | 0 | 0 | 0 | 0 | 0 | 0 | 0 | 0 | 0 | 0 | 1 | 0 | 0 | | | | | | |
| 7322 | 08560 | 07321 | 2 | | 0 | 0 | 0 | 0 | 0 | 0 | 0 | 0 | 0 | 0 | 0 | 0 | 0 | 0 | 0 | 0 | 0 | 0 | 0 | 0 | 0 | 0 | 2 | 0 | 0 | | | | | | |
| 7323 | 08561 | 07322 | 1 | | 0 | 0 | 0 | 0 | 0 | 0 | 0 | 0 | 0 | 0 | 0 | 0 | 0 | 0 | 0 | 0 | 0 | 0 | 0 | 0 | 0 | 0 | 1 | 0 | 0 | | | | | | |
| 7324 | 08563 | 07323 | 1 | | 0 | 0 | 0 | 0 | 0 | 0 | 0 | 0 | 0 | 0 | 0 | 0 | 0 | 0 | 0 | 0 | 0 | 0 | 0 | 0 | 0 | 0 | 1 | 0 | 0 | J03619 | 98.3 | 357 | 1 | 1932 | 2295 |
| 7325 | 08564 | 07324 | 1 | | 0 | 0 | 0 | 0 | 0 | 0 | 0 | 0 | 0 | 0 | 0 | 0 | 0 | 0 | 0 | 0 | 0 | 0 | 0 | 0 | 0 | 0 | 1 | 0 | 0 | | | | | | |
| 7326 | 08565 | 07325 | 1 | | 0 | 0 | 0 | 0 | 0 | 0 | 0 | 0 | 0 | 0 | 0 | 0 | 0 | 0 | 0 | 0 | 0 | 0 | 0 | 0 | 0 | 0 | 1 | 0 | 0 | | | | | | |
| 7327 | 08566 | 07326 | 1 | | 0 | 0 | 0 | 0 | 0 | 0 | 0 | 0 | 0 | 0 | 0 | 0 | 0 | 0 | 0 | 0 | 0 | 0 | 0 | 0 | 0 | 0 | 1 | 0 | 0 | | | | | | |
| 7328 | 08567 | 07327 | 1 | | 0 | 0 | 0 | 0 | 0 | 0 | 0 | 0 | 0 | 0 | 0 | 0 | 0 | 0 | 0 | 0 | 0 | 0 | 0 | 0 | 0 | 0 | 1 | 0 | 0 | | | | | | |
| 7329 | 08568 | 07328 | 1 | | 0 | 0 | 0 | 0 | 0 | 0 | 0 | 0 | 0 | 0 | 0 | 0 | 0 | 0 | 0 | 0 | 0 | 0 | 0 | 0 | 0 | 0 | 1 | 0 | 0 | | | | | | |

Table 204

| | A | B | C | E | G | I | K | M | O | Q | S | U | W | Y | AA | AC | AE | AG | AI | AK | AM | AO | AQ | AS | AU | AW | AY | BA | BC | BE | BF | BG | BH | BI | BK |
|---|---|---|---|---|---|---|---|---|---|---|---|---|---|---|---|---|---|---|---|---|---|---|---|---|---|---|---|---|---|---|---|---|---|---|---|
| 7330 | 08569 | 07329 | 1 | | 0 | 0 | 0 | 0 | 0 | 0 | 0 | 0 | 0 | 0 | 0 | 0 | 0 | 0 | 0 | 0 | 0 | 0 | 0 | 0 | 0 | 0 | 1 | 0 | 0 | | | | | | |
| 7331 | 08570 | 07330 | 1 | | 0 | 0 | 0 | 0 | 0 | 0 | 0 | 0 | 0 | 0 | 0 | 0 | 0 | 0 | 0 | 0 | 0 | 0 | 0 | 0 | 0 | 0 | 1 | 0 | 0 | | | | | | |
| 7332 | 08571 | 07331 | 2 | | 0 | 0 | 0 | 0 | 0 | 0 | 0 | 0 | 0 | 0 | 0 | 0 | 0 | 0 | 0 | 0 | 0 | 0 | 0 | 0 | 0 | 0 | 2 | 0 | 0 | | | | | | |
| 7333 | 08572 | 07332 | 1 | | 0 | 0 | 0 | 0 | 0 | 0 | 0 | 0 | 0 | 0 | 0 | 0 | 0 | 0 | 0 | 0 | 0 | 0 | 0 | 0 | 0 | 0 | 1 | 0 | 0 | | | | | | |
| 7334 | 08573 | 07333 | 1 | | 0 | 0 | 0 | 0 | 0 | 0 | 0 | 0 | 0 | 0 | 0 | 0 | 0 | 0 | 0 | 0 | 0 | 0 | 0 | 0 | 0 | 0 | 1 | 0 | 0 | | | | | | |
| 7335 | 08574 | 07334 | 1 | | 0 | 0 | 0 | 0 | 0 | 0 | 0 | 0 | 0 | 0 | 0 | 0 | 0 | 0 | 0 | 0 | 0 | 0 | 0 | 0 | 0 | 0 | 1 | 0 | 0 | | | | | | |
| 7336 | 08575 | 07335 | 1 | | 0 | 0 | 0 | 0 | 0 | 0 | 0 | 0 | 0 | 0 | 0 | 0 | 0 | 0 | 0 | 0 | 0 | 0 | 0 | 0 | 0 | 0 | 1 | 0 | 0 | | | | | | |
| 7337 | 08576 | 07336 | 1 | | 0 | 0 | 0 | 0 | 0 | 0 | 0 | 0 | 0 | 0 | 0 | 0 | 0 | 0 | 0 | 0 | 0 | 0 | 0 | 0 | 0 | 0 | 1 | 0 | 0 | | | | | | |
| 7338 | 08577 | 07337 | 1 | | 0 | 0 | 0 | 0 | 0 | 0 | 0 | 0 | 0 | 0 | 0 | 0 | 0 | 0 | 0 | 0 | 0 | 0 | 0 | 0 | 0 | 0 | 1 | 0 | 0 | | | | | | |
| 7339 | 08578 | 07338 | 1 | | 0 | 0 | 0 | 0 | 0 | 0 | 0 | 0 | 0 | 0 | 0 | 0 | 0 | 0 | 0 | 0 | 0 | 0 | 0 | 0 | 0 | 0 | 1 | 0 | 0 | | | | | | |
| 7340 | 08579 | 07339 | 1 | | 0 | 0 | 0 | 0 | 0 | 0 | 0 | 0 | 0 | 0 | 0 | 0 | 0 | 0 | 0 | 0 | 0 | 0 | 0 | 0 | 0 | 0 | 1 | 0 | 0 | | | | | | |
| 7341 | 08580 | 07340 | 1 | | 0 | 0 | 0 | 0 | 0 | 0 | 0 | 0 | 0 | 0 | 0 | 0 | 0 | 0 | 0 | 0 | 0 | 0 | 0 | 0 | 0 | 0 | 1 | 0 | 0 | | | | | | |
| 7342 | 08581 | 07341 | 1 | | 0 | 0 | 0 | 0 | 0 | 0 | 0 | 0 | 0 | 0 | 0 | 0 | 0 | 0 | 0 | 0 | 0 | 0 | 0 | 0 | 0 | 0 | 1 | 0 | 0 | | | | | | |
| 7343 | 08582 | 07342 | 1 | | 0 | 0 | 0 | 0 | 0 | 0 | 0 | 0 | 0 | 0 | 0 | 0 | 0 | 0 | 0 | 0 | 0 | 0 | 0 | 0 | 0 | 0 | 1 | 0 | 0 | | | | | | |
| 7344 | 08583 | 07343 | 1 | | 0 | 0 | 0 | 0 | 0 | 0 | 0 | 0 | 0 | 0 | 0 | 0 | 0 | 0 | 0 | 0 | 0 | 0 | 0 | 0 | 0 | 0 | 1 | 0 | 0 | | | | | | |
| 7345 | 08584 | 07344 | 1 | | 0 | 0 | 0 | 0 | 0 | 0 | 0 | 0 | 0 | 0 | 0 | 0 | 0 | 0 | 0 | 0 | 0 | 0 | 0 | 0 | 0 | 0 | 1 | 0 | 0 | | | | | | |
| 7346 | 08585 | 07345 | 1 | | 0 | 0 | 0 | 0 | 0 | 0 | 0 | 0 | 0 | 0 | 0 | 0 | 0 | 0 | 0 | 0 | 0 | 0 | 0 | 0 | 0 | 0 | 1 | 0 | 0 | | | | | | |
| 7347 | 08586 | 07346 | 1 | | 0 | 0 | 0 | 0 | 0 | 0 | 0 | 0 | 0 | 0 | 0 | 0 | 0 | 0 | 0 | 0 | 0 | 0 | 0 | 0 | 0 | 0 | 1 | 0 | 0 | | | | | | |
| 7348 | 08587 | 07347 | 1 | | 0 | 0 | 0 | 0 | 0 | 0 | 0 | 0 | 0 | 0 | 0 | 0 | 0 | 0 | 0 | 0 | 0 | 0 | 0 | 0 | 0 | 0 | 1 | 0 | 0 | | | | | | |
| 7349 | 08588 | 07348 | 1 | | 0 | 0 | 0 | 0 | 0 | 0 | 0 | 0 | 0 | 0 | 0 | 0 | 0 | 0 | 0 | 0 | 0 | 0 | 0 | 0 | 0 | 0 | 1 | 0 | 0 | | | | | | |
| 7350 | 08589 | 07349 | 1 | | 0 | 0 | 0 | 0 | 0 | 0 | 0 | 0 | 0 | 0 | 0 | 0 | 0 | 0 | 0 | 0 | 0 | 0 | 0 | 0 | 0 | 0 | 1 | 0 | 0 | | | | | | |
| 7351 | 08591 | 07350 | 1 | | 0 | 0 | 0 | 0 | 0 | 0 | 0 | 0 | 0 | 0 | 0 | 0 | 0 | 0 | 0 | 0 | 0 | 0 | 0 | 0 | 0 | 0 | 1 | 0 | 0 | | | | | | |
| 7352 | 08592 | 07351 | 1 | | 0 | 0 | 0 | 0 | 0 | 0 | 0 | 0 | 0 | 0 | 0 | 0 | 0 | 0 | 0 | 0 | 0 | 0 | 0 | 0 | 0 | 0 | 1 | 0 | 0 | J03779 | 96.3 | 375 | 1 | 4622 | 5508 |
| 7353 | 08593 | 07352 | 1 | | 0 | 0 | 0 | 0 | 0 | 0 | 0 | 0 | 0 | 0 | 0 | 0 | 0 | 0 | 0 | 0 | 0 | 0 | 0 | 0 | 0 | 0 | 1 | 0 | 0 | | | | | | |
| 7354 | 08594 | 07353 | 2 | | 0 | 0 | 0 | 0 | 0 | 0 | 0 | 0 | 0 | 0 | 0 | 0 | 0 | 0 | 0 | 0 | 0 | 0 | 0 | 0 | 0 | 0 | 2 | 0 | 0 | | | | | | |
| 7355 | 08595 | 07354 | 2 | | 0 | 0 | 0 | 0 | 0 | 0 | 0 | 0 | 0 | 0 | 0 | 0 | 0 | 0 | 0 | 0 | 0 | 0 | 0 | 0 | 0 | 0 | 2 | 0 | 0 | | | | | | |
| 7356 | 08596 | 07355 | 1 | | 0 | 0 | 0 | 0 | 0 | 0 | 0 | 0 | 0 | 0 | 0 | 0 | 0 | 0 | 0 | 0 | 0 | 0 | 0 | 0 | 0 | 0 | 1 | 0 | 0 | | | | | | |
| 7357 | 08597 | 07356 | 1 | | 0 | 0 | 0 | 0 | 0 | 0 | 0 | 0 | 0 | 0 | 0 | 0 | 0 | 0 | 0 | 0 | 0 | 0 | 0 | 0 | 0 | 0 | 1 | 0 | 0 | | | | | | |
| 7358 | 08598 | 07357 | 1 | | 0 | 0 | 0 | 0 | 0 | 0 | 0 | 0 | 0 | 0 | 0 | 0 | 0 | 0 | 0 | 0 | 0 | 0 | 0 | 0 | 0 | 0 | 1 | 0 | 0 | | | | | | |
| 7359 | 08599 | 07358 | 1 | | 0 | 0 | 0 | 0 | 0 | 0 | 0 | 0 | 0 | 0 | 0 | 0 | 0 | 0 | 0 | 0 | 0 | 0 | 0 | 0 | 0 | 0 | 1 | 0 | 0 | | | | | | |
| 7360 | 08600 | 07359 | 1 | | 0 | 0 | 0 | 0 | 0 | 0 | 0 | 0 | 0 | 0 | 0 | 0 | 0 | 0 | 0 | 0 | 0 | 0 | 0 | 0 | 0 | 0 | 1 | 0 | 0 | X58957 | 95.7 | 161 | 225 | 1755 | 2560 |
| 7361 | 08601 | 07360 | 1 | | 0 | 0 | 0 | 0 | 0 | 0 | 0 | 0 | 0 | 0 | 0 | 0 | 0 | 0 | 0 | 0 | 0 | 0 | 0 | 0 | 0 | 0 | 1 | 0 | 0 | | | | | | |
| 7362 | 08602 | 07361 | 2 | | 0 | 0 | 0 | 0 | 0 | 0 | 0 | 0 | 0 | 0 | 0 | 0 | 0 | 0 | 0 | 0 | 0 | 0 | 0 | 0 | 0 | 0 | 2 | 0 | 0 | | | | | | |
| 7363 | 08603 | 07362 | 1 | | 0 | 0 | 0 | 0 | 0 | 0 | 0 | 0 | 0 | 0 | 0 | 0 | 0 | 0 | 0 | 0 | 0 | 0 | 0 | 0 | 0 | 0 | 1 | 0 | 0 | | | | | | |
| 7364 | 08604 | 07363 | 1 | | 0 | 0 | 0 | 0 | 0 | 0 | 0 | 0 | 0 | 0 | 0 | 0 | 0 | 0 | 0 | 0 | 0 | 0 | 0 | 0 | 0 | 0 | 1 | 0 | 0 | | | | | | |
| 7365 | 08605 | 07364 | 1 | | 0 | 0 | 0 | 0 | 0 | 0 | 0 | 0 | 0 | 0 | 0 | 0 | 0 | 0 | 0 | 0 | 0 | 0 | 0 | 0 | 0 | 0 | 1 | 0 | 0 | | | | | | |

Table 205

| | A | B | C | E | G | I | K | M | O | Q | S | U | W | Y | AA | AC | AE | AG | AI | AK | AM | AO | AQ | AS | AU | AW | AY | BA | BC | BE | BF | BG | BH | BI | BK |
|---|---|---|---|---|---|---|---|---|---|---|---|---|---|---|---|---|---|---|---|---|---|---|---|---|---|---|---|---|---|---|---|---|---|---|---|
| 7366 | 08606 | 07365 | 1 | | 0 | 0 | 0 | 0 | 0 | 0 | 0 | 0 | 0 | 0 | 0 | 0 | 0 | 0 | 0 | 0 | 0 | 0 | 0 | 0 | 0 | 0 | 1 | 0 | 0 | | | | | | |
| 7367 | 08607 | 07366 | 1 | | 0 | 0 | 0 | 0 | 0 | 0 | 0 | 0 | 0 | 0 | 0 | 0 | 0 | 0 | 0 | 0 | 0 | 0 | 0 | 0 | 0 | 0 | 1 | 0 | 0 | | | | | | |
| 7368 | 08608 | 07367 | 1 | | 0 | 0 | 0 | 0 | 0 | 0 | 0 | 0 | 0 | 0 | 0 | 0 | 0 | 0 | 0 | 0 | 0 | 0 | 0 | 0 | 0 | 0 | 1 | 0 | 0 | | | | | | |
| 7369 | 08609 | 07368 | 1 | | 0 | 0 | 0 | 0 | 0 | 0 | 0 | 0 | 0 | 0 | 0 | 0 | 0 | 0 | 0 | 0 | 0 | 0 | 0 | 0 | 0 | 0 | 1 | 0 | 0 | | | | | | |
| 7370 | 08610 | 07369 | 1 | | 0 | 0 | 0 | 0 | 0 | 0 | 0 | 0 | 0 | 0 | 0 | 0 | 0 | 0 | 0 | 0 | 0 | 0 | 0 | 0 | 0 | 0 | 1 | 0 | 0 | | | | | | |
| 7371 | 08611 | 07370 | 1 | | 0 | 0 | 0 | 0 | 0 | 0 | 0 | 0 | 0 | 0 | 0 | 0 | 0 | 0 | 0 | 0 | 0 | 0 | 0 | 0 | 0 | 0 | 1 | 0 | 0 | | | | | | |
| 7372 | 08612 | 07371 | 1 | | 0 | 0 | 0 | 0 | 0 | 0 | 0 | 0 | 0 | 0 | 0 | 0 | 0 | 0 | 0 | 0 | 0 | 0 | 0 | 0 | 0 | 0 | 1 | 0 | 0 | | | | | | |
| 7373 | 08613 | 07372 | 1 | | 0 | 0 | 0 | 0 | 0 | 0 | 0 | 0 | 0 | 0 | 0 | 0 | 0 | 0 | 0 | 0 | 0 | 0 | 0 | 0 | 0 | 0 | 1 | 0 | 0 | | | | | | |
| 7374 | 08614 | 07373 | 1 | | 0 | 0 | 0 | 0 | 0 | 0 | 0 | 0 | 0 | 0 | 0 | 0 | 0 | 0 | 0 | 0 | 0 | 0 | 0 | 0 | 0 | 0 | 1 | 0 | 0 | | | | | | |
| 7375 | 08615 | 07374 | 1 | | 0 | 0 | 0 | 0 | 0 | 0 | 0 | 0 | 0 | 0 | 0 | 0 | 0 | 0 | 0 | 0 | 0 | 0 | 0 | 0 | 0 | 0 | 1 | 0 | 0 | | | | | | |
| 7376 | 08616 | 07375 | 1 | | 0 | 0 | 0 | 0 | 0 | 0 | 0 | 0 | 0 | 0 | 0 | 0 | 0 | 0 | 0 | 0 | 0 | 0 | 0 | 0 | 0 | 0 | 1 | 0 | 0 | | | | | | |
| 7377 | 08617 | 07376 | 1 | | 0 | 0 | 0 | 0 | 0 | 0 | 0 | 0 | 0 | 0 | 0 | 0 | 0 | 0 | 0 | 0 | 0 | 0 | 0 | 0 | 0 | 0 | 1 | 0 | 0 | | | | | | |
| 7378 | 08618 | 07377 | 1 | | 0 | 0 | 0 | 0 | 0 | 0 | 0 | 0 | 0 | 0 | 0 | 0 | 0 | 0 | 0 | 0 | 0 | 0 | 0 | 0 | 0 | 0 | 1 | 0 | 0 | | | | | | |
| 7379 | 08619 | 07378 | 1 | | 0 | 0 | 0 | 0 | 0 | 0 | 0 | 0 | 0 | 0 | 0 | 0 | 0 | 0 | 0 | 0 | 0 | 0 | 0 | 0 | 0 | 0 | 1 | 0 | 0 | | | | | | |
| 7380 | 08620 | 07379 | 1 | | 0 | 0 | 0 | 0 | 0 | 0 | 0 | 0 | 0 | 0 | 0 | 0 | 0 | 0 | 0 | 0 | 0 | 0 | 0 | 0 | 0 | 0 | 1 | 0 | 0 | | | | | | |
| 7381 | 08621 | 07380 | 1 | | 0 | 0 | 0 | 0 | 0 | 0 | 0 | 0 | 0 | 0 | 0 | 0 | 0 | 0 | 0 | 0 | 0 | 0 | 0 | 0 | 0 | 0 | 1 | 0 | 0 | M29064 | 94 | 402 | 1 | 838 | 1760 |
| 7382 | 08622 | 07381 | 1 | | 0 | 0 | 0 | 0 | 0 | 0 | 0 | 0 | 0 | 0 | 0 | 0 | 0 | 0 | 0 | 0 | 0 | 0 | 0 | 0 | 0 | 0 | 1 | 0 | 0 | | | | | | |
| 7383 | 08623 | 07382 | 1 | | 0 | 0 | 0 | 0 | 0 | 0 | 0 | 0 | 0 | 0 | 0 | 0 | 0 | 0 | 0 | 0 | 0 | 0 | 0 | 0 | 0 | 0 | 1 | 0 | 0 | | | | | | |
| 7384 | 08624 | 07383 | 1 | | 0 | 0 | 0 | 0 | 0 | 0 | 0 | 0 | 0 | 0 | 0 | 0 | 0 | 0 | 0 | 0 | 0 | 0 | 0 | 0 | 0 | 0 | 1 | 0 | 0 | | | | | | |
| 7385 | 08625 | 07384 | 1 | | 0 | 0 | 0 | 0 | 0 | 0 | 0 | 0 | 0 | 0 | 0 | 0 | 0 | 0 | 0 | 0 | 0 | 0 | 0 | 0 | 0 | 0 | 1 | 0 | 0 | | | | | | |
| 7386 | 08626 | 07385 | 1 | | 0 | 0 | 0 | 0 | 0 | 0 | 0 | 0 | 0 | 0 | 0 | 0 | 0 | 0 | 0 | 0 | 0 | 0 | 0 | 0 | 0 | 0 | 1 | 0 | 0 | | | | | | |
| 7387 | 08627 | 07386 | 1 | | 0 | 0 | 0 | 0 | 0 | 0 | 0 | 0 | 0 | 0 | 0 | 0 | 0 | 0 | 0 | 0 | 0 | 0 | 0 | 0 | 0 | 0 | 1 | 0 | 0 | | | | | | |
| 7388 | 08628 | 07387 | 1 | | 0 | 0 | 0 | 0 | 0 | 0 | 0 | 0 | 0 | 0 | 0 | 0 | 0 | 0 | 0 | 0 | 0 | 0 | 0 | 0 | 0 | 0 | 1 | 0 | 0 | | | | | | |
| 7389 | 08629 | 07388 | 1 | | 0 | 0 | 0 | 0 | 0 | 0 | 0 | 0 | 0 | 0 | 0 | 0 | 0 | 0 | 0 | 0 | 0 | 0 | 0 | 0 | 0 | 0 | 1 | 0 | 0 | | | | | | |
| 7390 | 08630 | 07389 | 1 | | 0 | 0 | 0 | 0 | 0 | 0 | 0 | 0 | 0 | 0 | 0 | 0 | 0 | 0 | 0 | 0 | 0 | 0 | 0 | 0 | 0 | 0 | 1 | 0 | 0 | | | | | | |
| 7391 | 08631 | 07390 | 1 | | 0 | 0 | 0 | 0 | 0 | 0 | 0 | 0 | 0 | 0 | 0 | 0 | 0 | 0 | 0 | 0 | 0 | 0 | 0 | 0 | 0 | 0 | 1 | 0 | 0 | | | | | | |
| 7392 | 08632 | 07391 | 1 | | 0 | 0 | 0 | 0 | 0 | 0 | 0 | 0 | 0 | 0 | 0 | 0 | 0 | 0 | 0 | 0 | 0 | 0 | 0 | 0 | 0 | 0 | 1 | 0 | 0 | | | | | | |
| 7393 | 08633 | 07392 | 1 | | 0 | 0 | 0 | 0 | 0 | 0 | 0 | 0 | 0 | 0 | 0 | 0 | 0 | 0 | 0 | 0 | 0 | 0 | 0 | 0 | 0 | 0 | 1 | 0 | 0 | | | | | | |
| 7394 | 08634 | 07393 | 1 | | 0 | 0 | 0 | 0 | 0 | 0 | 0 | 0 | 0 | 0 | 0 | 0 | 0 | 0 | 0 | 0 | 0 | 0 | 0 | 0 | 0 | 0 | 1 | 0 | 0 | | | | | | |
| 7395 | 08635 | 07394 | 1 | | 0 | 0 | 0 | 0 | 0 | 0 | 0 | 0 | 0 | 0 | 0 | 0 | 0 | 0 | 0 | 0 | 0 | 0 | 0 | 0 | 0 | 0 | 1 | 0 | 0 | | | | | | |
| 7396 | 08636 | 07395 | 1 | | 0 | 0 | 0 | 0 | 0 | 0 | 0 | 0 | 0 | 0 | 0 | 0 | 0 | 0 | 0 | 0 | 0 | 0 | 0 | 0 | 0 | 0 | 1 | 0 | 0 | | | | | | |
| 7397 | 08637 | 07396 | 1 | | 0 | 0 | 0 | 0 | 0 | 0 | 0 | 0 | 0 | 0 | 0 | 0 | 0 | 0 | 0 | 0 | 0 | 0 | 0 | 0 | 0 | 0 | 1 | 0 | 0 | | | | | | |
| 7398 | 08638 | 07397 | 2 | | 0 | 0 | 0 | 0 | 0 | 0 | 0 | 0 | 0 | 0 | 0 | 0 | 0 | 0 | 0 | 0 | 0 | 0 | 0 | 0 | 0 | 0 | 2 | 0 | 0 | M60627 | 95.1 | 286 | 1 | 997 | 1281 |
| 7399 | 08639 | 07398 | 1 | | 0 | 0 | 0 | 0 | 0 | 0 | 0 | 0 | 0 | 0 | 0 | 0 | 0 | 0 | 0 | 0 | 0 | 0 | 0 | 0 | 0 | 0 | 1 | 0 | 0 | | | | | | |
| 7400 | 08640 | 07399 | 1 | | 0 | 0 | 0 | 0 | 0 | 0 | 0 | 0 | 0 | 0 | 0 | 0 | 0 | 0 | 0 | 0 | 0 | 0 | 0 | 0 | 0 | 0 | 1 | 0 | 0 | | | | | | |
| 7401 | 08641 | 07400 | 1 | | 0 | 0 | 0 | 0 | 0 | 0 | 0 | 0 | 0 | 0 | 0 | 0 | 0 | 0 | 0 | 0 | 0 | 0 | 0 | 0 | 0 | 0 | 1 | 0 | 0 | | | | | | |

Table 206

| | A | B | C | E | G | I | K | M | O | Q | S | U | W | Y | AA | AC | AE | AG | AI | AK | AM | AO | AQ | AS | AU | AW | AY | BA | BC | BE | BF | BG | BH | BI | BK |
|---|---|---|---|---|---|---|---|---|---|---|---|---|---|---|---|---|---|---|---|---|---|---|---|---|---|---|---|---|---|---|---|---|---|---|---|
| 7402 | 08642 | 07401 | 1 | | 0 | 0 | 0 | 0 | 0 | 0 | 0 | 0 | 0 | 0 | 0 | 0 | 0 | 0 | 0 | 0 | 0 | 0 | 0 | 0 | 0 | 0 | 1 | 0 | 0 | | | | | | |
| 7403 | 08643 | 07402 | 1 | | 0 | 0 | 0 | 0 | 0 | 0 | 0 | 0 | 0 | 0 | 0 | 0 | 0 | 0 | 0 | 0 | 0 | 0 | 0 | 0 | 0 | 0 | 1 | 0 | 0 | D16626 | 99.7 | 321 | 1 | 2765 | 3085 |
| 7404 | 08644 | 07403 | 1 | | 0 | 0 | 0 | 0 | 0 | 0 | 0 | 0 | 0 | 0 | 0 | 0 | 0 | 0 | 0 | 0 | 0 | 0 | 0 | 0 | 0 | 0 | 1 | 0 | 0 | | | | | | |
| 7405 | 08645 | 07404 | 1 | | 0 | 0 | 0 | 0 | 0 | 0 | 0 | 0 | 0 | 0 | 0 | 0 | 0 | 0 | 0 | 0 | 0 | 0 | 0 | 0 | 0 | 0 | 1 | 0 | 0 | | | | | | |
| 7406 | 08646 | 07405 | 1 | | 0 | 0 | 0 | 0 | 0 | 0 | 0 | 0 | 0 | 0 | 0 | 0 | 0 | 0 | 0 | 0 | 0 | 0 | 0 | 0 | 0 | 0 | 1 | 0 | 0 | | | | | | |
| 7407 | 08647 | 07406 | 1 | | 0 | 0 | 0 | 0 | 0 | 0 | 0 | 0 | 0 | 0 | 0 | 0 | 0 | 0 | 0 | 0 | 0 | 0 | 0 | 0 | 0 | 0 | 1 | 0 | 0 | | | | | | |
| 7408 | 08648 | 07407 | 1 | | 0 | 0 | 0 | 0 | 0 | 0 | 0 | 0 | 0 | 0 | 0 | 0 | 0 | 0 | 0 | 0 | 0 | 0 | 0 | 0 | 0 | 0 | 1 | 0 | 0 | | | | | | |
| 7409 | 08649 | 07408 | 1 | | 0 | 0 | 0 | 0 | 0 | 0 | 0 | 0 | 0 | 0 | 0 | 0 | 0 | 0 | 0 | 0 | 0 | 0 | 0 | 0 | 0 | 0 | 1 | 0 | 0 | | | | | | |
| 7410 | 08650 | 07409 | 1 | | 0 | 0 | 0 | 0 | 0 | 0 | 0 | 0 | 0 | 0 | 0 | 0 | 0 | 0 | 0 | 0 | 0 | 0 | 0 | 0 | 0 | 0 | 1 | 0 | 0 | | | | | | |
| 7411 | 08651 | 07410 | 1 | | 0 | 0 | 0 | 0 | 0 | 0 | 0 | 0 | 0 | 0 | 0 | 0 | 0 | 0 | 0 | 0 | 0 | 0 | 0 | 0 | 0 | 0 | 1 | 0 | 0 | | | | | | |
| 7412 | 08652 | 07411 | 1 | | 0 | 0 | 0 | 0 | 0 | 0 | 0 | 0 | 0 | 0 | 0 | 0 | 0 | 0 | 0 | 0 | 0 | 0 | 0 | 0 | 0 | 0 | 1 | 0 | 0 | | | | | | |
| 7413 | 08653 | 07412 | 1 | | 0 | 0 | 0 | 0 | 0 | 0 | 0 | 0 | 0 | 0 | 0 | 0 | 0 | 0 | 0 | 0 | 0 | 0 | 0 | 0 | 0 | 0 | 1 | 0 | 0 | | | | | | |
| 7414 | 08654 | 07413 | 1 | | 0 | 0 | 0 | 0 | 0 | 0 | 0 | 0 | 0 | 0 | 0 | 0 | 0 | 0 | 0 | 0 | 0 | 0 | 0 | 0 | 0 | 0 | 1 | 0 | 0 | | | | | | |
| 7415 | 08655 | 07414 | 1 | | 0 | 0 | 0 | 0 | 0 | 0 | 0 | 0 | 0 | 0 | 0 | 0 | 0 | 0 | 0 | 0 | 0 | 0 | 0 | 0 | 0 | 0 | 1 | 0 | 0 | | | | | | |
| 7416 | 08656 | 07415 | 1 | | 0 | 0 | 0 | 0 | 0 | 0 | 0 | 0 | 0 | 0 | 0 | 0 | 0 | 0 | 0 | 0 | 0 | 0 | 0 | 0 | 0 | 0 | 1 | 0 | 0 | | | | | | |
| 7417 | 08657 | 07416 | 1 | | 0 | 0 | 0 | 0 | 0 | 0 | 0 | 0 | 0 | 0 | 0 | 0 | 0 | 0 | 0 | 0 | 0 | 0 | 0 | 0 | 0 | 0 | 1 | 0 | 0 | | | | | | |
| 7418 | 08658 | 07417 | 1 | | 0 | 0 | 0 | 0 | 0 | 0 | 0 | 0 | 0 | 0 | 0 | 0 | 0 | 0 | 0 | 0 | 0 | 0 | 0 | 0 | 0 | 0 | 1 | 0 | 0 | | | | | | |
| 7419 | 08659 | 07418 | 1 | | 0 | 0 | 0 | 0 | 0 | 0 | 0 | 0 | 0 | 0 | 0 | 0 | 0 | 0 | 0 | 0 | 0 | 0 | 0 | 0 | 0 | 0 | 1 | 0 | 0 | | | | | | |
| 7420 | 08660 | 07419 | 1 | | 0 | 0 | 0 | 0 | 0 | 0 | 0 | 0 | 0 | 0 | 0 | 0 | 0 | 0 | 0 | 0 | 0 | 0 | 0 | 0 | 0 | 0 | 1 | 0 | 0 | | | | | | |
| 7421 | 08661 | 07420 | 1 | | 0 | 0 | 0 | 0 | 0 | 0 | 0 | 0 | 0 | 0 | 0 | 0 | 0 | 0 | 0 | 0 | 0 | 0 | 0 | 0 | 0 | 0 | 1 | 0 | 0 | | | | | | |
| 7422 | 08662 | 07421 | 1 | | 0 | 0 | 0 | 0 | 0 | 0 | 0 | 0 | 0 | 0 | 0 | 0 | 0 | 0 | 0 | 0 | 0 | 0 | 0 | 0 | 0 | 0 | 1 | 0 | 0 | | | | | | |
| 7423 | 08663 | 07422 | 1 | | 0 | 0 | 0 | 0 | 0 | 0 | 0 | 0 | 0 | 0 | 0 | 0 | 0 | 0 | 0 | 0 | 0 | 0 | 0 | 0 | 0 | 0 | 1 | 0 | 0 | X52151 | 99.3 | 134 | 1 | 1868 | 2022 |
| 7424 | 08664 | 07423 | 2 | | 0 | 0 | 0 | 0 | 0 | 0 | 0 | 0 | 0 | 0 | 0 | 0 | 0 | 0 | 0 | 0 | 0 | 0 | 0 | 0 | 0 | 0 | 2 | 0 | 0 | | | | | | |
| 7425 | 08665 | 07424 | 1 | | 0 | 0 | 0 | 0 | 0 | 0 | 0 | 0 | 0 | 0 | 0 | 0 | 0 | 0 | 0 | 0 | 0 | 0 | 0 | 0 | 0 | 0 | 1 | 0 | 0 | | | | | | |
| 7426 | 08666 | 07425 | 1 | | 0 | 0 | 0 | 0 | 0 | 0 | 0 | 0 | 0 | 0 | 0 | 0 | 0 | 0 | 0 | 0 | 0 | 0 | 0 | 0 | 0 | 0 | 1 | 0 | 0 | | | | | | |
| 7427 | 08667 | 07426 | 1 | | 0 | 0 | 0 | 0 | 0 | 0 | 0 | 0 | 0 | 0 | 0 | 0 | 0 | 0 | 0 | 0 | 0 | 0 | 0 | 0 | 0 | 0 | 1 | 0 | 0 | X59834 | 97.6 | 248 | 1 | 1124 | 2727 |
| 7428 | 08668 | 07427 | 1 | | 0 | 0 | 0 | 0 | 0 | 0 | 0 | 0 | 0 | 0 | 0 | 0 | 0 | 0 | 0 | 0 | 0 | 0 | 0 | 0 | 0 | 0 | 1 | 0 | 0 | | | | | | |
| 7429 | 08669 | 07428 | 1 | | 0 | 0 | 0 | 0 | 0 | 0 | 0 | 0 | 0 | 0 | 0 | 0 | 0 | 0 | 0 | 0 | 0 | 0 | 0 | 0 | 0 | 0 | 1 | 0 | 0 | | | | | | |
| 7430 | 08670 | 07429 | 1 | | 0 | 0 | 0 | 0 | 0 | 0 | 0 | 0 | 0 | 0 | 0 | 0 | 0 | 0 | 0 | 0 | 0 | 0 | 0 | 0 | 0 | 0 | 1 | 0 | 0 | | | | | | |
| 7431 | 08671 | 07430 | 1 | | 0 | 0 | 0 | 0 | 0 | 0 | 0 | 0 | 0 | 0 | 0 | 0 | 0 | 0 | 0 | 0 | 0 | 0 | 0 | 0 | 0 | 0 | 1 | 0 | 0 | | | | | | |
| 7432 | 08673 | 07431 | 1 | | 0 | 0 | 0 | 0 | 0 | 0 | 0 | 0 | 0 | 0 | 0 | 0 | 0 | 0 | 0 | 0 | 0 | 0 | 0 | 0 | 0 | 0 | 1 | 0 | 0 | | | | | | |
| 7433 | 08674 | 07432 | 2 | | 0 | 0 | 0 | 0 | 0 | 0 | 0 | 0 | 0 | 0 | 0 | 0 | 0 | 0 | 0 | 0 | 0 | 0 | 0 | 0 | 0 | 0 | 1 | 0 | 1 | M93056 | 94.3 | 353 | 1 | 955 | 1316 |
| 7434 | 08675 | 07433 | 1 | | 0 | 0 | 0 | 0 | 0 | 0 | 0 | 0 | 0 | 0 | 0 | 0 | 0 | 0 | 0 | 0 | 0 | 0 | 0 | 0 | 0 | 0 | 1 | 0 | 0 | | | | | | |
| 7435 | 08676 | 07434 | 1 | | 0 | 0 | 0 | 0 | 0 | 0 | 0 | 0 | 0 | 0 | 0 | 0 | 0 | 0 | 0 | 0 | 0 | 0 | 0 | 0 | 0 | 0 | 1 | 0 | 0 | | | | | | |
| 7436 | 08677 | 07435 | 1 | | 0 | 0 | 0 | 0 | 0 | 0 | 0 | 0 | 0 | 0 | 0 | 0 | 0 | 0 | 0 | 0 | 0 | 0 | 0 | 0 | 0 | 0 | 1 | 0 | 0 | | | | | | |
| 7437 | 08678 | 07436 | 1 | | 0 | 0 | 0 | 0 | 0 | 0 | 0 | 0 | 0 | 0 | 0 | 0 | 0 | 0 | 0 | 0 | 0 | 0 | 0 | 0 | 0 | 0 | 1 | 0 | 0 | | | | | | |

Table 207

| | A | B | C | E | G | I | K | M | O | Q | S | U | W | Y | AA | AC | AE | AG | AI | AK | AM | AO | AQ | AS | AU | AW | AY | BA | BC | BE | BF | BG | BH | BI | BK |
|---|---|---|---|---|---|---|---|---|---|---|---|---|---|---|---|---|---|---|---|---|---|---|---|---|---|---|---|---|---|---|---|---|---|---|---|
| 7438 | 08679 | 07437 | 2 | | 0 | 0 | 0 | 0 | 0 | 0 | 0 | 0 | 0 | 0 | 0 | 0 | 0 | 0 | 0 | 0 | 0 | 0 | 0 | 0 | 0 | 0 | 1 | 1 | 0 | L02426 | 98.6 | 360 | 1 | 1209 | 1599 |
| 7439 | 08680 | 07438 | 1 | | 0 | 0 | 0 | 0 | 0 | 0 | 0 | 0 | 0 | 0 | 0 | 0 | 0 | 0 | 0 | 0 | 0 | 0 | 0 | 0 | 0 | 0 | 1 | 0 | 0 | J03571 | 94.7 | 266 | 1 | 2220 | 2484 |
| 7440 | 08681 | 07439 | 1 | | 0 | 0 | 0 | 0 | 0 | 0 | 0 | 0 | 0 | 0 | 0 | 0 | 0 | 0 | 0 | 0 | 0 | 0 | 0 | 0 | 0 | 0 | 1 | 0 | 0 | | | | | | |
| 7441 | 08682 | 07440 | 1 | | 0 | 0 | 0 | 0 | 0 | 0 | 0 | 0 | 0 | 0 | 0 | 0 | 0 | 0 | 0 | 0 | 0 | 0 | 0 | 0 | 0 | 0 | 1 | 0 | 0 | | | | | | |
| 7442 | 08683 | 07441 | 1 | | 0 | 0 | 0 | 0 | 0 | 0 | 0 | 0 | 0 | 0 | 0 | 0 | 0 | 0 | 0 | 0 | 0 | 0 | 0 | 0 | 0 | 0 | 1 | 0 | 0 | | | | | | |
| 7443 | 08684 | 07442 | 2 | | 0 | 0 | 0 | 0 | 0 | 0 | 0 | 0 | 0 | 0 | 0 | 0 | 0 | 0 | 0 | 0 | 0 | 0 | 0 | 0 | 0 | 0 | 1 | 0 | 1 | | | | | | |
| 7444 | 08685 | 07443 | 1 | | 0 | 0 | 0 | 0 | 0 | 0 | 0 | 0 | 0 | 0 | 0 | 0 | 0 | 0 | 0 | 0 | 0 | 0 | 0 | 0 | 0 | 0 | 1 | 0 | 0 | | | | | | |
| 7445 | 08686 | 07444 | 1 | | 0 | 0 | 0 | 0 | 0 | 0 | 0 | 0 | 0 | 0 | 0 | 0 | 0 | 0 | 0 | 0 | 0 | 0 | 0 | 0 | 0 | 0 | 1 | 0 | 0 | | | | | | |
| 7446 | 08687 | 07445 | 1 | | 0 | 0 | 0 | 0 | 0 | 0 | 0 | 0 | 0 | 0 | 0 | 0 | 0 | 0 | 0 | 0 | 0 | 0 | 0 | 0 | 0 | 0 | 1 | 0 | 0 | | | | | | |
| 7447 | 08688 | 07446 | 2 | | 0 | 0 | 0 | 0 | 0 | 0 | 0 | 0 | 0 | 0 | 0 | 0 | 0 | 0 | 0 | 0 | 0 | 0 | 0 | 0 | 0 | 0 | 1 | 1 | 0 | | | | | | |
| 7448 | 08689 | 07447 | 1 | | 0 | 0 | 0 | 0 | 0 | 0 | 0 | 0 | 0 | 0 | 0 | 0 | 0 | 0 | 0 | 0 | 0 | 0 | 0 | 0 | 0 | 0 | 1 | 0 | 0 | | | | | | |
| 7449 | 08690 | 07448 | 1 | | 0 | 0 | 0 | 0 | 0 | 0 | 0 | 0 | 0 | 0 | 0 | 0 | 0 | 0 | 0 | 0 | 0 | 0 | 0 | 0 | 0 | 0 | 1 | 0 | 0 | | | | | | |
| 7450 | 08691 | 07449 | 1 | | 0 | 0 | 0 | 0 | 0 | 0 | 0 | 0 | 0 | 0 | 0 | 0 | 0 | 0 | 0 | 0 | 0 | 0 | 0 | 0 | 0 | 0 | 1 | 0 | 0 | | | | | | |
| 7451 | 08692 | 07450 | 1 | | 0 | 0 | 0 | 0 | 0 | 0 | 0 | 0 | 0 | 0 | 0 | 0 | 0 | 0 | 0 | 0 | 0 | 0 | 0 | 0 | 0 | 0 | 1 | 0 | 0 | | | | | | |
| 7452 | 08693 | 07451 | 1 | | 0 | 0 | 0 | 0 | 0 | 0 | 0 | 0 | 0 | 0 | 0 | 0 | 0 | 0 | 0 | 0 | 0 | 0 | 0 | 0 | 0 | 0 | 1 | 0 | 0 | | | | | | |
| 7453 | 08694 | 07452 | 1 | | 0 | 0 | 0 | 0 | 0 | 0 | 0 | 0 | 0 | 0 | 0 | 0 | 0 | 0 | 0 | 0 | 0 | 0 | 0 | 0 | 0 | 0 | 1 | 0 | 0 | | | | | | |
| 7454 | 08695 | 07453 | 1 | | 0 | 0 | 0 | 0 | 0 | 0 | 0 | 0 | 0 | 0 | 0 | 0 | 0 | 0 | 0 | 0 | 0 | 0 | 0 | 0 | 0 | 0 | 1 | 0 | 0 | | | | | | |
| 7455 | 08696 | 07454 | 1 | | 0 | 0 | 0 | 0 | 0 | 0 | 0 | 0 | 0 | 0 | 0 | 0 | 0 | 0 | 0 | 0 | 0 | 0 | 0 | 0 | 0 | 0 | 1 | 0 | 0 | | | | | | |
| 7456 | 08697 | 07455 | 1 | | 0 | 0 | 0 | 0 | 0 | 0 | 0 | 0 | 0 | 0 | 0 | 0 | 0 | 0 | 0 | 0 | 0 | 0 | 0 | 0 | 0 | 0 | 1 | 0 | 0 | | | | | | |
| 7457 | 08698 | 07456 | 1 | | 0 | 0 | 0 | 0 | 0 | 0 | 0 | 0 | 0 | 0 | 0 | 0 | 0 | 0 | 0 | 0 | 0 | 0 | 0 | 0 | 0 | 0 | 0 | 1 | 0 | | | | | | |
| 7458 | 08699 | 07457 | 1 | | 0 | 0 | 0 | 0 | 0 | 0 | 0 | 0 | 0 | 0 | 0 | 0 | 0 | 0 | 0 | 0 | 0 | 0 | 0 | 0 | 0 | 0 | 0 | 1 | 0 | | | | | | |
| 7459 | 08700 | 07458 | 2 | | 0 | 0 | 0 | 0 | 0 | 0 | 0 | 0 | 0 | 0 | 0 | 0 | 0 | 0 | 0 | 0 | 0 | 0 | 0 | 0 | 0 | 0 | 0 | 2 | 0 | | | | | | |
| 7460 | 08701 | 07459 | 1 | | 0 | 0 | 0 | 0 | 0 | 0 | 0 | 0 | 0 | 0 | 0 | 0 | 0 | 0 | 0 | 0 | 0 | 0 | 0 | 0 | 0 | 0 | 0 | 1 | 0 | | | | | | |
| 7461 | 08702 | 07460 | 3 | | 0 | 0 | 0 | 0 | 0 | 0 | 0 | 0 | 0 | 0 | 0 | 0 | 0 | 0 | 0 | 0 | 0 | 0 | 0 | 0 | 0 | 0 | 0 | 3 | 0 | L24804 | 100 | 79 | 1 | 704 | 782 |
| 7462 | 08703 | 07461 | 4 | | 0 | 0 | 0 | 0 | 0 | 0 | 0 | 0 | 0 | 0 | 0 | 0 | 0 | 0 | 0 | 0 | 0 | 0 | 0 | 0 | 0 | 0 | 0 | 4 | 0 | | | | | | |
| 7463 | 08704 | 07462 | 1 | | 0 | 0 | 0 | 0 | 0 | 0 | 0 | 0 | 0 | 0 | 0 | 0 | 0 | 0 | 0 | 0 | 0 | 0 | 0 | 0 | 0 | 0 | 0 | 1 | 0 | | | | | | |
| 7464 | 08705 | 07463 | 1 | | 0 | 0 | 0 | 0 | 0 | 0 | 0 | 0 | 0 | 0 | 0 | 0 | 0 | 0 | 0 | 0 | 0 | 0 | 0 | 0 | 0 | 0 | 0 | 1 | 0 | | | | | | |
| 7465 | 08706 | 07464 | 1 | | 0 | 0 | 0 | 0 | 0 | 0 | 0 | 0 | 0 | 0 | 0 | 0 | 0 | 0 | 0 | 0 | 0 | 0 | 0 | 0 | 0 | 0 | 0 | 1 | 0 | | | | | | |
| 7466 | 08707 | 07465 | 1 | | 0 | 0 | 0 | 0 | 0 | 0 | 0 | 0 | 0 | 0 | 0 | 0 | 0 | 0 | 0 | 0 | 0 | 0 | 0 | 0 | 0 | 0 | 0 | 1 | 0 | | | | | | |
| 7467 | 08708 | 07466 | 1 | | 0 | 0 | 0 | 0 | 0 | 0 | 0 | 0 | 0 | 0 | 0 | 0 | 0 | 0 | 0 | 0 | 0 | 0 | 0 | 0 | 0 | 0 | 0 | 1 | 0 | | | | | | |
| 7468 | 08709 | 07467 | 1 | | 0 | 0 | 0 | 0 | 0 | 0 | 0 | 0 | 0 | 0 | 0 | 0 | 0 | 0 | 0 | 0 | 0 | 0 | 0 | 0 | 0 | 0 | 0 | 1 | 0 | | | | | | |
| 7469 | 08710 | 07468 | 1 | | 0 | 0 | 0 | 0 | 0 | 0 | 0 | 0 | 0 | 0 | 0 | 0 | 0 | 0 | 0 | 0 | 0 | 0 | 0 | 0 | 0 | 0 | 0 | 1 | 0 | M61831 | 96.3 | 405 | 8 | 1798 | 2211 |
| 7470 | 08711 | 07469 | 1 | | 0 | 0 | 0 | 0 | 0 | 0 | 0 | 0 | 0 | 0 | 0 | 0 | 0 | 0 | 0 | 0 | 0 | 0 | 0 | 0 | 0 | 0 | 0 | 1 | 0 | | | | | | |
| 7471 | 08712 | 07470 | 1 | | 0 | 0 | 0 | 0 | 0 | 0 | 0 | 0 | 0 | 0 | 0 | 0 | 0 | 0 | 0 | 0 | 0 | 0 | 0 | 0 | 0 | 0 | 0 | 1 | 0 | | | | | | |
| 7472 | 08713 | 07471 | 1 | | 0 | 0 | 0 | 0 | 0 | 0 | 0 | 0 | 0 | 0 | 0 | 0 | 0 | 0 | 0 | 0 | 0 | 0 | 0 | 0 | 0 | 0 | 0 | 1 | 0 | | | | | | |
| 7473 | 08714 | 07472 | 1 | | 0 | 0 | 0 | 0 | 0 | 0 | 0 | 0 | 0 | 0 | 0 | 0 | 0 | 0 | 0 | 0 | 0 | 0 | 0 | 0 | 0 | 0 | 0 | 1 | 0 | | | | | | |

Table 208

| | A | B | C | E | G | I | K | M | O | Q | S | U | W | Y | AA | AC | AE | AG | AI | AK | AM | AO | AQ | AS | AU | AW | AY | BA | BC | BE | BF | BG | BH | BI | BK |
|---|---|---|---|---|---|---|---|---|---|---|---|---|---|---|---|---|---|---|---|---|---|---|---|---|---|---|---|---|---|---|---|---|---|---|---|
| 7474 | 08715 | 07473 | 1 | | 0 | 0 | 0 | 0 | 0 | 0 | 0 | 0 | 0 | 0 | 0 | 0 | 0 | 0 | 0 | 0 | 0 | 0 | 0 | 0 | 0 | 0 | 0 | 1 | 0 | | | | | | |
| 7475 | 08716 | 07474 | 2 | | 0 | 0 | 0 | 0 | 0 | 0 | 0 | 0 | 0 | 0 | 0 | 0 | 0 | 0 | 0 | 0 | 0 | 0 | 0 | 0 | 0 | 0 | 0 | 2 | 0 | | | | | | |
| 7476 | 08717 | 07475 | 2 | | 0 | 0 | 0 | 0 | 0 | 0 | 0 | 0 | 0 | 0 | 0 | 0 | 0 | 0 | 0 | 0 | 0 | 0 | 0 | 0 | 0 | 0 | 0 | 2 | 0 | | | | | | |
| 7477 | 08718 | 07476 | 1 | | 0 | 0 | 0 | 0 | 0 | 0 | 0 | 0 | 0 | 0 | 0 | 0 | 0 | 0 | 0 | 0 | 0 | 0 | 0 | 0 | 0 | 0 | 0 | 1 | 0 | | | | | | |
| 7478 | 08719 | 07477 | 1 | | 0 | 0 | 0 | 0 | 0 | 0 | 0 | 0 | 0 | 0 | 0 | 0 | 0 | 0 | 0 | 0 | 0 | 0 | 0 | 0 | 0 | 0 | 0 | 1 | 0 | | | | | | |
| 7479 | 08720 | 07478 | 1 | | 0 | 0 | 0 | 0 | 0 | 0 | 0 | 0 | 0 | 0 | 0 | 0 | 0 | 0 | 0 | 0 | 0 | 0 | 0 | 0 | 0 | 0 | 0 | 1 | 0 | | | | | | |
| 7480 | 08721 | 07479 | 1 | | 0 | 0 | 0 | 0 | 0 | 0 | 0 | 0 | 0 | 0 | 0 | 0 | 0 | 0 | 0 | 0 | 0 | 0 | 0 | 0 | 0 | 0 | 0 | 1 | 0 | | | | | | |
| 7481 | 08722 | 07480 | 1 | | 0 | 0 | 0 | 0 | 0 | 0 | 0 | 0 | 0 | 0 | 0 | 0 | 0 | 0 | 0 | 0 | 0 | 0 | 0 | 0 | 0 | 0 | 0 | 1 | 0 | | | | | | |
| 7482 | 08723 | 07481 | 1 | | 0 | 0 | 0 | 0 | 0 | 0 | 0 | 0 | 0 | 0 | 0 | 0 | 0 | 0 | 0 | 0 | 0 | 0 | 0 | 0 | 0 | 0 | 0 | 1 | 0 | | | | | | |
| 7483 | 08724 | 07482 | 1 | | 0 | 0 | 0 | 0 | 0 | 0 | 0 | 0 | 0 | 0 | 0 | 0 | 0 | 0 | 0 | 0 | 0 | 0 | 0 | 0 | 0 | 0 | 0 | 1 | 0 | | | | | | |
| 7484 | 08725 | 07483 | 1 | | 0 | 0 | 0 | 0 | 0 | 0 | 0 | 0 | 0 | 0 | 0 | 0 | 0 | 0 | 0 | 0 | 0 | 0 | 0 | 0 | 0 | 0 | 0 | 1 | 0 | | | | | | |
| 7485 | 08726 | 07484 | 1 | | 0 | 0 | 0 | 0 | 0 | 0 | 0 | 0 | 0 | 0 | 0 | 0 | 0 | 0 | 0 | 0 | 0 | 0 | 0 | 0 | 0 | 0 | 0 | 1 | 0 | | | | | | |
| 7486 | 08727 | 07485 | 1 | | 0 | 0 | 0 | 0 | 0 | 0 | 0 | 0 | 0 | 0 | 0 | 0 | 0 | 0 | 0 | 0 | 0 | 0 | 0 | 0 | 0 | 0 | 0 | 1 | 0 | | | | | | |
| 7487 | 08728 | 07486 | 1 | | 0 | 0 | 0 | 0 | 0 | 0 | 0 | 0 | 0 | 0 | 0 | 0 | 0 | 0 | 0 | 0 | 0 | 0 | 0 | 0 | 0 | 0 | 0 | 1 | 0 | | | | | | |
| 7488 | 08729 | 07487 | 1 | | 0 | 0 | 0 | 0 | 0 | 0 | 0 | 0 | 0 | 0 | 0 | 0 | 0 | 0 | 0 | 0 | 0 | 0 | 0 | 0 | 0 | 0 | 0 | 1 | 0 | | | | | | |
| 7489 | 08730 | 07488 | 2 | | 0 | 0 | 0 | 0 | 0 | 0 | 0 | 0 | 0 | 0 | 0 | 0 | 0 | 0 | 0 | 0 | 0 | 0 | 0 | 0 | 0 | 0 | 0 | 2 | 0 | | | | | | |
| 7490 | 08731 | 07489 | 3 | | 0 | 0 | 0 | 0 | 0 | 0 | 0 | 0 | 0 | 0 | 0 | 0 | 0 | 0 | 0 | 0 | 0 | 0 | 0 | 0 | 0 | 0 | 0 | 3 | 0 | | | | | | |
| 7491 | 08732 | 07490 | 1 | | 0 | 0 | 0 | 0 | 0 | 0 | 0 | 0 | 0 | 0 | 0 | 0 | 0 | 0 | 0 | 0 | 0 | 0 | 0 | 0 | 0 | 0 | 0 | 1 | 0 | | | | | | |
| 7492 | 08733 | 07491 | 1 | | 0 | 0 | 0 | 0 | 0 | 0 | 0 | 0 | 0 | 0 | 0 | 0 | 0 | 0 | 0 | 0 | 0 | 0 | 0 | 0 | 0 | 0 | 0 | 1 | 0 | | | | | | |
| 7493 | 08734 | 07492 | 1 | | 0 | 0 | 0 | 0 | 0 | 0 | 0 | 0 | 0 | 0 | 0 | 0 | 0 | 0 | 0 | 0 | 0 | 0 | 0 | 0 | 0 | 0 | 0 | 1 | 0 | | | | | | |
| 7494 | 08735 | 07493 | 1 | | 0 | 0 | 0 | 0 | 0 | 0 | 0 | 0 | 0 | 0 | 0 | 0 | 0 | 0 | 0 | 0 | 0 | 0 | 0 | 0 | 0 | 0 | 0 | 1 | 0 | | | | | | |
| 7495 | 08736 | 07494 | 1 | | 0 | 0 | 0 | 0 | 0 | 0 | 0 | 0 | 0 | 0 | 0 | 0 | 0 | 0 | 0 | 0 | 0 | 0 | 0 | 0 | 0 | 0 | 0 | 1 | 0 | | | | | | |
| 7496 | 08737 | 07495 | 1 | | 0 | 0 | 0 | 0 | 0 | 0 | 0 | 0 | 0 | 0 | 0 | 0 | 0 | 0 | 0 | 0 | 0 | 0 | 0 | 0 | 0 | 0 | 0 | 1 | 0 | | | | | | |
| 7497 | 08738 | 07496 | 1 | | 0 | 0 | 0 | 0 | 0 | 0 | 0 | 0 | 0 | 0 | 0 | 0 | 0 | 0 | 0 | 0 | 0 | 0 | 0 | 0 | 0 | 0 | 0 | 1 | 0 | | | | | | |
| 7498 | 08739 | 07497 | 1 | | 0 | 0 | 0 | 0 | 0 | 0 | 0 | 0 | 0 | 0 | 0 | 0 | 0 | 0 | 0 | 0 | 0 | 0 | 0 | 0 | 0 | 0 | 0 | 1 | 0 | | | | | | |
| 7499 | 08740 | 07498 | 2 | | 0 | 0 | 0 | 0 | 0 | 0 | 0 | 0 | 0 | 0 | 0 | 0 | 0 | 0 | 0 | 0 | 0 | 0 | 0 | 0 | 0 | 0 | 0 | 2 | 0 | | | | | | |
| 7500 | 08741 | 07499 | 1 | | 0 | 0 | 0 | 0 | 0 | 0 | 0 | 0 | 0 | 0 | 0 | 0 | 0 | 0 | 0 | 0 | 0 | 0 | 0 | 0 | 0 | 0 | 0 | 1 | 0 | | | | | | |
| 7501 | 08742 | 07500 | 1 | | 0 | 0 | 0 | 0 | 0 | 0 | 0 | 0 | 0 | 0 | 0 | 0 | 0 | 0 | 0 | 0 | 0 | 0 | 0 | 0 | 0 | 0 | 0 | 1 | 0 | | | | | | |
| 7502 | 08743 | 07501 | 1 | | 0 | 0 | 0 | 0 | 0 | 0 | 0 | 0 | 0 | 0 | 0 | 0 | 0 | 0 | 0 | 0 | 0 | 0 | 0 | 0 | 0 | 0 | 0 | 1 | 0 | | | | | | |
| 7503 | 08744 | 07502 | 1 | | 0 | 0 | 0 | 0 | 0 | 0 | 0 | 0 | 0 | 0 | 0 | 0 | 0 | 0 | 0 | 0 | 0 | 0 | 0 | 0 | 0 | 0 | 0 | 1 | 0 | | | | | | |
| 7504 | 08745 | 07503 | 1 | | 0 | 0 | 0 | 0 | 0 | 0 | 0 | 0 | 0 | 0 | 0 | 0 | 0 | 0 | 0 | 0 | 0 | 0 | 0 | 0 | 0 | 0 | 0 | 1 | 0 | | | | | | |
| 7505 | 08746 | 07504 | 1 | | 0 | 0 | 0 | 0 | 0 | 0 | 0 | 0 | 0 | 0 | 0 | 0 | 0 | 0 | 0 | 0 | 0 | 0 | 0 | 0 | 0 | 0 | 0 | 1 | 0 | | | | | | |
| 7506 | 08747 | 07505 | 1 | | 0 | 0 | 0 | 0 | 0 | 0 | 0 | 0 | 0 | 0 | 0 | 0 | 0 | 0 | 0 | 0 | 0 | 0 | 0 | 0 | 0 | 0 | 0 | 1 | 0 | | | | | | |
| 7507 | 08748 | 07506 | 2 | | 0 | 0 | 0 | 0 | 0 | 0 | 0 | 0 | 0 | 0 | 0 | 0 | 0 | 0 | 0 | 0 | 0 | 0 | 0 | 0 | 0 | 0 | 0 | 2 | 0 | M32886 | 97.3 | 335 | 1 | 471 | 952 |
| 7508 | 08749 | 07507 | 1 | | 0 | 0 | 0 | 0 | 0 | 0 | 0 | 0 | 0 | 0 | 0 | 0 | 0 | 0 | 0 | 0 | 0 | 0 | 0 | 0 | 0 | 0 | 0 | 1 | 0 | X63547 | 91.7 | 338 | 1 | 5220 | 8201 |
| 7509 | 08750 | 07508 | 4 | | 0 | 0 | 0 | 0 | 0 | 0 | 0 | 0 | 0 | 0 | 0 | 0 | 0 | 0 | 0 | 0 | 0 | 0 | 0 | 0 | 0 | 0 | 0 | 4 | 0 | X70649 | 94 | 336 | 1 | 1501 | 2399 |

Table 209

| | A | B | C | E | G | I | K | M | O | Q | S | U | W | Y | AA | AC | AF | AG | AI | AK | AM | AO | AQ | AS | AU | AW | AY | BA | BC | BE | BF | BG | BH | BI | BK |
|---|---|---|---|---|---|---|---|---|---|---|---|---|---|---|---|---|---|---|---|---|---|---|---|---|---|---|---|---|---|---|---|---|---|---|---|
| 7510 | 08751 | 07509 | 1 | | 0 | 0 | 0 | 0 | 0 | 0 | 0 | 0 | 0 | 0 | 0 | 0 | 0 | 0 | 0 | 0 | 0 | 0 | 0 | 0 | 0 | 0 | 0 | 1 | 0 | | | | | | |
| 7511 | 08752 | 07510 | 1 | | 0 | 0 | 0 | 0 | 0 | 0 | 0 | 0 | 0 | 0 | 0 | 0 | 0 | 0 | 0 | 0 | 0 | 0 | 0 | 0 | 0 | 0 | 0 | 1 | 0 | | | | | | |
| 7512 | 08753 | 07511 | 1 | | 0 | 0 | 0 | 0 | 0 | 0 | 0 | 0 | 0 | 0 | 0 | 0 | 0 | 0 | 0 | 0 | 0 | 0 | 0 | 0 | 0 | 0 | 0 | 1 | 0 | | | | | | |
| 7513 | 08754 | 07512 | 1 | | 0 | 0 | 0 | 0 | 0 | 0 | 0 | 0 | 0 | 0 | 0 | 0 | 0 | 0 | 0 | 0 | 0 | 0 | 0 | 0 | 0 | 0 | 0 | 1 | 0 | | | | | | |
| 7514 | 08755 | 07513 | 1 | | 0 | 0 | 0 | 0 | 0 | 0 | 0 | 0 | 0 | 0 | 0 | 0 | 0 | 0 | 0 | 0 | 0 | 0 | 0 | 0 | 0 | 0 | 0 | 1 | 0 | | | | | | |
| 7515 | 08756 | 07514 | 1 | | 0 | 0 | 0 | 0 | 0 | 0 | 0 | 0 | 0 | 0 | 0 | 0 | 0 | 0 | 0 | 0 | 0 | 0 | 0 | 0 | 0 | 0 | 0 | 1 | 0 | | | | | | |
| 7516 | 08757 | 07515 | 1 | | 0 | 0 | 0 | 0 | 0 | 0 | 0 | 0 | 0 | 0 | 0 | 0 | 0 | 0 | 0 | 0 | 0 | 0 | 0 | 0 | 0 | 0 | 0 | 1 | 0 | | | | | | |
| 7517 | 08758 | 07516 | 1 | | 0 | 0 | 0 | 0 | 0 | 0 | 0 | 0 | 0 | 0 | 0 | 0 | 0 | 0 | 0 | 0 | 0 | 0 | 0 | 0 | 0 | 0 | 0 | 1 | 0 | | | | | | |
| 7518 | 08759 | 07517 | 1 | | 0 | 0 | 0 | 0 | 0 | 0 | 0 | 0 | 0 | 0 | 0 | 0 | 0 | 0 | 0 | 0 | 0 | 0 | 0 | 0 | 0 | 0 | 0 | 1 | 0 | D00760 | 97.5 | 326 | 1 | 541 | 866 |
| 7519 | 08760 | 07518 | 1 | | 0 | 0 | 0 | 0 | 0 | 0 | 0 | 0 | 0 | 0 | 0 | 0 | 0 | 0 | 0 | 0 | 0 | 0 | 0 | 0 | 0 | 0 | 0 | 1 | 0 | | | | | | |
| 7520 | 08761 | 07519 | 1 | | 0 | 0 | 0 | 0 | 0 | 0 | 0 | 0 | 0 | 0 | 0 | 0 | 0 | 0 | 0 | 0 | 0 | 0 | 0 | 0 | 0 | 0 | 0 | 1 | 0 | | | | | | |
| 7521 | 08762 | 07520 | 1 | | 0 | 0 | 0 | 0 | 0 | 0 | 0 | 0 | 0 | 0 | 0 | 0 | 0 | 0 | 0 | 0 | 0 | 0 | 0 | 0 | 0 | 0 | 0 | 1 | 0 | | | | | | |
| 7522 | 08763 | 07521 | 1 | | 0 | 0 | 0 | 0 | 0 | 0 | 0 | 0 | 0 | 0 | 0 | 0 | 0 | 0 | 0 | 0 | 0 | 0 | 0 | 0 | 0 | 0 | 0 | 1 | 0 | | | | | | |
| 7523 | 08764 | 07522 | 1 | | 0 | 0 | 0 | 0 | 0 | 0 | 0 | 0 | 0 | 0 | 0 | 0 | 0 | 0 | 0 | 0 | 0 | 0 | 0 | 0 | 0 | 0 | 0 | 1 | 0 | | | | | | |
| 7524 | 08765 | 07523 | 1 | | 0 | 0 | 0 | 0 | 0 | 0 | 0 | 0 | 0 | 0 | 0 | 0 | 0 | 0 | 0 | 0 | 0 | 0 | 0 | 0 | 0 | 0 | 0 | 1 | 0 | | | | | | |
| 7525 | 08766 | 07524 | 1 | | 0 | 0 | 0 | 0 | 0 | 0 | 0 | 0 | 0 | 0 | 0 | 0 | 0 | 0 | 0 | 0 | 0 | 0 | 0 | 0 | 0 | 0 | 0 | 1 | 0 | | | | | | |
| 7526 | 08768 | 07525 | 1 | | 0 | 0 | 0 | 0 | 0 | 0 | 0 | 0 | 0 | 0 | 0 | 0 | 0 | 0 | 0 | 0 | 0 | 0 | 0 | 0 | 0 | 0 | 0 | 1 | 0 | | | | | | |
| 7527 | 08769 | 07526 | 1 | | 0 | 0 | 0 | 0 | 0 | 0 | 0 | 0 | 0 | 0 | 0 | 0 | 0 | 0 | 0 | 0 | 0 | 0 | 0 | 0 | 0 | 0 | 0 | 1 | 0 | M77836 | 99 | 303 | 1 | 1491 | 1792 |
| 7528 | 08770 | 07527 | 1 | | 0 | 0 | 0 | 0 | 0 | 0 | 0 | 0 | 0 | 0 | 0 | 0 | 0 | 0 | 0 | 0 | 0 | 0 | 0 | 0 | 0 | 0 | 0 | 1 | 0 | | | | | | |
| 7529 | 08771 | 07528 | 1 | | 0 | 0 | 0 | 0 | 0 | 0 | 0 | 0 | 0 | 0 | 0 | 0 | 0 | 0 | 0 | 0 | 0 | 0 | 0 | 0 | 0 | 0 | 0 | 1 | 0 | | | | | | |
| 7530 | 08772 | 07529 | 1 | | 0 | 0 | 0 | 0 | 0 | 0 | 0 | 0 | 0 | 0 | 0 | 0 | 0 | 0 | 0 | 0 | 0 | 0 | 0 | 0 | 0 | 0 | 0 | 1 | 0 | | | | | | |
| 7531 | 08773 | 07530 | 1 | | 0 | 0 | 0 | 0 | 0 | 0 | 0 | 0 | 0 | 0 | 0 | 0 | 0 | 0 | 0 | 0 | 0 | 0 | 0 | 0 | 0 | 0 | 0 | 1 | 0 | M27937 | 98.7 | 311 | 1 | 326 | 832 |
| 7532 | 08774 | 07531 | 1 | | 0 | 0 | 0 | 0 | 0 | 0 | 0 | 0 | 0 | 0 | 0 | 0 | 0 | 0 | 0 | 0 | 0 | 0 | 0 | 0 | 0 | 0 | 0 | 1 | 0 | | | | | | |
| 7533 | 08775 | 07532 | 1 | | 0 | 0 | 0 | 0 | 0 | 0 | 0 | 0 | 0 | 0 | 0 | 0 | 0 | 0 | 0 | 0 | 0 | 0 | 0 | 0 | 0 | 0 | 0 | 1 | 0 | | | | | | |
| 7534 | 08777 | 07533 | 1 | | 0 | 0 | 0 | 0 | 0 | 0 | 0 | 0 | 0 | 0 | 0 | 0 | 0 | 0 | 0 | 0 | 0 | 0 | 0 | 0 | 0 | 0 | 0 | 1 | 0 | | | | | | |
| 7535 | 08778 | 07534 | 1 | | 0 | 0 | 0 | 0 | 0 | 0 | 0 | 0 | 0 | 0 | 0 | 0 | 0 | 0 | 0 | 0 | 0 | 0 | 0 | 0 | 0 | 0 | 0 | 1 | 0 | | | | | | |
| 7536 | 08779 | 07535 | 1 | | 0 | 0 | 0 | 0 | 0 | 0 | 0 | 0 | 0 | 0 | 0 | 0 | 0 | 0 | 0 | 0 | 0 | 0 | 0 | 0 | 0 | 0 | 0 | 1 | 0 | | | | | | |
| 7537 | 08780 | 07536 | 1 | | 0 | 0 | 0 | 0 | 0 | 0 | 0 | 0 | 0 | 0 | 0 | 0 | 0 | 0 | 0 | 0 | 0 | 0 | 0 | 0 | 0 | 0 | 0 | 1 | 0 | | | | | | |
| 7538 | 08781 | 07537 | 1 | | 0 | 0 | 0 | 0 | 0 | 0 | 0 | 0 | 0 | 0 | 0 | 0 | 0 | 0 | 0 | 0 | 0 | 0 | 0 | 0 | 0 | 0 | 0 | 1 | 0 | | | | | | |
| 7539 | 08782 | 07538 | 1 | | 0 | 0 | 0 | 0 | 0 | 0 | 0 | 0 | 0 | 0 | 0 | 0 | 0 | 0 | 0 | 0 | 0 | 0 | 0 | 0 | 0 | 0 | 0 | 1 | 0 | | | | | | |
| 7540 | 08783 | 07539 | 1 | | 0 | 0 | 0 | 0 | 0 | 0 | 0 | 0 | 0 | 0 | 0 | 0 | 0 | 0 | 0 | 0 | 0 | 0 | 0 | 0 | 0 | 0 | 0 | 1 | 0 | | | | | | |
| 7541 | 08785 | 07540 | 1 | | 0 | 0 | 0 | 0 | 0 | 0 | 0 | 0 | 0 | 0 | 0 | 0 | 0 | 0 | 0 | 0 | 0 | 0 | 0 | 0 | 0 | 0 | 0 | 1 | 0 | | | | | | |
| 7542 | 08786 | 07541 | 1 | | 0 | 0 | 0 | 0 | 0 | 0 | 0 | 0 | 0 | 0 | 0 | 0 | 0 | 0 | 0 | 0 | 0 | 0 | 0 | 0 | 0 | 0 | 0 | 1 | 0 | | | | | | |
| 7543 | 08787 | 07542 | 1 | | 0 | 0 | 0 | 0 | 0 | 0 | 0 | 0 | 0 | 0 | 0 | 0 | 0 | 0 | 0 | 0 | 0 | 0 | 0 | 0 | 0 | 0 | 0 | 1 | 0 | M87339 | 90.3 | 298 | 1 | 1051 | 1446 |
| 7544 | 08788 | 07543 | 1 | | 0 | 0 | 0 | 0 | 0 | 0 | 0 | 0 | 0 | 0 | 0 | 0 | 0 | 0 | 0 | 0 | 0 | 0 | 0 | 0 | 0 | 0 | 0 | 1 | 0 | | | | | | |
| 7545 | 08789 | 07544 | 1 | | 0 | 0 | 0 | 0 | 0 | 0 | 0 | 0 | 0 | 0 | 0 | 0 | 0 | 0 | 0 | 0 | 0 | 0 | 0 | 0 | 0 | 0 | 0 | 1 | 0 | | | | | | |

Table 210

| | A | B | C | E | G | I | K | M | O | Q | S | U | W | Y | AA | AC | AE | AG | AI | AK | AM | AO | AQ | AS | AU | AW | AY | BA | BC | BE | BF | BG | BH | BI | BK |
|---|---|---|---|---|---|---|---|---|---|---|---|---|---|---|---|---|---|---|---|---|---|---|---|---|---|---|---|---|---|---|---|---|---|---|---|
| 7546 | 08790 | 07545 | 1 | | 0 | 0 | 0 | 0 | 0 | 0 | 0 | 0 | 0 | 0 | 0 | 0 | 0 | 0 | 0 | 0 | 0 | 0 | 0 | 0 | 0 | 0 | 0 | 1 | 0 | | | | | | |
| 7547 | 08791 | 07546 | 1 | | 0 | 0 | 0 | 0 | 0 | 0 | 0 | 0 | 0 | 0 | 0 | 0 | 0 | 0 | 0 | 0 | 0 | 0 | 0 | 0 | 0 | 0 | 0 | 1 | 0 | | | | | | |
| 7548 | 08792 | 07547 | 1 | | 0 | 0 | 0 | 0 | 0 | 0 | 0 | 0 | 0 | 0 | 0 | 0 | 0 | 0 | 0 | 0 | 0 | 0 | 0 | 0 | 0 | 0 | 0 | 1 | 0 | | | | | | |
| 7549 | 08793 | 07548 | 1 | | 0 | 0 | 0 | 0 | 0 | 0 | 0 | 0 | 0 | 0 | 0 | 0 | 0 | 0 | 0 | 0 | 0 | 0 | 0 | 0 | 0 | 0 | 0 | 1 | 0 | | | | | | |
| 7550 | 08794 | 07549 | 1 | | 0 | 0 | 0 | 0 | 0 | 0 | 0 | 0 | 0 | 0 | 0 | 0 | 0 | 0 | 0 | 0 | 0 | 0 | 0 | 0 | 0 | 0 | 0 | 1 | 0 | | | | | | |
| 7551 | 08795 | 07550 | 4 | | 0 | 0 | 0 | 0 | 0 | 0 | 0 | 0 | 0 | 0 | 0 | 0 | 0 | 0 | 0 | 0 | 0 | 0 | 0 | 0 | 0 | 0 | 0 | 4 | 0 | | | | | | |
| 7552 | 08796 | 07551 | 1 | | 0 | 0 | 0 | 0 | 0 | 0 | 0 | 0 | 0 | 0 | 0 | 0 | 0 | 0 | 0 | 0 | 0 | 0 | 0 | 0 | 0 | 0 | 0 | 1 | 0 | | | | | | |
| 7553 | 08797 | 07552 | 1 | | 0 | 0 | 0 | 0 | 0 | 0 | 0 | 0 | 0 | 0 | 0 | 0 | 0 | 0 | 0 | 0 | 0 | 0 | 0 | 0 | 0 | 0 | 0 | 1 | 0 | | | | | | |
| 7554 | 08798 | 07553 | 1 | | 0 | 0 | 0 | 0 | 0 | 0 | 0 | 0 | 0 | 0 | 0 | 0 | 0 | 0 | 0 | 0 | 0 | 0 | 0 | 0 | 0 | 0 | 0 | 1 | 0 | | | | | | |
| 7555 | 08799 | 07554 | 1 | | 0 | 0 | 0 | 0 | 0 | 0 | 0 | 0 | 0 | 0 | 0 | 0 | 0 | 0 | 0 | 0 | 0 | 0 | 0 | 0 | 0 | 0 | 0 | 1 | 0 | | | | | | |
| 7556 | 08800 | 07555 | 1 | | 0 | 0 | 0 | 0 | 0 | 0 | 0 | 0 | 0 | 0 | 0 | 0 | 0 | 0 | 0 | 0 | 0 | 0 | 0 | 0 | 0 | 0 | 0 | 1 | 0 | | | | | | |
| 7557 | 08801 | 07556 | 1 | | 0 | 0 | 0 | 0 | 0 | 0 | 0 | 0 | 0 | 0 | 0 | 0 | 0 | 0 | 0 | 0 | 0 | 0 | 0 | 0 | 0 | 0 | 0 | 1 | 0 | | | | | | |
| 7558 | 08802 | 07557 | 1 | | 0 | 0 | 0 | 0 | 0 | 0 | 0 | 0 | 0 | 0 | 0 | 0 | 0 | 0 | 0 | 0 | 0 | 0 | 0 | 0 | 0 | 0 | 0 | 1 | 0 | | | | | | |
| 7559 | 08803 | 07558 | 1 | | 0 | 0 | 0 | 0 | 0 | 0 | 0 | 0 | 0 | 0 | 0 | 0 | 0 | 0 | 0 | 0 | 0 | 0 | 0 | 0 | 0 | 0 | 0 | 1 | 0 | | | | | | |
| 7560 | 08804 | 07559 | 1 | | 0 | 0 | 0 | 0 | 0 | 0 | 0 | 0 | 0 | 0 | 0 | 0 | 0 | 0 | 0 | 0 | 0 | 0 | 0 | 0 | 0 | 0 | 0 | 1 | 0 | | | | | | |
| 7561 | 08805 | 07560 | 1 | | 0 | 0 | 0 | 0 | 0 | 0 | 0 | 0 | 0 | 0 | 0 | 0 | 0 | 0 | 0 | 0 | 0 | 0 | 0 | 0 | 0 | 0 | 0 | 1 | 0 | | | | | | |
| 7562 | 08807 | 07561 | 1 | | 0 | 0 | 0 | 0 | 0 | 0 | 0 | 0 | 0 | 0 | 0 | 0 | 0 | 0 | 0 | 0 | 0 | 0 | 0 | 0 | 0 | 0 | 0 | 1 | 0 | | | | | | |
| 7563 | 08808 | 07562 | 1 | | 0 | 0 | 0 | 0 | 0 | 0 | 0 | 0 | 0 | 0 | 0 | 0 | 0 | 0 | 0 | 0 | 0 | 0 | 0 | 0 | 0 | 0 | 0 | 1 | 0 | | | | | | |
| 7564 | 08809 | 07563 | 1 | | 0 | 0 | 0 | 0 | 0 | 0 | 0 | 0 | 0 | 0 | 0 | 0 | 0 | 0 | 0 | 0 | 0 | 0 | 0 | 0 | 0 | 0 | 0 | 1 | 0 | | | | | | |
| 7565 | 08810 | 07564 | 1 | | 0 | 0 | 0 | 0 | 0 | 0 | 0 | 0 | 0 | 0 | 0 | 0 | 0 | 0 | 0 | 0 | 0 | 0 | 0 | 0 | 0 | 0 | 0 | 1 | 0 | | | | | | |
| 7566 | 08811 | 07565 | 1 | | 0 | 0 | 0 | 0 | 0 | 0 | 0 | 0 | 0 | 0 | 0 | 0 | 0 | 0 | 0 | 0 | 0 | 0 | 0 | 0 | 0 | 0 | 0 | 1 | 0 | | | | | | |
| 7567 | 08812 | 07566 | 1 | | 0 | 0 | 0 | 0 | 0 | 0 | 0 | 0 | 0 | 0 | 0 | 0 | 0 | 0 | 0 | 0 | 0 | 0 | 0 | 0 | 0 | 0 | 0 | 1 | 0 | | | | | | |
| 7568 | 08813 | 07567 | 1 | | 0 | 0 | 0 | 0 | 0 | 0 | 0 | 0 | 0 | 0 | 0 | 0 | 0 | 0 | 0 | 0 | 0 | 0 | 0 | 0 | 0 | 0 | 0 | 1 | 0 | | | | | | |
| 7569 | 08814 | 07568 | 1 | | 0 | 0 | 0 | 0 | 0 | 0 | 0 | 0 | 0 | 0 | 0 | 0 | 0 | 0 | 0 | 0 | 0 | 0 | 0 | 0 | 0 | 0 | 0 | 1 | 0 | | | | | | |
| 7570 | 08815 | 07569 | 1 | | 0 | 0 | 0 | 0 | 0 | 0 | 0 | 0 | 0 | 0 | 0 | 0 | 0 | 0 | 0 | 0 | 0 | 0 | 0 | 0 | 0 | 0 | 0 | 1 | 0 | | | | | | |
| 7571 | 08816 | 07570 | 1 | | 0 | 0 | 0 | 0 | 0 | 0 | 0 | 0 | 0 | 0 | 0 | 0 | 0 | 0 | 0 | 0 | 0 | 0 | 0 | 0 | 0 | 0 | 0 | 1 | 0 | | | | | | |
| 7572 | 08817 | 07571 | 1 | | 0 | 0 | 0 | 0 | 0 | 0 | 0 | 0 | 0 | 0 | 0 | 0 | 0 | 0 | 0 | 0 | 0 | 0 | 0 | 0 | 0 | 0 | 0 | 1 | 0 | | | | | | |
| 7573 | 08818 | 07572 | 1 | | 0 | 0 | 0 | 0 | 0 | 0 | 0 | 0 | 0 | 0 | 0 | 0 | 0 | 0 | 0 | 0 | 0 | 0 | 0 | 0 | 0 | 0 | 0 | 1 | 0 | | | | | | |
| 7574 | 08819 | 07573 | 1 | | 0 | 0 | 0 | 0 | 0 | 0 | 0 | 0 | 0 | 0 | 0 | 0 | 0 | 0 | 0 | 0 | 0 | 0 | 0 | 0 | 0 | 0 | 0 | 1 | 0 | | | | | | |
| 7575 | 08820 | 07574 | 1 | | 0 | 0 | 0 | 0 | 0 | 0 | 0 | 0 | 0 | 0 | 0 | 0 | 0 | 0 | 0 | 0 | 0 | 0 | 0 | 0 | 0 | 0 | 0 | 1 | 0 | | | | | | |
| 7576 | 08821 | 07575 | 1 | | 0 | 0 | 0 | 0 | 0 | 0 | 0 | 0 | 0 | 0 | 0 | 0 | 0 | 0 | 0 | 0 | 0 | 0 | 0 | 0 | 0 | 0 | 0 | 1 | 0 | | | | | | |
| 7577 | 08822 | 07576 | 1 | | 0 | 0 | 0 | 0 | 0 | 0 | 0 | 0 | 0 | 0 | 0 | 0 | 0 | 0 | 0 | 0 | 0 | 0 | 0 | 0 | 0 | 0 | 0 | 1 | 0 | | | | | | |
| 7578 | 08823 | 07577 | 1 | | 0 | 0 | 0 | 0 | 0 | 0 | 0 | 0 | 0 | 0 | 0 | 0 | 0 | 0 | 0 | 0 | 0 | 0 | 0 | 0 | 0 | 0 | 0 | 1 | 0 | | | | | | |
| 7579 | 08824 | 07578 | 1 | | 0 | 0 | 0 | 0 | 0 | 0 | 0 | 0 | 0 | 0 | 0 | 0 | 0 | 0 | 0 | 0 | 0 | 0 | 0 | 0 | 0 | 0 | 0 | 1 | 0 | | | | | | |
| 7580 | 08825 | 07579 | 1 | | 0 | 0 | 0 | 0 | 0 | 0 | 0 | 0 | 0 | 0 | 0 | 0 | 0 | 0 | 0 | 0 | 0 | 0 | 0 | 0 | 0 | 0 | 0 | 1 | 0 | | | | | | |
| 7581 | 08826 | 07580 | 1 | | 0 | 0 | 0 | 0 | 0 | 0 | 0 | 0 | 0 | 0 | 0 | 0 | 0 | 0 | 0 | 0 | 0 | 0 | 0 | 0 | 0 | 0 | 0 | 1 | 0 | | | | | | |

Table 211

| | A | B | C | E | G | I | K | M | O | Q | S | U | W | Y | AA | AC | AE | AG | AI | AK | AM | AO | AQ | AS | AU | AW | AY | BA | BC | BE | BF | BG | BH | BI | BK |
|---|---|---|---|---|---|---|---|---|---|---|---|---|---|---|---|---|---|---|---|---|---|---|---|---|---|---|---|---|---|---|---|---|---|---|---|
| 7582 | 08827 | 07581 | 1 | | 0 | 0 | 0 | 0 | 0 | 0 | 0 | 0 | 0 | 0 | 0 | 0 | 0 | 0 | 0 | 0 | 0 | 0 | 0 | 0 | 0 | 0 | 0 | 1 | 0 | | | | | | |
| 7583 | 08828 | 07582 | 1 | | 0 | 0 | 0 | 0 | 0 | 0 | 0 | 0 | 0 | 0 | 0 | 0 | 0 | 0 | 0 | 0 | 0 | 0 | 0 | 0 | 0 | 0 | 0 | 1 | 0 | | | | | | |
| 7584 | 08829 | 07583 | 1 | | 0 | 0 | 0 | 0 | 0 | 0 | 0 | 0 | 0 | 0 | 0 | 0 | 0 | 0 | 0 | 0 | 0 | 0 | 0 | 0 | 0 | 0 | 0 | 1 | 0 | | | | | | |
| 7585 | 08830 | 07584 | 1 | | 0 | 0 | 0 | 0 | 0 | 0 | 0 | 0 | 0 | 0 | 0 | 0 | 0 | 0 | 0 | 0 | 0 | 0 | 0 | 0 | 0 | 0 | 0 | 1 | 0 | | | | | | |
| 7586 | 08831 | 07585 | 4 | | 0 | 0 | 0 | 0 | 0 | 0 | 0 | 0 | 0 | 0 | 0 | 0 | 0 | 0 | 0 | 0 | 0 | 0 | 0 | 0 | 0 | 0 | 0 | 4 | 0 | | | | | | |
| 7587 | 08832 | 07586 | 1 | | 0 | 0 | 0 | 0 | 0 | 0 | 0 | 0 | 0 | 0 | 0 | 0 | 0 | 0 | 0 | 0 | 0 | 0 | 0 | 0 | 0 | 0 | 0 | 1 | 0 | | | | | | |
| 7588 | 08833 | 07587 | 1 | | 0 | 0 | 0 | 0 | 0 | 0 | 0 | 0 | 0 | 0 | 0 | 0 | 0 | 0 | 0 | 0 | 0 | 0 | 0 | 0 | 0 | 0 | 0 | 1 | 0 | | | | | | |
| 7589 | 08834 | 07588 | 1 | | 0 | 0 | 0 | 0 | 0 | 0 | 0 | 0 | 0 | 0 | 0 | 0 | 0 | 0 | 0 | 0 | 0 | 0 | 0 | 0 | 0 | 0 | 0 | 1 | 0 | | | | | | |
| 7590 | 08835 | 07589 | 1 | | 0 | 0 | 0 | 0 | 0 | 0 | 0 | 0 | 0 | 0 | 0 | 0 | 0 | 0 | 0 | 0 | 0 | 0 | 0 | 0 | 0 | 0 | 0 | 1 | 0 | | | | | | |
| 7591 | 08836 | 07590 | 1 | | 0 | 0 | 0 | 0 | 0 | 0 | 0 | 0 | 0 | 0 | 0 | 0 | 0 | 0 | 0 | 0 | 0 | 0 | 0 | 0 | 0 | 0 | 0 | 1 | 0 | | | | | | |
| 7592 | 08837 | 07591 | 1 | | 0 | 0 | 0 | 0 | 0 | 0 | 0 | 0 | 0 | 0 | 0 | 0 | 0 | 0 | 0 | 0 | 0 | 0 | 0 | 0 | 0 | 0 | 0 | 1 | 0 | | | | | | |
| 7593 | 08838 | 07592 | 1 | | 0 | 0 | 0 | 0 | 0 | 0 | 0 | 0 | 0 | 0 | 0 | 0 | 0 | 0 | 0 | 0 | 0 | 0 | 0 | 0 | 0 | 0 | 0 | 1 | 0 | | | | | | |
| 7594 | 08839 | 07593 | 1 | | 0 | 0 | 0 | 0 | 0 | 0 | 0 | 0 | 0 | 0 | 0 | 0 | 0 | 0 | 0 | 0 | 0 | 0 | 0 | 0 | 0 | 0 | 0 | 1 | 0 | | | | | | |
| 7595 | 08840 | 07594 | 1 | | 0 | 0 | 0 | 0 | 0 | 0 | 0 | 0 | 0 | 0 | 0 | 0 | 0 | 0 | 0 | 0 | 0 | 0 | 0 | 0 | 0 | 0 | 0 | 1 | 0 | | | | | | |
| 7596 | 08841 | 07595 | 1 | | 0 | 0 | 0 | 0 | 0 | 0 | 0 | 0 | 0 | 0 | 0 | 0 | 0 | 0 | 0 | 0 | 0 | 0 | 0 | 0 | 0 | 0 | 0 | 1 | 0 | | | | | | |
| 7597 | 08842 | 07596 | 1 | | 0 | 0 | 0 | 0 | 0 | 0 | 0 | 0 | 0 | 0 | 0 | 0 | 0 | 0 | 0 | 0 | 0 | 0 | 0 | 0 | 0 | 0 | 0 | 1 | 0 | | | | | | |
| 7598 | 08843 | 07597 | 1 | | 0 | 0 | 0 | 0 | 0 | 0 | 0 | 0 | 0 | 0 | 0 | 0 | 0 | 0 | 0 | 0 | 0 | 0 | 0 | 0 | 0 | 0 | 0 | 1 | 0 | | | | | | |
| 7599 | 08844 | 07598 | 1 | | 0 | 0 | 0 | 0 | 0 | 0 | 0 | 0 | 0 | 0 | 0 | 0 | 0 | 0 | 0 | 0 | 0 | 0 | 0 | 0 | 0 | 0 | 0 | 1 | 0 | | | | | | |
| 7600 | 08845 | 07599 | 1 | | 0 | 0 | 0 | 0 | 0 | 0 | 0 | 0 | 0 | 0 | 0 | 0 | 0 | 0 | 0 | 0 | 0 | 0 | 0 | 0 | 0 | 0 | 0 | 1 | 0 | | | | | | |
| 7601 | 08846 | 07600 | 1 | | 0 | 0 | 0 | 0 | 0 | 0 | 0 | 0 | 0 | 0 | 0 | 0 | 0 | 0 | 0 | 0 | 0 | 0 | 0 | 0 | 0 | 0 | 0 | 1 | 0 | | | | | | |
| 7602 | 08848 | 07601 | 1 | | 0 | 0 | 0 | 0 | 0 | 0 | 0 | 0 | 0 | 0 | 0 | 0 | 0 | 0 | 0 | 0 | 0 | 0 | 0 | 0 | 0 | 0 | 0 | 1 | 0 | | | | | | |
| 7603 | 08849 | 07602 | 1 | | 0 | 0 | 0 | 0 | 0 | 0 | 0 | 0 | 0 | 0 | 0 | 0 | 0 | 0 | 0 | 0 | 0 | 0 | 0 | 0 | 0 | 0 | 0 | 1 | 0 | | | | | | |
| 7604 | 08850 | 07603 | 1 | | 0 | 0 | 0 | 0 | 0 | 0 | 0 | 0 | 0 | 0 | 0 | 0 | 0 | 0 | 0 | 0 | 0 | 0 | 0 | 0 | 0 | 0 | 0 | 1 | 0 | | | | | | |
| 7605 | 08851 | 07604 | 1 | | 0 | 0 | 0 | 0 | 0 | 0 | 0 | 0 | 0 | 0 | 0 | 0 | 0 | 0 | 0 | 0 | 0 | 0 | 0 | 0 | 0 | 0 | 0 | 1 | 0 | | | | | | |
| 7606 | 08852 | 07605 | 1 | | 0 | 0 | 0 | 0 | 0 | 0 | 0 | 0 | 0 | 0 | 0 | 0 | 0 | 0 | 0 | 0 | 0 | 0 | 0 | 0 | 0 | 0 | 0 | 1 | 0 | | | | | | |
| 7607 | 08853 | 07606 | 1 | | 0 | 0 | 0 | 0 | 0 | 0 | 0 | 0 | 0 | 0 | 0 | 0 | 0 | 0 | 0 | 0 | 0 | 0 | 0 | 0 | 0 | 0 | 0 | 1 | 0 | | | | | | |
| 7608 | 08854 | 07607 | 1 | | 0 | 0 | 0 | 0 | 0 | 0 | 0 | 0 | 0 | 0 | 0 | 0 | 0 | 0 | 0 | 0 | 0 | 0 | 0 | 0 | 0 | 0 | 0 | 1 | 0 | | | | | | |
| 7609 | 08855 | 07608 | 2 | | 0 | 0 | 0 | 0 | 0 | 0 | 0 | 0 | 0 | 0 | 0 | 0 | 0 | 0 | 0 | 0 | 0 | 0 | 0 | 0 | 0 | 0 | 0 | 2 | 0 | | | | | | |
| 7610 | 08856 | 07609 | 1 | | 0 | 0 | 0 | 0 | 0 | 0 | 0 | 0 | 0 | 0 | 0 | 0 | 0 | 0 | 0 | 0 | 0 | 0 | 0 | 0 | 0 | 0 | 0 | 1 | 0 | | | | | | |
| 7611 | 08857 | 07610 | 1 | | 0 | 0 | 0 | 0 | 0 | 0 | 0 | 0 | 0 | 0 | 0 | 0 | 0 | 0 | 0 | 0 | 0 | 0 | 0 | 0 | 0 | 0 | 0 | 1 | 0 | | | | | | |
| 7612 | 08858 | 07611 | 2 | | 0 | 0 | 0 | 0 | 0 | 0 | 0 | 0 | 0 | 0 | 0 | 0 | 0 | 0 | 0 | 0 | 0 | 0 | 0 | 0 | 0 | 0 | 0 | 2 | 0 | | | | | | |
| 7613 | 08859 | 07612 | 1 | | 0 | 0 | 0 | 0 | 0 | 0 | 0 | 0 | 0 | 0 | 0 | 0 | 0 | 0 | 0 | 0 | 0 | 0 | 0 | 0 | 0 | 0 | 0 | 1 | 0 | | | | | | |
| 7614 | 08860 | 07613 | 1 | | 0 | 0 | 0 | 0 | 0 | 0 | 0 | 0 | 0 | 0 | 0 | 0 | 0 | 0 | 0 | 0 | 0 | 0 | 0 | 0 | 0 | 0 | 0 | 1 | 0 | | | | | | |
| 7615 | 08861 | 07614 | 1 | | 0 | 0 | 0 | 0 | 0 | 0 | 0 | 0 | 0 | 0 | 0 | 0 | 0 | 0 | 0 | 0 | 0 | 0 | 0 | 0 | 0 | 0 | 0 | 1 | 0 | | | | | | |
| 7616 | 08862 | 07615 | 1 | | 0 | 0 | 0 | 0 | 0 | 0 | 0 | 0 | 0 | 0 | 0 | 0 | 0 | 0 | 0 | 0 | 0 | 0 | 0 | 0 | 0 | 0 | 0 | 1 | 0 | | | | | | |
| 7617 | 08863 | 07616 | 1 | | 0 | 0 | 0 | 0 | 0 | 0 | 0 | 0 | 0 | 0 | 0 | 0 | 0 | 0 | 0 | 0 | 0 | 0 | 0 | 0 | 0 | 0 | 0 | 1 | 0 | | | | | | |

Table 212

| | A | B | C | E | G | I | K | M | O | Q | S | U | W | Y | AA | AC | AE | AG | AI | AK | AM | AO | AQ | AS | AU | AW | AY | BA | BC | BE | BF | BG | BH | BI | BK |
|---|---|---|---|---|---|---|---|---|---|---|---|---|---|---|---|---|---|---|---|---|---|---|---|---|---|---|---|---|---|---|---|---|---|---|---|
| 7618 | 08864 | 07617 | 1 | | 0 | 0 | 0 | 0 | 0 | 0 | 0 | 0 | 0 | 0 | 0 | 0 | 0 | 0 | 0 | 0 | 0 | 0 | 0 | 0 | 0 | 0 | 0 | 1 | 0 | X68742 | 94.1 | 101 | 1 | 263 | 3453 |
| 7619 | 08865 | 07618 | 1 | | 0 | 0 | 0 | 0 | 0 | 0 | 0 | 0 | 0 | 0 | 0 | 0 | 0 | 0 | 0 | 0 | 0 | 0 | 0 | 0 | 0 | 0 | 0 | 1 | 0 | | | | | | |
| 7620 | 08866 | 07619 | 1 | | 0 | 0 | 0 | 0 | 0 | 0 | 0 | 0 | 0 | 0 | 0 | 0 | 0 | 0 | 0 | 0 | 0 | 0 | 0 | 0 | 0 | 0 | 0 | 1 | 0 | | | | | | |
| 7621 | 08867 | 07620 | 1 | | 0 | 0 | 0 | 0 | 0 | 0 | 0 | 0 | 0 | 0 | 0 | 0 | 0 | 0 | 0 | 0 | 0 | 0 | 0 | 0 | 0 | 0 | 0 | 1 | 0 | | | | | | |
| 7622 | 08868 | 07621 | 1 | | 0 | 0 | 0 | 0 | 0 | 0 | 0 | 0 | 0 | 0 | 0 | 0 | 0 | 0 | 0 | 0 | 0 | 0 | 0 | 0 | 0 | 0 | 0 | 1 | 0 | | | | | | |
| 7623 | 08869 | 07622 | 1 | | 0 | 0 | 0 | 0 | 0 | 0 | 0 | 0 | 0 | 0 | 0 | 0 | 0 | 0 | 0 | 0 | 0 | 0 | 0 | 0 | 0 | 0 | 0 | 1 | 0 | | | | | | |
| 7624 | 08870 | 07623 | 2 | | 0 | 0 | 0 | 0 | 0 | 0 | 0 | 0 | 0 | 0 | 0 | 0 | 0 | 0 | 0 | 0 | 0 | 0 | 0 | 0 | 0 | 0 | 0 | 1 | 1 | | | | | | |
| 7625 | 08871 | 07624 | 1 | | 0 | 0 | 0 | 0 | 0 | 0 | 0 | 0 | 0 | 0 | 0 | 0 | 0 | 0 | 0 | 0 | 0 | 0 | 0 | 0 | 0 | 0 | 0 | 1 | 0 | M23613 | 94.8 | 290 | 83 | 966 | 1296 |
| 7626 | 08872 | 07625 | 1 | | 0 | 0 | 0 | 0 | 0 | 0 | 0 | 0 | 0 | 0 | 0 | 0 | 0 | 0 | 0 | 0 | 0 | 0 | 0 | 0 | 0 | 0 | 0 | 1 | 0 | | | | | | |
| 7627 | 08873 | 07626 | 1 | | 0 | 0 | 0 | 0 | 0 | 0 | 0 | 0 | 0 | 0 | 0 | 0 | 0 | 0 | 0 | 0 | 0 | 0 | 0 | 0 | 0 | 0 | 0 | 1 | 0 | | | | | | |
| 7628 | 08874 | 07627 | 1 | | 0 | 0 | 0 | 0 | 0 | 0 | 0 | 0 | 0 | 0 | 0 | 0 | 0 | 0 | 0 | 0 | 0 | 0 | 0 | 0 | 0 | 0 | 0 | 1 | 0 | | | | | | |
| 7629 | 08875 | 07628 | 1 | | 0 | 0 | 0 | 0 | 0 | 0 | 0 | 0 | 0 | 0 | 0 | 0 | 0 | 0 | 0 | 0 | 0 | 0 | 0 | 0 | 0 | 0 | 0 | 1 | 0 | | | | | | |
| 7630 | 08876 | 07629 | 1 | | 0 | 0 | 0 | 0 | 0 | 0 | 0 | 0 | 0 | 0 | 0 | 0 | 0 | 0 | 0 | 0 | 0 | 0 | 0 | 0 | 0 | 0 | 0 | 1 | 0 | | | | | | |
| 7631 | 08877 | 07630 | 1 | | 0 | 0 | 0 | 0 | 0 | 0 | 0 | 0 | 0 | 0 | 0 | 0 | 0 | 0 | 0 | 0 | 0 | 0 | 0 | 0 | 0 | 0 | 0 | 1 | 0 | | | | | | |
| 7632 | 08878 | 07631 | 1 | | 0 | 0 | 0 | 0 | 0 | 0 | 0 | 0 | 0 | 0 | 0 | 0 | 0 | 0 | 0 | 0 | 0 | 0 | 0 | 0 | 0 | 0 | 0 | 1 | 0 | | | | | | |
| 7633 | 08879 | 07632 | 1 | | 0 | 0 | 0 | 0 | 0 | 0 | 0 | 0 | 0 | 0 | 0 | 0 | 0 | 0 | 0 | 0 | 0 | 0 | 0 | 0 | 0 | 0 | 0 | 1 | 0 | | | | | | |
| 7634 | 08880 | 07633 | 1 | | 0 | 0 | 0 | 0 | 0 | 0 | 0 | 0 | 0 | 0 | 0 | 0 | 0 | 0 | 0 | 0 | 0 | 0 | 0 | 0 | 0 | 0 | 0 | 1 | 0 | | | | | | |
| 7635 | 08881 | 07634 | 1 | | 0 | 0 | 0 | 0 | 0 | 0 | 0 | 0 | 0 | 0 | 0 | 0 | 0 | 0 | 0 | 0 | 0 | 0 | 0 | 0 | 0 | 0 | 0 | 1 | 0 | | | | | | |
| 7636 | 08882 | 07635 | 1 | | 0 | 0 | 0 | 0 | 0 | 0 | 0 | 0 | 0 | 0 | 0 | 0 | 0 | 0 | 0 | 0 | 0 | 0 | 0 | 0 | 0 | 0 | 0 | 1 | 0 | | | | | | |
| 7637 | 08883 | 07636 | 1 | | 0 | 0 | 0 | 0 | 0 | 0 | 0 | 0 | 0 | 0 | 0 | 0 | 0 | 0 | 0 | 0 | 0 | 0 | 0 | 0 | 0 | 0 | 0 | 1 | 0 | | | | | | |
| 7638 | 08884 | 07637 | 1 | | 0 | 0 | 0 | 0 | 0 | 0 | 0 | 0 | 0 | 0 | 0 | 0 | 0 | 0 | 0 | 0 | 0 | 0 | 0 | 0 | 0 | 0 | 0 | 1 | 0 | | | | | | |
| 7639 | 08885 | 07638 | 3 | | 0 | 0 | 0 | 0 | 0 | 0 | 0 | 0 | 0 | 0 | 0 | 0 | 0 | 0 | 0 | 0 | 0 | 0 | 0 | 0 | 0 | 0 | 0 | 3 | 0 | | | | | | |
| 7640 | 08886 | 07639 | 3 | | 0 | 0 | 0 | 0 | 0 | 0 | 0 | 0 | 0 | 0 | 0 | 0 | 0 | 0 | 0 | 0 | 0 | 0 | 0 | 0 | 0 | 0 | 0 | 3 | 0 | | | | | | |
| 7641 | 08887 | 07640 | 2 | | 0 | 0 | 0 | 0 | 0 | 0 | 0 | 0 | 0 | 0 | 0 | 0 | 0 | 0 | 0 | 0 | 0 | 0 | 0 | 0 | 0 | 0 | 0 | 2 | 0 | | | | | | |
| 7642 | 08888 | 07641 | 1 | | 0 | 0 | 0 | 0 | 0 | 0 | 0 | 0 | 0 | 0 | 0 | 0 | 0 | 0 | 0 | 0 | 0 | 0 | 0 | 0 | 0 | 0 | 0 | 1 | 0 | | | | | | |
| 7643 | 08889 | 07642 | 2 | | 0 | 0 | 0 | 0 | 0 | 0 | 0 | 0 | 0 | 0 | 0 | 0 | 0 | 0 | 0 | 0 | 0 | 0 | 0 | 0 | 0 | 0 | 0 | 2 | 0 | | | | | | |
| 7644 | 08890 | 07643 | 1 | | 0 | 0 | 0 | 0 | 0 | 0 | 0 | 0 | 0 | 0 | 0 | 0 | 0 | 0 | 0 | 0 | 0 | 0 | 0 | 0 | 0 | 0 | 0 | 1 | 0 | | | | | | |
| 7645 | 08891 | 07644 | 1 | | 0 | 0 | 0 | 0 | 0 | 0 | 0 | 0 | 0 | 0 | 0 | 0 | 0 | 0 | 0 | 0 | 0 | 0 | 0 | 0 | 0 | 0 | 0 | 1 | 0 | | | | | | |
| 7646 | 08892 | 07645 | 1 | | 0 | 0 | 0 | 0 | 0 | 0 | 0 | 0 | 0 | 0 | 0 | 0 | 0 | 0 | 0 | 0 | 0 | 0 | 0 | 0 | 0 | 0 | 0 | 1 | 0 | | | | | | |
| 7647 | 08893 | 07646 | 1 | | 0 | 0 | 0 | 0 | 0 | 0 | 0 | 0 | 0 | 0 | 0 | 0 | 0 | 0 | 0 | 0 | 0 | 0 | 0 | 0 | 0 | 0 | 0 | 1 | 0 | | | | | | |
| 7648 | 08894 | 07647 | 1 | | 0 | 0 | 0 | 0 | 0 | 0 | 0 | 0 | 0 | 0 | 0 | 0 | 0 | 0 | 0 | 0 | 0 | 0 | 0 | 0 | 0 | 0 | 0 | 1 | 0 | M55409 | 100 | 79 | 1 | 1067 | 1155 |
| 7649 | 08895 | 07648 | 1 | | 0 | 0 | 0 | 0 | 0 | 0 | 0 | 0 | 0 | 0 | 0 | 0 | 0 | 0 | 0 | 0 | 0 | 0 | 0 | 0 | 0 | 0 | 0 | 1 | 0 | | | | | | |
| 7650 | 08896 | 07649 | 1 | | 0 | 0 | 0 | 0 | 0 | 0 | 0 | 0 | 0 | 0 | 0 | 0 | 0 | 0 | 0 | 0 | 0 | 0 | 0 | 0 | 0 | 0 | 0 | 1 | 0 | | | | | | |
| 7651 | 08897 | 07650 | 1 | | 0 | 0 | 0 | 0 | 0 | 0 | 0 | 0 | 0 | 0 | 0 | 0 | 0 | 0 | 0 | 0 | 0 | 0 | 0 | 0 | 0 | 0 | 0 | 1 | 0 | | | | | | |
| 7652 | 08898 | 07651 | 1 | | 0 | 0 | 0 | 0 | 0 | 0 | 0 | 0 | 0 | 0 | 0 | 0 | 0 | 0 | 0 | 0 | 0 | 0 | 0 | 0 | 0 | 0 | 0 | 1 | 0 | | | | | | |
| 7653 | 08899 | 07652 | 1 | | 0 | 0 | 0 | 0 | 0 | 0 | 0 | 0 | 0 | 0 | 0 | 0 | 0 | 0 | 0 | 0 | 0 | 0 | 0 | 0 | 0 | 0 | 0 | 1 | 0 | D11466 | 97.1 | 275 | 1 | 3253 | 3589 |

Table 213

| | A | B | C | E | G | I | K | M | O | Q | S | U | W | Y | AA | AC | AE | AG | AI | AK | AM | AO | AQ | AS | AU | AW | AY | BA | BC | BE | BF | BG | BH | BI | BK |
|---|---|---|---|---|---|---|---|---|---|---|---|---|---|---|---|---|---|---|---|---|---|---|---|---|---|---|---|---|---|---|---|---|---|---|---|
| 7654 | 08900 | 07653 | 1 | | 0 | 0 | 0 | 0 | 0 | 0 | 0 | 0 | 0 | 0 | 0 | 0 | 0 | 0 | 0 | 0 | 0 | 0 | 0 | 0 | 0 | 0 | 0 | 1 | 0 | | | | | | |
| 7655 | 08901 | 07654 | 1 | | 0 | 0 | 0 | 0 | 0 | 0 | 0 | 0 | 0 | 0 | 0 | 0 | 0 | 0 | 0 | 0 | 0 | 0 | 0 | 0 | 0 | 0 | 0 | 1 | 0 | | | | | | |
| 7656 | 08902 | 07655 | 1 | | 0 | 0 | 0 | 0 | 0 | 0 | 0 | 0 | 0 | 0 | 0 | 0 | 0 | 0 | 0 | 0 | 0 | 0 | 0 | 0 | 0 | 0 | 0 | 1 | 0 | | | | | | |
| 7657 | 08903 | 07656 | 1 | | 0 | 0 | 0 | 0 | 0 | 0 | 0 | 0 | 0 | 0 | 0 | 0 | 0 | 0 | 0 | 0 | 0 | 0 | 0 | 0 | 0 | 0 | 0 | 1 | 0 | | | | | | |
| 7658 | 08904 | 07657 | 1 | | 0 | 0 | 0 | 0 | 0 | 0 | 0 | 0 | 0 | 0 | 0 | 0 | 0 | 0 | 0 | 0 | 0 | 0 | 0 | 0 | 0 | 0 | 0 | 1 | 0 | | | | | | |
| 7659 | 08905 | 07658 | 1 | | 0 | 0 | 0 | 0 | 0 | 0 | 0 | 0 | 0 | 0 | 0 | 0 | 0 | 0 | 0 | 0 | 0 | 0 | 0 | 0 | 0 | 0 | 0 | 1 | 0 | | | | | | |
| 7660 | 08906 | 07659 | 2 | | 0 | 0 | 0 | 0 | 0 | 0 | 0 | 0 | 0 | 0 | 0 | 0 | 0 | 0 | 0 | 0 | 0 | 0 | 0 | 0 | 0 | 0 | 0 | 2 | 0 | | | | | | |
| 7661 | 08908 | 07660 | 1 | | 0 | 0 | 0 | 0 | 0 | 0 | 0 | 0 | 0 | 0 | 0 | 0 | 0 | 0 | 0 | 0 | 0 | 0 | 0 | 0 | 0 | 0 | 0 | 1 | 0 | | | | | | |
| 7662 | 08909 | 07661 | 1 | | 0 | 0 | 0 | 0 | 0 | 0 | 0 | 0 | 0 | 0 | 0 | 0 | 0 | 0 | 0 | 0 | 0 | 0 | 0 | 0 | 0 | 0 | 0 | 1 | 0 | | | | | | |
| 7663 | 08910 | 07662 | 1 | | 0 | 0 | 0 | 0 | 0 | 0 | 0 | 0 | 0 | 0 | 0 | 0 | 0 | 0 | 0 | 0 | 0 | 0 | 0 | 0 | 0 | 0 | 0 | 1 | 0 | | | | | | |
| 7664 | 08912 | 07663 | 1 | | 0 | 0 | 0 | 0 | 0 | 0 | 0 | 0 | 0 | 0 | 0 | 0 | 0 | 0 | 0 | 0 | 0 | 0 | 0 | 0 | 0 | 0 | 0 | 1 | 0 | | | | | | |
| 7665 | 08913 | 07664 | 1 | | 0 | 0 | 0 | 0 | 0 | 0 | 0 | 0 | 0 | 0 | 0 | 0 | 0 | 0 | 0 | 0 | 0 | 0 | 0 | 0 | 0 | 0 | 0 | 1 | 0 | | | | | | |
| 7666 | 08914 | 07665 | 1 | | 0 | 0 | 0 | 0 | 0 | 0 | 0 | 0 | 0 | 0 | 0 | 0 | 0 | 0 | 0 | 0 | 0 | 0 | 0 | 0 | 0 | 0 | 0 | 1 | 0 | M84810 | 97.2 | 71 | 1 | 2201 | 2284 |
| 7667 | 08915 | 07666 | 1 | | 0 | 0 | 0 | 0 | 0 | 0 | 0 | 0 | 0 | 0 | 0 | 0 | 0 | 0 | 0 | 0 | 0 | 0 | 0 | 0 | 0 | 0 | 0 | 1 | 0 | | | | | | |
| 7668 | 08916 | 07667 | 2 | | 0 | 0 | 0 | 0 | 0 | 0 | 0 | 0 | 0 | 0 | 0 | 0 | 0 | 0 | 0 | 0 | 0 | 0 | 0 | 0 | 0 | 0 | 0 | 2 | 0 | | | | | | |
| 7669 | 08917 | 07668 | 1 | | 0 | 0 | 0 | 0 | 0 | 0 | 0 | 0 | 0 | 0 | 0 | 0 | 0 | 0 | 0 | 0 | 0 | 0 | 0 | 0 | 0 | 0 | 0 | 1 | 0 | | | | | | |
| 7670 | 08919 | 07669 | 1 | | 0 | 0 | 0 | 0 | 0 | 0 | 0 | 0 | 0 | 0 | 0 | 0 | 0 | 0 | 0 | 0 | 0 | 0 | 0 | 0 | 0 | 0 | 0 | 1 | 0 | | | | | | |
| 7671 | 08920 | 07670 | 1 | | 0 | 0 | 0 | 0 | 0 | 0 | 0 | 0 | 0 | 0 | 0 | 0 | 0 | 0 | 0 | 0 | 0 | 0 | 0 | 0 | 0 | 0 | 0 | 1 | 0 | | | | | | |
| 7672 | 08921 | 07671 | 1 | | 0 | 0 | 0 | 0 | 0 | 0 | 0 | 0 | 0 | 0 | 0 | 0 | 0 | 0 | 0 | 0 | 0 | 0 | 0 | 0 | 0 | 0 | 0 | 1 | 0 | | | | | | |
| 7673 | 08922 | 07672 | 1 | | 0 | 0 | 0 | 0 | 0 | 0 | 0 | 0 | 0 | 0 | 0 | 0 | 0 | 0 | 0 | 0 | 0 | 0 | 0 | 0 | 0 | 0 | 0 | 1 | 0 | X69910 | 99.6 | 236 | 1 | 1840 | 2910 |
| 7674 | 08923 | 07673 | 1 | | 0 | 0 | 0 | 0 | 0 | 0 | 0 | 0 | 0 | 0 | 0 | 0 | 0 | 0 | 0 | 0 | 0 | 0 | 0 | 0 | 0 | 0 | 0 | 1 | 0 | | | | | | |
| 7675 | 08924 | 07674 | 1 | | 0 | 0 | 0 | 0 | 0 | 0 | 0 | 0 | 0 | 0 | 0 | 0 | 0 | 0 | 0 | 0 | 0 | 0 | 0 | 0 | 0 | 0 | 0 | 1 | 0 | | | | | | |
| 7676 | 08925 | 07675 | 1 | | 0 | 0 | 0 | 0 | 0 | 0 | 0 | 0 | 0 | 0 | 0 | 0 | 0 | 0 | 0 | 0 | 0 | 0 | 0 | 0 | 0 | 0 | 0 | 1 | 0 | | | | | | |
| 7677 | 08926 | 07676 | 1 | | 0 | 0 | 0 | 0 | 0 | 0 | 0 | 0 | 0 | 0 | 0 | 0 | 0 | 0 | 0 | 0 | 0 | 0 | 0 | 0 | 0 | 0 | 0 | 1 | 0 | | | | | | |
| 7678 | 08927 | 07677 | 1 | | 0 | 0 | 0 | 0 | 0 | 0 | 0 | 0 | 0 | 0 | 0 | 0 | 0 | 0 | 0 | 0 | 0 | 0 | 0 | 0 | 0 | 0 | 0 | 1 | 0 | | | | | | |
| 7679 | 08928 | 07678 | 1 | | 0 | 0 | 0 | 0 | 0 | 0 | 0 | 0 | 0 | 0 | 0 | 0 | 0 | 0 | 0 | 0 | 0 | 0 | 0 | 0 | 0 | 0 | 0 | 1 | 0 | | | | | | |
| 7680 | 08929 | 07679 | 1 | | 0 | 0 | 0 | 0 | 0 | 0 | 0 | 0 | 0 | 0 | 0 | 0 | 0 | 0 | 0 | 0 | 0 | 0 | 0 | 0 | 0 | 0 | 0 | 1 | 0 | | | | | | |
| 7681 | 08930 | 07680 | 1 | | 0 | 0 | 0 | 0 | 0 | 0 | 0 | 0 | 0 | 0 | 0 | 0 | 0 | 0 | 0 | 0 | 0 | 0 | 0 | 0 | 0 | 0 | 0 | 1 | 0 | | | | | | |
| 7682 | 08931 | 07681 | 1 | | 0 | 0 | 0 | 0 | 0 | 0 | 0 | 0 | 0 | 0 | 0 | 0 | 0 | 0 | 0 | 0 | 0 | 0 | 0 | 0 | 0 | 0 | 0 | 1 | 0 | | | | | | |
| 7683 | 08932 | 07682 | 1 | | 0 | 0 | 0 | 0 | 0 | 0 | 0 | 0 | 0 | 0 | 0 | 0 | 0 | 0 | 0 | 0 | 0 | 0 | 0 | 0 | 0 | 0 | 0 | 1 | 0 | | | | | | |
| 7684 | 08933 | 07683 | 1 | | 0 | 0 | 0 | 0 | 0 | 0 | 0 | 0 | 0 | 0 | 0 | 0 | 0 | 0 | 0 | 0 | 0 | 0 | 0 | 0 | 0 | 0 | 0 | 1 | 0 | | | | | | |
| 7685 | 08934 | 07684 | 1 | | 0 | 0 | 0 | 0 | 0 | 0 | 0 | 0 | 0 | 0 | 0 | 0 | 0 | 0 | 0 | 0 | 0 | 0 | 0 | 0 | 0 | 0 | 0 | 1 | 0 | | | | | | |
| 7686 | 08935 | 07685 | 1 | | 0 | 0 | 0 | 0 | 0 | 0 | 0 | 0 | 0 | 0 | 0 | 0 | 0 | 0 | 0 | 0 | 0 | 0 | 0 | 0 | 0 | 0 | 0 | 1 | 0 | | | | | | |
| 7687 | 08936 | 07686 | 1 | | 0 | 0 | 0 | 0 | 0 | 0 | 0 | 0 | 0 | 0 | 0 | 0 | 0 | 0 | 0 | 0 | 0 | 0 | 0 | 0 | 0 | 0 | 0 | 1 | 0 | | | | | | |
| 7688 | 08937 | 07687 | 1 | | 0 | 0 | 0 | 0 | 0 | 0 | 0 | 0 | 0 | 0 | 0 | 0 | 0 | 0 | 0 | 0 | 0 | 0 | 0 | 0 | 0 | 0 | 0 | 1 | 0 | | | | | | |
| 7689 | 08938 | 07688 | 2 | | 0 | 0 | 0 | 0 | 0 | 0 | 0 | 0 | 0 | 0 | 0 | 0 | 0 | 0 | 0 | 0 | 0 | 0 | 0 | 0 | 0 | 0 | 0 | 2 | 0 | | | | | | |

Table 214

| | A | B | C | E | G | I | K | M | O | Q | S | U | W | Y | AA | AC | AE | AG | AI | AK | AM | AO | AQ | AS | AU | AW | AY | BA | BC | BE | BF | BG | BH | BI | BK |
|---|---|---|---|---|---|---|---|---|---|---|---|---|---|---|---|---|---|---|---|---|---|---|---|---|---|---|---|---|---|---|---|---|---|---|---|
| 7690 | 08939 | 07689 | 1 | | 0 | 0 | 0 | 0 | 0 | 0 | 0 | 0 | 0 | 0 | 0 | 0 | 0 | 0 | 0 | 0 | 0 | 0 | 0 | 0 | 0 | 0 | 0 | 1 | 0 | | | | | | |
| 7691 | 08940 | 07690 | 1 | | 0 | 0 | 0 | 0 | 0 | 0 | 0 | 0 | 0 | 0 | 0 | 0 | 0 | 0 | 0 | 0 | 0 | 0 | 0 | 0 | 0 | 0 | 0 | 1 | 0 | | | | | | |
| 7692 | 08941 | 07691 | 1 | | 0 | 0 | 0 | 0 | 0 | 0 | 0 | 0 | 0 | 0 | 0 | 0 | 0 | 0 | 0 | 0 | 0 | 0 | 0 | 0 | 0 | 0 | 0 | 1 | 0 | | | | | | |
| 7693 | 08942 | 07692 | 1 | | 0 | 0 | 0 | 0 | 0 | 0 | 0 | 0 | 0 | 0 | 0 | 0 | 0 | 0 | 0 | 0 | 0 | 0 | 0 | 0 | 0 | 0 | 0 | 1 | 0 | | | | | | |
| 7694 | 08943 | 07693 | 1 | | 0 | 0 | 0 | 0 | 0 | 0 | 0 | 0 | 0 | 0 | 0 | 0 | 0 | 0 | 0 | 0 | 0 | 0 | 0 | 0 | 0 | 0 | 0 | 1 | 0 | | | | | | |
| 7695 | 08944 | 07694 | 1 | | 0 | 0 | 0 | 0 | 0 | 0 | 0 | 0 | 0 | 0 | 0 | 0 | 0 | 0 | 0 | 0 | 0 | 0 | 0 | 0 | 0 | 0 | 0 | 1 | 0 | | | | | | |
| 7696 | 08945 | 07695 | 1 | | 0 | 0 | 0 | 0 | 0 | 0 | 0 | 0 | 0 | 0 | 0 | 0 | 0 | 0 | 0 | 0 | 0 | 0 | 0 | 0 | 0 | 0 | 0 | 1 | 0 | | | | | | |
| 7697 | 08946 | 07696 | 1 | | 0 | 0 | 0 | 0 | 0 | 0 | 0 | 0 | 0 | 0 | 0 | 0 | 0 | 0 | 0 | 0 | 0 | 0 | 0 | 0 | 0 | 0 | 0 | 1 | 0 | | | | | | |
| 7698 | 08947 | 07697 | 1 | | 0 | 0 | 0 | 0 | 0 | 0 | 0 | 0 | 0 | 0 | 0 | 0 | 0 | 0 | 0 | 0 | 0 | 0 | 0 | 0 | 0 | 0 | 0 | 1 | 0 | | | | | | |
| 7699 | 08948 | 07698 | 1 | | 0 | 0 | 0 | 0 | 0 | 0 | 0 | 0 | 0 | 0 | 0 | 0 | 0 | 0 | 0 | 0 | 0 | 0 | 0 | 0 | 0 | 0 | 0 | 1 | 0 | | | | | | |
| 7700 | 08949 | 07699 | 1 | | 0 | 0 | 0 | 0 | 0 | 0 | 0 | 0 | 0 | 0 | 0 | 0 | 0 | 0 | 0 | 0 | 0 | 0 | 0 | 0 | 0 | 0 | 0 | 1 | 0 | | | | | | |
| 7701 | 08950 | 07700 | 1 | | 0 | 0 | 0 | 0 | 0 | 0 | 0 | 0 | 0 | 0 | 0 | 0 | 0 | 0 | 0 | 0 | 0 | 0 | 0 | 0 | 0 | 0 | 0 | 1 | 0 | | | | | | |
| 7702 | 08951 | 07701 | 1 | | 0 | 0 | 0 | 0 | 0 | 0 | 0 | 0 | 0 | 0 | 0 | 0 | 0 | 0 | 0 | 0 | 0 | 0 | 0 | 0 | 0 | 0 | 0 | 1 | 0 | | | | | | |
| 7703 | 08952 | 07702 | 1 | | 0 | 0 | 0 | 0 | 0 | 0 | 0 | 0 | 0 | 0 | 0 | 0 | 0 | 0 | 0 | 0 | 0 | 0 | 0 | 0 | 0 | 0 | 0 | 1 | 0 | K02581 | 98.7 | 78 | 1 | 1344 | 1421 |
| 7704 | 08953 | 07703 | 1 | | 0 | 0 | 0 | 0 | 0 | 0 | 0 | 0 | 0 | 0 | 0 | 0 | 0 | 0 | 0 | 0 | 0 | 0 | 0 | 0 | 0 | 0 | 0 | 1 | 0 | | | | | | |
| 7705 | 08954 | 07704 | 1 | | 0 | 0 | 0 | 0 | 0 | 0 | 0 | 0 | 0 | 0 | 0 | 0 | 0 | 0 | 0 | 0 | 0 | 0 | 0 | 0 | 0 | 0 | 0 | 1 | 0 | | | | | | |
| 7706 | 08955 | 07705 | 1 | | 0 | 0 | 0 | 0 | 0 | 0 | 0 | 0 | 0 | 0 | 0 | 0 | 0 | 0 | 0 | 0 | 0 | 0 | 0 | 0 | 0 | 0 | 0 | 1 | 0 | | | | | | |
| 7707 | 08956 | 07706 | 1 | | 0 | 0 | 0 | 0 | 0 | 0 | 0 | 0 | 0 | 0 | 0 | 0 | 0 | 0 | 0 | 0 | 0 | 0 | 0 | 0 | 0 | 0 | 0 | 1 | 0 | | | | | | |
| 7708 | 08957 | 07707 | 1 | | 0 | 0 | 0 | 0 | 0 | 0 | 0 | 0 | 0 | 0 | 0 | 0 | 0 | 0 | 0 | 0 | 0 | 0 | 0 | 0 | 0 | 0 | 0 | 1 | 0 | | | | | | |
| 7709 | 08958 | 07708 | 1 | | 0 | 0 | 0 | 0 | 0 | 0 | 0 | 0 | 0 | 0 | 0 | 0 | 0 | 0 | 0 | 0 | 0 | 0 | 0 | 0 | 0 | 0 | 0 | 1 | 0 | | | | | | |
| 7710 | 08959 | 07709 | 1 | | 0 | 0 | 0 | 0 | 0 | 0 | 0 | 0 | 0 | 0 | 0 | 0 | 0 | 0 | 0 | 0 | 0 | 0 | 0 | 0 | 0 | 0 | 0 | 1 | 0 | | | | | | |
| 7711 | 08960 | 07710 | 1 | | 0 | 0 | 0 | 0 | 0 | 0 | 0 | 0 | 0 | 0 | 0 | 0 | 0 | 0 | 0 | 0 | 0 | 0 | 0 | 0 | 0 | 0 | 0 | 1 | 0 | | | | | | |
| 7712 | 08961 | 07711 | 1 | | 0 | 0 | 0 | 0 | 0 | 0 | 0 | 0 | 0 | 0 | 0 | 0 | 0 | 0 | 0 | 0 | 0 | 0 | 0 | 0 | 0 | 0 | 0 | 1 | 0 | | | | | | |
| 7713 | 08962 | 07712 | 1 | | 0 | 0 | 0 | 0 | 0 | 0 | 0 | 0 | 0 | 0 | 0 | 0 | 0 | 0 | 0 | 0 | 0 | 0 | 0 | 0 | 0 | 0 | 0 | 1 | 0 | | | | | | |
| 7714 | 08963 | 07713 | 1 | | 0 | 0 | 0 | 0 | 0 | 0 | 0 | 0 | 0 | 0 | 0 | 0 | 0 | 0 | 0 | 0 | 0 | 0 | 0 | 0 | 0 | 0 | 0 | 1 | 0 | | | | | | |
| 7715 | 08964 | 07714 | 1 | | 0 | 0 | 0 | 0 | 0 | 0 | 0 | 0 | 0 | 0 | 0 | 0 | 0 | 0 | 0 | 0 | 0 | 0 | 0 | 0 | 0 | 0 | 0 | 1 | 0 | | | | | | |
| 7716 | 08965 | 07715 | 1 | | 0 | 0 | 0 | 0 | 0 | 0 | 0 | 0 | 0 | 0 | 0 | 0 | 0 | 0 | 0 | 0 | 0 | 0 | 0 | 0 | 0 | 0 | 0 | 1 | 0 | | | | | | |
| 7717 | 08966 | 07716 | 1 | | 0 | 0 | 0 | 0 | 0 | 0 | 0 | 0 | 0 | 0 | 0 | 0 | 0 | 0 | 0 | 0 | 0 | 0 | 0 | 0 | 0 | 0 | 0 | 1 | 0 | | | | | | |
| 7718 | 08967 | 07717 | 1 | | 0 | 0 | 0 | 0 | 0 | 0 | 0 | 0 | 0 | 0 | 0 | 0 | 0 | 0 | 0 | 0 | 0 | 0 | 0 | 0 | 0 | 0 | 0 | 1 | 0 | | | | | | |
| 7719 | 08968 | 07718 | 1 | | 0 | 0 | 0 | 0 | 0 | 0 | 0 | 0 | 0 | 0 | 0 | 0 | 0 | 0 | 0 | 0 | 0 | 0 | 0 | 0 | 0 | 0 | 0 | 1 | 0 | | | | | | |
| 7720 | 08969 | 07719 | 1 | | 0 | 0 | 0 | 0 | 0 | 0 | 0 | 0 | 0 | 0 | 0 | 0 | 0 | 0 | 0 | 0 | 0 | 0 | 0 | 0 | 0 | 0 | 0 | 1 | 0 | X70649 | 97.7 | 347 | 1 | 973 | 2399 |
| 7721 | 08970 | 07720 | 1 | | 0 | 0 | 0 | 0 | 0 | 0 | 0 | 0 | 0 | 0 | 0 | 0 | 0 | 0 | 0 | 0 | 0 | 0 | 0 | 0 | 0 | 0 | 0 | 1 | 0 | | | | | | |
| 7722 | 08971 | 07721 | 1 | | 0 | 0 | 0 | 0 | 0 | 0 | 0 | 0 | 0 | 0 | 0 | 0 | 0 | 0 | 0 | 0 | 0 | 0 | 0 | 0 | 0 | 0 | 0 | 1 | 0 | | | | | | |
| 7723 | 08972 | 07722 | 1 | | 0 | 0 | 0 | 0 | 0 | 0 | 0 | 0 | 0 | 0 | 0 | 0 | 0 | 0 | 0 | 0 | 0 | 0 | 0 | 0 | 0 | 0 | 0 | 1 | 0 | | | | | | |
| 7724 | 08973 | 07723 | 1 | | 0 | 0 | 0 | 0 | 0 | 0 | 0 | 0 | 0 | 0 | 0 | 0 | 0 | 0 | 0 | 0 | 0 | 0 | 0 | 0 | 0 | 0 | 0 | 1 | 0 | D13315 | 94 | 266 | 1 | 731 | 1993 |
| 7725 | 08974 | 07724 | 1 | | 0 | 0 | 0 | 0 | 0 | 0 | 0 | 0 | 0 | 0 | 0 | 0 | 0 | 0 | 0 | 0 | 0 | 0 | 0 | 0 | 0 | 0 | 0 | 1 | 0 | | | | | | |

Table 215

| | A | B | C | E | G | I | K | M | O | Q | S | U | W | Y | AA | AC | AE | AG | AI | AK | AM | AO | AQ | AS | AU | AW | AY | BA | BC | BE | BF | BG | BH | BI | BK |
|---|---|---|---|---|---|---|---|---|---|---|---|---|---|---|---|---|---|---|---|---|---|---|---|---|---|---|---|---|---|---|---|---|---|---|---|
| 7726 | 08975 | 07725 | 1 | | 0 | 0 | 0 | 0 | 0 | 0 | 0 | 0 | 0 | 0 | 0 | 0 | 0 | 0 | 0 | 0 | 0 | 0 | 0 | 0 | 0 | 0 | 0 | 1 | 0 | | | | | | |
| 7727 | 08976 | 07726 | 1 | | 0 | 0 | 0 | 0 | 0 | 0 | 0 | 0 | 0 | 0 | 0 | 0 | 0 | 0 | 0 | 0 | 0 | 0 | 0 | 0 | 0 | 0 | 0 | 1 | 0 | | | | | | |
| 7728 | 08977 | 07727 | 1 | | 0 | 0 | 0 | 0 | 0 | 0 | 0 | 0 | 0 | 0 | 0 | 0 | 0 | 0 | 0 | 0 | 0 | 0 | 0 | 0 | 0 | 0 | 0 | 1 | 0 | | | | | | |
| 7729 | 08978 | 07728 | 1 | | 0 | 0 | 0 | 0 | 0 | 0 | 0 | 0 | 0 | 0 | 0 | 0 | 0 | 0 | 0 | 0 | 0 | 0 | 0 | 0 | 0 | 0 | 0 | 1 | 0 | | | | | | |
| 7730 | 08979 | 07729 | 1 | | 0 | 0 | 0 | 0 | 0 | 0 | 0 | 0 | 0 | 0 | 0 | 0 | 0 | 0 | 0 | 0 | 0 | 0 | 0 | 0 | 0 | 0 | 0 | 1 | 0 | | | | | | |
| 7731 | 08980 | 07730 | 1 | | 0 | 0 | 0 | 0 | 0 | 0 | 0 | 0 | 0 | 0 | 0 | 0 | 0 | 0 | 0 | 0 | 0 | 0 | 0 | 0 | 0 | 0 | 0 | 1 | 0 | | | | | | |
| 7732 | 08981 | 07731 | 1 | | 0 | 0 | 0 | 0 | 0 | 0 | 0 | 0 | 0 | 0 | 0 | 0 | 0 | 0 | 0 | 0 | 0 | 0 | 0 | 0 | 0 | 0 | 0 | 1 | 0 | | | | | | |
| 7733 | 08982 | 07732 | 1 | | 0 | 0 | 0 | 0 | 0 | 0 | 0 | 0 | 0 | 0 | 0 | 0 | 0 | 0 | 0 | 0 | 0 | 0 | 0 | 0 | 0 | 0 | 0 | 1 | 0 | | | | | | |
| 7734 | 08983 | 07733 | 1 | | 0 | 0 | 0 | 0 | 0 | 0 | 0 | 0 | 0 | 0 | 0 | 0 | 0 | 0 | 0 | 0 | 0 | 0 | 0 | 0 | 0 | 0 | 0 | 1 | 0 | | | | | | |
| 7735 | 08984 | 07734 | 1 | | 0 | 0 | 0 | 0 | 0 | 0 | 0 | 0 | 0 | 0 | 0 | 0 | 0 | 0 | 0 | 0 | 0 | 0 | 0 | 0 | 0 | 0 | 0 | 1 | 0 | | | | | | |
| 7736 | 08985 | 07735 | 1 | | 0 | 0 | 0 | 0 | 0 | 0 | 0 | 0 | 0 | 0 | 0 | 0 | 0 | 0 | 0 | 0 | 0 | 0 | 0 | 0 | 0 | 0 | 0 | 1 | 0 | | | | | | |
| 7737 | 08986 | 07736 | 1 | | 0 | 0 | 0 | 0 | 0 | 0 | 0 | 0 | 0 | 0 | 0 | 0 | 0 | 0 | 0 | 0 | 0 | 0 | 0 | 0 | 0 | 0 | 0 | 1 | 0 | D14695 | 95.5 | 359 | 1 | 1316 | 1860 |
| 7738 | 08987 | 07737 | 1 | | 0 | 0 | 0 | 0 | 0 | 0 | 0 | 0 | 0 | 0 | 0 | 0 | 0 | 0 | 0 | 0 | 0 | 0 | 0 | 0 | 0 | 0 | 0 | 1 | 0 | | | | | | |
| 7739 | 08988 | 07738 | 2 | | 0 | 0 | 0 | 0 | 0 | 0 | 0 | 0 | 0 | 0 | 0 | 0 | 0 | 0 | 0 | 0 | 0 | 0 | 0 | 0 | 0 | 0 | 0 | 2 | 0 | | | | | | |
| 7740 | 08989 | 07739 | 1 | | 0 | 0 | 0 | 0 | 0 | 0 | 0 | 0 | 0 | 0 | 0 | 0 | 0 | 0 | 0 | 0 | 0 | 0 | 0 | 0 | 0 | 0 | 0 | 1 | 0 | | | | | | |
| 7741 | 08990 | 07740 | 1 | | 0 | 0 | 0 | 0 | 0 | 0 | 0 | 0 | 0 | 0 | 0 | 0 | 0 | 0 | 0 | 0 | 0 | 0 | 0 | 0 | 0 | 0 | 0 | 1 | 0 | | | | | | |
| 7742 | 08991 | 07741 | 1 | | 0 | 0 | 0 | 0 | 0 | 0 | 0 | 0 | 0 | 0 | 0 | 0 | 0 | 0 | 0 | 0 | 0 | 0 | 0 | 0 | 0 | 0 | 0 | 1 | 0 | | | | | | |
| 7743 | 08992 | 07742 | 1 | | 0 | 0 | 0 | 0 | 0 | 0 | 0 | 0 | 0 | 0 | 0 | 0 | 0 | 0 | 0 | 0 | 0 | 0 | 0 | 0 | 0 | 0 | 0 | 1 | 0 | | | | | | |
| 7744 | 08993 | 07743 | 1 | | 0 | 0 | 0 | 0 | 0 | 0 | 0 | 0 | 0 | 0 | 0 | 0 | 0 | 0 | 0 | 0 | 0 | 0 | 0 | 0 | 0 | 0 | 0 | 1 | 0 | | | | | | |
| 7745 | 08994 | 07744 | 1 | | 0 | 0 | 0 | 0 | 0 | 0 | 0 | 0 | 0 | 0 | 0 | 0 | 0 | 0 | 0 | 0 | 0 | 0 | 0 | 0 | 0 | 0 | 0 | 1 | 0 | | | | | | |
| 7746 | 08995 | 07745 | 1 | | 0 | 0 | 0 | 0 | 0 | 0 | 0 | 0 | 0 | 0 | 0 | 0 | 0 | 0 | 0 | 0 | 0 | 0 | 0 | 0 | 0 | 0 | 0 | 1 | 0 | | | | | | |
| 7747 | 08996 | 07746 | 1 | | 0 | 0 | 0 | 0 | 0 | 0 | 0 | 0 | 0 | 0 | 0 | 0 | 0 | 0 | 0 | 0 | 0 | 0 | 0 | 0 | 0 | 0 | 0 | 1 | 0 | | | | | | |
| 7748 | 08997 | 07747 | 1 | | 0 | 0 | 0 | 0 | 0 | 0 | 0 | 0 | 0 | 0 | 0 | 0 | 0 | 0 | 0 | 0 | 0 | 0 | 0 | 0 | 0 | 0 | 0 | 1 | 0 | X54941 | 98 | 152 | 1 | 566 | 717 |
| 7749 | 08998 | 07748 | 1 | | 0 | 0 | 0 | 0 | 0 | 0 | 0 | 0 | 0 | 0 | 0 | 0 | 0 | 0 | 0 | 0 | 0 | 0 | 0 | 0 | 0 | 0 | 0 | 1 | 0 | | | | | | |
| 7750 | 08999 | 07749 | 1 | | 0 | 0 | 0 | 0 | 0 | 0 | 0 | 0 | 0 | 0 | 0 | 0 | 0 | 0 | 0 | 0 | 0 | 0 | 0 | 0 | 0 | 0 | 0 | 1 | 0 | | | | | | |
| 7751 | 09000 | 07750 | 1 | | 0 | 0 | 0 | 0 | 0 | 0 | 0 | 0 | 0 | 0 | 0 | 0 | 0 | 0 | 0 | 0 | 0 | 0 | 0 | 0 | 0 | 0 | 0 | 1 | 0 | | | | | | |
| 7752 | 09001 | 07751 | 1 | | 0 | 0 | 0 | 0 | 0 | 0 | 0 | 0 | 0 | 0 | 0 | 0 | 0 | 0 | 0 | 0 | 0 | 0 | 0 | 0 | 0 | 0 | 0 | 1 | 0 | | | | | | |
| 7753 | 09002 | 07752 | 1 | | 0 | 0 | 0 | 0 | 0 | 0 | 0 | 0 | 0 | 0 | 0 | 0 | 0 | 0 | 0 | 0 | 0 | 0 | 0 | 0 | 0 | 0 | 0 | 1 | 0 | | | | | | |
| 7754 | 09003 | 07753 | 1 | | 0 | 0 | 0 | 0 | 0 | 0 | 0 | 0 | 0 | 0 | 0 | 0 | 0 | 0 | 0 | 0 | 0 | 0 | 0 | 0 | 0 | 0 | 0 | 1 | 0 | | | | | | |
| 7755 | 09004 | 07754 | 1 | | 0 | 0 | 0 | 0 | 0 | 0 | 0 | 0 | 0 | 0 | 0 | 0 | 0 | 0 | 0 | 0 | 0 | 0 | 0 | 0 | 0 | 0 | 0 | 1 | 0 | | | | | | |
| 7756 | 09005 | 07755 | 1 | | 0 | 0 | 0 | 0 | 0 | 0 | 0 | 0 | 0 | 0 | 0 | 0 | 0 | 0 | 0 | 0 | 0 | 0 | 0 | 0 | 0 | 0 | 0 | 1 | 0 | | | | | | |
| 7757 | 09006 | 07756 | 1 | | 0 | 0 | 0 | 0 | 0 | 0 | 0 | 0 | 0 | 0 | 0 | 0 | 0 | 0 | 0 | 0 | 0 | 0 | 0 | 0 | 0 | 0 | 0 | 1 | 0 | | | | | | |
| 7758 | 09007 | 07757 | 1 | | 0 | 0 | 0 | 0 | 0 | 0 | 0 | 0 | 0 | 0 | 0 | 0 | 0 | 0 | 0 | 0 | 0 | 0 | 0 | 0 | 0 | 0 | 0 | 1 | 0 | | | | | | |
| 7759 | 09008 | 07758 | 1 | | 0 | 0 | 0 | 0 | 0 | 0 | 0 | 0 | 0 | 0 | 0 | 0 | 0 | 0 | 0 | 0 | 0 | 0 | 0 | 0 | 0 | 0 | 0 | 1 | 0 | | | | | | |
| 7760 | 09009 | 07759 | 1 | | 0 | 0 | 0 | 0 | 0 | 0 | 0 | 0 | 0 | 0 | 0 | 0 | 0 | 0 | 0 | 0 | 0 | 0 | 0 | 0 | 0 | 0 | 0 | 1 | 0 | M25756 | 98.4 | 306 | 1 | 1824 | 2336 |
| 7761 | 09010 | 07760 | 1 | | 0 | 0 | 0 | 0 | 0 | 0 | 0 | 0 | 0 | 0 | 0 | 0 | 0 | 0 | 0 | 0 | 0 | 0 | 0 | 0 | 0 | 0 | 0 | 1 | 0 | | | | | | |

Table 216

| | A | B | C | E | G | I | K | M | O | Q | S | U | W | Y | AA | AC | AF | AG | AI | AK | AM | AO | AQ | AS | AU | AW | AY | BA | BC | BE | BF | BG | BH | BI | BK |
|---|---|---|---|---|---|---|---|---|---|---|---|---|---|---|---|---|---|---|---|---|---|---|---|---|---|---|---|---|---|---|---|---|---|---|---|
| 7762 | 09011 | 07761 | 1 | | 0 | 0 | 0 | 0 | 0 | 0 | 0 | 0 | 0 | 0 | 0 | 0 | 0 | 0 | 0 | 0 | 0 | 0 | 0 | 0 | 0 | 0 | 0 | 1 | 0 | | | | | | |
| 7763 | 09012 | 07762 | 1 | | 0 | 0 | 0 | 0 | 0 | 0 | 0 | 0 | 0 | 0 | 0 | 0 | 0 | 0 | 0 | 0 | 0 | 0 | 0 | 0 | 0 | 0 | 0 | 1 | 0 | | | | | | |
| 7764 | 09013 | 07763 | 1 | | 0 | 0 | 0 | 0 | 0 | 0 | 0 | 0 | 0 | 0 | 0 | 0 | 0 | 0 | 0 | 0 | 0 | 0 | 0 | 0 | 0 | 0 | 0 | 1 | 0 | | | | | | |
| 7765 | 09014 | 07764 | 2 | | 0 | 0 | 0 | 0 | 0 | 0 | 0 | 0 | 0 | 0 | 0 | 0 | 0 | 0 | 0 | 0 | 0 | 0 | 0 | 0 | 0 | 0 | 0 | 2 | 0 | | | | | | |
| 7766 | 09015 | 07765 | 1 | | 0 | 0 | 0 | 0 | 0 | 0 | 0 | 0 | 0 | 0 | 0 | 0 | 0 | 0 | 0 | 0 | 0 | 0 | 0 | 0 | 0 | 0 | 0 | 1 | 0 | | | | | | |
| 7767 | 09016 | 07766 | 1 | | 0 | 0 | 0 | 0 | 0 | 0 | 0 | 0 | 0 | 0 | 0 | 0 | 0 | 0 | 0 | 0 | 0 | 0 | 0 | 0 | 0 | 0 | 0 | 1 | 0 | | | | | | |
| 7768 | 09017 | 07767 | 1 | | 0 | 0 | 0 | 0 | 0 | 0 | 0 | 0 | 0 | 0 | 0 | 0 | 0 | 0 | 0 | 0 | 0 | 0 | 0 | 0 | 0 | 0 | 0 | 1 | 0 | | | | | | |
| 7769 | 09018 | 07768 | 1 | | 0 | 0 | 0 | 0 | 0 | 0 | 0 | 0 | 0 | 0 | 0 | 0 | 0 | 0 | 0 | 0 | 0 | 0 | 0 | 0 | 0 | 0 | 0 | 1 | 0 | | | | | | |
| 7770 | 09019 | 07769 | 1 | | 0 | 0 | 0 | 0 | 0 | 0 | 0 | 0 | 0 | 0 | 0 | 0 | 0 | 0 | 0 | 0 | 0 | 0 | 0 | 0 | 0 | 0 | 0 | 1 | 0 | J03191 | 98.6 | 69 | 4 | 725 | 793 |
| 7771 | 09020 | 07770 | 1 | | 0 | 0 | 0 | 0 | 0 | 0 | 0 | 0 | 0 | 0 | 0 | 0 | 0 | 0 | 0 | 0 | 0 | 0 | 0 | 0 | 0 | 0 | 0 | 1 | 0 | | | | | | |
| 7772 | 09021 | 07771 | 1 | | 0 | 0 | 0 | 0 | 0 | 0 | 0 | 0 | 0 | 0 | 0 | 0 | 0 | 0 | 0 | 0 | 0 | 0 | 0 | 0 | 0 | 0 | 0 | 1 | 0 | | | | | | |
| 7773 | 09022 | 07772 | 1 | | 0 | 0 | 0 | 0 | 0 | 0 | 0 | 0 | 0 | 0 | 0 | 0 | 0 | 0 | 0 | 0 | 0 | 0 | 0 | 0 | 0 | 0 | 0 | 1 | 0 | | | | | | |
| 7774 | 09023 | 07773 | 1 | | 0 | 0 | 0 | 0 | 0 | 0 | 0 | 0 | 0 | 0 | 0 | 0 | 0 | 0 | 0 | 0 | 0 | 0 | 0 | 0 | 0 | 0 | 0 | 1 | 0 | | | | | | |
| 7775 | 09024 | 07774 | 1 | | 0 | 0 | 0 | 0 | 0 | 0 | 0 | 0 | 0 | 0 | 0 | 0 | 0 | 0 | 0 | 0 | 0 | 0 | 0 | 0 | 0 | 0 | 0 | 1 | 0 | | | | | | |
| 7776 | 09025 | 07775 | 1 | | 0 | 0 | 0 | 0 | 0 | 0 | 0 | 0 | 0 | 0 | 0 | 0 | 0 | 0 | 0 | 0 | 0 | 0 | 0 | 0 | 0 | 0 | 0 | 1 | 0 | | | | | | |
| 7777 | 09026 | 07776 | 1 | | 0 | 0 | 0 | 0 | 0 | 0 | 0 | 0 | 0 | 0 | 0 | 0 | 0 | 0 | 0 | 0 | 0 | 0 | 0 | 0 | 0 | 0 | 0 | 1 | 0 | | | | | | |
| 7778 | 09027 | 07777 | 1 | | 0 | 0 | 0 | 0 | 0 | 0 | 0 | 0 | 0 | 0 | 0 | 0 | 0 | 0 | 0 | 0 | 0 | 0 | 0 | 0 | 0 | 0 | 0 | 0 | 1 | X57348 | 91.5 | 199 | 3 | 1219 | 1450 |
| 7779 | 09028 | 07778 | 1 | | 0 | 0 | 0 | 0 | 0 | 0 | 0 | 0 | 0 | 0 | 0 | 0 | 0 | 0 | 0 | 0 | 0 | 0 | 0 | 0 | 0 | 0 | 0 | 0 | 1 | | | | | | |
| 7780 | 09029 | 07779 | 1 | | 0 | 0 | 0 | 0 | 0 | 0 | 0 | 0 | 0 | 0 | 0 | 0 | 0 | 0 | 0 | 0 | 0 | 0 | 0 | 0 | 0 | 0 | 0 | 0 | 1 | | | | | | |
| 7781 | 09030 | 07780 | 1 | | 0 | 0 | 0 | 0 | 0 | 0 | 0 | 0 | 0 | 0 | 0 | 0 | 0 | 0 | 0 | 0 | 0 | 0 | 0 | 0 | 0 | 0 | 0 | 0 | 1 | | | | | | |
| 7782 | 09031 | 07781 | 1 | | 0 | 0 | 0 | 0 | 0 | 0 | 0 | 0 | 0 | 0 | 0 | 0 | 0 | 0 | 0 | 0 | 0 | 0 | 0 | 0 | 0 | 0 | 0 | 0 | 1 | M24069 | 95.1 | 265 | 56 | 989 | 1568 |
| 7783 | 09032 | 07782 | 1 | | 0 | 0 | 0 | 0 | 0 | 0 | 0 | 0 | 0 | 0 | 0 | 0 | 0 | 0 | 0 | 0 | 0 | 0 | 0 | 0 | 0 | 0 | 0 | 0 | 1 | | | | | | |
| 7784 | 09033 | 07783 | 1 | | 0 | 0 | 0 | 0 | 0 | 0 | 0 | 0 | 0 | 0 | 0 | 0 | 0 | 0 | 0 | 0 | 0 | 0 | 0 | 0 | 0 | 0 | 0 | 0 | 1 | | | | | | |
| 7785 | 09034 | 07784 | 1 | | 0 | 0 | 0 | 0 | 0 | 0 | 0 | 0 | 0 | 0 | 0 | 0 | 0 | 0 | 0 | 0 | 0 | 0 | 0 | 0 | 0 | 0 | 0 | 0 | 1 | | | | | | |
| 7786 | 09035 | 07785 | 1 | | 0 | 0 | 0 | 0 | 0 | 0 | 0 | 0 | 0 | 0 | 0 | 0 | 0 | 0 | 0 | 0 | 0 | 0 | 0 | 0 | 0 | 0 | 0 | 0 | 1 | | | | | | |
| 7787 | 09036 | 07786 | 2 | | 0 | 0 | 0 | 0 | 0 | 0 | 0 | 0 | 0 | 0 | 0 | 0 | 0 | 0 | 0 | 0 | 0 | 0 | 0 | 0 | 0 | 0 | 0 | 0 | 2 | | | | | | |
| 7788 | 09037 | 07787 | 1 | | 0 | 0 | 0 | 0 | 0 | 0 | 0 | 0 | 0 | 0 | 0 | 0 | 0 | 0 | 0 | 0 | 0 | 0 | 0 | 0 | 0 | 0 | 0 | 0 | 1 | | | | | | |
| 7789 | 09038 | 07788 | 1 | | 0 | 0 | 0 | 0 | 0 | 0 | 0 | 0 | 0 | 0 | 0 | 0 | 0 | 0 | 0 | 0 | 0 | 0 | 0 | 0 | 0 | 0 | 0 | 0 | 1 | | | | | | |
| 7790 | 09039 | 07789 | 2 | | 0 | 0 | 0 | 0 | 0 | 0 | 0 | 0 | 0 | 0 | 0 | 0 | 0 | 0 | 0 | 0 | 0 | 0 | 0 | 0 | 0 | 0 | 0 | 0 | 2 | M21302 | 93 | 256 | 1 | 423 | 683 |
| 7791 | 09040 | 07790 | 2 | | 0 | 0 | 0 | 0 | 0 | 0 | 0 | 0 | 0 | 0 | 0 | 0 | 0 | 0 | 0 | 0 | 0 | 0 | 0 | 0 | 0 | 0 | 0 | 0 | 2 | X66029 | 100 | 123 | 1 | 1555 | 1681 |
| 7792 | 09041 | 07791 | 1 | | 0 | 0 | 0 | 0 | 0 | 0 | 0 | 0 | 0 | 0 | 0 | 0 | 0 | 0 | 0 | 0 | 0 | 0 | 0 | 0 | 0 | 0 | 0 | 0 | 1 | | | | | | |
| 7793 | 09042 | 07792 | 2 | | 0 | 0 | 0 | 0 | 0 | 0 | 0 | 0 | 0 | 0 | 0 | 0 | 0 | 0 | 0 | 0 | 0 | 0 | 0 | 0 | 0 | 0 | 0 | 0 | 2 | | | | | | |
| 7794 | 09043 | 07793 | 1 | | 0 | 0 | 0 | 0 | 0 | 0 | 0 | 0 | 0 | 0 | 0 | 0 | 0 | 0 | 0 | 0 | 0 | 0 | 0 | 0 | 0 | 0 | 0 | 0 | 1 | | | | | | |
| 7795 | 09044 | 07794 | 1 | | 0 | 0 | 0 | 0 | 0 | 0 | 0 | 0 | 0 | 0 | 0 | 0 | 0 | 0 | 0 | 0 | 0 | 0 | 0 | 0 | 0 | 0 | 0 | 0 | 1 | | | | | | |
| 7796 | 09045 | 07795 | 1 | | 0 | 0 | 0 | 0 | 0 | 0 | 0 | 0 | 0 | 0 | 0 | 0 | 0 | 0 | 0 | 0 | 0 | 0 | 0 | 0 | 0 | 0 | 0 | 0 | 1 | | | | | | |
| 7797 | 09046 | 07796 | 1 | | 0 | 0 | 0 | 0 | 0 | 0 | 0 | 0 | 0 | 0 | 0 | 0 | 0 | 0 | 0 | 0 | 0 | 0 | 0 | 0 | 0 | 0 | 0 | 0 | 1 | | | | | | |

Table 217

| | A | B | C | E | G | I | K | M | O | Q | S | U | W | Y | AA | AC | AE | AG | AI | AK | AM | AO | AQ | AS | AU | AW | AY | BA | BC | BE | BF | BG | BH | BI | BK |
|---|---|---|---|---|---|---|---|---|---|---|---|---|---|---|---|---|---|---|---|---|---|---|---|---|---|---|---|---|---|---|---|---|---|---|---|
| 7798 | 09047 | 07797 | 1 | | 0 | 0 | 0 | 0 | 0 | 0 | 0 | 0 | 0 | 0 | 0 | 0 | 0 | 0 | 0 | 0 | 0 | 0 | 0 | 0 | 0 | 0 | 0 | 0 | 1 | M60047 | 95 | 261 | 1 | 103 | 1163 |
| 7799 | 09048 | 07798 | 1 | | 0 | 0 | 0 | 0 | 0 | 0 | 0 | 0 | 0 | 0 | 0 | 0 | 0 | 0 | 0 | 0 | 0 | 0 | 0 | 0 | 0 | 0 | 0 | 0 | 1 | | | | | | |
| 7800 | 09049 | 07799 | 1 | | 0 | 0 | 0 | 0 | 0 | 0 | 0 | 0 | 0 | 0 | 0 | 0 | 0 | 0 | 0 | 0 | 0 | 0 | 0 | 0 | 0 | 0 | 0 | 0 | 1 | | | | | | |
| 7801 | 09050 | 07800 | 3 | | 0 | 0 | 0 | 0 | 0 | 0 | 0 | 0 | 0 | 0 | 0 | 0 | 0 | 0 | 0 | 0 | 0 | 0 | 0 | 0 | 0 | 0 | 0 | 0 | 3 | | | | | | |
| 7802 | 09051 | 07801 | 8 | | 0 | 0 | 0 | 0 | 0 | 0 | 0 | 0 | 0 | 0 | 0 | 0 | 0 | 0 | 0 | 0 | 0 | 0 | 0 | 0 | 0 | 0 | 0 | 0 | 8 | J00269 | 94.6 | 351 | 1 | 1268 | 1617 |
| 7803 | 09052 | 07802 | 1 | | 0 | 0 | 0 | 0 | 0 | 0 | 0 | 0 | 0 | 0 | 0 | 0 | 0 | 0 | 0 | 0 | 0 | 0 | 0 | 0 | 0 | 0 | 0 | 0 | 1 | | | | | | |
| 7804 | 09053 | 07803 | 1 | | 0 | 0 | 0 | 0 | 0 | 0 | 0 | 0 | 0 | 0 | 0 | 0 | 0 | 0 | 0 | 0 | 0 | 0 | 0 | 0 | 0 | 0 | 0 | 0 | 1 | | | | | | |
| 7805 | 09054 | 07804 | 1 | | 0 | 0 | 0 | 0 | 0 | 0 | 0 | 0 | 0 | 0 | 0 | 0 | 0 | 0 | 0 | 0 | 0 | 0 | 0 | 0 | 0 | 0 | 0 | 0 | 1 | | | | | | |
| 7806 | 09055 | 07805 | 1 | | 0 | 0 | 0 | 0 | 0 | 0 | 0 | 0 | 0 | 0 | 0 | 0 | 0 | 0 | 0 | 0 | 0 | 0 | 0 | 0 | 0 | 0 | 0 | 0 | 1 | | | | | | |
| 7807 | 09056 | 07806 | 1 | | 0 | 0 | 0 | 0 | 0 | 0 | 0 | 0 | 0 | 0 | 0 | 0 | 0 | 0 | 0 | 0 | 0 | 0 | 0 | 0 | 0 | 0 | 0 | 0 | 1 | M93150 | 98.8 | 169 | 1 | 1576 | 2360 |
| 7808 | 09057 | 07807 | 1 | | 0 | 0 | 0 | 0 | 0 | 0 | 0 | 0 | 0 | 0 | 0 | 0 | 0 | 0 | 0 | 0 | 0 | 0 | 0 | 0 | 0 | 0 | 0 | 0 | 1 | | | | | | |
| 7809 | 09058 | 07808 | 1 | | 0 | 0 | 0 | 0 | 0 | 0 | 0 | 0 | 0 | 0 | 0 | 0 | 0 | 0 | 0 | 0 | 0 | 0 | 0 | 0 | 0 | 0 | 0 | 0 | 1 | | | | | | |
| 7810 | 09059 | 07809 | 1 | | 0 | 0 | 0 | 0 | 0 | 0 | 0 | 0 | 0 | 0 | 0 | 0 | 0 | 0 | 0 | 0 | 0 | 0 | 0 | 0 | 0 | 0 | 0 | 0 | 1 | | | | | | |
| 7811 | 09060 | 07810 | 1 | | 0 | 0 | 0 | 0 | 0 | 0 | 0 | 0 | 0 | 0 | 0 | 0 | 0 | 0 | 0 | 0 | 0 | 0 | 0 | 0 | 0 | 0 | 0 | 0 | 1 | | | | | | |
| 7812 | 09061 | 07811 | 1 | | 0 | 0 | 0 | 0 | 0 | 0 | 0 | 0 | 0 | 0 | 0 | 0 | 0 | 0 | 0 | 0 | 0 | 0 | 0 | 0 | 0 | 0 | 0 | 0 | 1 | | | | | | |
| 7813 | 09062 | 07812 | 1 | | 0 | 0 | 0 | 0 | 0 | 0 | 0 | 0 | 0 | 0 | 0 | 0 | 0 | 0 | 0 | 0 | 0 | 0 | 0 | 0 | 0 | 0 | 0 | 0 | 1 | X04790 | 98.5 | 326 | 1 | 2116 | 2458 |
| 7814 | 09063 | 07813 | 1 | | 0 | 0 | 0 | 0 | 0 | 0 | 0 | 0 | 0 | 0 | 0 | 0 | 0 | 0 | 0 | 0 | 0 | 0 | 0 | 0 | 0 | 0 | 0 | 0 | 1 | | | | | | |
| 7815 | 09064 | 07814 | 1 | | 0 | 0 | 0 | 0 | 0 | 0 | 0 | 0 | 0 | 0 | 0 | 0 | 0 | 0 | 0 | 0 | 0 | 0 | 0 | 0 | 0 | 0 | 0 | 0 | 1 | | | | | | |
| 7816 | 09065 | 07815 | 1 | | 0 | 0 | 0 | 0 | 0 | 0 | 0 | 0 | 0 | 0 | 0 | 0 | 0 | 0 | 0 | 0 | 0 | 0 | 0 | 0 | 0 | 0 | 0 | 0 | 1 | | | | | | |
| 7817 | 09066 | 07816 | 1 | | 0 | 0 | 0 | 0 | 0 | 0 | 0 | 0 | 0 | 0 | 0 | 0 | 0 | 0 | 0 | 0 | 0 | 0 | 0 | 0 | 0 | 0 | 0 | 0 | 1 | | | | | | |
| 7818 | 09067 | 07817 | 1 | | 0 | 0 | 0 | 0 | 0 | 0 | 0 | 0 | 0 | 0 | 0 | 0 | 0 | 0 | 0 | 0 | 0 | 0 | 0 | 0 | 0 | 0 | 0 | 0 | 1 | | | | | | |
| 7819 | 09068 | 07818 | 1 | | 0 | 0 | 0 | 0 | 0 | 0 | 0 | 0 | 0 | 0 | 0 | 0 | 0 | 0 | 0 | 0 | 0 | 0 | 0 | 0 | 0 | 0 | 0 | 0 | 1 | | | | | | |
| 7820 | 09069 | 07819 | 1 | | 0 | 0 | 0 | 0 | 0 | 0 | 0 | 0 | 0 | 0 | 0 | 0 | 0 | 0 | 0 | 0 | 0 | 0 | 0 | 0 | 0 | 0 | 0 | 0 | 1 | | | | | | |
| 7821 | 09070 | 07820 | 1 | | 0 | 0 | 0 | 0 | 0 | 0 | 0 | 0 | 0 | 0 | 0 | 0 | 0 | 0 | 0 | 0 | 0 | 0 | 0 | 0 | 0 | 0 | 0 | 0 | 1 | | | | | | |
| 7822 | 09071 | 07821 | 1 | | 0 | 0 | 0 | 0 | 0 | 0 | 0 | 0 | 0 | 0 | 0 | 0 | 0 | 0 | 0 | 0 | 0 | 0 | 0 | 0 | 0 | 0 | 0 | 0 | 1 | | | | | | |
| 7823 | 09072 | 07822 | 1 | | 0 | 0 | 0 | 0 | 0 | 0 | 0 | 0 | 0 | 0 | 0 | 0 | 0 | 0 | 0 | 0 | 0 | 0 | 0 | 0 | 0 | 0 | 0 | 0 | 1 | | | | | | |
| 7824 | 09073 | 07823 | 3 | | 0 | 0 | 0 | 0 | 0 | 0 | 0 | 0 | 0 | 0 | 0 | 0 | 0 | 0 | 0 | 0 | 0 | 0 | 0 | 0 | 0 | 0 | 0 | 0 | 3 | | | | | | |
| 7825 | 09074 | 07824 | 1 | | 0 | 0 | 0 | 0 | 0 | 0 | 0 | 0 | 0 | 0 | 0 | 0 | 0 | 0 | 0 | 0 | 0 | 0 | 0 | 0 | 0 | 0 | 0 | 0 | 1 | | | | | | |
| 7826 | 09075 | 07825 | 2 | | 0 | 0 | 0 | 0 | 0 | 0 | 0 | 0 | 0 | 0 | 0 | 0 | 0 | 0 | 0 | 0 | 0 | 0 | 0 | 0 | 0 | 0 | 0 | 0 | 2 | | | | | | |
| 7827 | 09076 | 07826 | 1 | | 0 | 0 | 0 | 0 | 0 | 0 | 0 | 0 | 0 | 0 | 0 | 0 | 0 | 0 | 0 | 0 | 0 | 0 | 0 | 0 | 0 | 0 | 0 | 0 | 1 | | | | | | |
| 7828 | 09077 | 07827 | 1 | | 0 | 0 | 0 | 0 | 0 | 0 | 0 | 0 | 0 | 0 | 0 | 0 | 0 | 0 | 0 | 0 | 0 | 0 | 0 | 0 | 0 | 0 | 0 | 0 | 1 | X04098 | 93.2 | 325 | 1 | 1551 | 1918 |
| 7829 | 09078 | 07828 | 1 | | 0 | 0 | 0 | 0 | 0 | 0 | 0 | 0 | 0 | 0 | 0 | 0 | 0 | 0 | 0 | 0 | 0 | 0 | 0 | 0 | 0 | 0 | 0 | 0 | 1 | D13638 | 91 | 78 | 1 | 3793 | 5167 |
| 7830 | 09079 | 07829 | 1 | | 0 | 0 | 0 | 0 | 0 | 0 | 0 | 0 | 0 | 0 | 0 | 0 | 0 | 0 | 0 | 0 | 0 | 0 | 0 | 0 | 0 | 0 | 0 | 0 | 1 | | | | | | |
| 7831 | 09080 | 07830 | 2 | | 0 | 0 | 0 | 0 | 0 | 0 | 0 | 0 | 0 | 0 | 0 | 0 | 0 | 0 | 0 | 0 | 0 | 0 | 0 | 0 | 0 | 0 | 0 | 0 | 2 | J04605 | 97.6 | 123 | 1 | 1627 | 1888 |
| 7832 | 09081 | 07831 | 1 | | 0 | 0 | 0 | 0 | 0 | 0 | 0 | 0 | 0 | 0 | 0 | 0 | 0 | 0 | 0 | 0 | 0 | 0 | 0 | 0 | 0 | 0 | 0 | 0 | 1 | X02902 | 98.2 | 337 | 1 | 584 | 1181 |
| 7833 | 09082 | 07832 | 1 | | 0 | 0 | 0 | 0 | 0 | 0 | 0 | 0 | 0 | 0 | 0 | 0 | 0 | 0 | 0 | 0 | 0 | 0 | 0 | 0 | 0 | 0 | 0 | 0 | 1 | | | | | | |

Table 218

| | A | B | C | E | G | I | K | M | O | Q | S | U | W | Y | AA | AC | AE | AG | AI | AK | AM | AO | AQ | AS | AU | AW | AY | BA | BC | BE | BF | BG | BH | BI | BK |
|---|---|---|---|---|---|---|---|---|---|---|---|---|---|---|---|---|---|---|---|---|---|---|---|---|---|---|---|---|---|---|---|---|---|---|---|
| 7834 | 09083 | 07833 | 1 | | 0 | 0 | 0 | 0 | 0 | 0 | 0 | 0 | 0 | 0 | 0 | 0 | 0 | 0 | 0 | 0 | 0 | 0 | 0 | 0 | 0 | 0 | 0 | 0 | 1 | | | | | | |
| 7835 | 09084 | 07834 | 1 | | 0 | 0 | 0 | 0 | 0 | 0 | 0 | 0 | 0 | 0 | 0 | 0 | 0 | 0 | 0 | 0 | 0 | 0 | 0 | 0 | 0 | 0 | 0 | 0 | 1 | | | | | | |
| 7836 | 09085 | 07835 | 1 | | 0 | 0 | 0 | 0 | 0 | 0 | 0 | 0 | 0 | 0 | 0 | 0 | 0 | 0 | 0 | 0 | 0 | 0 | 0 | 0 | 0 | 0 | 0 | 0 | 1 | X56976 | 92 | 287 | 1 | 3066 | 3419 |
| 7837 | 09086 | 07836 | 1 | | 0 | 0 | 0 | 0 | 0 | 0 | 0 | 0 | 0 | 0 | 0 | 0 | 0 | 0 | 0 | 0 | 0 | 0 | 0 | 0 | 0 | 0 | 0 | 0 | 1 | | | | | | |
| 7838 | 09087 | 07837 | 1 | | 0 | 0 | 0 | 0 | 0 | 0 | 0 | 0 | 0 | 0 | 0 | 0 | 0 | 0 | 0 | 0 | 0 | 0 | 0 | 0 | 0 | 0 | 0 | 0 | 1 | | | | | | |

Table 219

SEQUENCE LISTING

(1) GENERAL INFORMATION:

(i) APPLICANT:
(A) NAME: CHUGAI PHARMACEUTICAL CO., LTD.
(B) STREET: 41-8, Takada 3-chrome, Toshima-ku
(C) CITY: Tokyo
(E) COUNTRY: JAPAN
(F) ZIP: 171

(ii) TITLE OF INVENTION: GENE SIGNATURE

(iii) NUMBER OF SEQUENCES: 7848

(iv) COMPUTER READABLE FORM:
(A) MEDIUM TYPE: Diskette, 3.5 in., DS, 1.44 MB
(B) COMPUTER: IBM PC compatible
(C) OPERATING SYSTEM: PC-DOS/ MS-DOS
(D) SOFTWARE: MS-DOS

(v) CURRENT APPLICATION DATA
(A) APPLICATION NUMBER: EP 95900295.7

(vi) PRIOR APPLICATION DATA
(A) APPLICATION NUMBER: PCT/JP94/01916
(B) FILING DATE: 11. November 1994

SEQ ID NO:1
SEQUENCE LENGTH:704
SEQUENCE TYPE:nucleic acid
TOPOLOGY:linear
CLONE:HUMGS00001
SEQUENCE DESCRIPTION:

```
GATCTTCAAA CAAGCATCAG CGTTTTCCAG GGCTTCCCAG AGGTCTGTGC GACTAGCCCG  60
TGTCTATCAA AAGTTATTAG AGAGGATGAA GCATTAGCTT GAAGCACTAC AGGAGGAATG 120
CACCACGGCA GCTCTCCGCC AATTTCTCTC AGATTTCCAC AGAGACTGTT TGAATGTTTT 180
CAAAACCAAG TATCACACTT TAATGTACAT GGGCCGCACC ATAATGAGAT GTGAGCCTTG 240
TGCATGTGGG GGAGGAGGGA GAGAGATGTA CTTTTTAAAT CATGTTCCCC CTAAACATGG 300
CTGTTAACCC ACTTGCATGC AGAAACTTGG GATGTCACTT GCCTGACATT CACTTTCCAG 360
GAGAGGACCC TATCCCCAAA TGTGGAATTG ACTTGCCTAT GGCCAAGGTC CCTTGGNAAA 420
GGGAGCTTCA GTATTTGTGG GGGCNTCATA AAACCATGGN TTCAAGNCAA TCCAGCCTCA 480
TNGGGNNGGT CCTGGGNACA GTTTTTTGGT AAAGGCCCTT GGCCCAGNTG GGGGGAATGG 540
GCCTCCTTTT TAAGNTTTGG GNTGGAATNG TCTNGCAAAT TGGGGCTCCC ATTTCNCGGG 600
GGTTTGGGGG TTTTTTNGGG CCTTNCCNGG NNGGAAGGGN TGGGTTTGGG GGNTNGGTTN 660
CCNTTGGGNG GGCCTGGGGN TTTGATTTNA CCCGGGNCTT NGGN                 704
```

SEQ ID NO:2
SEQUENCE LENGTH:659
SEQUENCE TYPE:nucleic acid
TOPOLOGY:linear
CLONE:HUMGS00002
SEQUENCE DESCRIPTION:

```
GATCTTTAAA ATACACACTC AAATCAAGAA ACTTAAGGTT ACCTTTNTTC CCAAATTTCA  60
TACCTATCAT CTTAAGTAGG GACTTCTGTC TTCACAACAN ATTATNACCT TACAGAAGTT 120
TGAATTATCC GGTCGGGTTT TATTGTTTAA AATCATTTCT GCATCAGCTG CTGAAACAAC 180
AAATAGGAAT TGTTTTTATG GAGGCTTTGC ATAGATTCCC TGAGCAGGAT TTTAATCTTT 240
TNCTAACTGG ACTGGTTCAA ATGTTGTNCT CTTCTTTAAA GGGATGGCAA GATGTGGGCA 300
GTGATGTCAC TTAGGGCAGG GACAGGATAA GAGGGNTTAG GGAGAGAAGA TAGCAGGGCA 360
TGGCTGGGAA CCCAAGTCCA AGCATACCAA CACGGAGCAG GCTACTGTCA AGCTCCCCTC 420
GGAGGCGGNG CTGGTTCACA GCCAGCTGGC ACCAGNTTTT NTNGNGGAAG NCTTTTTCAA 480
ACAGTCTCAG GNAATCCAAT NTGCAAAGAC TTGCTTTNAG NAAAACCCAG NAGTTGAAAG 540
GCTCCCAAGN ATTTTAAGGG NACTTNCCAA AACGGGGCCC CNGGNNCCTT TTGGGTTTNG 600
GGGNTCAAAA CCCCGGAGGG GTTTGGGAAG NTTTTAATTG GNTTTAAAAN ATNNNTNTN  659
```

SEQ ID NO:3
SEQUENCE LENGTH:625
SEQUENCE TYPE:nucleic acid
TOPOLOGY:linear
CLONE:HUMGS00003
SEQUENCE DESCRIPTION:

```
GATCTAACTG GGTACCTGAG ATATTTNACA GCTGGACCTA GTTTCACAAT CTGTTGTCTC  60
```

CAGCTCTGCA TATGTCTGGC CAGGGGGCTT CTAGGAAGTA GGTTTCATCT ATCAAATGTC 120
TCCTCTGACT TCCTTTTGAA ACTTACTGCT CTTCTGTTTT ATTTTGTTTT GTTTGAAGCT 180
CAGAGGGAGA TGGGCAATTG ACAGGGATGC AATCCAGGGT GGGATTTCTT GAGGAAGTTA 240
CAAATAAGCT TGTTACAACA TCAAGATAGA TGGAATTGGA AGGATGCTAC CAGGAGAGTA 300
CTTACATAGT GCTCAGGAGT TTCTCTTCTT AAAATGTTTA CTGCTGAAAG ATGAGCAGGA 360
CCAGGGCGTT ATAGGCAGAG CCCTAGCCGA GAAACCTGCT GGCCTCTGCC TGTTTTCATT 420
TCCCACTTTT GGTTGTTGTG GCATTACTTT CAGAATTTGC ACTTTCCTGC TTGTCATGAC 480
TTTTTGGCA CACTTGCCAT GACGGGTGTT TCTGNGAACC ATGGAAGTTT TGCGGTAGTG 540
CCTCCAGGGG CAGGGGGNAA GGAGGNGGTG TANCTGCATT TNGTNCAAAT AAATCCNGCC 600
TATTGTTAAT NAACCAGTCT TTTGN 625

SEQ ID NO:4
SEQUENCE LENGTH:256
SEQUENCE TYPE:nucleic acid
TOPOLOGY:linear
CLONE:HUMGS00004
SEQUENCE DESCRIPTION:
GATCTGCCAG GCTGGGGTGT TTTCGGTATC TGCTGTTCAC AGGTCTCCAC TGTAATCCGA 60
ATACTTTGCC AGTGCACTAA TCTCTTTGGA GATAAAATTC ATTAGTGTGT TACTAAATGT 120
NAATTTTNTT TTGCGGAAAA TACAGTACCG TGTCTGANTT AATTATTAAT ATTNAAAATA 180
CTTCATTCCT TAACTCTCCC TCATTTGCTT TGCCCACAGC CTATTCAGTT CCTTTGTTTG 240
GCAGGNTTCT GCAAAA 256

SEQ ID NO:5
SEQUENCE LENGTH:616
SEQUENCE TYPE:nucleic acid
TOPOLOGY:linear
CLONE:HUMGS00005
SEQUENCE DESCRIPTION:
GATCAAGCTC AAGAATAAGC TGAAATATGG CCAGACTATC AGGCCCATTT NTCTCCCCTG 60
CACCGAGGGA ACAACTCGAG CTTTGAGGCT TCCTCCAACT ACCACTTGCC AGCAACAAAA 120
GGAAGAGCTG CTCCCTGCAC AGGATATCAA AGCTCTGTTT GTGTCTGAGG AGGAGAAAAA 180
GCTGACTCGG AAGGAGGTCT ACATCAAGAA TGGGGATAAG AAAGGCAGCT GTGAGAGAGA 240
TGCTCAATAT GCCCCAGGCT ATGACAAAGT CAAGGACATC TCAGAGGTGG TCACCCCTCG 300
GTTCCTTTGT ACTGGAGGAG TGAGTCCCTA TGCTGACCCC AATACTTGCA GAGGTGATTC 360
TGGCGGCCCC TTGATAGTTC ACAAGAGAAG TCGTTTCATT CAAAGTTGGT GTAATCAGCT 420
GGGGGAGTAA GTGGGATGTN TGCAAAAACC AGAAGGCGGC AAAAGCAGGT ACCTGNTTCA 480
NGGCCCGAAC TTTCACATCA NCTNTTTCAA GGTNTTNCCT GGNTGAGGNG GAACNTCCAN 540
GTTGGGGGTT TTGGGTTTTT TTAAGGGGTT CNTGTTNGCA AGGGGGTTGG GNTTNNTTTA 600
NNCCTGTTNN GNACCN 616

SEQ ID NO:6
SEQUENCE LENGTH:615
SEQUENCE TYPE:nucleic acid
TOPOLOGY:linear

CLONE:HUMGS00006
SEQUENCE DESCRIPTION:
GATCTCTGCT ATTTTANCCC CCCAAATAAG TTATTTGTCC TTTAAGGTTG GTTACNNATA 60
ATACCCCTCA GTAAGATTCC AGTATTAATT TCTGGGCAGT TTGTTCTCTG TATACAATTG 120
CAAATGATAA GCATTTTTGT GAGTGACCAC CTTTGCAATA TGTTTGTTAA TTNTTCATGT 180
TGGGTTCTTT CTGAAATGTA CATCTTTACA TAAAAACCTC ACATTCTACT TGATTTACAC 240
TTCCTAGTCT ACATTACATG TGGTTGAAGG TTTTATACAT TCTATATGCT TTTACTAAAT 300
ATACAAGATT TACTACTAGA AATTTGGAGA AAGAACACTA ACACATGTAC TTGTGATTTG 360
TTCATGTTAT ATTAAAACCT TGAGATTTGT GTATTTATGT AGGGGGGGGT ATTGNCCAGG 420
NCTGNTGGTT TTTTGCTCCN TGGGGCTATT NTAATAAANC NGGGGTATNG GGTTGNTGGC 480
CTNGGTTTGG GCCTAAANTT GGATATATNT GGGGTTCCTT NGNTTTTACC AAAATNGNTT 540
TTGGTTGGTA GGGTTNTTAT TGGACCCNNT CCTGGGCCTG GGATAATNTG GCNGNTTCCC 600
NGGANAAAAN NCCCN 615

SEQ ID NO:7
SEQUENCE LENGTH:608
SEQUENCE TYPE:nucleic acid
TOPOLOGY:linear
CLONE:HUMGS00007
SEQUENCE DESCRIPTION:
GATCAAACCA AAGGAAAAGT GTTGCTAGAG AAAATTGGGG AAAAGGTGAA AAAGAAAAAA 60
TGGTAGTAAT TGAGCAGAAA AAAATTAATT TATATATGTA TTGATTGGCA ACCAGATTTA 120
TCTAAGTAGA ACTGAATTGG CTAGGAAAAA AGAAAAACTG CATGTTAATC ATTTTCCTAA 180
GCTGTCCTTT TGAGGCTTAG TCAGTTTATT GGGAAAATGT TTAGGATTAT TCCTTGCTAT 240
TAGTACTCAT TTTATGTATG TTACCCTTCA GTAAGTTCTC CCCATTTTAG TTTTCTAGGC 300
TGAAAGGATT CTTTTCTACA TTATACATGT GTGTTGTCAT ATTTGGCTTT TGCTATATAC 360
TTTACCTTCA TTGTTAAATT TTTGTATTGT ATAGTTNCTT TGGNGGTATC TTAAAACCCT 420
ATTTTTGAAA ACCAACCTTG GCTTTGNTAA NCATTTGGGC CGCTTGGGTA NGTCCGGACC 480
TNNCCTTTNC CCCNAGGGCC TGTCAGGAGN GCCGGNTTNN CCGGGGNGTT GGNCCCGNNG 540
CCTTCCCGAT TTTTNGGGGT TGGGTCCTTN GGTTNCCGGC NTCTGTANGG GGTNCNCCTT 600
TTNNNCCN 608

SEQ ID NO:8
SEQUENCE LENGTH:606
SEQUENCE TYPE:nucleic acid
TOPOLOGY:linear
CLONE:HUMGS00008
SEQUENCE DESCRIPTION:
GATCGCTTCC TAGAAATAAG CAACACCTCT CCCAAAAAGC AGCCCACAAG GCAGGGGCCA 60
GCAGCCCAGC CATCACTCAT CTTTNAGGAA ATNAGTTGGT AGCCTCTGTG CACTGTTTGG 120
TGGCCACATC ANNGGTGATG TCCTGTTCAC ATACCTGCTT GTATTTAAAG CCCTCAGTCT 180
GTCCTGTTGT GTGGGGCGAA GTGATGGACT CTGCCAGGTG GACATGCTGT GGGTGGATGT 240
TCCCGGCGTG TGCCGGCCTG AATGGACAGG GGTCACTTCA CAAGCATGTC AGGGAAAATC 300
ACTGTCACAC AATTCCAATG GATTTTGTGC TCTTTTTGAA AAAAAAAAAT TCTTTAGCGT 360
AAACCATGNA TTTTTTTTCA ATGTAGNCCC TTGGGGANTG AANTGAAATT TTGGGCTTCT 420

TCANATNCGN AAAATNAAAT TTTTACCCCT GAGGGGGGGA GCCCCTTTCT GAAAGAAGGT 480
NTGGGCCAAA AGCCCTTTTA ATGNTTGCTG GCCNTTGNTG GTTTTNANNG TCCANTTTGC 540
TTGGGGCGAA NGCCGNNNTG ANAAAGGTGG GTTTCNCTGG NGGNTTTAAG GNGGTGGTTT 600
GNTTTN 606

SEQ ID NO:9
SEQUENCE LENGTH:606
SEQUENCE TYPE:nucleic acid
TOPOLOGY:linear
CLONE:HUMGS00009
SEQUENCE DESCRIPTION:
GATCTTCANN NGTTAAGCAC TTGCTCTNAA GATTAAAATT CCTTTTCTTT TTAAGGTTAA 60
GGGTGTGTAC GTATGGCAGT GATGTCTATG TTGAGATTAA CTTATGTATT GAGGAAAATT 120
TGAAGTTTAT TTTTTCGATG AATAAGGCTG TCAAATNATT TAGTATAGAT TAATGACATC 180
TTTTTTAGAA ATATTAAAGT GAGTATTCCT CATTATGTCA TCATTTCTGA TAATTAGAGT 240
GCTAATTTGA ATGTTAGATA ATGNTTCCAC ATCTATACCT ATTTCTTTCT AGGGCACTTC 300
TGACCCTGGG GCTTGGGGAT GGCCTTTAGG CACAAGTAGT GTCTGTGTTA AGTTCACTAA 360
ATGTGTATTT AATGAGAAAC ATTCCNATGT AAAAATGTGT GTATGTGAAC GTATGCNNAC 420
ATTGTTATTG TGCACCNGTA CATTGTGAAG AAGTAGTTTN GAAATTTTGT AANGCACAAC 480
CCTTAANGNG GTGTGGAGTT ATTAAANTGN TGTAGGCNCA AATGTAATGT TTAGCCTATA 540
AAAGGCCCTC CTATTGTCCN TNGGCAAGGC TTTGNCNCTT GNAANTAAAN CCCGTNTTTG 600
TTTAAA 606

SEQ ID NO:10
SEQUENCE LENGTH:606
SEQUENCE TYPE:nucleic acid
TOPOLOGY:linear
CLONE:HUMGS00010
SEQUENCE DESCRIPTION:
GATCAGGGGA GACCCCAACT GCCAGATATA TTTTAATGTA CAAAACTGAA ACCAGATGAA 60
ATAATGTTCT GTCACGTGAA ATATTTAAGT ATATAGTATA TTTATACTCT AGAACATGCA 120
CATTTATATA TATATGTATA TGTATATATA TATAGTAACT ACTTTTTATA CTCCATACAT 180
AACTTGATAT AGGAAGCTGT TTATTTATTC ACTGTAAGTT TATTTTTTCT ACACAGTAAA 240
ACTTGTACTA TGTTAATACC TTGTCCTATG TCAATTTGTA TATCATGAAA CACTTCTCAT 300
CATATTGTAT GTAAGTAATT GCATTTCTGC TCTTCCAAAG CTCCTGCGTC TGTTTTTAAA 360
GAGCATGGAA AAATACTGCC TAGGAAAATG CAAAATGGAA ATAGGAGAGA GTAGGTTTTC 420
CAGCTTAGTT TTGAGGGGGG CCGGTTACCT TGTATATTCC CCCATTCACA TTTGGTGTCC 480
ATGTGTAGGG GAAGGTAAAG GGGTGGTTCC ATAATCAAGT TCCCCGTGGG GTGTNCCCCC 540
TGTTAAATGT CCCTGGTTTG GTGTTACCCG GGCTTTATGG GGNCCTTTCA TTATTTCCGG 600
TNGGGN 606

SEQ ID NO:11
SEQUENCE LENGTH:598
SEQUENCE TYPE:nucleic acid
TOPOLOGY:linear

CLONE:HUMGS00011
SEQUENCE DESCRIPTION:
GATCAACAAA AGATAAACAA ATTTGCACGG AATACAAGTA GAATCACAGA GCTGAAGGAA 60
GAAATAGAAG TAAAAAAGAA ACAACTCCAA AACCTAGAAG ATGCTTGTAA TGACATCATG 120
CTTGCAGATG ATGATTGCTT AATGATACCT TATCAAATTG GTGATGTCTT CATTAGCCAT 180
TCTCAAGAAG AAACGCAGAA ATGTTAGAAG AAGCAAAGAA AAATTTGCAA GAAGAAATTG 240
ACGCCTTAGA ATCCAGAGTG GAATCAATTC AGCGAGTGTT AGCANGATTT GAAAGTTCAG 300
TTGTATGCAA AATTCGGGAG CAACATAAAC CTTGAAGCTN GATGAAAGTT AAACATTTTA 360
TAATACTTTT TTTATTTGNT TTAATAANCT TGATTATTTG TTTAANATGG TAATTTTTCC 420
TTCTTCANAT GGCGNNGGGT NAGCAAANCT TTCTTTTTTT AAAAAATTTT CCANTTGTTT 480
AATGGGGAAC TTTGCCCNTT TTCCNCATGG CNTGNTNATT NATTTTNTNT TTTTAAAGGG 540
GGGCCAGTTT TCCCCCNNGG TTTTTTGNCT TNNCGGTTNT TNCAGGNNTN GGGGGTCN 598

SEQ ID NO:12
SEQUENCE LENGTH:597
SEQUENCE TYPE:nucleic acid
TOPOLOGY:linear
CLONE:HUMGS00012
SEQUENCE DESCRIPTION:
GATCGACAGA CCTGAAGCCA TCAGTGAAGA GAGGTTGCGA GAGATGTTTG GTTTATATGG 60
TCAGACAACA GGAAAGGGGA GTATATCTCT GAAAGAACTG AATGCCCGAC CCTTAGAAGT 120
TTTCATGTGT AGTGTGCTCA AAAGACAAGG TTACGGAGAA GGCTTCCGCT GGATGGCACA 180
GTACATTGAT TAACACAAAC TCACATTGGT TCCAGGTCTC AACGTTCAGG CTTACTCAGA 240
GATTTGATTG CTCAACATGC ATAACTTGAA TTCAATAGAC TTTTGCTNGG TTATAAAACA 300
GATGTTTTTT AGATTATTAA TATTAANTCA ACTTAATTTG ANTGNGAATT NGAAANCTGA 360
TTCAAGTAAG NTTTGAGTAT CACANTGTTA GCTTTCCTAA TTCCATAAAN GTACCTTGGG 420
TTTTTTNCAG NTTTATAAAT CTGGCCATCA NCCCCNGCGG CCTTTTNGTA AAGGGGCACC 480
TTTTCCNGCA GGCCATTTGG NAGCNCTTTT TTAACCACCT TGGGNTTTTT AACCCTTTTT 540
TNAAGGGNCN NCNTGGTTAA NTTTTTTNNT GGGCCTTTNC NGGGCCTGGT NTTTAAN 597

SEQ ID NO:13
SEQUENCE LENGTH:593
SEQUENCE TYPE:nucleic acid
TOPOLOGY:linear
CLONE:HUMGS00014
SEQUENCE DESCRIPTION:
GATCTTCCAA AGCACTATTT GTTGTAATAA CTTTTCTAAA TGTAGTGCCT TTAAAGGAAA 60
AATGAACACA GGGAAGTGAC TTTGCTACAA ATAATGTTGC TGTGTTAAGT ATTCATATTA 120
AATACATGCC TTCTATATGG AACATGGCAG AAAGACTGAA AAATAACAGT AATTAATTGT 180
GTAATTCAGA ATTCATACCA ATCAGTGTTG AAACTCAAAC ATTGCAAAAG TGGGTGGCAA 240
TATTCAGTGC TTAACACTTT TCTAGCGTTG GTACATCTGA GAAATGAGTG CTCAGGTGGA 300
TTTTATCCTC GCAAGCATGT TGTTATAAGA ATTGTGGGTG TGCCTATCAT ACCANTTGTT 360
TTCTGTATCT TGAAAAAGTA TTCTCCACAT TTTANATGGT TTTATATTNG GGGATTCCTT 420
TAATGCCCCC TTGGNCAAAT TTATATATAT GGGCCCCATN GTTNCCNTTT NAATTTTTNG 480
GTTTTNGGGT GTAAGGGNCN TGCNCNTATG GTGGGGGCCT CCCAAAANTG GGANCANGNT 540

TTTNCCTNNG GACNCCCNNT TGGTTGTTNA GGGGGGCCAA TNTTTCNTNC CCN 593

SEQ ID NO:14
SEQUENCE LENGTH:574
SEQUENCE TYPE:nucleic acid
TOPOLOGY:linear
CLONE:HUMGS00015
SEQUENCE DESCRIPTION:
GATCGTNCTG CAGTGGGAGC GTGTGACTGC GGAAGTTGTG AAGCCACGGG AAACTCGGAG 60
CCTAGAGCAG CAGCTCAGCG CTTCTATGGG AAGCGGTCTC GAGCAGAAGC CCCACTGAAG 120
TGTCCCCTGG CAGACACCCA CATGAACTCT TCCGAGAAAC TCCAGTTCTA TAAAGAGAAA 180
GCCCCAGATT GCCATGGGCC AGTNTTGAAA CACGAAGCTA TCTCAAGCCA GGAGTCAAAG 240
AAGAGCAAGA AGAGACCTTT TGAGGAGTCA GAGACAGAAC AGAATAACTC TTCACAACCT 300
TCAAAGCAGA AATATGTATG TNTTGCTGTG GAAGACTGGG GACTTGTTAA ATTCCTATTG 360
ATTAAGTAGA TACAAGTTGA CCTTTCTCTG GCCCCCAGCT CTAGTGTTTG AGTAAAGGGA 420
GACTNAGGGT GGGTTTACTT TTTTNGGTTT GNATTTANCC TATTGGCATT TAGNCATAGG 480
TAAGGCGGTN TTTTCNNCCT TTTNTTGGNT TTGGGNGGGG GATTNTNTNN GGGGNNNNAA 540
AAAANTTNCC ANGGGNTTTT TCCAAANCAT NGGN 574

SEQ ID NO:15
SEQUENCE LENGTH:573
SEQUENCE TYPE:nucleic acid
TOPOLOGY:linear
CLONE:HUMGS00016
SEQUENCE DESCRIPTION:
GATCAGCCCA GAGGAATGCT GAGAAATCAC CTGGAGGAGG GAGCAGAAAG AGAAGGTTTT 60
TAAGGAGGGG CTTCTGAATA CTTGGGAGAT ACGGAACGGA CCAAGGACCA CACTCCAGGG 120
TGCATTCGTT GCTCCCTGGG GCACCACTTC TGGATTACAG TGTGCCAGGT CCTTTGGAGG 180
CCCTACCCCT TCCCCATTCA TTGCCACCAG TGAGAAATNG GGGTGCCCCT GTGTAAAGAA 240
ACCTACCAAA GGTTTACATT TGCACCTTAG CCTCAATAGC TACGAACCCT AGAGAAGCAG 300
CTAGCTGGAG CTCATGTGCA ACTCCTGATT CTCAGGAGAA AGATGGATTT TAACCNAAAA 360
TTATGAGTNA GCTGTTAACT CTAAAATGTA CTTNGGGAGA TAGGGCCAAG NGAGAGGTCA 420
TGGGCCAACT TAAGTGTTAT CCNGTAGGAA AGNNCAGGTA NCACTGGNTT TNTTTTTNNA 480
GGGTTGCTNT TNCCTTNTNC TAAANGGATT TGNTANTTCC NTGGGGNTNG NATTTAAATT 540
GGGTTNTAAA AAGNTTNGAC GTGANAAAAA TTN 573

SEQ ID NO:16
SEQUENCE LENGTH:567
SEQUENCE TYPE:nucleic acid
TOPOLOGY:linear
CLONE:HUMGS00017
SEQUENCE DESCRIPTION:
GATCAAAACA GTCTCACCAG CACCATATCC ACATTCTAGC CCATGGAAAG GGTAAAGAAA 60
AGAAAGTGGA AGATATTAGT TTCCATTTAA GGAAGTGACA TGGAGNTAAT ATAAGTCACC 120
TGTGTTCACA TTCCACTGGC AAAAATTCAG TCACAGGAAT GTACTTAGCT GCAGAGGAGG 180

CTAGGATATG CCCTCCAAAA ACTCAGGGGG GATGTCCTAC TTGTAAAAGG AAGAAATGAA 240
AANTGGACAC TGAGAGGAAA TCAGCAGTCT CAGAGGTAAT ATACTTACAC AAGTTTTTTA 300
AAAACCATAA AATGATGCAG AATGAAGTTT CCACTCTATT CACCATCTAT CCAGTCCTCA 360
TGCCTCCCAN CANGNGGTAA ATNCTTTATT ATTGTGTCAT GGTATCTTTC CAGGGTTTTT 420
CTAAGCAATT TTGCAACNGN TTTTAATTAT GTATCCTNAT TTCCTACNTT ACCCCNTTTT 480
TGTCCATGNA NGGNANANTN TCCTGNGGNN TAACCTTCNT GTNTCTTTTT TTCCCCTNAT 540
ATTTTTTGGC GGTNCCNTNN NTTTGGN 567

SEQ ID NO:17
SEQUENCE LENGTH:552
SEQUENCE TYPE:nucleic acid
TOPOLOGY:linear
CLONE:HUMGS00018
SEQUENCE DESCRIPTION:
GATCTTACCC GTGACAAAAT GTGTTCCATG GTCAAAAAAT GGCAGACAAT GATTGANGCT 60
CACGTTGATG TCAAGACTAC CGATGGTTAC TTGCTTCGTC TGTNCTGTGT TGGTTTTACT 120
AAAAAACGCA ACAATCAGAT ACGGAAGACC TCTTATGCTC AGCACCAACA GGTCCGCCAA 180
ATCCGGAAGA AGATGATGGA AATCATGACC CGAGANNNGC AGACAAATGN CTTGAAAGAA 240
GTGGTCAATA AATTGATTCC AGACAGCATT GGAAAAGACA TAGAAAAGGC TTGCCAATCT 300
ATTTATCCTC TCCATGGNTG TCTTCGTTAG AAAAGTAAAA ATGCTGAAGA AGCCCAAGTT 360
TGAATTGGGA AAGCTCATGG AGCTTCATGG TGAAGGCAGT AGTTCTGGAA AAAGCCACTT 420
GGGGNCCGNG ACAGGTGCTT AAAGGTTGAA CCGNGCTNGA TTGGTTNTGA ACCCACCAGT 480
CCCAGGAATT CTTGTTTAAA GTTCCAGNCN TTCAATTAGT TGGCAAATTA AAAANGTGCT 540
TNTTTGNGGA AA 552

SEQ ID NO:18
SEQUENCE LENGTH:581
SEQUENCE TYPE:nucleic acid
TOPOLOGY:linear
CLONE:HUMGS00019
SEQUENCE DESCRIPTION:
GATCGCCGTT CTGGTAAAAA GCTGGAAGAT GGCCCTAAAT TCTTGAAGTC TGGTGATGCT 60
GCCATTGTTG ATATGGTTCC TGGCAAGCCC ATGTGTGTTG AGAGCTTCTC AGACTATCCA 120
CCTTTGGGTC GCTTTGCTGT TCGTGATATN AGACAGACAG TTGCGGTGGG TGTCATCAAA 180
GCAGTGGACA AGAAGGCTGC TGGAGCTGGC AAGGTCACCA AGTCTNCCCA GAAAGCTCAG 240
AAGGCTAAAT CGAATATTAT CCCTAATACC TGCCACCCCA CTCTTAATCA GTGGTGGAAG 300
AACGGGTCTC AGAACTGTTT GTTTCAATTG GCCATTTAAG TTTTAGTAGT AAAAGACTGG 360
TTTAATGATA ACAATGCATC GTAAAACCTT CANGAAGGGN AANGGAGGAA TGNTTTTGTG 420
GGCCCACTTT GGGTTTTNCT TTTTTNCGGT GTNGGCAGTT TTTAAGGTTN TTAAGTTTTT 480
TNAAAATNCA GGACCTTTTT TAANTGGNAA CCAACTTTGG CCCAAAAATT TTGTCACCAG 540
ATTTTTTNGG GCCCCNTTTA AAAANGTNTT NATTNGGGAA A 581

SEQ ID NO:19
SEQUENCE LENGTH:556
SEQUENCE TYPE:nucleic acid

TOPOLOGY:linear
CLONE:HUMGS00020
SEQUENCE DESCRIPTION:

```
GATCAGCAGG GTTCTTTGTA AATAGTATTT TGAGACACTA AGATGTTTCT ACTGCTACGG  60
AATGTATTTT AAACACATAT CGTTTCTTTT TCTTGGAAAA AAAGTTGATT AGGACCACAG 120
NNNNGGTTTA GAAAGGGTAA TATTTTGAAA TACTACAAGG TTTAGACAGT CCATGAAATC 180
GACCTGTTTA ATAATTTACC ATCCTGAAAG TCCAGAATTA AAATATGGAA GCAAGAACTA 240
TATAATTGAT TAGGATGCTT GGTAGGTTTT TTTCATTGTT CAAATATTCA TTGCACAGTG 300
GATTGTTTTG ATTAGTTAGT ATGCTTTTTT TTTAATTAAT TCAGTCTTCT GTTAATTTTT 360
AAGNTTTGGT TAGTGCCACA AGGAATTTNA CTTNTTGATT TGTATAATNG GAACCTGACC 420
TNGGGATTNG TAGCGGGGNT TTGAAGGGTG GGGACCTNCC CNCAAAATAA GGGGGAGGTT 480
TCCAAANNTT CCNCTGGCCG NCCNNNNATC CCAGGTAAAG GGGGTNGAAA TANTNNGGGN 540
NCCCCCCAGG GGAAAN                                                556
```

SEQ ID NO:20
SEQUENCE LENGTH:555
SEQUENCE TYPE:nucleic acid
TOPOLOGY:linear
CLONE:HUMGS00021
SEQUENCE DESCRIPTION:

```
GATCTGGTAC GAGTGTGTGA AAACATCCCC ATTGTGTTGT GTGGCAACAA AGNGGATATT  60
AAGGACAGGA ANATGAAGGC GAAATCCATT GTCTTCCACC GAAAGAAGAA TCTTCAGTAC 120
TACGACATTT CTGCCAAAAG TAACTACAAC TTTGAAAAGC CCTTCCTCTG GCTTNCTAGG 180
AAGCTCATTG GAGACCCTAA CTTGGAATTT GTTGCCATGC CTGCTCTCGC CCCACCAGAA 240
GTTGTCATGG ACCCAGCTTT GGCAGCACAG TATGAGCACG ACTTAGAGGT TGCTCAGACA 300
ACTGCTCTCC CGGATGAGGA TGATGACCTG TGAGAATGAA GCTGGAGCCC AGCGTCAAGA 360
AGTCTAGTTT TATAGGGAAG TTGTCCTGTG ATGTCAGCGG TNCAGCGTGT GTNCCACNTC 420
ATTATTATCT AGCTAAGCGG ACATGTNTTC ATCTGTGGGN TCTTAAGGAG NTGAGTNGNN 480
TNGGNGTNAT NTGGANTTAA AAATAACTTC ATTNTTNGGC CNNATATTTA NGTNTTTTNG 540
CCCGNTTNNT CCCTN                                                 555
```

SEQ ID NO:21
SEQUENCE LENGTH:544
SEQUENCE TYPE:nucleic acid
TOPOLOGY:linear
CLONE:HUMGS00023
SEQUENCE DESCRIPTION:

```
GATCGATACA TGCAAATTTA ATGTAGTAAC TCACTTTTCC ATATATTTTN AATGTATATT  60
TCTATTTATG AATACCAATT TATAAAAAAT AATTACACAG AAAAAAATGG AATAGGAAAA 120
ATTATGCATC TAGCACATTT AAACTGTGCA AATATGAAAA TTTTTCGAGG ATTACATTTT 180
NTNNNTAGGC TGCATATTTT AACTGGCTTT AAAACTGTAA CACATCACAT AAAAGTACTT 240
TACCCGGTAT GTATTGCATT ATATCATTGC AATAATTATT GGAGTCTAGA TATCGAGCCA 300
TCCCAGGTGT TGGGCGGGGG GAGGGTTGTG GCAAGTTGTC TTTTCAATTT NGNGNGTTTT 360
CCTGTNGCTC CAGGGCAAGT ACCGGGTTGG AAAGCTGCCT GTAAGCGTTG GCACCTTCAT 420
AGNGTAGTGT TTNGGTGNCT TTTTTTNTCG GTTCTTGTAA ATTNGGTNCG GTNGGTGGTG 480
```

TTCAGATGNT TTTTTNCNCT NGTTCAGCAA CTTNCCCNNT NNCTTGTCTT GATAGGGNAC 540
NTCN 544

SEQ ID NO:22
SEQUENCE LENGTH:538
SEQUENCE TYPE:nucleic acid
TOPOLOGY:linear
CLONE:HUMGS00026
SEQUENCE DESCRIPTION:
GATCTCTATC CAGTTGGACA CTTAATTGCT TTCTTCATTC AGAAAGATAG TCATGTTCAC 60
TGGTATATTT GGTCACTCTT AGAACCTGTC CTTCACATAT GTTTTTTATG GGACCCATGA 120
ATGGTTAGCC TTTCTGTACT ATTGTAGAAG GAAATAAATA GGCGTAAAAA GACCATTGTA 180
GTAAATAAGT TCAAGGGGAA CTTGGGACCA GAAACCACTG GTATGTACAA AAAAGCTGGC 240
AATTTGAATA ACCTCAAGTT TGACAATAAT TTTTAAATTT GAACAGTTAT GATAAATTTC 300
AGTAGTTTTA TACACTAGAT GTGCCTAGAT GGTTCTCAAG GCTTATAGGA CTGGCTCACA 360
GTTCACCATT TCTAGTGGCT TTTCTTGCGT TTGGGTCTGG GGGGCTGGGG GCAATGGGCT 420
TTTTTCCTTG GGTTGCCAAT CGGTTTCTCT GGTTGGTCAG TTTCCAATCC TTNGGGGGGT 480
CNTTGTGNTN TTNNCNGCCC NGNNNGGTNN GTCCNGGGTG TTNGCCGGGN NCNTTTTN 538

SEQ ID NO:23
SEQUENCE LENGTH:535
SEQUENCE TYPE:nucleic acid
TOPOLOGY:linear
CLONE:HUMGS00028
SEQUENCE DESCRIPTION:
GATCCTGGAA TATGTCGAGG TTATATTACC AGGTATTTTT ATAACAATCN GACAAAACAG 60
TGTGAACGTT TCAAGTATGG TGGATGCCTG GGCAATATGA ACAATTTTNA GACACTGGAA 120
GAATGCAAGA NCATTTGTNA AGATGGTCCG AATGGTTTCC AGGTGGATAA TTATGGAACC 180
CAGCTCAATG CTGTGAATAA CTCCCTGACT CCGCAATCAA CCAAGGTTCC CAGCCTTTTT 240
GAATTTCACG GTCCCTCATG GTGTCTCACT CCAGCAGACA GAGGATTGTG TCGTGCCAAT 300
NNGAACAGAT TCTACTACAA TTCAGTCATT GGGAAATGCC GCCCATTTAA GTACAGTGGA 360
TGTGGGGGGA AATGAAAACA ATTTTACTTC CAAACAAGGA ATGTCTGAGG GGCATGTAAA 420
AAAGGGTTTC ATCCAAAGGA TTTCANAAGG GNGGCTNATT TAAAACCANA NGGAANNGGN 480
AGGAGGCNGG NNGTGAATTT GNNTTTTGGN GGAANTTTTT GNTTNAANNT TNTGN 535

SEQ ID NO:24
SEQUENCE LENGTH:528
SEQUENCE TYPE:nucleic acid
TOPOLOGY:linear
CLONE:HUMGS00029
SEQUENCE DESCRIPTION:
GATCTACACT TGGATGGATG CAACTTTGAA AGAACTGACA AGTTTAGTAA AAGAAGTCTA 60
CCCAGAAGCT AGAAAGAAGG GCACTCACTT CAATTTTNCA ATCGTTTTTA CAGATGTTAA 120
AAGACCTGGC TATCGAGTTA AGGAGATTGG CAGCACCATG TCTGGCAGAA AGGGGACTGA 180
TGATTCCATG ACCCTGCAGT CGCAGAAGTT CCAGATAGGA GATTACTTGG ACATAGCAAT 240

TACCCTTCCA AATCGGGCAC CACCTCCTTC AGGGCGCATG AGACCATATT AAATTCTATT 300
TACTATTTGT TGAATTTATT TTTCCGTCAG TTATGTAAAA TAAACATACT CTNCCTTCCT 360
CCCCTGGATT TATTGCCATT TAAGGCTTTT AAAATTCTAA TCAANNTTTG TNANTGCAAT 420
CATCTGTTTN GGGNGTTNNG TTTTGGGATG TGCTTTTTGN NTGGNTTNCG GNTTAGNCTT 480
GGNTTGTTTT AATGGCCNTT CNNGTNANAT TTGGNGGNAA NGNGCTTN 528

SEQ ID NO:25
SEQUENCE LENGTH:528
SEQUENCE TYPE:nucleic acid
TOPOLOGY:linear
CLONE:HUMGS00030
SEQUENCE DESCRIPTION:
GATCAAGGTA TAATGGAAAA ATATACCTAT TCTTGAAGTA GTTTATTATN GTTTTCAAAT 60
TGATTTATAC CATTATTAAC CTGATGTGGT CTGCTTAAAA AATGAATATA TCAGTATTTA 120
GAAATAAATT GCAAAGGTGG GAATATATAC TTAAATAATT TGTCTTAAGT AAATTAGCAT 180
TTGGTAGTCT GANATGGTGA CAGATTACTT GTTAAAATTG TGAAANCTCT GTTGTGTCCT 240
CTCTNCCTAC ATTTGTCCCT GAGAGTNCTC CACGATTACT AGGTTCTTGA TTCCCTTATA 300
TGGCAATCAG GCAGAGGCGT TCCTTAAGCA TTAGAGAGTT CTGAAGCTTA AGATTTGTTT 360
TGGTTGGATG AANGTCCTTA GTACAGTTGA AAANCAGAGC ATTAAAGNCT ANTCANTTTG 420
TTTTGNCCTC ACCAGTCATT TTAAAATNNG TNGGAATNCT TNTTNNCTCA GTGCTTAAAN 480
NTTTCCNTTG TTTCAANCTG NNGGGGGTTN GGATTAAACC AGGCCNCN 528

SEQ ID NO:26
SEQUENCE LENGTH:527
SEQUENCE TYPE:nucleic acid
TOPOLOGY:linear
CLONE:HUMGS00031
SEQUENCE DESCRIPTION:
GATCAGAATG CCCCTCCACT CATGAGACTC TTCATTTTGT CCACTTTGAC AGGAAAAGTG 60
GGAATGTATG CAGAGCTCTC AAAAGAAACA AAAAAGGCCA AAACGGTGCC TTCAGCCACA 120
TCCTCTGAAT TGGCCCTGAC TTGGACTAAA TGCACTAATG CAAAATCCCT TGACAAAAGC 180
GCATAGGTTA TTTCAAACCA GCATTGTTTT TTATGTAACC TGTTTTACCG CATCTTCTCA 240
GCAGCTTCTG ACCACTGCTC AATTTTCTCC TTTACAGCCA TTGTTCTGGT GGACAAATAA 300
CCTAGGTACT CCAAATCCTG GCAGGAAAAA TATACAGCAT TATGAANCAG CACTCANGTA 360
ATCCTAAAAT GGATTTCCAA AGCTGGTTAC ACATGGCCCT GGNAANGTCN TATTGANTTT 420
ANANGGGCTT TCTTCNTTTC AGGAGTTTNG GTCAACGGTG GCAAATCCNT GGGGTNNTTA 480
ANTGGNNNGG TTNNTTAANT TNTGNTANTT TCNTNGGGGC CANAGGN 527

SEQ ID NO:27
SEQUENCE LENGTH:520
SEQUENCE TYPE:nucleic acid
TOPOLOGY:linear
CLONE:HUMGS00033
SEQUENCE DESCRIPTION:
GATCAAAATG GTTGGTGAAC CTCCACATGT CCAGTTCTGT TGCCAAACTT TCCATTCAGA 60

GTATTTGGTG GAGTTTGAAT TTGAGCAAAC TAAATGCCTT CATCTTAGGT AGAAAGGGCC 120
TGAATCTTCC ATTTTATATT CAAACCTCAT TGTTATTTGG CCTAAGTAAA AAGTCAGATT 180
TCATTTCCAT TTACCTGAGT TCGCTTTAAA GAGCTTTTCA AAGAGAGCTT TATAGACACC 240
CACAATTGTC CCCAATCTCT TCATGATGTT GCATTAATAG TTGTTTTTGT CCCTTTCTTG 300
GAAATGTTAA TGCCAAAGNT TGCCTGAACA TTNGGGCGGG TTTTCTTAAA TTTGAANGTN 360
TAAAANTTTT NTAANGGGGG AATTNCCAAA NGGGTATTTA AAAGGGTTNG TTTTAACCAG 420
GTATTGTNGT GGGGGGATGG TCCAATAATC CTCCNGGGGG AGGGCTTTCA AGGGAAATCC 480
CNTTTTNGGG GAAATAAAAA NNGGGTTAAA ANNNNTTTTN 520

SEQ ID NO:28
SEQUENCE LENGTH:514
SEQUENCE TYPE:nucleic acid
TOPOLOGY:linear
CLONE:HUMGS00034
SEQUENCE DESCRIPTION:
GATCAGCGAG GCCGACAAGA AGAAGGTGCT GGACAAGTGT CAAGAGGTCA TCTCGTGGCT 60
GGACGCCAAC ACCTTGGCCG AGAAGGACGA GTTTAAGCAC AAGAGGAAGG AGCTGGAGCA 120
GGTGTGTAAC CCCATCATCA GCGGACTGTA CCAGGGTGCC GGTGGTCCCG GGCCTGGGGG 180
CTTCGGGGCT CAGGGTCCCA AGGNAGGGTC TGGGTCAGGC CCCACCATTG AGGAGGTAGA 240
TTAGGGGCCT TTCCAAGATT GCTGTTTTTN TTTTGGAGCT TCAAGACTTT GCATTTCCTA 300
GTATTTCTGT TTGTNAGTTC TCAATTTCCT GTGTTTGCAA TGTTGAAATT TTTTGGTGGA 360
AGTACTGAAC TTGCTTTTTT TCCGGTTTCT ACATGCAAGA GATGAATTTA TACTGCCATC 420
TTACCGGCTA TTTCTTCTTT TTTAATTCCA CTTAACTCAG GCCATTTTTT AAAGTTGGGT 480
ACTTGCAAAG TAAAATAAAC TTTAAAAATT CAAA 514

SEQ ID NO:29
SEQUENCE LENGTH:513
SEQUENCE TYPE:nucleic acid
TOPOLOGY:linear
CLONE:HUMGS00036
SEQUENCE DESCRIPTION:
GATCATCATT CTTCTGACTC TAGATGGGAC ACTTGACAGT GACTTGAAAC ATTTGCATAT 60
TCAGGAATGC ATGAGATTTC AAGAGAGCCT ACAGTATGAA ATCATTTTCA CAAAATAAGC 120
AGCTTGCTTC TGAAATGCTG TCTTTCCCAG TAGCTACTCA CCTGCCTCTG GTGGCTGGGA 180
TTCAGATGCC ACAAAACTGT CAGTATCTAT AGACCAGGTC TGTGCCACCT CCTCTCTCCT 240
CTGTGCTCAG TGAGGAGGCA GTAAATGAAG TTACAGGCTA GCACAATACC TAACTCATGT 300
TTCCCAGTAC ACCTGTTGGA TATTNNCTGT NCCTTTTAAT GGTTCTCAAG GGANTTAGGT 360
TNTTGNCCTG TTTCCAGNGG TTTCCAGGTT TTCTTTGGGT NCTTTTTNAA TTTTNAANTT 420
CCNAGGGGGG GGGNNTTTTG GGNAAGGGGG GGGCAAAGGG GNTTTTTTTT TTNTTGGGCC 480
NNGNTTTTTG GGGGGAAANC CTTTNNGGTN NCN 513

SEQ ID NO:30
SEQUENCE LENGTH:512
SEQUENCE TYPE:nucleic acid
TOPOLOGY:linear

CLONE:HUMGS00037
SEQUENCE DESCRIPTION:
GATCTAAATT GTTACATTTT ACCATTTCAT TCCGAAGTTG GTTTTACTTT ATTAAATGAA 60
GATTTAGTTT TCATATCGTA TACATAGCTG TATAGATTTC AAAATNAGGT TGTTAATTTG 120
TGTCACTTAC TATTTTTGTG TTGGTAATGC TTTAAATGCA TACTTAAAAA TGAAGTACTG 180
TTATCTAAGC TACTGTGTTT AGAAAATGTT AAGAATGAGC AGAAATTTTT ATAGAAAAGT 240
ATAACCGGAA GANGAGAGAA GATACTGCGA ATAGGCCCTC AANCTTAAAA NAGAAAAACC 300
TTTGCCAGTT TTANGGACAT ATTTTGATTC TTTCNGTATT CTTAACACCT TTTTAAACAA 360
NGTTCTTGAT AGTACCCACT ATTATTGGGT TTGTTTTATG CCATTATTTG ATTCTTGGAT 420
ATTCAAGCAT TTNCAATGTG GCATATTTNG NTTTCCNNTN NCCTTNCNTT TTTTNGGNCN 480
NCNTTACCCT TTCCNTTGGA AAGNCANTTN GN 512

SEQ ID NO:31
SEQUENCE LENGTH:510
SEQUENCE TYPE:nucleic acid
TOPOLOGY:linear
CLONE:HUMGS00038
SEQUENCE DESCRIPTION:
GATCAGCCAG GCACAGAATC TCCAGAACAA CCTAGAGAGT GAATGCTAAT TTGTAGAGCG 60
AACTTCCATT TGGCCCATNA TTTGTAACTG TGTAACTGCT CCAAGTGCCA GANTGCTTAC 120
ACGTTAAAGC AGCACCTTTC CATTTGCCCA CATATTCTTC TTGCACACCC CTTCCATTAC 180
TGCTGAATAG GACATTGCAT GGGAAGAGTA CAGAGGTGGC AGANTGANGC TAGAGTGGGC 240
AGGNCTAAAG ACTGAGCCCC AGAGTGCTCC CAGCAACCGC CACGTACANG GTCTGNAATG 300
NCANGGGCAN GNGTGAGATT GGAANCTGTG TGTGAANGGT AAGCCCTTGC AGTNTTTCTG 360
CCTCCCTTTC TTTCTGCCTT TCACCCCNCT TANTTGTNTG GTTNTTGGTT TGCCCGTTCT 420
TCTCTTGGTG GNTGCNCATT TGTTANATGG TGTTAGGGGT GTGGGGNTGA GGTTTTCCCC 480
TTTGATGTGG GNTTNTTCCN TTGGGTTTAN 510

SEQ ID NO:32
SEQUENCE LENGTH:507
SEQUENCE TYPE:nucleic acid
TOPOLOGY:linear
CLONE:HUMGS00039
SEQUENCE DESCRIPTION:
GATCGTGAAG CCCAATGGCG AGAAGCCGGA CGAGTTCGAG TCCGGCATCT CNCAGGCTCT 60
TCTGGAGCTG GAGATGAACT CGGACCTCAA GGCTCAGCTC AGGGAGCTGA ATATTACGGC 120
AGCTAAGGAA ATTNAAGTTG GTGGTGGTCG GAAAGCTATC ATAATCTTTG TNCCCGTTCC 180
TCAACTGAAA TCTTTCCAGA AAATCCAAGT CCGGCTAGTA CGCGAATTGG AGAAAAAGTT 240
CAGTGGGAAG CATGTCGTCT TTATCGCTCA GAGGAGAATT CTGCCTAAGC CAACTCGAAA 300
AAGCCGTACA AAAAATAAGC AAAAGNGTCC CAGGAGCCGT ACTCTGACAG CTGTGCACGA 360
TGCCATCCTT GAGGGACTTG GTCTTTCCNA AGCGGAAATT NTNGGCAAGA GGANTCCNGN 420
GTCAAACTTT GNTTGGCANG NCGGGCTCAT AAANGGTTCA TTTTGGNNCA ANNGACNAGN 480
AGGNCCAATT NTGGGNCAAA NAGGNTN 507

SEQ ID NO:33

SEQUENCE LENGTH:508
SEQUENCE TYPE:nucleic acid
TOPOLOGY:linear
CLONE:HUMGS00040
SEQUENCE DESCRIPTION:
GATCAGAAAC TCACCCTAAA TCTNAACGGG TGCCGCTATA ATTNGTNACA TCTGGCAAGA 60
TTTCCCTTTA TGTATATATT TAAACAATCC GCTTGGACAC GAACAAAGCC ACACTTCTAA 120
CTGCTTCTGG CGAACTGATT TAATTTTNAA TTTTTTNCAA TAAAGATATT CTTAGATACT 180
GAAAGAAATA GTTAATGAGT TTNCATTTGT CCTTGAGAAA ATTTGGCTCA AGTCCATTTG 240
GCTGTAGTGT CAACGATGTT TCCAGTAGTG TTTAGGATTT GGTGTCTTCA AAGGTAGTTG 300
ATTAAACCAA GTGTGTCTTT AATATCTTGT ATCAGAATAA CTTTGTATGT TACCAACTTA 360
AATTGCTAGA ATAAGGGTAA ATTGGATACA CAACTGCTGA TTTTTAATTT AGGANCTTTG 420
ACCNNATTTT GGGGTTTTCA AANCCGTTTT TGGNTGCTNT GTATCCTTAT GCTGTTTGGT 480
TNATTTCCAN TAAAAANTTC ACNCGNGN 508

SEQ ID NO:34
SEQUENCE LENGTH:505
SEQUENCE TYPE:nucleic acid
TOPOLOGY:linear
CLONE:HUMGS00041
SEQUENCE DESCRIPTION:
GATCTGTAAG TAACTTCACA TTAAAAAATG AAATATTTTT TAATTTAAAG CTTACTCTGT 60
CCATTTATCC ACAGGAAAGT GTTATTTTTA AAGNNAGGTT CATGTAGAGA AAAGCACACT 120
TGTAGGATAA GTGAAATGGA TACTACATCT TTAAACAGTA TTTCATTGCC TGTGTATGGA 180
AAANCCATTT GAAGTGTACC TGTGTACATA ACTCTGTAAA ANCACTGAAA ANTTATACTA 240
ACTTATTTAT GTTAAAAGAT TTTTTTTAAT CTAGACAATA TACAAGCCAA AGTGGCATGT 300
TTTGTGCATT TGTAAATGCT GTGTTGGGTA GANTAGGTTT TCCCCTCTTT TGTTAAATAA 360
TATGGCTATG CTTAAANGGT TGCATACTGG GGCCAAGTAT AATTTTTNTG GTAATGTGTG 420
GNAAAGGATG NCCAGTTATT GGTTACCNCT TTANGNNATC CNNTAANGGG AACCTTCCCC 480
TNGGTTAAAA NCANGNNGTA NANNN 505

SEQ ID NO:35
SEQUENCE LENGTH:62
SEQUENCE TYPE:nucleic acid
TOPOLOGY:linear
CLONE:HUMGS00042
SEQUENCE DESCRIPTION:
GATCTGGAAA GAGCTGTTTT GGATGAATGC AGTATAAAAT GTAAAANCCC TGCTAAATGA 60
AA 62

SEQ ID NO:36
SEQUENCE LENGTH:503
SEQUENCE TYPE:nucleic acid
TOPOLOGY:linear
CLONE:HUMGS00043

SEQUENCE DESCRIPTION:
GATCGAAAGG CACTCCATGA GCTAAAACTG GAAGAGTGGA AAGGCAGACT ACAAGTTACT 60
GAGCACCTCC CTGAGAAAAT TGAAAGTAGT TTACAGGAAG ATGAACCTGA GAATGNTGCT 120
AAGAAAATTG AAGCACTGCT AAACCTTCCT AGAAACCCTT CAGTAATAGA TAAACAAGAC 180
AAGGACTGAA AGTGCTCTGA ACTTGAAACT CACTGGAGAG CTGAAGGGAG CTGCCATGTC 240
CGATGAATGC CAACAGACAG GCCACTCTTT GGTCAGCCTG CTGACAAATT TAAGTGCTGG 300
TACCTGTGGT GGCAGTGGCT TGCTCTTGTN TTNTTCTNGN CTNTTTAACT AAGAATGGGG 360
CTGTTGTACT CTCACTTTAC TNATCCNTAA ATNTAAATAC ATACTGATGN TTTGTATTAA 420
TCGNTCCAAT ATATGNNTAC ATGNANTATA TCNACNCNCC TTNGATNTTT AAGCANGTAA 480
ATAAAACCAT TNNGCAATGG AAA 503

SEQ ID NO:37
SEQUENCE LENGTH:497
SEQUENCE TYPE:nucleic acid
TOPOLOGY:linear
CLONE:HUMGS00044
SEQUENCE DESCRIPTION:
GATCGATAAA GAATTGGCTA GTGGTGAATA CTTTTTGAAG GCAAATCAGA AGAAGCGGCA 60
GAAAATGGAA GCAATAAAGG CTAAACAAGC AGAAGCCATC AGTAAGAGAC AAGAGGAAAG 120
AAACAAAGCA TTTATTCCAC CTAAGGAAAA ACCAATTGTG AAACCTAAGG AAGCTTCTAC 180
TGAAACTAAA ATTGATGTGG CCAGCATCAA GGAAAAGGTT AAGAAAGCAA AGAATAAGAA 240
ACTGGGAGCT CTTACAGCTG AAGAAATTGC ACTTAAGATG GAGGCAGATG AAANGAAAAN 300
GANGAAAAAN NAGTANCATA CCCAAANCTC CTTGNCTNGG ACCTATCTCC TTTTTNGTAA 360
AGGGGTTTTT TGGGGTTTTC AGGCCTTTAG GTTNCCCTTT TTTTGNGGGA AANTTTTNTT 420
GGGGGGGTTT TTTNNCNTTT TTTNGGGGGG GGNGGGGGTT TTNNTCCTTG GGNGGGGTTT 480
TCTTTNNAAA AATTTTN 497

SEQ ID NO:38
SEQUENCE LENGTH:498
SEQUENCE TYPE:nucleic acid
TOPOLOGY:linear
CLONE:HUMGS00045
SEQUENCE DESCRIPTION:
GATCTTATGG ATGCTGAGCA TGTTCTGCAC TGGTGCTAAT GTCTAATATA ATNTTATATT 60
TACACACATA CGTGCTACCC AGAGATTAAT TTAGTCCATA TGAACTATTG ACCCATTGTT 120
CATTGAGACA GCAACATACG CACTCCTAAA TCAGTGTGTT TAGACTTTTC AAGTATCTAA 180
CTCATTTCCA AACATGTACC ATGTTTTATA AACCTCTTGA TTTCCAGCAA CATACTATAG 240
AAAACACCTG CTACTCAAAA CACAACTTCT CAGTGTCATC CATTGCTGTC GTGAGAGACA 300
ACATAGCAAT ATCTGGTATG TTTGCAAGCT TTCAAGATAG CCTGAACTTA AAANGTTGGT 360
GCATTAGTTG TATCTGATGG NTATAAATTT TGCCTCCTAG GTTCACTTTG GTGTCCAGGN 420
GCTAAACCTG TGGANCCTAA CTTTCCCCTN ATTGGGGGGG GAATAACCTG GAAAATAAAG 480
GGTTTTTTTC CAGGGNTN 498

SEQ ID NO:39
SEQUENCE LENGTH:494

SEQUENCE TYPE:nucleic acid
TOPOLOGY:linear
CLONE:HUMGS00046
SEQUENCE DESCRIPTION:
GATCTGTGCT GTAGAAAAAC ATTAACCCTT GTTCAAAAAA GAAATGGATA ANCTTGGCCT 60
TTCTAAGTGG TAAGAATGAC CTGTCACTAT AATATACTGT ATGTTTACAT TTNATTTAAA 120
TTTAATCTCT TATGTATAGG GTGATAACCT TCCCCAGAAA CAACAGTGAT TGCNATTGTT 180
TTCTAGAAAC TNCTTTAAAG TGCCACATTT GGCAGTACAA ATGAGTCTGA GTGTAATAGC 240
CCAGAGATTT ATATATAGTT GAATGTCTAA NATGGTAAAA TGTGCCACTG TGTCAAGTTA 300
CAGTGGCTTA TGTTTTTCAT AGTAATTCAN ATGANCTTCC TATTTTTGNT AGTAAATGGC 360
CATTTAATAG NATTCCTTGG CCATTTGAGG CTCACTGGCA AATTTTAGGT GCNGGGGGNG 420
GAANCCANTT TTTTANATGG NAATCCTTGG GTTTTTNCCN CCNTNNTNCC TGGNCCNTTC 480
CCCCAAAAAN CCTN 494

SEQ ID NO:40
SEQUENCE LENGTH:244
SEQUENCE TYPE:nucleic acid
TOPOLOGY:linear
CLONE:HUMGS00047
SEQUENCE DESCRIPTION:
GATCAGTTGA CAGTGGCAAT TAAACTGTAA ATAACTTGCC CTGGGGGCCT TTTTTTAAAA 60
AACAAAAACC ACAAAAATTC CCAAACCATA CTTGCTAAAA ATNCTGGTAA GTATGTGCTT 120
TTNTGTGGGG GTGGGATTTG GAAGGGGGGT TGGGTTGGGC TGGATATCTT TGTAGATGTG 180
GACCACCAAG GGGTTGTTGA AAACTAATTG TATTAAATGT CTTTTGATAA GCCTTCTGCT 240
CAAA 244

SEQ ID NO:41
SEQUENCE LENGTH:283
SEQUENCE TYPE:nucleic acid
TOPOLOGY:linear
CLONE:HUMGS00048
SEQUENCE DESCRIPTION:
GATCACTGTA AATGAACCTC CCTGTTGGCC GCTCTGTGGA TGANACTTTA AGACTAGTTC 60
AGGCCTTCCA GTTCACTGAC AAACATGGGG AAGTGTGCCC AGCTGGCTGG AAACCTGGCA 120
GTGATACCAT CAAGCCTGAT GTCCAAAAGA GCAAAGAATA TTTCTCCAAG CAGAAGTGAG 180
CGCTGGGCTG TTTTAGTGCC AGGCTGCGGT GGGCAGCCAT GAGAACAAAA CCTCTTCTGT 240
ATTTTTTTTT NCCATTAGTA AANCACAAGA CTTCAGATTC AAA 283

SEQ ID NO:42
SEQUENCE LENGTH:486
SEQUENCE TYPE:nucleic acid
TOPOLOGY:linear
CLONE:HUMGS00049
SEQUENCE DESCRIPTION:
GATCAGTTTT CNTTGTAACA CTGGGTTTAA TCTGAATGGC GCTGATTCTN CCAAGTGCAC 60

TGAGGAAGGA AAATGGAGCC CGGAGCTTCC TGTCTGTNCT CCCATCATCT GCCCTCCACC 120
ATCCATACCT ACGTTTGCAA CACTTCGTGT TTATAAGCCA TCAGCTGGAA ACAATTCCCT 180
CTATCGGGAC ACAGCAGTTT TTNAATGTTT GCCACAACAT GCGATGTTTG GAAATNATAC 240
AATTACCTGC ACGACACATG GAAATTGGAC TAANTTACCA GGAATGCAAG GGAAGTAAAA 300
TGCCCATTCC CATCAAGACC AGNCAATGGA TTTGTGGNAC TATCCTGCAA ANCCCAACAC 360
TNTNTTTCCA AAGGNTTAAA GGCCACATTT TGGGTTGGCC ATTGGTNGGG TTATTTNTCT 420
TGGGAGTTGG GCCCCGGANG GAANTTTGNN TNTGTTNCCN NAANCTTGGG GAACCCTTGG 480
GTTTNN 486

SEQ ID NO:43
SEQUENCE LENGTH:470
SEQUENCE TYPE:nucleic acid
TOPOLOGY:linear
CLONE:HUMGS00050
SEQUENCE DESCRIPTION:
GATCCACCAG CTGAGAATTC GTCCGCTCCC GAGGCTGAGC AGGGCGGGGC TGAGTAAATG 60
CCGGCTTACC ATCTCTACCA TCATCCGGTT TAGTCATCCA ACAAGAAGAA ATATGAAATT 120
CCAGCAATAA GAAATGAACA AAAGATTGGA GCTGAAGACC TAAAGTGCTT GCTTTTTGCC 180
CGTTGACCAG ATAAATAGAA CTATCTGCAT TATCTATGCA GCATGGGGTT TTTATTATTT 240
TTACCTAAAG ACGTCTCTTT TTGGTAATAA CAAACGTGTT TTTTAAAAAA GCCTGNGTTT 300
TTCTCAATAC GCCTTTAAAG GTTTTTAAAT TGTTTCATAT CTGGTCAAGT TGAGATTTTT 360
AAGNCCTTCA TTTTTAATTT GTAATAAAAN GTTTACCACC TTGGATTTTT TCAANAAAGG 420
TCAACCAANC TGCAANGCAC CTGTTAATAA NGGGTCTTTA ANTAATTAAA 470

SEQ ID NO:44
SEQUENCE LENGTH:479
SEQUENCE TYPE:nucleic acid
TOPOLOGY:linear
CLONE:HUMGS00051
SEQUENCE DESCRIPTION:
GATCTGTAAG TAACTTCACA TTAAAAAATG AAATATTTTT TAATTTAAAG CTTACTCTGT 60
CCATTTATCC ACAGGAAAGT GTTATTNTNA AAGGAAGGTT CATGTAGAGA AAAGCACACT 120
TGTAGGATAA GTGAAATGGA TACTACATCT TTAANCAGTA TTTCATTGCC TGTGTATGGA 180
AAANCCATTT GANGTGTACC TGTGTACATA ACTCTGTAAA ANCACTGAAA AATTATNCTA 240
ACTTATTTAT GTTAANNGAT TTTTTTTAAT CTAGACAATA TACAAGCCAA AGTGGCATGT 300
TTTGTGCATT TGTAAATNCT GTGTTGGGTA GAATAGGTTT TCCCCTCTTT TTGTTAANTA 360
ATATGGCTAT NCTTTAANGG GTTGCNTACT GGGCCAGGTN TAATTTTTTG TAATGGNGTG 420
AAAGGGTGCC ATTTTTTTGT CNCACTTNGG GGGTCTCANT GGGGGGNTTC TNNCNGGGN 479

SEQ ID NO:45
SEQUENCE LENGTH:477
SEQUENCE TYPE:nucleic acid
TOPOLOGY:linear
CLONE:HUMGS00053
SEQUENCE DESCRIPTION:

GATCTCTAAG GAACTCCTGT TGCTAAATAT GAAGAGTATG GAACATTCAT ATAGTCTCTG 60
TGAAGCATGG GGGGAGGGAA GACATTTCTT TTTCTTATAG GCTTTATGCT CAAATGTCAT 120
AGTCTCCTTT CAAAGAATTG TGTTGCATTT TAAATGCACC CAGCTTAAGT AGAAGACATT 180
GAAGGATGCA TTAATTTTCA GGAACTATTT TGAATTATGA AAAGATTCCC AATTGAAAAA 240
NTTATTCAAC AAGTAAAAGC TAAGAAATTT CATTGAAATC ATAAGGCAGT TTAAGCATAA 300
NTTGATAAAA ATAGCTGTGT ACTACTAATT AATAGAAAAT CATTCAACCA AGAGANGAGT 360
CANGTGAATA TCGTTTGTTT ATTTGCTAGT GAGTTTCTTT GTAACGTTGG ATTTTATTAA 420
NTGGTTAATN TTTGGTTAGG TATGTCCTAT GTTANTNAAA ANTGGNCCAA NTTTAAA 477

SEQ ID NO:46
SEQUENCE LENGTH:476
SEQUENCE TYPE:nucleic acid
TOPOLOGY:linear
CLONE:HUMGS00055
SEQUENCE DESCRIPTION:
GATCACCATT AGCAAATGGA AATNACATTT GAAAGCCATT AGACTTATAG GTGATGCAAG 60
CATCTAAGAG AGAGGTTAAT CACACTATAG AGGCATAAGT GGTATCAGTT TTCATTTTCN 120
TAATTGTTTA ANCTGTGTTT TATACCAGTN TTTGCAAGTA ATTGGGTGTT AGCTTGAGAT 180
GGTTAAAGGT GGTTTGGGGA GGGACTTCGT TGTAATGGTT TTCCTGTAAA ANATGTTTCC 240
AACTCCNCTG AAATGTTGCT GAAAAGCATG GTGCTGGTAA CAGTTCAACA ATCCCGTGGC 300
TGCTCATTCT TGGCCTACTT TTACTCTCCC ACTTGNNAGC AGGTTAGCGT TTGAAGGGTG 360
GTATGGGAAA AGCCTNGCAT TGCCTGGGCC AAATTCCTTT TGGGGTTCTN CTCCNTTCCC 420
CNCTTCNCCN TNTCCTTCCT TTCCCCCNTN ANGTCCNNCC NCTTNCNTTA GGTTTN 476

SEQ ID NO:47
SEQUENCE LENGTH:472
SEQUENCE TYPE:nucleic acid
TOPOLOGY:linear
CLONE:HUMGS00056
SEQUENCE DESCRIPTION:
GATCAAATCT GCACTGTGTC TACATATAGG AAAGGTCCTG GTGTGTGCTA ATGTTCCCAA 60
TGCAGGACTT GAGGAAGAGC TCTGTTATAT GTTTCCATTT CTCTTTATCA AAGATAACCA 120
AACCTTATGG CCCTTATAAC AATGGAGGCA CTGGCTGCCT CTTAATTTTC AATCATGGAC 180
CTAAAGAAGT ACTCTGAAGG GTCTCAACAA TGCCAGGTGG GGACAGATAT ACTCAGAGAT 240
TATCCAGGTC TGCCTCCCAG CGAGCCTGGA GTACACCAGA CCCTCCTAGA GAAATCTGTT 300
ATAATTTACC ACCCACTTAT CCACCTTTAA ACTTGGGGAA GGNNGCNTTT CAAATTAAAT 360
TTAATCNTNG GGGGNTTTTA AACTTTAACC CTTTTNCCNT TNTNGGGGTN GGNANTTGNC 420
CCCNTTAAAG GGGGNNCCCC TNCNNGGGGG AATAAAACAA NTTNNTTTTT TN 472

SEQ ID NO:48
SEQUENCE LENGTH:472
SEQUENCE TYPE:nucleic acid
TOPOLOGY:linear
CLONE:HUMGS00057
SEQUENCE DESCRIPTION:

GATCAAANCT GCACTGTGTC TACANATAGG AAAGGTCCTG GTGTGTGCTA ANGTTCCCAA 60
TGCAGGACTT GAGGAAGAGC TCTGTTATAT GTTTCCATTT CTCTTTATCA AAGATAACCA 120
AACCTTATGG CCCNTATAAC AATGGAGGCA CTGGCTGCCT CTTAATTTTC AATCATGGAC 180
CTAAAGNNGT ACTCTGAAGG GTCTCAACAA TGCCAGGTGG GGACAGATAT ACTCAGAGAT 240
TATCCAGGTC TGCCTCCCAG CGAGCCTGGA GTACACCAGA CCCTCCTAGA GAAATCTGTT 300
NTANTTTAGC AACCCAGTTA TCCNCNTTAA NNCTGNGGAG AGTGGTCTTT ACATCTTAAT 360
TTTATTCNTG TGGTGGTTNT TACCTTTAAC CCGGTTTCTT ATTTTTGGGT TTGTTATTGG 420
CCCTTTTTAG GGGTGGTCCC TNTTCCNGGT TGGNTTTCCC TTTTTTTGTG TN 472

SEQ ID NO:49
SEQUENCE LENGTH:319
SEQUENCE TYPE:nucleic acid
TOPOLOGY:linear
CLONE:HUMGS00060
SEQUENCE DESCRIPTION:
GATCTCATGG TCCGGAATGA CACCCCCTGT GGAACCACCA TTGGACCTAT CTTGGCTTCT 60
CGGCTGGGGC TGCGGGTGCT GGATTTAGGC AGCCCNNAAC TGGCCATGCA CTCTATCCGG 120
GAGATGGCCT GCACCACAGG AGTCCTCCAG ACCCTCACCC TCTTCAAGGG CTTCTTTGAG 180
CTGTTCCCTT CTCTAAGCCA TAATCTCTTA GTGGATTGAG CCCTCTTGGA AAGACTTCTC 240
TGCCATCCCT TTGCACCTGA GAGGGGAAGT TCTCAGCTGA GCTGAAGCTG GATTATTAAA 300
GTGGATTGTC ACTCAGAAA 319

SEQ ID NO:50
SEQUENCE LENGTH:461
SEQUENCE TYPE:nucleic acid
TOPOLOGY:linear
CLONE:HUMGS00061
SEQUENCE DESCRIPTION:
GATCTTCCTC TAATTGACAG CCTCATTACG GGTCTTACAA AATATGGAAC AGTGTCAGAA 60
AAAACCAGAG AACTCGGCAG AGTCTAACAC AGAGGAAACC TAAAAGGACT GATTTAACCC 120
AAGATGATTT CCACTTGAAA ATCTTAAAGG ATATTTTATG TGAATTTCTT TCTAATATTT 180
TTCAGGCATT AACAAAGGAG ACGGTGGCTC AGGGAGTAAA GGAAGGCCAG TTGAGCAAAC 240
AGAAGTGTTC CTCTGCATTT CAAAACCTTC TTCCTTTCTA TAGCCCTGTG GTGGAAGATT 300
TTATTGAAAA TCCTACGGTG AAGTTGATAA GGCGCTTTGC TGGATGGGCT TGGGATAAAA 360
ACCCTTTCCC AAGTTTTAAA GGGTTTCAGG TCTTTAAATC CCTGAAATTT TGGGATTTCT 420
TTCTTGTTCC AGGTTGTTTA AACCTTTTAT TTTTTCCTCC N 461

SEQ ID NO:51
SEQUENCE LENGTH:458
SEQUENCE TYPE:nucleic acid
TOPOLOGY:linear
CLONE:HUMGS00062
SEQUENCE DESCRIPTION:
GATCTAGCAT ATTTCACTAT TCTGTGGATG AATACATAGT TTGTGGGGAA AACAAACGTT 60
CAGCTAGGGG CAAAAAGCAT GACTGCTTTT CCCTGTCTGG CATGGAATCA CGCAGTCACC 120

TTGGGCATTT AGTTTACTAG AAATNCTTTA CCTTAAGCAG CACACACATT TACTACACAC 180
ACAGNCCTAA CAAAGCACTG TGCTTAGAGG GTAAAAAGGA ATCACAAAAC AAGAATCTTT 240
CCAAAGTTGT CTCATTCAGC AATGTTAAGG CATCTGTATC AAATTATTTT GGATGTAAAG 300
ATTCCTGTGT CTCATAATAT GAATGTATTT TTTGATATAC AAGGAAACTG GCCATAAAAA 360
TGGTGNGGNA ANCCGCCCNN TAATTTTNCC CCTGGGGCCC CAATTGGTNN NNTCNANTCT 420
NGGNTTNAGC NTTTGTCCTC AAATGGGATN CANTTNNN 458

SEQ ID NO:52
SEQUENCE LENGTH:459
SEQUENCE TYPE:nucleic acid
TOPOLOGY:linear
CLONE:HUMGS00064
SEQUENCE DESCRIPTION:
GATCTATCAC CCAAACATCG ACGAAAAGGG GCAGGTCTGT CTGCCAGTAA TNAGTGCCGA 60
AAACTGGAAG CCAGCAACCA AAACCGACCA AGTAATCCAG TCCCTCATAG CACTGGTGAA 120
TNACCCCCAG CCTGAGCACC CGCTTCGGGC TGACCTAGCT GAAGAATACT CTAAGGACCG 180
TAAAAAATTC TGTAAGAATG CTGAAGAGTT TACAAAGAAA TATGGGGAAA AGCGACCTGT 240
GGACTAAAAT CTGCCACGAT TGGTTCCAGC AAGTGTGAGC AGAGACCCCG TGCAGTGCAT 300
TCAGACACCC CGCAAAGCAG GACTCTGTGG AAATTGGCAC GTGCCACCGN CTGGCGTTCG 360
NTTGTGGCAG TTACTAACTT TTCTACAGTT TTCTTAATCA AAAGTGGTCT TAGGTAANCC 420
TGTAAAGGNA AGGGGTTTAN NAATTTTANG GTTGGTCTN 459

SEQ ID NO:53
SEQUENCE LENGTH:458
SEQUENCE TYPE:nucleic acid
TOPOLOGY:linear
CLONE:HUMGS00065
SEQUENCE DESCRIPTION:
GATCAGCTGG CCAACTCAGC CAATCCGCAC ATTTAGCTCT CCAACTACCA TACAACGTGC 60
TTGGTTTAGG TCGGAGCGCA AATNTNCTTG ACCATCTCTA CGTTGGTATT CCCCGTCCAT 120
CTGGAGAAAA ATCTATACGA AANCAAGAGT GGACTGCAAT NATTCCAAAT NCCCAGCTAA 180
TTGTCATTCC ATACCCTCAC AATGTCCCTC GAAGTTGGAG TGCCAAACTG TATCTTACAC 240
CAAGTAATAT TGTNCTGCTT ACTGCTATAG CTCTCATCGG TGTCTGTGTT TTCAATCTTT 300
GGCAATAAAT TGGCATTTTA CCATTTGNCA GGGAAAAGGA AAGGCNGGTT GGNTTGGGGG 360
GAAAACCGGC CAAGGGANGG CCCCCCCGGG TTTTCNATTT TTGGNGGGNT TNTTGGGNNT 420
TTTGCCTTTT TAAANTTTTT CCNAANAAAN NGGGANTN 458

SEQ ID NO:54
SEQUENCE LENGTH:454
SEQUENCE TYPE:nucleic acid
TOPOLOGY:linear
CLONE:HUMGS00066
SEQUENCE DESCRIPTION:
GATCAGTTNC GTGTCCGCNG GAGCAGGCCT TGCTGAGTGA AGACACTGGN ACTAGCTGGG 60
TCCTGGGGTG ACTTGGAGGC TTTGGGCCTA AAAGGGCAGC CTGAACCTGG AGTCTTATCT 120

CCCCCAGGAG CCGAAAGCAC TTTTCTTGAT TTCCCCCAGG AAATCAAGCG CTGCTTCTCA 180
GCTCCTGTGG TTTTAGTATT TATATATCTG TATCTTCTTT GTAGAAATTT ATTTATTTTT 240
GAATAAGAAT ACCTGCCTGG ACAAAATTTA AAAGGACGGG AGGGCGAANT GCAAGGGAAG 300
GCCTCTCCTA TGCCGNCCCA GAGNAGCACT GTACCAATTT CATGTGATTC CTTAACTCTG 360
TTTAAGGAAG CTCTGAAACT GTCATTTCCT TTGCAGATTG TTNTGAACCT GGAAACCCNG 420
AATTTATNGN TAANNCTCAN TTNCCACCNG GAAA 454

SEQ ID NO:55
SEQUENCE LENGTH:505
SEQUENCE TYPE:nucleic acid
TOPOLOGY:linear
CLONE:HUMGS00067
SEQUENCE DESCRIPTION:
GATCGTTGGG GAACCCAGCC CCTTGGAACT TGGAAGACCC GTGTTTCCTG GACCGCGAAT 60
CAGTGTGTTG GGCATCAGTG TTTTCTGCAA GGGTTGTGAC CTGAAACTTT TTAAAAACCA 120
CCCACCTTTG GGGAAGCATT TCTGAATTTA TCCATCACCA ACCATTTCTT CTTGGATACC 180
ATCAAGTAAC AGCTATTATT TGCCAAGTGG AGCTGTCATT TAATTTGATG CACCTCTGGN 240
TTCAGATGAA ACATTAAATT GTCTTCCTCG ATTCTCCATC GGGTGTAGAG TTTTTAAACT 300
ATCANTGGCA TTTCAAGTCT TCTGANACAA CATGGCTGTA TGTGCGTGGT CCATAGCACA 360
GTACATGCAG CATCTAATAA GNGTTTCCAT TTGTAGAATT NTTTTCNNCA NACTTNTAGT 420
TAAANNCAAA ATTTTTTAAT TTGNAAANAA GNGNTGNGTT GGTTATTTNN GNTGTTNTTT 480
GTNTNTGGTT GNTNTGTTTA TTNTN 505

SEQ ID NO:56
SEQUENCE LENGTH:450
SEQUENCE TYPE:nucleic acid
TOPOLOGY:linear
CLONE:HUMGS00068
SEQUENCE DESCRIPTION:
GATCAGAGAC TGGAGAGGTG GAGTGAGAAG TCTCCGCTGC TCGGGCCCTC CTGGGGAGCC 60
CCCGCTCCAG GGCTCGCTCC AGGACCTTCT TCACAAGATG ACTTGCTCGC TGTTACCTGC 120
TTCCCCAGTC TTTTCTGAAA AACTACAAAT TAGGGTGGGA AAAGCTCTGT ATTGAGAAGG 180
GTCATATTTG CTTTCTAGGA GGTTTGTTGT TTTGCCTGTT AGTTTTGAGG AGCAGGAAGC 240
TCATGGGGGC TTCTGTAGCC CCTCTCAAAA GGAGTCTTTA TTCTGAGAAT TTGAAGCTGA 300
AACCTCTTTA AATCTTCAGA ATGATTTTAT TGAAGAGGGC CGCAAGCCCC AAATGGAAAA 360
CTGTTTTTAG AAAATATGAT GATTTTTGAT TGCTTTTGTA TTTAATTCTG CAGGTGTTCA 420
AGTCTTAAAA AATAANGATT TNTANCAGGN 450

SEQ ID NO:57
SEQUENCE LENGTH:447
SEQUENCE TYPE:nucleic acid
TOPOLOGY:linear
CLONE:HUMGS00069
SEQUENCE DESCRIPTION:
GATCTTTGAT AGTNGAGAAA ATTATGCAAA GTTCCTCAGA AGTNGGTTAT NATGCTATGG 60

CTGGNGATTT TGTGAATATG GTGGAAAAAG GAATCATTGA CCCAACAAAG GTTGTGAGAN 120
CTGCTTTATT GGATGCTGCT GGTGTGGCCT CTCTGTTAAC TACAGCAGAA GTTGTAGTCA 180
CAGAAATTCC TAAAGANGAG AAGGACCCTG GAATGGGTGC AATGGGTGGA ATGGGAGGTG 240
GTATGGGAGG TGGCATGTNC TAACTCCTAG ACTAGTGCTT TACCTTTATT AATGANCTGT 300
GACAGGAAGC CCAAGGCAGT GTTCCTCACC AATAACTTCA GAGAANGTCA GTTGGAGAAA 360
AATGANGAAA AAGGGCTGGC TTGAAANTCA CTNTTAACCN NTTANGGTTG CTTGGGTTTC 420
ANGTTGGCCA NAGTTTTNNN TNNTGGN 447

SEQ ID NO:58
SEQUENCE LENGTH:445
SEQUENCE TYPE:nucleic acid
TOPOLOGY:linear
CLONE:HUMGS00070
SEQUENCE DESCRIPTION:
GATCACTGAG CAGTTTTCCC AGAGCTCCAT GGGAAGGCAA GCTCTCCCTC CCAATGGGAG 60
CCCCACTGTC ACTAACTGTA AACTCAGGCT CAGGCTTCAN CTGCCTACCC CCATCCTCAT 120
ATTTCTGTCT GTCCCAGCAC CTCAGGAGCA TTCTCATTGT GGCCGGCTAA CTCCGCCTGG 180
ATGTGAACAG GCAAGCACAG TGGGAAATNA GTCACGTACT TGTATTGCAC AGTGGACACC 240
TCTAGAGGTC CATTGGTTTA AAGGGATAGG GAAGGAGGAG GGATGAGACC ATCTCCCCCT 300
CCCAGGAAGT AAATCTAAGT ATCTAAGGTT TTCTTTATNG CCTTNGAGTC AAACTANTAA 360
CTGGCTAGTA CGGGAGGTGT NTGCTNGGTT TNTTTCGGGT GGTTTTTTCC TAATGNAATA 420
AACTTCATTT NTTGCNTGNT TGGNN 445

SEQ ID NO:59
SEQUENCE LENGTH:459
SEQUENCE TYPE:nucleic acid
TOPOLOGY:linear
CLONE:HUMGS00071
SEQUENCE DESCRIPTION:
GATCTTCGGT GGCCTCATGT AAACGTGGCA GCCAGCCTCT TCTAGAACCC TAGCCCAGGG 60
ACTGGAGCAG GAAAGGGACC TTCAAAGTGA AGACTGCCTT GTCCCGCAGC TCCTTCTGGC 120
TTAGATTGAA ACATGGGCTT CCTAATGGGT TAAATCCTTT AAAACAAGGA GTTGTGGGGG 180
AAGGGTGTCG TGCACTCCTA GAGAAAGGTA CACAGTTGCC CGGTTGGGAA TGTGCTTGGC 240
GCTGACCCTG CGGGCATCTG ACTGGTCTTC CAGCTCAGGA AAAAGAATTT GAAAGAGGCT 300
TAGCGTGAAG GGGAATCAAA GAGGAGGTTG TNATTTNGGT CGAAGGTGCC TTGGTTTAAG 360
TCCTNGTAAT TTGTNCTTAT TAATTTTTTT TNATATAATA TNATTTTNTT GGGGGGTAAA 420
CCATTTTTAA ATTAAACCAA CCATTTGTCT TNCTNGAAA 459

SEQ ID NO:60
SEQUENCE LENGTH:441
SEQUENCE TYPE:nucleic acid
TOPOLOGY:linear
CLONE:HUMGS00072
SEQUENCE DESCRIPTION:
GATCAGACAC TTCAAGGTCT AGGCTAGACA TGGCAGAGAT GAGGAGGTTT GGCACAGAAA 60

ACATAGCCAC CATTTTTTCC AAGCCTGGGC ATGGGTGGGG GGCCTTGTCT GCTGGCCACG 120
CAAGTTCACA TGCNATCTAC ATTAATATCA AGTCTTGACT CCCTACTTCC CGTCATTCCT 180
CACAGGACAG AAGCAGAGTG GGTGGTGGTT ATGTTTGACA GAAGGCATTA GGTTGACAAC 240
TTGTCATGAT TTTNACGGTA AGCCACCATG ATTGTTTTCT CTGGCCTCTG GGTTGACCTT 300
ACAAAAACCC ATTTGGAACT TGNNGACTTT GAAANGGTGC TCTTTGCTTA AGGCTTTNAT 360
ATNGNGCCTT GTTTAATTGG GANGGTCNCT TNAAAAGGCC NTTTCCTTTA NTTAANGGNG 420
GGGTTNTTAN GGNTGTAGAA A 441

SEQ ID NO:61
SEQUENCE LENGTH:436
SEQUENCE TYPE:nucleic acid
TOPOLOGY:linear
CLONE:HUMGS00073
SEQUENCE DESCRIPTION:
GATCCGTCAC TCTTCCTTGT GGTAATCCCT AGACTGGGAG CTCAGGTACT CTTTTAGTCA 60
TCTTTGTATG TCTTTAGCAG AGTTCTTGAC ATGTGGTAGG TGCTTAATAA ATNTTTGTTG 120
TTTATCAAAT TTTATGGTAG GGAGAGTAAG TCAGCATCGG TATAAAATCG CTTACTCCAC 180
GTAACTCTTC TTCTGATAGG GTTTGATTTT CTATTAGAAG CTCAATTTTA GTTTTTTTTC 240
ATATTATAAC TAAATATGTT TCCTGAGAGA TAAGAGAAAT AATGTTCCTA CAATAGTTGT 300
ATGTATCTAA GATAAGACAT ATAGATGCTT AAGACATTTT GTTTCACTTG CTATTCACTA 360
GTGTACTTGA ACCATGGTCA TTTTTAGCCC TTTTCCTAGG GACCATGCTT ATTTCTCAAT 420
AAGGAAATAC CTTCCN 436

SEQ ID NO:62
SEQUENCE LENGTH:434
SEQUENCE TYPE:nucleic acid
TOPOLOGY:linear
CLONE:HUMGS00074
SEQUENCE DESCRIPTION:
GATCTTTTTC CATCCAGCAG TGGAGTTTAG TACTTAAGAG TTTGTNCCCT TAAACCAGAC 60
TCCCTGGATT AATGCTGTGT ACCCGTGGGC AAGGTGCCTG AATTCTCTAT ACACCTATTT 120
CCTCATCTGT AAAATGGCAA TAATAGTAAT AGTACCTAAT GTGTGGGGTT GTTATAAGCA 180
TTGAGTAAGA TAAATANTAT AAAGCACTTA GAACAGTGCC TGGANCATAA AAACACTTAN 240
TAATAGCTCA TAGCTAACAT TTCCTATTTA CANTTCTTCT AGAAATAGCC AGTATTTTGT 300
TGGAGTGCCT ACNATGTTAG TTCCTTNTAC TAGTTGCTTT ACATGGATTA TCTTNATATC 360
CTGTTTTAAA GNTTNTTCAC AGGTACCAGG TTTTCATGGA ATTTTCCTTT NANTAAANGG 420
GGAGGNNAAN GNTN 434

SEQ ID NO:63
SEQUENCE LENGTH:433
SEQUENCE TYPE:nucleic acid
TOPOLOGY:linear
CLONE:HUMGS00075
SEQUENCE DESCRIPTION:
GATCTGTGAA TCTTGGCTGG GACTTCCTCT GAGTGATGCC TGAGGGTCAG CTCCTCTAGA 60

CATTGACTGC AAGAGAATCT CTGCAACCTC CTATATAAAA GCATTTCTGT TAATTCATTC 120
AGAATCCATT CTTTACAATA TGCAGTGAGA TGGGCTTAAG TTTGGGCTAG AGTTTGACTT 180
TATGAAGGAG GTCATTGAAA AAGAGAACAG TGACGTAGGC AAATGTTTCA AGCACTTTAG 240
AAACAGTACT TTTCCTATAA TTAGTTGATA TACTAATGAG AAAATATACT AGCCTGGCCA 300
TGCCAATAAG GTTCCTGCTG TGTCTGGTTA GGCAGCATTN CTTTTGATGC AAATTTCCTA 360
TTGGNCCCTN TNTTNNTCCA AAAAGGTAAA TGNCTTNNAT TNCCCGGTTA AAAAATNNTT 420
CCCNGGNNAT TTN 433

SEQ ID NO:64
SEQUENCE LENGTH:432
SEQUENCE TYPE:nucleic acid
TOPOLOGY:linear
CLONE:HUMGS00076
SEQUENCE DESCRIPTION:
GATCCTGGAG GTCTTTTCTA GTCTGAGCTT CTTTAGCTAG GCTAAAACAC CTTGGCTTGT 60
TATTGCCTCT ACTTTGATTC TNATAATGCT CACTTGGTCC TACCTATNAT CCTTCTACTT 120
GTCCAGTTCA AATAAGAAAT AAGGACAAGC CTAACTTCAT AGAAACCTCT CTATTTTTAA 180
TCAGTTGTTT AATAATTTAC AGGTTCTTAG GCTCCATCCT GTTTGTATGA AATTATAATC 240
TGTGGATTGG CCTTTAAGCC TGCATTCTTA ACAAACTCTT CAGTTAATTC TTAGATNCAC 300
TAAAANTCTG AGGAACTCTA CATGTAACTA TTTCTTCAGA GTTTGTCATA TACTGNTTGG 360
CATCTGAATG GCTACTCAGC ATTTGGTTAA CATTNGNGTA AATTTGGAAT AAANTTCCCC 420
AGTAAGCCAT TN 432

SEQ ID NO:65
SEQUENCE LENGTH:459
SEQUENCE TYPE:nucleic acid
TOPOLOGY:linear
CLONE:HUMGS00077
SEQUENCE DESCRIPTION:
GATCCATCGC AGAGTCCTAA AGAAGAACCC ACTGAAAAAC TTGAGAATCA TGTTGAAGCT 60
AAACCCATAT GCAAAGACCA TGCGCCGGAA CACCATTCTT CGCCAGGCCA GGAATCACAA 120
GCTCCGGGTG GATAAGGCAG CTGCTGCAGC ANCGGCACTA CAAGCCAAAT CAGATGAGAA 180
GGCGGCGGTT GCAGGCAAGA AGCCTGTGGT AGGTAAGAAA GGAAAGAAGG CTGCTGTTGG 240
TGTTAAGAAG CAGAAGAAGC CTCTGGTGGG AAAAAAGGCA GCAGCTACCA AGAAACCAGC 300
CCCTGAAAAG AAGCCTGCAG AGAAGAAACC TACTACAGAG GAGAAGAAGC CTGCTGCATA 360
AACTCTTAAA TTTGNTTATT CCATAAAGGT CAAATCATTT TGGNCAGCTT CTTTTTTGAA 420
TAAAAGNCCT GNTTTATACC AGGGCAGTGA GGAACCAAA 459

SEQ ID NO:66
SEQUENCE LENGTH:626
SEQUENCE TYPE:nucleic acid
TOPOLOGY:linear
CLONE:HUMGS00078
SEQUENCE DESCRIPTION:
GATCTACAAA GGCCATGGGA AAAATTCAGA GAGTTAGGAA GGAAAAACCA ATAGCTTTAA 60

AACCTGTGTG CCATTTTAAG AGTTACTTAA TGTTTGGTAA CTTTNATGCC TTCACTTTAC 120
AAATTCANGC CTTAGATAAA AGAACCGAGC ANTTTTNTGC TAAAAAGTCC TTGATTTAGC 180
ACTATTTACA TACAGGCCAT ACTTTACAAA GTATTTGCTG AATGGGGACC TTTTGAGTTG 240
AATTTATTTT ATTATNCCCG TTTNGTTTAA TGTCTGGTGC TNNCTATCAC CTCTTCTAAT 300
CTTTTAATGT ATTTGTTTGC AATTTTGGGG TAAGACTTTT TTATGAGTAC TTTTTCTTTG 360
AAGTTTTAGC GGTCAATTTG CCTTTTTAAT GANCATGTGA AGTTATACTG TGGGCTATGC 420
ACCAGCTCTC ACCTACNGGG GGNCTTACCT TGGGGGTAGN GNCCATACCA GNCCACTGTA 480
TGTTTACTTC CTCACCCATT TGGNGTTGCC CCANCTTGGT TNAACACTNG GGCANCATTN 540
TGGTTTNAGG GGNCCTTAGG GTTNACCAGN TCNTTTTAAC NGGNTATTTN CCCGGGGTTT 600
TTTNAAANTG GCCCAAAATN CTTAAA 626

SEQ ID NO:67
SEQUENCE LENGTH:534
SEQUENCE TYPE:nucleic acid
TOPOLOGY:linear
CLONE:HUMGS00079
SEQUENCE DESCRIPTION:
GATCAACAGT TCTAGTACTC TTCTTTGTCA GTATATCAAC CTACAGCTAT TGAATGCAAA 60
GCCACAAGAG TGTTTAATGG GGACAGTGGG CACTCTCCTG CTTGAAAACC CACTTGGGCA 120
GAATGGACTC ACCCACCAAG GTCTTCTGTA TGAAGCAGCC AAGGTGTTTG GCCTTCGGAG 180
CAGGAAGCTA AAGCTGTTTC TGAATGAGAC CCAAACGCAG GAAATTACAG AAGACATCCC 240
CGTGAAGACT TTGAATATGA AGACTGTGTA TGTTTCTGTG TTACCAACAA CAGCAGACTT 300
CTAGCATGTA CTTATCAATG TTGTTCGGTC AGCCCTTCCC TAATTACACC TATCCCCTAC 360
ACATACATGC ACATAGNCAC ACACATGNAC ACACTTGAAG GTATTTCCTT CAAGGTGTGT 420
GTAAAAATAT GCTGCTTGGN TTTGAATTCA AATGGGGTTG NTTAGGTCAA GTACTTTGNG 480
GCCTNANAGG NATCTTCACA CTTAACCTTA GGCACTTTTG ANGCATTGTT GGGN 534

SEQ ID NO:68
SEQUENCE LENGTH:417
SEQUENCE TYPE:nucleic acid
TOPOLOGY:linear
CLONE:HUMGS00080
SEQUENCE DESCRIPTION:
GATCTTAGTT GATATTTTGG GCTTGGGGCA GTGAGGGCTT AGGACACCCC AAGTGGTTTG 60
GNAAAGNAGG AGGGGAGTGG TGGGTTTATA GGGGGAGGAG GAGGCAGGTG GTCTAAGTCC 120
TGACTGGCTA CGTAGTTCNG GGCAAATCCT CCAAAAGGGA AAGGGAGGAT TTCCTTAGAA 180
GGATGGCGCT CCCAGTGACT ACTTTTTGAC TTCTGTTTGT NTTACGCTTC TCTCAGGGAA 240
AAACATGCAG GTCCTCTAGT GTTTCATGTA CATNCTGTNG GGGGGTGACA CCTTGGTTCT 300
GGTTAAACAA GCTGTACTTT TAATAGCTGT TNCAGGAAGG GTTAAGGCCA ACTACAAATT 360
AATGTTGGTT GCAAATGTAG TGTGGTTCCC TAACTTTNCG GGGTTTTCCT GAGGAAA 417

SEQ ID NO:69
SEQUENCE LENGTH:417
SEQUENCE TYPE:nucleic acid
TOPOLOGY:linear

CLONE:HUMGS00081
SEQUENCE DESCRIPTION:
GATCTGCCTG CCCACCAACT GGTGATGGAA GGTTACAAGT GGCACTTCAA TGAGACGGTG 60
CTCACTGTGT GGTCGGCACC AACTACTGCT ACCGTGTGGG AATGTGGCAG CATCTTGGAG 120
CTGGACGAGC ATCTCCAGAA AGATTTCATC ATCTTTGAGC TGCTCCCAAG AGCACGGGGC 180
ATCCCTCCAA GAGCCGTGCC GCTATTCCTG TGACCCGCCG GCCTGCCCTC ACCTTTGGCT 240
CGACATGTGC TTGCATTTCT AGCGAGCTGG CGTGGGGGCT GTCTGGTTGT GTCCCAAGAG 300
GTGTTGAGGT AGGTNTTGAG AGCTGAGACT AGTCATGTCT CTCTTTCCAT TACATGAGTT 360
CATATTTTTN TTTTCTNTTT TGTGTTAGTA ATTTGGAAAT GAAATTATAA GGAATGN 417

SEQ ID NO:70
SEQUENCE LENGTH:415
SEQUENCE TYPE:nucleic acid
TOPOLOGY:linear
CLONE:HUMGS00082
SEQUENCE DESCRIPTION:
GATCTTTCTG GGAACACAGC CCNGCTGGCG GCTAACCTGC TGTTTGAGAT GCTATGTGCT 60
CTCCCCAAAG TGACAACCGT CTGAGTCTTG TGCTCTTCAA GACAAAACAG ATTGCGTCGC 120
TGACAAGTTC TCAAGAAGAA CTTATGAGTA AGCAGTCTGA GAACTAAAGA GTTTATGCCA 180
AGAAAACTTT CTGCTGAAAG TGTCATTGCT GGCTGTGAAG TCGGGATAAT CAGTAGAATT 240
CTCACCCAAA CAGCAACATT TCTAAGGAAC TTGGATTAAT TGGGGGAAAA AAAANGGGGT 300
ACTTGTACTG CTTTGATTTG TTTTCCTTTG GNTGAAAAGN TGGGGGGTTA AANGGGGGAT 360
NGTGAGGGGG ANTTTNCTTN TNNAGGGNTT TNTTNTNANC CCATTTNGGN NTNCN 415

SEQ ID NO:71
SEQUENCE LENGTH:415
SEQUENCE TYPE:nucleic acid
TOPOLOGY:linear
CLONE:HUMGS00083
SEQUENCE DESCRIPTION:
GATCAGAATC ATTAAAAAAT ATTTTTGTTT AGTAAGTTTG AAGATTTCNN GCTTTNAGGC 60
CTTTCCTATT TTGTCCCATT TATTTTTNCA GGCAATCTTT TCCATGGAGG GCAGGGTATC 120
CATTCTTTAC CATGGGTGTA CCTGCTTAGG TTAAAAATCA TACCAAGGCC TCATACTTCC 180
AGGTTTCATG TTGCGTCTTG TTGAGGGAGG GAGAGCAGGT TACTTGGCAA CCATATTGTC 240
ACCTGTNCCT GTCACACATC TTGAAAAATA AAACGATAAT AGANCTAGTG ACTAATTTNC 300
CCTTACAGTT CCTGCTTGGN CCCACCCNAC TGNGGGTNGG CTCCATTGGT NNGTTCCGGG 360
GCCGTNNTTT AGGGGGNANT TGGGGGNTCG GTTAGGCCTN TNGGTTTGGG GAAAN 415

SEQ ID NO:72
SEQUENCE LENGTH:410
SEQUENCE TYPE:nucleic acid
TOPOLOGY:linear
CLONE:HUMGS00084
SEQUENCE DESCRIPTION:
GATCTCCCCN CTCTAGGGGT CAGGCTCCAT TAGGATTTGC CCCTTCCCAN CTCTTCCTAC 60

CCAACCACTC AAATNAATCT TTCTTTACCT GAGACCAGTT GGGAGCACTG GAGTGCAGGG 120
AGGAGAGGGG AAGGGCCAGT CTGGGCTGCC GGGTTCTAGT CTCCTTTGCA CTGAGGGCCA 180
CACTATTACC ATGAGAAGAG GGCCTGTGGG AGCCTGCAAA CTCACTGCTC AAGAAGACAT 240
GGAGACTCCT GCCCTGTTGT GTATAGATGC AAGATATTTA TATATATTTT TGGTTGTCAA 300
TATTAAATAC AGACACTAAG TTATAGTATA TCTGGACAAG CCAACTTGTA AATACACCAC 360
CTCACTCCTG TTACTTACCT AAACAGATAT AAATGGCTGG TTTTTAGAAA 410

SEQ ID NO:73
SEQUENCE LENGTH:406
SEQUENCE TYPE:nucleic acid
TOPOLOGY:linear
CLONE:HUMGS00085
SEQUENCE DESCRIPTION:
GATCGTGACG CTGAATAAAT GTCTTTTTTT TAATGTGCTG TGTAAAGTTA GTCTACTCTT 60
AAGCCATCTT GGTAAATTTC CCCAACAGTG TGAAGTTAGA ATTCCTTCAG GGTGATGCCA 120
GGTTCTATTT GGAATTTATA TACAACCNGC TTGGGTGGAG AAGCCATTGT CTTCGGAAAC 180
CTTGGTGTAG TTGAACTGAT AGTTACTGTT GTGACCTGAA GTTCACCATT AAAAGGGATT 240
ACCCAAGCAA AATCATGGAA TGGTTATAAA AGTGATTGTT GGCACATCCT ATGCAATATA 300
TCTAAATTGA ATAATGGTAC CAGATAAANT TATAGATGGG AATGAAGCTT GTGTATCCAT 360
TATCATGNGT AATCAATAAA CGGNTTNAAT TCNCTTGGAN TGGAAA 406

SEQ ID NO:74
SEQUENCE LENGTH:408
SEQUENCE TYPE:nucleic acid
TOPOLOGY:linear
CLONE:HUMGS00086
SEQUENCE DESCRIPTION:
GATCACATTG TAAAACTATG GATGGTCTGA TAAGGCTTTN ACTGACCCCA CTGACTTCAG 60
AGTTATACTC TGTTTGCNAC ATCATAATGC TGGTTTTCCT GACTTTTTGT NTTTTAATAT 120
ATTTATAAAA AAAGAAAAAG TTGGTGATTG CATTGGGAAA TTCCCAGGGT ATTACTGGAC 180
CTATGTGGTG TATTGTTAAA CCAGTGTCCT TGTNATACTG TTGCTCTTGA TGTTCCTGAT 240
ACAGGTAAGG ANGCAGTTGG TCAACTCTNA TACAAAGTAT ATATACAGTT CAGTATTGTC 300
TCTGTTCATT TTGTTTTAAT TTCATTGGNC AAANTCAANC CAGCATTCCC CATTTGTGTA 360
AATAAATGAT TTTCCTGGAA TAAAAGGNAA AGGNCTTNAA ATTCCAAA 408

SEQ ID NO:75
SEQUENCE LENGTH:407
SEQUENCE TYPE:nucleic acid
TOPOLOGY:linear
CLONE:HUMGS00087
SEQUENCE DESCRIPTION:
GATCAAACTA GCTCAGGCCA AACTTTAAGT TCATACCTGA GCTAAGAAGG ATAATTGTCT 60
TTTGGTAACT AGGTCTACAG GTTTNCATTT TTCTGTGTTA CACTCAAGGA TAAAGGCAAA 120
ATCAATTTTG TAATTTGTTT AGAAGCCAGA GTTTATCTTT NCTATAAGTT TACAGCCTTT 180
TNCTTATATA TACAGTTATT GCCACCTTTG TGAACATGGC AAGGGACTTT TTTACAATTT 240

TNATTTTATT TTCTAGGTAC CAGCCTAGGG GATTTCGGGT TAGGTACTCA TTTTGTATTC 300
ACTGTCACTT TTTCCTCATG GTCCTAATTA TAAATNGNCC CAAAATCAAG GNTTGCCTNA 360
AAAAGGGGGN AAAATGGTTG GCCCCNNGGT TNTTNGNNNC CCCNGTN 407

SEQ ID NO:76
SEQUENCE LENGTH:413
SEQUENCE TYPE:nucleic acid
TOPOLOGY:linear
CLONE:HUMGS00088
SEQUENCE DESCRIPTION:
GATCTACCGA CTCACTTCTG AGAATATTTT TNACAGATTA TCTTTGGGCC TTTCCATTAG 60
AAAGCTGTTT GTTTGTCCCC CTGTTGGTAC ATTTGGTTAC CTCATTTTGC CGTTTCAAAT 120
TGTAAAAGCT CACAGGGGTG TTTTTTGGAA TCATTTGCTG AGTCATTTTC TCAAATCATA 180
TTCCATTGTA TCAGTTAACA TATAGTTTTA AATGTATGTA TTATAAATNT CTGTANCCAA 240
ATCATTTGAA GGCTTGATAA ATTTNTAACA ANGTTTGTAC ATTTNTCATG AAAGTCACTA 300
GTAATGCTNG GNGNGGTAGT GCAATGGANT TTTCCNTTTT TCNTCCCTGT GCCCATTTTG 360
GAGTTGAGAG GGTTGTNGGT AATNAACTGT ATGGTGTACA NTGNANCCNA NNN 413

SEQ ID NO:77
SEQUENCE LENGTH:417
SEQUENCE TYPE:nucleic acid
TOPOLOGY:linear
CLONE:HUMGS00089
SEQUENCE DESCRIPTION:
GATCGGCAAG CCNCNCACTG TCCCTTGCAA GGTGACAGGC CGCTGCGGCT CTGTNCTGGT 60
ACGCCTCATC CCTGCACCCA GGGGCACTGG CATCGTCTCC GCACCTGTGC CTAAGAAGCT 120
GCTCATGATG GCTGGTATCG ATGACTGCTA CACCTCAGCC CGGGGCTGCA CTGCCACCCT 180
GGGCAACTTC GCCAAGGNCA CCTTTGATGC CATTTCTAAG ACCTACAGCT ACCTGACCCC 240
CGACCTCTGG AAGGAGACTG TATTCACCAA GTNTCCCTAT CAGGGAGTTC ACTGACCACC 300
TCGTCAAAGA CCCACACCAG AGGTCTCCGT GCAGNGGACT TCAGGNTNCA GNTTGTGGTT 360
ACAACATAGG GGNTTTTTAT ACAANGGAAA NGTAAAGGTG NNNTTAAAGN GGTGAAA 417

SEQ ID NO:78
SEQUENCE LENGTH:404
SEQUENCE TYPE:nucleic acid
TOPOLOGY:linear
CLONE:HUMGS00090
SEQUENCE DESCRIPTION:
GATCAGAAGA AACACTCCAA AAATTGAGAT GAAATGTTGG TGCAGCCAGT TATAAGTAAT 60
ATAGTTAACA AGCAAAAAAA GTGCTGCCAC CTTTTATGAT GATTTTCTAA ATGGAGAAAC 120
ATTTGGCTGC ATCCACATAG ACCTTTATGT TTTGTTTTCA GTTGAAAACT TGCCTCCTTT 180
GGCAACATTC GTAAATNAAG CAGAATTTTT TTTTCTCTTT TTTCCAAATA TGTTAGTTTT 240
GTNCTTGTAA GATGTATCAT GGGTATTGGT GCTGTGTAAT GAACAACGAA TTTTAATTAG 300
CATGTGGTTC AGAATATNCA ATGTTAGGTT TTTAAAAAAG TATCTTGATG GTTCTTNTTC 360
TATTTATAAT TTCNGACTTT CATAANGTGT ACCCANGANT TTCN 404

SEQ ID NO:79
SEQUENCE LENGTH:622
SEQUENCE TYPE:nucleic acid
TOPOLOGY:linear
CLONE:HUMGS00091
SEQUENCE DESCRIPTION:
GATCCCCGCA ACTCGCTTGT CCTTGGGTCA CCCTGCATTC CATAGCCATG TGCTTGTCCC 60
TGTGCTCCCA CGGTTCCCAG GGGCCAGGCT GGGAGCCCAC AGCCACCCCA CTATGCCGCA 120
GCGGCCCTAC CCACCTTCAG GCAGCCTATG GGACGCAGGG CCCCATCTGT CCCTCGGTCG 180
CCGTGTGGCC AGAGTGGGTC CGTCGTCCCC AACACTCGTG CTCGCTCAGA CACTTTGGCA 240
GGATGTCTGG GGCCTCACCA GCAGGAGCGC GTGCAAGCCG GGCAGGCGGT CCACCTAGAC 300
CCACAGCCCC TCGGGAGCAC CNCACCTCTG TGTGTGATGT AGCTTTCTCT CCCTNAGCTG 360
CAAGGGTCCC GATTTTGCCA TCGGAAAAAG ACAACCTCTA CTTTTTTNCT TTTGTATTTT 420
TGATAAACAN TTGAAGNTTG GAGCNTGTTA AAATTTATTN TTTGGGGGGA AACCTNAAGA 480
ACTGGGNCTT AATTTNGGNG TTCGTGGGAC CCTNTTANNT GGTTTTNAAT NAANCGGTTA 540
NGGAATTAAA CTGTTTTGGA ANANTTGGTT TAAAGNTTTA AAATTTTGGG AAAAAAAGGG 600
GCTTTTTAAA TTTTTTGGGT TN 622

SEQ ID NO:80
SEQUENCE LENGTH:400
SEQUENCE TYPE:nucleic acid
TOPOLOGY:linear
CLONE:HUMGS00092
SEQUENCE DESCRIPTION:
GATCTTCCTT TNTTCTNTGA ATGNGCTCTG TTGNCTTTTT CTCTTTTTTC TCATGTGTTC 60
TTCCCTCCAC CTCCACCCCT TTCTTTCTTT CTCTCTCTGA TTGAGAGGCA TTNAATTACG 120
TTTTCAGTAG TACAGGCTTC TTGCCGATAT GAAGGGAACT TTTCAGAAAG AGACCTACTC 180
TGGGTCATTT AATTTTGAAT ACAGTTTTCA ATCGTTCAAG TTTTGGNNNG NTTATATCTA 240
ATGTGTGTTT CATTTTTTTG GAAAGCTATA TTTTGTATTT AGGAAATGGT ATACTATTTT 300
GCTATTTGTA CTGAGTGAGT ACATTGGCAT AAATATAGAA ATTTATATAT ATACATATAT 360
ATAACTGTGC TNNTTGCCCT TTTTNNTGNG GGAAATTGGN 400

SEQ ID NO:81
SEQUENCE LENGTH:396
SEQUENCE TYPE:nucleic acid
TOPOLOGY:linear
CLONE:HUMGS00093
SEQUENCE DESCRIPTION:
GATCAGACTG TGGCATTGTA AATGTCAACA TTCCAACAAG TGGGGCTGAG ATTGGAGGTG 60
CCTTTGGAGG AGAAAAGCAC ACTGGTGGTG GCAGGGAGTC TGGCAGTGAT GCCTGGAAAC 120
AGTACATGAG AAGGTCTACT TGTACTATCA ACTACAGTAA AGACCTTCCT CTGGCCCAAG 180
GAATCAAGTT TCAGTAAAGG TGTTTTAGAT GAACATCCCN NAATTTGAGG GTGTTCCAGC 240
AGCTGTTTTT GGAGAAGACA AAGAAAATTA AAGTTTTCCC TGAATAAATG CATTATTATG 300
ACTGTGACAG TGACTAATCC CCCTATGACC NNAAAGNCCT GATTAAATCA AGAGATTCCT 360

TTTTTAAAAA TCAANTAAAA TTGTNACACC ATAAAA 396

SEQ ID NO:82
SEQUENCE LENGTH:400
SEQUENCE TYPE:nucleic acid
TOPOLOGY:linear
CLONE:HUMGS00094
SEQUENCE DESCRIPTION:
GATCGATGGT TGACAATCCA GAGTGGTGAA CAGCCCTACA AGATGGCTGG TCGATGCCAT 60
GCTTTTGAAA AAGAATGGAT AGAATGTGCA CATGGAATNG GTTATACTCG GGCAGAGAAA 120
GAGTGCAAGA TAGAATATGA TGATTTCGTA GAGTGTTTGC TTCGGCAGAA AACGATGAGA 180
CGTGCAGGTA CCATCAGGAA GCAGCGGGAT AAGCTGATAA AGGAAGGAAA GTACACCCCT 240
CCACCTCACC ACATTGGCAA GGGGGAGCCT CGGCCCTGAA CAGAGCAGCT GCTGATGTCT 300
GGAGGCTGAT TTTCCTGTTC TCTGTTCTCC ACTGGAAAGG TTGTTTACGA CAAACCTCCT 360
TGTCAAAGTN TGTAAAAATA AAGGATTGCT CCATCCTAAA 400

SEQ ID NO:83
SEQUENCE LENGTH:397
SEQUENCE TYPE:nucleic acid
TOPOLOGY:linear
CLONE:HUMGS00095
SEQUENCE DESCRIPTION:
GATCTGGGCA CTGTACTTNA GCCTGGGCGA CAGAGAGACC CATCTCAAAN AAAAAATTGG 60
AACCTGAGAA GGGGGTCGTG GGGTCCCCGG GGCCCACCGT CTGCACTTGG NATCTNAAGT 120
CGGGGTGGNC TTGTGGGACT NACCCNTTAC CCTGTGGGTT CTGTACTAGC TCCGGGNAAT 180
TGGTGACAGA NTCGAGTTAA ATTGTAGGAC ATCGCGTTGG TGTCTGAGAG GGAGTTGGAG 240
AGCTGGTTGG TGTGGAGGGA AAGGNTTACA CACATGTNAT TTCAGAAGCG TTCTGTGGGT 300
AGAGGAATCG TTTTCTCTTT GAGACTGTTA TGAGTATGTA CAAATTTTAT TTCCTGTAAA 360
AATATTTNCA TTTTTTAAAN TGGTTATTTT CTAGAAA 397

SEQ ID NO:84
SEQUENCE LENGTH:390
SEQUENCE TYPE:nucleic acid
TOPOLOGY:linear
CLONE:HUMGS00096
SEQUENCE DESCRIPTION:
GATCGAAGAT TTATCCCAGC AAGCACAACT AGCAGCTGCT GAGAAATTCA AAGTTCAAGG 60
TGAAGCTGTC TCAAACATTC AAGAAAACAC ACAGACTCCA ACTGTACAAG AGGAGAGTGA 120
AGAGGAAGAG GTCGATGAAA CAGGTGTAGA AGTTAAGGAC ATAGAATTGG TCATGTCACA 180
AGCAAATGTG TCGAGAGCAA AGGCAGTCCG AGCCCTGAAG ANCAACAGTA ATGATATTGT 240
AAATNCGATT ATGGAATTAA CAATGTAACC ATATGGANGC AACTTTTTTT TGGTGTCTCA 300
NAGGNGTAAC TGCAGCTTGG TTTGAAANTT TGTTACCTTG TTTCTTATCA TAAAATNAAN 360
NGTTATTNGC TTCCTTTTTT GGNTTGGAAA 390

SEQ ID NO:85

SEQUENCE LENGTH:392
SEQUENCE TYPE:nucleic acid
TOPOLOGY:linear
CLONE:HUMGS00097
SEQUENCE DESCRIPTION:
GATCACTTTC ACTNTCAATT ATTTGCCAGG NCTCACAGAA CTCAGAAAAG CTCAGANCAC 60
TCATGGTTAC TATTTAGTAA AGCAAAAAGA CACAAATNAA AATNAGCAAG TTTGGCCGGG 120
ATTGCAGGCA TGAGCCACTG AGCCCGGCCC CCAACTGTTA CATCAAAATA TTATTTGAGA 180
GTATATGTGT CCTCACGTCC CTAAAACACT AGAAACTGTC AANCTTTTAA TCTTTGTCAA 240
ACTCTCAAAA GTAGTATCTC TGCATTTGCA TGCCTTTGNG TNCTAATAAG GTTGAGTACT 300
GCTTTAAAAG TTTGCTGGNC ATCTNTTTGN TTTTTTTAAG GACCTGCGGT GGNNAGGCCC 360
NTTCAANANA TTNTTTCNTT AATTNGGGCC TN 392

SEQ ID NO:86
SEQUENCE LENGTH:393
SEQUENCE TYPE:nucleic acid
TOPOLOGY:linear
CLONE:HUMGS00098
SEQUENCE DESCRIPTION:
GATCAGTCTG GCTGGTGGTT TAACAGGTGT CACTCTGCAA ACCTGAATGG TGTATACTAC 60
AGCGGCCCCT ACACGGCTAA AACAGACAAT GGGATTGTCT NGNACACCTG GCATGGGTGG 120
TGGTATTCTC TGAAATCTGT GGTTATGAAA ATTAGGCCAA ATGATTTTAT TCCAAATGTA 180
ATTTAATTGC TGCTGTTGGG CTTTCGTTTC TGCAATTCAG CTTTGTTTAA AGTGATTTGA 240
AAAATACTCA TTCTGAACAT ATCCATGCGC AATCATGATA ACTGGTTGTG AGNAGTGCTT 300
TTCATTCTTC TCACTTGCCT TTGTTACTTA ATGTGCTTTC AGGACAGCAG ATATGCAATA 360
TTCACCAAAT AAATGTAGGC TGGTGGTAAT AAA 393

SEQ ID NO:87
SEQUENCE LENGTH:391
SEQUENCE TYPE:nucleic acid
TOPOLOGY:linear
CLONE:HUMGS00099
SEQUENCE DESCRIPTION:
GATCCAGAAA TACTTAACAC GTGAATATTT TGCTAAAAAA GCATATATAA CTATTTNAAA 60
TATCCATTTA TCTTTTGTAT ATCTAAGACT CATCCTGATT TTAACTATCA CACATGAATA 120
AAGCCTTTGT ATCTTTCTTT CTCTAATGTT GTATCATACT CTNCTAAAAC TTGAGTGGCT 180
GTCTTAAAAG ATATAAGGGG AAAGATAATA TTGTCTGTCT CTATATTGCT TAGTAAGTAT 240
TTCCATAGTC AATGATGGTT TAATAGGTAA ACCAAACCCT ATAANCCTGA CCTCCTTTAT 300
GGTTAATACT ATTTANGCAA GGANTGCAGT ACAGATTTGG NTACAGTACG GATTTGNCCA 360
AATAANTTCA NTAAAAGCCT TAAAGCTGAA A 391

SEQ ID NO:88
SEQUENCE LENGTH:390
SEQUENCE TYPE:nucleic acid
TOPOLOGY:linear

CLONE:HUMGS00100
SEQUENCE DESCRIPTION:
GATCAACCCA CAGAACAAAG CGGATTTCCA AGGCATCTCC CCAGAGCGAG CCTTTGCTGA 60
TTTTCTCTTT GCCAGCACCA TCCTGCACCT TGTTGTCATG AACTTTGTTG GCTGACTCAT 120
TCTCATTTAC TTAATTGAGG AGTAGGAGAC TAAAAGAATG TTCACTCTTT GAATTTCCTG 180
GATAAGAGTT CTGGAGATGG CAGCTTATTG GACACATGGA TTTTCTTCAG ATTTGCACTT 240
ACTGCTAGCT CTGCTTTTTA TGCAGGAGAA AAGCCCAGAG TTCACTGTGT GTCAGAACAA 300
CTTTCTAACA AACATTTATT AATCCAGCCT CTGCCTTTCA TTAAATGTAA CCTTTTGCCT 360
TCCAAATTAA GGACTCCATG CCACTCCTCN 390

SEQ ID NO:89
SEQUENCE LENGTH:390
SEQUENCE TYPE:nucleic acid
TOPOLOGY:linear
CLONE:HUMGS00101
SEQUENCE DESCRIPTION:
GATCTTTCTN AATGTGTATT GATTGGTCTT TTCAGCTACT CTGAACAGAT TACTAAGGCC 60
ATCTCCTCAT CTCTAAGGGA GAAAAATAGT CTGTAGATGA ATAATGTAAG GTAAAGAGTT 120
GCATGTCAGT CTTTGTAATN ATTTACACTT TAACTTTCTC CAGAACTCAG ACATGATTTC 180
AACATGGTGT TAGATTTGTG CATTTNATTT TCCTGACCAC CTCATTCCAG CCAATGTATG 240
GTTATCCACT CTGTGTGCNA AANCCAATCA TGCNTTTCAC GGCCCTTTAG TTCAGAGAAG 300
TTCTGCACTG ATTTTTAGTC TCTTGATGTC TCAATCTTAC ATGTATACCA ATCACAATGG 360
AATAAAAAGT GTTTGAGGTT GTACTGTGGN 390

SEQ ID NO:90
SEQUENCE LENGTH:391
SEQUENCE TYPE:nucleic acid
TOPOLOGY:linear
CLONE:HUMGS00102
SEQUENCE DESCRIPTION:
GATCCGAGGA GGCGGAACAA GTCCACGGAG TCCCNNGCAG GCCAACGTGC AGCGGCTGAA 60
GGAGTACCGC TCCAAACTCA TCCTCTTCCC CAGGAAGCCC TCGGCCCCCA AGAAGGGAGA 120
CAGTTCTGCT GAAGAACTGA AACTGGCCAC CCAGCTGACC GGACCGGTCA TGCCCGTCCG 180
GAACGTCTAT AAGAAGGAGA AAGCTCGAGT NATCACTGAG GAAGAGAAGA ATTTCAAAGC 240
CTTCGCTAGT CTCCGTATGG CCCGTGCCAA CGCCCGGNTC TTCGGCATAC GGGCAAAAAG 300
AGCCANGNAN GCCGCAGANC AGGATGTTNG TAAGGAAANN ATTTANAGCC CCTCCTGGGN 360
GACCTTTGGG ATTCAGTCGN CAGTCAATAA A 391

SEQ ID NO:91
SEQUENCE LENGTH:391
SEQUENCE TYPE:nucleic acid
TOPOLOGY:linear
CLONE:HUMGS00103
SEQUENCE DESCRIPTION:
GATCTGTGCC AAGCTCAGGG TGTAGCGCTG CAAACGATGA AGCAAGAGAT TCTCATTAAC 60

CTTGTGAAGC AAAAGCCACA AATAACAGAG GAACAACTTG AGGCTGTCAT TGCAGATTTC 120
TCAGGCCTGT TGGAGAAATG CTGCCAAGGC CAGGAACAGG AAGTCTGCTT TGCTGAAGAG 180
GGACAAAAAC TGGNGNCAAA AACTCGTGCT GCTTTGGGAG TTTAAATTAC TTCAGGGGAA 240
GAGAAGACAA AACGAGTCTT TCATTCGGTG TGAACTTTTC TCTTTAATTT TAACTGATTT 300
AACACTNTTT GGTGAATTAA TGAAATGNTA AAGACTTTTT ATGTGAGATT TTCCTTATCA 360
CAGAAATNAA NTNTCCTCCA AATGTTAATA N 391

SEQ ID NO:92
SEQUENCE LENGTH:385
SEQUENCE TYPE:nucleic acid
TOPOLOGY:linear
CLONE:HUMGS00104
SEQUENCE DESCRIPTION:
GATCTTTTCC CCTGGCCAAA GGGAAGTTGT ATTAGTCTGT GACATCTTGT GATGCTGTTT 60
ATCTTGGTTT GACATTGGAG ATACGCTAGT AACTGTGATA CCATACTATA AAACAGAAGA 120
ATTTTCTGCT ACTAAAAACT GCCTTTTTAC AAAATGACTG TAAATATTTG TAAAATAAAA 180
TAACACTAAA CTTTAAGCCC AAAAGGAGAG ATAGAGCCAT GTGTTCAGTT GTGGACCTGT 240
CCGTGGGGCA CAGTGCCACC CCATCACAGT GTTGCTGTCA TCAGGCAAAN GTGAATGTTT 300
GTTTATGGCA AATTCGNCTT TTGCGAATGG CTTANTTCTG ACACTACCTT TCTGGGAAAT 360
GTTAATANAT TTTTAATTNT TCAAA 385

SEQ ID NO:93
SEQUENCE LENGTH:381
SEQUENCE TYPE:nucleic acid
TOPOLOGY:linear
CLONE:HUMGS00105
SEQUENCE DESCRIPTION:
GATCTTTGAT AGTTTTGGTG AACTCTCTAA AATACATTCA CTGTGGGTCC GACGCAATTT 60
ATAAAAATNA TGTACTCAAG AAGGGAGACC TGTTTGTTTC ATTTCTCATC TGTTTGGGAG 120
ATGATTTTAG AGCACTAGAA AGGCACTGGG GAGATTCTCA GCTTAAAACA TCCAGCAGTT 180
TGAAGTATGA TTAGGTACAT CAGGGCTGCA TTGTCAATNT TCTCTTTAAG TCTTTTAACA 240
TTTATAGCAA TTTTTTTTTT CCCGGAGAGT TTAGGTTGCA AGTTTTGGGT TTCTTGTTTG 300
TTTTTGTTTT GCTTCCTGCT TTAATNCTTN AATTTNCAGT CATTACTGGT ATTGAAAAAT 360
AAAATATCTT TAAANCANNG N 381

SEQ ID NO:94
SEQUENCE LENGTH:380
SEQUENCE TYPE:nucleic acid
TOPOLOGY:linear
CLONE:HUMGS00106
SEQUENCE DESCRIPTION:
GATCTAAGAG ACTCAAGAGC TGGGTTTCTT TCAGCACTCT GTACTGTCCC AAATAGCAAA 60
CAAATNACTT TGTAGCCAGA TTTCTGAATG GAAATNAGAA ATTGAATTCT CCATGGACTT 120
TTAGGTTTAT GGGGGAGTTT TAGCTGTGTT TCTTGGTTTT ATTTCAGCCA AACATGTCTG 180
CTTTTGATTT TTTTTTTAAA GTATAAGTGG TCTATATATA TGTTCACCTT TTAAATGTAA 240

ATGTTTAAAA AGTAAGCATT TATGTGTTTC CATAACTGAC ATCTGATGCA GACCTCATTC 300
TCTCCCCCTC TTCTACCCTC CTCTTTTCCC CCTTTTCAAT ACTCTTGTAT TGGGTTCTAA 360
TAAAATGGGT TGCTTTTTCN 380

SEQ ID NO:95
SEQUENCE LENGTH:379
SEQUENCE TYPE:nucleic acid
TOPOLOGY:linear
CLONE:HUMGS00107
SEQUENCE DESCRIPTION:
GATCCATACT TGGATGATAT TGATGATGAG ATGGACCCAG AGATAGAAGA AGCTTATGAA 60
AAGTTTTGTT TGGAATCAGA GCGTAANGNA NAACAGTAAA GTTAAATTTC AGCATATCAG 120
TTTTATAAAG CAGTTTAGGT ATGGTGATTT AGCAGAACAC AAGAGAGCAA GAAAATGTGT 180
CACATCTATA CCAAATTGAG GATGTTGAGT TATGTTACTA ATGTATGCAA CTTTAATTTT 240
GTTTAACACT ATCTGCCAAA ATAAACTTTA TTCCCTATAA CTTAAAATGT GTATATATAT 300
ATAATAGTTT ATTATGTACA GTTAATTCTA CTGTTTTGGC TGCAATAAAA TCGATTTTGG 360
AAATAAATGG AATGTTGGN 379

SEQ ID NO:96
SEQUENCE LENGTH:384
SEQUENCE TYPE:nucleic acid
TOPOLOGY:linear
CLONE:HUMGS00108
SEQUENCE DESCRIPTION:
GATCTTTGCT GGCAAGCAGC TGGAAGATGG ACGTACTTTG TCTGACTACA ATATTCAAAA 60
GGAGTCTACT CTTCATCTTG TGTTGAGACT TCGTGGTGGT GCTAAGAAAA GGAAGAAGAA 120
GTCTTACACC ACTCCCAAGA NGAATAAGCA CAAGAGAAAG AAGGTTAAGC TGGCTGTCCT 180
GAAATATTAT AAGGTGGATG AGAATGGCAA AATTAGTCGC CTTCGTCGAG AGTGCCCTTC 240
TGATGAATGT GGTGCTGGGG TGTTTATGGC AAGTCACTTT NGNCAGACAT TATTGTGGCA 300
NATNTTGTCT GACTTACTGG TTNCAACAAN CCCAGAAGNC ANGTNAACTG TNTGANGTTN 360
ATNAAAAGNC ATGTNCTGAA CAAA 384

SEQ ID NO:97
SEQUENCE LENGTH:583
SEQUENCE TYPE:nucleic acid
TOPOLOGY:linear
CLONE:HUMGS00109
SEQUENCE DESCRIPTION:
GATCCTCATG AATGACTATC CTAAATTTAA GTATGCAGTT CTNTTTTTGC TGGGTTTATT 60
CGTGCTGGTT CATCGNGAGT NAGANGCCTG CCTTGCTGTT CCTGGGAAGA TGCCATAGTT 120
TTCGTTACTG GATGTTTGGA GTAGATACTG GTCTGTNATT GGTGGAATGG AGAACACACG 180
TGTTGGTGCT TCTGGGTAGC ACTGGTTTGC ATTAGTTTAT GTTTCCATGC CAGAGTTTGT 240
GTGGGCGGGC GCATGTCCAC CACAGAGTGC ACTCGAGGGG ACTTTCAGTC ACAGGATTTC 300
ATAATTGTNA TTGTCACACT TTCAAATTTT TGTACATCAG TGAATTTTTT TTATATTAAA 360
AGGTTGAGCC AAAGCCCCCA GTGTTTTGTA TTTTGAAGCC AAGCTTCACT TCTAAAAGTG 420

CCTACAGAGG ACTTGTAAAA TGGAAAATGC AGCTCTGCAC GGAGTTTGAA ACCGTCATAC 480
CTCCTTCTAT TAGGGAATNG GCATATACTG AGGGTGGTCC GGAAGNNNTT AACTTCCTAA 540
AATTTTTAAA TAAAAGGCCT TTGCACCATT GGACCCCNTT AAA 583

SEQ ID NO:98
SEQUENCE LENGTH:370
SEQUENCE TYPE:nucleic acid
TOPOLOGY:linear
CLONE:HUMGS00110
SEQUENCE DESCRIPTION:
GATCAAGAAA AGCAACTTAT GGAAACAACT ACAGAATTTA CAAAAAAGGA TACTCAAACC 60
AAAAGTATTA TTTCAGAGAC CAGTAATAAA ATTGACGCTG AAATTGCTTC CTTAAAAACA 120
CTGATGGAAT CTAACAAACT TGAGACAATT CGTTATCTTG CAGCTTCAGT GTTTACTTGC 180
CTGGCAATAG CATTGGGATT TTATAGATTC TGGAAGTAGT ATTAATGCTC ATCCTGCTGT 240
GGCTGTTGGC TTCTTAGAAC ACCAAACCGG GAGAGATTTA CTTTGAACAT TGTCAGTTGC 300
AGCAAAAATT TACTACACAA GATTATTCGA AGTGTATACG GACTAAAAGA GGAAGTGTTT 360
TAGAATGAAA 370

SEQ ID NO:99
SEQUENCE LENGTH:384
SEQUENCE TYPE:nucleic acid
TOPOLOGY:linear
CLONE:HUMGS00111
SEQUENCE DESCRIPTION:
GATCTGAAGA GCGTCCTGGG TCAACTGGGC ATCACTAAGG TCTTCAGCAA TGGGGCTGAC 60
CTCTCCGGGG TCACAGAGGA GGCACCCCTG AAGCTCTCCA AGGCCGTGCA TAAGGCTGTG 120
CTGACCATCG ACGAGAAAGG GACTGAAGCT GCTGGGGCCA TGTTTTTAGA GGCCATACCC 180
ATGTCTATCC CCCCCGAGGT CAAGTTCAAC AAACCCTTTG TCTTCTTAAT GATTGNCCAN 240
AATACCAAGT CTNCCCTCTT CATGGGAAAA GTGGTGAATT CCACCNAAAA ATAACTGNCT 300
GTNGGTNCTC AACCCCTTNC NNTTCATCCN TGGGCCCNTN GGCTTGGATN GANAATTAAA 360
AGAAGGGGTT GNGGCTNGGG NAAA 384

SEQ ID NO:100
SEQUENCE LENGTH:374
SEQUENCE TYPE:nucleic acid
TOPOLOGY:linear
CLONE:HUMGS00113
SEQUENCE DESCRIPTION:
GATCTTTGAA ACCTTGGTCC CGTTAACTTA CTAGTCACAT TGACCAATGT TTTATAGAAA 60
TGCCTAGAAT TTTGAGACTA ATNGTAGTTA TCCATTAACA TTCCAAAAGT TTTGTNCTTT 120
TNAAAATTTG TNTTGGTAAT TATCACATTT NTNCCTCTTA CCTTCCTTTA AATGGCCACA 180
GTGTGTACTG CTGGANTGTN CCATCCAAAA GATGTAGCTT CAGANGCACA GTGATTGCCC 240
CAGGGTCCAT GAGATATTGT TTGTATTATG ANGTTGGAGT GCTGTCTACT GAAATTATAC 300
TCTTAAATAA NTATGTATGT NGTGTGTAAT ATTTCCTAAT AAATNCTTTN GATAAACTAA 360
AAAACTNNNG CTNN 374

SEQ ID NO:101
SEQUENCE LENGTH:382
SEQUENCE TYPE:nucleic acid
TOPOLOGY:linear
CLONE:HUMGS00114
SEQUENCE DESCRIPTION:
GATCTGTGCA AGGTATTAAC GTGTCAGGGC TGAGTGTTCT GGGATTTCTC TAGAGGCTGG 60
CAAGAACCAG TTGTTTTGTC TTGCGGGTCT GTCAGGGTTG GAAAGTCCAA GCCGTAGACC 120
CAGTTTCCTT TCTTAGCTGA TGTCTTTGGC CAGAACACCG TGGGCTGTTA CTTGCTTTGA 180
GTTGGAAGCG GTTTGCATTT ACGGCTGTAA ATGTATTCAT TCTTAATTTA TGTAAGGTTT 240
TTTTTTGTAC GCAATTCTCG GATTCTTTTG AAGNAGATGA CAACAAATTT NGGTTTTCTA 300
CTTGTTATGT GAAGACCATT AAGGCCCCAA GCAACAAGNC AATTNTGTAA GGGAAANTNA 360
AAGTTCCTTG CNGTAANCCA AA 382

SEQ ID NO:102
SEQUENCE LENGTH:368
SEQUENCE TYPE:nucleic acid
TOPOLOGY:linear
CLONE:HUMGS00115
SEQUENCE DESCRIPTION:
GATCTGTCTC TGCTGTTTAA CTTCATTGGA TTAATCAGCT GGTTTCAACT CTACTGCGAA 60
ACAAAAATAG CTCCTTAAAA GTACTGTTCT CCTTCAGTGG CATGTAGTTA TCTAATCAAG 120
ACACCTCATT CAAACAAAAC CTGCCTTAGG AAAATTTAAT ATATTTNAAA TNATTTTAAA 180
AGAAATACAA CATCTTATTC TTTAGCTTTC TTAATCGGTG CTTTATGGAG GCCAGTGTAA 240
CGNTACATGA CTCGTTGAGA AAGTTGAGGA ATTTCCTCTA CCACCTTTGT TGCTTGAAGA 300
AAAACATGTC TTTTCAAAAT GAGAGGCTTT CATTGAAGAA AAGAAAAAAA CAACAGTTAA 360
AAGCTAAA 368

SEQ ID NO:103
SEQUENCE LENGTH:367
SEQUENCE TYPE:nucleic acid
TOPOLOGY:linear
CLONE:HUMGS00116
SEQUENCE DESCRIPTION:
GATCTCGGAT GACCAAACCA GCCTTCGGAG CGTTCTCTGT CCTACTTCTN ACTTTACTTG 60
TGGTGTGACC ATGTTCATNA TAATCTCAAA GGAGAAAAAA AACCTTGTAA AAAAAGCAAA 120
AATGACAACA GAAAANCAAT CTTATTCCGA GCATTCCAGT AACTTTTTTG TGTATGTNCT 180
TAGCTGTACT ATAAGTAGTT GGTTTGTATG AGATGGTTAA AAAGGCCAAA GATAAAAGGT 240
TTCTTTTTTT TCCCTTTTTT GTCTATGAAG TTGCTGTTTA TTTTTTTGGG CCTGTTTGAT 300
GTATGTGTGA AACANTTGTN GTCCAACATT AANCAGGANT TTTATTTTNC NGAGTNGTNC 360
TANCAAA 367

SEQ ID NO:104
SEQUENCE LENGTH:366

SEQUENCE TYPE:nucleic acid
TOPOLOGY:linear
CLONE:HUMGS00117
SEQUENCE DESCRIPTION:
GATCTTAGTA ACTATGNATG AAGATGGTGC TTGGCCTGTN CTTCTTGATG AATTTGTTGA 60
GTGGCAAAAA GTCCGTCAGA CATCATAGCA AGAACTATGT GAAGAAAATG CAAACCTTTC 120
AATTCCCACG TGTATACAAG CTAATGTGAT GAGGGGGAAA AAAATCCAAC GGGTGCATTT 180
TCATTCATAT GAAAGACTTC TCATAGTACT TTTTTTTCCN TTTTTAAAA GGAGGTTTTT 240
CTTGTTACAT GTGATGGGCA TTGAGCCACA CCNNTTCTTA GACTGAATAT NGAAGTTTTT 300
GTTTTGAGTT ATGTTTATAA CATTTATTTC AGAACANTAA TGATTCAGAT TTGTGACAAA 360
GGCAAA 366

SEQ ID NO:105
SEQUENCE LENGTH:122
SEQUENCE TYPE:nucleic acid
TOPOLOGY:linear
CLONE:HUMGS00118
SEQUENCE DESCRIPTION:
GATCCCCGAA ATTGGTGGGC TTGACCTCCT GGCAAATTGC TGCGTCTTTC CACTTGCTGT 60
TCAGGACCAC TAAATGCTGA AATNTGGATG CATACCGAAA TAAAAGNAAT TCATTGTGTA 120
AA 122

SEQ ID NO:106
SEQUENCE LENGTH:364
SEQUENCE TYPE:nucleic acid
TOPOLOGY:linear
CLONE:HUMGS00119
SEQUENCE DESCRIPTION:
GATCTTCAAT ATGAAGACAT GAGCTTTTCT CGCAGGAAAT TTTCTTTTTC ACAGAACTGG 60
TGTCAGGAAT CACTGAAGGG CTAACCGTGA TAGTCCTTGC AAGTAAGTCA AGGTTTTATC 120
CTGATTGGAA ATAGAAGACA TTTCCGGTTG AGAGAACAGA TTCGTTGGAA GCTTAACTTT 180
TGTTGCCTCT TAACGCCACC AAATTTTAGG GTAATTTGAT TATGAAAGAG TGAATTTTTC 240
TGGACAGAAA AGGGAGAGCT ACCAAATTGT TTTTTTCTTT TTAAAAGGAA GTTTAATGTC 300
CGTTGTATCA CAAATCAGTG TTAAAACACC AGAACTTTAG CCAAAATAAA TGTCTTACAT 360
TACN 364

SEQ ID NO:107
SEQUENCE LENGTH:358
SEQUENCE TYPE:nucleic acid
TOPOLOGY:linear
CLONE:HUMGS00120
SEQUENCE DESCRIPTION:
GATCAGCAGG GAGTTTATTT GAGGACATCA GTCACCTTTG GGGTTGCCAT GTACAATNAG 60
ATTTATAATC ATNATACTCT TCGGTGGTAG TTTCAAAAGA CACTACTAAT ACGCAGGAAG 120
CGTTCCAGCT ATTTAATGCT GGCAACTACT GTTTAATGGT CAGTTAAATC TGTGATAATG 180

GTTGGAAGTG GGTGGGGTTA TGAAATTGTA GATGTTTTTA GAAAAACTTG TGAATGAAAA 240
TGAATCCAAG TGTTTCATGT GAAGATGTTG AGCCATTGCT ATCATGCATT CCTGTCTCAT 300
GGCAGAAAAT TTTGAAGATT AAAAAATAAA ATAATCAAAA TGTTTCCTCT TTNCTAAA 358

SEQ ID NO:108
SEQUENCE LENGTH:430
SEQUENCE TYPE:nucleic acid
TOPOLOGY:linear
CLONE:HUMGS00121
SEQUENCE DESCRIPTION:
GATCTGTCTC TGGGGTCCCN CATACAGAGA AATGCATCTT GCTGAACAAG TGACCAATAA 60
TCTTAAAGAA CTTGCACAGC AAGTAACTCC AGGTGATATC GTAAGCACGT ATGGAGTTCG 120
AAAAGCAATG GGGATTTCCA TTCCTTCCCC CGTCATGGAA AACAACTTTN TGGATTTGAC 180
AGANGNNNCT GAAGAACCTA AAAAGACGGA TGTTGCTGAG TGTGGACCTG GTGGAAGTTG 240
AGGCTGCCTG GTATTTGATT ATATATTATG TACATACTTT TTCATTCTTA ACTTAGAAAT 300
GCTTTTCAGA AGATATTAAA TATTTGTAAA TTGTNTTTTT AATTAAACTT TGGAACAGCG 360
AATTTGGNTG TTCCAGAGGT TGGGCCTTGT ATTAGGGAAA TAAAAGCTTG GACCTGGGGC 420
CTCGTGGAAA 430

SEQ ID NO:109
SEQUENCE LENGTH:357
SEQUENCE TYPE:nucleic acid
TOPOLOGY:linear
CLONE:HUMGS00122
SEQUENCE DESCRIPTION:
GATCACTNGA TATTTTAGTC ATTCTGCTTC TCATCTAAAT ATTTCCATAT NCTGTATTAG 60
GAGAAAATNA CCCTCCCAGC ACCAGCCCCC CTCTCAANCC CCCAACCCAA AACCAAGCAT 120
TTTGGAATGA GTCTCCTTTA GTTTCAGAGT GTGGATTGTA TAACCCATAT ACTCTTCGAT 180
GTACTTGTTT GGTTTGGTAT TAATTNGACT GTGCATGNCA GCGGCAATCT TTTCTTTGGT 240
CAAAGTTTTC TGTTTATTTT GCTTGTCATA TTCGATGTAC TTTAAGGGTG TCTTTTATGA 300
AGGTTTGCTA TTCTTGGCAN TTAAGNTTTT TTAGGNCTTT TTAAANGNGN ANNNAAA 357

SEQ ID NO:110
SEQUENCE LENGTH:356
SEQUENCE TYPE:nucleic acid
TOPOLOGY:linear
CLONE:HUMGS00123
SEQUENCE DESCRIPTION:
GATCAAGGGA CGGCTGAACA GACTTCCCGC TGCTGGTGTG GGTGACATGG TGATGGCCAC 60
AGTCAAGAAA GGCAAACCAG AGCTCAGAAA AAAGGTACAT CCAGCAGTGG TCATTCGACA 120
ACGAAAGTCA TACCGTAGAA AAGATGGCGT GTTTCTTTAT TTTGAAGATA ATGCAGGAGT 180
CATAGTGAAC AATAAAGGCG AGATGAAAGG TTCTGCCATT ACAGGNCCAG TAGCAAAGGA 240
GTGTGCAGAC TTGTGGCCCC GGATTGCATC CAATGCTGGC AGATTGCATG ATTCTCCAGT 300
ATATTTGTAA AAANTAAAAA AAAGCTAAAC CCATTAAAAA GTATTTGTTT TGCAAA 356

SEQ ID NO:111
SEQUENCE LENGTH:375
SEQUENCE TYPE:nucleic acid
TOPOLOGY:linear
CLONE:HUMGS00124
SEQUENCE DESCRIPTION:
GATCCTACCT ATCAAGCACT AAAAAGTTGA ACCATTATAC TTTATATCTG TAATGATACT 60
GATTATGAAA TGTCCCCTCA AACTCATTGC AGCAGATAAC TTTTTTGAGT CATTGACTTC 120
ATTTTATATT TAAAAAATTA TGGAATATCA TCTGTCATTA TATTCTANTT AANGTTGTGC 180
ATAATGCTTT GGAANAATGG GTCTTTTATA GGAAAAAACC TGGGATAACT GATTTCTATG 240
GCTTTCAAAG CTNAAATATN TAATATACTA AACCANCTCT AATATTGCTT CTTGTGTTTT 300
ACTGTCAGNT TAANTTACAG CTTTTATGGG TGGTTAACTT TTCGTNCATT TTCAAAAAAN 360
CCNGGGGNNN NNNNN 375

SEQ ID NO:112
SEQUENCE LENGTH:356
SEQUENCE TYPE:nucleic acid
TOPOLOGY:linear
CLONE:HUMGS00125
SEQUENCE DESCRIPTION:
GATCTCTGTT TTGTTGTTGA AAATTCATTT GTATACTTTT GTTTTNATCT AGGACTTCAT 60
GTTTTTTNAA AGCACTGGCA GCCAGGAACA AAAATCAGGA GTGTGGTAGT GGATTAGTGA 120
AAGTCTCCTC AGGAAATCTG AAGTCTGTAT ATTGATTGAN ACTATCTAAN CTCATACCTG 180
TATGANTTAA GCTGTAAGGC CTGTAGCTCT GGTTGTATAC TTTTCCTTTT CAAATTATAG 240
TTTATCTNCT GTATAACTGA TTTATAAAGG TTTTTGTACA TTTNTNAATA CTCATTGTCA 300
ATTTGAGAAA AAGGACATAT GAGTTTTTNC ATTTATTAAT GNAACTNCCT TTGAAA 356

SEQ ID NO:113
SEQUENCE LENGTH:351
SEQUENCE TYPE:nucleic acid
TOPOLOGY:linear
CLONE:HUMGS00127
SEQUENCE DESCRIPTION:
GATCACATTA TNATAAATAA ATGAAAAAAT GATTTAATCT GTAATAAACT GGTTTATTGT 60
GCAGTGACTG TAATATACTA GAGTTATAAT AAATTGTTTA CTCTGCCTCA CCAAACACAT 120
GCTAGGATAT AACCCCCAAA ATAAGTATTT AACTTTGCAT TAGGTATAAA GGAGACTGGG 180
TGCTATAATN AGATTATTTT GAGGCAGACA GAGAGCTGTT ATCCTAACTG ATTTAGTATG 240
TTCTGTAATT GAGAAAATGT TCACCAAATN ATACTTTTTA GTGATTTACA TGTACATTTT 300
ATAGGGGACA TGTTCTGTGT ATAGCGAATA AATAACTTTT ATAGTATCAC N 351

SEQ ID NO:114
SEQUENCE LENGTH:352
SEQUENCE TYPE:nucleic acid
TOPOLOGY:linear
CLONE:HUMGS00128

SEQUENCE DESCRIPTION:
GATCTTGAAC CTGGTGCTCC ATCCATGGNA GCNNAAAGCC TTTGCATCCC CTTCAAACCA 60
CTCTGTGAAC TGCAGCCTGG AGCCAAATNT GTCTGTGGCA AGAACCCTGC CAAGTACTAC 120
ACCTTATTTG GTCGCAGCTA CTGNGGGATG NACGAAAGCC CCCTCTTCAA CTCCTCTCAC 180
TTTTTAAAGC ATTGATATTA GTATCTTCTC AGATACAGAC CGTTTTATGA TTTTTTAAAA 240
AGTAAAAGTT CTAAAATGAA GTCACACAGG ACAATTATTC TTATGCCTAA GTTAACAGTG 300
GATAAAAGAC TTTTCTGTAA ACAACTCCAG TAATAAATAT CATGNACTNA AA 352

SEQ ID NO:115
SEQUENCE LENGTH:348
SEQUENCE TYPE:nucleic acid
TOPOLOGY:linear
CLONE:HUMGS00129
SEQUENCE DESCRIPTION:
GATCAAGTCG TGCTCCTGGC AGGCGCGCCC CTGGAGGATG AGGCCACTCT GGGCCAGTGC 60
GNGGTGGAGG CCCTGACTAC CCTGGAAGTA GCAGGCCGCA TGCTTGGAGG TAAAGTCCAT 120
GGTTCCCTGG CCCGTGCTGG AAAAGTGAGA GGTCAGNNTN NTNAGGTGGC CAAACAGGAG 180
AAGAAGAAGA AGAAGACAGG TCGGGCTAAG CGGCGGATGC AGTACAACCG GCGNTTTGTC 240
AACGTTGTGC CCACCTTTGG CAAGAAGAAG GGCCCCAATG CCAACTCTTA AGTCTTTTGT 300
AATTCTGGGC TTTCTTCTAA TAAAAAAGCC ACTTNAGTTC AAGTCAAA 348

SEQ ID NO:116
SEQUENCE LENGTH:344
SEQUENCE TYPE:nucleic acid
TOPOLOGY:linear
CLONE:HUMGS00130
SEQUENCE DESCRIPTION:
GATCTCCCAC AATTAATTTA TCTTTTGACA AAGGGGATAA AGAGTTTCAG TTTAGCTCCT 60
TTTGATTGTA TATNATTTTT TCCTTTTTNA TTGTGAAAAG AGGTAGGTTT TATTTGTGGA 120
GAGAGAGTTG AAGATTAGGG AACCAGTGAT TTTAATTATG CTACTTTTCC TTCTAAGAGA 180
TAAATTGATA TATCATTCAG TGTCATGAAA AACATGAATG TNGTACAATT TTCTNCCTCA 240
AAAAACTTTT TAAATGTAAG TATCCTTATT TNNTTTTTAA AAGAGCACAA TGTAGGTGTA 300
TTTGGGTATT TCCAAGAAAA GANTAAANCC ATTAATGCAG TAAA 344

SEQ ID NO:117
SEQUENCE LENGTH:351
SEQUENCE TYPE:nucleic acid
TOPOLOGY:linear
CLONE:HUMGS00131
SEQUENCE DESCRIPTION:
GATCTGGGCT CCAAGCCAGG AAAGGTGAAC AGAAACCACA AGTNTCCAGC CCTCGGTGCT 60
GGAGTGGACG TTAATTGTNA GCCACCAGAC TGTCCCGGCA CCTACAGAGA ATGTTTCACA 120
GTTCTGGCAT TTAAATCCTT TGATAGTGGA TTGTGCTGCT GTTAGCCTTA GTTTCAGTGC 180
TTTACAAGTC TCGCTTATNA TCTCATTGGT ATTTAGGTAT ACAAAACAGT TGATTATTCA 240
CCACGCCAAT ATCTGGGTCT CTGTATCTCA TGTAGAACAT AAGAAAATGG GAACTAATAG 300

GGAAATTTAT TTATAGCATG AAAATAAACC TGGTGGCTGG AGTCTGCTAA A 351

SEQ ID NO:118
SEQUENCE LENGTH:343
SEQUENCE TYPE:nucleic acid
TOPOLOGY:linear
CLONE:HUMGS00132
SEQUENCE DESCRIPTION:
GATCCTAAGG CAATAAAAGA ATAAGGAGAT TTGGAAAACC ATTGTCTGTA ATCTCTGAAG 60
AAAAGTGGAC ATTAGGGGAG TCAGTTGAAA AGCAAAGCTA TCACCATTTT CTAAAGAGGA 120
AAAAGGTGAA CCTCACAAAC TATAGACCAA AAAAATAGGA CATCGAGAGA AAGAATATGA 180
AGCTAGCATA GGTTCACAAA GAATGAGTCA AATCAAACAA CATGCATTTT TTATTTATAA 240
AGCATGACTT GTTCATTGTC AATTCATGTT AGCTTAATCA TTAGGCATTA ATGCCATCAC 300
TGCAATGCAT ATGTCAGCAA TAAATAATCA AGGCCCGGCT TCN 343

SEQ ID NO:119
SEQUENCE LENGTH:345
SEQUENCE TYPE:nucleic acid
TOPOLOGY:linear
CLONE:HUMGS00133
SEQUENCE DESCRIPTION:
GATCTTCCCA CAACACCACA GGACTGCAGG GTGCACAACT CCCCTGCCAA GGAAAACCAT 60
GCAGTCCTCC CCTCCCTGGT CTCCTGCTTC AGCTCTGTAC AACGAGGGCA AAGATGCTAA 120
ATCTTGCTTT GCATTCAGTA AAGTGTCAAG TGATTAAGTG TGTATTTGTA CCCTAGATGA 180
TATGAACCAG CAGTCTTGTT TTGGCATCAT CCTCATCATG TTGTATTCCA GCTTCTTAAG 240
TGGAAGGAAA AGAGTGCTGA GAAATGGCTC TGTATAATCT ATGGCTATCC GAATTCTCTG 300
AAAAANTANT AAAAGTCCCC TCTNTTATAT GAGCCTGTAC AGAAA 345

SEQ ID NO:120
SEQUENCE LENGTH:358
SEQUENCE TYPE:nucleic acid
TOPOLOGY:linear
CLONE:HUMGS00134
SEQUENCE DESCRIPTION:
GATCCCCCCT CTCGAGGGCG ATGAGGATGC GTCTCGCATG GAAGAAGTCG ATTAGGTTAG 60
GAGTTCATAG TTGGAAAACT TGTGCCCTTG TATAGTGTCC CCATGGGCTC CCACTGCAGC 120
CTCGAGTGCC CCTGTCCCAC CTGGCTCCCC CTGCTGGTGT CTAGTGTTTT TTNCCCTCTC 180
CTGTCCTTGT GTTGAAGGCA GTAAACTAAG GGTGTCAAGC CCCATTCCCT CTCTACTCTT 240
GACAGCAGGA TTGGNTGTTG TGTATTGTGG TTTATTTGAA TTNCCTTCAT TTTGTTCTGA 300
AATTAAAAGT ATGCAANAAT AAAAGAATTA TGCCCNTTTT TNATACAANA NNAANAAA 358

SEQ ID NO:121
SEQUENCE LENGTH:350
SEQUENCE TYPE:nucleic acid
TOPOLOGY:linear

CLONE:HUMGS00135
SEQUENCE DESCRIPTION:
GATCTTCATG CCCTGGGTTC TGCCCGNACG GACCCNCATC TCTGTGACTT CCTGGAGACT 60
CACTTCCTAG ATGAGGAAGT GAAGCTTNTC AAGAAGATGG GTGACCACCT GACCAACCTC 120
CACAGGCTGG GTGGCCCGGA GCTGGGCTGG GCGAGTATCT CTTCGAAAGG CTCACTCTCA 180
AGCACGACTA AGAGCCTTCT GAGCCCANCG ACTTCTGAAG GGCCCNTTGC AAAGTAATAG 240
GGCTTCTGNC TAAGGCTCTG CCTNCAGNCA ATAGGNANGC TTTTTAACN ATCCTAACAN 300
GGNTTGGGAC CAAATGGNAA TAAAGNTTTG TNGATGCAGG AGATATGAAA 350

SEQ ID NO:122
SEQUENCE LENGTH:338
SEQUENCE TYPE:nucleic acid
TOPOLOGY:linear
CLONE:HUMGS00136
SEQUENCE DESCRIPTION:
GATCTGAGCT GAATTTGAAG ACTATTAATA AGTTATGTTT GGAAGTTTTA ACTTCAATGA 60
AGTAATTATT TGCTGTGAAA GAAACAAACA TTGAATTACT AAACAAAGAT GGTGCAATAT 120
CTTTGTTTTT TTTTTATGAG GCTCCTGAGA ATCAACCCAA CTGAAGCATT TCAATTCACT 180
TGAATGAGAA ACGTGTTTAG TATCAAAAGA GCCCAAGAAG ACACTGGTGT GAAAGGTACA 240
NTCTCAGAGG TTGGTCAATT ACCGTGGCAC ANTTTCTGGT CACTTTGTAC AATGTAGATT 300
TGAAGTACAG TGGTGAAAAC ATTAAATGTG ACATTTGN 338

SEQ ID NO:123
SEQUENCE LENGTH:337
SEQUENCE TYPE:nucleic acid
TOPOLOGY:linear
CLONE:HUMGS00137
SEQUENCE DESCRIPTION:
GATCAAAAAA CATGAGGNAG AAGAAGCCAA AGCTGAGCGT GAGAAGAAGA NNAAAAGAAC 60
AGAAAGAAAA GGATAAATAG AATCAGAGAT TTTATTACTC ATTTGGGGCA CCATTTCAGT 120
GTAAAAGCAG TCCTACTCTT CCACACTAGG AAGGCTTTAC TTTTTTNAAC TGGTGCAGTG 180
GGAAAATAGG ACATTACATA CTGAATTGGG TCCTTGTCAT TTCTGTCCAA TTGAATACTT 240
TATTGTAACG ATGATGGTTA CCCTTCATGG ACGTCTTAAT CTTCCACACA CATCCCCTTT 300
TTTTGGAATA AAATTTGGAA AATGGAAATN AAGGAAA 337

SEQ ID NO:124
SEQUENCE LENGTH:336
SEQUENCE TYPE:nucleic acid
TOPOLOGY:linear
CLONE:HUMGS00138
SEQUENCE DESCRIPTION:
GATCACCATT TGAGATACGC GGCTTAACGC ACATGTGAGT GTAGCTTGCT ACATGAAAAT 60
GCTAGGCTCT AGGGCATGTA AAACATGAAT ACAGAATACT AGATTGTTCT AAGTAATGTC 120
ATTTCGGTTT GTGANTTTGA TTTTTCCCTT CATTTCATGT CATATTGNAA ATGCAAACAA 180
ACTGCTCTCA AGAACACCCA GAAGCTATCT GTGTTACCAG ATGTGTTGTG NACACTCTAC 240

TNTTTTTCAT AGGTGCTACC TGGNAATATA TGTCCATTGT AGTGGTGGNG NGGGNCTNGA 300
CTCTNTCAGG CTCTTNTCTN GCCAGNTGNC TNCNGN 336

SEQ ID NO:125
SEQUENCE LENGTH:338
SEQUENCE TYPE:nucleic acid
TOPOLOGY:linear
CLONE:HUMGS00139
SEQUENCE DESCRIPTION:
GATCTGGGCT GCAGGAGCTG GGGCCACCCA CAGCCCCCCT ACCGACCTGG TGTGGAAGGC 60
ACAGAACACC TGGGGCTGCG GGAACAGCCT GCGTACGGCT CTCATCAACT CCACTGGGGA 120
AGAAGTGGCC ATGCGCAAGT TGGTGCGCTC AGTGACTGTG GTTGAGGACG ACGAGGATGA 180
GGATGGAGAT GACCTGCTCC ATCACCACCA CGTGAGTGTA AGCCGCCGTT GAGGCCGAGC 240
CTGCACTGGG GCCACCNAGC CAGGCCTGGG GGNAGCNTTT CCCNAGNNTN CNNGTGCCAA 300
AANTTTTTTN ATTAAAAGAT TGTTTTGGGA ACTTTAAA 338

SEQ ID NO:126
SEQUENCE LENGTH:347
SEQUENCE TYPE:nucleic acid
TOPOLOGY:linear
CLONE:HUMGS00140
SEQUENCE DESCRIPTION:
GATCAAGCAG ATTCCACGAA TCCTNCGGAC CAGGTTTAAA TANGGCAGGA AAGTTCCCTT 60
CCCTGCTCAC ACACAACGAA AACATGGTGG CCAAAGTGGA TGAGGTGAAG TCCACAATCA 120
AGTTCCAAAT NAAGAAGGTG TTATGTCTGG CTGTAGCTGT TGGTCACGTG AAGATGNCAG 180
ACGATGAGCT TGTGTATAAC ATTCACCTGG CTGTCAACTT CTTGGTGTCA TTGCTCAAGG 240
AAANCTGGCA GANTGTCCGG GCCTTATATA TCANNGNGCA CCATGGGCAA NGCCCCAGCG 300
GCTTATATTT AAGGCACATT TTNNATAAAT TCTATTNACC CGGTAAA 347

SEQ ID NO:127
SEQUENCE LENGTH:335
SEQUENCE TYPE:nucleic acid
TOPOLOGY:linear
CLONE:HUMGS00141
SEQUENCE DESCRIPTION:
GATCAGTTGT AATCAGAATA CAACTGNGTC TTGTAGTTGT AATATGTTCT ATCTTAACCA 60
CCACTTTCGT ACCAGGAACC TGCTCAGGTT TGTTCTCTAG AAGCTCCCAA CATAGATAGT 120
CTACATTTCA GACTACTAAG TTATTAACAA ACCCTTTGGG CCCATGTTCA CTTTAGGGTT 180
GAGCATAGTG TGAGGAGATG TAAATTAAAT TATAATCCTA TATGTGTGTG TAATAAATAT 240
TAAAGTGTAT AAATTAAACA GCAGATTCTA AGTATCCAAC AAGAGTCAAA TAAATGATAC 300
AAAGTCACCA AATAAATAAT ATTTAATCTC ATCTN 335

SEQ ID NO:128
SEQUENCE LENGTH:356
SEQUENCE TYPE:nucleic acid

TOPOLOGY:linear
CLONE:HUMGS00142
SEQUENCE DESCRIPTION:
GATCTAGTTA AAGTTATTCA ACAGGAGTCT TACACATATA AAGACCCAAT TACAGAATTT 60
GTTGAATGTT TATATGTTAA CTTTGACTTT GATGGGGCTC AGAAAAAGCT GAGGGAATGT 120
GAATCAGTGC TTGTGAATGA CTTCTTCTTG GTGGCTTGTC TTGAGGNNNN CATTGAAAAT 180
GCCCGTCTCT TCATATTTGA GACTTTCTGT CGCATCCACC AGTGTATCAG CATTAACATG 240
TTGGCAGATA AATTGAACAT GACTCCAGAA GAAGCTGAAA GGTGGATTGT AAATTTGATT 300
AGAAATGCAA GACTGGATGC CAAGATTGAT TCTAAATTNA GGTCATGTGG TTATGN 356

SEQ ID NO:129
SEQUENCE LENGTH:333
SEQUENCE TYPE:nucleic acid
TOPOLOGY:linear
CLONE:HUMGS00143
SEQUENCE DESCRIPTION:
GATCAAAGNA CTCTGACTGC AGAACTGCCG CTCTCAGTGG ACAGGGCATC TNTNACCCTG 60
AGACCTGTGG CAGACACGTC TTGTTTTCAT TTNATTTTTG TTAAGAGTGC AGTATTGCAG 120
AGTCTAGAGG AATTTTNNTT TCCTTGATTA ACATGCTTTT CCTGGTTGTN ACATCCAGGG 180
CATGGCAGTG GCCTCAGCCT TAAACTTTTG TNCCTACTCC CACCCTCAGC GAACTGGGCA 240
GCACGGGGAG GGTTTGGCTA CCCNTGCCCA TCCNTGAGCC AGGTACCACC ATTGTAAGGA 300
AACACTTNCA GAANTTCAGC TGGTTCCTCC AAA 333

SEQ ID NO:130
SEQUENCE LENGTH:332
SEQUENCE TYPE:nucleic acid
TOPOLOGY:linear
CLONE:HUMGS00144
SEQUENCE DESCRIPTION:
GATCTCTGTA GATATTCTGT TTTATTTTGG TCATCTTTAG AAGTTATCAG GAATGTGTTT 60
AAAACAAGAA GAGAACTTTT CTAAGGAATG ATACATAGAA AAGATTTTAT TTTAAAATGA 120
GTTGTAAAGC TTGTTTTTCT TTGTTGCTGC AANTATCTGC CCAAGTTAAT GCAAATGGAC 180
ACATTTTTTA TGTCAGAAAA ACACACACAC ACACACACAC ACACACACAC ACACACNCGC 240
GNNACACAGN GANAAAAGTG CTTGNGCTTN NNCTCNCTNC CCCTTGCNGT CTGTTGTGTG 300
CGCAGCCTGT TTATNTCTCT NNTATTGTGT CN 332

SEQ ID NO:131
SEQUENCE LENGTH:332
SEQUENCE TYPE:nucleic acid
TOPOLOGY:linear
CLONE:HUMGS00145
SEQUENCE DESCRIPTION:
GATCTGTGAC CTTACCCCCA AACCTGTNCT CTCTGAAACA TGTGCTGTGT CCACTCAGGG 60
TTAAATGGAT TAAGGGCGGT GCAAGATGTG CTTTGTTAAA CAGATGCTTG AAGGCAGCAT 120
GCTCCTTAAG AGTCATCACC ACTCCCTAAT CTCAAGTATC CAGGGAGACA AACACTGCGG 180

AAGGCCGCAG GGTCCTCTGC CTAGGAAAAC CAGAGACCTT TGTTCACTTG TTTCTTTGTT 240
CACTTGTTTA TCTGCTGACC TTCCCTCCAC TATTGTCCTA TGACCCTGCC AAATCCCCNT 300
NTGATGAAAA ACACCCAAGN ATANTCANTA AA 332

SEQ ID NO:132
SEQUENCE LENGTH:333
SEQUENCE TYPE:nucleic acid
TOPOLOGY:linear
CLONE:HUMGS00146
SEQUENCE DESCRIPTION:
GATCGAGGTT GTTTGCAACG ACCGTCTGGG GAAGAAGGTC CGCNTTAAAT GCAACACGGA 60
TGATACCATC GGGGACCTTA AGAAGCTGAT TGCAGCCCAA ACTGGTACCC GTTGGAACAA 120
GATTGTCCTG AAGAAGTGGT ACACGATTTT NAAGGACCAC GTGTCTCTGG GGGACTATGA 180
AATCCACGAT GGGATGAACC TGGAGCTTTA TTATCAATAG ATGAGAATCC TCATCTTNCT 240
GCCCCGCTNT CCNNCTNCCA TCCTCATCCC CCACANTNGG GATAGATGCT TNGTTTGTAA 300
AAACTCANCN NAATAAAGAC TTAGATGTTG AAA 333

SEQ ID NO:133
SEQUENCE LENGTH:330
SEQUENCE TYPE:nucleic acid
TOPOLOGY:linear
CLONE:HUMGS00147
SEQUENCE DESCRIPTION:
GATCAGGTCT GTAAATGTGT ACTAAAAAAA TNAGAGTTTA TTTATAAACA AAATAGTTTA 60
TTTAAAGAGA AGGTCTCTTC CTTATTGATA TCATGGTATG CATTAATTCC ATTTGTTACT 120
ATTGTGCACA AAAGCCCTGT TCACAGGGGA ATGGTGTAAA CATTTATACT GTTTTGTTCA 180
CTGTATTTAG TAGACATAAC TGTTGAATAG TTACTGAATC ATGATGTAAA GAATATGTGA 240
CCATCTTCAG GTATGGGATT TCTGAACGTT TCAAATTTCA ATCAATGAGC ACTGTCAACA 300
CCCACAGGNG AGAATAAAAT TACCTGTGCN 330

SEQ ID NO:134
SEQUENCE LENGTH:326
SEQUENCE TYPE:nucleic acid
TOPOLOGY:linear
CLONE:HUMGS00148
SEQUENCE DESCRIPTION:
GATCGAACCA CTGCACTCCA GCCTNGGTGA CAGAGAGAGA CACTGCCTTN GAAAAAAAAA 60
GAATCTCACT CACTATCTAG AGAGGATTGT CAGANTATTC ACGATTCAGN TCTTGAAACT 120
TTGATTATGC AAAAGAGGTA TATATAAATA TTTCATTATG ATTCAGGTTT TAGGCTTTGC 180
AGCTTCTATA AGGTGTTCTC AGGTGGCCCT TGTACNNCTN AAAGCATCCT TTAGGAAATN 240
CTTTAAGGGN GGCTTTNTAT AAGGAATAGG NGGNTGTTGA ATTTTTACAG GGGGGTTTGG 300
GTCATTNAGN CCCCGATTNT GTANGN 326

SEQ ID NO:135
SEQUENCE LENGTH:325

SEQUENCE TYPE:nucleic acid
TOPOLOGY:linear
CLONE:HUMGS00149
SEQUENCE DESCRIPTION:
GATCATGANC TCTGAAAAAA AGAGAAACCT TATCTTTNCT TTGTGGTTCC TTTAAACACA 60
CTCACACACA CTTGGTCAGA GATGCTGTGC TTCTTGGAAG CAAGGNCTCA AAGGCAAGGT 120
GCACGCAGAG GGACGTTTGA GTCTGGGATG AAGCATGTNC GTATTATTTA TATGATGGAA 180
TTTCACGTTT TTATGTNAAG CNTGACAACA CCAGGCAGGT ATGAGAGGAA AGCAAGGCCC 240
GTCCATNGCT GTCCGTACNC TTACGGNTTG CTTGTNGGAG NCATTTNGGT ATTGTTTGTT 300
GTAANANCCA AAANGGGCTT TGGNN 325

SEQ ID NO:136
SEQUENCE LENGTH:326
SEQUENCE TYPE:nucleic acid
TOPOLOGY:linear
CLONE:HUMGS00150
SEQUENCE DESCRIPTION:
GATCNACGCT GTGCCTTGGC AGGGCACAAT GACCTTGTCG AAATCCACCT GTCAGGACGC 60
CTAGGGGTCT GTACCGGGCT GGCCTGTGCC TATNACCTCT NATGCACACC TCCCACCCCC 120
TGTATTCCCA CCCCTGGACT GGTGGCCCCT GCCTTGGGGA AGGTCTCCCC ATGTGCCTGC 180
ACCAGGAGAC AGACAGAGAA GGCAGCAGGC GGCCTTTGTT GCTCAGCAAG GGGCTCTGCC 240
CTCCCTCCTT CCTTCTTGCT TCTNATAGCC CCGGTGTGCG GTGCATACAC CCNCACCTCC 300
TGCAATAAAA TAGTAGCATC GGCAAA 326

SEQ ID NO:137
SEQUENCE LENGTH:324
SEQUENCE TYPE:nucleic acid
TOPOLOGY:linear
CLONE:HUMGS00151
SEQUENCE DESCRIPTION:
GATCAAGNNG CCCTGGAGGC GGTGGGCGGC ACCGTGGTTC TGGAGTAGCC TCCAGCTCGG 60
AGGACTTGTN TNCAGGGGTC CTGGGCCCCG GNCAAGGTCC CGCCCTCCCG TGGTCACTGG 120
CTCCGCCCCA GCACCAGGCG CCCAGTGGAG CCGTTTGGAA GAATTGCCTG CNGCACGAGC 180
GGGGCCGGAC AGGCGCACAG ACCTACTGTN GCGGGAGGAA GGGGCGGCTG CTGCCTGGTG 240
ACGGCACCCG GANGCCCACC AGGACGCGCC ACCGGTNAAT GTNNCTCTNG TGGCTGCTGA 300
GAAAAATACA CTGTGCAAGT CAAA 324

SEQ ID NO:138
SEQUENCE LENGTH:323
SEQUENCE TYPE:nucleic acid
TOPOLOGY:linear
CLONE:HUMGS00152
SEQUENCE DESCRIPTION:
GATCTCAGCT TTTGGTTTCC CATGATACCA TCTCTAGGGG TAGCAGCTGG CTATAATAAC 60
TAATGTCTGG ATTATCCTAA CTCTTCTGTT TGTNCTTTCA GGTATTTAAC AATGTTGTGA 120

CTAATTGGCT TCCATTATTT CCCTAGAGTA GGTTCTGTTA TNACCCTGGA ATATGTTTGT 180
AATAGAGTGG GCTATTACAA TCATCTAGGA TAAAGATAAT CGTGGCTTGG AATAAGGGTG 240
GTAGCAATGG AAGTGATGAG AAGTCATTAG ATGCAGAATA TATTTTATAG ATGGANTGTG 300
ATAAANTAAA AAATAAACTG GGN 323

SEQ ID NO:139
SEQUENCE LENGTH:322
SEQUENCE TYPE:nucleic acid
TOPOLOGY:linear
CLONE:HUMGS00153
SEQUENCE DESCRIPTION:
GATCCATTCT CGGAATTCAC AGAATTTTNA TAACATCTNA CTCTCAGGGG GCATGAAGTG 60
CATAATCTTC CCTAGATTAC AAAAAACATA TAGATGACGG GTTTGCCTAT NAAACTTCAG 120
TACTACAAGA AACATAAAAT ATTTAAATAT ATGANATTTA AATATATTTA AAATTATTAA 180
AGTAATATAA ACATTTTTNA GTGACTGTGT TATGTTTTTC TGGTTATTTT GTTTTCTACT 240
AGTATATTTT TCTGTAAAAA TTGTAAAACT ATATCAGCAA TTTCTAATGC CAANAANGTA 300
AAACCTNGTG TGTATAAATG CN 322

SEQ ID NO:140
SEQUENCE LENGTH:323
SEQUENCE TYPE:nucleic acid
TOPOLOGY:linear
CLONE:HUMGS00154
SEQUENCE DESCRIPTION:
GATCAGAGGC ACAAGTNCAG AGGCTGTGGT CATGCGGAAC ACTCTGTTAT TTAAGATGGC 60
TATCCAGATA ATCCTGAACA CTGTGTATTT ATTTAATTTA GACTACCAGC AAAGATTAAA 120
GCATGAAATG TAAAACATCT GATAAAACTT ACAGCCCCCT ACACCAAGAG TGTATCTGTG 180
AAAGAGCTCC TACACTTTGA AAACTTAAGA NTCCCTTNTC ATGAAGTTTG CCTGTNCTAG 240
AATTGTAAGA TTGTTAATTT CCNTCAATCT CTAGTGACAA CACTTAATTT CTTTNCTAAT 300
ANAAAAAGCC TNGTNGGTGN AAA 323

SEQ ID NO:141
SEQUENCE LENGTH:328
SEQUENCE TYPE:nucleic acid
TOPOLOGY:linear
CLONE:HUMGS00155
SEQUENCE DESCRIPTION:
GATCAAGTTT AAATGACTGT GCTGCCCCTT TCACATCAAA GAACTACTGA CAACGAAGGC 60
CGCGCCTGCC TTTCCCATCT GTCTATCTAT CTGGCTGGCA GGGAAGGAAA GAACTTGCAT 120
GTTGGTGAAG GAAGAAGTGG GGTGGAAGAA GTGGNGTGGG ACGACAGTGA AATCTAGAGT 180
AAANCCAAGC TGGCCCAAGG TGTCCTGCAG GCTGTAATGC AGNTTAATCA GAGTGCCATT 240
TTTTTTTTTG GGTTCAAANG NTTTTTAATT TNTTNGGAAT NGNNCCANTT TTTTNAATTT 300
NGCAANTAAA AANGTTTAAA ANCTTAAA 328

SEQ ID NO:142

SEQUENCE LENGTH:369
SEQUENCE TYPE:nucleic acid
TOPOLOGY:linear
CLONE:HUMGS00156
SEQUENCE DESCRIPTION:
GATCTCTGGC AGTGGAGGAA GTCTCTTTAA GAAAATAGTT TAAACAATTT GTTAAAAAAT 60
TTNCCGTCTT ATTTCATTTC TGTAACAGTT GATATCTGGC TGTCCTTTTT ATAATGCAGA 120
GTGAGAACTT TCCCTACCGT GTTTGATAAA TGTNGTCCAG GTTCTATTGC CAAGAATGTG 180
TTGTCCAAAA TGCCTGTTTA GTTTTTAAAG ATGGAACTCC ACCCTTTGCT TGGTTTTAAG 240
TATGTATGGA ATGTTATGAT AGGACATAGT AGTAGCGGTG GTCAGACATG GAAATGGTGG 300
GGAGACAAAA TTATACATGT GAAATAAACC TCAGTATNTT AATAAAGTAG CACGGNTTCT 360
ATTTGNAAA 369

SEQ ID NO:143
SEQUENCE LENGTH:324
SEQUENCE TYPE:nucleic acid
TOPOLOGY:linear
CLONE:HUMGS00157
SEQUENCE DESCRIPTION:
GATCATGAAG GAACACATAG CACCAAGAGA GGCCATGCTA AATCTCGCCC TGTCAGANNN 60
NTCCACACTT CTCCTTTGGG GAAGNCTTCC CTGTCCCCCT AGACTAAGTT AAATATTTCT 120
GCACAGTGTT CCCATGGCCC CTTGCATTTC CTTCTTAACT CTCTGTTACA CGTCATTGAA 180
ACTACACTTT TTTGGTCTGT TTTTGTGCTA GACTGTAAGT TCCTTGGGGA CAGGGCCTTT 240
GTCTGTCTCA TCTCTGTATT CCCAAATGNC TAACAGTACA GAGCCATGAC TCAATAAATA 300
CATGTTAAAA TGGGATGAAT GAAA 324

SEQ ID NO:144
SEQUENCE LENGTH:316
SEQUENCE TYPE:nucleic acid
TOPOLOGY:linear
CLONE:HUMGS00158
SEQUENCE DESCRIPTION:
GATCATTGAA CGAGACAGAA AGCGACCATC CTGGTTCACC CAGAATTGAC ACCAAAGATG 60
TTAAAAGGAT AACTTCACAG TAAATCATTT CTCCTGAAAT AGAGGAAGAT TCTTTACGTT 120
GTTGTNCTTG TTTTTAAATC ATCAGTATAG TTTAACACAT TCTTTCTAAG CAGTTTTGTG 180
TGGGATAATT TGAAGAATAT ATTATGAGTA ANCTCCGAAA ATTTTGTTTA TCCAAAGGCT 240
CANTGGATTA TGTTTCTATT ATNTACAAGG TTTTAAGTAA ACATANNNTT TCCNGNNCNG 300
AGNTTANAGN NATTTN 316

SEQ ID NO:145
SEQUENCE LENGTH:323
SEQUENCE TYPE:nucleic acid
TOPOLOGY:linear
CLONE:HUMGS00159
SEQUENCE DESCRIPTION:

GATCTTCATT TTATTGCATT AGTTCATGTA GATGGGCATC TCTATGAATT AGATGGGCGG 60
AAGCATTTCC AATNAACCAT GGTGAAACTA GTGATGAAAC TTTATNAGAG GATGCCATAG 120
AAGTTTGCAA GAAGTTTATG GAGCGCGACC CTGATGAACT AAGATTTAAT GCGATTGCTC 180
TTTCTGCAGC ATAGCTTGTC AATAATGGAA ACACCAAAAA CTGTATTATT TGCAACTAAA 240
TTTTCTCTGC CATACACTAA CTCAAAAATT TTGATATTTT CATTAACTTG ATGATTAAAC 300
TTTATGTGAG TTAANCTTTG AAA 323

SEQ ID NO:146
SEQUENCE LENGTH:319
SEQUENCE TYPE:nucleic acid
TOPOLOGY:linear
CLONE:HUMGS00160
SEQUENCE DESCRIPTION:
GATCTTACTG CTTGTNACTT GAATCCCGTG ATTGTNATAC ATCTCTGGTA TAAGCAACAT 60
TTGATTTTTG AAGTGTGTAG ACCATCTCTT CATATTTTCA AGATGTAATT TTACATTNCT 120
GCATTTTTAA AACAGTTTGG CCATAATCCT AGATGCACGC TTCTAATTCA TGTACCTGCA 180
CATGTGACCT TTGTGAACAG NAATTTGCAT GNATAATTNG TGTTTACTTG TAACTNTCTG 240
GTTATATACT GCTTATATCT GTGGATTCAA GTTACTGAAG TGGANTNCCA ATAGAAAGNA 300
ANCCCTAGGC CATGTTAAA 319

SEQ ID NO:147
SEQUENCE LENGTH:316
SEQUENCE TYPE:nucleic acid
TOPOLOGY:linear
CLONE:HUMGS00161
SEQUENCE DESCRIPTION:
GATCTCTGAT TACCAGCCTG ACATCAACAA ATCCCCTCAG TTACAACGTA TAGGTTAAAC 60
AAAGCTTTTA AAAGCTCATG TGGTATGACC TCAAGGTTGC TAACCTGGTC ACTCATGGTA 120
ATNAGAAACT CTGATTGGCA GCTTTGTATT TCTTGACTAA AAACCTAAAT AAACTGATTA 180
GGTTTTAGGC GTTCTTTCAA AGAGGTTCTT GAGAAGATTG AGAACTATCC TATTTGGTGC 240
TTAGTGAAAA GATTTTGAAT TACTGTACGT ACCAGTTGTT GCCATTTCTT TATTAAATTC 300
AGAAGTTTTT TTGCCN 316

SEQ ID NO:148
SEQUENCE LENGTH:319
SEQUENCE TYPE:nucleic acid
TOPOLOGY:linear
CLONE:HUMGS00162
SEQUENCE DESCRIPTION:
GATCTTGGAC AGCNTGGGTA TCGAGGCGGA CGACGACCGG CTCAACAAGG TTATCAGTGA 60
GCTGAATGGA AAAAACATTG AAGACGTCAT TGCCCAGGGT ATTGGCAAGC TTGCCAGTGT 120
ACCTGCTGGT GGGGCTGTAG CCGTCTCTNC TGCCCCAGGC TCTGCAGCCC CTGCTGCTGG 180
TTCTGCCCCT GCTGCAGCAG AGGAGAAGAA AGATGAGAAG AAGTAGGAGT CTNAAGAGTC 240
AGNTGNTGNC ATGGGATTTG GCCTTTTTTG GTTAAATTCC TGNTNCNCTG CAAATAAAGG 300
CTTTTTTTAC AGANGTAAA 319

SEQ ID NO:149
SEQUENCE LENGTH:313
SEQUENCE TYPE:nucleic acid
TOPOLOGY:linear
CLONE:HUMGS00163
SEQUENCE DESCRIPTION:
GATCAATGCC CTCATTAAAG CAGCCGGTGT AAATATTGAG CCTTTTTGGC CTGGCTTGTT 60
TGCANAGGCC CTGGNCAACG TCAACATTGG GAGCCTCATC TGCAATGTAG GGGCCGGTGG 120
ACCTGCTCCA GCAGCTGGTG CTGCACCAGC AGGAGGTCCT GCCCCCTCCA CTGCTGCTGC 180
TCCAGCTGAG GAGAAGAAAG TGGAAGCAAA GAAAGAAGAA TCCGAGGAGT CTGATGATGA 240
CATGGGCTTT GGNCTGTTTG ACTAAAACCT CTTTTATAAC ATNGTNCANT AAAAAGGCTG 300
GAGCTTTAAT AAA 313

SEQ ID NO:150
SEQUENCE LENGTH:313
SEQUENCE TYPE:nucleic acid
TOPOLOGY:linear
CLONE:HUMGS00164
SEQUENCE DESCRIPTION:
GATCCAGAAT CCACGGGGTC TGGAGCATAA GGTTTATCTC AAGTNTCAAT TGANCTGCCT 60
CCTCTTGTNA GGCAGGGACA ACTGGGAGGA TGAGCCCCAA GAGCCTCAAG AACCCAACAA 120
GGTGCCCCTA GAAGACACAG AGACAGATGA NCTTTGGGCA TCCTTGGAGG CAGCTGCCAA 180
GCGGANAGCT CTCGGGTTTG GAGCAGCCCC AAGGAGCTCT CCAAACGAGA CGGAGAAAGA 240
AGAAGCGGCC TGGGTCCACC AGCCCCTGAC GCCCCTGTNN CCACTTTGTA AATAAACTTG 300
CTGAACACCC AAA 313

SEQ ID NO:151
SEQUENCE LENGTH:313
SEQUENCE TYPE:nucleic acid
TOPOLOGY:linear
CLONE:HUMGS00165
SEQUENCE DESCRIPTION:
GATCATCAAC AAAGAAAGTC TGAGAAATTG TCCCGGAATA AAGGGGCCTA AGGAGACATA 60
ACATCTAAAT GTAATGTAGT ATCCTGGATG GACTCCTGCA ACAGAAAAAG AACTTTAAGT 120
AAAAATTAAG GGAATATTAA TAAAGTATGC ATTTTGGTTA ATAATGTATC AATATTGGTT 180
TATTAGTTGT GACAAATGTA CCAGAGGAAT GTAAAATGTC AACAATAAAG GAATTGGATG 240
TGGGGTCCAT GAGATGCTGT ACTATTTTTG CACTTTTCTT AAATCTAAAC TCTTATAAAT 300
TTAANCATAA AGN 313

SEQ ID NO:152
SEQUENCE LENGTH:317
SEQUENCE TYPE:nucleic acid
TOPOLOGY:linear
CLONE:HUMGS00166

SEQUENCE DESCRIPTION:
GATCTGGCCA TCTACGAGCC AAAGACTTTN AAATCTTTGG CTGCCTTGGC CAGTAGGAGG 60
CGACACGAAG GATTTNCTGC TGCCTTGGGG GATGGGAAGN AACCTGAAGG CATTTTTNCC 120
AGAGTGGTGC AGTACCACTN AGGACTGTTG CTGTATTGAT TAGGAAAAGA GACAGAGTAA 180
TTTNCAGTTT GTTTGATTTA TACTTTTGTT TATCTACAAC CCAATAACAG ACATGAGGGA 240
TGGCCCTGTC TCTCTGGGAC AGAGCCTCAA AGATGATGTC CATGTTTTGT GTGAATGAAA 300
CTCAAACACT CTTCAAA 317

SEQ ID NO:153
SEQUENCE LENGTH:310
SEQUENCE TYPE:nucleic acid
TOPOLOGY:linear
CLONE:HUMGS00167
SEQUENCE DESCRIPTION:
GATCCCCTGC CCCCTGTCCC CTGCCTCTTT TCCCAATTCC CTTCCTTATG CTGGACTTTT 60
AAAGCTTAAA AAAAATCCGA TTGAATATAA ATGCCTAATT TCATTCTTTG TGAAATGGTT 120
GCTTCCTCCT GATTCCCTAA TTGTGCTGTG TTCGTGTCTT GCACTGGAAT TCAACATTCC 180
CTTCTCCTTT TGTACTGTGT TGTGCTTGCT GTCTCTCCCG GACANCCTTA AAGACTGTCT 240
TTTTAGCAAA AAATTTCAGT AAAGTGTTTT CTGTAATCTT TTTTTAAAAG GTGAGAACTA 300
ATTATTGTCN 310

SEQ ID NO:154
SEQUENCE LENGTH:309
SEQUENCE TYPE:nucleic acid
TOPOLOGY:linear
CLONE:HUMGS00168
SEQUENCE DESCRIPTION:
GATCACCACG GTTTTCAGCC ATGCTCAGAC AGTGGTTCTT TGTGTAGGTT GTTCAACAGT 60
GTTGTGCCAG CCTACAGGAG GAAAGGCCAG ACTCACAGAA GGGTGTTCAT TTAGAAGAAA 120
GCAACACTAA TGATTCAAAC AGCTTCCTGA ATTTTAATTT TGTGTTGTCT CACAGAAAGC 180
CTTATCATAA ATTCCATAAT TCTAATTAAT TTACCAAGAT AATGTAATTA CATTTGGTTT 240
TGTAAGGTAT ACAGCAGTAA TCTCCTATTT TGGTGTCAGT TTTTCAATAA AGTTTTGATT 300
ATGGGCAAA 309

SEQ ID NO:155
SEQUENCE LENGTH:307
SEQUENCE TYPE:nucleic acid
TOPOLOGY:linear
CLONE:HUMGS00169
SEQUENCE DESCRIPTION:
GATCACATAC AGGGGAAAAG CCCTATGNAA TGTAACACAT GCAGGAAAAC CTTCTCTCAA 60
AAGTCAAATC TCATTGTACA TCAGAGAACA CACATAGGAG AAAANCCTTA TGANTGATTT 120
GGATATTAGA AATTNCCAGC CACAAGTCAG CCTCCATAAT GCNTCAGAGT CTTCACACTG 180
TGGAGANGGG CCTGTTGACA TCCTGATTGT TCANTAACTA TCCACAACCT CGCCTTATGT 240
TACTCCANNG TAACAGTAGG GGTTAANCCC ATAGNCTACA ACACCTNTNG GNTGGCTTTT 300

NTTAGGN 307

SEQ ID NO:156
SEQUENCE LENGTH:307
SEQUENCE TYPE:nucleic acid
TOPOLOGY:linear
CLONE:HUMGS00170
SEQUENCE DESCRIPTION:
GATCCACCAA CTTTGGCCTC CCAAAGTGTT CAGATTACAG GTGTGAGCTG CTACCATGCC 60
TAGCCCCAGC TTCTACTGCT TGANGGCTCT CTTTGGCATC TCCACACATC ATCCTTAGCA 120
GCCCAGACTG CATTTCTGTA GCAGCCTCTT CCCTGGTCTC TTCTTTCAGT CTCTCTGCCT 180
CTAATCCAGT GGCTTTAAGA ATTTTTTGGC TGTGACTTCC AGTAAGAAAT ACAATTTACA 240
TTGTGACCTA GTAAATATGT GTGTAAGATT TATTAACTGA AATAAAAATG TTATGATTTA 300
ATTTTTN 307

SEQ ID NO:157
SEQUENCE LENGTH:306
SEQUENCE TYPE:nucleic acid
TOPOLOGY:linear
CLONE:HUMGS00171
SEQUENCE DESCRIPTION:
GATCTGTCCC TGTGGTGGTG TCTAAGAATC GGACACCTTG GTTTTTGTGT TAGATTGAGC 60
TGGGCAGCTG CAATCAGCTA CTTAATATGC AAATTAGGCA CGTCCCATCT GTGGGTCCTT 120
GTTGGTGGCT AATGAAGTGA GGGGAGGGAG GGATGTCACC CCAAAAGTAG GCCCTCCCAT 180
TGGCTTTGGC CAGGCCAGAC ACTTCACATC GTTTACATGG TTCTGTGTAA TTTTAAAGTT 240
TATGTGTATA AAGCGACGGT TTTCTGTGAA CTGTATATTT TGTAAATAAA TATATTGCTA 300
CTTTGN 306

SEQ ID NO:158
SEQUENCE LENGTH:310
SEQUENCE TYPE:nucleic acid
TOPOLOGY:linear
CLONE:HUMGS00172
SEQUENCE DESCRIPTION:
GATCTTCAGA TAAATTCTGC CATTTTNATT TCACTTCCTG AAAGTNAGGG TCGGCTTGTN 60
AAAAGTTGTT AAACAACATG CTAAATGTGA AATGTCAACC CTCACTCTAA ACTTTCCCTG 120
TTCAGAGCAT CAGATGAAGA CTTCATTGGG TTTTATAGTG GCTTTCTGAT TTTNGGTAGT 180
CCATTGAAGA AGGGAGTTTG AAAGTTGTTG TATACTGTTA ACGATTGTCT GCCCATGTCC 240
TGCCTGAAAT ACCATGATTG TNTATGGAAA GTATCTTTAA TAAAGCTGGA TACAGTTTGG 300
CTTGGAGAAA 310

SEQ ID NO:159
SEQUENCE LENGTH:306
SEQUENCE TYPE:nucleic acid
TOPOLOGY:linear

CLONE:HUMGS00173
SEQUENCE DESCRIPTION:
GATCTACTTT GGAAAAACAA AGGATATCGT CAATGGGCTG AGGTCTGTNC AGACTTTTGC 60
AGACAAATCA AAACAAGAAG CTCTGAAGAA TGACCTGGTG GAGGCTTTGA AGAGAAAGCA 120
GCAATGCTAA ACCTCTGTTT CATGCTAACC AGACACGCCG TGCACTCGTT AGATTCCTTT 180
CTTAGAAAAC TCGTTTTCTG CTCCCTTCCC TCGTCCCTTC CCTCCCCGAC AGGTCACATA 240
ACAGCTGCAT CATTGACCGC ACAGCGCCAT CTCTCCCTGA GAATAAAGCC GATAGCCACC 300
NTCAAA 306

SEQ ID NO:160
SEQUENCE LENGTH:329
SEQUENCE TYPE:nucleic acid
TOPOLOGY:linear
CLONE:HUMGS00174
SEQUENCE DESCRIPTION:
GATCTATTGG ACTTTTTTTT GCAGGAAGTG CATTCTCTGG TCCTTCCCTA TTTTCTGTTC 60
TGGATGTCAG TGCAGTGCAC TGCTTACTGT TTTATCCACT TGGCCACAGA CTTTTTCTAA 120
CAGCTGCGTA TTATTTCTAT ATACTAATTG CATTGGCAGC ATTGTGTCTT TNACCTNGTA 180
TACTAGCTTG ANATAGTGCT GTCTCTGATT TCTAGGCTAG TTACTTGAGA TATGAATTTN 240
CCATAGAATA TGCACTGATA CAACATTACC ATTCTTCTAT GGAAAGAGAA ACTTTTGATG 300
ATGAAACAAT AAAGNTTTTA AATATCAAA 329

SEQ ID NO:161
SEQUENCE LENGTH:303
SEQUENCE TYPE:nucleic acid
TOPOLOGY:linear
CLONE:HUMGS00175
SEQUENCE DESCRIPTION:
GATCTGAATA AAGCAAATCT GCATAAATGG TAACCAGTAG CTCTACTTTN ATTTTTNATG 60
TTGCTTAACT GTTTTATTTG AAGGAAACCT GTGTGATTTA AAAAGTTATA GCTTTTGCAA 120
CTTTATTACT GGTTATATAC ATTTGGCCAT TATNATGTGC AAGCAATTGG AAAAAAAGTC 180
AAGTAAATGC TTGTTTTTGT AGTAGTTTGT TCTTGTTAAA AATGTTTATA TGATAATGTC 240
TGTAAACAGC ATCACTTTGA TTACAATAGA TGTAGTGTTG TAATAAACTG TTTAATGGGG 300
AAA 303

SEQ ID NO:162
SEQUENCE LENGTH:298
SEQUENCE TYPE:nucleic acid
TOPOLOGY:linear
CLONE:HUMGS00176
SEQUENCE DESCRIPTION:
GATCTCTCTA GCTTTGTCAT AGTTATGTGA TTTTCCTTTG TAGCTACTTT TGCAGGATAA 60
TAATTTTATA GAAAAGGAAC AGTTGCATTT AGCTTCTTTC CCTTAGTGAC TCTTGAAGTA 120
CTTAACATAC ACGTTAACTG CAGAGTAAAT TGCTCTGTTC CCAGTAGTTA TAAAGTCCTT 180
GGACTGTTTT GAAAAGTTTC CTAGGATGTC ATGTCTGCTT GTCAAAAGAA ATAATCCCTG 240

TAATATTTAG CTGTAAACTG AATATAAAGC TTAATAAAAN CAACCTTGCA TGATTAAA 298

SEQ ID NO:163
SEQUENCE LENGTH:309
SEQUENCE TYPE:nucleic acid
TOPOLOGY:linear
CLONE:HUMGS00177
SEQUENCE DESCRIPTION:
GATCTCCTTC ATCCCTCTCC AGAAGAGGAG AAGAGGAAAC ACAAGAAGAA ACGCCTGGTG 60
CAGAGCCCCA ATTCCTACTT CATGGATGTN AAATGCCCAG GATGCTATAA AATCACCACG 120
GTCTTTAGCC ATGCACAAAC GGTAGTTTTG TGTGTTGGCT GCTCCACTGT CCTCTGCCAG 180
CCTACAGGAG GAAAAGCAAG GCTTACAGAA GGATGTTCCT TCAGGAGGGA AGCAGCACCT 240
AAAAGGCACT CTGAGGTCAA GGATGAGGTG GGGAANCCAT CTCAATTAAC CACCATTTTT 300
TGGGTTAAA 309

SEQ ID NO:164
SEQUENCE LENGTH:295
SEQUENCE TYPE:nucleic acid
TOPOLOGY:linear
CLONE:HUMGS00178
SEQUENCE DESCRIPTION:
GATCTCGGTA AAAATCTACC ATTCCCTACA TATTTTCCTG ATGGAGATGA AGAGGAACTG 60
CCAGAAGATT TGTATGATGA AAACGTGTGT CAGCCCGGTG CGCCTTCTAT TACATTTGCC 120
TAACATCTTT GGACGTGGCA GAACCTTACA TATTCTGTGA GCTTCGATGA GCCAGAGTGA 180
TATCATAACC ACCAGAAATC ATACTCTCCT TTCTTAGTCA CAACAAAATC ACACATGTCA 240
TCTTTGTCAA GGGCATAAAT ATATCATTCA TACCCCCATT AAATTTTGTT AGAAA 295

SEQ ID NO:165
SEQUENCE LENGTH:289
SEQUENCE TYPE:nucleic acid
TOPOLOGY:linear
CLONE:HUMGS00179
SEQUENCE DESCRIPTION:
GATCCCCACC CCATGTGTTT TAAAAAGGCA GTAGCCTTTG CAGGGACCTG TCTGTCCCAA 60
CTGTTTGAAC AGTGTGCTCC TCAGATTCTG TGTTCAGAAG NCCCCTGNTG CATTGAGACT 120
TGAAACCTTT GGATAGGGGA AAAAATTATA TATATATATA TNNNTTTGTT CTGTTTGCAT 180
TTCTTAATTT GTGCTTGGAA TGTGTTGATG TGCACAGCTA ATGATTCAAT GCGAGACAAG 240
ATTGGCGTCT GTGTTGTGGA GGTTTCAAAT AAAGAGCACT CTTCATAAA 289

SEQ ID NO:166
SEQUENCE LENGTH:300
SEQUENCE TYPE:nucleic acid
TOPOLOGY:linear
CLONE:HUMGS00180
SEQUENCE DESCRIPTION:

GATCTGTAAA ATGTGATTTT TTACTTCCAC TTATAATACT TGTGATTGGG GAGGTTTGTG 60
GAAATTCAAT TATGATGAAA AACCTATCTT TTTTGTAATG TTGGCATACT TGGGGAATTT 120
AGTGGCAAAT ACATTCCCCA GCAGGCCTTT TGTTGGTTGC ACTAACTGCA AGGTTGCTGG 180
GAAGTAGAGT CCATTTGGTT GATGAGCTTT GACTGCGGTT TTGGAACCTT ACCTCTCCTC 240
CTTAGCCCAA TATGCTGTCT TGGGTCCTAT TCAAATAAAG TTATTTCTCC TGGTCTCAAA 300

SEQ ID NO:167
SEQUENCE LENGTH:292
SEQUENCE TYPE:nucleic acid
TOPOLOGY:linear
CLONE:HUMGS00181
SEQUENCE DESCRIPTION:
GATCAAAAAA CCATCTCCAC ATTTAAAAGA GATGTAAGGT GTATTCATAG GGATGGTGGC 60
TCAACAAATC AAGCAAACTG GAATCAAGGG GAGGGGGAAG GGAATNAAAT GGAAAGGGAG 120
GCTGATTCCC TTCCCCTGAC TTACCACTAA TTTACTAGGC TACCTACTTT NATGAGTAAC 180
CTCTCACAGC TACCCAGCAC ATGCCACAAT CCTATGCTCT TGCCTTCTTT NATCTGCACT 240
GTGTGAAGGG ACTCTTTTAA ATAAATNAGC AAGTGTCCTA AGCTATGTCA AA 292

SEQ ID NO:168
SEQUENCE LENGTH:292
SEQUENCE TYPE:nucleic acid
TOPOLOGY:linear
CLONE:HUMGS00182
SEQUENCE DESCRIPTION:
GATCTTGGCT GTATTTAATG GCATAGGCTG ACTTTTGCAG ATGGAGGAAN TTCTTGATTA 60
ATGTTGAAAA AAAACCCTTG ATTATACTCT GTTGGACAAA CCGAGTGCAA TGAATGATGC 120
TTTTNTGAAA ATGAAATATA ACAAGTGGGT GAATGTGGTT ATGGCCGAAA AGGATATGCA 180
GTATGCTTAA TGGTAGCAAC TGAAAGAAGA CATCCTGAGC AGTGCCAGCT TTCTTCTGTT 240
GATGCCGTTC CCTGAACATA GGAAAATAGA AACTTGCTTA TCAAAACTTA AA 292

SEQ ID NO:169
SEQUENCE LENGTH:333
SEQUENCE TYPE:nucleic acid
TOPOLOGY:linear
CLONE:HUMGS00183
SEQUENCE DESCRIPTION:
GATCCACATG AACGCACGCC TGAGATTTGG CCACTCACCT ATGTTTTGGG TGGATTGCCT 60
AGGAAAGCAA GTCATATGGC CATTGATAGT TCTCATGTAA TTAGTTTTGC TCACCACTAG 120
TACAGATGAC CCGTTTACAC GTGGCTTCCC TCGGAAGCCT CCTCAACAGT AGCTGGTGTG 180
AAAGACTAAA TCAGTAGAGT TGGAAAAGCT TTATAACCGG TGTGTCATAT GCTTGCTATT 240
TAAAGCTGTG TGTTGGTTTT GTTTTTCTGC CACATTCACT AGTTTTTTAA TAAATATTTT 300
CCAAAANTGG AAAAAAANAA NNCCCNCNNC CCN 333

SEQ ID NO:170
SEQUENCE LENGTH:401

SEQUENCE TYPE:nucleic acid
TOPOLOGY:linear
CLONE:HUMGS00184
SEQUENCE DESCRIPTION:
GATCTGGANG GGACACGTCA TGCCTTGGGC CTAGAATACC CTGATGNGAA AAGAGAAGAN 60
AAAGGGAGGC CATATCTACA ACACAGCCTC TCGGCACTGC TGCTCCTTAT TTTAACTTTG 120
TNTTGCATTG TCCTGTATTT ATCACAGTTT CTGTTGAACA GCTTTTCAAG TATTTGGGGA 180
GTTTATCTTG CCATCCTCCC CTTCTGGTTC TCTGCACCCA CCTGTCCCAC TGCAGTTCCT 240
TCCGTGCTCT GTGACTTTAA GAGAAGAAGG GGGGAGGGGT CCCGGATTTT ATGTTTGTTT 300
GNTNTTTCTC CTTAGCAGTA GGACTTGATA TTTTCAATTT TGGAAGAACT AAAAGATGAA 360
TAAACTGGGT TTTTTTTGTT GTTTGNTTTT GNAAATTCAA A 401

SEQ ID NO:171
SEQUENCE LENGTH:305
SEQUENCE TYPE:nucleic acid
TOPOLOGY:linear
CLONE:HUMGS00185
SEQUENCE DESCRIPTION:
GATCGAGCTC GCCTATNAGC AAGTGGCAAA CCCCCTCAAA TAAGCCCCTC CTGGGACTCC 60
CTCAACCCCC TCCATTTTCT CCACAAAGGC CCTGGTGGTT TCCACATTGC TACCCAATGG 120
ACACACTCCA AAATGGCCAG TGGGCAGGGA ATCCTGGAGC ACTTGTTCCG GGATGGTGTG 180
GTGGAAGAGG GGATGAGGGA AAGAAATGGG GGGCCTGGGT CAGATTTTTA TTGTGGGGTG 240
GGATGAGTAG GACAACATAT TTCAGTAATA AAATACAGAA TAAAAATCAA GTGTTTTTAC 300
GCAAA 305

SEQ ID NO:172
SEQUENCE LENGTH:289
SEQUENCE TYPE:nucleic acid
TOPOLOGY:linear
CLONE:HUMGS00186
SEQUENCE DESCRIPTION:
GATCTGAGGC AAGCTGGACA GGAGAGGTGG ATATTTNTTG ATGGAAGAAT TCAAGTTTAT 60
AATCAATTCC CACTTAGCAC CTACTGTGTG CTAGGAACTT GAATGTGTAT GTTTGACAAG 120
TCCTGCTTGG CCTGATGGGT GGGAGANGGA ACCTGAGCCT GGCTGAGATG GCTAGGCGGA 180
GGGCTTTGAA GTCCAAGCAG CTGAACTGGC TGGGTGGGTT TCTACCTTTG AAACTGCAAG 240
ACTTNTTTTG GAGCTCTTAA TTACAATATC TGATATTTTT ACAGTCTGN 289

SEQ ID NO:173
SEQUENCE LENGTH:286
SEQUENCE TYPE:nucleic acid
TOPOLOGY:linear
CLONE:HUMGS00187
SEQUENCE DESCRIPTION:
GATCCCTACC CTTNCCGTTG GTCTCTNTCG CTGACTCGAG GCACCTAACA TCCATTCACA 60
CCCAACACAG GCCAGCGACT TCTGGGGCTC AGCCACAGAC ATGGTTTGTN ACTNTTGAGC 120

TTCTGTTCCT AGAGAATCCT AGAGGCTTGA TTGGCCCAGG CTGCTGTNTG TNCTGGAGGC 180
AAAGAATCCC TACCTCCTAG GGGTGAAAGG AAATNAAAAT GGAAAGTTCT TGTAGCGCAA 240
GGCCTGACAT GGGTAGCTGC TCAATAAATG CTAGTNTGTT ATTTCN 286

SEQ ID NO:174
SEQUENCE LENGTH:290
SEQUENCE TYPE:nucleic acid
TOPOLOGY:linear
CLONE:HUMGS00188
SEQUENCE DESCRIPTION:
GATCGGGTTC TAAAGGAAAG GGTGGAGAGA TTCAACCAGT TAGCGTGAAA GTTGGAGATA 60
AAGTTCTTCT CCCAGAATAT GGAGGCACCA AAGTAGTTCT AGATGACAAG NATTATTTCC 120
TATTTAGAGA TGGTGACATT CTTGGAAAGT ACGTAGACTG AAATAAGTCA CTATTGAAAT 180
GGCATCAACA TGATGCTGCC CATTCCACTG AAGTTCTGAA ATCTTTCGTC ATGTAAATAA 240
TTTCCATATT TCNCTTTNAT AATAAACTAA TGATAACTAA TGNCATCAAA 290

SEQ ID NO:175
SEQUENCE LENGTH:284
SEQUENCE TYPE:nucleic acid
TOPOLOGY:linear
CLONE:HUMGS00189
SEQUENCE DESCRIPTION:
GATCAAGGCG CGGACGTATC TACGACCACA TCAACGAGGG GAAGCTGTGG AAACACATCA 60
NGCACAANTA TGACAACAAG TAGTTCCTTG GNGGCCCTAT CCAGGCCAGA AGGCCANNGC 120
CACCCAGCAG CTGTTTGCCA GAGCTGGAGC TCAGTTGAAG ATGATGCTCA AGGTACTCTT 180
CATGGCCACC ATTCGTGTTG GAAGAACGNT TTACTNCNNA CAGCTCTTTA CCTTCTGTGT 240
GTTTNAGNTG TTAGNAGATN TCAGGAATAA TGTGATTGCC TTGN 284

SEQ ID NO:176
SEQUENCE LENGTH:282
SEQUENCE TYPE:nucleic acid
TOPOLOGY:linear
CLONE:HUMGS00190
SEQUENCE DESCRIPTION:
GATCAGAACA TGAAATGCCC TCCTAAATGT CAGCTGTTGT CACACAGTAG CTCCAACACT 60
TTGAGCATTT TTAAGGGAGT GGCCTCATTT CACTAGAGAC AAATCTTTAA GAATAGTTCT 120
AAAATTGGGC TTGTGATTTC CATTTCTGAT GTCTCCAGAT TGGCACCCCT TTCTAGTTCA 180
ATGCCTCACG AGATTTTGCC AGGGGCATCC AAGGCAAACA ATCCCAATCT TTCTATATAA 240
ANTGTATTCA AGCAAACATC AAATAANTTT CTGGGATATT TN 282

SEQ ID NO:177
SEQUENCE LENGTH:278
SEQUENCE TYPE:nucleic acid
TOPOLOGY:linear
CLONE:HUMGS00191

SEQUENCE DESCRIPTION:
GATCAGAGTT TGAAATGAAA TGTTTGTCAG GGTGTTGGAA AAATTTTGGT GAGTTCTGCA 60
CATTTCCCCT GGTTCAGGCT GGGCATGGAC CAGCCTTCAG ATGGCAGAAG TGGAAGATGA 120
GCCTACTTGT GAGCGATGTG ACTTTAAGGA AATGAAGACT GGGGAAGAAT AATTAGTGTT 180
TATAAGACAT TTAAGAGGCC CTTTTTCATA TACTGACTCA CTGATGAATC AGCATTNGCA 240
TTNTATGGAA NAATATAAAT CCAAAGAAAT AATTTAAA 278

SEQ ID NO:178
SEQUENCE LENGTH:281
SEQUENCE TYPE:nucleic acid
TOPOLOGY:linear
CLONE:HUMGS00192
SEQUENCE DESCRIPTION:
GATCAAAATA ACCCTCGTAA AAATATATGT ANGGGGTACA CAAAGTAAGC CTCTTTATGA 60
AACAATTGAG GATAATNATG TGAAAGGTTT TAATGATGAT GTTCTACTTC ANATAGTTCA 120
CTTTCTACTG AATAGACCAA AAGAAGAAAA ATCACAGCTG TTGGAAAACT GAAAAAGCAT 180
ATTTNATTGA GAACTGTGGG AATATTTAAA TTTTACTGAA GGACCAATAA TGATGAGATT 240
TGTAACTGTC AACTATTAAA TACATTGATT TTTGAGACAA A 281

SEQ ID NO:179
SEQUENCE LENGTH:278
SEQUENCE TYPE:nucleic acid
TOPOLOGY:linear
CLONE:HUMGS00193
SEQUENCE DESCRIPTION:
GATCAGGCCC AGGAAGGGCA CAGGGGCTGA GCACTACAGA AGTCACATGG GTTCTCAGGG 60
TATGCCAGGG GCAGAAACAG TACCGGCTCT CTGTCACTCA CCTTGAGAGT AGAGCAGACC 120
CTGTTCTGCT CTGGGCTGTG AAGGGGTGGA GCAGGCAGTG GCCAGCTTTG CCCTTCCTGC 180
TGTCTCTGTT TCTAGCTCCA TGGTTGGCCT GGTGGGGGTG GAGTTCCCTC CCAAACACCA 240
GACCACACAG TCCTCCAAAA ATAAACATTT TATATAGN 278

SEQ ID NO:180
SEQUENCE LENGTH:278
SEQUENCE TYPE:nucleic acid
TOPOLOGY:linear
CLONE:HUMGS00194
SEQUENCE DESCRIPTION:
GATCTACATT AATATCAAGT CTTGACTCCC TACTTCCCGT CATTCCTCAC AGGACAGAAG 60
CAGAGTGGGT GGTGGTTATG TTTGACAGAA GGCATTAGGT TGACAACTTG TCATGATTTT 120
GACGGTAAGC CACCATGATT GTGTTCTCTG CCTCTGGTTG ACCTCNACAA AAACCATTGG 180
AACTGTGACT TTNAAAGGTG CTCTTGCTAA GCTTATATGT GCCTGTTAAT GAAAGTGCCT 240
GAAAGACCTT CCTTAATAAA GAAGGTTCTA AGCTGAAA 278

SEQ ID NO:181
SEQUENCE LENGTH:277

SEQUENCE TYPE:nucleic acid
TOPOLOGY:linear
CLONE:HUMGS00195
SEQUENCE DESCRIPTION:
GATCTCTCCC CCGTGAAGGA GTTGAGCACA TTAGCAACAA TGTACATTAA TTTTGGATTT 60
TCATTTTCAT GTTTTATTTT GTAAATATTA TCTGATGTTT GGAGCTTGAG TATACAGACT 120
GTAAATATAG TTCTTGTATT TGTACTAATT CTGATTCTTT TGCTNNCNNG CCTTAGATGT 180
GCAATGCAGA CACTATCTAA CTGTGTGTGG TAACCTTGCG TCACGGAGCT GTTAGTGAAC 240
GAGGTAAAAA TAATAAAGGT ACAGCCAGTG CATCAAA 277

SEQ ID NO:182
SEQUENCE LENGTH:282
SEQUENCE TYPE:nucleic acid
TOPOLOGY:linear
CLONE:HUMGS00196
SEQUENCE DESCRIPTION:
GATCAAAACN GATTTACGGT GGGGGAGGAA TNTGAGCTGG AGACAATNAC AGGGGAGAAA 60
GTCAAGACAG TGGTTCAGTT GGAAGGTGAC AATAAACTGG TGACAACTTT CAAAAACATC 120
AAGTCTGTGA CCGAACTCAA CGGCGACATA ATCACCAATA CCATGACATT NGGTGACATT 180
GTCTTCAAGA GAATCAGCAA GAGAATTTAA ACAAGTCTGC ATTTCATATT ATTTTAGTGT 240
GTAAAAATTA AATGTAATTA AAAGTGANCT TTNGTTTTTA AA 282

SEQ ID NO:183
SEQUENCE LENGTH:277
SEQUENCE TYPE:nucleic acid
TOPOLOGY:linear
CLONE:HUMGS00197
SEQUENCE DESCRIPTION:
GATCCAGATG ACCGTGGTNG TTGGGGTATA TCTCCTCGAG GAGCTGGTTA CACCTTTGGG 60
CAAGATATTT CTGAGACATT TAATCATGCC AATGGNCTCA CGTTGGTGTC TAGAGCTCAC 120
CAGCTAGTGA TGGAGGCATA TAACTGGTGN CNNGGCCCGG AATGTAGTAA CGATTTTCAG 180
TGCTCAAACT ATTGTTATCG TTGTGGTAAC CAAGCTGCAA TCATNGANCT TTTGCGATAC 240
TCTAAAATAC TCTNTCTTGC AGTTTCACCN AGNNNNN 277

SEQ ID NO:184
SEQUENCE LENGTH:277
SEQUENCE TYPE:nucleic acid
TOPOLOGY:linear
CLONE:HUMGS00198
SEQUENCE DESCRIPTION:
GATCTGAGAT TTCCGTGTTT GGCTGAACCT GGGAGACCAG CTGGGCCTCC AAGTAGGATA 60
ACCCCTCACT GAGCACCACA TTCTCTAGCT TCTTGTTGAG GCTGGAACTG TTTCTTTAAA 120
ATCCCTTAAT TTTCCCATCT CAAAATTATA TCTGTACCTG GGTCATCCAG CTCCTTCTTG 180
GGTGTGGGGA AATGAGTTTT CTTTGATAGT TTCTGCCTCA CTCATCCCTC CTGTACCCTG 240
GCCAGAACAT CTCACTGATA CTCGAATTCT TTTGGCN 277

SEQ ID NO:185
SEQUENCE LENGTH:277
SEQUENCE TYPE:nucleic acid
TOPOLOGY:linear
CLONE:HUMGS00199
SEQUENCE DESCRIPTION:
GATCGGTTTT TGTTTCCTGC TTACCATATG ATTGTAAATT GTTTTATGTA TTAATCAGTT 60
AATGCTAATT AATTTTTGCT GATGTCATAT GTTAAAGAGC TATAAATTCC AACAACCAAC 120
TGGTGTGTAA AAATAATTTA AAATTTCCTT TACTGAAAGG TATTTCCCAT TTTTGTGGGG 180
AAAAGAGCCA AATTTATTAC TTTGTGTTGG GGTTTTTAAA ATATTAAGAA ATGTCTAAGT 240
TATTGTTTGC AAACATAAAT ATGATTTTAA ATTCTCN 277

SEQ ID NO:186
SEQUENCE LENGTH:276
SEQUENCE TYPE:nucleic acid
TOPOLOGY:linear
CLONE:HUMGS00200
SEQUENCE DESCRIPTION:
GATCCTGACA CTGACATGAA GGCAAGCCTT GATTTCGTAT GAACGTTGCT GAAGTGGTAA 60
TTGAGGAAAA CAGTTCCCCA GATTGTTAAG AGTTCACTGA AGATATTGAC ACAATTTNNA 120
AAAATCAGTA AAGGAATGTA TATAATATTG CNCTCGTGTT TTACAGTAAG ATTTGTTGCT 180
CTCAGACTGT GTAAAACAAA ATTNATNGNT GTTTTCTGCA TNTTAAAAAA TCTTATTGTA 240
CCANCTGGTA ANCTATTAAN TGCCTATAAN NCTAAA 276

SEQ ID NO:187
SEQUENCE LENGTH:275
SEQUENCE TYPE:nucleic acid
TOPOLOGY:linear
CLONE:HUMGS00201
SEQUENCE DESCRIPTION:
GATCAAGGAN GAAAGANTGA CTTCAAATAT GCCTTGTTAG TGTAAATGTG ACTTNTNGAA 60
CTGTATGAGT ATTTTAAGAT TATTNGAGTA AAGTAAGTTT TAAAAAGCAG TCCCTAATCA 120
TCAAAAGTAA AAAACTCTTG ATGTAGTCAT ATAACCACAC TAAGAACTCT TCCAGGTGAC 180
TTCAAAACAT AGGACAGTAC ATCTCTAGTA GANTNTGCCC TGAGAATGAA AAGAATGTAA 240
CAGTGTTAGT ATTTTGAATA AACATGTTAT TACTN 275

SEQ ID NO:188
SEQUENCE LENGTH:273
SEQUENCE TYPE:nucleic acid
TOPOLOGY:linear
CLONE:HUMGS00202
SEQUENCE DESCRIPTION:
GATCACTTTT TTTAGAGTGA AGAAAGAACA ANCTTGTTTT TTGTGTTTTT TAAAGGAATA 60
TAAAATAATG AAGGATGTAT AATTGATGCC AAATAAGCTT GTNCTTTAGT CACACCGACG 120

TCTTATTTTT CCCTTTAGGC CAGTTCTGTT TTTAAGGTGT ACATGGNCAA TGTTACAGTG 180
TAAGAAACTC CATATCCATA TGTNCCCATT CGCATTTTGT ATTGGTTCAT GTATACCATT 240
TTTNCAAAAN ANANGGAANA ANNGGNAGTN CTN 273

SEQ ID NO:189
SEQUENCE LENGTH:271
SEQUENCE TYPE:nucleic acid
TOPOLOGY:linear
CLONE:HUMGS00203
SEQUENCE DESCRIPTION:
GATCTGAAAA GCTCACTTTA AACTCATACT ACATTCGTNA CGAGTATTTN ACGTTAACAT 60
AATTGAAAAG TACAAGGTCC AAGCTGGCTT TCAAATNATG TCTAAACAGA AATGGGACAA 120
ATAGACTTGA AAATAGAAGG GATTTATTCC ACCCCTGCAA GGGTAAGAGT CAGGTGAGAG 180
TCCCTTGGTG AGTCATTTGT ACATCAGTGT CATTTCTTCT TAACCTCTGA AGAAAGATGG 240
GCATCAGAAA TAAAGACAAA GCACTATCAA A 271

SEQ ID NO:190
SEQUENCE LENGTH:270
SEQUENCE TYPE:nucleic acid
TOPOLOGY:linear
CLONE:HUMGS00204
SEQUENCE DESCRIPTION:
GATCTGCCAC GAGGGCAGCA GCCAAGAGGA CTTGCTCTCT CCATGTGAAT GTACAGGGAC 60
CTTGGGGACA ATTCATCGGA GCTGCCTGGA GCACTGGCTG TCATCCTCAA ACACCAGCTA 120
CTGTGAACTC TGCCACTTCA GGTTTGCAGT CGAGCGAAAA CCCAGGCCGT TAGTGGAGTG 180
CCTGGGAAAC CTTGGCCCCC AGATTGGGAG GGGNCTTTTT TTTGGNGANA TGGTNNNTTT 240
TGGGTTNTAA ATCNCCNGGG NNCCATNTNN 270

SEQ ID NO:191
SEQUENCE LENGTH:166
SEQUENCE TYPE:nucleic acid
TOPOLOGY:linear
CLONE:HUMGS00205
SEQUENCE DESCRIPTION:
GATCTCTGAG CTGCCCAGCA TAGTCCAAGA CCTAGCCAAT GGCAACATCA CATGGGCTGA 60
TGTGGAGGCC AGGTATCCTC TGTTTGAAGG GCAAGAGACT GGTAAAAAAG AGACAATCGA 120
GGAATGAGGA CAATTTTGAC AACTTTTGAC CACTTGCGCT AATAAA 166

SEQ ID NO:192
SEQUENCE LENGTH:276
SEQUENCE TYPE:nucleic acid
TOPOLOGY:linear
CLONE:HUMGS00206
SEQUENCE DESCRIPTION:
GATCTACATG AGAAGTATAG TGGCTCTACC CCCTGAAAGA GGGTGGATGC AGCTGCTTGT 60

GTTTCTTGGG GTGACTGTCA TTGGTAATAC GGACACAGTG ACCCATCCTC CATCCTATTT 120
ATAGTGGAAG GGCCTTCAAT TGTATCAGTA CTTTNTTTTA AGCTCTGGCA CATTGACCTC 180
TATGTGTTAC CAGTCATTAA TGAGCTGCTG CAGAGGTGAC TATTTGTTTT ACTTTCTTGG 240
ATGTTAACAT TACACTACTC ACTACTCAAT CTCAAA 276

SEQ ID NO:193
SEQUENCE LENGTH:273
SEQUENCE TYPE:nucleic acid
TOPOLOGY:linear
CLONE:HUMGS00207
SEQUENCE DESCRIPTION:
GATCTGACAT TTGACATGAA CACAAAGTTG CTAGATGCTC TTGTTGACTT CCAGCAGATG 60
GGATGGGGGA AACACAGCAG TTCTTGGTAA AGTCCTTTGT AATAATAGTT TGATTTTTTT 120
ATTTCGAGAG AATCTTTCAT TTTCCTATGT ATGCTTTTTN CCTTTTTTGC CCAGTTTCCT 180
TATCACTTGC TGTAGATGGC TTATNTNGCA TTCATGCAGA CTATGTTGCA AGTCTGTTTC 240
ATCTAGTAAA CTGAAAATNA TTGCTTAATC AAA 273

SEQ ID NO:194
SEQUENCE LENGTH:267
SEQUENCE TYPE:nucleic acid
TOPOLOGY:linear
CLONE:HUMGS00209
SEQUENCE DESCRIPTION:
GATCCCAGAG AGGGAAGAGA ACCAGGGGCC CTTTTCTCTT TNAGAATTTN CTTTTAATCA 60
GCCCACCTTT TGACTCCCCG CCCGCCCCAA TCCATTATNT TTTTCTGCCT TCCGGGTCCC 120
ATCCTTAGAG CTCGAGTCGT TCCTTCCCCT CCTGGATTCC GGGTAGCAGA GGCAGCGCCG 180
CAGGAGGCGG GTGCCCGTTT GTCCCAGGNC TGGGCGTGGT GGAGNNAGGG GTGCTGGAAC 240
AATAAACGGC ACNNNNCAAA TGTCAAA 267

SEQ ID NO:195
SEQUENCE LENGTH:384
SEQUENCE TYPE:nucleic acid
TOPOLOGY:linear
CLONE:HUMGS00210
SEQUENCE DESCRIPTION:
GATCCAAAAC ACTACTCAGC TCTCTTGCAC TGAGGAAATT TTTCCCCCTA CATTGACTCC 60
TGGCCTACAT CAGCCAAACT TAACCTTGGT GGGGTTTGGA TTTGATAGCC AATTAGTTCT 120
GTGCTGGTTG CAAAGAATTG ATATTTAGAT GGTTTTTAAT ACTCAGCAGA TTGTCTTCCT 180
TTATATTGTG TCTTTTTTAT GTNGCATGTN GCTTTTGTTA TCAGCCTGAT TTTTTGCTCA 240
GTATATGATA GTNCTGCTGA TGTTTTGTTT ATTGGGCAGA CATATCTTCA TTAAGAGTTT 300
TTGGAAAACT CATCAAATTC GATGAATACA TTTTCTTCAT AACCCATTTG GAATTATTCC 360
TAATAAAATG ATAAAATACG TAAA 384

SEQ ID NO:196
SEQUENCE LENGTH:277

SEQUENCE TYPE:nucleic acid
TOPOLOGY:linear
CLONE:HUMGS00211
SEQUENCE DESCRIPTION:
GATCTAAAAA AATTCAGAAG AAATATGATG AAAGGAAAAA GAATGCCAAA ATCAGCAGTC 60
TCCTGGAGGA GCAGTTCCAG CAGGGCAAGC TTCTTGCGTG CATCGCTTCA AGGCCGGGAC 120
AGTGTGGCCG AGCAGATGGC TATGTGCTAG AGGGCAAAGA GTTGGAGTTC TATCTTAGGA 180
AAATCAAGGC CCGCAAAGGC AAATAAATCC TTGTTTTGTC TTCACCCATG TAATAAAGGT 240
GTTTATTGTT TTGTTCCCAC ATTTATGTTG CCTGAAA 277

SEQ ID NO:197
SEQUENCE LENGTH:271
SEQUENCE TYPE:nucleic acid
TOPOLOGY:linear
CLONE:HUMGS00212
SEQUENCE DESCRIPTION:
GATCGGCGGC GCGCAGNAAC CGCTCCTACA GCAAGCTGCT GTGCGGCCTG CTGGCCGAGG 60
CGCCTGCGCA TCAGCCCGGA CAGGGTCTAC ATCAACTATT ACGACATGAA CGCGGCCAAT 120
GTGGGCTGGA ACAACTCCAC CTTCGCCTAA GAGCCGCAGG ACCCACGCTG TCTGCGCTGG 180
CTCCACCCGG GAACCCGCCG CACGCTGTGT TCTAGGCCCG NCCACCCCAA CCTTCTGGTG 240
GGGAGAAATA AACGGTTTAG AGACTAGGAA A 271

SEQ ID NO:198
SEQUENCE LENGTH:264
SEQUENCE TYPE:nucleic acid
TOPOLOGY:linear
CLONE:HUMGS00213
SEQUENCE DESCRIPTION:
GATCCTGTAG TGTTCCTGGA GAAGCTAGAG CCTGATTGTA GGCTACTACT CATCAATTAA 60
CTTCTACAGT GGAGACTACT TCTGGGACTG GAATATAAAA AAGAATCAAA GGTTCTGATT 120
TTNAGTTGCA ATAAAGGGAA AGACCATGCT CATAGCAGTG CCAACATCTG AAGTGTGGAG 180
CCTTACCCAT TTCATCACCT ACAACGGAAG TAGTTAACTG GAAGAGATTA CCAAGAGAAT 240
AAAAAGAGAC TCATTCAGTG GAAA 264

SEQ ID NO:199
SEQUENCE LENGTH:257
SEQUENCE TYPE:nucleic acid
TOPOLOGY:linear
CLONE:HUMGS00215
SEQUENCE DESCRIPTION:
GATCTGCCTT AAAGAAAAGA AAATTTTAGA AAGAAATATT GTTGCTCAGT GTTGTTAATA 60
TAGCTCAAGA ATTGAGTTTA TATTTGCAGT ATGCTATAAA TGATACCCCC CTACCACACC 120
CACACACACA GTTTTTGTCT AATGAAAATG TTGCTGTGAT TATTTATAAT TGGTAGTATT 180
TCTTCCAGAA GAAGCTAAAA TAAGACTGGC ACTTACCCTG AAGTGCATTA ATAAAACCAC 240
ACTTTAAAAT TAANAAA 257

SEQ ID NO:200
SEQUENCE LENGTH:374
SEQUENCE TYPE:nucleic acid
TOPOLOGY:linear
CLONE:HUMGS00216
SEQUENCE DESCRIPTION:
GATCAACCTG AGCNTGGAGA ATGCCTGGGG CATTTAACGC TGCGTCATTG ACATCTGCAT 60
GAAGCTGGAG GAGGGCAAAT ACCTCATCCT CAAGGACCCC AACAAGCAGG TCATCCGTNT 120
CTACAGCCTC CCTGATGGCA CCTTCAGCTC TGATGAAGAT GAGGAGGAAG AGGAGGAGGA 180
AGAAGNGGAA GAAGATGNGG ANGAAACTTA AACCAGTGAT GTGGAGCTGG AGTTTNTCCT 240
TCCACCGAGA CTACGNGGGC CTTTNATGCT TAGTGGAATG TNTGTCTAAC TTTGCTCTCT 300
TGACATTTTA GCAGTTGAAA TTAAATTATA TANTCTGTTT TNNGTCTTTT NAAATAAANA 360
AAANNANATT NTGN 374

SEQ ID NO:201
SEQUENCE LENGTH:256
SEQUENCE TYPE:nucleic acid
TOPOLOGY:linear
CLONE:HUMGS00217
SEQUENCE DESCRIPTION:
GATCTGAACT ATTTGCTTTC TCTTCAAGAT AAGTTGTATT TTACCATGGA AAAATACAGT 60
ATCTAACATT ACCATTCACG TTAAATGAAG TTTCCTCATA ACATTTATCT TTAGTTTTAT 120
GAAGTCATCG TGACCAATGT TACAGTAATT TCTGTTAGCT GATTGTGGTA AACAATGTTT 180
AATGNGAAAA GAAATTAAAA CTTTCTTCAT CTGTTGTAGA ATATTTCTCT TCTTTAAAAT 240
GGCTTCTATT CATAAA 256

SEQ ID NO:202
SEQUENCE LENGTH:256
SEQUENCE TYPE:nucleic acid
TOPOLOGY:linear
CLONE:HUMGS00218
SEQUENCE DESCRIPTION:
GATCCCCACT GGAGCAGCCT CTGCAAAAGG GAGCCCATGT AGTGGCCAGG GGCTGTCCAA 60
ACTCCAGCTT CTTCCCCTGG GAAAAAACCC AAAGAACCAA AAACAAACCA CCCCAAGGAT 120
AATAATAGCT ACACTGCTAG CTTCTCAAGT TCTTGTGAAA AACAATTTAC ATAATGACAC 180
AGTAGATGTG GAACACCTAG CCAGTGCCTG GGCAGGTCCC TATTATCATA AATGAACATA 240
AAGTGCTCTA AAAACN 256

SEQ ID NO:203
SEQUENCE LENGTH:265
SEQUENCE TYPE:nucleic acid
TOPOLOGY:linear
CLONE:HUMGS00219
SEQUENCE DESCRIPTION:

GATCCAAATC AGGAAAAGAC GGCGCCACTA CAATGGAGAA GCATATNAGG ATGATGAACA 60
TCATCCCAGA GGTGGTGTTC AGTGTCAGAC CTCTTAATGG GCCAGTGAAT AACACTCACT 120
GCTGGCATTT AATGTGCAGT AGTGAATGAG TGAAGGACTG TAATCATAAT ATGCTCACTA 180
CTTGCTCTTG TTTTTGTTTT AATAAACTAT AGTAGTGTTA TAANNNGTTA AATGAAGAAT 240
AAACGCAAAT ATAANAGCTC TGAAA 265

SEQ ID NO:204
SEQUENCE LENGTH:253
SEQUENCE TYPE:nucleic acid
TOPOLOGY:linear
CLONE:HUMGS00220
SEQUENCE DESCRIPTION:
GATCTAAAGA GACACTTAGA GACTCTTGGG GACCTCAGAT TTCCACCNCT CCACAGCATT 60
CTGCCTACAC AGTTCCCCTC ATTTTNCCTA GTTACTCGGA AGAGCGCTGG ACTTGAAATC 120
AAAATAATTG CGTTATGTCT TTGGTTATGT CGCTTCATCA TAGCACTTTC TAAAACTATT 180
TGACAAACAT GTATTGCATA CCTACNGCAT TCCAGTTCTN GTACANGTAA TTAAATGCTC 240
GACTAACGNN AAA 253

SEQ ID NO:205
SEQUENCE LENGTH:253
SEQUENCE TYPE:nucleic acid
TOPOLOGY:linear
CLONE:HUMGS00221
SEQUENCE DESCRIPTION:
GATCTGGAAT TGGACATTTC TCTGTCAGAG CACAGAGGAG GCTCATATCA CCTCTTCCCT 60
CTCCTACTTG GCCCAGCTGC TTGGAGGACC GACCCCATGG CTGAGAATAT NACGGCAAGA 120
GGAACAGAGN TTGCTCCAAG TGGGAAAGGG TCCCAAGCAG TCCAGAGAAG ATGTCTGTGT 180
GGCTTTCCCT CCCTGCCTCC CCCAGCTCCC ACACTGGCCT TTGTAAATAA ATGGCGTGGT 240
CTTTGTTGTG AAA 253

SEQ ID NO:206
SEQUENCE LENGTH:253
SEQUENCE TYPE:nucleic acid
TOPOLOGY:linear
CLONE:HUMGS00222
SEQUENCE DESCRIPTION:
GATCTGCCGT CTGTCACCTC TCTCCAAGTT GAGACAGGGG CCTGGATTTC AGCCCTTCCT 60
GCCGAGAAAT CTTNTAAATT TCAACCTACC TTTAAAAATA AAGTCTCCCT ACTTAAAATC 120
CAGTAGATAT CATGGCACTA ATGCTAACAC CCCTTTCCCC AAATTAATAA AACAAAAATA 180
AAAAAAAGAA AAAANTGCAG GCATGCAAGC TTGGCGTANT CATGGNNTNA GCTGTTTCCC 240
NGTCACGGCG TGN 253

SEQ ID NO:207
SEQUENCE LENGTH:251
SEQUENCE TYPE:nucleic acid

TOPOLOGY:linear
CLONE:HUMGS00223
SEQUENCE DESCRIPTION:
GATCGAGACA CGTGATGGGA AGCTGGTGTC TGAGTCCATN ACGTCCTNCC AAGTGAACAG 60
CTGCGGCAGC CCTCCCAGCC TACCCCTCCT GCGCTGACCC AGAGCCTGGG AAGGAGGCCG 120
CTATGAGGGT AGCACTGGGA ACAGGAGACC CACCTNAGNC TCAGCCTNGC CTCAGTNTAC 180
TGGGGAGTTT ACTACCTGGG GCCCANTTGA CATGCTCCAG CTACANACAN TTANTTGCTT 240
TTTTNTTGGN N 251

SEQ ID NO:208
SEQUENCE LENGTH:243
SEQUENCE TYPE:nucleic acid
TOPOLOGY:linear
CLONE:HUMGS00224
SEQUENCE DESCRIPTION:
GATCGATTCT TGTATATTNA TTTTATCTCT TTCTGTATCT ATAGGTAAAT CTCAAGGGTA 60
AAATGTTAGG TGTTGACATT GAGAACCCTG AAACCCCATT CCCTGCTCAG AGGAACAGTG 120
TGAAAAAAAA TCTCTTGAGA GATTTAGAAT ATCTTTTCTT TTGCTCATCT TAGACCACAG 180
ACTGACTTTG AAATTATGTT AAGTGAAATA TCAATGAAAA TAAAGTTTAC TATAAATAAT 240
AAA 243

SEQ ID NO:209
SEQUENCE LENGTH:249
SEQUENCE TYPE:nucleic acid
TOPOLOGY:linear
CLONE:HUMGS00225
SEQUENCE DESCRIPTION:
GATCACANTT AATGATTCAG TATAATGTTT GAGGCCAGAC AAGATATATA TTGTGCCTCT 60
TACAGCCTTT GGNNCNNTTG TTTCCATTTT TTAAATATCT TCTATATCCA TATAGTATTC 120
AAATNATTAA TGCTCATGTA CCAAGGTTTT GCTATAAAAG TTTTGNCTGT ATGAATAATG 180
TGGCTTTAGT AAATAATCAT TTTTCAACTG TAAACTTATT CTGAAATAAA GTAAAATTCT 240
AATTGTTTN 249

SEQ ID NO:210
SEQUENCE LENGTH:248
SEQUENCE TYPE:nucleic acid
TOPOLOGY:linear
CLONE:HUMGS00226
SEQUENCE DESCRIPTION:
GATCATAGAA ATATATGTAA AGTATTCAAT TTTCAATCAT TTTCAAATNA CTGTTATAAA 60
TTGTTTTTGC TGAGTTGTAA TACTTTTNAG ATACAATGTA TTCCTTGTAC TGAAAGAATG 120
AAAAAGGACT TTTTCAGCAT TTGAGGTAAG TNCTTTAACG TTTCATTAAA ANCATTTTTT 180
ACAAATATTT TGTACATGCA CTTGCAGTAT TGAGGTTAAT CATTTTAATA AATNCGGAAA 240
TTAAAACN 248

SEQ ID NO:211
SEQUENCE LENGTH:247
SEQUENCE TYPE:nucleic acid
TOPOLOGY:linear
CLONE:HUMGS00227
SEQUENCE DESCRIPTION:
GATCTCAGCN TTGCAAACAC ANTTNCTACA TAGATAGTAC TAGGTATNNT TTAGATATGT 60
AAAGAAAGAA ATCACACCAT TAATAATGGT AAGATTGGTT TATGTGATTT TAGTGGTATT 120
TTTGGCACCC TTATATATGT TTTCCAAACT TTCAGCAGTG ATATTATTTC CATAACTTAA 180
AAAGTGAGTT TGAAAAAGAA AATCTCCAGC AAGCATCTCA TTTAAATAAA GGTTTGTCAT 240
CTTTAAA 247

SEQ ID NO:212
SEQUENCE LENGTH:256
SEQUENCE TYPE:nucleic acid
TOPOLOGY:linear
CLONE:HUMGS00228
SEQUENCE DESCRIPTION:
GATCAAAACA AACAATCCAG ATGTATAAGT ACTAGGCAGA AGCCAATTTT AAAATTTCCT 60
TGAATAATCC ATGAAAGGAA TAATTCAAAT ACAGATAAAC AGAGTTGGCA GTATATTATA 120
GTGATAATTT TGTATTTTCA CAAAAAAAAA NGTTAAACTC TTCTTTTCTT TTTATTATAA 180
TGNCCAGCTT TNGGTATTTC ATTGTTACCA NGTTCTATTT TTNGANTAAA ATTGTTCTCC 240
TTCTAAANGT TTTAAA 256

SEQ ID NO:213
SEQUENCE LENGTH:244
SEQUENCE TYPE:nucleic acid
TOPOLOGY:linear
CLONE:HUMGS00230
SEQUENCE DESCRIPTION:
GATCGGCAAG CCCCACACTG TCCCTTGCAA GCCAAGGCCA CCTTTNATGC CATTTCTAAG 60
ACCTACAGCT ACCTGACCCC CGACCTCTGG AAGGAGACTG TATTCACCAA GTNTCCCTAT 120
CAGGAGTTCA CTGACCACCT NGTCAAGACC CACACCAGAG TCTCCGTGCA GCGGACTCAG 180
GCTCCAGCTG TGGCTACAAC ATAGGGTTTT TATACAAGAA AAATAAAGTG AATTAAGCGT 240
GAAA 244

SEQ ID NO:214
SEQUENCE LENGTH:243
SEQUENCE TYPE:nucleic acid
TOPOLOGY:linear
CLONE:HUMGS00231
SEQUENCE DESCRIPTION:
GATCAGATTG GGTCCTGCTC CTCTCAACCT TGAAGTCCCC ACGTATGAGT TCACCAGTGA 60
CGATATGGTG ATTGTTGGTT AAGAGACTTG GACTCAAGTC ATAGGCTTCT TTCAGTCTTT 120
ATGTCACCTC AGGAGACTTA TTTGAGAGGA AGCCTTCTGT ACTTGAAGTT GATTTGAAAT 180

ATGTAAGANT TGATGATGTA TTGGCAAACA TTAATGTGAA GTAAATNGAA TTNAATGTNG 240
AAA 243

SEQ ID NO:215
SEQUENCE LENGTH:243
SEQUENCE TYPE:nucleic acid
TOPOLOGY:linear
CLONE:HUMGS00232
SEQUENCE DESCRIPTION:
GATCGCCAGT GGAAGAAGAT TAGTGCAATC ATTGAGAAGA GGAAGAAGAT GGAAGCTGAT 60
GGGGTTGAAG TCAAAAGACC AAAATACTAA TCACTAGTTA CAACCAGAGA TGCTCCACAA 120
GGATATGCTC CCCACGGTTT TCTTTCTACA ATTTCCAAAG GTTGCAAGAT GTTTTTTTGT 180
GGATGAATAT AAAATTTTAT TGTGTAATTA CTTGGTTCCA TTAAAATTGG TTAACTTGCT 240
AAA 243

SEQ ID NO:216
SEQUENCE LENGTH:243
SEQUENCE TYPE:nucleic acid
TOPOLOGY:linear
CLONE:HUMGS00233
SEQUENCE DESCRIPTION:
GATCGTAGAT GTTAACCAAA TCTACAAAAT CCACTACTCC CCCGTTATCA ACGGGATACG 60
TTCCAAGACC CCCAGTGGAT GCCTGAAACT GGCTAATGCT GAACCCTACA TATACTATGT 120
TTTTNCTGTA CATATATATG ATAAAGTTTA AATNATAAAT NAGGTACAGT AACAACAATA 180
ACAGTAAAAC AACAGTTATA ACAATATACT GTAATAAAAG TCATGTGAAT GTGGTGTTTC 240
TCN 243

SEQ ID NO:217
SEQUENCE LENGTH:242
SEQUENCE TYPE:nucleic acid
TOPOLOGY:linear
CLONE:HUMGS00234
SEQUENCE DESCRIPTION:
GATCTTCTCC AGATTGGCAG AAAGTTGATA TAGGTGGACT TTTTTACAGG TCAGTTGAGG 60
CAAAAAACTA TGGGTTTTTT CAGGTGAACC TCCCCCATTT AAATACTCAG AAGATAAGGT 120
GTGAATGTAT GTATTATTAG AGTCCTAAAG TATTTTATAA GTACTGGTTT CACCACGCTT 180
TGTGGGAGAG AAATCATTCA AATCATTTTT TTTGTCCGTA CAATAAAGTT TACTAAAAAC 240
CN 242

SEQ ID NO:218
SEQUENCE LENGTH:238
SEQUENCE TYPE:nucleic acid
TOPOLOGY:linear
CLONE:HUMGS00235
SEQUENCE DESCRIPTION:

GATCTTCGTG ATACTGTACA TAGCTGTTTG AAATGCCAGA ATGACTTCTG ACATNCCAAG 60
TTTTTCACAA AATATATTTN ATCTGTGATT AGCCATTTGA CTAATAATAC TGGCTAACAG 120
ATGTTGAAAA AAATTGTCTG TTTTCTCATT AATTTTGGTC TAAAACATGT TTGCACTTGT 180
NTTTGACTTG TGTTTTATTA ACATTGATTG GCATATTAAA AGTCACTCNG AGCTTAAA 238

SEQ ID NO:219
SEQUENCE LENGTH:239
SEQUENCE TYPE:nucleic acid
TOPOLOGY:linear
CLONE:HUMGS00236
SEQUENCE DESCRIPTION:
GATCCAAANN ATGACTTCAG AAAAAACTTG AAAGTAACAG CAGTGCCTAC ACTACTTAAG 60
TATGGAACAC CTCAAAAACT GGTAGAATCT GAGTGTCTTC AGGCCAACCT GGTGGAAATN 120
TTGTTCTCTG AAGATTAAGA TNGGTAGGAT GGCAATCATG TCTTGATGTC CTGATTTGTT 180
CTAGTATCAA TAAACTGTAT ACTTGCTTTG AATTCATGTT AGCAATAAAT GATGTTAAA 239

SEQ ID NO:220
SEQUENCE LENGTH:250
SEQUENCE TYPE:nucleic acid
TOPOLOGY:linear
CLONE:HUMGS00237
SEQUENCE DESCRIPTION:
GATCAGAGGT GAAGGGACAG AGAGAGGAGA GGAGGAAGAT TGAGCTGGGG GCAACAGCCA 60
AGCTCACCTG GGCAGGTCTC TGCCACCTCC TTGCTCTGTG AGCTGTCAGT CTAGGTTATT 120
CTCTTTTTTT GTGGCTATTT TTAATTGCTT TGGATTTGTT AAATGTTTTC TGTCTTCTGT 180
TAAGTGTGTT TTCTCTGGAG ATAGAATGTA AACCATATTA AAAGGAAAAA GTTTCAGACA 240
AGCAATTAAA 250

SEQ ID NO:221
SEQUENCE LENGTH:237
SEQUENCE TYPE:nucleic acid
TOPOLOGY:linear
CLONE:HUMGS00238
SEQUENCE DESCRIPTION:
GATCAGCTTT GCTCCTGAAA TTGCATCCGA AGAAGAAAGA AAGGGGATGG TGGCTGCGTG 60
GTCCCAGAGG CTGCAGACCA TCTGGAAGGA AGAGCCCATC CCCTGCACAG CCCACTGGCA 120
CTTCGGGCAA TAACTCTGTG GCACGTGGGC ATCACGTAAG CAGCACACTA CGAGGCCCAG 180
GCGCAGGCAA AGAGAAGATG GTGCTGTCAT GAAATAAAAT TACAACATAG CTACAAA 237

SEQ ID NO:222
SEQUENCE LENGTH:243
SEQUENCE TYPE:nucleic acid
TOPOLOGY:linear
CLONE:HUMGS00239
SEQUENCE DESCRIPTION:

GATCAGTGTA GAACTGGTCA TAGAGGAAGA GCTAGAAATC CAGTAGCATG ATTTTTAAAT 60
AACCTGTCCT TGTTTTTGAT GTTAAACAGT AAATGCCAGT AGTGACCAAG AACACAGTGA 120
TTATATACAC TATACTGGAG GGATTTCATT TTTAATTCAT CTTTATGANG ATTTAGAACT 180
CATTCCTTGT GTTTAAAGGG AATGTTTAAT TGAGAAATAA ACATTTGTGT ACAAAATGCT 240
AAA 243

SEQ ID NO:223
SEQUENCE LENGTH:232
SEQUENCE TYPE:nucleic acid
TOPOLOGY:linear
CLONE:HUMGS00240
SEQUENCE DESCRIPTION:
GATCTAATAA CAGGTTGACA TAAGAAATAT TTGTCTCAAA AATCAATGTA TTTAATAGTT 60
GACAGTTACA AATCTCATCA TTGAAAGATT TAATTTTAGT TACCTTTTGT TGATTTANNN 120
NNNATTGCAT TTGTATATTG CTAACTGATA AGACAAATTG AGTTATTGAG CTATTAANTG 180
CACATTTTAA TATAANTGCA GAAATCCCAA ATAAAATGCT AACATACTGA AA 232

SEQ ID NO:224
SEQUENCE LENGTH:237
SEQUENCE TYPE:nucleic acid
TOPOLOGY:linear
CLONE:HUMGS00241
SEQUENCE DESCRIPTION:
GATCACCGTG ACATCCGAGG TGCCTTTCTC CAAAAGGTAT TTGAAATATC TCACCAAAAA 60
ATATTTGAAG AAGAATAATC TACGTGACTG GTTGCGCGTA GTTGCTAACA GCAAAGAGAG 120
TTACGAATTA CGTTACTTCC AGATTAACCA GGACGAAGAA GAGGAGGAAG ACGAGGATTA 180
AATTTCATTT ATCTGGAAAA TTTTGTATGA GTTCTTGAAT AAAACTTGGG AACCAAA 237

SEQ ID NO:225
SEQUENCE LENGTH:236
SEQUENCE TYPE:nucleic acid
TOPOLOGY:linear
CLONE:HUMGS00242
SEQUENCE DESCRIPTION:
GATCTTCACT TAANCTAAGT CTGTGAATTA CTTTTATATT ATTTTGAAAN ACTCCTTGCA 60
GTATATTGGC ATGATACAGT AAAAGCANTT TCCACAGATT GTTATCACCT TCTTTAAAAG 120
AAGTCAAAAT TTAAAAAATA CAATAGCACG TTGTTGGTGT CATATTCAAT AACATTTCCA 180
ATGCTACATA TAATTTTATA GACATAATAA AGAAGGTATT GAAAAAACTA AATAAA 236

SEQ ID NO:226
SEQUENCE LENGTH:227
SEQUENCE TYPE:nucleic acid
TOPOLOGY:linear
CLONE:HUMGS00243
SEQUENCE DESCRIPTION:

GATCGCCACC TACCGCCGCC TGCTGGAAGA TGGCGAGGAC TTTAATCTTG GTGATGCCTT 60
GGACAGCAGC AACTCCATGC AAACCATCCA AAAGACCACC ACCCGCCGGA TAGTGGATGG 120
CAAAGTGGTG TCTGAGACCA ATGACACCAA AGTTCTGAGG CATTAAGCCA GCAGAAGCAG 180
GGTACCCTTT GGGGAGCAGG AGGCCAATAA AAAGTTCAGA GTTCAAA 227

SEQ ID NO:227
SEQUENCE LENGTH:205
SEQUENCE TYPE:nucleic acid
TOPOLOGY:linear
CLONE:HUMGS00244
SEQUENCE DESCRIPTION:
GATCAGCAAG CAGGAGTATG ACGAGTCCGG CCCCTCCATC GTCCACCGCA AATNCTTCTA 60
GGCGGACTAT GACTTAGTTG CGTTACACCC TTTCTTGACA AAACCTAACT TGCGCAGAAA 120
ACAAGATGAG ATTGGCATGG CTTTATTTGT TTTTTTTGTT TTGTTTTGGT TTTTTTTTTT 180
TTTTTGGGTT NNCCCCNGGT TTAAA 205

SEQ ID NO:228
SEQUENCE LENGTH:226
SEQUENCE TYPE:nucleic acid
TOPOLOGY:linear
CLONE:HUMGS00245
SEQUENCE DESCRIPTION:
GATCTGCCCA AAGAGGTGTT GGCTGAGCTT NAGGCCCTGG AGAGACGTGT GCACAAAATG 60
TNACCTGAGG CCCTAGTCTA GCAAGAGGAC ATAGCACCCT CATCTGGGAA TAGGGAAGGC 120
ACCTTGCAGA AAATATGAGC AATTTGATAT TAACTAACAT CTTCAATGTG CCATAGACCT 180
TCCCACAAAG ACTGTCCAAT AATAAGAGAT GCTTATCTAT TTTAAA 226

SEQ ID NO:229
SEQUENCE LENGTH:228
SEQUENCE TYPE:nucleic acid
TOPOLOGY:linear
CLONE:HUMGS00246
SEQUENCE DESCRIPTION:
GATCAGCCCC AGATTTGACG TGCAACTCAA AGACCTGGAA AAATGGCAGA ATAATCTGCT 60
TCCATCCCGC CAGTTTGGTT TCATTGTACT GACAACCTCA GCTGGCATCA TGGACCATGA 120
AGAAGCAAGA CGAAAACACA CAGGAGGGAA AATCCTGGGA TTCTTTTTCT AGGGATGTAA 180
TACATATATT TNCAAATAAA ATGCCTCATG GACTCTGGTG CTTCCAAA 228

SEQ ID NO:230
SEQUENCE LENGTH:308
SEQUENCE TYPE:nucleic acid
TOPOLOGY:linear
CLONE:HUMGS00247
SEQUENCE DESCRIPTION:
GATCTGCGAC CATTTCTGTA CAACACAAGC TGGCCTTGGC AGTTTCGGTG CATAGAAAAT 60

CAGGTGCTAC AGCTCGAGAG GGCAGAGCCA CAGTCCCTGG ACGGCGTGGA CTGAGGCCGG 120
TTCCTTCCTG GAGGCCTCCT GTCCTCGGGG ACCCCAGCAC CTCATCATCA GCATTGCTGG 180
AGCCAAGGGT AGGAGCCCTA CACTAGGAGC CCAGGATGGG ACGGNGNATN AGCCGAGAGG 240
NAGGGAACTT TTNAGTNAAA TTCCTCAAAA AGAGGNTTAG AATAAANCCT TNGGCTTAAA 300
AAGAGAAA 308

SEQ ID NO:231
SEQUENCE LENGTH:230
SEQUENCE TYPE:nucleic acid
TOPOLOGY:linear
CLONE:HUMGS00248
SEQUENCE DESCRIPTION:
GATCCAAAGA CAGACCACAG ACTGGGAAAA GTTGGAAGAT GAACACTTGA GGACTTCAGC 60
TTCTCACCTA CTTAGTACAG TTGGGAACCA TACACTTCTG GCATGTTTGG AAATCAAAAT 120
GTCACATTCT CGGGGGAGGA AGCCCAGAAA ATTGGGTATG TTCTAGAGAT TTACCACCAT 180
TGCTTATTGC TTTTNCTCTT TAATAAAGTT TAGGAAAGTA GAATTTTAAA 230

SEQ ID NO:232
SEQUENCE LENGTH:228
SEQUENCE TYPE:nucleic acid
TOPOLOGY:linear
CLONE:HUMGS00249
SEQUENCE DESCRIPTION:
GATCTAAAGA TTTCTCTATC CAATGAATCT AACAAAGTCA ATGGAAATTG AACTCTAGAA 60
TTGTCTCTAG AAAACATAGC TTCTTACTGA ACTTGAACAT TTTTACAACA TTCACTGGTT 120
TTTGTTTTGT TAGCTAATAA TCTATAATAG TTGAGTATCT CTGGGAATGG GGAGGGAAAT 180
TATATGTAAT AGAGCTTAAA AATAAAGTGT CAATTTCCAA GGNCTAAA 228

SEQ ID NO:233
SEQUENCE LENGTH:221
SEQUENCE TYPE:nucleic acid
TOPOLOGY:linear
CLONE:HUMGS00250
SEQUENCE DESCRIPTION:
GATCAATAAG CATGTCAGAC TGATTAATGT CTACTGTGAA AATTTGGTAG TAAATTTNCA 60
TTTGATATTA GATATAAATA TCTGAATATA AATAATTTNA ATATACTAGT CATGATGTGT 120
GTTGTATTTN AAAAATTATC TGCAACCTTA ATTCAGCTGA AGTNCTTTAT ATTTCAAAAG 180
AATGAATAAC ATTGATAATA AAATCGCTAC TTTAAGGGAA A 221

SEQ ID NO:234
SEQUENCE LENGTH:219
SEQUENCE TYPE:nucleic acid
TOPOLOGY:linear
CLONE:HUMGS00251
SEQUENCE DESCRIPTION:

GATCTCTATG AATGTCAGAG CCCTAACTTT CAGGCTTTGC ATTTTGTATA TGGGAAGAAA 60
TATGACAATC CTAGGTAATT AAACCATAGA CCCAAAGCCC TTACGTTTGA TGCAATTTAT 120
TTTTAAAATA GGCCTTGTTT TTCAGCTTCA TCTGCAGTTC TATGTGAAGA TTGATAAATC 180
AGTTTTTACT TGTTTTATTA ATAAAACGTA ATTNGGAAA 219

SEQ ID NO:235
SEQUENCE LENGTH:287
SEQUENCE TYPE:nucleic acid
TOPOLOGY:linear
CLONE:HUMGS00252
SEQUENCE DESCRIPTION:
GATCTCCGTT CCGCTCCCAG CGGCTCCAGT GTAAATTCCC CTTCCCCCTG GGGAAATGCA 60
CTACCTTGTT TTGGGGGGTT TAGGGGTGTT TTTGTTTTTC AGTTGTTTTG TTTTTTTGTT 120
TTTTTTTTTN CCTTTGCCTT TTTNCCCTTT NATTTGGNGG GAATGGGAGG AAGTGGGANC 180
AGGGAGGTGG GAGGTGGATT TTGTTNATTT TTTAAGCTCA TTTCCAGGGG TGGGANTTTT 240
TTTTNAANAT GNGNCATGAA NAAAGTTTGT TTTTGAAANT AACCAAA 287

SEQ ID NO:236
SEQUENCE LENGTH:222
SEQUENCE TYPE:nucleic acid
TOPOLOGY:linear
CLONE:HUMGS00253
SEQUENCE DESCRIPTION:
GATCAGAATT GGCAGCACAA AGAAAACGCC CTCTCCTGAC TTGTATTGTG GCAGTCTGAA 60
CGNCCCCAGA AAATTGTGCC AAAGAGTTTA GAAAAATAAA TATACAATAA AAGTAAACAC 120
ATACACACAA AACAGCAAAC TTCAGGTAAC TATTTTGGAT TGCAAACAGG ATAAATTAAA 180
TGTTCAAACA ATCTGATAAA ATAACCATTT GGGNCTTGNA AA 222

SEQ ID NO:237
SEQUENCE LENGTH:221
SEQUENCE TYPE:nucleic acid
TOPOLOGY:linear
CLONE:HUMGS00254
SEQUENCE DESCRIPTION:
GATCATCTTT CCTNTTCCAG AGAAGTGGGC TGGATGTCTC CATCTCTGTC TCAACTTTAC 60
GTGTACTGAG CTGCAACNTC TTACTTCCCT ACTGAAAATA AGAATCTGAA TATAAATTTG 120
TTTTCTCAAA TATTTGCTAT GAGAGGTTGA TGGATTAATT AAATAAGTCA ATTCCTGGAA 180
GTTGAGAGAG CAAATAANGT CCTGAGAACC TTCCAGACAA A 221

SEQ ID NO:238
SEQUENCE LENGTH:217
SEQUENCE TYPE:nucleic acid
TOPOLOGY:linear
CLONE:HUMGS00255
SEQUENCE DESCRIPTION:

GATCAGACGT TTTACAAATN CATGAAGCGA ATTGCTGCTT GTAAGGAGCA GATTTTAAGG 60
TATTCCTGGA GTGGAGAGCC ACTCTTTTTA ACCTGCCCTA CATCAGANGT CACCGAGCTC 120
CCAGCCTGCA GCCAGTNTGG AGGCCAAAGG ATATTNNATT TTCANCTTAT GCCAGCNCTG 180
GTCAGCATGC TCAANAGTGC TANTTTAGGT CTTNCTN 217

SEQ ID NO:239
SEQUENCE LENGTH:217
SEQUENCE TYPE:nucleic acid
TOPOLOGY:linear
CLONE:HUMGS00256
SEQUENCE DESCRIPTION:
GATCTCTCTG AGTCCTGGCA ACATCCAGCA AAACTACTGC TTATTCTCCA AAGAATATTG 60
GGAGCTCTCA ATCCTCGGTG ATATGGGAAA GAGAACTGAG TATTTGCCCT ATGACTGAGC 120
TTTCTATAGG AATTTTATTA AAGAATGTTT AATTTNGTTG TCCTNCTNAA TGTTCTCAGT 180
CAAATAAATG AGTGAGCTGG TTTCGGCTGC TCTTGGN 217

SEQ ID NO:240
SEQUENCE LENGTH:216
SEQUENCE TYPE:nucleic acid
TOPOLOGY:linear
CLONE:HUMGS00257
SEQUENCE DESCRIPTION:
GATCCCATGG ACATTTGGGG AAAGGGCTCC TTGGGCTGCT GGTGAACTTC TGTGGCCACC 60
ACCTCCTGCT CCTGACCTCC CTGGGAGGTG CTATCAGTCT GTCCTGGCCT TTCAGTTTTA 120
TAAGTNGNTT CCAGCCCCAG TGTCCTGACT TCTNCTGCAN AATAGGAGGG AGGCCTCCTT 180
TTNGGANGGN NGTACTTTGG GNGATAGTGN GGCCTN 216

SEQ ID NO:241
SEQUENCE LENGTH:220
SEQUENCE TYPE:nucleic acid
TOPOLOGY:linear
CLONE:HUMGS00258
SEQUENCE DESCRIPTION:
GATCTTTTAA GTTTCCTTCC CTACCCAGTC CCCATTTTCT GGTAAGGTTT CTAGGAGGTC 60
TGTTAGGTGT ACATCCTGCA GCTTATTGGC TTAAAATGTA CTCTCCTTTT ATNTGGTCTC 120
TTTGGGGCCG ATTNGGNGAA AGCGAAATCA NTAGTGCAAC TGTTTTGATA CTGAATATTG 180
ACAAGTGTCT NTTTNAAATA AAGACCCANT CCCTTCCAAA 220

SEQ ID NO:242
SEQUENCE LENGTH:223
SEQUENCE TYPE:nucleic acid
TOPOLOGY:linear
CLONE:HUMGS00259
SEQUENCE DESCRIPTION:
GATCACTAAT CAATAATCTG ATATTTAACA AAATATGGAC AGGCCACTTA TGCTCAGTTT 60

TACCTTAGTT ATTCCTTGGT ATCCACAGGC CCAAGTCCCT TTAAATAAAA TACCCTCATA 120
TTTCCATATA ATCTACATAC ATTCTCCCAT ATACTTTAAA TCATCTCTAG ATTACTNATA 180
ATGTCTAATN CAAAATAAAT GCTATGTAAA TGTAATTATT AAA 223

SEQ ID NO:243
SEQUENCE LENGTH:216
SEQUENCE TYPE:nucleic acid
TOPOLOGY:linear
CLONE:HUMGS00260
SEQUENCE DESCRIPTION:
GATCAACTTA ATTCCTTTTC TTTATCTTCC NTCCCTCACT TCCCTTTTCT CCCACCCTCT 60
TTTCCAAGCT GTTTCGCTTT GCAATATATT ACTGNNTAAT NAGTTGCAGG ATAATGCAGT 120
CATAACTTGT TTTCTCCNAA GTATTTGAGT TCAAAACGCC NGTATCTAAA GAAATACGGT 180
TGGGGTCATT AATAAAGAAA ATCTTTCTAT CTTAAA 216

SEQ ID NO:244
SEQUENCE LENGTH:212
SEQUENCE TYPE:nucleic acid
TOPOLOGY:linear
CLONE:HUMGS00261
SEQUENCE DESCRIPTION:
GATCTCAGAA CAATCAGATG CAAAGCTGAA AGAGATTGTA ACAAATTTCT NTGGCTGGAT 60
TTGAAGCTTA AACTCCTGTG GATTCACATC ANATACCAGT TCAGTTTTGT CATTGTTCTA 120
GTAAATTAGT TCCATTTGTA AAAGGGTTAC TCTCATACTC CTTATGTACA GAAATCACAT 180
GAAAAATAAA GGTTCCATAA TGCATAGTTA AA 212

SEQ ID NO:245
SEQUENCE LENGTH:221
SEQUENCE TYPE:nucleic acid
TOPOLOGY:linear
CLONE:HUMGS00262
SEQUENCE DESCRIPTION:
GATCCATCTG CCTTTGTGGC TGCTGCCCCT GTGGCTGCTG CCACCAACAG CTGCTCCTGC 60
TGCTGCTGCA GCCCAGCTAA GGTTGAAGCC AAGAAAGAGT CGGAGGAGTC GGACGAGGAT 120
ATGGGATTTG GTCTCTTTGA CTAATCACCA AAAAGCAACC AACTTAGCCA GTTTTATTTG 180
CAAAACAAGG AAATAAAGGC TTACTTCTTT AAAAAGTCAA A 221

SEQ ID NO:246
SEQUENCE LENGTH:214
SEQUENCE TYPE:nucleic acid
TOPOLOGY:linear
CLONE:HUMGS00263
SEQUENCE DESCRIPTION:
GATCCCATTG GAAGGAATGC TCTACCTCAC AGAACTCTGA ACCCTACAGA AATATGGGCC 60
TGCTGCCATT TCCTGAAGAC CGGGGCATCG GGGTGGGGTG ATAAAGGATA CAACCTGCAC 120

AGGGGGAAGT TATTAAAGAG GCTGCAAAGT CCAGCCACCC TGAAGATACT CCCCAGTGCT 180
CCCCTCCTGC TAAAGAACCA GTTACCCCAG GAAA 214

SEQ ID NO:247
SEQUENCE LENGTH:208
SEQUENCE TYPE:nucleic acid
TOPOLOGY:linear
CLONE:HUMGS00264
SEQUENCE DESCRIPTION:
GATCCTCAAC TATTGGAAAT NATGGACTGG TGGCCCTGGT ACAGAACCAT GACTGGCTGC 60
TGAATTCTGA AAACCAGGAC TTGGTTCAAC ATTTAAATTT GATAGTTGCC CTGATTCCCA 120
TTTTGGGTTT GTGAAAAGTG TATGTATTTA AATTTGCTGT AAAACATAAT CACTAATAAT 180
ATGCAATAAA TATTTCCTTG AAGGGAAA 208

SEQ ID NO:248
SEQUENCE LENGTH:208
SEQUENCE TYPE:nucleic acid
TOPOLOGY:linear
CLONE:HUMGS00265
SEQUENCE DESCRIPTION:
GATCTGTGGG AGGAATGGCA AGAGAAGCAA CCGGACCCTG AGAGAAGAGT GTTAAGGAAC 60
CTGCGCATGT GGTTAGCTTG ACCTTTCTGT TGGGCATGAC ATGGGGTTTT GCATTCTTTG 120
CCTGGGGACC NTTAAATATC CCCTTCATGT ACCTCTTCTC CATCTTCCAN TTCATTACAA 180
GGTAAGATAA ATTGTACATG AATAGTCN 208

SEQ ID NO:249
SEQUENCE LENGTH:201
SEQUENCE TYPE:nucleic acid
TOPOLOGY:linear
CLONE:HUMGS00266
SEQUENCE DESCRIPTION:
GATCAAATAT CACTAAATAC TTTAAATTGT TTTACTTAAG AGTCTAATCT GGGAAGTTTT 60
CAAATCATAC TATTAATGTG TAATCTAAGC TCTTCAGATG TATCCATGAA TAATCCTGGA 120
ACAATATTGC TTGTATTCCT GTCATAGAAC AGGTTTTGTA ATCTTTAAAA GAAATGAAAA 180
TTTATATAAT AAAGTTTCAA A 201

SEQ ID NO:250
SEQUENCE LENGTH:211
SEQUENCE TYPE:nucleic acid
TOPOLOGY:linear
CLONE:HUMGS00267
SEQUENCE DESCRIPTION:
GATCACCTTG GTGTTCCTTG TTTGGAAGAT TATTTCCTCT GAATTTCTGG GCTTGGTCTT 60
CCAGTTGGCA TTTGCCTGAA GTTGTATTGA AACAATTTAN TGAAAATATT AAATATTTGG 120
TTTCAAAAGG CAGATTTATC TTCTCCCAAC ATTCTGTTAT TTCTGATACT TTTGAAAAAC 180

TAATAAAAAT TAATAAAAGA CATGAACTAA A 211

SEQ ID NO:251
SEQUENCE LENGTH:208
SEQUENCE TYPE:nucleic acid
TOPOLOGY:linear
CLONE:HUMGS00268
SEQUENCE DESCRIPTION:
GATCCAAGAA AACGCCTCAC TGCCTTAACC TTAACTGTTC TTCCTGGCGC TAAAAAGAGC 60
TGTATTTTTT AAAGTGCTGG GGCAAACAAA GNANCCCCAA AAGAGTTGAT GTGTGTTTTA 120
AAAGNAAAAA CCCAATGAGG AACAATTGGA GATTTTTATG CAGAAACTAA ATAATCCTTA 180
ATAAATAAAT CTCTATTTTG GAATCAAA 208

SEQ ID NO:252
SEQUENCE LENGTH:205
SEQUENCE TYPE:nucleic acid
TOPOLOGY:linear
CLONE:HUMGS00269
SEQUENCE DESCRIPTION:
GATCTTCTAA AAAAGGAACA GAAAATGGTG TGAATGGAAC ATTAACTNCA AATGTAGCAG 60
ACTCTCCCCG GAATAAAAAA GAGAAATCTT CATAATGAAT TATAANCTAA TTGATTAATG 120
TCCCCAAAGA AATCTGCTTT CTACTATATC TTTCAGCATT AGAGATTTTC CTGTTCTNGA 180
AAATNCAGTC TGTGCTCTTT GATTN 205

SEQ ID NO:253
SEQUENCE LENGTH:212
SEQUENCE TYPE:nucleic acid
TOPOLOGY:linear
CLONE:HUMGS00270
SEQUENCE DESCRIPTION:
GATCTAGGCA AAGAAGAATA CAAATNAAAC CCCNTTCTTT CTCGTTTCCN GTCCAACAAC 60
TCTGTAGAGC TCTCTGCACC CGTTACCCCT TTCCACCTTT TGTATTTAAT TTTAAAGTCA 120
NGTGTACNTG CAAGGAATGC TGGATGCAAG ATAGATACTA TATTAAACTG TACTGTTATT 180
TAAGATGTAA TAAAGCAGTT TGACATGAGA AA 212

SEQ ID NO:254
SEQUENCE LENGTH:209
SEQUENCE TYPE:nucleic acid
TOPOLOGY:linear
CLONE:HUMGS00271
SEQUENCE DESCRIPTION:
GATCTAAGAT GATTATTTTG TAAAAGACTT TCTAGTGTAC AAGACACCAT TGTGTCCAAC 60
TGTATATAGC TGCCAATTAG TTTTCTTTGT TTTTACTTTG TCCTTTGCTA TCTGTGTTAT 120
GACTCAATGT GGATTTGTTT ATACACATTT TATTTGTATC ATTTCATGTT AAACCTCAAA 180
TAAATGCTTC CTTATGTGAA AAAAACAAA 209

SEQ ID NO:255
SEQUENCE LENGTH:197
SEQUENCE TYPE:nucleic acid
TOPOLOGY:linear
CLONE:HUMGS00272
SEQUENCE DESCRIPTION:
GATCTGCCAG CAGTGTTCTT GCAATATGAG GAAGACAGTT ACAGCCACAT TATGGCTCTC 60
ATTGAACAGT ACGCAGCACC CCTGCCCCCA GCCGTCTTTC TGGGGCTTGC GCGCAAAATC 120
TACAAGCGGA GAAAGTGACC TAGAGATTGC AAGGGCGGGG AGAGGAGGCT CTCAATAAAT 180
AATCGTGTAA CCTTAAA 197

SEQ ID NO:256
SEQUENCE LENGTH:196
SEQUENCE TYPE:nucleic acid
TOPOLOGY:linear
CLONE:HUMGS00273
SEQUENCE DESCRIPTION:
GATCTATCAC CTGTAATCAT AACTGGCTTC TGCTTGTAAT CCACACAACA CCAGGACTTA 60
AGACAAATGG GACTNATGTC ATCTTGAGCT CTTCATTTAT NTTAACTGTA ATTTATTTGG 120
AGTGGAGGCA TTGTTTTNAA GAAAAACATG TCATGTAGGT TGTCTAAAAA TAAAATGCAT 180
TTAAACTCAT TTGAAA 196

SEQ ID NO:257
SEQUENCE LENGTH:196
SEQUENCE TYPE:nucleic acid
TOPOLOGY:linear
CLONE:HUMGS00274
SEQUENCE DESCRIPTION:
GATCTCACCA GGAGAAATGA ATATGTGAGG TGATGGATGT AACTAGCTTG ATTGTGGTAA 60
TCAATTTCGC AATGTGTACA TATATCAAAA CATCACATTG TACAAAATAC ATACAGTTTT 120
TGTCAATTTA AAGATATCAG AATTCTAGAA TATGATAAAG TTGTGTTTTC AAGCAAGTAA 180
AGATAGNTTA CTTAAA 196

SEQ ID NO:258
SEQUENCE LENGTH:197
SEQUENCE TYPE:nucleic acid
TOPOLOGY:linear
CLONE:HUMGS00275
SEQUENCE DESCRIPTION:
GATCAGATTT TNCATTTTTN AATGTTCCGT GTTTTCTTAA GTAGCATGTA TGACATTTAT 60
AATNTTAAAA AATNTTTTAA AATATGTGTA TGATACATAT TTTCNATTGT CTTAGGGCAG 120
GCTTTTGAAA ATNTCAGCCT GTAGCCAAAT GCAAGATTTT CTCCATCCTT TAATAAAAAG 180
CACACTGAGA AATCCTN 197

SEQ ID NO:259
SEQUENCE LENGTH:199
SEQUENCE TYPE:nucleic acid
TOPOLOGY:linear
CLONE:HUMGS00276
SEQUENCE DESCRIPTION:
GATCTCTGTC TTGAGTTTCT CCTTCCCCAT CAGCTGAAGC ACTCTTCAGA GACTACGTCC 60
ACAGACACTG ATGCTGAGGC CTCCCTGGAG GAAGGAGGGT TAGGGGTGCC TATCCTCAAG 120
TATTGGAAGA GCAGAATTGA GGGAGAGACC TTTCTTCCTT GTTGAGGGTG AAAAATAAAT 180
ANGAATTACA TGTCCTAAA 199

SEQ ID NO:260
SEQUENCE LENGTH:197
SEQUENCE TYPE:nucleic acid
TOPOLOGY:linear
CLONE:HUMGS00277
SEQUENCE DESCRIPTION:
GATCAAGAAG GCTGGAACGG AACTGGTTAA CTTCTTGAGC TATTTCGTGG AACTTGGAAC 60
ACAGCCTGCC ACCCAGTGAA GTGTCCAGAC CATTGTCTTC CAACCCCAGC TGGCCTCTAG 120
AACACCCACT GGCCAGTCCT AGAGCTCCTG TCCCTACCCA CTCTTTGCTA CAATAAATGC 180
TGAATGAATC CNNNAAA 197

SEQ ID NO:261
SEQUENCE LENGTH:195
SEQUENCE TYPE:nucleic acid
TOPOLOGY:linear
CLONE:HUMGS00278
SEQUENCE DESCRIPTION:
GATCTGAGTC AGTCGGAATT TGTGAAACAG GGTAGCAAAC AAGATATTTT ACTTCCATGT 60
ATACAATAAT TTTTTTAAAN NNTGCAATTT GCGTTGCAGC AATCAGTGTT AAATCATTTG 120
CATAAGATTT AACAGCATTT TTTATAATGA ATGTAAACAT TTTAACTTAA TGGTACTTAA 180
AATAATTTAA AAGGN 195

SEQ ID NO:262
SEQUENCE LENGTH:193
SEQUENCE TYPE:nucleic acid
TOPOLOGY:linear
CLONE:HUMGS00279
SEQUENCE DESCRIPTION:
GATCTAGTGT AATGGAAGAC CTTTGAGAAC CTGGGTGTAT TAACTTTGTG TATATAGTGT 60
AAATATCCCC ACTGTACTGT TAGAGGCCAA CAATTCTAGT ATGGCTTGTT GGCAAAGAGT 120
GCTACACCGT TTCAATGAAA CAATGTATGT TTGTTTTAAC TGAACTAAAA TAAATACATG 180
CTTAATCCTG AAA 193

SEQ ID NO:263

SEQUENCE LENGTH:196
SEQUENCE TYPE:nucleic acid
TOPOLOGY:linear
CLONE:HUMGS00280
SEQUENCE DESCRIPTION:
GATCCCTGGG ACCAACCGCA TCCTCAGCTT CTTCCCCGAG AAATGCTGGA GCAGGCTGTT 60
CAGACCGACG TTGCCATCAA AACACATACA CCCAGAAAGA AACAACAGAA ACCAAAACTC 120
ACAAGGCGCA TGATTACTTG TTTTATATTT CATGTTGGGT TTTCCCTCCC ACTATTAAAC 180
AGTCTGTTTC CGTAAA 196

SEQ ID NO:264
SEQUENCE LENGTH:192
SEQUENCE TYPE:nucleic acid
TOPOLOGY:linear
CLONE:HUMGS00281
SEQUENCE DESCRIPTION:
GATCTGTTAA AAAAGAAATC TGTTTCAACA GATGACCGTG TACAATACCG TGTGGTGAAA 60
ATGAATTCAG ACTTATTAAA TGATGAACTT GTTAAATCTN CTCAGTGTCT ATTTATCAGC 120
ACAATACACA CAGGAGANCT GTTGATGGCA TATTGAATAG ATTTNCCTGA ATAANTTGCT 180
CTGGAAACCA AA 192

SEQ ID NO:265
SEQUENCE LENGTH:189
SEQUENCE TYPE:nucleic acid
TOPOLOGY:linear
CLONE:HUMGS00282
SEQUENCE DESCRIPTION:
GATCCTCGTC TTACAGCGAA TGGTTTCAAG ATAAAATTGA TACCAGGAGT TTCAATTACT 60
GAAAATTACT TGGAAATAGA AGGAATGGCT AATTGTNTCC CATTCTATGG AGTAGCAGAT 120
TTAAAAGAAA TNCTTAATGC TATATTAANC AGAAATGCAA AGGNNGTTTA TGANTGTNGA 180
CCTCGCANN 189

SEQ ID NO:266
SEQUENCE LENGTH:188
SEQUENCE TYPE:nucleic acid
TOPOLOGY:linear
CLONE:HUMGS00283
SEQUENCE DESCRIPTION:
GATCTTTTGT ACTTTAGGAC ATTAAATTGT ACAACTTTTG TATATATAAA AGCTTAGGAA 60
CTTTCTGTTT AGCAGGAAGG CAACACATTC CTACACTTTT AATGTATATG TTTGTTATAA 120
TGTCCATGTA AACATGCCCT ATGTTTGTGC CTTTTAATTA GTTTGTCTCA ATAAACAAAA 180
TGTAGAGN 188

SEQ ID NO:267
SEQUENCE LENGTH:206

SEQUENCE TYPE:nucleic acid
TOPOLOGY:linear
CLONE:HUMGS00284
SEQUENCE DESCRIPTION:
GATCCTGAGA ACTTGGAATT CCTTGTAACT GGAGCTCGGA GCTGCACCGA GGGCAACCAG 60
GACAGCTGTG TGTGCAGACC TCATGTGTTG GGTTCTCTCC CCTCCTTCCT GTTCCTCTTA 120
TATACCAGTT TATCCCCATT CTTTTTTTTT TCTTACTCCA AAATAAATCA NGGCTGCAAT 180
GCAGCTGGTG CTGTTCAGAT TCTAAA 206

SEQ ID NO:268
SEQUENCE LENGTH:190
SEQUENCE TYPE:nucleic acid
TOPOLOGY:linear
CLONE:HUMGS00285
SEQUENCE DESCRIPTION:
GATCAGAAAA AGAAAGAAGC CAAAGAGAAA GGTACCTGGG TTCAACTAAA GCGCCANCGT 60
GCTCCACCCA GAGAAGCACA CTTTGTGAGA ACCAATGGGA AGGAGCCTGA GCTGCTGGAA 120
CCTATTCCCT ATGAATTCAT GGCATAATAG GTGTTAAAAA AAAAANTAAA GGGCCCTCTG 180
GGGCTACAAA 190

SEQ ID NO:269
SEQUENCE LENGTH:189
SEQUENCE TYPE:nucleic acid
TOPOLOGY:linear
CLONE:HUMGS00286
SEQUENCE DESCRIPTION:
GATCAAGATA TTAAAATNTC GGATTTATCT TTCCCCATAT CCAAGTACCA ATNCTGTTGT 60
AAACAACGTG TATAGTGCCT AAAATTGTAT GAAAATCCTT TTAACCATTT TAACCTAGAT 120
GTTTAACAAA TCTAATCTCT TATTCTAATA AATATACTAT GAAATAAAAA AAANNGNTTG 180
AAAGCTAAA 189

SEQ ID NO:270
SEQUENCE LENGTH:189
SEQUENCE TYPE:nucleic acid
TOPOLOGY:linear
CLONE:HUMGS00287
SEQUENCE DESCRIPTION:
GATCTTGTGT ATTGAGCTTA TTGTTGAAAG GGATTTTTGA AGGACAGAAC AATTACTGCA 60
TGATGAATCT TCCTNTCTCT GCCTTCTGAG CACCGNCTTT AATTTCCATA TCTTCAAGTC 120
TTGAAGAAGT TGATGTTAAT TGAAGAATTC ACTTGTCTGG TTGAAATAAA GCCTGTTTCT 180
GTTGTGAAA 189

SEQ ID NO:271
SEQUENCE LENGTH:186
SEQUENCE TYPE:nucleic acid

TOPOLOGY:linear
CLONE:HUMGS00288
SEQUENCE DESCRIPTION:
GATCTGATTT GCTAGTTCTT CCTTGTGAGA GTTATAAACT GAGAGTGACG TCACTTCAGC 60
CAGAACATAT TCTCCATACT CTGCATATAA TTTGTGGCTG CAGAATATTG TAATTTGTTG 120
CACACTATGT AACAAAACAA CTGAAGATAT GTTTAATAAA TATTGTACTT ATTGGAAGTA 180
ATATCN 186

SEQ ID NO:272
SEQUENCE LENGTH:185
SEQUENCE TYPE:nucleic acid
TOPOLOGY:linear
CLONE:HUMGS00289
SEQUENCE DESCRIPTION:
GATCTAAAAT GTCAGCATCA TGCAAAGTGC ACGATATATA GTGAATTTNG CTCTAAAAGA 60
GCATGAACAA GTCTTTCTAA TGTTTTGTAC AGTGCCTGGC ACTCTGTGGG TGCTCAATAA 120
ATGGATAGGA GTTTTCATTT GAAGGATATT TGAATTTTTA AAATAAAGTG TTTTATTCCC 180
NTAAA 185

SEQ ID NO:273
SEQUENCE LENGTH:184
SEQUENCE TYPE:nucleic acid
TOPOLOGY:linear
CLONE:HUMGS00290
SEQUENCE DESCRIPTION:
GATCTGGTCA CTGTGGTTCC TGCATGAAGA CAGTGGCTGG CGGTGCCTGG ACGTACAATA 60
CCACTTCCGC TGTCACGGTA AAGTCCGCCA TCAGAAGACT GAAGGAGTTG AAAGACCAGT 120
AGACGCTCCT CTACTCTTTG AGACATCACT GGCCTATAAT AAATGGGTTA ATTTATGTAA 180
CAAA 184

SEQ ID NO:274
SEQUENCE LENGTH:185
SEQUENCE TYPE:nucleic acid
TOPOLOGY:linear
CLONE:HUMGS00291
SEQUENCE DESCRIPTION:
GATCTGATTT GCTAGTTCTT CCTTGTAGAG TTATAAATGG AAANATTACA CTATCTGATT 60
AATAGTTTCT TNATACTCTG CATATAATTT NTGGCTGCAG AATATTGTAA TTTGTTGCAC 120
ACTATGTAAC AAAACAACTG AAGATATGTT TAATNNATAT TGTACTTATT GGAAGTAATA 180
TCAAA 185

SEQ ID NO:275
SEQUENCE LENGTH:188
SEQUENCE TYPE:nucleic acid
TOPOLOGY:linear

CLONE:HUMGS00292
SEQUENCE DESCRIPTION:
GATCAAAGAC ATCCTCATCC AGTATGACCG GACCCTGCTG GTAGCTGACC CTCGTCGCTG 60
CGAGTCCAAA AAGTTTGGAG GCCCTGGTGC CCGCGCTCGC TACCAGAAAT CCTACCGATA 120
AGCCCATCGT GACTCAAAAC TCACTTGTAT AATAAACAGT TTTTGAGGGA TTTTAAAGTT 180
TCAAGAAA 188

SEQ ID NO:276
SEQUENCE LENGTH:182
SEQUENCE TYPE:nucleic acid
TOPOLOGY:linear
CLONE:HUMGS00293
SEQUENCE DESCRIPTION:
GATCGGGNTA CTACAAAGTT CTGGGAAAGG GAAAGCTCCC AAAGCAGCCT NGTCATCGTG 60
AAGGCCAAAT NCTTCAGCAG AAGAGCTGAG GAGAAGATTA AGAGTGTTGG GGGGGCCTGT 120
NTCCTGGTGG CTTGAAGCCA CATGGAGGGN GTTTCATTAA ATGCTAACTA CTTTTNCCTA 180
AA 182

SEQ ID NO:277
SEQUENCE LENGTH:195
SEQUENCE TYPE:nucleic acid
TOPOLOGY:linear
CLONE:HUMGS00294
SEQUENCE DESCRIPTION:
GATCCAGCCA GAGGATGCCA CGCCAGCCCA GCGCTACCAG GCTGCCCAAG GGGGGCGGGC 60
CTGGGAAGAG CCCTACACGG GGCAGCACCT AGGATGGGGC AGAGACTTGT TGCATCTTTG 120
TCCCCAGCAA AGGCTACATG TTACCTCCTT CAATTGATAA TAAACCTTTC TGAGATGCAG 180
AGGGTCCAGG TCAAA 195

SEQ ID NO:278
SEQUENCE LENGTH:327
SEQUENCE TYPE:nucleic acid
TOPOLOGY:linear
CLONE:HUMGS00295
SEQUENCE DESCRIPTION:
GATCCCATTA ACTCGATGCT GAGTATCTAC ATGGATACAT TAAATATATT TATGCGAGTT 60
GCAACTATGC TGGCAACTGG AGGCAACAGA AAGAAATNAA GTGACTCAGC TTCTGGCTTC 120
TCTGCTACAT CAAATATCTT GTTTAATGGG GCAGATATGC ATTAAATAGT TTGTACAAGC 180
AGCTTTCGTT GAAGTTTAGA AGATAAGAAA CATGTCATCA TATTTAAATG TTCCGGTAAT 240
GTGATGCCTC AGNTCTGCCT TTTTTTCTGG AGAATAAATG CAGTAATCCT CTCCCAAAAA 300
AAAAAAAAAN NNNTNNNTNN NNNTNNN 327

SEQ ID NO:279
SEQUENCE LENGTH:183
SEQUENCE TYPE:nucleic acid

TOPOLOGY:linear
CLONE:HUMGS00296
SEQUENCE DESCRIPTION:
GATCCAAATC CTCATCTTAC TTTCCCGACC TTAAGGATGT AGCTGCTGCT TGTCCTGTTC 60
AAGTTGCTGG AGCAGGGGTC ATGTGAGGCC AGGCCTGTAG CTCCTACCTG GGGCCTATTT 120
CTACTTTCAT TTTGTATTTC TGGTCTGTGA AAATGATTTA ATAAAGGGAA CTGACTTTGG 180
AAA 183

SEQ ID NO:280
SEQUENCE LENGTH:180
SEQUENCE TYPE:nucleic acid
TOPOLOGY:linear
CLONE:HUMGS00297
SEQUENCE DESCRIPTION:
GATCAAAGTG GCTGCAGCAG AGTTAGCTGT CTAGCGCCTA GCAAGGTGCC TTTGTACCTC 60
AGGTGTTTTA GGTGTGAGAT GTTTCAGTGA ACCAAAGTTC TGATACCTTG TTTACATGTT 120
TGTTTTTATG GCATTTCTAT CTATTGTGGC TTTACCAAAA AATAAAATGT CCCTACCAGN 180

SEQ ID NO:281
SEQUENCE LENGTH:180
SEQUENCE TYPE:nucleic acid
TOPOLOGY:linear
CLONE:HUMGS00298
SEQUENCE DESCRIPTION:
GATCACATGA TTTTTGTTCT CAGTTCTATT AATGTTCTTG GATTCTGTTC AATGTTCTGT 60
TCACATTGCA GAAAAAGCAT TTGACAAAAT AATTTCAGTA GCTGCTGAAA AAGCATTTGA 120
TAAAATTCAG CATACCTTTA TGATACAAAA AACCTTCAAT AAACTGGGTA TATATGNACN 180

SEQ ID NO:282
SEQUENCE LENGTH:178
SEQUENCE TYPE:nucleic acid
TOPOLOGY:linear
CLONE:HUMGS00299
SEQUENCE DESCRIPTION:
GATCTTGAAT TATTTATAAA CTGGAAAGTG GTTTGATTAT TGTGAGTCAA AACTCTAAGT 60
GGTTAAAAAT TAGTATGAAT TTTTTAGCTT CTTAATGAAT ATGGATTTAA AACTCTCCAG 120
TTCTTATTTT ATGAAATGAC TTGCCTTTCT GGTAATACAA TGCTGATTTT TTAGTAAA 178

SEQ ID NO:283
SEQUENCE LENGTH:182
SEQUENCE TYPE:nucleic acid
TOPOLOGY:linear
CLONE:HUMGS00300
SEQUENCE DESCRIPTION:
GATCCCAAAT ATGAAGTCAT CGAAAAACCC CAGGCCTGAA GAAATAAAGT AAAAATNAAT 60

CTGGTAATTT GTCACGGATT AGTTGTACAA CTAGTTAGAA GTTTCAGAAT AAACATGCAT 120
TTCATAACTG TCAAATGTTC TTTTAATTCT GAGTCCAAAT AAATTATTTG GTGATGTTGA 180
AA 182

SEQ ID NO:284
SEQUENCE LENGTH:179
SEQUENCE TYPE:nucleic acid
TOPOLOGY:linear
CLONE:HUMGS00301
SEQUENCE DESCRIPTION:
GATCTCTGAG TGAGAGGGAA ANAGGTCAGA TTTATACAAC TGAGCGCCAG AGGGGAAAAT 60
GCACCTTGTT GGAGTGAGAA ATGTTCTGAA ACTGAATTAC TTCTTGTACA GCTGAGATAG 120
CTTCTTCTGA ACTATTATTA AATAAGTGAA TACAAAGGCC CTATGATGGG AAATCCAGN 179

SEQ ID NO:285
SEQUENCE LENGTH:201
SEQUENCE TYPE:nucleic acid
TOPOLOGY:linear
CLONE:HUMGS00302
SEQUENCE DESCRIPTION:
GATCACCTGT TTGAAACCAT CTCCCAAGCC ATGCTGAATG CTGTGGACCG GGATGCAGTN 60
TCAGGCATGG GAGTCATTGT CCACATCATC GAGAAGGACA AAATCACCAC CAGGACACTG 120
AAGGCCCGAA TGGACTAACC CTGTTCCCAG AGNCCACTTT TTTTTCTNTT TTTGANATAA 180
AATAGCCTGT CTTTCAANAA A 201

SEQ ID NO:286
SEQUENCE LENGTH:176
SEQUENCE TYPE:nucleic acid
TOPOLOGY:linear
CLONE:HUMGS00303
SEQUENCE DESCRIPTION:
GATCGGAGAT GCTTTGTAAT CTACTGTCCA GCTGGAAACA GCTCATGTTA CGCGGAAAAA 60
ACTACAAGTA ATGTTCAAAT CTATTTTGGG TCATTTTTAT GTACCTTTGG GTTCAGGCAT 120
TATTTGGGGG GTTTTGTTTC CAAAGGAACT AAATAAAGTC ATATTGCTTA TAGAAA 176

SEQ ID NO:287
SEQUENCE LENGTH:176
SEQUENCE TYPE:nucleic acid
TOPOLOGY:linear
CLONE:HUMGS00304
SEQUENCE DESCRIPTION:
GATCTTTGCT GGGAAACAGC TGTGAAGATG GACGCACCCT GTCTGACTAC AACATCCAGA 60
AAGAGTCCAC TCTGCACTTG GTCCTGCGCT TGAGGGGGGG TGTCTAAGTT TCCCCTTTTA 120
AGGTTTCAAC AAATTTCATT GCACTTTCCT TTCAATAAAG TTGTTGCATT CCCAAA 176

SEQ ID NO:288
SEQUENCE LENGTH:176
SEQUENCE TYPE:nucleic acid
TOPOLOGY:linear
CLONE:HUMGS00305
SEQUENCE DESCRIPTION:
GATCCGCAAG ACCAAGTACC GCCCCGACCT GCGCATGGCA GCCATCCGCA GGCCAGCNTC 60
ATCCTGCGCA NCAGAAGCCT GTAATNGTGA AGAGGAAGCG GACCCGACCC ACCAAGAGCT 120
CCTGAGCCCC CTGCCCCCAG AGCAATAAAG TCAGCTGGCT TTCTNACCTG AAGAAA 176

SEQ ID NO:289
SEQUENCE LENGTH:175
SEQUENCE TYPE:nucleic acid
TOPOLOGY:linear
CLONE:HUMGS00306
SEQUENCE DESCRIPTION:
GATCTCAAAA CACAGTGAGA GGTCTGAAGG CTGGCTTCTG AAGAATCCCT GATGTCTTAT 60
TGGAACAACC ACTGAGCTAC GGAGAGCTCT GCTGTGATGG GCTAGGCACT TTATATCTGT 120
GTGAATACAG ATTTATAAAA CAGGTTAATA AACTTATCCA AGGTCACATT TCAAA 175

SEQ ID NO:290
SEQUENCE LENGTH:165
SEQUENCE TYPE:nucleic acid
TOPOLOGY:linear
CLONE:HUMGS00307
SEQUENCE DESCRIPTION:
GATCTGAATT CTTTATGTAT ATTTGTAGCT ATATTTCATA CAAAGTGCTT TAAGTGTGGA 60
GAGTCAATTA AACACCTTTA CTCTTAGAAA TACGGATTCG GCAGCCTTCA GTGAATATTG 120
GTTTCTCTTT GGTATGTCAA TAAAAGTTTA TCCGTATGTC AGAAA 165

SEQ ID NO:291
SEQUENCE LENGTH:186
SEQUENCE TYPE:nucleic acid
TOPOLOGY:linear
CLONE:HUMGS00308
SEQUENCE DESCRIPTION:
GATCATTCCT TCTGTAGCTC AGGAGAGCAC CCCTCCACCC CATTTGCTCG CAGTATCCTA 60
GAATCTTTGT GCTCTCGCTG CAGTTCCCTT TGGGTTCCAT GTTTTCCTTG TTCCCTNCCA 120
TGCCTAGCTG GNTTGCAGAG TTAAGTTTAT GATTATGAAA TAAAAAACTA ACTGACAATT 180
NTCAAA 186

SEQ ID NO:292
SEQUENCE LENGTH:175
SEQUENCE TYPE:nucleic acid
TOPOLOGY:linear

CLONE:HUMGS00309
SEQUENCE DESCRIPTION:
GATCTTGTAG AAAATTTTGA TGAGGCATCA AAGAATGAAG CTAACTAAAA GTTTGGTTTT 60
TGGAAGCTGG CATGGACTAG ATTTAACAAA TCAGCTATGT GGTTCCAAAG TTTTACAGAC 120
ATGGAGAACA TCACCTGTTA CTAGTTCAGT AATATAAATA TTTTGTATAT TAATN 175

SEQ ID NO:293
SEQUENCE LENGTH:406
SEQUENCE TYPE:nucleic acid
TOPOLOGY:linear
CLONE:HUMGS00310
SEQUENCE DESCRIPTION:
GATCAGATTA CCAGGAACAT CAGGAGTGGA TTCCTGCCCC AACCAAACCG CATTCGTGTG 60
GATTTTTTTA TTCAACTTAA TTGGCTATTC CAAAGATTTT TTTTTTCCTA TTTTTGACGA 120
TTGGNGCCCT TAAGATGCAC GATGGAATTG TGTTTTNCGT TTTTNGGTAA AAGGAGCAAA 180
GCGNGGNCCT GGAGATAAAC GCTGGAGCAA TCTCCTTGGA AGGATTCAGC ACGAGTAGAT 240
GGTAAACATT TAAAGGGGAA AGGGGGGTTT GTTTAAAATA GTAAATCAGT AAGTCACTTC 300
TAAATTTAAA GAAACCAAAA TTGGGAGTTG AAGAATAAGT AGGGTTTCCA ATTGGGCTAT 360
TGCCGNTTTN CTTTGNAAAA ATTAAACCAT TNTTTAAAAA CCTAAA 406

SEQ ID NO:294
SEQUENCE LENGTH:218
SEQUENCE TYPE:nucleic acid
TOPOLOGY:linear
CLONE:HUMGS00311
SEQUENCE DESCRIPTION:
GATCCCATAG ACCAGAGCCC ACCTTTTNNA TAAACTTAGT AAAGTCTTNG AGACTAGAAG 60
CAAGATAGTT TGTGACACAT AAGCTTCCCA AAAACTNGAA TAGATTTTNA CTGAATAGTG 120
GTCTATCTGA TGGTATATGT TTCTTAAAGG TCCAANTGTA ATAAAAAAAA TTGAAAAANA 180
GGTCTCAGTG TTTTNAATGC ACTNCATATT TGTNTGCN 218

SEQ ID NO:295
SEQUENCE LENGTH:176
SEQUENCE TYPE:nucleic acid
TOPOLOGY:linear
CLONE:HUMGS00312
SEQUENCE DESCRIPTION:
GATCCACCAA GCACGCCTAT NAATACAAAG TAAACTATNA TTTTNATTGT GAAATTTTCA 60
TAGATGGAAA ATTGAATATN CTGTCCATTT CATTTTACAA TNATCTTACC ACTTATTTTT 120
GTACCATGTA TTTCAATTGC CTGTTTAGTG AAAAATAAAA ATTAAAAAAA CCTAAA 176

SEQ ID NO:296
SEQUENCE LENGTH:172
SEQUENCE TYPE:nucleic acid
TOPOLOGY:linear

CLONE:HUMGS00313
SEQUENCE DESCRIPTION:
GATCCCAGCA AGATAATGTC CTGTCTTCTA AGATGTGCAT CAAGCCTGGT ACATACTGAA 60
AACCCTATAA GGTCCTGGAT AATTTTTGTT TGATTATTCA TTGAAGAAAC ATTTATTTTC 120
CAATTGTGTG AAGTTTTTGA CTGTTAATAA AAGAATCTGT CAACCATCTA AA 172

SEQ ID NO:297
SEQUENCE LENGTH:173
SEQUENCE TYPE:nucleic acid
TOPOLOGY:linear
CLONE:HUMGS00314
SEQUENCE DESCRIPTION:
GATCGGTGAC ATCGTCAAGA TGGGCGAGTG CCGGCCTCTG AGCAAGACAG TGCGCTTCAA 60
CGTGCTCAAG GTCACCAAGG CTGCCGGCAC CAAGAAGCAG TTCCAGAAGT TCTGAGGCTG 120
GACATCGGCC CGCTCCCCAC AATGAAATAA AGTTATTTTC TCATTCCCAG AAA 173

SEQ ID NO:298
SEQUENCE LENGTH:167
SEQUENCE TYPE:nucleic acid
TOPOLOGY:linear
CLONE:HUMGS00315
SEQUENCE DESCRIPTION:
GATCGTTTCC AGATGAGAAT TCACAAGCGA CTCATTGACT TGCACAGTCC TTCTGAGATN 60
TGTTAAGCAG ATTACTTCCA TCAGTATTGA GCCAGGAGTT GAGGTGGAAG TCACCATTGC 120
AGATGCTTAA GTCAACTATT TTAATAAATT GATGACCAGT TGTTAAA 167

SEQ ID NO:299
SEQUENCE LENGTH:171
SEQUENCE TYPE:nucleic acid
TOPOLOGY:linear
CLONE:HUMGS00316
SEQUENCE DESCRIPTION:
GATCTGGTGG TGGAAGTGGT GGATATGGTA GCAGAAGGTT CTAAAAACAG CAGAAAAGGG 60
TTGAATGAGA ACCCTACTTG CCTAAATNAG GAATGTCTTT CCTACCATCT AAAATACGAA 120
GGTTTCTGGC TGGGTAAGGT TTGTAGTTGA CAGTAAAACC TGATGACACC N 171

SEQ ID NO:300
SEQUENCE LENGTH:176
SEQUENCE TYPE:nucleic acid
TOPOLOGY:linear
CLONE:HUMGS00317
SEQUENCE DESCRIPTION:
GATCCGAGTG TGATTTGAAT TCTGTGATAT TTTCACACTG GTAAATGTTA CCTCTATTTT 60
ACTTACTGCT ATAAATAGGT TTATATTATT GATTCACTTA CTGACTTTGC ATTTTCGTTT 120
TTAAAAGGAT GTATAAATTT TTACCTGTTT AAATAAAATT TAATTTCAAA TGTAAA 176

SEQ ID NO:301
SEQUENCE LENGTH:169
SEQUENCE TYPE:nucleic acid
TOPOLOGY:linear
CLONE:HUMGS00320
SEQUENCE DESCRIPTION:
GATCTTTGTT TTGTGTTTAA CCATAATGGT TGTGTACTGA ACCACTTCAT ATTTGTAATA 60
TATAATATAT ATATATNNGN TNCCCTNAAG ACTCAGCCTC CTGGTTTACC CCCCCGGCCT 120
GGGCATCTNA CCTCCCCCAC CCCAGTGTGA TTTAACATCC NGGNACTGN 169

SEQ ID NO:302
SEQUENCE LENGTH:174
SEQUENCE TYPE:nucleic acid
TOPOLOGY:linear
CLONE:HUMGS00322
SEQUENCE DESCRIPTION:
GATCTAGCTC TGAATGTATG TTTCCTGACG TTTTACATTT CCACTTTCCT ATTCCATTCA 60
TTAAGCTAGC CAACAATCCA CCATCCTTTA AAGATTGTTC TCATAACTGA ACAAAAACCA 120
CATAATCTAA ATAGAGCAAA GCTACAAGAA ATAAATTTAT TTAAACGCAA GAAA 174

SEQ ID NO:303
SEQUENCE LENGTH:177
SEQUENCE TYPE:nucleic acid
TOPOLOGY:linear
CLONE:HUMGS00323
SEQUENCE DESCRIPTION:
GATCCTAGCA TATGTTAAAA TTCAAATTAA TGTAAAACAG ATTAACAACA ACAAAGAAAC 60
TGTCTATTTG AGTGAAGTCA TGCTTTCTAT TATAATAACT TGGCTTCGGT TATCCATCAA 120
ATGCACACNN ATACTGTTAT CTGATTGTTT ATAATAAAGA ATACTGTACC TNNTAAA 177

SEQ ID NO:304
SEQUENCE LENGTH:167
SEQUENCE TYPE:nucleic acid
TOPOLOGY:linear
CLONE:HUMGS00325
SEQUENCE DESCRIPTION:
GATCAGATGG TTTTAGTATT GTGGCAGAAG CGAGAAAACT TTGTTTATTG AAAAAAAAAG 60
AAAAAGAAAG CAAGAAAAAA AGATACTATG GGGTCAAGTG TAACTCCATG GAAATGCCAC 120
GTCTGCTCTT CAGTGAAGAA GCTGGTTTAG AGTCTCACAG AAAACTN 167

SEQ ID NO:305
SEQUENCE LENGTH:170
SEQUENCE TYPE:nucleic acid
TOPOLOGY:linear

CLONE:HUMGS00327
SEQUENCE DESCRIPTION:
GATCGCTCAC AATNTTTCCT CCAAGAACCG CAAAGCCATC GTGGAAAGAG CTGCCCAACT 60
GGCCATCANA GTCACCAACC CCAATNCCAG GCTGCGCAGT GAAGAAAATA AGTAGGCAGC 120
TCATGTGCAC ATTTTCTGTT TAAATAAATG TAAAAACTGC CATCTGGAAA 170

SEQ ID NO:306
SEQUENCE LENGTH:171
SEQUENCE TYPE:nucleic acid
TOPOLOGY:linear
CLONE:HUMGS00328
SEQUENCE DESCRIPTION:
GATCGGGAAT GGTCTGTGTG TTATCAGCTG CGACTGGTTC ACTGCGNCTT AGACAAGCCT 60
CATGGGGACT GGGGATTCTG GCCAGTGTAA TTTCTGTCAA CCACGGACGT TTGCCTTCAT 120
GTGTAGAATT TACTGTTGTT ATGCAAATTA TATTTTCAAT TATAAATGAA A 171

SEQ ID NO:307
SEQUENCE LENGTH:164
SEQUENCE TYPE:nucleic acid
TOPOLOGY:linear
CLONE:HUMGS00329
SEQUENCE DESCRIPTION:
GATCAGACAG AATAATATTT NCTAGTTATT ATGTGTAAGA TGAGTTGCTA TTTTCCTAAT 60
GCTCATTCTG ATACAACTAT TTTCCGTGTC AAATATCTAC TGTGCCCAAA TGTACTCAAT 120
TTAAATCATT ACTCTGTAAA ATAAATAAGC AGATGATTCT TAAA 164

SEQ ID NO:308
SEQUENCE LENGTH:261
SEQUENCE TYPE:nucleic acid
TOPOLOGY:linear
CLONE:HUMGS00330
SEQUENCE DESCRIPTION:
GATCCCCGCC CTGGGGTCTG GTCCTCGCCC ATCCTGCAGG GATTGCCCAC CGTCTTCCAG 60
ACACCCCACC TGAGGGGGGC ACCAGGTTTA GTGCTGCTGC TTTCACTGNT GCACCCGCGC 120
CCTCGGCCGG CCCCCCGAGC AGCCTTTGTA CTCTGCTTGC GGAGGGCTGG GAGACCCTCC 180
AGGACATTCC CACNNTCNCC CATGCTGCCA AGTTNNNNCT ATAGCTACAA ATAAAAAAAA 240
ACCTTGTTTT CAAGAAATAA A 261

SEQ ID NO:309
SEQUENCE LENGTH:163
SEQUENCE TYPE:nucleic acid
TOPOLOGY:linear
CLONE:HUMGS00331
SEQUENCE DESCRIPTION:
GATCGAGTCA AGATGAGTTA GTGGAGCTGG GCTTGGCCAG GGAGTCTGGG GACAAGGAAG 60

CAGATTTTCC TGATTCTGGC TCTAGCTTCC CTGCCAAGAT TTTGGTTTTN ATTTTTTTAT 120
TTGAACTTTA GTCGTGTAAT AAACTCACCA GTGGCAAACC AAA 163

SEQ ID NO:310
SEQUENCE LENGTH:165
SEQUENCE TYPE:nucleic acid
TOPOLOGY:linear
CLONE:HUMGS00332
SEQUENCE DESCRIPTION:
GATCATGCTG CTGTGATACT GAGTTTTCTA AACAGCATAA GGAAGACTTG CTCCCCTGTC 60
CTATGAAAGA GTATAGTTTT GGAGGGGAGA AGTGGGACAA AAAAGATGCA GTTTTCCTTT 120
GTATTGGGAA ATGTGAAAAT AAAATTNTCA ACTCTTTCAG TTAAA 165

SEQ ID NO:311
SEQUENCE LENGTH:164
SEQUENCE TYPE:nucleic acid
TOPOLOGY:linear
CLONE:HUMGS00333
SEQUENCE DESCRIPTION:
GATCTCAGTT TCCTGGCTTT TCCTCCCTCA GCCCCTTCTC ACCCCTTTGC TGTCCTGTGT 60
AGTGATTTGG TGAGAAATCG TTGCTGCACC CTTCCCCCAG CACCATTTAT GAGTCTCAAG 120
TTTTATTATT GCAATAAAAG TGCTTTATGC CGGCTTTTCT CAAA 164

SEQ ID NO:312
SEQUENCE LENGTH:337
SEQUENCE TYPE:nucleic acid
TOPOLOGY:linear
CLONE:HUMGS00334
SEQUENCE DESCRIPTION:
GATCATTGAA TGTGAGACCC TTCTAACATG ATTTGAGAAG CTGTACAAGT ATAGGCAGAG 60
TTATTTTCCT GTTTACATTT TTTTTTTGTT TTGGGGAAAA AATTGGTAGG TGTCTAATNA 120
CTGTTTACTT CATTGTTATA TTGCAGTAAA AGTTTTAAAN CANCCATTGC ATGTTNGCTT 180
TTGATGTATC CCTTTGNGAA ATTAGCACTT TTGGGGCCAN TGGNGAAATG CAGCATTCAC 240
TCTCCCTGTC TTTTCCCCTT CCCTCAGCAG AAACGTGTTT ATCAGCANGT CGTGAGTCAA 300
ACTGCTGCCT TTTAAAAANC CCACAAANTT GNTNNGN 337

SEQ ID NO:313
SEQUENCE LENGTH:176
SEQUENCE TYPE:nucleic acid
TOPOLOGY:linear
CLONE:HUMGS00335
SEQUENCE DESCRIPTION:
GATCCGCCGT CACTGGGGTG GCAATGTCCT GGGTCCTAAG TCTGTGGCTC GTATCGCCAA 60
GCTCGAAAAG GCAAAGGCTA AAGAACTTGC CACTAAACTG GGTTAAATGT ACACTGTTGA 120
GTTTTCTGTA CATAAAAATA ATTGAAATAA TACAAATTTT CCTTCAGCCA GTGAAA 176

SEQ ID NO:314
SEQUENCE LENGTH:176
SEQUENCE TYPE:nucleic acid
TOPOLOGY:linear
CLONE:HUMGS00336
SEQUENCE DESCRIPTION:
GATCACCTCT GAGACCCACC TTGCTCATAA ACAAAATGCC CATGTTGGTC CTCTGCCCTG 60
GACCTGTGAC ATTCTGGACT ATTNNTGTGT TTATTTGTGG CCGAGTGTAA CAACCATATA 120
ATAAATCACC TCTTCCGCTG TTTTAGCTGA AGNATTANGN CATCTTGTCT ATTAAA 176

SEQ ID NO:315
SEQUENCE LENGTH:258
SEQUENCE TYPE:nucleic acid
TOPOLOGY:linear
CLONE:HUMGS00337
SEQUENCE DESCRIPTION:
GATCATATTT AATGAATTAT AGTATAATGC TTGCAGGCCC AGTACAAGCA TATATATNGT 60
GCCTCTTACA GCCTTTGGAA TACATTGTTT CCATTTTTTA AATATCTTCT ATATCCNNNT 120
AGTATTCAAA TTATTAATGC TCATGTACCA AGGTNTTGCT ATAAAAGTTT TGTCTGTATG 180
AATAATGTGG CTTTAGTAAA TAATCATTTN TCAACTGTAA ACTNATTCTG AAATAAAGTA 240
AAATNCTAAT TGTTTAAA 258

SEQ ID NO:316
SEQUENCE LENGTH:153
SEQUENCE TYPE:nucleic acid
TOPOLOGY:linear
CLONE:HUMGS00338
SEQUENCE DESCRIPTION:
GATCTTGGAC ACTTACAGAT TGAGCTGTAT GAATTCAGCG GGTCTCACTC CAGAGGGTCA 60
GAACGTTTGC TTTAGTTTTT TCATCTGTTT TGTTCCTTGA GTCAGTGCTG TTGATGATGA 120
GTTGTCTTGA ATAAATNATG TGTTCTTTGC AAA 153

SEQ ID NO:317
SEQUENCE LENGTH:157
SEQUENCE TYPE:nucleic acid
TOPOLOGY:linear
CLONE:HUMGS00339
SEQUENCE DESCRIPTION:
GATCAAAGCT AGAAAATGNA GATTCCTTAG CCTGGATTTC CTTCTAACAT GTTATCAAAT 60
CTGGGTATCT TTCCAGGCTT CCCTGACTTG CTTTAGTTTT TAAGATTTGT GTTTTNCTNT 120
NTCCACAAGG AATAAATGAG AGGGAATCGA CTGTAAA 157

SEQ ID NO:318
SEQUENCE LENGTH:161

SEQUENCE TYPE:nucleic acid
TOPOLOGY:linear
CLONE:HUMGS00340
SEQUENCE DESCRIPTION:
GATCTCCTTA TATAGCAGCC AAAATCAATG AAGCTAAAGA TTTACTAGAA GGTCAAGCTA 60
AAAAATGAAG TAAATGTATG ATGAATTTTA AGTTCGTATT AGTTTATGTA TATGAGTACT 120
AAGNNTTTTA TAATAAAATG CCTCAGAGCT ACAATTTTAA A 161

SEQ ID NO:319
SEQUENCE LENGTH:313
SEQUENCE TYPE:nucleic acid
TOPOLOGY:linear
CLONE:HUMGS00341
SEQUENCE DESCRIPTION:
GATCTGAAAA CATGTAGAGA AGATGAGTTG AGGACAGCTT TTCTAAGGCA ATGTNATGTC 60
TTTCCTTTCT NATTTCTNTT TCTCTGCGTT GTTAGTTTTN AAGAGTGGAG GAGCTAGGGG 120
CTCCAGAAAG AATCTTACAC ATGTTTTGAA GACATTGATG TCATAGGGAG CGGGGAGCTG 180
CATTCCCTTC TGGGCTGTTA CTGCTAAATC TCAGTATGAA CAGACCAGGC GGAAAGCTTG 240
GTGGCCAAGC AGTCTGTGTG CTTCCCCGCT GATGGAGAAC GTTGCGTTGT TCACAATAGG 300
GCCTCATGGG TGN 313

SEQ ID NO:320
SEQUENCE LENGTH:161
SEQUENCE TYPE:nucleic acid
TOPOLOGY:linear
CLONE:HUMGS00342
SEQUENCE DESCRIPTION:
GATCTTGTTC ACAAGTAATC TGTTGACAGT GCCAATAAAT NATAAAAAAA AAATTAACAT 60
GTCACAATGT AACGGATGAC CATATGCACA ATTCCATGAA TTAAATCTGT TTCCTGTGTT 120
AGTCAGTATT CTTAAATAAA ATTTATAATT GAAACATGAA A 161

SEQ ID NO:321
SEQUENCE LENGTH:163
SEQUENCE TYPE:nucleic acid
TOPOLOGY:linear
CLONE:HUMGS00343
SEQUENCE DESCRIPTION:
GATCTATTGA NAGCCCTCTC TCNCATTCTG TAATGAGTAC AGCAGAGACC TTCCTGCTTT 60
TAACTGGGGA CTCCAGATTT TCCCCAAACT TGCTTCTGTT GAGATTTTTC CCTCACCTTG 120
CCTCTCAGGC ACAATAAATA TAGTTATACC ACTGCCCATC AAA 163

SEQ ID NO:322
SEQUENCE LENGTH:156
SEQUENCE TYPE:nucleic acid
TOPOLOGY:linear

CLONE:HUMGS00344
SEQUENCE DESCRIPTION:
GATCATCCTA ACAATGTGGG GCTGTTAGGT TTTACCTTTG ANCTTTCATA GCACTGCAGA 60
AACCTTTAAA AAAAAAATGN TNNATGAATT TTTCCTTTCC TACAGTTGGG TAGGGTAGGG 120
GAAGGNGGNT AAGCTTTTTT TTTTNAAATG ACTGAN 156

SEQ ID NO:323
SEQUENCE LENGTH:255
SEQUENCE TYPE:nucleic acid
TOPOLOGY:linear
CLONE:HUMGS00346
SEQUENCE DESCRIPTION:
GATCATGCTG CCCTGGGACC CAACTGGTAA GATTGGCCCT AAGAAGCCCC TGCCTGACCA 60
CGTGAGCATT GTGGAACCCA AAGATGAGAT ACTGCCCACC ACCCCCATCT CAGAACAGAA 120
GGGTGGGAAG CCAGAGCCGN CTGCCATGCC CCAGCCAGTC CCCACAGCAT AACAGGGTCT 180
CCTTGGCAGC TGTATTCTGG AGTCTGGATG TTGCTCTCTA AAGACCTTTA ATAAAATTTT 240
GTACAAAGGC ACAAA 255

SEQ ID NO:324
SEQUENCE LENGTH:154
SEQUENCE TYPE:nucleic acid
TOPOLOGY:linear
CLONE:HUMGS00347
SEQUENCE DESCRIPTION:
GATCTCTAGT ATAACACTCA GGCTACTGAG GTATTTTAGA GCAACAAGCT GGGTTACTTT 60
CAGAGCAACC AGCTTGACTG GAACTGAGAG TAAATTGGGA ATGTATGACC AATCTTAGAC 120
CCTGAAAAAT GGCAGAAAAT ACATGGAAAT TTGN 154

SEQ ID NO:325
SEQUENCE LENGTH:153
SEQUENCE TYPE:nucleic acid
TOPOLOGY:linear
CLONE:HUMGS00348
SEQUENCE DESCRIPTION:
GATCTAGGGA AGACAACGTA GTCACCCTCG GTGCTTCCTC TGTCTCCTCT TTCTCCCTGG 60
CCTGTGGTTG TCCCCCAGCC TCTGCCACCC TCCACCTCCT CGGTCAGCCC CAGCCCCAGG 120
TTGATAAATC TATTGATTGA TTGTGATAGT AAA 153

SEQ ID NO:326
SEQUENCE LENGTH:154
SEQUENCE TYPE:nucleic acid
TOPOLOGY:linear
CLONE:HUMGS00349
SEQUENCE DESCRIPTION:
GATCTCTAAT ATTTTTAAGC CCAAGCCCCT TGGACACTGC AGCTCTTTTC AGTTTTTGCT 60

TATACACAAT TCATTCTTTG CAGCTAATTA AGCCGAAGAA GCCTGGGAAT CAAGTTTGAA 120
ACAAAGATTA ATAAAGTTCT TTGCCTAGTA TAAA 154

SEQ ID NO:327
SEQUENCE LENGTH:158
SEQUENCE TYPE:nucleic acid
TOPOLOGY:linear
CLONE:HUMGS00350
SEQUENCE DESCRIPTION:
GATCAAATTC TAATGGAATT GAGCCGGTTT CTTATCCTAA ATGTTTCCTC CCTTTTTACA 60
ATCTCTGTCC AGCACCTCTT GGTTAAATAA TGTATGCTGT GAGACATGNA ATTAAAACAG 120
GCCTATGGAA TAAATTATTT TAAAACCAGN AGGTTAAA 158

SEQ ID NO:328
SEQUENCE LENGTH:155
SEQUENCE TYPE:nucleic acid
TOPOLOGY:linear
CLONE:HUMGS00351
SEQUENCE DESCRIPTION:
GATCTTAAAG CAGAATGCCN TTTNCTTTTT TTGCTTCAGT TGTAAAGAAG AGGGAATACA 60
TGATAAAGTA ACTGGTTTGA TTTCTCGTTC ATTGTACACT GCCTCTGAAC ANCTAATTGT 120
TTTTAGTTGT CTAAATAAAA TGCCTCTAAA ACAAA 155

SEQ ID NO:329
SEQUENCE LENGTH:165
SEQUENCE TYPE:nucleic acid
TOPOLOGY:linear
CLONE:HUMGS00352
SEQUENCE DESCRIPTION:
GATCCAAGAG GAAGAATCCA GCTGCCTATG AAAATAACAA ATNAGCAACG CATCCGGATG 60
ACGGTTCCCT GTCTCTGAAA GACCTTTCTC TGGAAGAGGA GTCTGCATTG TAGTGTCTCA 120
AAGACACAAT AAACTTCCTA TGGTCTGCAC TGTTGTGATA TTAAA 165

SEQ ID NO:330
SEQUENCE LENGTH:149
SEQUENCE TYPE:nucleic acid
TOPOLOGY:linear
CLONE:HUMGS00353
SEQUENCE DESCRIPTION:
GATCTACTTT GTTTGAGCAA AACAGCATTA TTTGTTATGT TAATNATGGT TAATTTCCAT 60
TTTATTGGTT TTATGTTTAT TTTAATTTGT AAATGTTTTA GCATTTATNA TTGTATGTNA 120
NCTATATTTN CCTATTTNAT GTTGATAAA 149

SEQ ID NO:331
SEQUENCE LENGTH:151

SEQUENCE TYPE:nucleic acid
TOPOLOGY:linear
CLONE:HUMGS00354
SEQUENCE DESCRIPTION:
GATCAAAATG CTAAAACATG ATGATTAAGT GCACACCGTG TGCCATAGAA TGGCACATGT 60
CATTGCCCAC TTCTGTGTAG ACATGGTTCT GGTTTAACTA ATATTTGTCT GTGTGCTACT 120
AACAGATTAT AATAAATTGT CATCAGTGAA A 151

SEQ ID NO:332
SEQUENCE LENGTH:147
SEQUENCE TYPE:nucleic acid
TOPOLOGY:linear
CLONE:HUMGS00355
SEQUENCE DESCRIPTION:
GATCTTTGTT CTAGGCAGCT GGGAATAGAC ATGGTACTTA CCTTAGAGTT TTCCAATTTA 60
TCTCAATTTT ATATGGCTTG TGATTCATTT NCTTAATCCA AATATATATA ANCGTGTGTG 120
GTCTNATTCT NCCCCCCGCA ANANNAN 147

SEQ ID NO:333
SEQUENCE LENGTH:151
SEQUENCE TYPE:nucleic acid
TOPOLOGY:linear
CLONE:HUMGS00356
SEQUENCE DESCRIPTION:
GATCCTTACG GAAAAGGAAC AGATTGTNCC TAAACCAGAN GAGGAGGTTG CCCAGANGAA 60
AAAGATATCC CAGANGAAAC TGAAGAAACA AAAACTTATG GCACGGGAGT AAATTCAGCA 120
TTAAAATAAA TGTAATTAAA AGGAAAAGAA A 151

SEQ ID NO:334
SEQUENCE LENGTH:204
SEQUENCE TYPE:nucleic acid
TOPOLOGY:linear
CLONE:HUMGS00357
SEQUENCE DESCRIPTION:
GATCTTTGCA GTAATTTCTA GGAGCTGTTT ATGTTTGGAG GTAATTGGTC CTTTGTCCAT 60
ATATATGAGA TGTAAGTNTT ATTTTCCAGT TTATCTTTTT GCTTATTTTT TTTGACTTTT 120
TATTGTAAAA TAAAACATCA AACTGCACAG AACAGTTGAA TAGCTTAATG AATAACTACA 180
GTAAAAGCTA TGGTAACCAC TAAA 204

SEQ ID NO:335
SEQUENCE LENGTH:146
SEQUENCE TYPE:nucleic acid
TOPOLOGY:linear
CLONE:HUMGS00358
SEQUENCE DESCRIPTION:

GATCCAATTA ACATGTGGGG TTCTTGGTNT GGGTCTGGGG AGCTGAAGGA TTTNATGGAG 60
CTGGTGCTTT GGAGGAATCT TAAGGGAAAG NAGTAGAAGC TCAGGCCTTT AAAGGATTTC 120
ANCTCCTCCT CTCTGTAATT NNTNCN 146

SEQ ID NO:336
SEQUENCE LENGTH:146
SEQUENCE TYPE:nucleic acid
TOPOLOGY:linear
CLONE:HUMGS00359
SEQUENCE DESCRIPTION:
GATCGCATCT NTTAAACAGG TACAAGTTGA CATGAGGTTA GTTTAATTGT ACACCATGAT 60
ATTGGTGGTA TTTATGCTGT TAAGTCCAAA CCTTTATCTG TCTGTNATTC TTAATGTTGA 120
ATAANCTTTG ANTTTTTTCC TTTAAA 146

SEQ ID NO:337
SEQUENCE LENGTH:147
SEQUENCE TYPE:nucleic acid
TOPOLOGY:linear
CLONE:HUMGS00360
SEQUENCE DESCRIPTION:
GATCAGTGTT GAAGAAAGTG CAAAAGGAAC TTTTATATAT TTAACAGTGT AGGAAATTGT 60
CTATTCCTGA TATAATTACT GTAGTACTCT TGCTTAAGGC AAGNGTTTCA NATTTACNGT 120
TGAAATAAAC CCAACTCTTC NTGNAAA 147

SEQ ID NO:338
SEQUENCE LENGTH:147
SEQUENCE TYPE:nucleic acid
TOPOLOGY:linear
CLONE:HUMGS00361
SEQUENCE DESCRIPTION:
GATCTNGAAA ATNATCATTG AACATATTAA TGGTTATTTC TTTTTCTTGG ATTTCCAGAA 60
AAGCCTCTTA ATTTTATGCT TTCTCATCGA AGTAATGTAC CCTTTTTTTC TGAAACTGAA 120
TTAAATACTC ATTTNATCNN NTGNAAA 147

SEQ ID NO:339
SEQUENCE LENGTH:140
SEQUENCE TYPE:nucleic acid
TOPOLOGY:linear
CLONE:HUMGS00362
SEQUENCE DESCRIPTION:
GATCAGTTTT TTCACCTGGA AGCATTTGTT TCTACTTTGA TATGACTGTT TTTCGGACAG 60
TTTATTTGTT GAGAGTGTGA CCAAAAGTTA CATGTTTGCA CCTTTCTAGT TGAAAATAAA 120
GTGTATATTT TTCCTATAAA 140

SEQ ID NO:340

SEQUENCE LENGTH:144
SEQUENCE TYPE:nucleic acid
TOPOLOGY:linear
CLONE:HUMGS00363
SEQUENCE DESCRIPTION:
GATCTGAACT TTTCATCTGC AGAGGCAAGA AAAATATTTA ACATTGTGAC TTGACTGTGG 60
AAGATGATGG TTGCATGTTT CTAGTTTGTA TATGTTTCCA TCTTTGTAAT AAGATGATTT 120
AATAAATCTC TTTAAATACT TAAA 144

SEQ ID NO:341
SEQUENCE LENGTH:291
SEQUENCE TYPE:nucleic acid
TOPOLOGY:linear
CLONE:HUMGS00364
SEQUENCE DESCRIPTION:
GATCAAAAGC TTATTCATCT GTTTTNCTTT TTCGTTGGTG TAAAGCCAAC ACCCTGTCTA 60
AAAAACATAA ATTTCTTTAA TCATTTTGCC TCTTTTCTCT GTGCTTCAAT TAATAAAAAA 120
TGGAAAGAAT CTAATAGAGT GGTACAGCAC TGTTATTTTT CAAAGATGTG TTGCTATCCT 180
GAAAATTCTG TAGGTTCTGT GGAAGTTCCA GTGTTCTCTC TTATTCCACT TCGGTAGAGG 240
ATTTCTAGTT TCTTGTGGGC TAATTAAATA AATCATTAAT ACTCTTCTAA A 291

SEQ ID NO:342
SEQUENCE LENGTH:139
SEQUENCE TYPE:nucleic acid
TOPOLOGY:linear
CLONE:HUMGS00365
SEQUENCE DESCRIPTION:
GATCTTAAAC CTATGATTCA GTAACTTCTT ACCATATAAA AACGATAATT GCTTTATTTG 60
GAAAAGAATT TAGGAATACT AAGGACAATT ATTTTTATAG ACAAAGTAAA AAGACAGATA 120
TTTAAGAGGC ATAACCAAA 139

SEQ ID NO:343
SEQUENCE LENGTH:143
SEQUENCE TYPE:nucleic acid
TOPOLOGY:linear
CLONE:HUMGS00366
SEQUENCE DESCRIPTION:
GATCCGTGGC AGGGCTGCTG AGGCCTGTGG GTGGGACACC ANNTGCGAAA CCCTCATCCA 60
GTTTTCTCTC CATCTCTTTT CTTTGTACAA TCCCATTTCC TATTACCATT CTNTGCAATA 120
AACTCAAATC ACATGTCTGC AAA 143

SEQ ID NO:344
SEQUENCE LENGTH:139
SEQUENCE TYPE:nucleic acid
TOPOLOGY:linear

CLONE:HUMGS00367
SEQUENCE DESCRIPTION:
GATCCAGTGA CATTGTGAGT GAAGACGCAA ACAGGTTTTG ACTCCTGCAT GGCCGATGAC 60
CTTTTCTGTA GGCTTACCAG AAAAGTACAT NCAACAGTTC TTTGAGGTTT AACTAGAGCA 120
GCAAATAAAG CAAAAGTTN 139

SEQ ID NO:345
SEQUENCE LENGTH:140
SEQUENCE TYPE:nucleic acid
TOPOLOGY:linear
CLONE:HUMGS00368
SEQUENCE DESCRIPTION:
GATCAAGAAT CTTTTGTGAA ATTATAGAAA TTTACTATGT AAATGCTTGA TGGAATTTTT 60
TCCTGCTAGT GTAGCTTCTG AAAGGTGCTT TCTCCATTTA TTTAAAACTA CCCATGCAAT 120
TAAAAGGTAC AATGCAGAAA 140

SEQ ID NO:346
SEQUENCE LENGTH:142
SEQUENCE TYPE:nucleic acid
TOPOLOGY:linear
CLONE:HUMGS00369
SEQUENCE DESCRIPTION:
GATCCAGCTT GCCAGGGACT TAGGTTTATC CTGTTTTGTT TGCTACTGGT TACAAATTCT 60
ATTTTCTGTA CAATTAGTCA GACTAAAGTT TTCACTGTGT TTGTTTGGCA AAACAAATTA 120
AACAAAAAGT AAGGTTTTTA AA 142

SEQ ID NO:347
SEQUENCE LENGTH:156
SEQUENCE TYPE:nucleic acid
TOPOLOGY:linear
CLONE:HUMGS00370
SEQUENCE DESCRIPTION:
GATCTCCGTN AAACACATTT TTNTTCTTAG TCTATCTCTT GTACAAACGA TGTGCTTTGA 60
AGATGTTAGT GTATAACAAT TGATGTTTGT TTTCTNTTTG ATTTTAAACA GAGAAAAAAT 120
AAAAGGGGGT AATAGCTCCT TTTTTCTTCT TTCAAA 156

SEQ ID NO:348
SEQUENCE LENGTH:135
SEQUENCE TYPE:nucleic acid
TOPOLOGY:linear
CLONE:HUMGS00372
SEQUENCE DESCRIPTION:
GATCTACAAT NGGAGTTGTG AGTNGCAATC TTACATGGCT ACGNCTTTCG TTTGATAGCC 60
AGTCATGGTN ACCACATGAG AACCATATGC TGAGATGCAA TAAAGTAAGA GAATGTTTTC 120
TGACAAAAAA ATCTN 135

SEQ ID NO:349
SEQUENCE LENGTH:133
SEQUENCE TYPE:nucleic acid
TOPOLOGY:linear
CLONE:HUMGS00373
SEQUENCE DESCRIPTION:
GATCTTAAGC GTGTCTTGAG TTCCATGCAA ATTCAATTCT GTTGATAATG TGTCCATAAT 60
CAAATCATCA TCTTGCAATG CAAGGGCTAC CCCATAATTA TCAGACATTA AAATAGTTTA 120
TTTCTTTTTC AAA 133

SEQ ID NO:350
SEQUENCE LENGTH:137
SEQUENCE TYPE:nucleic acid
TOPOLOGY:linear
CLONE:HUMGS00374
SEQUENCE DESCRIPTION:
GATCTCGTTC CGCCGGTTCC CCTTGGCCGC CAGTTCCGTT CTCCTCACGG GCCGAACGGA 60
ACAAGGGGTC CAGCTTGCGG GGGACCCTCC CCAGCCCATT CCTGCTGTCA AACAAACAAA 120
ACCTTGCAAA GCGCAAA 137

SEQ ID NO:351
SEQUENCE LENGTH:132
SEQUENCE TYPE:nucleic acid
TOPOLOGY:linear
CLONE:HUMGS00375
SEQUENCE DESCRIPTION:
GATCAAGCGT GCTTTCCTTA TCCGAGGAGC AGAAAATCGT TGTGAAAGTG TTGAAGGCAC 60
AAGCACAGAG TCAGAAAGCT AAATAAAAAA ATGAAACTTT TTTGAGTAAT AAAAATGAAA 120
AGACGCTGTA AA 132

SEQ ID NO:352
SEQUENCE LENGTH:142
SEQUENCE TYPE:nucleic acid
TOPOLOGY:linear
CLONE:HUMGS00376
SEQUENCE DESCRIPTION:
GATCAAGTTC CCGCTGCCCC ACCGGGTNCT GCGCCGTCAG CACAAGCCAC GNTTCACCAC 60
CAAGAGGCCC AACACCTTCT TCTAGGTGCA GGGCCCTCTT CCGNGTTTTG CCCCAAATAA 120
ACTCANGAAC GNCCCGGTTA AA 142

SEQ ID NO:353
SEQUENCE LENGTH:142
SEQUENCE TYPE:nucleic acid
TOPOLOGY:linear

CLONE:HUMGS00377
SEQUENCE DESCRIPTION:
GATCAAGGAT ATTTGAAATC ACTACTGTGT TTTNCTGCGT ATCTGGGGCG GGGGCAGGTT 60
GGGGGGCACA AAGTTAACAT ATTCTTGGTT AACCATGGTT AAATATGCTA TTTTAATAAA 120
AATATTGAAA CTCACCAGTA AA 142

SEQ ID NO:354
SEQUENCE LENGTH:134
SEQUENCE TYPE:nucleic acid
TOPOLOGY:linear
CLONE:HUMGS00378
SEQUENCE DESCRIPTION:
GATCTCTAAT GAAAAAGGGA TGTCTTTTTG TTTATAGTCA TGTGGCAAGA TGAGAGTAAA 60
ACCAGAGAGC AAACCTCTAT AAGTNTTGAG TATATGTATA CATTTGAAAT AAACCAGAAA 120
TTTGTTACCT TAAA 134

SEQ ID NO:355
SEQUENCE LENGTH:132
SEQUENCE TYPE:nucleic acid
TOPOLOGY:linear
CLONE:HUMGS00379
SEQUENCE DESCRIPTION:
GATCAGCTCT GAGGTGCACT TCTTCACATA CTGTACATAC CTGTGACCAC TCTTGGGAGT 60
GCTGCAGTCT TTAATCATGC TGTTTAAACT GTTGTGGCAC AAGTTCTCTT GTCCAAATAA 120
AATTTATTAA TN 132

SEQ ID NO:356
SEQUENCE LENGTH:143
SEQUENCE TYPE:nucleic acid
TOPOLOGY:linear
CLONE:HUMGS00380
SEQUENCE DESCRIPTION:
GATCNGGGCT GGATTGACGG ATGTCACCCC CNATCCCCTC GTGACATGCA CGTCNGCAGG 60
AATGGGGGGT CTGCNGTGGT CGCCNGTCGT GTGAACAAGA TTCCGTCAAA ATATTTTCTG 120
TTAATAAATT GCCTTCATGT AAA 143

SEQ ID NO:357
SEQUENCE LENGTH:136
SEQUENCE TYPE:nucleic acid
TOPOLOGY:linear
CLONE:HUMGS00381
SEQUENCE DESCRIPTION:
GATCTAGAAG ATGATGTTCA AACTATGAAA CTGCTTGTGA ATTGTGAAAT GACTTTGTTC 60
TTTGCTTGTT TTTTTNAATT TCCTATAATG NACATACTAA CTTTTAAAAA ATAAAGGTTA 120
TTTTAAAAGC CTGAAA 136

SEQ ID NO:358
SEQUENCE LENGTH:133
SEQUENCE TYPE:nucleic acid
TOPOLOGY:linear
CLONE:HUMGS00382
SEQUENCE DESCRIPTION:
GATCTTCTAT GTAACAGTTG AAATNTGGAA GTGACGTCAC TTACCTGTCT AACGTGGTGT 60
GGGNGAGAAT TTACAAGTCC TTTATTGNAA GAATAATTGT TGCAAAATAT ATTGCTTCTA 120
CTTTGCCTGG AAA 133

SEQ ID NO:359
SEQUENCE LENGTH:130
SEQUENCE TYPE:nucleic acid
TOPOLOGY:linear
CLONE:HUMGS00383
SEQUENCE DESCRIPTION:
GATCAAGAAT TTGGGTGGGA GAAAAGAAAG TGGGTTATCA AGGGTGATNN GAAATTTTCT 60
GCAGCATTAA AGCTGGCGCT TAATAAGAAT AAGTAATAAT AAAGAAATTT CTAACATTCC 120
ATGTCAGAAA 130

SEQ ID NO:360
SEQUENCE LENGTH:157
SEQUENCE TYPE:nucleic acid
TOPOLOGY:linear
CLONE:HUMGS00384
SEQUENCE DESCRIPTION:
GATCTGAAAC TAATAGTAGG AGTTTCCCCA GAAGTCATTT TCAGCCTTAA TTCTCATCAT 60
GTATAAATTA CCATAAATNA TGCATGTNTG TTTACTTTAG TGACGTTCCA CAGAATAAAA 120
GGAAACAAGT TTGCCATCTT GGTGTTGCAA TATGAAA 157

SEQ ID NO:361
SEQUENCE LENGTH:132
SEQUENCE TYPE:nucleic acid
TOPOLOGY:linear
CLONE:HUMGS00385
SEQUENCE DESCRIPTION:
GATCTGAAAA GGCGTCTNCA CTGCTTTATC TCATGATGCT TGCTTGTAAA ACTTGATTTN 60
AGTTTTTCAT NNCTCAAATA GGAATACTAC CTTTGAATTC AATAAAATTC ACTGCAGGAT 120
AGACCAGTTA AA 132

SEQ ID NO:362
SEQUENCE LENGTH:197
SEQUENCE TYPE:nucleic acid
TOPOLOGY:linear

CLONE:HUMGS00386
SEQUENCE DESCRIPTION:
GATCTGCCAG GNTGGGTGGT TCTACTGCTT TCTCAATTTC TAAGAACCTT TTTTTTTTCT 60
NAAAGAGTTC TGCTGAATTA TTTGACAATA TTTGNAAGTA CCATGTTTCC TNGNGGGGTA 120
TGCTCTGTNC TGGTTTCTGT TTTNAAATCA AATGCCTGTT TGGGAGGAGA TGAACGNATT 180
NAGTCTATTA GATTTGN 197

SEQ ID NO:363
SEQUENCE LENGTH:128
SEQUENCE TYPE:nucleic acid
TOPOLOGY:linear
CLONE:HUMGS00387
SEQUENCE DESCRIPTION:
GATCACAGTG TCAGAGACGC GTCCTCTTTC TTGGGGAAGT TGAGGAGTGC CCTTCAGAGC 60
CAGTAGCAGG CAGGGGTGGG TAGGCACCCT CCTTCCTGTT TTTATCTAAT AAAATGCTAA 120
CCTGCAAA 128

SEQ ID NO:364
SEQUENCE LENGTH:127
SEQUENCE TYPE:nucleic acid
TOPOLOGY:linear
CLONE:HUMGS00388
SEQUENCE DESCRIPTION:
GATCCCAGGA GACACCAGGG CCAGAGTGAC CACAGCAGGG CAGGCATCAT CGTGTGTGTG 60
TGTGTGTGGA TGTGTGTGTG TGGGTTTTNT AAAGAATTCT TGACCAATAA AAGCAAAAAC 120
TGTCAAA 127

SEQ ID NO:365
SEQUENCE LENGTH:129
SEQUENCE TYPE:nucleic acid
TOPOLOGY:linear
CLONE:HUMGS00389
SEQUENCE DESCRIPTION:
GATCTACAAA TGGGAAGCTT GTGAGTGGCC CATCTTTGTT GGCCTACGAA CTTTGGTTTG 60
ATGCCAGTCA GGTGCCACAT GAGAACCTTT GCTGAGATGC AAATAAAGTA AGAGAATGTT 120
TTCCTGAAA 129

SEQ ID NO:366
SEQUENCE LENGTH:125
SEQUENCE TYPE:nucleic acid
TOPOLOGY:linear
CLONE:HUMGS00390
SEQUENCE DESCRIPTION:
GATCAGCTAT TAAATTTATA TAAAACATAG GCATGTTTGT ACTAATGAAA CGTACTGTCA 60
ACCTCTATCA CATTGTTAAA TTAACACTTT TGGTGGTAAC TCAATAAAAT TGAGAAAATT 120

GGAAA 125

SEQ ID NO:367
SEQUENCE LENGTH:246
SEQUENCE TYPE:nucleic acid
TOPOLOGY:linear
CLONE:HUMGS00391
SEQUENCE DESCRIPTION:
GATCTTTTTA ATTATAATTT TGTTGTATTT GTTTCCTAGG AGCAAGTGTT CCTGCTGCCA 60
GTTCTTTCCT CTTTAGGCGT GGTTGAGAAA AAGCAGAAAC TTTACATAAA GCTGTATTTC 120
TTAATCATCT TTAATTTGAA ACTTAAGNAA ATGAATTTAT TCTGTNATAT TTATGTAACT 180
NATTTCCTGG NAGTNATATC TACTAGTNTT GNTTGATAAT AATAAAATTN GGCTATACCT 240
TGNAAA 246

SEQ ID NO:368
SEQUENCE LENGTH:132
SEQUENCE TYPE:nucleic acid
TOPOLOGY:linear
CLONE:HUMGS00392
SEQUENCE DESCRIPTION:
GATCTAAAAT TTATTGTGGT GATGTTTGCA TAACAGTGCA AATATACTGA AAACCACTGA 60
ATTTTACACT TTAAATCAGT GGCTTCTGTG GTATGTTATC AATATTTCTC AATAAAACTT 120
CAAAAAAATA AA 132

SEQ ID NO:369
SEQUENCE LENGTH:131
SEQUENCE TYPE:nucleic acid
TOPOLOGY:linear
CLONE:HUMGS00393
SEQUENCE DESCRIPTION:
GATCTGGTAT TAGGAAATTA CTTTCACAGT AAATATCAAA GAAAAAAGAT TAAGGGTCTC 60
TTTGCCATGC TTTTCATCAT ATGCACCAAA TGTAAATTTT GTACAATAAA ATTTTATTTC 120
CTAAGTAGAA A 131

SEQ ID NO:370
SEQUENCE LENGTH:123
SEQUENCE TYPE:nucleic acid
TOPOLOGY:linear
CLONE:HUMGS00394
SEQUENCE DESCRIPTION:
GATCTGAAAA AGAACACTGT GCGAGATTGT ATTCCTGCTT ATCCTTTCCA AGTTAGTAGG 60
CAGATTGGAA CTATAGAATT TATCCGTGAT AATAATAAAG TCCGCATAAC TTTTGTCCTG 120
AAA 123

SEQ ID NO:371

SEQUENCE LENGTH:123
SEQUENCE TYPE:nucleic acid
TOPOLOGY:linear
CLONE:HUMGS00395
SEQUENCE DESCRIPTION:
GATCTAAAGA GAAACTGTAG ATTGTTTTCC TGACAGCAAA AGACTAATGT GACAAAATGA 60
AGTCATTGTA AAGAAGCGAT GCAACTTGTC AAATATTTAA TAAAGAATTA TGGAAGCTGG 120
AAA 123

SEQ ID NO:372
SEQUENCE LENGTH:124
SEQUENCE TYPE:nucleic acid
TOPOLOGY:linear
CLONE:HUMGS00396
SEQUENCE DESCRIPTION:
GATCACAACT GNAAGATAAC AAGAGATTTA AGTTTTAAGG GCATTTAATC AGGAGGAAAG 60
GTTTGGAAAA CTAACTCAGG TGTATTTNTT GTTTAAGCAG AAATAAAGTT TAATTTTTNC 120
TTGN 124

SEQ ID NO:373
SEQUENCE LENGTH:122
SEQUENCE TYPE:nucleic acid
TOPOLOGY:linear
CLONE:HUMGS00397
SEQUENCE DESCRIPTION:
GATCTGTGTT AGAATGAGTG CTTTCCCTTC CTACTGATGT GATTGTGGAT TAGGAATTCG 60
TGACCGAGTG ATTTTTGGCC AGTGGTTGGG TTTAAAATTC TATTAAAATT TGTAGTTTGG 120
GN 122

SEQ ID NO:374
SEQUENCE LENGTH:121
SEQUENCE TYPE:nucleic acid
TOPOLOGY:linear
CLONE:HUMGS00398
SEQUENCE DESCRIPTION:
GATCGCCGCC CTGCTGGCCA CCTGCGNTGG NGCTGGCNCT CGTGGTCGTC GCGCTGAGAA 60
AGTTTTCTCC CTCCTGAAGC GAATAAAGGG GCCGCNGCCG GCCGCGGCGC GACTCGGCAA 120
A 121

SEQ ID NO:375
SEQUENCE LENGTH:120
SEQUENCE TYPE:nucleic acid
TOPOLOGY:linear
CLONE:HUMGS00399
SEQUENCE DESCRIPTION:

GATCAGGCCC GGTGCCTGCA GACCTGGTGC TCCCTCGGGC AGGGCTGGGT GCCGCACCGC 60
CTGCTGGCTT TTCTGGCAGC TCCTCTGTAT CAGAACCAAT AAAGTGCACT TGTTCTCGGN 120

SEQ ID NO:376
SEQUENCE LENGTH:119
SEQUENCE TYPE:nucleic acid
TOPOLOGY:linear
CLONE:HUMGS00400
SEQUENCE DESCRIPTION:
GATCAGGCAG TCCTTNAGGA TAGACAGATA TACACACCAC ACACACACAC CACATACACC 60
ACACACACAC GTCCCCATCC ACTNACCCAC ACACTACACA GNCTGNTNCC TTATAGCTN 119

SEQ ID NO:377
SEQUENCE LENGTH:225
SEQUENCE TYPE:nucleic acid
TOPOLOGY:linear
CLONE:HUMGS00401
SEQUENCE DESCRIPTION:
GATCTNATTT GTAACCCACT GAGAGGACAG AGAGAAATAA GTGCCCTCTC CCACCCTCTN 60
CCTACTGGTC TCTCTATGCC TCTCTACAGT CTCGTCTCTT NTACCCTGGC CCCTCTCCCT 120
TGGGCTGTGA TGAAAAATTG CTGACTGTAG CTTTTGGGAG TTTAGCTCTG AGAACCGTAG 180
ATGGATTNCA GTTCTGGGAA AATAAAACCC GTTGATTACT NNAAA 225

SEQ ID NO:378
SEQUENCE LENGTH:125
SEQUENCE TYPE:nucleic acid
TOPOLOGY:linear
CLONE:HUMGS00402
SEQUENCE DESCRIPTION:
GATCTTAATA TATTTGAAAA AAACTTCATT CTCGTGAGTC ATTTAAATGT GTACAATGTA 60
CACACTGGTA CTTAGAGTTT CNGTTTGATT CTTTTTTAAT AAACTACTCT TTGATTTAAA 120
GCAAA 125

SEQ ID NO:379
SEQUENCE LENGTH:147
SEQUENCE TYPE:nucleic acid
TOPOLOGY:linear
CLONE:HUMGS00403
SEQUENCE DESCRIPTION:
GATCTCAGTT CTGCGTTTAT TGTAAGTTGA TAAAAACATC TGGAAGAAAA TAACTAAAAC 60
TGTTTGCATC TTTGTATGTA TTTATTACTT GATGTAATAA AGCTTATTTT CATTAACAAT 120
TTGTATTAAA ATNTGGGTTC CTTGAAA 147

SEQ ID NO:380
SEQUENCE LENGTH:116

SEQUENCE TYPE:nucleic acid
TOPOLOGY:linear
CLONE:HUMGS00404
SEQUENCE DESCRIPTION:
GATCACCTTT TCAGAAATTT AGATGTGAAC ACCAAAAGAA GCATTTTCTC AACAAAAATT 60
AATAGCTGGT TCTATTTTTT TTAAACCTAG AAAAAATAAA GTTGATTTTT TTCAAA 116

SEQ ID NO:381
SEQUENCE LENGTH:119
SEQUENCE TYPE:nucleic acid
TOPOLOGY:linear
CLONE:HUMGS00405
SEQUENCE DESCRIPTION:
GATCAATAGG GAGAGAAAAT CCACATTCTT GGGCTGAACG CGGGCCTCTG ACACTGCTTA 60
CACTGCACTC TGACCCTGTA GTACAGCAAT AACCGTCTAA TAAAGAGCCT ACCCCCAAA 119

SEQ ID NO:382
SEQUENCE LENGTH:115
SEQUENCE TYPE:nucleic acid
TOPOLOGY:linear
CLONE:HUMGS00406
SEQUENCE DESCRIPTION:
GATCTTTATT ATGGAAANCA TTTCAAGTTT ACTCCTTCTG TTTTAAGTTT TGTAGCAGTG 60
TACCCACGCT GGGTATTACN NCCNAAATAA TCTGTNAGTG AAAGTTGCCA TTATN 115

SEQ ID NO:383
SEQUENCE LENGTH:115
SEQUENCE TYPE:nucleic acid
TOPOLOGY:linear
CLONE:HUMGS00407
SEQUENCE DESCRIPTION:
GATCGANCTG CGCAANTGNG NAAGCTGCAG AGGACATCGC GTACCANCTC TCACGCTCTC 60
GGAACATCAC CTACCTGCCA GCGGGGCAGT CCGTGCTCCT CCAGCTGCCC CAGTN 115

SEQ ID NO:384
SEQUENCE LENGTH:113
SEQUENCE TYPE:nucleic acid
TOPOLOGY:linear
CLONE:HUMGS00408
SEQUENCE DESCRIPTION:
GATCTCTCAA AAAACAAAGA ATTACATGAG TTAGTACATG AAAAAATTAT GGGAAACTAC 60
ATGAAATATA CTGTTACGTT CAATAAACAT TAGCTTCTGT ATATAATANT AAA 113

SEQ ID NO:385
SEQUENCE LENGTH:116

SEQUENCE TYPE:nucleic acid
TOPOLOGY:linear
CLONE:HUMGS00409
SEQUENCE DESCRIPTION:
GATCTCTTCC CCCAACTTCC TAACACTTAT TAATTTATGA AACTGTTTTT CTCAGCGCAG 60
TTTTGTTTTG TGTGTCCATT GGATTACAAA CTTTATTAAA AAATATAAAA CACAAA 116

SEQ ID NO:386
SEQUENCE LENGTH:118
SEQUENCE TYPE:nucleic acid
TOPOLOGY:linear
CLONE:HUMGS00410
SEQUENCE DESCRIPTION:
GATCTCTCTC TTCTCGCGCG CGCACTCTCT CTTCAACACT CCCCTGCGTA CCCCGGTTCT 60
AGCAAACACC AATTGATTGA CTGAGAATCT GATAAAGCAA CAAAAGATTT GTCCCAAA 118

SEQ ID NO:387
SEQUENCE LENGTH:247
SEQUENCE TYPE:nucleic acid
TOPOLOGY:linear
CLONE:HUMGS00411
SEQUENCE DESCRIPTION:
GATCTATAAA AAGTCAGCAA CTGATGTGTT TGAAAAGCAT CCTTGTCTNT ATATCCTAAT 60
GTTTGGATGT GTCTTTNCTA AAGTCTCACA AAAATTAGTG GTAGCTCACA TGACCAAAAG 120
TGAACTATAT CTNCAAGACA CTGTCTNNGG GGGGCCAGGT CTTTTGTTTT TAGGNCCAGT 180
ACTTNNATAA TTTTNTAGAC GGATATGGTT GTCCTATGGA TGGCAATGGG TGNTTNCTCC 240
ATTTGNN 247

SEQ ID NO:388
SEQUENCE LENGTH:112
SEQUENCE TYPE:nucleic acid
TOPOLOGY:linear
CLONE:HUMGS00412
SEQUENCE DESCRIPTION:
GATCCTAGGA AGAGAGAACA GAGTGGCTCA CAAGCCCCAA CACAGTNAGC AGCAGATGAC 60
AGGCACNCTN AGACCACACT NTAGGCCACC CATGGGNCCA AAAGGGAACA GN 112

SEQ ID NO:389
SEQUENCE LENGTH:111
SEQUENCE TYPE:nucleic acid
TOPOLOGY:linear
CLONE:HUMGS00413
SEQUENCE DESCRIPTION:
GATCACCACN TAAGTCAGAA AAATGTATTT TTAAATGTTT CTTGAAGTGC CTTTTGAACA 60
TTTTTAAACA GCGGATTTAA ATAATGCATA AANTAAATTG CCATGNTCAA A 111

SEQ ID NO:390
SEQUENCE LENGTH:109
SEQUENCE TYPE:nucleic acid
TOPOLOGY:linear
CLONE:HUMGS00414
SEQUENCE DESCRIPTION:
GATCACCTTA GTTTGATTCT ATTTTTTAGC TTGCAAAAAG TGACTTATAT TCCAAAGAAA 60
TTAAAATGTT GAAATCCAAA TCCTAGAAAT AAAATGAGTT AACTTCAAA 109

SEQ ID NO:391
SEQUENCE LENGTH:111
SEQUENCE TYPE:nucleic acid
TOPOLOGY:linear
CLONE:HUMGS00415
SEQUENCE DESCRIPTION:
GATCGTNACG CTCGCATCTA TAGATAACGG CTCTCCAGAC CTGAGCTTTC CGCGTCANAA 60
TGTAGGAATN GTTTTTCCTG CAGAGAATAA AAGGACCACG TGNAATACTT N 111

SEQ ID NO:392
SEQUENCE LENGTH:109
SEQUENCE TYPE:nucleic acid
TOPOLOGY:linear
CLONE:HUMGS00416
SEQUENCE DESCRIPTION:
GATCTTATTG AAGGACATCT TACAGCTTCC CAATGAGAGG CCAGGAAGTG TGAACATACT 60
GATAGAAAAA GACTATATTT TATCCCTCAT AAAATGTTTT AAATGTAAA 109

SEQ ID NO:393
SEQUENCE LENGTH:116
SEQUENCE TYPE:nucleic acid
TOPOLOGY:linear
CLONE:HUMGS00417
SEQUENCE DESCRIPTION:
GATCACTGAG TGTACAGAAG AGAGAAATTC AAACAAAATA TTGCTGTTCT TCAGTTTTGT 60
TTGTGGAATT TAAAATNACT CAAATTTAAA ATAAATNACT GGACTGTGGA AATAAA 116

SEQ ID NO:394
SEQUENCE LENGTH:115
SEQUENCE TYPE:nucleic acid
TOPOLOGY:linear
CLONE:HUMGS00418
SEQUENCE DESCRIPTION:
GATCTCAAGA GTTCACCTGG CTNACAGAAA GAAGATGCCA GATGACACTT AAGACCTACT 60
TGTGATATTT AAATGATGCA ATAAAAGACC TATTGATTTG GACCTTCTTC TTAAA 115

SEQ ID NO:395
SEQUENCE LENGTH:114
SEQUENCE TYPE:nucleic acid
TOPOLOGY:linear
CLONE:HUMGS00419
SEQUENCE DESCRIPTION:
GATCATTCTG AACTGTACAT ATTTATGTNG CGAGAGGCAA AGGGCAAGTT TTGGATTTTC 60
CTTCTTCCAA GTTTGTTTTT AAACGACAAA TAAAAAAAGA ACATTTTAAA TAAA 114

SEQ ID NO:396
SEQUENCE LENGTH:106
SEQUENCE TYPE:nucleic acid
TOPOLOGY:linear
CLONE:HUMGS00420
SEQUENCE DESCRIPTION:
GATCTGAATC TNTGACTTAT TGATTATGGA ACCTGTCAAG TAGTTTTNAA CTCTCCCAGT 60
GAGGATAATT AAACATGCTC AGCCTGAGCC ACCTCTAAGT NTCAAA 106

SEQ ID NO:397
SEQUENCE LENGTH:107
SEQUENCE TYPE:nucleic acid
TOPOLOGY:linear
CLONE:HUMGS00421
SEQUENCE DESCRIPTION:
GATCGTTCTT CATGGGGGTA AGAAAAGCTG GTCTGGAGTT GCTGAATGTT GCATTAATTG 60
TGCTGTTTGC TTGTAGTTGA ATAAAAATAG AAACCTGAAT GAAGAAA 107

SEQ ID NO:398
SEQUENCE LENGTH:112
SEQUENCE TYPE:nucleic acid
TOPOLOGY:linear
CLONE:HUMGS00422
SEQUENCE DESCRIPTION:
GATCCCCTCA AAAGGCAGGA NTGCTGCCCT CTNCCATGGT GCCCGTNCCT CTTTGCTGTN 60
TATGTNAACC ACCCATGTAA GGGAATAAAC CTGGCACTAG GTCTTAAATA AA 112

SEQ ID NO:399
SEQUENCE LENGTH:105
SEQUENCE TYPE:nucleic acid
TOPOLOGY:linear
CLONE:HUMGS00423
SEQUENCE DESCRIPTION:
GATCANCTCT AAGGTGCAAC TNCCTCCACA TACTGTACAT ACCTGTNACC ACTCTTGGAA 60
GTCCTGCAGT CTTTAATCAT NCTGTTTAAN CTGTTGTGGC ACAAN 105

SEQ ID NO:400
SEQUENCE LENGTH:104
SEQUENCE TYPE:nucleic acid
TOPOLOGY:linear
CLONE:HUMGS00424
SEQUENCE DESCRIPTION:
GATCTGGAAC TTGAAGATGC CATTCATACA GCCACTTAAC CTAAAGGGAA AGCTTTGAAG 60
GGCAAATGAC AGAGGGTAAC ATAGGAGGTN GGATNCTNAA TNNN 104

SEQ ID NO:401
SEQUENCE LENGTH:104
SEQUENCE TYPE:nucleic acid
TOPOLOGY:linear
CLONE:HUMGS00425
SEQUENCE DESCRIPTION:
GATCTTTGAT ATATCATAGT CATTAAAAGA CNTTTTCGTA TTTGTATTGA TAATGTATTA 60
AAAGTNGTTT GTNCTTAATA AAAGACTTCT TTAANCATCT NAAA 104

SEQ ID NO:402
SEQUENCE LENGTH:109
SEQUENCE TYPE:nucleic acid
TOPOLOGY:linear
CLONE:HUMGS00426
SEQUENCE DESCRIPTION:
GATCCCCGGC CTCAGTCCCT ACTCTGCTTT GGGATAGTGT GAGCTTCATT TTGTACACGT 60
GTGACTTCGT CCAGTTACAA ACCCAATAAA CTCTGTAGAG TGGAACAAA 109

SEQ ID NO:403
SEQUENCE LENGTH:110
SEQUENCE TYPE:nucleic acid
TOPOLOGY:linear
CLONE:HUMGS00427
SEQUENCE DESCRIPTION:
GATCACCTGC AGCTGGCCAC ACCACAGGCC CCCGNTGCCT GCAGCACTAC TCNGTNCCTN 60
AAACACCTGG CCTGCTAGGA GGCTCCAATA AAGCTAACCC GGACCAGAAA 110

SEQ ID NO:404
SEQUENCE LENGTH:157
SEQUENCE TYPE:nucleic acid
TOPOLOGY:linear
CLONE:HUMGS00428
SEQUENCE DESCRIPTION:
GATCCTCAGA ACTTCTCTGG GACAATTTCA GTTCTAATAA TGTCCTTAAA TTTTATTTCC 60
AGCTCCTGTT CCTTGGAAAA TNTCCATTGT ATGTGCATTT TTTAAATGAT GTCTGTACAT 120

AAAGGCAGTT CTGAAATAAA GAAAATTTTA AAATAAA 157

SEQ ID NO:405
SEQUENCE LENGTH:103
SEQUENCE TYPE:nucleic acid
TOPOLOGY:linear
CLONE:HUMGS00429
SEQUENCE DESCRIPTION:
GATCAGCAAC ATTTGCTGAG CCTGTTTTTN AAGCTAATGT GTATTCTNAC TAATNTNCCT 60
ATCAAGAATG GATTTGTAAT ATATNCTGTC TATTTCTAAT GTN 103

SEQ ID NO:406
SEQUENCE LENGTH:105
SEQUENCE TYPE:nucleic acid
TOPOLOGY:linear
CLONE:HUMGS00430
SEQUENCE DESCRIPTION:
GATCTGTAAG CACAGTCTTA TTTNCTTTTG TTGTCCAGAA TACTTATAAT TCTTGAGCCT 60
CCCAGAAATT GGAAGCTAAA TAAAGCAACT CAAGTTTCCT TTAAA 105

SEQ ID NO:407
SEQUENCE LENGTH:104
SEQUENCE TYPE:nucleic acid
TOPOLOGY:linear
CLONE:HUMGS00431
SEQUENCE DESCRIPTION:
GATCAGCATC ATTGGAACAT GGGGACGAGT GACGGCAGGA GGACCACGAG GAAATACCCT 60
CAAAACTAAC TTGTTTACAA CAAAATAAAG TATTCACTAC CAAA 104

SEQ ID NO:408
SEQUENCE LENGTH:105
SEQUENCE TYPE:nucleic acid
TOPOLOGY:linear
CLONE:HUMGS00433
SEQUENCE DESCRIPTION:
GATCTATCAC TCTCGTNCTT GTAGCTCCCA GCCGAGGACG TCGGATGTAA TCGTCCTTNC 60
TGCTTTGCCA CCCCATTCCC GTCAATAAAG TGGTTTGAAC CCAAA 105

SEQ ID NO:409
SEQUENCE LENGTH:105
SEQUENCE TYPE:nucleic acid
TOPOLOGY:linear
CLONE:HUMGS00434
SEQUENCE DESCRIPTION:
GATCAAACCT TTCTGGCCTG TTATGATTCT NAACATTTGA CTTGAACCAC AAGTGAATCT 60

TTCTCCTGGT GACTCAAATA AAAGTATAAT TTTNACCTGC GGAAA 105

SEQ ID NO:410
SEQUENCE LENGTH:101
SEQUENCE TYPE:nucleic acid
TOPOLOGY:linear
CLONE:HUMGS00435
SEQUENCE DESCRIPTION:
GATCAACACA AAGCACAATG NATTACNCGN AATTCAGTAT TTTCAAATTT ACATATTTAA 60
AGTCATGCAA GCTGTAACTT CCCNGTCAAA ATTACTNGCT N 101

SEQ ID NO:411
SEQUENCE LENGTH:100
SEQUENCE TYPE:nucleic acid
TOPOLOGY:linear
CLONE:HUMGS00436
SEQUENCE DESCRIPTION:
GATCATAAAG NNCTATCAAG GAGTTCTATC AAGGCATCCA TGTCAGTGGT GCTATGCTGG 60
TTACAACTTG AGATTTTTGA AATAAAAAAT TTGTCATAAA 100

SEQ ID NO:412
SEQUENCE LENGTH:103
SEQUENCE TYPE:nucleic acid
TOPOLOGY:linear
CLONE:HUMGS00437
SEQUENCE DESCRIPTION:
GATCAACAGG CTTATTAGAA GAATGAACTA AGGTGTCTAC CATGATTATN TTTCTAAGCT 60
GGTTGGTTAA TAAACAGTAC CTGCTCTCAA ATTGAAAAAG AAA 103

SEQ ID NO:413
SEQUENCE LENGTH:99
SEQUENCE TYPE:nucleic acid
TOPOLOGY:linear
CLONE:HUMGS00438
SEQUENCE DESCRIPTION:
GATCTTGTGC TGTGTCAAAG TAACAGACTA GAACCTTCTT TCAAGTACCT GAATTGAAAT 60
NAAACTCATT TTGAATAATA AAAACTCTAG AAACTCAAA 99

SEQ ID NO:414
SEQUENCE LENGTH:99
SEQUENCE TYPE:nucleic acid
TOPOLOGY:linear
CLONE:HUMGS00439
SEQUENCE DESCRIPTION:
GATCTGTAAT AGCATATTGT AGATGCACTT TGCAGCAGTT GGAAAAGAAA GTGTTGTGTG 60

ATTTGATTGA AATAAAACTA AATGTGTTGT CCTCCTAAA 99

SEQ ID NO:415
SEQUENCE LENGTH:96
SEQUENCE TYPE:nucleic acid
TOPOLOGY:linear
CLONE:HUMGS00440
SEQUENCE DESCRIPTION:
GATCCCTGTG CCAGGAGCCA ACCTGGTCTT CCCGAGGGTC AGTGCCCCAG TGAAGACAGA 60
AGCGAGAGAA TAAAGTTCCC TGTAGGTCCT CTGTCN 96

SEQ ID NO:416
SEQUENCE LENGTH:97
SEQUENCE TYPE:nucleic acid
TOPOLOGY:linear
CLONE:HUMGS00441
SEQUENCE DESCRIPTION:
GATCCTCCCA TCCGTGTTGT GAGCACAGGC ATTTGTGTNT GGNCTGTCCT CCCTGTTGAT 60
TGGTCTGGCA TTTCCGGTAT TAAAATGATA ANATAAA 97

SEQ ID NO:417
SEQUENCE LENGTH:93
SEQUENCE TYPE:nucleic acid
TOPOLOGY:linear
CLONE:HUMGS00442
SEQUENCE DESCRIPTION:
GATCCTGCAT ATCTCAAGGA CCCTAAAGTT TGTAACATCA GATATCGGGA ATAAATTCTA 60
TCACGTTACC ACTAATAAAC TTATTTTACA GTN 93

SEQ ID NO:418
SEQUENCE LENGTH:97
SEQUENCE TYPE:nucleic acid
TOPOLOGY:linear
CLONE:HUMGS00443
SEQUENCE DESCRIPTION:
GATCTGTATT TTGCAAATAT TTTCTTCAAT ATGTGGCTTG TCTTTTGGTT CTCTTAACAA 60
GGTCTCTTCC AGAGTATAAN CTGTAAATAT TAAGAAA 97

SEQ ID NO:419
SEQUENCE LENGTH:98
SEQUENCE TYPE:nucleic acid
TOPOLOGY:linear
CLONE:HUMGS00444
SEQUENCE DESCRIPTION:
GATCTGGACA GAATCGCCGG ACAGGTGGCA GCTGCAACAA GAAGCATTAG AACAAACCAT 60

GCTGGGTTAA TAAATTGCCT CATTCGTAAT CCTGGAAA 98

SEQ ID NO:420
SEQUENCE LENGTH:100
SEQUENCE TYPE:nucleic acid
TOPOLOGY:linear
CLONE:HUMGS00445
SEQUENCE DESCRIPTION:
GATCTATTCT GAGTATTTTT TAGAGAGTTA ATATTTATAT TTTTAGTAAT TTTCTGGTAG 60
AAGGAAATTG CACAATAAAA TNATTTGGTT TGGTTTGAAA 100

SEQ ID NO:421
SEQUENCE LENGTH:93
SEQUENCE TYPE:nucleic acid
TOPOLOGY:linear
CLONE:HUMGS00446
SEQUENCE DESCRIPTION:
GATCGTTGGC ACCATAGCCT TATGGCCAAC AGGTGGTNTG TGGTGAAAGG GGCGTGGAGT 60
TTCAATATCA ATAAACCACC TGATATCAAT AAA 93

SEQ ID NO:422
SEQUENCE LENGTH:94
SEQUENCE TYPE:nucleic acid
TOPOLOGY:linear
CLONE:HUMGS00447
SEQUENCE DESCRIPTION:
GATCTGTGTT TNCCCTGACG AATGGAATTT ATCCTCACAA ATTGGTGTTC TAAATGTNTT 60
AAGAACCTAA TTAAATAGCT GACTACAAAA CAAA 94

SEQ ID NO:423
SEQUENCE LENGTH:206
SEQUENCE TYPE:nucleic acid
TOPOLOGY:linear
CLONE:HUMGS00448
SEQUENCE DESCRIPTION:
GATCCCAAAC CTTACGGCCA AGTTTCTTCT AGTATGATGG AAAGTTTCTT TTTTCTTTGC 60
TCTGAATAAA ACTGAACTGT GGGTTCTCTA TAAGTGGCAT TTTGGGCTTT CCCTCTTTTT 120
TGTAAAGCAA TGTCTGCCTA GTTTATTGTC CAGTTAACTT TAGTGACCTT TTAAAAGTTG 180
GCATTGTAAA TAAAACAACT TGCAAA 206

SEQ ID NO:424
SEQUENCE LENGTH:481
SEQUENCE TYPE:nucleic acid
TOPOLOGY:linear
CLONE:HUMGS00450

SEQUENCE DESCRIPTION:
GATCTCACCA CTGCACTCCA GCCTGGGCAA CAGAGCGAGA CTCTGTCTCA ACAACAACAA 60
CAAAAAGTCC TGAACATGAT TGTGGAAGTG TGTTGCTCTT TCAAGTTCTA TCACTTTTTG 120
TTTGCAAAGT TCAAAGCTGT ATTGTTTGGT ACATATACAT GTAGGTTTGC CAAGTCTTTG 180
TGGTGAATTG ACTCTTCTGT CATTATGTGA TGTCATTTTT TTGCCTTTTA ATAGTCTTGT 240
CAATACTTTA CCTGATGTTC TCATAGTGAC TCCTGCATAT TTTGATTAAT GTTTGCATGG 300
TTAATATTTC TTCATTTTAT TTTAAAGCTT ACCTGTATCA TTACTTATGA AGTCAGTTTC 360
TTTGAACAGC ATATACTCAG GCCATGCTTT TTTTNATTCA TTCCTGCATA TGGCTCTCCT 420
TAAATTGGGA ATGGTGGAAA ATGGNTTTAC CATTAANAAT AAATTAATTG GTATTTTTAA 480
A 481

SEQ ID NO:425
SEQUENCE LENGTH:87
SEQUENCE TYPE:nucleic acid
TOPOLOGY:linear
CLONE:HUMGS00451
SEQUENCE DESCRIPTION:
GATCTGGTCC ATGAGGCTGC CCAGAGAAAG CACTGCTTCT NTATGTCTCT TGTGGTATTG 60
GAACAATAAA CCCGTACAAC CTGCAAA 87

SEQ ID NO:426
SEQUENCE LENGTH:93
SEQUENCE TYPE:nucleic acid
TOPOLOGY:linear
CLONE:HUMGS00453
SEQUENCE DESCRIPTION:
GATCATACTT GAAAGTGAAC TTTAACATTG AAAAATCGTA CAGTCATTTC AAGAATAAGA 60
AAATAAAATT TTCTCTTTGT CTGAACCTGC AAA 93

SEQ ID NO:427
SEQUENCE LENGTH:85
SEQUENCE TYPE:nucleic acid
TOPOLOGY:linear
CLONE:HUMGS00454
SEQUENCE DESCRIPTION:
GATCCCATGT GCTCTCACAC CATGTTTTTG TACAGAACTG ATGGTTGAAT CTTTGTTCTC 60
TTGAAATAAA CAGAAGAAAA TGAAA 85

SEQ ID NO:428
SEQUENCE LENGTH:87
SEQUENCE TYPE:nucleic acid
TOPOLOGY:linear
CLONE:HUMGS00455
SEQUENCE DESCRIPTION:
GATCTATTAA AGAAGTAATT GGCCTTTCTG AGCTGATTTT TCCATCTTTT GTAATTATCT 60

TTATTAAAAA ATTGTACTTG GATTAAA 87

SEQ ID NO:429
SEQUENCE LENGTH:91
SEQUENCE TYPE:nucleic acid
TOPOLOGY:linear
CLONE:HUMGS00456
SEQUENCE DESCRIPTION:
GATCTCATTC CATGGGAAAA AAAAAAATCC TGTCTTNTTC ANAAATTGAC AATGTAAATA 60
AATTNAAATA TGGTTCACTG TTACTCTTAA A 91

SEQ ID NO:430
SEQUENCE LENGTH:84
SEQUENCE TYPE:nucleic acid
TOPOLOGY:linear
CLONE:HUMGS00457
SEQUENCE DESCRIPTION:
GATCTGAGAA ACAGGTGTGA CAAGAGCATG AACCANAGGT GCACCTGGGG CAGTTCCCTA 60
ATAAAACTGG TTTGTACAGT CAAA 84

SEQ ID NO:431
SEQUENCE LENGTH:83
SEQUENCE TYPE:nucleic acid
TOPOLOGY:linear
CLONE:HUMGS00458
SEQUENCE DESCRIPTION:
GATCATGAGA GTGCCTGTCC CTTGTGAGCA CTATGAAAGT GTTAGCTGTT CTTTACCAGA 60
ATAAATGCAT TTCTATATCT TCN 83

SEQ ID NO:432
SEQUENCE LENGTH:84
SEQUENCE TYPE:nucleic acid
TOPOLOGY:linear
CLONE:HUMGS00459
SEQUENCE DESCRIPTION:
GATCACCGAC TGAAAATATT GTTTTACAAT AGTTCTGTGG GGCTGTTTTT TTGTTATNAA 60
ACAAATAATT TAGATGGTGG TAAA 84

SEQ ID NO:433
SEQUENCE LENGTH:80
SEQUENCE TYPE:nucleic acid
TOPOLOGY:linear
CLONE:HUMGS00460
SEQUENCE DESCRIPTION:
GATCTTGATG GATTTNCATA CGATTGTAAA TGNAGCTATA TTAAAGTCTA TTAAAGGAAG 60

CCCTTCTTGT TTGAGGGAGN 80

SEQ ID NO:434
SEQUENCE LENGTH:86
SEQUENCE TYPE:nucleic acid
TOPOLOGY:linear
CLONE:HUMGS00461
SEQUENCE DESCRIPTION:
GATCTATGCT TGTTTGTTTT TGTAATCCAT ATCATAGTTG CTTTCTTTAA TTGTTCCTTC 60
TGAATAAACA GTTTATTTAA GATAAA 86

SEQ ID NO:435
SEQUENCE LENGTH:83
SEQUENCE TYPE:nucleic acid
TOPOLOGY:linear
CLONE:HUMGS00462
SEQUENCE DESCRIPTION:
GATCCAGTCA CTGACTCTGT CTGGTGTTGA CAGAGGATTT ATTTAAGCTA TTATTTTAAT 60
AAAGNACTTT GTACATTTTT AAA 83

SEQ ID NO:436
SEQUENCE LENGTH:85
SEQUENCE TYPE:nucleic acid
TOPOLOGY:linear
CLONE:HUMGS00463
SEQUENCE DESCRIPTION:
GATCTACATA CAAACAAATG CAACCAACTA TCCAAGTCGT TATACCAACG TAAAACCCCC 60
AATAAACCGT TGAACATGTG ACAAA 85

SEQ ID NO:437
SEQUENCE LENGTH:86
SEQUENCE TYPE:nucleic acid
TOPOLOGY:linear
CLONE:HUMGS00464
SEQUENCE DESCRIPTION:
GATCTGCTTT TACTTTGTAA TTTGTAGTTC TCAAAAGACT TTTTTTTAAA AAAATAAAGN 60
CCATACTTAC ACTTAGGCTT TATAAA 86

SEQ ID NO:438
SEQUENCE LENGTH:83
SEQUENCE TYPE:nucleic acid
TOPOLOGY:linear
CLONE:HUMGS00465
SEQUENCE DESCRIPTION:
GATCATTCTG AGTGTGCGAG TGTGTGTGCA CATGTTACAA AGGCANCTGC CATGTTAATA 60

AAATATTCAA TTTGAAATCC AAA 83

SEQ ID NO:439
SEQUENCE LENGTH:78
SEQUENCE TYPE:nucleic acid
TOPOLOGY:linear
CLONE:HUMGS00466
SEQUENCE DESCRIPTION:
GATCCAAACT GTCTTTTTTT TGTATCTGTT ATTTAAAGCC CAGTGGATAT TTCAATNAAA 60
AAAAAAATCT AAAGATGN 78

SEQ ID NO:440
SEQUENCE LENGTH:80
SEQUENCE TYPE:nucleic acid
TOPOLOGY:linear
CLONE:HUMGS00467
SEQUENCE DESCRIPTION:
GATCTGACCA CCTCTGCCCT GTCCACCAGG ATAAGTGACA CCTAGGACCC AGGAAATAAA 60
TGCCGATGAT TTGTGTGAAA 80

SEQ ID NO:441
SEQUENCE LENGTH:73
SEQUENCE TYPE:nucleic acid
TOPOLOGY:linear
CLONE:HUMGS00468
SEQUENCE DESCRIPTION:
GATCTTACAG GGAGAGAGAT TGGGTGCAAT TTGCCTCTTT CTTTGAATAA AAAGCTCTTT 60
GCTCACCCTC AAA 73

SEQ ID NO:442
SEQUENCE LENGTH:197
SEQUENCE TYPE:nucleic acid
TOPOLOGY:linear
CLONE:HUMGS00469
SEQUENCE DESCRIPTION:
GATCAAAAGT CTACATAACT AATACTCACA GCTGAGCTAT GTAGTATGCT ATGATTAAAT 60
TTACTTATGT AACTTTTATT GTCTTTGGCA TTAACAGTGT TTCAAAAAAT TTCCTGTGTA 120
TACCCATCAG TGATTCATTC CCAAATCTNC TAGAAGCATA AGTGTCTCAA TATATTAAAA 180
CATATTGAAT AATCAAA 197

SEQ ID NO:443
SEQUENCE LENGTH:75
SEQUENCE TYPE:nucleic acid
TOPOLOGY:linear
CLONE:HUMGS00470

SEQUENCE DESCRIPTION:
GATCCAGGCG CCACGCTGGC GGTTCGTGAG TGTCGAGGCA CCACTAAATA TAGCTGTCTG 60
CCGTCCACTC ATAAA 75

SEQ ID NO:444
SEQUENCE LENGTH:74
SEQUENCE TYPE:nucleic acid
TOPOLOGY:linear
CLONE:HUMGS00471
SEQUENCE DESCRIPTION:
GATCANATTG TAAGCTTTTC TGTTTNATTT CTTTTAAGAA CCTTTGAATA AAAAACATCT 60
GAAATTTTAA NAAA 74

SEQ ID NO:445
SEQUENCE LENGTH:73
SEQUENCE TYPE:nucleic acid
TOPOLOGY:linear
CLONE:HUMGS00473
SEQUENCE DESCRIPTION:
GATCTCTTTG TAGCCATCCT GTTAAATTTG TAAACAATCT AATTAAATGG CATCAGCACT 60
TTAACCAATG AAA 73

SEQ ID NO:446
SEQUENCE LENGTH:71
SEQUENCE TYPE:nucleic acid
TOPOLOGY:linear
CLONE:HUMGS00474
SEQUENCE DESCRIPTION:
GATCATGTCT GAATTATGTA TGAAAATTAT TCTATGTTTT TATAATAAAA ATAATATATC 60
AGACATCGAA A 71

SEQ ID NO:447
SEQUENCE LENGTH:69
SEQUENCE TYPE:nucleic acid
TOPOLOGY:linear
CLONE:HUMGS00475
SEQUENCE DESCRIPTION:
GATCTCTACC CACCCCATGC CTCTCCCNAG TCTTGGATAC TAATAAAATG ATAAGCATTC 60
TGGTTCTCN 69

SEQ ID NO:448
SEQUENCE LENGTH:68
SEQUENCE TYPE:nucleic acid
TOPOLOGY:linear
CLONE:HUMGS00477

SEQUENCE DESCRIPTION:
GATCGTAATG TAAAATTCTT TTACCATGTA CAAGAATTAT TAAAATACAG GTACTTGACC 60
ACATTCTN 68

SEQ ID NO:449
SEQUENCE LENGTH:72
SEQUENCE TYPE:nucleic acid
TOPOLOGY:linear
CLONE:HUMGS00478
SEQUENCE DESCRIPTION:
GATCGGGCCC CGGGGGCCTG AGCCTGGGAC CCCACCCNGT GTTAATGAAA AATGAGTTTT 60
GGCAGCGCCA AA 72

SEQ ID NO:450
SEQUENCE LENGTH:64
SEQUENCE TYPE:nucleic acid
TOPOLOGY:linear
CLONE:HUMGS00479
SEQUENCE DESCRIPTION:
GATCCTTTTG TAATGACTTA CACTGGAAAT GCGAACATTT GCAGTAAAAA AATATATATA 60
TAAA 64

SEQ ID NO:451
SEQUENCE LENGTH:66
SEQUENCE TYPE:nucleic acid
TOPOLOGY:linear
CLONE:HUMGS00480
SEQUENCE DESCRIPTION:
GATCATGCAT TTAGATTTAT ATTTTTNCCA NAAAATACAA GGTTATAATA AAACTAAGAN 60
CTACCN 66

SEQ ID NO:452
SEQUENCE LENGTH:70
SEQUENCE TYPE:nucleic acid
TOPOLOGY:linear
CLONE:HUMGS00481
SEQUENCE DESCRIPTION:
GATCTCTATT GTAATCTCTA TTGGAGATTA CAATGATTAA ATCAATAAAT AACTGAAACT 60
TGAANATAAA 70

SEQ ID NO:453
SEQUENCE LENGTH:65
SEQUENCE TYPE:nucleic acid
TOPOLOGY:linear
CLONE:HUMGS00482

SEQUENCE DESCRIPTION:
GATCACTAAT TTTGCATCAG TAAAATGAAT TTTTTTAAAA CCAATAAATC ATCAATTATT 60
AGAAA 65

SEQ ID NO:454
SEQUENCE LENGTH:63
SEQUENCE TYPE:nucleic acid
TOPOLOGY:linear
CLONE:HUMGS00483
SEQUENCE DESCRIPTION:
GATCACAGTT GCGTCATTGT GTATTAAATA CTTGGAATAA ATCAAGCAGG TCTCAACGCC 60
AAA 63

SEQ ID NO:455
SEQUENCE LENGTH:68
SEQUENCE TYPE:nucleic acid
TOPOLOGY:linear
CLONE:HUMGS00484
SEQUENCE DESCRIPTION:
GATCATTAAT TGTAAAGCGC TTTGTAAAAT TCACATTTAC AAAATAATAA AGTCAGTTCA 60
AACCTAAA 68

SEQ ID NO:456
SEQUENCE LENGTH:63
SEQUENCE TYPE:nucleic acid
TOPOLOGY:linear
CLONE:HUMGS00485
SEQUENCE DESCRIPTION:
GATCTGTGTC TGAGTCATCT TTGTATCTTG CCTAGCACCT ATCAATAAAT ACTTCTTGAA 60
TGN 63

SEQ ID NO:457
SEQUENCE LENGTH:62
SEQUENCE TYPE:nucleic acid
TOPOLOGY:linear
CLONE:HUMGS00486
SEQUENCE DESCRIPTION:
GATCATGGGA ATATGCAGAA TTTCCAATGT ATTTTTAAAT ACAAATAAAA TTGTAATTTA 60
GN 62

SEQ ID NO:458
SEQUENCE LENGTH:62
SEQUENCE TYPE:nucleic acid
TOPOLOGY:linear
CLONE:HUMGS00487

SEQUENCE DESCRIPTION:
GATCTAACAC TAACTGTATT GTTTTGTTCA CATCAAATAA ACATCTTCTG TGGACCAGGA 60
AA 62

SEQ ID NO:459
SEQUENCE LENGTH:61
SEQUENCE TYPE:nucleic acid
TOPOLOGY:linear
CLONE:HUMGS00488
SEQUENCE DESCRIPTION:
GATCTCGGCT CACTGCAATC TCTGCCTCCC GGGTTTCAAG CTTGTCCAGG NNNATCTCAA 60
A 61

SEQ ID NO:460
SEQUENCE LENGTH:61
SEQUENCE TYPE:nucleic acid
TOPOLOGY:linear
CLONE:HUMGS00489
SEQUENCE DESCRIPTION:
GATCAACCTG AGTTTTAAAA TACCTTTAAT AAATATNAGT NGAAAAAATG TCTACTTNAA 60
A 61

SEQ ID NO:461
SEQUENCE LENGTH:61
SEQUENCE TYPE:nucleic acid
TOPOLOGY:linear
CLONE:HUMGS00491
SEQUENCE DESCRIPTION:
GATCAAACAC CCCACCCTCA CAAAAATGGC CACGTTGCAA TAAAAATTGT GGCATATTAC 60
N 61

SEQ ID NO:462
SEQUENCE LENGTH:86
SEQUENCE TYPE:nucleic acid
TOPOLOGY:linear
CLONE:HUMGS00493
SEQUENCE DESCRIPTION:
GATCTTTATT TTCCCTTTGT ATTCATTTTA AGCATCTAAA TAAATTGCTG TATTGTGCTT 60
AATGTAAATA TTTGCTTTAT TACAAA 86

SEQ ID NO:463
SEQUENCE LENGTH:66
SEQUENCE TYPE:nucleic acid
TOPOLOGY:linear
CLONE:HUMGS00494

SEQUENCE DESCRIPTION:
GATCTCTACT ACTGTTGATT TTGCCCTCGG AGCAAACTGA ATAAAGCAAC AAGATGAAAA 60
CTGAAA 66

SEQ ID NO:464
SEQUENCE LENGTH:70
SEQUENCE TYPE:nucleic acid
TOPOLOGY:linear
CLONE:HUMGS00495
SEQUENCE DESCRIPTION:
GATCAAACTA GAACTCATAT GCCATACTAG ATATGGTTGT CAATAAACTT ATGACGTGAA 60
AAAAAAGAAA 70

SEQ ID NO:465
SEQUENCE LENGTH:57
SEQUENCE TYPE:nucleic acid
TOPOLOGY:linear
CLONE:HUMGS00496
SEQUENCE DESCRIPTION:
GATCCAAAAA GTGCGCGATG CGAGTAGTCA AGTCGTACTC CGCCATCTTG CCAAAGN 57

SEQ ID NO:466
SEQUENCE LENGTH:65
SEQUENCE TYPE:nucleic acid
TOPOLOGY:linear
CLONE:HUMGS00497
SEQUENCE DESCRIPTION:
GATCTAAAGC TCTTTCGATT TTATACTGAT TAAATCAGTA CTGCAGTATT TGATTAACCA 60
AGAAA 65

SEQ ID NO:467
SEQUENCE LENGTH:55
SEQUENCE TYPE:nucleic acid
TOPOLOGY:linear
CLONE:HUMGS00498
SEQUENCE DESCRIPTION:
GATCTAAGGC AAGAGTTTCA GATTTACTGT TGGAAATAGA CCCAACTCTT CATGN 55

SEQ ID NO:468
SEQUENCE LENGTH:56
SEQUENCE TYPE:nucleic acid
TOPOLOGY:linear
CLONE:HUMGS00500
SEQUENCE DESCRIPTION:
GATCATAAAT ATTAATGGNG AAAACACTGT AGTAATAAAT TTCNATATGC CAGAAA 56

SEQ ID NO:469
SEQUENCE LENGTH:52
SEQUENCE TYPE:nucleic acid
TOPOLOGY:linear
CLONE:HUMGS00503
SEQUENCE DESCRIPTION:
GATCTGTTCA GTGTCACTCT GTACCCTCAA CATATATCCC TTGTGCGATA AA 52

SEQ ID NO:470
SEQUENCE LENGTH:54
SEQUENCE TYPE:nucleic acid
TOPOLOGY:linear
CLONE:HUMGS00504
SEQUENCE DESCRIPTION:
GATCCCCGGT NGGTTTTGTG CTCAAAATAA AAAGCCTCAG TGACCCATGA GAAA 54

SEQ ID NO:471
SEQUENCE LENGTH:57
SEQUENCE TYPE:nucleic acid
TOPOLOGY:linear
CLONE:HUMGS00505
SEQUENCE DESCRIPTION:
GATCATCCGG TTATAGAGCA TAATTTGCCA ATAAAGCTTT TGGAAGCGGG AAAGAAA 57

SEQ ID NO:472
SEQUENCE LENGTH:60
SEQUENCE TYPE:nucleic acid
TOPOLOGY:linear
CLONE:HUMGS00506
SEQUENCE DESCRIPTION:
GATCCAGTGT TGGNATTCTT TGGTGTAAAT AAACGTTTGG TTTTATTTAT NCAGGTTAAA 60

SEQ ID NO:473
SEQUENCE LENGTH:51
SEQUENCE TYPE:nucleic acid
TOPOLOGY:linear
CLONE:HUMGS00507
SEQUENCE DESCRIPTION:
GATCTCATTT ATTGCCACAG ATGCACAAAA TAAATAACCC AAAATCACAA A 51

SEQ ID NO:474
SEQUENCE LENGTH:80
SEQUENCE TYPE:nucleic acid
TOPOLOGY:linear

CLONE:HUMGS00510
SEQUENCE DESCRIPTION:
GATCATATAT TTTGACAAAA TATATTTATA ACTACGTATT AAAAGAAAAA AATAAAATGA 60
GTCATTATTT TAAAGGTAAA 80

SEQ ID NO:475
SEQUENCE LENGTH:77
SEQUENCE TYPE:nucleic acid
TOPOLOGY:linear
CLONE:HUMGS00511
SEQUENCE DESCRIPTION:
GATCCAAAAC TTTAATGTTG CACNTGTATT CCAAATAAAG GGTAAAAACA GAACCAAAGT 60
TATAACTCCA ACACAAA 77

SEQ ID NO:476
SEQUENCE LENGTH:669
SEQUENCE TYPE:nucleic acid
TOPOLOGY:linear
CLONE:HUMGS00532
SEQUENCE DESCRIPTION:
GATCAAAAAG AAGGCTTAGA ATTCTGCAGT TAAGCTGAGG TTTAAACTAA AAANTGTTTC 60
CTTGGGTCAG TGGTTTTNAG GTCCAGTAGC TAGGCTTTTT TCTTTTGTCC TTCCTGTTGG 120
AATGAAAACA TTTCGATTTT CCTTCATCTG TGACTGGTGC CATAGACACA GGTTTATAGT 180
TTTAACTTAC AGTATTGTTT GAAATTTACC TGTTTTTNTT GTCAAACCTG AGCACTCCTC 240
CTGCTGAAGT TTCTTATTTA ATTCCAGAGT ACTGTCCTCT ACTCTAAGGC ATTACTTTTA 300
AGTGTATTAT GAAGGCAGTT TTCAAAGGAT ATGACCAGTT GGGGGTAATT CAAATTAAAA 360
AGGAAAAGAT TTGTTTGGGA AGTAACTGGG TGTCTCTAAG AGGGAATTTT TAGGATGTCC 420
AGTTTGGGAG GCTCTTTCCC CCCTCAAATT GAGANGCTCC TTGGTTAATT CAGAGCTCCC 480
ANGACTAGGC CCTGGGCTAA CCAANCATTN GGGNGGCCAA AGGTTAGGGA ACCATTNGNT 540
ACCAAGCTTT TGNANCAGGG GGNTTTNTNC CATTTGGGTA ATAGGGCCCT TTTCANGCCT 600
TTANGGGTAN GCTTTTTTAN CCCNGAAACC NTTNNTNNNT TTGNAATTAA ACCGGAACCT 660
TTTGNCAAA 669

SEQ ID NO:477
SEQUENCE LENGTH:651
SEQUENCE TYPE:nucleic acid
TOPOLOGY:linear
CLONE:HUMGS00533
SEQUENCE DESCRIPTION:
GATCAACCTG GAGCTCTACG CCTCCTACGT TTACCTGTCC ATGTCTTACT ACTTTNACCG 60
CGATGATGTG GCTTTNAAGA ACTTTGCCAA ATACTTTCTT CACCAATCTC ATGAGGAGAG 120
GGAACATGCT GAGAAACTGA TGAAGCTGCA GAACCAACGA GGTGGCCGAA TCTTCCTTCA 180
GGATATCAAG AAACCAGACT GTGATGACTG GGAGAGCGGG CTGAATGCAA TGGAGTGTGC 240
ATTACATTTG GAAAAAAATG TGAATCAGTC ACTACTGGAA CTGCACAAAC TGGCCACTGA 300
CAAAAATGAC CNCCATTTGT GTGACTTCAT TGAGACACAT TACCTGAATG AGCAGGTGAA 360

AGCCATCAAA GAATTGGGTG ACCACGTGAC CAACTTGCGC AAGATGGGAG CGCCCGANTC 420
TGGCTTNGGC GGAATATCTC TTTGACAAGC ACACCNTGGG AGACAGTGNT AATGGAAGCT 480
TAAGCCTTGG GGNTAATTTN CCCCATANGC NGTTGGGTTG ACTTCNCTGG TCANCAGGGC 540
AGTTCANTGA ATGTTNGGGG TTNCCTTTAC CTTTNCNTTA GGTNGTCCNA AACAATCCNT 600
NAAAGTCTTT GNTTTGNACC NTTCCGNNAA TAANGGATTN GGGCCCNGAA A 651

SEQ ID NO:478
SEQUENCE LENGTH:617
SEQUENCE TYPE:nucleic acid
TOPOLOGY:linear
CLONE:HUMGS00534
SEQUENCE DESCRIPTION:
GATCAAGAAA GTGGGGGGAA AAAAAACAAA CGTTAAAACC TCAATCCTCA GTAGGAAGGT 60
AGATTACATT AGGTGAAATT ATAGGTAATC TATGTATGTN CTAATGGGGT TGGAAAGAAC 120
CTTACAGAGC ATATTACCTG ATAAACTGGA GTGGGTTTGG GAGAACAAAC TAATAGGATT 180
ATNGTNTCTC CTAGTTGGTA CCTGGGAGCA ATTGACATGC CCCCTTCAGA ACCTTAACTG 240
TTAGTAGCAG TGGCTGTAAC AACACAAACC AGTGACCAGA GATAACAGCT TTTNGGCCAA 300
GCTGGCCTGA CGGTATGGCT GCAGGANGTG ACTGAGCAGT AGCGGTACTC AGCCAGACCA 360
AGACGGAGAG GGGAGAGTCC ACAGCTTTCT GGAGCTAAGG CATTCTGGTG GTAGAAAAGT 420
GTGCCCNAAG CCTTCATNGG CGGGTTATAN GGTCTNAAGA TAAGTCTCCT CTTGTNTGGG 480
ATNCCATACT NTGCTAAATA ACCNNGGTAT TANCCGGGTT TTCCNTGTAA CNGCCTCTNG 540
GGAGGAANTG ACTNNGNAAG NTGGCACAGG TNTTTAAGCN TNAATGGAAA GGGNNAAATC 600
CTNCTCAAAN TAGAACN 617

SEQ ID NO:479
SEQUENCE LENGTH:569
SEQUENCE TYPE:nucleic acid
TOPOLOGY:linear
CLONE:HUMGS00535
SEQUENCE DESCRIPTION:
GATCTGTCAG CTGCTTAATT AATTGAAACT TCTCTGTCAT TGATGTTGCA TTTCCAAGGA 60
GATAATCTCC TTCTTGGTGC CTAATTTTCT AGATGATAAT AGGCTAGTTT TGATTTCTTG 120
CTCATTTTCA GAATAACTTT CCAGGAAGAG ATGGCATTTA GAACTTCAGC TTTGGTGCTC 180
AGGTATAAAG CCAATTAAGG TACAATTGTA CCATAAAGGG AACAATCTGT TTCTGATTGC 240
ACAGTTTCTA ATTTTTAAAA CTGNNGTGGT TTGCATTTCA TAAAAGGCAA AGTTTACAGA 300
NCCATAAACA TTCTCAATTT TCTTTATGCT AGACATATAA ATTTATTTTT CCAAACTGTA 360
ATAGGATTTG GGGTAAAAAG NTTGTCTCAG GTNCCTCTNC CCANTTTGCC AATGGGGNAA 420
AAAAAAGGCT TAATTTTTTA CCATNNTACT TNAAATTTTC TAAAACCCNT GGTAACCCCC 480
CATTGGNACC CCNATTTTTC CANCTTTAAG GGTCTNGCAT NGGCNGGCTT TTTNAATTNN 540
CCCTGGGGGG GTTTTNCCTG GGAGGGCCN 569

SEQ ID NO:480
SEQUENCE LENGTH:556
SEQUENCE TYPE:nucleic acid
TOPOLOGY:linear

CLONE:HUMGS00536
SEQUENCE DESCRIPTION:
GATCCTGAGC NTGGTGCTGT GCTGTGGCAT CCGGAACAGC TCCGTGTACT GAGGCCCCGC 60
AGCTCTGGCC ACAGGGACCT CTGCAGTGCC CCCTAAGTGA CCCGGACACT TCCGAGGGGG 120
CCATCACCGC CTGTNTATAT AACGTTTCCG GTATTACTCT GCTACACGTA GCCTTTTTAC 180
TTTTGGGGTT TTGTTTTTGT TCTGAACTTT CCTGTTACCT TTTCAGGGCT GACGTCACAT 240
GTAGGTGGCG TGTATGAGTG GAGACGGGCC TGGGTCTTGG GGACTNGAGG GCAAGGGGTC 300
CTTCTGCCCT GGGGTCCCAG GGTGCTCTGC CTGCTCAGCC AGGCCTNTCC TGGGAGCCAA 360
TNGNCCAAGA GACTCAGCTT GGNCAAANTT GGGGGGGNTN TGTNCAACCA NGCCCGCNNN 420
TCCTNTTNGG GTTGAAAAGT TTAACCTTGT TTCCCTTTCT NGCCCCGGTT TTGGAGAACC 480
CGANTTTTTT GGGGNAATTT TTTGCTTTNA ATNAACTTNT NCCTTTTTTA AAAANGTGGG 540
TTTAAAACTN TNAATN 556

SEQ ID NO:481
SEQUENCE LENGTH:551
SEQUENCE TYPE:nucleic acid
TOPOLOGY:linear
CLONE:HUMGS00537
SEQUENCE DESCRIPTION:
GATCTACTGT CTTTGTTCAA AGGTCAAATA AAAACCTAGT CTCCTTTTAT TCTACTTTCT 60
ATTCTTAGCT AGAATGAAAC TCAGCATATA TACACTTCTG GACATAATAA TATTGAATAG 120
TAATTACCTT TACTAGATGA AAGAAATTTT CATTACAAAC TTAAATCATG TAAAACTCAA 180
CAACTCAGAT TCCTGGACCT GGTGTCCTGG NTGGGTCCAA GGTGATTTTA CAGAAGNAAA 240
AANCAACTNA AGCATTCTGG TGGCAACATA GAGATTGTAG GCTGCTTCTA AGGAAGTNAT 300
TAACAATTNG GAAATTCCNA AGTAGGATGA GAGTTAGTAA CTGGATACGA GTGAAGTTTA 360
TATCCAAGTT CAGNCTCAAA GGCATNATTA TGATTNGCTT CTTCCCATGT CTNCCATGGN 420
CCTGCTTCTC AAAGTTTTTC TNATCNATCA CACTGCTGCC TAACTGCTCT GAGNATGCAT 480
GNGGTNTTCA ATTCAGCGTN NTNTNAATCN GGNNTANCTN TGGATTGGGA TGGGGATACG 540
GACNTTAAGG N 551

SEQ ID NO:482
SEQUENCE LENGTH:520
SEQUENCE TYPE:nucleic acid
TOPOLOGY:linear
CLONE:HUMGS00538
SEQUENCE DESCRIPTION:
GATCTTAATT TTGAAATTGA CATGAAAGTG TCATATCAGT AATCTGTGAA CCACCAGTCC 60
TTGGTACCTA TCAGAGGGTC AAAAATCACG ATTAAATATA ACCAAAAAAC TTTATAGTGA 120
CTGATTCAAA TTTGAATACT GGTTTTAGCT AATGTAGTAG TAATGAACTG GTTTGGGGGT 180
AAGATTTTCC TGGTATCTTA TTGCTGTAGA AATTTTCCTT TAACAGTTAC AGTGTTTTCT 240
TCCAAATCCT TCACTTCTCT GTCCTGGCTT GTAAAGAAAA CATCTGAGGA CTGAGGGGTC 300
ATATTTGAAT TGCTCTNTAT AATACCATAG ACTACTCATT GCTTAGACTN TACTAAGCTA 360
GAAATCACAA GAGCATAAGC NACTCTNAAA ATTNATATNA TGNGAATGTA AAAGGTACCT 420
GNCTGCAAAT ATCTNGANCN TCACTTTGGC TCAAGTNTCN NGTTAACCTG TNNNNTAATA 480
CNGNNATGTG AATTNGGCCA CCAGGTCCAT GNTTGGCAAA 520

SEQ ID NO:483
SEQUENCE LENGTH:517
SEQUENCE TYPE:nucleic acid
TOPOLOGY:linear
CLONE:HUMGS00539
SEQUENCE DESCRIPTION:
GATCATGCTT TTNGTGCCTG TCACCAGGTC TCCCAAGTGC ACTCATCCAG GTCAGTGCTC 60
AGATGTGTTT AAGGAGACCC TATATTCAGG GAAGTTGCGT GAACACTGCA GTGGGGAGAA 120
TTGAGAATAG TCAGGCCTAT CAGTCTCACA GAATCACCCC TCTACCTTTG ATATTCCACT 180
TAGCTGTAGA GTCCATCTGT TTGTCCATCT GCTGAAATGA GAAAAGAAAA ATTTATGCAC 240
TGATTTAAAA CAAACCAAAA AAAAAGAAAA AAACAAAAAA AAAAATCCNT CCTTTCTNGC 300
TGACCAAAAN TGTGCAGTTA ATNCTGGGNG CTTGAAANTG CAGTGGTGAA TNTGGACCAA 360
GCCTGTCTGT ATATCTGGTA GCTCTTTTCT GGCTTNGTTT TTNCTTACCA GTATTCNGGC 420
CTAACGTTTT GCTTCGGGNN TGGTAATATN NCCTNGNAAG NACANCNGTG GGTTGTGGAA 480
ATGGGTTNGG CAAAANGGAA NTTCCNGGGG TNTTGGN 517

SEQ ID NO:484
SEQUENCE LENGTH:515
SEQUENCE TYPE:nucleic acid
TOPOLOGY:linear
CLONE:HUMGS00540
SEQUENCE DESCRIPTION:
GATCTTCTGG CTCTACCACC ACAAGATATT ATCCTTGCAT CTNATGTGTT CTTTGAACCA 60
GAAGATTTTA AAGACATTTT GGCTACAATA TATTTNTTAA TGCACAAGAA TCCCAAGGTC 120
CAATTGTGGT CTACTTATCA AGTTAGGAGT GCTGACTGGT CACTTGAAGC TTTACTCTAC 180
AAATGGGATA TGAAATGTGT CCACANNGNT CTTGAGTCTT TTGATGCAGA CAAAGAAGAT 240
ATAGCAGAAT CTACCNTTCC AGGAAGACAT ACAGTTGAAA TGCTGGTCAT TTCCTTTGCA 300
AAGNACAGTC TCTGAATNAT ACCNACAACC NGTNCTGGGA CAGTATCAAT ACTGATGAGC 360
AACCNGGCAC ACAAACTATG AGCAGACCAC TTCAGCTTGA GGAATGCAGT GGGTCTGAGG 420
ATGGTCAAGT CTGTTTGCCT TAGATTTTGN TGTCACTTGG CCACACTTGA AANCTNNTTT 480
GGAACAAAAN TTTAAATTCG GGTTTCCAAG GTAAA 515

SEQ ID NO:485
SEQUENCE LENGTH:510
SEQUENCE TYPE:nucleic acid
TOPOLOGY:linear
CLONE:HUMGS00541
SEQUENCE DESCRIPTION:
GATCTGCAGC TCTCAGAGGA CGACTGAGGC AGCCCATCTG GGGGGCCTGT AGGGGCTGCC 60
GGGCTGGTGG CCAGTNTTTC CACCTCCCTG GCAGTCAGGC CTAGAGGCTG GCGTCTGTGC 120
AGTTGGGGGA GGCAGTAGAC ACGGGACAGG CTTTATNATT TATTTTTNAG CATGAAAGAC 180
CAAACGTATC GAGAGCTGGG CTGGGCTGGG CTGGTGTGGC TGCTGAAGCC CCACAGCTGT 240
GGGCTGCTGA AGTCAGCTCC GCGGGGGAGC TGCCCTGACG TCAGCAGACC GAGACCAGTC 300
CCAGTTCCAG GGGGAGGCCT GCAGGCNCTG GCCCTTCCAC CACCTNTGCC CTNCGTCTGC 360

AGANCTTGGT NCATCTGCAC CAGGCTCTGC TTNACTCNNN NANAGTNTTT GGAAATTTGT 420
TCTNNTCCTN TGAAAGTCAC ATTTGNTTNT AAAAATTTTG TGGNTTGAAT CGGAAACGGG 480
AAGNAATAAA GCGGTGGGNG GNAGGGCAAA 510

SEQ ID NO:486
SEQUENCE LENGTH:507
SEQUENCE TYPE:nucleic acid
TOPOLOGY:linear
CLONE:HUMGS00542
SEQUENCE DESCRIPTION:
GATCCTTACA TCTGCCCATT CTGTGGTTAG TCAATGGCTT GCAATAAATG TGCAAACTGC 60
ATCTATAGGA AACATTTTTG TGATTACGGA ATACTTTAGT TGATTGCTGA AAATATTGAA 120
AGGTCTTCAT TTTACAGTGA TGAGTACATA TGCATGTTTC GGGGACTTGG CCCTTCTGAT 180
GAGGGGCCCT CGGTACTCTG GATAACGAAG CTTGTGCAGA GTGGTAACCA TGCTTACACA 240
CTAAACTATA ATATAAAGGA AATGAAGCCA TGTTAATCTG AGAGCAGTGT CGCCATAGTT 300
GTGTTGTTTA CAATACTCTA TAAATGGGGT TCCTGTTGCC CTGTAATTAA CCTGCTGCCC 360
GTAGAGGCCT TTCCAGTTCC TTTTCTGTCC TTNCCCCTTT CTTAACACAA GCTCAAATTT 420
TCCTAACTNG GTTTTNNATT TGGAGGNCTT TTAAAANGGN CCATTTTCAA TACCATNAAA 480
ANTAACCAGG GCTTTATAAT ANTTAAA 507

SEQ ID NO:487
SEQUENCE LENGTH:155
SEQUENCE TYPE:nucleic acid
TOPOLOGY:linear
CLONE:HUMGS00543
SEQUENCE DESCRIPTION:
GATCCACTAC CGGAAGAAGA AACAGCTCAT NAGGCTACGG AAACAGGCCG AGAAGAACGT 60
NGAGAAGAAA ATTGACAAAT ACACAGAGGT CCTCAAGACC CACGGACTCC TGGTCTTAGC 120
CCAATAAAGA CTGTTAATTC CTCAAAAAAA NGAAA 155

SEQ ID NO:488
SEQUENCE LENGTH:499
SEQUENCE TYPE:nucleic acid
TOPOLOGY:linear
CLONE:HUMGS00544
SEQUENCE DESCRIPTION:
GATCTTAAAA ACTAACTTCT AAGATGATTT CATCTTCTCA TAGTATAGAG TTTACTTTGT 60
ACACGTTTGA AACCAACTAC TGTAGAAGAT GAGGAATCTA TTGTAATTTT TTGCTTTATT 120
TTCATCTGCC AGTGGACTTA TTTGAAATTT TCACTTTAGT CAAATNATTT TTNGTATTAG 180
TTTTTGATGC AGACATAAAA ATAGCAATCA TTTTAAATNG TCAAAATTTC CAGATTACTG 240
GTAAAAATTA TTTGAAAACA AACTTATGGG TAATAAAGGC TAGTCAGAAC CNTATACCAT 300
AAAGTGTAGT TACCATACAG ATTAATATGT AGCAAAANTG TATGCTTGAT ATTNCTCACC 360
NGTGNTAATG TTNCTGCNGT ATTCCAGCNG ACCAAACCAA TATTAAGNAT GCATCTGTAT 420
AAAATGGGNG CCTATNGGNT AATGGGAATN ATTNGGGTAA TNGGCCTNTA CCNGGNTGGT 480
NATAATGGNG CCCTNTGGN 499

SEQ ID NO:489
SEQUENCE LENGTH:516
SEQUENCE TYPE:nucleic acid
TOPOLOGY:linear
CLONE:HUMGS00545
SEQUENCE DESCRIPTION:
GATCTACCCC GGACACGGGA GGCGCTACGC CAGGNCCGAC GGGAAGGTTT TCCAGTTTCT 60
TAATGCGAAA TGCGAGTCGG CTTTCCTTTC CAAGAGGAAT CCTCGGCAGA TAAACTGGAC 120
TGTCCTCTAC AGAAGGAAGC ACAAAAAGGG ACAGTCGGAA GAAATTCAAA AGAAAAGAAC 180
CCGCCGAGCA GTCAAATTCC AGAGGGCCAT TACTGGTGCA TCTTTTGCTG ATATAATGGC 240
CAAGAGGAAT CAGAAACCTG AAGTTAGAAA GGCTCAACGA GAACAAGCTA TCAGGGCTGC 300
TAAGGNAGCA AAANAGGGCT AAGCAAGCAT CTAAAAGGNC TTGCAAATGG CTGCTTGCTA 360
AGGCACCTTC AAAGGGCAGC ACCTTAGGCN AAAAGGATTT GTNTAAGCCN TGTTGAAAAG 420
TTTCCAGCTT CCCCGTNTTT TGGTTGGGAA NNGGNTAAAC CTTGGCAGGG TTTTGNTTTT 480
TTTAATTAAN AGGTTTGGGG TTTTAANCTN TTTAAA 516

SEQ ID NO:490
SEQUENCE LENGTH:497
SEQUENCE TYPE:nucleic acid
TOPOLOGY:linear
CLONE:HUMGS00546
SEQUENCE DESCRIPTION:
GATCAAGCTG ACAGACCCNN CTCTGCTCTG ACCTGGAGGT CTCCACTCTG GTCAGCAAGT 60
ATCCAGACAT CAGGGATGAC CACATCGGTG CGCTNCTGGC TGTGCGTGGG GACGCCAGCC 120
GTGACATGAA GCAGACCATC ATGGAGACCC TGGAGCAGGG CCCAGCACAG GCCAGCCCCA 180
GCTACGTGCC CCTCTTCAAG GACATTGTGG TGCCCAGCTG AACGTGGCCA AGCTGCTCAA 240
GTAGCCTCCG CGGNCTGCCT GCTCGCCCTC CACAGCTNGG TCCTGCTTTA GAACGCGGGC 300
AGTNATTGTC TCTTGGCACA CGTGTCCTTT TAGTGACGGC TGTNTTTAGG TGCANTGTNA 360
TGACNGGGTG TGCGTCGAGT GANGTCNGAG GGCACGTGCG GAGGCNGTAN TTTGCTGTAA 420
AGGCTGTGGG TTCAGNGTTT NCNGACAGCG TTNNTTGGGT GTTGTTNTTC AGNGGTGAAG 480
TGTTNGGGAA AGNGNCN 497

SEQ ID NO:491
SEQUENCE LENGTH:494
SEQUENCE TYPE:nucleic acid
TOPOLOGY:linear
CLONE:HUMGS00547
SEQUENCE DESCRIPTION:
GATCCAAGAA ACAATTTCTG ATAATTGTGT GGTGATTTTN TCAAAAACAT CCTGTTCTTA 60
CTGTACAATG GCAAAAAAGC TTTTCCATGA CATGAATGTT AACTATAAAG TGGTGGAACT 120
GGACCTGCTT GAATATGGAA ACCAGTTCCA AGATGCTCTT TACAAAATGA CTGGTGAAAG 180
ANCTGTTCCA AGANTATTTG TCAATGGTAC TTTTATTGGA GGTGCAACTG ACACTCATAG 240
GCTTCACAAA GAAGGAAAAT TGCTCCCACT AGTTCATCAG TGTTATTTAA NNNNAAGTAA 300
GAGGAAAGAA TTTCAGTGAT GTTTATACTA ATAAGTTTGC TAGTACAGTG TCAGTTATTT 360

AAAGTGGTAA TGCCCGNTAA TGTCTTTTAA ATGTTTTGAG GGATGTTTTA AAATACATGC 420
NATTGTCTTC ACGGAGGAGG GNTGTAAAAA TTANTGGGCC AATAAATTGC GGGTGGGAAN 480
CCNTNTTCTT NAAA 494

SEQ ID NO:492
SEQUENCE LENGTH:489
SEQUENCE TYPE:nucleic acid
TOPOLOGY:linear
CLONE:HUMGS00548
SEQUENCE DESCRIPTION:
GATCTTGACG AGGCTGCGGT GTCTGCTGCT ATTCTCCGAG CTTCGCAATG CCGCCTAAGG 60
ACGACAAGAA GAAGAAGGAC GCTGGAAAGT CGGCCAAGAA AGACAAAGAC CCAGTGAACA 120
AATCCGGGGG CAAGGCCAAA AAGAAGAAGT GGTCCAAAGG CAAAGTTCGG GACAAGCTCA 180
ATAACTTAGT CTTGTTTGAC AANGNTACCT ATGATAAACT CTTTAAGGAA GTTNCCAACT 240
ATANACTTAT AACCNNAGCT GTGGTCTCTG AGAGACTGAA GATTCGAGGC TCCCTGGCCA 300
GGGCAGCCNT TCAGGAGCTC CTTANGTAAA GGNCTTATCA AACTGGTTTC AAAGCACTGA 360
GCTCANGTAA TTTACACCAG AANTACCANG GGTNGAGATG CTCCAGCTTG CTTGTGAAGA 420
TGCATGATTA GGTCCACCAG CTGTACATTT GGAAGAANTA NANCTTNTGT TAAATCAATG 480
GNGTNNAAA 489

SEQ ID NO:493
SEQUENCE LENGTH:487
SEQUENCE TYPE:nucleic acid
TOPOLOGY:linear
CLONE:HUMGS00549
SEQUENCE DESCRIPTION:
GATCCTCTGN CACGGGATAA ATTTNCAGGN GAAGAGTGAG GTTGTCATGG CCTCAGCTAT 60
GCTTCCNGGC TCTCCCTCAA GAGTGCAACC TTGGCTAGAG AACTCACAGC TCTGGGAAAA 120
AGAGGAGCAG ACAGGGTTCC CTGGGCCCAG TCTCAGCCCA GCCACTGATG CTGGATGACC 180
TTGGCCTGAC CCTGGTCTGG TCTCANAATC ACTTTTCCCA TCTGTAAAAT TGAGATGAAT 240
TTTNGTGTTG AAAGTNCTTC CNNGAGCAGA TGTCCTAGAA GGTTTTAGGA ATAGTGACAG 300
AGTCAGGNCA CCCNAAGGGC CATGGGGAGC CAGCTGACCT GCTTNGCCGA AGGATTTCTG 360
ACAGACTATC TTTGGGGATG TTTTCAAAGA AGGGATATAG GTTATTGACN TNNGGGCATT 420
TAAAGNAAAT TNTNTCTCGG GGATTAANTT TTTAGGANAA TNAAAGCTTT NGTGTCTANN 480
GGCAAGN 487

SEQ ID NO:494
SEQUENCE LENGTH:481
SEQUENCE TYPE:nucleic acid
TOPOLOGY:linear
CLONE:HUMGS00551
SEQUENCE DESCRIPTION:
GATCCCCAGC TGCCACTGAA TCTGGCTGCC CTTCAGGCCC ACCTGGCCCA GGAGAACCGT 60
GTGGTGGCCT TCTTCAGCCT GGCTCTACTG CTTGCCCCAC TGGTGGAGAC GCTTATTCTA 120
CTGGACCGGC TGCTGTACCT TCAGGAACAG GGTTTCCATG CTGAGCTCCT GCCCATCTTC 180

AGTCCTGAAC TCTCTCCCAG AAACCTGGTT CTGGTGGCCA CCAAGATGCC CCTGGGTCAG 240
GCTCTTTNTG TTCTGGAGAC TGAAGACAGC TGATGCAGCC TGAGGAGACA TCTCAGACCC 300
CATCATCTGA AAGTGNCCAG AGAGCACAGT GGCAGAGTAC ATCTNATCCA GAGAAACAGC 360
ATCCTGCATC CTCCAGAGTC CTGGTTCCTT CAAGTTTCAT CNCTTTTNTC TCCTTTCCAT 420
GGGNTTATGT AAATACAATT GTAAAGTTTT AATTAAATTA AAAAATTGGG TTATCTGGAA 480
A 481

SEQ ID NO:495
SEQUENCE LENGTH:472
SEQUENCE TYPE:nucleic acid
TOPOLOGY:linear
CLONE:HUMGS00552
SEQUENCE DESCRIPTION:
GATCTAAGCT CCAGGCACGC CTGAAGATGT GTTGCTACTC TNACATCCCG AGTTTCTGTC 60
CACACATTGC ATGCACAGCG CCCCACACAT TGGATACTGT TGTTCACGAT AATTTCTCCC 120
GTTTTCCAGA GCATTTAACA TAGCTTGGAG GCGTAAAATG GCTCTGTATT TTAATAACAC 180
AGAAACATTT GAGCATTGTA TTTCTCGCAT CCCTTCTCGT GAGCNCTTAG ACCTTTTNCT 240
ATTTTAGTCG GATTTTGTTT TGGAATTTTG CTTTNGTATG AACACTCAGC AGAAAAGTAC 300
TTACTTCTNG CCAGTTATCT ATTAACCAAA ACCNTTGATT TGTAGTTTTA AAGNTTAACC 360
GNCAAAGTTC TNTNCATAAC TGCCTTGGCC AGTNNGGGGT NGTNCCGGTN CTGGTTAATN 420
GCCTGTGGCN TTTTNGGTGG TTTGTGNTTG GTNTTTACNT GNGCANTTAA GN 472

SEQ ID NO:496
SEQUENCE LENGTH:461
SEQUENCE TYPE:nucleic acid
TOPOLOGY:linear
CLONE:HUMGS00553
SEQUENCE DESCRIPTION:
GATCAACCAA TTTTNCTAAA AGTTCAGTCG AAAGCTTTTA AGTATAGCTT CCTCCCTTGA 60
AAAAAAATGT AAACTATGAC TGCTGAGTGA TAAAACACTG TGGTGTGAAA GTNTCATCTT 120
CACTGCCAAT CAGGCAAAGA CCGGAAAGAT TTGCATTTTA TTATGTCTGT CTTATCATGC 180
AATGGAAATN ATGCTTTTNG TAAGTATGCA TCTTACCAAT GATGTAACGG TTTAATACCN 240
TTGAATGTTT TAATAACCAA GTNGCTGCTG AACTTATACT AAATCAGGGG CCAAAAAACT 300
NGCTCTNATC NNCTCAAATN GTATNCNATA TCCATTAATG TATCAGTTAT NCCAAAGCCT 360
TCAGGTGGAG GGGTTTACCA CCNTCCTAGG TCGTTCAACC AGGTTTTGTG AGGAATGCAT 420
TCAAAGTGGC TNTATAAAAG ANGATTTTCT TTAGCATGAA A 461

SEQ ID NO:497
SEQUENCE LENGTH:459
SEQUENCE TYPE:nucleic acid
TOPOLOGY:linear
CLONE:HUMGS00554
SEQUENCE DESCRIPTION:
GATCCCAAAA TTCCACTTTT CAACACAGAT GTGGACAACC TAGAAGGGAA GACACCACCA 60
GTTTTTNCTT CTAAAGGCAA ATACAGGGCT CTGAAAATGG ATTTTCCTCT ACCCCCTTCT 120

ACTTACGNCA CCATGGCCAT TCGAGAAGTG CTAAAAATGG ATACCAGTAT CAAGAACCAG 180
ACGCAGCTGA ATACAACCTG GCTTCGCTGA GCAGTACCTT GTCCACAGAT TAGAAAACGT 240
ACACAAGTGT TTGCTTCCTG GCTCCCTGTG CATTTTTGTN TTAGTTCAGA CTCATATATG 300
GATTTCAAAT CTTTGTAATA AAAATTATTT GTATTTTTAA GTNTTTATTA GCTTAAAGAA 360
ATAATTNGCA ATATTNGTAC ATGTACACAA AATNCNGGAG GTTCTTANTT TTAGCTCAGG 420
NTATAAATNA GTCAAATNCN NNGGTNNNGG NTNNGNTGN 459

SEQ ID NO:498
SEQUENCE LENGTH:481
SEQUENCE TYPE:nucleic acid
TOPOLOGY:linear
CLONE:HUMGS00555
SEQUENCE DESCRIPTION:
GATCCAGCAG AGAAGGATGA AAAGGGCATG CCTGTGACAG CTCGTGTGGT GTTTGTTTTT 60
GGTCCTGATA AGAAGCTGAA GCTGTCTATC CTCTACCCAG CTACCACTGG CAGGAACTTT 120
GATGAGATTC TCAGGGTAGT CATCTCTCTC CAGCTGACAG CAGAAAAAAG GGTTGCCACC 180
CCAGTTGATT GGAAGGATGG GGATAGTGTG ATGGTCCTTC CAACCATCCC TGAAGAAGAA 240
GCCAAAAAAC TTTTCCCGAA AGGAGTCTTC ACCAAAGAGC TCCCATCTGG CAAGAAATAC 300
CTCCGCTACA CACCCCAGCC TTAAGTCTCT TGGAGAAGCT GGTGCTGTNA GCCAGAGGAT 360
GTCAGCTGCC AATTGTGTTT TCCTGCAGCA ATTCCATAAA CACATCCTGG GTGTCATCAC 420
AGCCAAGTTT TTTANGGTTN CTATACCAAT GGGTTTATTT AAATGAAAAT GGGCACTTAA 480
A 481

SEQ ID NO:499
SEQUENCE LENGTH:453
SEQUENCE TYPE:nucleic acid
TOPOLOGY:linear
CLONE:HUMGS00556
SEQUENCE DESCRIPTION:
GATCCAGATG CAGAGGCCAG GATGTGGGCC CAGCCCTGTG CCAGGAGGCT GGCTGGAATA 60
AAGGTACAGA TAGAGGCCTC ACCCCCTCTG GGACCACTGG CACTCAGGGT GTTTGCAGCC 120
TCAGAGCCCA CCTGCCCCCA GGGCCACAGC TGCATCTCCT GCCCTGCTGT CATTACAGGG 180
ATGGGCAGGC TGGCATGGGG GCACCCGCTG CCCCTGCCTG GNTGTTGCTG TGTATTCCTG 240
CCGGCCAGGG GCACTGCCAG GACCACGCCT CCNTTTTNAT ATCCNGATTC TTAAGTTCTG 300
CTATTGTGGT ATTCTGGTGG AGAAAAAAGA CCGNGTGGCT GTTTTTGAAC TGCCTGGAAC 360
CTAAGACCCT GAATTCTTTT CCCCCCAAGG GAAAATCTAT ATGGAAACAT TTATTTAAAT 420
ACAGGATGAA GTAATTAAAA GNTTTAATTC AAA 453

SEQ ID NO:500
SEQUENCE LENGTH:446
SEQUENCE TYPE:nucleic acid
TOPOLOGY:linear
CLONE:HUMGS00557
SEQUENCE DESCRIPTION:
GATCAAAGTT GGCAAGGTGC GGACTCGAGA CATGGGCGGC TACAGACCAC AACCGACTTC 60

ATCAAGTCTG TCATCGGTCA CCTGCAGACT AAAGGGAGCT AGAGCCCTTT ATTTCTTCCA 120
ACCTTGCAAG GACCACACTN CCCATACCNT TCAGTGCAGT GTACCAGGGA AGAGCCTTGT 180
GCCTCTAAGC AGTGGACCAT GGTCACCTTG CTGGGTAGAG CCTAGGTTGT CCTTGGGCCG 240
GCTTCCTTAG GGGACAGACT NTTGGGTGGT GATGGGGATT GTAGGATGGA GCCAGGCACA 300
TGGATGATGA TGATTCTCCC NCACAGGTTC GAACCTCTGA CATGGGTGGC TATGCTACTN 360
GCNATGCTTA NTGAGGNTGT CATTGCTGCT TTNCCNAACC ATAGGCCTGT CATACNNTGT 420
AAGGNGTCAA TAAGGACATG ACCAAA 446

SEQ ID NO:501
SEQUENCE LENGTH:434
SEQUENCE TYPE:nucleic acid
TOPOLOGY:linear
CLONE:HUMGS00558
SEQUENCE DESCRIPTION:
GATCCAATTC AGGTTAGGCT TGTTTGATTT TTTTTTTCAG AAAATGTATT CCATAAAGTT 60
TGTACTTAGA CCAGACGGGT GTACTAAGAA TCATTCATGA GTAAATGTGT GTTGAATATC 120
TACCCTTGAC CTTTTTTTGA GAAATAGAGT AAACACAGTC CCTGTAGTCT GACAGCTAAT 180
GGGGAGAGAG GGTAGACTTT TCATCGAATT AAATTTCTAC ATGCACCTTT CCCCCAGAAA 240
TCTTACTCAT GGCTGGTCTC AAGTAAGTCT TTATTGAAAA ATATNGACAT ATCTNCTTCC 300
TCTTCCTTCT CCTCACTGTC TTCCTTTTAG TAGTTAGGCA GAGTTAATAG GTAAGAAAAA 360
TTATCTGCAT TTATGTGTAG TTTGTAATCT ACTAAAGGGG TTCTAGAATA AATGTNGNCA 420
TNTNGTAACN GAAA 434

SEQ ID NO:502
SEQUENCE LENGTH:430
SEQUENCE TYPE:nucleic acid
TOPOLOGY:linear
CLONE:HUMGS00560
SEQUENCE DESCRIPTION:
GATCAAACAG GTTCAAAGTA AAACGTTAAA TTTCACATTT NTTTTAAAGA ACTCTTAAAG 60
TGTAACAGTT ACGCCATACT TCATAAGTGG TAAAGAAAGG TATAAAATTT GGAAACATTT 120
TGTTGGGCAT AGTAGTGATT GGGTGAAAAG GATAAATTAT ATCAAAATGA GAATGTNCTG 180
TAATTGGAAG TAGGGAGCTA AAGGATGTTT CTTTCAGTTT AGTAGAACTG GAACGTTTTA 240
CTATTAAACA TGGCTTTTAT AAATNCATGG TCCAATAATT TTATTCACTG TTAGTATTTA 300
ATTCACTGTC AGCTTATTAA TGTTTTCTGT ACCCATTAAT GAATTTTAAA TTACAAAAAA 360
TTGTCTAGCA GCTTCCAGTT TAANAAATGG AACCTAGGCC ATTAAAATTA AATTTGGTAA 420
ATTTTTTAAA 430

SEQ ID NO:503
SEQUENCE LENGTH:428
SEQUENCE TYPE:nucleic acid
TOPOLOGY:linear
CLONE:HUMGS00561
SEQUENCE DESCRIPTION:
GATCCTAAAT GTGTTGCTGA AATCAGGCAG CCCGAGCCTC TGGTCTCTCC AGAGAGCCCG 60

TNTTCACATT TGTNTATTCC TCAGCACTCA CCCGAAACTG AACAGATGGG GAGTGGTCTT 120
GATTGTCAAG ATAAAACTGG TGAAGAAAGC TAAATGCTGA GAAACTGAGC ATCTATTGTG 180
GTGTTTAAGC TTAGCTGGGT CCTTTCTAGT TTGTTTTTAC AGCTTACTAG GTGAAGTAGT 240
TTGCACTATT TTNGCAATAA ATTCATGGAA AACCTAACAG TTACTNGTNT NGTTTCTNAC 300
TGTGTGTATA TAANCTAATA CTAAANGTNT GGCATAGTGT TTNTGCACCT NCNTACATAA 360
CCNCTAACAT GCACAGAATG CTGGTAAATN TGATAAANTA TGANGTGANT GATGATNNGA 420
TANAGTGN 428

SEQ ID NO:504
SEQUENCE LENGTH:422
SEQUENCE TYPE:nucleic acid
TOPOLOGY:linear
CLONE:HUMGS00562
SEQUENCE DESCRIPTION:
GATCCTCACC CTCCTGAGGC CCAGTGGGGA AGAATGAACA TGGCTTCATC CAGGTTAACT 60
NATGCTGCCA TTTGCCCAGC CTCTTCCATC CCAGCCCTGT CAGTNAGCCC AGGTCTGGTG 120
CAACTNCTGC AGGATGCCTG TAGTAGGGAA CTCTGGAAGT GTATTGGGCT GAGGTGGGAT 180
TTTCCCTCCC CACAGTGCAC TGAGCAATGG AGGGTGGTGA GGGAGCCATG CTGCTGAATT 240
CTGGTTGGCA TTTCCCCATT ATGTAAAATG GGGTGTTGGG TAGGGCAGAC TCTGCTTGGG 300
TTTGGTTGTA AGATAAACCT GGAGGAGAAG CACAGTTGTC CCATTGAATT ATTTGAGCAA 360
AAACTACTGT AAATAACTTT TTTGGGCTNT TGTCAAATAA AATTTTTTTT TGTTTNTTTA 420
AA 422

SEQ ID NO:505
SEQUENCE LENGTH:417
SEQUENCE TYPE:nucleic acid
TOPOLOGY:linear
CLONE:HUMGS00564
SEQUENCE DESCRIPTION:
GATCTGATTC AGAAGGGCGT CATCCAACAC AAAGAGAAAT GCAACCAATG AAGAATCAAG 60
CCACTGAGGC AGGGCAGAGG GACCTTTGAT AGGCTACGAT ACTATTTTCC TGTGCATCAC 120
ACTTAACTCA TCTAACTNNT TCCCCGGACA CCCTCCACCT CTAGTTGTTA CTAAGTAGCT 180
GCAGTAGGCA TTGCTGGGGA AGAAACAAAC ACACACCAAA CAGTACTGCT ACTTAGTTTC 240
TAAGGCTGCA CAGGGAAGGG AAAGACTGGG CTTTGGACAA TCTAGAGGTA ATTTATATCC 300
GCCCCCAGGT GGAGCAACAT GCGATTNTGG AGGCACGGGG GTAACTGAAA GTGAGTACAT 360
ATAGTNTTTC TGGTTTCTGG GGATAACCCA TCAATAAAAG CTGCTTCCTC TNGTAAA 417

SEQ ID NO:506
SEQUENCE LENGTH:421
SEQUENCE TYPE:nucleic acid
TOPOLOGY:linear
CLONE:HUMGS00565
SEQUENCE DESCRIPTION:
GATCTTCTTT ATAATTCTAC TTTGAGTGCT GTCTCCATGT TTGATGTATC TNAGCAGGTT 60
GCTCCACAGG TAGCTCTAGG AGGGCTGGCA ACTTAGAGGT GGGGAGCAGA GAATTCTCTT 120

ATCCAACATC AACATCTTGG TCAGATTTGA ACTCTTCAAT CTCTTGCACT CAAAGCTTGT 180
TAAGATAGTT AAGCGTGCAT AAGTTAACTT CCAATTTACA TACTCTGCTT AGAATTTGGG 240
GGAAAATTTA GAAATATAAT TGACAGGATT ATTGGAAATT TGTTATAATG AATGAAACAT 300
TTTGTCATAT AAGATTCNNT ATTTACTTCT TATACATTTG ATAAAGTAAG GCATGGTTGT 360
GGGTAAANCT GGGTTTATTT TTNGTTCCAC AAGTTAAATA AAATCCATAA AACCTTGGAA 420
A 421

SEQ ID NO:507
SEQUENCE LENGTH:413
SEQUENCE TYPE:nucleic acid
TOPOLOGY:linear
CLONE:HUMGS00567
SEQUENCE DESCRIPTION:
GATCCCANAC TGGTCNTTGA ACAGACAGAA GGANGTAAAG GNTGGAAACT ACAGCCAGGT 60
GTGTACTGAA ATNAGGGCAG GATTAGAGGA AGGGTGGAGG GTCCTAACAG AATTGGGCAT 120
AGGAGGTCAG GGGATAAAAC ATCCCTTGCC CCCTCCTCTG AATCCAGGNC CTAGCCAATG 180
GNCTGGACAA CAAGCTCCGT GAAGACCTGG AGCGACTNAA GAAGATTCGG GCCCATAGAG 240
GGCTGCGTCA CTTCTGGGGG TGAGTGGGGG GTCTCATCTC CCTGCCTACC TCGACTCAGC 300
ATTCCTCCTA CTCGNTCTTC TTNTTTTCCC AACCTTTTTG TTTCTTGCTT GTNCATGACC 360
TNGTGACTTN TTCNTNTTTT TACCNTGCAN GCCTTTNGTN GTCCTAGGGN CAN 413

SEQ ID NO:508
SEQUENCE LENGTH:407
SEQUENCE TYPE:nucleic acid
TOPOLOGY:linear
CLONE:HUMGS00568
SEQUENCE DESCRIPTION:
GATCTNAGGG TGAACCACTT CATTCTGCAG GGTTCTCCCT CCCACCTTAA AGAAGTTCCC 60
CTTATGTGGG TTGCCTGGTG AATGGCCTTC CTTCCCGCCA NAGGGCTTGT AAACAGACCG 120
GAGAGGACAG TGGATTGTTT ATACTCCAGT GTACATAGTG TAATGTAGCG TGTTTACATG 180
TGTAGCCTAT GTTGTGGTCC ATCAGCCCCT CACATTCCTA GGGGTTTNAG ATGCTGTAGG 240
TGGTATGTGA CACCAAAGCC ACCTCTGTNA TTTGTNGTGA TGTCTTTNCT TGGCAAAAGC 300
CTTGTGTATA TTTGTATATT ACACATTTGT ACAGAATTTN GGAAGATTTT CNAGTCTAGT 360
TGCCAAATCT GGCTCCTTTA CCAAAAGGAN ATTACCCTTG NGGNAAA 407

SEQ ID NO:509
SEQUENCE LENGTH:402
SEQUENCE TYPE:nucleic acid
TOPOLOGY:linear
CLONE:HUMGS00569
SEQUENCE DESCRIPTION:
GATCAAACAG TTTCTGGAGT GTGCCCAGAA CCAGGGTGAC ATCAAGCTCT GTGAGGGTTT 60
CAATGAGGTG CTGCANCAGT GCCGACTTGC AAACGGATTG GCCTAATGAA GAAGTTCAAC 120
CTGGAGAGAT GGAAAATCAG CTCTCATAAC TANGTTAATT TAGTATAAAA NTAGAATTGA 180
TAGTGAGGGT ATAAAGTGTA ACCATCAGTT AAACCTCTCC TGTCATTCCT GGCTTCCTTG 240

CTTCAGANTT GAAATGGAAG TGGGGGTGTC CCTACTCTGT AGAATCTGGG NCTGGGCAAA 300
TGTTTGTTTG GCCTCCTTAA ACTAGCTGTT ATGTTATGAT TTTTNTTCTT TGTGAGTTAA 360
TTAGGAATAA AGTCATTTTC TTTCCAAGGG TATGGTTCCA AA 402

SEQ ID NO:510
SEQUENCE LENGTH:396
SEQUENCE TYPE:nucleic acid
TOPOLOGY:linear
CLONE:HUMGS00571
SEQUENCE DESCRIPTION:
GATCATGAGA AGGAATGGAA ACTAGGCCGG TGCATTTTAC GGTTCCCTGA GATTCTGCAA 60
AAGATTTTAG ATGACTTATT TCTCCACACT CTCTGTGATT ATATATATGA GCTGGCAACT 120
GCTTTCACAG AGTTCTATGA TAGCTGCTAC TGTGTGGAGA AAGATAGACA GACTGGAAAA 180
ATATTGAAGG TGAACATGTG GCGTATNCTG CTATGTGAAG NAGTAGCTGC TGTCATGGCC 240
AAGGGGTTTG ATATCCTGGG AATAAAACCT GTCCAAAGNA TGTAATCCTT CATAGGTTTG 300
ACACTGTGTG TTTTACCCAA GTGGCCATTG GNACTGTTTG CTTTTTTACA ATCATGTGGG 360
CACAAGCNTA AGAAAGGAAA TTNGCAACCA GGGAAA 396

SEQ ID NO:511
SEQUENCE LENGTH:384
SEQUENCE TYPE:nucleic acid
TOPOLOGY:linear
CLONE:HUMGS00572
SEQUENCE DESCRIPTION:
GATCCAATCA GATGCAGAGA ATGTGGATAC AGAATAATGT ACAAGAAAAG GACTAAAAGA 60
TTGGNCGTTT TTNATGCTCG ATGAATGCTG GGAATTCAGA GGAATGTNTT CACTTATACT 120
TGGATTTGCT CTCTTCCCAT TTCTGATTGT NGTATAGCTT TCGATTTTNC TTACAGTAGT 180
TCCCCCTTAT CTNCGGGAGA TACATTCCAA GGCCCCCAGT GAACTCCTGA AACCTCAAAC 240
AGTACCAAAC CTTTATACAC TGTTTTTTCC ATATATATAT ACCTATGATA AAGTATAATG 300
TATANNTTAA GCATAGCAAG AGATAATAAT AATGTNATAG NCCATTGNTA CNANCTATAN 360
TAANNGGTTA TGTGANTGTG AAAA 384

SEQ ID NO:512
SEQUENCE LENGTH:383
SEQUENCE TYPE:nucleic acid
TOPOLOGY:linear
CLONE:HUMGS00573
SEQUENCE DESCRIPTION:
GATCAGAGCA AAACATGCAG AGCCCTTAGC AGAAACCCAC TTTAATGCAT TTTCTTCATA 60
TCCCTAAAGT TCCTTAAAAA TATGTGACAA TGCATCAGGA AGAGGAGAAC TGAAGAGTAG 120
AAGTTCCCTT GCAGATTTTT TTATCAGTGA CATGTAATGA GCAATTCACA GATGAGCGCA 180
GGCAGAGCTC TGTGTGCCGT GTACATATGG ACCGTGCTAT GATGTGTCTC ACATTGGATG 240
ATATTCCACT TTGGGAATTT TAGTATTTGT ATATAGAAAA TGGGTTTAAT AACTCACCAT 300
GGTTTTNATT NGTCTTATAT TCGTTATTTC TTAAAACTCT NGTATGTGTT TTTATAATAA 360
ANAATAAAAG TAAGCCATGG AAA 383

SEQ ID NO:513
SEQUENCE LENGTH:381
SEQUENCE TYPE:nucleic acid
TOPOLOGY:linear
CLONE:HUMGS00574
SEQUENCE DESCRIPTION:
GATCCAAAAT CATACTGTCA AGACCATCAA ACAAAACAAA GAAGCTTAAG TGAACTAAGC 60
ACAGGGAAAA GTTGTTTTTT CACTCGTTCA TTTTGTCATC TGCTTCTAGA TGCTGCAATC 120
AAGAAGTAGC AAATGCCCAA GTTGCCATAG TGTTTGCAGG AAAAAAAGAG AAAAAATAAT 180
AAAAATAAGG AAGGAGCAAT GCCAAAAATT GAAGGAAATA TTATATAATT AANGCAAGAA 240
GNTATCTATC CATTGAGNGA AACAATTTTT ATATTATTTG CTTTTAGCNG CAAAGCATTA 300
GGAATTCTGA GATTGTTATA GCACTAAGAA GGTTTTNATT CTGTGTACAC ACTGGAAAAT 360
TAAAATTCTG GGTAAAGGAA A 381

SEQ ID NO:514
SEQUENCE LENGTH:381
SEQUENCE TYPE:nucleic acid
TOPOLOGY:linear
CLONE:HUMGS00575
SEQUENCE DESCRIPTION:
GATCCCCCTG GAAGAGGGAC TCCAATGGGC ATGCCCCCTC CGGGAATGCG GCCTCCTCCC 60
CCTGGNATGC NAGGCNTTCT TTNACCCTTG GCCACAGAGT ATGGAAGTAG CTCCGCAGAG 120
GCGTGGGCTC GATTCCTCAG GGCCACGTTA CCACAGACCT GTTTGTTTCT NATGCTGTTG 180
TTCGTGGAGT CTCATGGGAT TGTNTGGTTT CCNTTACAGG GCCCNCTCCC CNGGGAATGC 240
GCCCACCAAG GCCNTAGACT CATCTTGGCC NTCCTCAGCT CCCTGCCTGT TTCCGGTAAG 300
GCTGTACATA GTNCTTTTAT CTNCTTGTGG CCTATGAAAC TGGTTTATAA TAAACTNTTA 360
AGAGAACATT ATAATTGCAA A 381

SEQ ID NO:515
SEQUENCE LENGTH:377
SEQUENCE TYPE:nucleic acid
TOPOLOGY:linear
CLONE:HUMGS00576
SEQUENCE DESCRIPTION:
GATCTGCGTG GGGCTGGTGG TGGTTGGTGT CCTGCTCATC ATCCTGATTG TNCTGCTGGT 60
CGTCTTTCTC CCTCAGAGCA GTGACAGCAG TAGTGCCCCA CGGACCCAGG ATGCAGGCAT 120
TGCCTCAGGG CCTGGGAACT GACCCAGCTG GTCCTGAAGG AGAAGCCAAA TGGCTGCACT 180
GGCCGATTCT GGTCTCCAGA GGACCTTGGT GTTTGCTCTC CCTTGACCCA CCCCAGTNAG 240
TGCCAAAGGG CAGCCCCAAC ATGTGCACCC CTGCATTTCC TGTCATGCCA CAGACTGGCC 300
CTTGAGGGCA GCCTGCTGTA CTGGCCATGC TGGGCCAGCC NCACCTGGAG CTCAGTAAAA 360
ACTGCTGTTT GATTAAA 377

SEQ ID NO:516
SEQUENCE LENGTH:375

SEQUENCE TYPE:nucleic acid
TOPOLOGY:linear
CLONE:HUMGS00577
SEQUENCE DESCRIPTION:
GATCACACCA GTTCAGAGAG CTACACTTTA TGGGTAACAG TTTCACCTGT NATACAGTTC 60
CCTAGAACAT TTTCCCAAAA GTAGTGAACT GCAAAGTGCT TAGGTTTGAC ATTTATTGTA 120
GCAGAACAGT AATATCACAG TATGGGACAA AGGTTTACAC TTTGCAGGGT ATTCTTTGGG 180
GGAATGTTAA ATACTGTAAT AAAAACATGT TCAATCATGG TAAAATGTTC AACTNGTTAA 240
ANTTACAAAT GGNCAAAAAA NATTTTTTTC CTNATATATN GCCTAANTAC CAAATGAAGN 300
GCTTAANCTT AAGNTTCAAT GTGAAANCGA GTAAATNTGT TCCTAAATTT GCAGNAATAA 360
NAGATANCCN GTANN 375

SEQ ID NO:517
SEQUENCE LENGTH:374
SEQUENCE TYPE:nucleic acid
TOPOLOGY:linear
CLONE:HUMGS00578
SEQUENCE DESCRIPTION:
GATCCATGTA CTGTCATGTT TTTTTTCAGG AACAAGCACA TCATGATTGA TTTGGGGACT 60
GGCAACAACA ACAAGATTAA CTGGGCCATG GAGGACAAGC AGGAGATGGT GGACATCATC 120
GAGACGGTGT ACCGCGGGGC CCGCAAAGGC CGCGGCCTGG TGGTGTCCCC CAAGGACTAC 180
TCCACCAAGT ACCGCTACTG AGGCGCCTCA GTCTGCGCGG ATAAATGTCG TGGAGCCCTT 240
TTTGTATGGA AACGTTTTAA GCTATTTAAA GCCTTTGGAA AATACAGGAN GTNCAGGGCT 300
GGAGCACCTC TGAGATGGAA TTGATAACAT GGTCTTAACT CACCGAAATA AACAAGCACG 360
TNGTGAGNGG NAAA 374

SEQ ID NO:518
SEQUENCE LENGTH:374
SEQUENCE TYPE:nucleic acid
TOPOLOGY:linear
CLONE:HUMGS00579
SEQUENCE DESCRIPTION:
GATCTCACCG TGGGTCCGAT TAGCCTTTNC TCTGCCTTGC TTGCTTGAGC TTCAGCGNAA 60
TTCGAAATGG CTGGCGGTAA GGCTGGAAAG GACTCCGGAA AGGCCAAGAC AAAGGCGGTT 120
TCCCGCTCGC AGAGAGCCGG CTTGCAGTTC CCAGTGGGCC GTATTCATCG ACACCTAAAA 180
TCTAGGACGA CCAGTCATGG ACGTGTGGGC GCGACTNCCG CTGTGTACAG CGCANCATCC 240
TGGAGTACCT NACCGCAGAG GTACTTNAAC TNGGCAGGNA AATTGCATCA AAANGACTTA 300
AAGGTNAAAN GGTTTTACCC CTNGGTNANT TGCAACTTTG GTTATTTNGN TGGGGGATGA 360
AGGNTTTGGG TTNN 374

SEQ ID NO:519
SEQUENCE LENGTH:372
SEQUENCE TYPE:nucleic acid
TOPOLOGY:linear
CLONE:HUMGS00580

SEQUENCE DESCRIPTION:

GATCAGAGAG ATTAGGATTG TATTTTGACA TAGGATTTGG AACCCATCTA AATGTTGAAG 60
TTCCCTGAGA CAGCTCTCCA GCTGCTNNGC CTGCGCCAGG GGCTANGCAG CCCCTAATGA 120
GAGGCTCTGC TCCCTTTCCC ACCTCGCCAA TGTTGTTGTT GCTGCCTTTT TGATTTGTAT 180
CCTCTGTTAT AGACATTTTT NAAAAACGAT TTCCTCTTTC ATTGTGCACA AGTGCTGAGA 240
GTCTNAGGCC CCATTTCTGC TGTGTATATA TATCCTGACT CGGGGCTTTT ATTCAGCAAA 300
CTGTTCATTC TTCTGTCAGA CAATGTCATA TTCAACTCTG TTCATATTAA ACCACTTGTN 360
AAGCANTNNA AA 372

SEQ ID NO:520
SEQUENCE LENGTH:371
SEQUENCE TYPE:nucleic acid
TOPOLOGY:linear
CLONE:HUMGS00581
SEQUENCE DESCRIPTION:

GATCTGGTCG CTGCCCCAGG GGGACTGATG GGCAGNGTCG CCCCTGTGGC TGGACTGTNA 60
CCATCCCTGA TGGGGCCTGA CCGCGGGAGC TGAGGAAGCG CCGCTCCACC GTCTGCCCTC 120
CAAGGACCCG CATGGAGGCA GTGGGCTGGC AGCTTCCTGC TGCTCCGTGT NAGAGTCAAA 180
GCACAAATCC TCAGGACGGG CTCAAGGGCC AGGGCAGCCG AGGGAAGTNC AGGTGGGGAC 240
CACGTCTTCC TGAGGTTGGT GCCACTGCTT GGNACCGTTT GCAGTGGGGT GGCCTCCCCT 300
CTGTTTGCNT GGTGNAGNNA GCGTGGCGTG GGGACGTGAC TGAATAAAGC ACCATGGGTG 360
ATGTGTTGAA A 371

SEQ ID NO:521
SEQUENCE LENGTH:382
SEQUENCE TYPE:nucleic acid
TOPOLOGY:linear
CLONE:HUMGS00582
SEQUENCE DESCRIPTION:

GATCTNTGGG ACGTCAGCTG CTGAGAGGAG CAAGCGGTAG TACCACCCCT TAGTTGAGGG 60
AGTCAGCACA GTCCTTTCTG CAGCTTCTAA CCCAGGACCA TGAACTCAGG TGCCTAGAGA 120
AGCCAGGCAG CNNAAGGACA AGGAATGCTG GGGGCTGTGG GAACAGGAAT GCAGATACCC 180
TTTGAAGGAG CATTCCTGCT AAAAGAAGCT GAAAATGTAG ACCTATGTGA AGTGCTCTGA 240
TTTCTAAATA TTGTGAAGGT TAAGAAAAAC ATANATTTTN GGGTCTATGG GCTAGGATTN 300
AGNCCCACAG TTGGCCANTT TNTAGGNGGT NCCCAAATGG ANTGGTTAAA CCNNNGGTTT 360
NGNTTCCTAG CCTAGGGGTA AA 382

SEQ ID NO:522
SEQUENCE LENGTH:382
SEQUENCE TYPE:nucleic acid
TOPOLOGY:linear
CLONE:HUMGS00583
SEQUENCE DESCRIPTION:

GATCAAGAAC AATGCCTCCA CTGACTATNA CCTATCTGAC AAGAGCATCA ACCCTCTGGN 60
TGGCTTTNTC CACTATGGTG AAGTGACCAA TGACTTTNTC ATGCTGAAAG GCTGTGTGGT 120

GGGAACCAAG AAGCGGGTGC TCACCCTCCG CAAGTCCTTG CTGGTGCAGA CGAAGCGGCG 180
GGCTCTGGAG AAGATTGACC TTAAGTTCAT TGACACCACC TCCAAGTTTG GCCATGGCCG 240
NTTCCAGACC ATGGAGGAGA AGAAAGCATT CATGGGACCA CTGAAGAAAG ACCGAATTGC 300
ANAGNGAAGA AGGAGCTTAA TGCCAGGNAC AGATTTTGCA GTTGGTGGNN GTCTCAATTA 360
AGNGTTATTT NNCCACTGGA AA 382

SEQ ID NO:523
SEQUENCE LENGTH:367
SEQUENCE TYPE:nucleic acid
TOPOLOGY:linear
CLONE:HUMGS00584
SEQUENCE DESCRIPTION:
GATCTCCAAG CNAACTCAGC CTCCANCCAA NCTCCCTGTG GGTCCTAGCC ACAAGCTCTC 60
CAACAATTAC TATTGCACTC GCAATGGCCG CCGGGAATCT NTGCCCCCTT CCATCATCAT 120
CNCGTCGCAG AAGTCGCTGG TGTCAGGCAA GCCAGCAGAG AGCTCTGCTG TAGCTGCCAC 180
TGAGAAGAAG NCGGTGACTC CAGCTCCTCC CATAAAGAGG TGGGAGCTGT CCTCGGACCA 240
GCCTTACCNG TNACACTGCA CCCTNACGGC ACCNGACTAC TTTGCCTGCT TGGATTTCCT 300
CCAGGGGAAT GTGACCTAAT TTATGNCAAA TACGTAGAGT CAGGTATCAC TTCTAGTTTA 360
CTNTAAA 367

SEQ ID NO:524
SEQUENCE LENGTH:365
SEQUENCE TYPE:nucleic acid
TOPOLOGY:linear
CLONE:HUMGS00585
SEQUENCE DESCRIPTION:
GATCAAGAGG AAAGAGAACG TAACAACCAG AACCCCTGGA CATTTGAGCG AATAGCCACT 60
GCCAACATGC TGGGCATACG GAAAGTACTG AGCCCATATN ACTTGACCCA CAAGGGGAAA 120
TACTGGGGCA AGTTCTACAT GCCCAAACGT GTGTAGTGAG TGTAGGAGAT AACTGTATAT 180
AGGCTACTGA AAGAAGGATT CTGCATTTCT ATTCCCCTCA GCCTACCCAC TGAAGTCTTT 240
GGGTAGCTCT TAAGCCATAA CTAAGGAGCA GCATTTGAGT AGATTTCTGA AAAACAATGT 300
TATTTGTTGA TTTAAAAAGA AAACTGTATT NTTATTAAAT AAAATTTAAA CATCACTTCA 360
GGAAA 365

SEQ ID NO:525
SEQUENCE LENGTH:396
SEQUENCE TYPE:nucleic acid
TOPOLOGY:linear
CLONE:HUMGS00586
SEQUENCE DESCRIPTION:
GATCTATGGG TTGACTAATT AAACAATAAT TCAAGTAGAG TGTCCCAGAA AAAAACCACT 60
TGGGCTCCCT GTTTGGAGTC TGGCTGGCTC TGAGCATTGC CAATGGCCCC TACTCACCTG 120
ACTTTGTATC CTCTCCTTTT AGAGGCTTTG CATTCTGCAC CCAGCTTCAC TAACAGTGGG 180
CTGAAAACAT CCTTGGGTTG AGTGTTTCAT TTGGGAGTTA TTTGGCCAGG GCCTTTTGAA 240
CAGTAGTGTC CCCATGAAGT GCTAGATAAT ATATGTGTAA GAGTCAGCTT TTTTTTTTTT 300

TAAACTNTAA CACCNTTNAG AANTTTCTAA CTACTTNGNA ACTGNATGGT TTANCCCGGN 360
GNTAAAAGCN GTTTTTAAAA GTNTANGTTT TCCAAA 396

SEQ ID NO:526
SEQUENCE LENGTH:360
SEQUENCE TYPE:nucleic acid
TOPOLOGY:linear
CLONE:HUMGS00589
SEQUENCE DESCRIPTION:
GATCCGCGGG CTTCCACTNC ACCATCGGAT GTTTGCCACT CAGACTGAGG GGGAGCTCAG 60
AGTGACCCAA ATTCTCAAAG AAAAGTTTCC ACGAGCTACA GCTATAAAAG TCACTGACAT 120
TTCAGGCACT AAAAGAAGAA ATCAAAGAGA TGCATGGATT GCGGATATTT ACCTCTGTCC 180
CCAAACGCTG ACCACGGCCT GGCTGCATAG ATGCTGCTGC TTAAGACCTT GGATGAACTT 240
CACTGACATC ATTCTTCCCT AAGCAGTCAC CAAAAAATTT ATATATNTNG CTCATATACA 300
NTNCCATATN ATANTTATAG AAGATGTATA ATCTATTTTA GATGTNANTN AAAGGGTAAA 360

SEQ ID NO:527
SEQUENCE LENGTH:267
SEQUENCE TYPE:nucleic acid
TOPOLOGY:linear
CLONE:HUMGS00590
SEQUENCE DESCRIPTION:
GATCGGGTTT GGCCCCCAGC CCCGCTCACG CCAGTCCCTC TTCCTCTGCC GGGAGGGTGT 60
TTTCAACTCC AAACCCCAGA GAGGGGTTGT AGATTGGGTC CAGCTTTGCT TCAGTGTGTG 120
GAAATNTCTC GTGGGGTGGC ATCGGGGCTG CGGGGTGGGG ACCCCAAGGC TTTCTGGGGC 180
AGACCCTTGT CCTCTGGGAT GATGGGCACT GCTATCCACA GTCTCTGCCA GTTGGTTTTA 240
TTTNGAGGTT TNTGGGCTTT TTTTAAA 267

SEQ ID NO:528
SEQUENCE LENGTH:352
SEQUENCE TYPE:nucleic acid
TOPOLOGY:linear
CLONE:HUMGS00591
SEQUENCE DESCRIPTION:
GATCAATCAC CTTCTGCTGC TTGGATGGGT GGATTTGGTG CTCAGCCTCC CCAAGGACAA 60
GCTCCTCCCC CTGTAATACC TCCTCCTAAC CAAGCCGGAT ATGGTATGGC AAGTTACCAA 120
ACACAGTGAG CCGGGACTCT AAAAAAAAAT TGTAATTCAT GATAGGCTTC GATTTCCTGT 180
GACACTCTGA AGACATGAAA GTAGACATCG GAAAATGNAA ATATTTATTT TAAAAATTGA 240
AATGTTTGGA ACCTTTAGCA CAGATTTGCT TTGGTGAAGG ACACGTGTCT TCTAGTTCTG 300
CCTTTTNAAA GTTTNTTGTT CATGNTGGAT NTTGAACATN GNTTTTTNTT TN 352

SEQ ID NO:529
SEQUENCE LENGTH:351
SEQUENCE TYPE:nucleic acid
TOPOLOGY:linear

CLONE:HUMGS00592
SEQUENCE DESCRIPTION:
GATCAAGCCT TTCTTTCATT CCCTCTCTGA AAAGTATTCC AACGTGATAT TCCTTGAAGT 60
AGATGTGGAT GACTGTCAGG ATGTTGCTTC AGAGTGTGAA GTCAAATGCA TGCCAACATT 120
CCAGTTTTTT AAGAAGGGAC AAAAGGTGGG TGAATTTTCT GGAGCCAATA AGGAAAAGCT 180
TGAAGCCACC ATTAATGAAT TAGTCTAATC ATGTTTTCTG AAAACATAAC CAGCCATTGG 240
CTATTTAAAA CTTGTAATTT TTTTAATTTA CAAAAATATA AAATATGAAG NCATAAACCC 300
AGTTGCCATC TGCGTGACCA ATAAAACATT AATGCTAACC ACTTTTTTAA A 351

SEQ ID NO:530
SEQUENCE LENGTH:348
SEQUENCE TYPE:nucleic acid
TOPOLOGY:linear
CLONE:HUMGS00593
SEQUENCE DESCRIPTION:
GATCCAGAAT ACCCTGACCT CGCCCCAGTT CCAGCAGGCC CTGGGCATGT NCAGCGCAGC 60
TTTGGCCTCG GGGCAGCTGG GCCCCCTCAT GTGCCAGTNC GGTCTGCCTG CAGAGGCTGT 120
GGAGGCCGCC AACANGGGCG ATGTGGAAGC GTTTGCCAAA GCCATGCAGA ACAACGCCAA 180
GCCCGAGCAG AAAGAGGGCG ACACGAAGGA CAAGAAGGAC GAAGAGGAGG ACATGAGCCT 240
GGACTGAGCC ACGCGCCGTC CTCCGAGGAA CTGGGCGNTT GCAGTGCGTT GCACACCTTN 300
ACCTTCNACN TACTGATTAT TAATAAAGTT TTTTCTTTTA CCTGCAAA 348

SEQ ID NO:531
SEQUENCE LENGTH:347
SEQUENCE TYPE:nucleic acid
TOPOLOGY:linear
CLONE:HUMGS00594
SEQUENCE DESCRIPTION:
GATCCTGAAG AGATTGAAAA AGAAGAGCAG GCTGCTGCTG AGAAGGCAGT GACCAAGGAG 60
GAATTTCAGG GTGAATGGAC TGCTCCCGCT CCTGAGTTCA CTGCTACTCA GCCTGAGGTT 120
GCAGACTGGT CTGAAGGTGT ACAGGTGCCC TCTGTGCCTA TTCAGCAATT CCCTACTGAA 180
GACTGGAGCG CTCAGCCTGC CACGGAAGAC TGGTCTGCAG CTCCCACTNG CTCAGGCCAC 240
TGAATGGGTA GGAGCAACCA CTGACTGGTC TTAAGCTGTT CTTGCATAGG CTCTTAAGCA 300
GCATGGAAAA ATGGTTGATG GAAAATAAAC ATCAGTTTCT ATTTAAA 347

SEQ ID NO:532
SEQUENCE LENGTH:346
SEQUENCE TYPE:nucleic acid
TOPOLOGY:linear
CLONE:HUMGS00595
SEQUENCE DESCRIPTION:
GATCCGGTNT TGGTGCCAAT GTCTCCAACT TCACTTTTGC TCCTAGCACG ATTATATTTC 60
ACCTGGGACA TGCTGCTATG CTGGGACTCA TGTATGTCTA CTGGACTCAG CTCAACATGT 120
TCCAGACCTT GAAGTACCTG GCCATCTTGG GCAGTGTGAC GTTTCTGGCT GGCAATCGGA 180
TGCTGGCCCA GCAGGCAGTC AAGAGAACAG CACATTAGTT CCAGAAGAAA GATGGAAATT 240

CTGAAAACTG AATGTCAAGA AAAGGAGTCA AGAACAATTC ACAGTATGAG AAGAAAAATG 300
GAAAAAAAAA CCTTTATTTA AAAANGAAAA AAGTCCAGNT TGTAAA 346

SEQ ID NO:533
SEQUENCE LENGTH:346
SEQUENCE TYPE:nucleic acid
TOPOLOGY:linear
CLONE:HUMGS00596
SEQUENCE DESCRIPTION:
GATCTTGTAG CCTAGATAGG ATAGTNTGAC CTTCTAGCAT AGTCTTTTTG GCAAATNATT 60
TGTGTTTTCA GTGTGTGGGG AAGCTGTCCT GGGGGCTGGG GCGACAGATA GCACATAGGC 120
TGTTTCTGGG GCTGCAGGGG CTTCCNTGAG CTGGATGTTG TGGGTNTTGC CGTGCTTCAG 180
GAAGTNTGGC GACCAGAAAG CGTAGACCCG GGGCCCAGGG TCTGCCCGCC CCTGCAGCNT 240
GGCCTCCCCG CACAGGCTGT GGCTTGCACT CCAGCCGNTC TAGTNTCTNA GGAATTTNCT 300
TGTNACTTGT ACTGTGTAAA TAAAGCTTCC TGGTTCAATA CCNAAA 346

SEQ ID NO:534
SEQUENCE LENGTH:345
SEQUENCE TYPE:nucleic acid
TOPOLOGY:linear
CLONE:HUMGS00597
SEQUENCE DESCRIPTION:
GATCCTATGG CCATGACCCA GAAGTATGAG GNGCATGTNC GGGAGAGCAG GCTCAAGTAG 60
AGAAGGAGGA CTTCAGTGAC ATGGTGGCTG AGCACGCTGC CAAACAGAAG CAAAAAAAAC 120
GGAAAGCTCA GCCCCAGGAC AGCCGTGGGG GCAGCAAGAA ATATAAGGNG TTCAAGTTTT 180
AGGTCCCCTC ACACTAGCCC TTTTTTTGGC CCTACGTCTG GATGCCTGGG CTTCACACAA 240
GAACCACCTC TCCCGCAGTT CCCAAGGNCT TGTCATTTCA TGTTCTTATT TTAGACCTGT 300
TTTGTAAATA AAGCTGTTTC CCAAGGAAAG AGATGAATAT TTAAA 345

SEQ ID NO:535
SEQUENCE LENGTH:354
SEQUENCE TYPE:nucleic acid
TOPOLOGY:linear
CLONE:HUMGS00598
SEQUENCE DESCRIPTION:
GATCGTGTTT ATGGTGAATC CAAGTTGGGA GGAAATGAAA TAGTATCTTT NTTGAAAGGA 60
ATATTGACTC TTTTTGCTAC TACATAAAAG AAAGATACTC ATTTATAGTT ACGTTCATTT 120
CAGGTTAAAC ATGAAAGAAG CCTGGTTACT GATTTGTATA AAATGTACTC TTAAAGTATA 180
AAATATAAGG TAAGGTAAAT TTCATGCATC TTTTTATGAA GACCACCTAT TTTATATTTC 240
AAATTAAATA ATTTTAAAGT TGCTGGCCTA ATGAGCAATG TTCTCAATTT TCGTTTTCAT 300
TTTGCTGTAT TGAGACCTAT AAATAAATGT ATATTNTTTT TTGCATAAAG TAAA 354

SEQ ID NO:536
SEQUENCE LENGTH:343
SEQUENCE TYPE:nucleic acid

TOPOLOGY:linear
CLONE:HUMGS00599
SEQUENCE DESCRIPTION:
GATCCGTGAC AAGCGCACAG GCAAGACCAA GGGCTACGGC TTCGTCAGCT TCAAGGACCC 60
CAGCGACTAC GTGCGCGCCA TGCGTGAGAT GAATGGGAAG TATGTGGGCT CGCNCCCCAT 120
CAAGCTTCGC AAGANCATGT GGAAGGACCG GAATCTGGAC GTGGTCCGCA AGAAGCAGAA 180
GGAAAAGAAG AAGCTGGGCC TGAGATAGGG TCTGTGGCCA GGCACCCGCT CCCACCTGGC 240
CGGGCGCTGG CTCCTCCCTC AGTTCTCTTT GGGAAAACCC CCAGCTNGTC CACCCATCCN 300
NTGCCCCAAA ACCAGTTTCA GTAAATTTAC GTTCATTTCC AAA 343

SEQ ID NO:537
SEQUENCE LENGTH:341
SEQUENCE TYPE:nucleic acid
TOPOLOGY:linear
CLONE:HUMGS00600
SEQUENCE DESCRIPTION:
GATCGAACGG ACTGTGAAAT CCGCTCTTTG TCGGAAGCTG AGCAAGCTGT GGCTTTTTTC 60
CAACTCCGTG TGACGTTTCT AAGTGTAGTG TGGTAGGACC CCGGCGGGTG TGGCAGCAAC 120
TGCCCTGGAG CCCCAGCCCC TGCNTCCATC TGTGCTGTGC GCCCCACAGT AGACGTGCAG 180
ACGTCCCTGA NAGGTTCTTG AAGATGTTTA TTTATATTGT CCTTTTTTAC TGGAAGACGT 240
ACGCATACTC CATCGATGTT GTATTTGCAG TGGCTGAGGA ATTCTTGTAC GCAGTTTTCT 300
TTGGCTTTAC GAAGCCGATT AAAAGACCGT GTGAAATGAA A 341

SEQ ID NO:538
SEQUENCE LENGTH:339
SEQUENCE TYPE:nucleic acid
TOPOLOGY:linear
CLONE:HUMGS00601
SEQUENCE DESCRIPTION:
GATCCTCGGG TGTTCTTCCC ATCAGATAAA ATAGTTCACT ACAGACAGAT GTTTTTATCT 60
ACTGAACTAC AAAGAGTAGA AGAGCTTTAT GATTCATTAT TACAAGCTGT TGCCTTCTAT 120
GANTTAGCAG TGTTTGACTC TCAGCCTTAG AATTCTGAGG TTAACGTGCT AAAGTATAAT 180
TNTTAGCTCT AACGTAACAC CAACTGTTGT GAACATCCAT GTTATTGGAA AAGAACACAT 240
TTTCAGTGTA TTTTAGATGT TTAANTTCTG ACTTTTGGCT ATTAAATGGT TTACACAATA 300
AGCCAAGACC AAATCAATAA ACATTTTNTG AGAACGAAA 339

SEQ ID NO:539
SEQUENCE LENGTH:339
SEQUENCE TYPE:nucleic acid
TOPOLOGY:linear
CLONE:HUMGS00602
SEQUENCE DESCRIPTION:
GATCATCATC CGAGTGCAGA CCACGCCGGA CTACAGCCCC CAGGAAGCCT TTACCAACGC 60
CATCACCGAC CTCATCAGTG AGCTGTCCCT GCTGGAGGAG CGCTTTCGGG TGGCCATAAA 120
AGACAAGCAG GAAGGAATTG AGTAGGGGCC AGAGGGGGCT CTGCTCGGCC TGTGAGCCCC 180

GTTCCTACCT GTGCCTGACC CTCCGCTCCA GGTACCACAC CGAGGAGAGC GGCCGGTCCC 240
AGCCATGGCC CGCNTTGTGG CCACCNCTCA CCCTGACACC GACGTGTCCT GTACATAGAT 300
TAGGTTTTAT ATTCCTAATA AAGTATAGCG GAAGAGAAA 339

SEQ ID NO:540
SEQUENCE LENGTH:339
SEQUENCE TYPE:nucleic acid
TOPOLOGY:linear
CLONE:HUMGS00603
SEQUENCE DESCRIPTION:
GATCGTTATG AATATCAAAT GTCCATTTCT ATNGTAATGA ATTCAGTGGA ACCATCACAC 60
AAAAGCACAC AAAGACCTCC TCCTCCACAG GGGAGACAGA GGTGGGGAGG CTCTCTTGGC 120
TCACATAATC GTGTCTGTGT CACAAATAAT CATTAAATTA GCTATTTTCA GCTAACACAT 180
TTGTNGTTGC ACTTGAAAAA GAGTTAGTGA GCCTGTCTTG GAGTTTAAGT AGTTTCAAAT 240
AAAAAAAGGC TACAGTGCCT CACAAAGGAT GTTCCCAGCA AGTNGTTTAA ATTCCCAGCA 300
AGTTGTTAAA GTGTAAATAN AANTATATGA ANTTGTAAA 339

SEQ ID NO:541
SEQUENCE LENGTH:355
SEQUENCE TYPE:nucleic acid
TOPOLOGY:linear
CLONE:HUMGS00604
SEQUENCE DESCRIPTION:
GATCCTGAAA TCTACTAGAG ACACCCCTAA GCCATGAATG AACTACATCC AAATACCTGA 60
NTTTTTGGAA TCTGTTTCAT GGATTTTNCA TCTTCTACCG TATGTNAAAT TGCAAGTNTT 120
TGAAGATTTA TAAGTACAAA TTTGGGAACA TACAAATCTT TTAGGTAGTA GAGTTTAACG 180
TGTATAAGCT AAAAGTGAAA GTAACTGAGT GTTCTCTTGT TTCTTTGCAT TAATGTAACT 240
GTGTGGTTTG CCTTTGTCCC CCTGGATAGA ACGTGCATTT AAAGAATATA TTGTACTTAC 300
TGTGACAGCA GATAATAAAC CAGTCTCTTG GAGGGCACAA CCCTTATTTG ACAAA 355

SEQ ID NO:542
SEQUENCE LENGTH:331
SEQUENCE TYPE:nucleic acid
TOPOLOGY:linear
CLONE:HUMGS00606
SEQUENCE DESCRIPTION:
GATCTTTTTT ANATAAANNT TATGTATTGT GGCATAATCC TTTTTTTGAG CTCTACAGAG 60
AACAGTCTTT TGGTAATAGT GGCAGGTATT TATTCCTTCT GAATATATAC CCCATTATAG 120
GAATAACTGT TACTTATTTA GGATTCCATC ATTGAAAATT TTNACCCAAG GCACAGCAGT 180
GAANTTTATA GTNCTCANTT TAGTTGNCAT TATTGACAGG CATTGGNATT ATTAGTCATT 240
GCTAAGCAAC TAAAACTTCA TCAGTTCAAN TAAGTTTTAN TTGTCANATG GCNGTATAAN 300
CACATGANCT TTCTAGGAAA TATTCCCTCN N 331

SEQ ID NO:543
SEQUENCE LENGTH:330

SEQUENCE TYPE:nucleic acid
TOPOLOGY:linear
CLONE:HUMGS00607
SEQUENCE DESCRIPTION:
GATCGGTGTG CCCCGCTGCG AGGGGCCCCC CATGGGGCTG NTGGCCCNTC CGCAGTCAGG 60
ACATCCCAAC CCCTGGNTGG GACTGAACCA CCCAGAGCGG AGGGCNTCCC TTTTNAGCCT 120
TGTNAGTCAC CTGGCAGGCC CCAGCTGGGC TGGCTGTCCG TGTCCCTCAG CCTGGNTGGT 180
GATTCCTTGC AGGCCAGAAA TNAAGAGTCC CTGTAGGTTT TGGTTTTGTT TGTTTTATTT 240
TGTTCTTTCA CCTTTTTTCC TCATTAAAAA AAAAANGNCC CTGNGGAGTG TACTNATTNA 300
TTTTTTGATN AAAGGGANGT AAAATGNAAA 330

SEQ ID NO:544
SEQUENCE LENGTH:329
SEQUENCE TYPE:nucleic acid
TOPOLOGY:linear
CLONE:HUMGS00608
SEQUENCE DESCRIPTION:
GATCCAGACT GTAAGATGTT GTTTTAGGGG CTAAAGGGGA GAAACTGAAA GTNTTTTACT 60
CTTTTTCTAA AGTGTTGGTC TTTCTAATGT AGCTATTTTN NTTGTTGCAT CTTTTCTACT 120
TCAGTACACT TGGTGTACTG GGTTAATGGC TAGTACTGTA TTGNCNCTGT GAAAACATAT 180
TTNTGAAAAG AGTATGTAGT GGCTTCTTTT GAACTGTTAG ATGCTGAATA TCTGTCCACT 240
TTTCAATCCC AATTCTGTCC CAATCTTACC AGATGCTACT GGACTTGAAT GGTTAATAAA 300
ACTGCACAGT GCTGTTGGTG GCAGTNAAA 329

SEQ ID NO:545
SEQUENCE LENGTH:329
SEQUENCE TYPE:nucleic acid
TOPOLOGY:linear
CLONE:HUMGS00609
SEQUENCE DESCRIPTION:
GATCACTAGA TTTATGGAGG AATTNGTCAC AAATNACTTN TAGAAAAATG CTGTCATATA 60
GTTCATTTCA TCATTTTCTG TTGCAGGAAG CCACTCCACC ACAGAATGCT AATATGCCAG 120
TGGTACCCAG TACCTCTTGT ATATAGGTTA TTGCAAATAT TGTNCTGAAA TGCTTAACTT 180
CAGAATTACA TTTTTTAAAG TAAATAATTG TTTTAAATCT ATTTTGTAAA GNTATAAAGT 240
ACAATAGAAT TTCTGGAGTA CAGATTAAAC TATTTGCACT AACACACGTG CCGTGCATGA 300
TTTAATAAAA TANCTNNACT CTCCNTAAA 329

SEQ ID NO:546
SEQUENCE LENGTH:328
SEQUENCE TYPE:nucleic acid
TOPOLOGY:linear
CLONE:HUMGS00610
SEQUENCE DESCRIPTION:
GATCACCGTG TGCTCAGGCC AGGTGTGAAT CCTGAGGTCC ATGGAGGTGC AGAGATGAGA 60
TTACTCCTAT TCACGTTGAA GTGATTTGCT TTGTTAACAA AAAATTGCAG CTATTGTCTA 120

GCTTTCATTT TTTTACTGAG AACTTTAAAT TAGTCCCCTA TTAGAATAGG GTTGCTACTC 180
ANCTNTTTTN AAAAACCGAA TTTCATCATT TATCTAAAGA GNAAATATGC AGANTAACTG 240
GTCTTGTTAA GAGTGCAATA TTATATNNNN ANGTAAAACT AAAANTNAAT TTGGGGGGAT 300
TATTTATNCA GCATGANACC TANTNTGN 328

SEQ ID NO:547
SEQUENCE LENGTH:328
SEQUENCE TYPE:nucleic acid
TOPOLOGY:linear
CLONE:HUMGS00611
SEQUENCE DESCRIPTION:
GATCCCNGGG ACGTGAGACT TANNCTTCCA GCCAGTGTGA ATCATTGTAT TTTGTCTCAT 60
AATCACAGCA CNCCTGCATG ACACAACAAC GTGCAGCATT TTTTACATAA AAATATGGTA 120
GANTTAATTT ATGACATGGA AATGCCTTAC GTGGTATCAC ACTTAGTCTT GAAAAAAACA 180
CCNAGGTGAC GTTTAAAATT TTTAGTACAT ATCCTCAAAT TGGAGCTAAG TTATACTTCT 240
TTTATAACCT TTTGGGCATC TGGTCGAGAG AAGACAAGAT TTTNTCTATT TACAGTGATG 300
CAATAAATAT GTTTGCCACC TTTGGAAA 328

SEQ ID NO:548
SEQUENCE LENGTH:322
SEQUENCE TYPE:nucleic acid
TOPOLOGY:linear
CLONE:HUMGS00612
SEQUENCE DESCRIPTION:
GATCTTGAAG AGACCGCTGG CAGCACCAGT ATTCCCAAGA GGAAGAAGTC TACACCCAAG 60
GAGGAAACAG TTAATNACCC TNAGGAGGCA GCCACAGAAG TGGCTCCAAG AAAAANAGGA 120
ATTNTCCAAA GAGGAGCCGG TCAGCAGTGG NCCTGAAAGA GGCGGCTGGC AAGANCAGCT 180
CCAAGAAGAA GAAAATGTTC CATAAAGCAT CCCAGGANGA TTAGAATCAA ATGGACANTC 240
TCTNGGAGGT GGGNATACCA TAGNCCAAGG TNCATTTCCC ACCNTGTGCC GTGTTCCCAA 300
TAAANACAAA TTCACAAGGA AA 322

SEQ ID NO:549
SEQUENCE LENGTH:318
SEQUENCE TYPE:nucleic acid
TOPOLOGY:linear
CLONE:HUMGS00614
SEQUENCE DESCRIPTION:
GATCCACCTA GCCTCAGCCT CCCAAAGTGC CGCCGGGATT ACAGGCGTNA ACACCATGAC 60
TGGCCTTCAT TATCTCTNTT TTAAAAATGA AAAAGTTTAT AATTTACATT CAGTAAAATC 120
ACCCTTTTTA GTGTCTAGTC TGTGAATTTT GACAAATGCA TGGTTTTGTA ACCAATCGAT 180
AGGNCAGTTC TGCCACCCAG GACATTCCCN TCTGTTCCTC TGTTCCTCTC TTCTCCTGCC 240
CCCTAGCAAC CACTGGTGTT TTCTGTCCNT CTGGTTCATT TGACATTTAT TTTAAAATAA 300
AATATTTTAA AATCTAAA 318

SEQ ID NO:550

SEQUENCE LENGTH:318
SEQUENCE TYPE:nucleic acid
TOPOLOGY:linear
CLONE:HUMGS00615
SEQUENCE DESCRIPTION:
GATCCTCACC GTGGAGGACC ATNATAATNA AGGTGGCATT GGTGAGGCTG TGTCCAGTGC 60
AGTAGTGGGC GAGCCTGGCA TCACTGTCAC CCACCTGGCA GTTAANCGGG TACCAAGAAG 120
TGGGAAGCCG GCTGAGCTGC TGAAGATGTT TGGTATCGAC AGGGATGCCA TTGCACAAGC 180
TGTGAGGGGC CTCATNACCA AGGCCTAGGG CGGGTATGAA GTGTGGGGCG GGGGTCTATA 240
CATTCCTGAG NTTCTGGGAA AGGTGCTCAA AGATGTACTG AGAGGAGGGG TAAATATATG 300
TTTTGNGNAN AATGCAAA 318

SEQ ID NO:551
SEQUENCE LENGTH:314
SEQUENCE TYPE:nucleic acid
TOPOLOGY:linear
CLONE:HUMGS00616
SEQUENCE DESCRIPTION:
GATCCCCTCT CTGAAGAGAA AGGAGGAAAG AAAAGAAAAA AACAGAAACA GAAGCTCCTG 60
TTCAGCACCT CAGTCGTCCA CACCAAGTGA CACTACTGGN CCAGGCTACC TTCTCCATCT 120
GGTTTTTNTT TTTTNTTTTT TTTCCCCCAT GCTTTTGTTT GGNTGCTGTA ATTTTAAAGT 180
ATTTGAGTTT GANCAGATTA GCTCTGGGGG GAGGGGGTTT CCACAATGTG AGGGGGAACC 240
AAGAAAATTT TAAATACAGT GTATTTTCCA GCTTCCTGTC TTTACACCAA AATAAAGTAT 300
TGACACAAGA GAAA 314

SEQ ID NO:552
SEQUENCE LENGTH:313
SEQUENCE TYPE:nucleic acid
TOPOLOGY:linear
CLONE:HUMGS00617
SEQUENCE DESCRIPTION:
GATCCCTACT TGGNAGTTAA CCCTAACTAC TTGCTCGAAG ATTGAGATAG TGAAAGTAAC 60
TGACCAGAGC TGAGGAACTG TGGCACAGCA CCTCGTGGCC TGGAGCCNGG CTGGAGCTCT 120
GCTAGGGACA GAAGTNTTNC TGGAAGNNAT NCTTCCAGGA TTTNTTTTTC AGAAACAAGA 180
ATTGAGTTGA TGGTCCTATG TNTCACATTC ATCACAGGTT TCATACCAAC ACAGGCTTCA 240
GCACTTCCTT NGGTGTGTTC CTNTCCCAGT GAAGNTGGAA CCAAATAATG TGTAGTCTCT 300
ATANCCANTA CCN 313

SEQ ID NO:553
SEQUENCE LENGTH:310
SEQUENCE TYPE:nucleic acid
TOPOLOGY:linear
CLONE:HUMGS00618
SEQUENCE DESCRIPTION:
GATCTTAGCT AGTTCATCAC TTCCTCAGGG AAACATTCCC TAATTTCTGT CACAGAGTAA 60

GACCCGTTGT TATACGTCTC ACCTGACCAT GTACCTCTAC TTAGCATTTA ACACGTGTAA 120
TNTTGTATGT ATTGGTTTGA TTAATGTCAG ANCCACTAGA TTGTAAACTC CATGAGGACC 180
GGAATTTNGT TTCTGTCATT GTCATTGTTC AACTTTGTAT TTTTNATGCC TCATACAGTG 240
CCTGGCACAT AGTAGGTGCT CAATTAACTT NATTGGAAAG AATAAAATGA ATGGATGAGG 300
TATCAAGAAA 310

SEQ ID NO:554
SEQUENCE LENGTH:310
SEQUENCE TYPE:nucleic acid
TOPOLOGY:linear
CLONE:HUMGS00619
SEQUENCE DESCRIPTION:
GATCTGGGGG CCACCACCCT GTGCCGGTGG CCTCTGGGCT GCCTCCCGTG GTGTNAGGGC 60
GGGGCTGGTG CTCATGGCAC TTCCTCCTTG CTCCCACCCC TGGCAGCAGG GAAGGGCTTT 120
GCCTGACAAC ACCCAGCTTT ATGTAAATAT TCTGCAGTTG TNACTTAGGA AGCCTGGGGA 180
GGGCAGGGGT GCCCCATGGC TCCCAGACTC TNTCTGTGCC GAGTGTATTA TAAAATCGTG 240
GGGNAGATGC CCGGCCTGGN ATGCTGTTTG GAGACGGAAT AAATGNTTTC TCATTCAGTC 300
TNCAGTCAAA 310

SEQ ID NO:555
SEQUENCE LENGTH:308
SEQUENCE TYPE:nucleic acid
TOPOLOGY:linear
CLONE:HUMGS00620
SEQUENCE DESCRIPTION:
GATCCNGTGC TGAAAGAGAA ACCAAGAAAA AAGATGACAT TCCAGAAGAA GACAAAGGAA 60
ATGTAAAACA ATGTGAAATC AATTATGTAA AGAAATTTCA GAGCTTCCAA GACCACAAAC 120
TTAAAATAAG TAAAGANGAC AGTAAAATTN TTAAAAAGGC TCGGAAAGAT GGATTTTTGC 180
ATGAGACGCT TCTGGACAGG NGAGCCAAAT TGAAAGCCGA CAGATACTGC AAGTNACTGG 240
GATTTTTNTT TCTGCCTTAT CTTNCTGTNG TTTTTCTGA NTAAAATATT CAGAGGAATG 300
CTTTTAAA 308

SEQ ID NO:556
SEQUENCE LENGTH:300
SEQUENCE TYPE:nucleic acid
TOPOLOGY:linear
CLONE:HUMGS00621
SEQUENCE DESCRIPTION:
GATCTTCCTG AAGTNTCTCC CCGTTTNGTG CAGCTGGCCA TACCCCAGGT GGACATNATG 60
AGTCAGGCTG ACTTAATTNC TCATGAGCAG ACCATCCCAG TGAATGCAAA GGGCATGGGC 120
TCCACAGCCT GGACCCTGGC ATGGAGTCCA GCNNCCTGCT CACCGGCCAG GAGGCCTGGG 180
GGCGGGTCAC TTACCCTTNT NAGCCTCANT TTCTNTTTCT GGAAGCGGAG ATGGTAATAG 240
CTTTNACATT NGAGGTGAAT GTNAGAATTA AACTTGGGCA CATGGAGGAA TACACCTAAA 300

SEQ ID NO:557

SEQUENCE LENGTH:299
SEQUENCE TYPE:nucleic acid
TOPOLOGY:linear
CLONE:HUMGS00622
SEQUENCE DESCRIPTION:
GATCACTTGT CCTATTACCC TATACCTAGC ACTTGTGACA CCACCCCTAA ATCACTTTGA 60
GCCTGGGAAA TAAGCCCCCT CAACTACCAT TCCTTCTTTA AACACTCTTC AGAGAAATCT 120
NCATTCTATT TCTNATGTAT AAAACTAGGA ATCCTCCAAC CAGGCTCCTG TGATAGAGTT 180
CTTTTAAGCC CAAGATTTTT TATTTGAGGG TTTTTTGTTT TTTAAAAAAA AATTGAACAA 240
AGACTACTAA TGACTTTGTT TGAATTATCC ACATGAAAAT AAAGAGCCAT AGTTTCAAA 299

SEQ ID NO:558
SEQUENCE LENGTH:301
SEQUENCE TYPE:nucleic acid
TOPOLOGY:linear
CLONE:HUMGS00623
SEQUENCE DESCRIPTION:
GATCTGCTTC TCCAGTTTTT GAGGAGCCAG CCAGGGGTCC AGCACAGCCC TACCCCGCCC 60
CAGTATCATG CGATGGTCCC CCACACCGGT TCCCTGAACC CCTCTTGGAT TAAGGAAGAC 120
TGAAGACTAG CCCCTTTTTC TGGGGAATAA CTTTCCTCCT CCCTGTGTTA ACTGGGGCTG 180
TTGGGGACAG TGCGTGATTT CTCAGTGATT TCCTACAGTG TTGTTCCCTC CCTNAAGGCT 240
GGGAGGGTGN TAAACACCAA CCCAGGANTT CTCAATAANT TTTTNATTAC TAAACCTGAA 300
A 301

SEQ ID NO:559
SEQUENCE LENGTH:297
SEQUENCE TYPE:nucleic acid
TOPOLOGY:linear
CLONE:HUMGS00624
SEQUENCE DESCRIPTION:
GATCAGATTT TGCTAAATGG AAATAATATA ACAATGCTGG TTCCTGGAGG AGAAGGACCT 60
GAAGTGTGAA TGAGTTTCCT TGACTTACAC TAGATTTTGT TTTGGCTTAT AATGACAAGA 120
AAATGGAATT TTTTTTTCCC ACTTTCTAAT GTTTAAATCC CATAAAGCTA AGTTTCCCGT 180
TAAAGGGAAG TGCTTTGAAG ATGTGTACCC ATTTTTGTAA GTTAATCATG ATTATCCTGG 240
AAAAAGAAGA AAAGAGCTTC TTCTTTGCAG ATGAAAATAA AGGTGTTTTT GGTTAAA 297

SEQ ID NO:560
SEQUENCE LENGTH:304
SEQUENCE TYPE:nucleic acid
TOPOLOGY:linear
CLONE:HUMGS00625
SEQUENCE DESCRIPTION:
GATCTATTTG TAGATTAGGA TTAAAATGGA TTTAATCCAT TTTTAAGGCT GTGTGAATTT 60
TTCTAAACAA GAACCATTTG CAATATGGAT TTCTTAGAGA TTAAACCAAT TATAACTTAT 120
TAGCAGTCGC GAGCACATGT TCATATAGTC AATGTAAAAA TACACTAATG AGTATTTGGT 180

AAATCCCAGT AGGCTTTTAC CATTAGCATA ATTTTGTGTT GTACAATTAA GTTACAATTA 240
CATCTCTAAT TTTGGATAAT ATTCATTGGT TAACANTANA GTGACAAAAG CTCATGCCTT 300
CAAA 304

SEQ ID NO:561
SEQUENCE LENGTH:301
SEQUENCE TYPE:nucleic acid
TOPOLOGY:linear
CLONE:HUMGS00626
SEQUENCE DESCRIPTION:
GATCTGGAAC TTTGCACATG TCACTACTGG GGAGGTGTTC CTGCTCTAGC TTCCACGATG 60
AGGCGCCCTC TTTACCTATC CTCTCAATCA CTACTCTTCT TGAAGCACTA TTATTTATTC 120
TTCCGCTGTC TGCCTGCAGC AGTACTACTG TCAACATAGT GTAAATGGTT CTCAAAAGCT 180
TACCAGTGTG GACTTGGTGT TAGCCACGCT GTTTACTCAT ACAGTACGTG TCCTGTTTTT 240
AAAATATACA ATTATTCTTA AAAATAAATT AAAATCTGTA TACTTACATT TCAAAAAGAA 300
A 301

SEQ ID NO:562
SEQUENCE LENGTH:294
SEQUENCE TYPE:nucleic acid
TOPOLOGY:linear
CLONE:HUMGS00627
SEQUENCE DESCRIPTION:
GATCTNAGGA GCTTTAGGGA GAAGACTTGG TGGGGCTGGA GCACACCTTG GGNCTCANTG 60
GTTTCTGTGT CCCNGTGGTG CCANTCCTTC TGGGCAGTGC AGGCGGCTGC CAGGCCCAGC 120
CCTGACTTCC ACTCTGGCTC AGCAACCTGG TTATTTATGT GGGGCCGTGC AGGCATGGGC 180
CCACTGCCTG TCCATCCTGT TTCTNTTATA AATTGAAACT CACCATTGCC CTATCCTTGT 240
GTCTCCACCC GCTTCCATGT GTTGAATAAT AAAAGGTGGG AAAGTGCTGT CAAA 294

SEQ ID NO:563
SEQUENCE LENGTH:296
SEQUENCE TYPE:nucleic acid
TOPOLOGY:linear
CLONE:HUMGS00628
SEQUENCE DESCRIPTION:
GATCTGCCAT AAGAAAATCT AGTTCAACTC TAATTTTATG TAGTAAATAA ATTGGCAGGT 60
AATTGTTTTT ACAAAGAATC CACCTGACTT CCCCTAATGC ATTAAAAATA TTTTTATTTA 120
AATAACTTTA TTTATAACTT TTAGAAACAT GTAGTATTGT TTAAACATCA TTTGTTCTTC 180
AGTATTTTTC ATTTGGAAGT CCAATAGGGC AAATTGAATG AAGTATTATT ATCTGTCTCT 240
TGTAGTACAA TGTATCCAAC AGACACTCAA TAAACTTTTT GGTTGTTAAA CTGAAA 296

SEQ ID NO:564
SEQUENCE LENGTH:307
SEQUENCE TYPE:nucleic acid
TOPOLOGY:linear

CLONE:HUMGS00629
SEQUENCE DESCRIPTION:
GATCGACTAC AGAGTACTTN TTTCTTATGA TGATTGGTGT AGAAATGTGT GATTTGGGTG 60
GGCTTTTACA TCTTGCCTAC CATTGCATGA AACATTGGGG TTTCTTCAAA ATGTGTGTGT 120
CATACTTCTT TTGGGAGGGG GGTTGTTTTC TTCTGTTTAT TTTCTGAGAC TCCTACAGGA 180
GCCAAATTTG TAATTTAGAG ACACTNAANT TTGTTAATCC TGTCTGGGAC ACTTAAGTAA 240
CATCTAAAGC ATTATTGCTT TAGAATNNNC AAATAAAATT TTCCTGACCA AATTGTTTTG 300
TGGGAAA 307

SEQ ID NO:565
SEQUENCE LENGTH:296
SEQUENCE TYPE:nucleic acid
TOPOLOGY:linear
CLONE:HUMGS00630
SEQUENCE DESCRIPTION:
GATCTTGCTC CTTCAGACTC TGACCTGAGT GGAGACCTTT CCACCAGACA CAGCTCGGGC 60
CTGTGTAATT GTAGGAGAAG ACACTCAGCA GTGATTGCCA TGGCACAGAG CCGTGGTCAT 120
TGTTGCTGTT ACAAAGAAGA AAACCATCTG AGTTCTAACT CCTTGGTTGC TTAAAAGTAG 180
TTCCCAAGAG TCTGAGAAGC TATTTCTATT TTTAAGAGTC ATTTTTTGTA ATNTTTGTAA 240
NACAAAAGTA CCAATCTGTT TTGTAAATAA AANTCATCCT AAAATTCGAN GTTAAA 296

SEQ ID NO:566
SEQUENCE LENGTH:288
SEQUENCE TYPE:nucleic acid
TOPOLOGY:linear
CLONE:HUMGS00631
SEQUENCE DESCRIPTION:
GATCTTTNGC TAGGTGGATG ACTAGTNATA TTCAAAGCCT TTTCTCAAAG CCCTTTCAGT 60
TACAACCACC CCACTATGGA ATCAGTATTT AGTTATACAT TTGTATAAGA NCCTGTATTT 120
TGAAAAACAC ATTCATGTAT ATTTATTCCT GGAATTATTT GCCTGTTAAA CAGTGTCTTT 180
CATGTTCTCT CCCCAGATTG TAAACTCTGT AAGAAGCTGC TNGTATCTGT ATCCCTTGTT 240
GAAACTCTGA AAACACTGAA TAACTAAANT CTTCTTCTCA TCCNTAAA 288

SEQ ID NO:567
SEQUENCE LENGTH:292
SEQUENCE TYPE:nucleic acid
TOPOLOGY:linear
CLONE:HUMGS00632
SEQUENCE DESCRIPTION:
GATCTTGATA TTCTGTACAA GTTGATGTAA TACCCTGATG CGTTTTAGAG GACTTGGCAT 60
AAAATNAAAG NTTGGCAAAG GCCCTTGAGG GGCTTGGGGA TGAGAGTATG GAACTGTCTG 120
CATTGGACCC TAAACTGGAC TAGANGAGGC ATCTTCAAGG TTCATACGTT GTCCAGCTGT 180
AAGTTCATTT GAGTAGCAGA GCTAACAAAT ATTTGAGGTC AAAACCCTAC CATGTTAAAA 240
CAAACAAAAA CTTATCATGT TAATAAAAGT ATTCATTTGC TTGANANANA AA 292

SEQ ID NO:568
SEQUENCE LENGTH:291
SEQUENCE TYPE:nucleic acid
TOPOLOGY:linear
CLONE:HUMGS00633
SEQUENCE DESCRIPTION:
GATCCTGACA AGAAGAAAAT NAAGCTCAAA GTCAAAAAAT CTCGTGAAAA ACGGAGTTTG 60
GCCTCTCATC TCAGTGGATA TATCCCTNCC AAAAGGAAAC AAGGGCAAGG CTTATCTTTG 120
TGTCAAAACG GAGAGTCACC CAACTGTGTG GAAGACAAGA TGCTCTCGAC AGTTGCAGTA 180
CTTACCCTTG GCTAAGAACT GCACTGCTTT GTTTAAAGGN CTGCAGACCA AGGAGCGAGC 240
TTTCTCTCAG AGCATGCTTT TCTTTATTAA AATTACTGAT GCAGANAAAA A 291

SEQ ID NO:569
SEQUENCE LENGTH:285
SEQUENCE TYPE:nucleic acid
TOPOLOGY:linear
CLONE:HUMGS00634
SEQUENCE DESCRIPTION:
GATCANGCTG GCTGCAAAGA AGGGACTGGA CCCATACAAT GTACTGGCCC CAAAGGGAGC 60
TTCAGGCACC AGGGAAGACC CTAATTTAGT CCCCTCCATC TCCAACAAGA GAATAGTAGG 120
CTGCATCTNT NAAGAGGACA ATACCAGCGT CGTCTGGTTT TGGCTGCACA AAGGCGANGC 180
CCAGCNNTGC CCCCGCTNGT GGAGCCCATT ACAAGCTNGT NCCCCAGCAN CCTGGCACAN 240
TGAGGNACCT GCACTAAATT ACTNAAAATG TGCTGTAAAG NTTTN 285

SEQ ID NO:570
SEQUENCE LENGTH:285
SEQUENCE TYPE:nucleic acid
TOPOLOGY:linear
CLONE:HUMGS00635
SEQUENCE DESCRIPTION:
GATCACCCTT GTAATGTGTT ACGGGTCCAT TTTTCCTGGA ATCGTTTAAT CTAAAGCAGT 60
TTCCCCTGTT TTGGAGATTT TNTAGTTAAT TTTAATTTTG GCTATTGTTT GGAAAAGATG 120
AGCTGTCTGT GTAGATATGA AGTATAGTTT TTNCCATAAA ACAGATGTTT ATTTTGTATT 180
AAAAAATACC ACTGTACTTG TTTTACACCA TTTGTATACA TGTGGTGATA TTAATGCTAA 240
ACTGTAAAAT TCAGGAATTA AAATGTGACC CTGTAATTCC ATAAA 285

SEQ ID NO:571
SEQUENCE LENGTH:287
SEQUENCE TYPE:nucleic acid
TOPOLOGY:linear
CLONE:HUMGS00636
SEQUENCE DESCRIPTION:
GATCTAACCA TTTTCATACT CTTAACTGAT TGAAACAGAT TCAAAGAAGT ATCGAGTGCT 60
ATGCATTGAA ACTTGTTTTT AAATGTTAGA TGGCACTATG TATATTAATG TAAANCAATG 120
TTAATTTACT CAAGTTTTCA GTTTGTACCG CCTGGTATGT CTGTGTAAGA NGCCAATTTT 180

NGTGTATTGT NACAGTTTCA GGTNATTNAT ATTCGATGTT TTGTAAANCT CAAATANCGA 240
CTATACTNAT GGGNCCAAAT AAATGGGCAT CTGCATTCTN GGTTAAA 287

SEQ ID NO:572
SEQUENCE LENGTH:282
SEQUENCE TYPE:nucleic acid
TOPOLOGY:linear
CLONE:HUMGS00637
SEQUENCE DESCRIPTION:
GATCTNTGGT GGCAATGNCT GACCAGTAGA CTGGTGGCTC ACTTCTNCCC ACCTGCCGGC 60
AACACCAGTG CCAGGAAAAG GCCAAAAGAA TGTNTGTTTC TAACAAATCC ACAAATAGCC 120
CCGAGATTCA CCGTCCTAGA GCTTAGGNCT GTTTTCCACC CCTCCTGACC CGTATAGTGT 180
GCCACAGGAC CTGGGTCGGT CTAGAACTCT CTCAGGATGC CTTTTCTACC CNATCCCTCA 240
CAGCCTCTTN CTGCTAAAAT AGATGTTTCA TTTTTNTGGA AA 282

SEQ ID NO:573
SEQUENCE LENGTH:279
SEQUENCE TYPE:nucleic acid
TOPOLOGY:linear
CLONE:HUMGS00638
SEQUENCE DESCRIPTION:
GATCTACTGG CGAGCGATGA AAATGTTGCA GGGAGAGTCA GCAGAGGCAT TTGTAGCTAA 60
ACATGCTATG CATCAGACTG GCCATTTATG AAGATGAAGA ATACAGTCAG CTTTGTGAAA 120
TAGTATTGCA AGCAAGCCCC GTGGGCAAAT TTGTATTGAG TCCATCTGTA ATTTGCTCAG 180
TGATGGCAGA CAAGATGGCT GTCTGGTTTT GAGACACACT TTAATTTTAT GTTAACTTGT 240
TAAATCTTTT TAAAAATTAA AAAATTTTTA TGATTGAAA 279

SEQ ID NO:574
SEQUENCE LENGTH:279
SEQUENCE TYPE:nucleic acid
TOPOLOGY:linear
CLONE:HUMGS00639
SEQUENCE DESCRIPTION:
GATCATTGCA TTTTCCTGTA TTTTCTAAAA TGGCTCCAAT TTTGTNTTTT AAGCTTCAGC 60
TTAAGAGGAA GTTTATGTTC TAATTCTTGA CTGAGAATAC AGTATTGAGA TTCTNTGTTT 120
TACAGATAAC AACTGGTTTT TATTACTCAT TAAGTTCATT TGCATCCCGT AGCCCTCTGT 180
AAATGTTTCC CCTAGTTGTA TGTACGTAAA TGCACGCTTA TCCAGTNTAT ATTAGACATT 240
TTTGTGCTAA AATATATTAA GTGGGATTTT TGTAGCAAA 279

SEQ ID NO:575
SEQUENCE LENGTH:280
SEQUENCE TYPE:nucleic acid
TOPOLOGY:linear
CLONE:HUMGS00640
SEQUENCE DESCRIPTION:

GATCTTTGCA AGGGCAAAAC TACAAGTAAC GAGTTTTATA TAATTAATTT AAATTTNTNA 60
CAGGTTTTCA TGTTCAGGAT AAACCATACT TCCACCTTGG GTGAGAACAC TTGCAACAGT 120
TTATTAATGA GGTGACTTTC ACCTTAGGAC AACTGTTGCA TGCCAAGTTT TTTGTGTGTG 180
TGAAACACTN TCAAAACTGA TTTAAAAGAT GTAAATTTAA AATTGGTTGT ATCTAATATG 240
CCCCAGGTTC GGTAAATAAA CAATTCTTTT TAAAAACAAA 280

SEQ ID NO:576
SEQUENCE LENGTH:300
SEQUENCE TYPE:nucleic acid
TOPOLOGY:linear
CLONE:HUMGS00641
SEQUENCE DESCRIPTION:
GATCTGCCTG TCCCTTTTTC CCCTGGGGTT TGACACACAG GCTCCTCTCA GCATGAGGTG 60
GAGCAGTGAC CAGGTGGAGC AGTGACCAGG ACGCCTCTGG CCCAGTGCTG CCCAGCCTCC 120
CCGNCCGCTC CCAGGCGCCC CATGTCCTCA CAGGCCAGGA CGCCATGNCA GGATGGAGAG 180
GACTTGGTGG ATTTTTGTTT CTTGCCTGAC CTCAGTTTCA TGAAAGAAAG TGGAAGCTAC 240
AGAATTATTT TCTAAAATAA AGGCTGAATT GTCTGAAAAA TAAAATATAT TTGTATTAAA 300

SEQ ID NO:577
SEQUENCE LENGTH:278
SEQUENCE TYPE:nucleic acid
TOPOLOGY:linear
CLONE:HUMGS00642
SEQUENCE DESCRIPTION:
GATCTGGGAC GTGGTTCGGC GGANAGTCCC CAGCCCGGCC CCCTGCCTGG GACCACCAGG 60
CCCCCAGGAG AAGCCGCCTG AGCCACAACC TTGCGGCATG CAAATNAGAT GGCCGCTCCA 120
GGCCTGGAAT GTTCCGTGGC TGGGCCCCAC GGGAAGCCTG ATGTTCAGGG TTGGGGTGGG 180
ACGGGCAGCG GTGGGGCACA CCCATTCCAC ATGCAAAGGG CAGAAGCAAA CCCAGTAAAA 240
TGTTAACTGA CTTCCAGCCT CACCCGTGGG CGGTCAAA 278

SEQ ID NO:578
SEQUENCE LENGTH:277
SEQUENCE TYPE:nucleic acid
TOPOLOGY:linear
CLONE:HUMGS00643
SEQUENCE DESCRIPTION:
GATCAGGGCC CACTGATGAT GGTGAAGAAG AGATGGAAGA AGACACAGTC ACAAACGGGT 60
CCTGAGCAGT GAGGCAGATG TATAATAATA GGCCCTCTTG GAACAAGTNT TGCTTTTNGA 120
ACATGGTATA ATAGCCTTGT TTGTNTTAGC AAAGTGGAAT CTATCAGCAT TGTTGAAATG 180
CTTAAGGCTG CTGCTGATAA TTTNNTAATA TAAGTTTTGA AATCNAAATG TCAATTTNCT 240
ACAAATNATA AAAATAAACT CCACTCACNA TGCTAAA 277

SEQ ID NO:579
SEQUENCE LENGTH:277
SEQUENCE TYPE:nucleic acid

TOPOLOGY:linear
CLONE:HUMGS00644
SEQUENCE DESCRIPTION:
GATCCGAGTC GTCCGGAAAT CCATTTGCCC GTNTTCTCAC AGTTATTAAC CAGACTCAGA 60
AAGAAAACCT CAGGAAATTC TACAAGGGCA AGAAGTACAA GCCCCTGGAC CTGCGGCCTA 120
AGAAGACACG TGCCATGCGC CGCCGGCTCA ACAAGCACGA GGAGAACCTG AAGACCAAGA 180
AGCAGCAGCG GAAGGAGCGG CTGTACCCGC TGCGGAAGTA CGCGGTCAAG GCCTGAGGGG 240
CGCATTGTCA ATAAAGCACA GCTGGCTGAG ACTGAAA 277

SEQ ID NO:580
SEQUENCE LENGTH:276
SEQUENCE TYPE:nucleic acid
TOPOLOGY:linear
CLONE:HUMGS00645
SEQUENCE DESCRIPTION:
GATCAGGACC CACCTNCAGT NCTTCTGAAA GTGTGACAGT GTCCAGCCGG TTCTGCAGCA 60
CTAGGGGAGG GGGCAGATGG TGGTTGCATG GGCTTCCTGG GTCTCCACTC TCCGTCTGGC 120
CTAAAGGTGA TGTATTTGGT GTTTGGCCCT GCAGTCCCCA CTCTTGAGGC TTAAGGCGCA 180
TGTGGCACAN CACTNCTTCC AGCAGTAGTC GCTTTACTGT TACCNGTTTA GGCCTAGAAG 240
TTTTCCNNCA TCTGTAAATG TGATTTAAAA TNTAAA 276

SEQ ID NO:581
SEQUENCE LENGTH:275
SEQUENCE TYPE:nucleic acid
TOPOLOGY:linear
CLONE:HUMGS00646
SEQUENCE DESCRIPTION:
GATCTACTTA CTCAAGTCTN ATGAATNCTG NGCCTTTCAT CACATTCCTA GCCCACTCTC 60
ATCATTACTG CAGAAGGGTG TTGTGATGAC CAGTNTTATA CTGTGTTTTG ATATGTCTAG 120
CAATAACTTA AAGAAAAAAA AACCTGGGAA ATCTTCAACA TGNNNTNGGA ACATATATGT 180
ATGTATTAAT GNATATACAT GGCTTAACTT ATACGGTTAT GGCAGCNCCT GTATACAGTT 240
TGAACTCATG NACCTGAAAA ANAATTCTTA ANTTN 275

SEQ ID NO:582
SEQUENCE LENGTH:307
SEQUENCE TYPE:nucleic acid
TOPOLOGY:linear
CLONE:HUMGS00647
SEQUENCE DESCRIPTION:
GATCGCCATC ATGAACGACA CCGTAACTAT CCGCACTAGA AAGTTCATGA CCAACCGACT 60
ACTTCAGAGG AAACAAATGG TCATTGATGT CCTTCACCCC GGGAAGGCGA CAGTGCCTAA 120
GACAGAAATT CGGGAAAAAC TAGCCAAAAT GTACAAGACC ACACCGGATG TCATCTTTGT 180
ATTTGGNTTC AGAACTCATT TTGGTGGTGG CAAGACAACT GGCTTTGGCA TGATTTATGA 240
TTCCCTGGNT TATGCAAAGA AAANTGGAAC CCNAACATAG NCTTGCAAGT CATGGCCTGT 300
ATGNGGN 307

SEQ ID NO:583
SEQUENCE LENGTH:272
SEQUENCE TYPE:nucleic acid
TOPOLOGY:linear
CLONE:HUMGS00648
SEQUENCE DESCRIPTION:
GATCTGAATT TTTTCCTCCT TTTGGTTTTA TTTTGTTGGT TTATTTTGTG TTTTCTTTTC 60
TCCTTTTTGG GGGGTATTCA GAGTGGGCTG GGCCCCTGGG CGAGACACAG CTACCTCTGT 120
TGGCATCTTT TTAATACCAG GAACCCAGCG GCTCTAGCCA CTGAGCGGCT AAATGAAATA 180
AAGTGGAAAA AAAAAAANGG GAAAAACCCA AAGGNTTAAA AACCCACNGG AATTTTNTTG 240
TNGAAANTNG AAAATAAAGG TTTCCNNGTA AA 272

SEQ ID NO:584
SEQUENCE LENGTH:279
SEQUENCE TYPE:nucleic acid
TOPOLOGY:linear
CLONE:HUMGS00649
SEQUENCE DESCRIPTION:
GATCTATACC AATTAAACAT TTTCATAGTT CTGCCTATTG TCCTTCCCTG AGGCTCCATT 60
GCTGCTTGGT GGCCATTCTC TGCCTTTTTA CAGTCACCTG AACAATGACC CATCATCTCT 120
TGCTTGCTTG AAATCTTGCT GAAATGTTCT CATTTCCTGT TTGCTGTATG GGCTCGGGTG 180
GGATGTTTGT TGGCTCTGTT GTGTTTATTC ACCAATTTGT ACATTATTTG TTGTCCTTTA 240
CTACTGTAAA CAGTAAATAT AGTTTGGTAT TCTGTCAAA 279

SEQ ID NO:585
SEQUENCE LENGTH:273
SEQUENCE TYPE:nucleic acid
TOPOLOGY:linear
CLONE:HUMGS00650
SEQUENCE DESCRIPTION:
GATCCCAGAC TGGTTCTTGA ACAGACAGAA GGATGTAAAG NATGGAAAAT ACAGCCAGGT 60
CCTAGCCAAT GGTCTGGACA ACAAGCTCCG TGAAGACCTG GAGCGACTGA AGAAGATTCG 120
GGCCCATAGA GGGCTGCGTC ACTTCTGGGG CCTTCGTGTC CGAGGCCAGC ACACCAAGAC 180
CACTGGCCGC CGTGGCCGNA CCGTGGGTGT GTCCAGNAAG AAATAAGTCT GTAGGCCTTT 240
GTCTGTTAAT AAATAGTTTT ATATACCTNN AAA 273

SEQ ID NO:586
SEQUENCE LENGTH:275
SEQUENCE TYPE:nucleic acid
TOPOLOGY:linear
CLONE:HUMGS00651
SEQUENCE DESCRIPTION:
GATCTNCCAC GTCTCCATCT CAGTACACAA TCATTTAATA TTNCCCTGTC TTACCCCTAT 60
TCAAGCAACT AGAGGCCAGA AAATGGGCAA ATTATCACTA ACAGGTCTTT GACTCAGGTT 120

CCAGTAGTTC ATTCTAATGC CTAGATTCTT TTGTGGTTGT NGCTGGCCCA ATGAGTCCCT 180
AGTCACATCC CCTGCCAGAG GGAGTTCTTC TTTTGTGAGA GACACTGTAA ACGACACANG 240
AGAACAAGNN TAAAACAATA ACTGTGTGTG TTAAA 275

SEQ ID NO:587
SEQUENCE LENGTH:269
SEQUENCE TYPE:nucleic acid
TOPOLOGY:linear
CLONE:HUMGS00652
SEQUENCE DESCRIPTION:
GATCTTATGG ATAAAACTCA GAAAGTGAAG GTGAAGAAAG AAACGGTGAA CTCCCCAGCT 60
ATTTATAAAT TTCAGAGTCG TCGAAAACGT TGACGTGTTA TAGATAAGCC TTGTCATTNT 120
GTATCAAAAA TCTGTTGTCG TTTTCTAGTA ACTTCAAATT CCATTACTCC AAATGGCATG 180
GTTTTCCGGT TTGTAACCAT AACTAAATTG TCAGTCTGAC ATTTAATGTC TTTCTATGGA 240
CAACATTAAA TCNCCCTCCC TTCTGTAAA 269

SEQ ID NO:588
SEQUENCE LENGTH:272
SEQUENCE TYPE:nucleic acid
TOPOLOGY:linear
CLONE:HUMGS00653
SEQUENCE DESCRIPTION:
GATCAAGTGG CTTTCCCTGG GACCTGCCCA GCTTTGAGAA TCTCTNCTCA TCCACCCTCT 60
GGCACCCAGC CTCTNAGGGA AGGAGGGATG GGGCATAGTG GGAGACCCAG CCAAGAGCTG 120
AGGGTAAGGG CAGGTAGGCG TGAGGCTGTG GACATTTTCG GAATGTTTTG GTTTTNTTTT 180
TTTTAAACCG GGCAATATTG TGTTCAGTTC AAGCTGTGAA GNAAAATATA TATCANTGTT 240
NNCCAATANA ATACAGTGAC TANCTGAACA AA 272

SEQ ID NO:589
SEQUENCE LENGTH:268
SEQUENCE TYPE:nucleic acid
TOPOLOGY:linear
CLONE:HUMGS00654
SEQUENCE DESCRIPTION:
GATCGTCAAA TCTTTTNCAA ATTTAATNTA TATGTGTATA TAAGGNAGTA TTCAGTGAAT 60
ACTTGAGANA TGTACAAATC CTTCATCCAT ACCTGTGCAT GAGCTGTATT CTTCACAGCA 120
ACAGAGCTCA GTTAAATGCA ACTGCAAGTA GGTTACTGTA AGATGTTTAA GATAAAAGTT 180
CTTCCAGTCA GTTTTTCTCT TAAGTGCCTG TTTGAGTTTA CTGAAACAGT TTACTTTTGT 240
NCAATAAAGT TTGTATGTTG CATTTAAA 268

SEQ ID NO:590
SEQUENCE LENGTH:267
SEQUENCE TYPE:nucleic acid
TOPOLOGY:linear
CLONE:HUMGS00656

SEQUENCE DESCRIPTION:
GATCAAAAAT AAAATGTTAT TTTTAAAGTT TCTNTTTGAG ATTTTNCTTA AGTTTTGGTA 60
GATATTCTTA AGTTTTAGTG ACCTCAGTTT GGGAATTAAG TAAGCTAAAC ATTGTGTCCT 120
TATTATNAGT TATATAAAAC TATGCTTTAG ACTTTGTNAG AAACTTCTGC CCCACCTTGA 180
CTGACTGCTT TNCCATTTNT GGTTGTACAA AATGAATTCA CACTTTAATG CTATGGCCAC 240
CTTTAAATAA AGTACAGCGT GACTAAA 267

SEQ ID NO:591
SEQUENCE LENGTH:265
SEQUENCE TYPE:nucleic acid
TOPOLOGY:linear
CLONE:HUMGS00657
SEQUENCE DESCRIPTION:
GATCTAGATT CTACATGTTA CCATTGGTTT ATTCTTGTGC TTTCTGTATT TAAAACTTTG 60
GCTGTACTAA GCAAATGCAA GGTTATAATT TAGCTAATAG TAGTTTACAG ACAATTCTGA 120
TGATTATGAT TTCATTTGGT TTAACTAAGC TGTACTAGTT CATTTCATAA GGAAATGATA 180
CTGTAGACAA ATGTAAATAA AGCCTGTGAG TCAAGCATCA AGTGGTGTTT GTTAGAAATA 240
ANCTAGAGAT TTTTAANCTC TGAAA 265

SEQ ID NO:592
SEQUENCE LENGTH:264
SEQUENCE TYPE:nucleic acid
TOPOLOGY:linear
CLONE:HUMGS00658
SEQUENCE DESCRIPTION:
GATCTGGGGC CAGCTCACCG CTCACGTCCC CGTCATCGAC AACTCCACCC TNTACATCAG 60
TAGAGCATGC ACCATTTTGA ACGTGACATT TNCGGTAAAG TAAACTATGC TGATTTCTCA 120
GACTTTAAAG ATGCTCTNNT TCTGTGTGTN AAATAGGACC CAAAGTGTCT CGATTGCTGA 180
AGTGATGAAC AAGTGGGAAA GCAGATTTGA GACTATTTCC TTATCTGAAT ATTTAAATGA 240
AATACAGCAT CTTTAAAANG CAAA 264

SEQ ID NO:593
SEQUENCE LENGTH:262
SEQUENCE TYPE:nucleic acid
TOPOLOGY:linear
CLONE:HUMGS00659
SEQUENCE DESCRIPTION:
GATCTTTTAA AAAGATGATG CAGTTCTGTA TTTATTGTGC TGTGTCTGGT CCTAAGTGGA 60
GCCAATTAAN CAGGTTTCAT ATGTATTTTN CCAGTGTTGA ATCTCACACA CTGTACTTTG 120
AAAATTTCCT TCCATCCTGA ATAACGAATA GAAGAGGCCA TATATATTGC CTCCTTATCC 180
TTGAGATTTC ACTACCTTTA TGTTAAAAGT TGTGTATAAT TGTTAAAATC TGTGAAAGAA 240
TAAAAAGTGG ATTTAAATTA AA 262

SEQ ID NO:594
SEQUENCE LENGTH:260

SEQUENCE TYPE:nucleic acid
TOPOLOGY:linear
CLONE:HUMGS00661
SEQUENCE DESCRIPTION:
GATCCATCCA AAAACAAGGA CTGCAGCCTA AATTCCAAAT ACCAGAGACT GAAATTTTCA 60
GCCTTGCTAA GGGAACATCT CGATGTTTGA ACCTTTGTTG TGTTTTGTAC AGGGCATTCT 120
CTGTACTAGT TTGTCGTGGT TATAAAACAA TTAGCAGAAT AGCCTACATT TGTATTTATT 180
TTCTATTCCA TACTTCTGCC CACGTTGTTT TCTCTCAAAA TCCATTCCTT TAAAAAATAA 240
ATCTGATGCA GATGTGTAAA 260

SEQ ID NO:595
SEQUENCE LENGTH:259
SEQUENCE TYPE:nucleic acid
TOPOLOGY:linear
CLONE:HUMGS00662
SEQUENCE DESCRIPTION:
GATCCCTTTA TGAAACCTTG TGAATAGATG AATGTNTGGA GATGGCGACT AGTGGACAAC 60
AGAACAATAT TGGAATGGTG GTAATACGAG GAAATAGTAT CATCATGTTA GAAGCCTTGG 120
AACGAGTATA AATAATGGCT GTTCAGCAGA GAAACCCATG TCCTCTCTCC ATAGGGCCTG 180
NTTTACTATG ATGTAAAAAT TAGGTCATGT ACATTTTCAT ATTAGANTTT TTGTTAAATA 240
NNCTTTTGTA ATAGTCAAA 259

SEQ ID NO:596
SEQUENCE LENGTH:257
SEQUENCE TYPE:nucleic acid
TOPOLOGY:linear
CLONE:HUMGS00663
SEQUENCE DESCRIPTION:
GATCNNGGCT AAGCCAGCCA GCCCNCCCGC GCCAGGNAAA ACAGGGCTGC AGGTGTCCTG 60
TCTCCCAGCC TCATCTGGCC GGCCTCCCCA AACATTTGCC TGTCCATCAG CTCTTCCTCC 120
TTTCGAGTCA TGTGGAAAGG GACAGGNCCA AGTGGCCTTG GTGTTTAAAT CTTGCCCTAA 180
ATTGTAACTC ACATGATTAT TTAAAGTCAC TAGANATAAG TAAGCACAGC AATAAAGNTT 240
TAATGGAATA AAAGAAA 257

SEQ ID NO:597
SEQUENCE LENGTH:252
SEQUENCE TYPE:nucleic acid
TOPOLOGY:linear
CLONE:HUMGS00664
SEQUENCE DESCRIPTION:
GATCCCAGAA TTCAACCTGT ATTTATAAAT GTATAATGTA TTTAGCTACT TTTTGGTTTA 60
AATGAACTTG TTGGGTTAGC TTGGTAAATG TTATAATTTT NACTATTTTC TACAAAGAAA 120
ATATTTTCTA ATTTAAGTTG GAGCTATCTG TGCAGCAGTT TCTCTACAGT TGTGCATAAA 180
TGTTTTTNCT ATAAAATGAG CTAATGTATA ANATACTGCT GTATACCATA ATAANGATAG 240
TAATACTTGA AA 252

SEQ ID NO:598
SEQUENCE LENGTH:250
SEQUENCE TYPE:nucleic acid
TOPOLOGY:linear
CLONE:HUMGS00665
SEQUENCE DESCRIPTION:
GATCATTAGT TGAATCGGTA TCATCTTCAC CAAATAAAGA AAGTAATGAA GAAGAGCAAG 60
TGTGGCACTT CCTTGGCAAG TGATTGAAAC ATCTGAAATT CTGCTGTCAA GATTCCCATC 120
TCTAAGGACT CCAAGTGCTA GAGACAAGGG GGTCTATGAG CATTTACTGA CTTCCTGTTA 180
AAACTTCATT TTTTCAAACT TTTTGAGCTA TGCAATATAT AANTAAACAG TAAGAATTTT 240
AAATTACAAA 250

SEQ ID NO:599
SEQUENCE LENGTH:250
SEQUENCE TYPE:nucleic acid
TOPOLOGY:linear
CLONE:HUMGS00666
SEQUENCE DESCRIPTION:
GATCCTTCTT CCTTTCACTG GTCGTGCCTC CCANNAGGTG CAGAGATTCT TAGAAGAGGA 60
GGTGTATCCC CTGTTAAAAC CATATGAAAG CGTGATGAAG GTGAAAGCAG AATTATGTCT 120
GTAGAGTTGG AAGAGAATTA AACGAAAATC ATTGTTAATT GCTGAGGCAT GAAAATTGTG 180
TTACTATAAT GCCTTATTTT ACCTCGAGAA TTGTTACCTT AAATTAGTAC AGCACTTTCT 240
TCTTCCCAAA 250

SEQ ID NO:600
SEQUENCE LENGTH:247
SEQUENCE TYPE:nucleic acid
TOPOLOGY:linear
CLONE:HUMGS00667
SEQUENCE DESCRIPTION:
GATCCTTCAG TTACATACAA TTTGTTTAAT GAAATGTCAT GGCTCTGTTC ATATTTTNNT 60
NTTGTNCTTC CAATTGGTAT ATACAACTTT CAGAGCCTCT TGTATTTGGA AGGCTGGAAG 120
GGCCCAGACT TTGGAATAGT GTCTTGGTTT CACTGTTTTN GTTTTGATTT TTTTTTTGTT 180
TNGATTTTTT TAAAACTAAA GCTATATAAA GCTTGNGGAT TAANCAGANT AAATTCCTAA 240
ATTTAAA 247

SEQ ID NO:601
SEQUENCE LENGTH:246
SEQUENCE TYPE:nucleic acid
TOPOLOGY:linear
CLONE:HUMGS00668
SEQUENCE DESCRIPTION:
GATCCAGGGT GTGTGTGAGT TGAGGGTGGG TGGAGGGGTT TGCAGTGTGG GAATGTGGCC 60
CTGCAGTTGA CCTGAGCTGC TTCACATGGT TGTCCATTCT GGGGCTTAAA GAACTGGGAC 120

CAGACCAAGT AGAGGCCTTG GTGCTGNTTG GGGTGGGGCC TGCAGANTCT TAGTTACTGA 180
TTTCATTTTC AATAAATGTA GGTTTGTTAC ATGAGTTTCC CAATTAAAAA AAAAATGACT 240
TCTAAA 246

SEQ ID NO:602
SEQUENCE LENGTH:284
SEQUENCE TYPE:nucleic acid
TOPOLOGY:linear
CLONE:HUMGS00669
SEQUENCE DESCRIPTION:
GATCAGAATT TCACCAGGGA GTAAAATTAC CTGAAAACGT AAGANGTTTT AAACAGCTTT 60
TCACACAAAT TAGATGCAAC TGTTCCCATG TCTGAGTACT TATTTAAAAG AAAGGTAAAG 120
ATTGGCCTGT TAGAAAAAGC ATAATGTGAG CTTTGGATTA CTGGATTTTT TTTTTTTTNA 180
AACACACCTG GNGNGGNCAT TTGAAAACAC TNTTCTTACC CTCGANCCCT GATGTGGTNC 240
CATTATGTAA ATATTTCAAA TNTTAAAAAT GTATATATTT GAAA 284

SEQ ID NO:603
SEQUENCE LENGTH:249
SEQUENCE TYPE:nucleic acid
TOPOLOGY:linear
CLONE:HUMGS00670
SEQUENCE DESCRIPTION:
GATCGAGGTG GAGAAGCCCT TTGCCATCGC CAAGNAGTAG GGCACAGGGA CATCTTTCTT 60
TNAGTGACCG TCTGTGCAGG CCCTGTAGTC CGCCACAGGG CTCTGAGCTG CACTNGCCCC 120
GGTGCTGGCA TCTGGTGGAG CGGACCCACT CCCCTCACAT TCCACAGGCC CATGGACTCA 180
CTTTTGTAAC AAACTCCTAC CAACACTGAC CAATAAAAAA AAATGTGGGT TTTTTTTTTT 240
TTAAATAAA 249

SEQ ID NO:604
SEQUENCE LENGTH:244
SEQUENCE TYPE:nucleic acid
TOPOLOGY:linear
CLONE:HUMGS00671
SEQUENCE DESCRIPTION:
GATCGGAATG GTGGAGAACT TCAACCAGGC ACTCAAGGAA ATTGGGGATG TGGAGAACTG 60
GGCTCGGAGC ATCGAGCTGG ACATGCGCAC CATTGCCACT GCACTGGAAT ATGTCTACAA 120
AGGGCAGCTG CAGTCTGCCC CTTCCTAGCC CCTGTTCCCT CCCCCAACCC TATCCCTCCT 180
ACCTCACCCG CAGGGGAAAG GAGGNAGGCT GACAAGCTTG AATAAAACAC AAGCCTCCGT 240
TAAA 244

SEQ ID NO:605
SEQUENCE LENGTH:244
SEQUENCE TYPE:nucleic acid
TOPOLOGY:linear
CLONE:HUMGS00672

SEQUENCE DESCRIPTION:
GATCCCCTTT CCGTAAAAGC GTGTAACAAG GGTGTAAATA TTTATAATTT TTAATACCTG 60
TTGTGAGACC CGAGGGGCGG CGGCGCGGTT TTTNATGGTG ACACAAATGT ATATTTTNCT 120
AACAGCAATT CCAGGCTCAG TATTGTGACC GCGGANCACA GGGGACCCCA CGCACATTCC 180
GTTGCCTTAC CCGATGGCTT GTGACGCGGA GAGAACCGAT TAAAACCGTT TGAGAAGCTC 240
CAAA 244

SEQ ID NO:606
SEQUENCE LENGTH:242
SEQUENCE TYPE:nucleic acid
TOPOLOGY:linear
CLONE:HUMGS00673
SEQUENCE DESCRIPTION:
GATCTTTCCC ATNTCTACCT AAGTCAGCTT TCATCTTTGT GGATGGTGTC TCCTTTACTA 60
AATAAGAAAA TAACAAAGCC CTTATTCTCT TTTTTTNTTG TCCTCATTCT TGCCTTGAGT 120
TCCAGTTCCT CTTTGGTGTA CAGACTTCTT GGTACCCAGT CACCTCTGTN TTCAGCACCC 180
TCATAAGTCG TCACTAATAC ACAGTTTTGT ACATGTAACA TTAAAGGCAT AAATGACTCA 240
AA 242

SEQ ID NO:607
SEQUENCE LENGTH:245
SEQUENCE TYPE:nucleic acid
TOPOLOGY:linear
CLONE:HUMGS00674
SEQUENCE DESCRIPTION:
GATCCGGGTG GATGCACAGC CCGTCAAGGT CTATGCTGAC GNCTCCCTGG TCTTCCCCCT 60
GCTTGTGGCT GAAACCTTTG CCCAGAAGAT GGATGCCTTC ATGCATGAGA AGAACGAGGA 120
CTGAGCGGCT GCGGTCCCAG GAAGGTCTTA CCCCCTCTNC TATTTATNAA TTTGCAGACC 180
CAGCCCNTCC CCTACTTTTT GGTCAGCTAC GNCTCTAGAA TAACNCCCGG TATCTGAAGT 240
CCAAA 245

SEQ ID NO:608
SEQUENCE LENGTH:245
SEQUENCE TYPE:nucleic acid
TOPOLOGY:linear
CLONE:HUMGS00675
SEQUENCE DESCRIPTION:
GATCTCTACC ATTTAATTAA GAAAGCAGTT GCTGTTCGAA AGCATCTTGA GAGGAACAGA 60
AAGGATAAGG ATGCTAAATT CCGTCTGATT CTAATAGAGA GCCGGATTCA CCGTTTGGCT 120
CGATATTATA AGACCAAGCG AGTCCTCCCT CCCAATTGGA AATATGAATC ATCTACAGCC 180
TCTGCCCTGG TCGCATAAAT TTGTCTGTGT ACTCAAGCAA TAAAATGATT GTTTAACTAA 240
ACAAA 245

SEQ ID NO:609
SEQUENCE LENGTH:241

SEQUENCE TYPE:nucleic acid
TOPOLOGY:linear
CLONE:HUMGS00676
SEQUENCE DESCRIPTION:
GATCTAAAAT GTCACATTCA GATTTTNAGG AAGAAAATCT TCATTACAGT GGAGCACAAA 60
TNTTCCATAC AAGACATCAT TGAGGNAGCA TGCTGTCCCC TTCTAACCTG AAACACATTC 120
TTTCCCATCC NGGTTGGGCT TCTNTACCNC CTTATTAATT TATGAACCNG AAGTTGCTTG 180
AAGTGTTTTG GGCTTAATAA ATGGGGTGAA AGTATAGGTA GCAGTAACAC CTACATGNAA 240
A 241

SEQ ID NO:610
SEQUENCE LENGTH:240
SEQUENCE TYPE:nucleic acid
TOPOLOGY:linear
CLONE:HUMGS00677
SEQUENCE DESCRIPTION:
GATCTTCTGA GGTCAGGAGT TTGAGACCAG CCTGACCAAC ATGGAGAAAC CCAGTCTCTA 60
CTGAAAATAC AAAATTAGCC GGGCATGGTG GTGCACGCNT GTAGTCCCAG CTACTTGGGA 120
GGCTGAGGCA GGAGAATCGC TTGAACCCAG GAGGCGGAGG TTGCGGTGAC CCTCCAGCTT 180
GGGCAACATG TTATGANTGA AACTCCATCT CAAAAAATAA AAAAAAAAAA GGGNNGCAAA 240

SEQ ID NO:611
SEQUENCE LENGTH:240
SEQUENCE TYPE:nucleic acid
TOPOLOGY:linear
CLONE:HUMGS00678
SEQUENCE DESCRIPTION:
GATCTGTGAA GGCTTCCCTG ACCNNTGCCC AGGAAGAGTT CACTGGTCGC TCTGTTGTGC 60
CCCACAGCAC TTTGTTATAC CTCTGCCACA CACTTCACGC AGCGCGTTGT AACTCATGTG 120
TTTACATGTC TGTCCCNCCA GACTGTNAGC TCCTTGAGGG CAGGGACTGT ACATTCTCCA 180
GCTCTGTGTC CCCAGGGCCT GGCACATTGT AGACGCTTAA TAAATTTCTG TTAAATGAAA 240

SEQ ID NO:612
SEQUENCE LENGTH:242
SEQUENCE TYPE:nucleic acid
TOPOLOGY:linear
CLONE:HUMGS00679
SEQUENCE DESCRIPTION:
GATCAGCTCC TTGACCTCTG AGGGGCAGGN GTGCTTCCTG GTGTGTGTAT TAGAATCCCT 60
TCCTGCCTTG TTTCATGGCA GTGAAATGCC TCTTGGTCCT GTCCAAGTGT ATCTTTCACT 120
GATTTCTGNA TCATGNTCTA GTTGCTTGAC CCTGCCANAT GGGTCCAGTG TTCATCTGAG 180
CATAACTGTA CTAAATCCTT TTTCCATATC AGTATAATAA AGGAGTGATG TGCAATAGCA 240
AA 242

SEQ ID NO:613

SEQUENCE LENGTH:239
SEQUENCE TYPE:nucleic acid
TOPOLOGY:linear
CLONE:HUMGS00680
SEQUENCE DESCRIPTION:
GATCGCAACA ATNAGCCATC CACATNCGTT TTTCAGGGTC ACACCCAAGT AATTGAAAAG 60
ACACTCCTCC ACTTATCCCC TCCNTAATAT GGCTCTNCGC ATGCTGAGTA CTGGACCTCG 120
GACCAGAGCC ATGTAAGAAA AGGCCTGTCN CCTGGAAGCC AAAGGACTCT GCATTGAGGG 180
TGGGGGTAAT TTTTTCTTGG NGGGCCCAGT TAGTGGGCTT NCGNANTGTN TGTATGNGN 239

SEQ ID NO:614
SEQUENCE LENGTH:238
SEQUENCE TYPE:nucleic acid
TOPOLOGY:linear
CLONE:HUMGS00681
SEQUENCE DESCRIPTION:
GATCCAAATA AACAGACCCC GTCTGGCAAG AAATGCATTG CAGCCAAAAA AATTAAACAG 60
TCGGTGGGAA ACAAAAGCAT GTCCTTTCCA ACTGGAAAGT CAGACAGAGG CTTCAGGTAC 120
AACTGGCCAC AGAGATAGTC CTGGAAGACA CGTGGCGCCT GTGGACCGGA AGCACCAAAT 180
GCTGGTGCTG CTTTTGTACA TACATATTTT TAAACCATTA AAATTCTTCC TGAAGAAA 238

SEQ ID NO:615
SEQUENCE LENGTH:254
SEQUENCE TYPE:nucleic acid
TOPOLOGY:linear
CLONE:HUMGS00682
SEQUENCE DESCRIPTION:
GATCCATAGT CAGAAAAGTT ACTGCAGCTT AAACAGGAAA CCCTTCTTGT TCAGGACTGT 60
CATAGCCACA GTTTGCAAAA AGTGCAGCTA TTGATTAATG CAATGTAGTG TCAATTAGAT 120
GTACATTCCT GAGGTCTTTT ATCTGTTGTA GCTTTGTCTT TTTCTTTTTC TTTTCATTAC 180
ATCAGGTATA TTGCCCTGTA AATTGTGGTA GTGGTACCAG GAATAAAAAA TTAAGGAATT 240
TTTAACTTTT CAAA 254

SEQ ID NO:616
SEQUENCE LENGTH:247
SEQUENCE TYPE:nucleic acid
TOPOLOGY:linear
CLONE:HUMGS00683
SEQUENCE DESCRIPTION:
GATCAAAGAA AGAAGGCATA CGCNGATTTC TACAGAAACT ATGATGTCAT GAAAGATTTT 60
NAGGAGATGA GGAAGGCTGG TATCTTTCAG AGTGTAAAGT AATCTTGGAA TATAAAGAAT 120
TNCTTCAGGT TGAATTACCT AGAAGTTTGT CACTGACTTG TGTTCCTGAA CTATGACACA 180
TGAATATGTG GGCTAAGAAN TAGTTCCTCT TGATAAATAA CCANTTAACA AATNCTTTNG 240
ACAGAAA 247

SEQ ID NO:617
SEQUENCE LENGTH:235
SEQUENCE TYPE:nucleic acid
TOPOLOGY:linear
CLONE:HUMGS00684
SEQUENCE DESCRIPTION:
GATCTGCACC TGAGCCAAAG AAACCTGAGG AAAATCCAGC TTCTAAGTTC AGTTCTGCAA 60
GCAAGTATGC TGCTCTCTCT GTTGATGGTG AAGATGAAAA TGAGGGAGAA GATTATGCCG 120
AATAGACCTC TACATCCTGT GCTTTTNTCC TAGTTTCTCT CCACCCTGGA ACATTCGAGA 180
GCAAATCAAA ACCTCTATCC AGACAAGACA AAATAAAACT CAACATCTCC TGAAA 235

SEQ ID NO:618
SEQUENCE LENGTH:238
SEQUENCE TYPE:nucleic acid
TOPOLOGY:linear
CLONE:HUMGS00685
SEQUENCE DESCRIPTION:
GATCTGTTCC TTGGCAGTGG ACTCAGAAAG CCAACATGTG GCTCCTCCCA GCCCATAACC 60
AGTATTTTTC CTGCTTCTAA ATACAAATNG GTTGGTTTTA ACTTCANATT GANCTTACTG 120
TAGCCTCAAA TGATTTCCCC CCTCCGCCTC CAGGAAGAAA GAATGTNACT GCCTTAATAA 180
AAAATGAAAA GAGAATGATG CTCAAAATCT TTCCAAATAA AATGTTCCCT ATATTAAA 238

SEQ ID NO:619
SEQUENCE LENGTH:234
SEQUENCE TYPE:nucleic acid
TOPOLOGY:linear
CLONE:HUMGS00686
SEQUENCE DESCRIPTION:
GATCCGCCCT CGAATGGACA CATTACCAGT GAAGGGGCAT TTNTNGTCAA TGTAGGTGCC 60
CTCAATAGCC TCCTTGGGTG TNTTGAAGCC CAGACCGATG TTCTTGTAGT ACCGCGGGAG 120
CTTCTCCTTG CCAGTTTCTC CCAGCAGGAC CCTCTTCTNG TTTTGAAAGA TGGTCGGCTG 180
CTTTTGGTAG GCACGCTCAG TCTGAATGTC CGCCATCTTC CCGGCCGGCT GAAA 234

SEQ ID NO:620
SEQUENCE LENGTH:251
SEQUENCE TYPE:nucleic acid
TOPOLOGY:linear
CLONE:HUMGS00687
SEQUENCE DESCRIPTION:
GATCCCCAAG CCTGGCAAGG GAATTTNTTC AACTCCCTGC CCCCCAGCCC TCCTTATCAA 60
AGGACACCAT TTTGGCAAGC TCTATCACCA AGGAGCCAAA CATCCTACAA GACACAGTGA 120
CCATACTAAT TATACCCCCT GCAAAGCCCA GCTTGAAACC TTCACTTAGG AACGTAATCG 180
TGTCCCCTAT CCTACTTCCC CTTCCTAATT CCACAGCTGC TCAATAAAGT ACAAGAGCTT 240
AACAGTNNAA A 251

SEQ ID NO:621
SEQUENCE LENGTH:241
SEQUENCE TYPE:nucleic acid
TOPOLOGY:linear
CLONE:HUMGS00689
SEQUENCE DESCRIPTION:
GATCCATCAT CCGNCAATGT TAAAAGGCCC CGTGCGCGAG GGCGACGTGC TCACCCTTTT 60
GGAGTCAGAG CGAGAAGCCC GGAGGTTGCG CTGAGCTTGG CTGCTCGCTG GGTCTTGGAT 120
GTCGGGTTCG ACCACTTGGC CGAGGGGAAT GGTCTGTCAC AGTCTGCTCC TTTTTTTTGT 180
CCGCCACACG TAACTGAGAT GCTCCTTTAA ATAAAGCGTT TGTGTTTCAA GTTAACTCAA 240
A 241

SEQ ID NO:622
SEQUENCE LENGTH:231
SEQUENCE TYPE:nucleic acid
TOPOLOGY:linear
CLONE:HUMGS00690
SEQUENCE DESCRIPTION:
GATCCTCCTG CCTNGGCCTC CAAAAGTGCT GGGATTACAT GCATACCTGA NCTACTGAGC 60
ACTTTNATCT TGAATGAGTG TTGGATTTTG TCAAATGCTT TTCCTGTACC TATTAATACA 120
ATCATGATTT TNNCCTCTTT AGTATGTTGA TATGATGGGT TACATTAATT GATNNTCAAA 180
TGTTGAATCA GTCTNGCATA CCTGGAATAA ATCCCACTTG GTCATAATAA A 231

SEQ ID NO:623
SEQUENCE LENGTH:231
SEQUENCE TYPE:nucleic acid
TOPOLOGY:linear
CLONE:HUMGS00691
SEQUENCE DESCRIPTION:
GATCTATGAA ATCTGTGTAG GTTTTAAATA TTTTAAAAAT TATAATACAA ATCATCAGTG 60
CTTTTAGTAC TTCAGTGTTT AAAGAAATAC CATGAAATTT ATAGGTAGAT AACCAGNTTG 120
TCNCTTTTTG TTTAAACCAA GCAGTTGANA TGGCTATAAA GACTGACTCT AAACCAAGAT 180
TCTGCAAATN NTGATTGGNA TTGCACAATA AACATTGCTT GNTGTTTTAA A 231

SEQ ID NO:624
SEQUENCE LENGTH:231
SEQUENCE TYPE:nucleic acid
TOPOLOGY:linear
CLONE:HUMGS00692
SEQUENCE DESCRIPTION:
GATCCCATTT AGGAANCGGC ATTCACTTCA GAAGGTACTT TTTAACTGCT CAGTTTTTGA 60
CTATTTTAAA TAGTTTGCTG AAAACTCCTG ATAACACTTG CTACATATCA TGTTTTAATT 120
GCTTGTACAG TTAACCTTTA ATTTTATTTA GTAAAGTGTA TCAAAGTAGG ACTTTTTTGA 180
ATTGTAAATA GGTGGTTTTA TTAAATAAAA GTCAATGTAA AAATTGTTAA A 231

SEQ ID NO:625
SEQUENCE LENGTH:230
SEQUENCE TYPE:nucleic acid
TOPOLOGY:linear
CLONE:HUMGS00693
SEQUENCE DESCRIPTION:
GATCCGGAGG GAAATGTGTT AGAGGGTCTG GAAAATTCAG TGCTTTTGAG TTACTTGTTT 60
TTATTAAAAA TTTCCTCACA AAAGAGAGTC CTCAAGTTGT GGCTGTTCTT GGGAAAGGGG 120
TCACCGTGTC TGACAAAGTG TAACTTTAAA AAGCACGTTG ATTTTTTACA AATGTAAGTG 180
TGCTTGGGAA TTCCTTAAAT TTTGTGCAAT AAACTATTTT TTGGAAGAAA 230

SEQ ID NO:626
SEQUENCE LENGTH:202
SEQUENCE TYPE:nucleic acid
TOPOLOGY:linear
CLONE:HUMGS00694
SEQUENCE DESCRIPTION:
GATCCAATCA TATTTNCTGT AGGGTGGAGG AGGTTTCAGA CCATCCCGCT CTGTTATATC 60
GAAGACCACA ATGGAAGACA AAGGCTTCTA AAGTATACCC CACAGCACGT GCATCGCGGA 120
NAGCCTNCTT GGGATAAAAT ATGTTTACAA TAACTTGCCT ATTGCTGAGA TTAAACCTTA 180
CAGGCTGCGT TATTTTAGCA AA 202

SEQ ID NO:627
SEQUENCE LENGTH:226
SEQUENCE TYPE:nucleic acid
TOPOLOGY:linear
CLONE:HUMGS00695
SEQUENCE DESCRIPTION:
GATCCATTGT ATCAGTACCT CACAATCAGA GTTGGCAAAT GATGGATGAG TGATTCAAGC 60
AGTGCACCCG GTGGAAGCTG AAATCCATCT GTGAATGGAA CTGAAGTGAA CGTGAATATG 120
CTGACTATAT CCTGGAAGCA TTTTTATACC ATCTTGAAAT TTCAACANAC TGGCTTTTGC 180
CAGTTAATCC AGCTGTCTTT CAAGAATAAA AGTTGGGGTT TTCAAA 226

SEQ ID NO:628
SEQUENCE LENGTH:226
SEQUENCE TYPE:nucleic acid
TOPOLOGY:linear
CLONE:HUMGS00696
SEQUENCE DESCRIPTION:
GATCACCTGA GGTCAGGAGT TCGANACCAG CCTGGCCAAC AGGGTGAACC CCGTCTCTAC 60
TAAAAGTACA AAAATTAGCT GGGCGTGGTG GCGGACGCTG TAATCTCAGC TACTTGGNAG 120
GCTGAAGCAG GAGAGGTGCT GGAACCTGGN AGGCGGAGGT TGAAGTNAGC CGAGATTGCC 180
CTATTGCACT CCAGCTCGGG CGNCAACTGC AAGACTCCAT CTCAAA 226

SEQ ID NO:629

SEQUENCE LENGTH:226
SEQUENCE TYPE:nucleic acid
TOPOLOGY:linear
CLONE:HUMGS00697
SEQUENCE DESCRIPTION:
GATCTGTTAA TTTCCTATCT AATAAATGCC TTNAATTGTN CTCATAATNA AGAATAAGTA 60
GGTATCCCTC CATGCCCTTC TGTAATAAAT ATCTGGAAAA AACATTAAAC AATAGGCANA 120
TATATGTNAT GTGCATTTCT AGAAATACAT AACACATATA TATGTCTGTA TCTTATATTC 180
AATTGCAAGT ATATAATAAA TAAACCTGCT TCCAAACAAC AATAAA 226

SEQ ID NO:630
SEQUENCE LENGTH:226
SEQUENCE TYPE:nucleic acid
TOPOLOGY:linear
CLONE:HUMGS00698
SEQUENCE DESCRIPTION:
GATCAGAGTA ATTCTTTTGT ACATTGAAAT NAGGGGCTTG GTTTAAAAAA AGACCTTTCC 60
CTCTCCCTGC CCCTAGAACA ACCAGTATTA GAAGGTGCCA CCATTGGTGC TGCCTTCTNT 120
TCCCACAGCC TGTAACTCAG TGTTTTGTAC TTCACTGAAT TGTGATGGNT AGAAACTTCG 180
TGGGTAGNNN NTGGGAATCA TCCNGTTAAA CAANACGGGN TTTAAA 226

SEQ ID NO:631
SEQUENCE LENGTH:222
SEQUENCE TYPE:nucleic acid
TOPOLOGY:linear
CLONE:HUMGS00699
SEQUENCE DESCRIPTION:
GATCCTTGGT GTACTGAGCA GTTTCTTTGG GGCTTTTTCT TTCTGGGAAG CGGGAGGGAA 60
AGGAGCAAGG TGTCATCCTG CTCTTCATTT GTATTTTGGT CCCAAAATGT AAATACAATT 120
TNTTATGTTA CTTTTTTGTG GTAACTACCG AGATGAATAT TTTAATTAGA TAAGTTATAT 180
GAAAAGGAAA ATTCCATGTC TAAATAAAAA ACAAACTCCA AA 222

SEQ ID NO:632
SEQUENCE LENGTH:222
SEQUENCE TYPE:nucleic acid
TOPOLOGY:linear
CLONE:HUMGS00700
SEQUENCE DESCRIPTION:
GATCTTCTGG ACAAACTTCT GCGATACGAC CATCAACAGA GACTGACTGC CAAANAGGCC 60
ATGGAGCACC CATACTTCTA CCCTGTGGTG AAGGAGCAGT CCCAGCCTTG TGCAGACAAT 120
GCTGTGCTTT CCAGTGGTCT CACGGCAGCA CGATGAAGAC TGGAAAGCGA CGGGTAATGC 180
GGCATTGATG CTTNCCAATA AAACCAACCA ACCAAACACA AA 222

SEQ ID NO:633
SEQUENCE LENGTH:221

SEQUENCE TYPE:nucleic acid
TOPOLOGY:linear
CLONE:HUMGS00701
SEQUENCE DESCRIPTION:
GATCCACCCA GTTCTTTAGG CAACCACTGA TAGCATTTTC TTAAGTATTC TTCCAGATAT 60
CGTCTATGCA TATGTAAAAG TATCTGNCTT TCTCCNTTTA AAAACACAAT TGGNAATATA 120
TCATACTNGC TGGTTTGCAC CTNGCTTTTT TTGCTTAATA TATCTAGTTT ATAATGNCCN 180
AATGNGCAAA TTTTNGCATC NGCCCTAAAT ATCTACTGGN N 221

SEQ ID NO:634
SEQUENCE LENGTH:220
SEQUENCE TYPE:nucleic acid
TOPOLOGY:linear
CLONE:HUMGS00702
SEQUENCE DESCRIPTION:
GATCACTCGT TTAAGTCCTT AGTTGTATGT NATCTCTTCT CTAGCAGGAA TTGGCAAACT 60
TTTTTGTAAA GGGGTAGAAA GTGAAGATTT TAAGGCTTTG CAGGCCATAT ATCCTCTNCT 120
GCAAATNCTC AGCCCTGCTG TTGTAATGTA AAANCTNCCA CAGACACTAC ATGAACACGA 180
ATGAGTGTGG CTGGTGTTCC AATAAAACTT TATTTACAAA 220

SEQ ID NO:635
SEQUENCE LENGTH:220
SEQUENCE TYPE:nucleic acid
TOPOLOGY:linear
CLONE:HUMGS00703
SEQUENCE DESCRIPTION:
GATCATCAAA CCAGTCCACA AGCACAGGGA GATGCGTGGG CTGACATCTG CAGGCCGAAA 60
GAGCCGTGGC CTTGGAAAGG GCCACAAGTT CCACCACACT ATTGGTGGCT CTCGCCGGGC 120
AGCTTGGAGA AGGCGCAATA CTCTCCAGCT CCACCGTTAC CGCTAATATA NGTAAAGTTT 180
NGTAAAATTC ATACTTAATA AACAATTTAG GACAGTCAAA 220

SEQ ID NO:636
SEQUENCE LENGTH:219
SEQUENCE TYPE:nucleic acid
TOPOLOGY:linear
CLONE:HUMGS00704
SEQUENCE DESCRIPTION:
GATCCACATC TCAAAGAAGT GGGGCTTCAC CAAGTTCAAT GCTGATGAAT TTGAAGACAT 60
GGTGGCTGAA AAGCGGCTCA TCCCAGATGG CTGTGGGGTC AAGTACATCC CCAGTCGTGG 120
CCCTCTGGAC AAGTGGCGGG CCCTGCACTC ATGAGGGCTT CCAATGTGCT GCCCCCCTCT 180
TAATACTCAC CAATAAATTC TACTTCCTGT CCACCTAAA 219

SEQ ID NO:637
SEQUENCE LENGTH:215
SEQUENCE TYPE:nucleic acid

TOPOLOGY:linear
CLONE:HUMGS00705
SEQUENCE DESCRIPTION:
GATCCTCTCT CGACTNGCCA TACATTTCTT TCACAGCATT TACATAGTCC ATGATAGTTT 60
ACTTGTGGGA TTATTTGGTT AATCTTTGCC TTTAACACCA GGGTTCCTTG GGTGAAGGAG 120
CTTCTTTATC TNGGTAACAG CATTATTTCA AGCATAACTN GTAATATAGT NATATTACAT 180
ATATANCATA TATATATATN NCANANCANA TATAN 215

SEQ ID NO:638
SEQUENCE LENGTH:214
SEQUENCE TYPE:nucleic acid
TOPOLOGY:linear
CLONE:HUMGS00706
SEQUENCE DESCRIPTION:
GATCTGGTTT CTTAGCAAAT TTCCCAGTAG GATGTCATGT AAGTNCCTTC CCCCTCTTAG 60
AGATTGAAGG CTGTAAGAGT CCAGATGGTG GAGCCAGGCT GTCTGGGTTC AAATGCCATC 120
TTTGACACTT GCAAGCTAAA TNACATTACT CAAATTAATC GTTCTGCACT TCAGCTTCCN 180
TGTCTATCAA ATAAAAAGAA TAGTACCNGC CAAA 214

SEQ ID NO:639
SEQUENCE LENGTH:215
SEQUENCE TYPE:nucleic acid
TOPOLOGY:linear
CLONE:HUMGS00707
SEQUENCE DESCRIPTION:
GATCATTGTA GATGANCTGA AGCAAGANGT TATCAGTACC AGCAGCAAGG CAGAACCACC 60
CCAGTGCACC TCCCTGGCCT GGNCTGCTGA TGGCCAGACT CTGTTTGCTG GCTACACGGA 120
CAACCTGGTG CGAGTGTGGC AGGTGACCAT TGGCACACGC TAGACGTTTA TGGCAGNGCT 180
TTACATGTGG GGGAATAACN TGGCTTTTCT GTAAA 215

SEQ ID NO:640
SEQUENCE LENGTH:220
SEQUENCE TYPE:nucleic acid
TOPOLOGY:linear
CLONE:HUMGS00708
SEQUENCE DESCRIPTION:
GATCAAACCA AGGCCCAGGC TGCAGCCCCA GCTTCAGTTC CAGCTCAGGC TCCCAAACGT 60
ACCCAGGCCC CTACAAAGGC TTCAGAGTAG ATATCTCTGC CAACATGAGG ACAGAAGGAC 120
TGGTGCGACC CCCCACCCCC GCCCCTGGGC TACCATCTGC ATGGGGCTGG GGTCCTCCTG 180
TGCTATTTGT ACAAATAAAC CTGAGGCAGG ATTTGTTAAA 220

SEQ ID NO:641
SEQUENCE LENGTH:210
SEQUENCE TYPE:nucleic acid
TOPOLOGY:linear

CLONE:HUMGS00710
SEQUENCE DESCRIPTION:
GATCGAGAAC CACATCCTCA AGCTCTTCGA GAGCAACCTG GTGCCCGCTA AACCCTGAGT 60
GAAGGCCGCC TGCCGGGGAC TCAGACACTC AGGGAACAAA ATGGTCAGCC AGAGCTGGGG 120
AAACCCAGAA CTGACTTCAA AGGCAGCTTC TGGACAGGTG GTGGGAGGGG ACCCTTCCCA 180
AGAGGAACCA ATAAACCTTC TGTGCAGAAA 210

SEQ ID NO:642
SEQUENCE LENGTH:208
SEQUENCE TYPE:nucleic acid
TOPOLOGY:linear
CLONE:HUMGS00711
SEQUENCE DESCRIPTION:
GATCTGGTTT TATTCCTGTA ATTCAGCCAC CTGATTTTGT GAGGGGGGGG AATAATATGT 60
GGTTTTTGTA CAAACATGTT TCTCAGTGTG TTGTNATTTT GGAAAAAATG AGGGGAGGGA 120
GTTTGGCAAG AATGGAGAAA ATGAATGAAG AAGGCCTAAT CTCTCTCTTT TTCAGTNAAT 180
AAATGGAACA CCATTTCTGG ATTCTAAA 208

SEQ ID NO:643
SEQUENCE LENGTH:206
SEQUENCE TYPE:nucleic acid
TOPOLOGY:linear
CLONE:HUMGS00712
SEQUENCE DESCRIPTION:
GATCTGTACA TTGTAAAACA CCATTCAAGT GTCAGAATCA TTATTTTCCA CCACTTATCA 60
TGGTGCTTGA CAAGTCTTCC CAATAAATAC TGAATGAACA AATGAATGGC AGAAACATTA 120
AAATGAACAC TATGGGGAAA GGGGAAGAGA GGCAGAATCA GAAATTATCC NGAATAAATA 180
TTTATNCCAT TTGTCATCCN NCNAAA 206

SEQ ID NO:644
SEQUENCE LENGTH:244
SEQUENCE TYPE:nucleic acid
TOPOLOGY:linear
CLONE:HUMGS00713
SEQUENCE DESCRIPTION:
GATCTATGTG GTGAAAATGC ACAGGAGCTT GGTAGACTGC GGGGGAAAGA GAGAGCTCCT 60
TTCGCCATGT TTTACCAGTN TGCTGTTATA ACCTCTTAGG TTGTATCCTT TAATTTCCAG 120
CCTTTTAGGT TAGTTTCTGT AACAGAACAA GTGAGTCTGG GATGAAGTCC TCAAAGTACT 180
TCAAATGGTA ATTTTTTTGT TTTTGTAATA GCTNAACAAA TAAACCNAGG GTTTCTATAT 240
TAAA 244

SEQ ID NO:645
SEQUENCE LENGTH:231
SEQUENCE TYPE:nucleic acid
TOPOLOGY:linear

CLONE:HUMGS00714
SEQUENCE DESCRIPTION:
GATCTATGAA TGANAGGAGG GCAGACCACA TTGCTTTTNA CATCCATTTC CCCTCCTTCC 60
CATGGGCAGA GGACCAGGCT GTAGGAAATC TAGTTATTTA CAGGAACTTC ATCATAATTT 120
GGAGGGAAGC TCTTGGAGCT GTGAGTTCTC CCTGTACAGT GTTACCATCC CCGACCATCT 180
GATTAAAATG CTTCCTCCCA GCATAGGATT CATTGAGTTG GTTACTTCAA A 231

SEQ ID NO:646
SEQUENCE LENGTH:200
SEQUENCE TYPE:nucleic acid
TOPOLOGY:linear
CLONE:HUMGS00715
SEQUENCE DESCRIPTION:
GATCTACATC TTTTTCTAAA GAAAAGTGGA GCTTGCCTCC AGTTCAATTC ACAAGAGCAT 60
TTTCCCTCCC ATGCCCACCT TTTCTTGTGG CTGTCGCTAG GAAGGATGCA GAGGCTGTGT 120
GGTTTACCAA ATGCCTTAAC TTAGCAGTGA ATGACAACTG TCAAACACAT GTTGAGGGGA 180
AATTTTTACT GATTCACAAA 200

SEQ ID NO:647
SEQUENCE LENGTH:200
SEQUENCE TYPE:nucleic acid
TOPOLOGY:linear
CLONE:HUMGS00716
SEQUENCE DESCRIPTION:
GATCTGCCGC AAGTGCTATG CTCGCCTTCA CCCTCGTGCT GTCAACTGCC GCAAGAAGNA 60
GTGTGGTCAC ACCAACAACC TGCGTCCCAA GAAGNNGGTC AAATAAGGTT GTTCTTTCCT 120
TGAAGGGCAG CCTCCTGCCC AGGCCCCGTG GCCCTGGAGC NTCAATAAAG TGTCCCTTTN 180
ATTGACTGGG GNNGNANAAA 200

SEQ ID NO:648
SEQUENCE LENGTH:199
SEQUENCE TYPE:nucleic acid
TOPOLOGY:linear
CLONE:HUMGS00717
SEQUENCE DESCRIPTION:
GATCCAGAAG GGGTTTGGTC TGGGACTTCC TTGCTCTCCC TCTTCTCAAG TGCCTTAATA 60
GTAGGGTAAG TTGTTAAGAG TGGGGNAGAG CAGGCTGGCA GCTCTCCAGT CAGGAGGCAT 120
AGTTTTTACT GAACAATCAA AGCACTTGGA CTCTTGCTCT TTCTACTCTG AACTAATAAA 180
TCTGTTGCCA AGCTGGAAA 199

SEQ ID NO:649
SEQUENCE LENGTH:206
SEQUENCE TYPE:nucleic acid
TOPOLOGY:linear
CLONE:HUMGS00718

SEQUENCE DESCRIPTION:
GATCTTTTGT CCTCACTGCT TTCTAATGGG GAGGGCTGAG GGTTCCCTGT CCCCACAGCA 60
GGTATGTTGG GNTCTGCCCC AGCCCCACAC TTGCTCTGAA AACCAAGTGN NAGAGCCCCT 120
TCCCCTTGTT TTTATTTTAC TGTTATAATA ATTATTAACT TCCTTGTAAT AGAAATAAAG 180
TTTGTACTTG GAGTTCAGCT CAGAAA 206

SEQ ID NO:650
SEQUENCE LENGTH:201
SEQUENCE TYPE:nucleic acid
TOPOLOGY:linear
CLONE:HUMGS00719
SEQUENCE DESCRIPTION:
GATCCTCGTT TTCTNGGTTT TGGTGATGTT GGAGGAGTAC CCCCCAGCCC ACCGCCCCGA 60
TTCCTTTTTG CTTCTGGTTT GGAGCTCCGG ACCAGGACCT TCGTCCTGGT CAGTTTTTAA 120
ATAATNATTT AGCAGTGTAA CTTTTAAACC TGCGTGACAT CTACANNNNG CCCAATAAAG 180
AAAGAGGAAG CCACGGTCAA A 201

SEQ ID NO:651
SEQUENCE LENGTH:198
SEQUENCE TYPE:nucleic acid
TOPOLOGY:linear
CLONE:HUMGS00720
SEQUENCE DESCRIPTION:
GATCTGTTAT CTAGCTGAGT TCATTTCATC TCTCCCTTTT TTATATCAAG TTTGAATTTG 60
GGATAATTTT NCTATATTAG GTACAATTTA TCTAAACTGA ATTGAGAAAA AATTACAGTA 120
TTATTCCTCA AAATAACATC AATCTATTTT NGTAAACCTG TTCATACTAT TAAATTTTGC 180
CCTAAAAGAC CTCTTAAA 198

SEQ ID NO:652
SEQUENCE LENGTH:197
SEQUENCE TYPE:nucleic acid
TOPOLOGY:linear
CLONE:HUMGS00721
SEQUENCE DESCRIPTION:
GATCTTAAAC ATCGGTCAGA TGAGTCATAC ATTGGGTTAT TTTTTATATA CATGTATACA 60
CAAAATATTT CAAATTGAAA GCAACATCTT AATGGATTCA AAACTATTAC AAGCTGTTGT 120
CTAAAACAGG TGAGAAAAAA ATTTATAACT GTAAAANCAA ATGCACATAT TGATATTTAA 180
AATGCGTAAT TAAGAAA 197

SEQ ID NO:653
SEQUENCE LENGTH:197
SEQUENCE TYPE:nucleic acid
TOPOLOGY:linear
CLONE:HUMGS00722
SEQUENCE DESCRIPTION:

GATCTGGACT GTCCTGGCAT CGAACTCTCC CTCTGTGTGT AATTGGAGGA GACCACAAGC 60
TGTTGTTTTG GGTGACTGAA GTATAAAGTG TTTNCTGTAC CTTAGATTCA CAAACTTTGT 120
ATTTTNAGTA CATATTTNNA AGAATTTCTA TAGTACATAT NTNNAAGAAT TTTNATATCA 180
AATATACCGT ATACTNN 197

SEQ ID NO:654
SEQUENCE LENGTH:201
SEQUENCE TYPE:nucleic acid
TOPOLOGY:linear
CLONE:HUMGS00723
SEQUENCE DESCRIPTION:
GATCACCGCC GCGAGTAAAA AAGGCTCCAG CCCAGAAGGT TCCTGCCCAG AAAGCCACAG 60
GCCAGAAAGC AGCGCCTGCT CCAAAAGCTC AGAAGGGTCA AAAAGCTCCA GCCCAGGNNN 120
GCACCTGCTC CAAAGGCATC TGGCAAGAAA GCATAAGTGG CAATCATAAA AAGTAATAAA 180
GGTTCTTTTT GACCTGTTAA A 201

SEQ ID NO:655
SEQUENCE LENGTH:199
SEQUENCE TYPE:nucleic acid
TOPOLOGY:linear
CLONE:HUMGS00725
SEQUENCE DESCRIPTION:
GATCTGCAGT TTAAGTTGCC ATGCTGCTAG GAAATTGTCC TTTTNCTTTC TAGCTGTTAA 60
CCTACTTCCT GGAAAAAGTA GTAGCTCTCT GTAGCATTAT GGAGTTTCAG TGGAACCAAA 120
TNTTTGCCAT TAAAAACTGG CATTATACTG AACTATACAT TGAGAAATCA ATCAAAATAA 180
AANTTTTNAC TTTCACAAA 199

SEQ ID NO:656
SEQUENCE LENGTH:196
SEQUENCE TYPE:nucleic acid
TOPOLOGY:linear
CLONE:HUMGS00726
SEQUENCE DESCRIPTION:
GATCTGGTTT CTAGCAAATT CCCAGTAGGA TGTCATGTAA GTCCTTCCCC CTCTTAGAGA 60
TTGAAGNTGT AAGAGCCAGA TGGTGGAGCA GGCTGTCTGG GTTCAAATGC ATCTTTGCAC 120
TTNAAGCTAA ATGACATACT CAAATTAATC GTCTGACTCA GTTCCTTGCT ATCAAATAAA 180
AAGATAGACC TGCAAA 196

SEQ ID NO:657
SEQUENCE LENGTH:201
SEQUENCE TYPE:nucleic acid
TOPOLOGY:linear
CLONE:HUMGS00727
SEQUENCE DESCRIPTION:
GATCTGCCTG CCTCAGCCTC TCAAAGTGTT GAGATTACAG GCGTGAGCAN CCGCTCCCTG 60

CCCAACACAT ATACCATCTG AAAATGTTAG AATTCTGAGT TGTGATTTTA TTGACTTGTT 120
GCTTGCTTTT CCTNAGGCTT TGTAACTTGT AATATGTTAA AGTGTACTAT CCTAATAAAC 180
TGAATACTTT GGTATCTTAA A 201

SEQ ID NO:658
SEQUENCE LENGTH:197
SEQUENCE TYPE:nucleic acid
TOPOLOGY:linear
CLONE:HUMGS00728
SEQUENCE DESCRIPTION:
GATCCTCCAT TGGAGTGGCC CAAATCTTTC CATCTAGGGC AAGTCCTGAA AGGCCCAAGG 60
CCCCCTCCCC AGTCTGGCCT TGGCCNCCAG CCTGGAGAAG GGCTAACATC AGCTCATTGT 120
CAAGGCCACC CCCACCCCAG AACAGAACCG TGTCTCTGAT AAAGGCTNTT GAAGTGAATA 180
AAGTTTTAAA ANCTAAA 197

SEQ ID NO:659
SEQUENCE LENGTH:195
SEQUENCE TYPE:nucleic acid
TOPOLOGY:linear
CLONE:HUMGS00729
SEQUENCE DESCRIPTION:
GATCGTNTTT GTTTTGTTTT TAAAGAAAGG TGAGATTGGC TTGGTTCTTC ATGAGCACAT 60
TTNATATAGC TCTTTTCCTG TTTTNCCTTG CTCATTTCGT TTTGGGGAAG AAATCTGTAC 120
TGTATTGGGA TTGTAAAGAA CATCTCTGCA CTCAGACAGT TTACAGAAAT AAATGTTTTT 180
TTTGTTTNNC AGAAA 195

SEQ ID NO:660
SEQUENCE LENGTH:196
SEQUENCE TYPE:nucleic acid
TOPOLOGY:linear
CLONE:HUMGS00730
SEQUENCE DESCRIPTION:
GATCTCAAAC TCCAGGCTCA GAACTGTGAA GACTGTTTCC AGCCTGGCTG TGAGCCAAGA 60
CCTGGTTCCT GGTGGACCCT GAGGACAAAG TGTGATAAAA CCTCTGGCTC AGACTTGCTC 120
TACTGAAGGC TTCTTGGTTA TAAGATGCAT AAAGTCACTG GGGCTAGCTA AACAATAAAG 180
AGTTTATTGT GAGAAA 196

SEQ ID NO:661
SEQUENCE LENGTH:194
SEQUENCE TYPE:nucleic acid
TOPOLOGY:linear
CLONE:HUMGS00731
SEQUENCE DESCRIPTION:
GATCTAATGT ACTGTAACTT TATCAGTGAA AGGTAAAATC TCAAATAACA AGTACAAACA 60
TTGAACAATT ACCTATAAAG ATTTNTAAAA GTAAAATTTT TCCAATAGAT TTCATTCTTG 120

TCATTTTGTA AGACGACCCT GCAGTCCACC NGTTTGTAAC TTTTTTAATA AAATAGACAT 180
CTGTATTACT GAAA 194

SEQ ID NO:662
SEQUENCE LENGTH:246
SEQUENCE TYPE:nucleic acid
TOPOLOGY:linear
CLONE:HUMGS00732
SEQUENCE DESCRIPTION:
GATCAAGAAA ATAAGGACAA CGTGAAGTTT AAAGTTCGAT GCAGCAGATA CCTTTACACC 60
CTGGTCATCA CTGACAAAGA GAAGGCAGAG AAACTGAAGC AGTCCCTGCC CCCCGGTTTG 120
GCAGTGAAGG AACTNAATAA ACCAGACACA CACAGAACAG GCGATTATTT ATTTGTTTTT 180
AATTTATTTT GTCATATTTT TGTAAAACGG CAGAAATGCA ATAAAACCTA TATTTCAACA 240
GTGAAA 246

SEQ ID NO:663
SEQUENCE LENGTH:192
SEQUENCE TYPE:nucleic acid
TOPOLOGY:linear
CLONE:HUMGS00733
SEQUENCE DESCRIPTION:
GATCATATTT TATACATGTG TAATAGATAA AAATAAACCA GATTGCAAAT CCTTTTTTAA 60
AATCCTAAAC CATGTACCAA GTTTTTGGTC CAAATTATGT AGGATAAGTT AAACTTAAAT 120
TGCATTCTAT TAACCAATAT GAGTGTATTT CTGTAAGCAT AGTTATGTTG AAATAAAGTT 180
TTAAAAACCA AA 192

SEQ ID NO:664
SEQUENCE LENGTH:191
SEQUENCE TYPE:nucleic acid
TOPOLOGY:linear
CLONE:HUMGS00734
SEQUENCE DESCRIPTION:
GATCTTCACA CTAATGATGA GTGTGTGGCT ACATACAAAG GAGTTCCCTT TGAGGTGAAA 60
GGGAAGGGAG TATGTAAGGG CTCAAACCAT GAGCAACAAG TGGAATCAAA TAAAATGCTT 120
CCACNACCAA AAGACATTAG AGAAAACCTT AAAAGTAATA AAGNGAAATA TATTTNTCAC 180
TTATACCTAA A 191

SEQ ID NO:665
SEQUENCE LENGTH:188
SEQUENCE TYPE:nucleic acid
TOPOLOGY:linear
CLONE:HUMGS00735
SEQUENCE DESCRIPTION:
GATCTGAATC TNCCGGGGCC CCAGCCCACT CCACCCTGCC AGCAGCTTCC AGCCAGTCCC 60
CACAGCCTCA TCAGCTCTCT TCACCGTTTT TTGATACTAT CTTCCCCCAC CCCCAGCTAC 120

CCATAGGGGC TGCAGAGTTA TAAGCCCCAA ACAGGTCATG CTCCAATAAA AATGATTCTA 180
CCTACAAA 188

SEQ ID NO:666
SEQUENCE LENGTH:186
SEQUENCE TYPE:nucleic acid
TOPOLOGY:linear
CLONE:HUMGS00736
SEQUENCE DESCRIPTION:
GATCTNAAAC CCAAGGGTCT GAGGCCAGGG CCGACTGCCG TAAGATGGGT GCTGAGAAGT 60
GAGTCAGGGC AGGGCAGCTG GTATCGAGGT GCCCCATGGN AGTAAGGGGA CGNCTTCCGG 120
GCGGATGCAG GGCTGGGGTC ATCTGTATCT AAAGCCCCTC GGAATAAAGC GCGTTGACCG 180
NCGAAA 186

SEQ ID NO:667
SEQUENCE LENGTH:184
SEQUENCE TYPE:nucleic acid
TOPOLOGY:linear
CLONE:HUMGS00737
SEQUENCE DESCRIPTION:
GATCTGTAAA TAATCATTGC CAGTNTGACT TTTGTTCAAC AAAAGGATTG TACTGTATTA 60
AGAACCGATG AAAAAAATTN TCCTGTAACA TTTTTTTAAG AAAACTTTGT TTGTTTAAAG 120
AAAAAGTATT GTATAANTNA TAATTTTAAT TTAAATAAAC CTAAAATGCT TTGTGCTAAG 180
GAAA 184

SEQ ID NO:668
SEQUENCE LENGTH:180
SEQUENCE TYPE:nucleic acid
TOPOLOGY:linear
CLONE:HUMGS00738
SEQUENCE DESCRIPTION:
GATCCCAGAA AAGTTCTAAT TTTCATTAGC AATTAATAAA GCTATACATG CAGAAATGAA 60
TACAACAGAA CACTGCTCTT TTTGATTTTA TTTGTACTTT TTGGCCTGGG ATATGGGTTT 120
TAAATGGACA TTGTCTGTAC CAGCTTCATT AAAATAAACA ATATTTGTAA AAATCATAAA 180

SEQ ID NO:669
SEQUENCE LENGTH:179
SEQUENCE TYPE:nucleic acid
TOPOLOGY:linear
CLONE:HUMGS00739
SEQUENCE DESCRIPTION:
GATCTATCCA AGGTTTTGAC ATGTATCGAG AGTTTATTCC TTTTTATTGC TGAATAGTAT 60
TAATATTCTA TAGTATGGAT GTAACATAGT GTGTTTAAAC ATTCGCCTGT TGAAGGACAC 120
TTGGGTTGTT TCCAGTTTCA GGTTCTTACA AATAAAGCTA CTCTGTGTGT TCATGTAAA 179

SEQ ID NO:670
SEQUENCE LENGTH:179
SEQUENCE TYPE:nucleic acid
TOPOLOGY:linear
CLONE:HUMGS00740
SEQUENCE DESCRIPTION:
GATCAAATGC CAGTGTCATT TTGTACTTAA GTTCCAAAGT AGGAACATTN TATACTTTTT 60
NCTGTATTGT AATAGGTAGT TTTGTATGAA ATCTTTTCTC CTCTCCCGTT GTACCGCATT 120
CTTTCCAGCA TTGTGCTTTT TCCCTGGNCT TATTTGAAAA TTTTACTGTT TTATACAAA 179

SEQ ID NO:671
SEQUENCE LENGTH:177
SEQUENCE TYPE:nucleic acid
TOPOLOGY:linear
CLONE:HUMGS00741
SEQUENCE DESCRIPTION:
GATCCAAGAA ACCAGGGCCA TGACCAGGTC CACTGTGGAG CAGCCATCTA TCTACCTGAC 60
TCCTGAGCCA GGCTGCCGTG GTGTCATTTC TGTCATCCGT GCTCTGTTTC CTATTGGAGT 120
TTCTTCTCCA CATTATNTTT GTTCCTGGGG AATAAAAACT ACCATTGGAC CTAGAAA 177

SEQ ID NO:672
SEQUENCE LENGTH:175
SEQUENCE TYPE:nucleic acid
TOPOLOGY:linear
CLONE:HUMGS00742
SEQUENCE DESCRIPTION:
GATCTGCAAG TCCCGGAGAG CAACAGCACA GCTCTGCCTG ACGCTCTCAT TAAAATCTAT 60
GCAGCCAAGC TCGGCACTTT GTAGCAGCCG GCCTTGCGAA GCCTCCTCAG CTCGGGGGGC 120
CGGGGACCCA GTGAGCCGAG AGCCCTCTGG NCTCCACTTA TGCATATGCA CCAAA 175

SEQ ID NO:673
SEQUENCE LENGTH:180
SEQUENCE TYPE:nucleic acid
TOPOLOGY:linear
CLONE:HUMGS00743
SEQUENCE DESCRIPTION:
GATCCGGAGA ATAGGGCNNN AATATGTGCC GCCAGTGTTT CCGTCAGTAC GCGAAGGATA 60
TCGGTTTCAT TAAGTTGGAC TAAATGCTCT TCCTTCAGAG GATTATCCGG GGCATCTACT 120
CAATGAAAAA CCATGATAAT TCTTTGTATA TAAAATAAAC ATTTGAAAAA ACCCTTCAAA 180

SEQ ID NO:674
SEQUENCE LENGTH:176
SEQUENCE TYPE:nucleic acid
TOPOLOGY:linear
CLONE:HUMGS00745

SEQUENCE DESCRIPTION:
GATCTATTCA GATGGTTCTG ATGAAGTGAA ACGNGCCATG AACAAATCCT TTATCNNNGT 60
CGGGTGGTAC AGTTTTGAGT ACCAACTGGT CTGATGTAGG TAAAAGGAAA GTTGAAATCA 120
ATCCTCCTGA TGATATGGNN NGGAAAAAGT ACTAAATAAA TTAATTTGCT CTCAAA 176

SEQ ID NO:675
SEQUENCE LENGTH:173
SEQUENCE TYPE:nucleic acid
TOPOLOGY:linear
CLONE:HUMGS00746
SEQUENCE DESCRIPTION:
GATCAAAACC AGTTTGATTT GGGAATCTTC CCCTTTCCAA ATGAAATAGA GATGCAGTAC 60
TTAACTTTCC TTGGTGTTTG TAGATATTGC CTTGTGTATT CCACTTAAAA CCGTAATCTA 120
GTTTGTAAAA GAGATGGTGA CGCATGTAAA TAAAGCATCA GTGACACTCT AAA 173

SEQ ID NO:676
SEQUENCE LENGTH:173
SEQUENCE TYPE:nucleic acid
TOPOLOGY:linear
CLONE:HUMGS00747
SEQUENCE DESCRIPTION:
GATCCTAGCA TCCCTTTTCA CATGGTTTCT CCATGTATAT AACAGAATCA AGAAACAAAT 60
TTNAATTAAA CAATCTGTAA CAGAATCAAG AAACAAATAC ATTTTAATTA AACAATCTAT 120
ATGGAACAAA CATTCCCAAA TNCTAAGAAT AANTNTTCNT NTAAGTTTTC AAA 173

SEQ ID NO:677
SEQUENCE LENGTH:182
SEQUENCE TYPE:nucleic acid
TOPOLOGY:linear
CLONE:HUMGS00748
SEQUENCE DESCRIPTION:
GATCCAAATA ATTTATTAAA TGACTGGTCT CAGAAACTGA ACTCATTAAT GTCTCTGGTT 60
AACAAAACTA CGCATCTCAT AGCCAAAGAG GAGATGATAC ATAATCTACA ATAAGGGTCT 120
TAGTGCTTTA GAAAAAAGTT AAAATTGGAA GTCATTAAAA AAAGACTGTT ATAATGGTGA 180
AA 182

SEQ ID NO:678
SEQUENCE LENGTH:173
SEQUENCE TYPE:nucleic acid
TOPOLOGY:linear
CLONE:HUMGS00749
SEQUENCE DESCRIPTION:
GATCTTATGA GAAGAGGAAG GAGAGANGTC TCTNCCTATG CTCACACACA AAGGAAAGTC 60
CACATGAGGA CACAAGGAGA AGCCAAGAGN GCCCTTACCA GGANTTAAAC CTGCTAATCC 120
CTATTTTAAT TAGAACAGTG AGAAANTAAA TATCTGTATT TAAGCCTCAC AAA 173

SEQ ID NO:679
SEQUENCE LENGTH:173
SEQUENCE TYPE:nucleic acid
TOPOLOGY:linear
CLONE:HUMGS00750
SEQUENCE DESCRIPTION:
GATCCAGAGA AAGCCAAAGC TCTTAGTAAA CACTTGCCAT CGTCAGTNTN ATGTCTCTAA 60
AAGTAGATGT TGAGGCTCTT NAAAATNCTC CTGGTGCTAC ATACATTCGG AAGAAGGGTG 120
GAAAAGTTAC TGGAGATAGT CAACCAAAGG AACAAGGACA GGNAGATTTG AAA 173

SEQ ID NO:680
SEQUENCE LENGTH:172
SEQUENCE TYPE:nucleic acid
TOPOLOGY:linear
CLONE:HUMGS00751
SEQUENCE DESCRIPTION:
GATCCAATCC ATTCAGCAGT CCATTGAAAG GCTCTTAGTC TAAACCTGTG GCCTCTGCCA 60
CGTNGCTCCC TGCCAGCTTC CCCCCTGAGG TTGTGTATCA TATTATCTGT GTTAGCATGT 120
AGTATTTTCA GCTACTCTCT ATTGTTATAA AATGTAGTAC TAAATCTGGA AA 172

SEQ ID NO:681
SEQUENCE LENGTH:170
SEQUENCE TYPE:nucleic acid
TOPOLOGY:linear
CLONE:HUMGS00752
SEQUENCE DESCRIPTION:
GATCAAATTT AAACTTCATT TTGGGGGGTA TTTTGGTACT GTAATGGGGT CATCAAATNA 60
TTAATCTGAA AANAGCAACC CAGAATGTAA AAAAGAAAAA ATTGGGGGGA AAAAGACCAG 120
GTCTACAGTG ATAGAGCAAA GCATCAAAGA ATCTTTAAGG GAGGTTTAAA 170

SEQ ID NO:682
SEQUENCE LENGTH:239
SEQUENCE TYPE:nucleic acid
TOPOLOGY:linear
CLONE:HUMGS00753
SEQUENCE DESCRIPTION:
GATCGGAACA GCTCCTTACT CTGAGGAAGT TGATTCTTAT TTGATGGTGG TATTGTGACC 60
ACTGAATTCA CTCCAGTCAA CAGATTCAGA ATGAGAATGG ACGTTTGGTT TTTTTTTGTT 120
TTTGTTTTTG TTTTTTCCTT TATAAGGTTG TCTGTTTTTT TTTTTNAAAA AATNGCANCA 180
GTNCATGGCC CCCATCATTA ANANGNGAGG ANTNCANCAG AAAATAAAAT ATNCACTCN 239

SEQ ID NO:683
SEQUENCE LENGTH:170
SEQUENCE TYPE:nucleic acid

TOPOLOGY:linear
CLONE:HUMGS00754
SEQUENCE DESCRIPTION:
GATCCAGACA GCCATCAGGG AAAGCTTGTN TTTAACCGAA CTGTCACACT GAAGGAAGAC 60
CCAGGAAAGG TGTGAGCTGG AAGCACTGAA CCTACCTCAT CCTCCTGGAG GGTGTGGCTA 120
CCCTCGCCAC CCCAAATTCC ATGTCAATAA AGAACAGCTA AATTCTCAAA 170

SEQ ID NO:684
SEQUENCE LENGTH:168
SEQUENCE TYPE:nucleic acid
TOPOLOGY:linear
CLONE:HUMGS00755
SEQUENCE DESCRIPTION:
GATCTTTCCA TTGGAAATAA CTGGAAGTGA AGAGGTTTTG TTGCTTGTAC AGTGTCAGAT 60
GAGGAACACC ACTATCTTAA TTTTGTGATA CACTGCATTT GCTGGTGCTA TTTTNATACA 120
GTGAAGCAAC AGCTTTGCAG CAAAATAATA AAATACTTCT ANGTTAAA 168

SEQ ID NO:685
SEQUENCE LENGTH:169
SEQUENCE TYPE:nucleic acid
TOPOLOGY:linear
CLONE:HUMGS00757
SEQUENCE DESCRIPTION:
GATCCATCGT NATGTCTTAT TTAAGGGGAA CGTGTGGNCT ATTTAGGCTT TATGGCCCTG 60
AAGTAGGAAC CAGATGTCGG ATACAGTTCA CTTTAGCTAC CCCCAAGTNT TATGGGCCCG 120
GAGCGAGGAG AGTAGCACTC TTGTNCGGGA TATTGATTTC ACGGAGAAA 169

SEQ ID NO:686
SEQUENCE LENGTH:171
SEQUENCE TYPE:nucleic acid
TOPOLOGY:linear
CLONE:HUMGS00758
SEQUENCE DESCRIPTION:
GATCTGCTAC TAAACAGAGT TCAAAAACTT TCCAGAGTAA TTAATATGTA AAGCCANGTA 60
ACTAACAAAN GATTTGCTTT AGAGATAATT ATTTGGAATT TTTATAGCTN ACTTCACAAT 120
GTGCCCAGGT CAGCTGTATA AAATAAATAC TGCATTNTNG TTTCTTTCAA A 171

SEQ ID NO:687
SEQUENCE LENGTH:167
SEQUENCE TYPE:nucleic acid
TOPOLOGY:linear
CLONE:HUMGS00759
SEQUENCE DESCRIPTION:
GATCCTGACA CTAAGGAAAT GCTGAAGCTT TTGGACTTCG GCAGTCTGTC CAACCTTCAG 60
GTCACTCAGC CTACAGTTGG GATGAATTTC AAAACGCCTC GGGGACCTGT TTGAATTTTT 120

NCTGTAGTGC TGTATTATTT TCAATAAATC TGGGACAACA GCAGAAA 167

SEQ ID NO:688
SEQUENCE LENGTH:166
SEQUENCE TYPE:nucleic acid
TOPOLOGY:linear
CLONE:HUMGS00760
SEQUENCE DESCRIPTION:
GATCAGCGAT GAAGAGGAAG AAGATGATGA TTGAAGTATG AAATATGAAA ACATTTTATA 60
TATTTNATTG TACAGTTATA AATATGTAAA CATGAGTTAT TTTGATTGAA ATGAATCGAT 120
TTGCTTTTGT GTAATTTTAA TTGTAATAAA ACANTTTAAA AGCAAA 166

SEQ ID NO:689
SEQUENCE LENGTH:175
SEQUENCE TYPE:nucleic acid
TOPOLOGY:linear
CLONE:HUMGS00761
SEQUENCE DESCRIPTION:
GATCTATTTG GTCTTTCTCA TGTCCCCCAC TGGTCTGTAC CCCAGGGAGC GGGTGCTTGT 60
ACTGTGTGAA TCCAGTGTTC ACATTCACAC TTAATGACTT CCTTGGCACC AATCATGTAT 120
TTCACCGTTT GCACTTNTTG TATTTCAATA AAAATGTTGA TGCAAAACTG CTAAA 175

SEQ ID NO:690
SEQUENCE LENGTH:165
SEQUENCE TYPE:nucleic acid
TOPOLOGY:linear
CLONE:HUMGS00762
SEQUENCE DESCRIPTION:
GATCGACTCT ATCATCCAAC GCTCCGAGGA CAGCCCATGT CCCCACCCCN GNGACCCGGA 60
CCCGGCCAGC AGGACCCACT GAGANGGGCT GCCCGGGNCT NCTCAGCTGC CCACACCCAC 120
ACTGTCCAGC ATCTGGCACA ATAAACATNC TCTGTTTTGT AGAAA 165

SEQ ID NO:691
SEQUENCE LENGTH:164
SEQUENCE TYPE:nucleic acid
TOPOLOGY:linear
CLONE:HUMGS00763
SEQUENCE DESCRIPTION:
GATCAAGAAA TAAACATAAA CATCTCAGAA TGCTCCTTCA TTACCAGAGT CACTACCTGA 60
TTATGTCTTA ATGGGTTACA TAATGACAGA GGGTATCTCA TATATGTNCT TTTCCAAACA 120
TAAAATAACT TTTTGTTTTG TTTGATTGAA AAAAAATTTA GAAA 164

SEQ ID NO:692
SEQUENCE LENGTH:171
SEQUENCE TYPE:nucleic acid

TOPOLOGY:linear
CLONE:HUMGS00764
SEQUENCE DESCRIPTION:
GATCCAGATT CTCACTTAAT GGGGTTTATA TGGACTTTCT TCTCATAAAT GGCCTGCCGT 60
CTCCCTTCCT TTGAAGAGGA TATGGGGATT CTGCTCTCTT TTCTTATTTA CATGTAAATA 120
ATACATTGTT CTAAGTCTTT TNCATTAAAA ATTTAAAACT TTTCCCATAA A 171

SEQ ID NO:693
SEQUENCE LENGTH:164
SEQUENCE TYPE:nucleic acid
TOPOLOGY:linear
CLONE:HUMGS00765
SEQUENCE DESCRIPTION:
GATCGACTCT ATCATCCAAC GCTCCGAGGA CAACCCNTGT CCCCACCCCC GGGACCCGGA 60
CCCGGCCAGC AGGACCCACT GAGAAGGGCT GCCCGGNTCA CCTCAGGGGG NCACANTTTA 120
CACTCTCCAG CATCTGGCAC AATAAACATN CTCTGTTTTG TAAA 164

SEQ ID NO:694
SEQUENCE LENGTH:168
SEQUENCE TYPE:nucleic acid
TOPOLOGY:linear
CLONE:HUMGS00766
SEQUENCE DESCRIPTION:
GATCTAATGG TCTAAAAACT GGAGTTTCCG GACACAAGCT CTCCCTTTNC CTGCAACCAT 60
CCATGCAAGA TGTNACTTGC TCCTCTNTGC TTTCTGCCAT GATTGTAAGG CCTCCCCAGC 120
TACATGGAAC TNTAACTCCA TTAAACCTCT TTNTTTTGTA AATTGAAA 168

SEQ ID NO:695
SEQUENCE LENGTH:160
SEQUENCE TYPE:nucleic acid
TOPOLOGY:linear
CLONE:HUMGS00768
SEQUENCE DESCRIPTION:
GATCCGCCCA CCTCAGCCTC CTAAAGTGCT GGGATTACAG CTGTGAGCCA CCCTGCCCGG 60
CCACTTTTGT ATGATTTCTA ATGTATTTGT AATTTACCTA ACAAATTGCC TAATCTGCTA 120
TGTTAATGTA TTTATGAATT AAAATAAATA CGACTGCAAA 160

SEQ ID NO:696
SEQUENCE LENGTH:161
SEQUENCE TYPE:nucleic acid
TOPOLOGY:linear
CLONE:HUMGS00769
SEQUENCE DESCRIPTION:
GATCCGTAGC GGCATGTTCT GGCTGCGCTT CTAGGCGGGA AGCCTATGTA AGCAAGAGGG 60
CAGGGCCGGG GTTTGTGGTC CCCCCCCCAC CACAAACACA GCACTTCGGC TCCTCTAACC 120

TGTGCCACAG GTGACCACCA ATAAAATCCT CTGCTGAGAA A 161

SEQ ID NO:697
SEQUENCE LENGTH:159
SEQUENCE TYPE:nucleic acid
TOPOLOGY:linear
CLONE:HUMGS00770
SEQUENCE DESCRIPTION:
GATCTCCCTG CCCCCACCCC AGTTCCCCAA CCCACTCCCT TCCAACAACA ACCAGCTCCA 60
ACTGACTCTG GTCTTGGGAG GTGAGGCTTC CCAACCACGG AAGACTACTT TAAATGAAAA 120
AANGAAATTG AATAATAAAA TCAGGAGTCA AAATTCAAA 159

SEQ ID NO:698
SEQUENCE LENGTH:159
SEQUENCE TYPE:nucleic acid
TOPOLOGY:linear
CLONE:HUMGS00771
SEQUENCE DESCRIPTION:
GATCTTTTNN TTTTTTTAA GTCTCACAAG ACATGGGGCA TCTCCACAAA TTTAAGTTCC 60
TGTCCATTTG GAAATTTGTT TCTATGTGTA CAGTTTGTCA GAGAAAAACA AAGTTTTTGT 120
ATGANTACAG AATGTGATTT ACGCAAGATT TGACAGAAA 159

SEQ ID NO:699
SEQUENCE LENGTH:161
SEQUENCE TYPE:nucleic acid
TOPOLOGY:linear
CLONE:HUMGS00772
SEQUENCE DESCRIPTION:
GATCTACCTA TCTTCAGGAT GGAACCTTGG GGAAAAATAA AATTGAGGGG AAGTAAAAAG 60
TATGTAACAC TTCCAGTTGT GAGCCAAGAT TGTAACCAGA GAGCAGCCAG GAGCTTCCTG 120
TCAGTAACCA TNTTTTCAAT AAATACTCTT TCATGTACAA A 161

SEQ ID NO:700
SEQUENCE LENGTH:157
SEQUENCE TYPE:nucleic acid
TOPOLOGY:linear
CLONE:HUMGS00773
SEQUENCE DESCRIPTION:
GATCCTCTCA CCTCAGATTT CCAAAGTGTT GGATTATAGG TGTAAGCCAC TGAGCCCAGT 60
TTGAATGCTT TTTTATATAT TTTTTGGCCA TTTGTATGTC ATCTTTGGAG AAATGTCTAT 120
NCAAATCCNT TGCTCATTAA AATNATTTNC TGATAAA 157

SEQ ID NO:701
SEQUENCE LENGTH:160
SEQUENCE TYPE:nucleic acid

TOPOLOGY:linear
CLONE:HUMGS00774
SEQUENCE DESCRIPTION:
GATCTGGCCG TNAGCCGCGA NCCGCTGNGA ACTCCACTCG GGGAACTCCT TTCCAAGCTG 60
ACCTCAGTTT TCTCACAAGA ACCCAGTTAG CTGATGTTTT ATTGTAATTG TCTTAATTTG 120
CTAAGAACAA GTAATAAGTA AATTTTTAAA AAGCCTTAAA 160

SEQ ID NO:702
SEQUENCE LENGTH:161
SEQUENCE TYPE:nucleic acid
TOPOLOGY:linear
CLONE:HUMGS00775
SEQUENCE DESCRIPTION:
GATCAAATTG TGCAGTACTT TGTGCATTCT GGATTTTAAA AGTTTTTNAT TATGCATTAT 60
ATCAAATCTA CCACTGTATG AGTGGAAATT AAGACTTTAT GTAGTTTTNA TATGTTGTAA 120
TATTNCTCCA AATAANTCTC TCCTATAANC CACCAGGGAA A 161

SEQ ID NO:703
SEQUENCE LENGTH:154
SEQUENCE TYPE:nucleic acid
TOPOLOGY:linear
CLONE:HUMGS00777
SEQUENCE DESCRIPTION:
GATCAGAGCT TATACTTAAT TAAGGTTTTA TACACACCAG TTCCCCAGTA AATNCAAATT 60
TAACAAGAAA ATCAGACATG TCATATGTNC AAAATGCTCA TGGCAAACAA TCATTTTGCA 120
TTCCTGCAAA TAAAATTGTT TTATACTGTA GAAA 154

SEQ ID NO:704
SEQUENCE LENGTH:153
SEQUENCE TYPE:nucleic acid
TOPOLOGY:linear
CLONE:HUMGS00778
SEQUENCE DESCRIPTION:
GATCTGTTTG TTCCCTGAGC TTTTTAAATA CCCTGTGAAA ATTTTNTTTC CTCCCTTGGT 60
CATCATGCAT CTAATTGTGG GGAAATGTTT GTCAAACCAA CCTGCAAAGC AGCATGGTGT 120
AGTTGAGAAG AATAAACAGA GAAGACTGGG AAA 153

SEQ ID NO:705
SEQUENCE LENGTH:152
SEQUENCE TYPE:nucleic acid
TOPOLOGY:linear
CLONE:HUMGS00779
SEQUENCE DESCRIPTION:
GATCTTTGGA CAGAAGCAGC TCTTTCCCGA ACACTTGTGG CGTCTGGNAC GGCCCCACCC 60
NTCCCCCCAC ACTCCCTCCC ACGGGGCTCC GGGAGACAGG CCGGCCCTGC ACCTNACCCC 120

ACCGTGACCT CAATAAACGT TGAAACTNCA AA 152

SEQ ID NO:706
SEQUENCE LENGTH:152
SEQUENCE TYPE:nucleic acid
TOPOLOGY:linear
CLONE:HUMGS00780
SEQUENCE DESCRIPTION:
GATCTATCCT TTACTTGAAA GCTTTTGAAA AGTGGAAAGG TCATTTTGTT GCATTTCCCC 60
ATTTCTTGTT TTTAAAAGAC CAACAAATCT CAAGCCCTAT AAATGGCTTG TATTGAACTT 120
TTACATTTGA ATTAAAGATG TTAAACATGA AA 152

SEQ ID NO:707
SEQUENCE LENGTH:151
SEQUENCE TYPE:nucleic acid
TOPOLOGY:linear
CLONE:HUMGS00781
SEQUENCE DESCRIPTION:
GATCTAGTAT GCTCCTGGTC TAATGCATTT ACATTGTTTA GGTAACTGGT TCCTAATAAA 60
AAGAATTATA AAATACCCTC AAATTAACAA TTCAATNGCA TATAATAGCC TAACTCAGTA 120
AGANTATTAA AACTTACTAT TATNCTTCAA A 151

SEQ ID NO:708
SEQUENCE LENGTH:152
SEQUENCE TYPE:nucleic acid
TOPOLOGY:linear
CLONE:HUMGS00782
SEQUENCE DESCRIPTION:
GATCTANTCT GTGCTACCTG ATTAACTCAC AGCAGGCTTA CTGANTGGCT TCATTTCAGA 60
TTTAGTTGAT TTCTCCACCA AATNCATGTC ATGTATTCTC AATAGGCTGT ATTCCCAGCA 120
GNCAATAAAT GGAACACCCG TANAANCNCA AA 152

SEQ ID NO:709
SEQUENCE LENGTH:147
SEQUENCE TYPE:nucleic acid
TOPOLOGY:linear
CLONE:HUMGS00783
SEQUENCE DESCRIPTION:
GATCCCCAGG GTTTCTTTGT CTTATTTATG GAGAAAAACC GGTCACTTTG TCCAGCGCAC 60
TGTGAGGCCC CCACTCAGGC CAGCCCTGGC CCCCCCTTGG TACTTGGAAC CGAAGTTACA 120
GATTATATTA AAATAATAAT GTACAAA 147

SEQ ID NO:710
SEQUENCE LENGTH:146
SEQUENCE TYPE:nucleic acid

TOPOLOGY:linear
CLONE:HUMGS00784
SEQUENCE DESCRIPTION:
GATCTTATAA AAGGAAATTC TAGCAGTTTT AGAAATAGGT GGGAAAAACT CAAATATTCC 60
TCCTATCTGC ACCAAAAAGT TTATTTGTGG TATATAAAAT GAATATTGTT TTATAATAAC 120
TTGTTAATAA AGTACTTTCT AATAAA 146

SEQ ID NO:711
SEQUENCE LENGTH:145
SEQUENCE TYPE:nucleic acid
TOPOLOGY:linear
CLONE:HUMGS00785
SEQUENCE DESCRIPTION:
GATCAATGAA GTGAGAAATT GTTGAGAAGG ATACAGTTTG TTTTTAGATG TCCTTTGTCC 60
AATGTGAACA TTTATTCATA TTGTTTTGAT TACCCTCGTG TTACTACAAG ATGGCAATAA 120
ATACTATGGG ATTGTTTGTA TTAAA 145

SEQ ID NO:712
SEQUENCE LENGTH:145
SEQUENCE TYPE:nucleic acid
TOPOLOGY:linear
CLONE:HUMGS00786
SEQUENCE DESCRIPTION:
GATCTCCCTT CAGCAACTTA TTTTGCTTTA ATTGCTTTAA ATCTTAAGCA ATATTTTTAA 60
TTCAGTAAAC AAATTCTTTC ACAAGGTACA AAATCTTGCA TAAGCTGAAC TAAAATAAAA 120
NTGAAAAGGA GAGATTAANG GTAAA 145

SEQ ID NO:713
SEQUENCE LENGTH:145
SEQUENCE TYPE:nucleic acid
TOPOLOGY:linear
CLONE:HUMGS00787
SEQUENCE DESCRIPTION:
GATCTAGAAG CAGAGGAATC CCAGCGCCTT TTAAAAGTTG TTATGTGGTT TTCTTTTAAA 60
AAGCTCCTGT TTTTGGAAAG TAGAATTTAT GGGTACAACG TATGTTCATT ATTTGTACAT 120
AAAATAAAAC CATTTAATAA GTAAA 145

SEQ ID NO:714
SEQUENCE LENGTH:154
SEQUENCE TYPE:nucleic acid
TOPOLOGY:linear
CLONE:HUMGS00788
SEQUENCE DESCRIPTION:
GATCTATGCC TAACAGAGCC CCAGTACAAC TATTTTNCAG AATGGCTGTT ACCCTAGAAT 60
TACTATAGCA CATATTGAGA TATAGTTGTA CTCCCTAGTA GATAGGAACT GACCCCAACA 120

ATAAACTTTG ATAATAAAGA AAAAAAANCG NAAA 154

SEQ ID NO:715
SEQUENCE LENGTH:144
SEQUENCE TYPE:nucleic acid
TOPOLOGY:linear
CLONE:HUMGS00789
SEQUENCE DESCRIPTION:
GATCAACCTT AAAGGAAACT GCTATCCGAA CTTGGCTATC TCACAGCAGA GCCAGTTTGA 60
CGAATGGGTA AAACCTAAGG ACATGCTGGG TCCAAAGTGA TTTACATAAA TNTATAATGA 120
AAATAAACAT GTATAANATT TAAA 144

SEQ ID NO:716
SEQUENCE LENGTH:149
SEQUENCE TYPE:nucleic acid
TOPOLOGY:linear
CLONE:HUMGS00790
SEQUENCE DESCRIPTION:
GATCCGTGAT GCCACTTACC TGTGTGTTTG GTAACAACAA ACCAACATCA TGGAGGTCCC 60
TGGATTGAAA AAGGAGCCTC TCCCACTCCT CCTACCACCA AAGTGGTTAG GACNCTATAT 120
AANTAAAAAC AAGGCTTTTG GAAAATAAA 149

SEQ ID NO:717
SEQUENCE LENGTH:147
SEQUENCE TYPE:nucleic acid
TOPOLOGY:linear
CLONE:HUMGS00791
SEQUENCE DESCRIPTION:
GATCTGGCTG AACCAGTTCC ACAAGGTTAC TGTATACATA GCCTGAGTTT AAAAGGCTGT 60
GCCCACTTCA AGAATGTCAT TGTTAGACTT TGAAATTTCT AACTGCCTAC CTGCATAAAG 120
AAAATAAAAT CGTTTTAAAT CAAGAAA 147

SEQ ID NO:718
SEQUENCE LENGTH:115
SEQUENCE TYPE:nucleic acid
TOPOLOGY:linear
CLONE:HUMGS00792
SEQUENCE DESCRIPTION:
GATCTGGGGC AGCCACCTTG CTACCATGAA GGAAAGGCCA AGACAATCAT CCACAGCTAT 60
TCCCTCCAGC ATCTGGTTCT GTACAAAAAT TAAATGCTTA TTTNTTTAAG TCAAA 115

SEQ ID NO:719
SEQUENCE LENGTH:142
SEQUENCE TYPE:nucleic acid
TOPOLOGY:linear

CLONE:HUMGS00793
SEQUENCE DESCRIPTION:
GATCGCCACT GTAAAGGTCC TAGAGTTGCC TGTTTGTCTC TGGAGATGGA ATTAAACCAA 60
ATAAAGAGCT TCCACTGGAG GCTTGTATTG ACCTTGTAAC TATATGTTAA TCTCNTGTTA 120
AAATAAAATA TAGCTTGTGA AA 142

SEQ ID NO:720
SEQUENCE LENGTH:142
SEQUENCE TYPE:nucleic acid
TOPOLOGY:linear
CLONE:HUMGS00794
SEQUENCE DESCRIPTION:
GATCTGTGCC TACNTTTTAC CACCCTCTTG ATTGGAGCTT TTGTNATGCA GCTACCATNN 60
TTCAAAAAAA TTAAAAATTA AAAAAAAAAA ATCTGCCACT TATCCAAGTC CACTAGAGGC 120
CACTGTCTTC AAAGNTTNTN TN 142

SEQ ID NO:721
SEQUENCE LENGTH:147
SEQUENCE TYPE:nucleic acid
TOPOLOGY:linear
CLONE:HUMGS00795
SEQUENCE DESCRIPTION:
GATCCAGGTG ACTCTGAACA TCATTAGAAG CATGCCAGAA CAGACTGGTG AAAAGTAAAC 60
CTTTTCACCT ACAAAATTTC ACCTGCAAAC CTTAAACCTG CAAAATTTTC CTTTAATAAA 120
ATTTGCTTGT TTTAANAACA NNNGAAA 147

SEQ ID NO:722
SEQUENCE LENGTH:140
SEQUENCE TYPE:nucleic acid
TOPOLOGY:linear
CLONE:HUMGS00796
SEQUENCE DESCRIPTION:
GATCCAGAGC CTCCCGGCCC TTCTCCGGTG TCCTGTACCA ACTCTTCTAT TTAAGAGAAC 60
CTCAGATGAT GTACCTGAGC CTCAGGGTTT TGTTTCAGAG GGATATAAAT NATTTAAAAA 120
TTAAATGAAA ACGTTGCAAA 140

SEQ ID NO:723
SEQUENCE LENGTH:150
SEQUENCE TYPE:nucleic acid
TOPOLOGY:linear
CLONE:HUMGS00797
SEQUENCE DESCRIPTION:
GATCGGCCAC TACCTGGGCG AGTTCTCCAT CACCTACAAG CCCGTAAAGC ATGGCCGGCC 60
CGGCATCGGG GCCACCCACT CCTCCCGCTT CATCCCTCTC AAGTAATGGC TCAGCTAATA 120
AAGGCGCACA TGACTCCAAA AAAAAATAAA 150

SEQ ID NO:724
SEQUENCE LENGTH:140
SEQUENCE TYPE:nucleic acid
TOPOLOGY:linear
CLONE:HUMGS00798
SEQUENCE DESCRIPTION:
GATCAGGGTA AGGCAGTCAG GCGGGTGTTC ACCACTGCCT TTCCTTCCTC TGAGCGTGAG 60
AACACTGAAC CCAGCCACTG CCCCTGGGTC CCTGTCCTGG AAATNGTCTA ATAAATCCTT 120
TNCCCTTCTT GAGCTACAAA 140

SEQ ID NO:725
SEQUENCE LENGTH:141
SEQUENCE TYPE:nucleic acid
TOPOLOGY:linear
CLONE:HUMGS00799
SEQUENCE DESCRIPTION:
GATCCGAATC GAGGCCAACG AGGCCCGGGA TGAAGGCCCN GGAGTAGGCG AGCCAGACGA 60
CAAGGTTGAC CTCAGCTTCG GAGCCACCTC TGGATGAACT GCCCCCAGCC CACGNCCNAT 120
TAAAGACCCG GAAGCCTGAA A 141

SEQ ID NO:726
SEQUENCE LENGTH:136
SEQUENCE TYPE:nucleic acid
TOPOLOGY:linear
CLONE:HUMGS00800
SEQUENCE DESCRIPTION:
GATCAGCTTT ACCTATGGTG CTTTGCCTTT AACTAGAGTG TGTGATGGTA GATTATTTCA 60
NATATGTATG TAAAACTNTT TCCTGAACAA TAAGATGTAT GACCCGGAGC AGAAATAAAT 120
NCTTTTCCTA ATTAAA 136

SEQ ID NO:727
SEQUENCE LENGTH:135
SEQUENCE TYPE:nucleic acid
TOPOLOGY:linear
CLONE:HUMGS00801
SEQUENCE DESCRIPTION:
GATCATGTCA TTCAATTCCA GTCACCTCTT CTGCAATCAT GACCTCTTGA TGTCTCCATG 60
GTGACCTCCT TGGGGGTCAC TGACCCTGCT TGGTGGGGTC CCCCTTGTAA CAATAAAATC 120
TATTTAAACT TTAAA 135

SEQ ID NO:728
SEQUENCE LENGTH:135
SEQUENCE TYPE:nucleic acid
TOPOLOGY:linear

CLONE:HUMGS00802
SEQUENCE DESCRIPTION:
GATCTTAAAC ATTGTTTTGT AGTGTATATT ACTTGTCCAT TCCTTTAAGG GGAGCAGCCT 60
GCACTCTTTT GTAGATTACT TTTGGGGGAT ATATTTNNAG AATNATGAAA CGGAATAAAA 120
TTGTAAAAAA CTAAA 135

SEQ ID NO:729
SEQUENCE LENGTH:134
SEQUENCE TYPE:nucleic acid
TOPOLOGY:linear
CLONE:HUMGS00803
SEQUENCE DESCRIPTION:
GATCATGTTA CCATATCAAG CTGAAAATGT CACCACTATC TGGAGATTTC GACGTGTTTT 60
CCTCTCTGAA TCTGTTATGA ACACGTTGGT TGGCTGGNTT CAGTAGGGGG NTATTTNAGG 120
CCTTTCTTTT TAAA 134

SEQ ID NO:730
SEQUENCE LENGTH:134
SEQUENCE TYPE:nucleic acid
TOPOLOGY:linear
CLONE:HUMGS00804
SEQUENCE DESCRIPTION:
GATCTANGTT GCCTACCTTG AATTTTTTTT TAAATATATT TGATGACATA ATTTTTGTGT 60
AGTTTATTTA TCTTGTACAT ATGTATTTTG AAATCTTTTA AACCTGAAAA ATAAATAGTC 120
ATTTAATGTT GAAA 134

SEQ ID NO:731
SEQUENCE LENGTH:134
SEQUENCE TYPE:nucleic acid
TOPOLOGY:linear
CLONE:HUMGS00805
SEQUENCE DESCRIPTION:
GATCTCACTA AAGGATTTCT ATTTGCTGTC AGTTAAAAAT AAAGCCCTAA ATACATTTTT 60
ATTCTTTCTA CTGAGGGCAT TGTCTGTTTT CTTTGTAAAT GCCGTACAAT AAACAAATTA 120
TTTAATAACC TAAA 134

SEQ ID NO:732
SEQUENCE LENGTH:137
SEQUENCE TYPE:nucleic acid
TOPOLOGY:linear
CLONE:HUMGS00806
SEQUENCE DESCRIPTION:
GATCTTGGCA CTCTCCATGT TCTCTACAAG AAGCTGTGGT GATTGGCCCT GTGGTCTATC 60
AGGCGAAAAC CACAGATTCT CCTTCTAGTT AGTATAGCGG ACTTAATAAA AGAGGAAAAA 120
ACTCTTGCTT CAGTAAA 137

SEQ ID NO:733
SEQUENCE LENGTH:134
SEQUENCE TYPE:nucleic acid
TOPOLOGY:linear
CLONE:HUMGS00807
SEQUENCE DESCRIPTION:
GATCCTCGAA CGGAAAGCCA AATCTCGCCA AGTAAGGAAA GGAAAAGGGC AAATACAAGG 60
AAGAAACCAT TGAGAAGATG CAGGAATAAA GTAATCTTAT ATACAAGCTT TGATTAAAAC 120
TTGAAACAAA GAAA 134

SEQ ID NO:734
SEQUENCE LENGTH:132
SEQUENCE TYPE:nucleic acid
TOPOLOGY:linear
CLONE:HUMGS00808
SEQUENCE DESCRIPTION:
GATCAGTGGC TTTGAATGAA ATACAGATGC ATTATCCAGA ACTGAAGTTG CCCTACTTTT 60
AACTTTGAAC TTGGCTAGTT CAAAGATAGA CTCTTCTTTT GTAAAGTAAA TAAATTCTTC 120
AAAATGCTTA AA 132

SEQ ID NO:735
SEQUENCE LENGTH:132
SEQUENCE TYPE:nucleic acid
TOPOLOGY:linear
CLONE:HUMGS00809
SEQUENCE DESCRIPTION:
GATCNNAACC TTTTCAATAA AAGGCAAAAC AAACCAATNT CCNAACATAG CATTACAGCC 60
TTTAAAACCA TTCACTNCTC ATAGTGATTC ACAGAGGACA AGAGATTAAA GTGCTGGATT 120
TTAAATGTCA AA 132

SEQ ID NO:736
SEQUENCE LENGTH:131
SEQUENCE TYPE:nucleic acid
TOPOLOGY:linear
CLONE:HUMGS00810
SEQUENCE DESCRIPTION:
GATCANATGC AACCNCACAA CCTCGGCTGA GTCTTGAGAC TGAAAGATTA AGCCATAATG 60
TAAACTGCCT CAAATTGGAC TTTGGGCATA AAAGAACTTT TTTATGCTTA CCATCTNTTT 120
TTTTTCTTAA N 131

SEQ ID NO:737
SEQUENCE LENGTH:383
SEQUENCE TYPE:nucleic acid
TOPOLOGY:linear

CLONE:HUMGS00811
SEQUENCE DESCRIPTION:
GATCATACAC CTGCTCACAG GCGAGAACCC TCTGCAGGTC CTGGTGAACG CCATCATCAA 60
CAGTGGTCCC CGGGAGGACT CCACACGCAT TGGGCGCGCC GGGACTGTGA GACGACAGGC 120
TGTGGATGTG TCCCCCCTGC GCCGTGTGAA CCAGGNCANN TGGCTGCTGT GCACAGGCGC 180
TCGTNAGGCT GCCTTCCGGA ACATTAAGAC CATTGCTGAG TGCCTGGCAG ATGAGCTCAT 240
CAATGCTGCC AAGGGCTCCT CGAACTCCTA TGCCATTAAG AAGAAGGACG AGCTGGAGCG 300
TGTGGCCAAG TCCAACCGCT GATTTTCCCA GCTGNTGCCC AATAAACCTN GTCTGCCCCT 360
TTTGGGGGAA GCCCCGAAGC AAA 383

SEQ ID NO:738
SEQUENCE LENGTH:128
SEQUENCE TYPE:nucleic acid
TOPOLOGY:linear
CLONE:HUMGS00813
SEQUENCE DESCRIPTION:
GATCCAGCTG CCAGAGGATG AGTGACCAGT TGCTAAGTGG GGCTCAAGAA GCACCGCCTT 60
CCCCACCCCC TGCCTGCCAT TCTAACCTCT TCTCAGAGCA CCTAATTAAA GGGGCTGAAA 120
GTCTGAAA 128

SEQ ID NO:739
SEQUENCE LENGTH:128
SEQUENCE TYPE:nucleic acid
TOPOLOGY:linear
CLONE:HUMGS00814
SEQUENCE DESCRIPTION:
GATCTGGATT ACNATGTAAA TTCACAGCAG TAAGATAATA TAAATTTTGT TGAATGTATT 60
AACATCATAT GGTCTGAAAA TGTGGGTTTT NATTTGGCAC ATTTAAATAA AATGTTTCTA 120
ACTAGAAA 128

SEQ ID NO:740
SEQUENCE LENGTH:128
SEQUENCE TYPE:nucleic acid
TOPOLOGY:linear
CLONE:HUMGS00815
SEQUENCE DESCRIPTION:
GATCAAGAAT GAAAAAGACA TCATATGAAN NGGGAGAAAC TATTTGCACA CCATCTATCT 60
CATGGTTTGG TTAATATTCA AACTATATNA GCAGAATGTG TAAGGATATC CTACAACTCA 120
ATAGCAAA 128

SEQ ID NO:741
SEQUENCE LENGTH:138
SEQUENCE TYPE:nucleic acid
TOPOLOGY:linear
CLONE:HUMGS00816

SEQUENCE DESCRIPTION:
GATCCAAGGG GAAACTGCAG GTCAAGGGCT GATAACGGCC ATGCAGGATG CTTGATGCTG 60
CGTCCCCCGC TGCTTGCCGC CCCCCACCCC GCCATTTTGT ATAATAAAGC TCCCTGTGTA 120
TTCTCAAAAA AAANCAAA 138

SEQ ID NO:742
SEQUENCE LENGTH:127
SEQUENCE TYPE:nucleic acid
TOPOLOGY:linear
CLONE:HUMGS00817
SEQUENCE DESCRIPTION:
GATCCCTGAG ACTGAGGGGT TTACGGGCTG TGAATGGACC TTCAGCCCTN CCCACCCTCC 60
CTCCCCACTG CTGCTGAGTC TGTCTGATGT TTTGGTTGTG TGAATAAATA TAATTCCCCT 120
CTGGAAA 127

SEQ ID NO:743
SEQUENCE LENGTH:124
SEQUENCE TYPE:nucleic acid
TOPOLOGY:linear
CLONE:HUMGS00818
SEQUENCE DESCRIPTION:
GATCGGGTAG CTCAAAAGAA GGCAAGCTTC CTCAGAGCTC AGGAGCGGGC TGCTGAGAGC 60
TAAACCCAGC AATTTTCTAT GATTTTTTCA GATATAGATA ATAAACTTAT GAACAGCAAC 120
TAAA 124

SEQ ID NO:744
SEQUENCE LENGTH:124
SEQUENCE TYPE:nucleic acid
TOPOLOGY:linear
CLONE:HUMGS00819
SEQUENCE DESCRIPTION:
GATCAGAAAT TCTCTTGCTT GAGAGATTTT TTTTTGTCCT CTGTTGACTA CATAGTTTCA 60
AATCTCTCTN TATTTCATGA TGATATATAA ATNGCTTTTA ATTATATNAA ATNTTAATTN 120
NCCN 124

SEQ ID NO:745
SEQUENCE LENGTH:125
SEQUENCE TYPE:nucleic acid
TOPOLOGY:linear
CLONE:HUMGS00820
SEQUENCE DESCRIPTION:
GATCTCATTA CCCTGTTTCC GAATTCTGCC GTGTGTATCC CCAACCCTTG ACCCAATGAC 60
ACCAAACACA GTGTTTTTNA GCTCGGTATT ATATATNTTT TTCTCATTAA AGGTTTAAAA 120
CCAAA 125

SEQ ID NO:746
SEQUENCE LENGTH:123
SEQUENCE TYPE:nucleic acid
TOPOLOGY:linear
CLONE:HUMGS00821
SEQUENCE DESCRIPTION:
GATCCGTGGC TTAAGACAGG AGATTATCTC TNTACTCCAG TGGCATCTCC TTAGCCAAGA 60
TGTGAAATTA AAATCATAGT TCGCCTCATT TAAAAATNCT AATAAAGCAC TCAAACTTTG 120
AAA 123

SEQ ID NO:747
SEQUENCE LENGTH:122
SEQUENCE TYPE:nucleic acid
TOPOLOGY:linear
CLONE:HUMGS00822
SEQUENCE DESCRIPTION:
GATCCAAATC TGGTTCAAAC ATTCAAAACT TCAAAGATAA TTCATCTTTC AGCTAATGCT 60
TGTGGTTCTG TTGTTCCCTT GAAAAAAAAT AAAAACAGTT GCCTTCNGGG AAAANTTNNA 120
AA 122

SEQ ID NO:748
SEQUENCE LENGTH:122
SEQUENCE TYPE:nucleic acid
TOPOLOGY:linear
CLONE:HUMGS00823
SEQUENCE DESCRIPTION:
GATCTTGTGT TTTAGGNTGG GCATTTTCAC TCTTCTGCCT TAAATCCCTA ACCCCATGGA 60
GCTGACATTC TAGTGCGGCT GAGGGGAGGG GAAACATTGT AAAATAAATC ATAAAAATTA 120
AA 122

SEQ ID NO:749
SEQUENCE LENGTH:122
SEQUENCE TYPE:nucleic acid
TOPOLOGY:linear
CLONE:HUMGS00824
SEQUENCE DESCRIPTION:
GATCTCTGGG CTGGGGACTG AATTCCTGAT GTCTGAGTCC TCAAGGTGAC TGGGGACTTG 60
GAACCCCTAG GACCTGAACA ANCAAGACTT TAAATAAATT TTAAAATGCA AAAACTCGGA 120
AA 122

SEQ ID NO:750
SEQUENCE LENGTH:122
SEQUENCE TYPE:nucleic acid
TOPOLOGY:linear
CLONE:HUMGS00825

SEQUENCE DESCRIPTION:
GATCNTAGAA GGGCTTCCCA ACCNNATTTG CAACATCCAA ATTGTCTTCA ATTNAAGGAA 60
GGCCTTATCA GTTCATAGAT GANCTTCATT GTAAAAATAA ATGTACTTTG CACCACTTCA 120
AA 122

SEQ ID NO:751
SEQUENCE LENGTH:273
SEQUENCE TYPE:nucleic acid
TOPOLOGY:linear
CLONE:HUMGS00826
SEQUENCE DESCRIPTION:
GATCCACTTC TGTNATTANG TAAATGGATG TNTCGTGATG CGTCTACAGT TATTTATTGT 60
TACATCCTTT TCCAGACACT GTAGATGCTA TAATAAAAAT AGCTGTTTGG TAACCATAGT 120
TTCACTTGTN CAAAGCTGTG TAATCGTGGG GGTACTATCT CAACTGCTTT CGTATTCATT 180
GTATTAAAAG AATCTGTTTA AACAACCTTT ATCTTCTCTN CGGGTTTAAG AAACGTTTAT 240
TGTAACAGTA ATTAAATGCT GCCTTAATTG AAA 273

SEQ ID NO:752
SEQUENCE LENGTH:144
SEQUENCE TYPE:nucleic acid
TOPOLOGY:linear
CLONE:HUMGS00827
SEQUENCE DESCRIPTION:
GATCAAAAAG AAACTTTGTT TTTCCGCAAT TGAAGGTTGT ATGTAAATCT GCTTTGTGGT 60
GACCTGATGT AAACAGTGTC TTCTTAAAAT CAAATGTAAA TCAATTACAG ATTAAAAAAA 120
AAAGCCTGTA TTTAAAGAAC GAAA 144

SEQ ID NO:753
SEQUENCE LENGTH:132
SEQUENCE TYPE:nucleic acid
TOPOLOGY:linear
CLONE:HUMGS00828
SEQUENCE DESCRIPTION:
GATCAGAGGA AAAATCCAGT GTGACAGAGT GCAAGTNAGA AGACCTGGCT TTTNATCCCA 60
GCTTTGAAAC TTGGAACTTT TTGATTGACA AATTAATAAA CCTCTCTATG CCTCAGGCTC 120
CTCATCTGTA AA 132

SEQ ID NO:754
SEQUENCE LENGTH:117
SEQUENCE TYPE:nucleic acid
TOPOLOGY:linear
CLONE:HUMGS00829
SEQUENCE DESCRIPTION:
GATCTAGGCT TGAGCTTGGT TGGGATTGCT NTTTTCTTCT TCTTCTTTAT AAACGATTCT 60
NTGTAACTNT TTGTATTGAC AGTTTCAAAC TTACAGTAAA ATTGCAACAC GAGTAAA 117

SEQ ID NO:755
SEQUENCE LENGTH:117
SEQUENCE TYPE:nucleic acid
TOPOLOGY:linear
CLONE:HUMGS00830
SEQUENCE DESCRIPTION:
GATCAGTACA TAGCATTTGG CTCNTGAACN NAATTNTAAA CTTTCAGGTA TTTTTGTACA 60
AATAAGGGAC TGATGTTCTG TTTCTTGTAA TTAGAAATAA ACATTAATAC AGTGAAA 117

SEQ ID NO:756
SEQUENCE LENGTH:119
SEQUENCE TYPE:nucleic acid
TOPOLOGY:linear
CLONE:HUMGS00831
SEQUENCE DESCRIPTION:
GATCTATCAT TACTGCAAAA ACCTGCTCTG TTGTGCTGGC TGGNAGGCCC TGTGGCTGCT 60
GGCTGAGGGT TCTGCTGTCC TGTGGCACCC CATTAAAGTG CAGTTCCCTC CGGGCCAAA 119

SEQ ID NO:757
SEQUENCE LENGTH:167
SEQUENCE TYPE:nucleic acid
TOPOLOGY:linear
CLONE:HUMGS00832
SEQUENCE DESCRIPTION:
GATCTGAAAT TAAATACTCA ACAGACTCCT CCTTTTTTAG CTGTATTTTT CAGGTACTGT 60
GTGGTGACCG CCCCACTGGT GTCTATTACA GGCCACTTTG GTAGTTGTGT ATCTGNTCAT 120
GTATGTGATT TGACAAACCA GTTTTTTAAA ATAAATGGCT TTTTAAA 167

SEQ ID NO:758
SEQUENCE LENGTH:379
SEQUENCE TYPE:nucleic acid
TOPOLOGY:linear
CLONE:HUMGS00833
SEQUENCE DESCRIPTION:
GATCCAACTA GAAATGAATG GAAGATGATG GGAAATATGA CTTCACCAAG GAGCAATGCT 60
GGGATTGCAA CTGTAGGGAA CACCATTTAT GCAGTGGGAG GATTCGATGG CAATGAATTT 120
CTGAATACGG TGGAAGTCTA TAACCTTGAG TCAAATGAAT GGAGCCCCTA TACAAAGATT 180
TTCCAGTTTT ANCAAATTTA AGACCCTCTC AAACTANCAG GCTTAGTGAT GTAATTATGG 240
TTAGCAGAGG TACACTTGTG AATAAAGNGG GTGGGTGGGT ATAGATGTTG CTANCAGCAC 300
CACAANGCTT TTCCATATTN GCNTCCTNTT AACCATGCNT GTCCATAAAC CCNGGGANGN 360
NNANTTGNGG GGTTNAANN 379

SEQ ID NO:759
SEQUENCE LENGTH:121

SEQUENCE TYPE:nucleic acid
TOPOLOGY:linear
CLONE:HUMGS00834
SEQUENCE DESCRIPTION:
GATCTACTAC TNGGCCTTCA GTGCGAAGAG CCACATCCAG GCCTGAGGGC GGCACCCCAG 60
CCCTGCCCTT GCTTCCTTCA ATAAACATCA CAGGACCTGG GACTGCACAG GNCCTGGGAA 120
A 121

SEQ ID NO:760
SEQUENCE LENGTH:303
SEQUENCE TYPE:nucleic acid
TOPOLOGY:linear
CLONE:HUMGS00835
SEQUENCE DESCRIPTION:
GATCGAAGCA ATTGANGTAT CATGGATTGG ATTGTTACTG ATTTCAGTAA AGTATGTTTT 60
GCCAATTAGA TACATATATA CAAGATAAAG GAATAGGATG GTAATATATT TGTNTGAAAT 120
TAAATTACTG TTTTNATTAA AAAATACTGC TTCATTGGGC TGATTTTGTA AAATGTAATG 180
AGTAAAATGA ATTACTGTAT TTNCCCTTTT ATGTCCACAG AATGAGAGTC ATATGTNGTN 240
ATATNCTAAA TNTNCATTAA ATATTCATGT CACCTTGAGT TGTCATGATA AGTATGTTTT 300
AAA 303

SEQ ID NO:761
SEQUENCE LENGTH:373
SEQUENCE TYPE:nucleic acid
TOPOLOGY:linear
CLONE:HUMGS00836
SEQUENCE DESCRIPTION:
GATCTACTAT GTNATGTCAG GGCCTGCGTG GCAACTCATG CAGCAATTCC AGAACCCTGA 60
CTTCCCACNC GAAGTAGAGG AACAGGATGC CAGCACCCTG CCTGTGTCTT GTGCCTGGAA 120
GAGTGGGATG AAACGCCACA GAGCAGCCTG TGCTTCGGCT AGTATTAATG TGTAGATAGC 180
ACTCTGGTAG CTGTTAACTG CAAGTTTAGC TTGAATTAAG GGATTTGGGG GGACCATGTA 240
ACTTAATTAC TGCTAGNNNN GGAATGTCTT TGTAAGAGTA GGGTCGCCAT GATGCAGCCA 300
TATGGAAGNC TAGGGTATGG GTCACACTTT ATCTGTGTTC CTATGGAAAC TNATTTNGNA 360
TATTTNGNTT TGN 373

SEQ ID NO:762
SEQUENCE LENGTH:406
SEQUENCE TYPE:nucleic acid
TOPOLOGY:linear
CLONE:HUMGS00837
SEQUENCE DESCRIPTION:
GATCTAGGGA GATGTGGAAG AAACTGTGAC TACTATAGAA ATTGATGAAG AAACATATGA 60
AGAGATATAT AAATCAACGN AACGGAATAT TCCAATGCTC TTTGTCCGGG GAGATGGCGT 120
TGTCCTGGTT GCCCCTCCAC TGAGAGTTGG CTGAAACAAA GAATTTGTCC TGTATGGAAA 180
NCGNNNNNNN GGTGTACAGT GGCCTCTCTA AAAGTACAAA ACATTCATAA GAGAAACCCG 240

CATACATTTT GATATTAAGA AATAATTCCG GGGATTCTTC CACTCCTGAA ATGAGTTGAT 300
TTGCAGATAA CTCACAACTT CTTAAGCTAA ATGGTATTTT CATTTTTCTC AAGCTCTCCN 360
ATAANTATGG CCACCNNNGG NNANNGNGTG GGGAAAAAAA NAATTN 406

SEQ ID NO:763
SEQUENCE LENGTH:120
SEQUENCE TYPE:nucleic acid
TOPOLOGY:linear
CLONE:HUMGS00839
SEQUENCE DESCRIPTION:
GATCCCCACG CCACAGCCCT TTTGTCTCTG CAAACTGCCT TCTTCGGAAA GAAGAAGGTG 60
GGAGGNTGTN AATTGTTAGT TTCTGAGTTT TACCAAATAA AGTAGAATAT AAGACGNAAA 120

SEQ ID NO:764
SEQUENCE LENGTH:115
SEQUENCE TYPE:nucleic acid
TOPOLOGY:linear
CLONE:HUMGS00840
SEQUENCE DESCRIPTION:
GATCTTGATG GTGTTTCTTT CCCCAAAAAT TGACTTAGAT ATTAAAATTT GGTGCTTATA 60
AGAGAGAGTT AAAAAAAAAT AGGATTGCTT CAATTAAAAT TACAAAAGAG NCAAA 115

SEQ ID NO:765
SEQUENCE LENGTH:113
SEQUENCE TYPE:nucleic acid
TOPOLOGY:linear
CLONE:HUMGS00841
SEQUENCE DESCRIPTION:
GATCCAGTGA CAGCAGGTGT CATGGGTCAA GCATAAATCA TATATAGCAT TTTCAGGCAT 60
GTTCCTGGTA GTTCTTTTGA GTCTGACATT CTAATAAAAT AATTTGTAGG AAA 113

SEQ ID NO:766
SEQUENCE LENGTH:112
SEQUENCE TYPE:nucleic acid
TOPOLOGY:linear
CLONE:HUMGS00842
SEQUENCE DESCRIPTION:
GATCTTTGTG AGGATTAGGA ATTAGGTTAA AAGAAATTAA GAACCATCTT CAAGCAAAAN 60
TTAAACTTTA TTTCTNCTTA ANCAATAAAT ACACCTGANT TAGTTTTCCA AA 112

SEQ ID NO:767
SEQUENCE LENGTH:112
SEQUENCE TYPE:nucleic acid
TOPOLOGY:linear
CLONE:HUMGS00843

SEQUENCE DESCRIPTION:
GATCAAAGAC CCAAAGGAAT GCAACANTTT ATCTNTTATC TACCTATNAC CTGCGAGCTG 60
CCCACCACCC CCANGTTGTN GCGCCTTTCC AGACAGAACC AGTGTACATC TN 112

SEQ ID NO:768
SEQUENCE LENGTH:130
SEQUENCE TYPE:nucleic acid
TOPOLOGY:linear
CLONE:HUMGS00844
SEQUENCE DESCRIPTION:
GATCCAAAAC TTAAATACAT CTATGAATTT CCTGGGGCTA TTGTGAGTAC TGTGTATGTA 60
TTTAGCAAAT ATTTAAGACC TAGTAAGTGC TCAATAAATT GTAGCTGTTA TTGCTGTTGT 120
NGTTTGTAAA 130

SEQ ID NO:769
SEQUENCE LENGTH:111
SEQUENCE TYPE:nucleic acid
TOPOLOGY:linear
CLONE:HUMGS00845
SEQUENCE DESCRIPTION:
GATCGTGCCA CTGCTCTCCA GCCTGCATGA CGGGAGTNAG ACACCATCTC AAAAAATACA 60
TATAATAATA TAAATAAAAA TATCTTTTTN GAAAATAATT TAATATNNCN N 111

SEQ ID NO:770
SEQUENCE LENGTH:110
SEQUENCE TYPE:nucleic acid
TOPOLOGY:linear
CLONE:HUMGS00846
SEQUENCE DESCRIPTION:
GATCNAGTAN TGNAGGGGCT GTTAGGAGCT TCCTGCAAAT CCCTGAGAGG GCAGAAGATA 60
GCTTCTGTTA ATTCATTATT CTTCCAATAA ATGTTGATTG AGTACCTAAA 110

SEQ ID NO:771
SEQUENCE LENGTH:109
SEQUENCE TYPE:nucleic acid
TOPOLOGY:linear
CLONE:HUMGS00847
SEQUENCE DESCRIPTION:
GATCACCGNC CAGTAATGGG CTCAGAGCAG GTCTTCATCA TGCCTTGTCC TTTTTTAACT 60
GAGAAAGGAG ATTTTTTGAA AAGAGTACAA TTAAAAGGAC ATTGTCAAA 109

SEQ ID NO:772
SEQUENCE LENGTH:109
SEQUENCE TYPE:nucleic acid
TOPOLOGY:linear

CLONE:HUMGS00848
SEQUENCE DESCRIPTION:
GATCTTCTTC AATATGTGAA TTTGGGCTCA CAGAATCAAA GCCTATGCTT GGTTTAATGC 60
TTGCAATCTG AGCTCTTGAA CAAATAAAAT TAACTATTGT AGTGTGAAA 109

SEQ ID NO:773
SEQUENCE LENGTH:109
SEQUENCE TYPE:nucleic acid
TOPOLOGY:linear
CLONE:HUMGS00849
SEQUENCE DESCRIPTION:
GATCAACAAG GTTTGCCACT GCTTGTATTA CCAGGGACTG GTTACAACCA TTATTTCTNT 60
TCATTTGCTT GGCTTATCTC ATATTAAAGT GAGTTTGGAG TTCTCCAAA 109

SEQ ID NO:774
SEQUENCE LENGTH:110
SEQUENCE TYPE:nucleic acid
TOPOLOGY:linear
CLONE:HUMGS00850
SEQUENCE DESCRIPTION:
GATCCAGTTC TAAACTTTGG GATATTTTTT TTCAATTTTG AAGAGAAAAT GGTGAAGCCA 60
TANGAAAAGT TACCCGAGGG AAAATAAATA CAGTGATATT CTTACGCAAA 110

SEQ ID NO:775
SEQUENCE LENGTH:108
SEQUENCE TYPE:nucleic acid
TOPOLOGY:linear
CLONE:HUMGS00851
SEQUENCE DESCRIPTION:
GATCTGTGTG CTCTTATCAC CAATCAGTTC AGACCTGGTT GATTTTGTAC TTTGGAACTG 60
TACCTTGGAT GGTTTTGTTT ATTAAAAGAG AAACCTGAAG TACTCAAA 108

SEQ ID NO:776
SEQUENCE LENGTH:108
SEQUENCE TYPE:nucleic acid
TOPOLOGY:linear
CLONE:HUMGS00852
SEQUENCE DESCRIPTION:
GATCACCATC TTTAAATTTA CTTCAAAATA AAAGCATGTA AGTNACTGTT TTTCAAGAAG 60
AAATGTGTTT CATAAAAGGA TATTTATATC TCTNTNGCTT TGACTNNN 108

SEQ ID NO:777
SEQUENCE LENGTH:106
SEQUENCE TYPE:nucleic acid
TOPOLOGY:linear

CLONE:HUMGS00853
SEQUENCE DESCRIPTION:
GATCCCCCTC GGAAGAGGGA CTCCAATGGG CATGTCCCCT CCGGAAATNC GGCCTCCTCC 60
CCCTGGAATG CNAGGNCCCC CTCCCCCGGN AATNCGCCCA CCAAGN 106

SEQ ID NO:778
SEQUENCE LENGTH:130
SEQUENCE TYPE:nucleic acid
TOPOLOGY:linear
CLONE:HUMGS00854
SEQUENCE DESCRIPTION:
GATCCCATCA TGAATTCATT GGAATTTGTG TTGCATGTAA GGCAATCTTT TCCNTGTTGT 60
AAATCTTCCG TTTTTTAATG TACATATATT TTGAAAAATA TGAATAAACA TGAAATTTTA 120
AAAGCTGAAA 130

SEQ ID NO:779
SEQUENCE LENGTH:104
SEQUENCE TYPE:nucleic acid
TOPOLOGY:linear
CLONE:HUMGS00855
SEQUENCE DESCRIPTION:
GATCATGGTG GGTCAGCTGT ACTGATTGTN ATCCTGACTT TGGCATTGGC AGCTCTTATA 60
TNCCGACGAA TATATCTGGC AAACNNATAC ATATNTAACT TTAN 104

SEQ ID NO:780
SEQUENCE LENGTH:107
SEQUENCE TYPE:nucleic acid
TOPOLOGY:linear
CLONE:HUMGS00856
SEQUENCE DESCRIPTION:
GATCTTTTTA CAGTATCCAT TTATTATGTA ATNCTTNTNA GAAAAGAATC TTATAGTACA 60
TNTTANTATA TGCAACCAAT TAAAATGTAT AAATTAGTGT AAGCAAA 107

SEQ ID NO:781
SEQUENCE LENGTH:109
SEQUENCE TYPE:nucleic acid
TOPOLOGY:linear
CLONE:HUMGS00857
SEQUENCE DESCRIPTION:
GATCAAAATG AAAGAAAATC ACAGAAATTA TCCTATGTGT ACTCCTCATC CCTCCTGCTG 60
TATATNTTCT NATTTTTTGC GTAATAAATN ATGTTAATTA CCAAATAAA 109

SEQ ID NO:782
SEQUENCE LENGTH:125
SEQUENCE TYPE:nucleic acid

TOPOLOGY:linear
CLONE:HUMGS00858
SEQUENCE DESCRIPTION:
GATCTCCAAT GTTTTGGGGA TGCTTTGAGT CTCAAAAAAA ATTGATAATC AGAAAAGTAA 60
TTTTTGTTTG TTTGTTTAAT GTATCCCTGT TCTGTTTTTA ATTAAACTCC AAGTCTCATT 120
TTAAA 125

SEQ ID NO:783
SEQUENCE LENGTH:103
SEQUENCE TYPE:nucleic acid
TOPOLOGY:linear
CLONE:HUMGS00859
SEQUENCE DESCRIPTION:
GATCGAGGTG ACAAATANTC AGTCCNTANG TCCCCACAAT GACCTCACCA NNATGGCTTT 60
GGGGAGCTCT TCACCCTAAA GATTCGGTCT GGTTTGCTAA TGN 103

SEQ ID NO:784
SEQUENCE LENGTH:102
SEQUENCE TYPE:nucleic acid
TOPOLOGY:linear
CLONE:HUMGS00860
SEQUENCE DESCRIPTION:
GATCTGGAGG CAAGATGCCA GGCCCCACAG GTGTTCTCAG GGCAGTTCTT GGTGTCTGCT 60
TCTCAGATAC CAAGGACTGG AATTAAAACC TTTCCTGGGA AA 102

SEQ ID NO:785
SEQUENCE LENGTH:99
SEQUENCE TYPE:nucleic acid
TOPOLOGY:linear
CLONE:HUMGS00861
SEQUENCE DESCRIPTION:
GATCACAGCC GAAGAGTGAA AGGTGCTGCA ATGAATGTTA GCTGTGGCCA CTGTGGATTT 60
TTCGCAAGAA CATTAATAAA CTAAAAACTT CATGTGAAA 99

SEQ ID NO:786
SEQUENCE LENGTH:94
SEQUENCE TYPE:nucleic acid
TOPOLOGY:linear
CLONE:HUMGS00862
SEQUENCE DESCRIPTION:
GATCAAGTTA TTTTNAATTT GGTTTTCACA TTGGAAACAA GTCAGTCATT CAGATATGAT 60
TCAAATGTCT ATAAACCGAA CTGATGTAAG TAAA 94

SEQ ID NO:787
SEQUENCE LENGTH:102

SEQUENCE TYPE:nucleic acid
TOPOLOGY:linear
CLONE:HUMGS00863
SEQUENCE DESCRIPTION:
GATCTCTTCT TCTCCCTGTG GCCCCTGCGC TGTTGCCCCG TCCCCGTCAC CCCGCCCGNN 60
ACTGAAATNT ATAATCTGAC TTCCTGTACA GAAACCTGCA AA 102

SEQ ID NO:788
SEQUENCE LENGTH:93
SEQUENCE TYPE:nucleic acid
TOPOLOGY:linear
CLONE:HUMGS00864
SEQUENCE DESCRIPTION:
GATCTTGTTA GCAATGCTGT TTTTNCTGTT AGTCGGGTTA GAGTTGGCTC TACGCGAGGT 60
TTGTTAATAA AAGTTTGTTA AAAGTTTAAT AAA 93

SEQ ID NO:789
SEQUENCE LENGTH:100
SEQUENCE TYPE:nucleic acid
TOPOLOGY:linear
CLONE:HUMGS00865
SEQUENCE DESCRIPTION:
GATCTTCAAG TGAACATCTC TTGCCATCAC CTAGCTGCCT GCACCTGCCC TTCAGGGAGA 60
TGGGGGTCAT TAAAGGAAAC TGAACATTGA ACCCTTTAAA 100

SEQ ID NO:790
SEQUENCE LENGTH:92
SEQUENCE TYPE:nucleic acid
TOPOLOGY:linear
CLONE:HUMGS00866
SEQUENCE DESCRIPTION:
GATCAGAATT TAAAATGTGT GATTCTTTTT CTTTCTGTAA GTATGTATTG CTATGATAAA 60
TAAAAAATGG CAGGACCATT NTTTTTATNA AA 92

SEQ ID NO:791
SEQUENCE LENGTH:93
SEQUENCE TYPE:nucleic acid
TOPOLOGY:linear
CLONE:HUMGS00867
SEQUENCE DESCRIPTION:
GATCTCCAAC CAGGCCAGAG AAGATTCTCA CAGAAGGTTT TGAACTCTAA GAAATAAATT 60
GGTTTGGTAA TAAATGGCTT CTGGTCAGAT AAA 93

SEQ ID NO:792
SEQUENCE LENGTH:114

SEQUENCE TYPE:nucleic acid
TOPOLOGY:linear
CLONE:HUMGS00868
SEQUENCE DESCRIPTION:
GATCTTGTAT CTTTGTATAA CGGATGTNAT TTGTACGAAG GGCAGTTCGT AAACAGCACT 60
TGTNCTTTTA ATAAAAGAAT GTTTTGCAAA AAAAAAAAAA AAANCCCNAG GAAA 114

SEQ ID NO:793
SEQUENCE LENGTH:90
SEQUENCE TYPE:nucleic acid
TOPOLOGY:linear
CLONE:HUMGS00869
SEQUENCE DESCRIPTION:
GATCTGTTTT GTACTTTTTA TACTGTTGGA TACTTATAAT CAAAACTTTT ACTAGGGTAT 60
TGAATAAATC TAGTCTTACT AGAAAATAAA 90

SEQ ID NO:794
SEQUENCE LENGTH:88
SEQUENCE TYPE:nucleic acid
TOPOLOGY:linear
CLONE:HUMGS00870
SEQUENCE DESCRIPTION:
GATCCATTTC ATAAAGTATG ATTTGCCCAA ACCTGTACCA TTTCCGTATT TCTCCTGTAG 60
AAGTAAGAAA TAAATTTCCT TAAATAAA 88

SEQ ID NO:795
SEQUENCE LENGTH:89
SEQUENCE TYPE:nucleic acid
TOPOLOGY:linear
CLONE:HUMGS00871
SEQUENCE DESCRIPTION:
GATCTGGCGC TTGGGGGTAA GTGGNATGAT TTGCTAATAT TGAGNATCTG TTGTATCAAA 60
CATAATAAAC TTTTTTTTGA GATGTGAAA 89

SEQ ID NO:796
SEQUENCE LENGTH:89
SEQUENCE TYPE:nucleic acid
TOPOLOGY:linear
CLONE:HUMGS00872
SEQUENCE DESCRIPTION:
GATCAGGGTG TCTCCTTGTC CTTCTNAGAT GTGGAGAAGA GGCTGCTGGC TACCCTAAAA 60
NTTGAAATAA AAGATTTTTG CCTTTGAAA 89

SEQ ID NO:797
SEQUENCE LENGTH:86

SEQUENCE TYPE:nucleic acid
TOPOLOGY:linear
CLONE:HUMGS00873
SEQUENCE DESCRIPTION:
GATCCATTGA GCCCAGCAGT CCAACCTGGG CAAAATAAGT GAGAGACCCT GTATCTGAAA 60
GTAATAATAA AAATAAAAAA TATAAA 86

SEQ ID NO:798
SEQUENCE LENGTH:86
SEQUENCE TYPE:nucleic acid
TOPOLOGY:linear
CLONE:HUMGS00874
SEQUENCE DESCRIPTION:
GATCCTTTTT GATAATCTCA TTCCTAGAAA TTTAACCTTA ATGAAATCCC TAATAAAACT 60
CAGTGCTGTG TTATTTGTGC CTCAAA 86

SEQ ID NO:799
SEQUENCE LENGTH:87
SEQUENCE TYPE:nucleic acid
TOPOLOGY:linear
CLONE:HUMGS00875
SEQUENCE DESCRIPTION:
GATCGAACAT TTCACCTCTC ATATTAAGTC TGGCAATGAT GACTATATGT ATTCCTGCCT 60
AAATAAATCA TCTATTAATC ATTAAAA 87

SEQ ID NO:800
SEQUENCE LENGTH:86
SEQUENCE TYPE:nucleic acid
TOPOLOGY:linear
CLONE:HUMGS00876
SEQUENCE DESCRIPTION:
GATCTCCGAG TCAGGACGGT CGGCCAGACC CACGGGGTAA CGGGTCTAAT CGTGTAGGAA 60
TAAAGCTGTA TTCCAGTGCT TCCAAA 86

SEQ ID NO:801
SEQUENCE LENGTH:84
SEQUENCE TYPE:nucleic acid
TOPOLOGY:linear
CLONE:HUMGS00877
SEQUENCE DESCRIPTION:
GATCCCCGCG ATACTTCAAC GCCTTCTGAC TTCCAGGTGA TGACTGGGCC CCCAATAAAT 60
CCCGTCTTTG GGTCTCTCTG CAAA 84

SEQ ID NO:802
SEQUENCE LENGTH:84

SEQUENCE TYPE:nucleic acid
TOPOLOGY:linear
CLONE:HUMGS00878
SEQUENCE DESCRIPTION:
GATCCCTCAA AACCTCACTA ACTGGAAGGA TGATTTTGTC TCAGTTTGTA CTCCTAAATA 60
AAAAGTAAAC ATGACACCTC TAAA 84

SEQ ID NO:803
SEQUENCE LENGTH:88
SEQUENCE TYPE:nucleic acid
TOPOLOGY:linear
CLONE:HUMGS00879
SEQUENCE DESCRIPTION:
GATCTGTGAA GAAATGAAAT AAAATGGTAT TTAGTAAGAA ATCTCTATTT TAAGAAAAAA 60
AGTAAAACCT GTTATAAACA CATGCAAA 88

SEQ ID NO:804
SEQUENCE LENGTH:82
SEQUENCE TYPE:nucleic acid
TOPOLOGY:linear
CLONE:HUMGS00880
SEQUENCE DESCRIPTION:
GATCGTGCCA TTGTGATATG AATATGCCTT ATATGCTGAT ATGAATATGC CTTAAAATAA 60
AGTGTTCCCC ACCCCTGCCA AA 82

SEQ ID NO:805
SEQUENCE LENGTH:81
SEQUENCE TYPE:nucleic acid
TOPOLOGY:linear
CLONE:HUMGS00882
SEQUENCE DESCRIPTION:
GATCTTAAGT CATACATTTN AATTGTNTAG AGGTTGTTCA ACTGAAGGAA TAAATGTCTA 60
TNAANCTAAA ACAAATGGAA A 81

SEQ ID NO:806
SEQUENCE LENGTH:78
SEQUENCE TYPE:nucleic acid
TOPOLOGY:linear
CLONE:HUMGS00883
SEQUENCE DESCRIPTION:
GATCAAGTTT GTACATAACA CTAGTGGCAT TTCTTATCAA AAGGATTGGA TAATAAAAAT 60
AAGTTTCTAC TGGGTAAA 78

SEQ ID NO:807
SEQUENCE LENGTH:78

SEQUENCE TYPE:nucleic acid
TOPOLOGY:linear
CLONE:HUMGS00884
SEQUENCE DESCRIPTION:
GATCCCCCCA GCAAGGATAN CATTCAAAGG AGCTCACATT TATGGAATGG ATGAATCAAT 60
AAATTAATTC ACTTTAAA 78

SEQ ID NO:808
SEQUENCE LENGTH:77
SEQUENCE TYPE:nucleic acid
TOPOLOGY:linear
CLONE:HUMGS00885
SEQUENCE DESCRIPTION:
GATCCCATTT CTGATGGATG TGTCACACCT TTTCTGTCAA AATAAAATGT CTTGGAGGTT 60
ATGACTCCTT GGTGAAA 77

SEQ ID NO:809
SEQUENCE LENGTH:77
SEQUENCE TYPE:nucleic acid
TOPOLOGY:linear
CLONE:HUMGS00886
SEQUENCE DESCRIPTION:
GATCTGTGTT AATCTGAGTA ACTTATTGCC TAGCCTATAA ATAAATTCCA AAATATCCAA 60
TTCATTTCTT CTTGAAA 77

SEQ ID NO:810
SEQUENCE LENGTH:75
SEQUENCE TYPE:nucleic acid
TOPOLOGY:linear
CLONE:HUMGS00887
SEQUENCE DESCRIPTION:
GATCATCCTT CCTGGCAAAT AAATTCCCGT TTCTATCCAA AAGAGCAATA AAAAGTTTTC 60
AGTGAAATGT GCAAA 75

SEQ ID NO:811
SEQUENCE LENGTH:76
SEQUENCE TYPE:nucleic acid
TOPOLOGY:linear
CLONE:HUMGS00889
SEQUENCE DESCRIPTION:
GATCTGAAAG CCTGAGTGTG TGTACGTGCG CGCGTGCGTG AAGGCCCTGC CACGATTAAA 60
GACTGANACC GGCAAA 76

SEQ ID NO:812
SEQUENCE LENGTH:129

SEQUENCE TYPE:nucleic acid
TOPOLOGY:linear
CLONE:HUMGS00890
SEQUENCE DESCRIPTION:
GATCTTTAAT ACAATATAGA AATTGTGTAA TAGTTATTAT AAATGTTAAT ACACAACTTT 60
CAGGTAATTT TAACTGATTA TTTCTTTTGC TCTTTTAACT TAAGTTATTA AAGTTTAAAA 120
GTTCGTAAA 129

SEQ ID NO:813
SEQUENCE LENGTH:74
SEQUENCE TYPE:nucleic acid
TOPOLOGY:linear
CLONE:HUMGS00891
SEQUENCE DESCRIPTION:
GATCTGGTCT CGGTGGTCCT TCCCCGCAGG CAGGTGTCAG GACCGGCCTA ATAAACATGT 60
GTGGCCTCCT CAAA 74

SEQ ID NO:814
SEQUENCE LENGTH:82
SEQUENCE TYPE:nucleic acid
TOPOLOGY:linear
CLONE:HUMGS00892
SEQUENCE DESCRIPTION:
GATCCAAATC CCATTACAGT TGTATAAAGA AATAAAATTT TGTACTNATA TTATTAAAAA 60
TCACATTTTT AATATTTGTA AA 82

SEQ ID NO:815
SEQUENCE LENGTH:72
SEQUENCE TYPE:nucleic acid
TOPOLOGY:linear
CLONE:HUMGS00894
SEQUENCE DESCRIPTION:
GATCCCCAGA GACCCCATTT GCCTCTCAAC ACTCAGACCT TCAACTGTTT TTNAATAAAT 60
CTACTTTTTA AA 72

SEQ ID NO:816
SEQUENCE LENGTH:72
SEQUENCE TYPE:nucleic acid
TOPOLOGY:linear
CLONE:HUMGS00895
SEQUENCE DESCRIPTION:
GATCCTACAC CCNGAGCCTC AGAGCACTGC TACTTTTTAA AATACTTCTT TCTCTTAAAA 60
GTCTTTACCA AA 72

SEQ ID NO:817

SEQUENCE LENGTH:71
SEQUENCE TYPE:nucleic acid
TOPOLOGY:linear
CLONE:HUMGS00896
SEQUENCE DESCRIPTION:
GATCAGATGT TAAGACTGAC ATTTCCAAGG TTGGCTACTA TGTAAAATTA AAATTACACA 60
AATTGTGCAA A 71

SEQ ID NO:818
SEQUENCE LENGTH:71
SEQUENCE TYPE:nucleic acid
TOPOLOGY:linear
CLONE:HUMGS00897
SEQUENCE DESCRIPTION:
GATCTGAAGT AATTGTGCTG TATTTATGTT TATTCACCAG TCTTTGATTA AATAAAAAGG 60
AAAACCAGAA A 71

SEQ ID NO:819
SEQUENCE LENGTH:84
SEQUENCE TYPE:nucleic acid
TOPOLOGY:linear
CLONE:HUMGS00898
SEQUENCE DESCRIPTION:
GATCTAGCTC TCTGATTCCA TACATTCCAG ACTTCTCAGT GGATTTGTAA TAAACTATAA 60
ATAAAAATAG CTCTCATTTA TAAA 84

SEQ ID NO:820
SEQUENCE LENGTH:73
SEQUENCE TYPE:nucleic acid
TOPOLOGY:linear
CLONE:HUMGS00900
SEQUENCE DESCRIPTION:
GATCATGTCT TTTCCATGTG TACCTGTAAT ATTTTTCCAT CATATCTCAA AGTAAAGTCA 60
TTAACATCAG AAA 73

SEQ ID NO:821
SEQUENCE LENGTH:69
SEQUENCE TYPE:nucleic acid
TOPOLOGY:linear
CLONE:HUMGS00903
SEQUENCE DESCRIPTION:
GATCTGATTA TTTACTTTGT TTATTGTCTA TATGCCTTTT AAAAAAATAA ACTTGTTATG 60
CAAAATAAA 69

SEQ ID NO:822

SEQUENCE LENGTH:69
SEQUENCE TYPE:nucleic acid
TOPOLOGY:linear
CLONE:HUMGS00904
SEQUENCE DESCRIPTION:
GATCCAGTTG TAGCTGCCAT CAGATGCCGG AGACTCGCCC NTCAATAAAA AAATCTCTTC 60
TAGCTGAAA 69

SEQ ID NO:823
SEQUENCE LENGTH:72
SEQUENCE TYPE:nucleic acid
TOPOLOGY:linear
CLONE:HUMGS00905
SEQUENCE DESCRIPTION:
GATCACTGTA AATGGTAATC AGTTGGAATT CTCCTAAATG TCTTCCAGAC ACTAGTAAAA 60
AACGACCTGA AA 72

SEQ ID NO:824
SEQUENCE LENGTH:68
SEQUENCE TYPE:nucleic acid
TOPOLOGY:linear
CLONE:HUMGS00906
SEQUENCE DESCRIPTION:
GATCTTTCTA CCTGCCTTTC CATGTCATGA GAGGAAGAAA CAAGAATGAC AAGTGTATGA 60
CTNCCAAA 68

SEQ ID NO:825
SEQUENCE LENGTH:67
SEQUENCE TYPE:nucleic acid
TOPOLOGY:linear
CLONE:HUMGS00908
SEQUENCE DESCRIPTION:
GATCACGTAC CTGTGCAGAA ACCGCCTCTG TGGCTGCATT TGAAATAAAA CCCGACCCAG 60
CAGCAAA 67

SEQ ID NO:826
SEQUENCE LENGTH:342
SEQUENCE TYPE:nucleic acid
TOPOLOGY:linear
CLONE:HUMGS00910
SEQUENCE DESCRIPTION:
GATCATATTT TATGAACAGA AAGACTCAGG ACATATTAAA AAATAAACTG AACTAAAACA 60
ACTTTTGCCC CTGACTGATA GCATTTCAGA ATGTGTCTTT TGAAGGGCTA TGATACCATT 120
TATTAAATAG TGTTTTATTT TAAAAACAAA ATAATTCCAA GAAGTTTTTA TAGTTATTCA 180
GGGCACTATA TTACAAATAT TACTNNGTTA TTACACAAAA AGTGATAAGA GTAACATTTG 240

CTATACTGAT GNTTGTNTAC TCAAAAACCT CNGNTNAACN GTATGTAATC TNAGTTCACT 300
GCACTTAAGT TCACCNAACA TNNATNAATG TCAATGNAGA AA 342

SEQ ID NO:827
SEQUENCE LENGTH:72
SEQUENCE TYPE:nucleic acid
TOPOLOGY:linear
CLONE:HUMGS00911
SEQUENCE DESCRIPTION:
GATCTAAGAA GTTGTGAATG TTGTTAATCA TTTAGCCGTT GCAATAAATG TAGAGGAAAT 60
GCAGTGTGCA AA 72

SEQ ID NO:828
SEQUENCE LENGTH:65
SEQUENCE TYPE:nucleic acid
TOPOLOGY:linear
CLONE:HUMGS00912
SEQUENCE DESCRIPTION:
GATCACCTGA GGCCATGAGT TTNAGACCAG TCCTGGTAAC ATAGCAAGAC CTCCATCTCT 60
ACAAA 65

SEQ ID NO:829
SEQUENCE LENGTH:65
SEQUENCE TYPE:nucleic acid
TOPOLOGY:linear
CLONE:HUMGS00913
SEQUENCE DESCRIPTION:
GATCCTAGAC AGCGCCTTAT CTATGATTGA GTGTCCGTGT AAATAAATTC CTACTTAGAC 60
TTAAA 65

SEQ ID NO:830
SEQUENCE LENGTH:65
SEQUENCE TYPE:nucleic acid
TOPOLOGY:linear
CLONE:HUMGS00914
SEQUENCE DESCRIPTION:
GATCTGGGAA CTTTTTNCTG TACAAATCTG TTTAAAAAAA AAAAAAGGNA CCNCATTGAT 60
TTAAA 65

SEQ ID NO:831
SEQUENCE LENGTH:63
SEQUENCE TYPE:nucleic acid
TOPOLOGY:linear
CLONE:HUMGS00915
SEQUENCE DESCRIPTION:

GATCCAAATT AAAACCTGGT AGAATCTAAT ACATTGACTG CAATTAAAAT GTTTGCCTGG 60
AAA 63

SEQ ID NO:832
SEQUENCE LENGTH:68
SEQUENCE TYPE:nucleic acid
TOPOLOGY:linear
CLONE:HUMGS00917
SEQUENCE DESCRIPTION:
GATCCAGTTC TAAGTGTCAT CTTTTATNAT NAAGACAATA AAATCTTGAG TTTATGCTTC 60
ACTTNAAA 68

SEQ ID NO:833
SEQUENCE LENGTH:63
SEQUENCE TYPE:nucleic acid
TOPOLOGY:linear
CLONE:HUMGS00918
SEQUENCE DESCRIPTION:
GATCCACGGT TGTNACCATG TATTACCACA AATTTAACAA TAAAAAATTG TTTTAAGAGT 60
AAA 63

SEQ ID NO:834
SEQUENCE LENGTH:109
SEQUENCE TYPE:nucleic acid
TOPOLOGY:linear
CLONE:HUMGS00919
SEQUENCE DESCRIPTION:
GATCTACTGT CATTTGNATG CAATTTCCTG TTACCTTGAA AAAATAAAAA TGTTAACAGG 60
AATGCAGTGT GCTCATTCTC CCNAAATAGT AAANCCCACT GTATACAAA 109

SEQ ID NO:835
SEQUENCE LENGTH:62
SEQUENCE TYPE:nucleic acid
TOPOLOGY:linear
CLONE:HUMGS00920
SEQUENCE DESCRIPTION:
GATCCAATTA CACACATTCG TTCACAACTC AACACAAATT CCTATTAAAT ATTAAAAGTA 60
AA 62

SEQ ID NO:836
SEQUENCE LENGTH:61
SEQUENCE TYPE:nucleic acid
TOPOLOGY:linear
CLONE:HUMGS00922
SEQUENCE DESCRIPTION:

GATCTTAAAC ATAGGAAAAC CATACGTGTT CATGATAATA AAATGCTTTC TATGAAATAA 60
A 61

SEQ ID NO:837
SEQUENCE LENGTH:56
SEQUENCE TYPE:nucleic acid
TOPOLOGY:linear
CLONE:HUMGS00924
SEQUENCE DESCRIPTION:
GATCATACCA CTGCTCTCCA GCCTGGCTAT CAGAGTGAGA CTCTGTCTCA CAGAAA 56

SEQ ID NO:838
SEQUENCE LENGTH:56
SEQUENCE TYPE:nucleic acid
TOPOLOGY:linear
CLONE:HUMGS00925
SEQUENCE DESCRIPTION:
GATCAGACAC TTAACCCTTA TAANTTAAAG TCAATAAAGC ACCTTTTTAA AGGAAA 56

SEQ ID NO:839
SEQUENCE LENGTH:57
SEQUENCE TYPE:nucleic acid
TOPOLOGY:linear
CLONE:HUMGS00926
SEQUENCE DESCRIPTION:
GATCAAAGTG AAACAATGTT TGGATGCAAC GCAGAATAAA AGAATATAAG AAATAAA 57

SEQ ID NO:840
SEQUENCE LENGTH:52
SEQUENCE TYPE:nucleic acid
TOPOLOGY:linear
CLONE:HUMGS00929
SEQUENCE DESCRIPTION:
GATCTAATTA AAAGACCTTC TGCACAGCAA AAGAAACTAA CAACAGAGTA AA 52

SEQ ID NO:841
SEQUENCE LENGTH:52
SEQUENCE TYPE:nucleic acid
TOPOLOGY:linear
CLONE:HUMGS00930
SEQUENCE DESCRIPTION:
GATCCCGGCA GAAGCTATGA AAGGGAATAA AGAGAAAAGA AGTACCCAGA AA 52

SEQ ID NO:842
SEQUENCE LENGTH:52

SEQUENCE TYPE:nucleic acid
TOPOLOGY:linear
CLONE:HUMGS00931
SEQUENCE DESCRIPTION:
GATCTTTTAG TTTCAACTCA GCTTTTACAA TAAAANGGAT TTGTATTGCA AA 52

SEQ ID NO:843
SEQUENCE LENGTH:58
SEQUENCE TYPE:nucleic acid
TOPOLOGY:linear
CLONE:HUMGS00932
SEQUENCE DESCRIPTION:
GATCTCTTTT CAGAAGTGTC TATAGAACAA TAAAAATCTT TNACTTCTGA CCTTGAAA 58

SEQ ID NO:844
SEQUENCE LENGTH:53
SEQUENCE TYPE:nucleic acid
TOPOLOGY:linear
CLONE:HUMGS00934
SEQUENCE DESCRIPTION:
GATCCTAAAT CATGACTTAC CTGCTAATAA AAACTCATTG GAAAAGTGAG AAA 53

SEQ ID NO:845
SEQUENCE LENGTH:52
SEQUENCE TYPE:nucleic acid
TOPOLOGY:linear
CLONE:HUMGS00935
SEQUENCE DESCRIPTION:
GATCAAGCTG TAAAAAAACN AAAAAATTAA TAAAAATTTC GAGAAATANA AA 52

SEQ ID NO:846
SEQUENCE LENGTH:51
SEQUENCE TYPE:nucleic acid
TOPOLOGY:linear
CLONE:HUMGS00936
SEQUENCE DESCRIPTION:
GATCAGCATT GTGACTTGGA GATAATAAAA TTTAGACTAT AAACTTGGAA A 51

SEQ ID NO:847
SEQUENCE LENGTH:62
SEQUENCE TYPE:nucleic acid
TOPOLOGY:linear
CLONE:HUMGS00937
SEQUENCE DESCRIPTION:
GATCTGAGGT AAACTTTGAA GTAAAATAAA AGCTGTGTTT GAGCATCATT TGTATTTCGA 60

AA 62

SEQ ID NO:848
SEQUENCE LENGTH:59
SEQUENCE TYPE:nucleic acid
TOPOLOGY:linear
CLONE:HUMGS00938
SEQUENCE DESCRIPTION:
GATCCTCCCT CCCCTAATTA AAGTCTCTTT TTGCCCCTTT GGGCTGNCAT GAGGTCAAA 59

SEQ ID NO:849
SEQUENCE LENGTH:71
SEQUENCE TYPE:nucleic acid
TOPOLOGY:linear
CLONE:HUMGS00955
SEQUENCE DESCRIPTION:
GATCAGAATT TTAAATNAAA GGTTTTTTCT TTAAATNATT TGTATTACTT TATTAAAACT 60
CTGATATTAA A 71

SEQ ID NO:850
SEQUENCE LENGTH:661
SEQUENCE TYPE:nucleic acid
TOPOLOGY:linear
CLONE:HUMGS00972
SEQUENCE DESCRIPTION:
GATCCGGTTC TGGGACAGCA GGGGGCCCCA CTGCACCCAG GTCATCCCTG TGCAGGGCCG 60
GGTCACCTCC CTGAGCCTCA GCCACGACCA ACTGCACCTG CTCAGCTGTT CCCGAGACAA 120
CACACTCAAG GTCATCGACC TGCGTGTCAG CAACATCCGC CAGGTGTTCA GGGCCGATGG 180
CTTCAAGTGT GGTTCTGACT GGACCAAAGC TGTGTTCAGC CCGGACAGAA GCTATGCACT 240
GGCAGGCTCC TGTNATGGGG CCCTTTACAT CTGGGATGTG GACACCGGGA AACTGGAGAG 300
CAGACTACAG GGACCCCATT GCGCTGCCGT CAACGCCGTG GCCTGGTGCT ACTCCGGGAG 360
CCACATGGTG AGCGTGGACC AGGGCAAGGA AGGTTGTGCT TTTGGCAGTA GGGCCACGAC 420
CTGCCTGCTT GGGNTGGAGN TTTTTNCCCG AAGCTNAAAG TTTCTTNNGG GGCAATGAAG 480
GGGTTTGGGG TTTGGGATTN GAGNTTNGNC TTGGGATTTA ATTGGGNAAG AAGGCTTGGA 540
AAGACCTTGN CTTTTTTNTT TAAAANTNAA GTATTGGTTT GGGGGNTTAA GGTAATTTTT 600
TTTTNGAATT TTAANTTNAT NTCTAAATTT TTTTCCAAAT TTTGAAAAAT TNTTTTTNAA 660
A 661

SEQ ID NO:851
SEQUENCE LENGTH:641
SEQUENCE TYPE:nucleic acid
TOPOLOGY:linear
CLONE:HUMGS00973
SEQUENCE DESCRIPTION:
GATCTTGGAT GTCTATTATA GGAGAAGTAT GTCCTGCCAA TGTACAAGAA GGCAGCATTG 60

TAGGATTAAC ATTCTTGTCT ACTGTATATT ATCTTGGAAG GCTCTTGTTA ATATGTTACA 120
CTTAATATTC TCCACAGTTA CCTTTAGAGA GAATTTATGA GAAGTTAGTT TCTGATGCAG 180
AGGTTTTTAG GCTGTGATTT CATCAAAAGT CCTAATAGCA TTCTACCTCA AAGGGACACT 240
TAGNATGCCT AAAATTTATT CACTTAGTTT TCCTTTTTTA TTTGAAAAAA TACATGACAT 300
GTAATCTTTT TTTCTTGAAT TCTTTCTCAG ATTTTAAAGT ACTATATTAA AGAAAAAAAT 360
TAATGTCTAA AGGCCTAGCA TTCCTTGCAG GACCCCTATA CTAACCATGG TAATGGGGGA 420
GAGGGGTGGG GCAGNTNNGT AGGGGNACCA GGTTCCAGGC CTCAAGCTTC CCAAAGCCAT 480
TTTTTNTAAA TGGGAAATCC NTNAANTTNT GGAACCCGCT TTGNTATNGG NGCCCCTTTT 540
TTTAAAATTC CNGGCCTTTT TTTNNTTGGT AATGGGGGTT NCTGTTTGNG GTTTTAACCT 600
NANCCTGGNC CGGGGGGGTT TAAAGGAATG CTGNCTGCAA A 641

SEQ ID NO:852
SEQUENCE LENGTH:627
SEQUENCE TYPE:nucleic acid
TOPOLOGY:linear
CLONE:HUMGS00974
SEQUENCE DESCRIPTION:
GATCTGCTNC AGTGCTCTGA GCCCTAGGAT TCATCTNTCT TTTCACCGTA GNGCGCNNGA 60
CTGGCATTGT ATTAGCAAAC TCATCACTAG ACATCGTACT ACACGACACG TACTACGTTG 120
TAGNTCACTT CCACTATGTC CTATCAATAG GAGCTGTATT TGCCATCATA GGAGGCAACA 180
TTCACTGATT TCCCCTATTC TCAGGCTACA CCCTAGACCA AACCTACGNC AAAATCCATT 240
TCACTATCAT ATTCATCGGC GTAAATCTAA CTTTNTTCCC ACAACACTTT CTCGGCCTAT 300
CCGGAATGCC CCGACGTTAC TCGGACTACC CCGATGCATA CACCACATGA AACATCCTAT 360
CATCTGTAGG CTCATTCATT TCTCTAACAG CAGTAATATT ANATAATTTT CATGATTTGA 420
GAAGCCTTCG GTTTCGAAGC GAAAAGTCCT AAATAGGTAG GANGAACCCT TCCATTAAAC 480
CTGGAGTGAC TATATGGNTT GCCCNTACCC TTACCANACA TTCNGAGGAN CCCGTATACA 540
TAAAATTNTN GNNAAAAAAN GGANGGNTTC GNACCCCCCA AAGGTTGGTT TNANGNCAAC 600
CCCCNTGGGC TCNATGGTTT TTTTAAA 627

SEQ ID NO:853
SEQUENCE LENGTH:617
SEQUENCE TYPE:nucleic acid
TOPOLOGY:linear
CLONE:HUMGS00976
SEQUENCE DESCRIPTION:
GATCAGANTG CCCCTCCACT CATGAGACTC TTCATTTTGT CCACTTTGAC AGGAAAAGTG 60
GGAATGTATG CAGAGCTCTC AAAAGAAACA AAAAAGGCCA AAACGGTGCC TTCAGCCACA 120
TCCTCTGAAT TGGCCCTGAC TTGGACTAAA NCCNCTAATG CAAAATCCCT TGACAAAAGC 180
GCATAGGTTA TTTCAAACCA GCATTGTTTT TTATGTAACC TGTTTTACCG CATCTTCTCA 240
GCAGCTTCTG ACCACTGCTC AATTTTTTCC TTTACAGCCA TTGTTCTGGT GGACAAATAA 300
CCTAGGTACT CCAAATCCTG GCAGGAAAAA TATACAGCAT TATGAAACAG CACTCAGTAA 360
TCCTAAAATG GATTTTCCAA AGCTGGTTAC ACATGNCCTG CAAAGTCTTA TTTAAATTTA 420
AAAGGCCTTT CTCATTTACC AGGGGTTTAG GTCAACGNNG GCAAACCCCT GGGGGAATTT 480
AAATTGGGAA GGTTANTTNC CTTTNGTAAA TTCATAGGGN CCAAANGGCN GGGNAGTTAA 540
TTTTTCCATN GGGTTGGTGG CNCCCGGGNT TCATTGGTNT TNGGCCCCAN GGAATTTAAT 600

TTTTTTAAAN CCTTAAA 617

SEQ ID NO:854
SEQUENCE LENGTH:602
SEQUENCE TYPE:nucleic acid
TOPOLOGY:linear
CLONE:HUMGS00977
SEQUENCE DESCRIPTION:
GATCCCGTGC GGCGCGCTGC CCGAGGGGAG CAAGGACAGC TTTGCAGTTC TCCTGGAGTT 60
CGCTGAGGAG CAGCTGCGAG CCGACCATGT CTTCATTTGC TTCCACAAGA ACCGCGAGGA 120
CAGAGCCGCC TTGCTCCGAA CCTTCAGCTT TTTGGGCTTT GAGATTGTNA GACCGGGGCA 180
TCCCCTTGTC CCCAAGAGAC CCGACGCTTG CTTCATGGCC TACACGTTCG AGAGAGAGTC 240
TTCGGGAGAG GAGGAGGAGT AGGGCCGCCT CGGGGCTGGG CATCCGGCCC CTGGGGCCAC 300
CCCTTTTNAG CCGGGTGGGT AGGAACCGTA GACTCGCTCA TCTCGCCTGG NTTTGTCCGC 360
ATGTTGTAAT CGTGCAAATA AACGNTCACT TCCGAATTAA GCGGTNTATT TNTTGAANGT 420
TTAATAATTG TGTTTTNTGA ATACTGAAGT ATTTGGCTTT AAATTCTTAA NTTAAAAATT 480
TAATNTTTTA CTTTTTTAAT TGCTGGGTTT AAGATNGTTN AAGATTATCC TTGNAACTTT 540
NNGGGGGANG TTNTTATTTT NGAGTCTTTT NGGAANAGNC TTNAGGCTTT TNNACTTNGA 600
AN 602

SEQ ID NO:855
SEQUENCE LENGTH:595
SEQUENCE TYPE:nucleic acid
TOPOLOGY:linear
CLONE:HUMGS00978
SEQUENCE DESCRIPTION:
GATCCCAGGG CTCCCTGCCA TTTTAGTGTC TTGGTGTAGT GTAACCATTT AGTGGTTGGT 60
GGCAACAATT TTATGTACAG GTGTATATAC CTCTATATTA TATATCGACA TACATATATA 120
TTTTNGGGGG GGGGCGGACA GGAGATGGGT GCAACTCCCT CCCATCCTAC TCTCACAGAA 180
GGGCCTGGAT GCAAGGTTAC CCTTGAGCTG TGTGCCACAG TCTGGTGCCC AGTCTGGCAT 240
GCAGCTACCC AGGCCCACCC ATCACGTGTG ATTGACATGT AGGTACCCTG CCACGGCCTA 300
TGCCCACCTG CCCTGCTTCC TGGCTCCTTA TCAGTGCCAT GAGGGCAGAG GTGCTACCTG 360
GCCTTCCTGC CAGGAGCTTT NCACCCACTN ACATTCCGTC CCCGCGGCTT AACTGNAGCA 420
AGCGTGGNCC TAGGACAGNA GGAGCTTCGG GCCCNGTTTN ACCTTGCGGT GGGGCTNANG 480
GGTTGGCATT TCTTGCCTGG GGCCACTGGG TTNAAATTTT GGGNTGATNA TTGGGGNGAG 540
GGGTGGGGTA ACAAAACCAN TTTTGGNNAA GNTTGGGANG NTTTTGNCTT TTAAA 595

SEQ ID NO:856
SEQUENCE LENGTH:581
SEQUENCE TYPE:nucleic acid
TOPOLOGY:linear
CLONE:HUMGS00979
SEQUENCE DESCRIPTION:
GATCTGGAAT TGCAGCCCAC ATAAACATAA AGGAGGATGT CCCTGGTCTG TTCCATCCCC 60
ACGGATGGTG TTGCTGCTGG GCAACAGTGT TGGCTTCTTT NAAGTACCCC CTTTCCTCCT 120

CACCCACCTC CAAACTGACT AGCACTCAGA GGGACTTATG ATAAAGGTTC AGCTCCAGGG 180
GTAGTACCTG AGTGTGTGCC ATGCCCCTTC AGACCAGCTG CTTCCATCAG AATTCCAGGG 240
TCACAGCCCC AACAGAAGCA GCAGTGCCTC TGTAGGAGGG GTGCTGGGCT CTGGNCTTCT 300
NATGCAGAGA GGTCCGGGAC AGGGTCAGTA TCGTGGGCAT GTNTATAGCT TCCCAAGTTC 360
TTTTACAAGT CCCNTGCTGG GACTCCCTGA NTTTACTTTT GGTNGGNTTC CTAGGTNCTA 420
ATTGGTTTTA CAAACTTACA NTTTTNTAGG AATTTGANTT ANGATTANCT TGNTTTAATT 480
TAATTGTAGA NTTTNGGGGC CTTTTTGGGN CTCAAATTTT NCCATTACAA GGNNTTATTN 540
GGGAAANAAA GNNGTTTNAA TNAAAATCCT TGGCCAGGAA A 581

SEQ ID NO:857
SEQUENCE LENGTH:569
SEQUENCE TYPE:nucleic acid
TOPOLOGY:linear
CLONE:HUMGS00980
SEQUENCE DESCRIPTION:
GATCTAAATC AGACAGGAGT TGGTCTACAT AGTAGTAATC CATTGTTGGA ATGGAACCCT 60
TGCTATAGTA GTGACAAAGT GAAAGGAAAT TTAGGAGGCA TAGGCCATTT CAGGCAGCAT 120
AAGTAATCTC CTGTCCTTTG GCAGAAGCTC CTTTAGATTG GGATAGATTC CAAATAAAGA 180
ATCTAGAAAT AGGAGAAGAT TTAATTATGA GGCCTTGAAC ACGGATTATC CCCAAACCCT 240
TGTCATTTCC CCCAGTGAGC TCTGATTTCT AGACTGCTTT GAAAATGCTG TATTCATTTN 300
GCTAACTTAG TATTTGGGGT ACCCTGCTCT TNTGGCTGTN CTTTTTTTGG AGCCCTTCTC 360
AGTCAAGTCT GCCGGATGTC TTTTTTTACC TACCCCTCAG TTTTCCTTAA AACGGGNACA 420
CAANCTCTAG NGNGGTGTTA NGANTAATNG TTACTNGGGT TANTGGGGTA NTTNNTGGGG 480
TNTNGGTTTG GGGCTAGGCA TTGTGGTAGG TTTTNAANAA TTAGNGGGTT GGNCCCCNTT 540
NGNTGGGGTG NTTTCANGGT NGAATNAAN 569

SEQ ID NO:858
SEQUENCE LENGTH:566
SEQUENCE TYPE:nucleic acid
TOPOLOGY:linear
CLONE:HUMGS00981
SEQUENCE DESCRIPTION:
GATCCCACCC NGGCCTCTCC TGGAACTCTG AACCTGCTGT GGAAGGAATT GGCCATGACC 60
TTCACCTCTG GAGAGTAGGG TCTATGGCGA GGGAAAAGGG NNTTCACCAT GATAACCTAG 120
TGCCTCCATA GAGGGGTTTG GAAAAATTCC AGTCCGATTT CTTTGTGTGT CAGCTGACTT 180
CCTTAGCTGA TTGTTCCCAC TTGCACCTCT CCACCTTTGG CACTAGAACT CCTGAGACAC 240
CACTTCTCAT GCTTCTCCCT CCCTACCAGC GGTCAAGGCT TTGGAGCCAC TCTTTTGTAA 300
CTCCAGATTA TTTAAAGAGA AAAGTACAAG ACAGAAATCT TCTAGCACTT TGTAAACACA 360
GTTGATTAAC CCTCTTGGGN GTATTTTTTG GGCTTTATAT AAAANCANGG TTTTTTAATT 420
NGTAAAGTNT AAGTGCCATT AGGAANATGC ACCAGGGCAT ATTTTTGGTT NAAGGTGGTT 480
TTTTCAATGG TTTTNCAGGN TTNCATTTTC AAAAAAANGG TTTTTTTAAT GGAGGTTGTT 540
NTTNAAANNT TCNTGANTGG TGGAAA 566

SEQ ID NO:859
SEQUENCE LENGTH:556

SEQUENCE TYPE:nucleic acid
TOPOLOGY:linear
CLONE:HUMGS00982
SEQUENCE DESCRIPTION:
GATCTACAAT TACTAATTAA AGCTGTGAAT CTNTTCCCTG CTGGAACAAA TTCAAGATGG 60
GAAGTTATTG CTAATTACAT GAACATACAT TCTTCCTCTG GAGTCAAAAG AACTGCCAAA 120
GATGTTATTG GCAAAGCAAA GAGTCTCCAA AAACTTGACC CTCATCAAAA AGATGACATA 180
AATAAAAAGG CATTTGATAA GTTCAAAAAA GAACATGGNG TGGTACCTCA AGCAGACAAC 240
GCAACGCCTT CAGAACGATT TGANGGTCCA TATACAGACT TCACCCCTTN GACAACAGAA 300
GNACAGAAGC TTTTNGAACA AGCTTTGAAT ACATACCCAG TAAATACANC TGAAAGATGG 360
GNANAAATAG CAGTAGCGGT GCCTGGCAGG NCAAAGGAGG GNCTGCATNN ANCCGGTTNC 420
AGGGGACTTT GTCGNGNTGG GTAAAGCCAA AGGAAGCTTG TTCCAGGTCN ANGTGCTGGA 480
TGCAAGTTGG GGCNTGGNAT TNNCNATTTT TTGTTGGGGT GTTCCTTTTT TTANTAAACC 540
TGNANTTCTT TTTAAA 556

SEQ ID NO:860
SEQUENCE LENGTH:555
SEQUENCE TYPE:nucleic acid
TOPOLOGY:linear
CLONE:HUMGS00983
SEQUENCE DESCRIPTION:
GATCTGGATT ATTTGTCTAA GTGAGAGATT GCGAATATCA AAATATCTGT CTCACTTCTT 60
CTGTGAATNA CACAGAGTAG AAATAAATTC ACTTTAAAAA TATGACTGAA TTTTGAAAAT 120
CAAGACTGAA TCTCACATAG CTGCAGACAG GAACTAAGCC AGCCTCTTTG TATGTGGTAA 180
CAAGTACAGT ATAAGANTGA AAGATTTACC ATCCTTGAAA GCTCTAATGA AAATCAAATC 240
CAGCAATATA TATTCAACTG TGTACAGGAT TTAAGAACTT ATTTTATGAA GGAGTAATAG 300
TGTGTAGATA TAGATTCTGA AGTCTTTAAA CGTGCCTTAA TAAATNAAGT TCNCTGGCAT 360
TGAGNTGANN ACCAGGTGAC CNTTGGGGNC AAAAACCCNC ACAAGTGATT NGCACACCAG 420
TATACNTTCA CCANTATACT NTNTGCACAC ACANCNTTTG TTTNGGTTCA GGGTTTTGCA 480
AATNGGTCCN ATGTATTGGC ACTGGCGTCT TTGNATTGTG TAAGTGGNTA TTTTNTGAGG 540
NTAGCGTGGT NNCNN 555

SEQ ID NO:861
SEQUENCE LENGTH:554
SEQUENCE TYPE:nucleic acid
TOPOLOGY:linear
CLONE:HUMGS00984
SEQUENCE DESCRIPTION:
GATCCATGTC TCAGCCTCCA CCCCGCCACA CCTGTCTGTG CAGCCCACCG GCCTTACCTT 60
CTACCCTGCC GTGGATGTCC AGGCCTTTGC CGTCCTCCCC AACTCCTCCC TGGCTTCCCT 120
CTTCCTGATT GGCATGGTAA GCAGTTCCTG GGTTGGACAG ATGAGGAGCC CCAGACAGTC 180
CCAACAGCAC TGTCTTTGGA GTCAGGAGAC CATGTGAATC CTGTCTGGAT TCAAACCTGG 240
ACTGTGTCAC TCCGGAGCCT GAGGCTTGAG TCACTGTACT CAATGGTGCC GACTCCTGGA 300
GGTATTCATT CACCCAGCCA TTCACTAGTG CGTTTGTTTA CTTATTCATT CAATTATTCA 360
TTCAGTCAAT TTCTCATTCA TTCANTTATT CATTCCATGT TGGCTTGAAA TATGTGTACT 420

GTNCCAATTN ATCCATTTAT ATCTTTAGTC ATTCAATTAT GCATTNGTGG TATTTGTTCA 480
TTNATTCANT TNTTAATTTN ATTNAGTTAN TNNNTNGGTT GGTGNCTTGG NGTANNGTNA 540
TACATTTGNG GAAA 554

SEQ ID NO:862
SEQUENCE LENGTH:549
SEQUENCE TYPE:nucleic acid
TOPOLOGY:linear
CLONE:HUMGS00985
SEQUENCE DESCRIPTION:
GATCCTATGG TTCAGGAGGC CAGAAATTTC CACCTCTAGG AGGTGGTGGT GGCATAGGTT 60
ATGAAGCTAA TCCTGGCGTT CCACCAGCAA CCATGAGTGG TTCCATGATG GGAAGTGACA 120
TGCGTACTGA GCGCTTTGGG CAGGGAGGTG CGGGNCTGTG GGTGGCAGGG TCCTAGAGGA 180
ATGGGNCTGG AACTCCANCA GGAATATGGT AGAGGGAGAT AAGAGTACGA AGNCCAAACA 240
AAAAACCCCG ATTTAGATG TGATATTTAG GCTTTCATTC CAGTTTTGTT TTGTTTTTTT 300
GTTTAGATAC CAATCTTTTA AATCNTTGCA TTTTAGNAAG AAGCTATCTT TTAATGGTTG 360
TAGCAGTTAT TGACCTAATA TTTGAAATGG CTGTTGGCAG TAAATTATGA ATCAGTTTTT 420
GACCAGGNGA TTTTTTTNCN TNTATTCCTT ANTTTCCTGT TNCTGATATN CCCCCAAGTA 480
TGCAGTTNCT NNNCNCTNAA TTCCANGGNN CCATTTTTTT TTCNGGGTTC AANAATNGAA 540
TNGNTTAAA 549

SEQ ID NO:863
SEQUENCE LENGTH:543
SEQUENCE TYPE:nucleic acid
TOPOLOGY:linear
CLONE:HUMGS00986
SEQUENCE DESCRIPTION:
GATCAGCAGT CTTCTGCTGC CTTCTTAATG TTTCACCTAA GTTCCCATAG TTGCTCTCAA 60
AAAGTTTCTT GGCCCTCGTG GGCCAGTNTG GAGGGGCTGT ATCATCATTC ATNAGGAGCG 120
TCACTTGGTG GTTTAGGGGG AAATAATCTA TTCTNAAGAT TGAAGAGAGT GCAGGATTTG 180
GGGGGTTGAC TCTACATCCT TCAGTATCAG GGCTTATCTC CTTGTNTTAC CTCCTAGGAG 240
ACCCTCCTGT TCTTAACTGT GGGCGATGAG AAAGGTGGTG GACTCTTCTT ACTGGCAGGG 300
CCACCTGCGT CTGTGGAGAC CCTGGGGCCC AGGGTGGCTG AGGTCCTGGA AGGCAAAGGA 360
GCAGGGAAGA AAAGGCCGTT TTTCAGGGCA AGGCCACCAA GATGAGCCGG CGGATGGAGG 420
GCGCAGGCGG TTCTCCAGGN CTACATCAGC AACGNAGAGT NCTAAGNANT TANGGCTTTA 480
GGGCACTTAN CTNCTGGTTT CCACAGGAAT CTTTTNGTCA ATNAAATTAG TTTGCCTCAG 540
AAA 543

SEQ ID NO:864
SEQUENCE LENGTH:538
SEQUENCE TYPE:nucleic acid
TOPOLOGY:linear
CLONE:HUMGS00987
SEQUENCE DESCRIPTION:
GATCATTTCT ATAAAAGAAA TGGTCATTTT ACTTAGATGC CAGTCTACTT TATAAAGACA 60

AATGGATTAT AGACTTAAAA ATAGTCATTT TTCTTATTCA TAAATCTGAC AGGCATAAAC 120
CCAAATCAAA GATAATTTGG TGCCCATTAT GAATTTGAAG TTAAGTGATA GCTCACTTGT 180
AAAGTGACTA CCTTAATGTG TATAGAGACC CCAGTCTACT ATTATTTGGG AAAATTGTTT 240
AGGTTATATG GGAAAAGTAG CTCTTTAAAA ATCATATTGC CCAACAGAAA CCTTAGGCTG 300
AATTTACAGG TATGATAATT TTTGTAATTA ATTTTCTTAG AATTGTGCAG GCTGGGATGG 360
GGATAATGNC ATACTCTTTT ACACTGTACC AGCAGCATTT ATTNNCCTNG GACCTTTTAA 420
CCNTTTTAGG GGTTAGGGTN CTNGGGGAAC CAACCTTAAT TNGGNCATCC TCCATTTNNC 480
TTNTNTNCCN NNNNCCCNNT TTTTTTTTTG GNCCCCNTTN GGNCCCCTTA AACNACCN 538

SEQ ID NO:865
SEQUENCE LENGTH:533
SEQUENCE TYPE:nucleic acid
TOPOLOGY:linear
CLONE:HUMGS00988
SEQUENCE DESCRIPTION:
GATCAAAACT ATANGTGGCT ATGGCGGATA TGATTATACT GGGTATAACT ATGGGAACTA 60
TGGATATGGA CAGGGATATG CAGACTACAG TGGCCAACAG AGCACTTATG GCAAGGCATC 120
TCGAGGGGGT GGCAATCACC AAAACAATTC CCAGCCTTTC CACATTGGCT TTCCCATGTA 180
GTCCTTAGTG TGTCTGCTNC TCCTCTCTCT CCTCATCACA GTTCCCAGCC CCCACCTTCA 240
ACTGAACTCA ACAAAATCTT CAACTTCATA CAGTAGTCAC ATTGTTAGTA ATAACACTGG 300
GCATTTTTAT TTTGATANAN TAGACCGTTT AAATTTTTGA GATTCTACCT TATATTTTTT 360
GAATTATATA CTAAAGCANA TAAGTAGGTG NTGTAATGTC CATTGGGGNC CAAGNTTTTT 420
AGGTGTAAAT GGNAAAAGGG TANCAAATTT NAANCTCAAG TAAACACCCT GTAGGCTTTC 480
CCCATGGNTT GGGGNATNTC CGGATTAAGG NCAGGGTTTT CCNTTTTCTN AAA 533

SEQ ID NO:866
SEQUENCE LENGTH:532
SEQUENCE TYPE:nucleic acid
TOPOLOGY:linear
CLONE:HUMGS00989
SEQUENCE DESCRIPTION:
GATCTGAAGT GGTGGATTCC TTGTTTTTGC TAGTATCTCA TTTAGAGTTG AGATGGACCT 60
TAAAACTCAT CTGTTTTAAC TCACTTTTTA ATAGATGAGT TAAACTTAAT TTACTTAAGG 120
ANNNNCAGTT AGAGCCTGGA ACTTCAACCA TTATTCACTC CCCATGCCCT GTTTCCCCCC 180
ACTTCGAAAT TAAATGCGGT TAGCATCATA TAGTTCATTT TCCCCCTCCA TGCTGCTGTG 240
TGATTCTTGA CCTTGGGTAT GAGTTTTTCA TCCTTCATGC AGGGTTCTGT CAGTTCATGG 300
TATAGTGATT CAGTGTTAAA ATGGTGGTGT CTCAGCTGTG CTGTGCACAT TTCCAACCTT 360
GTCAAATTAA TAGTCCTGAG CAAGCAAGAA AAAGAGGTAA TAACATACCC ATTTTCTTTT 420
ATGGANTATA AGCTTAATAA TATTTTTTTC NATGNGCCTA TTTTTTACCT GNGCAAATTN 480
GTATGGNCTC ACATGGTTAA CCCCAATNAA TTANTCTTGG NCAATTTTTA AA 532

SEQ ID NO:867
SEQUENCE LENGTH:528
SEQUENCE TYPE:nucleic acid
TOPOLOGY:linear

CLONE:HUMGS00990
SEQUENCE DESCRIPTION:
GATCGCAAAT NCACCTAAAC AATACATTTA CAAAGCCATC TTTACATGCA TTAAACGAGG 60
GCTACAACAA TATTGTTTTA CAAATACTAG CACTTTTTTC CTGTTATGTA CTTAGTGTTA 120
GAGGGTCAAA ATAATCTTTC TGCTTAGCAT CTCTTAAACC ATACCTGCAA ATATAGCAGG 180
ATTNTTACAT TTACAGTACT TTAATACTTG TATAANCTAT GCAGAAATTT TTAATAAAGT 240
GTAATATATT TNATAAGCTA ATAAGACTGA ATGGGTAAAG GTTTTTNGCA TGCGTTAGTA 300
TACTTGCAGA TACTGAAACA TTTTGGTAAT CTTTCTTACT AAAGGATGTG AATGTTTAAT 360
GTACCTTCTC TGTTTCTACT CTGTAGTCCA ATGGGAATTC AGTAATGNCA TTTTGNCATG 420
TCAACCTGGG GACCATAAAN TTGGTCCTGG TCCAGGCCCT CATNTCCTAT ATCCAGTATG 480
CAATNTTATN TNNTNTNCCT GGTNAATNAA CCCCTCCGGG NTTTTAAA 528

SEQ ID NO:868
SEQUENCE LENGTH:526
SEQUENCE TYPE:nucleic acid
TOPOLOGY:linear
CLONE:HUMGS00991
SEQUENCE DESCRIPTION:
GATCTTTCCT CAGTATGTGC TGATGTTTGG GTTGCTTGTG GAATCACAGA CACTCCTAGA 60
GGAGAATGCT GTTCAAGGAA CAGAACGTAC TCTTGGATTA AATATAGCAC CTTTNATTAA 120
CCAGTTTCAG GTACCTATAC GTGTATTTTT GGACCTATCC TCATTGCCCT GTATACCTTT 180
AAGCAAGCCA GTGGAACTCT TAAGACTAGA TTTAATGACT CCGTATTTGA ACACCTCTAA 240
CAGAGANGTA AAGGTATACG TTTGTAAATC TGGAAGACTG ACTGCTATTC CATTTNGGTA 300
TCATATGTAC CTTGATGAAG GGGATTAGGT TGGATACTTC ANGTGAGGCC TCCCNCTGGA 360
AACAAGCTGC AGTTGTTTTA GNTANTCCCA TCCNGGTTGA NATTGGGNGN GGNCCTTGNN 420
CCTAGCATTN CGCATCACNA AGGCAATGTC NGCNTCACAG TTANGGCATT GNGGGGCCGT 480
TTTNCCATGN GNACTGGGTT ATTGGGGNCT NACCAGGTCC AANTTN 526

SEQ ID NO:869
SEQUENCE LENGTH:526
SEQUENCE TYPE:nucleic acid
TOPOLOGY:linear
CLONE:HUMGS00992
SEQUENCE DESCRIPTION:
GATCCTGTTG CTTCTGCAGG CCATTTTCTG AAAACCCCTG TTAGGAAGGT TGGATTTGGC 60
GTGACTTGCT TGAGCAAGAG TCCTGGGGAG AGATTTTNAG GTTTAATTTA ACGGTATATC 120
CAGAGCTAAC AGTGACTCAA CTCGTCTAGT TCTGCAAGTC AGATGTACAC TTAGAGTCTC 180
TCTGTGAAGG GTTTGGGTCT GAGCTGTATA GTATGTCAAA CTGCCAGTAA GCCAGCCCCT 240
CACCNTCTGA TAGATATTCC TTTAATGCAC CAGACTTCAT GTTTGATAAA TGATTAATGG 300
TTGAAATTGT TTCTCTTCTT TTGTGTTTTC CCAGTTAATA GATGGTCACT GTTTCCACAA 360
TGTTTTATAC TTTCCAGCTT TTNGTAACTN AACCTATAAT TACTTNAATT TTAATTTTTT 420
TTAAAGCTTN GTTGGTGGNC CTAATGNGAA GGTNTTTTTC CAGTGCATNA ATGGTTTTTT 480
NTGGNGCTTC TGNNAAATGN CCNTCCCAAT TGTGGGTTGG GTTTTN 526

SEQ ID NO:870

SEQUENCE LENGTH:520
SEQUENCE TYPE:nucleic acid
TOPOLOGY:linear
CLONE:HUMGS00993
SEQUENCE DESCRIPTION:
GATCAGGACG GCTGGATTCA GGTGTCGTAC GAACAGTACC TGTCCATGGT CTTCAGTATC 60
GTATGACCCT GGCCTCTCGT GAAGAGCAGC ACAACATGGA AAGAGCCAAA ATGTCACAGT 120
TCCTATCTGT GAGGGAATGG AGCACAGGTG CAGTTAGATG CTGTTCTTCC TTTAGATTTT 180
GTCACGTGGG GACCCAGCTG TACATATGTG GATAAGCTGA TTAATGGTTT TGCAACTGTA 240
ATAGTAGCTG TATCGTTCTA ATGCAGACAT TGGATTTGGT GACTGTCTCA TTGTGCCATG 300
AGGTAAATGT AATGTTTCAG GCATTCTGCT TGCAAAAAAA TCTATCATGT GCTTTTCTAG 360
ATGTCTCTGG CTCTATAGTG CAAATGCTTT TATTAGCCAA TAGGAATNTT AAAATACCAT 420
GGACCTTACA CAAAAGGCTT TCATGNCCTT ACTTTNTNAA AAGGGGTTAT TGTATTCATT 480
GGATATGTGC CGTAGCAATN NNGGGNTGTT AGCGGNTAAA 520

SEQ ID NO:871
SEQUENCE LENGTH:517
SEQUENCE TYPE:nucleic acid
TOPOLOGY:linear
CLONE:HUMGS00994
SEQUENCE DESCRIPTION:
GATCTCCGTG GCTTTGGGTT AAAAGACACA CTTGTCCACA TAGGTTTAGA GATAAGAGTT 60
GGCTGGTCAA CTTGAGCATG TTACTGACAG AGGGGGTATT GGGGTTATTT TCTGGTAGGA 120
ATAGCATGTC ACTAAAGCAG GCCTTTTGAT ATTAAATTTT TNAAAAAGCA AAATTATAGA 180
AGTTTAGATT TTAATCAAAT TTGTAGGGTT TCTAGGTAAT TTTTACAGAN TTGCTTGTTT 240
GCTTCAACTG TCTCCTACCT CTGCTCTTGG AGGAGATGGG NACAGGGCTG GAGTCAAAAC 300
ACTTGNANTT TTGTATCTTG ATGTCTTTGT TAAGACTGCT GAAGATTTAT TTTTTTCCNN 360
TTATAATANG GGGNTAANCC CCACCTTNAT TCCTTCAATT CANCCTACCA TTTTCNNGGG 420
TTCTTNGTGT TGGGCTTGTG GCAGGNCCAG CTTNTGGGTT TTCCTTTTTN CCATGCCAAN 480
NTTNTNAATT NCCCATGTAC CAGTTTGNTN CAAAGGN 517

SEQ ID NO:872
SEQUENCE LENGTH:517
SEQUENCE TYPE:nucleic acid
TOPOLOGY:linear
CLONE:HUMGS00995
SEQUENCE DESCRIPTION:
GATCAGAAAT ACAGATTTTG ATAGCAAAGC GACGTTAGNT NGANGCTCTT GTGAGGAAAG 60
TCATTGGCTT TATCCTCTTT AGAGTTAGAC TGTTGGGGTG GGTATAAAAG ATGGGGTCTG 120
TAAAATCTTT CTTTCTTAGA AATTTATTTC CTAGTTCTGT AGAAATGGTT GTATTAGATG 180
TTCTCTATCA TTTAATAATA TACTTGTGGA CTAAAAGATA TAAGTNCTGT ATAAANNCNN 240
CCAATTATGT TAAACTAGCA TATCTGCCTT TATTGTGTTT GTCATTAGCC TGAGTAGAAA 300
GGCCTTTAAA ATTTTTTTAG AAAGCATTTG AATGCATTTT GTTTGGTATT GTATTTATTC 360
AATAAAGTAT TTAATTAGTG CTAAGTGTGA ACTGGACCCT GTTGCTAAGC CCCAGCAAGC 420
AATCCTAGGT AGGGTTTAAT CCCCAGTAAA ATTGCCATAT TGCACATGGT CTTAATGGAN 480

GTTTGAATCT TAAATAAATT GGATATTCAC TTTTAAA 517

SEQ ID NO:873
SEQUENCE LENGTH:515
SEQUENCE TYPE:nucleic acid
TOPOLOGY:linear
CLONE:HUMGS00996
SEQUENCE DESCRIPTION:
GATCTGCGAA AGACCAACTT TTAGGCAGTG ATACTTTTCT CCCATTCCCT GGGGTGGGGG 60
GAGTATGCAG TTGGTGCTTT CTGTAATTCC CTTGTNCTGT TTTGTTTCTG TAAGCTTTTC 120
CCCTGGTGTC ATGGAAAGGA CTTCTTAAAT AACCACATTG TGGGTGGCTG TATCCAAAGT 180
TTAAATAATT GGCCAGAAGT GCAGAGTATC CTTTCCTGGA TTCGTGTCAG AAAAGGGCTC 240
CTTGCCACAA CTGAACTTAC TGTATAAAAA CCTGGCTAGG GAGATTTAAT TTTACTAAAA 300
TTACAGTTTA ATGTTACCGT CTAGCCACAA ATCAAGCAGC AAAAGCTATT TTGATGATGA 360
AAGGGGGTCC CGTTGAGCTG GCCATCTAGT GCAGTGTGCT CTCAGATNCC ATGTTTGTTG 420
ATTGTGTGCT TCACAAGNCC NTCTCTGGTG CTTGAATTGG ATTTGAATTC TTGGTNAGAA 480
GNCTCAGCAT CTCCTTGGGG TNGGCTTGGG CCAAA 515

SEQ ID NO:874
SEQUENCE LENGTH:514
SEQUENCE TYPE:nucleic acid
TOPOLOGY:linear
CLONE:HUMGS00997
SEQUENCE DESCRIPTION:
GATCAGNNAA TNAAGTGCAG CAATATCATG AATTCTCAGA AGCCCTTTCA GGGAGCCAGT 60
NAGTCATACA GTATCCACAG TTGAGTCACT TAAAGATGTC AGTATACGAA ACATTATTCA 120
CAATCCTTGG GCAATCTCAT TTTTTTTTCC TTCTCCCCTC CTCCCCTGCC CCCCATACAT 180
TTNTATCCTT GAGTTAGTTT TGGNGGGGCA GGAAGTACTT AACATCTCAG AAGCTAGATT 240
GGGAAACATG CTCAGCTATA AGAACTGAGC TTTAAATTTT GAGTTTAAAA ATGTACATCA 300
GGAGCAGNTG GGGAGGGTCT TTTTTTTNAA AAAAATCTTT CCAATTTTGG GTTTTCTNTG 360
CCATATGGCC GTTTTGTAAA TNCTTTNGGG GTTTTTNATT NTTTTNGAAA GTGGNTGAAA 420
TCTTGTTNTG GGNTTTTTTT CCCCGAAACA TTTNNAATAT AACCCNGTTT ATTTTTNNAT 480
GNAAATTAAA CCTTNTTTGG GTAAAAAGGT TAAA 514

SEQ ID NO:875
SEQUENCE LENGTH:513
SEQUENCE TYPE:nucleic acid
TOPOLOGY:linear
CLONE:HUMGS00998
SEQUENCE DESCRIPTION:
GATCTTCTAT TAAANTGATT CCTCTTTATT AGAGAAGGAA AATTTAGTTG CTATACATCT 60
TATATTTNCA CAGTTTATTA AAGTCAGTNC CCTAAAGGTA CCTNCTTTNC TTTGTGGCAT 120
ATATGGCATC TNCTGTCTTC AGATTTNCTT ACACTTTTGT GATTTATAAT GTTAGTGATT 180
GGTGCCTTAT TCTCTGAGGA AAGATGGAGG GTTCATAAAG CAATGCCTTA TCCACAGCAG 240
ATTTNCTTGT ATATTAAGTT AAACAGAATT CTGTAAATTA TTATGAAGGG TTCAAGCTCT 300

TTAGGGGGAG TTTTTTTTTC TTGTTGGTTA ACAAATTGGT TGCAATCTNT TATATTTCGC 360
AATTGGNTTA GATATTACAG TCTACTTATT TTTNCANGNG TAAATTAATT GTNTAAGGTT 420
TGGGTTNGGT ATAAATGGNT AAAATATTAA TATNGTGGGG GGTAAAATTT GATTNGGNGT 480
TTTTTTTTTT NAAGGCCNNN GGNTTTANGG AAN 513

SEQ ID NO:876
SEQUENCE LENGTH:510
SEQUENCE TYPE:nucleic acid
TOPOLOGY:linear
CLONE:HUMGS00999
SEQUENCE DESCRIPTION:
GATCAGTATG AGANGCAATA CCTAATCCTA TGTTGCTATT GTATTTTTNC CTAGTTGGTG 60
TGCCTGCTCA GAAAAACATA TACTGTATGT GTATACATAC CTGTGTATAT ATAAAAGGTC 120
AATTTATATA TNTNCCTATA GGAAAATGGA GTAACAAGTT CCCTATCTCC CATATTTATT 180
TGTCCATAGT AAAATGGCCA CATTGATGAT AATTTCTAGA ACTAGTTTCT GAGATTGTCA 240
GCCCTTTGTC TAAAATAATG GCAGTATTAA TGATTGACTT CTGTCACTGC CATAGTTACC 300
TGGATTGTCA GCCTNGGTAG CCTTTGTCTA AAGTCCTAAA GAGTTCCAAA AAAAATGTGT 360
TGAAATAATT GCTAAATAGT GGTGGGTGAT TCTTNCAGTA GGNATTTGTA ATAATTTCNT 420
GGCAAANAAG GTTATTNCCT GCTATTGGTA TTGGATNATT NGNCTTNTAT NCNGGTATTT 480
TNNAAAAGGC AGGNNTATAN GGNTNNNCCN 510

SEQ ID NO:877
SEQUENCE LENGTH:504
SEQUENCE TYPE:nucleic acid
TOPOLOGY:linear
CLONE:HUMGS01000
SEQUENCE DESCRIPTION:
GATCTTCCAT CCTCAAATGA CTCTTTTTTC TTTATATGTT AACATATATA AAATGGCAAC 60
TGATAGTCAA TTTTGATTTT TATTCAGGAA CTATCTGAAA TCTGCTCAGA GCCTATGTGC 120
ATAGATGAAA CTTTTTTTTA AAAAAAGTTA TTTAACAGTA ATCTATTTAC TAATTATAGT 180
ACCTATCTTT AAAGTATAGT ACATTTTACA TATGTAAATG GTATGTTTCA ATAATTTAAG 240
ACCTCTGAAA CANTCTACAT ATACTTATTA CCCAGTACAG TTTTTTTCCC CCTGAAAAGC 300
TGTGTATAAN ATTTATGGTG GATAACCTTT TATGGTTTCC CTTTCCAAAG GCCCAGGGTG 360
GGAGGGGGGA TTAAGGGGGC CTAAGGTNTA TGCCTCCNNG GTTTTAAANT TAAATNCCCT 420
CNNGGTATTT AAATTANNTT TTNCCNANGG TTTNTNGGGG GANTGGGGGG GTTTANANTT 480
GCCTTNTTTN GGGGTTTGGG GAAA 504

SEQ ID NO:878
SEQUENCE LENGTH:500
SEQUENCE TYPE:nucleic acid
TOPOLOGY:linear
CLONE:HUMGS01001
SEQUENCE DESCRIPTION:
GATCTGGGCT CTGAGCACAA GTCAGGAAAC ACCAACATAT TCACACTCTC CCAGTAGGTT 60
CCTCAGTCCG ATGGTGAATG GCTATTCGTA AATGGCTGGT CTGGCTCTTT GGTGTTGGAG 120

CCTTTCCAAT AGCCCCATGA AAAGAAGCAT CACCCAAGGA TATTGTAAAA AGGATGTAAC 180
AAGGAGATAG GGTAGACATT GTACTCAGTG GGCCTTGGGG CCTAGCCCAG CTCTGAGCAG 240
AGGACTGTGG CATTCACTGT CCTTGAGTGT TTCACCTTCT TGGATAACAC ACGGGCCTTC 300
TCTTCTGGAT TTCATCAGAG ATTACAGCCA GATGGGGGCT GAAGACCATC CTCTTTGACC 360
ACAGAGGGTG TGACTGTGGG GAATTCCTCC CAATTTATGG TTTCCNAGGA AAATCTTAGT 420
TCCTTTTATT TATAGGAATG CATGNCNTTT TGGTGTTAAG GAAACCCAAG GGGNANTTAA 480
NGGGACCANT CCTANTNAAA 500

SEQ ID NO:879
SEQUENCE LENGTH:500
SEQUENCE TYPE:nucleic acid
TOPOLOGY:linear
CLONE:HUMGS01002
SEQUENCE DESCRIPTION:
GATCTCCAGC CTCCCAGAAG CCTGCTGTGC TTTCGTCCCA CAGCTTTCTG CCCATTGTTT 60
CTTACTAGTT TCTTGAATTG TNCTTGTGGA CTTTNCCTCA GGGATACATT GGCCTGCAGG 120
TCCCAGTTCA CATGTAGTCC CCTGCTCACC ATTGGAGAAT CAGCTCACTG CTCTCTAGAA 180
ACGTGGCGTT GGTGAACGGA CCATGCTTCC GTAGCTCTGA CCTGGGCAGC TTGGACCTGG 240
TCATCCTCTA CTGCCATACC TTTCCCTGGG GGCTTGAACA CAGAACAGGG AGATGGACAA 300
CCACTTCAAA GAAAGACCCA CCGAATGCAG TTTCTGCTTG ANTGACTGGG NCTGCAGTTC 360
CNTTNTCCTG GGACTTAGAG GTGGNCAGAT NTANGGCCCC TTTACTCATC CANCTTNGTN 420
TTCAACTGGN ACTNCCNAAT NANTNAAAGA GCCTNAAATT TTAAACTNGN TGTGGATNGG 480
GNATATGGGA NTAGGGTTGN 500

SEQ ID NO:880
SEQUENCE LENGTH:500
SEQUENCE TYPE:nucleic acid
TOPOLOGY:linear
CLONE:HUMGS01003
SEQUENCE DESCRIPTION:
GATCGTCAGC AAGTACTGTA NCTGTAAAGG AAAATCTCTC TCTCTGGAGA ACCCACACAA 60
ACCATTGACC TGAGTGCGCA TGACAGCCAC TGGNNATNTT TTCTATGATT GAAAATCTGC 120
CATCGCTGAC TGTTGGCCAG TTTCAAAGGG ACCCATTGTA TACAGGGTGC AAATGTATTA 180
TACGGATGTT TCCTTTTGTA CACTTCATTT TTACAAGTTT TGCTACTCAC AAGCTTTATG 240
TAGTGGAGGA TAGAGGTATT TTTGGTCTTT AGAAGCTTGT CGGGGTGAGG GCTGCTAACT 300
TACACTTCAG AGGCCTGTGT CCCAAAGGCC TGGCTGCGTT TGCCGTGCTG TGCGAGGACC 360
TGTGTACACA GGCAGGTGTT CGCCTGCCCG AGCGCGAGTA GCTCTTTGTG TAGTNGGTGA 420
AAATGCTTGC AGGCATCTGT TTAATTAAAA ATTNCCTGCT GTTAAAGNCA GGGGTTAAAA 480
ATNTCCACAA TTTANGGAAA 500

SEQ ID NO:881
SEQUENCE LENGTH:498
SEQUENCE TYPE:nucleic acid
TOPOLOGY:linear
CLONE:HUMGS01004

SEQUENCE DESCRIPTION:
GATCAGGATT CAGANGTGGA CATCTTCCCC GCAGACTNCC CTACTGAGCC ACCTAGCACT 60
GCCACGAACC GGTCGGGCTA GGAAAGAAGT AAAATATTTT GCAGAGTCTG ATGAAGAAGA 120
AGATGATGTT GATTTTGCAA TGNNNTAATT AAGTGCCCAA AGAGCACAAA CATTTTTCAA 180
CAAATATCTT GTGTTGTCCT TTTGTCTTCT CTGTCTCAGA CTTTTGTACA TCTGGCTTAT 240
TTTAATGTGA TGATGTAATT GACGGTTTTT TATTATTGTG GTAGGGCCTT TTAACATTTT 300
GTTCTTACAC ATACAGTTTT ATGCTCTTTT TTTACTCATT GAAAATGTCA CGTACTGTCT 360
GATTTGGCTT NGTAGGAATT GTTTATAGGN CTGCCCGTGC ATTAGGCACA GGATTTTTAA 420
ATTGTCCATG GGTTNCCANC CTACCAGACC CTGCTTTTTT NGNNAATNGG AATTTTNAAC 480
CATTNANAAA TNGGGAAA 498

SEQ ID NO:882
SEQUENCE LENGTH:494
SEQUENCE TYPE:nucleic acid
TOPOLOGY:linear
CLONE:HUMGS01005
SEQUENCE DESCRIPTION:
GATCTTACTC CACGNATACT TTTTGGTNTG TGAAGGCATC GGTTAAGGGC ACAAAGACAG 60
CCATGGGGAC ATTTATGTAA ATACGTCTCT AATTGCCACA CTGCAGCTGA ACAGTGTGTA 120
GTATTTTCCC AGTCAGCTTT GCCATACTGA CGTCAATCAT TTGAGAGAAA TTATTCAGAT 180
TTNATTTTTG TATCTGTGGT AACAAAACAT TAACCAAAAG ATTTTNTGTC CAGAAGCCTC 240
CCCGNCCCCC CAAGCTATTT GCTCACATTA ACANATTAAA GTGCCTGAAG CATAATTCAT 300
TCTTTACCTG TATACTAAAA ACCCTGTTGT ATTGGTTTTT TTTNTAATAA GCCTTTTTAC 360
CTCTGTGTAA ANANATATAT ATACCAGGTG TATGATGGTN CATTTTGGGT CTTNANCTTT 420
TTTTTAATGG TTTCTAATNT GTNTGNCCNA ATGTTGGCNT TGNTTTTANA NTTGTNCCGG 480
GGTNNATTTT TANN 494

SEQ ID NO:883
SEQUENCE LENGTH:493
SEQUENCE TYPE:nucleic acid
TOPOLOGY:linear
CLONE:HUMGS01006
SEQUENCE DESCRIPTION:
GATCTTGATG CTGTGGAAGT AGTTTGAGGA ACATCCTATG AGTTTNCTTA GAATGTATAA 60
AGGTTGTAGC CCATCCAACT TCAAAGAAAA AAATGACCAC ATACTTTGCA ATCAGGCTGA 120
AATGTGGCAT GCTTTTCTAA TTCCAACTTT ATAAACTAGC AAAAAAGTGT TTGCTTATTC 180
CACCAGTTCT ACTGTGACAT ACTCGAGTAT AAAGACATGT AGCAATAACG GGGAGTGGGG 240
GGGGAGTCTC ACAGTGCCTT TGGAAGGGCC CGAACTTGCC TTAAATCTTC CTCAACCAAA 300
TAAGTATTTT ATTAGTGCTT GAGAGAATCT GGAATGTAGG NTGGGTTCAA CTGCACAAAN 360
GGAAAANGNT TTTTACCACT NTTTTTATAT AGNTATAAAG TGNAGCAACC GCCTTAGTGC 420
CTGAATATGT AGTCCATGAN TATGCCTTGT NTAATTTCCA GAAATTCCAN ACCTTGTACT 480
GTTTTTTTTC CCN 493

SEQ ID NO:884
SEQUENCE LENGTH:492

SEQUENCE TYPE:nucleic acid
TOPOLOGY:linear
CLONE:HUMGS01007
SEQUENCE DESCRIPTION:

```
GATCAGCACT GCCAGTGGAG ATGGGCGTCA CTACTGCTAC CCTCATTTCA CCTGCGCTGT  60
GGACACTGAG AACATCCGCC GTGTGTTCAA CGACTGCCGT GACATCATTC AGCGCATGCA 120
CCNNNGTCAG TACGAGCTGN NCTAAGAAGG GAACCCCCAA ATTTANTTAA AGCCTTAAGC 180
ACAATTAATT AAAAGTGAAA CGTAATTGTA CAAGCAGTTA ATCACCCACC ATAGGGCATG 240
ATTAACAANG CAACCTTTCC CTTCCCCCGA GTGATTTTGC GAAACCCNCT TTTTCCCTTC 300
AGCTTGCTTA GGATGTTCCA ATTTTAGGAA AGCTTAAGGC GGCCTACAGA AAAGGGANAA 360
ANGGGCCACA AAAGTTTCCT TTTAACTTTT NAGTAAAAAT TAANTTAAAN CAGCAGCAGC 420
AACCANTTTA AATTGGATTT AANGGGTCCN AATTGGAATT NAATTTTTTG GNTTNNNNCG 480
GGNTTTNAAA AN                                                     492
```

SEQ ID NO:885
SEQUENCE LENGTH:490
SEQUENCE TYPE:nucleic acid
TOPOLOGY:linear
CLONE:HUMGS01009
SEQUENCE DESCRIPTION:

```
GATCGAGTTT NATGCATCAC AGTTAACATG TCAGCTGGCC CTCCAGGCCC CCGCCCCCAT  60
CCCGTCCACG TTGCTGTGTC GTGAGGTGCA GCGGGTCACC CTGTGGCCCG TCCTGTGACC 120
CATATTTAGC CGTGTTTGGG ACTCCGTGTC TTCAATGGTT TGTTAGTTGC CATTACAACT 180
TTGTCTGGGT AGAGTTTTTG AGTTTTTNCA GTTCAGTATC CCTCTGTCTA TTCACACTTC 240
GTGTTAGTGG TAACTCAGTT TGTCTTTAAA TAGTTACAGA AGGGATACGT CATTTGTNAA 300
TGCTTTTGTG AAGTGAGTTA AACGAGCTTT CTGTATTTTA ATGCTTTAGT GTTTCAGTTT 360
TATAAGTGAA GATTTTATTT TAAAAACCAG TGGGAAAGAG TGGGGGGTTT CTTTTTATGT 420
CTGGGTCATT CAGGCAGTAC ATCTGNTTTA AAGCTGAATG TAGGACANTT AATGAAATCC 480
ANATCTGAAA                                                        490
```

SEQ ID NO:886
SEQUENCE LENGTH:487
SEQUENCE TYPE:nucleic acid
TOPOLOGY:linear
CLONE:HUMGS01010
SEQUENCE DESCRIPTION:

```
GATCAGCTAT ATACTATTTA TATACAAGTN ATAATACAGA TTTGTAACAT TAGTTTTAAA  60
AAGGGAAAGT TTTGTCCTGT ATATTTNNTT ACCTTTTACA GAATAAAAGA NTTACATATG 120
AAAAACCCTC TAANCCATGG CACTTGATGT GATGTGGCAG GAGGGCAGTG GTGGAGCTGG 180
ACCTGCCTGC TGCAGTCACG TGTAAACAGG ATTATTATTA GTGTTTTATG CATGTAATGG 240
ACTATGCACA CTTTTAATTT TGTCAGATTC ACACATGCCA CTATGAGCTT TCAGACTCCA 300
GCTGTGAAGA GACTCTGTTT GCTTGTGTTT GTTTGTTTGC AGTCTCTCTC TGCCATGGCC 360
TTGGCAGGCT GCTGGAAGGC AGCTTGTGGN NGGCCGTTGG NTCCGNCCAC TCANTNCTTC 420
TGGNGCACTG GTTTNTNCTT TANAGTTANG GTNCCATGNN NCAAGGGGGG TTCCNANGNG 480
GAGGNCN                                                           487
```

SEQ ID NO:887
SEQUENCE LENGTH:485
SEQUENCE TYPE:nucleic acid
TOPOLOGY:linear
CLONE:HUMGS01011
SEQUENCE DESCRIPTION:
GATCGCAAAT ACACTAAATG TGGAGTGTAG GAACCAAAAT GAAACCTGCT GTATGGAAAC 60
TACTTTCACT TATGGTTCAT TGGTTTTTGT ACCAATATTT TTTATGCACT TCAGTGCAAG 120
TNTTGTCAGT TAACCTTACT TTATGAGTAA GCTAAATAAC CCAAATTACA TTTNTTTAAA 180
CCTGTTTTAC TACTATGGCA CTTTGATAAA ATGGTCAGGA ACCAACTTTA CTGGCAAAAG 240
GGTCCATGTA CCACCATGTG CTGGAGCATC TGTTCTACAT GTGGATATCT ATGANTGGTA 300
ATGTTTTCCT TCATGTAAGT GCCTATTCAG AGTTTCAGAA TTTTAAAATG CCAAATATTT 360
TCATGGGTCA TTTGCATGTA GTAAGCCAGA AAATATTCAA NGGGATTTTG GAAAACCAAT 420
TGGTATTTAA CCAGCCTCAA ATTGTGCAAC CATGGTTGTA TAATAANGGA TTTGGAACCC 480
GGAAA 485

SEQ ID NO:888
SEQUENCE LENGTH:485
SEQUENCE TYPE:nucleic acid
TOPOLOGY:linear
CLONE:HUMGS01012
SEQUENCE DESCRIPTION:
GATCTAATGN CATATTCCAA AAGCTCAACA GCAACACCCA GGTAGTTTTG CTGTCAGCCA 60
CAATGCCTTC TGATGTGCTT GAGGTGACCA AGAAGTTCAT GAGGGACCCC ATTCGGATTC 120
TTGTCAAGAA GGAAGAGTTG ACCCTGGAGG GTATCCGCCA GTTCTACATC AACGTGGAAC 180
GAGAGGAGTG GAAGCTGGAC ACACTATGTN ACTTGTATGA AACCCTGACC ATCACCCAGG 240
CAGTCATCTT CATCAACACC CGGAGGNAGG TGGACTGGCT CACCGAGAAG ATGCATGCTC 300
GAGATTTCAC TGTATCCGCC ATGCATGGAG ATATGGACCA AANGGAACGA GACGTGATTN 360
TNTGGNANTT TCGTTCTNGC TCTAGCAGAG TTTTGATTTC CANTGACCTT TTGGNCAGAG 420
GAATTNTTGT TTNNGAAGGT TTTTTTTTAG TCATCANNCT NTTGTCCTTT CAACTATCAG 480
GGGTN 485

SEQ ID NO:889
SEQUENCE LENGTH:492
SEQUENCE TYPE:nucleic acid
TOPOLOGY:linear
CLONE:HUMGS01013
SEQUENCE DESCRIPTION:
GATCGCAACG CCAAAGGTGA AGAACCANCT CAAGGAGACC ACTGAGGCAG CCTGCAGATA 60
CGGAGCCTTT GGCCTGCCCA TNACCGTGGC CCATGTGGAT GGCCAAACCC ACATGTAATT 120
TGGNTCTAAC CGGATGGAGC TGCTGGCGCA CCTNCTGGNA GANAAGTGGA TGGNCCCTAT 180
ACCTCCAGCC GTGAATGCNA GATTTTAAGA TTGCCCGGAG GAAGCAAACT TTTCGTATAA 240
AAAAAGCAGG CCATCTGCTT AACCNTTGGN TCCACCATAA GGCACTGGGA CTNGGATTTT 300
TNTATCTGAT AGAGGTATTT NTTGTGGCCC TGGGAGCTGT CTGNTTTTCC CCTACCCCCA 360

AGGNTGCCAG GAAGACGTCC ACCATTAGCC ATGTGGNAAC CTTTACTTCT ATGCTTACAA 420
GTGCCTTTNA GNGAGCCCCA ATTCTGGTTT TNCCACAAAA TAAACCTAAT GCNNTCAGGG 480
AAAACNNTTA AA 492

SEQ ID NO:890
SEQUENCE LENGTH:478
SEQUENCE TYPE:nucleic acid
TOPOLOGY:linear
CLONE:HUMGS01015
SEQUENCE DESCRIPTION:
GATCTTGCTT CCAGGCAGCA GCTTGAATTC CCGAATCTNC CTGCAAGCNG CATACAAATG 60
CAGCGTGAGA ATCCATACAC GTAATCCATA TTCACCTTCC CATCCATCCC GCAGAAGAGG 120
CATGGTGACA CCCAGGCTAC TGTCCATGCT TGAGAGGACG TATTTGAAGG TTCTGTTACT 180
ACAAGTTGGG AATATTCACG GGCCATGCCT GAATACCCGG NCTGTANCTC ACACNGTGGT 240
CTGTGTAAGG GGNTACCCTN GGGGCGGCCT GGTTTAATCN TGATTAATAT CTGAAAGCNT 300
GGGTTNNNTG GGGAATGTNA GGGTTTTCCT AATGCCATTA AATTTTTTTT TAGGCNGTAA 360
AAATTAAAAT NCATTTTNTT ATCCAGCAGG CCTCTTTTAT ACCTTTATNG GGGAATCTNC 420
CATACTTACT TTCCGGCCAN NTTTTCAATA ATNAAATTTA TTTTGGAAAN TTTTTAAA 478

SEQ ID NO:891
SEQUENCE LENGTH:474
SEQUENCE TYPE:nucleic acid
TOPOLOGY:linear
CLONE:HUMGS01016
SEQUENCE DESCRIPTION:
GATCTTACAC TCTGCTTTTG TCCAAATAAA ATGCAATAGT ATCAATATCA ATTTCAGAAA 60
AATGGACTGA ATATGCTTTT TTGGTGATGA AATCTCATGT ACGATATTTA TAGTGATGTG 120
CTTTTATTTN CTCATGAGAT ACTAAATATT AATTGTGTTG TACATTTGTN CTTAGCATAT 180
ATTAAAGTTT TGAACCAAAT GTGTTAAAGC TTACGCTTTG CCATGTAAAT TTCCCAGAAG 240
TTGTTGAGCT CAAATGTATC CTACATCCAG CTGTAGAAAT TTGTCAGAAA TTGTTTAAAT 300
TTTGTATATA ATTGTACTGT TTAATTCTAG CCATTGCGCT GAACAGTATT TGAGTTACCA 360
TATAATATGG CTTTACACAA NGGNAATGTG TGGCTTTTGT TTTGGTATTT TTTCCAGTAT 420
AGGAAGTTCC CTGTGGCCTT ATTTAAAATA AAGGTTATTA GGTAAAACTG GAAA 474

SEQ ID NO:892
SEQUENCE LENGTH:473
SEQUENCE TYPE:nucleic acid
TOPOLOGY:linear
CLONE:HUMGS01017
SEQUENCE DESCRIPTION:
GATCACAGCT CACTGCAGGT TCAGCCTTCT GAGTTCAAGC AATCCTTCTG TCTCAGTCTC 60
CTCAGTAGCT GGGGCTTTAG GTGGGCACTG CCACACCGTA CTAATTTTNG TATTTTTTGT 120
AGAGACGAGG TCCCACCATG TTGCCCAGGC TGGTGTCAAA CTCCTGGGCT CAGTCAGTCC 180
CCCCATCTCA CCCTCCCCAA GTGCTGGAAT TACAGGCGTG ANTTACTGTG CCCAGCCTTA 240
CGGACATCCT TTTGAATTAT CTTTTTCACT CATAGAATAT GAATACATTT ATTTAGACTT 300

TTTCTAGAAC TTTCCTGTTT TCATGTCTTT GNTTCACCTG GAATTGGGTT TAACACCCTT 360
TTATAAAGTT TGTGGTTTGN AAAATTTCCA TTGGGGCCAT CAATACGGAA ATATATTTGG 420
TAANATTNGG GGGTTCNATT TTTTAATTAA AATGGCAAAT GANNGGCAGG AAA 473

SEQ ID NO:893
SEQUENCE LENGTH:473
SEQUENCE TYPE:nucleic acid
TOPOLOGY:linear
CLONE:HUMGS01018
SEQUENCE DESCRIPTION:
GATCGGGTTG AGGATTGGGG CTAGCTCGAT NACANTAAGG CCCCAACATC GNGGGACCTG 60
CTGTGGCGCG GATTCTTAGG AACGCTGTTC TAGCCGGCCC CCTCTCCAGG GGTCGCCGTG 120
GCCGGCATTA TTTCCTAGTT CTTCTTGTAA CCCTGAGGTG CCAGCGCGGG GAGTGAGGAG 180
GGGTCAGGGG GCTAAGGATG CAACCTCTGA CGTTCTGCGC CTTCCTAGGA GAGTCTTACA 240
TGTNTTGAGA TTTCACAAGC AATGCGAGTT GTAAAATACC AGCTCTACAN GAAGCTAGGC 300
TCTGTGACGG CATAGTTTTC AGTAGCTNTA TCACAATATT CACAATGGAG AATTATATGA 360
CATGGTAAGC AGAAATAGGC CCCTTTTAAT GNGNTGCTTC TATTTTACCT CANATTGGTG 420
GNTNTAGGNT AATCANTAAA AATCNATCCA NNGCNTTTCA CAACACTNNN AAA 473

SEQ ID NO:894
SEQUENCE LENGTH:468
SEQUENCE TYPE:nucleic acid
TOPOLOGY:linear
CLONE:HUMGS01019
SEQUENCE DESCRIPTION:
GATCAGGGGG CCAGGCCAGC AGCTCGGGGG CCACAAGGAG ATGGATAATG TGCCTGTTTT 60
TTAACACAAC AAAAAAGCCT ACCTCCAAAA TCCCCTTTTT GTTCTTCCTG GACCTGGGCA 120
TTCAGCCTCC TGCTCTTAAC TGAATTGGGA GCCTCTGCCA CCTGCCCCGT GTATCCTGGC 180
TCTCAGCTCA TGGGGAAGCC ACATAGACAT CCCTTTCTTC CCTTGCACGC TCGCTAGCAG 240
CTGGTAAGGT CTTCACACCC TGATTCCTCA AGTTTTCTGC TTAGTGGCAC TGACATTAAG 300
TAGTGGGGGG ACAGTCCATG CCAGGACACC CTGGAGTAGC CTTCCCCCTT GGCCGTGGGG 360
CAGGNCCTAA CTCACTGTCG CTTTGGAGTT GAGGGTGTCT TTTCTTNTTC TTTCTTTAGT 420
TCCTGTATTC TAAACATTAG TAAAAATAAA TGTTTTTTAC ACAGGAAA 468

SEQ ID NO:895
SEQUENCE LENGTH:462
SEQUENCE TYPE:nucleic acid
TOPOLOGY:linear
CLONE:HUMGS01020
SEQUENCE DESCRIPTION:
GATCCTGCAG GACTACAAAT CCCTCCAGGA TATCATTGCC ATCCTGGGTA TGGATGAACT 60
TTCTGAGGAA GACAAGTTGA CCGTGTCCCG TGCACGGAAA ATACAGCGTT TCTTGTCTCA 120
GCCATTCCAG GTTGCTGAGG TCTTCACAGG TCATATGGGG AAGCTGGTAC CCCTGAAGGA 180
GACCATCAAA GGATTCCAGC AGATTTTGGC AGGTGAATAT GACCATCTCC CAGAACAGGC 240
CTTCTATATG GTGGGACCCA TTGAAGAAGC TGTGGCAAAA GCTGATAAGC TGGCTGAAGA 300

GCATTCATCG TGAGGGGTCT TTGTCCTCTG TACTGTCTCT CTCCTTGCCC CTAACCCAAA 360
AAGCTTCATT TTTCTGTGTA GGCTGCACAA GAGCCTTGAT TTGAAGATAT ATTCTTTCTG 420
ACCAGTATTT AAGGGTTTCC AATAAAATGT ACACCNCTCA AA 462

SEQ ID NO:896
SEQUENCE LENGTH:462
SEQUENCE TYPE:nucleic acid
TOPOLOGY:linear
CLONE:HUMGS01021
SEQUENCE DESCRIPTION:
GATCTAAATC CTCATTTATC TCTNCTATGT CTAGTATTTT ACTGTCACTG GAGGCTCTGT 60
GGGCTGTCAT AGTTAATTGA CCATAATTAG CAATATACTT TTAAAGTGGG AAAGCTGAAT 120
GACACTNTTT AAGACAATGA ACATTATCAA AACAAAATGT ATAATTNCTT AATTTGAATA 180
ATAAATTAGG CGTTTAAATG CTATTTGTAG TCTTGATATA CAGAAATAAA ATAATTAGGG 240
TTGGTCTTTT TTATTTTAGG TTGTTTTATG TTGAATGTTC TATATCTTAT TAGTTAATTN 300
GTATATTTNA TTAGTATTTN GGGAAATAGC ATATCTGAGA CTGAAGGGGA AATTGGCCAA 360
TTCACTTATT TGTGGTTTTT TTCCTCAGCT ATTCTGAGCT TATTTATTAA TTGNATGGCC 420
TAATGGCTAA CCATTTACAT TAAAATGGTT TTTTNCCCCA AA 462

SEQ ID NO:897
SEQUENCE LENGTH:459
SEQUENCE TYPE:nucleic acid
TOPOLOGY:linear
CLONE:HUMGS01023
SEQUENCE DESCRIPTION:
GATCTNCTCT TTCCTCTCCT TTTCCTCATT TATTCCTAAA GGAATCTGAC CATTAAAACG 60
NCTCTACGGC CCAAAAAAAG ACAAAAATAA AAATTCCTTT TTATTCCTGT CAACTGGATG 120
GAAACACAAA TTTCATGGAG CTGTGTACCA TCGAAGAAAC CTGGTGTCTG GCATGAAATT 180
ACTGTAAAGA ACTTCCTGTA AAACACGTTC TTTAACAAAC TGAAATGAAA AGCATTGGAG 240
CGTCTGANTG AAAGACGTGA CCTCCTGCTG GGACTCTGAT GGTCTTCAGC ATTCACCTTC 300
GTGTGTCTTC AGTGTCTCAT TGTCATCCCT NGCTTCTGGN TTGGNCCTTA GGAGTNGTTT 360
GGGATATAAC CTNAAATTGT NGGATGGGTA AANGGGAAAT TTNNATGNNG TTTTTTTGGT 420
TTTTTAAAAT NATTTTNAAA NCGGGGTCAN TTTTTTAAA 459

SEQ ID NO:898
SEQUENCE LENGTH:457
SEQUENCE TYPE:nucleic acid
TOPOLOGY:linear
CLONE:HUMGS01024
SEQUENCE DESCRIPTION:
GATCCACCTG AGCGACCTCC GGGAGTACAG GCGCTTTGAG AAGGAGAAGC TCAAGTCCCA 60
GTGGAACAAT GATAATCCCC TTTTCAAGAG CGCCACCACG ACGGTCATGA ACCCCAAGTT 120
TGCTGAGAGT TAGGAGCACT TGGTGAAGAC AAGGCCGTCA GGACCCACCA TGTCTGCCCC 180
ATCACGCGGC CGAGACATGG CTTGCCACAG CTCTTGAGGA TGTCACCAAT TAACCAGAAA 240
TCCAGTTATT TTCCACCCTC AAAATGACAG CCATGGCCGG CCGGGTGCTT CTGGGGGCTC 300

GTCGGGGGGA CAGCTNCACT CTGACTGGCA CAGTCTTTGC ATGGGAGACT TGAGGAGGGG 360
AGGGGNTTNA GGTTGGTGAG GTTAAGGTGC GTGTTTCCTG GTGCAAGTCA AGACCATCAG 420
TCTTATTAAA AGGTGGGTGC CAATTTTTTT TACNAAA 457

SEQ ID NO:899
SEQUENCE LENGTH:457
SEQUENCE TYPE:nucleic acid
TOPOLOGY:linear
CLONE:HUMGS01025
SEQUENCE DESCRIPTION:
GATCCAGTAT CTTCCTCGGC TTTTTAGGGA GCAGGAAAAA TGCGTCTNAN AGCAACTTTT 60
TTTAAAAACC TGCCCTGTTG TATATAACTG TGTCTGTTTC ACCGNGTGNC CTCCCAAGGG 120
GGTGGGAACT TGATATAAAC GTTTAAAGGG GCCACGATTT GCCCGAGGGT TACTCCTTTG 180
CTCTCACCTT GTATGGATGA GGAGATGAAG CCATTTCTTA TCCTGTAGAT GTGAAGCACT 240
TTCAGTTTTC AGCGATGTTG GAATGTAGCA TCAGAAGCTC GTTCCTTCAC ACTCAGTGGC 300
GTCTGTGCTT GTCCACATGC GGTGGGCGTC TTGGGACCTT GAATGCCTGC CCTGGTTGTG 360
TGGACTCCTT TAATGCCAAT NATTTCTTCA NTTTCTCTTG GGACACCNAG GGNTGCCNGT 420
TNGACAAAGT TTTGGNGAAC NTCCTAATTT AAAATGN 457

SEQ ID NO:900
SEQUENCE LENGTH:454
SEQUENCE TYPE:nucleic acid
TOPOLOGY:linear
CLONE:HUMGS01026
SEQUENCE DESCRIPTION:
GATCCGANGA CCACATCAAT GGGCGCGTGC TCTACTATGC CACCTGCAAG TAATGCTACA 60
GCTTCCAGCC CGTTGCCCCA CTCATCTGCC GCCTTTGCTT TTGGTTGGGG GCAGATTGGG 120
TTGGAATGCT TTCCATCTCC AGGAGACTTT CATGTAGCCT AAAGTACAGC CTGGACCACC 180
CCTGGTGTGT AGCTAGTAAG ATTACCCTGA GCTGCAGCTG AGCCTGAGCC AATGGGACAG 240
TTACACTTGA CAGACAAAGA TGGTGGAGAT TGGCATGCCA TTGAAACTAA GAGCTCTCAA 300
GTCAAGGAAG CTGGGCTGGG CAGTATCCCC CGCCTTTAGT TCTCCACTGG GGAGGAATCC 360
TGGACCAAGC ACAAAAACTT AACAAAAGTG ATGTAAAANT GAAAAGCCAN ATAAAAATCT 420
TTGGAAAAGA GCCTTGGAGG TTCAACGGGG GAAA 454

SEQ ID NO:901
SEQUENCE LENGTH:453
SEQUENCE TYPE:nucleic acid
TOPOLOGY:linear
CLONE:HUMGS01027
SEQUENCE DESCRIPTION:
GATCTTNCTT NTTAAGTGGA TAAGACAGTC CCACAGTCCA GCCTAACTAT GGGACAGCTT 60
TACGAGAAGG AAAAAGATGA AGATGGATTC TNATATGTGG CCTACAGCGG AGAGAACACT 120
TTTNNAANCT GAGGGCCATT GCTGGGCTAG GTGCACCGTA ACTGCTTGTG TATCTTGTAA 180
ATAGCCAGCC ATTTNCAGTT ATTATACCAG AACCTCTTCA CATAGACCTA TTAGTGCATT 240
TGTAACTGGN TTTATTTCTT AATATATTGG AAGGTTTGTT TCCTTAGNCT AGTAAATTAT 300

CATACAGNGT TTTATTTTGA GGTTTTTCTT NNNTGTGCAT TNTCCTCATG GCCTGTAAAC 360
CNCCAGGAAA CCTTTTCCTT CTNGGAAATC ATATTTGAAA TGATAATTCN TATATCCGAN 420
GTGAGGNTAG GNNCCGGGTC CTCCCAATAA ANN 453

SEQ ID NO:902
SEQUENCE LENGTH:452
SEQUENCE TYPE:nucleic acid
TOPOLOGY:linear
CLONE:HUMGS01028
SEQUENCE DESCRIPTION:
GATCTTAAGT GAAGAAGGAA GACTTGGCCT TTTGTATTGC TTGAATATTA ACTGTCTTGG 60
AAGGAAGTTA TGCTACAAGA AGTCATACTT TCATAAAATT ATTTGCATCT GTGTCAAATG 120
CAGTTTAGTC AGAACGTAAG ACATAATAGG TGTGGACATG AACTCTGGAG TGTGAAATAA 180
AATCCACAGT TACTTAAGCA GTCTGTTTTG ATGGAAAGTA TCTTGGGATA ATACTTTCCT 240
CTGTGGGATT TTGTTCATTT TAGATGGTGC ANGGNAGTAT CAGTCTTTAA TTTTTTTGTT 300
GTTGTTTTTA TCANTCATTT GCTCTGATGG TATGATGCAT GGGCTTCAGG ACTCCAGCTG 360
CACCACTGTA TAAAACTCAG TTCAGGTTTN CTAGCGGTCN TTGGNTAATT TTCGGGGCNT 420
ANCCCGATNC CAATTNTTTT TAATGTGNTA AA 452

SEQ ID NO:903
SEQUENCE LENGTH:448
SEQUENCE TYPE:nucleic acid
TOPOLOGY:linear
CLONE:HUMGS01029
SEQUENCE DESCRIPTION:
GATCAGGGAC CCTCCCNGCT TTCCTGGGCC TCTNAGTTGA ACAAAGCAGC AAAACAAAGG 60
CAGTTTTATA TGAAAGATTA GAAGCCTGGA ATAATCAGGC TTTTNAAATG ATGTAATTCC 120
CACTGTAATA GCATAGGGAT TTTGGAAGCA GCTGCTGGTG GCTTGGGACA TCAGTGGGGC 180
CAAGGGTTCT CTGTCCCNGG TTCAACTGTG ATTTGGCTTT CCCGTGTCTT TCCNGGTGAT 240
GCCTTGTTTG GGGTTCTGTG GGTTTGGGTG GGAAGGAGGG CCATCTGCCT GANTGTAACC 300
NGCTAGCTCT CCGANGCCCT ACGGGCCTGN CTNGTGTGAG CGTGTGGACA GTGGTGGCCG 360
GCGCTGTGCC TNCTCGTGTT GCCTACANTG TNCNTGGCTG TTGAGGCGCT GCTTCANGCC 420
TGCAACNCGT CCNTNGTCTC ANTACAAA 448

SEQ ID NO:904
SEQUENCE LENGTH:461
SEQUENCE TYPE:nucleic acid
TOPOLOGY:linear
CLONE:HUMGS01030
SEQUENCE DESCRIPTION:
GATCGGTTTA CAGATGAGGA AGTGGATGAG CTGTACAGAG AAGCACCTAT TGATAAAAAG 60
GGGAATTTCA ATTACATCGA GTTCACACGC ATCCTGAAAC ATGGAGCCAA AGACAAAGAT 120
GACTGAAATA ACTTCAAATT CCAGCCAAAC GTTCCTTGTT GCCACTTTGG GTATTCTGAG 180
ATTTTCTCTT GCATGCCCTT AGCTTTACAG CTTTTGCATT TCCTGTTGTA TTTATTCTCA 240
GCCATTTTGG GCATATGTAT CTTTATAATC AGACTGGAAA CGGGACTTTC TATTAATATC 300

ATTTTTCAGA ATAAAAAATA GGGTAATTTA ACCTACCAGC CCTTCTCCCC CAATAACTGT 360
GGGCCTATAC AGNGTCAATA TATTTTTTNC AGNGAAAGGT TTATTCGGCT CGATTTTTTT 420
CTGGAANTCC ATAATTTAAC CTTTTATGGG TTAAANTTAA A 461

SEQ ID NO:905
SEQUENCE LENGTH:453
SEQUENCE TYPE:nucleic acid
TOPOLOGY:linear
CLONE:HUMGS01032
SEQUENCE DESCRIPTION:
GATCAGGGAC CATGAAAAGA AACTTGTGCT TCACCGAAGA AAAATATCTA AACATCGAAA 60
AACTTAAATA TTATGGAAAA AAAACATTGC AAAATATAAA ATAAATAAAA AAAGGAAAGG 120
AAACTTTGAA CCTTATGTAC CGAGCAAATG CCAGGTCTAG CAAACATAAT GCTAGTCCTA 180
GATTACTTAT TGATTTAAAA NCAAAAAAAC ACANAAAAAT AGTAAAATAT AAANCCAAAT 240
TAATGTTTTA TAGACCCTGG GAAAAAGAAT TTTCAGCANN GTACAAAAAT TTANCGCATT 300
CCTTTCTTTA ATTTTGTANT TCTTTACTGT GGGAATAGCT CAGAATGTCA GTTCTGTTTT 360
ANGTAACAGN NTTGGATAAC TGAGCAGGGN ANCGNAATTT TGGNTTTNTA AAATTCCTTG 420
CTTTNANTAN ANNTTCCCTT NNCCCGGTGG AAA 453

SEQ ID NO:906
SEQUENCE LENGTH:445
SEQUENCE TYPE:nucleic acid
TOPOLOGY:linear
CLONE:HUMGS01033
SEQUENCE DESCRIPTION:
GATCCGATGG AGAAGGGGGG ACCCAGGCCA GCAGGAGACA GGACCCCCGA AGCTGAGGCC 60
TTGGGATGGA GCAGAAGCCG GAGTGGCGGG GCACGCTGCC GNCTTCCCCA TCACGGAGGG 120
TCCAGACTGT CCACTCGGGG GTGGAGTGAG ACTGACTGCA AGCCCCACCC TCCTTGAGAC 180
TGGAGCTGGC GTNTGCATAC GAGAGACTTG GTTNAACTTG GTTGGTCCTT GTNTGCACCC 240
TCGACAAGAC CACACTTTGG GACTTGGGAG CTGGGNCTGA AGTTGNTCTG TACCCNTGAA 300
CTCCCAGTTT GCGAATTATA GAGACAATCT ATTTTGTTAC TTGCACTTGT TATTCGACCA 360
CTGAGAGCGA GATNGGGAAG CATAGATATC TATATTTTTA ATTTCNCTAT NGAGGGCCTN 420
GTAAATAAAT TTCTAAAAGC CTAAA 445

SEQ ID NO:907
SEQUENCE LENGTH:444
SEQUENCE TYPE:nucleic acid
TOPOLOGY:linear
CLONE:HUMGS01034
SEQUENCE DESCRIPTION:
GATCTCAATG AAATTAAATT AATAACTATA TCTAGAAAAA CTCCCAATAT GTGAATATTA 60
AGCAACATAC TTATAAAAAA TTCATAGTTC ATGGATGAAA TCAAAAGAGA ATTCAAAATA 120
TTTCAAAATA AATTATGATG ATTATATAAN ATGTAGAAAT GTGTGGGATG CCACTACACC 180
AGTTCTTAGA GTGAAATGCA CAGCTTTCAA GGCTTCTGTT AAAAGAGTTG GGAATTACAA 240
AACAAGGAGC AGCGACTGCC AATGGGTGTG GAGTCTTTTT TGGGGTGATG ATGAAAATGT 300

TCAAAAATTG ATTTTATTGA TGGTTACACA CGTCTATAAA TATATTTNAN CGGNAGAATT 360
ATATGGATAT ATATGTGATA TATGGAGGTA TATCCTCTGT AAAATTCCTA NGGGTTTAAN 420
GGAGGATTGG GTATCCACAC CAAA 444

SEQ ID NO:908
SEQUENCE LENGTH:440
SEQUENCE TYPE:nucleic acid
TOPOLOGY:linear
CLONE:HUMGS01035
SEQUENCE DESCRIPTION:
GATCCCAGTG TTGCCTTAAC AGGGTGTCTG TCGTGCCGCA GTAGAGCACT GCTGCTTCCT 60
CCAACCCCAA AATTTATGTT CCTAAGTAAG TCAGGTCCCT AAGCCCCGTC CCAAGAAGTG 120
ACACAAGTGG CCAACATCCA CACTGTAGGC TTGCAGGCTA CCCGCCCTGA GATTTGGTAA 180
AGAACACTGC CTTGTTCCCC ATCAGTAAAC AAGGTTACCT ACCTCAGGAG GCTGCTTGTG 240
AGAGAGCAAA TGCAGTATCT TCAGANTGAT TTATTTTTTT ANTTAATTGT AAAGACTTGT 300
GCCATTGGCT GCTCTTTCTA GTCCCCTAAN TTTCTGTTCT AGTTTTAANT TTCTCTAGAN 360
CTTGCAATNG TTGGGGGGTT TTTATANTGG TGTTTTTNCA ATGNTTTGTT TCNTTANNNT 420
AAANCCTTAA AAGTTCCAAA 440

SEQ ID NO:909
SEQUENCE LENGTH:439
SEQUENCE TYPE:nucleic acid
TOPOLOGY:linear
CLONE:HUMGS01036
SEQUENCE DESCRIPTION:
GATCGGCGTG GGGGTGCTGC TCATCTTCCT TGTCAAGTAC GACCTTAACA ACCNGGACAA 60
GCACGCCAAG CTGGACTTCC TCAACAACCT GGCCACGGGN CTGGTGTTCA TCATCGTGGT 120
AGTCAACATC TTCATCACGG CCTTCGGGGT CCAGAAGCCC TTGATGGACA TGGCACCCCA 180
GCAGTAGGAC ACCCAGGACC NTGGATGCTG CCTGCCNNTG CAACTCAGCT GCCCGACCCC 240
AGGAGTCGCC ATACCTGTNA GGTGTCCACC TCCCTGCACA TGGCACTACC CAGANTGCCA 300
GAGCCCAGGC TGGNCTCATC TGCACCATGT CCCCGGACCA GCCCTTGCTC TGANTGCGGG 360
CCAAGNACCA NGTAGGAGGN CACTNTTGTT TNTNAGCAGN TTTTTCCAGG NGGGNAGTTN 420
NNTTCTGGGA AATTGGGGN 439

SEQ ID NO:910
SEQUENCE LENGTH:435
SEQUENCE TYPE:nucleic acid
TOPOLOGY:linear
CLONE:HUMGS01037
SEQUENCE DESCRIPTION:
GATCCAAGGA CCCACACTTT GTCACCTCAT ATCTCTCTCT CTCTCCCACT TTCGCTTTCT 60
CTGTCTCTTC TTAGTTCTGC TTTCCTCCAC TGTGCCTTCG CTCTCAGAGA GAGCTCTCCC 120
CTGGCAGTGA CAAGATGGCT GCAGCAGCTC CAGCAACCTC AGAGGACTCC CCCATCCAGG 180
GTCCTTGTGA GCTCCTCATC TGTAGGATGT GCAGTAAACA CTCACGTGTC CTTTCCTGAG 240
GAGCCCAGTG GCTGGGGTGG GGGCTGAGGG GCAGCCCCTA TGCCCTCACA GTGCAGCAAC 300

CTTGGTTAGC TCACCCATCA GGGCAGACTT GGGCAGAAAT CATGTCTTGG CATATTGTTT 360
TGTAATCTGC TTTTTAAATT GTCACTATAT ATTATGAGCA TTTCCCTATA ATATACAATA 420
TTCTTCCACA TTAAA 435

SEQ ID NO:911
SEQUENCE LENGTH:433
SEQUENCE TYPE:nucleic acid
TOPOLOGY:linear
CLONE:HUMGS01038
SEQUENCE DESCRIPTION:
GATCATATGA AAAAGTAACA AGCTGTTCCN TGTTTCTGAT ACATAAAATN ATTTTAAGCA 60
TTTTATCAAT CATTAAAATT TACTGCCAGT TGTGAGTGGC TTTTTAATTA ACTTGTCTTT 120
CATTGCACTT CACTCTGCCT GTTTTCAAGG GGAGTAAGAT TGGTAACATT TGGGGAGACT 180
GTATCTGTCT ACTTAGCGTG GCTGTTTTGA GGGACTGTCC CATCAGTGAA CAAACTGCAT 240
GGCCTTGGAG AGAGACTCTG GGCTCTTGGC TCAGATGTGT TCATCAAATA CTCCTTTCAG 300
AGCTGTTGTG GGTGTAAGTG ACATGATGTG GCCAAAAAAT CCAAACTGTG CAGTTGCGNT 360
TGTGACAACC ATGCAATGTG NCTGTAAAAA TTNANTTNCA GTTTAAATTN ANATNNTTTA 420
TATTNNGTGG AAA 433

SEQ ID NO:912
SEQUENCE LENGTH:431
SEQUENCE TYPE:nucleic acid
TOPOLOGY:linear
CLONE:HUMGS01039
SEQUENCE DESCRIPTION:
GATCTTTCTA ATTCGAAAGC TGTGTTCTTT TTGAATACCG TGCATGGGGG TTAAGCTGAT 60
GTTAAAACAG TTTGCAATAA AAAAAAATGA ATNAGCTTAA GTCATTTAAT CATTTCAAGT 120
GCATTCTGCA TCCTTTAAAA ATAAGTTTAA GAAATTTAAG AGAATTGTGT TTTCATTAAG 180
TTTTGCATAT CTTTTGTTAT GCCATGTAAA TNCCCTTTTN CGTATGATTA AAGGAAGGTT 240
ATGATAAAAT GATTAGTTCA TTTACATTCA CTTGTAGCAA TTACATGAGA ATTTGAATTT 300
NGTCGTGTTT GGGTTTGTNC ATTCCTGTGA ATGATGGTNC AGTTAGGTGA GATTTNCTGT 360
TATGGNACCC CAACTCACCA TTTGGNCCTC TTTAATCTTT GGGGGGTTCC AATAAAAATT 420
GGTCACTNAA A 431

SEQ ID NO:913
SEQUENCE LENGTH:431
SEQUENCE TYPE:nucleic acid
TOPOLOGY:linear
CLONE:HUMGS01040
SEQUENCE DESCRIPTION:
GATCCAAANT TTAAGGGATA TTGCCTATTT TGGAAGAATA AAACTAAAAT GTTAAAACTG 60
TTGAATTACA GAGAACAGAT GTACTCTGAG ACATAATTTT AAACAAATAT TTAAAATANG 120
GCAGGTTAAC ATTTGCGTTT AGGCACAATA AATCTGTATT AAAGGGAAGC ACATCAAGGA 180
AATATATACA TGTTGANTAA TGTAACTNAA AAATATTTTT TAAAANCCAC TTAAAAATGA 240
NAAATAATTT GATGGGTACT TTAAGCATTG TAGATAGAAA TTAATGTATA ATAGTGTCCT 300

CCCNGTCTTT GTATGAAAAN TTAAAANCTC TCTAGTCCTT TAATGAGCAT GANTTTTATA 360
CTTCTACATT TTGTTGCCTA GGNAAAATTN TCCTCNNGTA CCTTTGAGGT NATTCCGGAT 420
TTTATGGTTT N 431

SEQ ID NO:914
SEQUENCE LENGTH:430
SEQUENCE TYPE:nucleic acid
TOPOLOGY:linear
CLONE:HUMGS01041
SEQUENCE DESCRIPTION:
GATCTATAGA GAGAGATATA TACACTTTTG ATTGTTTTCT AGATGTCTAC CAATAAATGC 60
AATTTGTGAC CTGTATTAAT GATTTAAAGT GGGGAAACTA GATTAAAATA TTTGTTTTTT 120
AACTAGTTTA TTAGTTTCTN TGGAATCTGC CTGTGTCCCT GGGTTTGGGT TTTGCTCTTG 180
GCAGCAGCAG GTGCCTCTTG GGTGCTCCTC CTGCTCCTGC CTGCAGCCCT AAGAGCAGGT 240
GGGTGCCGAG TGTCTGGCAC AGCTTGGATG CCGCCCACTG AAGACAGCAG AGGGGGGTTG 300
TNTTGAAGCT CCCGNGACAC AGTCAAGCAT CTTCTGAGNC TTCGATGTCT TCGGNAAGTA 360
AAAATGGGGN TTTAGTAAAA CCCTGCCCCN GTTTNTCACA GGGGGTTNTT TTGCAAGNNA 420
AANTGATAAA 430

SEQ ID NO:915
SEQUENCE LENGTH:428
SEQUENCE TYPE:nucleic acid
TOPOLOGY:linear
CLONE:HUMGS01042
SEQUENCE DESCRIPTION:
GATCAGATTC TAATTTGACA GGCAACCAGT CAATGAAACA GACACACCTG CACAGTTGGA 60
AATGGAGGAT GAAGATACAA TTGATGTGTT TCAGCANCAG ACAAGAAGTG TCTACTGNAA 120
AAGGANCCTG CTTCTTTACT CTAGAACTTT GTTCTTATAG ACCAAGATTA CATTCTCAAT 180
TAGAAAACTG CAATTTGGTT CCACCACATC ATGACTATTA CTATAGTATA GTTTTCTCTA 240
TTCTTTTATT TTTCCCTTTA CCCATTCCCT TATTTGTACA TAAAATAATG GGTGTATGTT 300
CACAAGCATT TTGCTGGTTT TNAAATATTA ANTGGCCAAT GACATCCACT TGATGTCANT 360
CAANACNATA TCTGTGGGGG NAAANTACCG NTTCTTNGAA ATTTNCCTCC NNTTTTTCCA 420
TNAGTGGN 428

SEQ ID NO:916
SEQUENCE LENGTH:428
SEQUENCE TYPE:nucleic acid
TOPOLOGY:linear
CLONE:HUMGS01043
SEQUENCE DESCRIPTION:
GATCGCCAGG TTCTACAAGC TGCACGAGCG GAGGTNTGAG CCCATTGCCA TGACAGTNCC 60
TCGAAAGTCG GACCTGTTCC AGGAGGACCT GTACCCACCC ACCGCAGGGC CCGACCNTNC 120
CCTCACGGCT GAGGAGTGGC TGGGGGGTCG GGATGCTGGG CCCCTCCTCA TCTCCCTCAA 180
GGATGGCTAC GTACCCCCAA AGAGCCGGGA GCTGAGGGTC AACCGGGGCC TGGACACCGG 240
GCGCAGGAGG GCAGCACCAG AGGCCANTNG CACTCCCAGC TCGGATGCCG TGTNTCGGCT 300

NNGAGGAGGA GATGCGGGAA GTTCCAGGCC ACGGTGCAGG ANCTCCAGAA GNNGTTNGGA 360
CAAGGNTTGG AGGAGACAAG TCCAAGNNCA AGTAGNAGCC CCTGAAGGNN TTTNCATCAN 420
GGTTTCAN 428

SEQ ID NO:917
SEQUENCE LENGTH:424
SEQUENCE TYPE:nucleic acid
TOPOLOGY:linear
CLONE:HUMGS01044
SEQUENCE DESCRIPTION:
GATCCAATAT TCAATTCATT TGTGTACTCC CACATGCAAA ATGCTAAATT ACAATGCAGA 60
CATTAAGAAA AAGTATTGAC TGGAGGGGTT GAATTCCTTG AGAATTTATT TTATAGTCTA 120
AATCACAAAT ACTTTACTCA ATTTAGTTTT TAAAATAGTA AACTGAATAT TTTTGTTGTA 180
AGCCTATCAG AGTCAATCCT TCGTTTGGAA TTGTTTTCCT GTTTTNCCTT ACTATAAATC 240
ATTTAAAAAC TGAATTCATT TTCTTAGATG GCATAAGTCT GTCTCTTGAG AAATAAGTAA 300
AATACTCCTA TTTTCAGTAT CTGTAGCACC TGAAATAGGT CTTTGTATAG CCAGAANCAA 360
GTTATGNTTG AAGTTAGCTT TTCTTTGTCA CCAGTTTTGG NCAATAAAAA TCTGAANGTT 420
TAAA 424

SEQ ID NO:918
SEQUENCE LENGTH:422
SEQUENCE TYPE:nucleic acid
TOPOLOGY:linear
CLONE:HUMGS01045
SEQUENCE DESCRIPTION:
GATCAACATA ATGGACCACT CCTGAATGAG ACTTAATTTT GTCTTTCAAA TTTACTGTCT 60
TAAATCAGTT TATTAAATCT GAATTTTAAA ACATGCTGTT TATGACACAA TGACACATTT 120
GTNGCACCAA TTAAGTGTTG AAAAATATCT TTGCATCATA GAACAGAAAT ATATAAAANT 180
ATATGTNGAA TGTTAACAGG TATTTTCACA GGTTTGTTTC TTGATAGTTA CTCAGACACT 240
AGGGAAAGGT AAATACANGT GANCAAAATA NGCAACTAAA TGAGNCCTAA TAATTGGCCT 300
TCGATTTTAN ATATTNGTTC TTATAAACCT TGTCAATAAA AATAAATCTA AATCAAAAAA 360
AANTTGGTTC CACCTNTGCA GGTTTTTATA ANTGGTGCCA ATTAAAGGTT TTTGTTTTTA 420
AA 422

SEQ ID NO:919
SEQUENCE LENGTH:422
SEQUENCE TYPE:nucleic acid
TOPOLOGY:linear
CLONE:HUMGS01046
SEQUENCE DESCRIPTION:
GATCCTTACT AAGTNTTTCA TGGGAGACTT CCTTCATCAC ATCTTATGTT GAAATCACTT 60
TCTGTAGTCA AAGTATACCA AAACCAATTT ATCTGAACTA AATTCTAAAG TATGGTTATA 120
CAAACCATAT ACATCTGGTT ACCAAACATA AATGCTGAAC ATTCCATATT ATTATAGTTA 180
ATGTCTTAAT CCAGCTTGCA AGTGAATGGA AAAAACNNNC AAGCTTCAAA CTAGGTATTC 240
TGGGAATGAT GTAATGCTCT GAATTTAGTA TGATATAANG AAAACTTTTT TGTGCTAAAA 300

ATACTTTTTA AAATCAATTT CNTTGATTGT AGGTAATTTC TATTTGCACT GGGCCTTTCA 360
ACTCCAGAAA CATTCTGANG GTGGTACTTG GGGTTTAANT TAAAAAGGTC CACTTTGGTA 420
AA 422

SEQ ID NO:920
SEQUENCE LENGTH:410
SEQUENCE TYPE:nucleic acid
TOPOLOGY:linear
CLONE:HUMGS01047
SEQUENCE DESCRIPTION:
GATCGCTGTT TCCAGAACGG GGAGGAGTAT CTCATTGTGA AACAGACTCT AGAGTGGTTC 60
TATTTGGTCT TCAGTGTTTT AGCCTCATTA GTTCATATTT GGCATGCAGC TTGTGGTGAG 120
TACTGTTCTA GGACTGGCCA AAAATGGGCA AAATGTATCA CTCCAAACAC TACTGATTCA 180
GCATTGTTTT CATGTCTTAA AATTGCCACC TGCACTTTGT TTCTGCACTA TTATGTAGTG 240
CATTTTAACT TAAATTTTTT CCAGCAACAT GTTACTTATT TANGATACAT TACTGATATT 300
TCATTATAAT TANGTTCATC TTCCCTGTGA AACAAGAGAA TTGTAAAATG TTGTGGGAAA 360
ATGATACATA TGTGGGATGC TAATGNAAAT CATAGGTATT TTTGTGTAAA 410

SEQ ID NO:921
SEQUENCE LENGTH:409
SEQUENCE TYPE:nucleic acid
TOPOLOGY:linear
CLONE:HUMGS01048
SEQUENCE DESCRIPTION:
GATCACANTT GANCTGGCAG CGGGATGGCG AGGACCAAAC TCAGGACACC GAGCTTGTGG 60
AGACCAGACC AGCAGGAGAT AGAACCTTCC AGAAGTGGGC AGCTGTGGTG GTGCCTTCTG 120
GAGAAGAGCA GNGATACACA TGNCATGTAC AGCANGAGGG GCTGCCAAAG CCCCTCACCC 180
TGAGATGGGA GCCATCTTCC CAATCCNCCN CCCCCATCGT GGGCATTGTT GCTGGCCTGG 240
NTGTCCTAGC AGTTGTGGTC ATCGGAGCTG TGGTCGCTGC TGTAATGTGT AGGAGGAAGA 300
GCTCAGGTGG AAAAGGAGGG AGCTACTCTN AGGCTGCGTG CAGCGACAGT NCCCAGGGCT 360
CTTAATGTGT CTTTNACAGN TTGAAANGCC TGAGACAGCT TGTTTTGTN 409

SEQ ID NO:922
SEQUENCE LENGTH:407
SEQUENCE TYPE:nucleic acid
TOPOLOGY:linear
CLONE:HUMGS01049
SEQUENCE DESCRIPTION:
GATCCAGGCT GTCATGTGAT TTATGGTGGC ATGTGTTGTG TATTTGTTGG CTACTTGTGT 60
CTTGAAATCT AGAATTATTT CACGCAGAAT TGTCACTGTT TGTCAGGAAG AGAAAATGGG 120
CTAGTGGAAG CCCAGTCTTG AGTTCTTGTC TTGTTACCAT TTAAAATTGA CATTTAATTT 180
TCAAATCACT GTTGGTGCCT AATCACTNAA GTTATTAATT TATTCTGTTG TATTCTTTTT 240
TTTNAAATNG TAACATATTT ATCCGGTGGG TGGGACAGGA GTGTGTTCAA GTGGGTCATG 300
TTTTTNCTGT GGTGACACAT GGTACAGGCT TGGAGCTTGC AGGTCCCTTT CTACTGTGGT 360
TTTGGAGCAG GNCAATTAAA GTCCACTANG AAATNCACCN CTTTAAA 407

SEQ ID NO:923
SEQUENCE LENGTH:411
SEQUENCE TYPE:nucleic acid
TOPOLOGY:linear
CLONE:HUMGS01051
SEQUENCE DESCRIPTION:
GATCCAGGTT CCTCCAGAAA TAAGATATAC AGATTACTTT GTNATTGTAA GTGGAACTTC 60
TACCCGACAC TTACATGCCA TGGCCTTCTA CGTTGTGAAA ATGTACAAAC ACCTGAAATG 120
TAAACGTGAC CCTCATGTTA AGATAGAAGG GAAGGACACT GATGACTGGC TGTGCGTGGA 180
TTTTGGCAGC ATGGTGATTC ATTTGATGCT TCCAGAAACC ANAGAAATCT ATGAATTAGA 240
GAAATTATGG ACCCTACGTT CTTATGATGA CCAGTTAGCT CAGATAGCAC CTGAGACAGT 300
ACCTGAAGAC TTTCATTCTT GGAATAGAAG GTGATACTTN ATCTGTNACT NCANTGGGNG 360
TTAAAATGTG GATTAAATTN TTTTANTGCA NTGNNGTTAG TCATTTTCAA A 411

SEQ ID NO:924
SEQUENCE LENGTH:109
SEQUENCE TYPE:nucleic acid
TOPOLOGY:linear
CLONE:HUMGS01052
SEQUENCE DESCRIPTION:
GATCAGAGCN TATNTTATGA TTGTTGATAA CTAACCAAAG TAGNTGCCTG CAGAGACTTT 60
AAAATGTAAA ATAAAGATGT ATGCTGCCTG TCAGCTATTC TCATTTAAA 109

SEQ ID NO:925
SEQUENCE LENGTH:405
SEQUENCE TYPE:nucleic acid
TOPOLOGY:linear
CLONE:HUMGS01053
SEQUENCE DESCRIPTION:
GATCCGAGGC TGGGAAGAAG GGGTTGCCCA GATGAGTGTG GGTCAGAGAG CCAAACTGAC 60
TATATCTCCA GATTATGCCT ATGGTGCCAC TGGGCACCCA GGCATCATCC CACCACATGC 120
CACTCTCGTN TTCGATGTGG AGCTTCTAAA ACTGGAATGA CAGGAATGGC CTCCTCCCTT 180
AGCTCCCTGT TCTTGGGTAA GGAAATGGAA TACTGAAGGG CCCTTCACTG CCTTTGCTCC 240
TCCCATGTTA TGCCCAGCGT TTGATGGGTA GCAGAGAGGA CANANATCAC CACATGGCTA 300
TTTTTCCCNC TGAATNCTGT CTNGNATTGN GTACCTNTCA AGTGTTATTA GTGNATGCTT 360
TNGAAATGAA AAATTTGGGC NACCTTATGG GNAGGGNGTN GGAAA 405

SEQ ID NO:926
SEQUENCE LENGTH:405
SEQUENCE TYPE:nucleic acid
TOPOLOGY:linear
CLONE:HUMGS01054
SEQUENCE DESCRIPTION:
GATCTTAAGC AGTAATCTGT CAGTGTTTGT ATTTGTATTC TCTGCAATTT TACTGTGAAA 60

AAAAATTTGT TTTCAACAAT TGGTGTCATT TTCTTGATGT CACTATTTGT NGGAGAGTTA 120
AATGGTCTCT NCCCTTTGTG TATCTTACCT AGTGTTTACT CCTGGGCACC CTTAATCTTC 180
AGAGGTGCTA AATTGTCTGC CATTACACCA GAAGGATGCC TCTGATAGGA GGACAACCAT 240
GCAAATTGTG AAATAGTCCT GANGTTCTTG GATTACTTTA CACCTCAGTA TTGATTTGTC 300
CCAGAATTTN CTGGCCTTNC ATGGCANTGA AANTNTTNGG GGGAAAGATT TAANGTATTT 360
NANTTTTAAN GAGTGTGTTA TAANNATANT TGTACTGNNT NCTNN 405

SEQ ID NO:927
SEQUENCE LENGTH:404
SEQUENCE TYPE:nucleic acid
TOPOLOGY:linear
CLONE:HUMGS01055
SEQUENCE DESCRIPTION:
GATCTATGTT ACATTTACCA CACTGAAGTT TTTTTTGTTG TTTTTTGTTT GTTTTTAAAG 60
AATCACCCTC ATTGTTGAAA GTAAATGTAC TCTTAGGGTG CGAATATTAG TGTTCCAATA 120
AGCATGTGAT TATATTAAGG TGGTGGTAGC GGGAAGATAA TTCTGATTCC ATTGGGAATC 180
TTAGGTTTTC GTAAATTTAT TGGGAAAATA GTTTTTCCTG TACTGCTGAA GTTTCTTTTT 240
GGTAAACAGT ATCTTTCTAA AAGAAAAAAG CATGAAGGGA GAAATTGAGG TGTGTATACA 300
TTTCCTCAAA TGACCAGCAT TGTATTCGTG AATACTGTGT ATCTTGCAGT GAACAGTGTG 360
GAAGCTGTTC ATTTTTCAAT CTGAAGTAAA ATACTTTCAA GAAA 404

SEQ ID NO:928
SEQUENCE LENGTH:399
SEQUENCE TYPE:nucleic acid
TOPOLOGY:linear
CLONE:HUMGS01057
SEQUENCE DESCRIPTION:
GATCANGTGT AAATGTGACC TTGTACAGTT TACTAAAATT ACTGATATTT TTCACTACAT 60
TGAGACAGTT ACTGTGAGAA TAGGACACAA ACACCAGCTA TTGCCTGCAT CTGGGAAATT 120
GCTGAATCGC ACAGCAGTCA TGTCATAATC AGAAAATTAC TGCCAAATAA TTGTAAAATT 180
TGTAAAGTAT AAAGTATATA AAGTAGATAC TAAATACAGN CACTTCANTA TTTTGTTGAA 240
GCTATTGACT GTACANTTAG ACATTTTCAN ANGGGTGTAA TTTATTTANN GTTGTCTCAT 300
TTTGGTAAAA TTTATGTGAA CTTTTAAAGC TAANTATTAA NCCTTAATAT GCTATGTAAA 360
TNTTTTCCNT NTATACCATT TNCTGGTGGT NTTTTTTTN 399

SEQ ID NO:929
SEQUENCE LENGTH:397
SEQUENCE TYPE:nucleic acid
TOPOLOGY:linear
CLONE:HUMGS01061
SEQUENCE DESCRIPTION:
GATCTGCAGA AGGTATCTGG TGATGCAGAG ACTCTTTCCC CGCATCCNTC ACATGAAAGA 60
CCCCATCGGT GACAGCTTCC AAAACGACAA GCTGGTGGTC TGGGAGGCGG GCAAAGCCGG 120
CCTGGAGGAG TGTCTGGTGA CTGAAGTACA GGTCGTGCAG AAAACTTGAG ACTGGGGTTC 180
AGGGCTTGTG GGGGTCTGCC TCAATCTCCC TGGCCGGGCC AGGCGCCTGC ACAGACTGGC 240

TGCTGGACCT GCGCACGAGC CCAGGAATGG ACATTCCTAA TGGGTGGTGG GCATGGGAGA 300
TGCCTGTTTA ATTTCGTCCG AAGCTGCCAA GGAAGAAGAC CAGAACTTTG TGTGTTTATT 360
TCATGATAAA GTGATTTTTT TTTTTTTTNA ACCTAAA 397

SEQ ID NO:930
SEQUENCE LENGTH:402
SEQUENCE TYPE:nucleic acid
TOPOLOGY:linear
CLONE:HUMGS01062
SEQUENCE DESCRIPTION:
GATCCCCAAC AATGTCAAGA CAGCCGTCTG TGACATCCCA CCTCGTGGCC TCAAGATGGC 60
AGTCACCTTC ATTGGCAATA GCACAGCCAT CCAGGAGCTC TTCAAGCGCA TCTCGGAGCA 120
GTTCACTGCC ATGTTCCGCC GGAAGGCTTC CTCCACTGGT ACACAGGCGA GGGCATGGAC 180
GAGATGGAGT TCACCGAGGC TGAGAGCAAC ATGAACGACC TCGTCTCTGA GTATCAGCAG 240
TACCAGGATG CCACCGCAGA AGAGGAGGAG GATTTCGGTG AGGAGGCCGA AGAGGAGGCC 300
TAAGGCAGAG CCCCCATCAC CTCAGGCTTC TCAGTTCCCT TAGCCGTCTT ACTTCAACTG 360
CCCCTTTNCT NGTCCNTCAA GAATTTNGTG TTTTGCTTGC CN 402

SEQ ID NO:931
SEQUENCE LENGTH:396
SEQUENCE TYPE:nucleic acid
TOPOLOGY:linear
CLONE:HUMGS01063
SEQUENCE DESCRIPTION:
GATCTGCCGA CCTTGCTATA GATGCCATGT TACCAATGAT TTCCTGTGGT GGGGGCTTGC 60
CATTNTTTAC TCTCTTATTT ACCAACTTCT GGCCTAGGCA TGACAGTGGG CACCTTCCCC 120
CAGCCCTGGN TGGGCCCAGC GCCTNTTTTC TGTGTTAGAA AGGTTTTATA TATATATAAA 180
ATTACATATA TATGTAGAAA TATATGTAAT NTTGGGGGCC CTGTTCCTTG CACATTTTAC 240
AGTTACCTCA TTTTTNCCCA TGTATGTATT TGAGAAAATG CTAATATATA GNGAAAAAAA 300
TGGGTTCTTA AAGCTNAAAT GTGNGGTTTT TNCCATTCCA GGGGNTCACA TTNGGTTTGN 360
GGCATNGACN ATACCTNGTA TGTCGNNNTA TAAANN 396

SEQ ID NO:932
SEQUENCE LENGTH:394
SEQUENCE TYPE:nucleic acid
TOPOLOGY:linear
CLONE:HUMGS01064
SEQUENCE DESCRIPTION:
GATCCGTGGA TTTTTGGTGC AATTCCCCTT TTATTTCTTG TCTGAAGAAA GCCTACTGCC 60
TTCTGTTGGG ACCAAAGAGG CCATAGTGCC CATGGAGGTT TGGACTTAAG AGATATTCAT 120
TGGCAGCTCA AAGACTTCCA CCCTGGAGAC CACACTGCAC ACAGTGACTT CCTGGGGATG 180
TCATAGCCAA AGCCAGGCCT GACGCATTCT CGTATCCAAC CCAAGGACCT TTTGGAATGA 240
CTGGGGAGGN CTGCAGTCAC ATTGATGTAA GGNCTGTAAA CATCAGCAAG NCTTTATAAT 300
TCCTTNTGCC TAANTTGTAA AANGGGGGNC TGCATTCTTG TTGGAAGNAT GNACTNTNTT 360
TNNGGAAAAC CACATTTTTA AAANTTCCCG TAAA 394

SEQ ID NO:933
SEQUENCE LENGTH:393
SEQUENCE TYPE:nucleic acid
TOPOLOGY:linear
CLONE:HUMGS01065
SEQUENCE DESCRIPTION:
GATCCACAGG CTTTTGTACT CAACGCTGAC AACAACCCTA AGAGGTAGGT ATCATTATAT 60
CAACCATTTT ATGAATAAGA AAACAACAGC ACAGAGAGAT GCAGTCACTT GCCCAAGGTC 120
ACACAGGGCC AGGGGTTGGG CCAGGATTCG AAGCAGGCAG GCTGTCTCCT GGGTCTGAAC 180
TCTCAACTAC TACACCCTAA TCAAACAATC CCTCTGGTCA AATGTGAGTG ATAATAATAG 240
TACCCACCTC GTGGGTGTTG AGGGTGAGCC CAAGTTAGCA TTCAGCGTGG GCATGTGAAC 300
AATTATAGTC AATATTGANT GGAGACCTAT GATGCTTTTA TGAAGGTTTC TATTTTGGGT 360
TAAAAAATGC ATAAAATTTC TCCTGACCAG AAA 393

SEQ ID NO:934
SEQUENCE LENGTH:393
SEQUENCE TYPE:nucleic acid
TOPOLOGY:linear
CLONE:HUMGS01066
SEQUENCE DESCRIPTION:
GATCACCTTA AGTGAAATNA TTTNCCTTTA ATCTTTNATG TATTTATTCA CTTTTGGAAG 60
CTAGGAATGA GCAACACAAA TTTTACTCTG AAGTCAGAAG AGCTCATATA TAATANTTCT 120
AATGTCCCAC CTATTTTCAC TTGTCCATTC CATGTACCAG CTTAGTTATG ATANNNNGTC 180
ACATAATTAT CTTTGATAAA GGTAGAGGCA CAAAGAGGCA AACTAAGCAA GTCAAATTCT 240
AATGTGTGTA CTTCATAATA ATTTTTATC CATTTTCATC TTTATATTCT GTAACATGAA 300
NCTTACCTAA TCTTCAAATG TTAGCTTCCA TTTTTTACCT TTGAAATACT TAAATCTTTC 360
TGGANTAAAT ATAATGGGGC CTNTAAAANT AAA 393

SEQ ID NO:935
SEQUENCE LENGTH:392
SEQUENCE TYPE:nucleic acid
TOPOLOGY:linear
CLONE:HUMGS01067
SEQUENCE DESCRIPTION:
GATCCTGTTC TTTCAGCAGG TGAAAAATAA AACGCANTCA AATTTCATGG TTTTAATTTT 60
NAACTCAGAA GCACTCAAAA ATGCAAAATG TGATAATGGG CACTTGTTTA AAAGANTTAG 120
TGTATCCAGC CTTCACTCCA GCTGGTTAAA AATGTTGCAC TTATCAGCAA CCNNACCACT 180
TTCATCTGCT GAAAGGNCAA ATGTGCTTGG TTTTACTATT ATGTAATCAC AACTTTCTTT 240
TNTGCTTGTA GTTGCTTAAA ATTATGTATT TGGTCTNGGG CTGCAATTTG GTTTNATGCT 300
NTATNTGATT ATTACNGCAG TAGGTTGCCT NTCCNGTATG GGAAAAATAA AGTGGAATNG 360
CCCNAATTAA ACCNCCTCTN TCTTAAGGTA AA 392

SEQ ID NO:936
SEQUENCE LENGTH:391

SEQUENCE TYPE:nucleic acid
TOPOLOGY:linear
CLONE:HUMGS01068
SEQUENCE DESCRIPTION:
GATCAGGCAG CCTCCTGATG CCAGANCACC TCAGGCAGAG CCTACTCAGC TGTACCTGTN 60
TGCCTGGACT GTCCCCTGTC CCCGCATCTC CCCTGGGACC AGCTGGAGGG CCACATGCAC 120
ACACAGCCTA GCTGCCCCCA GGGAGCTCTG CTGCCCTTGC TGGCCCTGCC CTTCCCACAG 180
GTGAGAAGGG TCCTGTCCAC CAGCACACTC AGTTCTNTTC CCTGCAGTGT TTCATTTNAT 240
TTTAGCAAAN ATTTTGCCTG TTNNTGTTNA AACATGATAG TTGATATGAG CTGAACCCCT 300
GGGTTNGGNG GGAATTGGTC AGAGTGGCAA CCTGGGACTG TGAGCCCTGT TCGGNACAGN 360
NTATGGATAT GAAAANTCTG NCCNNNGCNA N 391

SEQ ID NO:937
SEQUENCE LENGTH:390
SEQUENCE TYPE:nucleic acid
TOPOLOGY:linear
CLONE:HUMGS01069
SEQUENCE DESCRIPTION:
GATCTTTCCC AAGATTGTAA CTGAAAACTG CTGTCTCTTG TTTTNTTCGT TTTGGGGGTG 60
GTGGTGCTGG CTGGGCCATG CTTGTAAAGT GATGTGTGTC TCTGATTTAA CGGATTCACT 120
GTTTTCTCTG CTAATTGAGA GAGCGTTATT TACATTATTT ATTTGTTTTG ACACAAGTNC 180
TTTCAGTGTT TTATCCTAGC TAATGGCTTC TTAAAGGTAA TAAAACCCTT CCAACGTAAT 240
TGGTCAGATA AAACTTTTTT CCTTGTATGC TTAAATAAAG CAATTAGTGA AGCACTTCTA 300
TCCAAAATGA CTTTTTTGTC CTTTTTTAAA ACCAATTTAC TGTTACTGGA AACTTTGTAC 360
AATAAAGCAA ATCACGCAGA TTAANGGAAA 390

SEQ ID NO:938
SEQUENCE LENGTH:389
SEQUENCE TYPE:nucleic acid
TOPOLOGY:linear
CLONE:HUMGS01070
SEQUENCE DESCRIPTION:
GATCAGAGGG AAAGAATGAC CAACCNNGCA ATAAGTGTAC TAAACTCTAC GCTCTGGTTA 60
ATGTAATGTA CTCTCCTGGA CTGAATGCAG TGTATAATTN CTGTCTACAG CTAGAAGCTG 120
TGCCCCAGTT CCACATTTGA TTACACATGT NAGATTTGCT GCTGTTGCAG TATAAACACT 180
AGGTATAATA GGATTTGAAA TTGCATTACA GTTCATAAAA NTNGAAAATG AGGAATTAAA 240
CCNGCAAGTG AACATTTGAA CGNTTATNCT NTCTACATAA GACATGGTTG GGACATCAGT 300
ACTNACAAGA TGGTTTANGT ATGGTACTAG NGNANTTAAG NTTTCTTTCT CTCTGGTTTA 360
TNGATNGGGT TATTTCCATT ATGTATTNN 389

SEQ ID NO:939
SEQUENCE LENGTH:390
SEQUENCE TYPE:nucleic acid
TOPOLOGY:linear
CLONE:HUMGS01071

SEQUENCE DESCRIPTION:

GATCTCACGC TGCCTCTGTG GTTCCCTCCC TCATTTTTCC TGGACGTGAT AGCTCTGCCT 60
ATTNCAGGAC AATAATGGCT ATTCTAAACG CTAAGGAAAA AAAACAAACA CAGAACTGTT 120
TCAAGTACTC AAGACTGACT TACAGACCAA CCAACCACCT TGCTGGAACC CTTGCTAGCA 180
GGCATTCTTA TAAAAGAAAC TTTCGAGCCT CCTTATATTG CTGGAAACTC AGCTGTGCTC 240
CAGACTAGAG CCTCCTTACC TATGCTATGG ATTTTTAATT TATTTTCTCT TATTTCATGT 300
ACACTGCTTT TTTTGGTTAC AGTGTATGAT GGATGTGTAT GAAAAAAATG TATCTTTGGG 360
GAACCAATTA CAGTTTTGTT AATTTGGAAA 390

SEQ ID NO:940
SEQUENCE LENGTH:543
SEQUENCE TYPE:nucleic acid
TOPOLOGY:linear
CLONE:HUMGS01072
SEQUENCE DESCRIPTION:

GATCCACACA CGTTGGTCTT TTAACCGTGC TGAGCAGAAA ACAAAACAGG TTAAGAAGAG 60
CCGGGTGGCA GCTGACAGAG GAAGCCGCTC AAATACCTTC ACAATAAATA GTGGCAATAT 120
ATATATAGTT TAAGAAGGCT CTCCATTTGG CATCGTTTAA TTTATATGTN ATGTTCTAAG 180
CACAGCTCTC TTCTCCTATT TTCATCCTGC AAGCAACTCA AAATATTTAA AATAAAGTTT 240
ACATTGTAGT TATTTTCAAA TCTTTGCTTG ATAAGTATTA AGAAATATTG GACTTGCTGC 300
CGTAATTTAA AGCTCTGTTG ATTTTGTTTC TGTTTGGATT TTTGGGGGAG GGGAGCACTG 360
TNGTTTATGC TGGAATATGA AGTCTGAGNC CTTCCGGTGC TGGGGACCAC ACANGNGTTN 420
GTTGNAAAGT TTGACCAAGN AGNCCTGCGC ATNNNCTCTG GATGCCTNTG GTATCCATTC 480
TTNGANGCAA TCCGCTCGGG NCCCGTGGGC CCAATAAANC NGGTATTTNT CCCAANGGGG 540
AAA 543

SEQ ID NO:941
SEQUENCE LENGTH:132
SEQUENCE TYPE:nucleic acid
TOPOLOGY:linear
CLONE:HUMGS01073
SEQUENCE DESCRIPTION:

GATCTCAAGA TTTCTAAATT GTCAAGATTT ACATGGCATT GTGGTGGAAC TAGTTAACAC 60
TTAGAGCTTT TGGTATGTAA TAACTATTTG CTATGGACTG ATTAAATGTT TCAAAAGATT 120
GTGTTCTTCA AA 132

SEQ ID NO:942
SEQUENCE LENGTH:387
SEQUENCE TYPE:nucleic acid
TOPOLOGY:linear
CLONE:HUMGS01074
SEQUENCE DESCRIPTION:

GATCAGCACT AAGTCCTGCA TTCCTGTTAA AGCCACTTGG GTCATAAGAA GGGAGTAAAA 60
AATGAAGTCT GACTAGNAAC CTATTGCAGA GGCCAAGTAC ATTTAGTATG GCATTGAGTT 120
GTGATATAGT TTTACTTTGA TGTGCATTTT GAATTTCAGC TACACCTAGA TAGACGTAAA 180

ATGATAATTA AAATGCTGTA ACCAACTTAT CTAATAAAAT TGGCAACCAG CCACTATTTT 240
GTTGACTATG AGAAAGTTAA AAGTTTATGT TAATTTTTNG GGTCTGATAG AATATTTCAT 300
GTGTATTACA GTGGTATTCA TATGCTATGT CTCTAAACTT TATTTTCAAA AGCTTANGGC 360
CCANATACAA NCTTCTCTGG GNNTAAA 387

SEQ ID NO:943
SEQUENCE LENGTH:386
SEQUENCE TYPE:nucleic acid
TOPOLOGY:linear
CLONE:HUMGS01075
SEQUENCE DESCRIPTION:
GATCAGANAA AAAGTCCATA TGGACTGTGG ATACCTATCT AAAAGAAGAA AACTGATGGC 60
TAAGTTTGCA TGAAAACTGC ACTTTATTGC AAGTNAGTGT TTCTAGCATT ATCCCATCCC 120
TTTGAGCCAT TCAGGGGTAC TTGTGCATTT AAAAACCAAC ACAAAAAGAT GTAAATACTT 180
AACACTCAAA TATTAACATT TTAGGTTTCT CTTGCAGATA TGAGAGATAG CACAGATGGA 240
CCAAAGGTTA TGCACAGGTG GGAGTCTTTT GTATATAGTT GTAAATATTG TCTTGGTTAT 300
GTAAAANTGG AATTTTTTAG ACACAGTAAT TGAACTGTAT TCCTGTTTTG TATATTTAAT 360
AAATTTCTTG GTTTTCATTC TTTAAA 386

SEQ ID NO:944
SEQUENCE LENGTH:383
SEQUENCE TYPE:nucleic acid
TOPOLOGY:linear
CLONE:HUMGS01076
SEQUENCE DESCRIPTION:
GATCTTCTAG CTACCATNCA TTTTCTTCAC TGTTCACAAA AGATGAGTGT GAAATTCAGT 60
GAATGCTGTT ACTAATCCTG TTACGAGATG AATCTCATTT CACCAAAATT AAATTATGTT 120
TTTCCGCTAA AATGATGATA CAAGTTGAAG ACACATCACT CTGAAATTGG AAGACCTCAC 180
CACTTAAGGC TCCACAGTGG CTTACTCAGC TGAACTCTAG GTTACTACTC TTTACTTTGT 240
TCACCCATTG GGGGGTGCAG TTTTTTTAAA ATGTTGGGAG ATGGCCATTC TAACTACTGT 300
TGAATGTCTC TGTTTTGGGA AGGTATAACA NGAANTAAAA ANGNNTATAT ATGANGGGAG 360
AGNCTGGTTA TCTCCTCCCC AAA 383

SEQ ID NO:945
SEQUENCE LENGTH:382
SEQUENCE TYPE:nucleic acid
TOPOLOGY:linear
CLONE:HUMGS01077
SEQUENCE DESCRIPTION:
GATCTCTCCA TGTACCCTGC AGGGGGCTTG GTACTGTGAA ATNAGTAACT TAATCCTGAC 60
AACCGTAGTG CAAGGTATGG CCCATCTCCT GTACGCTTGG AGCGACCTTT GGCTACGTGG 120
CTGGCCTTGT TATTTCACCA CTCTGGATAT ACTGGAATAG AAAGCAACTT ACATACAAGA 180
ACAATTAACT GGAGCAAAGG GAGATATTTC TTTGTGCAGA TTCTGTAAGG GCTGGGCAGA 240
AATGTGTATG GTCAAAGCCA AGCAGTTCCA TTTACAGCTC TGTTTTTTAC GTAGTTACAA 300
CATGATGTGA TTGTAGCTTT TTAAACTATG AAACCCCTGA GAGATTGTAC CTTCCTAGTT 360

GAAATAAAGT ATTTATAATA AA 382

SEQ ID NO:946
SEQUENCE LENGTH:381
SEQUENCE TYPE:nucleic acid
TOPOLOGY:linear
CLONE:HUMGS01078
SEQUENCE DESCRIPTION:
GATCCGAACA CCTCCAGATT CCGGCTTCTA CATGGNNCAG ACGGGGACGC ACAGGCCACC 60
TTCCTTCTGG CAGGGACTCT TATTTATTCC CATTGCTCTA GGGCTTTCNN TTTCCCNTTC 120
TTCCGGTAGG CCGCGTAGAG GCATGCACCG GGTAGGTTTC CGCGGTGACC CCGCGGCGGC 180
CTGAGGGACG CTCCCTGCCC CATCCCGGCT GTTGGGCTGG GCCGCTTTGC CTCTGCTTCG 240
NCCTGTGCTG TGTTCTCCAG CTTTGTAGCA GCAGCTTGAC AAACCCAGGC GCACTGTACC 300
AAGGCAATGT AACTTTTNAT TTTCGGTCAA TTTAAGTTCT TTTTGTCACC AAATATTAAT 360
AAACCAGTTT TGGACTTNAA A 381

SEQ ID NO:947
SEQUENCE LENGTH:380
SEQUENCE TYPE:nucleic acid
TOPOLOGY:linear
CLONE:HUMGS01079
SEQUENCE DESCRIPTION:
GATCTGAGTC CAGAGTTGGC CACTTTGTGT GGGTCCTCAC AAGCAAAGAG AGCACTAAAC 60
TTGACATTGG GGGTCCACCA CTCCAACTTT NCTTTCTGAA GGTTTTGGTG TACATTGAGC 120
CCCAGAAGGA AAGGAGAGTA TCTGTGAGTG GGGGCCTCCC TTGACCCCAG TACGAAGTCT 180
ATGCCCTGAA TCCCCAGAGT AGCCCTTCCT GGTGCCCAAC TGGCCTGGGG ACAANCAGCG 240
TCCACTACAT CTAGGCTGCC GGCTAAGTGG CACACTTCTT GACCTCCTAC CAGGACTTTG 300
GTAAAAGCTA GCTTTGGGGA GGGGTGGGTT AAATATGAGA GGTGGAGGNG CCANTGGTAG 360
AATAACATGG GTAGACTAAA 380

SEQ ID NO:948
SEQUENCE LENGTH:380
SEQUENCE TYPE:nucleic acid
TOPOLOGY:linear
CLONE:HUMGS01080
SEQUENCE DESCRIPTION:
GATCTGCATC AGTTGTAAAG GGGAATTGGT ATATTCACAG ACTGTAGACT TTCAGCAGCA 60
ATCTCAGAAG CTTACAAATA GATTTCCATG AAGATATTTN NNNNCAGAAT TAAAACTGCC 120
CTTAATTTTA ATATACCTTT CAATCGGCCA CTGGCCATTT TTTTCTAAGT ATTCAATTAA 180
GTGGGAATTT TCTGGAAGAT GGTCAGCTAT GAAGTAATAG AGTTTGCTTA ATCATTTGTA 240
ATTCAAACAT GCTATATTTT TTAAAATCAA TGTGAAAACA TAGACTTATT TTTAAATTGT 300
ACCAATCACA AGAAAATAAT GGCAATAATT ATCAAACCTT TTAAAATAGN TGCTCATATT 360
TTTAAAAATA AGGTTTTAAA 380

SEQ ID NO:949

SEQUENCE LENGTH:399
SEQUENCE TYPE:nucleic acid
TOPOLOGY:linear
CLONE:HUMGS01081
SEQUENCE DESCRIPTION:
GATCAAAGCA TGCAATAAGC AATACAAAAT ACCAAGCCTT ATACTTAAAA GAAGTTTAAC 60
ATATTGGTTA ATATACTGGT TAATATACTG GTTAAACATA TTGAATGTAT ATAAGTGGCA 120
AAACTAGATT TTTAAGGAAG TGTACATTAT AATATTGGAG CTCAGTACTG CATGANGAGA 180
CTTCATTAAA ACTAAGAAAN CATTTATTTG GGGAGAAATT TTAGGCATTT AAGANCTTGT 240
ATTTTTCTAT TTTAAAAAGT TAAATTATTC CGTAATTTGG ANGGAAGTTT CGTTGAATGT 300
AGGCCATAAC CGTTTGANGG GTTTTCCTTT GGANAACTTG GTGTNTTTNG GTGCCCTTAN 360
TATTTTGGTC CTTTTAATAA AAATGCNCCT GNATTTTCN 399

SEQ ID NO:950
SEQUENCE LENGTH:379
SEQUENCE TYPE:nucleic acid
TOPOLOGY:linear
CLONE:HUMGS01082
SEQUENCE DESCRIPTION:
GATCAGGCCC TGGGCCCCCC TGCATCTTTT ATAGCAGTGG GTGTCCAGTC CAGGACACTG 60
GTGCTTTTTT ATACAAGAGA ACGAGCCAGA GTTCACTCCT TCCTCCTGGC TCTCTATATA 120
CCTGTGAATA TGTGAAATAG TGTAAATATG AAAGAACTTG TACCTATCAC TTCAACCCCT 180
GCCTTGTACA TAATACTATT CCATCCACAC AGTTTCCACC CTCACCTGCC CCNTCATACG 240
GAGTTGGATG GGGGCCGAGT NAGGTAACCA GGTGGCATCT ACCNNATGTT TTATAAGGAA 300
TTTTGTACAG TCTTNGTGAA ATAAAATAAC GTGCTTCATT TGNAAAAATN NNGTTNNNTT 360
TNTNTTTNNT NGNGGGTTN 379

SEQ ID NO:951
SEQUENCE LENGTH:376
SEQUENCE TYPE:nucleic acid
TOPOLOGY:linear
CLONE:HUMGS01083
SEQUENCE DESCRIPTION:
GATCTAGTAG AAACTAAGGG ATGGGAAAAA CCTTTTCATT TTTCATCTNC TTTCCAACAA 60
TATGAATTTC TTAGTTTTCA AACTATACTC AGGAAAGCTG GGCTTTAGGA TTTAACATGT 120
AGTGATGAGT TCTGTGTGTA TTTTAATATT TNACTCAGGA TTCCTATTAA TTGAAAAAAA 180
TTTTTAACTT TTTTATTATA AATCTTTTTT TCAGGGAGGN GATATCACCA AACATGATGG 240
AACAGGCGGA CAGTCCATTT ATGGAGACAA ATTTGANGAT GAAAATTTTG ATGTGAACAT 300
ACTGGTCCTG GTTTACTATC CATGGCCANT CAAGGCCAGG ATTACCAATA ATTCNTCATT 360
TTTGTTATAC CACTGN 376

SEQ ID NO:952
SEQUENCE LENGTH:375
SEQUENCE TYPE:nucleic acid
TOPOLOGY:linear

CLONE:HUMGS01084
SEQUENCE DESCRIPTION:
GATCAAGCCA CCACGTGCCC TACGATGGCC TAACAGGAGT GCCCATTGGC AGATTACACA 60
TGTAAATATG ACCTCAGACA AAAAGGAACC AGAGGCCCAA GGGCAATAAT AAGGTGGAAT 120
TTNCAGGTCA GCCCAGGAAT TGGCAGAGGA AGTAGGTGTC TGATAACCCT TTGTGGAGAA 180
TGAGATTCCC CCCACCTGTG TGAGAAAAAT AAACAGCTCT GGAGTCTTGT NCCTGACTCC 240
AGAGGAACGA GAGCATTCCA GGAAAGAGAG ATTCCCTGGA AAATTGAAAA TGTGAATCCT 300
AGGGGGAAAT TGGGGATTGT NTCTTTCCCT GTTGAAAATG TTTNGNTGGG AATAAATATC 360
TTCAGGAACC ATAAA 375

SEQ ID NO:953
SEQUENCE LENGTH:374
SEQUENCE TYPE:nucleic acid
TOPOLOGY:linear
CLONE:HUMGS01085
SEQUENCE DESCRIPTION:
GATCTTCTNC ACACACCCCA TCACCCAGAT AATTTACAGT TCTGTTAACA GTGAGGTTGA 60
TAAAGTATTA CTGATAAAAA ATTATCTAAG GAAAAAAACA GAAAATTATT TGGTGTGGCC 120
ATCTTACCTG CTTATGTCTC CTACACAAAG CTAAATATTC TAGCAGTGAT GTAATGAAAA 180
ATTACATCTT ACTGTTGATA TATGTATGCN CTGGTACACA GATGTCATTT TNGTTGGTCA 240
CAGCACTACA GTGAAATACA CAAAAAATGA AATTCATATA ATGACTTAAA TGTATTATAT 300
GTTAGANTTG ACAACATAAA CTACTGTNGC TTNGAAATGA TGTATGCTTC AGTAAAATCA 360
TATTCAAATN TAAA 374

SEQ ID NO:954
SEQUENCE LENGTH:376
SEQUENCE TYPE:nucleic acid
TOPOLOGY:linear
CLONE:HUMGS01086
SEQUENCE DESCRIPTION:
GATCAAAGCA AAGAGCGCCT TTCCATGTAT CTCATGAAAG CTGACCTGAT GCCTTTCCTG 60
TATTGGAATA TNATGCTAAG GGGTTACTGG GGAGGACCAG CGTTTCTNCG CAAGTTGTTT 120
CATCTAGGNA TGAGTTAAGN ATGGCTCAGC ACTTGCTCAT CTTGGATGGC TTCTGGGCCA 180
AAACTGCAGT CACTGAATGA CCAAGAGCAG CACGAAGGAC TTGGAACCTA TCCTTGTAAA 240
GAGTTCCTTG ATGGGTAATG GTGACCAAAT GCCTCCCTTT TCAGTACCTT TGAACAGCAA 300
CCATGTGGGC TACTCATGAT GGGCTTGATT CTTTGGGAAT ANTAAATTGA AATANTACTT 360
TTNTTTTCTG AATAAA 376

SEQ ID NO:955
SEQUENCE LENGTH:371
SEQUENCE TYPE:nucleic acid
TOPOLOGY:linear
CLONE:HUMGS01087
SEQUENCE DESCRIPTION:
GATCAGAACC TCCAAATACT GCCATGAGAA ACTAGAGGGC AGGTCTTCAT AAAAGCCCTT 60

TGAACCCCCT TCCTGCCCTG TGTTAGGAGA TAGGGATATT GGCCCCTCAC TGCAGCTGCC 120
AGCACTTGGT CAGTCACTCT CAGCCATAGC ACTTTGTTCA CTGTCCTGTG TCAGAGCACT 180
GAGCTCCACC CTTTTCTGAG AGTTATTACA GCCAGAAAGT GTGGGCTGAA GATGGTTGGT 240
TTCATGTTTT TGTATTATGT ATCTTTTTGT ATGGTAAAGA CTATATTTNG TACTTAACCA 300
GATATATTTT TACCCCAGAT GGGGATATTC TTTGTAAAAA ATGAAAATNA NAGGTTTTTT 360
NAANTGGNAA A 371

SEQ ID NO:956
SEQUENCE LENGTH:368
SEQUENCE TYPE:nucleic acid
TOPOLOGY:linear
CLONE:HUMGS01088
SEQUENCE DESCRIPTION:
GATCTGGGGA GGGCTAGCCC AAAACCTCCC GCATCGGGCA GGCACCCCCT GAAGTACTTC 60
CTTCAGGGTT TCCCCTTTGT NAGGGTGTCG AGTAGCCTCA CCGGCCTGTN TGGAGGAGCA 120
GCTGGCTCTG CTCTGAGAAA CTCTGGCAAG TGGACGCCAT TCTNTTGCCC TTAGGATTCA 180
CTGCTCTCTC CTACAGCCGC CAGNCCTGGG GTCCTGAAAG ACCTTGGGTG GTAAAGCTGT 240
ACTTGGTGGG AGTNAGGGCG TGGGGAGGAA CCATGCAAAT CGCCTTCCAT GGGTTTTTAA 300
NTGCAGTAAA TAACATTTCT GGATGAGACT NGTTTCCAAA NTAAACCNNG CTATTATCTG 360
TTTTGAAA 368

SEQ ID NO:957
SEQUENCE LENGTH:369
SEQUENCE TYPE:nucleic acid
TOPOLOGY:linear
CLONE:HUMGS01089
SEQUENCE DESCRIPTION:
GATCCGAGCT CTGGAGCGTG GATACCGGAT GTCTCGCCCA GAGAACTGCC CAGAGGAGCT 60
CTACAACATC ATGATGCGCT GCTGGAAAAA CCGTCCGGAG GAGCGGCCGA CCTTCGAATA 120
CATCCAGAGT GTGCTGGATG ACTTCTACAC GGCCACAGAG AGCCAGTACC AACAGCAGCC 180
ATGATAGGGA GGACCAGGGC AGGGCCAGGG GGTGCCCAGG TGGTGGCTGC AAGGTGGCTC 240
CAGCACCATN CGCCAGGGCC CACAACNCGN TTTNCTACTT TCCNAGACAA CNACCNTCGG 300
TTTCAGGCCA CAGTTTTCTT CATCTGTCCA GTTGGGGTAG GTTTGGGACT TGGNAAAATN 360
TTTTTTTN 369

SEQ ID NO:958
SEQUENCE LENGTH:366
SEQUENCE TYPE:nucleic acid
TOPOLOGY:linear
CLONE:HUMGS01090
SEQUENCE DESCRIPTION:
GATCGAGCCA CTGCACTCCT GGGGCAACAG AGCAAGACTT CGTCTCAAAA TAAATAAATA 60
AATAAAGTGG CTCTTGGGGA AAAGCAATTT AATGTACCAC GATGAATAGC TAACTGTTCC 120
CAAGTGTTTG CTATGTGCAA CACACCGCGT GAGAGTGTTA CCTGCATTAT TACATTAGGC 180
TGAGAGGTAA AATAATTTGC CCGAAGACAT ACAGCTAGTG ACGAATGGAC TGATGGTTTG 240

AACTTAACGT CTATTTGACT TAAGGTCCTG CACCCTGCCA CTTGTAATTT TCAGANTCAC 300
TGATAATCTG AAATAATGCA GCTTAAACCA TGTTTTCTTA ATTAAAAGTA TAATTGGATG 360
GTGAAA 366

SEQ ID NO:959
SEQUENCE LENGTH:364
SEQUENCE TYPE:nucleic acid
TOPOLOGY:linear
CLONE:HUMGS01091
SEQUENCE DESCRIPTION:
GATCCTGAAG GTGAAATGAA ACCAGGAAGA AAAGGTATTT CTTTAAATCC AGAACAATGG 60
AGCCAGCTGA AGGAACAGAT TTCTGACATT GATGATGCAG TAAGAAAACT GTAAAATTCG 120
AGCCATATAA ATAAAACCTG TACTGNTCTA GTTGTTTTAA TCTGTCTTTT TACATTGGCT 180
TTTGTTTTCT AAATGTTCTC CAAGCTATTG TATGTTTGGA TTGCAGAAGA ATTTGTAAGA 240
TGAATACTTN CCTTTAATGT GCATTATTAA AAATATTGAG TGAAGCTAAT TGTCAACTTT 300
ATTAAGGATT ACTTTGTCTG CCCACCACCT AGTGTAAAAT AAANTCAAGT AATACANTCT 360
TAAA 364

SEQ ID NO:960
SEQUENCE LENGTH:364
SEQUENCE TYPE:nucleic acid
TOPOLOGY:linear
CLONE:HUMGS01092
SEQUENCE DESCRIPTION:
GATCTGGGGC CGNCCTTACG GGGCAGGGCT CAGTCCTGAC GCTTGCCACC TGCTCCTACC 60
CGGCCAGGAT GGCTGAGGGC GGAGTCTATT TTACGCGTCG CCCAATGACA GGACCTGGAA 120
TGTACTGGCT GGGGTAGGCC TCANTNAGTC GGCCGGTCAG GGCCCGCAGC CTCGCCCCAT 180
CCACTCCGGT GCCTCCATTT AGCTGGCCAA TCAGCCCAGG AGGGGCAGGT TNCCCGGGGC 240
CGGCGCTAGG NTTTGCACTA ATGTTCCTNT CCCNGCGGGT GGGNGCGGGG AAATTCATAT 300
CCCCTGTTTC GTNTNATGTT GTGTCNNNCG NNCCCAAATT TAAAAAGGNA ATTTNAAAAN 360
GGTN 364

SEQ ID NO:961
SEQUENCE LENGTH:362
SEQUENCE TYPE:nucleic acid
TOPOLOGY:linear
CLONE:HUMGS01093
SEQUENCE DESCRIPTION:
GATCGAGGGG CGGGGGTCAG CTATGCAGCC CATCACGTGT GTTTTTCATC TGGGATGAAA 60
AAGCCTGGTT CTCTTTTGAA ATGCTTGATT GTACTTATTG AGCTAAACAA GNCTTGGTGA 120
CTNTTGTTGA TTTGCCTCAA AAGTTTTAAG TCCTGGGTTT TCAGACTACT GTGTAGCAGC 180
TGTGTGTTTA ACATACTGTA GCTTTTTCTC CCTTGGGGGC ACATACAAAT AGGATGTGTT 240
GATGTGGACT CTAAACTGTA ATTTCCTGT AACTATTTTG GAATGATGCA TATTTCTAAT 300
GTTTGTTATA CTTGTACAGA GTATTTGCTG TTGGTTGCTT TTTTTTTTN TTCANNGGGA 360
AA 362

SEQ ID NO:962
SEQUENCE LENGTH:360
SEQUENCE TYPE:nucleic acid
TOPOLOGY:linear
CLONE:HUMGS01094
SEQUENCE DESCRIPTION:
GATCCAGGCC AAGGCACTGG CTGTNAGTGG CAGAGTTTGG CTGTAACCTT TGCCCCTAAC 60
ACGAGGAACT CGTTTNAAGG GGGCAGCGTA GATTGTNTNA TTTGCCACCT GGATGAAGGC 120
AGACATCAAC ATGGGTCAGC ACGTTNAGTT ACGGGAGTGG GAAATTACAT GAGGCCTGGN 180
CCTCTNCTTT CCCAAGCTGT GCGTTCTGGA CCAGCTACTG ANTTATTAAT CTCACTTAGC 240
GAAAGTNACG GATGAGCAGT AAGTAAGTAA GTNTGGGGNT TTAAACTTGA GGGGTTCCCT 300
CCTGACTAGC CTNTNTTACA GGANTTGTGG NAATATTAAN TGCAAATTTA CAACTGCAAA 360

SEQ ID NO:963
SEQUENCE LENGTH:358
SEQUENCE TYPE:nucleic acid
TOPOLOGY:linear
CLONE:HUMGS01095
SEQUENCE DESCRIPTION:
GATCCAATTT GTAGCTTCCT GCCTGGCTTC AGAGAGCCCA GCAACCTTCT AGGCCTGCTT 60
TCCAGACTTC TGAGATAGCC TGGGATGAGC AATCCTGTTA TAGTACATCT GGACCTTCCC 120
TACCTGGGCT CTGGGGAGGC TGTGGGCCTG GAGAGGGAAA AGGAGGGAGG GGGTGTCTGC 180
ACCACCTGGG AAGATAGCAC AAGGCCTAAT GAGGTCACCC TGACTCCCCA CCCCAGCATT 240
TCATTCATAC CAGATAATAG CTGCATTACT GCCANCTGAC CTTATAACCC TCTGCACCTT 300
CAAAAAGGTT CATGGTTTTT AATTGCTGCT TTTTAATAAC ATTTTGTTNA AGNTTAAA 358

SEQ ID NO:964
SEQUENCE LENGTH:195
SEQUENCE TYPE:nucleic acid
TOPOLOGY:linear
CLONE:HUMGS01096
SEQUENCE DESCRIPTION:
GATCGGCCCT ACTAAGATGC AGAGACCCCG NCAGAGCTNG CATTGACTAC CAGATTTATT 60
TTTNAAACCA GAAAATNTTT TAAATTTATA ATTCCATATT TATAATGTTG GCCACAACAT 120
TATGATTATT CCTTGTCTGT ACTTTAGTAT TTTTNACCAT TTGTGAAGAA ACATTAAAAC 180
AAGTTAAATG GTAAA 195

SEQ ID NO:965
SEQUENCE LENGTH:356
SEQUENCE TYPE:nucleic acid
TOPOLOGY:linear
CLONE:HUMGS01097
SEQUENCE DESCRIPTION:
GATCCAAGAC TGGCTGACTT CATTTGAAAT GGTTGAATCT GCTGTGTAAT AAAGTGGTTC 60

AACCATGATT AGGAACTGAA ATTTAGTAGA AGAGGGAAAA GGAGTTAATG TAACAAATTA 120
TTTTAGCTAC AAACCCCGGT AATAGAGCAC TTGGGGGATG GGATGGGGTG GGTTGGTGAG 180
ACAATCAGAA TGGTAAATTG ATTAAATGCT CCTAACCCTG TAATTTTGTG CATAGAGCAC 240
CCTATGCTGT GGAAATAACT GTTCTTAGAT TTCATTGTAA CTGGACTGTT CAGGTTGCCC 300
AGAGGGAAAG ACCATTCCTA ATTCTAATAA AATAACCTTT TATTTTGTTA TTCAAA 356

SEQ ID NO:966
SEQUENCE LENGTH:356
SEQUENCE TYPE:nucleic acid
TOPOLOGY:linear
CLONE:HUMGS01098
SEQUENCE DESCRIPTION:
GATCTCCACC ACCATCTCCC CTCTACTTCT CATTCCCTAA CTCTCTGCTG AATATGGGGT 60
TGGTGTTCTC ATCTAATCAA TACCTACAAG TCATCATAAT TCAGCTCTTG AGAGCATNCT 120
GCTCTNCTTT AGATGGCTGT AAATCTATTG GCCATCTGGG CTTCACAGCT TGAGTTAACC 180
TTGCTTTTCC GGGAACAAAA TGATGTCATG TCAGCTCCGN CCCTTGAACA TGACCGTGGC 240
CCCAAATTTG CTATTCCCGT GCATTTTGTT TGTTTCTTCA CTTATCCTGT TCTCTGAAGA 300
TGTTTTGTGA CCAGGTTTGT GNTTTCTTAA AATAAAATGC AGNGACATGT TTTAAA 356

SEQ ID NO:967
SEQUENCE LENGTH:356
SEQUENCE TYPE:nucleic acid
TOPOLOGY:linear
CLONE:HUMGS01099
SEQUENCE DESCRIPTION:
GATCTTCCAC AAAACATCTA GCCATCTAAA ATGGAGAGAT GAATCATTCT ACCTATACAA 60
ACAAGCTAGC TATTAGAGGG TGGTTGGGGT ATGCTACTCA TAAGATTTCA GGGTGTCTTC 120
CAACTGAAAT CTCAATGTTC TCAGTACGAA AAACCTGAAA TCACATGCCT ATGTAAGGAA 180
AGTGCTATTC ACCCAGTAAA CCCAAAAAAG CAAATGGATA ATGCTGGCCA TTTTGCCTTN 240
CTGACATTTC CTTGGGAATC TGCAAGAACC TCCCCTTTCC CTTCCCCCAN TAGGNCCATT 300
TAAGTGTGTG TTAAACANCT ACAGNATACT ANNTAANAAG TTTGGCCAAN NCCAAA 356

SEQ ID NO:968
SEQUENCE LENGTH:356
SEQUENCE TYPE:nucleic acid
TOPOLOGY:linear
CLONE:HUMGS01100
SEQUENCE DESCRIPTION:
GATCAGATTT TTGGGTCATG TCTGTTGTAT TTTCAGTAAT GTGATTTCAG ATGGTCATCT 60
GGATTCTCCC ACTTCTCTAC TCCATTATTT CTCTACTTTT CCTTCCAGCA NACCTGANNC 120
GTGAGGGAGA TGGATTAATG TGAGTAACAG GAATGTGTCT TTAAAAAGCT AGAGTGGTTA 180
CATTTAATCA GGCAGTAAGA TAATTTGGGT TCTTGAGTTG TTTTGGNGTA ATATCCCACA 240
ACTGGGGTAG GAAGCTCAGG ACTTTTTTNT TTAAAGCTAG TCATTTCAAA AGCATATTGT 300
ATTTTTTTGA NTGACTACAG TATGCCCAAN TTCAANANCC AAANCCCNCT TTGGGN 356

SEQ ID NO:969
SEQUENCE LENGTH:356
SEQUENCE TYPE:nucleic acid
TOPOLOGY:linear
CLONE:HUMGS01101
SEQUENCE DESCRIPTION:
GATCCAACAA TTTTNAAGAG CTCTCTCTTA ATCTCTGACA TAATGAGTCT GAAACAAAGA 60
AAAGTTACCT TACCGTGTCT TTACTTCCTT TCTTCTGGGC TGTGAACTCA AGTGCCTTGA 120
GGGCCAGCTA AGAGCTTTTT GGGATATTTG TCTAACTTAA TTGAACTGTT ACTGAAAGAT 180
AAATTAACAA AATGGTTCAG AGTTTTGGAT TAAGACCTTT GTAACTAACT GACCGTCAGC 240
ACAGGAGCTT CGGTTTCCTT CTCTGTAAAA CAGGGCTCCT CATTCCAATT CCACCTATCC 300
TGTAGCCTTG TGGGAATAAA AGGAGGCACC ACGTGGAGGT GCTTGGCAGG NTGAAA 356

SEQ ID NO:970
SEQUENCE LENGTH:355
SEQUENCE TYPE:nucleic acid
TOPOLOGY:linear
CLONE:HUMGS01102
SEQUENCE DESCRIPTION:
GATCATANAA NGGCCATCTG GGCCCAGCTN GTGTACAGCG AGGGNGGGCA GCCCCCTCCA 60
CTCCACTCTG CTTCCACAAA GTCGGCTCCC GAGAGCTCGA GGCTGCTTCT TTTTATATGT 120
GCAGGGCCCG GGCGGGTGAA GGGTCAGAGA GACGGACACA AGGAGCCGGC AGGAGNGCGG 180
ANCGAGGATG TCCTTTCCCG GGAGACAAGT CGGGAAAGCC TGGCTGGACT GCCTCAGCCC 240
CGNGTGANTC CTGGNCTNAA GGNTTCCCCG TCCTGAGCTC GGGAGATNTT CAGAGTCACA 300
CTGNCGNCCT GTCTTGCCAC GGAGAGGTCA ACTTGCCACC GGNAGTNCNT GGTAN 355

SEQ ID NO:971
SEQUENCE LENGTH:355
SEQUENCE TYPE:nucleic acid
TOPOLOGY:linear
CLONE:HUMGS01103
SEQUENCE DESCRIPTION:
GATCCAAACA ATATCTTCAA GGCATTCTTT GGCGGTCCTG GCGGCTTCAN CTTTGANGCA 60
TCTGGTCCAG GGAATTNNTT TTTNCAATTT GGCTAATGAA GGGCAACCAC CCAGAACCCA 120
GAAAATNCAG ATTCACTCAG TTTAATCTTG AATGTGGAAA CAGTTCACCT CCTCCCTTCA 180
TCACGTCTCC GTGTGCTTAG AGCAGTTTCG TTTTCTCAGT TGGATGCCCT GTGTCTCTGT 240
GAGTGGGGTG GAGCAAAGGG AACCAATGCC GAAGACCGAG GGCAGGGGAG GGGAGGCGGG 300
GGTNGGACAG NGAGGCAGCT TGTGAATTTT TGTTTTACTG TTTAACTTTA TTAAA 355

SEQ ID NO:972
SEQUENCE LENGTH:352
SEQUENCE TYPE:nucleic acid
TOPOLOGY:linear
CLONE:HUMGS01104
SEQUENCE DESCRIPTION:

GATCNTAGGC TGGGGCCAAC TGGAACCATT GGCAGCTGCA CGCTCATTAC TACCCTCCGC 60
TCCTGCGCTC TGCCACTGTC CGGAAATTCA TGGTTGGCTA CGAAATGCTT GCTCAGGCTC 120
AGAGGGACCT CACCCCTGAG CAGGCTGCAG AGAGACTAAG GGCACTTCCT GAGGTTCATT 180
ACCACCTGGG GCAGAAGGAC AGGGAGACAG CAACCATCGC CTGACCACGC CGACCACAGG 240
GCCTTGAATC CTTTTTTGTT TTCAACAGTC TTGCTGAATT AAGCAGAAAG GGCCTTGAAT 300
CCTGGCCTGG AATTTGGGCA GATATAGCAT TAATAAAACT GTGCATCTCA AA 352

SEQ ID NO:973
SEQUENCE LENGTH:358
SEQUENCE TYPE:nucleic acid
TOPOLOGY:linear
CLONE:HUMGS01105
SEQUENCE DESCRIPTION:
GATCAAGGGG GTTGGGAGGG GGGAAAGAGA CCAGCCTTGG TCCCTAAGCC TCCACNNAAC 60
GTCTTCTTAA TCCNCACCTT TTCTTACTCC CAAAAAAGAA TGAACACCCC TGACTCTGGA 120
GTGGTGTATA CTGCCACATC AGTGTTTGAG TCAGTCCCCA GAGGAGAGGG GAACCCTCCT 180
CCATCTTTTT TGCAACATCT CATTTCTNCC TTTTGCTGTT GCTTCCCNCN TCACACACTT 240
GGTTTTGTTC TATCCTACAT TTGAGATTTC TAATTTTATG TTGAACTTGC TGCTTTTNTT 300
TCATATTNGA AAAGATGACA TCGGCCCCAA GGNGCCAAAA NTAAAATGGG ANTTGAAA 358

SEQ ID NO:974
SEQUENCE LENGTH:354
SEQUENCE TYPE:nucleic acid
TOPOLOGY:linear
CLONE:HUMGS01106
SEQUENCE DESCRIPTION:
GATCTTAGGC AAAATACCAG NTGATGAAGG CATCTGATGC CTTCATCTGT TCAGTCATCT 60
CCAAAAACAG TAAAAATAAC CACTTTTTGT TGGGCAATAT GAAATTNTTA AAGGAGTAGA 120
ATACCAAATG ATAGAAACAG ACTGCCTGAA TTGAGAATTT TGATTTCTTA AAGTGTGTTT 180
CTTTCTAAAT TGCTGTTCCT TAATTTGATT AATTTAATTC ATGTATTATG ATTAAATCTG 240
AGGCAGATGA GCTTACAAGT ATTGAAATAA TTACTAATTA ATCACAAATG TGAAGGTTAT 300
GCATGATNGT AAAAAATACA AACATTCTAA ATTAAAGGCT TTTGCAACCA CAAA 354

SEQ ID NO:975
SEQUENCE LENGTH:351
SEQUENCE TYPE:nucleic acid
TOPOLOGY:linear
CLONE:HUMGS01107
SEQUENCE DESCRIPTION:
GATCAGAGCT TGAACACAGG CTTATTTTTA AAANNANAAA TATTTTTAAC ATGGGTTTCC 60
TTATTGAAAA ATCAGTGTAT TAGTCATAAA ACACCATCAT TAAGAATAAT TGAACAATAA 120
AGTTTGCTTT CAGATGCAGT TTTCAAATTA TAATCTCATT TCAATTTATA ACGTTCTCAG 180
TCCTTTGTTA TAATTTTCCT TTTTCATGTA AGTTTAATTA TCTGCATTTA TCTTTTTTCC 240
TAGTTTTTCT AATACTAATG TTATTTCTTA AAATTCAGTG AGATATAGGG NTAAAATAAT 300
GCTTTGAGGA GNATGTTTAA TAGGAAATTA AAATAACTTT TTCTGGCCAA A 351

SEQ ID NO:976
SEQUENCE LENGTH:420
SEQUENCE TYPE:nucleic acid
TOPOLOGY:linear
CLONE:HUMGS01108
SEQUENCE DESCRIPTION:
GATCCAGAAC ACTTCAAGAA CTCGTCAAAC AGCTCGATAA GCCTTTTTNA CTGTNTACAT 60
CTGTACCGGG AATAACATTC CTAGGCTGAA ATTTCCACAA AGAATAGAAC CTGTACCCAG 120
TTCTTCAGGC TGATTTCCCT GACCTCTTGG GCATTTGTAT TTGTAGTAAA GTATTGCAGA 180
GATTCCTAAG TNTTTTATAG CAGCCATCAA ATTTGGACTT TGTATTGTTT ATTCATAAAA 240
GACACTTGGT AATAGACTTC AGTGAACTCT GTATGAATGC AGTAGTGTGC GTGCAAAATC 300
CGCTTCCTGA GCGTAGGGTG CTGAGCTGGC GCTAGGGCTC GGTTGTTGAA ATACAGCGTA 360
GGTCAGCCCT TGCGCTNAGT GTAGAAACCC ACGGTCTTTA AGGTTCGGGC CTTGGTCCAN 420

SEQ ID NO:977
SEQUENCE LENGTH:349
SEQUENCE TYPE:nucleic acid
TOPOLOGY:linear
CLONE:HUMGS01109
SEQUENCE DESCRIPTION:
GATCCTCATG TACTCAGAGG CACTTCCCTC CTAAGTCAAA GACCATCCTC ACTGACTATG 60
TGCCAACGCC TCGTTTCAGG CTTGTNACTC AACAAAGGGC TTTTCCATTG ATAGAAGCAG 120
TTTGGGATTT GTAGTTGCGA CTTCTTCGAT AGTTACCTGC ACGTCCATTG CTGGCAACTG 180
ACTTGTCATT AAAACCTGGC TCTTTGGTTA AGGGAGCTAC GCTGTGGTTT ATTCTTAAGT 240
TACGTGGATA AACTAACCTC TAACAGAAAT ATACTTTGGT TAATTTTGAA ATGTGTCATT 300
TTTAAACAAT CTTAAAAGTA ATACAGAATT GTGATTTATT AATTTTAAA 349

SEQ ID NO:978
SEQUENCE LENGTH:349
SEQUENCE TYPE:nucleic acid
TOPOLOGY:linear
CLONE:HUMGS01110
SEQUENCE DESCRIPTION:
GATCAGAGTG GGTTAAGCTG ACCAGGAACA CCCATTTAAC CCCTTTTNCT TTTTGCTTTC 60
ATTTTTATAA AGGAAAAGAG GACCTGTCAG ATAGGCAGCC CCATGCTACG TGATTCTTTA 120
TGTTGTGTTG TTTTGTTTTG TAAATTGTAT AATTTTTAAA TATCTGAGTT TTAAAAAAAG 180
AAAAAAGTAC AAAAAAATCT TGTTATGGCC TTAAGAAGGG NNTAGTGCAT CTTTCAGGGG 240
TCACTCTGCC ATGGGGATAA AATAGCTGTT TCACAAACAG TTTTATTTAA AAAANCAANN 300
ACCANNAAAN ANTCAAAATN TCATGNAANN TNTTNAACCT TCATTTTNN 349

SEQ ID NO:979
SEQUENCE LENGTH:345
SEQUENCE TYPE:nucleic acid
TOPOLOGY:linear

CLONE:HUMGS01111
SEQUENCE DESCRIPTION:
GATCACGTGG GTGTCAGTAT CTTTAACGGC CTTCATTCTT GGTTGTNAGA TTTTATTTGA 60
TATGCCCACT CACCCTCGAC GAATCTGCCC GCTTTGGGCT GTGGTGCCTG TGTATCTTTG 120
CCCGTCTGGT CTCCAGTTGG TGGAATNACC TTTTTTGTAC TGCCACTTCT CAGCATCTTT 180
NAAATTTGAC ATAATGTTGC TTCATTTCAG TTTTTTAAGT TCTGTAATTT GTTGATTGTA 240
TTTAACTATG TNAGTTCTGT TGTNATGTTT ACTGTATTGT AAAGCACCTC ATTCATGTNA 300
TGAGTGCTCT ATAAATCAAT AAATGATGAC TTAGAGGGCT GTAAA 345

SEQ ID NO:980
SEQUENCE LENGTH:344
SEQUENCE TYPE:nucleic acid
TOPOLOGY:linear
CLONE:HUMGS01112
SEQUENCE DESCRIPTION:
GATCTGCTTG GNGTTTTCTN CCCCCCACCC CAAACTTCTG TCGAGGAGCA AGGCTTGCCA 60
GCAAGTCAGA AGGATTTGAA CCGAGCAGCC AATCTTTCCA GCCCTCCCNT ACCGACCTCT 120
GTCTGGAGAC GCAGCAGCCT GTGTCCTCCA GGGCCTCTGG TTTGTNGTAT TATAGTATAT 180
TTNGCTGTGG AAAATGTCAC GTTTAGTCAC CTTGGAGCCA CTCACCTGGT CCTGTTGTTT 240
TANCCCATCC CTTCTNTNGN GGGCTATTGA TTTNTTCTNA GGAGAGTACA NCGTCACTAT 300
TGTAGNGTAA CCCTGTACTC AATATTACCA TAGNNCGNTG NCGN 344

SEQ ID NO:981
SEQUENCE LENGTH:357
SEQUENCE TYPE:nucleic acid
TOPOLOGY:linear
CLONE:HUMGS01115
SEQUENCE DESCRIPTION:
GATCAGAAAC CAACCATGAA TGAACCCCTG GCTCCTTCAC CACCCCCACG ATTGGTATGA 60
TGCTGCCGGC ACAGNTGGGA TACACACGGC TCCCCCAGGC CTGAGCTGCT TCACTAGGGA 120
ATCCTGCCAC CACCCTGTCT TCCTCTGCAA GTGCTCAGGG AAATGGCCTT NCCGCCGGAG 180
NCATNCTATC TGNCTGACAG GCTGTGACTC TTCTCTCAAC CTTGGCCTTC TCCCCTCTTC 240
TGAGCTAGTT GGTTGAATNN NNGTTAATGC TTAAGATTTG TTTTTCTCTT TTCACAGCAA 300
CATTTTCTTG AATTTTTTTC TGCACAGCTT TTCCAAAATA AAAACCTTCC AAACAAA 357

SEQ ID NO:982
SEQUENCE LENGTH:340
SEQUENCE TYPE:nucleic acid
TOPOLOGY:linear
CLONE:HUMGS01116
SEQUENCE DESCRIPTION:
GATCAGAAAT AAGATTGACT TGGGTGTTAT ATTTCATCTC TCTCCAGACT CTAGGTATAT 60
TTCCAACTTT ATATATCACA GTATTTAAAA AGACATGTTT GCATTGAGAA ATTAACCCTA 120
AAGGGTTTTC AATAGGGTGT AGACCTCCAG TACCTTTGTA ACTAAAGTCT GTCTAGTCAT 180
NGTAAATATT TATCTGTCAG TTTTGACAGA TTGGGGCCAG CTTGATGTTT TAAATCTTCA 240

GCCCGGTATG AAAACTTAAA GGTATATATT CANTTTTTTA CCATTTTATG GAAAATATTT 300
AAAATCTGTT TTTACAGGGT TTTTTTTTTT TTTTTTTTTN 340

SEQ ID NO:983
SEQUENCE LENGTH:340
SEQUENCE TYPE:nucleic acid
TOPOLOGY:linear
CLONE:HUMGS01117
SEQUENCE DESCRIPTION:
GATCTGGTTC GGCTGCTGTC GGATGAGGAC GTAGCGCTCA TGGTGCGGCA GGCTCGTGGC 60
CTCCCCTCCC AGAAGCGCCT CTTCCCCTGG AAGCTGCACA TCACGCAGAA GGACAACTAC 120
AGGGTCTACA ACACGATGCC ATGAGCTGAC GGTGTCCCTG GAGCAGTGAG GGGACACCAG 180
CAAAAACCTT CAGCTCTCAG AGGAGATTGG GACCAGGAAA ACCTGGGAGG ATGGGCAGAC 240
TTCCTGTNTT TGAGGCTAAT GGACCCGTGG GGCTTGTAAT CTGTCTCTTT CTACTATTTA 300
CATCTGATTT AAATAAACCA TTCCATCTGA AAGGGGCAAA 340

SEQ ID NO:984
SEQUENCE LENGTH:339
SEQUENCE TYPE:nucleic acid
TOPOLOGY:linear
CLONE:HUMGS01118
SEQUENCE DESCRIPTION:
GATCTATGTA TTATTAGAAA AATGAAGTAT TTCTGACATG GAACAAAGAA AGTGGAAACT 60
GGTACTTAAT GGGGGAAGCA AAATTAGCTG GGACTAAAAC GGACATGTTT TGTTTTGTGA 120
ATTCTACCTA AATGTCTCTC TATCCACAGA GAAACTAGTA TTACTTGAAG ATGTGAAAGT 180
TCCTGTGGTA GCCATACCTT GAAGCACAGT GTTTGTACAT AAGTAAATAT CTTGATTCTA 240
AATTAAATCC AGATTTATCT AATATATATN ATTTNATATC TTTGTTGTAT TAAANTGGTT 300
TAATANTCAC TANAANTANN ACATTTTGNA TGTTGGAAA 339

SEQ ID NO:985
SEQUENCE LENGTH:339
SEQUENCE TYPE:nucleic acid
TOPOLOGY:linear
CLONE:HUMGS01119
SEQUENCE DESCRIPTION:
GATCTCAGGA ATTTTTGTAG GGGATTGAAG CCAGANCTAG TTGCGTCCCA GGGACCAAGA 60
GAAAGAAGCA GATATCCAAA GGGTGCAGCC CCTTTTGAAA GGGGTGTTTA CGAGCAGCTG 120
TGAGTNAGGG GACAAGGGGC AGGTCCCAGG AGCCACACAC TCCCTTCCTC ACTTTGGACT 180
GCTGCTTCTN TTAGCTCCTC TGCCTCTGAA AAGCTGCTCG GGGTTTTTNA TTTATAAAAC 240
CTCTCCCCAC CCNCCACCCN CCAACTTCCT GGGTTTTCTC ATTGTCTTTT TGCATCAGTA 300
CTTTGTATTG GGATATTAAA GAGATTTAAC TTGGGTAAA 339

SEQ ID NO:986
SEQUENCE LENGTH:339
SEQUENCE TYPE:nucleic acid

TOPOLOGY:linear
CLONE:HUMGS01120
SEQUENCE DESCRIPTION:
GATCCTTTCC GACACACATG TCTGAAGACT TATTTTCAAA GNCAGCACAT TTTTGGAAAC 60
TAATCTCTTT TCCGTAATAT TTCCTTTATT TCAATGATTC TCAGAAGGCC AATTCAAACA 120
AACCCACATT TAAGGTTCTT TAGGATTATA GAATAAATTG GCTTCTGAGT GTTAGCTCAG 180
TGAGCTAGGA AAGCACCAAT CGATATTTGT TTCCTTTAGG GATACTTTGT TCTCACCACT 240
GTCCCTATGT CATCAAATTT GGGAGAGATT TTTTAAAATA CCACAATCAT TTGAAGAAAT 300
GTATAAATAA ANTCTACTTT GAGGACTTTA CCAAGTAAA 339

SEQ ID NO:987
SEQUENCE LENGTH:337
SEQUENCE TYPE:nucleic acid
TOPOLOGY:linear
CLONE:HUMGS01122
SEQUENCE DESCRIPTION:
GATCCAAAGT GTTAAAAATG CTGAAGTCAT GTCAAGTACT GTCTGGAGGG TTTTTTTAAG 60
AAAAGGCATT TGGCATTTAA CTGTCTCTTG TTTTATTTTT AAGTTTTTGG AAACCTTTTG 120
ACATAAAATG CTGCCAAGTA TCTAAGAAAT GTATATACTG ACAGAAGATA TTTGAAAGTG 180
GAAAATTGGA AATGAAATAT GTTGCTGGGN GCGTTAATCA CCTCCGCCCA GGATTTAGTC 240
ACTTGCAGGA CCTCTTTATA GTCTAGGATG GCAGAGCAGA AGATTTTAAT ATGCTTTTAT 300
TAAGTGATGT AAAATAAATG CTTTTTGGAT TATCAAA 337

SEQ ID NO:988
SEQUENCE LENGTH:371
SEQUENCE TYPE:nucleic acid
TOPOLOGY:linear
CLONE:HUMGS01123
SEQUENCE DESCRIPTION:
GATCGGCCAG CCCCCACCTA CAGCAACATG GAGGAGGTGG ATTAGCAGGT CCCTGGCTGA 60
TGGGGGGGAC TGGGTTTGGG ACACCCACAC AGNGGGCCAG CTCCTTGCCG CTTCTCCTTC 120
TCTAACCCAG AGGACACTGG CTCTGTCAGT GGGAAGCTGA GGGGTATGAT TTGGGTGTGG 180
AGACCTCTCA GGTTGGGACT TCTTGTCAGC TTGGACCCCT GACCAGTGGG CTTTGGCTTC 240
TCCAGCCGCC TCCAGTGCTG CGTGATTTGA TTCTGTTGTA CCTTCAATTC TTCTGACCCG 300
CATTATAAAC ATTATAATTN NATTCTAAAA ATTGTAATTT TTTTTGCAAT TTTGGAAGTG 360
ACTGCTGCTG N 371

SEQ ID NO:989
SEQUENCE LENGTH:334
SEQUENCE TYPE:nucleic acid
TOPOLOGY:linear
CLONE:HUMGS01126
SEQUENCE DESCRIPTION:
GATCTTCTTT ATATTCTACT TGAGTGCTGT CTCCATGTTG ATGTATCTNA GCAGGTTGTC 60
CACAGGTAGT CTAGGAGGGT GGCAACTTAG AGGTGGGGAG AGAGATTCTC TTATCCAACA 120

TCAACATCTT GGCAGATTTG ACTCTCAATC TCTTCACTAA AGTTGTTAAG NTCNNCCGGT 180
GATAAGTACT TCAATTTCAA CTTGTAGNNT GGGGAAATTT AGAATTATGC AGNTTATGGA 240
ATTGTATATG ATGACATTTG CAATAGGTCT ATTCTCTATC ATTGTAAGAA GNTGTGTGTA 300
CTGGTATTTG NCCCAAGTAA TAANCTAACT GAAA 334

SEQ ID NO:990
SEQUENCE LENGTH:333
SEQUENCE TYPE:nucleic acid
TOPOLOGY:linear
CLONE:HUMGS01127
SEQUENCE DESCRIPTION:
GATCTGNCGT GCCCACCAAC TGGTGATGGA AGGTTACAAG TGGCACTTCA ATGAGACGGT 60
GCTCACTGTG TGGTCGGCAC CCAACNACTG CTACCGCTGT GGGCGTGTGG CAGCCATCTT 120
GGAGCTGGAC GCGCATCTCC AGAAAGATTT CATCATCTTT NAGGCTGCTC CCCAAGAGAC 180
ACGGGGCATC CCCTCCAAGA AGCCCGTGGC CGACTACTTC CTGTGACCCC GCCCGGCCCC 240
TGCCNNTTNC AACCCTTCTG GCCCTCGCAC CACTGTGACT CTGNCATCTT CCTNAGACGN 300
AGGNTGGGCG TGGGNGGGNN TGTNCTGGNT NTN 333

SEQ ID NO:991
SEQUENCE LENGTH:329
SEQUENCE TYPE:nucleic acid
TOPOLOGY:linear
CLONE:HUMGS01128
SEQUENCE DESCRIPTION:
GATCGGACTG AACAGGAATC CTCGGGGGGT GAACAGCCAT TCCTTCGTGA CCTGTGCACG 60
NCTTCTGCAA CCCTGGAGCT CTGCTCGGCT AGTCTGACTC GAAAAGGGCG TGACTCAAGC 120
TGACGGGACT CCAGTAGGGA CTTTGAGAGC ACATTTTGTA AAAATATTTA TCTAGACGCA 180
AATGCTTATC CATGAATGTC CTCTTAGACC ATTTGGGGAT GAAGCCATCT TAATAATTAG 240
TAATAATTAA TTAGTAATAA TTAGTAAGCA TTTTCTCAAT GCTCTGATTC CATCATGTTT 300
TCTTAACATG ATAACTTAAA AAATTGAAA 329

SEQ ID NO:992
SEQUENCE LENGTH:332
SEQUENCE TYPE:nucleic acid
TOPOLOGY:linear
CLONE:HUMGS01129
SEQUENCE DESCRIPTION:
GATCCTCCTC AGAAACTACC GGACTTGTTT TCTGTATTGG TGTGTTTTGT ATCTTGCTTG 60
AACTTCCTGT TCTTCTTGGT ATACTTTAAC ATTATNATNA TGTGGGATTC CAAAAGTGGA 120
AGAAATCAGA AGAAAATCAG CTAGCTGTAT TCCTAAACAA ATTGTTTCCT AAACAAATGT 180
GAAAATGTGA ACAGTGCTGA AAGGTTTTGT GAACTTTTTG CTATGTATAA NTGAAATTAC 240
CATTTTGAGA ACCATGGAAC CACAGGAAAG GAAATGGTGA AAAGTCATTG TTGTCTACAC 300
AAAATAAATG TATATGGAGA CCAAAGACCA AA 332

SEQ ID NO:993

SEQUENCE LENGTH:330
SEQUENCE TYPE:nucleic acid
TOPOLOGY:linear
CLONE:HUMGS01130
SEQUENCE DESCRIPTION:
GATCAAGAAA TCAGGATGGC CATTTATTTA ATATCCATTC ATTTCATGTT AGTGGGACTA 60
TTAACTTGTC ACCAAGCAGG ACTCTATTTC AAACAAAATT TAAAACTGTT TGTGGCCTAT 120
ATGTGTTTAA TCCTGGTTAA AGATAAAGCT TCATAATGCT GTTTTTATTC AACACATTAA 180
CCAGCTGTAA AACACAGACC TTTATCANGA GTNGGCAAAG TTTTCCAGGN TTCATATACA 240
GNTAGGCTAT NNGNCATGTA TTTTGAAACG CAGTGTTNCA TNATGAAAGN GCTCTCAAGT 300
NGCTTNAAAG NTANTTTATT AAANGGGTNN 330

SEQ ID NO:994
SEQUENCE LENGTH:330
SEQUENCE TYPE:nucleic acid
TOPOLOGY:linear
CLONE:HUMGS01131
SEQUENCE DESCRIPTION:
GATCCGGGCC TTGCGTGCAG CCTCCCAACC ATGGGCTGGG TTTNGTGCTT ACTGTATGTT 60
GGCGACTTGG NNAGGGCAGG AGACGCAGCG TGGAGCCTAC CTCCCGACAT TCACGCTTCG 120
CCCACGNTGC TCCGACTGGC TGCAGCGGAC ACTGCCCAAA GCAGAGGGGA GTCTCAGTGT 180
CCTGCNAGCC AGCCGAACAC TTCTCTCCGG AAGNAGGCTG GTTCGACTGT NAGGTGTTGA 240
CTAAACTGTT TCTCTGACTC GCCCANAGGT CGTGGCTAAA GGCACTTAGG CGNCTTAAAT 300
TTGTAAATAA AATGTTACTA CGGTTTTAAA 330

SEQ ID NO:995
SEQUENCE LENGTH:338
SEQUENCE TYPE:nucleic acid
TOPOLOGY:linear
CLONE:HUMGS01132
SEQUENCE DESCRIPTION:
GATCTGAATG ATGATGGAAA CATGCAGACA GCCTCTCAGT CTTACTATTT AATGTTGTAG 60
CTGGGAAAAA ACCCAGAGAG GTTAACTGAT ATACTGGGTT GGGACTAGGA TGTGGGTTTT 120
GTNACTCTNA ATCCCATGTC CTCAAACTAC GCTGCCTTCC GAAGTCTGGC ATTTGTNAGC 180
TCATGCTTCC TTGTAGTCCA GCTTCTTATG TGCCTGTAAT ATTCTCCAGT ANGATTGTAA 240
GCCCCTTAAG GGCAGGGACG TCTTTNCATC TCTAGCACTG CTATAGTGTT CTATCCTTAG 300
TTATGGACCT AGATAAATAA NTNGGTGGTG GCAACAAA 338

SEQ ID NO:996
SEQUENCE LENGTH:328
SEQUENCE TYPE:nucleic acid
TOPOLOGY:linear
CLONE:HUMGS01133
SEQUENCE DESCRIPTION:
GATCTTTATA TTTNATACTC TAAANTCGTA CAAGCCAATC TNCATTTCNA CTAGTGGAAA 60

CTGTATAGCT GGTCATCTTT CCAGGACCCT TTTATCAAGA AACAATGCAG CTTCTACATT 120
TGTNCTGCTT CTACACCAAA ACAGCTGGAA TGTATATNGT ATGGTTCTGG ATGCTCTTGT 180
ATACCTNACT CTTCATTTCT NACCTAACCC ATGTGCTATG ATTTGAATGT TTCTCCCCTG 240
CAAAACTCAT GTTGAAATGT AATTGCCATG NTAACAGTAT TANTAGGTGG NNTATTTNAG 300
NGGTGNTTAG GGTGGGATTG GTGNTGTN 328

SEQ ID NO:997
SEQUENCE LENGTH:326
SEQUENCE TYPE:nucleic acid
TOPOLOGY:linear
CLONE:HUMGS01134
SEQUENCE DESCRIPTION:
GATCTTGTGT CTTAGAGAAG CCCCCATACC TGGTAGAGCA TGTACCATCT TACATGCTTA 60
AATAACTCCA CATTTATTTG TGTTTATNAC TCTGTGTTAT AAATATACAT TTGTNGGTCT 120
CTCTCTTGGA TTATTTTGTT TCTTTGTCCT GTAACTACCA CTGAAAGGGT GCAATACAGC 180
TTTCTTGAAA TGTGTATTGA ACGGATGAAT GTATAAATAA AANTTAAATT TTGTAAATTT 240
CTGCTTATNC TTAGAAAAAG AATCTAAATN GTGACAAATC AGAATTGAAA AANGTATTCT 300
AATAAAGANA AACANGCTTT TATAAA 326

SEQ ID NO:998
SEQUENCE LENGTH:325
SEQUENCE TYPE:nucleic acid
TOPOLOGY:linear
CLONE:HUMGS01135
SEQUENCE DESCRIPTION:
GATCCCCAGT GTTGGAGGTG GGACCTAAGT GGCAGGTGTT TGGGNCATGG GGATGGATTC 60
CTCACAAACG GCTCGGTGGC CTCCCTGCAG TAACGAGTGA GTTCACACAC TATTAGCTCA 120
CATGAAACCT GGTTATTAAA GAGTCTGGGA CCTCCCTCCA TGCTCTCTCT CTTGCTCCTT 180
TCTCTCACCA CATCACACGT GGCTCCCCTT GCCTTCTGCC ATGAGTGAAA GCTTCCTGAG 240
GGCCTCACCA GACACAGATG CTGGTGTCAT GCTTTTTGTA CAGTCTGCAG AACCCCGAGT 300
CAAATAAACC TCTTTTCTTT ATAAA 325

SEQ ID NO:999
SEQUENCE LENGTH:322
SEQUENCE TYPE:nucleic acid
TOPOLOGY:linear
CLONE:HUMGS01136
SEQUENCE DESCRIPTION:
GATCACTTTC AATTGAAGTC AGGGTATTGT GCATAATAGA AAGTATTGGA CTGAGATATT 60
TGGTTACCAT GGAGGCCAAT GCTTTTTTCA TCTTATTAAA TGTGATGTGA CTTTTTNCTT 120
TGTACAGAAG AGTACTGTAT TTTTGAATAG CCTACTCCCA AGTAAGAGCA AATCTGTATG 180
ATAACATTTT TNCCNCTGGA CATAAGACAT AACAGTAACA CGATGTACAT TTACAAGCGG 240
CCTTATGTAC ATTTCCCAAC ANTCTTTTTA AGGCAAAATT GTGACCATAT GTGTATAATT 300
AAAATCGTTT TTAATCCNTA AA 322

SEQ ID NO:1000
SEQUENCE LENGTH:322
SEQUENCE TYPE:nucleic acid
TOPOLOGY:linear
CLONE:HUMGS01137
SEQUENCE DESCRIPTION:
GATCTTAGAA ACCTCTGCAC AGAAGCTGCT TTGCTGGCTC TGCAAGAAAA TGGACTAGAC 60
GCAACTACAG TNAAACAAGA GCACTTTCTA AAATCACTTA AGACTGTAAA ACCGTCGTTA 120
AGTTGCAAGG ACTTGGCTTT ATATGAAANC TTATTTAAGA AAGANGGATT TTCTAACGTG 180
GAAGGTATTT AAAANTCACC TTAAACTCTT GTNCAGTTCA CATTAATTGA AATGTGAACT 240
TGCCTGTCGT TTGCAACTTC ACACTTTTAG AATTTGTGTT TATATTTCCT GTANGTGAAT 300
AAATANANCA NNNCAGNNCA AA 322

SEQ ID NO:1001
SEQUENCE LENGTH:353
SEQUENCE TYPE:nucleic acid
TOPOLOGY:linear
CLONE:HUMGS01138
SEQUENCE DESCRIPTION:
GATCTCAGAA CAACCTTTCT TGTTAGTAAC ATATTTTTGG CAATACATAN CAACCTGGGC 60
CTGGTGGATA ACCAACAAGA TGGGGAAGAA AAGNATTGAG AACTTTAAGA GTGGTGTGGA 120
TGCAGACTCT TCTTATTTNA AAATCTTTAA GACAAAACAT GACTGAAAAG AGCACCTGTA 180
CTTTTCAAGC CACTGGAGGG AGAAATGGAA AACATGAAAA CAGCAATCTT CTTATGCTTC 240
TGAATAATCA AAGACTAATT TGTGATTTTA CTTTTTAATA GATATGACTT TGCTTCCAAC 300
ATGGAATGAA ATAAAAAATA AATAATAAAA GATTGCCATG GANTCTTTGC AAA 353

SEQ ID NO:1002
SEQUENCE LENGTH:320
SEQUENCE TYPE:nucleic acid
TOPOLOGY:linear
CLONE:HUMGS01139
SEQUENCE DESCRIPTION:
GATCTGACCT GGTGAGATTA TTTCTGATGA CCTCATCAAA AAATAAACAA TTCCCAATGT 60
TCCAGGTGAG GGCTTTGAAA GGCCTTCCAA ACAGCTCCGT CGCCCCTAGC AACTCCACCA 120
TTGGGCACTG CCATGCAGAG ACGTGGCTGG CCCAGAATGG CCTGTTGCCA TAGCAACTGG 180
AGGCGATGGG GCAGTNAACA GANTAACAAC AGCAACAATG CCTTTGCAGG CAGCCTGCTC 240
CCCTGAGCGC TGGGCTGGTN ATGGCCGTTG GACTCTGTNA GATGGAGAGC CAATCTNACA 300
TTCANGTNTT CACCAACCNN 320

SEQ ID NO:1003
SEQUENCE LENGTH:318
SEQUENCE TYPE:nucleic acid
TOPOLOGY:linear
CLONE:HUMGS01140
SEQUENCE DESCRIPTION:

GATCCTNCNA GTTAGCCTAG TACTGCTGTA CTGGCCTGTA TGTACATGGG GTCCTTCAAC 60
TGAGGCCTTG CAAGTNAAGC TGGCTGTGCC ATGTTTGTAG ATGGGGCAGA GNCATCTAGA 120
ACAATGGGAA ACTTAGCTAT TTATATTAGG TACAGCTATT AAAACAAGGT AGGAATGAGG 180
CTAGACCTTT AACTTCCCTA AGGCATACTT TTCTAGCTAC CTTCTGCCCT GTGTCTGGCA 240
CCTACATCCT TGATGATTGT CCTCTTACCC ATTCTGGAAT TTTTTTTTN GNNGATANNT 300
ACAGAAAGCA TNTTGAAA 318

SEQ ID NO:1004
SEQUENCE LENGTH:320
SEQUENCE TYPE:nucleic acid
TOPOLOGY:linear
CLONE:HUMGS01141
SEQUENCE DESCRIPTION:
GATCTTTCTG TCTTCTGGGT TCCATTTTTN AAATGTTTAA AAATATGTTG ACATGGTAGT 60
TCAGTTCTTA ACCAATGACT TGGGGATGAT GCAAACAATT ACTGTCGTTG GGATTTAGAG 120
TGTATTAGTC ACGCATGTAT GGGGAAGTAG TCTCGGGTAT GCTGTTGTGA AATTGAAACT 180
GTAAAAGTAG ATGGTTGAAA GTACTGGTAT TGTTGCTCTG TATGGTAAGA NCTAATTCTG 240
TNNCGCCATG GTNCATAATT NCCTATNCAC CTTNCCTNCC CCTTTNCAGC CCAATTAAAG 300
GTTGGGGTCN TAACCTCAAA 320

SEQ ID NO:1005
SEQUENCE LENGTH:315
SEQUENCE TYPE:nucleic acid
TOPOLOGY:linear
CLONE:HUMGS01142
SEQUENCE DESCRIPTION:
GATCTGGGGG CTCGAGACTC TGAAGGCAGG GACCCTCTGA CCATCGCCAT GGAAACAGCC 60
AACGCTGACA TCGTCACCCT GCTACGACTG GCAAAGATGA GGGAGGCTGA AGCGGCCCAG 120
GGGCAGGCAG GAGATGAGAC GTATCTTGAC ATCTTCCGCG ACTTCTCCCT CATGGCGTCA 180
GACGACCCGG AGAAGCTGAG CCGTCGNAGT CATGACCTCC ACACGCTGTN ACCCGAGGCC 240
CACGGGGCCG CGCCTGCNTC CCTTCCCCGN NACCGNGCNN TCTGCCATTA AAGCCTCCGT 300
GCTTCGNTCT TCAAA 315

SEQ ID NO:1006
SEQUENCE LENGTH:315
SEQUENCE TYPE:nucleic acid
TOPOLOGY:linear
CLONE:HUMGS01143
SEQUENCE DESCRIPTION:
GATCCCTTGC NTGCCCCTCC CTGTGGCAGG GCTAACTGCC TGGCCCTCCT GGCTCGCAGC 60
CAGCCAGNCC CCTGGCAGCA GGTTCTCCTC AGGGCTTGGN TCTTCAACCT GTGGCGACAG 120
GAGGCAGGGC AGACTGTGGA GGACAGGATG CAGGTCAGGG AGAGGGAAGG CAGGGGTGGA 180
CCGCCATGAG CATGAAAAGC CCGAAGCAAG TTGACTCTTN AATTTGCAAC TGTTATGNTC 240
TGAAAATGAG AACGATGTAT CAANTTGATG CANTTTNGAT GTTGTACTTA CAATAANGTT 300
TTAATGTGTN TTAAA 315

SEQ ID NO:1007
SEQUENCE LENGTH:315
SEQUENCE TYPE:nucleic acid
TOPOLOGY:linear
CLONE:HUMGS01144
SEQUENCE DESCRIPTION:
GATCTGAGGN CAGGANTTAA ACCACAGAAT GTATNTGCCT GTAAAGCACA GGGGAAGAAA 60
CGACTCATTA GAACTACACC TGTTACATAC CATTCGGTAA ATGNTTTAAG NGGGGAATGG 120
TGTGACAAAC CTTCAAAAAA NATGAACACC TTAATGTTCA GGACTGAAGC TAACTCCCTA 180
TGNTTAGGCA CAGCTTGATA CGAGCGGAGA CTTGGCAGTC AATTCCANGT CTTTTATACT 240
NATTACCTCA TCGTNACTGT NAGTGCAACT ATAGTCTGTT GTNGGAATTT GGNCATCCCT 300
TAGTNTCNGA TGGTN 315

SEQ ID NO:1008
SEQUENCE LENGTH:314
SEQUENCE TYPE:nucleic acid
TOPOLOGY:linear
CLONE:HUMGS01145
SEQUENCE DESCRIPTION:
GATCAGAAAA AACAGAAGCC AAACTCGGGG TCATCTTTGT TTTTAAAGCT GAAGTGGGAC 60
TGTCTGGCAC TCTGTGTATT TATGCGTTCC AGCATCTGGA ACCTCCCATC CCTGCCCTCC 120
TCCTGTGTAG CTGCCACCTC CCCGCTGGGC CCAGCATGGC TCACCTGTCC CGTGGGCTGT 180
GTTTCTTGTT GTTTTTCTCT TTGCAAAGAC ATAGCTAGGA AAGCGAATGA TAAGGGAAAA 240
GTTCTCAGGG AATTGAAGTG TTGTTGCTAT GGTGACGTCC TTTTGCTGTG AATAAAGGTG 300
CTCTTTGCAG CAAA 314

SEQ ID NO:1009
SEQUENCE LENGTH:313
SEQUENCE TYPE:nucleic acid
TOPOLOGY:linear
CLONE:HUMGS01147
SEQUENCE DESCRIPTION:
GATCCACAAT AGTGGCATGA GGCAGGCGAC TGTNTGTNAC CTCTATGTCC GCAGAGTCCA 60
GCACGCTAAC AGCTGGGAGA TAAAAGCAGT GGAGAGGGCT GTTGGGGGAC ATGCCATGGA 120
AACTACCTAG GACCTGTTCC CTGAGTTAAC ATTCTAGCCT CATCTACTTG TNTTGCCCCT 180
GCAGCTCATA TACANACTGG CCCACCATTT ACGNACCATC CCCTCAAGTA ATCTTAAAGG 240
TTCTCAGCCC ANACANATTA ACTGTTCTGA CCCCACCTNC TTAATAAACA ATCCTGGGNT 300
CAGCCATNTG AAA 313

SEQ ID NO:1010
SEQUENCE LENGTH:311
SEQUENCE TYPE:nucleic acid
TOPOLOGY:linear
CLONE:HUMGS01148

SEQUENCE DESCRIPTION:
GATCTGGCAG TTGAAAATTG TGGGAAAGAG AATTTGTATG GGCACTGTAT CTATGAAATA 60
CCTCATAACT TACGTTTACA TGTTTTCCTA ACTTTTNGTA TTTTCNTNGT ATAGCCACCT 120
AGAGAATTCT TCATAGATTA AGAACTACAG TTTTNACCAC TTAACATAAG TAAAACAAAG 180
TCCTTCATAA TTNAACCATT AGCATCTTTG GCCAAACCAA AATAAAGANA AGCATCTNCT 240
CCTAGTTGTG TGTGGGCAAC AGANACANGT TAAGGNAACA NAAATACTTA TATATACACN 300
GANCANANGT N 311

SEQ ID NO:1011
SEQUENCE LENGTH:308
SEQUENCE TYPE:nucleic acid
TOPOLOGY:linear
CLONE:HUMGS01149
SEQUENCE DESCRIPTION:
GATCTCCCTT GGTCTTCCAT GGGATGGTTA GTGTGGAGGG GAGATATAGA TTGTCCGGCC 60
GNTTTGTGAT TCCATGGGAT TGATTCAGTC TTCTGGATTT TTTTTNCTGT ATATTTNGGG 120
TACTGGAGCT TTTAAAAATG CTTGGNTTCA GGTATTTTNA TTCATGTGAA GTGTATATGA 180
TTCTNTTGAG ATAAGGTTTT AAGCTAAAAT GTNACTCCCT GNTTNAGCNT CTGAACCCTG 240
ACAGATTNAC AGGGACTTTG CTGGTGTAGG CTTTTTAAAG GGNTTANTAN TCCACTTTGA 300
GCCTNAAA 308

SEQ ID NO:1012
SEQUENCE LENGTH:308
SEQUENCE TYPE:nucleic acid
TOPOLOGY:linear
CLONE:HUMGS01150
SEQUENCE DESCRIPTION:
GATCTGGAGT TACCTGAGGC CATAGCTGCC CTATTCACTT CTAAGGGCCC TGTTTTGAGA 60
TTGTTTGTTC TAATTTATTT TAAGCTAGGT AAGGCTGGGG GGAGGGTGGG GCCGTGGTCC 120
CCTCAGCCTC CATGGGGAGG GAAGAAGGGG GAGCTCTTTT TTNACGTTGA TTTTTNTTTT 180
TCTACTCTGT TTTCCCTTTN TCCTTCCGNT CCATTTGGGG CCCNGGGGGT TTCAGTCATC 240
TCCCCATNTG GNCCCCGGGA CTGTCTTNGT TGATTCTAAC TNGGNNNGGA AAGAAANTAT 300
TATTCAAA 308

SEQ ID NO:1013
SEQUENCE LENGTH:307
SEQUENCE TYPE:nucleic acid
TOPOLOGY:linear
CLONE:HUMGS01151
SEQUENCE DESCRIPTION:
GATCCATGGG GCCGCAATGA AGCTTGGAGA TACATGGGTG GCTTTGCAAA GAGTGTTTCC 60
TTTTCTGATG TATTCTTTAA AGGATTCAAA TGGGGATTTG CTGCATTTGT GGTAGCTGTA 120
GGAGCTGAAT ATTACCTGGA GTCCCTGAAT AAAGATAAGA AGCATCACTG AAGATAATAC 180
CTGGAAGCAT CATAGTGGTT TCTTAACTCT CCAAAATAAG ATTTCTTCTC TGTAGCCTAC 240
TTGTCTGGTT TATCCCTTAC AGAATATTAG TAAGATTTAA TCAATTAAAA TATATATATA 300

TGCCAAA 307

SEQ ID NO:1014
SEQUENCE LENGTH:306
SEQUENCE TYPE:nucleic acid
TOPOLOGY:linear
CLONE:HUMGS01152
SEQUENCE DESCRIPTION:
GATCGGTCCA CGAAAGTTAG CCCATATGTA TATCTTGAAT AGTATAGGGG AGGGTATTCA 60
TAAAGTCCTT ATGTGGTTTT AACTAAGTGA AATTATGGAC AAGAGAANNN NTTGTAAAAT 120
CGTCTTAAAG GCAAATTTAA TTTTNACTCC TGTTTATGGG ACATTCGTTC TATTAACTGT 180
CAGACACAAT TTCTGTTTTC ATCTGAGAGC CAGTTTTCCT TTATTTCTAC ATCTAAAATA 240
AGANCATATT GTACACTATT ATATAATACA GAATTGTCTT AAACTTTAAT AAATTCGCAT 300
TTTAAA 306

SEQ ID NO:1015
SEQUENCE LENGTH:306
SEQUENCE TYPE:nucleic acid
TOPOLOGY:linear
CLONE:HUMGS01153
SEQUENCE DESCRIPTION:
GATCTCAGCA GTGGGGCAGG AGGGTGCCTG ATTTCGGGGA GTCCTGACCC GAGCCTGTTG 60
TCAGAGTTGG GAGGGGCTCT GAGCAGTGTT GGGCAGGCCG GGTCTCCCAT CCCGAGGCCA 120
GCGTTCCTGT GCAGAGCCCC ATCCACTGGT TCTTGCCCTG AGCCACATAT GTCTGTNCCA 180
TGGGCTGAGT GCCACGACAG GCCCGTGTGA CAGCTGCTGC CCACGCATNT NGAAGCTAGG 240
TGGGACTCAT TCCTAATTCT GCCGTTGTAA TGAGACTTGA TTAAAACACC GCCACTTTTT 300
TGCAAA 306

SEQ ID NO:1016
SEQUENCE LENGTH:304
SEQUENCE TYPE:nucleic acid
TOPOLOGY:linear
CLONE:HUMGS01154
SEQUENCE DESCRIPTION:
GATCAACAAG AAATGTTATG AAATGGCCTC CCACCTTCGG CGTTCCCAGT ACTGACCTCG 60
TCTGTCCCTT CCCCTTCACC GCTCCCCACA GCTTTGCACC CCTTTCCTCC CCATACACAC 120
ACAAACCATT TTATTTTTTG GGCCATTACC CCATACCCCT TATTGCTGCC AAAACCACAT 180
GGGCTGGGGG CCAGGGCTGG ATGGACAGAC ACCTCCCNNT ACCNATATCC CTCCCGTGTG 240
TGGTTGGAAA ACTTTTGTTT TTTGGGGTTT TTTTTTTCTG AATAAAAAAG ATTCTACTAA 300
CAAA 304

SEQ ID NO:1017
SEQUENCE LENGTH:304
SEQUENCE TYPE:nucleic acid
TOPOLOGY:linear

CLONE:HUMGS01155
SEQUENCE DESCRIPTION:
GATCTAAAGC AGGTNGTGTT GTTTACATGT TTCTACACAT TTCATCCTTT AAAAAGTTGT 60
TGAGAGAGGT TGTATTTACC TTCCCAAGGT TGGAAAGCAG GGGAATTTCC CAGTGTCCTA 120
GTTTTCCACC AGAGGAATAT GTGTAAGTAG CAAAGTATTT GCTGCTTACA TATAGTGTGT 180
ATGTATGTAT ATATGTAAAT NGTGTGTTAA AGAGCTGATA CTGATTTTCA TATGNCAATG 240
TTAAGGCAAA GGCCTCCCTG CATTTGANGA GCAGGTNTTC ATTTATATGT ATTTTNGGGA 300
TAAA 304

SEQ ID NO:1018
SEQUENCE LENGTH:304
SEQUENCE TYPE:nucleic acid
TOPOLOGY:linear
CLONE:HUMGS01156
SEQUENCE DESCRIPTION:
GATCAGAAAT NAAGTGCAGC AATATCATGA ATTCTNAGAA GNCTTTAAGG GAGCCAGTNA 60
GTCATACAGT ATCCACAGTT GANTCACTTA AAGATGTCAG TATACGAACA TTATTCACAA 120
TCCTTGGGCA ATCTCATTTT TTTTCCCTTC TCCCCTCCTC CCCTGCCCCC ATACATTTNT 180
ATCCTTAANG TAGTTTTGGA GGGGCAGGAT GTACTTAACA TCTCANAAGC TAGATTGGGA 240
ACATNTCANT ATAAGACTGA GTTTAAATTT ANGGTTAAAA TGNCATCAGA ANANTTGGGN 300
GGGN 304

SEQ ID NO:1019
SEQUENCE LENGTH:169
SEQUENCE TYPE:nucleic acid
TOPOLOGY:linear
CLONE:HUMGS01157
SEQUENCE DESCRIPTION:
GATCTAATAC TACTGTCAGT TTTAATGTGC ACTGTGTTTT ATACAGTATC TTTTTTTGTT 60
CACTTNGGAA ATTTTTACTA AAAATTGCAA AAAATAAAGT ATTGTGCAAA GATGTAAGGN 120
TTTTTGANAC TTGNNGTGCA TTAATAANTA GACGATTAAN TCAAGGAAA 169

SEQ ID NO:1020
SEQUENCE LENGTH:303
SEQUENCE TYPE:nucleic acid
TOPOLOGY:linear
CLONE:HUMGS01158
SEQUENCE DESCRIPTION:
GATCCTCTAC CACAGACATT AATAGCTGAG CAGGAGCCAC ATGGATTGAT TGTATCCACT 60
CACCATTGAC GATGGCATTG AGCGTANTAG CTTATTTCCA TCACTACGTG TTTTTGAGCT 120
TGCTCTTACG TTTTAAGAGG TGCCAGGGGT ACATTTTTGC ACTGAAATCT AAAGATGTTT 180
TAAAAAACAC TTTTCACAAA AATAGTCCTT TGTCATTACA TTATTTACTC ATGTGTTTGT 240
ACATTTTTGT ATGTTAATTT ATGAATGATT TTTTCAGTAA AAAATACATA TTCAAGAACC 300
AAA 303

SEQ ID NO:1021
SEQUENCE LENGTH:303
SEQUENCE TYPE:nucleic acid
TOPOLOGY:linear
CLONE:HUMGS01159
SEQUENCE DESCRIPTION:
GATCCTTNGG CTCAGAATCT CGAGAACCGC CTGCTCCTAA CAATTCAGCA AGTCAGGGGC 60
TTCCTCTCTG TTAGTCCCCA AATCCTTACT TATTTTAAAA AGACTAGACC CTCTCTAAAG 120
ACTGTTCCAT TTTAACATGT CCTGATTCTG CATCCGTGGG TTTTGTGAAA GAGAGCTAGC 180
TGGCGGTTAG AGCCTGGAAG AAGGAGGGAA GTGGCACCTC ACTAGCATTT ATCACTTTTT 240
TCCTTCTCTT TTTAAAAATA AAACCAGACT CTGTTCTGAA AATAAAAAAC TTGAGACTTG 300
AAA 303

SEQ ID NO:1022
SEQUENCE LENGTH:303
SEQUENCE TYPE:nucleic acid
TOPOLOGY:linear
CLONE:HUMGS01160
SEQUENCE DESCRIPTION:
GATCTGNCTT ATCCGAGCTT GTTATTGGGG AGCCATAAGA GTCAGTTATC CAGAACACAG 60
TTTTGCATAA GCTTGTTTAT GATTCAGTAA TGCAGGTGAG AGTGTCTAGC AGTTCTTGGT 120
AAGCTACTCT GGACATCTTT AAATTATTTA TCCTAATGGA TTCCATTCTG GTTTATGTAT 180
AATCGTTTCA AGACTTTGGG AGTCTTTTAT GAACAAATGC TCATTGCACT ATATTATATG 240
CAAATTGTNN NGCTGCTAGG TTTTCAAAAT TTGAATAATA AAGCCTTTTC ATGTTCTTTT 300
AAA 303

SEQ ID NO:1023
SEQUENCE LENGTH:303
SEQUENCE TYPE:nucleic acid
TOPOLOGY:linear
CLONE:HUMGS01161
SEQUENCE DESCRIPTION:
GATCAAAATC CTGTACAATA CTATAAATAT ATATTNATNT TTTCACAGTC ACCAAGTGTA 60
TTGTAATGTA TACTTGAAAA ATGTTATAAC TTATGAAGTA AAGTTTCTNA TAGTAGTCTT 120
TAAAAGATAT AAGACTTAAT ATGTTTTATT CAGCTTCTAT AAGTGTGACC AGTTTTNATA 180
TTTATTTATG CTAATATTTT TAACAAGTCA TTTCAAAATA TGTGTATCTC AAATCCTCCC 240
NAAAGTGTTG TGGCCTTAAC TGTTCAGTAT TGCAATAAAA NATATATNTN NNTATGTGGT 300
AAA 303

SEQ ID NO:1024
SEQUENCE LENGTH:302
SEQUENCE TYPE:nucleic acid
TOPOLOGY:linear
CLONE:HUMGS01162
SEQUENCE DESCRIPTION:

GATCTATGGA AAACTGGGAA TGTAATGTAG GATTCTGTCA GAGCTCCTAC AGAGCACAGT 60
TGCCTTTAGT TTCCTTTAAA GATGTAAAAA TATTGTATAA TACAGTTTTG TCCCTACACA 120
ATTGTATTTG CCAAGCTTAG TGCATTATGA TACCTTTATT TATTTGTTTT GGGCAGTATT 180
ACTATATATA TATAANCATA CAGTTACTGT TTTATATATT CTTAGGTCAT TCAAAGCCAT 240
GTATGCTGTA AATGTGCTAG TCTTTAGAAT GACACATAAT AAATAACTGA CAAGATATTA 300
AA 302

SEQ ID NO:1025
SEQUENCE LENGTH:435
SEQUENCE TYPE:nucleic acid
TOPOLOGY:linear
CLONE:HUMGS01163
SEQUENCE DESCRIPTION:
GATCGAGCCA TTGAAAGCTC ATTACCAGTA GGACATAATT TTTGGCTCTC CCTATTCACA 60
ACCAGTGCAC AGTTTGACAC AGTGGCCTCA GGTTCACAGT GCACCATGTC ACTGTGCTAT 120
CCTACGAAAT CATTTGTTTC TAAGTTGTGT TTATTCCTGG AGTGACATGC CACCCCGAAT 180
GGCTCACTTT CACTGAGGAT GCTGTCCTCT GATTTAGCTG CTGCCTCCAG CCTCTGGCTT 240
GAGAACTTAC TAAAGGCACT TCCTTCCTGT TAAACCCCTG TTAACTCTCC ATAAATTTGG 300
TGATTCTCTG CTAGGCCTAA GATTTTGAGT TAACATCTCT TGAAGCCAAA CTCCACCTTC 360
TGTGCTTTTT TGCTTGGGGA TAATGGAGTT TTTTCTTTTA GGAAACCAGT GCCAAGGAAT 420
GNCAAAGGTN TTAAA 435

SEQ ID NO:1026
SEQUENCE LENGTH:298
SEQUENCE TYPE:nucleic acid
TOPOLOGY:linear
CLONE:HUMGS01164
SEQUENCE DESCRIPTION:
GATCCACATC CTCTACAGGT CGGGGACCAA AGGCTGATTC TTGGAGATTT AACACCCCAC 60
AGGCAATGGG TTTATAGACA TTATGTGAGT TTCCTGCTAT ATTAACATCA TCTTAGACTT 120
TGCAAGCAGA GAGTCGTGGA ATCAAATCTG TGCTCTTTCA TTTGCTAAGT GTATGATGTC 180
ACACAAGCTC CTTAACCTTC CATGTCTCCA TTTNCTTCTC TGTGAAGTAG GTATAAGAAG 240
TCCTATCTCA TAGGGATGCT GTGAGCATTA AATAAAGGTA CACATGGAAA ACACCAAA 298

SEQ ID NO:1027
SEQUENCE LENGTH:328
SEQUENCE TYPE:nucleic acid
TOPOLOGY:linear
CLONE:HUMGS01165
SEQUENCE DESCRIPTION:
GATCCACTTT GTTGGTTGTT GTTGCAGAAG ACTGAACTGT TTTGGAATAN TTAACAATTA 60
CAGAAACAGT CAAGTGTTTT CCAATGTGGT TGTCCGGTTT CTATGGCCTT GCTGTGTACT 120
TTCCCTCTTT TTGACAGTAA ACTTCTGCCT ATGGCTTACA GTTTGACATT TAATTTATTA 180
GCGCTGCTCT GCACCCCTNC CTTGGGAGGG AGACTTCATG TGGTTTATTG CGAGTTTTTT 240
TGTTTACTTT TCAGGGTTNG TACCTACAAA GGTTTTAATA ATAAAAANCA AAGNTTTTTT 300

NGGCNATTNG TCTTGTCTTN GTGGGAAA 328

SEQ ID NO:1028
SEQUENCE LENGTH:297
SEQUENCE TYPE:nucleic acid
TOPOLOGY:linear
CLONE:HUMGS01167
SEQUENCE DESCRIPTION:
GATCTACAGT GCTGATGAGA AGAGAGCCCT TGCATCCTTT AACCAAGAAG AGAGACGAAA 60
GAGAGAGAAC AAGATTCTGG CCAGTTTTCG AGAAATGGTT TACAGAAAGA CCAAAGGGAA 120
GGATGACAAA TAAAGATTTT NTGATTGTCC AGAAGACATT TTTAACAACA AAAAAGAAAG 180
TCTGGGTTCC ACACATACAT AGAAAAAGAT TATTATGTTC TGAGAAAGCT TTACAGTGCT 240
ACTGTGCCTT CTATTTAATT CTTTCAGTCC TTCAATAAAA AGCTGCTTAT TGATAAA 297

SEQ ID NO:1029
SEQUENCE LENGTH:297
SEQUENCE TYPE:nucleic acid
TOPOLOGY:linear
CLONE:HUMGS01168
SEQUENCE DESCRIPTION:
GATCCTGCTG AAATACATCT GCAGCTGACA ATGAGAGAAG AAACAGAAAA TGTCATGTGA 60
TGTCTCTCCC CAAAGTCATC ATGGGTTTTG GATTTGTTTT GAATATTTTT TNCTTTTTTC 120
CTTTTCCCTC CTTTATGACC TTTGGGACAT TGGGAATACC CAGCCAACTC TCCACCATCA 180
ATGTAACTCC ATGGACATTG CTGCTCTTGG TGGTGTTATC TAATTTTTGT GATAGGGAAA 240
CAAATTCTTT TGAATAAAAA TAAATAACAA AACAATAAAA GTTTATTGAG CCACAAA 297

SEQ ID NO:1030
SEQUENCE LENGTH:296
SEQUENCE TYPE:nucleic acid
TOPOLOGY:linear
CLONE:HUMGS01169
SEQUENCE DESCRIPTION:
GATCCAGAAC ATGGGAAGTT AGGGAAAATG TGTGATTTTG TGTTTTGAAT TACTGTCAGA 60
ATTACATACA CAATTACAAC AAACTTTTTT TAAAAGACAT TTCATTGTAC TGCAAAAATC 120
TGAATATTTA TATTTCTNGT TTTTTTCTTT ATATGTTTTG CATTTTANTA TGTTGAGCCA 180
CTGGAAAATT TGTAACAGNT TANTTTGTTA TNGGCGTTTA ANTGTGTTGT CATTGNCTCC 240
ATTGTCTTTG TCCAGAGCCT ATTATTATGG AACCAATAAA NTTTAATGGG GTCAAA 296

SEQ ID NO:1031
SEQUENCE LENGTH:294
SEQUENCE TYPE:nucleic acid
TOPOLOGY:linear
CLONE:HUMGS01170
SEQUENCE DESCRIPTION:
GATCCTAGTC CCCTGCCCTC TGGCACAGCT GCTTCCTGCA AGANAGCAAG TNTTTGGTCT 60

CCCTGAGAAG CCATGTCCCT CGTNCTGTNT CTTGCCTGTC CCACCTGTGC CCTGCCCTCC 120
AGCTTGTATT TAAGTCCCTG GGCTGCCCCC TTGGGGTGCC CCCNGCTCCC AGGTTCCCCT 180
CTGGTGTNAT GTCAGGCATT TNGCAAGGAA AAGCCACTTG GGGAAAGATG GAAAAGGACA 240
AAAAAAATTA ATAAATTTCC ATTGGCCCTC GGGTGAGCTG AGGGTTTTTG CAAA 294

SEQ ID NO:1032
SEQUENCE LENGTH:293
SEQUENCE TYPE:nucleic acid
TOPOLOGY:linear
CLONE:HUMGS01171
SEQUENCE DESCRIPTION:
GATCTTCAAG AAGTGAAAGA GACCGAAAAT CAGACAGGAA AGACAAAAGG CGTTAATGGA 60
AGAAGCCAGG CTTTCTTAGC CATTCTTTGC AGCAGAAGAT TTCTTGATAA AAAAGGATTA 120
CCTTTCCTTG TAAAGAGGAT GCTGCCTTAA GANTTGCATG TNGTAAAANN NCTTTTTGGA 180
AAATACAGAC TGTTTGTTTA CCAGACATTC TNGTACTGNT NGCATAATNN GGTAAGAGTT 240
ATTNATCAAA ATNATGTGAG GTTCCAAAAT ATGTAAAANT GATATNATAA AAN 293

SEQ ID NO:1033
SEQUENCE LENGTH:293
SEQUENCE TYPE:nucleic acid
TOPOLOGY:linear
CLONE:HUMGS01172
SEQUENCE DESCRIPTION:
GATCAGCGGT TCTTTTTGCA GCAAAGCCTG CATCTGTGTT GACTTGCAAG ATTTTGCGTT 60
TATTCAGGCA AAAACTGGTC AAAATGGTTA CTACATGATT TGTTCCCAGA GGTTTGAAAC 120
ATTCAGTGAA ACTTTTTAAA ACTTTGATTG CATGATGTAT TTTTTTTTNA GAAAGTTATT 180
GTTTGAGAAT AATGTCTTTT TATACCAGGA AAATAGTTAT CCNGAATGAC GTTGAAAACT 240
CCCCCTCCCC TTNATTTTTN TTTAATCANT ACATGTGAAA GTNNCCANGC AAA 293

SEQ ID NO:1034
SEQUENCE LENGTH:308
SEQUENCE TYPE:nucleic acid
TOPOLOGY:linear
CLONE:HUMGS01173
SEQUENCE DESCRIPTION:
GATCCCAATT CCACATAAGC ACTTTTGGAA GAAAACAGCC AAAGTTGGCC TAAAATTGGC 60
GCTGGAATTT GGNCTGGGAA AAATCTTGTG GTTATTTCCT TTAAAAAGGA ACAAAACTTT 120
AGTATTTAAT TAGTTGATTT ATTTAATGTA ATTNCAAACA ATTAAATTAT GAATAATGCA 180
ATGTACAGTA GAATCACGTT TTGATTTTAT TAACACTGAC CAAGTTTAAC TCCATATGAN 240
GTGTAAGCTT GATATCGTTT ATGATGTCTA TCAACTGTAC CAAAAGTAAA ACATTTAAAA 300
NCANNAAA 308

SEQ ID NO:1035
SEQUENCE LENGTH:292
SEQUENCE TYPE:nucleic acid

TOPOLOGY:linear
CLONE:HUMGS01174
SEQUENCE DESCRIPTION:
GATCTCAAGT GACCCGCCTG TCTCGACCTC CCAAAGTGCT ATGATTACAG GCATGAGCCA 60
CTGCACCCAG CCAAACATGA CTTTTCCATC CAGAGTAAAT CCAACTAACA AGAATCCACC 120
CTTGGAGTTC ATGTAAAAAT ACATGACACA GGGTGATGAA AGTGCTTTGA AACTAGATAC 180
AGGCAGTGGT TCTATAGCAT GGTGAATGTA CTCAAGGCAA CTTCTTTACT TTAAAATCGT 240
TAATTTTATG CCATGTGAAT TGCATCTCAA TAAAAATTGT TTCATTTTA AA 292

SEQ ID NO:1036
SEQUENCE LENGTH:363
SEQUENCE TYPE:nucleic acid
TOPOLOGY:linear
CLONE:HUMGS01175
SEQUENCE DESCRIPTION:
GATCGGGAAC TCCTGCTTCT CCTTGCCTCG AAATGGACCC CAACTGCTCC TGCTCGCCTG 60
TTGGCTCCTG TGCCTGTGCC GGCTCCTGCA AATGCAAAGA GTGCAAATGC ACCTCCTGCA 120
AGAAGAGCTG CTGCTCCTGC TGCCCTGTGG GTGTGCAAGT GTGCCCAGGG CTGCATCTGC 180
AAAGGGACGT CAGACAAGTG CAGCTGCTGT GCCTGATGCA GGACAGCTGT GCTCTCAGAT 240
GTAAATAGAG CAACCTATAT AAACCTGGAT TTTTTTTTTT TTTTTTTTNN AAAANCCCTG 300
NCCNNTTTGT AAAATTTTTT TTTNNNTGAA ATANGNAANG GNAATAATTN ATCNGGNNTN 360
TTN 363

SEQ ID NO:1037
SEQUENCE LENGTH:288
SEQUENCE TYPE:nucleic acid
TOPOLOGY:linear
CLONE:HUMGS01176
SEQUENCE DESCRIPTION:
GATCTTGGAA AGCACTAGAA ACTAAACATC TTCACCAGGT GCTGAAGAAA AGTGTCTTCG 60
TTTTAATTGC CAAGCAGGGA TGTGGACATT TGGATGGTGA CTTTCCTGGG TGGTTCCCCA 120
TAGATTCACC ATTGCCTCTA ATGGTGTCTA CACCCGTCAT ACTACCAGCT GAGATGGTGG 180
TGGGCATAAG GAGAATTTGT GCCTATAACC CTTAGTGTGT TCTGGTTTTT TTTCTTTTAA 240
TTTTTAAATT GTCGTAAAAT ACTCATAAAA CATACTGTCT TCACCAAA 288

SEQ ID NO:1038
SEQUENCE LENGTH:288
SEQUENCE TYPE:nucleic acid
TOPOLOGY:linear
CLONE:HUMGS01177
SEQUENCE DESCRIPTION:
GATCACCTTG TAGTCTTTAA ATTCTTGGTC CCTGAGGCCA AGTCCACAAC TTGCCTTCTA 60
GTCACTTGCC TGCCCGCAGT GGTGGTGGAT GTGTTAGCTG GTAGATTTGG AATCAGTCAC 120
CAGTCTTTCT GTACTGTCTT GGTTAGCTCT ATATAAGTAG GGGCAGCTTA GCCCTGAGGC 180
CCAGAGACCT GCTGTCCTTT TTCTCCTTGA GGGAGGAAAT AAAACTGCGG AATACAATGT 240

CCTTCCATAG CATGGGAAGA AGAAAATAAA CATCTCCTTT CCAACAAA 288

SEQ ID NO:1039
SEQUENCE LENGTH:288
SEQUENCE TYPE:nucleic acid
TOPOLOGY:linear
CLONE:HUMGS01178
SEQUENCE DESCRIPTION:
GATCCACCCT CCTGTAAATA TGGAACAAAT ATCTGAATGA AATCCACCCT AGGAGACGGA 60
GCAAACTAAA CTTGTGGTTT TNCATTTAAC TTTTGACTAC AGCATGGCCC CATGGCATCC 120
ACACCAAGAG GGTGTTGTGA TGAGGTGCCG GTGTGCAAAG GGAACTTTAG TTTTTCCACT 180
GGTTCTTATC TGCTAGCCTT TTACATACAT GTGTACTATA TTTGTTTATA GACTGTAGGT 240
GGATATATAA TTTAAAAGCT TGATTTAATA AACATTTAAC CCCNTAAA 288

SEQ ID NO:1040
SEQUENCE LENGTH:287
SEQUENCE TYPE:nucleic acid
TOPOLOGY:linear
CLONE:HUMGS01179
SEQUENCE DESCRIPTION:
GATCTTCAAG TATTTGCNAC TTTCGAGTAC AGCTAATTGG ATAATCTCAA ACCCTTTAGT 60
GAAAATATCT TAAATGCATT GAGAATATTT CCTAATTACC TGTGTATGCT ACAGTACAGA 120
CATTAATTCT ATAAACATGT TCATAGGTCT TCCCCCTCGC CCCGNCCGTC TTCTAAGGGC 180
ATTTCCTGTT TCTNTTNAGT GAGTTCATGN ATGTTTACCG GTTCTGGCGN AANGTTTCTT 240
GCATNCTGAG CATAAAAATA NTAAAACCNA CTGATANTTG CTTGAAA 287

SEQ ID NO:1041
SEQUENCE LENGTH:289
SEQUENCE TYPE:nucleic acid
TOPOLOGY:linear
CLONE:HUMGS01180
SEQUENCE DESCRIPTION:
GATCTGCACA CGCATAATAA TCAGCATTGA GGGCAACAAA ATGCCATTGT GACCTTGCCT 60
GGAATGTGTC CCCATCTCTA CTCTAAGAAA TGCGCAATGG ACTCTTTGGA GAAAGAAGAT 120
ATTTTAAAAC ATTTTTAGTG TGTCTGTAAA TGGTTCAGCG TGTATCAGAT GTTGTCATAG 180
GACTCACATT TCTCTCAGTT ATATTTAAAA CCGTTGTGTA CTTTGTACAA NGGAATACTA 240
GTCATACTTC TATAAACTTT NCACAATAAA ATTNTCATTC TGGGTTAAA 289

SEQ ID NO:1042
SEQUENCE LENGTH:287
SEQUENCE TYPE:nucleic acid
TOPOLOGY:linear
CLONE:HUMGS01181
SEQUENCE DESCRIPTION:
GATCGCACCA CTGCACTCCA GCCTGGGNAA CACAGTGAGA CCCTCTCTCA AAATAAATAC 60

AGNCAGGCAT AGTGGCTCAT GCCTGTAATC CCNGCATTTT GGGNGGTAGA GGTGGGTGAA 120
TCACCTGAGG TCAGGAGTTC ANGCCCAGCC TGGCCAACAT GGCGAAACCC CATCTCTACT 180
AANTATACAN AANTTAGCCA GGTGTGGTGG CCTGCACCTG TAATCCCAGC TACTCAGGAG 240
GCTGAGGTAG CTTGANCCCN GGAGGCANAN NTTGCAGTTA NGCCAAN 287

SEQ ID NO:1043
SEQUENCE LENGTH:286
SEQUENCE TYPE:nucleic acid
TOPOLOGY:linear
CLONE:HUMGS01182
SEQUENCE DESCRIPTION:
GATCTCTCCT GGAGTATGAA GACCTCCAAG GACTCACTTT CCCTCCCTTC TGATGCCAGA 60
GCAGACCAAG CTGTCACACT CCAGTCTCAT GCTGAAGTCT CCAGCTTCTC AAGCTTAGAA 120
GAGTTTTTNG AAGAGTCACT TTCAGCTCAT GCAGCTCTCA CAAGTGTGAA GGGAGTGGAT 180
TGGGGGTGTT TTCCTTGCCA TTTTCGAAAA GAAAAAAATT ACCTGGTGAT TGGTGGAAAG 240
ATACAACTGT CAAAAATGCA TGATTGAAGC AATTTAGGTT GGGAAA 286

SEQ ID NO:1044
SEQUENCE LENGTH:286
SEQUENCE TYPE:nucleic acid
TOPOLOGY:linear
CLONE:HUMGS01183
SEQUENCE DESCRIPTION:
GATCAATAAA TNATACCAAA TATATGTTTA CAGTATGATT TAAAGTCTGA TTCAGACCAG 60
GGACTCTATT TTAAGTTCAA CTGAAATAAC ACTGGGTTTT AATTATATCA CAGGAAAAAA 120
AAAGTGCATT TAAGTATTGT NATCGTGGAC TTTATAAAAG CAAAGGAAAT TGAAAGTAAC 180
TTTNGATTCT GTATCANGAA TCATATTTNC ATACAGTCAT AACTGTCTTN CTGTGACCCT 240
TTCACAGGGC ACTGTAGGAT GGATTAAAGG TGGCAATTTA CTGAAA 286

SEQ ID NO:1045
SEQUENCE LENGTH:439
SEQUENCE TYPE:nucleic acid
TOPOLOGY:linear
CLONE:HUMGS01184
SEQUENCE DESCRIPTION:
GATCATACCG GTAAAGCAGG AATNACAAAG CTTGCTTTTC TGGTATGTNC TAGGTGTATT 60
GTGACTTTAA CTGTTATATT AATTGCCAAT ATAAGTAAAT ATAGATTATA TATGTATAGT 120
GTTTCACAAA GCTTAGACCT TTACCTTCCA GCCACCCCAC AGTGCTTGAT ATTTCAGAGT 180
CAGTCATTGG TTATACATGT GTAGTTCCAA AGCACATAAG CTAGAAGAAG AAATATTTCT 240
NGGNGCACTA CCATCTGTTT TCAACATGAA ATGCCACACA CATAGANCTC CANCAGCATC 300
ANTTNCATTG CACAGACTGA CTGTNGTTAA TTTNGTCACA GNGTCTATGG ACTGANTCTA 360
ATGCTTCCNA AANTGTTGGT TTGTTTGCAN GTTTTCGANC CGTTGTTATG GCANGANGTT 420
NGTTTAGTTT CNGNTTGTN 439

SEQ ID NO:1046

SEQUENCE LENGTH:285
SEQUENCE TYPE:nucleic acid
TOPOLOGY:linear
CLONE:HUMGS01186
SEQUENCE DESCRIPTION:
GATCTGGAAA CAGTTGAAAA CGAGACAATA TAGCCGGAGA CGCCTTATAT GATGATGCAG 60
TATCGACTAC ATTAATGAAA GTAATGCCAA ATNCAACAAG AAAGCTGAAG ATTCTATGGG 120
AATACACAGC TGAATTAACA GATTTGGAAG AGAACANCTG NTAATCCTTC AGGACTGTTA 180
TAGAGTTNAG ATGGGTAAAT TCTCCTANAA ATCAAGTCTT TTGAATTTNC AGAATCAGAN 240
TTAGAGCCNG CTCTACTAGA TTGNATAANT GNGGTCTAAC GGAAA 285

SEQ ID NO:1047
SEQUENCE LENGTH:285
SEQUENCE TYPE:nucleic acid
TOPOLOGY:linear
CLONE:HUMGS01187
SEQUENCE DESCRIPTION:
GATCCGGGNG GACCCCTTTG CCCTTCCCTC GGCTCCCAGC CCTACAGACT TGCTGTGTGA 60
CCTCAGGCCA GTGTGCCGAC CTCTCTGGGC CTCAGTTTTC CCAGCTATGA AAACAGCTAT 120
CTCACAAAGT TGTGTGAAGC AGAAGAGAAA AGCTGGAGGA AGGCCGTGGG CCAATGGGAG 180
AGCTCTTGTT ATTATTAATA TTGTTGCCGC TGTTGTGTTG TTGTTATTAA TTAATATTCA 240
TATTATTTAT NTAATACTTA CATAAAGATT TTGTACCAGT GGAAA 285

SEQ ID NO:1048
SEQUENCE LENGTH:283
SEQUENCE TYPE:nucleic acid
TOPOLOGY:linear
CLONE:HUMGS01188
SEQUENCE DESCRIPTION:
GATCGAAGTG ACAAAGTGTG TTTTCANTCA CAGTGGAGGC TACATCAAGC AAGGGGAGGT 60
CCAGCCCTCT TGCAAGTGTG GTGAGAGGCT CTACTAGCAA AGACATGGGC ACCGGAGTAG 120
GTCCCGTGTA GCATGCGGGT GCTGTAGAGA AAATTCAGTG ACGTACATGG CTCTGGTTCT 180
GGACACAAAA TCTGTACTGG AGAGGAAATG ACTGCTGAAA TAAGGCGATT GTATGAATAT 240
TTAAAATGCC TGGAACACTA AAGTAAAGTA ATGATATTTC AAA 283

SEQ ID NO:1049
SEQUENCE LENGTH:283
SEQUENCE TYPE:nucleic acid
TOPOLOGY:linear
CLONE:HUMGS01189
SEQUENCE DESCRIPTION:
GATCCTCGCA GCTTCCTNCG AGCGGGGTGT CGCAGTCTTG TGCACAGAGT AAACTTTNCT 60
AGCTGCCCCT TTCTGTAATA GTGAAAGTTG GTATTTAACA TTTATNCATT TTTAAAATAT 120
TTGGAAGGTC TGANCTTGTG AAAAGAAAGT GGTTGGNCTG AGGTTGGAGG NAGCTGAATG 180
GAATCTNACG GTTGGNAGTG GTGGAAATTG GAAGGATACC AGGAGGTATT TGGGAAGGCC 240

AATGGCGTGG CTCCTTTGAG GAAATAAAAC ACTAAGCATG AAA 283

SEQ ID NO:1050
SEQUENCE LENGTH:281
SEQUENCE TYPE:nucleic acid
TOPOLOGY:linear
CLONE:HUMGS01191
SEQUENCE DESCRIPTION:
GATCCTTTTC ATACCTNATT TGGAATTGCT GGATTGTAAC TTTTGGNAGA AGAACAGATT 60
AAACCTGTTA ATCCTGTCTT TTGCATGCCT GAAGAAGTGC TTCAAAGAGT GAATGTTCAG 120
CCTGAGCTAG TGAGCTAGAT TCATTGAATT GAAAGTTGCA TAGTATAGTT TTGCCATTTT 180
AACATTTCTG TATTTNAAGT GCTTATCGAA TCTAAAAGTG ACTACTGTNA ATATTNNGTA 240
TATNGTGTNA AATTAATTNN ANTAAATNAT ATAATTNTAA A 281

SEQ ID NO:1051
SEQUENCE LENGTH:226
SEQUENCE TYPE:nucleic acid
TOPOLOGY:linear
CLONE:HUMGS01192
SEQUENCE DESCRIPTION:
GATCAATGAC AGAGCCTTCT GGAGGACATT CCAAGACAGT ATACAGTCCT GTGGTCTCCT 60
TGGAAATCCG TCTAGTTAAC ATTTCAAGGG CAATACCGTG TTGGTTTTGA CTGGATATTC 120
ATATAAACTT TTTAAAGAGT TGAGTGATAG AGCTAACCCT TATCTGTAAG TTTTGAATTT 180
ATATTGTTTC ATCCCATGTA CAAAACCATT TTTNCCTACA AATAAA 226

SEQ ID NO:1052
SEQUENCE LENGTH:277
SEQUENCE TYPE:nucleic acid
TOPOLOGY:linear
CLONE:HUMGS01193
SEQUENCE DESCRIPTION:
GATCACAGCA TAAAAGAATC ATAAGATAAA ACATCAAACT ACCCAGCAAC CTGAGAAGCA 60
CAGAGTGTTA AAGCCTCCAC CGTGTGAGAA ACTAAATTAG GGTAACTAGC TATTGAGTAT 120
ATTGAGTACC TTCAAAGCAC TCAACTGACA GGTTTTACAG ACTGGAAATT ATAATACTTA 180
TGACATTTCT ACCTTTTATA TAACCAATAA TCTACCATAG AATGTAGTAT TTTTANAGCT 240
ATTAGCANGC AATATATTAN NNTANTANTG NATTAAA 277

SEQ ID NO:1053
SEQUENCE LENGTH:277
SEQUENCE TYPE:nucleic acid
TOPOLOGY:linear
CLONE:HUMGS01194
SEQUENCE DESCRIPTION:
GATCCAACCC GAATAAGATT AGAAGCTTTC CATCAGTAAA AGGATGTTTT CTTTTTTCAC 60
ACAGTAAAAA TTCTTATCAT TCAAGGATAT TGGAACCACA GGACTATTTG GATAAAAAAC 120

ATTATTTGCA AATTAATGCG CATAGGCCAT CTTACTTTTA TTGCAAAATG GCATGTGCTG 180
CCATCTATTA TTCATTTTTA AATGGTCATT TCTTATTCAG TGAGTGCTTT AGTGTTTTAA 240
ACTATATGGA TAAGAATGCA GGTAGGATAA TATTCTN 277

SEQ ID NO:1054
SEQUENCE LENGTH:283
SEQUENCE TYPE:nucleic acid
TOPOLOGY:linear
CLONE:HUMGS01195
SEQUENCE DESCRIPTION:
GATCCTGAGG ATTACAGCTG TGGAATTTTT GTCCATGCTT CAAATAATTT TGAAAGAAAT 60
TTTCCCATAT NAAAAAAGGA GAGAACACTN GCATCTGTTG AAATTTGGAA NTTCTGAAAT 120
NATAGTATTT TTAAAAATTG CACTGAAGTG TATACACATA AAGCAGGTCT TTTATCCAGT 180
GAACAGGATG TTTTGCTTTA GCAGCAGTGA CATAAANTTC CATGTTAGAT AAGCATNTGT 240
TNACTTACCT NGTTATTAAA TATTTNTTGG AAAAGCAGTG AAA 283

SEQ ID NO:1055
SEQUENCE LENGTH:277
SEQUENCE TYPE:nucleic acid
TOPOLOGY:linear
CLONE:HUMGS01196
SEQUENCE DESCRIPTION:
GATCTGTGTA TGCTGTTGGG TCGGAGTGCC AGTNACTGCT TTGGAAGTCT GNGTTCTGGG 60
GCTGCAGAAT GACAAACGTG TCATGGGATT AAAACCAATC AACTGTGAAT TGTGAAATTG 120
AAGCTACTCT TTCGGTTTTA TTTTCTTTAG CATATTGAGT ATAGAAATCT GAAACTTATT 180
TAAAATTTAT ACTGCTTTTG TTGATGGCTC ATTTTGGCTG TGTATCCTCA CTTATGTACT 240
GATTTCTGAT AAAGGCTTGA CATTATTATA ACANAAA 277

SEQ ID NO:1056
SEQUENCE LENGTH:103
SEQUENCE TYPE:nucleic acid
TOPOLOGY:linear
CLONE:HUMGS01197
SEQUENCE DESCRIPTION:
GATCGACCTT NTCTGTTTTN TTTTGTTTTN NTTTCTNTTT CCTGGCCATG AGGACAAAAA 60
TTACTGAGTG GCCCTTAAAG AGGGAAGTTT GTTTTCAGCT GTN 103

SEQ ID NO:1057
SEQUENCE LENGTH:291
SEQUENCE TYPE:nucleic acid
TOPOLOGY:linear
CLONE:HUMGS01198
SEQUENCE DESCRIPTION:
GATCGACCAC ATCCTGGATG CCCTGTAGCC CCTGCCCGCA TCCTCCAGGG GGCCCAGGGT 60
GCCTGCACTT TNCTGTGGCA GGCAGATTGG GTGGTAGTGG GAGGTTGTGC ATGGAGGCCA 120

GTNAAAGCTG ACATCTGTAA AAGGCCTTCA AGGAAGAGAA ACCAGGCCCT GCGTCAGGCA 180
GTGTGAGTTT GCCGTTTGTC CTTAACTTTC TTTTTTTTTT TTTAAAAAAN GGAAANNTTT 240
AAAAAANCTC CCTTTAAAAC CAAANCATNT TTGNNTTTNN NCCAAGGGAA A 291

SEQ ID NO:1058
SEQUENCE LENGTH:276
SEQUENCE TYPE:nucleic acid
TOPOLOGY:linear
CLONE:HUMGS01199
SEQUENCE DESCRIPTION:
GATCCCGCTC TGTNTTTTCC AGGTTTGTGC TATTCTNATT ACATTATCGA CTAGTCTGAA 60
GCAGAGCTGA TATCTCTTTA CCTGGGGAGT CAGCTTCACC AGCCACAGCT GCTGAAAGAA 120
TAGCTTGGAG ATTTCACCCA CTGCATTTCT GTNGCTCAAA CTTTTTGACC TTTGTGCTAT 180
TTGAGAAATC TTTGAAATGC TGAAGGTATG ATTCTCCTTC AGGGGGAACA TGCTTTGGGA 240
AAAACGCCCA CTTAATAAAA TGTATTTCNT TTCAAA 276

SEQ ID NO:1059
SEQUENCE LENGTH:276
SEQUENCE TYPE:nucleic acid
TOPOLOGY:linear
CLONE:HUMGS01200
SEQUENCE DESCRIPTION:
GATCTGTATA TGGTAAACAG GGGTTTAACC ACATGTGGTT AACATGGATT AATGTGGGAN 60
TTTGGCTTCA AGAACACAAC CTTAGGACCT TGGNCCCCAA AAGCTGGTGG TGAAATGAGA 120
GGNGCCAATT TAAGAAGACC CTTATGGAGA CCTGAGGCTG CAGAAACTGG TAGGTTTCAT 180
CAGGTGGTTA AAGTCGTCAA AGTTGTAAGT GACTAACCAA GATTATTTCA TTTTAAAACC 240
ACAGAATAAA AATGACACCT GAGCTTCTCT NTNAAA 276

SEQ ID NO:1060
SEQUENCE LENGTH:276
SEQUENCE TYPE:nucleic acid
TOPOLOGY:linear
CLONE:HUMGS01201
SEQUENCE DESCRIPTION:
GATCTAGTTC CCCTGGAAAA GCTGCTGTAT TTTTAATTTT TAATGGAATG TAGCTTTTNN 60
AATCCTGTCA CTGGCATCAA CAAAAGGAAT TATACCATGA GACCTTATAG CTGTACTTAA 120
AAGCCATTCA GTTCAGCTAT TGGGAGTTCA TGATGAATTA GCATATGCCA GAAAGGTTGC 180
TAACCTTAAC ATCTGAGAGC AGTAACACTG ATTTTATCTG CTGTATGAGA CTTTGTGCAT 240
TTTACTTTGA AATAAAGATT TTTTTCCACA CTGAAA 276

SEQ ID NO:1061
SEQUENCE LENGTH:276
SEQUENCE TYPE:nucleic acid
TOPOLOGY:linear
CLONE:HUMGS01202

SEQUENCE DESCRIPTION:
GATCCTGTTT CTNGCTCTGA GTNCTAGCTA GCCAGCTGTN TTCACACTGT AAACATTCAT 60
CAAGCTGTAC ATTTGGTGCA CTTTTCTGTG TCATACCACA ATAAAAAAAA NCCTATCATC 120
TTACAAAAAC AAGACACCCA AGTCCAGGCC CAAGGAGTAA GTACAAATAT TCCTGTTTCT 180
GANCCATTAC TGTAATTGGC TCTNAAGNCT TGAGGTANCC TTATAGGTTA CTCATAGGGC 240
ATATACAAAT AAACTNGTTT GTTTTCTTTT TNCAAA 276

SEQ ID NO:1062
SEQUENCE LENGTH:275
SEQUENCE TYPE:nucleic acid
TOPOLOGY:linear
CLONE:HUMGS01203
SEQUENCE DESCRIPTION:
GATCTTCAAC GGATACTAGA AAATGAAAAA GACTTGGAAG AAGCTGAGGA ATATAAAGAA 60
GCACGTTTAG TACTGGATTC AGTGAAGTTA GAAGCCTGAA ACTTTTCTCG TATGGGGTGG 120
TTTTTGCATT AAATCCTGGG GTCCATTTTA CAATCCATTA TTTTTGACCA CTGCTATGTG 180
TTCAAGTAGT ATGAGAATGT GATTGTTTTT ATCTGGTTAC ATATATATTT CTTTGTCTAA 240
TTTAATATGT CAAATAAATG AGTTCATCTA ATAAA 275

SEQ ID NO:1063
SEQUENCE LENGTH:274
SEQUENCE TYPE:nucleic acid
TOPOLOGY:linear
CLONE:HUMGS01204
SEQUENCE DESCRIPTION:
GATCTCACCT CTTCCTCAGG ATGGGGAGCT CACTCCGAGA ACAGGAGAAA TCAACATTGC 60
AGTAACAAGG TAGAATGGTT TTGAAGAAGA AAAAACCTGC TTTCTGACTG ATTTTGCCTT 120
GAAGGAAAAA AGAACCTATT TTTGTGCATC ATTTACCAAT CATGCCACAC ANGCATTTAT 180
TTTTAGTACA TTTTATTTTT TCATAAAATT GCTAATGCCA AAGCTTTGTA TTAAAAGAAA 240
TANATANTAN AATAAAAAGT CTGTGCTGTT GAAA 274

SEQ ID NO:1064
SEQUENCE LENGTH:274
SEQUENCE TYPE:nucleic acid
TOPOLOGY:linear
CLONE:HUMGS01205
SEQUENCE DESCRIPTION:
GATCCAAGTT CCTAGACCTC ATGGCTGTCC CCTCCCACCA GTCACCTCCA CTGCACAACT 60
CGGGCGGGGG TGTGACACCT CTCCCCCACC NCCGACTCCG TGGTTTCCGT ATCGTCAACC 120
CTTCAGCCGC CGACCCGGGA GGGGTCTGGC CTACACTGGT CTTCCCCTTC CCATCAACTC 180
TTTCTGCTTG ACAATGTAGC AACCCAGGCC CCCCACCCAC GGTCCTCCCC TTTTTCCTCT 240
CCCTGACAAT AAAGTCTGAA TTTGTTCTGC NAAA 274

SEQ ID NO:1065
SEQUENCE LENGTH:269

SEQUENCE TYPE:nucleic acid
TOPOLOGY:linear
CLONE:HUMGS01207
SEQUENCE DESCRIPTION:
GATCTATCCA TCCAATGTTG TCATTATATT TGACTGTGGT TCAACAGTAT TGCGTTGTCA 60
GACTAGGAAA GTTAAACGAA CAAAATGGTT TTAGTTTTGC TGAAGACTGG CCTTATTAAT 120
GGACAGCTTT CCTAACAAGA GATTATTAAC TTTTATCAGG TGTTAACATC TGTTTCAGGA 180
ACATGGCAGT ATGTTTACAT GTCAGAAGTT TTGTTTAATT CTATGGTATT TCTAAATTGA 240
CTTGTTTAAA TAAATTCAGC AAATGGAAA 269

SEQ ID NO:1066
SEQUENCE LENGTH:269
SEQUENCE TYPE:nucleic acid
TOPOLOGY:linear
CLONE:HUMGS01208
SEQUENCE DESCRIPTION:
GATCTGGTTG GCATTTTCTC CCTGATGATG GGAGCGTCAT TCTTTTGTCT TCATGGTTAC 60
TTGTGTGATA TAACATACAT CTGTNAAAGA AAATCACTTC TTTCTAGGGG AGGGAGGTAG 120
AAAAGTATCT TTCAAACTTG GTTTTTNAGT TTGTNTCTTG TCTTAACTTT GTGTNGGCTC 180
TAACTNAAAC ATGCTGATAT GTGTTTNCAN GANTTTTGGT TTAAGGANGT ATTGTATGGA 240
NGTCCACANA TTGGNAGGTN GTTCATCTN 269

SEQ ID NO:1067
SEQUENCE LENGTH:269
SEQUENCE TYPE:nucleic acid
TOPOLOGY:linear
CLONE:HUMGS01209
SEQUENCE DESCRIPTION:
GATCGATTAA GGAAGGTGGT TATGGCTGGG TGGTTCAGGG GTTTTTTTGG GTTTNTTTTT 60
TTTTTTCTTT GTCTTTTNAA CCTAAAGCTG TTTAAGTTGA AGCATTCTNA GATGTTTGGG 120
GGGAAACATC CTCTNAAAAT GGGNCCTTGT GCTTGCNTTC TGGGGAGGCG GTCCTGAGCA 180
GGTGANTCAT ANGGCATTTA TGCATATGTN ATATGNGGAC TGNACCCACC TTTCCCCCCN 240
AGCCTTTGCC TCTTGGGTTG TTGTNCTGN 269

SEQ ID NO:1068
SEQUENCE LENGTH:268
SEQUENCE TYPE:nucleic acid
TOPOLOGY:linear
CLONE:HUMGS01210
SEQUENCE DESCRIPTION:
GATCCTCTTT GTTTGTNCAA AGGACCAGTT TTCCTAGGCC AAAGAAGTCT CTNCCCCATG 60
TAAGNCCTAT GCCTTNAAAT ATCATGCACC ATGACCCACA GCCATCTGGT TATGTCTTAT 120
TTTTTTCCTA AAAGATAATG TTTATNNTTA AAAAGGAAGG ANGGAGCAAG TGAAGTTTCA 180
TTCTGCTCCA GCGGTGGGGG ANGCCGCTGA ATCCACCTGN TTCTCCTTTT GCAACCGNCA 240
GCANGCAGCT TTTCTCCGGG CNNCAGGN 268

SEQ ID NO:1069
SEQUENCE LENGTH:267
SEQUENCE TYPE:nucleic acid
TOPOLOGY:linear
CLONE:HUMGS01211
SEQUENCE DESCRIPTION:
GATCTAGAAG GTGAAGAATT TTTTNATGTA TATATAGACA TATCTATATA AATNGTCTGG 60
CTGAGGCAGG GCCTTCANCT ATCATTTGGT TAATAAATAC ATTTNAGTAT TTNCATTTCC 120
TACTGCCTGC AGAGTTTCAG GTGCTTGTNG TGTGAAAGTC CTGTAGATGT GTGCAAATTT 180
AACGAAATGA AATTGTATGT GTAAAANTGT ACGATTTTCC ACTGTGCAAC TGTAAATNAT 240
AAATAANANA TATTTTTNCT ATTCAAA 267

SEQ ID NO:1070
SEQUENCE LENGTH:266
SEQUENCE TYPE:nucleic acid
TOPOLOGY:linear
CLONE:HUMGS01212
SEQUENCE DESCRIPTION:
GATCCTAGTG ATTTCAGCCC ATGCATTAAA CAGGAAACAA TAATAAATTT GTAGAATTCA 60
TATTTTTCTA AAGGGAACTT AAAAACTGCT GCTACATGTT ATGTACAAAA CTGGTTTATG 120
CCACATGGAC AGAGAATCAC ANGTTTGGTT TTGGTACTTT NNGTTCCTCT TTGTATTCAG 180
TTGTATAGAC CTNCCAAATT CAGAATGAGA NGAAAGCTGT CTGTATCAAA CCATTTANGC 240
ANTAATTGTT ATATNTNANA GCTAAA 266

SEQ ID NO:1071
SEQUENCE LENGTH:266
SEQUENCE TYPE:nucleic acid
TOPOLOGY:linear
CLONE:HUMGS01213
SEQUENCE DESCRIPTION:
GATCCAAGTT ATACATGAAT AGAAAAAGAT GGTGTTAAAT TTGTGTGTAG GCTGGGAATT 60
CTTGCTGAAG GAATTGGAGA AAACCTGTTG CTGCAAAATT TTACATGTTC CAGATGGAAA 120
GGGAAGTCTA AGCNCTTTTT AAAACAATTT TTTTTTGTAT TTAATTAAGC AATTNCAGTT 180
ATCTGGGATT TTTGGGTCAG AATTTTAAAT TCTGTTTGAT TCTCCATATT CCAGTNAATA 240
AAATACAAAA GCATTGTNTT TTTAAA 266

SEQ ID NO:1072
SEQUENCE LENGTH:265
SEQUENCE TYPE:nucleic acid
TOPOLOGY:linear
CLONE:HUMGS01214
SEQUENCE DESCRIPTION:
GATCTTCAAT TCCTTGAGTC TGAGCTTGTG GGTGGAATTC TAAATTTGTA TCATAATCTG 60
TCTTTTGTGA AACATTTTGA AAATATGTAT ATATAATATT GTATATGCAA ATTGTGTTGT 120

TTCACTTGTA AAGGGAAAAG GCTTATTTTN CTTTATATTT CTGATAACTT GTTTTGCATA 180
TGACCAGCAC TGACTGAAAG GCATGTGTAG CTGCAAACAC TGTTGCTTTT TTTGTGAAAT 240
GNAAATAAAA GTATTTAAAT ACAAA 265

SEQ ID NO:1073
SEQUENCE LENGTH:265
SEQUENCE TYPE:nucleic acid
TOPOLOGY:linear
CLONE:HUMGS01215
SEQUENCE DESCRIPTION:
GATCGTGCCA TTGCACTCCA GCCTGGGTGA CAAGAGCAAA ACTCCATCCC CCCTGCCCAA 60
AAAAAAAATN AATTTTCACA GAAAATTAAT AGCATAGGTA TTATNATCCT CATTTTACAG 120
AGAAAGAAAC AGCCATAGAG AAAAATGACT TGCTCACAAC ACAGGCAACT TTGACTCTAG 180
AGATANCACT TATTACAGTA AAATCCCTCT TCAGNCACAA AATACATGAT TATCTTAAAC 240
ACATTCTTAA TAAAANTTTA NCAAA 265

SEQ ID NO:1074
SEQUENCE LENGTH:268
SEQUENCE TYPE:nucleic acid
TOPOLOGY:linear
CLONE:HUMGS01216
SEQUENCE DESCRIPTION:
GATCAAAGAT TGTAATAATA ATTTTGTAAA TTGTAAGCAA AAAGTTATTT TTATATTATA 60
TACAGTCTAA TTGTTCATCC TAATTGTTCC TGTTTTCATC TAGTCAGAGA TTCAGTAAGT 120
NCCTTGGAAC AATATTGAAT TCTCTTAGCT TGTGTGTGTT TCTTTAATAT TTGAACTCAA 180
GTGGGATTAG AAGACTATNA NNNTACATGT ATGTTTCAGG ATATTTGACC TGTCATTAAT 240
AAAAACAAAC AGTTTTACAG TGCCTAAA 268

SEQ ID NO:1075
SEQUENCE LENGTH:305
SEQUENCE TYPE:nucleic acid
TOPOLOGY:linear
CLONE:HUMGS01217
SEQUENCE DESCRIPTION:
GATCTCCATG TAGTCCATAT GAAACCTGCA GAGTGATTTT CCAGAGTGCT CGATACTGTT 60
AATTACATCT CCATTAGGGC TGAAAAGAAT GACCTACGTT TCTGTATACA GCTGTGTTGC 120
TTTTGATGTT GTGTTACTGT ACACAGAAGT GTGTGCACTG AGGCTCTGCG TGTGGTCCGT 180
ATGGAAAGCC TGGTAGCCCT GCGAGTTAAG TACTGCTTCC ATTCATTGTT TACGCTGGAA 240
TTTTTCTCCC CATGGAATGT AAGTAAAACT TAAGTGTTTG TCATCAATAA ATGGTAATTC 300
CTAAA 305

SEQ ID NO:1076
SEQUENCE LENGTH:263
SEQUENCE TYPE:nucleic acid
TOPOLOGY:linear

CLONE:HUMGS01218
SEQUENCE DESCRIPTION:
GATCCTGTAA GTTGTCATCA AAATATGATT TAGAAATATT GGCCAAGGTG TTGCTTTAAC 60
TGAGGAGAAA AGAAAGCACA CTGCCTAAAT GTGTAAAAGA AAAATGCAGA GGTTATTAAA 120
ATGTAAAGAA GTAACAATCT TTGGATTTGT CTATACATAT ATATATATAT ATATNGNTTT 180
GCCTTAATAT ACCCCCTTTT TTGTTTGTGA CTTTCAACTG TAATCAGTTA ATAAAGTATT 240
TATTCTCTGC ATTCAGGTTC AAA 263

SEQ ID NO:1077
SEQUENCE LENGTH:354
SEQUENCE TYPE:nucleic acid
TOPOLOGY:linear
CLONE:HUMGS01219
SEQUENCE DESCRIPTION:
GATCGACAGC AAGCAATCCT TAAGGGACTT TCAGAACTGA GACAGGGCCT TCTCCAGAAG 60
CAAAAGGAGT TGGAAAGTAG TCTCCTGCCT TTAGCTGAAA ATCAAGAAGA GAGTTTTGGT 120
TCTTCATTTT AAATGTAGAA AATCAAATCC TTCACATTTG ATTTGTGTCT TCCAAATTAT 180
AAAATGTGCT CACTGGCTCA ACTGTATTTT TCAAATAGCC TAGATTTACT TATTTTTTTA 240
ANNGNNCATT AAAAACTTGT ATACTATGTA GTAAAATGCT GTACTTGTNC TATACAATAA 300
ANCAGATACT TCTTTTGTAA AAGCTTAGTA GTAAAACNCC ACCNNNNNNN NNTN 354

SEQ ID NO:1078
SEQUENCE LENGTH:260
SEQUENCE TYPE:nucleic acid
TOPOLOGY:linear
CLONE:HUMGS01220
SEQUENCE DESCRIPTION:
GATCGTGCCT GTGAATAACC ACTGCACTCC AGCCTGGGCA ACATAGTAAG TAAGACCTTG 60
TCTCTTAAAA AAAATACATT CTGAAGAAAG TTCTACTTAT GANTACATTT TATTTATAAC 120
AAACTGGTGA AAATTTTAGA CCAAACCATG TCTTTCTGGG TTGTAGTGAT TAAAAAATGG 180
TTAAGAGAAT GTTCCCTATA CAAGGCATAT GTTATTAANC ATGAAATTTA GGNTTAGTTT 240
TCTCTTTGAA NNTCCTTTTN 260

SEQ ID NO:1079
SEQUENCE LENGTH:260
SEQUENCE TYPE:nucleic acid
TOPOLOGY:linear
CLONE:HUMGS01221
SEQUENCE DESCRIPTION:
GATCCCGAGA CTTGTGTTCT CTTGGCTGAA NACACTNAGG TGCTCCCATC TGTNCGTGGC 60
CCATGANCTG GGATGGTCCT CCAGCTGCCC ACAAGGTCCG CCCCTCTNTC TCTGCACCAC 120
CTGTTTGCAT AAACACACTT TGCTACAATC TTGCTAGTNC GTTTTCTTAA AAGATAATCT 180
ATTTACTGTA AAAATAAATT GGACTTTGCA AAAGCTTTTA GAAGGAAAAG AAAGAGGATT 240
AAAGAGAATT GCTGGTGAAA 260

SEQ ID NO:1080
SEQUENCE LENGTH:259
SEQUENCE TYPE:nucleic acid
TOPOLOGY:linear
CLONE:HUMGS01222
SEQUENCE DESCRIPTION:
GATCCAAATT TCCTTTTGAT ATTATTTTAC TTCTGCCTGA AACATTTTCT TTAACATTTT 60
TTATAGTGTT GTTTTACTGG TGATGATTTC TTTCAGGTTC TAAGTGCCTG AACAAAATCT 120
TTATTTCACC TACATCTTGA AAGATAGTTT CTCTGAGTCT ACAATTCCCA GCTGACAATG 180
TTTATCTTCC AGTACTTTAA AGATGTTGCT TCATTATCTG CTAATTTNNT TTGTTTCCAA 240
TAAAATGTTT GCTGGCAAA 259

SEQ ID NO:1081
SEQUENCE LENGTH:316
SEQUENCE TYPE:nucleic acid
TOPOLOGY:linear
CLONE:HUMGS01223
SEQUENCE DESCRIPTION:
GATCTGCGGT GAAGCCAAGC CGCAAGGTTA CAAGGCATCC TCACCAGGGA TACCCGCCTG 60
CTGCTCCCAG GTGGCCTGCG GCATNGCTAT GCTCAAGGAC CTGGAAACCC ATGCTTCGAG 120
ACAACGTGAC TTTAATGGGA GGGTGGGTGG GCCGCAGACA GGCTGGCAGG GCAGGTGCTG 180
CGTGGGGCCC TCTCCAGCCC GTCCTACCCT GGGCTCACAT GGGGCCTGTG CCCACCCCTC 240
TTGAGTGTCT TGGGGACAGC TCTTTCCACC CCTGGAAGAT GGAAATAAAC CTGCGTGTGG 300
GTGGAGTGTT AGGAAA 316

SEQ ID NO:1082
SEQUENCE LENGTH:259
SEQUENCE TYPE:nucleic acid
TOPOLOGY:linear
CLONE:HUMGS01224
SEQUENCE DESCRIPTION:
GATCCAAATG GATTACCAGT GTGCTACAGA CTTCTTATTA TAGAACAGCA TTCTATTCTA 60
CATCAAAAAT AGTTTGTGTA AGTTAGTTTT GGTTACCATC TAAAATATTT TTAAATGTTC 120
TTTACATAAA AATTTATGTT GTGTTTTAAA ATCCTTAGGG GCTTTATCTA TTTTTCTAAG 180
TCAGTTAACT GTACTTCTAA AAAAAGTATT TTGTATCTAC TTTTGTAACT TCGTCAGAAT 240
AAAATATATT GAANGCAAA 259

SEQ ID NO:1083
SEQUENCE LENGTH:258
SEQUENCE TYPE:nucleic acid
TOPOLOGY:linear
CLONE:HUMGS01225
SEQUENCE DESCRIPTION:
GATCGCTTCA CATNTATAAA AAAAATAANA ATACCCGGGC AAGCTTTCTT TGAAGNTGCT 60
ACAGCATTAA ATATCGAGAA TTTTGGGTGG GAGAGAGCAG TTCAATTTTC TTTACCAGCT 120

GAAGTTCATT TATGATACAA AAGAGATGAA ATGGAAGTGG CAATATAAGG GGATGAGGAG 180
GCATGCTGGC AACCCTTCTT TTAAGATGTG CTCAATTTGT ATAANTGGTG TTTCATGNAA 240
TAATCATCTT GGAGGAAA 258

SEQ ID NO:1084
SEQUENCE LENGTH:342
SEQUENCE TYPE:nucleic acid
TOPOLOGY:linear
CLONE:HUMGS01226
SEQUENCE DESCRIPTION:
GATCGCCGCC CAGGGTTTCA CGGTCGCAGC CATCTTGCTG ACGTCTGNCT GNCACTGCTA 60
TGAAGTCTCG ACCCTAAGCC CAGGGTCTGG CCTTGAAAGC TCCGCAGAAA TGATTCCANA 120
ACCCAGGGAG CAACCACTGG CCCTACCGTG GGACTTACTC CCTCCTCTCC TTTGAGAGGC 180
CCATGTGTCG CTGGGGAGGA AGTGACCNTT TGTGTAACTG TAACCGAAAG TTTTTTCAAA 240
AATCCTAGAT GCTGTTGTTT GAATGTTACA TACTTCTATT NNNGCCACAT CTCCCNTCCA 300
CTCCCNTGCT TAATAAACTC TAAAAATCCA CTTGTATTTA AA 342

SEQ ID NO:1085
SEQUENCE LENGTH:260
SEQUENCE TYPE:nucleic acid
TOPOLOGY:linear
CLONE:HUMGS01227
SEQUENCE DESCRIPTION:
GATCTAAGAA GANACTAGCC TTGTGGAGTA TATAGATGCT TTTCATTATA CACACAAAAA 60
TCCCTGAGGG ACATTTTGAG GCATGAATAT AAAACATTTT TATTTCAGTA ACTTTTCCCC 120
CTGTGTAAGT TACTATGGTT TGTGGTACAA CTTCATTCTA TAGAATATTA AGTGGAAGTG 180
GGTGAATTCT ACTTTTTATG TTGGAGTGGA CCAATGTCTA TCAAGAGTGA CAAATAAAGT 240
TAATGATGAT TCCAAATAAA 260

SEQ ID NO:1086
SEQUENCE LENGTH:256
SEQUENCE TYPE:nucleic acid
TOPOLOGY:linear
CLONE:HUMGS01228
SEQUENCE DESCRIPTION:
GATCCTTAAT GTTTTGATTN TTGTTTTCTG AAATTGGATT TTATTTTATT TTATCTTATA 60
ATNNCAGTTC ATCTAAATTG TGTGTTCTGT ACATGTGATG TTTGACTGTA CCATTGACTG 120
TTATGGAAGT TCAGCGTTGT ATGTCTCTCT CTACACTGTG GTGCACTTAA CTTGTGGNNT 180
TTTTATACTA AAAATGTAGA NTAAAGACTA TTTTGAAGAT TTGANTAAAG TGNNGNNGTT 240
TGCATTACAC CTCAAA 256

SEQ ID NO:1087
SEQUENCE LENGTH:254
SEQUENCE TYPE:nucleic acid
TOPOLOGY:linear

CLONE:HUMGS01229
SEQUENCE DESCRIPTION:
GATCTTTAAA CTCTGGCTTC TTCCTCCTCA ATCTTGACAG AAAAAGGGTG CAGACGTCTG 60
GTTCAAAGAG TTGGATATCA ACACTGATGG TGCAGTTAAC TTCCAGGAGT TCCTCATTCT 120
GGTGATAAAG ATGGGCGTGG CAGCCCACAA AAAAAGCCAT GAAGAANGCC ACAAAGAGTA 180
GCTGAGTTAC TGGGCCCAGA GGCTGGGCCC CTGGACATGT ACCTGCAGAN TAATAAAGTC 240
ATCAATACCT CAAA 254

SEQ ID NO:1088
SEQUENCE LENGTH:297
SEQUENCE TYPE:nucleic acid
TOPOLOGY:linear
CLONE:HUMGS01230
SEQUENCE DESCRIPTION:
GATCCATCCC ATTCACCCAG TGACTTCTTT TTGCCCAGGC CGGGACTTTT TGCATCAGTN 60
ACGTTAACCA GATGACTTTG CCTGTAACCA AACCTCATGC ATCCACGTTT GCGTCTGGGG 120
AGGAATAAAA AGACATCGTT CCCGCTTCTN CGTTTTGTNA TTCCTACTGC CGCCATAGGA 180
ATTATTTCGT TGGCTGANCG TTACCAGCAC CCCGAGANCA CATTTTGATN GANTCAGAGT 240
AGAGGNCATG GCTGTNTTCT NAAAANGCCN CGCCATGNAA NTGCCAATCC CCTTTTN 297

SEQ ID NO:1089
SEQUENCE LENGTH:253
SEQUENCE TYPE:nucleic acid
TOPOLOGY:linear
CLONE:HUMGS01231
SEQUENCE DESCRIPTION:
GATCGCATTT TTGTAAAAGA ACCATGTGTG TTTATATGTG TTTATATATA TACTTGTGTA 60
TGCAAAGGTA AAAGTCTGAA AGGATATATG CTAACTGTTC ACAATGATAA CCCCCCAGGA 120
ATGGGATTGG AGGGGAGGGG GCTTCTGTGT TTGTNATGTA TGCTGGGTGG GANNTTGTGC 180
TTTTATTTCT ATATTGTTTG AATTTTTTTA CAGTATGTAT TATTTTTGTA ATAAAAATTT 240
TAAAAAATTC AAA 253

SEQ ID NO:1090
SEQUENCE LENGTH:252
SEQUENCE TYPE:nucleic acid
TOPOLOGY:linear
CLONE:HUMGS01232
SEQUENCE DESCRIPTION:
GATCTGCCTT GAAATTTAAA AATCTAAATA GCTCTTAGTT GAACAGGGGA GATATAAACA 60
AAGTTTGCAA AATTCCTAAA ACACGATGAT AATAAACATC ATACATCAGA NTTTTGAGAT 120
ATAATTAAAG CAGTACTTAG AAGAAAATGT ATAGCCTTAA ATATTTAACA TCAGTAAAAN 180
TGANAGGNTG AAAATTGGGA TTTAAATTCC CAACTCAAAG AGCTAGAAAN NGANTTACAA 240
AGCAAGNNGA AA 252

SEQ ID NO:1091

SEQUENCE LENGTH:251
SEQUENCE TYPE:nucleic acid
TOPOLOGY:linear
CLONE:HUMGS01233
SEQUENCE DESCRIPTION:
GATCTCATGG GCTTTCCTGG AGGAAAGTTT TTTTTGTTCG TTTTTTTTTA AGACTTGAAA 60
CTTGTAACTG AGATGCTGTA GTTTTTTGCC ATCTGTAGTG ATGTAAAGAT TTAAACCTGA 120
GAGACTTTTN CTTTGTTAGA TTATGAGAAG NACTAGATGC TTTAGGTTTC ATTTTCCCTT 180
AATTGCNATT CTTGTGCGCT NGTTGGGNGG GAACTGTTAT TTTCCNCAAT AAAAAGTAAG 240
TCTTATCGAA A 251

SEQ ID NO:1092
SEQUENCE LENGTH:250
SEQUENCE TYPE:nucleic acid
TOPOLOGY:linear
CLONE:HUMGS01235
SEQUENCE DESCRIPTION:
GATCATTAAA ATCAACGTTA TCTAAGACAG CTGTATCACA TTTTTGGGAT ACATCATGGT 60
ACAGTCAGAA GCATATAAAA TTATGGTTCT GCTTGTCAGN CNCATACAGA ATCCAATACA 120
TTTTGACAAA CTGCCCATTC CTGCAAGTTA ATGCCCTTTT TGAAGCTTCA TTTCCTCTTG 180
TAAAGTACAG ATAGGAATCA TTATTTTGTG GAGTTGCTGT AAAGATTAAN TAAAGGTGAA 240
TGAAAATAAA 250

SEQ ID NO:1093
SEQUENCE LENGTH:248
SEQUENCE TYPE:nucleic acid
TOPOLOGY:linear
CLONE:HUMGS01236
SEQUENCE DESCRIPTION:
GATCCCAACA AAGATACAAG TGAAAACGCA GAAGGTCAAA GNGATGAGAA CAAGGACGAC 60
TATACAATCC CAGATGAGTA TAGAATTGGA CCATATCAGC CCAATGTTCC TGTTGGTATA 120
GACTATGTGA TACCTAAAAC AGGGTTTTAC TGTAAGCTGT GTTCACTCTT TTATACANNN 180
CNAAGAAGTT GCAAAGANTA CTCATTGCAG CAGCCTTCCT CATTATCAGA AATTAAAGAA 240
ATTTCTGN 248

SEQ ID NO:1094
SEQUENCE LENGTH:248
SEQUENCE TYPE:nucleic acid
TOPOLOGY:linear
CLONE:HUMGS01237
SEQUENCE DESCRIPTION:
GATCACTGNC AAAGTGGGAG CACTAAGGGG TGGGTGGGGA AGTGAAATGT TAGGCGATGA 60
ATTCCTGAGC ACCTTGTTTT TNTNCCAAGG TTCGTAGCTC CTCTNTGCCC TTCCAAGCCT 120
GTAACCTCGG AGGACTATCT TTTGTCCTTN ATCCTTTGTN TTGTTTGAGT GGGNCAGCCC 180
CAGAGGAACT GATAAGCAAA TGGCAAGTTT TTAAAGGAAG AGTGGAAAGN NCTGCAANTA 240

AAANNCCN 248

SEQ ID NO:1095
SEQUENCE LENGTH:246
SEQUENCE TYPE:nucleic acid
TOPOLOGY:linear
CLONE:HUMGS01238
SEQUENCE DESCRIPTION:
GATCTCCATG CGGTCCCTGG AAGTACCCAT TGAAACATGC GTATTTGTGT ATAGCAGAAC 60
TCTGAAATAA TATTCTGACA GCAGTTATCT CTGAGGAATT GGGTTATAGG TGATTTTCCC 120
TTTCCGCATG ATAAATTTAT GTAATATTTG ACTGACTTGA CCGTAAGTAT GTTACTTGTA 180
TAATAAAAGG AAAAAAGGTA CTTCTATTTT GAAAAAATAA AAATAAAAGC CTTTGGGTTC 240
TTGAAA 246

SEQ ID NO:1096
SEQUENCE LENGTH:315
SEQUENCE TYPE:nucleic acid
TOPOLOGY:linear
CLONE:HUMGS01239
SEQUENCE DESCRIPTION:
GATCCTGTCA GGGNGTCCCC CATGCCTGGA AGAGGAGCTG GTGGCTGCCA GCCCTNGGGC 60
CCGGCACAGG CCTGGNCCTT CCCCTTCCCT CAAGCCAGGG CTCCTCCTCC TGTCGTGGGC 120
TCATTGTGAC CACTGGCCTC TCTACAGCAC GGCCTGTGGC CTGTTCAAGG CAGAACCACG 180
ACCCTTGACT CCCGGGTNNN NNNGTGGCCA AGGATGCTGG AGCTGAATCA GACGCTGACA 240
GTTCTTCAGG CATTTCTATT TCACAATCGA ATTGAACACA TTGGNCAAAT AAAGTTGAAA 300
TTTTACCACN TGAAA 315

SEQ ID NO:1097
SEQUENCE LENGTH:245
SEQUENCE TYPE:nucleic acid
TOPOLOGY:linear
CLONE:HUMGS01240
SEQUENCE DESCRIPTION:
GATCCCGGGA GCCTTGCCGC ACTGCCTTGT GGGTGGCTTG GCGCTCGTAA TTGCTTCCTG 60
TGAACGCCTC CCAAGGACGA GCCCAGTGTA GTTGTGTGGC GTGAACTCTG CCCGTGTGTT 120
CTCAAATTCC CCAGCTTGGG AAATAGCCCT TGGTGTGGGT TTTATCTCTG GTTTGTGTTC 180
TCCGTGGTGG AATTGACCGA AAGCTCTATG TTTTCGTTAA TAAAGGGCAA CTTAGCCAAG 240
TTAAA 245

SEQ ID NO:1098
SEQUENCE LENGTH:245
SEQUENCE TYPE:nucleic acid
TOPOLOGY:linear
CLONE:HUMGS01241
SEQUENCE DESCRIPTION:

GATCTGCAGC AGCCGAAAAT GCGTTNTGGT AAACCTGGCC TCAAATTCAT ACTACCATAA 60
CTGTTTTTAT ATATTGCCAC TAATTTTGAC TGGATTTAAT AGCACTTTAT TGTACANCTA 120
CAAAAAAAAA TATATNCCTA GAATNGTNNC CAGTGTAATT CCTCTAATGT CCTGGTGCCT 180
TTTCATATAT TTCCAGNATT TTNATACTAT ATNGGTATTT CCTTTGTATA AATNGATNGA 240
TNAAN 245

SEQ ID NO:1099
SEQUENCE LENGTH:251
SEQUENCE TYPE:nucleic acid
TOPOLOGY:linear
CLONE:HUMGS01242
SEQUENCE DESCRIPTION:
GATCTTAGGC TTTTCACTTT TTTTGTTTTG TTTTGTTTTT GAAAGAAAGA AAAAAATACA 60
ATTAACAAGC CTCTTTTGTA AATGGGTTTC CTTTCTATGT ATAAAATCGT GGTGGTCCCT 120
TGTTTTTACA TGTTCATGCT GTGTAATTTT GAGATGTTAC TGAGATATGT TCTGAACATA 180
ATGTGCATTT TTTNCTGTAC AGATGAAATG GGAGANTTTA ATAAAGAGTT TGCAGGTTTT 240
TNCTTGTTAA A 251

SEQ ID NO:1100
SEQUENCE LENGTH:247
SEQUENCE TYPE:nucleic acid
TOPOLOGY:linear
CLONE:HUMGS01243
SEQUENCE DESCRIPTION:
GATCCAGCCC TGAGCATCAC CCAAGTGCCT GATGCCTCAG GTGACAGAAG GCAGGACGTT 60
CCATGCCGAG GCTGCCCCCT CACCCAGAAG TCTGAGCCCA GCCTCAGGAG GGGCCAAGAA 120
CCAGGGGGCC ATCAAAAGCA TCGGGATTTG GCATTGGTTC CAGATGAGCT TTTAAAGCAA 180
ACATAGCAGT TGTTTGCCAT TTCTTGCACT CAGACCTGTG TAATATATGC TCCTGGAAAC 240
CATCAAA 247

SEQ ID NO:1101
SEQUENCE LENGTH:266
SEQUENCE TYPE:nucleic acid
TOPOLOGY:linear
CLONE:HUMGS01244
SEQUENCE DESCRIPTION:
GATCTTCCCT AAAGAGCAGN GGACCANATA NGAAGAGGAA AATTTCTACC TTGAACCGTA 60
TCTGAAAGAG GTTATTCGGG AAAGAAAAGA AAGAGAAGAA TGGGCAAAGA AGTAATCATG 120
TAGTTGAAGT CTGTGGATGC AGCTGTTATG AAGATGGTTA AACTTGAANC AAACAATTTT 180
AAGANTTATT TNGTCTGAAN GATGTTTTAC TTTAAATAAA TGTCTCNTTG NAATGGCTGG 240
AGGTTTTTGG GNGCCAAACC NTTAAA 266

SEQ ID NO:1102
SEQUENCE LENGTH:256
SEQUENCE TYPE:nucleic acid

TOPOLOGY:linear
CLONE:HUMGS01245
SEQUENCE DESCRIPTION:
GATCCCAGGT CCCAAGGAGT GACAGGGGCT TCCTCCCACC TTCTGTCCTT GTCCAGTNAT 60
GTAAATAATG TGCTTTTCCT CTCCCCGAGT CTTTTTTTTT AAAACCTACC GTGGTTCCTN 120
AGCTAACTGC ATTCCCTACC CAGGCAGAGA CTGTCCTATG CCTCGNGCTT CCAAACGAGA 180
CTCAGACCGC GACANAGCCA CCGTATTTAT GGATTGCCAA ANTAANTAAN GCCCAAANCC 240
ATCGGTCTCT GTGAAA 256

SEQ ID NO:1103
SEQUENCE LENGTH:240
SEQUENCE TYPE:nucleic acid
TOPOLOGY:linear
CLONE:HUMGS01246
SEQUENCE DESCRIPTION:
GATCCTGGTG CTGGACAACG CTGCCATTGT CTGCAACTTT GGCAGTGAGC TCAGCCTGGT 60
GTATGTGCCC TCTGTGCTGG AGAAGCTGGA CTGAGCGCAG GGCCTCCTTG CCCAGGCAGG 120
AGGCTGGGGT GCTGTGTGGG GGCCAATGCA CTGAACCTGG ACTTGGGGGA AAGAGCCGAG 180
TATCTTCCAG CCGCTGCCTC CTGACTGTAA TAATATTAAA CTTTTTTAAA AAACCATAAA 240

SEQ ID NO:1104
SEQUENCE LENGTH:245
SEQUENCE TYPE:nucleic acid
TOPOLOGY:linear
CLONE:HUMGS01247
SEQUENCE DESCRIPTION:
GATCTGGGCA GTNAGATAGT GCTCTATGCC TAAGGTGAAG CCACACTAGG NTGAAGCCTC 60
ACTTCCCTGT TTAAGCAATG CAGTGCCTGC TGCCCGTGTG CANGAAGGTA CAGCCATTCA 120
GATAAGTGGA ACTATTGAGT TACATAAAGA AAGTAGATTT GCANTNNTCN GGCAGACGTT 180
TATACANCAC CACGGTGCTT TTATACATTG TNCTNATTTT AATAAAACTG ANGTTCTATG 240
TGAAA 245

SEQ ID NO:1105
SEQUENCE LENGTH:240
SEQUENCE TYPE:nucleic acid
TOPOLOGY:linear
CLONE:HUMGS01248
SEQUENCE DESCRIPTION:
GATCTGGCTT CCATTTGGCC CCCTCATTTC CCAAATGTTT AAATGTATTG GATTTGGATT 60
CTCAATGTAT AAGTTGCCTT ATCTGTTAAT GTCTATCTTC TGTCTCTTTA ATTTTGTATA 120
TCTGCTGTTT TGCTTTTGGA TACATTTTCT AATTAGAAGT CACATGATAA ATATAATCAG 180
TATAGTAATA ATACCATAAT GTGCACATAC TCAATAAATA AATGACTGCA TTGTTGTAAA 240

SEQ ID NO:1106
SEQUENCE LENGTH:240

SEQUENCE TYPE:nucleic acid
TOPOLOGY:linear
CLONE:HUMGS01249
SEQUENCE DESCRIPTION:
GATCCATTTN TCCNTGTAAC TNGGAGAAAG GCCAGTCCCT GTAACGGGGC AGCCCTCTCT 60
TTCCCTCGGT CAGCTCGTGT NAATCCTGGN ACCTCTTCCG GTCGGCTCTG CCCGCTGTTC 120
TGGGGTCGAC TGCCACGACT TTNNATTCAA GAAGCTTCCT CCAGGCGGNA GCGGCTATTT 180
TCCCTAAATN AGAATTGTTA CATTGCAAAT NGTTGAATAA AATATTTNGC GGTCCTTAAA 240

SEQ ID NO:1107
SEQUENCE LENGTH:239
SEQUENCE TYPE:nucleic acid
TOPOLOGY:linear
CLONE:HUMGS01250
SEQUENCE DESCRIPTION:
GATCTGAAGA AATGATGGAA TGGGGAGTGT AGAGAAATGA GAGTCTGTAT GATTCTGGAA 60
CAGAGACATC AGAAGGAAAG ACTGGTGAAA AGATGTATCT TTGTATATTA ATAGCTGTAA 120
TGTAGCTTCC TGATGCTTGA CTAATTGAGG TGTTAATTCT GACTTGAGAA TCTTTTTCAT 180
GAATGATTTT AAAGAAAAAT TTGGATTTTA AAGGTATTAA AATATTTTNG TTTTGTAAA 239

SEQ ID NO:1108
SEQUENCE LENGTH:239
SEQUENCE TYPE:nucleic acid
TOPOLOGY:linear
CLONE:HUMGS01251
SEQUENCE DESCRIPTION:
GATCCGCAGT CGAAAAAGAA CAAGCCACAG AAACGGGCTC GTCGTGCCAG GACACAGCAG 60
TGTCTTTCAA AAAATCAAAA CCAGAAGNTT TATCANCAGC AGGAAGNATG TGGGCTCTGT 120
CAAGTTCACC GTCACCATCA AGCCACTGCT GTGGAAGAGT TTGNCAACAG GNCAGTGTCA 180
CAGCACANCT TCAGANGCAG CATCCGNGTG TCGTCCAACA GANGNCCTNG TCCGGTCAN 239

SEQ ID NO:1109
SEQUENCE LENGTH:238
SEQUENCE TYPE:nucleic acid
TOPOLOGY:linear
CLONE:HUMGS01252
SEQUENCE DESCRIPTION:
GATCTATGTC CGGTGTGGGT TTTTGTCCTC TCGAGTTTTG TCTCTAATAA AGGCCTTTTT 60
TGTTTCAAAT TACATACGCT TTTTACTGCA CAATTTTTGT ATTGACCTTA TTTCAACTGA 120
AGCGATTATT TCCAGCAGGT TTAATTTCGA CAATAAGATA TATTTNAATA GTTTTACCTT 180
TCGCTAGAAT TTTGTATTGT TTAAAACAGT AAATGAATAA NTTTGCTGAT GATTCAAA 238

SEQ ID NO:1110
SEQUENCE LENGTH:238
SEQUENCE TYPE:nucleic acid

TOPOLOGY:linear
CLONE:HUMGS01253
SEQUENCE DESCRIPTION:
GATCAATGAA ATGCACATAA ATNAACTGGT TCCATCAAGA CTGTGCACCC AGGCCTTACA 60
GTCCAACCTT TTTCTGTGTC TGGCTAATAT TTAAAACTAG AAAAACTATT CCTAATCAAC 120
ATGGAGTGGA GAGTTTATTC ACTGTCTTAT CTGCAGAAAT TTGCTGTCAA TATATAACCC 180
GCCTGCAGTG GAAAGTGTAT AGTGTTTTGT AATAAATGGC CTGATGCTAA TGTGTAAA 238

SEQ ID NO:1111
SEQUENCE LENGTH:237
SEQUENCE TYPE:nucleic acid
TOPOLOGY:linear
CLONE:HUMGS01254
SEQUENCE DESCRIPTION:
GATCTACCGG GGGCTATTAG TTTCAATGTA GTGATGAACA CAAACTATAT TTTGAGTTCT 60
CTGCAGCAAG TGGAATGTGG TATGAAAATA TCTGATATTT ACAAAGATGA TACTGCTAAG 120
TCAGAGTCAC GTTTATTGCT AATATGATGG TGGAGTGTTG TCTTCATTCA TAATGAAGGG 180
AAATGTTATT TTTAATAGNG GTTAGTGAAA TAAAANTAAN CCTATTTTCT AANCAAA 237

SEQ ID NO:1112
SEQUENCE LENGTH:268
SEQUENCE TYPE:nucleic acid
TOPOLOGY:linear
CLONE:HUMGS01256
SEQUENCE DESCRIPTION:
GATCTGGAGC ATTTGAGCAA GTTTATAGAA GAACATGCCA CAAAACTGAG CAGGACCAAG 60
GAAGAGCTTT GAAGGCCTGA GGTCTGCGNA AGGTGGGAGG AGGCAGACGC CCTGCGTGGC 120
CCATGGTCGG GGCGTCCACG CCGAGGCCGG CAACAAACGA CAGTATCTCG GATTCCTNTT 180
TTTTTTTTTT AAATTTTTNA AACTNNGGGG TTNCACTNCA NGNTCTGAAT ACTGANTANC 240
CATGAATNCC TGAATAGTTT AGNCCAGN 268

SEQ ID NO:1113
SEQUENCE LENGTH:236
SEQUENCE TYPE:nucleic acid
TOPOLOGY:linear
CLONE:HUMGS01257
SEQUENCE DESCRIPTION:
GATCAGCGCT GAGGACGGNC TCAAGCATGA GTATTTCCGC NAGACCCGNN NCCCCATCGA 60
CCCCTCCATG TTCCCCACGT GGCCCGCCAA GAGCGAGCAG CAGCGTGTGA AGCGGGGCAC 120
CANCCCGAGG CCCCTGAGGG AGGCCTGGGC TACAGCCAGC TGGGTGACGA CGACCTGAAG 180
NAGACGGGCT TCCACCTTAC CACCACGAAC CAGGGGCCTC TNCNGCGGGN CCNGGN 236

SEQ ID NO:1114
SEQUENCE LENGTH:235
SEQUENCE TYPE:nucleic acid

TOPOLOGY:linear
CLONE:HUMGS01258
SEQUENCE DESCRIPTION:
GATCTGTTGA CTTCCCTGGG TAGGACACTG CCACCTCTGG GCTCAGGTCC TNATGCCTCC 60
AAATGGCATC TAGAGTTTGA GCAGCCTTCT TGGCTGCAAG GCAGGCCTAG CCTGTGGCAG 120
CGGGCTAGGG CCCGCAGAGC ATTTGGTGCC CCTCCATGTT GCAATGCAAA CACCTTCACC 180
ACTGGGGCAG TGGGGAGAGA TGGCTATATT AATAAAATAA CGTGTGTCTT TCAAA 235

SEQ ID NO:1115
SEQUENCE LENGTH:235
SEQUENCE TYPE:nucleic acid
TOPOLOGY:linear
CLONE:HUMGS01259
SEQUENCE DESCRIPTION:
GATCATNATT TTTNCTGCGT AGTTGACAGA CTTAGCATAT TAGTTTTTNN TACTCCTACA 60
AGTGTAAATT GAAAAATCTT TATATTAAAA AAGTAAACTG TTATGAAGCT GCTATGTACT 120
AATAATACTT TGCTTGCCAA AGTGTTTGGN NTTTNTTGTN GTTTGTTTGT TTGTTTGTTT 180
TTGGTTCATG AACAACAGTN TCTAGAAACC CATTTTGAAA GNGGAAANTT ATTAN 235

SEQ ID NO:1116
SEQUENCE LENGTH:234
SEQUENCE TYPE:nucleic acid
TOPOLOGY:linear
CLONE:HUMGS01260
SEQUENCE DESCRIPTION:
GATCACAATT NGCTTCATGA ATCAAGGTGT GGAAATGGTT ATATATGGAT TGATTTAGAN 60
AATGGTTACC AGTACAGTCA AAAAAGAGAA AATGAAAAAA ATACAACTAA AAGGAAGAAA 120
CACAACTTCA AAGATTTTTC AGTGATGAGA ATCCACATTT GTATTTCAAG ATAATGTAGT 180
TTAAAAAAAA AAAANGGNAA AAACNTTGTT GNAANTNCCN CCTTTNCCTC NGGN 234

SEQ ID NO:1117
SEQUENCE LENGTH:234
SEQUENCE TYPE:nucleic acid
TOPOLOGY:linear
CLONE:HUMGS01261
SEQUENCE DESCRIPTION:
GATCCCCCGN TCAGCNCCNA AAGTGCTGGG ATACAGGCAT GAGCACCACG TCCGNCTAGA 60
CTTTACCTTT CTAAAGAAAT TGTTTACTGG ATTTATAAGA AGTTAATTTT TGAAAATNAC 120
ATATTTTTGT NTGATAGAAA GAATGGAGCA AGTNGTGCCT ATTTCCTCCA AGTCAGATAA 180
GGTTTCTAAA ATAAATAAAT TTCTAGCATA TAAAGGGTAG AGATAAACTC NNNN 234

SEQ ID NO:1118
SEQUENCE LENGTH:233
SEQUENCE TYPE:nucleic acid
TOPOLOGY:linear

CLONE:HUMGS01262
SEQUENCE DESCRIPTION:
GATCACAGGT CTTCCCTGTG AACTTTGGTT TCTTTCTATA AATGTGTGTG GTTTTCAGCG 60
CTCAACTCCT GTCTTCAAAT GGTAGTAAGT NCTACTTCTA CTTCTGTCAT TCAGAACATT 120
TTATGTCAAA TGATGTAATG CAGAAATNCT TGTGCATATT TGTAACTGAA GGAAGCTTTT 180
TAGATTTATT TNNGTTTTTA ATAAAATTCA GATTCCTATT CTAAACTGGT AAA 233

SEQ ID NO:1119
SEQUENCE LENGTH:230
SEQUENCE TYPE:nucleic acid
TOPOLOGY:linear
CLONE:HUMGS01263
SEQUENCE DESCRIPTION:
GATCTNCTAT GCAGTTCTGC CATGCNTCCT GTTGGTCTCT CTGTGTTCTT TGTTACTTGG 60
GTGCAATAGC AACTTCCCTA CCCCGTGCAT TCCATCTTTN ATGTTGTGTA AAGTTCTTCA 120
CTTTTTNNTC TGAGGGCTGG GGGTTGGGGG AGTCAGCATG ATTATATTTN AATGTAGAAA 180
ATGTGACATC TGGATATAAA ATGAAAATAA ATGTTAAATT AAATGGAAAA 230

SEQ ID NO:1120
SEQUENCE LENGTH:237
SEQUENCE TYPE:nucleic acid
TOPOLOGY:linear
CLONE:HUMGS01264
SEQUENCE DESCRIPTION:
GATCTAAGTT AGTCCAAAAG CTAAATGATT TAAAGTCAAG TTGTAATGCT AGGCATAAGC 60
ACTCTATAAT ACATTAAATT ATAGGCCGAG CAATTAGGGA ATGTTTCTGA AACATTAAAC 120
TTGTATNTAT GTCACTAAAN TTCTAACACA AACTTAAAAA ATGTGTCTCA TACATATGCT 180
GTACTAGGCT TCATCATGCA TTNGTAAATT TGTGTATGAT TTGCNNNTNT GNNNGNN 237

SEQ ID NO:1121
SEQUENCE LENGTH:234
SEQUENCE TYPE:nucleic acid
TOPOLOGY:linear
CLONE:HUMGS01265
SEQUENCE DESCRIPTION:
GATCTGGCCA GTTGTACTTT TAGCTCCCAG AGGGAGAGTT GGTGGTATTA TGAGTTGAGT 60
AAAAACCATC CAGGGGAACT TGAGGGAGCA GTCTGTTGCC AGTAATGTTC CTTGTGTGCC 120
ATTAAACCAC CTCCAGATGA GTGGAGGAAC ATCACTTTTT AATTTTTTAA TTGTATTTGG 180
AATTGTTGCC GTGTACTAAG AACTTGACCT AAATAAAATC CCACAAAGTA TAAA 234

SEQ ID NO:1122
SEQUENCE LENGTH:232
SEQUENCE TYPE:nucleic acid
TOPOLOGY:linear
CLONE:HUMGS01266

SEQUENCE DESCRIPTION:
GATCCTCAGG ACGCAGGTCA CATTCACCTG TGGGCAGAGG GACAGGTCAG ACACCCAGGC 60
CCACCCCAGA GACCCTCCAT GAACTGTGCT CCCAGCCTTC CCGGCAGGTC TGGGAGTAGG 120
GAAGGGCTGA AGCCTTGTTT CCCTTGCAGG GGGGCCAGCC ATTGTCTCCC ACTTGGGGAG 180
TTTCTTCCTG GCATCATGCC TTCTGAATAA ATGCCGATTT TATCCATGGA AA 232

SEQ ID NO:1123
SEQUENCE LENGTH:235
SEQUENCE TYPE:nucleic acid
TOPOLOGY:linear
CLONE:HUMGS01267
SEQUENCE DESCRIPTION:
GATCAGGAGG CATCATTGAG GCCAGGAGCT CTGCCCGTAA CCTGTATCCC ACGTACTCTA 60
TCTTCCATTC CTCGCCCTGC CCCCAGAGGC CAGGAGCTCT GCCCTTGACC TGTATTCCAC 120
TTACTCCACC TTCCATTCCT CGCCCTGTCC CCACAGCCGA GTCCTGCATC AGCCCTTTAT 180
CCTCACACGC TTTTCTACAA TGGCATTCAA TAAAGTGTAT ATGTTTCTGG TGAAA 235

SEQ ID NO:1124
SEQUENCE LENGTH:238
SEQUENCE TYPE:nucleic acid
TOPOLOGY:linear
CLONE:HUMGS01268
SEQUENCE DESCRIPTION:
GATCGATGCC ACGTTCGTAA GGTTCTAAGT CCTTCTTGGC TCCTNATGTG GTCCCTCTCC 60
TCGGAAGAAC TGCCCAGCCA CGGGTTTTNA ACCCACCTGT TGCTCCTNAG GTCGTCACTA 120
TATCAACAGT CACAAACCCA ATGGCAATAA AGGCACTGAC GATAGCTGGC GCGCGCNACG 180
CCACACACCA TTTTNAGATG CCGTTGCAAT TAAATCTTGC CACACTGTCC TCCTGAAA 238

SEQ ID NO:1125
SEQUENCE LENGTH:231
SEQUENCE TYPE:nucleic acid
TOPOLOGY:linear
CLONE:HUMGS01269
SEQUENCE DESCRIPTION:
GATCCCTGGC TGGAATGTCA TACCATTGAC CCATTTGAAG AGTTAAAGCT GGATTTGACT 60
GCTCTATTCT ACCAGGAATA TTGTTAGGGT AGCCTTTTAC CAGTTTCTAA ACANTNGTAA 120
TCATTTATTG ACTCAGCAAT TCCTCAGATA ACAGGTCAAA AGATGTACAG ATACATTCNN 180
NAGTTTTCTT GCTATTAAAG GCACAAGAGT TTCCTTGTAT TTTGACTGAA A 231

SEQ ID NO:1126
SEQUENCE LENGTH:230
SEQUENCE TYPE:nucleic acid
TOPOLOGY:linear
CLONE:HUMGS01270
SEQUENCE DESCRIPTION:

GATCTACAGT CATGCTTTAG CATGGCTATG GAGCTAATTA TCAAGCTTAA AGGGTAACTT 60
TGGGAGGACT CCTCCCTTCA CTCCTAGTCT CCCTTGGAAG AGCAGTCCAG GCTCAGGGAA 120
GGGAATGGAT AGGATGACAC AGTAAGTACA GAAACTGAAG CTGTCAATAG TGAAGGAAAA 180
AGGGGAATTC TTCGTTGCTT TGGCATTNAC ACATAAGTAC TTTGATTAAA 230

SEQ ID NO:1127
SEQUENCE LENGTH:229
SEQUENCE TYPE:nucleic acid
TOPOLOGY:linear
CLONE:HUMGS01272
SEQUENCE DESCRIPTION:
GATCCAAAAT TTATAGCATT CTTCTACCAC CACAGGGGCT CATATGACAT TAACAGACAA 60
GACACTTTTC AGAAAGACAG GTTGTTCTTT TGGGCCACAT CTAAGAAATC TTTGTCTAGC 120
CAAAAGCCAC AAATAATTTC TCATTTTTTG GCTTCTGGAA GATGTACAGT TAAATGTTAT 180
ATTTGGGACT GTTATCTAAT TTGATTAAAT TGCACTCTAC AATATGAAA 229

SEQ ID NO:1128
SEQUENCE LENGTH:229
SEQUENCE TYPE:nucleic acid
TOPOLOGY:linear
CLONE:HUMGS01273
SEQUENCE DESCRIPTION:
GATCAGGGCT GAGGGTAAGG AAAAGAAGAG ACTAGGAAAG CTGGGCCCAA AACTGGAGAC 60
TGTTTGTNTT TCCTGGAGAT GCAGAACTGG GCCCGTGGAG CAGCAGTTTC AGCATCAGGG 120
CGGAAGCTTA AAGCAGCAGC GGGTGTGCCC AGGCACCCAG ATGATTCCTA TGGCACCAGC 180
CAGGAAAAAT GGCAGCTCTT AAAGGAGAAA ATGTTTNAGC CCAGTCAAA 229

SEQ ID NO:1129
SEQUENCE LENGTH:227
SEQUENCE TYPE:nucleic acid
TOPOLOGY:linear
CLONE:HUMGS01274
SEQUENCE DESCRIPTION:
GATCCTTACT ACTGGAATTA CCGGGTTAAA AGGAAATGCT TACCACTAAG ATGTCAGTAT 60
TCTCCTCATG GATATTTTCA ATCTCAATGT TGCCAGTCTA ATAGTATAGT ATGTGGTTGC 120
TTTACTGCTG TTCTCCCCAC CCCCGTGGAG TTGTGTCATT ATTTTAATGA ATGTGAGCTC 180
TTGACTTACT CTAGAATTCT AATACAGATA CTTTCTGCAC TAGTAAA 227

SEQ ID NO:1130
SEQUENCE LENGTH:227
SEQUENCE TYPE:nucleic acid
TOPOLOGY:linear
CLONE:HUMGS01276
SEQUENCE DESCRIPTION:
GATCCGCTTG GTGACGGGCG TCTTCCCAGA TGCTGGCGTC ACCGCTAGAC CAAGGAGCCC 60

TCTGGTGGCC CTGTCCAGGC ATAACAGAAG GCTCGCACTC CTGTCTTCTG GTCACTTCTC 120
ACTATGTCCC CTCAGCTCCT ATCTCTGTAT GGCCTGGTTT TTCCTAGGCT ATGATTATTG 180
AGTGAGGATT ATCATAATAT TGGAATAAAA AGTAATTGCT ACCCAAA 227

SEQ ID NO:1131
SEQUENCE LENGTH:227
SEQUENCE TYPE:nucleic acid
TOPOLOGY:linear
CLONE:HUMGS01277
SEQUENCE DESCRIPTION:
GATCAGGATT GTCCTACAGT TGTAAATAAG ANTAGGTCCT GTTTATTTTG ACATCTNTTT 60
ACAAATGCAT TGTATTAGGG TGTGAATATT CTGAACCATC CTCTTGTTTA AAGTTTGGAA 120
ATTTTTATTG TTAAATGTAA CATTTTAATG GTTGTAATAA TTATTTGTAT AGATATGAAT 180
ATAGTATTTN ATTTAAGANA ATAAACTTTG CATTTTTGCA TNGTAAA 227

SEQ ID NO:1132
SEQUENCE LENGTH:227
SEQUENCE TYPE:nucleic acid
TOPOLOGY:linear
CLONE:HUMGS01278
SEQUENCE DESCRIPTION:
GATCTGAAAT TTGTGTATNC TAAAGTAATT TNGTTTTATG TATTGGAAGT TCACTTAAAA 60
ACTGGAAATA TTTTCTAGAA GGGTACCACA CAAAAGGAAT CATCTTTAAG CTGTTTAATT 120
AACCTAATAA AATAATNTGA TGGGGAAGGC ATTCTAATTG TTTCTGATTT TNNGAGTGGT 180
TCCTGCTAAN TCATCANTAT CANTACAGCT TGATTTGATT TAAGAAA 227

SEQ ID NO:1133
SEQUENCE LENGTH:226
SEQUENCE TYPE:nucleic acid
TOPOLOGY:linear
CLONE:HUMGS01279
SEQUENCE DESCRIPTION:
GATCTTAAAT GTTATATTGA TAACCATGCT CAGCAATGAG CTATTAGATT CATTTTGGGA 60
AATCTCCATA ATTTCAATTT GTAAACTTTG TTAAGACCTG TCTACATTGT TATATGTGTG 120
TGACTTGAGT AATGTTATCA ACGTTTTTGT AAATATTTAC TATGTTTTTC TATTAGCTAA 180
ATTCCAACAA TTTTGTACTT TAATAAAATG TTCTAAACAT TGCAAA 226

SEQ ID NO:1134
SEQUENCE LENGTH:225
SEQUENCE TYPE:nucleic acid
TOPOLOGY:linear
CLONE:HUMGS01280
SEQUENCE DESCRIPTION:
GATCTTTACC AAATTCCTGA CTCAGAGTTC CTCCCATTTN CTTCTGACTC TCATTTTATT 60
CTTACCTCTC ATTTTATTCT TATGATGTTT ACCATTTCTN TTCTCCTCAG TGTCCCTCTC 120

TGAGTGGTAA GAGTATGTTA ATAAGCCAGT NCTGAGACCG GAGCACGAGG CAACTGCTCA 180
ATGTTGGTCA TTGTCACTGT TATTAAATGT AACTTAAGGG NNAAA 225

SEQ ID NO:1135
SEQUENCE LENGTH:224
SEQUENCE TYPE:nucleic acid
TOPOLOGY:linear
CLONE:HUMGS01281
SEQUENCE DESCRIPTION:
GATCTGACTT AAAAACCCAC CAGCATGCTC AATCCCTTGT NATCCTTATG GAATCTGTAT 60
GTTAACTCTC TGGGTGTTCA GGCTTCTATT TGACTGCTGT TGTNACCCTG TTTGCAAAAT 120
GAATATGACA CTCTGTGGAT TATTTNCTCT GTAAGGCACA AGTNCTTCTN ATGATTANTT 180
TGACGTTTCA NGAGCAAAAG CAAATTNANA CTCTCTTCAG CAAA 224

SEQ ID NO:1136
SEQUENCE LENGTH:223
SEQUENCE TYPE:nucleic acid
TOPOLOGY:linear
CLONE:HUMGS01282
SEQUENCE DESCRIPTION:
GATCCAATAG GAGACACCAG TTCCTTGACT GANCCATGCC CCCACCTAAG TNACAAAATG 60
AGGGAAGTGG GGAGTTAGAT TTCAGAGTCC AGGCCCCTAG GTTGGGACCC ACTCCAAATA 120
ATCTCCTCGG TGTGGGTNGT GGTTCTATAG AGGNATANAT GAATAATAAA CATTGTTAAA 180
NTATACGATA ATGNATAAAG TAATCCTTTC ATCANNTGTG AAA 223

SEQ ID NO:1137
SEQUENCE LENGTH:222
SEQUENCE TYPE:nucleic acid
TOPOLOGY:linear
CLONE:HUMGS01283
SEQUENCE DESCRIPTION:
GATCCATCCG ATGGCCTACC AGCTGCAGCT CCAAGCCGCC AGCAACTTCA AGAGCCCAGT 60
CAAGACGATT CGCTGATTCC CTCCCCCACC TGTCCTGCAG TCTTTGACTT TTCCTTTCTT 120
TTTTGCCACC CTTTCAGGAA CCCTGTATGG TTTTNAGTTT AAATTAAAGG AGTCGTTATC 180
GTGGTGGGAA TATGAAATAA AGTAGAAGAA AAGGCCATGA AA 222

SEQ ID NO:1138
SEQUENCE LENGTH:432
SEQUENCE TYPE:nucleic acid
TOPOLOGY:linear
CLONE:HUMGS01284
SEQUENCE DESCRIPTION:
GATCGCACAT CCCTGCGCCC CCATGCCCCC ATGCCCCTCT GAGTCACACA GGACAGAGGA 60
GGCAGAGCTT CTGCCCACTG TTATCTTCAC TTTCTTTGTC CAGTCTTTTG TTTTTAATAA 120
GCAGTGACCC TCCCTACTCT TCTTTTTAAT GATTTTNGTA GTTGATTTGT CTGAACTGTG 180

GCNACTGTGC ATTCCTTGAA TAATCACTTG TAAAAATTGT CAGTGCTTGA NGCTGTTTCC 240
TTTACTCACA TTGAAGGGAC TTCGTTGGTT TTTNGGAGTC TTGGTTGTGA CNNCAAGAGC 300
AGAGTGAGGA AGACCCCCAA GCATAGACTC GGGTACTGTG ATGATGGCTG CAGTCCAGTT 360
TTTATGATTC TGCTTTTTAT GTGTCCCTTG ATACCAGTGG NCTTANCAAT TATACATTNC 420
CTCATANTTA AA 432

SEQ ID NO:1139
SEQUENCE LENGTH:220
SEQUENCE TYPE:nucleic acid
TOPOLOGY:linear
CLONE:HUMGS01285
SEQUENCE DESCRIPTION:
GATCAGACCC AGAGAAAAGT AGTTGTCAGT CATAGCACAC ATCGGACATT TGGAAAACAG 60
CAGTGGCAAC AACTGTATGA CACACTTAAT GCCTGGAAAC AAAATCTGAA CAAAGTGAAA 120
AACAGCCTTT TGAGTCTTTC TGATACCTGA GTTTTTATGC TTATAATTTT TGTTCTTTGA 180
AAAAAAAGCC CTAAATCATA GTAAAACATT ATANNCTAAA 220

SEQ ID NO:1140
SEQUENCE LENGTH:219
SEQUENCE TYPE:nucleic acid
TOPOLOGY:linear
CLONE:HUMGS01286
SEQUENCE DESCRIPTION:
GATCTTGGGG CCTGTTTTCC CATGGGATTC AAGAGGGACA GCCCCAGCTT TTTTTGTTTT 60
TAAGCTTAGG AATCGCCTTT ATGGAAAGGG CTATGTGGGA GANTCAGCTA TCTTGTNTGG 120
TTTTTTTGAG ACCTCAGATG TGTNTTCAGC AGGNCTGAAA NCTTTTNTNC TTTAATAATG 180
AGAAATGTAT ATTTTACTAA TAAATTATTN ACCGAGAAA 219

SEQ ID NO:1141
SEQUENCE LENGTH:219
SEQUENCE TYPE:nucleic acid
TOPOLOGY:linear
CLONE:HUMGS01287
SEQUENCE DESCRIPTION:
GATCTAGAGA CAAAGGATAC TCAATGAGGA GCTTTTTTCC CCTCTTGGAA CAGGTAAAAT 60
GCTTTTCCTT ATTAATATAA TTATAAAACA GTATTTTATG TAACAGCTAT TCCCATATTC 120
TAGGAGTGGC CTAAGAAATG CGTGTTTCAG TGACTAGATT ATAAATATNC TCTATTGTGA 180
ATAGTTGAAT AAAACAGCTG TTTTTTTCTG CTTCCTAAA 219

SEQ ID NO:1142
SEQUENCE LENGTH:219
SEQUENCE TYPE:nucleic acid
TOPOLOGY:linear
CLONE:HUMGS01288
SEQUENCE DESCRIPTION:

GATCTGTGAA ATGCTATCTC TCCTGANGCA ATACTGTTGA CCAGAAAGGA CACTCCATAT 60
TGTGAAACCG GCCTAATTTT TCTGACTGAT ATGGAAACGA TTGCCAACAC ATACTTCTAC 120
TTTTAAATAA ACAACTTTGA TGATGTAACT TGACCTTCCA GAGTTATGGA AATTTTGTCC 180
CCATGTAATG AATAAATTGT ATGTATTTTT CTCTATAAA 219

SEQ ID NO:1143
SEQUENCE LENGTH:219
SEQUENCE TYPE:nucleic acid
TOPOLOGY:linear
CLONE:HUMGS01289
SEQUENCE DESCRIPTION:
GATCCCTGGA CACTTACGTA CAATGCTTCG TNCTGCCTGA TGACAGCCGG GCCAGCCGCC 60
AGCNTACAAG GNTTGTNCGA CGCAGCCTCA GCCCTGTNTT CAATCACACC ATGGTGTACG 120
ATGGCTTTGG GCCTGCTGAC CTGCGCCAGG CTTGTNCCGA GCTCTCCCTC TGGGACCATG 180
GGGCCCTGGG CAACCGNCAG NTNGGGAGGC ACACGCNTN 219

SEQ ID NO:1144
SEQUENCE LENGTH:218
SEQUENCE TYPE:nucleic acid
TOPOLOGY:linear
CLONE:HUMGS01290
SEQUENCE DESCRIPTION:
GATCTCGGTG CTTCTTCCTG TGCTGTGGTT TACCCCAAAC CTTTAGGTTG TTTATTCATT 60
CAGATTAGAT AGACTGGAGC CATAAAGTTA ATTTGCACCT AGCTTTTTGG AGAATAGCCA 120
TGATTAACTG CTATTCGTGG TGGGGGTGGG GGGGAACCCT ATGATTTACT ATGCAGATGA 180
AGAGGGTAGG AACTAAATAA AGGACTTTGT AAGCCAAA 218

SEQ ID NO:1145
SEQUENCE LENGTH:218
SEQUENCE TYPE:nucleic acid
TOPOLOGY:linear
CLONE:HUMGS01291
SEQUENCE DESCRIPTION:
GATCAAGAGG GAGTTCATGC AACACCTGAG AATCTGATTA ATGCACTGAA TAAGTCTGGA 60
TTAAGTGACC TTGCAGAAAG TCTAACTAAT GACAATGAGA CAAATAGTTA GCTTCTTTTT 120
TTTTTCTTTT TATTAAANCT GNGATAGATT TTNTTACCAA GCAGCATTTG ATAAGAGGTC 180
CACTGGTTTT GGTAANCAAT AANCATTTTT ATACCAAA 218

SEQ ID NO:1146
SEQUENCE LENGTH:218
SEQUENCE TYPE:nucleic acid
TOPOLOGY:linear
CLONE:HUMGS01292
SEQUENCE DESCRIPTION:
GATCCCCAGC TGTTTATGCA TAGATAATCT CTCCATTCCC GTGGAACGTT TTTCCTGTTC 60

TTAAGACGTG ATTTTGCTGT AGAAGATGGC ACTTATAACC AAAGCCCAAA GTGGTATAGA 120
AATGCTGGTT TTTCAGTTTT CAGGAGTGGG TTGATTTCAG CACCTACAGT GTACAGTCTT 180
GTATTAAGTT GTTAATAAAA GTACATGTTA AACTTAAA 218

SEQ ID NO:1147
SEQUENCE LENGTH:217
SEQUENCE TYPE:nucleic acid
TOPOLOGY:linear
CLONE:HUMGS01293
SEQUENCE DESCRIPTION:
GATCCTATCC AGTTGAGGAA TGCTTGCAAT GCTCATTGAA GGGATTTGCT TTGGGACTTT 60
GTCATCTTCC AGAAAGGAAA CATATTGTAT ATTTGGCCCA GTGTGATTGA TTGCTTTATC 120
TTTGGTAACT TTTACTTGAA TGGGATTTGC TGAATTAATG ACTATTGAAT TTAAAACTAA 180
TTATGAGTTG ACAAATAAAT AAAAGGTAGT GTTTAAA 217

SEQ ID NO:1148
SEQUENCE LENGTH:217
SEQUENCE TYPE:nucleic acid
TOPOLOGY:linear
CLONE:HUMGS01294
SEQUENCE DESCRIPTION:
GATCATAGTC AGAAAGTACT GCAGTTAACA GGAACCTTCT TGTTCAGGCT GTCATAGCCA 60
CAGTTGCAAA AAGTGCAGTA TTGATTAATG CAATNAGTGC AATNAGTGAC ATCCTGAGTC 120
TTTTATCTGT TNAGCTTGCT NTNCTNTTCT NTCATACATC AGGATATTGC CTGTAATGTG 180
GAGTGGACAG GATAAAAATA AGNATTTTAC TTTCAAA 217

SEQ ID NO:1149
SEQUENCE LENGTH:124
SEQUENCE TYPE:nucleic acid
TOPOLOGY:linear
CLONE:HUMGS01295
SEQUENCE DESCRIPTION:
GATCTGAAGA AAGCTTTTGG TGCTTGTCCT CACAACCACC TCAGTCCTCC CTCCCTGTCC 60
TCCCCTGTCT CCTTTCCTCC TCCTGGGTTC ATGTTGTAAT AAAAGAAGAT TGTTGGTGTG 120
TAAA 124

SEQ ID NO:1150
SEQUENCE LENGTH:215
SEQUENCE TYPE:nucleic acid
TOPOLOGY:linear
CLONE:HUMGS01296
SEQUENCE DESCRIPTION:
GATCATAGCT TGTTTTATTT TGTGCTATAA AATTAACAGT ATTAAATGAC TTATATTCTT 60
AGAATACATC GAGTGTCTTT TCTTAACAGA TTAGTGCCTT TTNATTTTTG TATTCCGTTT 120
TACGTTACTG GTCCCAGCAT CAAAACCCTT GTTTCCATGG CCTGTTTGTA TATTGTCTCA 180

ATAAAACTTG CATCAGCCGG TGGTGGCGGC AGAAA 215

SEQ ID NO:1151
SEQUENCE LENGTH:299
SEQUENCE TYPE:nucleic acid
TOPOLOGY:linear
CLONE:HUMGS01297
SEQUENCE DESCRIPTION:
GATCATAGTG TTTTGTAATC ATTTAATGTC TGCAGCCAAA TTTTTAAGGG TAATTTAGAC 60
CTAATACTGC TCTTGCTGTG TCTTATTAAG TTAAAATTAA TGAATGANTN CTGGTAAAAA 120
TTCANNNNGG CACTCTGTGA GTAGAGAGTA TCATTTAAGC TTATTTNAGT CACATGTAGT 180
ATATATCTCC TTAAAGCTGT CACTCTCACT TTNCTTACCA TTCTCTTGAT TTNNTCAGAA 240
NCCATCTAGT CATCATCTTT ATACTCTACC TGCTTCTGCA ATTGTATATC ANATNGNGN 299

SEQ ID NO:1152
SEQUENCE LENGTH:214
SEQUENCE TYPE:nucleic acid
TOPOLOGY:linear
CLONE:HUMGS01298
SEQUENCE DESCRIPTION:
GATCAAAGTT TAATTGCTTC ATTTTTGTTT AAAAAGGGAT ACTGATGTCA GAAAATCTGT 60
AATATGTTTT ATTCAAAAGA TGTAAATAAT GTATACAGAC TTGTATGTGA TGGGATGGGA 120
AATATTTAAA TNCTAGGTGT TTTTTTTTTT TTNAANGNGG AACCNCAATG TTTATAGGAA 180
AAAANTGATT AANTNGTTCC GTTTGGCCNT GAAA 214

SEQ ID NO:1153
SEQUENCE LENGTH:214
SEQUENCE TYPE:nucleic acid
TOPOLOGY:linear
CLONE:HUMGS01299
SEQUENCE DESCRIPTION:
GATCCCTCTC CTCGCCTGCC TTCTGGAAGA CTTCAGAAGA TTGAGCCTCA CTGGTGCCAG 60
GAAGCCAAAG CTTACTTTGT AGAACTGACA CTAAACTACC CGAAGACTTA GGTGCTTTGT 120
GTACTTAACC CCAGGACCTN CTTACTTTTT AATATAAAGA GTGATGTTGT ATTTCGTGTT 180
CTGCACTTTT TAATATAAAG AGTGATGTTG TAAA 214

SEQ ID NO:1154
SEQUENCE LENGTH:214
SEQUENCE TYPE:nucleic acid
TOPOLOGY:linear
CLONE:HUMGS01300
SEQUENCE DESCRIPTION:
GATCCCAAAG CCACGCTCAT CCAATCCTGG NGAATCATTT ATTTTGAATA TAGACCATTG 60
AATAGGTGAA AAATGGAAAA GTCACTTTTT GTACATTTGC ATTCCTTCAA TAATGATTAC 120
CATTTGTNCA CATATTTGTT GACCATTGGA ATTGATTTTA GCACAGTGCC TAAAATATAG 180

TAGGTGCTCA ATAAATATTT GTTAATTGAA TAAA 214

SEQ ID NO:1155
SEQUENCE LENGTH:214
SEQUENCE TYPE:nucleic acid
TOPOLOGY:linear
CLONE:HUMGS01301
SEQUENCE DESCRIPTION:
GATCCAAACT AATAAGAATA AGCTGTACAG GAACTAGTGC TCAATATACA TTGTATAAAT 60
TTGTGGAAAT CTCTTGGATG TNAATTGTTA CTTCAAGTGG CTTTTATTAA GATTTNCTCA 120
GACTTACTTG GAGGTTAAAG CAAACCCAAA TGTGTATTAT TTTGTTACAG AGCTCTGCTT 180
TATAATTTTG TAATAAAGTT TCAATACAGA CAAA 214

SEQ ID NO:1156
SEQUENCE LENGTH:213
SEQUENCE TYPE:nucleic acid
TOPOLOGY:linear
CLONE:HUMGS01302
SEQUENCE DESCRIPTION:
GATCGCAGTG GAAGAAGATT AGTGCATCAT TGAGAGAGGA GAAGTGGAGT GTGGGGTGAG 60
CAAAAGCCAA AATACTAATC ACTAGTACAC CAGAGATGCT CCACAAGGTA TGCTCCCCAC 120
GGTTTCTTCT CAATTTCAAA GGTNAAGATG TTTTTTTGTG GTGATATAAA ATTTATTGTG 180
AATACTTGGT CCATNAAATT GGTACTTGCT AAA 213

SEQ ID NO:1157
SEQUENCE LENGTH:213
SEQUENCE TYPE:nucleic acid
TOPOLOGY:linear
CLONE:HUMGS01303
SEQUENCE DESCRIPTION:
GATCTTGTTG AGGCATTTAG CTGCCATGCA CCTGTCCCCC TTTAATACTG GGCATTTTAA 60
AGCCATCTCA AGAGGCATCT TCTACATGTT TTGTACGCAT TAAAATAATT TCAAAGATAT 120
CTGAGAAAAG CCGATATTTG CCATTCTTCC TATATCCTGG AATATATCTT GCATCCTGAG 180
TTTATAATAA TAAATAATAT TCTACCTTGG AAA 213

SEQ ID NO:1158
SEQUENCE LENGTH:212
SEQUENCE TYPE:nucleic acid
TOPOLOGY:linear
CLONE:HUMGS01304
SEQUENCE DESCRIPTION:
GATCTCATTT CAATTTCTTT ATTAGAGGGC CTTATTGATG TGTTCTAAGT CTTTCCAGAA 60
AAAAACTATC CAGTGATTTA TATCCTGATT TCAACCAGTC ACTTAGCTGA TAATCACAGT 120
AAGAAGACTT CTGGTATTAT CTCTCTATCA GATAAGATTT TGTTAATGTA CTATTTTACT 180
CTTCAATAAA TAAAACAGTT TATTATCTCA AA 212

SEQ ID NO:1159
SEQUENCE LENGTH:212
SEQUENCE TYPE:nucleic acid
TOPOLOGY:linear
CLONE:HUMGS01305
SEQUENCE DESCRIPTION:
GATCTGGTCT GTTCCTGCGT CTGCGGANGG CCCTTGTCTC CCAGCTATCT ATAACCTTAG 60
CTAGAGTGTC GCCTTGTGGG TTCCTGTTGC TGAGACTTCC TGGATGGAGC CGCCTCACCG 120
CCGGGCCCGT GGCCTGCNCG GANTGTGTCC AATAAAGTTC TTGGATGTGA AAACTTAACA 180
ATTTTGTGTA ATAAAAATGG AGAAGCTCTA AA 212

SEQ ID NO:1160
SEQUENCE LENGTH:211
SEQUENCE TYPE:nucleic acid
TOPOLOGY:linear
CLONE:HUMGS01306
SEQUENCE DESCRIPTION:
GATCCTTGGT GTGTAGTTCA GTCTTGCAGT ATACAAGCTT TTGTGTATAA ATGTTTTATG 60
ATATGATTCC CTGTATTTTG CAGGGGTTTT TTTCTCTTTT NCTTTTTAGA TAAATATGTA 120
TATCAATATT TTAAATNCAT CTTTGCTTTT TTTAGAGGAG TTTGTAATCA CCTTATAACA 180
TGAAAATAAA CATTTCCTTT TTAACATCAA A 211

SEQ ID NO:1161
SEQUENCE LENGTH:210
SEQUENCE TYPE:nucleic acid
TOPOLOGY:linear
CLONE:HUMGS01307
SEQUENCE DESCRIPTION:
GATCCTGTAT ATGTGTGTTT TGGGGGAGCT ATGATAAGTT TTATGGCAAA CGGTTGGTAT 60
TGTTAACTTT TTATTGTCAT CAAAAGTTCA TAAAAGTCCT ATTAATCCCC ATATTCTNNN 120
NCTGCCCTTA ACTCTGGTAT ACACCAAAAA GAAATCTTTA CTTTCCTTGT TTTATCATTA 180
TAAAAATAAA GTATTTTGCT AGTATGGAAA 210

SEQ ID NO:1162
SEQUENCE LENGTH:210
SEQUENCE TYPE:nucleic acid
TOPOLOGY:linear
CLONE:HUMGS01308
SEQUENCE DESCRIPTION:
GATCCGCCCA TGTNAGCCTC CCAAAGTGCT AGGGTTACAG GCATGAGCTA CCATGCCTGG 60
CAACAGCTTT CATATTTGTA AGTTTTTTTT CCTAGGTAAC CCAAGGTCTA TTGAAATTNC 120
ATATAGCTTT CTTTNCTATT ACATATTTAA ATAGATTTNN CCTGATTTNA GAAAAGCTGT 180
AGATTTTNAT ATGTNAATCT TGTNTCCNTN 210

SEQ ID NO:1163
SEQUENCE LENGTH:209
SEQUENCE TYPE:nucleic acid
TOPOLOGY:linear
CLONE:HUMGS01309
SEQUENCE DESCRIPTION:
GATCTNGGAC TCTNCCTCTC ACGACTGCGC CTTAGTCACT CAGACATACG GCGCANTGCA 60
GGAAAAGCCT ACACAGACGA CCTGGGTGCT GTGGGTGGCG CCTGCNTGGA GGACGAGCCA 120
GCNTCCTGCN CTGGATGAGG ACAGCGAGCA CCCGCCATNA TTCTNCGGAC TGACTGAACT 180
TNACCTGTGA CCTCTTACCN GTGGAGCAN 209

SEQ ID NO:1164
SEQUENCE LENGTH:209
SEQUENCE TYPE:nucleic acid
TOPOLOGY:linear
CLONE:HUMGS01310
SEQUENCE DESCRIPTION:
GATCACTNCA GGCCAGGNGT CCAAGACCAG CACGNCCAAC ATGGCAAAAC CCAGTCTGTA 60
CTAAAAATAG AAAAATTAGC TGGGCGTGGT GGCGTGTGCC TGCAATACCA GCTACTCAGA 120
AGGCTGNAGC AGGNTAATTG CTTGANCCTG GTAGGTGGAG GTTNCAGTGA GCTGANATCA 180
TGCCACTGNA CTCCAGCCTG GGTNACAGN 209

SEQ ID NO:1165
SEQUENCE LENGTH:207
SEQUENCE TYPE:nucleic acid
TOPOLOGY:linear
CLONE:HUMGS01311
SEQUENCE DESCRIPTION:
GATCCTTACC GTCCTAGAGA GCAGACGCTT TCTGAAAACT ACTTGCTCCA AAAGACCCTC 60
TGAGTTAACG TTTCAGCTGT ATCATTAGAC TTGTATTTAG AGCGTGTCAC TTCCTCTGAA 120
CTGTTACTGC CTGAATGGAG TCCTGGACGA CATTGGGTTT TTCCTCTAGG AGAATACAAG 180
CCTTAATAAA CAATACTATT TAGCAAA 207

SEQ ID NO:1166
SEQUENCE LENGTH:207
SEQUENCE TYPE:nucleic acid
TOPOLOGY:linear
CLONE:HUMGS01312
SEQUENCE DESCRIPTION:
GATCTGAACA AAGTCTGCAT TTNGTTAATA GTGTGGTACC AATGTTGGTT TCATAGTTTT 60
GGCCATTGTG CTGTGTATTT ATAAGATGCT AACATTAGGG ATAGCTGAAT GTGGTATATA 120
TAGGAACTCT GTGCTGAGTT TATAACTCCT CTGTATGTCT AAAACTATTT CAAAATAAAA 180
NGTTTAAAGG TAAAANAAAA GATTAAA 207

SEQ ID NO:1167

SEQUENCE LENGTH:206
SEQUENCE TYPE:nucleic acid
TOPOLOGY:linear
CLONE:HUMGS01313
SEQUENCE DESCRIPTION:
GATCCAGCCA CCACTCTGAA ACTCATCACA TCTTCATTGA CAGGGAGGGA GCCCAGGACA 60
TATGTGTGGC TCATTGACCA GAAGGCTTTC TTAGTCCCAA CAGCCATGAA CCATGCACTT 120
ATGGATACCC AGCCTTTTAG GGCTACGTGA AATGCATCCT TGTANCATCA TTGTATTCTT 180
TCAATAAATA GCCTTCTGAG TTGAAA 206

SEQ ID NO:1168
SEQUENCE LENGTH:213
SEQUENCE TYPE:nucleic acid
TOPOLOGY:linear
CLONE:HUMGS01314
SEQUENCE DESCRIPTION:
GATCTGCCTG CCTATNAGAG CTTTGAGAAG CTCCGCCACA TGCTACTGTT GGCTATCCAG 60
GAGTGCTCTG AAGGCTTTGG GCTGGCCTAA TAAGGCCCTG CCCAACTCCG TGGGGTTTTT 120
TTAACCATTG TTGGACCTGG GGAGGGGGGA GTTAAAAAAA GAACCAGAAA GAAATTGTCA 180
AAAACCAATA AATGAAATCC ACCAACTCAC AAA 213

SEQ ID NO:1169
SEQUENCE LENGTH:215
SEQUENCE TYPE:nucleic acid
TOPOLOGY:linear
CLONE:HUMGS01315
SEQUENCE DESCRIPTION:
GATCTAAAGT GACCTTGATG GACAGTGGAA GAAATCACAA CATGGAATTC CTCGAATAAC 60
AATTTATTGA CTTTAAATAA TTTTGTCTAA TGCTACATAT ACACAATTAA AAAACCTTTA 120
CACTATTTCT AGAAAGTCAG CATGTATTTT TGGCTCGNNG TTTCNCCNCG TGTTTTCTGT 180
GGAAGGAATA AAAATTTGAG TTTCAGTTGT GTAAA 215

SEQ ID NO:1170
SEQUENCE LENGTH:349
SEQUENCE TYPE:nucleic acid
TOPOLOGY:linear
CLONE:HUMGS01316
SEQUENCE DESCRIPTION:
GATCTGTGAC AGCAGCAGCT TCATGTTGTA TTTTTTTTAC TGAAATTGTA AAATATCCAT 60
CTTAAAGACA TCAACTATTC TAAAANTTGT TTACAGGATA TTCCTTTAGT GGTGGAATTA 120
AAATNTACGA NTACTTGCTT TTTCAAAAAA ATGTATTTTN TGTTAAAAGT TTAAAGATTT 180
TTGCTATATA TTATGGAAGA AAAATGTAAT CGTAAATATT AATTTTNTAC CTATATTGTG 240
CAATACTTGA AAAAAACGGT ATNGAAAGTA TTTTGAGTCA GTGTCTTACA TGTTAAGAGG 300
GACTGAAATA GTTTATATTA AGTTTGTATT AAANTTCTTT AAAATTAAA 349

SEQ ID NO:1171
SEQUENCE LENGTH:205
SEQUENCE TYPE:nucleic acid
TOPOLOGY:linear
CLONE:HUMGS01317
SEQUENCE DESCRIPTION:
GATCCAAGCT GTATATACCA TATATAAACA TTTTACATGA ATCATTTAGT TTTTTAATTC 60
ATTTACTAAT GCTATAAAAT TTCCTATATT ACCCCAGTAA TTTGCATCAG CTGGTTTATA 120
TACTAAAGCA ACATGTTTTG ATGAGTTTCT TACATCCTTA TCGAGGAATT GGGTTAGGAA 180
AAAATACATA ATTGTAAAAC TGAAA 205

SEQ ID NO:1172
SEQUENCE LENGTH:205
SEQUENCE TYPE:nucleic acid
TOPOLOGY:linear
CLONE:HUMGS01318
SEQUENCE DESCRIPTION:
GATCTTCAGG NTTCTACAAA TAGGGTAATT GTAAATTTAA AGCATTAGCA TTTATTGGTG 60
AATAATGTAT ATATCCCCAT TCCAAGAAAT ATAAGTNAGT NANGTTGAAA TAAAATCTTT 120
AAAATTTACT ATATTGCCAG TGGTTTCACA NCAGTTCTCT TGTATTTATT TATCAATTAA 180
ATCAAATAAA AATGATTATG TCAAA 205

SEQ ID NO:1173
SEQUENCE LENGTH:204
SEQUENCE TYPE:nucleic acid
TOPOLOGY:linear
CLONE:HUMGS01319
SEQUENCE DESCRIPTION:
GATCCCCTGA CCTGCATTAC CTTGGTAACC ATTTCATTTT TTAATTTAAT TTCATTTTTT 60
AATTTTGGTG TACAAGCTGT AACATTTCAT CTTTCAAAGT GTAACACGCT GATTTCCTCA 120
AATAGAGATA CCCCTTTGAG TGATAAATTT GCAAAATGCT GTCTTCATTT TCTGTATTAA 180
AATTCATTTC AGTTTTAAAA TAAA 204

SEQ ID NO:1174
SEQUENCE LENGTH:204
SEQUENCE TYPE:nucleic acid
TOPOLOGY:linear
CLONE:HUMGS01320
SEQUENCE DESCRIPTION:
GATCAGAGCT GCTGAGTTCA GGATGCCTGN GNGTGGTTAG GGTAGCTTCT TACATGGATG 60
TCAGGAGAGC TGCTGCCCTT GGCGTGAGTG CGTATTCAGG TGTTTTGCTG CTTTGGCAGA 120
GAGTGGTTGA GTGTNGAATC GTTCAGCTCT NAGGTTCTGT NCCCTGTGGT GGAGAGGACG 180
CAGCAGCNAN NTCTGGGNTC TNAN 204

SEQ ID NO:1175

SEQUENCE LENGTH:205
SEQUENCE TYPE:nucleic acid
TOPOLOGY:linear
CLONE:HUMGS01321
SEQUENCE DESCRIPTION:
GATCAGTGCT CTCTATTGAT GTTCTTGCTG GTCTCCAGNC ACATTCCTGT TGCATTAAGN 60
CTTGAAAGAC TTGTAGATGT GTGATGTTCA GGCACAGGAT GCTGNAAGTA TGTTACTATT 120
CTNAGTTTGT AAATTGTCCT TTTGATACCA TCATCTNGTT TTCTTTTTGT AGGTATAAAT 180
AAAANCACTG TTGNCANTAA GGAAA 205

SEQ ID NO:1176
SEQUENCE LENGTH:203
SEQUENCE TYPE:nucleic acid
TOPOLOGY:linear
CLONE:HUMGS01322
SEQUENCE DESCRIPTION:
GATCTCAGCC ATAAAGTGCC AGTTTGCTTA GTTCTCACTG TCTCCTGGTC TGTGCTGCCC 60
TGCTCTGGGG ATGCACGGCG GCAGGGTGGG GGAGGGAGGT TCCTCGCAGG TCTCAGCCCG 120
GGACAGGGTC TTGCAAGCAG CCTCCTGGGC AGTCGTAAGG GTTGCGGCGT GATGTCTTCA 180
ATAAATTAAG TTTTATTTGG AAA 203

SEQ ID NO:1177
SEQUENCE LENGTH:203
SEQUENCE TYPE:nucleic acid
TOPOLOGY:linear
CLONE:HUMGS01323
SEQUENCE DESCRIPTION:
GATCAAAATG AAACAAATAT ACCTTATCCT AAAGAGCTCA TAACAAATAA GTTACCTCCA 60
CTCTATAAAC TCAGACCTAC TTTTTGAAGA TAACTGCTTT TAACCTCTCC TTACAAGATT 120
TTTGTTGTTG ATGTATTTAA TTTTAGCCCA TGTCTCAATT CTCATTTTCA AAGAATCAAT 180
ATATTAATAT ACCTTTGGTC AAA 203

SEQ ID NO:1178
SEQUENCE LENGTH:202
SEQUENCE TYPE:nucleic acid
TOPOLOGY:linear
CLONE:HUMGS01324
SEQUENCE DESCRIPTION:
GATCGCTCTT GTGTTTTTGC ACTATTAATT AACTTCAAGT GCAATTTTGG CAGGCAGGCC 60
AAGGGTCTCA ACAGGCTATT TGGAATGAGT TCTCTTTTAA GGCCTTCAAC GTAGTNNGAC 120
ATTTTCAAAT CAAAAAGTAC AGCAGGAGCC AGCCTTTATT TTGTCTGTGT ACACAATAAA 180
ATATTCCGCT TCTATAGGCA AA 202

SEQ ID NO:1179
SEQUENCE LENGTH:211

SEQUENCE TYPE:nucleic acid
TOPOLOGY:linear
CLONE:HUMGS01325
SEQUENCE DESCRIPTION:
GATCAGCTGA AGGTTCATGG GTTTTAAGTG CTTGTGGCTC ACTGAAGCTT AAGTTAGGAT 60
TTCNTTGCAA TGAGTAGAAT TTCCCTTCTT TCCCTTGTCA CAGGTTTAAA AACCTCACAG 120
CTTGTATAAT GTAACCATTT GGGGTCCGCT TTTAACTTGG ACTAGTGTAA CTCCTTCATG 180
CAATAAACTG AAAAGAGCCA TGCTGTCTAA A 211

SEQ ID NO:1180
SEQUENCE LENGTH:199
SEQUENCE TYPE:nucleic acid
TOPOLOGY:linear
CLONE:HUMGS01326
SEQUENCE DESCRIPTION:
GATCCCAGGG TGCAAAGTGG CATTGAGACA GCAGCAACAG CTCAAGAGAT ATCTCCTGCC 60
TACTTGCCCC TCCTTCCAGG CCGGCTCTAA GAGAAAGGCC CATCTACTCA GGAAGAGGGC 120
CAGGNCCTTG GGTTCTGGGG ATTGGCCCCT GAGAGGGCTA GTCCTGTGGC TGAAAATAAA 180
GCATGTCCCG CCCNNTAAA 199

SEQ ID NO:1181
SEQUENCE LENGTH:199
SEQUENCE TYPE:nucleic acid
TOPOLOGY:linear
CLONE:HUMGS01327
SEQUENCE DESCRIPTION:
GATCTTTTTT TGGATATTTA TACTTTTAGA TATATAGTAC CTTTAAGTAG CAGTATGGGA 60
CAAGGCTTGT AAATGTTTTG TCTAATGTTC TATTGTCACC TTTTATGCAT TTATCACTTC 120
CAAATCTAAC TTTGCACAAG TAACCCATGT AAAAAAAAAT GTACATTTTT CAAAAGTTGT 180
AAATAAAAAT AACCTTAAA 199

SEQ ID NO:1182
SEQUENCE LENGTH:252
SEQUENCE TYPE:nucleic acid
TOPOLOGY:linear
CLONE:HUMGS01328
SEQUENCE DESCRIPTION:
GATCTGGACA GCTGTCCCTC CACTACAGAA ACCTCACAGA ACACAGCAAA GNATAAGTGC 60
ANGAAGGCTG CTTCCAGCTC CAAAGCACCT ANGAATGGAG GTAAAGCGAA GGATTCAGCA 120
ANGACAGCAG AGGAAACTTC CANGCCAAAA GNTGACTAAA GNAGTACAGG TTANGGTATC 180
TGGTATCTGC ATGTAGANTC TTCAGCTGGT GGATGGTGAC TTTTGAAGTA CNAAAGGCTT 240
TGGCAACNGN AN 252

SEQ ID NO:1183
SEQUENCE LENGTH:197

SEQUENCE TYPE:nucleic acid
TOPOLOGY:linear
CLONE:HUMGS01329
SEQUENCE DESCRIPTION:
GATCTAGGGA GTTACCTAGC AGACCTTCCG GACTGACGTG ATGCTACAGG GGTCCCATCT 60
TTCCTTGTCA TTGAGCTATT TTACAGTTCT GTGAATTGTA AAGAACTGAG GGTAATGCGA 120
ATNACTCTTG TTCATAGAGG CAAATGAATT TTGTCCCATG GAGATTTAAT TGATATTACT 180
CTGTGGATGT TGACAAA 197

SEQ ID NO:1184
SEQUENCE LENGTH:146
SEQUENCE TYPE:nucleic acid
TOPOLOGY:linear
CLONE:HUMGS01330
SEQUENCE DESCRIPTION:
GATCATAGCT ACTACTTCAT TGCAACCTTT ATTAACTGAC CACATCAGAC ATCATGCTAA 60
ATACCTGAAT GCATGAAAAA ACTCCAAATA AGAGAATCTC TTCAGGATTA TAAAAGTTGT 120
AAAATGCAAC TGTATTGCTG AGCAAA 146

SEQ ID NO:1185
SEQUENCE LENGTH:197
SEQUENCE TYPE:nucleic acid
TOPOLOGY:linear
CLONE:HUMGS01331
SEQUENCE DESCRIPTION:
GATCCGGCTG CACACCAGGT GCGGAGGCTG AGCCATCCCT GCTGGACTCC CTACCGCAGA 60
CGGAGTCANG NACGCAGCCG CAGCCNCNTT CCTTCACACC CCCTCACAGA CTCCTTGTGT 120
CCAACGGGAA TAGGAAGAAT TAGTTACTGA CTTCACCTGA GAAAAAAATA AATNCTCTAT 180
GGTGGTTTCA CAGGAAA 197

SEQ ID NO:1186
SEQUENCE LENGTH:196
SEQUENCE TYPE:nucleic acid
TOPOLOGY:linear
CLONE:HUMGS01332
SEQUENCE DESCRIPTION:
GATCTCTTTG TGAACTCTGT GTTCTCTATA TTAGATTGCT GTTTATATGT AAGAATTTTA 60
TTGCTTATGT GGCATACAAT ATTTATAACT ATAAACTTTA TAGAAGTACA GTATTAAAGT 120
CAGTGGTACA CAGACATTCT GTACATATCC TGTGAAACGT GCTGTCATAT GAAATAAATA 180
TATCTGTCTT TACAAA 196

SEQ ID NO:1187
SEQUENCE LENGTH:196
SEQUENCE TYPE:nucleic acid
TOPOLOGY:linear

CLONE:HUMGS01333
SEQUENCE DESCRIPTION:
GATCTTCCCC ATTGTCCACT CAAGTCGTGG CCTGGGGAAC ACAGACGGAG CTGTCCCCAG 60
TGTCCTCCGT CCCTCAGCCC CTGGCCTGGC TGAGTTTGGC AGGGCCTGGG CCATCCCTGG 120
GACAAAGGTG CGTCCCTTCA GCTCTTCTCC GTGGAGCTCG GGGCTTTCTG TATTTATGTA 180
TTTGTACGAA TGTAAA 196

SEQ ID NO:1188
SEQUENCE LENGTH:196
SEQUENCE TYPE:nucleic acid
TOPOLOGY:linear
CLONE:HUMGS01334
SEQUENCE DESCRIPTION:
GATCTTCGGC CTCATTCTGA ATACCTNTTC TTTGGACAGT NTTTTTCCTT TGGTGCTCTC 60
TTGCCTTTAG CTACCTTCTC TAATATGTAT GCTACCATCA CTAATAAAGT GATGGGAATG 120
GGTTTGAGAG TCGTAATTTA TATTAAAAAG TTGTTGGACT TTTAAATACA TTTTTNCAAA 180
TAAAAAATTA AGCAAA 196

SEQ ID NO:1189
SEQUENCE LENGTH:193
SEQUENCE TYPE:nucleic acid
TOPOLOGY:linear
CLONE:HUMGS01335
SEQUENCE DESCRIPTION:
GATCATGTGT GCACAATACT TGTGGCCCAC AAAATTTCAC AATGACTGCT GAGGAATCAT 60
TCTTTTTGCC TGTAAAATAT AACAAAGGGC ATCATTAAGT AGACCAGGTA ATTACTGCTT 120
GTNTCTCAAG GCTGCTGTCT TTATCAGCAC TAACTAAATA AATTTGTTGG TTCAGTTGTA 180
CTTGTCCTGC AAA 193

SEQ ID NO:1190
SEQUENCE LENGTH:192
SEQUENCE TYPE:nucleic acid
TOPOLOGY:linear
CLONE:HUMGS01336
SEQUENCE DESCRIPTION:
GATCCTGTGT CCTAAATTAA TATACACCAG TGGTTCCTCC TCCCTGGTAA AGACTAATGC 60
TCAGATGCTG TTTACGGATA TTTATATTCT AGTCTCACTC TCTTGTCCCA CCCTTCTTCT 120
CTTCCCCATT CCCAACTCCA GCTAAAATAT GGGAAGGGAG AACCCCCAAT AAAACTGCCA 180
TGGNCTTTTA AA 192

SEQ ID NO:1191
SEQUENCE LENGTH:189
SEQUENCE TYPE:nucleic acid
TOPOLOGY:linear
CLONE:HUMGS01337

SEQUENCE DESCRIPTION:
GATCAGTGCC TTGATGCCAA CTAAGGAAAT TTGTTTAGCA TTGAATCTCT GAAGGCTCTA 60
TGAAAGGAAT AGCATGATGT GCTGTTAGAA TCAGATGTTA CTGCTAAAAT TTACATGTTG 120
TGATGTAAAT TGTGTAGAAA ACCATTAAAT CATTCAAAAT AATAAACTAT TTTTATTAGA 180
GAATGTAAA 189

SEQ ID NO:1192
SEQUENCE LENGTH:189
SEQUENCE TYPE:nucleic acid
TOPOLOGY:linear
CLONE:HUMGS01338
SEQUENCE DESCRIPTION:
GATCAAATTT TTGTAAAATT TTTTAAGAAG AGATGGCTTA TTAACAGGGA AGAAGCTTGT 60
NATATTCCAG TTGTAAGAAT AGCCTTAGTG TTTAGATTTT TTNATGATAG GNNAGATGCG 120
GNCATCACTG GGATATTTNC AAATCCCAAG GNCATCAGAG TGAAGTGTCA GTTGTCAGAT 180
GATTTNNAN 189

SEQ ID NO:1193
SEQUENCE LENGTH:188
SEQUENCE TYPE:nucleic acid
TOPOLOGY:linear
CLONE:HUMGS01339
SEQUENCE DESCRIPTION:
GATCCCAGCC AGGCCCTGGA GGTCTGACAG CCCCTCCCTC CCAGAGCTGG TTCCTCCCTG 60
GGAGGGCAAC TTCAGGGCTG GCCACCCCCC GTGTTCCCCA TCCTCAGTTG AAGTTTGATG 120
AATTNANGTC GGGCCTCTAT GCCAACTGGT TCCTTTTGTT CTCAATAAAT GTTGGGTTTG 180
GTAATAAA 188

SEQ ID NO:1194
SEQUENCE LENGTH:187
SEQUENCE TYPE:nucleic acid
TOPOLOGY:linear
CLONE:HUMGS01340
SEQUENCE DESCRIPTION:
GATCTGAATG ATACTGTCTG CTGTGTCTTT TTTCCATGAG AAATCACTGT TGCAAATTGC 60
CTATAAATTG ACTCTACTAA AATAACAATG TTTCAGTCTG AAAATTTGAA TTGAAAAAAA 120
TGTATAATAT AAAATTGTAA TACACTCAAA TGATTATAAA AGTAAAAGTT GGTAATTTAG 180
GCAGAAA 187

SEQ ID NO:1195
SEQUENCE LENGTH:187
SEQUENCE TYPE:nucleic acid
TOPOLOGY:linear
CLONE:HUMGS01341
SEQUENCE DESCRIPTION:

GATCAGGGGG GAAATGGAAT GAGTGAGGCG GGCCAGGGAG CCGCTCAGCT CCAATCTTTG 60
TCACTGTGTG AAATGTGGAC TTGGTATGAC CTGACTGTCC AATTTTCAAG ATGAACCAGA 120
AATCCAGACC NTTATATAAA ATCTCCTGGA TTTTTAAATG TTGGCAATTA ATCAGAATGT 180
TTTTAAA 187

SEQ ID NO:1196
SEQUENCE LENGTH:186
SEQUENCE TYPE:nucleic acid
TOPOLOGY:linear
CLONE:HUMGS01342
SEQUENCE DESCRIPTION:
GATCACTTAA TTCCTTTCTT ATCTTCCCCC TCACTTCCCT TTCTCCCACC CTCTTTTCCA 60
AGCTGTTTCG TTTGAATATA TTACTGGAAT GAGTTGCAGG NAATGCAGCA NACTTGTTTC 120
NCCTAGATTT GAGTCAAACT CCTGNTCAAA GAATCGGTNG GGCATANAAG AAATNTTCTT 180
NTTAAA 186

SEQ ID NO:1197
SEQUENCE LENGTH:186
SEQUENCE TYPE:nucleic acid
TOPOLOGY:linear
CLONE:HUMGS01343
SEQUENCE DESCRIPTION:
GATCTTTTTA ATGACAGTAC AGACTGAGAT TTGAAGGAAA CATGCACAAA TCTGTAAAAC 60
ATAGACCTTC GCTTTATTTT TGTAAGTATC ACCTGCCACC ATGTTTTGTA ATTTGAGGGT 120
CTTGATTTCA CCATTGTCGG TGAAGAAAAT TTTCAATAAA TATGTATTAC CCGTCTGAAG 180
CTTAAA 186

SEQ ID NO:1198
SEQUENCE LENGTH:186
SEQUENCE TYPE:nucleic acid
TOPOLOGY:linear
CLONE:HUMGS01344
SEQUENCE DESCRIPTION:
GATCTTCAGG TATTTTAAAT TTTAGTTACA TCAATTAATG TAAATNAATC CAAATGCTAA 60
TTTTTGTGGT GCAAGAAGTT TCTTATGTAA ATNCAGGGTA TGGATAAGCT AATTAAGATA 120
TCCATCTTTG GTGGCTCTAA GTGTATTATT TGTNTTTAAA TAAAGTGTAC AAATATAGAT 180
AACAAA 186

SEQ ID NO:1199
SEQUENCE LENGTH:184
SEQUENCE TYPE:nucleic acid
TOPOLOGY:linear
CLONE:HUMGS01345
SEQUENCE DESCRIPTION:
GATCTAAACA CGAGGAAACA TTATTCATTG GAAAAGTGCA TGGTGTGTAT TTNAGGGATT 60

ATNAGCTTCT TTCAAGGGCT AAGGCTGCAG AGATATTTCC TCCAGGAATC GTGTTTCAAT 120
TGTAACCAAG AAATTTCCAT TTGTNCTTCA TGAAAAAAAA CTTCTGGTTT TTTTCATGTG 180
GAAA 184

SEQ ID NO:1200
SEQUENCE LENGTH:183
SEQUENCE TYPE:nucleic acid
TOPOLOGY:linear
CLONE:HUMGS01346
SEQUENCE DESCRIPTION:
GATCGCCCGC CCCAGCCCGG GGCCGCTCAG GTCTGCTTGG AGGATGCCTC CCCCAGGAGG 60
GCAGTGAGGG ATGCCGCAAC CTCGACTTCT CAGCCTCCTG GGGTTCCGCC GGCCAACACT 120
GTCTGTNTCA AATACTGTGC TGTGAGTTGT TTCAATAAAG GGGCCCCAAG GGCTGGGCTG 180
AAA 183

SEQ ID NO:1201
SEQUENCE LENGTH:184
SEQUENCE TYPE:nucleic acid
TOPOLOGY:linear
CLONE:HUMGS01347
SEQUENCE DESCRIPTION:
GATCTCTGCT CAGCTTCCCT TGCGTTTTAA GGCCTGCCCT AGCCAGGGTT CCCTCCTGCT 60
TCCAGTACCC TCTCATGGCA TAGGNTGCAA CCCAGCAGAG GGCAGCTAGA TGGACATTTC 120
CCCTGCTCGG AAGGGTTNGC CTGCCTGGCT GGGGNGGTCA GTAAACTTTG AATAGTAAGC 180
CAAA 184

SEQ ID NO:1202
SEQUENCE LENGTH:183
SEQUENCE TYPE:nucleic acid
TOPOLOGY:linear
CLONE:HUMGS01348
SEQUENCE DESCRIPTION:
GATCCCCTTT GGTTTTACTA CCCAAATCTA AATAGATACT TTTGATAATA GATAACTGCT 60
CTTTTACTAA GACATAGTCT CTACCTATAG AAATGTATTT TGAAAACACT TATTTTACAC 120
AGCAATTTTG TATCCATTTA AACTAACCTT TTATCAATAA AGCACTATTG TTTAGATATT 180
AAA 183

SEQ ID NO:1203
SEQUENCE LENGTH:183
SEQUENCE TYPE:nucleic acid
TOPOLOGY:linear
CLONE:HUMGS01349
SEQUENCE DESCRIPTION:
GATCTCATTC TCCCTTTTTA TCCTGAGTTA CCATCAGTGC TAATGTTCCT CTTTTGGGTA 60
TAGTTCTCCA GGAGAACTTG TCCTATATTT NTTTGTGTGT GTTTTTTTCC TTCCAGCTTA 120

AACAAATTAA AACTAAATAT CTAAACCTTG TATACAGACA TTAAAATTAG TGAGAGAAAC 180
AAA 183

SEQ ID NO:1204
SEQUENCE LENGTH:183
SEQUENCE TYPE:nucleic acid
TOPOLOGY:linear
CLONE:HUMGS01350
SEQUENCE DESCRIPTION:
GATCTGCTTT TNCTATTTCT CCCAACCAAA TCCTCTTAAA GACCCTTTGC TATGTAGTCT 60
CATGGTCTAG CATGCATCTT GTAGAAACAA GGCATNCTGG CAGATTGCAG GGTTGAGATG 120
TGTTTTATCT GTTTTATATT TTAAAAGATT CTGCCAGAAA ATAAAACCAG ACCTTGTTCT 180
AAA 183

SEQ ID NO:1205
SEQUENCE LENGTH:183
SEQUENCE TYPE:nucleic acid
TOPOLOGY:linear
CLONE:HUMGS01351
SEQUENCE DESCRIPTION:
GATCTGAATA GAGAAATGCT TACAGATAAT CATTAGCCCA CATACCAGTA ACTTATACTT 60
AAAGATGGGA TGGAGTTGTA AAGTGCTTTT ATAATACAAT ATAATTGTTA AAGGCAAGGG 120
TTGACTCTTT GTTTTATTTT GACATGGCAT GTCCTGAAAT AAATATTGAT TCAATATGGC 180
AAA 183

SEQ ID NO:1206
SEQUENCE LENGTH:182
SEQUENCE TYPE:nucleic acid
TOPOLOGY:linear
CLONE:HUMGS01352
SEQUENCE DESCRIPTION:
GATCCTTGGG GGCCAGGGTG AAGGGTATTT TACGGGAACT CTATAAAGCA GGAAGAAGCA 60
AGTTTATTCT TTAGACCAGT AGCTCTCAAC CATGATGTGG TCGTATATTT ATGGGTCAAC 120
ATGTGTTGTG GGGATATCCC AAGTAACTTG TTATTAATAA AAGTTAAGTT GCAAAATTTA 180
AA 182

SEQ ID NO:1207
SEQUENCE LENGTH:182
SEQUENCE TYPE:nucleic acid
TOPOLOGY:linear
CLONE:HUMGS01353
SEQUENCE DESCRIPTION:
GATCAAAATT GAAGACACAT TCAGAGGTTT GATTGGTTGA GATTAACTGG TGTGGTGGTT 60
GGTGTATGTA TGTTTNATTT TNATGTCTTT GTATGTAGTT CTACATAATG CAAATTGTGC 120
TTTCTGATGG ACAAGACCTC ATAACTGTGA TTAATATCAA TAAAAAGGGG ATGTTGTGGA 180

AA 182

SEQ ID NO:1208
SEQUENCE LENGTH:183
SEQUENCE TYPE:nucleic acid
TOPOLOGY:linear
CLONE:HUMGS01354
SEQUENCE DESCRIPTION:
GATCCGTCAA TCTTGGGTGG AATNATTGTN CGCATTGGCG AGAAATATGT TGACATGTCT 60
GTCAAGACCA AGATTCAGAA GCTGGGCAGG GCTATGCGGG AGATTGTCTA AAAGTGTTGG 120
TTTTCTGCCA TCAGTGAAAA TTCTTAAACT TGGAGCAACA ATAAANAGCT TCCAGAACAG 180
AAA 183

SEQ ID NO:1209
SEQUENCE LENGTH:180
SEQUENCE TYPE:nucleic acid
TOPOLOGY:linear
CLONE:HUMGS01355
SEQUENCE DESCRIPTION:
GATCATGTTA TATTCTATAT CAGACAAACT ATTTTNTTTT GACCTTTCTT CCCCTCCATC 60
CAGTATTTCG GTTGATTTCA TTTTCTCCCC TCTCTTCCCC TTCCACGAAC TGCAATACCA 120
GTAACCTTGG TATATATTTT TTGATACTGT ACACATGGAT GTNTTGTTTC TATGTGCAAA 180

SEQ ID NO:1210
SEQUENCE LENGTH:180
SEQUENCE TYPE:nucleic acid
TOPOLOGY:linear
CLONE:HUMGS01356
SEQUENCE DESCRIPTION:
GATCAAAGAT TCTCCCCATC TCACAGACAA GGAAACTNAG GCCAGAGGGA GGAGAGAATT 60
GCTCATGGCT CCANAACTGG TGGCAAGTTT CTCTGGACTC TTAGGTTTAT TTTNAATATG 120
AAATATAAAA NCAGTTTCAA ATATCTNATT GAGGGAGANG TAAAACCTTA TTTAANCAAA 180

SEQ ID NO:1211
SEQUENCE LENGTH:179
SEQUENCE TYPE:nucleic acid
TOPOLOGY:linear
CLONE:HUMGS01357
SEQUENCE DESCRIPTION:
GATCTATAAA ATGGAAGGTG ACCAATGTCA CTGCTTCTAA AATACTGTCC TAAACAACCC 60
AGAAGATTTT ATTTCAGCTC ATGAATAGCT GTTCTAAATC TGTTGTATGT TAGACATACT 120
ATGAACTACC TCTTCACTTA TTATGGGGAA GTTTCCTTAA TAAAAGTGTG ACGATTAAA 179

SEQ ID NO:1212
SEQUENCE LENGTH:179

SEQUENCE TYPE:nucleic acid
TOPOLOGY:linear
CLONE:HUMGS01358
SEQUENCE DESCRIPTION:
GATCCAAGAC CAGGGNGGTT GGGACGGCCT CCTNTCCTAC TTTGGGACGC CCACGTGGCA 60
GACCGTGACC ATCTTTTTGG CGGGAGTNCT CACCGCCTCA TTCACCATCT GGAAGAAGAT 120
GGGCTGAGGC CCCCAGCTGC NTTGGACTGT TTTTTNCCNC CATAAATNAN GGCATTTTN 179

SEQ ID NO:1213
SEQUENCE LENGTH:178
SEQUENCE TYPE:nucleic acid
TOPOLOGY:linear
CLONE:HUMGS01359
SEQUENCE DESCRIPTION:
GATCTGAGAT GTTCCTTAAC ATGTAGCCAT GTAATTAGAA CTCACAAGGG GCATTGAAAT 60
NTTTTCCTTT TTCANATAGT CTGGGGCTAT TTACTTATTT NAAGTAAGCC AAGTCTACCA 120
TTTGAAAAAT GGTGCTTTAT TTCCATATCT GANGCCTGAN GTTATTTNNN AGTTTGTN 178

SEQ ID NO:1214
SEQUENCE LENGTH:178
SEQUENCE TYPE:nucleic acid
TOPOLOGY:linear
CLONE:HUMGS01360
SEQUENCE DESCRIPTION:
GATCTGGCAT CCNCAGGCTG CAGCTTTATT AGCTTATAAC TTACTCATCT CTATCTTTAC 60
CAGCAGGCTC TGTATTGTTG ATATTTGCAA CTNGNTTTGC TTTCCCATTG GTGGAATTGA 120
AATAATTAGT TTTNAATNAC ATAAGANGCC TGTTTGCTAT TNGGNGGAAG ATAGATGN 178

SEQ ID NO:1215
SEQUENCE LENGTH:176
SEQUENCE TYPE:nucleic acid
TOPOLOGY:linear
CLONE:HUMGS01361
SEQUENCE DESCRIPTION:
GATCAAAGAG GTTATCTCTG CCAGTCACAA GTGTGGCTGG TGTCATTCTG GGTCTGACTG 60
GAGCCCTCCT GGACTGTTTC TTTAATTTCA AAAGCCCTGC AGACATAGTA CCTGGTCAGA 120
ACTATGCCTC GGTTTATTTA TCATTTTGAA ATAAAATCAA AATTTCAACC TGTAAA 176

SEQ ID NO:1216
SEQUENCE LENGTH:176
SEQUENCE TYPE:nucleic acid
TOPOLOGY:linear
CLONE:HUMGS01362
SEQUENCE DESCRIPTION:
GATCAAAACA ATTACCTAAT TGCAAAAGAG AAACTGAAAT GGAACATAGT CTCANATTCT 60

NCTAATGTGT ATCTCACAAT GTCATGTAAT GTAAAGNAAA CCCTTTTGGA ATTAGAATTC 120
TTGTNCTGAA TGCTGAACTA TTTGGTAATA AAGTGCTTAT NTGCAGATAA CAGAAA 176

SEQ ID NO:1217
SEQUENCE LENGTH:175
SEQUENCE TYPE:nucleic acid
TOPOLOGY:linear
CLONE:HUMGS01363
SEQUENCE DESCRIPTION:
GATCTGTGCT GGGGAACAAA GCTTTTGCAG TACCTTATAT TGTAGTTAAA ATTTTATTTA 60
ACATATCCTT CAGTGAGCTC ATTTCACACT GTAGCCTCTT CCTTAAAATT TGTGGTGCTC 120
CTGTAACAGT AAGANCTAAT TCTGAAATAA AAGACATCTC CTAATGCTGT GCAAA 175

SEQ ID NO:1218
SEQUENCE LENGTH:174
SEQUENCE TYPE:nucleic acid
TOPOLOGY:linear
CLONE:HUMGS01364
SEQUENCE DESCRIPTION:
GATCAGGATA ATAATCTGCT TAGTAAGAGA AACAATTTGA ATTTTAGAAG GAAATTGCCT 60
TACCATTTGC AAATTAAGGT AATTAAAATA CAGTGAATTT CAAAATGCCT TTTTAATGAC 120
AATGTGTGAA CTTAATTTGT TTTAATAAAC CAAAATTGTT GTTATTGTGT TAAA 174

SEQ ID NO:1219
SEQUENCE LENGTH:174
SEQUENCE TYPE:nucleic acid
TOPOLOGY:linear
CLONE:HUMGS01365
SEQUENCE DESCRIPTION:
GATCTGAGCA GGCAAAGCTC AAAAGAGAGT TTGGAGGTTA AAAATAATTT ATTTTTGCAG 60
TAGTGTGCTT TGAAATGTGT AAATCTTATT TCTAATGTAT ACAACCACAT TTCACATAAA 120
AATATGCAAT TTATATGCCA GATAAAAATA AAACAAGTGA ATTTGCAAGT GAAA 174

SEQ ID NO:1220
SEQUENCE LENGTH:173
SEQUENCE TYPE:nucleic acid
TOPOLOGY:linear
CLONE:HUMGS01366
SEQUENCE DESCRIPTION:
GATCAACATG ATGGTGACTG GGAAAAAATT ACTTCAAGTA ACATGCTTAG CTTTCCCTCC 60
TTAATGTGAA AAATCAAGGG CTTACTGACA TAGGAACAAC AGAAATGCTC CTGGAACTTC 120
AAGTTGCTGA ATTATAAGTT TATTTTTNAT CAATAAATAT NNNNATACTN AAA 173

SEQ ID NO:1221
SEQUENCE LENGTH:216

SEQUENCE TYPE:nucleic acid
TOPOLOGY:linear
CLONE:HUMGS01367
SEQUENCE DESCRIPTION:
GATCCATATT TAGTTTTATT AAGCTTTTCC CTGTTTTTAG TTTTGTTTTG GGTTTTTTGG 60
CTCATGAATT TNATTTCTGT TTGTCGATAA GAAATGTAAG AGTGGAATGT TAATAAATTT 120
CAGTTTAGTT CTGTAATGTC AAGAATTTAA GATTTAAAAA NCGGATTGGT TAAAAANTGC 180
TTCATATTTG AAAAAGCTGG GAATTGCTGT CTTAAA 216

SEQ ID NO:1222
SEQUENCE LENGTH:173
SEQUENCE TYPE:nucleic acid
TOPOLOGY:linear
CLONE:HUMGS01368
SEQUENCE DESCRIPTION:
GATCGNCTCT GAACCATCCC NTTTCTGCTG CCCGGAGCTG GGGAAGCTCG CTGTGGAGCG 60
TCTCGCACGT GGCAGGCAGG CAGAGAACTT GCTGCACCTT CTAGGAAATT GCAATCGATT 120
TGTAATGCAC TTTCGCTAAT TGGGAGTTCA AAATTAAACT TTAATAAAGC AAA 173

SEQ ID NO:1223
SEQUENCE LENGTH:179
SEQUENCE TYPE:nucleic acid
TOPOLOGY:linear
CLONE:HUMGS01369
SEQUENCE DESCRIPTION:
GATCTTAAGA GCAGACTTAA AGTAGCTTTG TACGCCTTAA TGTTCATTTT GATTTATTTT 60
AAATCTTTAC ATTCAGAAAT NAGATACTGT ATTATCAGAC CAGGAGGCAT TGCTGTGAAA 120
GATAATNTCC TATTCTAAAA TATCAAATTT AAAATAAAGA TAATGAAAGA AAACATAAA 179

SEQ ID NO:1224
SEQUENCE LENGTH:172
SEQUENCE TYPE:nucleic acid
TOPOLOGY:linear
CLONE:HUMGS01370
SEQUENCE DESCRIPTION:
GATCCAAAAA TGGTAGCCAC TCACCCTTCA CAAACTGAAG TCCATGGACC ACGGAAGTCG 60
AGAATTAATG TACACCTGTA TCATGTGTAG GAAACCAGAA ATGTGTTCCT TATTTNTTGT 120
TCCCAAACAG GATTAACTGT GAAGACTAAT TTATAAATGT GAACCTAAGA AA 172

SEQ ID NO:1225
SEQUENCE LENGTH:179
SEQUENCE TYPE:nucleic acid
TOPOLOGY:linear
CLONE:HUMGS01371
SEQUENCE DESCRIPTION:

GATCAGCATG AGGACAGAAG GCAGGAGACT TTGGTCAGTT ACCTGGGAAT TCTGGGCTGC 60
CAGGAAACGA TTTGGGCCTC TGTCAGTTTC TTTTCCATGT ATGAGGAGGG GGAAATTTGT 120
ATATTAGAAA CTTATTCATC CCACTCAGGA CAATAAAAAC GAATGTACAA AAAGCCAAA 179

SEQ ID NO:1226
SEQUENCE LENGTH:175
SEQUENCE TYPE:nucleic acid
TOPOLOGY:linear
CLONE:HUMGS01372
SEQUENCE DESCRIPTION:
GATCACAGTG GCCACGNCCA GGCCACAGTC ATGGTGGCCA CGTCCACAGC CACTAATCAG 60
NAGNCCAGGC CACCCTGCCT CTACCCAACC AGGGCCCCGN GGCCTGTTAT GTCAAACTNT 120
CTTGGCTGTG GGNCTAGGGA CTGGGGCCAA ATAAAGTCTC TTCCTCCAAG TNAAA 175

SEQ ID NO:1227
SEQUENCE LENGTH:318
SEQUENCE TYPE:nucleic acid
TOPOLOGY:linear
CLONE:HUMGS01373
SEQUENCE DESCRIPTION:
GATCTTTAAT CTCATGAAGT ACGACAGCTA CAGCCGCTTC TTAAAGTCTG ACTTGTTTTT 60
AAAACACAAG CGAACCGAGG AAGAGGAAGA AGATTTGCCT GATGCTCAAA CTGCAGCTAA 120
AAGAGCTTCC AGAATTTATA ACACATGAGC CCCCAAAAAG CCGGGACTGG CAGCTTTAAG 180
AAGCAAAGGA ATTTCCTCTC AGGACCGTGC CGGGTTTATC ATTGCTTTGT TATTTGTAAG 240
GACTGAAATG TACAAAACCC TTCAATGGGA TGTGTGTTTT ATTAACTGCT TCACCAGTAA 300
ATTTTGCATG ATGGGCTN 318

SEQ ID NO:1228
SEQUENCE LENGTH:171
SEQUENCE TYPE:nucleic acid
TOPOLOGY:linear
CLONE:HUMGS01374
SEQUENCE DESCRIPTION:
GATCTGGAAG AGGCTTGTNA CCTGAACTTC TGTGTGGTGG CAGTACTGTG GCCCACCAGT 60
GTAATCTCCC TGGATTAAGG CATTCTNAAA ANCTTAGGCT TGGCCTCTTT CACAAATNAG 120
GCCACGGCCT AAATAGGAAT NCCCTGGATT GTGGGNANGT GGGCGNAAGT N 171

SEQ ID NO:1229
SEQUENCE LENGTH:170
SEQUENCE TYPE:nucleic acid
TOPOLOGY:linear
CLONE:HUMGS01375
SEQUENCE DESCRIPTION:
GATCTGATGG TTTTAAAAAG AGGAGTTTCC CTGCTCAAGC TCTCTCTCTT TGCCTGCTGC 60
CATCCATGTA AGATGTGACT TGCTCCTCCT TGCCATCTGC CATGATGTGA GGCTTCCCCA 120

GCCACGTGGA ACTGTAAGTC CAATTAAACC TCTTTTCTTT GTAAATTAAA 170

SEQ ID NO:1230
SEQUENCE LENGTH:170
SEQUENCE TYPE:nucleic acid
TOPOLOGY:linear
CLONE:HUMGS01377
SEQUENCE DESCRIPTION:
GATCCATGGT GTTAGAAGCC AGGGGAACAG TTAACAGGGG AGGGATACTG GGGAGGGGCA 60
TCCTGGAGTG CTGGTCTACC TCATCTGGGT GTTGATTTCA TGAGTATTGT CAGTTTGTTT 120
CCAGACTCCC TGTTGGAGAT GTGGAAATAA AAACCACCTA ANCANGNAAA 170

SEQ ID NO:1231
SEQUENCE LENGTH:170
SEQUENCE TYPE:nucleic acid
TOPOLOGY:linear
CLONE:HUMGS01378
SEQUENCE DESCRIPTION:
GATCCATCAC AAAGCGAAGT CATGGGAGAG CCACACTTGA TGGTGGAATA TAAACTTGGT 60
TTACTGTAAT AGTGTGCTGT TCATGGAAAC CGAGGGCTGC ATCTTGTTTA TAGTCATCTT 120
TGTACTGTAA TTTGATGTAC ACAACATTAA AAGTACTGAC ACCTGAGAAA 170

SEQ ID NO:1232
SEQUENCE LENGTH:168
SEQUENCE TYPE:nucleic acid
TOPOLOGY:linear
CLONE:HUMGS01379
SEQUENCE DESCRIPTION:
GATCTGGGAT GGAATATGGT TTTCTTGATT CCCTTTCAGC CTTCATTTCT CTCTCTCAGG 60
ACTACTACTT TTTAATTACT TTTCACTTAA TTTCCCAATA CTGATGAAAT AAAGAAAAAT 120
GAGGGTTATT TATATACATT TCAATAAAAT CCAATTTGAT TTTTCAAA 168

SEQ ID NO:1233
SEQUENCE LENGTH:167
SEQUENCE TYPE:nucleic acid
TOPOLOGY:linear
CLONE:HUMGS01380
SEQUENCE DESCRIPTION:
GATCTAGAGA ACATAACAAA TGAAACTCTA GAAATTTGAA AGAAAAAAAA ACACTTAGCT 60
GTAAGTGCTT TGACCTATTT TTTTTAAAAA AAAAAAAACC TGTNTTAATT CTGTGACTGN 120
ATTGNACCCT TCACCAGCAC TANGNGAAGT NGGANTGGAN GAAATTN 167

SEQ ID NO:1234
SEQUENCE LENGTH:167
SEQUENCE TYPE:nucleic acid

TOPOLOGY:linear
CLONE:HUMGS01381
SEQUENCE DESCRIPTION:
GATCAAGCCA TCGGAGCTGC TAGAGTTCTG TCTGGACTTT CCAGAGACCA GTATTCCCTT 60
TTGCTGCCTC TAAAAGGCCT GTCCCTGCAG ACATGAGAGA CAGCAGGTCT CATGGGGGTG 120
ACAAGCTTTT TTTTTTTTTN TAAANGATTT TCNAAANNNA ANTNCCN 167

SEQ ID NO:1235
SEQUENCE LENGTH:167
SEQUENCE TYPE:nucleic acid
TOPOLOGY:linear
CLONE:HUMGS01382
SEQUENCE DESCRIPTION:
GATCTTTTGT CTTCCAGTCT TCTCTTAGTG TCCTGCTCCT AGGTTTCCCT CTCTTCTGGT 60
TCTTCTCCCA GGTATTCTCT TCCCAGGCCT CTCTGGCCAC TGCTTTGTAT CAGGGTTTTT 120
CACGCTTTTG TAGAACTGAG GTTTCAATAA ACAGTTTCAG TTGCAAA 167

SEQ ID NO:1236
SEQUENCE LENGTH:167
SEQUENCE TYPE:nucleic acid
TOPOLOGY:linear
CLONE:HUMGS01383
SEQUENCE DESCRIPTION:
GATCCTCCAG CCCACCACCA CATAGTAATC ACTGACACTG TGGGGTGTCT CAGCTGGGGT 60
TCTGCTGTGT GTGTGTGTAT TTNACAAAAT ATGTATGGTT TCTGTTCAGC TCTTTTCAGT 120
TAACAGATGA ACTGNNNNTA TCATTAAATA TTTTTGAAAA CATGAAA 167

SEQ ID NO:1237
SEQUENCE LENGTH:171
SEQUENCE TYPE:nucleic acid
TOPOLOGY:linear
CLONE:HUMGS01384
SEQUENCE DESCRIPTION:
GATCCATCAG TAATGTAGCT AGCTGTAGAG CTTGCAACTT AATAGCAGCA GCTGCCCAAT 60
GCCATGTNAA GTAACAAACT GGTTTTTGGT TTTTTTTTTC CCCTTCAGTT TAAATGTNAT 120
GTGNAATGTA TTAAAACCCT TATTTAAATA AANCTTGTTT TCAGAAATAA A 171

SEQ ID NO:1238
SEQUENCE LENGTH:166
SEQUENCE TYPE:nucleic acid
TOPOLOGY:linear
CLONE:HUMGS01385
SEQUENCE DESCRIPTION:
GATCTGGGGT TGGGTCATGA CACCAGCTAC CAATTTNAGA ATATTATTCC TNGGTTTCTT 60
TATGAAAAAT GGGTGCTAGT GGTAATTCCT TNGTGGCTTA GTAAACTACT CTNTGGATGA 120

TTTCCAAACA TTCAAAGCCA ATAGCCTNGT NATNAACAAG ANATTN 166

SEQ ID NO:1239
SEQUENCE LENGTH:165
SEQUENCE TYPE:nucleic acid
TOPOLOGY:linear
CLONE:HUMGS01386
SEQUENCE DESCRIPTION:
GATCAAAACC CAGCAGAGTG CAAGCAGCAG TGAAGCAGGA TGGTCTACCT TCTGCTTCCC 60
TGGAAAGGAT GAATTTACAT CATTTGACAA GCCTATTTTC AAGTTATTTG TTGTTTGTTT 120
GCTTGTTTTT GTTTTTGCAG CTAAAATAAA AATTTCAAAT ACAAA 165

SEQ ID NO:1240
SEQUENCE LENGTH:165
SEQUENCE TYPE:nucleic acid
TOPOLOGY:linear
CLONE:HUMGS01387
SEQUENCE DESCRIPTION:
GATCTGTCTG TGTTGTTATA TATTCTTTTA TGCATACTTA AAGTTAAAGG GTTTTATCCA 60
CTGTCATTTC AATTGGATAA CATTTTGTCA AGTTTTTTTT TCCTGATTAT TTGATGTAGC 120
TGGATTCAAG AATGGATTGC CTNATCAATA AAGAATATTT AGAAA 165

SEQ ID NO:1241
SEQUENCE LENGTH:166
SEQUENCE TYPE:nucleic acid
TOPOLOGY:linear
CLONE:HUMGS01388
SEQUENCE DESCRIPTION:
GATCCTATTT TGGGCCTGGG GTGGGTATAC CTGNGGCTGG TCTTAGGAGG GTGCTAGGCT 60
GCAGACTGCC TTGTACTCCC TGGACACCCT CAAATGGGGT TTTCTGTGTT ATTTCATAAA 120
ATTCTTTGAA GTCCAATAAA GCATGTAGGA GATTTTAACC ACTAAA 166

SEQ ID NO:1242
SEQUENCE LENGTH:164
SEQUENCE TYPE:nucleic acid
TOPOLOGY:linear
CLONE:HUMGS01389
SEQUENCE DESCRIPTION:
GATCAAAGGN CAAAAGACCA AAAATCAGCG GGCTGAGGAT GGAGCACAGC CATGAACCTG 60
CTCACGACAA GACGCACCCA TGCTTCTCAG GGTCAAGGCT TTATGTTAAA GCTTCCTGTC 120
GGGGCTGCTA GGTCAGCATT AAAGTAAGGC AACCAACAGT NAAA 164

SEQ ID NO:1243
SEQUENCE LENGTH:163
SEQUENCE TYPE:nucleic acid

TOPOLOGY:linear
CLONE:HUMGS01390
SEQUENCE DESCRIPTION:
GATCTGCTCA GTAATATAAT TTGCCATTTT TATTAGAAAT TTAATTTCTT CATGTGATGT 60
CATGAAACTG TACATACTGC AGTGTGAATT TTTTTGTTTT GTTTTTTAAT CTTTTAGTGT 120
TTACTTCCTG CAGTGAATTT GAATAAATGA GAAAAAATGC AAA 163

SEQ ID NO:1244
SEQUENCE LENGTH:163
SEQUENCE TYPE:nucleic acid
TOPOLOGY:linear
CLONE:HUMGS01391
SEQUENCE DESCRIPTION:
GATCTGGATT TGCTTTACCT TGTTAATATT ATCTAGGGGA AAAAGTGCAA ATTGCTCCAT 60
GTTCTTCTCT CCCTTATGTA ACATCTCCTG AGGGTGTTTA GTTGCATGGC TGTTCAGAAA 120
GGTATTAAGG GCTTAGGCCA AATCTTACTT TGAGTATGTT AAA 163

SEQ ID NO:1245
SEQUENCE LENGTH:162
SEQUENCE TYPE:nucleic acid
TOPOLOGY:linear
CLONE:HUMGS01392
SEQUENCE DESCRIPTION:
GATCGTCAAA AAGAGAACCA AGAAATTCAT CCGGCACCAG TCAGACCGAT ATGTCAAAAT 60
CAAGCGTAAC TGGTGGAAAC CCAGAGGCAT TGACAACAGG GTTCATAGAA GGTTCAAGGG 120
CCAGGTCTTG ATATGATGCC CAGCATTGGT TATAGGNGCA AA 162

SEQ ID NO:1246
SEQUENCE LENGTH:197
SEQUENCE TYPE:nucleic acid
TOPOLOGY:linear
CLONE:HUMGS01393
SEQUENCE DESCRIPTION:
GATCTGGAAG TGAACACAGT TTATGTACAG GGAAAAGGAT TTTATTATCC TTAGGAATGT 60
CATCCAAGAC GTAGAGCTTG AATGTGACGT TATTTAAAAA CAACAACAAA GAAGGCAGAG 120
CCAGGATATA ACTAGAAAAA GGATGTCTTT TTTTTTTTTT TNNCNCCCCC TNTAANCNCT 180
GNTGCTGCCT NANTTTN 197

SEQ ID NO:1247
SEQUENCE LENGTH:160
SEQUENCE TYPE:nucleic acid
TOPOLOGY:linear
CLONE:HUMGS01394
SEQUENCE DESCRIPTION:
GATCTCAATG CCAATCCTCC ATTCTTCCTC TCCAGATATT TTTGGGAGTG ACAAACATTC 60

TCTCATCCTA CTTAGCCTAC CTAGATTTCT CATGACGAGT TAATGCATGT CCGTGGTTGG 120
GTGCACCTGT AGTTCTGTTT ATTGGTCAGT GGAAATGAAA 160

SEQ ID NO:1248
SEQUENCE LENGTH:160
SEQUENCE TYPE:nucleic acid
TOPOLOGY:linear
CLONE:HUMGS01395
SEQUENCE DESCRIPTION:
GATCTGAAAA TCGACCTCAA CTCAAGGGTG GTCAGCTCAA TGCTACACAG AGCACGGACT 60
TTTGGATTCT TTGCAGTACT TTGAATTTAT TTTTCTACCT ATATATGTTT TATATGCTGC 120
TGGTGCTCCA TTAAAGTTTT ACTCTGTGTT GCACTATAAA 160

SEQ ID NO:1249
SEQUENCE LENGTH:159
SEQUENCE TYPE:nucleic acid
TOPOLOGY:linear
CLONE:HUMGS01396
SEQUENCE DESCRIPTION:
GATCTGCATG ACCTATAATC TTTGAACCAC TTTCGTACCT CATGTTTTTA TCCAGCACTC 60
TTATTGTAAT ATGTACTAGT CTGTGAACAA TGTCAAATAA AAGAGAACGA ACAGGTAGTT 120
TGGTGGAGCT GAGCTAGTGT ACAATACACT AGTTGTAAA 159

SEQ ID NO:1250
SEQUENCE LENGTH:159
SEQUENCE TYPE:nucleic acid
TOPOLOGY:linear
CLONE:HUMGS01397
SEQUENCE DESCRIPTION:
GATCTTGGAA TTCTTGTNTG GATTTNCACA GTTTTTAAAA TTAATCATGT CAGCCCAATG 60
CAGCTGTGTC ATTGCCACCT AGTGGTAAAA CTAACATTAC TGTTAAGCTG TGTGATTTTA 120
AACTTGAATC CCCAGTAAAN GGTTCACTGT TCTGTGAAA 159

SEQ ID NO:1251
SEQUENCE LENGTH:159
SEQUENCE TYPE:nucleic acid
TOPOLOGY:linear
CLONE:HUMGS01398
SEQUENCE DESCRIPTION:
GATCCCTCTG AGAGTTGATG AGGATGTGTA ACAAGTATTT NCTTCTATNG TGCCTGCCAG 60
GGCTGAAGCT GCCTGGTATC CAGGAGGGGA ATGCTGGTAT CCCCATATGN CTGTNTTTGT 120
TTGAGATTTT TAATAATAAA TAATAAATTT TTGAAGAAA 159

SEQ ID NO:1252
SEQUENCE LENGTH:158

SEQUENCE TYPE:nucleic acid
TOPOLOGY:linear
CLONE:HUMGS01400
SEQUENCE DESCRIPTION:
GATCCTATTC TTGTAAAAAT ATTTGTATGT ATGCACAGAA ATCTGCAAAG ATGTACACTT 60
AGTGAACTGG TTACCAACGA ATGGTGGGAC TAACTAAAAT GGTCTTTTTA CTTATATGTG 120
CATTTCTTTT TATAATAAAA ATGGGTTATA TGCCTAAA 158

SEQ ID NO:1253
SEQUENCE LENGTH:158
SEQUENCE TYPE:nucleic acid
TOPOLOGY:linear
CLONE:HUMGS01401
SEQUENCE DESCRIPTION:
GATCTGCCCT GTGTTATCCA GTTATGAGAT AAAAAATGAA TATAAGAGTG CTTGTCATTA 60
TAAAAGTTTC CTTTTTTATT CTCTCAAGCC ACCAGCTGCC AGCCACCAGC AGCCAGCTGC 120
CAGCCTAGCT TTTNCCCNTT TTTTTTTTTT NNNCNNTN 158

SEQ ID NO:1254
SEQUENCE LENGTH:157
SEQUENCE TYPE:nucleic acid
TOPOLOGY:linear
CLONE:HUMGS01402
SEQUENCE DESCRIPTION:
GATCTCAATC TTCGGGGTGT GATGAATAGC GAATCATCTC AAATCCTTGA GCACTCAGTC 60
TAGTGAAGAT GTTGTCATTA TGTACAATAC ATAACTAGTT TAATTAACTA TGTGATGTTA 120
ACTATTATTA ATAAATTTTA ACATTTTCCA AAATAAA 157

SEQ ID NO:1255
SEQUENCE LENGTH:157
SEQUENCE TYPE:nucleic acid
TOPOLOGY:linear
CLONE:HUMGS01403
SEQUENCE DESCRIPTION:
GATCCGGATG NGGGGAGCTC TGTACAGAGG GCTGGTGATT GTAAAAATTT CTTTTGTAAA 60
GTAGAAGTTG GGGGTGGGGT GGGTGCTGGC TGCAAAAATT TCTGGCTTCT CTTACCCCTA 120
TTGCCCCCGG CAATAAATTG TTTCTATATG CCAGAAA 157

SEQ ID NO:1256
SEQUENCE LENGTH:160
SEQUENCE TYPE:nucleic acid
TOPOLOGY:linear
CLONE:HUMGS01404
SEQUENCE DESCRIPTION:
GATCTTATTT GGAATTGACA TTCTCTATTG TAATTTTNTT CCTGTTTATT TTTAAATTTN 60

CTTTTTGTTT CACTGGAAAG GAAAGATGAT GCTCAGTTTT AAACGTTAAA AGTGTACAAG 120
TTGCTTTGTT ACAATAAAAC TAAATGTGTA CACAAAGAAA 160

SEQ ID NO:1257
SEQUENCE LENGTH:156
SEQUENCE TYPE:nucleic acid
TOPOLOGY:linear
CLONE:HUMGS01405
SEQUENCE DESCRIPTION:
GATCTTTGTN CTTGACCTCT AGACCCCAAG ATGTGAACAG TGCACGTTTT AATGTCATCT 60
TTNCTCATGT GTTATAAGCC CCAAGTTGCT GTATATTTNC ACAAGTATGT CTACACACTG 120
GTCATGATTT TNATAATAAA TAACGATAAA TCGAAA 156

SEQ ID NO:1258
SEQUENCE LENGTH:155
SEQUENCE TYPE:nucleic acid
TOPOLOGY:linear
CLONE:HUMGS01406
SEQUENCE DESCRIPTION:
GATCTTGCTC TGATTATAAT GCCAGTGAAT GTTGCTGAAC TCTTTGTATA TGCAAATTGC 60
AAGATTTAAA CCATTCTGAT GCAAGGATAA ACCTTTACTT TGACTACCAG CCTGTGTTTT 120
TGTCTTTAAA TCTCTTAATT TCATTCCTCT GCAAA 155

SEQ ID NO:1259
SEQUENCE LENGTH:155
SEQUENCE TYPE:nucleic acid
TOPOLOGY:linear
CLONE:HUMGS01407
SEQUENCE DESCRIPTION:
GATCTGCACT CCCACTCCCC CCACACTTCC CAAAGTGCTG GGATTACAGG CATAAGCCAC 60
AAGCCACCTC ACCCAGCCAA CATGTTACAT CTTAATTCTT GGATTTTCTT CACTGCAGGG 120
CTTTGGGTGG AGAAATAAAA CTCTTCAAAT GCAAA 155

SEQ ID NO:1260
SEQUENCE LENGTH:155
SEQUENCE TYPE:nucleic acid
TOPOLOGY:linear
CLONE:HUMGS01408
SEQUENCE DESCRIPTION:
GATCTGGGAG TGCTGGACGT CATCTTCCAC CNCACCCAGC CGTGGGTCTT CTCCTCGGGG 60
GCAGACGGGA CTGTCCGCCT CTTCACCTAG CTGTTCTGCC TGCCTGGGGC TGGGGTGGTC 120
GTGCTGAAGT CAACAGAGCC TTTACCCTGT GCAAA 155

SEQ ID NO:1261
SEQUENCE LENGTH:158

SEQUENCE TYPE:nucleic acid
TOPOLOGY:linear
CLONE:HUMGS01409
SEQUENCE DESCRIPTION:
GATCTTGGAG GCCAGCCAGC TGAAGGAGAC ACTGCAGGCG GTGCCCAAGC CAGGGGCCTT 60
TGACCTGGAG CAGGTGAAGC GTTCCACCTA CTTNTTCAGC TGACACCCCG TGAGCCTTGT 120
CAGTGTGTAA ATAAAGCTCT TTTGCCACCC CCAGGAAA 158

SEQ ID NO:1262
SEQUENCE LENGTH:151
SEQUENCE TYPE:nucleic acid
TOPOLOGY:linear
CLONE:HUMGS01410
SEQUENCE DESCRIPTION:
GATCCATCAT NGATGTAAAA GTTCACAATC ATGGTTCAAA TGTAACAGTG CAGAATTGAA 60
TATGGAGGCA TGCATAACCT TCCTCTTAGA AAATGGCAGG TGTTGTAATT TCAAATTTTT 120
GTGCAATTAG ATTAAATCAT AATGCAACAA A 151

SEQ ID NO:1263
SEQUENCE LENGTH:154
SEQUENCE TYPE:nucleic acid
TOPOLOGY:linear
CLONE:HUMGS01411
SEQUENCE DESCRIPTION:
GATCTGATGG GTTTATCAGG GGTTTCCACT TTTGTTTCTT CATTTTCTCT TGCCACCAGC 60
ATGTAAGAAG TGCCTTTGGT CTCCTACCAT GATTCTGAGG CCTCCCTAGC CATGTGGAAC 120
TGTAAGGCCA ATTAAACCTC TTTTTCTTCC CAAA 154

SEQ ID NO:1264
SEQUENCE LENGTH:154
SEQUENCE TYPE:nucleic acid
TOPOLOGY:linear
CLONE:HUMGS01412
SEQUENCE DESCRIPTION:
GATCAGCACC TTGTATAGGA ATTCCCATGA ATTATGACTT CTCATTCTGT TTTATCAGAG 60
TGCATATATG TCCTACTTCA GGAAAAGTAA AACAGTCATT TACGAAAGAA AGTCAATCTG 120
TATCCTAAGC ATTTTAATAA AAAGTTAAAA CAAA 154

SEQ ID NO:1265
SEQUENCE LENGTH:153
SEQUENCE TYPE:nucleic acid
TOPOLOGY:linear
CLONE:HUMGS01413
SEQUENCE DESCRIPTION:
GATCCTGGCC TGAGAATTGA GGGGAGGTGG CCAGCCCGCA GAGGTGGGGT GCTGGGGCTG 60

CATGATTTTN GCCCTGCGTC CCTTCTCTTT GGGGCTCCTT TCCCCTCTCA TACATAAAAT 120
CGCTTTCAAA TTAAAATCGC TGTTTTCTGG AAA 153

SEQ ID NO:1266
SEQUENCE LENGTH:163
SEQUENCE TYPE:nucleic acid
TOPOLOGY:linear
CLONE:HUMGS01414
SEQUENCE DESCRIPTION:
GATCCGAGTG AAGCCCCAGG TCTCANAGAG CAAGCTGTAG CCAGATGGTA CCAGCTTCGC 60
CTGGGGCTTC AAGAACCTCC CATCTATCCC CATTCCTGAN ACAGGGAGTT ACAGTCCCTT 120
TTTGGCCCTC ACATNCAATA AANGTGACTG ATANCACTGG AAA 163

SEQ ID NO:1267
SEQUENCE LENGTH:153
SEQUENCE TYPE:nucleic acid
TOPOLOGY:linear
CLONE:HUMGS01415
SEQUENCE DESCRIPTION:
GATCTGGCAA CACGTTTTNN TTGGAATATT TGTGTTTTCT TGAGGAGGTA TAATTACTGT 60
ATCCTAGGTG TGAATTTTTG AGTGCAGATG CACATTTTAA AGAAATNATG ATTAANCTGA 120
TAATGTTTTT NGGCTGAAAA TATAAAANNA AAN 153

SEQ ID NO:1268
SEQUENCE LENGTH:152
SEQUENCE TYPE:nucleic acid
TOPOLOGY:linear
CLONE:HUMGS01416
SEQUENCE DESCRIPTION:
GATCATAAAT CAAGGAATGC TGACAGCCAC TAAAGCTGGA GAAGGCCAAG GACAGATATT 60
CAATCCTCCC GTAGAAGTAC AGCCCTGCTA ATGCATTGAT TTTGGACTTC TGGCCTCAGA 120
ACTGTAAGAG AATAAATTTC TATTGTTTTA AA 152

SEQ ID NO:1269
SEQUENCE LENGTH:152
SEQUENCE TYPE:nucleic acid
TOPOLOGY:linear
CLONE:HUMGS01417
SEQUENCE DESCRIPTION:
GATCAGACCA ACAGTGCTGT TTCCCGGGAG GAACACTTTT TNATTNTTAC CCTTTGCAGC 60
TCCCACCTTT AATCTGTTTT ATACCTTGCT TATTAAATNN GCGCTTAAAT NATTGAAATA 120
ATGCTGNCTT AGTAGCAACT AAAATGTGNC TN 152

SEQ ID NO:1270
SEQUENCE LENGTH:156

SEQUENCE TYPE:nucleic acid
TOPOLOGY:linear
CLONE:HUMGS01418
SEQUENCE DESCRIPTION:
GATCCCCCCT ACCCACCCCA GTCCCAAATC CAGTCCTCTG GCCCTTGCCT AGCCCTGAAT 60
TGCTTCTCTA AGCTGGTGTT CCCATGCACA GGGCCATTCA GGAAGGGCTG GGGGAGTGTG 120
TGTGGCAATA AAGCTTGAAG GCACCGTGGG AGCAAA 156

SEQ ID NO:1271
SEQUENCE LENGTH:151
SEQUENCE TYPE:nucleic acid
TOPOLOGY:linear
CLONE:HUMGS01419
SEQUENCE DESCRIPTION:
GATCCTCTTC TCTCCAAATG TTAGCCATCC TGAAGTAGCC GAACAGTAGA AACTTTGGTG 60
GGGATTAACC GGGAGCTTGA AAATTTGTNT TTGGTAACCT GATACTGGAC AGCTGAACTG 120
AATGGCTGCA AAATAAATAC CTCACATGAA A 151

SEQ ID NO:1272
SEQUENCE LENGTH:93
SEQUENCE TYPE:nucleic acid
TOPOLOGY:linear
CLONE:HUMGS01420
SEQUENCE DESCRIPTION:
GATCCTGGAG GACCCTGGGC CCCAGGCCAG CTCCCATCGC TGGGGGACGG TGAACGGCCA 60
TGTGTTAATG TTACGATGTT TTTAAAAGAC AAA 93

SEQ ID NO:1273
SEQUENCE LENGTH:149
SEQUENCE TYPE:nucleic acid
TOPOLOGY:linear
CLONE:HUMGS01421
SEQUENCE DESCRIPTION:
GATCCCCTTT GTCAGTGGGG AAACCAAGGC AGAGCTGAGG GGACAGGGAG GAGCAGAAGC 60
CATCAAGATG GTCAAAGGGC CTGCAGAGGG AGATGTGGCC CTTCCTCCCC CTCATTGAGG 120
ACTTAATAAA TTGGATTNAT GACACCAAA 149

SEQ ID NO:1274
SEQUENCE LENGTH:151
SEQUENCE TYPE:nucleic acid
TOPOLOGY:linear
CLONE:HUMGS01422
SEQUENCE DESCRIPTION:
GATCTCAACG TGCTGATATG GAAAGTGCTT CAGAATGTAT TAAGGACATA AATTAAGTGT 60
ACAATAATGT GTGTGTGTGT ATATATGTAT ATGCTTACGT GTGTATGGAA AGTATCTCAG 120

CAGATACAAT AAAAACTTAA TTGTGATTAA A 151

SEQ ID NO:1275
SEQUENCE LENGTH:149
SEQUENCE TYPE:nucleic acid
TOPOLOGY:linear
CLONE:HUMGS01423
SEQUENCE DESCRIPTION:
GATCCTCCCT CTATTTTGCA AACAGTCTGT AAGTAACTNA TAAAACTTTA AAATATGCAA 60
ATTTTAAAAT TATATAGTTT GATTTACTCA TCAAATTATC ATGTATGCTG TTATTTAAGT 120
ATGAATAAAG GCTTTTTTAA ATNGGGAAA 149

SEQ ID NO:1276
SEQUENCE LENGTH:254
SEQUENCE TYPE:nucleic acid
TOPOLOGY:linear
CLONE:HUMGS01424
SEQUENCE DESCRIPTION:
GATCCTACTC CTTTGGAGTA AAACTAGTGC TTACCAGTTT CCAATTGTAT TTAGCTTCTG 60
GTTGGAATTT GAAAAAAAAA GAAAAAAAGA AAAAGAAAAC CTAAATAAAA TAGGTGAAAG 120
TTCCCTGACT ATTCAGGTGA ATACACAAAG TTTGAAGTGT TAACTTTTTC TTTCCATTTC 180
ACTGATGTTA CTGGTCACCT TAGAGAATTA TTTCATAGTC TGTGGCTAAA TAGTAAATTC 240
AGANGAAAAA TAAA 254

SEQ ID NO:1277
SEQUENCE LENGTH:148
SEQUENCE TYPE:nucleic acid
TOPOLOGY:linear
CLONE:HUMGS01426
SEQUENCE DESCRIPTION:
GATCCTACTG TTGAGTTAGG AAAATATGGT TAGACAGACT CACATTACTT TTTTTCAGAG 60
GTAAACTCTA GATTACTGTG TCAACCCAAT ACTATTTGGC CATAGATGTA AAAACTACCA 120
AATAAAAGTG GATTTTGTGG TCTACAAA 148

SEQ ID NO:1278
SEQUENCE LENGTH:148
SEQUENCE TYPE:nucleic acid
TOPOLOGY:linear
CLONE:HUMGS01427
SEQUENCE DESCRIPTION:
GATCCACAGT GGAGTCTCAG TAATTATATC TCCTTGATTT CTTCATTTNC TCTTCTGCTA 60
TAAAAGTAGA GATAATGTGT AGTCACTNCT CATTTAGTGA ACCAATTGTN ATANTTCTGG 120
AAATCTNTTN TCTTTAAGTG TAAATANN 148

SEQ ID NO:1279

SEQUENCE LENGTH:124
SEQUENCE TYPE:nucleic acid
TOPOLOGY:linear
CLONE:HUMGS01428
SEQUENCE DESCRIPTION:
GATCCCAATC AGATTCCCGC TAATGGAAGA AGTTTAGAAT CTTTCAGGTG GAATAAAGTC 60
ACATGAAAAC AAAACACAAC TATATATATT TCCAGTTTTT TTGCCTTATT GATTTTTTNC 120
CAAA 124

SEQ ID NO:1280
SEQUENCE LENGTH:147
SEQUENCE TYPE:nucleic acid
TOPOLOGY:linear
CLONE:HUMGS01429
SEQUENCE DESCRIPTION:
GATCTTTGGG GAGATACATC TTATAGAGTT AGAAATAGAA TCTGAATTTC TAAAGGGAGA 60
TTCTGGCTTG GGAAGTACAT GTAGGAGTTA ATCCCTGTGT AGACTGTTGT AAAGAAACTG 120
TTGAAAATAA AGAGAAGCAA TGTGAAA 147

SEQ ID NO:1281
SEQUENCE LENGTH:147
SEQUENCE TYPE:nucleic acid
TOPOLOGY:linear
CLONE:HUMGS01430
SEQUENCE DESCRIPTION:
GATCCTGACA CTAAGGAATG CTGAAGCTTT GGACTTCGGC AGTCTTCCAC CTTCAGTCAT 60
CAGCTACAGT TGGAATGAAT TTNAAAACGC TCGGGACCTG TTAATNTNTC CTGTAGNCTG 120
TATTATNTNA AAAATCTGGG CAACAAA 147

SEQ ID NO:1282
SEQUENCE LENGTH:147
SEQUENCE TYPE:nucleic acid
TOPOLOGY:linear
CLONE:HUMGS01431
SEQUENCE DESCRIPTION:
GATCTCGCTC CCCCCTTCGG TTCTTTCGAC CGGTCCCCCC TCCCTTTTTT GTTCTGTTTT 60
GTTTTGTTTT GCTACGAGTC CACATTCCTG TTTGTAATCC TTGGTTCGCC CGGTTTTCTG 120
TTTTCAGTAA AGTCTCGTTA CGCCAAA 147

SEQ ID NO:1283
SEQUENCE LENGTH:146
SEQUENCE TYPE:nucleic acid
TOPOLOGY:linear
CLONE:HUMGS01432
SEQUENCE DESCRIPTION:

GATCTCAGTT CCTGGCTTTC CCCCCAGCCT TCTCACCCTT TGTGTCTGTG TAGTGATTTG 60
GTGAGAAATC GTTGCTGACC CTTCCCCAGA CCATTTATGA GTCNAAGTTT ATTATTCAAT 120
AAAAGTGCTN TATGCGCTTT CTCAAA 146

SEQ ID NO:1284
SEQUENCE LENGTH:146
SEQUENCE TYPE:nucleic acid
TOPOLOGY:linear
CLONE:HUMGS01433
SEQUENCE DESCRIPTION:
GATCCAAAGA TGAGGGGATT NTTTCAGAAA GACAATCTCG GCATGCATTA TTTCTTTNNT 60
TTGAAGATTC ACTCATGTTG CATGCATCTN TAGCTTGTNC CTTTTTAATT CCTAGTAGAT 120
TCTGTCATAT GCCTATCTNC AATTNN 146

SEQ ID NO:1285
SEQUENCE LENGTH:167
SEQUENCE TYPE:nucleic acid
TOPOLOGY:linear
CLONE:HUMGS01434
SEQUENCE DESCRIPTION:
GATCCCCATG CTAAGAAAAT GCCCAAAAAA ATAGGCAAAA CACGAGAAGA GCTAGGGTAA 60
GAGAAGGACG TAAACAGAAC CTGACACCAG CTCCTTTTCC TTCTATACAT TATTTAATAC 120
CTATTAAATA AAATNATTTT TGGAATAAAG CTTGTGGGAA CATTAAA 167

SEQ ID NO:1286
SEQUENCE LENGTH:179
SEQUENCE TYPE:nucleic acid
TOPOLOGY:linear
CLONE:HUMGS01435
SEQUENCE DESCRIPTION:
GATCAAAAAG GCTTATAAAA CAGAGTAATC TTGTTGGTTC ACNCTTGNGA CCGTGAAGAT 60
ACTTTGTATT GTCCTATTAG TGTTATATGN ACATNCAAAT GCATCTTNCA TGTGTTGTTC 120
TTGGCAACAA ATTTTGAAAA GTAATATTTA TTAAATNTTT TTGTATGCAA ACATGCAAA 179

SEQ ID NO:1287
SEQUENCE LENGTH:145
SEQUENCE TYPE:nucleic acid
TOPOLOGY:linear
CLONE:HUMGS01436
SEQUENCE DESCRIPTION:
GATCCGATGG GAGTGTAAAT GTGAGACACA ATGTCTTGAT TATACCTGTT TGTGGTTTAG 60
CTTTGTATTT AAATAAGGAA ATAAACTTGA AAATNATTTG TCATCATAAA AATGAAACAA 120
ATNAAAATAT TTATTGCCAG GCAAA 145

SEQ ID NO:1288

SEQUENCE LENGTH:145
SEQUENCE TYPE:nucleic acid
TOPOLOGY:linear
CLONE:HUMGS01437
SEQUENCE DESCRIPTION:
GATCAGGGCA TGCAACCAAA AGCAGCTTAA ATGAAATATT TTAAAATAAA ATATCAGGAA 60
GCTATTTTTA GATTTCTNCT GGCTTATGTT TCTACTTTAG GACCCTCATT GTNCTCTTAT 120
TAAAAAAAAT TATTTCCTGT GCAAA 145

SEQ ID NO:1289
SEQUENCE LENGTH:145
SEQUENCE TYPE:nucleic acid
TOPOLOGY:linear
CLONE:HUMGS01438
SEQUENCE DESCRIPTION:
GATCAGATTT TTTTTGTACC TACGTAAGAG TACTTGAAGT TTTATTTAAA ATAAAATGTT 60
GTGGAAAAGG TAGCATTCTT TTTTTAGGAG TGTTATTTTT CACTATGTGT GGCACGGATA 120
CAATAAAAGA CTTTTACAAA CTAAA 145

SEQ ID NO:1290
SEQUENCE LENGTH:145
SEQUENCE TYPE:nucleic acid
TOPOLOGY:linear
CLONE:HUMGS01439
SEQUENCE DESCRIPTION:
GATCCCAGGG CTCCCTGCCA TTTTAGTGTC TTGGTGTAGT GTAACCATTT AGTGGTTGGT 60
GCCATTTTTT TTTTTGTNCA AATGATTTAA ATNATTGGAA TNCACAATTT TTTAAATATN 120
CAAATAAAAN GTTTAAAACC TTAAA 145

SEQ ID NO:1291
SEQUENCE LENGTH:145
SEQUENCE TYPE:nucleic acid
TOPOLOGY:linear
CLONE:HUMGS01440
SEQUENCE DESCRIPTION:
GATCAGGAAT TCTAAATAAA TTTCCCAGTT AAAGATTATT GTGACTTCAC TGTATATAAA 60
CATATTTTTA TACTTTATTG AAAGGGGACA CCTGTACATT CTTCCATCAT CACTGTAAAG 120
ACAAATAAAT GATTATATTN ACAAA 145

SEQ ID NO:1292
SEQUENCE LENGTH:144
SEQUENCE TYPE:nucleic acid
TOPOLOGY:linear
CLONE:HUMGS01441
SEQUENCE DESCRIPTION:

GATCTGCCAG CACCCTGTGG GGCCCAGACT ACAGGCTGAT GGCGGAGGCT TCGAGTGACC 60
CGGGTGCCGA GGAGCGGGAA GAGTTGCTGG GGTAAGGGTC TGCGGCGACG CCGGCGCCCT 120
GTGCCGTGTC CACGGGTGCA TAAA 144

SEQ ID NO:1293
SEQUENCE LENGTH:144
SEQUENCE TYPE:nucleic acid
TOPOLOGY:linear
CLONE:HUMGS01442
SEQUENCE DESCRIPTION:
GATCTAAATA TTTTNNAGCT GAGTTATTAG GGAGTCATTA TTCTGTGGTA CAATGCTGCA 60
AAAAGCATCA TGTGGAAGAA TGGGAACTAT GCTTACTTTA TGAAGTGATG TATAACACAA 120
TGAAATCTGT TTTACAACTA CAAA 144

SEQ ID NO:1294
SEQUENCE LENGTH:372
SEQUENCE TYPE:nucleic acid
TOPOLOGY:linear
CLONE:HUMGS01443
SEQUENCE DESCRIPTION:
GATCTTGGTA GTATCCCTGG CTGCTAAGCC TATGGCAGGG GTGGTCCTTT CTACATTCCC 60
ATTCACTTAA CAGCTCTTTT GGGATTGGGT GTTTCATTCC ATTTCTGCCC ACTCCCTCTC 120
CTCTCCTCTC TGGTAGGTTT AATTTATGC TTTCCCTGAT TCCAGCTTTC TGCTTCCTGA 180
GGACTCCCGC TCCCNCCACC CCAAAGTTTG TCTGTGGGTG TTATAGTGGT AACTGCAGTT 240
CCCTCCTCTG GGAATNGTAG GCTGTAATAG GNTTAATAAA CTCNACNTNC TCTAATCNTT 300
GCTNAAAANG TGGGGGTAAN GGGGATGGCN TTGNCCCNGG GTGGGAAANT TAAANNTGGT 360
GTTTANGGNA AA 372

SEQ ID NO:1295
SEQUENCE LENGTH:145
SEQUENCE TYPE:nucleic acid
TOPOLOGY:linear
CLONE:HUMGS01444
SEQUENCE DESCRIPTION:
GATCTTCTCT GTGGGGGAAA AGGAAGAGGA GGGTCTTGTT CTCCCANTNT GTTTATTCTT 60
TGGGCTCTGG GAACAGGGGA CTACTTTGGG GCTTTCTCCA ATNCTNTTGT ATGNTGTTAT 120
TAAAAGCGAG CTATTGCATT TCAAA 145

SEQ ID NO:1296
SEQUENCE LENGTH:141
SEQUENCE TYPE:nucleic acid
TOPOLOGY:linear
CLONE:HUMGS01445
SEQUENCE DESCRIPTION:
GATCTTATAT CCTGTGACCT GTCTAAATTC AATCTGTTAG TTTTATCATT TTTTAAAAAA 60

ATGTCCGTGT GTGTCTTCCT TGAGATTTTC TACATTATCA TGTCATCTGC ANATAAAGAC 120
ATTTACTTCT TTCTTTCCAA A 141

SEQ ID NO:1297
SEQUENCE LENGTH:141
SEQUENCE TYPE:nucleic acid
TOPOLOGY:linear
CLONE:HUMGS01446
SEQUENCE DESCRIPTION:
GATCCAGTTG TACTAAGGTG TACAGGATAT TTGCAGATAT AGGTTAACTG AATGAAGCAT 60
ATTAATAACT GCATTTNCCT AACTTTGAAA AATTTTNCAA ATGTCTAGGT GATTTAAAAA 120
ATGAGATTGG GCTATTGCAA A 141

SEQ ID NO:1298
SEQUENCE LENGTH:140
SEQUENCE TYPE:nucleic acid
TOPOLOGY:linear
CLONE:HUMGS01447
SEQUENCE DESCRIPTION:
GATCTAAAAT AGATATGTTG GATTGATATT CTCACGTGTT CGGTGTGGAA AACAAAACAA 60
GTACATGCTT TAGAAGCAAC AACAATGAAA TCCTTTTGAA ATNTGTGTTA ATATCGTTTA 120
NTAAAATACC TAGTTTGAAA 140

SEQ ID NO:1299
SEQUENCE LENGTH:140
SEQUENCE TYPE:nucleic acid
TOPOLOGY:linear
CLONE:HUMGS01448
SEQUENCE DESCRIPTION:
GATCCATAGA AGGGCTTCCC AAACCTTGTT TTGCAACATC CCAAATTGTC TCCAGTTGAA 60
GGAAGGCCTT TATCAGATTC ATAGATGAGC TTTCATTGTA AAAATAAATG TACTTTGCAC 120
CACTTCATGA TGGAGGGAAA 140

SEQ ID NO:1300
SEQUENCE LENGTH:140
SEQUENCE TYPE:nucleic acid
TOPOLOGY:linear
CLONE:HUMGS01449
SEQUENCE DESCRIPTION:
GATCTGAGGT TTTTAGATTT TAAATATTTA TGTGGAATTA ATTAAAGGTA GTTGGCTATA 60
TCGCTATCAT TTCATTCTTT TGACATTATT TGAATATTTT ACTGGAAAAT AAGACTAATA 120
AATTGTTAAA AGTTTTTAAA 140

SEQ ID NO:1301
SEQUENCE LENGTH:139

SEQUENCE TYPE:nucleic acid
TOPOLOGY:linear
CLONE:HUMGS01450
SEQUENCE DESCRIPTION:
GATCTTCACA AAGTTGTCTT TTCACTGTGT TTTGTCAACG TGAAATTAAA TTGTAGTTAT 60
AAGCAAAAGT TGGTTGCCTA GGGAACAATT GTATATNCAG TTTAACAGAA ATAAAAGAAT 120
ATTTGTCTTA AGATGCAAA 139

SEQ ID NO:1302
SEQUENCE LENGTH:138
SEQUENCE TYPE:nucleic acid
TOPOLOGY:linear
CLONE:HUMGS01451
SEQUENCE DESCRIPTION:
GATCGNCACA TTTCCCAAAA ATAATAAAAA AATCACTAAC CTTTTTTAAG GAAAATATTT 60
AAAGTTTTAC AAAATTCAAT ATTGCAATNA TCAATGTAAA GTACATTTNA NTGCCTTATN 120
AAAACTTTCC CAATTAAA 138

SEQ ID NO:1303
SEQUENCE LENGTH:137
SEQUENCE TYPE:nucleic acid
TOPOLOGY:linear
CLONE:HUMGS01452
SEQUENCE DESCRIPTION:
GATCATTTTT TCGGTCTCCG AGGTGAAATG ACTTATTAAT TAAAATTTGT AAACTCACAT 60
ATGCATATTG TATATGTGTA GAAATGTAAT CACACTTTGT CTTGGAATTA CATTAAACTG 120
TTTGAAATCA CTGTAAA 137

SEQ ID NO:1304
SEQUENCE LENGTH:136
SEQUENCE TYPE:nucleic acid
TOPOLOGY:linear
CLONE:HUMGS01453
SEQUENCE DESCRIPTION:
GATCTTTTTG TGATTGCTTT CCCGTCTCTA AGTAGTATTC TGTTGTGTCT AGAGAACTGA 60
TTTTTTCCTA CATACGCAAA TTGTACATTT GTAAGTGAAA ATNTCAATAC ATTAAAAGCA 120
TTANCCTAAA ANCAAA 136

SEQ ID NO:1305
SEQUENCE LENGTH:136
SEQUENCE TYPE:nucleic acid
TOPOLOGY:linear
CLONE:HUMGS01454
SEQUENCE DESCRIPTION:
GATCAGCTGG GTTTTGGCCA GGAAGTTGTC TTTGTGGACT CTGCCTGCAT GGCTTAGTAG 60

TTGAAGGAAA TTTTTTTTTG GTTTTGTTTT TTATAATTCA GTTTAATCAA TAAACATGTA 120
TTTATTGACT GTTAAA 136

SEQ ID NO:1306
SEQUENCE LENGTH:136
SEQUENCE TYPE:nucleic acid
TOPOLOGY:linear
CLONE:HUMGS01455
SEQUENCE DESCRIPTION:
GATCCCTTTA TGAGTTTTGC TATTTTGGCA TGCTACATGT AAGTTTTGCT TAATGTTTTT 60
ATGTAAAGCA TCTTCCTCCT TTTACTTCTT TTATTGTGAT AAAACTCATA TAAAATTGAC 120
CGTTTTGAAG TGTAAA 136

SEQ ID NO:1307
SEQUENCE LENGTH:136
SEQUENCE TYPE:nucleic acid
TOPOLOGY:linear
CLONE:HUMGS01456
SEQUENCE DESCRIPTION:
GATCTGGGCC CGACCCTCAC TACCCCTGAG ATATTAGTTC CCAGGCCTGT TTTCCCACAG 60
GATTGTGGGC TCTCTGCTTC CTTAGTCGGA AGTNTTTTCA ACTAATCAAA TAAATGAATG 120
AATGATGAAT AAGAAA 136

SEQ ID NO:1308
SEQUENCE LENGTH:135
SEQUENCE TYPE:nucleic acid
TOPOLOGY:linear
CLONE:HUMGS01457
SEQUENCE DESCRIPTION:
GATCCCAAAG ATATTAAATA TATGCAAATA TTCCAAAGTC TGAAAAAATC CAACATCCAA 60
AAACACTTCT GACCCAAGCA TTTCAGATAA GGGACCAGAA TTATTAGATT AAATAAGGTA 120
TATTATTAAG TTAAA 135

SEQ ID NO:1309
SEQUENCE LENGTH:135
SEQUENCE TYPE:nucleic acid
TOPOLOGY:linear
CLONE:HUMGS01458
SEQUENCE DESCRIPTION:
GATCAGGCCA AGAGGTTGCA CAGGGCATGA TACTGCACCC TGCCTGACCC AGCTGGGCTC 60
ACAGGTCAGG GGAGAGTTGG GGCAGGTGAA TGTCAGCATC AGGCTTCTNN TTTTAAACTT 120
TTAAANAAAT ACAAA 135

SEQ ID NO:1310
SEQUENCE LENGTH:135

SEQUENCE TYPE:nucleic acid
TOPOLOGY:linear
CLONE:HUMGS01459
SEQUENCE DESCRIPTION:
GATCCTGCCT CAAAAAAAAC AAAAAAATTC TTTATTTTCT CCATAAACTA CAGTTTATAT 60
AAGCAAAAGT TTCAGTACTA AGCAATTTNA GTCTCTGCAG TCTCTNGTNT TGANTTAATA 120
CAACTTTNGT NAATN 135

SEQ ID NO:1311
SEQUENCE LENGTH:139
SEQUENCE TYPE:nucleic acid
TOPOLOGY:linear
CLONE:HUMGS01460
SEQUENCE DESCRIPTION:
GATCTACCTG TGGCCCGGCC TCCCTAATGT NATTCACATT GAATGGGGAT GAGGTCGGAC 60
AGTGGCTCAT AGAGCGAGTA TGAGCCCTAG CTGTGGGCTA GAAATNTCCT TAATAAACAT 120
CCTTATTTTC NTNTTTAAA 139

SEQ ID NO:1312
SEQUENCE LENGTH:134
SEQUENCE TYPE:nucleic acid
TOPOLOGY:linear
CLONE:HUMGS01461
SEQUENCE DESCRIPTION:
GATCCTAGTA TGTTGATAGC TTTNATTTTG TGAGTGGTTT ACTAGTCATT TATTATGATT 60
CCCATGANCT CTGATATGAT TCATTGTGGT TTTAACTCAG GTTGAATAAA AGCATCCATT 120
TCTTTTATAG GAAA 134

SEQ ID NO:1313
SEQUENCE LENGTH:237
SEQUENCE TYPE:nucleic acid
TOPOLOGY:linear
CLONE:HUMGS01462
SEQUENCE DESCRIPTION:
GATCTCAAGG TTGATAGTGG TGTTCACTAG GAGACGTGGA ATTGAGACTA ATAACTTGGA 60
TGTTAACACT GTTTACTGTT TTTTCACATG TAGAAATGTT CTTTGTGTAT TTTTTCTACA 120
GAGGATTTTC TCTGATTTTA TTTTCTTTGT TTCTGACTCT AATAATTAGT TGGAAACTCA 180
TATAAAATGA GCTTTCCTAA ATTAAATCTA TTTTAAATAA AGGTTATTAC TATTAAA 237

SEQ ID NO:1314
SEQUENCE LENGTH:134
SEQUENCE TYPE:nucleic acid
TOPOLOGY:linear
CLONE:HUMGS01463
SEQUENCE DESCRIPTION:

GATCTATAGG TTTATTAGAA NCCTNACCAC AGNATCCAGA TTATCTTCAG TATTCTNTCA 60
GTACAGCTCT CTGCAGCTTA AACTCGGTGG TACATAAAGA AGATGATGAA CCCAAAATGN 120
TGGNCACTGT ATAN 134

SEQ ID NO:1315
SEQUENCE LENGTH:133
SEQUENCE TYPE:nucleic acid
TOPOLOGY:linear
CLONE:HUMGS01464
SEQUENCE DESCRIPTION:
GATCAGAAAA TCCCTCTGAC ATCTCCACTG CCCCCAAAGA CCTCCGTTGA ACATTCTGTA 60
TGGAAAAGAG CCCTGGAGCA TCAGGTTCCC CAGATAGGCC CCCAAATAAA GACCTGTCTA 120
TGGCTCTCCC AAA 133

SEQ ID NO:1316
SEQUENCE LENGTH:132
SEQUENCE TYPE:nucleic acid
TOPOLOGY:linear
CLONE:HUMGS01466
SEQUENCE DESCRIPTION:
GATCTAGCTG GCTGTAGTAT TGCNTTGATA ATTTTTTTCT TTTAATTTAC CTAATATATA 60
TAAGGAAGGG GTTTGGATAT ATTAAAATAG GTGTTAATTT TATCTATTTA CCAATAAATT 120
CATCTCTTTA AA 132

SEQ ID NO:1317
SEQUENCE LENGTH:130
SEQUENCE TYPE:nucleic acid
TOPOLOGY:linear
CLONE:HUMGS01467
SEQUENCE DESCRIPTION:
GATCCAGCCA TTCCTGAAGC CCACCCTGCA CCTCATTCCA ACTCCTACCG CGATACAGAC 60
CCACAGAGTG CCATCCCTGA GAGACCAGAC CGTTCCCCAA TACTCTCCTA AAATAAACAT 120
GAAGCACAAA 130

SEQ ID NO:1318
SEQUENCE LENGTH:130
SEQUENCE TYPE:nucleic acid
TOPOLOGY:linear
CLONE:HUMGS01468
SEQUENCE DESCRIPTION:
GATCTAAAAG CCAACTTTTT CTCAGTNTTA CTCAGTGGAA AGATAAACTA AGTTTTAATG 60
TTATNTTTTT AAATNTAAGC AAAATTTATT TCTGTNCTNT AATAAATAAG AAAATGTGGT 120
CCACTGCAAA 130

SEQ ID NO:1319

SEQUENCE LENGTH:129
SEQUENCE TYPE:nucleic acid
TOPOLOGY:linear
CLONE:HUMGS01469
SEQUENCE DESCRIPTION:
GATCCCCTGC TCCTGTTGTG TTCTGTTGTA AATCATTTGG CGAGACTGTA TTTTAGTAAC 60
TGCTGCCTAA CTTCCCTGTG TTCTATTTGA GAGGCGCCTG TCTGGATAAA GTTGTCTTGA 120
AATTTCAAA 129

SEQ ID NO:1320
SEQUENCE LENGTH:128
SEQUENCE TYPE:nucleic acid
TOPOLOGY:linear
CLONE:HUMGS01470
SEQUENCE DESCRIPTION:
GATCAGAATT TTTAAAGCAG AATTTTGTCA AAAGGGTCAT TTTTTTGTCT ACCCCTTTTA 60
CACTTTTCAG ATTCTCAAAG TGTCTCATCT CAACTTTTAA AAGAATAAAG AATATCTTGC 120
TGGGCAAA 128

SEQ ID NO:1321
SEQUENCE LENGTH:128
SEQUENCE TYPE:nucleic acid
TOPOLOGY:linear
CLONE:HUMGS01471
SEQUENCE DESCRIPTION:
GATCCTTGTC AGATGAAAAT GGATTCACAG CTCTGGCAGT TCCCAATGTC TGGGGAGGGG 60
TATAGGTTTG AAAGGCTGTT TGAAAGAGGA ATGTTTAATA AAGGCTTTGA TTTAATCTTG 120
AAAAGAAA 128

SEQ ID NO:1322
SEQUENCE LENGTH:128
SEQUENCE TYPE:nucleic acid
TOPOLOGY:linear
CLONE:HUMGS01472
SEQUENCE DESCRIPTION:
GATCTCGAAG TNACTCCGGG CTGAGCAGTG GGGCGGCTGG GGGAGGGGTG ACGATTCTCC 60
TCAGGCTTTG GCCCTGCAAG CAAACCCACA TATCTNCTCT GTATGTAATA AATNTNTTAA 120
CGTCGAAA 128

SEQ ID NO:1323
SEQUENCE LENGTH:129
SEQUENCE TYPE:nucleic acid
TOPOLOGY:linear
CLONE:HUMGS01473
SEQUENCE DESCRIPTION:

GATCCTGAAG CNGCACTTAC CTNTGAANAG TCTTCAAACT TTTAAACCTT GCCAGTNAGG 60
ACTTTTNCTA TTGCAAATAG AAAACCCAAC TCAACCTGCT TAAGCAGAAA ATAAATTTAT 120
TGATTCAAA 129

SEQ ID NO:1324
SEQUENCE LENGTH:127
SEQUENCE TYPE:nucleic acid
TOPOLOGY:linear
CLONE:HUMGS01474
SEQUENCE DESCRIPTION:
GATCTGTGAA GTTATTTTGT AAGGACATAC ATTTGGTAAG TAAGTTTGTG TCCCAGGAAA 60
TGTATGTNTT TTTAAACCCT TTCTAAATAT GCAGGCCATT AATAAATAAG ATTGTTTCTT 120
CCCTAAA 127

SEQ ID NO:1325
SEQUENCE LENGTH:127
SEQUENCE TYPE:nucleic acid
TOPOLOGY:linear
CLONE:HUMGS01475
SEQUENCE DESCRIPTION:
GATCAGAGAG ATTTTTNTTT TAAACTACCA TGGTCCCAGG ATTCCATCCT GAAATNAATT 60
TTCCTTTGTA TGAATATGTG TAAATAATTT AAAAATAAAA CTGTAAAANA TTTGTNCGAA 120
GAATAAA 127

SEQ ID NO:1326
SEQUENCE LENGTH:137
SEQUENCE TYPE:nucleic acid
TOPOLOGY:linear
CLONE:HUMGS01476
SEQUENCE DESCRIPTION:
GATCCCCCTT CATTTGATGT TTGGAAAATN CCAGTAATTA TCATTTTTGC AACGAATATG 60
GATACCACAT AGTACTTTGG TGTTACCTGC TTTTGAAAAA TAAAGTCTTT GGTTCACCCG 120
GTGAACTATT TATGAAA 137

SEQ ID NO:1327
SEQUENCE LENGTH:126
SEQUENCE TYPE:nucleic acid
TOPOLOGY:linear
CLONE:HUMGS01477
SEQUENCE DESCRIPTION:
GATCCTCAGC CTCCCAGTGG TCTTTGTAGA CTGCCTGATG GAGTCTCATG GCACAAGAAG 60
ATTAAAACAG TGTCTCCAAT TTTAATAAAT TTTTGCCCAT TTCTTATTAA AAAAACTTGT 120
TGTAAA 126

SEQ ID NO:1328

SEQUENCE LENGTH:129
SEQUENCE TYPE:nucleic acid
TOPOLOGY:linear
CLONE:HUMGS01478
SEQUENCE DESCRIPTION:
GATCCATCTG TGTAGTTTCT GAACAGTCAG CGATTCCAGG TTTTAAATAG TTTGTAAATT 60
TTCAGTTTCT ACACACTTTA TCATCCNCTC GTGATTTTTT AATTAAAGCG TTTTAATTCC 120
TTTCTCAAA 129

SEQ ID NO:1329
SEQUENCE LENGTH:125
SEQUENCE TYPE:nucleic acid
TOPOLOGY:linear
CLONE:HUMGS01479
SEQUENCE DESCRIPTION:
GATCCATGCT TTACTGTGTT TAATGGGGGT AACAGGGGTC CCTACAGCCC TCCCAGCTAA 60
ACATTTGGAA CAAAACACCA GCCCTTTTGT AGTGGATGCA GAATAAAATT GTTAATCCAA 120
TCAAA 125

SEQ ID NO:1330
SEQUENCE LENGTH:124
SEQUENCE TYPE:nucleic acid
TOPOLOGY:linear
CLONE:HUMGS01481
SEQUENCE DESCRIPTION:
GATCTGTGTT GAAAGTNGTA TATTTTTATC TGTNCGGTGC TGAGTGCAGG CCACCAGCTC 60
CTAAATAGAG GTTCCCTATA TGCGCGTATG ACATGGTGAA TAAACACAAC TCTCTCCACT 120
CAAA 124

SEQ ID NO:1331
SEQUENCE LENGTH:124
SEQUENCE TYPE:nucleic acid
TOPOLOGY:linear
CLONE:HUMGS01482
SEQUENCE DESCRIPTION:
GATCGTTGTA ATTNNTTGAC ATTCCTTTNA GAAGTTGTGA AATGTTACAA CTTGTNCTTA 60
TGTAGACACA ATCTCCTGTC TCAGTACAGA GGCACTGACT TCAATAAAGT CTATTTATAC 120
TAAA 124

SEQ ID NO:1332
SEQUENCE LENGTH:129
SEQUENCE TYPE:nucleic acid
TOPOLOGY:linear
CLONE:HUMGS01483
SEQUENCE DESCRIPTION:

GATCAAAAAA TGGAAATGTA TAATTAAATC ATACTTAGCA AATCTAACAC ATGAAATGTA 60
ACATCTGCAT ATGGAGAATC GTGTTACTTT ATTGAAAAAC ATTAAANGTT TGAGANCTTA 120
AGTTGGAAA 129

SEQ ID NO:1333
SEQUENCE LENGTH:122
SEQUENCE TYPE:nucleic acid
TOPOLOGY:linear
CLONE:HUMGS01484
SEQUENCE DESCRIPTION:
GATCTGCCAA GATTCTTTGA ATACAGATAA TTAATGTAAA CAATTATCAT AAGTATACTA 60
ACATGTTATN CTTTTTAAAT AAGAAGGTAT AATAAAATAT CCCATTGGTT TNATGTATTA 120
AA 122

SEQ ID NO:1334
SEQUENCE LENGTH:122
SEQUENCE TYPE:nucleic acid
TOPOLOGY:linear
CLONE:HUMGS01485
SEQUENCE DESCRIPTION:
GATCGGGCCN TTGGCTGTAA GACCCGACCC TTCGAGAACC CGANACGAAA CGCTCCATTA 60
CCACTGCNCA GTGAGATGAG GGACTCACAG TTCCAAGAGG NTTCTTTCCC GTGGNCCCCT 120
NN 122

SEQ ID NO:1335
SEQUENCE LENGTH:121
SEQUENCE TYPE:nucleic acid
TOPOLOGY:linear
CLONE:HUMGS01486
SEQUENCE DESCRIPTION:
GATCCAACCC TGTACTGATA GTACTTCCCA GTATGATATT GTGATGTTTC ATACAATGCA 60
GTGAACATAA CCAACTTGTT ACCTAAATAA AGANTTGATA AAAACAGTGT GACATATTAA 120
A 121

SEQ ID NO:1336
SEQUENCE LENGTH:120
SEQUENCE TYPE:nucleic acid
TOPOLOGY:linear
CLONE:HUMGS01487
SEQUENCE DESCRIPTION:
GATCAAGGTC TGCAAGGGAA TTCTTGTGTG CTGCTTTCCA TTTGACACCG CAGTTCTGTT 60
CAGCCATCAG AAGAGAGACA AGGNATTAAA AATTTCTTTT TAATCNNGTT ACCAAATAAA 120

SEQ ID NO:1337
SEQUENCE LENGTH:119

SEQUENCE TYPE:nucleic acid
TOPOLOGY:linear
CLONE:HUMGS01488
SEQUENCE DESCRIPTION:
GATCTAGTAG GCACGTCTGT CAACAGGACA CATGCCTCCT CTGACTATAA CCTCTTAATA 60
GTTGTGTATA ATGAAAACTG TAAACTTTTT TAAATAAACN GTGTATATAC CTTGGCAAA 119

SEQ ID NO:1338
SEQUENCE LENGTH:120
SEQUENCE TYPE:nucleic acid
TOPOLOGY:linear
CLONE:HUMGS01489
SEQUENCE DESCRIPTION:
GATCCTGAAG CCATGGTTTC TTCCCTGCCA GAAATGAAAG GTTCAGTTAT GAGGCAACCC 60
TCTAGTAAGG CATTGTAAAA GTTACTGGAT TTGGTTTAAT AAAAGTTGAA ATAAAGTAAA 120

SEQ ID NO:1339
SEQUENCE LENGTH:322
SEQUENCE TYPE:nucleic acid
TOPOLOGY:linear
CLONE:HUMGS01490
SEQUENCE DESCRIPTION:
GATCTTCCGA GTGCTTGGGG CCCACAGAGA CATCATCCTG GAGAGCATTC CCACTGACAA 60
CCCAGAGGCG CACAGCAACC TCTACATCCT CACGGGCCAC CAGAGCACCT ACTAAGAGCA 120
GCGGGCCTGT CCAGGGGCTC CCCGNCCCAC CCCACGCCTT AGCTGCAGGC CCTTTTGGGC 180
AAAGGGGCCC ATCCTGGGCC ATCCATTCCA TTTTGTTCCA CATTTCCTTT CTACTCTTTC 240
TGCCAAGAGN CTGCCCCTGC ATTTGTCCTG GGAAACACGG TATTTAAGAG AGAACTATAT 300
TGGTATTAAA GNTGGTTTGT TN 322

SEQ ID NO:1340
SEQUENCE LENGTH:118
SEQUENCE TYPE:nucleic acid
TOPOLOGY:linear
CLONE:HUMGS01491
SEQUENCE DESCRIPTION:
GATCTTTGGA GCAAAAGCCA ACGGCAGGAA AAAATAGTTT GTACCAGTTT CATGAAGTAT 60
GTCTTTGGGT TTTTGTAAAT AATTTTAACT CAAATAAAAT TGCTACTTTC AATACAAA 118

SEQ ID NO:1341
SEQUENCE LENGTH:118
SEQUENCE TYPE:nucleic acid
TOPOLOGY:linear
CLONE:HUMGS01492
SEQUENCE DESCRIPTION:
GATCAGAATN AAATNTGTAT AGAGCAGAGT TTTAAAATGA ATGTAAATAG CACTAAACGT 60

NTNCTTTCTG CAACCTGTAC TTACAGATTC TCCCTGTAAA CTAAATAAAA AAAAANTN 118

SEQ ID NO:1342
SEQUENCE LENGTH:118
SEQUENCE TYPE:nucleic acid
TOPOLOGY:linear
CLONE:HUMGS01493
SEQUENCE DESCRIPTION:
GATCACAAAA TGTGCATGAG NGTTATTNAC TTTATTCTNG TACAGTACTA GGATTCCTGT 60
AACCACTCTT TTTTTTCTTC GNNGTATTGA AAACTGGTTC AGTGTTAACC AGGCAAGN 118

SEQ ID NO:1343
SEQUENCE LENGTH:118
SEQUENCE TYPE:nucleic acid
TOPOLOGY:linear
CLONE:HUMGS01494
SEQUENCE DESCRIPTION:
GATCAATTCA CTTAAAAGNA TGGCCCANAT AGCACNNATA GGACCAAGGG ACACATGTAG 60
TCANTTTTTA AAAACATGTA CTTGGTCTTT TGTGTGTGTC TGTTATATTC CATTAGAN 118

SEQ ID NO:1344
SEQUENCE LENGTH:117
SEQUENCE TYPE:nucleic acid
TOPOLOGY:linear
CLONE:HUMGS01495
SEQUENCE DESCRIPTION:
GATCCCAGGA GAGCTGGGCT ATGACTGCAA TAAGGAGTTG TTCCTTCACC TGAGATGTGC 60
TTCTTTTGGT TCATTTCTGG CTTGACAACA AGAAATAAAC GTGGTATGTT CCTGAAA 117

SEQ ID NO:1345
SEQUENCE LENGTH:119
SEQUENCE TYPE:nucleic acid
TOPOLOGY:linear
CLONE:HUMGS01496
SEQUENCE DESCRIPTION:
GATCTTGAGG GACCCAACAT TTGTAGGGGC ACTAATCCAG CCCTTAAATC CCCCAGCTTC 60
CAAACTTGAG GCCCACCATC TCCACCATCT GGTAATAAAC TCATGTTTTC TCTGCTAAA 119

SEQ ID NO:1346
SEQUENCE LENGTH:116
SEQUENCE TYPE:nucleic acid
TOPOLOGY:linear
CLONE:HUMGS01497
SEQUENCE DESCRIPTION:
GATCAGATTT GGGTGGGAGA AAGAAGTGGG TATCAAGGGT GATTTGAATT TTCTGCAGCA 60

TTAAAGTGGC GTTAATAAGA TAAGTAATAA TAAAGAATTC TAACATCCAT GTCAAA 116

SEQ ID NO:1347
SEQUENCE LENGTH:116
SEQUENCE TYPE:nucleic acid
TOPOLOGY:linear
CLONE:HUMGS01498
SEQUENCE DESCRIPTION:
GATCCGGTGG AAATCCAAGC TCTGGGCTGG TAATTTTTAT GAGCATTTTC AGCTTTTGCA 60
AATACAAAAT ATAATNCTTT ACAAAAATAA ATTTTTATNC TAATCTAAAT CTGAAA 116

SEQ ID NO:1348
SEQUENCE LENGTH:115
SEQUENCE TYPE:nucleic acid
TOPOLOGY:linear
CLONE:HUMGS01499
SEQUENCE DESCRIPTION:
GATCTTCTCT ATGATTGATA CATGGCACAG TGAGAGATTA ATGGGCATTG TGTACAAATT 60
GCTTCTCACC ATCCCCATTA GACCTACGAA TAAAGCATCC GGTTCTAAAA TTAAA 115

SEQ ID NO:1349
SEQUENCE LENGTH:118
SEQUENCE TYPE:nucleic acid
TOPOLOGY:linear
CLONE:HUMGS01500
SEQUENCE DESCRIPTION:
GATCTTTTTG TAAAAGGACC AAATGTTCTT TTATAAATGT AATAAGGAAT ATCTTGCTCT 60
TTAAAATTTA TTAGGNNTTT AATGAGTAAT TTTNATTAAA AGATTTCTTT TTTTGAAA 118

SEQ ID NO:1350
SEQUENCE LENGTH:192
SEQUENCE TYPE:nucleic acid
TOPOLOGY:linear
CLONE:HUMGS01501
SEQUENCE DESCRIPTION:
GATCCTATTT GTTTCATTTT ATTGTAAATN CCCATTTGCA TCAAAACCTA ATNATAGTGA 60
TNGGTAAGTA AAAACAAATG GTGTATTGCT TTTCATACAA GTGTTTTCAC AAAAGCCATT 120
TGCCTAGGCA GCAAAAAATA TTAATTTGTT AAAAAAAATT TTCCTTCGTG TCCATCCNCA 180
NAAANTNGNG NN 192

SEQ ID NO:1351
SEQUENCE LENGTH:115
SEQUENCE TYPE:nucleic acid
TOPOLOGY:linear
CLONE:HUMGS01502

SEQUENCE DESCRIPTION:
GATCTGTGGA CTTTCATCAN ATTATGAGAC TNNCTCAATT TCATGACTGT ACTACCTGAA 60
ACAAAGTGAG AAAGGACAGG TGTATTTTNN TAAGTCATCA AGATAAATCC TTAAN 115

SEQ ID NO:1352
SEQUENCE LENGTH:150
SEQUENCE TYPE:nucleic acid
TOPOLOGY:linear
CLONE:HUMGS01503
SEQUENCE DESCRIPTION:
GATCTTAGTT TGTAGCTTAT GACTTATTTA ATGAATGGAT GCCCAGCCAA GCTCAGAGTA 60
GGCGCCCAAA GCATTGTGGA TTATTTTCCT GTTTTGTCTT TTTTTTTTTT TTTTTTNAAG 120
CCATGNCANC CCNGANGGGG CCAGNGANTN 150

SEQ ID NO:1353
SEQUENCE LENGTH:113
SEQUENCE TYPE:nucleic acid
TOPOLOGY:linear
CLONE:HUMGS01505
SEQUENCE DESCRIPTION:
GATCGNNAAG AGGTTACCCA GACCACACAC AGTTTGAGAA AACATNCCCA TTATNACCCA 60
TCTAGCAAAG AGGCACCCTA AGTGGTCCAT GAAGAGTTTA ATTTTATNTT AAN 113

SEQ ID NO:1354
SEQUENCE LENGTH:113
SEQUENCE TYPE:nucleic acid
TOPOLOGY:linear
CLONE:HUMGS01506
SEQUENCE DESCRIPTION:
GATCGGGTTT AGACGNNAGN TCATTCAGTG AAGCAAGCCA AAAGCNCACA TTTGTATGCC 60
TTAGGTCTTC TTAAAATGGT ATCTGTAAAC ATGTGTCCAA TATAAAANCT ATN 113

SEQ ID NO:1355
SEQUENCE LENGTH:411
SEQUENCE TYPE:nucleic acid
TOPOLOGY:linear
CLONE:HUMGS01507
SEQUENCE DESCRIPTION:
GATCCTCCCT GCCCGCGAAG TGNACAGTTT ACAAAATTAT TTTCTGCAAA AAAGAAAAAA 60
AAGTTAGGTT AAAAACCAAA AAACTACATA TTTTATTATA GAAAAAGTAT TTTTTCTCCA 120
CCAGACAAAT GGAAAAAAAG AGGAAAGATT AACTATTTGC ACCGAAATGT CTTGTTTTGT 180
TGCGACATAG GAAAATAACC AAGCACAAAG TTATATTCCA TCCTTTTTAC TGATTTTTTT 240
TTCTTCTATC TGTTCCATCT GCTGTATTCA TTTNTCCAAT CTCATGTCCA TTTTGGTGTG 300
GGAGTCGGGG TAGGGGGTAC TCTTGTCAAA AGGCACATTG GTGCATGTGT GTTTGCTAGC 360
TCACTTGTCC ATGANAATAT TTTATGATAT TAAGGNAAAT CTTTTGAAAA A 411

SEQ ID NO:1356
SEQUENCE LENGTH:112
SEQUENCE TYPE:nucleic acid
TOPOLOGY:linear
CLONE:HUMGS01508
SEQUENCE DESCRIPTION:
GATCATGGTG TAATAAGACA TAACGTTTTT CCTTTAAAAA AATTTAAGTG CGTGTGTAGA 60
GTTAAGAAGC TGTTGTACAT TTATGATTTA ATAAAATAAT TCTAAAGGNA AA 112

SEQ ID NO:1357
SEQUENCE LENGTH:112
SEQUENCE TYPE:nucleic acid
TOPOLOGY:linear
CLONE:HUMGS01509
SEQUENCE DESCRIPTION:
GATCAAGTGC ATTTGACAGA AAAGTTCAGT TTCTTGGGAA GAAACACCTT TTAAGCTGAA 60
TGGAGAAAAT NCCAAAATAA ATTATATCAC CACAATGGTG TATACTCAGA AA 112

SEQ ID NO:1358
SEQUENCE LENGTH:112
SEQUENCE TYPE:nucleic acid
TOPOLOGY:linear
CLONE:HUMGS01510
SEQUENCE DESCRIPTION:
GATCTGGGAA AAAAACAAGA GACTCAATGG AGACAGAAGA AAATCCCAAG GTTCTAATAA 60
CTNCNTTCTN AAAAANTATC TACCCCATTT GGTGAAGTGA AAANCAGAAA AN 112

SEQ ID NO:1359
SEQUENCE LENGTH:112
SEQUENCE TYPE:nucleic acid
TOPOLOGY:linear
CLONE:HUMGS01511
SEQUENCE DESCRIPTION:
GATCCTGGTT GGAGTAAACA TTCCATGGGA ACTCGGGCTG TNAGAATNTC CTAACCACCT 60
GANTGCAGAA ACATCCTTAT CACATCCTNC TGGGAAAGNC CAACAGCCTG AN 112

SEQ ID NO:1360
SEQUENCE LENGTH:112
SEQUENCE TYPE:nucleic acid
TOPOLOGY:linear
CLONE:HUMGS01512
SEQUENCE DESCRIPTION:
GATCCATCAC CTGNGCAGCA TAACTGGCTT CNNCTCAGTC ATCCACACAA CACCAGGACT 60
TAAGACAAAT GGGACTCCCT GTCATCTNGA GCTATNCATT TATTTAAACT GN 112

SEQ ID NO:1361
SEQUENCE LENGTH:111
SEQUENCE TYPE:nucleic acid
TOPOLOGY:linear
CLONE:HUMGS01513
SEQUENCE DESCRIPTION:
GATCCCTTCA TTGATGTTTG GAAATTCCAT ATTACATTTT GCACGATATG GTACACATAG 60
TACTTTGGTG TACCTGCTTT TGAAAAATAA AGCTTTGGTC ACCCGGTGAA A 111

SEQ ID NO:1362
SEQUENCE LENGTH:111
SEQUENCE TYPE:nucleic acid
TOPOLOGY:linear
CLONE:HUMGS01514
SEQUENCE DESCRIPTION:
GATCAGAGAA TGCAGCAGCA GTTTTTTTCC TNGTTTNCTT ACCACTTTAT TCTTTCANAG 60
TTTAAAGAAA ATGGACTCAT GCACAGAACA CTATGCATTT NAAAACTNGT N 111

SEQ ID NO:1363
SEQUENCE LENGTH:111
SEQUENCE TYPE:nucleic acid
TOPOLOGY:linear
CLONE:HUMGS01515
SEQUENCE DESCRIPTION:
GATCGGGGGC GTANATNCAT AGTAGTTTTT ACAGCTGTGT TATTCTTTGC GTGTAGCTAT 60
GGAAGTTGCA TAATTATTAT TATNATTATN ATAACANGTG TGTCTTACGT N 111

SEQ ID NO:1364
SEQUENCE LENGTH:110
SEQUENCE TYPE:nucleic acid
TOPOLOGY:linear
CLONE:HUMGS01516
SEQUENCE DESCRIPTION:
GATCTCAAAA CAGTGCTAAA ATCAAAGNTG TNGACTGTAA AGAAAAACAT GTATATATAT 60
TGCACCTNAA AGTTGTCAGA AGNTAGAAAC TNAAATAAAC TAACTTTAAA 110

SEQ ID NO:1365
SEQUENCE LENGTH:109
SEQUENCE TYPE:nucleic acid
TOPOLOGY:linear
CLONE:HUMGS01517
SEQUENCE DESCRIPTION:
GATCCCACGC CACAGCCTTT TGNTNTGCAA CTGCCTTCTT CGGAAAGAAG AAGTGGGAGG 60
ATGTGAATTT TAGTTCTGAG TTTACCAAAT AAAGAGATAT AAGACGAAA 109

SEQ ID NO:1366
SEQUENCE LENGTH:109
SEQUENCE TYPE:nucleic acid
TOPOLOGY:linear
CLONE:HUMGS01518
SEQUENCE DESCRIPTION:
GATCATGTAC TGAAGTAGTT TAAGCAGGCT GGCTAACTTA GACTNATTGA TTCTGCNTTT 60
GTACTNNAAT AGGGGTTATA ATTGTAAGAT AAAAATGTGT GTGTNCAAN 109

SEQ ID NO:1367
SEQUENCE LENGTH:236
SEQUENCE TYPE:nucleic acid
TOPOLOGY:linear
CLONE:HUMGS01519
SEQUENCE DESCRIPTION:
GATCTGGGGG TTTCTTCATA TTCCTGCTGT TGGAAGCAGT TGACCAGAAA TGCTTGNCAG 60
NACTGCCAAA GCACTGCTGT GAAATGTGAA GTACTTTGTT TTTTTATTTT TAATNATTTT 120
CTTTTTGTTA TTAATATTTT TCTCTGTTCC TTTGTTATTA CTTGCATGGT TTGGCGTCAG 180
AAGTCCTTAC CTCTTTATAT TGTTTGCAGG TTTAAATAAA ACAGTGTGGT GCCAAA 236

SEQ ID NO:1368
SEQUENCE LENGTH:108
SEQUENCE TYPE:nucleic acid
TOPOLOGY:linear
CLONE:HUMGS01520
SEQUENCE DESCRIPTION:
GATCTTGAAG TTATTTTTAA GACATACATT TGGTAAGTAA GTTGGTCCCA GGAATGTATG 60
TNNTTNAAAC CCTTTCTAAA TATGCAGNCA TTAATAAATA ANATTGTN 108

SEQ ID NO:1369
SEQUENCE LENGTH:107
SEQUENCE TYPE:nucleic acid
TOPOLOGY:linear
CLONE:HUMGS01521
SEQUENCE DESCRIPTION:
GATCCAACCC AAATCAAATT GTTAAATGCC CTCTTGAATT TTTTTGTCTG TTATTTAATT 60
ATATGGTGGA ATTAATAATA AAATAAACTT CATGTCTCTG ATTCAAA 107

SEQ ID NO:1370
SEQUENCE LENGTH:107
SEQUENCE TYPE:nucleic acid
TOPOLOGY:linear
CLONE:HUMGS01522
SEQUENCE DESCRIPTION:

GATCTNGAGC TCTGTCTTCA GCAGATTTCA GGTGTAACAT TTGTTAACTC GTACTNGAAG 60
GTGTGTCCTC AAGAAGAAAG TNTTCAAATT AAAAAAGCTG CTGCAAA 107

SEQ ID NO:1371
SEQUENCE LENGTH:107
SEQUENCE TYPE:nucleic acid
TOPOLOGY:linear
CLONE:HUMGS01523
SEQUENCE DESCRIPTION:
GATCATGTAC ATTGTAACGT GTGTCGTCAG TACTGCAGTT CCTCAACTTT NTTTGTCTNT 60
NATTACCATG ACATTTTTAA AGATACAGCT ATTTTNTCGA TGTNAAA 107

SEQ ID NO:1372
SEQUENCE LENGTH:286
SEQUENCE TYPE:nucleic acid
TOPOLOGY:linear
CLONE:HUMGS01524
SEQUENCE DESCRIPTION:
GATCACATCT AAAGCTTTAT CTTTGTGTAA TCTAAGTATA TGTGAGAAAT CAGAATTGGC 60
ATAATTTGTC TTAGTTGATA TTCAAGGCTT TAAAAGTCAT TATTCCTGGG CTTGGTAAGT 120
GAATTTATGA GATTTACTGC TCTAGAAAGT ATAGATGGCG AAAGGACCGT TTTGTATTGC 180
TTCCTGATTA CCAGTCTGAT TATACCATGT GTGCTAATAT ACTTTTTTTG TTATAGATTG 240
TCTTAATGGT AGGTCAAGTA ATAAAAAAGA GATGAAATAA TTTAAA 286

SEQ ID NO:1373
SEQUENCE LENGTH:107
SEQUENCE TYPE:nucleic acid
TOPOLOGY:linear
CLONE:HUMGS01525
SEQUENCE DESCRIPTION:
GATCCGCGCA CCTAAGCCTC CCAAAGTNCT GGAATTACAG GCATGANCTA CCATNCCTGG 60
CCTTCTAATG TTTTTCNTAA TTAAGGCTCT NAACTTCCAA GACTGTN 107

SEQ ID NO:1374
SEQUENCE LENGTH:106
SEQUENCE TYPE:nucleic acid
TOPOLOGY:linear
CLONE:HUMGS01526
SEQUENCE DESCRIPTION:
GATCTAGAGG NGAGAAAAAG ATGANTTGCT CCTTACATTC GATAATCAGT GACCACGAAA 60
CACTCAGACC AGAGCCTGGC TTATCAAAAA CCTTNAGTGA GNNCTN 106

SEQ ID NO:1375
SEQUENCE LENGTH:106
SEQUENCE TYPE:nucleic acid

TOPOLOGY:linear
CLONE:HUMGS01527
SEQUENCE DESCRIPTION:
GATCCACGTG CCATTGTGGA GGCAGAGAAA AGAGAAAGGN TTTATATACG GTACTTATTT 60
AATATCCCTT TTTAATTAGA AATTAAAACA GTTAATNCNC TTCAAA 106

SEQ ID NO:1376
SEQUENCE LENGTH:117
SEQUENCE TYPE:nucleic acid
TOPOLOGY:linear
CLONE:HUMGS01528
SEQUENCE DESCRIPTION:
GATCCTGAGC TCCCTTTGCA GTCTGAAAAA GGTATTGCAG TCAGAACTGT GTACTGATGA 60
TAAANGCCTC TGGTAGCAAT AAAAAGTTGT CCCTAACAAA AGAGGCAAAA AAATAAA 117

SEQ ID NO:1377
SEQUENCE LENGTH:105
SEQUENCE TYPE:nucleic acid
TOPOLOGY:linear
CLONE:HUMGS01529
SEQUENCE DESCRIPTION:
GATCCAGCAA ACAGGTTCTN TTTAAGAAAA ATAATTTATA CTAAATTNAG TAAAATGGAC 60
TTCTTATTCA AAGCATCAAT AATTAAAAGA NTTATTTNAA TGAAA 105

SEQ ID NO:1378
SEQUENCE LENGTH:105
SEQUENCE TYPE:nucleic acid
TOPOLOGY:linear
CLONE:HUMGS01530
SEQUENCE DESCRIPTION:
GATCAGCAAA ATGAGATAAA TGTTTCTNTT TTCCTTTCTG ACTGCATTAA ATCAGATACA 60
ACTCAGCATT AAAAAGCTAT CTNTGTAAAT NTNGTNACTA ATAAN 105

SEQ ID NO:1379
SEQUENCE LENGTH:105
SEQUENCE TYPE:nucleic acid
TOPOLOGY:linear
CLONE:HUMGS01531
SEQUENCE DESCRIPTION:
GATCTAACAC GAGGAACATA TCATGGAAAG TGCATNGTAT NTATTTTAGG GTTATGAGTT 60
CTTTCAAGGG CTAAGNTGCA GAGNATTTCC TCCANGAATC GTGTN 105

SEQ ID NO:1380
SEQUENCE LENGTH:105
SEQUENCE TYPE:nucleic acid

TOPOLOGY:linear
CLONE:HUMGS01532
SEQUENCE DESCRIPTION:
GATCAGGTTA CATGAGGGNT CACTCTTCTT GTTGTACANN CTGTGAGTTC GGGCAAATNT 60
GTAATGGCAT ATCTCCACTA TTACAGTNTC ACACAGAATT ATTTN 105

SEQ ID NO:1381
SEQUENCE LENGTH:332
SEQUENCE TYPE:nucleic acid
TOPOLOGY:linear
CLONE:HUMGS01533
SEQUENCE DESCRIPTION:
GATCGGTGCG TTCTCCTGAT GTTTTNCCNT GGCTTGGGGA TTGTACACGG GACCAGCTCA 60
CGTAATGCAT TGCCTGTAAC AATGTAATAA AAAGCCTCTT TCTTTTTGGG GTGGNNNTTG 120
TCCTTCTGTC AGCTAAAATG GGAGCTCATG AGAGAAGGAC GTCAGGGAAA CGGGGTTGAG 180
GGTGGTCTCG GTGCAGAGAG AAGGGTGTCA GGGAAACGGG GGGTGAGGGT GGTCTTGGTG 240
CCAGACGTAG GGAATGGTGT TGGGAGTGGC CCGAGTGCCT GGCACAGTTG TCTNGTTCAT 300
TCATGTAACA TGATAATTTT TAAATCATTA AA 332

SEQ ID NO:1382
SEQUENCE LENGTH:157
SEQUENCE TYPE:nucleic acid
TOPOLOGY:linear
CLONE:HUMGS01534
SEQUENCE DESCRIPTION:
GATCCTGCTG CTGTAATGGG AACCCCTCCC CCATTTACTT CTCCACCTCC CGTCCTCCCC 60
ATCATTGGTT TTTTTTTGTG TGTCAACTGT GCCGTTTTTA TTTTATTCCT TTTATTTTCC 120
CCCTTTTCAC AGAGAAATAA AGGTCTAGAA GTAGAAA 157

SEQ ID NO:1383
SEQUENCE LENGTH:103
SEQUENCE TYPE:nucleic acid
TOPOLOGY:linear
CLONE:HUMGS01535
SEQUENCE DESCRIPTION:
GATCTACTGT TTGTTGAAGT GTGGGAAAAT AGCCTCTCTA AGGCAGCCCA GATGGGACCA 60
AAATCAGTAC AAACATATTT AAGTAAATTT TAAAATGCGT AAA 103

SEQ ID NO:1384
SEQUENCE LENGTH:284
SEQUENCE TYPE:nucleic acid
TOPOLOGY:linear
CLONE:HUMGS01537
SEQUENCE DESCRIPTION:
GATCCCCTTG TCCCTGGAGT AGGGACTAAC TATAGCACAA AGTAATATGT GCCAATGCTA 60

TTTGTGAAAT GTTTGGGCTT TCTAAACGAC TAAAGGATTT GTNGGGTTTT TNCTTAAGTT 120
TTGAACCAAA TCCTAGAGCC AGCTGATAAT ATTTAATAAT CTAGAGGAGA GAATAATGAT 180
GTACCANTAA GTGGAGATTC CTCCTTATGA TGTATGCTAG GTTATGGAAG ATGTAAAATA 240
TTCAACTTTT TCCTNCGTTT TTTGGACTTT GTATTTTACT GNAN 284

SEQ ID NO:1385
SEQUENCE LENGTH:103
SEQUENCE TYPE:nucleic acid
TOPOLOGY:linear
CLONE:HUMGS01538
SEQUENCE DESCRIPTION:
GATCTGGAAC TCCTAAGCCA TCTACTCCTA CACCAACCNN TTCATCGAAC NCACACCCTN 60
CTNATGCTCA GAGCTCAACT CCTAGTACCC CTTCAGCCAC CCN 103

SEQ ID NO:1386
SEQUENCE LENGTH:104
SEQUENCE TYPE:nucleic acid
TOPOLOGY:linear
CLONE:HUMGS01539
SEQUENCE DESCRIPTION:
GATCGCCGTT CTGTTTNGCA TNNTCCCACC GGGAGTTGCC NGGCAGGAGC ATGGGGTGCT 60
TGGTTGTTTC CTTCCTAATA AAATAAACGC GGGTCGCCAT GAAA 104

SEQ ID NO:1387
SEQUENCE LENGTH:102
SEQUENCE TYPE:nucleic acid
TOPOLOGY:linear
CLONE:HUMGS01540
SEQUENCE DESCRIPTION:
GATCTTAGCC TTAGGGTAAG TAAAATGGGT CTTTTAATAT AANAGTGTGA AAACTATTTC 60
NATCTAATAG TACTCTTTTN AATAAAAGTC AGTAGTNGGA AA 102

SEQ ID NO:1388
SEQUENCE LENGTH:378
SEQUENCE TYPE:nucleic acid
TOPOLOGY:linear
CLONE:HUMGS01541
SEQUENCE DESCRIPTION:
GATCCTNGGT TTTTGTGGTT TGACTTCTAT GGNGTTTTAA AAAAACACAG ATTTTTAGTG 60
TTAATATTGT GTAAATGTAC TCACCTTAGG GATTCATTTG AATGATGGTN TTATACCATG 120
ATTGTNTACA GTTTGTGAAA TTGTTGCAAG GGCAAAGATA ACTCTTAAAA AACCGTCGAG 180
ATTACAATGC TCTAGAATCA GCATATAAGA AAATAAATGA TATCTNCATG TNGANTNNGG 240
GTGGATGGGG GGAGCACGCA TAATTTTTAA GTGTGAAGCT TTGCATCANA GAAATTATTA 300
AAANGCTTTT TTNCTCCNGT ATNTTCCTGT ATTAATCCTT AATGTTTATG GCAAATAAAA 360
TGTAAGGGGN ACATGAAA 378

SEQ ID NO:1389
SEQUENCE LENGTH:102
SEQUENCE TYPE:nucleic acid
TOPOLOGY:linear
CLONE:HUMGS01542
SEQUENCE DESCRIPTION:
GATCGTAACC GTAACCGTAA CCGNNAAGCA CAAACGGGGG GAGCGGGGCA GTGAGCGGGG 60
CAGGNATGAG NCCCGAGGTG GGGTCGGTGG CCAGNACAAC GN 102

SEQ ID NO:1390
SEQUENCE LENGTH:102
SEQUENCE TYPE:nucleic acid
TOPOLOGY:linear
CLONE:HUMGS01543
SEQUENCE DESCRIPTION:
GATCCAGAGG TCCNNGTCAC TTGGAGAAAG NCCAGTCCCT GNGACGGGGC AGCCCTCTNT 60
GTCCCTCGGG CAGCTCGTGT GAATCCTGGG ACCTCTTCCG GN 102

SEQ ID NO:1391
SEQUENCE LENGTH:101
SEQUENCE TYPE:nucleic acid
TOPOLOGY:linear
CLONE:HUMGS01544
SEQUENCE DESCRIPTION:
GATCTCAGCA ATTTGAACAC TAACCTCTCC CCTCCTGGCT CAAGAATTAC TCCGAAGTCA 60
GTCTGCAGAA AATAAATATT TAGTATGACA TGACACTTAA A 101

SEQ ID NO:1392
SEQUENCE LENGTH:101
SEQUENCE TYPE:nucleic acid
TOPOLOGY:linear
CLONE:HUMGS01545
SEQUENCE DESCRIPTION:
GATCTACTCA GGCAACNACC AATCTTCTAN TCTGTCACTA TAGATTAATT TGCATTNTTA 60
AAGAAATNNA CATACATGGA ACCATACATC ATCTATGCTT N 101

SEQ ID NO:1393
SEQUENCE LENGTH:101
SEQUENCE TYPE:nucleic acid
TOPOLOGY:linear
CLONE:HUMGS01546
SEQUENCE DESCRIPTION:
GATCCTAGGA AGTNTGTCCC TGTCCTCCCT GTGCAGGGTA TCCTGTAGGG TGACCTGGAA 60
TTCGAATTCT NTTTCCCTTG TAAAATATTN NTNTGTCTCT N 101

SEQ ID NO:1394
SEQUENCE LENGTH:100
SEQUENCE TYPE:nucleic acid
TOPOLOGY:linear
CLONE:HUMGS01547
SEQUENCE DESCRIPTION:
GATCCACGTG CCATTGTGGA GGAGAGAAAG AGAAGTGTTT ATATACGGTA CTTATTTATA 60
TCCCTTTTAA TTAGAATTAA AACAGTTAAT TTAATTCAAA 100

SEQ ID NO:1395
SEQUENCE LENGTH:100
SEQUENCE TYPE:nucleic acid
TOPOLOGY:linear
CLONE:HUMGS01548
SEQUENCE DESCRIPTION:
GATCTTAGGT TACATAAAGT TTCTAAAGTT TCAAAGAGTC TTGATACAAA ATCAGTTTAT 60
ATTCTGAAAA TATTTATAAT AAAGTATTCT AATTTCTAAA 100

SEQ ID NO:1396
SEQUENCE LENGTH:98
SEQUENCE TYPE:nucleic acid
TOPOLOGY:linear
CLONE:HUMGS01549
SEQUENCE DESCRIPTION:
GATCAATTCT TCAATTTGAT TGAACTGTTC AGCCTTTTCA AGATTTCTTT ATTTACAAAT 60
GATTACATTT AAATGAATGT ACATTCTTCT CACTGAAA 98

SEQ ID NO:1397
SEQUENCE LENGTH:98
SEQUENCE TYPE:nucleic acid
TOPOLOGY:linear
CLONE:HUMGS01550
SEQUENCE DESCRIPTION:
GATCTGTAAC ATTTGTTTCA AAATGCTGTT TCATTTTTAT AAAGTACCAG TGTTTAGCTG 60
CTTTTTATAC ATTAAATTAG CAATTTGAAA AACTCAAA 98

SEQ ID NO:1398
SEQUENCE LENGTH:98
SEQUENCE TYPE:nucleic acid
TOPOLOGY:linear
CLONE:HUMGS01551
SEQUENCE DESCRIPTION:
GATCCTCAGT GTCCTTACCC CCTCCTACCT CTTTTCTGTG CCACCTGCTG TGGGTCCAGC 60
AGGCNTTTAC TTGAGTACAA TAAAAAGTCT GAGTCAAA 98

SEQ ID NO:1399
SEQUENCE LENGTH:98
SEQUENCE TYPE:nucleic acid
TOPOLOGY:linear
CLONE:HUMGS01552
SEQUENCE DESCRIPTION:
GATCCAAGTG TAGTGGGACC CCCTACTAGG GTCAGGAAGT GGACACTAAC ATCTGTGCAG 60
GTGTTGACTT GAAAAATAAA GTGTTGATTG GCTAGAAA 98

SEQ ID NO:1400
SEQUENCE LENGTH:104
SEQUENCE TYPE:nucleic acid
TOPOLOGY:linear
CLONE:HUMGS01553
SEQUENCE DESCRIPTION:
GATCTCCAAA GTGGTGAGTT TATGTGTGAT TTTTATTTTG TTTATGCTCT TCTGTATTTT 60
CCGAATTTCA TACAATAAAT ATCTGTTACT TTTACAATAT GAAA 104

SEQ ID NO:1401
SEQUENCE LENGTH:94
SEQUENCE TYPE:nucleic acid
TOPOLOGY:linear
CLONE:HUMGS01554
SEQUENCE DESCRIPTION:
GATCCAGAGA GTTCAAGGGA TTGGGGAAAG AGAGGCGTCA AGTCATTTGC ACTTTGTACC 60
TGTAAGTTAG GTAATAAACT ATTATACTCG TAAA 94

SEQ ID NO:1402
SEQUENCE LENGTH:92
SEQUENCE TYPE:nucleic acid
TOPOLOGY:linear
CLONE:HUMGS01555
SEQUENCE DESCRIPTION:
GATCTAGTNT CTNGCATTTT TATTATGTTG CTATATACTT TTGTTATCCG TATACTAAAA 60
AAAAAGAATA AATAAATGTT TTGATTGTTA AA 92

SEQ ID NO:1403
SEQUENCE LENGTH:92
SEQUENCE TYPE:nucleic acid
TOPOLOGY:linear
CLONE:HUMGS01556
SEQUENCE DESCRIPTION:
GATCTNTACA TTCTTCAGAC TCATCGTGTG TTTGANACTT TTTATAATGA ACATATATCA 60
TTTTNNTTAG AAAAGAATAA AGTTTTTGAA AA 92

SEQ ID NO:1404
SEQUENCE LENGTH:119
SEQUENCE TYPE:nucleic acid
TOPOLOGY:linear
CLONE:HUMGS01557
SEQUENCE DESCRIPTION:
GATCCTCTGG GGAAGCCAGG ACCAGGAGAG AAGCAAGGTC AAGAAATCCC ACAGTTTGAT 60
GTATTAAAGA AATNACTTAT TTCTACTCAA AATAAATGGC ATTGAAGTCT TTCTTTAAA 119

SEQ ID NO:1405
SEQUENCE LENGTH:90
SEQUENCE TYPE:nucleic acid
TOPOLOGY:linear
CLONE:HUMGS01558
SEQUENCE DESCRIPTION:
GATCTTGTGT CTCCTCTCCA TCTCTGCCTT TGTTACCAGT GTGCATGTGT TTGTGTGTTT 60
TTTAATAAAA TATTGACTCG GCCAGTTAAA 90

SEQ ID NO:1406
SEQUENCE LENGTH:90
SEQUENCE TYPE:nucleic acid
TOPOLOGY:linear
CLONE:HUMGS01560
SEQUENCE DESCRIPTION:
GATCTGTTAT GGCATTCACT TCCAGATTAA TTTTCCGTGT TTGAAGTATG TNCATATGTN 60
CTTTACAGAA TAAAACATCT GAATTTTAAA 90

SEQ ID NO:1407
SEQUENCE LENGTH:87
SEQUENCE TYPE:nucleic acid
TOPOLOGY:linear
CLONE:HUMGS01561
SEQUENCE DESCRIPTION:
GATCCATTTT CTTTCAAAAT TTTCTTTATT GTAAAGTATG CAAAATATAT ATTCATACGA 60
TTTATTAAAT CAGAATGTTT ATACAAA 87

SEQ ID NO:1408
SEQUENCE LENGTH:85
SEQUENCE TYPE:nucleic acid
TOPOLOGY:linear
CLONE:HUMGS01562
SEQUENCE DESCRIPTION:
GATCTGGAAT CCTTTTTCTG CATTGACAAG GACCACAGCT AAAGGACAAT AATATGAATG 60
ACTCATTAAA ATCTCAAAAC TTAAA 85

SEQ ID NO:1409
SEQUENCE LENGTH:85
SEQUENCE TYPE:nucleic acid
TOPOLOGY:linear
CLONE:HUMGS01563
SEQUENCE DESCRIPTION:
GATCCAAGTT ATNTTNAGAA GAAAAACCTA ATTGAACAGG TATGGGTTGG GAGCATAATA 60
AATGTGTTTT GAGAATTGTT CTAAA 85

SEQ ID NO:1410
SEQUENCE LENGTH:84
SEQUENCE TYPE:nucleic acid
TOPOLOGY:linear
CLONE:HUMGS01564
SEQUENCE DESCRIPTION:
GATCATTATT TCATGACTGG TGCGTTCCTA AACTCTGAAA TCAGCCTTGC ACAAGTACTT 60
GAGAATAAAT GAGCATTTTT TAAA 84

SEQ ID NO:1411
SEQUENCE LENGTH:84
SEQUENCE TYPE:nucleic acid
TOPOLOGY:linear
CLONE:HUMGS01565
SEQUENCE DESCRIPTION:
GATCTAAGCC CATGAGCACA GGGACTGGCT ATCCCAAGAC CTGGCAGATG TGGCTGCTCA 60
ATAAACACTT GTTGAACCAT CAAA 84

SEQ ID NO:1412
SEQUENCE LENGTH:86
SEQUENCE TYPE:nucleic acid
TOPOLOGY:linear
CLONE:HUMGS01566
SEQUENCE DESCRIPTION:
GATCACCCAA GGGTTTTTTT GTGTCGGACT ATGTAATTGT AAACTATACC TCTGGTTCCC 60
CATTAAAAGT NCCCATTTNA GTTAAA 86

SEQ ID NO:1413
SEQUENCE LENGTH:83
SEQUENCE TYPE:nucleic acid
TOPOLOGY:linear
CLONE:HUMGS01567
SEQUENCE DESCRIPTION:
GATCTGTGTT TGTATCCCTT TATGTAATGT AAAATTTAAG GGTATTTTGA TTCTAAATAT 60
GATAAAATAA TTTCTCACCT AAA 83

SEQ ID NO:1414
SEQUENCE LENGTH:83
SEQUENCE TYPE:nucleic acid
TOPOLOGY:linear
CLONE:HUMGS01568
SEQUENCE DESCRIPTION:
GATCAGCAGT CCACCACAAA AGCTTCTGTT AAAAGACCCT ACACAAATGC ACAAATTCAG 60
ATTAAACAAG GAAAAGACGG AAA 83

SEQ ID NO:1415
SEQUENCE LENGTH:83
SEQUENCE TYPE:nucleic acid
TOPOLOGY:linear
CLONE:HUMGS01569
SEQUENCE DESCRIPTION:
GATCTTGGTG TTCAAAACAG AACTGTATTT TTGCCTTTAA AATTAAATAA TATAACGTGA 60
ATAAATGACC CTATCTTTGT AAA 83

SEQ ID NO:1416
SEQUENCE LENGTH:82
SEQUENCE TYPE:nucleic acid
TOPOLOGY:linear
CLONE:HUMGS01570
SEQUENCE DESCRIPTION:
GATCCTTCTG TAAAGGTTTT TGGAATTATG TCTGCTGAAT AATAAACTTT TTTGAAATAA 60
TAAATCTGGT AGAAAAATGA AA 82

SEQ ID NO:1417
SEQUENCE LENGTH:86
SEQUENCE TYPE:nucleic acid
TOPOLOGY:linear
CLONE:HUMGS01571
SEQUENCE DESCRIPTION:
GATCTGCATA TTTCTGTATA TTTGTCATGA CAGTGCTTGC ATCCTATTTG GTGTACTCAG 60
CAAATAAACT TTTCATTTTA AACAAA 86

SEQ ID NO:1418
SEQUENCE LENGTH:81
SEQUENCE TYPE:nucleic acid
TOPOLOGY:linear
CLONE:HUMGS01572
SEQUENCE DESCRIPTION:
GATCCCAACA TGGTCCTAGC ACTGCACACT CAGTTTTNCT CTAAGAAGCT GCAATAAAGT 60
TTTTTNAAGT CACTTTGTAA A 81

SEQ ID NO:1419
SEQUENCE LENGTH:81
SEQUENCE TYPE:nucleic acid
TOPOLOGY:linear
CLONE:HUMGS01573
SEQUENCE DESCRIPTION:
GATCTTCANT TAATCNCACT TTAAAAATGA CCAAAACATG TCTTTCTTGA ATTAACTTTG 60
AATAAAAGTT TGTATATTAA A 81

SEQ ID NO:1420
SEQUENCE LENGTH:81
SEQUENCE TYPE:nucleic acid
TOPOLOGY:linear
CLONE:HUMGS01574
SEQUENCE DESCRIPTION:
GATCAGAGCT CAGTTCCTGT AGAAAACGAA CTGTAAAAGA CCATGCAAGA GGCAAAATAA 60
AACTTGAAGT GAATGCTTAA A 81

SEQ ID NO:1421
SEQUENCE LENGTH:80
SEQUENCE TYPE:nucleic acid
TOPOLOGY:linear
CLONE:HUMGS01575
SEQUENCE DESCRIPTION:
GATCTTGGGT TTGGTCTACC CACCCAAGAG AAAAGACTGT TAACTGGAAG AAAAAATATA 60
TATATNNAAT TTTATGTAAA 80

SEQ ID NO:1422
SEQUENCE LENGTH:79
SEQUENCE TYPE:nucleic acid
TOPOLOGY:linear
CLONE:HUMGS01576
SEQUENCE DESCRIPTION:
GATCACAATT ACCTTTAGTT GTTTTTTTTG TAATAATTGT AGCCAAGTAA ATCTCCAATA 60
AAGTTATCGT CTGTACAAA 79

SEQ ID NO:1423
SEQUENCE LENGTH:79
SEQUENCE TYPE:nucleic acid
TOPOLOGY:linear
CLONE:HUMGS01577
SEQUENCE DESCRIPTION:
GATCCTTATG AATGACAGGT TACTGTTTTG CCTTATTGCT TAACTTAATG TAGTGAAATA 60
AAGCAGACAA AGCTTGAAA 79

SEQ ID NO:1424
SEQUENCE LENGTH:79
SEQUENCE TYPE:nucleic acid
TOPOLOGY:linear
CLONE:HUMGS01578
SEQUENCE DESCRIPTION:
GATCTTCTGC ATGAGACCTG GAGTTGGGGA AGCAAGGTTA CATTTGTATT NGTTTATCCT 60
ATGAATACTN TTCTTCAAA 79

SEQ ID NO:1425
SEQUENCE LENGTH:78
SEQUENCE TYPE:nucleic acid
TOPOLOGY:linear
CLONE:HUMGS01579
SEQUENCE DESCRIPTION:
GATCAGGCAG TCTGCTCAGA TACATTGAGT GGCGATTTTA AGTTTTGTTT TGAAAAAATA 60
AACAGATTAA CCTGCAAA 78

SEQ ID NO:1426
SEQUENCE LENGTH:78
SEQUENCE TYPE:nucleic acid
TOPOLOGY:linear
CLONE:HUMGS01580
SEQUENCE DESCRIPTION:
GATCTAAATT TCTAATGTGT TCTATGGGTT TCAATTCTGA AAAAAGAAAA TGAATAAAGA 60
TTTTAATAAA TATTGAAA 78

SEQ ID NO:1427
SEQUENCE LENGTH:78
SEQUENCE TYPE:nucleic acid
TOPOLOGY:linear
CLONE:HUMGS01581
SEQUENCE DESCRIPTION:
GATCTTCATT GTGCTGGGTG CCAGGNCAGT NATCCATTTT AAAATTTGTA ATTCAATAAA 60
GTTTTTTTTG TTNTTAAA 78

SEQ ID NO:1428
SEQUENCE LENGTH:77
SEQUENCE TYPE:nucleic acid
TOPOLOGY:linear
CLONE:HUMGS01582
SEQUENCE DESCRIPTION:
GATCTCCAGG CTTGGCCTCC AGAGCAGCCC ACACCAACNC CAAAATAAAA AAATGTATAT 60
ATTCCTTTAG CTCTAAA 77

SEQ ID NO:1429
SEQUENCE LENGTH:76
SEQUENCE TYPE:nucleic acid
TOPOLOGY:linear
CLONE:HUMGS01583
SEQUENCE DESCRIPTION:
GATCCCAGGG ACCTCAGTCG GCTTAATCAG AGTGTGGCAT AGAAGCTATT TAATGATTAA 60
AGTCATTTGC AGTAAA 76

SEQ ID NO:1430
SEQUENCE LENGTH:76
SEQUENCE TYPE:nucleic acid
TOPOLOGY:linear
CLONE:HUMGS01584
SEQUENCE DESCRIPTION:
GATCAAACTT TTNATGTTCT TAATAAGCTT GCAATTGANT AAAATAGAAT ATAAAATAAA 60
GGTGAAATAA TATAAA 76

SEQ ID NO:1431
SEQUENCE LENGTH:79
SEQUENCE TYPE:nucleic acid
TOPOLOGY:linear
CLONE:HUMGS01585
SEQUENCE DESCRIPTION:
GATCCCAAGT ACAATGAAAA GTTTGCACTG TATGCTGGAC GGCATTCCTG CTTATCAATA 60
AACCTGTTTG TTTTACAAA 79

SEQ ID NO:1432
SEQUENCE LENGTH:75
SEQUENCE TYPE:nucleic acid
TOPOLOGY:linear
CLONE:HUMGS01586
SEQUENCE DESCRIPTION:
GATCTCCAAC NTTGGAAAAT ATACATGATG TGAAACTGNG GGTNCTATGT TAAAAATAAA 60
TGTATGATAA CTAAA 75

SEQ ID NO:1433
SEQUENCE LENGTH:74
SEQUENCE TYPE:nucleic acid
TOPOLOGY:linear
CLONE:HUMGS01587
SEQUENCE DESCRIPTION:
GATCCAAACA TTTTTTTGTG TGTGTATGGC ATTGATGCAG AATAGAATAA AATTATACTT 60
AAGTTCTTTT TAAA 74

SEQ ID NO:1434
SEQUENCE LENGTH:73
SEQUENCE TYPE:nucleic acid
TOPOLOGY:linear
CLONE:HUMGS01588
SEQUENCE DESCRIPTION:
GATCCCGGCG CGGGAAAGTC ACAGAGCTGC CTGGGCTTGT ACCTGGNCAC ATAAAGCCCC 60
ANTTTAAAGC AAA 73

SEQ ID NO:1435
SEQUENCE LENGTH:73
SEQUENCE TYPE:nucleic acid
TOPOLOGY:linear
CLONE:HUMGS01589
SEQUENCE DESCRIPTION:
GATCCCANAG CNATAAAATA AAATTTTATT CCAAAATAAC AAAACAAATT NATCTACTGT 60
ACACAATCTG AAA 73

SEQ ID NO:1436
SEQUENCE LENGTH:72
SEQUENCE TYPE:nucleic acid
TOPOLOGY:linear
CLONE:HUMGS01590
SEQUENCE DESCRIPTION:
GATCCCAGGC TTCTGCGGAC CGACGATACG TTTAAATGTT GTTCTAGTAA AGTTTTCGAT 60
ATGTTTCTGA AA 72

SEQ ID NO:1437
SEQUENCE LENGTH:71
SEQUENCE TYPE:nucleic acid
TOPOLOGY:linear
CLONE:HUMGS01591
SEQUENCE DESCRIPTION:
GATCCCTTGG NGGNCTAGTT CGTATTTTTG TNTTAAACTA TTTGTTAGAA TAAAGTAATT 60
TTGCTAATAA A 71

SEQ ID NO:1438
SEQUENCE LENGTH:70
SEQUENCE TYPE:nucleic acid
TOPOLOGY:linear
CLONE:HUMGS01592
SEQUENCE DESCRIPTION:
GATCTTAAGT ATAAAAATTT TGTAATTGGG CCTTTACTCT CTCAATAATA AAGTATTTTG 60
TTTATATAAA 70

SEQ ID NO:1439
SEQUENCE LENGTH:68
SEQUENCE TYPE:nucleic acid
TOPOLOGY:linear
CLONE:HUMGS01593
SEQUENCE DESCRIPTION:
GATCTGTATT TTCTAAGTCC CCAGTTCTCC TGGCTCTCCT TTCTGAAATA AAGGATTGAA 60
AACNGAAA 68

SEQ ID NO:1440
SEQUENCE LENGTH:67
SEQUENCE TYPE:nucleic acid
TOPOLOGY:linear
CLONE:HUMGS01594
SEQUENCE DESCRIPTION:
GATCTGTTTT TAAAAATCAT AAACTGTGTG CAGACTCAAT AAAATCATGT ACATTTCTGA 60
AATGAAA 67

SEQ ID NO:1441
SEQUENCE LENGTH:71
SEQUENCE TYPE:nucleic acid
TOPOLOGY:linear
CLONE:HUMGS01595
SEQUENCE DESCRIPTION:
GATCAACAGT TACTGTNACT GAGTCGGCCC ATTCTGTTTA GAAATATATT TNAAATGTTT 60
AGTAATTGAA A 71

SEQ ID NO:1442
SEQUENCE LENGTH:66
SEQUENCE TYPE:nucleic acid
TOPOLOGY:linear
CLONE:HUMGS01596
SEQUENCE DESCRIPTION:
GATCTGCCAT TTCATGCCNT GTGACTACNN ATCATTGGCC CTGCAATAAA ATCATTTATT 60
TTTAAA 66

SEQ ID NO:1443
SEQUENCE LENGTH:64
SEQUENCE TYPE:nucleic acid
TOPOLOGY:linear
CLONE:HUMGS01597
SEQUENCE DESCRIPTION:
GATCCTGCAT GCTACTCTTA GATAGAAGAT GGCAAAACCA TGGTATTAAA ATATGAATGA 60
TAAA 64

SEQ ID NO:1444
SEQUENCE LENGTH:64
SEQUENCE TYPE:nucleic acid
TOPOLOGY:linear
CLONE:HUMGS01598
SEQUENCE DESCRIPTION:
GATCAAGTCA CTNTTGACAA CATCCAGGNG AATATAAAAA CTTAATAAAG CTGTGGAAAG 60
GAAA 64

SEQ ID NO:1445
SEQUENCE LENGTH:62
SEQUENCE TYPE:nucleic acid
TOPOLOGY:linear
CLONE:HUMGS01599
SEQUENCE DESCRIPTION:
GATCAATTGG TTTAACTTCT TTTATGTAAG TATGGTATAT AAATTTCAAG ACGAACACTA 60
AA 62

SEQ ID NO:1446
SEQUENCE LENGTH:62
SEQUENCE TYPE:nucleic acid
TOPOLOGY:linear
CLONE:HUMGS01600
SEQUENCE DESCRIPTION:
GATCACTTTT CACTTTTTGG AATGTTTTGT ATTGAAACTT AATAAAACTT TAACATGGCA 60
AA 62

SEQ ID NO:1447
SEQUENCE LENGTH:62
SEQUENCE TYPE:nucleic acid
TOPOLOGY:linear
CLONE:HUMGS01601
SEQUENCE DESCRIPTION:
GATCGNTCTC AGGCCCTCCC CCCGGAGTAC TTCAGAATGC AATAAATCAA AATAATGGCA 60
AA 62

SEQ ID NO:1448
SEQUENCE LENGTH:62
SEQUENCE TYPE:nucleic acid
TOPOLOGY:linear
CLONE:HUMGS01602
SEQUENCE DESCRIPTION:
GATCTGTATG TGTTCTATTC AGCACAAGGA AATAAAATTT TAGTTGAGGA TTCAGCACTA 60
AA 62

SEQ ID NO:1449
SEQUENCE LENGTH:62
SEQUENCE TYPE:nucleic acid
TOPOLOGY:linear
CLONE:HUMGS01603
SEQUENCE DESCRIPTION:
GATCCCCATT CTTTTCAAAA ACAAAATAAA ACAATAAAGA CTGCAAGGAA GACTGANGGA 60
AA 62

SEQ ID NO:1450
SEQUENCE LENGTH:62
SEQUENCE TYPE:nucleic acid
TOPOLOGY:linear
CLONE:HUMGS01604
SEQUENCE DESCRIPTION:
GATCCAGTCT GGAATAACAT TTTGTAAAAA AAAAATATAT ATATATATAT ATATNGCTGA 60
AA 62

SEQ ID NO:1451
SEQUENCE LENGTH:61
SEQUENCE TYPE:nucleic acid
TOPOLOGY:linear
CLONE:HUMGS01605
SEQUENCE DESCRIPTION:
GATCGGGGGC ACCAGAGGGG CAGAGGCACC AACATCTGAA TAAAGCCATT GTTCTCCCAA 60
A 61

SEQ ID NO:1452
SEQUENCE LENGTH:61
SEQUENCE TYPE:nucleic acid
TOPOLOGY:linear
CLONE:HUMGS01606
SEQUENCE DESCRIPTION:
GATCTTGGAC NTCTCGCATT CAGAACTGTG AGAAATAAAT ATCTATTATT TACAAATTAA 60
A 61

SEQ ID NO:1453
SEQUENCE LENGTH:60
SEQUENCE TYPE:nucleic acid
TOPOLOGY:linear
CLONE:HUMGS01607
SEQUENCE DESCRIPTION:
GATCTCCGTC TTGTATGGCT GAATGTTGGC CTAAAATAAA GATTACTGTT GTAAAATAAA 60

SEQ ID NO:1454
SEQUENCE LENGTH:62
SEQUENCE TYPE:nucleic acid
TOPOLOGY:linear
CLONE:HUMGS01608
SEQUENCE DESCRIPTION:
GATCTGTGGA GAATGTACAC AGTTTAAACA CATCAATAAA TACTTTAACT TCCACCGAGA 60
AA 62

SEQ ID NO:1455
SEQUENCE LENGTH:72
SEQUENCE TYPE:nucleic acid
TOPOLOGY:linear
CLONE:HUMGS01609
SEQUENCE DESCRIPTION:
GATCAAGAAT CCTGCTCCAC TAAGAATGGT GCTAAAGTAA AACTAGTTTA ATAAGCAAAA 60
AAAAAAACCA AA 72

SEQ ID NO:1456
SEQUENCE LENGTH:59
SEQUENCE TYPE:nucleic acid
TOPOLOGY:linear
CLONE:HUMGS01610
SEQUENCE DESCRIPTION:
GATCTAGATG TTTCTTTAAC CAAGATGAAT TAAAATATAG TAGAGTTCCA CTGTNCAAA 59

SEQ ID NO:1457
SEQUENCE LENGTH:63
SEQUENCE TYPE:nucleic acid
TOPOLOGY:linear
CLONE:HUMGS01611
SEQUENCE DESCRIPTION:
GATCAAATTG TACCTTTTTA GAGAAAAGGA CCAAAATAAA AGAAAAATGA ATTATGAACT 60
AAA 63

SEQ ID NO:1458
SEQUENCE LENGTH:59
SEQUENCE TYPE:nucleic acid
TOPOLOGY:linear
CLONE:HUMGS01612
SEQUENCE DESCRIPTION:
GATCAGCAGT CTTGGATGGN AGGNAACAAA GCTAAATAAA TGTTAGTTTG GTGAGCAAA 59

SEQ ID NO:1459
SEQUENCE LENGTH:65

SEQUENCE TYPE:nucleic acid
TOPOLOGY:linear
CLONE:HUMGS01614
SEQUENCE DESCRIPTION:
GATCTCCCTT ATATTCTTAT GATGAGGCTA AATAAAAGTC TAATAAAAAT GTTAAATATG 60
TGAAA 65

SEQ ID NO:1460
SEQUENCE LENGTH:56
SEQUENCE TYPE:nucleic acid
TOPOLOGY:linear
CLONE:HUMGS01615
SEQUENCE DESCRIPTION:
GATCAAAATC AAGTTTTAAG TTTTTTTGAC CAGATAAATT TAATGATTTT GGCAAA 56

SEQ ID NO:1461
SEQUENCE LENGTH:58
SEQUENCE TYPE:nucleic acid
TOPOLOGY:linear
CLONE:HUMGS01616
SEQUENCE DESCRIPTION:
GATCTTAATT ACTTTCAGAA TATTTTCAAA ATAGATATAT TTTNAAAATC CTTACAAA 58

SEQ ID NO:1462
SEQUENCE LENGTH:55
SEQUENCE TYPE:nucleic acid
TOPOLOGY:linear
CLONE:HUMGS01617
SEQUENCE DESCRIPTION:
GATCGTTCTT CTCTCCGTAT TGGGGAGTGA GAGGGAGAGA ACGCGGTCTG AGAAA 55

SEQ ID NO:1463
SEQUENCE LENGTH:55
SEQUENCE TYPE:nucleic acid
TOPOLOGY:linear
CLONE:HUMGS01618
SEQUENCE DESCRIPTION:
GATCCCTGAG ACGGGGTAAG TTATAATAAA CAGAAATGTA TTGGCTCAGA AGAAA 55

SEQ ID NO:1464
SEQUENCE LENGTH:55
SEQUENCE TYPE:nucleic acid
TOPOLOGY:linear
CLONE:HUMGS01619
SEQUENCE DESCRIPTION:

GATCTGTTAT GTATTTCAAC ATAATCATGT TTCATAAAGA TTTAGTCTTC TGAAA 55

SEQ ID NO:1465
SEQUENCE LENGTH:56
SEQUENCE TYPE:nucleic acid
TOPOLOGY:linear
CLONE:HUMGS01620
SEQUENCE DESCRIPTION:
GATCCCACCT TTGCTCCTGA CAACCCTCAT TTCAATAAAG ACCAGTGAAG ACCAAA 56

SEQ ID NO:1466
SEQUENCE LENGTH:54
SEQUENCE TYPE:nucleic acid
TOPOLOGY:linear
CLONE:HUMGS01621
SEQUENCE DESCRIPTION:
GATCAGAATC CTGCTCCACT AAGAACGGTG TAAAGTAAAT TAGTTATAAG CAAA 54

SEQ ID NO:1467
SEQUENCE LENGTH:63
SEQUENCE TYPE:nucleic acid
TOPOLOGY:linear
CLONE:HUMGS01622
SEQUENCE DESCRIPTION:
GATCTTGAGC CTTAACTGGA CATGAGGGGC ATGAAAATAA AGCTGAACTG CAGCCTCCTG 60
AAA 63

SEQ ID NO:1468
SEQUENCE LENGTH:54
SEQUENCE TYPE:nucleic acid
TOPOLOGY:linear
CLONE:HUMGS01623
SEQUENCE DESCRIPTION:
GATCTTTTCT AAATGTTATT ACTTGTAAAT AAAGTCTATT TTTCTCCCGT GAAA 54

SEQ ID NO:1469
SEQUENCE LENGTH:54
SEQUENCE TYPE:nucleic acid
TOPOLOGY:linear
CLONE:HUMGS01624
SEQUENCE DESCRIPTION:
GATCTGTATA TTTTTTTCTA AGAGAAAATG TAAATAAAGG ATTTCTAGAT GAAA 54

SEQ ID NO:1470
SEQUENCE LENGTH:54

SEQUENCE TYPE:nucleic acid
TOPOLOGY:linear
CLONE:HUMGS01625
SEQUENCE DESCRIPTION:
GATCTGCGCA TATATATATA TGTATAAAAA ATAATAAAAT AATGGAAGNT GAAA 54

SEQ ID NO:1471
SEQUENCE LENGTH:54
SEQUENCE TYPE:nucleic acid
TOPOLOGY:linear
CLONE:HUMGS01626
SEQUENCE DESCRIPTION:
GATCCTAAAT NCACGCACCC CGTGGGAGCN CAATAAAGAT TTACTGAATT GAAA 54

SEQ ID NO:1472
SEQUENCE LENGTH:53
SEQUENCE TYPE:nucleic acid
TOPOLOGY:linear
CLONE:HUMGS01627
SEQUENCE DESCRIPTION:
GATCCGCCAT CTGTAATGTC CTTGGCACAA TAAAACCAAA TGTCAGTTTC AAA 53

SEQ ID NO:1473
SEQUENCE LENGTH:52
SEQUENCE TYPE:nucleic acid
TOPOLOGY:linear
CLONE:HUMGS01628
SEQUENCE DESCRIPTION:
GATCCCTAGT GATTACAGCC CTGAAGAAAA TTAAATCTGA ATTAATTTTA AA 52

SEQ ID NO:1474
SEQUENCE LENGTH:52
SEQUENCE TYPE:nucleic acid
TOPOLOGY:linear
CLONE:HUMGS01629
SEQUENCE DESCRIPTION:
GATCCAGTGT GGATTCTTNG AGTAATAACG TTGGTTTTAT TTATCATATA AA 52

SEQ ID NO:1475
SEQUENCE LENGTH:52
SEQUENCE TYPE:nucleic acid
TOPOLOGY:linear
CLONE:HUMGS01630
SEQUENCE DESCRIPTION:
GATCTTCAAT GTTTATTTTA AAATAAAATA AAATAAGTTC TTGACTTTTA AA 52

SEQ ID NO:1476
SEQUENCE LENGTH:56
SEQUENCE TYPE:nucleic acid
TOPOLOGY:linear
CLONE:HUMGS01632
SEQUENCE DESCRIPTION:
GATCTTGAAT GAAAGTCTTC TCAGGCTGTA GGGTGGTTCC TACAACCACA GCCAAA 56

SEQ ID NO:1477
SEQUENCE LENGTH:51
SEQUENCE TYPE:nucleic acid
TOPOLOGY:linear
CLONE:HUMGS01633
SEQUENCE DESCRIPTION:
GATCTCATCT GAATCCCCAA CACCCAATAA AGTTCCTTTT TAACACACAA A 51

SEQ ID NO:1478
SEQUENCE LENGTH:55
SEQUENCE TYPE:nucleic acid
TOPOLOGY:linear
CLONE:HUMGS01642
SEQUENCE DESCRIPTION:
GATCAAAATC CTATTTAGAA AAAATAAAAC TACTTTCTGT TTATCTCTTT AGAAA 55

SEQ ID NO:1479
SEQUENCE LENGTH:34
SEQUENCE TYPE:nucleic acid
TOPOLOGY:linear
CLONE:HUMGS01671
SEQUENCE DESCRIPTION:
GATCCAAAAA CATCCGTGAA CCTCTGTCTG TAAA 34

SEQ ID NO:1480
SEQUENCE LENGTH:305
SEQUENCE TYPE:nucleic acid
TOPOLOGY:linear
CLONE:HUMGS01673
SEQUENCE DESCRIPTION:
GATCAAAATA AAGGATGATA ATAGATATTA TTTTTNCTTA TGACAGAAGC AAATGATGTG 60
ATTTATAGAA AAACTGGGAA ATACAGGTAC CCAAAGAGTA AATCAACATC TGTATACCCC 120
CTTCCCAGGG GTAAGCACTG TTACCAATTT AGCATATGTC CTTGCAGAAT TTTTTTTTCT 180
ATATATACAT ATATATTTTT AACCAAAATG AATCATTACT CTATGTTGTT TTACTATTTG 240
TTTGACATAT CAGTATATCT GAAACACCTT TTCATGTCAA TAAATGTTCT TCTCTAACAT 300
TTAAA 305

SEQ ID NO:1481
SEQUENCE LENGTH:734
SEQUENCE TYPE:nucleic acid
TOPOLOGY:linear
CLONE:HUMGS01686
SEQUENCE DESCRIPTION:
GATCCAGCCC ATTCTCTACT CTTACTCCTT TCATGGGCCA CCAGAGCCAG TACTCTTGGA 60
TAGCAGCAGC ATTCTAGCTG ACAGAATTTT GCTGATGGAT ACTTTCTTTC AAATTGTCAT 120
TTATCTTGGT GAGACCATAG CCCAGTGGCG TAAAGCTGGC TACCAGGACA TGCCCGAGTA 180
TGAAAACTTC AAGCACCTTC TGCAGGCACC ACTGGATGAT GCTCAAGAAA TTCTGCAAGC 240
ACGCTTCCNG ATGCCACGTT ACATCAACAC GGAGCATGGA GGCAGTCAGG CTCGATTCCT 300
TTTGTCCAAA GTGAACCCAT CTCAGACACA CAATAACCTG TATGCTTGGG GACAGGAAAC 360
TGGAGCACCC ATCCTAACTG ATGATGTTAG CCTGCAGGTG TTCATGGACC ATTTGAANGA 420
AGCTGGCTGT CTTCCAGTGG CCTGTTAAAG CTGAGGATAC AACCAGGGAA ATGCAAACGG 480
TNTCAAGATT TGGTGGTTCA AAATTGTCTA GAAAGGGTTT GATAACCATT CCNGGTTACT 540
TTTTCTTNGG ANGTTTTAAC CAAATAATCA ANGGCNATTT TATTATGGAA CCTCTTTAGG 600
TTATAATTNA TTNGGAATNC CGNCNTTGGC CTTTTCTTGN CCTTTAAATT TTAAGGGAAN 660
AAANNTTTGG GCNTNAGGNG GTTNNGGNTT TTTGGNNCNA ACTTTNGGGN TTAATAAANC 720
NGGGGNNTNT TAAA 734

SEQ ID NO:1482
SEQUENCE LENGTH:731
SEQUENCE TYPE:nucleic acid
TOPOLOGY:linear
CLONE:HUMGS01687
SEQUENCE DESCRIPTION:
GATCAACCGG AGCGCTTCCG AGCCATCCTT GCATCGGGCA GCCCACACTG AGGATATCAA 60
TGCTTGCACG CTGACCACGT CCCCGAGGCT GCCTGTNTTC TAGTTGACTT TGCACCTGTN 120
TTCAGGCTGC CAGGGGAGGA GGAGAAGCCA GCAGGCACCA CTTTTCTGCT CCCTTTCTCC 180
AGAGGCAGAA CACATGTTTT CAGAGAAGCT GCTGCTAAGG ACCTTCTAGA CTGCTCACAG 240
GGCCTTAACT TCATGTTGCC TTCTTTTCTA TCCCTTTGGG CCCTGGGAGA AGGAAGCCAT 300
TTGCAGTGCT GGTGTGTCCT GCTCCCTCCC CACATTCCCC ATGCTCAAGG CCCAGCCTTC 360
TGTAGATGCG CAAGTGGGAT GTTGATGGTA GTACAAAAAG CAGGGGCCCA GCCCAGCTGT 420
TGGCTACATG AGTATTTAGN GGGAAGTAAG GTAGCAGGCA GTCCAGCCCT GANNTTGGAG 480
ACACATGGGG TTTTTGGAAA TAAGNTTCTT GNGGGATGAA TGTAACAGGN GGGNTTTCTT 540
CANGGAGTGG TNCAGCNCAG ACATTTNNAC ATAAGGACCA AACAGCCCAG NNTNCCGNAT 600
TTTGNCNCCC AAGNGCCTTT TTTGGACTTG GANCTTTNTT NGGGGAAAGC CNCTTNAANG 660
TTTAAGNGCC AGNATNGGTG TTTCNGNAAG NATTGCCATC CGATTTAGCC TTTANGGGAN 720
GTTCCANAAG N 731

SEQ ID NO:1483
SEQUENCE LENGTH:687
SEQUENCE TYPE:nucleic acid
TOPOLOGY:linear

CLONE:HUMGS01688
SEQUENCE DESCRIPTION:
GATCGGTGGC TCCATCCTGG CCTCACTGTC CACCTTCCAG CAGATGTGGA TTAGCAAGCA 60
GGAGTACGAC GAGTCGGGCC CCTCCATCGT CCACCGCAAA TGCTTCTAAA CGGACTCAGC 120
AGATGCGTAN ATTNTGCTGC ATGGGTTAAT TGAGAATAGA AATTTGCCCC TGGCAAATGC 180
ACACACCTCA TGCTAGCCTC ACGAAACTGG AATAAGCCTT CGAAAAGAAA TTGTCCTTGA 240
AGCTTGTATC TGATATCAGC ACTGGATTGT AGAACTTGTT GCTGATTTTN ACTTTGTATT 300
GAAGTTAACT GTTCCCCTTG GTATTAACGT GTCAGGGCTG AGTGTTCTGG GATTTCTCTA 360
GAGGCTGGCA AGAACCAGTT GTTTTGTCTT GCGGGGTCTT GTCAGGGTTG GAAAAGTCCA 420
AGCCGTAGGA CCCCAGTTTC CTTTCTTAAG CTGATGTCTT TTGGCCAGAA CACCGTGGGC 480
TNGTTAATTG CTTTTAAGTT GGGAAGCGGT TTGCAATTAC GGCNCTAAAA NGTATTCATC 540
CTNAATTNAA GGAAAGGGTT TTTTTGTAC CGAAANNNCG ATTCTTTGAA GNGNTGNCAC 600
CAAATTTTGG GTTTTCANCC GGTAAGGGGN GACATTAGGN CCCAGNACAA GGNATTNTGA 660
AAGGGAANAN AANGGCNNCC GAAAAAN 687

SEQ ID NO:1484
SEQUENCE LENGTH:649
SEQUENCE TYPE:nucleic acid
TOPOLOGY:linear
CLONE:HUMGS01689
SEQUENCE DESCRIPTION:
GATCAAACTG TATTGCCCAG GCCAGCTCCT GAAGAACTGT GAACTATGAA CGTCTCAGCC 60
TAGAAGGATA ATGTGACCTT CAATTTGCAC ACCATCCATT GTCTCTTTCA AACTAAGAGC 120
CTCTCTAAGC TAGATAGGCC AAGGATTATT TTTTTAACTT TTATTTTAGG TTCAGGGGTA 180
CATATACAGG TTTGTTACAT AGGTAACCTC ATGTCATGAG GGATTTTGTA TAGATTATTT 240
GGTGACCCAG GTACTAAACC TAGTACCCAT TAGTTGTTTT TNCTGCTCCT CTCCCTCCTC 300
TCACCCTCCA CCCTCAGTTA GTTCCCAGTG TGTGTTGTTT CCCCACATCT ATCCATGTGT 360
TCTTATTATT TAAGCTCCCA CTTATAAGTA AGAACATGCA GTGTTTGGTT TTCTGTTCCC 420
GATTAGGTAA TGCTGAGGGA TAATGGCTTC CAATTNCCAT CCATNGTTTC TGCAAAGGAN 480
CATGNNCTCA TTNCTTTAAA TATGGGCTNG CATAGNNGTT CCATGGGNGG TGAATGTACC 540
ACATTTTCCT TTNANCCAGG TCTNATCAAT TGGNGGGCCA TTGNGGGTGA TTCCAAGGCT 600
TNGGTAATGG TGAANAGGGC ACAATAANCC AGTACCATGG GGCATGAAA 649

SEQ ID NO:1485
SEQUENCE LENGTH:549
SEQUENCE TYPE:nucleic acid
TOPOLOGY:linear
CLONE:HUMGS01691
SEQUENCE DESCRIPTION:
GATCCTCTGA TGGGAGCTGA AAGGATGAGA GGTGGGCATC TAGATTTAGG GAGGCTGTTC 60
AGGCTTTGCA GGTCCCTTAC CTGAACACAT AGAAACCCTG GAGCTGTGAC TGTGTCCATG 120
TGTGTGTGTT TGTCTGTGTG TGTTGCGGGG GATGGGCACC TGCATGAATG TGGTAGAGAA 180
AATGGCTCTG CTCAGAGGGA AGATACGCAT AGCAAGGCAG GGACCAGAGG AATCACAGGC 240
GCCTGGAGAG CAGCCGGGCA ACGNCTCCAG GGACCTGCCG GCTTCCCTCA GTCCTCCAGG 300
GGCCCAGCAC TCTTCCTTTA GGCCCTGTGA GCGTCCCTTG TCAGGATACA TTCTCTCATT 360

TTGCTGAAGC TGATTTGATT GGGTGTCTGT TTCTCGCAGC CAAAAGAGCT CTGAAATGAG 420
GAAAGTGCTT CTGTGCTAAC TCCCCGGGTC TCCTGAATTT CAGTCATTCA TGTACCCGCC 480
TCGAAATTTT TTGCAATATC TGTGTACCCA ATGTCCATTT AATTAATAAA GAAGTTTTCT 540
TAAATTAAA 549

SEQ ID NO:1486
SEQUENCE LENGTH:543
SEQUENCE TYPE:nucleic acid
TOPOLOGY:linear
CLONE:HUMGS01692
SEQUENCE DESCRIPTION:
GATCGANGTT TACCTTGTGT CAGGAAAAAA TGTGGGGTAT GCCAAGTATG CCGATAGAAT 60
AAGTNCTAAT GATGCCACTN GCCACTCTAC ATNGAAAGAT TCTGAATGGG GTGAGACTTA 120
AAGTTATGCT GGCAGATTCG CCAAGAGAAG AATCTAACAA ACGGCAAAGA ACTTACTGAT 180
TCTTGAGAAC ACCCGACTAA ATAATGACAT ANTCCTCAGC TGACTGACTG AAAATGTGAC 240
TGGACGCATT CCCTGTGGAC AGTTGACAGC TTTTTTTTTT TCCATATACC TGNTAGTCTG 300
TGTCCAGCAT TGTTTTGTCT GGGAAGCAGG GTTTGCTGNC ATGTATTTTN NATTCCATAC 360
ATTANTGCTA AACCGNTTAT AGTAGTTGTN CCTTAGNGCA NTATGTTGTT ACGTGTAGCA 420
GAANTAAAGT TTTCTTTGCT TTANCTAAAT CATGNCTTTA TTTNTGNGAA GGCCAGGACC 480
GGGAAATTTT ATTNGNCAGT CTTTGGNTTT TGCCTAATGN CATTGNCATG NNTTTGGGGT 540
NCN 543

SEQ ID NO:1487
SEQUENCE LENGTH:533
SEQUENCE TYPE:nucleic acid
TOPOLOGY:linear
CLONE:HUMGS01693
SEQUENCE DESCRIPTION:
GATCCAGAGT TGCATACAGA CCAATTTCTG CATCAGTGTT ATCTCGACCA GAGGCTAGTA 60
GGACTGGAGA GGGCTCTACG GTATTTAATG GGGCCCAGAA TGGTGTGTCT CAGCTAATCC 120
AAAGGGAGTT TCAGACCAGT GCAATCAGCA GAGACATTGA TACTGCTGCC AAATTTATTG 180
GTGCAGGTGC TGCAACAGTA GGAGTGGCTG GTTCTGGTGC TGGTATTGGA ACAGTCTTTG 240
GCAGCCTTAT CATTGGTTAT GCCAGAAACC CTTCGCTGAA GCAGCAGCTG TTCTCATATG 300
CTATCCTGGG ATTTGCCTTG TCTGAAGCTA TGGGTCTCTN TTGTTTGATG GTTGCTTTCT 360
TGATTTTNTT TGCCATGTAA CAAATTACTG CTTGACATGT TGGCATNCAT ATTAATTACG 420
GATGNAATCC TGGGNANCTA CCTGTNCCTC CGAAAACCTG TAGGTANTTG GGGGTCANGG 480
GGAATTGNNC GGTAATTCCC AAAGCCATTT NATTAAAGGN GGAAACCTTT AAA 533

SEQ ID NO:1488
SEQUENCE LENGTH:531
SEQUENCE TYPE:nucleic acid
TOPOLOGY:linear
CLONE:HUMGS01694
SEQUENCE DESCRIPTION:
GATCCAAAAG ANCTGCTTTC TGAAGCAAGT TTCCAAGANG CTCTTCAGGA AAGCATTCCT 60

GACATTCAAG CGCACGAGTG GGTGCCGCTG TGGCTACTGC GGTATTCGGT CATTGTGAAA 120
AGTAGAGGAA TTATCAAATC AAAAGGCTAC ATTTTACAAG CTAAAAGAAG GGGTTCTTAA 180
CTGACTTAGG AGCATAACTT ACCTGTAATT TCCTTCAATA TGAGAGAAAA TTGAGATGTG 240
TAAAANTCTA GTTACTGCCT GTAAATGGTG TCATTGAGGC AGATATTCTT TCGTCATATT 300
TGACAGTATG TTGTCTGTCA AGTTTTAAAT ACTTATCTNG CCTCCATATC AATCCATTCT 360
CATGAACCTC TGTATTGCTT TCCTTAAACT ATTGNTTTTC TAATTGANAT TGTCTATANG 420
GANATNCTTG CAATATATTT NNNCNTTANT TTTTATGNCT AATATNAATN CAGGNNAATT 480
TGTTGTTTGG TATTTTTGGG CNNGGGTTCC GGGGAATGNT TNTNCANTTT N 531

SEQ ID NO:1489
SEQUENCE LENGTH:526
SEQUENCE TYPE:nucleic acid
TOPOLOGY:linear
CLONE:HUMGS01695
SEQUENCE DESCRIPTION:
GATCGGCGTG ACCANCCCTT GCCGGAGGTG GCCCATGTCA AGCACCTGTN TGCCAGCCAG 60
AAGGCACTGA NAGGAGAAGG AGAAGGCCTC CTGGAGCAGC CTCTCCATGG ATGAGAAAGT 120
CGAGTTGTAT CGCATTAAGT TCAAGGAGAG CTTTGCTGAG ATGAACAGGG GCTCGAACGA 180
GTGGAAGACG GTTGTGGGCG GTGCCATGTT CTTCATCGGT TTCACCGCGC TCGTTATCAT 240
GTGGCAGAAG CACTATGTGT ACGGCCCCCT CCCGCAAAGC TTTGACAAAG AGTGGGTGGC 300
CAAGCAGACC AAGAGGATGC TGGACATGAA GGTGAACCCC ATCCAGGGCT TAGCCTCCAA 360
GTGGGACTAC GAAAAGAACG AGTGGAAGAA GTGAGAGATG CTTGGCCTNC GGCTTCAACT 420
TGCGGCTGGC TCTTTCACCG CCATGCAAAC TCCATGCCTA TTTACTNGGA AACCTGTTAT 480
NCCAAACAGT TGTNCNCTGC TAATAAATTG NCCAGTTTAC CTGAAA 526

SEQ ID NO:1490
SEQUENCE LENGTH:525
SEQUENCE TYPE:nucleic acid
TOPOLOGY:linear
CLONE:HUMGS01696
SEQUENCE DESCRIPTION:
GATCAGCAGA GGNATCAAAC CAACACCTTC TTTGGCTCCC CTCCCGCAGC CACAGAGGCA 60
ACCCACGTTG TCAGCACCAT CCCTGAGTCA TTACAATAGC ACCCTGCAGC TATGCTGGAA 120
AACTGAGCGT GGGACCCTGC CAGACTGAAG AGCAGGTGAG CAAAATGCTG CTTTCTGCCT 180
TGGTGGCAGG CAGAGAACTG TCTCGTACTA GAATTCAAGG AGAAAAGAAG AAGAAATAAA 240
AGAAGCTGCT CCATTTTTCA TCATCTACCC ATCTATTTGG AAAGCACTGG AATTCAGATG 300
CAAGAGAACA ATGTTTCTTC AGTGGCAAAT GTAGCCCTGC ATCCTCCAGT GTTACCTGGT 360
GTAGATTTTT TTTTCTGTAC CTTTCTAAAC CTCTCTTCCC TCTGTGATGG TTTTGTGTTT 420
AAACAGTCAT CTNCTTTTAA ATAATATCCA CCTNTCCTTT TTGCCATTTC ACTTATTGGN 480
TCCATAANGT GAATTTTAAT TTAAGGTTAT GCCACACATG CATGN 525

SEQ ID NO:1491
SEQUENCE LENGTH:522
SEQUENCE TYPE:nucleic acid
TOPOLOGY:linear

CLONE:HUMGS01697
SEQUENCE DESCRIPTION:
GATCAGCTGT TTGTCATAGG GCAGTTGGAA ACGGCCTCCT AGGGAAAAGT TCATAGGGTC 60
TCTTCAGGTT CTTAGTGTCA CTTACCTAGA TTTACAGCCT CACTTGAATG TGTCACTACT 120
CACAGTCTCT TTAATCTTCA GTTTTATCTT TAATCTCCTC TTTTATCTTG GACTGACATT 180
TAGCGTAGTA AGGTGAAAAG GTCATAGCTG AGATTCCTGG TTCGGGTGTT ACGCACACGT 240
ACTTAAATGA AAGCATGTGG CATGTTCATC GTATAACACA ATATGAATAC AGGGCATGCA 300
TTTTGCAGCA GTGAGTCTCT TCAGAAAACC CTTTTCTACA GTTAGGGTTG AGTTACTTCC 360
TATCAAGCCA GTACGTGCTA ACAGGCTCAA TATTCCTGAA TGAAATATCA GNCTAGTNGN 420
CAAGCTCCTG GTCTTGAGAT GTCTTCTCGT TAAGGAGATG GGCCTTTTGG GGGTAAAGGN 480
TAAAATGGAT GAGGTCTGNC ATGNTTCACT ATTCTAGGAC TN 522

SEQ ID NO:1492
SEQUENCE LENGTH:509
SEQUENCE TYPE:nucleic acid
TOPOLOGY:linear
CLONE:HUMGS01698
SEQUENCE DESCRIPTION:
GATCCCAACA ATATTCCCAA GGCCTCAATG ACAAGTGGTT TGGTTCAGCA GTTCCAACAC 60
ACAGTCCTTC CCTCAGTGAC TCCCTTGGNN CCCTCATCTG CACACTGATT GCCATATTGC 120
CCTCTATTTT CTGTCTTTGG TTTAAACCCC AAGGGCCCAG AGGCTTTCTC CGATGTCTAA 180
CTCTTTGTGC CTTGAGCTCC TTTATGTTTG GGTGGCATGT TCATGAAAAA GCCATACTTC 240
TAGCAATTCT CCCAATGAGC CTTTTGTCTG TGGGAAAAGC AGGAGACGCT TCGATTTTTC 300
TGATTCTGAC CACAACAGGA CATTATTCCC TCTTTCCTCT GCTCTTCACT GCACCAGAAC 360
TTCCCATTAA AATCTTACTC ATGTTACTAT TCACCATATA TAGTATTTCG TCACTGAAGG 420
ACTTTATTCA GAAAAGGAAA AACCTCTTTT TTAATTGGGA TGGGAACTTT CTANCCTGCT 480
TTGGNCNGGG GGCCTCTGGG AAGGNCTTN 509

SEQ ID NO:1493
SEQUENCE LENGTH:497
SEQUENCE TYPE:nucleic acid
TOPOLOGY:linear
CLONE:HUMGS01699
SEQUENCE DESCRIPTION:
GATCCTGTCA AAATAGTCCG ATGCCATGAA CATATAGAAT CCTTACAGTA AATGGAGAAT 60
TACTCTTTTT TAGACAAAGA GAAGGGCCTT TTTATCCAAC CCTAAGATTA CTTCACAAAT 120
ATCCTTTTAT CCTGCCACAC CAGCAGGTTG ATAAAGGAGC CATCAAATTT GTACTCAGTG 180
GAGCAAATAT CATGTGTCCA GGCTTAACTT CTCCTGGAGC TAAGCTTTAC CCTGCTGCAG 240
TAGATACCAT TGTTGCTATC ATGGCAGAAG GAAAACAGCC TGCTCTATGT GTTGGAGTCA 300
TGAGATGTCT GCAGAGNCAT TGNGAAGTCA CCAAAGGATT GGCATTGAAA ATATCCATTA 360
TTTAAATGAT GGGCTGTGGN TATGAGGCAT ATAAATGNGC TCAGAGGGAT GCACTTGGGT 420
NAATATGGNT ATTGTGCTGT ATCTGTGTTG TGNCTGTGTG TGCANCTGAG GTATGCCTGT 480
GGTNTGCTGA TAAATCN 497

SEQ ID NO:1494

SEQUENCE LENGTH:472
SEQUENCE TYPE:nucleic acid
TOPOLOGY:linear
CLONE:HUMGS01700
SEQUENCE DESCRIPTION:
GATCAAAATA TCATGGATTG AACCTCATCA ATTGATAGCA GTGAGTGACT GANGCTTCCA 60
AATCAAGAAA AGCCGGCACC AAGAACTTCC ATTCTAATCT AGAGCTGACC AGTTTGAGCT 120
GATTCTNTCT TTGAAGAGTC CTTCTTGATT GCAGTGCAGT ACTGGCATTT CTGAATGGAT 180
GTANGNGGAG TATTTTAGTC TAAAGGCTTT TCAAATTACT TGAATTTTTT TAAAAATTGA 240
GGAGCTTTAT TTCTATTTAC CCTTCCATTT TTGTATATCA AATTTCCATN GTCATTAAAA 300
ACTGTATCTT GAAACTTTGT GAACTGACTT GCTGTATTTG CACTTTGAGC TCTTGAAATA 360
ANTGTGATTT TNGTGTGATT ATCTGGTTTC CNGTTTTAAC CATTACCTGT CACCNTTNAT 420
CCTTAACCTT GAAAGTCCAG AAACCATNNN ATTATTAAGG TNGTCCAATA AA 472

SEQ ID NO:1495
SEQUENCE LENGTH:496
SEQUENCE TYPE:nucleic acid
TOPOLOGY:linear
CLONE:HUMGS01701
SEQUENCE DESCRIPTION:
GATCTGTGTG GATGGCAAAC TCCCCAGGCC ATTCTGGGAC CTAAGTTTAA GAAGTGCCGT 60
CCTCGAACTT NCTGACTCTA AGCTCCTGAG CGGGAGTNAG ACTTAGCCCT GAGCCTGCAC 120
TTCCTGTTCA GGTGCAGACA CTGAACAGGG TCTCAAACAC CTTCAGCATG TNTNTTGTGT 180
GCTCACGTGC CACACAGTGT CTCATGCACA CAACCCAGTG TACACACCAC CTACATGCAC 240
ACAGCGTCCT TNCACACTGT GTATGTGAAC AGCTTGGGCC CTGCAACACA ACCATCTACA 300
CACATCTACA ACCNNNAGGA CACACACATG GGTCCGTGCC ATGTCACCTC CNTAGGGGAN 360
GGNTTTCTTT CCAAGGTGTN GCCAGGCCAG GCAAGCCTTC CAGGCCATGA ATTCCTTACT 420
TAGGTACCTN GGGGTTTGGG GTGGGNGNCC CAGNCAAAAT TCTNGGGTTN CGTNCNCTTT 480
GGTTNNGNCC AGNTTN 496

SEQ ID NO:1496
SEQUENCE LENGTH:492
SEQUENCE TYPE:nucleic acid
TOPOLOGY:linear
CLONE:HUMGS01702
SEQUENCE DESCRIPTION:
GATCCTGTAC AGCCAGTGTG GGGATGTGAT GAGGGCCCTG GGCCAGAACC CTACCAACGC 60
CGAGGTGCTC AAGGTCCTGG GGAACCCCAA GAGTGATGAG ATGAATGTNA AGGTGCTGGA 120
CTTTGAGCAC TTTCTGCCCA TGCTGCAGAC AGTGGCCAAG AACAAGGACC AGGGCACCTA 180
TGAGGATTAT GTCGAAGGAC TTCGGGTGTT TGACAAGGAA GGAAATGGCA CCGTCATGGG 240
TGCTGAAATC CGGCATGTTC TTGTCACACT GGGTGAGAAG ATGACAGAGG AAGAAGTAGA 300
GATGCTGGTG GCAGGGCATG AGGACAGCAA TGGTTTGTAT CAACTATGAA GAGCTCGTCC 360
GNATGGGTGC TGAATGGCTG ANGGACCTTT CCCAGTNTTC CCCAGATTNC CGTTGCCTTT 420
CCNTNGTGTG AATTTTGGTN ATCTAAGCCT AAAAGTTTTC CCTAAGGCTT TCTTGTCTTC 480
AAGCAACTTT CN 492

SEQ ID NO:1497
SEQUENCE LENGTH:490
SEQUENCE TYPE:nucleic acid
TOPOLOGY:linear
CLONE:HUMGS01703
SEQUENCE DESCRIPTION:
GATCCAAATA CACAGAATCA AGGGAAGGAG TTGCTTCTTC TAAGAGTGAT GCTTAATCTT 60
TTGGGTCATG GATGAATTGA AGATTTGATT AAAGTTACAA TAAAAAGAGN CCCCNTCAAA 120
GCACGTACAN NCTGTATCAC GAACGGTGCC TGGCCTACTT TTTCCTTTTC TACCCACCCC 180
ACCCCAACCC CCCCTGTCTC AGTGAAAACC TGGTTGTTAC TAAAGTGAAA CTTTAATAAG 240
GATATTGCCT AGGGAAGATT AGTTGTTTTC CTTGTCATTC AAGTTCATTC TGGACCTCTT 300
CCTCTGAGCT GTTAATCAGT GTTGCTAAAC AGACAGGGAA AGACAAGGGA GAGAAAAATG 360
CTGATTCATT CTTCAGAACT TTTAACCNTT TTAACCNCTA ATTCTTCTCC TTGAGAAGCT 420
ATTCTTTGAT TGTGAAAGCT TTGTTGTTCA GGGNAATATG GGGTAATAAA AATAGCTAAC 480
CATTTTTAAA 490

SEQ ID NO:1498
SEQUENCE LENGTH:477
SEQUENCE TYPE:nucleic acid
TOPOLOGY:linear
CLONE:HUMGS01704
SEQUENCE DESCRIPTION:
GATCAAACTT CCATATTTGC CTTGGGAATA ATGGACAAAG GGAAATACTC TTAATTCATG 60
AATAAAAACT TTGCAGAAAA TTAGACAGTG TTTAATTTTC GAAAACTTCC CTCTCTAGAC 120
AGTAGATACC ACCTACTGAT GGTTACATAT ACTAGGGAAA TTTTAAAATT AGGAAATGCT 180
GATAGCTCAT ATTATAAATT TCTAAATCCT AGGAAGAAAC GCTTGGAGTG CTTCTGAATA 240
TACAGAAGTT CCATTTAAGG GCAAGTTTCC CCGTAGATGT ATCAAAATAC TACCAACTGT 300
AAATTGAGGT TTAATTCCCA AATGTATTCT ACTTGTTCTA AAACAATCTG TCCNCAAATA 360
TANAACTATA AAGTAATAAA TTGTTATTTT CCGCACCAAT GGGGNATCTC TAATGGTGGA 420
AAATGGTAAT CCTATGGAAA ATTAATTTTT TTTAANTAAA ANGGTNATTT TAATAAA 477

SEQ ID NO:1499
SEQUENCE LENGTH:476
SEQUENCE TYPE:nucleic acid
TOPOLOGY:linear
CLONE:HUMGS01705
SEQUENCE DESCRIPTION:
GATCCCCGAG TCTTCACAAA TNCTCACTGA AGAAAATTCC TGGAACAATT CAGGGTCCTT 60
TCATAACCTC TACTCTGAGG TGTTANTAAA AAACCTTAGT AACTTAAAAA AAATGAGCTG 120
TACACAAATA CTGAACAATA ATGCTNCNTA TGTTAAGTAT GTANGAAAAA TATATACTTT 180
GACATANATA AGAAACGGTG AGTTGATAAT TGGATAGAAT GGTGGATAGA GTGATAGATA 240
TGTAGTAAAG CAAATATAAC AAAATGATAA TTGTACAATC TAAGTGGTTG GACTATAAAT 300
ATGCACTTCC CACAACNTTT TTATATGNTT AAACAGTTTT ATAATACCAT ATTANGGGAA 360
ACTGTTTGTC TCAAGGAAAT AGAGNTTGTG ATATGTTCTA GTNCAATGNA GGTGTAATCA 420

TGGTNAAATT AAAAGCTTTT ACTTCCTGGG CAATTAAAGG TAATCCTGGT AGGAAA 476

SEQ ID NO:1500
SEQUENCE LENGTH:488
SEQUENCE TYPE:nucleic acid
TOPOLOGY:linear
CLONE:HUMGS01706
SEQUENCE DESCRIPTION:
GATCCTGACT GCTGTCATGG CGTGCCCTCT GGAGAAGGCN CTGGATGTGA TGGTGTCCAC 60
CTTCNANAAG TACTCGGGCA AAGAGGGTGA CAAGTTCAAG NTCAACAAGT CAGAACTAAA 120
GGAGCTGCTG ACCCGGGAGC TGCCCAGCTT CTTGGGGAAA AGGACAGATG AAGCTGCTTT 180
CCAGAAGCTG ATGAGCAACT TGGACAGCAA CAGGGACAAC GAGGTGGACT TCCAAGAGTA 240
CTGTGTCTTC CTGTCCTGCA TCGCCATGNG GTNTAACGAA TTCTTTGAAG GCTTCCCAGA 300
TAAGCAGCCC AGGANGGAAA TGAAAACTCC TCTGATGTGG TTTNGGGGGG TCTGCCAGCT 360
GGGGGCCCTT CTGNGTNGNC CANTGGGGNA CTTTTTTTTT TTTTCCCACC NTGGGNTCCT 420
TTNANGANAA NGGGCTTNGA TTGCTTGAGC AAAGTTTCAA ATAAAAGNTT TNTNGGGAAG 480
NTTTTAAA 488

SEQ ID NO:1501
SEQUENCE LENGTH:472
SEQUENCE TYPE:nucleic acid
TOPOLOGY:linear
CLONE:HUMGS01707
SEQUENCE DESCRIPTION:
GATCCAGAAG CTGATGGATG TGGGTCTGAT TGCAATTCGG TGATGACTTG TTCATACCCC 60
CTTCCCTTCG CCCTCATGTG GAAAGAGGAG CTGGGACCGC GGCGAGCAGC ACGGAGCGGA 120
AGGGAGAGCA GGGGAGAGAA GGCCTCATNT CTCTATATTT ATACATAACC CCGGGGAAGA 180
CACAGAGACT CGTACCTGCG CTGTTTGTGC CGCCGCTGCC TCTGGGCCCT CCCAGCACAC 240
GCATGGTCTC TTCACCGCTG CCCTCGAGTT CCATGTCTCT TTCCCCTGCC CCTAGTTGCT 300
GTCTCGGCTG CTCTCCCATA GTTGGTTTTT TTTTTNATTT GGGGCAGTGG GCATGTTAAT 360
GGGGGAGGGG AGGGGGTTCT TCCAGCCTNA GGTCCCAGTT GGTCTNACGT NGTTTAATTC 420
TTGGGTCCCC TTTTTCCAAT TAAAACAAGC CANTNGGGGC GTGGGTTNTA AA 472

SEQ ID NO:1502
SEQUENCE LENGTH:469
SEQUENCE TYPE:nucleic acid
TOPOLOGY:linear
CLONE:HUMGS01708
SEQUENCE DESCRIPTION:
GATCGCAAAT TGCTNGAGCT GTTGTGGAAT AAATACTGGG TGAATACGTT GAGTTCTTCT 60
AGCTTGCTTA CTAATGCAGA CTATACCACT GGTCAGGTCT TTGATTTGTC TGAAAAGTTA 120
GAGCAGTCAG AAGCCCAGCT GGGACGAGGG AGTTTCATGT TGGGTTTAGA AACGCATGAC 180
CGAAAATCAG AAGACAAACT TGCCAAAGCT ACAAGAGACA GCTGTAAAAC TACCATAGAA 240
GCTATCCATG GATTGATGTC TCAGGTTAAT TAAGGATAAA CTGTTTAATC AAATTAACAT 300
CTCTTCCACA GTCTCTGAGA AGGGCTNTAC CTGAAAGACA GTATGNGGGA AAATATTCAA 360

GGTAACACTT TTAAANCCAG TTNCCNAAAA TCTGATTAGG ANGTNTANGG TTCTCTGAAG 420
TGGTCCTAAA TATTANTATC CCTGTAATAA ANGCTCTTTA AANTGGAAA 469

SEQ ID NO:1503
SEQUENCE LENGTH:466
SEQUENCE TYPE:nucleic acid
TOPOLOGY:linear
CLONE:HUMGS01709
SEQUENCE DESCRIPTION:
GATCTGCAGT GGCTGTTTGA CCAGACCACA AAGTTCACAT CTCCTGAGCT TAGTGTCCGT 60
GGCTGTCCAC CTCCCAGCCA TACTTGACTG TCCCCAAACT CTCCCTGCAG CCACATGTTT 120
CCCATGACCT GTGGGCTCTG CAGATGGACC TCTCTCCGCT AGAGATGCCC TTCTCCCAAA 180
TGGCTTCCCT CCTGGAAGGC CCAGCCTGAG TCCTCGTCTC CTTTCCAGTG CTTCTGCCAG 240
AAGCATCCCC ATGATGTTGT GACCGCACAG CACTTTGTGT CTTGCTTTGA GCACTTGCCA 300
CTCTGGCTGG TGCTGCTGCC ACTGATTGTG TACTGTCTTG CTGCCCTTTC TAGACTGTGA 360
GCTCCTCGTG GGCAGGGACC GCCTGTGTTC TCTGTATTTC CCACGGAGCC TAAGNACAGT 420
GCCTTGCACT TGATAAGGTG CTTAATAAAT GTCTGNTCAA CTGAAA 466

SEQ ID NO:1504
SEQUENCE LENGTH:465
SEQUENCE TYPE:nucleic acid
TOPOLOGY:linear
CLONE:HUMGS01710
SEQUENCE DESCRIPTION:
GATCACTGTG TTCAGTGTTG TTGGAATGGA TTCAGACTGG CTAATGGGGG AAAGGGGAAA 60
CCAGAAGGGC AAGGTGCCAA TTACCTACTT AGAACTGCTC AATTAAGTAG GTGGACTATG 120
GAAAGGTTGC CCATCATGAC TTTGTATTTA TATACAATTA ACTCTAAATA AAGCAGGTTA 180
AGTATCTTCC ATGTTAATGT GTTAAGAGAC TGAAAATACC AGCCATCAGA AACTGGCCTT 240
TCTGCCAATA AAGTTGCATG GTAAATATTT CATTACAGAA TTTATGTTAG AGCTTTCATG 300
CCAAGAATGT TTTCTTACAA AATTCTCTTT TTATTGAGGT TTCACTAATA AGCAGCTTCT 360
ACTTTTGAGC CTCAACTTAA AGCAGANCTG TTTTCTACTG GGATTTTTCA TTAACAGCAA 420
GCCTTTTCCT TTTATGTAAA ATTAAATCTA TTGTGGAATT TGAAA 465

SEQ ID NO:1505
SEQUENCE LENGTH:462
SEQUENCE TYPE:nucleic acid
TOPOLOGY:linear
CLONE:HUMGS01711
SEQUENCE DESCRIPTION:
GATCTTGTCT TTAAAACATG ATAGTCCTTT CAGTATAATG TCTTAGATTA AAGACGTTGC 60
CTTTAATATC TGTTGGGAAG GAAATGTCCA GACTTTTCAA ATCTCTTATT ATATGTTTCC 120
TTTTNTTGTT TACATAGGGA ACAATGTTTA TAGTCGTGTG TACAGTGGGG GTCTACANCN 180
NGAAGTGTAT ATTTTCAAAC AATTTTTTAA NGATTTAACA ATTTTTGTAA ATCATTTTCA 240
GGCTTCTGCA GCTGTAGATT CTCACTGTGA ATCCCTTGCT TGCTCATGCA TAAGTGTATT 300
TGCAATACCA NATATACAGG TTTAGTATTT TTGCCTGTTA GTNGATTGTT TCACATGNGT 360

AACGTNTTGG GTGAGATGTT ANATGGTTGG NCGAGGTACT GTGGANGTGA ATGTGGGNAG 420
TAATTTTAAN NCATATGNAA TTGGTCACCA GGGCCNAATT TN 462

SEQ ID NO:1506
SEQUENCE LENGTH:459
SEQUENCE TYPE:nucleic acid
TOPOLOGY:linear
CLONE:HUMGS01713
SEQUENCE DESCRIPTION:
GATCCCAAGC AGGCTCACAA ATTTAAATGA GGGCTTTGTG TGCAGAAAGA GGAATAAGTA 60
CAGATTATTT TCCTACCACT AGATTTTTGG GGAGAGTCAC CATGGAATGT TGACAATTAC 120
TTAAAATATT TTAAGCTCCC TTGCTGAATT CCTGTCCTGT CCCTGAGGAA TCAGATGGTC 180
ATACAGCCAT AGGCACCCAC CCGAAATTTC CCTAGGAGTT GGAGTAATGC TAGAATTGAA 240
GACCTTCTGA GTAAAGGGCT TCTCTGCCTT CTCAGAGGCA GGAGAATTTG CACTGGTTGT 300
GTTAAATGTA TAAAAAGCTA TATGTTCACC AGTTTACTCA TTTCCAATGT GTAGATGAAT 360
AAAATGTAGT GTACAAATTA TTTGAAAATC CCAGAAGGGA AGGTACTTTT CAAANACAGT 420
ATTTTTTTTA ACAAATAAAC TTACGNTTTT TACAGCAAA 459

SEQ ID NO:1507
SEQUENCE LENGTH:454
SEQUENCE TYPE:nucleic acid
TOPOLOGY:linear
CLONE:HUMGS01714
SEQUENCE DESCRIPTION:
GATCTCCTGT CCATCAGCCA GGACAGTCAG CTCTCTCCTT TNAGGGCCAA TCCCCAGCCC 60
TTTTNTNGAG CCAGGCCTCT CTCACCTCTC CTACTCACTT AAAGCCCGCC TGACAGAAAC 120
CACGGCCACA TTTGGTTCTA AGAAACCCTC TGTCATTCGC TCCCACATTC TGATGAGCAA 180
CCGCTTCCCT ATTTATTNAT TTATTTGTTT GTTTGTTTTA TTCATTGGTC TAATTTATTC 240
AAAGGGGGCA AGAAGTAGCA GTGTCTGTAA AAGAGCCTAG TTTTTAATAG CTATGGAATC 300
AATTCAATTT GGACTGGTGT GCTCTCTTTA AATCAAGTCC TTTANTTAAC ACTGAAAATA 360
TATAAGCTCA GATTATTTTA ANTGGGNATA TTTATAANTG NGCAAATATC ATACTGTTCA 420
NTGGGTTCTG GAATAAACTT CACTGGAGGG NAAA 454

SEQ ID NO:1508
SEQUENCE LENGTH:453
SEQUENCE TYPE:nucleic acid
TOPOLOGY:linear
CLONE:HUMGS01715
SEQUENCE DESCRIPTION:
GATCGAGCAG TGTGACTACC CCCCACTCCC CGGGGAGCAC TACTCCGAGA AGTTACGAGA 60
ACTGGTCAGC ATGTGCATCT GCCCTGACCC CCACCAGAGA CCTGACATCG GATACGTGCA 120
CCAGGTGGCC AAGCAGATGC ACATNTGGAT GTCCAGCACC TGAGCGTGGA TGCACCGTGC 180
CTTATCAAAG CCAGCACCAC TTTGCCTTAC TTGAGTCGTC TTCTCTTCGA GTGGCCACCT 240
GGTAGCCTAG AACAGCTAAG ACCACAGGGT TCAGCAGGTT CCCCAAAAGG CTGCCCAGNC 300
TTACAGCAGA TGCTGAAGGA GAGCAGCTGA GGGAGGNGGN GCTGGCCACA TGTCACTGNT 360

GGGTCAGNTT CCAAAGTCCT TTNNTTANCA CTGTTGTGGA CAATNTNANN TNGGGTCAAN 420
TNAAGGGCAG GTNGGTTNAA GNGGNGCCNN GGN 453

SEQ ID NO:1509
SEQUENCE LENGTH:450
SEQUENCE TYPE:nucleic acid
TOPOLOGY:linear
CLONE:HUMGS01718
SEQUENCE DESCRIPTION:
GATCCTGGGG AAGGTGGGGA ACATGCTTGC AGTATCTNTC CCTGTNTGTN TGCTCACATA 60
AGCATTCCGT CCATCTGAGC TCATCGTGCT ACTGGTATGT GTATGTNCAG TTACACAGTT 120
TTNTGTATCA TAGATTCTAG TGTGTTTATA CAAGGNGACA TCTGTGGTTT CCCCAACCGT 180
TCCAAAAGGC TATTTCAAAG GAACCAGCCA ACGTATGAGA AATGANTGTA ACACTGTGGA 240
CATTGACTTC CCGCATAAGG CAGGGTGACC CCCTGAACTC CAGATGTNTG CACAGTATCT 300
NATGTGTTGT TTTCCGTTGT GACGAATGTG NATTGGAACA TTTGGGGGAG CACCCAGAGG 360
GATTTNTNAG TGGGAAGCAT TACACTTTGC TAAATCANGT ATTTNNTTCC TGNTTAAACC 420
AACCTANTTA ANTNTTTANC CCTTGGCAAA 450

SEQ ID NO:1510
SEQUENCE LENGTH:449
SEQUENCE TYPE:nucleic acid
TOPOLOGY:linear
CLONE:HUMGS01719
SEQUENCE DESCRIPTION:
GATCCATGGA TGTAGTGGGG AGTGGAAAAT GGCACAATAT CAAGTGTGAG ANAGGNGATA 60
NACTTCGACT CTNCTGCCTT CAACTGAGAN CAGTTGACCG CAAGCTGAAA CTGGTGTGTG 120
GNAGTCACAG CTTCATCAAG GTCATCAAGG CCAAGAAAAA CAAGGAAGGA CCAATGAATG 180
TTAATTGAAA TATGAAAGCT GAAATGCAAC AAACAACTTC CGCTTAAAAC AATTAAGTTG 240
TTAATAACTG TGATTTTGTA AATTTCAGTA ATTCATTTAA ATGATGTTTC AGTAGATATA 300
TTCTAGCATA TTANGAGCTT TTATAACTGA GTTATAGATT AGTTTGCTTT CTGGAATAAA 360
ATTTCTTCT TATACTCTTC CTTTTTTTA GATATNACAT TTTGCTTTTA TGNCATTCAC 420
GGGGGCAAAA ANTAAAATAT CTTTTTTTN 449

SEQ ID NO:1511
SEQUENCE LENGTH:464
SEQUENCE TYPE:nucleic acid
TOPOLOGY:linear
CLONE:HUMGS01720
SEQUENCE DESCRIPTION:
GATCAGAATC ATGGTCTCCC GCAGTGAAGT GGACATGTTG AAAATNAGGT CTGAATTCAA 60
GAGAAAGTAC GGCAAGTCCC TGTACTATTA TATCCAGCAA GACACTAAGG GCGACTACCA 120
GAAAGCGCTG CTGTACCTGT GTGGTGGAGA TGACTGAAGC CCGACACGGC CTGAGCGTCC 180
AGAAATGGTG CTCACCATGC TTCCAGCTAA CAGGTCTAGA AAACCAGCTT GCGAATAACA 240
GTCCCCGTGG CCATCCCTGT NAGGGTGACG TTAGCATTAC CCCCAACCTC ATTTTAGTTG 300
CCTAAGCATT NCCTGGCCTT CCTGTCTAGT CTCTCCTGTA AGCCAAAGAA ATGANCATTN 360

CAAGGGAGTT TGGAAGTNAA GTCTATGANT GTGAAACAAC TTTTNNCCTC CTGTNGTNCT 420
GTGTTAATAA ANCAGGTGNA TTAAACTGGN TTTNGTACTT TAAA 464

SEQ ID NO:1512
SEQUENCE LENGTH:445
SEQUENCE TYPE:nucleic acid
TOPOLOGY:linear
CLONE:HUMGS01721
SEQUENCE DESCRIPTION:
GATCTTGCCA TGGAAGATAC TAGCCCAGCC TAGCAGAAAA GTGCAATATG TATAGCATAC 60
TTTGACATTT TAAACATGAT AGTCCATAAC CATTTTGAAA TGCTGGGCAA ACTACATGAA 120
GTTATTTATA ATTAATTCAC AGCTAATCAG GCATTTTGAA AGCTTAATTG GATTCAAAAA 180
CCATAATGTT GGAATTTGGT AAAATTTTAA TGTTGATTTT TACTGTGAAA AGGTTTTTAT 240
AAGATATACA CACCCTAGTT TAATGTTGTG TCTTGGTGTG GATTTACAGA TTTACTACAG 300
GTATTCTGAA CCAGGAACAC ANTCAGGTTT TCAGGCCAGT TTGAATACTG GCTGGCCTTA 360
AATTCTAATT ATNNGGAGTA GGGACATCAT ACCTAAATNT TTATGTCAGT GGGGNCTGTA 420
CTTGTCTGTG GGANCTTAGC AAATN 445

SEQ ID NO:1513
SEQUENCE LENGTH:435
SEQUENCE TYPE:nucleic acid
TOPOLOGY:linear
CLONE:HUMGS01722
SEQUENCE DESCRIPTION:
GATCAATCGG ATTGAGAAGA ACATCCTGAG CTCAGCGGAC TACGTGGAAC GTGGGCAGGA 60
GCACGTCAAG ACGGCCCTGG AGAACCAGAA GAAGGCGAGG AAGAAGAAAG TCTTGATTGC 120
CATCTGTGTG TCCATCACCG TCGTCCTCCT AGCAGTCATC ATTGGCGTCA CAGTGGTTGG 180
ATAATGTCGC ACATTGTTGG CACTAGGAGC ACCAGGAACC CAGGGCCTGG CCTTCTCTCC 240
CAGCAGCCTG GGGGCAGGGC AGAGCCTCCA GTCGGACCCC TTCCTCACAC TGGCCCCTAT 300
GCAGANGGTN AGACAGTTCT TCTGGGGTTG GNAGCTGCTC ATTCATGATG GCCTNCTCCT 360
TNAGGCCTCA ATGCCTGGGG GGANGGCTGC ACTGTCCTAT TTGGNCGGGA CANACGGTTT 420
TNTAAAAATT TTAAA 435

SEQ ID NO:1514
SEQUENCE LENGTH:434
SEQUENCE TYPE:nucleic acid
TOPOLOGY:linear
CLONE:HUMGS01724
SEQUENCE DESCRIPTION:
GATCTAACCT AGACTAAAAT TGGGAACTTA TTTGCAATTT TTGACCCNGN CCACTAACTA 60
GTGATTCTNC TCCAAAATTG AGAAAGACAG CACCCATTGA AGCAGATATG TGTGTGAAAG 120
TATATTTTTC AATTCCAGAT TTTTAATTTT AAGGCTCCAG GAAAGAAAGG AGAGTAGAAC 180
ATTTTTCCTC ATTTTATCAA ATCCTCTCTT GCCCTCCCTC AATTCCCCTG TAACATTCCT 240
GAAGCTGTTC CCACTCCCAG ATGGTTTTAT CAATAGCCTA GAGGTAAAGA ACTGTCTTTT 300
TCTCTGATTC TTTAATAAAT TATCTTTATA GGAATATGCA CAAGTTTTTT TTACACTCAG 360

TGTTAAAAGT ATTTATTAAT NGGGAAGTCA ACTTAATGTT TTGAAATAAA NTATATGNCT 420
CTGGTTAATG CAAA 434

SEQ ID NO:1515
SEQUENCE LENGTH:423
SEQUENCE TYPE:nucleic acid
TOPOLOGY:linear
CLONE:HUMGS01726
SEQUENCE DESCRIPTION:
GATCCAGTGA TTCANTCACT TAAATNAAGT CTTTTGGTCA GAAATNACCT TTTTGACACA 60
AGCCTACTGA ATGCTGTGTA TATATTTATA TATAAATATA TCTATTTGAG TGAAACCTTG 120
TGAACTCTTT AATTAGAGTT TNCTTGTATA GTGGCAGAGA TGTCTATTTC TGCATTCAAA 180
AGTGTAATGA TGTACTTATT CATGCTAAAC TTTTTATAAA AGTTTAGTTG TAAACTTAAC 240
CCTTTTATAC AAAATAAATC ANGTGTGTTT ATTGAATGGT GATTGCCTGC TTTATTTCAG 300
AGGACCAGTG CTTTGATTTT GATTATGCTA TGTNATAACT GACCCNAAAT AAATNCAAGT 360
NCAAATTTAT GTNGNCTGTA TAAGGTTTGT AATAANCCAT GNCTGAGGNC AAAAAAGGGN 420
AAA 423

SEQ ID NO:1516
SEQUENCE LENGTH:419
SEQUENCE TYPE:nucleic acid
TOPOLOGY:linear
CLONE:HUMGS01727
SEQUENCE DESCRIPTION:
GATCTGTAAT CTCCACTGCT TGGATGTCTG CCCTCTACCC CAGAGGAATT TACAGAAACT 60
TGCCCTGTGC CTGTGTCCCC CATGCTAGGG GCGGAGGGGT CTTTTCCTTC TTCTTTCCTA 120
CCTACCCCTT TTCTCTTGGC CAGGGGCCTC GTATCCTACC TTTCCTTGTC CCCTGGGCTG 180
GCTGCACAGA GGATTGCCCC TTCTCTTTTC AGAGCTGGCC CTCGATGCCA AATTAGCATT 240
TAGTATTTTG CACAAAGTCT AAGGGACCAT GGCTGCCTGC CTTGGGGAGG AACCATAGCT 300
CCNTCTGGGC CGCTTCTGGC CTCTTGGAGC CATGGGCCAA AGGCAAGGGG ATGGGCAGAG 360
GTCTGTGTTT GGTCTGGCCC AGTTCCNCAT CATTAAACTC AGCCTGACTG CTGCCTAAA 419

SEQ ID NO:1517
SEQUENCE LENGTH:419
SEQUENCE TYPE:nucleic acid
TOPOLOGY:linear
CLONE:HUMGS01728
SEQUENCE DESCRIPTION:
GATCCTCAGT AGANCAGTCC AGAAGCCTGA NAGACAGTGG CCCCTTGATA ATCTGGGTCT 60
CACGGGNCCA GCTAGGGGTC CAGGTTTCAN TCAGTAAATA AGAGTGGTCC ACGTCCTAAA 120
GACACCTCTC CTNNACAAAG ACTTGTGATG CTCTGGGGNT TCTNTGGCCA AGCCCCACCC 180
TTTCCTGGTC ATGGTACCCG TACAGCGTTG ATGGCCACAG CTCGAAGGGG GGCTTTCGTG 240
TCCCCCTGTG CGGTCAGTGT TTTCAGTACC ACCTCTCTCC CGTGCCCACT TGGCTATTTA 300
CTTATTTATT TATTGTGTGC CAGTNATGGT GGGTGGGGGC TGGGCCTTCC CCGCCACCTN 360
CANCCCTGTT GTGACCTGTC CTTCCGTACT TAATAAAGTG CGCGTGNGGA GTTGTTAAA 419

SEQ ID NO:1518
SEQUENCE LENGTH:418
SEQUENCE TYPE:nucleic acid
TOPOLOGY:linear
CLONE:HUMGS01729
SEQUENCE DESCRIPTION:
GATCTNAGCC CTCCGGTTCC TGGGCAGAGA GAAAGGCTAC AGGTTCATTT NCCTGACGAC 60
AGCAGTCACA AACAGCGCTC GCNTTATGGA GGCCATNAGT GAGGTGAAAG CCTGATGTTT 120
TTCCCGGCCA NTNTTGACAT CTTCCCTGAA CACATTCCTC AGTGAGATGC AGGCATCTGG 180
CACCCAGCTG CTATAACCAA GTGTCCACCA ACTACCTGCT AAGAGCCGGG AGCATGGAAC 240
GTGTTGGGAT TTAGAGAACA TTATCTGAGA AAAGAGTTCA CTTCCTGCTC CCAGGATATT 300
TCTCTTTTCT GTTTATGAAG TACAACCCAT GCTGCTAAGA TGCGAGCAGG AAGAGGCATC 360
CTTTGCTAAA TCCTGTTTGA ATGTCATTGT AAATAAAGCC TCTGCTCTCA GATGTAAA 418

SEQ ID NO:1519
SEQUENCE LENGTH:404
SEQUENCE TYPE:nucleic acid
TOPOLOGY:linear
CLONE:HUMGS01730
SEQUENCE DESCRIPTION:
GATCCAATTT ACAGAAATCA GAGTTAGCTA CAGCTAGGAC TCGTTTGGTT GGGGTTTTTT 60
AGTTTGTCTT TCTAAAGTCA TGTGGACCTT AATTTAATTA CAAAAGTCTA CCCTGGTGGT 120
CATAAAATAG GCAGGCCTAT GAAGAAAGGC CTTTTACTCT TCCATCTCGT CCCAGCCCCG 180
AGTTGACCCA CGTTGCTGCT CCTCACACCA TGGTGATGCA GGTCTCGTAG TGTGGGCACA 240
GGCCTGGCTA CCTCATCTTT TTAGTGCCTC TCTCCTCTTC CACAGGATGG GGTCCCACAG 300
CTGCAGCAGC TGGCCCGGTA GTTGAGCATG TGTGGTTATC CTGTAGAGCT TTTCCCAAGA 360
AGGGTGTTTG AACTTAGAGT CTTAATAAAA TCTTACCAAA TAAA 404

SEQ ID NO:1520
SEQUENCE LENGTH:402
SEQUENCE TYPE:nucleic acid
TOPOLOGY:linear
CLONE:HUMGS01731
SEQUENCE DESCRIPTION:
GATCTACAGA TACGTGATAT TNTGGTATAA CTAGAATCTT GATTTCTTTC ATAAAGTTCT 60
GCCATGTTCT ATTTCTTTCC TTAATGTTTT TTTCTTCCCT ACTGTTTTAT CCTCCCTTTG 120
CTTTGGAAGG ATATTNCTGC ATATCGAGCT AAAGGAAAGC CTGATGCAGC AAAAAAGGGA 180
GTTGTCAAGG CTGAAAAAAG CAAGAAAAAG AAGGAAGAGG AGGAAGATGA GGAAGATGAA 240
GAGGATGAGG AGGAGGAGGA AGATGAAGAA GATGAAGATG AAGAAGAAGA TGATGATGAT 300
GAATAAGTTG GTTCTAGCGC AGTTCTTTTT NNCTTGTCTA TAAAGCATTT AACCCCCCTG 360
TACACAACTC ACTCCTTTTA NNGAAAAAAA TTGAAATNTA AA 402

SEQ ID NO:1521
SEQUENCE LENGTH:407

SEQUENCE TYPE:nucleic acid
TOPOLOGY:linear
CLONE:HUMGS01732
SEQUENCE DESCRIPTION:
GATCTCAGCT GAACAAATTA GATGTTTCAG TTGCTCTTGG GTCAACTGGC TTACAGATTT 60
ACATGTGCAC ACACACACAA ATTTCTTATC ACATTTTCGC CTTCTTCACT TGACCTAACT 120
GATTATGCGA AATACCCAAG ATTCATGCTA CTGTACCACA GATTTGTTTT CACAGCAATA 180
AATCTTCAGT TCTTTGTTTA TGATTCCACT TAACAAAAGG CCTGCAGAAG TGATTTATTA 240
TTTGGGTATT TGGAGATAAT ACATTTGATG GTTTTTTGGG AAACCTTTTT CACTCCATAC 300
TCAGATATGC TTCATTGTCA AATGCATATT TAGGNTAGGT TAATNGAATT GTAATGTTTA 360
ACCTGCTGCT TNTTTTTTAA ATAAAAATTT GACTGAAAAT GGTTAAA 407

SEQ ID NO:1522
SEQUENCE LENGTH:397
SEQUENCE TYPE:nucleic acid
TOPOLOGY:linear
CLONE:HUMGS01733
SEQUENCE DESCRIPTION:
GATCTAGTAG CCACGCTAAA AAACAAGTAA AATTAATTTT AATATATTTA ACTCAATACA 60
TTGGAGATAT TATTTCAGAA CGATATTTTT CATTCTTATG AAACTAAGTC TGTCTGGTTT 120
GCATATCTCA GTTGAGACAC TAAAGTTTTA CTGATAATAC TTAATCCATN NATTAGATTT 180
CATAAAATCT AAATTTGCAA AGTAAATTTA CATACCCATG TNGGTCTAAA TGTGCTTAAA 240
AGTTTTCCAA TAACTGAAAT GAGTATCAGT TTTAAATTTA AAAATTTGTT ACTTGGTTAC 300
TCCATATTTC AGGTATTAGT GGCTACCATA TTGGTCAGAG CAGATTCATA GGGATGATTT 360
CCCAGGTTTG AAGTGTGTGT ATATTGCTGG TATTATN 397

SEQ ID NO:1523
SEQUENCE LENGTH:395
SEQUENCE TYPE:nucleic acid
TOPOLOGY:linear
CLONE:HUMGS01734
SEQUENCE DESCRIPTION:
GATCTGTACG AAATGTGAAA TNTTTAGGGA CATCTCCATG CTGTCACTTG TNATTTGCCC 60
TCTNATGTAT TTNGGTCATA TTGCCAACTG GAAAGTCAAA ATTTCCTAAC AACTTTAAGT 120
AAGTCCTTTN AAGACTNAGT GCTGTTTTNA ATCCAGTTTA GAAAGTAACT TAATTTTAAT 180
ACCGCTACTA AAAATNCGAA AATTTCTCCT TTAATCACAT TCAATATGGT TAAANGAACA 240
ACACTAATTG ACATTGCGTG GGCTTTTCCT CCCTTTGTTT AAANANGTCA TTTTGTTGAG 300
CAAGNGGTTG TATAGTATTA TCTACTNNCT TGAGGCTGTT AAATTTNCNA TTCCAGNGGT 360
TTTGTAAATG TATCCCGGGG CCNTGNTGCA TTGGN 395

SEQ ID NO:1524
SEQUENCE LENGTH:391
SEQUENCE TYPE:nucleic acid
TOPOLOGY:linear
CLONE:HUMGS01735

SEQUENCE DESCRIPTION:
GATCTCAATG ACCTGGCGAA GACTGGAAAA TACAACTCCC ATCACCAGCA GAGTTGCTAG 60
GCTGCTGCTG TATGGGTACA GAGAAGGGTT TGACAAAATT GACCTGACTG TGGAAGAACT 120
TCCAGCAGTT TACACAGGAC TGTTTTATAA ACTCTATCAT AAGGAACTGC CAGAGAATTA 180
TAAAACTATG AAAGGAATTG ATTAAATTCA CATTTATATG TTTAGAAACA TGTAGACTAA 240
CGAATGACAT AAGAAATAGT GGACATTTTG GATTGATTAA ACATCTGACT GTGATTTTCT 300
AATGTATATG ATTTCCATGA AGAAATTTTG TTTCTAAACA TGCACATTTT AAAAGCCTCT 360
TTTCGAATAA AGCAAATGCG TGAAAANGAA A 391

SEQ ID NO:1525
SEQUENCE LENGTH:388
SEQUENCE TYPE:nucleic acid
TOPOLOGY:linear
CLONE:HUMGS01736
SEQUENCE DESCRIPTION:
GATCCGGCGC CACAAGACCA CCATCTTCAC GGACGCCAAG GAGTCCAGCA CGGTGTTCGA 60
ACTGAAGCGC ATCGTCGAGG GCATCCTCAA GCGGCCTCCT GACGAGCAGC GGCTGTACAA 120
GGATGACCAA CTCTTGGATG ATGGCAAGAC ACTGGGCGAG TNTGGCTTCA CCAGTCAAAC 180
AGCACGGCCA CAGGCCCAGC CACAGTGGGG CTGGCCTTCC GGGCAGATGA CACCTTTGAG 240
GCCCTGTGCA TCGAGCCGTT TTCCAGCCCG CCAGAGCTGC CCGATGTGAT GAAGCNCCAG 300
GACTCGGGAA GCAGTGCCAA TGAACAAGCC GTGCAGTGAG ACCCCCAAGA GGCCCATTTC 360
CCCCAATAAA AGAGATTTGG GAGTCAAA 388

SEQ ID NO:1526
SEQUENCE LENGTH:388
SEQUENCE TYPE:nucleic acid
TOPOLOGY:linear
CLONE:HUMGS01737
SEQUENCE DESCRIPTION:
GATCTTATTT CTTCTACTCA ATTGTATATT TATACCCATT AATCAACTTC TGTTCTTGCT 60
CTCTCCGTGC TACCCTTCTG GCCTTTGGTA ATCGCCAATC AATCTACTCT CTATCTTCAT 120
GAGACCAGCG TATTTTAGCT CCCACATATG AATGAAAACA TATAATATTT GTCTTTCTGT 180
GCTTGACTTA TTTTACTTAA CATAATGACC TCCAATTCCA TCCATCTTGT GGCACTGACA 240
GTATTTCATT CATTTTTGTG TGAATTATAT TTCGTTGTGA ATATACATTA CATTTTCTTT 300
ATCCATTCAT CACTGATGTG CACTTAGGTT GATTCCATAT TTGGGCTATT GTGAATAGTG 360
TGTTAATAAA CATGAGAGTG CAGTTAAA 388

SEQ ID NO:1527
SEQUENCE LENGTH:385
SEQUENCE TYPE:nucleic acid
TOPOLOGY:linear
CLONE:HUMGS01738
SEQUENCE DESCRIPTION:
GATCTTCCTG GTCAGCGCTC TCTCTAGCAT ACTCTTCCTC TATTTGGCTC ACAAACAGGC 60
ACCAGAGAAG CAAATGGCAC CTTGAACTTA AGCCTACTAC AGACTGTTAG AGGTGATACT 120

ATGACCATGA GTAGCATCAG CCAGAACATG AGAGGGAGAA CTAACTCAAG ACAATACTCA 180
GCAGAGAGCA TCCCGTGTGG ATATGAGGCT GGTGTAGAGG CGGAGAGGAG CCAAGAAACT 240
AAAGGTGAAA AATACACTGG AACTCTGGGG CAAGAGATGT CTATGGTAGC TGAGCCAAAC 300
ACGTAGGATT TCCGTTTTAA GGTTCACATG GAAAAGGTTA TAGCTTTGCC TTGAGATTGG 360
CCTCATTAAA ATCAGNGACT GTAAA 385

SEQ ID NO:1528
SEQUENCE LENGTH:377
SEQUENCE TYPE:nucleic acid
TOPOLOGY:linear
CLONE:HUMGS01739
SEQUENCE DESCRIPTION:
GATCCGTACT CAGAACGTCC AGGAGAGACG CATGGCCCGA AGTCAAAGTG CTGGAATTTT 60
CCAAAACAGC CTGTTCTCTC CTCTCTCCTC CCCAGAGCAC CCCCTGCCAT CAGGGGGGTT 120
GAAATCCCTC TCCCCCAGGA GCCCTGCTGC TTTGCTTGGT GGTAGGGCAG GAGAGCAAAC 180
AAACAGTCAT GGTCTAAAAC CCACATAGCA CTTTGCTCTT AGTTACATGT AAAATTTTAG 240
ATTTCTAAAA CAGGTGGGCA ATCATTTTGA ATACTGTTCT GTGACCCTGA CTGCTAGTTC 300
TGAGGACACT GGTGGCTGTG CTATNGTGTG GCCATGCTCC NTTGTCCCCG TNCCTGTTGG 360
CTGCTTCTGT TTAGGTN 377

SEQ ID NO:1529
SEQUENCE LENGTH:167
SEQUENCE TYPE:nucleic acid
TOPOLOGY:linear
CLONE:HUMGS01740
SEQUENCE DESCRIPTION:
GATCCAAGGC CTGTGGTAAA CGGGAGANCT TGTNTTTTTC AAGTGGAAAA AAACCCAAGA 60
GTTTGTNCAG ACATCCTGTC TTCCCAGAGA AGGTGGACAC TCTTGGNCTC ATTGTAAAGT 120
GCCTGCTGNA TCAATAAAGC TCTTGGCTTA TTAGTCTATA AAACAAA 167

SEQ ID NO:1530
SEQUENCE LENGTH:372
SEQUENCE TYPE:nucleic acid
TOPOLOGY:linear
CLONE:HUMGS01741
SEQUENCE DESCRIPTION:
GATCCACACG TTCCGGGACT ACCTGCACTA CCACATCAAG TGCTCTAAGG CCTATATTCA 60
CACACGTATG CGGGCGAAAA CGTCTGANTT CCTCAAGGTG CTGAACCGCG CACGACCAGA 120
TGCCGAGAAA AAAGAAATGA AAACAATCAC GGGGAAGACG TTTTCATCCC GCTAATCTTG 180
GGAATAAGAG GAGGAAGCGG CTGGCAACTG AAGGCTGGAA CACTTGCTAC TGGATAATCG 240
TAGCTTTTAA TGTTGCGCCT CTTCAGGTTC TTAAGGGATT CTCCGTTTTG GTTCCATTTT 300
GTACACGTTT GGAAAATAAT CTGCAGAAAC GAGCTGTGCT TGCAAAGACT TTCATAGTTC 360
CCAAGANTTA AA 372

SEQ ID NO:1531

SEQUENCE LENGTH:372
SEQUENCE TYPE:nucleic acid
TOPOLOGY:linear
CLONE:HUMGS01742
SEQUENCE DESCRIPTION:
GATCCACTGT TGGGGCCTCG ACGAGTTCCT GTCCTGGGAA AGGAGTACAC CGAGAAGACC 60
CCCATTTCTG AGCATGCTGT TTTCAATGTG GACCTCATGA GCAAGAAAAT TCATCTGACT 120
GAGAATGGGA TAAGGGTGGA TATTGGCGAT ACAATAATCT ATCTGGTTCA TTAAACTCAT 180
GCACATTGGA GATTTATCCT GGTTTCTTAG GAATACTACT ACTCTGATTG TGTCTACTGA 240
TTGGCTATCA GAACCTTAGG CTGGACCTAA ATAGATTGAT TTCATTTCTA ACCATCCAAT 300
TCTGCATGTA TTCATAATTC TATCAAGTCA TCTTTGATTC CTGGACCTAA TAAATTTTTT 360
TTCCCTTTCA AA 372

SEQ ID NO:1532
SEQUENCE LENGTH:368
SEQUENCE TYPE:nucleic acid
TOPOLOGY:linear
CLONE:HUMGS01745
SEQUENCE DESCRIPTION:
GATCCCAGTC ACAATTATCA CCGGGTATTT AGGTGCTGGG AAGACAACAC TTCTGAACTA 60
TATTTTGACA GAGCAACATA GTAAAAGAGT AGCGGTCATT TTAAATGAAT CTGGGGAAGG 120
AAGTCCGCTG GAGAAATCCT TAGCTGTCAG CCAAGGTGGA GAGCTCTATG AAGAGTGGCT 180
GGAACTTAGA AACGGTTGCC TCTNCTGTTC AGTGAAGTGA GGAATGTGTT TACTGTGTAC 240
ATGGTTTACT AGAAATGTTT ATTGATTATA TTTCCAGCTT TAATTTTCTT GAGTAATTTA 300
ACTGAATTTA CACAGTTTGC TTCATTGTAT TTTCAAACAA ATAGAAAATA AACTTATTAG 360
GAAGCAAA 368

SEQ ID NO:1533
SEQUENCE LENGTH:364
SEQUENCE TYPE:nucleic acid
TOPOLOGY:linear
CLONE:HUMGS01746
SEQUENCE DESCRIPTION:
GATCGTCCCC GTATGATGAT TGTNAGAAGA CAGGACTAAG TAGCAGAGAA TAGCTAAGAG 60
ATAAATTGGG CTGGGGAAAC TTGTCAGAAA GCACTGAACA ATTAAGAAAT TTTCCAAGAA 120
AATGTGCAGT ATTCTCTGCT ACTTCTGAAT CTGTTTTGTC TTCCTAATCT ATCACAATTG 180
CCACCCATCG GGTTTTGGGT GTGTGTTTTC ATAGCGTGGT TACTTTCTAT AATGCTGTAC 240
CCAGATTCTA AGAACCTGGA GAAGGATTAG CAGTTCTTAG TAAGTTTACT GTGTATAGGA 300
ACGGTTTGTA TTTCATTACA GCTATTCATC TTTTCTACAT TAAAAATATT TTTCTCTAAA 360
GAAA 364

SEQ ID NO:1534
SEQUENCE LENGTH:362
SEQUENCE TYPE:nucleic acid
TOPOLOGY:linear

CLONE:HUMGS01747
SEQUENCE DESCRIPTION:
GATCTGTGCC ATAAAATTTC AGTGTAATAA GACTTCTTCA ATACATCTTC CAATAAGGGG 60
TGCTTCTTTG TGACAGTATT TTTATTTCTG ACATTCATTT TATTTGGGTA CATAGTGTGG 120
TTGTTGATAC CTTGCAATAG TATTGCTTCT GAAAGTAATA AAAAATTTTA GGAGAATTTG 180
AGAAGTTTAC AGAATTACTT ATTCATTGTT TTCTTAGTAA GTCAGTTTAA TGTTTATTTT 240
TCTCATTATT TCATCACTGC AATAAAGAAT AAGGGTGTTT GAGCTCACCT CCATGCAAAG 300
ACTTCAGTTT TAAAACATTA TTTGCCTAAA ATTAGCATTG TGATGCTTTC TGAAAGAAAT 360
AN 362

SEQ ID NO:1535
SEQUENCE LENGTH:361
SEQUENCE TYPE:nucleic acid
TOPOLOGY:linear
CLONE:HUMGS01748
SEQUENCE DESCRIPTION:
GATCCACCTC TCCTGTGCTA AGATTAAGAN GACCAACGTC CCCGANTTCT TTNATTGCCA 60
GAAATGCAAG GAACTGAGGC CAGAGGCCNN GCGGTTAGGG GGGCCTCCCA AATCTGGAGA 120
GCCCTGATGG CACCAACTTT AGCCTGGAAC TTCCAAATNA CAACATGATT TGGGAACTGA 180
GCCTCAGGGT CCTCAGCCTA TCCCCTGGAG CTTGGATACT GTCTGCACTT CAAGGCAGGA 240
ATTCTCAAGG GAGACTTGTT TGAAAATNAG TGTCTCACTT TCCCACCCTA TCCTTCCTCC 300
CCACTCTGTG GACTTGAAAT TGAATCCATT ACGGTTGGGG ATGGGAGGCT GTCTGTGTCC 360
N 361

SEQ ID NO:1536
SEQUENCE LENGTH:360
SEQUENCE TYPE:nucleic acid
TOPOLOGY:linear
CLONE:HUMGS01749
SEQUENCE DESCRIPTION:
GATCGATTCT CTCTATGAAG GAATCGACTT CTATACNTCC ATTACCNGTG CCCGATTNNA 60
AGAACTGAAT GCTGACCTGT TCCGTGGCAC CCTGGACCCA GTAGAGAAAG CCCTTCGAGA 120
TGCCAAACTA GACAAGTCAC AGATTAATGA TATTNTCCTG GTTGGTGGTT CTACTCGTAT 180
CCCCAAGATT CAGANGCTTC TCCANGACTT CTTCAATGGA AAAGAACTGA ATANGAGCAT 240
CAACCCTGAT GAAGCTGTTG CTTATGGTGC AGCTGTCCAG GCAGCCATCT TNNNTGGAGA 300
CAAGTNTNNG AATGTTCAAG ATTNGCTGGC TCTTAGGNTG TCACTACTCT TTTCCCTTAN 360

SEQ ID NO:1537
SEQUENCE LENGTH:358
SEQUENCE TYPE:nucleic acid
TOPOLOGY:linear
CLONE:HUMGS01750
SEQUENCE DESCRIPTION:
GATCCCAGTG AATCCTGGGT CCAGGAGTAC GTGTATGACC TGGAACTGAN CTGAGCTGCT 60
CAGAGACAGG AAGTCTTCAG GGGAGGTCAC CTGAGCCCGG ATGCTTCTCC ATGAGACACA 120

TCTCCTCCAT ACTCAGGACT CCTCTCCGCA GTTCCTGTCC CTTCTCTTAA TTTAATCTTT 180
TTTATGTGCC GTGTTATTGT ATTAGGTGTC ATTTCCATTA TTTATATTAG TTTAGCCAAA 240
GGATAAGTGT CCCCTATGGG GATGGTCCAC TGTCACTGTT TCTCTGCTGT TGCAAATACA 300
TGGATAACAC ATTTGATTCT GTGTGTTTTC ATAATAAAAC TTTAAAATAA AATGCAAA 358

SEQ ID NO:1538
SEQUENCE LENGTH:354
SEQUENCE TYPE:nucleic acid
TOPOLOGY:linear
CLONE:HUMGS01751
SEQUENCE DESCRIPTION:
GATCCCTCCT CTAGGGGCCT GGGGACTTTN ACTGATGCTC TTCCTGATTC TAGAGCAAAG 60
GTGTGGGAAG GGGAAATGGA GGAATGCCCT CCTGTCTGTN TCGTTCTCTG TGCCACAGCT 120
ACAGATGCAG AAGGTTTCTC TGGATAGCAC ACCTCTGANT GTAAATCATG ATAAAATGGA 180
TATTTGGAAA CTTACTCCTA AGCTGTGATT TAGGGTGTAT TTCTACTTCT GGACTGCCTC 240
AATATCAAGG GCTGAGACTT TTGAATTTTG AATATTCGTT GGGTTTCATG TTAAGANGCC 300
TGTGGTCTAG GAGTGCTATT CAGTGTTTCT TTTCCTGATA AACACTTTGA ATAN 354

SEQ ID NO:1539
SEQUENCE LENGTH:352
SEQUENCE TYPE:nucleic acid
TOPOLOGY:linear
CLONE:HUMGS01752
SEQUENCE DESCRIPTION:
GATCTCAGTA TGGACAGTGT AACAACAAAA CCAAAATGGC TGGACAGACT TCTTGTGTTT 60
TGTAAATATA AACTAGGACA GTTCTGTAGG TTTGTTCAGT GTGCTAGTGG AGTATTTCCT 120
TAATGTAAAA CTTCACTTAA CAGAGAGATT CTTTGTTTAG CAAGCTTGGN GTGATGATAN 180
AGNGGTAAGA AATAATATAA ATGTTGAAGA AAGCATCACA ACAGAACTAT AGGAGTCTAA 240
ATTTAATAAA TCTTTAAAAA AAACCAGTGT CTAGAATATA TACCATGTTT TATTATTTAA 300
AATCATTGTC TTAAATTTTT GTTCAAAAAA TAAAAATTTG AATACAATCA AA 352

SEQ ID NO:1540
SEQUENCE LENGTH:351
SEQUENCE TYPE:nucleic acid
TOPOLOGY:linear
CLONE:HUMGS01753
SEQUENCE DESCRIPTION:
GATCAGACAA GCCCAAACTG AGAGACACTC TACAAAATAA CTGGCCAGTA CGTTTTNCAA 60
GTNCCAAGGC CGTGAACAAT AAGGAAAGAC TGAGGAACAG TCATGGACTG GAAGAGAGTA 120
AAGAAATATG ATAACTAACT GCATTGTGAG ATACCGATTT AGATGTTGGA TTAGAAAAAG 180
GACCAGAAAT AAGATTTCAT CAATGTCAAT TATTTGGTTT TGATAATTGT ACTGTGGTTA 240
TGTAAGATGT TAACATTAGT AAGAGCTGGG TGAAGGGTAC ATGGAAACTA TTATTTTTGC 300
CACCCTTTGA AAGTCTAACT TTTTTCAAAA TAAAAAGTTT TAAAAAATAA A 351

SEQ ID NO:1541

SEQUENCE LENGTH:357
SEQUENCE TYPE:nucleic acid
TOPOLOGY:linear
CLONE:HUMGS01754
SEQUENCE DESCRIPTION:
GATCCCAGAT GCAGTGACTG GTTACTACCT GAACCGTGCT GGCTTTGAGG CCTCAGACCC 60
ACGCATAATT GGGCTCATCT CCTTAGCTGC CCAGAAATTC ATCTCAGATA TTGCCAATGA 120
TGCCCTACAG CACTGCAAAA TGAAGGGCAC GGCCTCCGGC AGCTCCCGGA GCAAGAGCAA 180
GGACCGCAAG TACACTCTAA CCATGGAGGA CTTGACCCCT GCCCTCAGCG AGTATGGCAT 240
CAATGTGAAG AAGCCGCACT ACTTNACCTG AGCCACCCAA CCTAAATGGT ACTTATCTGT 300
NCCCATGTNC NTACACCAGC CTGTTTTNAT AATAAACTTT ATTGGTGGTA GGGNAAA 357

SEQ ID NO:1542
SEQUENCE LENGTH:412
SEQUENCE TYPE:nucleic acid
TOPOLOGY:linear
CLONE:HUMGS01755
SEQUENCE DESCRIPTION:
GATCAGGGCA GTAGGCATAA TTCAGCAACA AACAATCTTC CTTTGGGAGA AACCTNTTCA 60
TTCCAATCTT CTAATTACAG TGGTTCCTAT CTCAGGGATA CTGGACTTTN TGACGCAGAT 120
GAACAATTAA GGGGAAAAGC TTCCCTTTTC CCTCTGTGGC AGTTACGATT TTNACTTCAG 180
TCCTGAGAAA AACTTCAGGT TTTGAAAATC AGATGATGTC TTCTCCTTTT CCAANCACCA 240
CACGTTGAAA GCNTTTATAA ATCCAAGTCT GAAACTCTGC GCTCTAGTAC TGCTGTTAAG 300
NTACACAACT TGTTTTCTNN GTTCATATAA TCTNGGGGTA CNCACNCACA CACNCNTGTA 360
TGGGGGGGGG GGNNTGGCGN TTNCCACCGC GCGTTCCNTT TTTGNNGGGT CN 412

SEQ ID NO:1543
SEQUENCE LENGTH:343
SEQUENCE TYPE:nucleic acid
TOPOLOGY:linear
CLONE:HUMGS01756
SEQUENCE DESCRIPTION:
GATCCCAAAA TGCTGCGGGG CAATGACAGC TCAGTTCCCA GAAATAAAAA TCCATTCCAA 60
GAGGCCATTG TTTTTGTGGT GGGAGGAGGC AACTACATTG AATATCAGAA TCTTGTTGAC 120
TACATAAAGG GGAAACAAGG CAAACACATT TTATATGGCT GCAGTGAGCT TTTTAATGCT 180
ACACAGTTCA TAAAACAGTT GTCACAACTT GGACAAAAGT AACACAGAAG AACCTTACTA 240
TGATAATCTA CTTGGAATGT GGATAAATGT AAAANGNAGA AAAGTTAGAA NGNGCANTAT 300
GTTTCCTTCT CTGTAACAGT GTCCTAACAG TGAAAATTCA GGN 343

SEQ ID NO:1544
SEQUENCE LENGTH:340
SEQUENCE TYPE:nucleic acid
TOPOLOGY:linear
CLONE:HUMGS01758
SEQUENCE DESCRIPTION:

GATCGANCTT NCTATGGACT CAAGCAGGTG NNGAAGGCCA ATNAAGCCAT GGCAATTGAC 60
ACATTGCTCA TCAGCGATGA GCTCTTCAGG CATCAGGATG TAGCCACACG GAGCCGGTAT 120
GTNAGGCTGG TGGACAGTGT GAAAGAGAAT NCAGGCACCG TTAGGATATN CTCTAGTCTT 180
CACGTTTCTG GGGAACAGNT CAGCCAGTTG ACTGGGGTAG CTGCCATTCT CCGCTTCCCT 240
GTTCCCGAAC TTTCTGACCA AGAGGGTGAT TCCAGTTCTG AAGAGGATTA NTGATTGAAA 300
CTTAAAATTT GNGACAATCT TTGTGTTTTC CTAAACTGTN 340

SEQ ID NO:1545
SEQUENCE LENGTH:339
SEQUENCE TYPE:nucleic acid
TOPOLOGY:linear
CLONE:HUMGS01759
SEQUENCE DESCRIPTION:
GATCTGCTTT CATTAACTGG AATTCTGTAG GAGATACTGG TGACCTAAGC TAAGTTGCAC 60
TCAGCATACT CAGTGTCAAG CTAATGAGGT TCTATTATAA AGGTTCTACT TTTAATCTGA 120
GGGAAAACAT GTTCAGGGCT TCTAGAACAC TAAAAAATTN GGNTTAAACC AGTGTNCAGT 180
CTGGTGCCAA ACTTCGAATG GAATACAAAT NNACATAATC TGANCTTTGT NCACAGGTTA 240
TCCTAATAGA GTAATTCTCC ACTTTGCTCT ATTGAACTGT CTTAAGGGAT TTGTTTAAAC 300
AGCTAAGTTA CTTGATTAAA ATAATGATAA AATTGTAAA 339

SEQ ID NO:1546
SEQUENCE LENGTH:362
SEQUENCE TYPE:nucleic acid
TOPOLOGY:linear
CLONE:HUMGS01760
SEQUENCE DESCRIPTION:
GATCTGGTAG AAGGTACCAG CTTCCTTTCC AGCTGGAGAG GCCCCAACAC TGGATGGTTC 60
TGTAGGGAGC CTAGGGAGCC TGGTCATCAA CTTGCAATAC CTCACAGAGC CAGTTCACAT 120
CCCACTCTGA GCTCCCACGA GAAACACTGC TTCTCCAGGC CCGGGGTTGT TGGGGAGAGA 180
GGCAGAGGCA GCTGGAGCGC CGTTCTCTCC TGCTGGGACA CCGCTTGGGC TTTGGTATTG 240
ACTGAGTGGC TGACAGTTAT CTTCCAACCC CAACTGGCTT GGGGGCAGGA CAAGGGCTTA 300
GGCTTGATGG TGGNCAGGCT TGNCTGCTCC CCACCTGNGA TGCCCCTGCT CTGGGACCTC 360
TN 362

SEQ ID NO:1547
SEQUENCE LENGTH:335
SEQUENCE TYPE:nucleic acid
TOPOLOGY:linear
CLONE:HUMGS01761
SEQUENCE DESCRIPTION:
GATCCTTTCA GAAAAGAAAC ACTGGGTCCC ATAGCTAAAT TCTCAACCGC CANGCACCTT 60
CAGAAGAATC CAGCCTAATA CTGGAATTTG TGCTATTATC TTCCTCTCCA GCCCCCCAAC 120
TCCATCCCTC ACCACAGTTG TCTAGGAAAT GACATGAATT CAATATCTAA TGTCAACCAA 180
TGGGGAGAGC CACAACTCCA GGAGAGGTTC CTGAGCTGAG TCCCTTAATT TCTGGATGAA 240
GAAGACCAAC AAGTTTTGTC CAATGTATTT GTTTCTCAGA CCTTGCCTAG GCACTAAAAA 300

TAAAATACTA GGTCATTGGA GGCTAATGTG GGAAA 335

SEQ ID NO:1548
SEQUENCE LENGTH:330
SEQUENCE TYPE:nucleic acid
TOPOLOGY:linear
CLONE:HUMGS01763
SEQUENCE DESCRIPTION:
GATCATTAAG GAAGGCGATG TAGATGTTTC AGATTCTGAT GATGAAGATG ATAATCTTCC 60
TGCAAATNTT GACACATATC ACAGAGCCTT GCAAATAATA GCAAGATATG TACCATCATG 120
AGTATACTGN TCCTTATTTT GAATGTTTAA TTCTCAAGAA AATTGTAATC AATTAGTAAA 180
AATTATAAAA TGTTAATAGT ATTAAAGCTT GAGTCTTACA TTGCATTTTT TTTTTTNGNA 240
NCCACTTGGG GAACCATTCC ATNCTCCAAA ANGGGGCATT CCCATTTCCT NTTNATCCCC 300
TNNAATGGTT TTNCAAAGTN NGTNTGCNGN 330

SEQ ID NO:1549
SEQUENCE LENGTH:330
SEQUENCE TYPE:nucleic acid
TOPOLOGY:linear
CLONE:HUMGS01764
SEQUENCE DESCRIPTION:
GATCATGGTG GATGAGGAAG CCTCAACGTA GATTCCTGAA CTCAAGGTAC CAGCAAGANT 60
GCNTTCTCCC AGTGTGCTCT CCCCAACATC CTAGGCACAG CTTTCATAAC CCAGTTTCTT 120
AGGTGTAAGA AACTGTTTTN ATCTCATTTA TTAAGTCTCA GAACTTAACA GAAAAGGAAG 180
CCTTTTAAAT ATTCTTTTTA ATNTTATTTT AGATTAACAG TTTTGNACTT TACATTTTTT 240
TATACAACCA NCCAGTTTCT TTNCTAGCCA ATCATCTCTG ANGAGTTGCT GTTTCTTACT 300
GACAATAAAA NNTGTNCTCT TGGTTCGAAA 330

SEQ ID NO:1550
SEQUENCE LENGTH:329
SEQUENCE TYPE:nucleic acid
TOPOLOGY:linear
CLONE:HUMGS01765
SEQUENCE DESCRIPTION:
GATCAACATC TTTCCTTGCC TCTGTCCCCT TCTCTCATCT CTTAGCTCCC CTCCAACCTG 60
GGGGGCAGTG GTGTGGAGAA GCCACAGGCC TGAGATTTCA TCTGCTCTCC TTCCTGGAGC 120
CCAGAGGAGG GCAGCAGAAG GGGGTGGTGT CTCCAACCCC CCAGCACTGA GGAAGAACGG 180
GGCTCTTCTC ATTTCACCCC TCCCTTTCTC CCCTGCCCCC AGGACTGGGC CACTTNTGGG 240
TGGGGCAGTG GGTCCCAGAT TGGCTCACAC TGAGAATGTA AGAACTACAA ACAAAATTTC 300
TATTAAATTA AAATTTNTGT GTCTCCAAA 329

SEQ ID NO:1551
SEQUENCE LENGTH:334
SEQUENCE TYPE:nucleic acid
TOPOLOGY:linear

CLONE:HUMGS01766
SEQUENCE DESCRIPTION:
GATCCCAACT GCTCCCTNCC CTCGCCGGTG ACTCCTGCAC CTGCGCCGGC TCCTGCAAAT 60
GCAAAGAGTG CAAATNCACC TCCTGCAAGA AAAGCTGCTG CTCCTGCTGC CCTGTGGGCT 120
GTGCCAAGTG TGCCCAGGGC TGCATCTGCA AAGGGGCGTC GGACAAGTGC AGCTGCTGCG 180
CCTGATGCTG GGACAGCCCC GCTCCCAGAT GTAAAGAACG CGACTTCCAC AAACCTGGAT 240
TTTTTATGTA CAACCNTGAC CGTGACCGTT TGCTATATTC CTTTTTCTAT GAAATAATGT 300
GAATGNTAAT AAAACAGCTT TNACTTGAAA CAAA 334

SEQ ID NO:1552
SEQUENCE LENGTH:328
SEQUENCE TYPE:nucleic acid
TOPOLOGY:linear
CLONE:HUMGS01767
SEQUENCE DESCRIPTION:
GATCTGTTTG GACTATGTTT TCTTTTCTTC TCCCACTTGC TCAGCAGCTT GGGCTTCCAT 60
TCTAGTTCTT TTACCAAGAT TTTTGTGTGA CCATGTTGAC TTCATTTGGA TTGCCCTCTT 120
TCAATTTCCT TGTGAAAACA CCCTTAACTT TCTCTTTACC CTTAGCTGAA ATGTTTACAT 180
AGCTTCTGGT GATATCTTTN CATGATTTTA TATCTCTTAA AATGGTGATG GATGTGACAC 240
CTCATAAAAG TGAGCTTTGA ACTGTAGATA ACTCTTAAAG AAAATGTCAT TTTAGACAAT 300
TAAAATATTT GTGCTCAACT GCTTGAAA 328

SEQ ID NO:1553
SEQUENCE LENGTH:327
SEQUENCE TYPE:nucleic acid
TOPOLOGY:linear
CLONE:HUMGS01768
SEQUENCE DESCRIPTION:
GATCGAAATG TTAACACCGG GAGCTCTNCA GGACACTCAC CCAGCGACGC TCGTGGGGGA 60
AACATACTAA ACGGACAGAC TCCAAGANCT GCCACCGCTG GGCCTGCACT GCGGCCCCCC 120
ACGTGAACTC GGTTGTAACG GGCCNGGGAA GAAAAGCAGA GAGAGAATTG CAGAGAATCA 180
GACTCCTTTT CCAGGGCCTC AGCTCCCTCC AGTGGTGGCC GCCCTGTACT CCCTGACGAT 240
TCCACTGTAA CTACCAATCT TCTACTTGGT TAAGACAGTT TTGTATCATT TTGCTAAAAA 300
TTATTGGCTT AAATCTGTGT AAAGAAA 327

SEQ ID NO:1554
SEQUENCE LENGTH:326
SEQUENCE TYPE:nucleic acid
TOPOLOGY:linear
CLONE:HUMGS01769
SEQUENCE DESCRIPTION:
GATCAGCAGA CCACGTATTT AAAATTCTGA ATCTTCTGGG ACAGGGTTGT AACTCAGCCT 60
TCCAAAGGGA AGAGTGCAGG GGGACGGGGC CATGATATGG GGAAATGGTG TAAACTAATG 120
TATTTNTTTA TTGGCTGTTA TTCTGTATAA CACTCATATC TTTGCCAAAG TTCAATTTTA 180
TATTTAGGCA ACTGATGGTC CTTTTGCATT TAGGATTTTN GTTGTTGTTA CCTTATACCT 240

CATGATATAA GGAATGGGCT CATGTGTCTT CCGTCTTTTG GAAGGAGGTT GACATATTTT 300
AAATAAATGC TTTTAAATAC AGTAAA 326

SEQ ID NO:1555
SEQUENCE LENGTH:387
SEQUENCE TYPE:nucleic acid
TOPOLOGY:linear
CLONE:HUMGS01770
SEQUENCE DESCRIPTION:
GATCAAAAAG AACAGAACCC TCTCCAGCCT GCTGACCCGA ACCCAACCAC ACAATGGTTT 60
GTCTCAATCT GACCCAGCGG CTGGACCCTC CGTAAATTGT TGACGCTCTT CCCCCTTCCC 120
GAGGTCCCGC AGGAGCCTAG CGCCTGGCTG TGTGTGCGGC CGCTCCTCCA GGCCTGGCCG 180
TGCCCGCTCA GGACCTGCTC CACTGTTTAA CACTAAACCA AGGTCATGAG CATTCGTGCT 240
AAGATAACAG ACTCCAGCTC CTGGTCCACC CGGCATGTCA GTCAGCACTC TGGCCTTCAT 300
CACGAGAGCT CCGCAGCCGT GGCTAGGATT CCACTTCCTG TGTCATGACC TCAGGAAATA 360
AACGTCCTTG ACTTTATAAA AGCCAAA 387

SEQ ID NO:1556
SEQUENCE LENGTH:325
SEQUENCE TYPE:nucleic acid
TOPOLOGY:linear
CLONE:HUMGS01771
SEQUENCE DESCRIPTION:
GATCACAGAT GGATNTTGTT ATAAGCATCA ATGTGACACT TGCAGGNCAC TACAACGTGG 60
NACATTGTTT GTTTCTTCCA TATTTGGAAG ATAAATTTAT GTGTAGACTT TTTTGTAAGA 120
TACGGTTAAT AACTAAAATT TATTGAAATG GTCTTGCAAT GACTCGTATT CAGATGCTTA 180
AAGAAAGCAT TGCTGCTACA AATATTTCTA TTTTAAGAAA GGGTTTTTAT GGACCAATGC 240
CCCAGTTGTC AGTCAGAGCC GTTGGTGTTT TTCATTGTTT AAAATGTCAC CTGTAAAATG 300
GGCATTATTT ATGTTTTTTT TTTTN 325

SEQ ID NO:1557
SEQUENCE LENGTH:322
SEQUENCE TYPE:nucleic acid
TOPOLOGY:linear
CLONE:HUMGS01772
SEQUENCE DESCRIPTION:
GATCTGTAAT TCATAGTCAC ATCAAGGTCA TCAAGACCAG GAAAAACAAG AAAGACATAC 60
TCAATCCTGA TTCAAGTATG GAAACTTCAC CAGACTTTTC CTTCTAAAAT CTGGATGTCA 120
TTGACGATAA TGTTTATGGN GATAAGGTCT AAGTGCCTAA NAAAATGTAC ATATACCTGG 180
TTGAAATACA ACACTATACA TACACACCAC CATATATNCT AGCTGTTAAT CCTATGGAAT 240
GGGGTATTGG GAGTNCTTTT TTAATTTNCC ATAGTTTTTT TTAATAAAAT GGCATATTTN 300
GCATCTACAA CTTCTATAAT TN 322

SEQ ID NO:1558
SEQUENCE LENGTH:319

SEQUENCE TYPE:nucleic acid
TOPOLOGY:linear
CLONE:HUMGS01773
SEQUENCE DESCRIPTION:
GATCTTTCTT TAATAGTGAA CCCCTGGGCC ACTGAAGAGT AACATGGCTC CACTGGACAC 60
AAAAGAGGGA TGGAATCAAC AGGCAGGGGG CCTTTTATAA GCCTTAGGAA AAGAAAATGA 120
AACTATTTCA TCTTTGGACT TTTCAATACT ATTGGAGTGA TTTTTTTTTT TCTAAACAGG 180
GAAAATAATG TTACAAAAGC ATCTTTTTTG TNATTTGTTT GCATCCCTCC CCCACACCCT 240
GGTGTTTTAA ANTGANGAAA AAAANCCATC ACCTTTTGTA CAAAANCTCT TAATGNTTAA 300
CAAACAAACA AANCAGAAA 319

SEQ ID NO:1559
SEQUENCE LENGTH:316
SEQUENCE TYPE:nucleic acid
TOPOLOGY:linear
CLONE:HUMGS01774
SEQUENCE DESCRIPTION:
GATCTGGCTG CGCTATGACT CCGGGAGCGN CACCCACAAC ATGTACCGGG AATACCGGGA 60
CCTGACCACC GCAGCGCTGT CACCCAGTGC TACCGAGACA TGGGTGCCCG GCACGCGCCC 120
GAGCCCACTC CATTCANATC TNACAGCAGT GTTCTTGCAA TATGAGGAGA CAGTTACAGC 180
CACATTATGG CTCTCATTGA ACAGTACGCA GCACCCCTGC CCCCAGCCGT NTTTTTGGGG 240
CTTGCGCGAA AATCTACAAG CGGGAANGTG ACCTNGGATT CAAGGGCGGG GAGAGANGCT 300
TNAATAAATA ATCGTN 316

SEQ ID NO:1560
SEQUENCE LENGTH:390
SEQUENCE TYPE:nucleic acid
TOPOLOGY:linear
CLONE:HUMGS01775
SEQUENCE DESCRIPTION:
GATCTAGAGG GTCGGACCCA CATATTTAAG ATTCACGNTC GTTCAATGAG TGTTGAAAGA 60
GATATCAGAT TTGAACTGTT AGCACGACTG TGNCCAAATA GCACTGGTGC TGAGATTAGA 120
AGCGTCTGCA CAGAGGCTGG TATGTTTGCC ATCAGAGCAC GGCGAAAAAT TGCTACCGAG 180
AAGGATTTCT TGGAAGCTGT AAATAAGGTC ATTAAGTCTT ATGCCAAATT CAGTGCTACT 240
CCTCGTTACA TGACATACAA CTGAACCCTG ANGGCTTTCA AGTGAAAACT TTAAATTGGA 300
ATCCTAACCN TATATAGACT TGTTAATAAC CAATTCATAA ACAAATAAAT GGCTTCAAAA 360
TTGAAAAAAA NNNGANNNNN NNNNAGNNNN 390

SEQ ID NO:1561
SEQUENCE LENGTH:311
SEQUENCE TYPE:nucleic acid
TOPOLOGY:linear
CLONE:HUMGS01776
SEQUENCE DESCRIPTION:
GATCTGCTTA GAGTGGATTT TCACTCAAGT CCTTAGTAAG TGGATTTTGG GGAAAAAAGC 60

ACCTGGGCTT CTGGTTCTTT TTGATAATAT ATAAAATTAT TCATTATGAG GTTGCAGTTG 120
TTTGCAAAGG AGAGGCACTC AAATTTGAAA GGTTATTTTA ATGTGATAAT TTGGAAGACT 180
TACTCAGATG TTGGTCATTG ACCACTCTGT GCATATATTT CTGCAGAGCT CTGTGAAGGC 240
AATGAGTGTC ACTTCCCTCT GCTCTAATAA AGCAATAAAT AATAGCTAAA GGGCTGACTT 300
TCACTTCGAA A 311

SEQ ID NO:1562
SEQUENCE LENGTH:310
SEQUENCE TYPE:nucleic acid
TOPOLOGY:linear
CLONE:HUMGS01777
SEQUENCE DESCRIPTION:
GATCTTTCTA CAACATTTAT CCTGGTTGTT AAGCCCTCCT TACAACATTC TTCTCTCTTT 60
GTTTTTATAG CTCCATCTCT CCTGCTTCTT TAACTTGATA ATGCATACTT GATTTTCCTA 120
TTTGTNATTT CATAAACCAA TTAATACACA GATAAAATGA CTGTATATCA AACCATGTTT 180
GTATAGAAAA ANTGGATTTN GGATGCCTCT CATATGTAAT TAGTTCTATT AAACATATTA 240
ATNGTATTGT TTAATTNGTC AGGTTTTTGA CAGANTTTTG TTTACAGGTA ATAAANNTNT 300
TATCTCCAAA 310

SEQ ID NO:1563
SEQUENCE LENGTH:307
SEQUENCE TYPE:nucleic acid
TOPOLOGY:linear
CLONE:HUMGS01778
SEQUENCE DESCRIPTION:
GATCGAAAAA AAAATCCAGA ACCTTGGGAA ACTGTGGACC CTACTGTACC TCAAAAGCTT 60
ATAACAATCA ACCAACAATG GAAACCCATT GAAGAGTTGC AAAATGTCCA AAGGGTGACC 120
AAATGACGAG CCCTCGCCTC TTTCTTCTGA AGAGTACTCT ATAAATCTAG TGGAAACATT 180
TCTGCACAAA CTAGATTCTG GACACCAGTG TGCGGAAATG CTTCTGCTAC ATTTTTAGGG 240
TTTGTCTACA TTTTTNGGGC TCTGGATAAG GAATTAAAGG AGTGCAGCAA TAACTGCACT 300
GTCTAAA 307

SEQ ID NO:1564
SEQUENCE LENGTH:305
SEQUENCE TYPE:nucleic acid
TOPOLOGY:linear
CLONE:HUMGS01779
SEQUENCE DESCRIPTION:
GATCCTGTGG TTGGCCACTG GCCATAGCTG CTGCCCAGCT CTACCCCTCC CAGGGACCTA 60
CCCCTCCCAG GGACCGACCC CTGGCCCAAG CTCCCCTTGC TGGCGGGCGC TGCGTGGGCC 120
CTGCACTTGC TGAGGTTCCC CATCATGGGC AAGGAAGGGA ATTCCCACAG CCCTCCAGTG 180
NACTGAGGGT ACTGGCCTAG CCATGTGGAA TTCCCTACNC TGACTCCTTC CCCAAACCCA 240
GGGAAAANAG CTCTCAATTT TTNATTTTTA ATTTTTGTTT GAAATAAAGT CCTTAGTTAG 300
CNAAA 305

SEQ ID NO:1565
SEQUENCE LENGTH:304
SEQUENCE TYPE:nucleic acid
TOPOLOGY:linear
CLONE:HUMGS01780
SEQUENCE DESCRIPTION:
GATCACAGCT AGAGAATTGA GAATTAACTA TACTACTAGC CATTTTAGGG CACCAAAACT 60
TGGGATTAAA CACTTCCTAC TTCCCACTCC CAACTCCTGA AATGAAGTCT TGCTATCTGT 120
GACTAGTTTT ATTTTTGTGC TTTTAATAGT CCGAGCAGTC TTACCTTGTT TACACATGTA 180
TTGACACCAT TTGCTTCAGG CCATGGAGCA CTGTTTCTCC CNTTTTACTA TTTATAGGAT 240
TCCGTTTTTT CACAAGACTT TTAATAAAAA GAAATTGTAG AAATAAACAC ATTAAAATTT 300
GAAA 304

SEQ ID NO:1566
SEQUENCE LENGTH:304
SEQUENCE TYPE:nucleic acid
TOPOLOGY:linear
CLONE:HUMGS01781
SEQUENCE DESCRIPTION:
GATCTGCTTT CTCGTAATTA TAGATTGGTT TGCATTTTCT AGAAATTTTT AAATAAATGG 60
AAACATATAG TGAGTACTCT TCTTGGGGTG GAAGGAAGTG TGTGTGGCTT TTTTCACTCA 120
AGCATAATTA ATTTGATACT CATCCAGATT ATGCATGTAT CAATAGTTTA TTCCTTTTTT 180
ATTGCAGAGT AGTAGTTANN NNCCTGGGTA TNCACAATTT GTTACCTGTT CATCTATTGA 240
TGGNCAATTG GGGTATTTCC AGTTTTAGCT ATTACAAATA AACCTGCTGT GACATCATGN 300
CAAA 304

SEQ ID NO:1567
SEQUENCE LENGTH:304
SEQUENCE TYPE:nucleic acid
TOPOLOGY:linear
CLONE:HUMGS01782
SEQUENCE DESCRIPTION:
GATCTGCTAT AGCTGTCCAT CAGAGAGAAT ACACGTGGCT ATAACATCTA TAACAAAACG 60
ACGATTCCTC TACAAGAGGC TGTTTCTNAC TGCTAACGTT GGTGTTTCTG GCGTGGGAAG 120
AAATGCACAG GCGTGCATGG CATGCACGTN CAGACAGCTG CATTGTAAGA NTNCTGTCAT 180
GCAGTCTGAA AAGGGAAGAA ACAGGATGGC TTTCTGTAGC CACACCTGTG AGGCGTGATG 240
ATTGTNGTAT TATTAGATTA CTGATTTTCC TTTTCTGAAA ATACATTTGN NTTTTAATCA 300
CAAA 304

SEQ ID NO:1568
SEQUENCE LENGTH:304
SEQUENCE TYPE:nucleic acid
TOPOLOGY:linear
CLONE:HUMGS01783
SEQUENCE DESCRIPTION:

GATCTGCTGT TGCTGAGAGT NTGAGCGTGG ACTTGATGCA GTNATGACAA ATCATTGCTT 60
AGAATTAATG TTTTCAAATG TGCAACTCTA GTTTTTAAAA CAAAATTTGG TTCTTTACAT 120
TCATTATTTC GTTTTTGTTT CCCTTTAGTA TTTAATGGTC TTTGGAGAAA AAATAATAAA 180
ACAGAGTGTT ATATATATAT TTTTTGGTGC AAGATAAGAT TTTCTGTTTT TTGAAATAAG 240
TCTGTAGCAT AAAAGGTTAA ACTATTTTAA GGACANANTA AAAATAGGAG TGTATTTAAA 300
CAAA 304

SEQ ID NO:1569
SEQUENCE LENGTH:303
SEQUENCE TYPE:nucleic acid
TOPOLOGY:linear
CLONE:HUMGS01784
SEQUENCE DESCRIPTION:
GATCCAGGGA TGAGGATAGA GTGGCCTGAG AGCAGTGCTT GGATTCAGCC TCCTGCTGGG 60
TCCTTCTGCT GGATACAGGC ACCAAGAGGC GGGGGTGGAG CAGGGAGCTG CGCCTTCCTG 120
GGGTGCCNGG TGGTGTGTAG AGAAAAGCTG CTTGTTTACT CCTTAAGTCA ATGTATTGGT 180
GACTGTTGAT TTGTTGAACA ATTCAGGAAT CAAGGGCTGT GGAGAAACTC CCTCATGTTG 240
TTGGCAACAG GTGAATGAAC CTAGAGCGGT GACATGAAAA TAAAGCTCAC TGTTACTCGC 300
AAA 303

SEQ ID NO:1570
SEQUENCE LENGTH:302
SEQUENCE TYPE:nucleic acid
TOPOLOGY:linear
CLONE:HUMGS01785
SEQUENCE DESCRIPTION:
GATCCCCCGA CGACGTGCTC GAGTTCCTGA AGGTGTATGA GANGCACTCT GCCCAGTGAG 60
CACCTGCCCT GCCTGCATCC GGAGAATTGC CTCTACCTGG ACCTTTTGTC TCACACAGCA 120
GTACCCTGAC CTGCTGTGCA CCTTACATTC CTAGAGAGCA GAAATAAAAA GCATGACTAT 180
TTCCACCATC AAATGCTGTA GAATGCTTGG CACTCCCTAA CCAAATGCTG TCTCCATAAT 240
GCCACTGGTG TTAAGATATA TTTTGAGTGG ATGGAGGAGA AATAAACTTA TTCCTCCTTA 300
AA 302

SEQ ID NO:1571
SEQUENCE LENGTH:307
SEQUENCE TYPE:nucleic acid
TOPOLOGY:linear
CLONE:HUMGS01786
SEQUENCE DESCRIPTION:
GATCCATTTT GCTTACACTG TTGCATCACA AGGGACTCAC CCAGGGACCA TGACCTGCTG 60
GTGTGTGTGT ATATTTACAA AAACAAAACA AACAAACCAC CCATTGGGAT ATAAGGTAGC 120
AATCACAAAC TAAAGACTGC GGCTTGTTGA GGTGCAATAC CCTGACTCCC AAAGTTAGTT 180
ACAGTGGGTT TTATTGTTTT TGTGACTGAA GGATTTATTC AGACTGCTGT ACTCTTCATT 240
TGATGTAACA AAATGCTATT AATCTAAATA TTTGTAAATA AAGTACCTGT ATCTAGATTA 300
AATTAAA 307

SEQ ID NO:1572
SEQUENCE LENGTH:301
SEQUENCE TYPE:nucleic acid
TOPOLOGY:linear
CLONE:HUMGS01787
SEQUENCE DESCRIPTION:
GATCCATTAC CTAGGGTAAA ATTCTCCTGA ATGTCAAACA AAGAGATAAA CTACATTTGG 60
GTTTTGGGAA GTCCCCTGTA ATGATGAATC AAGAATCCTC AAGTCTGTCT TGCCACCCAT 120
TTAATACGTA TTTTTGTTAA GGCTGAAGTT TAGAGTTAGA ATCAGGACAT TTTGGCCTAT 180
TGAGAGGTTC TGAATTCCAA CAGAAGATGC CATGTAAATC AGTGAATTTN ATTCTTTTAA 240
AAGCAACTTA CAAAATTTAC AAGATGTATT CAATAAAGCA GTTTAGCTTT GGTATATCAA 300
A 301

SEQ ID NO:1573
SEQUENCE LENGTH:303
SEQUENCE TYPE:nucleic acid
TOPOLOGY:linear
CLONE:HUMGS01788
SEQUENCE DESCRIPTION:
GATCCTCAGT GTGCTCACGG GGCTGCTGTT CGGCAGCNAT GGCTACTACG TGGCGCTGGC 60
CTGGACCTCA TCGGCGCTCA TGTACTTNAT TGTGCGCTCT TTGCGGACAG CAGCCCTGGG 120
CCCCGACAGC ATGGGGGGCC CCGTCCCCCG GNAGCGTCTC CAGCTCTACC TGACTCTGGG 180
AGCTGCAGCC TTTNCAGCCC CTCATNATAT ACTGGCTGAC TTTCCACCTG GTCCGGTGAC 240
GTCTNGCCCC AGATGGNAAT NAGTTTTTTA ATTTCATTGN AAGGATTTTG AANTTCCTTG 300
AAA 303

SEQ ID NO:1574
SEQUENCE LENGTH:297
SEQUENCE TYPE:nucleic acid
TOPOLOGY:linear
CLONE:HUMGS01789
SEQUENCE DESCRIPTION:
GATCTGTGTT CGAATCCTCC CCACCCCTTT CTTTGTGGAG TTTCCTAACC TGCTGCTGAA 60
GCACAATGTT TTGGTGCTTT CTTTTCTCNN TTGTTAAAGG CAGTGTCCAA AAGCCATTCC 120
AGATGCCAAG ACCAGGGGCT TATTTCTAGG GAAGGTAGGT CGGTTTCCAT GTTTCCCTCC 180
CGTTATTTTT ATTTTTNACT TTTTGCCTGA GACAAGCCGA GTATGAGGTG GTTTGATTTA 240
AGAAAAATCA ATGAAATTGT TTACTACTGT TTTAAAATAA AACCGTAAAC TCTGAAA 297

SEQ ID NO:1575
SEQUENCE LENGTH:299
SEQUENCE TYPE:nucleic acid
TOPOLOGY:linear
CLONE:HUMGS01790
SEQUENCE DESCRIPTION:

GATCAGGCCG TTNCTGAGCT CAACGGGACC CAGGTGGAGT CTGTACAGCT CAAAGTCAAC 60
ATAGCCCGAA AACAGCCCAT GCTGGATGCC GCTACTGGCA AGTTTGTNTG GGGCTCCCTC 120
GCTGTCCAGA ACAGCCCTAA GGNTTGCCAC CGGGACAAGA GGACCCAGAT TGTTTACAGT 180
GATGACGTCT ACAAGGAAAA CCTTGTGGAT GGCTTCTAGG GAACAGNGCT GGATTCCTTG 240
TGCCTCATAT GCCCCAAATG CTGGTTTTCA GTAAANCACT TGAGGGTGGA AGCTTNAAA 299

SEQ ID NO:1576
SEQUENCE LENGTH:300
SEQUENCE TYPE:nucleic acid
TOPOLOGY:linear
CLONE:HUMGS01791
SEQUENCE DESCRIPTION:
GATCCAAGAT AAAGAAGGCA TCCCCCCCGA CCAGCAGAGG CTCATCTTTG CAGGCAAGCA 60
GCTGGAAGAT GGCCGCACTC TTTCTGACTA CAACATCCAG AAAGAGTCGA CCCTGCACCT 120
GGTCCTGCGC CTGAGGNGGT GGCTGTTAAT TCTNCAGTCA TGGCATTCGC AGTGCCCAGT 180
GATGGCATTA CTCTGCACTA TAGCCATTTG NCCCAACTTA AGTTTAGAAA TTACAGGTTT 240
CAGTAATAGC TNGAACCTGT TCAAAATGTT AATANAGGTT TCGTTGNATG GGAGCATAAA 300

SEQ ID NO:1577
SEQUENCE LENGTH:295
SEQUENCE TYPE:nucleic acid
TOPOLOGY:linear
CLONE:HUMGS01792
SEQUENCE DESCRIPTION:
GATCTGGCTT CTTTCCCAAG GNGGGGGTGG GGTGTNCCTN GCGTCCCTGT CCTTGANGGA 60
CCTCCTTCCC CCAGCCTCAT CACCGTGCTC TTCTCAGCGC CACCCTCAGC AGCCAGATTG 120
CAACACCAGG GAGAGGCGGA TGCAGAGCCC CACCGGTGGG AAAGTTGCCT GTGGAAGGGA 180
GCCTTTTGCT ACAATTTGTA ACTTATTTNC TAAAGTCTAT TTTGTAACAA TTTATTTAAG 240
TTTAAAAAAA GGAAAACTGC TGCCCNCCAA AAAAAGAAAT TTTCAAAACA AGAAA 295

SEQ ID NO:1578
SEQUENCE LENGTH:297
SEQUENCE TYPE:nucleic acid
TOPOLOGY:linear
CLONE:HUMGS01793
SEQUENCE DESCRIPTION:
GATCTAAAGT TCTGGCTGTC CATTAACCTC CAACTATGGT CTTTATTTCT TGTGGTAATA 60
TGATGTGCCT TTCCTTGCCT AAATCCCTTC CTGGTGTGTA TCAACATTAT TTAATGTCTT 120
CTAATTCAGT CATTTTTTTA TAAGTATGTC TATAAACATT GAACTTTAAA AAACTTATTT 180
ATTTATTCCA CTACTGTAGC AATTGACAGA TTAAAAAAAT GTAACTTCAT AATTTCTTAC 240
CATAACCTCA ATGNCTNNTT TTAAAANATA AAAATTAAAA ATGAAAAGNG NTANAAA 297

SEQ ID NO:1579
SEQUENCE LENGTH:289
SEQUENCE TYPE:nucleic acid

TOPOLOGY:linear
CLONE:HUMGS01794
SEQUENCE DESCRIPTION:
GATCTCTCTT GTCCNCTNCT GCTCTTTNCT TGGTGCTCTT TTTNCTCGGT GGGGTGTGGG 60
TAATAGAACA GCCGTGGGCT TTTGGGGACC TTTAACTTTT TTTNCTCTCT TTTGTTTATA 120
AAAAACACTA AACATTCAAT TCCAGAGAAC CAAAAATCCC ACCTTCCCAC CGAACACTAC 180
TAAGGGGCTT GTGTTCTGCN CCATACCTTT NCTCTTTNCT TTCTGTCTTG TTAATGCTTT 240
TAAAAACAAA TGAGTTTTTT ATATAAATAA AGTTTTTAAA GTGTGTAAA 289

SEQ ID NO:1580
SEQUENCE LENGTH:287
SEQUENCE TYPE:nucleic acid
TOPOLOGY:linear
CLONE:HUMGS01795
SEQUENCE DESCRIPTION:
GATCCCATCC GCATGGAGTT TGAAAATAAG GAAGACTTGT CGGGAACACA GGCAGGGCTC 60
AACGTCATTA AAGAGGCAGA GGCGCACNCC NGGTGGGCAG CCAAGGAGCT GAGAAGANCG 120
AAGAAGCTTT CAGACTACGT GGGGAAGANT GAAAAANCCA AAATTATCGC CAAGNTTCAG 180
CAAAGGGGAC AGGGAGCTCC AGCCCGAGAG CCTATTATTA GCAGTNAGGA GCAGAAGCAG 240
CTGATGCTGT ACTATCACAG AAGNCANGAG GNGCTCANGA GATTGGN 287

SEQ ID NO:1581
SEQUENCE LENGTH:285
SEQUENCE TYPE:nucleic acid
TOPOLOGY:linear
CLONE:HUMGS01796
SEQUENCE DESCRIPTION:
GATCTATTGG CATATTCGGG AGCTTCTTAG AGGGATGAGG TTCTTTGAAC ACAGTGAAAA 60
TTTAAATTAG TAACTTTTTT GCAAGCAGTT TATTGACTGT TATTGCTAAG AAGAAGTAAG 120
AAAGAAAAAG CCTGTTGGCA ATCTTGGTTA TTTCTTTAAG ATTTCTGGCA GTGTGGGATG 180
GATGAATGAA GTGGAATGTG AACTTTGGGC AAGTTAAATG GGACAGCCTT CCATGTTCAT 240
TTGTCTACCT CTTAACTGAA TAAAAAAGCC TACAGTTTTT AGAAA 285

SEQ ID NO:1582
SEQUENCE LENGTH:280
SEQUENCE TYPE:nucleic acid
TOPOLOGY:linear
CLONE:HUMGS01797
SEQUENCE DESCRIPTION:
GATCCTCCCC TTTTGAAATG GCCCTGCTGT GTCAGTTTCC CTGTGGCCTT TTGAACTGTA 60
CATCTCACAT GTTGGGAAAC GCTGGCCACT GGGAAATCAT TAGAAAGGAG GCTGTAGAAT 120
ATTTGCCGAG CCTCTACTGT ATACCAGGGG CTAACTCACC AAGCACATTC TAGGAATTGG 180
GCCCTGCTCA TGAGGAGCCT TAGTGGAGAT TCCAGGTGAA TATTTATGAA AAAGTCAACA 240
TTAGAACTGA AAATGGAAAT AAACTGCTTG AAAAGACAAA 280

SEQ ID NO:1583
SEQUENCE LENGTH:273
SEQUENCE TYPE:nucleic acid
TOPOLOGY:linear
CLONE:HUMGS01798
SEQUENCE DESCRIPTION:
GATCAGAATG GACTTCTNCC ACTGAGCATG ACANGCACTC GAAAGGCTAA ATCAACCAGA 60
AACATCGAAA CAAAAGCTCA GGTTGTCCCC CCGGCACGCT CCACANCTGG TGACCCGACA 120
GTTCCTGGCT CCTTGTTCAG ACAGCTTGTC AGTNAAGAAG ACANCACGTC TGCACCTNCA 180
TTATTCAAAC TTGGCTGGCT CTCTAGTATG ACTACGNACA TGGAACACAT CTGTCTCTCC 240
CTAAATAANT ACTACCACAT TATTNCTTCT AAA 273

SEQ ID NO:1584
SEQUENCE LENGTH:270
SEQUENCE TYPE:nucleic acid
TOPOLOGY:linear
CLONE:HUMGS01799
SEQUENCE DESCRIPTION:
GATCCATATT TGTTTTGTGT TCTGCTTAAA TCAGCAAGAA TGATAAATTT GATGGTGTGA 60
AATTGGAAGT ATCAAGGGCT TTCTTTGGTG ATTGAGGGAA ATAATGTCTC TACTTGTAAT 120
TTATTGTGAC CCTTTTTCAC TGTATATGCT TTGTATGTCT AATATTTATT TCAATGCAAA 180
TTCAATTNNC CCTTCATCTG TATTGTTATA TCTAAGATTT TATTGATGTT AAAATCTAAT 240
TGTGGAATAA AAATCTCTCT GGAATTTAAA 270

SEQ ID NO:1585
SEQUENCE LENGTH:270
SEQUENCE TYPE:nucleic acid
TOPOLOGY:linear
CLONE:HUMGS01800
SEQUENCE DESCRIPTION:
GATCTGCTTG ACAGTGATTA AATCCTTAGC TCACATCCAT TCCCATCTTT NGGGCTCCTT 60
AGGCCCAAGG ATGGCATGTG ACTGGTCCCT GCAAGGTCCT TTCTTTGTCA CCAGCCAAGG 120
CATTGATAAC CAAGTAGCCA TTTTCCTCTT AAGGTTTCCT CTACAACCCC AAGGACTTTC 180
ATGATTATCC TCAGGGACAG GATTGGAGGC ATTGAGCGTG TTTATTAACA AATTGTTTTT 240
GGTAATAAAA TAAATGCTTG GACTCTTAAA 270

SEQ ID NO:1586
SEQUENCE LENGTH:269
SEQUENCE TYPE:nucleic acid
TOPOLOGY:linear
CLONE:HUMGS01801
SEQUENCE DESCRIPTION:
GATCTNGTAA ACTCTCTGTA TATCTTCCTA CCTTTCAAAA TCGTTCTTAG GGTTAGTCAA 60
GTCTGGAATA TAATTGCTGA CTATAAAGTT AGCAATTATG CTTTTAAGGT GTTGTCACAT 120
CAACCTAAAG AGAACCATCT ATGGAAGGTA TGGTTGAAAC ATCTGTAGGA ACACAGAACT 180

GGGATTTCAC TGAGTTTACC ANNTCAACTG TGTGAACTGT TTCTGCACTG CTTGCTAATG 240
GCTTCATCTA ATAAATGTTT ACTTATAAA 269

SEQ ID NO:1587
SEQUENCE LENGTH:267
SEQUENCE TYPE:nucleic acid
TOPOLOGY:linear
CLONE:HUMGS01802
SEQUENCE DESCRIPTION:
GATCTAGGGA AGTCAACACA TAGTGTAAAA TAATTACATG CCTAATGAAA AGAAGATGTC 60
ATTTCCTAAT TTTNATATCT CATTTTCGGC ATTTTTTAAA ATGTAAAAGG AAAACCTCTT 120
GTGTTCACAC AGATTGCTGA ATTGATTTCT CCATATTTGT TAATAATTTA CTATTATTTA 180
CAAAGATTCA AATGCTTTTA TGACTAATGT AAAATGAAAA GAGGCTTACA TTTTAAATGT 240
TATTAAAATT ATGTACTTAA ATCTAAA 267

SEQ ID NO:1588
SEQUENCE LENGTH:288
SEQUENCE TYPE:nucleic acid
TOPOLOGY:linear
CLONE:HUMGS01803
SEQUENCE DESCRIPTION:
GATCGCTTTG AGGTCAATAT TAGTGAGCTG CCTGATGAGA TAGACATCTC CTCCTACATT 60
GAACAGACAC GGTAGAAGAC TCGCCCATTT TGGAATGTGA CCGTCTNTCC TTCAGGNGAG 120
GACACCAGGN TGGGGGTGAA GGAGACACTA CTGCCCCCAC CCCTGACAGC CCCCACCCCA 180
TGGCTTNCAT CTTTTGCATN ACCANCACTC CTGAACCCCN ATTTCTNATT TGTCAGAATT 240
TTTTTNNTTA ACAAAACTAG AAATGNANCA CATGGGGTCT GTGGTAAA 288

SEQ ID NO:1589
SEQUENCE LENGTH:266
SEQUENCE TYPE:nucleic acid
TOPOLOGY:linear
CLONE:HUMGS01804
SEQUENCE DESCRIPTION:
GATCTTGCCT GTTTCACAAT GTAACAAGAC TCTACCTGGG TCCCCTGGTG ATGAGTTTCA 60
GCATAGAATA ATGTTCAAGG AAAAGAAAAC GAAAACAGTT TAAATCTCTA CCACAGCCTC 120
ACAAGCAAAT GCTAAGGGGA ACATACATGT AAAANGCCAG CAAACTATCT TCAAACTCTT 180
CCGTCCTTAA TGTCTTCCAT GGCTATTGCC CCCACAATGG TCTCTTTNNT CCCTGCTCCC 240
TTATTAANGA ACTCTTTCTG AAGAAA 266

SEQ ID NO:1590
SEQUENCE LENGTH:266
SEQUENCE TYPE:nucleic acid
TOPOLOGY:linear
CLONE:HUMGS01805
SEQUENCE DESCRIPTION:

GATCTATGAC TCCAACCGTA TGATTTTNTT GCCTCCCCAG TTTTCATCCC AGTGGTAACG 60
CCTGATTTTT GGTAGCTATG CCATCTTCTG CTGAGGATTA CCATTACTGG GTGTATCACC 120
CCAGTACTTC CAAATNCCTC TCCTCTATTG ATTCAGTTGT TATTGCAGTG TCTGCTTCAT 180
CCGTGGAACT TGAGGCTCAG ATGCCCCCTG CTCTGTAACC CTGGGGAATG TCAGAGGCAG 240
GTAATAAAGG TTGTTTAAAC AATAAA 266

SEQ ID NO:1591
SEQUENCE LENGTH:265
SEQUENCE TYPE:nucleic acid
TOPOLOGY:linear
CLONE:HUMGS01806
SEQUENCE DESCRIPTION:
GATCAGGAGA CTTTGAATTN ATATATTTTA ATCCTNATTA AGCTTAAATA GGAAAGTTTC 60
TTCAACAGGA TTACAGTGTA GCTACCTACA TGCTGAAAAA TATAGCCTTT AAATCATTTT 120
NATATTATAA CTCTGTATAA TAGAGATAAG TCCATTTTTT AAAAATGTTT TCCCCAAACC 180
ATAAAACCCT ATACAAGTTG TTCTAGTAAC AATACATGAG ANAGATGTCT ATGTAGCTGA 240
AAATAAAATG ACGTCACAGG GCAAA 265

SEQ ID NO:1592
SEQUENCE LENGTH:254
SEQUENCE TYPE:nucleic acid
TOPOLOGY:linear
CLONE:HUMGS01807
SEQUENCE DESCRIPTION:
GATCTGAGAG AAGCCCTGTC CTCCACTCAC CTCACCCGCC GCTGCCACCA TCTCCTCTGT 60
GCCAACTCCT TGTGGACCGC AAGAAAGCAT GACTTTGAAA AAGGGAAGCC ATTCCGAGAT 120
TTTAAAATGT TCATGGACTA TTCCATATTA AAAGCTGTTT TTGTTGTACA AAATTCACTG 180
ATGTTCAGTT CTATTTTATT TTGCCTTCAG AAAAGAAGAA AGTCAAAAAT AAAACTTTTG 240
TGTATTACAG CAAA 254

SEQ ID NO:1593
SEQUENCE LENGTH:255
SEQUENCE TYPE:nucleic acid
TOPOLOGY:linear
CLONE:HUMGS01808
SEQUENCE DESCRIPTION:
GATCCTGGAG GATTTCCTAC CCCCGTCCTC TTCGAGNCCC CAGTCGTGAT GTGGAGGAAG 60
AGCCACCTGC AAGATGGACA CGAGCCACAA GCTGCACTGT GAACCTGGGC ACTCCGCGCC 120
GATGCCACCG GCCTGTGGGT CTCTGAAGGG ACCCCCCCCC AATCGGACTG CCAAATTCTC 180
CGGTTTGCCC CGGGATATAA TAGAAAATTA TTTNTATGAA TAATGNAAAT AAAACACACC 240
TNGTGGCATG GCAAA 255

SEQ ID NO:1594
SEQUENCE LENGTH:282
SEQUENCE TYPE:nucleic acid

TOPOLOGY:linear
CLONE:HUMGS01809
SEQUENCE DESCRIPTION:
GATCCATGAA GGAGGGCTGT GTGGAGAAGA TTGGGGACTG GCTGAGGAAA AATGTGCTGG 60
TGGTAGCTGC AGCAGCCCTN GNGAATTNCT TTTGTCGAGG TTTTGGGAAT TGTCTTTNCC 120
TGCTGCCTCG TGAAGAGTAT CAGAAGTGGC TACGAGGTGA TGTAGGGGTC TNGGCTCCTC 180
AGCCTNCTCA TCTGGGGGAG TGGAATAGTA TCCTCCAGGT TTTTCAATTA AACGGATTAT 240
TTTTTCAGNC CGAAAAGNGA TGGTCTGAGT TTNTCTTAGA AA 282

SEQ ID NO:1595
SEQUENCE LENGTH:256
SEQUENCE TYPE:nucleic acid
TOPOLOGY:linear
CLONE:HUMGS01810
SEQUENCE DESCRIPTION:
GATCAAATAA ACCAGTGTAG GATATTAAGA CTATCAATTG GTGGGATTAG GCTTGACATT 60
TTATTTTTAT AAATATATAT TTNTCCTGAA TGTGTCTGAG TCCAAGAGTG GGCAAAAAAT 120
AATTTNCTAC TTTGGACTAA TCTATAGAGG TTTTTGAAAG TCTGCATTAC TAACTTGTTG 180
AATTCATGAT ATTCTGCCTA TGGCACAAAT TGTAAACCTT TGTTTTNCTA AAATAANGTA 240
ATTGAAAACC TGNAAA 256

SEQ ID NO:1596
SEQUENCE LENGTH:204
SEQUENCE TYPE:nucleic acid
TOPOLOGY:linear
CLONE:HUMGS01811
SEQUENCE DESCRIPTION:
GATCTGCGCT TCCAGAGCGC ACTAATNCGG TGCTTTGCAG GAGGCAAGTG AGGCCTATCT 60
GGTTGGCCTT TTTGAAGACA CCAACCTGTG TGCTATCCAT GCCAAACGTG TAACAATTAT 120
GCCAAAAGAC ATCCAGCTAG CACGCCGCAT ACGTGGAGAA CGTGCTTAAG AATCCACTAT 180
GATGGGAAAC ATTTCATTCT CAAA 204

SEQ ID NO:1597
SEQUENCE LENGTH:246
SEQUENCE TYPE:nucleic acid
TOPOLOGY:linear
CLONE:HUMGS01812
SEQUENCE DESCRIPTION:
GATCATTATT ATATATAGGT ATTGATTGCT ACCCTGACCA CAGTGCTTTG GACTATGAGA 60
AACTTCTTAG ATTTTNATAT GTAAATNCTG TGGACCACTG GGAGCACAAT GCCCACATCA 120
TCTTAAGNNG AGTTTATGTG CAGCATTTAA ATCACTGTGT TTTCCTTGTT AACTAAAACA 180
GACATGGGCT TTGATTTTTT TCATACTATT AGACCATATC TCATAAAACC TTTTGAATTA 240
ATGAAA 246

SEQ ID NO:1598

SEQUENCE LENGTH:285
SEQUENCE TYPE:nucleic acid
TOPOLOGY:linear
CLONE:HUMGS01813
SEQUENCE DESCRIPTION:
GATCACGCAC ACACAATAAG TCTTTCTCTC CGAAACCGGA AGTAAATCTA TATCTGTTAG 60
AAATAATGTA GCCAAAAGAA TGTAAATTTG AGGATTTTTT TGCCAATAGT TTATAGAAAA 120
TATATGANCC AAAGTGATTT GAGTTTGTAA AAATGTAAAA TAGTATGANC AAAATTTGCA 180
CTCTACCAGA TTTGAACATC TAGTGAGGTT CACATTCATA CTAAGTTTTC AACATTGTGT 240
TCTTTTTGCA TTCATTTTTT ACTTTTATTA ANGGNTCAAA CCCGN 285

SEQ ID NO:1599
SEQUENCE LENGTH:241
SEQUENCE TYPE:nucleic acid
TOPOLOGY:linear
CLONE:HUMGS01814
SEQUENCE DESCRIPTION:
GATCTAACTA TGTTTCCCCC CTTTCAAGAA TAGAACTATT GGGGTGAGGA TAAGGGGTGG 60
GGGAGAAAAA ATCACTGTTT GTTTTTAAAA AGCAAATCTT TCTGTAAACA GAATAAAAGT 120
NCCTCTCCCT TCCCTTCCCT CACCCCTGAC ATGTACCCCC TTTNCCCTTC TGGCTGTTCC 180
CCTGCTCTGT TGCCTCTCTA AGGTAACATT TATAGAAGAN NTGGAATGAA TCTCCANGAA 240
A 241

SEQ ID NO:1600
SEQUENCE LENGTH:239
SEQUENCE TYPE:nucleic acid
TOPOLOGY:linear
CLONE:HUMGS01815
SEQUENCE DESCRIPTION:
GATCCAGAAT ACCCTGACCT CGCCCCAGTT CCAGCAGATG TGGAAGCGTT TGCCAAAGCC 60
ATGCAGAACA ACGCCAAGCC CGAGCAGAAA GAGGGCGACA CGAAGGACAA GAAGGACGAA 120
GAGGAGGACA TGAGCCTGGA CTGAGCCACG CGCCGTCCTC CGAGGAACTG GGCGCTTGCA 180
GTGCGTTGCA CACCCTCACC TCCCACCCAC TGATTATTAA TAAAGTCTTT TCTTTTAAA 239

SEQ ID NO:1601
SEQUENCE LENGTH:236
SEQUENCE TYPE:nucleic acid
TOPOLOGY:linear
CLONE:HUMGS01816
SEQUENCE DESCRIPTION:
GATCCAAAGT AATATGTAGG TTCAATATTT TGTAGGTTTT ATAATCAAAT GTCTCCCTAA 60
AGAGATGACA TAGGGAAGAA AAGCCTAATG TATCATCTTA ATGGATTCAG TTTTTATTAT 120
GCACAAATAA CTGGGCTTGT AACCTAGGAA TATGATTAGA GCGTGATGCT GGCTTAAAAA 180
ATAGGACTNG TGAATCACAG TTTGTNNNTA ATAAACTACT GGTTGGAAAT ATTAAA 236

SEQ ID NO:1602
SEQUENCE LENGTH:235
SEQUENCE TYPE:nucleic acid
TOPOLOGY:linear
CLONE:HUMGS01817
SEQUENCE DESCRIPTION:
GATCTCCCCG CCGCAGCCTC CTCAGAAGAC ATCGAGCGGT CCTGAGAGCC TCCTGGGCAC 60
GTTTGTCTGT GTGCTGTAAC CTGAAGTCAA ACCTTAAGAT AATGGATAAT CTTCGGCCAA 120
TTTATGCAGA GTCAGCCATT CCTGTTCTCT TTGCCTTGAT GTTGTGTTGT TATCATTTAA 180
GATTTTTTTT TTTTGGNAAT NATTTTGAGT GGCAAANTAA NGATTAGCAN TTAAA 235

SEQ ID NO:1603
SEQUENCE LENGTH:324
SEQUENCE TYPE:nucleic acid
TOPOLOGY:linear
CLONE:HUMGS01818
SEQUENCE DESCRIPTION:
GATCTGCCCA GGTGCGCCAG ACTTCCTAGC ACACGTGCGC GGGAGCAGCT GCTTTGAGTG 60
CACACACTAC CAATCGTTCC TGGAATACAG GGAGGTGGTG GACGGCCTGG AGAAGGCCAT 120
CTACAAGGGC CCAGGCAGCG AAGAGGGCCC TGACTGCCCC CCCCGGCCCC CCTCTCGGGC 180
TCTCTCACCC AGCCTGGTAC TGAAGGTGCC AGACGTGNTC CTGCTGACCT TCTGCGGCTC 240
CGGGCTGTGT CCTAAATGCA AAGCACACCT CGGCCGAGGN CTGCGNCCTG ACANGGNNAC 300
CTCTCTGAAC TGCAACACTT GGAN 324

SEQ ID NO:1604
SEQUENCE LENGTH:235
SEQUENCE TYPE:nucleic acid
TOPOLOGY:linear
CLONE:HUMGS01819
SEQUENCE DESCRIPTION:
GATCAAAAAG CAAGCAGAAA TGAATCCACA TTCTAGTCCT TTATGCAGTA TACAAGGAGA 60
ACTGTCCTGT AGGATATTCT CTTCCTGATG GTGCAGAACC CAGAATTAGA AGTTTGTGGT 120
TACAGCATAC TCTGTCCTTC AGAAAGGCGT GATTCTAGCT GTTGACCCCT TGCAGCTGTT 180
GGAATCTCTG CAAGAACCTC TGTATTCTTC TAATAAATTC CCTCTTTTAT TTAAA 235

SEQ ID NO:1605
SEQUENCE LENGTH:235
SEQUENCE TYPE:nucleic acid
TOPOLOGY:linear
CLONE:HUMGS01820
SEQUENCE DESCRIPTION:
GATCCCAACA ATGAAGCCAT TCGAAATGCT ATGGGCTCCC TGGCCTCCNA GGCCACCAAG 60
GACGGCAAGA AGGACAAGAA GGAGGAAGAC AAGAAGTGAG ACTGGAGGGA AAGGGTAGCT 120
GAGTCTGCTT AGGGGACTGC ATGGNAAGCA CGGAATATAG GGTTAGATGT GTGTNATCTG 180
TAACCATTAC AGCCTAAATA AAGCTTGGCA ACTTTTTTCC CTTTTTTGCT TCAAA 235

SEQ ID NO:1606
SEQUENCE LENGTH:234
SEQUENCE TYPE:nucleic acid
TOPOLOGY:linear
CLONE:HUMGS01821
SEQUENCE DESCRIPTION:
GATCAAAAAG AGAGTGGTTG TGGAACCTGT TGGCCCAGTT GGTTTCAAGC CAGAGACATT 60
CAGAAAGTTT TTAGCTCTAT ATTTGCATGG TGCTGCGTGA NGGAGGACCC CTCTGAATCC 120
TGAAACCCCT CTTGCCTCTC TTCCACGGAA GAGGGCCTGG GCCCCGTGGA GCCTCAGTGC 180
CCGTTTGGCC TGCTGCTCTC GCTGACAATA AAGAGCCCTT GCGTTGCACT GAAA 234

SEQ ID NO:1607
SEQUENCE LENGTH:234
SEQUENCE TYPE:nucleic acid
TOPOLOGY:linear
CLONE:HUMGS01822
SEQUENCE DESCRIPTION:
GATCTGGAAC CTCAGCCTGG CCAGACACAG GCCCTCCCTG TTCCCCAGAG AAAGGGGAGC 60
CCACTGTCCT GGGCCTGCAG AATTTGGGTT CTGCCTGCCA GCTGCACTGA TGCTGCCCCT 120
CATCTCTCTG CCCAACCCTT CCCTCACCTT GGCACCAGAC ACCCAGGACT TATTTAAACT 180
CTGTTGCAAG TGCAATAAAT CTGACCCAGT GCCCCCACTG ACCAGAACTA GAAA 234

SEQ ID NO:1608
SEQUENCE LENGTH:356
SEQUENCE TYPE:nucleic acid
TOPOLOGY:linear
CLONE:HUMGS01823
SEQUENCE DESCRIPTION:
GATCACATAA AACAGATTTG CATAAAATTA CCATGATTGC TTTATGTTTA TATTTAACTT 60
GTATTTTTGT ACAAACAAGA TTGTGTAAGA TATATTTGAA GTTTCAGTGA TTTAACAGTC 120
TTTCCAACTT TTCATGATTT TNATGAGCAC AGACTTTCAA GAAAATACTT GAAAATAAAT 180
TACATTGCCT TTTGTCCATT AATCAGCAAA TAAAACATGG CCTTAACAAA GTTGTTTGTG 240
TTATTGTACA ATTTGAAAAT TATGTCGGGA CATACCCTAT AGAATTACTA ACCTTACTGC 300
CCCTTGTAGA ATATGTATTA ATCATTCTAC ATTAAAGAAA ATAATGGGTT CTTAAA 356

SEQ ID NO:1609
SEQUENCE LENGTH:230
SEQUENCE TYPE:nucleic acid
TOPOLOGY:linear
CLONE:HUMGS01824
SEQUENCE DESCRIPTION:
GATCACCTAT GCCAAGAAAT AACATTTGGG ATAGTCGTCT TTAAAAGACT TGGTGTTATT 60
TACAGTGTTT GTTTTGATAA CATTTGGCTG GGTCATTTTA ATAGTTAGAG ATGAGGAGGA 120
GTAAAAGTGA AATTTTTGTG AAGGACTTAA ATTATCCAGT GTTTCTTTAG CCTTGGTGAA 180

CTATGAAATA CGAAGNCCTT AATTTTGTAC AATAAACTTT TATTTGTAAA 230

SEQ ID NO:1610
SEQUENCE LENGTH:227
SEQUENCE TYPE:nucleic acid
TOPOLOGY:linear
CLONE:HUMGS01825
SEQUENCE DESCRIPTION:
GATCTGTTGA ATATTTGCTA AAGACCAGTT CTTTAAGCTA AGACATGTAA AAAATCCCAA 60
ATGGCAGTAC CTCATTGTTT ACTTAGCTTT TGTACTTATA TTTTTCAGAG GAAAAAACAC 120
TACTGTAAAT TGTGAATAGC CAATACATAA CTGTATTGTA TGCAAATCTG TGATTGTTGG 180
CAGTGTCATC TCTGAGAAAC AGATAAATAA AGTTTATTTA CTATAAA 227

SEQ ID NO:1611
SEQUENCE LENGTH:234
SEQUENCE TYPE:nucleic acid
TOPOLOGY:linear
CLONE:HUMGS01826
SEQUENCE DESCRIPTION:
GATCTAGCCA AGGCTGGTCT GCAGTATCAG ATGTCCAAAC TCATCTACTA TTAGCCATAT 60
TTTGTGAGTC GTTTGTCTAA ACTTTGTCAA AAATGCCTTT GCCATGATTT TGTTGCTATC 120
TGGATTTCAA ACATGGACAG TTAGGAAGAT GTGCATTGAA GTAGGAAAAT TTTGTTCAGA 180
TTTGCTGTTA TTTATTTTTT AAATTAAAAA TGGAAATGTA TTTTAAAGTT TAAA 234

SEQ ID NO:1612
SEQUENCE LENGTH:226
SEQUENCE TYPE:nucleic acid
TOPOLOGY:linear
CLONE:HUMGS01827
SEQUENCE DESCRIPTION:
GATCTGTTGG GTTCTCTATT AATTTATTTC ATACATTATA GATTCTGGGT TGATAAATTC 60
TGCTCAATTT GAAAAATTTT ATATTTTGTC TCAAATCTGT TACAATTTAT ATTGGTGGGN 120
GCAAAAGTAA TTGCGGTTTT TGCCGTAGAC AGTAATGGCA AAAACTGACA ATTGCTTTTG 180
CACCACCCTA ATAATATTAA NTATAATCAT GACTTTAAAC ATCAAA 226

SEQ ID NO:1613
SEQUENCE LENGTH:224
SEQUENCE TYPE:nucleic acid
TOPOLOGY:linear
CLONE:HUMGS01828
SEQUENCE DESCRIPTION:
GATCGGGAAC TGGCTCCGTT GTGCTGAGGT CATCTTTGGT CATCAGCCTC CAGCATNTGG 60
AAACACCTCC AACGCAATGT GGCTTTTACA TTTCTTTCTT TCTTTCTTTT TTTTTCCTGG 120
TACTGGGAAT ACACAACACC AGCTGTTTTA TTATTATTTG GGGAGGGGGT TGTGATTTTA 180
TTATTNGTTT TTTTAAAATG AAAAATAAAA NGTTATATAT TAAA 224

SEQ ID NO:1614
SEQUENCE LENGTH:222
SEQUENCE TYPE:nucleic acid
TOPOLOGY:linear
CLONE:HUMGS01829
SEQUENCE DESCRIPTION:
GATCAGCAGA TACATGTCAG TGCAGAGGTT CTCTGCCCTC CAGAAGCCCT CGTCTGGGAA 60
TTCGAGAGAG GAGGAGGACC CAGGGCCAAA GGAATCCTCA AACTTCCCTC CAAGGGAGAT 120
ATTGTAAGGG GAAAACAAAG TGTTTATTTC CCCAAAACAA CCCAGCTTCA CCCCATGTCC 180
CTTCTACACG CTTACTCACT GAGGGTGAGT GGGAACGNTA AA 222

SEQ ID NO:1615
SEQUENCE LENGTH:222
SEQUENCE TYPE:nucleic acid
TOPOLOGY:linear
CLONE:HUMGS01830
SEQUENCE DESCRIPTION:
GATCAGAATA TGTATGCAGT TCTTTCTTTA ACCCTTTTTT CTCCTATGTA CTGTGAACAT 60
TTAAAAAGTA CTTTTAAATA TTCTTTGGAA ACTTAGCTTT TAGTAACTAC TTGGTTGTAT 120
CATAATTNAT TAACTANTGN NTTATTGGTA AACATTGGCT TGCCTCCCAT GACTTGCCAT 180
TATAATTAAA ACTGTGACTA CTATCTGTCT CCATTNCATA AA 222

SEQ ID NO:1616
SEQUENCE LENGTH:219
SEQUENCE TYPE:nucleic acid
TOPOLOGY:linear
CLONE:HUMGS01831
SEQUENCE DESCRIPTION:
GATCCCNTTC CCACCCTCTC TGTTGGCCTC AGAGTCACTC CTGCCCCCTC TCCCTGACTT 60
GGTGCTCACA TGCACCTCAC TAGGGTTTGT GACCAGGGTC TGGATGAGCT TGAATTTGAA 120
TGAATTGAGT TTGTATTTNT AGAACCCTGG GTTTTTACAT GTTTGGTCTT TTTTNGTTTT 180
GGTTTGTCAC CNTCGATAAA GGAAGTATAT TCATCCAAA 219

SEQ ID NO:1617
SEQUENCE LENGTH:220
SEQUENCE TYPE:nucleic acid
TOPOLOGY:linear
CLONE:HUMGS01832
SEQUENCE DESCRIPTION:
GATCGCGGCG GANTNGGCCC CGCCGCCTGG TACTCAAGCC CGCGGGGACA TTGGGAAGGG 60
GACCCCCGCC CCCTGCCCTC CCCTCTCTGC ACCGTACTGT GGAAAAGAAA CACGCACTTA 120
GTCTCTAAAG AGTTTATTTT AAGACGTGTT TGTGTTTGTN TGTGTTTGTN CTTTTNATTG 180
AATCTATTTA AGTAAAAAAA AAAATTGGTT CTTTATTAAA 220

SEQ ID NO:1618
SEQUENCE LENGTH:216
SEQUENCE TYPE:nucleic acid
TOPOLOGY:linear
CLONE:HUMGS01833
SEQUENCE DESCRIPTION:
GATCANGGCA CTTCAACTCA TTTGGAAAAT NTGAACACTG ATGACATGGT ATAGGAGTGG 60
GNGGGGTGTT GAGCCACCCA TCANACCCTC TTTAGCTGTG CAAGATAAAA GCAGCCTGGG 120
TCACCCAGGG CCACAAGGCC ATGGTTAATT NTTANCGGCA AGGCAAATCC ATNGTTGNGA 180
AGTGCANTGG GCATAGTAAA NGTGCATGAT TTTAAN 216

SEQ ID NO:1619
SEQUENCE LENGTH:214
SEQUENCE TYPE:nucleic acid
TOPOLOGY:linear
CLONE:HUMGS01834
SEQUENCE DESCRIPTION:
GATCATAGAA AATAAATAGA AGAGACAGTG AAGCAAGTAA AAAGAAAAGC ATTGTTTTAA 60
TTTGTTTGCA TTAATTTTTT TCATTTGTCA AAATGCTTCT TTTGTTGCCA CAGTAAAGAA 120
CAGTTTTTAT TGTTTTGTAA GTAAAATTAC GTAGCTGATT TTGTATGTAA AGATTAATTT 180
CCATAATAAA AATTATTGTA TGTTTACTGT GAAA 214

SEQ ID NO:1620
SEQUENCE LENGTH:214
SEQUENCE TYPE:nucleic acid
TOPOLOGY:linear
CLONE:HUMGS01835
SEQUENCE DESCRIPTION:
GATCAGAGTG GCCCACAGAC ATTGCTTTCT TATCACCTAT CATGTGAATT CTACCTGTAT 60
TCCTGGGCTG GACCACTTGA TAACTTCCAG TGTCCTGGCA GCTTTTGGAA TNACAGCAGT 120
GGTATGGGGT TTATNATGCT ATAAAACAAT GTCTGAAAAG TTGCCTAGAA TATATTTTGT 180
TACAAACTTG AAATAAACCA AATTTGATGT TAAA 214

SEQ ID NO:1621
SEQUENCE LENGTH:211
SEQUENCE TYPE:nucleic acid
TOPOLOGY:linear
CLONE:HUMGS01836
SEQUENCE DESCRIPTION:
GATCTGTGTC ATAAGTGACT CCGGATGCAT CAGTGTCCAC CAGTTGGAAG CAATGACAAG 60
GATGGCTGGC TGGTGTTTTT CAGCCTTCCG GTTTATAGAC TGTATTTATC TAGTGGATTC 120
CTGCAGGCCC CATACTGAGC CTGGACTGAA AGTATCCACT CGGACCATCT GTTATCTCTC 180
TACACTGAAA ATAAAACCTC TTCCACCCAA A 211

SEQ ID NO:1622

SEQUENCE LENGTH:211
SEQUENCE TYPE:nucleic acid
TOPOLOGY:linear
CLONE:HUMGS01837
SEQUENCE DESCRIPTION:
GATCTGAGAT GGAAGAGTTT CATCCCAAAA CCATCTCCCC CTGACCCCCA GTCCATGGAA 60
AAATTGTCTT CCACAAAACC GGTCCCTGGT GCCAAAAAGG TTGGGGAACT GCTGGTCGGT 120
ACAAAAGTAA TTGTGGTTTT TGCACNGTTG GAATTTGTCA TNTGATATTG GAATACATTC 180
TTAAATAAAT GTGGTTATGT TATACATCAA A 211

SEQ ID NO:1623
SEQUENCE LENGTH:212
SEQUENCE TYPE:nucleic acid
TOPOLOGY:linear
CLONE:HUMGS01838
SEQUENCE DESCRIPTION:
GATCAGCGAG GTTCTCCAGG ACCTTAGGTT TGATGCGGAA TCTGCCGAGT GATGGCGGCT 60
CCCCAGGGAT GCCGCCGAGG GAGATGGGAA ACGGGGCGGA TGGCGCCCAG CCCAGCCCTA 120
ACTGCCAGCT GGCTGGGGTC GCGCCCCACT GCGCTGCTGA CCTTCCTGCA GTTCCAGACA 180
CCTCCCACAA TAAAGAGCTC CTCCTCTGTA AA 212

SEQ ID NO:1624
SEQUENCE LENGTH:208
SEQUENCE TYPE:nucleic acid
TOPOLOGY:linear
CLONE:HUMGS01839
SEQUENCE DESCRIPTION:
GATCTATGAC AATGTATAGT CAATAAATGA TAGCAATTAT TTTTAATTAT CAAAATAAAT 60
TGTTAGAGAT TCAAGTTAGC ATACAAAATG GAAATACATT GACAATCTAA AAACTATACT 120
AAAACATAAA CTCAAGACTT TATTCCTCTT CCTGTTCTAT CAAAATTGTG CCAAGTAAAA 180
AANTAAAANT TGTTCTTTTT ATTTCAAA 208

SEQ ID NO:1625
SEQUENCE LENGTH:210
SEQUENCE TYPE:nucleic acid
TOPOLOGY:linear
CLONE:HUMGS01840
SEQUENCE DESCRIPTION:
GATCTAGATG GCAGATAGGA ACTTTNTTGT CACAAAAATA CTGGAGGAAA ATNTTGTAAA 60
AATAGACTTT TGGACACACA GCTGTTGGGG CTGCACTGAG CTGCAATTTT TAACATGGAT 120
TTATAACTTA ATGTTTCTGT TTATAAAATA CTAATGATTT GCAATGTATT TTACTGGCCA 180
ATTAAAACAG ATGTTTTATT CTTTCTGAAA 210

SEQ ID NO:1626
SEQUENCE LENGTH:208

SEQUENCE TYPE:nucleic acid
TOPOLOGY:linear
CLONE:HUMGS01841
SEQUENCE DESCRIPTION:
GATCCAGGAT TCTGGGAGGC TTCAGTTACC GCTGGCCGAG CTGAAGAACT GGGTATGAGG 60
CTGGGNCGGG GCTGGAGGTG GCGCCCCCTG GTGGGACAAC AAAGAGGACA CCATTTTTCC 120
AGAGCTGCAG AGAGCACCTG GTGGGGAGGA AGAAGTGTAA CTCACCAGCC TCTGCTCTTA 180
TCTTTGTAAT AAATGTTAAA GCCAGAAA 208

SEQ ID NO:1627
SEQUENCE LENGTH:203
SEQUENCE TYPE:nucleic acid
TOPOLOGY:linear
CLONE:HUMGS01842
SEQUENCE DESCRIPTION:
GATCTCACAG CCACGAATTT AGAACTTCCT CCTATAGCAT TTTCTATCTT TTTTATGGCA 60
TTTATTTTAT TCTATCTTGT TATGATTACA TAATCTGTTT AATCCCCAAT TATATATTGT 120
AAATACCTTG AAGGAAAGGA GTCTTTATTA TCTTTGGTGC CTGGTGTTTA GTTTTAATAT 180
TTTATTAAAT ACATCCAAGG AAA 203

SEQ ID NO:1628
SEQUENCE LENGTH:203
SEQUENCE TYPE:nucleic acid
TOPOLOGY:linear
CLONE:HUMGS01843
SEQUENCE DESCRIPTION:
GATCCACTGT TCAATCTGCA CAAGATTNGG GNNCCAGACA TGGGAGACTT CAGCTGCCTC 60
AGAGGACCGT GGACAGGGAA GGCAGCCTCG CATCCCTCTG TCCATGCCTG GNNTGACTTT 120
AATAACCAAG AGTTTTATTT TTGAATTTGT AGCTGTCGTT CACTTTTTAC CCACCCATTC 180
AATAAACCTT ACAGAATTGC AAA 203

SEQ ID NO:1629
SEQUENCE LENGTH:201
SEQUENCE TYPE:nucleic acid
TOPOLOGY:linear
CLONE:HUMGS01844
SEQUENCE DESCRIPTION:
GATCCAAGTA ACAGTGACTG CAGTTAGGGT CGAGAGCTTC TCCGAAGCAG CGGTCATGCA 60
GGCTTTTGTG TGGCTGACGC TTCCCTTTTT CACAACAGTA AAAAGCTATC AATGGGAAAT 120
TGTGGACTTT TCCCACCAGG GACTCAATGG GGACTCAATG TGCAATATTC AGTCAACATA 180
AAACTCTTTA AGAACTCCAA A 201

SEQ ID NO:1630
SEQUENCE LENGTH:201
SEQUENCE TYPE:nucleic acid

TOPOLOGY:linear
CLONE:HUMGS01845
SEQUENCE DESCRIPTION:
GATCACAGAC CACGAGTGCC TTTCCCGGAC CTGGACGTTG CCTCCAGAGC AGGCACCANC 60
TCTTTCCCTC TCTAGACAGA AATATTTTTG TAAGGTTCTG GGGCAGGGAG GGAGCATGAA 120
GTACGAGGAA AACTTGAATT CCAGATTTTN AATGCAAAGT ATTTATCATT TCTACCAGAA 180
ATAAACGTTT TAAGTTTTAA A 201

SEQ ID NO:1631
SEQUENCE LENGTH:217
SEQUENCE TYPE:nucleic acid
TOPOLOGY:linear
CLONE:HUMGS01846
SEQUENCE DESCRIPTION:
GATCTTGAGG TCACTTCGTT TAGGTCATTN TCTCAACCCA ACCTCCCCAA ACTCTGGTTT 60
GAGCCAATAT GTGTTCTATT GTTCTCAGAG CACCAGCCGA CTGTACAACA ATTGTTATAA 120
AAATGTTTAT TGTTTACCAA AACCAGTGGA CCTCTTATCA AATGCTGCTT GGTAACAAAA 180
TCTATCACAG TTTTAATAAA AAGAAAAAAA AAGGAAA 217

SEQ ID NO:1632
SEQUENCE LENGTH:200
SEQUENCE TYPE:nucleic acid
TOPOLOGY:linear
CLONE:HUMGS01847
SEQUENCE DESCRIPTION:
GATCTGTTTT NNTTAAACAC TAACAGAATA ATTCTTTATA AATAGGTAAG CCTTACACTT 60
GTTAAAGAAA TTTACCTCTA ATTTCAGTCT CACTAATGTA AAATACTGGG ACTTAAGTAT 120
ACAATTCAGT CACTAACTGT ACAGTTTTAT GTGGGGAACA ATTCATGCAG GCTACTGGAA 180
AATTAAATCT TATTACCAAA 200

SEQ ID NO:1633
SEQUENCE LENGTH:200
SEQUENCE TYPE:nucleic acid
TOPOLOGY:linear
CLONE:HUMGS01848
SEQUENCE DESCRIPTION:
GATCCNGGGC ATTTNCTCTG GGGAGACAGG CCTGAGGCCA GCTCGGNAGC CAAGCAGATT 60
CCAAACCACT ACAACCCTTG CCAAACACAG CCCACTCCAT TTNANACCGC CTTCCTGAGG 120
AGAAAATGCA GACTCAGGGT TCCAGTAACC AGTGATGGAT TCACCCCATC TCCCAAATAA 180
TGGTTTACTT GTTTTACAAA 200

SEQ ID NO:1634
SEQUENCE LENGTH:333
SEQUENCE TYPE:nucleic acid
TOPOLOGY:linear

CLONE:HUMGS01849
SEQUENCE DESCRIPTION:
GATCTTCTAA GAATTTTGCA AAATTCTAAA CAAATATAGA GATGGTATAT AGAATTCATA 60
TCTAGAAAAC TTTGATTTTA ATGTGAGCTT ATCAAATTTG TNCTGGCTTT TTTGGCACTA 120
AGGCAAAAAC ATGTTAACCA GAAATAATTT ATTCTTCATG TATGTAAAAT ATTTGAGAAT 180
GTTTAGCCTT TTATTAGAAT TTTATTTGGA AAATATTTAT CTTTCTACAC ATTTTACACT 240
TATGTNCCTT TGCTTATAAC CCAATTTCTT AACTTTTTTG TTACTTAAGC AAATATCAAT 300
NATGTTTTAT TATCTAATAA AGTGTAAGAT TCN 333

SEQ ID NO:1635
SEQUENCE LENGTH:199
SEQUENCE TYPE:nucleic acid
TOPOLOGY:linear
CLONE:HUMGS01850
SEQUENCE DESCRIPTION:
GATCGAATGG CTTTTTGCAG CTAACTACTA TGTGTAGACA GGTTTTATAT TATAAAGTAT 60
GCATTCTTAT CACCTAGTAT ATAGTTAGTT TGTAGAGTGA TTTCCCCCCA GTTTCTTGAA 120
CATGGTATCT TCACATCTTG GACCTTGGTC AGTTGTGCTA TTCATTATTA AACACTAAAA 180
CTTTGGCGGT TCTTGCAAA 199

SEQ ID NO:1636
SEQUENCE LENGTH:196
SEQUENCE TYPE:nucleic acid
TOPOLOGY:linear
CLONE:HUMGS01851
SEQUENCE DESCRIPTION:
GATCTGGATG TTGAAAAGAG TCTGGCACCT CCTCCNTCTC TCTTGCTCAC ACTCTCACCA 60
CGTGATATGC TGCCTCCTCC TGTGCCTTCC ACCATGAGTA NAAGCTTCCT TANNCTCTTN 120
TNAGAAGCAG ATGCTGGCAG CATGATTCTT GTACAGTCTG CAGAACAGTG AGCCAAATAA 180
ACCTCTTTTC TCTAAA 196

SEQ ID NO:1637
SEQUENCE LENGTH:196
SEQUENCE TYPE:nucleic acid
TOPOLOGY:linear
CLONE:HUMGS01852
SEQUENCE DESCRIPTION:
GATCCAAGTA TTTCTNCATC AAGCAGTTTT TAAAAGGAAA ACGATAATAA TCAGTAGGCT 60
CCATGGAAGC CTTTNCCTTA ATAGCTATGT GCCAAATACT TTNATCTNGT GTGACAGTCA 120
TGTCAGAGTG AAATCTCTCA GGAAAAGTGT AACTAGTAGT TACAAAGTAA ATAAAGGATT 180
TCATTTTAAG GTGAAA 196

SEQ ID NO:1638
SEQUENCE LENGTH:195
SEQUENCE TYPE:nucleic acid

TOPOLOGY:linear
CLONE:HUMGS01853
SEQUENCE DESCRIPTION:
GATCCTCCTG CAGGGCTAGG CGGATTGTTC TGGATTTCCT TTTGTTTTTC CTTTTAGTTT 60
TCCATCTTTT CCCTCCCTGG TGCTCATTGG AATCTGAGTA GAGTCTGGGG GAGGGTCCCC 120
ACCTTCCTGT ACCTCCTCCC CACAGCTTGC TTTTGTTGTA CCGTCTTTCA ATAAAAAGAA 180
GCTGTTTGGT CTAAA 195

SEQ ID NO:1639
SEQUENCE LENGTH:194
SEQUENCE TYPE:nucleic acid
TOPOLOGY:linear
CLONE:HUMGS01854
SEQUENCE DESCRIPTION:
GATCTTAGCT TTGATAAATG ACTGANGCTG GAGAAGCCGT GGTTGAAGTC AGCCTACACT 60
ACAGTGCACA GTTGAGGAGC CAGAGACTTC TTAAATCATC CTTAGAACCG TGACCATAGC 120
AGTATATATT TCCCTCTTGG AACAAAAAAC TATTTTTCCT GTATTTTTAC CATATAAAGT 180
ATTTAAAAAA CAAA 194

SEQ ID NO:1640
SEQUENCE LENGTH:268
SEQUENCE TYPE:nucleic acid
TOPOLOGY:linear
CLONE:HUMGS01855
SEQUENCE DESCRIPTION:
GATCTGAGTA ATTAGCAGGT ATGATGCTGG GACTGGAAAA TAGAAAGTAA TAACTAAAGG 60
GTTAATGTGC AACGTTATTT TTTGGCCTTG TTCATAATTT TATGTTTTCA GTGGCNTGTG 120
TACATATAGA ATTGTTAAAG TTGTCATTTC CAATATTTAT ATTAGAAAAA TTATTTAGAT 180
ACTTTATAAT TTTAACCGGC ATTTTTAATA ATGACACTTG CATTTATTGT ATTGTAATAA 240
ATTTCACTTT TAACTTTAAA AGTTTAAA 268

SEQ ID NO:1641
SEQUENCE LENGTH:193
SEQUENCE TYPE:nucleic acid
TOPOLOGY:linear
CLONE:HUMGS01856
SEQUENCE DESCRIPTION:
GATCTCTGCT CAGAGAAGTG CAGGGGGAGC CTTCCAGCTC ACTCTCCCTG AGGACTGGCT 60
TGACAGGGGC TATGGGTTTG CTTTGGTGTT GTTTTTAAAA AAAGAAAATA TATTTTTTTG 120
AAAAAACGAC TGCCCATCCC GGGTCCTTTC CCTGATGGGT TGGGGCAGTT ACCTGGTTGC 180
TGTTTTAATT AAA 193

SEQ ID NO:1642
SEQUENCE LENGTH:193
SEQUENCE TYPE:nucleic acid

TOPOLOGY:linear
CLONE:HUMGS01857
SEQUENCE DESCRIPTION:
GATCACATCA CTCCACCCCT GCCAGGCCCC AGGTTAGGAA TAGTGGTGGG AGGAAGGGGA 60
AAGGGCTGGG CCTCACCGCT CCCAGCAACT GAAAGGACAA CACTATCTGG AGCCACCCAC 120
TGAAAGGGCT GCAGGCATGG GCTGTACCCA AGCTGATTTC TCATCTGGTC AATAAAGCTG 180
TTTAGACCAG AAA 193

SEQ ID NO:1643
SEQUENCE LENGTH:193
SEQUENCE TYPE:nucleic acid
TOPOLOGY:linear
CLONE:HUMGS01858
SEQUENCE DESCRIPTION:
GATCCTTAGT ACAACATGTG AAAGAAGATA TGTTGTCTTT ACTCACAGTG GAGGCATTTT 60
TCTAGCTGTG TTTGATTTGG CTTCCCTATA GATTCAGGAC CCATAACTCT TGTTCTCACT 120
CATCTGCTAT GCTGCTGATA AGGACTTTCA GGTCAACAGC TGTAACTGCT AAATGAAGTT 180
AATACTCTGG AAA 193

SEQ ID NO:1644
SEQUENCE LENGTH:192
SEQUENCE TYPE:nucleic acid
TOPOLOGY:linear
CLONE:HUMGS01859
SEQUENCE DESCRIPTION:
GATCTAGGCA AACCTTTATA AAGTTGCAGT ACCTAATCTG TTATTCCCAC TTCTCTGTTA 60
TTTTTGTGTG TCTTTTTTAA TATATAATAT ATATCAAGAT TTTCAAATTA TTTAGAAGCA 120
GATTTNCCTG TAGAAAAACT AATTTTNCTG CCTTTTACCA AAAATAAACT CTTGGGGGAA 180
GAAAAGTGGA AA 192

SEQ ID NO:1645
SEQUENCE LENGTH:188
SEQUENCE TYPE:nucleic acid
TOPOLOGY:linear
CLONE:HUMGS01860
SEQUENCE DESCRIPTION:
GATCTGTCTG TTGCTTGTTT AATATATTTC TTTTATGACA TTACTTAAAG TTTAAAAGGG 60
TTTTNTATCC ACTGTCAATT TCAATTGGAT AACATTTTGT CAAGNTTTTT TTTTCCTGAT 120
TATTTGATGC TAGCTGGAAT TCAAGAAATG GCATTGACCT TATTCAAATA AAGAAATATT 180
TTAGTAAA 188

SEQ ID NO:1646
SEQUENCE LENGTH:190
SEQUENCE TYPE:nucleic acid
TOPOLOGY:linear

CLONE:HUMGS01861
SEQUENCE DESCRIPTION:
GATCAAAAAC TAATTTGGGA TATGTATGGG TAGGGTAAAT CAGTAAGAGG TGTTATTTGG 60
AACCTTGTTT TGGACAGTTT ACCAGTTGCC TTTTATCCCA AAGTTGTTGT AACCTGCTGT 120
GATACGATGC TTCAAGAGAA AATGCGGTTA TAAAAAATGG TTCAGAATTA AACTTTTAAT 180
TCATTCGAAA 190

SEQ ID NO:1647
SEQUENCE LENGTH:183
SEQUENCE TYPE:nucleic acid
TOPOLOGY:linear
CLONE:HUMGS01862
SEQUENCE DESCRIPTION:
GATCAGTTGG GTGCTATGTG CTCAGCTTTC CTACTGCAGC TTCATTTAAT GCAGAGTGGT 60
GTTTGAATTT GGCTTAGATG GGCTGGCAGG CATGATTTTA AAAAATGTAA TCATCAGTAA 120
GAAGTACTTC CATGTTAAAG ATGCAAAAAA AAAAGTCTTC ATTAAAATTN CTATTTAANG 180
AAA 183

SEQ ID NO:1648
SEQUENCE LENGTH:361
SEQUENCE TYPE:nucleic acid
TOPOLOGY:linear
CLONE:HUMGS01863
SEQUENCE DESCRIPTION:
GATCCAAACA AATACACATT CTGTNTTTTA GCTCAGTGTT TTCTAAAAAA AGAAACTGCC 60
ACACAGCAAA AAATTGTTTA CTTTGTTGGA CAAACCAAAT CAGTTCTCAA AAAATNACCG 120
GTGCTTATAA AAAGTTATAA ATATCGAGTA GCTCTAAAAC AAACCACCTG ACCAAGAGGG 180
AAGTGAGCTT GTGCTTAGTA TTTACATTGG ATGCCAGTTT TGTAATCACT GACTTATGTG 240
CAAACTGGTG CAGAAATTCT ATAAACTCTT TGCTGTTTTT GATACCTGCT TTTNGTTTCA 300
TTTTGTTTTG TTTTGTAAAA ATGGTAAANC TTCAGAAAAT AAAATGTCAG TGTTGNNTAA 360
A 361

SEQ ID NO:1649
SEQUENCE LENGTH:183
SEQUENCE TYPE:nucleic acid
TOPOLOGY:linear
CLONE:HUMGS01864
SEQUENCE DESCRIPTION:
GATCAGGACC TGGGGGCCTA CAGTTCTGCC AGGAAGTGCC TGGCCAAACA GAGGCAGAGG 60
ATGCTGCAAG AGAGAAAAGC TGCAAAANAG GCCGCCGCTG CCACCTCCTG AGGCAGNTGT 120
GGGTGCCCCT GCTGTGTGGC TCTGTATGAC TGTTGCTGAA ATATAAAGCC CTGCAACCTG 180
AAA 183

SEQ ID NO:1650
SEQUENCE LENGTH:178

SEQUENCE TYPE:nucleic acid
TOPOLOGY:linear
CLONE:HUMGS01865
SEQUENCE DESCRIPTION:
GATCTCAGCA CAAGCAGGGG AGCAGCTGGG GGAACACAGC TTTATGTAAT TACCTCTGTT 60
CAATATTTAG CAAGTTGCAT TGTAACATTG TAACTGTTTG TTTCTCCTTG GATGATGAGC 120
TACCCCCAAG AAGGGGTCAT ATCAGATTGG GTCTCATTAA AATGTCTGAT GAATGAAA 178

SEQ ID NO:1651
SEQUENCE LENGTH:178
SEQUENCE TYPE:nucleic acid
TOPOLOGY:linear
CLONE:HUMGS01866
SEQUENCE DESCRIPTION:
GATCTATGGC TTAAAAAATT TGTTTCTGTG ACAATNTTTG TAAATCTAGC CAATAGAGTC 60
ATTTACAGAA GAAAAATNAG CATGTAATAA TACAAGAACT GTTTCCCCCT CAAAACCTGA 120
ACCTGAATTA TTTGTAAAAA CTGAAATTTA ATGATTAAAG AGAAGCCAGA ATTGTAAA 178

SEQ ID NO:1652
SEQUENCE LENGTH:189
SEQUENCE TYPE:nucleic acid
TOPOLOGY:linear
CLONE:HUMGS01867
SEQUENCE DESCRIPTION:
GATCTGGAGC TAATAATGAC TGAGATGGAG ATATCTCGAG CAGCAGCAGA ACGCAGTTTG 60
CGGGAACACA TGGGCAACGT GGTAGAGGCG CTTATTGCCC TAACCAACTG ATGCGTGCTT 120
TCTCAAATAT ACCTACTGGA TTAATTTATG GCAATAAAAT TTTTTTTTGT CTTTTTCAGT 180
TTTATCAAA 189

SEQ ID NO:1653
SEQUENCE LENGTH:174
SEQUENCE TYPE:nucleic acid
TOPOLOGY:linear
CLONE:HUMGS01868
SEQUENCE DESCRIPTION:
GATCTCTGCT CTGCCCTCTC CCAGATTGGT GGGGAGGGAG GGCGGGAGGT AGATATAGGC 60
CTGTCCTTTT TAGCAATGTG ATTCTTGTTG TTGATTCTCT CTCTGGAGTT CATGTGCTGC 120
CTCANNNGAC TCTGATTTTA TATTTNAGAA AAATAAAGGC GTTCAATCTG CAAA 174

SEQ ID NO:1654
SEQUENCE LENGTH:173
SEQUENCE TYPE:nucleic acid
TOPOLOGY:linear
CLONE:HUMGS01869
SEQUENCE DESCRIPTION:

GATCAGGCTC ATCTCATCTG CACCAACTGC TCTACCTGTT AGATGGAGAC CTTGCNTCAT 60
GAATTTCTCG AAATGCTCCT GGAACTTATT TATATGCCTC AAAATCCTCT AAACTCATTT 120
ATAGTAACCC ATAGTTTTAA TTTTATAAAT AAACGTATTT ATTAAATCTT AAA 173

SEQ ID NO:1655
SEQUENCE LENGTH:172
SEQUENCE TYPE:nucleic acid
TOPOLOGY:linear
CLONE:HUMGS01870
SEQUENCE DESCRIPTION:
GATCTCTAAT TTAAAGAAAC AGTTTTTATA TACCTAGAAT GAAGATTCTG TTTAAAGCGG 60
TTTTGGTTAA CTTGGTTCTT GACATTTGCC TAAAATNTTN TTGGATTGGG ACTAAAATGT 120
NCTATTAGCC AATGAATAAC GTCCAAGTAA ATTTNTGTTT TATTTTGGGA AA 172

SEQ ID NO:1656
SEQUENCE LENGTH:171
SEQUENCE TYPE:nucleic acid
TOPOLOGY:linear
CLONE:HUMGS01871
SEQUENCE DESCRIPTION:
GATCTCAAAG GAGTCTGAAA TATCATATTT CTGTGTGTGT CTCTCTCAGC CCCTGCCCAG 60
GCTAGAGGGA AACAGCTACT GATAATCGAA AACTGCTGTT TGTGGCAGGA ACCCCTGGCT 120
GTGCAAATAA ATGGGGCTGA GGCCCCTGTG TGATATTGAA AGGGTAAGAA A 171

SEQ ID NO:1657
SEQUENCE LENGTH:171
SEQUENCE TYPE:nucleic acid
TOPOLOGY:linear
CLONE:HUMGS01872
SEQUENCE DESCRIPTION:
GATCGNCATT ATCCCCAGNA AAAAGCTCCG CAACAAGATA GCAGNTAATG TCACGCATCT 60
GATGAAGCGA ATTCAGAGAG GCCCAGTAAG AGGTATCTCC ATCAAGCTGA NCGAGGAGGA 120
GAGAGAAAGG AGAGACAATT ATGTTCCTGA GGTCTCAGCC TTGGNNCCNN N 171

SEQ ID NO:1658
SEQUENCE LENGTH:171
SEQUENCE TYPE:nucleic acid
TOPOLOGY:linear
CLONE:HUMGS01873
SEQUENCE DESCRIPTION:
GATCAAAGAC ATCCTCATCC AGTATGACCG GACCNNCTGG TAGCTGACCC TCGTCGNTGC 60
GAGTCCAAAA AGTTNGGAGT CCTGTGCCCG AGTTCGTACC AGAATCCACC GTAAGCCATC 120
GGNCTAAAAC TTACTTNATA ATAACAGTTT TGAGGGATTT AAAAGTTCAA A 171

SEQ ID NO:1659

SEQUENCE LENGTH:170
SEQUENCE TYPE:nucleic acid
TOPOLOGY:linear
CLONE:HUMGS01874
SEQUENCE DESCRIPTION:
GATCAAAAAT CCAAGGAGAA TGGTGCCAGT GTATGATAAA ATCCATGTAG TGATGAGGAA 60
TGGTGTTAAA TAATGTAATA TATAANANTC ATGATATANG ANTGTTTGAA GGTGATGCAT 120
GTTTGATTTT NGTAGTATAA NTGTATTTNA GTTCAAATGA TGTATAANGN 170

SEQ ID NO:1660
SEQUENCE LENGTH:167
SEQUENCE TYPE:nucleic acid
TOPOLOGY:linear
CLONE:HUMGS01875
SEQUENCE DESCRIPTION:
GATCCTCGCT CCGTTGCACG GGCGCCNTAA GTTATTGGAC TATCTAATAT CTATGTATTT 60
ATNTCGCTGG TTCTTTGTAG TCACATATTT AATAGTCTTA ATATCTTGTT TTTGCATCAC 120
TGTGCCCATT GCAAATAAAT CACTTGGCCA GTTTGCTTTT CTACAAA 167

SEQ ID NO:1661
SEQUENCE LENGTH:167
SEQUENCE TYPE:nucleic acid
TOPOLOGY:linear
CLONE:HUMGS01876
SEQUENCE DESCRIPTION:
GATCTGGGCG ATTCTGAAGC CATGCCATTT TTAACCTTAT GTCTGCTAGA AAGTGTTGTA 60
GTTGATTGAC CAAACCAGTT CATAAGGGGA ATTTTTTTTA AAAAACAACA AAAAAAAAAC 120
CATCCAAGGN NGGGTTTNTG ATTAAATTTT GTGGNGTTAC CAGTAAA 167

SEQ ID NO:1662
SEQUENCE LENGTH:165
SEQUENCE TYPE:nucleic acid
TOPOLOGY:linear
CLONE:HUMGS01877
SEQUENCE DESCRIPTION:
GATCTTCCCC AAGAGGGACT GGGGTTTCTG GGGTCCATTC TCTGAGTCAG TGGTTATTTG 60
AAAATTTGAT TTTGATTTTA TTTTTTCTCT GTAAACTTCC AAGCTGGCTT TTCCCATTTC 120
AATTCCTGTG ATTTATGCCA ATAAAGTTTG CCCATGATTT TCAAA 165

SEQ ID NO:1663
SEQUENCE LENGTH:165
SEQUENCE TYPE:nucleic acid
TOPOLOGY:linear
CLONE:HUMGS01878
SEQUENCE DESCRIPTION:

GATCCCTNCC TTGGTGGGCA GTTCTGCCTT CCAAGGAAGA AGGGGAAGAA AAGGACCTGT 60
GGGTGGCTCA GGNCNAAGCA GACCCCGGGC TCCACCCCAG CCCCGNCCAG GNTGCTGCCA 120
GTNCACACTT TTACAAATTT AATATAAAGC AAGTCCAGTN TTAAA 165

SEQ ID NO:1664
SEQUENCE LENGTH:161
SEQUENCE TYPE:nucleic acid
TOPOLOGY:linear
CLONE:HUMGS01880
SEQUENCE DESCRIPTION:
GATCAGGCTG CTGAGGGAAT AAATTTAATC AAGGTCTTTG CAAAAACAGA AGCACAGAAG 60
GGAGCCTATA TAGAACTAAC ACTGCAGACT TATCAAGAAG CACTCAGTCG CCATTCTGCA 120
GCTTCCTAAA AATATTTTAA AAATACATTT ATTTTACTAA A 161

SEQ ID NO:1665
SEQUENCE LENGTH:158
SEQUENCE TYPE:nucleic acid
TOPOLOGY:linear
CLONE:HUMGS01881
SEQUENCE DESCRIPTION:
GATCAGTCCA TCCCAGAGGG ACCGGAGTTA TGACAAGCTT TCCAAATATT TTGCTTTATC 60
AGCCGATATC AACACTTGTA TCTGGCCTCT GTGCCCCAGC AGTGCCTTGT GCAATGTGAA 120
TGTGCGCGTC TCTGCTAAAC CACCATTTTA TTTGGAAA 158

SEQ ID NO:1666
SEQUENCE LENGTH:158
SEQUENCE TYPE:nucleic acid
TOPOLOGY:linear
CLONE:HUMGS01882
SEQUENCE DESCRIPTION:
GATCGTGTAT TGAGATTTCT TAAATAATGC TTCAGATATT ATTGCTTTAT TGCTTTTTTG 60
TATTGGTTAA AACTGTACAT TTAAAATTGC TATGTTACTA TTTTNTACAA TTAATAGTTT 120
GTCTATTTTA AAATAAATTA GTTGTTAAGA GTCTTAAA 158

SEQ ID NO:1667
SEQUENCE LENGTH:155
SEQUENCE TYPE:nucleic acid
TOPOLOGY:linear
CLONE:HUMGS01883
SEQUENCE DESCRIPTION:
GATCTTTGTT AGTTGCATAT TATATTTCAG TGTTTATCTT CTTGGTGAAT TGACCCTTTT 60
ATCATTATGT AATTCCCCTA TATTTATATC TTTATATTTA ANATAGGTTT CCTTAACATT 120
TGAGAAGTAA AANTAAAATA AAATGTAATA GCAAA 155

SEQ ID NO:1668

SEQUENCE LENGTH:155
SEQUENCE TYPE:nucleic acid
TOPOLOGY:linear
CLONE:HUMGS01884
SEQUENCE DESCRIPTION:
GATCACTCTG GGCTCACTTG CCTGCCTAAT GGTCATCTCC CCAGTAGACT GTAAGCTCCT 60
TGAGGGCAAG GATTGTGTTG GAATTTTTGT ATTAACAGTG CCTGGCTTGG TGCCTGGCAC 120
CTAGAAAGCA CTCAATAAAT GTTTGTTTAA TGAAA 155

SEQ ID NO:1669
SEQUENCE LENGTH:161
SEQUENCE TYPE:nucleic acid
TOPOLOGY:linear
CLONE:HUMGS01885
SEQUENCE DESCRIPTION:
GATCAGATGC TTGAAATTTA ACACTTTTCA CTTGGTTCTT ATACTGAATG CCGACTCTGC 60
TCTGTGTTAG AGATATGAAA TGGTGTTTGA TACTGTTTGA GACATTATGG AGAGATTTAA 120
TTATTTGTAA TAAANGNTTT GCTGCAGTCT GAAAACTGAA A 161

SEQ ID NO:1670
SEQUENCE LENGTH:153
SEQUENCE TYPE:nucleic acid
TOPOLOGY:linear
CLONE:HUMGS01886
SEQUENCE DESCRIPTION:
GATCTATTGT AATGTGTTAT TTNGGCTGAN GTATGTNAAG AAAATACTAC CTTACAAAGN 60
TATGTATTNT CAAAAGGAAA TACATATCAG AAAGTTTAAC AGGGCCAGTG GGTGATACTG 120
AAGTTGTCGG GATGGATGGT GTCTATCTGG NNN 153

SEQ ID NO:1671
SEQUENCE LENGTH:444
SEQUENCE TYPE:nucleic acid
TOPOLOGY:linear
CLONE:HUMGS01887
SEQUENCE DESCRIPTION:
GATCTAGAAG TTTTACTTTT ATAAAGATGG TGTCCGGAAG ATGTTGCTAA TGTATTTTAC 60
TTCAACATAG GGAACAAACT TTTTAAGTAT ATTAATAAAC CTGTATGGTT AGTTTTTAAC 120
AGTTTTTTAA AATAAACTAT GGATATGACA AATATTCTGT GTTTTACTAA GTGCTTGGAT 180
AGGCTTTCTA ATTTNGTATA CGTGCTAGAG TTAATTATTG ANCATTTTTA TCCAAATTTA 240
GTTGTAACTC TGTTTATACT ACTGATTGCT CATTCGTTTA AATGATATTN TNNNGTAAAA 300
GTCATAACCA ACATATGANC AGACAGATTT ATGTCTTTAA ACACAGATTG TNAGCTATAG 360
GTTTAATCTG ATACCAGTTG CTGGAAGGTT GCCATTTGGT TTTTCTNAAA ACCTATACCN 420
CTAAAACTTT CTTTNAAGGT TAAA 444

SEQ ID NO:1672

SEQUENCE LENGTH:151
SEQUENCE TYPE:nucleic acid
TOPOLOGY:linear
CLONE:HUMGS01888
SEQUENCE DESCRIPTION:
GATCAGCAGC CCTCGCAATG TGCTCAGCGA GTACCAGCAG AGGCCGCAGC GGCTGGTGAG 60
CTACTTCATC AAGAAGAACT GAGCAAGGCC TGAGCGCTGC CTGAACTCCG AAGNCCTGTG 120
TGATGCTTTC CATTAAAAAN NATTGTCCAA A 151

SEQ ID NO:1673
SEQUENCE LENGTH:150
SEQUENCE TYPE:nucleic acid
TOPOLOGY:linear
CLONE:HUMGS01889
SEQUENCE DESCRIPTION:
GATCCAGATG ATAAACCAGT GAACTATGTC AAAAGCACTC TCAATATTAC ATTTGACAAA 60
AAGTTTTGTA CTTTNCACAT AGCTTGTTGC CCCGTAAAAG GGTTAACAGC ACAATTTTTT 120
AAAAATAAAT TAAGANGTAT TTATNGGAAA 150

SEQ ID NO:1674
SEQUENCE LENGTH:148
SEQUENCE TYPE:nucleic acid
TOPOLOGY:linear
CLONE:HUMGS01890
SEQUENCE DESCRIPTION:
GATCCGNCCG TCTCTGCCTT CCAAAGTGCT GGGATTACAG GCGTGAGCAC ATCTGCCCGG 60
CTTATTTTTC TTTATGTTTT TNCTTCGTAA GAGGTTCTGT TGAGCAGTGA TTTGCAACTC 120
TTGCTGACGT TGCTGGGGAA GCTTTAAA 148

SEQ ID NO:1675
SEQUENCE LENGTH:568
SEQUENCE TYPE:nucleic acid
TOPOLOGY:linear
CLONE:HUMGS01891
SEQUENCE DESCRIPTION:
GATCTACCCC AGGGAGAATC TGAGACATCT TGCCTACTTT TCTTTATTAG CTTTCTCCTC 60
ATTCATTTCT TTTATACCTT TCCTTTTTGG GGAGTTGTTA TGCCATGATT TTTGGTATTT 120
ATGTAAAAGG ATTATTACTA ATTCTATTTC TCTATGTTTA TTCTAGTTAA GGAAATGTTG 180
AGGGCAAGCC ACCAAATTAC CTAGGCTGAG GTTAGAGAGA TTGGCCAGCA AAAACTGTGG 240
GAAGATGAAC TTTGTCATTA TGATTTCATT ATCACATGAT TATAGAAGGC TGTCTTAGTG 300
CAAAAAACAT ACTTACATTT CAGACATATC CAAAGGGAAT ACTCACATTT TGTTAAGAAG 360
TTGAACTATG ACTGGAGTAA ACCATGTATT CCCTTATCTT TTACTTTTTT TCTGTGACAT 420
TTATGGTCTC ATGTAATTTG CATTACTCTG GTGGATTGTN CTAGTACTGT ATTGGGCTTC 480
TCGGTAATAG GTTATTTCCA TATACTATAA TTGGNAATAT TTTTGTACCA ATGGTTATAA 540
CNCTAGGGGT NTAAAAACCA GGTTCTGN 568

SEQ ID NO:1676
SEQUENCE LENGTH:146
SEQUENCE TYPE:nucleic acid
TOPOLOGY:linear
CLONE:HUMGS01892
SEQUENCE DESCRIPTION:
GATCTCTAAG GTATTATGTT TGCCTTCTCT TAGTTATTTC TGGGTTGTCA CAAAGCTCAG 60
TATCATGGTT TGACAGAAGC AGTTATGTGA ACTTTTATGT TAGGACATTA CTAAATAAAG 120
AATTCCCTAG CTGCTTATAA AGTAAA 146

SEQ ID NO:1677
SEQUENCE LENGTH:144
SEQUENCE TYPE:nucleic acid
TOPOLOGY:linear
CLONE:HUMGS01893
SEQUENCE DESCRIPTION:
GATCCATTCT CCNTTTANTT CCCCCACCCT CCTCTCTNGG ATATGGTTGG NTTTGGCTCA 60
TTTCACAATC AGCCCAAGGC TGGGAAAGCT GGAATGGGAT GGGAACCCCT CCGCCGTGCA 120
TCTNAATTTN AGGGGTCATG CNCN 144

SEQ ID NO:1678
SEQUENCE LENGTH:151
SEQUENCE TYPE:nucleic acid
TOPOLOGY:linear
CLONE:HUMGS01895
SEQUENCE DESCRIPTION:
GATCGAGATG AGCTTTAAAA ACTTGAAAAA CAGTTTGTAA GCCTTTCAAC AGCAGCATCA 60
ACCTACGTGG TGGAAATAGT AAACCTATAT TTTCATAATT CTATGTGTAT TTTTATTTTG 120
AATAAACAGA AAGAAATTTT GGGTTTTTAA A 151

SEQ ID NO:1679
SEQUENCE LENGTH:140
SEQUENCE TYPE:nucleic acid
TOPOLOGY:linear
CLONE:HUMGS01896
SEQUENCE DESCRIPTION:
GATCTTGATG GTCGCTGCAG AGTAACTTCA GCGCTGAGTT CGTTTGAAAC CTTCGGTTTG 60
TTAAGATTCA GAATAACCAA TTGCTTTGTC TGTTAAATCT CCCGATTTCT GTTAGGAAAA 120
TAAAAGCCTC ATTTCTTAAA 140

SEQ ID NO:1680
SEQUENCE LENGTH:139
SEQUENCE TYPE:nucleic acid
TOPOLOGY:linear

CLONE:HUMGS01897
SEQUENCE DESCRIPTION:
GATCACTATG TAGGCAATGC TGGATACTGG NCTGTTATTT ACACATTTGT ATCACCTTTT 60
ATTTCATAAG ATAGCATTGT CAGANTAATT TAGTATTTCA ACATAATCAA CAAGTAAAAT 120
AAAATTAATG AGAATGAAA 139

SEQ ID NO:1681
SEQUENCE LENGTH:139
SEQUENCE TYPE:nucleic acid
TOPOLOGY:linear
CLONE:HUMGS01898
SEQUENCE DESCRIPTION:
GATCTCCACA TTCAGGTTTC CTGATGCACT TTNCGACTCT TTGGGCAACC TCTGGACTCC 60
TTGTNCCCAG GGTCCACATT TAGTTTTATC TTTACTGCAT TGCTTTTATG AAAAAGAATA 120
AAATTGGATG AAACAGAAA 139

SEQ ID NO:1682
SEQUENCE LENGTH:143
SEQUENCE TYPE:nucleic acid
TOPOLOGY:linear
CLONE:HUMGS01899
SEQUENCE DESCRIPTION:
GATCAGTTAA TTTGTATGTA GCAGTGTATG CTCTCATATA CAATNACTGA CCTATGCTCT 60
AAAACATGAA TGCTTTGTTA CAGACCCAAG CTGTCCATTT CTGTGATGGG TTTTGAATAA 120
AGTATTCCCT GTCTTAAATG AAA 143

SEQ ID NO:1683
SEQUENCE LENGTH:136
SEQUENCE TYPE:nucleic acid
TOPOLOGY:linear
CLONE:HUMGS01900
SEQUENCE DESCRIPTION:
GATCGAATCA CCTGAATGTT CTATGTANTG TAAAATATTC TTTTCTTGCT TTCTTGTGTT 60
AAGGTATATA TTCTATTTGT ATGGAATTCT TATTCAAATA CAGTTCTATT AAAGAGTATA 120
CTCCTATTGG ATGAAA 136

SEQ ID NO:1684
SEQUENCE LENGTH:136
SEQUENCE TYPE:nucleic acid
TOPOLOGY:linear
CLONE:HUMGS01901
SEQUENCE DESCRIPTION:
GATCTCAGCG GCATTAAGCT GTGCCTGAGC GAGTTTGTAG TGACTCACTG CACAGCACCN 60
CCAGACTAGC ATGTGGTTCT ATATTTGTAA AGTTATTGGG ATAAGAAACA ATTAAACAGT 120
TTGTAGTAAA CACAAA 136

SEQ ID NO:1685
SEQUENCE LENGTH:136
SEQUENCE TYPE:nucleic acid
TOPOLOGY:linear
CLONE:HUMGS01902
SEQUENCE DESCRIPTION:
GATCTTCTAC TTCCCTNTGG GGAGGGGAGT GACAGGTCCA CACACCACAC TGGGTCACCC 60
TGTCCTGGAT GCCTCTGAAG AGAGGGACAG ACCGTCAGAA ACTGGAGAGT TTCTATTAAA 120
GGTCATTTAA ACCAAA 136

SEQ ID NO:1686
SEQUENCE LENGTH:135
SEQUENCE TYPE:nucleic acid
TOPOLOGY:linear
CLONE:HUMGS01903
SEQUENCE DESCRIPTION:
GATCTTTAAA AAAATTTTCC ATATGTCAAA GTGTAGAGAT GTTTCTAGCA GCATTTATCT 60
TGTCATCTAT AGATTGAACA AAAAGTGGTA GCTGTNAAAT TGTGATTTAT AATAAATACA 120
TATTTGGTCT TCAAA 135

SEQ ID NO:1687
SEQUENCE LENGTH:135
SEQUENCE TYPE:nucleic acid
TOPOLOGY:linear
CLONE:HUMGS01904
SEQUENCE DESCRIPTION:
GATCAGCAGT TCCAGCCCCT ACGCTNGCTG GGGGCGCAAC CACCCCTTCC TTAGGTTGAT 60
GTGCTTGGNA AAGCTCCCTC CCCCTCCTTC CCCAAGAGAG GAAATAAAAG CCACCTTCGC 120
CCTAGGGCCA AGAAA 135

SEQ ID NO:1688
SEQUENCE LENGTH:136
SEQUENCE TYPE:nucleic acid
TOPOLOGY:linear
CLONE:HUMGS01905
SEQUENCE DESCRIPTION:
GATCTTCATC TGTAGCCAAG AAGAGAGCAT TAAACCCAAG ANCATTGTGG AGAAGATTGA 60
CTTTGACAGT GTGTCCAGCA TCATGGCCTC CTCCCAGTAA CTTCAGGTGT TTAATAAAGA 120
TGTGTTGACT CAGAAA 136

SEQ ID NO:1689
SEQUENCE LENGTH:134
SEQUENCE TYPE:nucleic acid
TOPOLOGY:linear

CLONE:HUMGS01906
SEQUENCE DESCRIPTION:
GATCTTAATG TTTCAAAGGA GTGCAGCCCT TCACAGCCAT CAGATATGAG GGCACTGTTC 60
TGTCTGGTGT TGTAGCCATC TCAAGAACAA ATCAACAGCA ACAAAAGAGA AAGAATAAAT 120
TTTTAAAATT TAAA 134

SEQ ID NO:1690
SEQUENCE LENGTH:134
SEQUENCE TYPE:nucleic acid
TOPOLOGY:linear
CLONE:HUMGS01907
SEQUENCE DESCRIPTION:
GATCTGTGAC CCTGGGCGCT GAAAATGGGA CCCAGGAATC CCCCCCGTCA ATATCTTGGC 60
CTCAGATGGC TCCCCAAGGT CATTCATATC TCGGTTTGAG CTCATATCTT ATAATAACAC 120
AAAGTAGCCA CAAA 134

SEQ ID NO:1691
SEQUENCE LENGTH:134
SEQUENCE TYPE:nucleic acid
TOPOLOGY:linear
CLONE:HUMGS01908
SEQUENCE DESCRIPTION:
GATCTCTACT ACCACAAGGA AAATAGTTTA GGAGAAACCA GCTTTTACTG TTTTTNAAAA 60
ATTACAGCTT CACCCTGTCA AGTTAACAAG GAATGCCTGT GCCAATAAAA GGTTTCTCCA 120
ACTTGAAGTC TAAA 134

SEQ ID NO:1692
SEQUENCE LENGTH:140
SEQUENCE TYPE:nucleic acid
TOPOLOGY:linear
CLONE:HUMGS01909
SEQUENCE DESCRIPTION:
GATCAGTGTG GAGGACCCGC CCCAGCGCAC GGCCGGCGTC AAGGTGGAGA CCACTGAGGA 60
CCTGGTGGCC AAGCTGAAGG AGATTGGGCG GATTTGAGCC CCTCCCAGAG ATGGCAATAA 120
AACTGACTCT CAACATCAAA 140

SEQ ID NO:1693
SEQUENCE LENGTH:133
SEQUENCE TYPE:nucleic acid
TOPOLOGY:linear
CLONE:HUMGS01910
SEQUENCE DESCRIPTION:
GATCTAACCT TTGANGCTTT AAAAAAGGAG AAAGAGGGTA GGGGTGGGAA ACTGGCATAC 60
TGTGTGTATA GCACTGCCGA TTGGCTAGGC CACTGTGTCT CTGCTACAAA TTAAAGAAAT 120
CCTAAAAGTT AAA 133

SEQ ID NO:1694
SEQUENCE LENGTH:133
SEQUENCE TYPE:nucleic acid
TOPOLOGY:linear
CLONE:HUMGS01911
SEQUENCE DESCRIPTION:
GATCGCGAAT AAAAATCAAC AAATGTGAAA GCCCAGAAAA ATATATTCGT ATTTCTGGTT 60
TTGCTGGATT TTTACATTTT TATATAATAA AAATGNTATT TTGAAATAAA GATTATGCTG 120
ACTCAAATGC AAA 133

SEQ ID NO:1695
SEQUENCE LENGTH:131
SEQUENCE TYPE:nucleic acid
TOPOLOGY:linear
CLONE:HUMGS01912
SEQUENCE DESCRIPTION:
GATCTATTGC AGATATTTGA TGTAGTTTTC TCTTTTAAAT TAATCAGAAA CCCCACTTCC 60
ATTGTATTGT CTGACACATG CTCTCAATAT ATAATAAATG GGAAATGTCG ATTTTCAATA 120
ATAGACTTAA A 131

SEQ ID NO:1696
SEQUENCE LENGTH:130
SEQUENCE TYPE:nucleic acid
TOPOLOGY:linear
CLONE:HUMGS01913
SEQUENCE DESCRIPTION:
GATCCGCCCG GCTCAGCAGA ATTCACGCTC CTGTATGCCT CAGCCCCTCG ATATAACTCC 60
ATCGGATTCT AGCAGCCTGG TATTCTAGCA ATAAAACCCA AGGCCTGAGA AACAGGGGCC 120
ATCCTGCAAA 130

SEQ ID NO:1697
SEQUENCE LENGTH:130
SEQUENCE TYPE:nucleic acid
TOPOLOGY:linear
CLONE:HUMGS01914
SEQUENCE DESCRIPTION:
GATCAGACTG TCACTTTATA GTTTTTCTAT ATAAAGATTA TATAGTTGCA AACAAAGGTT 60
GTGAAATTTC CCTTTTGTTG TTTTTNACTT TTAATTAATA AAAGTACATT GTTTTCATAG 120
CAAACTTAAA 130

SEQ ID NO:1698
SEQUENCE LENGTH:130
SEQUENCE TYPE:nucleic acid
TOPOLOGY:linear

CLONE:HUMGS01915
SEQUENCE DESCRIPTION:
GATCGAAGCT ACCGTAATGT GTTCTTGAGG TCAACATGTT TTGTGGAAGT CACTGTATTT 60
GCCCCNTTAT GTCAATTACC TATTATAAAC CGAGAGAAAT GGGAAAATTA AAAACCTGTT 120
TTGAATTAAA 130

SEQ ID NO:1699
SEQUENCE LENGTH:129
SEQUENCE TYPE:nucleic acid
TOPOLOGY:linear
CLONE:HUMGS01916
SEQUENCE DESCRIPTION:
GATCCCTTGT AAACATGAAA TCATTCCATG GATGGCTGCC TTATAATTTT GTCTCTTTCC 60
ACTTTAATTG TGAATGGTTA AAAAAATGCT GTTTTCTGAT ATTAAATTTT TATTAGTGCA 120
TACCTTAAA 129

SEQ ID NO:1700
SEQUENCE LENGTH:82
SEQUENCE TYPE:nucleic acid
TOPOLOGY:linear
CLONE:HUMGS01917
SEQUENCE DESCRIPTION:
GATCTGTACC TAGTACCCCT CCCATCTACT GATTTGTTTG TTTTTGTAAC CAAACACATT 60
TTCAGATAGA AGGAGCCTTA AA 82

SEQ ID NO:1701
SEQUENCE LENGTH:128
SEQUENCE TYPE:nucleic acid
TOPOLOGY:linear
CLONE:HUMGS01918
SEQUENCE DESCRIPTION:
GATCCCCTAT TCCCTCCACA ATAACAGAAA CACTCCCAGG GACTCTGGGG AGAGGCTGAG 60
GACAAATACC TGCTGTCACT CCAGAGGACA TTTTTTTTAG CAATAAAATT GAGTGTCAAC 120
TATTTAAA 128

SEQ ID NO:1702
SEQUENCE LENGTH:137
SEQUENCE TYPE:nucleic acid
TOPOLOGY:linear
CLONE:HUMGS01919
SEQUENCE DESCRIPTION:
GATCTACAAT NAAGCCCTCA AGGGCTGAAA ATAAATAGGG AAGATGGAGA CACCCTCTGG 60
GGGTCCTCTC TGAGTCAAAT CCAGTGGTGG GTAATTGTAC AATAAATTTT TTTTGGTCAA 120
ATTTAAAAAA AAATAAA 137

SEQ ID NO:1703
SEQUENCE LENGTH:128
SEQUENCE TYPE:nucleic acid
TOPOLOGY:linear
CLONE:HUMGS01920
SEQUENCE DESCRIPTION:
GATCTGAGGA GGAAATGTGA CGCCGGACGC TTCCNTGTNT TTGACCTTTT NAAAGGANGA 60
GGAAGTTCAC CGACANTACC CATCACCCAC AAAAGTCTCA CTTCTTGGAA GATTTGTGTT 120
AGGCTGNN 128

SEQ ID NO:1704
SEQUENCE LENGTH:127
SEQUENCE TYPE:nucleic acid
TOPOLOGY:linear
CLONE:HUMGS01921
SEQUENCE DESCRIPTION:
GATCTGGCGT NCTACTATGA GGCTTCTTTT GACCTCATTN NTGTGGTNGC CGGCGTAGCC 60
AGACCATCCT ATACTGTGAC TACTTCTACT NGTACATTAC AAAAGTACTC AAGGGAAAGA 120
AGCTCNN 127

SEQ ID NO:1705
SEQUENCE LENGTH:146
SEQUENCE TYPE:nucleic acid
TOPOLOGY:linear
CLONE:HUMGS01922
SEQUENCE DESCRIPTION:
GATCATAGCT ACACTGTATG TTTAGGTGGT GTGAAATNAT TTATAATCAC AGCTTGAACT 60
GTGTTTGCTT GGTACTGTCA TAGTGATTAC AAATTTCATG GAATGCGAAG AGCAACAATA 120
AATAAAAAAT ACCACTCACC CTCAAA 146

SEQ ID NO:1706
SEQUENCE LENGTH:240
SEQUENCE TYPE:nucleic acid
TOPOLOGY:linear
CLONE:HUMGS01923
SEQUENCE DESCRIPTION:
GATCAAAAAG GACTGAACCC CCACTATCAT AATCCATACA TTCATTTCCA TGTTGTTTGA 60
CTAGCAGTGC ATATATGAAT GAATATGGTA ATTAATCTTA CCTATAAGAA CCAAATACTT 120
TAATTATATT AAGATAGTGA ATAAAGATGT ATAATATTTT NATTAATACC TTGAATATAT 180
TCAGTGGATT GAATGTGACT TCATAACTGT ACTACGATTG TATTAAAATA TTTCTGGAAA 240

SEQ ID NO:1707
SEQUENCE LENGTH:124
SEQUENCE TYPE:nucleic acid
TOPOLOGY:linear

CLONE:HUMGS01924
SEQUENCE DESCRIPTION:
GATCTTATGA AGGAAGATTT GTGACCCTAC GTATATATAT ACACACACAT ACATATATAT 60
ATATATCCCG AACCAACANC GGGACTTTGT TTATATTGCA AATAAATATT ATTTTTCCTT 120
TAAA 124

SEQ ID NO:1708
SEQUENCE LENGTH:124
SEQUENCE TYPE:nucleic acid
TOPOLOGY:linear
CLONE:HUMGS01925
SEQUENCE DESCRIPTION:
GATCCCCTTT CTCTATGTCG GGACACTCAT TAGCAAGAAC TTTNCTGCTC TACTNGAGGN 60
ACATGACATT TTTGTTCCAG AGGATGATGA TGATGATGAC TAACAGGAAT TACAGAANGN 120
NGAN 124

SEQ ID NO:1709
SEQUENCE LENGTH:146
SEQUENCE TYPE:nucleic acid
TOPOLOGY:linear
CLONE:HUMGS01926
SEQUENCE DESCRIPTION:
GATCCGAGCA GTCCTCTGCC CGCCTCCCGT GAAGAAGAGG AAGAGAAAAT GCCTGCTGTT 60
GTAAATGTCT CAGCCCCTCG TTCTTGGTCC TGTCCCTTGG AACCTTTGTA CGCTTTGCTC 120
AAAAAAAAAC AAAAAAAAAA AACAAA 146

SEQ ID NO:1710
SEQUENCE LENGTH:120
SEQUENCE TYPE:nucleic acid
TOPOLOGY:linear
CLONE:HUMGS01927
SEQUENCE DESCRIPTION:
GATCCTGTGT AGAAGCTGTT CTCATTAAAC ACCAAACAGT TAAGTCCATT CTCTGGTACT 60
AGCTACAAAT TCGGTTTCAT ATTCTACTTA ACAATTTAAA TAAACTGAAA TATTTCTAAA 120

SEQ ID NO:1711
SEQUENCE LENGTH:121
SEQUENCE TYPE:nucleic acid
TOPOLOGY:linear
CLONE:HUMGS01928
SEQUENCE DESCRIPTION:
GATCTGCGCC TCTCAGTGCC TTTTGAGGGG TTCCCATCAT CCCTCCCTGA TATTGTATTG 60
AAAATATTAT GCACACTGTT CATGCTTCTA CTAATCAATA AACGCTTTAT TTAAAGCCAA 120
A 121

SEQ ID NO:1712
SEQUENCE LENGTH:125
SEQUENCE TYPE:nucleic acid
TOPOLOGY:linear
CLONE:HUMGS01929
SEQUENCE DESCRIPTION:
GATCGGGGCA AGAGAAGGCT GAGTACGGAT GGGAAACTAT TGTGCACAAG TCTTTCCAGA 60
GGAGTTTCTT AATNAGATAT TTGTATTTAT TTCCAGACCA ATAAATTTGT AACTTTGCAG 120
CGAAA 125

SEQ ID NO:1713
SEQUENCE LENGTH:338
SEQUENCE TYPE:nucleic acid
TOPOLOGY:linear
CLONE:HUMGS01930
SEQUENCE DESCRIPTION:
GATCAGCCCA TCTCAGCTTG CTGTTTCAAT CACAATGGAA ACATATTTGC ATACGCTTCC 60
AGCTACGACT GGTCAAAGGG ACATGAATTT NATAATCCCC AGAAAAAAAN TTACATTTNC 120
CTGCGTAATG CAGCCGAAGA GCTAAAGCCC AGGAATAAGA AGTAGTGGCT GGAGACTCTG 180
GCTCAGCCAG AGTTGTTTCT CTCCANTCTG CCTCATCTCT GTACGANTTT GGGTCCCAGC 240
CTTGTTGGGT TGTCAGCCAT GGACATGGAT TTCAACCCCT GGAGAAAACG NTGTCATTGT 300
TCAGCAGCTG AGNGCCCCAG GCGTCCGNGG CGNCTTTN 338

SEQ ID NO:1714
SEQUENCE LENGTH:85
SEQUENCE TYPE:nucleic acid
TOPOLOGY:linear
CLONE:HUMGS01931
SEQUENCE DESCRIPTION:
GATCCCATAT CTATTACCGT GTCCATAGGA ATAATAGGTA AGGGCTCTGT CTCTGTCAAG 60
CCATGTAACA AAGGACACTG TTAAA 85

SEQ ID NO:1715
SEQUENCE LENGTH:260
SEQUENCE TYPE:nucleic acid
TOPOLOGY:linear
CLONE:HUMGS01932
SEQUENCE DESCRIPTION:
GATCCAGCCC TGGTTCTGGC TGTGGTCAGC AGATGCCAGT NAAGGGTTTT GTGTGTTTAG 60
GCCTCATTTC TTTGTCTTTT TCCTACTCCG TTCCTGGCAT TTGCTGATTT CTAGTGTATA 120
CTCTGTAGTC TCAGTTCGTG TTTGATTCCA TTCCATGGAA ATAAAAAGTA TGTTGTACAT 180
ACTGCCGAAG AATTGTCTTG CAAGTNAAGG CTTCCCCCTT TACTATAAGA CTATAAATAA 240
AANCTTATTT NATCCNNAAA 260

SEQ ID NO:1716

SEQUENCE LENGTH:430
SEQUENCE TYPE:nucleic acid
TOPOLOGY:linear
CLONE:HUMGS01933
SEQUENCE DESCRIPTION:
GATCTTGTGC TGTGCTATCC GCAGAACCGC GAGATGGTCT AGAGTCAGCT TACATCCCTG 60
AGCAGGAAAG TTTACCCATG AAGATTGGTG GGATTTTTTG TTTGTTTGTT TTGNTTTGTT 120
TGTTGTTTGT TGTTTGTTTT TTTGCCACTA ATTTTAGTAT TCATTCTGCA TTGCTAGATA 180
AAAGCTGAAG TTACTTTATG TTTGTCTTTT AATGCTTCAT TCAATATTGA CATTTGTAGT 240
TGAGCGGGGG GTTTGGTTTG CTTTGGTTTA TATTTTTNCA GTTGTTTGTT TTTGCTTGTN 300
ATATTAAGCA GAAATCCTGC AATGAAAGGT ACTATATTTG CTAGACTCTA GACAAGATAT 360
TGTACATAAA AGAATTTTTT TGNCTTTAAA TAGATACAAA TGTCTANCAC CTTTAATCAA 420
GTTGTACCTN 430

SEQ ID NO:1717
SEQUENCE LENGTH:171
SEQUENCE TYPE:nucleic acid
TOPOLOGY:linear
CLONE:HUMGS01934
SEQUENCE DESCRIPTION:
GATCGAGCCA CTGCGCTCCG GCCTGGGTGA CAGGAGACTC CATCTCAAAA AATAAAATTA 60
AAAAAAAAAC TACTACAATA AATTATCACC TTGGACTGTA TAAAACAACT TTNAAACTAG 120
TCTTTTNAAA AAGTACACTT AAATAAAAAT CTAANCATAA GNGANTGTAA A 171

SEQ ID NO:1718
SEQUENCE LENGTH:115
SEQUENCE TYPE:nucleic acid
TOPOLOGY:linear
CLONE:HUMGS01935
SEQUENCE DESCRIPTION:
GATCGCTCAG AAAATGGGAA CCCAGGGCAA ATTGTATGTG CTCCTTACTG GGTTTATTAT 60
AAGTGTCACA TGTTTTTTAT AATAAAACAT AGGTGATTTC ACCTTAATGG ACAAA 115

SEQ ID NO:1719
SEQUENCE LENGTH:115
SEQUENCE TYPE:nucleic acid
TOPOLOGY:linear
CLONE:HUMGS01936
SEQUENCE DESCRIPTION:
GATCCTCCCA CCTCAGCCTC CTGAGTAGCT GGGACCATAG GCTCACAACA CCACACCTGG 60
CAAATTTGAT TTTTTTTTTT TTTCCAGAGA NGGGGTNTTG NANCATTGCC CAGNN 115

SEQ ID NO:1720
SEQUENCE LENGTH:115
SEQUENCE TYPE:nucleic acid

TOPOLOGY:linear
CLONE:HUMGS01937
SEQUENCE DESCRIPTION:
GATCTAGCAC TTTACAAAGT AAGTTTTTCT GTNACTTAGA GCTGAAAATA AAAGCAATGT 60
AGAGTTACGC TTTTATAAGT ATTTNANGTT TCANAAATNA TGCATACAGC AATTN 115

SEQ ID NO:1721
SEQUENCE LENGTH:114
SEQUENCE TYPE:nucleic acid
TOPOLOGY:linear
CLONE:HUMGS01938
SEQUENCE DESCRIPTION:
GATCATTTAG TTCTATCTAT TTAGAAATAT GTAAAACTGG ATTTTTTTTT AAGTAATATG 60
TGACCAAAGT TAATTTTGTC CCAAAGGTCT AAATAAAGAG CAGTTTCCCA TAAA 114

SEQ ID NO:1722
SEQUENCE LENGTH:119
SEQUENCE TYPE:nucleic acid
TOPOLOGY:linear
CLONE:HUMGS01939
SEQUENCE DESCRIPTION:
GATCGCCACA GCCCTTCTTG GAAGAAAGGC GTCTGTGTTT CAGGTTCCAC GCGAGTCACC 60
TCTTTCGTCT TAATGTTCAC CGTCCACAGC TTTGGAATAA ACCATCCTGG GAAGTTAAA 119

SEQ ID NO:1723
SEQUENCE LENGTH:113
SEQUENCE TYPE:nucleic acid
TOPOLOGY:linear
CLONE:HUMGS01940
SEQUENCE DESCRIPTION:
GATCTTTGAC TGTATCATGC ATTGTACAAT TGATTTCATA TTTTATGAAA TGCCTTAATT 60
TTCCTACTAT AACATAAAGA CAATGATGAA TAAAGTTTAT GTGTATGATT AAA 113

SEQ ID NO:1724
SEQUENCE LENGTH:113
SEQUENCE TYPE:nucleic acid
TOPOLOGY:linear
CLONE:HUMGS01941
SEQUENCE DESCRIPTION:
GATCAGAAAC ATACATACCC TNCCTAGGGA TTTAGAAAGT GGGTTGGCAG TCTTTCCTCA 60
CGTCCATCAC GCANTTGGNA CCTACTNCAG TGTATTGTAA ACTTTTTTCT CNN 113

SEQ ID NO:1725
SEQUENCE LENGTH:111
SEQUENCE TYPE:nucleic acid

TOPOLOGY:linear
CLONE:HUMGS01943
SEQUENCE DESCRIPTION:
GATCCCAGAG GTCANGACCA AANCCAATCA GTGAAAATTC AACTNTAGCT GTGAAAACAT 60
TGCACTAACA GGAGCTATCC AACAATGGAC CAGAATGTTG ACAATAAAGT N 111

SEQ ID NO:1726
SEQUENCE LENGTH:113
SEQUENCE TYPE:nucleic acid
TOPOLOGY:linear
CLONE:HUMGS01944
SEQUENCE DESCRIPTION:
GATCCCGCAG GTGGCACGTG ACAGCTAGGG TTCAAAACGT TCTCACCAAA TCCAATGCTC 60
CTCACATATT AATTTTATAA CCAGACAAAT AAATATTAGA GACAACCACC AAA 113

SEQ ID NO:1727
SEQUENCE LENGTH:109
SEQUENCE TYPE:nucleic acid
TOPOLOGY:linear
CLONE:HUMGS01945
SEQUENCE DESCRIPTION:
GATCTCAAAA TTCCAGAATT CCCTGTACAT CTNTCCACGT GCTTGTGCTC CAGGTGTGAC 60
TTGTAAACTG TCTAGTGTTT GCNTTAAATA AAATGGCACC GAGCATAAA 109

SEQ ID NO:1728
SEQUENCE LENGTH:329
SEQUENCE TYPE:nucleic acid
TOPOLOGY:linear
CLONE:HUMGS01946
SEQUENCE DESCRIPTION:
GATCCGTATC GGTTTAAGAA GCGGACGGAG CTGTTCATTG CCGCCGAGGG CATTCACACG 60
GGCCAGTTTG TGTATTGCGG CAAGAAGGCC CAGCTCAACA TTGGCAATTT GCTCCCTGTG 120
GGCACCATGC CTGAGGGTAC AATCGTGTGC TGCCTGGAGG AGAAGCCTGG AGACCGTGGN 180
AAGCTGGCCC GGGCATCAGG GAACTATGCC ACCGTTATCT CCNACAACCC TGAGACCANG 240
ANGACCCGTG TGNAGNTGCC CTCCGANTCC AAGANGGTTA TNTCCTNAGN CATCAGNGCT 300
GTGGTTGGTG TGGTGGCTGG NGNTNGNCN 329

SEQ ID NO:1729
SEQUENCE LENGTH:109
SEQUENCE TYPE:nucleic acid
TOPOLOGY:linear
CLONE:HUMGS01947
SEQUENCE DESCRIPTION:
GATCTGTCAA TTTCTTTGTN CAGAATGATT TGAAGTATTG TATTCAGTTT ACATGCATTA 60
TTGGNTTATA ATNAATATCT AATGAAAATA CATGTTGTTA TATTGTAAA 109

SEQ ID NO:1730
SEQUENCE LENGTH:215
SEQUENCE TYPE:nucleic acid
TOPOLOGY:linear
CLONE:HUMGS01948
SEQUENCE DESCRIPTION:
GATCAGCTGA TGGAGCTCCT GATTTGACAA AGGAGCTTNC CTCCTTTGAA TGACCTAGAG 60
CACAGGGAGG AACTTGTCCA GTAGTTTGGA ATTGTGTTCT TCGTAAAGAC TGAGGCAAGC 120
AAGTGCTGTG AAATAACATC ATCTTAGTCC CTTGGTGTGT GGGNNNTTTG TTTTTTTTTT 180
ATATTTTGAG AATAAAACTT CATATAAAAT TGAAA 215

SEQ ID NO:1731
SEQUENCE LENGTH:107
SEQUENCE TYPE:nucleic acid
TOPOLOGY:linear
CLONE:HUMGS01949
SEQUENCE DESCRIPTION:
GATCGCACCG CCCCTCGCAG AGAGTGTATC ANCTGTTTTA TTTTTGTAAA AACAAAGTGC 60
TAAATAATAT TNATNACTTG GTTTGGTTNC AAAANCGGAT TAAATGN 107

SEQ ID NO:1732
SEQUENCE LENGTH:107
SEQUENCE TYPE:nucleic acid
TOPOLOGY:linear
CLONE:HUMGS01950
SEQUENCE DESCRIPTION:
GATCACTGTG GTNTTCATAT TTCTAAAATA AATGAATTAA AAANTTTAAN GNCTTAGATG 60
CTTATCTCTA TCATAGCTCA GAATGCAACT NCAATACCCC CATTCTN 107

SEQ ID NO:1733
SEQUENCE LENGTH:106
SEQUENCE TYPE:nucleic acid
TOPOLOGY:linear
CLONE:HUMGS01951
SEQUENCE DESCRIPTION:
GATCAACTGT ACGCCTTTGG TATCTGACCA TAAAGTCTTT TGCTCCGCTG ACATTTGGGT 60
GATGTCTTCA CATGGAAATA TAATAAAAAT AAAAATCTAG TTTAAA 106

SEQ ID NO:1734
SEQUENCE LENGTH:106
SEQUENCE TYPE:nucleic acid
TOPOLOGY:linear
CLONE:HUMGS01952
SEQUENCE DESCRIPTION:

GATCGAGGCT TCAGTGAGAT ATGGCTGAGA CACTGCTCTC AGCCTGGATG ACAGAGTGAG 60
AACCTGTCTC AAACAAGAGA AAAAAATAAA TCAAANNCTA TTCAAA 106

SEQ ID NO:1735
SEQUENCE LENGTH:151
SEQUENCE TYPE:nucleic acid
TOPOLOGY:linear
CLONE:HUMGS01953
SEQUENCE DESCRIPTION:
GATCTGAGGA GGCTTCGTGG GCTTTTGGGT CCTCTAACTA GGACTCCCTC ATTCCTAGAA 60
ATTTAACCTT AATGAAATCC CTAATAAAAC TCAGTGCTGT GTTATTTGTG CCTCATTTCC 120
CCAGGAATAG TCACTGCCTT GAGGAACCAA A 151

SEQ ID NO:1736
SEQUENCE LENGTH:106
SEQUENCE TYPE:nucleic acid
TOPOLOGY:linear
CLONE:HUMGS01954
SEQUENCE DESCRIPTION:
GATCCACCCA CCTNAGGCCT CCCAAAGTGC TGGGANNACA GNCGTGANAA CCACGGCTTC 60
GCCAACAATG GATTTTTAAA TTATTTCCAC GCTCACAAAA AACCTN 106

SEQ ID NO:1737
SEQUENCE LENGTH:106
SEQUENCE TYPE:nucleic acid
TOPOLOGY:linear
CLONE:HUMGS01955
SEQUENCE DESCRIPTION:
GATCNANCCT CAACACCTAG TGCAATTCTT TTTACACATT AGGNACTTAG TAATTNGGGT 60
TGAATTGTGT TGAACTAACC TGGCGGTATA GGCAAAANTT GGAAGN 106

SEQ ID NO:1738
SEQUENCE LENGTH:105
SEQUENCE TYPE:nucleic acid
TOPOLOGY:linear
CLONE:HUMGS01956
SEQUENCE DESCRIPTION:
GATCTCCCAT TTCTAGGAAC CCCAGTCCTG CTTCTCCGCA ATGGCACATG CTTCCACTCC 60
ATCCATACTG GCATCCTCAA ATAAACAGAT ATGTATACAT ATAAA 105

SEQ ID NO:1739
SEQUENCE LENGTH:105
SEQUENCE TYPE:nucleic acid
TOPOLOGY:linear
CLONE:HUMGS01957

SEQUENCE DESCRIPTION:
GATCCAAGAG ATATATTTGG CAACTTTTTC TAGAAAAGGC ACATTGGGTA TAATTCATTA 60
CATTCTTGAG TTTTTTTGGG TTTTTTTTTT TTTTTTNGNG NNANN 105

SEQ ID NO:1740
SEQUENCE LENGTH:107
SEQUENCE TYPE:nucleic acid
TOPOLOGY:linear
CLONE:HUMGS01958
SEQUENCE DESCRIPTION:
GATCCAGCTG GAAAGCTGAA GTATTTCGAT AAACTGAATT GAGAAATACT TCTTCAAGTT 60
ATGATGCTTG AAAGTTCTCA ATAAAGTTCA CGGTTTCATT ACCNAAA 107

SEQ ID NO:1741
SEQUENCE LENGTH:119
SEQUENCE TYPE:nucleic acid
TOPOLOGY:linear
CLONE:HUMGS01959
SEQUENCE DESCRIPTION:
GATCTTAAGG TATCAGTATT TCAGAATCCT GCGACGATTT TATTTCTAAA TTCATGTACT 60
GTATGTCCAT AAGTGAAAAT AAAATNTCAT ATTCTTTTCT ATNATTGGTA TCATTCAAA 119

SEQ ID NO:1742
SEQUENCE LENGTH:114
SEQUENCE TYPE:nucleic acid
TOPOLOGY:linear
CLONE:HUMGS01960
SEQUENCE DESCRIPTION:
GATCCTTAGC TCTTTGCTCT ATGGCCCTTC CTCATCAGGG GACCGTTTCC CCCCTCTTCC 60
TTCACAGTAT TTAAGAAATA AAAGTCGGAT TTTNCTGGCT GCTTTCTCTC TAAA 114

SEQ ID NO:1743
SEQUENCE LENGTH:102
SEQUENCE TYPE:nucleic acid
TOPOLOGY:linear
CLONE:HUMGS01961
SEQUENCE DESCRIPTION:
GATCGCCCCA TTGCACTNCA ANCTGGACAG CAAGAGCAAA ACTCCGTNTC AAAAAACAAA 60
AACAAAANCA AACAAAAAAN TTCCCCTGAG AGAAAACCTA TN 102

SEQ ID NO:1744
SEQUENCE LENGTH:101
SEQUENCE TYPE:nucleic acid
TOPOLOGY:linear
CLONE:HUMGS01962

SEQUENCE DESCRIPTION:
GATCACACAC TAAGAGAACG TTGATTGCCT CGGCTATTGT GCTGGCTGGA CACTTTGGTC 60
ACTTTTGAAG CATGTTAATA AATGTCACTG ATTAAAACAA A 101

SEQ ID NO:1745
SEQUENCE LENGTH:100
SEQUENCE TYPE:nucleic acid
TOPOLOGY:linear
CLONE:HUMGS01963
SEQUENCE DESCRIPTION:
GATCCAAGTT TGAAAACAAT TTTAGGCTAC TAGATATGAA CAACATCTTG ATTATGTAGT 60
TGAAGGAAAT TAAAGATGAA TGGTTTAATT AAAAATTAAA 100

SEQ ID NO:1746
SEQUENCE LENGTH:99
SEQUENCE TYPE:nucleic acid
TOPOLOGY:linear
CLONE:HUMGS01964
SEQUENCE DESCRIPTION:
GATCAAATTA AATAATTCAA GCCCTGCCTT CAAAATGAAA TTTTTTTTT AGTATTATTT 60
AGCAAAANCA ATAAAACCCA AATTTTTTA ACCATCAAA 99

SEQ ID NO:1747
SEQUENCE LENGTH:97
SEQUENCE TYPE:nucleic acid
TOPOLOGY:linear
CLONE:HUMGS01965
SEQUENCE DESCRIPTION:
GATCACTTCA AGCAAGCCCC CATGATGGTT CTCAGTCCTG CTTCTCTGTG GGTACGTGCC 60
CCTCTGTTTA AAAATAAACT GAATATGGAT GTTTAAA 97

SEQ ID NO:1748
SEQUENCE LENGTH:100
SEQUENCE TYPE:nucleic acid
TOPOLOGY:linear
CLONE:HUMGS01966
SEQUENCE DESCRIPTION:
GATCAAAATG AAAACCTGTT GATGTGAATT CAGTTATTGA ACTTGTTACT TGTTTTTGCC 60
AGAAATGTTA TTAATAAATG TCAATGTGGG AGATAATAAA 100

SEQ ID NO:1749
SEQUENCE LENGTH:95
SEQUENCE TYPE:nucleic acid
TOPOLOGY:linear
CLONE:HUMGS01967

SEQUENCE DESCRIPTION:
GATCTGATGT TCCTTTTATT TATAACAATG TTAAATGCAA TTGTCTTGTA CCTTGAGTTG 60
AGTATTACAC ATTAAAGTAA AGTACAAGCT GTAAA 95

SEQ ID NO:1750
SEQUENCE LENGTH:94
SEQUENCE TYPE:nucleic acid
TOPOLOGY:linear
CLONE:HUMGS01968
SEQUENCE DESCRIPTION:
GATCCAGAAG TAAATGTATC TNCAAGCTGA TTTAAATTGT AGATTTNNTC TTCATTGCAT 60
ATTTNCTGAT GTTGAATAAA GTCTGAGTAT TAAA 94

SEQ ID NO:1751
SEQUENCE LENGTH:89
SEQUENCE TYPE:nucleic acid
TOPOLOGY:linear
CLONE:HUMGS01969
SEQUENCE DESCRIPTION:
GATCCAAAGT ATATTTTTAC TGAGAATATA TCTGAAAAAA TTTGAATTGA GATTAAATTT 60
TTAAATGAAA TATATTTACT TTTTTTAAA 89

SEQ ID NO:1752
SEQUENCE LENGTH:89
SEQUENCE TYPE:nucleic acid
TOPOLOGY:linear
CLONE:HUMGS01970
SEQUENCE DESCRIPTION:
GATCCTGGCC CCTCAACCTC CTGTGGAACC CAGCAGTTCT GTTATATCCC CTGCTACCCT 60
AGATGAATTA AGACGGTTAA ATACTGAAA 89

SEQ ID NO:1753
SEQUENCE LENGTH:89
SEQUENCE TYPE:nucleic acid
TOPOLOGY:linear
CLONE:HUMGS01972
SEQUENCE DESCRIPTION:
GATCTGTCTT GCTTATTGTT GCCCCCTGCG NCTAGCACAA TTCTGACACA CAATTGGAAC 60
TTACTAAAAA TTTNTTTTTA CTGTTTAAA 89

SEQ ID NO:1754
SEQUENCE LENGTH:87
SEQUENCE TYPE:nucleic acid
TOPOLOGY:linear
CLONE:HUMGS01973

SEQUENCE DESCRIPTION:
GATCATATGG TAGTTTTGTT TTACTAATCT AAGGGTACTA GCATCTACAA TNATATAGAC 60
AAAATAAAAT ATTTCTTTAA TGGCAAA 87

SEQ ID NO:1755
SEQUENCE LENGTH:85
SEQUENCE TYPE:nucleic acid
TOPOLOGY:linear
CLONE:HUMGS01974
SEQUENCE DESCRIPTION:
GATCTGTTTT CTCTAAAATC TTACCATATT GTCTGTATAT GGTTGTAAAT NCAAATGGAA 60
AGTAAAACGT TTTGGCCCTG ATAAA 85

SEQ ID NO:1756
SEQUENCE LENGTH:88
SEQUENCE TYPE:nucleic acid
TOPOLOGY:linear
CLONE:HUMGS01975
SEQUENCE DESCRIPTION:
GATCCTGCTG GAAACCACGG CAACCTGTAT CCACTATTAG GAGGTAAAAA TCAATAAAAT 60
GGCCCATTCA TTTGTGTTGT AGCTCAAA 88

SEQ ID NO:1757
SEQUENCE LENGTH:84
SEQUENCE TYPE:nucleic acid
TOPOLOGY:linear
CLONE:HUMGS01976
SEQUENCE DESCRIPTION:
GATCTACAAC ATATAAAATT GTGTATTTTC NTTTGGTTGT CCCTATTAAC AAAAAAGTAT 60
TTNAATAAAA ANTTGAAATG NAAA 84

SEQ ID NO:1758
SEQUENCE LENGTH:82
SEQUENCE TYPE:nucleic acid
TOPOLOGY:linear
CLONE:HUMGS01977
SEQUENCE DESCRIPTION:
GATCCCACTG TAAAATTGGA AATGGGCAGT TCTGAATTTT CACGTTTGAA ATGTAAAATA 60
TAAACTTCAG TCAATATCCA AA 82

SEQ ID NO:1759
SEQUENCE LENGTH:82
SEQUENCE TYPE:nucleic acid
TOPOLOGY:linear
CLONE:HUMGS01978

SEQUENCE DESCRIPTION:
GATCTGGACG TTCACGTGCA CTCTCTTCCT GTACAGTATT TATTGTTCCT GGCACTTTAT 60
TTAAAGATAT TTGACCCTCA AA 82

SEQ ID NO:1760
SEQUENCE LENGTH:81
SEQUENCE TYPE:nucleic acid
TOPOLOGY:linear
CLONE:HUMGS01979
SEQUENCE DESCRIPTION:
GATCCTTACT CCTGCATTGT TCTTTGCCAG NGNCCTATTT AAAAATTTTA AAATTCTCAT 60
TAAAGTCAGC TTGGGTTTAA A 81

SEQ ID NO:1761
SEQUENCE LENGTH:79
SEQUENCE TYPE:nucleic acid
TOPOLOGY:linear
CLONE:HUMGS01981
SEQUENCE DESCRIPTION:
GATCTAAAGA GAAAAGGGAG AAGAGGGAAA AAGGAAACAA AGGAGAAGAA AGAGGGAAAG 60
GAAAAAAAAG AAAAAGAAA 79

SEQ ID NO:1762
SEQUENCE LENGTH:79
SEQUENCE TYPE:nucleic acid
TOPOLOGY:linear
CLONE:HUMGS01982
SEQUENCE DESCRIPTION:
GATCATGAAA GGTGATAAGC TCTTCTATGA TAGGGGAAGT AGCGTCTTGT AGACCTACTT 60
GCGCTGTTGT ACTCTGAAA 79

SEQ ID NO:1763
SEQUENCE LENGTH:80
SEQUENCE TYPE:nucleic acid
TOPOLOGY:linear
CLONE:HUMGS01983
SEQUENCE DESCRIPTION:
GATCAAATAT TTTGCCTTTN ACTNAAATCA GCCAGCCCAT NACCCCCAAT AAAGGGCAGC 60
TGCCTCTGCT CCCTCTGAAA 80

SEQ ID NO:1764
SEQUENCE LENGTH:79
SEQUENCE TYPE:nucleic acid
TOPOLOGY:linear
CLONE:HUMGS01984

SEQUENCE DESCRIPTION:
GATCAAATTT CTTAAAGCAA ACAACAAAAA TGTACATTTC TGTTTTTCCT TTTAATAAAC 60
AGGTGTACTC TTTATCAAA 79

SEQ ID NO:1765
SEQUENCE LENGTH:78
SEQUENCE TYPE:nucleic acid
TOPOLOGY:linear
CLONE:HUMGS01985
SEQUENCE DESCRIPTION:
GATCTAGTGT CCTTTTTGTA CACTAAACAG GTGAATGCTG ACTTTTTTCA TTCTCCAATA 60
AATNTCACTG AACTGAAA 78

SEQ ID NO:1766
SEQUENCE LENGTH:75
SEQUENCE TYPE:nucleic acid
TOPOLOGY:linear
CLONE:HUMGS01986
SEQUENCE DESCRIPTION:
GATCTTTGTT TAGTTTCAAG TCTTTTTATA ACTAAATCTG CTGGCATTAG ACAATAAATA 60
CATTTTCAAA ATAAA 75

SEQ ID NO:1767
SEQUENCE LENGTH:75
SEQUENCE TYPE:nucleic acid
TOPOLOGY:linear
CLONE:HUMGS01987
SEQUENCE DESCRIPTION:
GATCATATAT ACTTGCATAA TATCATCCCT TCCCTTGATT TCTTTCAATC TAAAAATAAA 60
TATGAGAAAA ACAAA 75

SEQ ID NO:1768
SEQUENCE LENGTH:78
SEQUENCE TYPE:nucleic acid
TOPOLOGY:linear
CLONE:HUMGS01988
SEQUENCE DESCRIPTION:
GATCGATATT AATGTATCCA ATGAAATAAT CGACTTGTNC TTGATAGCCT CATTAAAGCA 60
TTTGGTTTTN CACATAAA 78

SEQ ID NO:1769
SEQUENCE LENGTH:74
SEQUENCE TYPE:nucleic acid
TOPOLOGY:linear
CLONE:HUMGS01989

SEQUENCE DESCRIPTION:
GATCTGAGCC CCCAAGATGG AGAATGGGGA GGAGCTTTTT ATGGCGGACT GATTAAAACT 60
CTTAAGCATT TAAA 74

SEQ ID NO:1770
SEQUENCE LENGTH:72
SEQUENCE TYPE:nucleic acid
TOPOLOGY:linear
CLONE:HUMGS01990
SEQUENCE DESCRIPTION:
GATCTGCTTG GCCTTGATAA GCTATGTTGT TGCACTTTAA ACATTTAAAT TATACAATCA 60
TCAACCCCCA AA 72

SEQ ID NO:1771
SEQUENCE LENGTH:69
SEQUENCE TYPE:nucleic acid
TOPOLOGY:linear
CLONE:HUMGS01991
SEQUENCE DESCRIPTION:
GATCCTCCCC TTCAAATGAC TGTAATTAAC AACACTTAAA AAACTTGAAT AAAATATTGA 60
AACCTCAAA 69

SEQ ID NO:1772
SEQUENCE LENGTH:68
SEQUENCE TYPE:nucleic acid
TOPOLOGY:linear
CLONE:HUMGS01992
SEQUENCE DESCRIPTION:
GATCTATTCT GAAGCTGTTC ATTTTTAACA AATAAAATGT TACAGGTTTC ACATGATTTA 60
TTCTCAAA 68

SEQ ID NO:1773
SEQUENCE LENGTH:66
SEQUENCE TYPE:nucleic acid
TOPOLOGY:linear
CLONE:HUMGS01994
SEQUENCE DESCRIPTION:
GATCCGTAAC TNCGGGATAA GGATTGGCTC TAAGGGCTGG AGAAGGAATG AATAAAAAAA 60
AAGAAA 66

SEQ ID NO:1774
SEQUENCE LENGTH:67
SEQUENCE TYPE:nucleic acid
TOPOLOGY:linear
CLONE:HUMGS01995

SEQUENCE DESCRIPTION:
GATCCCATGT CTCTGCCAGG ACCAAGGCNG ATGTTTCCCC ACTAATAAAG TGCCGGGTGT 60
CAGCAAA 67

SEQ ID NO:1775
SEQUENCE LENGTH:65
SEQUENCE TYPE:nucleic acid
TOPOLOGY:linear
CLONE:HUMGS01996
SEQUENCE DESCRIPTION:
GATCCTTTTT AATACCACAG CATTTGTACT GTTCCTTTTT AATATACTGA AAATATAAAA 60
GGAAA 65

SEQ ID NO:1776
SEQUENCE LENGTH:71
SEQUENCE TYPE:nucleic acid
TOPOLOGY:linear
CLONE:HUMGS01997
SEQUENCE DESCRIPTION:
GATCCGATTA TTTCTGCCAG TNTATTTTGG NCACTTTATA ATCATTAAAG CACTTTCTTG 60
GCAGGCNCAA A 71

SEQ ID NO:1777
SEQUENCE LENGTH:72
SEQUENCE TYPE:nucleic acid
TOPOLOGY:linear
CLONE:HUMGS01998
SEQUENCE DESCRIPTION:
GATCTTACAA TAATGTTCCA CTCTGCAAAT TTTTAAGGTT CAAATAAAGT TTAATTGTTT 60
GCAAACTGTA AA 72

SEQ ID NO:1778
SEQUENCE LENGTH:65
SEQUENCE TYPE:nucleic acid
TOPOLOGY:linear
CLONE:HUMGS01999
SEQUENCE DESCRIPTION:
GATCATTGCA CCATGTCAGA CTTTTGTATA TNCCTTGAAA ATAAATGAAA GTNAGAATCN 60
TCAAA 65

SEQ ID NO:1779
SEQUENCE LENGTH:62
SEQUENCE TYPE:nucleic acid
TOPOLOGY:linear
CLONE:HUMGS02000

SEQUENCE DESCRIPTION:
GATCCTGTCC TGATTCTCAA AAATTATTTT CTCTGTATGA TTAAAAGTTT ATTCCATTTA 60
AA 62

SEQ ID NO:1780
SEQUENCE LENGTH:66
SEQUENCE TYPE:nucleic acid
TOPOLOGY:linear
CLONE:HUMGS02001
SEQUENCE DESCRIPTION:
GATCCCTTCA TTTGAATATT AATGGCTTTT TCCATTAAAG AATAAAATAT TTTGGACAAT 60
GCCAAA 66

SEQ ID NO:1781
SEQUENCE LENGTH:68
SEQUENCE TYPE:nucleic acid
TOPOLOGY:linear
CLONE:HUMGS02002
SEQUENCE DESCRIPTION:
GATCAAATTT ATAAGTTATT ATTTTTAATT TTCTAAATTA AATAAAAGAA AGAATGCAAA 60
CCAGGAAA 68

SEQ ID NO:1782
SEQUENCE LENGTH:59
SEQUENCE TYPE:nucleic acid
TOPOLOGY:linear
CLONE:HUMGS02003
SEQUENCE DESCRIPTION:
GATCAATAAA GAGTAATTTC TTGCTAAATA AATAAAAGAA ACCTTGTTGA AAAACTAAA 59

SEQ ID NO:1783
SEQUENCE LENGTH:59
SEQUENCE TYPE:nucleic acid
TOPOLOGY:linear
CLONE:HUMGS02005
SEQUENCE DESCRIPTION:
GATCTGAATN ATGCACCCCA CCCCCACCCC AATAAAGAAA TAACAGAAAA CCCTCGAAA 59

SEQ ID NO:1784
SEQUENCE LENGTH:58
SEQUENCE TYPE:nucleic acid
TOPOLOGY:linear
CLONE:HUMGS02006
SEQUENCE DESCRIPTION:
GATCTTCATT TTTTTTTTA AACTTGGCGT TTTTTGAATA AAAACCTTTT GTCTTAAA 58

SEQ ID NO:1785
SEQUENCE LENGTH:59
SEQUENCE TYPE:nucleic acid
TOPOLOGY:linear
CLONE:HUMGS02007
SEQUENCE DESCRIPTION:
GATCTCAGTT GTAAATAGAA AAATCTAATT CAATAAACTC TGTATCAGCC CCCAACAAA 59

SEQ ID NO:1786
SEQUENCE LENGTH:52
SEQUENCE TYPE:nucleic acid
TOPOLOGY:linear
CLONE:HUMGS02010
SEQUENCE DESCRIPTION:
GATCCAAAGT CATGTGTCCA CACATTCATA AATAAAAATT TTACCTATGA AA 52

SEQ ID NO:1787
SEQUENCE LENGTH:51
SEQUENCE TYPE:nucleic acid
TOPOLOGY:linear
CLONE:HUMGS02011
SEQUENCE DESCRIPTION:
GATCCAGTGA AGCCAGAAGC CAAATAAACT CAAAAGCTGT CTCCCCACAA A 51

SEQ ID NO:1788
SEQUENCE LENGTH:54
SEQUENCE TYPE:nucleic acid
TOPOLOGY:linear
CLONE:HUMGS02012
SEQUENCE DESCRIPTION:
GATCCTAATT TATGCCTATG CAAAAAATAA ATAACGCCCA AGAGCTGTGG GAAA 54

SEQ ID NO:1789
SEQUENCE LENGTH:53
SEQUENCE TYPE:nucleic acid
TOPOLOGY:linear
CLONE:HUMGS02013
SEQUENCE DESCRIPTION:
GATCGCCTAG TATGTTCTGT GAACACAAAT AAAATTGATT TACTGTCTGC AAA 53

SEQ ID NO:1790
SEQUENCE LENGTH:58
SEQUENCE TYPE:nucleic acid
TOPOLOGY:linear

CLONE:HUMGS02025
SEQUENCE DESCRIPTION:
GATCCGTAGT AATAAATTCT CAGAGGACTC AGCCTTTCCT GCGGGTCTGG AGCTCAAA 58

SEQ ID NO:1791
SEQUENCE LENGTH:597
SEQUENCE TYPE:nucleic acid
TOPOLOGY:linear
CLONE:HUMGS02039
SEQUENCE DESCRIPTION:
GATCAGAGTC TCTTGGGCAT TTTATATTTT GCATTCTGAT GTACCTAGGA GTTTTGTTAA 60
ACAGATGATG TATGTNAGTA TTTATCCCAT TTTATGCAAT TAACCAAATC AACCAAAAAA 120
AGTGACCATG AAGTCCTGTA TTTGTNTTTT TACTACATGT AGGAACTCTC ATGTGAATGA 180
GTACTGTAGT AATCCATTCT ATGGGAGCCT TATTTCAGAA ATATTTCAAA CTGGTGCAAA 240
TGGAAAAGAC TTTCTCTTTT CCTTTAAAGC TAAAGACAAG AATATCATGC TATACAGGTG 300
CAACTCAATC CCCGTTAATA AAAACCAATG TAGGTATAGG CATTCTACCC TTTGAAATAG 360
CTGTGTCCCA ACCTGTTGCC ATTGATTTTT TGGGAATGGC TTTAGGAAAT ATCCAAGTTG 420
TCCTTGGAAT TGTCTAACCA TGGNCATAAA CAGTTGTCTC CCTTCTACTT GTGTAGAATA 480
CTTTGGNCTT AATTTTCCTN CCAGATACAG GGGGGTACCT GCCTGTTTTT CNAAGGGTTT 540
ATTACTGGNG GTACCATTTG GTAGGATTGT TTTAAATAAA AAAACCTGGT TTTTAAA 597

SEQ ID NO:1792
SEQUENCE LENGTH:518
SEQUENCE TYPE:nucleic acid
TOPOLOGY:linear
CLONE:HUMGS02040
SEQUENCE DESCRIPTION:
GATCCAAAAC TGGACCCTTG GAGCCGTCGA CTCACAGATG GATGACATGG ACATGGACTT 60
AGACAAGGAA AAAANNCAGG ACTTGAAGGA GCTCAAGGTG CTAGTGGCTG ACAAGGACCT 120
TCTGGACCTG CACAAGAGCC TGGTGTGCAC TGCTCTCCGG GGAAAGCTGG GCGTCTTCTC 180
TGAGATGGAA GCCAACTTCA AGAACCTGTC CCGGGGGCTG GTGAACGTGG CCGCCAAGCT 240
GACCCACAAT AANGATGTCA GAGACCTGTT TGTGGACCTC GTGGAGAAGT TTGTGGAACC 300
CTGCCGCTCC GACCACTGGC CACTCAGCGA CGTGCGGTTC TTCCTGAATC AGTATTCAGC 360
GTCTGTCCAC TCCCTCGATG GCTTCCGACA ACAAGGCCCT CTGGGACCGC TACATGGGCA 420
CCNTTCCGNG GCTGNCTNCT GCGGCCTGTN ATCATGACTG AGGTGGCCTN CCAAANGTTN 480
CGCCCAAGGT TGACAATAAA GTTGCTCTGA GTTTGAAA 518

SEQ ID NO:1793
SEQUENCE LENGTH:513
SEQUENCE TYPE:nucleic acid
TOPOLOGY:linear
CLONE:HUMGS02041
SEQUENCE DESCRIPTION:
GATCTGTAAT TTTAATTTTC ACCCTCTGTA CCCCATGACC TTATCCTTCC TCTCCTTCCT 60
TGTTACCCAT GAAAAACTGG CAACATTCCA AGAATAGCAT CTGTACAAAG GGGAAAGAAC 120

ATAAAGGTAA AACAAAACAA AACAACATTT TGAGAACAAA GATGACCATA ACCACTGAAG 180
GGAATCACAT CTTTTAAGAC AAATTCATAT TCTTTTATTT GTTATGGCAG ATGACAAGAT 240
GGTACAACCT TTATTCTTTT NCAAAATAAA ACAAAGGGCA CAGCATCTGT AGTCAGCCGA 300
CAACTATTNC GGCCTTTTGG GGGTNGGTCT NGCCGTACTT GTGATTTCGA TGGTACGTGA 360
CCNTNTTCTG GAAGGCTTGC CCCNTTGCCC GTGTACATAG GTGCATTGGT TNCTNTGGGA 420
GGGGCCCAAG NACTTTTNGG CNANNGTTGT ACTGGTATTG TNATGAAATT TNCGGTTGGN 480
CCTCTTGNNA NTTTTTTNTG CANGAANGNN GGN 513

SEQ ID NO:1794
SEQUENCE LENGTH:499
SEQUENCE TYPE:nucleic acid
TOPOLOGY:linear
CLONE:HUMGS02042
SEQUENCE DESCRIPTION:
GATCTCTTAA GAGATGATGG ACTCATGTAT GTCACCCGAC TTCGCAACAC TGGGGTTCAG 60
GTGACTCATA ACCATGTTGA GGATGGATTC CATGGAGCAT TTTCATTTCT GGGACTTAAA 120
ATTAGTCACA GACTTATAAA TCAGTATATT GAGTGGCTAA AGGAAAATCT ATAGTAAAAC 180
ATGTAGCTAT AACATATTTT AAAAATAAAA TCTGAAAACC TCAGAAAATT TGCATTAGAA 240
ATTGGTCTTT CTTAGAATGG TCTAGTTAAG TTCCACATGT AGCATAATTC TTAAATAGGC 300
ACTTTTCTGT TTTTTTTTNC TNACTGTGGG ATTTCATNNC AATTTTCTAC ATTGTCTATC 360
TGCTTTTNCT GNGATTTTCC NCTTACACTG TTAATCTNAT TTTAAAAAAA TGTACATNCC 420
NNGNANACNT TAANTTTGGG GAGTTNGGCT ACNANTTNAC GGTTGCCAGG GGGNTTAAAT 480
GGTGGGGCCA ATTTTTAAA 499

SEQ ID NO:1795
SEQUENCE LENGTH:483
SEQUENCE TYPE:nucleic acid
TOPOLOGY:linear
CLONE:HUMGS02043
SEQUENCE DESCRIPTION:
GATCAAAGCG CCACTGCCCT GCACTTCCTG GGCCGCGTGG CAACCCGCTG NGCACAGCAT 60
GAGGCCAGGG NCCCAGAACA CAGTGCCTGG CAAGGCCTCT GCCCNTGGCC TTTGAAANAA 120
AGGCCAGCAG CAGATAACAA CCCCGGACAA ATCAGCGATG TGTCACCCCC AGTCTCCCAC 180
CTTTTCTTCT AATGAGTCGA CTTTNNGCTG GAAAGCAGCC GTTTCTCCTT GGTCTAAGTG 240
TGCTGCATGG AGTGAGCAGT AGAAGCCTGC AGCGGCACAA ATGCACCTCC CAGTTTGCTG 300
GGGTTATTTT AGAGAATGGG GGTGGGGAGG CAAAGAACCA GTGTTTAGCG CGGGACTACT 360
GTTCCAAAAA GANTTNCAAC CGNCCAGNTT GTTTGTGAAA CAAAAAAGTG TTNCCTTTTT 420
CAAGTTTGAG AACAAAAATT GGGGTTTTAA AATTAAAGTA TACCATTTTT TGCATTTGNC 480
AAA 483

SEQ ID NO:1796
SEQUENCE LENGTH:481
SEQUENCE TYPE:nucleic acid
TOPOLOGY:linear
CLONE:HUMGS02044

SEQUENCE DESCRIPTION:
GATCCAAAAC GCTTCGAGGC ACCCCAAATT ACCTGCCCAT TCGTCAGGAC ACCCACCCAC 60
CCAGTGTTAT ATTCTGCCTC GCCGGAGTGG GTGTTCCCGG GGGCACTTGC CGACCAGCCC 120
CTTGCGTCCC CAGGTNNNNA GCTCTCCCCT GGGCCACTAA CCATCCTGGC CCGGGCTGCC 180
TNGTCTGACC TCCGTGCCTA GTCGTGGCTC TCCATCTTGT CTCCTCCCCG TGTCCCCAAT 240
GTCTTCAGTG GGGGGCCCCC TCTTGGGTCC CNTCCTCTGN CATCANCTGA AGACCCCCAC 300
GGCAAACACT GAATGTCACC TGTGNCTGCN GNCTCGGTCC ACCTTGCGGG CCGTGTTTGA 360
CTAAACTTNA ACTTCTTTTA ACGGTAATAT TTCCGGCAAA AATCCAATGG TTTGGGTTTT 420
GGTCTTTTAA CNTTGTAAAC GGTTGCAAAT CCAAATAAAA GCAATTAAAA AGTCAATGAA 480
A 481

SEQ ID NO:1797
SEQUENCE LENGTH:462
SEQUENCE TYPE:nucleic acid
TOPOLOGY:linear
CLONE:HUMGS02045
SEQUENCE DESCRIPTION:
GATCTAAAAG GGTTTTCTTA GAAAGGGCAA TATTGTCCAA TGAAGTAAGC AGAAGGACTC 60
TGGGTTAGAA GCATCTGCAC AAAAACTGGT GAGACCTACT CTCCACTGCT CTGCAGCTGG 120
ATGGCTGATG GCAGGCTGAG CAGTGGGGAA GCAGGTTTTA ACAACAGGGA GTCCTTCCAG 180
GTCACTGTAT ATTGNGAAGA AACATAAAAC TATTGTCTGT TACATTCCGA GGTCAGCCTT 240
CTNCTTAACG TTTTATAATA TGCAAATNCC AGCTTCTGGA AAGCANGTAT CATCATGTAC 300
CAAATGCTTT ATACACCATC ACATTCATGA NTTTTTNAGC ATGGTCAGAN CTTGTGTAAA 360
TATGTCTCTT AGGATGATTT TGGGGGAGGA TGTGATTTAT TTTCCATAAT TTCCAAANTN 420
GCATTTTCCA TTTCAAAATA AGGNTAATCT NTTGGGGCCA AA 462

SEQ ID NO:1798
SEQUENCE LENGTH:448
SEQUENCE TYPE:nucleic acid
TOPOLOGY:linear
CLONE:HUMGS02046
SEQUENCE DESCRIPTION:
GATCAGCCTG CAGCCATCCC CCGACCCTGC TGCTAGGCCA CGGTCTGCGC CCTGGGGCCT 60
CATCTCCCCC AGCCCACTTG TTTTCCCCCC TTTTATTCCC TAGGCCCTTT TCAGACTCCT 120
GGGCCCTTGG NNNCTCTTCT CCCATCTCCC TTCACAGGAT GACACCCTCC CATACCCCAT 180
AGCTGGGGCC AGCAGGTTCT GCTGAGGGTG GGGCTGGTGT AGGGACCCCC AAGAGACCCC 240
TGTCCTGTCC CTTCACCAGT CCTGGGGAGG CTGGGACTCC CCCTGCCACA AGCCTGGGCC 300
ACAGTTCACA TTCCACTGCT GGGAGAAGAA ACAGGCCGAG GCCCAANAGT GGCCTGCCCC 360
CGGGAGCCAA AGACCCNAGT GGCCACANTG GGATTAGGGT NGGGGAGGNT TGGNAGCGCT 420
NTTTTTATAA ATATTATACA TAAGGAAA 448

SEQ ID NO:1799
SEQUENCE LENGTH:448
SEQUENCE TYPE:nucleic acid
TOPOLOGY:linear

CLONE:HUMGS02047
SEQUENCE DESCRIPTION:
GATCTGGTTG GTGGATGAAC AAGTGTCACG CTGGCCATCT CAATGGNGTT TATTACCAAG 60
GTGGCACTTA CTCAAAAGCA TCTACTCCTA ATGGTTATGA TAATGGCATT ATTTGGGCCA 120
CTTGGAAAAC CCGGTGGTAT TCCATGAAGA AAACCACTAT GAAGATAATC CCATTCANCA 180
GACTCACAAT TGGAGAAGGA CAGCAACACC ACCTGGGGGG AGCCAAACAG GCTGGAGACG 240
TTTAAAAGAC CGTTTCAAAA GAGATTTACT TTTTTAAAGG ACTTTATCTG AACAGAGAGA 300
TATAATATTT TTCCTATTGG ACAATGGACT TGCAAAGCTT CACTTCATTT NNNNGAGCAA 360
AAGACCCCAT GTTGGAAAAC TCCATACCAG TTTTATGCTG ATTGATAATT TATCTACATG 420
CATTTCAATA AACCTTTTGT TTCCTAAA 448

SEQ ID NO:1800
SEQUENCE LENGTH:432
SEQUENCE TYPE:nucleic acid
TOPOLOGY:linear
CLONE:HUMGS02048
SEQUENCE DESCRIPTION:
GATCTGAATT ATCTGGCACC CTCCTGAATG GAACCCCAGA GTACCTCCTG TGTGGAAGGG 60
TCCTGGATTT TCCCTAACAC CCACCCTCTC CCCCTTCAGC CATGCTGATG GCAGAGAAGA 120
TAAGAACTTG GAGCCCATTT CTCACTGGAG AGGAAAACTT GTCATCTGGC TTTGCGGAGA 180
AGGTTCCACC TTACGCTCGT AGTACATTAT CTTTACTATG TGCTAGGATA TCATATTTAA 240
AAGGACAAAA AAATGTAAAA TACTTGAATG AGCTTGTATT ATANCATTAA TATTATNNNG 300
NGTATCTGCT TTCCAGGCTG AAGTGATTCA TTCATTATTC TAGTCCTGCT TTAGTCCTTT 360
GTAATTTGTG GTAATTATGC TTTTCTTTTT AATACAAAAA AATGTATAAA ATTAAACACT 420
TGAAAAGGCA AA 432

SEQ ID NO:1801
SEQUENCE LENGTH:430
SEQUENCE TYPE:nucleic acid
TOPOLOGY:linear
CLONE:HUMGS02049
SEQUENCE DESCRIPTION:
GATCCGNGCG AGGGCAACAG CGGNTCACCT ACAGCGCACT GTCGATGGCT GCACGAGTCA 60
CACCGGAGCC TGGGGCAAGA CAGTGATTGA ATACAAAACC ACCAAGACCT CCCGCCTGCC 120
CATCATCGAT GTGGCCCCCT TGGACGTTGG TGCCCCAGAC CAGGAATTCG GCTTCGACGT 180
TGGCCCTGTC TGCTTCCTGT AAACTCCCTC CATCCCAACC TGGCTCCCTN CNACCCAACC 240
AACTTTNCCC CCAACCNGGG AAACAGACAA GCAACCCAAA CTGAACCNNN TCAAAAGCCA 300
AAAANTNGGG AGACAATTTC ACATGGACTT TGGAAAAATA TTNTTTTCCT TTGCATTCAT 360
NTNTCAANCT TAGTNTTNTA TCTTTGANCA ACCGGANCAT GGCCAAAAAN CAAAAGGTGC 420
ATTCANCCTN 430

SEQ ID NO:1802
SEQUENCE LENGTH:428
SEQUENCE TYPE:nucleic acid
TOPOLOGY:linear

CLONE:HUMGS02050
SEQUENCE DESCRIPTION:
GATCCCAAGG GAAAGATGAT TTGTATGTTA AAGTGACTGC ACAAGTAAAA GTCCAATGTT 60
GTGTGCATGA AAAGGATTCC TTGGTTATGT GCAGGGAATC ATCTCACATG CTGTTTTTCC 120
TATTTGGTTT GAGAAACAGG CTGACACTAT TCTCTTTGAT TAGAAAATAA ACTCATAAAA 180
CTCATAATGT TGATATAATC AAGATGTAAC CACTATAAAT ATGTAGAAGA GGNCGTTTTA 240
AAAGACCTTA AGCTGGCATT GTGAAGGAAC ACCATGGTAG ACTCTTTTTG TAAATGTATT 300
TTGTATTTAA TGAAATGCAG TATAAAGGTT GGTGANGTGT AATATANTTG TGTAAACAAA 360
TCCTGTTAAT AGAGAGATGT ACAGAATCGT TTTGGTACT GTATCTTGGA AACCTTTGTG 420
AAAATAAN 428

SEQ ID NO:1803
SEQUENCE LENGTH:401
SEQUENCE TYPE:nucleic acid
TOPOLOGY:linear
CLONE:HUMGS02051
SEQUENCE DESCRIPTION:
GATCAAAACC AAACTGTTCA ATGTGACAGT CTGAAAATTA TATACTCTAC TTCATTCATG 60
TTACACTTTT TTCTACTCCT TACCTGCTTT TACTAATGCA TTTATCCCCA AGAAAACATC 120
CAACAATATG GGACTAATTT GGAACAATAT CCAGCAATTG GTATTTAAAT AATTTACTGG 180
TTACAAAACA ATATTATGAC CAGACTCCAT TTTAGAAAAA ATTAAATCTG CATCGAAAAG 240
GGCCTTAAAA AATAGATTTC TAAAATCAAA ATCATGGTTG GCTCATGGGT GGGATTATTA 300
GTGTTCCTTA TAGTAGTTTG TCTGGATGTA CTTTCTGATT CTCCTGTAAT AAGCATGCAT 360
TGCTTTTGTA ATGNGGGAAT AATANACCAA TAAAAAGGAA A 401

SEQ ID NO:1804
SEQUENCE LENGTH:396
SEQUENCE TYPE:nucleic acid
TOPOLOGY:linear
CLONE:HUMGS02052
SEQUENCE DESCRIPTION:
GATCCGGCTG CAAAATTCAT ACCAACCAAC AAATAAAGGA AGATACAAAG TCTTATAGAC 60
ATCCGGAAAA AAGATTTTTA CCTGTGCTGG TCTATGATGT ATGTGGCAGT TGCTGTCTGC 120
AGTTTACAAT GTATTGTAAA TGAAGATTTT TTAAATTCTA TCTTGCTGNT TNNNNTNNAA 180
ATATAAGAAA CTGGTACTTG GTAAAGAAAT CTGTCCGTAA GTACCCCCAC AATCAGTCAA 240
ACTATATTTA AAGCCAGCCT GTTTTCAGAG TATGATGTCC TTTAATGTAA ACTCAAATAT 300
CAATATTTTA AATGTCCGGA TAATATTCTA GAGGTTTAAA AAATGGAAAT ATTTGANCTT 360
TCTATTGAAG ACAATAAAGG TACACAAGTC GTTAAA 396

SEQ ID NO:1805
SEQUENCE LENGTH:392
SEQUENCE TYPE:nucleic acid
TOPOLOGY:linear
CLONE:HUMGS02053
SEQUENCE DESCRIPTION:

GATCAAGAGC AGTGCTTCTC CATTTGTTTT GCAGAGAAAT GTTTTTCATT TCCCGTGTGT 60
TTCCATTTCC TTCTGAAATT CTGATTTTAT CCATTTTTTT AAGGCTCCTC CCCATCTCCT 120
TTCTTAAGGC ACTGTTGCTA TGGCACTTTT CTATAACCTT TTCATTCCTG TGTACAGTAG 180
CTTAAAATTG CAGTGATTGA GCATAACCTA CTTGTTTGTA TAAATTATTG AAATCCATTT 240
GCACCCTGTA AGAATGGACT TAAAAGTACT GCTGGACAGG CATGTGTGCT CAAAGTACAT 300
TGATTGCTCA AATATAAGGA AATGGCCCAA TGAACGTGGT TGTGGGAGGG GAAAGAGGAA 360
ACAGAGCTAG TCAGATGTGA ATTGTATCTG TN 392

SEQ ID NO:1806
SEQUENCE LENGTH:389
SEQUENCE TYPE:nucleic acid
TOPOLOGY:linear
CLONE:HUMGS02054
SEQUENCE DESCRIPTION:
GATCCTATTT GGTATGTTTT TGTCCACTGT TATGATTCAT CATGTATCTT ACAAGAGCCA 60
CTCAAGCAAG ACTCTGCTTC TATGTATGGT GAGGCCTTGT TGTTCTAGGC TAGAATAAAC 120
TCTTTGTATG CCTCAGTGAA TATGCCAGGT AAAATATATG CAGTCAAGAA TGAATTATTT 180
TTCTGACTAA AGTGTGTAGC AGTAAGTTCA AAATNGTGCC CTTGTTTTAA CAGTTTCTGT 240
CAACATCTTC TCATTTTTCC CTACAAAAAC ACCAGGGTGT ATTATAAGTA CTGCCTGTGA 300
GAATTTGCAC TTTATGTATT TGTGTGTGGG ATTTCTTGTG GTTTTAGCCA AATGAAGTGT 360
TATCNNTAAT AACCAGGTCT CTTCATAAA 389

SEQ ID NO:1807
SEQUENCE LENGTH:388
SEQUENCE TYPE:nucleic acid
TOPOLOGY:linear
CLONE:HUMGS02055
SEQUENCE DESCRIPTION:
GATCCAATTG ATGTATATGT TTATATACTG GGTTCTTGTT TTATATACCT GGCTTTTACT 60
TTATTAATAT GAGTTACTGA AGGTGATGGA GGTATTTGAA AATTTTACTT CCATAGGACA 120
TACTGCATGT AAGCCAAGTC ATGGAGAATC TGCTGCATAG CTCTATTTTA AAGTAAAAGT 180
CTACCACCGA ATCCCTAGTC CCCCTGTTTT CTGTTTCTTC TTGTGATTGC TGCCATAATT 240
CTAAGTTATT TACTTTTACC ACTATTTAAG TTATCAACTT TAGCTAGTAT CTTCAAACTT 300
TCACTTTGAA AAATGAGAAT TTTATATTCT AAGCCAGTTT TCATTTTGGT TTTGTGGTTT 360
TGGGTTAATA AAACAATACT CAAATAAA 388

SEQ ID NO:1808
SEQUENCE LENGTH:379
SEQUENCE TYPE:nucleic acid
TOPOLOGY:linear
CLONE:HUMGS02056
SEQUENCE DESCRIPTION:
GATCTTTTCC ACCAGCACCC AGAGTGTCCT CTTTCTGGGC AAGGTCGTCG ACCCCACGAA 60
ACCATAGCCC TCCCAGGGCT GCTCATCTGT TCCAAGCAGG AGGATGTGGC AGGGGAGGGC 120
TGGGAGTGAG TAGCTCTGTG TTTAGAGTTG GGGACAAGGA TGACACCGAA GTCCAGGAGT 180

CCAGGACAGC AGGTGCTGGC CGGTGGGGAG CGGGGAGGGG CACTGAGATG GGCAGGGCCT 240
GGACATTCCA CACCCTGGTG CTGTGCAGCC TCTGGCAGAG CATCCGACCT CTTGGAGCAA 300
GTTTCTGCCT CTGGAAAGGG GGCGGGCCCT TTTCACAACA GGCTGGTTTG TACCGAGTAA 360
ACAACACGNT GCCATGAAA 379

SEQ ID NO:1809
SEQUENCE LENGTH:379
SEQUENCE TYPE:nucleic acid
TOPOLOGY:linear
CLONE:HUMGS02057
SEQUENCE DESCRIPTION:
GATCCTAGGG GGTTTTGTTT GGTTTTNAAT TGTGAGGAAT AAAAAATNTT CTGCCCACAC 60
TGGCATTTTA AGGTGACTGA GGTCAAACGT TGTTTCCTTA GGTTGAAATA GCAGCCAAAA 120
CATTCTTNAC GCAGGGGCTT GGGATATGGC TGCTGGCAAC ACATTTTGTT GTGGGCTCCT 180
TAATTTAATG ATAAAATTTA AGCTAAACAC AAGCCAAAAA TGAATAGGTT TTTTNAATTT 240
TNATTTTTCA CTAAACAGGC AATTGAAATA CATGGTACAA AAATAAGTGG TAAGATAATT 300
GTAAAATGGA AATGGGCAGA NTATTCAATT TTCCATCTAT GNAAATTTTC NCATTAAAAC 360
TCATAGNTTT CTTTGNAAA 379

SEQ ID NO:1810
SEQUENCE LENGTH:358
SEQUENCE TYPE:nucleic acid
TOPOLOGY:linear
CLONE:HUMGS02058
SEQUENCE DESCRIPTION:
GATCNGTTCA GCCCTGAAAA GTCCAAACTC CCAGGTATTG TTGCAGAAGG NCGAGATGAC 60
CTCTATAAAT CAGATGCATT CCATAAGGCA TTTNTTGAGG TAAATGAAGA AGGCAGTGAA 120
GCAGCTGCAA GTACCGCTGT TGTGATTGCT GGCCGTTCGC TAAACCCCAA CAGGGTGACT 180
TTCAAGGCCN CCNGGCTTNA AAAAGTTTTT ATAAGAGAAG TTCCTCTGAA CACTATTATC 240
TTCATGGGCA GAGTAGCCAA CCCTTGTGTT AAGTAAAATG TTCTTATTCT TTGCACCTCT 300
TCCTATTTTN GGTTTGTGAA CAGAAGTAAA AATAAATACA AACTNNTNNC ATCTCAAA 358

SEQ ID NO:1811
SEQUENCE LENGTH:358
SEQUENCE TYPE:nucleic acid
TOPOLOGY:linear
CLONE:HUMGS02059
SEQUENCE DESCRIPTION:
GATCGTTTCT NATCTCNAAG TNCCTCATTG AGNTCGGTGC ATCTGGCCAN TGAGTCTGCT 60
GAGACTCTTG ACAGCACCTC CAGCTCTGCT GCTTTCAACA ACAGTGACTT GCTCTCCAAT 120
GGTATCCAGT GATTCGTTGA AGAGGAGGTG CTCTGTAGCA GAAACTGAGC TCCGGGTGGC 180
TGGTTCTCAG TGGTTGTCTC ATGTCTCTTT TTCTGTCTTA GGTGGTTTCA TTAAATGCAG 240
CACTTGGTTA GCAGATGTTT AATTTTTTTT TTAACAACAT TAACTTGTGG CCTCTTTCTA 300
CACCTGGAAA TTTACTCTTN GAATAAATAA AAACTCGTTT GNCTTTGNCT TCTGCAAA 358

SEQ ID NO:1812
SEQUENCE LENGTH:344
SEQUENCE TYPE:nucleic acid
TOPOLOGY:linear
CLONE:HUMGS02060
SEQUENCE DESCRIPTION:
GATCTATACT AGATAATCCT AGATGAAATG TTAGAGATGC TATATGATAC AACTGTGGCC 60
ATGACTGAGG AAAGGAGCTC ACGCCCAGAG ACTGGGCTGC TCTCCCGGAG GCCAAACCCA 120
AGAAGGTCTG GCAGNNNTCA GGCTCAGGGA GACTCTGCCC TGCTGCAGAC CTCGGTGTGG 180
ACACACGCTG CATAGAGCTC TCCTTGAAAA CAGAGGGGTC TCAAGACATT CTGCCTACCT 240
ATTAGCTTTN CTTTATTTTT TTAACTTTTT GGGGGGAAAA GTATTTTTAA GAAGTTTGTC 300
TTGCAATGTA TTTATAAATA GTAAATAAAG TTTTTACCAT TAAA 344

SEQ ID NO:1813
SEQUENCE LENGTH:338
SEQUENCE TYPE:nucleic acid
TOPOLOGY:linear
CLONE:HUMGS02062
SEQUENCE DESCRIPTION:
GATCCAGTAG TAGAGACATC ATCGGTCCAA AAGACTTTGC ATCTCATTAG TTGTCCATAG 60
TGACTACCAG GAGGTGGCTG GCATCTATAA CGTGTGGGAC GTTTTTATGT AAAGAACTGC 120
CTGTGTTTTT TTTTTNAACC AAAGACACTG NCCATAGATT GACTTATACT TTTATAAGTC 180
TAATTGAAAT ACTTGGGGTA CTATGAAAGG NCTGTNCCTT GNGTGGCTAC GATTAGAAAA 240
TCAGGNCTAA CAAACTTTTC TTTGTCTGGA NGCATATATA TTTGTGATAT AATTTNAATA 300
TGTATAATTT TGGTTTAATA AATCAAATCT ATGCCAAA 338

SEQ ID NO:1814
SEQUENCE LENGTH:336
SEQUENCE TYPE:nucleic acid
TOPOLOGY:linear
CLONE:HUMGS02063
SEQUENCE DESCRIPTION:
GATCCATAGA ATGGATACTG GGAGACAATT AGTAGACTTT GCTACAGGTA GCCAATAGCC 60
CAGCCTGCAG AGTCACGTCA GATATGAAAT CCCAAAGTTC CTCCTGACTA TTGGCAACAA 120
CAATCACTAA CTGCTACTAC AAAAAGGTAA AACAAAAATG AACTTACACT TTCAAAATAT 180
CATTCATGTA TATTAAACAA ATACTTCTTG AGTGTCTACC CAGTGCCAGA CAGTGGTCTA 240
GGTTCTGGGA GGTACAGCAT CGTGGGCCAC ATGGTTAGAA GCTGAGTGTT CCTAACAAGG 300
AGGCTGATTT TGCTGCTCTA CTTTGCACTG GTTAAA 336

SEQ ID NO:1815
SEQUENCE LENGTH:335
SEQUENCE TYPE:nucleic acid
TOPOLOGY:linear
CLONE:HUMGS02064
SEQUENCE DESCRIPTION:

GATCTGGATG TGGATGATGC GCCTGGAAAC AGTCAGCAGG CAACTCCGAA GACAACGAGA 60
TAAGCACCTT TCACAACCTC GGGAACGTTC ATTCCCCGCT GAAGCTTCTC ACCAGCATGG 120
CCATCTCGGT GGTGTGCTTC TTCTTCCTGG TGCACTGACT GCCTGGTGCC CAGCACATGT 180
GCTGCCCTAC AGCACCCTGT GGTCTTCCTC GATAAAGGGA ACCACTTTCT TATTTTTTTC 240
TATTTTTTTT TTTTNGTAAN CCGGNANACC TCCTCCAGCC ATGAGGNNGG GGNNTANCCA 300
NGTGTNATGT TTNGGAAANN NAAATGGNAT CTTTN 335

SEQ ID NO:1816
SEQUENCE LENGTH:335
SEQUENCE TYPE:nucleic acid
TOPOLOGY:linear
CLONE:HUMGS02065
SEQUENCE DESCRIPTION:
GATCCATGTG TAATATCACA AGAAATTATG GAAAAATATA ACATAAAATT AAAGTGGACA 60
AACCAACAAA AGCTTTATTC AAGAACAGGT GACATAGTTG AATTTGTTTG TAAATCTGGA 120
TATCATCCAA CAAAATCTCA TTCATTTCGA GCAATGTGTC AGAATGGGAA ACTGGTATAT 180
CCCAGTTGTG AAGAAAAATA GAATCAATGG CATTACTATT AGTAAAATGC ACACCTTTTT 240
CTGAATTTAC TATTATATTT GTTTTCAATT TCATTTTTCA AGTACTGTTT TACTCATTTT 300
TATTCATAAA TAAAGTTTTG TGTTGATTTG TGAAA 335

SEQ ID NO:1817
SEQUENCE LENGTH:334
SEQUENCE TYPE:nucleic acid
TOPOLOGY:linear
CLONE:HUMGS02066
SEQUENCE DESCRIPTION:
GATCTTGATG CGGGACTTCA GTCCTAGTGG CATTTTCGGA GCGTTTCAAA GAGGTTACTA 60
CCGNNACTAC AACAAGTACA TCAATGTGAA GAAGGGGAGC ATCTCGGGGA TTACCATGGT 120
GCTGGCATGC TACGTGCTCT TTAGCTACTC CTTTTCCTAC AAGCATCTCA AGCACGANCG 180
GCTCCGCAAA TACCACTGAA GAGGACACAC TCTGCACCCC CCCACCCCAC GACCTTGGCC 240
CGAGCCCCTC CGGNNGGAAC ACAATCTCAA TCGTTGCTGA ATCCTTTCAT ATCCTAATAG 300
GAATTAACCT CCAAATAAAA CATGACTTGG TAAA 334

SEQ ID NO:1818
SEQUENCE LENGTH:328
SEQUENCE TYPE:nucleic acid
TOPOLOGY:linear
CLONE:HUMGS02067
SEQUENCE DESCRIPTION:
GATCCAAACA TGAAAGCTGG AGCTACAAGT GAAGGCGTTC TGGCAAATTT CTTCAACAGT 60
TTGTTGAGTA AAAAGACTGG CTCTCCAGGA GGCCCTGGTG TGAGTGGTGG TAGCCCTGCA 120
GGTGGGGCTG GAGGTGGAAG CAGTGGTTTA CCACCATCCA CCAAAAAGTC AGGCCAGAAG 180
CCTGTCTTAG ATGTTCATGC AGAACTAGAC AGAATTACAC GAAAACCAGT TACAGTTTCT 240
CCCACAACAC CTACATCTCC TACGGAAGGA GAAGCTTCTT GAAGATACCA AATAAAGCCA 300
TTTATTCTGT TTTCTGGGNT AATGTAAA 328

SEQ ID NO:1819
SEQUENCE LENGTH:328
SEQUENCE TYPE:nucleic acid
TOPOLOGY:linear
CLONE:HUMGS02068
SEQUENCE DESCRIPTION:
GATCGGGGCT GCTTATGCCT TTCGTTTATC CTTGGGGTTT GAGAGCGCTG TATTTGGGAG 60
AGAGTTTAAA AATACATTAG GAGAGAGAAA CCATTAAAAG TTTCACTGTC AGAGATATTG 120
TAGGTGCTAA TACTGGATTT CGTCTCANAT TTAATTTCTT TNATGGGTCT GTTAGTCATT 180
CANCAAATCC CATAAGTATG TGTTAATATT TNAATTGTGT AAAACTCATT TGTTACTTTA 240
CAGCCTGTAA TAGTGTGTCT GCATTTNCAA CCTGTTGCAA TAACTTTNCT GAAATATTAA 300
CACATTAATA AAACTTTTCT TAAACAAA 328

SEQ ID NO:1820
SEQUENCE LENGTH:328
SEQUENCE TYPE:nucleic acid
TOPOLOGY:linear
CLONE:HUMGS02069
SEQUENCE DESCRIPTION:
GATCAGAGAA GAGGCTACTG GGGGAGAATT CAGTGCCCCC TTCGCCCTCT AGGGNGCAGA 60
CCTCCACTGC CATTGTCCTG TNAGCCGCCA AANACCCCAC GGGGTGCCCG CATGTCCCTG 120
TCTANNNGCA GCCCAGGGCC CCCACTCCTG GCTCCTNACA CTTGCCTCCC CTATGGCCGC 180
TCTCCAGACC CTCCTCCTTT CTTCTCCCCA CATCCGCACC TGCTGTTCCC ACTCTGGGGT 240
TCTCAAGTCC ATGAACAGAT ATTGTTGCAT TTTCCACAAT GCTGATTAAA CATAATAAAC 300
AATCCAGAAA AGCAGTTTTG CCCAGAAA 328

SEQ ID NO:1821
SEQUENCE LENGTH:319
SEQUENCE TYPE:nucleic acid
TOPOLOGY:linear
CLONE:HUMGS02070
SEQUENCE DESCRIPTION:
GATCCAAAAG CCAGTCTCAG GAGTTTACCC CTGGGATGGG GGATGCATCT NCACCTGACT 60
TTGGGGCCAC GTGCCCTGTG GCACCCCAGC TCACTGGGAG TCTCAGGAGG GATAACCGGA 120
TTTNTGCTCT TTCCCCTGTC ACTCCCACAT CACACAGAAA AATGGCATTC CTCTCTGTCT 180
CTCCCTGGCA TGGAGAGGGC AGACTGTGCA CATTTCACTA GGGTCCAAAT ACAGAAGGGC 240
CCAGGGCCCA GGGGCTTGCA GCTTCGTGAG GGGTCTCTGG CCCAGTTTCC AATGAATAAA 300
GTTCTCTTGA CAGCTCAAA 319

SEQ ID NO:1822
SEQUENCE LENGTH:315
SEQUENCE TYPE:nucleic acid
TOPOLOGY:linear
CLONE:HUMGS02071

SEQUENCE DESCRIPTION:
GATCATCAGA TGGTCGAGTA CTCTTGGAAT GTCTCTTTGC TGTTTACTGT GGCCTCTTTA 60
CAATGGTGAG ACTTATTTCT ACCCTCTACT TTCCACTATT ATTNTTTTCC CCTATTGGAT 120
AATACTAGTC TGAGCAGCCC TAATACTAGA TGTACCTAAA CAGAGATACA AGCCAAGTCA 180
TTGTTCAGTG TAGTCATTTC TTAGTATCTG TTTATATTTT GTCACACATC AGAATGTATA 240
TTTTATAGAG CATGTGTTTG TCTACTNGTT TGTCTACTAT AATATGTCTT TAATAAAGCT 300
AAAANCATTC TGAAA 315

SEQ ID NO:1823
SEQUENCE LENGTH:315
SEQUENCE TYPE:nucleic acid
TOPOLOGY:linear
CLONE:HUMGS02072
SEQUENCE DESCRIPTION:
GATCGCTTTT TTGCCGCTCA TATTTGAATG CATTGGGTTT CCTGGACCAG CTATTTGCAC 60
AAGACTAGGG GGAGGTTGAG GGTTGGCATA GCAGGCAGGG GCTGGGATAG ACATTCAACC 120
TAGCTNNNNT TCATGCTCTT AAGGCTTTAT TTTTCTAGAA TGCCAAGAAA CCAACTTTCT 180
TTAAACATGA CTCATCTCTT CGATTTTCTT GTATAGGTCT TCCTCCCACC CCAAATCTAC 240
ACTTGTTAGA AACAGAATAA TTACCTTTGC TTACTCCATT ATCAACACTT GGAGATAGAG 300
ACTTTACACT TCAGN 315

SEQ ID NO:1824
SEQUENCE LENGTH:332
SEQUENCE TYPE:nucleic acid
TOPOLOGY:linear
CLONE:HUMGS02073
SEQUENCE DESCRIPTION:
GATCGAAAAG GGACCCTGCT TCCAATTTCC CTCTTCCATT CCTCGAGCTA CTCCAGGGCT 60
TAGAAGAATG CTCTTGGTCT GTGGGTCCAG TGTTGTCTGT CATCCATTTA AGTGTTCCCA 120
CTTTCAAGTG ACAATCCTCT CCTTGGCCCT GCCATAGGGC AGAGCATGTN TGGCATAGCA 180
GCCTGACTTT NATGCCCTAA TCTTGAGTTG AGGAAATATA TGCACAGGAG TCAAAGAGAT 240
GTCTTTATAT CTGACTGTAT ATAAATGAAG TTTTTTNGTT TTTTTNGTTT TCCTTTTTGG 300
TGCAATAAAG TTTGTTTTGG CAGAAGGAGA AA 332

SEQ ID NO:1825
SEQUENCE LENGTH:311
SEQUENCE TYPE:nucleic acid
TOPOLOGY:linear
CLONE:HUMGS02074
SEQUENCE DESCRIPTION:
GATCAAGGCG CGGACGCTAT CTACGACCAC ATCAACGAGG GGAAGCTGTG GAAACACATC 60
AAGCACAAGT ATGAGAACAA GTAGTTCCTT GGAGGCCCCC ATCCAGGCCA GAAGGACCAG 120
GTCCACCCAG CAGCTGTTTG CCCAGAGCTG GAGCCTCAGC TTGAAGATGA TGCTCAAGGT 180
ACTCTTCATG GACCACCATT CGCTGTTGGC AAGAAACGGC TTTACTTACA AAACAGACTC 240
TTTACCTTCT GCTGTGTTTG AAGTATGTTT AGTCAGCATG CTCAGGAAAT AAATGTGAAT 300

TGCCCTTGAA A 311

SEQ ID NO:1826
SEQUENCE LENGTH:309
SEQUENCE TYPE:nucleic acid
TOPOLOGY:linear
CLONE:HUMGS02075
SEQUENCE DESCRIPTION:
GATCTAGTGT TTGGAACGGA CGTTACCTGC TGGTTTGTGC ACAACAGTGG AAAAGGATTC 60
ATTGACGGGC ATTACAAGGA TTACTTCGTG CCTNAGCTCT ACAGCTTTCT CAAACGGCCT 120
TAAAGGTTTA TAGTTTGGGA AATTATATAT TATTATACAT CTTTCCCCTG TCACTTTTGC 180
AGATATTCTT CGGTNCCNAT AATTAAAATG AACCAGATAT CAGGGTGGTT AATTAAAATG 240
AACCAGATAT CAGGGTGGTT TATAAAGCCT GTAAACACAC CTNAGAAAAT ANACATTTTA 300
CANATGAAA 309

SEQ ID NO:1827
SEQUENCE LENGTH:301
SEQUENCE TYPE:nucleic acid
TOPOLOGY:linear
CLONE:HUMGS02076
SEQUENCE DESCRIPTION:
GATCATGAAT AACTGATTAG TAAGTCTTGC CTATATTTTC CTGATAGCAT ATGACAAATG 60
TTTCTAAGGT AACAAGATGA GAACAGATAA AGATTGTGTG GTGTTTTGGA TTTGGAGAGA 120
AATATTTTAA TTTTTAAATG CAGTTACAAA TTATAATGTA TTCATATTTG TACTTTCTGT 180
TAAAATGCAT GATTGCAGAA TTGTTTAGAT TTTGTGTTTA TTCTTGATGA AAAGCTTTGT 240
TTGTTCTTGT TTTTAAGTTT GCACTCAAAT CTTAAGAAAT AAATCCACCC ATGTTATCAA 300
A 301

SEQ ID NO:1828
SEQUENCE LENGTH:298
SEQUENCE TYPE:nucleic acid
TOPOLOGY:linear
CLONE:HUMGS02077
SEQUENCE DESCRIPTION:
GATCAGTGAA CTCTGGTTTT AAGATAATCT GAAACAAGGT CCTTGGGAGT AATAAAATTG 60
GTCACATTCT GTAAAGCACA TTCTGTTTAG GAATCAACTT ATCTCAAATA GTAACTCGGG 120
GCCTAACTAT ATGAGATGGC TGAAAAAATA CCACATCGTC TGTTTTCACT AGGTGATGCC 180
AAAATATTTT GCTTTATGTA TATTACAGTT CTTTTTAAAA CACTGGAAGA CTCATGTTAA 240
ACTCTAATTG TGAAGGCAGA ATCTNTGCTA ATTTTTCAGA TTAAAATTCT CTTTGAAA 298

SEQ ID NO:1829
SEQUENCE LENGTH:293
SEQUENCE TYPE:nucleic acid
TOPOLOGY:linear
CLONE:HUMGS02078

SEQUENCE DESCRIPTION:
GATCCCTTAT CCAACATGGT TCTAACCTTC AGTACTAAAG AAGATGCAGT TTCCTTTGCA 60
GAAAAAAATG GATGGAGCTA TGNCATTGAA GAGAGGAAGG TTCCAAAACC CAAGTCCAAG 120
TCTTATGGTG CAAACTTTTC TTGGAACAAA AGAACAAGAG TATCCACAAA ATAGGTTGGC 180
ACTGACTATA TCTCTGCTTG ACTGTGAATA AAGTCAGCTG TNCAGTATTT ATAGTCCATG 240
TATAATAAAT ACATCTCTTA ATCTCCTAAT AAATTGGACC TTTAAACTAC AAA 293

SEQ ID NO:1830
SEQUENCE LENGTH:291
SEQUENCE TYPE:nucleic acid
TOPOLOGY:linear
CLONE:HUMGS02079
SEQUENCE DESCRIPTION:
GATCANNATT TGAAAAGGNT TTCTAGAAAT TNGGGGTAAG AAGTACTACC AAAATGTAAC 60
TNCTAATCAA GGGTGATGCA CAGCAAAAGC AATGGACCCC ATCCCTCTAA AGCCTGCCCT 120
CCTTTGCCTT CAACTGTATA TGCTGGGTAT TTCATTTGTN TTTTTATTTT GGAGAAAGCG 180
NTTTTAACTG CAACTTTCTA TAATGCCAAA NTGACACATC TGTGCAATAG AATGATGTCT 240
GCTCTAGGGA AACCTTCAAA NGCANTAAAA ATGCTGTNTT GAAATGCCAA A 291

SEQ ID NO:1831
SEQUENCE LENGTH:288
SEQUENCE TYPE:nucleic acid
TOPOLOGY:linear
CLONE:HUMGS02080
SEQUENCE DESCRIPTION:
GATCTGGGCC TGAATAAGGG TTATCGAATG GTGGTGAATG AAGGTTCAGA TGGTGGACAG 60
TCTGTCTATC ACGTTCATCT CCATGTTCTN GGAGGTCGGC AAATGCATTG GCCTCCTGGT 120
TAAGCACGTT TTGGGGATAA TTTTCTCTTC TTTAGGCAAT GATTAAGTTA GGCAATTTCC 180
AGTATGTTAA GTAACACACT TATTTTTGCC TGTNTATGGA GAGATTCAAG AAATAATTTT 240
AAAACCGCAT ACATAATAAA AGACATTGTT GCATGGCTTA TAGTCAAA 288

SEQ ID NO:1832
SEQUENCE LENGTH:288
SEQUENCE TYPE:nucleic acid
TOPOLOGY:linear
CLONE:HUMGS02081
SEQUENCE DESCRIPTION:
GATCCAGCCT TAATNTTTTT CTGACATCCT AGTAATAGCA GACCCTGCAC AAAGTGGATA 60
TCAGACTTAA TACTGTNAGG AGACAAAATA ATGAGAATAG TTTTACCAAA GATAATNAAA 120
TCTCGCATCA TGTCTGCATT TTACCATAGA TTGGAGTGCA TCCTTAGGAA ACTGAATGTA 180
ACAAAAACCC AGGACATTTT TGGAAATTGT ATGCTCTCTA GCAACTTTGT GTGAATTTTT 240
ATACAAACAC TGTTCTATGA GTTATATAAA ATGTTATAAA ACTAGAAA 288

SEQ ID NO:1833
SEQUENCE LENGTH:285

SEQUENCE TYPE:nucleic acid
TOPOLOGY:linear
CLONE:HUMGS02082
SEQUENCE DESCRIPTION:
GATCCTGAGA ACTTCAGGCT CCTGGGCAAC GTGCTGGTCT GTGTGCTGGC CNNNCACTTT 60
GGCAAAGAAT TCACCCCACC AGTGCAGGCT GCCTATNAGA AAGTGGTGGC TGGTGTGGCT 120
AATGCCCTGG CCCACAAGTA TCACTAAGCT CGCTTTCTTG CTGTCCAATT TCTATTAAAG 180
GTTCCTTTGT TCCCTAAGTC CAACTACTAA ACTGGGGGAT ATTATGAAGG GCCTTGAGCA 240
TCTGGATTCT GCCTAATAAA AAACATTTAT TTTCATTGCA AGAAA 285

SEQ ID NO:1834
SEQUENCE LENGTH:282
SEQUENCE TYPE:nucleic acid
TOPOLOGY:linear
CLONE:HUMGS02084
SEQUENCE DESCRIPTION:
GATCTCATGG GCTTTCCCTG GAGGAAAGGT TTTTTTTGTT GTTTTTTTTT NAAGAACTTG 60
AAACTTGTAA ACAAGAGATG TCTGTAGCTT TTTTNCCCAT CTGTAGTGTA TGTNAAGATT 120
NCAAAACCTG AGAGCACTTT TCCTTTGTTT AGAATTATGA GAAAGGCACT AGATGACTTN 180
AGGATTTNCA TTTTNCCCTT TATTGCCTCA TTTCTTGTGA CGCCTTGTTG GGGAGGGAAA 240
TCTGTTTATT TTTNCCTACA AATAAAAAGC TAAGATTCTA AA 282

SEQ ID NO:1835
SEQUENCE LENGTH:279
SEQUENCE TYPE:nucleic acid
TOPOLOGY:linear
CLONE:HUMGS02085
SEQUENCE DESCRIPTION:
GATCTTAAGC TTTGATAAGA GCTGTGCTGT GGCTGAGTAT GGTGTGTATG TGAAGGTGAC 60
TTCCATCCAG GACTGGGTTC AGAAGACCAT AGCTGAGAAC TAATGCAAGG CTGGCCGGAA 120
GCCCTTGCCT GAAAGCAAGN CTTCAGCCTG GAAGAGGGCA AAGTGGACGG GAGTGGACAG 180
GAGTNGATGC GATAAGATNN GGTTTGAAGC TGATGGGTGC CAGCCCTGCA TTGCTGAGTC 240
AATCAATAAA GAGCTTTCTT TTGACCCAAA AAAANTAAA 279

SEQ ID NO:1836
SEQUENCE LENGTH:275
SEQUENCE TYPE:nucleic acid
TOPOLOGY:linear
CLONE:HUMGS02086
SEQUENCE DESCRIPTION:
GATCCATAGT GCTACCTGCA CCTCTGGATT CTGGATTCAC AGACCAAGTC CAAGCCCGTT 60
CTTACGTCGC CATAAAGGCC CCCGAACGGC ATTCTCGGTA CTTCTGTTTG TTTTTGTACA 120
TTTNATTAGA AAGGACTGTA AAATAGCCAC TTAGACACTT TACCTCTTCA GTATGCAAAT 180
GTAAATAAAT TGTAATATAG GAAATCTTTT GTTTTAATAT AAGAATGAGC CTGTCCAATT 240
TCTGCTGTAC ATTATTAAAA GTTTTTATTC ACAAA 275

SEQ ID NO:1837
SEQUENCE LENGTH:269
SEQUENCE TYPE:nucleic acid
TOPOLOGY:linear
CLONE:HUMGS02087
SEQUENCE DESCRIPTION:
GATCCGAAGA GGAACTCCTG GTCATCTTTA ACAAGAAAAT CAGCAAGAAC CCTACCTTTA 60
AGTTATTAAA GGACAAAGTC AAGCTTGTGA AATGAACATT TGTGTATTTA AAANTTGAAT 120
CCATTCTGCT GACTTCTTAC TTTCACTGCT GTTTATAAAA TGTGTAATGA ATTCTAACAA 180
CTCAAATTTT GCTTTTTGAC GCNGTATTTT TANGTTANGN AAATATATTT NTGGTATAAC 240
TTTTATGCGA NAAATAAAAT ATATTCTGN 269

SEQ ID NO:1838
SEQUENCE LENGTH:268
SEQUENCE TYPE:nucleic acid
TOPOLOGY:linear
CLONE:HUMGS02088
SEQUENCE DESCRIPTION:
GATCTCATTG TTTATTAACC TGTATTCTGT TTACATGTCT TTAAAACAGT GGTTCTTAAA 60
TTGTAAGCTC AGGTTCAAAG TGTTGGTAAT GCCTGATTCA CAACTTTGAG AAGGTAGCAC 120
TGGAGAGAAT TGGAATGGGT GGCGGTAATT GGTGATACTT CTTTGAATGT AGATTTCCAA 180
TCACATCTTT AGTGTCTGAA TATATCCAAA TGTTTTAGGA TGTATGTTAC TTCTTAGAGA 240
GAAATAAAGC ATTTTTGGGA AGAATAAA 268

SEQ ID NO:1839
SEQUENCE LENGTH:265
SEQUENCE TYPE:nucleic acid
TOPOLOGY:linear
CLONE:HUMGS02089
SEQUENCE DESCRIPTION:
GATCTATCTA AATATATTAA GTAAAATTAC ACCATTCACT TGTTGGGAAA ATAATCTTTG 60
GTTTGGAAGA TATTAACATA ATGGGCATCT TAGAATCATA AATCACATGA AATGAGAGAC 120
AATGCAATAT TGTATAATTC CTGGATGATG CAATTGTTTT AATTGANTTT TCAAGTGCCA 180
TTATAAAGTT TTAAAAATTA TCAATATGAG TTGGTGCCTA ATTTTTNNTT TCCTAAAAAT 240
AAAATTTTTC CTTTTTATGA GTAAA 265

SEQ ID NO:1840
SEQUENCE LENGTH:261
SEQUENCE TYPE:nucleic acid
TOPOLOGY:linear
CLONE:HUMGS02090
SEQUENCE DESCRIPTION:
GATCATAAAA CCTTCATTCC ATAGGTACCC TTTATCCTCA CAGATACAGA GACACCAAGA 60
AGAATCTGGA CAAATAGGAC TTGCTAAGTT CTCCACAGTT TATTACCATT AGATTATGTC 120

CTTTTGTNCT CTGATTATAC TTCTGCATAA GACTGTACAT AAAANTACAC AAGTTTTCCT 180
ATTTNTTTTT TGTCTTCATT TCTGAAGATT CCTGTCATGT AAAACTTACA GTAAATAAAT 240
GTGTGTGCTT TTNTCAATAA A 261

SEQ ID NO:1841
SEQUENCE LENGTH:263
SEQUENCE TYPE:nucleic acid
TOPOLOGY:linear
CLONE:HUMGS02091
SEQUENCE DESCRIPTION:
GATCAGGTTA GGGCGAGCGC TACCTCTCCA GCCTCAGCTC TCAGTTGCAG CCCTGCTNAT 60
GCCTGCTTGG ACTTGGCCCC TGCCACCTCC NGCCTCAGGT GTCCGCTATC CACCAAAAGG 120
GCTCCCTGAG GGTCTGGGCA AGGGACCTGC TTCTATTAGC CCTTCTCCAT GGCCCTGCCA 180
TGCTCTCCAA ACCACTTTTT GCAGCTTTTT CTAGTTCAAG TTCACCAGAC TCTATAAATA 240
AAACCNGACA GACCATGAAC AAA 263

SEQ ID NO:1842
SEQUENCE LENGTH:264
SEQUENCE TYPE:nucleic acid
TOPOLOGY:linear
CLONE:HUMGS02092
SEQUENCE DESCRIPTION:
GATCAGAAAG AAACCTTTTG TAGCAATATC AAGAATCTAG TTTCATCTGA GAACTTCTGA 60
TTAGCTCTCA GTCTTCAGCT CTATTTATCT TAGGAGTTTA ATTTGCCCTT CTCTCCCCAT 120
CTTCCCTCAG TTCCCATAAA ACCTTCATTA CACATAAAGA TACACGTGGG GGTCAGTGAA 180
TCTGCTTNAN NATCCTGAAA GTTTCTGGGG CTTAAGATTC CAGACTCTGA TTCATTAAAC 240
TATAGTCACC CGTGTCCTGT GAAA 264

SEQ ID NO:1843
SEQUENCE LENGTH:246
SEQUENCE TYPE:nucleic acid
TOPOLOGY:linear
CLONE:HUMGS02093
SEQUENCE DESCRIPTION:
GATCAAATGT CACACAGCAG TTTCCTTGCA ACACTTTCAG CTCCCCATGC TCCAGAATAC 60
CCACCCAAGA AAATAATAGG CTTTAAAACA ATATCGGCTC CTCATCCAAA GAACAACTGC 120
TGATTGAAAC ACCTCATTAG CTGAGTGTAG AGAAGTGCAT CTTATGAAAC AGTCTTAGCA 180
GTGGTAGGTT GGGAAGGAGA TAGCTGCAAC CAAAAAAGAA ATANATATTC TATAAACCTT 240
CAGAAA 246

SEQ ID NO:1844
SEQUENCE LENGTH:243
SEQUENCE TYPE:nucleic acid
TOPOLOGY:linear
CLONE:HUMGS02094

SEQUENCE DESCRIPTION:
GATCTGTGTG CATGTNTGTG TTTGTGTATA TATACATATC TAGGGCTAGT ACTTAGTTTC 60
ACACCCGGGA GCTGGGAGAA AAAACCTGTA CAGTTGTCTT TCTCTNATTT TNAATAAAAT 120
AGAAAAAATCG CGCACTTGCG GTCCCCCCCC CACCCCCTTT TTTAAACAAG TGTTACTNNN 180
NCCGGGAAAA TTTTNCTGTC TTTGTAATTT TAAAACTTTA AAATAAATTG GAAAAGGGAG 240
AAA 243

SEQ ID NO:1845
SEQUENCE LENGTH:240
SEQUENCE TYPE:nucleic acid
TOPOLOGY:linear
CLONE:HUMGS02095
SEQUENCE DESCRIPTION:
GATCTAGTGA ACATTCGACC TCCATTACGG AGAGTTTCCT ATGTTTCACT GTGCAAATAT 60
ATCTGCTATT CTCCATACTC TGTAACAGTT GCATTGACTG TCACATAATG CTCATACTTA 120
TCTAATGTTG AGTTATTAAT ATGTTATTAT TAAATAGAGA AATATGATTT GTGTATTATA 180
ATTCAAAGGC ATTTCTTTTC TGCATGTTCT AAATAAAAAG CATTATTATT TGCTGAGAAA 240

SEQ ID NO:1846
SEQUENCE LENGTH:239
SEQUENCE TYPE:nucleic acid
TOPOLOGY:linear
CLONE:HUMGS02096
SEQUENCE DESCRIPTION:
GATCTAAGCA GCGTTCCCAC AGCCCCCTCT AGATTCTTGG AGTTCCGCAG AGCTTACTAT 60
ACGCGGTCTG TCCTAATCTT TGTGGTGTTC GCTATCTCTT GTGTCAAATC TGGTGAACGC 120
TACGCTTACA TATATTGTCT TTGTGCTGCT GTNTGACAAA CGCAATGCAA AAACAATCCT 180
CTTTCTCTCT CTTAATGCAT GATACAGAAT AATAATAAGA NTTTCATCTT TAAATGAAA 239

SEQ ID NO:1847
SEQUENCE LENGTH:231
SEQUENCE TYPE:nucleic acid
TOPOLOGY:linear
CLONE:HUMGS02097
SEQUENCE DESCRIPTION:
GATCCATCCT CTCTCCTAGC TGAGTAAATC CGGGTCTCTA GGATGCCAGA GCAGCGCACA 60
CAAGCTGGGA AATCCTCAGG GCTCCTACCA GCAGGACTGC CTCGCTGCCC CACCTCCCGC 120
TCCTTGGCCT GTCCCCAGAT TCCTTCCCTG GTTGACTTGA CTCATGCTTG TTTCACTTTC 180
ACATGGAATT TCCCAGTTAT GAAATTAATA AAAATCAATG GTTTCCACAA A 231

SEQ ID NO:1848
SEQUENCE LENGTH:231
SEQUENCE TYPE:nucleic acid
TOPOLOGY:linear
CLONE:HUMGS02098

SEQUENCE DESCRIPTION:
GATCCAAAAT GCTGACAGGT TCAAACAAAA TTGGAATTGA AAATCAGAGT GCGTTCAAGA 60
GTATCAAACA ATACTATCTT GTTACTTGCT TATTACCTTA GTAGACTGGA AGCAACACTT 120
CACACAAAAA AAGGGTTTGG ATGTAATTTC GGATAAGAAG AGATGTTTCT GTAAAGTCTT 180
TCCTGAGANG CATATTATTT GAGAAAAACA CATATTTCTG TTTTTAGTAA A 231

SEQ ID NO:1849
SEQUENCE LENGTH:228
SEQUENCE TYPE:nucleic acid
TOPOLOGY:linear
CLONE:HUMGS02099
SEQUENCE DESCRIPTION:
GATCTACCAT GTAAAATGTT GTAGAGATAG AGCCATATAA CGTCACGTTT CAAAACTAGC 60
TCTACAGTTT TGCTTCTCCT ATTAGCCATA TGATAATTGG GCTATGTAGT ATCAATATTT 120
ACTTTAATCA CAAAGGATGG TTTCTTGAAA TAATTTGTAT TGATTGAGGC CTATGAACTG 180
ACCTGAATTG GAAAGGATGT GATTAATATA AATAATAGCA GATATAAA 228

SEQ ID NO:1850
SEQUENCE LENGTH:236
SEQUENCE TYPE:nucleic acid
TOPOLOGY:linear
CLONE:HUMGS02100
SEQUENCE DESCRIPTION:
GATCTACCGG CCCCGCTGGG GTGCCCTTGG GGACTACCTC TCCTTCACCA TACCCCTGGG 60
CACCCCCTGA CAACTTCTGC ACATACTGGG GCCCTGCTTA TTNTCCCAGG ACAGGCTCCT 120
TAAAGCAGAG GAGCCNGTNC TGGGAGCCCC TTCTCAAACT CCTAAGACTT GTTTTCATGT 180
CCCACGTTCT CTGCTGACAT CCCCCAATAA AGGACCCTAA CTTTNGGNNN TNNAAA 236

SEQ ID NO:1851
SEQUENCE LENGTH:227
SEQUENCE TYPE:nucleic acid
TOPOLOGY:linear
CLONE:HUMGS02101
SEQUENCE DESCRIPTION:
GATCTATGGT TTCTGCCTAC CCAGGATAGT CTTGTCTCTA GACAATGTAT TGATGATGCA 60
AATGTTCTGG AACTAAATAG TGGTGATGGT GGCACAACAC TGTGAATGTA CTAAATGTCA 120
CTGATTTGTA CGTATTTAAT ATGAATTAAA TGTTAAAATG GTGAATTATA TTTATGTATA 180
TTTNACCATA ATAAAAATAT GCATTGGAAG ATGCATTTTT TGTGAAA 227

SEQ ID NO:1852
SEQUENCE LENGTH:229
SEQUENCE TYPE:nucleic acid
TOPOLOGY:linear
CLONE:HUMGS02102
SEQUENCE DESCRIPTION:

GATCTTTGGA CCAGTTTCTA GCAAAGTTGT GTTTGAAAGA TACTCTATTA AAGAAGACTG 60
TGGAATCTTT TTATCGGTGC CCATTATATC CTTAAGTTTG GATATTTAGC TGACCTTCGC 120
TTTAACATAG GTCTAATTTA TTTGCCGTGT CATTTTCCAT ACAAATCAGT TGATTTAAAA 180
AAGTTCATTT CTCATACTGT GCATTAAAAT AAAAATTTGA ACAATTAAA 229

SEQ ID NO:1853
SEQUENCE LENGTH:224
SEQUENCE TYPE:nucleic acid
TOPOLOGY:linear
CLONE:HUMGS02103
SEQUENCE DESCRIPTION:
GATCTCCTGT TTTTTTCAGG CTCCTAAAGT AACATCAGCC ATNTTTNAGA TTGGTTCTGT 60
TTTCGTACCT TCCCACTGGC CTCAAGTGAG CCAAGAAACA CTGCCTGCCC TCTGTCTGTC 120
TTCTCCTAAT TCTGCAGGTG GAGGTTGCTA GTCTAGTTTC CTTTTTGAGA TACTATTTTC 180
ATTTTTGTGA GCCTCTTTGT AATAAAATGG TACATTTCTA TAAA 224

SEQ ID NO:1854
SEQUENCE LENGTH:223
SEQUENCE TYPE:nucleic acid
TOPOLOGY:linear
CLONE:HUMGS02104
SEQUENCE DESCRIPTION:
GATCAAGGCC GGACCCCACT GCCCCACTGC CCAACTGATA GCCACGCTGA NNGAATGGAA 60
GGAAAATTTG CTTGGACCTG CAAGCCCCGC TGTACAAGAA AATAATTAAG AAACTTTTGG 120
AGAGTTAGCT ACTAGCTGCC TACGTGTGTN CATTTGCTAT ATAGCATACT TCTTTTTTCC 180
AGTTTCAATC TAACTGTGAA AGAACTTCTG ATATTTTGTG TTN 223

SEQ ID NO:1855
SEQUENCE LENGTH:227
SEQUENCE TYPE:nucleic acid
TOPOLOGY:linear
CLONE:HUMGS02105
SEQUENCE DESCRIPTION:
GATCCTAGTA ATTGCCTAGA AATATCTTTC TCTTACCTGT TATTTATCAA TTTTTCCCAG 60
TATTTTTATA CGGAAAAAAT TGTATTGAAA ACACTTAGTA TGCAGTTGAT AAGAGGAATT 120
TGGTATAATT ATGGTGGGTG ATTATTTTTT ATACTGTATG TGCCAAAGCT TTACTACTGT 180
GGAAAGACAA CTGTTTTAAT AAAAGATTTA CATTCCAAAA AAACAAA 227

SEQ ID NO:1856
SEQUENCE LENGTH:219
SEQUENCE TYPE:nucleic acid
TOPOLOGY:linear
CLONE:HUMGS02106
SEQUENCE DESCRIPTION:
GATCTATACA AAGTCTTTGA GAGGTGTTAA ATATGGTTAT TTATGCACTG TGGGATGTGT 60

TCTTCTTTCT CTGTATTCCG ATACAAAGTG TTGTATCAAA GTGTGATATA CAANGTGTAC 120
CAACATAAGT GTTGGTAGCA CNTAAGACTT ATACTTGCCT TCTGATAGTA TTCCTTTATA 180
CACAGTGGAT TGATTATAAA TAAATAGATG TGTCTTAAA 219

SEQ ID NO:1857
SEQUENCE LENGTH:219
SEQUENCE TYPE:nucleic acid
TOPOLOGY:linear
CLONE:HUMGS02107
SEQUENCE DESCRIPTION:
GATCAAGAAA GACAAAAAGC CAGCCTGCAG CAAGCACTTT GGAGAGCAAA TCTGNAAAGA 60
GAGTAACGCG GAGTGTCAAG AGGTGTGCAG AAAATCCAAA GAAGGCTGAG GACAATGTGT 120
GTGTCAAGAA AATAAGAACC AGAAGTCATA GGGACAGTGA AGATATTTGA CAGAAAAATC 180
GAACTGGGAA AAATATAATA AAGTTAGTTT TGTGATAAA 219

SEQ ID NO:1858
SEQUENCE LENGTH:219
SEQUENCE TYPE:nucleic acid
TOPOLOGY:linear
CLONE:HUMGS02108
SEQUENCE DESCRIPTION:
GATCTGTCGG AACCAGGACA CAAAAGCAGC AAGAAGCGGG GAGAGAGAGG GATAGAAAAC 60
AAGCGCAGGA GAGCCTGCGA ACGCAAAANT GAATGAGGGC TTTTTGTGGC TGGGGATGGG 120
TTTTGGTTTT GGGGTTTTTT TTTTNAAATT GTTTTGACTT CGTACAGGGT ACTTTTTCCC 180
AACCTCATCT NTCAGAAATC CATGTGGGNT TCCTGGAAA 219

SEQ ID NO:1859
SEQUENCE LENGTH:217
SEQUENCE TYPE:nucleic acid
TOPOLOGY:linear
CLONE:HUMGS02110
SEQUENCE DESCRIPTION:
GATCTGAAGA AATTACCAAA CATTAAAATG AGANAAATTT GCGCTAATGA TGCAATTCCC 60
AAGACGTGCA AGAGGAAAAC TATTATAACT GTAGACCAAG ATTTGGGGGA ATTGGAACTA 120
AACGATGAAT CAAGTGATTC AGATGAGGAA ATGACTGCAG TGGCCTAATT GTCTTCACTT 180
GAATTGAAAA TAAATAATTT GAGAGCTTCA AATTAAA 217

SEQ ID NO:1860
SEQUENCE LENGTH:216
SEQUENCE TYPE:nucleic acid
TOPOLOGY:linear
CLONE:HUMGS02111
SEQUENCE DESCRIPTION:
GATCCATTTC TTTAAAATAC TGACATATAG AGTTGTACCT TATATAGAAT ATAGTTGTAT 60
CTTGAAGTCA ACATATTAAA TTATTCTCAA AATTATGTAT TTNCAGATTG TACTTGTAAG 120

TTTCAAAGAA AAATTACCGT CTTTTCATAT TGACCTGGAA ACTAAATAGG ATGTGATTCA 180
GCTACATTAA TTTCTTAATA CAATCTAGGA AAGAAA 216

SEQ ID NO:1861
SEQUENCE LENGTH:213
SEQUENCE TYPE:nucleic acid
TOPOLOGY:linear
CLONE:HUMGS02112
SEQUENCE DESCRIPTION:
GATCCTTTTC TTGAAAGAAT AACAGAAAAT GTGTCATCCA TTTTTCAGAA TATAACCACT 60
CAAGCTACTG GCACATAGTG AAAGATTACT TCTGACATTC CATTNCTCTT CTTTTGAAAA 120
TTAGTATNGT AATTAAATGT ACTTTTTGAA AATTAATAGA ATTATTTAAA TTACAGTATA 180
TGTNGCAAAA TATATCACTT TTGATACAAT AAA 213

SEQ ID NO:1862
SEQUENCE LENGTH:210
SEQUENCE TYPE:nucleic acid
TOPOLOGY:linear
CLONE:HUMGS02113
SEQUENCE DESCRIPTION:
GATCTATCTC ATCAAACAAC ACAATGAAGA TATTTTAGAG TACAAAAGAA GAAATGGGCT 60
GGAATAAACT TTTGAAACAC TAATGTAGTA TGCTCCGTAT AGTGATTGTA GCTGTTCCTC 120
TGGATTCACC ATCTGTTGAG TTGTAAATGT GNGANAAAAA GTTATATGTG AATATATATC 180
AAGCCAGCAT TTGTATTTTG CATCATTAAA 210

SEQ ID NO:1863
SEQUENCE LENGTH:209
SEQUENCE TYPE:nucleic acid
TOPOLOGY:linear
CLONE:HUMGS02115
SEQUENCE DESCRIPTION:
GATCGGGGCT GCCAGAGGGN TAGAAGCGAG GGCACCAGCA GTTGTGGGTG GGGAGCAANG 60
GAAGAGAGAA ACTCTTCAGC GAATCCTTCT AGTACTAGTG GAGAGTTTGA CTGTGAATTA 120
ATTTTATGCC ATAAAAGACC AACCCAGTTC TGTTTNNNNC TGTAGCATCT TGAAAAGAAA 180
AATTATAATA AAGCCCCAAA ATTAAGAAA 209

SEQ ID NO:1864
SEQUENCE LENGTH:208
SEQUENCE TYPE:nucleic acid
TOPOLOGY:linear
CLONE:HUMGS02116
SEQUENCE DESCRIPTION:
GATCTGGAGC AACAGCTAGG AAATATCATT AATTCTGCTC TTCAGAGATG TTAAAAATAA 60
ATTACACGTG GGAGCTTTCC AAAGAAATGG AAATTGATGG GAAATTATTT GTCAAGCAAA 120
TGTTTAAAAT CCAAATGAGA ACTAAATAAA GTGTTGAACA TCAACTGGGG AATTGAAGCC 180

AATAAACCTT CCTTCTTAAC CATTCAAA 208

SEQ ID NO:1865
SEQUENCE LENGTH:201
SEQUENCE TYPE:nucleic acid
TOPOLOGY:linear
CLONE:HUMGS02117
SEQUENCE DESCRIPTION:
GATCTATACT TAGAATGCAA TGTNGACAGA GGGCAGTTAA AATATCCAAG ATGTATTCCA 60
AGACAAAGCA AACAAACAAC TGGAAGTGGT AATCAATTCA ACAGGCCTTC TGAATTNTCT 120
GAAGCNAAAG CAACGTTTGC CATNNGATAT TTGTGACACT TTGGCAAATG GAAAAATAAA 180
CCAATTTATA CGTGCAATAA A 201

SEQ ID NO:1866
SEQUENCE LENGTH:200
SEQUENCE TYPE:nucleic acid
TOPOLOGY:linear
CLONE:HUMGS02118
SEQUENCE DESCRIPTION:
GATCATGAAG GTATCCACAC TTCCCCTTGC ATTTCCTTCT TAACTCTCTG TTACACGTCA 60
TTGAAACTAC ACTTTTTTGG TCTGTTTTTG TGCTAGACTG TAAGTTCCTT GGGGGCAGGG 120
CCTTTGTCTG TCTCATCTCT GTATTCCCAA ATGCCTAACA GTACAGAGCC ATGACTCAAT 180
AAATACATGT TAAATGGAAA 200

SEQ ID NO:1867
SEQUENCE LENGTH:202
SEQUENCE TYPE:nucleic acid
TOPOLOGY:linear
CLONE:HUMGS02119
SEQUENCE DESCRIPTION:
GATCACATTT TGAAATGCCT AAAAGACTTT ATTGTTCTAA TTATCCAGAT GTACCTTTGT 60
AAAATAGCTC TTTTATGAAT TAGCTGATAA GGCTGTATGT TTCTGGAACA AAATATTGGT 120
CATCTAAAAN CTTTCTGTTT NCTGGGGTCT GGGAAAATAG AAAATAAGAT TTCAAATATT 180
AAATAAGCTT AANGGCACCA AA 202

SEQ ID NO:1868
SEQUENCE LENGTH:200
SEQUENCE TYPE:nucleic acid
TOPOLOGY:linear
CLONE:HUMGS02120
SEQUENCE DESCRIPTION:
GATCAGGCCC TTCTTCCCAC AGCAATAGTC CCCAATACGT AGATTTTTGC TCTTCTGTAT 60
GTGACAACAT TTTTGTACAT TATGTTATTG GAATTTNCTT TCATACATTA TATTCCTCTA 120
AAACTCTCAA GCAGACGTGA GTGTGACTTT TTGAAAAAAG TATAGGATAA ATTACATTAA 180
AATAGCACAT GATTTTCAAA 200

SEQ ID NO:1869
SEQUENCE LENGTH:194
SEQUENCE TYPE:nucleic acid
TOPOLOGY:linear
CLONE:HUMGS02121
SEQUENCE DESCRIPTION:
GATCTNTNTG TTCATTGTAT TCTTTTTNTC TCAAGAGTTC CATTTAATGG AAATAAAACG 60
GTATAATTAA TAATTGTCTA GGGGGGAAAA ATGAAGCAAA TNTCATTGGA TATTTTTAAA 120
GGTCTCCACA GAGTTTATGC CATATTGGAA TTTTGTTGTA TAATTGTCAA ATAAATATTT 180
TGGTGANGCA TAAA 194

SEQ ID NO:1870
SEQUENCE LENGTH:190
SEQUENCE TYPE:nucleic acid
TOPOLOGY:linear
CLONE:HUMGS02122
SEQUENCE DESCRIPTION:
GATCCAATAC TATTTAGTTT ATTATTGAAA TTGGAAGGAT TCATTGAGCA GCATAGAAGT 60
TTGTTTACAT GTTACTTTGA GATGCTAGGT ATTTGTGGAA TTAAAAAGAA TCAGGCTCTT 120
TTGTACTNAN TTTTTAAATC TGTGATGCTT NTCAAATTTA ATTCATAATA AATTGATGCA 180
ATTTCATAAA 190

SEQ ID NO:1871
SEQUENCE LENGTH:188
SEQUENCE TYPE:nucleic acid
TOPOLOGY:linear
CLONE:HUMGS02123
SEQUENCE DESCRIPTION:
GATCGAACAG TTTTGAAGCT ACTGTGTGTG TGAATGAACA CTCTTGCTTT ATTCCAGAAT 60
GCTGTACATC TATTTTGGAT TGTATATTGT GTTTGTGTAT TTACGCTTTG ATTCANNAGT 120
AACTTCTTAT GGAATTNATT TGCATTGAAC ACAAACTGTA AATAAAAAGA AATGGCTGAA 180
AGAGCAAA 188

SEQ ID NO:1872
SEQUENCE LENGTH:184
SEQUENCE TYPE:nucleic acid
TOPOLOGY:linear
CLONE:HUMGS02124
SEQUENCE DESCRIPTION:
GATCCTATTT ATTTGTTTAG GCCATCTTTA ATTTCTTTCA GCAGTGTTTT GTAGTTTCCA 60
TTAGAGATTT TCCATGTCNT TTGTTAAGTT TATTCCTAGG TATTTTATTT TTTATGGTAT 120
TNTATTATAA TCCTNNGATG TCTATTATAT AGAAATATAA TTNATTTTNG TTATTGACTG 180
NNNN 184

SEQ ID NO:1873
SEQUENCE LENGTH:181
SEQUENCE TYPE:nucleic acid
TOPOLOGY:linear
CLONE:HUMGS02126
SEQUENCE DESCRIPTION:
GATCCAAGTN CTGACTTTTC CCCCTGACAG CTGTGTGACC TTCGTGAAGT CGCCAAACCT 60
CAAATGAGCC CCAGTCATTG CTAGTAAGAC CTGCCTTTGA GTTGGTATGA TGTTCAAGTT 120
AGATAACAAA ATGTTTATAC CCATTAGAAC AGAGAATAAA TAGAACTACA TTTCTTGCAA 180
A 181

SEQ ID NO:1874
SEQUENCE LENGTH:181
SEQUENCE TYPE:nucleic acid
TOPOLOGY:linear
CLONE:HUMGS02127
SEQUENCE DESCRIPTION:
GATCTTAAAT TTTCTCATCT CAAAAAAATA TGTAAAGTGA TAGATATGTT AAATAGCTNN 60
TATGTATTTC GCAATGTGTA GTGTATATAT ATAAACATCA CACAGTACAC CATAAATATA 120
TTTAATTTTT GTCAATTACT CTTTAAACCT TAATAAAGTT GATAAAACAT TCTTTTTTAA 180
A 181

SEQ ID NO:1875
SEQUENCE LENGTH:179
SEQUENCE TYPE:nucleic acid
TOPOLOGY:linear
CLONE:HUMGS02128
SEQUENCE DESCRIPTION:
GATCAGGTTA GCAAATGATG TAAAAGAAGC TTTATTGTCT AGTTGTTTTT TTTCCCCCAA 60
GACAAAGGCA AGTTTCCCTA AGTTTGAGTT GATAGTTATT AAAAAGAAAA CAAAACAAAA 120
AAAAGGCAAG GCACAACAAA AAAATATCCT GGGCAATAAA AAAANTATTT TAAACCAAA 179

SEQ ID NO:1876
SEQUENCE LENGTH:178
SEQUENCE TYPE:nucleic acid
TOPOLOGY:linear
CLONE:HUMGS02129
SEQUENCE DESCRIPTION:
GATCCGGGAG CACACCGTTT CCTGATTGCT CAGGGACTCA TCTTTCCCTC CTTGGTGATT 60
CCGCAGTGAG AGAGTGGCTG GGGCATGGAA GGCAAGATTG TGTCCCATTC CCCCAGTGCG 120
GCCAGCTCCG CGCCAGGATG GCGANGGAAC TCAATAAAGT GCTTTGAAAA TGCTGAAA 178

SEQ ID NO:1877
SEQUENCE LENGTH:175
SEQUENCE TYPE:nucleic acid

TOPOLOGY:linear
CLONE:HUMGS02130
SEQUENCE DESCRIPTION:
GATCATTCTC CGAAAGCAAC TCCTGACAAT TAAGCATTTT TTTCTCCAAA TACAAAGTAT 60
ATTCTCTTTA TTGGAAAATA AATTAATAAA TATATTCTGT ATTTTNNCTC TCCGTGAAAA 120
ACAAAAGAGC CTCTGACATT ACTGTCTCTC AGTGTTGGTT CAGATTGAGG CTTTN 175

SEQ ID NO:1878
SEQUENCE LENGTH:172
SEQUENCE TYPE:nucleic acid
TOPOLOGY:linear
CLONE:HUMGS02131
SEQUENCE DESCRIPTION:
GATCAATCCT TCTTATGTTT GCTTTAATGT GTATTGAAGA AAAGCACTTT TTAAAAAGTA 60
CTCTTTAAGA GTGAAATAAT TAAAAACCAC TGAACATTTG CTTTGTTTTC TAAAGTTGTT 120
CACATATATG TAATTTAGCA GTCCAAAGAA CAAGAAATTG TTTCTTTTCA AA 172

SEQ ID NO:1879
SEQUENCE LENGTH:170
SEQUENCE TYPE:nucleic acid
TOPOLOGY:linear
CLONE:HUMGS02132
SEQUENCE DESCRIPTION:
GATCTGATTG TTTAAAAGTA CACAGCACCT CCCTCCCCAC TCTCTCTTGC TCCTGCTTTC 60
ATCAAGTGAA AACTCCCTGA GGCCTCCTCA GAAGCTGAGT AGAGGCCAGC ACTATGCTTC 120
CTGTACAGCC TGTGGAACCA TGAGGCAATT AAACCTCTTT TCTTTATAAA 170

SEQ ID NO:1880
SEQUENCE LENGTH:168
SEQUENCE TYPE:nucleic acid
TOPOLOGY:linear
CLONE:HUMGS02133
SEQUENCE DESCRIPTION:
GATCAGAAAA ATTCATCTTT TTTAACCCTG CCCTAATTTT TCTTGAGGAA TTAAATAGAG 60
CAAACTATTT TCAGGTTATG CTTACAATAA AATATACTTA AGAAAATGAC TGAAGATGTA 120
TGTTTTTGAA TGTTTTGATT AAATAAATGT ACACATTTAG AACACAAA 168

SEQ ID NO:1881
SEQUENCE LENGTH:168
SEQUENCE TYPE:nucleic acid
TOPOLOGY:linear
CLONE:HUMGS02134
SEQUENCE DESCRIPTION:
GATCAGCCTC CCTGTAAGCC AGTTATCAGT ATCCAAGTGC TTTAACACAG GCTTCTCAGA 60
AGAAATCATT TTTATTCCTA CCATTATTTC ATACCAAAGC ATCATTATGT GCTACAGTTC 120

CAATTTCTTA CACAAGAATG AAAAGGGTTA CCAATGCAGC AAGTTAAA 168

SEQ ID NO:1882
SEQUENCE LENGTH:167
SEQUENCE TYPE:nucleic acid
TOPOLOGY:linear
CLONE:HUMGS02135
SEQUENCE DESCRIPTION:
GATCTTACTG TAAGTTGAAG GGAGTTTTGC CCTAACTCAT GGATTGTGCA AGAATGAACT 60
GCTGTTGGGT TTGACTGACT GTCGATGGAT TGTGGTGTGG TGTATCTNAA GGCTATTGAA 120
TGCAACTTAC AATGCTTAGG TAAAAATCTT TATNCTTTTA GTATAAA 167

SEQ ID NO:1883
SEQUENCE LENGTH:165
SEQUENCE TYPE:nucleic acid
TOPOLOGY:linear
CLONE:HUMGS02136
SEQUENCE DESCRIPTION:
GATCCTTGAT GAGCCAACAT TGCACTGTGG ATACATATCT ATGTTTACGC GCTATTAGAA 60
CAGAAGGCGC TGTATATAGA AATGTTGCTT TGAAGCAATA TTTGCAAAAC ACNCAGACTN 120
CAGNATCTGT ATTTGGAAAA AATAAAACAG GTTCTTGTTG CTAAA 165

SEQ ID NO:1884
SEQUENCE LENGTH:168
SEQUENCE TYPE:nucleic acid
TOPOLOGY:linear
CLONE:HUMGS02137
SEQUENCE DESCRIPTION:
GATCTGACCT CCACGGAGCC GCTGTCCCCG NCCCCCTTGC TTCCCGTCTG TCTGTCCTGT 60
CTGATTCTNT TAGGTGTCAT GTTCTTTTTT CTGTCTTGTC TTCAACTTTT TTTAAAACTA 120
GATTGCTTTG AAAACATGAC TCAATAAAAG TTTCCTGNCC AATTTAAA 168

SEQ ID NO:1885
SEQUENCE LENGTH:163
SEQUENCE TYPE:nucleic acid
TOPOLOGY:linear
CLONE:HUMGS02138
SEQUENCE DESCRIPTION:
GATCACGATG GCTTCACAAG CCAGCTGTTG GGTTTGCTAT GTCACTGTGG CTCAGTCACA 60
TCCCTGCGTG TATACTGTCT GCGGGGCACA TATGTATCCA TTTAGAGCTA AAGGAATCAG 120
TGTACACTAC AGCTAATCCT AATAAATCCG ATGTTTTCGG AAA 163

SEQ ID NO:1886
SEQUENCE LENGTH:159
SEQUENCE TYPE:nucleic acid

TOPOLOGY:linear
CLONE:HUMGS02139
SEQUENCE DESCRIPTION:
GATCTAGCAG CATGCAAACA CATCACCTAA ACTACAAAAG AAAGGTCCTC ACTGTCTCTA 60
AAAAATAAAC TCATTAACTT TGAGTTATTT GCAGACAGCC AAATCACCCA GGGCAACTTT 120
AAAGAGTCAG AAAATAAAAC TTTCCCGTTG TATGACAAA 159

SEQ ID NO:1887
SEQUENCE LENGTH:154
SEQUENCE TYPE:nucleic acid
TOPOLOGY:linear
CLONE:HUMGS02140
SEQUENCE DESCRIPTION:
GATCATGGAG GAAAAAGGTA CTGGATAAAA GTAAACTTCA AACCTTAGGG CGGGANACTA 60
AAACCAAAAT ACATGTATTA TTTATAGAAA ATATTTTCTG TTTTAATCTT TTCTNTTTAA 120
ACAAGGACTC ATACTTAAAA AAATGTTTAG CAAA 154

SEQ ID NO:1888
SEQUENCE LENGTH:151
SEQUENCE TYPE:nucleic acid
TOPOLOGY:linear
CLONE:HUMGS02141
SEQUENCE DESCRIPTION:
GATCTCTGAT GACCTAACCT AAGCAAAACC ACTGAGCTTC TGGGAAGACA ACTAGGATAC 60
TTTCTACTTT TTCTAGCTAC AATATCTTCA TACAATGACA AGTATGATGA TTTGCTATCA 120
AAATAAATTG AAATATAATG CAAACCATAA A 151

SEQ ID NO:1889
SEQUENCE LENGTH:150
SEQUENCE TYPE:nucleic acid
TOPOLOGY:linear
CLONE:HUMGS02142
SEQUENCE DESCRIPTION:
GATCTATATT CTTCTTAATT TTTATGGCTA TATTTAATGT TTTCCATCAT TGTGCCTTTA 60
AAAAGTATAT GTACATGTAT GCATAAATTT ATGTGAAGTT ATATATANGN NTCCGTTATT 120
CCAGATTAAA GTTTATCTTT AGTAATCAAA 150

SEQ ID NO:1890
SEQUENCE LENGTH:149
SEQUENCE TYPE:nucleic acid
TOPOLOGY:linear
CLONE:HUMGS02143
SEQUENCE DESCRIPTION:
GATCAAAAAT ATGTGACCTT AATGAGATTT TTATGATTTC TAAAGTAACA ATAAAAGCAG 60
TTTTTAGAGT TGAGTTCCAG AGAGGGCAGG GCAATGGCAG TNACATGTTT GTCATTTTAA 120

TAATAAATAA CATCTATTGA GTNCTTAAA 149

SEQ ID NO:1891
SEQUENCE LENGTH:148
SEQUENCE TYPE:nucleic acid
TOPOLOGY:linear
CLONE:HUMGS02144
SEQUENCE DESCRIPTION:
GATCCGCGGG GACCCTGCCG AGGGGGCTGT CATGCGGTTT CCAAGGTGCA CATTTNCCAC 60
GGAAACAGAA CTCGATGCAC TGANCTGCTC CGCCAGGANA GTGAGCGTGT AGGTNCCTGA 120
GGAATAAAAT GTTCCGATGA TGTGGAAA 148

SEQ ID NO:1892
SEQUENCE LENGTH:147
SEQUENCE TYPE:nucleic acid
TOPOLOGY:linear
CLONE:HUMGS02145
SEQUENCE DESCRIPTION:
GATCCCCGTT CTTCAAGCTT CTCTGCTCAG TGTCTACTAA CGACCGACAT TTGCTAATGT 60
AAATAATAGT AAATNATTGA GAATTCTAAT TCTTTTACAC AGTCTGTTTT TAATCTATTT 120
TAATTAAATA AAATCTATGA CTCTAAA 147

SEQ ID NO:1893
SEQUENCE LENGTH:146
SEQUENCE TYPE:nucleic acid
TOPOLOGY:linear
CLONE:HUMGS02146
SEQUENCE DESCRIPTION:
GATCTGGGAG TAAACTTAAC ATTCACTGTG TCTCTGCTCT GCATCCGCCA TTTGTGTGTG 60
TTTCTGGACT GTGGGCTGTG TGTACCTTGG TTGGTGACTC AGTGAGAAGA AGCAGGAATN 120
CCAAAGATAC TGTGAATNTT CTGAAA 146

SEQ ID NO:1894
SEQUENCE LENGTH:144
SEQUENCE TYPE:nucleic acid
TOPOLOGY:linear
CLONE:HUMGS02147
SEQUENCE DESCRIPTION:
GATCTAAATG TCTAGTTTTG TATTCTTTTG TGTGTGTTCA CTGTTTCTCA GTATTACCAC 60
TTGAATAATT CTCTGTACAG GGGGGTTTGT GCTATACACT GGGATGTCTA ATTGCAGCAA 120
TAAAGCCTTT CTTTAAAAAG GAAA 144

SEQ ID NO:1895
SEQUENCE LENGTH:141
SEQUENCE TYPE:nucleic acid

TOPOLOGY:linear
CLONE:HUMGS02148
SEQUENCE DESCRIPTION:
GATCTTGGAA ATGCTTGAAG ACCACAAGGC TGAAAAGTGC TTTGCTGGAG TCCTGTTCTC 60
AGAGCTCCAC AGAAGACACG TGTTTTTGTA TCTTTAAAGA CTTGATGAAT AAACACTTTT 120
TCTGGTCAAT GTCAAANNAA A 141

SEQ ID NO:1896
SEQUENCE LENGTH:142
SEQUENCE TYPE:nucleic acid
TOPOLOGY:linear
CLONE:HUMGS02149
SEQUENCE DESCRIPTION:
GATCCAGATA AACTTTCCTG TCATGTGTTA GAACTTTATT ATTATTAATA TTGTTAAACT 60
TCTGTGCTGT TCCTGTGAAT CTCCAAATTT TGTACCTTGT TCTAAGCTAA TATATAGCAA 120
TTAAAAAGAG AGAAAGAGGA AA 142

SEQ ID NO:1897
SEQUENCE LENGTH:133
SEQUENCE TYPE:nucleic acid
TOPOLOGY:linear
CLONE:HUMGS02150
SEQUENCE DESCRIPTION:
GATCAAAGTG TGGGATGNGC TGCAAGACGT AGAACCTACC TGCCCTGCCC CCGTCCCCNC 60
CCTTCCTTAT TTATTCCTGC TGCCCCAGAA CATAGGTCTT GGAATAAAAT GGCTGGTTCT 120
TTTGTTTTCC AAA 133

SEQ ID NO:1898
SEQUENCE LENGTH:130
SEQUENCE TYPE:nucleic acid
TOPOLOGY:linear
CLONE:HUMGS02151
SEQUENCE DESCRIPTION:
GATCTAAGTT GCATCTTTAA TCCTGGTGGT CTTGCCTTCT GATTTTTAAT TTGTATCCTT 60
TTCTATTAAG ATATATTTGT CATTTTCTCT TGAATATGTA TTAAAATATC CCAAGCAATC 120
CAGTAACAAA 130

SEQ ID NO:1899
SEQUENCE LENGTH:247
SEQUENCE TYPE:nucleic acid
TOPOLOGY:linear
CLONE:HUMGS02152
SEQUENCE DESCRIPTION:
GATCGAGGAG ATGCCCTCTG AACGCCTGTC CCGGAGCACC CGCCAGCGGG CAGCCTGTGC 60
CCAGCTCAAC GACTTCCTCC AGGAGTATGG CACTCAGGGG TGCCAGGTCN TNAGGGCTGC 120

CCTCCCACNN CCGCTGGGAG GAACCTNAAC CTGGGAACCA TGAAGCTGGA AGCACTGCTG 180
TGTCCGCTTT NATGAACACA GCCTGGGACC AGGGCATATN AAAGGCTTTT GGCAGCAAAG 240
TGTCAAA 247

SEQ ID NO:1900
SEQUENCE LENGTH:132
SEQUENCE TYPE:nucleic acid
TOPOLOGY:linear
CLONE:HUMGS02153
SEQUENCE DESCRIPTION:
GATCTTCCTG CTACCCTCTT GGATTCTAAG TGGTTCCAAG CTTAACTTGA GACCTTCCCT 60
TCAAATCTAA AATTGGCAAA AAGTCACTTA AAATAGTGGA CTTCTGTAAT AAAGGTTGCC 120
TAAAATAACA AA 132

SEQ ID NO:1901
SEQUENCE LENGTH:128
SEQUENCE TYPE:nucleic acid
TOPOLOGY:linear
CLONE:HUMGS02154
SEQUENCE DESCRIPTION:
GATCTACAGG TTNCAATTCA ACCTGTTTCT AGGTTTTTTT GTAAATTTAG TTTTGATTAA 60
GCATTATAAG CATTTGAGTC TATAAACTTT ATAGTAGCAT CTTTCAGAAT AAACATTTTT 120
AATTGAAA 128

SEQ ID NO:1902
SEQUENCE LENGTH:132
SEQUENCE TYPE:nucleic acid
TOPOLOGY:linear
CLONE:HUMGS02155
SEQUENCE DESCRIPTION:
GATCCATGAG TTTGCCCTGG TTTCACTGGC CCAAGTGGTT TGTGCTAACC ACGTCTGTCT 60
TCACAGCTCT GTGTTGCCAT GTGTGCTGAA CAAAAAATAA AAATTATTAT TGATTTTATA 120
TTTCAGGGGA AA 132

SEQ ID NO:1903
SEQUENCE LENGTH:128
SEQUENCE TYPE:nucleic acid
TOPOLOGY:linear
CLONE:HUMGS02156
SEQUENCE DESCRIPTION:
GATCCAAGAC AGAATAATGA ATGGACTCAA ACTGCCTTGG CTTCAGGGGA GTCCCGTCAG 60
GNCGTTGAGG ACTTTTCGAC CAATTCAACC CTTTGCCCCA CCTTTATTAA AATCTTAAAC 120
AACGGAAA 128

SEQ ID NO:1904

SEQUENCE LENGTH:126
SEQUENCE TYPE:nucleic acid
TOPOLOGY:linear
CLONE:HUMGS02157
SEQUENCE DESCRIPTION:
GATCCACAGG GGTGGTGTCA AATGCTATTG AAATTGTGTT GAATTGTATG CTTTTTCACT 60
TTTGATAAAT AAACATGTAA AAATGTTTCA AAAAAATAAT AAAATAAATA AATATGAAAC 120
CTTAAA 126

SEQ ID NO:1905
SEQUENCE LENGTH:122
SEQUENCE TYPE:nucleic acid
TOPOLOGY:linear
CLONE:HUMGS02158
SEQUENCE DESCRIPTION:
GATCTACAGA TGAATATTTA ACTCAATAGA AAAATTATTT TAGAACACAT TGTATTGGTA 60
TTACAACCAG ATTATATTCT TGACGTTGAC TTCATTAAAA TTATCTACAA TTTCCTAATA 120
AA 122

SEQ ID NO:1906
SEQUENCE LENGTH:121
SEQUENCE TYPE:nucleic acid
TOPOLOGY:linear
CLONE:HUMGS02159
SEQUENCE DESCRIPTION:
GATCTCATGG ATACAAACAG GTGACCTTGC CAATTGGTGC TTTTTGTGTG TTTAAGAAAA 60
GAACAGTAGT AACCTTGCTC CCTGGNTACC AATAAAAAAT CTATTATTTT TTTAAAAGAA 120
A 121

SEQ ID NO:1907
SEQUENCE LENGTH:119
SEQUENCE TYPE:nucleic acid
TOPOLOGY:linear
CLONE:HUMGS02160
SEQUENCE DESCRIPTION:
GATCTGACCC CTGTCAGATG NAAATGATTC ACAGCTCTGG CAGTTCCCAA GTCTGGGGAG 60
GGGTATAGGT TTGAAAGGCT GTTTGAAAGA GGAATGTTTA ATAAAGGCTT TGATTTAAA 119

SEQ ID NO:1908
SEQUENCE LENGTH:121
SEQUENCE TYPE:nucleic acid
TOPOLOGY:linear
CLONE:HUMGS02161
SEQUENCE DESCRIPTION:
GATCCCAGCT GGCCCCTATG TTGTGTTCTG NGACTGAGGC CTTTGCTGTG AACTGCAGTG 60

TTTCATACGA ACCATCTTTC CTAGTGCATG AGAAATAAAG ATTATTTAAG TAATGAGGAA 120
A 121

SEQ ID NO:1909
SEQUENCE LENGTH:116
SEQUENCE TYPE:nucleic acid
TOPOLOGY:linear
CLONE:HUMGS02162
SEQUENCE DESCRIPTION:
GATCTCACTT TTCAGGAAAC TTAAAATTTA CCCATTATTG GGAAGCTTAA ATGAATTGAA 60
GCTATGCAAG TCTTTTATAT TATTAAATAT TTAAAAGTAA ACCTGTACTA ATCAAA 116

SEQ ID NO:1910
SEQUENCE LENGTH:116
SEQUENCE TYPE:nucleic acid
TOPOLOGY:linear
CLONE:HUMGS02163
SEQUENCE DESCRIPTION:
GATCGATATG CTCATAAAAG TGCTCAATTA TATTTNCTGT ATTTTGTATG NTGTATTTTC 60
CAAGACGTAT ATTATTTTAC TATTAAAGAA AAAAATCATT TTTTTTTCCC GAGAAA 116

SEQ ID NO:1911
SEQUENCE LENGTH:124
SEQUENCE TYPE:nucleic acid
TOPOLOGY:linear
CLONE:HUMGS02164
SEQUENCE DESCRIPTION:
GATCCGGGCA TGGGAGACCC CACTTTAGAA AGGGTCGTCA CTCCTTTAAT CCTCTACTCA 60
ACAATGTACT CTTTTACTTT TATATTAAAA AAAATAAAAT AAATATGTGC CTAAAACCTC 120
CAAA 124

SEQ ID NO:1912
SEQUENCE LENGTH:115
SEQUENCE TYPE:nucleic acid
TOPOLOGY:linear
CLONE:HUMGS02165
SEQUENCE DESCRIPTION:
GATCCACTTC TACTTAATGA GTAGTAAATA TATTTCNTTT TCCTTATGAT TTTCTTACTA 60
TTTNCTTTAG CTTGCTTTAT TATAAGANTA TAGCATATAA TATAAATAAA ATAAA 115

SEQ ID NO:1913
SEQUENCE LENGTH:112
SEQUENCE TYPE:nucleic acid
TOPOLOGY:linear
CLONE:HUMGS02166

SEQUENCE DESCRIPTION:
GATCCAGAAA TTGTGAACAA TGTTTGATTT AGTAGCGTGA CTTGCCTTTC CCTTTAAAAA 60
CATCTTTTAC AAATCTGTCT TGGAATAAAG TCTATTTTCT GCCTTTTGGA AA 112

SEQ ID NO:1914
SEQUENCE LENGTH:112
SEQUENCE TYPE:nucleic acid
TOPOLOGY:linear
CLONE:HUMGS02167
SEQUENCE DESCRIPTION:
GATCCTAATT GCCTAGAAAT ATCTTTCTCT TACCTGTTAT TTATCAATTT TNCCCAGTAT 60
TTTTATACGG AAAAAATTGT ATTGAAAACA CTTAGTATGC AGTTGANNNN GN 112

SEQ ID NO:1915
SEQUENCE LENGTH:112
SEQUENCE TYPE:nucleic acid
TOPOLOGY:linear
CLONE:HUMGS02168
SEQUENCE DESCRIPTION:
GATCAACCGT GAAGCGTAAC AAATTCTGTA TTTAGTTTTT TTTTCTGTTG TGGTGGTTTT 60
TGTTTTGTTT TTTGTTTTTG TAAGATTCTA AATAAATNAA ATACGAGTCA AA 112

SEQ ID NO:1916
SEQUENCE LENGTH:112
SEQUENCE TYPE:nucleic acid
TOPOLOGY:linear
CLONE:HUMGS02169
SEQUENCE DESCRIPTION:
GATCAAGACT NCAGTTTGGG AATGCATGGA CACCGGATTT GTTTCTTATT CCTTCACTTT 60
TGGGGAAAAT CTCNCCNTNT TTAAAAAGTG ATAAATTTGG TGTTAGGTCA AA 112

SEQ ID NO:1917
SEQUENCE LENGTH:112
SEQUENCE TYPE:nucleic acid
TOPOLOGY:linear
CLONE:HUMGS02170
SEQUENCE DESCRIPTION:
GATCAACGCG ACTGTATTTT GAAACATTCC AGGAAGGTTA CTTCTTGTCA AACTTGCCTG 60
GCAGTGTTTG TTCAAANCTT GTATTTAATA AATGANCATC TGACTTTACA AA 112

SEQ ID NO:1918
SEQUENCE LENGTH:162
SEQUENCE TYPE:nucleic acid
TOPOLOGY:linear
CLONE:HUMGS02171

SEQUENCE DESCRIPTION:
GATCTATCTT AAGAAGTGAC CAGGAAAGAG GTTCATTGAA ATAATCATGC ATGAAGCGCC 60
AAAGATGCAC CATGTAGAAT TTTCACTTTG TACTGGCAGG CTCGTTTTAC CTGATTCTAG 120
AATATTTAAG AATCTAAAAA TAAAGGGCAA CTCTGACTTA AA 162

SEQ ID NO:1919
SEQUENCE LENGTH:111
SEQUENCE TYPE:nucleic acid
TOPOLOGY:linear
CLONE:HUMGS02172
SEQUENCE DESCRIPTION:
GATCAGGGAA CTAAGCATTA TGTGAATTCA CCAGCAAGAT GTACAGNACG CCTGTNTTTA 60
CATTGTTTTT ATGGAACTAG CAGAATAAAN CATCTATTTT AAAAATGCAA A 111

SEQ ID NO:1920
SEQUENCE LENGTH:275
SEQUENCE TYPE:nucleic acid
TOPOLOGY:linear
CLONE:HUMGS02173
SEQUENCE DESCRIPTION:
GATCTGCGAG GAAGCCGCCT ACTCCAACCC CAGCCTACCT NTGGTGCACA NATCCGTCCC 60
ATAGCAAAGC CCCTGCACAG ACTCCAGCCG AGCCCACACC TGGNTATNAG GTGGGCCAGC 120
GGAAGCGCCT CATCTCCTCG GTGGAGGACT TCACCGAGTT TNTNTGAGGC CGGGGCCCTC 180
CCTCCTGCAC TGGCCTTGGA CGGTATTGCC TGTCAGTGAA ATAAATAAAG TCCTGACCCT 240
AGTGCACAGA CATAGAGGCA CAGATTGCAG TCAAA 275

SEQ ID NO:1921
SEQUENCE LENGTH:114
SEQUENCE TYPE:nucleic acid
TOPOLOGY:linear
CLONE:HUMGS02174
SEQUENCE DESCRIPTION:
GATCAGGACA GAGACTTGGG GGCCATCCTG CCCCTCCAAC CCGGACATGT GTACCTCAGC 60
TTTTTCCCTC ACGTTGCATC AATAAAGCTT CTGTGTTTGG AACAGNTAAA CAAA 114

SEQ ID NO:1922
SEQUENCE LENGTH:108
SEQUENCE TYPE:nucleic acid
TOPOLOGY:linear
CLONE:HUMGS02175
SEQUENCE DESCRIPTION:
GATCCCAAGC NTCCCGTCCC TTGGAAAGTN CAGCCAGAAT CTNCGTCCTG ACCCCCGACA 60
GCCCAGCCTA GCCCACTTGT CATCCATAAA GCAAGCTCAA CCTTGAAA 108

SEQ ID NO:1923

SEQUENCE LENGTH:272
SEQUENCE TYPE:nucleic acid
TOPOLOGY:linear
CLONE:HUMGS02176
SEQUENCE DESCRIPTION:
GATCCCCCAG GTTCAGACTG AGCTCCCCCT TCCCAGTAGC TCTTGCATCC TCCTCCCAAC 60
TCTAGCCTGA NTNCTTTNCA ATAAAAAATA CAATTCAAGT TGCTTCTCAT GGATGGCACT 120
GCTTTCCTGA GGACTCAAGG TGCCAAGATG GAGGGGCTGA CTCAGTCCAG CCAACNTTTA 180
ATGAGNACCT ANNNGGTGTA TGGAGCTCTA ACCCATGGGT CCATGGGAAT AAAGCAGTGA 240
ATAGTAACAA TAAATAATTG TAACAGCANA AA 272

SEQ ID NO:1924
SEQUENCE LENGTH:109
SEQUENCE TYPE:nucleic acid
TOPOLOGY:linear
CLONE:HUMGS02177
SEQUENCE DESCRIPTION:
GATCTGACGT TTTCTACGTA GCTTTTGTAT TTTTTTTTTT TAATTTNAAG GAACACTTAT 60
GAAGCCCTGC CATNCCCCTC CCGNGTCTAA TAAAACGTAT AATCACAAA 109

SEQ ID NO:1925
SEQUENCE LENGTH:104
SEQUENCE TYPE:nucleic acid
TOPOLOGY:linear
CLONE:HUMGS02178
SEQUENCE DESCRIPTION:
GATCAACTTG TGCTTGCCAG AGGACGCCAA TGAAGTTTGA AACACCAACA ATCAGAGATT 60
TTGTTTCTGT TCCTCATTAA ATCATGAGCT TTTGTGCCGA GAAA 104

SEQ ID NO:1926
SEQUENCE LENGTH:104
SEQUENCE TYPE:nucleic acid
TOPOLOGY:linear
CLONE:HUMGS02179
SEQUENCE DESCRIPTION:
GATCGTGTGT GTAGGTGGTG TTGTGTGGTT TTCCTTTGTG AAGGAGAGAG GGAAACTATN 60
TGTAGCTTGT TTTATAAAAA ATAAAAAATG GGTAAATCTT GAAA 104

SEQ ID NO:1927
SEQUENCE LENGTH:103
SEQUENCE TYPE:nucleic acid
TOPOLOGY:linear
CLONE:HUMGS02180
SEQUENCE DESCRIPTION:
GATCCTCCCA CTTCCACCTC CCAAGTAGCT GTGGCTACAG GTGTGTGCCA CCATGTCCAG 60

CTGATTTTNG TATTTTNAGT AGGGACAGTA TTTCTCCATG AAA 103

SEQ ID NO:1928
SEQUENCE LENGTH:106
SEQUENCE TYPE:nucleic acid
TOPOLOGY:linear
CLONE:HUMGS02181
SEQUENCE DESCRIPTION:
GATCTAAATC TCATTTTATG CTGTATTTTA TATCTNAGTT GTNTTTGAAA ACGTTTTGAT 60
TTTTGGAAAC ACATCAAAAT AAATAATGGC GTTTNTTGTA TGCAAA 106

SEQ ID NO:1929
SEQUENCE LENGTH:272
SEQUENCE TYPE:nucleic acid
TOPOLOGY:linear
CLONE:HUMGS02182
SEQUENCE DESCRIPTION:
GATCTGGACG AATGATACCA AACGTTTCTT TCTAACATTG TATTTTATAA CTCTGGCCTA 60
TGATTGTNTT GTGTCTTGCA TTAAAAAAAA AAATTTGAGA GTGGTAGAAT TACTTCTGTT 120
ATCTGAAATA CCTGAGATGC ACTTTAAACT GTTGAAATGT AAGCNCTGAG GGCAGGGNCC 180
GTATCTNATT TACTGTTAGT ATCCGTTGCA TCTAGTGTGG TGCACCTGGC ACACAGTAGG 240
CACTCAATAA ATATGTATTG ATTAAAANCA AA 272

SEQ ID NO:1930
SEQUENCE LENGTH:171
SEQUENCE TYPE:nucleic acid
TOPOLOGY:linear
CLONE:HUMGS02183
SEQUENCE DESCRIPTION:
GATCTACAGC TCCTGGATAA TTGCTTGATT CTCGCCAGAG ACTGTCAATA TTTNATTCAT 60
TGCCATTACC AATAAATCAT TGCTTTTGTT NAGATGGTAT CACTAGTGTT TCCATTGTAA 120
TCTTGACACA TGCAATTGTA AATAAAAGTC ACCACTTTTG CCAAGCTTAA A 171

SEQ ID NO:1931
SEQUENCE LENGTH:99
SEQUENCE TYPE:nucleic acid
TOPOLOGY:linear
CLONE:HUMGS02184
SEQUENCE DESCRIPTION:
GATCATACCA CTGCACTCTA GTCTGGGTGA CAGAGCGAGA CCCTGTCTTG AAAAAAATAA 60
ATAAAAATAA ATAAAATAAA AACTGGCACT ACGTTTAAA 99

SEQ ID NO:1932
SEQUENCE LENGTH:99
SEQUENCE TYPE:nucleic acid

TOPOLOGY:linear
CLONE:HUMGS02185
SEQUENCE DESCRIPTION:
GATCTGTGTC TTAAAACTTA CTGGAATGGA AATCTATGAA TTATTGCAAA TTGTAATGCT 60
GGAAACAAAA AATAAATCCT TGGTTAAAGG GTTTGTAAA 99

SEQ ID NO:1933
SEQUENCE LENGTH:99
SEQUENCE TYPE:nucleic acid
TOPOLOGY:linear
CLONE:HUMGS02186
SEQUENCE DESCRIPTION:
GATCCTGACA CTCTANCACT CGACTCTGCT GCTCATGGGA AGAACAGAAT TNCTCCTGCA 60
TGCAACTAAT TCAATAAAAC TGTCTTGTGA GCTGATAAA 99

SEQ ID NO:1934
SEQUENCE LENGTH:90
SEQUENCE TYPE:nucleic acid
TOPOLOGY:linear
CLONE:HUMGS02187
SEQUENCE DESCRIPTION:
GATCTTGACT TATATTCTGT ACCACATACA CTTCTGTGGA CTTTTAGCAT TTGTGGGTAG 60
ACTTAATAAA GCATGTATAA AAGTGGCAAA 90

SEQ ID NO:1935
SEQUENCE LENGTH:91
SEQUENCE TYPE:nucleic acid
TOPOLOGY:linear
CLONE:HUMGS02188
SEQUENCE DESCRIPTION:
GATCAGGNTG CGGATAGACT TGTAAGGGTG TTTGCTGCAT ACAGTGTAAG CATTGTGACC 60
GCCAATAANC TTCAATGGTT TCTACTGNAA A 91

SEQ ID NO:1936
SEQUENCE LENGTH:91
SEQUENCE TYPE:nucleic acid
TOPOLOGY:linear
CLONE:HUMGS02189
SEQUENCE DESCRIPTION:
GATCTTTCGT NTTTCTAAGC GACTCTACTT TCATTGTTTG CCAGCNTAGC TCGTTGCTGT 60
TGCCCAATAA AGCTNGTGTA CGTNCTGCAA A 91

SEQ ID NO:1937
SEQUENCE LENGTH:86
SEQUENCE TYPE:nucleic acid

TOPOLOGY:linear
CLONE:HUMGS02191
SEQUENCE DESCRIPTION:
GATCTTACAT CTGCAGCTCT TTCTTCTTTG AATTTCCTAT CTGTATGTCT GCCTAATTAA 60
AAAAATATAT ATTGTATTAT GCTAAA 86

SEQ ID NO:1938
SEQUENCE LENGTH:86
SEQUENCE TYPE:nucleic acid
TOPOLOGY:linear
CLONE:HUMGS02192
SEQUENCE DESCRIPTION:
GATCATCAGT GTTCCTTTTT ACTTATAAAG TTGGATTCTT TTTTAGAATT TGTAATAAAT 60
AAAAACTGCT GCTTTACCAC TGTAAA 86

SEQ ID NO:1939
SEQUENCE LENGTH:91
SEQUENCE TYPE:nucleic acid
TOPOLOGY:linear
CLONE:HUMGS02193
SEQUENCE DESCRIPTION:
GATCTACCCT GCCTGTACTA CTCTAGGGAG TATGCTGGAG GCAGAGGGCA AGGGAGGGGT 60
GGTATTAAAC AAGTCAATTC TGTGTNGTAA A 91

SEQ ID NO:1940
SEQUENCE LENGTH:84
SEQUENCE TYPE:nucleic acid
TOPOLOGY:linear
CLONE:HUMGS02194
SEQUENCE DESCRIPTION:
GATCATGAGT GACCCAGCTG TCTCACGTTG TTTATTCTGC GTCCCCTTCT CCAATAAAAC 60
AAGCCAGTTG GGCGTNGTTA TAAA 84

SEQ ID NO:1941
SEQUENCE LENGTH:79
SEQUENCE TYPE:nucleic acid
TOPOLOGY:linear
CLONE:HUMGS02195
SEQUENCE DESCRIPTION:
GATCCCCATG CCTAGCAGAG TGCTGGCACT TAGTAGGTCC TCAATAAATA TTTATTAAAT 60
GATGAGATGA TGGAGCAAA 79

SEQ ID NO:1942
SEQUENCE LENGTH:78
SEQUENCE TYPE:nucleic acid

TOPOLOGY:linear
CLONE:HUMGS02196
SEQUENCE DESCRIPTION:
GATCCAATCC CATAGACAGC TCTGGGCCTC TTGCATTTGA GTTTTTTAGA ATTAAACTGC 60
AGTATTTTGG AAAGCAAA 78

SEQ ID NO:1943
SEQUENCE LENGTH:77
SEQUENCE TYPE:nucleic acid
TOPOLOGY:linear
CLONE:HUMGS02197
SEQUENCE DESCRIPTION:
GATCCACTAA GGAAATGTTT TAACAGCTAG AGACCACTGC TTGCCTGAAA GGGCGTTCTT 60
AAATTTGGTG CAGCAAA 77

SEQ ID NO:1944
SEQUENCE LENGTH:76
SEQUENCE TYPE:nucleic acid
TOPOLOGY:linear
CLONE:HUMGS02198
SEQUENCE DESCRIPTION:
GATCCACTTC AAGAATTGCT TGCTTTCAGG AAGAGAGATG TGTTTCAACA AGCCAACTAA 60
AATATATTGC TGCAAA 76

SEQ ID NO:1945
SEQUENCE LENGTH:76
SEQUENCE TYPE:nucleic acid
TOPOLOGY:linear
CLONE:HUMGS02199
SEQUENCE DESCRIPTION:
GATCGACTTT GTGTCTCTGT TGTCTAAAAT AGGTTTTCCC TGTTCTGGAC ATTTCATATA 60
AATGGAATCA CACAAA 76

SEQ ID NO:1946
SEQUENCE LENGTH:75
SEQUENCE TYPE:nucleic acid
TOPOLOGY:linear
CLONE:HUMGS02200
SEQUENCE DESCRIPTION:
GATCATAGAT TTCTGATTTT ATGTAAAATT TTGCCTAATA CATTAAAGCA GTCACTTTTC 60
CTGTGCTGTT TCAAA 75

SEQ ID NO:1947
SEQUENCE LENGTH:74
SEQUENCE TYPE:nucleic acid

TOPOLOGY:linear
CLONE:HUMGS02202
SEQUENCE DESCRIPTION:
GATCTGTCAA AGAAAACTTC CAAAAAGATT TATTAATTAA ACCAGACTCT GTTGCAATAA 60
AAATCCTACA GAAA 74

SEQ ID NO:1948
SEQUENCE LENGTH:73
SEQUENCE TYPE:nucleic acid
TOPOLOGY:linear
CLONE:HUMGS02203
SEQUENCE DESCRIPTION:
GATCCACTTT AGCAAACCCT GCTGCAGAGG ACTGTAAAAA CAAATAACTA AAAATAAACT 60
TAGAAAATAC AAA 73

SEQ ID NO:1949
SEQUENCE LENGTH:73
SEQUENCE TYPE:nucleic acid
TOPOLOGY:linear
CLONE:HUMGS02204
SEQUENCE DESCRIPTION:
GATCGTAATG TTTATCATGT TACTTCCCCA CCCCTACATT TTTTGAAATA AAATAAGGAA 60
TTTTATTCTC AAA 73

SEQ ID NO:1950
SEQUENCE LENGTH:72
SEQUENCE TYPE:nucleic acid
TOPOLOGY:linear
CLONE:HUMGS02205
SEQUENCE DESCRIPTION:
GATCNCTGCA ACTAAGNTTA AAGCAGTGTG ACTGTGTTGC TNAAATATCA AGTATTGTTT 60
ATAACCACCA AA 72

SEQ ID NO:1951
SEQUENCE LENGTH:66
SEQUENCE TYPE:nucleic acid
TOPOLOGY:linear
CLONE:HUMGS02206
SEQUENCE DESCRIPTION:
GATCTCAGCT ATTAAGATGC AAGTCTGCCA ATGCTCCTGG CCAAATAAAC CTCTTCCTTC 60
TTTAAA 66

SEQ ID NO:1952
SEQUENCE LENGTH:65
SEQUENCE TYPE:nucleic acid

TOPOLOGY:linear
CLONE:HUMGS02207
SEQUENCE DESCRIPTION:
GATCCTCTTG TACTGGGAGA GGTGCCTTTT GTATCCCCAA TTAAAGGTAG AAAACCACCC 60
TGAAA 65

SEQ ID NO:1953
SEQUENCE LENGTH:62
SEQUENCE TYPE:nucleic acid
TOPOLOGY:linear
CLONE:HUMGS02208
SEQUENCE DESCRIPTION:
GATCTCGAAG AAAAAAGGGG GAGACCAAAG AATAAAAGAA GGGTTTCCCC ACTAAAATGA 60
AA 62

SEQ ID NO:1954
SEQUENCE LENGTH:62
SEQUENCE TYPE:nucleic acid
TOPOLOGY:linear
CLONE:HUMGS02209
SEQUENCE DESCRIPTION:
GATCCAGCTG TNCTTAAGAG CCAGTAATGT CTTAATAAAC ATGTGGCAGC TTTNNTTTGA 60
AA 62

SEQ ID NO:1955
SEQUENCE LENGTH:61
SEQUENCE TYPE:nucleic acid
TOPOLOGY:linear
CLONE:HUMGS02210
SEQUENCE DESCRIPTION:
GATCTCACAT TTCACCCCAG GCTCAACTGA GGATGTGGCT TATTAAACAC GGAAGTGCAA 60
A 61

SEQ ID NO:1956
SEQUENCE LENGTH:59
SEQUENCE TYPE:nucleic acid
TOPOLOGY:linear
CLONE:HUMGS02211
SEQUENCE DESCRIPTION:
GATCAGCCAT GTNTATCTTT TTAAAAAATA AACAGGTTGC TTTATTTCTT AGCTTCAAA 59

SEQ ID NO:1957
SEQUENCE LENGTH:59
SEQUENCE TYPE:nucleic acid
TOPOLOGY:linear

CLONE:HUMGS02212
SEQUENCE DESCRIPTION:
GATCAGATGA GATTTAATGA AGACCCAGTG TAAAGAATAA ATGANTCTTA CTCCTTAAA 59

SEQ ID NO:1958
SEQUENCE LENGTH:57
SEQUENCE TYPE:nucleic acid
TOPOLOGY:linear
CLONE:HUMGS02213
SEQUENCE DESCRIPTION:
GATCTACACC TGGACTGCCC AGGTCTATAA GCCAATAAAG CCCCTGTTTA CTTGAAA 57

SEQ ID NO:1959
SEQUENCE LENGTH:56
SEQUENCE TYPE:nucleic acid
TOPOLOGY:linear
CLONE:HUMGS02214
SEQUENCE DESCRIPTION:
GATCGAAGAA AAAGGGGNGN CAAAGAATAA AAGAAGGGTT TCCCACTAAA ATGAAA 56

SEQ ID NO:1960
SEQUENCE LENGTH:111
SEQUENCE TYPE:nucleic acid
TOPOLOGY:linear
CLONE:HUMGS02215
SEQUENCE DESCRIPTION:
GATCAGAAAT GTTGANTGTG CATTGAGTAT TAAAAAATTA GATGTATATT ATNCATTGTC 60
CTTTACTCAT GAGTACCTTA TAATANTANT ANTGTATTCT TTGTTAACAA A 111

SEQ ID NO:1961
SEQUENCE LENGTH:167
SEQUENCE TYPE:nucleic acid
TOPOLOGY:linear
CLONE:HUMGS02216
SEQUENCE DESCRIPTION:
GATCTATTCC CCATCCCAAG GCAAGCATGA ATAAAATTAG GTTAAACGTA GCATGTGGCA 60
TCGCAGTCTC TTAGAATTTG TTTCATCTAT TTNATTTTAT TGAATACTGT CTGTATCTTT 120
GGTTATCCTG TTTGAAGAAA AAGGACAAAT AAAACATGGC CAGCAAA 167

SEQ ID NO:1962
SEQUENCE LENGTH:516
SEQUENCE TYPE:nucleic acid
TOPOLOGY:linear
CLONE:HUMGS02237
SEQUENCE DESCRIPTION:

GATCCTCATT CTCACCCATT TGGCTAATCC AGGAATATTG TTATCCCTNA NCCATTATAT 60
TNAAGTTGAG AAATGTGACA GAGGCATTTA GAGTATGGAC TTTCCTTTCC TTTTNCTTTT 120
NCTTTTTTCC TTTTTGAGAT GGAGTCACAC TCTCCAGGCT GGAGTNCAGT GGCACAATCT 180
CGGCTCACTG CAATTNCCGT CTCCCAAGTT CAAGCGATTC TCCTGCTTTA GACTATGGAT 240
TTCTTTAAGG AATACTGGTT TGCAGTTTTG TTTTCTGGAC TATATCAGCA GATGGTAGAC 300
AGTGTTTATG TAGATGTGTT GTTGTTTTTA TCATTGGGAT TTTAACTTGG NCCGAGTNAA 360
ATAATCAGGT TTTTGTCATT CACACTCTCC CCCAGTTTTT GGGAATAACT TTGGAAGGTA 420
AGGTTCANTN CCCTAAGGCC GGTGGGGTTN CTGTTGGACC TATGGGGGGN CCACAACTGN 480
ACTATTTAAT TCCNCCCCAC TGTAAGGGGC AAAGGN 516

SEQ ID NO:1963
SEQUENCE LENGTH:483
SEQUENCE TYPE:nucleic acid
TOPOLOGY:linear
CLONE:HUMGS02238
SEQUENCE DESCRIPTION:
GATCTTTCCA GATGACTTGT CCAAGGTCAC ACAGGAGTCA GATGGCCTTT CCACTACCCC 60
ACAACACAGA TGCATAGTTA CTCTGTGGGC TAATACAATT CAGAATTCAG AGCTCTGCCC 120
ATGGTCACCG GTGGAATCCA CCAGCCATCT CCCTGACCTT GCTCTCCTCT GTTCTCCTCG 180
TCTCCAGGGT CCCATGATTC AGGGCGAGCT GGCTTCAGGA TGTGGGCAAG AGGGGTTTTA 240
GATGGAAGGG TTTTCCTCCA GGACAGAATG TTAAGCCAAG CCTGGACTTA ATTGTTCCAA 300
GATGTCGTTA GAATTGGTGG ACAATTTTTT TTTAATGATT AGCAAAGGAC AAGGCTTTTG 360
GTGGTCATTA GTATTGTTCA TCAAATATTC CAGTTCTCCA CAATTCTGGG GTACCATGGT 420
ANGGATGAAC CTTCCCTGTC TTTTCAGGGT TAAGTTTAGC TCTNGTGATG TGCTTTTGTC 480
AAA 483

SEQ ID NO:1964
SEQUENCE LENGTH:452
SEQUENCE TYPE:nucleic acid
TOPOLOGY:linear
CLONE:HUMGS02239
SEQUENCE DESCRIPTION:
GATCGCTGGA GCTGTGGTCG CTGCTGTGAT GTGGAGGAGG AAGAGCTCAG ATAGAAAAGG 60
AGGGAGCTAC TCTCAGGCTG CAAGCAGTGA CAGTGCCCAG GGCTCTGATA TGTCTCTCAC 120
AGCTTGTAAA GTGTGAGACA GCTGCCTTGT GTGGGACTGA GAGGCAAGAT TTGTTCCTGC 180
CCTTCCCTTT GTGACTTGAA GAACCCTGAC TTTGTTTCTG CAAAGGCACC TGCATGTGTC 240
TGTGTTCTTG TAGGCATAAT GTGAGGAGGT GGGGNGACCA CCCCACCCNN ATGTCCACCA 300
TGACCCTCTT CCCACGNTGA CCTGTGCTCC CTCCCCAATC ATCTNTCCTG TTCCAGAGAG 360
GTGGGGCTGA GGTGTCTCCA TCTCTGCCTC AACTTCATGG TGCACTGAGC TGTAACTNTT 420
TCCTGNNTAT TAAAATAAGA CCTGAGTTTA AA 452

SEQ ID NO:1965
SEQUENCE LENGTH:433
SEQUENCE TYPE:nucleic acid
TOPOLOGY:linear

CLONE:HUMGS02240
SEQUENCE DESCRIPTION:
GATCAGACAC CAGAGGGTTG GGCATTCTTG CAATACCTTA ACAGTGCTGA AATCTGCAGC 60
ATGGTACTAA GGAAGTTAAA GTTTGAATGT AACCACTTTA TTTAAAAGGT TTTTTTCTTT 120
AATTTAAATG AAATGGTGGT TGAAGTGAAC ATGATTTTGT TGACCATGTT CGTGAATTAC 180
AGATGCAACA TGCATTGGTA GAATCGTGTG ATGGTCTTTT GTGATACTTA ATTTTTACAT 240
ATCCCAGTCT CTGTATGTAT CTGCATAGAC AAAGAAAAAA CAAACTCCTG CTTTGCTTTT 300
ATTGAANGGG TTTCCAGGAC TGCGTGTCTG CTCCTGAGCT CTGTTTTAAG TATGTGTATC 360
CTTTGCTTGT ATTTTGTAAT TAAAAAAATA AGGAAAAGGA NGCCTTTATT TGTTGGNGCA 420
TGTTGGCATT GTN 433

SEQ ID NO:1966
SEQUENCE LENGTH:424
SEQUENCE TYPE:nucleic acid
TOPOLOGY:linear
CLONE:HUMGS02241
SEQUENCE DESCRIPTION:
GATCTGCAAA GAAAGCTTTT CTTNCGTCGC TGCCTCAGNC TCCTCCCTAT GCCTCTNGCA 60
CCTGCGCAGA AGTGCTGGCT GTGCTGANGT CACCATCATC TTCCTCTCCC CCAGCCTCCC 120
AGGCTGGATG GCATGGACTG TTTGCTGACC TCTGTTCTCT TAGGGCATGG GAGGTGGGAG 180
GATATCAAAT TCTNTAGCCC TTTCCTCCTA TTCTCCCAAG NAGAGAGNTT CCCATTTCTC 240
CTCGGCCATT GTACCTAGCT CTTGTCCCTA GCTGCATTTC AGTGGACCAT GGATAGAGGG 300
ACTGAGNGTT AGACGGGGAA GACTGGCAGN GAGGCACGCA GGTACTGTGA AAATCCTNNC 360
CTTTGACCTC CNCCAGTGGG AGAGGGGNGT TGGGGTTTTC AATGTGAGAA CAGNAACAAA 420
TAAN 424

SEQ ID NO:1967
SEQUENCE LENGTH:418
SEQUENCE TYPE:nucleic acid
TOPOLOGY:linear
CLONE:HUMGS02242
SEQUENCE DESCRIPTION:
GATCNCCTGC TCTCCAAGCA TGGCATCTAT GTGCAGGCCA TCAACTACCC AACTGTCCCC 60
CGGGGTGAAG AGCTCCTGCG CTTGGCACCC TCCCCCCACC ACAGCCCTCA GATGATGGAA 120
GATTTTGTGG AGANGNTGCT GCTGGCTTGG ACTGCGGTGG GGCTGCCCCT CCAGGATGTG 180
TCTGTGGCTG CCTGCAATTT CTGTCGCCGT CCTGTACACT TTGAGCTCAT GAGTGAGTNG 240
GAACGTTCCT ACTTCGGGAA CATGGGGCCC CAGTATGTCA CCACCTATGC CTGAGAAGCC 300
AGNTGCCTAG GATTCACACC CCACCTGCGN TTAACTTGGG TCCAGGCCTA CTCCTGTCTT 360
CTGCTTTGTT GTGTGCCTCT AGCTGAATTG AGCCTAAAAA TAAAGNACAA ACCACAAA 418

SEQ ID NO:1968
SEQUENCE LENGTH:413
SEQUENCE TYPE:nucleic acid
TOPOLOGY:linear
CLONE:HUMGS02243

SEQUENCE DESCRIPTION:
GATCATTGAA TAAAACGGGT GTAGAGTACA GGAATGGGGC AGACGCGATT CAGGTGAACA 60
GCACANGNAG AATATGAGGT GGTTCCTAGG AGCAACACTT TCGACCTCCA GTTCTCCNTG 120
ATGACAGTAG CTGTCTCCNN GNGAAAAATC CTCACTTATT AACTCTCTTT TCTTGCATCT 180
CATTTTTATA GAGCTACTCA TCCTTATTTG GAAAAACCAA CAACAAAAAA GGCTTTTAGA 240
AAATGGTTGT AAATCTGACT TCTTTGCAAG TAACTATGTA TATTGTAAAT AGGATATAAA 300
AAGGCCTTTT TTCTAAATAA GGCCTTAACT GCCTGTAACA TGAAACTTCA AACTAAACCA 360
CTTAACTCAN TGNACTACCT TATGGGTTTG TCTGACATCC CTCAACTTAC CAN 413

SEQ ID NO:1969
SEQUENCE LENGTH:412
SEQUENCE TYPE:nucleic acid
TOPOLOGY:linear
CLONE:HUMGS02244
SEQUENCE DESCRIPTION:
GATCCCACCG AGAAGAACCA TGGGTGGACC CGAACTCCCC GGTGCTCTNA GAGGACCCAN 60
NNCTTTGTGC CTTGGCAAAA AAGCACAAGC GAACCCCAGC CCTGATTGCC CTGCGCTACC 120
AGCTACAGCG TGGGGTTGTG GTCCTGGCCA AGAGCTACAA TNAGCAGCGC ATCAGACAGA 180
ACGTGCAGGT GTTTGAATTC CAGTTGACTT CAGAGGAGAT GAAAGCCATA GATGGCCTAA 240
ACAGAAATGT GCGATATTTG ACCCTTGNTA TTTTTGCTGG NCCCCCTAAT TATCCATTTT 300
CTGATGAATA TTAACATGGA GGGCATTGCA TGNGGGTCTA CCAGAAGNNC NTNGGGTNTT 360
GGANTGGTNG CANAAGNGGG TNGGTTCTTA TGGCTGGGGG NACTTGGGCA AN 412

SEQ ID NO:1970
SEQUENCE LENGTH:406
SEQUENCE TYPE:nucleic acid
TOPOLOGY:linear
CLONE:HUMGS02245
SEQUENCE DESCRIPTION:
GATCCAAAGT TCATACATTT TATGTACAAG ATACATTAGA CTTACAATAN AGTCTAATTA 60
TTAGACTTTT ATGTCAGTGG ATTGTGCTTG CAGAAATACA GAAGTAACAT ACTGACCCTA 120
GGTGAGGAGT TTCTTACATG GTTAACTGGA TTGACCCAAT TGGAGTAAAA GATTTTTGCT 180
GTTACCTCGA TGCTCCTGCA GTTGTTACCT CAGCATGCCA CTGTAGTCCT TGACTCTCAC 240
ATTAAAACTA ACTTTGGCTA GGTACTGGTG GCTCACACCT GTANGTAATC CCAGTACTTT 300
AGGAGGCTGA GACTGGAGGA TTACTTGAGT CCAGGAGTTG GAGACCAGCC TGGCCAATAT 360
AGACTGTCCC TACAAAGGAA AATAAANTTT GTTTCATCTT TCNAAA 406

SEQ ID NO:1971
SEQUENCE LENGTH:401
SEQUENCE TYPE:nucleic acid
TOPOLOGY:linear
CLONE:HUMGS02246
SEQUENCE DESCRIPTION:
GATCAAATAT GGCATCAACA ATATCCGGGA GCTGGTGGGC CACAAGGTGA ACCTGCAGAT 60
GGTGTATGAC AGTCCCCTGT NCCGCCTGGA TGCCGAGCCG AGGCCCCCTC CCACACAGGA 120

GGCTGCGTGA CATGGGCCAC TCTAGGACAG GTCATCCTCC CCGAGTCCCT GCTGCTGCGC 180
TCCTTTGCAT CCCTGGCCAG TNACCTTGTA TTTATGAGGC CTCTGTNAGG CAGCCCCCAC 240
CTTCCTCTTT CCCACCTGTC CCAGGACCAG AATCCCAGGG ACAGAGGACT GGGTAGCAGG 300
TTCCTTCTGT TGTCCTGTGT GGTGTGTCTA CTGTGAGGGT GGGCCCTGAG GAGACCTGTG 360
GGCCANCTAT TGTCTAATAA AGTGGGGCAG TTGCCCCCAA A 401

SEQ ID NO:1972
SEQUENCE LENGTH:384
SEQUENCE TYPE:nucleic acid
TOPOLOGY:linear
CLONE:HUMGS02247
SEQUENCE DESCRIPTION:
GATCTTTNAT GTTGTTNATT TTGGAGAGAA GCACAAGGAG TATTCTCTTT CTAGGGAAAG 60
TTGTGAACCC AACGGAAGCG TAGTTGGGAA AAAGGCCATT GGCTAATTNC ACGTGTGTAT 120
TGCAATGGGA AATAAATAAA TAATATAGCC TGGTGTGATT GATGTGAGCT TGGNCTTGCA 180
TTCCCTTATG ATGGGATGAA GATTGACCCC TGGCTGACCT TTNTTGGCTG TGGAAGAGGN 240
CAATCCTATG GCAGAGCATT CAGAATGTCA ATGCGTAATT CATTATTATC CAAAGCATAG 300
GAAGGCTCTA TGTNTGTATA TNNCCCTNTG TCGGAATACC CCCTTANCTC ATTTTCCNCT 360
TAATAAATTT CACTGGGTTG NAAA 384

SEQ ID NO:1973
SEQUENCE LENGTH:383
SEQUENCE TYPE:nucleic acid
TOPOLOGY:linear
CLONE:HUMGS02248
SEQUENCE DESCRIPTION:
GATCCTGCTT CTCCCCTGGG GTCTTCTTGG TTCCCTGAAG CCCCTCTTCC GGAGTCCCTG 60
CCCTCTATCG GCTCTGCCTC CTGCTTCTTT TCTGTATTGC CATGATTTTT GCTAAGGATA 120
AAGGACAACT TCATTGGCTT CTTTGGGAAA CTTGCAATCT CCCCAAACAG GCCCCTCTGA 180
GATTTCTCCT TTTGCCACCT TCGATATTTT CTCCAGTTTC CTTGATTCCT TTGATACGTC 240
CCCATTCAGG CCACTCTGGC CTTCCATCCA ACCTGCCAGA ACTTTTTCCA CTTCAGCACT 300
TCAGTCACTT AAACATTAGG GCTCCCTGCC CTAAAGGGAC TCAAAATCCT GCAAAANCAA 360
CCACNNAAGG ATAAAAAAAT AAA 383

SEQ ID NO:1974
SEQUENCE LENGTH:375
SEQUENCE TYPE:nucleic acid
TOPOLOGY:linear
CLONE:HUMGS02249
SEQUENCE DESCRIPTION:
GATCAACAGG AAGAACATGG TGGTCTCCTT TGGAGATGGG GTTACCTTCG TGGTCGTCCT 60
ACACCAGGTG TGGAAGAAAC ATCCTGTCCA CCGTGACTTT CTAGGCTTCT ACGTGGTGGA 120
CAGTCACCGG ATGTCAGCAC AGACGCATGG GCTGNTGGGG CAATTCTTCC AACCCTTTGA 180
CTTTAAAGTG TCTGACATCN GGCCAGGCTC TNACCCCACA AAGCCAGATG CCACATTGGT 240
GGTGAAGAAC CATCANGNTG ATTNTNACCA GGGGCTCCCN GAAAGANTAC AGNAAAGGNA 300

TGCCANGCAT CGGCACGAAN GGTTGTCTTC TTGGTTCGNN CACANCAACG GGGAATGTTG 360
TTGNTTGANT GGTGN 375

SEQ ID NO:1975
SEQUENCE LENGTH:370
SEQUENCE TYPE:nucleic acid
TOPOLOGY:linear
CLONE:HUMGS02250
SEQUENCE DESCRIPTION:
GATCTATGTG CACACACCCN CATCACATGG AATGGGCTGG TCTAGGCTGT GGCCTGNCAC 60
CTTCCTTAGA GTGAATAGGG CGGACACTCC TCTCTTTTCC TGTAGCGTGT GGACCGGTCC 120
ACGCAATTCT TTATCCTGTG AACTCATCTG AGTGGGAGGT GGTGGACACT GGGGTTTCCT 180
TCCCTCTCTC CGTAGCATCC GTTGGTCTTT CTCTCCATCT CTGTTGGTTT GTCTGTCTCT 240
GTCTTCCTCC CAATCCCTAG GGGAAGGGGG CATTTGGCTA GGGGGTGCCC CTGTGAGCCT 300
NGACCTTGCC CCCTCGTCCN TCTCCCCAGT GTTTCCAGGA CCCCCAATAA ACCTTGTCCT 360
GTCAGTCAAA 370

SEQ ID NO:1976
SEQUENCE LENGTH:367
SEQUENCE TYPE:nucleic acid
TOPOLOGY:linear
CLONE:HUMGS02252
SEQUENCE DESCRIPTION:
GATCGATACA GGGGAGGGGG GAGGTGGAAA CAAACAACAA AACTGTACAT TTTTTTTAAA 60
GTTTGTTGAA AAGAATATTG TCTTATTCTA TAAAACATTT CAAACCTAGT TAGAGATTTG 120
TAATCAAAAA ACATTTGCGC AGAAAGCAGC ACTTAGGGCT GCCTGTTCTA TACCCTACAG 180
TCAGACAGGA AAAGANCTGA AAATGGCACC CTTCTGACAT TCTGNGGCAG CTGGNCTNGC 240
AGCCANGTAA NGGNGAGTTT ATGAGGTGGT GTGGGGAGGG TGGGGGAGGC NGCGTGGGGG 300
NTTCTCTCCA CAGCAGGTGG GNGCAGGCAA GNNTTTGGNG GCCAGANGGN GNGCTTTGTN 360
CNTGGGN 367

SEQ ID NO:1977
SEQUENCE LENGTH:353
SEQUENCE TYPE:nucleic acid
TOPOLOGY:linear
CLONE:HUMGS02254
SEQUENCE DESCRIPTION:
GATCTCCAGC CTTACCGCGG CTCGAAATGG ACCCCAACTG CTCCTGCACC ACTGGTGTCT 60
CCTGCGCCTG CACCGGCTCC TGCAAGTGCA AAGAGTGCAA ATGCACCTCC TGCAAGAAGA 120
GCTGCTGCTC CTGCTGCCCC GTGGGCTGTG CCAAGTGTGC CCACGGCTGT GTCTGCAAAG 180
GGACGTTGGA GAACTGCAGC TGCTGTGCCT GATGTGGGAA CAGCTCTTCT CCCAGATGTT 240
AATAGAACAA GCTGCACAAC CTGGATTTTT TTTTCAATAC GATACTGAGC CATTTGNNGC 300
ATTTCTTTTT ATGTTAAATA TGTGAGTGAC AATAAAACAA TTTTGACTTG AAA 353

SEQ ID NO:1978

SEQUENCE LENGTH:351
SEQUENCE TYPE:nucleic acid
TOPOLOGY:linear
CLONE:HUMGS02255
SEQUENCE DESCRIPTION:
GATCTNACCT GAGGTGCTGG GTGAGAAATC TTAGGGACCA GATGGAAGAG GGCCTGTNTA 60
TANNGACATT AGGAAGGAGG TTTGGGAAGG TTTGGGAGGG CAGCCTGACT TGTCTCCAGA 120
GAAAGTTAGA ACTGGTTGAC TTAGCAACCA AGGGNCACTG GGTCAAAGAC GAGGANTCAG 180
GACGTCGGTG CCTAGTGACT GAGCGCCANC CAGCCCATTT CTNTACATTG CCATGGGCTA 240
AACCCAGTGT GCTGTAAACT CTTGCAGTTC CTTGNGGTTA TGGGATTTTG NTATCTTGNC 300
NGTGTTTCCT CAANTAAAGG NNCTTGTTTG TAAAAATTGG GNAANAAGAA A 351

SEQ ID NO:1979
SEQUENCE LENGTH:342
SEQUENCE TYPE:nucleic acid
TOPOLOGY:linear
CLONE:HUMGS02256
SEQUENCE DESCRIPTION:
GATCTGTTTC CTACAGACTT CAAATTTGAC ACCCCAGTGG ACAAACTACC ACAGCTATAA 60
ATTGAGGCAG CTAACGTCAA ATTCTTGAAT ACAAAACTTT GCCTGTTGTA CATAGCCTAT 120
ACAAAATGCT GGGTTGAGCC TTTCATGAGG AAAAACAAAA GACATGGTAC GCATTCCAGG 180
GCTGAATACT ATTGCTTGGC ATNNNGTATG TATATACTAG TGAAACATAT TTAATGATTT 240
AAATTTCTTA TCAAATTTCT TTTGTAGCAA TCTAGGAAAC TGTATTTTGG AAGATATTTG 300
AAATTATGTA ATTCTTGAAT AAAACATTTT TCAAAACTCA AA 342

SEQ ID NO:1980
SEQUENCE LENGTH:340
SEQUENCE TYPE:nucleic acid
TOPOLOGY:linear
CLONE:HUMGS02257
SEQUENCE DESCRIPTION:
GATCAGCATG ACCAGCACTC GTTGGNGGAA AGGTGTCTNT NAGGAGACGT CTGGAGCTTA 60
TGAGAAAACA GATACTGATG GGAAGTTTCT CTATCACAAA TCCAAATGGA ACATAACCAT 120
GGCGTCCTAT GTGGTCCACA CCAACTATGA TGAGTATGCC ATTTTCCTGA CCAAGAAATT 180
CAGCCGCCAT CATNGACCCA CCATTACTGC GGTGTCCCTG GTGATGGNGA TGAGGAGCTN 240
CTGCGCTTCT CCAACTGACA ACTGGCCGNT CTGCANGTCA GAGGATGGNC AGTGTCTGTC 300
CNGGGGTACT GTGGCAGNCA GCGACAAGCA ACCTGNGTCN 340

SEQ ID NO:1981
SEQUENCE LENGTH:339
SEQUENCE TYPE:nucleic acid
TOPOLOGY:linear
CLONE:HUMGS02258
SEQUENCE DESCRIPTION:
GATCNTNTCG NCTCTGAAGA AGACATGGTG ACTGTGGTGG AGGACTGGAT GANNTTCTAC 60

ATCAACTATT ACAGGCAGCA GGTGACAGGG GAGCCCCAAG AGCGAGACAA GGCTCTGCAG 120
GAGCTTCGGC AAGAGCTGAA CACTCTGGCC AACCCTTTCC TGGCCAAGTA CAGGGACTTC 180
CTGAAGTCTC ATGAGCTCCC GAGTCACCCA CCGCCCTCCT CCTAGCTCAG GGACCCAGCC 240
CCTCCTCTCT GAGAAACTCT GACCTTCATG TCCTTAGGCT GTGCTCCTGC CACTCTACCC 300
TGACACCTCA ATAAAGACCA GTGCTGGTTT TGTTGGAAA 339

SEQ ID NO:1982
SEQUENCE LENGTH:334
SEQUENCE TYPE:nucleic acid
TOPOLOGY:linear
CLONE:HUMGS02259
SEQUENCE DESCRIPTION:
GATCCTTNCG TGCTGAATAT CTGAAAAGAG AAATTTTTCC TACAAAATCT CTTGGGTCAA 60
GAAAGTTCTA GAATTTNAAT TGATAAACAT GGTGGGTTGG CTNAGGGNAA GAGTATATGA 120
GGAACCTTTT AAACGACAAC AATACTGCTA GCTTTCAGGA TGATTTTTAA AAAATAGATT 180
CAAATGTGTT ATCCTCTCTC TGAAACGCTT CCTATAACTC GAGTTTATAG GGGAAGAAAA 240
AGCTATTGTT TACAATTATA TCACCATTAA GGCAACTGCT ACACCCTGCT TTGTATTCTG 300
GGCTAAGATN CATTAAAAAC TAGCTGCNCT TAAA 334

SEQ ID NO:1983
SEQUENCE LENGTH:332
SEQUENCE TYPE:nucleic acid
TOPOLOGY:linear
CLONE:HUMGS02260
SEQUENCE DESCRIPTION:
GATCTTGTGC CAAGCTNAGG GTGTAGCGCT GCAAACGATG AAGCAAGAGT TTCTCATTAA 60
CCTTGTGAAG CAAAAGCCAC AAATAACANA GGAACAACTT GAGGCTGTCA TTGCANATTT 120
CTCAGGCTNT TGGAGAAATG CTGCCAAGGC AGGACAGGAG TCTGCTTTGT GAGAGGGCAA 180
AAACTGATTT CAAAAACTCG CCTGCTTTGG GNTTTAAATT ACTTCAGGGN AGANAGCAAA 240
CAGNNCTTTC ATTCGGTGTN ACTTTTCTCT NNAATTTTAA CTNNTTTAAC ACTTTTTGTC 300
GATTAATGNA ATGATAAACG CTTTTATCGC NN 332

SEQ ID NO:1984
SEQUENCE LENGTH:331
SEQUENCE TYPE:nucleic acid
TOPOLOGY:linear
CLONE:HUMGS02261
SEQUENCE DESCRIPTION:
GATCCTGTGT GTGTGTACTA CCATAGAATG GGCCCCAAGA TGCATGATNA AGTGAGGAAA 60
AAAGCCCATG GTATCCTAAT GTTTGGAATG GAGAAAGTGT GTAGATGTAT GTGTGTGCAT 120
ATGTTTTCAC ACAGACGTAT ACACGTGTAT ATCATATGTC CACCTGCATA TGCCTAGGAT 180
GTCTCTGGGA GGATATCTAG CAGCCCGGCA GCCTGAATTG CCTCTGGGAA GAGGGGTGGG 240
AATGACTTTT CAATGTACAC CTTTTGTACC TTTTGAATTT TCGTACCATG TACATGTATT 300
ATCTATTAAA AAATAGTTCT TAATTTTTAA A 331

SEQ ID NO:1985
SEQUENCE LENGTH:326
SEQUENCE TYPE:nucleic acid
TOPOLOGY:linear
CLONE:HUMGS02262
SEQUENCE DESCRIPTION:
GATCACCCGG CAAGTTCAGG CTGAATTCTG AAGGCAAACT TGAGCAGACG GTCTCCATGG 60
CAACCCGAGC TTCAGAGGCC GAGGATGCCT CCCTTCTCAG CTTCATGCAG GGCTACATGA 120
AGCACGCCAC CAAGACCGCC AAGGATGCAC TGAGCAGCGT GCAGGAGTCC CAGGTGGCCC 180
AGCAGGCCAG GGGCTGGGTG ACCGATGGCT TCAGTTCCCT GAAAGACTAC TGGAGCACCG 240
TTAAGGACAA GTTCTCTGAG TTCTGGGATT TGGACCCTGA GGTCAGACCA ACTTCAGCCG 300
TGGCTGCCTG AGGACCTCAA TACCCN 326

SEQ ID NO:1986
SEQUENCE LENGTH:321
SEQUENCE TYPE:nucleic acid
TOPOLOGY:linear
CLONE:HUMGS02263
SEQUENCE DESCRIPTION:
GATCCTTACA TCTGCATCTA GTTATCAGTA ATTTAGATAT TGAGCTATTT TGATTTATAT 60
TTAAGAAATT AATACATTAG CACTGAAAGT TAAATAGTGT GTTTAAGGTA GTTAATTTCA 120
GGTTGAATGG GTTTTTTTTA ATGAAGTGTA AATAATACCA ATGTATAAGN GTATATTATN 180
ATATTAAATA TTATAGTAAA AAGGAATGTG TGGTATTTNC TNCAGCAAAA CTATTTTTGT 240
GATNTTTTNA TTCTCANCTT TNTATTTAAA AAATGTTACA TCTGCAGAAA AGTTGAAAGT 300
ATAATAAACT CTTCAGTTAA A 321

SEQ ID NO:1987
SEQUENCE LENGTH:321
SEQUENCE TYPE:nucleic acid
TOPOLOGY:linear
CLONE:HUMGS02264
SEQUENCE DESCRIPTION:
GATCTGCGCT GTGGCTGTCC CTGGACGTGC TGCAGCCCTC CTGTCCCTTC CCCCCAGTCA 60
GTATTACCCT GTGAAGCCCC TTCCCTCCTT TATTATTCAG GAGGGCTGGG GGGGCTCCCT 120
GGTTCTNAGC ATCATCCTTT CCCCTCCCCT CTCTTCCTCC CCTCTGCACT TTGTTTACTT 180
GTTTTGCACA GACGTGGGCC TGGGCCTTCT CAGCAGCCGC CTTCTAGTTG GGGGCTAGTC 240
GCCGAATTCT ACAATCCCGC TGGGGCGGCC GGGGCGGGAG AGAAAGGTGG TGCTGCAGTG 300
GTGGCCCTGG GGGGCCATTC N 321

SEQ ID NO:1988
SEQUENCE LENGTH:315
SEQUENCE TYPE:nucleic acid
TOPOLOGY:linear
CLONE:HUMGS02266
SEQUENCE DESCRIPTION:

GATCAAAAAT TTCTATCTGG AGAACCTCTG CGGACAAACC GAGTGCTTCC TGCAATTCCA 60
AGTCAACGAA GACACAGCAC AGCAGCAGAA GAGAGTGAAC ATTCTGCCAA CCCCACCAGT 120
GATGAAAATT AACTGTGGGC CACTCGCTGC AGAAATGTAG ATGAATATGT ATTTTCAACT 180
CTCAAAGGAC AAGATTACTC CAGTTTGTAA GAACGAAGAC CAATTTAGTA AGCTGCATTC 240
TATAAGCCAT CAGTTTTATA ACTCGAAATT CTTTATTCCA AATAAAGATA CTCCCTAAAT 300
AAGCACTTAG ANAAA 315

SEQ ID NO:1989
SEQUENCE LENGTH:312
SEQUENCE TYPE:nucleic acid
TOPOLOGY:linear
CLONE:HUMGS02268
SEQUENCE DESCRIPTION:
GATCAAAGCT GGGGCTGGAG GGTGGAGGTG TCAAGCCAGG CCAGGCAAAG GGAGATTTGA 60
AGCCAGAGCA GGTGGGCGCA TCTCCCAGGA AACCCTGCAT CATTCTTTCC TGGTGATTCT 120
TTCCTTCTTT GTTATGGTTC ACATGAGTCT ATGGAGTGCT CATTCTACAT TCTCCGTTCT 180
TATCTTCATC CTATGCAACT TTAGACCGCT ACTGTTTTTT AAAAGTGATT TGATTTGNGG 240
CTGTAACCTT TTTTTATTGT TGTTAGCTTT GACTTTGAAA TGTGTAATAA AGATTCAAGT 300
GAGCATTAGA AA 312

SEQ ID NO:1990
SEQUENCE LENGTH:310
SEQUENCE TYPE:nucleic acid
TOPOLOGY:linear
CLONE:HUMGS02269
SEQUENCE DESCRIPTION:
GATCTTGGGG TCCCCCGACG ACGTGCTCGA GTTCCTGAAG GTGTATGAGA AGCACTCNGC 60
CCAGTGAGCA CCTGCCCTGC CTGCATCTGG AGAATTGCCT CTACCTGGAC CTTTTGTCTC 120
ACACAGCAGT ACCCTGACCT GCTGTGCACC TTACATTCCT AGAGAGCAGA AATAAAAAGC 180
ATGACTATTT CCACCATCAA ATGCTGTAGA ATGCTTGGCA CTCCCTAACC AAATGCTGTC 240
TCCATAATGC CACTGGTGTT AAGATATATT TTGAGTGGAT GGAGGAGAAA TAAACTTATT 300
CCTCCTTAAA 310

SEQ ID NO:1991
SEQUENCE LENGTH:305
SEQUENCE TYPE:nucleic acid
TOPOLOGY:linear
CLONE:HUMGS02270
SEQUENCE DESCRIPTION:
GATCCGAGTG AATGCAGTAA ACCCCACAGT GGTGATGACG TCCATGGGCC AGGCCACCTG 60
GAGTGACCCC CACAAGGCCA AGACTATGCT GAACCGAATC CCACTTGGCA AGTTTGCTGA 120
GGTAGAGCAC GTGGTGAACG CCATCCTCTT TCTGCTGAGT GACCGAAGTG GCATGACCAC 180
GGGTTCCACT TTGCCGNTGG AAGGGGGCTT CTGGGCCTGC TGAGCTCCCT CCACACACCT 240
NAAGCCCCAT GGGCGTGCTC ATCCTACCCC CAATCCNTCC AATAAACCTG ATTCTGCTGG 300
CCAAA 305

SEQ ID NO:1992
SEQUENCE LENGTH:301
SEQUENCE TYPE:nucleic acid
TOPOLOGY:linear
CLONE:HUMGS02271
SEQUENCE DESCRIPTION:
GATCGAGACA CGTGATGGGA AGCTGGTGTC TGAGTCCTCT GACGTCCTGC CCAAGTGAAC 60
AGCTGCGGCA GCCCTCCCAG CCTACCCCTC CTGCGCTGCC CCAGNNNCTG GGAAGGAGGC 120
CGCTATGCAG GGTAGCACTG GGAACAGGAG ACCCACCTGA GGCTCAGCCC TAGCCCTCAG 180
CCCACCTGGG GAGTTTACTA CCTGGGGACC CCCCTTGCCC ATGCNTCCAG CTACAAAACA 240
ATTCAATTGC TTTTTTTTTT TGGTCCAAAN TAAAACNTCA GTTAGCTNTG CCAATTTNAA 300
A 301

SEQ ID NO:1993
SEQUENCE LENGTH:311
SEQUENCE TYPE:nucleic acid
TOPOLOGY:linear
CLONE:HUMGS02272

SEQUENCE DESCRIPTION:
GATCTGAACT CCAGCTGCCC TACAAACTCC GTCACAGCTT TTNTTCTCAC TTCATGTGAA 60
AACTACCCCA GTGGCTGACT GAATTGCTGA CCCTTCAAGC TCTGTCCTTA TCCATTACCT 120
TAAAGCAGTC ATTCCTTAGT AAAGTTTCCA ACAAATAGAA ATTAATGAAT AAATGTTGAC 180
ACTTTGATAG CACTGATATG GAGATTATCC TTTCATTGAG CCTTTTATCC TCTGGTCTTC 240
TTTGAAGAAC CCCTCACTGT CACCTTCCTG AGAATACTCT AAGACCAATA AATACTTCAG 300
TATTTCAGAA A 311

SEQ ID NO:1994
SEQUENCE LENGTH:297
SEQUENCE TYPE:nucleic acid
TOPOLOGY:linear
CLONE:HUMGS02274
SEQUENCE DESCRIPTION:
GATCTAAAAC TNAAGAACAA ATTCTATTTA TTTATTATTG GAAAATNAAA AGCAACTCAA 60
AACAACTTCA ACCTGGAGGT GCATTTATAA TTCATTCTGC ATTTATTCTG TAAAAAGGTG 120
ACTGTTTTAT AAATNCTTTT AATTTATGTT CAATATATAT AAAAAGTGCA TCTNTTTTNT 180
TTTTCCCTTT TTTCTCCATA ATTTTAAGAA ATGAATNNGN TTGTTGTCAA CACATTTGTG 240
AAGTCTTGTG CTATAAAGGG GAACTTCCCC TAATAAAAGG GCCTTGGAAA CCTCAAA 297

SEQ ID NO:1995
SEQUENCE LENGTH:293
SEQUENCE TYPE:nucleic acid
TOPOLOGY:linear
CLONE:HUMGS02275
SEQUENCE DESCRIPTION:
GATCTAGGAG AGGCGGCTCT GAACGATTAC CTGCGGGTCA AGACAGTNAC CTTCGAATAC 60
TGAAGAAAGG TCTTTTTAAG AAGAAAGTCC CTGCCCCTCC CTCGTGGCTG GGGCCCCCTC 120
CCTNTTAAGC CTGGNTGCAC AGCACCTCCC ACCTGGGGGG CTAGTGGAAG CCCTCCTGCN 180
TGCACACCAT GTTTGCNTCT TGGACGCCCT CTTTCCAGTN ANAAGCAGCC CTTGGNTGGN 240
TNAGGTGTGC CCCTCCCAGG NAGAATAAAG CTTCTAAAGA NAGACCGTCC AAA 293

SEQ ID NO:1996
SEQUENCE LENGTH:290
SEQUENCE TYPE:nucleic acid
TOPOLOGY:linear
CLONE:HUMGS02276
SEQUENCE DESCRIPTION:
GATCAGGAGG AAGTGAGCAG TCGCCTGCCT GCAGCAGGNA GCTTTCTACT CCTGCCTCAT 60
GCGTACGTCC CACAAATGCA GGTGTCCTGA GCACCACACC CAGTGGGAAG AGTGTGGGGG 120
AGGCGCACAG TGTGAGCCCG CCCCCACGTC GTGGGTTAAC ATCTGTTATC AAACTGCTGT 180
CGTTGTTGTG GGTTTGGTCC TTGAACAAGA TGTGGGCCTT GCAAGATGGG AGAGTAAACC 240
TTGAAGGGCT TTATTAAAGA AATAAAAAAG NACTTNTTGT ATCTTTTAAA 290

SEQ ID NO:1997

SEQUENCE LENGTH:289
SEQUENCE TYPE:nucleic acid
TOPOLOGY:linear
CLONE:HUMGS02277
SEQUENCE DESCRIPTION:
GATCCTGAAG AGGATGGTGT GGGAATGCAC TTTGAGAAAT GNCTATCTAA CCTTTNGAAA 60
CTTAAAGTGT TCCCAGTCCC CATTATACTT TTTAAAACCA CAATAGTCAC ATAAGGAAAT 120
AATCTAGCAG CTCATCCCTT TGCATCCAAT GTTTGAACTA TTTTTAGAAA GTGCTCTNGT 180
TCTGTCAAAG AACCTTCAAC ACAGAAAAAT ACANAAATGA GGGAAATGTA TAATTCCTGC 240
AGGAGAGAAG TGTTGTTAAC ATGTCAATAN ATGTTCTTAG ANAGTTAAA 289

SEQ ID NO:1998
SEQUENCE LENGTH:279
SEQUENCE TYPE:nucleic acid
TOPOLOGY:linear
CLONE:HUMGS02278
SEQUENCE DESCRIPTION:
GATCAGAGAA GCTAGGAGAG CTCCAGCAGG GGCACAGAGG ATTGGNNGCA GGAGGAGTCT 60
GGAACACAGC CTTCATGCCC CCTGACCCCA GGCCGACCCT CCCCACACCC TAGGGTACCC 120
CAGTCGTATC CTCTGTCCGC ATGTGTGGCC AGGTCTGACA AACACCTGTA GATGACTGNT 180
GGCCNAACCT GGGNCCTGNN CAGGAGGTTG GAGCAGNAAG GGCTCTCCCT AGGGGTGGTG 240
TNTCTCCTCT AGGGTATTGG GNTGCATGTN NTGCACTNN 279

SEQ ID NO:1999
SEQUENCE LENGTH:274
SEQUENCE TYPE:nucleic acid
TOPOLOGY:linear
CLONE:HUMGS02279
SEQUENCE DESCRIPTION:
GATCATGAAG GAACACATAG CACCAAGAGA GGCCATGCTA AATCTCGCCC TGTCAGAGGT 60
ATCCACACTT CTCCTTTGGG GAAGNCTTCC CTGTCCCCCT TGCATTTCCT TCTTAACTCT 120
CTGTTACACG TCATTGAAAC TACACTTTTT TGGTCTNTTT TTNTGCTAGA CTGTAAGTTC 180
CTTGGGGNCA GGGCCTTTGN CTGNCTCATC TCTGTATTCC CAAATGCCTA ACAGTACAGA 240
GCCATGACTC AATAAATACA TGNTAAATGG NAAA 274

SEQ ID NO:2000
SEQUENCE LENGTH:280
SEQUENCE TYPE:nucleic acid
TOPOLOGY:linear
CLONE:HUMGS02280
SEQUENCE DESCRIPTION:
GATCTTAACA AAATTCGTAG CAGTGGAACC TTGAAATGCA TGTGGCTAGA TTTATGCTAA 60
AATGATTCTC AGTTAGCATT TTAGTAACAC TTCAAAGGTT TTTTTTTGTT TGTTTTCTAG 120
ACTTAATAAA AGCTTAGGAT TAATTAGAAG AAGCAATCTA GTTAAATTTC CCATTTGTAT 180
TTNATTTNCT TGAATACTTT TTNCATAGTT ATTTGTTTAA AANGATTTAA AAATCATTGC 240

ACTTTGGTCA GAAAAATAAT AAATATATCT NATAAACAAA 280

SEQ ID NO:2001
SEQUENCE LENGTH:271
SEQUENCE TYPE:nucleic acid
TOPOLOGY:linear
CLONE:HUMGS02281
SEQUENCE DESCRIPTION:
GATCTGCATG TTCTCTCTTT GGGTTCAAGC ATTTCCTTAC AGAAGAGCCA CCGTGGAAGT 60
CATGGGTAAA TATGTGTTGA ATTGGTAACT CCCTCTTGGA GAATTTCTTG TGAATTACAC 120
AGCAATAGGG GAACTCATTT AACTGGAGAC ATAATCTCAA TTTGTAAAGT GTGGCCCATT 180
TTCTAACATT TTNANNTTGC ATACCCTCCC CTCTCTTCTC GATTGATGAA ACTAACAAAG 240
AGGTTAATAA AAGCCCATCT CGTCATGTAA A 271

SEQ ID NO:2002
SEQUENCE LENGTH:270
SEQUENCE TYPE:nucleic acid
TOPOLOGY:linear
CLONE:HUMGS02282
SEQUENCE DESCRIPTION:
GATCTTACCC GTGACAAAAT GTGTTCCATG GTCAAAAAAT GGCAGACAAT GATTGAAGCT 60
CACGTTGATG TCAAGACTAC CGATGGTTAC TTGCTTCGTC TGTCCTGTGT TGGTTTTACT 120
AAAAAACGCA NCAATCAGAT ACGGAAGACC TCTTATGCTC AGCACCCNCA AGGTNCGNCN 180
AATNCGGGAG AAGATGATGG GNAATCATTG GCCCCGTGAG GGTTCAAGGC AAANTTTGCT 240
TTTGAANGNA AGGGGGGNNA ATAAATTNGN 270

SEQ ID NO:2003
SEQUENCE LENGTH:265
SEQUENCE TYPE:nucleic acid
TOPOLOGY:linear
CLONE:HUMGS02283
SEQUENCE DESCRIPTION:
GATCCCAACA GAAGAACATC GGAGACCAGA GAGAGGAACT CAAAGGGGCG CTGCCTCCGG 60
GTCTGGGGTC CTGGCCTGCG TGGCCTNTTG GCACGTGTTT CTCTTCCCCG CCCGGCCTCC 120
AGTTGTGTGC TCTCACACAG GCTTCCTTCT CGACCGGCAG GGGCTGGCTG GCTTGCAGGC 180
CACGAGNGTN GGCTCTACCC NACACTGGCT TTGCTGTGTA TACGCTTNTT GCCCTTAAAT 240
AAATATGCAC ATTTTATCCA TTAAA 265

SEQ ID NO:2004
SEQUENCE LENGTH:249
SEQUENCE TYPE:nucleic acid
TOPOLOGY:linear
CLONE:HUMGS02285
SEQUENCE DESCRIPTION:
GATCCACATT GTTAGGTGCT GACCTAGACA GAGATGAACT GAGGTCCTTG TTTTGTTTTG 60

TTCATAATAC AAAGGTGCTA ATTAATAGTA TTTCAGATAC TTGAAGAATG TTGATGGTGC 120
TAGAAGAATT TGAGAAGAAA TACTCCTGTA TTGAGTTGTA TCGTGTGGTG TATTTTTTAA 180
AAAATTTGAT TTAGCATTCA TATTTTCCAT CTTATTCCCA ATTAAAAGTA TGCAGATTAT 240
TTGCCCAAA 249

SEQ ID NO:2005
SEQUENCE LENGTH:242
SEQUENCE TYPE:nucleic acid
TOPOLOGY:linear
CLONE:HUMGS02287
SEQUENCE DESCRIPTION:
GATCAGGAGC AAACAGACCC TGCAAGGTCC TCCAGGCCTG GGGACAGGAA AGCCACTGAC 60
CCAGCCCGGG AGGCAGAACC AGGCAGCCTC CNTGGCCCCA GGCAGCCCTT TTCCCTCCAG 120
TGGCACCTCC TGGAAACAGT CCACTTGGGC GCAAAACCCA GTGCCTTCCA AATGAGCTGC 180
AGTCCCCAGG CCATGAGCCT CCCGGGAATG TTTAATAAAG GGCCTGGCCA CCTCTCCTCA 240
AA 242

SEQ ID NO:2006
SEQUENCE LENGTH:239
SEQUENCE TYPE:nucleic acid
TOPOLOGY:linear
CLONE:HUMGS02288
SEQUENCE DESCRIPTION:
GATCTAAGTT TGATAAGAGC CTGTGTGGTG AGTATGGTGT GTATGTGAAG GTGACTTCCA 60
TCCAGGACTG GGTTCAGAAG CCATAGCTGA GACTAATGCA AGGCTGGCGA AGCCTTGCTG 120
AAAGAAGATT TCAGCTGGAG AGGCAAAGTG GCGGGAGTGG CAGAGTGGTG CGTAAGTGTG 180
GTTAAGTNTG GGTCCANCCT GCATTCTGAG CAATCAATAA AGTNTTTCTT TTNCCCAAA 239

SEQ ID NO:2007
SEQUENCE LENGTH:238
SEQUENCE TYPE:nucleic acid
TOPOLOGY:linear
CLONE:HUMGS02289
SEQUENCE DESCRIPTION:
GATCCTGAAG GTAATACAAT CAGCAACATT AAAACATTTC TTTCATTTTC TGTTACTGAA 60
GTAAAATAAG CATTCCACAT TTATAAACAA CAGGTTATTT CAGGAAGATA TTTTCATATC 120
TTACAGCGAA AAGTCTTTTC AAACAGAAGC ACCTAATATT TAATATATTA TTTTGTAAAT 180
TATTGACTAA CAAAATGATG ACTCGACTGG CGAAATAAAA TACAGTTTTT AAATCAAA 238

SEQ ID NO:2008
SEQUENCE LENGTH:236
SEQUENCE TYPE:nucleic acid
TOPOLOGY:linear
CLONE:HUMGS02290
SEQUENCE DESCRIPTION:

GATCCTGAGA NCTTCAAGCT CCTGGNNAAT GTNCTGGTGA CCGTTTTGGC AATCCATTTC 60
GGCAAAGAAT TCACCCCTGA GGTGCAGGCT TCCTGGCAGA AGATGGTGAC TGGAGTGGCC 120
AGTNCCCTGT CCTCCAGATA CCACTGAGCT CACTGCCCAT NATGCAGAGC TTTCAAGGAT 180
AGGCTTTATT CTGCAAGCAA TCAAATAATA AATNTATTCT GCTANGNNAA AACAAA 236

SEQ ID NO:2009
SEQUENCE LENGTH:235
SEQUENCE TYPE:nucleic acid
TOPOLOGY:linear
CLONE:HUMGS02291
SEQUENCE DESCRIPTION:
GATCATTACC ATTACAGGAA CACAGGACCA GATACAGAAT GCACAGTATT TGCTGCAGAA 60
CAGTGTGAAG CAGTATGCAG ATGTTGAAGG ATTCTAATGC AAGATATTTT TCCTTTTTTA 120
TAGTGTGAAG CAGTATTCTG GAAAGTTTTT CTAAGACTAG TGAAGAACTG AAGGAGTCCT 180
GCATCTTTTN NNGTTTAANC TGCTTCTGTT NAAAANGCCA NCATTCCTCT GTTTN 235

SEQ ID NO:2010
SEQUENCE LENGTH:233
SEQUENCE TYPE:nucleic acid
TOPOLOGY:linear
CLONE:HUMGS02292
SEQUENCE DESCRIPTION:
GATCCTGAAG AACTTCAAGC TCCTGGGAAA TATGCTGGTG ACCGTTTTGG CAANNNGTTT 60
CGGCAAAGAN TTCACCCCTG AGGTGCAGGC TTCCTGGCAG AAGATGGTGA CTGGAGTGGC 120
CAGTGCCCTG TCCTCCANAT ACCACTGAGC TCACTGCCCA TAATGCAGAG CTTTCAAGGA 180
TAGGCTTTAT NCTGCAAGCA ATACAAATAA TAANTCTNTC CTCCTAAGAG AAA 233

SEQ ID NO:2011
SEQUENCE LENGTH:229
SEQUENCE TYPE:nucleic acid
TOPOLOGY:linear
CLONE:HUMGS02293
SEQUENCE DESCRIPTION:
GATCCTTGAC GAGGAGAGAG AGCTTGAAAA GCTGTTTCAG CTGGGCCCCG CTTCACCTGT 60
GAAGATGCCC TCTCCACCAT GGGAATCCAA TCTGTTGCAG TCTCCTTCAA GCATTCTGTC 120
GACCCTGGAT GTTGAATTGC CACCTGTTTG CTGTGACATA GATATTTAAA TTTCTTAGTG 180
CTTCAGAGTT TGTGTGTATT TGTATTAATA AAGCATTCTT TAACAGAAA 229

SEQ ID NO:2012
SEQUENCE LENGTH:223
SEQUENCE TYPE:nucleic acid
TOPOLOGY:linear
CLONE:HUMGS02294
SEQUENCE DESCRIPTION:
GATCTGGCAT AAATAAATTN AATAAATATT TTAATGCTCC ATTTTTTAAT GTTGCTTTTC 60

ATACTAAAGA ATGGTGTAGA CTTGTTTTGC AACTNTAAGG TACCCAGTTA TCAATTTAAT 120
CAATGTTTTA GAGNAGGAAA TNATTTTTTN GGTAGAAATT GTTCANGAAC CTTAATTGAA 180
TGCATTAAAT GATGGTGGCA AAATAAACCT ATTAGAAATT AAA 223

SEQ ID NO:2013
SEQUENCE LENGTH:221
SEQUENCE TYPE:nucleic acid
TOPOLOGY:linear
CLONE:HUMGS02295
SEQUENCE DESCRIPTION:
GATCAATGCC CTCATCTGCA ATGTAGGGGC CGGTGGACCT GCTCCAGCAG CTGGTGCTGC 60
ACCANAGGAG GTCCTGCCCC CTCCACTNCT GCTGCTCCAG CTGAGGAGAA GAAAGTGGAA 120
GCAAAGAAAG AAGAATCCGA GGAGTCTGAT GATGACATGG GCTTTGGTCT TTTTGACTAA 180
ACCTCTTTTA TAACATGTTC AATAAAAAGC TGAACTTTAA A 221

SEQ ID NO:2014
SEQUENCE LENGTH:219
SEQUENCE TYPE:nucleic acid
TOPOLOGY:linear
CLONE:HUMGS02296
SEQUENCE DESCRIPTION:
GATCTGTAGT GTCATGGGTG CTGCCGCAGA CGNAAGTTGA NCTGGGGGTG CCTGCAGCCT 60
TCCACTCCTG CCCCGCCTCA CCCCACATGC TCCCTGTTTC TAATGCTTTC TCTAACTTCC 120
TCACCCCTTA ACCAAAAAGG TGTGTTTNCT TTTGTNCATA TAGCCATTCT NAAATATNAG 180
TAATGTAAAC CTNACTTTAT TAAAAAATTA TCCAGCAAA 219

SEQ ID NO:2015
SEQUENCE LENGTH:216
SEQUENCE TYPE:nucleic acid
TOPOLOGY:linear
CLONE:HUMGS02297
SEQUENCE DESCRIPTION:
GATCAGGTTT GTTGAGTTTT TTAGCCTAAT TCCAAAGCAT GGAAGAGTGC TCTAGGTAGG 60
AAAGAAAGCT TTTTCTTACG ATTTGTAGCT ACCTACTGTG CCTGACTTGG TGCCTGTGTG 120
AGGATTAAGC CCTTAGTCTG CTCTTGCAAT TATTCAAATG ACAAATTAAA TTTGCTTTTG 180
TAATAACAAT AAAAGTTGTC ATCTTCCCTT TTGAAA 216

SEQ ID NO:2016
SEQUENCE LENGTH:210
SEQUENCE TYPE:nucleic acid
TOPOLOGY:linear
CLONE:HUMGS02298
SEQUENCE DESCRIPTION:
GATCCTGCCA TCACTGCTGC AAAGAGAACA CGAGACTCAA GGAAACTCCT CCTCCACCGC 60
AGCCCAACTG TGCCTGCTAG TNTCCCCGTG CACACAGGCA GCTCCAGGGG GCTCTGGTTG 120

CCAACAAACA GCATTTGTAA ATGGTCTATT AGCCTTCATT TATACTGCCT AACAATTATT 180
TGAAGGAATA AATTGATGTC AATGGCTAAA 210

SEQ ID NO:2017
SEQUENCE LENGTH:219
SEQUENCE TYPE:nucleic acid
TOPOLOGY:linear
CLONE:HUMGS02299
SEQUENCE DESCRIPTION:
GATCTAGCCT GATTCCTGCG TGTCCGAAAG AACTTAACGT TTTAAAGGTN ATTGTNAAGT 60
AACTGTGTGG GGTTCTAATG CCAGTTTCCT AATTCCATCT CACTGGAGAT GTTTAAAGTT 120
GGCCTCTATC CTAATGACTC AAAACTTGGT TCTTAACTAC CATGATTGCT TTTNAGGGCC 180
CGGAATTATA AATATATATN ATATTTAANT TGTTTGAAA 219

SEQ ID NO:2018
SEQUENCE LENGTH:208
SEQUENCE TYPE:nucleic acid
TOPOLOGY:linear
CLONE:HUMGS02300
SEQUENCE DESCRIPTION:
GATCCGGGGC ACAAAGAGGG TGGGGAACAT GGGGGCTATA CTGGGGAAAG CAGCCATGCT 60
CCCCCCNACC TCCAGCCGAG CATCCTTCAT NAGCCTGCAG AACTGCTTTC CTATGTTTAC 120
CCAGGGGACC TCCTTTCANA TGAACTGGGA AGAGATGAAA TGTTTTTCCA TATTTAAATA 180
AATAAGAACA TTAAAAAGCA ACCCCAAA 208

SEQ ID NO:2019
SEQUENCE LENGTH:202
SEQUENCE TYPE:nucleic acid
TOPOLOGY:linear
CLONE:HUMGS02301
SEQUENCE DESCRIPTION:
GATCTCAAGG CTGCCGTACT GGAGGCCATG ACTGCCTTCC GCAGAGCAGG TGCTGACATC 60
ATCATCACCN ACTACACACC GCAGCTGCTG CAGTGGCTGA AGGAGGAATG ATGGAGACAG 120
TGCCAGGCCC AAGAACTAGA ACTTTAAAAC GTTCCCGGGG CCTCAGACAA GTGAAAACCA 180
AAGTAAATGC TGCTTTTAGA AA 202

SEQ ID NO:2020
SEQUENCE LENGTH:224
SEQUENCE TYPE:nucleic acid
TOPOLOGY:linear
CLONE:HUMGS02302
SEQUENCE DESCRIPTION:
GATCTTCTTT GAGACAAGGC AGGCTGTGGC CATGTAGCCC CATCACACTG TGTTTGTNAT 60
TGTCTGTGTG TCTGTCTCCC CCACCAGACT GTGAGCTCCA TGAGGGCAGG GACCGTGTCT 120
TGTCCGTTCT CTGTATCCCC AGTGCTTGGA ACAGAGCGAG TGCTCACTGT GTATTTAATA 180

AATGGACAAA GAGAGAGGAT GACCCTCACG GGGAGACAGA GAAA 224

SEQ ID NO:2021
SEQUENCE LENGTH:195
SEQUENCE TYPE:nucleic acid
TOPOLOGY:linear
CLONE:HUMGS02303
SEQUENCE DESCRIPTION:
GATCGGCAAA TGCATTGGCC TCCTGGTTAA GCACGTTTTG GGGATAATTT TCTCTTCTTT 60
AGGCAATGAT TAAGTTAGGC AATTTCCAGT ATGTTAAGTA ACACACTTAT TTTTGCCTGT 120
GTATGGAGAG ATTCAAGAAA TAATTTTAAA ACCGCATACA TAATAAAAGA CATTGTTGCA 180
TGGCTTATAG TCAAA 195

SEQ ID NO:2022
SEQUENCE LENGTH:193
SEQUENCE TYPE:nucleic acid
TOPOLOGY:linear
CLONE:HUMGS02304
SEQUENCE DESCRIPTION:
GATCTGTGCC GTGGTCCTGA GGCAGTTGTC TCCACACAAG TACCTCTAGA ACAATCCCCT 60
TTTTTCCATC AAGCTGTAGC CTGCAGAGAA TGGAAACGTG GGAAAGGAAT GGTATGTGGG 120
GGAAATGCAT CCCCTCAGAG GACTGAGGCA TAGTCTCTCA TCTGCTATTG AATAAAGACC 180
TTCTATCTTG AAA 193

SEQ ID NO:2023
SEQUENCE LENGTH:192
SEQUENCE TYPE:nucleic acid
TOPOLOGY:linear
CLONE:HUMGS02305
SEQUENCE DESCRIPTION:
GATCTNCCCT GCATCCTGAC CAAGACCCCA ACCGTGCCCC CAACAGACTC CCCCTGGGAG 60
ATTCAGCCTA TTCACTGATG AGGAAAGTGA AGCCGGGTCT CCTGTCTCCC CAAATTCATT 120
GCACCTACGA TATAGTCCAG GATTGGGTAG CAAGATTAAA CACAAGCGCT TCAAATTGAT 180
GAAAACCACA AA 192

SEQ ID NO:2024
SEQUENCE LENGTH:191
SEQUENCE TYPE:nucleic acid
TOPOLOGY:linear
CLONE:HUMGS02306
SEQUENCE DESCRIPTION:
GATCCCTAAC TACCAACCCC CTGAAGGCAG GTACAATGAC ATCACCAAGG TGTACACACA 60
AGTGGAGTTT AAGAGATAGA CTTGCAGGCT GCTATCCTTA ACATGCTGCC CCTGAGAGTA 120
GGAATGACCA GGGTTCAAGT CTGCTTTCCA CAGAATCAGG CATNCTGTTA ATAAATACTG 180
GTTTAATCAA A 191

SEQ ID NO:2025
SEQUENCE LENGTH:186
SEQUENCE TYPE:nucleic acid
TOPOLOGY:linear
CLONE:HUMGS02307
SEQUENCE DESCRIPTION:
GATCGTATTT CAGTTTTTTG GGTCAATATA TGAACAATGT GTGAACCAGA ACGTATGGTA 60
TTTGACATCA AAGCTATGTC TTGGCATATG TTTATGTTGA TATACTTGAA TTCATTTAAA 120
ATGGAATGTT AATTACATCG ACTTCTTACT ATAAAAAATA AAAAGTTATT GTCCAGTGCA 180
CAGAAA 186

SEQ ID NO:2026
SEQUENCE LENGTH:380
SEQUENCE TYPE:nucleic acid
TOPOLOGY:linear
CLONE:HUMGS02308
SEQUENCE DESCRIPTION:
GATCCATTGA GAAAGAGATG ATGTAATAAA TAATTAAGAT TAGTAATAAT ATTATCAGGG 60
GTGATTATGA CCAGTTGAAT AATCTCTTTC CCTTGAATTA TTTAGCTAAC AAATTAACTC 120
TCCCAAATAT NTAAANTAAT GTAAANTCAT ATTTNACTGC CCATTATTAA CTAAAATATT 180
TNTGTNTGAC TTTGAGCACC AACTGGTAAT ACTAGTAAAT ACCCATGTCA TGCAGATGGC 240
TGGGCGAATA AGAGATGTCT AAAAATATGC ACTGGTCTTG GGAAACATGG CACAAGTAAG 300
GNTATCATAT ATGANGTCTG GTTTATTTTA TGTCTGATTT CTTTTTGANT GAGTAGTTNG 360
GGACTCCANT TCTAAGGNTN 380

SEQ ID NO:2027
SEQUENCE LENGTH:183
SEQUENCE TYPE:nucleic acid
TOPOLOGY:linear
CLONE:HUMGS02309
SEQUENCE DESCRIPTION:
GATCTCTCCA CAGGGCTTGT TTTCCAAAGA AAAGTATTGT TTGGAGGAGC AAAGTTAAAA 60
GCCTACCTAA GCATATCGTA AAGCTGTTCA AAAATAACTC AGACCCAGTC TTGTGGATGG 120
AAATGTAGTG CTCGAGTCAC ATTCTGCTTA AAGTTGTAAC AAATACAGAT GAGTTAAAAG 180
AAA 183

SEQ ID NO:2028
SEQUENCE LENGTH:188
SEQUENCE TYPE:nucleic acid
TOPOLOGY:linear
CLONE:HUMGS02310
SEQUENCE DESCRIPTION:
GATCTGCAGC TGCCTTGGGA CTTGAATCCA TGCAATGTTT AGAGTGTGAA GTCAGTTACT 60
TGTTGATGTT TTCTTACTGT ATCAATGAAA TACATATTGT NATGTCAGTN CTTGCCAGGA 120

ACTTCTCAAC AAAATGGAAT TTNNTTTTNC AGTATTTCAA TAAATATTGA TATGCCCAGC 180
CTGNTAAA 188

SEQ ID NO:2029
SEQUENCE LENGTH:178
SEQUENCE TYPE:nucleic acid
TOPOLOGY:linear
CLONE:HUMGS02311
SEQUENCE DESCRIPTION:
GATCCATGCT GATTTTTATT GCACAAGAAT TAGGTTTGAA CTCTTGAGCT GGAACCTCAG 60
CAAACTAGAG TATATATTGT TCAGTATNNC TTTGGAAACA TTTCATTAAT GTACTTGTCT 120
TACANAAATT TCTGAACTTT AGTAAAAAAA AAATAAAGTT AAACTTTTAA AACTCAAA 178

SEQ ID NO:2030
SEQUENCE LENGTH:176
SEQUENCE TYPE:nucleic acid
TOPOLOGY:linear
CLONE:HUMGS02312
SEQUENCE DESCRIPTION:
GATCGGTATG TTTTGAAAAG AGTAATTTAA CTTTTGGGTG CCAGGAAATG GGTTTTCTCA 60
AAGTCCATTG CCGGCAATGG GCAGGCCTGC AAATACTGGC ACAGAGCATT AATCATACAC 120
CTTATTAACG GTGAGGTGAA TAACTTTGAA ATAAAGTTTT AGAGAAATGT TTCAAA 176

SEQ ID NO:2031
SEQUENCE LENGTH:174
SEQUENCE TYPE:nucleic acid
TOPOLOGY:linear
CLONE:HUMGS02313
SEQUENCE DESCRIPTION:
GATCCGCCTG GCAGCCATTG CAGAGTCAGG GGTAGAGCGG CAAGTACTTT TGGGAGACCA 60
GATACCCAAA TTCGCCGTTG CCACTTTACC ACCCGCCTGA ATCCTGGGAT TCTAGTATGC 120
AATAAGAGAT GCCCTGTACT GATGCAAAAT TTAATAAAGT TTGTCACAGA GAAA 174

SEQ ID NO:2032
SEQUENCE LENGTH:172
SEQUENCE TYPE:nucleic acid
TOPOLOGY:linear
CLONE:HUMGS02314
SEQUENCE DESCRIPTION:
GATCCAGAAG TTATGGTGGC TTTCCAGGAT GTGGCTCAGA ACCCAGCAAA TATGTCAAAA 60
TACCAGAGCA ACCCAAAGGT TATGAATCTC ATCAGTAAAT TNTCAGCCAA ATTTGGAGGT 120
CAAGCGTAAT GTCCTTCTGA TAAATAAAGC CCTTNCTGAA GGAAAAGCAA CN 172

SEQ ID NO:2033
SEQUENCE LENGTH:170

SEQUENCE TYPE:nucleic acid
TOPOLOGY:linear
CLONE:HUMGS02315
SEQUENCE DESCRIPTION:
GATCTCAGGA CCTCTTTACA CTTTTCAAAG TTACTTAAGA CTCTGAAGAG CTTTTCTTTA 60
TGAGGTTATA TCAATATTTA CTACATTAAA AATTAAAACA GAAAATTTAA AGTAGGTATT 120
TATTGATTTA TTTAAACAAT AAAAATAATA AAGTATTACA TGCTAACAAA 170

SEQ ID NO:2034
SEQUENCE LENGTH:169
SEQUENCE TYPE:nucleic acid
TOPOLOGY:linear
CLONE:HUMGS02316
SEQUENCE DESCRIPTION:
GATCCGTTTG CTGTGAACCC TATGTTATTT CCATGTGTCA AGTGGGTCTT GTGTTGCCAG 60
CTTCTATTTG AAGATTGCGT TTGCACTCAG TGTAAGTTTC TGTCAGCAGT AGTTTCACCC 120
ATTTGCATGG AAAAATTTAA AGCTAATAAA GCAATTTAAA AAGCACAAA 169

SEQ ID NO:2035
SEQUENCE LENGTH:159
SEQUENCE TYPE:nucleic acid
TOPOLOGY:linear
CLONE:HUMGS02317
SEQUENCE DESCRIPTION:
GATCCCACAG TCTCAGCCCT GCTTACTAGT GAGAAAGACT GGCAAGGTTT CCTAGAGCTC 60
TACTTACAGA ACAGCCCTGA GNCCTGTAAC TATGGGCTCT GAAGGGGGCA GGAGTCAGCA 120
ATAAAGCTAT GTCTGATATT TTCCTTCACT AATATGAAA 159

SEQ ID NO:2036
SEQUENCE LENGTH:151
SEQUENCE TYPE:nucleic acid
TOPOLOGY:linear
CLONE:HUMGS02318
SEQUENCE DESCRIPTION:
GATCCGGGGN AGCTAGGATG GGGAACCTGC CACAGCCAGA ACTGAGGGGC TGGCCCCAGG 60
CAGCTCCCAG GGGGTAGAAC GGCCCTGTGC TTAAGACACT CCTGCTGCCC CGTCTTGAGG 120
GTGGCGATTA AAGTTGCTTC ACATCNTCAA A 151

SEQ ID NO:2037
SEQUENCE LENGTH:148
SEQUENCE TYPE:nucleic acid
TOPOLOGY:linear
CLONE:HUMGS02319
SEQUENCE DESCRIPTION:
GATCTAACAT CAAACTAACA GCTTTCAGAA AGGAGACTNT TGNCATCTTT TCACCAAGGA 60

GTATATTTGT AGAGTAATAT ATGTGTGGAA NTTTNAAATT ATATCATTTG ACATGATTTA 120
TGAGGCTTAT TTAAGCTGGA GTNNNCCN 148

SEQ ID NO:2038
SEQUENCE LENGTH:143
SEQUENCE TYPE:nucleic acid
TOPOLOGY:linear
CLONE:HUMGS02321
SEQUENCE DESCRIPTION:
GATCTGGTCT AGTTAACCTA GAAGTATTTT TGTCTCTTAG AAATACTTGT GATTTTTATA 60
ATACAAAAGG GTCTTGACTC TAAATGCAGT TTTAAGAATT GTTTTTGAAT TTAAATAAAG 120
TTACTTGAAT TTCAAACATC AAA 143

SEQ ID NO:2039
SEQUENCE LENGTH:142
SEQUENCE TYPE:nucleic acid
TOPOLOGY:linear
CLONE:HUMGS02322
SEQUENCE DESCRIPTION:
GATCTGGAGG AGGTGAAGGC CAAGGGACCT GCTTCTATTA GCCCTTCTCC ATGGCCCTGC 60
CATGCTCTCC AAACCACTTT TTGCAGCTTT CTCTAGTTCA AGTTCACCAG ACTCTATAAA 120
TAAAACCTGA CAGACCATGA AA 142

SEQ ID NO:2040
SEQUENCE LENGTH:156
SEQUENCE TYPE:nucleic acid
TOPOLOGY:linear
CLONE:HUMGS02323
SEQUENCE DESCRIPTION:
GATCGAAAGA AGAACATCAT GAAAAANAAA TNAACTTTGC TTAGTGGATT AGACTCCTTT 60
GCTGAAGTCA GTTATTCATC AAGAATGCAA TTAGACTAAT TGTGAATAAA TGATTGAATG 120
AAGATATAAT AAATAAAAGC TATAATTATA GATAAA 156

SEQ ID NO:2041
SEQUENCE LENGTH:138
SEQUENCE TYPE:nucleic acid
TOPOLOGY:linear
CLONE:HUMGS02324
SEQUENCE DESCRIPTION:
GATCTCTCCT TCAGTCTGCT CTGTTTAATT CTGCTGTCTG CTCTTCTCTA ATGCTGCGTC 60
CCTAATTGTA CACAGTTTAG TGATATCTAG GAGTATAAAG TTGTCGCCCA TCAATAAAAA 120
TCACAAAGTT GGTTTAAA 138

SEQ ID NO:2042
SEQUENCE LENGTH:137

SEQUENCE TYPE:nucleic acid
TOPOLOGY:linear
CLONE:HUMGS02325
SEQUENCE DESCRIPTION:
GATCCACAGG CAGAGCCAAG GGAAGGTGTG ATTCTNTGAG GAAAGAGTGA TTCTGATATA 60
TGTACTTGTC ACATTGGTGT TGGACACATT TGCGCCAAAA GTATGGTAAT NCTATTATNA 120
AATAATTCTC TGAGAAA 137

SEQ ID NO:2043
SEQUENCE LENGTH:134
SEQUENCE TYPE:nucleic acid
TOPOLOGY:linear
CLONE:HUMGS02326
SEQUENCE DESCRIPTION:
GATCCAAACT GGAANCAGCT CAAATNTCCA TCAATAGCAG AAAGATAAAC AAATGTNGGG 60
ATGTNCAGAG CTTGAAATAT GACACANTAT AAAAGGACAA ACTATTAAAA CTCACAACAG 120
CATGGATAAN TCTN 134

SEQ ID NO:2044
SEQUENCE LENGTH:133
SEQUENCE TYPE:nucleic acid
TOPOLOGY:linear
CLONE:HUMGS02327
SEQUENCE DESCRIPTION:
GATCCAAGCA GGCCTTGAAT GCAATNGCAA GTGGTTTATA GTCCCTTGCT CTTACAACTT 60
GCAGGGACAT GTGGTTATTT AGAAATTGTG ACTGAGCGGA CCCAAGAATN TAAATAATAT 120
TCATAAACCT AAA 133

SEQ ID NO:2045
SEQUENCE LENGTH:132
SEQUENCE TYPE:nucleic acid
TOPOLOGY:linear
CLONE:HUMGS02328
SEQUENCE DESCRIPTION:
GATCACCTGT AGGGAAATGA AGTGTNCCCT GGAACAAGGA GGTGGGGGCA NTGTGGCCCC 60
TTCCCCAACT GGGGGTGGAC AGCTGTNTCC TGGGGTGGGT TGGTATTAAA GAGGAAAGCN 120
ATTTTTTGGA AA 132

SEQ ID NO:2046
SEQUENCE LENGTH:130
SEQUENCE TYPE:nucleic acid
TOPOLOGY:linear
CLONE:HUMGS02329
SEQUENCE DESCRIPTION:
GATCCCAGGA ACTGTGGGCA CCCATTTTCT GTGTCTCCCA GCCCATTTCC ACTCCTAGTT 60

TGTCATGGAT AATNTTTGTT GTTCCCTGTG TGATTTTTGC CATCAAAATA AAAATTTGAG 120
ACTCGTTAAA 130

SEQ ID NO:2047
SEQUENCE LENGTH:130
SEQUENCE TYPE:nucleic acid
TOPOLOGY:linear
CLONE:HUMGS02330
SEQUENCE DESCRIPTION:
GATCCAAGGT CAAAAAAACA AATNCATCAA TTCAGCACAC CACCAACTNA NAGGCTAAGC 60
ATCTTACTGC TAATTCATTG ANGCTGCCAT TTGTCAAGTG CCAAATTGAN TTATTGATTT 120
GTCAATANTN 130

SEQ ID NO:2048
SEQUENCE LENGTH:130
SEQUENCE TYPE:nucleic acid
TOPOLOGY:linear
CLONE:HUMGS02331
SEQUENCE DESCRIPTION:
GATCAGATAT ACAAAAATAA AGTTTTTNAT TTCATTAAAT ATTTAATTAA AACTGCTAAG 60
TTTGAAAATT TTTACCAAGA AATANTTTTN ANAAATTTTN CTAACTTGAT AAAGGCTTTT 120
AAGCAATAAA 130

SEQ ID NO:2049
SEQUENCE LENGTH:129
SEQUENCE TYPE:nucleic acid
TOPOLOGY:linear
CLONE:HUMGS02332
SEQUENCE DESCRIPTION:
GATCTATATT CCCTTATTGC ATTTTCCTTA TGTATAATTT TCCAGATGGT GATGTTACTT 60
TTCAGTGTAC TCATATGTCT CATTTTCATC TAAAATTAAA TGGCAGGAAA CAAGGACTGC 120
ATAGAGAAA 129

SEQ ID NO:2050
SEQUENCE LENGTH:129
SEQUENCE TYPE:nucleic acid
TOPOLOGY:linear
CLONE:HUMGS02333
SEQUENCE DESCRIPTION:
GATCAAAAGC TTATTCNTTT TTTTTTTTTT TTCTTTGGTG TAAAGCCAAC ACCCTGTTTA 60
AAANANATAA TTTTTTTTAA TCATTTTGCN TCTTTTTTTT TTGCTTCATT NAATAAAAAN 120
TGGAAAGAN 129

SEQ ID NO:2051
SEQUENCE LENGTH:148

SEQUENCE TYPE:nucleic acid
TOPOLOGY:linear
CLONE:HUMGS02334
SEQUENCE DESCRIPTION:
GATCTGAACA CCACAGCCCC TGTACTTGGG TTGCCTCTTG TCCCTGAACT TCGTTGTACC 60
AGTGCATGGA GAGAAAATTT TGTCCTCTTG TCTTAGAGTT GTGTGTAAAT CAAGGAAGCC 120
ATCATTAAAT TGTTTTATTT CTCTCAAA 148

SEQ ID NO:2052
SEQUENCE LENGTH:132
SEQUENCE TYPE:nucleic acid
TOPOLOGY:linear
CLONE:HUMGS02336
SEQUENCE DESCRIPTION:
GATCCANCTN TNTCAACCTC CCAAGTAGGA TTACAAGCAT ACGCCGACGA TGCCCAGAAT 60
CCAGAACTTT GTCTATNACT CTCCCCAACA ACCTAGATGT GAAAACAGAA TAAACTTCAC 120
CCAGAAAACA AA 132

SEQ ID NO:2053
SEQUENCE LENGTH:126
SEQUENCE TYPE:nucleic acid
TOPOLOGY:linear
CLONE:HUMGS02337
SEQUENCE DESCRIPTION:
GATCCATCCT TCCTGCCTCC AAGGAGGATA CACAGAGAAT GGCTTCCTGT TGTTTTGTTT 60
ATTTTCTTAA CGTGTACAGA TGGAAACTTC ATTTAAAAAT AAAAACAAAA CAACTCAAAA 120
AGGAAA 126

SEQ ID NO:2054
SEQUENCE LENGTH:126
SEQUENCE TYPE:nucleic acid
TOPOLOGY:linear
CLONE:HUMGS02338
SEQUENCE DESCRIPTION:
GATCAAAAAA GCTCACGTCG TATTTCTTCT TTTCCTTTCT CTTTTCTAGA AATTGGGTGT 60
TTGTACCAGA ATGGAATTTT GCTTCTCGGT TATCCTGTGC TTCAGATGAT TATAATCTAA 120
CCCAAA 126

SEQ ID NO:2055
SEQUENCE LENGTH:125
SEQUENCE TYPE:nucleic acid
TOPOLOGY:linear
CLONE:HUMGS02339
SEQUENCE DESCRIPTION:
GATCAAATCT TACAGTTCAT CACAGAATCC ATGTTGGTGA TAAATNCTAT AAAAGTAATA 60

GGGGTGGTAA GAACATCAGA GANTCCACAC AGGAAAAAAA ATCTATAAAA NGATTCTTTG 120
TGAAA 125

SEQ ID NO:2056
SEQUENCE LENGTH:124
SEQUENCE TYPE:nucleic acid
TOPOLOGY:linear
CLONE:HUMGS02340
SEQUENCE DESCRIPTION:
GATCCCACAG AAATGTTTTG GAGAGCGGGA GGTTTCCCCC AATCTCCTCC AAGTTCTTCT 60
CCCTCCAACC AGAGTTGTGT CTAACTTTAG GCATCTTTTA ATAAATNTNA TTGCGACTCT 120
GAAA 124

SEQ ID NO:2057
SEQUENCE LENGTH:207
SEQUENCE TYPE:nucleic acid
TOPOLOGY:linear
CLONE:HUMGS02341
SEQUENCE DESCRIPTION:
GATCACTTCT GAATAAGCAG TTTGCCTTTA TAAAAACTTG CTGCCTGNCT AAAGATTAAC 60
AGGTTATAGT TTAAATNTGT AATTAATGCT ACCATCTTGC AATAAAGTGA CAATTGAATG 120
NAACAGGGTT TTTCAAGTTG TATAATTCTC TGAAATACTC AGCTTTTGTC ATATGGGTAA 180
AANTTAAAGA TGTCATTGGN CTACAAA 207

SEQ ID NO:2058
SEQUENCE LENGTH:120
SEQUENCE TYPE:nucleic acid
TOPOLOGY:linear
CLONE:HUMGS02342
SEQUENCE DESCRIPTION:
GATCTTTACT CTTGAGAAAT GAATAAGCTT TCTCTCAGAA ATGCTGTCCC TATACACTAG 60
ACAAAACTGA GCCTGTATAA GGAATAAATG GGAGCGCCGA AAAGCTCCNT AAAAAGCAAA 120

SEQ ID NO:2059
SEQUENCE LENGTH:119
SEQUENCE TYPE:nucleic acid
TOPOLOGY:linear
CLONE:HUMGS02343
SEQUENCE DESCRIPTION:
GATCCAAGGT ATCCNTAACA NTGGAGCAGC CAAGTTTNTT TTCAGGCACA ATGACCCTGA 60
CCACCTAAAG AAACTTNTAG AGAAGTNTAA CCCTAAGATA CCCAAAATTT TGGCCTTTN 119

SEQ ID NO:2060
SEQUENCE LENGTH:127
SEQUENCE TYPE:nucleic acid

TOPOLOGY:linear
CLONE:HUMGS02345
SEQUENCE DESCRIPTION:
GATCCCCTCT TCTCAGACAG CGCCAGGCCG GGGTGGGGCC GGGGTTGGGG CCGAGCCCCA 60
CAGCTGCCCC CCTCCCNTCC CNTTTTGTAT AATTTAATAA AGAAATGGTC GCGCTTCTGT 120
TTTTAAA 127

SEQ ID NO:2061
SEQUENCE LENGTH:117
SEQUENCE TYPE:nucleic acid
TOPOLOGY:linear
CLONE:HUMGS02346
SEQUENCE DESCRIPTION:
GATCGAGGTG GAGAAGATGC GGCGGGCGGA GNCTTATNAC CGNNTAGAGA ACAGCCCACA 60
GNTGGATGGG AGCCCCCCAG GGCTCGAGGG TCTGCTGGGG GGCATTGGGG AGAAAGN 117

SEQ ID NO:2062
SEQUENCE LENGTH:115
SEQUENCE TYPE:nucleic acid
TOPOLOGY:linear
CLONE:HUMGS02347
SEQUENCE DESCRIPTION:
GATCTGCTGA GGACACATGC GCTTTTGTAG AATTTAACAT CTGGTGTTTT TCTGAAAAAA 60
TATATATACA TATATTGCTT TATTTGAAAC AAATNAAAAT ATGCTGCATT TGAAA 115

SEQ ID NO:2063
SEQUENCE LENGTH:114
SEQUENCE TYPE:nucleic acid
TOPOLOGY:linear
CLONE:HUMGS02348
SEQUENCE DESCRIPTION:
GATCTGGAGG AGGTGAAGGC CAAGGTGCTG CCCGCTGGGC CTCCCAACGG GCCCTCCTCC 60
CCTCCTTGCA CCGGCCCTTC CTGGTCTTTG AATAAAGTCT GAGTGGGCGG CAAA 114

SEQ ID NO:2064
SEQUENCE LENGTH:114
SEQUENCE TYPE:nucleic acid
TOPOLOGY:linear
CLONE:HUMGS02349
SEQUENCE DESCRIPTION:
GATCCAAAGT ATCAGGTCTG TTAGTGGCCA TCAGCACTTT AATATTGCCT CGAGGAGGAT 60
TTCTTGGNCN CNNNAAATAA GGTCATTAAG TCTTATGCCA AATTCAGTGC TCCN 114

SEQ ID NO:2065
SEQUENCE LENGTH:113

SEQUENCE TYPE:nucleic acid
TOPOLOGY:linear
CLONE:HUMGS02350
SEQUENCE DESCRIPTION:
GATCCTAGAT AAGCAGGTGA AATTTAGGCT TCAGAATATA TCCGAGAGGT GGGGAGGGTC 60
CCTTGGAAGC TGGTGAAGTC CTGTTCTTAT TATGAATCCA TTCATTCAAG AAA 113

SEQ ID NO:2066
SEQUENCE LENGTH:109
SEQUENCE TYPE:nucleic acid
TOPOLOGY:linear
CLONE:HUMGS02351
SEQUENCE DESCRIPTION:
GATCTACGCT GTGCCTTGGC AGGGCACAAT GACCTTNTAG ANATCCACCT GTTAGAACGC 60
CTAGGGGTCT GAACCGGGCT GGNCTGANNC TATCACCTCT TATGCACAN 109

SEQ ID NO:2067
SEQUENCE LENGTH:108
SEQUENCE TYPE:nucleic acid
TOPOLOGY:linear
CLONE:HUMGS02352
SEQUENCE DESCRIPTION:
GATCCCAAGG CCTAGCCTGT GAAGGTCTCC TATCGGAAGA NTACCCCCAT NNAATGGGAA 60
GTGGAATTCC TGGTCAANTT GGTCTTNATG CTCTGGAAGA CGTAAGAN 108

SEQ ID NO:2068
SEQUENCE LENGTH:108
SEQUENCE TYPE:nucleic acid
TOPOLOGY:linear
CLONE:HUMGS02353
SEQUENCE DESCRIPTION:
GATCTTAAGC AAAAATACTC ACTGAAATAG TATGTGGATG AATTCACCTA CTTACAATTT 60
TATGGTTTCT TTGTAAATAA TAAATGTGAA TCTCAATTCT GCTTTAAA 108

SEQ ID NO:2069
SEQUENCE LENGTH:104
SEQUENCE TYPE:nucleic acid
TOPOLOGY:linear
CLONE:HUMGS02354
SEQUENCE DESCRIPTION:
GATCCTNGGC TCCACCTGGG TGGCCCTGAC CACGGGAGCC TTGGGCCTAG AGCTGCCCTT 60
GTCCTNCCAG GACCCCCNCC TGGCCACTGC CCGCCTACTN NCTN 104

SEQ ID NO:2070
SEQUENCE LENGTH:408

SEQUENCE TYPE:nucleic acid
TOPOLOGY:linear
CLONE:HUMGS02355
SEQUENCE DESCRIPTION:
GATCAGAGCT GCTGAGTTCA GGATGCCTGC GTGTGGTTTA GGTGTTAGCC TTCTTACATG 60
GATGTNAGGA GAGCTGCTGC CCTCTTGGCG TGAGTTGCGT ATTCAGGCTG CTTTTGCTGC 120
CTTTGGCCAG AGAGCTGGTT GAAGATGTTT GTAATCGTTT TCAGTCTCCT GCAGGTTTCT 180
GTGCCCCTGT GGTGGAAGAG GGCACGACAG TNCANGCGCA NGTTCCTGGG CTCCTCAGTC 240
GCAGGGGTGG GATGTGAGTC ATGCGGATTA TCCACTCGCC ACAGTTATCA GCTGCCATTG 300
CTCCCTGTCC TGTTTCCCCA CTCTCTTATT TGTGCATTCG GTTTGGTTTC CTGTAGTTTT 360
AATTTTTTAA TAAAGTTGAN TAAAANTATA AAAANAAGAG NNGGCGNN 408

SEQ ID NO:2071
SEQUENCE LENGTH:101
SEQUENCE TYPE:nucleic acid
TOPOLOGY:linear
CLONE:HUMGS02356
SEQUENCE DESCRIPTION:
GATCAAAAGC TTATTCATCT TTTTTCTTTT TCCTTTGGTG TAAAGCCAAC ACCCTGTTTA 60
AAAANCATAA TTTCNTTAAN CNATTTTCCC TCTTTCTCNT N 101

SEQ ID NO:2072
SEQUENCE LENGTH:102
SEQUENCE TYPE:nucleic acid
TOPOLOGY:linear
CLONE:HUMGS02357
SEQUENCE DESCRIPTION:
GATCAAAAAA AGAGTTTGAA GCTAATTTTG GCAAAATTCA GGCCAAAAAA GTAAGGCCAT 60
TATTTAACCC TGTGGCACCA TTAAATGTTG AAGAATGACA AA 102

SEQ ID NO:2073
SEQUENCE LENGTH:95
SEQUENCE TYPE:nucleic acid
TOPOLOGY:linear
CLONE:HUMGS02358
SEQUENCE DESCRIPTION:
GATCTTTTTT GTCAGTTTGT TTGTATGAAA CTAAAGCCNT TATTTGTTAA TAGTTCCTGC 60
TAAAACAATG AATAAAAACT CAAGGAGCAA CTAAA 95

SEQ ID NO:2074
SEQUENCE LENGTH:92
SEQUENCE TYPE:nucleic acid
TOPOLOGY:linear
CLONE:HUMGS02359
SEQUENCE DESCRIPTION:

GATCGTCTCT AGGATGATAT GCATGTTTCA AGTGGTATTG AAAGCCGCAC TGATGGATAT 60
GTAATAATAA ACATATCTGT TATTAATATA AA 92

SEQ ID NO:2075
SEQUENCE LENGTH:101
SEQUENCE TYPE:nucleic acid
TOPOLOGY:linear
CLONE:HUMGS02360
SEQUENCE DESCRIPTION:
GATCTAAGTT AGGAAAGACG ATGGAGGTGG AATCCTTTAA GATTATNTCC AGTTATTTGC 60
TTTAATAAAG AAGAAGTTAC CCTTGTCAAA ATCAGAACAA A 101

SEQ ID NO:2076
SEQUENCE LENGTH:87
SEQUENCE TYPE:nucleic acid
TOPOLOGY:linear
CLONE:HUMGS02361
SEQUENCE DESCRIPTION:
GATCTTCATT TATTTTAATG GTAATGGTTT TAAAATATGT TCCTGATTGT ACATATTNTA 60
AAATAAACAT GTTTTTTAAC ATGCAAA 87

SEQ ID NO:2077
SEQUENCE LENGTH:88
SEQUENCE TYPE:nucleic acid
TOPOLOGY:linear
CLONE:HUMGS02362
SEQUENCE DESCRIPTION:
GATCACAAAC ATTCAACAAA AAAGTTAACT TATGTGACTT GGCAGTTATT CTATACCATT 60
TCCTGTCCAT TAAAATTTTT AAAGGAAA 88

SEQ ID NO:2078
SEQUENCE LENGTH:85
SEQUENCE TYPE:nucleic acid
TOPOLOGY:linear
CLONE:HUMGS02363
SEQUENCE DESCRIPTION:
GATCCCATTT AATATTTAAA AAAATCAGTA GCACAAATAT ATNTNAATTG TCAACTTACA 60
AAATAAAATA CATTTACAGT CTAAA 85

SEQ ID NO:2079
SEQUENCE LENGTH:82
SEQUENCE TYPE:nucleic acid
TOPOLOGY:linear
CLONE:HUMGS02364
SEQUENCE DESCRIPTION:

GATCATCAAA AAAGAGGTAA TCTACGTTAT TTCCTATTCT AATGTCTTTT CCTAATAAAA 60
AACTTCAACT TTCTAAGTTA AA 82

SEQ ID NO:2080
SEQUENCE LENGTH:80
SEQUENCE TYPE:nucleic acid
TOPOLOGY:linear
CLONE:HUMGS02365
SEQUENCE DESCRIPTION:
GATCCTTTCG GAATTGCACT TTTACATGTT GGGCGAATTT GTGTCCGTGC TGAAGTTTAT 60
TAAAGGAAAA TAGATGGAAA 80

SEQ ID NO:2081
SEQUENCE LENGTH:80
SEQUENCE TYPE:nucleic acid
TOPOLOGY:linear
CLONE:HUMGS02366
SEQUENCE DESCRIPTION:
GATCAAGCCT TGCTTGAGAT TGGCCTTACC TCTGGNCNTT TTTTGTTATA TGAGCTAATA 60
AATNCTGTTT ATTGTTTAAA 80

SEQ ID NO:2082
SEQUENCE LENGTH:80
SEQUENCE TYPE:nucleic acid
TOPOLOGY:linear
CLONE:HUMGS02367
SEQUENCE DESCRIPTION:
GATCCATGAA GTNATTCAGT GGAAANATGC ACGTTNATAC TATTTNAGAG CACAAATAAA 60
CTCACTATAC AATGGTCAAA 80

SEQ ID NO:2083
SEQUENCE LENGTH:76
SEQUENCE TYPE:nucleic acid
TOPOLOGY:linear
CLONE:HUMGS02368
SEQUENCE DESCRIPTION:
GATCAAACCT CAAAGCAGCT TTNACTTGGA ATTTAAAAAT AAACAGTTCA AAGATACTCT 60
ACATTATCAC CNTAAA 76

SEQ ID NO:2084
SEQUENCE LENGTH:70
SEQUENCE TYPE:nucleic acid
TOPOLOGY:linear
CLONE:HUMGS02369
SEQUENCE DESCRIPTION:

GATCCATTTC ATGCAGGATT GTGTTGTTTT AACTGTTGTT GAGGAAACTA ATAAATAATT 60
AAATTGTAAA 70

SEQ ID NO:2085
SEQUENCE LENGTH:64
SEQUENCE TYPE:nucleic acid
TOPOLOGY:linear
CLONE:HUMGS02370
SEQUENCE DESCRIPTION:
GATCTAAGAT GTCCCAGGTC CTGGGAAGTT TACTCAATAA AGCTGGCTTT CCCCTGCCCT 60
CAAA 64

SEQ ID NO:2086
SEQUENCE LENGTH:64
SEQUENCE TYPE:nucleic acid
TOPOLOGY:linear
CLONE:HUMGS02371
SEQUENCE DESCRIPTION:
GATCCGTTGT AATGATGTGA ACATTTTATG AGTTTAAATA AAGTCATCTT ATGGTGTCAT 60
TAAA 64

SEQ ID NO:2087
SEQUENCE LENGTH:61
SEQUENCE TYPE:nucleic acid
TOPOLOGY:linear
CLONE:HUMGS02372
SEQUENCE DESCRIPTION:
GATCTTAAAA TCAGTGATTA TCTTTTTCTA AATAAAATAT CACCAGAATT CATCAGTTAA 60
A 61

SEQ ID NO:2088
SEQUENCE LENGTH:60
SEQUENCE TYPE:nucleic acid
TOPOLOGY:linear
CLONE:HUMGS02373
SEQUENCE DESCRIPTION:
GATCTAAGAT GTCCCAGGTC CAGGAAGTTT ACTCAATAAA GTGGCTTTCC CTGCCANAAA 60

SEQ ID NO:2089
SEQUENCE LENGTH:66
SEQUENCE TYPE:nucleic acid
TOPOLOGY:linear
CLONE:HUMGS02374
SEQUENCE DESCRIPTION:
GATCTCCGGG CAGCCACCAC CTCCTCGGTC TGCCCCCTCA TTAAAATTCA CGTTCCCACC 60

CTGAAA 66

SEQ ID NO:2090
SEQUENCE LENGTH:59
SEQUENCE TYPE:nucleic acid
TOPOLOGY:linear
CLONE:HUMGS02375
SEQUENCE DESCRIPTION:
GATCATGCAA AGAAAACTTC CAAAAAGATT TATTAACTTA AACCAGCCTC TNTNGCAAA 59

SEQ ID NO:2091
SEQUENCE LENGTH:74
SEQUENCE TYPE:nucleic acid
TOPOLOGY:linear
CLONE:HUMGS02376
SEQUENCE DESCRIPTION:
GATCTCAAAT TGCCTCTGAA CCTTTTATAA GACAGTTTAT CTTCAAATAA ATTTATTTTG 60
CAATACCACG CAAA 74

SEQ ID NO:2092
SEQUENCE LENGTH:56
SEQUENCE TYPE:nucleic acid
TOPOLOGY:linear
CLONE:HUMGS02377
SEQUENCE DESCRIPTION:
GATCAAAGTC TATTTTGCAT AAAATGTCCA ATAATTAAAT ATTGTTATAA AATAAA 56

SEQ ID NO:2093
SEQUENCE LENGTH:55
SEQUENCE TYPE:nucleic acid
TOPOLOGY:linear
CLONE:HUMGS02378
SEQUENCE DESCRIPTION:
GATCGGCAGT TGGGAGGGGC GCTCTGAGAT TAAAGAGTTT TACCTCTGAG ATAAA 55

SEQ ID NO:2094
SEQUENCE LENGTH:54
SEQUENCE TYPE:nucleic acid
TOPOLOGY:linear
CLONE:HUMGS02379
SEQUENCE DESCRIPTION:
GATCCANTCT TTGACAAACT GAAAAGAACT GAAAATAAAC ATTGCTCTTT CAAA 54

SEQ ID NO:2095
SEQUENCE LENGTH:52

SEQUENCE TYPE:nucleic acid
TOPOLOGY:linear
CLONE:HUMGS02380
SEQUENCE DESCRIPTION:
GATCCCTGCC CCTCCCCACT GGGACGGAAT AAATGCTCTG CAGACCTGGA AA 52

SEQ ID NO:2096
SEQUENCE LENGTH:266
SEQUENCE TYPE:nucleic acid
TOPOLOGY:linear
CLONE:HUMGS02382
SEQUENCE DESCRIPTION:
GATCTGCTTA TAGAGCAGGA AGAATAAAGC CACCAACTTT TACCTAGCCC GGCTAATCAT 60
GGAAGTGTGN CCAGGCTTCA AGTAACTTGA GTTTTAATTT TTTTTTTTC TTGGNAGAGT 120
AATGTAAAAT TTAAATGGGG AAAGATATTT AANATTTAAT ACTAAGCTTT AAAAAGAAAC 180
CTGCTATCAT TGCTATGTAT CTTGATGCAA AGACTATGAT GTTAATAAAA GAAAGTACAG 240
ANGACACTTG GCATTCAAAG NTTAAA 266

SEQ ID NO:2097
SEQUENCE LENGTH:451
SEQUENCE TYPE:nucleic acid
TOPOLOGY:linear
CLONE:HUMGS02399
SEQUENCE DESCRIPTION:
GATCTAGAGA GTGCTCAGAA TTAGGGCCTG GCATTTGGAA TCACAGGANA TATCATCACA 60
GAAACAACTG TTTTAAGATT AGTTCCATCA CTCTCATCCT GTATTTTNAT AAGAAACACA 120
AGAGTGCATA CCAGAATTGA ATATACCATA TGGGATTGGN GAAAGACAAA TGTGGAAGAA 180
ATCANNGNGC TGGAGACTAC TTTTGTGCTT TACAAAACTG TGAAGGATTG TGGTCACCTG 240
GAACAGTCTC CAATCTATGT TAGCACTATG TGGCTCAGCC TCTGTTACCC CTTGGNTTAT 300
ATATCAACCT GTAAACATGT GCCTGTAACT TACTTCCAAA ATCAAANTCA TACTTATTNG 360
AGGAANATTC TGATTTTNTA GANAAAANNT NGAGCNAGGN GATTATANCN TGTTTGCAAG 420
TCATGTGGTT TCTTTCTCAA TNGGGGGNNA N 451

SEQ ID NO:2098
SEQUENCE LENGTH:441
SEQUENCE TYPE:nucleic acid
TOPOLOGY:linear
CLONE:HUMGS02400
SEQUENCE DESCRIPTION:
GATCCCAAAC TGTTCCCTTT TTCATTTCTT GAAATGTTAC CACTACAGAC ATTTTTTNAA 60
GGTGAATAAA CAGTTGTNAT GTGCTGTACC TAAAATCATG TTTAATCGTA TAAGGAAACA 120
TTTCAATACA CTTATACAGG AAGAAAACTA TAGATGAAGT ACATGTGTGT GATTCAGTCT 180
GATTCACAGA ATTCTGAGAG TAATATGGAA TAAAACAACT CCACTTAGAT GATAACTGAA 240
GCATTTCCTG CCTTGTGAAA ATTTGGNTTT TAAATTGCTG TTAGAATGGG NAATTTGGAC 300
ACTTTATATC ATTGTATANT TNCAGACTTT AGNTTCTGTA TCTNTTGGGA ACCATGGTTA 360

TAGCAAAACC NTTGGNAATA ATCCTGTTTC CNANACCNCC CTNNATGTAA ACCTGGTATG 420
CTTGGCTGGT AACNCCTAAG N 441

SEQ ID NO:2099
SEQUENCE LENGTH:440
SEQUENCE TYPE:nucleic acid
TOPOLOGY:linear
CLONE:HUMGS02401
SEQUENCE DESCRIPTION:
GATCTATGTC TNCTGCCTGG CTCCTAGATG GCTCTCCGGG CAGGTNCTGG CCAAGGACAT 60
CATCTAGGCA GGGGGAGAGC CTGGNCTGAA CAGCTGTNAC CAAAACTCCC TTCTNCCCCA 120
CCCTGCCCCC TCCACTTCCT GCCCTCTGTT CCATCTTCCC CCTTCCCAAA GGCCACAGCC 180
TTTATTCCAG GCCCAGGGAT GTAGGAGGGG GAAGGAGGAA ACAGGAAGCC CAGAGAGGGC 240
AAAGGGCCTA CCTCGGGGCG CGAACCATGC CCCANACTAT TATCTCAGGG CTTTCTGGGC 300
ACTNGCACTT CAGCGTGGCC CACCTGCCCA TGCCCTGAGG CCAGTTNGCG AGGGGTGGCT 360
CCTGAGGGTT TTTATAACCT TTGTTTGNTA ATGTTTAATT TTTGCATCAT AATTTCTACA 420
TTGTCCCTGG AGTGTAAGAN 440

SEQ ID NO:2100
SEQUENCE LENGTH:411
SEQUENCE TYPE:nucleic acid
TOPOLOGY:linear
CLONE:HUMGS02402
SEQUENCE DESCRIPTION:
GATCAAAAGG CTGAAAGGTT TGCTCACCCA GCACGGCATC CCCTACACGA GGCCCACAGA 60
AACTTCCAAC TTGGAGCACT TGGGCCACGA GACGCCCAGA GCCAAGTCTC CAGAGCAGCT 120
GCGGGGTGAC CCAGGACTGC GTGGGAGTTT GTGACCTTGT GGTGGGAGAG CAGAGGTGGA 180
CGNGGCCGAG AGCCCTACAG AGAAGCTGGC TGGTAGGACC CGCAGGACCA GCTGACCAGG 240
CTTGTGCTCA GAGAAGCAGA CAAAACAAAG ATTCAAGGTT TTAATTAATT CCCATACTGA 300
TAAAAATAAC TCCATGAATT CTGTAAACCA TTGCATAAAT GCTATAGTGT AAANAAATTT 360
AACCAAGTGT TACTTTAAAC AGTTCGTACA AGTAATGNTT ATAATTCTAA A 411

SEQ ID NO:2101
SEQUENCE LENGTH:408
SEQUENCE TYPE:nucleic acid
TOPOLOGY:linear
CLONE:HUMGS02403
SEQUENCE DESCRIPTION:
GATCTGGAAA GCTTGAGCCT CCTTGGGTTC GTCTACAAAT TGGAAGGAAA TATGAATGAA 60
GCCCTGGAGT ACTATGAGCG GGCCCTGAGA CTGGCTGCTG ACTTTGAGAA CTCTGTGAGA 120
CAAGGTCCTT AGGCACCCAG ATATCAGCCA CTTTCACATT TCATTTCATT TTATGCTAAC 180
ATTTACTAAT CATCTTTTCT GCTTACTGTT TTCAGAAACA TTATAATTCA CTGTAATGAT 240
GTAATTCTTG AATAATAAAT CTGACAAAAT ATTAGTTGTG TTCAACAATT AGTGAAACAG 300
AATGTGTGTA TGCATGTAAG AANGAGAAAT CATTTGTATG AGTGCTATGT AGTAGAGAAA 360
AAATGTTAGT TAACTTTGTA GGAAATAAAA CATTGGACTT ACACTAAA 408

SEQ ID NO:2102
SEQUENCE LENGTH:397
SEQUENCE TYPE:nucleic acid
TOPOLOGY:linear
CLONE:HUMGS02404
SEQUENCE DESCRIPTION:
GATCCGAGAG GTGGCCAACA AAGTCAAGGT CCCCCTGCTG GATTTAGGCA GCCCCCAACT 60
GGCCATGCAC TCTATCCGGN AGATGGCCTG CACCACAGGA GTCCTCCANA CCCTNACCCT 120
CTTCAAGGGC TTCTTTAAGC TGTTCCCTTC TCTAAGCCAT AATCTCTNAG TGGATTGAGC 180
CCTCTTGGAA AGACTTCTCT GCCATCCCTT TGCACCTGAN AGGGGAAGTT CTCAGCTGAG 240
CTGAAGCTGG ATTATTAAAG TGGATTGTAA CTCAGACTCT CCGTGCTACG CTTATTTGGA 300
GACTAGAGGA GTGGGAGTTG AGCCTGGCTT GAACCTTTGG AACCAGAAAA GTTGGGGAGC 360
AGGTGGAGGA GGCCACACTC CTGGGAGCTT GATGGTN 397

SEQ ID NO:2103
SEQUENCE LENGTH:219
SEQUENCE TYPE:nucleic acid
TOPOLOGY:linear
CLONE:HUMGS02406
SEQUENCE DESCRIPTION:
GATCAGCCCT TTTTCCCATC CTGCCCTATG GTTCTCTAGC CACCTGTGCA TGCATGTGTA 60
TTTCTGCCTN GTTCTATGGT GTGTGGATGT GTGTGCATGA ATCTGTCATA TAGAGGGGGT 120
CCGAGCTGGA ATCCTAGAGC ATTGCTGCCC TGGGGCCTGA TGTTCTTGGC TTCCTCAGAG 180
CATGTAACAG GAAATTAAAT GGGATGAGTG TTTGGTAAA 219

SEQ ID NO:2104
SEQUENCE LENGTH:380
SEQUENCE TYPE:nucleic acid
TOPOLOGY:linear
CLONE:HUMGS02408
SEQUENCE DESCRIPTION:
GATCCTACCT CCAACTCCTG ACTCTATAGG TCTCACTCTT TTTTTATTGT CCTANNNNTA 60
CATAACATAA AATTTATAAA TTTAATATTC TAGGGTATAG TTCAGTGGTA TTAAGTACAT 120
TCACACTGAT GTGCAACCAT CATTATCATC CATTCCCAAA ACTTTTCATC ACCGCAAATA 180
GAAAAGCTGT ACCCATGAAA CAATAACTTC CATTGCCTCC CCCTCAGCCT TTGGTAACCT 240
CTATTCTACT TTCTGTTTCT ATGAATTTGA CTGTTCTAGG TACCTCATAT AAGTGGAATC 300
ACACAATATT TGTCCCTTTG TGTCTAACTT ATTTCACTTA GCATGTTTTC AAGATTTATC 360
TGTACTGTAC CATGTATCAN 380

SEQ ID NO:2105
SEQUENCE LENGTH:378
SEQUENCE TYPE:nucleic acid
TOPOLOGY:linear
CLONE:HUMGS02409

SEQUENCE DESCRIPTION:
GATCTGTGCT CTCCTAGCCC AAGAGACCCC TGGAGGGGCT GGAGTTTTAT CCAGCGNCTC 60
GTCGTATGTT TGGCTGAGCA CCTGTGGCCT GGGTGCAGGT TAACTTCTTG TTATCAGGAG 120
CCCACTATGC AGAGGCCAAA GGTCGGCAGC CAGCGAGGCT ATGAATTGGA CCTTTTTGGT 180
ATCTGTGTNA CTGNTCTGTG NCCATNCTTA GNCAACTTGC TGGCGTGACA AGTGCCCACA 240
AGTAACACAC CAGGTACCCA GAGNAGGGTG GACAGGAGAG ACCTGAATNA CAGCAGTANG 300
GAATTCCTCA ANGNTGTGNC CTCGCCCATG AANTTGTTCC AANTTGAAGG TNANNAGGGN 360
CACCAATCAG GGTTGAGN 378

SEQ ID NO:2106
SEQUENCE LENGTH:374
SEQUENCE TYPE:nucleic acid
TOPOLOGY:linear
CLONE:HUMGS02411
SEQUENCE DESCRIPTION:
GATCCAGAAG TACAAGGTGG TCCTGGCCCC GGAGACGGNG GAGTTGAAGA GTGTGGCCCG 60
CATTGTCCTG GTTCCCAATA AGAAAGTGGG CCTGCAGTTC CTGCAGAGAC AGTGCTGAGC 120
TGAGTCTCCG CCTTGCTGGG GCTTGTCCTA GAGGCTCCAG CTCTGGCACA GTGGTTCCTG 180
GCTGCTGCCA TGTCTCANAT GAGGAGGGAG AGAAGGAGGC CGCCAGACTC GAGAGGTGGG 240
AGGAACTCCT TGCACACACC CTGAGCTTTT GCCACTTCTA TCATTTTTGA GAACTCCCTC 300
TCAGCTAAAA GGCCACCCTT TTATCGAATG CTGTCCTTGG GAAGATATAA AATAAAGGGC 360
TNTTATTTCT TAAA 374

SEQ ID NO:2107
SEQUENCE LENGTH:369
SEQUENCE TYPE:nucleic acid
TOPOLOGY:linear
CLONE:HUMGS02412
SEQUENCE DESCRIPTION:
GATCCAGCCC GACCTGCGAG ANTGATGGAG ACCATGCACC GCATGAGCCA CCTCCCACCC 60
GACTTTGAGG GCCGCCAGAC GGTCAGCCAG TGGCTGCAGA CCCTGAGCGG ATGTCGGCGT 120
CAGATGAGCT GGACGACTCA CAGGTGCGTC AGATGCTGTT CGCCTGGAGT CAGCCTACAN 180
CGCCTTCANC CGCTTCCTNA TGCCTGAGCC CGGGGANTAG CCTTGNACAG CCGGGAGAGT 240
CTGAGGCNTG GTCCTGGTCC CTGTCCGCAN AGAGGCCGTG NNATCACANA GCTANTGTCT 300
GAGTGGCTGT CTGGNTCTGC TTTGGGTAAG ACTTTGGGGC CGGCCTCCCA NAATAAGGTG 360
TCTCGGAAA 369

SEQ ID NO:2108
SEQUENCE LENGTH:368
SEQUENCE TYPE:nucleic acid
TOPOLOGY:linear
CLONE:HUMGS02413
SEQUENCE DESCRIPTION:
GATCAGAGCC AAGAAGCGTC TGCGGCAGAG TGGGGAAGAG TTGCCGCCAA CCTCCTAGGC 60
GCCCCGCCCA GCTCCCTTTG ACCCCTGGGG CAGGGCAGGG GGCAGGGAGA GACAAGGCTG 120

CTGCTATTAG AGCCCATCCT GGAGCCCCAC CTCTGAACCA CCTCCTACCA GCTGTCCCTC 180
AGGCTGGGGG AAAACAGGTG TTTNATTTGT NACCGTTGGA GCTTGGATAT GTNCGTGGCA 240
TGTGTGTGTN TGTGTNANAG TGTGAATGCA CAGGTGGGTA TTTAATCTGT ATTATTCCCC 300
GTTNTTGGAA TTGNNTTCCC CNATGGGNCT GGGGTACTTT ACATTCAATA AATACTGTTT 360
AACCCAAA 368

SEQ ID NO:2109
SEQUENCE LENGTH:368
SEQUENCE TYPE:nucleic acid
TOPOLOGY:linear
CLONE:HUMGS02414
SEQUENCE DESCRIPTION:
GATCACAGGA AGTGGAGGAG CCAGAGGTGC CTTTGTGGAG GACAGCAGTG GCTGCTGGGA 60
GAGGGCTGTG GAGGAAGGAG CTTCTCGGAG CCCCCTCTCA GGCTTACCTG GGCCCCTCCT 120
CTAGAGAAGA GCTCAACTCT CTCCCAACCT CACCATGGAA AGAAAATAAT TATGAATGCC 180
ACTGAGGCAC TNAGGCCCTA CCTCATGCCA AACAAAGGGT TCAAGGCTGG GTCTAGCGAG 240
GATGCTTGAA GGAAGGGAGG TATGAGACCG TAGGNANAAN GGCACCATCC TCGTACTGTT 300
GTCACTATGA GCTTAAGAAA TTTGATACCA TAAAATGGTA AAGACTTGAA AAAAGTANAA 360
NATGTTTN 368

SEQ ID NO:2110
SEQUENCE LENGTH:368
SEQUENCE TYPE:nucleic acid
TOPOLOGY:linear
CLONE:HUMGS02415
SEQUENCE DESCRIPTION:
GATCTTGGTT TTGATGTGGA CATAGGCCCT CTTTTCCAGA CTCCTCGTGA AGTGGCCCAG 60
CAGGCTGTGG ATGCGGATGT GCATGCTGTG GGCGTAANAC CCTCGCTGCT GGTCATAAAA 120
CCCTAGTTCC TGAACTCATC AAAGAACTTA ACTCCCTTGG ACGGCCAGAT ATTCTTGTCA 180
TGTGTGGAGG GGTGATACCA CCNTCAGGAT TATNAATTTC TGTTTGAAGT TGGTGTTTCC 240
AATGTATTTG GTCCTGGGAC TCGAATTCCA AAGGCTGCCG TTCAGGTGCT TNATGATATT 300
GAGAAGTGTT TNGAAAAGAA GCAGCAATCT GTATAAAATA AAAATATCAC AAGAGAACAC 360
CTGTACTN 368

SEQ ID NO:2111
SEQUENCE LENGTH:365
SEQUENCE TYPE:nucleic acid
TOPOLOGY:linear
CLONE:HUMGS02416
SEQUENCE DESCRIPTION:
GATCGAAAGA AATCTAAGTG ATACGGCCCA ATGAAGCTAA AATATAGCCT TCTGTTAAGC 60
AAATAGTATT TCCTTTCCCC AAGTAGTTCA TTTTCTAGAT GCTTGTCAAA TGAATTAATG 120
TCCTCTGATG AAGAGTGTCC TTCCGTTTCT AAGGTCTTCT CAATCTCAGC AATAGAGCTT 180
CCCAGCAGCG TTCAAGACAC ATCATTTATA CACAGGCACA GGGGCCTTCC TGAAATGGGT 240
GCATTTTTAC CAACTACAAT CATGTAATTT TTTTGGAAAT TTTTTAAAAT TTTCGNTNCT 300

TTACATTACA ATTGGGTGAA ACACATTTTA CAGCTCTCAA TAAATGTTTG CTGGTCGCTC 360
TGAAA 365

SEQ ID NO:2112
SEQUENCE LENGTH:363
SEQUENCE TYPE:nucleic acid
TOPOLOGY:linear
CLONE:HUMGS02417
SEQUENCE DESCRIPTION:
GATCAACGAT TACGTGGAGA AGGGTACTCA AGGGAAAATT GTGGATTTGG TCAAGGAGCT 60
TGACAGAGAC ACAGTTTTTN CTCTGGTGAA TTACATCTNC TTTAAAGGCA AATGGGAGAG 120
ACCCTTTGAA GTCAAGGACA CCGAGGAAGA GGACTTCCAC GTGGACCAGG TGACCACCGT 180
GAAGGTGCCT ATNATGAAGC GTTTAGGCAT GTTTAACATC CAGCACTGTA AGANGCTGTC 240
CAGCTGGGTG CTGCTGATGA AATACCTGGG CAATGCCACC GCCATCTTCT TCCTGCCTGA 300
TGAGGGGAAA CTACAGCACC TGGAAAATGA TTGACCAAAA TACCAAGTCT CCCCTTCTTC 360
ATN 363

SEQ ID NO:2113
SEQUENCE LENGTH:362
SEQUENCE TYPE:nucleic acid
TOPOLOGY:linear
CLONE:HUMGS02418
SEQUENCE DESCRIPTION:
GATCCCAGTC GCATACCAGG TGTCCTCCTC CAGGTCGTGA ACGGCAAAGG CACTGCCCGC 60
ATCGCCATAG CAGGTGTCTT CTTGGTACTT AGACATGCCA GCACAGAAGG TGTGTTCATT 120
CAGTATGGGC TGCACCCCTA CAGGGCTCTT CGGTGTCTTC CATCCAGGAC TGGGTTCAGA 180
AGACCATAGC TGAGAACTAA TGCAAGGCTG GCCGGAAGCC CTTGCCTGAA AGCAAGATTT 240
CAGCCTGGAA GAGGGCAAAG TGGACGGGAG TGGACAGGAG TNGATGCGAT AAGATGTGGT 300
TTGAAGCTTG ATGGGTGCCA GCCTGCATTG CTGAGTCAAT CAATAAAGAG CTTTTCTTTT 360
GN 362

SEQ ID NO:2114
SEQUENCE LENGTH:362
SEQUENCE TYPE:nucleic acid
TOPOLOGY:linear
CLONE:HUMGS02419
SEQUENCE DESCRIPTION:
GATCNGACAG GCACCAGCAA TGACACAGCA TAAACTGTTG GGCACAGGAA AGAGGCTTGA 60
CACCACCAGC CCACCAGGAA ATGCAGTTCA GCCACCACGA GGACACGAGG AACCTGCACC 120
ACAGCCGCAG TCAAGAGAAT GCTGGTGGTG CCAAGNTGCA GGGTGTGGCC TGCTGGAATG 180
GAAGCGCATG GTGGTNNCTA TAAAGTCAAT GNCACAGCAC ATGCCCAGCA ATCCACTGTG 240
GGATTNGNGA TGANACTANG CNCATAAAAC TGTNCGAATT TTNCTGTGCT TTTCATATCN 300
TCAATNTGAA TCAANTGCTT CATTGGGTAC TGTAACTGTG GNTACTTNAG TGAATCTCTN 360
TN 362

SEQ ID NO:2115
SEQUENCE LENGTH:355
SEQUENCE TYPE:nucleic acid
TOPOLOGY:linear
CLONE:HUMGS02420
SEQUENCE DESCRIPTION:
GATCATATAG TAGGTTTAGG TTTAACTTTT TAAGAAATTG CCAAGCTTTT CCAAAGTGGC 60
TGTCCCTCTT TACATTTCCA CCAGCAATGT ATAATGAATG GTTCAGGTTC CTCCACATTC 120
TTGTCGGCAC TTGGACTTTT CTGTTTTGTT GTAGCCATTC TAGTGGATGC AAAGTGGCAT 180
CTCATTATGG TTCATTTGCA TTTCTCTGAC AACTAATGAT TTTGAGCATC TTCATGCGAT 240
TGTTAACCAT TTGTATATCT TTAGTGAAAT ATCTATTCAA TTATTGCCCA TTTTTAAATT 300
GGNTCACATG GCTNATTAAT GNATTGTAAG AAAGGTTTAC ATATTCCTGG ATACN 355

SEQ ID NO:2116
SEQUENCE LENGTH:350
SEQUENCE TYPE:nucleic acid
TOPOLOGY:linear
CLONE:HUMGS02421
SEQUENCE DESCRIPTION:
GATCATGATT TTGTAGTACA GGAAGACTTC ATGAAAGCAG TCAGAAAAGT GGCTGATTCT 60
AAGAAGCTGG AGTCTAAATT GGACTACAAA CCTGTGTAAT TTACTGTAAG ATTTTTGATG 120
GCTGCATGAC AGANGTTGGC TTATTGTAAA AATAAAGTTA AAGAAAATAA TGTATGTATT 180
GGTAATGATG TCATTAAAAG TATATGANTA AAAATATGAG TAACATCATA AAAATTAGTA 240
ATTCAACTTT TAAGATACAG AAGAAATTTG TATGTTTGTT AAAGTNGCAT TTATTGCAGC 300
ANGTTACAAA GGGGAAGTGT TGAAGCTTTT CATATTTGCT GCGTGACATN 350

SEQ ID NO:2117
SEQUENCE LENGTH:375
SEQUENCE TYPE:nucleic acid
TOPOLOGY:linear
CLONE:HUMGS02422
SEQUENCE DESCRIPTION:
GATCCGCAGN CAGAAGCTCT GCCCTTCTGT ATCCTATGTA TGCAGTGTNN TTTTNCTTGC 60
CAGCTTGGGC CATTCTTGCT TAGACAGTCA GCATTTGTNT CCTCCTTTAA CTGAGTCATC 120
ATCTTAGTCC AACTAATGCA GTCGATACAA TGCGTAGATA GAAGAAGCCC CACGGGAGCC 180
AGGATGGGAC TGGTCGTGTT TGTNCTTTTN TCCAAGTCAG CACCCAAAGG TCAATGCACA 240
GAGACCCCGG GTGGGTGAGC GCTGGCTTCT CAAACGGCCG AAGTTGCCTC TTTTAGGAAT 300
CTCTTTGGAA TTGGGAGCAC GNTGNNCTCT TGAGTTTGGA GCTTTTAAAA GTACTTTNTT 360
TACCACATTT GGAAA 375

SEQ ID NO:2118
SEQUENCE LENGTH:339
SEQUENCE TYPE:nucleic acid
TOPOLOGY:linear
CLONE:HUMGS02423

SEQUENCE DESCRIPTION:
GATCTCTAAC CAGAAATAAA CAGGAAGACT CAGTNGTTTA CAAAAGACAT TTTAGTTTTA 60
CACGTACAGA AAATTCTACC CNGGCATTAC AGAATTCTTA GACTTCTTAA ATTCCTGATT 120
TTCCTTGGTA TTTACATTTT GATAAGGGAG CCAATGGTTC TTCAAACATT TGAAAAGAAC 180
ACATACACAC TGGACAATTT CAAACGNTAC AAACAAACGC AACTGTCCTT CTTTCTCATT 240
CCTGTCCTTC AGCCACCCAG TTTCTTCTCC CCTGAGGGAA TCAGTTATCA TTNGCTGGGT 300
CATACTTCCA GATTATCCTG TGCCTATGTA AGTGTGTGN 339

SEQ ID NO:2119
SEQUENCE LENGTH:334
SEQUENCE TYPE:nucleic acid
TOPOLOGY:linear
CLONE:HUMGS02424
SEQUENCE DESCRIPTION:
GATCTGGGAC GTNCACGAGT NAGGGGCTGG CCACCCAGCC CACAGCCTTG CCTGACCACC 60
CTCCAGCAGA TAGACGCCGG CACCCCTTCC TCTTCCTAGG GTGGAAGGGG CCCTGGTNCN 120
NGCCTGTAGA CCTATCGGNT CTCATCCCTT GGGATAAGCC CCAGTCCAGG TCCAGGAGGC 180
TCCNTCCCTG CCCAGCGAGT CTTCCAGAAG GGGTGAAAGG GTTNCAGGTC CCGACCACTG 240
ACCNTTCCCG GGTGNCCTCC CTCCCCANCT TACACCTNAA GCCCAGCACG NAGTAGNCTT 300
TGAACAGAGG GAGGGAAGGA CCCATGGGTC TCCN 334

SEQ ID NO:2120
SEQUENCE LENGTH:330
SEQUENCE TYPE:nucleic acid
TOPOLOGY:linear
CLONE:HUMGS02425
SEQUENCE DESCRIPTION:
GATCAAAGAG AAAATAAAAA ATCGCTACTT CCCTGCCTTT GAAAAAGTCT TAAAGAGCCA 60
TGGACAAGAC TACCTTGTTG GCAACAAGCT GAGCCGGGCT GACATTCATC TGGTGGAACT 120
NCTCTACTAC GTCGAGGAGC TTGACTCCAG TCTTATCTCC AGCTTCCCTC TGCTGAAGCT 180
CTCCAGGACA CTGGCCCCCC ACACTGTATC TCTCACTGAG AAAAGGTGGT CCCATGGCCC 240
TGAAAACCAG AATNAGCAAC CTGNCCCACA GTGAAGAAGT TTCTACAGCC TNGGCAGCCC 300
AAGGAAAGCC TCCCATTGGA TGAGAAATCN 330

SEQ ID NO:2121
SEQUENCE LENGTH:327
SEQUENCE TYPE:nucleic acid
TOPOLOGY:linear
CLONE:HUMGS02426
SEQUENCE DESCRIPTION:
GATCGAAGTA AAATAGAAAT GGGACTNGCT TTCCACAGGA AGTAAACTGC TTCAGAGCCC 60
ACAGTCCCCT GCTCAGTGTC CGGAAAGAAG TCAGTCATCC CTGTTGGCAG TAAATCTTCC 120
CACAGGCCGT CCATTAGAGA TTTAACTAGA TATGTTCAAT AGAAAGAGTC TGAGGCAAGT 180
GGAAATGAGG NNCGGAAACT TAGGTTGGGA GAATATTTTT NTTNTATTCA TTCTGTTTGC 240
TTAATTCAGA GTACAGTTTG TGCTATTTCA TATCTGTACT CCAGGCAGAA ATATAACTTG 300

AAAATACTGT GTCTAAAGAA ATTTCAN 327

SEQ ID NO:2122
SEQUENCE LENGTH:324
SEQUENCE TYPE:nucleic acid
TOPOLOGY:linear
CLONE:HUMGS02427
SEQUENCE DESCRIPTION:
GATCCTCGTC ATCAAGGATG GCTGCATCGT GGAGAGGGGA CGACACGAGG CTCTGTTGTC 60
CCGAGGTGGG GTGTATGCTG ACATGTGGCA GCTGCAGCAG GGACAGGAAG AAACCTCTGA 120
AGACACTAAG CCTCAGACCA TGGAACGGTG ACAAAAGTTT GGCCACTTCC CTCTCAAAGA 180
CTAACCCAGA AGGGAATAAG ATGTGTCTCC TTTCCCTGGC TTATTTCATC CTGGTCTTGG 240
GGTATGGTGC TAGCTATGGT AAGGGAAAGG GACCTTTCCG AAAAACATCT TTTNGGGGAA 300
ATAAAAATGT GGACTGGTGC GAGN 324

SEQ ID NO:2123
SEQUENCE LENGTH:355
SEQUENCE TYPE:nucleic acid
TOPOLOGY:linear
CLONE:HUMGS02428
SEQUENCE DESCRIPTION:
GATCTGAACA TTCAAAAGAA AGCTTTGGAA AAAAAGAGCT GGCTGGCCTA AAAACCTAAA 60
TATATGATGA AGATTGTAGG ACTGTCTTCC CAAGCCCCAT GTTCATGGTG GNNCNNTGGT 120
TATTTGGTTA TTTTACTCAA TTGGTTACTC TCATTTGAAA TGAGGGAGGG ACATACAGAA 180
TAGGAACAGG TGTTTGCTCT CCTAAGAGCC TTCATGCACA CCCCTGAACC ACGAGGAAAC 240
AGTACAGTCG CTAGTCAAGT GGTTTTTAAA GTAAAGTATA TTCATAAGGT AACAGTTATT 300
CTGTTGTTAT AAAACTATAC CCACTGCAAA AGTAGTAGTC AAGTGTCTAG GTCTN 355

SEQ ID NO:2124
SEQUENCE LENGTH:324
SEQUENCE TYPE:nucleic acid
TOPOLOGY:linear
CLONE:HUMGS02429
SEQUENCE DESCRIPTION:
GATCTCAGAA CTGAAGGCCG CCCTGACATG AAGACTGAGC TCTTTTCCAG CTCATGCCCA 60
GGTGGAATCA TGCTGAATGA GACAGGCCAG GGTTACCAGC GCTTTCTCCT CTACAATCGC 120
TCACCACATC CTCCCGAAAA GTGTGTGGAG GAATTCAAGT CCCTGACTTC CTGCCTGGAC 180
TCCAAAGCCT NCTTATTGAC TCCTAGGGAA TCAAGAGGCC TGTGAGCTGT CCAATAACTG 240
ACCTGTAACT TCATCTAAGT CCCCAGATGG GGNACANTGG GGAGNTNGAG TTGTTGGAGG 300
GAGGAAGCTT GGAGACTTCC CAGN 324

SEQ ID NO:2125
SEQUENCE LENGTH:324
SEQUENCE TYPE:nucleic acid
TOPOLOGY:linear

CLONE:HUMGS02430
SEQUENCE DESCRIPTION:
GATCCATCTT CATCCATTGC ATTGGAAACT NCTTTATGCT GCTGCAGTCT GCAAAGTCTA 60
GAGCTTTTAT CAGGCCATGT CATACCCAAG AAAGCACCTA TTTAAAGAAA AAACAATTCC 120
CTGAGCTCTC AACTCCAAGT TGTAGATTTG GTGTCTTCCT TGTCCTTACT TTAAAAAGTC 180
ATGTGTTAAT TTTNNTCCTG CCTGTATTTG TATGCAAAAT GTCCTCTATC TGCTATNAAA 240
GAAAAGCTAC GTAAACCACT ACATTGTANC CTTCTAAGTN ATTNTTAATT AGNAGGGNAT 300
TATNTTGCAG TACCANTTGG GAAA 324

SEQ ID NO:2126
SEQUENCE LENGTH:321
SEQUENCE TYPE:nucleic acid
TOPOLOGY:linear
CLONE:HUMGS02431
SEQUENCE DESCRIPTION:
GATCGTGAAG ACCTGCTGAG CCAGCCTGTT CTCCGGGCCT GAATGTCTGG GGTGCTTGTG 60
CCTTTTCTGA GAAGCGTTGT GACTGCTCAA CATCCCCATC AAGGTTTGAG TCCACAAAAG 120
TGGACCTCCC TATCATGCTT CCCCTTCCCT CTAGCATGTG GGAAGGGACT GCTGTGAAGA 180
ATGNCAGATG TGGGGCCTCT GCCAAGTTCT TGCATTGCTA AATAAGGGCT TCCTCTGCCT 240
TCTACCTACA GTGCATTTGA ACTGCCTTCT GAAAGAGGTC CAGGGAGGGA TTTAGGAAAT 300
AAAGNTTCTA CCTATTTGAA A 321

SEQ ID NO:2127
SEQUENCE LENGTH:319
SEQUENCE TYPE:nucleic acid
TOPOLOGY:linear
CLONE:HUMGS02432
SEQUENCE DESCRIPTION:
GATCCTAAGC GAAGATGGAT TTCAGTTCAT CAAATCATCA TTAATGACTT TATGTATTAT 60
TTGCACAGGG AGAATTGAAA CTGAGTATAA TCAATAAGCT AGATACGAAA TCAGTTTCTC 120
AAACTGAGCT TCAGAAAGGG GCATTTTGTA CTCTTGTTTT TGCATAACTG GTTTTGTTTT 180
TTTGCAGAAT TAACTATAAC ANTCACTGGC TACCGAAGTA AACTGATGTA CTGAATTCCA 240
TAATACATAA CATTCAATTT TTACCACTNC TGTTTAGCGA ACTTGTATAC TTATTTCCTG 300
TTCAGNTTAA AAAAAGAAA 319

SEQ ID NO:2128
SEQUENCE LENGTH:315
SEQUENCE TYPE:nucleic acid
TOPOLOGY:linear
CLONE:HUMGS02433
SEQUENCE DESCRIPTION:
GATCACACCT GAAAGCAGTG GTCCCAGCTC TGTGCTGTGG GACATCACAG GTCCTAAACA 60
GTGATAGAGA AAGGGCTGGG GAGTGGCAGC TGGAATCTCT GCTTCCGTCT GAACATCTGG 120
ACACACATGT CAGAGAATCT TTGCATTAAA TGGACTGCCC AATCTATTTG GTGATTAAAT 180
GGCTGTGCGG TGAGGGGAGA ACCCTGGGCA GTCTGGAGAC TGAGGTGCCA TTCTCGGCTA 240

GAGACACCAC CCAAAGAAGA GATGGGCTGG CTACTAAAGA CAAGTNTCAG TTGAATAAAT 300
GTCTTTAAAC CTAAA 315

SEQ ID NO:2129
SEQUENCE LENGTH:311
SEQUENCE TYPE:nucleic acid
TOPOLOGY:linear
CLONE:HUMGS02434
SEQUENCE DESCRIPTION:
GATCATTGTC CCTACAGACA CCCAGAACAT CTTCTTCATG AGCAAAGTCA CCAATCCCAA 60
GCAAGCCTAG AGCTTGCCAT CAAGCAGTGG GGCTCTCAGT AAGGAACTTG GAATGCAAGC 120
TGGATGCCTG GGTCTCTGGG CACAGCCTGG CCCCTGTGCA CCGAGTGGCC ATGGCATGTN 180
TGGCCCTGTC TGCTTATCCT TGGAAGGTGA CAGCGATTCC CTGTGTAGCT CTCACATGCA 240
CAGGGGCCCA TGGACTCTTC AGTCTGGAGG GTCCTGGGNC TNCTGACAGN AATAAATAAT 300
TTCGTTGGAA A 311

SEQ ID NO:2130
SEQUENCE LENGTH:309
SEQUENCE TYPE:nucleic acid
TOPOLOGY:linear
CLONE:HUMGS02435
SEQUENCE DESCRIPTION:
GATCCCAACT GCTCCTGCGC CGCCGGTGAC TCCTGCAAAT GCAAAGAGTG CAAATGCACC 60
TCCTGCAAGA AAAGCTGCTG CTCCTGCTGC CCTGTGGGTT GTGCCAAGTG TGCCCAGGGC 120
TGCATCTGCA AAGGGGCGTC GGACAAGTGC AGCTGCTGCG CCTGATGCTG GGACAGCCCG 180
CTCCCAGATG TAAAGAACGC GACTTCCACA AACCTGGATT TTTATGTAC AACCCTGACC 240
GTGACCTTTT GCTATATTCC TTTTTCTATG AAATAATGTG AATGATAATA AAACAGCTTT 300
GACTTGAAA 309

SEQ ID NO:2131
SEQUENCE LENGTH:307
SEQUENCE TYPE:nucleic acid
TOPOLOGY:linear
CLONE:HUMGS02436
SEQUENCE DESCRIPTION:
GATCCTGGCC TCGCAGTTCC TATCCCNTGA CCCAGAACAG CTCACCAAGG AGCTGCAGCA 60
GCATGTGAAG TCAGTGCAGC CCATACAAGT CCCAAANAAG ATAGAGTTTG TNTTGAACCT 120
GCCAAAGCTG TCACAGGGAA ATTCAACAGA CCAAACTTCG NGNCAAGGAG TGGAAGATGT 180
CCGGAAAAGC CCGTNCGAGT GAGNCGTCTA GGAGCATTCA TTTGGATTCC CTCTTCTTTC 240
TCTTTCTTTT CCCTTTGGGC CTTGGCTTAC TATNATGATA TGAGTCCTTT ATGAAGACAT 300
GATGAAA 307

SEQ ID NO:2132
SEQUENCE LENGTH:306
SEQUENCE TYPE:nucleic acid

TOPOLOGY:linear
CLONE:HUMGS02437
SEQUENCE DESCRIPTION:
GATCCTGGTG GCGACCATGG AGAGGAAACT GGAGGGCAGG GATGGAGCTG AAANCTTGGC 60
GGCCCAGAGA GAGGTCCACC CCAAGCAGCC TGAGCCCTCA GCCACCCCCC AGCTCCCTGG 120
CAGCTCCCCT CCACCTGCCA ATGTCAGCGC CACACTGGTG TCTGAAAGGA ATAAGGAGAA 180
CAGGACAGAC TAACTTTTTA AATGATATGA AGAAATCAGA GGTGAAAATT GTACATTGGG 240
AATATATTTA TGCAAATTTT ATTGAAATTT ATTGTAAATA AAGATTTTCT CAGTGGTCTA 300
GANAAA 306

SEQ ID NO:2133
SEQUENCE LENGTH:306
SEQUENCE TYPE:nucleic acid
TOPOLOGY:linear
CLONE:HUMGS02438
SEQUENCE DESCRIPTION:
GATCTGGGCT GGTCCCTTTC CCATAAAATN AGGTCCCTGG TTGTATGTTC CCATAGCACC 60
CCATACTTCC TCTCTNAGAA TAATCATTTC CCTTGTAATG CTCAGCATCC GCATCCTGCT 120
TGACTGCAAA CTTGCTGAAG GTAGGGACTG TTTGTCTTGG ACTTCGCTGC CAGTCCTTAG 180
AACAGTGTCT GGGACACAGT GTGTTCTCAA ATATTTGTTG CTGGAATAAA TGAATGACCT 240
AAATCAGTCT TTTAGGGATT TACTGTTAAC CACCATGGGA AAATTAAATA AATGCGGGGA 300
AGGAAA 306

SEQ ID NO:2134
SEQUENCE LENGTH:305
SEQUENCE TYPE:nucleic acid
TOPOLOGY:linear
CLONE:HUMGS02439
SEQUENCE DESCRIPTION:
GATCTGACCC CACAGCCTTT GGCCTGCTCA CCCGACCAGC CTGGGCCGCA TTCCCTGCAG 60
GACCTCCCGG GTCAGGCAGG GCGGCCCCCT GCTCCACACA CTGCTGCATC TTGGGTCTCA 120
GGGACCCAGA CAGATGGACT TACATGGAGC TGGGCACTGC CCTGCCTCAA CAGGGTCCAT 180
TGCCTCCTCG CCTCCAGAAC TTGGAGCAGG GAAGTGGGCA CCCTGAGGCC TCCAGCACCA 240
GTTCCGTCAT TCTCGTTCCT GGGGAACCCC CACTCTGACC TGTTATTAAA GTTCACATTT 300
TGAAA 305

SEQ ID NO:2135
SEQUENCE LENGTH:251
SEQUENCE TYPE:nucleic acid
TOPOLOGY:linear
CLONE:HUMGS02441
SEQUENCE DESCRIPTION:
GATCAACCTG AGAAAAATTG CTGAGCTCAT GCCAGGAGCA TCAGGGGCTG AAGTGAAGGG 60
CGTGTGCACA GAAGCTGGCA TGTATGCCCT GCGAGAACGG CGNGTCCATG TCACTCAGGA 120
GGACTTTGAG ATGGCAGTAG CCANGGTCAT GCAGAAGGNC AGTGAGAAAA ACATGTCCAT 180

CAAGCANTTA TGGANGTGAG TGGACAGCCT TTGTGTGTAT CTCTCCAATA CAGCTCTGTG 240
GGCCAAGTAA A 251

SEQ ID NO:2136
SEQUENCE LENGTH:301
SEQUENCE TYPE:nucleic acid
TOPOLOGY:linear
CLONE:HUMGS02442
SEQUENCE DESCRIPTION:
GATCTGCATC CCCAAAAGTA TCACTCCTTC TCGAATCCTT CAGAACATCA AGGTGTTTGA 60
CTTCACCTTT AGCCCAGAAG AGATGAAGCA GCTAAATGCC CTGAACAAAA ATTGGAGATA 120
TATTGTGCCT ATGCTTACGG TGGATGGGAA GAGAGTCCCA AGGGATGCAG GGCATCCTCT 180
GTACCCCTTT AATGACCCGT ACTGAGACCA CAGCTTCTTG GCCTCCCTTC CAGCTCTGCA 240
GCTAATGAGG TCCTGCCACA ACGGAAAGAG GNAGTTAATA ANNGCCATTG GANGCATNAA 300
A 301

SEQ ID NO:2137
SEQUENCE LENGTH:302
SEQUENCE TYPE:nucleic acid
TOPOLOGY:linear
CLONE:HUMGS02443
SEQUENCE DESCRIPTION:
GATCCGAGAG GGCATGGCCG CCCTGCAGTC TGACCCCTGG CAGCAAGAGC TCTACCGCAA 60
CTTCAAAAGC ATCTCCAAGG CCTTGGTGGA GCGGGGTGGT GTGGTCACCA GCAACCCACT 120
TGGCTTCTGA ATACTCCCGG CCAGGGCCTG TCCCAGTTAT GTGCCTTCCC TCAAGCCAAA 180
GCCGAAGCCC CTTTCCTTAA GGCCCTGGTT TGTCCCGAAG GGCCCTAGTG TTCCCAGCAC 240
TGTGCCTGCT CTCAAGAGCA CTTACTGCCT CGNAAATAAT AAAAATTTCT AGCCAGGTCA 300
AA 302

SEQ ID NO:2138
SEQUENCE LENGTH:297
SEQUENCE TYPE:nucleic acid
TOPOLOGY:linear
CLONE:HUMGS02444
SEQUENCE DESCRIPTION:
GATCATCCAG AAGGAGCCCG TTGAAGTGGC CCCAAGCCAG CTCTCTGCAT TTCGTACTCT 60
CCTGTTTTCT GCACTTGGTG AAGAGGAGAA GATGATGGTG AACAACTATC GCCCACTTCA 120
ACCCTTGATG AACCGGAAGG TCTGGGCGTC CTTCCAGGCC ACTAATGAGG GCACAAGGTC 180
CTAGAGACAT TAGGTCCACA TGAATAGCAG AACTGACTTT GAAGGAAGGA AGCGTTGTTT 240
CCCAAGTTTC ACAATGTGAT TGTACATGAC TTCTGAAATT AAAAAGAGAG CATGAAA 297

SEQ ID NO:2139
SEQUENCE LENGTH:295
SEQUENCE TYPE:nucleic acid
TOPOLOGY:linear

CLONE:HUMGS02445
SEQUENCE DESCRIPTION:
GATCTATGCC AGCAAAACAT CATTTTGAGA CAAACATTTT TGTGGCAGAT GTTTTTCCTA 60
AAAAGTACTA TATCATCCAA GAAATATTTG AGTAAAATCC CTTGTTCTTT TGGGTGACAT 120
TAACTGACAT TTGCTTTTTT TCAAGACCTA ATAGAAAATA AGAAAGCCCA TAATGTATTT 180
AGAAACAGGA ATCCTCAGAG CAATTCTCTG TATTCTCATA TAATTTCAAT GTAAAACAGA 240
AAACATATTG ATGTGTTGGT GATAGGCTTG AATTATTAAA AACTTCAAAA ACAAA 295

SEQ ID NO:2140
SEQUENCE LENGTH:294
SEQUENCE TYPE:nucleic acid
TOPOLOGY:linear
CLONE:HUMGS02446
SEQUENCE DESCRIPTION:
GATCGAGGCT GCAGGATATG CTCAGACTCT AGAGGCGTGG ACCAAGGGGC ATGGAGCTTC 60
ACTCCTTGCT GGCCAGGGAG TTGGGGACTC AGAGGGACCA CTTGGGGCCA GCCAGACTGG 120
CCTCAATGGC GGACTCAGTC ACATTGACTG ACGGGGACCA GGGCTTGTTT GGGTCGAGAG 180
CGCCCTCATG GTGCTGGTNC TGTTGTGTGT AGGTCCCCTG GGGACACAAG CAGGCGCCAA 240
TGGTATCTGG GCGGANTCAC AGAGTTCTTG GAATAAAAGC AACCTCAGAN CAAA 294

SEQ ID NO:2141
SEQUENCE LENGTH:292
SEQUENCE TYPE:nucleic acid
TOPOLOGY:linear
CLONE:HUMGS02447
SEQUENCE DESCRIPTION:
GATCTGGTTG GTGGATGAAC AAGTGTCACG CTGGACATCT CAATGGNGTT TATTACCAAG 60
GTGGCACTTA CTCANANGCA TCTACTCCTA ATGGTTATGA TAATGGCATT ATTTGGGCCA 120
CTTGGANAAC CCGGTGGTAT TCCATGAAGA ANCCACTATG AAGATAATCC CATCAACAGA 180
CTCACAATTG GAGATGACAG CAACACCACC TGGGGGGAGC ANACAGGTGG AGACGTTAAA 240
NGCCGTTCAN TGNGTTTTCT NTTTAAAGNC TTTATCTGAC CNGGGGTANN GN 292

SEQ ID NO:2142
SEQUENCE LENGTH:280
SEQUENCE TYPE:nucleic acid
TOPOLOGY:linear
CLONE:HUMGS02448
SEQUENCE DESCRIPTION:
GATCGGAAAA AAATTCTTGA ACGCAAAGCC AAGTCTCGAC AAGTTGGAAA AGAGAAAGGC 60
AAATATAAAG AAGAACTTAT TGAGAAAATG CAGGAATAAA TAGAACCTGT TGTGCAACCA 120
CGGTTTAACC GGAGATTTTG AGGCTAGGGT GTGTTTCTTT CGAACTTTTC GGAATGTCTG 180
GAACATTTCA TTTCCTGTTT TGTTACCTGT GCCTCTGTAA ATCTACTTTT GCAATTTTAA 240
GTAATAATTT TATGAATAAN AATGGGAAAT GCTTCCTAAA 280

SEQ ID NO:2143

SEQUENCE LENGTH:280
SEQUENCE TYPE:nucleic acid
TOPOLOGY:linear
CLONE:HUMGS02449
SEQUENCE DESCRIPTION:
GATCCCATAA CAGGCTACCC CTTGGCCTCA TGCTGGAGTT GTGTGTGTCT GTCTTCATCC 60
CAGGCTGAGC TCCTTGAGGT GAGGATGTTG TGCTGTTTGC CTCCCTCACA GTGCCTTGGT 120
CTTAGTGGAT GCCCAGTTGT CTTGTGAATG ACTTTTAAGA AGTGTACTTA AGAGAAAAAT 180
CCTACCNTAT NNNNNGTAAT TACAAGTCAT GTTTTTGTNG CTTAAAGGTG ATAAATCAGT 240
GTATATNATN NGNTAATGTC CATTAAAGCC AGTTTTTAAA 280

SEQ ID NO:2144
SEQUENCE LENGTH:278
SEQUENCE TYPE:nucleic acid
TOPOLOGY:linear
CLONE:HUMGS02450
SEQUENCE DESCRIPTION:
GATCCTAGAG CCTGACGTTC AGTGAACCCA TGTTTCTGGG TGGGTGAAAG GGGCCCAACC 60
CTGCCCACTT CAGCCCAGCC CGCCCAAGGG GACTTGTGCC AGCAGAACAT GTNGGAGGAA 120
GAAGCCCCGT TTCCAGGGCA TCCGCAGCCC AGGGTAGGGA GAAATATTCT CTCCACTTTG 180
GGGGAGAGTT CTTGCTCTCG ACCTAGTGGT TTCTACTCTC ACCGACTTAT TCTGATTTCA 240
GAAATAAAAT GAAATGTCTT ATTTTTGGGA AAGTTAAA 278

SEQ ID NO:2145
SEQUENCE LENGTH:315
SEQUENCE TYPE:nucleic acid
TOPOLOGY:linear
CLONE:HUMGS02452
SEQUENCE DESCRIPTION:
GATCCCACTG GGGCCTNTGG GAAGGNGACA GACTTATNAC TAGATGATTC GCTGGTGTCC 60
ATCTTTGGGA ATCGACGTCT CAAAAGGTTA TCCATGGTGG TACAGGATGG CATAGTGAAG 120
GCCCTGAATG TGGAACCAGA TGGCACAGGC CTCACCTGCA GCCTGGCACC CAATATCATC 180
TCACAGNTCT GAGGCCCTGG GCCAGATTAC TTCCTCCACC NNTCCNTATC TNACCTGCCC 240
AGCCCTGTGC TGGGGCCCTG CAATTGGAAT GTTGGCCAGA TTTCTGCAAT AAACACTTGT 300
GGTTTGCGGG TCAAA 315

SEQ ID NO:2146
SEQUENCE LENGTH:275
SEQUENCE TYPE:nucleic acid
TOPOLOGY:linear
CLONE:HUMGS02453
SEQUENCE DESCRIPTION:
GATCCCAGGA CCATTTGGCT AGTGTGCCTA GGTGACCACA TGATTGCTGT TTTACCAGGA 60
ATGCAGCATC CCATTGACAA AACAAGTGCT CTGAGAAGGT TTAAAATACT ACAGNGAATA 120
TGGGANCACA GNCCTTGAAA TTTAGCTGAG TTGTAACAGC TGAAACTCCA AGAGGTGTCT 180

TCCTTGTTTN GAGGTGNACT AGTGTTGCTT CCAGAGGGCA GCTTGAAACC CGTAAAGCCT 240
GTTTTGGGGA ATCCTTTTTT GNCTTGCTTT GNTTN 275

SEQ ID NO:2147
SEQUENCE LENGTH:274
SEQUENCE TYPE:nucleic acid
TOPOLOGY:linear
CLONE:HUMGS02454
SEQUENCE DESCRIPTION:
GATCATCAAT GATTTATTGG CACGTTCTAT TGGGATGGTC GAAGTTAATA CAGTAGACAA 60
ATACCAAGAT AAGGGACAAA AGTATTGTCC TAGTATCTTT TGTTAGAAGT AATAAGGATG 120
GAACTGTTGG TGAACTCTTG AAAGATTGGC GACGTCTTAA TGTTGCTATA ACCAGAGCCA 180
AACATAAACT GATTCTTCTG GGGTGTGTGC CCTCACTAAA TTGCTATCCT CCTTTGGAGA 240
AGCTGCTTAA TCATTTAAAC TCAGAAAAAT TAAA 274

SEQ ID NO:2148
SEQUENCE LENGTH:266
SEQUENCE TYPE:nucleic acid
TOPOLOGY:linear
CLONE:HUMGS02455
SEQUENCE DESCRIPTION:
GATCGCAGAG AAGGCAGCTG ACATTATCAA GGGGCAGCCT GCACTCTGGG ACAAAGATGT 60
CCCTGTCTAC AAGCCCAGGA CGCTGGCCAC CCAGCGCTAA GACAGTTGCT GCTGGAGGAT 120
GACCAGGGAA GCCCCCTGAT AAGCCAAGAG GGCCAGCACA GCCCTTGCTC CCAGGCTCCT 180
GCCTGAAACT ATCTAGCACA CTAGGACCCA GGTGGTACCC TACTCAGTGG CTGAGAATTG 240
GATAAAGTCT TGGGAAATGA GACAAA 266

SEQ ID NO:2149
SEQUENCE LENGTH:265
SEQUENCE TYPE:nucleic acid
TOPOLOGY:linear
CLONE:HUMGS02456
SEQUENCE DESCRIPTION:
GATCTTTAAC AAATGTTTTA AGCAATTTTA AAAAGGCAGG ATGTTATTGA CATTATACAC 60
TGAAGTCTTA ACATTTTAAC ATTTATAGTG CTTATTTGCA AAATTGTATA ATTAGGAATT 120
ATTTCAGAGA CAATGTTTTC TTTTTCAGGT GAGTAGTTGC CGCGTAATAT CATTGGAGTA 180
CATTCTTTAT ACTGTTTGTG AAATTAATAC TAGCATATTA AGTGTACAAA TAGATTTAGA 240
AAACAATAAA AAATTGCATG CTAAA 265

SEQ ID NO:2150
SEQUENCE LENGTH:264
SEQUENCE TYPE:nucleic acid
TOPOLOGY:linear
CLONE:HUMGS02457
SEQUENCE DESCRIPTION:

GATCCTAGTG TTTTAATTAC CTGTGTGCAG CTATTTTAAA TAGCATTTTA CTTGAATAAT 60
ATGTATGTTG TCATTGTTTC ATGCTATACT TTGTGGGATA AAACTTGGGA ATGAGTGTGG 120
TAAGAAATTT ATAAAGTTTT GCTTTTAAAA CGTGGACATA ACTCATTTTT CTAGTTTTTG 180
ACAATTGTGT GTTTTAGTGT CTAGTCTGCA GAGAGCTGTG TGATTAATAA ACGTGGAATT 240
AACAGAATTT CCTCTCCCTG TAAA 264

SEQ ID NO:2151
SEQUENCE LENGTH:259
SEQUENCE TYPE:nucleic acid
TOPOLOGY:linear
CLONE:HUMGS02458
SEQUENCE DESCRIPTION:
GATCCTGGGC TCATAGGCAG TCCCTTTCAC TTCCTTGTCT TGCTCCCTGC TATGCTGGAG 60
ATGAATGTGA CTAAAAGGGC CATCTTGCTG GCTTAATGTG TGGCTGGAGA GACCAGCCTG 120
GAGACAATGT GGCAAAATGG GGCGCTTCAT CCAGTCTGTC TAAGCCCTGT CGACTTGGGG 180
AGGTGATTTC TTTCCTGGTT CTATATGTNA AGCAAAATAA ATGTTTTAAA ATTAAAAGCA 240
NNAAAGCAGA ATGTGAGTN 259

SEQ ID NO:2152
SEQUENCE LENGTH:255
SEQUENCE TYPE:nucleic acid
TOPOLOGY:linear
CLONE:HUMGS02459
SEQUENCE DESCRIPTION:
GATCAAAGCT GGACTGGAAA TTGTATCGTG TAATTATTTT TGTGTTCTTA ATGTTATTTG 60
GTACTCAAGT TGTAAATAAC GTCTACTACT GTTTATNCCA GTGTCTACTA CCTCAGGTGT 120
CCTATAGATT TTCCTTCTAC CAAAGTTCAC TTTCACANTG AAATTATATT TGCTGTGTGA 180
CTATGATTCC TAAGATTTCC AGGGCTTAAG GGCTAACTTC TATTAGCACC TTACTGTGTA 240
AGCAAATGTT ACAAA 255

SEQ ID NO:2153
SEQUENCE LENGTH:248
SEQUENCE TYPE:nucleic acid
TOPOLOGY:linear
CLONE:HUMGS02461
SEQUENCE DESCRIPTION:
GATCTGTGCA GCAAAGCACC ATGGTACATG TNNTACCTAT GTAACAAACC TGCACATCCT 60
GCACATGTAC CCTGGAACTT AATAAAAGTT GGAAATTTTT AAAAAGAATG AATAAGACCT 120
GGTATTTGAT AGCACAACAG GGAGACTATA GTCAACAGCA ATTTAATTGT ATATTTTAAT 180
ATGACTAAAA GAGTATAATG GNTTGTTTGT AACACAANTA AATGCTTGAG GGGATGGNCA 240
CCCCNAAA 248

SEQ ID NO:2154
SEQUENCE LENGTH:245
SEQUENCE TYPE:nucleic acid

TOPOLOGY:linear
CLONE:HUMGS02462
SEQUENCE DESCRIPTION:
GATCTTAAGC TTTGATAAGA GCTGTCCTGT GGCTGAGTAT GGTGTGTATG TNAAGGTGCT 60
TCCATCCAGG CTGGGTTCAG AAGCCATAGC TGNGACTAAT GCAAGCTGGC CGAAGCCTTG 120
CTGAAAGAAG CTTTAGCTGG AGAGGCAAAG TGGCGGGAGT GGCAGGAGTG GATNNGATAA 180
GATGTGGTTT AAGTNATGGG TNCAGCCTGA TTCTGAGCAA TAATAAAGAN TTTCTTTTGC 240
CCAAA 245

SEQ ID NO:2155
SEQUENCE LENGTH:244
SEQUENCE TYPE:nucleic acid
TOPOLOGY:linear
CLONE:HUMGS02463
SEQUENCE DESCRIPTION:
GATCCAGGAA GCTCCCAAAC CAGAGTGTGA GAAGGCACTT CTTGCCTTTC AGGAGAGTAA 60
GAACCTCTGC GAANCATGGA GAACTTTATG CAACAATTAA AGGAAAGTGG CATGACAATG 120
GAGGAGCTAA AATATTCTCT GGAGCTGAAG AAAGCTGAGT TGAAGGCAAA ATTGTTGTAA 180
CACTACAGCT GAGCAGATGT AATAGAAATA AACCTATGAA TAAATTTNCT TCTTGGTTCT 240
GAAA 244

SEQ ID NO:2156
SEQUENCE LENGTH:237
SEQUENCE TYPE:nucleic acid
TOPOLOGY:linear
CLONE:HUMGS02464
SEQUENCE DESCRIPTION:
GATCCTATAT CTTGGTNAAT ATGCTCCCAG ATACTTTAAA CATGGCAACC TTTTGGCCTA 60
AGAGAATGTT TGTTCATGGA AAAAAGCTTT TGAGATGAGA GGGTGTCTTA CTTTCTTGTG 120
GCAATTGATT TTCTGTTTTA ACACCCTTTG GGTAAAATCT TGCAAAGAGC TTTTATAATT 180
NGTTTTACTG AATTGTATGG AGATTGTATA CCAAGTAAAG CTCTTTTAAA TTACAAA 237

SEQ ID NO:2157
SEQUENCE LENGTH:234
SEQUENCE TYPE:nucleic acid
TOPOLOGY:linear
CLONE:HUMGS02465
SEQUENCE DESCRIPTION:
GATCCACGCT GGGCCGAGGA TGGGACGTTT TTCTTCTTGG GCCCGGTCCA CAGGTCCANT 60
GACACCCTCC CTCCAGGGTC CTCTCTTCCA CAGTGGCGGG CCCACTCAGC CCCGAGACCA 120
CCCAACCTCA CCCTCCTGAC CCCCATGTAA ATATTGTTCT GCTGTCTGGG ACTCCTGTCT 180
AGGTGCCCCT GATGATGGGA TGCNCTTTAA ATAATAAAGA TGGTTTTGAT TAAA 234

SEQ ID NO:2158
SEQUENCE LENGTH:233

SEQUENCE TYPE:nucleic acid
TOPOLOGY:linear
CLONE:HUMGS02466
SEQUENCE DESCRIPTION:
GATCCCTAAG CTGTAGAACA TTTTAACGTT GATGCCACAA CGCAGATTGA TGCCTTGTAG 60
ATGGAGCTTG CAGATGGAGC CCCGTGACCT CTCACCTACC CACCTGTTTG CCTGCCTTCT 120
TGTGCGTTTC TCGGAGAAGT TCTTAGCCTG ATGAAATAAC TTGGGGCGTT GAAGAGCTGT 180
TTAATTTTAA ATGCCTTAGA CTGGGGATAT ATTAGAGGAA GCAGATTGTC AAA 233

SEQ ID NO:2159
SEQUENCE LENGTH:229
SEQUENCE TYPE:nucleic acid
TOPOLOGY:linear
CLONE:HUMGS02467
SEQUENCE DESCRIPTION:
GATCTGTGCA CACTTTCTAA ACAGCTTGTN ATGCAAGTNT GAGCCTATTG TGTTACTTGA 60
CCTTATTTTG GAAGTTTTGA ATTGGCCTAG GAGGAAACCC AGAAATGAAC CAGGGGTATG 120
TNATCACTTT TTTNATATCA AGTCCTCACC CTCCTNCCAC ATAATGCTCT ATCCGGCTAA 180
GGTTGGAACT CTGAAGTTGG AGAAGGTGGA ATAAAGTTAC ACCTGGAAA 229

SEQ ID NO:2160
SEQUENCE LENGTH:227
SEQUENCE TYPE:nucleic acid
TOPOLOGY:linear
CLONE:HUMGS02468
SEQUENCE DESCRIPTION:
GATCCCTGCG AATTACGAAA ACTACTACAT CCATGATTGC CCCAACCCCC TTGGGGTGGG 60
GCAGAAGGTG AAGCATCCCA ACTGACTAGA GACTCAGGCC CTGCAGGNCC TTATAGAACC 120
TGTGGCCATC CCTGAGCACC CTGCCACCAG GTGACCTCGG CCATACTCAC TGCCCCCCTT 180
GGGCACCCAC TCACCNTAGA ATAAACTTAA CAGTGTCCCG TGGTAAA 227

SEQ ID NO:2161
SEQUENCE LENGTH:226
SEQUENCE TYPE:nucleic acid
TOPOLOGY:linear
CLONE:HUMGS02469
SEQUENCE DESCRIPTION:
GATCTTACGA TGCCCTCTGT ACTGACCTGA AGGAGACCTA AGAGTCCTTT CCCTTTTTGA 60
GTTTGAATCA TAGCCTTGAT GTGGTCTCTT GTTTTATGTC CTTGTTCCTA ATGTAAAAGT 120
GCTTAACTGC TTCTTGGTTG TATTGGGTAG CATTGGGATA AGATTTTAAC TGGGTATTCT 180
TGAATTGCTT TTACAATAAA CCAATTTTAT AATCTTTAAA TTTAAA 226

SEQ ID NO:2162
SEQUENCE LENGTH:224
SEQUENCE TYPE:nucleic acid

TOPOLOGY:linear
CLONE:HUMGS02471
SEQUENCE DESCRIPTION:
GATCTGCCTT GCTCACTATT TGCCTATTGG CATATATGAT TACTTTGCTA AAAGACATTT 60
TGGCCAAGAC AAGCCCATNT CCAGAGCTCT AAGAATGCCT AACTACAAGA AAAAGGCCAC 120
CTAGGCAATG GAAGCCCTCA AAGAAGTCGG AATGTCATAG TCTTGAAATG AAAGGGAAAC 180
TGGGAAACTG GGTTTCTCAT TAAAGTTGTT TCCCACTCTN TAAA 224

SEQ ID NO:2163
SEQUENCE LENGTH:221
SEQUENCE TYPE:nucleic acid
TOPOLOGY:linear
CLONE:HUMGS02472
SEQUENCE DESCRIPTION:
GATCTAATTT GATAAACAAC ATGGCTTGTG TGAAAACTGA GCAGGTGTTT GTTTACCCAT 60
AGTGTTCTGT GTAGTTATTG CTTANTCTGC AGAAAATAAT GACTTAGATG AGATGTCTGA 120
CTTGCTTTCA CTTATTAAAC ATGTTCACCA TGGGATGATG TCTGTAACAT CAGATATTGT 180
TCAACTAGAC TAGGATTTAA TAAAAATTGT GAAAGCTTAA A 221

SEQ ID NO:2164
SEQUENCE LENGTH:210
SEQUENCE TYPE:nucleic acid
TOPOLOGY:linear
CLONE:HUMGS02473
SEQUENCE DESCRIPTION:
GATCTTAATT TACAGTTCCA TGGGATTAAA ACAAGTTTGC ATTTATGTAA TTTGCATTAC 60
TTTAAAAATT ACAACAACAT AATCTCTGAA ACTTACTTCA ATCAATTCAC ATCCCTACAA 120
CTGTTAAGAT TTATTTTGTG ATTAGNATCT TATAGTGGGT ATTCTTTTNA GTGCTGATAT 180
TTCTATTCAT TAAAATCCTG TGNACACAAA 210

SEQ ID NO:2165
SEQUENCE LENGTH:209
SEQUENCE TYPE:nucleic acid
TOPOLOGY:linear
CLONE:HUMGS02474
SEQUENCE DESCRIPTION:
GATCCTGCAT TAAATTTTTA TTTGTGAAAA TTTGAAAATT ATGAGCTATA ACTTATAAAG 60
TTTGCCTTCT TATGNCTAAA ATCTTTTAAA GGATTATTTG CATTTATGTA AAACTGGAAA 120
TGAAGTTATA TATGAGTGCA AAGAAATTGA CTTCTGCTCA AATATGTGTT GATACCCTAC 180
AGTAAATTGA ATTAAATTTT TATCCCAAA 209

SEQ ID NO:2166
SEQUENCE LENGTH:203
SEQUENCE TYPE:nucleic acid
TOPOLOGY:linear

CLONE:HUMGS02475
SEQUENCE DESCRIPTION:
GATCAGTCAA TTTTCCGTCG TCCGTAACCA GCGGGCCCCT GGTCAAGTNC TGGCTCTGCT 60
GTCCTTGCCT TCCATTTCCC CTCTGCACCC AGAACAGTGG TGGCAACATT CATTGCCAAG 120
GGCCCAAAGA AAGAGCTACC TGGACCTTTT GTTTTCTGTT TGACAACATG TTTAATAAAT 180
AAAAATGTCT TGATATCAGT AAA 203

SEQ ID NO:2167
SEQUENCE LENGTH:200
SEQUENCE TYPE:nucleic acid
TOPOLOGY:linear
CLONE:HUMGS02476
SEQUENCE DESCRIPTION:
GATCTGGACT TCGAAACTTT CCTCATGGAT TCTGAATAAA AGAAACACTC TACACCTCTT 60
AAATCAAGGT CATGTAGATA ATGACTTTGT TATAAATGCT ACAGCTGTGA GAGCTTGTTT 120
CTGATTTCAT TGTTCGCTTC TGTAATTCTG AAAAACTTTA AACTGGTAGA ACTTGGAATA 180
AATAATTNGT TTTAATTAAA 200

SEQ ID NO:2168
SEQUENCE LENGTH:200
SEQUENCE TYPE:nucleic acid
TOPOLOGY:linear
CLONE:HUMGS02477
SEQUENCE DESCRIPTION:
GATCTTAAGT GTCTCCTTGC TCTGGTAAAA GATAGATTTG TAGCTCACTT GATGATGGTG 60
CTGGTGAATT GCTCTGCTCT GTCTGAGATT TTTAAAAATC AGCTTAATGA GAGTAATCTG 120
CAGACAATTG ATAATAACAT TTTGAAAATT GGAAAGATGG TATACTGTTT TTAGAGGAAT 180
AAACGTATTT GTGGTTTAAA 200

SEQ ID NO:2169
SEQUENCE LENGTH:197
SEQUENCE TYPE:nucleic acid
TOPOLOGY:linear
CLONE:HUMGS02478
SEQUENCE DESCRIPTION:
GATCCAAATC CCAGCTCTAC TGTCTCGTCC GAATTAGCCT TGGGAAAATC ATTTATATGC 60
TAAATAATTT ACCTTTTTAT CTAGGAGATG AAAAGAGGAT AATGTTTCCT TCCATAAAGA 120
AAGTTCTTGT AAGAATCAAA AGAAATGGTG AGCTTTAAGT GGTTTGTAAA CCATAAAACA 180
CATCATAAAA GTTCAAA 197

SEQ ID NO:2170
SEQUENCE LENGTH:201
SEQUENCE TYPE:nucleic acid
TOPOLOGY:linear
CLONE:HUMGS02479

SEQUENCE DESCRIPTION:
GATCCATGNC ATCTCTCTCT TGCCTGAGGG GAAAGAGAGA TGGGCCAGGC AGAGAACAGA 60
ACTGGAGGCA GTCCATCTAG GGAANNNNAC TGTGAGGCCA TACTTGTGAA ACGTCTGGAC 120
TGCTATTCTA GAGCTTTTAT TTGNGTGTGT TCGTTGCACA NCTTGTTTTA AATGTTTAAT 180
AAAAGCTTTA TAAACTTTAA A 201

SEQ ID NO:2171
SEQUENCE LENGTH:218
SEQUENCE TYPE:nucleic acid
TOPOLOGY:linear
CLONE:HUMGS02480
SEQUENCE DESCRIPTION:
GATCCCCCAA GTACAGGCGC TAATTNTTGT GATAATTTGT AATNGTGACT TGTACTCCCC 60
GGCTGGCAGC GTAGTGGGGC TGCCAGGCCC CAGCTTTGTT CCCTGGTCCC CCTGAAGCCT 120
GCAAACGTTG TCATTGGCAG GGAAGGGTGG GGGGCAGCTG CGGTGGGGNG CTATAAAANT 180
GACAATTAAA CGNGACANTN GTNTTTTATT TCCTNAAA 218

SEQ ID NO:2172
SEQUENCE LENGTH:195
SEQUENCE TYPE:nucleic acid
TOPOLOGY:linear
CLONE:HUMGS02481
SEQUENCE DESCRIPTION:
GATCCTTGCA GGGAGCTTGG AACCTTAGTG CACCTACTTC AGTTCAGAAC ACTTAGCACC 60
CCACTGACTC CACTGACAAT TGACTAAAAG ATGCAGGTGC TCGTATCTCG ACATTCATTC 120
CCACCCCCNT CTTATTTAAA TAGCTACCAA AGTACTTCTT TTTTAATAAA AAAATAAAGA 180
TTTTTATTAG GTAAA 195

SEQ ID NO:2173
SEQUENCE LENGTH:194
SEQUENCE TYPE:nucleic acid
TOPOLOGY:linear
CLONE:HUMGS02482
SEQUENCE DESCRIPTION:
GATCCGGCAA CATCAACCCG AGTCATTCGT TCTGTGGAGG GACAAGTGGA CTCAGGGCAG 60
CGCAGGCTGA CCACAGCACA GCCAACACGC ACCTGCCTCA GGACTGCGAC GAAACCGGTG 120
GGGCTGGTTC TGTAATTGTG TGTGATGTGA AGCCAATTCA GACAGGCAAA TAAAAGTGAC 180
CTTTTACACT GAAA 194

SEQ ID NO:2174
SEQUENCE LENGTH:192
SEQUENCE TYPE:nucleic acid
TOPOLOGY:linear
CLONE:HUMGS02483
SEQUENCE DESCRIPTION:

GATCAAATGG AAAGGAGAGG AAAGAACTCA GTGCTGCCTA TTAGTAGTTA ATTCTGTCAC 60
TCACCACTAC ATCACTTGAG ACAAATCTAT GCCACTCAGA ATCTCCTTCT TTCCTGGACT 120
TAACTCTAAT TCTAGAGTCT CTGTTACTGC TTGGGCTATA CCTGGGCATA CTAATAAAGT 180
ATGGTATTGA AA 192

SEQ ID NO:2175
SEQUENCE LENGTH:192
SEQUENCE TYPE:nucleic acid
TOPOLOGY:linear
CLONE:HUMGS02484
SEQUENCE DESCRIPTION:
GATCAGAACT GTTACCAAAA AACAACTGTC AGTTTTATTG AGATGGGAAA AATGTAAACT 60
CGANNNTTAT TACTTAAGAC TTTATGGGAG AGATTAGACA CTGGAGGTTT TTAACAGAAC 120
GTGTATTTAT TAATGTTCAA AACACTGGAA TTACAAATGA GAAGAGTCTA CAATAAATTA 180
AGATTTTTGA AA 192

SEQ ID NO:2176
SEQUENCE LENGTH:187
SEQUENCE TYPE:nucleic acid
TOPOLOGY:linear
CLONE:HUMGS02486
SEQUENCE DESCRIPTION:
GATCGTCTTG GAAGGGAGTG GTTTTCAGAG ACATTTCAGA AAGTGAAGGA GAAACTCAAG 60
ATTGACTCAT GAGGACCTGA AGGGTGACAT CCCAGGAGGG GCCTCTGAAA TTTCCCACAC 120
CCCAGCGCCT GTNCTGAGGA CTCCCTCCAT GTGGCCCCAG GTGCCACCAA TAAAAATCCT 180
ACAGAAA 187

SEQ ID NO:2177
SEQUENCE LENGTH:187
SEQUENCE TYPE:nucleic acid
TOPOLOGY:linear
CLONE:HUMGS02487
SEQUENCE DESCRIPTION:
GATCATTGAA ATGCTGAAAA TTTTAACAGT CTTCTTAAAA GTATTGAGGG GGCAAAAATT 60
ACCAATTATG GTATACAAAA ATAAGCCTAT AAATGTNTTT CACATTGCTA ACTTGAGTTT 120
CAGTTGATTC AGTTTGTAAT AACTAGTAAT GAGCTTCTGT TTACAATAAA AATNCTGTAA 180
ATTGAAA 187

SEQ ID NO:2178
SEQUENCE LENGTH:186
SEQUENCE TYPE:nucleic acid
TOPOLOGY:linear
CLONE:HUMGS02488
SEQUENCE DESCRIPTION:
GATCAGTTGC TTATAGATAA TGTTCAATGA CCTCAAGACA TATATTTTTG AGAAATTATC 60

ATTTTAAAAA ATTTGGTCTA TACTGATTGT TTTCACTGAT TCCAATATTA TTACTTATAA 120
CACTGACCTC TGGAAAATAT TTTGTNCACA AGAAATAATA AAGTATAATG ATTTGTNGCA 180
NNNAAA 186

SEQ ID NO:2179
SEQUENCE LENGTH:179
SEQUENCE TYPE:nucleic acid
TOPOLOGY:linear
CLONE:HUMGS02489
SEQUENCE DESCRIPTION:
GATCCACAAG GCCTGAGGAG CAGTGTGGGG GGACAGACAG GAGGTGGATT TGGAGACCGA 60
AGACTGGGAT GCCTGTCTTG AGTAGACTTG GACCCAAAAA ATCATCTCAC CTTGAGCCCA 120
CCCCCACCNC NTTGTCTAAT CTGTAGAAGC TAATAAATAA TCATCCNTNC TTGCCTAAA 179

SEQ ID NO:2180
SEQUENCE LENGTH:179
SEQUENCE TYPE:nucleic acid
TOPOLOGY:linear
CLONE:HUMGS02490
SEQUENCE DESCRIPTION:
GATCCAATGG AGCCTGGNGA GGTGTGCCCA GAAAGCTTGT CTGTAGCGGG TTTTNTGAGA 60
GTNAACACTT TCCACTTTTT GACACCTTAT CCTGATGTAT GGTTCCAGGA TTTGGATTTT 120
AATTTTCCAA ATGTAGCTTG AAATTTCAAT AAACTTTGCT CTGTTTTTCT AAAAATAAA 179

SEQ ID NO:2181
SEQUENCE LENGTH:177
SEQUENCE TYPE:nucleic acid
TOPOLOGY:linear
CLONE:HUMGS02491
SEQUENCE DESCRIPTION:
GATCTGTCCA CTCCTGGTCA TTGGTGGATG TTAAACCCAT ATTCCTTTCA ACTGCTGCCT 60
GCTAGGGAAA ACTGCTCCTC ATTATCATCA CTATTATTGC TCACCACTGT ATCCCCTCTA 120
CTGGGCAAGT GCTTGTCAAG TTCTAGTTGT TCAATAAATT TGTTAATAAT GCTGAAA 177

SEQ ID NO:2182
SEQUENCE LENGTH:176
SEQUENCE TYPE:nucleic acid
TOPOLOGY:linear
CLONE:HUMGS02493
SEQUENCE DESCRIPTION:
GATCAAAAAA CCACAGGGCT TTTGGGCACT GCCTCCTTGG GAAGTTAGTG GCCACAGAAG 60
AGAGATGAAA CCTGTAAGAA GTCTGGAGTC TTTTGGAACT TCAGCCATTT CCCCAGGTTG 120
TTACTTTCTT AGTATGTACA GTCTTCTCAG GATGAGCAGT AAAACCTTTG AACAAA 176

SEQ ID NO:2183

SEQUENCE LENGTH:175
SEQUENCE TYPE:nucleic acid
TOPOLOGY:linear
CLONE:HUMGS02494
SEQUENCE DESCRIPTION:
GATCCTAAAC TCTCTAGCTT AAGTGTAAAT GAAGTACAGT AGTTTCCCTA CTGAACCCTA 60
CCTCTTGTGT CCCTGGAACC TTCTAGAACA CCTGCCTTCT ACCCTCTGGT TGGGAGATGC 120
AGCCACCACA TCCCTTCATA TCATACTGTT TTGAATAAAT TTTCAAATCC TTAAA 175

SEQ ID NO:2184
SEQUENCE LENGTH:166
SEQUENCE TYPE:nucleic acid
TOPOLOGY:linear
CLONE:HUMGS02495
SEQUENCE DESCRIPTION:
GATCCGGAGG GCCCAGGAGA ACCGGAGCGT GCTTCAGGGT GCCCGCAGAA CAGGAGCTGC 60
TCAGCCAAGA ACTGTGGCGG CGGCTGCTGC CAGGATGCCG AAGATACCCC ACTAGCACCC 120
CTNAGGGGGT CATGTGGTCA ATAAAAGTCC TTAGCTGCTG CCTAAA 166

SEQ ID NO:2185
SEQUENCE LENGTH:164
SEQUENCE TYPE:nucleic acid
TOPOLOGY:linear
CLONE:HUMGS02496
SEQUENCE DESCRIPTION:
GATCAAAATC AAGAAATCCG GGCATCACAG AGAAGTTGGG TTTAGGACAG CAGGTGCTGT 60
TCCGAGACTC AGTCCTAAAG GGTTTTTTTT CCCACTAAGC AAGGGGCCCT GACCTCGGGA 120
TGAGATAACA AATTGTAATA AAGTAACTTC TCTTTTCTTC TAAA 164

SEQ ID NO:2186
SEQUENCE LENGTH:164
SEQUENCE TYPE:nucleic acid
TOPOLOGY:linear
CLONE:HUMGS02497
SEQUENCE DESCRIPTION:
GATCAGCCCC TTAAAATNAG TTCTAGAGCA GGTCTTCTGT TCCAGAAGGG GAGAAGCATA 60
GAGGGCCTGT GAGCTCACGT GTGTTCTTTG TCATAGGGGT GAAAAACTAA CTTCAAGTGT 120
CCCTTGTTTG AAATAAACTT AGCAGAGTCA CTTTCTATCT TAAA 164

SEQ ID NO:2187
SEQUENCE LENGTH:163
SEQUENCE TYPE:nucleic acid
TOPOLOGY:linear
CLONE:HUMGS02498
SEQUENCE DESCRIPTION:

GATCTAATGG TTTTATAAAT NAAAGTTCCC CTGGACAAGT TCTCTTGCCT GCCACCATGT 60
AAGATGTACC TTTGCTACTC ATTCACCTTC TGTCATGATG GTGAGGCCTT CCCAGCAATG 120
TGGAACTGTG AGTCCATTAA ACCTCTTTCC TTTATAAATT AAA 163

SEQ ID NO:2188
SEQUENCE LENGTH:162
SEQUENCE TYPE:nucleic acid
TOPOLOGY:linear
CLONE:HUMGS02499
SEQUENCE DESCRIPTION:
GATCCTAACC AGGTAACCTG GTCCTNTGAA TTGAAGCTTA TCATATACCC AAAGCCTTGA 60
CTAGAAATAC CTAAATAGAA TACCATGGTC ATTGGAAAAA ATAGATAGTN TTNTAAAGTA 120
TTATTTNAAT AAATCCTAAA GTNTGTCTNT CTTCTCCTCA AA 162

SEQ ID NO:2189
SEQUENCE LENGTH:154
SEQUENCE TYPE:nucleic acid
TOPOLOGY:linear
CLONE:HUMGS02500
SEQUENCE DESCRIPTION:
GATCCTGGCC ATGAATGAAA AGGATAAGAA GAAAGAGAAG AAATGAAGTG ACCATCCAGC 60
CTTTCCCAAT NAGACTTCCT CTCCTTCCAC CCCTCATTTC CTTTTTGCAC ACATTACAGG 120
TGGTGTGTTC TGTGATAATG AAAAGCATCA GAAA 154

SEQ ID NO:2190
SEQUENCE LENGTH:154
SEQUENCE TYPE:nucleic acid
TOPOLOGY:linear
CLONE:HUMGS02501
SEQUENCE DESCRIPTION:
GATCAAAGCC AGCNACCCCC ACCCCAACAC ACTCGGTGTC CCANTCATAC TAGGGCCTGT 60
GTAAATCCCA GCCTGGCCAT ACCCTCAACC TCAGTGGGCT GGAAATNACA GTGGGCCCTA 120
TAGCAGTGGC AGAATAAACT CAGNTGTGTT CAAA 154

SEQ ID NO:2191
SEQUENCE LENGTH:153
SEQUENCE TYPE:nucleic acid
TOPOLOGY:linear
CLONE:HUMGS02502
SEQUENCE DESCRIPTION:
GATCGCCCCA CTGCAATCCA GCCTGGGAGA CATAATTCAA ATCTATTTTG GTCTTATATC 60
TTCTTATGTT TGTGTTATTT CATGTGTGCC AGTTTCTCTT TTTTTCACAA CAATTTATCA 120
ATTTGTGGCA ATAAAAATNA TTTACCTTTT AAA 153

SEQ ID NO:2192

SEQUENCE LENGTH:151
SEQUENCE TYPE:nucleic acid
TOPOLOGY:linear
CLONE:HUMGS02503
SEQUENCE DESCRIPTION:
GATCATGGTG ATTTTACTGC TGCTTGTGGC TATCGTGGTT GTTGCAGTCT GGCCGACCAC 60
CAGCAGTNAC ACCTGCCAAT NACAGATGCA AGCCCAACAC CCTTTTGGTA CGCAAAACCT 120
GCTCTCAATA AATTCCCCCA AAGCTCTGAA A 151

SEQ ID NO:2193
SEQUENCE LENGTH:371
SEQUENCE TYPE:nucleic acid
TOPOLOGY:linear
CLONE:HUMGS02504
SEQUENCE DESCRIPTION:
GATCCCAGGA ACCTTCCTNC TCCAGGGCAG CACAGGACTC AGCCATGTNT GGACCGGCCC 60
TGCTGAGGCT ACAGTCACTC TGGAAGCTCT GCGCTTCATC AGGNGGCAGG ACTGTGGCGG 120
GAGGGGTCCT TGAAGATGGG TGTGGGGAGC AGTGGGTCAG GAAGTGGGAG CCAGAGGTTT 180
GACTCACTTT GCTTTATTTT TCAGGCTACA ATACAGGTCA GAGACAATGG CTTATAAAGG 240
TTTAGTGTGG TCTCAGGATG TGACAGGCAG TCCAGCCTGA CCTTTCTGCA CACTCCAGAC 300
AAACTTCCCA GACAAGCTCC TTTGTGCCTC TACGTGGAGA GGGTGTGGGA AAGTTATCAC 360
ATTAAAAGGT N 371

SEQ ID NO:2194
SEQUENCE LENGTH:147
SEQUENCE TYPE:nucleic acid
TOPOLOGY:linear
CLONE:HUMGS02505
SEQUENCE DESCRIPTION:
GATCAAGAAA GAGATGGAGG AGGAGGACTG AGCCCAGAAT TCACTAGGTT CGAATCCAGA 60
NAGCAGTGTG GAAAAAAAAA ACCAAAAANC AACTGNCCAG TTGTTGATAA CCACTAAGAG 120
TCTCTATTAA AATTACTGAT GCAGAAA 147

SEQ ID NO:2195
SEQUENCE LENGTH:145
SEQUENCE TYPE:nucleic acid
TOPOLOGY:linear
CLONE:HUMGS02506
SEQUENCE DESCRIPTION:
GATCTGTGTC ATTNCCCATG AGGGGCCGGG GCAGGTGGCT GGGTGGGGGC ACAGGCTGGA 60
GTATTCTNAG TTCTACTGGT TCTACACTGT GAGGTGGCAA TGGGATTTGC TCAGATGCCA 120
CCCAATAAAA TGCCTGTAAC TTAAA 145

SEQ ID NO:2196
SEQUENCE LENGTH:144

SEQUENCE TYPE:nucleic acid
TOPOLOGY:linear
CLONE:HUMGS02507
SEQUENCE DESCRIPTION:
GATCGCTACT CTTAAGAATA TACATGTATG TATTCATAGG AACATTTTTT CTCAATATTT 60
GTATGATTCG CTTACTGTTA TTGTGCTGAG TGAGCTCCTG TGTGCTTCAG ACAAAAATAA 120
ATGAGACTTT GTGTTTACGT TAAA 144

SEQ ID NO:2197
SEQUENCE LENGTH:143
SEQUENCE TYPE:nucleic acid
TOPOLOGY:linear
CLONE:HUMGS02508
SEQUENCE DESCRIPTION:
GATCAGCTGG AAGCTCACTA GTCATCTCCT GCCCAATCCC CAGAAACCCT GATTCAGGTC 60
TGCAGGCTCC TGCGGGCTCA CCACGGCTGC TGGCTCCGGT ACCATGTAAN NCCTAGGAAG 120
GTAAAGGNGC AGGCAACCTC AAA 143

SEQ ID NO:2198
SEQUENCE LENGTH:141
SEQUENCE TYPE:nucleic acid
TOPOLOGY:linear
CLONE:HUMGS02509
SEQUENCE DESCRIPTION:
GATCCAGTGA GATAGAATAC ATGTAAGTNT GGTTTTGTAA TTTAAAAAGT GCTATACTAA 60
GGGAAAGAAT TGAGGAATTA ACTGCATACG TTTTGGTGTT GCTTTTCAAA TGTTTGAAAA 120
TAAAAAAAAT GTTANGANAA A 141

SEQ ID NO:2199
SEQUENCE LENGTH:137
SEQUENCE TYPE:nucleic acid
TOPOLOGY:linear
CLONE:HUMGS02510
SEQUENCE DESCRIPTION:
GATCCCTTGT GGCTATGCAG ATACCTACCT CACAGAGTTG TTGTAGAAGA CTGGTGGTTT 60
GGTTCAAACC TTGTGATTAA AGAGTTTGTC AAGCATTTTA TTCTTTTGAA TAAAAGCAAC 120
ATATCNAAAA CATTAAA 137

SEQ ID NO:2200
SEQUENCE LENGTH:135
SEQUENCE TYPE:nucleic acid
TOPOLOGY:linear
CLONE:HUMGS02511
SEQUENCE DESCRIPTION:
GATCCCTGTT TAACTCCAAA TTACAGTCGG ACTTGGATAC ATCATTTGTA ACATTGTAGG 60

AAAGAAAAAA GTCTTGGTTN TGAAAAACGA TTTGCATTTG GGTAAAATAA AGTGACCATG 120
CTTTTGTTCT GTAAA 135

SEQ ID NO:2201
SEQUENCE LENGTH:135
SEQUENCE TYPE:nucleic acid
TOPOLOGY:linear
CLONE:HUMGS02512
SEQUENCE DESCRIPTION:
GATCCAGCTC AAGTGCCATC CTGCCAGTGG CCCCCAGACT GTGGGGTTGG GACGCCTGGC 60
CTCTGTGTCC TAGAAGGGAC CCTCCTGTGG TCTTTGTCTT GATTTTNCTT AATAAACGGT 120
GCTATCCCCG CCAAA 135

SEQ ID NO:2202
SEQUENCE LENGTH:133
SEQUENCE TYPE:nucleic acid
TOPOLOGY:linear
CLONE:HUMGS02513
SEQUENCE DESCRIPTION:
GATCACTACT GCTCTTGGAG TTCCTTCTCC TAAAGCTAAG GCCAGCCTCC ATCACAAATG 60
CCCATAAAAT CTGGCTCGTC TTTGATGAAC AATAAATATT TNTTGAACAA ATAAATAACT 120
GCCCTGCATT AAA 133

SEQ ID NO:2203
SEQUENCE LENGTH:138
SEQUENCE TYPE:nucleic acid
TOPOLOGY:linear
CLONE:HUMGS02514
SEQUENCE DESCRIPTION:
GATCCTCCCG CTGGCTTCCA GACAGACCTG GGATTTTGGC AGTCATGCCG GGTGATGGTG 60
TTCCTGCGGA GACCCTCAGT TGTCCTATTC CTTCCTAGCT TCCCTGCAAT AAAATCAAGC 120
TGCTTTTGTT GGAATAAA 138

SEQ ID NO:2204
SEQUENCE LENGTH:130
SEQUENCE TYPE:nucleic acid
TOPOLOGY:linear
CLONE:HUMGS02515
SEQUENCE DESCRIPTION:
GATCATTTCA CCTGATGTTT TTAAAGCATC CTAAGTACAG TAGAGTAGAA AACTNATTTC 60
TTTGTTAATT GTACACTGAA TAATGCCTTT AAAAAATCAA AATAAAATTA ACAAATAATG 120
GTGAATGAAA 130

SEQ ID NO:2205
SEQUENCE LENGTH:176

SEQUENCE TYPE:nucleic acid
TOPOLOGY:linear
CLONE:HUMGS02516
SEQUENCE DESCRIPTION:
GATCTCCTAT GTTACCAATG TGTATCGTCT CCTTCTCCCT AAAGTGTACT TAATCTTTGC 60
TTTCTTTGCA CAATGTCTTT GGTTGCAAGT CATAAGCCTG AGGCAAATAA AATTCCAGTA 120
ATTTCGAAGA ATGTGGTGTT GGTGCTTTCC TAATAAAGAA ATAATTTAGC TTGAAA 176

SEQ ID NO:2206
SEQUENCE LENGTH:130
SEQUENCE TYPE:nucleic acid
TOPOLOGY:linear
CLONE:HUMGS02517
SEQUENCE DESCRIPTION:
GATCATGTTA TGAATTTGTA ACATTCACAC AACACCTCAC TTTTNAATCT ATAAAAGAAT 60
TGCACGTATG ANAAACCTAT ATTTNAATAC TGCTGAAACA GACATGAAAT AAAGAATTTA 120
AAGAATGAAA 130

SEQ ID NO:2207
SEQUENCE LENGTH:128
SEQUENCE TYPE:nucleic acid
TOPOLOGY:linear
CLONE:HUMGS02518
SEQUENCE DESCRIPTION:
GATCTAGCTT CTGGCTCTGT GTGTTTATTG ACCAGTGGTG TGCCTTGAGA AACCAGAACT 60
CTCTGAATTT CAGTTTTGTC TCTNTNTAAA TGAAGAAGGT TGTAATAAAA AAAATCTCAA 120
GTTTCAAA 128

SEQ ID NO:2208
SEQUENCE LENGTH:349
SEQUENCE TYPE:nucleic acid
TOPOLOGY:linear
CLONE:HUMGS02519
SEQUENCE DESCRIPTION:
GATCCGTCAT GGNCAACGAG AAGCACAGCA AGAACATCAC CCAGCGCGGC ATACGTCGCC 60
AAGACCTCGA GAAATGCCCC CGAAGAGAAG GCGTCTGTAG GCCCTGGTTA TTGGCTCTCT 120
TCACTTTTGT NGTCTGTGGT TCTGCAATTT TCCAGATTAT TCAAAGTATC AGGATGGGCA 180
NNTGAAGTGA CTGACCTTAA NTGTTTCCAT TCTNCTNTGA ATTTAACTTA ACTCATTCCT 240
GATGTTTGAN CCCTGGTTNA ACAATTCAGT AAANATCTGC TAGAATNCTT NCTATCATNT 300
TCATGTGCAT TCAAGNTTCT CATNGCATCC ANGTTCTAGN CAACACAGN 349

SEQ ID NO:2209
SEQUENCE LENGTH:127
SEQUENCE TYPE:nucleic acid
TOPOLOGY:linear

CLONE:HUMGS02520
SEQUENCE DESCRIPTION:
GATCACCAGG CAGCGACCTC CGCNCACCCC AGCAGTCGCG AGCCTCGGCC CAGCCANAGG 60
AAGCGNTGCC GNCGGACCTG ACTGACTTCC CAGTGGAACT GCCTTCTTNG GCTGGGACAG 120
CCNTTTN 127

SEQ ID NO:2210
SEQUENCE LENGTH:126
SEQUENCE TYPE:nucleic acid
TOPOLOGY:linear
CLONE:HUMGS02521
SEQUENCE DESCRIPTION:
GATCTGGGCC ACTTCCCTCC TTCCAGTCAT GAGTAATCAT CAAGGAGCAA GTTGGAGTGT 60
TTCAGGTGTA TATTTTGTAG AACCCAAAAG ATTGGAGCCT TAACAATAAA CATCAGCACT 120
AAGAAA 126

SEQ ID NO:2211
SEQUENCE LENGTH:126
SEQUENCE TYPE:nucleic acid
TOPOLOGY:linear
CLONE:HUMGS02522
SEQUENCE DESCRIPTION:
GATCTTTCAT AAAGCAGAAT GGTACGTATC GGATTGTTTT AATGTTATAT ATTGGATTGT 60
ATTCGTTGTT ACAAAACCAA TATTCTATGG AGAATGAAAA AAATAAAGGA CTAAATTAAC 120
TGGAAA 126

SEQ ID NO:2212
SEQUENCE LENGTH:127
SEQUENCE TYPE:nucleic acid
TOPOLOGY:linear
CLONE:HUMGS02523
SEQUENCE DESCRIPTION:
GATCTCGCAA AGTTATTCTT CCACCATGGC CAACAACGAA GGACTTTTCT CCCTGGTGGC 60
GAGGAAGCTG AGCAGACCCC TGTGATGCCT GTNACCTCAA TNAAAGCAAT TCCTTTGACC 120
TGTCAAA 127

SEQ ID NO:2213
SEQUENCE LENGTH:125
SEQUENCE TYPE:nucleic acid
TOPOLOGY:linear
CLONE:HUMGS02524
SEQUENCE DESCRIPTION:
GATCAATGTA CTTTTTATAA ACTTGCCATA GATATCTCAG ATTTGAAACC TCAAGACAGA 60
TTTATTATTC TTAAATGCTG TATGATAATG AAGAAAAATA AAAATTTATT TCTTGCAAAG 120
TTAAA 125

SEQ ID NO:2214
SEQUENCE LENGTH:121
SEQUENCE TYPE:nucleic acid
TOPOLOGY:linear
CLONE:HUMGS02525
SEQUENCE DESCRIPTION:
GATCAGTTTG GAGAGTAGGG GGCCACTCAT ATTCTGGGCT CCTGGAACCA ATCCCGTGAA 60
AGAATTATTT TTGTGTTTCT AAAACTATGG TTCCCAATAA AAGTGACTCT CAGCGAGCAA 120
A 121

SEQ ID NO:2215
SEQUENCE LENGTH:124
SEQUENCE TYPE:nucleic acid
TOPOLOGY:linear
CLONE:HUMGS02526
SEQUENCE DESCRIPTION:
GATCCCCGCA TGGTGTTGGG GGTGCTGGTG TGTCTTGGTG CCTGGACTTG AGTCTCACCC 60
TACAGATGAG AGGTGGCTGA GGCACCAGGG CTAAGCAATT AAACCAGTTA AGTCTCCCAG 120
GAAA 124

SEQ ID NO:2216
SEQUENCE LENGTH:120
SEQUENCE TYPE:nucleic acid
TOPOLOGY:linear
CLONE:HUMGS02527
SEQUENCE DESCRIPTION:
GATCAAAAAG GGGTCTGAGG TTGCATCAGG GAGACAACTG TAATGATATA AGCAATAGTT 60
GAGAACCTGA CAGCAAGTAA TAAGACAGGA AGAATAAAAC TAGAAGAAGT CAGAATGAAA 120

SEQ ID NO:2217
SEQUENCE LENGTH:120
SEQUENCE TYPE:nucleic acid
TOPOLOGY:linear
CLONE:HUMGS02528
SEQUENCE DESCRIPTION:
GATCTGAGTT CTGAGCAAGT CAGACTCCTT CCTTTTGGCC TCCAAAGCCA CAGATGTTGG 60
CCGGCCCACC TGTTTAACTC TGTATTTATT TCCCAATAAA GAAGGGCTTC CAAAGGCAAA 120

SEQ ID NO:2218
SEQUENCE LENGTH:120
SEQUENCE TYPE:nucleic acid
TOPOLOGY:linear
CLONE:HUMGS02529
SEQUENCE DESCRIPTION:

GATCGGAACT GTAAAGCTAT AATCCCCAGA ATTAAAGTTT TTATTATTTT TTATACATTG 60
TAAAACATAG ACGTTTATTT ATGTGATTAA NTTCTATTAA AATTTACATG CTAAANTAAA 120

SEQ ID NO:2219
SEQUENCE LENGTH:115
SEQUENCE TYPE:nucleic acid
TOPOLOGY:linear
CLONE:HUMGS02531
SEQUENCE DESCRIPTION:
GATCCAGTTT TTCTTGGGAG GTTGTATCTG GTCATAAGGT AAACATTCTA TATATTCTAT 60
GCCTGCTCTA GAATTGAAAG ACTTCAGCAG TATTAAAGCA TTTTTTAATC TTAAA 115

SEQ ID NO:2220
SEQUENCE LENGTH:122
SEQUENCE TYPE:nucleic acid
TOPOLOGY:linear
CLONE:HUMGS02532
SEQUENCE DESCRIPTION:
GATCTGTATT TGTATATGCT GATGCAATGA TAAAAATCAC TGTAATAGNT TCATTGTGTT 60
GTACTGGATG CAAAGCTAGA AAATATTGCA ATANNTGAGA CCGATGAAAG ACTTCTCTGA 120
AA 122

SEQ ID NO:2221
SEQUENCE LENGTH:110
SEQUENCE TYPE:nucleic acid
TOPOLOGY:linear
CLONE:HUMGS02534
SEQUENCE DESCRIPTION:
GATCTTGGGC AAGTTAATCT CTGGGAACTT TGGGTTTCTT ATCCTCAAAA AAGGCGATGG 60
AAGGNCTGGG NCNCGTGATT AAATAAAAGC AACGCAAGAA AAATGCCAAA 110

SEQ ID NO:2222
SEQUENCE LENGTH:109
SEQUENCE TYPE:nucleic acid
TOPOLOGY:linear
CLONE:HUMGS02535
SEQUENCE DESCRIPTION:
GATCGATGGT ACTTTTTGAA AATGCTTTAT TGCAAAAAAG TGGCTAGGGT TTTACAAGGN 60
CTATGAAGAA ACATATTAAG GTTAATAAAG CTTTTTATTG AGTGTTAAA 109

SEQ ID NO:2223
SEQUENCE LENGTH:105
SEQUENCE TYPE:nucleic acid
TOPOLOGY:linear
CLONE:HUMGS02537

SEQUENCE DESCRIPTION:
GATCCTCCCT TAGAGCATTT GGAAGAGTGT GGCCCTGCCA ACACCTTGAC TTCAAACTTC 60
TGGCCTCCAT AACTATGAAA TAAATNCTTG TTGTTTTAAG CCAAA 105

SEQ ID NO:2224
SEQUENCE LENGTH:108
SEQUENCE TYPE:nucleic acid
TOPOLOGY:linear
CLONE:HUMGS02538
SEQUENCE DESCRIPTION:
GATCTGTCCA CTTCTGGTGT CAAAGATTTT ACTCATCTTC TTAGTACATT CTATGTATTT 60
NATATGTATA ATTTTATACA ATTAAAAATA GATTTTNGTC TAGTGAAA 108

SEQ ID NO:2225
SEQUENCE LENGTH:102
SEQUENCE TYPE:nucleic acid
TOPOLOGY:linear
CLONE:HUMGS02540
SEQUENCE DESCRIPTION:
GATCTTATAT CATTACAGTA GCCTTTTTGT AGAATCCATC CTATTTTCCA CATGGATAAT 60
CATGTCATGA AGAATAAAGA CAGTTTTACT TCTTTCCTTA AA 102

SEQ ID NO:2226
SEQUENCE LENGTH:100
SEQUENCE TYPE:nucleic acid
TOPOLOGY:linear
CLONE:HUMGS02542
SEQUENCE DESCRIPTION:
GATCCCAGGG GAGGGGTCTC TCCTCCCACC CCAAGNCATC AAGCCCTTCT CCCTGCACTC 60
AATAAACCCT CAATAAATAT TCTCATTGGT CAATCAGAAA 100

SEQ ID NO:2227
SEQUENCE LENGTH:97
SEQUENCE TYPE:nucleic acid
TOPOLOGY:linear
CLONE:HUMGS02543
SEQUENCE DESCRIPTION:
GATCTGCTGC TTCCAAGTTG TGTGACCTTG GCTAAGTCAC TTAACCTTTC TGATTGTCAT 60
TTCGCTTTTT AATAAAGTGG GTCTGGTGAA CAAGAAA 97

SEQ ID NO:2228
SEQUENCE LENGTH:97
SEQUENCE TYPE:nucleic acid
TOPOLOGY:linear
CLONE:HUMGS02544

SEQUENCE DESCRIPTION:
GATCCCACTG TATGGCTATA CGGTATTTTA TCAGTGCATC CACTGTCGGG TTTTTCCCCT 60
TGTGGACTAT TATGAATAGT GGACGCTTGT GTGCAAA 97

SEQ ID NO:2229
SEQUENCE LENGTH:94
SEQUENCE TYPE:nucleic acid
TOPOLOGY:linear
CLONE:HUMGS02545
SEQUENCE DESCRIPTION:
GATCAGGTGA GCTGTGTCCA GAAAACCAAC GAGAAGGAGT GGAAGGAGGA ATGAACGTTT 60
CATTCTCGTT AATAAAGGCA TTATCCTAAT TAAA 94

SEQ ID NO:2230
SEQUENCE LENGTH:102
SEQUENCE TYPE:nucleic acid
TOPOLOGY:linear
CLONE:HUMGS02546
SEQUENCE DESCRIPTION:
GATCTAATCT ATTCCTGGGG CCATAACTCA TGGGAAAACA GAATTATCCC CTAGGACTCC 60
TTTCTAAGCC CCTAATAAAA TGTCTGAGGG TGTCTCATGA AA 102

SEQ ID NO:2231
SEQUENCE LENGTH:93
SEQUENCE TYPE:nucleic acid
TOPOLOGY:linear
CLONE:HUMGS02547
SEQUENCE DESCRIPTION:
GATCATATAT TTGTGGCATA CAGTGTGATG TTTTGACATG NATATAATTT TGTATACAAT 60
GTGGAATGAT TAAAACAAGC TAATTAACAT AAA 93

SEQ ID NO:2232
SEQUENCE LENGTH:92
SEQUENCE TYPE:nucleic acid
TOPOLOGY:linear
CLONE:HUMGS02548
SEQUENCE DESCRIPTION:
GATCTGCACT TCCTCCCCTT TCACCTACCT GTACACCTAA CCTGGCCTTA GACTGAGCTT 60
TATTTAAGAA TAAAATCGTG GTGGTGGTCA AA 92

SEQ ID NO:2233
SEQUENCE LENGTH:91
SEQUENCE TYPE:nucleic acid
TOPOLOGY:linear
CLONE:HUMGS02549

SEQUENCE DESCRIPTION:
GATCAGTGGC TGCTGGGTGG CGGGTACCCT TNCTCAGATG CCTGGCAGGN CTGGNTGGCG 60
ATTCATAAAG ACCTCGTGTT GATTCCCCAA A 91

SEQ ID NO:2234
SEQUENCE LENGTH:89
SEQUENCE TYPE:nucleic acid
TOPOLOGY:linear
CLONE:HUMGS02551
SEQUENCE DESCRIPTION:
GATCTAACCC CTTACCCATC TNTCTACTGC TGGACTGTGG AGGGTCACCA GGTTGGGAAC 60
ATGCTGGAAA TAAAACAGCT GCAACCAAA 89

SEQ ID NO:2235
SEQUENCE LENGTH:88
SEQUENCE TYPE:nucleic acid
TOPOLOGY:linear
CLONE:HUMGS02552
SEQUENCE DESCRIPTION:
GATCTTTTTG AGAGAAACAA ATGAGGATTG TAAAGTTTGG GGACTTACCT CTGTAGCATT 60
GTGAAAATAA ACTTTGATTA AGCTGAAA 88

SEQ ID NO:2236
SEQUENCE LENGTH:88
SEQUENCE TYPE:nucleic acid
TOPOLOGY:linear
CLONE:HUMGS02553
SEQUENCE DESCRIPTION:
GATCACCTCG GCGTCTGTTT TGTATGCTCT TTACCTATGG ACTAGTTTGT GGACGGCTAA 60
GAAATACAGC CTCAGCATTG ATTTGAAA 88

SEQ ID NO:2237
SEQUENCE LENGTH:88
SEQUENCE TYPE:nucleic acid
TOPOLOGY:linear
CLONE:HUMGS02554
SEQUENCE DESCRIPTION:
GATCTTAAGG AGTGTAATTT GCAATCTAAT TTTATCCTCT TACTCTGTGT TGTTAAACCA 60
TATACAATAA ACGGTGTTTA TGGAGAAA 88

SEQ ID NO:2238
SEQUENCE LENGTH:96
SEQUENCE TYPE:nucleic acid
TOPOLOGY:linear
CLONE:HUMGS02555

SEQUENCE DESCRIPTION:
GATCTGGCTG TGAGGCCCTC AGGGCAGGGA TACAAAGCGG GGAGAGGGTA CACAATGGGT 60
ATCTAATAAA TACTTAAGAG GTGGAATTTG TGGAAA 96

SEQ ID NO:2239
SEQUENCE LENGTH:86
SEQUENCE TYPE:nucleic acid
TOPOLOGY:linear
CLONE:HUMGS02556
SEQUENCE DESCRIPTION:
GATCCGGGGA CCAGGGGCTG ACCTCCATTC GTACCCCACT GCTTTGGAGN TGATGGGACT 60
ATCAATAAGA ACTCTGTTCA CGCAAA 86

SEQ ID NO:2240
SEQUENCE LENGTH:90
SEQUENCE TYPE:nucleic acid
TOPOLOGY:linear
CLONE:HUMGS02557
SEQUENCE DESCRIPTION:
GATCAAACCA TTTNACCTGG AGTTTGGTAC TGGTTTTAAC TTCTCTNAAT CTGTATAAGA 60
AAAATGAAGA CAATTGAACT TTCAAACAAA 90

SEQ ID NO:2241
SEQUENCE LENGTH:85
SEQUENCE TYPE:nucleic acid
TOPOLOGY:linear
CLONE:HUMGS02559
SEQUENCE DESCRIPTION:
GATCCAAGTT TTGCGGGGTC TAAAGCTGTG TGTGTTGAGG GGGATACTAA GTTTATGAAA 60
AAGAATAAAA AACACAACCA CGAAA 85

SEQ ID NO:2242
SEQUENCE LENGTH:84
SEQUENCE TYPE:nucleic acid
TOPOLOGY:linear
CLONE:HUMGS02560
SEQUENCE DESCRIPTION:
GATCTCGCCA CTNTTCTCCA ACCTGGGCAA TAAAGCAAGA NCCTGTCTTC AAACAAGCAA 60
ATAAAAACTT CCTTATCCTG CAAA 84

SEQ ID NO:2243
SEQUENCE LENGTH:80
SEQUENCE TYPE:nucleic acid
TOPOLOGY:linear
CLONE:HUMGS02562

SEQUENCE DESCRIPTION:
GATCCTGTTA GCTTTCTGAA TGTTCTGTGG TTGAATGTGT TTTTGCTTAA ATAAAGCTTT 60
TGGTATTTGT TTAAATTAAA 80

SEQ ID NO:2244
SEQUENCE LENGTH:79
SEQUENCE TYPE:nucleic acid
TOPOLOGY:linear
CLONE:HUMGS02563
SEQUENCE DESCRIPTION:
GATCCCAACC GTTTGGCACA GCTTTGGCCA CAGCCAGGCC CCTCTGGAAT TGTCCTTATT 60
AAACCAGTTT CCCGAGAAA 79

SEQ ID NO:2245
SEQUENCE LENGTH:78
SEQUENCE TYPE:nucleic acid
TOPOLOGY:linear
CLONE:HUMGS02564
SEQUENCE DESCRIPTION:
GATCCGGGTC AATGCTTCGT TTTCTAAAGG ATGCTGCTGT TGAAGCTTTG AATTTTACAA 60
TAAACTTTTT GAAACAAA 78

SEQ ID NO:2246
SEQUENCE LENGTH:76
SEQUENCE TYPE:nucleic acid
TOPOLOGY:linear
CLONE:HUMGS02565
SEQUENCE DESCRIPTION:
GATCTTGGTT TTCCATGAAT ACAGCATGTA TAATAAAAAT TTTAAGAAAT AAATGTTATT 60
CTACTTTATT AACAAA 76

SEQ ID NO:2247
SEQUENCE LENGTH:74
SEQUENCE TYPE:nucleic acid
TOPOLOGY:linear
CLONE:HUMGS02567
SEQUENCE DESCRIPTION:
GATCTTCTGC CGGGCGTGCC CCAGAGGACA GTGGGTGGAG TGGTCCTACT TATTAAATGT 60
CTCAGCCCCT CAAA 74

SEQ ID NO:2248
SEQUENCE LENGTH:73
SEQUENCE TYPE:nucleic acid
TOPOLOGY:linear
CLONE:HUMGS02568

SEQUENCE DESCRIPTION:
GATCTCACCT TTGCAAAGTT GGATAGTCAA TTTGTTTTTA GAGGAAATAA AACACTTATT 60
CTGTGATTTT AAA 73

SEQ ID NO:2249
SEQUENCE LENGTH:73
SEQUENCE TYPE:nucleic acid
TOPOLOGY:linear
CLONE:HUMGS02569
SEQUENCE DESCRIPTION:
GATCTGGAGA GAAAAACATT CAAACCATTG CATCATACAA AAAAAAGGCT ATTAAAAATT 60
GGATTATAGC AAA 73

SEQ ID NO:2250
SEQUENCE LENGTH:72
SEQUENCE TYPE:nucleic acid
TOPOLOGY:linear
CLONE:HUMGS02570
SEQUENCE DESCRIPTION:
GATCAGAGTA AGAATGTCTT AAGAAGAGGT TTGTAAGGTC TTCATAACAA AGTGGTGTTT 60
GTTATTTACA AA 72

SEQ ID NO:2251
SEQUENCE LENGTH:74
SEQUENCE TYPE:nucleic acid
TOPOLOGY:linear
CLONE:HUMGS02571
SEQUENCE DESCRIPTION:
GATCAAATCT GTACAGTTTC TATTGTTCCA GATTTTTTTA AGTTTGTATT AAAAGCATGA 60
TACATAATAG TAAA 74

SEQ ID NO:2252
SEQUENCE LENGTH:71
SEQUENCE TYPE:nucleic acid
TOPOLOGY:linear
CLONE:HUMGS02572
SEQUENCE DESCRIPTION:
GATCTTTTCA GTGTTTTTTC ACTTTAGCTA TGTGGATTAG CTAGAATGCA CACCAAAAAA 60
ATACTTGAGC N 71

SEQ ID NO:2253
SEQUENCE LENGTH:71
SEQUENCE TYPE:nucleic acid
TOPOLOGY:linear
CLONE:HUMGS02573

SEQUENCE DESCRIPTION:
GATCCAGGAG GGAGCCACCC CCAAGGACCC TGCCAGCCAC AGACCTCCAA TAAAGACAGC 60
ATGGAAACAA A 71

SEQ ID NO:2254
SEQUENCE LENGTH:71
SEQUENCE TYPE:nucleic acid
TOPOLOGY:linear
CLONE:HUMGS02574
SEQUENCE DESCRIPTION:
GATCTGATGT GAATTCAGAT TTCCAATCTT CTCCTAGCCA ACCATTTTCC TGGAATNAAA 60
AATTCAGTAA A 71

SEQ ID NO:2255
SEQUENCE LENGTH:69
SEQUENCE TYPE:nucleic acid
TOPOLOGY:linear
CLONE:HUMGS02575
SEQUENCE DESCRIPTION:
GATCCAGAAG TTCTAAATCC AGGATTTTAA TTAGTAACTC AAATAAAATG CCACACATTT 60
TCATACAAA 69

SEQ ID NO:2256
SEQUENCE LENGTH:69
SEQUENCE TYPE:nucleic acid
TOPOLOGY:linear
CLONE:HUMGS02576
SEQUENCE DESCRIPTION:
GATCATAGTA GTGCCGCTGT TGAGTGATAA AGCAACTTGT CATTAAAGAT GTGTGTNCAG 60
ACATGGAAA 69

SEQ ID NO:2257
SEQUENCE LENGTH:69
SEQUENCE TYPE:nucleic acid
TOPOLOGY:linear
CLONE:HUMGS02577
SEQUENCE DESCRIPTION:
GATCCCAGCC CAAATNNGCC CATTCACACA ATCAGGAGCN AAATAAATTA CTGTTGTCTT 60
AACACTAAA 69

SEQ ID NO:2258
SEQUENCE LENGTH:68
SEQUENCE TYPE:nucleic acid
TOPOLOGY:linear
CLONE:HUMGS02578

SEQUENCE DESCRIPTION:
GATCTTCAGC TGTTTGCCCG TNCTTATTAC ATAAACTGAA AACAGGATAA AAACGGAGTG 60
CAATGAAA 68

SEQ ID NO:2259
SEQUENCE LENGTH:63
SEQUENCE TYPE:nucleic acid
TOPOLOGY:linear
CLONE:HUMGS02580
SEQUENCE DESCRIPTION:
GATCTGTCAA AGATATGAAC ATGAAAGAAC AGCTTATTTC AATAAACCCA CTTTACATAC 60
AAA 63

SEQ ID NO:2260
SEQUENCE LENGTH:63
SEQUENCE TYPE:nucleic acid
TOPOLOGY:linear
CLONE:HUMGS02581
SEQUENCE DESCRIPTION:
GATCAAAAGT AAATGTTCTC TTTGCCATTT TAGATGGAAA AATAAAAAAA ATTAAAAAGT 60
AAA 63

SEQ ID NO:2261
SEQUENCE LENGTH:86
SEQUENCE TYPE:nucleic acid
TOPOLOGY:linear
CLONE:HUMGS02582
SEQUENCE DESCRIPTION:
GATCTACATG AAGGAACTGT AGATGATGTA TTTTTTTCAT CTTTTTTGTT AACTGATTTG 60
CAATAAAAAT GATACTGATG GTGAAA 86

SEQ ID NO:2262
SEQUENCE LENGTH:60
SEQUENCE TYPE:nucleic acid
TOPOLOGY:linear
CLONE:HUMGS02583
SEQUENCE DESCRIPTION:
GATCCCTTTT TTATTTTGTG GTTTTTTAAT ATAGTATAAA AATCCTTTTA AAAAAACAAA 60

SEQ ID NO:2263
SEQUENCE LENGTH:67
SEQUENCE TYPE:nucleic acid
TOPOLOGY:linear
CLONE:HUMGS02585
SEQUENCE DESCRIPTION:

GATCAAAACA GGATTCCATT GACCTAATTA TATTAAGCTA ATAAACTAAT TTTTTGAATT 60
TTTGAAA 67

SEQ ID NO:2264
SEQUENCE LENGTH:58
SEQUENCE TYPE:nucleic acid
TOPOLOGY:linear
CLONE:HUMGS02586
SEQUENCE DESCRIPTION:
GATCAATATT TATTGACTTG TAGTAACTGC CACCAATAAA GCAGTCTTTA CCATGAAA 58

SEQ ID NO:2265
SEQUENCE LENGTH:55
SEQUENCE TYPE:nucleic acid
TOPOLOGY:linear
CLONE:HUMGS02588
SEQUENCE DESCRIPTION:
GATCAATAGA CTGTACTTAT TTTCCAATAA AATTTTCAAA CTTTGTACTG TTAAA 55

SEQ ID NO:2266
SEQUENCE LENGTH:53
SEQUENCE TYPE:nucleic acid
TOPOLOGY:linear
CLONE:HUMGS02589
SEQUENCE DESCRIPTION:
GATCATTCTC AGTATCCACT GTCTATGTAC AATAAAGGAT GTTTATAAGC AAA 53

SEQ ID NO:2267
SEQUENCE LENGTH:53
SEQUENCE TYPE:nucleic acid
TOPOLOGY:linear
CLONE:HUMGS02590
SEQUENCE DESCRIPTION:
GATCCATTTN TTTTGTGAAT GTGCTAAATA AAAAGTGTTA TNAATTGCTG AAA 53

SEQ ID NO:2268
SEQUENCE LENGTH:52
SEQUENCE TYPE:nucleic acid
TOPOLOGY:linear
CLONE:HUMGS02591
SEQUENCE DESCRIPTION:
GATCCTGCAT CCCACTCCCT GGGAGCCAAT AAAGTGCATT TTCACAGGCA AA 52

SEQ ID NO:2269
SEQUENCE LENGTH:50

SEQUENCE TYPE:nucleic acid
TOPOLOGY:linear
CLONE:HUMGS02592
SEQUENCE DESCRIPTION:
GATCACAAAG TCAAAAACCC ATAAGAAAAA TTGAAAAAGC TACAAGCAAA 50

SEQ ID NO:2270
SEQUENCE LENGTH:50
SEQUENCE TYPE:nucleic acid
TOPOLOGY:linear
CLONE:HUMGS02593
SEQUENCE DESCRIPTION:
GATCATATTT TCTATTAAAG TGGCTAACAC CTGGCTACTC TTACTGTAAA 50

SEQ ID NO:2271
SEQUENCE LENGTH:290
SEQUENCE TYPE:nucleic acid
TOPOLOGY:linear
CLONE:HUMGS02612
SEQUENCE DESCRIPTION:
GATCTGTAAA TAAAATCAGT GCACTGTGAA TCACACCCAG CCCCTTTCCC TGCTGTGTGG 60
ATTAGGTGTC AAGACACCTA GTTCTTTCTG GGGCCACCCG GCTGGCCTCA CTGCTTATAT 120
TAAGGCTCCT CCCAACTCTC ATTTTCCTTT GGAAAACAAG ACTTTTTTCC CCATGGTTAC 180
CGCTGAGATA CTGGGGCTGT AGTAGTATAA AAGCTCACAG TTCCTTCTGA GTGCTGAAAA 240
GAGTGCATGA GTTGCTTCGA AATAAAAGGG TCAAGCATTC CTACCTGAAA 290

SEQ ID NO:2272
SEQUENCE LENGTH:556
SEQUENCE TYPE:nucleic acid
TOPOLOGY:linear
CLONE:HUMGS02627
SEQUENCE DESCRIPTION:
GATCTGTGCT TCCNACTCAC ATGTNAGGCC ACGCCATCCT CTTGCTGAAN CCCTATTGAT 60
GCTTTCTAAC AGGTTCTCGT GTCTCTCCCT GACCAGGCCC TCTTACAGAC TGCAGAATTA 120
CCATCCACAG TGTACATTGG ATGAGAATGT GTCACCACCA TGCTTAGAAC CACTCCTATC 180
ACTGACAGGT CAATATCTGA GCTCCTTGGC ACAACACACA TGTCCAGGGA GTGGCCAGTA 240
ATTAGTTGTG CCTGGAGTAT TGAGAGCATC AGTGGCCGGG AACATCAGGC AAGATGTGAT 300
GTTTTAAAGA GCCTTGAATG CCATGCTAAG GACTTNGAAC TGCATTGCAC AGCCCATGGG 360
GAGCTCTGGA AGGTTTAAAA GCAGNGAAGA NCCTTGCCTT TGTCTAAATT CTGTAAGTCT 420
CAAGCACTTC TGCTTTCCTT CATAGNACTT TGTAAATTAT TTCAATTGGC TTGGTTNATT 480
TTTTNCNNNA TTTTCCCCTT AAATTTCAAN GTTTTTGGGC GGGATTAAAT NNTGGCTTTT 540
TTGNTCNCNT TTTAAA 556

SEQ ID NO:2273
SEQUENCE LENGTH:527

SEQUENCE TYPE:nucleic acid
TOPOLOGY:linear
CLONE:HUMGS02628
SEQUENCE DESCRIPTION:
GATCTGAACA TCTGGCCCAA GTGAAGCATG GCATATAGTG CCCTTGGAAG AAAATTAGGC 60
CTCAAATGAC AGTAGCATTG AAGTGTTTGC TGCAGAGTTG AGGGAAACCC CCAGCCACCC 120
TCCCGGAATC CGAGATAGGG TGGCACATCT GTCCTGACAG ACGAGGAGTG TAACTNAACC 180
AGGAATATTT CCTCCATTCC TGCTCTCCCA CTGCACACAG GGTGGTGGCA CATTATCCCT 240
CTGGGGGGTG GGGACGCCTG TTGTTTTGGC TCAATTTGGG TTTGTTGGTC ACATGGAGCT 300
CTTCCATTTC GTTTAGCTGA ATAATGAGTT GTTCCTAGAG GAGACAGCCT GTCTCTCCTT 360
GTTNNCCNCA AAGCCCATGN CCTGNCGTGG TGGANCTGGG GCTTTTGGNT GGGGAGGGGN 420
CCCAACAATG GATNTTNTTN CCTTNCTCCG AATTCCAACG GANTNNAATG TNCAAAGGCC 480
CTTTNGAAAC TTGGNGGGAA ANTTAATTNC TNTNCCCTNG NNGTGGN 527

SEQ ID NO:2274
SEQUENCE LENGTH:458
SEQUENCE TYPE:nucleic acid
TOPOLOGY:linear
CLONE:HUMGS02629
SEQUENCE DESCRIPTION:
GATCAGCAGC CAATATGGGT TTGTTTGGTT TTTTTAATTC TTAAAAACAN CCTNTAGAGG 60
AATAGAAACA AATTTTTATG AGCATAACCC TATATAAAGA CAAAATGAAT TTCTGACCTT 120
ACCATATATA CCATTAGGCC TTGCCATTGC TTTAATGTAG ACTCATAGTT GAANTTAGTG 180
CAGAAAGANC TCAGATGTAC TAGATTTTCA TTGTTCATTG ATATGCTCAG TATGCTGCCA 240
CATAAGATGA ATTTAATNNN NNNCAGCCAA AGCAATATAC TCTTACATGA TTTCTAGGCC 300
CCATGACCCA GTGTCTAGAG ACATTAATTC TAACCAGTTG TTTTGCTTNT AAATGAGTGA 360
TTTCCATTTT GGGGAACAGG TTTCAAATGG ANTATATNTA CATGGGTAAA ATTCCCCTGT 420
GCTAGTGTAG GNCTTTCCTN GGGGAATGTT TTATGGGN 458

SEQ ID NO:2275
SEQUENCE LENGTH:452
SEQUENCE TYPE:nucleic acid
TOPOLOGY:linear
CLONE:HUMGS02630
SEQUENCE DESCRIPTION:
GATCGACTAC AAGCCAGCCC CTANGCACCT GCTGCTACAT CATGAAGNTA NCTCCAGAGA 60
GCATCCCCAG TCTTGAGGNT CTCAATAGAA AAGTCCACAA CTTCCAGGCC AAGCCCGCAG 120
TGCCTACGTC TAAGCTGGGC CAGGCAGAGG GNCGAGATGC AGGCTCAGCA CCCTCCGGAG 180
GGGACCCGGC CTTCCTGGGN NTNGCCGTNA NCACCCTGTG TGGCGAGGTG CCGCTCTACT 240
ACATCTAGGA CGNCTCCGGT GAGCAGGGTC AGTGGAAGCC CCAACGGGAA AGGAAACGCC 300
CCGGGCAAAG GGTCTTTTGC AGCTTTTGCA GACGNGCAAG AAGCTGCTTT CTTCCCACAA 360
CCGNAGGACC AAAACCCTTG GAGAAANTGN GNAGNTTGGN GGAGATGGAT TTGGGAGTTT 420
GGCAANAGGG TTGGACCCNA NGNGGGCCCG GN 452

SEQ ID NO:2276

SEQUENCE LENGTH:451
SEQUENCE TYPE:nucleic acid
TOPOLOGY:linear
CLONE:HUMGS02631
SEQUENCE DESCRIPTION:
GATCTTGTTT ACTTTTGGAC ATCAAGCCCA TCACTGCCAG CCAGTNAAGA AGGATTCCAG 60
CCTATGCCCT CAATCACAAT AAGACCACCA GATGACCAAC ATCTTCCTAC TGCAAATACT 120
TGCATTTCTC GACTTTACGT CCCACTCTAT TCCTCTAAAC AGATTCTCAA ACAGAAATTG 180
TTACTCGCCA TTAAGACCAA GAATNTTGGC NNNGTGTAGA GTATAAAAAG TGTGTATTGC 240
TGTGTAATAT TACTAGCAAA TTTTGTAGAT TTTTNTCCAT TTGTCTATAA AAGTTTATGG 300
AAGTTAATGC TGTCATACCC CCCTGGTGGT ACCTTAAAGA GATAAANTGC AGACATTCCT 360
TGCTGAGTTT ATAGCTTAAA AGGCCTAAGG GAGCACTAGC AANCATTTTG GGCTATATTT 420
GGGTTTGCTA GGTCACCAAC TTTCCTGGGG N 451

SEQ ID NO:2277
SEQUENCE LENGTH:446
SEQUENCE TYPE:nucleic acid
TOPOLOGY:linear
CLONE:HUMGS02632
SEQUENCE DESCRIPTION:
GATCCAGCCA GGCATCTCGG CGCTTTGGCA CCATGAGTTC TATGTCTGGG GCCGACGACA 60
CTGTGTACAT GGAGTACCAC TCATCGCGGA NAAGGCGCCC AGCAAACATG TACACCCGCT 120
CTTCAAGCGC TTTAGGAAAT NATNCTTAGG CAGGGTACTT CGTTCAAGAC CGGCGCTTGG 180
CACCCTTGTT GGAAAGGGNT TTTCAGCATA ACATTTTCCT TCCACCTCTT TGACCTTCCC 240
TCCAGCGTTG GCCAAATTGT GCTGAGGAAG ATGCATCAAG NGNTTGGCTG TGCCTTCATA 300
GGTCATCTAG GGTTTTATAA AGGAGGAGGA GACAATATTT NTTCAAACTT TTTTGGGAGT 360
GGGGTCATTT TCTGTATATA AANAATGTTA ATATTTAAGG TGTAGTTTAT GTTACCGTTC 420
TGAATAAACA GATTNGGCCA TTNAAA 446

SEQ ID NO:2278
SEQUENCE LENGTH:425
SEQUENCE TYPE:nucleic acid
TOPOLOGY:linear
CLONE:HUMGS02633
SEQUENCE DESCRIPTION:
GATCCACACC TGACCCTGTT GGGTCGAGTT AGGNTGGGCC TTGGTCTGCA CTGTAGCACC 60
TNTGTTCTTT GAGTTCACAT CATGAATGTG GTGACTTNCC AGNTACCATA TCAGGCTTAA 120
CCTAGCACAT CCTATTTCTT TNCTTCTATG ATATCCAAAT TGGACTGACC TCACTTCAAA 180
GTTGCTGTCC CATTTTGTCA CCCTATCTTA TCTCGGGGAA ATTGCAGACT GATGGCCAGA 240
CCAACTCTGT TGAAATTCTT GCATAGAGCA AACCTGTGCT CATTTTTAAG TGGCATGGGA 300
GAGGCCCCCA GCCTAGTAAA GCCTAGTCTG TGTCTTCACA GTGCTGGTAG NATGNGTTTT 360
GTGTGTATAA ATATATGATA TAGGATTGAT AATATGTTNN CTAACGGCCA TATTATTGAA 420
GGGCN 425

SEQ ID NO:2279

SEQUENCE LENGTH:414
SEQUENCE TYPE:nucleic acid
TOPOLOGY:linear
CLONE:HUMGS02634
SEQUENCE DESCRIPTION:
GATCTGCCTT AACTCCAACT TACATGTTTT GGTCACTATT ACAAACTGTC ATCCCAGAAT 60
GATGCTGCAG AGGCTAGGGC TAGGACACAG ACCAGTGTTT CCATGTGGGA ATTCCCTCCC 120
AGTATTTCTT AGGAANNNTA TGTTTTTTGA ATCCATAATC CCTAGAAAAA TCAGTTGAGG 180
AAATGAGAAG TATTGTAATT ATNCTGTGAA TAGTAACACT TACCATTATG GAGACATCAC 240
TAGTTTGAAA GANTCCAACT TCATCAAATN TTAACGTACC GAGTTGAAGG NTACAAGANC 300
TGAGACAGGA GCATAGCAGA GGAGAAACGG TCACCATCTC ATTAGCCCTA TTTTTTGGNT 360
TGNTGTGATG CCATTACATC TGNAATAATC TGGGCCATNT CAGCTGCTAA TGGN 414

SEQ ID NO:2280
SEQUENCE LENGTH:293
SEQUENCE TYPE:nucleic acid
TOPOLOGY:linear
CLONE:HUMGS02635
SEQUENCE DESCRIPTION:
GATCTCACCT TCCTTCGAAT TCGCTCCAAG AAAAATGAAA TTATGGTTGC ACCAGATAAA 60
GACTATTTCC TGATTGTAAT TCAGAATCCA ACCGAATAAG CCACTCTCTT GGCTCCCTGT 120
GTCATTCCTT AATTTAATGC CCCCCAAGAA TGTTAATGTC ANNNNNGTCA GTGGACTAGC 180
ACATGGCAGT CGCTTGGAAC CCACTCACAC CAATCCAGTG ACCGTGTGTG GGCTGGNGGC 240
TCTNTNTCCC NCNCCAACGG AACCCCNGTG TGCACCAACC TTTNNCNCAG AGN 293

SEQ ID NO:2281
SEQUENCE LENGTH:400
SEQUENCE TYPE:nucleic acid
TOPOLOGY:linear
CLONE:HUMGS02637
SEQUENCE DESCRIPTION:
GATCTGGCAG GGCACCACAG CGCCCCCTAC TGAACCATCA GCATGTCAGT GGCATTTAAA 60
GCCATGCAGC TGGAGGGGCC ACTGAGATTG TCTCTGAGTA TTACTGAGAA GCAACAGAAA 120
AGAGCCATGG ATGGAGCCCT TGGGCTCTCT GGGAAATGGG AAATCAGCCA AAGGACTGAG 180
AAGGAGTTAC CTTAAGGTCA GAGAAAACCA AGAGAGTGTG GTGTTCTGGA AGCTGAGCTT 240
TCTTTATTCA ACCTCATTCC CTTCTCCAAA TAAGCCACTT GTGTAGTTGG GCCCCTCCAG 300
GGTTGAAGGC AAGAGGAGAA AGGCACAGCG TTTGGGAAAC AAGACTTTTC CTGCAATAGC 360
CTGGGGAAGG AATAAAANGG ATAGAGGTGT TTGGGGGAAA 400

SEQ ID NO:2282
SEQUENCE LENGTH:400
SEQUENCE TYPE:nucleic acid
TOPOLOGY:linear
CLONE:HUMGS02638
SEQUENCE DESCRIPTION:

GATCTGTCCA ACGCCTGCAG CGTGGATGTG GAGGCGGACT AATCAACNAG TGCTCTGTTA 60
TTATTTCTAC CCATCCCTTT TCTCCATGCT AATTTGCTTA GAACTTTAAC ATTGAATGGA 120
CAGCAAATGT CATGTTCTGT TTAATTATCA CTCCTTGACC ATTATGTTAA TTTCTTTCCC 180
TAACAAGGTG ACGCATTGTT TTAAGTAACA ATGACCAACT CATCTACTCT TCTTCCAAAT 240
TGTGTAAATT GAANGGGTAA AATGTGGGAA AACGCTAAAT TTGAATGCTA AATTTGAACA 300
TTTCACACAA CCAATAATTT TGAAATGCTT CTTNGCAATA TTATNGCGGT TTCGGNACCA 360
GGTTAAAAAT NNCTTTCATT TTCAGGNCAT TTTGGTCAAA 400

SEQ ID NO:2283
SEQUENCE LENGTH:386
SEQUENCE TYPE:nucleic acid
TOPOLOGY:linear
CLONE:HUMGS02639
SEQUENCE DESCRIPTION:
GATCGATAAT ATAATTTATA ATGAAATTGC TTTATCAGCT ATTAATGTTA ACTACATCAT 60
CTTCATATAG CNTTATAACT CATTTGTGCT ATTCCATTTT CCTCTGTTCC TTTTATTTTC 120
ACTTCCCTTG TAATGTTAGT CTCTTTGTAC TGATTTCTGA AGAGTTCATT TATGATTAAA 180
CATGTTAACA TTTTGTCTAG AATTGCAAAT ATGTTTTTCC TCATTCATTT TACTATGGTG 240
TTTTATTTTT GGGTTATACA GAAGTNGTAT ATTGAAATAT AATCTNGTNC TGTTTTATGA 300
CTTTGGAGTT TTGTGGTTTT TTNAAACCAN GTTTACAGTG GNATCCNTNN ATTNGGTATT 360
GCCGTCNTAN TNCCNGCAGT TAGGTN 386

SEQ ID NO:2284
SEQUENCE LENGTH:384
SEQUENCE TYPE:nucleic acid
TOPOLOGY:linear
CLONE:HUMGS02640
SEQUENCE DESCRIPTION:
GATCTNNTCA AACAGCAAAC GCCCACAGAN GGCCCAGAGG NGGTGGTAAT CAGGGTGTGT 60
GGGTGNTTTT AGGGTTCTTT AGTGTTGTTT CTTTCACCCA GGCGGTGGTG GTCCCAGCCA 120
GTTTGGTGCT GACGGTGAGA GGAAATNAGA ATCTGTTTGC AAATTGTCCA ACCCACCCCC 180
TCAACATGAG GGGCTTCCAT TTTNTGTGTT TTGTAAGGGA ACTGTTTCCT TCATGCCGCC 240
ATGTTCCTGA TATTAGTTCT GATTTCTTTT TAACAAATGT TATCATGATT ANGAAAATTT 300
CCAGCACTTT AATGGCCAAT TAACTGAGAA TGTAAGAAAA TTGATGCTGT ACAAGGCAAA 360
TAAAGCTGTT TATTAACCTT GAAA 384

SEQ ID NO:2285
SEQUENCE LENGTH:383
SEQUENCE TYPE:nucleic acid
TOPOLOGY:linear
CLONE:HUMGS02641
SEQUENCE DESCRIPTION:
GATCTTTTCT ATAATATCCT ACTACTTTGG TTTTCCTAGC TCCATACCAC ACACCTAAAC 60
CTGTATTATG AATTACATAT TACAAAGTCA TAAATNTGCC ATATGGATAT ACAGTACATT 120
CTAGTTGGAA TCGTTTACTC TGCTAGAATT TAGGTGTGAG ATTTTTTGTT TCCCAGGTAT 180

AGCAGGCTTA TGTTTGGTGG CATTAAATTG GTTTCTTTAA AATGCTTTGG TGGCACTTTT 240
GTAAACAGAT TGCTTCTAGA TTGTTACAAA CCAAGCCTAA GACACATCTG TGAATACTTA 300
GGATTTGTAG CTTAATCACA TTCTAGACTT GTGAGTTGAA TGACAAAGCA GTTGGACCAA 360
AAANTTTATG GGCATTTAAA GGN 383

SEQ ID NO:2286
SEQUENCE LENGTH:424
SEQUENCE TYPE:nucleic acid
TOPOLOGY:linear
CLONE:HUMGS02642
SEQUENCE DESCRIPTION:
GATCTAAAAT ACCTGGTGGC TTTTNACAAA AAGCAGATTT TNTTCATGTA CTGTNATGTC 60
TGATGCAATG CATCCTAGAA CAAACTGGCC ATTTGCNAGT TTACTCTAAA GACTAAACAT 120
AGTCTTGGTG TGTGTGGTCT TACTCATCTN CTAGTACCTT TAAGGACAAA TCCTAAGGAC 180
TTGGACACTT GCAATAAAGA AATTTTATTT TANACCCAAG TCTCCCTGGA TTGATAATAT 240
ATACACATTT GTCAGCATTT CCGGTCGTGG TGAGAGGCAG CTGTTTGAGC TCCAATGTGT 300
GCAGCTTTGA ACTAGGGCTG GGGTTGTGGG TGCCTCTTCT GAAAGGTCTA ACCATTATTG 360
GATAACTGGC TTTTTTCTTC CTCTTTGGAA TGTACCATTA NAAATAATTT TTTGAACCAT 420
CAAA 424

SEQ ID NO:2287
SEQUENCE LENGTH:378
SEQUENCE TYPE:nucleic acid
TOPOLOGY:linear
CLONE:HUMGS02643
SEQUENCE DESCRIPTION:
GATCCGAAGA GGAAGGGCGG TCCGGGCTCC ACACTTAGCT TCGTGGGCAA ACGCAGAGGC 60
GGGAACAAAC TAGCCCTNAA GACGGGAATA GTAGCCAAGA AGCAGAAGNC GGAGGATGAG 120
GTATTAACAA GTAAAGGTGA CGCGTGGGCC AAGTNCNNGG CAGAAGTGAA AAAGTACAAA 180
GCTCACCAGT GCGGTGACGA TGATAAAACT CGGCCCCTGG TGAAATNACG CCCCTCCCCC 240
ACCTGCCCAT GGCCTGGGAC TCTCTGCGAT GTACATAACT ATTTAATGCA GCGGCANGTC 300
GACAGCTTTC CCTGAGAGGA CTTAAAAGCA GAAGGAAACC GAGATGCTTC CCGNAAGCCG 360
TGGACGATTC TCCAAGGN 378

SEQ ID NO:2288
SEQUENCE LENGTH:370
SEQUENCE TYPE:nucleic acid
TOPOLOGY:linear
CLONE:HUMGS02644
SEQUENCE DESCRIPTION:
GATCTTCTTG ACTATGCCAG TTGTAGTACC AGCTTCTGTA TCTGCACTGA ATTCTGNCTC 60
AGTAATATGC CTTTGTTTAA AATTTAAATA TTTTTTCATT TTTTTAACCT AGAAAATAAT 120
TATAATGAAN NNNNTAAGTA TCTCATTTTA GGATTCTGAT TTACATAGGT ATCACTGTAA 180
CTTGTGCTGT TTGCATAGGT ATACTCTATC TTGTGCTATC TGCATAAAAT ATCCAAGTAA 240
ACACATTGTG ATTTACATC CGTGCATAGA AAAAAAAATC ATCTGAACTC AAATCAATCT 300

GTTGATACTG ACTAGATTGG TGACGTGTTT ATGTGTACCA CTAGTGATAA TGCACGGTCC 360
TGTACAAGCN 370

SEQ ID NO:2289
SEQUENCE LENGTH:370
SEQUENCE TYPE:nucleic acid
TOPOLOGY:linear
CLONE:HUMGS02645
SEQUENCE DESCRIPTION:
GATCACAGGA ACAGCAGGTG ACAGGCCTAG CTATAGTTAG GAATACACAA GCCGTAAAAT 60
CGAGTCCTTA CAGCCATACC ACAAGGTACG TCCANNTGGA CTACAAGAAG AGCTTCCTTT 120
AAAGTTCCTA TTTCAGCATA AAGAGGCTGT CCTTTTTTTT NAGGAATAGT TTGGACCTTG 180
TGCCTCCTGT GGGAGGCTGA GGACTGCAAG AGGAGAGCTA GCAGATATGC CTGTTCACCC 240
CTCTCTGGTA CTTGTGGCTT GCTAGTATGT TTTNATGATA ATCTCGGGCA TTGTTTGCAT 300
TTGTGTTTAT TAATAGGGTT TTTGTTTTTT GTTGTTTCCT NTTTTNACAG TAANGGCTGA 360
ATTACATAAA 370

SEQ ID NO:2290
SEQUENCE LENGTH:367
SEQUENCE TYPE:nucleic acid
TOPOLOGY:linear
CLONE:HUMGS02646
SEQUENCE DESCRIPTION:
GATCTGAAAG GAATGCTAGA AGTGTTTAGA GAGCTGACAG ATGTCCGACG CTACTGGGGG 60
CATGCNACAG GGGGAATGGC TCGCTTCAGT GCCCTGCTGC TCAAAGCTCT AGGGNAGAAT 120
GCAGTCGGGT AGNCTGTGGG GCTCTAACCC CACAGCAACC TCTAGGGATG ACGTCACTAA 180
AAACCGATGA AGCCTCCATA ACATTGGTTC CTTACTGACG CTGAGGAGCA NGGCAATCTT 240
CACACCTTAA CCAATGCTAT TCATGTAGGA TGACCAAGAA ATGAATGTTT CTTACTGAAA 300
TAATACATAT TTTTGGCATC TTTTGGTTAC CCATAGACTA ATTAAANCTA ATCCNNGGNT 360
TTATAAA 367

SEQ ID NO:2291
SEQUENCE LENGTH:359
SEQUENCE TYPE:nucleic acid
TOPOLOGY:linear
CLONE:HUMGS02648
SEQUENCE DESCRIPTION:
GATCCTGACA GAGCCCTGAA CAGATTATAT CACTCGGCTA GAGGCACTGC AAAGACGACT 60
TGGAACTATA CAGTCAGGTT CAACTACTCA ATTTCATGCT GGCATGAGAA GATAATCCTT 120
TGAAACATCA TTAATTGAAG TGATTTTAAA TAGATTTCCT TTTGTAAATC AATGGTTCTT 180
TTGTGCTTTN GNNNNNNCGA ATATTCAATG GGACCAATAT GAACACAGCT TATGATTGTA 240
TACAAATCCC TTGCCAGCAC ATGAAANCAA ACTGGANTTT GTATATATAA GCATTGTGTA 300
TGTATTCATG CACAATAATT ATNGANTTAC CTGTATATTT GTGGGAATGC TAATTTAAN 359

SEQ ID NO:2292

SEQUENCE LENGTH:357
SEQUENCE TYPE:nucleic acid
TOPOLOGY:linear
CLONE:HUMGS02650
SEQUENCE DESCRIPTION:
GATCTGTGGG TCCGGCTGNA GGAGAGTGAC ATAGTCCCGC AACTCCANNT TGCCTGTCCT 60
GGACAGGCAC ATCTCCACTG AACTGCGGCT CCNTCGCCGT GGGCATTCCC ACCACAGCCA 120
GATGGTGGCC AGCAGTGCCT GCCTGTNTCT CTGGACTCCT GTGTTCTGGG TGCTGGTGCT 180
GGCTTTCCAA ACAGAGACAC CGCTCCTGTA ACGACTGGAA GCACCAGGCT AAGNACCTNA 240
CCCCTCGNAC TTNGAAGGAA TGGGCATTNC NGTACTTCAA CATTCTGGGT CTAGGCCTTG 300
TTGGGGCCAA TTCAAGAAGN GGCCTTGTTN TTNGAAAAAA GTGGGCCNNG TTTTNAN 357

SEQ ID NO:2293
SEQUENCE LENGTH:355
SEQUENCE TYPE:nucleic acid
TOPOLOGY:linear
CLONE:HUMGS02651
SEQUENCE DESCRIPTION:
GATCCTTCTT TTTGGTTTTG GAAATATAAT CATTGTTAAT GTTTTCCCTC CAAATAGAAT 60
ACTGTTTTAT CCATACAAAT CATAACAGCA TCTATCCCAT GCTAGGGTTG GAAACTGATA 120
TTGGTATTAC TTGTGTTTTT CCTTAGTGTG TTTTATTTCC CAGTTTCATC TNCTTTTAAA 180
AATGAAAATA TGGTGCCTTC CCTCCCTCCA GGAAGACTGG CAAATATTNC CTTTTATTTA 240
CTGCTGCTGT GGAGTGATGA GATATGCACT TTACTCTTTA AGATTCAGCA AAAAGCTTTT 300
CACTTCTCAG TATATCCAGA ATACATCATA TCTGGGACTT AGGAAAATTT GCCAN 355

SEQ ID NO:2294
SEQUENCE LENGTH:330
SEQUENCE TYPE:nucleic acid
TOPOLOGY:linear
CLONE:HUMGS02652
SEQUENCE DESCRIPTION:
GATCTTTTAC ACCACATCAC AGTGANCACA CTGGGGAGAC GTGCTTTTTT GGNAAACTCA 60
AAGGTGCTAG CTCCCTGATT CAAAGAAATA TTTCTCATGT TTGTTCATTC TAGTTTATAT 120
TTNCATTTAA AATCCTTTAG GTTAAGTTTA AGCTTTTTAA AAGTTAGTTT TGAGAATTGA 180
GACACAATAC TAATACTGTA GGAATTGGTG AGGCTTGACT TAAAACTTTC TTTGTACTGT 240
GATTTCCTTT TGGGTGTATT TTGCTAAGTG AACCTTGTTA AATNTTTNGT TAACTAAATN 300
TTTTCCTTAA AATAAAGACT TTTTCACAAA 330

SEQ ID NO:2295
SEQUENCE LENGTH:352
SEQUENCE TYPE:nucleic acid
TOPOLOGY:linear
CLONE:HUMGS02653
SEQUENCE DESCRIPTION:
GATCCTATGG ATTTTCCCGG CTGGTTGCCA CTACTNTACA ACATTCANAG CCCACATCCA 60

TCTGTGCCAT TAAGCTTTTT TGAGACATGA GAGATGCCTC TTCCCTGCTG TATGACATGC 120
ATTTGGGAAG TTGGAAAGAA ATGACAAAAT CAGGGAGAAA ACATCCAAGC TTCTTACCTG 180
TAGATAGAAT CAGCCCTCAC TTGGTGCTTA TTACCAGTTA TTCANGGACA ATAACAACAA 240
CAAAATTAGT AGACATNCAT GANGCACATN TTTAGGNCCA AAGGNTAGCA TCAACTTGTA 300
TTTNGAAGGG ACTGTAGTTT TCGGCATTTT ATGGCNATTT TTTATNAAAG TN 352

SEQ ID NO:2296
SEQUENCE LENGTH:351
SEQUENCE TYPE:nucleic acid
TOPOLOGY:linear
CLONE:HUMGS02654
SEQUENCE DESCRIPTION:
GATCGCAGCC GCTTCGCACG CCGCATCACC CAGGCCCAGG AGGAGCTGAG CCCCTGCCTC 60
ACCCCTGCTG CCCGGGCCAG AGCCTGGGCA CGCCTCAGGA ACCCACCTTT AGCCCCCATC 120
CCTGCCCTCA CCCAGACCTT GNCTTCCTCC TCTGTCCCTT CGTCCCCAGT NCAGACCACG 180
CCCTTGAGCC AAGCTGTGGC CACACCTTCC CGCTCGTNTG CTGCTGCAGC GGCTGCCCTG 240
GACCTCAGTG GGAGGCGTGG CTGAGACCAA CTGGTTTGCC TATAATTTAT TAACTATTTA 300
TTTTTTCTAA GTGTGGGTTT ATATAAGGAA TAAAGGCTTT TGATTTGTAA A 351

SEQ ID NO:2297
SEQUENCE LENGTH:357
SEQUENCE TYPE:nucleic acid
TOPOLOGY:linear
CLONE:HUMGS02656
SEQUENCE DESCRIPTION:
GATCCCCGTT CCTGACATCA CAGCAGCCTC CAACACAAGG CTCCAAGACC TAGGCTCATG 60
GACGAGATGG GAAGGCACAG GGAGAAGGGA TAACCCTACA CCCAGACCCC AGGCTGGACA 120
TGCTGACTGT CCTNTCGNGT NCAGCCNGTG GCCTTGGCTT TTCTAGCCTA TTTACCTGCA 180
GGCTGAGCCA CTNTCTTCCC TTTCCCCAGC ATCACTCCCC AAGGAAGAGC CAATGTTTTN 240
CACCNATAAT NCTNTCTGCC GANCCCTAGT TCCCTCTGCT CAGCCAAGGT TTGTTATCAG 300
GTTTTCAGGG GCCATGGGTT CACATTAAGG ATTTAAAAGG GTAGTAATTT GGGGAAA 357

SEQ ID NO:2298
SEQUENCE LENGTH:344
SEQUENCE TYPE:nucleic acid
TOPOLOGY:linear
CLONE:HUMGS02657
SEQUENCE DESCRIPTION:
GATCAAATTT NAGTGGCTTT GGACAGAAAA GAAGGCTCTG GATTTAAGCG GGTGGTCACC 60
TGTGAGACCA GGTCTACCTT GGGACTGTTA TTTAACTGAA TCAGTTATTT CCTTGAAATT 120
TCACAGTAGT GGGTGGGCCT GTTTTAAGGC TCTGACAGAT ACCACGAAAC ATGANGCNCG 180
NNGAACTACA AGACCCCCGG GGTCTTTCTG AGTGCAAGGC TGAAATGGAC AAGGGCTCCT 240
CACGGGGGTG GAGGGAGCCG GAGCCTGCCT TGTGTTCCTT TTTTGACTTG TGACATTTTT 300
CAAACACATA ATTAAAAGGA CTTATGCTCT GCTGTCTCAG GAAA 344

SEQ ID NO:2299
SEQUENCE LENGTH:462
SEQUENCE TYPE:nucleic acid
TOPOLOGY:linear
CLONE:HUMGS02658
SEQUENCE DESCRIPTION:
GATCTGGTCC CTGAGAAACA TATTGTGATG AAGTGGAGGT TTAAATCTTG GCCAGAGGGA 60
CACTTTGCCA CCATCACCTT GACCTTCATC GACAAGAACG GAGAGACTNA GCTGTGCATG 120
GANGGTCGAG GCATCCCTGC TCCTGAGGAA GAGCGGACAC GACAGGGCTG GCAGCGGTAC 180
TACTTTGAGG GCATTAAACA GACCTTTGGC TATGGCGCAC GCTTATTTTA GGGCCAGCGG 240
CAGGGGACTC CAGCCTGCTG GACACTTCAN GTCCAGCTCT CTCCTGACTG GGGCTTNCGA 300
CTCACAGGAT TGCATCGNCC CAGCTGCTAA CTTNGGGCCG GGGGCCCNTC CCTTNCACAA 360
TATACCTTGG GTTTNTGCAT GTTTTCCTNN CTGGGTTGGG GTTCAGAGGG GCAATTTCNN 420
CTTTTAATGG TGTACATATN NCTANATANN CATAATTTTA AA 462

SEQ ID NO:2300
SEQUENCE LENGTH:341
SEQUENCE TYPE:nucleic acid
TOPOLOGY:linear
CLONE:HUMGS02659
SEQUENCE DESCRIPTION:
GATCAACTTC CCACAAACTG AGGAATGAAT TCCACGAGCC TGTTCTGAAA ATNTGGACGT 60
AAGACAAACA CGTGCTCGTC CTTTAATGGA GTTCACCAGC ACACTTGTTA ACCAGTCCTG 120
TTTGCTTTCG TCTTTTTTTG TGCGTAATAA AGTCAACTGA CCAAGTGACC ATGAAAAGGG 180
GCTGTCTGGG GCTCCTGTTT TTNAGCTGCT GTTCTTCAGC TCCGACCATG TTGCTGTGTG 240
ATTATCTCAA TTGGNTTTAA TTGAGGCAGA AACTGAAGCT CTACCAATGA ACTGTTAGAA 300
ACAAGACACA CTNTNGTATT AAAATTGCTT GCAGTAACAA A 341

SEQ ID NO:2301
SEQUENCE LENGTH:339
SEQUENCE TYPE:nucleic acid
TOPOLOGY:linear
CLONE:HUMGS02660
SEQUENCE DESCRIPTION:
GATCNGTGGC AAAACCAAGT GTGGTCTTTC AGCTTCAGTG TGTTAGCATC CCAGCACCTG 60
GGATGGGGTT TGTGGGCAAG TTGATTTTGC CACACTGTAG GGCCCAGGTN NNCCCATTGC 120
CACCGTGAAG CCAAATNCTA GCCCAAGTCT GGTAGAGCAG TCGGGCAGCT AGTTTTCCGG 180
GGAGGATGAT GTGGTGGGAC AGAGGACCTC AGTGGCCTAT TACGGAGCCC TCTTCCCAGA 240
CTAAATTCAT AGCCGAGAGC TCGGGGGGAT GGTAGAGTTA AAATTTAAAA TAATTTTCTA 300
AATTCTGTAT TTGGATATTA GTGTTTAAGT GGTAATTTN 339

SEQ ID NO:2302
SEQUENCE LENGTH:441
SEQUENCE TYPE:nucleic acid
TOPOLOGY:linear

CLONE:HUMGS02661
SEQUENCE DESCRIPTION:
GATCCAGGGG CTGCCTCTGG CCTCTTAGGG AGCAGAGAGC AGAACTCCGC AGCCCAGCCC 60
AGAGGAGTGT CACCTCCCAC CTTTGGAGAG GAATCCTTCC CTCCCCTGGA CAAAGTTGCT 120
GACAAGCCCC TGAAGTGGCC TCTCCATATT CCAGCTGAGC CTGAATCTGA CTCTTGAGGG 180
TTGGGGCTGC ACTTATTTAT TGCGGGGAGA CAGCTCTCTC TCCCACCTNC TCCCCAGATG 240
GGAGGAGAGC CTGAGGCCCA AGCAGGACCC GGGGGTTCCA GCCCCTAGCT GCTCTGGAGT 300
GGGGGAGGTT GGTGGACCAT GGGAGTNCCT GGTGCTGCCC CTNAAGGTGG GACCCAGGCG 360
TTNTCAAGCT GTACCCTTTT NCCGATGGAA TTTGTGTTTT TGAANATNNG TGTCTNNTAC 420
TANTTNTTTT AAATAAAAAA N 441

SEQ ID NO:2303
SEQUENCE LENGTH:336
SEQUENCE TYPE:nucleic acid
TOPOLOGY:linear
CLONE:HUMGS02662
SEQUENCE DESCRIPTION:
GATCATGGCA TCCAAGACCA GAGTCTCAGA ACTCATTAAG AAACAGTTTA CTTGGAATGG 60
AGAATACCCA TCTGTAATAC AGGTCCTGTC ATTTCATTCA TCTCAAATTA TTTTGAATTC 120
TTCCCAAATG GCTGCTGGAT TTAGGTGGTA ATAGGGGCTG TGGGCCATAA ATCTGAAGCC 180
TTGAGGAACC TTGGGTCTGG AGAGCCATGA AGAGGGAAGG AAAAGAGGGC AAGTCCTGAA 240
CCTAACCAAT GACCTGATGG ATTGCTCGAC CAAGNCACAG AAGTGAAGTC TGTGTCTGTG 300
CACTTTCCAC AGACTGGNGT TTTTGGTNCT GAATAN 336

SEQ ID NO:2304
SEQUENCE LENGTH:363
SEQUENCE TYPE:nucleic acid
TOPOLOGY:linear
CLONE:HUMGS02663
SEQUENCE DESCRIPTION:
GATCAAGTNA CTTCTGCCCA CCTGGACACA ACACCAAAAA GGCTTCTAAA NACGGGTGTN 60
AGTCTTTTNA GCAACCCGGT CCAGTACTGG GAGATACAGC CATCCACCTT CANATGTGTC 120
TACGTGCGCT CTGCCATTCA ACTCGGAAAC TATAAGTAAT TCTNAAGAAA GCCCTCATTT 180
TTATAACCTG GCAAAATCTT GTTAATGTCA TTGCTAAAAA ATAAATAAAA GCTAGATACT 240
GGNAACCTAA CTGCAATGTG GATGTTTTAC CCACATGGCT TTATTATGCA TAANGCCAAT 300
ATTTCCTGTT TTAAGTAATN GCCTTCAATT NNNAAGNTAA TTTTGGCCTG NCCCANAAAT 360
AAA 363

SEQ ID NO:2305
SEQUENCE LENGTH:232
SEQUENCE TYPE:nucleic acid
TOPOLOGY:linear
CLONE:HUMGS02664
SEQUENCE DESCRIPTION:
GATCAGAGTT CCTCCTACTT ACAACCCAGG GTGTGAACAT GTNCTCCATT TTCAAGCTGG 60

AAGAAGTGAG CAGTGTTGGA GTGAGGGCTG TAAGGCAGGC CCATTCAGAG CTATGGTGCT 120
TGCTGGTGNC TGCCACCTTC AAGTTCTGGA CCTGGGCATG ACATCCTTTN TTTTAATGAT 180
GCCATGGCAA CTTAGAGATT GCATTTTTAT TAAAGCATTT CCTACCAGCA AA 232

SEQ ID NO:2306
SEQUENCE LENGTH:330
SEQUENCE TYPE:nucleic acid
TOPOLOGY:linear
CLONE:HUMGS02665
SEQUENCE DESCRIPTION:
GATCTTAAGT ATAATTACAA GGAAGAAGTG AAGAAGAATA TTCTCTTAGA AGAGAAGGTA 60
AAAAAACTTT CAGAACAATT GGGAGTTGAA TTAACTAGCC CTGTTGCTGC TTCTGAAGAG 120
TTTGAAGATG AAGAAGAAAG NNCTGTTAAT TTCCCCATTT ACTAAAGGTC ACCTATAAAC 180
TTTGTTTCAT TTAACTATTT ATTAACTTTA TAAGTTAAAT ATACTTGGAA ATAAGCAGTT 240
CTCCGAACTG TAGTATTTCC TTCTCACTAC CTTGGTACCT TTATACTTAG ATTGGAATTC 300
TTAATAAATA GAATTATATG AAATTTTCAN 330

SEQ ID NO:2307
SEQUENCE LENGTH:328
SEQUENCE TYPE:nucleic acid
TOPOLOGY:linear
CLONE:HUMGS02666
SEQUENCE DESCRIPTION:
GATCGACTTA CTCAACATGA GACAATTCAT ACTGGTGTGA ACCACANAAA TGCAAAGAAT 60
GTGGTAAGGC CTTTAGTCAT TGCTATCAAC TTAGTCAACA TCAAAGATTT CACCATGGTG 120
AGAGACTCTT AATGTAATGA GAGGGAAAGC CTTTAGCCAT GGAACATTTT TACTGTTGTC 180
ACTATTATNA TGCTATAGTG AAGATTAAAC TAGTTAATAT AGAATATAAA TACTTGGAAA 240
GGCATCTGGC ACATCGTATT TGCTTACTAA ATACATTATT TTTATGATAA TTGTTAGAAT 300
TACTAAAAAT AAATCTTATA GAAGGAAA 328

SEQ ID NO:2308
SEQUENCE LENGTH:327
SEQUENCE TYPE:nucleic acid
TOPOLOGY:linear
CLONE:HUMGS02667
SEQUENCE DESCRIPTION:
GATCTCAGGT ATCAGACCAC TGAGCAACCC ACCGCCAGNC TGCAGGCTTT CAGAGGCCCA 60
CCTGGGCCCA GCGTGGCCTG CCCCAGGGTG GGCTCCCAGC GCAACTGCAG GCATCCTCTA 120
GTGGGGCCTC TGGTAACCCT AGCAGATGGT GGTGACCCCC CTGAGATGAG GAAGCTGGTG 180
ACCTNNGACT GAGCAGCAGC CTATGGGCTC CGGGTCAAGT GCTATTCCCA GCGGATGCCC 240
TTCCCCTTGC GNCAGTCCCT CCTTTNCTGA GTGTCCAGGC GCNANTGNAA AACAGNAACC 300
CTAGGGTCTG GAAACTACTT TTTTTTN 327

SEQ ID NO:2309
SEQUENCE LENGTH:326

SEQUENCE TYPE:nucleic acid
TOPOLOGY:linear
CLONE:HUMGS02668
SEQUENCE DESCRIPTION:
GATCCAGTTG TTATAGTGAA CTCATGGTAA TGGTTTGTGA GAACAATAGA GATTTTNATT 60
TCTATGTAGA TGAGTTGGTA TGAGAATATA TGGAATTTTT AAGGGACTGT TTAAATCTTT 120
GATTTGTAGA CTATTAAATA TACCGTATGC ATAAAGTAAG CCTTTAGCTC TAAGGTAAAG 180
ACGACACGTT TTCGGTTTGT GACTACAAAT AGGTTAAAAA TAGATTTTAA TTTTATTAAA 240
AATATAATTT AATGCAGGTT GTTTGAAGCA TCTGTCTTCA TATGATGGCA TTAGAACACC 300
TTGGTATAAT AAAAAGTTAC CGTAAA 326

SEQ ID NO:2310
SEQUENCE LENGTH:322
SEQUENCE TYPE:nucleic acid
TOPOLOGY:linear
CLONE:HUMGS02669
SEQUENCE DESCRIPTION:
GATCAAGAAG GAGCTAGAGA CCATTTCAAA GAAAAAAAAT GCTTTATAGA GTTTTAAGTA 60
TGACTTAGAT GGGTCCAGGC AAATAAACTA AAAAGAAGTG AAGGCAACAT GTATCGTCTG 120
GCAGAACTAA ATCTTGGAGT GGGGTGAGGG ATGAAAGACT ACATATTGGG TACAGTGTAC 180
ACTGCTCGGG TGATGGGTGC GCTAAAGTCG CAGAAATCAC TAAAGAACTC ATCCATGTAA 240
CCAAACACCA CCTGTACCCC AAAAACGAAA TAAAAAAACA AAACCTTGGG GCCCATTCCC 300
CCTAGGAATG GACTACTGTA AA 322

SEQ ID NO:2311
SEQUENCE LENGTH:321
SEQUENCE TYPE:nucleic acid
TOPOLOGY:linear
CLONE:HUMGS02670
SEQUENCE DESCRIPTION:
GATCTAAATT ACCTTGGGTT TCGCATATGT CTATGAAATN CTGTGATAAN GTTTTTCAAT 60
ACATTGNTTC ACTGGCGTCT GTTTTCANTT TATACTTTTA ATAACTCATC ACTGGTGGTA 120
CTTTATCTTG AAAAGTAATA TTTTTNATAT TTTANCATTG GACAGTGTTA GCCAGTTGTA 180
ATGATGTATC AGANGTAAAG AAAAACCCAT TAAAGTTATA GCTAATAGAT GCTGTTGGGG 240
GTTAAATTAA TAGTAAANTA ATCCAATATA GCACTNTNNA TGATTTNTTA TATAAANGTC 300
AACTGGTACA TTTCATTCAG N 321

SEQ ID NO:2312
SEQUENCE LENGTH:319
SEQUENCE TYPE:nucleic acid
TOPOLOGY:linear
CLONE:HUMGS02672
SEQUENCE DESCRIPTION:
GATCGAAGGG TTTTTAGAGA ATGTNTTTCA AAACCATGCC TGGTATTTTC AACCATAAAA 60
GAAGTTTCAG TTGTCCTTAA ATTTGTATAA CGGTTTAATT CTGTCTTGTT CATTTTGAGT 120

ATTTTTAAAA AATATGTCGT AGAATTCCTT CGAAAGGCCT TCAGACACAT GCTATGTTCT 180
GTCTTCCCAA ACCCAGTCTC CTCTCCATTT TAGCCCAGTG TTTTCTTTGA GGACCCCTTA 240
ATCTTGCTTT CTTTAGAATT TTTACCCAAT TGGATTGGAA TGCAGAGGTC TCCAAACTGA 300
TTAAATATTT GAAGGGAAA 319

SEQ ID NO:2313
SEQUENCE LENGTH:327
SEQUENCE TYPE:nucleic acid
TOPOLOGY:linear
CLONE:HUMGS02673
SEQUENCE DESCRIPTION:
GATCGACAAA ACTTTGAACG AGGCTGACTG TGCCACCGTC CCGCCAGCCA TTCGCTCCTA 60
CTGATGAGAC AAGATGTGGT GATGACAGAN TCAGCTTTTG TAATTATGTA TAATAGCTCA 120
TGCATGTTTC CATGTCATAA CTGTNTTCAT ACGCTTCTGC ACTCTGGGGA AGAAGGAGTA 180
CATTGAAGGG AGATTGGCAC CTAGTGGCTG GGAGCTTGCC AGGAACCCAG TGGCCAGGGA 240
GCGTGGCACT TACCNGNGGT CCCTTGCTTC ATTCTTGTGA GATGATAAAA CTGGGCACAG 300
CTCTTAAATA AAATATAAAT GANCAAA 327

SEQ ID NO:2314
SEQUENCE LENGTH:313
SEQUENCE TYPE:nucleic acid
TOPOLOGY:linear
CLONE:HUMGS02675
SEQUENCE DESCRIPTION:
GATCAACCAC ATGCACATCC TTACTACAGA ATCCGTCCTT TCATTTCAAC ATTATAGCAA 60
GCTATGATTT TNATATATAA ATATTATATA AATNANGTAT AANACATTAA AAGTTAACTA 120
TGTAAGATAT TATNTCTGAA ACAATTTAGC TATATCCACT ATGATTATAA ACTGTGTCTC 180
GACCTGTGTT ATNTACANAA GCTGCTTAAA AAAGCATTGA GTTAATTTTN TTAAATATCA 240
ACTAAAATAT CATAGTTCTG TGGNNGACAT TGTGTTATAA TGANATAACT GCAACTAGAG 300
AAANCTGTAT AAN 313

SEQ ID NO:2315
SEQUENCE LENGTH:365
SEQUENCE TYPE:nucleic acid
TOPOLOGY:linear
CLONE:HUMGS02676
SEQUENCE DESCRIPTION:
GATCGAGGAC ATAAGGAACT AGTCACAGTG TTGCTGCAAC ATAGAGCTGA CATTAACTGT 60
NAGGACAATG AAGGCCAAAC AGCTCTACAT TATGCCTCTG CCTGTAAGTT TCTGGATATT 120
GTAGAGCTGC TGCTCCAGTC TGGTGCTGAC CCCACTCTCC GAGACCAGGA TGGCTGCCTG 180
CCAGAGGAGG TGACAGGCTG CAAAACAGTT TCTTTGGTGC TGCAGCGGCA CACAACTGGC 240
AAGGCTTAAT CAAAAGACTG GAAAACTGCA GTCTGTAATA GCATAAGGCT TCCATTATGA 300
AAGAAAACTA CAGAAATAAT ACTTCTTTTC CACCCGTCTT TGGTATGTAT TGGCTTATAA 360
AATTN 365

SEQ ID NO:2316
SEQUENCE LENGTH:308
SEQUENCE TYPE:nucleic acid
TOPOLOGY:linear
CLONE:HUMGS02677
SEQUENCE DESCRIPTION:
GATCCTGGAG CTGGCTGGCA ACGCGTGCGT NACAACAAGA AGACCAGGAT AATTCCCCGC 60
CACCTGCAGC TCGCCATCCG CAACGACGAG GAGTTAAACA AGCTGCTGGG CAAAGTGACC 120
ATCGCTTAGG GCGGCGTCCT GCCCAACATC CAGGCCGTGC TGCTGCCCAA GAAGACGGAG 180
AGTCAGAAGA CGAAGAGCAA ATNACCCTGA CGCCGNCCTC AGGGAGCTGN CTNCGNCANA 240
TAAGGCCCTT TTNATGGTCG TCCCGCAATG CTTTTGAATG TGCTGGNTGT CATGGAGGGC 300
CGGTGACN 308

SEQ ID NO:2317
SEQUENCE LENGTH:306
SEQUENCE TYPE:nucleic acid
TOPOLOGY:linear
CLONE:HUMGS02678
SEQUENCE DESCRIPTION:
GATCTAGATA ACTCATGACA TTTTATTTGA CCAACATAGC ACATGATGAG ATATCAAGGT 60
AATTAAAATA GCATGCTTGA AAAAAAAATA CGTAATCTGT TTCACCTGTA ACTGTTTAAG 120
CCAATAAACT TTTCAAAATT TATGTAATGT GGGGCTTTTA TGTAGCACTT TACGTTTTCA 180
TGCTGCTTAT TGTTTTATTC TACTGAAAAA AATGANNTTC AAGATTCTCA ACTTTNTTAA 240
TTTCAAAAAT NGTTTATGGT TTTGACTATA GGAATACAAA ANTTCCTATT TNGGGNGAAT 300
AAGAAA 306

SEQ ID NO:2318
SEQUENCE LENGTH:305
SEQUENCE TYPE:nucleic acid
TOPOLOGY:linear
CLONE:HUMGS02679
SEQUENCE DESCRIPTION:
GATCCCCTGG CTCCCCAGCA CACATTCCTT TGAAATAAGG TTTCAATATA CATCTACATA 60
CTATATATAT ATTTGGCAAC TTGTATTTGT GTGTATGTAT ATATATATAT GTTTATGTAT 120
ATATGTGATT CTGATAAAAT AGACATTGCT ATTCTGTTTT TTATATGTAA AAACAAAACA 180
AGAAAAAATA GAGAATTCTA CATACTAAAT CTCTCTCCTT TTTTAATTTT AATATTTGTN 240
ATCATTTATT TATTGGTGCT ACTGTTTATC CGTAATAATT GTGGGGAAAA GATATTAACA 300
TCAAA 305

SEQ ID NO:2319
SEQUENCE LENGTH:303
SEQUENCE TYPE:nucleic acid
TOPOLOGY:linear
CLONE:HUMGS02680
SEQUENCE DESCRIPTION:

GATCGAAATC ACTATAATTT TAACCCTNGT GATTGGAATG AATATGGTTT GTGCTGGAAG 60
CTCCGAGGTG GAAGAGACTC GTGCAATGGA GATTCTGGAA GCCCTTTGTT GTGCGAGGGT 120
GTTTTCCGAG GGGTCACTTC CTTTGGCCTT GAAAATAAAT GCGGAGACCC TCGTGGGCCT 180
GGTGTCTATA TTCTTCTCTC AAAGAAACAC CTCAACTGGA TAATTATGAC TATCAAGGGA 240
GCAGTTTAAA TAACCGTTTC CTNTCATTTA CTGTGGCTTC TTAATCTTTT CACAAATAAA 300
ATN 303

SEQ ID NO:2320
SEQUENCE LENGTH:302
SEQUENCE TYPE:nucleic acid
TOPOLOGY:linear
CLONE:HUMGS02681
SEQUENCE DESCRIPTION:
GATCCCTGCT GAATCCACAG GCCAAAATAG TCAATGTAAC TGCAAATCTA ATTTCATCCT 60
CCTTTCCTGA GGCCAACTCA GGAAATNAAA GGACGATTCT AATTTCCACT GCGGTTACTT 120
TTGTGGATGT GTCTGCACCT GCAGAGGCAG GCTTCANAGC TCCACCAGCC ATCAATGCCA 180
GGCTGCCCTT TAACTTCTTC TTCCCGTTTG TTTGACAATG CTCAGATGCA TCAGTTCCTT 240
AATATACACG TGAAATTTGA AAACTGTACA TTCGGTGAGA TTAAATTTTA TATACAACTA 300
AA 302

SEQ ID NO:2321
SEQUENCE LENGTH:301
SEQUENCE TYPE:nucleic acid
TOPOLOGY:linear
CLONE:HUMGS02682
SEQUENCE DESCRIPTION:
GATCCAATTA AAAAAAATTA AAACCAATTT AAAAAAAAAA AGANCACAGG AGATTCCAGT 60
CTACTTGAGT TAGCATAATA CAGAAGTCCC CTCTACTTNA ACTTTTACAA AAAAGTAACC 120
TGANCTAATC TGATGTNAAC CAATGTATTN ATTTCTGTGG TTCTGTTTCC TTGTNCCAAT 180
TTGACAAAAC CCACTGTTCT TGTATTGTAT TGCCCAGGGG GAGCTATCAC TGTACTTGTA 240
GAGTGGTGCT GCTTTAATTC ATAAATCACA ANTAAAAGCC AATTAGCTCT ATAACTANAA 300
A 301

SEQ ID NO:2322
SEQUENCE LENGTH:345
SEQUENCE TYPE:nucleic acid
TOPOLOGY:linear
CLONE:HUMGS02684
SEQUENCE DESCRIPTION:
GATCAAGCGG AAAGAGTTTA AGTGTCTAAC AAACTTAAAG CTACTGTAGT ACCTAAAAAG 60
TCAGTGTTGT ACATAGCATA AAAACTCTGC AGAGAAGTAT TCCCAATAAG GAAATAGCAT 120
TGAAATGTTA AATACAATTN CTGAAAGTAA TGTTTTTTTN CTATCATCTG GTATACCATT 180
GCTTTATTTT NATAAATNAT TTCCTCATTG CCATTGGAAT AGATATCTCA GATTGTGTAG 240
ATATGCTATT TAAATAATTT ANCAGGAAAT ACTGCCTGTA GAGTTAGTAT TTCTATTTNN 300
ATATAANGTT TGCACACTGA ATTGAAGAAT TGTTGGTTTT TTCCN 345

SEQ ID NO:2323
SEQUENCE LENGTH:299
SEQUENCE TYPE:nucleic acid
TOPOLOGY:linear
CLONE:HUMGS02685
SEQUENCE DESCRIPTION:
GATCTTTTCT AATATGGTAT TACAATGAAA AGAATAAAGA GAAGATTTGA ATTTNCAGTT 60
TCATTTTCAA AAACTATTTA CCAAAACAAA TGGAGAAGAA ACATCCAAAA GCACATTTCA 120
TTTCTCCAAA CTGTGTGTTT TAAATTATAG TTATAAATNG TAAGGTAATT TTAAATTGTC 180
CCTCGTATTA TTNCTCCACG CCTGTTTTAG TNTNNGGTCT CCTAAGCTTT TCTCTCATAG 240
CGTAGACCTA GGGAAGGGAT GGGAAGATTG CCCAGTCCCC GATGGCTGCG CACACAGGN 299

SEQ ID NO:2324
SEQUENCE LENGTH:297
SEQUENCE TYPE:nucleic acid
TOPOLOGY:linear
CLONE:HUMGS02686
SEQUENCE DESCRIPTION:
GATCCCTCTG TACAGGCTGG ATGGAAGGGG CCCTCCACAC TTCCTGGGAG GTCAGAGACA 60
AACTGTTTCA GAGAGTCAGA TGGACTTCCC AAGACTTGTT GAGAGATGTG ACATGGTTCT 120
TGGATTTCCT CTGTAGCAGC CTCCTGGACT TCCTGAGGAC TCGACATTGT CCACAGATGT 180
ACTGGCCATT ACATGAAACA AGAAACCAAG CATCTTTGCT GTTGTTAATT ATTATATGTG 240
CCATTGTTAC AGGAGATTAT AGGCTAGTCT GTAATAAATT ATCTTATAGC AGCCAAA 297

SEQ ID NO:2325
SEQUENCE LENGTH:297
SEQUENCE TYPE:nucleic acid
TOPOLOGY:linear
CLONE:HUMGS02687
SEQUENCE DESCRIPTION:
GATCAGCAGC CGAGAAAAGT ACATCAACAA TCAGCTTGAG AATTTGGTTC AAGAATATCG 60
TGCAGCTCAA GCCCAGCTGA GTGAGGCAAA GNAGCGATAC CAGCAGGGAA ATGGAGGNGT 120
GACGGAAAGA ACCAGACTCC TCTCTGAGGT TNNGGAAGAA TTAGAAAAGG TAAAACAAGA 180
AATGGAAGAA AAGGGCAGCA GCATGACTGA TGGTGCTCCT TTGGTGAAGA TTAANCNNNG 240
CTTNNCANAA CTGAAGCAAG AANCTGTAGN GATGGACATT AGANTTGGCA TTGTGGN 297

SEQ ID NO:2326
SEQUENCE LENGTH:297
SEQUENCE TYPE:nucleic acid
TOPOLOGY:linear
CLONE:HUMGS02688
SEQUENCE DESCRIPTION:
GATCCNACTT TAGAAGCCTA CTTTNTAACC AAGGAGCTCA ATTTTTTTTT TAAAGCTTTA 60
CTAATCTACC ANAGCATTGT AGATATTTTT TTTTAACATC TATTGTTTAA AATAGATNAT 120

TATAACGGGG CAGAGAACTT TNTTTTCTCT GCAAGAATGT TACATATTGT ATAGATAAAT 180
NAGTGNCATT TCATACCATG TATATATAGA GATGTNCTAT AAGTGTGAGA AAGTATATNC 240
TTTAATAGAT ACTGTAATTA TAAGATATTT TAAATTAAAT ATTTTTTTGT AAATAAA 297

SEQ ID NO:2327
SEQUENCE LENGTH:324
SEQUENCE TYPE:nucleic acid
TOPOLOGY:linear
CLONE:HUMGS02689
SEQUENCE DESCRIPTION:
GATCCTATGT CTGTCTGTCC ACCACGAGAT GGGAGGAGGA GAAAAAGCGG TACGATGCCT 60
TCCTGACCTC ACCGGCCTCC CCAAGGGTGC CGGCACTCTG GGTGGACTCA CGGCTGCTGG 120
GCCCCACGTC AAAGGTCAAG TAAGACGTAG GTCAAGTCCT ACGTCGGGGC CCAGACATCC 180
TGGGGTCCTG GTCTGTNAGA CAGGCTGCCC TAGAGCCCCA CCCAGTCCGG GGGGGNTGGN 240
GAGCAGTTCC AAGACCACCN CACCNCTTTT TGTAAATNTT GTTCATTNTA ANTCAAATAC 300
AGCGTNTTTT TCANTTCTGG GAAA 324

SEQ ID NO:2328
SEQUENCE LENGTH:291
SEQUENCE TYPE:nucleic acid
TOPOLOGY:linear
CLONE:HUMGS02690
SEQUENCE DESCRIPTION:
GATCTGCAGG GGTCTGGGAG TCAGGCCCGG CCTATTGCTT GGGTCTCTCT CTATTTATAT 60
ATCTAAGTTC ACAGTGTTTC TTATTCCCCC TAAGCTTCTA GAGGCTCATG GCCCTGTAGT 120
TAGGCCTGGC TCATTCTGCA CCTTTCCAGG GAGGTGGAAG GACCCTGTGC CCTCCTTCCC 180
AATCTTCTTT TTCAGGCTCG CCAAGGCCTA GGACCTATGT TGTAATTTTA CTTTTTATTT 240
CTAAAGTTGT AGTGAAGCTC TCACCCATAA TAAAGGTTGT GAATGTTCAA A 291

SEQ ID NO:2329
SEQUENCE LENGTH:290
SEQUENCE TYPE:nucleic acid
TOPOLOGY:linear
CLONE:HUMGS02691
SEQUENCE DESCRIPTION:
GATCTAGAAT CTCTTTATGT TCTCCAGAGG AAGGTGGAAG AAACCATGGG CAGGAGTAGG 60
AATTGAGTGA TAAACAATTG GGCTAATGAA GAAAACTTCT CTTATTGTTC AGTTCATCCA 120
GATTATAACT TCAATGGGAC ACTTTAGACC ATTAGACAAT TGACACTGGA TTAAACAAAT 180
TCACATAATG CCAAATACAC AATGTATTTA TAGCAACGTA TANTTTGCAA AGATGGNCTT 240
TANAAGATGC TGTGTAGCTA ANCTGAANTA ATTCATTTAC TTATTATTTN 290

SEQ ID NO:2330
SEQUENCE LENGTH:231
SEQUENCE TYPE:nucleic acid
TOPOLOGY:linear

CLONE:HUMGS02692
SEQUENCE DESCRIPTION:
GATCACTCCG CCTTNTCCTG GGCTCCCGTA GCACACTATA ACATCTGCTG AAGTGTTGCT 60
GTTGCACCAT ACTTTCTTGT ACATTTGTGT CTCCCTTCCC AACTAGACTG TAAGTGCCTT 120
GCGGTCAGGG ACTGAATCTT GCCCGTTTAT GTATGCTCCA TGNNNGGNCC ATCATCCTGC 180
TTGGNGCAAG TAGGCAGGNG CTCAATAAAT GTTTGTTGCA TGNNGGGNAA A 231

SEQ ID NO:2331
SEQUENCE LENGTH:285
SEQUENCE TYPE:nucleic acid
TOPOLOGY:linear
CLONE:HUMGS02693
SEQUENCE DESCRIPTION:
GATCGCCTAC AAGCCAGCCC CTGGCACCTG CTGCTACATC ATGAAGATAG CTGCAGAGAG 60
CATCCCCAGT CTTGAGGCTC TCAATAGAAA AGNCCACAAC TTCCAGATNG AATGCTCTCT 120
GCAGGCCAAG CCCGCAGTGC CTACGTCTAA GCTGGGCCAG GCAGAGGGGC GAGATGCAGG 180
CTCAGCACCC TCCGGAGGGG ACCCGGNCTT CCTGGGCATG GCCGTGAACA CCCTGTNTGG 240
CGAGGTGCCG CTCTACTACA TCTAGGACGN CTCCGGGTCA TGTGN 285

SEQ ID NO:2332
SEQUENCE LENGTH:285
SEQUENCE TYPE:nucleic acid
TOPOLOGY:linear
CLONE:HUMGS02694
SEQUENCE DESCRIPTION:
GATCTAGAGG TTCCANTGCT ATNCTCTCTT CTTGTCTGAG AGCCAGGACC CTGGGGGAAA 60
TAACGGGCAA AAAAGCACTT TNAAGACAGA TATAGTGGGA ATTNATAAAC GACTTGATAG 120
AAACAGAAAT GAACTGTTTT ATTTNATNCT CTGAGGACTC TGCTCATCAC CCANTAGTAC 180
CATTTCTTTT CTCAGAATGG ATTCTTTCCC CTATCTATCT AATCATTAGA GATGTAGCTA 240
NGAAATTACA TGTATGCATA TGTNTAATAT GTATATGTNT ATGTN 285

SEQ ID NO:2333
SEQUENCE LENGTH:283
SEQUENCE TYPE:nucleic acid
TOPOLOGY:linear
CLONE:HUMGS02695
SEQUENCE DESCRIPTION:
GATCTTAAAG CTTTGAGCAC CTGCCATTTT GCCTTGCATC GTTTCCCTCG TCATGCATTT 60
CCACATATCC ACAAACACAG AACGACTTTA GACAAGCACA TGTTACACCT GTGTTGCCAC 120
AAGCAGTCAT TCTTGACGGC TCCAGTTTTT ATTTGACACT TGAGTTTAGT TTTCTCTTTT 180
ATAAACCCAG TGAACTCCTG CACTGGCATT TGGATGTGTG TTAATGCTAT TTGTTTTGTC 240
TTAAAAGTAA AACCTTTCTC AGTTTGAACT TGTATTCTCT AAA 283

SEQ ID NO:2334
SEQUENCE LENGTH:283

SEQUENCE TYPE:nucleic acid
TOPOLOGY:linear
CLONE:HUMGS02696
SEQUENCE DESCRIPTION:
GATCTATTAA AAAGGTTAAG AGGCCAGGTT ACCCACCANT CCTTGCACTG TNCTGACACT 60
TTCCCCAGGA GGAAAACAAG TACAAAGGTT ACGGTGGAGG CATAAGTAGA AGAGATTGTN 120
AAGAAGGGTA ATTCATGTNT CTTTGCTCTT NCTGCTTTAT GCCTCANTTT GGTTTAAAAA 180
CTTCTNTACT GGCAAATGGT GGTATTCAGT GTGGGATAGT GTCATAACTA ATTTGACAAT 240
TTATTAATGA TAAAATAACA ATAAATGTCT AGCTTTTACA AAA 283

SEQ ID NO:2335
SEQUENCE LENGTH:283
SEQUENCE TYPE:nucleic acid
TOPOLOGY:linear
CLONE:HUMGS02697
SEQUENCE DESCRIPTION:
GATCACAAAA AAATCAGTCT TTAAGCATTT GCTTGGTAAG GTTTCTTAAN ATNAGGTTTA 60
TAATACAACC ATCTGTAATG TATCTNTCGN TTGANCTTGT GGGCCATACA ATTCATTAAC 120
TAGATGNATA CATTGTGGAC AGCATCCTCA CTACCCCTCT CTACTCACTC ACAAAGAACC 180
ATGATACACT GGAATGTTTT TNTNTGGAAT CCTCTTTCTA CTCTTGTATT AAAATTTTNC 240
CCCACTTCAT GATTATATNC AAAATAAACA ACTACATCAT AAA 283

SEQ ID NO:2336
SEQUENCE LENGTH:282
SEQUENCE TYPE:nucleic acid
TOPOLOGY:linear
CLONE:HUMGS02698
SEQUENCE DESCRIPTION:
GATCAGCTTG CAGAGTCTTG CTTTTAGGTT AGATACAAAC AAAGTAAATC ATAGTTGGTG 60
TAAATCCAGC AAAAAACAGC TGGCTTTGGA ATGGAGAACA CTACAATTCA AATTTGAAGT 120
ATATTCAGAA GAAAACTTTG GAATTAGCTT TACATTTGTT TGTAAATCTA AACAAATNTG 180
CAAAATTGGT CAAAATGTAA GTNTATNGCA TTTTTAAAGA TTANTGGTTC CTTTTATGTG 240
CTGATTTCTT TGTATTCTGT TCTCTGCATT CATCATTCAG GN 282

SEQ ID NO:2337
SEQUENCE LENGTH:284
SEQUENCE TYPE:nucleic acid
TOPOLOGY:linear
CLONE:HUMGS02699
SEQUENCE DESCRIPTION:
GATCTGTACC ACACCTTCCG GCCAGCTGTC CTCCTGCTGA TGTTCCTCAG TGTCTACAAG 60
GCCTTTGTTA TGGAGACCTT CGTCCACCTC TGCTCGCTGG GCAGTTGGGC AGCTCTACTG 120
GCCCGAGCAG TGGTAACGGG GCTGCTGGCC CTCAGCACTT TGGCCCTGTA TGTCGCCGTT 180
GTCAATGTGC ACTCCTAGGC TTGGTGTCTC AGACATTTGA TGTACCTTTT CCCTGNCTCG 240
NTCCAGGTTT TTAGTGAAGT AAACAGGTAT TTTGGAAAGT TAAA 284

SEQ ID NO:2338
SEQUENCE LENGTH:280
SEQUENCE TYPE:nucleic acid
TOPOLOGY:linear
CLONE:HUMGS02700
SEQUENCE DESCRIPTION:
GATCAACGAC AGCCTCCAGT TTCCACTTNG TGAGAGAACA AAGGAACCGG GGCATGCTCA 60
NCTATGGCTG CAAGCCNTTC AAGTGAAGCT GGGCACTTAC CCCCTTNATT CCATTCTCCT 120
CAGGATAACA GATTAAAATT CCAGAAGTGT GCACCTGTNG TNTATTCAGG AGATTNTCGT 180
TNTGCATAAA GAGACCACTT GAGGCCCTGT AAGCAAAGAC TAATTNGGTC TNAGTCAAAA 240
TCTTGGGAAT CTGATNAACT AACACACTCA AATGAAACTN 280

SEQ ID NO:2339
SEQUENCE LENGTH:285
SEQUENCE TYPE:nucleic acid
TOPOLOGY:linear
CLONE:HUMGS02701
SEQUENCE DESCRIPTION:
GATCTGGAGC AGGAAGGCCT CGAGGCACAC AGGGGCTGCT GGCCGGCGAG TGGGCCCCAC 60
CCCTCTGGGA GCTGGGCAGC CTCTTCCAGG CCTTCGTGAA GAGGGAGAGC CAGGCTTATG 120
CGTAANTTCA TAGCTTCTNC TGGCCTGGGG TGGACCCAGG ACCCCTGGGG CCTGGGTGCC 180
CTGAGTGGTG GTAAAGTGGA GCAATCCCTT NACGCTCCTT GNCCATGTTC TNAGCGGNCA 240
GNTTGGNTTT NNCCTTNAAT AAATNTGCTT TATTTNCTCT TCAAA 285

SEQ ID NO:2340
SEQUENCE LENGTH:320
SEQUENCE TYPE:nucleic acid
TOPOLOGY:linear
CLONE:HUMGS02702
SEQUENCE DESCRIPTION:
GATCGTAAAC TTGTTGATAA AGAAGATATC GACACTAGCA GCAAAGNAGG CTGTGTCCAA 60
CAGGCTACTG GCTGGAGGAA AGGGACAGGC CTGGNATATG GCCATCCTGG ATTGGCTTCA 120
TCAGAGGAGG CTGAAGGCCG GATGAGGGGC CCCAGTGTTG GAGCCTCAGG AAGAACCAGC 180
AAAAGACAGT CCAACGAGAC TTATCGAGAT GCTGTTCGAA GAGTCATGTT TNCTCGATAT 240
AAAGAACTCG ATTAAGNAAG GNGNCAAGTT CCATGGGATA CAACCTACCT CTTGTTTTGT 300
TTGTCTCTCC TTTTCTTTTN 320

SEQ ID NO:2341
SEQUENCE LENGTH:519
SEQUENCE TYPE:nucleic acid
TOPOLOGY:linear
CLONE:HUMGS02703
SEQUENCE DESCRIPTION:
GATCTGAAAG GAGCAAGGAA CAGCAAGGAA TTATTTTCCA GAATGACACC CGCAGCAGAA 60

TGTTGGAGTG GAAATGATGG CTGGCTATGA AGAGGAGGTC AACGTGTGTG GTCTCCTCAG 120
TCTCTGTCAG AGGGGTGGGG AGGTGGGAAA CAGGAATCCT CTGCAAAGCC CAATCTGCAG 180
AGTCGAGACC CCTGGTGCTC TCTGCCCCGC TGCCTGGCAC TGGTCCTTTG CAGCCAGCCA 240
CCAACGGCCC CCTTGCCCTT NCAGAGGCAG AAGCCTNCGT CTNAACCTGC ACCTCTGACC 300
GTTTNAGCAC CNTTGGGTTN TTACCANGTC CTANAACTCT GANATTTNTT NGTTCTAAAA 360
GGGGTTTCTA TTTCAACTTG NTGAGTTTGT NTTTTGGGNC CTTCCAATTT GGGNTACCTT 420
GNTATNTTTN ATGGTTTTAT AAATCCTTNA CTCTTGGNAN GTTTTAATTN TTTACAAAAT 480
CAGGGATAAC TTTNGTTTNA ANACTGGTTT GAAGGAATN 519

SEQ ID NO:2342
SEQUENCE LENGTH:272
SEQUENCE TYPE:nucleic acid
TOPOLOGY:linear
CLONE:HUMGS02704
SEQUENCE DESCRIPTION:
GATCCCAACT AATTTTACCG AACCTAAAAC CCACAAAGAG GTTGTTTGTG TTATTGTTCA 60
ATCTTCAGTT GTAAGAGTAA TTCTCTATTT TNATATTGAA ACATAATTAC TTGATAGCTC 120
AGGGTCTACA TTTCATTCAA CTTTTTACAC CAAATTCTGC AGAGTGGTCA AAATGGAATA 180
TTGGGGGCTG TTGTAAACAG AGGCTTAATT TTATTAGAAG TAGCCAGTTA TTTATTAAAG 240
CATGATGTTA ATAAAATAGG CATATTCTGA AA 272

SEQ ID NO:2343
SEQUENCE LENGTH:272
SEQUENCE TYPE:nucleic acid
TOPOLOGY:linear
CLONE:HUMGS02705
SEQUENCE DESCRIPTION:
GATCTGACAT GATTCCTTCC TGTTCTGAAC TGTGGGGTGT GCACATCTCT ACTTGAGTCA 60
GGTTTNAGTA GAGGCTTAGA GACAGTTAGG TGAGAACAAC CAAAATCTTA TCATGGTCTC 120
AGTCANNATC ATTAGGGGGA ACTCTAGCCA AATNGTTTAA CTTCTGCCTG TGGAACTGGG 180
GATTGGGTGG GCAGGAAAAG GTGATATCCA TTCTTTCTGA TAACTAGATG GTGCTGAGAA 240
GCTTTTGAAT AAAAACTTTG CTAAATGAGA AA 272

SEQ ID NO:2344
SEQUENCE LENGTH:273
SEQUENCE TYPE:nucleic acid
TOPOLOGY:linear
CLONE:HUMGS02706
SEQUENCE DESCRIPTION:
GATCTTTCCC AGGCCGCACC ATTTCTNTCA CTCACATGGA CCCAAGATAN AAGAATGGCC 60
AAACCCTCAC AACCCCTGAT GTTTGAAGAG TTCCAAGTTG AAGGGAAACA AAGAAGTGTT 120
TGATGGTGCC AGAGAGGGGC TGCTCTCCAG AAAGCTAAAA TTTAATTTCT TTTTTCCTCT 180
GAGTTCTGTA CTTCAACCAG CCTACAAGCT GGCACTNGCT AACAAATCAG AAATATGACA 240
ATTTAATGAT TAAAGACTGT GATTGCCACC AAA 273

SEQ ID NO:2345
SEQUENCE LENGTH:277
SEQUENCE TYPE:nucleic acid
TOPOLOGY:linear
CLONE:HUMGS02707
SEQUENCE DESCRIPTION:
GATCCATGAA GCTGCCCTGC CCACGCCTCC CCTCCCTGTA GCAACACCTC TGGGTGTTTG 60
GAGTTTAGCT TTTGTGGGTT TGCTCTCCCT ATCCCATCTC CTGTACTACA CAGTTCATGG 120
CAGGGTGGGG AGNNGTGGGG TTGGTTCGGG TGGGTGAGGG TCTTTTTCCT CTGTGTGCGA 180
TGTTGTTATC TGACAGNTCT CCGTCCCTAC TGGCCTTTCT CCTCGTCTTC ATATTTGTAC 240
GGTACAAGCA ATAAAGACAC TCATTTCAGA CCAGAAA 277

SEQ ID NO:2346
SEQUENCE LENGTH:431
SEQUENCE TYPE:nucleic acid
TOPOLOGY:linear
CLONE:HUMGS02708
SEQUENCE DESCRIPTION:
GATCTTNCTA GGAGGGAGAC ACTGGCCCCT CAAATCGTCC AGCGACCTTC CTCATCCACC 60
CCATCCNTCC CCANTTCATT GCACTTTNAT TAGCAGCGGA ACAAGGAGTC AGACATTTNA 120
AGATGGTGGC AGTAGAGGCT ATGGACAGGG CATGCCACGT GGGCTCATAT GGGGCTGGGA 180
GTAGTTGTTT TTCCTGGCAC TAACGTTGAG CCCCTGGAGG CACTGAAGTG CTTAGTGTAC 240
TTGGAGTATT GGGGTCTGAC CCCAAACACC TTCCAGCTCC TGTAACATAC TGGCCTGGAC 300
TGTTTTNTCT CGGCTCCCCA TGTNGTCCTG GTNNCGGTTT TNTCCACCTA NGACTGTAAA 360
CCTCTTCGAG GGCAAGGGAC CACACCCTGT AACTGTTCTG TGNCTTTTAA CAGGTTCCTN 420
CCATTAATGC N 431

SEQ ID NO:2347
SEQUENCE LENGTH:268
SEQUENCE TYPE:nucleic acid
TOPOLOGY:linear
CLONE:HUMGS02709
SEQUENCE DESCRIPTION:
GATCTTTTGT GTCTGGTTCC TTTCACGTTG AACGCTATTT TTAAGGTTCG TGCCTGTTGT 60
AGACCACAGT CACACACTGC TGTAGTCTTA CCCCATCCTC ATTCCCAGCT GCCTCCTCCT 120
ACTGTTTCCC TCTATCANNN NGCCTCCTTG GCGCAGGTTC CCTGAGCTGT GGGATTCTGC 180
ACTGGTGCTT TGGATTCCCT GATATGTTCC TTCAAATCCA CTGAGNATTA AATAAACATC 240
GNTAAAAGAA TGACCTTCCC AAGGNAAA 268

SEQ ID NO:2348
SEQUENCE LENGTH:268
SEQUENCE TYPE:nucleic acid
TOPOLOGY:linear
CLONE:HUMGS02710
SEQUENCE DESCRIPTION:

GATCAGAAAC CAGTCTCCTT CCCCGTAGTC TGGACTGGGT ATCCCCTCTT AAATNTTTCC 60
AAAGCAACAC AAATTTAATT CCGTCACAGA ACTTGCCACT CTGTGCTGTG TCCTCTGTTT 120
CCTTGCCATC TTCCTCAATN AGCTGGAAGC CCATTGGCAG CAGGGACTGT TTCTTGTTCA 180
CAGCTGCACC TCAGTGCTAA GCATAGTGCC TGGTAGGTAA TAGACTCCTT AAGTATTTGT 240
TGAATTAATA AAGGATGNNT TTTTTAAA 268

SEQ ID NO:2349
SEQUENCE LENGTH:268
SEQUENCE TYPE:nucleic acid
TOPOLOGY:linear
CLONE:HUMGS02711
SEQUENCE DESCRIPTION:
GATCAGAAGC CATTTNATTT GGTAGGTGTG TCAGAAGGGA GAATGATGGN AGACGAACTG 60
CTGGAAGAGG TCAGAAGATA GCCATGCTAA AATGCAATTA TATCCTCATG TTTATCCCAA 120
ACTAATCTTG GACTTTTCCA CTCATTAGCT TTGTTNTGCC CTTGTTTCCC TTGAAGGTTT 180
AAGTTCAACC ATATTCTGTC AACTGTTCAG TTTCAGTGGA ATCTTGTATT TCTGGTTCAT 240
TATAACAAAC TGTTCGCTTA AATCCAAA 268

SEQ ID NO:2350
SEQUENCE LENGTH:398
SEQUENCE TYPE:nucleic acid
TOPOLOGY:linear
CLONE:HUMGS02712
SEQUENCE DESCRIPTION:
GATCCGAGAA CGCTCTAAGC CTGTCCACGA GCTCAATAGG GAAGCCTGTA ATGACTACAG 60
ACTTTGCGAA CGCTACGCCA TGGTTTATGG ATACAATNCT GCCTATAATC GCTACTTCAG 120
GAAGCGCCGA GGGGCCAAAT NAGACTGAGG GAAGAAAAAA AATCTCTTTT TTTCTGGAGG 180
CTGGCACCTG ATTTTGTATC CCCCTGTAGC AGCATTACTG AAATACATAG GCTTATATAC 240
AATGCTTCTT TCCTGTATAT TCTCTTGTCT GGCTGCACCC CTTTTTCCNG NCCCCAGATT 300
GNTAAGTAAT NGAAAGTGCA CTGCAGTGAG GGTCAAAGGN GAGGCAACAT ATTNTNNTTT 360
TCCATAATAA AACTTTCTTG GGGGNNATAC TTTTCAAA 398

SEQ ID NO:2351
SEQUENCE LENGTH:381
SEQUENCE TYPE:nucleic acid
TOPOLOGY:linear
CLONE:HUMGS02713
SEQUENCE DESCRIPTION:
GATCTGCAAG CATTTGCTCT TGTTTCCACA TCACCTCTGG GATATTTCAG CTGTTGTTTC 60
CAAATGGCAA ATCATCAACT AAANGCACTT GTTTCAAGTT TTGTTCTGCA CTCCCACGAC 120
TGAAGTTGTA GATTGAGCTG AATAACCATG GGAAGTGANC AAGCAAAGAC ACTCGATTGG 180
AGTCAGTTGA ATATTTGTAC CCTCAGTGGA GCCCTTCTGG TCTTTTCTTC CACTTCTGCA 240
GAATTTCCTC TAGCAAATAC TTCTTTCTCC TTGCTTGCCT CCACCATGAT ATTTGAATAA 300
GAGATGGCCA GAGGATAACA CTTGTCTCTT AAAAACTAAG CTAAAAAGGA CCTAGGAACC 360
TTCAATTTGA GCAGTTGGTT N 381

SEQ ID NO:2352
SEQUENCE LENGTH:265
SEQUENCE TYPE:nucleic acid
TOPOLOGY:linear
CLONE:HUMGS02714
SEQUENCE DESCRIPTION:
GATCTGCCCG CCTTGGCCTC TCAAAGTGTT GGGATTGCAG GCGTGACACG TGCTCCTGGC 60
TTGAATTTTC TTATAATCCC ATGAAACCAT CATCAGAGTC ACAATAATNA ATTTATCTAA 120
TCTATCACCT TTGTATTCAC CTGGAGCCTT TATAAAGTTT TTNTCTATGC AGACAACCAT 180
TGATTTACTC TGCTGTATAA TACTTTCCAT TTTCTAGAAT TTTCTGACTG ATGTAATAAA 240
GTGTTTCCTC TTATAATCCC TGAAA 265

SEQ ID NO:2353
SEQUENCE LENGTH:265
SEQUENCE TYPE:nucleic acid
TOPOLOGY:linear
CLONE:HUMGS02715
SEQUENCE DESCRIPTION:
GATCGGGGGC TTGAACCCAG GTGGTCAGGC TCTGGAGCCC ACAATTGTNT TACCCACTAT 60
GCCCCTCTCT AGTCATGGTC CCCAAGAGGG GCTTGGAGAC CCACTTAGCA GGTGAAAGCA 120
ATGGCAGCCT TCCTTATTTG ATTATGCACC TAAGAATAAA TGGTATTTGG GCATGTATTC 180
CCAATATGTG TATATTTATT TATAAATATA TACAGATACT ATNATCTGTA TGTNAGTAAT 240
AAAGCTTAAA TTATTCCATT TTAAA 265

SEQ ID NO:2354
SEQUENCE LENGTH:379
SEQUENCE TYPE:nucleic acid
TOPOLOGY:linear
CLONE:HUMGS02716
SEQUENCE DESCRIPTION:
GATCGGCGGA CGCCCATCAC CGTGGTGAAG CAAGGCTTTG AGCCTCCCTC CTTTGTGGGC 60
TGGTTCCTTG GCTGGGATGA TGATTACTGG TCTGTGGACC CCTTGGACAG GGCCATGGCT 120
GAGCTGGCTG CCTGAGGAGG GGCAGGGCCC ACCCATGTCA CCGGTCAGTG CCTTTTGGAA 180
CTGTCCTTCC CTCAAAGAGG CCTTAGAGCG AGCAGAGCAG CTCTGCTATG AGTGTGTGTG 240
TGTGTGTGTG TTGTTTCTTT TTTTTNTTTN NNNAGTNTCC AAAAANAGCC CTNAAAAAAT 300
TCAGGGNCCT TGAAAAATTN TNTAAAATGC CAGNNTTTGG GGAAATNAAA CCCANTAAAA 360
ACNTTTTGAG GGGTGNAAA 379

SEQ ID NO:2355
SEQUENCE LENGTH:261
SEQUENCE TYPE:nucleic acid
TOPOLOGY:linear
CLONE:HUMGS02717
SEQUENCE DESCRIPTION:

GATCACAAAA CAATTCTAAA ACCTAACTGT TTTTACCATT GAAATTTAAA TTGTGATAAT 60
AGGTTTTAAA TGTCTAGAAT GCAACTGATA GGCTTTTCTT GAACTGTTAG TTTTTTTGAA 120
GTAGTTTTTT CATGTTTANN NNGTATTTGT AAAAAAACAA AAAGCAAAAA AATTCCCAAA 180
ACCCAGATAA CAACCAGAGC AAAACTGTTG TGCCTTCTAT TTATCTTTGA TTTCAGTCTT 240
GGCAATTGTT TAAAAAAAAA N 261

SEQ ID NO:2356
SEQUENCE LENGTH:259
SEQUENCE TYPE:nucleic acid
TOPOLOGY:linear
CLONE:HUMGS02718
SEQUENCE DESCRIPTION:
GATCATTGGA GAGAGAATTG CACAGAAAGT CCTGAAGTTT AAAACACTTT ATGACCAGCT 60
TTGGCTCGGG AGAGTGGGGC TGCTTGTAGA ACTGGAAGTG AATAACTTTT TCAAGCAATA 120
TCAGTGAGTG GGTCCCATCG ACAGGGTTCC AGGACCTGGA ACACTTTAAC AGAAGGAAAT 180
GCCGAAGCAG CTTGCACAGT NGCTTGACAG ACTTCCAAGN NGGCTGATTC TGGCTTCAAG 240
ATGGAGCCTT GGAGTTGGN 259

SEQ ID NO:2357
SEQUENCE LENGTH:296
SEQUENCE TYPE:nucleic acid
TOPOLOGY:linear
CLONE:HUMGS02719
SEQUENCE DESCRIPTION:
GATCTGGGAG TACACCAGGC AGCAGCTGCT CTCGNACGGA GATACCAAGC CATGATGTGC 60
CNNNNTTAGT GAACTGCTGC TGCTGTTCAG ACTTTTTTAA AAAAAACTAT TTGGACTAAA 120
GAAACAGATT CTGAAATTTA TTGTGATAAT TTGTATTTCT TTTTTCTTGC AATTTAATGC 180
CAAAAGTTTG CCATGTGCCT TAAACATATT ACTATATATT TNCCCCTTTA ATAAACACTT 240
TTNGTTAAAT TGTATTCTTC CTTTAATAAA ATATTTTAAG CAATTGTGGA AATAAA 296

SEQ ID NO:2358
SEQUENCE LENGTH:254
SEQUENCE TYPE:nucleic acid
TOPOLOGY:linear
CLONE:HUMGS02721
SEQUENCE DESCRIPTION:
GATCAGAATA CATGATGGAT GAAATTCTTT ACATGTTTTA GCAGAATNAA TTTGTTTAAT 60
ATAATAAAGT TTGCTACTTA TCTGTATGTA GGTTGCTAAA AANGATTTTC TTAACTCAGA 120
TTTTAAGCCA AATAACCATT TAACACTAGT ATTTGTTAAA TGGGTTATTT TNCTGTATTT 180
GTATGTTTCA CTATAATAAG GGAATTAAGG ATAATGTGCA TTGAGAATAT TTTGAAAANT 240
AATTGACTCA AATN 254

SEQ ID NO:2359
SEQUENCE LENGTH:253
SEQUENCE TYPE:nucleic acid

TOPOLOGY:linear
CLONE:HUMGS02722
SEQUENCE DESCRIPTION:
GATCAGTTAT AAGTAGCAGG CCAAGTCAGG CCCTTATTTT CAAGAAACTG AGGAATTTTC 60
TTTGTGTAGC TTTGCTCTTT GGTAGAAAAG GCTAGGTACA CAGCTCTAGA CACTGCCACA 120
CAGGGTCTGC AAGGTCTTTG GTTCAGCTAA GCTAGGAATG AAATCCTGCT TCAGTGTATG 180
GNAATAAATG TATCATAGNA ATGTAACTTT TTGTAAGNCA AAGGTTTTCC TCTTCTATTT 240
TGTAAACTCA AAN 253

SEQ ID NO:2360
SEQUENCE LENGTH:253
SEQUENCE TYPE:nucleic acid
TOPOLOGY:linear
CLONE:HUMGS02723
SEQUENCE DESCRIPTION:
GATCAATCCC AAGAATCCAT CAGCAACCTC AGACCAACCC AAGAAGATAA TTTAAATCTA 60
TACTGCTTAT TGGTCAATAT ATTTGGTTCT AGTATTAATA AAGAAAAATG TTATTAAAAT 120
AGCATACATA GTAGTAAAAT AAANTACANA AAGTGTGTTG ATTTATAGCT GTTTGAGATG 180
ATAAAAGTGN AGCAAAGCCT GTTAAATCAT TGGAAGACTT GGANANTTAT TTTAAATAAN 240
CAATTACATG TAN 253

SEQ ID NO:2361
SEQUENCE LENGTH:252
SEQUENCE TYPE:nucleic acid
TOPOLOGY:linear
CLONE:HUMGS02724
SEQUENCE DESCRIPTION:
GATCTGCATA AACAGGGTTC CTGACCTCAC CGAAAAGACT AATGTGCCTT AGAACAAGCA 60
TTTGCTGTGT TTTGTTAGCA CCTGGTTCCA GGACAGACCC TCAGCTTCCC AAGAGGATAC 120
TGCTGCCAAG AAGTTGCTCT GAAGTCAGTT TCTATCGTTC TGCTCTTTGA TTCAAAGCAC 180
TGTTTCTNTC ACTGGGCCTC CAACCATGTT CCCTTCTTCT TAGCACCACA AATAATCAAA 240
ACCCNACATA AA 252

SEQ ID NO:2362
SEQUENCE LENGTH:251
SEQUENCE TYPE:nucleic acid
TOPOLOGY:linear
CLONE:HUMGS02725
SEQUENCE DESCRIPTION:
GATCTTCTCA GCCAGGAGAA GAGGGTGGTG TTTTCACGCG GGCAACTGCT CGCCGGCCTA 60
CATGGGGTTA ATTCAAGTCT GCTGCGAGCA CGACTCCGCC CTTGGCACTG GCCTCCAGCA 120
AGCCCTGTTC TCTTTGGGGT ACAGGGGAAC GGGATGGTTT AGACTTTCCT GCTCAGTGTG 180
TAAAAAATGT AGCTAAAGCC ACTATTTTTG CTCTCCTTAA GCTGTTCAAT AAACCGGTTC 240
CTCATTTTAA A 251

SEQ ID NO:2363
SEQUENCE LENGTH:308
SEQUENCE TYPE:nucleic acid
TOPOLOGY:linear
CLONE:HUMGS02727
SEQUENCE DESCRIPTION:
GATCTGGAAC ACTCCCCTGA CCCCTTCACA CCTGGCCCTC CCTGGGCTTA AAGCTCCTGA 60
TATTCCTCAT CCCCTTCCTT GTTTTCCAGA ATCGGTCGAG GTGGACGGTT TGGCCGTAAA 120
GGTGTGGCTA TTAACATGGT GACAGAAGAA GACAAGAGGA CTCTTCGAGA CATTGAGACC 180
TTCTACAACA CCTCCATTGA GGAAATGCCC CTCAATGTTG CTGACCTCAT CTGAGGGGCT 240
GTNCTGCCAC CNAGCCNCAG NCAGGGCTCA ATCTCTGGGG GGCTGAGGGA GCAGCAGGGA 300
GGGGTGGN 308

SEQ ID NO:2364
SEQUENCE LENGTH:245
SEQUENCE TYPE:nucleic acid
TOPOLOGY:linear
CLONE:HUMGS02728
SEQUENCE DESCRIPTION:
GATCGTAGAT TTTTAACAAT CTGTATCTTT AACAATTCTG GGTGCNAGTG TNAGAGTGTG 60
AGCAGGGCTT GCTCCTGCCA ACCACAATTC AATGAATCCC CGACCCCCCT ACCCCATGCT 120
GTACTTGTGG TTCTCTTTTT GTATTTTGCA TCTNACCCCG GGGGGCTGGG ACAGATTGGC 180
AATGGGCCGT CCCCTCTCCC CTTGGTTCTG CACTGTTGCC AATAAAAAGC TCTTAAAAAC 240
GCAAA 245

SEQ ID NO:2365
SEQUENCE LENGTH:249
SEQUENCE TYPE:nucleic acid
TOPOLOGY:linear
CLONE:HUMGS02729
SEQUENCE DESCRIPTION:
GATCTAACCA ACTCAACTGA TAGAAAGCTG AATTGTTCCC AGTGGACAAG TGGTATTTGA 60
TGTTTAGCAG GTTTTTTTGT TGTTATTTTT TTGGTAAACT CTGTGGTAAA ATCTAAAATA 120
ATGTTAAAAC CCTAAAAGTA ACTATTGANT ATNCTGTAAT CTTAGACATG TATTTTTCAA 180
GCTAACCCTT TGAAGAAATG TGTTCAAGGT AGGAAAATAT AAAATTTTTT GGTTATTTGT 240
AAATTTAAA 249

SEQ ID NO:2366
SEQUENCE LENGTH:240
SEQUENCE TYPE:nucleic acid
TOPOLOGY:linear
CLONE:HUMGS02730
SEQUENCE DESCRIPTION:
GATCATGCAT TGTTGAGGTG GTCTGAATGT TCTGACATTA ACAGTTTTCC ATGAAAACGT 60
TTTATTGTGT TTTTAATTTA TTTATTAAGA TGGATTCTCA GATATTTATA TTTTAATTTN 120

ATTTTTTTCT ACCTTGAGGT CTTTTGACAT GTGGAAAGTG AATTTGAATG AAAAATTTAA 180
GCATTGTTTG CTTATTGTTC CAAGACATTG TCAATAAAAG CATTTAAGTT GAATGCGAAA 240

SEQ ID NO:2367
SEQUENCE LENGTH:251
SEQUENCE TYPE:nucleic acid
TOPOLOGY:linear
CLONE:HUMGS02731
SEQUENCE DESCRIPTION:
GATCTATACT GTGTGGTCAT GAGTTGTGTA TAGTTCCATA ACACTGTATT TTNCTNCTGT 60
CAGTACCCTT AGGATACACT NTAAAACACC TTAAGGTCTG ATGTTATGGC AACAAACTAC 120
TTNTTCAAAC CTAAATAGGA ACCATGTAAT TNCTCAAAAG TGATTGAACA GTTTGCCCAC 180
ACTTAGTTTG TTGGTCTTAT GTAAAACATT GGCTCAAAAT AAAGNNCACA CTGATTTATT 240
TNACTGNNAA A 251

SEQ ID NO:2368
SEQUENCE LENGTH:239
SEQUENCE TYPE:nucleic acid
TOPOLOGY:linear
CLONE:HUMGS02732
SEQUENCE DESCRIPTION:
GATCTGAAAA TTTATTTCAC CCAGCTTTCA CAATTAAGTC TGTATAAAAT GTGGTACCTT 60
TTNGTACCTA TCCAGAGAAA CCTGTACATA TTCAATGTTA TCTAGATGTG ATTGGATAAT 120
GGAGAAAATN TATTTATAAT GATTTTNCAA TGGCATCAAT AACATTTAAA TGATTTTGAA 180
GAAGTATTAT TTTACTACCC TATAAAATGA ATCGAAAATA ATAAAGTTCT TAACATAAA 239

SEQ ID NO:2369
SEQUENCE LENGTH:239
SEQUENCE TYPE:nucleic acid
TOPOLOGY:linear
CLONE:HUMGS02733
SEQUENCE DESCRIPTION:
GATCAAATTA TCTTCAGCAT GTATATCTGG GGAAAAAAGG TCCGAATTTT CACATTTATA 60
TTTAAANTTC AATTTTTTAT ATTTAAANTT CAATTTTTTA GCAACAGCTG AATAGCTTTG 120
CGGAGGAGTT TAATAGTTAC ACATTCATGC TAATATACAT TNCCTTTAAA CATCCACAAA 180
TTCTTAAAAA GATTGAATCA GTANATTTCA TTTCAGCTAA AAATGGAGTC TAATATATN 239

SEQ ID NO:2370
SEQUENCE LENGTH:237
SEQUENCE TYPE:nucleic acid
TOPOLOGY:linear
CLONE:HUMGS02734
SEQUENCE DESCRIPTION:
GATCCACCCG CCTTGGCCTC CCAGAGTGCT GTGATTACAG CTGTGAGCCT CCGTGCCCAG 60
GCCAGTGTTC TCTCTTAAAT GTGTTTCAAT ATGGTGGACA TTTATTGAGC ACTTGTGTTG 120

GGCGTTTCAA CAGGAGGCAC TGGTACGAAA AGTAGCTTGT TCTAGTTCAT ATATCATTTG 180
CACTTTGCTT AACTTGTATA AGTACTTTTT ACTGTTTAAT AAATATTTTA TTAGAAA 237

SEQ ID NO:2371
SEQUENCE LENGTH:236
SEQUENCE TYPE:nucleic acid
TOPOLOGY:linear
CLONE:HUMGS02735
SEQUENCE DESCRIPTION:
GATCAAAAGA GCAAACAATA CTTTCTATGG CTTATCAGCA GGAGTGTTTA CCAAAGACAT 60
TGATAAAANCC ATAACAATCT CCTCTGCTCT GCAGGCAGGA ACANTNTGGG NGAATTGCTA 120
TGGCGTGGTA AGTNCCCAGT GCCCCTTTGG TGGATTCAAG ATGTCTGGAA ATGGNAGAGA 180
ACTGGGAGAG TACGGTTTCC ATGAATATAC AGNGGTCAAA ACAGTCACAG TGAAAN 236

SEQ ID NO:2372
SEQUENCE LENGTH:236
SEQUENCE TYPE:nucleic acid
TOPOLOGY:linear
CLONE:HUMGS02736
SEQUENCE DESCRIPTION:
GATCCNATTC CCGTTGCCTC AAAGCACCAG CTCTCTCGCT CCATCCTCCC AAGAGCCTCC 60
AAGGCGCCAT TGGCTTCCTG GGACTCTGAG TCTGCCGGCC TCTTTTCCTG ACTCCAGTTT 120
CTTCTGGCCT GAGCCAATGG GGCATGTTCT GTGACTGCAT TTGCATCTCG GATTGTGTTT 180
TTAAACCCAT TTTCTCCCAT ATTTGCCTGA AAAATATATA GAAACTGATA TACAAA 236

SEQ ID NO:2373
SEQUENCE LENGTH:245
SEQUENCE TYPE:nucleic acid
TOPOLOGY:linear
CLONE:HUMGS02737
SEQUENCE DESCRIPTION:
GATCAGGCTG GTACAGGATG CCTTAAGGTG ATGAGAGGTG AGGGTGCATG AAGAATAATG 60
AGCACAGGGA AGAGAGAAGC AGGACAAAGT AGCAGATAAA ATGCCGGCAA AGCACAGATG 120
ANTGTTTTCA AGAAGCTCTT GTATTTNTNT GCACAGTGTA AATATCCTTG CTATTTCAGG 180
ATGGCGGCTG GCCTGCTCAG TAACATACAT GTTCCAAATA AAGATTTTGC ATGAAAGTAC 240
CTAAA 245

SEQ ID NO:2374
SEQUENCE LENGTH:231
SEQUENCE TYPE:nucleic acid
TOPOLOGY:linear
CLONE:HUMGS02738
SEQUENCE DESCRIPTION:
GATCTGACTG CCTCCTTTCT CTGGCCTCTN CCCCCTNCCC TCTTCTCTTC AGCTAGGCTA 60
GCTGGTTTGG AGTAGAATGG CAACTAATTC TAATTTTAAT TTATTAAATA TTTGGGGTTT 120

TGGTTTTAAA GCCAGAATTA CGGCTAGCAC CTAGCATTTC AGCAGAGGGA CCATTTTAGA 180
CCAAAATGTA CTGTTAATGG GTTTTTTTTT AAAATTAAAN GATTAAATAA A 231

SEQ ID NO:2375
SEQUENCE LENGTH:236
SEQUENCE TYPE:nucleic acid
TOPOLOGY:linear
CLONE:HUMGS02739
SEQUENCE DESCRIPTION:
GATCCACTGT GAGCACCAAC CTGCTGCAGT CTCTTGGGCC CCTGCTGGCA GCTCTGCCAC 60
GTCACCGNCT GCCTGGCTCC CACACAGCCA TGCATTGTCA CTCTGCCTCC GGGACCCNAG 120
CTTNGGAGCN NNTGGGTCTG CCAGGTCCCA CCTCCTCTGT CCCCNATGCC ACAACCTGGG 180
CTCCTGGCTA CAGCAGGGNT CCAGGGACTN CAAATAAATG TTCAGTGACT GGCAAA 236

SEQ ID NO:2376
SEQUENCE LENGTH:235
SEQUENCE TYPE:nucleic acid
TOPOLOGY:linear
CLONE:HUMGS02740
SEQUENCE DESCRIPTION:
GATCGGTCAC ACAAGCTGTT CCACGATAAT TNTTGTNTCA NAAGGGGAGN CTGACTACCA 60
GGACCACTGG CCCCACTTCC AGCCCCTCCA TTCTNAGTAC ACTTCAGAGG GCTCTGGCAG 120
GGTGAGGGGG TCTTGNCCGT GCCACAGAGA GTAAAGGTCT AGCACACCCA TTGTGGAGAT 180
ATTTCATGGG GACANCCCAG TGACTTCACT TTGAAGTAAA GTGGGATTTT CCAAA 235

SEQ ID NO:2377
SEQUENCE LENGTH:253
SEQUENCE TYPE:nucleic acid
TOPOLOGY:linear
CLONE:HUMGS02741
SEQUENCE DESCRIPTION:
GATCGNAGGC TACCCTGGCT CAGTNACCAG CTGCTCACTG TGTCCTTCTG TGGTCTCAGG 60
AGCTGCAGTT GTTGCTGTTG TATGAATAGA CGATAGTNAA TTAGACTGGA TGTGTCTGTA 120
CTGGCAGTNA TTTTCCAACT NCCTCTGTAT AAGCAATACA TTTGTAAATC ATGGGAATGT 180
ATTTAATGGT AATTGCCTGT GGTGGTTGTA TCATGATTTA NTNNTATNAT GAGCTTTTGG 240
CTTTNTNTAT NTN 253

SEQ ID NO:2378
SEQUENCE LENGTH:232
SEQUENCE TYPE:nucleic acid
TOPOLOGY:linear
CLONE:HUMGS02742
SEQUENCE DESCRIPTION:
GATCTGGGGT CCCAGTGTCA TTGGTGAAGG CCTTGGGATT CGAGGCAGCT GAGTCCTCAC 60
TGACCAAGGA TGCCCTTGTG CTTACTCCAG CCTCCTTGTG GAAACCCAGC TCTCCTGTCT 120

CCCAGTGAAG ACTTGGATGG CAGCCATCAG GGAAGGCTGG GTCCCAGCTG GGAGTATGGG 180
TGTGAGCTCT ATAGACCATC CCTCTCTGCA ATCAATAAAC ACTTGCCTGA AA 232

SEQ ID NO:2379
SEQUENCE LENGTH:232
SEQUENCE TYPE:nucleic acid
TOPOLOGY:linear
CLONE:HUMGS02743
SEQUENCE DESCRIPTION:
GATCTCTGAA AAGNAGGTTT CAGCCACGAG GCAGCTGNTC CCAGGACACT GAGGCCAAGA 60
GAAATGTAAC AGAGCCACAG CTCCACAGGC CTGCACTCGG AGTCTGGGGC CTCTGCAGAG 120
CCAGCAAGGG GAAAAGTATA ATCTGGGGGA CCTTCAACCA CTAAGCCTCT TGTCAGAGCC 180
CTCAGGCAGG CAGATGTGTC ACCCAAATAA ACAGTGATAT TGTCTCCAGA AA 232

SEQ ID NO:2380
SEQUENCE LENGTH:232
SEQUENCE TYPE:nucleic acid
TOPOLOGY:linear
CLONE:HUMGS02744
SEQUENCE DESCRIPTION:
GATCTNAGCT GGACAGAGGA GGAGGAAGAG GAGGAAGCAA TGGGGGAATA GTGACACCAG 60
ACAGTTGATG TCTAGATAGG ACCTCAATNA TTCCNTTAGA ATCTNAGATA CCAGGATATT 120
GTTGGCCATG TGGCATCATT GAGCAGCAGG AGGCTGAAGG AGGNGAGAAC AAAATTGTCC 180
AAACCATGCT GTTTTTTTCC CTTAAATAAA TCTTGTATTC TTCAGTTTCA AA 232

SEQ ID NO:2381
SEQUENCE LENGTH:236
SEQUENCE TYPE:nucleic acid
TOPOLOGY:linear
CLONE:HUMGS02745
SEQUENCE DESCRIPTION:
GATCTANGGA ACCAAACGGA AGGTTTTGAG GTGATGTAGG TCTTTCACAG TTAGCTTTGG 60
GGAATACAGA ATACTCAAAT AAAGTGCTTT GTTATTATTT CAGAGGGAAT GGCGATTGAA 120
ATGTTACAAC AGAGATTTCT TGGTGGTAGC TATTTNGGTA AAGGGTATAT GGNTATTTTT 180
CTGTACATGT GAAATTATAT AGANNTAAAA GTTATATANA TTACATTGGC CAGAAA 236

SEQ ID NO:2382
SEQUENCE LENGTH:229
SEQUENCE TYPE:nucleic acid
TOPOLOGY:linear
CLONE:HUMGS02746
SEQUENCE DESCRIPTION:
GATCAAGTTT AAGTCAGTCA GTTAAGGGGA GGAGAAGGAG AGGTTATACN TTCAGGGGGC 60
TACCAGACAG TGTTCTCAAC TTGGTTAAGG AGGAAGAAAA CCCAGTCAAT GAAATTCAAT 120
GAAATTCTTG GAAACTTCCA TTAAGTGTGT AGATTGAGCA GGTAGTAATT GCATGCAGTT 180

TGTACATTAG TGCATTAAAA GATGAATTAT TGAGTNCTTA AAGATTAAA 229

SEQ ID NO:2383
SEQUENCE LENGTH:227
SEQUENCE TYPE:nucleic acid
TOPOLOGY:linear
CLONE:HUMGS02747
SEQUENCE DESCRIPTION:
GATCTGATTT CAGATTTTTC CTGTATCTTT TTTACATACA TCAGAAAAAG AAATGTTTAC 60
TATATTTTTG GTTCCATTNA TGATTGTTTT AAGCATTTGA CGNATAAGGA AAACTAACAA 120
TTAANTCAAT TAGAAAAGCA ACATAAAATT AANTGATATT TAGGAAATCA GTTATATGTG 180
AGCTTGGGTA TTCAAATGTC ACAANTAAAA GGCNTANACC CATNAAA 227

SEQ ID NO:2384
SEQUENCE LENGTH:223
SEQUENCE TYPE:nucleic acid
TOPOLOGY:linear
CLONE:HUMGS02749
SEQUENCE DESCRIPTION:
GATCAAATNA CCTGTGAAGG AATATTTTGC CTTCATCCTA GCTGCTGGGG AAGCGGGGAG 60
AGGGGTCAGG NAGGCTAATG GTTGCTTTCC TGAATGTTTC TGGGGTACCA ATACGAGTNC 120
CCATAGGGNC TGCTCCCTCA AAAAGGGAGG GGACAGATGG GNAGCTTTTN TNACCTATTC 180
AAGGAATACG TGCCTTTTCC TTAAANNCTT TCATTGATTG AAA 223

SEQ ID NO:2385
SEQUENCE LENGTH:221
SEQUENCE TYPE:nucleic acid
TOPOLOGY:linear
CLONE:HUMGS02750
SEQUENCE DESCRIPTION:
GATCAGGGTT TNGGGTCTGT GTTCTTTCTA CCGTCAGCAC CTGTGTGGTC AATTCTGGAC 60
ACTTCCCAGA GAAGTCTTTG AGTAGAGAAT CCTACTCAAA TTTCACTGTA TATNTTAAGC 120
ATTCCTCTCC TNTCCCNTTG CCTNCCCTGT TGCCTTNTCT TCCCCTGATT TCTCCTCTGG 180
TCATCTNCTC TCCCTNCTGC GTGTAAGCCA TGGGAAAGGG N 221

SEQ ID NO:2386
SEQUENCE LENGTH:220
SEQUENCE TYPE:nucleic acid
TOPOLOGY:linear
CLONE:HUMGS02751
SEQUENCE DESCRIPTION:
GATCATGAAA ACAATGTAGG TAAAGTAAAG TATGTATTTG TCTGTGCTTG AAAAATATTG 60
TAAAATGTTA TGTATTTGGA ATATATTTTG TGGTTTGTCT AATATTTATA AACACAACTC 120
GGGGTGAATT CACANTGAGT TTATTTCATT GAAATACTAG AGATATGGGG GCCATGTTAC 180
TGTGCTTGGN GCCTTACCNT TTGTATAGTG AAATTTGCAN 220

SEQ ID NO:2387
SEQUENCE LENGTH:220
SEQUENCE TYPE:nucleic acid
TOPOLOGY:linear
CLONE:HUMGS02752
SEQUENCE DESCRIPTION:
GATCCGCCTG CCTTGGCCTC CCGAAGTGCT GGAATTACAG GCATGAGCCA CTGCTCCTGG 60
CCTAAGATGT TTTTTAAATA ATGTTTTTAA CTAAATTTTA AGAATGTTTT TAACTAAAGT 120
TATTTATATT TGGAATAATT CGTTAATGAT TTGTGTCATT TCCCCAACCC CTTTTTAAAT 180
TTGTGAATTT GAGGACTGGA TTAAAAGATA AAAAGTTAAA 220

SEQ ID NO:2388
SEQUENCE LENGTH:339
SEQUENCE TYPE:nucleic acid
TOPOLOGY:linear
CLONE:HUMGS02753
SEQUENCE DESCRIPTION:
GATCTAATTA ATAGCTGACC TCCCAAATCT GACAGGATAG ACACTGCCAC GTGCAAGGCC 60
TGCCAGCCCC TCAGACGCAC AAAATGCGTA AAACAAATGC ATCCTTTCCT GGCTAAGCGA 120
GTATTACTCT CTTAGCCCTG CACCAAACCT CCAATCTAGC CACATTTAAC TNTTCATTTC 180
TTAGACCCGC AGAGTGTCTT CCTGCCTCTG AGCTGTGAGT GTTGTTCCCT TTGCCCGGGA 240
TGCTCTTGTT TTTAATACCA GTTCAAGTCC CACTCTCTCA GTGAAGCACT CCCTTCCCCA 300
CTNATAGCCT TTAGTGAACC CTCGTTTCTT GCTTCTTTN 339

SEQ ID NO:2389
SEQUENCE LENGTH:219
SEQUENCE TYPE:nucleic acid
TOPOLOGY:linear
CLONE:HUMGS02754
SEQUENCE DESCRIPTION:
GATCCATGTG CAGTAATGCC TGGTGGCTCC AGGTCTGCCC CGCCGTCCTG TGGGGCTGTG 60
AGCTTTCCCA GCCTCCTGCC CGTGTTTGTG AATATCATTC TGTCCTCAGC TGCATTTCCA 120
GCCCAGGCTG TTTGGCGCTG CCCAGGAATG GTATNAATTC CCCTGTTTCT CTTGTAGCCA 180
GTTACTAGAA TAAAATCATC TACTTTAAAA TCTTTCAAA 219

SEQ ID NO:2390
SEQUENCE LENGTH:219
SEQUENCE TYPE:nucleic acid
TOPOLOGY:linear
CLONE:HUMGS02755
SEQUENCE DESCRIPTION:
GATCTTGACC GGGAAAAGGG ACTTGAGAGA ACCTTCTAGG ATAATTTNAT TTCTTGATTA 60
GGTGGTGGTT ACATTTGTAA AAATCATCGA CCTCTAAATT TAGGGTTTGT GCCCTTCACA 120
TACCTTGTTG TATGCATTTT GCCACTTGTT TTTTCAAAGC AATGAAAAAG TATCACTGCA 180

ATTAGGTTTT AAATAAATTT GTTTCGTTAA CATGTCAAA 219

SEQ ID NO:2391
SEQUENCE LENGTH:218
SEQUENCE TYPE:nucleic acid
TOPOLOGY:linear
CLONE:HUMGS02756
SEQUENCE DESCRIPTION:
GATCTGGAAT CGGGGTCAGC GGGGCTACAG TCCTTCCAGG GGCTNTGGGG CAGCTCCCAG 60
CCTCTTCCCA TGCTGGTGGC CACCGTGTCC CTTGCTGCGG CTGCATCTTC CAGTCTCTCC 120
TCCGTCTTCC AGTGGCCGCT CTCTTTATAA GAACCCTGGT CATTGAATTT AAGGCCCACC 180
CCAAGTCCAG AATGACCTCG CAAGACCCTT AACTCAAA 218

SEQ ID NO:2392
SEQUENCE LENGTH:217
SEQUENCE TYPE:nucleic acid
TOPOLOGY:linear
CLONE:HUMGS02758
SEQUENCE DESCRIPTION:
GATCCATTTT TAATGGTTCA TTTCCTTTAT GGTCATATAA CTGCACAGCT GAAGATGAAA 60
GGGGAAAATA AATGAAAATT TTACTTTTCG ATGCCAATGA TACATTGCAC TAAACTGATG 120
GAAGAAGTTA TCCAAAGTAC TGTATAACAT CTTGTTTATT ATTTAATGTT TTCTAAAATA 180
AAAAATGTTA GTGGTTTTCC AAATGGCCTA ATAAAAN 217

SEQ ID NO:2393
SEQUENCE LENGTH:216
SEQUENCE TYPE:nucleic acid
TOPOLOGY:linear
CLONE:HUMGS02760
SEQUENCE DESCRIPTION:
GATCAGAGGA ACTTTNAGGT GTCTGCCGGC TAACATTGTG TCATTCCTGG AGTCCACAGT 60
ACACGTCCCC TGCCTCAACA GGCACAGCTC TCACAAAGCT CTTCAAGCAT GGAAGTGGGA 120
GTTGTGTTGT ACTTCATGGC ACTCTGATGC CTGCTGTCTC AGTGTTTGGT TATTATGCAA 180
ACAAGTAATG TTTGAAATAT ATAATAGCAC TGGAAA 216

SEQ ID NO:2394
SEQUENCE LENGTH:215
SEQUENCE TYPE:nucleic acid
TOPOLOGY:linear
CLONE:HUMGS02761
SEQUENCE DESCRIPTION:
GATCCATTTC TACCTGCATT TCCCAGAGGA CTAGCAGGAG GCAGCCTTGA GAAACCGGCA 60
GTTCCCAAGC CAGCGCCTGG CTGTTCTCTC ATTGTCACTG CCCTCTCCCC AACCTCTCCT 120
CTAACCCACT AGAGATTGCC TGTGNCCTGC CTCTTGCCTC TTGTAGAATG CAGCTCTGGC 180
CCTCAATAAA TACTTCCTGC ATTCNNCTGN NNAAA 215

SEQ ID NO:2395
SEQUENCE LENGTH:214
SEQUENCE TYPE:nucleic acid
TOPOLOGY:linear
CLONE:HUMGS02762
SEQUENCE DESCRIPTION:
GATCTAAATG TAAAAGCAAA TGAAAAATGC ATGTNTTTTT NCCTGTCAAA CATGTATACC 60
CTTATGTATA GAGACCAGTA GTCACGTATG GTGACTGAAA CAGGATTATG TAATCCCTAA 120
AAAGCAGAAT ATGTAAAANT CACATGTATG CGTTTGGTTT AGGAATGTNC TTTGTACTT 180
CCACTTGAAT AAAGGTGTGT TTGGTATTCT GAAA 214

SEQ ID NO:2396
SEQUENCE LENGTH:212
SEQUENCE TYPE:nucleic acid
TOPOLOGY:linear
CLONE:HUMGS02764
SEQUENCE DESCRIPTION:
GATCCAGAAA TTCAAACTTC GAGAGCAGAT GGAACGACAT CTAAATCTNT GAATAAAGCA 60
GCAGGCCTGT CCTGGCCGGT TGGCTTGACT CTCTCCTGTC AGAATGCAAC CTGGCTTTAT 120
GCACCTAGAT GTCCCCAGCA CCCAGTTCTG AGCCAGGCAC ATCAAATGTC AAGGAATTGA 180
CTGAACGAAC TAAGAGCTCC TGGATGGGTC CN 212

SEQ ID NO:2397
SEQUENCE LENGTH:212
SEQUENCE TYPE:nucleic acid
TOPOLOGY:linear
CLONE:HUMGS02765
SEQUENCE DESCRIPTION:
GATCTATTAA AGTGTCAACT TTGCATTATA GAACTGAACA TGAAGTAAAT TGAGTGTTTC 60
TGAGTTTNCT ATAGAAAATT TATGCTACCT CTCATCTTTT CACATACAAA TAAGCAACTT 120
TTAATGAAGG CTTAGGGTGN AAATTTTAAT TTTAGCACTT TACATTACAT GATGAATGGT 180
GAATATTAAA GGACTTTGAA CATTTTAATA AA 212

SEQ ID NO:2398
SEQUENCE LENGTH:213
SEQUENCE TYPE:nucleic acid
TOPOLOGY:linear
CLONE:HUMGS02767
SEQUENCE DESCRIPTION:
GATCGAGAAG CTGCTCAATT ATGCACCCCT GGAGAAGTGA CCACGCTGAA ACCCACCCAC 60
CCGCTGTGCT GACCATGGGC CCTGAGCGTC CTACCCCGAA TTCACGAGGC TGAGGCATCC 120
GGGAGCTGGC GTAATGCCTG GCCGCAGTGT GTGTGTATCC CATACCCCAC TCTGGAAGGA 180
ACCATCCAGT AAAGGTCTTT CAGAACCACT AAA 213

SEQ ID NO:2399
SEQUENCE LENGTH:211
SEQUENCE TYPE:nucleic acid
TOPOLOGY:linear
CLONE:HUMGS02768
SEQUENCE DESCRIPTION:
GATCAGAGAT GCACCACCAA GCCAAGGGAA CCTGTGTCCG GTATTCGATA CTGCGACTTT 60
CTGCCTGGAG TGTATGACTG CACATGACTC GGGGGTGGGG AAAGGGGTCG GCTGACCATG 120
CTCATCTGCT GGTCCGTGGG ACGGTGCCCA AAGCCAGAGG CTGGGTTCAT TTGTGTAACG 180
ACAATAAACG GTACTTGTCA TTTCGGGCAA A 211

SEQ ID NO:2400
SEQUENCE LENGTH:211
SEQUENCE TYPE:nucleic acid
TOPOLOGY:linear
CLONE:HUMGS02769
SEQUENCE DESCRIPTION:
GATCAGACAC CCCTTCACGT GTATCCCCAC ACAAATGCAA GCTCACCAAG GTCCCCTCTC 60
AGTCCCCTTC CCTACACCCT GACCTGGCCA CTGCCGNACA CCCACCCAGA GCACGCCACC 120
CGCCATGGGA GTGTGCTCAG GAGTCGCGGG CAGCGTGGAC ATCTGTCCCA GAGGGGGCAG 180
AATCTCCAAT AGAGGACTGA GNACTGCTAA A 211

SEQ ID NO:2401
SEQUENCE LENGTH:206
SEQUENCE TYPE:nucleic acid
TOPOLOGY:linear
CLONE:HUMGS02770
SEQUENCE DESCRIPTION:
GATCACCTAC TTACTGTATT CTACATTATT ATATGNCATA GTATAATGAG ACAATATCAA 60
AAGTAAACAT GTAATGACAA TACATACTAA CATTCTTGTA GGAGTGGTTA GAGAAGCTGA 120
TGCCTNGNTT CTACATTCTG TCATTAGCTA TTATCNTCTA ACGGTTCAGT GTATCCTTAC 180
AGGAATAAAA GCAGCATNTG GNTAAA 206

SEQ ID NO:2402
SEQUENCE LENGTH:205
SEQUENCE TYPE:nucleic acid
TOPOLOGY:linear
CLONE:HUMGS02771
SEQUENCE DESCRIPTION:
GATCGAATGA AGGATTCAAA ATTAACCACT CCAAGGGGGG ATTGAAGGAA GAACCACTCT 60
TAATGGACAA AAAGAAAGAA AGGGGAGGGA GTAACAGGGA TATNAGCTCT AGCCGCCCAA 120
GCTAGCAATG GCAACCCTTC TGGGTCCCCT TCCAGCATGT GGAAGCTTTC CTTTCGCTTC 180
ATTCAATAAA CAGCTGCTGC TCAAA 205

SEQ ID NO:2403

SEQUENCE LENGTH:228
SEQUENCE TYPE:nucleic acid
TOPOLOGY:linear
CLONE:HUMGS02772
SEQUENCE DESCRIPTION:
GATCTATAGA CATNAGGAAG GGAGTNAGAC GGCTCCTCCA CCATCCTCAG CCAGTGCAAC 60
CCATTCCCTC TNCTTCTCTC TCTCTCTCTC TCTCTCCCTC CCTCTCCTTC CCTACCCTCT 120
CACCATCTTT NTTGGCCTCT CTNAGGGTCT CTCTGTGCAT CTNTTTAGGA ATCTNGCTCT 180
CACTCTCTAC GTAGCCACTC TCCTTCCCCN ATTTNTGNGT CCACCCNN 228

SEQ ID NO:2404
SEQUENCE LENGTH:273
SEQUENCE TYPE:nucleic acid
TOPOLOGY:linear
CLONE:HUMGS02773
SEQUENCE DESCRIPTION:
GATCTAGCTA GGGCTTTGTC TNTTCATCTT TGTGCATAAC TTACCTGTTA CCAGTATAGG 60
TGGGATATAC ATNTATCTTG CAGGAAATTC CCCAAAGCTC AGAGTCCAGT TCCTTCCATA 120
AAACAGGCTG GACAAATGAC CACTATGTTA GACCCCCCGG NTCGACTTCA GGGGTCAGTG 180
TTCCTGTCCC AAACCCNCAC ACAGAATNCT CTGNCTGNGG TTCATGTNGA AACTGAGCAA 240
AAACTANAGT ATCTNTCAAN ANGTGTAACN TGN 273

SEQ ID NO:2405
SEQUENCE LENGTH:203
SEQUENCE TYPE:nucleic acid
TOPOLOGY:linear
CLONE:HUMGS02774
SEQUENCE DESCRIPTION:
GATCCACCTC ATATGTGAGT CCGTCCAAAA GATGTTACTG CTCTGGGTGG GCCAGTGTTC 60
TATATCGGTT ATACTAACTT TCATTTAAAG TATTTATTCT AAAATGCCTC TGAGAAACAG 120
TAAAAAATAA AAACAACAAG TTGTCTAAAA TGCAACAGCT TTTATAGTAA ATGTACATTT 180
ATAAATAAAA TACTCAAATC AAA 203

SEQ ID NO:2406
SEQUENCE LENGTH:203
SEQUENCE TYPE:nucleic acid
TOPOLOGY:linear
CLONE:HUMGS02775
SEQUENCE DESCRIPTION:
GATCTCTGAT GGATTTGTTG ACTCCTCTGT GGGAGAAAGG CTTGGTCTAC TGCAGGTGTT 60
AACGCTAAGC CAGGCTGGAG CTGCGGACGT NAGTTTCCTC AGCNCCCCGC TGTTCGCTTC 120
GAACGTNACC TCGACGTCTG CTCTCCCCTT CCCTTTAGCT GACAATGTGA CAGTNCTTGA 180
ATAAAAGTNA GTCTGACAGG AAA 203

SEQ ID NO:2407

SEQUENCE LENGTH:205
SEQUENCE TYPE:nucleic acid
TOPOLOGY:linear
CLONE:HUMGS02776
SEQUENCE DESCRIPTION:
GATCTGAATG TGAATTTCTA ATTGTATCAG ACGTTAATGT TTTAAAGCTA TAACAAAGTT 60
TAAAATTTCT ACTTTTTGTT TTTNATTTAT TTTAACTGTT CTTTTATCTA TTAAATTGTT 120
GTATGTGGAT GGGGAAGTTT TGTTTCTCCT CTTAGCATTT GTTTCTATAA CCAGAAATAA 180
AATTCTATAT TAAAGAAATG ACAAA 205

SEQ ID NO:2408
SEQUENCE LENGTH:201
SEQUENCE TYPE:nucleic acid
TOPOLOGY:linear
CLONE:HUMGS02777
SEQUENCE DESCRIPTION:
GATCCCAATC TGAGAAAAGG CAAAAGAATG GCTACTTTTT TCTATGCTGG NGTATTTTCT 60
AATAATCCTG CTTGACCCTT ATCTGACCTC TTTGGAAACT ATAACATAGC TGTCACAGTA 120
TAGTCACAAT CCACAAATGA TGCAGGTGCA AATGGTTTAT AGCCCTGTGA AGTTCTTAAA 180
GTTTAGAGGC TAACTTACAG N 201

SEQ ID NO:2409
SEQUENCE LENGTH:200
SEQUENCE TYPE:nucleic acid
TOPOLOGY:linear
CLONE:HUMGS02778
SEQUENCE DESCRIPTION:
GATCTTTAAC TGCCTGCTGA TAAGATATTG GGCCAACTGA AAATAGAAAT TGTTCTTTCT 60
TAGAAATATT TTAATGCTAA CTTGTTAGTT TTCCTCAGAA AGCTAGTATT TGAAGCCATT 120
CGAGTTTAAG ATNCTCTAAT TTCAATAAAA TAGCTTCCAA ATATTTAAAA TGTAATAAAA 180
TATATGTTTG ACACTCTAAA 200

SEQ ID NO:2410
SEQUENCE LENGTH:198
SEQUENCE TYPE:nucleic acid
TOPOLOGY:linear
CLONE:HUMGS02779
SEQUENCE DESCRIPTION:
GATCAAAACG CTCCATCTTT TTACAGTGGC ATAGGAAGAC GGCAAAAATT TCCTAAAGTG 60
CAATAGATTT TCAAGTGTAT TGTGCCTTGT TCTAAAACTT TTATTAAGTA GGTGCACTTG 120
ACAGTATTGA GGTCANNNNN TATGGTGCTA TTTCANTNAG TCTAGGTTTA GGCCCTTGTA 180
CATNTTGCCC ATAACTNN 198

SEQ ID NO:2411
SEQUENCE LENGTH:198

SEQUENCE TYPE:nucleic acid
TOPOLOGY:linear
CLONE:HUMGS02780
SEQUENCE DESCRIPTION:
GATCTGCCCA TTGCTGAAAC TTCAGTACTT AAGTCCCCTA TTATNATTGT ATGGCAATCT 60
ATCTGTCCAC TCAGAAGTTA TTATATTTGT TTTATATGAT TGAGCACTTC TGTTTTGGGT 120
ATATATGTAT TTACAATNAC TATAACCTCT TGCTGAATTA ACCCCTNTTA CCATTATGTA 180
ATAAACTTAT TTCCCAAA 198

SEQ ID NO:2412
SEQUENCE LENGTH:198
SEQUENCE TYPE:nucleic acid
TOPOLOGY:linear
CLONE:HUMGS02781
SEQUENCE DESCRIPTION:
GATCTTGAAT CACTTGACAG TGTTTGTTNG AATTGTGTTN GTTTTTNCCT TTGATGGGCT 60
TAAAAGAAAT NATCCAAAGG GAGAAAGAGC AGTATGCCAC TTCTTAAAAC AGAACAAAAC 120
AAAAAAAGAA AATTGGGCTC TNTTCTAATC CAAAGGGTAT ATTTNCAGCA TGCTTGACTT 180
TACCAATNCT GATGACAN 198

SEQ ID NO:2413
SEQUENCE LENGTH:359
SEQUENCE TYPE:nucleic acid
TOPOLOGY:linear
CLONE:HUMGS02782
SEQUENCE DESCRIPTION:
GATCCAAAGT AACTAAACAG GAGCCCACAA GACGGTCTGC CAGATTGTCA GCGAAACCTG 60
CTCCACCAAA ACCTGAACCC AAACCAAGAA AAACATCTGC TAAGAAAGAA CCTGGAGCAA 120
AGATTAGCAG AGGTGCTAAA GGGAAGAAGG AGGAAAAGCA GGAAGCTGGA AAGGANGGCA 180
CAGAAAACTG NTTCTGTAGA TAACGTGGGA GAATGANTTG TCATGAAAAT TTGGTGGTTG 240
ATTTTTTTGT NTGTCTTGGG ACAACTTTTA AANGCTATTT TTNCCANGTN TTTTTNGTNN 300
TTGGTTTTTT TTTTAGGGNT CTNCTTGTTG GCATNCGGNA GTTTTTTANG GTTTGGNCN 359

SEQ ID NO:2414
SEQUENCE LENGTH:198
SEQUENCE TYPE:nucleic acid
TOPOLOGY:linear
CLONE:HUMGS02783
SEQUENCE DESCRIPTION:
GATCCCAGGG GTGCACATGG GCCCCTTGGG TGTCTGAACA GAAGGGCATG GNAGGAAGGG 60
CTGCACCCCT GCAGTCTTAC TCTGCTGGTG TAGCGGGCAG CTGCCCACTC CCACCCCACC 120
CTGCACCGCG GGCTCCTNAG TCGGCAGATT AAGCATTTNA TAAATTGTAT TTTAAATACA 180
TGTTTTAAAC TTGTCAAA 198

SEQ ID NO:2415

SEQUENCE LENGTH:167
SEQUENCE TYPE:nucleic acid
TOPOLOGY:linear
CLONE:HUMGS02785
SEQUENCE DESCRIPTION:
GATCCCATCC ATCAGCTCCC TTTTCTTTTT CCCTTGAACT GTTCTGGCCT CAGACCAACT 60
CTCTCCNNNN NTAATCTCTC TCCTGTATAA CCCCACCTTG CCAAGCTTTC TTCTACAACC 120
AGAGCCCCCC ACAATGATGA TTAAACACCT GACACATCTT GCANAAA 167

SEQ ID NO:2416
SEQUENCE LENGTH:196
SEQUENCE TYPE:nucleic acid
TOPOLOGY:linear
CLONE:HUMGS02786
SEQUENCE DESCRIPTION:
GATCTGTGAT TGGNTGGTTA ATGGGAAACG GTTTTTTNCT TTGGCTGCAG GTGTTCTGCT 60
GATATCAACA GCTTCCCTAT TTTNAATGCA GAAAACAGGG TCTGGGACAT TAGTCGTTAT 120
ATTTNACTTG AAAAGAAAGA AACCAAGTGC GCTTTGCAAT ATTTATTACA CAAAGAACTT 180
GCTGCTGCAT TCTAAA 196

SEQ ID NO:2417
SEQUENCE LENGTH:194
SEQUENCE TYPE:nucleic acid
TOPOLOGY:linear
CLONE:HUMGS02787
SEQUENCE DESCRIPTION:
GATCACAGAT TTCCCTGCCA GGGTGTCTGT GGTTATCAGC TGCAGGCTCA GCTNGGGTCG 60
GCCGNTTCAC ACAAGCCACT CTGTACCACG TGCCCTACCT TAGTGACGGG AGTAAGGAGC 120
TTCCTTCCCC TCCATGTCAT TCCTNCCTGT TCCNTNCATT CCCCAACAGC AAATAAAACT 180
GTTGAATGCT CAAA 194

SEQ ID NO:2418
SEQUENCE LENGTH:193
SEQUENCE TYPE:nucleic acid
TOPOLOGY:linear
CLONE:HUMGS02788
SEQUENCE DESCRIPTION:
GATCTTCCTA CGCACAACAC AAACGTCAGT TAATGTTCCA TCCATGCTGC TTAAAGAGCA 60
TTCCTGTCCT AGTAAAATGG GCAAGTCCCT CTACCCCCCA CCCTCACCTG GTATGCTTAC 120
ATTAATAGCT AAAGTCAATC CTGTAATGAA ATAAAGCAAG TGGTAGCTGT CTGGTAGCCT 180
CCACTACTGC AAA 193

SEQ ID NO:2419
SEQUENCE LENGTH:193
SEQUENCE TYPE:nucleic acid

TOPOLOGY:linear
CLONE:HUMGS02789
SEQUENCE DESCRIPTION:
GATCAAAACC CAGCAGAGTG CAAGCAGCAG TGAAGCAGGA TGTATGTGGA CTTGAGGATA 60
ACCTGCACTG GTCTACCTTC TGCTTCCCTG GAAAGGATGA ATTTACATCA TTTGACAAGC 120
CTATTTTCAA GTTATTTGTT GTTTGTTTGC TTGTTTTTNT TTTTGCAGCT AAAATAAAAA 180
TTTCAAATAC AAA 193

SEQ ID NO:2420
SEQUENCE LENGTH:192
SEQUENCE TYPE:nucleic acid
TOPOLOGY:linear
CLONE:HUMGS02790
SEQUENCE DESCRIPTION:
GATCAGACCT CAATAATTTT ATACTGTAAT AAGCTCTTTG AATGTNTATA TTATTATTTT 60
TNACAATCAT TTCATTTTGT ATTTAATATC AATGGACTGA GTCTGATTCA GAAAATAATT 120
GTAATGTTCA TATTCAATAT CTTACAGTGA TACAGTTTTG TATTTACATA TAAATATACC 180
GACATGACCA AA 192

SEQ ID NO:2421
SEQUENCE LENGTH:276
SEQUENCE TYPE:nucleic acid
TOPOLOGY:linear
CLONE:HUMGS02791
SEQUENCE DESCRIPTION:
GATCAGGGTT TCAAGAATGA CTGCAGTGGG TTTTGGAAAC AGACTTATCA TTATTGATTT 60
GAGGTTTCCC AGAGATATAG TTCACAGTTA ATTGTTGCGC TCTAATACAA CTGACCATTT 120
AAAATTGAAC AAGTTTATTG TTTTGTAACA ATGTCAGTTG TTAAACCTTG NCATTTCAAT 180
TAAAACATGA NTTGTAGTTA TAACTCANTG CAANTNCAAC AGTTGTATTT GGNGTTAANT 240
TATTTTCACA CGTNNCTTTN TTTCATGGAN NNCCCN 276

SEQ ID NO:2422
SEQUENCE LENGTH:191
SEQUENCE TYPE:nucleic acid
TOPOLOGY:linear
CLONE:HUMGS02792
SEQUENCE DESCRIPTION:
GATCTTTTAT TTTCTCATTA TTTATAACTT CAGACTTGGG CCCCCTGTTC TTTCTTTCCC 60
ATTAACTTGA GTGACCTGTG TGAGAGACAG ACAGATGCCC CACGAGGATG GCTGGACAAG 120
GACTTTTACT TTTTATTACA TAAAAATATT AAAAAATAAA TAAAAAAAAT AAAATTTTAA 180
ACTAACTTAA A 191

SEQ ID NO:2423
SEQUENCE LENGTH:189
SEQUENCE TYPE:nucleic acid

TOPOLOGY:linear
CLONE:HUMGS02793
SEQUENCE DESCRIPTION:
GATCGGAATG CGGGGTCGAG AGCTGATGGG CGGCATTGGG AAAACCATGA TGCAGAGTGG 60
CGGCACCTTT GGCACATTCA TGGCCATTGG GATGGGCATC CGATGCTAAC CATGGTTGCC 120
AACTACATCT GTCCCTNCCC ATCAATCCCA GCCCATGTAC TAATAAAAGA AAGTCTTTGA 180
GTAGTCAAA 189

SEQ ID NO:2424
SEQUENCE LENGTH:187
SEQUENCE TYPE:nucleic acid
TOPOLOGY:linear
CLONE:HUMGS02794
SEQUENCE DESCRIPTION:
GATCGAGACC ATCCTGGCTA AAACGCCTGG AATGCAGTGG CACAATCACT GCTCACTGCA 60
GCCTTGACCG TNTGGACTCA AGTAATTCTC CTACTTCAAC CTCCCAAGTA GCTGGGACTA 120
CAGTCATGCA CCATCATGCC TATAAAGATA ATTTAATTTA TAATAAATNA TTTATACAAA 180
CAATAAA 187

SEQ ID NO:2425
SEQUENCE LENGTH:187
SEQUENCE TYPE:nucleic acid
TOPOLOGY:linear
CLONE:HUMGS02795
SEQUENCE DESCRIPTION:
GATCAATCGT GCATCCTNAG TGAACTTCTG TTGTCCTCAA GCATGGTCTN NNTACTTGTG 60
AACTATGGTG TTCAGTTTTG CCTCTGTTAG AAATTCACAC TGTTGTTGTA ATNATGTGGA 120
ACTCCTCTAA AANTTACAGT ATTGTCTGTG AAGGTATCTA TACTAATAAA AAAGCATGTG 180
TAGNAAA 187

SEQ ID NO:2426
SEQUENCE LENGTH:185
SEQUENCE TYPE:nucleic acid
TOPOLOGY:linear
CLONE:HUMGS02796
SEQUENCE DESCRIPTION:
GATCNCGTTT TNAACCCGNT CCTGCCCCAC CTGCCCTATA GTTATGGCCT GTGTTCTGCT 60
CTCCTGGTCC CAGTGCACCG TCTGCTCTGT GAACTCCTCC CACCAGGCCC TTCTTACCCC 120
ACGCGTGTCT GTCCCCNTCG TTCTGTAGCG GGNGGTACAT AATAAAACAA TGGNGTGGNG 180
ACAAA 185

SEQ ID NO:2427
SEQUENCE LENGTH:181
SEQUENCE TYPE:nucleic acid
TOPOLOGY:linear

CLONE:HUMGS02797
SEQUENCE DESCRIPTION:
GATCTATGTT TTCTGAAAAT AATTGGTTAA TGGAAGTTAT CTAATATATT TNAACTGTTC 60
CTGTTAAAAA CAATAGGCTT CAAGATGACA TAACACCAAA TCAAAAATNA CCAAAGGAAT 120
CATTTTGTTT GTTAGATTTG TAATTTAGCA TCATTGGCAA TAAATCTACT CAAACGTTAA 180
A 181

SEQ ID NO:2428
SEQUENCE LENGTH:180
SEQUENCE TYPE:nucleic acid
TOPOLOGY:linear
CLONE:HUMGS02798
SEQUENCE DESCRIPTION:
GATCCCAAAG GGGTTGGAGG CAGGGAATAT TTGAAAATTC AGACTGGAAT TCCAGGCCAA 60
AAGCTCAAGA CCACCAGCCT CTCCTCCTGC CTGGAAAAAT AAGCCTTTGT GAAAGACTGA 120
TTTACCATGT ACAATTGTGA TTGTGAACGT TGTTGAGTAA ACCTCCAGAC TTTCTCTAAA 180

SEQ ID NO:2429
SEQUENCE LENGTH:186
SEQUENCE TYPE:nucleic acid
TOPOLOGY:linear
CLONE:HUMGS02799
SEQUENCE DESCRIPTION:
GATCATAAGT NAAAAATGTA TTTAATATAC CTAACCTACC AAACATCATA GCTTAGCCTA 60
GCCTGCCTTA AACATGCTCA GAACACTTAC ATTAGCCTAC AGTGGGCAAA ACTATCCAAC 120
ACAAAATCTA TATTGTAATA AAGTTATAAA GANTTTTGAA TAAAANTTCA ATATTTGANG 180
TACAAA 186

SEQ ID NO:2430
SEQUENCE LENGTH:180
SEQUENCE TYPE:nucleic acid
TOPOLOGY:linear
CLONE:HUMGS02800
SEQUENCE DESCRIPTION:
GATCTGGTGG AGGGAACTGA AGATGAAGGC CTCTCAGGCA CCATGGATGG NATGGGTGTG 60
TGTNAAGGGG GCAGGGTCCC CAGGAGCTGC CCCAGGTCTG GCTNGACAGT TGCACGGNAA 120
GTCCTGTTTG TAAACTCAAC GTTTCCACGN CTTTTCCATT AAACTTTACC CCAAATCAAA 180

SEQ ID NO:2431
SEQUENCE LENGTH:180
SEQUENCE TYPE:nucleic acid
TOPOLOGY:linear
CLONE:HUMGS02801
SEQUENCE DESCRIPTION:
GATCTATTTT TCCTAAAACA TTCCCCTCCC CACTCCTCTC CCACAGAGTG CTNNNNTGTT 60

CCAGGCCCTC CAGTGGGCTG ATGCTGGGAC CCTTAGGATG GGGCTCCCAG CTCCTTTCTC 120
CTGTGAATNG AGGCAGAGAC CTCCAATAAA GTGCCTTCTG GGCTTTTNCT AACCTTTAAA 180

SEQ ID NO:2432
SEQUENCE LENGTH:179
SEQUENCE TYPE:nucleic acid
TOPOLOGY:linear
CLONE:HUMGS02802
SEQUENCE DESCRIPTION:
GATCTTCACC AATGGCAAGT GGAATGACAG GGCTTGTGGA GAAAAGCNNN TTGTGGTCTG 60
CGAGTTCTGA GCCAACTGGG GTGGGTGGGG CAGTGCTTGG CCCAGGAGTT TGGCCAGAAG 120
TCAAGGCTTA GACCCTCATG CTGCCAATAT CCTAATAAAA AGGTGACCAT CTGTGCAAA 179

SEQ ID NO:2433
SEQUENCE LENGTH:273
SEQUENCE TYPE:nucleic acid
TOPOLOGY:linear
CLONE:HUMGS02803
SEQUENCE DESCRIPTION:
GATCTAAAAA TTCAATGCAA ATATCTTTTA TTTGGTAGTT TTGTCTACAT ATTTNATGCT 60
CTAGCATGTG CAATATATCT TTGTAAAGCA CGATGATACA AATCTGGTGC CAGTGTTATA 120
TTTNGCATAA CATATTTGTA ACAGCATAAA ATATTGTTTG ATGATTTCAG TGGGATTTTG 180
TCTATAATGT TTTCTTATGT AAATNGGAGT TGAATGACTC TGGTAAATGT CATGACTGTA 240
AAANTGGGGA AANTGACTTT NAGTTCAGTG AAN 273

SEQ ID NO:2434
SEQUENCE LENGTH:178
SEQUENCE TYPE:nucleic acid
TOPOLOGY:linear
CLONE:HUMGS02804
SEQUENCE DESCRIPTION:
GATCCAAACT AATTTTACAT TTAAAAAAAA AGTTAAACTA AACTTCTTTA CTGCTGATAT 60
GTTTCCTGTA TTCTAGAAAA ATTTTNACAC TTTCACATTA TTTTTGTACA CTTTCCCCAT 120
GTTAAGGGAT GATGGCTTTT ATAAATNTGT ATTCATTAAA TGTTACTTTA AAAATAAA 178

SEQ ID NO:2435
SEQUENCE LENGTH:176
SEQUENCE TYPE:nucleic acid
TOPOLOGY:linear
CLONE:HUMGS02805
SEQUENCE DESCRIPTION:
GATCTAAAAT AAATATTGTT AAAAGCATTA CCTCTAGGGA ATGGGATTTA GATTTAAAAA 60
GGGTGGGATG GGAAACTGTG TTTTTCATTT TAAGTCCTTC TGTACTANNT AATTTGTNAC 120
CTTGTGCATG TATTACTTTG AAAAAATTTT TAATAAACCC AAATAAAANT CTTAAA 176

SEQ ID NO:2436
SEQUENCE LENGTH:175
SEQUENCE TYPE:nucleic acid
TOPOLOGY:linear
CLONE:HUMGS02806
SEQUENCE DESCRIPTION:
GATCANCCCT CAAGGGCACA TGCCAAGGGC AGAGCAGCCC ATNTAGACAG CTTCGNAGGG 60
CATGGGGGTG TAGGGAGTTC GGGGTAGCTC CTCATTAACT ATTTGTNGGN TGAGTAAAGG 120
GGTGAGGCTC AGTGGCAGGT ACCTCTNCAA TNACAAGCTG CCNCCCCTCT ATGTN 175

SEQ ID NO:2437
SEQUENCE LENGTH:175
SEQUENCE TYPE:nucleic acid
TOPOLOGY:linear
CLONE:HUMGS02807
SEQUENCE DESCRIPTION:
GATCGGCAGC AAGAGTAAAG ACACAGCTCC AGAGGCCCAC ACTGTGGGGT CTGGGCCCTG 60
CCTTAGGCAG CCCCCCTCTT TGGCCCCCTC CTGTCAGGCC CAGGGCTTGG AGTGAAAGTN 120
ACTCTCAGGT GGTGGGGTGG GGAATGTGAA TAAACATGAT TTCTTGCCGG GCAAA 175

SEQ ID NO:2438
SEQUENCE LENGTH:174
SEQUENCE TYPE:nucleic acid
TOPOLOGY:linear
CLONE:HUMGS02808
SEQUENCE DESCRIPTION:
GATCCCTTCA CTGGCCCCCT CGGGAGNCCT GGGTTGGACT CAGGGNCTCC NCCAGCTGGG 60
GNGGNGGACC GCAGCACCTA TCTGAGCAGT TAGAGCGTCT TTTTTTTCAG ATTGTGTACA 120
GTAGATTATT TATTTTGTNA TTTTGGAATA AAATTTATTT TATGGCTTAG GAAA 174

SEQ ID NO:2439
SEQUENCE LENGTH:172
SEQUENCE TYPE:nucleic acid
TOPOLOGY:linear
CLONE:HUMGS02809
SEQUENCE DESCRIPTION:
GATCTTGTCT GAAATGCTAG TTTCTGACTA CAAAAACTAC AGGACATAGT AGATATGCTC 60
AGTAGAGCTG CTTAAAATAA TTCCCTTTTT TTTTCTGTTG AATTCTGTTA CAAAACCAGG 120
TCTGTGTTAT AATAGAAANC ACACTTATGA ATAAAAATCT TTAAAAATTA AA 172

SEQ ID NO:2440
SEQUENCE LENGTH:171
SEQUENCE TYPE:nucleic acid
TOPOLOGY:linear
CLONE:HUMGS02810

SEQUENCE DESCRIPTION:
GATCAATGTC CCTGGTTACC TATGTGTTTG AATTATCTTC GTGTTACAGG TGTTTAATGA 60
TTTTNCTCCT TCTAGCTTAT TTGTATTTCA CCTGTTTTTC TTTAAATNAA CATGGTTACA 120
CTNTGTTTCA GCAACTGTAT AAATTAAACA CAGATNATTA CTACTGCTAA A 171

SEQ ID NO:2441
SEQUENCE LENGTH:170
SEQUENCE TYPE:nucleic acid
TOPOLOGY:linear
CLONE:HUMGS02811
SEQUENCE DESCRIPTION:
GATCGTAAAA TTTTAAGCAC TAAAAATTTT TTCATAACCT TTCATAATAA AGTTTAATAA 60
TAGGTTTATT AACTGAATTT CATTAGTTTT TTAAAAGTGT TTTTGGTTTG TGTATATATA 120
CATATACAAA TACAACATTT ACAATAAATA AAATACTTGA AATTCTCAAA 170

SEQ ID NO:2442
SEQUENCE LENGTH:196
SEQUENCE TYPE:nucleic acid
TOPOLOGY:linear
CLONE:HUMGS02812
SEQUENCE DESCRIPTION:
GATCTACTTT GAATTCCACA CTCTCATTAA TAAATTGANT AAAAGGGAAT ATTTTGGCAC 60
CTGATATAAT CTGCCAGGCT ATGTGACAGT AGGAAGGAAT GGTTTCCCCT AACANGCCCA 120
ATGCACTGGT CTGACTTTAT AAATNATTTA ATAAAATGAA CTATTATCAA ATAAAACGTA 180
TGATTCAGTC CTTAAA 196

SEQ ID NO:2443
SEQUENCE LENGTH:216
SEQUENCE TYPE:nucleic acid
TOPOLOGY:linear
CLONE:HUMGS02813
SEQUENCE DESCRIPTION:
GATCAAATTT TTTTCTTGCA ATNATGTGAT GAGTTGCCAA ATAATTGANA TTATTTTAAA 60
ATGTTTTGTT CATATTCTTG TTTTATAATT AAAATTTACA TTCAGTGTGT ATGGGTTTTT 120
TTTTNATTTT NACTCTTAAT GTAAGGTGGA TATTNCTGTN ATTTTACATG GTTTCTTACT 180
GAGATTTTAT ATATAANTNA TAAAANGTTT ACCAAA 216

SEQ ID NO:2444
SEQUENCE LENGTH:173
SEQUENCE TYPE:nucleic acid
TOPOLOGY:linear
CLONE:HUMGS02814
SEQUENCE DESCRIPTION:
GATCGTTAAT GCCCTTGAAG TGGTTTTTGT GGGTGTGAAC AAATGGTGAG AATTTGAATT 60
GGTCCCTCCT ATNATAGTAT TGAAATAAAG TCTACTTAAT TTATCAAGTC ATGTTCATGC 120

CCTGATTTTA TATACTTGTA TCTATCAATA AACATTGTGA TACTTGATGT AAA 173

SEQ ID NO:2445
SEQUENCE LENGTH:223
SEQUENCE TYPE:nucleic acid
TOPOLOGY:linear
CLONE:HUMGS02815
SEQUENCE DESCRIPTION:
GATCAGCTCC GTGGGGCTGG TTTTGGTCCA CAGCATAACA GGCACTGCCT CCTTACCTGT 60
GAGGAATGCA AAATAAAGCA TGGATTAAGT GAGAAGGGAG ACTCTCAGCC TTCAGCTTCC 120
TAAATTCTGT GTCTGTGACT TTCGAAGTTT TTTAAACCTC TGAATTTGTA CACATTTAAA 180
ATTTCAAGTG TACTTTAAAA TAAAATACTT CTAATGGAAC AAA 223

SEQ ID NO:2446
SEQUENCE LENGTH:168
SEQUENCE TYPE:nucleic acid
TOPOLOGY:linear
CLONE:HUMGS02816
SEQUENCE DESCRIPTION:
GATCTGTCAC TTTGTATAAA AACAAGAAAC GATTAGTTCA TTAAACTTTT TCTTTACCTT 60
CTGGTAGTAA AAATAGATAA AATAATTCCG TNTCAACTAC TGTGTGTAAA AAGCCTGTAT 120
CATATGTAAC TGGGACTTTT GTAAATAAAT NTTTTTGCGC TCTGCAAA 168

SEQ ID NO:2447
SEQUENCE LENGTH:168
SEQUENCE TYPE:nucleic acid
TOPOLOGY:linear
CLONE:HUMGS02817
SEQUENCE DESCRIPTION:
GATCATAAAA TGTATCTTTT CCATGGCCAA CAAGGNGCAT CTNTTATAAA TGCATAATAA 60
CCCAGTTNGT ATCAAAGGGT ATCGACTTAA GTGAAATTTC AACATGCTGT TACTTTTTCC 120
TTTTAATGTA ATNCTGTTTT CCAAATAAAT GGGGGAGACA AATGGAAA 168

SEQ ID NO:2448
SEQUENCE LENGTH:135
SEQUENCE TYPE:nucleic acid
TOPOLOGY:linear
CLONE:HUMGS02818
SEQUENCE DESCRIPTION:
GATCTGTGCA ATAAAGTGTT TGCAGTCTTA TTATTCTCTA AGAAATTCCT TATGGACGTC 60
ATAATTGTTG TATATTGAAC AAAATATTTA TACTTATGCA GTTGCATAAC ATTGAAATAA 120
AAATTTAGCA TGAAA 135

SEQ ID NO:2449
SEQUENCE LENGTH:167

SEQUENCE TYPE:nucleic acid
TOPOLOGY:linear
CLONE:HUMGS02819
SEQUENCE DESCRIPTION:
GATCCTGCCA GAAAATTTCA AAGACAGTAG ATTGAAAAGG AAAGGCTTGG TAAAAAAGGT 60
TCAGGCATTC CTAGCCGAGT GTGACACAGT GGAGCAGAAC ATCTGCCAGG AGACTGNGCG 120
GCTGCAGTCT ACAAACTTTG CCCTGGCCGA GTGAGGTGTA GCAGCAN 167

SEQ ID NO:2450
SEQUENCE LENGTH:167
SEQUENCE TYPE:nucleic acid
TOPOLOGY:linear
CLONE:HUMGS02820
SEQUENCE DESCRIPTION:
GATCAGNAGG CATCACTGAG GCCAGGAGCT CTGCCCATGA ACCTGTATCC CACGTACTCC 60
AACTTCCATT CCTCGCCCTG CCCCCGGAGC CGAGTCCTGT ATCAGCCCTT TATCCTCACA 120
CGCTTTTCTA CAATGGCATT CAATAAAGTG CACGTGTTTC TGGTAAA 167

SEQ ID NO:2451
SEQUENCE LENGTH:413
SEQUENCE TYPE:nucleic acid
TOPOLOGY:linear
CLONE:HUMGS02821
SEQUENCE DESCRIPTION:
GATCCGGTTT CTTTTTGCTC GCCCCTGTTT TTTGTAGAAT CTCTTCATGC TTGACATACC 60
TACCAGTATT ATTCCCGACG ACACATATAC ATATGAGAAT ATACCTTATT TATTTTTGTG 120
TAGGTGTCTG CCTTCACAAA TGTCATTGTC TACTCCTAGA AGAACCAAAT ACCTCAATTT 180
TTGTTTTTGA GTACTGTACT ATCCTGTAAA TATATCTTAA GCAGGTTTGT TTTCAGCACT 240
GATGGAAAAT ACCAGTGTTG GGTTTTTTTT TAGTTGCCAA CAGTTGTATG TTTGCTGATT 300
ATTTATGACC TGAAATAATA TATTTNGTNC TTCTAAGANG ACATTTTGTT ACATAAGGGG 360
ATGGCCTTTT TTATACAANG GGAATAANAT TNTGGGCATT TCCTATGGAGGGCATT TCCTATGGAA AAA 413

SEQ ID NO:2452
SEQUENCE LENGTH:166
SEQUENCE TYPE:nucleic acid
TOPOLOGY:linear
CLONE:HUMGS02822
SEQUENCE DESCRIPTION:
GATCTGTNAT TGGCATCTTG GTTCTACCTT TTATCAGATG TTGTTGCCTT GTGCAAATNA 60
CATTTTCTCT GAGTCTCCAT TTTCTCATTT ATAACATGCG GAGAATAATA CTTATATTTG 120
TGGTAGTAGT GTGAACATTA AAAAGATAAT GTATGTGAAG TACAAA 166

SEQ ID NO:2453
SEQUENCE LENGTH:165
SEQUENCE TYPE:nucleic acid

TOPOLOGY:linear
CLONE:HUMGS02823
SEQUENCE DESCRIPTION:
GATCCATGCT AATATTGTAT TTTTTNTTAA AACATAGCTT TCCTGTAATT TAAAGTGCTT 60
TTATGAAAAT ATTTGTAATT AATTATATAT AGTTGGAAAT AGCAGTAAGC TTTCCCATTA 120
TAATATATTT TGNTATACAA ATAAAATTTG AACTGAAGTC TGAAA 165

SEQ ID NO:2454
SEQUENCE LENGTH:164
SEQUENCE TYPE:nucleic acid
TOPOLOGY:linear
CLONE:HUMGS02824
SEQUENCE DESCRIPTION:
GATCTTGTTA GAAGGCAGTA GGTCTGGGGT GGGGCCTGAG ATTCTGCATT TCTAACCAGG 60
TCCTGGGAGA TGCTGATGCT ATCGAGCCAC AACCACACTT TGAGTAGCAA GCCTCTGGCC 120
TATCCTTANN NNTTGTAATA TTAAAATGCT GCCTCTCAGT CAAA 164

SEQ ID NO:2455
SEQUENCE LENGTH:164
SEQUENCE TYPE:nucleic acid
TOPOLOGY:linear
CLONE:HUMGS02825
SEQUENCE DESCRIPTION:
GATCAATGCC AGATTTTNTT NTTGGGGTAA GTTAGCTGAA GTCATTTAAA GATGGAAAGG 60
TGGGAAAATN CTTTGATATT TGATGTCATT GTATCCACAT TTNTTGTAAG ACATATTGCA 120
TACCANTTAT AATNATATCA ATTAAAGTTG ATAAAAGCTT CAAA 164

SEQ ID NO:2456
SEQUENCE LENGTH:164
SEQUENCE TYPE:nucleic acid
TOPOLOGY:linear
CLONE:HUMGS02826
SEQUENCE DESCRIPTION:
GATCCCTTCT GCCCTGGCAG AATGGCAGGG GTAATCTNAG CCTTCTNCAN TCCTTTACCC 60
TAGCTGACCC CTTCACCTCT CCCCCTCCCT TTNCCTTTGT NTTGGGATTC AGAAAACTGC 120
TTGTAAGAGA CTGTTTATNT TTAATNAAAA ATATAAGGCT TAAA 164

SEQ ID NO:2457
SEQUENCE LENGTH:162
SEQUENCE TYPE:nucleic acid
TOPOLOGY:linear
CLONE:HUMGS02827
SEQUENCE DESCRIPTION:
GATCCAGAAT ATTTTCATTT AAAGCTCAAC CATTAATTGG AACATGGTGA AACATTGTAC 60
ACATTGTAAA AGTAGGGAAC AGTACAAAAG AATGGAATCA AATACTATAT TATGTGAATA 120

ANCTAGAGTG ATTTTGGAAT TTCACTCTCT TCACCCCTTA AA 162

SEQ ID NO:2458
SEQUENCE LENGTH:161
SEQUENCE TYPE:nucleic acid
TOPOLOGY:linear
CLONE:HUMGS02828
SEQUENCE DESCRIPTION:
GATCAAAAAC CTCTATAGTA GCCACTAGGG CAAAAACTGT GTGTATGTGT GTGTGTAAGT 60
GTGTGTACAC TGTTCAATAT GGTTCAATAT GGTACCAATA GCCACATGTG ACTATTTAAA 120
TTCATTGCAA TGNAATAAAA TTAAAGGTAT ACTAGCTCAA A 161

SEQ ID NO:2459
SEQUENCE LENGTH:159
SEQUENCE TYPE:nucleic acid
TOPOLOGY:linear
CLONE:HUMGS02829
SEQUENCE DESCRIPTION:
GATCTTCACC TCTCTGCCTC CCTACTGCCC CAACATAGCC TTGAGGAGGN TTCTCAGCCA 60
CCAAAGGGTT CTGGCCCCTT CTCACTCTCC TCTCCTCTCA GATGGAACTC TGGTTATAAT 120
GGTGTTAGTT ATCGAATAAA AACGACTTCA GAATGCAAA 159

SEQ ID NO:2460
SEQUENCE LENGTH:159
SEQUENCE TYPE:nucleic acid
TOPOLOGY:linear
CLONE:HUMGS02830
SEQUENCE DESCRIPTION:
GATCAATTTA AAATATTTNA TTTCTTAAAA GCCTTTTTTG CCTGTTGTAA TGTGCAGGAC 60
CCTTCTCCTT TCATGGGAGA GACAGGTAGT TACCTGAATA TAGGTTGAAA AGGTTATGTA 120
AAAAGAAATT ATAATAAAAG GGATACTTTG CTTTTCAAA 159

SEQ ID NO:2461
SEQUENCE LENGTH:158
SEQUENCE TYPE:nucleic acid
TOPOLOGY:linear
CLONE:HUMGS02831
SEQUENCE DESCRIPTION:
GATCTATTTA ATTTGAGGTT GATGTTCTAT CCAATGGCCG AAGATAGCAG CAGGTTTTTT 60
TTTTCATATG TTGATTTGTT TCATTTGGAT TTTTTTTTTA TNATNCTTNA TTGTCTTTTT 120
TTTNCCCCAT CCCTGTCTTG NGATTTNCNG GCATGTTN 158

SEQ ID NO:2462
SEQUENCE LENGTH:256
SEQUENCE TYPE:nucleic acid

TOPOLOGY:linear
CLONE:HUMGS02832
SEQUENCE DESCRIPTION:
GATCTGAAAT GTGATGATTA TTGGAATGCA TAAACATTGA CGTATTTTGG AATGTATTAA 60
AACTGAAATG GATAAAAATT AGTATATATG TTTATTTTGT TCTATCTCCT TTGGTTGTAT 120
TTTTCTCTGC AGTATTAAAC ATTTGGAAGA GACTAAAGGT ATGCAAAANT AATGTCTTAT 180
TTTCATTATG TGTGCTTCCA TTTGACACAT GGGAAATTAG ATTAATTTCA CTATTAANTA 240
GNACTTTATA CAGAAA 256

SEQ ID NO:2463
SEQUENCE LENGTH:156
SEQUENCE TYPE:nucleic acid
TOPOLOGY:linear
CLONE:HUMGS02833
SEQUENCE DESCRIPTION:
GATCTTGTGT CTACTCTAGG ACTGGGCCCG AGGGTGGTTT ACCTGCACCG TTGACTCAGT 60
ATAGTTTAAA AATCTGCCAC CTGCACAGGT ATTTTTGAAA GCAAAATAAG GTTTTCTTTT 120
TTCCCCTTTC TTGTAATAAA TGATAAAATT CCGAAA 156

SEQ ID NO:2464
SEQUENCE LENGTH:156
SEQUENCE TYPE:nucleic acid
TOPOLOGY:linear
CLONE:HUMGS02834
SEQUENCE DESCRIPTION:
GATCTTCTCC TTTGTGAAGC ATCCCTCACC ACTACTACTG TGATGGGTCG GGGCCTTTCA 60
TCTGTTTTTC CACAGCATCT ATGCTTCTNT NCACTGTATC ATTTATCAAA TTGATNNTTN 120
CTGCGTCCTG TAAAAAACAN NGAGTACCTT AAGAAA 156

SEQ ID NO:2465
SEQUENCE LENGTH:191
SEQUENCE TYPE:nucleic acid
TOPOLOGY:linear
CLONE:HUMGS02835
SEQUENCE DESCRIPTION:
GATCTGCATT TATCATGAGT TCCTTTTTTT TTTAAACTTT ATTTTTGGGA AAGTAACACA 60
TGANGTAGTT CAGTCATGTC AGGTTTGTCT GGGGTGGANT GGACCAGTCA GGTAGTTGAA 120
AGTTTTTTTT TAGNGATGAA AAGCTTGTGA NCNCCTGTAA ANCATGCTGN ATTTGAAATN 180
CATCTGTTNA N 191

SEQ ID NO:2466
SEQUENCE LENGTH:155
SEQUENCE TYPE:nucleic acid
TOPOLOGY:linear
CLONE:HUMGS02836

SEQUENCE DESCRIPTION:
GATCCACCCC CTGCCCACAA AAAATTGCTC CTAACTCCAC CGCCTATCCC AAACCTACAA 60
GAACTAATGA TAATCCCACC ACCCTTTGCT GACTCTTTTT GGACTCAGCC CACCTGCACC 120
CAGGTGATTA AAAAGCTTTA TTGTTCACAC ANAAA 155

SEQ ID NO:2467
SEQUENCE LENGTH:154
SEQUENCE TYPE:nucleic acid
TOPOLOGY:linear
CLONE:HUMGS02837
SEQUENCE DESCRIPTION:
GATCCTAGCA AATTTGCCCA ACCANCTTTA CTAAAGGGGG AGGAAGGGAG GGCAAAGGGA 60
TGAGAAGACA AGTTTCCCAG AAGTGCCTGG TTCTTTTTAC TTTTCCCTTT GTTGTCGTTN 120
TTGTAGTTAA AGGAATTTAA TTTTTAAAAA GAAA 154

SEQ ID NO:2468
SEQUENCE LENGTH:154
SEQUENCE TYPE:nucleic acid
TOPOLOGY:linear
CLONE:HUMGS02838
SEQUENCE DESCRIPTION:
GATCGGGCCT AGCCCGGCAA CACTAATGTA GAAACCTTTT TTTTACAGAG CCTAATTAAT 60
AACTTAATGA CTGTNTACAT AGCAATGTGT GTGTATGTAT ATGTCTGTGA GCTATTAATG 120
TTATTAATTT TCATAAAAGC TGGAAAGCAG CAAA 154

SEQ ID NO:2469
SEQUENCE LENGTH:153
SEQUENCE TYPE:nucleic acid
TOPOLOGY:linear
CLONE:HUMGS02839
SEQUENCE DESCRIPTION:
GATCACCCGG GGCTGTCTGC ATACAGACTT CTCAGGGGAG TTCTCAGCTT GGACCCTTAT 60
CTCCCCAGAA TCCTGGAACC TGCTCCTTCT GCTCTCGTGA CTGACTGTGT TCTCTATGCA 120
ACTTCCAATA AAACCTCTTC ATTTGAAAGG AAA 153

SEQ ID NO:2470
SEQUENCE LENGTH:154
SEQUENCE TYPE:nucleic acid
TOPOLOGY:linear
CLONE:HUMGS02840
SEQUENCE DESCRIPTION:
GATCTTAAGC CCAGCTCTGT TCTGCCCCCG CTTTCCTCTG TTTGATACTA TAAATTTTCT 60
GGTTCCCTTG GATTTAGGGA TAGTGTCCCT CTCCATGTCC AGGAAACTTG TAACCACCCT 120
TTTCTAACAG CAATAAAGAG GTGTNCTTGT CAAA 154

SEQ ID NO:2471
SEQUENCE LENGTH:152
SEQUENCE TYPE:nucleic acid
TOPOLOGY:linear
CLONE:HUMGS02843
SEQUENCE DESCRIPTION:
GATCACAGGC CTTGCTTTTG TAACAATGAT GACCCCGGCC TGTCTCATAT TCTGAAGAGG 60
AAAAGTCAAA GTGTTGCTGN GGCTCCATAT TTCAACTAAA AATATATCTG TTGGAGAAAG 120
AAATTAACAA TAAAGAATTT TCATAGGTTA AA 152

SEQ ID NO:2472
SEQUENCE LENGTH:151
SEQUENCE TYPE:nucleic acid
TOPOLOGY:linear
CLONE:HUMGS02844
SEQUENCE DESCRIPTION:
GATCGTATTC CCTGCTTTAA GGAACTGNTA GNCTTGTTTG GGTGATGAGA CAAAGTTCTT 60
GAGACATTTA AACTGTATTT ACCAGTGTGA AAAATGTCAT TTATGGAGAT TAATTCATTA 120
TGGAAATAAA ACATTGCCTA AGCCCTTGAA A 151

SEQ ID NO:2473
SEQUENCE LENGTH:150
SEQUENCE TYPE:nucleic acid
TOPOLOGY:linear
CLONE:HUMGS02845
SEQUENCE DESCRIPTION:
GATCCAATCA GCTGTAAGTN TTAAGCACAG GGCTGAGGCT CCCTCCAAGT ACCCACTGGG 60
AACAGCAGCT TCAACCTGAG CCCTGNGTCC CAGCCAGCCC CCACAATCAC CTATGCCCAT 120
TCTTCAGAAT ACATTGGATG CATGTGCAAA 150

SEQ ID NO:2474
SEQUENCE LENGTH:149
SEQUENCE TYPE:nucleic acid
TOPOLOGY:linear
CLONE:HUMGS02846
SEQUENCE DESCRIPTION:
GATCCAGAGA GTCTGAAGAC ATTTCTNATG CCTTTATATA TTGGAGAACC CTTCCCCATT 60
TTNATATCCT TTCTTCAAAA ATCTAAATNA TGTGCCTAAA AATAAGACAT GGCATAATAA 120
GGTATAATTA AAGAGATAAT AGAATGAAA 149

SEQ ID NO:2475
SEQUENCE LENGTH:153
SEQUENCE TYPE:nucleic acid
TOPOLOGY:linear
CLONE:HUMGS02847

SEQUENCE DESCRIPTION:
GATCCAGTAC CTGAAAGATT TGATAGAAGA GGTCCGCAAG GCAAGGGGGA AGAAAAGGGT 60
CCCCTAGTTG AGGATAGTCA GGAGCGTCAA TGTGCTTGTA CATAGAGTGC TGTAGCTGTG 120
TGTTCCAATA AATTATTTTG TAGGGAAAGT AAA 153

SEQ ID NO:2476
SEQUENCE LENGTH:147
SEQUENCE TYPE:nucleic acid
TOPOLOGY:linear
CLONE:HUMGS02848
SEQUENCE DESCRIPTION:
GATCCTTTTG GAGTCAGACA GATGTGGTTG CATCCTAACT CCATGTCTCT GAGCATTAGA 60
TTTCTCATTT GCCAATAATA ATACCTCCCT TAGAAGTTTG TTGTGAGGAT TAAATAATGT 120
AAATAAAGAA CTAGCATAAC ACTCAAA 147

SEQ ID NO:2477
SEQUENCE LENGTH:207
SEQUENCE TYPE:nucleic acid
TOPOLOGY:linear
CLONE:HUMGS02849
SEQUENCE DESCRIPTION:
GATCTCCAGT GATTGGCGAT TTGACCTGGT TTCTNCCTGG ACTATTTGAA TGGGTGGATT 60
TGTTGGTGCA TCTCTTGTCT TCTTTCCTTT TCCATTCTCC TNTATTNTTN TATTTACATA 120
CTTGTATGCA ACTNTGTAAT CTGATTNCCC CATGTAACAT TNTAATTTGA NGGGTGGTCT 180
GCGTGTNCAC TGGAAAACAT ATNTATN 207

SEQ ID NO:2478
SEQUENCE LENGTH:146
SEQUENCE TYPE:nucleic acid
TOPOLOGY:linear
CLONE:HUMGS02850
SEQUENCE DESCRIPTION:
GATCGAGCTC ACAGGGGGCA CCCATCCTGN TAGTCAACAG AGTCTGAAGC AGTCAGGGTG 60
TTGGATTCCT CTGTTGTTGT CATCAATTCC TGCTGAGGGG TTTCTGGGGT TTTGTTTTTA 120
ATAAATAACT CCTTTGTAGC CTTAAA 146

SEQ ID NO:2479
SEQUENCE LENGTH:144
SEQUENCE TYPE:nucleic acid
TOPOLOGY:linear
CLONE:HUMGS02851
SEQUENCE DESCRIPTION:
GATCCCGGCC AGCCAGGTGA CCCCCACGCT CTGGATGTCT CTGCTCTGTT CCTTCCCCGA 60
GCCCCTGCCC CGGCTCCCCG CCAAAGCACC CCTGCCCACT CGGGCTTCAT CCTGCACAAT 120
AAACTCCGGA AGCAAGTCAG TAAA 144

SEQ ID NO:2480
SEQUENCE LENGTH:143
SEQUENCE TYPE:nucleic acid
TOPOLOGY:linear
CLONE:HUMGS02852
SEQUENCE DESCRIPTION:
GATCCAGGCA AATTCATGTT CTTTCTTTGT AACTCACTTT CTTCGTCAAC ATTTCTGTGA 60
CTCAATGGAT AGCATATTTC CATCAAGTAG CACGTATAAC GTGATGCTTT CATGTTTCTG 120
CCTTAAATAA AATAACCCTC AAA 143

SEQ ID NO:2481
SEQUENCE LENGTH:290
SEQUENCE TYPE:nucleic acid
TOPOLOGY:linear
CLONE:HUMGS02853
SEQUENCE DESCRIPTION:
GATCTCTTTG ACTGCTGTAG TACTAAAGTG TACTTAATGT TACTANGTTN AATGCCTGGC 60
CATTTTCCAT TTATATATAT TTTTTAAGAG GCTAGAGTGC TTTTAGCCTT TTTTAAAAAC 120
TCCATTTATA TTACATTTGT AACCATGATA CTTTAATCAG AAGCTTAGCC TTGAAATTGT 180
GAACTCTTGG AAATGTTATT AGTGAAGTTC GCAACTAAAC TAAACCTGTA AAATTATGAT 240
GATTGTATTC AAAAGNTTAA TGAAAANTAN ACATTTCTGT CCCCCTGAAA 290

SEQ ID NO:2482
SEQUENCE LENGTH:143
SEQUENCE TYPE:nucleic acid
TOPOLOGY:linear
CLONE:HUMGS02854
SEQUENCE DESCRIPTION:
GATCTGATTG AGATGTACAT AGTTTTTTTT TTTACCATAA CTGAATTATT TNATCTCTTA 60
TGTTAACATG AGAAATGTAT GCCAAATNAT TAGTTGATGT ATGTTTTTNA ATTTAATATT 120
TAAATAAAAT ATTTGGAAGG AAA 143

SEQ ID NO:2483
SEQUENCE LENGTH:269
SEQUENCE TYPE:nucleic acid
TOPOLOGY:linear
CLONE:HUMGS02855
SEQUENCE DESCRIPTION:
GATCCTTATC AGGGAGAGCA GGAATCCTTA TTCCCGGTGT CGCTAGTACT CATCTCTGCC 60
GCCTCCTGTC TGCCCCCAGG CTGCTGCTNC TGCTGCCCTG TGGGTTGTGC CAAGTGTGCC 120
CAGGGCTGCA TCTGCAAAGG GGCGTCGGAC AAGTGCAGCT GCTGCGCCTG ATGCTGGGAC 180
AGCNCGCTCC NAGATGTAAA GAACGCGACT TCCACAAACC TGGNNTTNNT TATGANCAAC 240
CCTGACNGTN ACCGTTTGCN ATATTNCNN 269

SEQ ID NO:2484
SEQUENCE LENGTH:140
SEQUENCE TYPE:nucleic acid
TOPOLOGY:linear
CLONE:HUMGS02856
SEQUENCE DESCRIPTION:
GATCATACAT TTTATATGGT TGGGACTTCT CTCTTCGGGA GATGATATCT TGTTTAAGGA 60
GACCTCTTTT CAGTTCATCA AGTTCATCAG ATATTTGAGT GCCCACTCTG TGCCCAAATA 120
AATATGAGCT GGGGATTAAA 140

SEQ ID NO:2485
SEQUENCE LENGTH:139
SEQUENCE TYPE:nucleic acid
TOPOLOGY:linear
CLONE:HUMGS02857
SEQUENCE DESCRIPTION:
GATCCAACCC CACCCTCCTG GGTGCGGCCA GAGGCAAGGC AGTCGCCCGA GCTCCTGAAT 60
CCCAAGAATG GTTCTGGCAA GTACTGCTGT TTGTTTGTAG GGGCAAAGAG TTAAAATAAA 120
ACGAGGTTCT GCCATGAAA 139

SEQ ID NO:2486
SEQUENCE LENGTH:139
SEQUENCE TYPE:nucleic acid
TOPOLOGY:linear
CLONE:HUMGS02858
SEQUENCE DESCRIPTION:
GATCTGCCGG CTCCCGTCCA GCCTGTGTGG AAAGGAGGCC CACGGGCACT AGGGANNCNG 60
AATTCTACAA TCCCGCTGGG GCGGCCGGGT CGGGAGAGAA AGGTGGTGCT NCAGTNGTGN 120
CCCTGGGGGG TCATTCGAN 139

SEQ ID NO:2487
SEQUENCE LENGTH:138
SEQUENCE TYPE:nucleic acid
TOPOLOGY:linear
CLONE:HUMGS02859
SEQUENCE DESCRIPTION:
GATCTTCAAT GCCTCTGAGT CATTGTTATA AAAAATCAGT TATCACTATA CCATGCTATA 60
GGAGACTGGG CAAAACCTGT ACAATGACAA CCCTGGAAGT TGCTTTTTTT AAAAAAATAA 120
TAAATTTCTT AAATCAAA 138

SEQ ID NO:2488
SEQUENCE LENGTH:231
SEQUENCE TYPE:nucleic acid
TOPOLOGY:linear
CLONE:HUMGS02860

SEQUENCE DESCRIPTION:
GATCATGGTT CTCTTTGTAC GATAGTTGGG CATCTGTATT TCCACTTGTG TGAATTTGCC 60
TTTAAATTTT GGTTATGGGT TTCACCTTTT AAAATAATCA AACATATTTA TCTTTTCCTG 120
TGTGATAGGT TTTTTNCTGT ATCTTTTCCT GTTAAACACA CAGACCCCTC CCCAATCTGG 180
ACATTGAATA CNTATTCATT TTCCTTTGCN NNAATANNCC NNANACANAC N 231

SEQ ID NO:2489
SEQUENCE LENGTH:137
SEQUENCE TYPE:nucleic acid
TOPOLOGY:linear
CLONE:HUMGS02861
SEQUENCE DESCRIPTION:
GATCTGCCCC TGCATTGTAG CTTGCTTAAC GGAGCACTTC TCCTTTTTCC AAAGGTCTAC 60
ATTCTAGGGT GTGGGCTGAG TTCTTCTGTA AAGAGATGAA CGCAATGCCA ATAAAATTGA 120
ACAAGAACAA TGATAAA 137

SEQ ID NO:2490
SEQUENCE LENGTH:135
SEQUENCE TYPE:nucleic acid
TOPOLOGY:linear
CLONE:HUMGS02862
SEQUENCE DESCRIPTION:
GATCTTTAAA TTTTGAGACA GTATAAGGAA AATCTGGTTG GTGTCTTACA AGTGAGCTGA 60
CACCATTTTT TATTCTGTGT ATTTAGAATG AAGTCTTGAA AAAAACTTTA TAAAGACATC 120
TTTAATCATT CCAAA 135

SEQ ID NO:2491
SEQUENCE LENGTH:286
SEQUENCE TYPE:nucleic acid
TOPOLOGY:linear
CLONE:HUMGS02863
SEQUENCE DESCRIPTION:
GATCCAAATA TTTTCAAGCC ATGTAATCCA TTGGTTTTGT GGGCAGTTTA ATAAACCTGA 60
ACCTTTGTGT GTTTTCTAAT TGTACCTGAG TTGACCATCC TTTCTTTTTA NAGTATATTT 120
CTNGTATGAT ATTNTGTAAA GCTCTCACCT GGTTCTTTTA TGGGGACTTT TCGTTTTTGG 180
GCAACTGCAG TGTATTTATG TGAANCTTTA TANGAGAATT ANTTTTTCCA TGTGCATATT 240
AATATGGTNC CTCCACACAT GGNNAGGGCA CAGTGGCTTC GTGTTN 286

SEQ ID NO:2492
SEQUENCE LENGTH:135
SEQUENCE TYPE:nucleic acid
TOPOLOGY:linear
CLONE:HUMGS02864
SEQUENCE DESCRIPTION:
GATCATGCCT CTTTCCAACA CGTGTTCACA ATCTCCAAAG GGACTGTATT TNTNCTCTGT 60

GCTTAATNTG ATTTGAAATA TGTTGAATCA AAGTGAAATA TTTATNTTTT GAATAAAGGA 120
GATAATAGCC TTAAA 135

SEQ ID NO:2493
SEQUENCE LENGTH:135
SEQUENCE TYPE:nucleic acid
TOPOLOGY:linear
CLONE:HUMGS02865
SEQUENCE DESCRIPTION:
GATCGAAAAC AAACGNTAGA TAATNCCCAA GGAGCTTACC AAGAGGCATT TNATATAAGC 60
AAGAAAGAGA TGCAACCCAC ACACCCAATC CGCCTGGGGN TTGCTCTTAA CTTTCCNGTA 120
TTTNACTATG AGATN 135

SEQ ID NO:2494
SEQUENCE LENGTH:135
SEQUENCE TYPE:nucleic acid
TOPOLOGY:linear
CLONE:HUMGS02866
SEQUENCE DESCRIPTION:
GATCATGCCA CTNCACTCAC ACTGACACTG CCTCGGACAA CAGAGCAANA CTNCATCTCA 60
AAAACAAACA AAAAAAATCA CAGNCTCATT TTAAAGGAGC AGCAACCNAA CCAAATACAA 120
NCTTCATTTG ACAAN 135

SEQ ID NO:2495
SEQUENCE LENGTH:134
SEQUENCE TYPE:nucleic acid
TOPOLOGY:linear
CLONE:HUMGS02867
SEQUENCE DESCRIPTION:
GATCATTAAC ATGCCAGGGC AGTTCCCACT GATTTAGATG GTCCAAGATA ATCTCATTCA 60
GGAGGCTTGA AACATTAATG GTTTAGTCTT GTGAATTTTA ACAGTTCTCT GTCATCGTTT 120
AACAAAACCA ACAN 134

SEQ ID NO:2496
SEQUENCE LENGTH:134
SEQUENCE TYPE:nucleic acid
TOPOLOGY:linear
CLONE:HUMGS02868
SEQUENCE DESCRIPTION:
GATCATGCCA CTGGACTCTG TGCTGAGCAA CAGAGTTAGA CCCTGTCTCT AAATAAATAA 60
ATAAACAAAT AAACAGTAAA AAAATGTATA GTTCAATCCT TATCTGTATT AAACTTCCTT 120
TAACTTGTGT GAAA 134

SEQ ID NO:2497
SEQUENCE LENGTH:133

SEQUENCE TYPE:nucleic acid
TOPOLOGY:linear
CLONE:HUMGS02869
SEQUENCE DESCRIPTION:
GATCACATGT GGGCCCGTGT CTGGCCGCCG CCGCCCNGCC CCGTCCCTGC GGNCACCACC 60
TAATTTATTG CCGTGCGTCC TGCTGCTGTG ACTGCTTTTG TACCTTTGCA ATAAAGAATT 120
TTCTGGTTTC AAA 133

SEQ ID NO:2498
SEQUENCE LENGTH:132
SEQUENCE TYPE:nucleic acid
TOPOLOGY:linear
CLONE:HUMGS02870
SEQUENCE DESCRIPTION:
GATCCTGGAC ATATTTTATA TTTATACCAA ATATTTTATA TATTTNCTAC TGTTGTCAAT 60
GGTAGTGGTT TTTAAATTTT CAAACATTCA TTACTAGTAT ATAGAAATAN AATCACTTTT 120
TAAAAATGTA AA 132

SEQ ID NO:2499
SEQUENCE LENGTH:131
SEQUENCE TYPE:nucleic acid
TOPOLOGY:linear
CLONE:HUMGS02872
SEQUENCE DESCRIPTION:
GATCCTGTTT TTTCTTAACA AGTTGAGGCG TGGGTAGAGC AGGAATTGGT TTTCCAGCAT 60
TGTGTCCGTA AACCTGAGTT AGAATAAGAT GTAACGGAAG CCACGATAAA GACTCGGTCA 120
AATCCTGCAA A 131

SEQ ID NO:2500
SEQUENCE LENGTH:133
SEQUENCE TYPE:nucleic acid
TOPOLOGY:linear
CLONE:HUMGS02873
SEQUENCE DESCRIPTION:
GATCCTGGAT GGAACTGGCT TCTTTATCGA GAAGAATATA ATTCTCCATG AGGACTTAAT 60
GAATCCAAAC CTGTGTCATG CCTGGTTTGC ATACCCAATT AAACACTNGA AATAAAAATT 120
NTTNGGGTNN AAA 133

SEQ ID NO:2501
SEQUENCE LENGTH:130
SEQUENCE TYPE:nucleic acid
TOPOLOGY:linear
CLONE:HUMGS02874
SEQUENCE DESCRIPTION:
GATCTCTGCT CTTTGTCAGA GATTTGCAAA GACTCACGTT TTTNTTGTTT TCTCATCATT 60

CCATTGTGAT ACTAAGAAAC TAAGAAGCTT AATGAAAAGA AATAAAATGC CTATGTTGTT 120
GTTCTAGAAA 130

SEQ ID NO:2502
SEQUENCE LENGTH:130
SEQUENCE TYPE:nucleic acid
TOPOLOGY:linear
CLONE:HUMGS02875
SEQUENCE DESCRIPTION:
GATCAAAAAA GGCAGATGNA ANTACTTAAN ACTCAGTGTT TTGGAGAGAT TTCCTTTNAG 60
TTTGTTGGTT GGCTGGTTTG AACGATAGAA ATATGCAGCA TGCAATATAT GCTTATATTT 120
NATTTNAATN 130

SEQ ID NO:2503
SEQUENCE LENGTH:129
SEQUENCE TYPE:nucleic acid
TOPOLOGY:linear
CLONE:HUMGS02876
SEQUENCE DESCRIPTION:
GATCCCCCAC CTGGCAGAGG ACCCAGTACA AAATAGGCAC TCAATAGATG TTACACCAAC 60
TTTGGAAGGG CAAACATATT TCTTAATGAG AGGCAGTCCT TCATGTTTTN CAATAAAATG 120
ACTTTTAAA 129

SEQ ID NO:2504
SEQUENCE LENGTH:129
SEQUENCE TYPE:nucleic acid
TOPOLOGY:linear
CLONE:HUMGS02877
SEQUENCE DESCRIPTION:
GATCTTCTCC GAGGCCCACC GAAGGTACTG AAGAGCCTCA CCTGGGGGCA TTTTGTGGGT 60
GGAGGGCCAG AGTGTGTATA CCCAGGCTTG TCTGGAAGGA GAAGGCCTTT GCTGCCTGAA 120
AGTCTCAAA 129

SEQ ID NO:2505
SEQUENCE LENGTH:129
SEQUENCE TYPE:nucleic acid
TOPOLOGY:linear
CLONE:HUMGS02878
SEQUENCE DESCRIPTION:
GATCTAAATA AGTGTTTCAA GGAAAGTTTT CCTAAGCAAA TGTAATATTA CCTCATTTGG 60
GCATCATTAC TCTGTTAATT CTATATCAAA GGAAATAAAC TTGCTACTTG CACTAAATGA 120
AAAAACAAA 129

SEQ ID NO:2506
SEQUENCE LENGTH:128

SEQUENCE TYPE:nucleic acid
TOPOLOGY:linear
CLONE:HUMGS02879
SEQUENCE DESCRIPTION:
GATCGTGCCC CGGGGCGCCG GCCCCTGNAG GGGCTCACCT GGATGGGGCC TGCAGTGCGT 60
TCCCGCTTTG CTTCCTTCCC TGGACGGCCC GCTCCCNGAA ACGCGCGCAA TAAAGTGATT 120
CGCAGAAA 128

SEQ ID NO:2507
SEQUENCE LENGTH:127
SEQUENCE TYPE:nucleic acid
TOPOLOGY:linear
CLONE:HUMGS02880
SEQUENCE DESCRIPTION:
GATCACTCTT GTGGTTTTGG GGGATGGCAT TATAAGAGAG GAGACTGGAN ACAGGGGGGC 60
TATTGTNATG CAGATTTGTT GGATGTCCAC CCAGCATTCA CTTTTNCCAT CTTTTGCCAG 120
GGGNNNN 127

SEQ ID NO:2508
SEQUENCE LENGTH:128
SEQUENCE TYPE:nucleic acid
TOPOLOGY:linear
CLONE:HUMGS02881
SEQUENCE DESCRIPTION:
GATCCCACGC AGCGCTGAAC CGAACCGAGT AGGAAGCCTT TCTCCCCANG GCACGTGGCT 60
TCAGGGCGTT TCCCATTGAC CAAGTTTGAC CCTGNTTTGA ATAAAGAGAA GTGCGTTTGG 120
ATTAGAAA 128

SEQ ID NO:2509
SEQUENCE LENGTH:129
SEQUENCE TYPE:nucleic acid
TOPOLOGY:linear
CLONE:HUMGS02882
SEQUENCE DESCRIPTION:
GATCGAGCTT CTCCTCTGAG TTCTAACAAA ATGGTGCTTT GAGGGTCAGC CNTTAGGAAG 60
GTGCAGCTTT GTTGTCCTTT GAGCTTTCTG TTATGTGCCT ATCCTAATAA ACTCTTAAAC 120
ACATTGAAA 129

SEQ ID NO:2510
SEQUENCE LENGTH:125
SEQUENCE TYPE:nucleic acid
TOPOLOGY:linear
CLONE:HUMGS02883
SEQUENCE DESCRIPTION:
GATCTCTGAT TTAACCGTGT ACTATCCACA TGCATTACAA ACATTTCGCA GAGCTGCTTA 60

GTATATAAGC GTACAATGTA TGTAATAACC ATCTCATATT TAATTAAATG GTATAGAAGA 120
ACAAA 125

SEQ ID NO:2511
SEQUENCE LENGTH:124
SEQUENCE TYPE:nucleic acid
TOPOLOGY:linear
CLONE:HUMGS02884
SEQUENCE DESCRIPTION:
GATCTTCCAT CTCCTCAGCA AAAAAATAGG AGCCCTGGCC CCCCAACTTT TTNCANAGTA 60
ATAGCCTTAN TTCCTTCCCT ATCTCCTTAC CAAAGTACAN GTNACATCTT TCCCACCTTT 120
CNTN 124

SEQ ID NO:2512
SEQUENCE LENGTH:124
SEQUENCE TYPE:nucleic acid
TOPOLOGY:linear
CLONE:HUMGS02885
SEQUENCE DESCRIPTION:
GATCTACAGA TTCAATGCAA TTNCTATTAA AATCTNATCT GGCTTTTTTT GCATAAGCAA 60
CAAAGGAAAA ANTAAGACAT CAAAATTTNA AGANCTTTTG TCCTACAAAC AATATCATGA 120
NGAN 124

SEQ ID NO:2513
SEQUENCE LENGTH:343
SEQUENCE TYPE:nucleic acid
TOPOLOGY:linear
CLONE:HUMGS02886
SEQUENCE DESCRIPTION:
GATCAGAAAA TTCATATTTA AGCAAAGTGA TACAAACACA GTGATTTGGG AATGCCTTCA 60
TTTACAATGC AATACTTAAA TTTTAATACT CTTGTAGGAG AAAAAGCAAC TGTATAAATG 120
AATGTAGAGT GACTTTCTGC AATATTTCAA ACCTATATCA GAGAATTACA CTGTGGGAAA 180
NCTACCATTG TAATAAGTGT AGCAAAATCT CCTTAGATAT CTGAAAAGTC ATACTGGATG 240
GAATCTGTAG GAAACGGTTC TATTTTGAGG GAAGGGGGAT TCCTTTTTGT TTTTTAAGTG 300
AATTCAGAAA ATGTTATAAA TAANTCTTTT GGTTTATTAT AAA 343

SEQ ID NO:2514
SEQUENCE LENGTH:123
SEQUENCE TYPE:nucleic acid
TOPOLOGY:linear
CLONE:HUMGS02887
SEQUENCE DESCRIPTION:
GATCTNTACC CCACCCCTAT CTAACACCAC CCTTGGNTCC CACTCCAGCT CCCTGTATTG 60
ATATAACCTG TNAGGCTGGC TTGGTTAGGT TTNACTGGGG CAGAGGATAG GGAATCTNTN 120
ATN 123

SEQ ID NO:2515
SEQUENCE LENGTH:121
SEQUENCE TYPE:nucleic acid
TOPOLOGY:linear
CLONE:HUMGS02888
SEQUENCE DESCRIPTION:
GATCCAAAAT TACCTTCCTA TTGCATTTCC TACTCCTTTG CTACGNGTAT TGGACCTCTG 60
GCCAAATNAC AAAAGATGTA CTCTTTGTNC TCTAAAACCA CACTAAAGTT TTCTNACTNN 120
N 121

SEQ ID NO:2516
SEQUENCE LENGTH:122
SEQUENCE TYPE:nucleic acid
TOPOLOGY:linear
CLONE:HUMGS02889
SEQUENCE DESCRIPTION:
GATCAACCAA CCTTAAATAT ATGTATGTAT ATATGTATAT GTGAAAAACA GTTTGTATAG 60
TTGGAATATT TGTNTTTGTA ATTACTTGTG ATGTTTTAAA ATAAAANTTT TATTCAGTTA 120
AA 122

SEQ ID NO:2517
SEQUENCE LENGTH:122
SEQUENCE TYPE:nucleic acid
TOPOLOGY:linear
CLONE:HUMGS02890
SEQUENCE DESCRIPTION:
GATCTGGCAC CCCCTGGGTG GAGTGTCCCT CGGGGGCTTT GGGAAAGCAT GGCACCCTCA 60
GACCACACAG TAGCCAAGTT CTGGAGCAAA TAAAAGGCCT GTGTTATNTC TTGTNCTTGA 120
AA 122

SEQ ID NO:2518
SEQUENCE LENGTH:119
SEQUENCE TYPE:nucleic acid
TOPOLOGY:linear
CLONE:HUMGS02891
SEQUENCE DESCRIPTION:
GATCTCAAAG ATATGCACCA GTTTGAATAA ATGCTTGTTT AAAGATTAAA TTTTACAATT 60
CATTTTAAAT TNTCATTTTG TGACAATATT TCTAAACAAC GNCCATTTTA ATNACCAAA 119

SEQ ID NO:2519
SEQUENCE LENGTH:118
SEQUENCE TYPE:nucleic acid
TOPOLOGY:linear
CLONE:HUMGS02893

SEQUENCE DESCRIPTION:
GATCATAGGA TAGCTGACTT TGACAGTCAC ATTTATAAAG TAATTCACTT AAAGATATAT 60
ATTTTTTTCA AACAAGTTTT GCTACTTTTG AAAATAAATC TTTCTTTATA TTGCTAAA 118

SEQ ID NO:2520
SEQUENCE LENGTH:118
SEQUENCE TYPE:nucleic acid
TOPOLOGY:linear
CLONE:HUMGS02894
SEQUENCE DESCRIPTION:
GATCCAAACC CATCTTCCTG GACCTGAAGC TTATGCTTCC AGGCACCCCA CTCCTGAGCT 60
GAATAAAGAT GATTTAAGCT TAATAAATCG TGAATGTGTT CACATGAGTT TCCATAAA 118

SEQ ID NO:2521
SEQUENCE LENGTH:118
SEQUENCE TYPE:nucleic acid
TOPOLOGY:linear
CLONE:HUMGS02895
SEQUENCE DESCRIPTION:
GATCTGCACA GTGTTATGGC ACCCTACTTC CTGCAGCAAC GGGACACNCT GCGGCGCCAT 60
GTNCAGAAAC AGGAGGCCGA GANCCAGCAG CTGGCAGATG CCGTCCTNNC AGGGCGGN 118

SEQ ID NO:2522
SEQUENCE LENGTH:118
SEQUENCE TYPE:nucleic acid
TOPOLOGY:linear
CLONE:HUMGS02896
SEQUENCE DESCRIPTION:
GATCCTATCT TTNCTGGCAG GACTGGNAGC CATGGTGGGC TACGTTNTGN TCAGCGGCAT 60
TNTTTCCATC CAGCGGGCAA CGCTGCTCGG GCCCCAGGCA CCCGGACCCT GGGCATGN 118

SEQ ID NO:2523
SEQUENCE LENGTH:117
SEQUENCE TYPE:nucleic acid
TOPOLOGY:linear
CLONE:HUMGS02897
SEQUENCE DESCRIPTION:
GATCTAGTCC GGACTTGCTG TGTATATTGT AACGTTAAAT GAAAAAAGAA CCCCCCTTTG 60
TATTATAGTC ATGCGGTCTT ATGTATGATN AACAGTTGAA TAATTTGTCC TCAGAAA 117

SEQ ID NO:2524
SEQUENCE LENGTH:116
SEQUENCE TYPE:nucleic acid
TOPOLOGY:linear
CLONE:HUMGS02898

SEQUENCE DESCRIPTION:
GATCGTNATT NAGAATCCAT GTGAATAGAC CTGCGATGGC CAAGGCTGTT TAAGCNACAC 60
TGGGTTGGAA ACACTTGGGC TCTCTCATNA TTATTAAAAT TCTATTTCAA TCTAAA 116

SEQ ID NO:2525
SEQUENCE LENGTH:116
SEQUENCE TYPE:nucleic acid
TOPOLOGY:linear
CLONE:HUMGS02899
SEQUENCE DESCRIPTION:
GATCCTTTGC NTTCAAGACT AAGTTAATAG AGAAGATGTT TGTAACTNTG GCCCAAGCTT 60
GGNCTCCCAG TGTATTGAAG CTAACTCCTT TNAGAAGCAC CACTCTCATA TATNAN 116

SEQ ID NO:2526
SEQUENCE LENGTH:117
SEQUENCE TYPE:nucleic acid
TOPOLOGY:linear
CLONE:HUMGS02900
SEQUENCE DESCRIPTION:
GATCTCTGGG TAAAGAGGCC GTTTATCTTT GTAAACACGA AACATTTTTC CTTTCTCCAG 60
TTTTCTGTTA ATGGCGAAAG AATGGAAGCG AATAAAGTTT TACTGATTTT TGAGAAA 117

SEQ ID NO:2527
SEQUENCE LENGTH:119
SEQUENCE TYPE:nucleic acid
TOPOLOGY:linear
CLONE:HUMGS02901
SEQUENCE DESCRIPTION:
GATCTAGGAA GACTTTGGAA AGAGTAGGAA GACTTTGGAA AGACTTTTCC AACCCTCATC 60
ACCAACGTCT GTGCCATTTT NTATTTTACT AATAAAATTT AAAAGTNTTG TGAATCAAA 119

SEQ ID NO:2528
SEQUENCE LENGTH:115
SEQUENCE TYPE:nucleic acid
TOPOLOGY:linear
CLONE:HUMGS02902
SEQUENCE DESCRIPTION:
GATCCCCAGG AGGCCTGGNC CGCCCAGAGG CTCCTCTCAG GCTGGGCCCC GACGTTTGCG 60
NCAGTGTTCC TTGTGCCCGT GGGGCCGGGA GCNAGTAAAG TCTGGGCCAG GCAAA 115

SEQ ID NO:2529
SEQUENCE LENGTH:119
SEQUENCE TYPE:nucleic acid
TOPOLOGY:linear
CLONE:HUMGS02903

SEQUENCE DESCRIPTION:
GATCACGTTT CAGGGTCCTC CAAAANGCTA GCCCGGCACT ACACTAGGGA ATTTCNCCCT 60
CTCCTGTCAC TTGTCATGGT CTTCCTTGGT ATTAAAGGCC ACCATTTGCA CAAAACAAA 119

SEQ ID NO:2530
SEQUENCE LENGTH:114
SEQUENCE TYPE:nucleic acid
TOPOLOGY:linear
CLONE:HUMGS02904
SEQUENCE DESCRIPTION:
GATCCCAGAA ATGTAGCTTG TTTCATATTT TAGTCTTCTT ATTTTTGTAA AATGTGTAGA 60
ATTTGCTGTT TTTNTTTTTC TTTTGACAAC TCAGGAAGAA ACTGACCTCA GAAA 114

SEQ ID NO:2531
SEQUENCE LENGTH:119
SEQUENCE TYPE:nucleic acid
TOPOLOGY:linear
CLONE:HUMGS02905
SEQUENCE DESCRIPTION:
GATCAGCCCC CAAAGAAATG GGGGTGCATG CTGGGCCTGG CCCCCTGGCC CACCCCCACT 60
TTCCAGGGCA AAAAGGGCCC AGGGTTATAA TAAGTAAATA ACTTGTNTGT ACAGCCAAA 119

SEQ ID NO:2532
SEQUENCE LENGTH:113
SEQUENCE TYPE:nucleic acid
TOPOLOGY:linear
CLONE:HUMGS02906
SEQUENCE DESCRIPTION:
GATCTTGGCT TCTCAACAGG GCAAAGATAC CAGGCCTNCT GCTGAGGTCA CTGCCACTTC 60
TCACATNCTG CTTAAGGGAG CACAAATAAA GGTATTCGAT TTTTAAAGAT AAA 113

SEQ ID NO:2533
SEQUENCE LENGTH:348
SEQUENCE TYPE:nucleic acid
TOPOLOGY:linear
CLONE:HUMGS02907
SEQUENCE DESCRIPTION:
GATCAAAATG TTTGAAGAAA GGAACTTTAT TTTTGCAAGT TACGTACAGT TTTTATGCTT 60
GAGATATTTC AACATGTTAT GTATATTGGA ACTTCTACAG CTTGATGCCT CCTGCTTTTA 120
TAGCAGTTTA TGGGGAGCAC TTGAAAGAGC GTGTGTACAT GTATTTTTTT TCTAGGCAAA 180
CATTGAATGC AAACGTGTAT TTTTTTAATA TAAATATATA ACTGTCCTTT TCATCCCATG 240
TTGCCGCTAA GTGATATTTC ATATGTGTGG TTATACTCAT AATAATGGGC CTTGTAAGTC 300
TTTTCACCAT TCATGAATAA TANTANATAT GTACTGCTGG CATGNAAA 348

SEQ ID NO:2534

SEQUENCE LENGTH:111
SEQUENCE TYPE:nucleic acid
TOPOLOGY:linear
CLONE:HUMGS02908
SEQUENCE DESCRIPTION:
GATCTGATGG TTTTAAAAAT GGGAGCTTCC CTNCACAGAC TCTNTCTCTC GTGCCGCTGC 60
CATGGCCATG TAAGNAGTGC CTTTNACCTT CCCCCGTGAT TGTNAGGCCT N 111

SEQ ID NO:2535
SEQUENCE LENGTH:110
SEQUENCE TYPE:nucleic acid
TOPOLOGY:linear
CLONE:HUMGS02909
SEQUENCE DESCRIPTION:
GATCCCAACA GGACAAATCT GTGTACCTAT CTTTCTATCC TCCCTGAGCC TGTACCAACT 60
AGATTCTTCA TATGTATTTG TAACAGATTA AATTGGAAAG CAAACACAAA 110

SEQ ID NO:2536
SEQUENCE LENGTH:110
SEQUENCE TYPE:nucleic acid
TOPOLOGY:linear
CLONE:HUMGS02910
SEQUENCE DESCRIPTION:
GATCCCCAAC TGCTCCAGCC TCTGCCCAAC TGAATCTTCA GCATGTTCTC ATCGGCGGAC 60
TNTCTTGTGT AATGTAAACT GTGCCATGTT ATTAAAAAAT GTGAACTAAA 110

SEQ ID NO:2537
SEQUENCE LENGTH:110
SEQUENCE TYPE:nucleic acid
TOPOLOGY:linear
CLONE:HUMGS02911
SEQUENCE DESCRIPTION:
GATCTGTTTG GAAAACATAG ATTGTTTTCC CCTCAAATAA GGGGAAAAAA AAANACCCTT 60
TNTNCAAATN GNTTCTTTTG TAAAAAATTA TTTTAAAAGG AAATCACAAN 110

SEQ ID NO:2538
SEQUENCE LENGTH:109
SEQUENCE TYPE:nucleic acid
TOPOLOGY:linear
CLONE:HUMGS02912
SEQUENCE DESCRIPTION:
GATCTTTTTA ACTCTACTGT GTCATTAAGA TGCTTTGTAC AATGCCAAAC CATAGTTGGA 60
GAAACTAAGT TTAATAAATA TCAAATAGAT TTTCTGTATT AAAGTTAAA 109

SEQ ID NO:2539

SEQUENCE LENGTH:109
SEQUENCE TYPE:nucleic acid
TOPOLOGY:linear
CLONE:HUMGS02913
SEQUENCE DESCRIPTION:
GATCTCCCCC ACCCTTGGAT TAGAGTTCCT GCTCTACCTT ACCCACAGAT AACACATGTT 60
GTTTCTACTT GTAAATGTAA AGTNTTTAAA ATAAACTATT ACAGATAAA 109

SEQ ID NO:2540
SEQUENCE LENGTH:109
SEQUENCE TYPE:nucleic acid
TOPOLOGY:linear
CLONE:HUMGS02914
SEQUENCE DESCRIPTION:
GATCTCCTNT CCCTCACTCA GTGCACGGGA CAGCTNCTCC TCCGTCACAG CTTTGGNATG 60
AAAGACAGAA GACAGCATGA TTGGCCTCAC CCACGTGCAG GNCAAATNN 109

SEQ ID NO:2541
SEQUENCE LENGTH:107
SEQUENCE TYPE:nucleic acid
TOPOLOGY:linear
CLONE:HUMGS02915
SEQUENCE DESCRIPTION:
GATCTGTCAC TCAACTTGCT GCGTNACCTG AACACGTCAC TTTACCTCTC TGTGCCTCAG 60
TTTTCCCATG CATGAAAAAT AAAATAAAAT AAAACGGGGA TTCTAAA 107

SEQ ID NO:2542
SEQUENCE LENGTH:112
SEQUENCE TYPE:nucleic acid
TOPOLOGY:linear
CLONE:HUMGS02916
SEQUENCE DESCRIPTION:
GATCAGTAAG GATTTCACCT CTGTTTGTAA CTAAAACCAT CTACTATATG TTAGACATGA 60
CATTCTTTTT CTCTCCTTCC TGAAAAATAA AGTGTGGGAA GAGACAAATA AA 112

SEQ ID NO:2543
SEQUENCE LENGTH:109
SEQUENCE TYPE:nucleic acid
TOPOLOGY:linear
CLONE:HUMGS02917
SEQUENCE DESCRIPTION:
GATCATAGAA GGTTGGGGTT CCAGAAAGGC ATCTGTGTGA TGGTTCCATT GATGTGGGAT 60
TTCCCTACTT GCTGTATTCT CAGTTTCTAA TAAAAAGAAC CAAATNAAA 109

SEQ ID NO:2544

SEQUENCE LENGTH:108
SEQUENCE TYPE:nucleic acid
TOPOLOGY:linear
CLONE:HUMGS02919
SEQUENCE DESCRIPTION:
GATCCAGGAG ATANTTTTTT CATTCATCCT GACCAAGACT GAGCCAGCTT AGCAACTNCT 60
GGGGAGACAA ATNTNAGAAC CTTGTCCCAG CCAGTNAGGA TGACAGTN 108

SEQ ID NO:2545
SEQUENCE LENGTH:343
SEQUENCE TYPE:nucleic acid
TOPOLOGY:linear
CLONE:HUMGS02920
SEQUENCE DESCRIPTION:
GATCGAGCCC AGCGTACAAC TTCCTCAAGT CGCCCAAAGA CAACGTGGAC GACCCCACGG 60
GGAACTTCCG CAGCGGGCCC CTGACGGGGT GGCGGGTGTT CCTGCTGCNN NNGTGCGCTC 120
TCCTGGGCAT CGTTGTCTGC GCCGTGGTGG GGGCCGTGGT GTTCCAGAAG CGGCAGGAGC 180
GGAACAAGCG CTTCTACTGA GTGGCGCCTC CGGCGGTGGC CTGTCCCTGG GCCCAGGAGC 240
CAATGTGAAC TTTTTTGGNT ACCGGGATTA TAAANGAACA ACAAGATGAC CTTATTTCTT 300
AACTGTTTCA AATAAAATGA TTAAAAGTAT TTTCAAAAAA AGN 343

SEQ ID NO:2546
SEQUENCE LENGTH:107
SEQUENCE TYPE:nucleic acid
TOPOLOGY:linear
CLONE:HUMGS02921
SEQUENCE DESCRIPTION:
GATCCTGCCG TGCCCCCATC CTGGCTGTTG GGGGTGTCCT GAGCCCACCT NNNTNCGCCT 60
GTTCCCTTCA GCCAACCCGT TTCTGCAGTA AAATTAAGCC TGTCAAA 107

SEQ ID NO:2547
SEQUENCE LENGTH:105
SEQUENCE TYPE:nucleic acid
TOPOLOGY:linear
CLONE:HUMGS02922
SEQUENCE DESCRIPTION:
GATCACTGAA CCAGACACAG CATTGCTGAC ACATGAGACT AACACGTGCA ATTATTTAAA 60
AAGATTTCAA TAAAACTNCC TGGCTGGCTC CGGGCCGCCC CTAAA 105

SEQ ID NO:2548
SEQUENCE LENGTH:103
SEQUENCE TYPE:nucleic acid
TOPOLOGY:linear
CLONE:HUMGS02923
SEQUENCE DESCRIPTION:

GATCCTGTGG GTTTAGTATC ATTATCTTCA GCTCTTTGAT ACNCTGTGTT AGAGTAATAG 60
CTAAAGGAAG TTCATGTCAA TAAATTCATA CTTATATCAC AAA 103

SEQ ID NO:2549
SEQUENCE LENGTH:104
SEQUENCE TYPE:nucleic acid
TOPOLOGY:linear
CLONE:HUMGS02924
SEQUENCE DESCRIPTION:
GATCCAGAGC TGGGGTGGAA GGAGAGCCAT TCTGAACGNC ACGNCTGGCC CGGTCAGTGC 60
TGCATGCACT GCATATNAAA TAAAATCTGC TACACGCCAG GAAA 104

SEQ ID NO:2550
SEQUENCE LENGTH:103
SEQUENCE TYPE:nucleic acid
TOPOLOGY:linear
CLONE:HUMGS02925
SEQUENCE DESCRIPTION:
GATCCCNAGG CTCTGTTCCA TGAGCCTGTT GCANGTTTTG GTACTTTAGA AATGTAACTT 60
TTTGCTCTTA TAATTTTATT TTATTAAATT AAATTACTGC AAA 103

SEQ ID NO:2551
SEQUENCE LENGTH:102
SEQUENCE TYPE:nucleic acid
TOPOLOGY:linear
CLONE:HUMGS02926
SEQUENCE DESCRIPTION:
GATCGATGAG GCAGGTCAGT TGTAAGTGAG TCACATTGTA GCATTAAATT CTAGTATTTT 60
TGTAGTTTGA AACAGTAACT TAATAAAAGA GCAAAAGCTA AA 102

SEQ ID NO:2552
SEQUENCE LENGTH:102
SEQUENCE TYPE:nucleic acid
TOPOLOGY:linear
CLONE:HUMGS02927
SEQUENCE DESCRIPTION:
GATCATATCT AGTNTTNAAA TCTCTAGGAA CAATAGAGAT TATATCATAA CTCTGAAGAT 60
GGACTCTAAA CTTTGGTTTA CCTCCATGGA AAATAANCGN NN 102

SEQ ID NO:2553
SEQUENCE LENGTH:101
SEQUENCE TYPE:nucleic acid
TOPOLOGY:linear
CLONE:HUMGS02928
SEQUENCE DESCRIPTION:

GATCGAATGA GGGTTAAATG AATTATTTCA TGTAAAGCTC TTAGCCCTGT GCCTGGTACA 60
TAGTAAATNC TAAATAAATA GTGGTATTCT TACTGTTTAA A 101

SEQ ID NO:2554
SEQUENCE LENGTH:390
SEQUENCE TYPE:nucleic acid
TOPOLOGY:linear
CLONE:HUMGS02929
SEQUENCE DESCRIPTION:
GATCCAGCTT ATTCTTTTAT TTTCAAGTCC ATTCTTGGGG CTGGTGGGGA GGCAGGAGAA 60
TACCCCTCCC TAAGCCCTTA GTGTGTGCCG AGCTTGCTTT NTGATGTTGG CAGGGGAGGG 120
GAGACCTGGG TGGTGNCTGA GTTCCCTTTA TCAAACCCTT CAATGGGCAC AAAATTGAGT 180
GCTTNNTTNN TAGGTTTTAT TTNNNNATGA ATGTCCAAAT CTGTGTTTCC CCCTGCCANA 240
ACAGACTGTG TGGCCAGTTG AAAGTGTCTT GGTTTGTGGT TCATCTCTCC CTCATTTTCT 300
TGGAGGCAGG GCCTGAGANC CCTGNCANAA TCTCCTATGG TTNTGAATCC ACGGCTTCTT 360
TTTGGACATT AAAGGTTGAT TTGATGCAAA 390

SEQ ID NO:2555
SEQUENCE LENGTH:227
SEQUENCE TYPE:nucleic acid
TOPOLOGY:linear
CLONE:HUMGS02930
SEQUENCE DESCRIPTION:
GATCTATCCT CAGCACTGCT GAGTGTGCAG TCACACTTTC CTACCAACCC NCTTCTTACC 60
ATCTCTAGCT GCCATTTGTG GGGGGAACAA AACAAGGGAA TCCTGATTGT GTACAGTATA 120
AATTGTATTA TATTTTTTGT ACTTATGTTT TATGTAAATA GTTTGTCTCA TTCAATTGTA 180
TGTGAGCATT GAAATAAATC CTACCATTTA GGGGAAAAAA ATNNAAA 227

SEQ ID NO:2556
SEQUENCE LENGTH:106
SEQUENCE TYPE:nucleic acid
TOPOLOGY:linear
CLONE:HUMGS02931
SEQUENCE DESCRIPTION:
GATCTTCAAA AATAATGCCT CTGTCTAGCA TGCCAACAAG AATGCATTGA TATTGTGAAC 60
ATTTGTGATA TATGTATTAA TAAATAGAGC AATTACAAGC AGCAAA 106

SEQ ID NO:2557
SEQUENCE LENGTH:99
SEQUENCE TYPE:nucleic acid
TOPOLOGY:linear
CLONE:HUMGS02932
SEQUENCE DESCRIPTION:
GATCTTTCTT ACACAGTGAT TCATTCCTCT ATTTGTACAG TGGCTTTATG AAATATTGTG 60
AATTGATACT GTAATCAATA AAGTGCTTTT AACCAGAAA 99

SEQ ID NO:2558
SEQUENCE LENGTH:98
SEQUENCE TYPE:nucleic acid
TOPOLOGY:linear
CLONE:HUMGS02933
SEQUENCE DESCRIPTION:
GATCCTGGTT CCCTGTTTGG AAAATTCACC ACCTCTAGCT CCTCACTGTT CTTTGTAATT 60
AACACGCTGT TGGTAATCTT ATTAATTATT TAACCAAA 98

SEQ ID NO:2559
SEQUENCE LENGTH:97
SEQUENCE TYPE:nucleic acid
TOPOLOGY:linear
CLONE:HUMGS02934
SEQUENCE DESCRIPTION:
GATCTGGGGA CCCTGACTGC ACAGAACAGT GGGCGCNTTA CAGACCACCG ACCGCCCGAG 60
GGTGAATTAC ATCTTAATAA AGCTACTGTG AGAGAAA 97

SEQ ID NO:2560
SEQUENCE LENGTH:93
SEQUENCE TYPE:nucleic acid
TOPOLOGY:linear
CLONE:HUMGS02935
SEQUENCE DESCRIPTION:
GATCGCCGCC AACATCCAGG CTGACTACTT GTATCGGTGA CGCCCCACCG GCCCGCAGCC 60
CCTGCTGCCC AATAAAACCA CTCCGACCCC AAA 93

SEQ ID NO:2561
SEQUENCE LENGTH:92
SEQUENCE TYPE:nucleic acid
TOPOLOGY:linear
CLONE:HUMGS02936
SEQUENCE DESCRIPTION:
GATCATCCCT TGACAGTAGT TTGCTTTCAT CTCACCTTTC ATTTGTCCCA AANNCACCTA 60
TATTTAATAA AGTCCCCCCC NGTNGTCTCA AA 92

SEQ ID NO:2562
SEQUENCE LENGTH:91
SEQUENCE TYPE:nucleic acid
TOPOLOGY:linear
CLONE:HUMGS02937
SEQUENCE DESCRIPTION:
GATCCACAAA ATGAGACAAT GCATGTGAAA ATCCATGCTC ATGTTCTAAA CATGGAAACT 60
AGGAGCCTAA TAAACTTCCT AATTCAGTAA A 91

SEQ ID NO:2563
SEQUENCE LENGTH:96
SEQUENCE TYPE:nucleic acid
TOPOLOGY:linear
CLONE:HUMGS02938
SEQUENCE DESCRIPTION:
GATCGTATTT TTATATCATA TTCACATATT TNTTTTTTTA ATTGGTGTTA GATGACATGA 60
TTAATAAAAA AGGCAAGATA TTTTCAGAAT TTGAAA 96

SEQ ID NO:2564
SEQUENCE LENGTH:88
SEQUENCE TYPE:nucleic acid
TOPOLOGY:linear
CLONE:HUMGS02940
SEQUENCE DESCRIPTION:
GATCGCGCCA TCGCACTCTC AGCCTGGGCA ACAAGAGTGN AACTCCATCT CAAAAAAATA 60
AAAATAAAAT AAGTAAGNAA TACCTAAA 88

SEQ ID NO:2565
SEQUENCE LENGTH:87
SEQUENCE TYPE:nucleic acid
TOPOLOGY:linear
CLONE:HUMGS02942
SEQUENCE DESCRIPTION:
GATCTAATAT ATATCATCAG AAAATATATA TTGTATGTTT ACTCTTATTT TCAGAAAGAG 60
AAAATGAATA AAAATGATGA CATCAAA 87

SEQ ID NO:2566
SEQUENCE LENGTH:87
SEQUENCE TYPE:nucleic acid
TOPOLOGY:linear
CLONE:HUMGS02944
SEQUENCE DESCRIPTION:
GATCGCTTGA NCCCAGGAGT TTAAAACCAG CCCGAGGAAC ATGGCGAAAC CCCATCTTTA 60
CAAAAAATAC AGAAATNAGC CAAGAAA 87

SEQ ID NO:2567
SEQUENCE LENGTH:86
SEQUENCE TYPE:nucleic acid
TOPOLOGY:linear
CLONE:HUMGS02946
SEQUENCE DESCRIPTION:
GATCTAAGGG GTTGCCATGG TGTTGAACAA TGCAACTTTT TATTTAAAAA AGCTCTGCAC 60
TGCCATGTAT GAAAGTNTCT TTATGN 86

SEQ ID NO:2568
SEQUENCE LENGTH:86
SEQUENCE TYPE:nucleic acid
TOPOLOGY:linear
CLONE:HUMGS02947
SEQUENCE DESCRIPTION:
GATCCNGGAT TGCTGGGAAT GGAAGCCAGG TGGGGTCATG GCACAAGTTC TGTAATCTTC 60
AAAATAAAAC TTTTTTTTTG TACAAA 86

SEQ ID NO:2569
SEQUENCE LENGTH:85
SEQUENCE TYPE:nucleic acid
TOPOLOGY:linear
CLONE:HUMGS02948
SEQUENCE DESCRIPTION:
GATCCTGGCC ACTATGCCAG TTCTGACCTC GCATCCCCCT ACCCCGAGCC CATGCAGTCT 60
GGGAACATGC CGCCTTCTCT CCAAA 85

SEQ ID NO:2570
SEQUENCE LENGTH:85
SEQUENCE TYPE:nucleic acid
TOPOLOGY:linear
CLONE:HUMGS02949
SEQUENCE DESCRIPTION:
GATCAAATTG TACTAGTGTC TGCAGGGTTT GTCAGTACTC GTCAAAGCCA AGTCCAATTA 60
AAAAAAAAAG TCTTTGCCCT CCAAA 85

SEQ ID NO:2571
SEQUENCE LENGTH:85
SEQUENCE TYPE:nucleic acid
TOPOLOGY:linear
CLONE:HUMGS02950
SEQUENCE DESCRIPTION:
GATCTTTTCT TTAAAATTTC TCTGCACACA CAGGACTTCT CATTTTCCAA TAAATGGGTG 60
TACTCTGCCC CAATTTCTAG GGAAA 85

SEQ ID NO:2572
SEQUENCE LENGTH:85
SEQUENCE TYPE:nucleic acid
TOPOLOGY:linear
CLONE:HUMGS02951
SEQUENCE DESCRIPTION:
GATCTTTTAC CCCTTTCACT CTTGGCTTTC TTATGTTGCT TTCATGAATG GAATNGAAAA 60
AAGATGACTC AGTTAAGGCA CCAAA 85

SEQ ID NO:2573
SEQUENCE LENGTH:84
SEQUENCE TYPE:nucleic acid
TOPOLOGY:linear
CLONE:HUMGS02953
SEQUENCE DESCRIPTION:
GATCTGCCTG ATGGGAGGGC TCCTGTCGAG GGAGACAGAC ATATCCCCAC TGAAAAATTA 60
ATAAAATTGG TTTTACATAT CAAA 84

SEQ ID NO:2574
SEQUENCE LENGTH:84
SEQUENCE TYPE:nucleic acid
TOPOLOGY:linear
CLONE:HUMGS02955
SEQUENCE DESCRIPTION:
GATCTGACTG GAAAACAATC CTGTATCCCC TCCCAAAGAA TCATGGGCTT TTTTTTTNAN 60
TTAAAAAGCA GACAAATAGA CAAA 84

SEQ ID NO:2575
SEQUENCE LENGTH:82
SEQUENCE TYPE:nucleic acid
TOPOLOGY:linear
CLONE:HUMGS02957
SEQUENCE DESCRIPTION:
GATCAAAAGA AACTTAAAAA GTGGAATGGG ATATGGCTTT TGGTACCTGA AAATAAATNC 60
TTTAATAATT GTAAGCCAGA AA 82

SEQ ID NO:2576
SEQUENCE LENGTH:82
SEQUENCE TYPE:nucleic acid
TOPOLOGY:linear
CLONE:HUMGS02958
SEQUENCE DESCRIPTION:
GATCAAAAAT ATAGTTATAA TTTTNTAAAT TTNAAAAATG TGATTGCNCT AATAAAGAAT 60
AAAANCTATG CTTTTAAACA AA 82

SEQ ID NO:2577
SEQUENCE LENGTH:85
SEQUENCE TYPE:nucleic acid
TOPOLOGY:linear
CLONE:HUMGS02959
SEQUENCE DESCRIPTION:
GATCACTGCA GNTTCTAGGA CCCGGTTTCT TTTACTGATT TAAAAACAAA ACAAAAAAAA 60
ATAAAAAAGT TGTGCCTGNN ACAAA 85

SEQ ID NO:2578
SEQUENCE LENGTH:81
SEQUENCE TYPE:nucleic acid
TOPOLOGY:linear
CLONE:HUMGS02960
SEQUENCE DESCRIPTION:
GATCTCCCAT AATTTGAAAT TGAAATCGTA TTGTGTGGCT CTGTATATTC TGTTAAAAAA 60
TNAAAGGACA GAAACCTTAA A 81

SEQ ID NO:2579
SEQUENCE LENGTH:79
SEQUENCE TYPE:nucleic acid
TOPOLOGY:linear
CLONE:HUMGS02962
SEQUENCE DESCRIPTION:
GATCCACCAT CTTATCTTGG TGCCATCCCT GACTTGGCTT CTGTTCATAA ATGCCTATTA 60
AATGTTTCTT TCTGAGAAA 79

SEQ ID NO:2580
SEQUENCE LENGTH:79
SEQUENCE TYPE:nucleic acid
TOPOLOGY:linear
CLONE:HUMGS02963
SEQUENCE DESCRIPTION:
GATCCAATTG CTGGTTACAT TTTGGGAAGC TCTCATTTTA GTGCTATGTA GTTTCATTGA 60
ATAAACTGTT TAATATAAA 79

SEQ ID NO:2581
SEQUENCE LENGTH:75
SEQUENCE TYPE:nucleic acid
TOPOLOGY:linear
CLONE:HUMGS02964
SEQUENCE DESCRIPTION:
GATCTAAGAG GAAAGTGGTT ATCGGTTGCN NTTTATTTAT AAAGGATGCA AACATAAAAT 60
TAGATTTTTG GCAAA 75

SEQ ID NO:2582
SEQUENCE LENGTH:73
SEQUENCE TYPE:nucleic acid
TOPOLOGY:linear
CLONE:HUMGS02965
SEQUENCE DESCRIPTION:
GATCAGCCTT TTTGTTTCAA TGAGGTGTCC AACTGGCCCT ATTTAGATGA TAAAGAGACA 60
GTGATATTGG AAA 73

SEQ ID NO:2583
SEQUENCE LENGTH:72
SEQUENCE TYPE:nucleic acid
TOPOLOGY:linear
CLONE:HUMGS02966
SEQUENCE DESCRIPTION:
GATCCCAGAG TGGCCTGCCT ATCACAACCA CATCCCTTCC CCCCACAAGG CAATAAATCT 60
CATTTCTTTA AA 72

SEQ ID NO:2584
SEQUENCE LENGTH:72
SEQUENCE TYPE:nucleic acid
TOPOLOGY:linear
CLONE:HUMGS02967
SEQUENCE DESCRIPTION:
GATCAACGAC AAAATGCACT TCTCCCTCAA GNAGTGAGGC TTGGTCCAAT ACATGGCTCT 60
GCCCCCCAGA AA 72

SEQ ID NO:2585
SEQUENCE LENGTH:72
SEQUENCE TYPE:nucleic acid
TOPOLOGY:linear
CLONE:HUMGS02968
SEQUENCE DESCRIPTION:
GATCAATCTT GTATTTTCTG ACCACATAAA GGCTTCTTCT CTTTGTAATA AAGTAGAAAA 60
GCTCTCCTCA AA 72

SEQ ID NO:2586
SEQUENCE LENGTH:166
SEQUENCE TYPE:nucleic acid
TOPOLOGY:linear
CLONE:HUMGS02969
SEQUENCE DESCRIPTION:
GATCTTGTTT CTGAAGATGG TTTAAGTTAT AGCTTCTTAA ACGAAAGAAT AAAATACTGC 60
AAAATGTTTT TATATTTGGC CCTTCCACCC ATTTTTAATT GTGAGAGATT TGTCACCAAT 120
CATCACTGGT TCCTCCTTAA AAATTAAAAA GTAACTTCTG TGTAAA 166

SEQ ID NO:2587
SEQUENCE LENGTH:71
SEQUENCE TYPE:nucleic acid
TOPOLOGY:linear
CLONE:HUMGS02970
SEQUENCE DESCRIPTION:
GATCATAGGA GAGACCCATT NGCTAGCTAC TTCATTCGTT CTGACCTAAT AATAAAAGTT 60

AGAACCATAA A 71

SEQ ID NO:2588
SEQUENCE LENGTH:72
SEQUENCE TYPE:nucleic acid
TOPOLOGY:linear
CLONE:HUMGS02971
SEQUENCE DESCRIPTION:
GATCTGACGT TCCTTGTGAC TTAAGGGTCC GGCTTGGGAA TTAAAGTTTG TTTCTGGCCT 60
TTAGCCTACA AA 72

SEQ ID NO:2589
SEQUENCE LENGTH:70
SEQUENCE TYPE:nucleic acid
TOPOLOGY:linear
CLONE:HUMGS02972
SEQUENCE DESCRIPTION:
GATCTGGCCA GTCAAGGGTC CACAGGGTGC CAGCCTCTCA CCCTCAGAAT AAAGACATCA 60
GCCATTCAAA 70

SEQ ID NO:2590
SEQUENCE LENGTH:69
SEQUENCE TYPE:nucleic acid
TOPOLOGY:linear
CLONE:HUMGS02973
SEQUENCE DESCRIPTION:
GATCATCTAA ACTGAGTCCA GCTGCCTAAT TCTGAATATA TATATATATA TATATCTTTT 60
CACCATAAA 69

SEQ ID NO:2591
SEQUENCE LENGTH:68
SEQUENCE TYPE:nucleic acid
TOPOLOGY:linear
CLONE:HUMGS02974
SEQUENCE DESCRIPTION:
GATCCTTATT ACAGGAAAAG CATGAGTGGT GGCTAACCTG ACCAATAAAG TTATTTTATG 60
ATTGCAAA 68

SEQ ID NO:2592
SEQUENCE LENGTH:68
SEQUENCE TYPE:nucleic acid
TOPOLOGY:linear
CLONE:HUMGS02975
SEQUENCE DESCRIPTION:
GATCTTAGAT TTTATGTAGT ATTAAGTGAA AAAATACGAA AATAATAAAC ATTGAAGAAA 60

AAAATAAA 68

SEQ ID NO:2593
SEQUENCE LENGTH:75
SEQUENCE TYPE:nucleic acid
TOPOLOGY:linear
CLONE:HUMGS02976
SEQUENCE DESCRIPTION:
GATCACATTT TCTGTTGGTT AACATTNTTC CTAGCTTATA TGATGGAATT AAATATATTC 60
TGTGTAAAAA GGAAA 75

SEQ ID NO:2594
SEQUENCE LENGTH:67
SEQUENCE TYPE:nucleic acid
TOPOLOGY:linear
CLONE:HUMGS02978
SEQUENCE DESCRIPTION:
GATCCACCTA ACAAGAAACC TAAAGTGTAG AGATTGCCAT TTTNATTTGT AATTTTTTTT 60
TTTTTTN 67

SEQ ID NO:2595
SEQUENCE LENGTH:66
SEQUENCE TYPE:nucleic acid
TOPOLOGY:linear
CLONE:HUMGS02980
SEQUENCE DESCRIPTION:
GATCACACAC CGGCTGTATT TAATATGTAA CATTTTCACA NATATTAAAG ANACAGAAGT 60
ATTAAA 66

SEQ ID NO:2596
SEQUENCE LENGTH:65
SEQUENCE TYPE:nucleic acid
TOPOLOGY:linear
CLONE:HUMGS02981
SEQUENCE DESCRIPTION:
GATCAGCATG GGCCACGTAG NCGNGACTCC ATCTCTACAC ATAAATAAAA AATTAGCTGG 60
GCAAA 65

SEQ ID NO:2597
SEQUENCE LENGTH:63
SEQUENCE TYPE:nucleic acid
TOPOLOGY:linear
CLONE:HUMGS02982
SEQUENCE DESCRIPTION:
GATCCAATTT ATGTTTTTTC TTTNTTTATA TTTTGGGGAA AATTAAAATT TTTTTAAGGT 60

AAA 63

SEQ ID NO:2598
SEQUENCE LENGTH:61
SEQUENCE TYPE:nucleic acid
TOPOLOGY:linear
CLONE:HUMGS02983
SEQUENCE DESCRIPTION:
GATCACGCAA TAATGACTAT GTAATCTCAA AAAACAAATA AAATATTCTT AACATGGCAA 60
A 61

SEQ ID NO:2599
SEQUENCE LENGTH:60
SEQUENCE TYPE:nucleic acid
TOPOLOGY:linear
CLONE:HUMGS02984
SEQUENCE DESCRIPTION:
GATCCAAGCA GTGTGTCACT ATATAACTGA TAAAAGCACT TGGTCTTNGA TTATCAGAAA 60

SEQ ID NO:2600
SEQUENCE LENGTH:58
SEQUENCE TYPE:nucleic acid
TOPOLOGY:linear
CLONE:HUMGS02985
SEQUENCE DESCRIPTION:
GATCATTTTG CACATAGCTT TATCAACTTT TAAACATTAA TAAACTGATT TTTTTAAA 58

SEQ ID NO:2601
SEQUENCE LENGTH:58
SEQUENCE TYPE:nucleic acid
TOPOLOGY:linear
CLONE:HUMGS02986
SEQUENCE DESCRIPTION:
GATCTTTGCA GTTTTGNGAC AATTACAAGT AAAGCTGCTA TAAACATTTG TATGCAAA 58

SEQ ID NO:2602
SEQUENCE LENGTH:74
SEQUENCE TYPE:nucleic acid
TOPOLOGY:linear
CLONE:HUMGS02987
SEQUENCE DESCRIPTION:
GATCGACACG CAGCTAGCCT CCTGCATTGT ATGGTTATAA ATAGCACCCT AGTGAGTNCT 60
GTCGTCCAGT CAAA 74

SEQ ID NO:2603

SEQUENCE LENGTH:58
SEQUENCE TYPE:nucleic acid
TOPOLOGY:linear
CLONE:HUMGS02988
SEQUENCE DESCRIPTION:
GATCCTGGGG CAACCCATCT GGTCTCTTGA ATAAAGGTCA AAGCTGGATT CTCGCAAA 58

SEQ ID NO:2604
SEQUENCE LENGTH:56
SEQUENCE TYPE:nucleic acid
TOPOLOGY:linear
CLONE:HUMGS02989
SEQUENCE DESCRIPTION:
GATCACGTTT CGTGTTCATA CTCAACGTTA ATAAAAGGAG AGAGTTTGTA GTGAAA 56

SEQ ID NO:2605
SEQUENCE LENGTH:55
SEQUENCE TYPE:nucleic acid
TOPOLOGY:linear
CLONE:HUMGS02990
SEQUENCE DESCRIPTION:
GATCGCTGTG CCTGGCATAT AGTAGGTGTT CAATAAATGC CCTGTGACTC TCAAA 55

SEQ ID NO:2606
SEQUENCE LENGTH:59
SEQUENCE TYPE:nucleic acid
TOPOLOGY:linear
CLONE:HUMGS02991
SEQUENCE DESCRIPTION:
GATCTATCCT CAACAACAAC AGAAAAAAGG AATAAAATAT CCTTTGTTTC CTAGTGAAA 59

SEQ ID NO:2607
SEQUENCE LENGTH:54
SEQUENCE TYPE:nucleic acid
TOPOLOGY:linear
CLONE:HUMGS02992
SEQUENCE DESCRIPTION:
GATCCATTTT AAAATTTGTA ATTCAATAAA GTTTTTTTTG TTGTTAAACA TAAA 54

SEQ ID NO:2608
SEQUENCE LENGTH:53
SEQUENCE TYPE:nucleic acid
TOPOLOGY:linear
CLONE:HUMGS02993
SEQUENCE DESCRIPTION:

GATCTTGTAT TCAGTCAGGT TAAAACAACG GACAATAAAA GAATGAACAC AAA 53

SEQ ID NO:2609
SEQUENCE LENGTH:52
SEQUENCE TYPE:nucleic acid
TOPOLOGY:linear
CLONE:HUMGS02994
SEQUENCE DESCRIPTION:
GATCTGGGGT GGGAGTAACA GGGCAGAAAT GATTAAAATG TTTGAGCACA AA 52

SEQ ID NO:2610
SEQUENCE LENGTH:52
SEQUENCE TYPE:nucleic acid
TOPOLOGY:linear
CLONE:HUMGS02995
SEQUENCE DESCRIPTION:
GATCCTTTTT TTTTTCCACA AAATTTTTAC AAATAAAAAA TGTTCCCCTA AA 52

SEQ ID NO:2611
SEQUENCE LENGTH:52
SEQUENCE TYPE:nucleic acid
TOPOLOGY:linear
CLONE:HUMGS02996
SEQUENCE DESCRIPTION:
GATCTCAAAG TGCCTTCTGA AGCATCAGAG ATTAAATATT GTTCAAACTA AA 52

SEQ ID NO:2612
SEQUENCE LENGTH:53
SEQUENCE TYPE:nucleic acid
TOPOLOGY:linear
CLONE:HUMGS02997
SEQUENCE DESCRIPTION:
GATCTGAATG GGGCTTNCCT GATGTTGGTA TCTAAGGCTT AGGCCTATAG ATN 53

SEQ ID NO:2613
SEQUENCE LENGTH:50
SEQUENCE TYPE:nucleic acid
TOPOLOGY:linear
CLONE:HUMGS02999
SEQUENCE DESCRIPTION:
GATCTTGAAT GTATTTTCTT AAGTTTATTA ATAGACATTT TCACAATAAA 50

SEQ ID NO:2614
SEQUENCE LENGTH:50
SEQUENCE TYPE:nucleic acid

TOPOLOGY:linear
CLONE:HUMGS03002
SEQUENCE DESCRIPTION:
GATCTATCTG GAAAATGNAG GACTCCGAAT AAAAAGCTAT TACTAATAAA 50

SEQ ID NO:2615
SEQUENCE LENGTH:51
SEQUENCE TYPE:nucleic acid
TOPOLOGY:linear
CLONE:HUMGS03005
SEQUENCE DESCRIPTION:
GATCAGGGGA AATNTTTAAG CTAAATAAAT CTGGGGGGTT TTTTATTCAA A 51

SEQ ID NO:2616
SEQUENCE LENGTH:165
SEQUENCE TYPE:nucleic acid
TOPOLOGY:linear
CLONE:HUMGS03028
SEQUENCE DESCRIPTION:
GATCGAATGG TATATATTAG AAATTACATC TGTTGTAATT AAAATTGTGT GAGCAATTAA 60
ACATGGTTGA NTTTTTCAAG CAAAAATCAG TTCATCTTTT GATGTAATTT TCTAGGCTAA 120
ATGGCAATCT CTGAAAGATG AATAAAGCTA TATTTATTTA GCAAA 165

SEQ ID NO:2617
SEQUENCE LENGTH:254
SEQUENCE TYPE:nucleic acid
TOPOLOGY:linear
CLONE:HUMGS03030
SEQUENCE DESCRIPTION:
GATCACATTT TAAGCATCTN TCTCTCCTAC TCCATTTAGA AAAATAAGTA ACAGGTGAAA 60
TGTGGTCTCA GTGTTAACGG GATAATTCTG CTACCGGCTC CTCCCTGATG ATTCTNAAAT 120
ACACTACTGA ACGAGCTCTG GCTGGTCCTT TCTATCCTGG ATGTGGTTCT TCTGTGTAGC 180
AATTCCTTGA NGTCCAGGTT GGAAAGATGT ACTCTTCTCA ACCAAGGAAA ACCTTAAATC 240
CCGTCGTGGC CAAA 254

SEQ ID NO:2618
SEQUENCE LENGTH:271
SEQUENCE TYPE:nucleic acid
TOPOLOGY:linear
CLONE:HUMGS03031
SEQUENCE DESCRIPTION:
GATCAACCCC AGACTGGGCC AGAATGTTAG TGAAGGTTTT ATTGTGCCCG GTTGGAGGAT 60
AACGTTCTTT GGGTACTTTT TGTGGGTTGC AAATNAACTC AATTGCCACA AGTTTTAAAC 120
TGGTGTAAAT CAAGCTTGAC TTAATGTGAT TGTNACTGTT ATATCCAGNC TATACTGCTA 180
GCAGCTGCTC ATACTGCAGT CAATTACTGG AAGCGGATAT ATTTCCTATG CAAAAACTGT 240

TTAAACAATA AAATGAGCTA TGCTACAGAA A 271

SEQ ID NO:2619
SEQUENCE LENGTH:489
SEQUENCE TYPE:nucleic acid
TOPOLOGY:linear
CLONE:HUMGS03053
SEQUENCE DESCRIPTION:
GATCCACAGC TTAAAGATGG GAATTCAGGT ATGAAAGAAA ACAGGCAAGG AGGCACTGAG 60
GGAGAAAGAC ACAGACTTTA TCGCTCTGTG GCTCATTGTT ACTGGAATAT NCTAAAACTC 120
TTGTNCACAT GCTATTATGA CTTATAAAGC AGCAACAGCT GAGGCGCACC AGGACACAGC 180
TTCCATTTCT TTAACGTCTG TNCCCTTAAC ATCGCTGAAA TNATTTACTG TTGAAGAGAT 240
GCCTTGCGGT GTGGCCAGCT GTGAGGAGAA AGCAGCTGGC AGTGTTAGGA CATTAGTCCA 300
CCTTCAGCGC AGGGTCTCTG GCCGGGTCTG ACTCAGAAAC CTTGGTACTC GNCCCTTGGN 360
CACAGTGCCC AGACCCATGT AACCNACTNG NTCCTGCATT AACCNAGAAA TACCTCGGTT 420
CTATNTGTGC ACTTAGCTGG GGACTTACCC ACTGTAATCA CCTNAATNAA NGGGTTNTAA 480
ACATGGAAA 489

SEQ ID NO:2620
SEQUENCE LENGTH:471
SEQUENCE TYPE:nucleic acid
TOPOLOGY:linear
CLONE:HUMGS03055
SEQUENCE DESCRIPTION:
GATCCAAAAC GCTTCGAGGC ACCCCAAATN ACCTGCCCAT TCGTNAGGAN GACCCACCCA 60
CCCAGTGTTA TATTCTGCCT CGCCGGAGTG GGTGTNCCCG GGGGCACTTG CCGACCAGCC 120
CCTTGCGTCC CCAGGTTTGC AGCTCTCCCC TGGGCCACTA ACCATCCTGG CCCGGGCTGC 180
CTGTNTGACC TCCGTGCCTA GTCGTGGCTC TCCATCTTGT CTCCTCCCCG TGTCCCCAAT 240
GTNTTCAGTG GGGGGCCCCC TCTTGGGTCC CCTCCTCTGC CATCACCTGA AGACCNCCAC 300
GGCAAANACT GAATGTAANC TGTGNCTGNN GNCTCGGTCC AACTTGCGGN CCGTGTTTNA 360
CTNAACTNAG CTCCTTTAAC GGTAATATTT CNGGGAAAAT CCCATGNTTG GGTTTTGGTC 420
TTTAACCTTG GTAACGGTTG AAATCCAATT AAAGNTTTAA AAGTCATGAA A 471

SEQ ID NO:2621
SEQUENCE LENGTH:435
SEQUENCE TYPE:nucleic acid
TOPOLOGY:linear
CLONE:HUMGS03056
SEQUENCE DESCRIPTION:
GATCACCATT AAATCTGAAA GTACCTTTAA AAATACTGAG ATTNCCTTCA TACTGGGCCA 60
GGNATTTNAC GAAGNACCTG CAGATGACAG GAAAGTCAAG AGCACCATAA CCTTAGATGG 120
GGGTGTCCTG GTACATGTNC AGAAATGGGA TGGAAAATCA ACCACCATAA AGAGAAAACG 180
AGAGGNTGAT AAACTGGTGG TGGAATGCNT CATGAAAGGC GTCACTTCCA CGAGAGTTTA 240
TGAGAGAGCA TAAGCCAAGG GACGTTGACC TGGACTGAAG TTCGCATTGA ACTCTACAAC 300
ATTNTGTGGG NTATATTGTT CAAAAAGNTA TTGTNGTTTT CCATGGATTT AGCAAGCAAC 360

TAATTTTNTC CCAAGCTNGN TTTTTTTCAA TANTGGTNAC GTTGGNTAAA TAAANNTTTT 420
TTTAGGNTTT TNAAA 435

SEQ ID NO:2622
SEQUENCE LENGTH:411
SEQUENCE TYPE:nucleic acid
TOPOLOGY:linear
CLONE:HUMGS03058
SEQUENCE DESCRIPTION:
GATCTCATCA GGGGAAGGCT CTGTGATGCT GCAGGTTGAT GTAGACACAG TAAAAGGAGG 60
GCTGAAGTTG AACCCCAACT NNCTGGTGGA CTTCGGGAAG GAGCCCCTTG GCCCAGCCCT 120
TGCCCATGAG CTCCGCTACC CTGGGGGCGA TTGTAGCTCT GACATCTGGA TTTGAACTCC 180
ACCCTCATCA CCCACACTCC CTATTTTGGG CCCTCACTTC CTTGGGGACC TGGCTTCATT 240
CTGCTCTCTC TTGGCACCCG ACCCTTGGCA GCATGTACCA CACAGCCAAG CTGAGACTGT 300
GGCAATGTGT TGAGTCATAT ACATTTACTG ACCACTGTTG CTTGTTGCTC ACTGTGCTGC 360
TTTTCCATGA GCTCNTTGAG GCACCAAGAA ATAAACTNGT AACCCTGTAA A 411

SEQ ID NO:2623
SEQUENCE LENGTH:410
SEQUENCE TYPE:nucleic acid
TOPOLOGY:linear
CLONE:HUMGS03059
SEQUENCE DESCRIPTION:
GATCCAGGCG GGATGGAGTG TGGAGTGTGG GCACAGTGGC TTGTACAGGC CTGGNCTGGG 60
CAGACTTGCC CCGGAGTCTC AGGCCACTGC GGAAGGAGGA CGCGTGGCCA GCAGGTGCCA 120
CCAGGAGAGG AGAGGAGGGC CAGGCTGAGC TGCATCTNTG GACTCCAAGG AGAGCTTGGA 180
GAGGTGACTG AAACCCTGCC CCACCCCCAG CCTGTNTCCA TCTGTGGGTC CTTGGGCAAA 240
TTGCCTCATC TCTCTNAGCC TCGAATGCGT AGTTTCAGGA CTAACTAGGG TACTGTGTAC 300
CAACTGCCTC TGCCAACGGC CCTGCACTGG GCANGCCTGG GGTACCTGGG CTTTGGGGNA 360
AGGAAGTAGG CCTTNGGNAG GAGGCTGGAT TGCTTACAAT TTTNTTTTTN 410

SEQ ID NO:2624
SEQUENCE LENGTH:408
SEQUENCE TYPE:nucleic acid
TOPOLOGY:linear
CLONE:HUMGS03060
SEQUENCE DESCRIPTION:
GATCTGCGGC CCATCTACAT CTCTGTGCAG CCGCCTTCAC TGCACGTGCN GGAGCAGCGG 60
CTGCGGCAGG CATACACTGA AACCGAGGAG AGCCTGGTGA AGCGGCTNGC TGNTGCCAGG 120
CCGACATGGA GAGCAGCAAG GAGCCCGGCC TGTTTGATGT GGTCATCATT AACGACAGCC 180
TGGACCAGGC CTACGCAGAG CTGAAGGAGG CGCTCTCTGA GGAAATCAAG AAAGCTCAAA 240
GGACCGGCGC CTGAGGCTTG CTGTCTGTTC TCGGCACCCC GGGCCCATAC AGGACCAGGG 300
CAGCAGCATT GAGCCACCCC CTTGGGAAGG CGATACGGCA GCTCTGTGCC CTTGGCCAGC 360
ATGTTGGAGT TGGAGGAGAT NCTTGCCCCT GTTGGTTTGG ACCATCCN 408

SEQ ID NO:2625
SEQUENCE LENGTH:398
SEQUENCE TYPE:nucleic acid
TOPOLOGY:linear
CLONE:HUMGS03061
SEQUENCE DESCRIPTION:
GATCTCAACG AGACAAACGA TGGGTGGACC CGAACTCCCC GGTGCTCTTN GAGGACCCAG 60
TCCTTTNTGC CTTGGCAAAA AAGCACAAGC GAACCCCAGC CCTGATTGCC CTGCGCTACC 120
AGCTGCAGCG TGGGGTTGTG GTCCTGGCCA AGAGCTACAA TNAGCAGCGC ATCAGACAGA 180
ACGTGCAGGT TTTTNANTTC CAGTTGACTG CAGAGGACAT GAAAGCCATA GATGGCCTAG 240
ACAGAAATCT CCACTATTTT AACAGTGATA GTTTTGCTAG CCACCCTAAT TATCCATATT 300
CAGATGANTA TTANCATGGA GGGCTTTGCC TGATGGTNTA CCAGAAGCCC TGTGTGTGGN 360
TGGGTGACGC AGAGGGACGT CTCTNTGCCG GTGACTGN 398

SEQ ID NO:2626
SEQUENCE LENGTH:394
SEQUENCE TYPE:nucleic acid
TOPOLOGY:linear
CLONE:HUMGS03062
SEQUENCE DESCRIPTION:
GATCACAGCT GGGGGAAAAA AAGCTTTTTA ATTCTGTACC TTCCTAGTAG ATAAGTGAAG 60
AGCAGGGAAA GAGACCTTTA AATATTTTNC TATAAAAAAA TTTGTGATAA GTTTCTATCA 120
AAATGGGGAG ATTGCAGAAA AGGCTTCCCT TGGNTCCCAA GGAGGTGTAG CAGGTGTGAG 180
CAATATTAGT GCCATGTGCC TTTCACACAG GGTTTGCATT TATCAGTCTG TTTTCCGATG 240
NNNNGGTACA TGAAAGAGTA CACCATGTGA AGAGAAGAGA GAATGATTGA AAATGTTTTA 300
GTATAGACCT CTTCTTGCAG TGGGTTGCTA TTTTCTAGAT TTTACTTTTT AGGGACCAAA 360
NTAAANTCCT TTGTTAAACN TGGGTCAGNG NAAA 394

SEQ ID NO:2627
SEQUENCE LENGTH:392
SEQUENCE TYPE:nucleic acid
TOPOLOGY:linear
CLONE:HUMGS03063
SEQUENCE DESCRIPTION:
GATCGCACCA TTACACTCCA GCCTGGGTGA CAGAGAGAGA CCCTGTGCCN NTCAAAAAAA 60
TGTTTTTTTT AAACAAGAAT CTCTCAGGCA TACTGAAATN TTGAAGAGGA AATNATATTA 120
ATATCTGGGA CTTGTTTCCA AATTTGGAAT TGGGGCAGGG TGTAAATNGT TGGCAATATC 180
AAAGAAACGA GGAGTTGGAA TNATAATTGT AGAAGCTGCA TGATAGATAC ACATTCTTTA 240
TACTTCTGTA TATGTNGAAA TTTTTNATCA AATTATAATN GTGTATAATG ACTAATTATG 300
CTTNCATTGT CTATCCTGTG TGGTTATGTN ACTTGCTGAA ATNNATACAT AANGGGTCTT 360
CAACCTTNAN NTGGTCTTAT CCANGGCATA NN 392

SEQ ID NO:2628
SEQUENCE LENGTH:385
SEQUENCE TYPE:nucleic acid

TOPOLOGY:linear
CLONE:HUMGS03064
SEQUENCE DESCRIPTION:
GATCTGTAAT NATCTCTATT GGCCTGGGGT GCCTGTGCTA TAAATAAGTT TCTTCACATG 60
AAAAACACAG CCAGCCCAAG ATNACTTATC TGGGTTTAGG ATTCAATAGT ATTCACTAAC 120
TGCTTATNAC ATGAGCAATT TCATCAAATC TCCAAACTCT TAAAGNATGC TTTCGGAAAA 180
CACGCTGTAT ACCTAGATGA TGACTAAATG CAAAATCCTT GGGCTTTGGT TTTTTTCTAG 240
TAAGGATTTT AAATAACTGC CGACTTCAAA AGTGTTCTTA AAACGAAAGA TAATGTTAAG 300
AAAAATTTGA AAGCTTTGGA AAACCAAATT TGTAATATCA TTGTATTTTT NATTAAAAGT 360
TTTGTAATAA ATTTCTAAAT TTAAA 385

SEQ ID NO:2629
SEQUENCE LENGTH:385
SEQUENCE TYPE:nucleic acid
TOPOLOGY:linear
CLONE:HUMGS03065
SEQUENCE DESCRIPTION:
GATCGGGGCC CTGGGGCTGT NAGACCCCGA CCNTCTCGAG GAACCCTGCC TGAAACGCCT 60
CCATTACCAC TGCGCAGTGA GATGAGGGGA CTCACAGTTG CCAAGAGGGG TCTTTGCCGT 120
GGGNNGGNTC GCCAGCCACT CACCAGCTGC ACTGAGAGGG GAGGTTNGCA CACCCCTCCC 180
CTGGGCCGCT GAGGCCCGCG CACCTGTGCC TTAATCTTCC CTCCCCTGTG CTGCCCGAGC 240
ACCTNCCCCG CCCCTTTACT CCTGGGAACT TTGCAGCTGC CCTTCCCTNC CCGTTTTTAA 300
TGGCCTGCTG AAATATGTGT GTGAAGAATT ATTTATTTTC GNCAAAGCAC ATGTAATAAA 360
TGCTGCAGNC CNGCCTNTGC CCAAA 385

SEQ ID NO:2630
SEQUENCE LENGTH:385
SEQUENCE TYPE:nucleic acid
TOPOLOGY:linear
CLONE:HUMGS03066
SEQUENCE DESCRIPTION:
GATCCCGGGN AGTNACAGGT GCCGACGTGA TGTCGCTTCT NTGGTGCCCA GCTCCCTTCC 60
TGGTCTNANA CTAGCTCTGG GGGTGGCGGG GGCCCCCACA CGCTGCTCCC GCTCCACCCT 120
GCCCGTNCTG CTGCTCTGTG CCTGCTGTCA GAGCCCTGGT GGGGGAGGAT GTGGCCACCC 180
TGAGACCCGG AGGAGACGGG CGTCTGCCTG GNTTTGCGGA GAGCNGCTTA TGGGTGTGGT 240
CCGTCCAGAC ACCTTGTTTC AAGGGGGATG GGCGTNANGG GCAAGCAGAG CATCCCCACC 300
GNTGAGCAAG AACTTTTTCT TGTTTTTAAA CCATCACGTC CTCATTTCAC AATTGGAATA 360
AAGTGAGTTT TTTGGAAACC TGAAA 385

SEQ ID NO:2631
SEQUENCE LENGTH:381
SEQUENCE TYPE:nucleic acid
TOPOLOGY:linear
CLONE:HUMGS03067
SEQUENCE DESCRIPTION:

GATCGCAAGG AACTGGGCCG CCTACTACCC CGACCTGCCC ACCCCAGAAC AGCAGCAAGA 60
CATGGCCCAG TTCATACATT TATTTTCTAA GTTTTACCCC TGTAAGGAGT GTGCTGAAGA 120
CCTAAGAAAA AGGTTGTGCA GGAACCACCC AGACACCCGC ACCCGGGCAT GCTTCACACA 180
GTGGCTGTGC CCCNTGCACA ATGAAGTGAA CCGCAAGCTG GGCAAGCCTG ACTTCGACTG 240
CTCAAAAGTG GATGAGCGCT GGCGCGACGG CTGGAAGGAT GGCTCCTGTG ACTAGAGGGT 300
GGTCAGCCAG AGCTCATGGG ACAGCTAGCC AGGCATGGTT GGATAGGGGC AGGGCACTCA 360
TTAAAGTGCA TCACAGCCAA A 381

SEQ ID NO:2632
SEQUENCE LENGTH:370
SEQUENCE TYPE:nucleic acid
TOPOLOGY:linear
CLONE:HUMGS03068
SEQUENCE DESCRIPTION:
GATCCGTAAG TCCATGACCA AGGAGGCCAT CCGAGAGCAC CANATGGCCC GCACTGGCGG 60
CACGAGACAG ACCTGTNCAC CTGCGGCAAG TGCAGGAAAA AGAACTGCAC CTACACACAG 120
GTGCAGACCC GCAGCTCTGA TGAGCCCATG ACCACCTTTN TTGTCTGCAA CGAGTGTGGA 180
AACCGCTGGA AGTTCTGCTG ACCCCTCGTG TAGATGTGCT GCAGCCTTGG GCCCTCCCCG 240
GCCCACGTCC TCCGTTGACA CAGCTTCTNT GGAGACCCTA GAAGGCGGCA TGTCCTGCCC 300
TCAACCTGCC TGCNTGGATT GCACCTTTCT GCCCTTTCCC CNTNATTATT AAATGTTTCT 360
TTTTGCCAAA 370

SEQ ID NO:2633
SEQUENCE LENGTH:344
SEQUENCE TYPE:nucleic acid
TOPOLOGY:linear
CLONE:HUMGS03069
SEQUENCE DESCRIPTION:
GATCTTCTTC AAGCCATTAT TTTAACTCAA GAAAACTCTA GAGAAGAAAA GTGAAGAAGT 60
CATGTTGAAG AAGATGTAAG AATGTGTCAA GACCATCCAG AAATNATATG AGAAATACTG 120
ATATTTTAAA TGGTTGACAT CATCCAGCGA AATGAATCTA CATTAAATGT TGTTTTAACT 180
GCGCTATGAT TAAAACCATT CATATAGAGT TAGTNTTTAC AACTACTATT CTGTTATTTT 240
TTTTTTAATC TGACAACATT TGTCCTAAGT AAGATAAGCA AAAAAATTCT TCAACTCCTT 300
TTGGCAAGAA AACTGTAACA GAAAATAANT TTTGAATGTG TACN 344

SEQ ID NO:2634
SEQUENCE LENGTH:341
SEQUENCE TYPE:nucleic acid
TOPOLOGY:linear
CLONE:HUMGS03070
SEQUENCE DESCRIPTION:
GATCCCTGGT CCAGAATTCC ATTTACCTTC TGGGCCAGAT ACCACCAGAA TGCCCGCTCC 60
ANATTCCCTC AGACCTTTGC CGGTCCCATT ATTGGTCCTG GTGGTACAGC CAGTGCCAAC 120
TTCGCTGCCA ACTTTGGTGC CATTGGTTTC TTCTGGGTTG AGTGAGATGT TGGATATTGC 180
TATCAATCGC AGTAGTCTTT CCCCTGTGTG AGGCTGAAGC CTCAGATTCC TTCTAAACAC 240

AGCTATCTAG AGAGCCACAT CCTGTTGACT GAAAGTGGCA TGCAAGATAA ATTTATTTGC 300
TGTTCCTTGG GCTACTGNTT TTTTCCCCT TGTGTGCTGT N 341

SEQ ID NO:2635
SEQUENCE LENGTH:340
SEQUENCE TYPE:nucleic acid
TOPOLOGY:linear
CLONE:HUMGS03071
SEQUENCE DESCRIPTION:
GATCGNCAAG AAAATTNAGA TTGGCGATGG GGCAGAGCTG ACGGCAGAGT TCCTCTACGA 60
CGAGGNGCAC CCCAAGCAAC ACGNCTTCAA GCAGGCCTTC GCCAAGCCCA AGGGCCCAGG 120
GGGCAAGCGG GGCCATAAGC GCCTCATCCG NGGCCCGGGT GAAAATGGGG ATGACAGCTA 180
GGAGGCTGGA GCATNNGNCG GCCACGTNTG GACTGTGGGG CTGCCCACCT TCCGCTCCCT 240
GCCACCATCC TCCTTCCTGG GCTCCAGGAA AGTNTTTCTG GGAGGTCAGG AGGGCTGGCA 300
GCTTGAACGC ACTTGCAGCG TCCGANGGNC ACCGGGCTTN 340

SEQ ID NO:2636
SEQUENCE LENGTH:335
SEQUENCE TYPE:nucleic acid
TOPOLOGY:linear
CLONE:HUMGS03072
SEQUENCE DESCRIPTION:
GATCTGTCTG CNTTCCTCAA GACCATTGCA CTGAATGGTG TGGAGGACGT GCGAACCGTG 60
CTGGAGCACT ATGCCCTGGA GGATGACCCG CTGGCGGCTT TCAAACAGCG GCAAAGCCGG 120
CTAGAGCAGG AGGAGCAGCA GCGCCTGGCC GAGCTCTCCA AGTCCAACAA GCAGAACCTC 180
TTCCTTGGCT CCCTCACCAG CCGCTTGTGG CCTCGCTCCA AACAGCCCTG AACTCTGGGC 240
CTCCTCAAAC TCAGTGCCTG GGTCCAGGGC CCCAGTGCTT CCAGACCAAG ACTTGGGCCA 300
CCACTTNTCC AATAAAGTAC ATCCGAGACG GCAAA 335

SEQ ID NO:2637
SEQUENCE LENGTH:332
SEQUENCE TYPE:nucleic acid
TOPOLOGY:linear
CLONE:HUMGS03073
SEQUENCE DESCRIPTION:
GATCCAGTTG TGAACAAAGA GTCCTCTTNG TGGAAAAGAA AAAAATACAT NTCCCTTTAA 60
ACGGTGGATT GAAAATAACT TTNATTTATA AAGAGAAGAC TGAGGGCGGG GATACTGATT 120
CANNAATCCT GTAGCGTGTA ATAAAAGAAG AGGAAATGGC ATGGAATCAC TGCCTCCTGT 180
GATTTGAAGG CCATTGTGAA GGAAAACAAT GCAGTGAAAG AAAGTTCTTC ATATTAGGCC 240
AGATATCATT GCATCACATT TATTTATCTT TCTGGNATTT TTATAGCCCT TAATAAAAAA 300
TATTAAAATA GCCTGTGCTA TTGTGTCTTA AA 332

SEQ ID NO:2638
SEQUENCE LENGTH:335
SEQUENCE TYPE:nucleic acid

TOPOLOGY:linear
CLONE:HUMGS03074
SEQUENCE DESCRIPTION:
GATCAAGAAT TTGGTGTGGA CGTTGGCCCT GTTTGCTTTT TATAAACCAA ACTCTATCTG 60
AAATCCCAAC AAAAAAAATT TAACTCCATA TGTGTTCCTC TTGTTCTAAT CTTGTCAACC 120
AGTGCAAGTG ACCGACAAAA TTCCAGTTAT TTATTTCCAA AATGTTTGGA AACAGTATAA 180
TTTGACAAAG AAAAATGATA CTTCTCTTTT TTNGCTGTTC CACCAANTAC AATTCAAATG 240
CTTTTNGTTT TATTTTTTTT ACCAATTCCA ATTTCAAAAT GTCTCAATGG GGCTATAATA 300
AATAAACTTT CAACACTCTT TTATGGTNAA CCAAA 335

SEQ ID NO:2639
SEQUENCE LENGTH:384
SEQUENCE TYPE:nucleic acid
TOPOLOGY:linear
CLONE:HUMGS03075
SEQUENCE DESCRIPTION:
GATCAAGCCT GCCTCAATCA GTATTCATAT TTATAGCCAG GTACCTTCTC ACCTGTNAGA 60
CCAAATTNAG CTAGGGGGGT CAGCCAGCCC TCTTCTGACA CTAAAACACC TCAGCTGCCT 120
CCCCAGCTCT ATCCCAACCT CTCCCAACTA TAAAACTAGG TGCTGCAGCC CCTGGGACCA 180
GGCACCCCCA GAATGACCTG GCCGCAGTGA GGCGGATTGA GAAGGAGCTC CCAGGNGGGG 240
CTTCTGGGAA GACTCTGGTC AAGAAGCATC GTGTCTGGCG TTGTGGGGAT GAACTTTTTG 300
TTTTGTTTCT TCCTTTTTTA GTTCTTCAAA GATAGGGAGG GNAGGGGGGA ACCATGAGCC 360
TTTGTTGGCT AATCANTCCC AAGN 384

SEQ ID NO:2640
SEQUENCE LENGTH:352
SEQUENCE TYPE:nucleic acid
TOPOLOGY:linear
CLONE:HUMGS03077
SEQUENCE DESCRIPTION:
GATCAAAGGT TTAAAGTCTA ACTTCTAAGA TATATTTGCA GAAAGAAGCA ACATGACAAT 60
AGAGAGAGTT ATGCTACAAT NATTTCTTGG TTTCCACTTG CAATGGTTAA TTAAGTCCAA 120
AAACAGCTGT CAGAACCTCG AGAGCAGANC ATGAGAAACT CAGAGCTCTG GACCGAAAGC 180
AGAAAGTTTG CCAGGNAAAA AANNGGCATC ATTATTACCA TCGATTCAGT GCCTGGTTAA 240
NGNGGNAAGC TTACTTNGTT TNAGTGGCAG CCNCNTNGCA CGTGGTTTGC TATGNAGGTG 300
AANGGTTTCC CAATTCCTTC AGGCTTTTTN GGGGGTTTTC GGGCTCTNCC TN 352

SEQ ID NO:2641
SEQUENCE LENGTH:321
SEQUENCE TYPE:nucleic acid
TOPOLOGY:linear
CLONE:HUMGS03078
SEQUENCE DESCRIPTION:
GATCAAACAG CTCTAAGTAT GAAGCAAGAG TAAAGACTAA GGTTTCGAGA NCATTCCTAC 60
TCACATAAGT GAAGAAATCT GTCAGATAGG AATCTAAATA TTTATAGTGA GATTGTGAAA 120

GCAACCTTAA AGTTTTGAAG AAGACTGATG AGACTAGGTG CNNNGCTTCC TTTCATCAGG 180
TATCTTTCTG TGGCATTTGA GAACAGAAAC CAAGAAACAT GGTAATTNCT AANTTATGAG 240
GCTTTGCTTT TTGTTTGCTT TTAAGTAGAA AACCATGTTG GCAACATTGA GTTTTGGNGT 300
TGATTGAGAT AATATGNCTT N 321

SEQ ID NO:2642
SEQUENCE LENGTH:320
SEQUENCE TYPE:nucleic acid
TOPOLOGY:linear
CLONE:HUMGS03079
SEQUENCE DESCRIPTION:
GATCTTTGGC TGGAAAACTA GAATTTATAG CAGTTTATTA ATGATACCTT AAATNACTCA 60
GGACTTAATG TAGCATTGCA CTTCTGTGTA CAGTAAAACT GCTTTGTTTT ACTAAAGAGA 120
AAAATGTGAG TGGAAAAAAT ATGTATGTGT TATATACTCA AATGTATATA ATNCTATCTA 180
TAGATTTATA TATGTATACA TTCTGTACAG TAGTTCCATC AAAATATGTA ATAATTCACA 240
CCAATTTTAT TAAATGTATT TNCTTTTTCA AAATTTAAAT TGAGCTGCTA TCAATATTAA 300
ATGAAGTTAT GGCATCTAAA 320

SEQ ID NO:2643
SEQUENCE LENGTH:303
SEQUENCE TYPE:nucleic acid
TOPOLOGY:linear
CLONE:HUMGS03080
SEQUENCE DESCRIPTION:
GATCCCTTCG ATGTGGATTC AGGGAGAGAG TTTGGAAACC CCAACAGGCC TGTGGCATGC 60
ACCCGGCTGC CCTCGGACAC TGATGACAGT GATGCGTCTG AGGACCCAGG GCCTGGCGCC 120
GAGCGCGGAG GAGCCAGCAG CAGCTGCTGT GAAGAGGAGC AGACGCAGGG ACGGGGGGCT 180
GAGGCCAGGG CCCCGGCTGA GGTTTGGAAA GGAATCAAGA AACGGCAGAG AGACTNAGGG 240
TTGCAGACAC ATATATTTTT GAGGCTGGGT GACGAGAAAA TCTAGAGACA TGAGGGACAT 300
AAA 303

SEQ ID NO:2644
SEQUENCE LENGTH:315
SEQUENCE TYPE:nucleic acid
TOPOLOGY:linear
CLONE:HUMGS03081
SEQUENCE DESCRIPTION:
GATCCAGACG CAAGANGGCT GTCCTCTCTA AGGAATATGG TTTTTTNCTT CTAACTGGTG 60
CTGCCAGCTT TATAATGGTG GCCCACCTAG CCATCANTGT TTCCAAGGCC CGCAAGANGT 120
ACAAAGTGGA GTATCCTATC ATGTACAGCA CGGACCCTGA AAATGGGCAC ATCTTCAACT 180
GCATTCAGCG AGCCCACCAG AACACGTTGG AAGTGTATCC TCCCTTCTNA TTTTTNCTAG 240
CTGTTGGAGG TGTTTACCAC CCGCNTATAG CTTCTGGCCT GGGCTTGGCC TGGATTGTTT 300
GGACGAGTTC TTTAN 315

SEQ ID NO:2645

SEQUENCE LENGTH:314
SEQUENCE TYPE:nucleic acid
TOPOLOGY:linear
CLONE:HUMGS03082
SEQUENCE DESCRIPTION:
GATCCTTCTT GGCCCCAGAA AGTCATTTGG CATGGCTCCA GCCCCCATGG CATCCGCTTN 60
TGGATAACTA CTGTGAAGCA TGGCGAACCG CGGACACAGC GGTCACGGGA CTTGCCTCCC 120
CGCTGAGCAC GGGGAAGATT CTGGACCAGA AAGCATACAG CTGTGCTAAT CGGGCTAATT 180
GTCCTATGTA TCGAAAACAG TTTCATGNCA GNCGTTAGGA AGTAATTGGC TTCTTNATGA 240
TTCTTAAAAN GNGTTTTCAA TTTTTCCTAA TGNGAAGNGT TGNCACTGAA ATCTAAAATG 300
TTTANTTGTG NAAA 314

SEQ ID NO:2646
SEQUENCE LENGTH:310
SEQUENCE TYPE:nucleic acid
TOPOLOGY:linear
CLONE:HUMGS03083
SEQUENCE DESCRIPTION:
GATCACTGTG GACAGGGTGC AGCTCTACCA GTTCCTGTTT CTNCTGAGCC AGACCCTCTT 60
CAGGGAAGGG ACCAATTAAT TTTAAAACTC ACTTGAAGCA CAGCTGGTCA TGGGGCTTGG 120
TATAAAGTTC CTATTTCCAC CCTGATACTT CCAATTCCTG GAACCCCAGC CCACTCCNCC 180
ATCCCTCCTC CCTATCAAAC TAGTATAATG ATTTTGAATC GGTACAGTGT GTTTAACTGT 240
AACTAAGTTC GACAGACTAT TATTATCTTT GTAATAAATT AACCTAGCAA TAAAAATTAT 300
TCTGTNTAAA 310

SEQ ID NO:2647
SEQUENCE LENGTH:308
SEQUENCE TYPE:nucleic acid
TOPOLOGY:linear
CLONE:HUMGS03084
SEQUENCE DESCRIPTION:
GATCTATTAA GCTTGANACA TTANGTNTCA TCACGCACTG AAGACAGGAA GCAGTTCACT 60
GAGTCAGCTG GTTCCCAAGC TCGCACAGAA GGTGATAAGT TACTATCAAA TGCCAGTNAG 120
AATCTNCTTA TAGAATAACC TGGGCCCAAG TNATTTTNGT ACAAAACTTG CCCTTCTTTG 180
GTTTAATTTN CTATGTGCTT TTAGGTGTGA ATCCAGATAT GCGGTCTTAA TTCCTTTGGA 240
AATACACAGT TCGTTTAGTT ACTGTACACT CTGTTTGTTC AATAAACTGC ATATCANCTT 300
CCNACAAA 308

SEQ ID NO:2648
SEQUENCE LENGTH:302
SEQUENCE TYPE:nucleic acid
TOPOLOGY:linear
CLONE:HUMGS03085
SEQUENCE DESCRIPTION:
GATCAGCAGC CCCTGAGCGG AGAAGAGGAG CTAGAGCCTG AGGCCAGTGA TGGTTCAGGC 60

TCCTGGGAAG ATGCAGCTTT GCTGACGGAG GCCAACCTGC CTGCTCCTGC TCCTGCTTCT 120
GCTTCTGCCC CTGTCCTAGA GACTCTGGGC AGCTCTGAAC CTGCTGGGGG TGCTCTCCGA 180
CAGCGCCCCA CCTGCTCTAG TTCCTGAAGA AAAGGGGCAG ACTCCTCACA TTCCAGCACT 240
TTCCCACCTG ACTCCTCTCC CCTCGTTTTT CCTTCAATAA ACTATTTTGT GTCAGCTTCA 300
AA 302

SEQ ID NO:2649
SEQUENCE LENGTH:302
SEQUENCE TYPE:nucleic acid
TOPOLOGY:linear
CLONE:HUMGS03086
SEQUENCE DESCRIPTION:
GATCAGCGAG CTACCANGAC CAGAGACCAA CTCAAGCTAC AGCAGAAGNA TTTTCCCAAG 60
CCTGCTGACC ACAGTCACAT CACCCATCAG CACATGGAAG GCCCCTGGTA TGGACACTGA 120
AAGGAAGGGC TGGTCCTGCC CCTTTGAGGG GGTGCAAACA TGACTGGGAC CTAAGAGCCA 180
GAGGCTGTGT AGAGGCTCCT GCTCCACCTG CCAGTCTCGT AAGANATGGG GTTGCTGCAG 240
TGTTGGAGTA GGGGCAGAGG GAGGGAGCCA AGGTCACTCC AATAAAACAA GCTCATGGCA 300
AA 302

SEQ ID NO:2650
SEQUENCE LENGTH:303
SEQUENCE TYPE:nucleic acid
TOPOLOGY:linear
CLONE:HUMGS03087
SEQUENCE DESCRIPTION:
GATCCCAGCT GATGTAGACC CTCTCACCAT TACTTCATCC CTGTNATCTG ATGGGGTCCT 60
CACTGTGAAT GGACCAAGGA AACAGGTCTN TGGCCCTGAG CGCACCATTC CCATCACCCG 120
TGAAGAGAAG CCTGCTGTCA CCGCAGCCCC CAAGAAATAG ATGCCCTTTC TTGAATTGCA 180
TTTTTTAAAA CAAGAAAGTT TCCCCACCAG TGAATGAAAG TTTTGTGACT AGTGCTGAAG 240
CTTATTAATG CTAAGGGCAG GCCCAAATTA TCAAGCTAAT AAANTATCAT TCAGCAACAG 300
AAA 303

SEQ ID NO:2651
SEQUENCE LENGTH:296
SEQUENCE TYPE:nucleic acid
TOPOLOGY:linear
CLONE:HUMGS03088
SEQUENCE DESCRIPTION:
GATCTCAGCT GGAAGTTCTG TTTGGAGCCC ATTTCTGTNA GACCCTGTAT TTCAAATNTG 60
CAGCTGAAAG GTGCTTGTAC CTCTAATTTC ACCCCANAGT TGGAGTTCTG CTCAAGGAAC 120
GTGTGTAATG TGTACATCTG TGTCCATGTG TGACCATGTG TCTGTNAGGC AGGGAACATG 180
TATTCTCTGC ATGCATGTAT GTAGGTGCCT GGGGAGTGTG TGTGGGTCCT TGGCTCTTGG 240
CCTTTCCCCT TGCAGGGGTT GTGCAGGTGT GAATAAAGAG AATAAGGAAG TTCAAA 296

SEQ ID NO:2652

SEQUENCE LENGTH:291
SEQUENCE TYPE:nucleic acid
TOPOLOGY:linear
CLONE:HUMGS03089
SEQUENCE DESCRIPTION:
GATCTNACGG CCAGGCCTCT NTCCTGCTGC TGTTTTTAAT TTTCCCAGGT AGTGGGAGAG 60
AGGAAAGGAG GGAAGGCAAG ATTCTTTCCC CCTCCCTGCT GAAGCATGTG GTACAGAGGC 120
AAGAGCAGAG CCTGAGAAGC GTCAGGTCCC ACTTCTGCCA TGCAGCTACT ATGAGCCCTC 180
GGGGCCTCCT CCTGGGCCTC AGCTTGCCCA GATACATACC TAAATATATA TATATATATA 240
TGAGGGAGAA CGTNTCACCC AGNTTTNATC ATGCTGGAAA GAGTGTATGT N 291

SEQ ID NO:2653
SEQUENCE LENGTH:290
SEQUENCE TYPE:nucleic acid
TOPOLOGY:linear
CLONE:HUMGS03090
SEQUENCE DESCRIPTION:
GATCTAAAGG GTGTAAAAAT ATTGATACTT CAATATTTCA CTTGCTGCCA GGAAAAACAA 60
AATTCTCAAT CTTTTGTAAA TGGGAGGAGG ACTTTTGCAT ACATTTTTAC TCTTTAAATA 120
ACGACAACGA CACTTATACT GTCATAATAA CAATNATGTA TTTCTTTGTG GTTTTAATTT 180
TTTTTGTAAT TTTACATAAA ACAGTTATTT TCTATTTTNA CGCAGATAAA TATTTGTGCA 240
TAAAATGTAA AAATAGTAAA ATGAGAAAAA TAAAACTATT ATACAGTAAA 290

SEQ ID NO:2654
SEQUENCE LENGTH:287
SEQUENCE TYPE:nucleic acid
TOPOLOGY:linear
CLONE:HUMGS03091
SEQUENCE DESCRIPTION:
GATCTGTCCT CAGCTCTGGA CCCAATTCCC CTTACTTCAT TTTGGCAAAC ACTAAGTCAA 60
ATAGTGAAAT GCCTGTCACT ACATAGAACC TATTACCTGG GGCAAATACG AACAGATTGA 120
GTTTCCTTCA TCTTGTGTAA ATATGATGAA ACAGAGACCT GGTAACTTGG TGACACTGTT 180
AAACCCTTTT NGGGATAAAG CCAAATGTAA ATGGAAACAT TAAACAGATA ANTTGTGGTG 240
TTGAGACTTT TCTGAATTGA GAAAAATAAA TGTAATTTTG GAAGAAA 287

SEQ ID NO:2655
SEQUENCE LENGTH:287
SEQUENCE TYPE:nucleic acid
TOPOLOGY:linear
CLONE:HUMGS03092
SEQUENCE DESCRIPTION:
GATCTGACCG GCTTCAAGCT CAAGCCCTAC GTGAGCTACC TCGCCCCTNA NGAGCGAGGA 60
GACGCCCCTG ACGGCCGCGC ACTCTTTCAG CGAANCNGTG GCGCCTGCCA TCGAAAAGNA 120
CTTCAAGGAC GGTACCTTCG ACCCTGACAA CCTGGAAAAG TACGGCTTCG AGCCCACACA 180
GGAGGGAAAG CTCTTCCAGC TCTACCCCAG GAACTTCCTG CGCTANTGGG CGGGGGAGGG 240

GCGGCCTGCC CTCATCTCAT TTCTATTAAA CGNCTTTGCC AGCTAAA 287

SEQ ID NO:2656
SEQUENCE LENGTH:341
SEQUENCE TYPE:nucleic acid
TOPOLOGY:linear
CLONE:HUMGS03094
SEQUENCE DESCRIPTION:
GATCGTGGAG GAGGGACAAC GCTGCCACCG NTTCGNCTGC AACACGTGCC CCTACGTGCA 60
CAACATCACC CGCAAGGTAA CAAATCGGAA GTACCCAAAA CTGAAAGAAG TGGATGATGT 120
GCTTGGTGGA GCAGCTGCCT GGGAGANTGT TGACTCTACT GCAGAGTCGT GTCCCAAATG 180
CGAACATCCT CGTGCTTACT TCATGCAGCT TCAGACCCGC TCTGCAGATG AGCCGATGAC 240
CACCTTCTAC AAGTGCTGCA ATGCTCAGTG TGGACACCGC TGGNGGGATT NGGGNCAGGT 300
NTGGCCCAGC TGCCCTNGTG TGTGCTTGCC TTGTNCCTCN N 341

SEQ ID NO:2657
SEQUENCE LENGTH:279
SEQUENCE TYPE:nucleic acid
TOPOLOGY:linear
CLONE:HUMGS03095
SEQUENCE DESCRIPTION:
GATCCACAAG GTTGTCTGCT AAACCTGCTC CTCCAAAGCC ACAGCCCAAG CCTAAAAAGG 60
CCCCTGCAAA GAAGGGAGAG AAGATACCCA AAGGGAAAAA GGGAAAAGCT GATGCTGGCA 120
AGGAGGGGAA TAACCCTGCA GAAAATGGAG ATGCCAAAAC AGACCAGGCA CAGAAAGCTG 180
AAGGTGCTGG AGTTGCCAAG TGAAGTGTGT GCATTTTTGA TAACTGTGTA CTTCTGGTGA 240
CTGTACAATT TCAAATACTA TTTTTATCA AGTTTTAAA 279

SEQ ID NO:2658
SEQUENCE LENGTH:279
SEQUENCE TYPE:nucleic acid
TOPOLOGY:linear
CLONE:HUMGS03096
SEQUENCE DESCRIPTION:
GATCTGAATG TNCAGTTCTA GCCAAGGTAG ATTTTACTTT CAACTTTTTA ATCAGTATCA 60
CTTTCTGTGC TTAACTATTT GGTGTTACCT TGTCTGTTTT CATTTGTCTA AAATNCTGTA 120
GAGATGACTA AAATTTGACA TAATGGTGTA AATGGATTGT NAAGGTAACT TTCAGTTGAN 180
GATTAANGCA AGANGCCATT TTCCCCATGA CTTTGGTTTT GTTTACATTT TNCCCTTTCA 240
GTTAGTATAC ACTACACGTA CTGTAATAAA GTNCAATAN 279

SEQ ID NO:2659
SEQUENCE LENGTH:274
SEQUENCE TYPE:nucleic acid
TOPOLOGY:linear
CLONE:HUMGS03097
SEQUENCE DESCRIPTION:

GATCTTTNCT AAAGCAAGGC CTTGAGCAAG TAGGTTGCTG TCTCTAAGCC CCCTTCCCTT 60
CCACTATNAG CTGCTGGCAG TGGGTTTNTA TTCGGTTCCC AGGGGTTGAG AGCATGCCTG 120
TGGGAGTCAT GGACATGAAG GGATGCTGCA ATGTAGGAAG GAGAGCTCTT TGTGAATGTA 180
AGGTGTTGCT AAATATGTTA TTGTGGAAAG ATGAATGCAA TAGTAGGACT GCTGACATTT 240
TGCAGAAAAT ACATTTTATT TAAAATCTCC TAAA 274

SEQ ID NO:2660
SEQUENCE LENGTH:259
SEQUENCE TYPE:nucleic acid
TOPOLOGY:linear
CLONE:HUMGS03098
SEQUENCE DESCRIPTION:
GATCTGCCTG CNTTGGCCTC CCAAGGTGCT GGGATTACAG GTGTGAGCCA CCACCCCCAG 60
CCGCCTCTAT GCTTTTGGNT TCTTTGATAT ATCTAAACTT AAGGTCAGAA CAGAAATTCA 120
TGTGCTGAAC AGAATGGATT CTTTTTGCAG TACCAATCCA ATTGAATCCT CTTTTTATTT 180
TCCTGCGTCC TTACCAGTCA CTTTGATAGT GCTAACTAAC CTACACTATG GGGGTTATTC 240
ATGANTTAAG TATGATAAA 259

SEQ ID NO:2661
SEQUENCE LENGTH:272
SEQUENCE TYPE:nucleic acid
TOPOLOGY:linear
CLONE:HUMGS03099
SEQUENCE DESCRIPTION:
GATCTGTGTT TGTAATACTG GTTGTAATTA ATTTTTTTAA TTCATGAACN AGCGGAAAAT 60
TTATTAAATT AACTATTAAC CACATTCACC TTGTAAATGA CTGTATAAAA CTNGTNGACA 120
ATGCACTGAC TTTAGAAAGA TGTTAATGTG CATAANTNGA GTGTAAATAA NATAGTGTTG 180
ATGTACTGAN ATATGACCTG TATAANNNGT ATTAGTAATT GTNTATGGGG TGTACCTGTT 240
TATCTGTAAC TGTNATCCAA ACAAATNAAA TN 272

SEQ ID NO:2662
SEQUENCE LENGTH:270
SEQUENCE TYPE:nucleic acid
TOPOLOGY:linear
CLONE:HUMGS03100
SEQUENCE DESCRIPTION:
GATCTATCTG GNNTGTGCNT TAGACTNGAT GAGAAGGTGT GAACTCTGCA GAAAGTCTCT 60
CCTTCATCAC TGAATTCAGT CACTTGGAGA TGACAACTTC AAATGCTAAC CCGATGACCC 120
CAGAAAACCG TGTGAGATTC GTACCGAAGA ACCTTGTGGA ATCCCTTTNC TTAGGCCCAA 180
CCTGGTCGAT AGCTCGAGAA AGANTNTTTN CCAAGGAAAT GTCTCGGATA TGGGTACTGT 240
ATTTGAAAGC TGTTAGCTTT GTCAACACGN 270

SEQ ID NO:2663
SEQUENCE LENGTH:268
SEQUENCE TYPE:nucleic acid

TOPOLOGY:linear
CLONE:HUMGS03101
SEQUENCE DESCRIPTION:
GATCATGAAT CAAGCCCTGG AACAGATGAA GACAAAAGCG GATGAGTGAG TTATATAAAC 60
TTACTTCCAT TCTGTTTCGG ATTTTAAGTT TGAGAGACTT GCTAATNAAT CTCCTTTATG 120
TTGTTTTCCT TTTCATTGTT TTTGGATTGT TTTATGTTTG TCCTTTTTTT TCTTAATGTG 180
GATTTCATTG AGTTGATTTT TTGATAATCT GCAATCTGGA TAATTTGTAC TGCTAAAGTT 240
TTAATAAACT CGACATGAGA AAAACAAA 268

SEQ ID NO:2664
SEQUENCE LENGTH:267
SEQUENCE TYPE:nucleic acid
TOPOLOGY:linear
CLONE:HUMGS03102
SEQUENCE DESCRIPTION:
GATCAAAATA GTAATAAATC AACAAATAAT TTTTGATTGA CTGGTGGAAT TAGATTTACT 60
AAATAAAAAT GGCTTAAATT TTGACTGTAC TAACTATAAA TGTAATGAAT TATAATAATT 120
ATAAATGGTA TAAATGCATT CTACAATGTG ATTATTTTCT GTATTTTAGT ATATAGTATA 180
AAATGGAATT ATTGGGTTAA AGTACTTTTA TTACATTTCT TAATCCATTG TTTTTACCTA 240
ATAAAATTTC CTCCTCTATA TTCCAAA 267

SEQ ID NO:2665
SEQUENCE LENGTH:266
SEQUENCE TYPE:nucleic acid
TOPOLOGY:linear
CLONE:HUMGS03103
SEQUENCE DESCRIPTION:
GATCCGAATC TGTGCCCAGC GCTAAAGGCT CAGTGTTAGC ATGGCTTNNN CTGNCCGGTG 60
TGCCATATTC TTGTTGGAGA TGAACCGTAG CACCAGAGCC CATTCTTCCT TGTCAGTCTT 120
GGCCCAAAGA TGTCACCATT CCTAGTTATT TGTCACCACA TAATTGGTGT TGATTGGAAA 180
CTTTTTCTGA GATGGGACAG AACTGCTGGG TTGTCTTTTT CCATGTAACT TAAGCATAGT 240
AATATAAATA AAGTAATAGT TGGAAA 266

SEQ ID NO:2666
SEQUENCE LENGTH:265
SEQUENCE TYPE:nucleic acid
TOPOLOGY:linear
CLONE:HUMGS03104
SEQUENCE DESCRIPTION:
GATCCTCCCT GTCATGCCAC ATTTCACTGA TTGGAATGTG GAAATGGAAA AGGAATTTAG 60
GATGTGCATT TTCACCTGAG GTTTCCCTGC ATCCCTGCAG TGCCAAAGCC CCACCTTCAG 120
GGACCACCTG GAATGTGTGA GGGGCTGACA GCACAGTAAC GTGCATACAT ATCTGCAGGG 180
CTGGAATGGA AGCTTTAAAG GTGGTAGTGA TTTTNATTTT GGAAGAATCA TGTTACCTTT 240
TTGTTAAATA AAATTTGTAC TCAAA 265

SEQ ID NO:2667
SEQUENCE LENGTH:303
SEQUENCE TYPE:nucleic acid
TOPOLOGY:linear
CLONE:HUMGS03105
SEQUENCE DESCRIPTION:
GATCCAGTGT CCATGGAAAC ATTCCCACAT GCCGTGACTC TGGACTATAT CANTATTTGG 60
AAAGCAGGGT TCCTCTGCCT GCAAACAAGC CCACGTGGAC CAGTCTGAAT GTNTTTCCTT 120
TNCACCTATG TTTTTAAGTA GTCAAACTTC AAGAAACAAT CTAAACAAGT TTCTGTTGCA 180
TATGTGTTTG TAAACTTGTA TTTGTATTTA GTAGGCTTCT ATATTGCATT TANCTTGTTT 240
TTGTAACTCC TGATTCTCCC TTTNCGGATA CTATTGATGA ATAAAGGANT TAANGTGGAT 300
AAA 303

SEQ ID NO:2668
SEQUENCE LENGTH:263
SEQUENCE TYPE:nucleic acid
TOPOLOGY:linear
CLONE:HUMGS03106
SEQUENCE DESCRIPTION:
GATCAACCAA GGCAGATGTG ACAGCAAGGG CCACGGACCC CATGGCAGGG GTGGCGTCTT 60
CATNAGGGAG GGGCCCAAAG CCCTTGTGGG CGGACCTCCC CTGAGCCTGT CTGAGGGGCC 120
AGCCCTTAGT GCATTCAGGC TAAGGCCCCT GGGCAGGGAT GCCACCNCTG CTCCTTCGGA 180
GGACGTGCCC TCACCCCTCA CTGGTCCACT GGCTTGAGAC TCACCCCGTC TGCCCAGTAA 240
AAGCCTTTCT GCAGCAGCTG AAA 263

SEQ ID NO:2669
SEQUENCE LENGTH:258
SEQUENCE TYPE:nucleic acid
TOPOLOGY:linear
CLONE:HUMGS03108
SEQUENCE DESCRIPTION:
GATCTGGCCT AGGTCCCCCC TTTNTNCTGT CCCCTGTNTT TAAGTCGGGA TTTTTACAGA 60
GGGAGCTGTC TCCAGACAGC TCATCAGGAA CCAAGCAAAG GCCAGATAGC CTGACAGATA 120
GGCTAGTGGT ATTGTGTATA TGGGCGGGAC GTGTGTGTCA TTATNATTTG AGTTATGCTG 180
TTGTTTAGGG GTAAATAACA GTAAATAATT AATAATAATA ATAATAATAN TAATAATAAN 240
GGNGCNGACG TTCTTAAA 258

SEQ ID NO:2670
SEQUENCE LENGTH:257
SEQUENCE TYPE:nucleic acid
TOPOLOGY:linear
CLONE:HUMGS03109
SEQUENCE DESCRIPTION:
GATCCACCCG CCTCGGCCTC CCAAAGTGCT GGGATTACAG GCATGAGCCA TCACACCTGG 60
TCAACTTTCT TTTGATTAGT GTTTTTGTGG TATATCTTTT TCCATCATGT TACTTTAAAT 120

ATATCTATAT TATTGTATTT AAAATGTGTT TCTTACAGAC TGCATGTAGT TGGGTATAAT 180
TTTNATCCAG TCTAAAAATA TCTGTCTTTT AATTGGNGTT TAGACAATTT ATATTTAATA 240
AAATGGNNGA ATTTAAA 257

SEQ ID NO:2671
SEQUENCE LENGTH:257
SEQUENCE TYPE:nucleic acid
TOPOLOGY:linear
CLONE:HUMGS03110
SEQUENCE DESCRIPTION:
GATCAGATGT NGCTCAGAAA CTTTCATTGT TTACCTAATA ATTTAATATC ACTAGTTTCC 60
TAGTGGGTCA AGCAGATGCA AAANCCAGCT TATTTCCTCC TATGTNCTCT CAAGCTTATN 120
GCTTATTTNA AAGTAAAANC CTGAAAAAGG AAAATATTAG GTTGGTGCAA ACGTAATTNC 180
GGTTTTGCCA TTGTNGAAAT TTGCCGTTTT ATATTGGAGT ACATTCTTAA NTAAATGTGG 240
TTATGTTATA CATCAAA 257

SEQ ID NO:2672
SEQUENCE LENGTH:256
SEQUENCE TYPE:nucleic acid
TOPOLOGY:linear
CLONE:HUMGS03111
SEQUENCE DESCRIPTION:
GATCAATTAC TGTCCTCACT TAGAACAAAG CCTGAGTCCG AGAATATTTA TATTTTACCA 60
ATATATGCCT GTTACAAGAG AAGGAAATAT GAGTTATTTA AGTTTAACTT TTTTATGTGA 120
ATTCAGAGTT TATTTATCGA GGGAAATATG TACAAAGAAG CTTCAAATGG AATATTTACC 180
GACATTCCTT ATACATGACA GACACTNGGC TACATGGGAA GATGATGTTA ATAATAANNT 240
GATTTTTAAN TGGAAA 256

SEQ ID NO:2673
SEQUENCE LENGTH:256
SEQUENCE TYPE:nucleic acid
TOPOLOGY:linear
CLONE:HUMGS03112
SEQUENCE DESCRIPTION:
GATCTGGAGA GGATTAGGGA CGGCTCTTGT GGGCACATTT TNNCTCTAGT TTCATGGGTG 60
GAGATGGCAT CTCCNGTGGT CTGCAGCATG CAAGTAAAGA CACCAGGTGG CACCATTNAT 120
NAGCTTTCAA TCCCATGGAG AGGTCCCATC ATGTAACTNC CAAGAGGTTG AAGGAACCAA 180
CTACCTCTTG CATATTGTNG CTAATGGTTC TNGCCTTCTC CTTCCAGACT TGGGAAATAA 240
NTTTNAAAAT NGTAAA 256

SEQ ID NO:2674
SEQUENCE LENGTH:433
SEQUENCE TYPE:nucleic acid
TOPOLOGY:linear
CLONE:HUMGS03115

SEQUENCE DESCRIPTION:
GATCTACCTT GGTGATTTGG ACCCTGGTTG CTTTGTGTCT AGTTTTCTAG ACCCTTCATC 60
TCTTACTTGA TAGACTTACT AATAAAATGT GAAGACTAGA CCAATTGTCA TGCTTGACAC 120
AACTGCTGTG GCTGGTTGGT GCTTTGTTTA TGGTAGTAGT TTTNCTGTAA CACAGAATAT 180
AGGATAAGAA ATAAGANTAA AGTACCTTGA CTTTGTTCAC AGCATGTAGG GTGATGAGCA 240
CTCACAATTG TNGACTAAAA TGCTGCTTTT AAAACATAGG AAAGTAGAAT GGTTGAGTGC 300
AAATCCATAG CACAAGGTAA ATTGAGCTNG TTANGGCAAT CAGGTAAAAT AGTCATGNTT 360
CTNTGTNATG TAANCCCGTA AATGANTGTT CCTGGTTTCN CGGTGGTTTT TTNANGGGNC 420
NCNTTNANNT TTN 433

SEQ ID NO:2675
SEQUENCE LENGTH:250
SEQUENCE TYPE:nucleic acid
TOPOLOGY:linear
CLONE:HUMGS03116
SEQUENCE DESCRIPTION:
GATCAGAAGA AAGAAAGACA ACTTTCCTCT GCGCGGAACA CTCACACGGA AGGGCTGGCC 60
GCCTCCCTGA GCCGGCTGGG AGTNGACGAC AGGACCTACC TCCCCAGAGC AAGGGCCTGG 120
GGCTTCCCGC CAAAGCTGCC GCGGAACCCC GCTAGTGCGA CCACCCTCCC TCCGTCGGTA 180
TGTCCTGCTT TCCAGCTGAA CCCAAACTAC AAGTGGGTTT AAAAAAATAA ACACCACCAC 240
CAAAAACAAA 250

SEQ ID NO:2676
SEQUENCE LENGTH:247
SEQUENCE TYPE:nucleic acid
TOPOLOGY:linear
CLONE:HUMGS03117
SEQUENCE DESCRIPTION:
GATCTGAAAA AGATGGTCAT TCCGGACTCC TGACGCCGCC AGTCCCGCGG TGAGACGTGA 60
GCNCCATTGG GTCCAGTGGC CTCTNTTTCC GTGGCAACCT AGTAACCATT AATTTTNAAT 120
TAAAGGAGAC AAAAAGCTCG ATGACAGCTC CAGGTCTGCT GAAGATGTCA AGAATCTGTA 180
TTAATATACA GCAAGAGAGC ATAATTGTNT GTCCATCTTC CAGAGCAGCG AAAAATGGAG 240
GGATAAN 247

SEQ ID NO:2677
SEQUENCE LENGTH:245
SEQUENCE TYPE:nucleic acid
TOPOLOGY:linear
CLONE:HUMGS03118
SEQUENCE DESCRIPTION:
GATCAAAACC ATCCTGGCCA AAATGGTGAA CCCTGTCTCT ACTAAAAATA CAAAAATTAG 60
CTGGGCATAG TGGCACGCGC CTGCAGTCCC AGCTACTCAG GAAGCTGAGG CAGGAGAATC 120
GCTTGAACCC AGGAGGCAGA GGTTGCTGTG AGCCGAGATT GCACCACTGC ACTCCAGCCT 180
GGGCAACAGA GTGAGACTCC ATCTGAAAAA AAAAAANTNC AGGCNTGCAA GNTTGGCGTA 240
ATCAN 245

SEQ ID NO:2678
SEQUENCE LENGTH:347
SEQUENCE TYPE:nucleic acid
TOPOLOGY:linear
CLONE:HUMGS03119
SEQUENCE DESCRIPTION:
GATCAGCAGG GCTACCATCT CACTCTTCTG TAATTTCACA ACATTCTAAA GGAAGTAAAT 60
CACCAGATTT GCTGATGTAT CAGGGTCCAC CAGACACTGC AGAAATAATA AAAACATTAC 120
CTCAGNAATA CAGNAGGAAA CTTGTGTCTC AAGAAGAAAT GGAATTTATC CAACGTGGAG 180
GTCCTGAATA ACCATGGTGG CTGCTGTTTG TCATCAGACA NTAGAATTGT CTTTACAATA 240
AAGGNCTTCC AAAATGGCAG ATGNGAAACT GTATATTAAA CACCTTTAGT AANTANTATG 300
GANNAAGTGN NATNTAGGNN ATTTAGGTGG TCCCTNGGAT TNCCGNN 347

SEQ ID NO:2679
SEQUENCE LENGTH:242
SEQUENCE TYPE:nucleic acid
TOPOLOGY:linear
CLONE:HUMGS03120
SEQUENCE DESCRIPTION:
GATCTGGCCT GGAGAGGCCA TCCTATACCC CTTATTAGAG CCATGACAGC CTACAGAGTG 60
AGGTGAGGTG CTCCCACCTT CCCAGATGGT TCCTTTCTGC CCCTTCCTGG AAGGAAAGGT 120
GAGGCTGCCA ATAGCCTCCT GGCACCAGCC AGACCTCACC CTTGACCAAC CTCTCGGGGC 180
TGGGGGTTCA TTCCTGGGGC ACTGTGGCCT GGTTTTGCTT TGAAACCAAG AAAGAGCAAA 240
GN 242

SEQ ID NO:2680
SEQUENCE LENGTH:411
SEQUENCE TYPE:nucleic acid
TOPOLOGY:linear
CLONE:HUMGS03121
SEQUENCE DESCRIPTION:
GATCCCCAAC AATGTGAAAA CGGCTGTCTG TNACATCCCA CCTCGGGGGC TAAAAATNTC 60
CGNCACCTTC ATTGGCAACA GCACGGCCAT CCAGGAGCTG TTCAAGCGCA TCTCCGAGCA 120
GTTCACGGCC ATGTTCCGGC GNAAGGCCTT CCTGCACTGG TACACGGGCG AGGGNATGGA 180
CGAGATGGAG TTCACCGAGG CCGAGAGCAN CATGAATGAC CTGGTGTCCG AGTACCAGCA 240
GTACCAGGAT GCCACAGCCG AGNAGGAGGG CGAGTTCGAG GAGGAGGCTT AGGAGGAGGT 300
TGGCCTAGAG CCTTCAANTC ACTGGGGAAA AGCAGGGGAA GCAGTGTNGA ACTCTTTTAT 360
TCACTTCCNA GNCTGGTCCN NGTNGGNCTN GTTCCCACTG TGTGCAATTG N 411

SEQ ID NO:2681
SEQUENCE LENGTH:240
SEQUENCE TYPE:nucleic acid
TOPOLOGY:linear
CLONE:HUMGS03122

SEQUENCE DESCRIPTION:
GATCTGCGCA CGGTGCACAG CCCACCAGAG CACTACAGCC TTTTATTGAG TGGGGTGGGG 60
CAAGTGCTGG GCTGTGGTCG TGCCCTGACA GCATCTTCCC CAGGCAGCGG CTCTGTGGAG 120
GAGGCCATAC TCCCCTAGTT GGCCACTGGG GCCACCACCC TGACCACCAC TGTGCCCCTC 180
ATTGTTACTA CCTTGTGAGA TAAAAACTGA TTAAACCTTT GTGGCTGTGG TTGGCTGAAA 240

SEQ ID NO:2682
SEQUENCE LENGTH:237
SEQUENCE TYPE:nucleic acid
TOPOLOGY:linear
CLONE:HUMGS03123
SEQUENCE DESCRIPTION:
GATCACCTGG GGACAGAGGT GAAAGGCCTG CTGGGCCTGC TGGAGGAGCT GGCCTGGAAC 60
CTGCCCCCGG GACCCTTCAG CCCCGCTCCC GACCTTNTCG GAGATGGCTT CTNAGCCCTG 120
GAGCTGGAGC CCAGCAGTTG GAGGTGGTGC ACCTGCCAGG CAGCGCCCAC AGAACCAGCC 180
CTGTCCTCTC GACTTCCTTC CTTAGCTTCA TGTGAAATAA AAGCTATTCT GGTCAAA 237

SEQ ID NO:2683
SEQUENCE LENGTH:236
SEQUENCE TYPE:nucleic acid
TOPOLOGY:linear
CLONE:HUMGS03124
SEQUENCE DESCRIPTION:
GATCGGCATG GTCATTCACC AAGTGGAAGC CAAACCCTCT GTGTATTTCA TCAAGTTTGA 60
TGATGATTTC CATATCTATG TCTACGATTT GGTGAAAAAG TCCTAACTGT TAGGGTAAAA 120
TTTGGCACAT GTGTGGAAAC AAATGTATAA TTTGTAGACA TGCAAAAAAT GTTGCCTTTC 180
AGTGTATTGA AAGCTTATGG AATCCCTGAT AACTAAACAT CTTTGCCAGC ATTAAA 236

SEQ ID NO:2684
SEQUENCE LENGTH:236
SEQUENCE TYPE:nucleic acid
TOPOLOGY:linear
CLONE:HUMGS03125
SEQUENCE DESCRIPTION:
GATCACACAT ATCCTTCCTG AGANGAGCAG TGACTAAAAT GGAATATCTT TTTAAGANCA 60
GCTCCTCTTT AACAAAAAAN CTTAAAAGAC AAATGTGAGA TGGGCTTAGA GTTAGTNCTC 120
TGGGAACTTG AAAGACATTT ATGCCATATT ATTTATNCAC GTGTTTGTNC CTGGTGGGCA 180
AGATGCCATC TGAGGCTTCA GATGAGAAAT TGGGGTAANN TGGAAATTTT NCACTN 236

SEQ ID NO:2685
SEQUENCE LENGTH:234
SEQUENCE TYPE:nucleic acid
TOPOLOGY:linear
CLONE:HUMGS03126
SEQUENCE DESCRIPTION:

GATCAATCCC TCAAGATTTT GAAATCCTGA AGAAATTGAG AAGTATGTTG CTGAAATTGA 60
AAANGAAAAA GAAGAAAACG AAAAGAAGAA ACAAAAGAAA GCATCATGAT GAATAAAATG 120
TCTTTGCTTG TAATTTTTAA ATTCATATCA ATCATGGATG AGTCTCGATG TGTAGGCCTT 180
TCCATTCCAT TTATTCACAC TGAGTGTCCT ACAATAAACT TCCGTATTTT TAAA 234

SEQ ID NO:2686
SEQUENCE LENGTH:230
SEQUENCE TYPE:nucleic acid
TOPOLOGY:linear
CLONE:HUMGS03127
SEQUENCE DESCRIPTION:
GATCTAGGCC TGGCAAGANC TCTGGCCCCA AGGCCTCCTC TTCCCAGGGG CTGCCAAGTC 60
CTGGCCCTGG CCCTGGCATA TNACCCCGNA CTGTGGGGCC AGTCACCACT AGCCTGGCTC 120
AAATATTCCC CAGGGAGACT NCTGTGTGCT GCCCGGCTNC CTGCTGGCTC TCCCCCAGNC 180
CCACATCCCN TCTNGAAGAG AATGTAAAAT AAACCTGGAC ACAAGGGAAA 230

SEQ ID NO:2687
SEQUENCE LENGTH:232
SEQUENCE TYPE:nucleic acid
TOPOLOGY:linear
CLONE:HUMGS03128
SEQUENCE DESCRIPTION:
GATCCATGAG GTCCTAGCCC CTGGCTGCCT GGATGCNTTC CCCCTGCTCT CAGCATATNT 60
GGGGCGCCTC ANTGCCCGGC CCAAGCTCAA GGCCTTCCTG GCCTCCCCTG AGTACGTNAA 120
CCTCCCCATC AATGGCAACG GGAAACAGTG AGGGTTGGGG GGACTCTNAG CGGGAGGCAG 180
AGTTTGCTTT CCTTTCTCCA GGACCANTAA AATTTNTAAG NGAGNTACTA AA 232

SEQ ID NO:2688
SEQUENCE LENGTH:229
SEQUENCE TYPE:nucleic acid
TOPOLOGY:linear
CLONE:HUMGS03129
SEQUENCE DESCRIPTION:
GATCACAAAA TGTNATAAAA CTTTAAATGT ATAAAACTTT ATCAAATAAA GTTTTATTTT 60
CCCCTTTAAA ATGTATTTNT TTAGAGGCAT TACTTTTTTA AAAATATTGG TCAATTNCNG 120
ACATAAGATG TGAGGTTCAC AGTTGTATTC CAGTATTCAA GATAGATTCC TGATTTTCCA 180
ATTAGGAAAA GTAAAATCCA AAATGTTAGC AAAACAAAGT GCAATATTN 229

SEQ ID NO:2689
SEQUENCE LENGTH:223
SEQUENCE TYPE:nucleic acid
TOPOLOGY:linear
CLONE:HUMGS03131
SEQUENCE DESCRIPTION:
GATCCCATGC CCAGGTGTCC AGCAGACCTC AAGGCAGAAG GGTCACCTAA CCCAGGAGTC 60

CACAGACTGA TGTGACCTCA GGTTCCCACA TCANTGGCCA CAGGGCAGGG CCCACCTGGT 120
AGAAGTTTTC TGGATATGGC CAGGGTGGGT GTNTGGCTAA NTGGGCCTGA ACAGAGGGAA 180
CCTAGGGCCC TTGGCCAATG TGATTAAAGC TGCCATCTTG AAA 223

SEQ ID NO:2690
SEQUENCE LENGTH:222
SEQUENCE TYPE:nucleic acid
TOPOLOGY:linear
CLONE:HUMGS03132
SEQUENCE DESCRIPTION:
GATCCGCCCT GGAGAAGTGA GTGGCTGAGA CCCGGGTGGG GCCCTCCTAA CCTTGGNTCT 60
GGGGGCTTCC NTTCTGGGTC TTGTCCGGGA TATGGCCTTC CTGAAGGTCT TACCTCCCCC 120
AGCCGCCCTG CACTTGACCA CCAAAGCAAT CTCTAAGCTT ATAAAGGAGA AACATGTATA 180
TATGGGGTTT TTTGGAGTAA AGGAAAATTC TAAGATATTA AA 222

SEQ ID NO:2691
SEQUENCE LENGTH:222
SEQUENCE TYPE:nucleic acid
TOPOLOGY:linear
CLONE:HUMGS03133
SEQUENCE DESCRIPTION:
GATCCACCTG CCTTGGCCTT CCAAGGTGTT GGAATTACAG GCGTGAGCAA CCGCGCCTGG 60
TCTATATGTN ATTTCAAATN AGGTGGCCTT ATAAATATAA GCAGATGGGT TTCTGGGCAA 120
AATCAAATNA TTTCTGTCTC TATTTNNTTG AGTACCAATT GTAAAANCTG AAATTAGGTT 180
GAGGAAAGTN CTTTAGAAAT AATAAAGATG TACAANTACA AA 222

SEQ ID NO:2692
SEQUENCE LENGTH:220
SEQUENCE TYPE:nucleic acid
TOPOLOGY:linear
CLONE:HUMGS03134
SEQUENCE DESCRIPTION:
GATCTGCCCA CCTCAGCCTC TTGAGTAGCT GGGACTACAG GTGCATACCA CCACACCTGG 60
CTAATTTTTT AATTTTNATT TTTATTAAAG ACAAGGTCTC ACTATGCTGC CCAGGTTGGC 120
CTCCCAAAGT GTTGGGATTA CAAGTGTGAG TCACCGTGCC TGGTCCCATG TACTTGANGT 180
CATCACTANC AAAATGTATA GATATTGTAT AATGGCAACN 220

SEQ ID NO:2693
SEQUENCE LENGTH:219
SEQUENCE TYPE:nucleic acid
TOPOLOGY:linear
CLONE:HUMGS03135
SEQUENCE DESCRIPTION:
GATCTTTAAT AAAAATTTGA TATGAAAAGC ACAATGTGCA GANGTTATGG AAAACCTATA 60
GAGGATTACA ACAGGTAAAC GTTAAAGAGA ATACATTGCT GACTTATAGT GATGTGGCTA 120

AGANGTACAT GCTTTGTTGT AAAATTNCTT GAAAGCCCAT TGAAAGATGT ATCTGTTTAT 180
TTACAGTCTT TGAAGTAAAA GTTACCAATG TTTGCCAAA 219

SEQ ID NO:2694
SEQUENCE LENGTH:219
SEQUENCE TYPE:nucleic acid
TOPOLOGY:linear
CLONE:HUMGS03136
SEQUENCE DESCRIPTION:
GATCTNGCCC CTGCGCTCTN GCCTGGGTGA CAGAGCAAGA NTCTATCTNA AAAATGTTTT 60
TAAAAAATAC TTCCTAGGGA AACACACATT TNGCCCTATT TGGAACTTTG AAAAGTGAGC 120
CCTCCTCTGC CCATGGGCCG TGCTGCTCAG CCTGCCATAC TCGCTTGCTT TGCTGTTTGG 180
NATTTGCCTC CAGAATAAAG GTCCTTTTTG TTGTTGAAA 219

SEQ ID NO:2695
SEQUENCE LENGTH:224
SEQUENCE TYPE:nucleic acid
TOPOLOGY:linear
CLONE:HUMGS03138
SEQUENCE DESCRIPTION:
GATCCTAATC CCTTCCTCTC CCATTCACCC TGTGTAACAG NACCCCAAGG ACCTGCCTCC 60
CCGGAAGTGC CTTAACCTAG AGGGTCGGGG AGGAGGTTGT GTCACTGACT CAGGCTGCTC 120
CTTCTCTAGT TTCCCCTCTC ATCTGACCTT AGTTTGCTGC CATNAGTCTA GTGGTTTCGT 180
GGTTTCGTCT ATTTATTAAA AAATATTTGA GANCAAAAAA CAAA 224

SEQ ID NO:2696
SEQUENCE LENGTH:216
SEQUENCE TYPE:nucleic acid
TOPOLOGY:linear
CLONE:HUMGS03139
SEQUENCE DESCRIPTION:
GATCAAAGTC TTAAGTGTGT TTGCAGCTCA AAAATAAAGA TGTATTAAGG AGGGGAAAAC 60
CTGGTCTAAG TGCAAGGCAC ACTTACAGCG AGTTTTACTT TCGGTTGTAT TTTCTTTGTA 120
TATNATAAAC ATTTATTTAA CTTGTTGCCG TTTGAAGTAA AAAATTTCCA AAATGTATGC 180
TCAACAATAA TCATTAAAAT GTTTTCAGCG TACAAA 216

SEQ ID NO:2697
SEQUENCE LENGTH:216
SEQUENCE TYPE:nucleic acid
TOPOLOGY:linear
CLONE:HUMGS03140
SEQUENCE DESCRIPTION:
GATCAAATTG TGTTTAGTAT CACTATCTTC TCTCCTCGTT TCTCTCTTAC TCCTCATCCT 60
CCCAGAATCT ACCAGTTTAT GGTAGAAAGA TGGGAACCTT ATTTGAATGT GTTTTTTTTT 120
TCCATGATGT CCAATTTTGT NGTGGGAAAG GATTTGGATA AAANTTTNGT TTAAATTTNG 180

GTAGNTTTTN ATCTNTACAA NTTTAANTAA AATNAN 216

SEQ ID NO:2698
SEQUENCE LENGTH:216
SEQUENCE TYPE:nucleic acid
TOPOLOGY:linear
CLONE:HUMGS03141
SEQUENCE DESCRIPTION:
GATCTCAAAA GTATTCCTCA GGGTAGTCTC CATTCCTGTC ATTTTNCCTC CTATTCATGA 60
GACGGAAAAA TTATGACCAT GGTACAGAGT TGTATCAAGG AAATGAAGAA CTAGAACATT 120
GAATTGAAAG GTTTTGAAAG GGTTCACAAT TTCTTTTCAT TTNCTATTAA TGTCTTCTTT 180
TAAATNCTCT TAAGAATAAA ACTTTTTTCT AATAAA 216

SEQ ID NO:2699
SEQUENCE LENGTH:211
SEQUENCE TYPE:nucleic acid
TOPOLOGY:linear
CLONE:HUMGS03142
SEQUENCE DESCRIPTION:
GATCAGCAAA CAGGAATACG ATGAAGCCGG GCCTTCCATT GTCCACCGCA AATNCTTCTA 60
AAACACTTTC CTGCTCCTCT CTGTCTCTAG CACACAACTG TGAATGTCCT GTGGAATTAT 120
GCCTTCAGTT CTTTTCCAAA TCATTCCTAG CCAAAGCTCT GACTCGTTAC CTATGTGTTT 180
TTTAATAAAT CTGAAATAGG CTACTGGTAA A 211

SEQ ID NO:2700
SEQUENCE LENGTH:236
SEQUENCE TYPE:nucleic acid
TOPOLOGY:linear
CLONE:HUMGS03145
SEQUENCE DESCRIPTION:
GATCACGAAC ANTAGCTTGC GCTCTACTCT GTAGTTATGT GGATTGCCGA GCAATNACCC 60
TTTTCAATNT CTTATTTCTG TGTTACTGAG GACCCTAATC ACTTAGGGAT GTAATTTTAT 120
AGTATAAACT TTCTGNACAG TTTTNCTNAT AGTCTAATAA GTAAAAAGTG TCCNNCAAAT 180
TATGATAATT GCCTATGGTA CATGGATAAN TTAANGCACT GCACACGGTG TAAANN 236

SEQ ID NO:2701
SEQUENCE LENGTH:206
SEQUENCE TYPE:nucleic acid
TOPOLOGY:linear
CLONE:HUMGS03146
SEQUENCE DESCRIPTION:
GATCCATTTC TNACACACAC TGCCAGAGAT ACTCTAGGCA TGTAAAGCAC AAACATACAT 60
ATAAAANCTG CGGGCTTCAA AAAATATAAG TAGGATGTCA TCTATACTGT CATACACTTT 120
GTTTTTTATC ACTTACTTAA TGTTATATCT NGGATATTGT ATTACCCTGG GTATTAAAAN 180
GAACTCCTTT CACATTTTAA AATAAA 206

SEQ ID NO:2702
SEQUENCE LENGTH:206
SEQUENCE TYPE:nucleic acid
TOPOLOGY:linear
CLONE:HUMGS03147
SEQUENCE DESCRIPTION:
GATCTAAAAC TGGGGTTTGG GGGAGGGTTT CANCCTCTCC CCACTCCNCT TGCCCCACAC 60
CCTTTACTCC CCAGCCCAGA GAGACGCTGC TTTTACCAGG AAAGACTATT GAAAGATGTT 120
TTATATTATT TTNCTCTGAC CTTTCCATCC TTGAAAAAAT GGGGAAAAAA GAAGAAAAAA 180
GACAAAATCG ACCATAAAAG ACCAAA 206

SEQ ID NO:2703
SEQUENCE LENGTH:205
SEQUENCE TYPE:nucleic acid
TOPOLOGY:linear
CLONE:HUMGS03148
SEQUENCE DESCRIPTION:
GATCTTGTTA ATGCATTTTT GATGGGAAGA AAAGGTACAT GTTTACAAAG AGGTTTTATG 60
AAAAGAATAA AAATTGACTT CTTGCTTGTA CATATAGGNG CAATACTATT ATATTATGTA 120
GTCCGTTAAC ACTACTTAAA AGTTTAGGGT TTTCTCTTGG TTGTAGAGTG GCCCAGAATT 180
GCATTCTGAA TGAATAAAGG TTAAA 205

SEQ ID NO:2704
SEQUENCE LENGTH:203
SEQUENCE TYPE:nucleic acid
TOPOLOGY:linear
CLONE:HUMGS03149
SEQUENCE DESCRIPTION:
GATCCTGAGG CTTTGATGCT ATTTTNTTAA TCCAGCTGGT CTGTGGAGAA GCCTGGGACA 60
GAAAGAAGCA GCCAGACTCC AGAATCCCCC ATTTTAATAG CTATNNNATG CTATGTCCCC 120
GTCTTATTCC TGGCAACTTA CCTCTCAGCT CGTGCACTGT GACATTACAG CAAGAAGTAC 180
TAATATAAAT GACACAAANG AAA 203

SEQ ID NO:2705
SEQUENCE LENGTH:203
SEQUENCE TYPE:nucleic acid
TOPOLOGY:linear
CLONE:HUMGS03150
SEQUENCE DESCRIPTION:
GATCTGAAAA AAATACGGTT GAAAGTGGTG CAGGTAATTA CAATGTGTAG GCAAACCAGA 60
AAACCATGGC TTTAACCAGC AGCTTTTGTN AGAAATNATT TCTCCAATGA NTGTAGAAAC 120
GTTTGCNGCT GAATTGTGAC CTTNCCATTT NACCTGCTTT TCCTGCAAAG TATATTTNNC 180
AGACCCAGGC AGTGCTGCTC GCN 203

SEQ ID NO:2706
SEQUENCE LENGTH:198
SEQUENCE TYPE:nucleic acid
TOPOLOGY:linear
CLONE:HUMGS03151
SEQUENCE DESCRIPTION:
GATCCAATGC TAAACTCCAA TGGTTCAGTG ACTGTGGTTG CTCTTCTTCA AGGCAGCTGA 60
TACCTGTGCA TACTGCAGGC ATCTAAATTA GAAGACCTGC GAGTAAAACT GAAGAAAGAA 120
GGATATTCTA ATATTTNTNA TATNGTTGTT AATCATCAAG GNATCTNTTC TCGATTAAAA 180
TNCACACATC TTANGAAA 198

SEQ ID NO:2707
SEQUENCE LENGTH:201
SEQUENCE TYPE:nucleic acid
TOPOLOGY:linear
CLONE:HUMGS03152
SEQUENCE DESCRIPTION:
GATCCATAAT TCCTATTCAA TGAACATCAT TGTTGGTGGC TGAATACATT TTGGAAGAGA 60
GTTTTTCATC TTAGAGATTG GTGAACAAGT GTGAGGGTGT GAGAAACTCA CAGAATACAA 120
ATTTGCCTGT ATGTTTTGTG GGTTTTTTTT TTCCCCTTCA NGATGTTTNC TATTNCTAAN 180
TTAANGTAAT TNCAAGGTAA A 201

SEQ ID NO:2708
SEQUENCE LENGTH:196
SEQUENCE TYPE:nucleic acid
TOPOLOGY:linear
CLONE:HUMGS03153
SEQUENCE DESCRIPTION:
GATCTATTAG TTTAGTGTTT ATNAAAGAAT CAAATGTATA GAATTACCAA GCATTCGTGG 60
GGAATNCTGT GTAGCAAATG TAAAACTNAC CTGCTCGGAA GAAACGTAGG ANCGCTTCAA 120
ACCCACTGTA ATGTTTGGTT TGANNTTATT TCCATTGCTT TNAGAGTGAN CTGCCTAAGN 180
GTAGGCCTTA TAATAN 196

SEQ ID NO:2709
SEQUENCE LENGTH:193
SEQUENCE TYPE:nucleic acid
TOPOLOGY:linear
CLONE:HUMGS03154
SEQUENCE DESCRIPTION:
GATCTACAAG NATTTTCTTT TAGTAGTTAT ACAAAAGANT ATTACATTTA TTGGTCTGTA 60
CAGTGTAATA AACCAGCAAT AGGGACAACA ACAAAGAGAA TCTAAGAAAG ANGACTATGC 120
CTAANTATAG GGAAAGTTTA GAGAATCATT TTTAGACAGG ACACATTTTG CAGTAGTGAA 180
TGTGGTAGGT TTN 193

SEQ ID NO:2710

SEQUENCE LENGTH:193
SEQUENCE TYPE:nucleic acid
TOPOLOGY:linear
CLONE:HUMGS03155
SEQUENCE DESCRIPTION:
GATCAGTGTT GCAACATANT GNAAAAGATG GCTACTGTGC CTTGTGTTAC TTAATCATAC 60
AGTAAGNTGA CCTGGAAATG AATGAAACTA TTACTCCTAA GANTTACATT GTATAGCCCC 120
ACAGATTAAN TTTAATTAAT TANTTCAAAA CATGTTANCC GCNACTTTCA TGTACTATGG 180
AAAAGTACAN GTN 193

SEQ ID NO:2711
SEQUENCE LENGTH:192
SEQUENCE TYPE:nucleic acid
TOPOLOGY:linear
CLONE:HUMGS03156
SEQUENCE DESCRIPTION:
GATCTCGAAC CCTGTCTAGA AGGAATGTAT TTGTTGCTAA ATTTCGTAGC ACTGTTTACA 60
GTTTTCCTCC ATGTTATTTA TGAATTTNAT ATTCCGTGAA TGTATATNGT CTTGTAATGT 120
TGCATAATGT TCACTTTTTA TAGTGTGTCC TTTATNCTAA ACAGTAANGT GGTTTTATTN 180
CTATCACACA AA 192

SEQ ID NO:2712
SEQUENCE LENGTH:386
SEQUENCE TYPE:nucleic acid
TOPOLOGY:linear
CLONE:HUMGS03157
SEQUENCE DESCRIPTION:
GATCCAGAAT NGAGACTGGA CCTTCCAGAC CCTGGTAATG CTGGAAACAG TTCCTCGGAG 60
TGGAGAGGTT TACACCTGCC AAGTGGAGCA TCCAAGCATG ATGAGCCCTC TNACGGTGCA 120
ATGGAGTGCA CGGTCTGAAT CTGCACAGAG CAAGATGCTG AGTGGAGTCG GGGGCTTTGT 180
GCTGGGCCTG CTCTTNCTTG GNACAGGGCT GTTCATCTAC TTCAGGANTC AGAAAGGNCA 240
CTCTGGNCTT CAGCCANCAG GACTCTTTGN GCTGAAGTGC AGGTGNCCAC GATTCAAGGG 300
ANGAACCTTC TTGNCCCANG NTTTTCAAGA TGGAAAAGCT TTCCCCAACT TTGGNTTCTT 360
NTTTNTTCCC ACCAAAGAAN CTTNAN 386

SEQ ID NO:2713
SEQUENCE LENGTH:189
SEQUENCE TYPE:nucleic acid
TOPOLOGY:linear
CLONE:HUMGS03159
SEQUENCE DESCRIPTION:
GATCTTCCGG AAAAACTAAG CCCCTCCTTC ACCCCCGCAC ACCTGCATCC CTGCCGGAGT 60
CNTGNTGAGG CACGAACCCT GCCTCCCTAG GCCGGACCTT GTGGACGACA GCCCCACCCA 120
GTCTGTGCTC TCAGCCGCTG GCCGAAGGCC CAGCCTGCTC AGAATAAAGC ATGTCCTGCT 180
GCCGGCAAA 189

SEQ ID NO:2714
SEQUENCE LENGTH:189
SEQUENCE TYPE:nucleic acid
TOPOLOGY:linear
CLONE:HUMGS03160
SEQUENCE DESCRIPTION:
GATCCCATAG TTTTACATTT TNCCTGTTTA TTTTGATTTT TTNCACTGCT TTATTTCTTA 60
AAGTNCTAGC ACATCTGTNA CTCCTCCACT TCCACATTTT TNCACTGCTT ACACTTACGT 120
GCAATCTTAT TCCTNGTCTG CACACACATG TGGAAAGCTA GAAATAAATG TTAAAACTNA 180
CTTTTTAAA 189

SEQ ID NO:2715
SEQUENCE LENGTH:187
SEQUENCE TYPE:nucleic acid
TOPOLOGY:linear
CLONE:HUMGS03162
SEQUENCE DESCRIPTION:
GATCAAATCT CTCCTGGCTG TAGTAACCCA GTGGAATGAA TTTGGNCATG CCCCAATGCT 60
TCTATATGCT AAGTGAAATC TGTGTCTGTA ATTTGTTGGG GGGTGGATAG GGTGGGGTCT 120
CCATCTACTT TTTGTCACCA TCATCTGAAA TGGGGAAATA TGTAAATAAA TATATCAGCA 180
AAGCAAA 187

SEQ ID NO:2716
SEQUENCE LENGTH:187
SEQUENCE TYPE:nucleic acid
TOPOLOGY:linear
CLONE:HUMGS03163
SEQUENCE DESCRIPTION:
GATCCAAGGG CACAAGGAGA TGAGCAAGGT GTGGAACGAC CTGAGGCCCA AGCTCAGCTG 60
CCTCTTTGCG GGACCACACA GCACCCTGAC CCCACCGTGC TCCNNGNCGG AGGACGGCCT 120
GTGTCCTCAN TAGCGCCTGA GGCTGNGGTG GTGCTCCCTG CGGNCGCACT AAAACCTCTT 180
TNCNAAA 187

SEQ ID NO:2717
SEQUENCE LENGTH:188
SEQUENCE TYPE:nucleic acid
TOPOLOGY:linear
CLONE:HUMGS03164
SEQUENCE DESCRIPTION:
GATCCTCCTG TTTACCCTGT ACCTCCAATG TCTGGCACTT GTAGGTGCTC AAATATTCGT 60
TGAATGAATG AAAAATCCAT ATTGTAATTG ATGTCCTCTG GCCACATAGT TTTAAAATTA 120
GGTGATTGAT TATATGACCG AATAGAACTA TCAAATGTTT TCCTAATAAA GTCAATATTT 180
CAAACAAA 188

SEQ ID NO:2718
SEQUENCE LENGTH:183
SEQUENCE TYPE:nucleic acid
TOPOLOGY:linear
CLONE:HUMGS03166
SEQUENCE DESCRIPTION:
GATCTGCCCC CCTCAGCCTC CCAAAGTGCT GGGNTTACAG GCATGAGCCN TCGCGCCCGG 60
CCGGATTTTT TTTTTTTTTG ATTCAGCTTA TACCAGGGCT GAAAACCTCA NTTTATGTTC 120
ATGACAGTNG GGATTTTTTT AAATGCCTAC ATTCTTTCTA ATAAACTGTT GGAAGACNTT 180
AAA 183

SEQ ID NO:2719
SEQUENCE LENGTH:182
SEQUENCE TYPE:nucleic acid
TOPOLOGY:linear
CLONE:HUMGS03167
SEQUENCE DESCRIPTION:
GATCCAAGTG GCCCAGCAGA AACGGAATTN TNGTNAAAAT GACTGATTCT GGTGCATGTN 60
AAAGGCTCTG GGGAATNAAT ACCTGTTTGG TCCAAACTTA CTGTATCATA ATGCTGTATA 120
TGTNGAAAAC GTAATACCTG TNAATGTCCT AATGAATCAN GTTTTGCAGA AAACACCGTA 180
AA 182

SEQ ID NO:2720
SEQUENCE LENGTH:181
SEQUENCE TYPE:nucleic acid
TOPOLOGY:linear
CLONE:HUMGS03168
SEQUENCE DESCRIPTION:
GATCCTCCAT TGGAGTGGCC CAATCTTTCC TCTAGGGCAA TCCTGAAAGC CCAAGGCCCT 60
TCNCAGTCTG GCCTTGGCTT CAGCTGGAGA AGGGTAACAT CAGTCATTGT CAAGGCACCN 120
CCANCCCNGA CAGACCGTGT CTCTGATAAA GGTTTTGAAG TGATAAAGTT TTAAAACTAA 180
A 181

SEQ ID NO:2721
SEQUENCE LENGTH:178
SEQUENCE TYPE:nucleic acid
TOPOLOGY:linear
CLONE:HUMGS03169
SEQUENCE DESCRIPTION:
GATCCTCACT CCACTGTCAT GTTTGAAAGA GGTATTGGTG TTCTGGAGTG GGGGTCAACC 60
CCTCCCCAGT CTCTGTGCTA AGTTCACTTG GGAGGATGGC AGTAGGAGCC AGGGCTGGCC 120
ATGGGTCCCC TACGCTTNCC NNTAGGATAT ACCCTGGAGA ATGGCAAAAT GATTTAAA 178

SEQ ID NO:2722
SEQUENCE LENGTH:287

SEQUENCE TYPE:nucleic acid
TOPOLOGY:linear
CLONE:HUMGS03170
SEQUENCE DESCRIPTION:
GATCCTAAGG CAAAGAGGTG TTTTTCCTTC TGGTTGATTC ATCCCAAAGC TTTCCCACCC 60
AGGTTTTCTC TGAAAGCTTA GCCTTAAGAG AACACGCAGA GAGTTTCCCT AGATATACTC 120
CTGCCTCCAG GTGCTGGGAC ACACCTTTGC AAAATGCTGT GGGAAGCAGG AGCTGGGGAG 180
CTGTNTTAAG TCAAAGTAGA AACCCTCCAG TNTNTGGTGT TGTGTAGAGA NTAGGNCATA 240
GGGTAAAGNG GCCAAGCTGC CTGTAGTTAG TAGAGAAGAN TGGGGAN 287

SEQ ID NO:2723
SEQUENCE LENGTH:178
SEQUENCE TYPE:nucleic acid
TOPOLOGY:linear
CLONE:HUMGS03171
SEQUENCE DESCRIPTION:
GATCACCATG ACTGCAACAT GGTTTATATT CATGCTGCCC TAGAAACTTT TGTAATTATT 60
TGTTGCAAAT TTGTGACTGT CCTTATTAAC TTTCTTTNAT GTAAGTAATT TGTAAAAGTT 120
TCTNAAAATT TTTGCTTTTG CTTATTTAAT TTTNAATAAA AGCTAAATTC CTAATAAA 178

SEQ ID NO:2724
SEQUENCE LENGTH:178
SEQUENCE TYPE:nucleic acid
TOPOLOGY:linear
CLONE:HUMGS03172
SEQUENCE DESCRIPTION:
GATCCTGGAG CCCCCGTGTC GGGGGGCGAG GGNGGGGCGG TGNCCGACGA GTTGANCTTC 60
TNAACATGAG TGTCAACTTC AGGACTTGCT TCCAAGCCCT TCCCTCTGTT GGAAATTGGG 120
TGTGCCATNN CTCCCAAGGN AGGCCCATGT GACTAATAAA AAACTGTGAA CCCTGAAA 178

SEQ ID NO:2725
SEQUENCE LENGTH:337
SEQUENCE TYPE:nucleic acid
TOPOLOGY:linear
CLONE:HUMGS03173
SEQUENCE DESCRIPTION:
GATCCTTTTG AATTTTGCAC AGCCCTAGAT ACAATCCCTT TTGATAAAAG GGTCTTTGCT 60
TCTNATTACA GGAGCACTGT GGAACGTCTG TAAATATGTT TTTATAATTC CATGTATAGT 120
TGGTGTACAC TCAAAACCTG TCCCCGGCAG CCAGTGCTCT CTGTATAGGG CCATAATGGA 180
ATTCTGAAGA AATCTTGGGG AGGGAAGGGG AGTTNGAACA AATGTCTGTT CCCTGGGAGG 240
CCAGTCCAGT GCTCAGACCT TTAGACTCAT TGTAAGTTGC CACTGTCAAC ATGGGACCAA 300
AGGTGTGTGA CTAGTCAATG AAAGTGCGGN CANATTN 337

SEQ ID NO:2726
SEQUENCE LENGTH:180

SEQUENCE TYPE:nucleic acid
TOPOLOGY:linear
CLONE:HUMGS03175
SEQUENCE DESCRIPTION:
GATCCAAAAC ACTCAATCCT CGTAAAAGGG TACAGGTCCT ATTGTATGTT AACACACGTG 60
ATGATGTGAG CCATGTGAGA TGANCAATCA CCAGTTGTTA TGTTCGAATC TCCTTTGTGT 120
TGGGCCGCTT TAATGTCAGG GTTTCACTAA ATAAATGAAT TTTCATATAC TGAACTGAAA 180

SEQ ID NO:2727
SEQUENCE LENGTH:175
SEQUENCE TYPE:nucleic acid
TOPOLOGY:linear
CLONE:HUMGS03176
SEQUENCE DESCRIPTION:
GATCTGAGCC AGGATGGGAG TGAATCCCAG GCGGAGGCCA GAGCTGGCAG CCAGCTCTGC 60
CTTTCCACTG CCGGGAGTGC TGGGGGCCCA GCCTGGCCCC CNGAAGAGAC AGCCAAGTGT 120
CGTCCACATA CTCCTCCCAG AGTGAGCTCT AACCAGGCTC ATTTGCTCTN TCCAN 175

SEQ ID NO:2728
SEQUENCE LENGTH:175
SEQUENCE TYPE:nucleic acid
TOPOLOGY:linear
CLONE:HUMGS03177
SEQUENCE DESCRIPTION:
GATCCTGTTN CCNACCTCCT TCTCCTCCCC TGTACTCCCC AGTGCCTTCC ATTGTCCTGG 60
TGGAGCTGGG GTTTCTCTCC TCCCCAGTCC CACAACACTG CCAAAAATCT GTGTATGTNC 120
CATTGGGTGG GGCAGCCCCA AGCCTCCTGG GGAGGCAGGG CAAAAACAGG TGCCN 175

SEQ ID NO:2729
SEQUENCE LENGTH:174
SEQUENCE TYPE:nucleic acid
TOPOLOGY:linear
CLONE:HUMGS03178
SEQUENCE DESCRIPTION:
GATCAGAGGA AAGATGAGAA AGAGAACAAG CATACCTATC TNACTCCTTC CAGTGGTCTC 60
TGTTCCCTCC GTCTCCTGGA CCACGGCTAG AGTTCCCAGT TTGGCCTTTT GGGGCATTCA 120
TCCCATCTAC TTTATTTTCA TCTGCTTGGG CAGTAATGTA AACAAATTAA AGGN 174

SEQ ID NO:2730
SEQUENCE LENGTH:271
SEQUENCE TYPE:nucleic acid
TOPOLOGY:linear
CLONE:HUMGS03179
SEQUENCE DESCRIPTION:
GATCGTCTGG GAGCCCAGTG TCAAGTCTGC TGGAATGCAG GAAGTAGAAC AGAATCGCCA 60

CAGGACTGTT CTGGGGCCAG GTTCCCTTAA CTCTGTAGCC TGGCAGTCTG ACCCAAAGTT 120
GCCCTCACCC AAAGGTTCTG GCTCTTCCCT CCCTCACTTT TACTTTCCCT TCCCCCATAA 180
GTTGGAGGAT AAAATGGGTA TCAATGNTAA TNTTTCCAGG GNGNCCATGN NNACCAGCGG 240
TTTCTNNCTN TCTCTGTAAT CTGCTATGNA N 271

SEQ ID NO:2731
SEQUENCE LENGTH:178
SEQUENCE TYPE:nucleic acid
TOPOLOGY:linear
CLONE:HUMGS03180
SEQUENCE DESCRIPTION:
GATCTNCGGN CTGTCGTTAG CAGGCTTAAT NAAGAACACT TCTCAACAGT TTCCTTTTTN 60
TNTTCCTTTA TAATTCACTA AAATAAAGCA TCTATTAGTG TCTGATTTAG GAATGTAAAA 120
TAATTCTNTA TTAATGTAAA TAAGATTATC TATTGCAAAA NGATATTTCA ANCCTAAA 178

SEQ ID NO:2732
SEQUENCE LENGTH:172
SEQUENCE TYPE:nucleic acid
TOPOLOGY:linear
CLONE:HUMGS03181
SEQUENCE DESCRIPTION:
GATCTTACCA AAGAGGTCAA CACATGGGAC CTTTGTGTCA CATGAACCAT TTTTTTTCCT 60
CTTCTATTAA GTGTATTTNT GTTTAAGNTA CAGTTCTCTA AGAGAATTAC AATGTTTGTC 120
CCATTTCTAA GGGCTTCTNT TCAACTCTAA TAACAGCATT ATTCACGTTA AA 172

SEQ ID NO:2733
SEQUENCE LENGTH:170
SEQUENCE TYPE:nucleic acid
TOPOLOGY:linear
CLONE:HUMGS03182
SEQUENCE DESCRIPTION:
GATCAGGAAA CGGCAACAAG CCTCCCAGTA CTGACCTGAA AACTTGTGAC AAGAAGAACA 60
CCAACAAGTG CTCCCTGGGC TGAGGACCCT TTNTTGCCTC CCCACCCCGG AAGCTGNACC 120
TGAGGGAGAC AACGGCAGAG GGAGTNAGCA GGGGAGAAAT AGCAGAGGGN 170

SEQ ID NO:2734
SEQUENCE LENGTH:176
SEQUENCE TYPE:nucleic acid
TOPOLOGY:linear
CLONE:HUMGS03183
SEQUENCE DESCRIPTION:
GATCTGGATT TATGTATTTG TNAGATACAT ACGAATTGTT AAAATGGAAT GCAAGTTTTT 60
CAAAAGCCCA GGTCTAAATG TAATGGTTGG TTTATNGTNC TATAACCCCA GCCCATCATT 120
TCCTGTGTAA ATCATAAACA ATAAACAGAA TATACTCGGT GGCCATTTCT AATAAA 176

SEQ ID NO:2735
SEQUENCE LENGTH:163
SEQUENCE TYPE:nucleic acid
TOPOLOGY:linear
CLONE:HUMGS03185
SEQUENCE DESCRIPTION:
GATCCGCCCA CCTNGGCCTC CCAAAGTGCT GGGATTGCAG GCGTGAGCAN CCGCACCCGG 60
CCTCCTGAAT TAACATTTTC TAAAGATGCC TCCGGCGTGC TATCCTGAAA GCTGCCAGAG 120
GTGTCTTGGC TGCATTGGTG GTAATAAAAT TCATACTCAT AAA 163

SEQ ID NO:2736
SEQUENCE LENGTH:162
SEQUENCE TYPE:nucleic acid
TOPOLOGY:linear
CLONE:HUMGS03186
SEQUENCE DESCRIPTION:
GATCTCAACT ATGTNCCACT AAAGGCACAG GAATGGAAAA CTGAGAAAAG ATTTATTGGC 60
CTCACCAATT CCTTTGGTTT TGGTGGTACT AATGCAACAC TTTGTATTGC TGGNCTGTAG 120
AACATATANT TNGTAATNAA ATACTGATTT TNAAATGTTA AA 162

SEQ ID NO:2737
SEQUENCE LENGTH:160
SEQUENCE TYPE:nucleic acid
TOPOLOGY:linear
CLONE:HUMGS03187
SEQUENCE DESCRIPTION:
GATCCTGCCA CCCTGTAGCG CCAGTNTAAT CCCCCAGGAG AACATCTTTG ACACTCTGCA 60
GACTGCTAGT GTTCTGTCTA AAAACCAGAC AAGGAAATAC CCTTCTTTTA TGAGCAGAAG 120
GAAACAAAAA AAAAAANGAG GCCCNTTTNC CTGGANGAAA 160

SEQ ID NO:2738
SEQUENCE LENGTH:159
SEQUENCE TYPE:nucleic acid
TOPOLOGY:linear
CLONE:HUMGS03188
SEQUENCE DESCRIPTION:
GATCTGCCTT ATTGCATATN CCATGCATCA GATAATGGAT GCATCAGATA ATGGNGTTAG 60
ACAAAGCTTC ATTGTGAACA ACCTAATGCA TTTNNGAGAA ACANTCTCAT CACATTTTTC 120
CTAGCCTTNC CTACATTTAA NCTTGCTGTT GCCCAAATN 159

SEQ ID NO:2739
SEQUENCE LENGTH:157
SEQUENCE TYPE:nucleic acid
TOPOLOGY:linear
CLONE:HUMGS03189

SEQUENCE DESCRIPTION:
GATCAGANGT TTCACTTGTT TCTCAGTTAT TGGATATGTA TCTTTGTGTA CATATCTTTG 60
CAAAANTGGA TAAGTACAAA NCTTGATGTA AATNGTACCA ATGANTATGT AAACATACAG 120
TGACANCATT AAACTTAGAA AAGTTTTAAA ACTTAAA 157

SEQ ID NO:2740
SEQUENCE LENGTH:153
SEQUENCE TYPE:nucleic acid
TOPOLOGY:linear
CLONE:HUMGS03190
SEQUENCE DESCRIPTION:
GATCCACTGG AATTAGCCAA TACACTATAT AACAATACTA TTAAAGTATT TTTTCCTGGA 60
ATATAATTGG TATATGTCTT CCACTTTCCA TCATGTATGT AAAATTTCAT AGTAAAACTT 120
CCTGATAGTT TCAATAAAGA AATNATCTGC AAA 153

SEQ ID NO:2741
SEQUENCE LENGTH:151
SEQUENCE TYPE:nucleic acid
TOPOLOGY:linear
CLONE:HUMGS03192
SEQUENCE DESCRIPTION:
GATCTTNAAT GCTTTGGAAA TGCGTGTAAC AGTACTGCAA TAATCACAGC TCTGGGAAAA 60
ACAACGAAAC TTTCCCTTGT GGAGAGGAGG GATTTCCCTG CTCTATATAA GCAACATATT 120
TTNAGACATT AAAATATGGT ATNCNNNNCC N 151

SEQ ID NO:2742
SEQUENCE LENGTH:150
SEQUENCE TYPE:nucleic acid
TOPOLOGY:linear
CLONE:HUMGS03193
SEQUENCE DESCRIPTION:
GATCTTNCTN ATCCAAGCAA TAACGGGACG AGGGAGCCAG GGCCAAGGAC ACTCAGGGAC 60
ACTGTTGGGG CAGCGGCCCC ACATGGTAAT GTTTTNTTTA GAGAGAAGAA AAAACAACTG 120
GCTGTAAACG TAAATNATAT TTNTTGGAAA 150

SEQ ID NO:2743
SEQUENCE LENGTH:150
SEQUENCE TYPE:nucleic acid
TOPOLOGY:linear
CLONE:HUMGS03194
SEQUENCE DESCRIPTION:
GATCGTNTTT TTNCTTGTTA AATAATTAAA ANTCATCTTA AAATTCATAC TTTGACTTTA 60
ATTTAGACAC AAGGAGATGT AATCNCTGTG TAACATCAGA GAAAGCTATA TGGATTATNG 120
TCAGANGTAA ATGTTTCTAA CATTGACAAA 150

SEQ ID NO:2744
SEQUENCE LENGTH:149
SEQUENCE TYPE:nucleic acid
TOPOLOGY:linear
CLONE:HUMGS03195
SEQUENCE DESCRIPTION:
GATCGAGTTG GCCGAGGATG GATGATTNTG NGCAGCAGAA GCCGTTGCAG CCCCACGTTG 60
TGCTCTAGGC AGGGACCTTT GGCCCCTTTG GGGAGGGAGA GACAGACGGG CGGTTTGACT 120
TGGACACAAA GAAAGCCTTG GTTTCTAAA 149

SEQ ID NO:2745
SEQUENCE LENGTH:145
SEQUENCE TYPE:nucleic acid
TOPOLOGY:linear
CLONE:HUMGS03196
SEQUENCE DESCRIPTION:
GATCCCAGGT CTGGGGATGG GGGACACCTT NGGCCACAGG ATACTGGTTG CTTCAGGGGT 60
ACCCATGCCC NCTGCCCTNG CCTGGAATCA GTGTTACTGC ATCTGATTAA ATGTCTCCAG 120
AAATAAAGAA TAATTCTGCC AGAAA 145

SEQ ID NO:2746
SEQUENCE LENGTH:143
SEQUENCE TYPE:nucleic acid
TOPOLOGY:linear
CLONE:HUMGS03197
SEQUENCE DESCRIPTION:
GATCACTCNT AGAGTNGCCA TTCTGCANCT TCCTAAAAAT ATTTTAAAAA TACATTTATT 60
TTACTAAATA CTGACTACAT TTNTNTGTTA ATATTGAGCT AAATGTTAAA AAATGGCCAG 120
ATTAAAAGAT ATCAATTTGT AAA 143

SEQ ID NO:2747
SEQUENCE LENGTH:142
SEQUENCE TYPE:nucleic acid
TOPOLOGY:linear
CLONE:HUMGS03198
SEQUENCE DESCRIPTION:
GATCCATAGT GTGCATGGGC AGACACATTT TGCCTCTATG TCTCTTAAAA TTTTAATTAA 60
AAATACTCTT TCCAGTAATC CTAATTTGCA CGAAGATATA ATGTCCACAT TACGTGCCTT 120
GCCTTGAAAT CTAAAAAACA AA 142

SEQ ID NO:2748
SEQUENCE LENGTH:142
SEQUENCE TYPE:nucleic acid
TOPOLOGY:linear
CLONE:HUMGS03200

SEQUENCE DESCRIPTION:
GATCTCACAG GACTGAGAAC TCGTTCACCT CCAAGCATTT CATGAAAAAG CTGCTTCTTA 60
TTAATCATAC AAACTCTCAC CATGATGTGA AGAGTTTCAC AAATCTTTCA AAATAAAAAG 120
TAATGACTTA GAAACTGCCA AA 142

SEQ ID NO:2749
SEQUENCE LENGTH:141
SEQUENCE TYPE:nucleic acid
TOPOLOGY:linear
CLONE:HUMGS03201
SEQUENCE DESCRIPTION:
GATCTTTTCA TTTCTTACTA GCTTTTAAGA AATNAAATAC TTGCCTGAGA TAGAAATACT 60
TTATTTTTGT AACTTTAAGG TCTAAATNAC TAAACTTCAA AGTAAGATTT TNTCAGAATA 120
AATTGAGACC ATTAATCTAA A 141

SEQ ID NO:2750
SEQUENCE LENGTH:140
SEQUENCE TYPE:nucleic acid
TOPOLOGY:linear
CLONE:HUMGS03202
SEQUENCE DESCRIPTION:
GATCAGAAAA AGAAAGAAGC CAAAGAGAAA GGTACCTGGG TTCAACTANA ACGCCACCTT 60
GAGCTGCTGG AACCTATTCC CTATGAATTC ATGGCATAAT AGGTGTTAAA AAAAAAAANA 120
AAGGACCTCT GGGNTACAAA 140

SEQ ID NO:2751
SEQUENCE LENGTH:135
SEQUENCE TYPE:nucleic acid
TOPOLOGY:linear
CLONE:HUMGS03204
SEQUENCE DESCRIPTION:
GATCCTATTT TCNTACAACC TTTCAGTGAC ATCGTTCAGG NTTCTTTCTT GGCCATTTAA 60
AAAAACAAAT TTTTTTTTNC TCACTTGTAA GTCACCGCCA GTNCCTAAGT NAGGCTAACG 120
GNGACTTTGA CAGGN 135

SEQ ID NO:2752
SEQUENCE LENGTH:134
SEQUENCE TYPE:nucleic acid
TOPOLOGY:linear
CLONE:HUMGS03205
SEQUENCE DESCRIPTION:
GATCAGTAAT AGATNTAGAT ATACTATTTT NATATTGTAA GCAGTTACNT ACTCAGGATT 60
AGNATTCGAA TTCATTTGAT TTAATATGTA ATAGGTAATA TTAAAANTAA AGATTTTATG 120
CTTTGAAACT CAAA 134

SEQ ID NO:2753
SEQUENCE LENGTH:133
SEQUENCE TYPE:nucleic acid
TOPOLOGY:linear
CLONE:HUMGS03206
SEQUENCE DESCRIPTION:
GATCAGACGT CGCTGAGGGG CTGTNCACCA CCATCCTCGT TCTCCAGGGT CAAGGAAGTG 60
TTTTAACATG TCGTGTTTTC GACTTGACCT TGTGGTATTT TNCTNGGCCT AGTTGTGTGC 120
CATGGATATT AAA 133

SEQ ID NO:2754
SEQUENCE LENGTH:141
SEQUENCE TYPE:nucleic acid
TOPOLOGY:linear
CLONE:HUMGS03207
SEQUENCE DESCRIPTION:
GATCACTGTA GTTTAGTTCT GTTGACCTGT GCACCTACCC CTTGGAAATG TCTGCTGGTA 60
TTTCTAATTC CACAGGTCAT NAGATGCCTG CTTGATAATA TATAAACAAT AAAAACAACT 120
TTCACTTCTT CCTATTGTAA A 141

SEQ ID NO:2755
SEQUENCE LENGTH:132
SEQUENCE TYPE:nucleic acid
TOPOLOGY:linear
CLONE:HUMGS03208
SEQUENCE DESCRIPTION:
GATCGTTATA TTTNACATAA TATAAACAAA ATNACTAATG CATCTATATN CTTGGTTTCC 60
TTAAACTACA ATNAACCACA GAATGGGTGA AAATGCAGGC ATGCACGCTT GGCGTAATCA 120
TGGATGNNGC TN 132

SEQ ID NO:2756
SEQUENCE LENGTH:131
SEQUENCE TYPE:nucleic acid
TOPOLOGY:linear
CLONE:HUMGS03212
SEQUENCE DESCRIPTION:
GATCCGNCCG CCTCAGCCTC CCTATATGTT TTAAAATATA ANTTTATTCT TATAAAANTA 60
AATTNAANCT AGATTGAGGT AGTAATTACA ACAGTCCAGC ACATTATGGA TAATTAATAT 120
TNATATTTAT N 131

SEQ ID NO:2757
SEQUENCE LENGTH:136
SEQUENCE TYPE:nucleic acid
TOPOLOGY:linear
CLONE:HUMGS03213

SEQUENCE DESCRIPTION:
GATCTGATAT TTTAAGANTT TGGAGACCAT TTCCTGTGCC AGANTCACTG CTCTATTCCA 60
TACCGTGCCA TGGAGGCTGT TTTAGAAGTG GTTGGATAAC ATGTTAAATA AAATATTAAG 120
TGTAAAAAAA AATAAA 136

SEQ ID NO:2758
SEQUENCE LENGTH:129
SEQUENCE TYPE:nucleic acid
TOPOLOGY:linear
CLONE:HUMGS03214
SEQUENCE DESCRIPTION:
GATCCACCCA CCTTGGCCTC CCAAAGTCCT GGGATTACAG GTGGGAACCA CAGCACCTNA 60
CCCAGAATAT AAAGACACTT TTNTAATGTG CAATGAAAAA TATGTAAATA AANTATTTTT 120
ATCTNTAAA 129

SEQ ID NO:2759
SEQUENCE LENGTH:129
SEQUENCE TYPE:nucleic acid
TOPOLOGY:linear
CLONE:HUMGS03215
SEQUENCE DESCRIPTION:
GATCCACCGT ACCAGGNAGC AGAAATATTT CTACAAAGGG GGCCTAGTTT GGAATGAGAA 60
CANNTCTGAC GGCCGGTATG TGAAGGAGAA CTGCAAACCT CTGAAGAGTT ACATGCTCCA 120
AGACTCCCN 129

SEQ ID NO:2760
SEQUENCE LENGTH:408
SEQUENCE TYPE:nucleic acid
TOPOLOGY:linear
CLONE:HUMGS03216
SEQUENCE DESCRIPTION:
GATCATGTTG GGATTAACTT GCCTTTTTCC CCAAAAANNA AACTCTCAGG CAAGCATTTC 60
TTTAAAGCTA TTAAGGGNGT ATATACTTGA GTACTTATTG AAATGGACAG TAATAAGCAA 120
ATGTTCTTAT AATGCTACCT GATTTCTATG AAATGTGTTT GACAAGCCAA AATTCNNGGG 180
TGTAGANATC TGGAAAGTTC ATTTCCTGGG ATTCACTTCT CCAGGGATTT TTTAAAGTTA 240
ATTTGGGAAA TTANCAGCAG TTCACTTTAT TGTGAGTCTT TGCNACATTT GACTGAATTT 300
AGCTGTCATT TGTNCATTTA AAGCAGCTGT TTTGGGGNCT GTGAGAGTCC ATGTATTATA 360
TCCAAGCCCA CCAGGGCTTN CCCTAAGGAN TTGCCATNGT AATACCCN 408

SEQ ID NO:2761
SEQUENCE LENGTH:128
SEQUENCE TYPE:nucleic acid
TOPOLOGY:linear
CLONE:HUMGS03217
SEQUENCE DESCRIPTION:

GATCTAGGAA AGAAGGCAAC TGAAAAACCT GAAAGAAAAA AAATNAAAGA TTAAAAGTAT 60
ACGATATTCT ATTTTGATTT AGCAATTACT TGTTTNCNAG TGTNCTGTNA ATTTTTGGTN 120
ACTTTAAN 128

SEQ ID NO:2762
SEQUENCE LENGTH:258
SEQUENCE TYPE:nucleic acid
TOPOLOGY:linear
CLONE:HUMGS03218
SEQUENCE DESCRIPTION:
GATCGGCAGG CTGAGGACCA GGTCCTGAGG AAGCTGGTGG ATTTGGTCAA CCAGAGAGAT 60
GCCCTCATCC GCTTCCAGGA GGAGCGCAGG CTCAGCGAGC TGGCCTTGGG GACAGGGGCC 120
CAGGGCTAGA CGAGGGTGGG CCGTCTGCTT TCGTTCCCAC AAAGAAAGCA CCTNACCCCA 180
GCACAGTGCC ACCCNTGTTC ATCTGGGCTG NCTGGCAGAG AGCCTTGCTG TTTACAATTA 240
AAATGTTTCT GCCACAAA 258

SEQ ID NO:2763
SEQUENCE LENGTH:128
SEQUENCE TYPE:nucleic acid
TOPOLOGY:linear
CLONE:HUMGS03219
SEQUENCE DESCRIPTION:
GATCCTTGTT GGTCTAGCTA AATACTGTTA GGGGAGTGTG CCCCATCTCA TCATTTCGAA 60
GATAGCAGAG TCATAGTTGG GCACCCAGTG ATTGGGTTCA AAAATAAAGC TGGTCTGCCT 120
CTTCTAAA 128

SEQ ID NO:2764
SEQUENCE LENGTH:126
SEQUENCE TYPE:nucleic acid
TOPOLOGY:linear
CLONE:HUMGS03220
SEQUENCE DESCRIPTION:
GATCTNCCGC TCCCTCCCTG AAGGGCATGG GGCCAGGGNA GTCTGGACAG CCGCCCCCAG 60
CCTGCTGACC CCCAGCTTTC TACAGACACC ANATTTGTAA ATAAAGTTGG GGAATGGACA 120
GCCAAA 126

SEQ ID NO:2765
SEQUENCE LENGTH:126
SEQUENCE TYPE:nucleic acid
TOPOLOGY:linear
CLONE:HUMGS03221
SEQUENCE DESCRIPTION:
GATCTTTTTA GGCCTTCATT TTATGTTTTT CCTTAACTGT TATATTATNA TTGTAACATA 60
GATTATACTA CTACTAATTN NTGGATGTNT CAAAAGGTCA AGAAGTAAAA NATGTTAGAA 120
AGCAAA 126

SEQ ID NO:2766
SEQUENCE LENGTH:125
SEQUENCE TYPE:nucleic acid
TOPOLOGY:linear
CLONE:HUMGS03222
SEQUENCE DESCRIPTION:
GATCTCTGCA CAGTTTCTTG TTTAGTCTTA TGCCTTTTAG TTGAAAAACA AATTGTATTT 60
TTTTCCTNCT AAGTTTCATG GCCCTAGAGA AGAAGCCAAT NTNNNTAAAG ATGTTATCCC 120
NGAAA 125

SEQ ID NO:2767
SEQUENCE LENGTH:124
SEQUENCE TYPE:nucleic acid
TOPOLOGY:linear
CLONE:HUMGS03223
SEQUENCE DESCRIPTION:
GATCCTGGTT TCAATGACGG TTGGAAATAG AAATTNCCAG AGAAGAGAGT ATTGGGTAGA 60
TATTTTTCCT GAATACAAAG TGATGTGTTT AAATACTGCA ATTAAAGTGA TACTGAAACA 120
CAAA 124

SEQ ID NO:2768
SEQUENCE LENGTH:131
SEQUENCE TYPE:nucleic acid
TOPOLOGY:linear
CLONE:HUMGS03224
SEQUENCE DESCRIPTION:
GATCACTTAG CAACATGCTT GGNAATTTGG NATCTNTAAA GGTAGGAGAG TGGTGAACAG 60
ATAATCTATG CATATATNAC TAGTGCCAAG ACATAAAGCG GGGGAAAATA TATTTTANCC 120
CAAACATTAA A 131

SEQ ID NO:2769
SEQUENCE LENGTH:123
SEQUENCE TYPE:nucleic acid
TOPOLOGY:linear
CLONE:HUMGS03225
SEQUENCE DESCRIPTION:
GATCAATTAC CTAACTGTTC CTTGGTCTAT TTATGTATAA GAATGCTTTT TAAAGCACAT 60
GTCTCATTTT AAATNACGCA CAAACTGAAG ATGTTAATAA AATTTAAGAG TAATACAATG 120
AAA 123

SEQ ID NO:2770
SEQUENCE LENGTH:388
SEQUENCE TYPE:nucleic acid
TOPOLOGY:linear

CLONE:HUMGS03227
SEQUENCE DESCRIPTION:
GATCCATTTG TCTGGCAGGA GGTTTTTGAT GATAAAGCAA CGCTTGCGCC AAGGCACAAT 60
AGTTGAAGNA TGGAAAGACA GCGCATATCC TGAGGAACTC AGTAGAGTCA CAGCATCTGG 120
CTTCCCTGTA ATCCTTTCTN CTCCTNGGTA CTTAGATTNG ATTAGCTATG GACAAGATTG 180
GAGGAAATAC TATAAAGTGG AACCTCTTGA TTTTGGCGGT ACTCAGAAAC AGAAACAACT 240
TTTCATTGGT GGAGAAGCTT GTCTATGGGG AGANNNTGTG GATGCAACTA ACCTCACTCC 300
AAGATTTATG GCCTCGGGCA AGTGCTGTTG GTGAGAGACT CTGGAGGTTC CAAAGATGTC 360
AGAGATATGN GATGACGCCT ATNACAGN 388

SEQ ID NO:2771
SEQUENCE LENGTH:219
SEQUENCE TYPE:nucleic acid
TOPOLOGY:linear
CLONE:HUMGS03229
SEQUENCE DESCRIPTION:
GATCGATGGA CCGTAAATAA GCTGCCATTA ACACATCTGG TTACTGCTGT AACATGACTA 60
ATAAAACCGA ACGCCTGTTC CCCTTACCCG TGTGGGGGAC ACGCAGATGA GTGAATTGGA 120
ATGTCCAGCA GAGTTACCCT CCCAATTATA TGTTCATTTT GTATATTTTT TGGTCGGGGG 180
AAAAATTGAC CTGCAGTAAA AAAACCTTTG ACCATTAAA 219

SEQ ID NO:2772
SEQUENCE LENGTH:120
SEQUENCE TYPE:nucleic acid
TOPOLOGY:linear
CLONE:HUMGS03230
SEQUENCE DESCRIPTION:
GATCAGCACA GGAAGAGCCT TCCTAAGCAT GTGGTCCCAG GGAAGGGTGA AAAGTAGGAG 60
TTATGAACGT AATCCACCTA CTACAATGTT GAATTAAAGT TATAATCTCA TAACTTCAAA 120

SEQ ID NO:2773
SEQUENCE LENGTH:120
SEQUENCE TYPE:nucleic acid
TOPOLOGY:linear
CLONE:HUMGS03231
SEQUENCE DESCRIPTION:
GATCCGCCCG CCTCGGCCTC CCAAAGTGCT TGGATTACAG GCATGAGCCA CCGCACCCAG 60
CAAGAGTTAT TTNNTTAACT TGAAATTTTC TACTAGCCCT GGTGAACTTC TGTGCTTAAA 120

SEQ ID NO:2774
SEQUENCE LENGTH:119
SEQUENCE TYPE:nucleic acid
TOPOLOGY:linear
CLONE:HUMGS03233
SEQUENCE DESCRIPTION:

GATCCAAGGT GATACAGCTG GTAACTGATG GAGATGTTGG TAGTGAAATN CATGTACTTT 60
TAGTNCTTTC TATGGAATCT AGAGGNAGAA TACATGTACA AGAATATCGA CTACCGTCN 119

SEQ ID NO:2775
SEQUENCE LENGTH:117
SEQUENCE TYPE:nucleic acid
TOPOLOGY:linear
CLONE:HUMGS03234
SEQUENCE DESCRIPTION:
GATCCCAGCT GTGTGTCACC AGCAGCTTTC CCAGCTTCTC TGTGAGGGTC ACTGCTGCCC 60
ACTGCAGGGT CCCTGAGGTG AAGTAAACGC CGGCGCTGGG CTTGGCCAGN CGGCAAA 117

SEQ ID NO:2776
SEQUENCE LENGTH:115
SEQUENCE TYPE:nucleic acid
TOPOLOGY:linear
CLONE:HUMGS03235
SEQUENCE DESCRIPTION:
GATCATTCCC ATTTCATAGG TTTTTATTGT GGAATAGTCA ACTTAATATA GATTCACTTT 60
CAATTTGCAT GTATAANGTA ATACTCAATA AAATTTTTTC CATTNATTTT NCAAA 115

SEQ ID NO:2777
SEQUENCE LENGTH:217
SEQUENCE TYPE:nucleic acid
TOPOLOGY:linear
CLONE:HUMGS03236
SEQUENCE DESCRIPTION:
GATCCTGGGG TTGGTCTGCT TTGTGTATGG TACTTGAAAC CACGCTGTAA TTATTGTCCT 60
GTTGCCAAAC AAAAGCCAGT CATGTAACTC TAGAAGCAGT GACTGGTGGG GCTTTCTGAC 120
AGTTCCATGC TGATGTATCA GGCCATCTGT GTCATGCTTA TGTATTATGG CAAGAAGAGG 180
AAAACTGGAT TAATAAATAC GTTTTNNNNN NGTTAAA 217

SEQ ID NO:2778
SEQUENCE LENGTH:114
SEQUENCE TYPE:nucleic acid
TOPOLOGY:linear
CLONE:HUMGS03237
SEQUENCE DESCRIPTION:
GATCCTNAGC AACGAACACC TGCACTTAGA AAAAGTGGAC AGCTTCTNCC AACCACACCC 60
TACCCATGGN ACTGTATGCA ATTAACTCCT GGAAACGCCC CGTAAATNCN AGTN 114

SEQ ID NO:2779
SEQUENCE LENGTH:113
SEQUENCE TYPE:nucleic acid
TOPOLOGY:linear

CLONE:HUMGS03239
SEQUENCE DESCRIPTION:
GATCCAAAAT TACCTTAAAT GGAGTTAAGT AGGCTTTGCA CTAAATGGAT ATAAAAGAGG 60
CTGTCTGGNC TNCTATGAAA TNATGATTAA AATCCTTTGT GTTGTCTTTT AAA 113

SEQ ID NO:2780
SEQUENCE LENGTH:359
SEQUENCE TYPE:nucleic acid
TOPOLOGY:linear
CLONE:HUMGS03240
SEQUENCE DESCRIPTION:
GATCCTTAAA GAAGGTGTGG GGTCTTTCCC AACCTGAGGA TTTCTGAAAG GTTCACAGGT 60
TCAATATTTA ATGCTTCAGA AGCATGTGAG GTTCCCAACA CTGTCAGCAA AAACCTTAGG 120
AGAAAACTTA AAANTATATG ATTACATGCG CAATACACAG CTACAGACAC ACATTCTGTT 180
GACAAGGGAA AACCTTCAAA GCATGTTTNT TTCCCTCACC ACAGCAGAGC ATGCAGTACT 240
AANGCGATAT ATTTNTGATT CCCCATGTAA TTCTTCGGNG GNNGGNCAGT GCAGTNCTCT 300
TTCGGAAGCT TAAGATGGCC NTGNGGCCTG NCCNNNNGGN CATATGCCCC TGAAGGGCN 359

SEQ ID NO:2781
SEQUENCE LENGTH:191
SEQUENCE TYPE:nucleic acid
TOPOLOGY:linear
CLONE:HUMGS03241
SEQUENCE DESCRIPTION:
GATCTAGGGC AGGCTGTCTT CCAGTCCATG TGTTCTCGGT CGCCGTAGAC AGCGCTCTGG 60
CTACCACCGT GAGGCTACTT GAACTGTCAG GGGCATCTGC CTAAACCAGA ATCTTTTGTC 120
AGAAACCTTA ACCCAACAAA ACAAATCTTG AGTAGCTCAT GCCCGGCTCT TAGGAATTTT 180
GTCTGTTTAA A 191

SEQ ID NO:2782
SEQUENCE LENGTH:111
SEQUENCE TYPE:nucleic acid
TOPOLOGY:linear
CLONE:HUMGS03242
SEQUENCE DESCRIPTION:
GATCACTCAA CAGCACTGTN ATGTATTATT TNNAATGAGG TGCCTTTTTT AACTGACCAA 60
ATGCTGCCTT GTTTGGCCCC TAANTCAATA AAATATGTTA AAATTTGTAA A 111

SEQ ID NO:2783
SEQUENCE LENGTH:109
SEQUENCE TYPE:nucleic acid
TOPOLOGY:linear
CLONE:HUMGS03243
SEQUENCE DESCRIPTION:
GATCTAAGAA ATAGAATTAG GGAGAGTTTA CACTTACTTA CCTATATAAA AGCAGATTTT 60

CNTGGAAGAA ACCCTTTTTT TTTTTTTTTT TAAAANAGNA NCANCCAGN 109

SEQ ID NO:2784
SEQUENCE LENGTH:340
SEQUENCE TYPE:nucleic acid
TOPOLOGY:linear
CLONE:HUMGS03244
SEQUENCE DESCRIPTION:
GATCCAGAAG CTGGTGGACG TCACCACGGC ACAGGTGTAA CNTCCATGTN CCGTGTGAGC 60
AGAGTCCCTA CCAACGGGCA GGTCTGCATC CGGGGAGAAT GCAGCTGCTT CTGGCGACAA 120
TCCTGCTAGT AAACACTGGT CTTCGGTGAG CAACGAACAC TCGCCTGGGN TGGGAAACTG 180
CATGCCCACT TTNTGGGAGG GGTTAGTGCA GGTGCCGTGG ACAAAGGACA ACATTTCTNT 240
GGGGCTTTTT AACTTTTATT CCTAAGACTC TAAAGGCGTT GATTTCAACC CTCCTTCACT 300
CTGGCTTCTT CAGGNAACCC ACGTGGTCTC CTGTNAGNTN 340

SEQ ID NO:2785
SEQUENCE LENGTH:108
SEQUENCE TYPE:nucleic acid
TOPOLOGY:linear
CLONE:HUMGS03245
SEQUENCE DESCRIPTION:
GATCTTTAAG ACAACAAGTT AGGTTCCTAC TGGAGTTACC TGCCAGANTG GCCTCTTAAT 60
TAACTCAGGT AATGAAGAGC TAACTGTGTT ATAATCATCT NCCNNNTN 108

SEQ ID NO:2786
SEQUENCE LENGTH:108
SEQUENCE TYPE:nucleic acid
TOPOLOGY:linear
CLONE:HUMGS03247
SEQUENCE DESCRIPTION:
GATCTGCTAA AAATGGCAAT TGTAGAATNA TGTTGTATGT AATGTACATA TGGAGAAAGA 60
ATGTATTTTN TCCATTTCCT TAATAAAATG TTATATATGG NTGCTAAA 108

SEQ ID NO:2787
SEQUENCE LENGTH:107
SEQUENCE TYPE:nucleic acid
TOPOLOGY:linear
CLONE:HUMGS03248
SEQUENCE DESCRIPTION:
GATCTCTGAG GCTNCGGTCA CCACCCACAA GTTGTGGCCT TGGATACCAC ACTNCAGTTG 60
GGTGGTGATG GGGTNGTGCC TTCTAACTTT TNCCNTATCT TTACCGN 107

SEQ ID NO:2788
SEQUENCE LENGTH:125
SEQUENCE TYPE:nucleic acid

TOPOLOGY:linear
CLONE:HUMGS03249
SEQUENCE DESCRIPTION:
GATCAGTAAG GATGACTGGT GTTTTCTGAC TTTNACAAAA TTACTTGTTT GTTTTCATTA 60
AAAAAAAAGC ATAANCTATG TTTCTNATTA CTATTAGAAT TATTAAATTT CAAAATGAAT 120
CCAAA 125

SEQ ID NO:2789
SEQUENCE LENGTH:104
SEQUENCE TYPE:nucleic acid
TOPOLOGY:linear
CLONE:HUMGS03251
SEQUENCE DESCRIPTION:
GATCGATTCT NTGACACAAA CCCCACCAAT TGTNAATGCA AGTTTTTATT TGGCTGTATA 60
TACAATTTAA GCTATTAAAA TTTGTACAAT ATTTACAAAT NAAA 104

SEQ ID NO:2790
SEQUENCE LENGTH:104
SEQUENCE TYPE:nucleic acid
TOPOLOGY:linear
CLONE:HUMGS03252
SEQUENCE DESCRIPTION:
GATCATTACA TCTNAGATGT ACGTNGGTTT AGTCGGAAAG GTGGGACGAT TTGAAGGGGA 60
GGNACTTTCA GGTCATAGGC GGATTAAAAN ATGTCCTGAT TAAA 104

SEQ ID NO:2791
SEQUENCE LENGTH:104
SEQUENCE TYPE:nucleic acid
TOPOLOGY:linear
CLONE:HUMGS03253
SEQUENCE DESCRIPTION:
GATCCAAGCT ATGANATANC CAACAGTATT CAAGANGCAA CCAGCACCAT CATGTGATAA 60
TGGTACTATG GCATATATGC AACATTAAAN TTTTAANTTA GAAA 104

SEQ ID NO:2792
SEQUENCE LENGTH:132
SEQUENCE TYPE:nucleic acid
TOPOLOGY:linear
CLONE:HUMGS03255
SEQUENCE DESCRIPTION:
GATCCAGTCT TAATTTGAGC ATGAGAGCAA AATTTAGTCA TCTACACAAG AAGCAAAAGC 60
AAGGAATAGT TGTTGGGTTT TNGTTTTTTG GTTGTTGTNT TTTTTTGTTN GTTAGGNANG 120
ANGTGTTCCC GN 132

SEQ ID NO:2793

SEQUENCE LENGTH:102
SEQUENCE TYPE:nucleic acid
TOPOLOGY:linear
CLONE:HUMGS03257
SEQUENCE DESCRIPTION:
GATCCAGGTG GTGTTACATG TCCATTTCAT GTTTTGGGGG CTTTTAGCCC NACAAAACAC 60
CTTCAGTAGA GCCTTGATTA AAAGGAAACC TGNAGACTCA AA 102

SEQ ID NO:2794
SEQUENCE LENGTH:100
SEQUENCE TYPE:nucleic acid
TOPOLOGY:linear
CLONE:HUMGS03258
SEQUENCE DESCRIPTION:
GATCTTCCCC TAGGGGGCCC TCTTACTCCT NCCTCTCTCC TCCTCCTTCC CCATTGCTGT 60
AAATATTTNA ACGAAATGGA AAAGAAAAAA AAAAGACAAA 100

SEQ ID NO:2795
SEQUENCE LENGTH:100
SEQUENCE TYPE:nucleic acid
TOPOLOGY:linear
CLONE:HUMGS03259
SEQUENCE DESCRIPTION:
GATCACACAT CAAGACTATC TACAAAANTT TATTATATAT TTACAGNAGA AAAGCATGCA 60
TATCATTAAA CAAATAAAAT ACTTTTTATC ACAACACAAA 100

SEQ ID NO:2796
SEQUENCE LENGTH:98
SEQUENCE TYPE:nucleic acid
TOPOLOGY:linear
CLONE:HUMGS03261
SEQUENCE DESCRIPTION:
GATCTAAATA CAAAGGATAT ACAGTCTTGA NTCTAAAATA ATTTGCTAAC TATTTTGATT 60
CTTCAGAGAG AACTACTAAT AAAAATCTAA AAGGTAAA 98

SEQ ID NO:2797
SEQUENCE LENGTH:97
SEQUENCE TYPE:nucleic acid
TOPOLOGY:linear
CLONE:HUMGS03263
SEQUENCE DESCRIPTION:
GATCGTNCCT ATAAATAGTA GGACCAATGT TGTGATTAAC ATCATCAGGC TTGGAATGAA 60
TTCTCTCTAA AAATAAAATN ATGTATGATT NGTTAAA 97

SEQ ID NO:2798

SEQUENCE LENGTH:96
SEQUENCE TYPE:nucleic acid
TOPOLOGY:linear
CLONE:HUMGS03264
SEQUENCE DESCRIPTION:
GATCACTAAA GTATATCTTT AATTCTGGGA GAAATGAGAT AAAAGATGTA CTTNTGACCA 60
TTGTAACANT AGCACAANTA AAGCACTTGT GCCAAA 96

SEQ ID NO:2799
SEQUENCE LENGTH:93
SEQUENCE TYPE:nucleic acid
TOPOLOGY:linear
CLONE:HUMGS03267
SEQUENCE DESCRIPTION:
GATCTGCTGT CTCCGTCCCT CCTGGGTCCC AGCAGCTGTC CTGCAGCTGT CAGCAGGAGG 60
GCCTGGCTTT AATAAAGAGT GGACAAACTG AAA 93

SEQ ID NO:2800
SEQUENCE LENGTH:93
SEQUENCE TYPE:nucleic acid
TOPOLOGY:linear
CLONE:HUMGS03268
SEQUENCE DESCRIPTION:
GATCAAGTTC GAGGAGGCAG TCATCGCTCG AAACCGGGCC CTGGAGGGCC GCTNACCCTG 60
CCGGANATAA AGNATACAGA GAGCCCCTCC AAA 93

SEQ ID NO:2801
SEQUENCE LENGTH:89
SEQUENCE TYPE:nucleic acid
TOPOLOGY:linear
CLONE:HUMGS03271
SEQUENCE DESCRIPTION:
GATCCATTCT TGTATTGATA GATATTTGAA TGTTTCCAGT TTTTCCTATT ATGAATAAAA 60
CTGCTATGAA CATTCTTGTA TAAATCAAA 89

SEQ ID NO:2802
SEQUENCE LENGTH:95
SEQUENCE TYPE:nucleic acid
TOPOLOGY:linear
CLONE:HUMGS03274
SEQUENCE DESCRIPTION:
GATCGACAGC GTCCTGGCCT AGGGTGCCGG GGCCTGAAGG TCAGGGTCAC CCAAGCAACA 60
AAGTCCCGAG CAATGAAGTC ATCCACTCCT GCAAA 95

SEQ ID NO:2803

SEQUENCE LENGTH:84
SEQUENCE TYPE:nucleic acid
TOPOLOGY:linear
CLONE:HUMGS03279
SEQUENCE DESCRIPTION:
GATCTCTACA GAGAGAAGTG GAAAATGCTG TATCAAGGGT GGGCTTAGCT GTGCCTTTCC 60
AATAAAGATG TGAGAAGCTT CAAA 84

SEQ ID NO:2804
SEQUENCE LENGTH:79
SEQUENCE TYPE:nucleic acid
TOPOLOGY:linear
CLONE:HUMGS03281
SEQUENCE DESCRIPTION:
GATCTTTGCC CCTGGTATGC ATTTNTGGAC TCCACTGAAT CCTGNGAAAA AAAATTAAAC 60
TTCCTTCTTA CTTGCCAAA 79

SEQ ID NO:2805
SEQUENCE LENGTH:79
SEQUENCE TYPE:nucleic acid
TOPOLOGY:linear
CLONE:HUMGS03282
SEQUENCE DESCRIPTION:
GATCCTGTCA GTTCCATNAG CTATTCCTCT TTGGTTTGGC TTTTTGATAT NATTAAAATA 60
ATNTTTAATT CCTTTTAAA 79

SEQ ID NO:2806
SEQUENCE LENGTH:77
SEQUENCE TYPE:nucleic acid
TOPOLOGY:linear
CLONE:HUMGS03283
SEQUENCE DESCRIPTION:
GATCAATTTT TATAAAAATC GAGACAGTTC TGTGGTTAAA TCTACAAATT AAAGGGAAAT 60
TAGAAGTTGG CGTGAAA 77

SEQ ID NO:2807
SEQUENCE LENGTH:77
SEQUENCE TYPE:nucleic acid
TOPOLOGY:linear
CLONE:HUMGS03284
SEQUENCE DESCRIPTION:
GATCTATTTT ATGCATATTG TTTATAAAGA CACATTTACA ATTTACTTTT AATATTAAAA 60
ATNACCATAT TATGAAA 77

SEQ ID NO:2808

SEQUENCE LENGTH:75
SEQUENCE TYPE:nucleic acid
TOPOLOGY:linear
CLONE:HUMGS03286
SEQUENCE DESCRIPTION:
GATCTTCAGT AAGTCTCTCA GGCTCTCTGA GCTTGTTCAT CCTTTGTTTT GAAAAAATTA 60
CTCAACCAAT CCAAA 75

SEQ ID NO:2809
SEQUENCE LENGTH:74
SEQUENCE TYPE:nucleic acid
TOPOLOGY:linear
CLONE:HUMGS03289
SEQUENCE DESCRIPTION:
GATCCCTTCA CAGCAGTAAA CCAGCTCTTT GGAACCGATT CCATCACCCC AATAAAGGAG 60
CTCTTCACAG CAAA 74

SEQ ID NO:2810
SEQUENCE LENGTH:73
SEQUENCE TYPE:nucleic acid
TOPOLOGY:linear
CLONE:HUMGS03294
SEQUENCE DESCRIPTION:
GATCTGTTTC TTTTTAATT TACACTTTTN CTTTCCGGTT TGCCGGAATA AACAGGACCT 60
TTGACATTTG AAA 73

SEQ ID NO:2811
SEQUENCE LENGTH:66
SEQUENCE TYPE:nucleic acid
TOPOLOGY:linear
CLONE:HUMGS03296
SEQUENCE DESCRIPTION:
GATCCATTCT AAGACTTATT GGCTGTTTGG AAGTGATAGC AAATAAAANC CACCTTGAAC 60
TGGAAA 66

SEQ ID NO:2812
SEQUENCE LENGTH:67
SEQUENCE TYPE:nucleic acid
TOPOLOGY:linear
CLONE:HUMGS03297
SEQUENCE DESCRIPTION:
GATCCGAGGG GGAGGGGTGG GAAGGGGAGA AAATNAAATA AAAGATAATT ACTGAACACG 60
CCGNAAA 67

SEQ ID NO:2813

SEQUENCE LENGTH:63
SEQUENCE TYPE:nucleic acid
TOPOLOGY:linear
CLONE:HUMGS03299
SEQUENCE DESCRIPTION:
GATCATTGAA TTTAAGGCCC ACCCCAAGTC CAGANTGACC TCGCAAGACC CTTAACTCAC 60
AAA 63

SEQ ID NO:2814
SEQUENCE LENGTH:62
SEQUENCE TYPE:nucleic acid
TOPOLOGY:linear
CLONE:HUMGS03300
SEQUENCE DESCRIPTION:
GATCTTTTGT ACTTNTNATA ACCTAATGTC AGACTAAGAA AAATAAAATA TCTGAGAGTA 60
AA 62

SEQ ID NO:2815
SEQUENCE LENGTH:60
SEQUENCE TYPE:nucleic acid
TOPOLOGY:linear
CLONE:HUMGS03304
SEQUENCE DESCRIPTION:
GATCCCTTCG AGACTAANTC TGTTACCAGT CATGAAACAT TAAAACCTAC AAGCCTTAAA 60

SEQ ID NO:2816
SEQUENCE LENGTH:68
SEQUENCE TYPE:nucleic acid
TOPOLOGY:linear
CLONE:HUMGS03305
SEQUENCE DESCRIPTION:
GATCTTTGTC TTTTGTCATG ACAAAGCATT TATTTAATAA AGTTATGCAN TCAGTTAAAA 60
AAANCAAA 68

SEQ ID NO:2817
SEQUENCE LENGTH:58
SEQUENCE TYPE:nucleic acid
TOPOLOGY:linear
CLONE:HUMGS03306
SEQUENCE DESCRIPTION:
GATCAGATTT TTTATGCCAT CCTCATTAGT TTTCCAATAC AATATAAGTT GAAACAAA 58

SEQ ID NO:2818
SEQUENCE LENGTH:58
SEQUENCE TYPE:nucleic acid

TOPOLOGY:linear
CLONE:HUMGS03307
SEQUENCE DESCRIPTION:
GATCTCAGAC TTCTGGCTTC CACAACTGTA ACAATAAATT TATGTCGTTT AAGCCAAA 58

SEQ ID NO:2819
SEQUENCE LENGTH:57
SEQUENCE TYPE:nucleic acid
TOPOLOGY:linear
CLONE:HUMGS03308
SEQUENCE DESCRIPTION:
GATCCACTGG GTGAATCCTC CCGCTCAGAA CCAATAAAAT AGAATTGACC TTTTAAA 57

SEQ ID NO:2820
SEQUENCE LENGTH:56
SEQUENCE TYPE:nucleic acid
TOPOLOGY:linear
CLONE:HUMGS03310
SEQUENCE DESCRIPTION:
GATCTGTGGT CATTGTCCCT CTGCAGAATA AAGATTGCTC AGGCCTGCCT GGCAAA 56

SEQ ID NO:2821
SEQUENCE LENGTH:59
SEQUENCE TYPE:nucleic acid
TOPOLOGY:linear
CLONE:HUMGS03311
SEQUENCE DESCRIPTION:
GATCTTTGGT TTGTTTCCAG GTTTGGTTAT TATNAATAAA GTTGCTGTGA ATACTTAAA 59

SEQ ID NO:2822
SEQUENCE LENGTH:56
SEQUENCE TYPE:nucleic acid
TOPOLOGY:linear
CLONE:HUMGS03312
SEQUENCE DESCRIPTION:
GATCTTCATT GGGGGATTGA GCAGCATTTA ATAAAGTCTA TGTTTGTATT TTGAAA 56

SEQ ID NO:2823
SEQUENCE LENGTH:54
SEQUENCE TYPE:nucleic acid
TOPOLOGY:linear
CLONE:HUMGS03313
SEQUENCE DESCRIPTION:
GATCTAAGAA ATAAAGAGAA ATGTTTCANT TTGCATTGAA ATAGGAAAAC GAAA 54

SEQ ID NO:2824
SEQUENCE LENGTH:53
SEQUENCE TYPE:nucleic acid
TOPOLOGY:linear
CLONE:HUMGS03315
SEQUENCE DESCRIPTION:
GATCATNACT GGCTTTGCGC ATCGAGGGGC CATTAAAGAG TCTACTTTTC AAA 53

SEQ ID NO:2825
SEQUENCE LENGTH:52
SEQUENCE TYPE:nucleic acid
TOPOLOGY:linear
CLONE:HUMGS03318
SEQUENCE DESCRIPTION:
GATCTTTTTA AAACTGCAAG TGTTGAATAC TAGAGGTTGT TAGACCNTTA AA 52

SEQ ID NO:2826
SEQUENCE LENGTH:51
SEQUENCE TYPE:nucleic acid
TOPOLOGY:linear
CLONE:HUMGS03319
SEQUENCE DESCRIPTION:
GATCTTGCCA CTGTAGTCCA GGCTGGGCTA CGGAGAGACC CTGCCTCCAA A 51

SEQ ID NO:2827
SEQUENCE LENGTH:54
SEQUENCE TYPE:nucleic acid
TOPOLOGY:linear
CLONE:HUMGS03324
SEQUENCE DESCRIPTION:
GATCCTTGCT GATAAATATA ACCATCAATA AAGAAGCATT CTTTTCCAAA GAAA 54

SEQ ID NO:2828
SEQUENCE LENGTH:50
SEQUENCE TYPE:nucleic acid
TOPOLOGY:linear
CLONE:HUMGS03325
SEQUENCE DESCRIPTION:
GATCTCACCA CACTGCAACC TCTGGCGACA GAGCGAGACT TGGTTTCAAA 50

SEQ ID NO:2829
SEQUENCE LENGTH:50
SEQUENCE TYPE:nucleic acid
TOPOLOGY:linear
CLONE:HUMGS03326

SEQUENCE DESCRIPTION:
GATCCACCCA CTCTCTGAAA CTTCTTTGCT AATAAAACAT TCCTACTAAA 50

SEQ ID NO:2830
SEQUENCE LENGTH:55
SEQUENCE TYPE:nucleic acid
TOPOLOGY:linear
CLONE:HUMGS03331
SEQUENCE DESCRIPTION:
GATCTTTTTT TTTCCTTACA GACACCCATA ATAAAATATC ATATTAAAAT TCAAA 55

SEQ ID NO:2831
SEQUENCE LENGTH:53
SEQUENCE TYPE:nucleic acid
TOPOLOGY:linear
CLONE:HUMGS03332
SEQUENCE DESCRIPTION:
GATCCAGGGG GAAATGCTGG AAGTCAATAA AACTGAGTTT TGAGAGCTTG AAA 53

SEQ ID NO:2832
SEQUENCE LENGTH:131
SEQUENCE TYPE:nucleic acid
TOPOLOGY:linear
CLONE:HUMGS03356
SEQUENCE DESCRIPTION:
GATCCACATA AAAAGAAATG TGAAGTTTTC TTTTACTATC TTTTCATTTA TCAAGCAGAG 60
ACCTTTGTTG GGAGGCGGTT TGGGAGAACA CATTTCTAAT TTGAATGAAA TGAAATCTAT 120
TTTCAGTGAA A 131

SEQ ID NO:2833
SEQUENCE LENGTH:431
SEQUENCE TYPE:nucleic acid
TOPOLOGY:linear
CLONE:HUMGS03374
SEQUENCE DESCRIPTION:
GATCTGCTGA TTGCATTATT TTAATTTGCT TTTCTGGGAA AGCAGTTTTG CTAAAAGCTG 60
TACAGACTTT TNCTTTTGTA CCTAGCAGTA CTTTATATAG TATAGCTTTG GGCCATGTAG 120
CATTTTAAGA CTCAATTTTA AAAAATNATT AATCTGTTGC TGACTCTTAA TTCCTATTTC 180
AATATGTGTT TCCTTGAAGA ATTCAGGATA CAACTTCTTG TGTATGACAG CTTTCCTTCA 240
CACACTATTT TNGTGGGTGT GTATATATCT GATTTGGGAA GAATTTAAAA AACACATAGC 300
TTTTNAATTN GTTTGAAACA GACTTTCTGC CTGTGACATT TNNGCNTTTA NCCAATTAAA 360
GAAGCCAATG GCATTTTNGG TNTTATATNG TGTTTTCCAC TAGTATATCC CTGTGGATTT 420
GTTTGTGCCC N 431

SEQ ID NO:2834

SEQUENCE LENGTH:429
SEQUENCE TYPE:nucleic acid
TOPOLOGY:linear
CLONE:HUMGS03375
SEQUENCE DESCRIPTION:
GATCCCATGG GTGGCACATT GGGACTTCTN TCCCTCCCCC ATCTACACAG AAGACTGTCA 60
CCATGCTGAC AGAAGCCTGT CCTTGTAAGG CCCAGCCTTC CAGGGGAACA CTCAGACATG 120
TTCATTCTCT TCCTGCTTCT GCTCTGGGCC GGTGGGTGGC TCTCAGAAAA TACTTGCTGC 180
TGGCAAAAGG CCTGTACTCA GGCATTTGCT TTGACTTGAT GTTGCCAAGG GACTGAGGCC 240
ATTGGCAGGC TTAGTACCAC CTGCTCCTCA TCTTAGGAGT CTCCTTTTCA AATAATTAGG 300
CTCTGTTCCC ATTTTAAAAC TCTGATATTG GGCTTCACCT GTGACTGGAC ACTTTACTAG 360
AGGCCCATTT NCACTAAACA NTAAAATCTA AATAAATTGG AAGGAATAAC AACNACAAAG 420
GGNTAGAAA 429

SEQ ID NO:2835
SEQUENCE LENGTH:427
SEQUENCE TYPE:nucleic acid
TOPOLOGY:linear
CLONE:HUMGS03376
SEQUENCE DESCRIPTION:
GATCTGGGCT TCCTNACTTC TGGAGAAGTG GCCATCCCAT GCCCACCTTT NCCATGGAGG 60
AGTGGGCCCT GCCAGCTGCC ACAGCTGCAT GACCTGCTTC CCCACCCCAC GGTGTCGTTT 120
TNTTTTNAAA GGTCACCTGT CCTCACTCAC CCAGCCAGCC CTTCAGGTGC NTTCTACTCC 180
CAGTGCCAAA GCCAGACCAC TGGGGTTTCC TGCTGCAGGA ATTGGGGGCT GGGAACAGCA 240
GAGGGGATAG AAGTNTGGTG GAGGTGGAGT GGGCACGCNT TAGCCTACGG AAAGGNCCAT 300
TTTTTGGGNC CACTGAGCTG CACTGGGATT TTTCANTTCT GGNCCTGANT NCCTTTAGGG 360
NAAATAACAC AGCAGGACCN CGGTGGGGAT TTTAAGNACT TTTTTTTNNA GATTNAAANG 420
GNTTCTN 427

SEQ ID NO:2836
SEQUENCE LENGTH:416
SEQUENCE TYPE:nucleic acid
TOPOLOGY:linear
CLONE:HUMGS03377
SEQUENCE DESCRIPTION:
GATCCTGATG CTGCCAGAGC CATTGTGGAT GCTTTACCAC CACCCTGTGA ATCTGCCTGC 60
ACAGTACCAA CAGACGTGGA TAAGTGGTTC CATCACCAGA AAAACTAATG AGATTTCTCT 120
GGAATACANG CTGATATTGC TACATCGTGT TCATCTGGAT GTATTAGAAG TAAAAGTAGT 180
AGCTTTTCAA AGCTTTAAAT TTGTAGAACT CATCTAACTA AAGTAAATTC TGCTGTGACT 240
AATCCAATAT ACTCAGAATG TTATCCATCT AAAGCATTTT TCATATCTCA ACTAAGATAA 300
CTTTTAGCAC ATGCTTAAAT ATCAAAGCAG TTGTCATTTG GAAGTCACTT GTGAATAGAT 360
GTGCAAGGGG GNGCACATAT TGGNTGTATA TGTTTNCCNT ATGTTAGGGG ATAAAN 416

SEQ ID NO:2837
SEQUENCE LENGTH:398

SEQUENCE TYPE:nucleic acid
TOPOLOGY:linear
CLONE:HUMGS03378
SEQUENCE DESCRIPTION:
GATCAAAGAT GAGGCCACAA GGACACCAGC AACTGAAGAG GCTGCCTACT TGGTATCAAG 60
ACTGAAGGAG AACTACGTTT TGCTGAGCAC TGATGGCCCT GGGAGGAACA TCCTGAAGTT 120
TAAGCCCCCA ATGTGCTTCA GCCTGGACAA TGCACGGCAG GTGGTGGCAA AGCTGGATGC 180
CATTCTGACT GACATGGAAG NGAAGGTGAG AAGTTGTGAA ACGCTGAGGC TCCAGCCCTA 240
AGCCAGCCCT GCTCTGCCTA AGTGTACTCC AGAAGAAACT CATCTCATCC AAATACACGC 300
TATTGAGAAG GCGAGCCTGA CCTCCCTCTT ACAGATAAAG TCAGCTTTCA GAGGCTCAGG 360
GTGGGGGGGC CTGCCCGAGG CCATAATGCT ACCCACCN 398

SEQ ID NO:2838
SEQUENCE LENGTH:408
SEQUENCE TYPE:nucleic acid
TOPOLOGY:linear
CLONE:HUMGS03379
SEQUENCE DESCRIPTION:
GATCGCACAC ATGCTTTGTT TGGAATATGG AGTGAACACA ATTATGTACC AAATTTAACT 60
TGGCAAACTT NCTATTGCCT GTCCCATGTG CATCTNATTT AAAATTTCCC CCATGGAAAT 120
CACTCTCCTG TTGACTATTT CCAGAGCTCT AGGTGTTTAG GCAACGTGTG GTGTCCTGAG 180
ANGGCCATAG CGCCATCATG GGCTGATTTT AATTACCAGG TCCCCCAGAA GCAGGTGGGA 240
GGCTCTGCTT CCTGCTGGCC GTTCTGCAGC CTGGACCTGT GGACCCTGGT TGTAAAGNGT 300
AAATTGTTTT CTTANGGAAA CCAGTGTCAC CTTNTNTNAA CCTTTAAANT TTAAAAATAN 360
TTTGGGGGAA NAAATTNCNG NNACNGGAGG GTTAAAATTT ANCNNANN 408

SEQ ID NO:2839
SEQUENCE LENGTH:388
SEQUENCE TYPE:nucleic acid
TOPOLOGY:linear
CLONE:HUMGS03380
SEQUENCE DESCRIPTION:
GATCCACCAG CTGAGCAGGG CGGGGCTGAG TAAATGCCGG CTTACCATCT CTACCATCAT 60
CCGGTTTAGT CATCCAACAA GAAGAAATAT GAAATTCCAG CAATANGAAN TGAACAAAAG 120
ATTGGAGCTG AAGACCTAAA GTGCTTGCTT TTTGCCCGTT GACCAGATAA ATAGAACTAT 180
CTGCATTATC TATGCAGCAT GGGGTTTTTA TTATTTTNAC CTAAAGACGT CTCTTTTTGG 240
TAATAACAAA CGTGTTTTTT AAAAAAGCCT GGNTTTTCTC AATACGCCTT TAAAGGTTTT 300
TAAATTGTTT CATATCTGGT CAAGTTGAGA TTTTTTAAGA ACTTCATTTT TTAATTTNGT 360
AATAAAAGTT TTNCAACTTT GATTTTAN 388

SEQ ID NO:2840
SEQUENCE LENGTH:80
SEQUENCE TYPE:nucleic acid
TOPOLOGY:linear
CLONE:HUMGS03381

SEQUENCE DESCRIPTION:
GATCTTGCTG AAAGTGTTTC TAAAGATAGC ACCACTTTTT TTTTNAAAGC TTTTATATAT 60
NAAAAAACGT ATCATGCAAA 80

SEQ ID NO:2841
SEQUENCE LENGTH:384
SEQUENCE TYPE:nucleic acid
TOPOLOGY:linear
CLONE:HUMGS03382
SEQUENCE DESCRIPTION:
GATCTTGTCC TAGCCTTTTN ACTTTTGCAA TTTCAGTATC TTCATCTCTA AACTAGGGAA 60
ACACTNGGAT TCTTTCTTAG CTGTGGGGGA AGGTATTTGG TTAGATGACT TTGAATGAAT 120
AGACTGCTGT GCTGAAAGAG CTTTATCACA CTGTCTCAAA GTATGTAAAG ATACATAGGT 180
GGATGCTCTT ACTGCAGCAG TCATGAATAC ATTTTTAGCC ATTTACCTAA GGAAAAAGAC 240
AGTTTTTCTA GGTACCATGA AGGAAGATTG ACCCTGTTGG TATGCCTGTG GGGGTGGGAT 300
GTGAGTGGGA CTGATAAACT GATACTTTTG GTTCGTATGT ACATACTGGN AGAATCTTCA 360
TANTAAATGN GACTACACAA CAAA 384

SEQ ID NO:2842
SEQUENCE LENGTH:376
SEQUENCE TYPE:nucleic acid
TOPOLOGY:linear
CLONE:HUMGS03383
SEQUENCE DESCRIPTION:
GATCCAGGGC AAGGCCATTG GGATATTCTG AAACTTGAAT ATTTTGTTTT GTGCAGAGAT 60
AAAGACCTTT TCCATGCACC CTCATACACA GAAACCAATT TTNTNTNTNA TACTCAATCA 120
TTTCTAGCGC ATGGCCTGGT TAGAGGCTGG TTTTTTCTCT TTTCCTTTGG TCCTTCAAAG 180
GCTTGTAGTT TTGGCTAGTC CTTGTTCTTT GGAAATACAC AGTGCTGACC AGNCAGCCTC 240
CCCCTGTCCC CTCTATGACC TCGCCCTCCA CAAATGGGAA AACCAGACTA CTTGGGAGCA 300
CCGCCTGTGA AATACCAACC TGAAGACACC GTTCATTCAG GCAACGNACA TAACAGNAAA 360
TGAAGGTGGA CANGCN 376

SEQ ID NO:2843
SEQUENCE LENGTH:370
SEQUENCE TYPE:nucleic acid
TOPOLOGY:linear
CLONE:HUMGS03384
SEQUENCE DESCRIPTION:
GATCCCAAAC CTAGCCCCCT AGTGGGACAA GGACCTGACC CTCCTGCCCG CATACACAAC 60
CCATTTCCCC TGGTGAGCCA CTTGGCAGCA TATGTAGGTA CCAGCTCAAC CCCACGCAAG 120
TTCCTGAGCT GAACATGGAG CAAGGGGAGG GTGACTTCTC TCCACATAGG GAGGGCTTAG 180
AGCTCACAGC CTTGGGAAGT GAGACTAGAA GAGGGGAGCA GAAAGGGACC TTGAGTAGAC 240
AAAGGCCACA CACATCATTG TCATTACTGT TTTAATTGTC TGGCTTCTCT CTGGACTGGG 300
AGCTCAGTGA GGATTCTGAC CAGTGACTTA CACAAAAGGC GCTCTATACA TATTATAATA 360
TATTCGCTTN 370

SEQ ID NO:2844
SEQUENCE LENGTH:367
SEQUENCE TYPE:nucleic acid
TOPOLOGY:linear
CLONE:HUMGS03385
SEQUENCE DESCRIPTION:
GATCTGTCAT CTGGTGCTGC CAGCTGTGCT CACTCTGGTT TTCTGCTCAG GGTCTGAAGC 60
AGCTGCTGTC TNCCTCCTCT GCCCCCATCC CCTGGCTCTC CCCTGGGCAC AGTGCCACTC 120
CCTTGGAAGG GAGGGAACCA CCCGTNAGCC CCAGGGCTTG GNAAGCCTGA GGCGGTGNCT 180
CTGCCTCTCC CTGCCCCCAG CACAATTGGC AGAGATGAGG CGGGTGGTGG ACGGCTGGGG 240
GTGTCGTGGC AGGGTCTNGC ACAGGGNCAT GTCCTGGCTG TAACCCAGGC AGTGGGAGGT 300
CTGCAAGCCT GGTCATGGCC TTCACAGCAN GGTNCCTGTT GGACAGACAT ATCTGNATAT 360
TTATNAA 367

SEQ ID NO:2845
SEQUENCE LENGTH:366
SEQUENCE TYPE:nucleic acid
TOPOLOGY:linear
CLONE:HUMGS03386
SEQUENCE DESCRIPTION:
GATCTCCAAC ACTATCCGAT AAAGTAGAAG CAACTGAGGT ACCGTGAAAG AGAAAGGAGC 60
TGGAACTGAG AAAAAGCAGA GGATTCGGAC GGAGAGCGCG AGGACTCGGC GGCTGAGCGC 120
GCCCGACAGC AGCTAGAGGC GCTGCTCAAC AAGACTATGC GCATTCGCAT GACAGATGGA 180
CGGACACTGG TCGGCTGCTT CCTCTGCACT GACCGTGACT GCAATGTCAT CCTGGGCTCG 240
GCGCAGGAGT TCCTCAAGCC GTCGGATTCC TTCTCTGCCG GGGAGCCCCG TGTGCTGGGC 300
CTGGCCATGG TACCCGGACA CCACATCGTT TCCATTGAGG TGCAGAGGGA GAGTCTGACC 360
GGGCCN 366

SEQ ID NO:2846
SEQUENCE LENGTH:365
SEQUENCE TYPE:nucleic acid
TOPOLOGY:linear
CLONE:HUMGS03387
SEQUENCE DESCRIPTION:
GATCCCCCCA AGGNGAAGTG TGAGACAATG GTCTATCACC CCAACATTGA CCTCGAGGGC 60
AACGTCTGCN TCAACATCCT CAGAGAGGAC TGGAAGCCAG TCCTTACGAT AAACTCCATA 120
ATTNATGGCC TGCAGTATCT TTNCTTGGAG CCCAACCCCG AGGACCCACT NAACAAGGAG 180
GCCGCAGAGG TCCTGCAGAA CAACCGGCGG CTGTTTGAGC AGAACGTGCA GCGCTCCATG 240
CGGGGTGGCT ACATCGGCTN NACCTACTTT NAGCGCTGCC TGAAATAGGG TTGGCGCATA 300
CCCACCCNNG GCCACGGCCA CAAGCCCTGG GCATCCCCTG CAAATANTTN TTGGGGGGCC 360
ATGGN 365

SEQ ID NO:2847
SEQUENCE LENGTH:364

SEQUENCE TYPE:nucleic acid
TOPOLOGY:linear
CLONE:HUMGS03388
SEQUENCE DESCRIPTION:
GATCTAGAGT ATGTTGAAGA AGATGGACAT GTGGTGGTAC GAAAACAATT CCCCTGTGGC 60
CTGCTGGACT GGGTGGAACC AGAACAGGCT AAGGCATACA GTTCTTGACT TTGGACAATC 120
CAAGAGTGAA CCAGAATGCA GTTTTCCTTG AGATACCTGT TTTAAAAGGT TTTTCAGACA 180
ATTTTGCAGA AAGGTGCATT GATTCTNAAA TTCTCTCTGT TGAGAGCATT TCAGCCAGAG 240
GACTTTGGAA CTGTGAATAT ACTTCCTGAA GGGGAGGGAG AAGGGAGGAA GCTCCCATGT 300
TGTTTAAAGG CTGTAATTGG AGCAGCTTTT GGCTGCGTAA CTGTGAACTA TGGCCATATA 360
TAAN 364

SEQ ID NO:2848
SEQUENCE LENGTH:363
SEQUENCE TYPE:nucleic acid
TOPOLOGY:linear
CLONE:HUMGS03389
SEQUENCE DESCRIPTION:
GATCTGGGGA GGCAGGCATT CCTGAAGGGA ACTAGAGGTG TTACAGAGGA CCCTTACGTG 60
AGAAATAGCT GAAAAGGGCA CTCGCAACCC TGGGCTGGGG AGGAGGAGAG AGTCCCAGAG 120
CTCATCCCCC CTGCTGCCCA GTGCAAACCA CTTNTCCATG CTGCAAAGGA GAAGCACAGC 180
TCCTGCCAGG GTGAGCAGGG TCAAGCCTCT TATTCCAGGA GAAGGGGGCT CTGCCCAGGC 240
CCTACTACCN ATTGTNCCCT TCCTCTTCCT GCCCTTGGAA CCCCCTNCCT GTCCCAGGGC 300
CCTTCNAGCC CATTGCTGCC AAGGTGGAGG GANGGATAAA GNCACTTCTG GGTTTTGAAG 360
CCN 363

SEQ ID NO:2849
SEQUENCE LENGTH:361
SEQUENCE TYPE:nucleic acid
TOPOLOGY:linear
CLONE:HUMGS03390
SEQUENCE DESCRIPTION:
GATCTGGGTG TGGCCAAAAA CAATTTTNTT TATTCTGTCT ATCAAATAGT ACTTCTACCA 60
CTGTTTGGAG AAAATTGAAG AAAAGAATAA GATGATTAAA TGAATTCTCT AAAAGAACAT 120
ATTTTAAGAG ACAGAACTTA GACATAACCA AGTAGTTGTA TACCTGATTG TAACAATCAT 180
CTTTTATAAA AGCAAAATTA TGCATAAATG TAAAACACCC CTAAACAAAG GGAACAAATG 240
ANTTTTTGGT TATATTGTTT TCTTGGCTCA AAGTATCAAT TTATTATTAT AATAGAAATT 300
TATAAGTTGC TAGAAAGTCT ATATTTTTAA CGCACGGAAT AAAANTATGT TTTCTATTAA 360
A 361

SEQ ID NO:2850
SEQUENCE LENGTH:355
SEQUENCE TYPE:nucleic acid
TOPOLOGY:linear
CLONE:HUMGS03391

SEQUENCE DESCRIPTION:
GATCTGTCTC CAAGACCTTG GCCTCTCCTT GGAGGACCTT TAGGTCAAAN TCCCTAGTCT 60
CCACCTGANA CCCTGGGAGA NAAGTTTGAA GCACAACTCC CTTAAGGTCT CCAAACCAGA 120
CGGTGACGCC TGCGGGACCA TCTGGGGCAC CTGCTTCCAC CCGTCTCTCT GCCCACTCGG 180
GTCTGCAGAC CTGGGTTCCC ACTGAGGCCC TTTGCAGGAT GGAACTACGG GGCTNGNAGG 240
AGCTTTTGTG TGCCTGGTAG AAACTATTTC TGTTCCAGTC ACATTGGCCA TCACTCTTGT 300
ACTGNCTGCC ACCGNGGAGG ANNCTGGTGA CAGGNCAAAG GCAGTGGAAG GAAAN 355

SEQ ID NO:2851
SEQUENCE LENGTH:344
SEQUENCE TYPE:nucleic acid
TOPOLOGY:linear
CLONE:HUMGS03392
SEQUENCE DESCRIPTION:
GATCTAGTTC TACGTATTAC ACAGGTGTAA TATCTGTGAG TGTAAATAAC TGGAACTGTA 60
CACTGATTAA CATGNCAGTT TCTCTTTTGT TGTTCTTATC TGTACTGTAT TATAGTATGT 120
GGGTATAAAT ATCTACAAGT ATACACACAT ATGTNCTNGT ATTCCACTAT TGTAACCTGG 180
AAGAAAGACT ATGNATNCCC TNTTNNAATT CCGTACTGGT ATTNGTGTTA TTTAAAAAGC 240
AAAATNCGGC TCTATTTAGT TGTATAATAT TAGAGGGTAC TNNGCTTGNN CACAATTCCA 300
AGTGGCCTTA GAGCATTGTT TAGCTNNCCG AAGTATATAT AANN 344

SEQ ID NO:2852
SEQUENCE LENGTH:341
SEQUENCE TYPE:nucleic acid
TOPOLOGY:linear
CLONE:HUMGS03393
SEQUENCE DESCRIPTION:
GATCTATGAG CCTGTNTNGG ACAGCCAGAT GGTCATCATA GTCACGGTGG TGTCGGTNTT 60
GCTGTCCCTG TTCGTNACAT CTGTCCTGCT CTGCTTCATC TTCGGCCAGC ACTTGCGCCA 120
CNGCGGATGG GCACCTACGG GGTGCGAGCG GCTTGGAGGA GGCTGCCCCA GGCCTTCCGG 180
CCATAGCAAC CATGANTGGC ATGGCCACCA CCACGGTGGT CACTGGAACT NAGTGTGACT 240
CCTCAGGGTT GAGGTCCAGC CCTGGCTGAA GGACTTGTNA CAGGCAGCAG AGACTTGGGA 300
CATTGCCTTT NNTAGCCCGA NTACAAACAC CTGGACTTAA A 341

SEQ ID NO:2853
SEQUENCE LENGTH:567
SEQUENCE TYPE:nucleic acid
TOPOLOGY:linear
CLONE:HUMGS03394
SEQUENCE DESCRIPTION:
GATCTCAAGA CCAAAGACAG ATGTCAGTGG GCTGCTCTGG CCCTGGTGTG CACGGCTGTG 60
GCAGCTGTTG ATGCCAGTNT CCTCTAACTC ATGCTGTCCT TGTGATTAAA CACCTCTATC 120
TCCCTTGGGA ATAAGCACAT ACAGGCTTAA GCTCTAAGAT AGATAGGTGT TTGTCCTTTT 180
ACCATCGAGC TACTTCCCAT AATAACCACT TTGCATCCAA CACTCTTCAC CCACCTCCCA 240
TACGCAAGGG GATGTGGATA CTTGGCCCAA AGTAACTGGT GGTAGGAATC TTAGAAACAN 300

GACCACTTAT ACTGTCTGTN TGAGGCAGAA GATAACAGCA GCATCTNGGN CCAGTCTCTG 360
CCTTAAANGG AAATCTTTAT TAATCACGNA TGGTTCACAG ATAAATTCTT TTTTTAAAAA 420
AANCCCANNC TCCTNGGGAT GCACANCCTG TCAAGNGTCT TTTTACACAC AANTTTCAGG 480
TTTNNCATCA CGGGGCTTTG ANTTCNAGGA AATNAAAGGN GGGACAAATT TNGTTNGTTT 540
ACNCTATGGT NCTTTNNTAA NAAACNN 567

SEQ ID NO:2854
SEQUENCE LENGTH:349
SEQUENCE TYPE:nucleic acid
TOPOLOGY:linear
CLONE:HUMGS03395
SEQUENCE DESCRIPTION:
GATCTTTACT TTTTTACTTT TTATTCATCT TTGTGTTTTT ATTTATCTAA AATGGGTATT 60
GATTTTAAGG ACGGTTTTGA AAAAGAAAAG TGTTGGGAAT GAAGCAAGTG ATTGATTGGA 120
AAACATACTG AATGGAAGAA ATATTTAGAT TAAAAATGAG GTAGGTTGAA GTTTCTNCTC 180
TGAAATNATA GATAAATGGT GAAGATAAGG CTTATTGTGA GGNTTCAGTG AGGTAATATA 240
TGCAAAGTAC TTACAATGTT CTGGCACATA GTAATTNATT NNGANAATCG AGCACCCTTA 300
ATTACCTAGA ATGCAGGGTT GTTAGTTTTT NGGTTGACTT TTGTTTTNN 349

SEQ ID NO:2855
SEQUENCE LENGTH:334
SEQUENCE TYPE:nucleic acid
TOPOLOGY:linear
CLONE:HUMGS03396
SEQUENCE DESCRIPTION:
GATCCGCTGT ATTGTGGAGT ATCAGAACAA GGGCCGCGGG AACGAGNGCG TGCAGTACCA 60
GCATGTGTTA CATAGAAATC TCATTTATTT GGCTACCATT GCAGATGCCA GTCCAACCAG 120
CACTTCAAAA GCAATGGAAT AATCTTTCAA AAGCAATAGA ATAATCTTCC ATTTGGCTGT 180
CGTGAGGAGT AATTGAATGT AATCCATCTC TTACAAAATG GAGACAGGGT CTTTACCAAC 240
TCAACTGGTT AAAACATGAN TGAAACCTCT GTGGCTCTTT CAAAACGTGG AAAATGTGAA 300
GAANGCTACT TAACTTNGGC TGTATTCTCA TGGN 334

SEQ ID NO:2856
SEQUENCE LENGTH:332
SEQUENCE TYPE:nucleic acid
TOPOLOGY:linear
CLONE:HUMGS03397
SEQUENCE DESCRIPTION:
GATCGGCCTG CTGCTCCGCA ACTTCGACCG CTACGGCGTG GAGTGCTGAN GGACTCTGCC 60
TCCAACGTCA CCACCATCCA CACCCCGGAC ACCCAGTGAT GGGGGAGGAT GGCACAGTGG 120
TCAAGAGNAC AGACTCTAGA GACTGTCAGA GCTGACCCCA GCTAAGGCAT GGCACCGCTT 180
CTNTCCTTTC TAGGACCTCG GGGTCCCTCT GGGCCCAGTT TCCCTATCTG TAAATTGGGG 240
ACAGTAAATG TATGGGGTCG CAGGGTGTTG AGTGACAGGA GGCTGCTTAG CCACATGGGA 300
GGTGCTCAGT AAAGGAGAGC AATTCTTACA AA 332

SEQ ID NO:2857
SEQUENCE LENGTH:331
SEQUENCE TYPE:nucleic acid
TOPOLOGY:linear
CLONE:HUMGS03398
SEQUENCE DESCRIPTION:
GATCCTGGGT GTNTNTGTAA GGGTNTTGCC AAAGGAGGTT AACATTGGAC TCANTGGGCT 60
GGGGAAAGGC AGACCCACCC TTAATCTGAG TGGACACCAT CTAATCAGCT GCCAGCGAAA 120
ATAAAGCAGG CAGAAAANTG AAAAANNTAG ACTGGCCTAG CCTCCCAGCC TACATCTTTN 180
TCCCATGCTG GATGCTTCCT GCCCTTGAAA ATTGGACTCC AGGTTCTTNA GTTTTGGGAC 240
TTGGACTGGC TCTCCTTCCT CCTCAGCCTG CAGACATCCT ATTGTGGGAC CTTGTGTTTC 300
TGTAAGTTCA TACTTAATAA ACTCTCCATA N 331

SEQ ID NO:2858
SEQUENCE LENGTH:351
SEQUENCE TYPE:nucleic acid
TOPOLOGY:linear
CLONE:HUMGS03399
SEQUENCE DESCRIPTION:
GATCGAGTCT ATACGCTGAA GAAANNTGAC CCGATGGGAC AACAGACCTG CTCAGCCCAT 60
CCTGCTCGGT TCTCCCCAGA TGACAAATAC TCTCGACACC GAATCACCAT CAAGAAACGC 120
TTCAAGGTGC TCATGACCCA GCAACCGNGC CCTGTCCTNT GAGGGTCCCT TAAACTGATG 180
TCTTTTCTGC CACCTGTTAC CCCTCGGAGA CTTCGTAACC AAACTNTTCG GACTGTGAGC 240
CCTNATGCCT TTTTGCCAGC CATACTTCTT TGAGCATCCA GNNTNTNGTG GCGATTTGAT 300
TATGCTTGTT GTGAGGNAAT CATGGGTNGG CNTCNNCCNT TAANGGGGTG N 351

SEQ ID NO:2859
SEQUENCE LENGTH:330
SEQUENCE TYPE:nucleic acid
TOPOLOGY:linear
CLONE:HUMGS03400
SEQUENCE DESCRIPTION:
GATCCTGGGG GCCTGGGTTG GGGGCTAGGG AGACGCCATG TGATGGACAC TCCAGGGACA 60
CACAGCCTAG CACAGCAGCT TATAATGGGC TCTCCGGGGC CATTTGCAAT AACAGCTGCA 120
ATTCCCTGGA TAGACGAGTT GATTTCCTCC CTCTGCCCCT CCCCCAGCCA TGCCAGCTGG 180
CCTTTGTAAG TGCAGGAAAC CGAGTAGAAA ATGTGACCCT CCAAATGGAG AAGCTGCAGG 240
CTTTGCCATT GTGAACCATG GTGAAGTGCT TGGAACATAC TGTTCACTCA CTCTAAAGGN 300
GCTGAGACTG TGCTGTTGTT CTCGTTTTTN 330

SEQ ID NO:2860
SEQUENCE LENGTH:330
SEQUENCE TYPE:nucleic acid
TOPOLOGY:linear
CLONE:HUMGS03401
SEQUENCE DESCRIPTION:

GATCTGGAAA GGACATGACT TCTGAAATAG CCGCTGCTGG GTTTTAAAAG CTGAGGTCTC 60
TCAAAGTGTG GAGGAGACGT TGCCGTCAGN GGGAGCCAAG TGCCGGGAAG ATGTCTATTT 120
TTTTCCTTGT GTATTGAAAA TCATGATGTT TGTTATGACT GCTGATGCGA TTGTNTTTGT 180
AAATNTNATT GTGGCATATA CAGTATTGTC ATACAGTTGA AGAGAAACAA TGTTTCCTAA 240
TGTAAGTNCT CTGAAAATGT TGACACTGTA TATATATATA TNAGGATAGT TTGTNTTTCN 300
NTGTTTGGGG TTTNNNNTTN TTTCNGNTGN 330

SEQ ID NO:2861
SEQUENCE LENGTH:396
SEQUENCE TYPE:nucleic acid
TOPOLOGY:linear
CLONE:HUMGS03402
SEQUENCE DESCRIPTION:
GATCTTAAAC CCAAAGTACG AGAAATGTTT CTGTTGAATG TAATGCTACA TGAATGCTTG 60
ATTTATCAAG CGCCAAAAAG GCATTGTATA GTAGGAAATG TAAGTGGGGT GGCTTATGGC 120
TTCTTTATCC TCTGATTCTA GCACTTTCAA GTGAGCTGTT GCGTACTGTA TCATATTGTA 180
GCTATTAGGG AAGAGAAGAA TGTTGCTTAA GAAAGAACAT CACCATTGAT TTTAAATACA 240
AGTAGCAGGG TATTGCCTTT GATTCAACTG TTTTAAGTCC TCATTTTTCT CAAACTAAGT 300
GCTTGCTGTT CCCAAATATG GCAAGGNATA GCTTTTACAC TTTTTCCCTG CCAACANNTC 360
NTNGATTGGC TTTGCAGANA TNNAGGTTTG ANTTGN 396

SEQ ID NO:2862
SEQUENCE LENGTH:330
SEQUENCE TYPE:nucleic acid
TOPOLOGY:linear
CLONE:HUMGS03403
SEQUENCE DESCRIPTION:
GATCCAAAGT GACCCCACCA GGGGACAGCC AGAGGAAGGG GACCATGGGG TATCCNNAGT 60
GTCCTGGTCT ATCACCCCAG CTTCTTTGTC CCCCAGTACC CCCAGCCCAG CCAGCCAATA 120
AGAGGACACA AATGAGGACA CGTGGCTTTT ATACAAAGTA TCTATATGAG ATTCTTCTAT 180
ATTGTACAGA GTGGGGCAAA ACACGCCCCC ATCTGCTGCC TTTTCTATTG CCCTGCAACG 240
TCCCATCTAT ACGAGGTGTT GGAGAAGGTG AAGAACCCTC CCATTCACGC CCGNCTACCA 300
ACAACAAACG TGCTTTTTTC CTNTTTGAAA 330

SEQ ID NO:2863
SEQUENCE LENGTH:329
SEQUENCE TYPE:nucleic acid
TOPOLOGY:linear
CLONE:HUMGS03404
SEQUENCE DESCRIPTION:
GATCTTATTT TCTGAGAAAT TGATAGCAAA TATGCCAGAA AGTGGACCTA GTTATGAATT 60
CCATCTAACC AGACAAGAAA TAGTATCATT ATTCAACGCA TTTGGAAGAA TTTCTACAAG 120
TGTGAAAGAA TTAGAAAACT TCAGGAACTT GTTACAGAAT ATTCATTAAA GAAAACAAGC 180
TGATATGTGC CTGTTTCTGG ACAATGGAGG CGAAAGAGTG GAATTTCATT CAAAGGCATA 240
ATAGCAATGN CAGTCTTAAG CCAAACATTT TATATAAAGT TGCTTTTGTA AAGGAGAATT 300

ATATTGTTTT AAGTAAACNC ATTTTTAAA 329

SEQ ID NO:2864
SEQUENCE LENGTH:329
SEQUENCE TYPE:nucleic acid
TOPOLOGY:linear
CLONE:HUMGS03405
SEQUENCE DESCRIPTION:
GATCACTTCA GCTGCCTGCT GTGGACAAAG AACATCAAAT TACAGCATCA CGAGTGCTAT 60
TNTTGCCTGT GGTGGTCTCC CTGTCCAAGC GGGNCCGCTT TGCAGAGACC AGAGGCATAT 120
CGCGGCTTGA GCTGAAAATG CATTTNTTGC AGCTTAGGTT GAATTATTTT NCGTTTGCTC 180
TTTCTTCTAC ACGCGCCTGA TGGATAGTGA ACCTATTCAT CAAAAAAGTG CACTGCTCTN 240
CTGTCTATTG TACCGACTTA ACCTCTTCCA CCCAAGTCCG CATCTGTGTG TATCATCAAT 300
AAAGTTGGTN GTGCCTTGGN TTGGGCAAA 329

SEQ ID NO:2865
SEQUENCE LENGTH:328
SEQUENCE TYPE:nucleic acid
TOPOLOGY:linear
CLONE:HUMGS03406
SEQUENCE DESCRIPTION:
GATCGGCGTG ACCACCCCTT GCCGGAGGTG GCCCATGTNA AGCACCTNTT TGCCAGCCAG 60
AAGGCACTGA AGGAGAAGGA GAAGGCCTCC TGGAGCAGCC TCTCCATGGA TGAGAAAGTC 120
GAGTTGTATC GCATTAAGTT CAAGGAGAGC TTTGCTGAGA TGAACAGGGG CTCGAACGAT 180
NTGGAAGACG GTTNTNGGGC GGTNCCATGT TCTTTAATCG GTTTNAACGT GCTCGTTNCC 240
CCTNTGGAAA AAGGNCTTNT GTTTACCGGC CCCCTTCCCG AAAAGTTTTN ACAAAANGGT 300
GGGGTTGCCC AAGNAGGCCC AANAGGNN 328

SEQ ID NO:2866
SEQUENCE LENGTH:327
SEQUENCE TYPE:nucleic acid
TOPOLOGY:linear
CLONE:HUMGS03407
SEQUENCE DESCRIPTION:
GATCTACAGA GTTAGAAATT CGAAAACTAT TCCAGGACTA ATTCTTAATC GGCATTATTT 60
ATACAAGAGG TCAAGTAACA TTTACTAGCG CAATACTGCA CTTGTAAATG AATTATAAAC 120
GCTCTTCTGG AATATATTTA AATAACCATT AAAGAACTGC TTATTCATNC TGGACACTGC 180
ATGTNGATGT TGAATCAACT GATGCCAGCA GAAAGCTATT TTGATTTGTG AACATACTGC 240
CTTATTTAAA GGGTCCTGAT TGCTTGTATT TTANGNCATT CATTAAAAAG AAACCAGGAA 300
ACACTTTTGA ANTAACAGCA TANGGGN 327

SEQ ID NO:2867
SEQUENCE LENGTH:327
SEQUENCE TYPE:nucleic acid
TOPOLOGY:linear

CLONE:HUMGS03408
SEQUENCE DESCRIPTION:
GATCACCCTC CTCCCAAAAA GCAAGAGCTT GTTTATGGCT GAGGAATCGG CGGATTGTCT 60
GAATGACACA TATACAGAGC CCCCACGGAT TTCTNCACAC TCTGGGTCTG TGCTGGTGGA 120
ACATTGCCAA TNAGTTCTTA ATGAGGCACC TGTGTGTAAA TACATGCTTG GTCTTCTCTG 180
CAGAGAACTG AGGCTAAACT CTGTCCCTAC TTCTGGTTTT GCCCTGTNAT GTCGTAACGA 240
GGTGGGCCTT TTGAGGCCAT TTTAGTTTGA GTTCGAGCCA ACCACCTCTG GTTGGTTAGA 300
TGATTGAATA AAAAGGTTCT GAAGAAA 327

SEQ ID NO:2868
SEQUENCE LENGTH:326
SEQUENCE TYPE:nucleic acid
TOPOLOGY:linear
CLONE:HUMGS03409
SEQUENCE DESCRIPTION:
GATCCAATCA CCTCCCACCA GGCTCCACCT CGAATACTGG GGATTACCAT TCAGCATGAG 60
ATTTNGGCAG GAACACAGAC CCAAACCATA CCACACACAT TATCATTGTN AAACTTTGTA 120
AAGTATTTAA GGTACATGGA ACACACGGGN AGTCTGGTAG CTCAGCCCAT TTNTTTATTG 180
CATCTGTNAT TCACCATGTA ATTCAGGTAC CACGTATTCC AGGGAGCCTT TNTTGGCCCT 240
CAGTTTGCAG TATACACACT TGCCANGTAC TCTTGGTAGC ATCCTGTTTG GTATCATNGC 300
ACTGGGTCAC ATTGCCTTAC CTAAAN 326

SEQ ID NO:2869
SEQUENCE LENGTH:325
SEQUENCE TYPE:nucleic acid
TOPOLOGY:linear
CLONE:HUMGS03410
SEQUENCE DESCRIPTION:
GATCTATGGA ACAGAGGAGG AGCGATGCAG TTGGGAGAGG AAGCTAGAAG GGTTATGGTT 60
GGAGTTCTGT ACAGTGTTGA GTTTCCGACA GGGAAAGAGG ATTCCTCCAA TGCGCCTAGA 120
GAGAAAGCCT GAGCAGGAGA TGATGCAGCA GAGGGGAAGG GCCCTGTGGT GCCGCCGCCC 180
TTCCTTCAGC CTCCGAAGGT GATGGAAATG GAGAGTGGAG GACCAGGCCT CCAGCTGTNT 240
GGCCTNGCCN TTCACGCCTT AACACTAAGC CCACCTCCCC TTNTCTCCTT CCCAGCATTG 300
AGCCCTTGGT TGCCTGGGCC CAGGN 325

SEQ ID NO:2870
SEQUENCE LENGTH:325
SEQUENCE TYPE:nucleic acid
TOPOLOGY:linear
CLONE:HUMGS03411
SEQUENCE DESCRIPTION:
GATCTTCAGA AACCTTGCTA GGAGAAGTTA ATGAAAATAG AGTATCAATA GCATTGAAAA 60
AAACCTCTGA AATCCTTCAC GATATTGACT GTCTTCTATC AAACTCTAAA AAGTGAAAAA 120
TGGAATTATT ACAACATATT ATTAAAGTTT AAGGATGTTG TTTCAAGTGA CTGGTATAAA 180
ATTACTAGTA AGCCAAGATA TTGTACGTAT TACCCCGGCA CAATGATTGT GAAAGANCTG 240

ACATTTAACT TTGAAGGTAT TTTTAACTTG TACATTTTTA AAATATGTAT TTATATCTNA 300
NTANTAAATN TTTCGAAGTT TTAAA 325

SEQ ID NO:2871
SEQUENCE LENGTH:325
SEQUENCE TYPE:nucleic acid
TOPOLOGY:linear
CLONE:HUMGS03412
SEQUENCE DESCRIPTION:
GATCCAAGCG GAGATTGACG CTGCAGCGGA NACTCATCGA CTTCTTCCGG TTCAATGCCA 60
AGNATGCGGT GGAGCTGGAG GGGCAGCAGC CCATCAGCGT GCCCCCGAGC ACCAACAGCA 120
CGGTGTACCG GGGTCTGGAG GGCTTCGTGG CGGCCATCTC GCCCNTTAAC TTCACTGCAA 180
TCGGCGGCAA CCTGGCGGGG GCACCGGCCC TGATGCTTCG AGAGGGAGTT GTGACTCTGC 240
CTCTTTGCCA GATGACAAAA CTGAGACACA CAGCCGTGAC TTACTTGGCA AGACTGAGTG 300
ACTAGTAAAT AAATGATGGA GCAGN 325

SEQ ID NO:2872
SEQUENCE LENGTH:323
SEQUENCE TYPE:nucleic acid
TOPOLOGY:linear
CLONE:HUMGS03413
SEQUENCE DESCRIPTION:
GATCCTCCTT CTAGCTCATC TGTAGATACT AGTAGTAGTC AACCAAAGCC TTTCAGACGA 60
GTAAGACTTC AGACAACATT GAGACAAGGT GTCCGTGGTC GTCAGTTTAA CAGACAGAGA 120
GGTGTGAGCC ATGCAATGGG AGGGAGAGGA GGAATAAACA GAGGAAATAT TAATTAANTG 180
GTCTGTAAAC AATAACAACT GTGAATAAGA TTATCAAATC TNTTTTAGTG TAATGATTGT 240
CAAGTTTAAA ANCATTTTTN TATATAACCT GGTATACTCA TGTCAATATN CTTTATTAAT 300
AAAANGNTTT TCAGTGTCAT AAA 323

SEQ ID NO:2873
SEQUENCE LENGTH:322
SEQUENCE TYPE:nucleic acid
TOPOLOGY:linear
CLONE:HUMGS03414
SEQUENCE DESCRIPTION:
GATCTGCCCA TCTCAGCCTC CCAAAGTGCT GGGATTACAG GTATGAGCCA CAACGCCCAN 60
CCATCATTTA TCTTTTTCAC TCATTTTTTC ATGAGTATGC AGAGGAGTTT TCAAGAGGCT 120
ATCTGGTGTG ATATTGTAAT AGGCTGAATG CAAAAGCAGA TACAAGATTC CAGCTTTCGT 180
CTGTCATCAG ACATTGAAGA GATTTGGAGA AATGTAAAGC AGCAGTACTC TTCTCACTAA 240
TTTTTGTTGT GGTTAGTTAG AAAAATGGTT ATTTTNAGTG GGTTTTAATA TGTGAAAAAA 300
ATAAAATTAG CTATTTGCCA AA 322

SEQ ID NO:2874
SEQUENCE LENGTH:319
SEQUENCE TYPE:nucleic acid

TOPOLOGY:linear
CLONE:HUMGS03415
SEQUENCE DESCRIPTION:
GATCACCTNT GGCGTGTTCC TNCCAAAACN GCATAGCCCC AATCTAATAT CATCCCGAGG 60
AAATACCAGA CACACGCAAG GGAAAGGACA TTCTACAAAG GAACGGACTC ATACTCTTCG 120
AAAAGTGTCA AGGTCATCTA CGACAAAAGA CTGAAAGGAC CCCAGTCGAA AAAAGNCAAG 180
ATGACAAANT GCAACGTTTC ATCCTAGACT GGATTCTGGA CCCAAAAAAT CCCCTTCTTT 240
TGTCAGAAAG GAAATTTGTG GAACAACTGG CAAAATCTGG AGTTGGGTCT GCAGATTACA 300
CACTGGTATC GCAGCGAAA 319

SEQ ID NO:2875
SEQUENCE LENGTH:320
SEQUENCE TYPE:nucleic acid
TOPOLOGY:linear
CLONE:HUMGS03416
SEQUENCE DESCRIPTION:
GATCTCCCTT CGTGGAGACG ACACCGGGGT CCTCCTGCCC ACCCCAGGGG AGGCCCAGGA 60
TGCTGATTTG AAGGATGTAA ATNTNATTCC AGCCACCGCC TGACCATCCG CCATTCCGAC 120
TGCTAAAAGC GAATGTAGTC AGGCCCCTTT NATGCTGTGA GACCTCCTGG AACACTGGCA 180
TCTNTGAGCC TCCAGAAGGG GTTCTGGGCC TAGTTGTCCT CCCTCTGGAG CCCNGTCCTG 240
TGGTCTGCCT NAGTTTTNCC CTNCTAATAC ATATGGCTGT TTTCCACCTG GNTAATATAA 300
CCANGAGTTT GGGCCGNAAA 320

SEQ ID NO:2876
SEQUENCE LENGTH:308
SEQUENCE TYPE:nucleic acid
TOPOLOGY:linear
CLONE:HUMGS03417
SEQUENCE DESCRIPTION:
GATCTCTGCT GTGNCTCCCA GAGTTACTGC TGCCAGACTG AACTGGAAGG GGAGGATGTA 60
GGAGCCTGCA CAGCTCACAC CTACTCCTGT GGCTCTGGAG TGGGCATCTN CCTGAGAGTA 120
CGGGAATGTN AGGTGCTATT TTAAGCTGGC AAAACCAAAG GCTGTTTTNA TTCTCCTCCT 180
TACCTTGATG ACTATGGGGA GACCGACCAG GGACTCANAC GGGGAAATCC TTTACATTTA 240
TGCAAAGAGC GATTCAAGAA GATTCAGAAG CTCTGGCACC AACACAGTGT CACAGNGGAA 300
NTTGGNCN 308

SEQ ID NO:2877
SEQUENCE LENGTH:308
SEQUENCE TYPE:nucleic acid
TOPOLOGY:linear
CLONE:HUMGS03418
SEQUENCE DESCRIPTION:
GATCTTGACC ATGACAGTTT TNCACTTTTA ATGAAACGGG GAACCAAGAG GAACTTACTT 60
GTGTAACTNA CAATTNCTGC AGAAATCCCC CTTCCTCTAA ATNCCCTTTA CTCCACTGAG 120
GAGCTAAATC AGAACTGCAC ACTCCNTCCC TGATGATAGA GGAAGTGGAA GTCCCTTTAG 180

GATGGTGATA CTGGGGGACC GGGTAGTGCT GGGGAGAGAT ATTTCCTTAT GTTTATNCGG 240
AGAATTTGGA GAAGTGATTG ACCTTTTCAA GNCATTGGGA AACAAATAGA NCACANTATA 300
NTTTNNCN 308

SEQ ID NO:2878
SEQUENCE LENGTH:307
SEQUENCE TYPE:nucleic acid
TOPOLOGY:linear
CLONE:HUMGS03419
SEQUENCE DESCRIPTION:
GATCTAGAAG ACGATTAAGG GAAGGTCGTT CTCAGTGAAA ATCCAAAAAC CAGAAAAAAA 60
TGTTTATACA ACCCTAAGTC AATAACCTGA CCTTAGAAAA TTGTGAGAGC CAAGTTGACT 120
TCAGGAACTG AAACATCAGC ACAAAGAAGC AATCATCAAA TAATTCTGAN CACAAATTTA 180
ATATTNNNNT TNCTGAATGA GAAACATGAG GGNAATTGTG GAGTTAGCCT CCTGTGGTAA 240
AGGAATTGAN GAAAATNTAA CACCTTACAC CCTTTTCCAT CTTGACATTA AAAGTNCTGG 300
CTAACTN 307

SEQ ID NO:2879
SEQUENCE LENGTH:307
SEQUENCE TYPE:nucleic acid
TOPOLOGY:linear
CLONE:HUMGS03420
SEQUENCE DESCRIPTION:
GATCTTGAGG GCAGACAGCA GAATTCCTCT TATAAAGAAA ACTNTTTNGG AAAATACGTT 60
GAGGGAGAGA NGACCTTGGG CCAAGATGCT AAATGGGAAT GCAAAGCTTG AGCTGCTCTN 120
CAAGAGAAAA TAAGCAGGNC AGAGGATTTN CTCTGGACAG AGATGGAAGA GCCGGGAACA 180
GAGAAGTNTG GGGAAGAGAT AGGAACCAGC AGGATGGCAG GGGNNNNGGG CTCAAGGGTN 240
AGGAGGCCAG TGGGACCCCA CAGAGTTNGG GGAGATAAAG GAACATTGGG TTTGCTTTGG 300
TGGCACN 307

SEQ ID NO:2880
SEQUENCE LENGTH:305
SEQUENCE TYPE:nucleic acid
TOPOLOGY:linear
CLONE:HUMGS03421
SEQUENCE DESCRIPTION:
GATCTGCCGT GTCCTTATAA AGTGGATGAA AAATGTTTTG TACCCATCTG GAAAACCAAC 60
AACTTGAAAT CTCAGGTATT CCAGGTCACT GACATGAATT TGAAGATATA TCTATCTGTA 120
TGGATATATA TCTATATGTA TATAGATATA TAANTACAGA GAGATATCTG GCTTGGTTTT 180
AATTATGTNC TTAAATTTGT GTGCCAATAA TTGCATATAG ATTTTNTTNC TTAAATATTT 240
GACTGTGGAA CATGCCATTT TAAATATGTN GTAAGGCCTG TNNTAATAAA ACGTTTAGTA 300
TGAAA 305

SEQ ID NO:2881
SEQUENCE LENGTH:305

SEQUENCE TYPE:nucleic acid
TOPOLOGY:linear
CLONE:HUMGS03422
SEQUENCE DESCRIPTION:
GATCCACTGA TGGCTCCAGA CCCCAACTTT AGCCATTGCA CGTGTTGAAC TCCAGGTTGA 60
GCCTTTGCTG GAGCCGCCTG CAAGGNGCAT GTTTGACCAA TCCCCAGTGG AGGGGAGGGT 120
GGTGTTTCCT TTCTGGGACT TCCACAAGTT GAGAACAGAC CATGCTGCCC TGGGCAGTTC 180
TTGAAGGCTG TGAGAGTGGG CCTGACAAAG TTTATTCCTT TTTTTTTTC CATTCNCTTT 240
TATNATNATA ATTGTTTGAA ATNGNCAANT AAAAATNATG TATATTTATG ANGTNCANCA 300
NGAAA 305

SEQ ID NO:2882
SEQUENCE LENGTH:303
SEQUENCE TYPE:nucleic acid
TOPOLOGY:linear
CLONE:HUMGS03423
SEQUENCE DESCRIPTION:
GATCAGGAAA GTCATACTGT TCACTTTCAA GNTGGCTGTC CCCCCCGCCG AGCCCCCCCA 60
CCCCCATATG TACAGATGAT ANTAGGGTGT GGAATGTCGT CAGTGGCAAA CATTTCACAG 120
ATTTTTATTT TGTTTCTGTC TNNANCNTTT TTGNCACTGT GCTAATAGTT ATATTCAGTA 180
CATGAAAAGN TACTACTGTG TTGAAAGCTT TTTAGGAAAT TTTGNCAGTA TTTTTGTACA 240
AAACATTTTT TTGAAAAAAT ACTGGTTANT TTATNCTATT TTAATTNGCC AATGTCAATA 300
AAN 303

SEQ ID NO:2883
SEQUENCE LENGTH:375
SEQUENCE TYPE:nucleic acid
TOPOLOGY:linear
CLONE:HUMGS03424
SEQUENCE DESCRIPTION:
GATCACCGGA CACAATCCTG CAGAAGGACA CACGACTCTA TTTCCTACAG TGCGAAACTT 60
GTCATTCTAG ATGTNCTGTT GCCAGTATCA AAACCGGCTT CCAGGCTGTC ACGGGCAAGC 120
GAGCACAGCT CCGTGCCAAA GCTAACTAAT TTGCTAATCA CTGATTTTNC AAAGCTTGTT 180
GTGGAGATGT GGCTGGACAG GTTTGCCATC AGAGTGGATA TACCGTTGTA TTAAAAACAA 240
GATAAAAAAG CTGCCAAGAT TTTTGGCGNG TGGTTGGTCT GAAGTCCTTG CAAGACGCTG 300
NTGCTCAAGC TGTTGACATA CTCATTGGCC TACTTTTAAC ACCTGTCCAG AGGAAACGTG 360
NTATGGGGGT AAGTN 375

SEQ ID NO:2884
SEQUENCE LENGTH:301
SEQUENCE TYPE:nucleic acid
TOPOLOGY:linear
CLONE:HUMGS03426
SEQUENCE DESCRIPTION:
GATCAGATAG GAGCACAAGC AGGGGACGGA AAGAGAGAGN CACTCANGCG GCAGCATTCC 60

CTNCCAGCCA CTGAGCTGNC GTGCCAGCAC CATTCCTGGT CACGCAAAAC AGAACCCATT 120
TAGCAGCAGG GAGACGAGAA CACCACACAA GACATTTTTC TACAGTATTT CAGGTGCCTA 180
CCACACAGGA AACCTTGAAG AAAATCAGTT NCTAGGNNNN GCTGTTACCT CTTGTTTACA 240
GTTTATATAT ATATGATAGA TATGNGNTAT ATATATAAAA GGTACTGTTA ACTACTGTAA 300
A 301

SEQ ID NO:2885
SEQUENCE LENGTH:297
SEQUENCE TYPE:nucleic acid
TOPOLOGY:linear
CLONE:HUMGS03427
SEQUENCE DESCRIPTION:
GATCGGGGGA GGGTAAATAA TGCAAAAATT GCACAGTGGA AGAAGGGGTC TCACAAAAAG 60
CAATCCATCC TGTAGTATAG GTANNNGNGT TGGGGGAAGC AGCTTCCATT CTGGATGTTT 120
GGAACCCTTT AGCTTTGTTT TGGAATGGCC CACCATTCTC ACTGGAAAAC AGTGGTCTGC 180
TGTGAAAGGC CAGCTCTCGG CAGCCCCTGT GGTTTCAGCG CTGCCGCTCT GTGTCATTCA 240
GGTTGTGCAC ATTGTTTTTC TTCTGACTTC CAGAAATAAA AGTGTTTCCA TGGGAAA 297

SEQ ID NO:2886
SEQUENCE LENGTH:296
SEQUENCE TYPE:nucleic acid
TOPOLOGY:linear
CLONE:HUMGS03428
SEQUENCE DESCRIPTION:
GATCTGGGTG CCTTGGGAGA NCCAGTCCTT CCTTTTNACC CACCCCAGGA AGATGGAGTN 60
CTCTTTNCTA GGCCCATGTC CTGCCAGCAA CCGGGATGCG TGGGCAACTG GACTCTGCAC 120
GGGGGTCTAC AGGTTGAGGG AGGTTGGTCA CAATNAGAAC CTCGGGGTTT GAGGTGGCCA 180
TGGGCAGACA GCCNNAAGGG AGGGAGGGTG TGGGTGTNCG TGTGTGCATG TCCTGGTNTG 240
TAAGGGGGAA AGGGTCTTTC CTGGTTTTAT TTAAATAAAG TAGTTTATGT AACAAA 296

SEQ ID NO:2887
SEQUENCE LENGTH:296
SEQUENCE TYPE:nucleic acid
TOPOLOGY:linear
CLONE:HUMGS03429
SEQUENCE DESCRIPTION:
GATCTGTGCN GTGGTCCTNA GGCAGTTGTN TCCACACAAG TACTACTTCC TCGTGGGCTA 60
CAGTNAAACT TTGCTGTCCT ACTTTAACAA ATGTCCTGTG CGACTCCACC TCCAAACTGT 120
GCCCTCAAAG GTTGTGTATA AGTACCTCTA GAACAATCCC CTTTTTNCCA TCAAGCTGTA 180
GCCTGCAGAG AATGGAAACG TGGGAAAGGA ATGGTATGTG GGGGAAATGC ATCCCCTCAG 240
AGGNCTGAGG CATAGTCTCT CATCTGCTAT TGANTAAAGA CCTTCTATCT TGTAAA 296

SEQ ID NO:2888
SEQUENCE LENGTH:356
SEQUENCE TYPE:nucleic acid

TOPOLOGY:linear
CLONE:HUMGS03430
SEQUENCE DESCRIPTION:
GATCTACTCT GTATGGGTAC CAAAATCACC TTCTCTCTTC AAGTCAAAGT TGAATTTAGA 60
ACTTTGAATA CAGGTCAAAA AATGTAGAAA ATATCATTTT ATATTCCCTA ATCTATATAG 120
CTGAAAAAGG GTTGTATTTT CTTAGCTGAA TATGAATACC TCTTAAACAT AAATTAGTTC 180
CTCTCCAGTG AAGTATCTTA TTTTACCAAT CCATTTCAGG AAAGAGGTTC TGCTGCCGTA 240
AAGGCAGAAA TTTTATTTTT ATCCCTTTTA TTTGAATGAG AGATTTGAAA ATCGAATTAT 300
GTAAATATTT CAATGCATCT GCTATTATTT TGTGGAGTTT ATTAAACTAC TTTAAA 356

SEQ ID NO:2889
SEQUENCE LENGTH:293
SEQUENCE TYPE:nucleic acid
TOPOLOGY:linear
CLONE:HUMGS03431
SEQUENCE DESCRIPTION:
GATCCCAAAC CAAATCTTAG AATCANTTCA TTTAAAATAC TGAGCGGTAT TGANTACTTC 60
GAAGCAGAAC AGGCAATGTG CAGCCCTCAT TTATGAGAAA ACCCTCAGGA AACTCCCAGG 120
GTGATGCTTG GNGAAGCTGT GAGTTGAGCT GAAGCTGGAG AACTTCCTCC AGAGCAAAGG 180
GCTTAAGAAA GAAAGANGAA CTCTAAGCTG GGTCTGCTAA CATCACTCCA GTTTAGATGG 240
NTCCTGTTTT TCCTTAACAN GTTGAGGCGT GGGTAGAGCA GGANTTGGTT TTN 293

SEQ ID NO:2890
SEQUENCE LENGTH:289
SEQUENCE TYPE:nucleic acid
TOPOLOGY:linear
CLONE:HUMGS03432
SEQUENCE DESCRIPTION:
GATCTGAAAT AACATTAATT TGATTGTAAC CCTATTGGGG TTAGTTCCCA CCCTTTGTTC 60
CATCTTGAGC AGAGATGNGC TGCATTTCTT AACGTTTGGT CTCTATACAT TATACCATCT 120
GCTGAAGTTT AGCTTTGTGT GTGTGTATAT GTACCCATGT GTGCACTTTA AATATCAGTA 180
CAAACTGATA AACAATGTTT CTGGTTAATG TTGCAAAAAT GGAACACTGT ATTTNACATA 240
ATTGATTTTC TACTCCTGCC CTGTGAATTA AGGCAGAGTT TTACTGTAN 289

SEQ ID NO:2891
SEQUENCE LENGTH:286
SEQUENCE TYPE:nucleic acid
TOPOLOGY:linear
CLONE:HUMGS03433
SEQUENCE DESCRIPTION:
GATCTTCCCA CACCAGCCTC CCAAAATACT GGGATTACAG GCTTGAGCCT CCATGCCTGG 60
CCCAGGTAAC ATGTTTATTG AGCTGTACAT GCATATGAGA AATAAGAAAC TTTTTTTTCC 120
TACTATCATC TCTTAAATNT NGTNTTCTTT TTCTTTTGCT TCCTCTTCTT CTNTNCTATT 180
TNTNATAAAT ATCATGCACA ACTATAACCT ATGGGAATNA TGTAGTAACA CAGATTATTC 240
ATCTTGTTAG AGTTGTATTA AAAATAAACA AGCATTTCAA ATTAAA 286

SEQ ID NO:2892
SEQUENCE LENGTH:286
SEQUENCE TYPE:nucleic acid
TOPOLOGY:linear
CLONE:HUMGS03434
SEQUENCE DESCRIPTION:
GATCCAAGAC CAGGGTGGTT GGGGGCTGCC CCTGGCCGAG TCACTGAAGC GACTNATGTC 60
CCTGTNTCCA GGACGANCTC CTCTCCTACT TTGGGACGCC CACGTGGCAG ACCGTGACCA 120
TCTTTGTGGC GGNAGTGCTC ACCGCCTCAC TCACCATCTG GAAGAAGATG GGCTGAGGCC 180
CCCAGCTGCC TTGGACTGTG TTTTTCCTCG GGTAANNNGN GGCATTNTTC TGGGAGGGGT 240
GGGGATTGGG GGACATGGGC ATTTTTCTTA CTTTTGTAAT TATTGN 286

SEQ ID NO:2893
SEQUENCE LENGTH:285
SEQUENCE TYPE:nucleic acid
TOPOLOGY:linear
CLONE:HUMGS03435
SEQUENCE DESCRIPTION:
GATCTGCNGG GGCNTGTNCA GCTGCAGCAT TCCTGAAAGA TTTCGTAACT CATCCTAAGT 60
GGGCACATTT AGACATAGCA GGCGTGATGA CCAACAAAGA TGAAGTTCCC TATCTACGGA 120
AAGGCATGAC TGGGAGGCCC ACAAGGNCTC TCATTGAGTT CTTACTTCGT TTCAGTCAAG 180
ACAATGCTTA GTTCAGATAC TCAAAAATGT CTTCACTCTG TCTTAAATTG GACAGTTGAA 240
CTTAAAAGGT TTTTGAATAA ATGGATGAAA ATCTTTTAAC GGAAA 285

SEQ ID NO:2894
SEQUENCE LENGTH:284
SEQUENCE TYPE:nucleic acid
TOPOLOGY:linear
CLONE:HUMGS03436
SEQUENCE DESCRIPTION:
GATCAGCTCT TTCTATTTTT ACTCGTAAAA ATTATGGAAA TAAATAATTT TGCTAACAAC 60
TTTGAAATTT CAAACTTCTG GAAAANATGA AAATATTCAT TGTTCATTAT GAATTTAAAT 120
TGTAAGGTAT GAATGTNATT TGTCTGTACA TCTTGTATCT TTTCCAAAAA ATGATTCTGT 180
ATCTTTTGGA AAAAAGCCGA GAGTTGAAGA TAGTATATTT CTGGTAGTAC TGAATATTTA 240
CTTACAGTTT CTATCAAAAA TATATATTTG TTTCTAAAAT TAAA 284

SEQ ID NO:2895
SEQUENCE LENGTH:283
SEQUENCE TYPE:nucleic acid
TOPOLOGY:linear
CLONE:HUMGS03437
SEQUENCE DESCRIPTION:
GATCTCCTTG TTAGTGGTGG TAGGGAGCTA GACAAGGATG GCAACTATTT CTGTATCTTA 60
CATACCTTTN ATTTNGAGGC CCTGTCAATG TTTTATATAA TANNCATTTT TTGAAAAGGC 120

AACTCTTAAA ACTAAAACAA ACTTAACAGT CTGTCAAGTT GGTGATATAA CCCCACAGAA 180
GACTTACTTC AAGTGACTTG AAAACTTAGT ATTTTGTCTG TNCTTTGCTA ATGGGNATAN 240
ATCCTACAGA CCAAACANCC NCAGNTAANT CTTAANCTGC AAA 283

SEQ ID NO:2896
SEQUENCE LENGTH:279
SEQUENCE TYPE:nucleic acid
TOPOLOGY:linear
CLONE:HUMGS03438
SEQUENCE DESCRIPTION:
GATCCCGGGA GACCTGGGGC TGCCGAAGAA GCTCTGCTCC AAATGGAGAG TGTAGCCTGA 60
GGGCTGGTGT TGCCTGCCTC CCCTGTNCTT GTNCCTTGTC CCAAAATAAA TCCTTTCAGA 120
ATGTAGCACT CACGNCCTAA TAAGGAGCGA ATCCTACATC CACCAAGGCG GGCGCTCTGG 180
CCCTCCCTTC CTTAAGCCCA GTCCTGTGTC CTCTGAAAGA GGTTGCAGTC ACTCACACCN 240
GCTTGCGCTC ACCATCAATA AAAGTAATTT CACCNGAAA 279

SEQ ID NO:2897
SEQUENCE LENGTH:278
SEQUENCE TYPE:nucleic acid
TOPOLOGY:linear
CLONE:HUMGS03439
SEQUENCE DESCRIPTION:
GATCCCCTCC CCCAAGATAC TCTTTGTGGG GAAGAGGGGC TGGGGCATGG CAGNCTGGGT 60
GACCGACTAC CCCAGTCCCA GGGAAGGTGG GGCCCTNCCC CTAGGATGCT GCAGCAGAGT 120
GAGCAAGGGG GCCCGAATCG ACCATAAAGG GTGTAGGGGC CACNNNNCTC CCCCTGTNCT 180
GTTGGGGAGG GGTAGCCATG ATTTGTCCCA GCCTGGGGCT CCCTCTCTGG TTTCCTATTT 240
GCAGTTACTT GAATAAAAAA AATATCCTTT TCTGGAAA 278

SEQ ID NO:2898
SEQUENCE LENGTH:276
SEQUENCE TYPE:nucleic acid
TOPOLOGY:linear
CLONE:HUMGS03440
SEQUENCE DESCRIPTION:
GATCTGCTTA CCAAGCATAT TAGGAAATAC CTCTTAGGAA GCATTAGCGG TCTCAGGCCA 60
ATTACTGTGG AGCAGCTTTC ATTCCTACCC ACTTGCAAAC CTTGGCGCTG TTGTCTGAGA 120
TTGCTGCAGC CATTCTTGTT ACCATGGTAC TTCTCAAACT TTGTGAAAAC CTGCACTTTT 180
CCTTGCATGA CAGGTTCCTG TCTTGTCTGT CATGGGAGCC ATTCTGCCAA TTTAAATGCG 240
ACTGTGGTAT AAACAGTAAA ATGATTTAAA AGTAAA 276

SEQ ID NO:2899
SEQUENCE LENGTH:274
SEQUENCE TYPE:nucleic acid
TOPOLOGY:linear
CLONE:HUMGS03441

SEQUENCE DESCRIPTION:
GATCAGTGTT ATATTTTACT GGAGAAGCTA TTGAAGATGA TGATGATGAT TATAATGAAG 60
AAGGTGAAGA AGCGGATGAG GTAATGTTTA CCAAATAAGC AAATAATTCT NTGTGGTTAA 120
CACATTGAGA AGCAAATTGA ATNACTTATG TATTCCTTTG CTATAATCAT TCTGTGATTT 180
GGGATAAAAA CATTTTGGAA AACATGATTT TAGAATTCTA CAGCCAAACA ATGTATGTAT 240
AAGGTTTCAA TAAATAANTT GCAAAACTCA GAAA 274

SEQ ID NO:2900
SEQUENCE LENGTH:273
SEQUENCE TYPE:nucleic acid
TOPOLOGY:linear
CLONE:HUMGS03442
SEQUENCE DESCRIPTION:
GATCCCCACC TGCCATCCTA TAGTGTTGTC TTCCTGTGTG TTCCGGGGCT TCTGGGCAGC 60
TGGGCCTGCC CGGGGAAGTC CTTGCAGGTG GGAGGCCATA CAGAGACCCA CTGTGTGCCA 120
CTGAGCGTCC CACTGCTGCT GGGCAACTGG AGGACTGCAG GGGGCGCCAG GTGACTCTCT 180
CCTTTTATAT CACAGCAGCT CCTGTGCTGA CCTTCAAGTT ACGTTTTGGA ACTGTAATAC 240
TAAAGGAAGA AATAAACTAC TAATTTGTAT AAA 273

SEQ ID NO:2901
SEQUENCE LENGTH:359
SEQUENCE TYPE:nucleic acid
TOPOLOGY:linear
CLONE:HUMGS03443
SEQUENCE DESCRIPTION:
GATCTGGTTA AGTTGTGTAG TAAAGCATTA GGAGGGTCAT TCTTGTCACA AAAGTGCCAC 60
TAAAACAGCC TNCGGAGAAT NNNTGACTTG CTTTTCTAAA TCTNAGGTTT ATCTGGGCTC 120
TATCATATAG ACAGGCTTCT NATAGTTTGC AACTGTAAGC AGAAACCTAC ATATAGTTAA 180
AATCCTGGTC TTTCTTGGTA AACAGNTTTT AAATGTCTGA TATAAAACAT GCCACAGGCG 240
AATTCGGGGA TTTNAGTTTC TCTGAATAGC ATATATNTGA TGCATCGGAT AGGTCATTAT 300
GATTTTNNNC CATTTCGGCT TCATAATGNA AACCAGTTCA TTTTACCTAT CAGGTTATN 359

SEQ ID NO:2902
SEQUENCE LENGTH:268
SEQUENCE TYPE:nucleic acid
TOPOLOGY:linear
CLONE:HUMGS03444
SEQUENCE DESCRIPTION:
GATCAGAGCA CAGTTGAAAT TCATGGAACT GGCATTATTT AAGCAACCAG AATTCCACAC 60
TGTAGGAAGG TTTTGAAAAT TGTTTCGTTA TAGATTTTAT CTTATCTTTT GGTGTTGTCT 120
TGGTTTTTCT CATAGACTTT ATAAGTTTGA ACTGGACTTA TTTGATTATA ACCACAAATT 180
GTGTGTGTGT GTGTGTGTGT ATATATAGAA GTCATTATGG CAGATGCACA AATTGTGCAG 240
TGATGTAAAT ATACATACTT CACAGAAA 268

SEQ ID NO:2903

SEQUENCE LENGTH:264
SEQUENCE TYPE:nucleic acid
TOPOLOGY:linear
CLONE:HUMGS03445
SEQUENCE DESCRIPTION:
GATCGGTAGT GTTGTGTGTA GGCCATTCTT CTGGAGAGCC ACAAGCAGGA AGAGCAGCGC 60
TGTGTTGCAG AATGGAGTTC CATGGATTTC TACCAGACCA CTGAAGGAGT TCCTNGAAGC 120
CCTGCGGTAG CTAGCACTGA AGACTATTTT NCTATTGGTA TAACCNGCCC ACCTGAAGGG 180
GAAAGGGGAA ATCAANTTAA TTTTCTCGTT AGACATANGG AAATTTAAGG AAAAACAGNT 240
TTAGGANCAG TTACTCAGCG TAGN 264

SEQ ID NO:2904
SEQUENCE LENGTH:258
SEQUENCE TYPE:nucleic acid
TOPOLOGY:linear
CLONE:HUMGS03446
SEQUENCE DESCRIPTION:
GATCCATAGT GAAATTGCCA GAGTTTTGCC TGCTGCTTTC CTCGTGGCCT CTTCTTGGGT 60
AGTGAAATNA AGTGACATTT GGATTTTAAT TTGGGTGGGA GGGCTGGGAC AGTTTTTNTT 120
CTAGAAATGT CTGTTGAGAT TTCCCCCTTT AGTTTCCAAC CTTCTCCCCA ACCCTTGGAG 180
CTAAATGCGT TGTAAAATAT TGCCAAAATG AAAAGTGTTT TGTAATACTG CAATAAAGGC 240
TGCTTGTTTT TGTGGAAA 258

SEQ ID NO:2905
SEQUENCE LENGTH:264
SEQUENCE TYPE:nucleic acid
TOPOLOGY:linear
CLONE:HUMGS03447
SEQUENCE DESCRIPTION:
GATCGGACAT GAAAGNACCC TGNGAGTCGA TTGTCCTANC TCCAGCGGCC CTGTCATCCA 60
GCTCACTCAT CAATGGGGCC AGTCAGGCCC AGGCACTGGG CTCCGGAGGA CTCACCACTG 120
CCCCCTGCTG CCATGTGGAC TGGTGCAAGT TGAGGACTTC TTGCTGGTCT AGTCACGCAT 180
GCAGTGTTGG GGGATGCCTT GGTTTTTACT GCTCTGAGAA TTGGTTGAGA TACTTTTACT 240
AATNAAACTG TGTAGGTTGG GAAA 264

SEQ ID NO:2906
SEQUENCE LENGTH:257
SEQUENCE TYPE:nucleic acid
TOPOLOGY:linear
CLONE:HUMGS03448
SEQUENCE DESCRIPTION:
GATCTGCTTT CAACACTTTT AGCTCTTCTA GAATTTCAGA AAAATGGTAT CGGATGTACA 60
GCATGGACCC TTCTNTGGTG GGCTTCTTTA CTGTAATGCT TTTAANATTC ATCTGTGCTG 120
TTNAGTGTCT CTGCAGTCCA TTCCTTCTGT TGAGTGTATC AGTAGTCCAT TCCTTTTCAT 180
GGCTGAGTAG TATTTCATTG TATAATTATA CCACTATTTG TNCATTTACT AGATAATANN 240

CAGTTGGCTT GTTTAAA 257

SEQ ID NO:2907
SEQUENCE LENGTH:256
SEQUENCE TYPE:nucleic acid
TOPOLOGY:linear
CLONE:HUMGS03449
SEQUENCE DESCRIPTION:
GATCCATCCG CCTTGGCCTC CCAAAGTGCT GAGATGACAG GCATGAGCCA CTGCACCCGG 60
CCATATAATC AAATTTTTAT TGATTTTAGA GCAAAGGACT TTTTCAATGT AACTGCTTAG 120
AAATCGAGCA ATTTTCTATT GTATAGAGTG AAAGTCAAAC TTGTTTATTG TACAAGTCTT 180
TAAAGGGATA GTTAGGAGTA AGTTGTATTT GCTGAATATA TAGACCTCAT TTGTAGCCAT 240
TAGTGATTCT GTATTN 256

SEQ ID NO:2908
SEQUENCE LENGTH:256
SEQUENCE TYPE:nucleic acid
TOPOLOGY:linear
CLONE:HUMGS03450
SEQUENCE DESCRIPTION:
GATCTAATTA AAAAAAAGGC AATATTTTTA TATTAAAGTA CTATACTAGG AGAGAATGTT 60
TCANAACTCC CTGATGAATT TCTAAGTGAG CAACTTGATA TAAAATTGTA ATCTTCATTT 120
TTGTCAGTGT ATCCAGTTAC AGAATGCTAC ACACTTACCT TTTTATTGGC TGAGAAATCT 180
GGTTATTTCA TCTTAATCTC AAGATTGTTT TCAAGTGTTT TATAATTAAA TCATAATAGC 240
ATATTTTAAA ATCAAA 256

SEQ ID NO:2909
SEQUENCE LENGTH:255
SEQUENCE TYPE:nucleic acid
TOPOLOGY:linear
CLONE:HUMGS03451
SEQUENCE DESCRIPTION:
GATCAAACCA GCTCTTNTNA CCCAAACCAN TGCCGCTGCA TTAAAGGGAG GCCCAGCTAC 60
TCCTTCCCAG CTGAGATGTG GGTACAGGTC AGATTTNTCA GGGAGATGGC ACTAACACGA 120
CACAAGCTCA CTCCCAGAAC CGCAGCTGAT GTTTATAATA ATCGTATGTA CTGTGCAATT 180
TAGGAGGGGA AAAAAAGGCT GTACAAGCTT TAACTCTAAA NNAAATNTCT ATTTTTTNCC 240
TCTTGCAAAN TTAAA 255

SEQ ID NO:2910
SEQUENCE LENGTH:254
SEQUENCE TYPE:nucleic acid
TOPOLOGY:linear
CLONE:HUMGS03452
SEQUENCE DESCRIPTION:
GATCTGCCCT GTACAGCCAT GTAGGCTGTG CGCTGCATAA CTCCAGGGAC ATGAGTCACA 60

CAGACACAAT GTGAGTGTGC TCCCCCGTCA TGCAACATCT GGACACAACT AACAGAGCAT 120
GGTGAATACA TGCTGAATTG CATTCAGTAT GGCTGTGAAC TAGGCCTGGG GACAAGANTG 180
ANTTTTACAT GGAAAGAATT TCCTGTAGCA GGAACAGAGG GGATAACAAC AGCAGTAAAT 240
AATAATAAGN CAAA 254

SEQ ID NO:2911
SEQUENCE LENGTH:253
SEQUENCE TYPE:nucleic acid
TOPOLOGY:linear
CLONE:HUMGS03453
SEQUENCE DESCRIPTION:
GATCCCCCAC TAGTNCCCTC CCTCTNCCCC TGTAATCCTG GTCTAATAAC CCCCCACACA 60
TACACCTCTG GTGACCTATT TGCACAGACC GTNGTCTTCC CTCCAGTNTT CTNAGGGATA 120
GGGGACATTC CATCCCAAGC TTCTNCCTTA CCCACACCTA TCCTTTTNAG GGGCTTTGGG 180
GTGGGGCTGG GGCAAGCAGA GGGACTGGGT CTTCACTTCT NGGGCTAATA AAATTGTTTC 240
TTTGTGGACT AAA 253

SEQ ID NO:2912
SEQUENCE LENGTH:253
SEQUENCE TYPE:nucleic acid
TOPOLOGY:linear
CLONE:HUMGS03454
SEQUENCE DESCRIPTION:
GATCAAACCG GTTCTNTCCT TTCTTGTGTT GCCATGTTAC TATGCCTCAA GCCCAGTTTN 60
CTTTTNCCGC AGCGATGGGG CCAGTCTCAT TCCTCCCCAG GAGTGAAACT NGCTTCANCT 120
GAAAAGGTTG GGTGCATTGT CAGTAAAAAG GGCTTATTTG TTTCATTTNA CTTTCCTGCA 180
AAATTTTCTT CAAAGCAACA AGTCCTAGGN GCACACAAAG CAACCCAAAG GCTTTTCCCT 240
GGAAAAGCTC TTN 253

SEQ ID NO:2913
SEQUENCE LENGTH:254
SEQUENCE TYPE:nucleic acid
TOPOLOGY:linear
CLONE:HUMGS03455
SEQUENCE DESCRIPTION:
GATCCGGGGC ATGCAGAAGC TGAGCACACC CCAGAAGAAG TNAGGGTCCC CGACCCAGGA 60
GAACGGTGGC TCCCACAGGA CAATCGCTGC CCCCCAACCT CGTAGCAACA GCAATACCGG 120
GGGACCCTGG CGGCCAGGCC TGGTGCCATG AGCAGGGCTC CTCGTGCCCC TGGCCCAGGG 180
GTCTCTTCCC CTGCCCCNTC AGTTTTNCAC TTTTGGGGTT TTTTATTGNT ATTAAACTGA 240
TGGGNCTTTN TAAA 254

SEQ ID NO:2914
SEQUENCE LENGTH:249
SEQUENCE TYPE:nucleic acid
TOPOLOGY:linear

CLONE:HUMGS03456
SEQUENCE DESCRIPTION:
GATCCTTGTT TTGTTGTCAT GGAAACTTCG GGGCTGGTGG GACTTAGGCC AAAAGCTCAG 60
AGGCACAGCC AAAATTTAGA AGCTTGCTAC TCCTACGACT CGGCCTATAA GGAAGAGAGA 120
AGCTGTCTGT ACTTTGGGGA CTACATTGCT GAAGGAAAAA AATCACTCCC TGGCTAATTA 180
AGATTGCTTC CAAATTGGGG GAATGTGTGT CATTTCCTTT ACCAAGGCCA GTCATCCCTG 240
CTTCCACCN 249

SEQ ID NO:2915
SEQUENCE LENGTH:249
SEQUENCE TYPE:nucleic acid
TOPOLOGY:linear
CLONE:HUMGS03457
SEQUENCE DESCRIPTION:
GATCCAGGCC GGGCCTATAC AGAGGTGCTG GCTGCTTGTT TACATTCTCC TCTGGGGCTC 60
TACCTCTCCA CACTTCCCCA GAAGGGAAAA GGGCACCCTG GATTACTNNN TTGGAAATNA 120
CTCCTTGGTG GGCAGCATCC TGAGGGTTCC CCAGAACCAG GCCTCTGCTC TGGCCCTCTT 180
GCATCTGGAA CGCCAGGTGG GTTTTTNTGG CATAGGAGCC CACTTGCATT TTCATAGTTT 240
TATTTGATN 249

SEQ ID NO:2916
SEQUENCE LENGTH:249
SEQUENCE TYPE:nucleic acid
TOPOLOGY:linear
CLONE:HUMGS03458
SEQUENCE DESCRIPTION:
GATCACAGCC TCCAGGGCCC CCCAAATCCC AGGGAAGGAC TTGGAGAGAA TCATGCTGTT 60
GCATTTAGAA CTTNCTNCTT TGCACAGGAA AGAGTCACAC AATTAATCAA CATGTATATN 120
TCCNCTATAC ATAGAGCTCT ATTNCNCTNC GGTTTTATAA AAGCCTTGGG TTCCAACCAG 180
GCAGTAGATG TNCTTCTGAN CCGCAAGGNG CAAACACTGA AATAAAATAG TTTATTTTNC 240
ACACTCAAA 249

SEQ ID NO:2917
SEQUENCE LENGTH:249
SEQUENCE TYPE:nucleic acid
TOPOLOGY:linear
CLONE:HUMGS03459
SEQUENCE DESCRIPTION:
GATCTCCTTT TGTGAAAACC AGTTTGATGT GCTAAAAGTA AAAAGTCTAN TTTCCAGTGT 60
GGTCTTGTTC AGAAGCAGCC AGATTTCCAA TGTTGTTTTT CCCCTCCACT CAGAAACCCC 120
TGCCCTTTCC CTTCAGAAAA CGATGGCAGG CATTCCTCTG AGTTTACAAG CAGAGACTCA 180
CTCCAACCCA AACTAGCTGG GAGTTCAGAA CCCATGGTTG GAATAAAGAA ATTGTGCGTC 240
TTGGTCAAA 249

SEQ ID NO:2918

SEQUENCE LENGTH:245
SEQUENCE TYPE:nucleic acid
TOPOLOGY:linear
CLONE:HUMGS03460
SEQUENCE DESCRIPTION:
GATCTATCCC TGCGGNCTCC ACACCTGAAC TTGCCTAACT AACTGGCAGG GGAGACAGGA 60
GCCTAGCGGA NCCAGCCTGG GAGCCCAGAG GGTGGCAAGA ACAGTGGGCG TTGGGAGCCT 120
AGCTCCTGCC ACATGGAGNC CCCTCTGCCG GTCGGGCAGC CAGCAGAGGG GGAGTAGCCA 180
AGCTNCTTAA TCCTNAGCCT NCCCCTGTGT ATTCACCACC AATAAATCAG ACCATGAAAA 240
CCAAA 245

SEQ ID NO:2919
SEQUENCE LENGTH:384
SEQUENCE TYPE:nucleic acid
TOPOLOGY:linear
CLONE:HUMGS03461
SEQUENCE DESCRIPTION:
GATCAGGAAA CCAGAAATAC CACATTTATG GACCATGAAN GGTTGGTTCT TGACTCTGAA 60
GGGACTTTTG AGTTAATCAG CGTAAGGGGA TTTCTAAAGC AGGCAATCCC TGTAGCCGCA 120
GAGAATAAAC GCCTTCCCAA AATGGNAACT NCCCACAGCC ACATTTCAGA CCTGCTGAGA 180
CTGCTGAGTG AGGAATGGCA GTGAGGTTTC TTCAATNAGT CTCAGTTCTC TTAATTTTCA 240
GGAAGAAAGG GTAATTGCAG CCCCTCAGCC CCCAGGATTG ACCTCTNGGG AGNCATGGTA 300
GCGTTGTGTG CCAGGCCGTN GGTTCAGGTG TTNGCAGAAG CTTTCAGATT CGTCCGAAGG 360
GAAATAAAGT GTGTTGGCGT TAAA 384

SEQ ID NO:2920
SEQUENCE LENGTH:240
SEQUENCE TYPE:nucleic acid
TOPOLOGY:linear
CLONE:HUMGS03462
SEQUENCE DESCRIPTION:
GATCCCAGCA GCCCTCCCTT CACCGTGACC CCTGACCTTT GTTAGGAAGG TGCAGTTTTT 60
NTTCTCAATC TAAATNCCTT TNAGGTGGGC CGCTTCCTTG GCTACCTGGT TCCAGGGGGC 120
TGTTTTGTAA TNAGATGCTG CTGGCAGGCC ACTCAGAGGC TCCCAGCTGG GTTGGTGGGA 180
CAGCCAGGCC AGATGACCTG ATTCCAGCAA AAATAAAACT CAGATTTGGG CAAAATGAAA 240

SEQ ID NO:2921
SEQUENCE LENGTH:238
SEQUENCE TYPE:nucleic acid
TOPOLOGY:linear
CLONE:HUMGS03463
SEQUENCE DESCRIPTION:
GATCCCTAGC AGCTCATCCT GCCCTTTGAA TACCCTCACT TTCCAGGCCT GGCTCAGAAT 60
CTAACCTATT TATTGACTGT CCTGAGGGCC TTGAAAACAG GCCGAACCTG GAGGGCCTGG 120
ATTTNTTTTT GGGCTGGAAT GCTGCCCTGA GGGTGGGGCT GGCTCTTACT CAGGAAACTG 180

CTGTGCCCAA CCCATGGACA GGCCCAGCTG GGGCCCACAT GCTGACACAG ACTCACTN 238

SEQ ID NO:2922
SEQUENCE LENGTH:238
SEQUENCE TYPE:nucleic acid
TOPOLOGY:linear
CLONE:HUMGS03464
SEQUENCE DESCRIPTION:
GATCACCGGT TTCCGACTGA GTCTGGGGAT TTTGGCCTTG TTGACCCTCC TAGGTGCCCT 60
GGGAATTGCA AACAGCTTTC TGGATGAATA TCTGGACCTC AATATTGCCA AGAAACTGAG 120
GCGGCAATTC TAACTTTTTC TCTTCCCTTT AATGCTTGCA GAAGCTGTTC CCACCATGAA 180
GGTAATATGG TATCATTTGT TAAATAAAAA TAAAGTCTTT ATTCTGTTTT TCTTGAAA 238

SEQ ID NO:2923
SEQUENCE LENGTH:238
SEQUENCE TYPE:nucleic acid
TOPOLOGY:linear
CLONE:HUMGS03465
SEQUENCE DESCRIPTION:
GATCTACTGT GTGGTGTCTG GGCAGATGAA AGTACACTGC TTAGNGTGAT GCTTAGCATG 60
GGGCAGATGC TTGGTAAATT GTAATCACTT TTGTTGTCAG TTAAATGGAT ATTTCACTGT 120
TGCCTCTNAA ACGCTTACGG TAATATATAC TAATTATACA TGTTATTATC AGTTTATATT 180
TATTGAACAT TTCGGGGAAG TAATTATAAA ATAATCTTGT CTACTGTCTT ACCCCAAA 238

SEQ ID NO:2924
SEQUENCE LENGTH:347
SEQUENCE TYPE:nucleic acid
TOPOLOGY:linear
CLONE:HUMGS03466
SEQUENCE DESCRIPTION:
GATCTATGGT GCAGACTGCC TTCGTCGGGT GCAGACTGAG CGACCGNAGC CCCAGACACT 60
ACAGCTGGAT TNCTTGATGA AAATCTTGCC AAATTACCAC CACCTAAAGA AGACTATGNA 120
AGGAAGCTCA ACTCCAGTAA AGAACTAAGA ACAGTGTATA ACATGAAGAT AACATTTTGT 180
CTTTGACCAC TGTCTTTTGA ATGGGCCCAC AGTGTTTATG TACTCTTAAC AACTCACAGA 240
ATAATACATG TTCACTTTAT TTTGTAAAAT TGGGTTGAGA GGAAACTAAT GGAGTTTCAT 300
TGTAACTGTC CTTTNTAATT TATATAAATG TATTATTNTC CTATAAA 347

SEQ ID NO:2925
SEQUENCE LENGTH:236
SEQUENCE TYPE:nucleic acid
TOPOLOGY:linear
CLONE:HUMGS03467
SEQUENCE DESCRIPTION:
GATCCTGTGA TATGGTTTTN GGCCCAGCAA ATCTAGGAGA AGATGCAATT AAAAACTTCA 60
GAGCAAAACA TCACTGTAAT TNTTGCTGTA GAAAGCTTAA ACTTCCAGGT AAAAAATTTT 120

TTAATCAGTA TAGTATATAA TTNGTTACGA AACTNGGTTA CTACAATACC TCATAAGGCC 180
AGAGGTACAA NTGTTTAATG TAATGGTCTC TAATTGTGCC AGCTTCAGCA GCTCTN 236

SEQ ID NO:2926
SEQUENCE LENGTH:271
SEQUENCE TYPE:nucleic acid
TOPOLOGY:linear
CLONE:HUMGS03468
SEQUENCE DESCRIPTION:
GATCCCGCAT GCCTGAATTC ACTAAAGCCA AGGGTCTNTA AGCCACGCTN NNTCTTCTGA 60
GACTTCCATT CCTTTCTGAT TGGCACACGT GCAGCTCATG ACAATCTGTA GGATAACAAT 120
CAGTGTGGAT TTCCACTCTT TTCAGTCCTT CATGTTAAAG ATTTAGACAC CACATACAAC 180
TGGTAAAGGA CGTTTTCTTG AGAGTTTTAA CTATATGTAA ACATTGTATA ATGATATGGA 240
ATAAAATGCA CATTGTAGGA CATTTTCTAA A 271

SEQ ID NO:2927
SEQUENCE LENGTH:235
SEQUENCE TYPE:nucleic acid
TOPOLOGY:linear
CLONE:HUMGS03469
SEQUENCE DESCRIPTION:
GATCTAAAAT GTTTATTCCT TCTGTAGTGT ATTAATCTCT GTGTGTTTTC TTTGGTTTTG 60
GAGGAGGGGT ATGAAGTATC TTTGACATGG TGCCTTAGGA ATGACTTGGG TTTAACAAGC 120
TGTCTACTGG ACAATCTTAT GTTTCCAAGA GAACTAAAGC TGGAGAGACC TGACCCTTCT 180
CTCACTTCTA AATTAATGGT AAAATAAAAT GCCTCAGCTA TGTAGCAAAG GGAAA 235

SEQ ID NO:2928
SEQUENCE LENGTH:234
SEQUENCE TYPE:nucleic acid
TOPOLOGY:linear
CLONE:HUMGS03470
SEQUENCE DESCRIPTION:
GATCCATCTN CCCTCATAAA AGTGTTCAGG TACAGCAGCT GAGGCTGCCC TGAGGAATCA 60
NGGGGCCATT ACCAAGGGGC AGGTAAAGGG TATNTAAGAG GTGGCCTTCA TGGTAGAGCT 120
TGACCCAAGA NCTACTCCAC ATTCGGNTNG CCCAGACTGA CTCCATCCCC TGANTTTCCC 180
TTNGACTTCA CCCTGTTTGT AAATAAAACA NTAAAATGGA AGGTGCTNTG GAAA 234

SEQ ID NO:2929
SEQUENCE LENGTH:233
SEQUENCE TYPE:nucleic acid
TOPOLOGY:linear
CLONE:HUMGS03471
SEQUENCE DESCRIPTION:
GATCTCAAAT CCTGGAACCC CGATTTCAAT CTACGTTCTA GTCACTGGCC TCAAAGGACC 60
CCACAGCACC TGGGCCAGAC CAACAGCTCG AGGGAGAACC TGAAGGCCCA GGGGGTCCAG 120

GGCGGACCTG GGGCCCCGAC CACCAAGGAC AGCTCACGAC TGCCCCTTNA CTGCATGTCC 180
CCAAACTNAG CATGACTCCT GTCCTCTTCA ATAAAGACGT TTCTATGGCC AAA 233

SEQ ID NO:2930
SEQUENCE LENGTH:231
SEQUENCE TYPE:nucleic acid
TOPOLOGY:linear
CLONE:HUMGS03472
SEQUENCE DESCRIPTION:
GATCAGCAGA AAGCACAAAC ACAACTGCTT CTGTATTCAG GAGGTTGTGA GTGGGCTGCG 60
GCANCCGTTG GTGCCCTGCA TAGTGGGGAT GGCTCGCAAC GTCTCTTCAT TCTGGAAAAA 120
GAAGGTTATG TGAAGATACT TACCCCTGAA GGAGAAATTT TCAAGGAGCC TTATTTGGAC 180
ATTCACAAAC TTGTTCAAAG TGGAATAAAG GTTGGCTTTT TAAATTTTAA A 231

SEQ ID NO:2931
SEQUENCE LENGTH:231
SEQUENCE TYPE:nucleic acid
TOPOLOGY:linear
CLONE:HUMGS03473
SEQUENCE DESCRIPTION:
GATCAGCAAA AATAAAGGGG CTACAGAAAC ACTCATTTTT ATGCTGTTCC CTCTTGGGCT 60
TCATGCAAAG ACAATTCTNT GTAAATNTAC AGTTGACTCT GATTTGGAAA TATGAAAATC 120
AGTCCATCCT TGTTATAAAA AATTTTTTTA CAATTGTAAT TATATTGATG TTCATATTGT 180
GTAAAATAAC TCATTTAATA AAATAGTACT TTGATTTACG ACATCAATAA A 231

SEQ ID NO:2932
SEQUENCE LENGTH:230
SEQUENCE TYPE:nucleic acid
TOPOLOGY:linear
CLONE:HUMGS03474
SEQUENCE DESCRIPTION:
GATCCCACCC CCAGCCATTT GCATTGCTGG CCCAGCGCCT GGCCTGGGGG GCGGGGAGAG 60
GCAGCAGAAG GGGCTGGGCA GGGGCGGTGG AGGACTCAGG AACTNCCCGG GGAGAGTGGG 120
TATGGCGGCT GAGCCAGGGN CCCTCCTGTN TTTNACTTCC CGGGATGGGT CCTTGCTTNN 180
AAGCTGTNTC CGACCCCACC ATNTAATAAA ACCCAAAGGA ACAGCCCAAA 230

SEQ ID NO:2933
SEQUENCE LENGTH:328
SEQUENCE TYPE:nucleic acid
TOPOLOGY:linear
CLONE:HUMGS03476
SEQUENCE DESCRIPTION:
GATCAGCCGG AAACCCCTGG CAGCCAAGAA CAGATAAGGA AGGGATTGGC ATCGGCTGGC 60
CTTCCAGCAC CTTCTCTCTC CAACACTTCA TTCTCTCTTG CCCTGTNTCT CAAATAAACC 120
CAATGCTGCG TGTGAGGCCT TTTTTATTTT TCTTTTCACT CTCTTTCTAA TGCTTCCCAC 180

CTTACCTTTT AGATTCTTTT GCTAGGTGGG AGATTGTTAT AAGGTCTTTA AACCATTTCC 240
ATTTGTTTCT TTAACATTAC CAAAAGCAGG GNACAAAGCT CTTATTCAAC TGCGNATTCC 300
ATAGNGGGCT CTGGNTTNTT CTTGAATN 328

SEQ ID NO:2934
SEQUENCE LENGTH:220
SEQUENCE TYPE:nucleic acid
TOPOLOGY:linear
CLONE:HUMGS03477
SEQUENCE DESCRIPTION:
GATCTTGCCC GGTGTCCTGG TCCTCTTGCT TCCGTCGCGG CCGCATGTGC GTGTGTCCAA 60
GCAGGTCCTG GGCGCCTCAA CTGCTGCCCC TGGTTGAATG TTCTCTTGAT AGTGCTGGAC 120
CCTTTGTCTA TTTNAAAGCG AATTTTGTGT GATTTCCTGC CCTTTGCGTT ATATTGTATA 180
ATACCAACGT AAGGAAATAA ACCTTTGGAA TTGTTGGAAA 220

SEQ ID NO:2935
SEQUENCE LENGTH:219
SEQUENCE TYPE:nucleic acid
TOPOLOGY:linear
CLONE:HUMGS03478
SEQUENCE DESCRIPTION:
GATCCGAGGG CGATGGGACA GTGCACCGCA GATAGAAGAG GAGGACGTGC AGCATGGTGG 60
GAAACGGGGA CACCACCTCC CAGGACGACT GTTTCAGCAA AGAGCGCANT GCTCCAGGTG 120
ACCCAGCAAG GCTGTTGTNT GTATGGAAGG ACACGCTCGC GGCAAGGGCA GGGCCTGGGN 180
AGGGTGGCCT GTCCAGTCCT NNAGACAAGG GGAGGCCTN 219

SEQ ID NO:2936
SEQUENCE LENGTH:218
SEQUENCE TYPE:nucleic acid
TOPOLOGY:linear
CLONE:HUMGS03479
SEQUENCE DESCRIPTION:
GATCACCTGA GGTCAGGAGT TCGAGACCAG TCTGGCCAAC ATGGTGAGAC CCCATCTCTA 60
CTAAAACTAC AAAATTAACC AGGTGTGGTG ATGCATGCCT TTNACTCGGG AGGCTGAAGC 120
AGAAGAATCG CTTAAACTCG GGAGGCGGAG GTTGCAGTGA GTCCAGATTG TGCCATTGCA 180
TTCCAGCCTG GACAAGAGGA GCAACACTCT GTCTCAAA 218

SEQ ID NO:2937
SEQUENCE LENGTH:218
SEQUENCE TYPE:nucleic acid
TOPOLOGY:linear
CLONE:HUMGS03480
SEQUENCE DESCRIPTION:
GATCATGCCT GCGTGACAAA GCCTCCAGAA AAATCCTTGA AAGACAGGAC ATGGAGAGCT 60
GCTGGGTTGG CGAACACATC CATGTGCCGG GAGGATGGTG CACCCCAACT CCACAAGGAC 120

CCTTCCAGAC CTCACCCTGT GTATNTCTTC ATCNNNNTGT TCATTTGTAT CCTTTAAAAT 180
ATCCCCTGTA ATAAATCAGC AATAGTAAGT AAACTAAA 218

SEQ ID NO:2938
SEQUENCE LENGTH:218
SEQUENCE TYPE:nucleic acid
TOPOLOGY:linear
CLONE:HUMGS03481
SEQUENCE DESCRIPTION:
GATCTAAACA GTGAATAGTA CATGCAAAGG CATAGTCTAG AGGATAAAAG TGAAAATAAT 60
TAGTAGCAAA ATNAATGCCT TGTGGATGAG GTTTAGGGGC CAATAACTAT CTTGAGGTAA 120
GGCCAGGTTG TGGCTGCCTT TGTGTTGTAC TAAGGGGTTT GGACCTTCCT AGGAGATTAG 180
AGCTTNATTA AAGTCACAAC ATCCATAAGN GAAATAAA 218

SEQ ID NO:2939
SEQUENCE LENGTH:218
SEQUENCE TYPE:nucleic acid
TOPOLOGY:linear
CLONE:HUMGS03482
SEQUENCE DESCRIPTION:
GATCCCTTTG CTGTGAGGGT AGAAAACCTC ACCAACTGCA CCAGTGAGGA AGAAGACTGC 60
GTGGATTCAT GGGGAGCCTC ACAGCAGCCA CGCAGCAGGC TCTGGGTGGG GCTGCCGTTA 120
AGGACGTTCT TTCCTTACTG GTGCTGATAA CAACAGGGAA CCGTGCAGTG TGCATTTTAA 180
GACCTGGCCT GGAATAAATA CGTTTTGTCT TTCCCAAA 218

SEQ ID NO:2940
SEQUENCE LENGTH:216
SEQUENCE TYPE:nucleic acid
TOPOLOGY:linear
CLONE:HUMGS03483
SEQUENCE DESCRIPTION:
GATCTTAGAT TTGATGAAGC ACAGTATGCA GGTAGGCCTA ATGGGGGAAG ATGGTAATAT 60
AAAAGCAAGA AGTATTTTTT TTTTGNAATG NCTGAAAGCT GTTCTGTGGA TGACCTACCG 120
NTTCCTTTAA ACACGATTCT CTCACTTCCA ACTCCAAACT TGCTCAACTA ATCCTTAAAA 180
ATAAACTTGA GCTGGAATTT GAGGCTTGCC TGGAAA 216

SEQ ID NO:2941
SEQUENCE LENGTH:213
SEQUENCE TYPE:nucleic acid
TOPOLOGY:linear
CLONE:HUMGS03484
SEQUENCE DESCRIPTION:
GATCTCGGGG GTGTGGGGCC CTGTGCCTTC CTGAAGTGCT GGCAGCCCAG TGGCACCTCC 60
TTCAGGCCTT TGGGGTATTC CCCTAGTGTG CCCAAGTNAG CCTCATATTC TGGGCGGACA 120
GCTTGTCTGG ACTTCGGAGT TGGGGGTGGT CAGACACCAC AGGAGCTGTC ACCTCCTGCG 180

GATGGGCAAA TAAATTNGTG GAGGACNCCG AAA 213

SEQ ID NO:2942
SEQUENCE LENGTH:212
SEQUENCE TYPE:nucleic acid
TOPOLOGY:linear
CLONE:HUMGS03485
SEQUENCE DESCRIPTION:
GATCCTCTCC CCTAGTATNT TCAGCACATG CTCACTGTTC TCCCCATCCT TGTCCTTCCC 60
ATGTTCATTA ATTCATATTG CCCCGCGCCT AGTCCCATTT TCACTTCCTT TGACGCTCCT 120
AGTAGTTTTG TTAAGTCTTA CCCTGTAATT TTNGCTTTTA ATTTTGATAC CTCTTTATGA 180
CTTAACAATA AAAAGGATGT ATGGTTTTNA AA 212

SEQ ID NO:2943
SEQUENCE LENGTH:212
SEQUENCE TYPE:nucleic acid
TOPOLOGY:linear
CLONE:HUMGS03486
SEQUENCE DESCRIPTION:
GATCTTTTCA GAGTGGTGTT TCTAAAAGAG CATGTACAAA AGTGGCCTGT GGACATTTAG 60
GCCTAAAGTG ATGCATTTNC TCTTCCTNTT TGTGCCAATG TATCAATGTA GAGTTGCTCT 120
GTTTTCTNCA ACTGTATNNA TTGCTGCATT TCTCAGCATA AACTTATCCC ATTGTATTTT 180
TNATAAATAA ATATTTTTTT TGAACTTTCA AA 212

SEQ ID NO:2944
SEQUENCE LENGTH:212
SEQUENCE TYPE:nucleic acid
TOPOLOGY:linear
CLONE:HUMGS03487
SEQUENCE DESCRIPTION:
GATCAAGGAT GAGGATTAGC ATCTGCAAAT TGAACTCTAT CTAATTTNCA TNTTTATTTC 60
TTAGAATTGT AATTAAATTT GTGTAATAAA TGTNTTCCAT TTCATTTGTA CACTTAAAAC 120
TTGGGTGACT GAAATGCAGT TAAGTGAATT NCCTTTTNTT AAAAGCAAAT GTAGATTATN 180
CAATAAAGCA GCAAACCAAT ATTAANCATA AA 212

SEQ ID NO:2945
SEQUENCE LENGTH:211
SEQUENCE TYPE:nucleic acid
TOPOLOGY:linear
CLONE:HUMGS03488
SEQUENCE DESCRIPTION:
GATCTATGTA AAAGAATACA ATTCTTTTTT ACATAATTAG TGAAATTTTA TTTTTTATTA 60
GGAAACACTA AATAGTGTAA TATTTCTTTT GCTTTTAAAA AAATTCCTGG TAGCAAATCA 120
AGATAAATAA TTGNTTCATT TTCTTGAGCA ATACTGAAGC AGGATGAAGT AAGAGGAATG 180
CATTCATTTA AACATGCTTT GCTTGNTGAA A 211

SEQ ID NO:2946
SEQUENCE LENGTH:209
SEQUENCE TYPE:nucleic acid
TOPOLOGY:linear
CLONE:HUMGS03489
SEQUENCE DESCRIPTION:
GATCAAAGAA CTTTTCTTAT AACCTGCAAT CAGCTACTCA GCCAAAAAAC AAAACAAAAC 60
CTTGACTGCC TATGGAGGAA GACTGTGTTC GGGGGAGCTG GCATAGCTAG TGCAGAGTTC 120
AGATTTTCTG CTGATAATCT TTTACACCTT GGGAAAACTT TAATATCCGT ACCTGAAGGN 180
TGATTCACCT AAAAATGTGT TAACTGAAA 209

SEQ ID NO:2947
SEQUENCE LENGTH:208
SEQUENCE TYPE:nucleic acid
TOPOLOGY:linear
CLONE:HUMGS03490
SEQUENCE DESCRIPTION:
GATCTTCTCT GTGAATCCAC CCCTGGCTAC CCCCACCCTG GCTACCCCAA CGGCATCCCA 60
AGGCCAGGTG GGCCCTCAGC TGAGGGAAGG TACGAGCTCC CTGCTGGAGC CTGGGACCCA 120
TGGCACAGGC CAGGCAGCCC GGAGGCTGGG TGGGGCCTCA GTGGGGGCTG CTGCCTGACC 180
CCCAGCACAA TAAAAATGAA ACGTGAAA 208

SEQ ID NO:2948
SEQUENCE LENGTH:207
SEQUENCE TYPE:nucleic acid
TOPOLOGY:linear
CLONE:HUMGS03491
SEQUENCE DESCRIPTION:
GATCACACTT NGGCCTTCAC TGCAGCGTGG TGTGGCCACC GTCCGTTTCC TCTCGGCCTT 60
CCTCCGAGTC CAGGTGGACT CTGTGGATGT TTGGATGTGG CCCGAGCAGG CTCAGGCGGC 120
CCCACTNACC NACAGCATCC GCCGCCACCC CTTCGGGTGT NAGCNCTCAA TAAAAACAAC 180
ACACTATAAA GTGTTTTTAA ATCCAAA 207

SEQ ID NO:2949
SEQUENCE LENGTH:203
SEQUENCE TYPE:nucleic acid
TOPOLOGY:linear
CLONE:HUMGS03492
SEQUENCE DESCRIPTION:
GATCTGAAAA TTCTTGGAAA TTGTTCCATG TGATTAACAT GGAACTGCCT CTACTTAATC 60
ATTCTGAATN ATTAAATCGT TTCATTTTCT AAATGCNTTA TAATGTGTTT AGCCCTTTCT 120
TGTTGCTGTA TGTTTAGATG CTTTCCAATC TTTTGTTACT ACTAATAATG CTATAAAATA 180
AATATCCTTG TACTTCTTTG AAA 203

SEQ ID NO:2950
SEQUENCE LENGTH:201
SEQUENCE TYPE:nucleic acid
TOPOLOGY:linear
CLONE:HUMGS03493
SEQUENCE DESCRIPTION:
GATCTTNTCT GTNTGGGTGG GGAGCCGGCC GGCCGTGACC CAACGGCATC CCAAGGCCAG 60
GTGGGCCCTC AGCTGAGGGA AGGTACGAGC TCCCTGCTGG AGCCTGGGAC CCATGGCACA 120
GGCCAGGCAG CCCGGAGGCT GGGTGGGGCC TCANTGGGGG CTGCTGCCTG ACCCCCAGCA 180
CAATAAAANT GAAACGTGAA A 201

SEQ ID NO:2951
SEQUENCE LENGTH:200
SEQUENCE TYPE:nucleic acid
TOPOLOGY:linear
CLONE:HUMGS03494
SEQUENCE DESCRIPTION:
GATCGTATAT GTGGGTCCCT TCCCTAGAAG AATGGTTGCT GATATGGCTA CTGCTTCTAC 60
ATCTNGAGTT TTTNAATTTA CTTTTTTNAC ACTGTAGCAT TGAGACTGCT TGATTCAAGT 120
CTGGTGCTTT GCCAGATGTA TTAATTNCCA TAAATGCTTT GTGAGTTTGG TTAAAATGAA 180
GATTCACTTG GGAAANCAAA 200

SEQ ID NO:2952
SEQUENCE LENGTH:199
SEQUENCE TYPE:nucleic acid
TOPOLOGY:linear
CLONE:HUMGS03495
SEQUENCE DESCRIPTION:
GATCACAGCC GGAATGCAGC TGAAAGATTC CCTGGGGCCT GGTTCCAACC GCCCACTGTG 60
GACTCTAAGG CCTCTGCATT TGTGGGTGGT CTGNCTGTAA TATTTTGGTC ATNGGCTGGT 120
CTGGTCGGTT TCCCATTTGT NTGGCCAGTC TCTATGTGTC TTAATCCCTT GTCCTTCATT 180
AAAAGCAAAA CTAAAGAAA 199

SEQ ID NO:2953
SEQUENCE LENGTH:319
SEQUENCE TYPE:nucleic acid
TOPOLOGY:linear
CLONE:HUMGS03496
SEQUENCE DESCRIPTION:
GATCTCTAGT TTTTNTGGTT TAATTTTNAG TAGCAGGTCA AAAACCTGCC CTCCTGTNAC 60
TTATTCCCTG AGACTTTTCA GGAGAGCCAG CCCACAGATG ATGAAGAAAT GATGGAAGTT 120
CATTTGGAGA GTCAAATGGG AAAAAAACAA ACAAAAAACT GCCTTTAATA CAGGCAATTC 180
AGTGGACTAT AATAATAGTG GAGGGTTGAG ATGTAGAGTT TTTAAAAAGT GAACAGTTGC 240
TGTTCTTACA TCTNTAANGA AAGCCATAAT GTCTTTAAAT CACTCTTCTG TAAATAGATG 300
NCCNTTTTTG CAGTGTAAA 319

SEQ ID NO:2954
SEQUENCE LENGTH:193
SEQUENCE TYPE:nucleic acid
TOPOLOGY:linear
CLONE:HUMGS03497
SEQUENCE DESCRIPTION:
GATCCTTAAT GCAGAGCTAG AGGACTTCTG GCAGGGAAGT GGGGAAGTGT TCCAGATAGC 60
AGGGCATGAA AACTTAGAGA GGTACAAGTG GCTGAAAATC GAGTTTTTCC TCTGTCTTTA 120
AATTTTATAT GGGCTTTGTT ATCTTCCACT GGAAAAGTGT AATAGCATAC ATCAATGGTG 180
TGTTAAAGCT AAA 193

SEQ ID NO:2955
SEQUENCE LENGTH:193
SEQUENCE TYPE:nucleic acid
TOPOLOGY:linear
CLONE:HUMGS03498
SEQUENCE DESCRIPTION:
GATCTGTTTC ATTTATAAAA GGTCCAGTTT TTAGGACTAG TACATTCCTG TTATTTTCTG 60
GGTTTTATCA TTTTGCCTAA AATAGGATAT AAAAGGGACA AAAAATAAGT AGACTGTTTT 120
TATGTGTGAA TTATATTTCT ACTAAATGTT TTTGTATGAC TGTGTTATAC TTGATAATAT 180
ATATATATAT ATN 193

SEQ ID NO:2956
SEQUENCE LENGTH:193
SEQUENCE TYPE:nucleic acid
TOPOLOGY:linear
CLONE:HUMGS03499
SEQUENCE DESCRIPTION:
GATCCAAGAA TGTGCCAAGA GTCCCGCCAG CCTCAGCCAG GTGGGCCTGT ATATAGGGTC 60
CATGTGCAAT AGGGAGGGAC GTCTTCTATT TTTTNCTGCC CCCTCCCCGC CCACTGTCTG 120
GGGCAGGGGG AGAAGGTATT TTCGAGATAA AGCACAGGCA CCACAAATAA AAGTCGTGAA 180
GTTGCCACTC AAA 193

SEQ ID NO:2957
SEQUENCE LENGTH:192
SEQUENCE TYPE:nucleic acid
TOPOLOGY:linear
CLONE:HUMGS03500
SEQUENCE DESCRIPTION:
GATCTGGGAG CAAGCTACCA ACAGGGGAGA CTCTTTCCTG TATGGACAGC TGCTGTGGAA 60
ATACTGCCTG CTTCTCCCAC CTNCTCANAG CCACAGGAAA GAGGAGGTGA CAGAGAGAGA 120
GCAAGGAAAG TAATGAGGTG GATTGATACT TTCTACTTTN CATTAAAATT ATTTCTAGC 180
CTGCAGTCTA AA 192

SEQ ID NO:2958
SEQUENCE LENGTH:190
SEQUENCE TYPE:nucleic acid
TOPOLOGY:linear
CLONE:HUMGS03501
SEQUENCE DESCRIPTION:
GATCAGCCTG GGCATCATGG TGGCCTGCAC CTGTAGTCCC AGCTACTCAG GAGGCTGAGG 60
TGGGAGGATA GTTTGACCCC AGAAAGTTGA GGCTGCAGTG AGCCATGAAT GCACCACTAC 120
ACGCCAGCCT GGATGACAGA GCGAAACCCT GTCTCAAAGT AATTAATAAT AATTATATTA 180
ATTCAGCAAA 190

SEQ ID NO:2959
SEQUENCE LENGTH:190
SEQUENCE TYPE:nucleic acid
TOPOLOGY:linear
CLONE:HUMGS03502
SEQUENCE DESCRIPTION:
GATCTGCTTC CCACTGTGAC TGGGCTATGG GATTCTGACT ACCTTGCTTA CAGATTCATG 60
GTTTGATAAA TTTGTTGTAT TCAAAAACTT GAAATGCAGG ACGCCATTAA GTGTCTGTTT 120
ATATTTNTGG AATATTTGTA TTACTTACAA TTAATTAATA AAAGTGGGTT TAAAAAACCT 180
TTCCAGGAAA 190

SEQ ID NO:2960
SEQUENCE LENGTH:193
SEQUENCE TYPE:nucleic acid
TOPOLOGY:linear
CLONE:HUMGS03503
SEQUENCE DESCRIPTION:
GATCTTGCCC CCTTTGACCT TCCCCAAAGG ATGGTCACAC ACCAGCACTT TATACACTTC 60
TGGCTCACAG GAAAGTGTCT GCAGTAGGGN ACCCAGAGTC CCAGGCCCCT GGAGTTGTTT 120
TCGGCAGGGG CCNTNTCTCT CACTGCATTT GGTCAGGGGG GCATGAATAA AGGCTACAGG 180
CTCCAACNTG AAA 193

SEQ ID NO:2961
SEQUENCE LENGTH:185
SEQUENCE TYPE:nucleic acid
TOPOLOGY:linear
CLONE:HUMGS03504
SEQUENCE DESCRIPTION:
GATCAATCTG AAACTATCTT AGCCCAGTCA GGGAGCTCTG CTTCCTAGAA AGGCATCTTT 60
CGCCAGTGGA TTCGCCTCAA GGTTGAGGCC GCCATTGGAA GATGAAAAAT TGCACTCCCT 120
TGGTGTAGAC AAATANNAGT TCCCATTGGT GTTGTTGCCT ATAATAAACA CTTTTTCTTT 180
TTAAA 185

SEQ ID NO:2962

SEQUENCE LENGTH:191
SEQUENCE TYPE:nucleic acid
TOPOLOGY:linear
CLONE:HUMGS03505
SEQUENCE DESCRIPTION:
GATCCAACGA GGAATATTTT GCCACACGGC AAATATATAC CGTATAATTT TCTGTACCAC 60
TTGATAGAAA GGTAAAGAAT GAATATTGTT TTTTAAATAA CTTTTTTCTT ATATAAGTAA 120
TATGTGCTTA TCGTGTAACT ACCTAAAAAC ATTGAAAAAT AAAAGGAAAA TAAAATTCAT 180
ACATAACCAA A 191

SEQ ID NO:2963
SEQUENCE LENGTH:177
SEQUENCE TYPE:nucleic acid
TOPOLOGY:linear
CLONE:HUMGS03506
SEQUENCE DESCRIPTION:
GATCAGAAAT GGAGCATGGC CTTGTCCTTT AATGGGGATG CAAATAAAGT TTGTGGGGTT 60
AAAAGTTATA AGACAGNAGT GATACCCCAC TCTCTCCATT ATTGTCCAGC GGGGTGACAT 120
AATGACAGGT TAAATATTTG TGATTCATTG ATTAAATATT ATTTAAAGAA ATGTAAA 177

SEQ ID NO:2964
SEQUENCE LENGTH:176
SEQUENCE TYPE:nucleic acid
TOPOLOGY:linear
CLONE:HUMGS03507
SEQUENCE DESCRIPTION:
GATCACAGNG NTGGTGTCAA ATNCTATTNN AGGTTTGAAC TCTTGAGCTG GAACCTCAGC 60
AAACTAGAGT ATATATTGTT CAGTATTTCT TTGGAAACAT TTCATTAATG TACTTGTCTT 120
ACAGAAATTT CTGAACTTTA GTAAAAAAAA ATAAAGTTAA ACTTTTAAAA CTCAAA 176

SEQ ID NO:2965
SEQUENCE LENGTH:172
SEQUENCE TYPE:nucleic acid
TOPOLOGY:linear
CLONE:HUMGS03508
SEQUENCE DESCRIPTION:
GATCTGAACG ATGCCGTGTC CAGTTTGCGA AGTCCTATTC CCCTCCTCCT GTCGTGTGCC 60
TTTGTTCAGG TGGGGATGTA TTTCATGTAG AAGGTGGAAG AAGGCTGCTA TNACTCTTTG 120
GATGGGAGTC TGGCAAGAGG AAATTGGAAG ATAAAATAAA TAATAAGTGA AA 172

SEQ ID NO:2966
SEQUENCE LENGTH:172
SEQUENCE TYPE:nucleic acid
TOPOLOGY:linear
CLONE:HUMGS03509

SEQUENCE DESCRIPTION:
GATCTGCTCT GCTGCCCTGA ACTCTTACGG CAATTTAGGT TTCTCATTTT NNTTTTCTTT 60
TTACATATGT ACAAATTGTT TTAAGCTTTG GCCTCTATCC AGGTTATNCT GACAATGAAG 120
AAATGGGAGT TGTCAGAGCA TTAAAATGCA ATCTTCACTA AAANCAGTGA AA 172

SEQ ID NO:2967
SEQUENCE LENGTH:170
SEQUENCE TYPE:nucleic acid
TOPOLOGY:linear
CLONE:HUMGS03510
SEQUENCE DESCRIPTION:
GATCTCTAAT ACAGTATCTA ACACAAAAGA AGCTTTAAAA AGACAGTTTC TTTCCTTATT 60
TGATTAGCTA GAAGTTTATC TAGGTAAAAG CAAAAAAAAA ANTTTNCAAN GCATANCATA 120
NCATTGGGGT TCTTTTTATA CTCAAGATGT TTANCTGTTC AAANTGNCAN 170

SEQ ID NO:2968
SEQUENCE LENGTH:355
SEQUENCE TYPE:nucleic acid
TOPOLOGY:linear
CLONE:HUMGS03511
SEQUENCE DESCRIPTION:
GATCCAGGCG GGAGCAGAAG CTAAAGCCAA AGCCCAAGAG AGTGGCAGTG CCAGCACTGG 60
TGCCAGTACC AGTACCAATA ACAGTGCCAG TGCCAGTGCC AGCACCAGTG GTGGCTTCAG 120
TGCTGGTGCC AGCCTGACCG TCACTNTCAC ATTTGGGCTC TTCGCTGGCC TTGGTGGAGC 180
TGGTGCCAGC ACCANTGGCA GCTCTGGTGC CTGTGGTTTC TCCTACAAGT GAGATTTTAG 240
ATATTGTTAA TCCTGCCAGT TTTTTNTTTT TCAAGNCAGG GGTTGCATNC TNAGAAAACC 300
CTACTCNACA CAGCACTTCT TANGGTAGGC CNCTTATTCA ANTTCAATTT NNAGN 355

SEQ ID NO:2969
SEQUENCE LENGTH:258
SEQUENCE TYPE:nucleic acid
TOPOLOGY:linear
CLONE:HUMGS03512
SEQUENCE DESCRIPTION:
GATCTCGGAT GCCATCCCCG AGCTGAAGGC CTCCATCAAG AAGGGGGAAG ATTTCGTGAA 60
GACCCTGAAG TGAGCCGCTG TGACGGGTGG CCAGTTTCCT TAATTTATGA AGGCATCATG 120
TCACTGNAAA GCCGTTGCAG NTAAACTTTG TATTTNAATT TGCTTTGGTG ATGATTACTG 180
TNTTGACATC NTCATGCCTT CCAAATTGTG GGTGGCTCTG TGGGCGCATN ANTNANAGCC 240
GTCCTTGATT TTGGGAAA 258

SEQ ID NO:2970
SEQUENCE LENGTH:81
SEQUENCE TYPE:nucleic acid
TOPOLOGY:linear
CLONE:HUMGS03513

SEQUENCE DESCRIPTION:
GATCACAAGA GGCCCATTTG TAGTGGCTGG TTGACAACGG CTGGNGCAAA TTAAATAAAT 60
AAAATAGCTC TNTCTTTCAA A 81

SEQ ID NO:2971
SEQUENCE LENGTH:169
SEQUENCE TYPE:nucleic acid
TOPOLOGY:linear
CLONE:HUMGS03514
SEQUENCE DESCRIPTION:
GATCTNAGAC ATCCCCATCT TTGTAAGCAG AACAGTACGG CACTTCACCA CATCTNCTTC 60
CCACCATGCT TCTAAGCAGC TGTNTTCCCC CTGCTAATGT TACAACCAAA GCAGCCACCC 120
CACCTCCTNT NGTGTTGAGC CTCACGACCT NTGACCCAGC TGGAAAGCN 169

SEQ ID NO:2972
SEQUENCE LENGTH:168
SEQUENCE TYPE:nucleic acid
TOPOLOGY:linear
CLONE:HUMGS03515
SEQUENCE DESCRIPTION:
GATCTCATTA CTGCTTTTCT TTTATGTCCT TTTGTCTCCA TGGGAGGTTG AGCTTCTTTT 60
CCTACAAGTT TACTTACTGT TTGCATTTAC TTTTTCTTTG CATTGTCTGT TCCAGTCCTC 120
TGGCTATTTG ACTATTGTGT AATAAAAGGA CCAAAAACAA ATGTGGTN 168

SEQ ID NO:2973
SEQUENCE LENGTH:225
SEQUENCE TYPE:nucleic acid
TOPOLOGY:linear
CLONE:HUMGS03516
SEQUENCE DESCRIPTION:
GATCTTGCTG CAGCCACAGT GCAGNTCCAC ATTAACTCTA CAGACCAAAC CATTTGTATC 60
TGGCATCACT TACTAACACA CGNCATGCGG CTTTTCTGCA TCAACTGCTA TGACGGTTAA 120
GANTGTCAGT ATACAAGNAG GGATAGAAAA CTGGTACTGT TTTAAATAAT CTGTAATNTC 180
AATGTTTTTT TTTTTNTTTC GGAAANNCNT NGTTTTGCCC GNTTN 225

SEQ ID NO:2974
SEQUENCE LENGTH:169
SEQUENCE TYPE:nucleic acid
TOPOLOGY:linear
CLONE:HUMGS03517
SEQUENCE DESCRIPTION:
GATCTTCTGT GGTGCTTAAG GNAACTTACT AGAGCTCCAC TAACAGTCTC ATAAGGAGGC 60
AGCCATCATA ACCATTGAAT AGCATGCAAG GGTAAGAATG AGTTTTTANC TGCTTTGTAN 120
GCAAATGGNA AAGGTCAATA AAGNTATATT TCTTTAGNAA ATGGGCAAA 169

SEQ ID NO:2975
SEQUENCE LENGTH:167
SEQUENCE TYPE:nucleic acid
TOPOLOGY:linear
CLONE:HUMGS03518
SEQUENCE DESCRIPTION:
GATCCCAACT CCCATGACCT CTGGCTTCAG TGGTGGGTGG GGCAGGGCAG ATGAAAGGGC 60
TTCAGTGGGA ACCTCTGAGA GCATTTTCCT GTTCCCCCTA TCAACCGCCC CCAGTGATAA 120
CATCTGTGAA GCCAGCCATT ACTCAATAAA CTGCAAACTT GTCTAAA 167

SEQ ID NO:2976
SEQUENCE LENGTH:167
SEQUENCE TYPE:nucleic acid
TOPOLOGY:linear
CLONE:HUMGS03519
SEQUENCE DESCRIPTION:
GATCAGNAGC CCACTGATAA GGGGCCCTAG GGTACAGGGT GCTGCCCAGC AGGTCGCCAC 60
CGAGTGTCTT CTCATTTTAT TTCAGCTCCA TTTTGCCCAT AGATGGGCAG AGGGGTGAGA 120
TTGGCTCATC CCCCTTCCAG ATTCTGCAAT AAAGCGGTGT GAGGAAA 167

SEQ ID NO:2977
SEQUENCE LENGTH:167
SEQUENCE TYPE:nucleic acid
TOPOLOGY:linear
CLONE:HUMGS03520
SEQUENCE DESCRIPTION:
GATCATTCCC TCTTTTNGGA TGTATAAGAN CCTTCCGGAC AACAGAACCT ATTTNTGGAA 60
TTGCAGAAGA TAACATATTT CCCTTATTTN NATTTAATCA CCATAAACCA TACCTATTTA 120
ATGAGTGTAT TCTGTGCAAT TTTTTCCTCA GATTGTNTTT AACTNNN 167

SEQ ID NO:2978
SEQUENCE LENGTH:166
SEQUENCE TYPE:nucleic acid
TOPOLOGY:linear
CLONE:HUMGS03521
SEQUENCE DESCRIPTION:
GATCGACTGC CAAGCAGCAG CCGCTGCCGA GGGGCTGCGT CTGACATCCT GACCTCCTGG 60
ACTGTAGGAC TATATAAAGT ACTACTGTAG AACTGCAATT NCCATTCTTT TAAATGGGTG 120
AAAAATGGTA ATATAACANT ATATGATATA TAANCCTTAA ATGAAA 166

SEQ ID NO:2979
SEQUENCE LENGTH:165
SEQUENCE TYPE:nucleic acid
TOPOLOGY:linear
CLONE:HUMGS03522

SEQUENCE DESCRIPTION:
GATCTGGGCG GGGTGGTGCG CTCCTCAGGG CGGATGCCAC CGGGTTCCCC AGGGGAATAC 60
ATGTCCCCAG CTCTGGGAGG GGTTTGCTAC TGGCCTCCTA CTTTCCTCCC TAGGTGGACA 120
GTGCTCCTCT AGAGAGCTGC GACTTTAATT AAAAACAACA GGAAA 165

SEQ ID NO:2980
SEQUENCE LENGTH:165
SEQUENCE TYPE:nucleic acid
TOPOLOGY:linear
CLONE:HUMGS03523
SEQUENCE DESCRIPTION:
GATCAGTGTC AGATATTGTT GAGGGAAGTA ATTTTATAAA GTTACACAAA GGTAGTTATA 60
AAAAAAGCCC AGTTTGTTTT TCAGAAGGTG ACTTTNATGT GCTTGAAAAG TTTAATATTT 120
GANTATTGTG TTTAACCACA TGGTATTAAA ATTTTGCAAT ATAAA 165

SEQ ID NO:2981
SEQUENCE LENGTH:163
SEQUENCE TYPE:nucleic acid
TOPOLOGY:linear
CLONE:HUMGS03524
SEQUENCE DESCRIPTION:
GATCATGAAA TAATGTGCTT TGTAAAAAGA TTTCAAGTTA TTAGGAAGCA TACTCTGTTT 60
TTNAATCATG TATAATATTC CATGATACTT TTATAGAACA ATTCTGGCTT CAGGAAAGTC 120
TAGAAGCAAT ATTTCTTCAA ATAAAAGGTG TTTAAACTTT AAA 163

SEQ ID NO:2982
SEQUENCE LENGTH:162
SEQUENCE TYPE:nucleic acid
TOPOLOGY:linear
CLONE:HUMGS03525
SEQUENCE DESCRIPTION:
GATCCGCCTG CTTCGGCCTC CCAAAGTNCT GGGATTATAG GTGTGAGCCA CCCTGCTCAA 60
CCAGGTTTNA TTATTTAANT TAGTTAAACT TTGGATAGAT TGTATAATAT ATAGTTTAAT 120
GTAATCATGC TCATATTTTT NAAATAAATA AAACACTATA AA 162

SEQ ID NO:2983
SEQUENCE LENGTH:161
SEQUENCE TYPE:nucleic acid
TOPOLOGY:linear
CLONE:HUMGS03526
SEQUENCE DESCRIPTION:
GATCACTTGT TGTTTTACTA AAGAAAGATT ACTTAGAGGA AATAAGAAAA ATCATGTTTG 60
CTCTCCCGGT TCTTCCAGTG GTTTGAGACA CTGGTTTACA CTTTATGCCG GATGTGCTTT 120
TCTCCAATAT CAGTGCTCGA GACACAGTGA AGCAAATTAA A 161

SEQ ID NO:2984
SEQUENCE LENGTH:161
SEQUENCE TYPE:nucleic acid
TOPOLOGY:linear
CLONE:HUMGS03527
SEQUENCE DESCRIPTION:
GATCTGAGGT CTAACATTGT TATCCTATAT CATTTTCATC CCAAGTAGTG ATATCTGTGA 60
AATCACAGGT TTGATGTGTG CTAATNATGT ATTCTTCTAA TACATATTAA AAGACATAAC 120
TATCAAAACA AAATAAATTT GTCTGTTTTC AACCAAAGAA A 161

SEQ ID NO:2985
SEQUENCE LENGTH:161
SEQUENCE TYPE:nucleic acid
TOPOLOGY:linear
CLONE:HUMGS03528
SEQUENCE DESCRIPTION:
GATCTCCATT TGATATTTTC ATTTGTATAA CTCATTTGCA GTCTGAAAAT TTTTTTTAGT 60
GCCAGTCCCT GAACATATCA TTGAAAGTTA ATTTTCTTTG CATTTTAAAA TATCTGGATT 120
ATGAAGAAAA AGTGATGAAA ATAAATTAAA ACTGAATTAA A 161

SEQ ID NO:2986
SEQUENCE LENGTH:160
SEQUENCE TYPE:nucleic acid
TOPOLOGY:linear
CLONE:HUMGS03529
SEQUENCE DESCRIPTION:
GATCTGTGCT AGCTGTGAGG CAGCTCTGGA ACGTGAAGAG CTGTTTGGTT TGAACCGTGA 60
ACAAAACTGT GTTTTGAGTT TAGCTGACAT TATGGAAAAA AGTTCATCAC GTGACTGTTA 120
ATGTAAACCT GGTTATTAAA ATAACNATGA AATTACCAAA 160

SEQ ID NO:2987
SEQUENCE LENGTH:157
SEQUENCE TYPE:nucleic acid
TOPOLOGY:linear
CLONE:HUMGS03530
SEQUENCE DESCRIPTION:
GATCTCTCTG TNACTGACTT TGTGACTGTC CTGTGGTTTC TCCTGCCATT GCTTTGTGTT 60
TGGAAGGACA TGATGGGGGT GATGGACTGG AAAGAAGGTG CCAAAAGTTC CCTCTGTGTT 120
ACTCCCATTT AGAAAATAAA CACTTTTAAA TNATAAA 157

SEQ ID NO:2988
SEQUENCE LENGTH:157
SEQUENCE TYPE:nucleic acid
TOPOLOGY:linear
CLONE:HUMGS03531

SEQUENCE DESCRIPTION:
GATCAAANCA CCTNGGAGTG GTCACCAGGG GGACAGGGAG CCCCCCACCA ATGTATCANT 60
GGGTGATTTA TNATGCCTTC TGCCCTTTGG CGAGTGAATG GNTTTCCCAT AGGGGAAGTT 120
GGCCTCCCTC CGTGAGCTTT GGAAATNTTT TCTAATN 157

SEQ ID NO:2989
SEQUENCE LENGTH:157
SEQUENCE TYPE:nucleic acid
TOPOLOGY:linear
CLONE:HUMGS03532
SEQUENCE DESCRIPTION:
GATCCGAATG GNCTGGGTGA GCACGAATTA CCGAGGCCTT CCCTTTGNTA CAGTCCAGGA 60
TTTGTAAGGN ATGAAGACCC CTGGNCCCCA TTCTNTTGGG GTCCATACAT ACTCTCCGAA 120
GATAGCAACT TGCTTCAGGT CAAAGTNAAC CCNAGAN 157

SEQ ID NO:2990
SEQUENCE LENGTH:114
SEQUENCE TYPE:nucleic acid
TOPOLOGY:linear
CLONE:HUMGS03533
SEQUENCE DESCRIPTION:
GATCTCCTTA CGTAGCAGCC AAAATAAATG AAGCAAAAGA CTTGCTAGAA ACAACCACCA 60
AACATTGATG CTTAAGGACC ACACTGAAGG AAAAAAAAAG AGGGGACTTC GAAA 114

SEQ ID NO:2991
SEQUENCE LENGTH:155
SEQUENCE TYPE:nucleic acid
TOPOLOGY:linear
CLONE:HUMGS03534
SEQUENCE DESCRIPTION:
GATCTGATTT ACAAGAAAAA GTCAGAGCTA AACACTAGTG ATGGCATAGC ATTACTGAAA 60
TCATTGTTTC TTAATTTCAT TTTACTACAT TGTGAACTTG TGATTCAGAT TCCATTTTCT 120
CAGAGAATTA AAAACAAAAA AAGTACTCTT GTAAA 155

SEQ ID NO:2992
SEQUENCE LENGTH:156
SEQUENCE TYPE:nucleic acid
TOPOLOGY:linear
CLONE:HUMGS03535
SEQUENCE DESCRIPTION:
GATCTATTTC TNAGTATGTG GTTCATGCTG TTGTGAAAAA TGTTTTACCT TTNACCTTTG 60
TCAGTTTGTA ATNAGAGGAT TTCCTTTNAC CCTTTGTAGC TCAGAGAGCA CCTGATGTAT 120
CATCTCAAAC ACAATAAACA TGCTNCTGAA GGCAAA 156

SEQ ID NO:2993

SEQUENCE LENGTH:154
SEQUENCE TYPE:nucleic acid
TOPOLOGY:linear
CLONE:HUMGS03536
SEQUENCE DESCRIPTION:
GATCAAATTA GTAATTNNGT TTATGCTGCT CCTGATATAA CACTTTCCAG CCTATAGCAG 60
AAGCACATTT TCAGACTGCA ATATAGAGAC TGTGTTCATG TGTAAAGACT GAGCAGAACT 120
GAAAAATTAC TTATNGGATA TNCATTCTNN NCTN 154

SEQ ID NO:2994
SEQUENCE LENGTH:154
SEQUENCE TYPE:nucleic acid
TOPOLOGY:linear
CLONE:HUMGS03537
SEQUENCE DESCRIPTION:
GATCTCATTT ATAATTAAAT TATGTCTATT GGATTGTTTT NATTGTACTT NATGTTTTNT 60
NAAGAAATTG TGATTAAATA TAACTTAACG ACTTTCTATT TTNCAGTTAT TTTAATTAGT 120
TACTTCATTT AANCTTTTGG CATTNCATGA TTTN 154

SEQ ID NO:2995
SEQUENCE LENGTH:153
SEQUENCE TYPE:nucleic acid
TOPOLOGY:linear
CLONE:HUMGS03538
SEQUENCE DESCRIPTION:
GATCATATAT GTNTTAGTGG ACAGGGGTCT GAAGTACACT GGAATTTACT GAGAAACTTG 60
TTTGTAAAAA CTATAGTTAA TAATTATTGC ATTTTCTTAC AAAAATATAT TTTGGAAAAT 120
TGTATACTGT CAATTAAAGT GTTTTTGTGT AAA 153

SEQ ID NO:2996
SEQUENCE LENGTH:153
SEQUENCE TYPE:nucleic acid
TOPOLOGY:linear
CLONE:HUMGS03539
SEQUENCE DESCRIPTION:
GATCAAACTA CACATGNGTT TTCATTCCAA AAGTGGGTTC TAAANGCCTG GCTGCATCTG 60
TATGNAATCA AGGCACACTC CAGGAAGACT GCCGCGTCGC GCCAACACGT CATACTCAAT 120
GCCTCAGACT TTCATATTTC TGNNGTGCTG NNN 153

SEQ ID NO:2997
SEQUENCE LENGTH:152
SEQUENCE TYPE:nucleic acid
TOPOLOGY:linear
CLONE:HUMGS03540
SEQUENCE DESCRIPTION:

GATCCCAGCA TCTTCTCCAC TTCAGCGCTG AGTTCAGTAT ACACAAGTGT CTGCTACAGT 60
CGCCAAATCA CCAGTATTTG CTTATATAGC AATGAGTTTT ATTTTGTTTA TTTGTTTTGC 120
AATAAAGGAT ATGAAGGTGG CTGGCTAGGA AA 152

SEQ ID NO:2998
SEQUENCE LENGTH:152
SEQUENCE TYPE:nucleic acid
TOPOLOGY:linear
CLONE:HUMGS03541
SEQUENCE DESCRIPTION:
GATCATTTGG ATATAGCAAT CTACTCTGAG AAATGGAACA CAAGGAGTTA CCTATCACTT 60
TCACTTATAA TTCCAAAAGA TGACTACAAC CATGTCCATG CTCAGATTCA AACAGTTTTC 120
CATATCACTT TTGGGTGGTA ATTACAGTTT TN 152

SEQ ID NO:2999
SEQUENCE LENGTH:152
SEQUENCE TYPE:nucleic acid
TOPOLOGY:linear
CLONE:HUMGS03542
SEQUENCE DESCRIPTION:
GATCCCTGTN TGCNCGNCCC CTCTGCAATG TATGCCCCTT GCCCCTTCCC CACACTAATA 60
ATTTATATAT ATAAATATCT ATATGANGCT CTTAAAAAAA CATCCCAACC AAAACCAACC 120
AAACAAAAAC ATCCTCACAA CTCCCCAGGA AA 152

SEQ ID NO:3000
SEQUENCE LENGTH:151
SEQUENCE TYPE:nucleic acid
TOPOLOGY:linear
CLONE:HUMGS03543
SEQUENCE DESCRIPTION:
GATCCAGAAG CAAACACAAG CAATGCAAGA CCTCCACAGA GTGAGCTTGG AGAGAGAGAA 60
AGCCAAGATA AGAGAGGAGT ATGANGAGAA ANTCAGAAAG CTGGAAGATA AAGTGGAGCA 120
GGNAAAGAGA AAGANGCAAA TGGNGANGAN N 151

SEQ ID NO:3001
SEQUENCE LENGTH:151
SEQUENCE TYPE:nucleic acid
TOPOLOGY:linear
CLONE:HUMGS03544
SEQUENCE DESCRIPTION:
GATCTGGAGA TAANTTTATG CCTTTGGCCA TAGACAGGAC TGGAATCCCA GTTCTGCCAC 60
TTACTGGCTG TTTGACCTTA GGCAAGCCAC TCCTCCTTTN TNAGCCTCAG TTTCTTTNCC 120
TGTAAGATAA AAAGAATGAN TCATTACTAA A 151

SEQ ID NO:3002

SEQUENCE LENGTH:150
SEQUENCE TYPE:nucleic acid
TOPOLOGY:linear
CLONE:HUMGS03545
SEQUENCE DESCRIPTION:
GATCAGGGCT GGGGCGGGGA AACAAGGGAA GGACCTTGGA AGGGGCTGNN CCCAGGCCTG 60
GGGGGCAGTC GTGGGAGCCC CTCTCANCTG TGGGGCTGGC ACAGAGCCCC AGGCAAGCTT 120
TTAATAAACT GTTGGTTATT CTAACAGAAA 150

SEQ ID NO:3003
SEQUENCE LENGTH:149
SEQUENCE TYPE:nucleic acid
TOPOLOGY:linear
CLONE:HUMGS03546
SEQUENCE DESCRIPTION:
GATCTCAAAA ATAGGTAATT TTTCTTTGCC GACCTGTAAA AGTGTGCCAA TACACTAAAT 60
TTGTGATTTT AAATTAATTC CTCCAGCTGT TGAAATGAAG TCTGCCAAAT CTTGCTCTAA 120
CAAATAAAAT GTTATCTAAA TGAAGTAAA 149

SEQ ID NO:3004
SEQUENCE LENGTH:149
SEQUENCE TYPE:nucleic acid
TOPOLOGY:linear
CLONE:HUMGS03547
SEQUENCE DESCRIPTION:
GATCCTAAAA GGGAAAATTG CCTTGGTAAC TTTNAGATTC CTGTGGAATT GTGAATNCAT 60
ACTAAGCTTT CTGTGCAGTC TCACCATTTG CATCACTGAG GATGAAACTN ACTTTNGTNT 120
TTNGGAGAAA AAAAACTGTN CTGCTTGTN 149

SEQ ID NO:3005
SEQUENCE LENGTH:242
SEQUENCE TYPE:nucleic acid
TOPOLOGY:linear
CLONE:HUMGS03548
SEQUENCE DESCRIPTION:
GATCCTAGGA TGCGTCCCAG ACGCTCAGTC AGAAGTGCTG GAGGTGGGGC CTGGGAAGCT 60
GTATTTGTAA TNAACTCTGG NGTTTTTTGT CCATTAAAGT GTATCTTTGT CCATCCTATA 120
AGNTTANNGG AAAGAAAAAG CATCTCAAAT NAGTGTAAGT TGTTCTTGAG AAAAAAATGT 180
ATCAGACTTT TATGATTTGA ATGAAATGTA TTATAGNAAA ANATAAACAC NTTTAAANTA 240
AA 242

SEQ ID NO:3006
SEQUENCE LENGTH:148
SEQUENCE TYPE:nucleic acid
TOPOLOGY:linear

CLONE:HUMGS03549
SEQUENCE DESCRIPTION:
GATCCAGTTG TACTTAAGTG TGTAACAGGA ATATTTTGCA GAATATAGGT TTAACTGAAT 60
GAAGCCATAT TAATAACTGC ATTTTCCTAA CTTTGAAAAA TTTTGCAAAT GTCTTAGGTG 120
ATTTAAATAA ATNAGTATTG GGCCTAAA 148

SEQ ID NO:3007
SEQUENCE LENGTH:148
SEQUENCE TYPE:nucleic acid
TOPOLOGY:linear
CLONE:HUMGS03550
SEQUENCE DESCRIPTION:
GATCATCGTG CTCTCAGACT CTGATTAGCT GCCTCCCCTT CTCCCTGCCT CCAGAATGTT 60
CTGGGATAAC ATTTGGAGGA AGGTGGGAAG CAGATGACTG AGGAAGGGAT GGACTAAGCT 120
AATCCCCTTT TGGTGGTGTT TCTTTAAA 148

SEQ ID NO:3008
SEQUENCE LENGTH:148
SEQUENCE TYPE:nucleic acid
TOPOLOGY:linear
CLONE:HUMGS03551
SEQUENCE DESCRIPTION:
GATCAGTCCA CCCAGAGTTA GACATTGTTT TTGTAGAAAA CAGGTATTTA TTATGTCTAG 60
GGTTTTGTGT TTTTTTTTTT TCCTNANAGG NTAAAAGCCT TTATACAGAA ANAATTTGGA 120
ACCTTTNATA TAAAACCTTT TCTTTAAA 148

SEQ ID NO:3009
SEQUENCE LENGTH:171
SEQUENCE TYPE:nucleic acid
TOPOLOGY:linear
CLONE:HUMGS03552
SEQUENCE DESCRIPTION:
GATCGCTGTG GTTCTTATTA TCAAAATTAA GTTTACTTGT ATACGGAACA ACCACAAGAA 60
ATTTGATTCT GTAAAGAATC CTCTTTAGCT GTGGCCTGGC AGTATATAAA TGGTGCTTTA 120
TTTAACAGAA TACCTGTGGA GGAAATAAAG CACACTTGAT GTAAAANTAA A 171

SEQ ID NO:3010
SEQUENCE LENGTH:145
SEQUENCE TYPE:nucleic acid
TOPOLOGY:linear
CLONE:HUMGS03553
SEQUENCE DESCRIPTION:
GATCTTTTTA TTTTGCAATC CTGGGCCAGC TAGAAGCCAG GAGCTGATTG ACCTTTTAAC 60
TTTTTTCAGT GGCCACATTT TGGTTATCGA TGTACCTAGA AGTATGTAAA TNAGATTAAA 120
TTTCTCTTCT GGAAACACCC TGAAA 145

SEQ ID NO:3011
SEQUENCE LENGTH:144
SEQUENCE TYPE:nucleic acid
TOPOLOGY:linear
CLONE:HUMGS03554
SEQUENCE DESCRIPTION:
GATCATAGAG AATGGTGCTT TTTACTACAG TTAGCACATG CATTTTTAGA AACTACTACA 60
TGTTTTAGAG AATCTTTGCT GTGTATATGT AAACTGTATT GTTCAACTGT TAACAAATAA 120
TAAATTATTT CATTATTAAA GAAA 144

SEQ ID NO:3012
SEQUENCE LENGTH:144
SEQUENCE TYPE:nucleic acid
TOPOLOGY:linear
CLONE:HUMGS03555
SEQUENCE DESCRIPTION:
GATCTGCCCT NCTCCTTCTA AAGGGTAAGT CATAATCTGT GTAATACTAC AATTNATGGG 60
NTGCTCACTA TGCCCTGTTT CTNTNCTAAA CAATTTACAT GTAATGTCTC ATTCCTCACA 120
ATAACCCTTG TAAAGTGGGC ANNN 144

SEQ ID NO:3013
SEQUENCE LENGTH:142
SEQUENCE TYPE:nucleic acid
TOPOLOGY:linear
CLONE:HUMGS03556
SEQUENCE DESCRIPTION:
GATCCCCAGC TGCCTCACTT CTCTCTTGAG AACTTGGCTC AGGGCTCCTG AGGACCTTTC 60
CCAGCATTAC CTTCCCTTCC CTTGAAAGGC AATTGTTGGC TGTTTTCATA AGCAGGAAAA 120
NTAAACAGAA GTATAAAGGA AA 142

SEQ ID NO:3014
SEQUENCE LENGTH:142
SEQUENCE TYPE:nucleic acid
TOPOLOGY:linear
CLONE:HUMGS03557
SEQUENCE DESCRIPTION:
GATCAAATGT GCCTTAATAA ATTTNTTTTC ATTTAGATTT CAAACAGTGA TAGACTTGCC 60
ATTTTAATAC ACGTCATTGG AGGGCTGCGT ATTTGTAAAT AGCCTGATGC TCATTTGGAA 120
AAATAAACCA GTGAACAATA AA 142

SEQ ID NO:3015
SEQUENCE LENGTH:147
SEQUENCE TYPE:nucleic acid
TOPOLOGY:linear

CLONE:HUMGS03558
SEQUENCE DESCRIPTION:
GATCTAAAGA AACAGTTGGC TGTAGCTGAG GGGAAACCCC CTGAAGCCCC TAAAGGCAAG 60
AAGAAAAAGT AAAAGACCTT GGCTCATAGA AAGTCACTTT AATAGATAGG GACAGTAATA 120
AATAAATGTA CAATCTCTAT ATACAAA 147

SEQ ID NO:3016
SEQUENCE LENGTH:140
SEQUENCE TYPE:nucleic acid
TOPOLOGY:linear
CLONE:HUMGS03559
SEQUENCE DESCRIPTION:
GATCCCAGCA ATGAACTGTT CTAGGGAAAG TGGCTTCCTG CCCAAACTGG ATGGGACACG 60
TGGGGAGTGG GGTGGGGGGA GCTATTTCCA AGGCCCCTCC CTGTTTCCCC AGCAATTAAA 120
ACGGACTCAT CTCTGGCAAA 140

SEQ ID NO:3017
SEQUENCE LENGTH:153
SEQUENCE TYPE:nucleic acid
TOPOLOGY:linear
CLONE:HUMGS03560
SEQUENCE DESCRIPTION:
GATCTCAGCT TTGAGAAGCC TTCAGCTCCA GGGAATCCAA GCCTCCACAG CGAGGGCAGC 60
TGCTATTTAT TTTCCTAAAG AGAGTATTTT TATACAAACC TACCAAAATG GAATAAAAGG 120
CTTGANGCTG TGGCCTGAGT GCCTCACTGG AAA 153

SEQ ID NO:3018
SEQUENCE LENGTH:134
SEQUENCE TYPE:nucleic acid
TOPOLOGY:linear
CLONE:HUMGS03561
SEQUENCE DESCRIPTION:
GATCACGAAG GTGGTTTTCC CAGGGCGAGG CTTATCCATT GCACTCCGGA TGTGCTGACC 60
CCTGCGATTT CCCCAAATGT GGGAAACTCG ACTGCATAAT TTGTGGTAGT GGGGGACTGC 120
GTTCGCGCTT TAAA 134

SEQ ID NO:3019
SEQUENCE LENGTH:131
SEQUENCE TYPE:nucleic acid
TOPOLOGY:linear
CLONE:HUMGS03562
SEQUENCE DESCRIPTION:
GATCTATCTT NCAGAAAGTA TGTTTTTCCT CATAAAAGTG CCTCTTAATT GGCCATTGTA 60
CCAGCCACTT GTCCTAGCCA AATGTCCAAA ACACGCCCTT GGGCCCCGCC ACGTTACAAT 120
CCACANNNNN N 131

SEQ ID NO:3020
SEQUENCE LENGTH:131
SEQUENCE TYPE:nucleic acid
TOPOLOGY:linear
CLONE:HUMGS03563
SEQUENCE DESCRIPTION:
GATCACTGTN ATGTGTGGTC CATCTCTGAC CAAAACGTTA TGCAGTACGT GACTGTATCC 60
TNATCCTCAA TAATCCTTGG TTATTATTCT AGAATTTCAA ATAAATGGAA TCATATCATT 120
TACTATGTAA A 131

SEQ ID NO:3021
SEQUENCE LENGTH:207
SEQUENCE TYPE:nucleic acid
TOPOLOGY:linear
CLONE:HUMGS03564
SEQUENCE DESCRIPTION:
GATCCAGTGA ATGATTCAAG AGAGCTACAT TTGAAGCCTG TACAAAAGCT TATCCCTGTA 60
ACACATGTGC CATAATATAC AAACTTCTAC TTTCGTCAGT CCTTAACATC TACCTCTCTG 120
AATTTTCATG AATTTCTATT TCACAAGGGT AATTGTTTTA TATACACTGG CAGCAGCATA 180
CAATAAANCT TAGTATGAAA CTTTAAA 207

SEQ ID NO:3022
SEQUENCE LENGTH:130
SEQUENCE TYPE:nucleic acid
TOPOLOGY:linear
CLONE:HUMGS03565
SEQUENCE DESCRIPTION:
GATCCTGAGA ATTACTTTTA ATAAAATCAT TTTTTTGCTG TTATTAAAAC TAGNCNNGAA 60
TTGCCTAAAN CCAAGAACTC TGCTTGATAA AATAAGCATA GTNTTAGGAA CAGCCATGCA 120
GATATAANTN 130

SEQ ID NO:3023
SEQUENCE LENGTH:129
SEQUENCE TYPE:nucleic acid
TOPOLOGY:linear
CLONE:HUMGS03566
SEQUENCE DESCRIPTION:
GATCATCGAC CCCCAAGGGA CCCGCAGACC CTTAAAAAAA TCACCCACAA CCCTCTGGAA 60
GTGGCCTTGC CCGGTCCCCT TCCCAGGGGC GAGGTCGGCA AAGCAACATG GCAGAGCAGT 120
CATAGGAAA 129

SEQ ID NO:3024
SEQUENCE LENGTH:129
SEQUENCE TYPE:nucleic acid

TOPOLOGY:linear
CLONE:HUMGS03567
SEQUENCE DESCRIPTION:
GATCACCTTT TTTTTATGC GTTGTGAATA GCAGTCTGCA TCCTAAGTTT AAAATATTTG 60
AGAATCCTCT ATGGGACTCT CTGTAAATNT TTTAAATACT GTTGTTTTCA ATAAATATTT 120
TATACTAAA 129

SEQ ID NO:3025
SEQUENCE LENGTH:129
SEQUENCE TYPE:nucleic acid
TOPOLOGY:linear
CLONE:HUMGS03568
SEQUENCE DESCRIPTION:
GATCAGCTGT CCTCTCTGAA AAAAATTGCA CCNNTTTTTC TTTTTTAAAA TTTTGTTGCA 60
TCTTCGTAGT AATGTAATTG TATTTTATGC CTGCTTTTTT ATAAAAAAAT AAAATAAAAT 120
AAATGGAAA 129

SEQ ID NO:3026
SEQUENCE LENGTH:129
SEQUENCE TYPE:nucleic acid
TOPOLOGY:linear
CLONE:HUMGS03569
SEQUENCE DESCRIPTION:
GATCTAAATN TGACTGTGGT TGCACATATC TGTGNATATA CTAAAAACCA NTGATTTGTA 60
CACTTTACAT GGGTGAGTTG TATGGTGTGT GAATTATATT TCAATAAAGC TGGGTTGTTT 120
TTTGTCAAN 129

SEQ ID NO:3027
SEQUENCE LENGTH:140
SEQUENCE TYPE:nucleic acid
TOPOLOGY:linear
CLONE:HUMGS03570
SEQUENCE DESCRIPTION:
GATCCCAGGA TTCAGTATTC CTGGCCCAGA GGNCNTTGCT GGCTACTGGG TGTTAGTTTG 60
CAGTCCTGTG TGCTTCCCTC TCTTATGACT GTGTCCCTGG TTGTCAATAA AATATTTCCT 120
GGCCTCCTGG AATCTNTAAA 140

SEQ ID NO:3028
SEQUENCE LENGTH:127
SEQUENCE TYPE:nucleic acid
TOPOLOGY:linear
CLONE:HUMGS03571
SEQUENCE DESCRIPTION:
GATCCTACGA CAGGACCCAG ATGCCAGTGA AAGTGACCAC AGCCCTGCTC TAGTCACCCC 60
GGAAGCTGAC CAGTCTACGC ACGGCCGAAG CTTCGTCAAG GAAGTAAATG TAGTTAGAAA 120

TCTTAAA 127

SEQ ID NO:3029
SEQUENCE LENGTH:223
SEQUENCE TYPE:nucleic acid
TOPOLOGY:linear
CLONE:HUMGS03572
SEQUENCE DESCRIPTION:
GATCTAGAGT ATAAGCTGCG CAAGGGCAGA AGTTTTTATC TGGTTTGTTC ATGGATGTAT 60
TCTAAGAGCT GAGAACAGGG CCTGGACACA ATAAGCATTC AATAAATATT TACTGAATGA 120
ATGAACTCCT ACCTATATTC CTATTTATAA TTTGGCTCCA CTTTATCCTA CTTTAGCTCC 180
CATTCAATTC AATAAAAAAA ACATTTTTTN GNACACATAT AAA 223

SEQ ID NO:3030
SEQUENCE LENGTH:132
SEQUENCE TYPE:nucleic acid
TOPOLOGY:linear
CLONE:HUMGS03573
SEQUENCE DESCRIPTION:
GATCCATCTC AGTCACTTTT TCCCCTGCAA TGCGTCTTGT GAAATTGTGT AGAGTGTTTG 60
TGAGCTTTTT GTTCCCTCAT TCTGCATTAA TAATAGCTAA TAATAAATGC ATAGAGNAAT 120
TAAACTGTGA AA 132

SEQ ID NO:3031
SEQUENCE LENGTH:123
SEQUENCE TYPE:nucleic acid
TOPOLOGY:linear
CLONE:HUMGS03574
SEQUENCE DESCRIPTION:
GATCCAGTTT TNCTACAAAG AACAAAGTGG TTGACAACAG TGACAGTAAT TNCTGGAGCA 60
AAACCAAAAG ACATAGGTAA AGANCATATA ATTGTTAATA AAGCACACTG CTTTCAACAC 120
AAA 123

SEQ ID NO:3032
SEQUENCE LENGTH:124
SEQUENCE TYPE:nucleic acid
TOPOLOGY:linear
CLONE:HUMGS03575
SEQUENCE DESCRIPTION:
GATCCAAGTG TTTTAACAAT TGGGGCTGTT AAGTCTGACC ATACATCACT GTGATAGAAT 60
GTGGGCTTTT TCANGGGTGA AGATACAAGT CTTAACCACA GTGTAACTTA CAGTTTCCTT 120
TAAA 124

SEQ ID NO:3033
SEQUENCE LENGTH:270

SEQUENCE TYPE:nucleic acid
TOPOLOGY:linear
CLONE:HUMGS03576
SEQUENCE DESCRIPTION:

```
GATCAGAGCA AGCCTTGCCT CTTATCTTCG GGAAACCAGA ATATGTTTGG GGTGGTAGGA  60
GCCACACAGA CTCTTCTGAG CCCCCTCACT CACTTCCTTC CCCCTGTGCC TTTCCTTTGA 120
GAGTGAAGGT GGGTNGAGTT GACCAGAGAA AGGGGAGAGA ATTCGGGAAA GACCCAAAGT 180
NTTTGTAATT CTTCTTTGTC CTTTTACCTA CAGAAATGGT CACATGGTTC TATTTAAATA 240
GCCTAATAAA CACATCTGGA GCCTGTCAAA                                 270
```

SEQ ID NO:3034
SEQUENCE LENGTH:127
SEQUENCE TYPE:nucleic acid
TOPOLOGY:linear
CLONE:HUMGS03577
SEQUENCE DESCRIPTION:

```
GATCTCAAAG ATACACAGTA CCTACTTAAT TCCAGCTGAT GGGAGACCAA AGAATTTGCA  60
AGTGGATGGT TTGGTATCAC TGTAAATAAA AAGAGGGCCT GGGAATTCTT GCGATTCCAT 120
CTCTAAA                                                          127
```

SEQ ID NO:3035
SEQUENCE LENGTH:120
SEQUENCE TYPE:nucleic acid
TOPOLOGY:linear
CLONE:HUMGS03578
SEQUENCE DESCRIPTION:

```
GATCCCTACC TGCTCCCCGC TCCGGTTGCC AGCAGCACTC ACTGCACTCC TTTGTCATAT  60
ACTCTGCATC ACTGTCATAC TCACANCTTC GTGAATAAAG TTGTGTGCTT TATTCGTAAA 120
```

SEQ ID NO:3036
SEQUENCE LENGTH:120
SEQUENCE TYPE:nucleic acid
TOPOLOGY:linear
CLONE:HUMGS03579
SEQUENCE DESCRIPTION:

```
GATCAGGGAC CTCACGCATC TNTTTCTCAT ATACATGGNC TCTCTGTTGG CCTGCAAACA  60
CATTTNCTTC TCCGCTTATN AGACTATTTA NCTTTAATAA AGCACTGGAT ATAAATCAAA 120
```

SEQ ID NO:3037
SEQUENCE LENGTH:176
SEQUENCE TYPE:nucleic acid
TOPOLOGY:linear
CLONE:HUMGS03580
SEQUENCE DESCRIPTION:

```
GATCTAAAGC TNAACAATGA AAATCTTCAG CAGAAATAGA AATNGCCGTG GATTGTAATA  60
```

CACACTGAAA TTCTNACTTT CTGAATTTAA ATGTAGAATA AATTTNACCA ACTTGGAGTA 120
CTGTATGAGT ATTTCNAGTA GGGGGAATAA ACTCAAANTT ATATTTNTNT TGCAAA 176

SEQ ID NO:3038
SEQUENCE LENGTH:119
SEQUENCE TYPE:nucleic acid
TOPOLOGY:linear
CLONE:HUMGS03581
SEQUENCE DESCRIPTION:
GATCTGAAAT GTCTGAGAGT AATAGTTTCT GTTGAATTTT TTTTTGTTCA TTTTTCTGCA 60
CAGTCCATTC TGTTTTNATT ACTATCTAGG CTTGAAATAT ATAGTTTGAA ATNATGAAA 119

SEQ ID NO:3039
SEQUENCE LENGTH:234
SEQUENCE TYPE:nucleic acid
TOPOLOGY:linear
CLONE:HUMGS03582
SEQUENCE DESCRIPTION:
GATCAGAATA TGACATTGCA GTAAAGNGCT TAAATAGTTT CATCTNTGGT TTTTTTTTCT 60
AGACACTTTT CTTTTATCCC AGNCATCTCT GAATTTACAT TTTATATTAA AGAAATTGAG 120
CTATCCATAC AACCTGTATT TACTTTAATT TCACTATGCT TTCAGCTTAC TTTATTGTAT 180
GNTATGTAGG TCTAAAATAT AGTTTGAGTC AAATAAAATT AGGGGGGCTT TAAA 234

SEQ ID NO:3040
SEQUENCE LENGTH:132
SEQUENCE TYPE:nucleic acid
TOPOLOGY:linear
CLONE:HUMGS03583
SEQUENCE DESCRIPTION:
GATCATGACA CTNAAGCGTG ATGAGACGCT CCAGGATGGC TGTNATACTC ACTTCTGCAA 60
GGTCAATGAG AGAGGAGAGT ACTTCTGGGN GAAGAGGGTC ACAGGCTNCC CACCCTTTNA 120
TGAACACANG TN 132

SEQ ID NO:3041
SEQUENCE LENGTH:118
SEQUENCE TYPE:nucleic acid
TOPOLOGY:linear
CLONE:HUMGS03584
SEQUENCE DESCRIPTION:
GATCCCTAAA AATGTTGAGG GACTTCTGTT CATTCATCCC GAGAACATTG GCTTCCACAT 60
CACAGTATCT ACCCTTACAT GGTTTAGGAT TAAAGCCAGG CAATCTTTTA CTATGAAA 118

SEQ ID NO:3042
SEQUENCE LENGTH:117
SEQUENCE TYPE:nucleic acid

TOPOLOGY:linear
CLONE:HUMGS03585
SEQUENCE DESCRIPTION:
GATCGGACCG TTCATGCTGC CTATAGGCAT TATGTCCCTC AAAAAAAAAC TCCTTTNCCT 60
GCATCCTGTG TACAACATGA CATTTTTAAC CAATCCAATC TAAAAATGTN CCAGAAA 117

SEQ ID NO:3043
SEQUENCE LENGTH:117
SEQUENCE TYPE:nucleic acid
TOPOLOGY:linear
CLONE:HUMGS03586
SEQUENCE DESCRIPTION:
GATCAGAAGT GGCAGTCTAT AACTNAAAAT GTGGTAAAGT ACTTGANGCA AACATCCCGC 60
ATCGCTGTTG GACCTCTNAN ACTTTCTACT TTAACAGTTT CACAGTCTTT NCCAGTN 117

SEQ ID NO:3044
SEQUENCE LENGTH:116
SEQUENCE TYPE:nucleic acid
TOPOLOGY:linear
CLONE:HUMGS03587
SEQUENCE DESCRIPTION:
GATCTCCTCC ACCCTGTACC AGGCAGCAGC TCCAGTCCTC ACACCAGCCA AGGTCACAGG 60
CAAGAGCAAG AAGAGAAACT GACCCTGAAT GTTCAATAAA GTTGATTCTT TGTAAA 116

SEQ ID NO:3045
SEQUENCE LENGTH:116
SEQUENCE TYPE:nucleic acid
TOPOLOGY:linear
CLONE:HUMGS03588
SEQUENCE DESCRIPTION:
GATCTGAAAT CNNGGTCTTC CAAGGNAGAA AATCGANCTG GGAATAAAAG AGAAGACCTG 60
TGATGGGGCA GCAGTGACGC NCTGTGGGGG GACAGGTGGA CGTGGAGAGC TCTTTN 116

SEQ ID NO:3046
SEQUENCE LENGTH:116
SEQUENCE TYPE:nucleic acid
TOPOLOGY:linear
CLONE:HUMGS03589
SEQUENCE DESCRIPTION:
GATCCTGAAG AAATCATATG TTAAACATAC TTTNACACCT ACTGTGTTAT AAAATATATC 60
ATCAGATGTG CCTTGAGAAT AGTATATGTA ACATTAAAAA AAAGTTGCTG GCTAAA 116

SEQ ID NO:3047
SEQUENCE LENGTH:115
SEQUENCE TYPE:nucleic acid

TOPOLOGY:linear
CLONE:HUMGS03590
SEQUENCE DESCRIPTION:
GATCCTTGGA CTTTGTGTTT TTGATTNTAT GTTGATATTC TAAAAACATC TATTTTAATG 60
TTATTTCTGT TCTAAAAATA AGATAATAAA TATTAACAAA CTTTGCTTTT TTAAA 115

SEQ ID NO:3048
SEQUENCE LENGTH:179
SEQUENCE TYPE:nucleic acid
TOPOLOGY:linear
CLONE:HUMGS03591
SEQUENCE DESCRIPTION:
GATCTCAACA AGAAGCTGTA TTTTAAGTAT TAAGACAGTT CTTTGTTAGC TGGTTTCTAG 60
TTGGTTATCT AGTTACCAAT GCTGCAGTCC TGCAGTCACC TATACATTAT TTAAATNTAT 120
TTAACTGTTA AATGCGCTAC CCACCAATAA TGAAATAGAC CTTTATGAAA ACTGTGAAA 179

SEQ ID NO:3049
SEQUENCE LENGTH:239
SEQUENCE TYPE:nucleic acid
TOPOLOGY:linear
CLONE:HUMGS03592
SEQUENCE DESCRIPTION:
GATCCTGAGG AAAGACTTGG AAGAGACCAG CATGTCTTCA GTAGCCAAAC TACTTCTTGA 60
GCATAGATAG GTATAGTGGG TTTNCTTGAG GTGGTAAGGC TTTGCTGGAC CCTGTTGCAG 120
GCAAAAGGAG TAATTGATTT AAAGTACTGT TAATGATGTT AATGATTTNT TTTTAAACTC 180
ATATTATTGG GATTTTCACC CAAAATAATG CTTTTTGAAA AAAGGAAAAA AAAACCGGN 239

SEQ ID NO:3050
SEQUENCE LENGTH:114
SEQUENCE TYPE:nucleic acid
TOPOLOGY:linear
CLONE:HUMGS03593
SEQUENCE DESCRIPTION:
GATCTTGGCT TTCTNTTTCC TCTTCTCCTN CAGGGTGTCT GTNACTGCNN GGTACTTCCA 60
GCCAACCTCG TGAGCCAGGC GCCCCAGATA GGCAAACTTT CTTGTAGGCT TCAN 114

SEQ ID NO:3051
SEQUENCE LENGTH:113
SEQUENCE TYPE:nucleic acid
TOPOLOGY:linear
CLONE:HUMGS03594
SEQUENCE DESCRIPTION:
GATCTGTATA TGCTATCCTA ACTGTTAATT GTATTATTGA TTATNTTGAT TATCTTGCTT 60
GAAGGTTCAT ACTTTTCAAT TTGATAGAAA TAAAGTTTTT NCCTGCTTAT AAA 113

SEQ ID NO:3052
SEQUENCE LENGTH:113
SEQUENCE TYPE:nucleic acid
TOPOLOGY:linear
CLONE:HUMGS03595
SEQUENCE DESCRIPTION:
GATCCTCTCT TCATTTTACT CAGCCAGGTT TTGTACTGAT GTACAGGTGT TAAATNACTT 60
CAAGTATTTT TGTAAGAGGT GTATATAATT CAATAAAAAA GGTAAAACAT AAA 113

SEQ ID NO:3053
SEQUENCE LENGTH:161
SEQUENCE TYPE:nucleic acid
TOPOLOGY:linear
CLONE:HUMGS03596
SEQUENCE DESCRIPTION:
GATCGACAAG GAGTACTTGC TAAAAATGGC AACAGAGGAG TGAGGAGTGC TGCTGTAGAT 60
GACAACCTCC ATTCTATTTT AGAATAAATT CCCAACTTCT CTTGCTTTCT ATGCTGTTTG 120
TAGTGAAATA ATAGAATGAG CACCCATTCC AAAGCTTTAA A 161

SEQ ID NO:3054
SEQUENCE LENGTH:111
SEQUENCE TYPE:nucleic acid
TOPOLOGY:linear
CLONE:HUMGS03597
SEQUENCE DESCRIPTION:
GATCCGNACC CCAAGCCCAT GCAACCCACG CTCGGTCCCG TTCCGGCCCC TGCNCCCCGC 60
TGCNCTTCGC TCCCCGNCCT TGCNCCGTTA GTAAACATCG CTCAAACGAA A 111

SEQ ID NO:3055
SEQUENCE LENGTH:110
SEQUENCE TYPE:nucleic acid
TOPOLOGY:linear
CLONE:HUMGS03598
SEQUENCE DESCRIPTION:
GATCTAACAA GTTACATTGT ATAGTTTCTG GGACTGTTTG AATATTCTAT TCCAATGGGC 60
ATTTATTTTT TATCCTGTCA TTAAAAAAAA AAGACTGTTA CCCTGCTAAA 110

SEQ ID NO:3056
SEQUENCE LENGTH:109
SEQUENCE TYPE:nucleic acid
TOPOLOGY:linear
CLONE:HUMGS03599
SEQUENCE DESCRIPTION:
GATCTCCTCC ATCCTGCTGC CCACCCCTCT TGAGTGTCTT AAGGGACAGA NCTTTCCACC 60
CCTGGAAGAT GGAAATAAAC CTGCGTGTGG GTGGAGTGTT AGGACCAAA 109

SEQ ID NO:3057
SEQUENCE LENGTH:109
SEQUENCE TYPE:nucleic acid
TOPOLOGY:linear
CLONE:HUMGS03600
SEQUENCE DESCRIPTION:
GATCAGAGTG TTNTAAATCA TCACGCCCTT AGCTTATCCC TNGTCGAGCC AGGACACGGG 60
GTGCTTCAGT GGGTCTGTNA CCCTCTCTCC TTGAAGCATG TTGCTGNNN 109

SEQ ID NO:3058
SEQUENCE LENGTH:108
SEQUENCE TYPE:nucleic acid
TOPOLOGY:linear
CLONE:HUMGS03602
SEQUENCE DESCRIPTION:
GATCCCTGAA TGTGAACCTG AAGTTCAAAG GACTTGGAAA GCTCTGGAAT GTGTTGGTTT 60
TTCCCCCCCA AAATGGGTCC TAAGGAGGGT AAAGTGACTT GTTTCAAA 108

SEQ ID NO:3059
SEQUENCE LENGTH:108
SEQUENCE TYPE:nucleic acid
TOPOLOGY:linear
CLONE:HUMGS03603
SEQUENCE DESCRIPTION:
GATCAGTTTC TTTNGCTTCA AGATTTGAAA CCAGAAAGGA AAGTCCNACT TGCAGATGAT 60
TGTCCTTAAA ACCTAATGGA AAANTAAAAG ANAGACATAC CATGGAAA 108

SEQ ID NO:3060
SEQUENCE LENGTH:107
SEQUENCE TYPE:nucleic acid
TOPOLOGY:linear
CLONE:HUMGS03604
SEQUENCE DESCRIPTION:
GATCCATCAT TGTTCACAAA CTTCACTGTC AAGAACCTTT TCGTTGCATA ATGTGCAAAT 60
AGTTAAAACC ACTGTACTAT ATACTTAAAA ATGATTAAGA TAGTAAA 107

SEQ ID NO:3061
SEQUENCE LENGTH:252
SEQUENCE TYPE:nucleic acid
TOPOLOGY:linear
CLONE:HUMGS03605
SEQUENCE DESCRIPTION:
GATCTGGCAG CTGAGGCCCG AGGCAGGGTT GGNGTGATGC CAGGGCAAAG TGGTGAGGAG 60
AAAACAGGAA NCGGGCTTTC NCCNCATTGG TAAATGGGAA AGAGGTGAGC AACTTAAGAT 120

TGTCACAATT AATCACAAGT GTACAGGATT AGACTGGGTT TATATTTAAC TCTTGCTNCA 180
TAGGTGTACC ATTTAAAGAG TGTTATTTAA TGCTAAGTTT AACTGCTTTA ATAAAGTTTA 240
TTTTTAAATA AA 252

SEQ ID NO:3062
SEQUENCE LENGTH:103
SEQUENCE TYPE:nucleic acid
TOPOLOGY:linear
CLONE:HUMGS03606
SEQUENCE DESCRIPTION:
GATCAACAAA ATAACTTGTA TCTCCTGCTT AANTCCTTTA TACTACCTGG NGAAAAGTAT 60
TTCCCATATT TCCAATAAAT GTCAGACACT ATTGAGAAAG AAA 103

SEQ ID NO:3063
SEQUENCE LENGTH:103
SEQUENCE TYPE:nucleic acid
TOPOLOGY:linear
CLONE:HUMGS03607
SEQUENCE DESCRIPTION:
GATCCTGNAT NANGCCCGGG CCAGCGAGTG CAGGGACGTG GAGGGNTTCA AAACCGAGAT 60
GGCCATGCTG GTGACCCAGG CCAGGAAGAA CACCATCACC CNN 103

SEQ ID NO:3064
SEQUENCE LENGTH:159
SEQUENCE TYPE:nucleic acid
TOPOLOGY:linear
CLONE:HUMGS03608
SEQUENCE DESCRIPTION:
GATCCTCACA GTCAAGTGAC GAGGCTGGGG CTGAAAGCAG AAGCATGCAC AGGGAGGAGA 60
CCACTTTTAT TGCTTGTCTN GGTGGATGGG TCAGGAGGGT CTGAGGGCCT GTCCCAGACA 120
ATAAAGGTNC CCTCAGCGGA TGTGGGCCAT GTCACCAAA 159

SEQ ID NO:3065
SEQUENCE LENGTH:102
SEQUENCE TYPE:nucleic acid
TOPOLOGY:linear
CLONE:HUMGS03609
SEQUENCE DESCRIPTION:
GATCCTATCG CCTTTNAGTA GAATATGAAA TATTCTTTNA GAAATCCAAT ATAAATAGGT 60
TATAATAGCC ATATNCTTNA TNACTTTATT GAGATATAAT TN 102

SEQ ID NO:3066
SEQUENCE LENGTH:186
SEQUENCE TYPE:nucleic acid
TOPOLOGY:linear

CLONE:HUMGS03610
SEQUENCE DESCRIPTION:
GATCTNAGCT CCCACCTCCC TTCCCACCTA CTGCACTTTC CCCCTTCCCG CCTTCCAAAA 60
CCTGCTTCCT TCAGTTTGTA AAGTCGGTGA TTATATTTNT GGGGGCTTTC CTTTNATTTN 120
TAAAATGNAA AATTTATNTA TANTCCGTAT TTAAAGTTGT AAAAAAAATA NCCACAGAAC 180
AGNGAN 186

SEQ ID NO:3067
SEQUENCE LENGTH:100
SEQUENCE TYPE:nucleic acid
TOPOLOGY:linear
CLONE:HUMGS03611
SEQUENCE DESCRIPTION:
GATCTCAGAC AGTGTGCGGC GGCACATGAG CGGCTTTCCG CAGTGTCCTG TNTTGAGCCA 60
CTGCAGTCTG GGCCCCATCA TTAAACGGGC TGCGTTTAAA 100

SEQ ID NO:3068
SEQUENCE LENGTH:98
SEQUENCE TYPE:nucleic acid
TOPOLOGY:linear
CLONE:HUMGS03612
SEQUENCE DESCRIPTION:
GATCTCCAGT NAGACTCCAG GGTTCTAGAC AGAGGCCAGT NTTGGCCACT CACCCCACAC 60
CCCGCGGACC CCAATAAAGG CTTGCATAGC TCCAGAAA 98

SEQ ID NO:3069
SEQUENCE LENGTH:98
SEQUENCE TYPE:nucleic acid
TOPOLOGY:linear
CLONE:HUMGS03613
SEQUENCE DESCRIPTION:
GATCCTGAAG CTTNACCGGG CTCCAAAGGG CAAATGGAGC TGCCCTCNTC TGTATCAGGT 60
TTATTGGTTG TACATATAAA TNATACTTNC CTTTCAAA 98

SEQ ID NO:3070
SEQUENCE LENGTH:107
SEQUENCE TYPE:nucleic acid
TOPOLOGY:linear
CLONE:HUMGS03614
SEQUENCE DESCRIPTION:
GATCCTGTTT AACAAAGATA CTTGAGACAT CCATTTGTTT TAATGAAATC TGTATGGATA 60
TGGAAATNCT TGCCCTAATA AAAGCCTACA TATACCTTCT GGTTAAA 107

SEQ ID NO:3071
SEQUENCE LENGTH:97

SEQUENCE TYPE:nucleic acid
TOPOLOGY:linear
CLONE:HUMGS03615
SEQUENCE DESCRIPTION:
GATCTNTCCG TTCTCAGGCC AGACCCCTGG TGCTGCCGTT AATTTTTTTT TCTCTGTCTT 60
TGCTGCAATN TTNAAATAAA ATGCCAAAGA ACACAAA 97

SEQ ID NO:3072
SEQUENCE LENGTH:95
SEQUENCE TYPE:nucleic acid
TOPOLOGY:linear
CLONE:HUMGS03616
SEQUENCE DESCRIPTION:
GATCCTAAAC AAATCATCTT TGTCAGTTAA GTATAGTTGC GCAAAAATTG TTAAATCCTT 60
TGTCTTTATT AAAGAAAAAT TTGAGTAACA TTAAA 95

SEQ ID NO:3073
SEQUENCE LENGTH:94
SEQUENCE TYPE:nucleic acid
TOPOLOGY:linear
CLONE:HUMGS03617
SEQUENCE DESCRIPTION:
GATCTAAGTG ATGAGTCTAG GTGTGTTATT GTNCAGTTTT TNCAACTTTC CTGGAAAACG 60
AACTTTTCAA AATAAAAAAT TGGAAAAATA GAAA 94

SEQ ID NO:3074
SEQUENCE LENGTH:183
SEQUENCE TYPE:nucleic acid
TOPOLOGY:linear
CLONE:HUMGS03618
SEQUENCE DESCRIPTION:
GATCACTTTG AGAAACAAAC TTTTATTAAA TGTAAGGCAC TTTTCTATGA ATTTTAAATA 60
TAAAAATAAA TATTGTTCTG ATTATNACTG AAAAGATGTC AGCCATTTCA ATGTCTTGGG 120
AAACAATTTT TTGTTTTTGT TCTGTTTTCT TTTTGCTTCA ATAAAACAAT AGCTGGCTCT 180
AAA 183

SEQ ID NO:3075
SEQUENCE LENGTH:92
SEQUENCE TYPE:nucleic acid
TOPOLOGY:linear
CLONE:HUMGS03619
SEQUENCE DESCRIPTION:
GATCTGCCAT ATTTGNTNTT GGTTTTAATT TTCATATCCA GCCTAAAGGT GGTTGTTTAT 60
NATATAGTAA TAAATCATTG CTGTACAATA AA 92

SEQ ID NO:3076
SEQUENCE LENGTH:92
SEQUENCE TYPE:nucleic acid
TOPOLOGY:linear
CLONE:HUMGS03620
SEQUENCE DESCRIPTION:
GATCAGGCTG CCTCCTGGNT TGGNTGTCGG GGGAGCTGTC CGGCAGCCTG GCAGGGAGAT 60
GCAAGGCCTA AAGTAAAATT TTNTCAAGTA AA 92

SEQ ID NO:3077
SEQUENCE LENGTH:91
SEQUENCE TYPE:nucleic acid
TOPOLOGY:linear
CLONE:HUMGS03621
SEQUENCE DESCRIPTION:
GATCTAGCCC CTTCTTCAGG CCGAGAGAAT TTCGAGCGTT ANCCGTCTCT TGGCCACCAG 60
CTAAATAAAC GGATTCTTCA TGTGTCTCAA A 91

SEQ ID NO:3078
SEQUENCE LENGTH:91
SEQUENCE TYPE:nucleic acid
TOPOLOGY:linear
CLONE:HUMGS03622
SEQUENCE DESCRIPTION:
GATCTGCTAT TGTTATTTCT CCTCTTTATT GGAAAAAGGC CTCAGTTTTA ATTATTTTCT 60
TCCCAAAATA AATNACACAT TTGGTTACAA A 91

SEQ ID NO:3079
SEQUENCE LENGTH:239
SEQUENCE TYPE:nucleic acid
TOPOLOGY:linear
CLONE:HUMGS03623
SEQUENCE DESCRIPTION:
GATCCTGCAT CAGCATTGTA TATATGGNCT TAAGTGCCTG GCCTCCTTAT CCTTCAGAAT 60
ATTTATTTNA CTTACAATCC TCAAGTTTTA ATTGATTTTA AATATTTTCC AATACAACAG 120
TTTAGGTTTA AGATGACCAA TGACAATGAC CACCTTTGCA GAAAGTAAAC TGATTGANTA 180
ANTAANTCTC CGTTTNNTTC ANTTNATTTC AGTGTAATGA NAANGTTGCT TAGTATTTN 239

SEQ ID NO:3080
SEQUENCE LENGTH:196
SEQUENCE TYPE:nucleic acid
TOPOLOGY:linear
CLONE:HUMGS03624
SEQUENCE DESCRIPTION:
GATCCCTGCC CTGCACCAGC ACCCTGANAT GGAGCTGAGA CTTTATTTAT TGGGGGTAGG 60

GGGATGGAGG AGGTCCCTCC AACATGTTTG GACCCAGCTC CTTTGGGTTC CACTGACACC 120
CCTGCCCCTG CCCCTGCCCA GAACCAAGTG CCATTTCTCA CTCTGGAGCC TTAATAAACT 180
GCAATTTGTA TCCAAA 196

SEQ ID NO:3081
SEQUENCE LENGTH:90
SEQUENCE TYPE:nucleic acid
TOPOLOGY:linear
CLONE:HUMGS03625
SEQUENCE DESCRIPTION:
GATCTGTGTA CTGTTTACTT CCCACTTCCC AGAATCCCTT GTATCTCCTT TCTCGGGAAT 60
TGTATTTTCT AATAAATGAC ATTTGAGAAA 90

SEQ ID NO:3082
SEQUENCE LENGTH:90
SEQUENCE TYPE:nucleic acid
TOPOLOGY:linear
CLONE:HUMGS03626
SEQUENCE DESCRIPTION:
GATCTCTGGA AGTGTAATTN TGAAAGAAAC TTNCTTGCAG CTTTAACAAA ATGAGAAACT 60
TNCCAAATAA AACTTGTTTT GAAGTTTAAA 90

SEQ ID NO:3083
SEQUENCE LENGTH:89
SEQUENCE TYPE:nucleic acid
TOPOLOGY:linear
CLONE:HUMGS03627
SEQUENCE DESCRIPTION:
GATCCTCGGG AAGAACAAAG CTAAAGCTGC CTTTTGTCTG TTATTTTATT TTTTTGAAGT 60
TTAAATAAAG TTTACTAATT TTGACCAAA 89

SEQ ID NO:3084
SEQUENCE LENGTH:88
SEQUENCE TYPE:nucleic acid
TOPOLOGY:linear
CLONE:HUMGS03628
SEQUENCE DESCRIPTION:
GATCAATCTT TGATGGTGAG GGTTTTAGAA AGGAAAAATA TAGTAAAATG TGTAATTTGT 60
CTTAATAAAA TCTATCTNTA CATCTAAA 88

SEQ ID NO:3085
SEQUENCE LENGTH:94
SEQUENCE TYPE:nucleic acid
TOPOLOGY:linear
CLONE:HUMGS03629

SEQUENCE DESCRIPTION:
GATCTAGATT GGGTGAGGGG GACGGGGATG TNAGGGAGGC AAGTGTGTTN TGTTACTGTG 60
TCAATAANCT GGTTTAAAGT TGTAAAGTGN GAAA 94

SEQ ID NO:3086
SEQUENCE LENGTH:89
SEQUENCE TYPE:nucleic acid
TOPOLOGY:linear
CLONE:HUMGS03631
SEQUENCE DESCRIPTION:
GATCTGAATT TCACAAAAAG TTCATGGAGA AATATATTAA ATAGTACAGT TTTATGTGCT 60
TAATTAAAGA CTGTAAAACG TAAAGGAAA 89

SEQ ID NO:3087
SEQUENCE LENGTH:84
SEQUENCE TYPE:nucleic acid
TOPOLOGY:linear
CLONE:HUMGS03632
SEQUENCE DESCRIPTION:
GATCATACCA CGGCACTCCA GACTGGGGAC CAAAGTGAGA CCTTGTTTCT AAAANTTAAA 60
ATATTTAGAA TGTTTCCTAG CAAA 84

SEQ ID NO:3088
SEQUENCE LENGTH:82
SEQUENCE TYPE:nucleic acid
TOPOLOGY:linear
CLONE:HUMGS03633
SEQUENCE DESCRIPTION:
GATCCCAAGG ATGGAGAACA ACTTACCCAG TAGCTAGAAT GTTAATGGCA GAAGAGNAAA 60
CAATAAATCA TATTGACTCA AA 82

SEQ ID NO:3089
SEQUENCE LENGTH:81
SEQUENCE TYPE:nucleic acid
TOPOLOGY:linear
CLONE:HUMGS03634
SEQUENCE DESCRIPTION:
GATCTCCAGA TGTGCATTTC CCTCTCTTAT TTTAAGTTAT GTTAAGATTA CTAAAACAAT 60
AAAAGCTCCT AAAAAATCAA A 81

SEQ ID NO:3090
SEQUENCE LENGTH:79
SEQUENCE TYPE:nucleic acid
TOPOLOGY:linear
CLONE:HUMGS03635

SEQUENCE DESCRIPTION:
GATCCAACCG TGCCTGAAGC TAGAATATCC CCTGGACTTT TCAGTTATGT GAACCAATAA 60
ATACCCTTTT TTGCTTAAA 79

SEQ ID NO:3091
SEQUENCE LENGTH:78
SEQUENCE TYPE:nucleic acid
TOPOLOGY:linear
CLONE:HUMGS03636
SEQUENCE DESCRIPTION:
GATCTGNGAN ATATTATGGT AATTGTGTCG GGGCGCCATG AACCGCACCC ATATAACACG 60
GTAAACTTAA TCAGCAAA 78

SEQ ID NO:3092
SEQUENCE LENGTH:77
SEQUENCE TYPE:nucleic acid
TOPOLOGY:linear
CLONE:HUMGS03637
SEQUENCE DESCRIPTION:
GATCATGCTG AAAACCACTC AAACGATTAT ATTTTTTACA TTATAAGAAG TAAAATATAT 60
TATTCTGAAT AATGAAA 77

SEQ ID NO:3093
SEQUENCE LENGTH:75
SEQUENCE TYPE:nucleic acid
TOPOLOGY:linear
CLONE:HUMGS03638
SEQUENCE DESCRIPTION:
GATCTGTGCA TTGCTCCTTC TGATTTGTGC TAAGTTGTTA AATAAGAGGG ACCCTGAGAA 60
AGGCCTTACT CTAAA 75

SEQ ID NO:3094
SEQUENCE LENGTH:75
SEQUENCE TYPE:nucleic acid
TOPOLOGY:linear
CLONE:HUMGS03639
SEQUENCE DESCRIPTION:
GATCACACAG CTAACGAGGC TGCCTCCAGC ATTTCCTGAT TTCCTCTGTG GTAATAAAAG 60
CTTTCTGTGC TTAAA 75

SEQ ID NO:3095
SEQUENCE LENGTH:78
SEQUENCE TYPE:nucleic acid
TOPOLOGY:linear
CLONE:HUMGS03640

SEQUENCE DESCRIPTION:
GATCAGAACT TACAAACCAA ACTTTTATTC TGAGAAACTG GCTGTACAAT ATNTGAAAAG 60
AAAGTGACAT GNAGGAAA 78

SEQ ID NO:3096
SEQUENCE LENGTH:74
SEQUENCE TYPE:nucleic acid
TOPOLOGY:linear
CLONE:HUMGS03641
SEQUENCE DESCRIPTION:
GATCTTTTTN AAGTNTTCTG AAAGGAAGTA GACAGTATTA CACCCTGAAT AAATAAGGTG 60
TTGTTTTCCA CAAA 74

SEQ ID NO:3097
SEQUENCE LENGTH:267
SEQUENCE TYPE:nucleic acid
TOPOLOGY:linear
CLONE:HUMGS03642
SEQUENCE DESCRIPTION:
GATCTACTTC CCATGCAGAA GAGAAGTCAC ATCTTCCAGG GAATCGCAAT GTTGTGGCGT 60
CTGACTTGTA TGTCACATTT GTGTAAAATG GTATATNCTT TAAAATAGTG TTGATAACTG 120
GAATATTGTA TGTATGCTTG GAGATGCTTT GTGTGAACCT AAGACTGTCA CTCAACAGAT 180
GTTGGATTGG GGNAAATCCA AAGCACAACT TCAAANTAAN NTACATTTTT AGGTTTCGAT 240
GCTGCAAANC ANANAANANN ACANNAN 267

SEQ ID NO:3098
SEQUENCE LENGTH:73
SEQUENCE TYPE:nucleic acid
TOPOLOGY:linear
CLONE:HUMGS03643
SEQUENCE DESCRIPTION:
GATCATGGTG ATGTACGGGG TGAATTCTCT TGCCGTGTTG CAAATGTGTA AAATAAAGAT 60
TATCTGGCAG AAA 73

SEQ ID NO:3099
SEQUENCE LENGTH:71
SEQUENCE TYPE:nucleic acid
TOPOLOGY:linear
CLONE:HUMGS03644
SEQUENCE DESCRIPTION:
GATCCNAGNT CTANCGTCAT ATAATCATTT AATATAATTA AAGTTTAATT GCTGAAATAC 60
TTATAATGAA A 71

SEQ ID NO:3100
SEQUENCE LENGTH:69

SEQUENCE TYPE:nucleic acid
TOPOLOGY:linear
CLONE:HUMGS03645
SEQUENCE DESCRIPTION:
GATCATGCAA TTCCTTCAAT TATGATGGAA AGACTTGAAC TTTCTGAAAT AAAACAAAAA 60
TACAGCAAA 69

SEQ ID NO:3101
SEQUENCE LENGTH:67
SEQUENCE TYPE:nucleic acid
TOPOLOGY:linear
CLONE:HUMGS03646
SEQUENCE DESCRIPTION:
GATCCATTTA TTGCAATTCA TGCTGAATCT AAATTATAAA ATTTAAAATT AAATGCATAT 60
CCTCAAA 67

SEQ ID NO:3102
SEQUENCE LENGTH:65
SEQUENCE TYPE:nucleic acid
TOPOLOGY:linear
CLONE:HUMGS03647
SEQUENCE DESCRIPTION:
GATCCGAAAC CTGTTAAACA ATAAGATGTG TTCTCGGAAA ATNAAACAAG AGGTATCACA 60
AGAAA 65

SEQ ID NO:3103
SEQUENCE LENGTH:64
SEQUENCE TYPE:nucleic acid
TOPOLOGY:linear
CLONE:HUMGS03648
SEQUENCE DESCRIPTION:
GATCATCTCT TCTCCATAAG ATAGTGTGAT AAACACAGTC ATGAATAAAG TTATTTTCCA 60
CAAA 64

SEQ ID NO:3104
SEQUENCE LENGTH:64
SEQUENCE TYPE:nucleic acid
TOPOLOGY:linear
CLONE:HUMGS03649
SEQUENCE DESCRIPTION:
GATCGCTCCA ATAAACATAT ATTGTGAAAA TGCATCCACA ATAAATGGAA TTCCTTCCTG 60
CAAA 64

SEQ ID NO:3105
SEQUENCE LENGTH:63

SEQUENCE TYPE:nucleic acid
TOPOLOGY:linear
CLONE:HUMGS03650
SEQUENCE DESCRIPTION:
GATCTACCTG TCTTGTTGCC ACATGTTTCT AAACTTTCCA ATAAATCACC TTCTACTGAC 60
AAA 63

SEQ ID NO:3106
SEQUENCE LENGTH:68
SEQUENCE TYPE:nucleic acid
TOPOLOGY:linear
CLONE:HUMGS03651
SEQUENCE DESCRIPTION:
GATCAGCATC AGGCTGTTAT CAAGTCTGTT GGTGCTAAAA AGTAAAANAT GAAATGTTCA 60
AAGAGAAA 68

SEQ ID NO:3107
SEQUENCE LENGTH:58
SEQUENCE TYPE:nucleic acid
TOPOLOGY:linear
CLONE:HUMGS03652
SEQUENCE DESCRIPTION:
GATCTGTCTG GCTCCNTTGT TTCTTATTAA ATAAAATTCT GTCTTCTATT GTTTCAAA 58

SEQ ID NO:3108
SEQUENCE LENGTH:58
SEQUENCE TYPE:nucleic acid
TOPOLOGY:linear
CLONE:HUMGS03653
SEQUENCE DESCRIPTION:
GATCAGCCTT NNTATAATGT TTTTTAAATC ATTTCTAAAT AAAACAGAAA TACAGAAA 58

SEQ ID NO:3109
SEQUENCE LENGTH:55
SEQUENCE TYPE:nucleic acid
TOPOLOGY:linear
CLONE:HUMGS03654
SEQUENCE DESCRIPTION:
GATCTAGACT TATATGAATA AATNAAATTA CATGCCAAGG GCCCTAAAAA GCAAA 55

SEQ ID NO:3110
SEQUENCE LENGTH:53
SEQUENCE TYPE:nucleic acid
TOPOLOGY:linear
CLONE:HUMGS03655

SEQUENCE DESCRIPTION:
GATCAGGGCC GAGCAGAACC GCACTCTTCC CAAATAAAGC TTCCTCCTTG AAA 53

SEQ ID NO:3111
SEQUENCE LENGTH:55
SEQUENCE TYPE:nucleic acid
TOPOLOGY:linear
CLONE:HUMGS03659
SEQUENCE DESCRIPTION:
GATCATATTT TTTNATACAG GTATTTCAAT TAAAATGTTT TTGTACATAG TGAAA 55

SEQ ID NO:3112
SEQUENCE LENGTH:76
SEQUENCE TYPE:nucleic acid
TOPOLOGY:linear
CLONE:HUMGS03661
SEQUENCE DESCRIPTION:
GATCCAATCA NTTAAATAAA CCTGTATGTA TATGCATATG TNTACATGCA TATCCCCTCC 60
TGCTAAAACT CATAAA 76

SEQ ID NO:3113
SEQUENCE LENGTH:451
SEQUENCE TYPE:nucleic acid
TOPOLOGY:linear
CLONE:HUMGS03679
SEQUENCE DESCRIPTION:
GATCCCTCAG TTTCTTCTTG ATTTCAGCAT GTNTCGGGTT CCTAATTTAT GGTATGAGTT 60
AGCAAATTTA ACCATTGTGT TTGTGCCCTA CCCAGGGGAC TCCCCAGTTT CTGACTTGAA 120
GTAGACTGAG AAGAATCCAC GAGGTGCTAT CTGGCCAGAT TTAAGTAGAT TCTATTTCCT 180
TGGTTCTCCC TCTCCCTGAG GACCTCTTAT TTNATTGTCC CCTCTTCTAG GTTAATTCTC 240
CTTTGATTTG ACTTTGTTGA GAAGGAGGTT GGACAGTAGA TTAGCAAAGT TCCAAGTGCA 300
AAATTACAGT GTGTTAGAGT GTGGGGGGAA AATTAGTCTT ATTTTTCCCT ACATGGGATA 360
CAACACTGTG AATTCAATCT TCAACCTGAA GGGCCCTGCA GTTCCTCCTA AAACATNGTT 420
GTTTGNTTTT CTTTAACCAA GGTTTAAGCT N 451

SEQ ID NO:3114
SEQUENCE LENGTH:445
SEQUENCE TYPE:nucleic acid
TOPOLOGY:linear
CLONE:HUMGS03680
SEQUENCE DESCRIPTION:
GATCTAAGGA TGACTTGGAC ACACTCCNTG GCACTGAAGA GTCTGAACAN TGGCCTGTNA 60
TTGGTCCATT CCAGGACCTT CATTTGCATA AGGTATCAAA CCACANCAGC CTCTGATTGG 120
CCATGGGCAG ACCTGCACTN TGGCCAATNA TTGGTTCATT CCAGGACATT CATTTGCATA 180
AGGAGTCAAA CCACANCAGT CTTGGATTGG CTGTGAGCCA ATTCACCTCA GTCTCTAATT 240

GGCNNNGAGT CAGTCTTTCA TTTACATAGG GTGTAACCAT CAAGAAACCT CTACAGGGTA 300
CTTAAGCCCC AGAAGATTTT NCTACCAGGG CTCTTGAGCN ACTTGCTCTA GCCCACTCCC 360
ACCCTGTGGA ATGTNCTTTC ANCTTTTGNT TGGTTTCAAG TNCCTTGGTG CTTCCAATAA 420
NTNCCACTTC CTTCACCACC CNAAA 445

SEQ ID NO:3115
SEQUENCE LENGTH:439
SEQUENCE TYPE:nucleic acid
TOPOLOGY:linear
CLONE:HUMGS03681
SEQUENCE DESCRIPTION:
GATCGGCGCC ACTGCTGTCC TGCTGGCTTC NNCTGCGCAG CANGGGGTAC CAAGTGTTTN 60
CCCAGGGAGG CCCCGCGCTG GGACGCCCCT TTAAGGGACC CAGCCTTGAN ACAGCTGCTG 120
TAAGGGACAG TACTGAAGAC TCTGCAGCCC TCGGNACCCC ACTCGGAGGG TGCCCTCTGC 180
TCAGGCCTCC CTAGCACCTC CCCCTAACCA AATTNTCCCT GGACCCCATT CTNAGCTCCC 240
CATCACCATG GGAGGTGGGG CCTCAATCTA AGGCCTTCCC TGTAAGAAGG GGGTTGTGGC 300
AAAAGCCACA TTACAAGCTG CCATCCCCTC CCCGTTTNAG TGGACCCTGT GGCCAGGTGC 360
TTTTCCCTAT CCACAGGGGT GTTTTGTGTG TGTGCGCGTG GTGCGGTTTN AATAAANGTT 420
TGTACAACTT NTGTTTAAA 439

SEQ ID NO:3116
SEQUENCE LENGTH:431
SEQUENCE TYPE:nucleic acid
TOPOLOGY:linear
CLONE:HUMGS03682
SEQUENCE DESCRIPTION:
GATCTTGAAG TGCTTTTTAT ACAGCTCTCT AATAATTACA AATATCCGAA AGTCATTTCT 60
TGGAACACAA GTGGAGTATG NNAAATTNNN TATGAATTTT TCAGATTATC TAAGCTTGCA 120
GGTTTTATAA TTAGAAGATA ATGTGAGANT TAATGGGGTT TATATTTACA TTATCTCTCA 180
ACTATGTAGC NCATATTACT CACCCTATGA GTGAATCTGG AATTGCTTTT CATGTGAAAT 240
CATTGTGGTC TATGAGTTTA CAATACTGCA AACTGTGTTA TTTTATCTAA TCCATTGCTT 300
AATGAGTGTG TTTTTCCATG ATTGAATATA CCGTGGTTCA TATGTTAGCA TGGCAGCATT 360
TTCAGNTAGC TTTTTGGTTT GTTGGGAAGT TGGGGTTTTG GGGGGAGGGG GGAGTNTTNG 420
TACGTTGCCT N 431

SEQ ID NO:3117
SEQUENCE LENGTH:427
SEQUENCE TYPE:nucleic acid
TOPOLOGY:linear
CLONE:HUMGS03683
SEQUENCE DESCRIPTION:
GATCAAAGTA TTATATGCTG TGTGCTTTTA AGGTGTTTGT AAGTACTGAA AAAGGCAAAA 60
ATNCTTTCTA CATTGACATT CATTCCTATT TNACTGGGCA CCTATGAATG TATGCTGTGT 120
GCTAGAAATA GACTAAAACA TATTCCTATA GCATGTNAGT GTGTTTGCAT GTTTGCTGAA 180
AATCCTTTGT GTATAANCCA GTTTGTAAGG TTCTCTGGGT TAGGTAGGGA CTCTGCAGTT 240

TCTNCCTGTC NAAATCTCTC CTACCAAGAT GGTGTTCCAC TGTCCAGCCC AGCATGAGTA 300
GCAGGTAGNG CACAGCTTTA CTGGCTGGTT TGTATGCTTT GGTTTTAGGT GCAATTGTTG 360
TGGGTNGATT TACTTTNTCC AGAAAACCAT NGTATTGNNN ATGTCCTNGG ACCGGTGGAA 420
AATAGGN 427

SEQ ID NO:3118
SEQUENCE LENGTH:423
SEQUENCE TYPE:nucleic acid
TOPOLOGY:linear
CLONE:HUMGS03684
SEQUENCE DESCRIPTION:
GATCAAAAAT ATTAGAAAAA ATAAAANTAA TACAAATAAA AATACAGTAT AACAGTTATT 60
TAAATAGCAT TTACATTGCA TTAGGTATTA GTCTAGGGAT AAAGTATACA GGCGGATGTN 120
CGTTGGTTAT ATACAAATAT GTCATTTTAT GNAANNGACT TGAGTATACT TGGNTTTTNG 180
GTATCTNTGG GTTGGGGGGA CGGTCCAGGA ACCAATACCC CATGGATACC AAGGGACAAC 240
TGTACTTATT TNCCNTTATT GTCATTGCAA GCTTCTTATG GAAACTTTAT AGGGAATGAN 300
AATATACATG TTAAGGANGA TTAAACATTA GATAGTAGAT GGTTTGTTGC ATGCTAGAAC 360
TGTTAGTATT GTTGATTCAA TTACTTTGGT TTCATGAAAA ANTAAACGGT AANTATCTTT 420
AAA 423

SEQ ID NO:3119
SEQUENCE LENGTH:421
SEQUENCE TYPE:nucleic acid
TOPOLOGY:linear
CLONE:HUMGS03685
SEQUENCE DESCRIPTION:
GATCGAGACC ATCCTGGCCA ACATGGTGAA AACTCATCTT TACTAAAATA CAAAAAATTA 60
GCCACGCNTG GTGGCGCACG TTGTGGTCCC AGCTACTCGG GAGGCTGAGG CAGTGGAGTC 120
ACTTGAACCC GGGAGGCAGA GGTTGCAGTG AGCCGAGATT GCACCACTGT ACTCCAGCCT 180
GGCGACAGAG CAAGATTCTN TNTGCCCCCC CACCAAAAAA AAAGANTGAA ACACTGATAT 240
TTGCTACAAC ATGGNTATAC CTCCAAAGCA TCCTGTTAAG TGAAAACAGG GAGACACAGT 300
CACGTACTAT ATGATTGCNC TTACGTGAAA TATCCACANN GACGNNTCCA TGGNGACCAG 360
GCAGATTGCC GNTTGCCAGA GGCCAAGGGT TAGGGAAAAA TTGGTGAGGG ACCCTGCGTG 420
N 421

SEQ ID NO:3120
SEQUENCE LENGTH:410
SEQUENCE TYPE:nucleic acid
TOPOLOGY:linear
CLONE:HUMGS03686
SEQUENCE DESCRIPTION:
GATCTATGAC TTCTCAAAGC CGGGTCATGA ATGTCCATAA AATGCTAGGC ATTCCTATTT 60
CCAATATTTT NATGGTTGGA AATAATGCTT CANATTTGGA ACTGGACCCC ATGAAGGATA 120
TTCTCATCCT CTCTGCACTG AGGCAGATGC TGCGGGCTGC AGATGATTTT TTAGAAGATT 180
TGCCTCTNGA GGAAACTGGT GCAATTGAGA GAGCGTTACA GCCCTGCATT TGAGATAAGT 240

TGCCTTGATT CTGACATTTG GNCCAGCCTG TACTGGTGTG CCGCAATGAG AGTCAATCTC 300
TATTGACAGC CTGNTTCAGA TTTNNCTTTN GTNCGTTTTN CNTTCTGTCC TTGGACCAGT 360
CATATCTCAN GTTCCAAAGG CCANAACCCT GAGGAGCGGT NNGGCTAAGN 410

SEQ ID NO:3121
SEQUENCE LENGTH:402
SEQUENCE TYPE:nucleic acid
TOPOLOGY:linear
CLONE:HUMGS03688
SEQUENCE DESCRIPTION:
GATCTGAGGA CCCTGTACTG GTTACTCCTG TACACGTTCA CAATTTTCNT GTAAATNTAT 60
TAATATACTG AGCAGTCCAG GAGCAGCCAT TAGGCAAACA CCACTCCTTG CTTCCTTCAT 120
GTGACCTATC ACTGAGCTCA CCAGCTGAGG TCAATNACAT TCTTCCCAAC CTGGAGAACA 180
GAATTTGGGT CTGCTCAGAG GCATGATGTG AAACATGTCT GGGATAAAAG CTTTGTTCTA 240
TTGACAGCAA ACCTTTTTGT GTTAAGGGGT AGGGGAAGAG CANGTGAAGG ATGCAGATTA 300
ATTTCTATTT AGGANTATAC TTTTNTATAT NGTTTGAAAT TTTGGNCTTG TAAAAATATT 360
CATCCATNTT TTNACCAANA NAATTTCCTT GTGGACNGGT TN 402

SEQ ID NO:3122
SEQUENCE LENGTH:409
SEQUENCE TYPE:nucleic acid
TOPOLOGY:linear
CLONE:HUMGS03689
SEQUENCE DESCRIPTION:
GATCAGTTTG ATTCTGTAAT GAGCACAGCA CCTAATATTT TGAGGAGCTC TGTTTTGAGG 60
ACCAATGCTT AAGGTGGACT TTGTTCGTAA ACAATATCCC AATAGATTTG TTGACTTGAG 120
GTCTGGTTTG GTTTTGTTTT TGTTTTGTTT TGTTTTGTTT TGTTTCCAAT AGAATTAAGA 180
ATTCTAATGT TGAAAAACTG CACAAATTTT TATGGGACAA AGCCTAGAAA AGAGAAATGT 240
AGTTTGAATC ATAATCTAAA TCATCGTATG ATAGANGAGG GAAAGTTTTG GTGCCATAAT 300
TTCTCCTTTC ACTGGTGTTG GGNCTTAAAT CAAGTTGAAA TGTATTTCTG TACCACAATT 360
TTANGGCTTT CAATAAAAAG TTTTAAATNG TCTAGGTGAC CATTCCAAA 409

SEQ ID NO:3123
SEQUENCE LENGTH:400
SEQUENCE TYPE:nucleic acid
TOPOLOGY:linear
CLONE:HUMGS03690
SEQUENCE DESCRIPTION:
GATCAACATC CACAGCGAGA CCTCCGTGCC CGACCATGTC GTCTGGTCCC TGTTCAACAC 60
CCTCTTCTTG AACTGGTGCT GTCTGGGCTT CATAGCATTC GCCTACTCCG TGAAGTCTAG 120
GGACAGGAAG ATGGTTGGCG ACGTGACCGG GGCCCAGGCC TATGCCTCCA CCGCCAAGTG 180
CCTGAACATC TGGGCCCTGA TTCTGGGCAT CCTCATGACC ATTGGATTCA TCCTGTTACT 240
GGTATTCGGC TCTGTGACAG TCTACCATAT TATGTTACAG ATAATACAGG AAAAACGGGG 300
TTACTAGTAG CNGNCCATAG CCTGCAACCT TTTGNACTTC ACTTGTGCAA TGCTTGGCCC 360
TGNACGNTGG GGCTGTTGCC CTTGCCCCNT TTGGTCCTGN 400

SEQ ID NO:3124
SEQUENCE LENGTH:400
SEQUENCE TYPE:nucleic acid
TOPOLOGY:linear
CLONE:HUMGS03691
SEQUENCE DESCRIPTION:
GATCCTTCCT ACTTTGCTTC TNTCCACCCA TGACCTTTTT NACTGTGGCC ATCAGGACTT 60
TCCCTGACAG CTGTGTACTC TTAGGCTAAG AGATGTGACT ACAGCCTGCC CCTGACTGTG 120
TTGTCCCAGG GCTGATGNTG TACAGGTACA GGCTGGAGAT TTTNACATAG GTTAGATTCT 180
CATTCACGGG GNNGGTTAGC TTTAAGCACC CTAGAGGACT AGGGTAATCT GACTTCTCAC 240
TTCCTAAGTT CCCTTCTATA TCCTCAAGGT AGAAATGTCT ATGTTTTCTA CTCCAATTCA 300
TAAATCTATT CATAAGTCTT TTGGTACAAG TTTACCATGN TAAAAAGGAA ATGTGATTTT 360
GGTCTTCCCC NTCTTTTGCA CTTTTTGGAA ATANAGGGTN 400

SEQ ID NO:3125
SEQUENCE LENGTH:397
SEQUENCE TYPE:nucleic acid
TOPOLOGY:linear
CLONE:HUMGS03693
SEQUENCE DESCRIPTION:
GATCCTNAAT TTNAAAATTT NACACCATCA TAAATNAACA AAGGTAGGAA GGAGTTTGGC 60
TTCAATAGCT TACAGTAAAA GGAGTAAAGG AGTTCAGCCT CAAAACGATA TTGTTGACCT 120
GTNATGTTAT GAAAAATAAT CCAGTGTTTA TACTTAACGG ATGCCATCCC TTATNATTTA 180
CTATTTTCTT AGTCTAACTA AACAATCTTT ATTAAAATAA GCAATGATAT TTGACATGGG 240
CCTAAGAAAT ATATAGTTAG CTAACAATAT TGTCAGTTAG AGGGATTTCT TTTCTAAATT 300
AAGTACAGAT TTCTTCACCA AACTCCAGGN TAACAGAAAN GGGGCCATAA AATTTGGNCC 360
ATAGTTTCTG GGTTAAATAA NTGGTAATTT TAAANTN 397

SEQ ID NO:3126
SEQUENCE LENGTH:392
SEQUENCE TYPE:nucleic acid
TOPOLOGY:linear
CLONE:HUMGS03694
SEQUENCE DESCRIPTION:
GATCAAGTAA CATGTTGCAT GTGGCTTACT CTGGATATAT CTAAGCCCNT CTGCACATCT 60
AAACTTAGAT GGAGTTGGTC AAATNAGGGA ACATCTGGNT TATGCCTTTT TNAAAGTAGT 120
TTTNTTTAGG AACTGTCAGC ATGTNGTNGT TGAAGTGTGG AGTTGTAACT NTGCGTGGAC 180
TATGGACAGT CAACAATATG TACTTAAAAG TTGCACTATT GCAAAACGGG TGTATTATCC 240
AGGTACTCGT ACACTATTTT TTNGTACTGC TGGTCCTGTA CCAGAAACAT TTNCTTTNAT 300
NGTNACTNGC TTTTNAAACT TTGTTTAGCC ACTTAAAATC TGCTTATGGC ACAATTNGCC 360
TCAAANTCCC ATTCCCANGT NGGTAATATT GN 392

SEQ ID NO:3127
SEQUENCE LENGTH:391

SEQUENCE TYPE:nucleic acid
TOPOLOGY:linear
CLONE:HUMGS03695
SEQUENCE DESCRIPTION:
GATCTAAGGA TGACTTGGAC ACACTCCCTG GCACTGAAGA GTCTGAACAC TGGCCTGTNA 60
TTGGTCCATT CCAGGACCTT CATTTGCATA AGGTATCAAA CCACATCAGC CTCTGATTGG 120
CCATGGGCCA GACCTGCACT CTGGCCAATN ATTGGTTCAT TCCAGGACAT TCATTTGCAT 180
AAGGNGTCAA ACCACAGCAG TCTTGGATTG GCTGTGAGCC AATTCACCTC AGTNTCTAAT 240
TGGCTGTGAG TCAGTCTTTC ATTTACATAG GGTGTAACCA TCAGGAANCC TCTACAGGGT 300
ACTTAAGCCC CAGGATGATT TTGGCTTACC CGGGNGTTTC TTGNGGCCNC TTTGGTNTTG 360
GCCNAGTTNC NCACCTTTGT GGGAATTGGT N 391

SEQ ID NO:3128
SEQUENCE LENGTH:389
SEQUENCE TYPE:nucleic acid
TOPOLOGY:linear
CLONE:HUMGS03696
SEQUENCE DESCRIPTION:
GATCTCGGCT CACTGCAACC TCCACCTCCC GGGTTCAAGC GATTCTCCTN CCTCAGCCTC 60
CTGAGTATCT GGGATTACAG GCGTGCACCA CCATGCCTGG CTAAGTTTTG TGTTTTTTTT 120
AGTAGAGATG GGTTTTCACC ATATTGGTCA GGCTGGTCTC GAACTCCTGA CCTTGTGATA 180
CACCTGGCCT CAGCCTCCCA AAGGGNNGGT GCCACCGCGC CTGGCCCATT TNTTCTTTTT 240
TTGACCCATA CTTAATGTTG CAGAAACTAT TCTTGTCATA ACATTATCTC TCATGTACAG 300
TAATTATATG TAAATTAATT GAAGCAAATN TGGGAAACTT TACAATAGAA ATAAAGNTAG 360
GCAGCCAGCG TNTGTTTCCA NTTNTNAAA 389

SEQ ID NO:3129
SEQUENCE LENGTH:380
SEQUENCE TYPE:nucleic acid
TOPOLOGY:linear
CLONE:HUMGS03697
SEQUENCE DESCRIPTION:
GATCTCGGCT CACTGCAACC TCCACCTCCC GGGTTCAAGC ATTTCTCCTG TCTCGGCCTC 60
CCGAGTAGCT GGGATTACAG GCGTGAGCAC CATGCCCAGC CAGAAGTACT ATNATTTCTT 120
ATAACTACAT ATGAATCTAA AATTATCACA AAAGGAAACT TCTTTTAAGA AAAAGAAAAG 180
AATCAGAACA TAAATATACA GCATGGCAAA ACTACATCTA AAACAAGTAT TATAGGTCTC 240
ATGGTGGAAA ANTTGGACCC ACAAGAAAAG TTGTTAATTA TCTCTGATAT TCTGAACCAT 300
TGNTATCTGC AAAATGATTA CTAGGTATGT GCAATGANGG CTATTGATGT TACCCTGTAT 360
ATCTGCACAC TGTGTTCCAN 380

SEQ ID NO:3130
SEQUENCE LENGTH:352
SEQUENCE TYPE:nucleic acid
TOPOLOGY:linear
CLONE:HUMGS03698

SEQUENCE DESCRIPTION:
GATCTGCTAG TGCTGTAATA TTGTAAATAC TGGACTCAGG AACTTTTGTT AGGAAAAAAT 60
TGAAAGAACT TAAGTCTCGA ATGTAATTGG AATCTTCACC TCAGAGTGGA GTNNNNNCTG 120
CTATAGCCTA AGCGGCTGTT TACTGCTTTT CATTAGCAGT TGCTCACATG TCTTTGGGTG 180
GGGGNGNGAN GANGAATTGG CCATCTTAAA AAGCGGGTAA AAAACCTGGG TTAGGGTGTG 240
TGTTCACCTT CAAAATGTTC TATTTAACAA CTGGGTCATG TGCATCTGGT GTAGGAAGTT 300
TTTTCTACCA TAAGTGACAC CAATAAATGT TTGTTATTTA CACTAGTCTA AA 352

SEQ ID NO:3131
SEQUENCE LENGTH:375
SEQUENCE TYPE:nucleic acid
TOPOLOGY:linear
CLONE:HUMGS03699
SEQUENCE DESCRIPTION:
GATCGTGGGT TGCCGAGAGG ACCTGAGCGC TGCGGCTCTG CTGAATGGAN CCGGGTCCCT 60
CAGGCCGTGG ACGCCCTCGG AAGGGGGGTG ACTGTGGCTT GTNTCTGGAC AGGAATGTGT 120
CATTTCCCAC ATCTTCTAGA GGGCTGCCAG CTGGGAAGAC AGTTATNAGG GCAAGCTGTG 180
CTCTGAGTTT CGGGTTCTGC TCCTACAAAG AACGTGCGGT GCTGCGGGCN AGGGCCCCGG 240
CACGGACAAG GGCCACTGCA GAGTGTGTTT CTNCTCGTNA GCTGCCCTGG GCANGGAATG 300
GGCTGGNTNA TGCAGTTGGA TGCACATCTN ATTCTGTCAT GAATGTCCAG TAAAAAATCT 360
GAAATTGGTT GCAAA 375

SEQ ID NO:3132
SEQUENCE LENGTH:369
SEQUENCE TYPE:nucleic acid
TOPOLOGY:linear
CLONE:HUMGS03701
SEQUENCE DESCRIPTION:
GATCCAACCC AAGGGCAAGG GCATCCTGGG AAATACCATC AAGTGGAACT TCACCAAGTN 60
CCTCATNGAC AAGAACGGCT GCGTGGTGAA GCGCTACGGA CCCATGGAGG AGCCCCTGGT 120
GATAGAGAAG NACCTGCCCC ACTATTTCTA GCTCCACAAG TGTGTGGNCC CGCCCGAGCC 180
CCTGCCCACG CCCTTGGAGC CTTCCACCGG CACTCATNAC GGCCTGCCTG CAAACCTGCT 240
GGTGGGGCAG ACCCGAAAAT CCAGCGTGCA CCCCGCCGGA GGAAGGTCCC ATGGCCTGCT 300
GGGNTTGGCT CGGCGCNCCC ACCCCTGGCT ACCTTGTGGG AATAAACAGA CAAATTAGCC 360
TGCTGGAAA 369

SEQ ID NO:3133
SEQUENCE LENGTH:367
SEQUENCE TYPE:nucleic acid
TOPOLOGY:linear
CLONE:HUMGS03702
SEQUENCE DESCRIPTION:
GATCTTGGGA AAATTGCGGA GTGTACATTT ACAAAGATGC GTTCAAATAG TGCTCTAAGA 60
GTTTTGTTCA GTGGCTCACT TCGGCTAAAA TGCAGAAATG NATGCTGTCA GCGTTGGTAT 120
TTCACATTCA ATGGAGCTGA ATGTTCAGGA CCTCTTCCCA TTGAAGCTAT AATTTATTTG 180

GACCAAGGAA GCCCTGAAAT GAATTCAACA ATTAATATTC ATCGCACTTC TTCTGTGGAA 240
GGACTTTGTG AAGGAATTGG TGCTGGATTA GTGGATGTTG CTATCTGGGT TGGTACTTGT 300
TCAGATTACC CAAAAGGAGA TGCTTCTACT GGATGGAATT CAGTTTCTCG CATCATTATT 360
TGAAGAN 367

SEQ ID NO:3134
SEQUENCE LENGTH:366
SEQUENCE TYPE:nucleic acid
TOPOLOGY:linear
CLONE:HUMGS03703
SEQUENCE DESCRIPTION:
GATCAAGAAA TGCATTGACA TTCTAATTGA GAAAGAATAT TTGGAGCGAG TGGATGGTGA 60
AAAGGACACC TACAGTTACT TGGCTTAACC CTTCTGGAAG GGTCTGACTG TGTGACCCGC 120
AGCAAATAGT TCATGTTGGA AAGAATGAAA ACAACTCAAG TTCATAGCAG CCAGCCTGCC 180
GCCATTGGAC CTCCCTTTTA AAAACTGAGA CCAAGACTCC CATCAGCTGG TCTCGGGTTT 240
ACATCGGAAC TGCTCAGGAT TGATACATTT NAAGTCTGTA AATACGGACA CCAACGCCAT 300
TTACCCTAAT TTNANGAACA GCGGGGNCTG TCCNTCCGGT GCCGAGGGGC TTGCATTGCT 360
TACCGN 366

SEQ ID NO:3135
SEQUENCE LENGTH:364
SEQUENCE TYPE:nucleic acid
TOPOLOGY:linear
CLONE:HUMGS03704
SEQUENCE DESCRIPTION:
GATCCCTGAT TAAGCTGATG ACTAGACCTA CAATTAATTT TCCTGCAGTA TATGAAGTAT 60
TGTACCAGAG TATTAAAAGA TATGTAATAT TTNATTGATA AATCTATCCT TTAAAAGGAA 120
TACGTTTTAG GATGTCATCA TTTTGATGTG AATCATGTAA ATGTTGATAA TATGCTGTTT 180
ATTATACATT TAGTGTTTCA AGAGATTCAC TTAATTGCCT TTTTGCCCAC GTATATTATG 240
TAGTCTATTT GCAACTGTTC TTAAAAAANT GNCATTAAAG GACTAGTTTA TGTNGGGAAC 300
CATTAGTGGN TGTTATTNGN CTCCCCCCCT NTNTTTTTGG GGTGTTAGCG CACCTGCTTT 360
GCTN 364

SEQ ID NO:3136
SEQUENCE LENGTH:363
SEQUENCE TYPE:nucleic acid
TOPOLOGY:linear
CLONE:HUMGS03705
SEQUENCE DESCRIPTION:
GATCCTGACC AGCAAGCCCC CCCCCAGCCC CCCTTCCAAG TGACTCCGTG NCTTGAGTGT 60
GTCTGCGTGT TTACACCCGT CCCTCTGCTG GCCGCCCCCG TGCGAGCGGC ACCCCTGCCC 120
TGCCCTCCAC AGAATTGGGT TCCAAGGGCT GTTCCAGACA ACTGCCAACG TCACTGAGGG 180
CCCTGCCCCA GCGGNCCTGG CCCCAGGCTC TATTAACCTA AAATGTAGCT CCCTAGCGNT 240
AACCTAGGAA CCGGCGTTGN CTGCTTGGGG GGTCACGCCC TCATGNCCTT GTTCCCAGGG 300
CCGGGGGCCT TNAAGCGGTT NGAACAATTC NTTGGCTTTT TTTCANANTG TTTAATNGGA 360

ATN 363

SEQ ID NO:3137
SEQUENCE LENGTH:359
SEQUENCE TYPE:nucleic acid
TOPOLOGY:linear
CLONE:HUMGS03707
SEQUENCE DESCRIPTION:
GATCTGGTGG TCAGCCCTAG GATAAAAAGC CAGGGCTGGA GAACAAGAAA GGGCCAGGAG 60
ATGGAATTCC TTCAGGCCGG CACCCACACC CTAGGACATG TAAGCCCTCA TGTCCAAGGG 120
AGCCTCATGC AGATAGTAGG AAATCAGGTC TGGAAATTTA AAANTAAAAG GCATGAGACT 180
AAGGCTATCT GCTTCCNTTA TGCCCTGACT GGAGAGGGGA GGGTGGAGAG GCANGGCCCA 240
CAGTGGGCAT CCCNGCTNGG TCTTNGGATG NCTGCNGTGN GGTGAATTCC CGGGAACTNG 300
TTTTGGCACA ANGNTNNTTT TTGGCACTTT GTTTTTTTTG GTATTTAAAG GTTTTGCTN 359

SEQ ID NO:3138
SEQUENCE LENGTH:358
SEQUENCE TYPE:nucleic acid
TOPOLOGY:linear
CLONE:HUMGS03708
SEQUENCE DESCRIPTION:
GATCAGGGGA TAGTGAAGTT TACCAACTGG GTGATGTCAG TCAAAAGACA ACATGGCACA 60
GAATATCAGT ATTCCGGCCA GGCCTCAGAG ACGTGGCATA TCAATATGTG AAAAAGGGGT 120
CTCGAATTTA TTTGGAAGGG AAAATAGACT ATGGTGAATA CATGGATAAA AATAATGTGA 180
GGCGACAAGC AACAACAATC ATAGCTGATA ATATTATATT TCTGAGTGAC CAGACGNAAG 240
AGAAGGAGTA GAAAGGATGA TTCTTCTTTG GCCATCATTT GGTACAGTCT CATTTCCAAG 300
TCATGTATAA TCTTTATGGC TTCCAGGGAC ANGGNTTTAA AATACTCTTT TACGTAAA 358

SEQ ID NO:3139
SEQUENCE LENGTH:356
SEQUENCE TYPE:nucleic acid
TOPOLOGY:linear
CLONE:HUMGS03709
SEQUENCE DESCRIPTION:
GATCTCAGTC TCTCTGTCTC TCTTTCTCTC CTTTTCCTTT TGGTGTATCA AATATTTNAT 60
TGACAAAGTA AGGGCCTTGA TTAGGACCAA ATTCTCGTGT GTTGCTATGG TCTTTATTTA 120
GGACAACAAT TAACAATGCA GTGGCCCATT CTTGTCACTC TACACATATG ACTATACGGG 180
ACATATGTAA TATATAAATA TATATATAAA NCATTCCCCT CTGTCCCCTT GGCTTCGGAT 240
GGAGGCCTTT CTGTTGAGCT GAAATGCACC TGCAGCTGGG TGCTGCCAGC AGCTTGCAGG 300
CCCCAGCCCT GTTCCANTCA ATGCAGTTGN CAATAANTGG NATGAGTATC GTCAAA 356

SEQ ID NO:3140
SEQUENCE LENGTH:354
SEQUENCE TYPE:nucleic acid
TOPOLOGY:linear

CLONE:HUMGS03710
SEQUENCE DESCRIPTION:
GATCTTCAGC ATGTGCGGCC TCATGCTTAA GCTGAAGTGG TGTGCTTGGG TCGCTGTCTA 60
CTGCTCCTTC ATCAGCTTTG CCAACTCTCG GAGCTCGGAG GACACGAAGC AAATAATGAG 120
TAGCTTCATG CTGTCCATCT CTGCCGTGGT GATGTCCTAT CTGCAGAATC CTCAGCCCAT 180
GACGCCCCCA TGGTGATACC AGCCTAGAAG GGTCACATTT TGGACCCTGT CTATCCACTA 240
GGCCTGGGCT TTGGCTGCTA AACCTGCTGC CTTCAGCTGC CATCCTGGAC TTCCCTGAAT 300
NAGGCCGTCT CGGTGCCCCC AGCTGGGATA GAGGGAACCT GGGCCCTTTT NCTN 354

SEQ ID NO:3141
SEQUENCE LENGTH:352
SEQUENCE TYPE:nucleic acid
TOPOLOGY:linear
CLONE:HUMGS03711
SEQUENCE DESCRIPTION:
GATCTTGATG AGGGCCAACT TTCCCTCTTC CACAAGGGAT TAATCTTAGA ACTATAGCCA 60
ATTTATTATA AGTACTATTT AGCTACTCTG TGCTCCACAC ATCACGGAGT AATATCTTCA 120
AACTATGAGG TTTAGTAACA CTTGACTGCT GAGGTTTATT TCCTCAGCTC TGATGCTTCC 180
TGGGTGAAGA TGTAAGTNAT TAAATTAACT CAAATTTTAA AGCTAACACT TCATTCCATC 240
TAGACCAGGT AGCTCGTCAT TTGTACGTCT GATAGGTGCT TCAACATCTC AGAATTGGCT 300
TTATTTTTCA TGCATTGCAT ATTGTACTCN TTTGTAATAT TGGGNATGTA AN 352

SEQ ID NO:3142
SEQUENCE LENGTH:349
SEQUENCE TYPE:nucleic acid
TOPOLOGY:linear
CLONE:HUMGS03712
SEQUENCE DESCRIPTION:
GATCTGACCA CTGTTCTCCA GCAGGCCTCT GCTGCAGCTT TTNCTCTCAG TGTTCTGGCC 60
CTCTCCTCTC TTGAAAGTTC TCTGCTTACT TTGGTTTTCC CTCTGCTTGT AAAACATTGA 120
GTCCCCTCCC TGCCTTGGTT AATTGACTCA CACCAGCTGT GCGATGCCCG CTTTTACAAT 180
TAAAGGAAAA CTGTTTTGTT CAGTGTCACC TTGTCAGCAA CACTGTGTCC CTTCGCCCCA 240
CCGTTCTTCT CTGCTGCATT TGGACATGAG CCAAATTTGA ACCCAATCAA ATATAACGTG 300
TCTGACACTG ATTTTGTNTT TACTCAANTA AANGTATAGA CTACAGAAA 349

SEQ ID NO:3143
SEQUENCE LENGTH:348
SEQUENCE TYPE:nucleic acid
TOPOLOGY:linear
CLONE:HUMGS03713
SEQUENCE DESCRIPTION:
GATCTGTTCC AGCTGCAGGT GAACACCCTA CGACGTTATA AACGACACTA ACAAGTTGCA 60
GACCAGACCA GGCTTCAATA AGGCCCAGTT AGCAGAAACT GTGAGTCGAC ACTTCAGGAA 120
CATACCTGTG AATGAAAAAG AGACCCTTGC CTACTTCATC TACATGGTGA AGAGTAACAA 180
GAGTAGACTG GACCAGAAAT CGGAGGGNGG CAAGCAGCTT NAGTGAGGAT GAAGCACATC 240

TTAAAGGAAT GAAGTGTAAA TGCTTGATGC ACAGGTNGAT ATCTACTACA TTTAAGCCCA 300
TAAAGACTGT TTAAATATTA TTGTAAATAA AAAAGTTTGT ATGATTGN 348

SEQ ID NO:3144
SEQUENCE LENGTH:348
SEQUENCE TYPE:nucleic acid
TOPOLOGY:linear
CLONE:HUMGS03714
SEQUENCE DESCRIPTION:
GATCCAGATG CAGAGGCCAG GATGTGGGCC CAGCCCTGTG CCAGGAGGCT NGCTGGAATA 60
AAGGGATGGG CAGGCTGGCA TGGGGGCAGC CGCTGCCCCT GCCTNNNTGT TGCTGTGTAT 120
TCCTGCCGGC CAGGGGCCAC TGCCAGGACC ACGCCTCCCT TTTNATATCC CGATTCTTAA 180
GTTCTGCTAT TGTGGTATTC TGGTGGAGAA AAAAGAACCG CGTGGCTGTT TTTNAACTGC 240
CTGGAACCTA AGACCCTGAA TTCTTTTCCC CCCCAAGGGG AAAATCTATA TGGAAANCAT 300
TTATTTTAAA ATACAGGATG AAGTGAATTA AAAGATTTTA AATGCAAA 348

SEQ ID NO:3145
SEQUENCE LENGTH:379
SEQUENCE TYPE:nucleic acid
TOPOLOGY:linear
CLONE:HUMGS03715
SEQUENCE DESCRIPTION:
GATCACACCT GAGCTTATGC TAAAAGGTGG GATGACTCAG AGCTGGGGCT GGTCACCAGA 60
CAGACTGACC ATGTGATTAG AGGTTTGAGG CTTTGAGTCA GCCTGACCTA TGGGGCGGGG 120
GACTGGAGGC TGAGTTTAAT CATCTGGCCA GTGCTTTAAT CAGTACATCA TGAAACCCTA 180
ATAAAAACTC TGGACACTAA AGCTCAGCAT AGCTTCCTGG GTGGTGAAGC TGTGGCTATG 240
CCGGCAGGGC AGTGTGCCCT AATTCCACGA AGAGGGGGCA TGGGAGCACT GTGCTCAAGA 300
CCCTTCCAGA CCTTTCCCTG TGTCTTCCTT GGCTTGTGGA GCTGATTTGT ATCCTTTATA 360
ATAAACCTAA TCATAAGGN 379

SEQ ID NO:3146
SEQUENCE LENGTH:371
SEQUENCE TYPE:nucleic acid
TOPOLOGY:linear
CLONE:HUMGS03716
SEQUENCE DESCRIPTION:
GATCTGACCT GTGACAAGTC TGTACTATCC ACTATGTAAA TGGCTCACAA GTCAATTGCA 60
AACAAACTGA ATGTACAAAT CTTAGTGCTG GTGCTGAAAT CTCTTCAGAA TAGTNATCAT 120
GATTTAAAAG TTTGTTTTAA AATTTGCAAG CATCAAGTGT CAATCAAAAC TGAAGATGAA 180
ACACTAATGA ATGTTGAATT CTCATGCTTT TAGGTGTACT TCCTTTTTAG AATTTTCAGT 240
TTTCTGCTAT TTTTACCGTA ACTATTTCGT TTAAANTTGG TTTCATTTAN GTTTCCTACA 300
TGCTAACTTC GTTTGTGCGN TTGANTAAAN TTGCCGGTAT ATATNTGGTG GCCTTGTTTG 360
TGACACTTTG N 371

SEQ ID NO:3147

SEQUENCE LENGTH:346
SEQUENCE TYPE:nucleic acid
TOPOLOGY:linear
CLONE:HUMGS03717
SEQUENCE DESCRIPTION:
GATCACAGGC AAACCCATCA AGCTGACTCA GGTGGAACAC GAAGCTGGCA NNTAAGTGGA 60
ACATGGATGG GGCGGGAACC ACCCCCAGCC CAGGGCTGCA GCCTNNCCCA CCTNACCTTC 120
CCGCTGGACT ATNACCTTAA CCAGCCTTTN ATCTTCGTAC TGAGGGACAC AGACACAGGG 180
GCCCTTCTTT TCATTGGCAA GATTCTGGAC CCCAGGGGCC CCTAATATCC CAGTTTAATA 240
TTCCAATACC CTAGAAGAAA ACCCGAGGGA CAGCAGATTC CACAGGACAC GAAGGCTTGC 300
CCCTGTAAGG TTTCAATGCA TACANTAAAA GAGCTTTATC CCTAAA 346

SEQ ID NO:3148
SEQUENCE LENGTH:344
SEQUENCE TYPE:nucleic acid
TOPOLOGY:linear
CLONE:HUMGS03718
SEQUENCE DESCRIPTION:
GATCTNCTGC CGAGTGAATG GGGAAGTGNT CCAGAGCGGC AACACCAAAT AGATGGTATT 60
CAAGACAGAG GACCTGATAG CCTGGGTCTC CCAGTTTNTN ACCTTTNACC CAGGGGATGT 120
NATCCTAACT GGGACCCCCC CAGGTGTCGG TGTATTCAGG AAACCTCCTG TTTTTNTCAA 180
GAAGGGGGAT GAAGTCCAGT GTGAGATTGA NGAACTAGGT GTNATCATCA ACAAGGTGGT 240
GTGATGGCTC CTGCACAGGC CCTGCACATA GGTTGAGGGC ATCTGCTCCC ACTNAGCCTA 300
GCCCAGGGAA AGGCCCANTG ACAGGNTGTG GACAGGTTGC CAGN 344

SEQ ID NO:3149
SEQUENCE LENGTH:341
SEQUENCE TYPE:nucleic acid
TOPOLOGY:linear
CLONE:HUMGS03719
SEQUENCE DESCRIPTION:
GATCTTTGAA GTCTATTTAA ATATTCATTC CATTACATCT AGACTCACCN AGAACTACAT 60
GTTATGATGT TAAGTTGAAG TTGAAACATG ATGTTTTGCA TTAAATTTAA GATATGCAAA 120
TTTATGTAGA GAAAATAAAT GTTATATACC CTATAATCTT TCACCTAATT AGTATTTAAT 180
TATATGGATT TGNNTTATAT TATAAAAGAT GTTTTGATTT TGTCTNTNGA TATTGNCAAA 240
ATTGTTTGGA TATNCTTATG TTCTCAAGTC TGTATCTGCC TCCCCTGCCT TATNTCTNAT 300
GTTTTGCCAC AGTTAACCCA TTGTGCTTCT TTGTAATCAA N 341

SEQ ID NO:3150
SEQUENCE LENGTH:339
SEQUENCE TYPE:nucleic acid
TOPOLOGY:linear
CLONE:HUMGS03720
SEQUENCE DESCRIPTION:
GATCTNATCA GTCCTAAGCG GGTCATGGGG CAACACGGTT AGCGGGGAGA GCACGGGGTA 60

GCCGGAGAAG GGCCTCTGGA GCAGGTCTGG AGGGGCCATG GGGCAGTCCT GGGTGTGGGG 120
ACACAGTCGG GTTGACCCAG GGCTGTCTCC CTCCANAGCC TCCCTCCGGA CAATAAGTCC 180
CCCCTCTTGT NTCCCACCCT NAGATTGGGC ATGGGGTGCG GTGTGGGGGG CATGTNCTGC 240
CTGTTGTNAT GGGTTTTTTT TGCGGGGGGG NTTGCTTTTT TNTGGGGTCT TTGAGCTCCA 300
AAAAATAAAC ACTTCCTTTN AGGGAGAGCA CACCTTAAA 339

SEQ ID NO:3151
SEQUENCE LENGTH:337
SEQUENCE TYPE:nucleic acid
TOPOLOGY:linear
CLONE:HUMGS03721
SEQUENCE DESCRIPTION:
GATCCTCTCC CTGGGATAAG CACTCCCAGC CCCGTTTATC AGAAACACAG GCAAGGAAAT 60
TGGAACTGCC ACCCAGCCCA GCATGATGGC TCAATTGGTT GGTTNCGTTG TNAGTTGTCT 120
CTTNGTTTTG TTAAGNNTTT TAATAAAGTA CGTTTTGCAT AATTGCCTTT AAATGGTTTG 180
TAATATTTGT AACGGTTTTA GCAGCCTATA ACTTTTCAGC TGGTGCTTTT ACTTAGGGNA 240
AAAAACAATT TGTAAATACA GNACATTTGT TAAAAGGCAT AACCNTAGAA CATAGCTTCC 300
TGTTTTGTGG ATTTGGTTTC CTNTATATTC AAAGTAN 337

SEQ ID NO:3152
SEQUENCE LENGTH:336
SEQUENCE TYPE:nucleic acid
TOPOLOGY:linear
CLONE:HUMGS03722
SEQUENCE DESCRIPTION:
GATCAGAGGC AGACAATGAC GAGTGAAGAT GGTTGTAAAG CCCTCTTCAT TCCTGGAGGA 60
GCCTGCATCT AATCTNTNAG GCCCTCTTTC TCTGTGGGTC TCATGAACAG CAGTGGGGAC 120
CATTGAGNAC TTGAATGGCC TGTTTGTNTA TGGGCTTGCA AAGGACAAGC AGAGTTCACA 180
GAGCTCAGGA TAGAAACATC AGAGCCTCCT CCACGGGCTT CAGTGNAACT CCGATGAACT 240
GTACCTGAGG GAATTTTTTT NTTAATCAAC CCCTTGTGTG GATGAATACA GGACCACAAA 300
ANTTNGTNTA CGTTTGAAAA AAAANAANNT NTNNNN 336

SEQ ID NO:3153
SEQUENCE LENGTH:334
SEQUENCE TYPE:nucleic acid
TOPOLOGY:linear
CLONE:HUMGS03723
SEQUENCE DESCRIPTION:
GATCTGCATT CCGACTGCCT ATNAACGGGT GTGGGGGCCG GGGGCTGGAN TTGCTGAAGT 60
NTTCAACTTG CACTCGGAGC TCCTTTNATA CCTCAAAGCT GGCTGTNAGG TGGCAGCTCA 120
CACCCAGACT CACTGGCCAC ACCTCAGCAG GGGGGGAGTC GAGTGTCAGT NTCTTTCTGT 180
GAAGGCTTTT TTTTTCCTTT GGCCTGGGAA TTTTTCCCAT TTTAATGAAG GGGTTTTAAA 240
TTGTTTCATT TTGTGTGCTG TGCTTCAAAG CCTTAACTGT CAAATCTTGC ATTATCTNGT 300
TTGTACAGAA ATATACTGGC CTAGCAGNGG CAAA 334

SEQ ID NO:3154
SEQUENCE LENGTH:330
SEQUENCE TYPE:nucleic acid
TOPOLOGY:linear
CLONE:HUMGS03724
SEQUENCE DESCRIPTION:
GATCAAAATT TGTGTACTTG CAGGGCTTTC TTTCTTTTTN TTCAAATTTA CAAGGGTTCA 60
TTTTGGAAAC TACATTTTAA ACTTTGGAAT CAAATTGTTT CTTATTTGGG AGGATAATGT 120
ATATACATTG GTATTATGTT AAATAATAAA ATTGTNCTAA TTTGGTGCCA TTTCCTGAAT 180
CACAACTGTA TTTTTGNATC TCANGCTATT TTCATATGTN ATGTGTCAAT GTATCATCTC 240
TCAGAAAGGT TTTACANTCC AAACATTATA TGTNCTCTGT GTAACTGAAT TTNACTTATC 300
TNTTNTAAAC CAGNAACATT AATTGAGAAA 330

SEQ ID NO:3155
SEQUENCE LENGTH:330
SEQUENCE TYPE:nucleic acid
TOPOLOGY:linear
CLONE:HUMGS03725
SEQUENCE DESCRIPTION:
GATCTTCGCA AGCCAGCTTC TTCTGCATCT NAGGGGCTGC TGGCGCCCAN AGGAGGCAGA 60
CAGATGTCTN NTAGCTGAGT TTCTAACCGC ATGATGAGAC TCAGACCTTC CGCTGCACTA 120
GAAAATCTGC AACAGTGTCC CTGAGTCACT TCTCCTTAGT GGGCAGACTC GTGTTAGATT 180
TGTGGAACCC AGCTCTCTGA TTTACTCCTT TTGGAAAACC CATGGAATTT CATGTATAAG 240
GCTTTCATTT GTATTTNAAG GTTTTTCTGT TTGTTTTGAG TATATACATG GTGCTCAATA 300
GCAACATCTT AGCAGATGAA GCAGTTTATN 330

SEQ ID NO:3156
SEQUENCE LENGTH:329
SEQUENCE TYPE:nucleic acid
TOPOLOGY:linear
CLONE:HUMGS03726
SEQUENCE DESCRIPTION:
GATCTACTGC AAATCCTGCT ACGGAAAGAA GTATGGGCCA AAAGGCTACG GTTATNGCCA 60
GGGCGCTGNC ACGCTTAACA TGGACCGTGG CGAGAGGCTG GGCATCAAAC CAGAGAGTGT 120
TCAGCCTNNA CAGGCCTACA ACAAATCCAA ACACTTCTAA ATTTNCTCAG AAATATGGAG 180
GTGCNGNGAA GTGTTCCAGG NGTGGGGATT CTGTATATNC TGCCGAGANG ATAATTGGAG 240
CTGGAAAGCC CTGGCACAAA ANCTGTTTCC GATGTGCAAA GTGTGGGAAG AGTCTTGANT 300
CAACANCTCT GACTGNAAAN AGAAGGTGN 329

SEQ ID NO:3157
SEQUENCE LENGTH:329
SEQUENCE TYPE:nucleic acid
TOPOLOGY:linear
CLONE:HUMGS03727
SEQUENCE DESCRIPTION:

GATCGGGCGC ACGGNCCGCT TTGGCAAGAG GGGCCTGGCA GTGAACATNN TGGACAGCAA 60
GCACAGCATG ANCATCCTGA ACAGAATCCA GGAGCATTTT AATAAGAAGA TAGAAAGATT 120
GGACACAGAT GATTTGGACG AGATTGAGAA ANTAGCCAAC TGAGAAGCTC CACCAGCCAC 180
TGATGCCAGC CCTGGCACTG CCCCTGCACA GGAGACAAGT GCGTTCAGGG CACAGGCCCC 240
GACATCACCC CAAGGNCAAC GGCACAAGTA GAGAGANACT ACCTACCTCA NTTCANATTA 300
TGTTTGGCCT TGACAAAATT GTATGCAAN 329

SEQ ID NO:3158
SEQUENCE LENGTH:327
SEQUENCE TYPE:nucleic acid
TOPOLOGY:linear
CLONE:HUMGS03728
SEQUENCE DESCRIPTION:
GATCAAATTT ACTNGAACAA TTGTTAATTT ATCCATTGTG CTTAGCTTTG TGACACAGCC 60
AAAAGTTACC TATTTAATCT TTTCAATAAA AATTGTTNTT TGAAATCCAG AAATNATTTA 120
AAAAGAGGTC AGGTTTTTAA CTATTTATTG AAGTATGTGG ATGTACAGTA TTTCAATAGA 180
TATGAATATG AATAAATGGT ATGCCTTAAG ATTCTTTGAA TATGTATTTA CTTTAAAGAC 240
TGGAAAAAGC TCTTCCTGTC TTTTAGTAAA NCATCCATAT TTCATAACCT TATGTAAAAT 300
ATGTNGTNCC TGTTTCCAAT AGGTGAN 327

SEQ ID NO:3159
SEQUENCE LENGTH:324
SEQUENCE TYPE:nucleic acid
TOPOLOGY:linear
CLONE:HUMGS03729
SEQUENCE DESCRIPTION:
GATCTGAATT TGGACCATTT CACTAAAGAG AACATGAGTT TGCTCAGCCC TTTCCTCACA 60
AGAGGGAGGG CCCCGGTTCC CCAGACTTCT CCACGCGCTG GCTCCATAAA GGCCAGCTTT 120
GGCCAGGCTG CCACAGGGGC CTNAGGAGCT CACTCTGGGC CTACCTGGTT TCAGTTAGAG 180
GGTCCTCCTG TTATTTTTCC ATTTAAAAAG TATGTCCTCA GAAAACTGTA NTGGAAGGAT 240
GGGTGGCAGG AACTTGTATA GTTCAGCTTC CAACACTTTG GAACAGATTA AAAAGGGAAT 300
CTTTTAAATA AAAACGTATA NAAA 324

SEQ ID NO:3160
SEQUENCE LENGTH:322
SEQUENCE TYPE:nucleic acid
TOPOLOGY:linear
CLONE:HUMGS03730
SEQUENCE DESCRIPTION:
GATCTTGGTT TTAATTAACT TATTACTCTA TTAATATAAC CTTGGAATTC TATTCTAATN 60
ATGTTGTTCT GGCTGCTTGT AGTATCAGTT CGCCCCTCTT GTTAGGGAGA TATGTAACAA 120
TCTGTCATTT AATTGAGCAG TCTTTTTCAC ATAACAGTAA TAGCAAAAAT CCTACCTACT 180
CAGAATCATC TNCTTAGGTT GTCTCCTAAA TCTGAACAAT ACAGTTGTTT TATAAANTNN 240
CATTTNGTCT CTTGAGATTN AATCAATTAG AACTCTTTAG GGAGAAAATA AATANTAGAN 300
GAAANTATAT GCCTCANATT GN 322

SEQ ID NO:3161
SEQUENCE LENGTH:320
SEQUENCE TYPE:nucleic acid
TOPOLOGY:linear
CLONE:HUMGS03731
SEQUENCE DESCRIPTION:
GATCTGACTT TTTTCCTTCT CAAAAGAATC ATACTTGGGA TTACAGGTAC ATTTNATGTT 60
ATATNATGGA TAAGTGAAAA NTTTTTAAAG GAGATTTTAT ACCTTTTCAC ATTAAAAAAG 120
GTATTTATAT TATNACTTTG TAGTGATTGT CTTAAGAAAA AATATAGCCC AAATGTATAG 180
TAAAATCAGC AGCTCAAGAA GAATTTCTGC TTCTNTTTGT AGTTGATGCT TTGTTTTTNC 240
CTGCAGTCAG AAATTCCTTG TATTTGTCAA ATGTATAATC AGCTTGTATT GTTTTTAAAT 300
TAAAANAAAA TTTGANTAAA 320

SEQ ID NO:3162
SEQUENCE LENGTH:320
SEQUENCE TYPE:nucleic acid
TOPOLOGY:linear
CLONE:HUMGS03732
SEQUENCE DESCRIPTION:
GATCTGAAAA NAAACCTTAA TACGCTCATA TGGTTGGAGT GTTAAGTGAA CCTNTGATTT 60
TTTNAGGGTT TTTNTACGTG TAGGCGTGAA TAGGGGGCAC CCCTTCAAAA CTGTACAAAG 120
AAGACGACTG TTTTCCATTT CCATTTAAAC ATTTTTAGCC ACTTCATTTC TATTTATTGA 180
ACAGGTCAAA TTTGTNTTGT TATTTGTGAG TACAGTACAT TTAAAAAACA TCCTTATCGG 240
TTATTTTTTT TTCAGTCGGA GTTTGACGTA TAAATNGTTT ATGCTTTTGG TGTAATCTCT 300
TAATAAACTG GTTCTTCAAA 320

SEQ ID NO:3163
SEQUENCE LENGTH:180
SEQUENCE TYPE:nucleic acid
TOPOLOGY:linear
CLONE:HUMGS03733
SEQUENCE DESCRIPTION:
GATCAGCTAG CTTGATTCTT GTGTACTTTT TTNAAACTGT GGGTTTTCCT AGTAAATTTA 60
ATTTACAGAA ATNAATGGTA GCATTTAGTA ATCTACAAAG GAAATATCAA AGTNTTTTNN 120
AAGCCTGTNA TATTCAGTGT GTGCCACAGG ATTGAAATAA ATGACAATGT AATTATGAAA 180

SEQ ID NO:3164
SEQUENCE LENGTH:303
SEQUENCE TYPE:nucleic acid
TOPOLOGY:linear
CLONE:HUMGS03734
SEQUENCE DESCRIPTION:
GATCAGAATT CCGATTTGAC GGGCAACCAA TCAATGAAAC AGACACACCT GCACAGTTGG 60
AAATGGAGGA TGAAGATACA ATTGATGTGT TCCAACAGCA GACGGGAGGT GTCTACTGAA 120

AAGGGAACCT GCTTCTTTAC TCCAGAACTC TGTTCTTTAA AGACCAAGAT TACATTCTCA 180
ATTAGAAAAC TGCAATTTGG TTCCACCACA TCCTGACTAC TACCGTATAG TTTTCTCTAT 240
TCTTTCATTT CCCCCTTCCC CATTCCTTTA TTGTACATAA AGTAACTGGT ATATGTGCAC 300
AAA 303

SEQ ID NO:3165
SEQUENCE LENGTH:311
SEQUENCE TYPE:nucleic acid
TOPOLOGY:linear
CLONE:HUMGS03736
SEQUENCE DESCRIPTION:
GATCTAGAGC CAGGCTGGTC AAGACAAGAA TTGGCTGGAA TAGGCTGCTC TTCCCCCATT 60
CCATCATGTG CTGTCCCCAC CCCTTTGGCC ACCTGGGCTG ACTGTGTCTT AATACCTCAA 120
GTGCAAGTAT ATAGGAGTAA GAAATNAACA ATGCCTGCCT CCTTATACTC ATGCCTACAT 180
TGTATGACAT CTAGTATGAA AGGGAAACAT TAAAGGAAAA CCCTTGTTTT GCTCTAAAAC 240
CTGAGGACGG TAAACACTGA GAGTAACCTG GTGCTTGGTT TGAAGTAAAA CACAAATACT 300
TCCCTTTTAA A 311

SEQ ID NO:3166
SEQUENCE LENGTH:309
SEQUENCE TYPE:nucleic acid
TOPOLOGY:linear
CLONE:HUMGS03737
SEQUENCE DESCRIPTION:
GATCAAGGGC AATGCCAATG AACATCGGCA TGGATTATAA TTATGCCCTC CTGGAACTCA 60
AAAAGCCCCA CAAGAGAAAA TTTATGAAGA TTGGGGTGAG CCCTCCTGCT AAGCAGCTGC 120
CAGGGGGCAG AATTCACTTC TCTGGTTATN ACAATNNCCN NCCAGGCAAT TTGGTGTATC 180
GCTTCTGTGA CGTCAAAGAC GAGACCTATG ACTTGCTCTA CCAGCAATGC GATGCCCAGC 240
CAGGGGCCAG CGGGTCTGGG GTCTATGTGA GGATGTGGAA GAGACAGCAG CAGAAGTGGG 300
AGCGAAAAN 309

SEQ ID NO:3167
SEQUENCE LENGTH:309
SEQUENCE TYPE:nucleic acid
TOPOLOGY:linear
CLONE:HUMGS03738
SEQUENCE DESCRIPTION:
GATCTTGATA TGTTTTAACA TTATCATGGC AGGGAAATAT ATAAAGAAGA AAAATATTTT 60
NACATTAAAC CTTTTCTAAA ANTTGTAAAT AGAAAAATAA TTTGGTTTTT NATCAAGANC 120
AACACTTATC GTTATGTATT GTGTTAGTTA TATTGCCAGT CTGTTGCGAC TGACTCAAAA 180
AGTTAAATGT TGCCACTGCT GANGATGATT ATGNGCATCG CANACTTTGT TTCTGNCCCA 240
TTTTGGCAGT TTTNATATAC TCCNTTAAGA TGTTGAATGT TACAGGTTAN TAAAGTTAAT 300
ACCTTTAAA 309

SEQ ID NO:3168

SEQUENCE LENGTH:306
SEQUENCE TYPE:nucleic acid
TOPOLOGY:linear
CLONE:HUMGS03739
SEQUENCE DESCRIPTION:
GATCCTTTAC AGAAAAAGTT TTCTGACCTC AATTCTAAAG TAATTGTAGT AGGGAGCTGG 60
AGGACTTTCT TTCCCTTTAT GGTAATTTTT TGAGCTACAA AAGAGCCTTG CAGAAATGGG 120
TGAAGGGATT AANCTTTTAA AAATAAATGC TATATATTAG GAAAATAAAA AATATTTTAG 180
AGCCAAGTTA ACAAGTACTT CAGCAAAACA TGCTAGTTTT ATGCAGGGGA TTCTGTATTC 240
CAAATGGATA CANTCCGACA TATATAAAAG AATCAGATTC TTAACTATTG ACTCTTATTT 300
NGCAAN 306

SEQ ID NO:3169
SEQUENCE LENGTH:306
SEQUENCE TYPE:nucleic acid
TOPOLOGY:linear
CLONE:HUMGS03740
SEQUENCE DESCRIPTION:
GATCATACCC AGAATTCAAT GTTTGATATT TTAAGAATGT ATGTTCTAGT GTTTTTNAGA 60
GTGAGTCTAC CATCTGTATA AAAACACCTT GGGGGCAGGC AGGGGCATTT AAAAATGTAG 120
GACCTATCGT CCAGACTCAC AGAGTGGGGC TCCAGAATCT CCATTTTTAA CAAACTCTCT 180
TAAGTAATTC TGATGTGTAC CAAAATCAGT GCCATTGGTG TGTGTGTACG TAACTATATA 240
CATATGTGTG TGTGTGTATA TATATAATGT GTCATAACCG TAAACAATAA ACAATATCAA 300
GNTAAA 306

SEQ ID NO:3170
SEQUENCE LENGTH:306
SEQUENCE TYPE:nucleic acid
TOPOLOGY:linear
CLONE:HUMGS03741
SEQUENCE DESCRIPTION:
GATCTGTCAT GTTTAAACCC CCTACTTCTA AGGGAACTTC TCTAATCTCT TATCCTCATC 60
CCCAAATAGT GTTTTCTTCC TCTGGGTTCT TATAATNTTG GTATCAATCT CACAGCATTT 120
AGTGCTTCCT GCCTGGTGTG ACAGTTACCT GTGTGCATGT GCAATTTCTA ATTTCCCACG 180
CTAGACTGTG AGCTTCCTAA GGCAAGAATC ATGCCTTGTT GGTTTCTGTA TTCCTCATGG 240
TGCCAAACAC AGTGCCTTCT ACATTGCAGG CGCTGAATAA ACATTTTTAA AGCAAAAAAA 300
TTNAAA 306

SEQ ID NO:3171
SEQUENCE LENGTH:305
SEQUENCE TYPE:nucleic acid
TOPOLOGY:linear
CLONE:HUMGS03742
SEQUENCE DESCRIPTION:
GATCTCCAGT GGTCAAAAGA CATTTACCCT TAATATCAGA CAACATTTAT ATTTTAGTGA 60

AGAAACAAGT TCTCGGGTGG GGAATCTATG TTTCACTCAG ATTTATATGT TTGGAGGAAA 120
AAAGCCTTTT TTTGTAAAAT ATTTAAATTT ATATANGAAA ATGTTAGAAA AAAATATGGG 180
GGAGTGTATT ATAAANCCTG CTTTATTTGC CATGGGGCCN GGGGGAGGCC NGGCCTAATA 240
CTCCTAAAGT ANGTGTTNGG GNCCNTNTTT TCTTCCAATC CACCTGGTGC TGTTCCNTGT 300
AANGN 305

SEQ ID NO:3172
SEQUENCE LENGTH:303
SEQUENCE TYPE:nucleic acid
TOPOLOGY:linear
CLONE:HUMGS03743
SEQUENCE DESCRIPTION:
GATCTCCGGG AGGGACAGAT GGCCAATNTC TCCCCTTCCA AAGCAGGCCC TGCTCCCCGG 60
GCAGCCTCCC GCCGAGGGGC CCAGCCCCCA ACCCACAGGC AGGGAGGGGC ATCCCTCCCT 120
GCGGACCTCA GGGAGCGAAC GTGGATGAAA ACCACAGGGA TTCCGGACGC CAGACCCCAT 180
TTNATACTTC ACTTTTCTCT ACAGTGTTGT TTTGTTGTTG TTGGTTTTNA TTTTTNATAC 240
TTTGGCCATA CCACAGAGCT AGATTGCCCA GGTCTGGGCT GAATAAAACA AGGNTTTTCT 300
AAA 303

SEQ ID NO:3173
SEQUENCE LENGTH:306
SEQUENCE TYPE:nucleic acid
TOPOLOGY:linear
CLONE:HUMGS03744
SEQUENCE DESCRIPTION:
GATCACTTTT GAGCTCTGAA GCTTTGAATC ATTCAGTGGT GGAGATGGCC TTCTGGTAAC 60
TGAATATTAC CTTCTGTAGG AAAAGGTGGA AAATAAGCAT CTAGAAGGTT GTTGTGAATG 120
ACTCTGTGCT GGCAAAAATG CTTGAAACCT CTATATTTCT TTCGTTCATA AGAGGTAAAG 180
GTCAAATTTT TCAACAAAAG TCTTTTAATA ACAAAAGCAT GCAGTTCTCT GTGAAATCTC 240
AAATATTGTT GTAATAGTCT GTTTCAATCT TAAAANGAAT CAGTAAAAGC AAACAAGGGG 300
TTTAAA 306

SEQ ID NO:3174
SEQUENCE LENGTH:301
SEQUENCE TYPE:nucleic acid
TOPOLOGY:linear
CLONE:HUMGS03745
SEQUENCE DESCRIPTION:
GATCAGCAAA CACGATAGAG GAGACCAGTC AGTACTTCTT GGAGGGGGCN GGAGGAGAGA 60
GGAAAAGGGA GGGCGAGAAT NACCACACAA CACAGCCNTT NGACCATGAG CAGAAGCGTC 120
CGTGGGAACT CCACTGGGGT NGATGGGCTG CCTGCACAGC CCCTGGAGAG GGGGCCAGGC 180
ACACCCTCAG AGGAGCTGCA ACCCGTGGCC TGGCCTGCTA CATGCCCTGC TTCCACGTGG 240
NTGCCACGGT GACACANCCA CATTNACCAA TCCCNNCCGG GTCCTGGGGA CGNAGNCACG 300
N 301

SEQ ID NO:3175
SEQUENCE LENGTH:299
SEQUENCE TYPE:nucleic acid
TOPOLOGY:linear
CLONE:HUMGS03746
SEQUENCE DESCRIPTION:
GATCAAAGTT GTCATGAATA CTGACTTGGG CGTGGGACCC ATCCGAGATG TGCTGCACCA 60
CATCTACAGT GCGCTGTATG TGGAGCTGGT GGTGAAGAAT CCCCTGTGCC CGCTGGGCCA 120
AACTGTGCAA AGTGAGCTCT TTCGCTCCCG ACTGGACTCC TATGTTCGCT TTCTGCCCNN 180
NTTCTCCGCC CGGGCTGGCT GAAGCAACCT ACCTCAAGTN TCAGGAGAAT TCATGTCTNC 240
CTGGGGCCCT TCCGAACCTT GTGCCAACTA AGGGCCCCCA NTGTAAGCCC CCGTACCTN 299

SEQ ID NO:3176
SEQUENCE LENGTH:298
SEQUENCE TYPE:nucleic acid
TOPOLOGY:linear
CLONE:HUMGS03747
SEQUENCE DESCRIPTION:
GATCTGAATT ACTACAGAAA AATAATTATG TTGCTTGCTT AATNATTCCC AGGAAACTTC 60
GTTGTAGGCA TATATTTTAT AAGAGTATTA TACAGTGTTA ACTATGCAAG TAAGTTCTAA 120
AATNACACAA TNATCTGTGT AATGTTTTAG TCCAATNACT GTGATTTATT CAACTCTGTT 180
CTAAAGTTAT CTGGAATTGT CATGCTGCCT CAATTTACAG AAATCTATAA TAGATTTCTA 240
TAGAAATGTA TAAAGACGTA GCAGTCATTT TGTTGTTTCT AAATAGATAA CATATAAA 298

SEQ ID NO:3177
SEQUENCE LENGTH:297
SEQUENCE TYPE:nucleic acid
TOPOLOGY:linear
CLONE:HUMGS03748
SEQUENCE DESCRIPTION:
GATCAGAGTA TGGGTTCTCC ATGTAGCAAT ACTTCAGTGA GATTAAGTAT AAACAGTTTT 60
TGGCAAAAAA CAACACAGTC TACTCTTTCT GCTTACAAAG ACAAAGCCTT ACAAACTCAC 120
TATGAAGGTA AAGGGAGGAC AGCTTGCTTC TTTGCCCAGA CATTTACAAA GTTGTTTTTA 180
AANCACACTC ATAAGTAAGT TTGGCAAGTT GTTTAAAAAA TGTCTCTTTG TTTTGTACAG 240
TTCTGTTAGA TGTTGTNATA TTTTAANAGT TTAATTTANA ANNTTTNATT TNGTCCN 297

SEQ ID NO:3178
SEQUENCE LENGTH:297
SEQUENCE TYPE:nucleic acid
TOPOLOGY:linear
CLONE:HUMGS03749
SEQUENCE DESCRIPTION:
GATCTNATTA CCTTCACGGA AGGAAGCGGA CGCTCACCAC GCTATGCCCT CTGGTTCTNT 60
GTGGGGGAGT CATGGCCCCA NNACCAGCCG TGGACCAAGA GGCTCGTNAT GGTCAAGGTT 120
GTGCCCACGT GCCTCAGGGC CTTGGTAGAA ATNGCCCGGG TAGGGGGTGC CTCCTCCCTN 180

GAGAATACTG TNGACCTGCA CATTTCCAAC AGNCACCCAC TCTCNCTTNA CCTCCGACCA 240
GTACAAGNCC TACCTGCAGG ACTTGGTGGA GGGNATGGAT TTCCAGGGNC CTGGGTN 297

SEQ ID NO:3179
SEQUENCE LENGTH:295
SEQUENCE TYPE:nucleic acid
TOPOLOGY:linear
CLONE:HUMGS03750
SEQUENCE DESCRIPTION:
GATCATCACA TTTCGTGAAT TGAGGCCATG TTCAGGTGCT AAGGAAGTAT GCCTTTNACA 60
CAGAAGATTG AGAAAATTTC CTAGATATAA ATACAGATAA ATCAGACGTT ACAGTGGTGA 120
CGTAGTAACC ATCATGGCAA TGGAAAGNAG TCCAATTCAT AGCCTAAAAC TTCAAATGTA 180
TTCTTAGGAG TCAGATTTTA CTGAATATTT TACCCACAAT AGCTGCCTAT TTTGTTATAA 240
TAAAATATAT ATAANTNTAT NTCTNNNCCT TCCTTTAANC TGTAAAANNA NNNAN 295

SEQ ID NO:3180
SEQUENCE LENGTH:295
SEQUENCE TYPE:nucleic acid
TOPOLOGY:linear
CLONE:HUMGS03751
SEQUENCE DESCRIPTION:
GATCCCAGCA CTACTTTTTA TTACTGGAGA AATGGGGGGG ATAGAAAATT CTACTTTGAA 60
TTATTTAGTT TTTTTTAAAG AGTGGGTTGT GTTTGTNCTT CTCCCACCTT TCAGCATTTA 120
TAGAACATGC TGCCCCACAT ACAAAGTCAA GACCACTTAC TTTTATGTGA CACTAGTAGT 180
TTGGGGTTAA TGTTTTGTGT AAGANCAGCT GCATATGAGT AAAGTTACCC CAACCACAGT 240
GAGGAGGAAG ATGTTCACAT ACTGGAACTG TCCTGCCAAA TAAATTTTGC CCCTN 295

SEQ ID NO:3181
SEQUENCE LENGTH:294
SEQUENCE TYPE:nucleic acid
TOPOLOGY:linear
CLONE:HUMGS03752
SEQUENCE DESCRIPTION:
GATCAGTTAA TGCCTAAGAG TGAAAGTAGT TCTATTGACA TTCCTCAAGA TATTTAATAT 60
CAACTGCATT ATGTATTATG TCTGCTTTAA TCATTTAAAA ACGGCAAAGA ATTATATAGA 120
CTATGAGGTA CCTTGCTGTG TAGGAGGATG AANGGGGAGT TGATAGTCTC ATAAAACTAA 180
TTTGGCTTCA NGTTTCATGA ATCTGTAACT AGAATTTNAT TTTCACCCCA ATAATGTTCT 240
ATATAGCCTT TGCTAAAGAG CAACTNATAN NTTANCCCTA TTCTNTCTGT GAAA 294

SEQ ID NO:3182
SEQUENCE LENGTH:293
SEQUENCE TYPE:nucleic acid
TOPOLOGY:linear
CLONE:HUMGS03753
SEQUENCE DESCRIPTION:

GATCCGTGCT GGAAACTCGC AGGGGGACTT TTACATTAGG CAAATCAACA ACGTCAGCGC 60
CATGCTGGTC CTCGCCCGGC CGGTGACGTG CCCCCGGGAG TACGTGCTGG ACCTGGAGAT 120
GGTCACCATG AATTCCCTCA TNAGCTACCG GGCCAGCTCT GTACTGAGGC TCACCGTCTT 180
TGTAGGGGCC TACACCTTCT GAGGAGCAGG AGGGAGCCAC CCTCCNTGCA GCTACCCTAG 240
CTGAGGAGCC TGTTGTGAGG GNCAGAATGA GAANGGCAAT AAAGGGNGAA AGN 293

SEQ ID NO:3183
SEQUENCE LENGTH:293
SEQUENCE TYPE:nucleic acid
TOPOLOGY:linear
CLONE:HUMGS03754
SEQUENCE DESCRIPTION:
GATCTGTGGA CTTTNATTTT TTTAAGAGAC TTACTCAATT TNATGACTGT ACTACCTGAA 60
ACAAAGTGAG AAAGGACAGG TGTATTTTTC TAAGTNATCA AGATAAATCC TTAANAATTC 120
AGTCTAAATN AGCAACCAGG AAGGAAAAAT ATATTAAAAA CAACAAAAAA GTGGCACATG 180
TCCAGGCAGT GTGAGGATTT GCTGTATATA AGTTGCCTGC TTTGTATTNT TNAAATCTCT 240
GCATCACTCA TTGGAAGTGC TTCTNAAGAG AGCTGCTCTG TGTTCAGTTG AAA 293

SEQ ID NO:3184
SEQUENCE LENGTH:395
SEQUENCE TYPE:nucleic acid
TOPOLOGY:linear
CLONE:HUMGS03755
SEQUENCE DESCRIPTION:
GATCAAGATG ACCAAGATGT ATAAAGGGTT CCAAGCCTTA GGGGATGCCG CTGACATCCG 60
GTTCGTNTAC ACCCCCGCCA TGGAGAGTGT CTGCGGATAC TTCCACAGGT CCCACANNCC 120
GCAGCGAGGA GTTTNTCATT GCTGGAAAAC TGCAGGATGG ACTCTTGCAC ATCACTACCT 180
GCAGTTTTNT GGCTCCCTGG AACAGCCTGA GCTTAGCTCA GCGCCGGGGC TTCACCAAGA 240
CCTACACTGT TGGCTGTGAG GAATGCACAG TGTTTCCCTG TTTATCCATT CCCTGCAAAC 300
TGCAGAGTGG CACTCATTGC TTGTGGACGG ACCAGCTCCT CCAAGGCTCT GAAAAGGGNT 360
TCCAGTCCCG TCAACTTGCC TGCCTGCCTN GGGGN 395

SEQ ID NO:3185
SEQUENCE LENGTH:290
SEQUENCE TYPE:nucleic acid
TOPOLOGY:linear
CLONE:HUMGS03756
SEQUENCE DESCRIPTION:
GATCAGCACT TNANTCGGAA AGTTGGAACC TCTGACAAGA CCAAGTACTA CCTTCAAGGT 60
AATCCGTGGC TTCCCACAAA GTTCCACATT TAACTTCCTC CAAGGAAGGG GATTAAAATT 120
TNAAACTAAC TCCCTTCAGT TGAAATCTCG GACCACTNNN NAAAACAAAA ACCATGGTCT 180
AATTNTTTNC TTTGTTATGA ATTTNTTGAC AGTGGATTGA GCGATACGTG TCAGANCTAN 240
GCATTTCACT GTAGGCAAAT TGTGTCTTAG TTATCANGTA AATGTACAAA 290

SEQ ID NO:3186

SEQUENCE LENGTH:288
SEQUENCE TYPE:nucleic acid
TOPOLOGY:linear
CLONE:HUMGS03757
SEQUENCE DESCRIPTION:
GATCCAGATG ACTGAATTTC AGATAGCATT TTTATGATTC CCAACTCATT GAAGGTCTTA 60
TTTATATAAT TTTTTCCAAG CCAAGGAGAC CATTGGCCAT CCAGGAAATT TGGTACAGCT 120
GAAATATAGG CAGGATGTTC AACATCAGTT TACTTGCAGC TGGAAGCATT TGTTTTTGAA 180
GTTGTACATA GTAATAATAT GTCATTGTAC ATGTTGAAAG GTTTCTATGG TACTAAAAGT 240
TTGTTTTATT TTATCAAACA TTAAGCTTTT TTAAGAAAAT AATTGGGN 288

SEQ ID NO:3187
SEQUENCE LENGTH:288
SEQUENCE TYPE:nucleic acid
TOPOLOGY:linear
CLONE:HUMGS03758
SEQUENCE DESCRIPTION:
GATCCCCAAA AAGTGAATAT CAATGGAGGA GCTGTTTCTN TGGGACATCC AATTGGAATG 60
TCTGGAGCCA GGATTNTTGG TCATTTNACT CATGCCTTGA AGCAAGGAGA ATACGGTCTT 120
GCCAGTATTT GCAATGGAGG AGGAGGTGCT TCTNCCATGC TAATTCAGAA GCTGTAGACA 180
ACCTCTGCTA TTTAAGGAGA CAACCCTATG TGACCAGAAG GCCTGCTGTA ATCAGTGTGA 240
CTACTGTGGG TCAGCTTATA TTCAGATAAG CTGGTTTCAT TTTTNATN 288

SEQ ID NO:3188
SEQUENCE LENGTH:285
SEQUENCE TYPE:nucleic acid
TOPOLOGY:linear
CLONE:HUMGS03759
SEQUENCE DESCRIPTION:
GATCAGCAGC TGTGGAAATA AAGCTTGTNA GCCCTCTGCT GGCCACAGTA NAGGAAAGTA 60
GCACAAATAG GATACAGTTG TATGTAGTCA TTGGCAACAA TTGCATACAA TTTTACTACC 120
AAGAGAAGGT ATAGTATGGA AAGTCCAAAT NACTTCCTTG ATTGGATGTT AACAGCTGAC 180
TGGTGTGAGA CTTGAGGTTT CATCTAGTCC TTCAAAACTA TATGGTTGCC TAGATTCTCT 240
CTGGAAACTG ACTTTGTCAA ATAAATAGCA GATTGTAGTG TCAAA 285

SEQ ID NO:3189
SEQUENCE LENGTH:284
SEQUENCE TYPE:nucleic acid
TOPOLOGY:linear
CLONE:HUMGS03760
SEQUENCE DESCRIPTION:
GATCTCACCA TTATGACATC CATCCCCCTA GCTCACCACA TAGCACATAG AATGATATTT 60
TTAATTTGTA AGAGGCCATC CAGGTACTAA GGNCCCAAGG CATACAGATT CACAAAATNA 120
ACTAATCTTT TTGCCTCAGA ATACCAAAAC ANCAAAANTA TAAAGCTGTA TTTGGACANC 180
TANAAAACAC CAAACTATCT CATTGCAATT NGTATTGTAG CAGATGTTCA GCAACTATCC 240

TAATCANTGT TATTGTGTTC CANTTTAACT GGGTTAAATG TTTN 284

SEQ ID NO:3190
SEQUENCE LENGTH:282
SEQUENCE TYPE:nucleic acid
TOPOLOGY:linear
CLONE:HUMGS03761
SEQUENCE DESCRIPTION:
GATCTCGACT CCCCCCTGGC CACAGACCCC CAGGTCATTG TGTTCACTGT ACTCTGTGGG 60
CAAGGATGGG TCCAGAAGAC CCCACTTCAG GCACTAAGAG GGGCTGGACC TNTGCGGCAG 120
GAAGCCAAAG AGACTGGGCC TAGGCCAGGA GTTCCCAAAT NTGAGGGGCG AGAAACAAGA 180
CAAGCTCCTC CCTTGAGAAT TCCCTGTGGA TTTTTAAAAC AGATATTATT TTTNTNATTA 240
TTGTGACAAA ATGTTGNTAA ATGGGATATT AAATAGAATA AA 282

SEQ ID NO:3191
SEQUENCE LENGTH:279
SEQUENCE TYPE:nucleic acid
TOPOLOGY:linear
CLONE:HUMGS03762
SEQUENCE DESCRIPTION:
GATCTGGAGA AGTAAGATGG CCAAATAAAA GCCTCTACCA ATCATCCTCC CCACAGGAAC 60
ACCAAATTTA AGAACTATCT ACACAAAAAA GCACCTTCAT AAGAACCAAA AATCAGAGAG 120
AACAAGGATA AAGAAGTATC CAAATACAAA GAAAATGTTA TGCAAGTGAC CTTTAGAGAT 180
GTTTTAAAGA TGACAAAATA TTGATGANGA TGGGCCAACA AGTGTTACTG TTACCTCTAA 240
TAAAGTTTCA TCACTAGTTT CACCATGGTT AATTGGAAA 279

SEQ ID NO:3192
SEQUENCE LENGTH:277
SEQUENCE TYPE:nucleic acid
TOPOLOGY:linear
CLONE:HUMGS03763
SEQUENCE DESCRIPTION:
GATCTGCTCA AATGCACCAA CACTGCCAAG TGACTAAGGT AGAAAAGAAA AATAACAGGT 60
ATCGTCATCT GAAGGACAGA TGAATCTTTT TCTGCCCCTT CTTCACAATG GAATATAAGG 120
AACAATTATG GGATGTCATC AGAATGGATG CCATAGGACC TACAGCTCCC TTTCTNTTTA 180
TTGTNATTAT ACTTTAAATA TGACATTGTC TTTNATGTGT ATGTTCCTAT ATTTTCAATG 240
TATCTTTTTC CTTCAGTAAA CCTGATATTC AAATAAA 277

SEQ ID NO:3193
SEQUENCE LENGTH:277
SEQUENCE TYPE:nucleic acid
TOPOLOGY:linear
CLONE:HUMGS03764
SEQUENCE DESCRIPTION:
GATCACAGGG AGCCTGTGTT TGTTGGAGGT GTTCCAGAAT CTNTACTGAC ACCACGCTTG 60

GCCCCCAGCA AACCNTTCAC AGGCTGCATA CGCCACTTTG TGATTGATGG ACACCCAGTG 120
AGCTTCAGTA AAGCAGCCCT GNTANGCGGC GCCGTANGCA TCAACTCCTG TCCAGCAGCC 180
TGACATGACA NAGCACAGCT GCCCAANTAC AAAGTTCTTT AGNGCAATGN AAGNAACACA 240
AAAGCCAGNC CAGGAGGCNC CAGTTNCTTA TTCCTTN 277

SEQ ID NO:3194
SEQUENCE LENGTH:272
SEQUENCE TYPE:nucleic acid
TOPOLOGY:linear
CLONE:HUMGS03765
SEQUENCE DESCRIPTION:
GATCACAGAA GCCACTGCAC TCCAGCCTGG GTGACAGAGT GAGACTCTGT CTCAAAAAAA 60
AATTAAATAA ATTATTATAA CCTTTCAGAA ATGCTGTGTG CATTTNNATG TTCTTTTTTT 120
TAGCATTACT GTCACTCTCC CTAATGAAAT GTACTTCAGA GAAGCAGTAT TTTGTTAAAT 180
AAATACATAA CCTCATTCTG ANTAATGTCC CTCATTTTGA CTATAACTGT GCTTGGTTTC 240
AAANGCAAAN TTAANCAAAA ATCTCAGTCA AA 272

SEQ ID NO:3195
SEQUENCE LENGTH:272
SEQUENCE TYPE:nucleic acid
TOPOLOGY:linear
CLONE:HUMGS03766
SEQUENCE DESCRIPTION:
GATCTCCTGC TGGTCAGTGG GGCGGCGGCA GCACTACCGT GGCCTGGTAC CAGGTGAGCC 60
CGCTGGCCGC CCGCCTGCTC TACCCCTACC TGGCCTGGCT GGCCTTCGCG ACCACACTCA 120
ACTACTGCGT ATGGCGGGAC AACCATGGCT GGCATGGGGG ACGGCGGCTG CCAGAGTGAG 180
TGCCCGGCCC ACCAGGGANT GNAGNTGNAC CAGNAGGTGC CATNANGTTT NTAATNTAGT 240
GGCCGTNANG TTTTAATGAC CANTGGGNCT GN 272

SEQ ID NO:3196
SEQUENCE LENGTH:269
SEQUENCE TYPE:nucleic acid
TOPOLOGY:linear
CLONE:HUMGS03767
SEQUENCE DESCRIPTION:
GATCCCATCT NTTTTCCCTT CTTTCCTCCT GGTTCCGGTC AGTTCAGAGG ATTTAACAAT 60
CACAAGTGTC CTGCAAAAAT GCCTGAACAT TATTCTTAGG CCCTCGTGGA TTTTTTTTTT 120
CAGAAAACTT AAACAAAAAA NGACTTACTA NGAAATATGT ACAGCTACCC CTGTTTTCAG 180
GCACTATGTT TGNGANCATT TTNGCCATTG ANGTTCACAC GTGGCATCAG CCCATGCAAG 240
ATAGGTTTCT GTATTNNTAT ATNANNNTN 269

SEQ ID NO:3197
SEQUENCE LENGTH:269
SEQUENCE TYPE:nucleic acid
TOPOLOGY:linear

CLONE:HUMGS03768
SEQUENCE DESCRIPTION:
GATCCAGCGA GGGGTCCAGG CCCTCTGCAT CTCCTCAGGC TCCAACCCTG CTTCCTCAGC 60
AATNAGCTCT CCTCCAGCTT TGGCTTTGGG AAGCCAGACT CCAGGGACTG AAAAGNAGCA 120
ACAAGGAGAG GGTCTGCTTG AGAAATGCCA GATGCTTGGT CCCCAGGAAC TAAGGCGACA 180
GAGTGCAGGG TGGGGGCAAG ACTGGGCTGT AGGGGAGCTG GACTACTTTA GTCTTCCTAA 240
AGGACAAAAT AAACAGTATT TTATGCAAA 269

SEQ ID NO:3198
SEQUENCE LENGTH:267
SEQUENCE TYPE:nucleic acid
TOPOLOGY:linear
CLONE:HUMGS03769
SEQUENCE DESCRIPTION:
GATCCCCTGG AGCCCAGGAG GNAGGTGTGT NAGCTCAATC CGGACTGTAN CGAGTTGGCT 60
GACCACATCG GCTTTNAGNA GGCCTATCGG CGCTTCTACG GCCCGGTCTA GGGTGTCGCT 120
CTGCTGGCCT GGCCGGCAAC CCCAGTTCTG CTCCTNTCCA GGCACCCTTC TTTCCTCTTC 180
CCCTTGCCCT TGCCCTGACC TCCCAGCCCT ATGGATGTGG GGTCCCCATN ATCCCAGCTG 240
CTCCCAAATA AACTCCAGAA GAGGAAA 267

SEQ ID NO:3199
SEQUENCE LENGTH:266
SEQUENCE TYPE:nucleic acid
TOPOLOGY:linear
CLONE:HUMGS03770
SEQUENCE DESCRIPTION:
GATCTGAACC TGTCATTTGA AGTACAGGTT AAAGACTGTG TCCACTTTGG GCATGAAGAG 60
TGTGGAGACT TTTCTTCCCC ATTTTCCCTC CCTCCTTTTT CCTTTCCATG TTACATGAGA 120
GACATCAATN AGGTTCTCTT CTCTTTCTTA GAAATATCTG ATGTTATATA TACATGGTCA 180
ATAAAATAAA ACTGGCCTGA CTTAAGATAA CCATTTTAAA AAATTGGGCT GTCATGTGGG 240
AATAAAAGAA TTCTTTCTTT CCTAAA 266

SEQ ID NO:3200
SEQUENCE LENGTH:265
SEQUENCE TYPE:nucleic acid
TOPOLOGY:linear
CLONE:HUMGS03771
SEQUENCE DESCRIPTION:
GATCTGACTT GGGGAAAGGG TTATCAGAGC CTAGAGGGGC TTAAAAAGTA ATCATTTGAT 60
GTACATACCA CACTCCTTGG CTTCCTTTCT CTTCCCTTAA CCCTTTCTGC TTTTCATTAA 120
CCACATTCCT GCACAACTCA TTTCTGAAAA CCTACCATGT TTCTTTACAG AGCCATCCAA 180
AAATTTTTTG TCCCTACATA GCAATTTTCT GTGGCACTGA GAAACCATGT ATGACCACAA 240
TAAAAATCCA TTTTGTGAAA GGAAA 265

SEQ ID NO:3201

SEQUENCE LENGTH:262
SEQUENCE TYPE:nucleic acid
TOPOLOGY:linear
CLONE:HUMGS03772
SEQUENCE DESCRIPTION:
GATCTCAGCT CACTGCAACC TCTGCCTCCT GGGTTCAAGC GATTATCCTG CCTCAGCCTC 60
CTGAGTAGCT GGGATTACAG GCATGCGCCA CTATGCCCAG ATAATNTTTT TGTATTTTTA 120
GTAGAGACAG GGTTTTACCA TGTTGGCCAG GCTGGTCTGG AACTCCTAAC CTCAGGTTAT 180
CCACCCACCT TTGGGCCTNC CAAAGTGCTG GGATTATAGG CATGGANCCA CCGTGNCTGG 240
NCANAGATGT AAGTNTAAAT TN 262

SEQ ID NO:3202
SEQUENCE LENGTH:260
SEQUENCE TYPE:nucleic acid
TOPOLOGY:linear
CLONE:HUMGS03773
SEQUENCE DESCRIPTION:
GATCCACCTG CCTCGGCCTC CCAAAGTGCT GGGATTACAG GCGTGAGCAC CATGCCCANC 60
CACCTTTTCT ATNTTTAGAT ACATAAACAC TCACCACTGT GTCACAGTCA CCTACAGTAT 120
TCAGTACAGT AGCATATGCT GTACAGGCTT GTAGCCTAGG AGCAATAGGC TATACCATAT 180
AGCCTAGGTG TGTACACCAT CTAGGTTTTT CTAACTACAC TCTATGGNGT TCACACAATA 240
AAATTGCCCA NCGACGCAAA 260

SEQ ID NO:3203
SEQUENCE LENGTH:225
SEQUENCE TYPE:nucleic acid
TOPOLOGY:linear
CLONE:HUMGS03774
SEQUENCE DESCRIPTION:
GATCCCAGCT TATGGCCTTG ACCCAGCCGT CCTCACAGAT GCCGGGTGAC CCTCTAGCTC 60
TCTCTGCATC TCCCACCCCC CGACACCCTG GGACCCTCGA CCCCACCCTT CTTTCCTACC 120
AGCCCAGAGC CTTGTGGCTT GTACAGTTTT GAAACTCCCG TTCTATTTTA TGATGGTTGA 180
TAATAGTCAG TAACNNAATA AAGGAACGTT TGTTAAAATA TCAAA 225

SEQ ID NO:3204
SEQUENCE LENGTH:216
SEQUENCE TYPE:nucleic acid
TOPOLOGY:linear
CLONE:HUMGS03775
SEQUENCE DESCRIPTION:
GATCTGCCCG CGGCGNACCC GTGTTATGTT TTGTGCTGTG TCCACGCGCT AAGACGACCC 60
CCTCCCCCGT ACTGACTTCT CCTATAAGCG CTTCTCTTCG CATAGTCACG TAGCTCCCAC 120
CCCACCCTCT TCCTGTGTCT CACGCAAGTT TTNNACTCTA ATATNNNNAT GGCTTTTTTT 180
CTTCGGCAAA AAAATAATAA AACGTTTCTT CTGAAA 216

SEQ ID NO:3205
SEQUENCE LENGTH:256
SEQUENCE TYPE:nucleic acid
TOPOLOGY:linear
CLONE:HUMGS03776
SEQUENCE DESCRIPTION:
GATCTGACAC TTCCTTTCCA TTGCTTGGCC TGAACAGACT GACCTTGTNA ACTTAAGCCT 60
GGAGTCCATG CCTCGTCTTC CTTTTGTTCA TTGCTGTTAC CAAGAAAGCC AAGGAAGAGC 120
AGCCTGACTC ATTCTTCTTG GCTGCAGCCT CTTCCCCACT TCCTGGGAGT GACCCAGCGT 180
TATTCCTGCC TCCTCACTCC TATTCTCTTT GCCTTTGTGT AAAAATAAAA TGGAAATAAA 240
CAAGTTGCAC AGTAAA 256

SEQ ID NO:3206
SEQUENCE LENGTH:256
SEQUENCE TYPE:nucleic acid
TOPOLOGY:linear
CLONE:HUMGS03777
SEQUENCE DESCRIPTION:
GATCTCTTTT NATCATATAT GACATTAAGG CTACAGAAGT TTTGTGCAAN AATCTTAATT 60
CATATTTATN AATTTCAAGG GAATATTGAA AGGTTTTAAA ATGGCATACA GATAATGGAA 120
AATAAGCTTT ATGTTTCAAG AAGGGCAGAC TCACGAATTT TTTAGAAAAC AGTGCTTCTT 180
AATGATATGC TATTATNCTT ATNAGTTATG TGCAAGCTTT GTATATNCTT ATATNTATAT 240
NTAACTNTAA CCCTCN 256

SEQ ID NO:3207
SEQUENCE LENGTH:253
SEQUENCE TYPE:nucleic acid
TOPOLOGY:linear
CLONE:HUMGS03778
SEQUENCE DESCRIPTION:
GATCCCAATT CGAGAGGTTC TAACCCCAGA ANCGGACATC TCGATTGCAC ACGTTCTGGT 60
TGCCCGAGCC GACCTGTCTT GTCTCGTCCC AGCCACCAGC GTGGCTGTGC GNAGAGGGAC 120
CTGCTGTGCC ATCAACAAGG TGCTTATGGG NAACGTTCCA TACCGGGGGN GCCGCCCAAA 180
TNAGCTGGAG CCTCCCATGC CCACCTGGAG NAGCNGAAGA GAGGATGGAG GCCCCCAGTG 240
NNGGNNTCGC ANN 253

SEQ ID NO:3208
SEQUENCE LENGTH:252
SEQUENCE TYPE:nucleic acid
TOPOLOGY:linear
CLONE:HUMGS03779
SEQUENCE DESCRIPTION:
GATCATGNTT CATGTGCTAA AAATAAACTT GAAACACGGA AGTAGTGGTT GGTCCAGTTT 60
GAAAGCTCTT ATNAGTATTC TTCATCCTGG CTGTAATAAT AGCCATNATT TGTNATGCCT 120
TTGTTATGTA GCAGACACTC TTAAGNATTT NATGTGTATN ATTCAAATTG CTATNACTGT 180

TCTTTTTATA GTTGAGAATC TCAGGATACC TACATTTATC ACTTTTTCAA TATATATGTA 240
TTTCTTATTA AA 252

SEQ ID NO:3209
SEQUENCE LENGTH:251
SEQUENCE TYPE:nucleic acid
TOPOLOGY:linear
CLONE:HUMGS03780
SEQUENCE DESCRIPTION:
GATCAAGCTG TCCGAACCTA CCAAGAGCAC AAAGCAAGCA TGCATCCCGT GACTGCAATG 60
CTGGTGGGGA AAGACTTAAA AGTGGATTAA AGACCTGCGT ATTGATGATT TTAGAGATTT 120
CTCTTTTTTA AATGGAATTC GTAAAGAAAT ATAAAACTTC AGCTCACAAT TAAAACTGTC 180
TTTTTAGTTT TGGCTTTTTA TTGTTTTGTT GGTGATTTTA CTGAAATAAA GTTGAGCTAC 240
TTCTTCTTAA A 251

SEQ ID NO:3210
SEQUENCE LENGTH:250
SEQUENCE TYPE:nucleic acid
TOPOLOGY:linear
CLONE:HUMGS03781
SEQUENCE DESCRIPTION:
GATCTTGTGT TCAGATGAAA GTGCAAGTTT TTATTAATTT GGATTGCCTG AACAGTGTAT 60
CCCATGATGA TGAAGGAAAA TGGAGAGATT TTTCTTTTTA ACTCTGNNNN NTCAGAGATG 120
AAGCCACGCC TTTCCATTTT TCAATGCTGC ATATTTAATC TGCAACAAAA ATGTTAAGCC 180
ATAACAGCCT TTTTATATTT TAGTTTGTCA CCTTTGCATT GCAGAATAAA TACTGGNTAA 240
CCATTTTAAA 250

SEQ ID NO:3211
SEQUENCE LENGTH:251
SEQUENCE TYPE:nucleic acid
TOPOLOGY:linear
CLONE:HUMGS03782
SEQUENCE DESCRIPTION:
GATCTTACTT GCTCCTGCTT ACGAAGATTN CCCAATNACT GGTCATCTGA CCCTACTTGA 60
ACACTCCTGA ACAGTCATGT TTTTNAAAAT NTTCCTTTAT ATCAAGTCAG AGAGTATACT 120
TCTATAAATT TNACTCATGG ATGTTAGGAA ATCTAGTCAT CTTCCCTGTG ATTGCCCTGT 180
TAAGTATTTA ACCATAGCTA TCATGTGTTT CCCAAATCTT CTCTAGATTA AATATCTTCA 240
GTTACTTCAA A 251

SEQ ID NO:3212
SEQUENCE LENGTH:250
SEQUENCE TYPE:nucleic acid
TOPOLOGY:linear
CLONE:HUMGS03783
SEQUENCE DESCRIPTION:

GATCTAGACT GAGAATCAGC CTGAGCTTTA CACAGCTGGG GTCTGTTACT CGCGTTTTGT 60
AGACTTTTGT GTAACTATTT GTACCGTAGA CAGAATGTGA GGAGGAAGTA ACACACAGAG 120
GAGGATGTGT GTGTATGCAT GTGTTTGAAT TCACAAGGAA GAAATTATTT ATCTTGAGCT 180
TTTTCCTTTG TTATTCAATT TCTATTGATT TATTAGTAAT AACAATGATA ATAAAATGTA 240
AATGAGCAAA 250

SEQ ID NO:3213
SEQUENCE LENGTH:250
SEQUENCE TYPE:nucleic acid
TOPOLOGY:linear
CLONE:HUMGS03784
SEQUENCE DESCRIPTION:
GATCTAACGT GTTCCAGTGT CTGTCTGAGG TGTCTTAAAA AATCAGAACA AAACTTCTAT 60
TATCCAGAGT CATGGGAGAG TACACNCTTT CCAGGAATAA TGTTTTGGGA AACACTGAAA 120
TNAAATCTTC CCAGTATTAT AAATTGTGTA TTTAAAAAAA AGAAACTTTT CTGAATGCCT 180
ACCTGGCGGT GTATACCAGG CAGTGTGCCA GTTTAAAAAG ATGAAAAAGA ATAAAAACTT 240
TTGAGGNAAA 250

SEQ ID NO:3214
SEQUENCE LENGTH:250
SEQUENCE TYPE:nucleic acid
TOPOLOGY:linear
CLONE:HUMGS03785
SEQUENCE DESCRIPTION:
GATCATAGGT CTGGACACTC CATCCTTGCC AAACCTCTAC CCAAAAGTGG CCTTAAGCAC 60
CGGAATGCCA ATNAACTAGA GACCCTCCAG CCCCCAAGGG GAGGATTTGG GCAGAACCTG 120
AGGTTTTGCC ATCCACAATC CCTCCTACAG GGCCTGGCTC ACAAAAAGAG TGCAACAAAT 180
GCTTCTATTC CATAGCTACG GCATTGCTCA GTAAGTTGAG GTCAAAAATA AAGGAATCAT 240
ACATCTCAAA 250

SEQ ID NO:3215
SEQUENCE LENGTH:250
SEQUENCE TYPE:nucleic acid
TOPOLOGY:linear
CLONE:HUMGS03786
SEQUENCE DESCRIPTION:
GATCAAACTG CCATTCACAG TGTAAGGCAG CACTTAAATT TCGAACCTAA AGTTTAGATG 60
CATTGTATAA AAAAACCTAA AAGCAGTATC TGTTNNTNNN GCTGTAAACC AAGTTGGAAG 120
CTATTCGGAT AATTTCTTAA ATATTGATGA NCTTTGGAGT ACTGTTTCTN CCTTCAAACT 180
GAATGTAATT AATNCATGAT TAAATGCACC TNATATGTTT AANCAATCTT TGTATACTTN 240
TGGGNTTTTN 250

SEQ ID NO:3216
SEQUENCE LENGTH:252
SEQUENCE TYPE:nucleic acid

TOPOLOGY:linear
CLONE:HUMGS03787
SEQUENCE DESCRIPTION:
GATCGCCACA CTGCCACTCT GCTGCCCAGC CACCAGGATT TCTGCCTGGA GCCCTTTGCC 60
CTGTCCCTGC TGCCTGAGGT GGCGTAGAGC AGAGGAGAAG AATTTGCTCC AGGCCAGGAG 120
CACAAATCGT TGTTCAATGA ATTTNTCTCC AACTCGACCT TGGTAAACGG AAATNTTGGG 180
GGTGAAGAGA AACAATCACT ATTTTTTNCT TTTTTATCTG GTAAATAATT AAAAGAAAAA 240
CCGAACACTA AA 252

SEQ ID NO:3217
SEQUENCE LENGTH:336
SEQUENCE TYPE:nucleic acid
TOPOLOGY:linear
CLONE:HUMGS03788
SEQUENCE DESCRIPTION:
GATCTGTATT GGATAAGGAC CAATGNACAG CAAAGCAAAA ATGGCTTTAA AGCTTGGTGT 60
TACTTTTNTT AAGTTGTTTA ATTATAGTTA AGCAATTTCA AAAATGCTCC AAAGAAATGT 120
GAAAGGACCT TTTGTCACAG CACTTCAGAA AATACACAAC AGCCCCTTCT GCCCCCGCAC 180
AGAAATGCTG CAGAGTATAT AAAACTTGAG ACATTTTTGT AGGATGCCTG ACGAGGTGTN 240
GCCTTTTATC TTGTTTCCGG ATGCATATTT ATTACGAGTA CTCTGGTTAA ATATTGAAAA 300
GTTATATGCT GTAGTTTTTA GTATTTTGTC TTTGTN 336

SEQ ID NO:3218
SEQUENCE LENGTH:227
SEQUENCE TYPE:nucleic acid
TOPOLOGY:linear
CLONE:HUMGS03789
SEQUENCE DESCRIPTION:
GATCTCGAGT GTTATTTTCT GATTGTGGTG TTGAGAGTTG CACTCCCAGA AACCTTTTGA 60
GAGATGCATT TATAGCCCTA GGGGTGGTAT GACCCAAAGG TTCCTCTGTG ACAAGGTTGG 120
CCTTGGGAAT AGTTGGCTGC CAATNTCCCT GCTCTTGGTT CTCCTCTAGA TTGANGTTTG 180
TTTTCTGATG CTGTTCTTAC CAGNTTGGGA AAAAAGTGTG GATTAAA 227

SEQ ID NO:3219
SEQUENCE LENGTH:248
SEQUENCE TYPE:nucleic acid
TOPOLOGY:linear
CLONE:HUMGS03790
SEQUENCE DESCRIPTION:
GATCTGCATA GAAAATTTTN ATACAATTTT TTGAAAGTCC TTAGGTGAAA CATTTACCCA 60
TTAAAAAGGA AGCAGAAATA CTGAGACATG AAAGGCATTA TCAACTAACT CTAGACTCTA 120
GAACCCATTC TNGCATATCT CACGTGCAAT TTTTAAAAAT AAGTTAATAA TTCANCTCAT 180
ATCAACAAAA GCCTTTGNNA CATGGGTTTN CACTNGATAT CACCTAGTGC TNANGNTAGA 240
NGCCAANN 248

SEQ ID NO:3220
SEQUENCE LENGTH:245
SEQUENCE TYPE:nucleic acid
TOPOLOGY:linear
CLONE:HUMGS03791
SEQUENCE DESCRIPTION:
GATCCAGTGC TCTCCCATCT AACAACTAAA CAGGAGCCAT TTCAAGGCGG GAGATATTTT 60
AAACACCCAA AATNTTGGGT CTNATTTNNA AACTTTTAAA CTCACTACTG ATGATTCTNA 120
CGCTAGGCGA ATTTTTCCAA ACACATAGTG TGTGTNTTTT GTATACACTG TATGACCCCA 180
CCCCAAATCT TTGTATTGTC CACATTCTCC AACAATAAAG CACAGNGTGG ATTTAATTAN 240
GCAAA 245

SEQ ID NO:3221
SEQUENCE LENGTH:244
SEQUENCE TYPE:nucleic acid
TOPOLOGY:linear
CLONE:HUMGS03792
SEQUENCE DESCRIPTION:
GATCCACCCG CGTTGGCCTC CCAACGTGCT GGGATTATAG GCGTGAGCAC CACGCCCCGC 60
CTAAAATCCA TATTCAAAGA AGCAATTTCA GTTCCTTTCT AAGCTTTGTC AGTCAAGGGG 120
CTCCACTGAC TTCCTAGGCC CTGTAATTTA ACCAGTCTTT AAGGTTTTGC AGGAAAGTCC 180
CTTCTTCCAA GTGGTTTTTC CAAATCGCAC AATGGCAAAG CCAAACAGAG GAAGAAACAT 240
TAAA 244

SEQ ID NO:3222
SEQUENCE LENGTH:244
SEQUENCE TYPE:nucleic acid
TOPOLOGY:linear
CLONE:HUMGS03793
SEQUENCE DESCRIPTION:
GATCAGATTG CTGAAACACT ATGGGAACAG GTATTGAAGC CCCTGGGTGA TAATNTAATG 60
GAGGAAAACA TAAGGCAGTC TGTAACAAAC TCTATCAAAG CAGGCCTGAC TGACCAGGTG 120
TCTCATCATG CCAGATTAAA GACAGACTGA CAGTTCATCT CCTCACGGAC TCCACTCTCT 180
TTTTTTTTNA TGCTTGCTGT ACAATGAGAA CTCAAATAAA ANTAAACCAA AGTTTACAAT 240
CAAA 244

SEQ ID NO:3223
SEQUENCE LENGTH:246
SEQUENCE TYPE:nucleic acid
TOPOLOGY:linear
CLONE:HUMGS03794
SEQUENCE DESCRIPTION:
GATCCAGGAG GAGCAGCACC CGAGCCCTGG AGTGGCCCAG TACCCTTCCA AGAGGCCACA 60
GTCCCAGCCA GGACAAAGTA TGCGGCCCAT CCTGGTGCGN CAGGTGGGAC AATGTGAACA 120
TGGACTCGAA GACATGGCCC TTTCTCTGTA GTTGATTTTT TAAATGTGCC ATTATTGTTT 180

TTAAAAAANN CGGNAAAANG AAANGCANAC AAATAAAACA CCTTTAAGAG GCTTGAANGC 240
GNCAAA 246

SEQ ID NO:3224
SEQUENCE LENGTH:243
SEQUENCE TYPE:nucleic acid
TOPOLOGY:linear
CLONE:HUMGS03795
SEQUENCE DESCRIPTION:
GATCATAACT CATGAATATG TTTGTAATCA TGGTAATTTA GATTTTNATG AGGAATGAGT 60
ATCTGGAAAT ATTGTAGCAA TACTTGGTTT AAAATTTTGG ACCTGAGACA CTGTGGCTGT 120
CTAATGTAAT CCTTTAAAAA TTCTCTGCAT TGTCAGTAAA TGTAGTATAT TATTGTACAG 180
CTACTCATAA TTTTTTAAAG TTTATGAAGT TATATTTATC AAATAAAAAC TTTCCNATAT 240
AAA 243

SEQ ID NO:3225
SEQUENCE LENGTH:241
SEQUENCE TYPE:nucleic acid
TOPOLOGY:linear
CLONE:HUMGS03796
SEQUENCE DESCRIPTION:
GATCTGACCT TTTTTTTCCA GTTGAAATGT ATTAACACAC CTTCCACAAT NATTTTATAA 60
GAGTCAGCAT AACATAGTGG ATAAGAACTG TGAGATGTTT AACCTCTCAG TAACTCGGTT 120
CTCTCATTAT AAAATAGGAA TAAAATCAGT ACCTGTTTCA TATGAAGGTC GTTTCTGAGA 180
ATTAAATGGA CTAATGTATG CAAAAAGCCT GGCAAACAAT AAACACTCAT CTGACTTTAA 240
A 241

SEQ ID NO:3226
SEQUENCE LENGTH:240
SEQUENCE TYPE:nucleic acid
TOPOLOGY:linear
CLONE:HUMGS03797
SEQUENCE DESCRIPTION:
GATCAAAATA TATTTGTCCA GAAACTCTTG GGCCAGTTCT CTGAGAAGGA ACTGGCTGCT 60
GAAAAGAAAC GCATCCTGCA CTGCCTGGGG CTTGCAGAAG AAATCCAGAA ATATTGCTGT 120
TCAAGGAAGT AAGAGGAGGA GGTGATGTAG CACTTCCAAG ATGGCACCAG CATTTGGTTC 180
TTCTCAAGAG TTGACCATTA TCTCTATTCT TAAAATTAAA CATGTTGGGG AAACAAGAAA 240

SEQ ID NO:3227
SEQUENCE LENGTH:239
SEQUENCE TYPE:nucleic acid
TOPOLOGY:linear
CLONE:HUMGS03798
SEQUENCE DESCRIPTION:
GATCCTCCCA CCTNCAGCCT CCCAAGTAGC TGGGACTACA GGCACATGCT ACCATGCCCA 60

GCTAATTAAA AAAAATTTTT TTTTTGTAGA GACGGAGTTT CACTGTGTTG CTCAGGCTGG 120
TCTTGAACTC CTGAGTTCAN GCAACCCTCC ACTTCAGCTT CCCAAAGTGC TGGGATTACA 180
GGCATCACTG GCCCAGGCAG AGCCAATTNT GTATAATAAA TCTGTNTCTC TCTGTCAAA 239

SEQ ID NO:3228
SEQUENCE LENGTH:238
SEQUENCE TYPE:nucleic acid
TOPOLOGY:linear
CLONE:HUMGS03799
SEQUENCE DESCRIPTION:
GATCAAATTT NATTGAATGG TTTGAGGTTG TAGCTCTATA AATAGTAGTT TTTAACATGC 60
CTGTAGTATT GCTAACTGCA AAAACATACT CTTTGTACAA GAGGTGCTTC TAAGANTTTN 120
ATTGACATTA ATGACACTGT ATACAATAAA NGTGTAGTTN CTNAATCGCA CTACCTATGC 180
AACACTGTGT ATNAGGTTTA TCATCCTCAT GTATTNTNNT GTGACCTGTA TGTNTATN 238

SEQ ID NO:3229
SEQUENCE LENGTH:237
SEQUENCE TYPE:nucleic acid
TOPOLOGY:linear
CLONE:HUMGS03800
SEQUENCE DESCRIPTION:
GATCCAAAGC CATCCCAGAC CTTCCCGGGC CCCTCTTCCT GGCAGATGAC TCTCCCCGCT 60
TCTGCGTCCT GCCTGCCCAT TCATACCAGT TTGAACTGCA ACAGCCTAGC TCCTCACCTG 120
TGCACCTCCT TCCCCAGGTG TGCACGTCTC CAGCCCCGAG CGCGTGTCTT CCTGCTCATC 180
TGGGTTTCCT GGGCAAATAG TGGCCCTCAA TAAATNTTTG CTGAGTGAGA ACATAAA 237

SEQ ID NO:3230
SEQUENCE LENGTH:515
SEQUENCE TYPE:nucleic acid
TOPOLOGY:linear
CLONE:HUMGS03801
SEQUENCE DESCRIPTION:
GATCGCCAGA GCCCAGGAGT TTGAGGCTGC AGTGAGCTAT GAGGGTGCCT CTTTGCTCCA 60
TCCTAGGCAA CAGAGTGAGA CGCTGTTTAA AAAGGAAAAA ATCCTTCCNT AGAGCTAGTA 120
TCCTAAAGCT GCAGAGCTAG CCCATGACCT CATTGGTTTC NTTGTCCTTG GGGTGCTTTT 180
CCTGAATCTT TGGGGGTGAA GGGAGTNTTG CTCCCAGTCC AGAGGCCTGA TTCTTTTCGG 240
ACTGGGTTCT CAAGACACGA CCAGGTTCTC AAGACACGAG TCCCCTTNTT CCTNCCCATT 300
AAAGGGGGTT TNTNAGAAGC AAGANCAGCC CCTTTCCCCA AGTCACAGCC TGAAGGGAGG 360
CCCCNGAGAN GCTTCCCTCC TTTCCCCNCA CCCTGNTTCC TTAACCTTNT TTTGNCCCTG 420
NTTTTTTTAG GAANCTGGNA GTTTCAATTN GTTTTTAANG GGGGATTGGG GGGGAAGGGA 480
AGNCTTNGGG GGACAAAAAA NNTTTTTNTT NCAAN 515

SEQ ID NO:3231
SEQUENCE LENGTH:227
SEQUENCE TYPE:nucleic acid

TOPOLOGY:linear
CLONE:HUMGS03802
SEQUENCE DESCRIPTION:
GATCAGTGTA TGCGAAAAGG TTTTTAGGAA GTATGGCAAA AATGTTGTAT TGGCTATGAT 60
GGTGACATGA TATAGTCAGC TGCCTTTTAA GAGGTCTTAT CTGTTCAGTG TTAAGTGATT 120
TAAAAAAATA ATAACCTGTT TTCTGACTAG TTTAAAGATG GATTTGAAAA TGGTTTTGAA 180
TGCAATTAGG TTATGCTATT TGGACAATAA ACTCACCTTG ACCTAAA 227

SEQ ID NO:3232
SEQUENCE LENGTH:224
SEQUENCE TYPE:nucleic acid
TOPOLOGY:linear
CLONE:HUMGS03803
SEQUENCE DESCRIPTION:
GATCACTAGC TAATNCTCAT ATCCATGCCT TTTTGTCCTG TTTACAGTCT TAAAAGAGGT 60
AAAACAGCAA ATATTTTTTN AAGGGAACTA TAACCTTAGG ATTCCTGAAA ANAATTTCAA 120
AAAAAAAATA AAGACACTGT TGCCAATGTG NCCCAAAACT TAAGACCATA CTAATGGCAT 180
ATATTTGTNA CAATNAAAAA TTAANTTTAC TACTTAATCC TAAA 224

SEQ ID NO:3233
SEQUENCE LENGTH:310
SEQUENCE TYPE:nucleic acid
TOPOLOGY:linear
CLONE:HUMGS03804
SEQUENCE DESCRIPTION:
GATCGGTGCT TTGTGTAAAT ACATCTTTAA CATTTGGGTG GAGAGGGGCC TTAAGAAGGA 60
CAGTTCATTG TAGGAAAGCA ATTCTGTACA TGAGTTTAAG CATTCTTGTT GCATTGTCTC 120
TGCAGATTCT ATTTTTGTTT ACAATATTAA AATGTATGTN AGCAAAATGG GTGGATTTTC 180
AAATAAAATG CAGCTTCCAC AAAAGTTTTG TNATGGTATT CTGGTCTGAG ATGCATTTNC 240
ATTTTNCCTN TCTCTTNTTA TTATCAATAT TGTNATTTTT CCCTAATAAA ATATACCCAG 300
GTGNATTAAA 310

SEQ ID NO:3234
SEQUENCE LENGTH:220
SEQUENCE TYPE:nucleic acid
TOPOLOGY:linear
CLONE:HUMGS03805
SEQUENCE DESCRIPTION:
GATCAAATTT TTNAGACAAT CCTTGGTCTG GATTGGTTAA TGAAAATNTN TTGTTTAAGA 60
GGTTCTATTA AAAAATATTT CTAGTGTATT AGTCATAATT NTTTTTGTGG AGTGGGGGTA 120
AACCACACCA TTAAGGGAAT AAAATGTAAT TTGAAAGAGG TGATTATAAG ATTTGTTTCA 180
GGCCCCAAAA TATAAATAAA AATTGGTGTG GAATCTCAAA 220

SEQ ID NO:3235
SEQUENCE LENGTH:219

SEQUENCE TYPE:nucleic acid
TOPOLOGY:linear
CLONE:HUMGS03806
SEQUENCE DESCRIPTION:
GATCGATACA GAGCATCTGC ACTGGGATTG TCCGCCCCGC CTGTCAGGGA GGCCTCGGGG 60
GCTAATNGAG CTGAGGGTAT AGTCGGCCTT GGACCCTCGC TTTTTTCCTT TCCTGTACTA 120
TNAAGATAAC TGCCTTATCT CCCCCTCAGG ACTGTTTTGG GGATTAAATA GGACGTTTAA 180
ATNACCAACG CTCTTTGAAA TAAACATTTC AAAATTAAA 219

SEQ ID NO:3236
SEQUENCE LENGTH:219
SEQUENCE TYPE:nucleic acid
TOPOLOGY:linear
CLONE:HUMGS03807
SEQUENCE DESCRIPTION:
GATCAAAAAA ACTTTTCTTC TTTTGCAAAG AAAGCTTTTT GTTTTTAACN GCAACGTACT 60
TTTCCCCTAC CTTGAAGAGA CATGGTGGTC GCAGCTTCTN ATCTATATGA AAAAGTTTTC 120
GATGTATTGG AATNATTTGG GAATGCTTTT AAAACAATTT GTAATTATTT CTTTACAAAC 180
CAAAACAGAA CAGAAAGGTG TGGTGCTGGA ACATCGAAA 219

SEQ ID NO:3237
SEQUENCE LENGTH:265
SEQUENCE TYPE:nucleic acid
TOPOLOGY:linear
CLONE:HUMGS03808
SEQUENCE DESCRIPTION:
GATCTGCATT TTCAGAAGAG GACAATCAAT TGAAACTAAG TAGGGGTTTC TTCTTTTGGC 60
AAGACTTGTA CTCTCTCACC TGGCCTGTTT CATTTATTTG TATTATCTGC CTGGTCCCTG 120
AGGCGTCTGG GTCTCTCCTC TCCCTTGCAG GTTTGGGTTT GAAGCTGAGG AACTACAAAG 180
TTGATGATTT CTTTTTTATC TTTATGCCTG CAATTTTACC TAGCTACCAC TAGGTGGATA 240
GTAAATTTAT ACTTATGTTT CCAAA 265

SEQ ID NO:3238
SEQUENCE LENGTH:217
SEQUENCE TYPE:nucleic acid
TOPOLOGY:linear
CLONE:HUMGS03809
SEQUENCE DESCRIPTION:
GATCTACTAT AAGGTATTCT ATATTTATAT NACTTCAGAG ACGCGTATGT AATAAAGNAC 60
GCCCTCCCTC CAGTGTCCAC ATCCAGTTCA CCCCAGAGGG TCGGGCAGGT TGACATATTT 120
ATTTTTGTCT ATTCTGTAGG CTTCCATGTC CAGAATCCTG CTTAAGGTTT TAGGGTACCT 180
TCAGTACTTT TTGCAATAAA AGTATTTCCT ATCCAAA 217

SEQ ID NO:3239
SEQUENCE LENGTH:217

SEQUENCE TYPE:nucleic acid
TOPOLOGY:linear
CLONE:HUMGS03810
SEQUENCE DESCRIPTION:
GATCGGTGCT ACCAAGGAAG AGAGCACACA GATAAGACAG AGGGGAGGAG GTGGGCATTT 60
CCTACATTCC TCCTTGTTTN CCGCTGCTGA NATTGCAGTA TTTATTGCAA TNTAAATNTA 120
TCCTGAAGGT GGGGAGGAAT GTTTAANCTA CCATNTCCGT GTGTCATCTT GGTNTGTTTT 180
TNTCCCTGTT TGTAGCAAGA CTCTNATGAT AATNCTN 217

SEQ ID NO:3240
SEQUENCE LENGTH:217
SEQUENCE TYPE:nucleic acid
TOPOLOGY:linear
CLONE:HUMGS03811
SEQUENCE DESCRIPTION:
GATCTATATG AAGTGGGAAT ACAGCATATA TCTGGATATT CTTATAGTTA TCTTTTTAAC 60
ATCTTATTTT TTTCATTAAT TACATATCAA CATTAATNTT GTATCTNGAA GCAAATNGAT 120
TTTGTATAAT TAAATGTGTC AAGCATCTGT ATTAATTGAT TNGATGGCAT AAGGTATGAA 180
ANTANTGTAC TGCCCCATGT ATNACTGTTC CAACCAN 217

SEQ ID NO:3241
SEQUENCE LENGTH:214
SEQUENCE TYPE:nucleic acid
TOPOLOGY:linear
CLONE:HUMGS03812
SEQUENCE DESCRIPTION:
GATCATAAGA CCAGGAGTTC AAGACCGGTC TGGCCAACAT GGTGAAGCCT CATTTNTACT 60
AAAAATACAA AATNAGCCAG GTGTGGCGTC ACATGCCTGT AATCCCAGCT ACTCAGGAAG 120
CTGAGGCAGG AGAATTGTTT GAACCTGGAA GGTGGAGGTT GCAGTGAGCC GAGATTGTGC 180
CACTGCACTT GACAGAGCGA GACTCTGTCT CAAA 214

SEQ ID NO:3242
SEQUENCE LENGTH:214
SEQUENCE TYPE:nucleic acid
TOPOLOGY:linear
CLONE:HUMGS03813
SEQUENCE DESCRIPTION:
GATCATCGAG GACGAGAAGA TGGTGGTGGT GGCGNTGCAG CCGACTGCAG AGCTNGAGGT 60
GGGCTCGGCG GAGGTCATTG TGGAGTCCCT GGCCCAGGGN GGCCTGGCCT CCCAGCTCCC 120
CGGCCAGAGA CTGTGTGCAG AGNAGAGCTT CACCGGCCAG GTGTCCTGGA GCCCTCCCTC 180
ATNATNACAG CTGCTGTCCC CGAGGACTGT GATN 214

SEQ ID NO:3243
SEQUENCE LENGTH:212
SEQUENCE TYPE:nucleic acid

TOPOLOGY:linear
CLONE:HUMGS03814
SEQUENCE DESCRIPTION:
GATCTGGGGA GCTNTGACTG AAGGGGCTTG GTCTTCCACT CAGCATCAGC NTGGCAGTCA 60
CCACCCCAGT NAGGACCTCG ATGTCCAGCT GCTGTCAGGT CTGATAGTCC TCTGCTAAAA 120
CAACACGATT TACATAAAAA ATCTTACACA TCTGCCACCG GAAATACCAT GCACAGAGTC 180
CTTAAAAAAT AGAGTGCAGT ATTTAAACCA AA 212

SEQ ID NO:3244
SEQUENCE LENGTH:211
SEQUENCE TYPE:nucleic acid
TOPOLOGY:linear
CLONE:HUMGS03815
SEQUENCE DESCRIPTION:
GATCTCTGTA GACTAGAGTC TTGCAGCAAC ACAGAATGTA ATATAAGGCA AATGCATCTG 60
GGACTTGACC AAAGTTGTTC TGTTTTGTTT TTTTAACTGA AAGTAACAGA AGGACCTTTC 120
TTAAATGTGA CAGATGGTCC TGCAGTGTGA ACCTGAAGGA CAGTGTTAAA GCTGGGCTCT 180
AGTATATTGA TGATTTCTGC ATAAGTGTGG N 211

SEQ ID NO:3245
SEQUENCE LENGTH:210
SEQUENCE TYPE:nucleic acid
TOPOLOGY:linear
CLONE:HUMGS03816
SEQUENCE DESCRIPTION:
GATCTATTTT TGCACTGGAA TATCTGAGAA TTGCAAAACT AGACAAAAGT TTCACAACAG 60
ATTTCTAAGT TAAATCATTT TCATTAAAAG GAAAAAAGAA AAAAAATTTT GTATGTCAAT 120
AACTTTATAT GANGTATTAA NNTGCATATT TCTATGTNGT AATATANTGN GTCACAAANT 180
AANGCTGTGA CAGTNCTGTT GGTCTACAAA 210

SEQ ID NO:3246
SEQUENCE LENGTH:210
SEQUENCE TYPE:nucleic acid
TOPOLOGY:linear
CLONE:HUMGS03817
SEQUENCE DESCRIPTION:
GATCTTTTAC CACTAAAAGA GCATTATTCT CATGTCATAA TGAGAATAAT AATTTACATA 60
CTTGGCATAA TAAATGCCTA AAAGACATTT TATTNTCTGA ATCTATTTTT TTCTTGCTAT 120
AATGGGGATA TTGTAAATAA TGCATTTGTA TTAATGGTAT TTCTTAAAGC AATCTATGTA 180
ACTGTAAATT AAACCAATCT ACAAACTAAA 210

SEQ ID NO:3247
SEQUENCE LENGTH:210
SEQUENCE TYPE:nucleic acid
TOPOLOGY:linear

CLONE:HUMGS03818
SEQUENCE DESCRIPTION:
GATCAGAAGT GGCTAATTTA TTTNATTTCT NATTATTTTA TCCAGAGGGT ACTTTTTTAA 60
TGGATATTTG TAAATCTNCC ACTTAACCAC TGAAAATAAT NTTNTTTTAA TCCCACCCTT 120
CCATCCATAC CTCTGCCTCC CCAAAAAGCT CCTATNAATT TNCTTATCCC CCTCATGTAG 180
CTAGTTGAAT GTGAATAAAT AACATGGAAA 210

SEQ ID NO:3248
SEQUENCE LENGTH:206
SEQUENCE TYPE:nucleic acid
TOPOLOGY:linear
CLONE:HUMGS03819
SEQUENCE DESCRIPTION:
GATCTCCATT CTTTGCCATG AGGGCCAACA CATGAGACCC CACCTCCCTC CCTGCCTGCC 60
AGCCCTAGAC TTGTTGGAGC ATAGAGCCCT TCCTCCCCAG GCCTAAGTAT GTGGAGCTCT 120
GCTTTGGCAC TGCCCCGCAG AGGCCACGCC TATTTATTCT GTTTCTNTTG TTTTGTTTTT 180
AACAACTATA CTTTGCACAC ATGAAA 206

SEQ ID NO:3249
SEQUENCE LENGTH:209
SEQUENCE TYPE:nucleic acid
TOPOLOGY:linear
CLONE:HUMGS03820
SEQUENCE DESCRIPTION:
GATCCCCAGA GTTGGTCCAA GGAGGGAGAG TGGGTTCTCA ATACGGTACC AAAGATATAA 60
TCACCTAGGN TTACAAATAT TTTNAGGACT CACGTTAACT CACATTTATA CAGCAGAAAT 120
GCTATTTTGT ATGCTGTTAA GTTTTTCTAT CTGTGTACTT TTTTTAAGG GAAAGATTTT 180
AATATTAANC CTGGTGCTTC TCACTCAAA 209

SEQ ID NO:3250
SEQUENCE LENGTH:335
SEQUENCE TYPE:nucleic acid
TOPOLOGY:linear
CLONE:HUMGS03821
SEQUENCE DESCRIPTION:
GATCTGTGTG GTCCCCGACG TAACATCTGG CGAGACCCTG CCCTCTGCTG TTACCTGAGT 60
CCTGGGGATG AACAGGTCAA CTGCTTCAAC ATCAATTATC TGAGGAACGT GGCTCTAGTG 120
TCTGGAGACA CTGAGAACGC CAAGGGCCAG GGGGAGCNGG GCTCAACTGG AGGAACAAAT 180
ATCAGCTCCA CCTCTGAGCC CAAGGAAGAN TGAGTCACCC CAGAGCCCTA GAGGGTCAGA 240
TGGGGGGAAC CCACCCTGCC CCACCCATCT GANCACTCAT TACACTAAAC AACTTTTTGG 300
GNAANTNTNA TNTATGATGN GNNGNGNGTG GGGAN 335

SEQ ID NO:3251
SEQUENCE LENGTH:207
SEQUENCE TYPE:nucleic acid

TOPOLOGY:linear
CLONE:HUMGS03822
SEQUENCE DESCRIPTION:
GATCTAATTC TNCACTCCTG CTCCAAGGGC TGGGCTGTGG GTGGGATACT GGGATTTTGG 60
GCCACTGGAT TTTCCCTAAA TTTGTCCCCC CTTTACTCTC CCTCTATTTT NCTCTCCTTA 120
GACTCCCTCA GACCTGTAAC CAGCTTTGTG TCTTTTTNCC TNTNCTCTCT TTTAAACCAT 180
GCATTATAAC TTTGAAAGCA AAACAAA 207

SEQ ID NO:3252
SEQUENCE LENGTH:201
SEQUENCE TYPE:nucleic acid
TOPOLOGY:linear
CLONE:HUMGS03823
SEQUENCE DESCRIPTION:
GATCTCATTT NAATGATTTA TAGGTAAATA CTAATCAGAC ATTATTAAAA GCAAAACAGG 60
AAAAAGGTAA ACTTCTTAAA TTTAGTTACC TATAAAAATT GTCAATNTTN ATTCTTTAAA 120
AACACATGGA CTTACTATAA AAGCCTTTTT GTACTAGTGA AAAGAATCTT CAGCTATATA 180
GAAATAAAGT TATACTTTAA A 201

SEQ ID NO:3253
SEQUENCE LENGTH:201
SEQUENCE TYPE:nucleic acid
TOPOLOGY:linear
CLONE:HUMGS03824
SEQUENCE DESCRIPTION:
GATCCCTAAA AGACAAAAGA GTCTTGGAAA GGACTGCTGG CTTCGTCTTG AACGTTCGCC 60
CCATCACCAG TGTGAGTCCG TGGGGAAGTC TTCCTCAAGT GGGGTTCAGT CTGTAATTTT 120
CCTGTCTGTT CTTAGCNNNG TTCTGAGTTA TTTTGGATTT TNTTCTTTAG CCAAATAAAG 180
GCAGTGTGGT TTTACCTGAA A 201

SEQ ID NO:3254
SEQUENCE LENGTH:200
SEQUENCE TYPE:nucleic acid
TOPOLOGY:linear
CLONE:HUMGS03825
SEQUENCE DESCRIPTION:
GATCCTAATG GTGCTGCCCT ATTTATACCT GGGTCTGCAT TAAAAGGGAA AGCCCCCCCT 60
GTTGTAGATT TCATCTGCTT CCTCCTTAGG GNAGGCTGGG ATATGATGAG AGATTCCAGC 120
CCAAGCCCGG CCCCCCACCG CCAGGCCATA GGGCATANTT TGCATCTCAA ATCTGAGGAA 180
TAAACTGATG ACCTGTGAAA 200

SEQ ID NO:3255
SEQUENCE LENGTH:391
SEQUENCE TYPE:nucleic acid
TOPOLOGY:linear

CLONE:HUMGS03826
SEQUENCE DESCRIPTION:
GATCAAGCAT TTGCGTTTGG ATGGCAATCG CATCTCAGAA ACCAGTCTTC CACCGGATAT 60
GTATGAATGT CTACGTGTTG CTAACGAAGT CACTCTTAAT TAATATCTGT ATCCTGGAAC 120
AATATTTTAT GGTTATGTTT TTCTGTGTGT CAGTTTTCAT AGTATCCATA TTTTATTACT 180
GTTTATTACT TCCATGAATT TTAAAATCTG AGGGAAATGT TTTGTAAACA TTTATTTTTT 240
TTAAAGAAAA GATGAAAGGC AGGCCTATTT CATCACAAGA NCACACACAT ATACACGGTT 300
AGACATCAAA CTCAATGCTT TATTTGTNAA TTTAGTGGTT TTNTATTTCT ACTGTCAAAT 360
GATGTGCAAA ACCTTTTTNC CGGGTTGCAT N 391

SEQ ID NO:3256
SEQUENCE LENGTH:194
SEQUENCE TYPE:nucleic acid
TOPOLOGY:linear
CLONE:HUMGS03827
SEQUENCE DESCRIPTION:
GATCTTTAAT TTGNATGGTG ATGAATGTCT TAGTCATGAA GAGTTTCTAT GGGGTGTNAA 60
AAAACAGAAT GCATCGAGGT TNATGGGTAC CACAACATCA GAGTATACAA GAATACTGGA 120
AGTGTGTGAA GAAAGAAAGC ATTAAAGGAG TAAANGANGT CTGGAAACAN GCTGGAAANG 180
GTCTTTTTNA ATNN 194

SEQ ID NO:3257
SEQUENCE LENGTH:190
SEQUENCE TYPE:nucleic acid
TOPOLOGY:linear
CLONE:HUMGS03828
SEQUENCE DESCRIPTION:
GATCTAGTAG GTTTCTATTT TTCCTTTCTC TTTACAATGC ACATAATACT TTCCTGTATT 60
TATATCATAA CGTGTATAGT GTAAAATNTG AATNACTTTT TTTGTGAATG AAAATCTAAA 120
ATCTTTGTAA CTTTTTATAT CTGCTTTTGT TTCACCAAAG AAACCTAAAA TCCTTCTTTT 180
ACTACACAAA 190

SEQ ID NO:3258
SEQUENCE LENGTH:190
SEQUENCE TYPE:nucleic acid
TOPOLOGY:linear
CLONE:HUMGS03829
SEQUENCE DESCRIPTION:
GATCCCCTTT TNTNCTGGAG CTTCCGTGCC TCCCCTGCCT CTTCCCAGCC CTTGTAATCC 60
TCCTTCACAG GCCAGCCAGA TACCTGTNTG GCCTNTGGGC CCATTTTNTT CCCTGTCTGT 120
CCCCACCCCA CAAGAACCAG GTTTCTNTAA TAAATTCTCT CCCTTCTTTA GCCAGGACTT 180
AGCCCGCAAA 190

SEQ ID NO:3259
SEQUENCE LENGTH:189

SEQUENCE TYPE:nucleic acid
TOPOLOGY:linear
CLONE:HUMGS03830
SEQUENCE DESCRIPTION:
GATCTNAGAG CCTGCCCAAC AGCAGGGAAG CCAAGCACCC ATTCATCCCC CTGCCCCCAT 60
CTAACTGCGT ANTGANAAGG GGAACAGTGC CATGTACCAC ACAGATGTTC CTGTCTCCTC 120
GCATGGGCAT AGGGACCCAT CATTNATGAC TGATGAAACC ATGTAATAAA GCATCTCTGG 180
GGAGGGAAA 189

SEQ ID NO:3260
SEQUENCE LENGTH:188
SEQUENCE TYPE:nucleic acid
TOPOLOGY:linear
CLONE:HUMGS03831
SEQUENCE DESCRIPTION:
GATCCCCTTC CTATCAATCA CCTGATAGCA ACAGGGACAG CTGCCAATAC CCTGCTCTTT 60
ACTCAATGGT ACCCAGGGAG GGAGCATGGG AAGAGGGTGA GCTGAGGGCT GGAGGAGGGC 120
AACAGCCACT GGGTGAGCTG TTCACGGTCT TATACTATTG TTTGTNATTA AAAGTGCTTC 180
AACCCAAA 188

SEQ ID NO:3261
SEQUENCE LENGTH:184
SEQUENCE TYPE:nucleic acid
TOPOLOGY:linear
CLONE:HUMGS03832
SEQUENCE DESCRIPTION:
GATCAACAAG GCCACGCCCT ACAACTACCC AGTGCCCGTC CGTGATGATG GGAACATGCC 60
CGACGTGCCC AGCCACCCCC AGGACCCTCA GGGCCCCAGC CTGGAGTGGC TGAAGAAACT 120
GTGAGCACCT CCACTGACAG AGGCGGCCCC TCCCACGGNT CCCAATAAAA ATGTGAAAAC 180
CAAA 184

SEQ ID NO:3262
SEQUENCE LENGTH:184
SEQUENCE TYPE:nucleic acid
TOPOLOGY:linear
CLONE:HUMGS03833
SEQUENCE DESCRIPTION:
GATCCACCAA TGTTTCAAAT AGGACTATAA CTTATTCAAA GGCCAATNAT ACTTCGCATA 60
ATTCAATTTT AATATTATTC ACATTTCATG TCACAAATTT AGTAAGTTAC ATGTGTTATG 120
TGATGTTTTG TTTTGATAGA TTATATCTTA CTCCTAAATA AAANGTCAAG ATTTTTGTAG 180
CAAA 184

SEQ ID NO:3263
SEQUENCE LENGTH:181
SEQUENCE TYPE:nucleic acid

TOPOLOGY:linear
CLONE:HUMGS03835
SEQUENCE DESCRIPTION:
GATCTCTCTG TGTGGAATGC CTTGGTGAGA GAGATGCTTA TAATNACTAT NATNATTTCT 60
AACCAAGCTT CTATNAATNT AATTTCTAAT AATACACTAT CTTGATTGTA CTCTCCAGAA 120
AATTTTNCTG TCAGTGAAAA TAAAAGAAAA ATTAAAGTAA AGCTAAGGAA CTGTCTATAA 180
A 181

SEQ ID NO:3264
SEQUENCE LENGTH:180
SEQUENCE TYPE:nucleic acid
TOPOLOGY:linear
CLONE:HUMGS03836
SEQUENCE DESCRIPTION:
GATCCCTGGA CCATCTGCAT CCAGAGGACC GTCCGTAACG GCCGGGGGTC CAGGCGGACC 60
TTGTGGTGAC CCGGCTCGGG CNTNTCCTCG GTTTCNTTGC CTCACCCGCG GAGAGCGCTG 120
AACCTGGACA AGCAGCGGCT GGNAAGNACA GGTCCAATAA ACGCCCTCTG CGCCCAGAAA 180

SEQ ID NO:3265
SEQUENCE LENGTH:227
SEQUENCE TYPE:nucleic acid
TOPOLOGY:linear
CLONE:HUMGS03837
SEQUENCE DESCRIPTION:
GATCTAGTTC AGAAGGAAGC AAAATCCCTT AATCTATGTG CACCGTTGGG ACCAATGCCT 60
TAATTAAAGA ATTTAAAAAA GTTGTAATAG AGAATATTTT TGGCATTCCT CTAATGTTGT 120
GTGTTTNTTT TTNGTGTGTN CTGGAGGGAG GGGATTTAAT TTNAATTTNA AAATGTTTAG 180
GAAATTTNTN CAAGGAANCT TTTTAATAAN GTATATNGAA AGNTAAA 227

SEQ ID NO:3266
SEQUENCE LENGTH:171
SEQUENCE TYPE:nucleic acid
TOPOLOGY:linear
CLONE:HUMGS03838
SEQUENCE DESCRIPTION:
GATCACTTCT CCTTGGCTTC CTTCTTTTCT GTGCTTGCAT CAGTGTGGAC TCCTAGAACG 60
TGCGACCTGC CTCAAGAAAA TGCAGTTTTC AAAAACAGAC TCAGCATTCA GCCTCCAATG 120
AATAAGACAT CTTCCAAGCA TATAAACAAT TGCTTTGGTT TCCTTTTGAA A 171

SEQ ID NO:3267
SEQUENCE LENGTH:338
SEQUENCE TYPE:nucleic acid
TOPOLOGY:linear
CLONE:HUMGS03839
SEQUENCE DESCRIPTION:

GATCACTTTA TTTGCTAGGG AGGGAAGTCC TAGGGTGGTT TCAGTTTCTC CCAGACATAC 60
CTAAATTTTA ACATCAATCC TTTTAAAGAA AATCTGTATT TCAAAGAATC TTTCTCTGCA 120
GTAAATCTCG CAGGGGAATT TGCACTATTA CACTTGAAAG TTGTTATTGT TAACCTTTTC 180
GGCAGCTTTT AATAGGAAAG TTAAACGTTT TAAACATGGT AGTACTGGAA ATTTTNCAAG 240
ACTTTTACCT AGCACTTAAN TATGTATAAN TGTACATAAN GACAANCTAG TAAGCNTGGC 300
CTGGGGANAT GGTCAGNCCT TGTNTTGTGT TTTTGGCN 338

SEQ ID NO:3268
SEQUENCE LENGTH:170
SEQUENCE TYPE:nucleic acid
TOPOLOGY:linear
CLONE:HUMGS03840
SEQUENCE DESCRIPTION:
GATCATAGGA GAAAACCATT TCANATGACA AGAGCACCTC AAAGGCAGCA GCCTCAAGGA 60
GCAGCCATGG CCCCAGACTT GTCGCACGGA TGCAGAAAAC TTAATGGAGG AGGCTGAGGT 120
CAGAATGGGA AGAGTTTTTA AAAAATAAAA AGGGGAGCTA ATATGTGAAA 170

SEQ ID NO:3269
SEQUENCE LENGTH:168
SEQUENCE TYPE:nucleic acid
TOPOLOGY:linear
CLONE:HUMGS03841
SEQUENCE DESCRIPTION:
GATCAAGGGC AAAAACTGGT CATTAAGTNA TCTAANATTA AATNATTTAG CCACTAAGTA 60
ATTTNTTTAC TCTNACTTTA AACTCACCAA AGAAGATTCT TTTAAAGAAA TTATGAAAAA 120
TGTACAATTT AACATTTTAA ATAANTAGTG ACAGANGTTG TTTATAAA 168

SEQ ID NO:3270
SEQUENCE LENGTH:167
SEQUENCE TYPE:nucleic acid
TOPOLOGY:linear
CLONE:HUMGS03842
SEQUENCE DESCRIPTION:
GATCCTCCTG CCCCAGCATT CCAAAGTGCT GGGATTACAG GTGTGAGCNA CTGCCTGGCC 60
TGGCCGTAGG TTTGTAACTG TTTCATAGAA GAGCCCTGGA GAAGACAGTA GAATNAGCCT 120
ATCTAGTTTA AAAAATAAAG AAGCACGTTG ATTTCACCAC CGCCAAA 167

SEQ ID NO:3271
SEQUENCE LENGTH:167
SEQUENCE TYPE:nucleic acid
TOPOLOGY:linear
CLONE:HUMGS03843
SEQUENCE DESCRIPTION:
GATCAGCTTT GTTTTTCCCA GAGTTCATGC AAATAGAATC TGGCTTCTNT AAGTNAGCAT 60
CATGGACTTG GTTNAAATTC GCCCGTNTTG CTGAGTGTAT CTTTAGCTTG TCCCTTTTAA 120

CTGACGAGTA GTATTCCACA GTNTGGATGC ACTGTATTNN NTTTATN 167

SEQ ID NO:3272
SEQUENCE LENGTH:166
SEQUENCE TYPE:nucleic acid
TOPOLOGY:linear
CLONE:HUMGS03844
SEQUENCE DESCRIPTION:
GATCAGAGCA TTGTGCAATA CAGTTTCATT AACTCCTTCC CCCGCTCCCC CAAAAATTTG 60
AATTTTTTTT TCAACACTCT TACACCTGTT ATGGAAAATG TCAACCTTTG TAAGAAAACC 120
ANANTAAAAN TTGAAAANTA AAAACCNTAA ACATTTGCAC CAGAAA 166

SEQ ID NO:3273
SEQUENCE LENGTH:165
SEQUENCE TYPE:nucleic acid
TOPOLOGY:linear
CLONE:HUMGS03845
SEQUENCE DESCRIPTION:
GATCTNTTTT TTTCCCTTCT TGTATCATCC CTTTGGGTTT TTCGGTTTGC TTTGGTTTTT 60
AATTTTNCTT TGTTTTTNCT ATAAATCCTG TTTTAATCTT ATAAGGGAAA ATNCATTTTN 120
TTCAACTGTN CTAGTTAATA ATGAGAACTC AATTNCTGGA AATNN 165

SEQ ID NO:3274
SEQUENCE LENGTH:163
SEQUENCE TYPE:nucleic acid
TOPOLOGY:linear
CLONE:HUMGS03846
SEQUENCE DESCRIPTION:
GATCTCAGGA ATNACATTTT CCAACAGACC AAAAAATGTT TTCATGTAGC AGCAATGCAG 60
ATTTGGTGAA TATTTAATAT ATATTTAAGT ATGTATTTCA CTTTATNACT GACAATNAAA 120
AAATATTGTT TGGCCAAATA GTAAACACCC TTTTGAAACC AAA 163

SEQ ID NO:3275
SEQUENCE LENGTH:163
SEQUENCE TYPE:nucleic acid
TOPOLOGY:linear
CLONE:HUMGS03847
SEQUENCE DESCRIPTION:
GATCCCTTNG TTTGCTTAAT AAATTATAAA ATAATGGCTT GGAAAAGCAG GCTAGTCTAA 60
CCATGGTGCT ATNATNAGGC TTGCTTGTTA CACACACAGG TCTAAGCCTA GTATGTCAAT 120
AAAGCAAATA CTTACTGTTT TGTTTCTATT AATGATTCCC AAA 163

SEQ ID NO:3276
SEQUENCE LENGTH:164
SEQUENCE TYPE:nucleic acid

TOPOLOGY:linear
CLONE:HUMGS03848
SEQUENCE DESCRIPTION:
GATCTGTTCT TGGTATTTNN TTTTGTTTTG TTTTGGTTTT TAAATGTAAT CACCCATTGG 60
TCAGGCCCAG GACTGGTCAC CATGAGCTCT GCTAGCCACG GCCCCAACGA TGCTTCCGGC 120
TCTCATGGAT TCCACAGCAA ATAAAACTGT GCTGACAACA GAAA 164

SEQ ID NO:3277
SEQUENCE LENGTH:162
SEQUENCE TYPE:nucleic acid
TOPOLOGY:linear
CLONE:HUMGS03849
SEQUENCE DESCRIPTION:
GATCTGGGGT CCCTAAGTCC CTCTCTTTAA AGAACTTCTG CGGGTCAGAC TCTGAAGCCG 60
AGTTGCTGTG GGCGTGCCCG GAAGCAGAGC GCCACACTCG CTGCTTAAGC TCCCCCAGCT 120
CTTTCCAGAA AACATTAAAC TCAGAATTGT GTTTTCAGCA AA 162

SEQ ID NO:3278
SEQUENCE LENGTH:162
SEQUENCE TYPE:nucleic acid
TOPOLOGY:linear
CLONE:HUMGS03850
SEQUENCE DESCRIPTION:
GATCCTAGGA ACTCCTATNT TCAGAAAAGA ATTTTNTTCC TACTATATAA TTACAGTATT 60
TAGCTGTCAA TTTTAAGATG AATTTGGTAG AGCCTTATAG TAAAGNATGT ATCTTGGTCA 120
CACACAAAGC TTGGAAAAAG TAAAAGATGT CTAAACCATA AA 162

SEQ ID NO:3279
SEQUENCE LENGTH:160
SEQUENCE TYPE:nucleic acid
TOPOLOGY:linear
CLONE:HUMGS03851
SEQUENCE DESCRIPTION:
GATCAAGAAG TAGCCGAGGT TGATAAAAAC ATAGAACTTT TGAAAAAGAA GGATGAAGAA 60
CTCAGTTCTG CTCTGGAAAA AATGGAAAAT CAGTCTGAAA ACAATGATAT CGATGAAGTT 120
ATCATTCCCA CAGCTGCTCA ATAAAGTACA AGAGCTTAAA 160

SEQ ID NO:3280
SEQUENCE LENGTH:177
SEQUENCE TYPE:nucleic acid
TOPOLOGY:linear
CLONE:HUMGS03852
SEQUENCE DESCRIPTION:
GATCGTGGGT CAGGGACACC GAGAGAGTGA GGTCACTTCC ACTTCAAACC TTCAGTGAGG 60
GGGTGGGATG GAGAGAATGC TGAATCTTTT TTTTNACGGG ATGGGGTTTT CNTCTTTGTA 120

ATTATNNCTT TAGTTTAATT AACCTTTTGG TTGTTTGTNC AATATTATAT ATNNNAN 177

SEQ ID NO:3281
SEQUENCE LENGTH:232
SEQUENCE TYPE:nucleic acid
TOPOLOGY:linear
CLONE:HUMGS03853
SEQUENCE DESCRIPTION:
GATCAGGACT ATAATATTGT ACAGTTATTA TAGGGCTTTT GGGGAAGGGG AGGATAGCGA 60
GAAGATGCTC TGGGGGTTTT GTTTTTGCTT TTCCTTCAGG GTTTTATTTT TGACTGTTTT 120
GTTTTCTNGT NGGCCATTTC TGTATTGCTG GCATCTGTGC TAAGCTTTAC AGTGGCAAAA 180
ATAATGACAT GTAGCAAAGA TTTTCAAACA AAATATTTTT NCCTTTTGTA AA 232

SEQ ID NO:3282
SEQUENCE LENGTH:158
SEQUENCE TYPE:nucleic acid
TOPOLOGY:linear
CLONE:HUMGS03854
SEQUENCE DESCRIPTION:
GATCGATGGT GGCGCTTTCT CCTGTGCCCA CCCGTCTTCA ATCTCTGTTC TGCTCCCAGA 60
TGCCTTCTAG ATTCACTGTC TTTTGATTCT TGATTTTCAA GCTTTCAAAT CCTCCCTACT 120
TCCAAGAAAA ATAATTAAAA AAAAAACTTC ATTCTAAA 158

SEQ ID NO:3283
SEQUENCE LENGTH:158
SEQUENCE TYPE:nucleic acid
TOPOLOGY:linear
CLONE:HUMGS03855
SEQUENCE DESCRIPTION:
GATCTCATCA CAAGATGACC AAAAGAGTTT GAACTGTGTG TGCTATTTAG AAGATTTAAC 60
TTTNATGTAT CTATAGATAC CACAGCTGTT GCTTCTAATA AATTTACCTA CCTTTNAACT 120
GAAAGTTTAA TAAAGCCAAA TCAATTTGAA ATACCAAA 158

SEQ ID NO:3284
SEQUENCE LENGTH:390
SEQUENCE TYPE:nucleic acid
TOPOLOGY:linear
CLONE:HUMGS03856
SEQUENCE DESCRIPTION:
GATCTGCCTC ACGTGCACTG TGGTGGCCGT GTGCTACGGC TCCTTCTACA ATCTCCTCAC 60
CCGAACCTTC CACATCGAGG AGCCCCGCAC AGGTGGCCTG GCCAAGCGGC TGGCCAACCT 120
TATCCGGCGC GCCCGAGGTG TCCCCCCACT CTNATTCTNG CCCTTTCCAG CAGCTGCAGC 180
TGCCGTTTCT NTCTGGGGAG GGGAGCCCAA GGGCTGTTTC TGCCACTTGC TCTCCTCAGA 240
GTTGGCTTTT GAACCAAAGT GCCCTGGACC AGGTCAGGGC CTACAGCTGT GTTGTCCAGT 300
ACAGGAGCCA CGAGCCAAAT GTGGCATTTG AATTTGAATT AACTTTAGAA ATTCATTTGC 360

TCAACTTGTT AGTGGCCACC TCTTATATTN 390

SEQ ID NO:3285
SEQUENCE LENGTH:156
SEQUENCE TYPE:nucleic acid
TOPOLOGY:linear
CLONE:HUMGS03857
SEQUENCE DESCRIPTION:
GATCTCAACA CCTATGCCTC CACGTAAATT ATTTTTTTCA GCCTTATTTC CCTCAAAATT 60
GTAGAGTCCT AATCTTCTTC TATCTTGTGG TGTGAGACCT TGGATAAGTC TCCCTTCCAC 120
CTTTTCCTAT TACTTAAAAT GGTGAATTTG CATAAA 156

SEQ ID NO:3286
SEQUENCE LENGTH:155
SEQUENCE TYPE:nucleic acid
TOPOLOGY:linear
CLONE:HUMGS03859
SEQUENCE DESCRIPTION:
GATCAGGTGA TTTGTAAAAT TGTATTTATC TGTACATGTA TGGGCTTTTA ATTCCCACCA 60
AGAAAGAGAG AAATNATCTT TTTAGTTAAA ACCAAATTTC ACTGTTCAAA NTATCTTCCA 120
ACTTATTTAT TGGGTTGTCA CTCAATTGCC CTGTN 155

SEQ ID NO:3287
SEQUENCE LENGTH:137
SEQUENCE TYPE:nucleic acid
TOPOLOGY:linear
CLONE:HUMGS03860
SEQUENCE DESCRIPTION:
GATCTTGCAA CTCCATTTCG GAAGACACCG AGGAAGAGGA GGAAGATGAA GACCAGGACT 60
ACAGCTTTCC TATATCTCCT ATTCTAGAGT GGTAAACTCT CTNTAAGTNT TCAGTGTTGA 120
CATAGCCTTT GTGCAAA 137

SEQ ID NO:3288
SEQUENCE LENGTH:152
SEQUENCE TYPE:nucleic acid
TOPOLOGY:linear
CLONE:HUMGS03861
SEQUENCE DESCRIPTION:
GATCAGTGGG CACAGTTCTT CAGCTACATT GAGACCCTGA AATAAACAAT NATATTCTNA 60
CTCGACATCT TGTCCCCAAT CCTTCCAAAA ATATTGATGG TGATTTGTGC TACCATTTAC 120
TCGTTTATTT AATAAAGACA TTCAATCCCA AA 152

SEQ ID NO:3289
SEQUENCE LENGTH:152
SEQUENCE TYPE:nucleic acid

TOPOLOGY:linear
CLONE:HUMGS03862
SEQUENCE DESCRIPTION:
GATCAGNATT GTACGTATTC AAGGTGTAAA ATNTGATAAT TTGTCGTACA CGTACATTGT 60
GCAATAACAG TCACAATNAA TTCCTCAGCG CACCCATCAC CACGAATACG ATACATTAGA 120
TATTCTGAAC TTGCTCATCT TAGGACTTCA AA 152

SEQ ID NO:3290
SEQUENCE LENGTH:153
SEQUENCE TYPE:nucleic acid
TOPOLOGY:linear
CLONE:HUMGS03863
SEQUENCE DESCRIPTION:
GATCATAATA GTGACCAAAA TATACATGCA GACTTGTTTT TTATTATTGT TGTTTAAGCA 60
TAATTTAAGA AAAAAAATTT TTACCTGGTG AACTTGCTAT CTGCTCTGTT TCTAGTTAAA 120
ANATAATANA TNTTATCTNC CCTGTGCTGT AAA 153

SEQ ID NO:3291
SEQUENCE LENGTH:144
SEQUENCE TYPE:nucleic acid
TOPOLOGY:linear
CLONE:HUMGS03864
SEQUENCE DESCRIPTION:
GATCTTCTTT CTTTTTACAG GAACAATTGG CTTCTTTGCA TGCTTTTGGT TTGTNACCAA 60
AATATACAGT GTGGTGAAGG TTGACTGAAG AAGTCCAGTG TGTCCAGTTA AAACAGAAAT 120
AAATTAAACT CTTCATCAAC AAAA 144

SEQ ID NO:3292
SEQUENCE LENGTH:163
SEQUENCE TYPE:nucleic acid
TOPOLOGY:linear
CLONE:HUMGS03865
SEQUENCE DESCRIPTION:
GATCATGGTG AACTTACCAA AGCAAACAAT GCCTGTAAGA TGGTCCTGCA GCAGCCAACC 60
AGTGAACTCT TTTGGTGACA TCCTGTTCTT GTTGTATAAC TTTATATTCC TATAAATCCA 120
TTAAGGCCCC AATAAAGTTT GTCTCTAAGC GCTGTGTTAG AAA 163

SEQ ID NO:3293
SEQUENCE LENGTH:149
SEQUENCE TYPE:nucleic acid
TOPOLOGY:linear
CLONE:HUMGS03866
SEQUENCE DESCRIPTION:
GATCCAAAAT TAGAGCTCAT CAGANTTGTC ATTGAAAGTG TTAACAAAAG TAATATCAAA 60
TGACATTTGA AAAATATCCT TGTAAAATGA CTATATTAAT AGGTATAAGG NTTTTCCTTA 120

TTAATCTAAT AAAATNCCTT AATAGGAAA 149

SEQ ID NO:3294
SEQUENCE LENGTH:157
SEQUENCE TYPE:nucleic acid
TOPOLOGY:linear
CLONE:HUMGS03867
SEQUENCE DESCRIPTION:
GATCAGGACA CTTAGCAAAT GTAAAAATAA AATCTAACTC TCATTTGACA AGCAGAGAAA 60
GAAAAGTTAA ATACCAGATA AGCTTTTGAT TTTTGTATTG TTTGCATCCC CTTGCCCTCA 120
ATAANTAAAG TTCTTTTTTA GTTCCAAATT TNAGAAA 157

SEQ ID NO:3295
SEQUENCE LENGTH:148
SEQUENCE TYPE:nucleic acid
TOPOLOGY:linear
CLONE:HUMGS03868
SEQUENCE DESCRIPTION:
GATCCGNTAG GAGTAGTAAA TGNGAGACAC AATNGCTTGA TTATACCTCN TATNTGGTTT 60
AGCTTCAGTA TTTAAACAAG GAAATAAACT TGAAAATTAT TTGTCATCAT AAAAATGAAA 120
CAAANTAAAA TATTTATTGC CAGGNAAA 148

SEQ ID NO:3296
SEQUENCE LENGTH:332
SEQUENCE TYPE:nucleic acid
TOPOLOGY:linear
CLONE:HUMGS03869
SEQUENCE DESCRIPTION:
GATCTCAGAG GCTCAGAGAC TGCAAGCTGC TTGCCCAAGT CACACAGCTA GTGAAGNNCA 60
GAGCAGTTTC ATCTGGTTGT NACTCTAAGC TCAGTGCTCT CTCCACTACC CCACACCAGC 120
CTTGGTGCCA CCAAAAGTGC TCCCCAAAAG GAAGGAGAAT GGGATTTTNN TTTTGAGGCA 180
TGCACATCTG GAATTAAGGT CAAACTAATT CTCACATCCC TCTAAAAGTA AACCTACTGG 240
TTAGGAGCAG CAGTGTTCTC ACAGTGTGGG GTCAGCCGTC CTNCTTNATN NNNGGCCAAT 300
GGNTATTGAC ACGTGTNCNN NGTTTGGGCA GN 332

SEQ ID NO:3297
SEQUENCE LENGTH:147
SEQUENCE TYPE:nucleic acid
TOPOLOGY:linear
CLONE:HUMGS03870
SEQUENCE DESCRIPTION:
GATCTNCTGT CCTCCTTTCA CTACATACCA ACAACCCTCT GGCATTAAAC TTCCAGTGAT 60
AGTGCTATNA ACTTTGTGTC CTCAGGTCAA CAAGGGACCT CAGTAATTNG TTCTNTACAT 120
AAGTATATAT ATGGGTATTA NTNNGTN 147

SEQ ID NO:3298
SEQUENCE LENGTH:355
SEQUENCE TYPE:nucleic acid
TOPOLOGY:linear
CLONE:HUMGS03871
SEQUENCE DESCRIPTION:
GATCGCCCTC TACATGGCTT CGCAGCCCAA AAACAAATNA GTTAGGCTGC AGAGGACTGG 60
TTTGTTTTTT GGCATAAACC CTTTGAAGTT CCTTTTTCAT TGTTAAATTA AAATTTTTTT 120
NTTNACTTGG ATGGCTTAAC ATTTTTNCAA GAAAANTNGG AAGNTATGAA GATGNTGTTT 180
TGGTTTGTTT ATGAAANGCA TATGGCTTGN CAGAGCTCAT TCGACAGTTA AGGCCATTGT 240
TTAAAGAANC GGTGCTTTGC TCTGTGTTTG TNCTCCTGAT TTCCCTGGAG GTTCTGGATG 300
AGGGCTGANC ACAGGCTTNG TTANTGTNCA GTCTGTGCTG GGGGNCCTCA GGGGN 355

SEQ ID NO:3299
SEQUENCE LENGTH:147
SEQUENCE TYPE:nucleic acid
TOPOLOGY:linear
CLONE:HUMGS03872
SEQUENCE DESCRIPTION:
GATCACCAGG GTCAGTTTNT TTAATAATGG TTTCCAACTG GCCTAATACA TTAAGTAAGA 60
NTGGNTGATA ACATGACCAG ACAGACATAA AGACCCTGTT GGGAATNACA TTGAACTCTC 120
AAAGTCAAGA TTTNTTACAC AANTNTN 147

SEQ ID NO:3300
SEQUENCE LENGTH:147
SEQUENCE TYPE:nucleic acid
TOPOLOGY:linear
CLONE:HUMGS03873
SEQUENCE DESCRIPTION:
GATCCGCTAC TAAAAGTAAA AATAACCGGT TTGTAAAACA TTAAGAGAAA AANTAGGATA 60
TTTAATACAT GTGGTGAGTG GAGANCAGGG TAACTTTAAN TNAAAGTTTA AGGCAAATTG 120
TAAGCNCCAC AAGANATATC ANATTTN 147

SEQ ID NO:3301
SEQUENCE LENGTH:147
SEQUENCE TYPE:nucleic acid
TOPOLOGY:linear
CLONE:HUMGS03874
SEQUENCE DESCRIPTION:
GATCTAAAAA AATTAAAAAG AAATATGAAT GAAAGNAAAA AAAATGCNAA AATNAGCAGT 60
TTCCTGGAGG AGCAGTTCCA GCAGGGCAAG CTTNTTGCTT GCNTCGTTTC AAGGCCGGGA 120
CANTTTGGCC GAGCAGATGG CTATNTN 147

SEQ ID NO:3302
SEQUENCE LENGTH:147

SEQUENCE TYPE:nucleic acid
TOPOLOGY:linear
CLONE:HUMGS03875
SEQUENCE DESCRIPTION:
GATCATNTTA TTTTACAATC ATTTATAATG AATNAATGTT NCAGTTAGCT TTAAAAGGTA 60
TACGGTGCTA ATNAGTAAAA TATTGAAGGC AATATTTTAC TGCTAGCTTG CAAAGTTATG 120
AGAGTTTAAA AAATAAAATA TATGAAA 147

SEQ ID NO:3303
SEQUENCE LENGTH:147
SEQUENCE TYPE:nucleic acid
TOPOLOGY:linear
CLONE:HUMGS03876
SEQUENCE DESCRIPTION:
GATCTAGGGT CCTGGGTNTC TTGCATTTAT ATGTCAGAAA AGGGGCGATA TGCTGCTGAG 60
GGGTGAGTGC ATATAANTNT GGCCCTGAGG ACCAGGGCTG GCAGATNTTG TCTACCTGCT 120
GAAGAATAAA ANATTTCTTT TGGTAAA 147

SEQ ID NO:3304
SEQUENCE LENGTH:150
SEQUENCE TYPE:nucleic acid
TOPOLOGY:linear
CLONE:HUMGS03877
SEQUENCE DESCRIPTION:
GATCTNACCC CCAGCATGAT TGTATTTCTT TCCCCTCCAG TCAAACCGTA ATACAGTTTC 60
CAGAAAATTC GACAGTCTGC AATGCCAAAA GGGTAAAAAT CTGTATTTCA CAGTTGTATA 120
GAATAAAGGC TTTAGTTAAA ATATTTCAAA 150

SEQ ID NO:3305
SEQUENCE LENGTH:146
SEQUENCE TYPE:nucleic acid
TOPOLOGY:linear
CLONE:HUMGS03878
SEQUENCE DESCRIPTION:
GATCTAAAAA TAACTGTAAT TATTTAAATG ACTAANAGGA AAGTACATTT TTTAAAATNC 60
TGAAAATTGC CTTNNTGTGT TTATNCAAAC TGAAAAGCTG AGACCAAGAG CAAGGAAGGT 120
AAAANGTTAA CAGGCAAACA TTTNCN 146

SEQ ID NO:3306
SEQUENCE LENGTH:143
SEQUENCE TYPE:nucleic acid
TOPOLOGY:linear
CLONE:HUMGS03879
SEQUENCE DESCRIPTION:
GATCCTCACC AACCTAAGCA AGAGGAAGTG CCCCTCTCAA CCTGTGCGGG NTGCTGCGTC 60

CCAGGACTGA GACGCAGGCC AGCCCCGGCC CCTAGCCCTC AGGCCTTCTT TNTTATCCAA 120
ATAAATATTT CTTAATAAGG AAA 143

SEQ ID NO:3307
SEQUENCE LENGTH:143
SEQUENCE TYPE:nucleic acid
TOPOLOGY:linear
CLONE:HUMGS03880
SEQUENCE DESCRIPTION:
GATCAACAAG AGCACTGTAC TCCTGGCAAT NATTACATAT GTNAGAACAT GGATTTTGCA 60
CTGTAGACAA CATTTAACAC CAGTCTATGG GGTACTGCAT TGCTTTTNAT AAAGTTCAAA 120
ATAAAGATTT ATTTTCAAAC AAA 143

SEQ ID NO:3308
SEQUENCE LENGTH:142
SEQUENCE TYPE:nucleic acid
TOPOLOGY:linear
CLONE:HUMGS03881
SEQUENCE DESCRIPTION:
GATCCCTTTA CTGGAGCCCA GTATGTGCTG TGTGAGTTAG AAGTCATTCT TGCTGAGAAG 60
GTGAATAGGT AGGGATTTGC CTTGTTTTGT AAGTCTACAA TTTGCCAAGA GTAAATAACA 120
CTGGACCAGC TGTAAAAGTA AA 142

SEQ ID NO:3309
SEQUENCE LENGTH:142
SEQUENCE TYPE:nucleic acid
TOPOLOGY:linear
CLONE:HUMGS03882
SEQUENCE DESCRIPTION:
GATCTGCACA TTTAATCGTG GCTGTTTCTG TATAGCCTAT ACTGCATTAG CCNAAGANAT 60
TGTTGCTTTG TAACTTTTTG CACTATTGTT TTGGCTGGAT TTGTATTACA CACAGTTTTA 120
AAAAAAACAA TTCCACACTA AA 142

SEQ ID NO:3310
SEQUENCE LENGTH:141
SEQUENCE TYPE:nucleic acid
TOPOLOGY:linear
CLONE:HUMGS03883
SEQUENCE DESCRIPTION:
GATCCCTAAG CCCCCCAGCT GTAAATAGGC TGTGGCCAGT GCCTGGTCAT NAGAAGAGNG 60
AGGAGGAGCC CAGGCGTCTG TTTATGTATT TATTTATTTA TTTATTATAC CTATTAATAA 120
AAAAGGTGCT CAGCCTCCAA A 141

SEQ ID NO:3311
SEQUENCE LENGTH:139

SEQUENCE TYPE:nucleic acid
TOPOLOGY:linear
CLONE:HUMGS03884
SEQUENCE DESCRIPTION:
GATCTTGGCC TTCGCAGTGC CCAGTGATGG CATTACTCTG CACTATAGCC ATTTNCCCCA 60
ACTTAAGTTT AGAAATNACA AGTTTCAGTA ATAGCTGAAC CTGTTCAAAA TGTTAATAAA 120
GGTTTCGTTG CATGGTAAA 139

SEQ ID NO:3312
SEQUENCE LENGTH:138
SEQUENCE TYPE:nucleic acid
TOPOLOGY:linear
CLONE:HUMGS03885
SEQUENCE DESCRIPTION:
GATCTGGAAG ACAAGTAACA TCTTCCTTTT NTTGGTGCTC GCCTGCCGTG CTCTAGGTGC 60
TTTTAGAGAT GCTGGTCTGT GTGCTAGGGA AGCGATGAAC CATACTGTTA GTCTATTAAA 120
CATAACTCAA CCTGGAAA 138

SEQ ID NO:3313
SEQUENCE LENGTH:138
SEQUENCE TYPE:nucleic acid
TOPOLOGY:linear
CLONE:HUMGS03886
SEQUENCE DESCRIPTION:
GATCTATCTC TGTGTCCAAG TCTCCTCTTT TTATAAGGAA ACCAGTCATA ATGGACCCAC 60
CTGAATNACC TCATTTTAAC TTGATTACCT CTGTAAAAAA TGTATTTCCA AATAAAGTGA 120
CCTTATGAGG TATTGAAA 138

SEQ ID NO:3314
SEQUENCE LENGTH:136
SEQUENCE TYPE:nucleic acid
TOPOLOGY:linear
CLONE:HUMGS03887
SEQUENCE DESCRIPTION:
GATCAACAAA TCTCCTAGGC CTNTNACCAC ATACATTTAC ACCNACTACC NCAACTATCC 60
ATAAATCTAA GTATAGCCAT TCCACTATNA GCTGGAGCCG TAATTACAGG CTTCCGACAC 120
AAACTAAAAA GCTAAA 136

SEQ ID NO:3315
SEQUENCE LENGTH:139
SEQUENCE TYPE:nucleic acid
TOPOLOGY:linear
CLONE:HUMGS03888
SEQUENCE DESCRIPTION:
GATCCCACCT TCAACACCTT ACAAGTAAAG CCAATGAAGN ACAGTTGAAA CATGCAAAAT 60

ATGGNGCTTT TCATGTAATT ACTCTTTTAC TGTTTACCAT TCACTATAAT TCACAATTAA 120
AATTGTGTGN CTANACAAA 139

SEQ ID NO:3316
SEQUENCE LENGTH:133
SEQUENCE TYPE:nucleic acid
TOPOLOGY:linear
CLONE:HUMGS03889
SEQUENCE DESCRIPTION:
GATCTGTGTA GTTAATGTAT TTATTAATGC TTGACTTTTA AAATCCTGGG CATAAATAGT 60
GCAGAGCCTC GTATGTTTGT CAGTTCATGC CGAGATGAAA TAAATCACGC AGAAAGTGCC 120
AGTCCTCCTG AAA 133

SEQ ID NO:3317
SEQUENCE LENGTH:132
SEQUENCE TYPE:nucleic acid
TOPOLOGY:linear
CLONE:HUMGS03890
SEQUENCE DESCRIPTION:
GATCTGTGGA GTGTGTGTTT CAAAGAGAGA ACTACAGAAA TNTTAAAGCA GGAAAACCTG 60
AATGTNATGT GCACATTTTC ATCCCACATG GACAATGTAT GTGTTTTAAT AAATGGAATT 120
TTCAGATTCA AA 132

SEQ ID NO:3318
SEQUENCE LENGTH:132
SEQUENCE TYPE:nucleic acid
TOPOLOGY:linear
CLONE:HUMGS03891
SEQUENCE DESCRIPTION:
GATCAAATGT GGGCCCGTTT TTGGCCGCCG CCGCCCTGCC CCGTCCCTNC GGCCACCACC 60
TAATTANTTG CCGTGCTTCC TGCTGCTGTA ACTGCTTTNT TACCTTTCCA ATAAAGANTT 120
TCTTGGTTTA AA 132

SEQ ID NO:3319
SEQUENCE LENGTH:132
SEQUENCE TYPE:nucleic acid
TOPOLOGY:linear
CLONE:HUMGS03892
SEQUENCE DESCRIPTION:
GATCATTATT ATTGTAATAG TCTCATGTTT AAATGGGATT ATATAATGAN AACAGTTTAA 60
AGAAAATNAT AATCTTATAT NTCAAATGTG GATGCATATA ACCTGTNAGT GAAAAANCAC 120
TGANTGATTT AN 132

SEQ ID NO:3320
SEQUENCE LENGTH:131

SEQUENCE TYPE:nucleic acid
TOPOLOGY:linear
CLONE:HUMGS03893
SEQUENCE DESCRIPTION:
GATCATATTG CTGTAACTTC TTTTAAGTAG TTGATGTGGA AAACATTTTA AAGTGAATTT 60
NTCAAAATNC TGGTTTTGTG TTTTATCCAA CTTTTGTGCA TATATATAAA GTATGTCATG 120
GCATGGTTAA A 131

SEQ ID NO:3321
SEQUENCE LENGTH:131
SEQUENCE TYPE:nucleic acid
TOPOLOGY:linear
CLONE:HUMGS03894
SEQUENCE DESCRIPTION:
GATCCTCAGT GTAGTTCTGA AACTAAGACT ATAGATATTT NNTTTCTTTT AATTTCTCTT 60
TATACTAAAG AATCCAGAGT TGCTACAATA AAATAAGGGG AATAATAAAC TTGAGAGTGA 120
ATAACCATAA A 131

SEQ ID NO:3322
SEQUENCE LENGTH:131
SEQUENCE TYPE:nucleic acid
TOPOLOGY:linear
CLONE:HUMGS03895
SEQUENCE DESCRIPTION:
GATCTTAGAG TTTGCACAGC TTAAAAAAAT GGAAATATTG GAATCTGAAT CCAAAACAGC 60
CTGACTCAAA ACAATGTNCT TNCCATTATG CAAGGGAAAA AAAAAANGGT TGGGTNTCAG 120
NGNGAGGGTA N 131

SEQ ID NO:3323
SEQUENCE LENGTH:433
SEQUENCE TYPE:nucleic acid
TOPOLOGY:linear
CLONE:HUMGS03896
SEQUENCE DESCRIPTION:
GATCATAGTC TTAGGAGTTC ATTTAAACCA TAGGAACTTT TCACTTATCT CATGTTAGCT 60
GTACCAGTCA GTGATTAAGT AGAACTACAA GTTGTATAGG CTTTATTGTT TATTGCTGGT 120
TTATGACCTT AATAAAGTGT AATTNTGTAT TACCAGCAGG GTGTTTTTAA CTGTGACTAT 180
TGTATAAAAC CAAATCTTGA TATCCAGAAG CACATGANGT TTGCAACTTT CCACCCTGCC 240
CATTTTTGTA AAACTGCAGT CATCTTGGAC CTTTTAAAAC ACAAATTTTA AACTCAACCA 300
AGCTGTGATA AGTGGAATGG TTACTGTTTA TACTGTGGGT ATGTTTTTGN NTTACAGCAG 360
GTTAATGCTT TCTTTTTCCA GGTCGNCCTT TGAGGATTNA GGGAAAATTA NATAACTCTT 420
TGTACAGTTC AAA 433

SEQ ID NO:3324
SEQUENCE LENGTH:129

SEQUENCE TYPE:nucleic acid
TOPOLOGY:linear
CLONE:HUMGS03897
SEQUENCE DESCRIPTION:
GATCCACCCA CCTCGGCCTC CCAAAGTGCT GGGATTATAG GTGNNAGCCA CCGCGCCGGG 60
CCGGTTGCTG GCATCTTAAT GTTCTGTAGG TGGAATATTT CCAATAAACA CAAGGTGCCG 120
TAATTGAAA 129

SEQ ID NO:3325
SEQUENCE LENGTH:129
SEQUENCE TYPE:nucleic acid
TOPOLOGY:linear
CLONE:HUMGS03898
SEQUENCE DESCRIPTION:
GATCTGGTAA ACTTNNAATC AATTAAATTA ATGCANCAAT AGATTATNAA TATTAACTGA 60
CCATTTAAGT TTTTNAATAA GTTTTTTACA AAGAAAAGTT AAACATTAAA AAGAATTACA 120
GCTTTCAAA 129

SEQ ID NO:3326
SEQUENCE LENGTH:128
SEQUENCE TYPE:nucleic acid
TOPOLOGY:linear
CLONE:HUMGS03899
SEQUENCE DESCRIPTION:
GATCTGGAAA TNTTTTAATC AGTTAAATTG TGCAGCAATA GATTTTTAAC TTTAACTGAC 60
CATTTAAGTT TTTNAATAAG TTTTTTACAA AGAAAAGTTA AACATTAAAA AGAATTACAG 120
CTTTCAAA 128

SEQ ID NO:3327
SEQUENCE LENGTH:127
SEQUENCE TYPE:nucleic acid
TOPOLOGY:linear
CLONE:HUMGS03900
SEQUENCE DESCRIPTION:
GATCCTGTAG TGCCTCTATA GAAGTACCCA CAGAAAGTAA AGTATCACAT TTATAAATAC 60
CAAAGATGTA ACAATTTTAA AATTTTNTAG ATTACTCCAA TAAAGTGTTT TAAGTTTTCC 120
TATGAAA 127

SEQ ID NO:3328
SEQUENCE LENGTH:131
SEQUENCE TYPE:nucleic acid
TOPOLOGY:linear
CLONE:HUMGS03901
SEQUENCE DESCRIPTION:
GATCCATCGC GATACTATGT GTGCATTATT TGAAAGTTAT TGGAAATTTT ATTCAAACCG 60

TGGAACAAAT GTATGTNATT TTTTTATACT TCTTAATTTA AATAAAATAT TTAATGCACT 120
ATTAAAATAA A 131

SEQ ID NO:3329
SEQUENCE LENGTH:123
SEQUENCE TYPE:nucleic acid
TOPOLOGY:linear
CLONE:HUMGS03902
SEQUENCE DESCRIPTION:
GATCAGCCCT TTGTACAGAA AAATGTGTCT ATAAAAATTA TGTGTTATTT AATTCTGATA 60
CTTTTTGGCT TGTAAATAGC TTCTTGAACT TTTTACAATA AAAATGTTTT AGAAACTGTT 120
AAA 123

SEQ ID NO:3330
SEQUENCE LENGTH:123
SEQUENCE TYPE:nucleic acid
TOPOLOGY:linear
CLONE:HUMGS03903
SEQUENCE DESCRIPTION:
GATCATGACC GGAACATGGN CTGGGTTTCC ACCCAGCTCC AGGCCACATG CAATGTCCAC 60
AAGTNTGCCT TCAATNACTG GTTCAGTGGA CACCTCAACT TCCAGATTGA GCACCATCTN 120
NNN 123

SEQ ID NO:3331
SEQUENCE LENGTH:205
SEQUENCE TYPE:nucleic acid
TOPOLOGY:linear
CLONE:HUMGS03904
SEQUENCE DESCRIPTION:
GATCGATTCA ATGCCCGATG CTTCTGTTTC ATTCCCGACC CTTTCTACTA TGCATTTCCC 60
TTTTATCAGG TGTATAAAGT TAAATACTGT GTATTTATCA CTAAAAAGTA CATGAACTTA 120
AGNGNAAACT AAGCCTTTCG TGTTTTTCCA NAGGNCNGCN NGGCTTCTNT GTACAGTTGG 180
AATAAACAGA CAGCAAAATG GTAAA 205

SEQ ID NO:3332
SEQUENCE LENGTH:122
SEQUENCE TYPE:nucleic acid
TOPOLOGY:linear
CLONE:HUMGS03905
SEQUENCE DESCRIPTION:
GATCCTCAGG CGGCCCCCAC CAGGGCACAC CCTACTGTCC TTGTGCCTCA CGCCCCCTCC 60
TCATCCTGCA CCCCTTCCAT CCCACCTTCC CTTTCAATAA ACAGCTGGGA TGGATACTGA 120
AA 122

SEQ ID NO:3333

SEQUENCE LENGTH:120
SEQUENCE TYPE:nucleic acid
TOPOLOGY:linear
CLONE:HUMGS03906
SEQUENCE DESCRIPTION:
GATCTGATTT TTTCCCCCTT TACCTAGCTG TGCCCCCTCT GGTTATNTAT TTCCTTAGTG 60
CCAGGAGGGC ACAGCAGGGG AGCCCTGATT TTAAATAAAT CCGGAATTGT ATTTATTAAA 120

SEQ ID NO:3334
SEQUENCE LENGTH:119
SEQUENCE TYPE:nucleic acid
TOPOLOGY:linear
CLONE:HUMGS03907
SEQUENCE DESCRIPTION:
GATCACCAGT GCATCCTTCA TCACAGCATG TGCAATATGC CAAGATTACC CTCGGTCATT 60
CCTGTCAACA AGGGGTCAAT GTCATAAATG TCACAATAAA ACAATCTCTT CTTTTTAAA 119

SEQ ID NO:3335
SEQUENCE LENGTH:119
SEQUENCE TYPE:nucleic acid
TOPOLOGY:linear
CLONE:HUMGS03908
SEQUENCE DESCRIPTION:
GATCCATGAG GAAAAGATGC TCTCCCACCT AAGGCCAGGA ATCTGAGAGC AGGACTGGCT 60
GAGCTCCCAG GGCAAGGGGT TCACTAATGC TTATCAATAA AGAATATTGA GCCTGGAAA 119

SEQ ID NO:3336
SEQUENCE LENGTH:273
SEQUENCE TYPE:nucleic acid
TOPOLOGY:linear
CLONE:HUMGS03909
SEQUENCE DESCRIPTION:
GATCAGATTG CATGTNACTC TNAAGCTGAC GAACTTCCAT GGCCCTCATT AATGACACTT 60
GTCCCCAAAT CCGAACCTCC TCTGTNAAGC ATTCGAGACA GAACCTTGTT ATTTCTCAGA 120
CTTTGGAAAA TGCATTGTAT CGATGTTATG TAAAAGGCCA AACCTCTGTT CAGTGNNNNT 180
AGTTACTCCA GTGCCAACAA TCCTAGTGCT TTCCTTTTTN AAAAATGCAA ATCCTATGTG 240
NTTTTAACTC TGTCTNCACC TGATTCAACT AAA 273

SEQ ID NO:3337
SEQUENCE LENGTH:119
SEQUENCE TYPE:nucleic acid
TOPOLOGY:linear
CLONE:HUMGS03910
SEQUENCE DESCRIPTION:
GATCACCATG AACAAAGNTA TATTTCCTAT TTATTTATTA TATGTGCACT TCAAGAAGTC 60

ACTGTCAGAG AAATAAAGNN TTGTCTAAAT GTCATGTTGG AGATNCCTTT GCATNCTTN 119

SEQ ID NO:3338
SEQUENCE LENGTH:118
SEQUENCE TYPE:nucleic acid
TOPOLOGY:linear
CLONE:HUMGS03911
SEQUENCE DESCRIPTION:
GATCAGGACT TCAAGGGCCA GACCACAGAC TACTTATAGG AGCATCTTCA AGGCGGTCAC 60
AGTGAAAACC TTGAAATGGC CTTTTTNAAT ATATATAANT AANTGTNAAT ATTATTNN 118

SEQ ID NO:3339
SEQUENCE LENGTH:118
SEQUENCE TYPE:nucleic acid
TOPOLOGY:linear
CLONE:HUMGS03912
SEQUENCE DESCRIPTION:
GATCCAAATN ATGGCCACAG ATGAACTTCC ATGCAAGCNA TGGCTGAGTT TCATTGCTTA 60
AAATNCTCCT CTGTTTAGAG GGGCCTGGTA GAAATAAAAA CCCACCCTAT ATCTTAAA 118

SEQ ID NO:3340
SEQUENCE LENGTH:118
SEQUENCE TYPE:nucleic acid
TOPOLOGY:linear
CLONE:HUMGS03913
SEQUENCE DESCRIPTION:
GATCCTCCTG GCCGCCCCGC AGGTCTNTGG AAGGGCTGGA CGGCAAGTCC GTNTAGCTCA 60
CGGGCCCCTC CAGTGGAATG GNTCTTTCCG GTGGAGATAA AAGTTGATTT NCTCTAAA 118

SEQ ID NO:3341
SEQUENCE LENGTH:351
SEQUENCE TYPE:nucleic acid
TOPOLOGY:linear
CLONE:HUMGS03914
SEQUENCE DESCRIPTION:
GATCCCAGGT GCGTGTNTCT CTGTCTCTCC TAGCCCCTCT CTCACACATC ACATTCCCAT 60
GGTGGCCTCA AGAAAGGCCC GGAAGCGCCA GGCTGGAGAT ANCAGNCTCT TNCCCGTCGG 120
CCCTGCGTCG GCCCTGGGGT ACCATGTGGC CACAACTGCT GTGGCCCCCT GTCCNNAAGA 180
CACTTCCCCT TGTNTCCCTG GTTGCCTCTC TTGCCCCTTG TCCTGAAGCC CAGCGACACA 240
GAAGGGNGNG GGGCGGGTCT ATGGGGGAGA ANGGGAGCGA GNTCAAGAGG TGGGCATGGG 300
TTTGGNAAGG GTNGGCGTTT GGGGGGCCTN TNATGNTGGC TTTTTCAANC N 351

SEQ ID NO:3342
SEQUENCE LENGTH:117
SEQUENCE TYPE:nucleic acid

TOPOLOGY:linear
CLONE:HUMGS03915
SEQUENCE DESCRIPTION:
GATCATGCCG CTGCACTCCA ACCTGGGCAG CAGTGCAAGA CTCTGTCTCA AAAGGGGAAA 60
AAAAAAAANT TNCTGATGTG NCCCATGAAG GGACCTCATT TNCCTCGNAA TTTTGGN 117

SEQ ID NO:3343
SEQUENCE LENGTH:116
SEQUENCE TYPE:nucleic acid
TOPOLOGY:linear
CLONE:HUMGS03916
SEQUENCE DESCRIPTION:
GATCGGCCGT ACCAGCCGCC CCGCCCGCCG GTGGGCGCTC TCGGACTACT CGCTGTACTT 60
GCCGCTCTGA GTCAGTGGCC CCTTCGTTCC TTGTAAATAA ATCCCGCCCC CGGAAA 116

SEQ ID NO:3344
SEQUENCE LENGTH:116
SEQUENCE TYPE:nucleic acid
TOPOLOGY:linear
CLONE:HUMGS03917
SEQUENCE DESCRIPTION:
GATCCAGACA AGTGGGAGAG CCCGTGGGGG CAGGGGACCT GGAGCTGCCA GCACCAAGCG 60
TGATTCCTGC TGCCTGTATT CTCTATTCCA ATAAAGCAGA GTTTGACACC GTCAAA 116

SEQ ID NO:3345
SEQUENCE LENGTH:116
SEQUENCE TYPE:nucleic acid
TOPOLOGY:linear
CLONE:HUMGS03918
SEQUENCE DESCRIPTION:
GATCTCATAA GAAATGTAAT ATGTAAATAA TATCTTGCTT TATGTTGTAA AATATACATT 60
GTTTGCNCTA GAATAGAAAT AATTTCTTTT CAATAAAAAG AAAGAAGGAC TCTAAA 116

SEQ ID NO:3346
SEQUENCE LENGTH:116
SEQUENCE TYPE:nucleic acid
TOPOLOGY:linear
CLONE:HUMGS03919
SEQUENCE DESCRIPTION:
GATCGTTGTG TTGTTATTTT ACTGCACTTT TTACTTTTTT NCGTGTGGAG CTGTATTCCC 60
GAGACCAACG AAGCGTTGGG ATACTTCATT AAATGTAGCG ACTGTCAACA GCGAAA 116

SEQ ID NO:3347
SEQUENCE LENGTH:116
SEQUENCE TYPE:nucleic acid

TOPOLOGY:linear
CLONE:HUMGS03920
SEQUENCE DESCRIPTION:
GATCAAGGCC AAATAACCCG ATTTGGTTTG GGCACTCAAG CAATAGTAAA GCCTGTAAGA 60
CATGATAGGA TTATNTTCCC CTTTGGATTT AAATNTAATA TAGCAGTTGG ATTAAA 116

SEQ ID NO:3348
SEQUENCE LENGTH:115
SEQUENCE TYPE:nucleic acid
TOPOLOGY:linear
CLONE:HUMGS03921
SEQUENCE DESCRIPTION:
GATCCTGTAG ACCCTTTCTT TTGGCCAATT TCCCCCTTTT TGGGATGGAA ACATTTACCC 60
AATCCCTATA CCCCCATTGT ATCTTGGAAG TAAATAATTT GTTTTTGATT TTAAA 115

SEQ ID NO:3349
SEQUENCE LENGTH:115
SEQUENCE TYPE:nucleic acid
TOPOLOGY:linear
CLONE:HUMGS03922
SEQUENCE DESCRIPTION:
GATCTCTTCA CTGTNAAAAG TTGCCCGGGT GCAGCGCCTT TCCCTTCTAN CATGGGAAAT 60
GCAGGCTGGG CCCTTGGGNT NAGCCTGCGG GGCTCTGGTG CTGTCCCCGA CCCNN 115

SEQ ID NO:3350
SEQUENCE LENGTH:113
SEQUENCE TYPE:nucleic acid
TOPOLOGY:linear
CLONE:HUMGS03923
SEQUENCE DESCRIPTION:
GATCCACTGT TTGCAGCAGA ATTATATATA TGTATAGGAA AAATCCACTT TGAATAATCC 60
ATGTTTTGTA TTTGGAAATT GTTTTTAAAA ATAAAAAGGA AAGGAAATAT AAA 113

SEQ ID NO:3351
SEQUENCE LENGTH:113
SEQUENCE TYPE:nucleic acid
TOPOLOGY:linear
CLONE:HUMGS03924
SEQUENCE DESCRIPTION:
GATCTAGGCA GTGGGTTAAG CCAGACCCCT AAAAGTGACC CTTTCCTCTT CTGCCCTATT 60
CTNCTNAGAC CCTCAGAAAG NNNTCCAATT TTTTAAAATC CCTGTAGCTC CAN 113

SEQ ID NO:3352
SEQUENCE LENGTH:117
SEQUENCE TYPE:nucleic acid

TOPOLOGY:linear
CLONE:HUMGS03925
SEQUENCE DESCRIPTION:
GATCTGATGA ATTGGTTTTT GAATCCCAGA AANGGTCTGC CATGGAGTTG GCAGTCATCA 60
CGGTAGATGG CGTATGATTT TCCTGAATTT TAAATAAAAT GAAAACCATA AATTAAA 117

SEQ ID NO:3353
SEQUENCE LENGTH:112
SEQUENCE TYPE:nucleic acid
TOPOLOGY:linear
CLONE:HUMGS03926
SEQUENCE DESCRIPTION:
GATCAGAGCT GTTAGGTAAA GGCCTAACCA CCTCCCTGCA GNCTCTTAAN TCTCAAGCTT 60
TAGGAACCCA TTTCTAAANG TACACTAGCG GAGANTTTAT ATTGTCAGCC NN 112

SEQ ID NO:3354
SEQUENCE LENGTH:111
SEQUENCE TYPE:nucleic acid
TOPOLOGY:linear
CLONE:HUMGS03927
SEQUENCE DESCRIPTION:
GATCACGGTA CGTCCATAAA GCCAGTATTA CACTTAAATN NAGTATTCTT TTTGTAATC 60
GTTTTTTTTA GAAGGTAAAC AAATTTAATA AAGCTACCAA TAATGTTGAA A 111

SEQ ID NO:3355
SEQUENCE LENGTH:111
SEQUENCE TYPE:nucleic acid
TOPOLOGY:linear
CLONE:HUMGS03928
SEQUENCE DESCRIPTION:
GATCTAGAAA NTGCCCTCCT TATACCCCTA CCATGAGCCC TACAAACAAA AAACCTGCCA 60
CTAATAGTAT GGTCATCCCT CTNANTAAAN CATCATCCTG GCCCTAAGGN N 111

SEQ ID NO:3356
SEQUENCE LENGTH:111
SEQUENCE TYPE:nucleic acid
TOPOLOGY:linear
CLONE:HUMGS03929
SEQUENCE DESCRIPTION:
GATCTTTGCA ATTNAAGGNT TAGCCACGTG GCTTGCTTCA GCNAGTAATG TGAATGGAAA 60
TAACGTGTAT CANTGCTGGA TAGAAAACTT AANAGCCAAC ACATGGTTCC N 111

SEQ ID NO:3357
SEQUENCE LENGTH:110
SEQUENCE TYPE:nucleic acid

TOPOLOGY:linear
CLONE:HUMGS03930
SEQUENCE DESCRIPTION:
GATCCCGTAG AATATATATT TAATAATCCC ACAAACGGAG GCCAGACTTC TGCGTTAACT 60
TCAGTAACAC AAGCTTCTTT AAGCCAAATA CATCACTTGC CACTACCAAA 110

SEQ ID NO:3358
SEQUENCE LENGTH:110
SEQUENCE TYPE:nucleic acid
TOPOLOGY:linear
CLONE:HUMGS03931
SEQUENCE DESCRIPTION:
GATCTCCGTG GTGGTCGTGG GCCGCCAGGT GAATNANCCC CACATCCGCN TCCTGGTCAC 60
CGGCAAGACG GCCGAGTACG ACGTGGCCTA CGGCGAGAGC ACCTNTNCCN 110

SEQ ID NO:3359
SEQUENCE LENGTH:115
SEQUENCE TYPE:nucleic acid
TOPOLOGY:linear
CLONE:HUMGS03932
SEQUENCE DESCRIPTION:
GATCCTTCGA AGCACTCAGT TCACTCTTCC CCACCACAGT GGTTAAAAGG CGTTTGTATC 60
TGCCACTATG TGTGTGTTTT AAAATAANCT TTTGGAAACA TGTTTGGNGN GNAAA 115

SEQ ID NO:3360
SEQUENCE LENGTH:214
SEQUENCE TYPE:nucleic acid
TOPOLOGY:linear
CLONE:HUMGS03933
SEQUENCE DESCRIPTION:
GATCCACTAA TGTAGCTTAA GGTTATTAGN TTTGGGCTTT TAATCATGGN ATAATCTTAT 60
GTATTGGTGT AAGAGTTGAT GAATGACTTT AGCTGTGTGA ATATATAATA GTCAAACTGC 120
AAACATTTTG CATCCCTTTT GTGACCTAAT TTACAGNCAT TTAAATTGTG TTGCAGTTCT 180
GCTTTGCCGT TTAATAAAAA GCTATTTCAG NAAA 214

SEQ ID NO:3361
SEQUENCE LENGTH:110
SEQUENCE TYPE:nucleic acid
TOPOLOGY:linear
CLONE:HUMGS03934
SEQUENCE DESCRIPTION:
GATCANAGAT TTCAATNACA GTGCAGGAAA GCAGCACTGA GAAACTTTTA ATTGTNTTNA 60
ATTTCTNCTT TAATGTTCAC ACCACCACAT TCCCATTCAC TTTAATTTTN 110

SEQ ID NO:3362

SEQUENCE LENGTH:108
SEQUENCE TYPE:nucleic acid
TOPOLOGY:linear
CLONE:HUMGS03935
SEQUENCE DESCRIPTION:
GATCTTCCCC TGACCTGCTC CCAGGGCTAT AACAGATACG GACTTTGAAA CTGTATGGAG 60
TATAGAGCAG TGCTCTATAA TTTAAAATAT ACATATATTG CAACCAAA 108

SEQ ID NO:3363
SEQUENCE LENGTH:363
SEQUENCE TYPE:nucleic acid
TOPOLOGY:linear
CLONE:HUMGS03936
SEQUENCE DESCRIPTION:
GATCTTCAGC TTGGTAGATT TAATTGCCCA GTGAATGGCA CTTACGTTTT CATTTTNNAC 60
ATGCTAAAGC TGGCAGTGAA TGTNCCACTG TATGTCAACC TCATGAAGAA TGAAGAGGTC 120
TTGGTATCAG CCTATGCCAA TNATGGTGCT CCAGACCATG AAACTGCTAG CAATCATGCA 180
ATTCTTCAGC TCTTCCAGGG AGGCCAGATA TGGTTACGNC TGCACAGGGG AGCAATTTNT 240
GGAAGTAGCT GGGAAATATT CTACGGTTTC AGGCTATCTN CTTTTATCAA GGTTTGAATG 300
TCAGTNCAGT NTTTGACNAC TAAAAGGNNT NGGGGTNCTA ATTANGTNGG GTTTGAAGGG 360
TAN 363

SEQ ID NO:3364
SEQUENCE LENGTH:106
SEQUENCE TYPE:nucleic acid
TOPOLOGY:linear
CLONE:HUMGS03937
SEQUENCE DESCRIPTION:
GATCTGGGGA GTNTNAGGAG AGGGTGGCAT CAGNAGCTGC TCAGGCTTGG CGGAGGGAGC 60
GGCATGGCCG ATGTCACTCA GCCCCTTCCC GGTCCGCCCN NTTCCN 106

SEQ ID NO:3365
SEQUENCE LENGTH:105
SEQUENCE TYPE:nucleic acid
TOPOLOGY:linear
CLONE:HUMGS03938
SEQUENCE DESCRIPTION:
GATCGTTTTT TCCAGAGTCG CGGGGACACA GGAGTNTTCC TACAGTACAT NCACGCCCGC 60
NTCCACAGTT TGGAAGAGAC TTTTGGATGT GGGTACNTGA NTGAN 105

SEQ ID NO:3366
SEQUENCE LENGTH:105
SEQUENCE TYPE:nucleic acid
TOPOLOGY:linear
CLONE:HUMGS03939

SEQUENCE DESCRIPTION:
GATCATCTAA AAAATGCCAT GANTGGAAAT NCGAAGGCCC AGACACCAAT ATTTCNTAGA 60
AGTAAACAGC TCAAAGACAC TCTCCTATCT NAGGAAATNA ATGTN 105

SEQ ID NO:3367
SEQUENCE LENGTH:104
SEQUENCE TYPE:nucleic acid
TOPOLOGY:linear
CLONE:HUMGS03940
SEQUENCE DESCRIPTION:
GATCCAGTGG GGTCCGGACA CTGGGCCCCG CAGCGAAAGC ACGNTCCAGC CACCAGGAGG 60
CCACCTATTN TTTCAAAATA AAGACTGCGT TCCTCTCTTG GAAA 104

SEQ ID NO:3368
SEQUENCE LENGTH:104
SEQUENCE TYPE:nucleic acid
TOPOLOGY:linear
CLONE:HUMGS03941
SEQUENCE DESCRIPTION:
GATCAAAAAA GAAGAGTTAA AACAACACAT GTAAATNCCT TTTAATATTT NATGGGAATG 60
CCTCTCATTT AAAAATAGAA ATAAAGCATT TTGTTAAAAA GAAA 104

SEQ ID NO:3369
SEQUENCE LENGTH:104
SEQUENCE TYPE:nucleic acid
TOPOLOGY:linear
CLONE:HUMGS03942
SEQUENCE DESCRIPTION:
GATCCTTTAT CTTTCCATAT GGCACACGTA AGAAAGTGTT TTTCTACTAT TAATATTAAA 60
TTAAAACCTT TACTTNTGTA TAATAAATNA AAACTCAGAA TAAA 104

SEQ ID NO:3370
SEQUENCE LENGTH:104
SEQUENCE TYPE:nucleic acid
TOPOLOGY:linear
CLONE:HUMGS03943
SEQUENCE DESCRIPTION:
GATCTGGAAG AGGCTTGTAA CCTGAACTAN TATGTAGGAA GGCAGTACNA NCAGCCCACC 60
AGTGTAATCT CCCTGNATTA AGGCATTCTT AAAAACTTAG GCNN 104

SEQ ID NO:3371
SEQUENCE LENGTH:104
SEQUENCE TYPE:nucleic acid
TOPOLOGY:linear
CLONE:HUMGS03944

SEQUENCE DESCRIPTION:
GATCCGCTCT GGTCAAACCC TTCCAGGCCA GCCAGAGTGG GGATGGTCNN TNACCTGCTG 60
GGAAGGCANN CTGATGGGGC ACACCCTTGG CCTCTCGTCC ACGN 104

SEQ ID NO:3372
SEQUENCE LENGTH:102
SEQUENCE TYPE:nucleic acid
TOPOLOGY:linear
CLONE:HUMGS03945
SEQUENCE DESCRIPTION:
GATCAAAATT TNTTGATAAT TTAAAAATTG TGTGTGATTT TNAAATTTNA AGAAATTGCT 60
GAAAACTTAG AAAAATATAA TGGTATACAT ACAATAGNNA AA 102

SEQ ID NO:3373
SEQUENCE LENGTH:105
SEQUENCE TYPE:nucleic acid
TOPOLOGY:linear
CLONE:HUMGS03946
SEQUENCE DESCRIPTION:
GATCAATAAT TTAAATGTAA CTAGTTGGGA TTTTATAGTT AAAATNATAT TTGTGTATAT 60
AACATAACTA ATCTGTAAAT NGTAATAAAT ATATTTNCAA TTAAA 105

SEQ ID NO:3374
SEQUENCE LENGTH:99
SEQUENCE TYPE:nucleic acid
TOPOLOGY:linear
CLONE:HUMGS03947
SEQUENCE DESCRIPTION:
GATCATTTAG TACTTTTGTN TTCTCCCATG TGCTTGAAGG AAAAATAAAG TGTCACTACC 60
GTATTTNTTG TTTTCATCAA AAAATAAAAA TAATTTAAA 99

SEQ ID NO:3375
SEQUENCE LENGTH:97
SEQUENCE TYPE:nucleic acid
TOPOLOGY:linear
CLONE:HUMGS03948
SEQUENCE DESCRIPTION:
GATCTTTAGC ACAGTGATAT NACCATGACT TCACAGACAT GGTCTAGAAT CTGTACCCTT 60
ACCCACATAT GAAGAATAAA ATTGATTAAA GGTTAAA 97

SEQ ID NO:3376
SEQUENCE LENGTH:97
SEQUENCE TYPE:nucleic acid
TOPOLOGY:linear
CLONE:HUMGS03949

SEQUENCE DESCRIPTION:
GATCAATAAT AATNAGGAAA GCATGATATG TATATTGCTG AGTTGAAAGC ACTTATTGGA 60
AAANATTAAA AGGCTAACAT TAAAAGACTA AAGGAAA 97

SEQ ID NO:3377
SEQUENCE LENGTH:94
SEQUENCE TYPE:nucleic acid
TOPOLOGY:linear
CLONE:HUMGS03951
SEQUENCE DESCRIPTION:
GATCCCCCTC CTTCCCCCTC TCCCCATTGT ATTTATTTGC CTGCTGGAAA ATNACATCCG 60
GAAATAAAAT AGAAATATGT CTTTTNATTT TAAA 94

SEQ ID NO:3378
SEQUENCE LENGTH:93
SEQUENCE TYPE:nucleic acid
TOPOLOGY:linear
CLONE:HUMGS03952
SEQUENCE DESCRIPTION:
GATCCTAAAC TTTTTGGATA ATCTTTTATA TTTNTAACCT TTGAATTTAA TCATTGTNCT 60
NAGATTAAAA TAAAANATGC TATTGAAACT AAA 93

SEQ ID NO:3379
SEQUENCE LENGTH:91
SEQUENCE TYPE:nucleic acid
TOPOLOGY:linear
CLONE:HUMGS03954
SEQUENCE DESCRIPTION:
GATCTGGAAA TCCCTGCTGT AATCTTTTTA AGGGAATCCA CTGGAAATAA TTAGTTTTTG 60
TTTTAAATAA AACCATTCCT TACTTAACAA A 91

SEQ ID NO:3380
SEQUENCE LENGTH:91
SEQUENCE TYPE:nucleic acid
TOPOLOGY:linear
CLONE:HUMGS03955
SEQUENCE DESCRIPTION:
GATCTATGGC CTCTGGTGCT TTGTCCTGTA TTTGGTTTAA TNTTTTTGTC CTAATCTCTT 60
CAATCAATAA AATTGTGCGT ATTTAACTAA A 91

SEQ ID NO:3381
SEQUENCE LENGTH:91
SEQUENCE TYPE:nucleic acid
TOPOLOGY:linear
CLONE:HUMGS03956

SEQUENCE DESCRIPTION:
GATCAACTNA ATGTTTAAGA CTTTAGATGT CTTGTATTAA AAATTACACA AAAAAAGTAA 60
AACTTTTTAT ACTTACCCTT TTAACTCTAA A 91

SEQ ID NO:3382
SEQUENCE LENGTH:90
SEQUENCE TYPE:nucleic acid
TOPOLOGY:linear
CLONE:HUMGS03958
SEQUENCE DESCRIPTION:
GATCTCTGTA ATNTTATNTA GAGTTTGAGC TGAAGCCCCG TATATCTAAT NTATTTTGTT 60
AAACATGAAA GTGCATCCTT TCCCTCCAAN 90

SEQ ID NO:3383
SEQUENCE LENGTH:88
SEQUENCE TYPE:nucleic acid
TOPOLOGY:linear
CLONE:HUMGS03959
SEQUENCE DESCRIPTION:
GATCATACCT ATTAAAATAA TGCCAAACAC CAAATATGAA TTTNATGATG TACACTTTGT 60
GCTTGGCATT AAAAGAAAAA AACACAAA 88

SEQ ID NO:3384
SEQUENCE LENGTH:88
SEQUENCE TYPE:nucleic acid
TOPOLOGY:linear
CLONE:HUMGS03960
SEQUENCE DESCRIPTION:
GATCAAGAAG ACGNNTAAGG GAAGAACGTA CACAGTGAAA ANCCAAAAAA AGAAAAAAAT 60
GTTTATACAA CCCTAAGTCA ATAACCTN 88

SEQ ID NO:3385
SEQUENCE LENGTH:87
SEQUENCE TYPE:nucleic acid
TOPOLOGY:linear
CLONE:HUMGS03961
SEQUENCE DESCRIPTION:
GATCTAGGCA CACCTNGCTC TTNGCTGCAT GTGGTTATNA ACANCTTCCA GTGGAAGTCG 60
AATAAACAGT TTTTGGTAAA TCTCAAA 87

SEQ ID NO:3386
SEQUENCE LENGTH:85
SEQUENCE TYPE:nucleic acid
TOPOLOGY:linear
CLONE:HUMGS03962

SEQUENCE DESCRIPTION:
GATCCTGCCC TGAATCTCCA TAGTCTCCAC TGTGAACTGA GGAGGGGAGG GGTGTGCTGG 60
GGAATAAATC TTGTATGAGA ACAAA 85

SEQ ID NO:3387
SEQUENCE LENGTH:84
SEQUENCE TYPE:nucleic acid
TOPOLOGY:linear
CLONE:HUMGS03963
SEQUENCE DESCRIPTION:
GATCCATTTA NAGGCCAAAT NTCATTCTGC AGGTGCCTTC CCGATGGATT AAAGGTGCTT 60
ATNTTTTTTT AAGTTTNAAG TAAA 84

SEQ ID NO:3388
SEQUENCE LENGTH:81
SEQUENCE TYPE:nucleic acid
TOPOLOGY:linear
CLONE:HUMGS03964
SEQUENCE DESCRIPTION:
GATCGTGTTC ATTTTGNTTT CCGATTCTAG ATATAAGTTT TGNATGGGCA GGAAGACTAA 60
AATAAAAGTT TTTAAGGTAA A 81

SEQ ID NO:3389
SEQUENCE LENGTH:81
SEQUENCE TYPE:nucleic acid
TOPOLOGY:linear
CLONE:HUMGS03965
SEQUENCE DESCRIPTION:
GATCTCCAGA TGTAATTTCC CCTCTTTAAT TTAAANTAAT NTAAAGATTA CTAAAACAAT 60
AAAANCTCCT AAAAANTCAA A 81

SEQ ID NO:3390
SEQUENCE LENGTH:88
SEQUENCE TYPE:nucleic acid
TOPOLOGY:linear
CLONE:HUMGS03966
SEQUENCE DESCRIPTION:
GATCTTTCTT TCCAAAACAT TTCTGGACAG TACCTGATTG TATTTTTTTT TTTAAAATAA 60
AAGCACAAGT ACTTTNGNGN TTGTTAAA 88

SEQ ID NO:3391
SEQUENCE LENGTH:76
SEQUENCE TYPE:nucleic acid
TOPOLOGY:linear
CLONE:HUMGS03967

SEQUENCE DESCRIPTION:
GATCCCCCCA CAGCCCCTGG TCAGTCTGCC CTTGTCACTG GTCTGAGGTC ATTAAAATTA 60
CATTGAGGTT CCTAAA 76

SEQ ID NO:3392
SEQUENCE LENGTH:78
SEQUENCE TYPE:nucleic acid
TOPOLOGY:linear
CLONE:HUMGS03968
SEQUENCE DESCRIPTION:
GATCACAATC ATTTTGAATC CCTCTTCCTC ACTTTTTTTT CTAAGAAAAT AAACATTTTA 60
CTGTTTTTAT GGAATAAA 78

SEQ ID NO:3393
SEQUENCE LENGTH:73
SEQUENCE TYPE:nucleic acid
TOPOLOGY:linear
CLONE:HUMGS03969
SEQUENCE DESCRIPTION:
GATCACAGCT GACATTTACA AAATATTTTT NTACCTTAGA ATTTCTGCAT TAAATAAAAT 60
ATTTTGTTTT AAA 73

SEQ ID NO:3394
SEQUENCE LENGTH:68
SEQUENCE TYPE:nucleic acid
TOPOLOGY:linear
CLONE:HUMGS03971
SEQUENCE DESCRIPTION:
GATCTTTTGC TAATGCAATT AGNATTATGT TTTGCATGTA TGACTTAATA AATCCTTGAA 60
TCATAAAA 68

SEQ ID NO:3395
SEQUENCE LENGTH:67
SEQUENCE TYPE:nucleic acid
TOPOLOGY:linear
CLONE:HUMGS03972
SEQUENCE DESCRIPTION:
GATCCTCTAA ATNTGGAATT TTGATGTAAT AACTGCTAAT AAAATNATAT TGAAACTGTT 60
TTATAAA 67

SEQ ID NO:3396
SEQUENCE LENGTH:65
SEQUENCE TYPE:nucleic acid
TOPOLOGY:linear
CLONE:HUMGS03973

SEQUENCE DESCRIPTION:
GATCAAAGCC CGGAAAGGCA AATGAAGCNT CATGTGTAGT GTAATAAAGN TGTCTGCTGT 60
TCAAA 65

SEQ ID NO:3397
SEQUENCE LENGTH:65
SEQUENCE TYPE:nucleic acid
TOPOLOGY:linear
CLONE:HUMGS03974
SEQUENCE DESCRIPTION:
GATCGAAGCC TGTGTAAAAT GCTACCAAAT GGCAAAAAGC AACAATAAAC AGTTTGATTT 60
TTAAA 65

SEQ ID NO:3398
SEQUENCE LENGTH:64
SEQUENCE TYPE:nucleic acid
TOPOLOGY:linear
CLONE:HUMGS03975
SEQUENCE DESCRIPTION:
GATCCCCAAT ATATCTACCA TTGTATGTTA AATAAATNAC CATTTTTGTA GAAAAAATTC 60
TAAA 64

SEQ ID NO:3399
SEQUENCE LENGTH:71
SEQUENCE TYPE:nucleic acid
TOPOLOGY:linear
CLONE:HUMGS03976
SEQUENCE DESCRIPTION:
GATCTGTAAA TNACTTTAAA AGCTATTTTA GTAATTTAAA TAAATTTACT TTCTTGGTTT 60
ATACTCTCAA A 71

SEQ ID NO:3400
SEQUENCE LENGTH:63
SEQUENCE TYPE:nucleic acid
TOPOLOGY:linear
CLONE:HUMGS03977
SEQUENCE DESCRIPTION:
GATCTCTATA NTTTTTTTTA AATTTTCTCC AGCCTGGGTG ACAGAGCGAN ACTCCGTCTC 60
AAA 63

SEQ ID NO:3401
SEQUENCE LENGTH:63
SEQUENCE TYPE:nucleic acid
TOPOLOGY:linear
CLONE:HUMGS03978

SEQUENCE DESCRIPTION:
GATCTCCTGC TGCCTTTCCT GGAGTTTGTA AAATTNTNCC TGAATACAAG CCTATGCGTG 60
AAA 63

SEQ ID NO:3402
SEQUENCE LENGTH:62
SEQUENCE TYPE:nucleic acid
TOPOLOGY:linear
CLONE:HUMGS03979
SEQUENCE DESCRIPTION:
GATCCAAGCA TAGTAGAAAA GCTTCATGTG TTTTAAAAAA TAAACAATAA CAAAAAATTA 60
AA 62

SEQ ID NO:3403
SEQUENCE LENGTH:61
SEQUENCE TYPE:nucleic acid
TOPOLOGY:linear
CLONE:HUMGS03980
SEQUENCE DESCRIPTION:
GATCATGTGT TCTGGAGAGT GTTCTTTATT CAATAAAGTT TTAATTTAGT ATAAACATAA 60
A 61

SEQ ID NO:3404
SEQUENCE LENGTH:61
SEQUENCE TYPE:nucleic acid
TOPOLOGY:linear
CLONE:HUMGS03981
SEQUENCE DESCRIPTION:
GATCTAATTT TTCAAACCTT CACTATTTTG AAAATATAAC CAAGGCCGGT TGCGGTGGAA 60
A 61

SEQ ID NO:3405
SEQUENCE LENGTH:60
SEQUENCE TYPE:nucleic acid
TOPOLOGY:linear
CLONE:HUMGS03982
SEQUENCE DESCRIPTION:
GATCATGAAA CTGATTGTAA AGCTTTTTGA CAACTAATAA ATGTCTTGGT AATTGCTAAA 60

SEQ ID NO:3406
SEQUENCE LENGTH:59
SEQUENCE TYPE:nucleic acid
TOPOLOGY:linear
CLONE:HUMGS03984
SEQUENCE DESCRIPTION:

GATCAATGGA TTTGTTTACA ATGTGATATT TTCTATTAAA TCCAGTATTT TCAAATAAA 59

SEQ ID NO:3407
SEQUENCE LENGTH:58
SEQUENCE TYPE:nucleic acid
TOPOLOGY:linear
CLONE:HUMGS03985
SEQUENCE DESCRIPTION:
GATCTTGTAC TAACTTATGA TAGAATGTAT CAGAATAAAT ATTTTTAACA GTGTTAAA 58

SEQ ID NO:3408
SEQUENCE LENGTH:53
SEQUENCE TYPE:nucleic acid
TOPOLOGY:linear
CLONE:HUMGS03986
SEQUENCE DESCRIPTION:
GATCATNAAA GTATACAAAA TAAATAAAGT AGGAGATTTT TTTGCTGTTT AAA 53

SEQ ID NO:3409
SEQUENCE LENGTH:121
SEQUENCE TYPE:nucleic acid
TOPOLOGY:linear
CLONE:HUMGS03987
SEQUENCE DESCRIPTION:
GATCTGTTTA GTGAGATTTA GCATGTGTGA ATAAAGTATA TGCAGGAGGA AATTGCTTTG 60
TNTTCCCAAT CGGTAGAAAT TCGGGACCAT AAAAATTGTG TTTTACCATG TGGCCTACAA 120
A 121

SEQ ID NO:3410
SEQUENCE LENGTH:51
SEQUENCE TYPE:nucleic acid
TOPOLOGY:linear
CLONE:HUMGS03988
SEQUENCE DESCRIPTION:
GATCATTGTT AAAGGATTGC TGCAAATAAA TACACTTTAA TNTCAGTCAA A 51

SEQ ID NO:3411
SEQUENCE LENGTH:60
SEQUENCE TYPE:nucleic acid
TOPOLOGY:linear
CLONE:HUMGS03989
SEQUENCE DESCRIPTION:
GATCTTTAAA ACATTTTTAA TGAACTAAGT TGAATAAAGG CACAATTAAA AACTGTCAAA 60

SEQ ID NO:3412

SEQUENCE LENGTH:53
SEQUENCE TYPE:nucleic acid
TOPOLOGY:linear
CLONE:HUMGS03993
SEQUENCE DESCRIPTION:
GATCAAACAG AAGCAGCCGT GGGCAAAATA CAATTTCATT TAACAAATTG AAA 53

SEQ ID NO:3413
SEQUENCE LENGTH:521
SEQUENCE TYPE:nucleic acid
TOPOLOGY:linear
CLONE:HUMGS04020
SEQUENCE DESCRIPTION:
GATCTCATAG GACGGAGGGG GAAATGGTTT CCCTCCAAGC TTGGGTTAGT GTGTTANCTG 60
CTTATCAGCT ATTCAGACAT CTCCATGGTT TCTCCATGAA ACTCTGTGGT TTCATCATTC 120
CTTCTTAGTT GACCTGCACA GCTTGGTTAG ACCTAGATTT AACCCTAAGG TAAGATGCTG 180
GGGTATAGAA CGCTAAGAAT TTTCCCCCAA GGACTCTTGC TTCCTTAAGC CCTTCTGGCT 240
TCGTTTATGG TCTTCATTAA AAGTATAAGC CTAACTTTGT CGCTAGTCCT AAGGAGAAAC 300
CTTTAACCAC AAAGTTTTTA TCATTGAAGA CAATATTGAA CAACCCCCTA TTTNGTGGGG 360
ATTGAGAAGG GGTGAATAGA GGCTTGAGAC TTTCCTTTGT GTGGTAGGNC TTGGAGGAGA 420
AATCCCCTGG NCTTTCACTA ACCCNTNTGG ACATACTNCC CACAACCCAG TTNGATGGGN 480
TTTCCCGTAA TAAAAAGGGN TNGGGNNTTN CCTTTTTGAA A 521

SEQ ID NO:3414
SEQUENCE LENGTH:501
SEQUENCE TYPE:nucleic acid
TOPOLOGY:linear
CLONE:HUMGS04021
SEQUENCE DESCRIPTION:
GATCAGAAAT NACTGTGTCA TGGTGCTCAT TCACTGCAAA CTCCCAGTTG NAAGCTCCTT 60
GGCTCCCCCG GAGGAGCAAG AATCTCATAG TTCAGAGACA CAGAGGGCCT TTTAGCCCTA 120
ATNACCTTTT GGATGGGACT GCAACTCATG ACTATCCTGA TATTGGAAGA AAGGACTTTG 180
TTAATCTTCT CCCCCATAGC TCTGCTGCGT AGGTCTACAT CTTACTCAGA ATCACTACAC 240
ATTCCTTTAG TCTTCCTCCA AGCTCCAGAG CCATTGGTAC AAATGCTTTA TTGAAACTAA 300
ATACATANTN NACACAATGA GATGAAGACA ATATAGANGT NCGCATAGTC ATCATAATCC 360
CGTTCCTTGG CCGGTTGAGG CAGCTCAGTG GCTGAGCCCA GTCAAGCCAA CCCGGNAGTT 420
TCACTCACGG CNTTCAAGGN TTTTGATGCT AATTCTTTTG GGTTTCCTAC AGTTTATTAA 480
ATAAGTNGTC TGNGTGGGAA A 501

SEQ ID NO:3415
SEQUENCE LENGTH:485
SEQUENCE TYPE:nucleic acid
TOPOLOGY:linear
CLONE:HUMGS04022
SEQUENCE DESCRIPTION:

GATCTCAGTG AACTAATACA CAGCTTGAGC GTTTCCATGT GCTAATGTTG CACATTTACT 60
AAAAAACTTT GGAAATGGAA AATAATGTAT TAGTGCAACA GTTGATGTGC TTCTTTGGGC 120
AAAGATATAG TTTTGTTCCA CAATTTGTAC TTAAAAGCGA AAGAACATTG AAAACATAGA 180
CTTACTGGCT GTAGCAATGC TGGCCTGTTA ACTGATAACT AGAACTTAGG TTCACGTTTA 240
TGTAAAGTGT GTAAAACCTA GTAGAGCTTG CATAGTCGGG CACTCAGTAA ATGGTTTGGT 300
TCCTTTTGCC CCTTGGTAAG TTTATTTTAC CCATCCNCCC ACCTGCCCAT TCTGACTTTN 360
TATTAAAATC ANCCATGTGG GACCCAGNGG TGTTTAATGN GGATGTTATT NCCAGAAGGA 420
GGATTTGAGA AAAATTTGGG GATTNTCATT GCAGGTTAAC NATTCCAAAA ATNCTTTTTG 480
GTAAN 485

SEQ ID NO:3416
SEQUENCE LENGTH:471
SEQUENCE TYPE:nucleic acid
TOPOLOGY:linear
CLONE:HUMGS04023
SEQUENCE DESCRIPTION:
GATCTGTTTT TTCATCGAGG TNTCGAGGTA TNCTTTNAAA GATAGTGANA NGCAGACACT 60
GCTCCTTGTG CAGCTCTGGT ACCTCCTGCC CACTGCTGTC ACTTCAAGCC ACTGGCAATG 120
CTTCTGTCCT CGTGTCTTGG AGGAAAATCA CCTGGGGGGA GGGGACTTCT TGTGGTAAGA 180
GCAAGTGCAG GTATGAAATG CGAAGATTGC CCCAGCTAAA AGTGGACAAG TCCGCTTTGT 240
GAGATGAATA CTTCCTGAGA NNCNNNNCAA GTATCTCTCC ATTTTACCAT TATGAAAACT 300
ATCATTAAAA AAAACAGTTT AGATGCCTTC TCCTTTTGAG GGAAAAAGGG TGCTTTTTAT 360
TGTATAAAGC AGCGTCTTAT GTATTTTGAT ATACCATTGT TTGAACTTCC GTCTTTAGCT 420
GATAGATTCT CAAATATCCT NGATTTTGGG ATGTTCAGTA TGTTTGTGAG N 471

SEQ ID NO:3417
SEQUENCE LENGTH:466
SEQUENCE TYPE:nucleic acid
TOPOLOGY:linear
CLONE:HUMGS04024
SEQUENCE DESCRIPTION:
GATCAAATAT ATTTCAAGCT ATAAAAGCAG GAGGTTATCT GTGCAGGGGA CTGGCATCAT 60
GTATTTAGGG GCAAGTAATA ATGGAATGCT ACTAAGATAC TCCATATTCT TACCCGAATC 120
ACACAGACAG TTTCTGACAG GCGCAACTCC TCCATTTTCC TCCCGCAGGT GAGANCCCTG 180
TGGAGATGAG TCAGTGCCAT GACTGAGANG GANCCGACCC CTAGTTGAGA GCACCTTGCA 240
GTTCCCCGAG AACTTTCTGA TTCACAGTCT CATTTGACA GCATGAANTG TCCTCTTGAA 300
GCATAGCTTN NNNAATATCT TTTTCCTTCT ACTCCTCCNT CTGACTCTAA GAATTCTCTC 360
TTTCTGGANT CGCTTGAACC CAGGANGGCG GGAGGTTNCA GTAAGCCAAG GTCANTGCNA 420
CTGCACTNTT AGCCTTNGGT NANAANTNGA GGTTTCCATT TNTAAA 466

SEQ ID NO:3418
SEQUENCE LENGTH:464
SEQUENCE TYPE:nucleic acid
TOPOLOGY:linear
CLONE:HUMGS04025

SEQUENCE DESCRIPTION:
GATCGCCAGG CTGATTGAGA AGAACAAGAT GAGTGACGGG AAGAAGGAGG AGCTCCAGAA 60
GAGCTTAAAC ATCCTGACTG CCTTCCAGAA GAAGGGGGCC GAGAAAGAGG AGCTGTAAAA 120
AGGCTGTCTG TGATTTTCCA GGGTTTGGTG GGGGTAGGGA GGGGAGAGTT AACCTGCTGG 180
CTGTGAGTCC CTTGTGGAAT ATAAGGGGGT AGTGGGAAAA GTGGTACTAA CCCACGATTC 240
TGAGCCCTGA GTATGCCTGG ACATTGATGC TAACATGACC ATGCTTGGGA TGTCTCTAGC 300
TGGTCTGGGG ATAGCTGGAG CACTTACTCA GGTGGCTGGT GAAATGACAC CTCAGAAGGA 360
ATNAGTGCTA TAGAGAGGAG GAGAGGAGTG TACTGCCCAG GTCTTTGACA GGTGTAATTC 420
TCATTCANTT AAAGGTTTCA GTGTTTTTGG TTAAGGTGGA TAAA 464

SEQ ID NO:3419
SEQUENCE LENGTH:457
SEQUENCE TYPE:nucleic acid
TOPOLOGY:linear
CLONE:HUMGS04026
SEQUENCE DESCRIPTION:
GATCACCTTC CTGTCTGGAG GCCAGAGTGA GGAGGAGGCG TCCATCAACC TCAATGCCAT 60
TAACAAGTGC CCCCTGCTGA AGCCCTGGGC CCTGACCTTC TCCTACGGCC GAGCCCTGCA 120
GGCCTCTGCC CTGAAGGCCT GGGGCGGGAA GAAGGAGAAC CTGAAGGCTG CGCAGGAGGA 180
GTATGTCAAG CGAGCCCTGG CCAACAGCCT TGCCTGTNAA GGAAAGTACA CTCCGAGCGG 240
TCAGGCTGGG GCTGCTGCCA GCNAGTCCCT CTTCGTNTCT AACCACGCCT ATTAAGCGGA 300
GGTGTTCCCA GGNTGCCCCC AACANTTCAG GCCCTGCCCN NNTNCCAACT NTTTGAAAGA 360
GGAGGNCGCC TTCTTNGGGG GCTTCCAGGT TNGGNTTTNC CCGGGNTTTT TTTTTTTCCC 420
TTGGGTGANA NATGGGTNGT TTNGGNGGCG GTNTTTN 457

SEQ ID NO:3420
SEQUENCE LENGTH:452
SEQUENCE TYPE:nucleic acid
TOPOLOGY:linear
CLONE:HUMGS04028
SEQUENCE DESCRIPTION:
GATCTGGAGG GGTCAACGGA AGACGGAACA TGTCCACTTC CAGGCCCGAG CTTCTNAGCC 60
TGCCGTTTGC CACTCTCCAG CATCTGGCCC AGCCTGTCCA TCCTCATCTC TCTTCCTCCN 120
NNACTCCGTG CTCCCATCAC TNGGAACCAT TTGCATTTNT TTGTCTCAGC TATATTGTCT 180
CACCTCTGAG TTTTTGCCCA TGATGTTGGA TGCCATGGAA TGCCATATCN TCCCCATTAT 240
CTCCCCCTTG TNTGGATAAT TCCTACTCAT CCTACAATAC TGATTTTTAT CTGTGCAAAG 300
AAGGTCTTCC CCAAGTGGCC TCTGGGTTNG ANAAGGGGNG TTCCTCTGGC TTNTTCCAGA 360
CTTTTTTGTT TCCTNCAACC AAAAGGCTTT AGGCACCGTG GGGGAAGGAG GGGTNTTNNT 420
TNGTCCNAGG GAAAAAGNNT NGNGGCAAAT GN 452

SEQ ID NO:3421
SEQUENCE LENGTH:450
SEQUENCE TYPE:nucleic acid
TOPOLOGY:linear
CLONE:HUMGS04029

SEQUENCE DESCRIPTION:
GATCAAAACT AACCTTTCAG CGTATTCTCA GCTATGACAC AGTGGTGCAA ACAACTGGAG 60
ACTCGTGGCA CATACCATAT GATGAAGACT TGGTAGAAAG ATGTTCCTGT GAAGTACCAA 120
CCAAATGGCT TTGCCTCCAC CAAAAGACAT AGAGATTCTT ACCTCTATGC TAAGTTTGTG 180
TTTGGGTACC CTCTAGGTTG GCATCAGAGG CTCTTGAGCT GGTGTTTGTT TAGGGCATTG 240
CCTCTGTCCT GAAGATAAAA GGATTNTATT AACAGCATTG ACATTGATTT TTTAATGAAA 300
TGAGATATAT CTTTTCATAA CCAGCTGCGT TTTTTTCCCC TAACATTTGT TTTTGGAGGG 360
CTTATCAAGA GTTGGAGACC TTAGTGTAGA GCAAAACCTG CATTTCTCCT ACTGGGCCAG 420
CTATTCCACT TAGGCTTGGG TGACTAANAN 450

SEQ ID NO:3422
SEQUENCE LENGTH:444
SEQUENCE TYPE:nucleic acid
TOPOLOGY:linear
CLONE:HUMGS04030
SEQUENCE DESCRIPTION:
GATCCCAAGT ATTAGGAGCT ATTTCTTAAA TAGTTTAAAT TTGCCTTTGA AAAAAGTTCC 60
ATTACTTAAA AGTATTAATT TAAGCAAAAT CATGGAACAC ATTGACTAAA AAATACCTTT 120
TATTTTTACT CAACTTGTTA AAGGGGCAGA TAAAGACTTT GGCAAATTTG ATTAACTGCA 180
CAAAATAATG TTATCCAAAC TGAGTAATTT ATTGCTGGTC TGNGGCTTGC CTTGTTTACA 240
CTTAAGTAGA ATTTACGTTG TTTCCACAAA TGGCAGATAT CANCATTTAA AGGNATTTAA 300
TAGTGACCTA TTCAATAAGG CANTCATCTG GGGTAGCAAA CTTTTNTTTA AATCTNACAT 360
AACCTCAGGC TTTATAANAA NGCCACCATA ATTGGAAAAT TGGGGNTCCT CCCCTCTAAA 420
NGCCTTAAGG GATTCAAAGG CTCN 444

SEQ ID NO:3423
SEQUENCE LENGTH:439
SEQUENCE TYPE:nucleic acid
TOPOLOGY:linear
CLONE:HUMGS04031
SEQUENCE DESCRIPTION:
GATCTTGTGA AAATGCTGTT AGTATCCAAC CTTAAAATAT ATTAGTATAT GGGTTTTTAT 60
TAAAAGAATT ACTTTGAATT TNCTATTTAA TTCATATGTA AATAAAGGAA CATTTCATTT 120
CACTTAAAAA AATTATATCA GTTATTAGGC TGGGTGCAGT GGCTCATGCC TGTAATCCCA 180
GCACTTTGGG AGGCCAAGGC GGGTGGATTA CCAGAGTTCG GGAGTTTGAG ACCAGCTTGA 240
CCAACATGGA GAAACCCCGT CTCTACTAAA ATTACANAAT TNGCCAGGTG TGGTGGCGCA 300
TGCCTGTAAT CCTGGGCTAC TCAGGAGGCT TGAGGCAGGN GAATNGGNTT GAAAACCCCN 360
GGGGGNNAGA GGTTGCGGTT GAGCTGAGNN TTGCGCCATT NNACTTCCNN CCTGGGCAAG 420
GNGGGGNGNA ACCTNTNNN 439

SEQ ID NO:3424
SEQUENCE LENGTH:437
SEQUENCE TYPE:nucleic acid
TOPOLOGY:linear
CLONE:HUMGS04032

SEQUENCE DESCRIPTION:
GATCTAAGAA ATCATGTGCA TATATCTTTC AGATTTCTAT TTTGGGAAAN TGAAGGTTTC 60
TACAACATAT TGTTTCAGTG TTCAAATAAA CTGAAGGACT CAACATTACA TTTGAACTAT 120
ATCCTTCCTA GTGGGTTAGT GTGAAAAAGN GNTTGGCTGA TTCCTAAAAC TCTGCCAGCC 180
CTGCAGTAAT CTCCAGGGNC NNGTTATTGT TCAGACATTC CNTGGTGATT CCTGGGAAGG 240
AAGCTTGGCT GCTCAGTTTC TGAGTCTGGG GTGAGATAAT GTTCTGGGAA GGGACATCTG 300
TTCTTTGGTG TAATCTCTCA TGGTGAAATC TGCTCTGTAC ATCAGACAAT TGCATTGCTA 360
CCAAGTTTCA TACCAAATAT TTGAAAGGTT GGTATTGAAT CTAAANCCAA ATATTAGTTT 420
TTNTTAAACT CATGGGN 437

SEQ ID NO:3425
SEQUENCE LENGTH:424
SEQUENCE TYPE:nucleic acid
TOPOLOGY:linear
CLONE:HUMGS04033
SEQUENCE DESCRIPTION:
GATCCAAACC TTACACCTGA GTCTGTTAAG AGCGCCCTGC TACAGGAGGC CACAGACTGC 60
CTGATTCTNN NGGACCGCTG TTCTCAAGGA CGGGTAAAGG GTCTCCTGGC CAAAATTCCC 120
CAAAGCTGTG AAGACAGAAA AGTGGTGAAC TATATCAGAA AAGTGCAGCA GGTTTCTGAA 180
GGCGCACCCT ACAATGGAAC TTAAGACTTG TATTACTTTC CCAAGAGGAA AGGATTTTTT 240
TCCCATCCCN NNTTGTATGA ATGGAGTTAT TTANGAAAAA ANGATATTTT TACACGAAAC 300
TTTGTAAGTG AAAGCTGCTT TTTCCTTCCT TTCCTTTCCT TTTACCTCCA TAAGGAATAG 360
GGAANGTAGG ACCAAGANAN AATTGAACTC CTTCCCATTT TCTAAATAAA GGTTTTGGGG 420
GAAA 424

SEQ ID NO:3426
SEQUENCE LENGTH:422
SEQUENCE TYPE:nucleic acid
TOPOLOGY:linear
CLONE:HUMGS04034
SEQUENCE DESCRIPTION:
GATCTCNGAG CTACAATACT CACTANATAT ANTTTTCCAA TCAAAATATN CTATTCTATA 60
TTCTAAGGGT TAATATGTGA TTATAGTGTC CACTTGCCAC CATTTTTTTA AATCAATGGA 120
CTTGAAAAGT ATTAATTTAG ATGGATGCGC AGATATACCC TCAGTTCAGT CATAGATTGG 180
AGTTTGCATA TAATANNNCN CCGGTATGTN GACACTATTC TAAATAGTTC TATTATGACT 240
GAAATTTAAT TAAATAAAAN NGGTTGTAAA ATGNGATGTG TATGTGTATA TACNGTATGT 300
GTACTTTTTA AAATAGGGTG TATGTCCCAA CCCTTTTTTA TACAGGTNTG AATTTAAAAA 360
TACCATGGTT ATATACATAT ACCTNGNNTT NGTCCTAAAA TAAAGGNTTT TNATGCACGC 420
NN 422

SEQ ID NO:3427
SEQUENCE LENGTH:408
SEQUENCE TYPE:nucleic acid
TOPOLOGY:linear
CLONE:HUMGS04035

SEQUENCE DESCRIPTION:
GATCTTTCCA CCTCAGCCTC CTGAGTGGCT GGGACTACAG GTGCATGCCA CCAAGCCTGG 60
CTAACTTTTT GTAGAGATAG GGTTTTNCCA TGCTGCCCAG GCTGGCTTAG AATTCCTGAA 120
TTCAAGTGAT TTGTCTGCCT CAGGCTCCCA AAGTGCTGGG ATTACAGGCA TGAGCCACAC 180
TGCACCCAGT CCATGCTACC CTTTTTATAT GAAACACCTA TTAACACGGT ACAACAACAC 240
TTCAAAACAT ATTATTTGAA TTTNGGCNTC TGATTTTTTA GTCANCAATC CATTANGGCA 300
AANACCTNGT TTTCCAAACT CAGATGTTCT CCAACTTACA CTAATGTNTG GTTTTTTTGG 360
ACTTNGGTGN GGGGAAAAAN TTAACCNGGT TTTNNTTTTN TTATCAAA 408

SEQ ID NO:3428
SEQUENCE LENGTH:378
SEQUENCE TYPE:nucleic acid
TOPOLOGY:linear
CLONE:HUMGS04036
SEQUENCE DESCRIPTION:
GATCTTAGCT TCTTTAATCA GACTTTGTGA CTTAAAAGTT TGGGGGTTTT CTTTGAAAGT 60
TTCCAGCCCT ATTCAGAAAG CAACTCTTGG CTGTGTGCAT TTTTCAACTC CAAGCAGCCC 120
AGGGGTAAGT AAACAAAGTA TGGATGAAGG TCAGATTTTN TTGTCANNNT CTGAGAAACC 180
TGGCAGCCTG CTGTTAACAA CACAGGCCAG TATTGGGTTT TATTGAATTT GGTATGTGAC 240
CAAGGTCGGC CTAAAGGATG GCGCAGGTCC TGGGCAGGAA AGAATTTTTC CTTTATCACA 300
TAACTGTAAT ATTTGGTTGC TCAGCATAAG TNATGGAAGC AACCACTTAA TTTCTAATAA 360
ANTTGTGTTA ACCTCAAA 378

SEQ ID NO:3429
SEQUENCE LENGTH:376
SEQUENCE TYPE:nucleic acid
TOPOLOGY:linear
CLONE:HUMGS04037
SEQUENCE DESCRIPTION:
GATCACAACA CCAGTACCAG CACGAAGAGC CTCCTCCCGA AAGAGTCTCG ACTGGACACC 60
TTCTGGGACT AGCAGTGAAT CGGGACACAA ACCACCCACC CCATTGGGAG AAAAACCCAG 120
ACGCCAGGAA AAGAAGAAAC AACAAAGGCA GGAGAACAGC CACTTTCAGA CTTGAAAATG 180
ACAAAACCCT CAGTTGAGCC TGAGCCCCCG GCGCGGGGGC TGCTACACTA CAGGACACCC 240
AGCATCGGCT TTGACTGCAG ACTGTTCACC CACACGAGCC CTGTGCTTTT GGTGTAAATA 300
ATGTACAATT TGTGGATGTC ATTGAATCTA GAGGACTTTC CCCTTTTTAT ATTTGTATTA 360
ACTTTAACTT ATTAAA 376

SEQ ID NO:3430
SEQUENCE LENGTH:368
SEQUENCE TYPE:nucleic acid
TOPOLOGY:linear
CLONE:HUMGS04039
SEQUENCE DESCRIPTION:
GATCAAGCTT GTGTACTTGA CCGTTTTTAT ATTACTTTTG TAAGTATTCT TGTCCACATT 60
CTACTTCAGC TTTGGATGTG GTTACCGAGT ATCTGTAACC CTTGAATTTC TAGACAGTAT 120

TGCCACCTCT GGCCAAATAT GCACTTTCCC TAGAAAGCCA TATTCCAGCA GTGAAACTTG 180
TGCTATAGTG TATACCACCT GTACATACNN NGTATAGGCC ATCTGTAAAT ATCCCAGNGA 240
NCAATCACTA TTCTTAAGCA CTTTGAAAAT ATNNCTATGT AANTNATTGT AAACTTTTTC 300
AATGGTTGGG ACAATGGCAA TAGGACAAAA CGGGTTACTA AGGTGNAATT GNCCAAAAAA 360
TTTTTTAN 368

SEQ ID NO:3431
SEQUENCE LENGTH:374
SEQUENCE TYPE:nucleic acid
TOPOLOGY:linear
CLONE:HUMGS04040
SEQUENCE DESCRIPTION:
GATCAATCTC AATTCCTTTT CATGCCCTCC TGCATTGCTG CTGCGTGGGT ATTTGTCTCC 60
TTAGCCATCA GGTACAGTTT ACACTACAAT GTAAGCTATA GGTGGAGCAT CAGCAGTGAG 120
TGAGGCCATT CTTCATCCTT AGGATGTGGC AATGAAATNN TGGTGCAAGT TCCTTTCTCT 180
TTTGTGAATC TTTCCCCCCA TTTCCTGTTT ACATGTAACC CAACAAAATG CAATTTCTAG 240
NGCCTTCTGT CCAATCAGTT CTTTCCTCTG AGTGAGACGT ACTTGGCTAC AGATTTCTGC 300
CTTGTTTTGC GACATTNNTC CCATTCACAC AGNTATTTAN GGGGCTAATN AANNGGANAA 360
ATAAGGCTTA CAAA 374

SEQ ID NO:3432
SEQUENCE LENGTH:369
SEQUENCE TYPE:nucleic acid
TOPOLOGY:linear
CLONE:HUMGS04041
SEQUENCE DESCRIPTION:
GATCCTGGAG AACCTAAGAC TCCAAAAGCG TGGTACTGGA GGAGTGGACA CTGCTGCCAC 60
AGGCGGTGTC TTTAATATTN CTAATTTGGA CCGACTAGGC AAATCANAGG TGGAGCTGGT 120
GCAACTGGTC ATCGATGGAG TAAACTATTT GATTGATTGT NAACGGCGTC TGGAGAGAGG 180
CCAGGATATC CGCATCCCCA CACCTGTCAT CCACACCAGG CATTAACTCC CCATCGNCCA 240
GCTGATGACT CAAGATTCCA AGGAGTTCTG CTCATTCTAA TGGTGGGCCG TTCTACTTGN 300
TCTGGACCTG NCCTNGNATC CCCNTNGNCC TNTNNTTCCT TAGTTAAAGA CTTNGTTAGC 360
TGATGNAAA 369

SEQ ID NO:3433
SEQUENCE LENGTH:358
SEQUENCE TYPE:nucleic acid
TOPOLOGY:linear
CLONE:HUMGS04042
SEQUENCE DESCRIPTION:
GATCGTGGGG TTGTTGGCAG AGGGCAACCC TGGGCCCCAC ACCGTGTGGA CAGGCAGACA 60
CCAGATTGTC CAGGAGCAGG AGCTGCTGGG ACTGCGCTGG CCCCGGACCT AGTGGGCCTT 120
CTCCTGGCTG CTGAGATGTC GTCTGTGACT GGCCTGGCTG GAGGGGGAGT GTTGACAACC 180
CAAAGCTGTT CTCCAGTCTG GGGAGGGAGA GGCAGGGTCC CCAATGTCCG AGCTGNATCT 240
GGACGCTGCT CTTAAAGGAC CTCCTGGGGN AGGGGAAGCG GTAGGNTCTG GACTGGGNAA 300

GATGCTGTAT GACCTCCCTG AAGNACCCGT GACTTCCCCA TGNTTTACCC TTTTGTGN 358

SEQ ID NO:3434
SEQUENCE LENGTH:354
SEQUENCE TYPE:nucleic acid
TOPOLOGY:linear
CLONE:HUMGS04044
SEQUENCE DESCRIPTION:
GATCTAGAAA GCAACTTGGA AGTGTAAAGA GTCACCTTCA TTTTCTGTAA CTCAATCAAG 60
ACTGGTGGGT CCATGGCCCT GTGTTAGTTC ATGCATTCAG TTGAGTCCCA AATGAAAGTT 120
TCATCTCCCG AAATGCAGTT CCTTAGATGC CCATCTGGAC GTGATGCCGC GCCTGCCATG 180
TAAGAAGGTG CAATCCTAGA TAACACAGCT AGCCAGATAG AAGACACTTT TTTCTCCAAA 240
ATGATGCCTT GGGGTGGGGA GTGGTAGGGG GAAGAGCTCC CACCCTAAGG GGCACACACT 300
GAGTTGCTTA TGCCACTTCC TTGTTCAAAA TAAAGTAACT GCCTTAATCT TAAA 354

SEQ ID NO:3435
SEQUENCE LENGTH:354
SEQUENCE TYPE:nucleic acid
TOPOLOGY:linear
CLONE:HUMGS04045
SEQUENCE DESCRIPTION:
GATCATTAAT GAGGAGCTGA TGGCCTCCCT GGACCAGCCA ACACAGACAN TGGTGATGCA 60
CCGCACTGAG CCCACTGCCC AGCAGAACCT GGCTCTGCAG CTGGCCGAGA AGCTGGGCAG 120
CCTGGTGGAG AACAACGAAC GGNTGTTTGA CCACAAGCAG GGCACCTACG GGGGCTACTT 180
CCGAGACCAG AAGGACGGCT ACCGCAAAAA CGAGGGCTAC ATGCGCCGCG GTGGCTACCG 240
NCAGCAGCAG TNTCAGACGG CCTACTGAGC TCTCCACTCT GTTTCCCGCC TGGGCCATCC 300
AACCTTGAAG TCCTAAACCA CACCTCAGTC ACTAAAGGTC TGTTTAAAGT TAAA 354

SEQ ID NO:3436
SEQUENCE LENGTH:350
SEQUENCE TYPE:nucleic acid
TOPOLOGY:linear
CLONE:HUMGS04046
SEQUENCE DESCRIPTION:
GATCTAATTT ATTTATTTAT TGCCTGGCCG GTGACTCGGG GGAGGAGGCG ACCCTGTCAT 60
CTGTCCCACC TGCTGCTGCC CCTTGGAGCA GCCTGCACCT NCTCTCCTCC CATCCGGCAA 120
CAGTNTGAAA GTACGTGGAG GACGGGACCG GAAGACGAGA GAGGGCTGGA CATCCTGCCC 180
ACCGTNTCCC AGCCAGGGCA GGNGAGGGGA CCATGGCCCG NANGTCAAGG GGCCCCGATG 240
TGCACAGCTG CCACAGGGAG GGAGNTCTTG GGGAGATGGG CANTCAGGTG GCCCGTCCTT 300
GGTNGAGTGC ACANANTTGC GCGNACANAT CGGGGTTTTT CCTGGNTTTN 350

SEQ ID NO:3437
SEQUENCE LENGTH:348
SEQUENCE TYPE:nucleic acid
TOPOLOGY:linear

CLONE:HUMGS04047
SEQUENCE DESCRIPTION:
GATCTTGAAT CTCTTTCTTT GTTCTGTTTG TTTAGTTAGT ATCATCTGGT AAAATAGTTA 60
AAAAACAACA AAAAACTCTG TATCTGTTTC TAGCATGTGC TGCATTGACT CTATTAATCA 120
CATTTCAAAT TCACCCTACA TTCCTCTCCT CTTCACTAGC CTCTCTGAAG GTGTCCTGGC 180
CAGCCCTGGA GAAGCACTGG TGTCTGCAGC ACCCCTCAGT TCCTGTGCCT CAGCCCACAG 240
GCCACANTGA TAATGGTCTG TTTAGCACTT CTGTATTTAT TGTAAGAATG NTTATAATGA 300
AGATACACAC TATANCTACA AGAAATTATA AATGTTTTTC ACATCAAA 348

SEQ ID NO:3438
SEQUENCE LENGTH:347
SEQUENCE TYPE:nucleic acid
TOPOLOGY:linear
CLONE:HUMGS04048
SEQUENCE DESCRIPTION:
GATCTGAGCC ACCCTCCCCG CACAGCCCTG CACCCCGCCC CTAGGGTTGG CAGNCTCANT 60
TGGCCCCTGG CAGAGGAACA AGGACACAGA CATTCCCTCA GTGTGGGGGG CAGGGGACAC 120
AGGGAGAGGA TGGTTGTCCC TGGGGAGGGC CCTCTGGCCC CAGGCAACCT TAGCCCCTCA 180
GAACAGGGAG TCCCAGGACC CAGGGAGAGT NTGGGGACAG GACAGCCTGT TTCTTNTAGC 240
TTCCTGGGGT GGGAGGCACA GGGGCAAAGC ANTACCCCAG GGAAANTGGG AGGTGGTGCT 300
GGTGCTCTCT CCAGGNCCAC CATGCTGGGA GAGGCGGCCA GAGCTTN 347

SEQ ID NO:3439
SEQUENCE LENGTH:346
SEQUENCE TYPE:nucleic acid
TOPOLOGY:linear
CLONE:HUMGS04049
SEQUENCE DESCRIPTION:
GATCTTCCCT GGNGGCCTTC GGCTGCNTGC NTGCCTGAGG TCCCNGCTGC CAGTCCCGGG 60
CCCTGGAGAG CAGATGCTGT CTTGTNATGT ACAGGAGGAC CTTTTAAAAA AATCAAGTTT 120
CTATTTTTTN CTGGTAGTCC GCATACCCAT ACCCTCTGTT TTTNAAAGGC AAAGGCCAAT 180
NAGTCCCCAT TTGTAGCATG GCACCAGGGT CTTAGGCCTA GTCCTCTCAT TCCTCCCACC 240
CTCCGAGATG GTCAGTGTGT CATGGGAAGC CCACCCCCAG CTCTGCCAGT GCTCTCTGGG 300
CCTGGNTCCN AGTCAGTGGT GGCCACGATG CGGTACAGGG CATCCN 346

SEQ ID NO:3440
SEQUENCE LENGTH:343
SEQUENCE TYPE:nucleic acid
TOPOLOGY:linear
CLONE:HUMGS04050
SEQUENCE DESCRIPTION:
GATCCATGTG ATTATTAAAA TGACTGTAGC TGAGAGCTCT AATTTNCCTG TCTTGAAACT 60
GTATAAGANC TCATGTGATT AAGTTCACAG TTTATTGTTT GTNTGTTTAG TATTTNAGAA 120
ATATACCAGC ACTACTAATT AACTAATGTC TNTNATTTAT NATATTATGA TAAAGTAAAA 180
NTTTCACTTG CATTAAGTCT AAACTGAGAA GGTAATTACT GGGNGGNGAN TGAGCAGCTT 240

TGACTTTGAC AGGCGGTTTG TGCAGGAAAG CACAGTGCCG TGTTGTTTAC AGCTTTTCTA 300
GAGCAGCTGT GCGNCCAGGG TAGAGGAGTG TNGAAATTCA TTN 343

SEQ ID NO:3441
SEQUENCE LENGTH:340
SEQUENCE TYPE:nucleic acid
TOPOLOGY:linear
CLONE:HUMGS04051
SEQUENCE DESCRIPTION:
GATCTGCTCA GAAAGGAAGA GGCAGGCGCC AGGGGGAACC CCCTTCGTGT TTTGTAACCC 60
TCCCTTTNAG GTGAAGCCCT TTTTCTTGCT AAAACCGGCA ATTCTCCGGT TAGAAATNTT 120
ACTTGGTGTT TTTTGGTTTT GTGAAACGGC CGTCCCAAAG CTGGCTGGAT TCCTAGAAGA 180
GTCTGTGTTG AAGGCATCTT TCAAGCCCTC GCTCTGGTTC TNAGGGCAGC ATTTTCCAGG 240
CGGGTTTGTT TTGCATTTCT TGGAGCCTCT CCGNGNAGCA ACCAGACGGG AGATTTTAAT 300
TTTAAGCTGT TCATGCTGGG NCTGACAGCC TGCAGGGTTN 340

SEQ ID NO:3442
SEQUENCE LENGTH:339
SEQUENCE TYPE:nucleic acid
TOPOLOGY:linear
CLONE:HUMGS04052
SEQUENCE DESCRIPTION:
GATCCTTGGG CTGGCCTAGA ACCAGTATCT GTAGTGGATA TAAGCCAACA ATACAGCAAT 60
ACTCAAACAT TCACAGGCAA AAAAGGAAGA TACTTTTNTT AACATTTCTG AAATTCAACT 120
GGAAGCTTCA TGTGTCAGGA ACATCTTGGA CAAAACTTTA NGTTGTGTTG ATATAAATTT 180
ACCCAAAGAT GATGNCTTTG ATTGGNTAAT TAGTAAGGTC TTTTTGTTAT TTTNCATCGT 240
ATCAGGTATT GTTGATATTA GAGAAAAANG TAGGATAACT TGCAACATTT AGCTCTGGAA 300
GTACCTACCA CATTTTNGAG ATTTACCGTT TCCATATTN 339

SEQ ID NO:3443
SEQUENCE LENGTH:339
SEQUENCE TYPE:nucleic acid
TOPOLOGY:linear
CLONE:HUMGS04053
SEQUENCE DESCRIPTION:
GATCTGCCGT TNATGTTGCT TTCTCCTTTG TCCTCTTGGA CTTGAGGGCA TTGTGAAAAG 60
CTTTGCTGTG ATTTAAAAAT GCCAGCAATT TTAATCTAGC AGTGTTGAAG CTGGGAATTT 120
TTTGGCGCAA TCCATGTAGC AGTGACCCAG GCTTGGGAGC CAGAAACAAG TGTGACCTGG 180
GATTTNATTT AACACAACTG TTGCCAAAGA GTTGGCTTTG TTTATTTGGT TTTGGCGGGG 240
AGAGGAGTGG TATTTNATGC TTTCTGTGGA CANTGTAACC CTAAACACAN CANGTATTTT 300
AANTGCCACC TACATAANTA AACCATANGC NTATTGAAA 339

SEQ ID NO:3444
SEQUENCE LENGTH:339
SEQUENCE TYPE:nucleic acid

TOPOLOGY:linear
CLONE:HUMGS04054
SEQUENCE DESCRIPTION:
GATCTACATA AAAACTGCTA CATGNCAAAA ACCACACCTA AAGAAATTTT AAGAATTTGG 60
CACAGTTACT CACTTTGTGT AATCTGAAAT CTAGCTGCTG AATACGCTGA AGTAAATCCT 120
TGTTCACTGA AGTCTTTCAA TTGAGCTGGT TGAATACTTT GAAAAATGCT CAGTTCTAAC 180
TAATGAAATG GATTTCCCAG TAGGGGTTTC TGCATATCAC CTGTATAGTA GTTATATGCA 240
TATGTTTCTG TGCATGTTCT CTACACAATT GTAAGGTGNC ACTGTATTTA ACTGTTGCAC 300
TTGNCAACCT TTCAATAAAG CATATAAATG TTGGTTAAA 339

SEQ ID NO:3445
SEQUENCE LENGTH:334
SEQUENCE TYPE:nucleic acid
TOPOLOGY:linear
CLONE:HUMGS04055
SEQUENCE DESCRIPTION:
GATCAGCCTC TTCCTCTATA AACAATGACC AATTAGACGT TTCCGTAATT CCATGTATTA 60
TGTATAGTAC ACTCTATAAA TGTAAATGTA ATGCTTGTCT AAAAAGTGCA ATTTATTGTA 120
CATTGTCCCA ACAAATGTTT ACTTTTATAA TCGTTATGAA CTTGAATTGG ATTAGTATCT 180
TGTTTTTATG TGTGAATGAA GCCTTGTGAA ATAAACAAAT GCAACTGAGA AGGTAACAAG 240
GTGACTGTTT TTGTGAGCCA GTGATGTTTT CAATGCTTTG TGTTGCCCCT TTGGCCCCAT 300
TAAGCAGTAA TAAACATTTG TTCTGAAGTC CAAA 334

SEQ ID NO:3446
SEQUENCE LENGTH:334
SEQUENCE TYPE:nucleic acid
TOPOLOGY:linear
CLONE:HUMGS04056
SEQUENCE DESCRIPTION:
GATCCAAGAA GCGGGTCCCT GAGACGGGGG GTGGCTGCCC TCCCCAGACC ACCCCGGCAG 60
CCTGAGCAGN TCCAAAGCAC TGGCTTGGGG TCCGAGACCT TCAATGTAAA GCAGGCGGAA 120
TGGGGGGACA GGACAATTTC TCCCCCTCCA GGGGCTCCAG GACTCTCCCT GGGGGGCCCA 180
CCTCTTGCCC CCTAACCTCT TTCCCCCTTT TCTGCCCCCG TGGGGAGGAG CCCCTTGTAC 240
CTGCTCCGTG CCCAACACAT GCCCTCTCTG TACATCTTTT GGTAAATNAT GAGAAATAAA 300
GGAAGTGGAC GCAAAGTGGA TGCGGCAGCA GAAA 334

SEQ ID NO:3447
SEQUENCE LENGTH:332
SEQUENCE TYPE:nucleic acid
TOPOLOGY:linear
CLONE:HUMGS04057
SEQUENCE DESCRIPTION:
GATCCAATCT CAGTGTCTAA CTCATCATCC CAGATTATTC TGAAGTGGAA ACCACCCTCC 60
GACCCCAATG GCAACATCAC CCACTACCTG GTTTTCTGGG AGAGGCAGGC GGAAGACAGT 120
GAGCTGTTCG AGCTGGATTA TTGCCTCAAA GGGCGAGTCC AGTCATCAGC TCCGCTGTAA 180

GCTGTTGGCC CTGCATTCAA GTAACCAGAA CCACCCGTGA GAGCTGCCAC CAGGTANTGT 240
CACTCGAAGC TTCCTTTTTT TTTTTTTTNA ATTCCGGAAA GTTTAANTCT GTANTCCCAG 300
GGGNCCATTT AAANANGGTT TNTTTTTGTA AN 332

SEQ ID NO:3448
SEQUENCE LENGTH:332
SEQUENCE TYPE:nucleic acid
TOPOLOGY:linear
CLONE:HUMGS04058
SEQUENCE DESCRIPTION:
GATCCAAAAA AAGCTNGAAG CCTACATTTC CCAGAGCCCC TTTCAGCTTG AATTCCAAAT 60
GTGACTTAGC TTCCTCTATG CAGATGTACC TACTTGAAAC TGGGAAGGCC AAATNAGATA 120
GAGGNAATNT TTCTGCCAAC AAACAGCCAC AGAGCATTTT ATTTTTCTAG AAGAGCAATA 180
ACAGAGACCC CAGGGTGTGG TGTGGCAGGA CGAGGAGCAT CCATTTTGCT GGNGGGGATT 240
GTGGCTAAAA TAATGNGGCT CTGGAGCCAG CAATNGGCAG CATCTTCATG ACTTTCCAGN 300
TTCCAGGCTG TAAGCAGAGA TAGCATCTCT TN 332

SEQ ID NO:3449
SEQUENCE LENGTH:326
SEQUENCE TYPE:nucleic acid
TOPOLOGY:linear
CLONE:HUMGS04059
SEQUENCE DESCRIPTION:
GATCCACTAT ATGCCTCAGC ACATGGAACC TTAATGACCA AAGTGAAGAG CAGATTATTC 60
ATACGGTGTA ATAAGCATCT GGAATGGACC CATCCGTGTA CTTCATTCAA ATGTGTAAAT 120
GTCATATTCA TTCAGATTTA TAAAGCTAGT AGTGTATAGT CAGAAACAGA ATCAAAGTTA 180
GATATACATT TTTAAATATT TACTGCATAT GAGGCTTTCT GTTAATTTTT TAATGTGAAT 240
AATTTATATA TTGCACATTC TAGGGAATAA TATTGATTGT ATGNCTACTG TGCTGCATTA 300
AGAAAATAAA ATTCCTATAT NCCAAA 326

SEQ ID NO:3450
SEQUENCE LENGTH:329
SEQUENCE TYPE:nucleic acid
TOPOLOGY:linear
CLONE:HUMGS04060
SEQUENCE DESCRIPTION:
GATCAGAAAA ACTGAAGATG GAAGTTTTGG CCGGTGCTCA TTAGACATGA GTCCTCACTC 60
TGTNTCCTGA GCCCGTTTCA TTCTTCCAAC CTCCCTGCCC CCACACACTT ATCCCAGACA 120
CAACACCATG TGGTCTGGAG GTCCCAGCCC CCACCCTAAA AAGGTTATCC CNGAGAACTC 180
CACCAGACTT GGGAGCCCAA GTGCAGTGCC TGGNGCTGCT CCCATCTGCC GGCCCCNTTC 240
TTTCCTNGCA ATTGGTTTGN ACTCACTGGG CTGTGCTCTN CCCTGTTTAC CCGATGNNNG 300
GGAAATAAAG GCCCTTTTGC CTCCTGAAA 329

SEQ ID NO:3451
SEQUENCE LENGTH:325

SEQUENCE TYPE:nucleic acid
TOPOLOGY:linear
CLONE:HUMGS04061
SEQUENCE DESCRIPTION:
GATCTGGCGC TTCTACTTTG AGGGCTTCTT TGACCTCATT GCTGTGGTAN GCCGGCGTAG 60
TCCAGACCAT CCTATACTGT GACTTCTTCT ACTTGTACAT TACAAAAGTA CTCAAGGGAA 120
AGAAGCTCAG TTTGCCAGCA TAAGTGCCAA AGGCCATCAC CAGCATCTGT CCTTCAGGGT 180
GCTCGGACAG ANTTNTTACC ACAGCAANGG CATAAGGTGC TTTATACGGA AANTCAGNAA 240
CTTAACTCTT TTGTTGCAGN TAGTCATCAG TGGCTCTGTA AAAACGGAGG GGGAAAGNGN 300
CNGGAGGTTT CTGTTTAATG CATCN 325

SEQ ID NO:3452
SEQUENCE LENGTH:324
SEQUENCE TYPE:nucleic acid
TOPOLOGY:linear
CLONE:HUMGS04062
SEQUENCE DESCRIPTION:
GATCTGATTT TATGTTCGTA ACTTTCTGTA AAGAAAGACA CAAGGACTTT TCAAAGACAG 60
TTTATATCTT TCCAAGGCAG GAAGCTTTGC ATTTGCAATC TTAGTGTTGT GTCCCTTTCT 120
GATTTTCAAA TACATCACAC CTGCCTTCCT GTTTTGGGAG CAAAGATGTA ATTTCAATTC 180
CCCTTGCTAC CGTCCTTATT TAAATNATTT CCATTCTAAG AGATTTTNGT NATTTNNNTA 240
AATGTAATCT GAGCACTTTN TNGTATTCTA AAAGATAGAT GATATCAGAT AAATATGTTG 300
TACATTTTGA TTAAAATTAT TTTN 324

SEQ ID NO:3453
SEQUENCE LENGTH:339
SEQUENCE TYPE:nucleic acid
TOPOLOGY:linear
CLONE:HUMGS04063
SEQUENCE DESCRIPTION:
GATCTGGAAG ACAGACGTAC AGCTTGGAGG GCAGGGGGAC TCTAAGGCAA GGAGATTTAC 60
AGTTGGGAAG GAGGCAGTGG CAGAGGGGTG AGGGACAGGG GCCCTTAAGT CCAGCGAGGA 120
AAGCTCNNTG TGGGCCCGCT CTACGCTCCG TTTGGGGTGA CCTGGAACGC CTCTTCTCCC 180
AGCTCCCTCC AGCCATCAGC AGCCTCTTGT CAAGCTTCTG CCTCGCCCCA GTCTATCCCC 240
AACCCCAAAT CAAGACCACC TTTCTTCACG GTCACTATTT ATTCTTTGTT CCTTTTTCTT 300
TTTGTAAGAA ACATTCACAA AAACCAGTGC AAAACCAAA 339

SEQ ID NO:3454
SEQUENCE LENGTH:317
SEQUENCE TYPE:nucleic acid
TOPOLOGY:linear
CLONE:HUMGS04064
SEQUENCE DESCRIPTION:
GATCTCAATG CTGTCTGGGG ACCCTGAAGA GTTTTNTNAC CTGTTCAGTC TCATCTAACC 60
TTCCAATGTN TGATGTTCCT GCCAAATTCC TGCCTGATTC TGGGTCCGTC CTGACCTCCA 120

AAGGTCAGNT TGGTGCTTGA GGTCTCCNTG CTCTTGGTGG CAGTGGTAGC AGCAACAGCA 180
GCAGCAGCAG CAGCAGCAGC AGCAGAGACC TNTCCACTTT CCCTTAGCCC TNTGNTGGGT 240
AGAGGGAACT TAAGGGACTT CCTCCAGNNG CNTTTAATNT GGGAATGGGT AAGAAGGTTG 300
NGCTCCNCTG TTNGTTN 317

SEQ ID NO:3455
SEQUENCE LENGTH:315
SEQUENCE TYPE:nucleic acid
TOPOLOGY:linear
CLONE:HUMGS04065
SEQUENCE DESCRIPTION:
GATCCTCAAA AAACCCAAAA ACACCATTCT CTAATAGTCA TGACAAATGG CTTCAGTATG 60
GCTTGTTTTT NATTTNCCAG ATGGCTTTTN CTCTTATTTT TNGAAGCCCC AGTCTTTGAT 120
TTTACAGGTA ACTTTCAAAA CATCATGATG CTGCCAAATG TACTTTTGTA AACTTAAACA 180
TTATGATTCC TGTATTATTT CAGTGAGAGC TACAGTGTGA TATTNCAGAG TCTATTAAAT 240
AAAAATGTGA GTTTGAATTA CACCATCTGT GCCAATTACA AAGCAATTAA AGGATTTATN 300
TNTNATGAAA AAAAN 315

SEQ ID NO:3456
SEQUENCE LENGTH:312
SEQUENCE TYPE:nucleic acid
TOPOLOGY:linear
CLONE:HUMGS04066
SEQUENCE DESCRIPTION:
GATCTGAGGC TGTGTCTCTG CCTTGTTTTT AGAGGACTTC AGCGTCCAAG ACTGGGGCCC 60
ACCNTTNTCA CCAGCACTAA ATGCACTAAC AAGGACTCCA GACCTGCAGC CCCAGACCCG 120
CCGTAGTATA AGCCTAACAA GCAACANGTA GCACCTTAGT CTTTGTTCCA GGAGAGCTGA 180
GCAAGCTGGT GAAACCACTC TCCTTCCTTT AAACANCGTT TCAACCANCC TTTNCNTGGA 240
GCCAACNTGT AAAAAGTGGG TTGATTGCTG ACAGNATGGT CTTCCTTCCC TGGATTTNAG 300
ACATACCAGT TN 312

SEQ ID NO:3457
SEQUENCE LENGTH:309
SEQUENCE TYPE:nucleic acid
TOPOLOGY:linear
CLONE:HUMGS04067
SEQUENCE DESCRIPTION:
GATCTTTCCG GGGCCTACAG GCGTGTAAGA CAGCTTGGTC TGGTCTGTGC AGAAGTGGGG 60
AGTGATGGGC AGGTTCGGCA GCCTAACATT GTTCAGGCGC ATGGCCCCTG CGGTGTGTAC 120
ACGAACTCGG CTTCTTTTGT CCTAGGTACG CCAAGGGCAG GTTTCTGGAG ACTCCCTTGT 180
GCCCGGGATG GCAAGGGCAC CGGGCTGGCG TTTCCACATC TGTCTTCATT AGCAGANNAG 240
TGATGATGGA TTTTATTTCA CTCACACTCC AGTTTGTAAT AAAATGCCAA ATTCTGTCAG 300
CTATCCAAA 309

SEQ ID NO:3458

SEQUENCE LENGTH:309
SEQUENCE TYPE:nucleic acid
TOPOLOGY:linear
CLONE:HUMGS04068
SEQUENCE DESCRIPTION:
GATCAGCACC ATTTGTGGTA TGTTCCGTGA TGAGCGTTTA GTGAGCCTNN TGGCTGCAGA 60
GCACTATGAA ATCATGGTAC GTAGTCCCCG GCACCTGTCG TTATTCCTAT ATCCTCCTGC 120
AACTGTGGTT TGAAACTGCG CATTCTCTAG TAGTATATAT CGTGCCTGTC TTCAAAAACA 180
TTTCCCTTNN GTATACTCAT NCCCCCCAGG CATTGGGGTA GTGTCAGTCG GACTGCACAG 240
GGAACACGGT TTCCAGTGGC TTTGGCCCCT ACTCGGGAAA CGTCTGCCTG TTCTCGATNG 300
TGATGGGGN 309

SEQ ID NO:3459
SEQUENCE LENGTH:308
SEQUENCE TYPE:nucleic acid
TOPOLOGY:linear
CLONE:HUMGS04069
SEQUENCE DESCRIPTION:
GATCTAGCTG GTCACGACGA TGTTTTCACC AAGGTCACAG GAGCATTGCG TCGCTGATGG 60
GGTTGAAGTT TGGTTTGGTT CTTGTTTCAG CCCAATATGT AGAGAACATT TGAAACAGTC 120
TGCACCTTTN ATACGGTATT GCATTTCCAA AGCCACCAAT CCATTTTNTG GATTTAATGT 180
GTCTGTGGCT TAATAATCAT AGTAACAACA ATAATACCTT TTTNTCCATT TTGCTTGCAG 240
GAAACATACC TTAAGTTTTT TTTGTTTTGT TTTTGTTTTT TTGTTTTTNG TTTTCCTTTA 300
TGANGAAA 308

SEQ ID NO:3460
SEQUENCE LENGTH:351
SEQUENCE TYPE:nucleic acid
TOPOLOGY:linear
CLONE:HUMGS04070
SEQUENCE DESCRIPTION:
GATCAAGCAG AGAAANTAAT TAATTTCATG GGACTAAATG AACTAGTGAG GATAATATTT 60
NCATAATTNT TNATTTGAAA TTTTGCTGAT TCTTTAAATG TCTTGTTTCC CAGATTTCAG 120
GAAACTTTTT TCCTTTTAAG CTATCCACAG CTTACAGCAA TTTGATAAAA TATACTTTTG 180
TGNACAAAAA TTGAGACATT TACATTTCCT CCCTATGTGG TCGCTCCAGN NCTTGGGAAA 240
CTATTCATGG AGTATTTNTA TTGTATGGTA ATATAGTTAT TGCACAAGTT CANTAAAANT 300
CTGCTCTNTG TATAGCANGG NNNGGGTTGG GGGGGCGGGN GGNGGGGGGG N 351

SEQ ID NO:3461
SEQUENCE LENGTH:305
SEQUENCE TYPE:nucleic acid
TOPOLOGY:linear
CLONE:HUMGS04071
SEQUENCE DESCRIPTION:
GATCAGATAG TTTCCTGAGG CTAAAATGCC CCAACATGTA AAAGACTGCN ACAAGGGTTA 60

ATGAGCCAGG AACTGCGGAT GGTAACCTAC ATCTCTATAA TTACCATAGG ATATATATGA 120
GTACCATAGG ATTCTTTTGC ATAACTGTAG AAAGTACAGA CAACACCCTC CCGTGTTTGC 180
TATTAGAGTT TTTANTGTTG ATTGGAAAAT GGGAGATTCA CTGTGTGCCA TTCTTGGTAT 240
ACGTTGANGA AATTACATCA CTAGTACACA CNGNGGNNNT TCCACATTCA CACGCCCCAG 300
CTCTN 305

SEQ ID NO:3462
SEQUENCE LENGTH:303
SEQUENCE TYPE:nucleic acid
TOPOLOGY:linear
CLONE:HUMGS04072
SEQUENCE DESCRIPTION:
GATCAAGCTA CAGATATGGG ATACAGCAGG CCAGGAGCGA TTTCACACCA TCACAACCTC 60
CTACTACAGA GGCGCAATGG GTATCATGCT AGTATATGAC ATCACCAATG GTAAAAGTTT 120
TGAAAACATC AGCAAATGGC TTAGAAACAT AGATGAGCAT GCCAATGAAG ATGTGGAAAG 180
AATGTTACTA GGAAACAAGT GTGATATGGA CGACAANNGT GTTGTACCTA AAGGAAAAGG 240
TGANCAGATT GCNNGGGAGC ATGGTATTAG GTTTTTTGAG ACTAGTGCAA ANGCNGGTAT 300
AAN 303

SEQ ID NO:3463
SEQUENCE LENGTH:303
SEQUENCE TYPE:nucleic acid
TOPOLOGY:linear
CLONE:HUMGS04073
SEQUENCE DESCRIPTION:
GATCCGGTCA TACATCCACA TCAGGCCTGG CCAGCTGGAT GTTCTTAAAN AGTTCCACAA 60
GCAGATTACT GCACGGGCAA CAAAGGATGG GACTTTTGTC ATTTCAAAGA CATGATGTAT 120
GGGGATTAGA AAGAACTCAA GACACTCCTG CTTGATACAG AACAAAAAGA GCTTAACAGG 180
ACCAACAGGG CTTAAGCCCA GACTTGACGT AACAGAAATG TGCCAATAGG TAATAGGTAA 240
TTTTNCTTTC TCTGACTTGT TTTGTTTTCT TGAAATAACA CTGTTGTGTG GCTAGAAAGG 300
AAA 303

SEQ ID NO:3464
SEQUENCE LENGTH:348
SEQUENCE TYPE:nucleic acid
TOPOLOGY:linear
CLONE:HUMGS04074
SEQUENCE DESCRIPTION:
GATCTACTTT CACATTCTCA AGTTTTTNTC ATCTGCATTA GAGGTGCCCA GTAGGTTCCC 60
AGGTTCCAGC GTGCCCCTCC CTCAGACACA CGGACACAAT CAGCCGAGAA GTTCCTGGTC 120
TGAATCACGA GAATGTGGAG GGGTGGGGGG TGTCAGTGGA AAGGCATAAG GCTGAGCTGA 180
GACCAGTTGC TGGTGAAACT GGGCCAATCT GGGGAGGGGA ACATCCTTGC CAGGGAGTTT 240
CTGAGGGTCT GCTTTGTTTA CCTTTCGTGC GGTGGATTCT TTTTAACTCC GTCTACCTGG 300
CGTTTTGTTA GAAATGTCAG ATAGGAAAAT AAAAACCATT TGAGTAAA 348

SEQ ID NO:3465
SEQUENCE LENGTH:299
SEQUENCE TYPE:nucleic acid
TOPOLOGY:linear
CLONE:HUMGS04075
SEQUENCE DESCRIPTION:
GATCTGACCT GGTGAGATTA TTTCTNATGA CCTCATCAAA AAATAAACAN TTCCCAATGT 60
TCCAGGTGAG GGCTTTGAAA GGCCTTCCAA ACAGCTCCGT CGCCCCTAGC AACTCCACCA 120
TTGGGCACTG CCATGCAGAG ACGTGGCTTG GCCCAGAATG GCCTGTGCCA TAGCAACTGG 180
AGGCGATGGG GCAATGACAG ATACANCAGA ACANTGCTTT TGAGGCANGC TTCTTCCCTN 240
GNGGGTNGGT TGTNATGGCC NTGGCTTTTT NAGTTGGGNG NCATTTANAT TAAGGTTTN 299

SEQ ID NO:3466
SEQUENCE LENGTH:415
SEQUENCE TYPE:nucleic acid
TOPOLOGY:linear
CLONE:HUMGS04076
SEQUENCE DESCRIPTION:
GATCTACGTN ATCTCAGGGC AGTTCCTACA GATGAAGCAA GAGCTTTTNC AGAAAAGAAT 60
GGTTTGTNAT TCATTGAAAC TTCGGCCCTA GACTCTACAA ATGTAGAAGC TGCTTTTNAG 120
ACAATTTTAA CAGAGATTTA CCGCATTGTT TCTCAGAAGC AAATGTCAGA CAGACGCGAA 180
AATGACATGT CTCCAAGCAA CAATGTGGTT CCTATTCATG TTCCACCAAC CACTGAAAAC 240
ANGCCAAAGG TGCAGTGCTG TCAGAACATC TAAGGCATTT NTNTTCTCCC CTAGAAGGCT 300
GTGTATAGTC CATTTCCCAG GTNTGNGGAT TTAAATATAT TNGTAATNCT NGGTGTCACT 360
TTTGGTGTTT TATNACTTCA TACTTATGGN TTTTTCCATG NCCTAAGNCT TTTTN 415

SEQ ID NO:3467
SEQUENCE LENGTH:295
SEQUENCE TYPE:nucleic acid
TOPOLOGY:linear
CLONE:HUMGS04077
SEQUENCE DESCRIPTION:
GATCCACCTT CTTCCTTAGG TCCCCTCCTC CATCAGCAAA GGAGCACTTC TCTAATCATG 60
CCCTCCCGAA GACTGGCTGG GAGAAGGTTT AAAAACAAAA AATCCAGGAG TAAGAGCCTT 120
AGGTCAGTTT GAAATTGGAG ACAAACTGTC TGGCAAAGGG TGCGAGAGGG AGCTTGTGCT 180
CAGGAGTCCA GCCGTCCAGC CTCGGGGTGT AGGTTTCTGA GGTGTGCCAT TGGGGCCTCA 240
GNCTTCTCTG GTGACAGAGG CTCAGCTGTG GCCACCAACA CACAACCACA CACAN 295

SEQ ID NO:3468
SEQUENCE LENGTH:292
SEQUENCE TYPE:nucleic acid
TOPOLOGY:linear
CLONE:HUMGS04078
SEQUENCE DESCRIPTION:
GATCAAAGCA CTGGGCTTGT CCAGGCNCAT AATAAATGCT CCATTGAANG TACTATTCTN 60

GTTTTCCACT GCTGTGGAAA CCTCCTTGCT ACTATAGCGT CTTATGTATG GTTTAAAGGA 120
AATTTATCAG GTGAGAGAGA TGAGCAACGT TGTCTTTNCT CTCAAAGCTG TAATGTGGGT 180
TTNGTTTTAC TGTTTATTTG TTTGTNGTTG TATCCTTTTC TCCTNGTNAN TTGCCCTTCA 240
GAATGCACTT GGGAAAGGCT GGTTCCTTAG NNNCCTGGTT TGTGTCTNTT NN 292

SEQ ID NO:3469
SEQUENCE LENGTH:291
SEQUENCE TYPE:nucleic acid
TOPOLOGY:linear
CLONE:HUMGS04079
SEQUENCE DESCRIPTION:
GATCTCTTAA GAGATGATGG ACTTATGTAT GTTACAAGAC TTCGAAATGT TGGAGTCCAA 60
GTNGTTCATG AACATATTGA GGATGGAATT CATGGAGCTT TATCATTCAT GACTTCACCA 120
TTTTATTTAC GTCTAGGTCT TAGGATAAGA GATATGTATG TAAGTNGGCT GGATAAGANT 180
TTATAAATAT GTGATGTGTA TGTATAGCCC TTACATAGTG GATTGTAATT NGTGATATTT 240
TGTGGTTTTG GAGCAAAGAN CAATGTCATT TGAGTTATCT AAATCTACAT N 291

SEQ ID NO:3470
SEQUENCE LENGTH:290
SEQUENCE TYPE:nucleic acid
TOPOLOGY:linear
CLONE:HUMGS04080
SEQUENCE DESCRIPTION:
GATCTTTTGA TTCCTCTTGG GCTGAAGCAG ACCAAGTTCC CCCCAGGCAC CCCAGTTGTG 60
GGGGAGCCTG TATTTTTTTT AACAACATCC CCATTCCCCA CCTGGTCCTC CCNCTTCCCA 120
TGCTGCCAAC TTCTAACCGC AATAGTGACT CTGTGCTTGT NTGTTTAGTT CTGTGTATAA 180
ATGGAATGTT GTGGAGATGA CCCCTCCCTG TGCCGGCTGG TTCCTCTCCC TTTTCCCCTG 240
GTCACGGCTA CTCATGGAAG CAGGACCAGT AAAGGGACCT TCGATTAAAA 290

SEQ ID NO:3471
SEQUENCE LENGTH:287
SEQUENCE TYPE:nucleic acid
TOPOLOGY:linear
CLONE:HUMGS04081
SEQUENCE DESCRIPTION:
GATCCAAAGT CCTGTGGTTA ACGCCTTCAT TTATAGATGA GGCAGCTGAG GCCTGGGGAT 60
GTGAACAACC TGCTCACAGT CCTCATTTAC TGGATTTGAC TTCAGCCAGG TGAACTGGAA 120
TGCCTTGGGG CGTGGAAGGG CATTAGGAGT GTTTCATTTG ATATGTGAAT GCTCATAAAA 180
AAATGTCAAG GAATGAAGAA CAGCANCTCT CAGTGGTGCC TGCATTTATA ATTATTTATG 240
TGAAAGTCAA ATTCATGTAC AGTAAATTTG TTATAAGAAT ATTCAAA 287

SEQ ID NO:3472
SEQUENCE LENGTH:287
SEQUENCE TYPE:nucleic acid
TOPOLOGY:linear

CLONE:HUMGS04082
SEQUENCE DESCRIPTION:
GATCCAAAGT GCAAGGNTTT GATTATAAAC ATAATTTCCT AGACTGAAAG TTTTTGGAAA 60
AGATGCAGGG TCTGAGTCAG GCCTTCTGGT TATATTGTGC AGTTTCAAAA GAACTATTTA 120
ANACTCTTGA AAACTCATGT AAATAAAAAT CATAGGGTGA AAATTGTATT TGTTAAAATA 180
CCTTAATAAT TTAAAATGAC CTGATTTCCT GGAAAATTTT ATTATTCAAA AGGTGGAGGC 240
ATTGTAAAAA GGAAATAGTG ATGTAAATAA ACATGTTCTC TTTCAAA 287

SEQ ID NO:3473
SEQUENCE LENGTH:278
SEQUENCE TYPE:nucleic acid
TOPOLOGY:linear
CLONE:HUMGS04083
SEQUENCE DESCRIPTION:
GATCTGTTTG TCACTGACAG ACTGTAGTAG TGTCTGTGTG CTGACTGATA GATAGACTAT 60
AGTAAAATTT GGGTGTTGCC TGACTAACGG TCTAGGGTCT GTAAGCTGAC AGTCTGCCTG 120
CTTTCTGATT GTATCCATTG AAGTGTATGT ACATTATGGT AATNCTCTGT CTATTAAATG 180
TGTCTAACAA AGGAAGGAAT TAAGCACTCC ACATGTTTTC TTTATAGGGG AGTTCTGTAC 240
ACTATGATTT TAAATAGATA TTTCTTATAT AANGGAAA 278

SEQ ID NO:3474
SEQUENCE LENGTH:278
SEQUENCE TYPE:nucleic acid
TOPOLOGY:linear
CLONE:HUMGS04084
SEQUENCE DESCRIPTION:
GATCCCAACA CTGAAGGGGT AGAAGGCCAG GGGGGCATGG AGAGTGCAGC TCCATTATAG 60
TGAAGAGCCA AACATATGTN AACTGTTTGC TGTGTGGAGG TGTTAGTTCT GCTGCCTACC 120
ATCTTCATCT CTAGCACCTC CCCTGCCAAG AGTCAACCAC TAAGCAATCC CACCCAAGCC 180
TGGATGCTTC TAGAGGGGCC CACTCCCAGC TGGGAGAGTN TAGGGGATAT GCTCACACCA 240
CATTAGCAGC AACCAATAAA AATGCTGGAA ACAAGAAA 278

SEQ ID NO:3475
SEQUENCE LENGTH:277
SEQUENCE TYPE:nucleic acid
TOPOLOGY:linear
CLONE:HUMGS04086
SEQUENCE DESCRIPTION:
GATCTTAACT GTGTCATTTG AAACCCTGTG CGTTTGAAGT GGTGGAGACA GGACCTTTAT 60
CATATTCTGA GCTTTAAATA TATAAACNGG TAAATCATTC CATTTAATAA GACATAGCCC 120
AAGTCCCAGA ACGCGTTTTG AGAGTAACCA GAAACAGAGG TTATTTGTTC TGCTTTCTTT 180
TATCACTGCT ATTGGATGGG ATATCCCAAA CAAGTGGATG TGCGCCCCAA TTCTATTTTC 240
CACTATTCAA TGATTAAAAT AATTACAAAA ACGCAAA 277

SEQ ID NO:3476

SEQUENCE LENGTH:273
SEQUENCE TYPE:nucleic acid
TOPOLOGY:linear
CLONE:HUMGS04087
SEQUENCE DESCRIPTION:
GATCTTTCCA AATACCCAAA TGTCCCTGCA AGCCCGTTAA ATAATTCCCT ATGCTACCCT 60
TAATAACATA CAATGACCAC ATAGTGTGAG AACTTCCAAC AAGCCTCAAA GTCCCTTGAG 120
ACTCCCCAAT ACCTAATAAG GCATGCGAAA TGTTCTCATG AACTACCCCA CAACACGCCT 180
AAAACTCAAA ACACCCAAAA ATATNTCCTC CAATGTCCTG AAACATGAAC CCAAAAAGAG 240
ACCCACAATA AACTCGTGAC TTGTCCCNTC AAA 273

SEQ ID NO:3477
SEQUENCE LENGTH:272
SEQUENCE TYPE:nucleic acid
TOPOLOGY:linear
CLONE:HUMGS04088
SEQUENCE DESCRIPTION:
GATCCTCTCN ACCCTCCTCT AGCCATCCCT TGGGGAAGGG TGGGGAGAAN TATAGGATAG 60
ACACTGGACA TGGCCCATTG GAGCACNNGG GCCCCACTGG ACAACACTGA TTCCTGGAGA 120
GGTGGCTGCN CCCCCAGCTT CTNTCTCCCT GTNACACACT GGACCCCACT GGCTGAGAAT 180
CTGGGGGTGA GGNGGACAAG AAGGAGAGGA AAATNTTTCC TTGTGCCTGC TCCTGTACTN 240
GTCCTCAGCT TGGGGNTTCT TCCTCCTCCA TN 272

SEQ ID NO:3478
SEQUENCE LENGTH:270
SEQUENCE TYPE:nucleic acid
TOPOLOGY:linear
CLONE:HUMGS04089
SEQUENCE DESCRIPTION:
GATCTTGATA TTNGGCCTCT TTGAAGAGAC AGCCCTGGCT GCTTTCCTNN CCTACTGCCC 60
TGGAATGGGT GTTNCTCTTA GGATGTATCC CCTCAAACCT ACCTGGTGGT TCTTTGCCTT 120
CCCCTACTCT CTTCTCATCT TCGTATATNA CGAAGCAGAA AACTCATCAT CAGGCGACGC 180
CCTGGCGGCT GGGTGGAGAG GGAAACCTAC TATNAGCCCC CCGTCCTGCA CGCCGTGGAG 240
CATCAGGCCA CACACTCTGC ATCCGACACN 270

SEQ ID NO:3479
SEQUENCE LENGTH:267
SEQUENCE TYPE:nucleic acid
TOPOLOGY:linear
CLONE:HUMGS04090
SEQUENCE DESCRIPTION:
GATCACCAAC AGGGAAAGAC ATCCTGGTTC TTTTGATGTG GTGCATGTGA AGGATGCCAA 60
TGGCAACAGC TTTGCCACGA GGCTTTCCAA CATTTTTGTC ATTGGCAATG GCAATAAACC 120
TTGGATTTCC CTGCCCAGGG GAAAGGGCAT TCGACTTACT GTTGCTGAAG AGAGAGATAA 180
GAGGCTGGCC ACCAAACAGA GCAGTGGCTA AATTGCAGTA GCAGCATATC TTTTTTTCTT 240

TGCACAANTA AACAGTGAAT TCTCAAA 267

SEQ ID NO:3480
SEQUENCE LENGTH:263
SEQUENCE TYPE:nucleic acid
TOPOLOGY:linear
CLONE:HUMGS04091
SEQUENCE DESCRIPTION:
GATCCCTGGA GTGCCTTGGT GTTTCAAGCC CCTGCAGGAA GCAGAATGCA NCTTNTGAGG 60
CACCTCCAGC TGCCCCCGGC CGGGGGATGC GAGGCTCGGA GCACCCTTGC CCGGCTGTGA 120
TTGCTGCCAG GCACTGTTCA TCTCAGCTTT TCTGTCCCTT TGCTCCCGGC AAGCGCTTCT 180
GCTGAAAGTT CATATCTGGA GCCTGATGTC TTAACGAATA AAGGTCCCAT GCTCCACCCG 240
AGGACAGTTC TTCGTGCCTG AAA 263

SEQ ID NO:3481
SEQUENCE LENGTH:257
SEQUENCE TYPE:nucleic acid
TOPOLOGY:linear
CLONE:HUMGS04092
SEQUENCE DESCRIPTION:
GATCGGGAAC AAATGNAATC TGTGGATGCA TCAAGCTATC GTCAGTCAAA CCCCTTTAAA 60
ATGTTGCTTT GGCTTTGTAA ATTTAAATAT GTAAGTGCTA TATAAGTCAG GAGCAGCTGT 120
CTTTTTAAAA TGTCTCGGCT AGCTAGACCA CAGATATCTT CTAGACATAT TGAACACATT 180
TAAGATTTGA GGGATATAAG GGAAAATNAT ATGAATGTGT ATTTTTACTC AAAATAAAAG 240
TAACTGTTTA CGTTAAA 257

SEQ ID NO:3482
SEQUENCE LENGTH:255
SEQUENCE TYPE:nucleic acid
TOPOLOGY:linear
CLONE:HUMGS04093
SEQUENCE DESCRIPTION:
GATCTACATT TTGTACATAT TTATATAAAA TTTACCTTTA AGTATTTACT TTAAAAAATT 60
TAATGGCTTA ACTCGAACTT GAAGACACAT ACTTCAACTG TCCTTATTGT CCATTAANCT 120
GATAATTTTN ATTTTCCTNG CTTTTATAGA TTTNNCTATA TAGGAATCAA GATTTANGAA 180
ATTTTGCATT AAAANTAGTG TACCAATGCT TCATATACGT TAGTTATTTG CTATTATGTA 240
GGGAAGAGGA TTGTN 255

SEQ ID NO:3483
SEQUENCE LENGTH:255
SEQUENCE TYPE:nucleic acid
TOPOLOGY:linear
CLONE:HUMGS04094
SEQUENCE DESCRIPTION:
GATCTGAAAT NAGAAATATC CAACATTGCA CGAGTATCTT TNTTTATNCC TCCACAGACT 60

CCGCCAGAGA CACCTAGTCC TGATGAAACG TCTGCTCCTT GTCCTAATAT TCATATCAAC 120
AGCACCATTC CTGGCATTCA CATTTTAAAA ATTATGTGGA AGTGGATAGG AGAACTGCAG 180
CTGTCAATAG CCTAGGGCTG AATTTTTGTC AGNTAANTAA NATAAATCAT TCATCCTTTT 240
TTTTNGNTTT ATAAA 255

SEQ ID NO:3484
SEQUENCE LENGTH:253
SEQUENCE TYPE:nucleic acid
TOPOLOGY:linear
CLONE:HUMGS04095
SEQUENCE DESCRIPTION:
GATCTAGGTG GTTTGTAATT GTGCATTACT GACTGCATAT GTTTGTGTAT GTGTAAATGT 60
GGGCTCCCTG TTAAGTGGGG CTCATGGATA CGAGGCCTGA GGAAGTGTGG CTTGCTAGTC 120
TGTTACGTTA ACATGCTTTT CTAAAATTGC TTCACGTGTT AATTCATTTA CTCCTGCATT 180
CATTGACTGT TTTTGTTCTT TTCCATTCAC TTTGTACTTA TTTTTTTCAT TAAATTTTGC 240
ATTTATTTTG AAA 253

SEQ ID NO:3485
SEQUENCE LENGTH:251
SEQUENCE TYPE:nucleic acid
TOPOLOGY:linear
CLONE:HUMGS04096
SEQUENCE DESCRIPTION:
GATCCTAGTT CCTTCCTTCC CGAGTGATAC CCATGAACTG CCAGTAGAGG CTGCTATCGT 60
TCCATGTGTA AGGAATGAAC TGGTTCAAGG CGCGTCCTAC CCAGTCATTT TCTTTACCTT 120
ATACTAATTC TTCCTGAATA ATGTCTTCAG TTTCTTGAGG AGACTCCTAG TTTTGGTTTT 180
CAAATTACTT GGAGGGCTGC CTAGGAATCT ATCTCCCTCT GAAATAAAGT TTCCTCATCT 240
TCCACNNTAA A 251

SEQ ID NO:3486
SEQUENCE LENGTH:249
SEQUENCE TYPE:nucleic acid
TOPOLOGY:linear
CLONE:HUMGS04097
SEQUENCE DESCRIPTION:
GATCATACGG CCTTCCAGGA GACGCTGGGA TGTAAGATAC TCTTGGAGGN TGGCTCTGAA 60
CTCGGGTGTC TGAATCACAG GGCAAGGGTA GGCGTGGGCT GTGGCTAACA GCAGAGTCAC 120
TTCCCTGGCC TAGCTTGGGG ACCTGGGGAG GAGGAGGTAG CTCTGGACAC CAGGGTTCTT 180
CCCATTGTGG GAGAGTNAGT TTTTTAGGGG GAGACCTGTC CCCCAGATTA AAGAACTGGG 240
AGCCCTAAA 249

SEQ ID NO:3487
SEQUENCE LENGTH:248
SEQUENCE TYPE:nucleic acid
TOPOLOGY:linear

CLONE:HUMGS04098
SEQUENCE DESCRIPTION:
GATCAAAGAA GAGAGTAAAG CTATGTCTTC AATTTAATTT CAATACCTGA TTTGTACTTT 60
CCTTGAAAGC TTTACTTTAA AATACCTTAC TTATAGGCCT GGTGTCATGC TATAAGTATG 120
TACATACAGT TTCACTTCAA AAATAAAAAA AAANTCCCTA AAANCTCTCT ATACTCTCTA 180
TAACANTNCT TTATCANGAN CTCTGGNCAA TGGTATTATT TNGGANANTC ATGGTGATGT 240
ATTTNTNN 248

SEQ ID NO:3488
SEQUENCE LENGTH:245
SEQUENCE TYPE:nucleic acid
TOPOLOGY:linear
CLONE:HUMGS04099
SEQUENCE DESCRIPTION:
GATCTGTGTT GTAGAACATG AGGGTGTAAG CCTTCAGCCT GGCAAGTTAC ATGTAGAAAG 60
CCCACACTTG TGAAGGTTTT GTTTTACAAA TCACTTGATT TAACACACTC AGGTAGAATA 120
TTTTAATTTT TACTGTTTTA TACCCAGAAG TTATTTCTAC ATTGTNCTAC AGCAAGAATA 180
TTCATAAAAG TATCCCTTTC AAATGCCTTT GAGAAGANTA GANGAAAAAA AGTTTGTATA 240
TATTN 245

SEQ ID NO:3489
SEQUENCE LENGTH:246
SEQUENCE TYPE:nucleic acid
TOPOLOGY:linear
CLONE:HUMGS04100
SEQUENCE DESCRIPTION:
GATCCTGATN ATATTTGGCA GCTGTCCTCC AGTCTTAAAA GGTTTGATGA CAAATACACC 60
TTGAAGCTGA CCTTCATCAG TGGGAGAACA AAGCAGCAGC GGGAAGCCGA GTTCACAAAG 120
TCCATTGCTA AGTTTTTTTG ACCACAGTGG GACACTGGTC ATGGATGCAT ATGNGCCTGA 180
AATATCCAGG CTCCATGACA GTCTTGCCAT AGAAAGANAA ATAAAGTAGC CACTTCTANA 240
AGTAAA 246

SEQ ID NO:3490
SEQUENCE LENGTH:239
SEQUENCE TYPE:nucleic acid
TOPOLOGY:linear
CLONE:HUMGS04101
SEQUENCE DESCRIPTION:
GATCCTGGGT ACTGAAAGNC TTAGGGAAGC TGGCCTGAGA GGGGAAGCGG CCCTAAGGGA 60
GTATCTAAGA ACAAAAGCGA CCCATTCAGA GACTGTCCCT GAAACCTAGT ACTGCCCCCC 120
ATGAGGAAGG AACAGCAATG GTGTCAGTAT CCAGGCTTTG TACAGAGTGC TTTTCTGTTT 180
AGTTTTTACT TTTTTTGTTT TGTTTTTTTA AAGATGAAAT AAAGACCCAG GGGGAGAAA 239

SEQ ID NO:3491
SEQUENCE LENGTH:239

SEQUENCE TYPE:nucleic acid
TOPOLOGY:linear
CLONE:HUMGS04102
SEQUENCE DESCRIPTION:
GATCTGGGGA AAGGAGAGAC CATGGTGTGA ATGTAGAGAT GCCACCTANC TCTCTCTAAG 60
GCAGGCCTGT GGATGAAGGA GGAGGGTCAG GGCTGGCCTT CCTCTGTNCA TCANTCTGCT 120
AGGTTGGGGG CCCCNGNCCN NCCATACCTA CGCCTAGGGA GCCCGTCCTC CAGTATTCCG 180
TCTGTAGCAG GAGCTAGGGC TGCTGCCTCA GCTCCAAGAC AAGAATGAAC CTGGCTGTN 239

SEQ ID NO:3492
SEQUENCE LENGTH:244
SEQUENCE TYPE:nucleic acid
TOPOLOGY:linear
CLONE:HUMGS04103
SEQUENCE DESCRIPTION:
GATCTGTGTT TCCTCCCCAG TCATCTTTCT TGTTCCAGAG AGGTGGGGCT GGATGTCTCC 60
ATCTCTGTCT CAACTTTATG TGCACTGAGC TGCAACTTCT TACTTCCCTA CTGAAAATAA 120
GAATCTGAAT ATAAATTTGT TTTCTCAAAT ATTTGCTATG AGAGGTTGAT GGATTAATTA 180
AATAAGTCAA TTCCTGGAAT TTGAAAGAGC AAATAAAGAC CTGAGAACCT TCCAGAATCT 240
GAAA 244

SEQ ID NO:3493
SEQUENCE LENGTH:235
SEQUENCE TYPE:nucleic acid
TOPOLOGY:linear
CLONE:HUMGS04104
SEQUENCE DESCRIPTION:
GATCCATAAA AGATTGTATT TTTATTACTA TTTAAACAAG TGATTAAATT TAGTCTGCAC 60
ANNNAGCAAG GGTTCACATG CATTCTTTTA TACTGCTGGA TTTTGTTGTG CATCATTTAA 120
AACATTTTGT ATGTTTCTNC TTATCTGTGT ATACAGTATG TTCTTGAATG ATGTTCATTT 180
GTCAGGAGNN CTGTGAGAAA TAAACTATGT GGATACTGTC TGTTTATATT ATAAA 235

SEQ ID NO:3494
SEQUENCE LENGTH:233
SEQUENCE TYPE:nucleic acid
TOPOLOGY:linear
CLONE:HUMGS04105
SEQUENCE DESCRIPTION:
GATCGACTGT AAATAGAGGA CTTCTGGAAC ATTCCAAATA TTCTGGGGTT GAGGGAAATT 60
GCTGCTGTCT ACAAAATGCC AAGGGTGGAC AGGCGCTGTG GCTCACGCCT GTAATCCCAG 120
CACTTTGGGA GNCTGAGGTA GGAGGATTGA TTGAGGCCAA GAGTTAAAGA CCAGCCTGGT 180
CAATATAGCA AGACCACGTC TCTAAATAAA AATTANTAGG CCGGCCAGGC AAA 233

SEQ ID NO:3495
SEQUENCE LENGTH:243

SEQUENCE TYPE:nucleic acid
TOPOLOGY:linear
CLONE:HUMGS04106
SEQUENCE DESCRIPTION:
GATCATGGAG ACCTGGCTGG TAGCTGTAAC AGAGATGGTG GAGTCCAAGG AAACAGCCTG 60
TCTCTGGTGA ATGGNACTTT CTTTGGTGGA CACTTGGCAC CAGCTCTGAG AGCCCTTCCC 120
CTGTGTCCTG CCACCATGTG GGTCANATGT ACTCTCTGTC ACATGAGGAG AGTGCTAGTT 180
CATGTGTNCT CCATTCTTGT GAGCATCCTA ATNNNTCNNT TCCATTTTNA TGACAGGANG 240
AAA 243

SEQ ID NO:3496
SEQUENCE LENGTH:231
SEQUENCE TYPE:nucleic acid
TOPOLOGY:linear
CLONE:HUMGS04107
SEQUENCE DESCRIPTION:
GATCCAGAAC TGAGGTCACT GGGTTCTAGA ACNGNNACAT TTACCTCGAG GCTCTTCCAT 60
CCCCAAACTG TGCCCTGCCT TCAGCTTTGG TGAAAGGGAG GGCCCCTCAT GTGTGCTGTG 120
CTGTGTCTGC ACCGCTTGGT TTGCAGTTGA GAGGGGAGGG CAGGAGGGGT GTGATTGGAG 180
TGTGTCCGGA GATGAGATGA AAAAAATACA TCTATATTTA AGAATCCCAA A 231

SEQ ID NO:3497
SEQUENCE LENGTH:231
SEQUENCE TYPE:nucleic acid
TOPOLOGY:linear
CLONE:HUMGS04108
SEQUENCE DESCRIPTION:
GATCTGCAGC CAGGCCTTTC TGAAGGNGTT ATTCTGNTAA AAATGGTCTN AGTTNTCTGA 60
AAAGCCAGCT CTTGAACCTC TTCACAACAG TATCAACACT GGCTTCTCCC GGTTCATNTT 120
ATGCGTGCGA GAAGTCAGTG GTAACTNCTG CAGGGCTTAA TACATTAGTG GTAACTGGTT 180
TAAAAAACAA AGACTGTAAG CCTGTGTGTG CCACTGNNNG CTTCAACNGN N 231

SEQ ID NO:3498
SEQUENCE LENGTH:224
SEQUENCE TYPE:nucleic acid
TOPOLOGY:linear
CLONE:HUMGS04109
SEQUENCE DESCRIPTION:
GATCCCAGTT TGCAGCAAAC CCCACACCCC AGCTCACACA GCAAAAACAA TGGACAGGCC 60
CAGAGGGTGA AGCAAACAGT GTCCCTTCTG GCTGTNTTGG AGCCTCCCCA GTAACCACCT 120
ATTTATTTNA CCTCTTTCCC AAACCTGGAG CATTTATGCC TAGGCTTGTN AAGAATCTNT 180
TCAGTCCCTC TCCTTCTCAA TAAAAGCATC TTCAAGCTTG TAAA 224

SEQ ID NO:3499
SEQUENCE LENGTH:294

SEQUENCE TYPE:nucleic acid
TOPOLOGY:linear
CLONE:HUMGS04110
SEQUENCE DESCRIPTION:
GATCGGGAAC AAGCACGTTG TACCCTTGGC TGGACATGGC CAAGACACAA GGCATTCCAC 60
GGCGGCAAGC TGACCGCACA GCAGTCTGGC TTGATTTTCA GCCGTCATCA TTGGGTTCTG 120
TTTTGACAGC TCTGCTGCCC ATAGGGACTG CGACTGGGNC CAGGTCAACC ACGCCAGGGG 180
GGTGTCACCA GCCTTTTCTT TTTTNCTTTC TTTTNTTTTT TTCCCCCCTA AAGTTGTNNN 240
AAAGCNAANC CNTGGGAATA ATGGTTTNTT TGGANTNAAA CCCANNATTT CAGN 294

SEQ ID NO:3500
SEQUENCE LENGTH:221
SEQUENCE TYPE:nucleic acid
TOPOLOGY:linear
CLONE:HUMGS04111
SEQUENCE DESCRIPTION:
GATCCAAGTT TGATAACATT GGCATGAATG CCATGGCTAA CAAAGATAAT GCAAGTNTCA 60
AACAGCTTAG ATGGGAGGCT GAACGTGATG ACTGGCTACA CAACAGAGAT GCAAAANGTA 120
TCATCAGGAA AAGGAAACAT TTTAAAANGA AGAGGNTTAA AACCACTCAA AAANCTAAAA 180
NCCAAGGGAA ATGAGTTATT ANTGTAANTN ATAGNTTAAN N 221

SEQ ID NO:3501
SEQUENCE LENGTH:220
SEQUENCE TYPE:nucleic acid
TOPOLOGY:linear
CLONE:HUMGS04112
SEQUENCE DESCRIPTION:
GATCTAATTG TATTAGTATT GTGAATAATC ATGTGAAATG TTTTGAGACA GAGTACTATA 60
TTTGTGAATA TAATNNTATG GTTTTTTTCA CTTAGAACCT TTCTGTGTGG AAAACTAAGA 120
AAATTGCTTT CTGCTGTATA ATCTGGCATT CATTGTAGAT TAAAGCTTAT TTTNCTGTGA 180
ATAAAACGNN NNCAATAAAA TACTATTCTT TAAAATTAAA 220

SEQ ID NO:3502
SEQUENCE LENGTH:226
SEQUENCE TYPE:nucleic acid
TOPOLOGY:linear
CLONE:HUMGS04113
SEQUENCE DESCRIPTION:
GATCTGGACA CCCTGTCTCC TGAGGAGCGC CGGGCCAGGC TGCGGAAGTN TGAGGCTCAG 60
CTCCAGTCGA GGAAGGAGTA CGAGCAGGAG CTCAGTGATG ACTTGCATGT GGAGCGCTAC 120
CGACAGTTCT GGACCAGGAC CAAGAAGTGA CCGTGGCTCC AGCCACCCCG GGACATTGCT 180
AAGATGGGAG GGCTGTTCTT AAATNACTCG TTCTTGAAGC TGCAAA 226

SEQ ID NO:3503
SEQUENCE LENGTH:215

SEQUENCE TYPE:nucleic acid
TOPOLOGY:linear
CLONE:HUMGS04114
SEQUENCE DESCRIPTION:
GATCATGCCG AAAAAGTGGC GGAAAAGCTA GAAGCTCTCT CGGTGAAGAN GANNCCAAGG 60
AGGATGCTGA GGAGAAGCAA TAAATCGTCT TATTTNATTT NCTTTTCCTC TCTTTCCTTT 120
CCTTTTTTNA AAAAATTTTA CCCTGCCCCT CTTTTTCGGT TTGTTTTTAT TCTTTCATTT 180
TTACAAGGGA CGTTATATAA AGAACTGAAC TCAAA 215

SEQ ID NO:3504
SEQUENCE LENGTH:213
SEQUENCE TYPE:nucleic acid
TOPOLOGY:linear
CLONE:HUMGS04115
SEQUENCE DESCRIPTION:
GATCTNTTCT TAATGTACAT AGTGCTAACA TGAAGACCTT TTTNTGCACT ATATGCAAAC 60
AGGGTAACTA ACTAAAACAA AGCCACTTTC AATCTTCAAT CCTTGAAGGT ATATCTAGGT 120
TTATNACAGT AATTGTNTTT ACATTTNATG GTGCCTAGTA TTGACAAAAT GTTATTTCCC 180
TACATTAAAC ATGACTCCAT AGACCTTTTC AAA 213

SEQ ID NO:3505
SEQUENCE LENGTH:212
SEQUENCE TYPE:nucleic acid
TOPOLOGY:linear
CLONE:HUMGS04116
SEQUENCE DESCRIPTION:
GATCTAATGT AAAATCCAGA ACTTGGACTC CATCGTTAAA ATTATTTATG TGTAACATTC 60
AAATGTGTGC ATTAAATATG CTTCCACAGT AAAATCTGAA AAACTGATTT GTGATTGAAA 120
GCTGCCTTTC TATTTACTTG AGTCTTGTAC ATACATACTT TTTTATGAGC TATGAAATAA 180
AACATTTNAA NCTGAATTNN TTAACTNTGA AA 212

SEQ ID NO:3506
SEQUENCE LENGTH:211
SEQUENCE TYPE:nucleic acid
TOPOLOGY:linear
CLONE:HUMGS04117
SEQUENCE DESCRIPTION:
GATCAGAGAT GCTGTTCATC AGTCCCAACA AGATGGCCTA GAAATCGCAT TCTCACCTCG 60
CCTTGCTGCT GCTTTAATTC CAAGTTCTAT TTCTTCCCTT ATAGTTTTCT ATGGGAATGA 120
GGCGGATACA GGAAACACCC TATCTCCTCT GTATTTTTGT AGTGGAATTT CTATTTAAGG 180
GGCTCATTAA AGCATAGTAT TTATACACAA A 211

SEQ ID NO:3507
SEQUENCE LENGTH:209
SEQUENCE TYPE:nucleic acid

TOPOLOGY:linear
CLONE:HUMGS04118
SEQUENCE DESCRIPTION:
GATCGAGATN CAAAGCCACA TNCTCTCTCA CCAAGNTAAA CTATTTANAT TAATTGTTAG 60
CGGTGTTATG CTGCTTTCTT CTTTGACAAG GCCATGACTA AGAAGGCTCT AAGTAAAGGA 120
AGAATCATTT ATTCCATAGT CAGCTAACTG GTAGCAAAGC CTTCTNTTCC AAGACACCAA 180
GGGAGAGGGG AGGACATAAA TTATGCAAA 209

SEQ ID NO:3508
SEQUENCE LENGTH:208
SEQUENCE TYPE:nucleic acid
TOPOLOGY:linear
CLONE:HUMGS04119
SEQUENCE DESCRIPTION:
GATCCCCTCT TNAGTCCNCT GCCCCTTCCA AGGACACTAA TGAGCCTGGG AGGGTGGCAG 60
GGAGGAGGGG ACAGCTTCAC CCTTGGAAGT CCTGGGGTTT TTCCTCTTNC GCCCCTTGTG 120
GTTTCTGTTT TGTAATTTAA GAAGAGCTAT TCATCACTGT AATTATTATT ATTTTCTACA 180
ATAAATGGGA CCTGTGCACA GGAGGAAA 208

SEQ ID NO:3509
SEQUENCE LENGTH:322
SEQUENCE TYPE:nucleic acid
TOPOLOGY:linear
CLONE:HUMGS04120
SEQUENCE DESCRIPTION:
GATCCAAGCA TATAAAAAGC CTNTATATTT TTNAAAAACA CATCTTAACT CCACGCTTTA 60
CGATATTATA AAAGTTGAAT GGTTCCTCTT GGTAAGGATA TTTGCTTACA AGTGCTAGGA 120
AATAACTNNC TGATAGCTGC GTTAACATAC TTNGTTTTGC CTAGAGAGGG GCAATAAAAA 180
TGAACCAAAG GATATTTCCA GAAAGGATTA AGAAAGCTGT TTAAGANGGC CATGACTCTT 240
TAGGTGTGTA TGTGTACCTT TCAGCATCCT AGGAATTTTN ATACTAAAAG CAAAATGTTT 300
TTCCCAGTTA GTCTTCTTCA GN 322

SEQ ID NO:3510
SEQUENCE LENGTH:205
SEQUENCE TYPE:nucleic acid
TOPOLOGY:linear
CLONE:HUMGS04121
SEQUENCE DESCRIPTION:
GATCCGNCCA CCTNGGCCTC CCAAAGTGCT GGGATTACAG GTGTGAGCCA CCGTGCCCGG 60
CCTCTTTTAA TTTATNCCTA AAATATTACC TTGAGGCCAA ATNCTGCGCT TAAGGAGAAT 120
GTGCACCAAG TGCTGGGGTG GGGGCTGGTT ATAAACGAGG CCACAAATNA TGCTTGTAAA 180
TAAATTGTGT GGTTCAAATC TGAAA 205

SEQ ID NO:3511
SEQUENCE LENGTH:205

SEQUENCE TYPE:nucleic acid
TOPOLOGY:linear
CLONE:HUMGS04122
SEQUENCE DESCRIPTION:
GATCCCTCAC TTTATTTATA TTCCCACTAT AACCAGTAAG TTCATTTCAT AGGCCCTATC 60
ATGCATTAAT CATTGNATGN NAGNAGTTAA TGAAAACTTT TCCTGTTACA ACGCCCATTG 120
CCGGCAATGA ACGTACCAAA ACCGCCAAGG AAGTCATTGT TATTGCACAA TACATGAGGA 180
CCTGGAGCTT TTCCAAAAGC TTAAA 205

SEQ ID NO:3512
SEQUENCE LENGTH:204
SEQUENCE TYPE:nucleic acid
TOPOLOGY:linear
CLONE:HUMGS04123
SEQUENCE DESCRIPTION:
GATCTAAAAC GGCTATTTAA TATGTTACAA GGCACTTGAG TATGGTTGCA TGTCCAAATA 60
TAAATGTTTT TAAATTAACT CTAACATTTG GTTATAAAAG TTTANCCATA ATAATAGAAT 120
TTTTAAAACA CGNTTGTNTG GTTTAATCTT AGCATATTTT TCCAACTAAT AAAGCTCAGT 180
ATTCGTCTGN GTTCTCAGNG GAAA 204

SEQ ID NO:3513
SEQUENCE LENGTH:201
SEQUENCE TYPE:nucleic acid
TOPOLOGY:linear
CLONE:HUMGS04125
SEQUENCE DESCRIPTION:
GATCTCTCTC ATTCATTTCA ATGTATTTTT ACTTTAAGAT GAACCAAAAT TATTAGACTT 60
ATTTAAGATG TACAGGCATC AGAAAAAAGA AGCACATAAT GCTTTTGGTG CGATGGCACT 120
CACTGTGAAC ATGTGTACCC ACATATTANT ATGCAATATT GTTTCCAATC CTTNCTAATA 180
CAGTTTTTTA TAATGTTGAA A 201

SEQ ID NO:3514
SEQUENCE LENGTH:200
SEQUENCE TYPE:nucleic acid
TOPOLOGY:linear
CLONE:HUMGS04126
SEQUENCE DESCRIPTION:
GATCCAATTT ACATCCACAT TTTAGGTNCA ACAGCAAGAA GTTCAGAGAG AGATTTCCCA 60
ACCAGACATT GGGTCACTCA CTGGTCACCT TGCCAGTNCA TTTTATTAGA AGGGAATCTG 120
TTGTAGCAAA TGGGAATAAA CCTGGGTTTC TATAGACCCA GAACTGAAAA AATAAACATC 180
GTGCTGTTTT TAATTNGAAA 200

SEQ ID NO:3515
SEQUENCE LENGTH:209
SEQUENCE TYPE:nucleic acid

TOPOLOGY:linear
CLONE:HUMGS04127
SEQUENCE DESCRIPTION:
GATCTTAGTT TTCTTTTGTT TATTTCCCAG CTCATTTTTT TCTTCTGGTC AGTTTTTTTA 60
AGGGGGGGTG TTGTGGTTTT TTGTTTTTGT TTTGCTTCTG AGAAAGCATT TGCCTTTCTT 120
CCTCTCCCAA CATAACAATC GTGGTAACAG AATGCGACTG CTNGATTTAC CGATGTATTT 180
AATGTAAGTA AAAAAAGGAA AAAAAGAAA 209

SEQ ID NO:3516
SEQUENCE LENGTH:198
SEQUENCE TYPE:nucleic acid
TOPOLOGY:linear
CLONE:HUMGS04128
SEQUENCE DESCRIPTION:
GATCCATTCC CTGCAACTNA AGATTCTAAG GAACTGGGTT TCTCAGTATA CAATGGGAAT 60
GGTTGGNAGG AGGTAAAGAG TAGAAGACAG TATCAAGANT CCAGAGCCCA GCACCTGTAG 120
TCCTAACTAT NCANATTCCT TGAGCCCAGG AGTTTGAGTC CAGCCTGGAC AACATATTGA 180
GACCCCCATC TCTCTAAA 198

SEQ ID NO:3517
SEQUENCE LENGTH:191
SEQUENCE TYPE:nucleic acid
TOPOLOGY:linear
CLONE:HUMGS04129
SEQUENCE DESCRIPTION:
GATCATGAAT AGGAGCCCAT GCTAGAAGTA CATTCTCTCA GATTTGAACC AGTGAAATAT 60
GATGTATTTC TGAGCTAAAA CTCAACTATA GAAGACATTA AAAGAAATCG TATTCTTGCC 120
AAGTAACCAC CACTTCTGCC TTAGATAATA TGATTATAAG GNAATCAAAT AAATGTTGCC 180
TTAACTTCAA A 191

SEQ ID NO:3518
SEQUENCE LENGTH:191
SEQUENCE TYPE:nucleic acid
TOPOLOGY:linear
CLONE:HUMGS04130
SEQUENCE DESCRIPTION:
GATCCAGGCA GCCAAGAACT TGATGAATGC TGTGGTGCAG ACAGTGAAGN CATCCTACGT 60
CGCCTCTACC AAATACCAAA AGTCACAGGG TATGGNTTCC CTCAACCTTC CTGCTGTGTC 120
ATGGAAGATG AAGGCACCAG NGNNAAAGCC ATTGGTGAAG AGAGAGAAAC AGGATGAGAC 180
ACAGACCAAG N 191

SEQ ID NO:3519
SEQUENCE LENGTH:187
SEQUENCE TYPE:nucleic acid
TOPOLOGY:linear

CLONE:HUMGS04131
SEQUENCE DESCRIPTION:
GATCTCCCCC AAAGATTCCA GGCGGTGCAA CCCTGCNTGT TCGAGGTGGA GCTGCTCAAA 60
ATAGAGCGAC GAACTGAGCT GTAACCAGAC TGGGGAGGGG CAGGGGGAGA GGCCCCCATC 120
AGGGACCAGA CTGTTCCAGA AAAAAAACAA AAAACANAAA CAAACAAAAA AACACTTAAA 180
AGCCAAA 187

SEQ ID NO:3520
SEQUENCE LENGTH:184
SEQUENCE TYPE:nucleic acid
TOPOLOGY:linear
CLONE:HUMGS04132
SEQUENCE DESCRIPTION:
GATCGAGACT CTNCCAGAAA ACAAAACAAA ACAAAAAAAA ATCCGTTTCT AAGGACGGCG 60
GGGATTCGGT TCGAGCCCGT GGCTGCTCTG TTTTGTTTTG CTTTCTTGGT ATATAATCCT 120
ANAAATAAAA GGGTAATATA AATATTTNAG AAGAGGGAAA TTGCTGAAGA GTAGTAAGGG 180
CAAA 184

SEQ ID NO:3521
SEQUENCE LENGTH:177
SEQUENCE TYPE:nucleic acid
TOPOLOGY:linear
CLONE:HUMGS04133
SEQUENCE DESCRIPTION:
GATCAGATGA CCATTGAGGA CTTGAATGAA GCTTTCCCAG AAACCAAATT AGACAAGAAA 60
AAGTATCCCT ATTGGCCTCA CCAACCAATT GAGAATTTAT AAAATTGAGT CCAGGAGGAA 120
GCTCTGGCCC TTGTATTACA CATTCTGGAC ATTAAAAATA ATAATTATAC AGTTAAA 177

SEQ ID NO:3522
SEQUENCE LENGTH:220
SEQUENCE TYPE:nucleic acid
TOPOLOGY:linear
CLONE:HUMGS04134
SEQUENCE DESCRIPTION:
GATCCCATCC GAAGCTCAGG GTATGGAGAG CGGGGAGCTC CNCCAAAGAT TCCAGGCGGT 60
GCANCCTGGT GTTCGAGGTG GAGCTGCTCA AAATAGAGCG ACGAACTGAG CTGTAACCAG 120
ACTGGGGAGG GGCAGGGGGA GAGGCCCCCA TCAGGGACCA GACTGTTCCA AAAAAAAANC 180
AAAAANCAAA ANCAAACAAA AAAACACTTA AAAGCCCAAA 220

SEQ ID NO:3523
SEQUENCE LENGTH:172
SEQUENCE TYPE:nucleic acid
TOPOLOGY:linear
CLONE:HUMGS04135
SEQUENCE DESCRIPTION:

GATCCTACCT AAGGGGCACA ACTAGTAAGG GAGTAGAGCT GAGATTGGCT CCAGAGTTCT 60
TGTTTTCAGC CACCATTTTA TGCTGCCTCT CCAGTNATTT TTTTTCCCAT TTTCTTCAGT 120
NTTTCTATAG CAGAGATTTG ATAATAAAAA GAAAATCCTA AACAATGAGA AA 172

SEQ ID NO:3524
SEQUENCE LENGTH:169
SEQUENCE TYPE:nucleic acid
TOPOLOGY:linear
CLONE:HUMGS04136
SEQUENCE DESCRIPTION:
GATCGCACTG CGCAGGGGTG CCACGGAGCT CACCCACGAG GACTACATGG AAGGCATCCT 60
GGAGGTGCAG GCCAAGAAGA AAGCCAACCT ACAATACTAC GCCTAGGGCA CACAGGCCAG 120
CCCCAGTNTC ACGGCTGAAG TGCGCAATAA AAGATGGTTT AGGGTCAAA 169

SEQ ID NO:3525
SEQUENCE LENGTH:169
SEQUENCE TYPE:nucleic acid
TOPOLOGY:linear
CLONE:HUMGS04137
SEQUENCE DESCRIPTION:
GATCTCAGGC ATCGGATTAT TTCTCCTGTA AATATTTCAG AATGTATCTN TCCAAGATGA 60
GAGCTCATTA AAAGACAATT ACAAAGCTTA TCACATCCAA AAGANTTATC AATAATTTTN 120
AAATATTATT AANCGTGTAA TAAATNTNCA AAGTNCCACT TGCCTTAAA 169

SEQ ID NO:3526
SEQUENCE LENGTH:165
SEQUENCE TYPE:nucleic acid
TOPOLOGY:linear
CLONE:HUMGS04139
SEQUENCE DESCRIPTION:
GATCAAAANG AGTNAANCTA TCCAAAAAAA ATTTGTATAT TTGGGAAAAA TGGATTTTTA 60
CATAGCTTTT GTATGCAGAT ATAAAACTGT AATTATGAAT ATAGTGGGCG TAGATAAACT 120
CATAAGCTTA AATTCTAAAA ANGAAAAAGC TTATACCANG ATAAA 165

SEQ ID NO:3527
SEQUENCE LENGTH:165
SEQUENCE TYPE:nucleic acid
TOPOLOGY:linear
CLONE:HUMGS04140
SEQUENCE DESCRIPTION:
GATCAGCCGA TGACAGAGAT TGTAAGCCGT GTGTCGAAGC GAAAGTTGGA GCCGCCACGT 60
GCGGGCNCTG GTGCTTNANC TGTGCTGTAA CGACGAGAGC GGCGAGGATG TCGAGNTTCC 120
CTATGTCCGA NACACCATCC GCTGACCCCG TNTGNTCCTN TAGGN 165

SEQ ID NO:3528

SEQUENCE LENGTH:165
SEQUENCE TYPE:nucleic acid
TOPOLOGY:linear
CLONE:HUMGS04141
SEQUENCE DESCRIPTION:
GATCCATTTG AAAGTNATTT TGACATAAGG AGTAAGGTTA CTACTGATTC CTACTCAGGA 60
GGCTAAANTG GGAGGACCAC TTAAGTATGG NAGGTCGAGG CTATAGTGAG CCATTACTGA 120
GCCACTGCAC TACAATCTGG GCGACANAGC AAGACTCTGT CTAAA 165

SEQ ID NO:3529
SEQUENCE LENGTH:161
SEQUENCE TYPE:nucleic acid
TOPOLOGY:linear
CLONE:HUMGS04142
SEQUENCE DESCRIPTION:
GATCCTGTGT TTGCAACTGG GGAGACAGAA ACTGTGGTTG ATAGCCAGTC ACTGCCTTAA 60
GAACATTTGA TGCAAGATGG CCAGCACTGA ACTTTTGAGA TATGACGGTG TACTTACTGC 120
CTTGTAGCAA AATAAAGATG TGCCCTTATT TTACCTACAA A 161

SEQ ID NO:3530
SEQUENCE LENGTH:161
SEQUENCE TYPE:nucleic acid
TOPOLOGY:linear
CLONE:HUMGS04143
SEQUENCE DESCRIPTION:
GATCAGTCAT CCACCAGGAA CGAAGATTTC CTGAAGAAGA CCTGGTCCCT CTGGAGGTTG 60
CGGTGGCTGA AGGATGCATC ATGTGCTCCT ACCCTGCTCT ACCGCTTTNT TGGGTCACAG 120
AGGCCAAATG TGAGAGCATT GAATAAATAT CTTAAGCTAA A 161

SEQ ID NO:3531
SEQUENCE LENGTH:159
SEQUENCE TYPE:nucleic acid
TOPOLOGY:linear
CLONE:HUMGS04144
SEQUENCE DESCRIPTION:
GATCTCGTGA AGCCCGCAAG GACCGAACAC CCCCACCCCG ATTTAGACCT GCGGGTGCTG 60
CCCCACGTCC CCCACCAAAG CCCATGTAAG GAGCTGAGTT CTTAAAGACT GAAGACAGGC 120
TATTCTCTGG AGAAAAATAA AATGGAAATT GTACTTAAA 159

SEQ ID NO:3532
SEQUENCE LENGTH:386
SEQUENCE TYPE:nucleic acid
TOPOLOGY:linear
CLONE:HUMGS04145
SEQUENCE DESCRIPTION:

GATCCTTACA GTAGAGGTGA CCCCTGTAAT ACCATCTGCT GCCGTGAGGA CCTGAACTCA 60
CCTAACCCAA GTCCTGGAGG TTNNNNNGAC ACAAAGGTGG CAGATATCTA CCTAGCATCT 120
CAGTACACAT CCTATGCCAT AAGTGGTCCC ACAGTACAAG GTGGCCTCCC TGTTTTTCGC 180
TGGGACCGTT TCAACAAAAC TCTACATCAG GGCATGCCAG AGGTCTACAA CTTTGATTTT 240
ATTACCATGA AACCAATTTT GAAACTTGAT ATAAAATGNA GGAGGGAGAT GACGGACTAG 300
AAGACTGTAA ATAAGATACC AAAGGCACTA TTTTAGCTAT GTTTTTCCCA TCAGAATTAT 360
GCAATAAATT TNTTAATTTT GTCAAA 386

SEQ ID NO:3533
SEQUENCE LENGTH:391
SEQUENCE TYPE:nucleic acid
TOPOLOGY:linear
CLONE:HUMGS04146
SEQUENCE DESCRIPTION:
GATCAGATTA AACCATTGAA GTTGGAAGTT CATNAGGCTA AGCCTGTNCC AGAAAATCAC 60
CCACAGTGGG ATACAGCAAT AGAGGGGGAT GAAGACCAGG AGGACAGTGA GGGCTTTGAA 120
GATAGCTTTN AGGAAGAAGA GGAGGAAGAA GAAGATGATG ACTAAGCAGT ACTCTGAATG 180
GNCCACAGTG TTTGCACATA TTTNCAATTT TTTNCTGTTT TGGAAGTGTA TCATAAACCA 240
GAAACAGTAC AGAACTGATG TTGNGGGNGG TGTNGTTTTT TTACTCTAGA NATGGGTGCA 300
TAATATNACT AGGCAGTGGC GGTGCCTTGG TNCANCCTGN AAAATGTTAA GGCTTTNTTG 360
AAACCTTTCC AGTNGGGGNT GGTCCNTTNA N 391

SEQ ID NO:3534
SEQUENCE LENGTH:157
SEQUENCE TYPE:nucleic acid
TOPOLOGY:linear
CLONE:HUMGS04147
SEQUENCE DESCRIPTION:
GATCCGCCCG ACTCGGCCTC CCAAAATGCT GGGATTACAG GCGTGAGCAT TCAAGTCTGG 60
CGAGAGAGAT TGTTTCTAGA TGAGGGTGGG GGCGGGTGTC CTTAGCCCAA AGCTTGTGCC 120
AGTCTCTATC GGAAATAAAT GCCCCCAAAA CCTCAAA 157

SEQ ID NO:3535
SEQUENCE LENGTH:154
SEQUENCE TYPE:nucleic acid
TOPOLOGY:linear
CLONE:HUMGS04148
SEQUENCE DESCRIPTION:
GATCTACAGC AAAACACAGC GGGAGAGGTT CGCCTGGGCT CTCGCCATGG CAGGAGAAGA 60
CTTTGAATTC TAACGACGAG CCGTGTTGAA ACTTCTTTTG TATGTGTGTG TGTTTTTTTC 120
ACTATTAAAG CAGTACTGGG GAATTTTGTA CAAA 154

SEQ ID NO:3536
SEQUENCE LENGTH:151
SEQUENCE TYPE:nucleic acid

TOPOLOGY:linear
CLONE:HUMGS04149
SEQUENCE DESCRIPTION:
GATCTAGGCA ATGATTTAGA GGAAACCTCN TCACAAAAAC CCCAGAGGAA GAATATGACC 60
TGTGTTTCAA ATTTTGCCTT TCTGAACTAT TTAAAATTAT GTGTATGTTT TTGCTTTGTA 120
TTTTAAAAGT AAAGCTGCTG TCCTCAAGAA A 151

SEQ ID NO:3537
SEQUENCE LENGTH:355
SEQUENCE TYPE:nucleic acid
TOPOLOGY:linear
CLONE:HUMGS04150
SEQUENCE DESCRIPTION:
GATCTATGAA AGCGGGGAGA GGGAACCTGA ATATTAATTA TGAGCACAAA TTTGAAGGAA 60
AGAAAACAAA GAACCATTAT CTAATCAAGC TTTGAAAGTC CTGCATGTTT GCCTTTTATT 120
TTAGTGTTGA CGCCAACATA GACTGTCTAA GGTATTTTTT TCCCCAAACA CTTGAATCTT 180
GGTCGTTGGT ATGTAATCCA CTCTCTAGAG TCCAGTGTAC TTTAGACTTC ATCTGAGTCC 240
AATATATGTA CCACACTACT GTTTTATTAA TGTAAAAACC TTGTAAATGA ATTTCAGATG 300
GGTGATTTAA GTGAGTCACA AGTCACAAAA CTTTGCTATT CATAGTTTAT CTNGN 355

SEQ ID NO:3538
SEQUENCE LENGTH:148
SEQUENCE TYPE:nucleic acid
TOPOLOGY:linear
CLONE:HUMGS04151
SEQUENCE DESCRIPTION:
GATCTCTGCT TCCTGTACCT TGACATGCAA AAGGCTCTCC TAATACTCCA CATTCAAACT 60
GAAGAGGAAA ATTGAAATCT CTAATGAAGC TGCTGTGTGT ATTTATGANT ATTAATGAAT 120
AAAAACTGCT TGGATGGTTT ACCTTAAA 148

SEQ ID NO:3539
SEQUENCE LENGTH:148
SEQUENCE TYPE:nucleic acid
TOPOLOGY:linear
CLONE:HUMGS04152
SEQUENCE DESCRIPTION:
GATCAACAAC TNGNTACTCT CGGGAAGACT CCTCTACTCA CAGCTGAAGA AAATGAGCAC 60
ACCCTTCACA CTGTTATTAC CTATCCTGAA GATGTGATAC ACTGAATGGA AATAANTAGA 120
TGTAAATAAA ATTNAGTTCT CATTTAAA 148

SEQ ID NO:3540
SEQUENCE LENGTH:147
SEQUENCE TYPE:nucleic acid
TOPOLOGY:linear
CLONE:HUMGS04153

SEQUENCE DESCRIPTION:
GATCAGAGTA AACAAAAGGA ATCTCAAAGG AAATTTGAAG AAGAAACTGT AAAATCCAAA 60
GTGACTGTTG ATACTGGAGT AATTCCTGCC TCTGAAGAGA AAGCAGAGAC TCCCACAGCT 120
GCAGAAGATG ACAATGAAGG AGACAAA 147

SEQ ID NO:3541
SEQUENCE LENGTH:146
SEQUENCE TYPE:nucleic acid
TOPOLOGY:linear
CLONE:HUMGS04154
SEQUENCE DESCRIPTION:
GATCGTGGCG CATGGCTTGG GGATTAAACT ACCCTTGANG AGGACCCTTG TCCCAAACCC 60
TTCTTGTTCT CTCCTCCAAA AGTAGCTTCC TCCAACCCGC AGCCTCTCTG CACACTAATA 120
AAACATGTGG CTTGGAAAGG TTCAAA 146

SEQ ID NO:3542
SEQUENCE LENGTH:144
SEQUENCE TYPE:nucleic acid
TOPOLOGY:linear
CLONE:HUMGS04155
SEQUENCE DESCRIPTION:
GATCTTTAAG GACAAGTTTA TCCAGCATCC AAAAAACTTT GGACTAATTG CATCATACTT 60
GGAGAGGAAG AGTGTTCCTG ATTGTTTTTT GTATTACTAT TTAACCAAGA AAAATNAGAA 120
TTATAAAGCC CTCGTCAGAA GGGN 144

SEQ ID NO:3543
SEQUENCE LENGTH:140
SEQUENCE TYPE:nucleic acid
TOPOLOGY:linear
CLONE:HUMGS04156
SEQUENCE DESCRIPTION:
GATCTGGGTT TTNATATCTT TATCTTCATT CAGTGAAGCA AGCCAAAAGC NCACATTTGT 60
ATGCNTTAGG TCTTCTTAAA ATGGTATCTG TAAACATGTG TCCAATATAA AANCTATTTT 120
NAATAAAANG TTACATGAAA 140

SEQ ID NO:3544
SEQUENCE LENGTH:138
SEQUENCE TYPE:nucleic acid
TOPOLOGY:linear
CLONE:HUMGS04157
SEQUENCE DESCRIPTION:
GATCAGCAAT GAAGAACTGG NCGGACAAAA TTNAACGTTN ATGTAATGGN ATTCCAGATG 60
TAGGCATTCC CCCCAGGTCT TTTCATGTGC AGATTGCAGT TCTNATTCAT TTGAATAAAA 120
AGGAACTTGG AAANCAAA 138

SEQ ID NO:3545
SEQUENCE LENGTH:137
SEQUENCE TYPE:nucleic acid
TOPOLOGY:linear
CLONE:HUMGS04158
SEQUENCE DESCRIPTION:
GATCTAAATG GGCCCAGGTG TTTACCCTTA TNTNGTTTTC TGCTAAATNA TGGAGGTTTG 60
GGGAGTTCCT TCTCCACTTG TTTGTGGAGG CCTGGGGAGT TTTTNNAGAC CCCCAGTAAA 120
ACTNGTTTAA TCCTAAA 137

SEQ ID NO:3546
SEQUENCE LENGTH:136
SEQUENCE TYPE:nucleic acid
TOPOLOGY:linear
CLONE:HUMGS04159
SEQUENCE DESCRIPTION:
GATCCCTGTA GAGGTGGTAT TAAAGATGGT CACTGAGATT AAGAAGATTC CTGGTATTTC 60
TCGAATTATG TATGACTTAA CATCAAAGCC CCCAGGAACT ACTGAGTGGG AGTAATAAAC 120
TTCTTGTTCT ATTAAA 136

SEQ ID NO:3547
SEQUENCE LENGTH:135
SEQUENCE TYPE:nucleic acid
TOPOLOGY:linear
CLONE:HUMGS04160
SEQUENCE DESCRIPTION:
GATCCTAAAA TNNTCTACTT TTGTNGCCTT ATCAGTNCTT TGCAATCTGC CTGTGGTTAT 60
CAGCACTTAA AGCACAATTT TGAAGGGGAA AAAAATGATA ATCACCTTAG TCCCAAAGAA 120
ATAATTTGTC ANNCN 135

SEQ ID NO:3548
SEQUENCE LENGTH:134
SEQUENCE TYPE:nucleic acid
TOPOLOGY:linear
CLONE:HUMGS04161
SEQUENCE DESCRIPTION:
GATCTGGACT GGCTGGGAGT GGGGAGGGCG TGGAGACAGT CTACGGAAAG CGCTANAGGA 60
CCCCCGAGAG GGTGCAGTGG AGCCCTGAGC ATTGTAATAT GCGGCCCAGC CTATAAACAG 120
CCTCCGTGCT TAAA 134

SEQ ID NO:3549
SEQUENCE LENGTH:134
SEQUENCE TYPE:nucleic acid
TOPOLOGY:linear
CLONE:HUMGS04162

SEQUENCE DESCRIPTION:
GATCCTTTGC TGTAAACTNG GAGAGACCAG TCCCAAACAG AGGGGAATTT TAAGCCCTTC 60
TCATCACCCA ATTGGATGTT TTTGCTTATA GCAAATTCCT GCAAAATAAA TAAATAAATA 120
TTTGCAAAAC TAAA 134

SEQ ID NO:3550
SEQUENCE LENGTH:133
SEQUENCE TYPE:nucleic acid
TOPOLOGY:linear
CLONE:HUMGS04163
SEQUENCE DESCRIPTION:
GATCTATGGG AAGATACTTA TTTTTCTGAG GTCCTTATGT CCTGTCATAT AATTAAAGAC 60
TCAAGAGAAT TTATGTGAAA TGCTTTCTGT ATGCCCAAAT CTTTAGATTA AAATTATATA 120
GCTGCTCCTG AAA 133

SEQ ID NO:3551
SEQUENCE LENGTH:132
SEQUENCE TYPE:nucleic acid
TOPOLOGY:linear
CLONE:HUMGS04164
SEQUENCE DESCRIPTION:
GATCTCAGTT CCNTAAAAAA TGATAAAAAT TTTTCATGTA TATTACATAT CTACCTACCA 60
GAAATCCTTT TTACAATTTT ATATACAATC ATTTTAAGAC CTTTTTGAAA TAAATAATGC 120
AAATGAACTA AA 132

SEQ ID NO:3552
SEQUENCE LENGTH:154
SEQUENCE TYPE:nucleic acid
TOPOLOGY:linear
CLONE:HUMGS04165
SEQUENCE DESCRIPTION:
GATCACGGGG TGGNAGGNAA CCGNGGCGTC CCTGCNTGGG GCCCATGGGT GANACACTCC 60
AGTACTGAGA CCTAGAGTCC AGATGCTTGT AGGAGCCAAG TCGTGTTCTA AGTATTTATT 120
TAAAACAAAA GAATCACGTT TTCCCATTTG TAAA 154

SEQ ID NO:3553
SEQUENCE LENGTH:213
SEQUENCE TYPE:nucleic acid
TOPOLOGY:linear
CLONE:HUMGS04166
SEQUENCE DESCRIPTION:
GATCCTTAAT TGTAGCCTTT GAAGGATGCC CTGTGTAGAT TATTCAGTGC CACAAATTGA 60
AAGCTTCCAT GTTTAATNTT ATCCTCTTGC TATATAAATA AAGCAAATAT ATTTAGGCCA 120
GGGTCTCACT GAGGGGGAGC TGTCTTGTCA TCTTTTAGAG TAAACTATTC TATAAACATA 180
TGCAAACAGC CCTAAATAAA TCTAAAGTCT AAA 213

SEQ ID NO:3554
SEQUENCE LENGTH:125
SEQUENCE TYPE:nucleic acid
TOPOLOGY:linear
CLONE:HUMGS04167
SEQUENCE DESCRIPTION:
GATCCCAAGG GCCCAGATAA CGCCCCCAAC TTCTTGGGGT TCACACGCGT CCTGAGACAT 60
CTGGGAACTG CCTCTGCCTG GNCCAAAGCT ATTCCATTAA AACTTTATTG CTGTNCTTAC 120
TCAAA 125

SEQ ID NO:3555
SEQUENCE LENGTH:127
SEQUENCE TYPE:nucleic acid
TOPOLOGY:linear
CLONE:HUMGS04169
SEQUENCE DESCRIPTION:
GATCTGTGTA CAATTTTNGT CACTANGACT TTCCTCCAAG AACTAAGCCA ACTTGATGTN 60
NCAAAGCACA GCTGTATATA ATGGTGATGT CATAATAAAG TTGTTTTATC TTTTAAGTAA 120
AAGTAAA 127

SEQ ID NO:3556
SEQUENCE LENGTH:126
SEQUENCE TYPE:nucleic acid
TOPOLOGY:linear
CLONE:HUMGS04170
SEQUENCE DESCRIPTION:
GATCAAGGAC TCTGGATAAT TGGCATAACA TCCTGGAATA GCTGAAACAG AGATATTATT 60
CTCTGCTGTC CTCTGTTGTC TTTGTCTTTT CACGTCTTAA TAAAAGTGCT GGTGACAAGA 120
GTGAAA 126

SEQ ID NO:3557
SEQUENCE LENGTH:123
SEQUENCE TYPE:nucleic acid
TOPOLOGY:linear
CLONE:HUMGS04171
SEQUENCE DESCRIPTION:
GATCAGAGCC ATACCCCTCT TCACCAATGG GAAGTCAAGA CACCTATGGG TAAAAGTTCT 60
CTCTGATGCA TGAAAACTCA GAGTGATAAA GTAATTTTAA AATAAAAATT TGGAGAAAGC 120
AAA 123

SEQ ID NO:3558
SEQUENCE LENGTH:121
SEQUENCE TYPE:nucleic acid
TOPOLOGY:linear

CLONE:HUMGS04172
SEQUENCE DESCRIPTION:
GATCTAAAAG TTTTCACACA TAAATTTCAT TAATTTGAAA GATTATTTTT AAACTGCATA 60
GGTCCACTTG CCTCTAAATA AAATNAACCT AAAATAAAAA CAAACTATAC TTTGTGAGAA 120
A 121

SEQ ID NO:3559
SEQUENCE LENGTH:121
SEQUENCE TYPE:nucleic acid
TOPOLOGY:linear
CLONE:HUMGS04173
SEQUENCE DESCRIPTION:
GATCTCCCCC AACCCCACAA AATGCTTAAA AGATAACTTA GCTCTTTNTT CAGGGCTCAG 60
TCCTTTGGAT GTAATCCGAC TAGGCCAGTN AACCTAAATA ATAAATATCC TCCTCAACAA 120
A 121

SEQ ID NO:3560
SEQUENCE LENGTH:120
SEQUENCE TYPE:nucleic acid
TOPOLOGY:linear
CLONE:HUMGS04174
SEQUENCE DESCRIPTION:
GATCCTGAGT TTTCTGGGAC AATTCCAGCT TTAATCAATA CATTTTGTTA AATGTGCCAT 60
AAAATGAGAC TTTTTACGCC TTTATAAGGC CTTAGATGTA AATAAACTCA CCCAAACAAA 120

SEQ ID NO:3561
SEQUENCE LENGTH:119
SEQUENCE TYPE:nucleic acid
TOPOLOGY:linear
CLONE:HUMGS04175
SEQUENCE DESCRIPTION:
GATCATTGTA TTCAGAGATT GTAAATGAAA AAATATAGAA ACTATTTAGT TTTGGTAGAT 60
TTTTTTTCTG ACAATGTGAC CAGACTGAAT TTCCTCATAA AGAAAAAATG GCGTGCAAA 119

SEQ ID NO:3562
SEQUENCE LENGTH:118
SEQUENCE TYPE:nucleic acid
TOPOLOGY:linear
CLONE:HUMGS04176
SEQUENCE DESCRIPTION:
GATCCCAGCA TTTTCCATCG ACTTGTAATT GTTTCTGCTA CCTGACAATC ATCGCCTTGA 60
GTACTGGGAC AACCTTTGAT TACTCATTAT ATCCTCAATA AATATTTGTT GAACTAAA 118

SEQ ID NO:3563
SEQUENCE LENGTH:118

SEQUENCE TYPE:nucleic acid
TOPOLOGY:linear
CLONE:HUMGS04177
SEQUENCE DESCRIPTION:
GATCCTGGCT AGANTGNTAA TTAAAAGTAT TTAATTTGAA GCACCATTTN ANTGTTCGTA 60
CTAGTAGAAA ATGATGTGAA TTTCNTTTCT GTTCGGCTCC TATTTTCCTC ATCATTTN 118

SEQ ID NO:3564
SEQUENCE LENGTH:82
SEQUENCE TYPE:nucleic acid
TOPOLOGY:linear
CLONE:HUMGS04179
SEQUENCE DESCRIPTION:
GATCATAGTC GGGCATACTT ATCTATATCC NTAACCTCTA TATCTTTAAA TAAATGTGAG 60
NACTGTTCTC ATGGAGANGA AA 82

SEQ ID NO:3565
SEQUENCE LENGTH:214
SEQUENCE TYPE:nucleic acid
TOPOLOGY:linear
CLONE:HUMGS04180
SEQUENCE DESCRIPTION:
GATCTAAAGC NTTTNTGTCA GATTATTTAA TATNATGACT TCATGCTTTA TTATGCCTTA 60
TNATGGCTGA CGTATTACTG TGGTGAAACA AAATATCTTT AAAAGTTAAA ACATCCAGAT 120
ATATAAGCAA TTTTNCCCTA AGGATAAAGT ACCTTTAAGC ATGAGTGTAT CACAGCTTTC 180
ATTAGGNAAA CTTTTCATTA CATACTTGTT TAAN 214

SEQ ID NO:3566
SEQUENCE LENGTH:102
SEQUENCE TYPE:nucleic acid
TOPOLOGY:linear
CLONE:HUMGS04181
SEQUENCE DESCRIPTION:
GATCCCCNAA TGTGTATGAA TGACTGAGGC TTTGTAAATT AAGGGACGTT TGTTTGAATA 60
AAGTTATTTG ATGGGGTCAA AGAAGCCATA TGTGATTTGA AA 102

SEQ ID NO:3567
SEQUENCE LENGTH:101
SEQUENCE TYPE:nucleic acid
TOPOLOGY:linear
CLONE:HUMGS04182
SEQUENCE DESCRIPTION:
GATCCTCTCC TTAGTCTTAG CTCATGGGGC TCTTTATAAA GGAGTTGGGG GGTAGAGGCA 60
GNAAAATNGG AACCGAGCTG AANGCAGAGG CTGAGTTAGG N 101

SEQ ID NO:3568
SEQUENCE LENGTH:99
SEQUENCE TYPE:nucleic acid
TOPOLOGY:linear
CLONE:HUMGS04183
SEQUENCE DESCRIPTION:
GATCTGCAGT GCTTCACTGG AAATAATTTA TTCATTGCAG ATACTTTTAA GGTGGCATTT 60
NATTCATTTC CTGTGCTTTA AATAAACAAA TGTACCAAA 99

SEQ ID NO:3569
SEQUENCE LENGTH:97
SEQUENCE TYPE:nucleic acid
TOPOLOGY:linear
CLONE:HUMGS04184
SEQUENCE DESCRIPTION:
GATCACCCTG GGCACATGTT GTCAGGACCT CCTGGGGTTG TGTCATGGAT GCGTGTCCTC 60
AACCTTAGCA AAATAAACTT TCTAAATTAC TGAGAAA 97

SEQ ID NO:3570
SEQUENCE LENGTH:95
SEQUENCE TYPE:nucleic acid
TOPOLOGY:linear
CLONE:HUMGS04186
SEQUENCE DESCRIPTION:
GATCTTCAGT GTGTCTGCAT AAGCTAACTT AAGATGAATT TAAGTACANN TTTCTGAAAT 60
ATTTCTATTG AAATAAATTA CTTAAAATTA NNAAA 95

SEQ ID NO:3571
SEQUENCE LENGTH:94
SEQUENCE TYPE:nucleic acid
TOPOLOGY:linear
CLONE:HUMGS04187
SEQUENCE DESCRIPTION:
GATCTTTGTG AATTCTATGA ATGTATTAAG TTATCATATG TACTCCCCAA AATAGGTACA 60
TCTATTATGT ATCAATAAAA AANTTTAAAA CAAA 94

SEQ ID NO:3572
SEQUENCE LENGTH:93
SEQUENCE TYPE:nucleic acid
TOPOLOGY:linear
CLONE:HUMGS04188
SEQUENCE DESCRIPTION:
GATCCCTTGA GCCGAGGAGG TTGAGGCTGC AGTGAGCTAT GGTCACACCA CTGCACTCCA 60
GCNTTGGTTA TNATGCAAGA CCCTGTTTAC AAA 93

SEQ ID NO:3573
SEQUENCE LENGTH:89
SEQUENCE TYPE:nucleic acid
TOPOLOGY:linear
CLONE:HUMGS04189
SEQUENCE DESCRIPTION:
GATCGCACAC CTTTGCAACA GATGTGTTCT GATTCTCTGA ACCTACAAAA TAGTTATACA 60
TAGTGGAATA AAGAAGGTAA ACCATCAAA 89

SEQ ID NO:3574
SEQUENCE LENGTH:90
SEQUENCE TYPE:nucleic acid
TOPOLOGY:linear
CLONE:HUMGS04190
SEQUENCE DESCRIPTION:
GATCAGTGTC TCTAGTCCTA CCCAGTTTTA AAGTTCATGG TAAGATTTGA CCTCATCTCC 60
CTCAAATAAA TNTATTGGTG ATTTTNGAAA 90

SEQ ID NO:3575
SEQUENCE LENGTH:87
SEQUENCE TYPE:nucleic acid
TOPOLOGY:linear
CLONE:HUMGS04191
SEQUENCE DESCRIPTION:
GATCCGCCAG GTGCGGGCGC TACTGCAGGC ACAGCAGGCC TAGGTCCTCC CACACCTGCC 60
CCCTAATAAA GTGGGCGCGA GAGGAAA 87

SEQ ID NO:3576
SEQUENCE LENGTH:87
SEQUENCE TYPE:nucleic acid
TOPOLOGY:linear
CLONE:HUMGS04192
SEQUENCE DESCRIPTION:
GATCCACCCA CACCTAAGTC ACAGAATTTC TAAGTTCCCC AACTACTCTC ACACCCNTTT 60
AAAGATAAAG TATGTTGTAA CCAGAAA 87

SEQ ID NO:3577
SEQUENCE LENGTH:83
SEQUENCE TYPE:nucleic acid
TOPOLOGY:linear
CLONE:HUMGS04193
SEQUENCE DESCRIPTION:
GATCAAACTG AATGAAACCC CCACACACAC ACACATGCAT ACACACACAC ACACAGCCAC 60
ACACACAGAA AATATACTGT AAA 83

SEQ ID NO:3578
SEQUENCE LENGTH:83
SEQUENCE TYPE:nucleic acid
TOPOLOGY:linear
CLONE:HUMGS04194
SEQUENCE DESCRIPTION:
GATCCCTATT CTGCGTGTAT GTGTTTGCAC ATGAGTGTTT GTAGACCATA GACCATTAAA 60
AAATACGGAA TTCAAAAGTT AAA 83

SEQ ID NO:3579
SEQUENCE LENGTH:84
SEQUENCE TYPE:nucleic acid
TOPOLOGY:linear
CLONE:HUMGS04195
SEQUENCE DESCRIPTION:
GATCTATGAA TGTTTTTNTT AAAATTTACA AAGCTTTGTA AATTAGATTT TNTTTAATAA 60
AATGCCATTT GTGCAAGNTT TAAA 84

SEQ ID NO:3580
SEQUENCE LENGTH:112
SEQUENCE TYPE:nucleic acid
TOPOLOGY:linear
CLONE:HUMGS04196
SEQUENCE DESCRIPTION:
GATCACATAA AGGCAAGAGG ATACTTCATG AATAATACAT TTNAATGCAA ATAAACAGAT 60
GGTTCACTTC TACTAGCTAT NNCCCTGTCT TNNTATACAC TGAGTAGATT AN 112

SEQ ID NO:3581
SEQUENCE LENGTH:74
SEQUENCE TYPE:nucleic acid
TOPOLOGY:linear
CLONE:HUMGS04197
SEQUENCE DESCRIPTION:
GATCTATGTC TGTTGGTGGC AATGTGAGGG TGATACTCTC TCACTCTAAT AAACTTGGCA 60
CTTCTCCGAG TAAA 74

SEQ ID NO:3582
SEQUENCE LENGTH:74
SEQUENCE TYPE:nucleic acid
TOPOLOGY:linear
CLONE:HUMGS04198
SEQUENCE DESCRIPTION:
GATCCTGTAT GTGTGTTGAG ATTTAGAGGT TTCATTTATN TTGTCTGCTA ANAAATTGTT 60
ACTCTAATAA TAAA 74

SEQ ID NO:3583
SEQUENCE LENGTH:71
SEQUENCE TYPE:nucleic acid
TOPOLOGY:linear
CLONE:HUMGS04199
SEQUENCE DESCRIPTION:
GATCACTGTA TAGACTGTTA AATTTGATTT CTTATTACCN ATTGTTAAAT AAACTGTGTG 60
AGACAGACAA A 71

SEQ ID NO:3584
SEQUENCE LENGTH:71
SEQUENCE TYPE:nucleic acid
TOPOLOGY:linear
CLONE:HUMGS04200
SEQUENCE DESCRIPTION:
GATCTATTTC TCTGTCTACT TTGGANATGA TTGCATAATA AATAAAATTT TACTGTTTTT 60
TTAAAAGGAA A 71

SEQ ID NO:3585
SEQUENCE LENGTH:69
SEQUENCE TYPE:nucleic acid
TOPOLOGY:linear
CLONE:HUMGS04201
SEQUENCE DESCRIPTION:
GATCTAAAGA TGTTTAGGGA AGAGCTCGAC TAAAGAACAA TGAAATAAAT GGTCCAAGGG 60
GAAGTCAAA 69

SEQ ID NO:3586
SEQUENCE LENGTH:65
SEQUENCE TYPE:nucleic acid
TOPOLOGY:linear
CLONE:HUMGS04202
SEQUENCE DESCRIPTION:
GATCAAAAAT GTAAGTCTAT TGGTAGAGAT TAAGTAAAGT ATTTATTGCT ACATCATAGT 60
TGAAA 65

SEQ ID NO:3587
SEQUENCE LENGTH:53
SEQUENCE TYPE:nucleic acid
TOPOLOGY:linear
CLONE:HUMGS04203
SEQUENCE DESCRIPTION:
GATCTGTGTT GCATTTCAAA ATAAACTGGT GTGTGCTCTG CCTGGATTTG AAA 53

SEQ ID NO:3588

SEQUENCE LENGTH:153
SEQUENCE TYPE:nucleic acid
TOPOLOGY:linear
CLONE:HUMGS04204
SEQUENCE DESCRIPTION:
GATCTCAGCC CAGGACGNCA AGCAAGCAGG TCTTGTCAGC AAGATTTGTC CTGTTGAGAC 60
ACTGGTGGAA GAAGCCATCC AGTGTGCAGA AAAANTTGCC AGCAATTTCT AAAAATTTGT 120
AGTAGNCGAT GGNCNAAGNA TCAGTGGATN CAN 153

SEQ ID NO:3589
SEQUENCE LENGTH:29
SEQUENCE TYPE:nucleic acid
TOPOLOGY:linear
CLONE:HUMGS04205
SEQUENCE DESCRIPTION:
GATCCAAATA AAGGTAGCTG CTGACCAAA 29

SEQ ID NO:3590
SEQUENCE LENGTH:64
SEQUENCE TYPE:nucleic acid
TOPOLOGY:linear
CLONE:HUMGS04206
SEQUENCE DESCRIPTION:
GATCTCTCTA CAAGAGCCCC TGCCCCTCTG TTGGAGGCAC AGTTTTAGAA TAAGGAGGAG 60
GAGN 64

SEQ ID NO:3591
SEQUENCE LENGTH:64
SEQUENCE TYPE:nucleic acid
TOPOLOGY:linear
CLONE:HUMGS04207
SEQUENCE DESCRIPTION:
GATCCTTTGC GTGACAGTCT TGTATGGAAA ACAGATGCTG ACAGAATTGT AGACTACCAT 60
NCCN 64

SEQ ID NO:3592
SEQUENCE LENGTH:63
SEQUENCE TYPE:nucleic acid
TOPOLOGY:linear
CLONE:HUMGS04210
SEQUENCE DESCRIPTION:
GATCTCGCCT TGTTTCTTAC AAGCAAACCA GGGTCCCTTC TTGGCACGTA ACATGTACGT 60
ATN 63

SEQ ID NO:3593

SEQUENCE LENGTH:63
SEQUENCE TYPE:nucleic acid
TOPOLOGY:linear
CLONE:HUMGS04211
SEQUENCE DESCRIPTION:
GATCTTTGCG AATTACAATG CATATATGTC TATTTATTCA ATATCTGTCA TATAATANCT 60
ATN 63

SEQ ID NO:3594
SEQUENCE LENGTH:58
SEQUENCE TYPE:nucleic acid
TOPOLOGY:linear
CLONE:HUMGS04213
SEQUENCE DESCRIPTION:
GATCTTAAGA AAACTTCATA CTGTATGAAT AAGAAAATAA AATATTTAAA ACTTGAAA 58

SEQ ID NO:3595
SEQUENCE LENGTH:64
SEQUENCE TYPE:nucleic acid
TOPOLOGY:linear
CLONE:HUMGS04214
SEQUENCE DESCRIPTION:
GATCGCCAAG TTCAAAGCTG TGCACATGTG GACACTCAAT AAATGTTCAT TGGTGAAAAA 60
CAAA 64

SEQ ID NO:3596
SEQUENCE LENGTH:56
SEQUENCE TYPE:nucleic acid
TOPOLOGY:linear
CLONE:HUMGS04215
SEQUENCE DESCRIPTION:
GATCCAAAAA GAGTCGACCN GCATTNTTTA TATTAAAAAT AAAGTCAAGA TTTAAA 56

SEQ ID NO:3597
SEQUENCE LENGTH:52
SEQUENCE TYPE:nucleic acid
TOPOLOGY:linear
CLONE:HUMGS04216
SEQUENCE DESCRIPTION:
GATCTTTGTT AATGTCTTGA TTCTGTTCGC AAAGCACAGA CTAGTGCTTA AA 52

SEQ ID NO:3598
SEQUENCE LENGTH:52
SEQUENCE TYPE:nucleic acid
TOPOLOGY:linear

CLONE:HUMGS04218
SEQUENCE DESCRIPTION:
GATCGCACCA CTGAGCTCCA GCCTGGGCAG CTGNATGAGA CTNCATCTCA AA 52

SEQ ID NO:3599
SEQUENCE LENGTH:51
SEQUENCE TYPE:nucleic acid
TOPOLOGY:linear
CLONE:HUMGS04219
SEQUENCE DESCRIPTION:
GATCTGGAGG GACACACAGG AGACACCTGG CATAACCAAA AAATGATTAA A 51

SEQ ID NO:3600
SEQUENCE LENGTH:51
SEQUENCE TYPE:nucleic acid
TOPOLOGY:linear
CLONE:HUMGS04220
SEQUENCE DESCRIPTION:
GATCGAAAAC ATATAGANAA AATAAAATGT GTGGTTGCAC CTGTACTGAA A 51

SEQ ID NO:3601
SEQUENCE LENGTH:466
SEQUENCE TYPE:nucleic acid
TOPOLOGY:linear
CLONE:HUMGS04247
SEQUENCE DESCRIPTION:
GATCTAGAGG AGGATTTGCA GGAAGAGCTC GTGGAAGAGG TGGTGACCAN CAGAGTGGTT 60
ATGGGAAGNT ATCCAGGCGA GGTGGTCATC AAAATAGCTA CAAACCATAC TAAATNATTC 120
CATTTGCAAC TTATCCCCAA CAGGTGGTGA AGCAGTATTT TCCAATTTGA AGATTCATTT 180
GANGGTGGCT CCTGCCACTG CTTAATAGCA GTTCAAACTA AATTTTTTGT ATCAAGTCCC 240
TGAATGGAAG TATGACGTTG GGTCCCTCTG ANGTTTAATT CTGAGTTCTC ATTAAAAGAA 300
ATTTGCTTTC ATTGTTTTAT TTCTTAATTG CTATGCTTCA GANTCATTTG TGTTTTATGC 360
CCTTTCCCCC AGTATTGTAG AGCANGTCTT GTGTTAAAGC CCAGTGTGAC AGTGTCATGA 420
TGTAGTAGTG NCTACTGGTT NTTTAATAAA NCCNTNTTGT ATGAAA 466

SEQ ID NO:3602
SEQUENCE LENGTH:429
SEQUENCE TYPE:nucleic acid
TOPOLOGY:linear
CLONE:HUMGS04248
SEQUENCE DESCRIPTION:
GATCTATAAA TCAAGAAAAT CCATTGTCAT AACCATTTTT AAAAGTCAAT AATTAAGACA 60
TCCTTAATTA AAAAGTTTCA AATCTAGACA CTAAATGTGT GTGAATGTAC AAAGAAAACA 120
AACCATTGCT TATGCTGTTA TATACTAGAG AAATTTTGTT TTGCTTGCTG TTTTAACTTG 180
ACAGATGAAG GACTTTAGTT GAACTTCATA TTGTAAGAAC TGTTAATAAA AGTTGTCAAG 240

TAAAAAGCGC TATATCTAAA AAGACTTTAT GAACAGTTAT TCTATCAACT TTTAAAGGGT 300
TTTAAACCTG CCCAGAAATT ACCTTGGTAT CTGAAGTTTC CCTCTGTCTC CTCCCTCTAA 360
TTNAGGCTTG TTATTTGTCA TGCACCAGCA TTTGGGGGNT AATAANAATT TCTTNGGTTC 420
TNGTGGAAA 429

SEQ ID NO:3603
SEQUENCE LENGTH:419
SEQUENCE TYPE:nucleic acid
TOPOLOGY:linear
CLONE:HUMGS04249
SEQUENCE DESCRIPTION:
GATCTGTTGT TTGTTTTTAA GGTGGTGTTC TGTTAACTTC CAGCAGGATG CAGAATCTAA 60
CTGGTAAACC TATAAATTGG TCAAATTTNA CCCATGTTGT TTCTTTGAAA ACAATGTATA 120
TNCTCTAATA TTTGGATATA GGGTATATGA NCATTAATGC AACTTAATTG TATTTCCAAG 180
TACAGTACAT ATGTNCAGAT TTCTTTTTCT AACCTTATTT AGTGATAGAG GTGTGGTAAA 240
CNCTTCCATT ATGTTTATGT ATTANCACTG TAATTTTGTC ATTTTNATAT ATTTNGATGT 300
TATGTAACTA GATGCATACA GGATTAGGTA TTTCCTTCTG TATTCCTTTT TATGTGGAGA 360
TGATATTTTT ACATAGCAAA GTATTGTAAT GCATTAATAA TAAATGGTCA ACCATTAAA 419

SEQ ID NO:3604
SEQUENCE LENGTH:419
SEQUENCE TYPE:nucleic acid
TOPOLOGY:linear
CLONE:HUMGS04250
SEQUENCE DESCRIPTION:
GATCTGCTAG TTCCAGGCCT CTAAGACAGG AACGTATGTG CCATAAGTGN GTCTACTTCA 60
CAGACTCAAT GAGGCAGAAA TTATTGTAGT TTTCTCCTAT TTCTTCTGCA CCCAACTTTC 120
TNCTTGTATT TCAAAGGCCA GGCCATGTAC ACTAACGTCC TTGAAATTTG CAGTTCTGTA 180
TGCTTCTATT CCAAATCATT CATTACCAAT AAAAACGGAA TACCACCCTT TCCATTTTAT 240
AGNCCTCATC CCCTATTTCT GTCAGACAGT TATATGACAG GGTGGNCNGN GGGCCTCTTA 300
GTTCATCCAA AGTCTNNCCT GNAGTGCTAG NCTTTCAGAC TCTTATCACT GGAAATCNTT 360
AAGGGTTGAG GGGGNTTTAN TTTCCCAGNA CTTGTGNAGG GNTTCAACTG TCAAAACTN 419

SEQ ID NO:3605
SEQUENCE LENGTH:404
SEQUENCE TYPE:nucleic acid
TOPOLOGY:linear
CLONE:HUMGS04252
SEQUENCE DESCRIPTION:
GATCCGACAC AGAGAACAGG AGGNACTAGA GGCGACANAN AGNAGGGCTT TAAGCCAAAC 60
TACATGGAAA ATGGTGATAA AAGAAAATNT GGCTGAAGTT ACCCGAAGTT CAGCTTGCAG 120
TGTAATTCAG AAGAGTTAAG ACCAGAAGTA CTGGAATCAT ACTTCCTTTC AGTCTTGCCA 180
CTTGCCTAGT GAGGCTTTTC CGAACCTGGA AGGAGACGTC TAGAAATCCC AACTTTGCTG 240
TGTAAGGACC ATTAGCTGCA AGTCAGTGGA AGTCTATAGA AAANAGTGTG AATTCCATAG 300
TGGTCTTGAC TTCTTAGCAA GGATTTGGGA TAAGTCTAAA ACTTCAGACT TAAAGCTTTG 360

AAGCATGTTA ACTGCATGAN ATATTAANCA TTTCGAACTT CAAA 404

SEQ ID NO:3606
SEQUENCE LENGTH:403
SEQUENCE TYPE:nucleic acid
TOPOLOGY:linear
CLONE:HUMGS04253
SEQUENCE DESCRIPTION:
GATCCATGAA TTAACAGAGG CATATATTTA AAATGAAATT TAATGAGCAC TTAGTGACTT 60
CATTTGTTCA AGAATATTTG ATTTCCTATT GTGTGCATGG GAGGTTTACA AAAGACAGAC 120
TGTCCCTATG CTTGTNCAGC TTACAATCTA TTTAACTATT AGAGGCCCTG TGAGATGTAG 180
GCAGTTATTT ACAGATGAGA AAACTAACAC AGCAGTGGCA ATGCTGGGAA TGGCCAACAG 240
GCTTGGAATT CAACTCAGTT TAGCACCAAA GCCAGTNCTA TTAAGACCTG AAGTTCAACA 300
GTGGATTCCT TACCCAAATA GTTTTTAAAG TATGCTCTAA GGNCCAATGT TTTTAATGAA 360
ATCTTGGNTT NCAAAGTNGG NNTAAAAGGG TTCTGAGCAT AAA 403

SEQ ID NO:3607
SEQUENCE LENGTH:396
SEQUENCE TYPE:nucleic acid
TOPOLOGY:linear
CLONE:HUMGS04256
SEQUENCE DESCRIPTION:
GATCTTACTT CGTTGACCCA TATTTTTCCC CTGACCAAGT TACCTGTAAA CTGGAATTTG 60
CAAGGGGATG CTGTGATGAT AACCCCTTTC TATTGCTGTA ATGTTCATAT AACCTGGGAA 120
ACTGAGAGAA GGGGATGTGT AAATAAAAGC TTAAACATTT TAGTAATGTG TTAAAATGTC 180
ACTCTCTCTT ACCCTGTTTC CCTTTTTTGC CAGATGATGA TTTTTTTATT TTTATTTTGT 240
ACTTTACTGG ATGACTGTGA AGCGNTGAGT ATTGGGTTGG GGTAGGTGTG TTGATTTTGA 300
GAGTGCATGT TAAGACCTGA AGGGGAACTA CTTGAGGATG ACTTAAGAAG CATCCCATGC 360
AAATATCTTG TTTTTGCCNG AATAAAATAT TTCAAA 396

SEQ ID NO:3608
SEQUENCE LENGTH:392
SEQUENCE TYPE:nucleic acid
TOPOLOGY:linear
CLONE:HUMGS04257
SEQUENCE DESCRIPTION:
GATCTGCCCG CCTTGGCCTC CCAAAGTGCT GGGATTACAG ACATGAGCCA CCACGCTTGG 60
CCGGGATAGT ATATTTTTAT AGCACTTCCC CTACTGATTG CTGCCTTCTC TGTGGCTACA 120
AGGGACCCAC AGAATTACAG GGAAGTTACA GGGNNGCAGG TTTCATCTCA ATATTGGGAG 180
AGATTTCAAA CAATCACACC NNNNNNNCGA AGGAGTGGGC TGTCACTAGG AATTTTTATT 240
CCCAGTCCGT CAGGAATTTT GTAGAAGGGC TTCATGTGCT GGTACCAATA GGACAGGAAG 300
ATTTTAATCA GCTTTACTAT CTATGTTTTT TTATGGAAAC TGTGTGTATG TATACATACA 360
TTTTCCAAAA AGNANAAATT AAATGATTAT AG 392

SEQ ID NO:3609

SEQUENCE LENGTH:392
SEQUENCE TYPE:nucleic acid
TOPOLOGY:linear
CLONE:HUMGS04258
SEQUENCE DESCRIPTION:
GATCCGCCCA CCTCCGCCTC CCAAATTGCN GGGATTACAG NCCTNAGCCA TNCCCACCCG 60
GCCAATAAAA ATTTTTTTAA AGCTAAAATT GACTCTCATG CAAATAAGAT AAAATGAACA 120
TATGTTAAAT ATTTTAACTT AATGAGAGAA ATAGTAAGAT GTTACCAGTT ACTTGATGAT 180
GGAGGAAAGG ACGAAAAGCC AAGGAATACA TGGAATGCAG CTCTAGGTGC TGAAAAAGGC 240
ANGGAAAGGT TCTACGTTAG NGCTTCCAGA GGGAGCTCAG CCCTGTCANC ANCNTNGTTT 300
CAGCTCAGTC ATATCGNTGT TTAGACTTTC TGGTCCCCAG NGCTGGGCGG GGGATATATT 360
TCCTGCTGTT TTAAGNCNCC NNATTTTGTG GN 392

SEQ ID NO:3610
SEQUENCE LENGTH:391
SEQUENCE TYPE:nucleic acid
TOPOLOGY:linear
CLONE:HUMGS04259
SEQUENCE DESCRIPTION:
GATCCTAAGG GGTTCAGGGG GACCCTACCC CCACCTCAGG TTGGGCTTCC NTGGCCACTC 60
ATGCTCCACA CCAAAGCAGG ACACGCCATT TTCCACTGAC CACCCTCTAC CCTGAGGAAA 120
GGGAGACTTT CCTCCGATGT TTATTTAGCT GTTGCAAACA TCTTCACCCT AATAGTCCCT 180
CCTCCAATTC CAGCCACTTG TNAGGCTCTC CTCTTGACCA CTGTGTTATG GGATAAGGGG 240
AGGGGGTGGG CATATTCTGG AGAGGAGCAG AGGTCCAAGG ACCCAGGAAT TTGGCATGGA 300
ACAGGTGGTA GGAGAGCCCC AGGGAGACGC CCAGGAGCTG GCTGAANGCA CTTTTGTACA 360
TGTAATGTAT TATATGGGGT CTGGGGCTCC N 391

SEQ ID NO:3611
SEQUENCE LENGTH:386
SEQUENCE TYPE:nucleic acid
TOPOLOGY:linear
CLONE:HUMGS04261
SEQUENCE DESCRIPTION:
GATCCGACCG CGTGNTGCCG CTCTGCTCTC TCATACGCGT GTATGTTTGG TTCCATGTCA 60
CAGCCCCCTA GGAGCCAGTN ATGCTCGGCC TTGCGCCCGT NCCACCTCCC AGGCCACCCT 120
TCCTGGGCTT CTGGGCCACC TGCCCTCGGG GGGCCCCTGC NAGGGTGCCT GGNGTTCCCA 180
CGTGTCCCGG GGCTTTTCCA GGAAGCCCGA GCCCAGGACC TGTTGGCAGA GTTGCCAGGG 240
TTACATTTTT NAAGCACCTG CTCCTTTTNT TGCAGTGTAT TTTCTACAAC CAGATTGTAT 300
TAATATTTTT AACTTTGCCC TTTTAAAAAA TATACCTAAT ACAATATATT TAATTNTGAA 360
TTAAACTCTT AAACTTTTCT TCCAAA 386

SEQ ID NO:3612
SEQUENCE LENGTH:375
SEQUENCE TYPE:nucleic acid
TOPOLOGY:linear

CLONE:HUMGS04264
SEQUENCE DESCRIPTION:
GATCCCTTTG GGGCATGAAG TGTGACAAGT GTGGGCTCCT GAAAGGANTG TNCCAGAGAA 60
ACCAGCTAAA TCATGACACC TTCAATTTGC CATCATGACG CAGACCTGTA TACATTAGGN 120
TAAATCTGAA TTTCCACTGC TTTGGAGAGT CCCACCCACT AAGCACTGNG CATNNAAACA 180
GGTTCCTTTG CTCAGATGAA GGAAGTAGGG GGTGGGGCTT TCCTTGTGTG ATGCCTCCTT 240
AGGCACACAG GCAATGTCTC AAGTACTTTG ACCTTAGGGT AGAAGGCAAA GCTGCCAGTA 300
AATGTCTCAG CATTGCTGCT AATTTTGGTC CTGCTAGTTT CTGGATTGTA CAAATAAATG 360
TGTTGTAGAT GGAAA 375

SEQ ID NO:3613
SEQUENCE LENGTH:372
SEQUENCE TYPE:nucleic acid
TOPOLOGY:linear
CLONE:HUMGS04265
SEQUENCE DESCRIPTION:
GATCCTAGAC CAGCATGCCA GTGTGCCAAG GCCACAGGGA AAGCGAGTGG TTGGTAAAAA 60
TCCGTGAGGT CGGCAATATG TTGTTTTTCT GGAACTTACT TATGGTAACC TNTTATTTAT 120
TTNCTAATAT AATGGGGGAG TTTCGTACTG AGGTGTAAAG GGATTTATAT GGGGACGTAG 180
NCCGCNNTCC CNNGTGTTGT AGGTTTCTCT TTTTCAGGCT TATACTCATG AATCTTGTCT 240
GAAGCTTTTG AGGGCAGACT GCCAAGTCCT GGAGAAATAG TAGATGGCAA GTTTGTGGGT 300
TTTTTTTTTT NACACGAATT TGNGGAAAAC CATTAANTCA TTCAAAATAA TAANCTNTTT 360
TTATTNGGGA AA 372

SEQ ID NO:3614
SEQUENCE LENGTH:372
SEQUENCE TYPE:nucleic acid
TOPOLOGY:linear
CLONE:HUMGS04266
SEQUENCE DESCRIPTION:
GATCTAAATA TATATTTTGT ATATTATAAT ATTTTAGGAA TAGCTTAATT AAGANCTCAC 60
AGACTTTGAC AAAGTGGTAA CTAGACTTTT AATTACTAGA TTCTTCAAAT TAGGCTTAAT 120
GCCTGCTAGC ATGCCAATCC AATCCCATGA CTGATGTTAT TATACACTAT GGCAAATACA 180
AAGTTGTAGT ATTATGGTTC TTGATGGTGT GATTTACTTA TGTAAATTAT GTACAAATTT 240
AAGTTCAATG AAACACTGAT TTAATGTACT GAAAGGTAAA TAGTACCATA GTGAATTATT 300
TCCTGTCTTG CCTTAGGTTT ATTTTTATNA TCTTCAATAT TAGANTAAAT GCTAAATTAT 360
TTTCATTTTA AA 372

SEQ ID NO:3615
SEQUENCE LENGTH:370
SEQUENCE TYPE:nucleic acid
TOPOLOGY:linear
CLONE:HUMGS04267
SEQUENCE DESCRIPTION:
GATCTGAACT CCTTATACAT AAGAAGGTGT GTATATTAAT CCAATTATGG ACTTAAAATA 60

TTTTAAAAGT ATAAATACCC TTATTTGCTG CAAAGACCAG TGTGTAGGCA TTTGCTTTTT 120
AGCAATATTT TTAAGTGCTC CATTTTAATG CCGAGGAATA AGTCTTTTGG CAACACAAAC 180
TGGTCAATAA TAGGTAATGC AGGTATGTTC AGGTTAAGCC AACAATGTTT TGCATTTTTA 240
TGCTTATTTT CTGTCAACAC TAATGAAGTC AACATTGCCT GAATGTCTGA ATAATGAAAC 300
ACATCCCNGT TTAAAAGTAT GTAACTGAAA AAGAAATAAA AAANAAATTA AAAGGTAGGT 360
TTTTTTTAAA 370

SEQ ID NO:3616
SEQUENCE LENGTH:370
SEQUENCE TYPE:nucleic acid
TOPOLOGY:linear
CLONE:HUMGS04268
SEQUENCE DESCRIPTION:
GATCCCATTA TATAGTATGT ATAGCTGAAA TCTGTAATTC AATCACTTTT TCTCTTTTAT 60
CCTCTAACCA AAAAATTGTT TAATTTTGCA TCCCAAATGT TTTTAATCTT TGTATATTTT 120
TNAAAAATCC NTTTCTCCTC AGCANTGCCT TTTTNGTGGT TGTAAATAGA CTTACTTGCA 180
CTTTGAAGAT GAGTTACTCC TTGTCATCTT ACAAATATGT GATATGGTAA TTTNCATAAC 240
AGATGTCAGT TTTGANCCAA GAATTGGTGA TTTGTTTATA AGAAAAAAAC TGGCTTCATT 300
NCTGTGNAAT TGCTCTTTGG NAAATTNCTN NTTNACACGT GTAAGCCAAC CTGAGGTACC 360
GTGATGGGTN 370

SEQ ID NO:3617
SEQUENCE LENGTH:368
SEQUENCE TYPE:nucleic acid
TOPOLOGY:linear
CLONE:HUMGS04269
SEQUENCE DESCRIPTION:
GATCCCTGCT CAAGCAGCAC AGAGGAAGGG GCAAGACGTG GCCTGTAGGC ACTGTNTCAG 60
CCTGCAGAGA AGAAAGTNAG GCCGGGAGCN TGAGCCTGGG CTGGAGCNTT CTCCCCTCCC 120
CANTTGGACT AGGGGCAGTN TTAATTTTNA AAAGGTGTGG GTCCCTGTTT CCTCTTCCAG 180
GGGTCCAAGG GAACAGGAGA GGTCACTGGG CCTGTTTTTT CCCTCCTGAC CCTGCATCTC 240
CCACCCCGTG TATCATAGGG AACTTTCACC TTAAAATCTT TCTAAGCAAA GTGTGAATAG 300
GATTTTNACT CCCTTTGTAC NNCNGNCTGA GGAAACGCAA ATAANAAGGG CAACATGTTT 360
CTNTTAAA 368

SEQ ID NO:3618
SEQUENCE LENGTH:364
SEQUENCE TYPE:nucleic acid
TOPOLOGY:linear
CLONE:HUMGS04270
SEQUENCE DESCRIPTION:
GATCGGCCCA GCTCCTGAAC TNTGANGAGG CCATTCATAT GGCATTCCTG GAACAAAACA 60
CTGCACAGGT ACCAGCCTCT CCACTCCTGA CCGGGTTGGT GCTGAACAGT CAGGGATTGT 120
NCTTGAACTA GACTTCTGAT GCTTCTTGCA ATCTTCTTTC ATCTTTCCCT GAAATACACA 180
AAATAAACAA NTACAATAAC AAATAGTAAT TAANTGACTT TCAGGATAAC ATCTAGTTGT 240

TCAGACTTCA CCCTTCACAG GTGTGTGTGT ATGTGTGTTT ATGTCTGTAT ATTGANGCAN 300
TTTGAATTTA TTTTACTGTA TATNTNCTGA GTAAANGACT GANATGNNCT NCTTGGGTCA 360
GAAA 364

SEQ ID NO:3619
SEQUENCE LENGTH:363
SEQUENCE TYPE:nucleic acid
TOPOLOGY:linear
CLONE:HUMGS04271
SEQUENCE DESCRIPTION:
GATCTGAAGC CTTCCATAAA TNAGAGGCCA TTCATATGGC ATTCCTGGAA CAAAACACTG 60
CACAGGTACC AGCCTCTCCA CTCCTGACCG GGTTGGTGCT GAACAGTCAG GGATTGTTCT 120
TGAACTAGAC TTCTGATGCT TCTTGCAATC TTCTTTCATC TTTCCCTGAA ATACACAAAA 180
TAAACAAATA CAATAACAAA TAGTAATTAA ATGACTTTCA GGATAACATC TAGTTGTTCA 240
GACTTCACCC TTCACAGGTG TGTGTGTATG TGTGTTTATG TCTGTATATT GANGCAATTT 300
GANTTTATTT ACTGGTATAT GTTTCTGAGT AAAAGNCTGA AATGNNCTNC TTGGNNCNGG 360
AAA 363

SEQ ID NO:3620
SEQUENCE LENGTH:361
SEQUENCE TYPE:nucleic acid
TOPOLOGY:linear
CLONE:HUMGS04272
SEQUENCE DESCRIPTION:
GATCCAGGAT GTACTTGGAT GAGGAGGCCT GGCTTATCTA GGAAGTCGTG TCTGGGGTGC 60
TTATTGCTGC TCCATACAGC TGTACGTCAG CCCCTTGGCC TTCTCTGTAG GTTCTTGGCA 120
GCAANNNNGC AGCTTTCACT CAGTGACACA AGTAATTACT GAGTCCTAAT TTGATAGCCA 180
CCAACTGTAC CTGGGTAGGC AAAGTCAGAT TTTNGAGGAC CTTTTTCCTG ATTTGAAGTT 240
TTAATTACCT TATTTTCTNT TATGCTTTNC CTCTGTCTTG TAATCNTNCT CTTCCTAAAA 300
ANCCTCCCTA TAAATTCAAT TAATTTGGGT TAATTTTAGG ATAAACCCAT TTANTTCNAA 360
A 361

SEQ ID NO:3621
SEQUENCE LENGTH:359
SEQUENCE TYPE:nucleic acid
TOPOLOGY:linear
CLONE:HUMGS04273
SEQUENCE DESCRIPTION:
GATCCAGATN CTTTCGGATG TCAGGGATAT TCACATTGAC ATGTCTNNNN AGTGTTCCAG 60
GAGATTTTAG TTTGAATAGT ACAATTTTTT CTTCTTACAA CAAAACTTAG AACGGCTTCT 120
AACTTATTTC TTCATGGCCA AGAAACTACC CTAACTTATC TCGATAAATA TTTAGTTCTG 180
TCTTATTTCT AATGTGTAAA ACAGGTTTTA AAAATAATAT ATAGATTTTG CATTTATAAA 240
AAAAGATTTA ATTTATAGAA CCAAACTNGA ACATATGTTT AATTTAGCCT AATCAGAAGG 300
TTACAGAAAA AGTTGCTGTA AAGATAGAAT AAAGGGTATT GTACTGGTCT GTTTTTAAA 359

SEQ ID NO:3622
SEQUENCE LENGTH:356
SEQUENCE TYPE:nucleic acid
TOPOLOGY:linear
CLONE:HUMGS04274
SEQUENCE DESCRIPTION:
GATCTACAGC AGCTGCATCG AGGACTCCCA CAGAGGTGCT ATAGGGCTGC GCATTGCACT 60
GGCCATCTCA GCTATAGCCG TCTTCCTGGT CTTGGTGTCT GNCTGTATCC TTCGATTTGG 120
CACCAGGTCT CTCTGCAACT CCATCATCTC CTTGAACACT ACAATNAGCT GTNCTGAAGC 180
CCAGAAAATT CCATGGACAC CCCCTGGAAC TGCTCTGCAG TTTTACTCCA ACCTACACAA 240
TGNTGAAACC TNTTCTTGGG TGAATTTGGT ATTGTGGTGT GTGGTCTTGG TGCTCCAGGT 300
CGTGCAGTGG AAGTCTGAAG CCACCCCATA CCGGCCTCTG GAGAGGGGTG ACCCTN 356

SEQ ID NO:3623
SEQUENCE LENGTH:420
SEQUENCE TYPE:nucleic acid
TOPOLOGY:linear
CLONE:HUMGS04275
SEQUENCE DESCRIPTION:
GATCCCCCCT GNCAGACACC AAAGCTGTGA ACAGAAAGAC TGCCTGGCCA GCAAACCTTG 60
GGACATCAGC CTNGCCCAGC CTGAAAGCAT CCGNAGTGAC CTAGAGAGTT CTGATGCACA 120
GTCAGACGAT GTGCCAGACA TCACCTCANA TGAGTGTGGC TCCCCCCGCT CCCATACTGC 180
AGCCTGCCCC TCGACCCCCA GAGCCCAAGG TGCACCGAGC CCAAGTGCCC ATATGAACCT 240
CTCTGCCCTA GCCGAGGGAC AAACTGTCTT GAAGCCAGAA GGTGGAGAAG CCAGAGTATG 300
AAGTGGAATG AATGCTCCTG TTCTGAGANG ACACTTGTAA CTGCATCTTT TGGAATTTTT 360
TTTTTTTTTT GCCCAGGGGN NAGGGTTTNT GGTTTTTAAT TTANAGGNCN TTTGGGCAAN 420

SEQ ID NO:3624
SEQUENCE LENGTH:352
SEQUENCE TYPE:nucleic acid
TOPOLOGY:linear
CLONE:HUMGS04276
SEQUENCE DESCRIPTION:
GATCAGAATT AAACTAGTAC TATGAAATGT CCTTTTGAAT GTNAGGTCAA GAAATCCATG 60
TACAGAGTCG TGCACTTCAT TGGCTGTAAC TGTCAAACTT GCCTTGTATT AGAAGTTAAA 120
TCCCATCGTA AATACATCAC GAGGCCAGCT GTGTGATTTC TGAGACCTAG ATGAGAGTCC 180
NATTACTTCA AGCNTTGATT CAGTAAATNT TAAGCAGCAG AAGNCTTCGG ATGGGTTGAA 240
AATCAATNTT ACAAGGTTTT AACTCATAAA CCCATNNGGG NTTTTTTTTT TCCTCTGCAA 300
NNGGTTNCTA AGGCTANTCC NTGGTAACAC TNTGGGAAAN AAATNTGGAG GN 352

SEQ ID NO:3625
SEQUENCE LENGTH:348
SEQUENCE TYPE:nucleic acid
TOPOLOGY:linear
CLONE:HUMGS04278

SEQUENCE DESCRIPTION:
GATCTCCAGT NGGCCTTTGC AGGCCCGGCC TCCTGCCTCT NGAAGGCCTA NCACGGGCCC 60
GGCCTCGGCC TCGGCCTCAC AGCAGACTCT CCACGCCCAG CTAGCTCTTG CCTCACTGCG 120
GCCTCCCCAG TCCAAAGCTC CTGCCTTTNG GCCACTTCGG CAGGTCCAGC TCCTGCCTGC 180
CAGTGGCCTC TTTAGGCCCA GCTCATTCCT CACGTCGGCC ATTCCAGGCC CCGTTTTTCC 240
CTTCCGGCAG CCTCTTGGCC TCTAATTTGT TTATNTTTTG TGTATAAATC CCAAAATATT 300
GAATTTTGGA ATATTTCCAC CGGGGGNGTA AATATTTTGA TAGGTAAA 348

SEQ ID NO:3626
SEQUENCE LENGTH:347
SEQUENCE TYPE:nucleic acid
TOPOLOGY:linear
CLONE:HUMGS04279
SEQUENCE DESCRIPTION:
GATCTTTATT AAGACAGTGG AAAGTATACT ATTAAGTCAT CATAGTCTTT TGGAGACTTG 60
TCAAANACAG CAATNAGAAA ATAAGACCTA GCATACCGTG GAGAACCATT AAAAATTTNA 120
NANGAAACAA CAAGTATTAT GTCAACTTAC TTCAAAGGCG AGTTTTGGGA TTTNATGCAG 180
TAAAGATTCC CTGTTTTATG ATTGTCCCTT GAAAGTCAAA TGGGGGCCTG TCCATTGTCC 240
TCTATTAANC TTNCAGAACT GTCACCAACA AATTTGAGTT GCCTTGTCTA GGGTTCTGGG 300
TAGTGAAGAT ATTNNATNAT ATAATNAAAT ACACTGTTAG TACTTAN 347

SEQ ID NO:3627
SEQUENCE LENGTH:341
SEQUENCE TYPE:nucleic acid
TOPOLOGY:linear
CLONE:HUMGS04280
SEQUENCE DESCRIPTION:
GATCAGAGGA GGAGTGTGAG TGTCCTCATA CTCACAGCAA AAGGAGGGAA TTCAGGAGAG 60
GATTGAAGAA GCGGTTCCTG TTTTTAAGGC ATTTGCAATC ATTGCTGGAG AGACAGAACC 120
AACAGATGGG GAACAATGGC CAGGGACTCC AGGCCTGATT TAAGCCAATG CCGCCCTGTG 180
GTCTTTACCC ACGAATGGTG TGGAAAATNA GAGCAGGGAA GAGGATTTAC TCACTGGCTG 240
TGGAGTTAGG TCAGAAGGGG GNTGGAAAGA TGGAACCGTA TGGNTTTGGG GACATTTCTT 300
AATCTNTAGG CTTCACCTTC CTTAACTGAA ATTAAAAGCC N 341

SEQ ID NO:3628
SEQUENCE LENGTH:341
SEQUENCE TYPE:nucleic acid
TOPOLOGY:linear
CLONE:HUMGS04281
SEQUENCE DESCRIPTION:
GATCGACCTG TCATGTTTTT AAGGTTATCC AGGCTGTATA TCTCTGGAAN AGAAGGGAAT 60
GGCAAAGAGT GGGAGTCTGA GTCAAGTTTG GTTTTGNTTT NNNAAGAGAC AAGGTCTCTG 120
TAATCCAGGC TGCAGTACAC TGGCATGNCC ATGGCTCACT ATAGACTTGG GCTCAGCCAA 180
TCTTCCTGCC TCAGCCTCCT GAGTAGCTGG GNTTGGTTTT CCCCAGGGCC ATATGTCAGC 240
TTATTTACCC TACTCACTTG GTGTNTCCCA GNCTGCCAGC CATGCTGTTC ATCCAGTATC 300

TTCTNNATGG GGATTACCTA TTAAGGGGAC TAACACAAAA N 341

SEQ ID NO:3629
SEQUENCE LENGTH:340
SEQUENCE TYPE:nucleic acid
TOPOLOGY:linear
CLONE:HUMGS04282
SEQUENCE DESCRIPTION:
GATCATGGAA CCCCTCTTCC GGCAGTTGGC CAAATGTGTC TCCAGCCCAT ACTTCCAGGT 60
GGCAGAGCGA GCTCTCTATT ACTGGAATAA TGAATACATC ATGAGTTTAA TCAGTGACAA 120
CGCAGCGAAG ATTCTGCCCN NNCATGTTTC CTTCCTTGTA CCGCAACTCA AAGACCCATT 180
GGAACAAGAC AATACATGGC TTGATATACA ACGCCCTGAA GCTCTTCATG GAGATGAACC 240
AAAAGCTATT TGATGACTGT ACACAACAGT TCAANGCAGA GAAACTAAAA GAGTTGCTAN 300
AANTGNAAGA NCGGGNNGAN GCATGGGTTA AATTAGAAAN 340

SEQ ID NO:3630
SEQUENCE LENGTH:339
SEQUENCE TYPE:nucleic acid
TOPOLOGY:linear
CLONE:HUMGS04284
SEQUENCE DESCRIPTION:
GATCTGCCAC ATGTNTGAGG GTTGCAGAGC CCGCTGTGGA GGTAAGATTG GAAACACATG 60
NAGCAGAGGG AAGACATTGA AGAAAACATC TCTNCTGGAA TATTTGGAAA AGAACACTCT 120
TCTGGACCTG GTTGAAGCAG GAAAGATGGA GGCAAAGTAG TGAAATAATC CAGAATTTCA 180
ATGCTTTTNA ATGTNCTTAG TGATACTGAC CTGTGATAAT ATAATTCCCA GGGAGGACTG 240
GGAACCTTAT CTCTTGAGAT ATTTGCATAA TTTATTTAAT TTAAGCCTCA TTCTCCTTTT 300
GTNCATTTTG GTAATAAACT GGATTTGAAT TGTGAACAN 339

SEQ ID NO:3631
SEQUENCE LENGTH:339
SEQUENCE TYPE:nucleic acid
TOPOLOGY:linear
CLONE:HUMGS04285
SEQUENCE DESCRIPTION:
GATCCAGCCA GTATAGAACT AGCTCTGTAG GGGTGAGGAG GACTNTGCTA TGTATCATCC 60
TNGATTGTNT TCCTTCAAGG AGCATTGCAC TTGCCTTCCC AGACCTTCCA CCTTAGGTTC 120
TGCTGCAAAA AGCACCAGTG CTGAAGATGC TGCAAGACCA AATCATAGCT CATAAAATCA 180
GGTCCTGAGA TAGTTACCCA TAAAGAGGAA TCCTTTGAGT GTATGCCATT GGTGAGCCGA 240
TGAGCATGGA CCATAGAAGG GCTCAATGTA GANTGAAACT ACCCGTGTGT AAAAAGAAGN 300
TTGGNTTAAA ATGGCTACTT TGGATGGGNA CCAGTGTCN 339

SEQ ID NO:3632
SEQUENCE LENGTH:337
SEQUENCE TYPE:nucleic acid
TOPOLOGY:linear

CLONE:HUMGS04286
SEQUENCE DESCRIPTION:
GATCAGAGGG GAAACAAACG CCAAGGGGTT GGGAATGGGT CGGCATCTGG AGTCTGAGAC 60
AGCTGCCCTC CTGCGTCAAG GGCTGGGGGC CTCAGCTTCC ATCCTGTGCC AGCCTCCATG 120
TGGACAGCTC AGCTTATTGT CCACCACCTG GAAATNCCTC GAAGCTTCAT NNGTCCAGGT 180
CAAGAGAATC CTGTTCCCAG ACCTCTGCCC AGCCAGGCCT TCCCAGCTCA GGGCTGACTC 240
CTGCACCAAG TGACGCACTT TTNATAACTG GTCTACCAAT GNCTCCCCTT CAAGTGAGAA 300
CTACCCTTTA CATTTAGGAG TGTGGCCCCG CAGTAAA 337

SEQ ID NO:3633
SEQUENCE LENGTH:337
SEQUENCE TYPE:nucleic acid
TOPOLOGY:linear
CLONE:HUMGS04287
SEQUENCE DESCRIPTION:
GATCCCTGTA TGCNGCCAAG GATGGGACTT CCAGCTAATG AATTTGTACA TGCAGCCAAA 60
TTTACAGGAA TTTTTTTAAA AGGCAGAAAA ACTTGAAATA CCAACATTCT GGCAAAAAAN 120
ANTCAGTTTT ATGANGAGTA AGTGGAACCT GGGNTGCAGG ANCAAAAGAG GGAAATGTTG 180
GGCAAACATT TTNNTGGGAG CTCCCTTCGC TGTTGTGCAG CAGAAACAGA TTCTNAGTTC 240
ATTTTTACTC CCACTGTATT ATAGTTTANC AAAANTNGTT TATATCTTGG AAAAAAANCT 300
TTCTGTTTAA AAAANTTAAN CAAGTGAATG TNGGAAN 337

SEQ ID NO:3634
SEQUENCE LENGTH:390
SEQUENCE TYPE:nucleic acid
TOPOLOGY:linear
CLONE:HUMGS04288
SEQUENCE DESCRIPTION:
GATCCCACTG ATTGGCCAGC CGAGCGAGAA CCAGGCTGCT GCATGGCACT GACCGCCGCT 60
TCCAGCTTCC TCTNAGCCGC AGGGCCTGCN ACGCGGGCAA GCGTGCTGCC TCTNTNCTGT 120
GTCGTTTTGT TGCCAAGGCA GAATGAAAAG TCCTTAACCG TGGACTCTTC CTTTATCCCC 180
TCCTTTACCC CACATATGCA ATGACTTTNA ATTTTNACTN TTGTAGTTTA ATCCTTTGTA 240
TTACANCATG AAATATAGTT GCATATATGG ACACCGACTT GGGAGGACAG GTCCTGATTG 300
TCCTTTCTCC AGTGTACATG TTTTACTCAC ANATAANATT NTTTCAGCAA GTAAAGNGGG 360
ANNGGGTTTT GGTNGNGNGG NGTGNGGTGN 390

SEQ ID NO:3635
SEQUENCE LENGTH:73
SEQUENCE TYPE:nucleic acid
TOPOLOGY:linear
CLONE:HUMGS04289
SEQUENCE DESCRIPTION:
GATCTGGCCA TTTGGTACAT AATCCAGCAC AGATAAGCTG GGTGGTAATG ATAATAAAAA 60
TGGTTTTCTC AAA 73

SEQ ID NO:3636
SEQUENCE LENGTH:328
SEQUENCE TYPE:nucleic acid
TOPOLOGY:linear
CLONE:HUMGS04290
SEQUENCE DESCRIPTION:
GATCACCTGG GTTTCTTTAT TTATCGACTG TGTCATGACA AGGAAACTTA CAAACTGCAA 60
CGCAGAGAAA CTATTAAAGG TATTCAGAAA CGTGAAGCCA GCAATTGTTT CGCAATTCGG 120
CATTTTGAAA ACAAATTTGC CGTGGAAACT TTAATTTGTN CTTGAACAGT CAAGAAAAAC 180
ATTATTGAGG AAAATTAATA TCACAGCATA ACCCCACCCT TTACATTTTG TGCAGTGATT 240
ATTTTTTAAA GTCTTCTTTC ATGTAAGTAG CAAACAGGGC TTTACTATCT TTNCATCTCA 300
TTAATTCAAT TAAAACCATT ACCTTAAA 328

SEQ ID NO:3637
SEQUENCE LENGTH:328
SEQUENCE TYPE:nucleic acid
TOPOLOGY:linear
CLONE:HUMGS04291
SEQUENCE DESCRIPTION:
GATCCTGTGA ACCGTTACTT TGCCTAAATC ACTTGAGACT TGAGTNTTTA ATAACAAAGC 60
ATCAATATTC ACTAAAGTCA ATCTNTTTTN ANTTTCTGTN ACTTGGCTAG AAGCTCTTGA 120
CACTAAGGGA TTAGTGTTAA TTTTCCCTGG GGGTGTTCCA CTAGGGCATT ACTGTATAAT 180
GACTTGATGT TGCCACATAG ACTTCAAGAT ATATAATATT TTNAGGATTT TNTTGATTGG 240
CCTATGTTTT ATTGCATAGT GTGAACCGTG TAAAGCTTGG TTAACCTGTA TATAGATAGC 300
TTATTGTTGA CTAGTTATAG TGTATTTN 328

SEQ ID NO:3638
SEQUENCE LENGTH:326
SEQUENCE TYPE:nucleic acid
TOPOLOGY:linear
CLONE:HUMGS04292
SEQUENCE DESCRIPTION:
GATCCTTTCC CTGGATACAT AACCCTAGTA GATAATGTTA CTGCAAGATG TAATGNCCAA 60
ATCCAACCTA CTGCAAATNT TACTATGGAA ATCTCAATGC AAAAGTAGCC TTTGTNATTT 120
TTTTTTTAAA CCGTCTCCAT AACAGANCAC TTAGAATTAT TAGCCATTTG CTGCAAAGTA 180
TTTGTTTTAA AATGTCTGGC ATATACACCA CTAACATTTA ANTGCCCAAC ATTCGGTCTA 240
TACGTGAAAA ACCTAGANGA AANGCCCTGT TGGAGTTTTG ATGTAGAGTG CAATANTACA 300
TCAATAAAGG TATTTTAAAA TGGAAA 326

SEQ ID NO:3639
SEQUENCE LENGTH:326
SEQUENCE TYPE:nucleic acid
TOPOLOGY:linear
CLONE:HUMGS04293
SEQUENCE DESCRIPTION:

GATCTGTTGT ATGTAATAAG TAACACAGAG TTTTAAAACA AATTAATTAT TTAGCTTTAT 60
TGAAGTTTGT NTTTTNCCTT CCGAACCTGG AGTATCATAT TATAAACAGC AGTTTCACAC 120
CAGAATAGCA GTGCCCTTNC TTTTTGTACA TACTGATTGG NCCTTTCTTT ACTGTTACGT 180
GGACACTTTC TATGTTAGTT TTGATGCATA ATTCTTTGAA TCCTTTTATA CAAACTAGAA 240
TGTATGTGTA AGAATACCTG TCCCTGCAAT GTAGTAACTA CAAACTTATT TTTAAATAAA 300
TAGAAAACTT GTTTTTCTAA GCTAAA 326

SEQ ID NO:3640
SEQUENCE LENGTH:325
SEQUENCE TYPE:nucleic acid
TOPOLOGY:linear
CLONE:HUMGS04294
SEQUENCE DESCRIPTION:
GATCCAAGAG TTTAGTGATT TCAAAAGCCT TGGTCTCAGG AGAAGATTAA ACTTTCATAT 60
TGGGCAGTGG TTCACTTTAA AACACACACA TACACACACA AAACAATTTT TTAAGAAATC 120
CTAATAAGTA ACATACCCAA AATGCTCTGT CTTGAGTCAT GAGAACCATC AGTTCTTGAT 180
ATTGTCTAGA CTTGCATCTA GAGCTACGTT GTAAAATTCT TTTAGGCATG TGTTAGATTT 240
CTGTGTAAAC TTTGTTTAAA TGTAAACTTC ATACTACATT GTCAGTTTTT GTCTTAATAA 300
AACTATAGAT TTATAATCCC TGAAA 325

SEQ ID NO:3641
SEQUENCE LENGTH:391
SEQUENCE TYPE:nucleic acid
TOPOLOGY:linear
CLONE:HUMGS04295
SEQUENCE DESCRIPTION:
GATCGGAATT TCCACCAATC AGCGGGAAGC CTCGGCCCTG TAACTGCTAA TGGGAGACAG 60
CAGCGCCACG CCACAGGCTT TTCCCCTGGT TTCGGGAGGG NTGGGGAGCC AGGTGGGGCT 120
CCCGNCCAGA CCCTTTCCCG AGGTCCGNCC TCTCCGCCTT NTCTNTAAAT TCCTCTTTTG 180
AGTNCCCTCC CTTCCGGTTG AGAGGCGGGG GTTGGCCCGT AGTTGTACAC TCAGTCACCC 240
TGAACTGTGG AGGNGGNGGG NCTCCNTTGT GGACTGATTT GCGTGGGGAT TTGGTNTGTT 300
TTATTAAGGG ATTTGAAAAA ATTCAGATGG ACTTNACTAG TATGGACTGT TNGGGTAAAT 360
ATTNGCTTNC NGGGTTAATA AAATGGACAG N 391

SEQ ID NO:3642
SEQUENCE LENGTH:324
SEQUENCE TYPE:nucleic acid
TOPOLOGY:linear
CLONE:HUMGS04296
SEQUENCE DESCRIPTION:
GATCCAGTCA ATACATGCAG ACCAGTAAAA TCTGATTTGT GCAGAGTTCT CCATCTGACT 60
CTCACTTATT TCTGTAGATA TATACATATA TAAATACAAG TATGTNCTTA CGGCACAGTA 120
TTGCTGACCT TTAGTTCGAG GTTTTGTCGG TTGTTGTTTA TTTTCTTCCT CTTGCAAGTG 180
CTATCCATGT GAGTGTGTGA AGTTTCTCTA ATAAGTAAAA CACAGGCCCT TTTCCTTGTT 240
TGTTTTGTGT TAGTTTATTG TAAACAGCCA TTTGTTGTAA ATNATTATTG GCATTAAATT 300

ATAATTTATG ATTTTCAAAG CAAA 324

SEQ ID NO:3643
SEQUENCE LENGTH:323
SEQUENCE TYPE:nucleic acid
TOPOLOGY:linear
CLONE:HUMGS04297
SEQUENCE DESCRIPTION:
GATCCAGTCT AGGCCCGTAT GTTGCATTTT GCTGTCATGT CTTCTTAGTC TCCATCNCAT 60
CTCAGTGGTC TTTTCTTTTC CATGACTGTG ACAGAAATGA TTTGTATCCT TCATATATCT 120
GCAGTCACAT GAAGTCTATT TGTCCCACTA CTGGTGACGT AAATTTTGGT AACTTGGTTG 180
AGGGTGGTAC CGTCTAGGTT TCTCCATATT AAGTTGCTAT TTTNCCTTGT GTATTTAATA 240
AGTATCTTAT AGGGAAATCC TTTGAGACTA TGTAAATATC ATATTACATG TATTTNGCCT 300
GTTTTAGCAT CTTTTGAAGA TAN 323

SEQ ID NO:3644
SEQUENCE LENGTH:320
SEQUENCE TYPE:nucleic acid
TOPOLOGY:linear
CLONE:HUMGS04298
SEQUENCE DESCRIPTION:
GATCTGGAGC ACAAGGAACT CCCATACATT CCTAGTGGGA ATGCCTAATG TTAGAGTCTG 60
GAAGACATTT TGGCAATTNC TTAACCAAGC TCTTCTTACC ATAAGATGAA GCAACTCCAC 120
ACCTTGGTAT TTACCCAAAC GAGTTGAAAG CTTATGCCCA TACAAACACC TGTACAAGAA 180
TGTCTATAGC AGCTTTATTC ATAATTGCCA AANCTTGAGG GCATCCAAGA TATCACTTAA 240
TGGGTGGACA GATAAGCTGT GGTACATCTA TACAATGGAA TATTAAGGTG CTAAAAAGAA 300
ATGAGCTCTG AAGCCACAAA 320

SEQ ID NO:3645
SEQUENCE LENGTH:319
SEQUENCE TYPE:nucleic acid
TOPOLOGY:linear
CLONE:HUMGS04299
SEQUENCE DESCRIPTION:
GATCGGGATT AATATTAGTG CATTTTTAAA ACACTTAAAA GTTTTACAAN TGTATTTGTG 60
TATTTCCTGT TTTGGTGCCT GTTTTATAGA AGATGATTGA NGAATCACTG AAGATTAAAA 120
TAAAAAAGGA ATTAGAAATG GAAAATGNAT TAGAAATGAG TAATCAAGAA ATAAAAGACA 180
AATCTGCTCA CAGTGAAAAT CCTTTAGAGA AATACATGAA AATCATCCAG CAGGAGCAAG 240
ACCAGGAGTC GGCAGATAAG AGCTCAAANN NGATGGTCCA AGAAGGCTCC CTAGTGGACA 300
CGCTGCAATC TAGTGACAN 319

SEQ ID NO:3646
SEQUENCE LENGTH:318
SEQUENCE TYPE:nucleic acid
TOPOLOGY:linear

CLONE:HUMGS04300
SEQUENCE DESCRIPTION:
GATCATAGAA GAAGAANCCA GTAATTGANC AAATCCTATT TAATGACATC CTTGTAGCAT 60
AGATGGTCTA TAATGCTGAC CACAGATTTC TTAGAAATGC TGCTCTCTCT ATTTAACTAA 120
CATTTTGTNC AGTTTTGCCT CCAGTGGAAG CAGAAAGGGT TTTTNCAGCT GTTAAATCCT 180
AAAANTCAAT ATAATTNATT TATGTAAGAA AAATAACTCA ATCAATATAT TTTTGAACCT 240
TTTAAGTACT AATTTNNTNT TTATCAAGTA GAAAAAAAAT GTATTTGCCC TAAATCCTTA 300
AAATACAAAT GCTATAAA 318

SEQ ID NO:3647
SEQUENCE LENGTH:317
SEQUENCE TYPE:nucleic acid
TOPOLOGY:linear
CLONE:HUMGS04302
SEQUENCE DESCRIPTION:
GATCCTGAGG CTTTGGCTGA AATTCTGCTT GAAACTTGTA AATCAATCAC ATATTCTTTA 60
GAAAATAAGA CCTACTCATC TGTGAGAACA GAAGCTTTAT CTGTGATAGA ATTGCTGCTT 120
AAAAAACTTG AAGAATCTAA ACAGTGGGAA TGTTTGACAT CTGAATGCAG AGTGCTCCTA 180
ATTGAGTCTT TAGCTACTAT GGAGCCAGAC AGCAGACCTG AACTGCAGGA GAAAGCAGCG 240
TTACTGAAGA AAACACTTGA AAATCTGGAA TAANTTAGAN GGGGAAGAAA CAAACAAGTG 300
CCATGTTCAT TGGGGGN 317

SEQ ID NO:3648
SEQUENCE LENGTH:317
SEQUENCE TYPE:nucleic acid
TOPOLOGY:linear
CLONE:HUMGS04303
SEQUENCE DESCRIPTION:
GATCCTTCTC CTTCCCTGGG ATACAGCACC TCCACCCTGC CAGGCCACCC NTGTAAAGGC 60
AGCCAATTCA GAGCCACCTC TTATGCCTGC ATCTCCCCCA ACAATTCCTG CTGGGAAACC 120
ACTCCTCGTA TGGTGTCATC TACACCAGTG GGTTTCAAGC TTGAGAAGAA TCAAGTTCTA 180
CAGGGCTGCT TAAAACACAG GTTGCTGGGT CCTGTNTCTC AGAGTTTTTG ACTCAGTCTT 240
GGTTGAGAAT TTTCAGTTCT AGAAGCTCCT GGGTAATGCT AATCCTCCTG TTCTAGGGGC 300
CATAGTCATT GTCCATN 317

SEQ ID NO:3649
SEQUENCE LENGTH:317
SEQUENCE TYPE:nucleic acid
TOPOLOGY:linear
CLONE:HUMGS04304
SEQUENCE DESCRIPTION:
GATCTACACG CGGCTGGCCA CCATCTACCA CAACTNCCTC CTGGACCGTG AGAAGTCGCT 60
CTTNTTCTAC CAGAAGGCCA GGACCTTCGC CACAGAGCTC AACGTCCGCA GGGTCAACCT 120
GCCTCCTCTG CCACTCTGCG GGTGGGCCCC CTGGTTGGCC CCCAGCCACC CTNGCTGAGG 180
ACAGCATCCA AGGGAGTGGG TTTTGTGCAA GGGCTAGGGG TCTCCTGCCT NTCCTGGTGT 240

CGNCGGTGGC TCATTTTCTN GCAAATNGAG GCACGAACGC AGGGNCCAAA TAGCAATAAA 300
TGGGTTTTGT TTTTAAA 317

SEQ ID NO:3650
SEQUENCE LENGTH:317
SEQUENCE TYPE:nucleic acid
TOPOLOGY:linear
CLONE:HUMGS04305
SEQUENCE DESCRIPTION:
GATCTATGCA ATTTTTAAAC TAGTAATGGG CCAATTAAAA TATATATAAA TATATATNTT 60
NCAACCAGCA TTTTACTACT TGTTACCTTT CCCATGCTGA ATTATTTTGT TGTGATTTTG 120
TACAGANTTT TTAATGACTT TTTATAATGT GGATTTCCTA TTTTAAAACC ATGCAGCTTC 180
ATCAATTTTT ATACATATCA GAAANGNNCG ANTTATATCT AATTTATACA AAATAATTTA 240
ACTAATTTAA ACCAGCAGAA ANGTGCTTAG GAAAGTTATT GTGTTGCCTT AGCACTTCTT 300
TCCTCTCCAA TTGTAAA 317

SEQ ID NO:3651
SEQUENCE LENGTH:316
SEQUENCE TYPE:nucleic acid
TOPOLOGY:linear
CLONE:HUMGS04306
SEQUENCE DESCRIPTION:
GATCTGAATT CTAAAGCGTC TTTCATTGTT TACTTTTAGG ATGAGGAAGT ACAGACAATT 60
GGTCAGATAG AACTGTGCCT CACTAAGCAA GACCAGCAGC TGCAAAACTG CACCGAGCCG 120
GGGGAGCAGC CGTCCCCCAA GCAGGNNGTC TGGCTGGCAA ATGGGGCCGC CGAGAGCCGG 180
GGTCTGAGAG TCTGTGAAGA TGGCCCAGTC TTCTATCCCC CACCTAAAAA GACCAAGCAT 240
TGATGCCCAA GTTTTGGAAA TATTCTGTTT TAAAAAGCAA GAGAAATTCA CAAACTGCAG 300
CTTTCTAAAA AACAAA 316

SEQ ID NO:3652
SEQUENCE LENGTH:374
SEQUENCE TYPE:nucleic acid
TOPOLOGY:linear
CLONE:HUMGS04307
SEQUENCE DESCRIPTION:
GATCCTTGGG TTGTAAAGAG AGTAGAAACC CTAGGNAGCA GTGCTTTTGG GTCCTAGAAC 60
CTGTTGAGTT TCTAATGAAT ATTTGTAGAA TCTNATAAAA CAGTTTAAAT ACAAGCTTAA 120
GTGGCTTATG AATCCTGTNA AGCTCATTTA TGGNCTAGTG TAAAACAATG TGAAGCTCTA 180
CTAAGTNCTG TCCTTAATCA TAAATAATAG CCCCTTGNGG NCTAGCCTGT CCTCTGGTCA 240
CCTTACCAGT TGGGTTGCAC ATTGTGTGGT CGTCCAATTA AACTCAATCT TNCGTGTGNC 300
AGGNGNTAGT CTNTCCAATC ATGNCATAGN TTTCATCTGG TTTTTGNCTG GTGGACGNAC 360
CTTCGGANTA ANTN 374

SEQ ID NO:3653
SEQUENCE LENGTH:316

SEQUENCE TYPE:nucleic acid
TOPOLOGY:linear
CLONE:HUMGS04308
SEQUENCE DESCRIPTION:
GATCAGGACC AACCTGGAAA ATGCCAGATT TGGCTATGCT TTTTAANACA TTNATTTGGA 60
CAGCTCACCA ACCCCTGAAG GGGCCGTTAA GCCTGGTTAG TTTTNTACCT ACTCCGAGTN 120
TCCTCCCCTG CCCCACCAGA TTGCTGCAGG GGCGCGGTGT GCCTGGNAGC CAAATTNTTG 180
ACACTTCTTT TTTCCTATGC ACTGGTTTTA CACAGCTGTN ATTTTTNTTT CAAAATTGCA 240
GCAGTCCCAC AAGATGTNTG CATTTGGACA AATAGTACTT AAAAACAAAN CAAACAAGCA 300
CTCAGCCCAG TTCCTN 316

SEQ ID NO:3654
SEQUENCE LENGTH:315
SEQUENCE TYPE:nucleic acid
TOPOLOGY:linear
CLONE:HUMGS04309
SEQUENCE DESCRIPTION:
GATCTAATGC AGAACTTATC AGATATTGTG AAAAATCAGT GTGGGAGGTA TTCAGTCAAA 60
AGTCAGAAGG AAATGTATTA TCGTTATAAT TTCAAACCTT AAGAAAGCCT AAATTACCAA 120
TTGCATATCA AATTTTTTTG ATAATGTAAG GAAAACTTAA ATCCCCTCCA TATCCTAATT 180
GGCATTTATA ATAATATGAC ACTTAACCCT GACAGCTAGA GTCTTACTTT ATGCTAATGC 240
TAGATATCTA TTTTNAANTT CCACGTGTGT AAACATGCTA TACTCTTCTG ATATTAANCT 300
TACTAAGTAC ATAAA 315

SEQ ID NO:3655
SEQUENCE LENGTH:315
SEQUENCE TYPE:nucleic acid
TOPOLOGY:linear
CLONE:HUMGS04310
SEQUENCE DESCRIPTION:
GATCCAAGAA GAACTAGAAA AAAAGTTGTT TGCAGGCTTG CAACCTCTCA NAAATTTTAA 60
GGCTAGCTCT TCAACTATGT CTTTNAAAAA GAATATGTCT GCCCACAGCA GCCAGAAGTG 120
ACAGCATTCT TGTGAATGTT TACATCCTGC TGCTGATAGG TTCAAGGAAT TCTTTAAACC 180
AGATTCTGCT GACATGAGAA GCTCCAGTGA TTTGTTTTTT TGAAGGTCTG TATCAGAGCA 240
AGCCTAACAT CTAAATAANG TTTGATTCTG GATTTTCCAG GTGGGAAATA AATAAAGAAA 300
ATTTGGCTTG TCAAA 315

SEQ ID NO:3656
SEQUENCE LENGTH:347
SEQUENCE TYPE:nucleic acid
TOPOLOGY:linear
CLONE:HUMGS04311
SEQUENCE DESCRIPTION:
GATCCGTGCC CGCNTCCAGG AGAAGTTGTC ACCTCCCTAC AGCTCCCCAC AGGAGTTTGC 60
CCAGGATGTG GGCCGCATGT TCAAGCAATT CAACAAGTTA ACTGAGGACA AGGCAGACGT 120

GCAGTCCATC ATCGGCCTGC AGCGCTTNTT NGAGACGCGC ATGAACGAGG CCTTCGGTGA 180
CACCAAGTTC TCTGCTGTGC TGGTGGAGCC CCCGCCGATG AGCCTGNCTG GTGCTGGCCT 240
GANTTCCCAG GAGCTGTCTG GTGGCCCTGG TGATGGNCCC TNAGGCTGGG GGCCCCATGG 300
TCAGCCCAGN NTGGGTTCTG TTCTNTGGNN CTTGTCANNC NNAANCN 347

SEQ ID NO:3657
SEQUENCE LENGTH:313
SEQUENCE TYPE:nucleic acid
TOPOLOGY:linear
CLONE:HUMGS04312
SEQUENCE DESCRIPTION:
GATCATAACT GAACCAGAAT TTGGATTATA GANGTGCACT TCCTAGCTGT TTTTTATTTA 60
GGTATAGGAA CACACAATGA GAACCTTATG TTTAGAATGT AGCAGTAACA ACAGAGGGTA 120
ACTACACTGC AAGACTTAAA TGGTTTGAGC ATGTGACACT ATCCTTTTGT TGGGGGGAGC 180
AGGGTATAAN ANTATACATA CAGGAGATTA GTTTAATAGT AAAGTTTTAT ATGTATGTTT 240
AACTTCCATG ATATTTATNA TTTNGTAGAG ACATTTGTGA TTGTNTAGAG TTCTNGTNTT 300
TNTTCTATTT ATN 313

SEQ ID NO:3658
SEQUENCE LENGTH:307
SEQUENCE TYPE:nucleic acid
TOPOLOGY:linear
CLONE:HUMGS04313
SEQUENCE DESCRIPTION:
GATCTTAAAA GTGGAAAGCA GAAAAAATAC TGTTTCCTGA AATTNAAAAG TTTTGGCAGT 60
GCCCAGCAGG CCCTCAACAT TCTCACAGGC AAGGACTGGA AGCTGAAAGG CAGGCATGCC 120
CTAACCCCCA GGCACGTCCA TGCCTGGNTC AGAGGCTTAC CACCTGANTC AACAAGGCTC 180
CCAGGGCTTC GTGTTGTACC TCCCCCCTTT GAACAGGAGG TCTTGCAGAC TCTGAAACTG 240
GACCACCCGA AGATAGCAGC CTGGCGCTGG ANGCCGGAAG ATTGGAAAGC TCTACAACAG 300
TTTGTGN 307

SEQ ID NO:3659
SEQUENCE LENGTH:306
SEQUENCE TYPE:nucleic acid
TOPOLOGY:linear
CLONE:HUMGS04314
SEQUENCE DESCRIPTION:
GATCCCCAGA GAGGTGGCCC TGGACCTGGA GTCAGGAGGC GGTGTCCAGA AGAGTCGCTC 60
ATCAACCCCG GGTTCAAGAG TAAGAAACCA GCTGGTGGCG TGGACTTCGA TGAGACCTGA 120
AGGTGCAGCA CAAGCGTGGC CCCGCGGGGA GTCCGCCTAT NAGGGGAGAG GCAGTCTTTG 180
AGGCCCCCAT CAGAGACCCC CCGCCACCAC CTCCACCTGC CTGTCCTGGG CCAGGACTAA 240
CACGGCTCCT CAAATTCCTT CCCTGTCAAA TAAACAGCTC CCTTGGTTGG AGGCTCTNGT 300
GGGAAA 306

SEQ ID NO:3660

SEQUENCE LENGTH:371
SEQUENCE TYPE:nucleic acid
TOPOLOGY:linear
CLONE:HUMGS04315
SEQUENCE DESCRIPTION:

```
GATCCCCTGA GCAAGTCAGA AGTTACTCTC ATCAGTCGTT CATGGTCACA ACCTGAGGTA  60
CTCTGCTGAG TGGGCAAGGC TGAAGAAGAG GCCTGTGGAA TGCAGCATTA CCTGCTGGAC 120
AGAGCAGGGC AGGCAGTTCT ATGCCTTGGA GCTCCTGACT GCAGGGACTC TGTCCCCACA 180
CTCAGAAAGA CTCAGCTCAC TCAATGAGAG AATGTGATTT ACTTTATAGA ACGTATAATC 240
AACTTTGTTG AATAATTTGT TCTATTAAGG CTGTCTAAAG TATGTNATGT CTTCATCATA 300
GTATGAAGTG TTGAAAATTA ATAACGGGCC TAGTTTAGGA AAAAGCTGCT TAAANGNGTG 360
TGGCCCGTAN N                                                     371
```

SEQ ID NO:3661
SEQUENCE LENGTH:304
SEQUENCE TYPE:nucleic acid
TOPOLOGY:linear
CLONE:HUMGS04316
SEQUENCE DESCRIPTION:

```
GATCTGTATT GATATTTGAT AGTGAAAATG TTTATTCAAT TTTTCCCCAA AATGTATTTG  60
TTCCTCTTAC TTGTTTATTC AGCTGTGGTG ACTTCAAAAT ACTTTTTCCA GGTGACAAAA 120
AAAAACTCAG AAAAATTTTT AATCTTAATT GCATTAATAC TATAAATTGA TTTGGAAGGA 180
TTTAACATTT TATAATATNG GNNTTCCTCT GCAGTAATGT GGCATATTCT TCCNNNNGTT 240
CAAGCATTAA AAAAATCTCT TAGCAAAGCT GCACAGCCTT CTTCATAAGG GTTCTCTATA 300
TTTN                                                             304
```

SEQ ID NO:3662
SEQUENCE LENGTH:303
SEQUENCE TYPE:nucleic acid
TOPOLOGY:linear
CLONE:HUMGS04317
SEQUENCE DESCRIPTION:

```
GATCTGCGGT GGGCCAAGAG TATCCAGGGA GAAACCTGTC CCTGGCAGGG CTGGAGCAGG  60
GACACTCCAG GCCCAGCTGA GGCTTCCTGC TCTGCATGGC AGGGGACATC TCTNACCTGG 120
TCAGGACCCT GGAGGTGGGA CAGCTGGTGC TAAGGGAATG TNAGCGCATC AGGGTTGGGG 180
AGAAGCAGTG GGTGGTCCAG GAAACCCCTG AAACAGCTGG CCCTGGGGTC CCCTTTCAAG 240
TGTTTCCAGT TGTGGCAAGG GGTCAAGGAG ACCTTGCATT GCAAATCTNT TAGAAAAAAC 300
AAN                                                              303
```

SEQ ID NO:3663
SEQUENCE LENGTH:302
SEQUENCE TYPE:nucleic acid
TOPOLOGY:linear
CLONE:HUMGS04318
SEQUENCE DESCRIPTION:

GATCTGAAAG ATTACATTTA GAACATTTAG TACTATTACA CTCTCAGAAA CTGTNGTAAA 60
AAGTCACATT TTCAAAACTT CATGCATATT GTATTCTTGT TGGAAATAAG TCCTATAGTT 120
TCTTAATTGT CTTCATGCCG TCCATATTTA ACAAACATTG CAAGTCCTTT TTATATTTGG 180
GTAATNATTT GTAATTTAGT GAAGGAGACT AGGGGATGTT TTCTTCCAAA GGGAATTTAA 240
AATCAATTTT ATGGTATTTT GAAAGTAAAA TACTCCTTAA CGTGTCAATA TTTTTAAAAT 300
GN 302

SEQ ID NO:3664
SEQUENCE LENGTH:301
SEQUENCE TYPE:nucleic acid
TOPOLOGY:linear
CLONE:HUMGS04319
SEQUENCE DESCRIPTION:
GATCCTCTCA GAGTGCCAGG CATCCCCCCC GNACCAGCAG AGGCTCATCT TTATNAGGCA 60
AGCAGCTGGA AGATGGCCGC ACTCTTTNTG ACTACAACAT CCAGAAAGAG TCGACCCTGC 120
ACCTGGTCCT GCGCCTGAGG GGTGGCTGTT AATTNTTCAG TCATGGCATT CGCAGTGCCC 180
AGTGATGGCA TTACTCTGCA CTATAGCCAT TTGCCCCAAC TTAAGTTTAG AAATTACANG 240
TTTCAGTAAT AGCTGNACCT GTTCAAANTG TTTAATAAAN GGTTTCGGTT GCATGGGTAA 300
A 301

SEQ ID NO:3665
SEQUENCE LENGTH:300
SEQUENCE TYPE:nucleic acid
TOPOLOGY:linear
CLONE:HUMGS04320
SEQUENCE DESCRIPTION:
GATCTGAAAA ATGAAGTTTG GTATCAAAAA NGTCATGACC CTGTATGTAA AGCAGAAAAC 60
TTCAAATCTG ACTGTTTCCC ATTACCTGCT GAAGTATGTC TCCTGTTTCT TTTCAAAGAG 120
GAAGANGAGT TTACAACAGA TGANGCAGAT GAAACTAGGA GCAATGAAAC CCAGAATCCT 180
CATAAACCAT CACCTAGCAG GCTGTCAACA GGTGCATCTN CTGATGCTGT CTGGGNTAAT 240
GGCATTGATG ATGCTTATTT TTTAGAAGCT ACTGAAGATG CTGAATTAGC TGAAGCTGCN 300

SEQ ID NO:3666
SEQUENCE LENGTH:300
SEQUENCE TYPE:nucleic acid
TOPOLOGY:linear
CLONE:HUMGS04321
SEQUENCE DESCRIPTION:
GATCACTNAA TGGTCAAGTG GAACAAGCAC TACACGACTA ACCCCTATTG GGGTTTTTAA 60
CTTAAGGGAG GCTAATTTTT AATTTAAACT GCTCGAGATA TGAGTTCTGC AAAAGGTGGT 120
CCGCATCCTT GGCCCTCTGG ACATTATCAC TAAATTGCTT GTGCCTGTTA ACAAGAATAC 180
TGACCAGAAT GCTCTTCATG TAGCTTATAC AGTTGGTTCA CTTCATGCGG TTCTTGACAT 240
GTTTATTTCT ACCCTTAATG CAATGAAATG TTTCATTAAT AAAAAACCAC TTTATATAAA 300

SEQ ID NO:3667

SEQUENCE LENGTH:417
SEQUENCE TYPE:nucleic acid
TOPOLOGY:linear
CLONE:HUMGS04322
SEQUENCE DESCRIPTION:
GATCTTTCTG GCTCCACTCA GTGTCTAAGG CACCCTGCTT CCTTTGCTTG CATCCCACAG 60
ACTATTTCCC TCATCCTATT TACTGCAGCA AATCTCTCCT TAGTTGATGA GACTGTGTTT 120
ATCTCCCTTT AAAACCCTAC CTATCCTGAA TGGTCTGTCA TTGTCTGCCT TTAAAATCCT 180
TCCTCTTTCT TCCTCCTCTA TTCTCTAAAT AATGATGGGG CTAAGTTATA CCCAAAGCTC 240
ACTTTACAAA ATATTTCCTC AGTACTTTGC AGAAAACACC AAACAAAAAT GCCATTTTAA 300
AAAAGGTGTA TTTTTTCTTT TAGAATGTAA GCTCCTCANG AGCAGGGACA ATGTTTTCTG 360
TATGTTCTAT TGTGCCTAGT ACACTGTAAA TGCTCAATAA ATATTGATGA TGGGAAA 417

SEQ ID NO:3668
SEQUENCE LENGTH:298
SEQUENCE TYPE:nucleic acid
TOPOLOGY:linear
CLONE:HUMGS04323
SEQUENCE DESCRIPTION:
GATCTCTGAA AGCTCAAATG GATGGAATTT AGTTTGCGGG AAAGAGGCTT TGCTTTGCGC 60
ATATCAGGCT TAGGACTGTG GGAGGCTTAA GAAGCAGATG CTTCTTTNAT TGTACTCTTG 120
TNCTGCCCTT GTTTTTTGAA GGCTCTGACT TATAACTNCT GTATCAGAAG AAACATTTTG 180
ACAGTGTCTT GGTTGGAGAT GAACATCCCT AATTGACATG TGATGACTAT TTCTNATTCC 240
ATTCATCTAA GAGTCATTGA AATTTTGTTT TGCTTGTTTG TTTAGCTTCA AGGTCTTN 298

SEQ ID NO:3669
SEQUENCE LENGTH:294
SEQUENCE TYPE:nucleic acid
TOPOLOGY:linear
CLONE:HUMGS04324
SEQUENCE DESCRIPTION:
GATCATCTCT ATATCTAATT TGTATTATNA CTAATCTTAT TCCAACTATT TNCTTTATNA 60
TACTGAAACA GTTTGTNCCT TCAGTCTCTG CTTCGGCTAC CTAAAAGGGA AGGGCCCCCT 120
ATCCTGTAAT CACATGACTT ACTTCACCTT GTCAATCACT GAGAAGATTC ACCCTCCTTA 180
CCCTGCCCCC TTGTCTTGTA TGCAATAAAT ACCAGTGGGC CTAGCCCTCT GGGACCACTA 240
CCGGTCCCGG GGTCTTGATG GTAGTGGNCC CANAGCCCAG CTTGCNTNCN CAAA 294

SEQ ID NO:3670
SEQUENCE LENGTH:293
SEQUENCE TYPE:nucleic acid
TOPOLOGY:linear
CLONE:HUMGS04325
SEQUENCE DESCRIPTION:
GATCAAGAGG CCACCAGTTG TACTTCAGCA CCAATGTGTC TTACTTTATA GAAATGTTGT 60
TAATGTATTA ATGATGTTAT TAAATACTGT NCAAGAAGAA CAAAGTTTAT GCAGCTACTG 120

TCCAAACTCA AAGTGGCAGC CAGTTGGTTT TGATAGGTTG CCTTTTGGAG ATTTCTATTA 180
CTGCCTTTNN NTNCTNACTG TTTTATTACA AACTTACAAA AATATGTATA ACCCTGTTTT 240
ATACAANCTN GTTTCGTAAT AAAACTTNTC CCTTTTTTTA AAATGNAANT AAA 293

SEQ ID NO:3671
SEQUENCE LENGTH:292
SEQUENCE TYPE:nucleic acid
TOPOLOGY:linear
CLONE:HUMGS04326
SEQUENCE DESCRIPTION:
GATCTATAGT ATTTTAATGT GCATCTACTT TAAATNAGTC ATCTTGGGGT TTTNATAATT 60
CCCTTATGTT CTTGCCCCTC TACACTTGAA ATAACAAAAT GCCTTAATTT TATGGATTAG 120
TTCTCTTATA GTAGACAGGC AGCTATATGC AGCAAAACCA ATAAAGTTAT TTTTCAACTT 180
TCATAGTTGT AAAATATCTT ATAACAGAAT ACAAAACAGC TANGAAAACA TGCCACATTT 240
NATTTTNGCA TTTTCAAATA ATTNGTTTTT GGTGTAAGCA CAGGATAAAA AN 292

SEQ ID NO:3672
SEQUENCE LENGTH:291
SEQUENCE TYPE:nucleic acid
TOPOLOGY:linear
CLONE:HUMGS04327
SEQUENCE DESCRIPTION:
GATCAAGGAT GCAACCTCAG GTTGAGAAAG AAACAGGGTT CCCTGGGCCC ATTAGACTGT 60
TTGCAGGGCA TCACTGCTTC CCCCTGACAC CTCACAACTA GCAAAAATTG TCTTTGTCTT 120
TGGAAATNAT AGAGGGATTT GGGTATCCAG ATTGTGCAGA TGCAAACTTA GGCTGTCTTG 180
ATGNNNACTT AGAACCACAG AAATGCTTTT AAAATGCCTG TTTTAAGATG GAATTGTTGT 240
TTTNATAATT TGATTTTAGT GCTAAATAAA TGATTGGCTT TGTACATGAA A 291

SEQ ID NO:3673
SEQUENCE LENGTH:291
SEQUENCE TYPE:nucleic acid
TOPOLOGY:linear
CLONE:HUMGS04328
SEQUENCE DESCRIPTION:
GATCTAAAGG CTCTGGNTTT GCCTTCTTTC AGTCTGACAC TGACTAACAA CCTCGACAAG 60
GTGGGCATAT ACCTGGATTA TGAAGGAGGA CAGTTGTCCT TCTACAATGC TAAAACCATG 120
ACTCACATTT ACACCTTCAG TAACACTTTC ATGGAGAAAC TTTATCCCTA CTTCTGCCCC 180
TGCCTTAATG ATGGTGGAGA GAATAAAGAA CCATTGCACA TCTTACATCC ACAGTAATGA 240
GTCATAATAT TATACAAATT CAGAGTGTTA TTAAAGAGGT TTTGAAATAA A 291

SEQ ID NO:3674
SEQUENCE LENGTH:290
SEQUENCE TYPE:nucleic acid
TOPOLOGY:linear
CLONE:HUMGS04329

SEQUENCE DESCRIPTION:
GATCCCAAAA GCTGTTTTNA GGTATAAGGA CAAGGAGAGG AGACAAGTGA CGACAGCCAT 60
TCCCCTTTGC AGCTATCTAC TGTAGTGACA GCCATTTCTT GGTTGATGGG TTGGAAGTCA 120
TCAGAGGTTT GAAGAATTAC ACTGGCCTTT GTTTTTCNNG AAATGCCGAC CATGGAGATG 180
CTTTAGAGTC TTCTCAAATA GCTTAGATGT TGTAATGNGG TTAAGCTTTG CTTCATAAAA 240
CAGGGGCCCT CAGAAGTTCT CCTTTAAATT TTTCANATAA AAAATTTAAA 290

SEQ ID NO:3675
SEQUENCE LENGTH:288
SEQUENCE TYPE:nucleic acid
TOPOLOGY:linear
CLONE:HUMGS04330
SEQUENCE DESCRIPTION:
GATCTATCCC TGTCTCACTG AAAGCCCCTG TGTAGTGTCT GTGTTGTTTT CCCTTGACCC 60
TGGNNTTTCC TATCCTCCCA AAGACTCAGC TCCCCTGTTA GATNGCTCTG CCTGTCCTTC 120
CCCAGTCACC AGGGTGGGGG GGACAGGGNN AGCTGAGTGC ATTCATTTTG TGCTTTTCTT 180
GTGGGCTTTC TGCTTAGTCT GAAAGGTGTG TGGCATTCAT GGCAATCCTG TAACTTCAAC 240
ATAGATTTTT TTTGTGTGTG TGGAAATAAA TCTGCAATTG GAAACAAA 288

SEQ ID NO:3676
SEQUENCE LENGTH:285
SEQUENCE TYPE:nucleic acid
TOPOLOGY:linear
CLONE:HUMGS04331
SEQUENCE DESCRIPTION:
GATCAACCCC TGACCTAATC GGTTATGTTA TCTACAGATT ACAGACATTG TATAGAAATG 60
CACTGTGAAA ATCCCTATCC TGTTTTGTTC TAATCTAACT ACCGGTGCAT GCAGCCCCCA 120
GTCATGTACC CCCTGCTTGC TCAATCAATC ATGACCCTCT CACACACATC CCCTTAGAGT 180
TGTGAACCTT TAAAAGGGAC AGGAATTGCT CACTTGGGGA GCTCAGCTCT TGAGACAGGA 240
GTCTTGCCAA TGCCCCTGGC TGAATAAACC CCTTCCTTCT TTAAA 285

SEQ ID NO:3677
SEQUENCE LENGTH:285
SEQUENCE TYPE:nucleic acid
TOPOLOGY:linear
CLONE:HUMGS04332
SEQUENCE DESCRIPTION:
GATCACAAAA ATGGAACCCC CTGTTTCATG CTTCTACAGT CCAGCCAGCC AATCTGAAGA 60
TGTCATTCTT ATAAAAAAGT ATGACCAAAT GGCTATCTNC CACTGTTTAT NTTGGCCTTC 120
ACTGACTCTG CTAGGTGGTG CCCTGATTGT TGGCATGGTG AGATTAACAC AACACCTGTC 180
CTTACTGTGT GAAAAATATA GCACTGTAGT CAGAGATGAG GTAGGTGGAA AAGTACCTCA 240
TATAGAGCAG CATCAGTTCA AACTGTGCAT TATGAGGAGG AGCAN 285

SEQ ID NO:3678
SEQUENCE LENGTH:282

SEQUENCE TYPE:nucleic acid
TOPOLOGY:linear
CLONE:HUMGS04333
SEQUENCE DESCRIPTION:
GATCTCTGTG TTTGCAGTCA AAGGGCGGCT CACACATGGT CAAGTATTAT AAATNTCCTG 60
TGTATCTNAC AATGGAATCT ATTCTACAAT TTTAAGTATA CGCTTTCTGT ATATGGCCAT 120
TAAGTGAAGC TTAATAATCT TGTNTTTNCT GTCCTCATAG ATATTTTAAT CTGTGGCATC 180
CTCCTGTAAT CTCAGCATTT TGGGAGGCTG AAGATGAGAG GGTTGCTTGA GGCCAGGAGT 240
TCAAGACAAA CCTAGGCAAC ATAGCAACCC TGTCTCTCCA AA 282

SEQ ID NO:3679
SEQUENCE LENGTH:280
SEQUENCE TYPE:nucleic acid
TOPOLOGY:linear
CLONE:HUMGS04334
SEQUENCE DESCRIPTION:
GATCTGAGGT GATATGTTCT GAATGTAAGC ACCGTCAACA TCAGACACCT ACTCATGGAC 60
ATGTGGTTGC CGGATTTTCT TAAGATGTTT CCAGAAATGA CTGATATTTT ATATTTATAC 120
ATTTTAGATG ACAAAGCTTG ATATTTATTG CTGTCGCACA TTTTAAAGTT TTCTTTTTGG 180
GTTGCTCTGT GNCANGGCGT GGTTACATGG TGNTAAATCG GTACCTGATA ATGTACCCAA 240
ATACTATGGC CAGATAATAA ATTGNGGTGC AAACAACAAA 280

SEQ ID NO:3680
SEQUENCE LENGTH:278
SEQUENCE TYPE:nucleic acid
TOPOLOGY:linear
CLONE:HUMGS04335
SEQUENCE DESCRIPTION:
GATCACTGTG CAGTGGGACC ACCCTCACTG GCCTTCTGCA GCAGGGTTCT GGGATGTTTT 60
CAGTGGTCAA AATACTCTGT TTAGAGCAAG GGCTCAGAAC NCNNNAATAC TGTCATGGAG 120
GTGCTGAACA CAGGGNNGGT CTGGTACATA TTGGAAATTA TGAGCAGAAC AAATACTCAA 180
CTAAATGCAC AAAGTATAAA GTGTAGCCAT GTCTAGACAC CATGTTGTAT CAGAATAATT 240
TTTGTGCCAA TAAATGACAT CAGAATTTTA AACATAAA 278

SEQ ID NO:3681
SEQUENCE LENGTH:277
SEQUENCE TYPE:nucleic acid
TOPOLOGY:linear
CLONE:HUMGS04336
SEQUENCE DESCRIPTION:
GATCANAGAN TNACAACTGT TCATTATAGT GGTGCCTTAG GCAATCTTTN CAAAGTAAAT 60
TCAGGGCCCC ATTGCTACTT ATGCCATATT TGGACATACT TTTTTTTTCT TCAATTTTGT 120
AAACTTCCTG GAAAGCTGTC TTCACTAAGT ATCCCCNAGT CTCTATCTAT GNGGNTAGTA 180
GTCATGGAAA TAACACATAA AGTACGCCAG NAGTTNGNTG GAACGTGTTA GAAACTGTTT 240
TGTGCTTTNA TGGATGTCAT ACTTGNCANT ACATGTN 277

SEQ ID NO:3682
SEQUENCE LENGTH:275
SEQUENCE TYPE:nucleic acid
TOPOLOGY:linear
CLONE:HUMGS04337
SEQUENCE DESCRIPTION:
GATCTTTCTT TACTTGATGT GTCCTTCTNT TCGCAGATTG ATAACAGAGC TGTGCTAGAA 60
CTGAATGCAA GCTTTCCAAA ANTNTTCATA AAAAANAGCT TTACTCAGTG ACTTAATATA 120
TGTNCTGTAT TAAAATTAAT GTGCTTTGTT GGGGTTTAAT TTTGGNATTG GTTTTGGGTT 180
TTNTTTTTAG TTGTTTTAAT GGTAAGAATT AAGACATTTT NTAGATTTTA AAGAAAAATA 240
TGAAATTNTC CATTAAATCA AGTAAAAATG TGAAA 275

SEQ ID NO:3683
SEQUENCE LENGTH:269
SEQUENCE TYPE:nucleic acid
TOPOLOGY:linear
CLONE:HUMGS04339
SEQUENCE DESCRIPTION:
GATCTCCGCA GTCTTCAGCG CATTGGCCCG AAGAAGGCCC AGCTAATCGT GGGCTGGCGG 60
GAGCTCCACG GCCCCTTCAG CCAGGTGGAG GACCTGGAAC GCGTGGAGGG CATAACGGGN 120
ANACAGATGG AGTCCTTCCT GAAGGCAAAC ATCCTGGGTC TCGCCGNCGG CCAGCGCTGT 180
GGCGNCTCCT GACCGTCGTC TCCTCACTCC GNCTTTTCAA ATTTTTGTAT AACCCCGTGT 240
TGTGTAAATA CAGTTTTTGC TCCGGTAAA 269

SEQ ID NO:3684
SEQUENCE LENGTH:266
SEQUENCE TYPE:nucleic acid
TOPOLOGY:linear
CLONE:HUMGS04340
SEQUENCE DESCRIPTION:
GATCAGGCCA GTAGGTTGTG AATGCAGGCT GGAGCCCCCG AATGCCCCAN NCACACTGCA 60
GCATTGACCA GACCATCCGA AACCTGCGTC CCTGGTGATG TTCTCAAGCC TCGGAAGTGG 120
CAAATGGAAA TAATATGGCC GGTTGCGGTT GTAGGAGAGT TGTGACTTAG GCAGGAGTCG 180
ACCTCCTCAA GTAATGGAAC GATTTCAAAG GCAGGCTGCC CTGACCAAAA ATATCTGCCA 240
TGAATAAAGG TGCCTGAAAT CCTAAA 266

SEQ ID NO:3685
SEQUENCE LENGTH:266
SEQUENCE TYPE:nucleic acid
TOPOLOGY:linear
CLONE:HUMGS04341
SEQUENCE DESCRIPTION:
GATCTACTTT CAGGATTATT TNTTGTNTTT GAACGTGACA GTNTGGATGA GGCAAGTNAG 60
TNTAGATGCT GCGACCCTGC GAGAAGCAAT AGCTAGTGAA CACATTAGCT CCCTACATGG 120

GACGCAGCTC CCTCTGACAT GTGTCTCTGC CCTTAACCAA GACAGAAAGG GGGTAAATGG 180
AGGATTGGNT TACTGTCTTT GCTGGTTAGT TTTGTGAAAT TTTAAAAATG TTCAACTTTT 240
GANTAAACTC CTTTAAACTT CTTAAA 266

SEQ ID NO:3686
SEQUENCE LENGTH:270
SEQUENCE TYPE:nucleic acid
TOPOLOGY:linear
CLONE:HUMGS04342
SEQUENCE DESCRIPTION:
GATCTCCTTC AGTNGTCTAC CCACTTCCGT CATTGTCCTC CGCTAGAATA ACAGAGTAGT 60
TGGTAGTCAT GGCCAGAAGG GGGGTGGGGG CAGTGTGAGG ACTTGGGGTT TCTGTGCTGT 120
GTGACACAAG GAGATAAGTG ACAATNTTGA AGTCCTAACT TGCACTATTC TAGAAAGTAT 180
AGTGGTGATT TTTGTGCAAA GATTTGAAGT TTTAAAAAAG TACCAAGTTC CTGAAATTCA 240
ATAAAATATT TTTATTAATT TTAATGGAAA 270

SEQ ID NO:3687
SEQUENCE LENGTH:264
SEQUENCE TYPE:nucleic acid
TOPOLOGY:linear
CLONE:HUMGS04343
SEQUENCE DESCRIPTION:
GATCTCAGCT CACTGCAACC TTCACCTCCC GGGAGCATGC TGCTCTATCT TTTTTTNCAG 60
ATGTTTGAGC TATTCCAGGT TTTTTGGGAT TTCTGTTGNT TTGTTTTGTT TTTTGCTTTT 120
CTACATAATT GTTTGGATAA TCTTGTCAGT ATCTACGAAG TGCCTTGATA GTAAATATAT 180
NAACCCTGTG TATCAATNTG GGGTAATTNC ACATNTTTTT CTATGTTGAG TTATCAATAA 240
ATTGATATTG TATATGNCTC TAAA 264

SEQ ID NO:3688
SEQUENCE LENGTH:262
SEQUENCE TYPE:nucleic acid
TOPOLOGY:linear
CLONE:HUMGS04344
SEQUENCE DESCRIPTION:
GATCCTCAGT GCAAAATGTC TTGTGTANTT NTNTGTGAAT CCATGGGTCT GGCTAGAGGG 60
CCCAAAGCTT GTAAATATGG GGATAGTCTG GGTCAGACCC ATCTNTCCCT TACCCATCTT 120
GCTTCCAAGA CCATTTGTAG TGAGCGAGTG GATGCTGTGC TACGTGTGAA ATCTGTCTTT 180
GCGGGGCCTG TCTCAGTGAT TCGNTTTTGG TATTNGTTTG TAGCTTTCCT GGAAGTCAAA 240
TAAATGTTTC CCCCACTCCA AA 262

SEQ ID NO:3689
SEQUENCE LENGTH:256
SEQUENCE TYPE:nucleic acid
TOPOLOGY:linear
CLONE:HUMGS04345

SEQUENCE DESCRIPTION:
GATCTCAAAT GTGAACAGTT TACTAATGCA CTACTGAAGT TTAAATCTGT GGCACAATCA 60
ATGTAAGCAT GGGGTTTGTT TCTGTAAATT GATTTCTAAT NTGAAATTAC TGAACAACTC 120
CTATTCCCAT TTTNGCTAAN CTCAATTTCT GGTTTTGGTA TATATCCATT CCAGCTTAAT 180
GCCTCTAATT TTAATGCCAA CAAAATTGGT TGTAATCANA TNTTAAAATA ATAATAATTN 240
GGCCCCCCCT TTTAAA 256

SEQ ID NO:3690
SEQUENCE LENGTH:251
SEQUENCE TYPE:nucleic acid
TOPOLOGY:linear
CLONE:HUMGS04346
SEQUENCE DESCRIPTION:
GATCTAAAAA GCTGAATAGC ATGTNAGTTA CTTGGTTTCA ACTTGAGTTT NCTTTTAATG 60
TTAATAAGAT TGAAACTTTA GTATTTAGTG GGGAATGGAA AGAGTTGCCC TTGTTGCAAG 120
TAATGAAGCC TGATTTGATT ATGAAGCTGC TTAATCACTC TTCATGTGTT CAGAATTACT 180
GTTTTTNTTG TTTGTTTTNC CTTTTNGTCA CTGTGGTACA TTAAAAATTT NGGGAAGATG 240
CTTTACCTAA A 251

SEQ ID NO:3691
SEQUENCE LENGTH:250
SEQUENCE TYPE:nucleic acid
TOPOLOGY:linear
CLONE:HUMGS04347
SEQUENCE DESCRIPTION:
GATCCCAGAC CTGTNGTCTC TCCCACCCCC TCCCCAAAGC CACTGGAAGG AGCACATACT 60
ACCTAGAAGT AAGANGNNGA GCCTCAGAAG AAAACAAAGT TCTATTTTAT TAATTTCCTA 120
TGTGTTGTGT TTGTAGTCTT GTCTTAGCTC TGGACGTGAA ATACTTCGAT GATGATGATG 180
ATGATGATGA TGATAATAAT AATAATAATA ACAACAACAA CAACAATAAT AAAGATGTGA 240
AAACTCGAAA 250

SEQ ID NO:3692
SEQUENCE LENGTH:248
SEQUENCE TYPE:nucleic acid
TOPOLOGY:linear
CLONE:HUMGS04348
SEQUENCE DESCRIPTION:
GATCTCAGAT AACTCAGTAC AGATAGCAAT TAGTCAGCTG ATTTGATTAC AATGGAGTAA 60
CCGACAATAT ATTTATTTAT AAAGCACATA TTCATAATAA CGAGAAGAAT TCAGAAAACC 120
ACTTAAGCAA GACCCTTCTG AAATAAAANN TGTNGCTTTT TAAATAGTTT GTCCTAAGGT 180
GTTTAAANCA TGTCAACCTT ATGTAAGGNN AANTTTCCTG GTCCAANTAA NGTTGANGTT 240
TAAGGAAA 248

SEQ ID NO:3693
SEQUENCE LENGTH:248

SEQUENCE TYPE:nucleic acid
TOPOLOGY:linear
CLONE:HUMGS04349
SEQUENCE DESCRIPTION:
GATCCTGGTT GGAAGGATGG GGACTCTCTC AAGGGGCTTT GGAAGAGCTC TTCTAGCCCT 60
TTATAAAAGG AGGGCAGCAG CTGAGACTGA TGAGAGGAGG GCAGCCTGCT CTGTTCTTTC 120
AGGGCCCCCC ACCCCCATCT CCCCTACCCT AGCCCACCCT AGGGCCTCTA CCCAGCGGGA 180
GGGGTTGAAG ACCAGGCCTG GTTTTATTAG AATTCATTTT GTAATAAAAG CCTTTTTTAG 240
TGGTGAAA 248

SEQ ID NO:3694
SEQUENCE LENGTH:248
SEQUENCE TYPE:nucleic acid
TOPOLOGY:linear
CLONE:HUMGS04351
SEQUENCE DESCRIPTION:
GATCCTATTT TNATGAAATG TCCAGAACAG GCAAATCCAC AGAGACAGAA ACTAGACGAA 60
TGGTTGCCTG AGGGTGAGGG GAAACAGGGA GTGACTGATG ATGGGTTTTG TGTGGGGGCA 120
ATAGAATGTN CTAAAATTTG ATAGTAATGG TTATACAACT CTGAATATAC TAAAAGCCAT 180
TGTACACACG TTAAAAAGGG TGGATTGTAT GTGAATTCTA TCTCAATAAA GCTGTTCAAA 240
AATTCAAA 248

SEQ ID NO:3695
SEQUENCE LENGTH:245
SEQUENCE TYPE:nucleic acid
TOPOLOGY:linear
CLONE:HUMGS04352
SEQUENCE DESCRIPTION:
GATCCACTCC GCCATCGATG CATCCCAGAC CCCCGATGTC GTGTTCGCAA GCATCCTAGC 60
AGCCTTCTCC AAAGCCACAT GTAAAGACTT GGTTATGTTT ATCTAATGTT GGGTCCAAGA 120
AGGAATTTCT TTCCATCCCT GTNAGGCAAT GGGTGGGAAT NATAGGACAG GCAAAGAGAA 180
GCTTCCTCAG GCTAGCAAAA ATATCATTTN ATGTATTGAT TAAAAAAGCA CTTGCTTGAT 240
GTAAA 245

SEQ ID NO:3696
SEQUENCE LENGTH:243
SEQUENCE TYPE:nucleic acid
TOPOLOGY:linear
CLONE:HUMGS04353
SEQUENCE DESCRIPTION:
GATCTCAAAA CCACTATTTT TATTATTTCA TTATTTTTCA GAGGCCTTAA AATTCTGGAT 60
AAGAGAATGG AGGAAAATAC TCANCGGTAC TTCATTATTT NATTTCCTTT TATTAAAAAA 120
TTACTTCTAT GTTTTNATTG TCTCTTGAGC CTTAGTTAAG AGTAGTGTAG AAATGCATGA 180
NCTTCATCCT AATAAGGATA AANCTTAAGG AAAACCACAA TAAACCATGA AGGTGTACAC 240
AAA 243

SEQ ID NO:3697
SEQUENCE LENGTH:241
SEQUENCE TYPE:nucleic acid
TOPOLOGY:linear
CLONE:HUMGS04354
SEQUENCE DESCRIPTION:
GATCTNTCCA CGGTAGCACT TGNCCTTTTC GACGCTTAAC CTTTCCGCTG TCGCCCCAGG 60
CCCTCCCTGA CTCCCTGTGG GGGTGGCCAT CCCTGGGCCC CTCCACGCCT CCTGGCCAGA 120
CGCTGCCGCT GCCGNTGCAC CACGGCGTTT TTTTACAACA TTCAACTTTA GTATTTTTAC 180
TATTATAATA TAATATGGAA CCTTCCCTCC AAATTNTTCA NGTAAAAGGT GGCTTTTCAA 240
A 241

SEQ ID NO:3698
SEQUENCE LENGTH:239
SEQUENCE TYPE:nucleic acid
TOPOLOGY:linear
CLONE:HUMGS04355
SEQUENCE DESCRIPTION:
GATCAGCACT GCACTNTTAG CTGAGAGTGC GGGCAAGACA TAAACTGTCC AGAGTTTNAA 60
GNTTCTNGGA AAGACCGGAG GGCTTCTCCC CACAGAAGGC GGAGAGAGCT GGGNCTCAGA 120
CATGGNTGTG CACCTTAATA AACCCTGCTG TCTGCCTCCC TGACTCTGCT TCTNNGGAGC 180
ATGGTGAGCA GCCCTNGTGC TCANCAGCCA TACCTATGGG ACACACACTA CGNAAAGGN 239

SEQ ID NO:3699
SEQUENCE LENGTH:236
SEQUENCE TYPE:nucleic acid
TOPOLOGY:linear
CLONE:HUMGS04356
SEQUENCE DESCRIPTION:
GATCACAGGT GATTCACACG TACACACATA AACACACCCA CCAGTGCAGC CTGAAGTAAC 60
TCCCACAGAA ACCATCATCG TCTTTGTACA TCGTATGTAC AATGCAATCA TTTCATACTT 120
TAAACTGGTC AAAAAACTAA TTGTGATTTC TAGTCTTGCA AAGCTGTATG TAGTTAGATG 180
ATGTGACAAC CNCTAATATT TATCTAATAA ATATGTATTC AGATGAAACC TGTAAA 236

SEQ ID NO:3700
SEQUENCE LENGTH:235
SEQUENCE TYPE:nucleic acid
TOPOLOGY:linear
CLONE:HUMGS04357
SEQUENCE DESCRIPTION:
GATCCATCCT TTATCAGGAA GTCTGAAGCG ACTATAAAGG TTTTTNANTT CAGATTTAAA 60
AACCAACTTA TAAAGCATTG CAACAAGGTT ACCTCTATTT TCCCACAAGC GTCTCGGGAT 120
TGTNTTTGAC TTGTGTCTGT CCAAGANCTT TTCCCCCAAA GATGTGTATA GTTATTGGTT 180
AAAATGACTG TTTTCTCTCT CTATGGAAAT AAAAAGGAAG AAAAAAAAGG GNAAA 235

SEQ ID NO:3701
SEQUENCE LENGTH:232
SEQUENCE TYPE:nucleic acid
TOPOLOGY:linear
CLONE:HUMGS04358
SEQUENCE DESCRIPTION:
GATCCGTGTT CTCCATTTAT TAATGCATTG TAGACCAATT TAACTGCTGT GTTTCAGGAA 60
AATTCTTCCT AGTTTAATAA GCAAGCTAAA AGTTTTATTT TTTATATTTA GTGCTTAATC 120
TTTGCCTCAT GTTATGTAAA ATTAGCCTGC AGATATTTTC TCTCAATTCT GTAGACTCTC 180
GCAAGATAAA CATTCAAACA GTGAAACAAA CANTAAAATA AATAAACCTA AA 232

SEQ ID NO:3702
SEQUENCE LENGTH:231
SEQUENCE TYPE:nucleic acid
TOPOLOGY:linear
CLONE:HUMGS04359
SEQUENCE DESCRIPTION:
GATCCATCTG AACTATGAAG TGAGTACCTG GAGGAACTCC TACATTTAAG GCTTTCAAAG 60
GTTACAGGCT TTTCGTGTGT GTGTGTGTGT GTGTGTGTGT GTGCGTTTTG AGAAAAATGG 120
AATTCAAACA CTGTNNTTAG AATGTATGTT TACTTATATA GAATTAATGA ATNTATATAA 180
TAATAATNAC TGTATATAAT TNTAAAATAT AATAAAGTCT CTTTATCCAA A 231

SEQ ID NO:3703
SEQUENCE LENGTH:237
SEQUENCE TYPE:nucleic acid
TOPOLOGY:linear
CLONE:HUMGS04360
SEQUENCE DESCRIPTION:
GATCTGGAGT TGAGAGCCAT GGGTTTGGAC ATGACTGGCA CAAACAGCTG TCATATGTTC 60
ATGGTCAGAT GTCATACATT CTCAGCTGTC TTGTTCCACC AGTATTTACC AGGAAAACAA 120
AGAATGTGTT AAGGGATGCT CCCCCACCCC ACATCTTAAG TCAGTGTGCC AAGTACTGAG 180
ATGATTTTAG GGACATTTTA TTTTAAATTA AATTTACAAT CTAATGGTAA ATTGAAA 237

SEQ ID NO:3704
SEQUENCE LENGTH:231
SEQUENCE TYPE:nucleic acid
TOPOLOGY:linear
CLONE:HUMGS04361
SEQUENCE DESCRIPTION:
GATCATCAAC AAGATTTCCT TGTGCAAAAT ATTTGACTAT NCTGTATCTT TNATCCTTGA 60
CTAAATTCGT GATTTTAAAG CAGCATCTTC TGGTTTAAAC TTGTTTGCTG TGAACAATTG 120
TCGAAAAGAG TCTTCCAATT AATGCNNNNT TTATATCTAG GCTACCTGTT GGTTAGATTC 180
AAGGCCCCGA GCTGTTACCA TTCACAATAA AAGCTTAAAC ACATTGTCAA A 231

SEQ ID NO:3705
SEQUENCE LENGTH:229
SEQUENCE TYPE:nucleic acid
TOPOLOGY:linear
CLONE:HUMGS04362
SEQUENCE DESCRIPTION:
GATCAAAATT GAACCATTGT ACAGTTTGGN TTCTGTTTGC TTCAAAATAT GTAGAATTGT 60
GGTTGATGAT TAATTTGCGA GACTAACTTT GAGAGTGTAA CANTTTTGAN GAAAACATTG 120
AATGTTTTGC AAATGAAGGG GCTTCACGGA ATGTTACANT GNTACTAATA TAATTGGGCT 180
TTNNTNATGC AAATNGTTAA CACCAGCTAT TAAAATATAT TTNNGTAAA 229

SEQ ID NO:3706
SEQUENCE LENGTH:228
SEQUENCE TYPE:nucleic acid
TOPOLOGY:linear
CLONE:HUMGS04363
SEQUENCE DESCRIPTION:
GATCGCTACC CACCTTCCTT CCATGGTTCC CACCCTCCAC GTTATTTTCC CTTTCTGCAG 60
CGGTTGCACT ACAGGTAGCT ACTGTGTATT ATGGACAAAT GAGAAATGAA TTCTTTTTCT 120
GGCTGTCCAT CTATTTTATT TCAAATAAGG AAAAGTGTAT TTGGATTTTG TGTAAATACA 180
TCTAGTGATG NCATTTTTTC AATGTTTTNA AAAACCGTGT ACAGTAAA 228

SEQ ID NO:3707
SEQUENCE LENGTH:228
SEQUENCE TYPE:nucleic acid
TOPOLOGY:linear
CLONE:HUMGS04364
SEQUENCE DESCRIPTION:
GATCCTCATC CAATTGGTGT TTTTCAGAAG TNTTCCAATA TTATGAATTC TGTGTTGTGG 60
AGAAAAGCAA CCATGCATTT ACTGGTCAAT GCCTTCTTGT ATATGTAATT CAATACTTTT 120
ACTTTTAATA TCCTCACCTT ATCTAATCTT TGATTTTTGT CATGTAATTT ATTGCTTCAT 180
TAAGGTTACT TTTTGTTATA CAAAATAAAA GCTGATATCC AAGGCAAA 228

SEQ ID NO:3708
SEQUENCE LENGTH:226
SEQUENCE TYPE:nucleic acid
TOPOLOGY:linear
CLONE:HUMGS04365
SEQUENCE DESCRIPTION:
GATCTATGAA AATNTGTTTT GACCAAACTT AGTATTAGGC AGGTACACAG ATAAGATTTT 60
CCATTCTNAA CATTCTCACT GTAATGCACG ACATTTGANG CACCTGNATG TGCTATCAAT 120
CAGGTGGGAA ATTTAGCATT ATATATTGGA AGCATTGAGA AACATTGGTC TNTCATTTCT 180
ATTTNNTTTT NCCCCTCCTN TCCTCCTCCT GCCCCACANT ATATNN 226

SEQ ID NO:3709

SEQUENCE LENGTH:224
SEQUENCE TYPE:nucleic acid
TOPOLOGY:linear
CLONE:HUMGS04366
SEQUENCE DESCRIPTION:
GATCCTGAGA GCTTTGAGAG GGTGTTGCCA GTTTTAAGCT GAGATGGCCC ATTTGGAGCC 60
TTACAGAACT AGACCAGAAG ACATTTGTGG ATAAATCTGA ACTCACTTCC TGTGTGTAAC 120
TTCTAACTTC ACTGGTTCCT CCCATCTGAA TTGGCCACAT GCTTGCTGAA GAGACTTGTT 180
TTCTTCTTCT AAGACAGTGG TTTTGTTTTT TGGGTTTTTT TAAA 224

SEQ ID NO:3710
SEQUENCE LENGTH:223
SEQUENCE TYPE:nucleic acid
TOPOLOGY:linear
CLONE:HUMGS04368
SEQUENCE DESCRIPTION:
GATCTGGGCA GAATCCGCCA AGCGGCTGGG CCCAGGCTGT GGCATGATGN ACGGAGNAAN 60
GAAGCACCTG GACTTCTAGG GATTCCTCCT TAGTCGCTGC ATGCAGAATT CTATGACACT 120
CTAATNATGA TTGCTAATAG CTTATGTAAA TATTTTNNTT AACAATTTGG NCCTNCACTC 180
CTTNAAAAAC ACAAGGATTA TAAAATGGGT GCCNGCATTT NTN 223

SEQ ID NO:3711
SEQUENCE LENGTH:221
SEQUENCE TYPE:nucleic acid
TOPOLOGY:linear
CLONE:HUMGS04369
SEQUENCE DESCRIPTION:
GATCGTATTC NTNATAAACT ATTACCAGTA CAGTAGCTAG TAGATATNNT GTGACTGCCT 60
CATTGTTTAT CTTGCTGCTT TGGTCCCTAT GGCTCTAGTA TGAACATTCT CGTGTCTTCT 120
GTGGAAATGA TTTCTGTGTT ATAGTTNCAN CTTNGGTAGA GGTGGGGATG ATTGTATTTT 180
GTTTTAGTTG ACATCATTCT CAACTNTTAG AAATACTGTT N 221

SEQ ID NO:3712
SEQUENCE LENGTH:219
SEQUENCE TYPE:nucleic acid
TOPOLOGY:linear
CLONE:HUMGS04370
SEQUENCE DESCRIPTION:
GATCAGTTTG CGAGTTTTGA TGAGNTTTGT AAGGTTTNTN TTTTACAAAC TATGAATCAG 60
CAAATNTTTA AGATTGTACC ACATAGCAAA CATACAGCTG TTGAAAAATA ATGTATATAA 120
AATGCATATA ATAAATATTA AATNGTGTAC CTGTATGTTA CTGTTGGCTA CATCATTTTG 180
TGTTGAATAA TAAAGTGCAA TACNNNCCTT CTCCATAAA 219

SEQ ID NO:3713
SEQUENCE LENGTH:214

SEQUENCE TYPE:nucleic acid
TOPOLOGY:linear
CLONE:HUMGS04371
SEQUENCE DESCRIPTION:
GATCATTCTG TATATACATA TTTTAAATTA TGCAAGCAAA CTAGGAGGAG AATGTNCAAT 60
TTTAAGGGAT ACCTAATTTC CNTTCGGTTA GGGGATATTT TCCANCCTCT TGCTTTATAC 120
TCTGATATGG GGTGTNTTTA TGANCTTTTT AATGTTATTT ANTCATGCAG ATTTCCAATC 180
ANTGTATTGC NCATTTCCTG ACAAAANGGG NAAN 214

SEQ ID NO:3714
SEQUENCE LENGTH:215
SEQUENCE TYPE:nucleic acid
TOPOLOGY:linear
CLONE:HUMGS04372
SEQUENCE DESCRIPTION:
GATCCAAAAN ATAAAACTTG AAGCTATTCT GGAACTAACA TGGAAAAATG AAATGGCTAT 60
TGTTTAAAAA AATGATAGAA ATACATTGTT GATGGGATAT GAGTTAAGTT TATTTTCTAC 120
AAACCTGTAA TTGATGACGG CATGGATAAT ATCTTCATGT TTCTGACNAG TAATCTGTAT 180
GTGGGGGGAG GGGATAATAA ATATTTCTAA CCAAA 215

SEQ ID NO:3715
SEQUENCE LENGTH:335
SEQUENCE TYPE:nucleic acid
TOPOLOGY:linear
CLONE:HUMGS04373
SEQUENCE DESCRIPTION:
GATCAAAAGT GAACACCCTG GCCTATCCAT TGGGGATACT GCAAAGAAAT TGGGTGAAAT 60
GTGGTCTGAG CAGTCAGCCA AAGATAAACA ACCATATGAA CAGAAAGCAG CTAAGCTAAA 120
GGAGAAATAT GAAAAGGATA TTGCTGCATA TCGTGCCAAG GGCAAAANTG AAGCAGGAAA 180
GAAGGGCCCT GGCAGGCCAA CAGGCTCAAN GNAGAAGNAC GANCCAGNNG ATGAGGAGGA 240
GGAGGAGGAA GANGNAGATG AAGATGAGGA GGAAGAGGNT GAAGATGAAG AATAATTGGC 300
TATCCTTTAA ATGATGCGTG TGGAATGTGT GTACN 335

SEQ ID NO:3716
SEQUENCE LENGTH:211
SEQUENCE TYPE:nucleic acid
TOPOLOGY:linear
CLONE:HUMGS04374
SEQUENCE DESCRIPTION:
GATCAAATGT GCTGCAGCTT TTGCAGAAAA CAACTCAGAA ACACAAAACC CCCCAACAGC 60
TCAATTATTA TTTTTTCAAT GTTTTCCTAC AAGAGCCAAG TAGCACCATG TACAGAAGAC 120
GCCTTTTTTT TTGGAATATT GAAATCGTTC TGCATGTAAA ATATGGGATA ATGACCTGTT 180
TATATTAAAA TTCTGATTAA ATTATCTGAA A 211

SEQ ID NO:3717

SEQUENCE LENGTH:390
SEQUENCE TYPE:nucleic acid
TOPOLOGY:linear
CLONE:HUMGS04375
SEQUENCE DESCRIPTION:

```
GATCAAGAAT TCTACCATCC CTTGGGTCTT TGTGTATAAA CAATGTTAAA TACCAGGTAG  60
ACTCAGTCTT TAAGATATTA GACAGTTTTT TTAGTCCATG GGNTNNNNAA TATAAACATT 120
AACTTTCCTA TAAGANTATT TTGGCTTTGT AATCTATAGC CTCAAATTGG TATTTATTAT 180
GGNTTCACTA GACAAACAGC TGTTTCCTTA TTGTNTTTTN NCTTTAGTGT TTCTGATTTG 240
CTATCAGTAG CTGTTTTTAA AGCCGNCCAA GGNAAATANT TATTTGCAGT TTTTTGAAGT 300
CACTTTTTGA GCCCTCATNC AAGCTCTCAT TGTGGNTGGG GAGGGATACC NTTTTTGGTT 360
GTTAAAAGGC CTATNATTGG TTAAAGGGCN                                 390
```

SEQ ID NO:3718
SEQUENCE LENGTH:235
SEQUENCE TYPE:nucleic acid
TOPOLOGY:linear
CLONE:HUMGS04377
SEQUENCE DESCRIPTION:

```
GATCTTAGAG CTGAGGAATT GGCCTCCCAA TCCGAACAGG TGTTATAATC CTTTCTNAAT  60
AGGTTGTGCT GTGGACCCAA TGTNAGGGCT GTNCTGGTGT AAATGGTGAC ATATTGAGCT 120
GGGGGGATGC TTTCGGGGTG GGGGGACTGG TTCCATTCCA TCAAAAGGCC CTCTTGAGAG 180
TCTATCCNGG GNCCCATTGG TTNAATTTAA CAGGCCAGNA AAGANGGTTG NTNNN      235
```

SEQ ID NO:3719
SEQUENCE LENGTH:209
SEQUENCE TYPE:nucleic acid
TOPOLOGY:linear
CLONE:HUMGS04378
SEQUENCE DESCRIPTION:

```
GATCTCCTGA GCTCAGGCAA TCCGCCCACC TCGGCCTGTC AAAGTGCTAN GATTACAGGT  60
GTGAGCCACC ATGGGTGGCA TCTTTAATAT AGATTTATAA GGCCCCNNTG TTATATATTT 120
NNAAAAATTC AAATTAAAAC TANATCCCCA AATGTCCCNT TTATTAATGA AGTATTTTGT 180
CANATGGATG TTTTAAAGCT GTGTATGTN                                  209
```

SEQ ID NO:3720
SEQUENCE LENGTH:209
SEQUENCE TYPE:nucleic acid
TOPOLOGY:linear
CLONE:HUMGS04379
SEQUENCE DESCRIPTION:

```
GATCTAGGAT TACTCTGGAC CTCATTCGGG TGTTATGAGT ATCCCACGAA GGCCAGACGC  60
TGATTTNATT TNCTCATTTC CACAGATTGA CAAGGATAAG TCAGNNGTTT GTAAACTCTA 120
GGTAGCAGAT GAGAAATAAT TCACTTAATA TCAGAAATAT TTNCCAAACA CTTTCCTTNA 180
TTTTTCCTTC TGANTAAATA GANANCCNN                                  209
```

SEQ ID NO:3721
SEQUENCE LENGTH:208
SEQUENCE TYPE:nucleic acid
TOPOLOGY:linear
CLONE:HUMGS04380
SEQUENCE DESCRIPTION:
GATCCAAGGA ACGTTTGGGG TTTCCTAGGT GGGCCACATC CAATCAGTGG AAGGTCTTAA 60
GAGCAAAANC TGAGGTTCCT CAGAGAAGAA GGAAATGTTT TAAGACTTCA GCATCAATTC 120
CTGCCTGAGT TTCTAGCCTG CTGGCCTGCT CTACAAATTT CAAGCTTGCT AGCCCTCACA 180
NTCACATGAA TAAATTCCTT AAAATAAA 208

SEQ ID NO:3722
SEQUENCE LENGTH:208
SEQUENCE TYPE:nucleic acid
TOPOLOGY:linear
CLONE:HUMGS04381
SEQUENCE DESCRIPTION:
GATCCTTTCC CCATGGACAG TGACAGAGCA ACTGGGCCGC AATGGCATAG GCAGAAGAAA 60
ATNTAATCAC TCTGGGGGTG ANTGGTGGTT AAAACGACTC ATGTAATTAT TTGGATTGTA 120
ATTTCNTTAC ACATATTTNT TTGCTTAACC CAGCATGTNA GATATATNAT NTAATCTAAT 180
AAAAGATACA AACTTATGGG CACACAAA 208

SEQ ID NO:3723
SEQUENCE LENGTH:207
SEQUENCE TYPE:nucleic acid
TOPOLOGY:linear
CLONE:HUMGS04382
SEQUENCE DESCRIPTION:
GATCACCCTG GAGCTGTGGC TTCCCCGAGC AGCTGAGAGT GAAAGGAGGC AGTTCCTTTA 60
CTGTGAGCTC TCACGGCACG GTGCTCACGC CCCACACAGG GGCTGCCTTC ACTCGGCTTC 120
AGCAGCNGTT GACTTTTAAA TCATAAAAAG CCGCTCTATA TACAGTTTAC TCTGTTGCTA 180
GAAGGCAGTG GAACATAGGT ATTTAAA 207

SEQ ID NO:3724
SEQUENCE LENGTH:207
SEQUENCE TYPE:nucleic acid
TOPOLOGY:linear
CLONE:HUMGS04383
SEQUENCE DESCRIPTION:
GATCCACGTA CAGTTTGGAT TTTCCTTCTA GCCTTCATGT GTGGCCCAAG CATCTATTTC 60
AGACTGTCAT GGTTGGCATT GTTCTCTGAG GTTCTGTCTT CCCTTCGTGT TTCTATGAGC 120
TTTAATTTGT ATGGTTCTTG TTCTGTTAAT GCCACTAGGG ACTATGTATT CCTGATTCCA 180
CCATGACAGC TACAGTAAAA CAGTAAA 207

SEQ ID NO:3725
SEQUENCE LENGTH:204
SEQUENCE TYPE:nucleic acid
TOPOLOGY:linear
CLONE:HUMGS04384
SEQUENCE DESCRIPTION:
GATCAAGCAG GCACTTCAAG CCTCAATAGG ACCAAGGTGC TGGGGTGTTC CCCTCCCAAC 60
CTAGTGTTCA AGCATGGCTT CCTGGCGGCC AGGNCTTGCC TCCNTGGACT ANCTGGGGGG 120
TTCCGGGTCT CAAGAAGACA AGGGTGCTGG NCCTCCCTAA GCCAAGGAGA GGANTAAAGA 180
CAAAAAGCTN AAAAAAAAAG GAAA 204

SEQ ID NO:3726
SEQUENCE LENGTH:202
SEQUENCE TYPE:nucleic acid
TOPOLOGY:linear
CLONE:HUMGS04385
SEQUENCE DESCRIPTION:
GATCTCGAAC TGTACCAAAA TACATGGAGA CTAACAAACA GAACCACATG GAACTTTCAA 60
ACTGAAAAAA AAATTTGTCA CAAAAACTTT GTTGTCATAG TTAAGTTGAT TGTAGATGGT 120
AATTGAATAT ACTCCTTTGA AAATATTTCA TCAAGTATGT TTCCTGCTCA TTGTGATACA 180
TTAAAAAAAA ANTATGGGCA AA 202

SEQ ID NO:3727
SEQUENCE LENGTH:219
SEQUENCE TYPE:nucleic acid
TOPOLOGY:linear
CLONE:HUMGS04386
SEQUENCE DESCRIPTION:
GATCCAGAAG GCTTACGAGT ATTTTATTAT CTTGTCCAGG ACCTGAAGTG TTTGGTCTTC 60
AGTCTTATTG GATTACACTT CAAGATTAAA CCAATCTAGA CTGAATATTG GTGTGGACAT 120
GGGGGGTGGG TGGGAGTAGA AAATTTTGTG TATATCAGGG CAGTATTTTT TTATGAACTA 180
TAAATGATTG TCTTTAATAA ATATGTGATA AAATCCAAA 219

SEQ ID NO:3728
SEQUENCE LENGTH:200
SEQUENCE TYPE:nucleic acid
TOPOLOGY:linear
CLONE:HUMGS04387
SEQUENCE DESCRIPTION:
GATCATATTT AGAGGATATA ACAATTAGAA ATCTAGAAAA TAATTATCAC TTTNATAAAA 60
TNTTNAGTCA ACTGTACAAA TAATTACATA AAACATCAAT TAATNATGCT TAAAAATCAC 120
TAATNTTCAT AATATATAAT CACTATTTGT AATCAAAAGT TTAATTTTAT NTNAAAAAAT 180
AAAAAATGCT TACTTGGAAA 200

SEQ ID NO:3729

SEQUENCE LENGTH:198
SEQUENCE TYPE:nucleic acid
TOPOLOGY:linear
CLONE:HUMGS04388
SEQUENCE DESCRIPTION:
GATCAGCCGA ACTCTAGGGA CTTGGTGTTG CTTGGAAGGC ATCCATACCT GCATTTTGCA 60
TTCTTCGTAT GTAATCATAT TGCCAAAGAC AAACTATTTC ATCATTTATT GTAAATAACA 120
CTTTTCCCCA GACCTACCAT AAAGTTTCTG TGATGTATTG TCTTCCAGTT GCAATAAAAA 180
TTACTGAGTT GCATCAAA 198

SEQ ID NO:3730
SEQUENCE LENGTH:197
SEQUENCE TYPE:nucleic acid
TOPOLOGY:linear
CLONE:HUMGS04389
SEQUENCE DESCRIPTION:
GATCTAAGAT TAATTCCTCA ACTGTTTTGC ACTCAACAAA GACATACCTC TGAGTTGGCA 60
ACCAGCAGGG TGGATAACGG GCCAGTGGTG ATAAAATCAA AGAATAGGTA ATGAAACAAT 120
CATCCAGTTA ACAATCAGCA AGGTTCTTCA GAGCCTAATT AATGTTTAAT TCTAAATAAA 180
TTGCAACAAT TAAGAAA 197

SEQ ID NO:3731
SEQUENCE LENGTH:197
SEQUENCE TYPE:nucleic acid
TOPOLOGY:linear
CLONE:HUMGS04390
SEQUENCE DESCRIPTION:
GATCCAAAAG TTTAAATTAG TGCATACATC ATGTCATTTC ACCTCCTGTT CCTAGGAACT 60
CTCCATTCCC AAGCATTGCC AGTGTTTTCC AGATAATCTT AGCTGTTGTC TTGTGCTGTG 120
GAAATGGAAG AAACCATCTT CACAGACTGT AGGAGAATTC AACATATAAT TTCTTAATAA 180
ATACTGTTTC TTTTAAA 197

SEQ ID NO:3732
SEQUENCE LENGTH:195
SEQUENCE TYPE:nucleic acid
TOPOLOGY:linear
CLONE:HUMGS04391
SEQUENCE DESCRIPTION:
GATCTAAGGG AGTCCACTAT CTGTGCAATT GTATTTGGNT TTTTTTTGCA CTGTTTCAAT 60
GCTGGTAATT GAAACCATTT TAATATATTT GGTTGTATTC ACTTTATATG TCCTTCCAAA 120
AATNTTGTTG TGTACAAACC ATGNNNNNGA ATGTTGGCTT CCAAGTTTTT AAATAAGAAA 180
CTTTTTGTAT TTAAA 195

SEQ ID NO:3733
SEQUENCE LENGTH:195

SEQUENCE TYPE:nucleic acid
TOPOLOGY:linear
CLONE:HUMGS04392
SEQUENCE DESCRIPTION:
GATCCACGGA GCAGAGGTCA TCTGTCCCCA GCTTGGCCCA CTGAGGCCAG CATGGNTGGG 60
CCCAGGATGC TTGTCTCTCA GCTCCCATCC TGTGTACTTC CACATTGGTT TAACCAGAGG 120
AAAACCGAAA TCTACAATTG TNATAAACAC ATTTAAATGT GCGTAGAATC AGCCATACAA 180
ATTGTGAAAC ATAAA 195

SEQ ID NO:3734
SEQUENCE LENGTH:195
SEQUENCE TYPE:nucleic acid
TOPOLOGY:linear
CLONE:HUMGS04393
SEQUENCE DESCRIPTION:
GATCAACTCC CCCAAGGCCC GAAAGGTGCT GCATACCACG TACCANAGCC AGGAGCGTGG 60
CACACACAGC CTGGACATCA AGTGGTGAAG TNAGGCCAGG GNCTTCCAGC TGCTCTTGGG 120
GCCAGAGCCA AGAGCCTCTN AGTAGAGGGA GGGGCTGCCC TGAGTGGAGT ATTAAAGACA 180
CTTAAGAAAA CCAAA 195

SEQ ID NO:3735
SEQUENCE LENGTH:193
SEQUENCE TYPE:nucleic acid
TOPOLOGY:linear
CLONE:HUMGS04394
SEQUENCE DESCRIPTION:
GATCCTGTGC CCTAGGAGCA CCTAGGAAGA CTTGCTCCAT CTCCGCCCGC ATATCTGGGG 60
CACCAAGAGA GACAGAGTCG TGAGCCAGGC TCGCCTCATT CCTCGTCTTG GGAGAACCGG 120
ACCAACTTCC CCAAAAGGCC TTGCCGTGCA GAATTGGCCT GGTTTAATAA AGAACAGACC 180
TGNCCCAAGN AAA 193

SEQ ID NO:3736
SEQUENCE LENGTH:190
SEQUENCE TYPE:nucleic acid
TOPOLOGY:linear
CLONE:HUMGS04395
SEQUENCE DESCRIPTION:
GATCTGGCCC TCCCCAGAAC AATCTGGATT TCACGGAGAC AGCAACCAGA AGTTAAACCA 60
TGTGACTAAA AATGCATCTG GCTACTTTTT CATGTATGTA TGAGACAGAA ACTAATCCTT 120
ACTATCCTAT TAGGATACCA CTTTTCATTG CAAAGTTTGT GTCAATAAAG TCATTAATTT 180
TAAACATAAA 190

SEQ ID NO:3737
SEQUENCE LENGTH:190
SEQUENCE TYPE:nucleic acid

TOPOLOGY:linear
CLONE:HUMGS04396
SEQUENCE DESCRIPTION:
GATCATAAGT AGTGGAGGAT TTAAAACCAG GCTGGCTGGC TCTTAAGGCC TATGCTCTTA 60
ATTNACCATG TNGAATTAAT TTACCATATT GAATTACACT TATGTNCACC AAGTNAGGCA 120
TTTNGGCAGC CATTGTTTAC GTTTTANTTT TGGAATAATT TAGAAAAGTT ACCAGAGTAA 180
TATGAAAGCN 190

SEQ ID NO:3738
SEQUENCE LENGTH:187
SEQUENCE TYPE:nucleic acid
TOPOLOGY:linear
CLONE:HUMGS04397
SEQUENCE DESCRIPTION:
GATCCTCCGC ATCCCGTGCG AGGAAGAAGA TGTGGAGATG AGTGAGGACG CCTACACGGT 60
GCTGACCCGC ATCGGGCTGG AGACGTCACT GCGCTACGCA NNNNNGCTCA TCACAGCTGC 120
CAGCTTGGTG TGCCGGAAAC GCAAGGGTAC AGAAGTGCAG GTGGATGACA TCAAGCGGGT 180
CTNNNNN 187

SEQ ID NO:3739
SEQUENCE LENGTH:187
SEQUENCE TYPE:nucleic acid
TOPOLOGY:linear
CLONE:HUMGS04398
SEQUENCE DESCRIPTION:
GATCTGGCCC TAAACTTGAA ATACCTCCTG CTCCTTTGGG GACTGGNCTT TGCNAAGAGC 60
ACCTGGGGAG GAGACTGGTG CTAACTAAGC CAGTAAGTAA GTCCACTCTT TGGNTGCAGC 120
TGGTGCAAGG TCTACCCATA CCTTTTTATT TNCAGTGTTT AATAAAANTC CAGTGNGCAA 180
AGACAAA 187

SEQ ID NO:3740
SEQUENCE LENGTH:186
SEQUENCE TYPE:nucleic acid
TOPOLOGY:linear
CLONE:HUMGS04399
SEQUENCE DESCRIPTION:
GATCAAGTTC TGCGNCTGAN GAGGCTGCCA AAAGTCAAAA GNGGGGCCTG GGAAGGCAAG 60
CTCATTCCTC ACAACGGCCT TTCCAGGCCC NGTTTTTCCC TTCCGGCAGC CTCTTGGCCT 120
CTAATTTGTT TATCTNTNGT GTATAAATCC CAAAATATGG AATTTTGGAA TATTTCCACC 180
ATTAAA 186

SEQ ID NO:3741
SEQUENCE LENGTH:185
SEQUENCE TYPE:nucleic acid
TOPOLOGY:linear

CLONE:HUMGS04400
SEQUENCE DESCRIPTION:
GATCTGCCTT CCTGCTGAAA CTTGTTCCAC CTCAGTCCCC TCATCTGTCA CACGCATGTG 60
GGGTGGAGTA GGGAGATGCG GGGAGCAGGG TGGGCAGGAA TACTGTTATC TATGTNACGG 120
GGCAGTCGTG AGGCTGAGAT GAGAATGCGG ATTAAAATGC CTGGCGTGCT CACCGTAACA 180
CCAAA 185

SEQ ID NO:3742
SEQUENCE LENGTH:182
SEQUENCE TYPE:nucleic acid
TOPOLOGY:linear
CLONE:HUMGS04401
SEQUENCE DESCRIPTION:
GATCCCATCT NTACAANAAA CTTAAAAATT AGCCAGGCAT GATGGCACAT TCCTGTAGTC 60
CTAGCTACTC AGGAGGCTAA GGTAGGAGGA TTGCCTGAGC CCAGGAGTTC AAGGCTGCAG 120
TGAGCTAAGA ACGTGCCAGT ACACTCCANC NNGAGCCACA AAGTGAGACC CTGTCTCGCA 180
AA 182

SEQ ID NO:3743
SEQUENCE LENGTH:180
SEQUENCE TYPE:nucleic acid
TOPOLOGY:linear
CLONE:HUMGS04402
SEQUENCE DESCRIPTION:
GATCAAAGTG GTAAAGACAA TGTAAAATTT AACATTTTAA TACTGATGTT GTACACTGTT 60
TNTACTTAAC ATTTTGGGAA GTAACTGCCT CTGACTTCAA CTCAAGANAA CACTTTTTTG 120
TTGCTAATGT AATCGGTTTT NGTAATGGCG TCAGAAATAA AAGGATGCTT ATNATTCAAA 180

SEQ ID NO:3744
SEQUENCE LENGTH:179
SEQUENCE TYPE:nucleic acid
TOPOLOGY:linear
CLONE:HUMGS04403
SEQUENCE DESCRIPTION:
GATCTNCGGA ATGATGAGCA CACAAGGCGG GAGCTAGCCA AGATGAAGCA GGNGCCGGTG 60
AACCTGAAGA AGGCAGAGAT ATGGCAAACA GGATTNGCGC TTTTGGNNAC ATGGAGTGTT 120
CAGCAAAGAC CAAAGATGGA GTGAGAGAGG TTTTTNAAAT GGCTACGNGA NCTGCTCTN 179

SEQ ID NO:3745
SEQUENCE LENGTH:178
SEQUENCE TYPE:nucleic acid
TOPOLOGY:linear
CLONE:HUMGS04404
SEQUENCE DESCRIPTION:
GATCTGAGTG CCTCAAGATG GTTTTCAAAA AAATTTTTTT AAAGAAAATA ATTGTATACG 60

TGTCAACACA GCTGGCTGGA TGATTGGGAC TTTAAAACGA CCCTCTTTCA GGTGGATTCA 120
GAGACCTGTC CTGTATATAA CAGCACTGTA GCAATAAACG TGACATTTTA TAACGAAA 178

SEQ ID NO:3746
SEQUENCE LENGTH:332
SEQUENCE TYPE:nucleic acid
TOPOLOGY:linear
CLONE:HUMGS04405
SEQUENCE DESCRIPTION:
GATCCGAGTT CCTCACCGCG GGCCGGGATG TGACCACCCT NTTCAGAGGT TGGACCCCAG 60
GCTTCAAGCA CCGAGTGAGG GGTCTGTTGG GCCCCCTGGG AGAGTCTCTG GTGTGAAGTG 120
GCTTAGGTCT GGACTGGTCA GCTGTGGCAC CAGCCGGTCC TGCCCACGCT TCAGGATGGC 180
CGGGGGCTGC CCCTGTGGGA GAGTTGCCAA GACTCGGCGA TACCAAGGAT GGAAGCCAGA 240
GGCTGTGGCG AGGGAGAGCC AAGCATATTC CCAGCACCAG GGCCACTGCT GGCACGTCGT 300
AGACATCCAA TAAATATTTT TGGAAGGAGA AA 332

SEQ ID NO:3747
SEQUENCE LENGTH:177
SEQUENCE TYPE:nucleic acid
TOPOLOGY:linear
CLONE:HUMGS04406
SEQUENCE DESCRIPTION:
GATCTNNATA CTGTGAATAG CAGAATGTAC AGATGAAGAA TATTAAAATN AGGGGCGTCC 60
ACTCCAAAAC ATATCACCAG AGTGACAACT TCAGCCCTCA GCCTCTGCAA TCCATATATA 120
TNCTGGTCTT GGGAGTCCAT AGAATACTGT TTCCTTCTAA TAAAGGTTTC AAACAAA 177

SEQ ID NO:3748
SEQUENCE LENGTH:186
SEQUENCE TYPE:nucleic acid
TOPOLOGY:linear
CLONE:HUMGS04407
SEQUENCE DESCRIPTION:
GATCAATAAT AGTACCTTTN ATTATACATT TATTATTGTN TCTCTCTCTG ATGTACTGTG 60
GATTGTACAT TTAACTTTGG AATGGCTTTG TAATAATCAG TCTTAAGAAA ATNTTGACAA 120
GCTCTGGTTG CTTATTTTTA GAAAATAAGG ACATTTAATA ATAATAAAAA AAAAGGGATT 180
AATAAA 186

SEQ ID NO:3749
SEQUENCE LENGTH:175
SEQUENCE TYPE:nucleic acid
TOPOLOGY:linear
CLONE:HUMGS04408
SEQUENCE DESCRIPTION:
GATCCCCAGT GGATGCCTGA AACCGCAGAT AGTACTGAAC CTTATATATA CTGTTTGTTT 60
TCCTATGCAT ACATATCCCT ATGATAAAGT TTATGAATTT GGCACTTAAC AGCAGAACTA 120

ATAAGATGAA AGAGTTGTAA CAATATACTC TAATAAAAGT NATTTAAAAT GTAAA 175

SEQ ID NO:3750
SEQUENCE LENGTH:172
SEQUENCE TYPE:nucleic acid
TOPOLOGY:linear
CLONE:HUMGS04409
SEQUENCE DESCRIPTION:
GATCAACAAT GAGCAGAAAC ATCATCAGTC CTTCCCAAGG ACCATGGCGT TTAATGTCTT 60
GGGCACCCCT TGGAAATCAC AGAAAGTCAG CNGTACTGGC CGTGTGGAAC TTNCATCCCA 120
AGACCTACTT TNCAACTGAG TAAGAAGGTC ATTGTGCCCN CTGCATTNNN NN 172

SEQ ID NO:3751
SEQUENCE LENGTH:107
SEQUENCE TYPE:nucleic acid
TOPOLOGY:linear
CLONE:HUMGS04410
SEQUENCE DESCRIPTION:
GATCAAAAAA TGTTTATNCT GAATTCTNTA ATTTAAAAAA NTCATACCTA TNAGGTGTGC 60
TACAGGAATT CAGATACAAT AAGTTGCATA TAAAACCCGA CCTCANN 107

SEQ ID NO:3752
SEQUENCE LENGTH:169
SEQUENCE TYPE:nucleic acid
TOPOLOGY:linear
CLONE:HUMGS04411
SEQUENCE DESCRIPTION:
GATCTGACCA ACAAGGCCAC AGGAAAATTA ATAGCACAAG GAAGACACAC AAAACACCTG 60
GGAAACTGAG AGAACAGCAG AATGACCTAA AGAAACCCAA CAATGAATAT CAAGTATAGA 120
TTTGACTCAA ACAATTGTAA TTTTTGAAAT AAACTAGCAA AACCAGAAA 169

SEQ ID NO:3753
SEQUENCE LENGTH:167
SEQUENCE TYPE:nucleic acid
TOPOLOGY:linear
CLONE:HUMGS04412
SEQUENCE DESCRIPTION:
GATCTNCTTG GCCAGCAGGA CGTCCAAGGA CCAGAGCCTG TGTCATGTCC TNGAACAGGT 60
NTGCGGCGTC CTCAAGCAGG GGAGCTGAGC CTTCCANAGG CAGGGTGGGC TCCAGTTGTC 120
TTGAGGGTCC GGATGGGCTC AGGTAATAAA GAAACGGAAG CAGCAAA 167

SEQ ID NO:3754
SEQUENCE LENGTH:168
SEQUENCE TYPE:nucleic acid
TOPOLOGY:linear

CLONE:HUMGS04414
SEQUENCE DESCRIPTION:
GATCTGGGCT GTGGGGCCCT TGGGCCACGC TCTTGAGGAA GCCCAGGCTC GGAGGACCCT 60
GGAAAACAGA CGGGTCTGAG ACTGAAATTG TTTTACCAAC TCCCANGGTG GACTTCAATG 120
TGTGTATTTG TGTAAATGAG TAAAACATTT TATTTCTTTT TAGGTAAA 168

SEQ ID NO:3755
SEQUENCE LENGTH:162
SEQUENCE TYPE:nucleic acid
TOPOLOGY:linear
CLONE:HUMGS04415
SEQUENCE DESCRIPTION:
GATCTATGAT TTATNATGTC TTAATAGACC CTAAATTGTT CTTTAATTAC AAGAGTGGCA 60
GTCTCTGAAG TCATTTGTGA GCTTGTATGA CTTTTGTATT TAGCAATGTT GCATGCTCAC 120
ATAATTGAAA ATTAAAAGTA ACACATTTTT CTGAAATGTA AA 162

SEQ ID NO:3756
SEQUENCE LENGTH:161
SEQUENCE TYPE:nucleic acid
TOPOLOGY:linear
CLONE:HUMGS04416
SEQUENCE DESCRIPTION:
GATCAAAACA TAATTGTTTT AATTCTACAG CTGTAGGAGC TTTGTATTGC TGAACTTTCA 60
TCTGGAAAAG TTTCACAGTG ACATTTTTAA AAGAGAATTT TTTTATCTGC CGAATTCTAC 120
CAGTGTAACC TTTTTNNTAA ATAAACAATA GTTTTNTCAA A 161

SEQ ID NO:3757
SEQUENCE LENGTH:160
SEQUENCE TYPE:nucleic acid
TOPOLOGY:linear
CLONE:HUMGS04417
SEQUENCE DESCRIPTION:
GATCTCAAGA AGCCCCACAT CTTCCTAAGG GGCCCCATGG CCTNNTTTGG GNGCAGGGTA 60
GGTCCTGGGG CACTGTGGGC CGCCTGCCTG CTGATGTGGG CTCTAGGCCA GCTTGTTGTC 120
ACGTACGTGG TGTGAAATAA AGCCCAAGCA CTNGNTGAAA 160

SEQ ID NO:3758
SEQUENCE LENGTH:160
SEQUENCE TYPE:nucleic acid
TOPOLOGY:linear
CLONE:HUMGS04418
SEQUENCE DESCRIPTION:
GATCCTGGGT GACTTTGGGT GCACAGGGTG ACCGAGCATT TCTGCCCCTG TGAATGTGGC 60
ACTAACACTG TGCACTGTCT CCACCAAANA AGGTTTCCAC TGAGTTTCTT CTCATGTTAC 120
TGGGTTTGTA AATGAATAAA CACATTTTAA CTACTCTAAA 160

SEQ ID NO:3759
SEQUENCE LENGTH:158
SEQUENCE TYPE:nucleic acid
TOPOLOGY:linear
CLONE:HUMGS04419
SEQUENCE DESCRIPTION:
GATCAATAAG AAGANCCTAG TCTAGAGACA ATGATGCTAG TTTGCATATG TTTTCCTATG 60
CAATAGTTGT TTTCCCAGTN ATNCAAAGCA GCTTTCTATA TGTAGAGATG CAAATNATTA 120
AGTTGTTNCC AATACAATAA ATAAAAGCAT CNGTTNTN 158

SEQ ID NO:3760
SEQUENCE LENGTH:158
SEQUENCE TYPE:nucleic acid
TOPOLOGY:linear
CLONE:HUMGS04420
SEQUENCE DESCRIPTION:
GATCAGATTT GTAATTTNCC TGTATTAAAT GCCTAGAACT CTACAGTCAA CAGATGGCGC 60
TCTAGTTCCA ATTGTNACAG AAGTNCTCTG CCCATCCGCA AGCAAAGCGT TGTTTTCATC 120
AGTGAAAAGA GAATAANCAC TGTGCTTTAA CTTTTAAA 158

SEQ ID NO:3761
SEQUENCE LENGTH:290
SEQUENCE TYPE:nucleic acid
TOPOLOGY:linear
CLONE:HUMGS04421
SEQUENCE DESCRIPTION:
GATCTGTATG GTACTATAAA ATACTTATTT NATAATTCTG TAACCGTATG GCAGTGTTAT 60
GCCAAAAATG TATAAAGAGC AATAGTTTTT GTTGCTTACT GCTGTATTTT AAAATATTGT 120
TTCTAAAATA ATAGNGTTAG AGTTCCTTTT GAGTAATTAT TTTTAAGGAC TATTGCCAAA 180
TATACATCCT GTAAAACCTA ATAAAAGCCA CTCCATCTTT NGGTAACCAT TTTAGCATTT 240
GTGCTTGGNG TACATNNTGG GTGGTTTTTG CNCGNTTAAN NANNTTANNN 290

SEQ ID NO:3762
SEQUENCE LENGTH:158
SEQUENCE TYPE:nucleic acid
TOPOLOGY:linear
CLONE:HUMGS04422
SEQUENCE DESCRIPTION:
GATCCCACCC TCTNTCCCAC CTGGGCNACT GACGCTCTGG CCTCTNTGGN AATNACACTG 60
GGCTGACCTG GCATTGGGAG AGGGAGGGAG GAAGGAAGGG AGGGAGGGGC TGGAAGATAC 120
TGAAGGATTC CTTTTTNAAA GGTTTTTTTT ATTGTAAA 158

SEQ ID NO:3763
SEQUENCE LENGTH:157

SEQUENCE TYPE:nucleic acid
TOPOLOGY:linear
CLONE:HUMGS04423
SEQUENCE DESCRIPTION:
GATCCAAGTC CCCTGCCTGG TCCCCCACAA GGNCTCCCAT CCAGGCCCCC TCTGCCCTGC 60
CCCTTGTNAT GGACCATGGT CGTGAGGAAG GGCTCATGCC CCTTATTTAT GGGAACCATT 120
TCATTCTAAC AGAATAAACC GAGAAGGAAA CCAGAAA 157

SEQ ID NO:3764
SEQUENCE LENGTH:157
SEQUENCE TYPE:nucleic acid
TOPOLOGY:linear
CLONE:HUMGS04424
SEQUENCE DESCRIPTION:
GATCATTTTN CATCTGTNCA TAGCGTTTCT NCAGAAGGAA CAGTAGTCTC AAATCTTTCC 60
TCATAATGAT AACAAAATGC TCTTGCATGA TTTTNTAACA ATATATTTAA NCAGGAAGTT 120
GTCACTGATA TACTTTATTA AAAGGATTTT AATCAAA 157

SEQ ID NO:3765
SEQUENCE LENGTH:161
SEQUENCE TYPE:nucleic acid
TOPOLOGY:linear
CLONE:HUMGS04425
SEQUENCE DESCRIPTION:
GATCGTGTGT TGCCATTTNG TCTGGCTGTG GCCCCTCCTT CTCCCCTCCA GACCCCTACC 60
CTTTCCCAAA CCCTTCGGTA TTGTNCAAAG AACCCCCCTC CCCAAGGAAG AACAAATATN 120
ATTCTCCTCT CCCAAATAAA CTCCTTAACC ACCTAGTCAA A 161

SEQ ID NO:3766
SEQUENCE LENGTH:156
SEQUENCE TYPE:nucleic acid
TOPOLOGY:linear
CLONE:HUMGS04426
SEQUENCE DESCRIPTION:
GATCTTGAGG TTGTTTGATG CTTTAAATTT TTNAATTATA TTATTTNNTA GGTGTTTATT 60
GGTACATTGC AGTTTTTTTT TTNAAATTTA AAAATTTCTG TAAAACTTTG TNTTCAAGTA 120
ATCTGACAGC ATTAAATATT GCATTTAAAA ATTAAA 156

SEQ ID NO:3767
SEQUENCE LENGTH:155
SEQUENCE TYPE:nucleic acid
TOPOLOGY:linear
CLONE:HUMGS04427
SEQUENCE DESCRIPTION:
GATCCGCAAG AACAAGTACC GCCCGACCTG CGCNGGCAGC CATCCGCAGG CCAGCGCATC 60

CTGCGACAGA AGCCTGTAAT GGTAAGAGAA GCGACCCGCC CACCAAGAGT CCTGAGCCCC 120
TGCCCAGAGA ATAAAGCAGC TGGCTTTCTA CCAAA 155

SEQ ID NO:3768
SEQUENCE LENGTH:155
SEQUENCE TYPE:nucleic acid
TOPOLOGY:linear
CLONE:HUMGS04428
SEQUENCE DESCRIPTION:
GATCCCAAAA CATCATNAGG CATTTTNAAG TTGGTCACGT CGCAAGTCCG ATATCACAAC 60
TTAATTGTAA AAGGAGGGAA TCTGGTTTTG TNACTTGGCA GTGGTTTTTT CTCACCCTTC 120
CTTTTTAACA ATAAAATCCC ATTTGGGTCT TGAAA 155

SEQ ID NO:3769
SEQUENCE LENGTH:155
SEQUENCE TYPE:nucleic acid
TOPOLOGY:linear
CLONE:HUMGS04429
SEQUENCE DESCRIPTION:
GATCTATTTG ATAATTTTNT GGGTTTTTTG AAGAATTAAT GAGCATGTAC ATAGAAATAG 60
TGACTGCTTG ANTACTGTAT TTNCTTGCAC TCTTTCAGTA CATCAAGATT CCTGTATATN 120
TNAGNGTATA ATAAAACACA GATGTGAGTT GTAAA 155

SEQ ID NO:3770
SEQUENCE LENGTH:153
SEQUENCE TYPE:nucleic acid
TOPOLOGY:linear
CLONE:HUMGS04430
SEQUENCE DESCRIPTION:
GATCGGCACA AGATGCTCAG CTAGATGGGC TGGTGTGGTT GGGTCAAGNN CCCAACACCA 60
TGGCTGCCAG CTTCCAGGCT GGACAAAGCA GGGGGCTACT TCTCCCTTCC CTCGGTTCCA 120
GTCTTCCCTT TAAAAGCCTG TGGCATTTTT AAA 153

SEQ ID NO:3771
SEQUENCE LENGTH:153
SEQUENCE TYPE:nucleic acid
TOPOLOGY:linear
CLONE:HUMGS04431
SEQUENCE DESCRIPTION:
GATCTGAAGT GTGAGTAGAA TGTATTCAGC TGTTTAACAT GTAGTTTAGA TATTCAAAAG 60
TATGCATGTA GAGTTTAAAG AATATGTTAA AANTNATTAA TTTTAATATT TTGTTTGGAA 120
AAGCATGTTA TAATATAATG TTTTCACTAT AAA 153

SEQ ID NO:3772
SEQUENCE LENGTH:152

SEQUENCE TYPE:nucleic acid
TOPOLOGY:linear
CLONE:HUMGS04432
SEQUENCE DESCRIPTION:
GATCTGGNAC TCCTTGTAGT AAGCTGTTTT CTGCTCAGCC ACTGGGCTCT TTCACTTTTT 60
TAGTTCTTAA AAATTTATTT TTAAGTTCTA AAATAAAATA AAAATAAGTT CTTAAAATTT 120
ATTTTTTTCC NGAATAAATT GTATTTGGTA AA 152

SEQ ID NO:3773
SEQUENCE LENGTH:151
SEQUENCE TYPE:nucleic acid
TOPOLOGY:linear
CLONE:HUMGS04433
SEQUENCE DESCRIPTION:
GATCGCAAAT CACAGCTCAG ACAGCCAGCT CTTAAGTGAG ACCTGAGTGC CAGAGAGGGG 60
TGGATACAAG GCCAACAGTT ACTAAATGAA TGAAAATTGT GATTCCGATG AAGCCTGCCA 120
GAGAAATAAA GCATTTTTTA AAAGATGGAA A 151

SEQ ID NO:3774
SEQUENCE LENGTH:151
SEQUENCE TYPE:nucleic acid
TOPOLOGY:linear
CLONE:HUMGS04434
SEQUENCE DESCRIPTION:
GATCTAGCCA TAAAACTAGT AATCTTGAAT TCCTCTTGAG CTGGATGGTG ATTAGCCATT 60
GTGAAAACAG ATTATATGGA AATTGTGCAT ATTGCTAAAT TTTAAAACAT GGATTTATCA 120
ACTGATAATA AAATATATCT TACAAGATAA A 151

SEQ ID NO:3775
SEQUENCE LENGTH:151
SEQUENCE TYPE:nucleic acid
TOPOLOGY:linear
CLONE:HUMGS04435
SEQUENCE DESCRIPTION:
GATCTTTAGC TGCCTTTGTA TCAACTTCTN TGGAGCTTAT GTGATTTTCC AGAAAATATC 60
CAAGGCATAG TTTTGTCCCT AAGTCCCATG AATATGTAAT ATNTNGTGTC TCTCTATTAA 120
AATAGCACTT TTNGTGCATN CCATTAACAA A 151

SEQ ID NO:3776
SEQUENCE LENGTH:150
SEQUENCE TYPE:nucleic acid
TOPOLOGY:linear
CLONE:HUMGS04436
SEQUENCE DESCRIPTION:
GATCCATCAA AAGGTTCTTT TTTNATAATC CCTTTTGAAA ATAATAATCA AAGGAAGAGA 60

TGTGGTGTTT GGTCATGTGG AAAACTCAAT GTATAATTAA GACGTCTNTC AAAAATCCGA 120
CAAATAAAAT NAAGCTGGAA CGAAGGCAAA 150

SEQ ID NO:3777
SEQUENCE LENGTH:146
SEQUENCE TYPE:nucleic acid
TOPOLOGY:linear
CLONE:HUMGS04437
SEQUENCE DESCRIPTION:
GATCCNAAAG CACACCAGGA AGATGCTAAA GATGCTTATA CTTTTCCTCA AGAATTGAAA 60
GAAAAACCCA AAGAAGAGCC AGGAATACCA GCAATTCTGA ATGAGAGTCA TCCAGAAANN 120
NNNGTCTATA GTTATGTTTT GTTCNN 146

SEQ ID NO:3778
SEQUENCE LENGTH:168
SEQUENCE TYPE:nucleic acid
TOPOLOGY:linear
CLONE:HUMGS04439
SEQUENCE DESCRIPTION:
GATCAGAGAA GTTTATGCTC ACTGTCTGAT GCAACTNTCT GGTCTATTTG TNAGTAAATA 60
ACAGGGAAAT CATTTTCACT TTTTGTTAAA AATAAGGTAT TTACAAGCAT ACCTTGTAGT 120
TATTGTGGGT TCAGTTTCAG ACCACTGCAA TAAAGTGAAT ATCTCAAA 168

SEQ ID NO:3779
SEQUENCE LENGTH:149
SEQUENCE TYPE:nucleic acid
TOPOLOGY:linear
CLONE:HUMGS04440
SEQUENCE DESCRIPTION:
GATCTGACCC ACCAGTTTGT ACATCACGTC CTGCATGTCC CACACCATTT TTTCATGACC 60
TTGTAATATA CTGGTCTCTG TGCTATAGTG GAATCTTTGG TTTTGCATCA TAGTAAAATA 120
AAATAAACCC ATCACATTTG GAACATAAA 149

SEQ ID NO:3780
SEQUENCE LENGTH:144
SEQUENCE TYPE:nucleic acid
TOPOLOGY:linear
CLONE:HUMGS04441
SEQUENCE DESCRIPTION:
GATCGGGACC TGACCTGGGG CTGGCCTCAG GCCCACGTGC ACGTAACTGC CCTCTGTGGA 60
AGCCAGCTTA AACCCTAGCC CTNTNAGAGC TTCCTGTGCC CAGCAGGAAG GAAGTCAAAT 120
AAACCACACT GACTACCTGT GAAA 144

SEQ ID NO:3781
SEQUENCE LENGTH:144

SEQUENCE TYPE:nucleic acid
TOPOLOGY:linear
CLONE:HUMGS04442
SEQUENCE DESCRIPTION:
GATCTAGGGG GAAAGAGGAG CATTCATCAC AAGTTTCCTA GAGAGAGGAG ACAAATCGGT 60
GTGCCATTGA CAACATGAGC CAGGGTAAAG GCACCCTTNN NNNTTACTGA TTTCAAAGAT 120
TAATAAAGTA ATTCTATTTT TAAA 144

SEQ ID NO:3782
SEQUENCE LENGTH:139
SEQUENCE TYPE:nucleic acid
TOPOLOGY:linear
CLONE:HUMGS04443
SEQUENCE DESCRIPTION:
GATCTATGAC TCATTTGACA GAGAAGCCAA GGAAAAAAAG CTTGCTTGGC CCATTAACTC 60
AAATCCACCA AATACTTTTG TCTAAGTTCT CATTCTTTCA ATTGTTATGC ACCAGAGATT 120
AAAAAGCTTT AACTATAAA 139

SEQ ID NO:3783
SEQUENCE LENGTH:137
SEQUENCE TYPE:nucleic acid
TOPOLOGY:linear
CLONE:HUMGS04445
SEQUENCE DESCRIPTION:
GATCTGTGTC TTGAAGCAGC TGCCCTCATT CCGACTTCAG AAAATCGAAG CAGCTGGCTC 60
CTCCCCTTGT TCTCTCTCCC ACCCTCCCCC AAATCTGTTT TCATGTAAAA GACAAATAAA 120
TGATGACTTC CCCCAAA 137

SEQ ID NO:3784
SEQUENCE LENGTH:137
SEQUENCE TYPE:nucleic acid
TOPOLOGY:linear
CLONE:HUMGS04446
SEQUENCE DESCRIPTION:
GATCAAAATA AAAATGTACA TGACGGGGAA GGGAAAAACC TGTTGGAGTT GCTTCCAGCC 60
AGCCACGCTC CGGGCCAGCA TGTTGGAATC CAGCGTGGAG CAGATGCAGT AAAAATATGG 120
TTGGTTTCTG TGTGAAA 137

SEQ ID NO:3785
SEQUENCE LENGTH:137
SEQUENCE TYPE:nucleic acid
TOPOLOGY:linear
CLONE:HUMGS04447
SEQUENCE DESCRIPTION:
GATCTCTCTC TCNTTTTACC CCCCTTAGGC TGAGGGTAAA AAGCTGGGAT TGGTAGGCTG 60

GGTCCAGAAC ACTGACCGGG GCACAGTGCA AGGACAATTG CAAGGTCCCN TCTCCAAGGT 120
GCGTCATATG CNGNNNN 137

SEQ ID NO:3786
SEQUENCE LENGTH:133
SEQUENCE TYPE:nucleic acid
TOPOLOGY:linear
CLONE:HUMGS04448
SEQUENCE DESCRIPTION:
GATCGGCCAA GTATGCTNTT NTTTAGAGCA ATGTTTTGCC CTAGAGANTT GTAAAATTTA 60
TGTNATGACT CAGTACATAT GTGTTCGTAC ATATATGATN GGAATAAAAT GTTTATGAAA 120
TATTNACTCA TAN 133

SEQ ID NO:3787
SEQUENCE LENGTH:132
SEQUENCE TYPE:nucleic acid
TOPOLOGY:linear
CLONE:HUMGS04449
SEQUENCE DESCRIPTION:
GATCTGTGTG ACCTCACCTG CATTTGATTG AAAAAAGTAT GCGCGTAATT GTACCCACCC 60
AGTTCAAACC CGTGTGTAAG GGTCAACTGT ACAAAAAAGT TTGTGAAATA AACGTACTGG 120
AGAATCTTTA AA 132

SEQ ID NO:3788
SEQUENCE LENGTH:130
SEQUENCE TYPE:nucleic acid
TOPOLOGY:linear
CLONE:HUMGS04450
SEQUENCE DESCRIPTION:
GATCAGTATT TAANATTATA CCAGTTTTNA TTAAACCCTT TCCCTCCCCG ATAAAGAATG 60
TTCTATTTCT GCCTCCCCTT AAAGGGGAGA CCTCAGAAGT AAAGNAATTT NATGTTGTGT 120
TTTTNTTAAA 130

SEQ ID NO:3789
SEQUENCE LENGTH:129
SEQUENCE TYPE:nucleic acid
TOPOLOGY:linear
CLONE:HUMGS04452
SEQUENCE DESCRIPTION:
GATCCCCCTT CACCTTTTGC CATGGTCATA AGCTTCCTNA GGCCTCCCTG GAAGCTGAGC 60
AGATGCCAGC ACCATGCTTC CTGTACATCC TGCAGAACCA TAAGCCAATT AAACCTTTTT 120
AATAATAAA 129

SEQ ID NO:3790
SEQUENCE LENGTH:129

SEQUENCE TYPE:nucleic acid
TOPOLOGY:linear
CLONE:HUMGS04453
SEQUENCE DESCRIPTION:
GATCCNCCAT GGTGGACACG TCTGTNCTGT ATGTAAAAGG CATTACAAAT GTTTTTTAGC 60
AGATGAGACT TGAAGCCTTT CCACAGTCCT TGTNCTCTGA GATGGCTGTN GAGCTCTGCT 120
CANCTGTTN 129

SEQ ID NO:3791
SEQUENCE LENGTH:126
SEQUENCE TYPE:nucleic acid
TOPOLOGY:linear
CLONE:HUMGS04454
SEQUENCE DESCRIPTION:
GATCCCTACC CCTCANCAAC TAGCTCCCCT GTCGGCCAGC TAAGAGAGCT GGGACANTGG 60
AGGCTGGCAG AAGCTGAGAC GTGACTGTCT AGGAGTAACA CTCATTAAAG CTTTCNTTTT 120
GGCAAA 126

SEQ ID NO:3792
SEQUENCE LENGTH:126
SEQUENCE TYPE:nucleic acid
TOPOLOGY:linear
CLONE:HUMGS04455
SEQUENCE DESCRIPTION:
GATCTGAAAA AGCAACCCAT TTCCTAAATT CATATTTTNC CTAAAACTTT ACTATGTTTT 60
NATTTTAAGA GGTTTCCTGT TATATACACT TTTNACACAT GCAAATAAAC TTTATACCAA 120
GTGAAA 126

SEQ ID NO:3793
SEQUENCE LENGTH:126
SEQUENCE TYPE:nucleic acid
TOPOLOGY:linear
CLONE:HUMGS04456
SEQUENCE DESCRIPTION:
GATCTGTTTA GAAAATACCT TTGAAAACGA GGGTAACTTT AAAAAATGGA AACTTTCAAA 60
TCCATTTATA TTTTNNTTAT AANCAAAACT TAATTAAAAG TTTAACAAAC TGGCTGAAAA 120
CTCAAA 126

SEQ ID NO:3794
SEQUENCE LENGTH:126
SEQUENCE TYPE:nucleic acid
TOPOLOGY:linear
CLONE:HUMGS04457
SEQUENCE DESCRIPTION:
GATCACTGAG CTGCCCNTCA AGGGGACCTG GANNCCGGGT CCTGGGGTCA TGCTGCCTCC 60

NAGGCTCCAA GGTGAGGGTC ATCTTCACGA GCAAAGAGAA CCAATAAAGT GACAACGNAC 120
GTCAAA 126

SEQ ID NO:3795
SEQUENCE LENGTH:123
SEQUENCE TYPE:nucleic acid
TOPOLOGY:linear
CLONE:HUMGS04460
SEQUENCE DESCRIPTION:
GATCCAGGGC TTTCGAGGCC TCTCCTCATT GAGTTCAGAG TCACATATCA GAGGNTTAAA 60
TCCAAAAGAA ATATGCACAC CCCTCTCCTC CCTCCCAGTA AAAATAAATC GCTCCTCTTG 120
AAA 123

SEQ ID NO:3796
SEQUENCE LENGTH:125
SEQUENCE TYPE:nucleic acid
TOPOLOGY:linear
CLONE:HUMGS04462
SEQUENCE DESCRIPTION:
GATCTCTGTG CTGTAAACTA AAACAAATTG TGCATTCCTT CCGGGGCCAT CGTCTTTGTT 60
TTCTTTTTTG TCTTGAATGA ATTTTAAAAG GAAATATATA ATAAAAATGT TAACCAGAAG 120
GTAAA 125

SEQ ID NO:3797
SEQUENCE LENGTH:119
SEQUENCE TYPE:nucleic acid
TOPOLOGY:linear
CLONE:HUMGS04463
SEQUENCE DESCRIPTION:
GATCTGGGGG CCCCCCCAGC TGGCGGGAAC CCCAAATGGA CACAAACTGT ACATTTGCCA 60
ATGGGTTTTT TTCAGACCAT GGTTTTTACT TGCAAATAAA CCTGAGTTCT TTTCTGAAA 119

SEQ ID NO:3798
SEQUENCE LENGTH:133
SEQUENCE TYPE:nucleic acid
TOPOLOGY:linear
CLONE:HUMGS04464
SEQUENCE DESCRIPTION:
GATCTAAAAN NATACTNTGT TCCCTCATAT GCATGCCCTT CCTTTCTATA TCCTTGACAC 60
CTTACTTTCC CATTGTAACA ATAAAAAAAG TATCAATAAA ATAATTATTG GCAAATAAAT 120
TGGTGAGTTG AAA 133

SEQ ID NO:3799
SEQUENCE LENGTH:119
SEQUENCE TYPE:nucleic acid

TOPOLOGY:linear
CLONE:HUMGS04465
SEQUENCE DESCRIPTION:
GATCCTGCAG TGATGGAATG TTCTGTCTTG ATTGTAGCAA CACCAATATN CTGATTGTNA 60
TATTATACTA TAGTTTTGCA TAACAGTTAC CATTAAAGGA AATTGGGTAA AAGGCTAAA 119

SEQ ID NO:3800
SEQUENCE LENGTH:118
SEQUENCE TYPE:nucleic acid
TOPOLOGY:linear
CLONE:HUMGS04466
SEQUENCE DESCRIPTION:
GATCTCTTAG CAAATAGGAA TCTAGGTGTA GAATTTATAC ACATATATAT TTTTAAAAGT 60
ATAAAAATGA GAAAGTATGT ACAAAAAATC ATCCTAAATC TTAGAAAGGA GACATAAA 118

SEQ ID NO:3801
SEQUENCE LENGTH:117
SEQUENCE TYPE:nucleic acid
TOPOLOGY:linear
CLONE:HUMGS04467
SEQUENCE DESCRIPTION:
GATCTGGGAG CCAGCAGCTG GATGCTGTGG CTGGCCAGAN ACACCTCCAG GCTGTGGCCT 60
GGGGGCTGGG GGGAGCCCCA GGCTGAAAAG GGTCCAATTA AAACAAATGG AGCCAAA 117

SEQ ID NO:3802
SEQUENCE LENGTH:117
SEQUENCE TYPE:nucleic acid
TOPOLOGY:linear
CLONE:HUMGS04468
SEQUENCE DESCRIPTION:
GATCACATTC CAGCCCTCGC TGCTGGGGTG CACACCCCTT CCTTAGNTGA TGTGCTTGGG 60
AAACTCCCTC CCCCTCCTTC CCAAGAGAGA AATAAAANCA CCTTCGCCTA GGNCAAA 117

SEQ ID NO:3803
SEQUENCE LENGTH:116
SEQUENCE TYPE:nucleic acid
TOPOLOGY:linear
CLONE:HUMGS04469
SEQUENCE DESCRIPTION:
GATCCTGACC TCCTCCAAGG AAGAAATCCA GAAAGCCTTA AGACTAAGAC AACTTGACTC 60
TGCTGATTCT TTTTTCCTTT TTTTTTTTNN AAAANAAAAA TACTATNANC TGGAAA 116

SEQ ID NO:3804
SEQUENCE LENGTH:115
SEQUENCE TYPE:nucleic acid

TOPOLOGY:linear
CLONE:HUMGS04470
SEQUENCE DESCRIPTION:
GATCAGAACT CCAAAACCAC TCCCACCCCT GAAGGTCGGG AGGGTCTGAG CAGCCCTGGT 60
GGCTNCTTGT GCTCAGGTCC TCAGCTCCAT GGGAAATAAA AATGGCACCC TGAAA 115

SEQ ID NO:3805
SEQUENCE LENGTH:114
SEQUENCE TYPE:nucleic acid
TOPOLOGY:linear
CLONE:HUMGS04471
SEQUENCE DESCRIPTION:
GATCAACATA TTTAGCTTAT ACAGAAATAA AATTAAGTCA ATCCACTCAC AAAGAATTTC 60
TATTTTGTAA AAATGTAGCT TGTATTTCAG TATAATAAAA TCTGATGTAA GAAA 114

SEQ ID NO:3806
SEQUENCE LENGTH:114
SEQUENCE TYPE:nucleic acid
TOPOLOGY:linear
CLONE:HUMGS04472
SEQUENCE DESCRIPTION:
GATCANAGCT NTCCCAAAGA GGGAAAGCGG TGAGGTTTNA GGAGGGGCAG AAGCAGGGCC 60
GGCAAAGNTT GTACCTNCAT AAGGTGGTAT GGGGGGTTGG GGTCAGGCCC TGAN 114

SEQ ID NO:3807
SEQUENCE LENGTH:112
SEQUENCE TYPE:nucleic acid
TOPOLOGY:linear
CLONE:HUMGS04473
SEQUENCE DESCRIPTION:
GATCGAAAGA CTCAGGGAGA GCGGTCCTNA CCACCTGGAA GTAAGTCCCA CCTGGACCTT 60
ATNAAAACTA CTCACTCCAC CTGGGCCCCT GCTGAAGCCN NNTTTTAAAA TN 112

SEQ ID NO:3808
SEQUENCE LENGTH:111
SEQUENCE TYPE:nucleic acid
TOPOLOGY:linear
CLONE:HUMGS04474
SEQUENCE DESCRIPTION:
GATCTGTTTC GTGCATTGGA AGACCCCACC CAAGCTTGGC ANACTAANTN TTGTATCCTG 60
GGGCTCCCNT CATCTCCAGG GAGACCAAAC CCCGGCCCTA CCAGNGCCCG N 111

SEQ ID NO:3809
SEQUENCE LENGTH:108
SEQUENCE TYPE:nucleic acid

TOPOLOGY:linear
CLONE:HUMGS04476
SEQUENCE DESCRIPTION:
GATCAAGACT AACAAATAAC TTTGTGATGA AAATNCTTCA AAAATATAGC TACTCTTTTC 60
AAGTATACCA TTTAAAATAT TTCATCAGGC AGAGCCCTGA CCAGGAAA 108

SEQ ID NO:3810
SEQUENCE LENGTH:108
SEQUENCE TYPE:nucleic acid
TOPOLOGY:linear
CLONE:HUMGS04478
SEQUENCE DESCRIPTION:
GATCAAGAAN ANTNAGGACA ACGAGAAAGT TTAAAAGTTC GAAAGCAGCA AGATNCCTTT 60
CCCCCCTGGT CAATCNCTGA CACAAGAGAA GGCAGAGACC CTGAAGCN 108

SEQ ID NO:3811
SEQUENCE LENGTH:182
SEQUENCE TYPE:nucleic acid
TOPOLOGY:linear
CLONE:HUMGS04482
SEQUENCE DESCRIPTION:
GATCTTCAAG CAAGGGAAAT GATTCTTAAA AGGATTATCT GAAATTGAAA AACTAGTAAG 60
CAAATAAATG ATAAACATAA GTAAATTTAA NCTGTTGTTT GTATAAAACA ATTATAATGA 120
NGTGTTTATA AGANATTGTA AACAAATGTA AANCTAAATN ANCATTGTTA TGCAACATTA 180
AA 182

SEQ ID NO:3812
SEQUENCE LENGTH:105
SEQUENCE TYPE:nucleic acid
TOPOLOGY:linear
CLONE:HUMGS04483
SEQUENCE DESCRIPTION:
GATCTCCCCA AACTAAAAAG TACAGTACTT GGAATTGTGT TCTTTATGGT TGTAGTGTTG 60
GTAAAGCACT AATATGCAGA AAATAAAGGA ATTACACAGT GCAAA 105

SEQ ID NO:3813
SEQUENCE LENGTH:105
SEQUENCE TYPE:nucleic acid
TOPOLOGY:linear
CLONE:HUMGS04484
SEQUENCE DESCRIPTION:
GATCCNAAGC AGTGAACATG CTGTTAATAC TTGTAAGACT CCTAGCAAAC TGCATTTAAT 60
TTTTTAAATT AGTTTTAGTG TACATAAAGG CATGTNTTTN CCAAA 105

SEQ ID NO:3814

SEQUENCE LENGTH:105
SEQUENCE TYPE:nucleic acid
TOPOLOGY:linear
CLONE:HUMGS04485
SEQUENCE DESCRIPTION:
GATCCATGGG ATTNATAATT GAAATAGAGA TTGGAGACCC TCCTATTCAN TTCATAAGCA 60
ATNGCACCAC AGGTCANATG CAGTTATCTN AACTCCAGGA AATTN 105

SEQ ID NO:3815
SEQUENCE LENGTH:104
SEQUENCE TYPE:nucleic acid
TOPOLOGY:linear
CLONE:HUMGS04486
SEQUENCE DESCRIPTION:
GATCTTGCTA AATTATTAGT TCTAGTAGCT TTTTTGTAGA TTCATTAGGA TTTTNTACAT 60
ACACAATCAT GCTATTTGCA AATAAAATAA TGCTTTTATC CAAA 104

SEQ ID NO:3816
SEQUENCE LENGTH:104
SEQUENCE TYPE:nucleic acid
TOPOLOGY:linear
CLONE:HUMGS04487
SEQUENCE DESCRIPTION:
GATCGAGTCC AAGGNAGACT ATGTCCTCTN CTACCAACGN CAGGNCGTGG CGCGACGCCT 60
GCTGTCCCCG GCCGGCTCAT CTGGCGCCCC AGCCTCCCCT GNCN 104

SEQ ID NO:3817
SEQUENCE LENGTH:103
SEQUENCE TYPE:nucleic acid
TOPOLOGY:linear
CLONE:HUMGS04488
SEQUENCE DESCRIPTION:
GATCAGCACT GTGGTGAGCT CCAAGGAAGT GAATAAGTTT CANATGGCTT ATTCAAACCT 60
CCTTAGAGCT AACATGGNTG GGCTGAAGAA GAGAGACAAN NNN 103

SEQ ID NO:3818
SEQUENCE LENGTH:103
SEQUENCE TYPE:nucleic acid
TOPOLOGY:linear
CLONE:HUMGS04489
SEQUENCE DESCRIPTION:
GATCCCAAAC CTNAGCTCAA TCAGTATTGC CAGANAGGGG TAAGACTGGT TGGAAGCTGA 60
CTGCAGACTT NNTTTCCCCT TAGTATGTNC TGTGTTGTAA ATN 103

SEQ ID NO:3819

SEQUENCE LENGTH:102
SEQUENCE TYPE:nucleic acid
TOPOLOGY:linear
CLONE:HUMGS04491
SEQUENCE DESCRIPTION:
GATCACTAAG CAAAGCTGCT AGTGGGATTC TATATTTCGT GTCATCTTTT TTATNATAAT 60
NTATTGCAAA TATTTTTCTG AATAAATATA TGTTGTGTGA AA 102

SEQ ID NO:3820
SEQUENCE LENGTH:102
SEQUENCE TYPE:nucleic acid
TOPOLOGY:linear
CLONE:HUMGS04492
SEQUENCE DESCRIPTION:
GATCCTGCCA CCAGGGTTNT TTTGAAATAG TACCACATGT AAAAGGGAAT TTGGNTTTNA 60
NTTCATCTAA TCACTGANTT GTCAGGCTTT GATTGATAAT TN 102

SEQ ID NO:3821
SEQUENCE LENGTH:102
SEQUENCE TYPE:nucleic acid
TOPOLOGY:linear
CLONE:HUMGS04493
SEQUENCE DESCRIPTION:
GATCTTGTTC AATCGGAAAC CCCCGTTACC TCCTCTTTTT CTTTCTCTTT CTTTTTTTTT 60
TTTAACTNAA ANATTTTNAT GATGATTAAG ANGGAAGTNG TN 102

SEQ ID NO:3822
SEQUENCE LENGTH:102
SEQUENCE TYPE:nucleic acid
TOPOLOGY:linear
CLONE:HUMGS04494
SEQUENCE DESCRIPTION:
GATCCCAAAC ACTCNATCCT GTAAGNCGGG TACAGTCCTA TTGTNTGTTN NCACACTGAT 60
GATGTGAGCC TGTGAGATGA GCCAATCACC AGTTGTTATG TN 102

SEQ ID NO:3823
SEQUENCE LENGTH:101
SEQUENCE TYPE:nucleic acid
TOPOLOGY:linear
CLONE:HUMGS04495
SEQUENCE DESCRIPTION:
GATCTCATGT ACTGAAAAAA AATTCTTAAT TTTATTGGAG TTTATACCAC TGTGAAACTG 60
CAATATGACT GTAAGAGAGT AAAAAGATTG TNACATCTAA A 101

SEQ ID NO:3824

SEQUENCE LENGTH:101
SEQUENCE TYPE:nucleic acid
TOPOLOGY:linear
CLONE:HUMGS04496
SEQUENCE DESCRIPTION:
GATCTGGGAG TCTGAAATGG TAGCNTTTTG TCCCTGTCTT CACACTATCA TAGGGAGAAT 60
CAAAAGANCT AACAAATATA ANCATGCTTT GTGAATNNCC N 101

SEQ ID NO:3825
SEQUENCE LENGTH:100
SEQUENCE TYPE:nucleic acid
TOPOLOGY:linear
CLONE:HUMGS04497
SEQUENCE DESCRIPTION:
GATCTNTTTT NTNTCTATTT TTTGAGGTAC ATGCTACATA CATTTTNTNT GTAAACAACT 60
ATTTAGCATA TTGAATAAAA TAACATTTTA AAAATAGAAA 100

SEQ ID NO:3826
SEQUENCE LENGTH:97
SEQUENCE TYPE:nucleic acid
TOPOLOGY:linear
CLONE:HUMGS04498
SEQUENCE DESCRIPTION:
GATCCCACGG CNAACACTCA CCACNCGCTA CCATACGCCN TCCCAGAGCT AGGGGCTGCC 60
TTAAAGGAAA TTGTAGCCTA AGTAGGTCAT GGCAAGN 97

SEQ ID NO:3827
SEQUENCE LENGTH:96
SEQUENCE TYPE:nucleic acid
TOPOLOGY:linear
CLONE:HUMGS04499
SEQUENCE DESCRIPTION:
GATCAGCCAC ATCAACTCAG TTGTCCACCA CAGGGGAATT TTNAATGTCT TTTGTTTTTG 60
TNTTGTTTTG AAAAATAATA AACAGGCAAC TGTAAA 96

SEQ ID NO:3828
SEQUENCE LENGTH:95
SEQUENCE TYPE:nucleic acid
TOPOLOGY:linear
CLONE:HUMGS04500
SEQUENCE DESCRIPTION:
GATCTATTTG GCCTCTCAAA TGAACTGAGA TTCCTGTTAA AAAAGATTGA TGTTATTGTC 60
TCTTGTAGAG GAAACTAATA AAGCTGCAGG NGAAA 95

SEQ ID NO:3829

SEQUENCE LENGTH:94
SEQUENCE TYPE:nucleic acid
TOPOLOGY:linear
CLONE:HUMGS04501
SEQUENCE DESCRIPTION:
GATCTATCAT AATTGCTTTT CTGATGCATT TAAAAATAAA TGGAACACTT AAATGTATAT 60
TACCGATGCA AACTGTGGAT AAAGTTGAAA GAAA 94

SEQ ID NO:3830
SEQUENCE LENGTH:94
SEQUENCE TYPE:nucleic acid
TOPOLOGY:linear
CLONE:HUMGS04502
SEQUENCE DESCRIPTION:
GATCACAGGC CTGGCCGCGG TAACAGACTT TTACATGGAA TTGTTTAATT ATTTGTACTT 60
TTCATGCCAG AAAATAAAAG TTCAGAATCT TAAA 94

SEQ ID NO:3831
SEQUENCE LENGTH:94
SEQUENCE TYPE:nucleic acid
TOPOLOGY:linear
CLONE:HUMGS04503
SEQUENCE DESCRIPTION:
GATCACTACG ATTAACGACG GTCGTAATGT TAAGACGTCG TCTCCATGAN CTTTGGGGGG 60
ACTTTAATNT GGAATAAAGA AACTATCACT NAAA 94

SEQ ID NO:3832
SEQUENCE LENGTH:93
SEQUENCE TYPE:nucleic acid
TOPOLOGY:linear
CLONE:HUMGS04504
SEQUENCE DESCRIPTION:
GATCCAGATN AAGTGCTGAA GAAAAGAAAA AAGAAGTGGA GAAGAGTATG NTTATNCTTC 60
TCCTTATTAA AGAAATTTNC TCAAAACAGC AAA 93

SEQ ID NO:3833
SEQUENCE LENGTH:88
SEQUENCE TYPE:nucleic acid
TOPOLOGY:linear
CLONE:HUMGS04506
SEQUENCE DESCRIPTION:
GATCTATCCA GTGACGTGGC CTGGTGGGCG TTTCTCCTTG TACTTATGTG GTTTTTTGGC 60
TTTNAATACA GACATTTTCC TCCAGAAA 88

SEQ ID NO:3834

SEQUENCE LENGTH:86
SEQUENCE TYPE:nucleic acid
TOPOLOGY:linear
CLONE:HUMGS04507
SEQUENCE DESCRIPTION:
GATCCCAAAA TATGGTAAAG TGAACCCATC TGTCTGCATT TTCTACTCTG AGCCCATTTG 60
TTAATAAACA CTTATTTTTA TATAAA 86

SEQ ID NO:3835
SEQUENCE LENGTH:84
SEQUENCE TYPE:nucleic acid
TOPOLOGY:linear
CLONE:HUMGS04509
SEQUENCE DESCRIPTION:
GATCCGGGCC CCAGGCTGCT GCCGCTTTTT ATAACTTTAT ATTATTTTTN TTTTAAGAAA 60
AAAAGCTCTT TAAAATACCT CAAA 84

SEQ ID NO:3836
SEQUENCE LENGTH:82
SEQUENCE TYPE:nucleic acid
TOPOLOGY:linear
CLONE:HUMGS04510
SEQUENCE DESCRIPTION:
GATCTGAGGA GCAAACCTCC GTGGCACACA TTTACCTGCA TAACAAACCT GCACATCCTG 60
CATGTGTACC CCAGAACTTA AA 82

SEQ ID NO:3837
SEQUENCE LENGTH:81
SEQUENCE TYPE:nucleic acid
TOPOLOGY:linear
CLONE:HUMGS04511
SEQUENCE DESCRIPTION:
GATCCAGGAG GCTCAAAGAG AAGCCAAGTC AGCTTTGTTG TGATTTGATT TTTTTTAAAA 60
AACTCTTGTA CAAAACTGAA A 81

SEQ ID NO:3838
SEQUENCE LENGTH:81
SEQUENCE TYPE:nucleic acid
TOPOLOGY:linear
CLONE:HUMGS04512
SEQUENCE DESCRIPTION:
GATCGCTACT GTTATGTGGG ATTATTATTT CTAAATNTTA CTCATTGAAA TAAGCATACA 60
ATAANANGCA TTTATTGCAA A 81

SEQ ID NO:3839

SEQUENCE LENGTH:80
SEQUENCE TYPE:nucleic acid
TOPOLOGY:linear
CLONE:HUMGS04513
SEQUENCE DESCRIPTION:
GATCAAAACA AGACCCAGCT TATTTTCTGC TTGCTGGTAA ATTAAGCAAA CATGCTATAA 60
TAAAAACAAA ATGAAGGAAA 80

SEQ ID NO:3840
SEQUENCE LENGTH:78
SEQUENCE TYPE:nucleic acid
TOPOLOGY:linear
CLONE:HUMGS04514
SEQUENCE DESCRIPTION:
GATCTGGTTG AAAATTGTAT TTCTATGTAA ACTCAACGAT ATGTTTGGTT TTCCTGAAAA 60
TAAATAATTT TAAATAAA 78

SEQ ID NO:3841
SEQUENCE LENGTH:75
SEQUENCE TYPE:nucleic acid
TOPOLOGY:linear
CLONE:HUMGS04515
SEQUENCE DESCRIPTION:
GATCCTCACA CCTCAGCGTC CCAGAGTGCT GGGATTACAG TTGTGAGCCA CTGTGCCTGG 60
CCTTTTTTTT TTTTN 75

SEQ ID NO:3842
SEQUENCE LENGTH:74
SEQUENCE TYPE:nucleic acid
TOPOLOGY:linear
CLONE:HUMGS04516
SEQUENCE DESCRIPTION:
GATCAGAAAT CTGAGGTTCA ATGAGGCCAG AATAACTCAG CTTCAAAATG AATAAATGTG 60
GAAGCCAGCA CAAA 74

SEQ ID NO:3843
SEQUENCE LENGTH:72
SEQUENCE TYPE:nucleic acid
TOPOLOGY:linear
CLONE:HUMGS04517
SEQUENCE DESCRIPTION:
GATCTCCCCT TGGACTGATA CCCTTTTCCC ATTCATTCAC AAATAAATTA CAATGGGTGC 60
TGAGAACTTA AA 72

SEQ ID NO:3844

SEQUENCE LENGTH:78
SEQUENCE TYPE:nucleic acid
TOPOLOGY:linear
CLONE:HUMGS04518
SEQUENCE DESCRIPTION:
GATCTGTGTC ATGGTTTTAT AACCTTTNTN TNTTTGTAAA CTTGAATGTT CAAAATTAAC 60
ATGCTGTTTA CTCTGAAA 78

SEQ ID NO:3845
SEQUENCE LENGTH:71
SEQUENCE TYPE:nucleic acid
TOPOLOGY:linear
CLONE:HUMGS04519
SEQUENCE DESCRIPTION:
GATCAGGAAG AACTAAACAA AACCCACACT CCAAAAACTA ACAAAGAATA AATAAATAAT 60
ATAAAAATAA A 71

SEQ ID NO:3846
SEQUENCE LENGTH:71
SEQUENCE TYPE:nucleic acid
TOPOLOGY:linear
CLONE:HUMGS04520
SEQUENCE DESCRIPTION:
GATCTCAGAA CAGANTTAAA ATATTGTGAA AATTACAGCT TAATAAATGT TTATTGTAAT 60
TTTNTTGTAA A 71

SEQ ID NO:3847
SEQUENCE LENGTH:69
SEQUENCE TYPE:nucleic acid
TOPOLOGY:linear
CLONE:HUMGS04521
SEQUENCE DESCRIPTION:
GATCTAGGAC TTGCCTTATT TTATCATTTT TCTTTAATAA TCTCCAATAA ACTTTCTTTA 60
TTCATTAAA 69

SEQ ID NO:3848
SEQUENCE LENGTH:69
SEQUENCE TYPE:nucleic acid
TOPOLOGY:linear
CLONE:HUMGS04522
SEQUENCE DESCRIPTION:
GATCACCACT AAAGAACTTA CTCATATAAC CAAATACCAC CTGTNCTCCC AAAAACCTAT 60
GGAAATAAA 69

SEQ ID NO:3849

SEQUENCE LENGTH:69
SEQUENCE TYPE:nucleic acid
TOPOLOGY:linear
CLONE:HUMGS04523
SEQUENCE DESCRIPTION:
GATCAGTATG CTGTTTTAAT AATTATGTGC CATTTTAATA AAATGAAAGG GTCAACGGCC 60
CNNTTTAAA 69

SEQ ID NO:3850
SEQUENCE LENGTH:67
SEQUENCE TYPE:nucleic acid
TOPOLOGY:linear
CLONE:HUMGS04525
SEQUENCE DESCRIPTION:
GATCTACACT GATGATTAAA TCAAATTGGC TAAGATTATT CGTTCTAATA AAAGCACTGA 60
CTCCAAA 67

SEQ ID NO:3851
SEQUENCE LENGTH:67
SEQUENCE TYPE:nucleic acid
TOPOLOGY:linear
CLONE:HUMGS04527
SEQUENCE DESCRIPTION:
GATCTCTTCC TGTTTTGTAC ATAGATTTAT TTTTCAGTTC CAAGAAAGAT GAATACATTT 60
TGTNAAA 67

SEQ ID NO:3852
SEQUENCE LENGTH:67
SEQUENCE TYPE:nucleic acid
TOPOLOGY:linear
CLONE:HUMGS04528
SEQUENCE DESCRIPTION:
GATCTAGCAT ATGTATCTTT TTTAAAGGTA TTGTTAATAA ATATNCTGTC ATTTGTAAAG 60
ATANAAA 67

SEQ ID NO:3853
SEQUENCE LENGTH:66
SEQUENCE TYPE:nucleic acid
TOPOLOGY:linear
CLONE:HUMGS04529
SEQUENCE DESCRIPTION:
GATCCATCTT CAAATCAAGG GCTCCCTGGC TACCAAATNC TGTCTAGTAA AAATNATTCG 60
AGCAAA 66

SEQ ID NO:3854

SEQUENCE LENGTH:64
SEQUENCE TYPE:nucleic acid
TOPOLOGY:linear
CLONE:HUMGS04530
SEQUENCE DESCRIPTION:
GATCTATTCA ATGTACAAAA TATTTTGAAA GTTTCTGTGA TTAAATGTNC TTTGAAAACA 60
TAAA 64

SEQ ID NO:3855
SEQUENCE LENGTH:64
SEQUENCE TYPE:nucleic acid
TOPOLOGY:linear
CLONE:HUMGS04531
SEQUENCE DESCRIPTION:
GATCTGTTAT NAACACCTTT GTTGGCTTGG TTCAGTAATA AATATGTNAG ACTTTTCATT 60
TAAA 64

SEQ ID NO:3856
SEQUENCE LENGTH:62
SEQUENCE TYPE:nucleic acid
TOPOLOGY:linear
CLONE:HUMGS04532
SEQUENCE DESCRIPTION:
GATCTGTGTA AGCTCTGAAT GAACTTCTTT ACTCAATAAA ATTAATTTTT TGGCTTCTTA 60
AA 62

SEQ ID NO:3857
SEQUENCE LENGTH:61
SEQUENCE TYPE:nucleic acid
TOPOLOGY:linear
CLONE:HUMGS04533
SEQUENCE DESCRIPTION:
GATCCAAGAT ACGTCATTTC TGTATTGGCA AAATGCCACT ATTAAAGTGT AATTCTTGAA 60
A 61

SEQ ID NO:3858
SEQUENCE LENGTH:60
SEQUENCE TYPE:nucleic acid
TOPOLOGY:linear
CLONE:HUMGS04534
SEQUENCE DESCRIPTION:
GATCTCAAGT GCTTAATGAT AAGGTGTTGA CTTGTTAAAT TAAACCATTT GGAATACAAA 60

SEQ ID NO:3859
SEQUENCE LENGTH:60

SEQUENCE TYPE:nucleic acid
TOPOLOGY:linear
CLONE:HUMGS04535
SEQUENCE DESCRIPTION:
GATCAAATGC CCTAAAATGT AGTGACCCGT GAAAAGGACA AATAAAGCAA TGAATACAAA 60

SEQ ID NO:3860
SEQUENCE LENGTH:60
SEQUENCE TYPE:nucleic acid
TOPOLOGY:linear
CLONE:HUMGS04536
SEQUENCE DESCRIPTION:
GATCTGAGAC TTTGAGTCTC CAGAACTGTG AGAAAATAAA TTTCTGTTTC TTAAGCCAAA 60

SEQ ID NO:3861
SEQUENCE LENGTH:60
SEQUENCE TYPE:nucleic acid
TOPOLOGY:linear
CLONE:HUMGS04537
SEQUENCE DESCRIPTION:
GATCTTTCCT GTCTTCCCCA AGTTTGCATT TCCGACATTA AAGTTTACTT TTNAGTTAAA 60

SEQ ID NO:3862
SEQUENCE LENGTH:59
SEQUENCE TYPE:nucleic acid
TOPOLOGY:linear
CLONE:HUMGS04538
SEQUENCE DESCRIPTION:
GATCAATCAG GGAGAAAATA CAGAAGAGAA TAAAAAGAGA CTCATTCAGT GGAAGCAAA 59

SEQ ID NO:3863
SEQUENCE LENGTH:59
SEQUENCE TYPE:nucleic acid
TOPOLOGY:linear
CLONE:HUMGS04539
SEQUENCE DESCRIPTION:
GATCTGAATT AAGCTGTCTG CCATTTGGAG AAATTTAAAT ACTATTTTAA ACACACAAA 59

SEQ ID NO:3864
SEQUENCE LENGTH:58
SEQUENCE TYPE:nucleic acid
TOPOLOGY:linear
CLONE:HUMGS04541
SEQUENCE DESCRIPTION:
GATCCACCCA GGCCTTTCNG TGAAATAAAA GCTCCCAAGT TGGGTACAAA CCAGGAAA 58

SEQ ID NO:3865
SEQUENCE LENGTH:57
SEQUENCE TYPE:nucleic acid
TOPOLOGY:linear
CLONE:HUMGS04542
SEQUENCE DESCRIPTION:
GATCCCAGTC TGAAGGATTA TTGTGAAGAA TAAATGAGAG AATGTGTAAA TGGCAAA 57

SEQ ID NO:3866
SEQUENCE LENGTH:56
SEQUENCE TYPE:nucleic acid
TOPOLOGY:linear
CLONE:HUMGS04543
SEQUENCE DESCRIPTION:
GATCTGTGTA TTTGATTTTG TACTTTAAAT GTGACAAATA AACCTTTTGG GAGAAA 56

SEQ ID NO:3867
SEQUENCE LENGTH:55
SEQUENCE TYPE:nucleic acid
TOPOLOGY:linear
CLONE:HUMGS04546
SEQUENCE DESCRIPTION:
GATCTAATTC TGAAAACATT GTAATAAAAT AATNAGCTAT AATGGTATTT TCAAA 55

SEQ ID NO:3868
SEQUENCE LENGTH:55
SEQUENCE TYPE:nucleic acid
TOPOLOGY:linear
CLONE:HUMGS04547
SEQUENCE DESCRIPTION:
GATCTCGAGC GTTTATCTCG GGCTTTAATT TGCTAAAGCT NTGCACATAT GTAAA 55

SEQ ID NO:3869
SEQUENCE LENGTH:85
SEQUENCE TYPE:nucleic acid
TOPOLOGY:linear
CLONE:HUMGS04548
SEQUENCE DESCRIPTION:
GATCTGCCAT GCACTCCAGC CTAGGCAACA AGAGTGAACT CTGTCTCAAA ATAAAATAAA 60
ATAAAATAAT AATAGTAATA ATAAA 85

SEQ ID NO:3870
SEQUENCE LENGTH:52
SEQUENCE TYPE:nucleic acid

TOPOLOGY:linear
CLONE:HUMGS04552
SEQUENCE DESCRIPTION:
GATCAAAGGT CATTTGTGTA GATGAGTAAT TAAAAAATAT TTAAATCACA AA 52

SEQ ID NO:3871
SEQUENCE LENGTH:51
SEQUENCE TYPE:nucleic acid
TOPOLOGY:linear
CLONE:HUMGS04556
SEQUENCE DESCRIPTION:
GATCAAATAG AAACAGGGTT TGCAAGTNTC CATACAGATG CCTCTACCAA A 51

SEQ ID NO:3872
SEQUENCE LENGTH:50
SEQUENCE TYPE:nucleic acid
TOPOLOGY:linear
CLONE:HUMGS04557
SEQUENCE DESCRIPTION:
GATCCTATGT AAAATATATA TTTATGTATT AAAAATACTG AAAACATAAA 50

SEQ ID NO:3873
SEQUENCE LENGTH:443
SEQUENCE TYPE:nucleic acid
TOPOLOGY:linear
CLONE:HUMGS04597
SEQUENCE DESCRIPTION:
GATCCTATTT TATAAGGAAT CTTTTTTATA CGAATGTACT TATTCTTTTA GATATCAGTT 60
CTTGAATGTA TACTCCTTAT GATATACAGC AAGAGATTCT TGAGGTTCCC AAGAATTGTC 120
CTGGAAAGAA AATTGATTTT GAATTGAGAT TAGCAGTGTT TTAATTTCGC TGAGATAACT 180
CCTTTAACTC AGCACTTTCA CAAAACTTAA ATATTAAATA AATTGAATTT GAGGCTGGCA 240
TTCTTCTCTA AATTGAATTG GACTGGTTGT TTGCTGAGGT ATGAATGGAG CAGCTCGGAA 300
GTATTATAAT TCCGTAGCTG AAACAGGACG CTTATTTATG TATGNTCCAA TCTCTNCTCA 360
TTTAAAAATG NCTCAGCATT TCCNGCCATT TAGATAGGTA AATNAATAGT CCATTANNTT 420
TGNTNACCTA CCATAANTAT TTN 443

SEQ ID NO:3874
SEQUENCE LENGTH:423
SEQUENCE TYPE:nucleic acid
TOPOLOGY:linear
CLONE:HUMGS04598
SEQUENCE DESCRIPTION:
GATCTATGGT ACAGTTCATT GGCAGAGAAT CCAAGTACTG TGGACTTTGT GATTCCATAA 60
TAACCATCTA TCGGGAAGAG GGCATTCTAG GATTTTCCGC GGGTCTTGTT CCTCGCCTTC 120
TAGGTGACAT CCTTTCTTTG TGGCTGTGTA ACTCACNNCC CTACCTCGTC AATACCTATG 180

CACTGGACAG TGGGGTTTCT ACCATGAATG AAATGAAGAG TTATTCTCAA GCTGTCACAG 240
GATTTTTTGC GAGTATGTTG ACCTATCCCT TTGTGCTTGT CTCCAATCTT ATGGCTGTCA 300
ACAACTGTGG TCTTGCTGGT GGATGCCCTC CTTACTCCCC AATATATACG NCTTGGGATA 360
GACTGTTGGT GCATGCTACA AAAAGAGGGG GAATANGNAG CCGAGGGAAA TAGCTTATTT 420
TTN 423

SEQ ID NO:3875
SEQUENCE LENGTH:413
SEQUENCE TYPE:nucleic acid
TOPOLOGY:linear
CLONE:HUMGS04599
SEQUENCE DESCRIPTION:
GATCCAAATN ACCATCATCT CTGATGGAGA TGGGTTGGGT ACCTGGCCTT CATGGCACCT 60
TCACTGCTAG GGATGCTCAA GGGGCAGGCC TGGGGCCTTC CCTCCTGTCT CTTCTCGGTC 120
TTTCCTCTCT GAGCANCCTC CTACCTCCCC TGCCTGAGCC TCACTCCACA GCCCTCCCAG 180
GTACCTAGCA GAGGCTGTCA GTCCTTGGCT CACCTGGAAC AGGGCTGGGG CTGGGTTGGA 240
ACAAGGTGTG TGCCNCACCA CATCTCTATG ACTCTGTTCT CCCTCCCTGC ATTGTGGCTC 300
TTGTATTTGA GGGCCTCAAG AGAGTGAGGC CCTACCATCA CTGTCCATAT TAAGTCCAAG 360
CCCAAGTGNG GTTCCTCTGT TCCCTCNTAA GCAATCAATN GTTNAGTTTC TAN 413

SEQ ID NO:3876
SEQUENCE LENGTH:410
SEQUENCE TYPE:nucleic acid
TOPOLOGY:linear
CLONE:HUMGS04600
SEQUENCE DESCRIPTION:
GATCCACCTG CCTCGGACTC CCACAGTGCT GGGATTACAG GTGTTAGCCA CCACCCCGAC 60
ATTATTTGAA ACTTTTATTT TATCATGAGA GAGTTCCAGG AGTCAACTGG AGAGAGATTT 120
TTGGTATGAA AATTACATAT GCAAAAAGAC TGATTCCAGT ACATGAAATT AAATTCAACA 180
TTTACATTAA ATGCCTTCAA ATATGGTAAA ATGGTTTCTT TTGGCACNNT ACCTCATTAT 240
GTTTTGAATG ATTTGGTCTA TCATATGAAA TAACTTTTAT AAATATAGTA NCTCAGGCCT 300
GGGCACAGCG GCTCAAGTGG GAGGNCTGCT TAAGCACCCG AGTTTNAGAC CAGCGTGGAC 360
AACATGGGGG GACCCCTTNC TGTCCCAAAA NTAGCTGAGC ATGGCAGCGN 410

SEQ ID NO:3877
SEQUENCE LENGTH:402
SEQUENCE TYPE:nucleic acid
TOPOLOGY:linear
CLONE:HUMGS04601
SEQUENCE DESCRIPTION:
GATCCCAACA ATGGAACTTT CCTTCTCTGC TGTCAACAAA GGACTAAAAA ACTTCTGTGT 60
GATTTGATAC AGACAATTTA GAAAACTTTT GTTTCAAAGT TTCCCATCCA GACTAGCCAA 120
GGAGAGCAAG GGAGGGNNNC TAATAAGCCC ATTAAGGTGT CTTCCCAAAC AAATGTTTTC 180
TTTGTCCTTC TGCCCAAATG CAGAAACAGC TGCAGCTTTA AAAANTAGCA TACTTTTATC 240
TTTTGANGGA AATATGTGAA AGTTTTAGCA CTGGTTGTCT TTGGTTGGCA AGAAATTGTT 300

NAGTGTTATN NATTNCTTCC TATTTGNCTG AATTTTNCCA AATTTGTCCA CAGCAAGCAT 360
GACTACTTTN NTCATATNAA ATCTCCANTG NATTTATGCA AA 402

SEQ ID NO:3878
SEQUENCE LENGTH:393
SEQUENCE TYPE:nucleic acid
TOPOLOGY:linear
CLONE:HUMGS04602
SEQUENCE DESCRIPTION:
GATCTTGTCA CTAAAATCTA ATTTATATCA AATTTATGAG AGAAAGTATT TTCCTAATNA 60
TGGTCAAATA AATTTGGTTA ACATCCTAGT GATTCTCTTT CTATATAATA AGGCAATTAC 120
AGTTTTCAAA GCATTAAGTC TAACATAACT TTAAACATTC TCTTAGGTTT CAAGACACTT 180
CTATTTAATA TTCATTGGGG AAAAGTTGTC CAGCTATCAG CTAGNGAAAA CACATGCAAA 240
TATGGTTGTG TAAAGTTAAG GGTTATANGG NANNANANAT CAGTAGAATT ACATAATACT 300
AAANGTTGCA GTTGAAGGAA TATCCCAGTN TGTGTTGGTA GTTNCTNNAA GNNTTNTAGC 360
TGTNATTGCC TTGTTTTTNT AGCCCTTNGT TTN 393

SEQ ID NO:3879
SEQUENCE LENGTH:392
SEQUENCE TYPE:nucleic acid
TOPOLOGY:linear
CLONE:HUMGS04603
SEQUENCE DESCRIPTION:
GATCTNAAGC TTNCTNCTTG GACACCCCAT ACCCANAGTC CTCCAGGCCA CCCTTAAGTA 60
CAATGTTCTC CTACCTAAGA AGGCATCTGG ATTTTNTCTT TCCTTGGAAA TAGTAAAGAA 120
CTACTCTTTG ACTGTTTTTG ACCTCACAGT GAACCTCAAA TACACTGGAA TTCGCAATAA 180
ATCCAGTATG GTGGTTATAG ATGTAAAAAT GCTATCAGGA TTTACTCCAA CCATGTCATC 240
CATTGAAGAG CTTGAAAACA AGGGCCAAGT GATGAAGACT GAAGTCAAGA ATGACCATGT 300
TCTTTTCTAC TTGGAAAATG TTTTTGGTCG AGCAGACAGT TTCACTTTTT NCTGTTGAGC 360
AGAGCAACCT TGTGTTCAAC ATTCAGCCAG GN 392

SEQ ID NO:3880
SEQUENCE LENGTH:385
SEQUENCE TYPE:nucleic acid
TOPOLOGY:linear
CLONE:HUMGS04604
SEQUENCE DESCRIPTION:
GATCTCACCA CTGCACTCTA GCCTGGACGA CAGAGGGAGA CTGTCTCCAA AAAAAAACAA 60
AGAAAGAAAG AAAAAACTGT AAAAAAAAAT TTNCTGCAAT GGNCTTAATA TGTGTGTCCC 120
TTAAAAATGT ATATATTGAA ACTCTAATCC CAATGTGATG TTATTTAGAG GTGGGGCCTG 180
TAGGAGGTAA CTGGGTCCTA AGGGTAGAGC CCTCATGANT GAGATTAGTG CCCTTATAAG 240
ANGCCCAAAA GCTAGCTACC TCTNTTTCCA CTATGTAAGG NTACAAAGNG AAGTTGGCTG 300
TCTGCAAACT AGAAGAGGGC TGATACAGTT TGGCTGTGTC CCCACCCAAA TCTTCATCTT 360
AGGTTTTTAA CTNCCGCAAT TCCCN 385

SEQ ID NO:3881
SEQUENCE LENGTH:383
SEQUENCE TYPE:nucleic acid
TOPOLOGY:linear
CLONE:HUMGS04605
SEQUENCE DESCRIPTION:
GATCTTAAGG TAATTACAAA AGGGAAATTC CAAGAATGCA TAACACAATG ACAACATGGT 60
GAGAAAAGTG CAGTACTACT TCCAAGGTAG CTAGTGTAAA GGACACTTGA GTTTTTAAAA 120
CTGTGGTTAA ATGTTTTATG TGGAATTTGA TGAGTTTGTA TATTAAATAC CACTGCTTGC 180
TGCTTTTACT AGAATCAAGT TAAGGGNCAC GTTAGAAATA GAACAGCTTA ATATCACTGG 240
CTTCAGAGCA AAATGAGCTC GGGTGACTGC AGATTATGAT GGTAAATATG GCTTTAATTA 300
CAAACATGNG GNATGATTCA CTGAAGGTGA AAAGNTAGGG CGGNTGTGNT ATTGAAAAAA 360
AACCCANCAT TTTTGGCATT GAN 383

SEQ ID NO:3882
SEQUENCE LENGTH:383
SEQUENCE TYPE:nucleic acid
TOPOLOGY:linear
CLONE:HUMGS04606
SEQUENCE DESCRIPTION:
GATCTATTTC TCAGTAATGA GTGTGTNCTC ATGCCTGTTT CAATATTGGG TTTTGGAGCA 60
TTATTGTACC AGGCTGTTGA TTGCATACTG TAGAAGATTT GATGTTCANA CTGGTTCTTC 120
TTCATATACT ATGTTTNGTC TCAGTTNCTA TGCTATTGGA TGATACTTCA TTTGTTGTTC 180
TGGTGCTGAT GAAAAGGGNA ACCTGCTCTC CTCTATTTAG ACCTTGACAC TTATTACTGC 240
TCAGTTGTTN CTGGAGCTGA AGNAATGAAA NGTCTGGAAG GAACAGCTAT CAGATGTGGC 300
CATTTGGATT GTNACAAATG AGGAACCCTG TGTACTGGGA CAGTTGCCAN AATAATATAT 360
GTGGCCTNCT ATAATGTGCA TTN 383

SEQ ID NO:3883
SEQUENCE LENGTH:380
SEQUENCE TYPE:nucleic acid
TOPOLOGY:linear
CLONE:HUMGS04607
SEQUENCE DESCRIPTION:
GATCTGAAGC CAGAAGGGCT GAGTGTATTG TNAACTTATT CTTGCATGTN GCTGTCTGGG 60
AATGGACCAC ACTACAGCAG GTAGTNCTGG GGGCGATACT GCCGAAAGNC CGANCACATG 120
TATTTTGGCT GCAATTGAGG NNCTTGGGAT GCTATTAATT TTGTATTTCA GCAACTGCCC 180
CTTCTCCTAT CCCAAAGCAC CAATTACTNC CCTCTGCCTC AGNGGTACCA GTATAAGATG 240
ACATTCCAAA GACTGGAGGC AACTCAGCCT GAGTTAATTC ACAAAATTAT GCCATGCTGG 300
GGCTTGAGCT TGAGCTTGGG CTTAGGCTTG GGCTCAGCTT TTGACCCTCA GGCATCTCCT 360
TTNCCTTNCT GTCTTNCTTN 380

SEQ ID NO:3884
SEQUENCE LENGTH:377
SEQUENCE TYPE:nucleic acid

TOPOLOGY:linear
CLONE:HUMGS04608
SEQUENCE DESCRIPTION:
GATCTGTTCA CAAGCTGGTG TTCAGGAACC AATGTGTGAC TGGTCCATGC CCACTTCTGA 60
TTATATGGAC TCAGAGACAA TGTACATGGG AGTGCTTTGC CAGTGGTAAA GTTTCTACAA 120
ATGGGAGGTA ATTGCATGGC AGGTGTAAAG TTATTTGGAC CATTGAGGCC AGAAAAAATG 180
TAGATTTAAA ATATACTGAT ACTGAAAGCA GANCTGTTTG ANCTAATTGC TAATTTTAAG 240
GNATTCAGAC ATTCATCCAA AGGNTTTAGG CCAAATGTTG CCTAAATTGT TTCTCTATTG 300
CATATGAAAG TGATTTCNGC GGCTTAACNG TTCTCTNCTN TTTATGTGCA TGGCCATAGC 360
TGGTTGTNTT CACATGN 377

SEQ ID NO:3885
SEQUENCE LENGTH:367
SEQUENCE TYPE:nucleic acid
TOPOLOGY:linear
CLONE:HUMGS04609
SEQUENCE DESCRIPTION:
GATCAAGGTG AAGCTCACAG TGTCAGGCTT TCTGGGAGAG CTCACCTCCA GTGAAGTCGC 60
CACTGAGGTC CCATTCCGCC TCATGCACCC TCAGCCTGAG GACCCAGCTA AGGAAAGTTA 120
TCAGGATGCA AATTTAGTTT TTGAGGAGTT TGCTCGCCAT AATCTGAAAG ATGCAGGAGA 180
AGCTGAGGAG GGGAAGAGAG ACAAGAATGA CATTGATGAG TGAAGATGTC GGCTCAGGAT 240
GCCGGAAAAT GACCTGTAGT TACCAGTGCA ACGGGCAAAG CCCCACAGTT TAGTCCTTTG 300
GAGTTATGCT GCGTATGAAA GGATGAGTCT TCTTCCGAGA AATAAAGCTT GTTTGTTCTC 360
CCTGAAA 367

SEQ ID NO:3886
SEQUENCE LENGTH:360
SEQUENCE TYPE:nucleic acid
TOPOLOGY:linear
CLONE:HUMGS04610
SEQUENCE DESCRIPTION:
GATCAAAGTA GCTTACTATA TCTAAACTGT AAAACAATAT AGTTTCTCCT GAACACCTGC 60
TTTCCTTCTG GGAGTCTGGA ATTTTGGTAT GTGCCAGGCA GAGACTACCT TTGTGACCAG 120
CTCCCAGTAA AAACCCCAGG CACTCAGTCT CTAACAAGCT TTTCTGGTTG ACAGTGTTTC 180
ACAAGTGCTG TTACAACTGG TTGCTGGGAG AATTAAGCTC ATCCTCTGTG ATTCCACTGG 240
CGGAGGATTC TTGGAAGCTT GCACTTAGTT TCCCCTNGAC TTCACCCCAT GTGTCTTTTT 300
TCCTTTGCTG ATTTTGTTTT GTATCCTTTC ACTGTAATAA ATCATGGCCG TGAGCAGAAA 360

SEQ ID NO:3887
SEQUENCE LENGTH:359
SEQUENCE TYPE:nucleic acid
TOPOLOGY:linear
CLONE:HUMGS04611
SEQUENCE DESCRIPTION:
GATCGCAAGT GACTGCTTCC GCTTCCAGGA CTGGGCCAGC TAGTGGGGGT GGCAGAGGTC 60

TCTTTGCTTC ATTCAGCCCT AGCTCTGTAG AGAAATGCAA ACCTCGACTC TCAAGGATGT 120
GAGGAACACA AGTTCATTTC TGTTGTTGCG GAGACACTGC AGACTCCACT GTGCCGAGGT 180
TGAACTCTTN NTTGTTGCTC AAGTTCTAGG AGTCCCTTTC CTGAATATAT ACTTGTTTGT 240
CATAGTTTCC TTNNCAAAGT AGTAAACTTT TCTATTNNTC TACTTGCCCA GTAGAGACTC 300
TTGATTCTGG AANATTCTNG NCAAAATAAT TTTAATAATA CACATGGTTG CTTCTTAAA 359

SEQ ID NO:3888
SEQUENCE LENGTH:359
SEQUENCE TYPE:nucleic acid
TOPOLOGY:linear
CLONE:HUMGS04612
SEQUENCE DESCRIPTION:
GATCACTTGA GGCCAGGAGT TCAAGACCAG CCTGGGCAAC ATAGTGAGAA TGCATCTCTA 60
CAAAAAATAA AAAAAATTAG CTAGGCATAG TACCTGAGGC CAGGAGGTCC AGGCCGCAAT 120
GAGATGTGTT TGTGCCATTG CACACCAGTC TGGGTGACAG AGAAAGACCC TGTCTCANGG 180
GCANNTTAAA TAAAATATCT TCATATATAA ATTAAGCTAA TTAAAANTAT TTTTCCCTGT 240
ATTTNTTTAN TATTTTCTAA TCTGAACCCA TATNCAGCTG ACTANTTCAT TCTTAGAANT 300
GTGTTATCTG TAATCTNTCC TAAACAGTGT AGCCCACAAG GNTCCATGGC ACTTTTTAN 359

SEQ ID NO:3889
SEQUENCE LENGTH:359
SEQUENCE TYPE:nucleic acid
TOPOLOGY:linear
CLONE:HUMGS04613
SEQUENCE DESCRIPTION:
GATCTAAAGG GGACACTGTA CTCAAGCTTT TNACCTCATG CCTTGTGTAG TAAAAAAGGA 60
TTTGGGGGTT TTGTTTGGTT CCTGAGAGGG TTGTGTTTTG TTTTTGTTTC CTTTTGTTTA 120
TGTTTTGGCC TTTCCTCTTT GTCTTTCCAT GTAGACCAGA TATTTGAAAG GGCAGACGAT 180
GGCTAGAGGT GTAATGTGCA GCTTGTTTAT ACGGTATTTT GGGAAACTTA CCTTGGATGG 240
GAAATCGAAT CGTGGATTCA CCAGGCCGGT GCTGGCACAC TCACCCTCGC CCTTTCCCTC 300
CGGTTCAGTA CCTATTGTTT CTCCTTTCAA ATATGTGATT GTACTAGCTC TTTCCATAN 359

SEQ ID NO:3890
SEQUENCE LENGTH:358
SEQUENCE TYPE:nucleic acid
TOPOLOGY:linear
CLONE:HUMGS04614
SEQUENCE DESCRIPTION:
GATCTGATGG GCTCTAACAG TGCTTACTTG CAGCCTTGTG TCCACCACCA ACTTCTCATC 60
ATGTTTCTCT CCGTTGGCCG TTGGGTTTCC AACTCTTCAA NCTTCAGGGT CTGGGNCAGG 120
GAGTGGGCCC ACCATTTGTG GGGAAAGTAT CATTCCTCCA CCTCAGGCTT GGGTAGATTT 180
GGAAAAANAC AAGGTNAGGA TACGCCTGTT TGAGCTTTGG GGNATGAACT TTGCTTTTTA 240
TATTTANCTA NGGNTACTTT TATATGATGG GTGCTTTGAG TGTNATTNAG CAGGNTCTCT 300
TNGTTTCCCG AGGGNCCTGC TTTTGNAAGG TGACCNNGGT TACTTAAGCT AAGGNTTN 358

SEQ ID NO:3891
SEQUENCE LENGTH:356
SEQUENCE TYPE:nucleic acid
TOPOLOGY:linear
CLONE:HUMGS04615
SEQUENCE DESCRIPTION:
GATCAACCCA CATTAGAGTG TCTAAGGACT CCTGAGAATT CCTGTTACAG TAAACAAAAC 60
TAACGTAATC TACCATTTCC TACACTATTT GAGCATGGAA ATCATAGTCC CCACTCTGTG 120
AAAACTTAAC GCTTTTTGGA AGACATTTCT GTAGCATGTC AGTTTGGAGA AATGNNTGNG 180
CTACGCCTTG ATGAAAGAAC CGTGTTGGTG CTGCTAAGTT TAGCCATTAT GGTTTTNCCT 240
NTCTCTCTCT TAAGCCTTAT TCTTCAACTA AAAGATGAGG ATTAAGAGCA AGANGTTGGG 300
GGNNGATGTG AAAATAATTN TATGNGGTTG TCTAAAATAA AGAGTAGTTT CTTAAA 356

SEQ ID NO:3892
SEQUENCE LENGTH:355
SEQUENCE TYPE:nucleic acid
TOPOLOGY:linear
CLONE:HUMGS04616
SEQUENCE DESCRIPTION:
GATCTACAAA TGTACGTTGT TACAGGGCTG CTTCCTAAAG ATTTTTTTTA CCTCAGGTTT 60
CTCTTAATAT AGTTCTCCAG TCACTGACCT TGAATTGACT TACATAAATT ACTGCCAATG 120
TTTAAATTGC CCTTATGTTT ATATTTATTA TGTCAAGCCA ATTCGTACAT ACAATTTGGA 180
ATCAAATGTC ATAAGAATTT ATTATATAAA TTTNTCAAGA ATAAAAATGC CTCTCCAGCC 240
TTAAGTATTT ACATGCTCCC AGGTCATTGT CAGTTTATGG TATTATGTTG TTTTATTTAA 300
AGCATTGAAT TGATAGAAAA AATTGCTCTG TAATAAAAAT CTACTTTCAC ATAAA 355

SEQ ID NO:3893
SEQUENCE LENGTH:351
SEQUENCE TYPE:nucleic acid
TOPOLOGY:linear
CLONE:HUMGS04617
SEQUENCE DESCRIPTION:
GATCTCATTC TTNTTATGGC TGCATAGTAT TCCATGGTGT CTATGTACCA CATTTNCTTN 60
ATCCGGTCCA CCACTGGTGG CATTTAGGTT GATTCATGTC TTTGCTATTG TGAATGGTGC 120
TGCGATAAAC ATGCATCCAT GTGTCTTTAC AGCAGAACGA TTTATATTCC TTTGGGTATT 180
TTTTTTGAGT ATAGTAATAG GNNNNNNGGG TGGAGTGGCA GTTCTGTTTT AAGTTCTCTG 240
GGAAATCACC AAACTGCTTT CCACAGCAGA TGAACTAGTT TACACTCCCA CCAGCAGTGT 300
ATAAGTGTTC CCTTTTCTCT GCAACCTCAC CAGCATCTGT TATTTTTAGA N 351

SEQ ID NO:3894
SEQUENCE LENGTH:350
SEQUENCE TYPE:nucleic acid
TOPOLOGY:linear
CLONE:HUMGS04618
SEQUENCE DESCRIPTION:

GATCTTCTTG TAAGCACATC CAGAGTGAGA CTTTTCTATG TTTGAAAGAA GAATGTGTGT 60
ACATTTCAAG AATTTGGGTT TTTTGGAGGG AGGAGGAACT GTTTACGTTT TTCCTCCACA 120
CGTTTGATTT TTGACACATA CACCCCTAAT TCCTCAACAG CAGACCTCCT GCAGCCACCA 180
GGGGCCAGCT CTGTGTAGGA ACCAGATGGT CTTTTTCCAA GCNGCATCTT CAGCTGCCAG 240
CTAAACTCCA ACCCCAGNCA GGGAGGGGCA GGCTCAAGTC TTNCAGAATC ACACAGGGGC 300
AACACATGCA NNNNCCGGNA CCTGTNCTGT ACNGTCTNGG TTTNTTCNTN 350

SEQ ID NO:3895
SEQUENCE LENGTH:348
SEQUENCE TYPE:nucleic acid
TOPOLOGY:linear
CLONE:HUMGS04619
SEQUENCE DESCRIPTION:
GATCCATAGG AGGGTAAAAT GTCCGTTTTC TTCATTAAAA CTTCTCAGCT CAAATTTATA 60
CTAAAGCATA TCCAGGAATT AAAAAAAAAG GTTTTGATGA TATTTGCTTT CTAGTTTAAT 120
AAGTTTTAAT ACTGAATAGG TTAAATTAAT ACAAAATAAC ATAAAATGCT AATATTTATT 180
GAGTATTTAC TTGTGTACCC ACTACGNTTT TAAGTAATTT ACTGGGATTA TTTTATTTTA 240
GTTTAACAAT GCAGGTAGCT ACTATAATCG TCCNGGTTTT ACACTTGAGG AAACAGGCTA 300
AGAGAGATTA ANTTAGTGGC CCAAGGTCAG AAAGCTCGTA ATTCTTGN 348

SEQ ID NO:3896
SEQUENCE LENGTH:347
SEQUENCE TYPE:nucleic acid
TOPOLOGY:linear
CLONE:HUMGS04620
SEQUENCE DESCRIPTION:
GATCCCAAAT ACTGCCATTG TGTTGTCTAC TTATGAGTTA ATTGTGTACC TGTTAGAAGA 60
CCGTACTCAG TAACAGGCCG GAAAATTGTG CTCTAGAAGA ATAAAACTGA AAAACTCTAG 120
AGAATTTTTT TTCCCCATTG ATGTTTAGAA AGTTTGAGAC TGAAACAGGA AAGGCCATAA 180
AATATCTGGT TCATATCACC TGTTGGACAT TTCCTTTTGG ATTCATGCTT TCTGGAAGGT 240
TTAAATTCAT TAACGTTAAT AGTTAATTAT AACTNTTTNT TTAACTTAAG AGGATTCAGG 300
GTTAAGCACC AACTAAATTA AATCATGCTA TTTAATTTAN GTATAAA 347

SEQ ID NO:3897
SEQUENCE LENGTH:345
SEQUENCE TYPE:nucleic acid
TOPOLOGY:linear
CLONE:HUMGS04621
SEQUENCE DESCRIPTION:
GATCTAGAAT CTNTNCTNGG NTAAGCATAT AGGATGCCAC TTCTTGCTAC CTCCACAGCA 60
ATACATCACC TGGACAGCTT GCCACTCTTT GTTGCCTTGA ACTTCTCTGA TGTTTAGGCA 120
GCCCATTCTG GTACTGAGAA CACAGTGAAT GAGCTACATC CTCCTGCTTT CTTCTTCTAT 180
TATNACTTTT ACCTGATTTC TTCTCAGGAG GAAGCTGTAC ATTTTAACAG GACAGATAGG 240
AGGAAAAGCA ACTATTTTTC TGCCGAGATA ACAATTGACA ACTTGGGGCA GTATGTGCAG 300
AGTTTTCCTG GAGTTGTATT AAAAATTATC TGATAAGCTG CCAAA 345

SEQ ID NO:3898
SEQUENCE LENGTH:342
SEQUENCE TYPE:nucleic acid
TOPOLOGY:linear
CLONE:HUMGS04623
SEQUENCE DESCRIPTION:
GATCAGTGTA CTGTTACCCG GACATACCTA TTCCTTCACA AGTTCTGGTT CTTCAGTGCT 60
GCTTACTATT TTGGTAACTG GGCCTTTCTT GGGGTATTTT TGATTGGATT AATTGTATCC 120
TGTTGTAAAG GGAAGAAATC GGTTATTGAA GGAGTAGATG AAGATTCAGA CATAAGTGAT 180
GATGAGCCCT CTGTCTATTC TGCTTGACAG CCTTCTGTCT TAAAGGTTTT ATAATGCTGA 240
CTGAATATCT GTNATGCATT TTTAAAGTAT TAAACTAACA TTAGGATTTG CTAACNAGCT 300
TTCATCAAAA ATGGGAGCAT GGCTATAAGG CCAGCGATAT TN 342

SEQ ID NO:3899
SEQUENCE LENGTH:410
SEQUENCE TYPE:nucleic acid
TOPOLOGY:linear
CLONE:HUMGS04624
SEQUENCE DESCRIPTION:
GATCTGGTGC CCCTCTTCTC CTGGATTCAC ATCCCCACCC AGGGCCCGCT TTTACTAAGT 60
GTTCTGCCCT AGATTGGTTC AAGGAGGTCA TCCAACTGAC TTTATCAAGT GGAATTGGGA 120
TATATTTGAT ATACTTCTGC CTAACAACAT GGAAAAGGGT TTTNTTTTCC CTGCAAGCTA 180
CATCCTACTG CTTTGAACTT CCAAGTATGT CTAGTCACCT TTTAAAATGT AAACATTTTC 240
AGAAAAAATGA GGATTGCCTT CCTTGTATGC GCTTTTTACC TTGACTACCT GAATTGCAAG 300
GGNTTTTNAT ATATTCAATA TGTTACAAAG TCAGCANCCT CTCCTGATTG GTTCAATNAT 360
TGAATGTGCT GGNAAATTNA AGGTCGGTTN NANTTTAAAN CAAGGNTNCN 410

SEQ ID NO:3900
SEQUENCE LENGTH:338
SEQUENCE TYPE:nucleic acid
TOPOLOGY:linear
CLONE:HUMGS04625
SEQUENCE DESCRIPTION:
GATCAAGATA TAGAGCATCT CCATTTATAA AGTTCCTTCA TGCTCCTTCC CAGCCAGCAA 60
TTCTNCTTAC TGTCCTGCTG ACTCCTGTTT TCACCTTCAT CACCATCAGC TCATTTNGCT 120
TGTNCTTGAC TCATAAACAG AATCATATAG TAGGTGGTTC GTTCAATGTA GGCAGTCTGT 180
TTTNGCTTTA ATCATTACAT GAGTATACAT CCATATNGTG TGAAGCAGTA GTTCTTTNGC 240
ATTGCTGTGT AATATTCCAT TGTATAATTG TATAAATATA TGTGGTATAA ATGTAAGTAT 300
AGCACANTTG ATTCCTTATN GGGCCACTTG GTTTATTN 338

SEQ ID NO:3901
SEQUENCE LENGTH:238
SEQUENCE TYPE:nucleic acid
TOPOLOGY:linear

CLONE:HUMGS04626
SEQUENCE DESCRIPTION:
GATCCACTGG TAAAGGGCAT CCCAGAGGNC AAAAATCCCT TCAAGGAGCT CAAAGGAGGC 60
TGTGTGATTT CATAATACAA ACAAAAAGAA AAAAAATTAN ACAAATTCTT GGAAATATCT 120
CAAATGTTAA TAACAATATG ANTTTTTCTC ATGCATACTA TTACTACTAA GCATGTACGT 180
GANTTTTTAA ATGTATAGAT GTAAACTTTT ANTAAANNTT GGGGTGTGGT AACCCAAA 238

SEQ ID NO:3902
SEQUENCE LENGTH:331
SEQUENCE TYPE:nucleic acid
TOPOLOGY:linear
CLONE:HUMGS04627
SEQUENCE DESCRIPTION:
GATCTCTATA AGTTATTAAT TTAACCTGAA ACCAAAGTGG TCTCCATTTA AAGTTACTTA 60
ATCCCTTTGT ACCACCTATT TCTAGTTAAA TATATGTNGC TANNCAAATA GGTAAAGTGC 120
TTCCTTGCCA TGATGGTAAT GGATTGGAAC TATGAAGGCT CTCAGTGTAT TGGCTTCTGT 180
AAAGATGAGG CGTCTCCTCA GAANCCCNAA CTTTTCACAT TTCTGCTTAC TAGACCTGGG 240
TTGATGTACA TGGTAAGTCT CAAACAGATG CAAGCTATGT GCAAAANGGA ACTTTAGCCA 300
AATGGAAATA GCTGGATGCT TTGNGAATTG N 331

SEQ ID NO:3903
SEQUENCE LENGTH:67
SEQUENCE TYPE:nucleic acid
TOPOLOGY:linear
CLONE:HUMGS04628
SEQUENCE DESCRIPTION:
GATCCAAACT GTGTTGTTCT TAAATCAAAA ATTGGNTAAT TTTTAATATN TATGTATTAA 60
TCACAAA 67

SEQ ID NO:3904
SEQUENCE LENGTH:327
SEQUENCE TYPE:nucleic acid
TOPOLOGY:linear
CLONE:HUMGS04629
SEQUENCE DESCRIPTION:
GATCTGCCGG AGTCTTGAAA TTCAGCTGAT TGGAAGTGGT TTCTTTGACT TTCAAGGTCA 60
TTCCCTATCA GTTACTACTA AGAGTTCTCT TACTGTCAGA ATCTCTCTTG CAGTACAGCT 120
CAAAATAGTG GATGCATTTC AAACTTGACC ACCTTTTCCT ACCAGCTAGT TAGAAGTCAT 180
CAATATTTCT CTACATTTNG TTTATCTGTA AGTCCTTTAA ATCTATTTTN GCTAGGCATT 240
CATTATGATT AATAGTAGAC TTTTAAGACA ACATTTNTGT CACTCCCCAC CTCCTCATAT 300
TTNATAAAGG AGATATTTAC CTTGAAA 327

SEQ ID NO:3905
SEQUENCE LENGTH:326
SEQUENCE TYPE:nucleic acid

TOPOLOGY:linear
CLONE:HUMGS04630
SEQUENCE DESCRIPTION:
GATCAAAGAC CAAAACATTT TCTTAAATAT ATTTTATGTA ATATTTTATT TGTATACAGT 60
GTTGTTGATG AAATATTTAA CTAGAGCATG ATATTTTAAA TGTTAAGGTG TAACATATGT 120
TAAATAAAAC TGTTATTTTN GAATTTNAAA ATTNGTTTTT NGGGGGTATG ANCTACTAGA 180
GTTTAAAATT CTGCCAAACT ATTACTTATA TGTNCTATTG TGTAACATAC TTNCTNGAAA 240
TATTTNGGTT TATAGAATTG ANGGTTCTTA TCAGATGGGA TACTGGGGAC TATAAACAAT 300
GGAAATAAAG CCACTGTATT TNTAAA 326

SEQ ID NO:3906
SEQUENCE LENGTH:325
SEQUENCE TYPE:nucleic acid
TOPOLOGY:linear
CLONE:HUMGS04631
SEQUENCE DESCRIPTION:
GATCTAATTA AGCTAAATTT TGGTGCACAG CAGGTTGTAC ATGATTTCAT GTTTATGTAG 60
CAAAATGCAT TGAAATGTAT TAGTGTTGAA ACATACATGT AAAGGTAGTT GTGGAAATTG 120
TTATATTTNC CTTGTGTGTC ATTTATTTAG AGCATAAAGG TTTTTTTTAT TCAGCCNNNC 180
TGAATAAGGC TAAATGAATA CTGTAATTGA AAATATATTT NAAATCTTTG TCATGAGTTT 240
TTTTAAAAAA ANCTATTGCA AATTGTATCA TTCCTGATTA CACAGAACTT TGTGGGTGGT 300
TTTAANTAAN TNTNNTACTT CTAAA 325

SEQ ID NO:3907
SEQUENCE LENGTH:324
SEQUENCE TYPE:nucleic acid
TOPOLOGY:linear
CLONE:HUMGS04632
SEQUENCE DESCRIPTION:
GATCTGGCCC GAGCTCTCCT CAGACCTCCC CAGACCTGTC TTCTCATCAG CCACCGCTGT 60
TTTCTTTTGC CGAACGATGG TTGCATCTCT CGGACACAGT GTACCCCCTC CCAAGCCCTC 120
CCTGAGCCGT TCTCCTGAGT CCCTGAGGGC AGACACAGCT CCAGGGACCC AGGCCGGCTG 180
CCACACCCNC GACACTNCTG NTGTCCACTG GCCTTAGCTC CTGCAAGTTC CCCAGTGTCC 240
GCACCTGTTC CNTTCCCTGA CCTTACCTTN CTGGGGTCTG TGACTGACTG GAGATAAAAN 300
TAAAAGCAAT TGTGACANCT GAAA 324

SEQ ID NO:3908
SEQUENCE LENGTH:319
SEQUENCE TYPE:nucleic acid
TOPOLOGY:linear
CLONE:HUMGS04633
SEQUENCE DESCRIPTION:
GATCAAATAC CGAAATTGAA CTTTCTAAAA CCTACACATA GCATGCGTTT AAATTCTATT 60
ATATAAAGCC CCAAGTGCTT TACCTTCTAA AACATGCTTC TCTATAGGTG CAAAGTTATA 120
AGATATGTAG AAGAAAACAT CCATCAGAAT CTNCTGGAGA NTATGGTCAG AGTCTTAAAC 180

AGGCCCAGTG CTGAGTNCTT TGTCCACTTA GTCACACATC TTTTGTGGGA ATGGGAAAGG 240
AAGGAGACAG CCAAACTCTG ACAANGGCTT TTCCTTATAG ATTAACCCAA AGCCTAGGTG 300
AGGGTTGATA TATTAATGN 319

SEQ ID NO:3909
SEQUENCE LENGTH:317
SEQUENCE TYPE:nucleic acid
TOPOLOGY:linear
CLONE:HUMGS04634
SEQUENCE DESCRIPTION:
GATCTGAGCA AAATGTGAAC TCAGTATGTT TACTATTGCT CTTACTTGAA AACTTTTTTT 60
CAAAAAAAGC ACAAATTAAA GTAGTAAATT CATATCCATA GATAGTTCAT TCATTCAACA 120
AATATTTACC AAGTTCCTAA TATAAGTGAA GGNCCACTTC TCAGATTACT AAGTCATTTG 180
TATGAATATG TGTGGCAGTG AAGAGAACAG GTCTTTCAAA AAGCATTTGA TTATCTTTAT 240
TTNTTTTAAA TACACTCTCT NATNTTNCTA CTTGTTTTTT TGTTAATCAT AGCAGGATAT 300
GACAACTCTT ATTTGAN 317

SEQ ID NO:3910
SEQUENCE LENGTH:317
SEQUENCE TYPE:nucleic acid
TOPOLOGY:linear
CLONE:HUMGS04635
SEQUENCE DESCRIPTION:
GATCTACTCA GATGTCATNA TACTCCATAC CTGCTTTTCC CATGGCCGCC CTACGGAAAA 60
TCCCATCCAC AGAGGCCAGG GCTACCCAAG NCCCTCCAGG TGAGCTGGGC CTTTCCTTTA 120
TGAACCTCCA TCCTCCCAGC CAGCTACAGT AGGGCCTCCT CACCCCGTAC CCCACAGCTA 180
GACAGTGTCA GCACTCATCT CCTCCTCCCA CATTTNTGGA GCTTTTTTTT TCCTTCCNCA 240
TTGACCTTTG TGGTCTTCTG TGATTATTTA TGCTGCCTCC NAAGGATAGA ATTGAAATAA 300
AATGTTTTCA ACTTAAA 317

SEQ ID NO:3911
SEQUENCE LENGTH:316
SEQUENCE TYPE:nucleic acid
TOPOLOGY:linear
CLONE:HUMGS04636
SEQUENCE DESCRIPTION:
GATCAAAGGT TCTTCATTCA AAAAGAAGCA ATATCCTTTT GCCTGATATG GGGTGGGAAG 60
GGTAGGTGGC GATGATATTT CAAATGAATT AGAAACCACC TCCCATTTGA TGATAGCTGG 120
AGAAATGGAC TGACCTAGAA GTTACAGGAC CAGAAACTAC AGTCACCACT AACCTTTCTA 180
CGTGGCACCC AGCCCCACTT GGAGACATTA TTTTTCCATC AAACACAACC GTACTGCACA 240
GACATCTTCA CTGTCTGTTG CAAAGATTGG AATACGTAGA TTTCTGTTCA AAATTAAATA 300
ATATTTTAAA TACAAA 316

SEQ ID NO:3912
SEQUENCE LENGTH:316

SEQUENCE TYPE:nucleic acid
TOPOLOGY:linear
CLONE:HUMGS04637
SEQUENCE DESCRIPTION:
GATCCACTGG TAAAGGGCAT CCCAGAGGAC AAAAATCCCT TCAAGGAGCT CAAAGGAGGC 60
TGTGTGATTT CATAATACAA ACAAAAAGAA AAAAANTTAA ACAAATTCTT GGAAATATCT 120
CAAATGTTAA TAACANTATG ATTTTTNTC ATGCATACTA TTACTACTAA GCATGTACGT 180
GANTTTTTAA ATGTATAGNT GTAAACTTTT AATAAAANTN GGGGTGTGGT AACCCNGNGG 240
GNCTATGTTT TNNTTAACAT AGCTGGCACA GGGTTTAACA CATAATNGCC ATAANTATTG 300
CTTAAGGTNC TTTAAA 316

SEQ ID NO:3913
SEQUENCE LENGTH:313
SEQUENCE TYPE:nucleic acid
TOPOLOGY:linear
CLONE:HUMGS04638
SEQUENCE DESCRIPTION:
GATCCACCTG CCTTGGCCTC CCAAAGTGCT GGGATTACAG GTGTGAGCCA CTGTGCCCGG 60
CCAAACCATT TTTGTTTAAT TTCATTTCTA CATGGTCGTC AAACGTGCCT ATCCAATGAA 120
GCCTTCATAG AAGGCCCAAG AAGACAGAGT TTGGGGAGCT TCTGGATAGC GGANATGTGG 180
AGGTTCCAGG AGGGCAGGGT GCCCAGGAAG GGCATGGAAG CTCCGTGTCC CTTCCCCATA 240
CCTTGCCCTA TGCATTTCTT CATCTGTATC TATTGTGATA TCCTTNACAA TTAAACTGGT 300
AAACATANAT AAA 313

SEQ ID NO:3914
SEQUENCE LENGTH:312
SEQUENCE TYPE:nucleic acid
TOPOLOGY:linear
CLONE:HUMGS04639
SEQUENCE DESCRIPTION:
GATCACCAGC TCACGTCATG TTGCCTTCTC TTTTCTTTGT GTGTGTGTTT ATTTAAGTNA 60
TTTTTCTTCC TCCTCTCCCT TTTCTTTTTG GCCCTCCCTC CCTCCCTCTN CTGCCATGTA 120
ACTGGAGGAT GTGCTATGAG TTTGCAAACA GCTGGACTGT CAGGCTGCTT TTTTTCCAGA 180
TGTTCCTCCT CTGCCTCCCC TTCCCCTCCT CTCCCCTCCT TTTCCTTCCT TCCTTCCTTT 240
CCTTGGAGCA CTGAGCACCA TTTGGAAGCT TGAGAGAAAC CAAAATTAAA GAGAGAAAGA 300
GAGAGCGTGA AA 312

SEQ ID NO:3915
SEQUENCE LENGTH:311
SEQUENCE TYPE:nucleic acid
TOPOLOGY:linear
CLONE:HUMGS04640
SEQUENCE DESCRIPTION:
GATCTAGAGG AAGTACAGCC ACCCACTGAC ATCTGANTTT ATATACCTNT TGAGTTTTGA 60
GTGCACCCAA ACACTCGATA AACCAGGTGA AGAAATTTAG CTTCCATGTT CTACTTCAGC 120

TAAAACAGCT ACATACAACC TAGTACCCTT GANGTCAGAC AGACATTTCA GTTGCTTACC 180
TCCAGTACTG AGCCTTGCTT TGGGAAACTA AAAGATTTAG ACCAAGTCAC TGCCAGTTTT 240
NGCCTTNGTN GCATTTNGTA CAGTTTTTAT ATNTNGGATA TCTTGTAAAT AAAGACAGCC 300
AGCTTTTCAA A 311

SEQ ID NO:3916
SEQUENCE LENGTH:308
SEQUENCE TYPE:nucleic acid
TOPOLOGY:linear
CLONE:HUMGS04641
SEQUENCE DESCRIPTION:
GATCAAATCT AAAATAAGTA TAATGCATTG TAATTCCAGA GATAAATCCT AGACCCTTCT 60
TGGCCTCCTT CTNACATAAT TCTAATCCTA CAGTCTCAGA GATGCTGTTG TATCCTGCCC 120
CCCAACCCCA TGATAGTNAT AGTGGTTTTT GCCTTGAAGG AATTGCTTTG TATTTAGCTT 180
TTCCCCCTCT AGATTTCTAG TTCCTTTTCA GTATTGGATT GGATTTGAGA TTTGATTAAC 240
CTAGTACTCA GGTTCAGATG CTCGCCTCTT TGCAATTTTA ACACTCATTC GACAATAAAG 300
TCAGTNGN 308

SEQ ID NO:3917
SEQUENCE LENGTH:306
SEQUENCE TYPE:nucleic acid
TOPOLOGY:linear
CLONE:HUMGS04642
SEQUENCE DESCRIPTION:
GATCTAATGC ATCCTATATC CAGTAAGTAG AATTATCTCT TCATCTGGGA CCTGGAAATC 60
CTGAAATAAA AAAGGATAAT GCAATAAACA CAGTTGCAGG AAAGTATGTT AGCTATATAC 120
TATGAAGTAC TCTTAGTTTA CTTATGTTGA ATGGCTTAGC TATTAATACT CAAATTGAGT 180
TAAAATGAAA ATTCCTCCTT AAAAAATCAA ACGTAATATG TATTACATTT CATGGTACAT 240
TAGTAGTTCT TTGTATATTG AATAAATACT AAATCACCTA GGTGTCTATG TTCTATCACA 300
TCTAAA 306

SEQ ID NO:3918
SEQUENCE LENGTH:306
SEQUENCE TYPE:nucleic acid
TOPOLOGY:linear
CLONE:HUMGS04643
SEQUENCE DESCRIPTION:
GATCAGCTGC AGCAGGNTGG GTCCCTCTGC ATCTGTCCAT CACTGCGACT GATGGCAATG 60
ACTGAGTATA ACACCTTTCT TCATGGTCAA CTCTACCCAG GAACCACGTA GGGAAGGAAA 120
TTCTGGAAAA CAGCTCCCAG CCTTAGCCAA CTTGACATAT CATAATCCAA CAGANCTCAA 180
CCTCTACCAA CTTGGAAATC ATGTACATTT GTCAGGACTG TATTTAATTT CCACATAGAG 240
AGAAAGCAAA ATTATACTTT TGACCAAACT AATAGANTTA TCCATTAAAC ANACAAAACT 300
GTGAAA 306

SEQ ID NO:3919

SEQUENCE LENGTH:306
SEQUENCE TYPE:nucleic acid
TOPOLOGY:linear
CLONE:HUMGS04644
SEQUENCE DESCRIPTION:
GATCCTCCCA GCTCTGTCTC CCAAAGTTCT GGGATTACAG GCATGAGCTA CTNTGCACAA 60
GCTAAGAATA TGTGCTTTTA AATCCCATAA GGAAAAAAAT ATTTTTGGTA AATAAAATTA 120
AAGCATAAAT ATCATGATTT ACCTGACGTT TACCGTAAGC AACATAAAAG ATATGTGATA 180
CTGAATGACT AAAANGTAGT TTTGGTTAAA GAGAGTTCTA GTTTCCATGA GGNCTCTGAC 240
AATGCTGCAT TTTATTTTNC TTTTTAATGT GCACATTGGT ACANGTTTAA CTACTCTACT 300
CCTGTN 306

SEQ ID NO:3920
SEQUENCE LENGTH:304
SEQUENCE TYPE:nucleic acid
TOPOLOGY:linear
CLONE:HUMGS04645
SEQUENCE DESCRIPTION:
GATCCTGTTA TGAAAAGCCC TAAAAAATAG GGTATAGAGT TTGGGCATAT TTCAGAGGTA 60
ATAAGAAAGC ATTCTGCATT TTTAAAGTAA GGTTTAAAAA TGTTTCTAAT TAGNGATACA 120
GCAAAGTTGG TGTGAGAGGA TAAAGCTGAT ATACATAATC TGAGGAGTTC ATGAAAATTC 180
ATGTAAANCT GTAGTATGAA TTGGCCTAGG NGCAGCAGCT NAGGGCATAG NGACTTTAAG 240
ACTTTTCCAA GAGAGTGNAG GCTGGNGATA AATTGTGACT AAAATATATT TTGTGTTTGN 300
GAAA 304

SEQ ID NO:3921
SEQUENCE LENGTH:301
SEQUENCE TYPE:nucleic acid
TOPOLOGY:linear
CLONE:HUMGS04646
SEQUENCE DESCRIPTION:
GATCAAGTAA TTCTTTCACT AGGTTGGGTT TGGGGAGGGG GGAAAAGAGG GGCTTTTCCT 60
AGGAGAACGA TAAGAAATGG AAAGACTCCT TGAAGTGTTG CAAGGGAACC TCCTAGCACT 120
GTGAAAGTCA GAATCGCCTC AGCATTTCCA TGACGCACAT TATGCAAATC TNTTTAGCAC 180
TATTTTAAGT TTGAAAACTT TAACAATGAA GGGGAAGGGG AAGNTTTCCA CCAACTGANT 240
CATTTGTGCA CGTGTATAGC TCAAAGNGCT TAGACTTCAA ATATATCTGG TGAATGACAA 300
A 301

SEQ ID NO:3922
SEQUENCE LENGTH:301
SEQUENCE TYPE:nucleic acid
TOPOLOGY:linear
CLONE:HUMGS04647
SEQUENCE DESCRIPTION:
GATCTGCCTG CCTCAGCCTC CCAAAGTGCT GAGATTAGAG ACGTAAGCCA CCATGCGTGG 60

CCAATAAGAC TGACTTTTAA TTTTCCTTTC TTGTACTGTT CTTGTCTTGT TTTGGTATCA 120
AGGTTATATT AGCCTCATAA AAGGAGTTAA GAAGTGNTCG CTCTTTCTAT TTTCTGAAGG 180
AGTCTGCTAA GTATGAGAAC TCTCTATTTC ATGAATGATT GATAAAACTC TCTTGGAAAC 240
TGTCTAGACC TTATGGAAAT ATTTCAGTAA TAGTTACAAA GCTATTCAAG TTGTCTGGTT 300
N 301

SEQ ID NO:3923
SEQUENCE LENGTH:299
SEQUENCE TYPE:nucleic acid
TOPOLOGY:linear
CLONE:HUMGS04648
SEQUENCE DESCRIPTION:
GATCTCAGTT GGATAGTTGT ACCTCAAAAA AGTAGGTGAG AACTGGCTTC CTCATTGTCC 60
CAGGATGTCA AATACTAATG ACATAAGAGC TCACTTACGT GCCAAAATTC ACTTTTATAC 120
TAGTTTTTCA GTGCTTTAAT ATTTGTAACT NAAATTTNAA AACTCGTATT TACAAACACT 180
ACTGTAACTN CAGTGAAACT GAATTGTGCG ATTGANGCTT TTTGCTTATC ATAGTATNTA 240
TTACACTACT TANTTCAGTA AATATATAAA NGTAGCCTNC AATTGANGTG TNTATTGAN 299

SEQ ID NO:3924
SEQUENCE LENGTH:293
SEQUENCE TYPE:nucleic acid
TOPOLOGY:linear
CLONE:HUMGS04649
SEQUENCE DESCRIPTION:
GATCAGAAAG GTGAAATGTT TCGAATTTAA AATGTTTAAA GCATGTTTGG TTTTATTATT 60
TTTACATAAT TGTTTACCAC TAGTTTTTCC ACTAGCTTTT TATTATATAT GTTTAATTAT 120
GTAATTGTTA TTCACTAGCT TTTATTATAT AATTCCTTTT AAATAATACT ACTATTCATC 180
AACTCTTGTG GCATAAGAAT TGCAGTTTTT TCTACCAAAC TTTTACTTCA TCTATGAGTC 240
GTGTTAGAAA TAGTCATTGA AAAAATATAC AGTAAAATAT CTAGCAAAAT AAA 293

SEQ ID NO:3925
SEQUENCE LENGTH:293
SEQUENCE TYPE:nucleic acid
TOPOLOGY:linear
CLONE:HUMGS04650
SEQUENCE DESCRIPTION:
GATCTGGTGA AGGCCTNCTG GCAGCATCTT CTCAGTAAGA CCTCATGTGG CAGAAGGAAA 60
GGGAGCTATC TGGAATCTCC CTTATAAGGG CCCNNNNGCC ATTCATAAGG GCTGCACCCT 120
CATGACCTAA TCACCTTCCA AAGGCGTCAC CTCCTGGTAC TGTCACCTTG GGCGTTACGA 180
TTTCATCATA TGAATTTTGG GACGGCATAT TCAGTCCACA CCAGGAAGTT CTTGCACNTC 240
ATAGTCGAAA GTCAGTTTTG TGGCTATAAA AATCTTGGTT CCATTTTCCG CTN 293

SEQ ID NO:3926
SEQUENCE LENGTH:292
SEQUENCE TYPE:nucleic acid

TOPOLOGY:linear
CLONE:HUMGS04651
SEQUENCE DESCRIPTION:
GATCCACCCG TCGTCGCCTC CCAAAGTGCT GGAATTACAG GCGTGACATG CATGCCCGGC 60
CAACATATAA TTATTATACA ACCCAGCCAC TGCACTCTTG GGCATTTATT CTAGAGAAAC 120
AAAAACTTAT CCCCACTCAA AAGCATGTAC GTGAATGTTC TTAGCAGTTT ATTTTATAAT 180
GGTCAATATC TGGAAACAAT CCAAATGTCC TTCCATGAGT TAATGGTTAA AATAACTGTG 240
GTGCACCCAT ACCATGGAAT ACTGTTCAGC AATAAAATGG TATGAAATGA AA 292

SEQ ID NO:3927
SEQUENCE LENGTH:291
SEQUENCE TYPE:nucleic acid
TOPOLOGY:linear
CLONE:HUMGS04652
SEQUENCE DESCRIPTION:
GATCCTATCT AAAAATATGT AGTTATTAAA CATTCTGTGG ATATTTGTNA ATAAGGTAGT 60
TNCTATGGTC CGAATATCTG GGCCTGCCCC CCCAAATNAT GCTGAAACCT AATCACCAGT 120
GTGATGGTAA TAGGAGGTGG GACTGAGCTC TGATGAATGA GATTAGTGCC CTTATAAATT 180
ATGACCAAAA GAGCTCTTCA CTCCTCCTAC CATTTGAGGA TACAGTGAGA AGCCTTCATC 240
TATGAACCAC AAAGTAGCCC TCATCAGACA CTGAATCAGC CAGTGCCTTG N 291

SEQ ID NO:3928
SEQUENCE LENGTH:290
SEQUENCE TYPE:nucleic acid
TOPOLOGY:linear
CLONE:HUMGS04653
SEQUENCE DESCRIPTION:
GATCTCACAG AAGTTATTCA ATCTCTAGGT TGCTTTAGCA TCTACTTGTG TAGTGAGAAG 60
AGTTATTTAC TTATATTGTG CATGAGGCCG TTTAGATATG TTAAATGCAA AACAGAACTC 120
TTGCTAGCTA AACACAGTAG AACTTGAATT TTTTTTTTNA GAGGTTATTT AATANCAAAA 180
TCAGGTTATT TGTGACTTGG TTGAAGTAAA CGGTGTCTCA GATAAANTAA TTNGTTAAAA 240
GAGTTGCCCA NTTTATGTCT TATANCTTNA ANCAANTANN TANTAATAAN 290

SEQ ID NO:3929
SEQUENCE LENGTH:287
SEQUENCE TYPE:nucleic acid
TOPOLOGY:linear
CLONE:HUMGS04654
SEQUENCE DESCRIPTION:
GATCTGGGAT GTGAGTGTAT CAAATCTTAT CAAATCCATT GACAAATCAG TGGTGCTATA 60
CTGGAAATGG CTACTCCTTT GGATGAAGGA CCTCATTTCC CAGTTGCTTG GAACAAANGA 120
GAATTTACAT NNNNCTGTAA GAAGGGAGAG ACGGCAGTTG TTGGTAGACA CACAGNNAAG 180
GGTGCCCAAG AATAATTCTG TCTGGATTAA AGGTCCCAGG GAAGTGACGG NGCCATTTCT 240
GTACCAAGAG GTGAATATAA TTTGCATGTT NGNGTGCAGT AATTAAA 287

SEQ ID NO:3930
SEQUENCE LENGTH:290
SEQUENCE TYPE:nucleic acid
TOPOLOGY:linear
CLONE:HUMGS04655
SEQUENCE DESCRIPTION:
GATCCCAAGA TGACTGGGGT GGGAGGTCTT GCTAGAATGG GAAGGGTCAT AGAAAGGGCC 60
TTGACATCAG TTCCTTTGTG TGTACTCACT GAAGCCTGCN TTGGTCCAGA GCGGAGGCTG 120
TGTGCCTGGG GGAGTTTTCC TCTATACATC TCTCCCCAAC CCTAGGTTCC CTGTTCTTCC 180
TCCAGCTGCA CCAGAGCAAC CTCTCACTCC CCATGCCACG TTCCACAGTT GCCACCACCT 240
CTGTGGCATT GAAATGAGCA CCTCCATTAA AGTCTGAATC AGTGCAGAAA 290

SEQ ID NO:3931
SEQUENCE LENGTH:287
SEQUENCE TYPE:nucleic acid
TOPOLOGY:linear
CLONE:HUMGS04656
SEQUENCE DESCRIPTION:
GATCCTGAAG GGAAGGGGCA GGGATTTCTC CTTCTTCTTG GTCCTGGCTC CCAAGGGCTT 60
CTGTCTTCAT CTCTGCATGA GCTCTCCTTC CCAGAGACCA ACTCTTTTTN ATTTNATTTN 120
ATTTNATTTT TNAATTTATG TCTGGAGCCT GGCTACTCTG CATTTGGGNT TGGGGATGCT 180
GGGTGGGTGT GTGGTCCATG TTCAGCGTTC TAGCAACACG TGTGTGTGTG TGTGTGTAAA 240
GGCTATGCAG CCAAAATACC ATCTGGCCAG ACGGGCCCAC CCACAAA 287

SEQ ID NO:3932
SEQUENCE LENGTH:286
SEQUENCE TYPE:nucleic acid
TOPOLOGY:linear
CLONE:HUMGS04657
SEQUENCE DESCRIPTION:
GATCACCTGT TCCTACAGTG ACAACTGGAC CTGGCCCGAA CCCCTGGCAT CTGGCAACAT 60
TATTACCTGT CGAAACAGAA GTAGAGATTT GAAATAGAGG ATGCCAGTTC CCATTTCCTC 120
CTGCTGTCTG GAAGGAAGTC TGGCTTTCCA GCCCAACTCT AAGCTCATCT TTACATACAA 180
AATAAGGGTA TGTTTACATG GAAAGCAAAA ATAGAACCAT GGATTTACAG AAGGTTCCAT 240
AGCAGCAAAT ANCAGTGCTT ATGAAATAAA CATTTTAAAT ATTAAA 286

SEQ ID NO:3933
SEQUENCE LENGTH:283
SEQUENCE TYPE:nucleic acid
TOPOLOGY:linear
CLONE:HUMGS04658
SEQUENCE DESCRIPTION:
GATCTAATTG TGAAATCGTT CACCTTGTTA AACATCTAAG TAAGTCTGTA GTCGCTCTGC 60
AAACAGCCAT TATATTTATT TACTCTAGAA GAAACTGTAG AGTAGTAATT CGTGCTAATG 120
AGAAAAACAA AATACCATGT TCAAAACAGA TGTATTTGAA AACTTAATGA CATGGTTCCA 180

AAAACTAGAG CATGTATGTA TGCTGTGCAT CATCTCAGCA GACCTAAAAT ATCCCCAAGT 240
TGTCCCTTTA CAGCCATCAA TATATTTNAC ACTCTGCGGC AAA 283

SEQ ID NO:3934
SEQUENCE LENGTH:283
SEQUENCE TYPE:nucleic acid
TOPOLOGY:linear
CLONE:HUMGS04659
SEQUENCE DESCRIPTION:
GATCTGAGTC CTAGGNNCGC CCTCATTCAC TAGCAGTAAG AATTTAAGTG GTGCATGGCA 60
CATAGCACTG TACTAGATTC TGCAGGGGCA CAAACATAGA GCCAAACACT CTCCCTCTTG 120
GAGAATTTCA GACCCAGAAA AGGCCACGCA GTTCTAACTT TTGTCATCGG TTCCTTTTGC 180
TAAAAGGCAA AGGGTATGTT CCTTGCCTAT TGTCCAACAT ACCCCTTCCA AGATTGTGAG 240
AAGATGGGTA GCTGGGCATC ATTAAATATT GAATCAATTG AAA 283

SEQ ID NO:3935
SEQUENCE LENGTH:281
SEQUENCE TYPE:nucleic acid
TOPOLOGY:linear
CLONE:HUMGS04660
SEQUENCE DESCRIPTION:
GATCCACAGC TTGCAGGAGA AAGTCAGTGG CCCCAGACAC ACGTAACAGG GTGAGCACTG 60
AAATAAGAGC ACGCTGGGGC GTCCACACCA AGGGACTACG CCCCAGTCTG CCCACCTATC 120
TTTTCTCCAT CATTAACCCA GCGCTCTTTC TCCCTGGACT TCCAAGTACC AATGACCTTG 180
GGGCTTCTTT GCAGTTTAAA GGGAAAATGC TCTACAAANT AATTTCACTN TCAAACANNT 240
CAGCAGAAGA ATAAAATNTT TTTTCTGGNA TATCTAGTAA A 281

SEQ ID NO:3936
SEQUENCE LENGTH:280
SEQUENCE TYPE:nucleic acid
TOPOLOGY:linear
CLONE:HUMGS04661
SEQUENCE DESCRIPTION:
GATCCAATCA CATGGCAAAT GCCTCCTTTC ACAATAGAGC ATGGTGCTGT TTCCTCACTT 60
GANCTGAAAC TTCATAGAAG ACCAGGTGAG ACCACAGAAT ACTAGATGNN AATGTTCATG 120
TAGGTAAGCT TTATATTTCC TTCCAAGCTC CATTGCTACA AAGGTGTAAA TAAAATACAT 180
TTTTATGAGT TGAAGTAACT TCATGGTAGA AAATAATAAC TCTGCCTTCA AAAGCAAATA 240
ATGGTTTCTT TGTTAACCAA TAAAGCATAA GANAATGAAA 280

SEQ ID NO:3937
SEQUENCE LENGTH:278
SEQUENCE TYPE:nucleic acid
TOPOLOGY:linear
CLONE:HUMGS04662
SEQUENCE DESCRIPTION:

GATCTTTAAA TTTTTGTCCT TAGTTAAGTT CTGTATTTAG TGAATTAATT GCATCCTAAA 60
AAGTCAAACT TGAAAAGCAC ATTTTAAATG GCAAATCTAT TTTTTACATG TTTGTGAAGT 120
TTTTATTTTT TAGTAAACAG ACCATCAGAA GAGAACAATG GTACAGAGCA GGGGTCAGCA 180
GATGGATTTT GTANNGCATC AACTTGTAAA TATTTTCAAG TTTATTAGCT GTATGGCTCT 240
GGTTTCTGTT CCCTGTTCCA AATGTTAAAG TCTACTGN 278

SEQ ID NO:3938
SEQUENCE LENGTH:301
SEQUENCE TYPE:nucleic acid
TOPOLOGY:linear
CLONE:HUMGS04663
SEQUENCE DESCRIPTION:
GATCGACATT TAAAAAATGA GGTGAAAGAA AGCTATAGTG GCATAGAAAA AGTATAAAGC 60
TCAGTTAGTT TTTTTATTAT TATTATTATT AAAAGTTAAT TCAGGACTGA TGTGACCTAC 120
CAGATTTCAG AACATGTGTT AATAGTATAT ATGCCACTGA AAACTTAGGT CCTGTATCAT 180
ACTTTTTNCT TTAAGACTTT TTAAGAAATA TTACTTAAAC ATGTGGCTTG CTCAGTGTTT 240
AATTGCAAGT TTTCAATCTT GGACTTTGAA AACAGGATTA AACGTTAGTA TTCGTGTGAA 300
A 301

SEQ ID NO:3939
SEQUENCE LENGTH:276
SEQUENCE TYPE:nucleic acid
TOPOLOGY:linear
CLONE:HUMGS04664
SEQUENCE DESCRIPTION:
GATCTCCCTT TTACAGATAT CAGAAAGCAT GAAAAAAATA GAATTGGATT TAATGCTTTG 60
CAGATATTCT GTTTTTTTTC CCAATTATTT GATTTCACCT ATATACATAT TCTTTNNTCT 120
TTTTCCTAGC TTTAGAAGCA AACCAACAAA AGCAGTTTGG ACTTTAGTAA CTTTTTTTTA 180
CAGCAGCATT TAGAACAGTT TGCATGTTCT GAGGAATATT ATATGTGTGT TGGGGGGAAA 240
GGAATTCACA CTGAATAAAT TTTAGAAATA ATTAAA 276

SEQ ID NO:3940
SEQUENCE LENGTH:277
SEQUENCE TYPE:nucleic acid
TOPOLOGY:linear
CLONE:HUMGS04665
SEQUENCE DESCRIPTION:
GATCTTACTG CAAAGGAAAC TGACAGAAGA AAAAGAAAGT GCTTTTGAAT TTNTTTCCTC 60
TGCCTGACTA GACAATGATG TTACTAAATG CTTCAAAGCT GAAGAATCTA AATGTCGTCT 120
TTGACTCAAG TACCAAATAA TAATAATGCT ATACTTAAAT TACTTGTGAA AAACAACACA 180
TTTTAAAGAT TACGTGCTTC TTGGTACAGG TTTGTGAATG ACAGTTTATC GTCATGCTGT 240
TAGTGTGCAT TCTAAATAAA TATATATTCA AATGAAA 277

SEQ ID NO:3941
SEQUENCE LENGTH:279

SEQUENCE TYPE:nucleic acid
TOPOLOGY:linear
CLONE:HUMGS04666
SEQUENCE DESCRIPTION:
GATCCTAAGC CATAGACAGG CTAATTGCCC ACCACTCCCA GGAATATTGA AATAGCTACA 60
TGACCATAAT GTGTTTAAAA TGTGGTATGC TCTTGAGATA TTTAAAGTTT TGGCAGTAAA 120
ATACTCTGTT TTTAAGTATG AATGTATTTC ATTCATATTT CCTCTCACAA AGGAAAATGA 180
CTTCAGTATA GATTTGTTTT TATTAAAATG CATTTTTTAT TCTTAAGTGG TAGGAAGCAA 240
CATCCAAAAN TGCTTAATAA AATGCTTTTA AGCTGCAAA 279

SEQ ID NO:3942
SEQUENCE LENGTH:273
SEQUENCE TYPE:nucleic acid
TOPOLOGY:linear
CLONE:HUMGS04667
SEQUENCE DESCRIPTION:
GATCAACTGG CTTTTCTCTA TTATGACTTT AGATGAATCG TTTTAATAGA TTTGCTAATG 60
TTAAGACATC TCTGCATTTC TACAAGAATC TGATGTAGTC ATAGTATACT ATTCTTTTAA 120
TATACATCTC CTTTCAATGT TAACAGTGGT TATTTATGAT TGGTTGGTCC ATAGTAGATT 180
TTTATTTTGT GTTTCTTATC TGNACTTTCT GATTTTCCTA CAAAGATNNG NGNNNTACTT 240
GTATAATAAA AATAAGAGCT TTAAATNGTG AAA 273

SEQ ID NO:3943
SEQUENCE LENGTH:268
SEQUENCE TYPE:nucleic acid
TOPOLOGY:linear
CLONE:HUMGS04668
SEQUENCE DESCRIPTION:
GATCGCGTCC AGATGGAAAA CGGAAGCAGA GGCTTCTAAT CGTCGCATTT ACTGGCTCCA 60
GTGCAACACA TCCATCTGAA AACACTCGGA AGTCTGGTGC TTGGAGAGGG TGCCATTGTC 120
TCTTGTACAT AAGGTCATGA CGTGTCTATG TCAAAAGTTC TTATATATTT CTTTTATAAG 180
CTGAAAGAAG GTCTATTTTT ATGTTTTTAG GTCTATGAAT GGAACGTTGT AAATGCTTGT 240
CAAACAATAA AAATAACGAA AAGTGAAA 268

SEQ ID NO:3944
SEQUENCE LENGTH:268
SEQUENCE TYPE:nucleic acid
TOPOLOGY:linear
CLONE:HUMGS04669
SEQUENCE DESCRIPTION:
GATCTNGTGC CTGGAAATAG CCTGAGGTGG CTCAGGAAGC GGAGAAAGGG TGCCAGACCA 60
TTCTCTGGCG GGGACCAGGG CCCAAGGCCC CAGGGCTGGA AGNAGACCAA GGGGCAGCCG 120
CCCTGGAGGG ACATCAGTGC TTCCTCTTCC ACCCAATTCC CCCACGCGGT TCCATGTTTT 180
CCCACCAGCC TGTTGGCGAA GTTGCTGCTC CGGCATTCAG TACCTNNTNN NNCCAGAGAA 240
ATANNNNGAG TTTCTATTTT ATGTTAAA 268

SEQ ID NO:3945
SEQUENCE LENGTH:267
SEQUENCE TYPE:nucleic acid
TOPOLOGY:linear
CLONE:HUMGS04670
SEQUENCE DESCRIPTION:
GATCAGATGG TTTAAGGCTA CAACACGAGG CCAATATCTG TATGTNAAGG AGACAGAAAC 60
ATCAATGTTT GGAGTATATA TTGACCTTCA TTCTTACTTT CTCCTTATTC TCTTATGTAA 120
CTGTTAATTT TTCCCTTCCT TGGCTCTATG TATTTAATTA TAATAAGCTT TACCTACAAG 180
CAAAACAAAC CAAACATGAA CAATGAAAAC TACTTTTGGT CATGTAGCAG AAACAGCAGT 240
CAATAAAGGA GAGAATGAAA CTAGAAA 267

SEQ ID NO:3946
SEQUENCE LENGTH:266
SEQUENCE TYPE:nucleic acid
TOPOLOGY:linear
CLONE:HUMGS04671
SEQUENCE DESCRIPTION:
GATCCTGTGA AAGCTGATGC CCATGTGCCC TGAGAGCTGC CTCTCAATCC CTGTCCCAAG 60
CCACAGCTAT GGCGTTAATG TCACCAGTGT TCTCACCCTC TAGGCCCTGT GCCTGGAGGT 120
GCCTCCACAG CCGACCAGCA GCCACCCCGC CTGCTTCATC CACATCAGGA GGGTCCGGTG 180
AGGCTGCAGC AGTGGTTAAG GAGTAACACC TTCTTGTATT AAGGAATTTT AAACTAAATA 240
AAATGTATGT TGGAGATACT GTTAAA 266

SEQ ID NO:3947
SEQUENCE LENGTH:265
SEQUENCE TYPE:nucleic acid
TOPOLOGY:linear
CLONE:HUMGS04672
SEQUENCE DESCRIPTION:
GATCCCTAGG TCTTGGGAGC TCTTGGAGGT GTCTGTATCA GTGGATTTCC CATCCCCTGT 60
GGGAAATTAG TAGGCTCATT TACTGTTTTA GGTCTAGCCT ATGTGGATTT TNNCCTAACA 120
TACCTAAGCA AACCCAGTGT CAGGATGGTA ATTCTNATTC TTTCGTNCAG TTAAGTTTTT 180
CCCTTCANCT GGGCACTGAA GGGNTATGTG AAACAATGTT NAACATTTTT GGTAGTCTTC 240
AACCAGGGGT TNTTTCGGTT TAACN 265

SEQ ID NO:3948
SEQUENCE LENGTH:265
SEQUENCE TYPE:nucleic acid
TOPOLOGY:linear
CLONE:HUMGS04673
SEQUENCE DESCRIPTION:
GATCTAGNAA ATGCATATAT TTAGCGGGTA TTTGAAAGAT AGAAGCATTA TTTATAACTG 60
AAGAGTATAA CACTTATCTN CTTAAGTAAA TGCTCTCATA ACTTGTATAC ATTTTTNCAG 120

GTACAAATTC AAAAGTATGT AAGCATTGTA TAATGTGACA ATTGTATAAT TTATGTAATC 180
TGATGCATGA NTCAGAATTT TTATATAATA TATGATTATN GACTTATAGT TGATTAANGT 240
GTTTAANCTT AAAANANAAN ANNNN 265

SEQ ID NO:3949
SEQUENCE LENGTH:263
SEQUENCE TYPE:nucleic acid
TOPOLOGY:linear
CLONE:HUMGS04674
SEQUENCE DESCRIPTION:
GATCTCTAAA CTAGCTCTTT TGTTAGTATC GTCTCACAAA CTGATTATTT TCCTCTTTCT 60
TTGGTAAGGT CAATAACTGG GAGATGGACA AACTAGAGAT GGAAGATGCA GTCACATTTT 120
TGAAGACTAA AATCTATTCA GTAGAAGCTC TTCCTGTTGC TGCTGTAAAT GTGCAAAGCA 180
CACAATAAAG TGAAAATCAA CCTTTTCATA TTAGGAGACA TGCATTTGTA AAAATTAATA 240
AAGATGACAA GTCAGTTNTC AAA 263

SEQ ID NO:3950
SEQUENCE LENGTH:261
SEQUENCE TYPE:nucleic acid
TOPOLOGY:linear
CLONE:HUMGS04675
SEQUENCE DESCRIPTION:
GATCCACACA CTACAGGAAA CAGTTGGCTT TGGAAATCCA TCTTTCTGAT ACCCCTCCAG 60
CTGAACAATT TTCTGCATGG GACTCTGAGT TCCTTTCCTT CCCAGAGAGT TTTACCGTAT 120
GAAGCCTGGA GCTGGAGAAC GAAAGAATCT TCACTATAAA CAGAGACCCA TTCATTTATT 180
TCCATTGGCT GTGTTACTGG ATGTTTTACT GGTTGACGAG AAACTGGGTC ACAATAAAAA 240
AATGGAGATA TGAAACTCAA A 261

SEQ ID NO:3951
SEQUENCE LENGTH:315
SEQUENCE TYPE:nucleic acid
TOPOLOGY:linear
CLONE:HUMGS04676
SEQUENCE DESCRIPTION:
GATCTGTTTG TAATACAAGG TATTTGGNCT TGTCTTTAAC TGTATAGTGA TTTGTGGGCA 60
GGCTGCAGGA GCCAGGATAA AGAATGCATG TTTGAAATCT NCTCTATACA AATGCATCCA 120
CACTCTCCNG CAAAGGGATG AGTTTCTTAG CCGTTTTCAC TTGATACAAA TGCTATTGTT 180
TCCTTTGGAN GGNGTGAAAA ANTGNANGCT CCACCTGCTC CGTGAGTAGC ATTGCTCTCA 240
ACTGGTGTTC CCAGCCGTTC ACATGTCTAC CCATTCTCTC CCTTANGGTT TTCTTAGGGN 300
GCNGGNGGNT GNNTN 315

SEQ ID NO:3952
SEQUENCE LENGTH:258
SEQUENCE TYPE:nucleic acid
TOPOLOGY:linear

CLONE:HUMGS04677
SEQUENCE DESCRIPTION:
GATCTAAACT TGATATGTTA AATTTAAATT TAATTTCCCC AAGTGTGAGT ACTATATGTN 60
ATTGTAGATA ATTTGCAAAC TACAAAAAAT AAAAATAATT GGCTGGGCGT GGTGGCTCAT 120
GCCTGTAATC CCGGCGCTTT GGGAGGCCGA GGTNGGTGGA CCACCTGGGG TCAGGAGTTC 180
AGGACCGGCC TGGCCAACAT GGTGAAACCC GTCTCTGCTA AAGGTATAGA AGCTNGCCGG 240
GTGTGGTGGT GGGCACCN 258

SEQ ID NO:3953
SEQUENCE LENGTH:258
SEQUENCE TYPE:nucleic acid
TOPOLOGY:linear
CLONE:HUMGS04678
SEQUENCE DESCRIPTION:
GATCCTCACT CAACGAAAAC TCGNTTGGAA ACTGTTCCGN CTGGCAGTCC TTTTTTGTTG 60
TTTTCCATCT CATTTCCCTT CCATCTGAAA GTGGCATTCA GCTGACTNGC TCATTTAGAC 120
NGTTCACGGA GTCTGAATCT GCCAACGTGG TGTTGGAGGC TCCACCTTGA AAAGGGCCAC 180
AGTCAGGGCA ACTTTCCCCA TACAGGAAAA CTTGAAAATT ACATCAACAG TCTACGTCAC 240
AGCCAAATTA TATTTCCN 258

SEQ ID NO:3954
SEQUENCE LENGTH:257
SEQUENCE TYPE:nucleic acid
TOPOLOGY:linear
CLONE:HUMGS04679
SEQUENCE DESCRIPTION:
GATCCAGTGG CTGCAGCTTC AATCCCAGTG CTGNANTCAC ACATCCATTC TGCCCTGGGG 60
GACCCTGGAG CCTACTTGTG CGCTTTGCAT TTCATTGATT GACGNCTCCC TTCAACAAGC 120
ATTTACTGNG CGCCTACTAT GTACTAATGC TAGANGTTAG ATGTACAAAG AAGACAGTTT 180
TCATCCTCTA GGNACTCATA GGCTAATGGT GAGACACACA GACANACATC ATTATATTAN 240
AATANGCTAA GNGAAAA 257

SEQ ID NO:3955
SEQUENCE LENGTH:257
SEQUENCE TYPE:nucleic acid
TOPOLOGY:linear
CLONE:HUMGS04680
SEQUENCE DESCRIPTION:
GATCTACAAG GCCACACAGA TAATTNCAAA TACATCTCAT TTCCTTCTGA TTCAGGCCTC 60
GGNAGACCCC AAGGGGAAGG AGTTCTCATG GGNCACTATA ATCNATGAAG CTGAAGTTGN 120
AATATAGANG GCAGATTTAA AGTTACCATT TTTTTTTAAA CACCAGCTAT GGTCCTTTCA 180
AAATTGTACA AGCTGTCAGT ATCACCTGCA CATGAGCAAT TTAGTCTNNN GGNGGNGTGA 240
GAAGTTTAGG TCTTTNN 257

SEQ ID NO:3956

SEQUENCE LENGTH:256
SEQUENCE TYPE:nucleic acid
TOPOLOGY:linear
CLONE:HUMGS04681
SEQUENCE DESCRIPTION:
GATCTGCCCA CCTCGGCCTC CCAAAGTGCT GGGATTACAG GCGTGAGCAT GAGTGCCCAG 60
GCAGAGAAGT CCTACACCAC GTGTCAAACT CGGAACACAA AGGGNCACTG TCTTATTGCT 120
TGGTACAGTA CTCCTGTTTG TTGAGAGCCC ATTTACTAGA GCAGCTCTGT TTGTGCTTAA 180
TGAAAGCTCT TTTCTGAGAG GCAACAGAGT GTGTTCTATC ATAAATGAAG CGCTCTTCAA 240
GAACTTCCTG AGCAAA 256

SEQ ID NO:3957
SEQUENCE LENGTH:254
SEQUENCE TYPE:nucleic acid
TOPOLOGY:linear
CLONE:HUMGS04682
SEQUENCE DESCRIPTION:
GATCCACCGT CGGATTCCGT CTGCAGAGGA GGACGTAGGG GGCCGTCACA CCCACCAGGC 60
CTCCCTGCTG TGCTTTAACA CAGGCAGAAG AGGTTCATGG CAATATAATA GTCAATGAAC 120
TTTCAGTTTA AATTGTGTAC ATTTTTAAAT TGTAAGATTT TTACTGTATA TTGATGCATA 180
GTGTGATTCA ATAAATTGCT TGTAATTTAA AAACTATTTA ATATTCAAAA TAAATATAGT 240
TATATATTTA TAAA 254

SEQ ID NO:3958
SEQUENCE LENGTH:252
SEQUENCE TYPE:nucleic acid
TOPOLOGY:linear
CLONE:HUMGS04683
SEQUENCE DESCRIPTION:
GATCTGTTTT AAAGTTGGGG GGTGGGAAAT TTAGCTGACT AGGGACAAAC ATGTAAACCT 60
ATTTTCCTAT GAAAAAAATT TTAAATGTCC CACTTGAATA ACGTAATTCT TCATAGTTTT 120
TTTAATCTAT GGATAAATGG AAACCTAATT ATTTGTAATG AATTATTTAG ACAGTTCTAA 180
GCCCTGTCTT CTGGGAGTTA TCANTNTTAA AGAGANCTTT TGTGCAATTT CAAATGAAGT 240
TTTTATAAGT AN 252

SEQ ID NO:3959
SEQUENCE LENGTH:251
SEQUENCE TYPE:nucleic acid
TOPOLOGY:linear
CLONE:HUMGS04684
SEQUENCE DESCRIPTION:
GATCCGGAGT CTTTGAGGCC ATTNTTGAAC TACTAATTCA ACTAATCCTG AACCACCATA 60
TTTTCATATT CATCCATGAA GTGTTAAGTG TTCTTATGAT TTAAACCACT TAGCATTGGT 120
TTTTATATTT CTTGCAGCCA AAATCTTCCT AACAGTAAAA TAAAGTTTCA AGTTTCATAA 180
GTGCCAATGA TTTTTAAATG TTAATAAATT TGTACTAAAT AAAAATGGCA ACAGGAGCAG 240

GTAAACCTAA A 251

SEQ ID NO:3960
SEQUENCE LENGTH:251
SEQUENCE TYPE:nucleic acid
TOPOLOGY:linear
CLONE:HUMGS04685
SEQUENCE DESCRIPTION:
GATCTTCTAA CTTATAAATG TCAAATATAT TTTTGTATTA GATTGTGCCT ATNATGAGAG 60
AAATCTTTAA TGTATATTTN ATGTATGCTG AACACATTTT TATAATTTGN CACACTTGGA 120
GGCATGGTCT AGTAATAATA ATGTAATATG TACCTTGCTG GTTANTATAA CTAAGATTTT 180
GCCTTTATTG GGTTAGGTAT CTNTTNTTTA TTNTNGCACC NGNTAGCTGN CTTCTACTGA 240
GTAAAGANTT N 251

SEQ ID NO:3961
SEQUENCE LENGTH:251
SEQUENCE TYPE:nucleic acid
TOPOLOGY:linear
CLONE:HUMGS04686
SEQUENCE DESCRIPTION:
GATCCAACTT CAAAGTAAAA GTTGGAAAGG GCAAGAGTCT TTTAAGCGGA ACTTCAATTC 60
TNTTNNCATG GTTACTTATA TATAATGAAT ATAAATAGAC AAATAGGACT TGGAAAATGT 120
GGTATATTCT AAGTCAAGAT GCTATGAAGT TGTTATAGTT TAAGGGNCAT TCTTAAGAAG 180
GAATTACTTA AGTNCATTAC TCTNCTCATT TCAAGGACAT GAAAAAGTAA AGGATGGTCT 240
GGCACTATTT N 251

SEQ ID NO:3962
SEQUENCE LENGTH:250
SEQUENCE TYPE:nucleic acid
TOPOLOGY:linear
CLONE:HUMGS04687
SEQUENCE DESCRIPTION:
GATCCTGGAG ACGCCATTTC CGAGATTGTT CTGCATATTC ATTTGCACAT TGTTGTCTGG 60
GTTGGACATG CGTGTGGGCT TCAGTGTGAG GCTTTTAATA TGTATATCCT GTTATCAATA 120
AAACAATTAT CCAAGTGGTT GAATCCTGTG AGACTTGGCA AGTGTGTGCA AATNAAGTAT 180
ACTTGACTTT TCAACCTCTT CTTTCAATGT NGCNNTTATA TGANATAAAG TAATCCATTT 240
ANCAGTTAAA 250

SEQ ID NO:3963
SEQUENCE LENGTH:245
SEQUENCE TYPE:nucleic acid
TOPOLOGY:linear
CLONE:HUMGS04688
SEQUENCE DESCRIPTION:
GATCGACATG AGATACAGAC GAGAGATTAT GTCTCTTATG GCTTTATCAT CAATTAAACT 60

TAGCAAAGTC TGTTAAATTT ATTTCAGAGT CTCCCAGCTG TGAAAAGTAG AGTTCCACTT 120
ATGTCATCCA TATCTGCACA GTAATCTTTT TTTTAATCCT AAACTTTCTT AACAGGTTTC 180
TNCTCCTNAT TCTAGACAAT AATGCTATGT GTTTTCCTTG AATACCATGA TTTTAAACTN 240
TTAAA 245

SEQ ID NO:3964
SEQUENCE LENGTH:245
SEQUENCE TYPE:nucleic acid
TOPOLOGY:linear
CLONE:HUMGS04689
SEQUENCE DESCRIPTION:
GATCGGTCAT GCTGGGGCCA AAAACATGGC AAGAAACGTC CCACTGTCAA CCTCCCGGGC 60
AAGCGTGTGC ACCTGCAGCT CAGGGCCAGC CCACCACACT GCCTTAGTGA CACTGCAGCC 120
GGGGGAGCCA CAGGCCGCCA GCAGGCTGAG GAACAATGCC GTGTGACCTT TCCNNCNNNN 180
AAAAAACAAA AACTGCAATT TTTATTCAAA TAAAAAAAGG GTAAAAATAA AAGTTAATAA 240
GGAAA 245

SEQ ID NO:3965
SEQUENCE LENGTH:244
SEQUENCE TYPE:nucleic acid
TOPOLOGY:linear
CLONE:HUMGS04690
SEQUENCE DESCRIPTION:
GATCATGGAA AGTTTCAGTG AAAACCATCA TGCTGTCTTC CAGAACCACG TTCTCCTCTG 60
AGGAGGATGA ATGAAGGTTG GGTTTCAGTC ATCATGCCTG CCCTTTTTTT CTTTGTATTG 120
TTTCTTAAGC ATCTCAGAGT CCTCTCATGG AATTTGGTTT CTATTTCCCC TCCCAATCTC 180
CACCTCACTC CATATCATAA GACATGTCTG CATTCTGGAA TATTAAACGT GGACATGGCT 240
CAAA 244

SEQ ID NO:3966
SEQUENCE LENGTH:244
SEQUENCE TYPE:nucleic acid
TOPOLOGY:linear
CLONE:HUMGS04691
SEQUENCE DESCRIPTION:
GATCATGCTT TTCCCTACTA AACTTCTGTA ACTGCATGTA TGATACATTA TTGCAGAGGT 60
AAGAGATAGT TTAATGGATT TTTAAAAACA AATTACTATA ATTNATCTGA TGTTCTCTAG 120
TTGCATTTTG CTGAAATGTA GTGCTGTTCT AAATTCTGTA AATTGATTGC TGTTGAATTA 180
TCTTTCTGTT GAGAAGAGTC TATTCATGCA TCCTGACCTT AATAAATACT ATGTTCAGTT 240
TAAA 244

SEQ ID NO:3967
SEQUENCE LENGTH:242
SEQUENCE TYPE:nucleic acid
TOPOLOGY:linear

CLONE:HUMGS04692
SEQUENCE DESCRIPTION:
GATCCAAGCC CTTGTTCAGA TTTGGTGCCT GATAAGACAG GGGTTTCTCT TTTTGTGACC 60
TTTATTATTA TTATTTTGTT AACTGTTGTA ACCAGTTAGC TGTTGTGTTT TAAGATAGAA 120
AGGAACAAGA CTAAAATTGT AAATACTTTG TAAACATCAG CATTTGTACT TGAATAGTAG 180
GATTTTAAAG GGCATTGATA GCATACCAAA CAAAAGGCAA AATAAAGTGA CCTTTTTATA 240
AA 242

SEQ ID NO:3968
SEQUENCE LENGTH:241
SEQUENCE TYPE:nucleic acid
TOPOLOGY:linear
CLONE:HUMGS04693
SEQUENCE DESCRIPTION:
GATCTTCCTC CCATGAAAGA AAAANCAAGA ACCAGAGGCG TAGACTNACT NAAGACACAA 60
CTCCTGGCTT TCTNAAGCTA TGGACTTGGA TTGGATTGCT GGGGGTTTTT AGAGAAAGGT 120
GACAAATTTC AGTACCTCTG GCATGCTGTC CCAGGAAACT AGGGCTCCCA CTAACTTATG 180
AGGTTTTTAA ACACATTGAA AATGACATGA CACCTAAAAT AAATTTGGAT TTGCTCATAA 240
A 241

SEQ ID NO:3969
SEQUENCE LENGTH:240
SEQUENCE TYPE:nucleic acid
TOPOLOGY:linear
CLONE:HUMGS04694
SEQUENCE DESCRIPTION:
GATCAAGGGT AAGAATAAGT ATCCACCTCT TTCCTTTTAA AAATTTTCCC TCAGAGTCCC 60
TATGTTGCAT ATTTNATTCT GTTCAGAATC TAACCTTTCC CTAAGGCTTA TCTTTCTAAC 120
AGATACTAAT TATGAATGGG CATCAACAAC TCTAATTAAA CCATTCTTTA AAACTCCTCT 180
GGGTGTTTTC ACAAAAGAAA TTCTCCCATG TTATGATGTG TGTGGTTGCT AAAAAGTAAA 240

SEQ ID NO:3970
SEQUENCE LENGTH:238
SEQUENCE TYPE:nucleic acid
TOPOLOGY:linear
CLONE:HUMGS04695
SEQUENCE DESCRIPTION:
GATCCAGACT GGCCAGATTT GAACTCCAGC ACTATTCCTT GATACCTCTG AAACTTGGGA 60
CAAGTAACTT AGTCACTATG TATGAAAAGA GATAATAATA CTAGTACCTT CCTCATACTA 120
CAAGACTACA AGATGTGGTC AAGACCAGAA ATATATAAGC ACTGTAAAAA CAATGTAAAA 180
CCAAATGNAA ATTCTTCATT TGTATCTNCC AAAAATAAAA AAATAGCTAT TTGGGAAA 238

SEQ ID NO:3971
SEQUENCE LENGTH:238
SEQUENCE TYPE:nucleic acid

TOPOLOGY:linear
CLONE:HUMGS04696
SEQUENCE DESCRIPTION:
GATCAACCAG AAAAAAAAAA TATNATGAAA TGGATACACA CAGTGGACAC CTGTGAAGTT 60
ATCTTAGCTC CCCCAAAACT GAGANGTACA AATTAGTCTC CAAACCTAAT TACCAGTTTA 120
CAGGAAACAT NGGGAATAAA AGANCAANTT AACAACACAA AGAAGCAAAC ANCCAANTGC 180
ACAGTTTGGG AAATTCTGCA GAGGTAATGG CCTAGTTTTT TANCCAGTAC ATGTCAAA 238

SEQ ID NO:3972
SEQUENCE LENGTH:237
SEQUENCE TYPE:nucleic acid
TOPOLOGY:linear
CLONE:HUMGS04697
SEQUENCE DESCRIPTION:
GATCATAAAT GACAGGTGTG TGGAGGTCAT TGCAAAGGAA GGACAAAACC TGAAAGAGCT 60
ATATTNGGTG TCCTGTAAAA TCACAGATTA TGGTAGGTAA TGANCCATTT NCCTAATCCT 120
TTCTAAGANT AAAANGTTAC AAAATCATTG AAAGTNCTTA AAAGGAAATC AAGANCTATA 180
TCATAGAGAC TGTCTATCGT CACTTATGCT CATNGTGACC NTACCAAANA NTAAAAN 237

SEQ ID NO:3973
SEQUENCE LENGTH:236
SEQUENCE TYPE:nucleic acid
TOPOLOGY:linear
CLONE:HUMGS04698
SEQUENCE DESCRIPTION:
GATCGTAGAA GCCAAATGAA ATGTGACATA TCTACTGACT GATGACAAGG GAATTTCATT 60
AGGAAGAAGG TAAAGAAACA TCGTTGAGTA GCCTACCTTG ATTTCTGTCA AGTTCATAAC 120
CAGCTTCATN CTTTTAAAGG CTTCAGGTTT GAAATTAAGT CAACTGCATG CAGCTTTGCT 180
GATAAATGAA TAATTCTCTT TGATGCCATT TATGAGAAAA GACTTCAATA TCTGTN 236

SEQ ID NO:3974
SEQUENCE LENGTH:173
SEQUENCE TYPE:nucleic acid
TOPOLOGY:linear
CLONE:HUMGS04699
SEQUENCE DESCRIPTION:
GATCTTCCAG TTTAGGAGCA ATTTGGTGAA AGGAGTCGGG TGACCAGAGC TGGAATAACC 60
GTCTGCATGG CTGCACTCTG GTANCAGAGT GGNGACAGAN GTAAGGATAG TGTGGAGGGC 120
ACAGTTGCAA GTTGAGTCTT TCCATGAGAC AATGTGCCAA AGTTAAAAAA AAN 173

SEQ ID NO:3975
SEQUENCE LENGTH:234
SEQUENCE TYPE:nucleic acid
TOPOLOGY:linear
CLONE:HUMGS04700
SEQUENCE DESCRIPTION:
GATCCCTAAA TAAGTAGTTA TTCTGTTTTC CCTGATTTTN AACTTTTACT AATAGAATGA 60
GAGTAAATAT TTTTGGGTAT GTGGCTTCTT TTGTTCAACA TTGTTTTAAG ATTCATCCGT 120
GTTGCTTGTG TAGCTGTAAT TTGTTTTAAT CTTTATAGTA CATTCAGTTT TGTNAATGCT 180
TATTGTAGGA CTGTACCATA ATACAGGCAG CATGCTGCTG ATAAACACTG GAAA 234

SEQ ID NO:3976
SEQUENCE LENGTH:234
SEQUENCE TYPE:nucleic acid
TOPOLOGY:linear
CLONE:HUMGS04701
SEQUENCE DESCRIPTION:
GATCCTCCTG CCTCGGTCTC CCAAAGTNCT GGGATTGCAG GTCTTGGTCT TGATNCCAAG 60
TTTGGGAAAG NAGCTAAGAA GACATCCCAC TAGGAAGAGA GAGACTACCT CCACATCAAA 120
AATATTTAAG TNATTATCTC AAACAGTGAC ATCTCTTGGG AAAATGGACT TAATAGGAAT 180
ATGGGACTGA GTTCCAGTCT TTTTNAATAA AATAAAATCA AGCAANATCA TAAA 234

SEQ ID NO:3977
SEQUENCE LENGTH:234
SEQUENCE TYPE:nucleic acid
TOPOLOGY:linear
CLONE:HUMGS04702
SEQUENCE DESCRIPTION:
GATCAACCCC GTGAAGGTGG CCAACCTGGT GAAGGCGTAC GTGGACAAGT ACAGGCACAT 60
ANCNCAGGCA CANGAAACCA GAGGCCGGGG AGGAGCCGCC CACGCAGGGG GCCGAGGGCT 120
GAGGCCAGGC AATCACGGGC TATGCCNGGG AGCTGTCGGG AGTGGCGGGA ATCGGGGCCA 180
TGCCCGGGGA GCTGTCGGGA GTNGCGGGAA TNGGGGCCAT GCCCGGNGGA GCTN 234

SEQ ID NO:3978

SEQUENCE LENGTH:231
SEQUENCE TYPE:nucleic acid
TOPOLOGY:linear
CLONE:HUMGS04703
SEQUENCE DESCRIPTION:
GATCATCGCA TTGAAATGGT TTTGGGAAGT ATGGAAAATT AAATGGATTT TTGAGACACT 60
AGTTTATTGG CTATGCAAGT GACAGTTTTG AAAAAGGCTG AATCTTATTG TTGGAGCCTA 120
ATGTAAATGC ATATATAAAT TCTGACTGTA TTTCAGTCAG TGTTTTCTTG GAAAAATTCC 180
CAGATTGGAA GCAGAAAGTA CAGACCAAAT AAAGATTATT CACCCACCAA A 231

SEQ ID NO:3979
SEQUENCE LENGTH:231
SEQUENCE TYPE:nucleic acid
TOPOLOGY:linear
CLONE:HUMGS04704
SEQUENCE DESCRIPTION:
GATCGCTCTC ACTGGCCTGC TGCAGATNAG CCAGGGGGAG CCTAGGCCCA GCTCCTCCGC 60
GGTTGGCCCC CCAGACCATA CCTCTGACCC ACCCAGCCCC CNNTGGTAGC CCCAGCAGTT 120
CTCAGGGTGC TGACCTCTCT CTCCCACAGA CCCCAGACAC CCATTGTCCA TAGCCTTCTN 180
AGGGCAGAGT GGGCTGGTTG TGTTGACAAT AAAACAGTGT TGGTTTGCAA A 231

SEQ ID NO:3980
SEQUENCE LENGTH:242
SEQUENCE TYPE:nucleic acid
TOPOLOGY:linear
CLONE:HUMGS04705
SEQUENCE DESCRIPTION:
GATCAAGTCT TGTCAGTTCG TGCCCTCTTT CCCCATGTTC CCTGGGAAGA CGGGTGGTGG 60
CAGAGTGAGA AGGCCACTGG TTCTGTGCCG CAGCACGCAA AATTTAGAAT TCTACAGACT 120
AGCTCTATAC GTAGTGAGGA CCCAGATTTA GAGAAACTGA CCAATATTTA TCTCCGCATT 180
TGTGTGTGTG TCCAACTCTG TAGGNCAATA AACCANCAAG ACAAATGAAC TGTGCTTCCA 240
AA 242

SEQ ID NO:3981
SEQUENCE LENGTH:230
SEQUENCE TYPE:nucleic acid
TOPOLOGY:linear
CLONE:HUMGS04706
SEQUENCE DESCRIPTION:
GATCCACCTC TACACCAGCC CAGGAAGCCC CATCTGTGCC TGCCCTCAGG TGGTCCACCA 60
GTCTCCCCCT TTGGTTCCCT TCCAGTCTNT TCCCCCTTTC TATCCCAATN ACCAATAGAA 120
ATGCTAACAT CCCTGCCTGG TAGCCAGACT AGCCCACTAA AGCTCCCCTG TAAATGGGGG 180
CTCCATTAGT TCTGCTGCCG AGACTAATAA ACGATTTGGT TGGCTCTAAA 230

SEQ ID NO:3982

SEQUENCE LENGTH:229
SEQUENCE TYPE:nucleic acid
TOPOLOGY:linear
CLONE:HUMGS04707
SEQUENCE DESCRIPTION:
GATCTATTCC CACATGANGT GCGCCACCGA CACGNAGAAC GTCAAATTTT TTTTCGACGC 60
TGTCACCGAC ATCATCATCA AGGAGAACCT CAAAGACTGT GGCCTCTTNT NAGGCAGGGC 120
CTGTNCTGCA GTCGGGGACA AGGAGCTTCC NTTTGGCAAG GCCGGGGCAC AATTTGCACT 180
CCCCTCAGCT AGACGCACAG ACTCAGCAAT AAACCTTTGC ATCAGGAAA 229

SEQ ID NO:3983
SEQUENCE LENGTH:226
SEQUENCE TYPE:nucleic acid
TOPOLOGY:linear
CLONE:HUMGS04708
SEQUENCE DESCRIPTION:
GATCCTCCCT GCCCAAGTCT TACTTCGTGA GCTGGCCTTG CTCCCCATCC CCCAAGTGCC 60
TCACCAGTCC CCCAGACTGG GTGAAGGTAC AGCTGGCTCC TTTCGGGGGT GCAGCTTCAA 120
CTCTCTCGGC GGTAGGGCGG TGCCATCCCC ACCCATAGGG CTGGCTCACA TCCAGTCACT 180
CCCAACAGCG TCCANCACAC AAATAAAAGA CCCTTTGGCC CTGAAA 226

SEQ ID NO:3984
SEQUENCE LENGTH:226
SEQUENCE TYPE:nucleic acid
TOPOLOGY:linear
CLONE:HUMGS04709
SEQUENCE DESCRIPTION:
GATCACTTGN GGTCAGNCGT NCGAGACCAN TCTGGTCAAC ATGGCAAAAC CCCGTCTCTG 60
CTAAAAATGC AAAAATCAGC TGGGTGTAGT GGTGCGTNCC TGTAATCCCA GCTACTGGGA 120
GCTGAGGCGA AAGAATTGCT TGAACCTGGA AGGTGGTAGG CTGCAGTNAG CCAGGATTGT 180
GCCACTTCAC TCCAGCCCGG GCAACAGNGC AAGACTCTGT GTCAAA 226

SEQ ID NO:3985
SEQUENCE LENGTH:225
SEQUENCE TYPE:nucleic acid
TOPOLOGY:linear
CLONE:HUMGS04710
SEQUENCE DESCRIPTION:
GATCCTGGGC CAAACTGATT TACTATTTTA AATTTNAGTT TTAATGNGAC AGGGCCTTGC 60
TATGTTGCAG GGTGGTGTTG AACTCCTGGC CTCAAGCAAT CCTCTTGCCT CAGCTTGCCA 120
AAATGCTGGC ATTACAGGCC TGAGCCACTG CCCCTGGTCT GATTTACAAT TTTNAATTGA 180
GGGTTAGGGA GTCATAATAC CANCAAAGAT GGTAAAAGGA ATTTN 225

SEQ ID NO:3986
SEQUENCE LENGTH:221

SEQUENCE TYPE:nucleic acid
TOPOLOGY:linear
CLONE:HUMGS04711
SEQUENCE DESCRIPTION:
GATCATCCTC TAAACATTTT ATCATTTATT AATCCTCCCT GCCTGTGTCT ATTATTATAT 60
TCATATCTCT ACGCTGCAAA ATTTGGGTCT CAATTTTTAC TGTGCCTTTG TTTTTACTAG 120
TGTCTGCTGT TGCAAAAAGA AGAAAACATT CTCTGCCTGA GTTTTAATTT TTGTCCAAAG 180
TTAATTTTAA TCTATACAAT TAAAACCTTT TGCCTATCAA A 221

SEQ ID NO:3987
SEQUENCE LENGTH:221
SEQUENCE TYPE:nucleic acid
TOPOLOGY:linear
CLONE:HUMGS04712
SEQUENCE DESCRIPTION:
GATCATCATC TGTGAAGCAT CATGGTAGCT TCCAAGCATT TGCGGCCCAC TGGCCAGNTG 60
CATCCAAAAC ACATTCAGGA AACCCGAGAG TTGTAAGTTG CACATGTGTG GCCTACAGTG 120
ACTTCCATGT AGTAACAGGG NTGNGCAAGG CANGNTCTCC ACCAGACTTA AAGCATTCAA 180
CCACTCTGCC CCCAGGGCGA CACTGGNCAT GTGGTTCAAT N 221

SEQ ID NO:3988
SEQUENCE LENGTH:220
SEQUENCE TYPE:nucleic acid
TOPOLOGY:linear
CLONE:HUMGS04713
SEQUENCE DESCRIPTION:
GATCTTTTTA AGATNNNAAT AAAGAGCTTT CCTAAATAAA ATAAGCAATA TTTAACTTTA 60
GAAACTGTAC TCTATGAAAC GTGTTTTATG AAANCATCTN TTACAAAAAG TGCTTACTTA 120
ACTCAGCAAG AAAGGTTTAA TGTACACATT TNACANCTAA ANTCAAAACC TACAAGAAAC 180
TGCACTGCCT TCACTATTTA CAAATCGGCC TACAAGGAAA 220

SEQ ID NO:3989
SEQUENCE LENGTH:218
SEQUENCE TYPE:nucleic acid
TOPOLOGY:linear
CLONE:HUMGS04714
SEQUENCE DESCRIPTION:
GATCTGGCTA TCTCATTGAT TATATTTTTC TTCCCTTATA CTGCAAAGTA CATCATAGGT 60
ATAATTTTAT TTTGAGAAAG AACTATATTC CAATTTTACC TATATATTCC TACTGTAATA 120
ACCAAGAGGA CATTTCAAAA ATGTATATGC TACTATTTTC AGAAGGNTAT AGATACTAAA 180
TCTTTTTAAG CCTTATTAAA CTATGTTTTA TATTGAAA 218

SEQ ID NO:3990
SEQUENCE LENGTH:217
SEQUENCE TYPE:nucleic acid

TOPOLOGY:linear
CLONE:HUMGS04715
SEQUENCE DESCRIPTION:
GATCTGTTAA ATTGGATTTT ACATGTACAT TTGAATGCCA GAATTTCTAA ATAAATCCCC 60
TGGTTAGGAA ATTTTAAAAG TCAAAGCTTG TTTTCTTCAA CCACTACCTT CTACATTGGT 120
TGACTTAGAC CGTAAGCTTT TTAAGTTTCT CATTGTAATT TACCTTCTCA TGCAGATTGC 180
TGATGTTTTA TTAAACCTTA TTTTTACAAA AATGAAA 217

SEQ ID NO:3991
SEQUENCE LENGTH:281
SEQUENCE TYPE:nucleic acid
TOPOLOGY:linear
CLONE:HUMGS04716
SEQUENCE DESCRIPTION:
GATCTTATTA GACTCATACA TCTAATTATA GAAATACATA GACTTGATAG AATTTNATTT 60
TCAGGCNTGA AGAAATATTC TTTGGAAAAG CTAAATTTTT GGTGATTGAC ATAAAGATTT 120
ACTNGCTCAT ATTANCTNGG GGGGNTAGTA CTCTCCAAGA ATTAATGTGC CCTAAAAATT 180
TTCCTCCAAA ANCTTATCCT TATCATGTGA TAATGANGAN CATTTGATTT CTTGAAAGGA 240
GACTGCTGTA GGCAGCATCT GGGAATGCAA NTNNGGGGNN N 281

SEQ ID NO:3992
SEQUENCE LENGTH:217
SEQUENCE TYPE:nucleic acid
TOPOLOGY:linear
CLONE:HUMGS04717
SEQUENCE DESCRIPTION:
GATCAGCCTG GGCAACATAG CCATAGTGAG ACACCAACTC TCTACAACAG CAAAACAAAA 60
CAAAACAAAA AAACATGCAG CTGGGCATGG TGGTNACACC TGTGGTCCTA GCTACTCAGG 120
AGGCTGAGGT GGGAGGATTG CTGGAGCTAG GATGTCAAGN TTAGTGANCG TATTCGACCA 180
CTNACTCAGC TGGNAACAGA NGAGACCTGT CTAGAAA 217

SEQ ID NO:3993
SEQUENCE LENGTH:216
SEQUENCE TYPE:nucleic acid
TOPOLOGY:linear
CLONE:HUMGS04718
SEQUENCE DESCRIPTION:
GATCATTTTT TGAGGCAGGA GCATAACACC TTCTCTAGGA TTTGTCAGCT TCTAAAAAGA 60
GCCTTTTGTA AGGACTTGTA TGAGCTTGGG TTTCACCATC ATTACTGGGG AGGCACTAAG 120
ACTGCACATA TCTCTGGGTG AAGTAATGTC TACACANCAC CTTTCATAGC AATAGTTCCC 180
AGCTAGCATA CCTGCTTCTG CCAGTCCAGG GCCAAA 216

SEQ ID NO:3994
SEQUENCE LENGTH:220
SEQUENCE TYPE:nucleic acid

TOPOLOGY:linear
CLONE:HUMGS04719
SEQUENCE DESCRIPTION:
GATCCAGACT ATAATCCTGC TGACCCAGAG AGTGACTCAG CTGACTAATG GACTGTCCCC 60
ATCTGCAGAG AGGCTTGACT GCCACAGCAT CTGTGGCTAT GCTCAGAGGG TTATGATTTT 120
CCTTTCTTTT NNNTAAGAAA AAATTATTTT CAGGAGAATA TTCTTCTGAT AGCTTTCATC 180
ATTGAACTTA ATAAACTGAC CTTAAAATTT CAAATATAAA 220

SEQ ID NO:3995
SEQUENCE LENGTH:215
SEQUENCE TYPE:nucleic acid
TOPOLOGY:linear
CLONE:HUMGS04720
SEQUENCE DESCRIPTION:
GATCTAAGGA AGAACTTGCT GCTGCAGTAT TGAAACTGTG AAGAACTGAC ATTTGAAGAA 60
AAATAGATTA CCGTTGCGGG ACTAGAATGG GCGACTGCTT GGAGCCAGTG CTTGTTTTTA 120
TCTAGGACAC TTACTGTCCT GTGAAGTAGA ATACATTTAT CTGCATTTAG TTTGTTAATG 180
TCTGAAATGA ATAAAAAGAG GAAATTGCGA TTAAA 215

SEQ ID NO:3996
SEQUENCE LENGTH:214
SEQUENCE TYPE:nucleic acid
TOPOLOGY:linear
CLONE:HUMGS04721
SEQUENCE DESCRIPTION:
GATCCAGAGA CAGAGGCAGC CTGTCTTTTC AGTTGGTTTC TGCTTTTAAT TTACTTGTAC 60
AATTCATTGT TACTGTTCTG TTTTTCCTAT TAATCTTTTG TCAACTTCCT GATTATGTAA 120
CAAAGTATGT ACAGTCTACT TTTGACCTAT TTTTATCACA GTATNATTNA TTNCTTCTTT 180
CAATAAAGTA CTNGNGCATA TTTCCACTGC CAAA 214

SEQ ID NO:3997
SEQUENCE LENGTH:214
SEQUENCE TYPE:nucleic acid
TOPOLOGY:linear
CLONE:HUMGS04722
SEQUENCE DESCRIPTION:
GATCCGNAGT TCAAGGGAAG AGGAAGTTGC CTCTNAGCTC CATCTCTCTG CGCTGCTAGA 60
CATGGTGGAC ATTTGAGCAG CCTGACCTGT GGGGAGGGGG TCTCTCCCGA AGAGTTTCTG 120
TTTTTACTCA AAATAATGTT ATTCTCAGAT GCTTGATGCA CTGTTGGAAA NGGGGATTAA 180
TTTAATCATG CAGATAAACC ATTTAAATGT CAAA 214

SEQ ID NO:3998
SEQUENCE LENGTH:212
SEQUENCE TYPE:nucleic acid
TOPOLOGY:linear

CLONE:HUMGS04723
SEQUENCE DESCRIPTION:
GATCGAAACC AGCCTGGTCA ACATGGTAAA ACACTGTCCC TACTAAAAAT ACAGAAATNA 60
GCCAGCAATA CAAAAATTGG CAGCTGTAGT CCCAACTACT TGGNAGGCTG AAGCACAAGA 120
ATCGCTTGAN CCCAGGAGGT GGGAGGTTGC AGTGAGCTGA GATTGCGCCA CTGCACTCCA 180
GCCTGGGCGA CAAAGCGAGA CTCTGTCTCA AA 212

SEQ ID NO:3999
SEQUENCE LENGTH:211
SEQUENCE TYPE:nucleic acid
TOPOLOGY:linear
CLONE:HUMGS04724
SEQUENCE DESCRIPTION:
GATCTCAGNA ATTGGAAAAA TTGTCCTGTC TGTCACTTGT TTTGTTGCCT TAATAAGCAT 60
CTGAATGTTT GGTTGTGGGG CGGGTTCTGA AGCGATGAGA GAAATGCCCG TATTAGGAGG 120
ATTACTTGAG CCCTGGAGGT CAAAGCTGAG GTGAGCCATG ATTACTCCAC TGCACTCCAG 180
CCTGGGCAAC AGAGCCAGGC CCTGTATCAA A 211

SEQ ID NO:4000
SEQUENCE LENGTH:210
SEQUENCE TYPE:nucleic acid
TOPOLOGY:linear
CLONE:HUMGS04725
SEQUENCE DESCRIPTION:
GATCAAGGCT ATGAACATCT TGTGAAGTAG GATGTTAAGT CTGCAAATAT CAGAGTGAAT 60
CCAAACTTAT CAATTCAACA CTGGATTGTA AAACAGCTCA AAAAAAGACT AAAAACACAG 120
CTCTACACTT TAGGAGGTTG GGGCAGGCAG ATTGCTCGAG CTCAGGAGTG GGAGACAAGT 180
TTAGGAAACA TGGAAAAACC CTGTCTCAAA 210

SEQ ID NO:4001
SEQUENCE LENGTH:209
SEQUENCE TYPE:nucleic acid
TOPOLOGY:linear
CLONE:HUMGS04728
SEQUENCE DESCRIPTION:
GATCAGCATT CATACCATAG TAACTGATGT TGAAAACAGC ACAAATAAAC CAGAAGTTGA 60
AAATGGCAAT AACATGCTAA AAATTTGCAA GTCAATTTGA NNACACTTGT CCAAATCTTG 120
ATTTTTTTTC CTTCTCTATC GTTCCCCCCC CACCACCAGC TTTATGGAGG TATAATTAGC 180
AAATAAAAAG TATACATATT TAAGGTAAA 209

SEQ ID NO:4002
SEQUENCE LENGTH:209
SEQUENCE TYPE:nucleic acid
TOPOLOGY:linear
CLONE:HUMGS04729

SEQUENCE DESCRIPTION:
GATCCTATGA ATTAATTCAG AAAGCAACTA AAATAACAAT GGCCTGCCAA ATGTCATTTT 60
GTAAATATAC GTCTATGACT TTAGGAGCTG TCCTGGTTTG AAAACATGAG GACAGTTTAT 120
CCATTGGATG CCATCTATNT AGTCCCAATT AAGAAAGTTG TTTTTTTGTG AGAATGACCA 180
AGGTAAATTT AAATATACCA TTCAAACAN 209

SEQ ID NO:4003
SEQUENCE LENGTH:208
SEQUENCE TYPE:nucleic acid
TOPOLOGY:linear
CLONE:HUMGS04730
SEQUENCE DESCRIPTION:
GATCGATGAA GCACTTTTTT ATTAATATTT TCCTTTGTTA AAGGAGGAAC CGTAACTCTC 60
CATAGCTGTA CATATAACCC TTTTCTCCTA AAGAGGAGTC AGTCAGTGCT CCTATATTTT 120
TCATTTTTTG TCAAAGCAAG AAGTAAATAC TTTAGAATTG TTAAATATAT AAATAAAGCA 180
AATAAAGTTG AGTGTTCCAC ATGGTAAA 208

SEQ ID NO:4004
SEQUENCE LENGTH:207
SEQUENCE TYPE:nucleic acid
TOPOLOGY:linear
CLONE:HUMGS04732
SEQUENCE DESCRIPTION:
GATCTTTCCA CATACACTAC ATTGTATTGT TCAAAATTCA TAGAAATNAC TTGCAATATA 60
TTGATTTAAG CCTTATGTAG TGACTGTTGC TATATTTAAA GTAGCATTTT NAGTTTGCTG 120
TTTTTCCTCA CTGCTTAATC CTACAAAAAC CAGCCATCTG CCTAAAAAAT AAAAGAGCAA 180
AANTGNATCA GTGCAACCGG TATACGN 207

SEQ ID NO:4005
SEQUENCE LENGTH:207
SEQUENCE TYPE:nucleic acid
TOPOLOGY:linear
CLONE:HUMGS04733
SEQUENCE DESCRIPTION:
GATCCACATC TCAAAGAAGT GGGACTTCAC CAAGGTNCAA TGCTGATGAA TTTGAACNCA 60
TGGTGGCTGA AAAGGGGCTC ATCCCANGAT GGCTGTGGGG CAAGTCATCC CAGTCGTGGC 120
CTATGGCAAG TNNNGGGCCT NACTCATGAG GGCTTCAATG TGCTGCCCCC TCTTAATACT 180
CACCAATAAA TTCTACTTCT GTCCAAA 207

SEQ ID NO:4006
SEQUENCE LENGTH:205
SEQUENCE TYPE:nucleic acid
TOPOLOGY:linear
CLONE:HUMGS04734
SEQUENCE DESCRIPTION:

GATCTACATT ATTCAGCAGC CTCGAAAATG TTAAGCCTGG ATTTAAAACA CAGCCGTCTG 60
GCCAGCTGCC TCGAATATCT GACAGCTTAG CAAAAAGGGC CANAGCTTTC CATAGGCGTG 120
CTGCACTTGC TTGGTAAATT AAGCAGCTTT TGTATCTTCC CCTTTGACTT TAGGTAATAA 180
AGCATCCAAA CTTGTAAATC TGAAA 205

SEQ ID NO:4007
SEQUENCE LENGTH:204
SEQUENCE TYPE:nucleic acid
TOPOLOGY:linear
CLONE:HUMGS04735
SEQUENCE DESCRIPTION:
GATCTGGGAT AAAACCAGAC TGAAAGGAAT ACCTCAGAAG AGATGCTTCA TTGAGTGTTC 60
ATTAAACCAC ACATGTATTT TGTATTTATT TTACATTTAA ATTCCCACAG CAAATAGAAA 120
ATAATTTATC ATACTTGTAC AATTAACTGA AGAATTGATA ATAACTGAAT GTGAAACATC 180
AATAAAGACC ACTTAATGCA CAAA 204

SEQ ID NO:4008
SEQUENCE LENGTH:203
SEQUENCE TYPE:nucleic acid
TOPOLOGY:linear
CLONE:HUMGS04736
SEQUENCE DESCRIPTION:
GATCTNCTTT TTATCACTAT AGTTTGTATT CCTAGAATTG TATATAAATG GAATCCAACG 60
GGCACAGTGA CACATGCCTG TAGTTCCAGC TACTTGAGAG GCTGAGGTGG GAGGATTCCT 120
TCAGCCCAGT TCAAGGCTGC AATGAGCTAT GACCACGCCT ATGCACTNCA GCCTGGGCAA 180
TATAACAAGA CCCTATATCT AAA 203

SEQ ID NO:4009
SEQUENCE LENGTH:202
SEQUENCE TYPE:nucleic acid
TOPOLOGY:linear
CLONE:HUMGS04737
SEQUENCE DESCRIPTION:
GATCTGAAAA GAATTTNTAT TTTAATTGGG TTGAGGGAGA GTAGATACAA TGAAACCTTA 60
AATTTCAGCT TTNATTCTAA ACCCTTTAAT TTTTTATGGG TTATGTTTTA CTTGGCAAAA 120
CTATCTGTGT TTCTNTCTTA GTGATATAGC ACCCTTAAAG TAACTTGAGT GGTAAAGGAA 180
ATAAAATACA CCAGTTTTCA AA 202

SEQ ID NO:4010
SEQUENCE LENGTH:201
SEQUENCE TYPE:nucleic acid
TOPOLOGY:linear
CLONE:HUMGS04738
SEQUENCE DESCRIPTION:
GATCCTCTTC CTGCTCCAGT TGCTGGCCGA CCACGTCCCT GGCGTTGGCC TGGTCACAAG 60

GCCGCTCATG GATTACTTGC CCACCTGGCA GAAAATCTAC TTCTACAGTT GGGGCTGACA 120
GACCTCCCGG AAGGAGGGTG TGGGGAGGGG TGGGGCAGGG AGCCCCTCTT CCCTAATAAA 180
ACTGACTCCG GCAGCGTCAA A 201

SEQ ID NO:4011
SEQUENCE LENGTH:201
SEQUENCE TYPE:nucleic acid
TOPOLOGY:linear
CLONE:HUMGS04739
SEQUENCE DESCRIPTION:
GATCCTATTT GTACAAAAAA ATGTAAAACT TAAAATTGCA CAAAATTTGT NACCTGTACC 60
AGCTTTTAGA ACTGTTTATC TTATCCTCCT CAGTGATACA TCATGAAGTT GTGTGCTTTG 120
CCTAAAATGC CCAGTTACCT GAAATTGTAT AAATNCTTGC CAAAAGTGTT TGAACTTAAT 180
ACAAACTTCC CATCTCTTAA A 201

SEQ ID NO:4012
SEQUENCE LENGTH:201
SEQUENCE TYPE:nucleic acid
TOPOLOGY:linear
CLONE:HUMGS04740
SEQUENCE DESCRIPTION:
GATCTCATTT TATTTAGCAT CAGCTCAAGA AACTNAAGTC TTAGTGCACA GTATCACAAC 60
AAAGAAAAAG CTTTGTTTTT ATAACTGGTA AAAACAAGAA AAGATTCTCA TCAAAATGAA 120
AATATAAAAT TAATCATTTC TCACCAAAGA GTATGCCTGG GAGCCTCCAG CTGTTAAAAG 180
NCAATGCTAT TACTACTTCT N 201

SEQ ID NO:4013
SEQUENCE LENGTH:200
SEQUENCE TYPE:nucleic acid
TOPOLOGY:linear
CLONE:HUMGS04741
SEQUENCE DESCRIPTION:
GATCCAAGTT GCCCTTGGGG TATAAAACTT GACAGCAGAG GGCGTTCAGG GTCCCTCAGC 60
TGCAGTGTGA AGTGGGACAC ACAGGTGAGA CTCCATCTGC CCTGGGCAGG TTCCTGAGCC 120
TTNGGGGACC AGTTCACCCT ACATCCCAGG CTTCTGTTGT CCCTTGCCTG CCTGTAAGGA 180
ATAAAGTTGC TTTGCTTAAA 200

SEQ ID NO:4014
SEQUENCE LENGTH:200
SEQUENCE TYPE:nucleic acid
TOPOLOGY:linear
CLONE:HUMGS04742
SEQUENCE DESCRIPTION:
GATCTGACCT TTTCTTCAGA TACATAAGCA TTATTTTCTC TGTTTAGGTC CAAGGAAAAA 60
CAAAAGGTAA AGGNAATAAA GGGAATATAC ATAAGGCAAA CATTTCTCTT TTTTTTTTGC 120

ATTATATCTT ACCAATTAAT TAAATTGTGG CCTTTGTTAT TATAAATGTA ATGATTCATT 180
AAACTATATT ATGTAATAAA 200

SEQ ID NO:4015
SEQUENCE LENGTH:200
SEQUENCE TYPE:nucleic acid
TOPOLOGY:linear
CLONE:HUMGS04743
SEQUENCE DESCRIPTION:
GATCCTATGA CTGACATCAA CTAAAATTTA AAAGAAGAGG AAGACTCAGT TGGGAAATTT 60
TNCCATGAGG AAAATGTGCT TTGGTGCAAG GTACAAGGCC CACACCCTCT CTGAGAGATA 120
CTATGATTAA AAAAGCTTTA TATCTTGGGA TTTTNCACAA CTGATAATGT GCAAAGNTAT 180
AAACNGATTA ACTTGTCAAA 200

SEQ ID NO:4016
SEQUENCE LENGTH:200
SEQUENCE TYPE:nucleic acid
TOPOLOGY:linear
CLONE:HUMGS04744
SEQUENCE DESCRIPTION:
GATCTTTCAA GAATGAACCA AGGNTTTTTA AAAGAATTAT AGGAAACACT TCATTCTTTA 60
TAAAACTTTC TATAATGCCT TATTTGAATG TTAATCTTAT GTGCTTTCTA AAAAATGTTG 120
TGAAATACCA AACTTATGGA TTATCACTAG GTTATCAAGC ATATATNAGT CTTTATCAGA 180
NTAAAATGAA ATTNCATAAA 200

SEQ ID NO:4017
SEQUENCE LENGTH:199
SEQUENCE TYPE:nucleic acid
TOPOLOGY:linear
CLONE:HUMGS04745
SEQUENCE DESCRIPTION:
GATCTGAGAG GCAGTAAAGC AGTGATGAAG ACAGTGCAGC AGAACATTGA AGTCCTGATT 60
GCAGCAGTGA CTCTGCAACC TTGGATAAAT TATTTCANTC TTTATTTTGT AAAACGGAGA 120
TAACGGCCTA TAGGATTNTN AAGACTGANT ATTTTAAAAG NGCTTAGAAT GGTGCCTGAC 180
AAATGAGNNN CANTAAATN 199

SEQ ID NO:4018
SEQUENCE LENGTH:199
SEQUENCE TYPE:nucleic acid
TOPOLOGY:linear
CLONE:HUMGS04746
SEQUENCE DESCRIPTION:
GATCATGTGA TTTCTATTTC TNCCCCACAG GGTAAGGGAC GAGTCTTCTG GAAGGCTCTG 60
CCATGGACAT TTGTCCTCGG GCTCAGAGGC CCCACCCTGC CCCACACCTG CCCCTAATCA 120
CTGCAGTGTC CAGCCCAGTG TTGAACAGAT TGTAGCGTTC TGTCTCATTA CGAGCAAATA 180

AATAGACTTT CATTGGAAA 199

SEQ ID NO:4019
SEQUENCE LENGTH:198
SEQUENCE TYPE:nucleic acid
TOPOLOGY:linear
CLONE:HUMGS04747
SEQUENCE DESCRIPTION:
GATCACGCGG CATGTATTGA GGTGTGGCAT GCAGCATTTT GGAAGGAAAA TTGAAGACGT 60
GTTCAAGAAA ACATGAACAG AAGCAAATNA TGAAAATGAG CATTTTACTT GATGTTGATA 120
ACATCACAAT AAATTATGGA GAAAAATACA TATTTGGCTA ACTTTTAATT GCTGANCAAT 180
AAAGTGTTTT CTTTTAAA 198

SEQ ID NO:4020
SEQUENCE LENGTH:196
SEQUENCE TYPE:nucleic acid
TOPOLOGY:linear
CLONE:HUMGS04748
SEQUENCE DESCRIPTION:
GATCCCTACC GCCAGCTGCG AGAGAAGGAC CCCAAGTACA GTGCTCTCCG CCAGAATTTC 60
TTCCGCTACC ATGGGCTGTC CTCTCTTTGC AATCTGGGCT GCGTCCTGAG CAATGGGCTC 120
TGTCTCNCTG GCCTTGCCCT GGAAATAAGG AGCCTCTAGC ATGGGCCCTG CATGCTAATA 180
AATGCTTCTT CAGAAA 196

SEQ ID NO:4021
SEQUENCE LENGTH:196
SEQUENCE TYPE:nucleic acid
TOPOLOGY:linear
CLONE:HUMGS04750
SEQUENCE DESCRIPTION:
GATCATGAAG AAGCAGGGCC TCTACCTACA AAAGTNAATC TTGCTCATTC TGAAATTTAA 60
GCATTTTTCT TTTAAAAGAC AAGTGTAATA GACATCTAAA ATTCCACTCC TCATAGAGCT 120
TTTAAAATGG TTTCATTGGA TATAGGCCTT AAGAAATCAC TATAAAATGC AAATAAAGTT 180
ACTCAAATCT GTGAAA 196

SEQ ID NO:4022
SEQUENCE LENGTH:195
SEQUENCE TYPE:nucleic acid
TOPOLOGY:linear
CLONE:HUMGS04751
SEQUENCE DESCRIPTION:
GATCTGGTTC CAGGGGAAAA AAATAGCTGG TTGGTGTCTA GCCCCCCAAC ACTTTTGTCT 60
GTTGTGTATA AAAGAAGAAA GACTGGCATG TACCTTCATT TGCTTAGCTA TTTGAGTATC 120
TAGAGAAAAA TTAAAATGCA ATGAGTTAGC AGTATACCCT GGCACACTTA ATAAATTAAA 180
CATTTGTGGA GCAAA 195

SEQ ID NO:4023
SEQUENCE LENGTH:194
SEQUENCE TYPE:nucleic acid
TOPOLOGY:linear
CLONE:HUMGS04752
SEQUENCE DESCRIPTION:
GATCTAGAAG CTGGTAAAAA TGACAATATC AATCACATTA GGGGAACCAT TGTTGTCTTC 60
ACTTAATCCA TTTAGCACTA TTTAAAATAA GCACACCAAG TTATATGACT AATATAACTT 120
GAAAATTTTT TATACTGAGG GGTTGGTGAT AACTCTTGAG GATGTAATGC ATTANTAAAA 180
ATCAACTCAT CAAA 194

SEQ ID NO:4024
SEQUENCE LENGTH:193
SEQUENCE TYPE:nucleic acid
TOPOLOGY:linear
CLONE:HUMGS04753
SEQUENCE DESCRIPTION:
GATCCAACAG CAACACCATT TTTAAATTAT TGTGAAAAGA TTAACTGGCA ATGTACAGAG 60
TTTACTCAAA GCCCTTCTTA AGGGAAAACA CTACAAAAAG TCACAAGGAT ACCAAATGGA 120
AACACATGAT GATGCCTCTG GGTCTGTATG AGACCGTGAT GAAGTAGAAA TAAAGCCCTT 180
CTGAGATGGC AAA 193

SEQ ID NO:4025
SEQUENCE LENGTH:191
SEQUENCE TYPE:nucleic acid
TOPOLOGY:linear
CLONE:HUMGS04754
SEQUENCE DESCRIPTION:
GATCCACGGT GGGCTCCAGC TGCCAAGNCA CCCAAGGGAG TCTGGGCCCT AGGCCTAGCC 60
CCATCCCTCC CCATGAGGGG CCAAGACACT GCCTAAGGTG TGGGAGGGAC TGGCTGAGAT 120
TGCAGCCCAT GGTAGGAGCT GGACCAACTG TATATAGTTT TCAATAAACT TTTTCCTTTT 180
CTGTTCTCAA A 191

SEQ ID NO:4026
SEQUENCE LENGTH:264
SEQUENCE TYPE:nucleic acid
TOPOLOGY:linear
CLONE:HUMGS04755
SEQUENCE DESCRIPTION:
GATCCAGGAA GTTGGTGAAC CATCTAAAGA AGAGAAGGCT GTGGCCAAGT ATCTTCGATT 60
CAACTGTCCA ACAAAGTCCA CCAATATNAT GGGTCACCGG GTTGATTATT TNATTGCTTC 120
AAAAGCAGTG GACTGTCTNT TGGATTCAAA GTGGGCAAAG GCCAAGAAAG GNGAGGAAGC 180
TTTATTTACA NCCAGGGAGT CTNTGGTTGA CTACTGCAAC CAGGCTTTTA AAGTAGCAGT 240
TTNTTTCACC GAGCCCTAAA GGTN 264

SEQ ID NO:4027
SEQUENCE LENGTH:186
SEQUENCE TYPE:nucleic acid
TOPOLOGY:linear
CLONE:HUMGS04756
SEQUENCE DESCRIPTION:
GATCTTCACA TATTTTATAT GNATATATAT ATCAAGAAGT TATATATATA TAACTCAAGA 60
AACTCAAGTT ATATATATAT GTCAAGAAAT GTATGATTAT TTTGGAGAGA ATGGGCCCAA 120
ATGTGAAAAA GATATAAAAA AAACAAAAAA AACNNAAAAA AAACCNCTNT TTNCCCCTNC 180
GNANTN 186

SEQ ID NO:4028
SEQUENCE LENGTH:150
SEQUENCE TYPE:nucleic acid
TOPOLOGY:linear
CLONE:HUMGS04757
SEQUENCE DESCRIPTION:
GATCTGGAAG TCACCCTCCT CTGGCCCACG GAAAATTTTG GTAATCTTCT AGGTTCTAAA 60
ATGAAGATGT ATGGGTACTC TGGCAGACTG CATGTTGTAT AATTTGAAAA ATACTAAAAG 120
TGGAAAATAA AATTGAATTA AACTTTGAAA 150

SEQ ID NO:4029
SEQUENCE LENGTH:186
SEQUENCE TYPE:nucleic acid
TOPOLOGY:linear
CLONE:HUMGS04758
SEQUENCE DESCRIPTION:
GATCAACAAG TTCAAAACAC TGCTAAGTCT AAATCTAAAA CAGACCAAGA GTACCTTCAT 60
TTAANTTTAG CTTGCACAGA GCTTGATGCC TATCCTNCAT TCTTTNCAGA AGTAAAGATA 120
ATTATGGCAC TTATGCCAAA NTTCATTATT TAATAANGTT TTACTTGAGG TAACATTACT 180
GATTTN 186

SEQ ID NO:4030
SEQUENCE LENGTH:183
SEQUENCE TYPE:nucleic acid
TOPOLOGY:linear
CLONE:HUMGS04759
SEQUENCE DESCRIPTION:
GATCCAACAC AGAGACAATA GAAACATTGG GATTGAAGAG ATTCTTGGGA CTGTAAAAAT 60
CCCAAGAATA TGGGATTGTA AAAAACCTGT CATCTTACTG CTATGATAGA TGAAGATTTT 120
GCTTGCCTAT ACCTTGTNGC TATTCCTCTC TTCCTTTCCT CCAAATATAC TTGTAGGTAT 180
AAA 183

SEQ ID NO:4031

SEQUENCE LENGTH:183
SEQUENCE TYPE:nucleic acid
TOPOLOGY:linear
CLONE:HUMGS04760
SEQUENCE DESCRIPTION:
GATCTAGCAT TTTAATTTCT AGCATTGCTA TTCACCGGCT TCCTTATTTT ATATGTAAAA 60
ATTAAGATTT TATATTTAAT CTTCTTGTTT CTCATAGATA TTTTGTGAGC ATTTTNTTGT 120
TTATTTTGAA GAAATGTGGA TAAGATACTT GGTAGTATAA AACAGNCTCT CTGAGAGTAT 180
TTN 183

SEQ ID NO:4032
SEQUENCE LENGTH:182
SEQUENCE TYPE:nucleic acid
TOPOLOGY:linear
CLONE:HUMGS04761
SEQUENCE DESCRIPTION:
GATCAAGGTC TGTATATTAT CCCAAACTTT ATTAAGAATT GTTTTCTAAT TNGTTATAAC 60
ATTTTTNAAT TAATAGTTTC AAAACAAATT GTTAATACAA CTGTATAAAA TGAACATAAT 120
TTTCCTCACT TGTATTTTTG TTATTGAGCA AGTTTATCAA AATAAATNGT CTACTAAAGA 180
AA 182

SEQ ID NO:4033
SEQUENCE LENGTH:181
SEQUENCE TYPE:nucleic acid
TOPOLOGY:linear
CLONE:HUMGS04762
SEQUENCE DESCRIPTION:
GATCTCTTAT CAATTAGCAC TCACTAATGT ACTACTAATT GAGCAACCTA CGCACTCAGT 60
TGACTACGTA AATCTGTCAG GTCTTTTGTG ATTTCCTCTG ATGCAGGAGA GGAAAAATTG 120
TAATTGATGA AAATAATGAA TGAAAATCAA CAGATGAATA AATGGTTCTT TATAAGTGAA 180
A 181

SEQ ID NO:4034
SEQUENCE LENGTH:180
SEQUENCE TYPE:nucleic acid
TOPOLOGY:linear
CLONE:HUMGS04763
SEQUENCE DESCRIPTION:
GATCTGGAGT CCCACGTTCC CCAAGGCCAG CGGGATGTGT GCCCCTCCTC CTCCCAACTC 60
ATCTTTCAGG AACACGAGGA TTCTTGCTTT CTGGAAAAGT GTCCCAGCTT AGGGATAAGT 120
GTCTAGCACA GAATGGGGCA CACATTAGGT GCTTAATAAA TGCTGGATGG ATGCAGGAAA 180

SEQ ID NO:4035
SEQUENCE LENGTH:180
SEQUENCE TYPE:nucleic acid

TOPOLOGY:linear
CLONE:HUMGS04764
SEQUENCE DESCRIPTION:
GATCCTGTTT GTACAAAGTC TTGCTTTTAT AAGGTTTCAA TAATATCTAA AACAACACAT 60
TAAAAAGCTG AGACCATTTT ATGAAGATAA TTGTTTGTAA TCATAGGTGT TGAAAGTAAA 120
AAGGTGCCAT CTTGTGGTAT TGACTTGTAT TTATAACAAA TAAACTGCTC AAGAGACAAA 180

SEQ ID NO:4036
SEQUENCE LENGTH:180
SEQUENCE TYPE:nucleic acid
TOPOLOGY:linear
CLONE:HUMGS04765
SEQUENCE DESCRIPTION:
GATCCTGGTT ACAGCCATTT TGTGTGATTC ACTTCGGGGG TTAAGTAATG CAGGATTCTG 60
CAAACAAGGT GTCGCCGTCC AAATGTACTG TCCTGGCATA GAGAGCACTG CTTTGTTTTC 120
CACTGTTGTA GAGAAAACTA GGGAGAACTT TATTTTTCAA TAAACTTTTC TTGTGTGAAA 180

SEQ ID NO:4037
SEQUENCE LENGTH:178
SEQUENCE TYPE:nucleic acid
TOPOLOGY:linear
CLONE:HUMGS04766
SEQUENCE DESCRIPTION:
GATCATGTAT AAAAAGAAAA AAAAGAAGTC ATGCTNTGTG GCCAATTATA ATTNTTNNCA 60
AAGACTTTGT CACAAAACTG TCTATATTAG ACATTTTGGA GGGACCAGGA AATGTAAGAC 120
ACCAAATCCT CCATCTNTNC AGTGTGCCTG ATGTCACCTN ATGATTTGCT GTTACTNN 178

SEQ ID NO:4038
SEQUENCE LENGTH:177
SEQUENCE TYPE:nucleic acid
TOPOLOGY:linear
CLONE:HUMGS04767
SEQUENCE DESCRIPTION:
GATCAGGATT TGGGAAAATT GTGGTCAATA TTTGATTTTC CTTTTCTGTT TGATAGCCTT 60
ATCTAAATTC CCCTAGCTTG AATGTAGTAA ATATGAGTGT GTAAAAATGT GTATCTAATT 120
CACCTCTTNG GGGAGGTTTA ATGTAACTAT AAATAAAAAT TCTTTCCCCA GGTTAAA 177

SEQ ID NO:4039
SEQUENCE LENGTH:175
SEQUENCE TYPE:nucleic acid
TOPOLOGY:linear
CLONE:HUMGS04768
SEQUENCE DESCRIPTION:
GATCTTACCT CCTAACCAAC TCCATACATC ATGACCAATG GCTCAGTCCA AGCNGTCATC 60
TCTTGCTTGG TCACTGCAGG TACTTGCTAA CTTTCTTCCC AATAATCCAA CCCCCACAGA 120

GCAGCCAGAG TAGCCTTTTT TTGTTTTGTT TTGTTTTGTT TTGTTTTGTT TTGTN 175

SEQ ID NO:4040
SEQUENCE LENGTH:175
SEQUENCE TYPE:nucleic acid
TOPOLOGY:linear
CLONE:HUMGS04769
SEQUENCE DESCRIPTION:
GATCTTAATA GCTAAAATAT GAAAAATATT TGGNAAGTCT GAAATNAGGT CTCCTGGCCC 60
TGGTGTGCCC TTAATGCCTG TNACAGTTGG CCTCTGTGAA TATTGGTATA ATTNTAAATA 120
ATGTCAAACT CCATTTTNTA GCAAGTATTA ATAATTAAGG GAAGTATGTC TGAAA 175

SEQ ID NO:4041
SEQUENCE LENGTH:174
SEQUENCE TYPE:nucleic acid
TOPOLOGY:linear
CLONE:HUMGS04770
SEQUENCE DESCRIPTION:
GATCCTCAGG ATATGCAGGG CTCAAGAGAA ATGAAATATC TTGGGGATAT GGTTACTTAA 60
GACGTGATAC TGTCACAGTG AATTTCACAG AAGATGGTGC TGCCCTAACT GAGGTTCAAT 120
CCATGTATAA GCCAGACCAA TCTGAAGACA AATAAAGTAT TTCACAGCCA CAAA 174

SEQ ID NO:4042
SEQUENCE LENGTH:171
SEQUENCE TYPE:nucleic acid
TOPOLOGY:linear
CLONE:HUMGS04771
SEQUENCE DESCRIPTION:
GATCTTCAAA CATTAACTTA AGCAGACCAA AAATTCTGAT GACCGCATCT AGATTATTTT 60
TTTATAAAAA TGATTTTCAC TATAGCTATG TTACGCTAAG CTACTGTCCA ATCTCTTGTG 120
ATGTGTAACT TTTACATGTG AATATTAAAG TAGATTTCTC TGTCTTGTAA A 171

SEQ ID NO:4043
SEQUENCE LENGTH:170
SEQUENCE TYPE:nucleic acid
TOPOLOGY:linear
CLONE:HUMGS04772
SEQUENCE DESCRIPTION:
GATCTTTAAA GATGTGCTAA ATGACTTTTT TGGCCAAAGG CTTAGTTGTG AAAAATATAA 60
TTTTTAAATT ATACATTCAA GGTAGTGGCC AAATGTAACA CATCAATCAT GGAATGATTN 120
CTCTGCTAAC AGCCGNCTGT ATGTTTCAAT NAATTTGTCC AAAGCTCAAA 170

SEQ ID NO:4044
SEQUENCE LENGTH:169
SEQUENCE TYPE:nucleic acid

TOPOLOGY:linear
CLONE:HUMGS04773
SEQUENCE DESCRIPTION:
GATCCTTCCC AGTGGACAGA TGAAGAATTA GGTATCCCTC CTGATGATGA AGACTGAAGN 60
TGTAGACTCA GCCTCACTCT GTACAAGAGC CAGGTGAGAA TTTCAAGGAT TATCGACTTC 120
ATATTGCACA TTAAAGTTAC AAATTAAAGT GGCTTGGTCA AGAATGAAA 169

SEQ ID NO:4045
SEQUENCE LENGTH:169
SEQUENCE TYPE:nucleic acid
TOPOLOGY:linear
CLONE:HUMGS04774
SEQUENCE DESCRIPTION:
GATCTAGTGT GTAGTGAGCT CAGTATAGAG GACAGAGCTT TCATCATGTA AATTNATATG 60
CAAAGCCAAA TNAAACGTGG TGTGTACTTC CTACTGATGC GTCAGGGTAT AGGCCTCTTT 120
TTCTGCCATT CAAATAATGG AAGTTAATTA AATTTTAAAC AAATGTAAA 169

SEQ ID NO:4046
SEQUENCE LENGTH:169
SEQUENCE TYPE:nucleic acid
TOPOLOGY:linear
CLONE:HUMGS04775
SEQUENCE DESCRIPTION:
GATCCATACT GAAAAAATNC TGAGCCAGCC ATCTTTGGCA AAGNNCCCTG AGCTCTTGCT 60
ATCTCTCAAG AGTGCTGAGA NCCACGGTGA AAGTNCTGCT CTAGGCCCAC AAGTGTAACT 120
ATGCTGTTAA CAGCTGTCAA TAGATAATTA AAATTCATAC TGTATGAAA 169

SEQ ID NO:4047
SEQUENCE LENGTH:168
SEQUENCE TYPE:nucleic acid
TOPOLOGY:linear
CLONE:HUMGS04776
SEQUENCE DESCRIPTION:
GATCTCTGTT CTNGNTTGAA TGTATTTAAT TTCCAGCAGA ATGAGCCCCA TTCCTTATTT 60
TGATTGGCCA TTTATCATGT ACATATGGTG AAATGCCTAT TCGTGACTTA GCCAATGTTG 120
TTTCTTTTTC TTACTGATTA CTACAGTACA TTTTTATATG AAAGCAAA 168

SEQ ID NO:4048
SEQUENCE LENGTH:212
SEQUENCE TYPE:nucleic acid
TOPOLOGY:linear
CLONE:HUMGS04777
SEQUENCE DESCRIPTION:
GATCTGCTGA AGCATCTGTC CAGCTGGTAT CCTGTGAAAG TTTGTAATTT NCTGAGTAGA 60
CATTCTTATA GAGTATTGTC TTTAAAATCA GATTGTCTCT NCTATATTGA AAGCATTTNN 120

ATGTTTNCNA ATTTAAAAAT TAATATTTCC TTATAGATAT TGTGCAATAA AGCTGAAGTA 180
GAATGTGTGG TTTTTGCAAA TNCTTTAGCG GN 212

SEQ ID NO:4049
SEQUENCE LENGTH:167
SEQUENCE TYPE:nucleic acid
TOPOLOGY:linear
CLONE:HUMGS04778
SEQUENCE DESCRIPTION:
GATCTTGTCT CACAAAATAA ATTAAAAAGA TTTTTAAATA GCAGGTTTCT AAAACAATGA 60
GAAAGACAGC ATTACATATC AGTGTCTACA ATTTAAACAA AGCTATACTT AGAAGTAAAT 120
TCATTGTCTT AACTGCTTTT GTTCCAATAA AACAAAATAG CAGGAAA 167

SEQ ID NO:4050
SEQUENCE LENGTH:176
SEQUENCE TYPE:nucleic acid
TOPOLOGY:linear
CLONE:HUMGS04779
SEQUENCE DESCRIPTION:
GATCGTTACT ATGTGTCAAA GGGTTTGGAG AACATTGATT AAGGAAGCAT NTTCCTGATT 60
GATGAAAAAA ATAACTCAGT TATGGCCATC TACCCCTGCT AGAAGGTTAC AGTGTATTAT 120
GTAGCATGCA ATGTGTTATG TAGTGCTTAA TAAAAATAAA ATGAAAAAAA TGCAAA 176

SEQ ID NO:4051
SEQUENCE LENGTH:166
SEQUENCE TYPE:nucleic acid
TOPOLOGY:linear
CLONE:HUMGS04780
SEQUENCE DESCRIPTION:
GATCTATAAC TGTACTTTGC CTGGCGCTGT GCGAAGGTCA GAAAACTTAC TGCTAGTACC 60
TAGAAACACA CAAGGCTGCC CAGCCAAATC TTAATGTAAA GTAGCTAGAG CCATGGAAGT 120
ACAGTATGAA TTAAAAAGAA AAANGTATTG ANCTTCAAAT TCTAAA 166

SEQ ID NO:4052
SEQUENCE LENGTH:163
SEQUENCE TYPE:nucleic acid
TOPOLOGY:linear
CLONE:HUMGS04781
SEQUENCE DESCRIPTION:
GATCTTGTAA GTAGGAAAGC TGTAACTAAA AATTGTATTG TTTGCTTATT AGCCATGTAT 60
CTCTTAAAAT TTTGTTATGT TTACAACGAT GTACCTTATT GGCAACAAGT TATTAGTTTG 120
ATGTTTAACA ATAGTGCCTT TAGTAAATNA TTTTACAACT AAA 163

SEQ ID NO:4053
SEQUENCE LENGTH:162

SEQUENCE TYPE:nucleic acid
TOPOLOGY:linear
CLONE:HUMGS04782
SEQUENCE DESCRIPTION:
GATCCAAAAG CTGGCTNTCT AACCACCAGC CAAGGGCTCT CTTCACCACC AAGCCAACTC 60
TCAGGGCAGC AGCAATGTGT ACCTTATTAA CCTCTGCACC CNAGGTCCTA GCACAGGGCT 120
NGTCCATAAC AGGCGNCCAA TAAACATGTG TCATAATCTA AA 162

SEQ ID NO:4054
SEQUENCE LENGTH:161
SEQUENCE TYPE:nucleic acid
TOPOLOGY:linear
CLONE:HUMGS04783
SEQUENCE DESCRIPTION:
GATCCCTGGT CCTGCCGACA CGCAGCTACT GAGAAGACCA AAAGAGGTGT GTGTNTGTCT 60
ATGTGTGTGT TTCAGCACTT TGTAAATAGC AAGAAGCTGT ACAGATTCTA GTTAATGTTG 120
TGAATAACAT CAATTAATGT AACTAGTTAA TTACTATGAA A 161

SEQ ID NO:4055
SEQUENCE LENGTH:159
SEQUENCE TYPE:nucleic acid
TOPOLOGY:linear
CLONE:HUMGS04784
SEQUENCE DESCRIPTION:
GATCGGCACC ACACCAGACA TAATCCTGGA CGACTTGCTG GAGACGGACC GCGTCACCGC 60
CCACTTCTAG CCCCGTGGAT GCCGTCACCA CCAGCACACG GAACTACCTC CCACCCCCTT 120
TTTGTACAAA ACACAAGGAA AAACATTTTT TGCTTGAAA 159

SEQ ID NO:4056
SEQUENCE LENGTH:159
SEQUENCE TYPE:nucleic acid
TOPOLOGY:linear
CLONE:HUMGS04785
SEQUENCE DESCRIPTION:
GATCTAATTC TTTTGATAAA GTTCTAGCTC TAAAAGTGAT AGTGGGACTG TATGTTTTCT 60
GATACTGGTG GCTTATGTTA TTAANCCTTT TTTAAAAAAG GTCCACTCTA AAGGCTGANN 120
NCCACCCTAN GTTTCCAGCC TCCTGGCCTC NACCNGGGN 159

SEQ ID NO:4057
SEQUENCE LENGTH:158
SEQUENCE TYPE:nucleic acid
TOPOLOGY:linear
CLONE:HUMGS04786
SEQUENCE DESCRIPTION:
GATCTTGTGT TCTTTGTGCC AATATGAAAA GGAGAGGGTT GGTTCTTTCC TTTATTGTTG 60

AATGCTCCCA TTTAATGCTT TATGGCTTTT ACTGTATTAC TTTTTTAGAC TCCCGTCTGC 120
ACAAAATGCA ATAAAAATAA TTTTATTATA CCCTTAAA 158

SEQ ID NO:4058
SEQUENCE LENGTH:158
SEQUENCE TYPE:nucleic acid
TOPOLOGY:linear
CLONE:HUMGS04787
SEQUENCE DESCRIPTION:
GATCACACTG GGGAAAAGTG ACAGAACCAA ATTTCAAACT CCAGTCTTTC TGCTTCTAGA 60
GATTGAACTA CTAACATCTG CATTATATAA AATTAAGTNT CTCAAGGTAG TTTGTGAACG 120
TAACATACAA ATAAGTGATT AAAATTTTNG GTGATAAA 158

SEQ ID NO:4059
SEQUENCE LENGTH:308
SEQUENCE TYPE:nucleic acid
TOPOLOGY:linear
CLONE:HUMGS04788
SEQUENCE DESCRIPTION:
GATCCCAAGT GATTAAAATA GGGTTTCTGA ATGGATGTTG AATGGCGTGG ACTCGCTACT 60
CCGTTCTTCA CAGCTGCCTT CCAGAATGTG TTCAAAAGAA AGACAAGAAG GAGTGTATGG 120
CTTATAAAGT GAATCTAATA CAGTATTTGT TGCATTTAAA CAAACTAGAC ATTTTCTTAC 180
GGAAAANTTA TGAAATACAG CATATTTNAT GTNCTCCCAT TGACTCAATC ATGACAATAT 240
TTCTGCTTTA ACACCATCTT TCATGATTAG NAATGTTTGT NATTGGAAAT GTTACACCAT 300
GTAAATAN 308

SEQ ID NO:4060
SEQUENCE LENGTH:155
SEQUENCE TYPE:nucleic acid
TOPOLOGY:linear
CLONE:HUMGS04789
SEQUENCE DESCRIPTION:
GATCAGCCCC AGATTTGACG TGCACTCAAA GACCTGGAAA AATGGCAGAA TAATCTGCTT 60
CCATCCCGCC AGTTTGGTTT CATTGTACCT GACCACCCTC ACNGGCAANC ATGGACCCAT 120
GAAGNAGCAA NNCGGAAACN CACNAGAGGG AAAAN 155

SEQ ID NO:4061
SEQUENCE LENGTH:155
SEQUENCE TYPE:nucleic acid
TOPOLOGY:linear
CLONE:HUMGS04790
SEQUENCE DESCRIPTION:
GATCTCATTA GTATGTTTTT TAATGTGTAG AATTAACACT TTAAAATAAA CAGGTGTGAA 60
AATTATAAAG GAATATATAT GACAATTATT TNCTGTACAT TAATGTCTAA ACATTATACT 120
TACTTTTCCA TAATAAAGGA AATTTTGGAA CCAAA 155

SEQ ID NO:4062
SEQUENCE LENGTH:155
SEQUENCE TYPE:nucleic acid
TOPOLOGY:linear
CLONE:HUMGS04791
SEQUENCE DESCRIPTION:
GATCTTAAAA CCCAAGACAT TATATTTGAA GGGGCACAGC AAACAGTGCA AGAAAATCTG 60
CTGATGGAAG ATNATTTTNA TCAGTCCTCA GANCCAGAAA CTATAAAACT GGGAAGGAAA 120
GTTTAAATAA AGANTTTATT TCCAAATTCA GCAAA 155

SEQ ID NO:4063
SEQUENCE LENGTH:153
SEQUENCE TYPE:nucleic acid
TOPOLOGY:linear
CLONE:HUMGS04792
SEQUENCE DESCRIPTION:
GATCTAAGNA GGATTCAACA CCTTTNTTTG GTTGCTCAGT GTGTAATGAC TGAGATTTGT 60
AAATCTTTGT GAACATTCTG TACTGGTTCC CAAGAGCTAT TCATTCCCTG CTACCTGATT 120
TCAGCACAAT AAATATACTT CTGCTGTGGG AAA 153

SEQ ID NO:4064
SEQUENCE LENGTH:152
SEQUENCE TYPE:nucleic acid
TOPOLOGY:linear
CLONE:HUMGS04793
SEQUENCE DESCRIPTION:
GATCTTAGCT GTATGGAGTA ACTATTTCAG AAAACCCTAT AAGAAGTTCA TTTTCTTTCA 60
AAAGTAACAG TATATTATTT GTACAGTGTA GTATACAAAC CATTATGATT TATGCTACTT 120
AAAAATATTA AAATAGAGTG GTCTGTGTTA AA 152

SEQ ID NO:4065
SEQUENCE LENGTH:149
SEQUENCE TYPE:nucleic acid
TOPOLOGY:linear
CLONE:HUMGS04794
SEQUENCE DESCRIPTION:
GATCAGAGAA GGCTGAAGAA GGTGGAGAAA CTNAGGCACA AAAAGAGGGC AGTGAAGATG 60
CGGGCAACCT CCCTGAAGCC CAGGAGAAGA ATGAAGAAGA AGGAGAGACA GCCACAGAAG 120
AGACGGAAGA AATAGCCATG GAGGGTGCN 149

SEQ ID NO:4066
SEQUENCE LENGTH:149
SEQUENCE TYPE:nucleic acid
TOPOLOGY:linear

CLONE:HUMGS04795
SEQUENCE DESCRIPTION:
GATCAACTAT GGGTTTATAT AAATNCCAAA TTTAGTACAA CAAATTTAGG TCATCAGGTG 60
AATACAAGTA TTTTTTTTAA CAGGNAACAT TTTATTATAN NAAAAATATT AATGATAAAC 120
GCATATTGGT TGAAAAATAT TTTTTAACN 149

SEQ ID NO:4067
SEQUENCE LENGTH:148
SEQUENCE TYPE:nucleic acid
TOPOLOGY:linear
CLONE:HUMGS04796
SEQUENCE DESCRIPTION:
GATCTCTGTG ACCCATACCC TATTTNTACA CTCCCTCCCC TTTTGAAAAT CACTAATAAA 60
AAATTTNCTG GTTTTATGGC TCAGGGGGCA TCACAGAACC TGCCAACATG TGATGTCTGC 120
CCTGGACACC CAGCTTTAAA ATTTCAAA 148

SEQ ID NO:4068
SEQUENCE LENGTH:147
SEQUENCE TYPE:nucleic acid
TOPOLOGY:linear
CLONE:HUMGS04797
SEQUENCE DESCRIPTION:
GATCATTTTC TTGGGACCTT GAACTGTGAA TGTTTTGTCC TAACCATTTA ATATTTTCTA 60
GGTACTTGCT GCAAGTNCTT GAACTATTTT ACCAGCTTTA ACTTTGGGGC TCTTAGNTTC 120
TNTNCTCCAG ATTCTTGTTA TNNNATN 147

SEQ ID NO:4069
SEQUENCE LENGTH:148
SEQUENCE TYPE:nucleic acid
TOPOLOGY:linear
CLONE:HUMGS04798
SEQUENCE DESCRIPTION:
GATCAGAGAA GCAGATATTG ATTGAGACGG ACAAGTCAAC TATGAAGAAT TCGNACAGAT 60
GATGACTGCA AAATGGAAGA CCTACTTTCA ACTCCTTTTT CCCCCCTCTA GAAGAATCAA 120
ATTGAATCTT TTACTNACCT CTTGCAAA 148

SEQ ID NO:4070
SEQUENCE LENGTH:149
SEQUENCE TYPE:nucleic acid
TOPOLOGY:linear
CLONE:HUMGS04799
SEQUENCE DESCRIPTION:
GATCTTTCAC ACACTGGTGA CCCTGAGAGA GGAGGGAGGA GGGAACCTGG CGGGGGTGTC 60
TGAGGCCGCA CTGTCAGCTG GCCGGTCCAA GCCTGTGGCT GGAGCTGGGG TCTGTTTACC 120
TAATAAAGTC CCACAGGTGC CTCATTAAA 149

SEQ ID NO:4071
SEQUENCE LENGTH:146
SEQUENCE TYPE:nucleic acid
TOPOLOGY:linear
CLONE:HUMGS04800
SEQUENCE DESCRIPTION:
GATCCCCTTC AAGTCTCTCT GACCTTTCTT CTGCCCTCCA GCCTGGGTGA CAGAGCTAGA 60
GTCTGTCTCA AATAATAATA ATAATACAAT AAATAAATAA ATAAATAAAT AAATAAATAA 120
ATAAATAAAG TACGCTTTCC ATTAAA 146

SEQ ID NO:4072
SEQUENCE LENGTH:146
SEQUENCE TYPE:nucleic acid
TOPOLOGY:linear
CLONE:HUMGS04801
SEQUENCE DESCRIPTION:
GATCTCATAG AGCCCAGTTC TTAGAGTCAA CTAAAGAGTT GATAGGAATT TACTAGGTCC 60
AGGGAGAAAA GGCAGTGGTT GGGGTTACTG GAAATTTTGC TCATTTTCTC TAATGACTGT 120
ATGAATAAAA GTGAACTTAC TTGAAA 146

SEQ ID NO:4073
SEQUENCE LENGTH:146
SEQUENCE TYPE:nucleic acid
TOPOLOGY:linear
CLONE:HUMGS04802
SEQUENCE DESCRIPTION:
GATCTGAACT TACAGACAGT TGCCAAATTT CTCTAGATAA GAAAATAAAG AATAGAGGGA 60
ACCTAACTGG ATAACAAAAA AAAAGGAGAG AGAAAAATGC CATTGCAGTT AAAGGTTATT 120
AAATAAAGCT TAGTGTCAAT GTCAAA 146

SEQ ID NO:4074
SEQUENCE LENGTH:146
SEQUENCE TYPE:nucleic acid
TOPOLOGY:linear
CLONE:HUMGS04803
SEQUENCE DESCRIPTION:
GATCAGGAGG GACCACAAGG ACAGGAGAAT TAGCAACGAG AGACTGGCTG GCACCAGTNT 60
TGGGAAAACT TCAGAGTGAG CTCCATTCAT GGTCTATTAA AAGGCCTTTN TTGTCATTCA 120
GTGATATGTG NCTGGCCCCT GAGAAA 146

SEQ ID NO:4075
SEQUENCE LENGTH:144
SEQUENCE TYPE:nucleic acid
TOPOLOGY:linear

CLONE:HUMGS04804
SEQUENCE DESCRIPTION:
GATCAAAAAA TTATTTTTAT TTATCCTAGC TTTACGTGCA TCTCACGGAA ACAGGATACA 60
CGGTGTATAT ATTATNATGC AGTTCGTTGG AACTAGAATT TCAAAAAATT TTAATAAATA 120
TGTTTAATCT GTTGCCTCTG GAAA 144

SEQ ID NO:4076
SEQUENCE LENGTH:143
SEQUENCE TYPE:nucleic acid
TOPOLOGY:linear
CLONE:HUMGS04805
SEQUENCE DESCRIPTION:
GATCACTGGG CCTACCTGCA CTGTGGGGCC AAGCCCTGTC TTTTTCCCAA GCCCTCAAGC 60
ACACGCATGA ATGTTCATCC CGACTTGGTA GGGGGCTTTT CACCCTTACA AGATGGCAAA 120
AGATTCACAT TGCTTTTTTT AAA 143

SEQ ID NO:4077
SEQUENCE LENGTH:143
SEQUENCE TYPE:nucleic acid
TOPOLOGY:linear
CLONE:HUMGS04806
SEQUENCE DESCRIPTION:
GATCTGTCAT NNTTTAGCAG TCAAGTGGGA TGGGCATTAT ATAAACAACG TTACAATGTA 60
AGGAAAATCT NTAAGGNGAT GGGGAGAGAA AAAGGCAGCT GGTATAATCG GTTACTGCTG 120
CTTAGTTCTA CTTAATTTTN NNN 143

SEQ ID NO:4078
SEQUENCE LENGTH:142
SEQUENCE TYPE:nucleic acid
TOPOLOGY:linear
CLONE:HUMGS04808
SEQUENCE DESCRIPTION:
GATCTGCCTC TCAGGAAAAA GTACTAACTT GTTCTTTTTG TTCCTGGCTT TCATCAGTTT 60
GTGAGATTTC TCTATTTTTT TTAAATATAA TTTTATTTCT TTCAACAAAT ATAAAATAAA 120
AAACAACTTT GGNNCANTGA AA 142

SEQ ID NO:4079
SEQUENCE LENGTH:142
SEQUENCE TYPE:nucleic acid
TOPOLOGY:linear
CLONE:HUMGS04809
SEQUENCE DESCRIPTION:
GATCCTGGCT AACCCTNGCC TATTTAGATT CCTTCCTCTC ACCTGGACCT TCCCATTTCA 60
ATCATGCAGA TGGTTTCTTT TTGTAAAGAG TTCCGTTTGC CTTTCAATTT TTAGAGAAAA 120
TAAAGACTGC ATTCATCTCA AA 142

SEQ ID NO:4080
SEQUENCE LENGTH:141
SEQUENCE TYPE:nucleic acid
TOPOLOGY:linear
CLONE:HUMGS04810
SEQUENCE DESCRIPTION:
GATCTTCTTC TCTCTTGTCC ACCTATTAAT TGTTTACAAT ATTTGTTACA TCTTATGCAA 60
AATACTTGAN TGGGCCATGG TGCCTTTTTT CCTTGTNAGT ATTTAATTAA AAATGAATTG 120
TTTGTCATTT GCAATGTTAA A 141

SEQ ID NO:4081
SEQUENCE LENGTH:140
SEQUENCE TYPE:nucleic acid
TOPOLOGY:linear
CLONE:HUMGS04811
SEQUENCE DESCRIPTION:
GATCCCGTTT TCCCCATGAC AATGTTGTAG TGTCCCCCAC CCCCACCCCC CAGGCCTTGG 60
TGCCTCTTGT ATCCCTAGTG CTCCATAGTT TGGCATTTGC ACGGTTTCGA AGTCATTAAA 120
CTGGTTAGAC GTGTCTCAAA 140

SEQ ID NO:4082
SEQUENCE LENGTH:138
SEQUENCE TYPE:nucleic acid
TOPOLOGY:linear
CLONE:HUMGS04812
SEQUENCE DESCRIPTION:
GATCTGTGCA GCAAACCACC ATGGCACAGG TTTATCTATG GAACAAACCT GCACATCCTG 60
CACGTGTACC CCTGAAATTA AAGTAAAAAT TGGAAATAAA ACATTTAAAA AGAAAAGAAA 120
ACAAACAACA ACAACAAA 138

SEQ ID NO:4083
SEQUENCE LENGTH:138
SEQUENCE TYPE:nucleic acid
TOPOLOGY:linear
CLONE:HUMGS04813
SEQUENCE DESCRIPTION:
GATCTATCAA AGGAAAAGGA AACTGAGACC GAAAACTTAG GGTCTAAGTT GTTCTAAACC 60
CAGGGTTCTC AAATGTGTTG TACAAAAAGT TTCATGTAAT AAAATTAAGC AAATAAAACA 120
AATGAATAGA GGTCCAAA 138

SEQ ID NO:4084
SEQUENCE LENGTH:137
SEQUENCE TYPE:nucleic acid
TOPOLOGY:linear

CLONE:HUMGS04814
SEQUENCE DESCRIPTION:
GATCACAATG TCATTTCCTA ATACAAGGCA GGATATGTGG GAAGAATATG TACAATTATT 60
TGATTAAAAT TATTTCCCAC TGACCTAAAC TTTCAGTGAT TTGTGGGAAA AATAAATAAA 120
TGTTCTACAC CAAGAAA 137

SEQ ID NO:4085
SEQUENCE LENGTH:137
SEQUENCE TYPE:nucleic acid
TOPOLOGY:linear
CLONE:HUMGS04815
SEQUENCE DESCRIPTION:
GATCCACTGC CTCAGCCTCC CAAAGTGCTG GGATTATAGA CATTCATGAT GAATTCTTAT 60
TTTANNNNTA ATATAAAGCA AATACAATTA TTGTTAGATA ACAATGCACA AAATAAAATT 120
CAAGGGTGAA AAATAAA 137

SEQ ID NO:4086
SEQUENCE LENGTH:136
SEQUENCE TYPE:nucleic acid
TOPOLOGY:linear
CLONE:HUMGS04816
SEQUENCE DESCRIPTION:
GATCCATGCC TGTAGTCCAA GCTACTCAGG AGTCCGAGGC ACAAGAATTG CTTGAACCTG 60
GGAGGCAGAG GTCACAGTGA ACCATGTTCT GTCACTGCAC TACATCCTGG GTGACAGAGC 120
GAGACTCCAT CTCAAA 136

SEQ ID NO:4087
SEQUENCE LENGTH:136
SEQUENCE TYPE:nucleic acid
TOPOLOGY:linear
CLONE:HUMGS04817
SEQUENCE DESCRIPTION:
GATCTGGTCC CTGTAGCACA GAGGTCCGAA GAACCGCCTT CAGAAAGAGG AGGCATATTT 60
GGAAGTNATG GGAAAGACTT TNTGGACAAG GATGGAGAAC ACTTTGGAAA ATTGCATTGT 120
GCCAAGAGAC AACAAN 136

SEQ ID NO:4088
SEQUENCE LENGTH:135
SEQUENCE TYPE:nucleic acid
TOPOLOGY:linear
CLONE:HUMGS04818
SEQUENCE DESCRIPTION:
GATCTACAAC ACCTGCCAGT GACCCCTGCC CAAGGCCTAC CCCAGTTCCT AGCACTGCCC 60
CACATGCATG TCTGCCTATG CACTGAAGAG CTCTTGGCCC GGCAGGCCAC CAATAAACCA 120
GTGTGAACCC CCAAA 135

SEQ ID NO:4089
SEQUENCE LENGTH:133
SEQUENCE TYPE:nucleic acid
TOPOLOGY:linear
CLONE:HUMGS04819
SEQUENCE DESCRIPTION:
GATCTGCTGA CAGTGAGTAG TATTTTGTTT TAGGATGTTG TGACTTAGCA AAAATAATAC 60
AGATGTCTTC CCCCCTTTTG TAGCTTTGAC AATTTGAATT AGATTTCAAA TAAAATCTGA 120
ACAGAAAACT AAA 133

SEQ ID NO:4090
SEQUENCE LENGTH:132
SEQUENCE TYPE:nucleic acid
TOPOLOGY:linear
CLONE:HUMGS04820
SEQUENCE DESCRIPTION:
GATCCTTACA TATGCCATCC TTCTGTGTCA TTTTGTGGCT GTTCTGTGTT TTTCTTCCTA 60
GTTATTTATT ATTGTTAATA ACTTACTTTT TCTTACATTC TGTTGTAAAT AAAATACAAA 120
GCAATCTTCA AA 132

SEQ ID NO:4091
SEQUENCE LENGTH:132
SEQUENCE TYPE:nucleic acid
TOPOLOGY:linear
CLONE:HUMGS04821
SEQUENCE DESCRIPTION:
GATCAAACAG AGGCATGCTA AGATATATTG GGGCTTGAAG CAAAGGGAAA ACTATTTGTT 60
GCTATATGTT TGTAGGGATT TTTTGCCAGT TTTAAAAATA CATGTATCAT AAAGTTTACT 120
ATCTCAGCCA AA 132

SEQ ID NO:4092
SEQUENCE LENGTH:132
SEQUENCE TYPE:nucleic acid
TOPOLOGY:linear
CLONE:HUMGS04822
SEQUENCE DESCRIPTION:
GATCAGTGGG GGAGGGCACC TCAGCAACCT CTGAGTGTGG ACAATAGCTG CTTTCTTCTC 60
TATCCAAGAG CACCAGGCTG TGCTTGGNTC CTTGCTCTCA GAGTCTATAA ATAAAAGAAT 120
ATAATGATTA AA 132

SEQ ID NO:4093
SEQUENCE LENGTH:130
SEQUENCE TYPE:nucleic acid
TOPOLOGY:linear

CLONE:HUMGS04823
SEQUENCE DESCRIPTION:
GATCGGGGGT GGGATGGGAT GGAGTGAGCC CCATCCAGTT AGTTGGACTA GTTTTAAATA 60
AAGGTTTTCC GGTTTGTGTT TTTTTGAACC ATACTGTTTA GTAAAATAAA TACAATGAAT 120
GTTGAGTAAA 130

SEQ ID NO:4094
SEQUENCE LENGTH:128
SEQUENCE TYPE:nucleic acid
TOPOLOGY:linear
CLONE:HUMGS04824
SEQUENCE DESCRIPTION:
GATCACAGAG GGAGGAGCTC TGAGAACAGT CTCCTTCAAC AGCTCGGCCA AGCAGAACTG 60
CTGTACCTCT GACCACTTGT GTTAGGAAAA CTATCGGCTC CCTGTATAAT AAATCAAGCC 120
AGGTCAAA 128

SEQ ID NO:4095
SEQUENCE LENGTH:126
SEQUENCE TYPE:nucleic acid
TOPOLOGY:linear
CLONE:HUMGS04826
SEQUENCE DESCRIPTION:
GATCTTTGCC TNTAAGCTTG AATATTTGGC TTAAANTTTG TNCATATGAA TACTGTTAAA 60
GGTATATTTN ACTACATTTT GAAAGGAAAA AGGTAGTCCT GCTAAAATTG ACATTTAGGG 120
ATANNN 126

SEQ ID NO:4096
SEQUENCE LENGTH:124
SEQUENCE TYPE:nucleic acid
TOPOLOGY:linear
CLONE:HUMGS04827
SEQUENCE DESCRIPTION:
GATCTGAAAT TCTGAGATAT CAATCTAGGA ATTTCAAAAG AAACAGATTA TAGAATTTAA 60
AACTTAACAC CNGTCTTGTA TTAAGAGAAG TCAACATTTT AAATAAAACC TTGTTCTTAC 120
CAAA 124

SEQ ID NO:4097
SEQUENCE LENGTH:123
SEQUENCE TYPE:nucleic acid
TOPOLOGY:linear
CLONE:HUMGS04828
SEQUENCE DESCRIPTION:
GATCTCAGAA CTGTGCCAAG CAGACACTGG GACAAAGGGA GAATATCTTG CTCCCCTCCT 60
GAGTCAGCCT GGTGTTGCCC TTTATTCCCC TTATGTGCAT ATGATTAAAG AGTTATTTTT 120
AAA 123

SEQ ID NO:4098
SEQUENCE LENGTH:122
SEQUENCE TYPE:nucleic acid
TOPOLOGY:linear
CLONE:HUMGS04829
SEQUENCE DESCRIPTION:
GATCCTACAA ACAGACATTT TCCACTCGTG TTTCCATAGT TGTTAAGTGT ATCAGATGTG 60
TTGGGCATGT GAATCTCCAA GTGCCTGTNT AATAAATAAA GTATCTTTAT TTCATTCATA 120
AA 122

SEQ ID NO:4099
SEQUENCE LENGTH:121
SEQUENCE TYPE:nucleic acid
TOPOLOGY:linear
CLONE:HUMGS04830
SEQUENCE DESCRIPTION:
GATCTTCTCG CCTCAGCCTC CTGAGTAGCT GGAGCTACAG GTGTGAGCTA CCCAGCATGG 60
CTCATTTGAG ATTTCTGAGT AGAGAAGTAA CATGATTAAA CTTGGGTATT GAGATTATAA 120
A 121

SEQ ID NO:4100
SEQUENCE LENGTH:121
SEQUENCE TYPE:nucleic acid
TOPOLOGY:linear
CLONE:HUMGS04831
SEQUENCE DESCRIPTION:
GATCAATCTC TCCCTCAGCT TTCTTTCCTA CTTTTGCAGC TTTGCTGGCT TTTAACTGCT 60
TCATTCTTCT GCTTCATTGG TATCCTTTTT TCTTTTGAAA TAAAAACATG AAATACTTAA 120
A 121

SEQ ID NO:4101
SEQUENCE LENGTH:121
SEQUENCE TYPE:nucleic acid
TOPOLOGY:linear
CLONE:HUMGS04832
SEQUENCE DESCRIPTION:
GATCGACTGA GTCCAGAAAG TCAAGGCTGC AGAGAGCCAC ATTCACTGCA CTCCAGCATN 60
GNTGNCAGAG GNAGACCCTG TCTCAAAAAA AAAAGAAAAA AGAAAAAGTG ACAATGNCAA 120
A 121

SEQ ID NO:4102
SEQUENCE LENGTH:119
SEQUENCE TYPE:nucleic acid
TOPOLOGY:linear

CLONE:HUMGS04833
SEQUENCE DESCRIPTION:
GATCGGTAGG GGGCCTGCTC CTCGGACTCT GGTTACCTCT AAGGCTGGGA AAGGCCTGGT 60
TGCCCACTGC NTCCNTCCCT GCCCCTCCTT TNATGTCCAT AAAGTGGCGT GAAGTGAAA 119

SEQ ID NO:4103
SEQUENCE LENGTH:116
SEQUENCE TYPE:nucleic acid
TOPOLOGY:linear
CLONE:HUMGS04834
SEQUENCE DESCRIPTION:
GATCACCCTT AAATCNGNGT TGGGAAAATA TCAATTAACT GGACTCTCTG GTTTGAATTC 60
TCAATATGTA TCTTAATATG AAATAGCTCA TTAAAACTTC ATGTGTAACT ATTAAA 116

SEQ ID NO:4104
SEQUENCE LENGTH:115
SEQUENCE TYPE:nucleic acid
TOPOLOGY:linear
CLONE:HUMGS04835
SEQUENCE DESCRIPTION:
GATCTGGGTG GTAGTTACTG GGTGTGTTCT CTTTGTGATA CTCTCTCGAG ATGTACACTT 60
AGGATTTGCA ATCTTTTCTG TAAAATATTT CAATAAAAGA CATTTATTTT TTAAA 115

SEQ ID NO:4105
SEQUENCE LENGTH:115
SEQUENCE TYPE:nucleic acid
TOPOLOGY:linear
CLONE:HUMGS04836
SEQUENCE DESCRIPTION:
GATCTTAGCT AAATCTCGGT AGATGTCATT ACAACCTNGA AAATAAATCA CCCTAAGTGA 60
CACAAATTGA AGCATGTACA AATTATACAT AATAAAGTGT TTTTAATAAT TGAAA 115

SEQ ID NO:4106
SEQUENCE LENGTH:115
SEQUENCE TYPE:nucleic acid
TOPOLOGY:linear
CLONE:HUMGS04837
SEQUENCE DESCRIPTION:
GATCCTNCAC CTCTTGGNAA TTAAAATTGT TGGTCACTGG GGAAAGCCTG AGTTTGCAAC 60
CAGTTGTAGG GTTTCTGTTG TGTTTTTTTT TTTTTTTTAA AAAAAAANTN NAATN 115

SEQ ID NO:4107
SEQUENCE LENGTH:115
SEQUENCE TYPE:nucleic acid
TOPOLOGY:linear

CLONE:HUMGS04838
SEQUENCE DESCRIPTION:
GATCAGACAA GGAATGAAGC AATGNCTGTG GGCTGGGAAA CTGTACCTAC CTCTCTNCCC 60
ACTGCAAATN NCTGGGATAG ACCAAAAGTG AATTTAATTA TGTNTTGGCT GAAGN 115

SEQ ID NO:4108
SEQUENCE LENGTH:115
SEQUENCE TYPE:nucleic acid
TOPOLOGY:linear
CLONE:HUMGS04839
SEQUENCE DESCRIPTION:
GATCCCAACT TGAAACAACA GCCAGTGCCT GTGGTAACTT AANNTNTTGT CAAATACTTT 60
TATTGATTGG TTTATATGCC ATTNTTGTTA TAGAAGAATA TGCCTTTTAA AAANN 115

SEQ ID NO:4109
SEQUENCE LENGTH:112
SEQUENCE TYPE:nucleic acid
TOPOLOGY:linear
CLONE:HUMGS04840
SEQUENCE DESCRIPTION:
GATCAGCTCC GTGGGACTGA TATTGGTCCA CANCANAACA GGCACTGCCT CCTTCCCTCT 60
GGGGAATGCA AAAGAAANCA TGGATTAAGN GAGAAGGGAG ACTCTCAGCN TN 112

SEQ ID NO:4110
SEQUENCE LENGTH:111
SEQUENCE TYPE:nucleic acid
TOPOLOGY:linear
CLONE:HUMGS04841
SEQUENCE DESCRIPTION:
GATCGCCCAA TAAACATTCC CTTGGATGTA GTCTGAGGCC CCTTAACTCA TCTGTTATCC 60
TGCTAGCTGT AGAAATGTAT CCTGATAAAC ATTAAACACT GTAATCTTAA A 111

SEQ ID NO:4111
SEQUENCE LENGTH:111
SEQUENCE TYPE:nucleic acid
TOPOLOGY:linear
CLONE:HUMGS04842
SEQUENCE DESCRIPTION:
GATCGGCATA TGCATATCAA AAACAGCTCC CAGCTCTGTA AGGCATTAGA GTGAAAGCAN 60
ANAATTTTTT AATGAAACAT TTATTTGCCT TCTTTTACTG CAGTGCTNNN N 111

SEQ ID NO:4112
SEQUENCE LENGTH:111
SEQUENCE TYPE:nucleic acid
TOPOLOGY:linear

CLONE:HUMGS04843
SEQUENCE DESCRIPTION:
GATCATACTN CTTTATCTAG AGNTTTGTCT CAGCTTGCAG AGNTTGAGGA GAAGATAGAC 60
CAGTTACATC AAGAACAAGC TTTTCCTGAC TTTAATATGT TTNCNGAACT N 111

SEQ ID NO:4113
SEQUENCE LENGTH:110
SEQUENCE TYPE:nucleic acid
TOPOLOGY:linear
CLONE:HUMGS04844
SEQUENCE DESCRIPTION:
GATCACAGTG CAGATAATTA CCTCACACCT GTTAGGATGG CTTTCACCAA AAAGACAAAA 60
GATACGTGTA GGCAAGGATG TGGAGGAAAG AGAACTCTTG TATACGTAAA 110

SEQ ID NO:4114
SEQUENCE LENGTH:110
SEQUENCE TYPE:nucleic acid
TOPOLOGY:linear
CLONE:HUMGS04845
SEQUENCE DESCRIPTION:
GATCTTAGTC CGTTTTTTTT TTATATATTG TAACGTTAAA TGAAAAAAGA ACCCCCCTNN 60
TGTATTATAG TCATGCGGTC TTATGTATGA TAAACAGTTG AATAATTAAA 110

SEQ ID NO:4115
SEQUENCE LENGTH:110
SEQUENCE TYPE:nucleic acid
TOPOLOGY:linear
CLONE:HUMGS04846
SEQUENCE DESCRIPTION:
GATCTGGGAC TTCCAGAAAA CAGAACTNTN AAAANACAAG TTTCTTTTGT TTAAACCACC 60
CAATCTNAAG TATTTTTTNA TGGCAGTCCT GGGCAGACTA ATACAATATN 110

SEQ ID NO:4116
SEQUENCE LENGTH:109
SEQUENCE TYPE:nucleic acid
TOPOLOGY:linear
CLONE:HUMGS04847
SEQUENCE DESCRIPTION:
GATCTATCAT AAAAAGCAAT GTCTTCTCTG GAATGGGGCT TCAGTTTCCA CATTTACAAT 60
TAGTACATAT TACAATTAGT GATATGAATA AAGGCTGTTA TCCTACAAA 109

SEQ ID NO:4117
SEQUENCE LENGTH:109
SEQUENCE TYPE:nucleic acid
TOPOLOGY:linear

CLONE:HUMGS04848
SEQUENCE DESCRIPTION:
GATCAAAACA GGTGACAGTG AAGTGGTTTC GGCCCTGCCA ATCTGATATC GTGTATGTAT 60
GAGAGTGGTC AGCATTTTAC CACTGGTAAC AAGTTCCTTN CTGTNCAAA 109

SEQ ID NO:4118
SEQUENCE LENGTH:109
SEQUENCE TYPE:nucleic acid
TOPOLOGY:linear
CLONE:HUMGS04850
SEQUENCE DESCRIPTION:
GATCTATATA ATTTATGTAT CACCTTCATT GTAAATTTAG CAGGAAATGC ATCACAATTA 60
TNATTTTTTT TTTTTGNACC AGTGAAACAA TAAAGATGCT ATTACCAAA 109

SEQ ID NO:4119
SEQUENCE LENGTH:107
SEQUENCE TYPE:nucleic acid
TOPOLOGY:linear
CLONE:HUMGS04851
SEQUENCE DESCRIPTION:
GATCATGTTG CTGGCTTTTA TAAACTCTAA GCGAAGGAGG AGCACTGCCT CAGCCTTTGC 60
ACATGGTAAT GAAGCACTGT TTTTAAATAA AAGAGAGAAA CACCAAA 107

SEQ ID NO:4120
SEQUENCE LENGTH:118
SEQUENCE TYPE:nucleic acid
TOPOLOGY:linear
CLONE:HUMGS04852
SEQUENCE DESCRIPTION:
GATCCGTGAG GGCGTGANTT GGACTGGACC CACTGCTGAT TCCCTGCACC TAGCACAGTG 60
CTTGCCACAA AGTGGGCTCT CAATAAATAT TATTTGTCCA AGGAAGGAAT GAATGAAA 118

SEQ ID NO:4121
SEQUENCE LENGTH:108
SEQUENCE TYPE:nucleic acid
TOPOLOGY:linear
CLONE:HUMGS04853
SEQUENCE DESCRIPTION:
GATCGTCTGA GCCCCNCGGG CACTGGGTGG GGCAGAGCAC GAGTTATTTA AAACAGTTAC 60
ACTGCAGGGT TTCGCCCAAT AAAGGTGGAC TGACATTCCC TCTTCAAA 108

SEQ ID NO:4122
SEQUENCE LENGTH:107
SEQUENCE TYPE:nucleic acid
TOPOLOGY:linear

CLONE:HUMGS04854
SEQUENCE DESCRIPTION:
GATCTTTTAT ACTCAGNTTN AAACACTTTA CTTGGGTTTA CCAAGCCTCA ACTGGCATAC 60
CATAAACANG CCACANGGAC CGTTCCTGCA GGCCCAACCC ACAGGGN 107

SEQ ID NO:4123
SEQUENCE LENGTH:107
SEQUENCE TYPE:nucleic acid
TOPOLOGY:linear
CLONE:HUMGS04855
SEQUENCE DESCRIPTION:
GATCCGGAAC CCACTTTTTT ATTCACTCCC CATCGTCGTT TGGCCNTTCC ATCNNTCTTT 60
NTCTTTCCCT CTGCCATCCG TGACACTGAT AGTTNGTCAT ATAAATN 107

SEQ ID NO:4124
SEQUENCE LENGTH:106
SEQUENCE TYPE:nucleic acid
TOPOLOGY:linear
CLONE:HUMGS04856
SEQUENCE DESCRIPTION:
GATCTCCAAT CTCCTCGGTT GCAACACCTG AATAAAGCCT TCTTCCCTCA CAATACTTGN 60
NNNCTCAGTG ATTGGCTTAC TGTGCAGTGA GCAACCTAGA CCAANN 106

SEQ ID NO:4125
SEQUENCE LENGTH:102
SEQUENCE TYPE:nucleic acid
TOPOLOGY:linear
CLONE:HUMGS04859
SEQUENCE DESCRIPTION:
GATCAAAATC ACGTTTGTAG TATCATATCA AAAATTCTAA CCTGTTTACA TTGTTTTNAT 60
GTTCATGTTC CTATGTTATT AAAATATTAT TTTGTACTTA AA 102

SEQ ID NO:4126
SEQUENCE LENGTH:101
SEQUENCE TYPE:nucleic acid
TOPOLOGY:linear
CLONE:HUMGS04860
SEQUENCE DESCRIPTION:
GATCTACGGC AATGTGAATC ATTCAGATGT TTACAATAAA AAACACCACA TGAGTAAATG 60
AATTCACTAA TGTTAATGTT AAACTTCATG GAAAGNNNNN N 101

SEQ ID NO:4127
SEQUENCE LENGTH:100
SEQUENCE TYPE:nucleic acid
TOPOLOGY:linear

CLONE:HUMGS04861
SEQUENCE DESCRIPTION:
GATCCCCCCT CAGGACAAAT CTACTCCAGC CACGATGAGA AGTGGGTGAG CCAGGGTCTG 60
AGTTTCACAT TTGAACCAAA TAAAATGCTG TCAAGAGAAA 100

SEQ ID NO:4128
SEQUENCE LENGTH:97
SEQUENCE TYPE:nucleic acid
TOPOLOGY:linear
CLONE:HUMGS04862
SEQUENCE DESCRIPTION:
GATCATGCCA TTGCACTGCA CCTGGGCAAC AAGAGTGAAA CTCCGTCTTA AAAAATATAA 60
GAAATAAAAA AATAAAAACC TGTCCCCAGA CTGGAAA 97

SEQ ID NO:4129
SEQUENCE LENGTH:97
SEQUENCE TYPE:nucleic acid
TOPOLOGY:linear
CLONE:HUMGS04863
SEQUENCE DESCRIPTION:
GATCCTGAGC TAGGAGAGTA CATTTTGCAG ACCTTTGGTT TCCATATTAA GAAATTCAGA 60
TTTTNATGTA CAATAAAGAA GTTCTGGAAT TCTCAAA 97

SEQ ID NO:4130
SEQUENCE LENGTH:96
SEQUENCE TYPE:nucleic acid
TOPOLOGY:linear
CLONE:HUMGS04864
SEQUENCE DESCRIPTION:
GATCTCTNTN CAGCCCTAAA GCTCCTTGGT TCTGTAATTC CACCAATAGC CCATTTCAAA 60
AATAATTCTA CAGTAATCAG TGGGACTCCT GTTTTN 96

SEQ ID NO:4131
SEQUENCE LENGTH:95
SEQUENCE TYPE:nucleic acid
TOPOLOGY:linear
CLONE:HUMGS04865
SEQUENCE DESCRIPTION:
GATCACACCA CTGCACTCAA GCCTTGGCAT CAGAGTGAGA CTCTGTCTCA GTAAAAAAAA 60
AATAAATAAA TAAAAATGTT TAATTAACCA TGAAA 95

SEQ ID NO:4132
SEQUENCE LENGTH:95
SEQUENCE TYPE:nucleic acid
TOPOLOGY:linear

CLONE:HUMGS04866
SEQUENCE DESCRIPTION:
GATCGTAGGG CATCAGAAAA ACAAAAAGAA ATAGAGAGAG TAAAAGAGAA GCAACAGAAA 60
GAACTCAATA AACAAAAACA GATTGAAAAG GTAAA 95

SEQ ID NO:4133
SEQUENCE LENGTH:98
SEQUENCE TYPE:nucleic acid
TOPOLOGY:linear
CLONE:HUMGS04867
SEQUENCE DESCRIPTION:
GATCTTTATT GTTCCTCACC CCATTTTCCT CCTTGTGTAT GTACTTCCCC CACCCCCCTT 60
TTTTTAAGTA AAATGTAAAT TCAATCTGCT CTAAGAAA 98

SEQ ID NO:4134
SEQUENCE LENGTH:94
SEQUENCE TYPE:nucleic acid
TOPOLOGY:linear
CLONE:HUMGS04868
SEQUENCE DESCRIPTION:
GATCTAATAA GTATTGGAAT GCTTCCTATT TGCTGATAGA AGTACCAAAT AGTATTATTG 60
AAGTCTAACA AAGACTTTTT GTTGAGAACA CAAA 94

SEQ ID NO:4135
SEQUENCE LENGTH:94
SEQUENCE TYPE:nucleic acid
TOPOLOGY:linear
CLONE:HUMGS04869
SEQUENCE DESCRIPTION:
GATCGAGGTA AGAGGGACTT TNTTAAAGGC CTAGTCTATG GGATGGGGCG GCGGAGGGAA 60
TTTTTTGAGA AATAAAATGA AGCTGCAGTG TAAA 94

SEQ ID NO:4136
SEQUENCE LENGTH:92
SEQUENCE TYPE:nucleic acid
TOPOLOGY:linear
CLONE:HUMGS04870
SEQUENCE DESCRIPTION:
GATCACACCG CAGACATTTA GATTTTTATA CCCAAGGCAC TTTAAAAAAA TGTTTTATAA 60
ATAGAGAATA AATTGAATTC TTGTTCCATA AA 92

SEQ ID NO:4137
SEQUENCE LENGTH:91
SEQUENCE TYPE:nucleic acid
TOPOLOGY:linear

CLONE:HUMGS04872
SEQUENCE DESCRIPTION:
GATCTTTACT GATGCACTCA TGACAAGTAC CCAATGTATT TTAGCTATTT TAGTAGTATT 60
TGTTCAATAA ATACGCAAGC TGTAAGGTAA A 91

SEQ ID NO:4138
SEQUENCE LENGTH:91
SEQUENCE TYPE:nucleic acid
TOPOLOGY:linear
CLONE:HUMGS04873
SEQUENCE DESCRIPTION:
GATCTGCTTT TCTGCATGTT TGTCTGTGTG TCTGCGTTGT GTGTGATTTT CATGGAAAAA 60
TAAAATGCAA ATGCACTCAT CACAAACTAA A 91

SEQ ID NO:4139
SEQUENCE LENGTH:90
SEQUENCE TYPE:nucleic acid
TOPOLOGY:linear
CLONE:HUMGS04874
SEQUENCE DESCRIPTION:
GATCAAAAAA AATAAATCAC AATGGTATTT AGAAAATATT TTTCACTGAA GGCTAACAAA 60
ACACTACATA TTAAAATTAT AAAATGCAAA 90

SEQ ID NO:4140
SEQUENCE LENGTH:90
SEQUENCE TYPE:nucleic acid
TOPOLOGY:linear
CLONE:HUMGS04875
SEQUENCE DESCRIPTION:
GATCTTTTGA TGTGCACTAA TGCCATTATT GGTAATGCCG GTTATTGGTG AATACAGCAT 60
AGTTAAATAA ACTGTTACAG TAAATCTAAA 90

SEQ ID NO:4141
SEQUENCE LENGTH:89
SEQUENCE TYPE:nucleic acid
TOPOLOGY:linear
CLONE:HUMGS04877
SEQUENCE DESCRIPTION:
GATCTAGGGC AAACTTGTCC AACTGTTGGC CCATGGAACA GCTTTGAATG CAGCCCAACA 60
CAAATCTATA AATTTNCTTA AACATTAAA 89

SEQ ID NO:4142
SEQUENCE LENGTH:88
SEQUENCE TYPE:nucleic acid
TOPOLOGY:linear

CLONE:HUMGS04878
SEQUENCE DESCRIPTION:
GATCAGTCTC ATGGGCCATC TCTTCCTCAG ATGTAAATAA TATCTGGTTA AGTGTTATAT 60
GGAATAAAGT GGACATTTTA AAACTAAA 88

SEQ ID NO:4143
SEQUENCE LENGTH:88
SEQUENCE TYPE:nucleic acid
TOPOLOGY:linear
CLONE:HUMGS04879
SEQUENCE DESCRIPTION:
GATCTCAGTG ATGTTTGAAC CTTCTGTGTA ACTTTTTATT AAGTCTTTGT ATCTCTCGAC 60
TGATTAATAA AGAAGAGAAA CACGTAAA 88

SEQ ID NO:4144
SEQUENCE LENGTH:87
SEQUENCE TYPE:nucleic acid
TOPOLOGY:linear
CLONE:HUMGS04881
SEQUENCE DESCRIPTION:
GATCTTTATT GGTGACCTTT TGTAAGACAT TAGTTTGAGG TACTACCTAT GTACTTGAAA 60
ATAATAAAGT GGCATTTCTT TATGAAA 87

SEQ ID NO:4145
SEQUENCE LENGTH:86
SEQUENCE TYPE:nucleic acid
TOPOLOGY:linear
CLONE:HUMGS04882
SEQUENCE DESCRIPTION:
GATCCACTTT GTACTCCTAC CAGTAGGAAC GGTCTTCTAA CAGAGAAGAT TATAGCAAAA 60
AATAAAATCT CTTCTTAATT CCCAAA 86

SEQ ID NO:4146
SEQUENCE LENGTH:86
SEQUENCE TYPE:nucleic acid
TOPOLOGY:linear
CLONE:HUMGS04883
SEQUENCE DESCRIPTION:
GATCCAAAGT CCAGCTCTCT CACTGAAGCC TTATCTTTCG GAAAGCTTCC AACAGTAACT 60
CAGGAATTNA GCTTACTTGC TTTAAA 86

SEQ ID NO:4147
SEQUENCE LENGTH:85
SEQUENCE TYPE:nucleic acid
TOPOLOGY:linear

CLONE:HUMGS04884
SEQUENCE DESCRIPTION:
GATCTAAAGA AGAAGGTTCA GTTTTCAAAC AGAATTTAGA GAAAATATAA AGAAATAATT 60
TCTTGTCTCA AAAGGCCAAA GTAAA 85

SEQ ID NO:4148
SEQUENCE LENGTH:84
SEQUENCE TYPE:nucleic acid
TOPOLOGY:linear
CLONE:HUMGS04885
SEQUENCE DESCRIPTION:
GATCCAGCTG TGCCTAAAGC CTGCCCTACC TCCGGACTTT AAAGTTTTGT GAGCCAATAA 60
AGTCCCTTTC TTGTTTAAGA TAAA 84

SEQ ID NO:4149
SEQUENCE LENGTH:84
SEQUENCE TYPE:nucleic acid
TOPOLOGY:linear
CLONE:HUMGS04886
SEQUENCE DESCRIPTION:
GATCTGAAAA GGCATTAATT TATGTACTAA TTCTATAAAC ATGTATTAAT AATTGCAGTA 60
TTATTAAATA CAGATGGACT CAAA 84

SEQ ID NO:4150
SEQUENCE LENGTH:83
SEQUENCE TYPE:nucleic acid
TOPOLOGY:linear
CLONE:HUMGS04887
SEQUENCE DESCRIPTION:
GATCAGGCCC CCCAACTCTC CAGAACAGGC TACAAACATG CAAATTAGAA TTCTTTTAAT 60
ATAAAAAAAA GTACTAAAAT AAA 83

SEQ ID NO:4151
SEQUENCE LENGTH:83
SEQUENCE TYPE:nucleic acid
TOPOLOGY:linear
CLONE:HUMGS04888
SEQUENCE DESCRIPTION:
GATCAGAGGG NAGTACAGAA NCCCTGAACT AAAGCNTTAA GTACATACCA TACAGCAAAT 60
AAATGGTAGC AAAACATTCT AAA 83

SEQ ID NO:4152
SEQUENCE LENGTH:83
SEQUENCE TYPE:nucleic acid
TOPOLOGY:linear

CLONE:HUMGS04889
SEQUENCE DESCRIPTION:
GATCTAAAAT TTGTAATATT TTTGTCATGA ACTGTACTAC TCCTAATTAT TGTAATGTAA 60
TAAAAATAGT TACAGTNACT AAA 83

SEQ ID NO:4153
SEQUENCE LENGTH:81
SEQUENCE TYPE:nucleic acid
TOPOLOGY:linear
CLONE:HUMGS04890
SEQUENCE DESCRIPTION:
GATCACATGG TTCACTACCA AATNACCCTC AAATAAGCCA GATGAAAATT TGAAGAAAAA 60
GGTCACCCAG TTCTCTGGAA A 81

SEQ ID NO:4154
SEQUENCE LENGTH:81
SEQUENCE TYPE:nucleic acid
TOPOLOGY:linear
CLONE:HUMGS04891
SEQUENCE DESCRIPTION:
GATCAGAGCG TAGTTTTAAN TGTATTTTTG ATACACTTGT GAAATATTTT ATAGAAATAA 60
AAATTTTTAC TTAGGAGCAA A 81

SEQ ID NO:4155
SEQUENCE LENGTH:81
SEQUENCE TYPE:nucleic acid
TOPOLOGY:linear
CLONE:HUMGS04892
SEQUENCE DESCRIPTION:
GATCCTTCCT CCCACCCTAN GCCTTCCTTT CCTCTTTCCT CCTCACTCTC CCCGTCATGC 60
TCCCTCTGCC CNGNCCTCAA A 81

SEQ ID NO:4156
SEQUENCE LENGTH:80
SEQUENCE TYPE:nucleic acid
TOPOLOGY:linear
CLONE:HUMGS04893
SEQUENCE DESCRIPTION:
GATCCAAANT AGTAACNTTT TNTNATGAAC TGTACTACTC CTAATTATTG TAATGTAATA 60
AAAATAGTTA CAGTGACAAA 80

SEQ ID NO:4157
SEQUENCE LENGTH:79
SEQUENCE TYPE:nucleic acid
TOPOLOGY:linear

CLONE:HUMGS04894
SEQUENCE DESCRIPTION:
GATCATTAGT CAATTGCTTT AATTATAAGC CCTGTTTTTT TTTAAATCTA AAAACTAATA 60
AACATCTATA AGAATTAAA 79

SEQ ID NO:4158
SEQUENCE LENGTH:79
SEQUENCE TYPE:nucleic acid
TOPOLOGY:linear
CLONE:HUMGS04895
SEQUENCE DESCRIPTION:
GATCNACTGT CAAAAAGAAG GGTTCAAGTA AAATAAAATG AGAGCAATTA TATATATAAA 60
TATATATCAT ACACAGAAA 79

SEQ ID NO:4159
SEQUENCE LENGTH:76
SEQUENCE TYPE:nucleic acid
TOPOLOGY:linear
CLONE:HUMGS04897
SEQUENCE DESCRIPTION:
GATCACCTGA ATTCTGGAGG TTGAGGCTGC ACCACTACAT TCCAGCCTGG GAGATAGAGT 60
GAGAGCCTGT CTTAAA 76

SEQ ID NO:4160
SEQUENCE LENGTH:76
SEQUENCE TYPE:nucleic acid
TOPOLOGY:linear
CLONE:HUMGS04898
SEQUENCE DESCRIPTION:
GATCCCAAAT NTTCCCACAA GCTTTATTCC AAAAATAATT TTATTTAATA GGTATTAAAT 60
AATGTATAGA AGGAAA 76

SEQ ID NO:4161
SEQUENCE LENGTH:74
SEQUENCE TYPE:nucleic acid
TOPOLOGY:linear
CLONE:HUMGS04900
SEQUENCE DESCRIPTION:
GATCTTCCTG TTTCAGTCCC CAAGGTGTTG GGATTATGGG CATGAACTGC TGCACCCAGC 60
CCTTTATACT CAAA 74

SEQ ID NO:4162
SEQUENCE LENGTH:73
SEQUENCE TYPE:nucleic acid
TOPOLOGY:linear

CLONE:HUMGS04902
SEQUENCE DESCRIPTION:
GATCACTGGT ATGTAGATAA TAAAATGTGA AAATAAAAAT TTAAAAATAA AACAAAAATT 60
ATGTGATAAT AAA 73

SEQ ID NO:4163
SEQUENCE LENGTH:73
SEQUENCE TYPE:nucleic acid
TOPOLOGY:linear
CLONE:HUMGS04903
SEQUENCE DESCRIPTION:
GATCGTGCAA CTGCACTCCG GCTTGGGCTA CAGAGTGAGA CTCCATCAGA AAGAAAAGAA 60
GAGAGAGATG AAA 73

SEQ ID NO:4164
SEQUENCE LENGTH:74
SEQUENCE TYPE:nucleic acid
TOPOLOGY:linear
CLONE:HUMGS04904
SEQUENCE DESCRIPTION:
GATCAGCATG TAAAGTGTCT GGCGTGTAGT AGGCTCTTAA TAAACACTGG CTGAATATGA 60
ATTGGAATGA TAAA 74

SEQ ID NO:4165
SEQUENCE LENGTH:72
SEQUENCE TYPE:nucleic acid
TOPOLOGY:linear
CLONE:HUMGS04905
SEQUENCE DESCRIPTION:
GATCTCAACT CGTGGCACTA ACTTGGAAAA GGGTTGATTT AAAATAAAAG GGAAGACTAT 60
TTTACAAGCA AA 72

SEQ ID NO:4166
SEQUENCE LENGTH:70
SEQUENCE TYPE:nucleic acid
TOPOLOGY:linear
CLONE:HUMGS04907
SEQUENCE DESCRIPTION:
GATCCTAAAC AGATGCATTG TTGCTTTTCT GTGCTGTCTC TGCAGATTAA AACATAATGA 60
TTACACTAAA 70

SEQ ID NO:4167
SEQUENCE LENGTH:135
SEQUENCE TYPE:nucleic acid
TOPOLOGY:linear

CLONE:HUMGS04908
SEQUENCE DESCRIPTION:
GATCTATTAT GCCTTTTTAC AAAAAAAATG GCTGTAAATN ATTGTAAATA TTAAAGGAAC 60
TTTCCTTACT TCCTTCCCTT TCTCAGGCTT TTTTTGACTG TTCCTTTCCC TACCAACTCA 120
GGCCTTCTTA TTAAA 135

SEQ ID NO:4168
SEQUENCE LENGTH:69
SEQUENCE TYPE:nucleic acid
TOPOLOGY:linear
CLONE:HUMGS04909
SEQUENCE DESCRIPTION:
GATCTCCCTG GAGGTTTCAT AGCAAGGTTT GACTTGATGA ATTAATTAAT AAAGCTAAAT 60
ATCCTGAAA 69

SEQ ID NO:4169
SEQUENCE LENGTH:67
SEQUENCE TYPE:nucleic acid
TOPOLOGY:linear
CLONE:HUMGS04910
SEQUENCE DESCRIPTION:
GATCACCCAC GGGGAGGAGA AGGAGGAGTG AGACCCAGCC GGGTCAATAA ACCTGGACGC 60
TTGGAAA 67

SEQ ID NO:4170
SEQUENCE LENGTH:65
SEQUENCE TYPE:nucleic acid
TOPOLOGY:linear
CLONE:HUMGS04912
SEQUENCE DESCRIPTION:
GATCTGCTTA TAAATAAACT TAACAAATAC ACTATGGAGA TTAAAAACAA AATACCACCC 60
ACAAA 65

SEQ ID NO:4171
SEQUENCE LENGTH:64
SEQUENCE TYPE:nucleic acid
TOPOLOGY:linear
CLONE:HUMGS04913
SEQUENCE DESCRIPTION:
GATCTGTTTC TCTAGAAAAA AAGTCCATCT CTGGCTTTAA TAAAATTATG CATCAGAAAT 60
CAAA 64

SEQ ID NO:4172
SEQUENCE LENGTH:76
SEQUENCE TYPE:nucleic acid

TOPOLOGY:linear
CLONE:HUMGS04914
SEQUENCE DESCRIPTION:
GATCTCATGC TNAGCAGAGC AAAAATTGTA AAATATTTTG ATTAAAAATC TAGGGACCTT 60
TATGTCCTAT TTGAAA 76

SEQ ID NO:4173
SEQUENCE LENGTH:63
SEQUENCE TYPE:nucleic acid
TOPOLOGY:linear
CLONE:HUMGS04915
SEQUENCE DESCRIPTION:
GATCAGGAAG TGGCTTTGTG TCTTCCCACA TGTATGTATA AATAAAGTGT TCTTTTAAGC 60
AAA 63

SEQ ID NO:4174
SEQUENCE LENGTH:63
SEQUENCE TYPE:nucleic acid
TOPOLOGY:linear
CLONE:HUMGS04916
SEQUENCE DESCRIPTION:
GATCCTCATT TTTTCCCATT GTAAAATTGG GCTAATAAAA GATGCTACCT ACTGACTATT 60
AAA 63

SEQ ID NO:4175
SEQUENCE LENGTH:62
SEQUENCE TYPE:nucleic acid
TOPOLOGY:linear
CLONE:HUMGS04917
SEQUENCE DESCRIPTION:
GATCACGCTA CTGCACTCCA GCACTCCAGC GCAGGCAACA GAGGGAGACT CTTTGTCTCA 60
AA 62

SEQ ID NO:4176
SEQUENCE LENGTH:62
SEQUENCE TYPE:nucleic acid
TOPOLOGY:linear
CLONE:HUMGS04918
SEQUENCE DESCRIPTION:
GATCCTGAAT TTCCCAGCCA ACAGAACCAA GTGCTAAATA AAACTCTTTT TAATAAGTTA 60
AA 62

SEQ ID NO:4177
SEQUENCE LENGTH:62
SEQUENCE TYPE:nucleic acid

TOPOLOGY:linear
CLONE:HUMGS04919
SEQUENCE DESCRIPTION:
GATCAAAACT CTGTTCTCAA AAATAAATAA AAAATAAAAA ACAAAACAAC AACAACAACA 60
AA 62

SEQ ID NO:4178
SEQUENCE LENGTH:61
SEQUENCE TYPE:nucleic acid
TOPOLOGY:linear
CLONE:HUMGS04920
SEQUENCE DESCRIPTION:
GATCACGCCA CTGCATTCCA GCCTGGGAGA CAGAGTGAGA AATAAATAAC TGTAATGTAA 60
A 61

SEQ ID NO:4179
SEQUENCE LENGTH:60
SEQUENCE TYPE:nucleic acid
TOPOLOGY:linear
CLONE:HUMGS04921
SEQUENCE DESCRIPTION:
GATCCAAGCA AACTACTTTA AGCAAATATT TGTAAAATTA AAAACGGAGG ACCGCTCAAA 60

SEQ ID NO:4180
SEQUENCE LENGTH:60
SEQUENCE TYPE:nucleic acid
TOPOLOGY:linear
CLONE:HUMGS04922
SEQUENCE DESCRIPTION:
GATCTAACAG TTCTCCAATA AAATAGTTCT CATTCCAGAG AGCAGATGAA TATATTTAAA 60

SEQ ID NO:4181
SEQUENCE LENGTH:59
SEQUENCE TYPE:nucleic acid
TOPOLOGY:linear
CLONE:HUMGS04923
SEQUENCE DESCRIPTION:
GATCTATGAC TGTACTACAC TCCAGCCTGG TCAACAAAGC AAGACCATAT CTCTTTAAA 59

SEQ ID NO:4182
SEQUENCE LENGTH:59
SEQUENCE TYPE:nucleic acid
TOPOLOGY:linear
CLONE:HUMGS04924
SEQUENCE DESCRIPTION:

GATCCAAGTA TTTNATCTGT GTTGTCTCTC TAAACCCAAA TAAATGTGTA AATGTGAAA 59

SEQ ID NO:4183
SEQUENCE LENGTH:59
SEQUENCE TYPE:nucleic acid
TOPOLOGY:linear
CLONE:HUMGS04925
SEQUENCE DESCRIPTION:
GATCCAATAC ACACATANCT TACAACTATC ACAAATTCCT ATTAAATATT AAAAGTAAA 59

SEQ ID NO:4184
SEQUENCE LENGTH:59
SEQUENCE TYPE:nucleic acid
TOPOLOGY:linear
CLONE:HUMGS04926
SEQUENCE DESCRIPTION:
GATCTCAAAN NGAAATCTTA TACCAGTGAC AACTGTATTA GAATATATCT GTTCTTAAA 59

SEQ ID NO:4185
SEQUENCE LENGTH:57
SEQUENCE TYPE:nucleic acid
TOPOLOGY:linear
CLONE:HUMGS04927
SEQUENCE DESCRIPTION:
GATCTCATAT TAATATTTTA ATATTTCTTT TCAAAATAAA ACAGAAAAGC AAGTAAA 57

SEQ ID NO:4186
SEQUENCE LENGTH:57
SEQUENCE TYPE:nucleic acid
TOPOLOGY:linear
CLONE:HUMGS04928
SEQUENCE DESCRIPTION:
GATCTGTGTG TTCTTTCAAA GAGAAGTTAT GTCTAATTAA AATGTGTAAC GGACAAA 57

SEQ ID NO:4187
SEQUENCE LENGTH:57
SEQUENCE TYPE:nucleic acid
TOPOLOGY:linear
CLONE:HUMGS04929
SEQUENCE DESCRIPTION:
GATCCGNGAG AGAACGACCA CAATTTAAAC ACATCAATAA ATACTTTAAC TTCCAAA 57

SEQ ID NO:4188
SEQUENCE LENGTH:56
SEQUENCE TYPE:nucleic acid

TOPOLOGY:linear
CLONE:HUMGS04930
SEQUENCE DESCRIPTION:
GATCAAGAAG GACTACCTGT CATGTGAGAC CATCACTAAG AAACCATTTG CTGAAA 56

SEQ ID NO:4189
SEQUENCE LENGTH:52
SEQUENCE TYPE:nucleic acid
TOPOLOGY:linear
CLONE:HUMGS04940
SEQUENCE DESCRIPTION:
GATCCCACTT CGGTCTGTAA ATTCCCTCAA TAAATCACCC AATATTGACA AA 52

SEQ ID NO:4190
SEQUENCE LENGTH:51
SEQUENCE TYPE:nucleic acid
TOPOLOGY:linear
CLONE:HUMGS04945
SEQUENCE DESCRIPTION:
GATCCCCAAA GCTTAAAATA TCGACTATTT AGAGTGAGAC TCCATCACAA A 51

SEQ ID NO:4191
SEQUENCE LENGTH:51
SEQUENCE TYPE:nucleic acid
TOPOLOGY:linear
CLONE:HUMGS04947
SEQUENCE DESCRIPTION:
GATCAGCCAN GCATTCCAGC CTGGTGACAG AGCGAGACTC TGTCTCNGAA A 51

SEQ ID NO:4192
SEQUENCE LENGTH:56
SEQUENCE TYPE:nucleic acid
TOPOLOGY:linear
CLONE:HUMGS04948
SEQUENCE DESCRIPTION:
GATCTTGTTT GTTGAGGGAG ATGGGACTAT AATAAAATTG AATGCTGAAG TTGAAA 56

SEQ ID NO:4193
SEQUENCE LENGTH:50
SEQUENCE TYPE:nucleic acid
TOPOLOGY:linear
CLONE:HUMGS04950
SEQUENCE DESCRIPTION:
GATCGCGCCA CTGCGTTACA GCCTGGCGAT AATGCAGGAC TCAGTCCAAA 50

SEQ ID NO:4194
SEQUENCE LENGTH:113
SEQUENCE TYPE:nucleic acid
TOPOLOGY:linear
CLONE:HUMGS04965
SEQUENCE DESCRIPTION:
GATCTGTCTT GTAAACCACA TTCTTGACAA CTATTTGCTT TTGAGTAGTT TGTATTTTAA 60
TATGTGACTT TNGTCTTGAA AAGTAGTAAA GCCATAGACT TGTGCAAAAC AAA 113

SEQ ID NO:4195
SEQUENCE LENGTH:192
SEQUENCE TYPE:nucleic acid
TOPOLOGY:linear
CLONE:HUMGS04985
SEQUENCE DESCRIPTION:
GATCATTATG CAATAAAGCT GCTAAAGTNA CATTTGTGGT TGATACTCAA GCCTTGTACA 60
AGCACATATN ATGTAGACAT TTTNAAATGT NAAGCTTTAA CAGGAATAGG ATAGCTGTTT 120
CAAACCTTGT GCAATTGTAA ATAAATTGTT TAGCTGTGTT TCTTTCATTT TNAATAAAAA 180
ATTAAAAAGA AA 192

SEQ ID NO:4196
SEQUENCE LENGTH:50
SEQUENCE TYPE:nucleic acid
TOPOLOGY:linear
CLONE:HUMGS04986
SEQUENCE DESCRIPTION:
GATCAATTTG TAATTGCTTA CATTTTTACA ATAAATAATC TGTACGTAAA 50

SEQ ID NO:4197
SEQUENCE LENGTH:282
SEQUENCE TYPE:nucleic acid
TOPOLOGY:linear
CLONE:HUMGS04987
SEQUENCE DESCRIPTION:
GATCTTTCTT TGGCAACCAC TTTTTACTGT CGCCATAGTT CTTCCTTTTC TAGAATGTCA 60
TATTGGAATC ATATAGTATG TAGCCTTTTC AGACTGGCTT CTTTCACTTA ATAATATGCA 120
ATTAAGGTTC CTCCATGTCA TTTCATGGCT TAATAGTGCA TTTATTTTTA GCACTGAATA 180
ATACTCCATT GTCTAGATGA ATAGTTTATC CATTCACCTA TTGAAAGACT TCTTGGTGGT 240
TTCCAAGTTT TGGCAATTAT GAATAAAGCT GTTGTAAACA AA 282

SEQ ID NO:4198
SEQUENCE LENGTH:257
SEQUENCE TYPE:nucleic acid
TOPOLOGY:linear
CLONE:HUMGS04988

SEQUENCE DESCRIPTION:
GATCTTTTCA GTCAAAAATC TAAGATGATT TATTTTGTAT CACTTTGTTA AAAGCTGAAT 60
ATTGTTAACT ACAGTTAATA TTAACACTGT ATTTATACTT TCTCAAACTA CATCCGCCCC 120
ACCACTTCTG GTTGCCTCTG TTGACTATTA ATCCAGATGT AAACAACCAG ATGTTTTTTT 180
CTAACTTGTA CAAACTGACG TGTGTCAACT ATCATGGAAG GAAAAAAATG TACAGATTAA 240
AATTATTCAG NGTTAAA 257

SEQ ID NO:4199
SEQUENCE LENGTH:132
SEQUENCE TYPE:nucleic acid
TOPOLOGY:linear
CLONE:HUMGS04989
SEQUENCE DESCRIPTION:
GATCCAGGNG TTGACTATAA CCAGATTTAC TCTTTGCTTA CTAGGNTTTC CATNNTCACC 60
ATTGGATGAA CCNCTGGTTT AGCCACAGTT GTGAAAATAA ATGGAAGTTG GTTGATTGTC 120
TAGAAAGTGA AA 132

SEQ ID NO:4200
SEQUENCE LENGTH:51
SEQUENCE TYPE:nucleic acid
TOPOLOGY:linear
CLONE:HUMGS04990
SEQUENCE DESCRIPTION:
GATCCGCATC CAAGCTTAAC CAAGGCTCCA ATAAACGTGC TAGGAAGCAA A 51

SEQ ID NO:4201
SEQUENCE LENGTH:108
SEQUENCE TYPE:nucleic acid
TOPOLOGY:linear
CLONE:HUMGS04991
SEQUENCE DESCRIPTION:
GATCAGTCGC TGACTCATTA GATGAACCAG GAGCCTGCAT GCTACAGCCC ACCTGCCAAA 60
TCTTGCCCCC ACCTGCTTTT TAAAATAAAG TTTTATTGGA ACATGAAA 108

SEQ ID NO:4202
SEQUENCE LENGTH:113
SEQUENCE TYPE:nucleic acid
TOPOLOGY:linear
CLONE:HUMGS04992
SEQUENCE DESCRIPTION:
GATCTTTGCT CTGTTTCCAG TGGGGTTTGA AGCAGAGTTC AGGGAACCCT GCCCAAGGTC 60
CTCCTGTTCA GACATTCCTA TGTTGAATAA AGTATGTTTG ACTTCCCCGG AAA 113

SEQ ID NO:4203
SEQUENCE LENGTH:471

SEQUENCE TYPE:nucleic acid
TOPOLOGY:linear
CLONE:HUMGS04993
SEQUENCE DESCRIPTION:

```
GATCCCACCT CAAATTAGCT TGATTGCAAA ACAGATATTG GAACATGGAG CTGAGAAGAA  60
GATGGTCTGG GCTTCAGGCA CAGCTGGGTC CAGGGACTCA GACCNNNTTG TCTCCATCTA 120
TCTNCTGTGT TTTNCTGTAG GAGGTGGCCT CACTTTCCCC TAGTTCAAAA GGGCTTACTC 180
CATATGATGG GGGAAGGGAA GACAGGNCCA TGAACAGCCC TAGACTTCCA TCAGCCCACC 240
TGGGAAGCCC TCACAGGAAA ATNAGTCTCA TTCCTAGTAC ATGAAATCTC AGGGGAGGGA 300
ACTCCGATTT GGCCACCTTC AGTNATGTGC CCNGTCCCTN GGGGCCAAAT CCNCTTTTGG 360
GTTGGTGGGG GCGGGTTTCA AGTGATTGGG NAAATNGCCA ACCNAAAGTT CANGTTNGAA 420
GTTGGAGNAA NGGGGAANTT TTNCCCAAAA AGAAANGGNA AGGTTTGNTA N          471
```

SEQ ID NO:4204
SEQUENCE LENGTH:338
SEQUENCE TYPE:nucleic acid
TOPOLOGY:linear
CLONE:HUMGS04994
SEQUENCE DESCRIPTION:

```
GATCAGGAAT GGGTGATTGG AGATTATTAG ATTCTAGGTT AACTTCTACC ACTTTACCCT  60
AATACATAAA ACTTTTTCCT AAATAAATGA TGGAAGGAAT AATACTTGGT TACCTGGCAT 120
TATTTTTCAG TAAGAAAAAA GCTTTACTAA CCACTACATT TATGGAAATT TGTAGGGGTA 180
AGTATTTTAT AGGTCATAAA AAACACCATA ATATAACGAA TCTCATTTNC TTTAAATGTG 240
AATTAAATCC TAACAGTCAT CTTTATAAAA TGACCATAGG CTAAANTCTT ACGTGTAAGT 300
NCTACTACAN TAAATANTTT CTGGAAACCT TTTATAAA                         338
```

SEQ ID NO:4205
SEQUENCE LENGTH:237
SEQUENCE TYPE:nucleic acid
TOPOLOGY:linear
CLONE:HUMGS04997
SEQUENCE DESCRIPTION:

```
GATCACCTTC CAGACCACAG CAGATGTGGG ACACACAGCC TCCTGACTCC AGGAAGAGCC  60
AGANCTGTGC AGGGAGGAAG GGGTGAGAGG GGGGCCCCCA CACCTAGACT CAGTAAGGAA 120
GTCGGGTTNG ACCTTAACAT CTNCATTGGA CAACTCCACC CNTNCCTTGG CCTTGCCCCT 180
GCCCGNNTAC ACTCCTACGT NTCCANGGGC TTNGGCCGTG ACTTAGGNAG AGGAGTN    237
```

SEQ ID NO:4206
SEQUENCE LENGTH:313
SEQUENCE TYPE:nucleic acid
TOPOLOGY:linear
CLONE:HUMGS04998
SEQUENCE DESCRIPTION:

```
GATCATCCGC AGNCTCATGT TAAAAGGATT TTAGCTCACT AAAAGTGTAA TAATGGAAAT  60
GTGGAAAATA TCGTAGGTAA AAGAAACTAC CTCATGCTCT GAAGGTTTTG TAGAAGCACA 120
```

ATTAAACATC TAAAATGGCT TTGTTACACC AGAGCCATCT GGTGTGANGA ACTCTATATT 180
TGTATGTTGA GAGGGCATGG ANTAATTGTA TTTNGCTGGC AGTAGACACA TTCTTTATTA 240
TTTGCAGATT CCTCATCAAA TCTGTANTTA TGCACAGTTT CTGTTATCAT TAAAACAAAG 300
GAATCCNGTT AAA 313

SEQ ID NO:4207
SEQUENCE LENGTH:271
SEQUENCE TYPE:nucleic acid
TOPOLOGY:linear
CLONE:HUMGS04999
SEQUENCE DESCRIPTION:
GATCACATCT NTNTGAAGCC AAAGCCCCGT GGTTGCCCAT GAGAAGTGTC CTTTTTCATT 60
TTCACCCAAA TGAAGTGTGA ACGTGATGTT TTCGGATGCA AACTCAGCTC AGGNATTCAT 120
TTTGTNTCTT AGTTTTATAT GCATCCTTAT TTNTAATACA CCTGCTTCAC GTCCCTATGT 180
TNGGAAGTCC ATATTTGTNT GCTTTTNTTG CAGCATCATT GCCTNTACAA TACTGNCCGG 240
TGGACAAANT GACAATNGAT ATGTNTTGCT N 271

SEQ ID NO:4208
SEQUENCE LENGTH:345
SEQUENCE TYPE:nucleic acid
TOPOLOGY:linear
CLONE:HUMGS05001
SEQUENCE DESCRIPTION:
GATCGCACTG TGGGCCGGGG CCCTGGAGGG CTGCTCTGTG TTAATAAGAT TGTAAGGTTT 60
GCCCTCCTCA CCTGTTGCCG GCATGCGGGT AGTATTAGCC ACCCCCCTCC ATCTGTTCCC 120
AGCACCGGAG AAGGGGGTGC TCAGGTGGAG GTGTGGGGTA TGCACCTGAG CTCCTGCTTC 180
GCGCCTGCTG CTCTGCCCCA ACGCGACCGC TGCCCGGCTG CCCAGAGGCT GGATGCCTGC 240
CGGTCCCCGA GCAAGCCTGG GAACTCAGGA AAATTCACAG GACTTGGGAG ATTCTAAATC 300
TTAAGTGCAA TTATTTTTAA TAAAAGGGGT ATTTGGAATC AGAAA 345

SEQ ID NO:4209
SEQUENCE LENGTH:406
SEQUENCE TYPE:nucleic acid
TOPOLOGY:linear
CLONE:HUMGS05002
SEQUENCE DESCRIPTION:
GATCTTACTT TCATAATTCT TTGATTCTAG CTTGCAGAGT CAAGACGNAA AGNTAACTCA 60
TGGGATGGAC AAACTGGAAG ATGTAAAATA AGTAAGGCTT TNTGGGCCAA AAAGCCTCTT 120
CTTACAGAAA ATCAAATTTT AAAAGAACAT TGACCTCAAA ACAATAAAAC TGTCCTGGTT 180
ATGCAATAGA AATAGCTATA TAAATGGAAT CATATCCTTA ATGAACACCT CCTGTGGCTA 240
CCCAACTTTT CACACATCTT ACTGATTATA GGCCTTGCTG TGAATGCTTT CTTGCTCTAT 300
CTGAAGTATT CCCACCAGAG TTGTTTCCAA CNTTATTTCC TATATTATGA AAAATGANGT 360
TTTTTAACA TTGTAACAAA GTATTTGNAA GGTAATGTAT ACTGTN 406

SEQ ID NO:4210

SEQUENCE LENGTH:74
SEQUENCE TYPE:nucleic acid
TOPOLOGY:linear
CLONE:HUMGS05003
SEQUENCE DESCRIPTION:
GATCTAAAAA AANNTAAATT ACATTAGTAA CACAACATAA GAAAAAGACA GGGACAAAAA 60
CAAAAAAAAA TAAA 74

SEQ ID NO:4211
SEQUENCE LENGTH:230
SEQUENCE TYPE:nucleic acid
TOPOLOGY:linear
CLONE:HUMGS05004
SEQUENCE DESCRIPTION:
GATCCTATGT GGAAGGGCTG TTTTTTAAGA AAAAATTTTT GGTAAACAGT ATTGTGTAAA 60
ATTGCTTTTT GTATACCAAT ATATGCATGT TTTGTGCATG AGTAGTACTT GTGTTGATAC 120
TCCTGTTGAT GTTAAATTAC TATATAATAT AANCAGTATG TGTTTTTATA TATCATTGTG 180
TAAATTTAAT ATAACATATG CNGTAATAAN CCATTNGTNT TACGGGCAAA 230

SEQ ID NO:4212
SEQUENCE LENGTH:161
SEQUENCE TYPE:nucleic acid
TOPOLOGY:linear
CLONE:HUMGS05005
SEQUENCE DESCRIPTION:
GATCTCAATC AAGTTAGCAT GCTTNCTCCA GCTGCCCTAT NACCTGCCAG CTCATCACAA 60
AGAACCACGG CCACCCAGGT CATGGCAAAC TCTGCTGGAC TTAACTTCAT CAATGTAGTG 120
GGCTCTNTTT GTGGGGCCCA GGCTTTGATG AGTNGGNNNN N 161

SEQ ID NO:4213
SEQUENCE LENGTH:259
SEQUENCE TYPE:nucleic acid
TOPOLOGY:linear
CLONE:HUMGS05006
SEQUENCE DESCRIPTION:
GATCGAGACC ATCCTGGCCA ACATGGTGAA ACCCCGTCTC TACTAAAGAT ACAAAAATTA 60
GCTGGACATG GTGGCGGGCG CCTGTNTTCC CAGCTACTGG GGAGGCTGAG GCAGGAGAAT 120
GGCTTNGAGG CAGTAGAACT GCTTGAACCC TGGAGGCGGA GGTTACAGTT ATCCGAGGTT 180
GTGTCATTGC ACTCCAGCCT GGGGAACAGA GCGAGACTCC ATCTCAAAAA AAATAAAAAA 240
CTTCAGCTTT TGCATGAAA 259

SEQ ID NO:4214
SEQUENCE LENGTH:71
SEQUENCE TYPE:nucleic acid
TOPOLOGY:linear

CLONE:HUMGS05007
SEQUENCE DESCRIPTION:
GATCATGCTT TAAGGTTTAT GTAAAGAAAG TGTACTGATG TTCTTACATT AAAGCTTTAC 60
AAAGATTTAA A 71

SEQ ID NO:4215
SEQUENCE LENGTH:67
SEQUENCE TYPE:nucleic acid
TOPOLOGY:linear
CLONE:HUMGS05008
SEQUENCE DESCRIPTION:
GATCTTGGTG GTAGTAGCTG TCTATGATTC TNGCTCAGCA AAGTAAAATA AATGTTAAAT 60
ATGGAAA 67

SEQ ID NO:4216
SEQUENCE LENGTH:143
SEQUENCE TYPE:nucleic acid
TOPOLOGY:linear
CLONE:HUMGS05009
SEQUENCE DESCRIPTION:
GATCAAATGA AATAATGTAC ATGAAAAGAC ATATATAAAA TGTATAATAC AGACAAAGGG 60
NCCTGTTACA CAGTTGGAAG TCATNCCCTC AGATTTTGGG CCTAGGAAGG TAGGTGATTT 120
AAACTCACTG AAAGCANNGN NNN 143

SEQ ID NO:4217
SEQUENCE LENGTH:362
SEQUENCE TYPE:nucleic acid
TOPOLOGY:linear
CLONE:HUMGS05010
SEQUENCE DESCRIPTION:
GATCACAGCC ACTGGTCCAT AACAATTAGG AAAACTCATT GGACAATGGT TGTCAGGTTC 60
CTCCATTGGA GGGAATTGTG TTTCATGATT AGGCACTGTT TCTGCTCCTT AAAAATGACT 120
TTCCAGTCTA TCCTCCACAG CTCTTTGTTC TACACTCTAG GGAATCATTC TTTTTCCCTC 180
ATCTTCCTTA GTCACTTGCA AAGGACATTA GAGAATATGC CCTTTTTGGT GGATAAATGG 240
GTTCTTCAAC CTGCTTTCTG CCTGTAGAAA ATTAGAGGCC AATAATCTGT TTATATCTTG 300
TAGAATGTCT ATTTTAGCTG GTACCGGTAC TTTTGTCAAT AAAGTTCTTT TTTAAACTTA 360
AA 362

SEQ ID NO:4218
SEQUENCE LENGTH:213
SEQUENCE TYPE:nucleic acid
TOPOLOGY:linear
CLONE:HUMGS05011
SEQUENCE DESCRIPTION:
GATCTGNGAA GAGCACATAT GTGTCAGGGC ACAATTCCCN CTCATAAAAA CCACACAGCC 60

TGGAAATTGG CCCTGGCCCT TCAAGATAGC CTTCTTTAGA ATATNATTTG GCTAGAAAGA 120
TTCTTAAATA TGTGGANTAT GNTTATCCTT AGCTGGAATA TTTNCTCTAC TTCCTGTCTG 180
CATGCCCNAG GCTTCTGAAG CAGCCAATGT CGN 213

SEQ ID NO:4219
SEQUENCE LENGTH:169
SEQUENCE TYPE:nucleic acid
TOPOLOGY:linear
CLONE:HUMGS05013
SEQUENCE DESCRIPTION:
GATCACTACA ACANTTTNTT TGCCTCCAAG GTCCTCTGAG GCAGCAGGCT CTGGGGCTTC 60
TNCTGTCCTT TGGAGGGTGT CTTCTGGGTA GAGGGATGGG AAGNAAGGGA CCCTTACCCC 120
CGGNTCTTCT CCTGACCTGC CAATAAAAAT TTATGGTCCA AGGGAGAAA 169

SEQ ID NO:4220
SEQUENCE LENGTH:235
SEQUENCE TYPE:nucleic acid
TOPOLOGY:linear
CLONE:HUMGS05014
SEQUENCE DESCRIPTION:
GATCGCGCAG GATTTCAAAA CCGACCTGAG GTTTCANAGC GCANCATCGG TGCGCTGCAG 60
GAGGCTAGCG AAGNANCCTG GTGGGTCTGT TCGAAGATAC CAACCTGTGT GCCATCCACG 120
CTAAGAGAGT CACCATCATG CCCAAAGACA TCCAGTTGGC TCGCCGGATA CGGGGAGAGA 180
GAGCTTAAGT GAAGGCAGTT TTTATGGCGT TTTGTAGTAA ATTCTGTAAA ATAAA 235

SEQ ID NO:4221
SEQUENCE LENGTH:196
SEQUENCE TYPE:nucleic acid
TOPOLOGY:linear
CLONE:HUMGS05016
SEQUENCE DESCRIPTION:
GATCAAAGTC CTACAGTGGC TGTCACGCAG CCACNAGGTC ATCTCCTTTC ATCCCCACCC 60
CAAGGCGCTG GCTGTGACTC TGCTTCCTGC ACTGACCCAG AGNCTCTGCC TGTGCATGGC 120
CAGCTGCGTC TACTCAGGTC CCAAGGGGTT TCTGTTTCTA TTCTTTCCTC AGACTGCTCA 180
AGAGAAGCAC ATGAAA 196

SEQ ID NO:4222
SEQUENCE LENGTH:262
SEQUENCE TYPE:nucleic acid
TOPOLOGY:linear
CLONE:HUMGS05018
SEQUENCE DESCRIPTION:
GATCTGCACC AGACTGTTAC CTTCTGGGGG AGATGGAGAT TTGACTGTTT AAAAACTGAA 60
AACAGCGAGG AGTCTTTCTA GAACTTTTGA ACACTAAAAG GATGAAAAAA ATTAGCAAAC 120
CGAAGTTTCT TCAATGACCC CTCGAGAACT TTGGGACCAG TTTCCTATGG GGGACTCAGT 180

TTCAGAGAAC TGAGACAGAA GCTCTTCTGT CGTTATATTC TTCTTTCCTT TTTTTGGATT 240
TATTAAATAT TTTCTGTGGA AA 262

SEQ ID NO:4223
SEQUENCE LENGTH:277
SEQUENCE TYPE:nucleic acid
TOPOLOGY:linear
CLONE:HUMGS05019
SEQUENCE DESCRIPTION:
GATCCTGCAG TGTCCTGTGG GTTCATCTCG GATGGGCGCT GCATAAACAG CCTGCAGGGA 60
ACGTCTGGGA GAACCAGCTT CACAACAGAT ATAATAAGGA AGAAGTTTGA AATATTGCAA 120
GANTTGCGAA AGTGTGACAC ACAGATACAA AGTGGGCACA TGCTCGTGGG AAAATGTTGC 180
CAATAGACTT GCTTGACTCT GGAGTTACCC CGAACCTTCA ATTTGTTTAA AAAAAAAAAN 240
GGGCANTTTN TTGNNGNGCA NTAAGGTGNC CTGCAAA 277

SEQ ID NO:4224
SEQUENCE LENGTH:271
SEQUENCE TYPE:nucleic acid
TOPOLOGY:linear
CLONE:HUMGS05021
SEQUENCE DESCRIPTION:
GATCGCGTCC ACCTTCATCG CTGTGGTGCA AGCCATCGTC CGCACGGCTG GTGGCTGGGC 60
AGCTGCCTCG GACTCCAGGA AAGGCGGGGA GCCTGCCGGC TACTGAGTGC TCCAGGAGGA 120
CAAGGCTGAC AAGCAATCCA GGGACAAGAT ACTCACCAGG ACCAGGAAGG GGACTCTGGG 180
GGACCGGCTT CCCCTGTNAG CAGCAGAGCA GCACAATAAA TNAGGCCACT GTGCCAGGNT 240
CCAGGTGGCC TNCCTGGNCT GTTTCCCCAA A 271

SEQ ID NO:4225
SEQUENCE LENGTH:138
SEQUENCE TYPE:nucleic acid
TOPOLOGY:linear
CLONE:HUMGS05023
SEQUENCE DESCRIPTION:
GATCCACGGA AGACTGTCAG CAGACTTCAC CAGAAAACTT TCACGCCAGA NGGCAGTCGT 60
GATATATACA AAANGCTGAA AGGAAANCAC TGCCATCCAG GANTACTATC TCCAACTNTC 120
CTGTGTTTCA AAANTTAN 138

SEQ ID NO:4226
SEQUENCE LENGTH:64
SEQUENCE TYPE:nucleic acid
TOPOLOGY:linear
CLONE:HUMGS05024
SEQUENCE DESCRIPTION:
GATCTGCATT TAAAAATATG GTCTTTGTTT TATATGATTA AATGGTTTGT TTTCATAGAT 60
GAAA 64

SEQ ID NO:4227
SEQUENCE LENGTH:296
SEQUENCE TYPE:nucleic acid
TOPOLOGY:linear
CLONE:HUMGS05025
SEQUENCE DESCRIPTION:
GATCCCGGCT GTTGGTGAGC AAAGAGCCCA GGCCCCTAGA GTGCGCATGT NCAGGCTCCG 60
CTGTTAGANT CACAGCGGTT CAAATCCGGC ATCTGGTCGC TGAGTGGCCT CAGGCAGTGA 120
CCACGCTCCC GGACCCAACC TTCAGCTTGC CCAAAGCAAT AATCTTTCCT AAAGAAGTGC 180
TTGGNTGGGG ATGGTGGCTC ACGCTTGTAA ATCCCAGTAC TTTGGGAGGC CAAGGCAGTC 240
TGGGCAATAT AGTGAGGCTC CCATCTGTAC TAAAAATAAA AAAGTTAGGC GTGAAA 296

SEQ ID NO:4228
SEQUENCE LENGTH:135
SEQUENCE TYPE:nucleic acid
TOPOLOGY:linear
CLONE:HUMGS05026
SEQUENCE DESCRIPTION:
GATCAGACCT CTCATGCTGT GGAGATGAAA AAGCCCTAAA GTTTATGTTT CTAATGTGTC 60
ACAGAATAGG ACGATATGAT TCTACAACAT AATCAACTCC CTATTAAATT CTTTAATGAA 120
GATTTTTTTT TTAAA 135

SEQ ID NO:4229
SEQUENCE LENGTH:337
SEQUENCE TYPE:nucleic acid
TOPOLOGY:linear
CLONE:HUMGS05027
SEQUENCE DESCRIPTION:
GATCCTCTTT CTAGCATGAA TGAAGGATGC CAAGAATGAG AAAAAGCAAG GGGTTTGTCC 60
AGGTGGCCCC TGAATTCTGC GCAAGGNATG GGCCTGGGGG AACTCAAGGG AGGGCCTAAA 120
GCACTTGTAA CTTTGAACCG TCTGTCTGGA GGTCAGAGCC TGTTGGAAAG CAGGGGTAGA 180
GGGGAGCCCT GGAAGCAGGG CTTTTCCGGA TGCCTAGGGG TGGGCAGTGC CAGCCCCTCC 240
TCACCACTCT TCCCCTTGCA GTGGAGGAGA GAGCCAGAGT GGATACTATT TTTNATTAAA 300
TATATTATTA TATGTTAATA AAAAANTCAT ATCCAAA 337

SEQ ID NO:4230
SEQUENCE LENGTH:179
SEQUENCE TYPE:nucleic acid
TOPOLOGY:linear
CLONE:HUMGS05028
SEQUENCE DESCRIPTION:
GATCCTTCGC GAGCGAGTGA ACAAGAGGCG GGTGAAGGAG ATGGTGAAGG AGTTCACACT 60
GCTGTGCCGG GGTCTCCATG GCACAGGGTA CACAGCTGAC TACTGAGGGG TGCCCCCATC 120
CCATCCACCC CTTCTCTTCA TCCTTCCCTA TTCCCAAAGN GTAAACCTGG NCCCTNAAA 179

SEQ ID NO:4231
SEQUENCE LENGTH:207
SEQUENCE TYPE:nucleic acid
TOPOLOGY:linear
CLONE:HUMGS05029
SEQUENCE DESCRIPTION:
GATCGCTGGA CGCTATGAGA GGGCGTCCCA GGGCCCAGCC TCCCACAGCC GTTTCAGCAG 60
GGACAGGGGC TGAACAGGCC CTATTCCAGC CCCCTTGCTT CACTCTACCG GACAGACGGC 120
AGCAGTCCCA GCTCTGGTTT CCTTCTCGGT TTATTCTGTT AGAATGAAAT GGTTCCCATA 180
AATAAGGGGC ATGAGCCCTT CCTCAAA 207

SEQ ID NO:4232
SEQUENCE LENGTH:386
SEQUENCE TYPE:nucleic acid
TOPOLOGY:linear
CLONE:HUMGS05030
SEQUENCE DESCRIPTION:
GATCTGAAAT AGTTTTCTTC CACTACATTG TTGAAATCAA TGAAGCAATT AGTTTCTCAT 60
TCAGAAATGT GCACACTAAT ATTTAGTTTT GCTTTCTCGT GGATAATATT AAGCNCTTAC 120
TCTGCAGTTT CCTGGAAGTT GTGTCAACTG CAGTGATACT ATTCAGGATG GTGGGAAATC 180
CCCAAAAATA TGTATGTGTG GGCTTGCTTA GATTACTATA TTTCATAGTT AATCTTTTGT 240
CTCTTGCGGT GCTCATGATG TGTGGGGCAC ACGGAAGGCA TTGCTGTAGT CAGTCATTTT 300
GGTTTTCTTC TATAGCCATT TTATTATTTT AGTGTATTAG TTATGAAGAT AATATTNNCT 360
ATTTGTAAAT NGCTACTTTG TATTTN 386

SEQ ID NO:4233
SEQUENCE LENGTH:356
SEQUENCE TYPE:nucleic acid
TOPOLOGY:linear
CLONE:HUMGS05031
SEQUENCE DESCRIPTION:
GATCTTCANA TTGCTGTGAT TAATGCATCC AGATTNTTTT CCTAGTATTT CCAGGTTAGA 60
ACCTGTGGAT TGTTTCANTT GCATATAGCT TGGTTTCCAT AACATAGAGC ATTGGTTGAC 120
TGTTTACAGA AGACTCACTC ACCAGGATAA ACATTGCNGT ATGTNACAGT AAAGCTATCT 180
GGAGAGANCA CATAAATGAT TTTGGCATAC CATTAGAGAA ACCATTTGTA AAACTCAAAT 240
GNCCACATAA NGCTTATCAG GGGGTCTAGA TTGGTNTTGT NNTATACCAT ATGGGATGAN 300
GNAAANTAGA AATGTCAGTA GAACTCATTG AGGGTGCTCT NGCCAGCTGC NGANAN 356

SEQ ID NO:4234
SEQUENCE LENGTH:105
SEQUENCE TYPE:nucleic acid
TOPOLOGY:linear
CLONE:HUMGS05033
SEQUENCE DESCRIPTION:

GATCTGCTTT TTTNAAATGT TAAGGACATA TTTTNAAGTT ACTGCTGAGT GAAGAAAGAA 60
CATAGCTCCC CCTCATTCCC TGCGTAAGAA AGGAGCTTAA AGAAA 105

SEQ ID NO:4235
SEQUENCE LENGTH:343
SEQUENCE TYPE:nucleic acid
TOPOLOGY:linear
CLONE:HUMGS05034
SEQUENCE DESCRIPTION:
GATCAGGGCC TTCCTGCCTC CCGCTGGGCA GGTCTGGCCT TGCNCTCTTG GCAGGGCCCC 60
AGCCCCTCTA ACCACTCTGC AGCTCACCAT GCAGCTGATG CCAAANTTGT GGTGTCCAGT 120
GTGCAGCAGC CCTGGGAGCC ACTGCCACCT TCAGAGGGGT TCCTTGCTGA GACCCACATT 180
GNTTCACCTG GCCCCACCAT GGCTGCTTGC CTGGCCCAAC CTAGCGTTCT GTGCCATGNT 240
AGAGCTTGAG CTGTTGCTCT NCTTCAGGGG AGGAAATAGG GTNGAGAGNG GGAAGAGNNT 300
TNGCTCCTAA GTGTTGCTGC TGTGGCTTTT TTTGCCTTCT GCN 343

SEQ ID NO:4236
SEQUENCE LENGTH:370
SEQUENCE TYPE:nucleic acid
TOPOLOGY:linear
CLONE:HUMGS05035
SEQUENCE DESCRIPTION:
GATCATTTCA AAACTCATTT CCTATGTAAC TGCATTGAGA ACTGCATATG ATTCGCTGAT 60
ATATGTGTTT TTCACATTTG CGAATGGTTC CATTCTCTCT CCTGTACTTT TTCCAGACAC 120
TTTTTTGAGT GGATGATGTT TCGTGAAGTA TACTGTATTT TNACCTTNTT CCTTCCTTAT 180
CACTGACACA AAAAGTAGAT TAAGAGATGG GTTTGACAAG GTTCTTCCCT TTTACATACT 240
GCTGTCTATG TGGCTGTATC TNGTTNTTCC ACTACTGNTA CCACANCTAT ATTATCATGC 300
AAATGCTGTA TTCTTCTGTT GGGTGGAGAN AAAGGATTTC TTGNAGGTTT NGTTTTTAAA 360
ATTNAAAGCN 370

SEQ ID NO:4237
SEQUENCE LENGTH:529
SEQUENCE TYPE:nucleic acid
TOPOLOGY:linear
CLONE:HUMGS05037
SEQUENCE DESCRIPTION:
GATCTGTTTC GTGCATTGGA AGACCCCACC CAAGCTTGGC AGNCGAGCNN ATTGTATCCT 60
GGGGCTCCCT TCATCTCCAG GGAGTCCNNT CCCCGGCCCT ACCAGCGCCC GCTTGGCTGA 120
GCCCCTACCC CACACCAGGC CGTCCTCCCG GGCCCTCCCT TGGAAACCTG CCCTGCCTGA 180
GGGCCCCTCT GCCCAGCTTG GGCCCAGCTG GGCTCTGCCA CCCTACCTGT TCAGTGTCCC 240
GGGCCCGTTG AGGATGAGGC CGCTAGAGGC CTNAGGATGA GCTGGAAGGA GTGAGAGGGG 300
ACAAAACCCA CCTTGTTGGA GCCTGCAGGG TGGTGCTGGG ACTTAGGCCA GTCCCAGGGG 360
CAATGTATTG GCCTGGAAGG TGGGGTTTGG GATTTNGGGG GCTTGGTGCC AAGCTTTCCT 420
TTTNNANGNT GACCTTTNTT TNGTNCTTNC CCCTTTGGGG NGGGNTNAAA AGNCCCAAGT 480
TTGANANTGG GAAATAAANT TTTTTTGGGC CTCCGGNTTT CCCTTNAAA 529

SEQ ID NO:4238
SEQUENCE LENGTH:365
SEQUENCE TYPE:nucleic acid
TOPOLOGY:linear
CLONE:HUMGS05039
SEQUENCE DESCRIPTION:
GATCGTTTTC TGATTTGTAT GCTTCACAAA CTTGGATTTG ACAAAGAAAA TGTTTATGNN 60
NGAATTGCGA CAGTGTATTC GCAACTNTCC TCAGTTCAGA TTTGACTGGT TTCTTAAGTC 120
CAGAACTGCA ATGGAGCTCC AGNGGAGATG TAATACCTTA ATTNCTTTGA TTGAAAGAGA 180
AAACATGGAA CTAGAAGAAA AGGAGAAGGC AGAGAAAAAG AAACGAGGAC CAAAGCCTTC 240
ANCACAGAAA CGTAAAATGG ATGGCGCACC TGATGGTCGA GGTAGAAAAA NGTGGCTGGA 300
ANCTATGNNT ATGGTTTTNT TTCATAAATC ACTNAGCTTT ANACCNAGTA GTTCCTNTNA 360
NTTTN 365

SEQ ID NO:4239
SEQUENCE LENGTH:114
SEQUENCE TYPE:nucleic acid
TOPOLOGY:linear
CLONE:HUMGS05040
SEQUENCE DESCRIPTION:
GATCATTACA ACANTTGTNT GCCTCCAAGT CCTTGAGGCA GCAAGTCTGG GGCTTCTGTN 60
TCNTTTGAGG GTGTCTTCTG GGTAGAGCGA TGGGAAGGAA GGACCCTTAC CCNN 114

SEQ ID NO:4240
SEQUENCE LENGTH:312
SEQUENCE TYPE:nucleic acid
TOPOLOGY:linear
CLONE:HUMGS05041
SEQUENCE DESCRIPTION:
GATCATCCGC GGCGGCCGGG CTCGTGGGGC GCCTGGAGTG AGGGTTCTGG TTCCCGCCGG 60
CGAGGATTGT TAAAATNAGT CTTCGGAAGN AGACCCCTAG TGACTTCTTA AAGCAAATCA 120
TCGGACGACC AGTTGTGGTA AAATTAAATT CTGGAGTGGA TTATCGAGGG GTCCTGGCTT 180
GCCTGGATGG CTACATGAAT ATAGCCCTGG AGCAGACAGA AGAATATGTA AATGGACAAC 240
TGAAGAATAA GTATGGGGAT GCATTTATCC GAGGAAACAA TGTGTTGTAC ATCAGTACAC 300
AGNAGAGACG GN 312

SEQ ID NO:4241
SEQUENCE LENGTH:306
SEQUENCE TYPE:nucleic acid
TOPOLOGY:linear
CLONE:HUMGS05042
SEQUENCE DESCRIPTION:
GATCGGAGTA CANAGTNACA NTTGGAGGGC AGGGGGACTN TAAGGCAAGG AGATTACAGT 60
TGGGAAGGAG GCAGTGGCAG AGGGGTGAGG GACAGGGGCC CTTAAGTCCA GCGAGGAAAG 120

CTCGGTGTGG GCCCGTCTAC GCTCCGTTTG GGGTGACCTG GAACGCCTCT TCTACAGCTC 180
CTNCAGCATC AGCAGCCTCT TGTCAAGTTT TGCCTCGCCC ANTCTATCCC AACCCAAATA 240
AGACACTTTC TTCACGNCAT ATTATCTTTG TCCTTTCTTT TTTAAGAACA TCACAAAACC 300
ATGAAA 306

SEQ ID NO:4242
SEQUENCE LENGTH:287
SEQUENCE TYPE:nucleic acid
TOPOLOGY:linear
CLONE:HUMGS05043
SEQUENCE DESCRIPTION:
GATCTTAAAC CCAGTCTTGA CAGACTAGAA GTTAAGTTTA ATACAGAAAA TGTCCTGTTC 60
ACTTGAAAGA AAAGACCTAC TTTTTAAATG GACACTATCT TGGTTCTTTT TTTTTAAGTC 120
ATCTTTTTGT TATAAGTGAC CTTTGTCTGG AATGTCTGAA AAGTAGTTAA TGCTTTTNGG 180
TATTGAAGTA ATGGGTAACT AAAATGGACT TCCATAGTAT TGACTGTAGA AGGAGCCTCT 240
ACAATATTGA CTATATATNN NNATAAACTA CTGGCAAGGN ACTTAAA 287

SEQ ID NO:4243
SEQUENCE LENGTH:324
SEQUENCE TYPE:nucleic acid
TOPOLOGY:linear
CLONE:HUMGS05044
SEQUENCE DESCRIPTION:
GATCTCCACT GTTGGGTGGG TGGGCCCTGC CGGGACCCTG CTTCACAGGC AGGCTTTTCC 60
TCCAACCTGT GGCGCTGCAG CAGGGCAGGA AGGGGAAACA CAGCTGATGA ACTGTGGTGC 120
CACATGACCC TTGTGGCACA GATGCCCACG TATGTGAAAC ACACATGGAC ATGTGTCCCA 180
GCCACAGTGT TATGCTCTGT GGCNGGCTCA CCTTTGCTGA GTTCCGGGGT GCAATGGGGG 240
AGGGAGGGAG GGAAAGCTTC CTCCTAAATC AAGCATCTTT CTGTTACTTG ATGTTCAATA 300
AAAGAATAGT TGGCAAGGCT GAAA 324

SEQ ID NO:4244
SEQUENCE LENGTH:65
SEQUENCE TYPE:nucleic acid
TOPOLOGY:linear
CLONE:HUMGS05045
SEQUENCE DESCRIPTION:
GATCATTCAT ATGTGTACAT AAAATTTTAA AAATAAAGGG AATTGACTGC TTTGTTAATG 60
AGAAA 65

SEQ ID NO:4245
SEQUENCE LENGTH:288
SEQUENCE TYPE:nucleic acid
TOPOLOGY:linear
CLONE:HUMGS05046
SEQUENCE DESCRIPTION:

GATCCAGAGA ATGAATCCCT GACCGCATCA CCTAAACTGT CTTCCAAACA TGAGACAAAG 60
CTGACTGTTC ACACTGATTG CCCAGCACAT ACCGTCTTGC CAGTTTCTTC TTTTCTCCCA 120
GTCTCCTGTT CATCCATTCT GTTCTCCCTT GGGGTGGGAA TCTATGATGG AGGTTACTGG 180
GGAAACAGCT CAGCAGATTT TTGGAGACCA AACCAAAGGT CTCACTAGGA AATTTATCTG 240
TTTTAAAACA TTGCTTCCTT CCTGGCTCTG CTAAATTGAA TGCTCATN 288

SEQ ID NO:4246
SEQUENCE LENGTH:333
SEQUENCE TYPE:nucleic acid
TOPOLOGY:linear
CLONE:HUMGS05047
SEQUENCE DESCRIPTION:
GATCTCTGCT CCTAACGATG AAGACTGGGC CTATTCACAG CAGCTCTCTG CCCTCAGTGG 60
TCAGGCGTGC AATTTTGGTC TGCGCCACAT AACCATTCTG AAGCTTTTAG GCGTTGGAGA 120
GGAAGTTGGG GGAGTGTTAG AACTGTTCCC AATTAATGGG AGCTCTGTTG TTGAGCGAGA 180
AGACGTACCA GCCCATTTGG TGAAAGACAT TCGTAACTAT TTTCAAGTGA GCCCGGAGTA 240
CTTCTCCATG CTTCTAGTCG GAAAAGACGG AAATGTCAAA TCCTGGTATC CTTCCCCAAT 300
GTGGTCCATG GTGATTGTGT ACGATTTAAT TGN 333

SEQ ID NO:4247
SEQUENCE LENGTH:288
SEQUENCE TYPE:nucleic acid
TOPOLOGY:linear
CLONE:HUMGS05051
SEQUENCE DESCRIPTION:
GATCCTCCAC AGAGAGGAGG GGACCAATTC TGGACAGACA GATGTTGGGA GGATACAGAG 60
GAGATGCCAC TTCTCACTCA CCACTACCAG CCAGCCTCAG AAGGCCCCAG AGAGACCCTG 120
CAAGACCACG GAGGGAGCGA CACTTGAATG TAGAATAGGC AGGGGGCCCT GCCCCACCCN 180
ATCCAGCCAG ACCCCACGNT GACCATGCGT CAGGGGNCTA GAGGTGGAGT TCTTAGCTAT 240
NCTTGGCTTT NAGAGCCAGC CTGGNTCTGN CCNCTCCCNC CATGGGGN 288

SEQ ID NO:4248
SEQUENCE LENGTH:170
SEQUENCE TYPE:nucleic acid
TOPOLOGY:linear
CLONE:HUMGS05052
SEQUENCE DESCRIPTION:
GATCTCTAAG TGACCACCAG GGGCTCTGAA CTGCAGCTGA TGTTATCAGC AGGCCATGCA 60
TCCTGCTGCC AAGGGTGGAC ACGGCTGCAG ACTTCTGGGG GAATTGTCGC CTCCTGCTCT 120
TTTGTTACTG AGTGAGATAA GGTTGTTCAA TAAAGACTTT TATCCCCAAA 170

SEQ ID NO:4249
SEQUENCE LENGTH:136
SEQUENCE TYPE:nucleic acid
TOPOLOGY:linear

CLONE:HUMGS05053
SEQUENCE DESCRIPTION:
GATCTACATA GATTTGGAAA CTGTTTTCCT CTGTTTTGGT CTCTTGGGCA ACATTTTTGG 60
CCCAAGTTTG GGCAACATTT GGCCCAAGNT TGGGCATTTT GGCAGTAGCT GTATGGNAGA 120
AAAAGAGTAA GAGGAN 136

SEQ ID NO:4250
SEQUENCE LENGTH:100
SEQUENCE TYPE:nucleic acid
TOPOLOGY:linear
CLONE:HUMGS05054
SEQUENCE DESCRIPTION:
GATCNTTTGC TTTAGNGTGT AAAGTNTGTC TCTGAACAGA ATTATTTGTA AAAGTTAGGA 60
GTTCTTTTTT AAATCATTAA AAGAGGCTTG NTGAAGGAAA 100

SEQ ID NO:4251
SEQUENCE LENGTH:204
SEQUENCE TYPE:nucleic acid
TOPOLOGY:linear
CLONE:HUMGS05055
SEQUENCE DESCRIPTION:
GATCCTAGAG CCCTTCACTT CGGNTTACTC CCTCTTTTTT GCCTCTNTTT NTTAGTTGGA 60
AGAAATAAAC TCACAAATTA TGGTGCAGTA ATTTNCCGGG GAAAGTAAAG CCTCAGGAAT 120
GCCCACGCCT TTTTTCCAAA GCCTTTNTCT CTGAGACCTC TTAAGTTCTA AGATTAAATG 180
CCCCTCGCTG TTTCTCCTCT GAAA 204

SEQ ID NO:4252
SEQUENCE LENGTH:310
SEQUENCE TYPE:nucleic acid
TOPOLOGY:linear
CLONE:HUMGS05056
SEQUENCE DESCRIPTION:
GATCAAGGAG CAATTATGAA GGTCTACAAG CAAGANCAAG AGGAAGAATT ATNGAAAAAG 60
TTGGCAAATG NCCCCAGATG GAAGAGATAC AGGAGATGGA TGAAGAATGA AGGGCCTGGG 120
CGGTTAACAT TTGTGGATGA CTGANGATTG ATGGAATGCT ACTATGCCAA ACCTTAATTG 180
TGATATTATT TTCATAACTG ANTTATTTTA GAAATGTATC ANTNGACTGC TGCTCAGCAG 240
TAACTAAAAT TCCTCAAGTA TTTGATTAAA CAGATTAATG TCAAANTTTA AACCTTCCCT 300
TAAAACTTTN 310

SEQ ID NO:4253
SEQUENCE LENGTH:267
SEQUENCE TYPE:nucleic acid
TOPOLOGY:linear
CLONE:HUMGS05058
SEQUENCE DESCRIPTION:

GATCTAATTG CATTGGTTAA AAGCAGCTAA CCAGGTCTTT AGAATATGCT CTAGCCAAGT 60
CTAACTTTAT TTAGACGCTG TAGATGGACA AGCTTGATTG TTGGAACCAA AATGGGAACA 120
TTAAACAAAC ATCACAGCCC TCACTAATAA CATTGCTGTC AAGTGTAGAT TCCCCCCTTC 180
AAAAAAAGCT TGTGACCATT TTGTATGGCT TGTCTGGAAA CTTCTGTAAA TCTTATGTTT 240
TAGTAAAATA TTTTTTGTTA TTCTAAA 267

SEQ ID NO:4254
SEQUENCE LENGTH:307
SEQUENCE TYPE:nucleic acid
TOPOLOGY:linear
CLONE:HUMGS05059
SEQUENCE DESCRIPTION:
GATCANAACT CAAACGGGCC GGNNTTTCTA AGGTGTNGGT ATGTGGGGAG TGGTACAAAA 60
TGGTCTGATG CTCCTTCAAA AACATTCACT TTTTACAACG TCAAGGAATT AAGCATAAAA 120
AAGATTGGTT AAAAGCTTTG GTTTCTAGTA AAGGTTAGTG TGTGTGGTTT TTTTAAGAAG 180
CTGTNTTGCT AAATNATTTT TACTTGGAAT GTTTCAAACA GATTTCAGGC TGCAAACTTG 240
TTTNATAATC GTNNGCTTCT CCAAGTGAAG CTTCAGGAAT ACCTGGAAAA TAGCTGTAAC 300
GTTCGCN 307

SEQ ID NO:4255
SEQUENCE LENGTH:297
SEQUENCE TYPE:nucleic acid
TOPOLOGY:linear
CLONE:HUMGS05060
SEQUENCE DESCRIPTION:
GATCTAATAA AAATTGAAAT ATGCCTTTTA GAATGGCTTT CAAAGATAAC AAATGCTGGA 60
GAGGATGTAG AACAACTGGA ACCTCTCGGT TATTGCTGGT GAGACAGCCG CTTTGAAAAA 120
GTTTGAGTTT CTTACAAAAT TAAACTTACA CTTACACTTA CCATATGACC CAAAAATTCC 180
ACTGCTTGCT CTTTACTCAA GTATAAGGAA AATCTATGTA CACACAAAAC TNGTACGTGA 240
ATATTTATTA ATAGTCATTT TATGCCCCAA ACTAGNAATA GTCCAAATGT TCTGGGN 297

SEQ ID NO:4256
SEQUENCE LENGTH:208
SEQUENCE TYPE:nucleic acid
TOPOLOGY:linear
CLONE:HUMGS05061
SEQUENCE DESCRIPTION:
GATCTNTATT NTTTTNGCAA CAGACTTCAA GTGGTCCCTC TTTTTTCCCT ACACACTCAG 60
CTCTTCTCTA AAAGCAAGGA TTTGGAATGA NCAATTCAGT TTGACAGAAA AAGCTGAATA 120
AAATGGCATC TGTAATTGAN TTGTTGGAAA TGCCTTTTTN GCTCCTAAAT NTTAAACATC 180
ATTAATAAAA TANTGTTTCA CAAAGAAA 208

SEQ ID NO:4257
SEQUENCE LENGTH:208
SEQUENCE TYPE:nucleic acid

TOPOLOGY:linear
CLONE:HUMGS05062
SEQUENCE DESCRIPTION:
GATCACAGGA AGACTTTGTA AGGACAGTTT AAGTTCTCCT GCAAGGTTTA ATTTGTTATC 60
ATGTAAATAT TCCAAAGCAG GNTGCCTTGT GGTTTTGGCC AGCCTTGTGC TATGTTGATA 120
AGATTGATTT ACTGCTTAAA ATCACTTTAC TTTATCCAAT TTTTACTGAA CTTTTTATGT 180
AAAAAAATAA AATCAATTAA AGAACAAA 208

SEQ ID NO:4258
SEQUENCE LENGTH:126
SEQUENCE TYPE:nucleic acid
TOPOLOGY:linear
CLONE:HUMGS05063
SEQUENCE DESCRIPTION:
GATCAGTTTT CTATTTTAAT GTGTAAAAAA AATAACTTGT CGTATCCCAT TTAAAGGCCA 60
ATTTCTGTAT TCAGGCAGGC ATATGTACAT ACATGAATAA AGCCAACAAA AGTGTGCACA 120
TGTAAA 126

SEQ ID NO:4259
SEQUENCE LENGTH:552
SEQUENCE TYPE:nucleic acid
TOPOLOGY:linear
CLONE:HUMGS05064
SEQUENCE DESCRIPTION:
GATCGGGGTG CCCCTCAGCC TTCAGACACT CTGAGCCCCA GANGCAGCAA AGGGTGAGCC 60
TCCTGCCTTG GTTTCTACCA CCATCTGGTG GTCAATGGCG GGACTGCAGT CGGCCAGGGA 120
CTGCGGTTGA CCTACACCTT CCTCCCCTGA GACATCAGCT GGCATGCCTC CAAGCCCTCT 180
GCGCATGCCC TCATTCTCTC CTGCCCCCTG GCTGACTTTG CATGTTGACT TGAACCCCTC 240
TGGGGAATCT NTGTGCATGT CCACAGCCCC TGGTTATGCT CTCACAGCCA CTGCCCCTNT 300
CTNTGTTCAG GCTGAGCTGG CTGACCGACC CAATCCCNCT AACCGGCCTC TGCCCGNTGA 360
NCCGGTGGTT GAGAAGCCCG AAGTCTNAGG GGGCCAGCAA GGCCCCAACC NCAAGGNAGG 420
CAATTGCTTG CNGGACCCCA AGGGGNCGGN TNGCCAATGG GGGTGNACTT TGCCGGGCCA 480
AAGGGGNTNN GAATTTCCGG CNCTTAAGTN GTNACCCTTC AANANNAANG GGGCAAACGG 540
GTTTNGNAAA AN 552

SEQ ID NO:4260
SEQUENCE LENGTH:180
SEQUENCE TYPE:nucleic acid
TOPOLOGY:linear
CLONE:HUMGS05065
SEQUENCE DESCRIPTION:
GATCAGACCT TCTTGGGGTC TTCTGGCCAG AGCTTGTCAC CAGCCCCATG GCCTCTCCAG 60
GCGTGCTCAT GCCCACAACC CGCGGCCAGC CCCACGTGGT GCCGCTCAGC CTTCTCTGCC 120
CCTCCTGGGA ATCTGTCATT CGTGGGTGCT TCAGAGTAAA ATCAATGAGT TTCTGAGAAA 180

SEQ ID NO:4261
SEQUENCE LENGTH:553
SEQUENCE TYPE:nucleic acid
TOPOLOGY:linear
CLONE:HUMGS05066
SEQUENCE DESCRIPTION:
GATCAGCGTG CGGGGTGTGA AGATAGGCGT CAAGGCCGAT GACTCCCAGG AGGCCAAGGG 60
GAATAAATGT AGCCACTTTT TCCAGTTAAA AAACATCTCT TTCTGCGGAT ATCATCCAAA 120
GAACAACAAG TACTTTGGGT TCATCACCAA GCACCCCGCC GACCACCGGT TTGCCTGCCA 180
CGTCTTTGTG TCTGAAGACT CCACCAAAGC CCTGGCAGAG TCCGTGGGGA GAGCATTCCA 240
GCAGTTCTAC AAGCAGTTTG TGGAGTACAC CTGCCCCACA GAAGATATCT ACCTGGAGTA 300
GCTGTGCAGC CCCGNCCTTC TTGGGGTCCC CCCAGCCCCT NAGGGCCAAG TTGCCAGGGA 360
CAAGCTTGGC TTGCTGGACA AGGGATGTTG GCAACTTGNT TTNAGGGAGG GGGGCAACTT 420
GNCCAACCGG CCANNNGGGN CAAAGGGAAA GTTNGGGGGG NCCCGTTTGG NCCAANGGGT 480
TAGGGGGAAG GGGTTGGGGG GNAATTNGGG GGNGGNGGGG AAATTTNCNA GTTTTANTTG 540
TTAATTTTTT AAA 553

SEQ ID NO:4262
SEQUENCE LENGTH:314
SEQUENCE TYPE:nucleic acid
TOPOLOGY:linear
CLONE:HUMGS05068
SEQUENCE DESCRIPTION:
GATCTCCCGG ACCCCTGAGG TCANATGCGT GGTGGTGGAC GTGAGCCACG AAGACCCTGA 60
GGTCAAGTTC AACTGGTACG TGGACGGCGT GGAGGTGCAT AATGCCAAGA CAAAGCCGCG 120
GGAGGAGCAG TACAACAGCA CGTACCGTGN GGTCAGCGTC CTCACCGTCC TGCACCAGGA 180
CTGGCTGAAT NGCAAGGAGT ACAAGTGNAA GGTCTCCAAC ANAGCCCTCC CAGCCCCCAT 240
CGNGAAAACC ATCTCCAAAG CCAAAGGGCA GCCCCGAGAA CCACAGGTGT ACACCCTGCC 300
CNCATCCCGN GACN 314

SEQ ID NO:4263
SEQUENCE LENGTH:176
SEQUENCE TYPE:nucleic acid
TOPOLOGY:linear
CLONE:HUMGS05069
SEQUENCE DESCRIPTION:
GATCCAGGGA ACAATGACTA TAGGTGAGCA GATGCTTTAA ATTAAGGAAA GATGGAGAAG 60
AAGCCAACTA GGAAATTAGA GAACACAGCA AGCAAAGAGA AGCAAGCGAG TCCCCTTGAG 120
TGATGCCAAT ACTTTTTTTT TTTTTTTTTG NGANANATTT TNTNNCCANT NTGGTN 176

SEQ ID NO:4264
SEQUENCE LENGTH:389
SEQUENCE TYPE:nucleic acid
TOPOLOGY:linear
CLONE:HUMGS05070

SEQUENCE DESCRIPTION:

```
GATCTTTNAG GAAAGACGGA TTGCNTTAGA AATAGACAGT ATATTTTTNG TCACAAGAGC  60
CCAGCAGGGC CTCAAAGTTG GGGCAGGCTG GCTGGCCCGT NATGTTCCTC AAAAGCACCC 120
TTGACGTCAA GTNTCCTTCC CCTTTCCCCA CTCCCTGGCT CTCAGAAGGT ATTCCTTTNG 180
TNTACAGTGT GTAAAGTGTA AATCCTTTTC NTTTATAAAC TTTAGAGTAG CATGAGAGAA 240
TTGTATCATT TGAACAACTA GGCTTCAGCA TATTTNTAGC AATCCATGTT AGTTTTGNAC 300
TTTCCTGTTG CCACAACCCC TGTTTTTAAT ACTGTACCTT TANTTAAATT TCNNGGTTNN 360
TAGTTTTTTG CAACAGGTTT TTTCCCAAA                                   389
```

SEQ ID NO:4265
SEQUENCE LENGTH:436
SEQUENCE TYPE:nucleic acid
TOPOLOGY:linear
CLONE:HUMGS05071
SEQUENCE DESCRIPTION:

```
GATCACCATG AAAGAAGAAT AGTTTTTTGT CCCCAGAGAC ATTCATTTAG TTGATATAAT  60
CCTACCAGAA GGAAAGCACT AAGAAACACT CGTTTGTTGT TTTTAAAGGC AACAGACTTA 120
AAGTTGTCCT CAGCCAAGGA AAAATGATAC TGCAACTTTA AAATTTAAAG TATCTTGCAC 180
TGATAAATAT ATTTAANANT TGTATGTTTA TAAAGTTATT AATTTGTNNN GNCNGTGTTA 240
CAAAATGTTC AGTTTATATT GTTTTAGATT GTTTTGTAAT TTTTAAAGGT GTAAAATAAC 300
ATATTTNTCC TTTATGGNNA TCTATAAAAC TTTCTGGTAG TAAAATGTTT TCATTTTTAC 360
TGGGTATATT ATTTGCTTCC ATGNTTTTTG TNNCCNTCCA TAAGGNTTTN NTGCCAGNTT 420
TTTTTTTTNN CAGGNN                                                 436
```

SEQ ID NO:4266
SEQUENCE LENGTH:533
SEQUENCE TYPE:nucleic acid
TOPOLOGY:linear
CLONE:HUMGS05073
SEQUENCE DESCRIPTION:

```
GATCTGTCCC CATCCGAAGA GGCTTTGTGA ACTGCCTGCT GAATGGAAGG AAAGCAACTT  60
TAAAACATTT AGAGTTTTGC TGAGTATAGC CAAATGCCTG TNCTGAAACT TATAGGAAAT 120
TGGAATTTTG TTTAAAATTT TACACTTTAA GGTAGCAAAT GTCAATTATT NGNCCGAGCA 180
TATTGTGAAG AATTAAACTA AAATTAATAC GTACAGTTTT CTCTAATTAG GTGACTGTTC 240
ATAATGGGTA TATTTGGAAT TTAGAAGAAA TAATTTTGTA GNACTTGTGG CCCTTATGTT 300
TAACAGATTA ATTCAGCATT GGCGTATTTN CTTTGTCCCA ATACAAGGAT GCCAAAGGAG 360
GGAAACAGGA AAAATTGCCG TCCCATTTTT AAAAATTTTA GGGGTTTGGN TGGTTTTAAA 420
CCCNTAAATT TGTTTCCCAN GGANCNTTTT TTTGGGGACC TTNCTAAATT TTTTGGGGGN 480
CATTTTNAAN TTAAAGGNAT TGNNGGGNNC NAAGGNNGTN TTTGANTTNN TGN        533
```

SEQ ID NO:4267
SEQUENCE LENGTH:124
SEQUENCE TYPE:nucleic acid
TOPOLOGY:linear
CLONE:HUMGS05074

SEQUENCE DESCRIPTION:
GATCAAGGAA GAGATGGAGG CTTTCCCCGA AAAGTTCTAG CTGAGTGGCA GAAGTGAGAA 60
TTTGTAAACT TATGTACAAT GTACGTGTAA ATAAATGGAT TGAATTTCAG TTTGTCATCA 120
GAAA 124

SEQ ID NO:4268
SEQUENCE LENGTH:451
SEQUENCE TYPE:nucleic acid
TOPOLOGY:linear
CLONE:HUMGS05075
SEQUENCE DESCRIPTION:
GATCTGGGAC AGATTTCACT CTCACCATCA CCAACCTGCA GCCTGAAGAT TTTCCAACTN 60
ATTACTGCCA ACAGTATGAT AGTTACCCGC TCACTTTCGG CGGAGGGACC AAGGTGGAGG 120
TCAATCGANC TGTGGCTGCA CCATCTNTNT TCATCTTCCC GCCATCTGAT GAGCAGTTGA 180
AATCTGGAAC TGCCTCTNTT GTGTGCCTGC TGAATAACTT CTATCCCAGA GAGGCCAAAG 240
TACAGTGGAA GGTGGATAAC GCCCTCCAAT CGGGTAACTC CCAGGAGAGT NTCACAGAGC 300
AGGACAGCAA GGACAGCACC TACAGNCTCA GCAGCACCCT TGACGNTNAG CAAAGCAGNC 360
TACGNGAAAC ACANAGTNTA CGGCTGCGAA GTTNACCCCN TCAAGGGCCT TGAGCTTNGN 420
CCNGTCACAA AGGNGNTTTN AAACAGGNGG N 451

SEQ ID NO:4269
SEQUENCE LENGTH:197
SEQUENCE TYPE:nucleic acid
TOPOLOGY:linear
CLONE:HUMGS05076
SEQUENCE DESCRIPTION:
GATCTAGAAA CTGAGTTGTG GAGCTGACTC TAATCAAATG TGATGATTGG AATTAGACCA 60
TTTGGCCTTT GANCTTTCAT AGGAAAAATG ACCCAACANT TCTNAGCATG AGCTACCTCA 120
TCTCTAGAAG CTGGGATGGA CTTACTATTC TNGTNTATAT NGNAGATACT GAAAGGTGCT 180
ATGCTTCTNT NATTATN 197

SEQ ID NO:4270
SEQUENCE LENGTH:69
SEQUENCE TYPE:nucleic acid
TOPOLOGY:linear
CLONE:HUMGS05077
SEQUENCE DESCRIPTION:
GATCCCATCC CCTTGTCTGA ACTGGAGCCA TGGGCACAAA GGGCCCAGAT TAAAGTCTTT 60
ATCCTCAAA 69

SEQ ID NO:4271
SEQUENCE LENGTH:54
SEQUENCE TYPE:nucleic acid
TOPOLOGY:linear
CLONE:HUMGS05079

SEQUENCE DESCRIPTION:
GATCTTGCCT CCCATCAGCC ATCTTTCTCC CAATAAATTT TTGTTTTGTG CAAA 54

SEQ ID NO:4272
SEQUENCE LENGTH:103
SEQUENCE TYPE:nucleic acid
TOPOLOGY:linear
CLONE:HUMGS05080
SEQUENCE DESCRIPTION:
GATCTAACCA ATTNAAATAT GANTTCATAC TCNAAAGTAT CAACAAAAAA ATGAAAAGAA 60
ATAGCTGGTT TGGNTGTAAG AAATGGTGTA ATTAATGGNT TTN 103

SEQ ID NO:4273
SEQUENCE LENGTH:449
SEQUENCE TYPE:nucleic acid
TOPOLOGY:linear
CLONE:HUMGS05081
SEQUENCE DESCRIPTION:
GATCACTGCT GGCCTACTCT GGTTACGGGG ACACTGACGC CCGGGTCACC GCTGCCATAG 60
CCAGTAACAT CTGGGCCGCC TACGACCGGA ACGGGAACCA AGCGTTTAAT GAAGACAATC 120
TCAAATTCAT CCTCATGGAC TGCATGGAGG GCCGTGTAGC CATCACCCGA GTGGCCAACC 180
TTCTGCTGTG TATGTATGCC AAGGAGACCG TGGGCTTTNN NNTNCTCAAG GCCAAGGCCC 240
AGGCTTTGGT GCAGTACCTG GAGGAGCCCC TNACCCAAGT GGCGGCATCT TAACGGCATT 300
GGTGGAAGCT GGGGGTCAGA AAAGAGAAAT GACCCATTTG NGAGGGGCTG GGGGNCCTCC 360
TAGGAAGAAC CTTTNTTAGG ACAATGGGGG GNAAGGGATG GGACTTTTNN TTTTTTTCCC 420
AAGNATTAAA ACTTNAAACT CCCTGTAAA 449

SEQ ID NO:4274
SEQUENCE LENGTH:247
SEQUENCE TYPE:nucleic acid
TOPOLOGY:linear
CLONE:HUMGS05082
SEQUENCE DESCRIPTION:
GATCTNCATA ANCAGGGTTC CTGAGCTCAC TGAAAAGACT ATTGTCCCTT AGGAAAAGCA 60
TTTCCTGTGT NTCGTNAGCA TCTGGCTCCA GGACAGACCT TCAACTTCCA AATTGGATAC 120
TGCTGCCAAG AAGTTGCTCT GAAGTNAGTT TCTATCATNC TNCTCTTTGA TTCAAAGCAC 180
TGTTTCTCTC ACTGGGCCTC CAACCATGTC CCCTTCTCCT NAGCACCACA AATAATCAAA 240
ACCCAAA 247

SEQ ID NO:4275
SEQUENCE LENGTH:424
SEQUENCE TYPE:nucleic acid
TOPOLOGY:linear
CLONE:HUMGS05083
SEQUENCE DESCRIPTION:

GATCATTTCC CAAGATTGGT TTCCCTTGAG TTTTTGCTAA AACAAATCTT AGTAGTTTTG 60
CCCGTTTAAA ACAACTCACA ATCGTAAATG CTACTATTCC TAAGATATCT NACCTTTTNA 120
TTTCAGTTTA GCCATGTATT GTATGAGTGT ATTAGTCTAA GCAGTGAGAA TCTTTNCTAT 180
GCCTCTATTC CAGCAAAAAG TAGAAGTATC AAATAAAANG GGCAACTTTT AAAATATTAA 240
GCCTGAAGAC TTCTAAAANG ACANGANACA TGGCCTAANT ANCCAACATA GGATTTACAT 300
AGTANGTTTC ACACTACCTT ATTACCAAAA GCAAACCACC TCTTACTTTT NNNCTACATT 360
TATCCATGTA TATCTNTNGT ATGCTGGTCT TTTNCTTTTT NGCCAAANTC NANCATTTTT 420
TGNN 424

SEQ ID NO:4276
SEQUENCE LENGTH:308
SEQUENCE TYPE:nucleic acid
TOPOLOGY:linear
CLONE:HUMGS05085
SEQUENCE DESCRIPTION:
GATCTAAAAC TTCTGTTGCT TTTGCCATTC CAAAACTTTG TCTTTGCCAG AAAAGTGTTG 60
GTAACTATAA AGAAAATTAT ATATGANCAC GGCAGTTTGC ACTGTGTTTG AGTAGAACGT 120
GTAAATGAAT TGTNCCCACC TTTGGTTTGC CAGTAAGTGA CTGGATTCTT GGCACATTTA 180
TGTTCACCAA AGTAGAACAA GAAGATATTA TTNCTATTTA TCAAGCAAAA GGAATTTTAA 240
GATTTTNTTT TTNCNTTAAA ACCAANTTGG GATTTTNNNN TTTNNTTTTN TTTGGGGGAA 300
AAGGNTTN 308

SEQ ID NO:4277
SEQUENCE LENGTH:374
SEQUENCE TYPE:nucleic acid
TOPOLOGY:linear
CLONE:HUMGS05086
SEQUENCE DESCRIPTION:
GATCGAGTCC GCCATGGAGA GGGACCGCTA CATGAGCCCC ATGGAGGCCC AGGAGTTTGG 60
CATCTTAGAC AAGGTTCTGG TCCACCCTCC CCAGGACGGT GAGGATGAGC CCACGCTGGT 120
GCAGAAGGAG CCTGTAGAAG CAGCGCCGGC AGCAGAACCT GTCCCAGCTA GCACCTGAGA 180
GCTGGGCCTC CTCTCCAGAN TCATGTGGAG GGGCCAGAGG CTGCCAGACC CCCAGCTGGG 240
CCCTGCTCAC CCCTTGTTGC TGGGCTTGGA GGGGCCTNTT GAGGAACTTT TAATTTGCAG 300
GGGTGCCCGN TATGGACGGG GCATTNCAGC TGAGACACTG TGGATTTTAA ATTAAATCTT 360
TTGTGGTCTT TAAA 374

SEQ ID NO:4278
SEQUENCE LENGTH:510
SEQUENCE TYPE:nucleic acid
TOPOLOGY:linear
CLONE:HUMGS05087
SEQUENCE DESCRIPTION:
GATCTGTGCT TTCCAGCTCA CCCCCCACAC TCACTCCTGA GACCCCTGGC CTCCGGCGTC 60
ACNTCCAGCN TCTGTTCCCC TAGTAAGTGC CTTCCATGTC GGCCTCTAAC CCCAGGCCCC 120
GAGGACCCAG ACCCAGTGGG GAGGCGGACG TTCCAGCCGG CATGGNTGGG AACTGCAGAC 180

CTGTCCTCCT GGTGGGTCCA GGGGCCCCTC CAGCTTGTGG AGCCCCACAN TGGGGTGCCG 240
CCTGCCCGTT TTTTTCCCAT GGAGCCCCAG CCCCCTTTGG GCCCAGGGAC ACCAGCCAGG 300
CTCTGTGCTG ACCCTCCTGT TGCACCCAGN CCTGGTNTCA GCAGCGACCA CCCCTTGCNT 360
TCACCCTTTG GAGCTTTTGC AATGTTTCCA NTAAANCCCN GGGNTGGGTT NGGNAAGGTT 420
GGGAGGTTGT TAAGGTTGTT TNGGTGNNCT TTTTGAAAGG GGNAGGATNA ATTAACCTTG 480
ANCCNNTGGG GCGGNGNGNC TTTTGNGGTN 510

SEQ ID NO:4279
SEQUENCE LENGTH:466
SEQUENCE TYPE:nucleic acid
TOPOLOGY:linear
CLONE:HUMGS05088
SEQUENCE DESCRIPTION:
GATCTAGCTT GGAGAGCTTG TGAATCTTAG GAGAGCTAGG CATAGAATGA CTAAAGTTTG 60
TCACCGCATG AGATAAAATA AATGAAAAGC CACTTCTGGA GGGAATGAGG CAGTCTGCCT 120
TTNTTGGAGA GATACATATT GTTGAACAGC TCTGGGCAGT TGATTGTTTG CCTGGGAGAG 180
CTAACCATGT TTACTCTACA CTTGACCTGT GATGGTCTGT ATTGATTGTN TCACACGCTT 240
AGCTTTCTAA GGCTAACCAG GTCATCGTCC TCAGTCCCTG CCTGCAACTA GGTTGCCAAC 300
TGGCCCTTGA TTTCCCACCC AACTTGTTCA GTTTGCCCTC AATCCTGGGG CCTCACTNAT 360
TTCCCCAAAA NGATATGGTT TGGTACCACC TGTAGTTTNC CTGTTGCATG GTTAAACCTG 420
ACTTTTTGGG GAAACTNAAA ATANAACANT TTTTCAAAAC GGGAAA 466

SEQ ID NO:4280
SEQUENCE LENGTH:470
SEQUENCE TYPE:nucleic acid
TOPOLOGY:linear
CLONE:HUMGS05089
SEQUENCE DESCRIPTION:
GATCGAGCCA CTGCTGAAGA ATGTCTAAAG CACCCCTGGT TGACACAGAG CAGTATTCAA 60
GAGCCTTCTT TCAGGATGGA AAAGGCACTA GANGAAGCAA ATGCCCTCCA AGAAGGTCAT 120
TCTNTGCCTG ANATTAATTC GGATACCGAC AAATCAGAAA CCGAGGAATC CATTGTAACC 180
GAAGAGTTAA TTGTAGTTAC TTCATATACT CTAGGACAAT GCAGACAGTC TGAAAANGAG 240
AAAATGGNGC AAAAGGCCAT TTCCAAACGA TTTAAATTTG AGGAACCTTT GCTACANGAN 300
ATTCCAGGNG ATTTTATCTA CTGAGCAATA TTTCCCTTTN GAACTTCAAG ATTTCTNCAC 360
TGNANAATTN TTAATNTTTN TTTTTTTGGG GCCCTCTGGG CCAAATTGGN GNCATGTTCC 420
TTTGNAGGTG GGTTAACCCA GTTNTCACTT TNCACCAGTC CANNTNTTTN 470

SEQ ID NO:4281
SEQUENCE LENGTH:476
SEQUENCE TYPE:nucleic acid
TOPOLOGY:linear
CLONE:HUMGS05090
SEQUENCE DESCRIPTION:
GATCTACAGT CACCATATAT ATCAGCAGGA CCTAAGGNAA AAGATTTTGG ATGTTGGGAA 60
AAGGTTAGAT GTGACTGGAT TTTGCATGAC AGGNAAGCCT GGTATAATCT GTGTGGAGGG 120

TTTCAAAGAG CACTGTGAGG NGTTCTGGCA CACAATTAGG TACCCCAATT GGAAGCACAT 180
TTCCTGTAAG CATGCTGAAA GCGTGGAGAC AGAAGGAAAT GGTGAGGACC TGCGCCTTTN 240
CCATTCTTTT GAAGAGTTAC TCCTTGAGGC TCATGGTGAC TATGGATTAN GGAATGACTA 300
TCACATGANT CTGGGCCAAT TCTTAGAATT TNTCAAGNTG CACAAAATTT NAGGCATGTT 360
TTTTTCCNGT TACTATTTTG GGTNTTNTNA AGCAAAAANT TTCAGNCTTC NTAAAAAGCG 420
GNTTAAAGGG GGNCCTATTG NCCTGTNAAT TTTCCACTNA GGNCAGGTNT TTCTTN 476

SEQ ID NO:4282
SEQUENCE LENGTH:277
SEQUENCE TYPE:nucleic acid
TOPOLOGY:linear
CLONE:HUMGS05092
SEQUENCE DESCRIPTION:
GATCTGAATT GTNCGAAAAG CAGCCAGTGC CTAANTTGTA GCTCCACTTA TGCCAACTGT 60
ATATATACCT CTCGTGAGCA TAGCAAGTNA TTTANTATTT TNAAAAGATG GCTAAAATCC 120
TTTTAATGAA CAGCACTAAA GTTATATGTA TTAGAGGAGA ATTATTGANT GANATGGAGA 180
AAGAGTTCTG AAATTANTAT TTNCATTTGG GCTTTTTTAC AGATAATATT ATATNNCTAA 240
GTGACCAGAC GAAAGAGAAG GGNGTAGAAA AGGNTGN 277

SEQ ID NO:4283
SEQUENCE LENGTH:314
SEQUENCE TYPE:nucleic acid
TOPOLOGY:linear
CLONE:HUMGS05094
SEQUENCE DESCRIPTION:
GATCCCCAGA CCTCACTTGC CTCTGCCTCC ATCTTGGCCC TGATTCAACC CTAAGATAAT 60
AGCACAACAA AATTCTNCAT AAAGATATTT TAATTCACCT GTNCCGTGCT ATATGGAGGA 120
GGCCAAGTCC ATTTAGTGAC ATTNCTNCCC ATAATGTGAG TGGGGAGGAT TGTGGGGAGG 180
AGGGGCTTTG GGTTCCTGTG TTTGTGCATA TGAAGGGAGA TGGGGGTTAG GTGGAGGAGG 240
AGAGCAGCGT GGTTAGCTAA GGTTATTGCT TTTTGTGGCA AATCTAATTA AATGACAGGA 300
ATCTCTTCAC GAAA 314

SEQ ID NO:4284
SEQUENCE LENGTH:489
SEQUENCE TYPE:nucleic acid
TOPOLOGY:linear
CLONE:HUMGS05095
SEQUENCE DESCRIPTION:
GATCTTCATG GAGCTGAATG ACGCCTGGTC GGAGTTTGAG AACCAGGGCT CNCGGCCCCT 60
CTTCTGAGGC CGCAGTGGTG GTGGCTGCGC ACACAGCTCC ACAGGTTGGG AGCCGTCGTG 120
GGACCTGGGT CCCCACCGTN AGGACCCCGT GGGCGACAGA GGTGTGGCCA GGGTGGGGCT 180
CCGAGCCCCG GGTCACCGCC CGCCCAGCGT TCCAGGCACA TGAAGAGAAA GCATTCCAAA 240
GCCTCTNATT GTTGTTTCCT TTTTNTCCTC CCGAAGAACA GCTGATTCAT GCTCCTCCCG 300
CAATTNTNAC GTCTGTAATT TATTTGGTGT TTCGGGCGTN GTCTCTTGGA GCCCCGGCAC 360
GTTNGTGGGG CCCACGGTTG CTTGGCGGTT CAATGGGGCC CTTGGTGTTT TGCACCGGAA 420

CTTTTGTAAA TCAAGTNCCG TGGGTTGGTN TTGTTACAAG AATTNAAAAC TAATTTTTCC 480
CGNTTGAAA 489

SEQ ID NO:4285
SEQUENCE LENGTH:292
SEQUENCE TYPE:nucleic acid
TOPOLOGY:linear
CLONE:HUMGS05096
SEQUENCE DESCRIPTION:
GATCATATTT CCCCTTGGAC ACTTGGTTAG ACGCCTTCCA GGTCAGGATG CACATTTCTG 60
GATTGTGGTT CCATGCAGCC TTGGGGCATT ATGGGTTCTT CCCCCACTTC CCCTCCAAGA 120
CCCTGTGTTC ATTTGGTGTT CCTGGAAGCA GGTGCTACAA CATGTNAGGC ATTCGGGGAA 180
GCTGCACATG TGCCACACAG TGACTTGGCC CCAGACGCAT AGACTGAGGT ATAAAGACAA 240
GTATGAATAT TACTCTCAAA ATCTTTGTAT AAATAAATAT TTTTGGGGCA AA 292

SEQ ID NO:4286
SEQUENCE LENGTH:173
SEQUENCE TYPE:nucleic acid
TOPOLOGY:linear
CLONE:HUMGS05099
SEQUENCE DESCRIPTION:
GATCCCAGTG GAGGCTGTAA CCCACCTGCC CCCGCACCAC CCCCCNTGAC CCCTGTTACC 60
GCATTTGTGT GTATTAATGC TGAAGAATTA AATGTTTAAA GAGTTTAAAT TTTGAAGGCG 120
TTTGCTATAT ACAGTTGTCC TGCATTATTA TAAAGAGTTT TCAGGAAGTT AAA 173

SEQ ID NO:4287
SEQUENCE LENGTH:307
SEQUENCE TYPE:nucleic acid
TOPOLOGY:linear
CLONE:HUMGS05100
SEQUENCE DESCRIPTION:
GATCAGAAAT GTGAAGCACA AATGCTGCTT CTCCCCCTCC TGTTCCCATG TCAGAGATGG 60
AGAAGAAAGC ACAGTGAGCT GTTCACCAGT CCAGCGGTGA CCTCGGAGTC CTTTGTAACA 120
GTGTTTCCAA TANCTTCTGC CAATAAAACA GAATTTNNGT TAGAACCTAT CTGCTGTCCC 180
TCAACTAGTG TCTGATAGCT GAGAATGTAA TTCGTTTNTN TGTCTATTAG AAAATTAGGC 240
AGGCACCACA GACAGTAGCA ACATAAAGGA ACCTGCTTAG AAGNGGTTTT NATGGGNNNN 300
NNCNCNN 307

SEQ ID NO:4288
SEQUENCE LENGTH:244
SEQUENCE TYPE:nucleic acid
TOPOLOGY:linear
CLONE:HUMGS05102
SEQUENCE DESCRIPTION:
GATCCTTAAA TCTCCATTCT GTTTGTGGTT GCCCCCTCAA CCTCCCCTAC ACCCTTCCTA 60

TTCTTTTNCA TTCTTCTTGC AGTTCTGGGA GTAAAGCTCC CAGCATATTT AGATAATAGG 120
GCAGGGGAAG CACCCTCTTT CTTTCTAGAC TGGATTATGC TCACATGCTC CCTTGCCCTG 180
ACATTTTTGT AAATNCTGTG CCCTTTGCTG TAGCTACACT TCAGATTAAA GTAGGAGAAA 240
GAAA 244

SEQ ID NO:4289
SEQUENCE LENGTH:69
SEQUENCE TYPE:nucleic acid
TOPOLOGY:linear
CLONE:HUMGS05103
SEQUENCE DESCRIPTION:
GATCAAAGTT GGCGGGGGCT TGGCTGTGCC CTCAGATTCC GCACCAATAA AGCCTTCAAA 60
CTCCCTAAA 69

SEQ ID NO:4290
SEQUENCE LENGTH:341
SEQUENCE TYPE:nucleic acid
TOPOLOGY:linear
CLONE:HUMGS05104
SEQUENCE DESCRIPTION:
GATCTTTNAT CCTTTGTTGC AAAAGTGTGG ACACTTTTNA NAGCTTGGCA GATTTNAACT 60
CCAGAAGCAC TTTATGAAAT GGTACACTGA CTAATCCAGA AGACATTTCC AACAGTTTGC 120
CAGTGGTTCC TCACTACACT GGTACTGAAA GTNTAATTNN TTAGAGCCAA AAAACTGGAG 180
AAACAAATAT CCTGCCACCT CTAACAAGTA CATGAGTACT TGATTTTAAT GGTATAAGGC 240
AGAGCCTTTN NTTCCTCTTC TTGATAGATG AGGCCATGGT GTAAATGGAA GTTTCAGAGA 300
GGACAAAATA AAACGGAATT CCATTTTTTT NTCACTGTAA A 341

SEQ ID NO:4291
SEQUENCE LENGTH:395
SEQUENCE TYPE:nucleic acid
TOPOLOGY:linear
CLONE:HUMGS05105
SEQUENCE DESCRIPTION:
GATCTTAGAG TCACTTTGGA ATAAGNTCTT ACATAAATAC CCCCAGCCTT TTGAGAACGG 60
GGCTTGTTAA AGGACGCGTA TGTAGGGCCC GTACCTACTG GCAGTTGGGT TCAGGGAAAT 120
GGGATTGACT TGGCCTTCAG GCTCCTTTGG TCATAATTTT AAAATATGGG AGTAGAAAAC 180
AACANAGNNT GGAATGGACT CTTAAAACAA TGAAAGNGCA TTTATCGTTT GTCCCTTGAA 240
TGTAGAATTT TTTTTTGATT TCATAATTCT NCTGGTAAAT NTGNCAGTTA AAATGGTGCA 300
TTATGTATAT ATTANTATAA TTTAGANATT CCATTTTATA NTTNTTCCTA TTCCAGGGTN 360
GCATAANGCA TTTTNNANTT TNGGGGTTTT GGNTN 395

SEQ ID NO:4292
SEQUENCE LENGTH:349
SEQUENCE TYPE:nucleic acid
TOPOLOGY:linear

CLONE:HUMGS05106
SEQUENCE DESCRIPTION:
GATCATATTT TGCAGTTTTA GTAAAAGGGA AATATTGTTA TACATTTNTT AAATATACTT 60
CCCCCATGAN GTGAAAAGGT TAATTTTNCT GANTGTTTTA AGTTGAAGTT ACTTCATGGA 120
TGTCATACCC ATGANGTGCA TTTGGNTGAG ATAGCNGAAA TTTTTTTTTA AAAAGTTTAA 180
GTACCAAAGG TAGTCTAGTC TAGAACGATA AGTTAATACG TNTTGGCTTT NNTAATTTGT 240
ACTGTAACAT CCTTATACTT TCTATTTNAA GTATATCTGT TTCTTAAGTA AACAACTTAG 300
ATATTTTCCA CACCTTTTTT TTTTTTTNCC CNTGNNGGGT CCNGGGTNN 349

SEQ ID NO:4293
SEQUENCE LENGTH:257
SEQUENCE TYPE:nucleic acid
TOPOLOGY:linear
CLONE:HUMGS05107
SEQUENCE DESCRIPTION:
GATCTGCCAT TTATTAGCTG TGGGATTTTG GGCAAATTTN TTGACTTTCT GTGCTTCANT 60
TTTCTTAGCT GTAAAATTGG AAGAGTTGTT ATGAAAATNC ACTGAAAGTA TATTTGTATA 120
CATCATAGGA TAGTGTAAGA ATATAGTATG GCTTTGAGAA ATGTTCATNA TTATNACTCC 180
CAGAGGAGTT TTAGGTATTA AGTGATGCCA AATATAATTT NTNAATTGTA TAATAAAAAT 240
CTATATNCTT ACTGAAA 257

SEQ ID NO:4294
SEQUENCE LENGTH:212
SEQUENCE TYPE:nucleic acid
TOPOLOGY:linear
CLONE:HUMGS05108
SEQUENCE DESCRIPTION:
GATCACCATC CCAGTAACCT TCNAGTCGCG GGCCCAGCTT GGGGGCCCAG AAGCTGCAAA 60
ATCCGATGAG ACTGCCGCCA AGTAAAGCCT TAGCCCGGAT GCCCACCCCT GCTGCCGCCA 120
CTGGCTGTGC CTCNCCCGTN ACCTGTGTGT TCTTTTNATA CATTTATCTT CTGTTTTTNT 180
CAAATAAAGT TCAAAGCAAC CACCTGNANA AA 212

SEQ ID NO:4295
SEQUENCE LENGTH:559
SEQUENCE TYPE:nucleic acid
TOPOLOGY:linear
CLONE:HUMGS05109
SEQUENCE DESCRIPTION:
GATCCAGTGG GGATGTTGTN ATGACTCAGT CTCCACTCTC CCTGCCCGTN ACCCTTGGAC 60
AGCCGGCCTC CATCTCCTGC AGGTCTAGNC AAAGCCTCCT ATACAGGGAT GGAAACACGT 120
ACTTGANTTG GTATCAGCAG AGGCCAGGCC AACCTCCAAG GCGCCTCATT TATGAGGTTT 180
CTAAGCGGGA CTCTGGCGCC CCAGACAGAT TCAGCGGCAG TGGGTCAGCC ACTGATTTCA 240
CACTGACANT TAGCAGGGTG GAGGTTTGAC GATGTTGGGA TTTACTATTT GCATGCAAGG 300
AACACACTGG NCTCAGACGT TCGGCCAAGG GGACCAAAGG TGGNAAATTC GAACGAACTT 360
GTGGCCTTGC ACCNATCTTT TTTTCATCTT TNCCGNCATC TTGGTTGNGC AAGTTTGNAA 420

ATNTTGGGAA CCTNGCCTCC TTTTTGGTGN TGCCTTGNCT TGAAATAACT TNNTTATTCC 480
CAAGAGGAGG GNCAAANTTC AAGTTGGGAA GGTTGGTTTA ACCGGCNTTC CAATTCNGGG 540
GNAANTCCCN TGGNGGGNN 559

SEQ ID NO:4296
SEQUENCE LENGTH:50
SEQUENCE TYPE:nucleic acid
TOPOLOGY:linear
CLONE:HUMGS05110
SEQUENCE DESCRIPTION:
GATCTCTATG TATGTGTGTA TATAAATATA GTTTTTTATC TATATATAAA 50

SEQ ID NO:4297
SEQUENCE LENGTH:548
SEQUENCE TYPE:nucleic acid
TOPOLOGY:linear
CLONE:HUMGS05111
SEQUENCE DESCRIPTION:
GATCAAACGA ACTGTGGCTG CACCATCTTT NTTCATCTTC CCGCCATCTG ATGAGCAGTT 60
GANATNTGGA ACTGCCTCTT TTGTNTGCCT GCTGAATAAN TTCTATCCCA GAGAGGCCAA 120
AGTACAGTGG AAGGTGGATA ACGCCCTCCA ATCGGGTAAC TCCCAGGAGA GTGTCACAGA 180
GCAGGACAGC AAGGACAGCA CCTACAGCNT NAGCAGCACC CTGACGCTGA GCAAAGCAGA 240
CTACGAGANA CACAAAGTNT ACGNCTGCNA AGTCACCCAT CAGGGCCTGA GCTCGCCCGT 300
CACAAAGAGC TTCAACAGGG GAGAGTTTTA AGANGGGAGA AGTGCCCCCN ANCTGGNTCC 360
TCAGTTCCAG CNTGACCCTC TCCNATCCTT TGGCCTGTGA CCCTTTTTTT TCCACAGGGG 420
GNCCTACCCN TTTTGGNGGT CCTCCAGCTC ATTTTTTTAA CCTTAACCCC GNTTNNTTCT 480
TCCTTTGGGG TTTAAATTAT TGNTTAATTT TTGGGGGNGG ATTGNTTTAA ATTAAAGGGA 540
ATNTTTTN 548

SEQ ID NO:4298
SEQUENCE LENGTH:415
SEQUENCE TYPE:nucleic acid
TOPOLOGY:linear
CLONE:HUMGS05112
SEQUENCE DESCRIPTION:
GATCAGCGCC TGAGAAGTCG TCGGGCGATG TCCCCGCGCC GTGTCCCTCT CCAAGCNCCG 60
CCCCTGGGTG GGCTCGGTGG AGCAGACCCC GCGCAAGAGG CTGCGGTGAG CCAATTTAGA 120
GCCCAAAGAG CCCCGAGGGA ACCTGCCGGG GCAGCGGACG TTGGAAGGGC GCTGGGCCTC 180
GGNTGGNACC GTTCATGTAG CAGCAACCGG CGGCGNNTGC CCAGNAGCAG CGTTCGGTTT 240
TTTTTTTAAA TTTTNAAAAC TGTGCAATGT ATTAATAACG TNTTTTTATA TCTAAATGGT 300
ATTCTGCACG AGANGNTACA CTGGGTCCCA AAGGTGTAAA AGCTTTTTAA GAGTCATTGT 360
ATTATAAAAN TNNNTTTAAT CTNTTGGTTG AAATCTTNAA GTGNNANAGT ATTNN 415

SEQ ID NO:4299
SEQUENCE LENGTH:156

SEQUENCE TYPE:nucleic acid
TOPOLOGY:linear
CLONE:HUMGS05113
SEQUENCE DESCRIPTION:
GATCTAATCT NAGATGTAAA CATTCAAATT NACCCTGTAG ACTNGGGTGA CAAGTTTCGN 60
TTGGTCATAG CTAGTACCTT GTATGANGAT GGTACCCTGG ATGATGGTGA ATACAACCCC 120
ACTNATGATA GGCCTTCCAG GGCTGACCAG TTTNAN 156

SEQ ID NO:4300
SEQUENCE LENGTH:310
SEQUENCE TYPE:nucleic acid
TOPOLOGY:linear
CLONE:HUMGS05114
SEQUENCE DESCRIPTION:
GATCTTACTT TCATCCAGTC TAATAGGTTT GGAGATTAAA CCTTTNCTCA ACTTGTGCTG 60
TTTATATAGC CAAGCTTCCG TCAATAAGGC TTCATTGTGA CTTTAACAAA CATNATCTTC 120
CCACATACCA GGAACTATTG GACATTTATT TTACATGGGA AAAATTATTT GGAATAATAA 180
AGCAGGNACT TTNCCTGANG TTGCAATTTA TACTGTATGG CTNCTNNTTC ATGTTTCATC 240
TAGGTTTTAN GAAGTGAAGT ATANGTAAAT NTNGGTTCGT TAAATNGTGA TGGCGCTGGN 300
NATTACATGN 310

SEQ ID NO:4301
SEQUENCE LENGTH:231
SEQUENCE TYPE:nucleic acid
TOPOLOGY:linear
CLONE:HUMGS05115
SEQUENCE DESCRIPTION:
GATCACTCAA GCACAGGATT TTAAGACCAG CCTGGGCAAC ACAGTGAGTG AGACCCTGTC 60
TCTACAAAAA AATTAAAAAT TAGCTGGATA CTGTGGTGTG TGCCTGTAGT CCCAGGTACT 120
TGGGGCATTG AGGCAAGAGG TTACTTTNAG CTCGGGTGTT TGAGGCTATG ATGAACTATG 180
ATTGTAGTGC TGTACTCCAG CCTGTGCAAA ANAGTAAAAC CTTGTCTCAA A 231

SEQ ID NO:4302
SEQUENCE LENGTH:52
SEQUENCE TYPE:nucleic acid
TOPOLOGY:linear
CLONE:HUMGS05116
SEQUENCE DESCRIPTION:
GATCGGGCCA CTGCACTCCA GCATGGGTGA AAAAGTGAGA CCCTACCTCA AA 52

SEQ ID NO:4303
SEQUENCE LENGTH:419
SEQUENCE TYPE:nucleic acid
TOPOLOGY:linear
CLONE:HUMGS05117

SEQUENCE DESCRIPTION:
GATCACCAAC AGCCGTCCTC CCTGCGTCAT CCTGTGACTG CACAGGACTC TGGGTTCCTG 60
CTCTGTTCTG GGGTCCAAAC CTTGGTCTCC CTTTGGTCCT GCTGGGAGNT CCCCCTGCCT 120
CTTTCCCCTA CTTAGCTCCT TAGCAAAGAG ACCCTGGCCT CCACTTTGCC CTTTGGGTAC 180
AAAGAAGGAA TAGAAGATTC CGTGGCCTTG GGGGCAGGAG AGAGACACTC TCCATGAACA 240
CTTCTCCAGC CACCTCATAC CCCCTTCCCA AGGGTAAGTG CCCACGAAAG CCCAGGTCCA 300
CTCTTNGNCT CGGGTAATAC CTGTCTNGNT GCCCACAAGA TTTTATTTNA TTCTCCCCTT 360
AACCCNAGGG CAATGTCANN NTATTTGGCA GTAAAAGTGG GGGTTACAAA ANCACTAAA 419

SEQ ID NO:4304
SEQUENCE LENGTH:75
SEQUENCE TYPE:nucleic acid
TOPOLOGY:linear
CLONE:HUMGS05118
SEQUENCE DESCRIPTION:
GATCTCCCTA GGGCCCCACC TGGACCATTT TCCCTCCGTT TTATTTTGTT AATTAAATTC 60
TTTCCAAATT GGAAA 75

SEQ ID NO:4305
SEQUENCE LENGTH:171
SEQUENCE TYPE:nucleic acid
TOPOLOGY:linear
CLONE:HUMGS05119
SEQUENCE DESCRIPTION:
GATCTCAGTT CACTGCAACC TCCGCCTCCN AGGTTCAGGT GATTCCNTTG TCCCAGCTAC 60
TTGGGAGGCT GAGGCAGGAG AATCACATGA GCCTGGGAGA TGGAGGTTGC AGTGAGCCGA 120
GATTGCACCA CTGCACTCCA GCCTGGGCAA CGAGCAAACC TGCNNCTCAA A 171

SEQ ID NO:4306
SEQUENCE LENGTH:309
SEQUENCE TYPE:nucleic acid
TOPOLOGY:linear
CLONE:HUMGS05120
SEQUENCE DESCRIPTION:
GATCCCTGGT GTGTTGTGTG GGCCTCTTTA ACGTTTGCCA CTGAGCCTTA ACCTCACTGT 60
ACTTCACTGT ACTTCACACG CATTGGTGTT AACATTTTAA TCTTAGAAGA CCCTGACCCA 120
CTGAGGGTTT GTTGTGAGAA TTGCTGAAGC CACGTAGAAG CACCTTGAAA TCTGTAAAAC 180
CACAAGAAAG TACTTTATAA AAGGTATCCT TATTTGAAGT GGATAAATCT TGTAACTCGA 240
AAAGTTGTGA TTTAGAAGAC AGGATTGTTT TTGAACATTA GGAATTAAAG GCTATATCTG 300
GTCCTTAAA 309

SEQ ID NO:4307
SEQUENCE LENGTH:587
SEQUENCE TYPE:nucleic acid
TOPOLOGY:linear

CLONE:HUMGS05121
SEQUENCE DESCRIPTION:
GATCTAAAAA CTCATCTTTG GGGTAAAGAG TTAAGTGTCC AAAGGTTGTC ACAGTTCATG 60
AGGTCAGAGG GAGCTAGCCT GGCACCTGGA CTCTGCCCAT CCACAGCTGA CAGATTCCAA 120
CAGAAGTGTA TTTAAATTCT CCAGTAGACA ATGCTGGGTA AGGGAGGGGN TAGNNCTGGG 180
TTATTAAGAT ACAGGCTGCT GTATTTTACA TTGGTTGTGG GGGAAGGGGA GCCTGGAGAA 240
AACAAAGTCA CTATTCCCTT TTTTGAAACA GGAAAAAAAA TTATTTTTTG TTCAGTAAAA 300
ATGGGTAGGN GAATTCCAAT GTCCCTAGCC ACAAGGGNCC AGTTTCCACT GAGGAAGTGA 360
ACCAGTGGGG AACTCAAAAT TTCCAGGNAA CATTGGGGGG GAGGGGGAAA ANTTGGGCTT 420
TTCNCTTAAA TTTGGCAGNT NTTTNCCAGT TGGGGNCNGG GNGGGGGGCT CCTGTTTTTT 480
TTTTTTGGGG NTTGTGNTTA NTGNTNNTAT NGTACCGGNT TATTTTTGGC CCCGGGGTTT 540
TTCTTGGGNT TTNTNAAGGG TTNCCCCAAA NTTTGGGTNA AAACNTN 587

SEQ ID NO:4308
SEQUENCE LENGTH:111
SEQUENCE TYPE:nucleic acid
TOPOLOGY:linear
CLONE:HUMGS05122
SEQUENCE DESCRIPTION:
GATCCCCCCA TCTCTNCAGC ACTCTCTTGC TCCTCTCCCT TTTCATTTCC TTACTTTGTA 60
TCTTNTCAGA TTAGAACATA TTTTNNTTGT CATGACAAAG GACTCTCCAA A 111

SEQ ID NO:4309
SEQUENCE LENGTH:311
SEQUENCE TYPE:nucleic acid
TOPOLOGY:linear
CLONE:HUMGS05123
SEQUENCE DESCRIPTION:
GATCTNAACT CCTTGGCTTC TTTACTTAGT TCAAGCCCCA GCCTAGGAAA GCCAGTTACA 60
TAAAAGTTGG CTCAGGAGTC TTAGAGCTTT ACCTAAATAT GAGCCCAGAA AACGGAGGAT 120
GGGGGTGGGG CGCCTTCCTG GAGGTGACAC TTGATGGGGG TGTGTTCTGG TTACTGTTCT 180
AAGGCTGTGC CATCAGCTCC TTCCTCCCCT GTTCATTCTG CATTCTCTAG TCAGTTGGCT 240
AAGAAGTGAC TCTTGCAACT AAAAAAATTA AGAAATTCAC TTCCCCTCTA GGAGGTGATG 300
ATAGGGTTTT N 311

SEQ ID NO:4310
SEQUENCE LENGTH:366
SEQUENCE TYPE:nucleic acid
TOPOLOGY:linear
CLONE:HUMGS05124
SEQUENCE DESCRIPTION:
GATCAGAGTT TCTGTGGAAT TCTGTTTGTT AAATCAAATT AGCTGGTCTC TGAATTAAGG 60
GGGAGACGAC CTTCTCTAAG ANGAACAGGG TTCGCCCCAG TCCTCCTGCC TGGAGACAGT 120
TGATGTGTCA TGCAGAGCTC TTACTTCTCC AGCAACACTC TTCAGTNCAT AATAAGCTTA 180
ACTGATAAAC AGAATATNNA GAAAGGTGAG ACTTGGGCTT ACCATTGGGT TTAAATCATA 240

GGGACCTAGG GCGAGGGTTC AGGGCTTCTN TGGAGCAGAT ATTGTCAAGT TCATGGCCTT 300
AGGTAGCATG TATCTGGTCT TAACTCTGAT TGTAGCAAAA GTTCTGAGAG NAGCTGAGCC 360
CTGTTN 366

SEQ ID NO:4311
SEQUENCE LENGTH:240
SEQUENCE TYPE:nucleic acid
TOPOLOGY:linear
CLONE:HUMGS05127
SEQUENCE DESCRIPTION:
GATCACGCTG ACCTGGCAGC GGGATGGGGA GGAACAGACC CNGGACACAG AGCTTGTGGA 60
GACCAGGCCT GCAGGGGATG GAACCTTCCA GAAGTGGGCC GCTGTGGTGG TGCCTTCTGG 120
AGAGGAACAG AGATACACAT GCCATGGTGC AGCACGAGGG GCTNCCCCAG CCCNTNATCC 180
TGAGATGGGA GCAGTCTCCC CAGCCCACCA TCCCCATCGT GGGCATCGTT GCTGGCCTTN 240

SEQ ID NO:4312
SEQUENCE LENGTH:96
SEQUENCE TYPE:nucleic acid
TOPOLOGY:linear
CLONE:HUMGS05128
SEQUENCE DESCRIPTION:
GATCTTTTCT CCATCATGTA CTTAGTATTT CCCATTAACC TACACACTGA TTTTTNTGCT 60
ACTCCTTGTA GAAACAAAAT TNTGGNTGGA CTCAGN 96

SEQ ID NO:4313
SEQUENCE LENGTH:354
SEQUENCE TYPE:nucleic acid
TOPOLOGY:linear
CLONE:HUMGS05129
SEQUENCE DESCRIPTION:
GATCTGCTGG TCCTTGCAGG CAAAGCTACA GCCAGANTGT CCGTTTGANA CTCCTAGCTC 60
ATCTGTCACC GAGCTTNATC CGAATGTGCC ACGGAGCTTN CTCTCCACTT CCTCCGTGCA 120
GCGGCCCTGC CACAGCCCTC CCTCGGCACA CTTTGACCCT TTGTAGGGTT GGNATTAGCA 180
GGACTCGGCT ATTTAAAGCA CCAGTCTGGG GTCGCCTGGG CCCCTGCTGA CCCNTTCCTN 240
CAGAGCAGCC AGCCCAGCCC GGGAACAAGA CGGACTTNCT ATNCCTTCGG ACTCACAGCC 300
TTTGAAGAGT CAAGCTNCAC TTNAAGCTCA CTCAGTAATA TCCTTTCAAT GNGN 354

SEQ ID NO:4314
SEQUENCE LENGTH:161
SEQUENCE TYPE:nucleic acid
TOPOLOGY:linear
CLONE:HUMGS05130
SEQUENCE DESCRIPTION:
GATCCGCCAC TTTCGGCCGG AGCTCGAGGA GCGGATGCAG CGGTTTGCCC AGCAGCATCA 60
GGCCCGGCAG GCTGCCTCTT AGCCCACCAC CCTGGCCTGC TGTCCTGCGT CTATCCATGT 120

GGAATGCTGG ACAATAAAGC GAGTGCTGCC CACCCTCCAA A 161

SEQ ID NO:4315
SEQUENCE LENGTH:129
SEQUENCE TYPE:nucleic acid
TOPOLOGY:linear
CLONE:HUMGS05132
SEQUENCE DESCRIPTION:
GATCCTGGCT TTCCAGGGTG GGCAAGGGCA AGGAGCAGGC TTGGAGCCAG GGACCAGTGG 60
GGGCTGTAGG GTAAGCCCCT GAGCCTGGGA CCTACATGTG GTTTGCGTAA TAAAACATTT 120
GTATTTAAA 129

SEQ ID NO:4316
SEQUENCE LENGTH:227
SEQUENCE TYPE:nucleic acid
TOPOLOGY:linear
CLONE:HUMGS05133
SEQUENCE DESCRIPTION:
GATCTTTGAG GTGAACAACT TGCCTGTGGA ATCGTGGCAG TAACTGTTTC CACTCACAGC 60
CTGAAATTCT CCTNTGTCCC AAACCCCAGG GGGCCCCAGC AGCTTCGAAC CTACACCTGA 120
GGGCTACCAG CAGGTGGCGC TNTGGCTTTG CACTGCAAAA ACTGGGGACC AGCCCNCTTC 180
TNCCACAAAT AAAGCCCAAT AAAGCCTGAG AAGTGAGGAA AGCCAAA 227

SEQ ID NO:4317
SEQUENCE LENGTH:154
SEQUENCE TYPE:nucleic acid
TOPOLOGY:linear
CLONE:HUMGS05134
SEQUENCE DESCRIPTION:
GATCTCCGTG GATGAACTGC GTCTGGACTC TTAGATTCAT AAAATATTCG AGGGTTTGGA 60
AGTNACAGAC CCTCCCCTCT CCTCAGTGCA CTTTGGCATT TNCACGGTGT CTTCCCCGGA 120
CAGCACAGCA ATAAATNGNG TGATTGCGTG GAAA 154

SEQ ID NO:4318
SEQUENCE LENGTH:181
SEQUENCE TYPE:nucleic acid
TOPOLOGY:linear
CLONE:HUMGS05135
SEQUENCE DESCRIPTION:
GATCAGGTTT GGNCTTCTCC CACTGAGCAT GACAAGCACT CGAAAGGCTA AATCAACCAG 60
AAACATCGAA ACAAAAGCTC AGTACGATGC CAACTNTTAA GCCTGCAGGA NTCCTCCTGG 120
GTTGTCCCCC CGGCACGNTC CACAGCTGGT NGACCCGGCA GTTCCTGGNT CCTTGTTCAG 180
N 181

SEQ ID NO:4319

SEQUENCE LENGTH:315
SEQUENCE TYPE:nucleic acid
TOPOLOGY:linear
CLONE:HUMGS05136
SEQUENCE DESCRIPTION:
GATCTGCCCT TGNTNGCCTC ATACCTCTGT GCTCCACACT GCGGCCAGGC CAGCTGAGTC 60
CCTCCATCCG TGGATGCTTT CCTGCAGCTA TGTGGTATGG GGGTCATTCC TGCCTCTTGG 120
CACCAGGTTG GGGGGCATGT GCTTGTTGGG CACCAAAGTG ATGGAACCCT CAGGTGCTCT 180
CCGGGAGCCT GAACCTCCTG ACTGAGGAAC ATGGGCAGAA CATGTTTATT GCACAGAGTG 240
GGCGCTGCGC ACAGGCGTGG CTGTACACGT GCTCTCAGCT CATCATCCTT TCCAGTAACT 300
TTAAAAAAAC ATCCN 315

SEQ ID NO:4320
SEQUENCE LENGTH:255
SEQUENCE TYPE:nucleic acid
TOPOLOGY:linear
CLONE:HUMGS05137
SEQUENCE DESCRIPTION:
GATCGNGNNA CTTGGGGNGN GGCCCTAGGC ACTACCAGCA GCGCACGCTC ACCTTCGCCC 60
TCAGGGCTGA CGACCCCCTG CTCAAGCTCT TGCAGGAGGC TCAGCAAAGC TGAATTAACT 120
CAGGCAGAAG AGCACAGATG TGTGGGATTG GGGGAGGAGT GGGGACAAGA TTTTTNATCT 180
CTAAGTGAAT TTTCTAAAAA TGTATTTTAT ACCGGCTTAT TCCTAGTATT GNATAAACTA 240
GCGGGNTCAN TCAAA 255

SEQ ID NO:4321
SEQUENCE LENGTH:212
SEQUENCE TYPE:nucleic acid
TOPOLOGY:linear
CLONE:HUMGS05138
SEQUENCE DESCRIPTION:
GATCAAACAT GCATGGNTGG GTCCTCACGC AGACACACCC ACAGAAGGAC ACTAGCCTGT 60
GCACGCGCGC GTCNATCTAC ACACACACAC AAGAGTTCAT AATGTGGTGA TGGCCCTAAG 120
TTAAGCAAAA TGCTTCTGCA CACAAAACTC TCTGGTTTAC TTCAAATTAA CTCTATTTAA 180
ATAAAGTCTC TCTGACTTTT TGTGTCTTCA AA 212

SEQ ID NO:4322
SEQUENCE LENGTH:136
SEQUENCE TYPE:nucleic acid
TOPOLOGY:linear
CLONE:HUMGS05139
SEQUENCE DESCRIPTION:
GATCGAGTTA TTATGATAAC TGTGGTAATT TGTTGTTATA AATAGCAGTA ACATAGGAAG 60
GTTTTGGTAA CTNCTGACAA GAAATTTNAT GTAGAGAGTT AATTTCGAGC ACATTAAAAT 120
GACTGTCCCA CTCAAA 136

SEQ ID NO:4323
SEQUENCE LENGTH:228
SEQUENCE TYPE:nucleic acid
TOPOLOGY:linear
CLONE:HUMGS05140
SEQUENCE DESCRIPTION:
GATCTGCATT ATTCTNGGAN AGTATTCAAG TGGTATCTTG ACTATTAAAC TACGTATAGT 60
GTTNNTGNAA TAGAAAGAAA ACAGCATTGG AATTGGATTC ATGTATCGTG GGATACAGGT 120
GTTATTTCAG GTGATGTACT TGCATTATTT TCTTTAGCCA TAGTAACTTT TTGTCACAAT 180
AACTAAGTAT TCAATTATAT ATAANGAGTG AAACATTAAA ATGGNAAA 228

SEQ ID NO:4324
SEQUENCE LENGTH:297
SEQUENCE TYPE:nucleic acid
TOPOLOGY:linear
CLONE:HUMGS05141
SEQUENCE DESCRIPTION:
GATCCTGCCA CTGCACTCCA GGCTGGGCAA CAGAGTGAGA CCATGTCTCA AAAAATAAAA 60
ATAAAATAAA ATAATATCAG GNTGCATACA TCAGAGGCTG TTCCTAGTGT AAAGGCACTT 120
TGGAGGGAGA AGACTTTCAG AGTTAGGCAG ACCAACTAAG AGGTCAGCTG ANGCACCTAA 180
CCAGTTGTAA GGAGGTGAAA GACAGCACCC CAAGANGAGA TGTGCAGGAN GGAGGAAAGA 240
GGCTTGGTCA TAAAGGNTGG AGGAATTCCA AAGTGACACT GANACAGGCT GCGTTTN 297

SEQ ID NO:4325
SEQUENCE LENGTH:90
SEQUENCE TYPE:nucleic acid
TOPOLOGY:linear
CLONE:HUMGS05142
SEQUENCE DESCRIPTION:
GATCGTTCTG CTGAGTATGT TAAGCTCTTT ATGACTGTTT TTGTAGTGGT ATAGAGTACT 60
GCAGAATACA GTAAGCTGCT CTATTGTAAA 90

SEQ ID NO:4326
SEQUENCE LENGTH:411
SEQUENCE TYPE:nucleic acid
TOPOLOGY:linear
CLONE:HUMGS05143
SEQUENCE DESCRIPTION:
GATCTGCTGC TGCCATTTTA ATCTTGCTCA TTAACCTTAC TCCTTTGAGA ATTCTTTAAC 60
AATATTTAAA ATTGGTAACA AAAATAGTTT AGCCATAATT GTTTAGCCAT GTGAGTTTCA 120
GGTTGGTACA CGTTCAGACA GAACTGCTGT ATCACATTCC AATTTTGAAT AGCCAGTGAG 180
CAATCAAGTG TAGAGAAATG ATAAATGGCC TAAGAAGGCA TACAGTGGCA TAAACGATGC 240
TCTTCCTAGT AGCTTAATAG GCCACAAGCT AGTTTCTGTT GCACTCTGAA ATAAAATATG 300
CTTTAAAAAT GTAGGGANCA GTGCTTAGAA AAGCAAAANC TAGGTGTGTC ATTGANATAA 360
TAGGCATAAN ACTTAAATGT TCCATAGGCC ACTATTTGGA AGGGGGCCTT N 411

SEQ ID NO:4327
SEQUENCE LENGTH:99
SEQUENCE TYPE:nucleic acid
TOPOLOGY:linear
CLONE:HUMGS05144
SEQUENCE DESCRIPTION:
GATCTACACA TTTTTATAGT CATTTAAAAT TGTATGACTC TGTCAAATNA TTTAAGTAAT 60
TTTGGTGGAT TTTTAAAAAT AAAAAAATAA AAATAGAAA 99

SEQ ID NO:4328
SEQUENCE LENGTH:80
SEQUENCE TYPE:nucleic acid
TOPOLOGY:linear
CLONE:HUMGS05145
SEQUENCE DESCRIPTION:
GATCAGTTTT TATAGCATCT ATGGACATAG AAAATCAGTC ACTGAAAAAA ATAAACACAG 60
CTTTCAATGT CTCACTCAAA 80

SEQ ID NO:4329
SEQUENCE LENGTH:263
SEQUENCE TYPE:nucleic acid
TOPOLOGY:linear
CLONE:HUMGS05147
SEQUENCE DESCRIPTION:
GATCCAGAAT CACCAAGACC TCCAGCCCTG GATGATGCCT TTTCCATCCT GGACTTATTC 60
CTGGGACGTT GGTTCCGGTC CCGGTAGCCT TGTTAACCCT CAGAGGCCTT CAAGTCCTTT 120
CCACCTNTCA CCCATTGCCC ACCATTAANA AGCTTAGCTG NNTCTTGCCA CCTCAGGGGC 180
TTGGATATGT GGAATAGTGA ACTGGGNCCA TGTCAGTTTG TCACTNACCC AAACTGACCA 240
ATAAAACCTN NATTTATGNT AAA 263

SEQ ID NO:4330
SEQUENCE LENGTH:111
SEQUENCE TYPE:nucleic acid
TOPOLOGY:linear
CLONE:HUMGS05148
SEQUENCE DESCRIPTION:
GATCCTAANN CTGACAAGAT TCAAAGNACT AAATTTAATT CAGTCATNAA CACTACCAAT 60
TACCGTTTAT NGGTAGACAT CTTTGGAAAT TTCCACAAGA GCAAGCTAGG N 111

SEQ ID NO:4331
SEQUENCE LENGTH:264
SEQUENCE TYPE:nucleic acid
TOPOLOGY:linear
CLONE:HUMGS05149

SEQUENCE DESCRIPTION:
GATCTGACCT GTTTATTGTT GGCGCTTGTT GAAAACGAGC TTTCTTTCCC ATGATAGTGC 60
TTCGTTTTTG AAGTGTTGAA GCTGTGCTCC CCTNAAATCG TGGCAGGAGA GATTAAGGTA 120
ATTACAACAC TCAGTTCTAT GTCTTACAAG CACTTTGTCT TGTCTCTGCA AGAAAATTCG 180
ATNCCNGTCA TNTCCCATAA AATACAGNCA TTTTACCANC ATANTATGCT TTGATTGANG 240
CAGCANTATG CTTTTGGGCA GTAN 264

SEQ ID NO:4332
SEQUENCE LENGTH:259
SEQUENCE TYPE:nucleic acid
TOPOLOGY:linear
CLONE:HUMGS05150
SEQUENCE DESCRIPTION:
GATCCTCCCG CCTCAGCCTC CCAAGTAGCT GGNATTATAG GTGTGTGCCA CTGTGCCAAG 60
CTAATTTTTT AATTTTTNGT AGAGATGNGA TTTNACCATG TNACTCAGGC TGGTCTCAAA 120
CTCCCAGCCT CAAGCNATGC TCCCACCTCC ACCTCCCAAA GTGCTGGGAT TACAGGCGTG 180
AGCACCATAC CTGGCGCATG CATATTTTTN AAGAGTACCC ACTGGNGTCT AATATATAGC 240
CTGGGNTACC TGGGATGAN 259

SEQ ID NO:4333
SEQUENCE LENGTH:250
SEQUENCE TYPE:nucleic acid
TOPOLOGY:linear
CLONE:HUMGS05151
SEQUENCE DESCRIPTION:
GATCTAACCA ATTTGAATAT GACTTCCTAC TCCAACGTAT CACCAAAACA CTGAAAAGCA 60
ATAGCTGGTT TGGTTGTTAG AAATGGTGTA ATTAATGGTT TTNGTTAGTT CACTTCAGCT 120
TAATAAGTAT TTATNGCATA TTTGCTATGT CCTCAGTGTA CCACTACTTA GAGATATGTA 180
TCATAAAAAT AAAATCTGTA AACCATAGGT AATGATTATA TAAAATACAT AATATTTTTC 240
AATTTTGAAA 250

SEQ ID NO:4334
SEQUENCE LENGTH:260
SEQUENCE TYPE:nucleic acid
TOPOLOGY:linear
CLONE:HUMGS05152
SEQUENCE DESCRIPTION:
GATCGCATTT ATATAACATT CTTGAGGTGA CAAAATTATT GAAATGAAGA ACAGATTAAT 60
GGTTGCCAGG GATAGGGACT GGAGGGGGTG TGGGGTATGT ATGACTATAA AGGGATGACG 120
CAAGGAGTTC CTTTGTGCTG ATGGAACAGT TCTATATGAT TATGATGATT ACGATGTTGA 180
TTATAACATT TGATTATGAG GTTAAGCACA TGAGTCTTTA CCTGTNCTGA AGTTGTATGG 240
ACCTACATAC ACACAAATGN 260

SEQ ID NO:4335
SEQUENCE LENGTH:261

SEQUENCE TYPE:nucleic acid
TOPOLOGY:linear
CLONE:HUMGS05153
SEQUENCE DESCRIPTION:
GATCAAGACG AGCCTGCACC GCCTGAAGCC CGACACGGTG CCAGCGCCCT GCTGCGTGCC 60
CGCCAGCTAC AATCCCATGG TGCTCATTCA AAAGACCGAC ACCGGGGTGT CGCTCCAGAC 120
CTATGATGAC TTGTTAGCCA AAGACTGCCA CTGCATATGA GCAGTCCTGG TCCTTCCACT 180
GTGCACCTGC GCGGGGGAGG CGACCTCAGT TGTCCTGCCC TGTGGAATGG GCTCAAGGTT 240
CCTGAGACAC CCGATTNCTG N 261

SEQ ID NO:4336
SEQUENCE LENGTH:145
SEQUENCE TYPE:nucleic acid
TOPOLOGY:linear
CLONE:HUMGS05155
SEQUENCE DESCRIPTION:
GATCCTCAGT AAACTCTGAG AAATAATGAA TGTTGGTTGT TTTAAAATGG TAAATNTTGA 60
GGTATTATGT TATGTGGCAA TAGATAGCTA ATATATAACT NATTTGANTC AAACAATATG 120
TTAANTTAAA GCNCAGAAGA NTAAA 145

SEQ ID NO:4337
SEQUENCE LENGTH:264
SEQUENCE TYPE:nucleic acid
TOPOLOGY:linear
CLONE:HUMGS05157
SEQUENCE DESCRIPTION:
GATCTAAGGA AAGAAATGGA GCAACTAGTG CTTGACAAAA AGCAAGAGGA GACAGCCGTA 60
CTGGAAGAGG ATTCTGCAGA TTGGGAAAAA GAACTGCAGC AGGAACTTCA AGAATATGAA 120
GTGGTGACAG AATCTGAAAA ACGAGATGNN AACTGGGATA AGGAAATAGA GAAAATGCTT 180
CAAGAGGAAA ATTAGCTGTT CCTGAAATAG AAGAATAATC CTTAACAGTC TGCAAACTGA 240
CATTAAATTC TAGATGTTGA CAAA 264

SEQ ID NO:4338
SEQUENCE LENGTH:109
SEQUENCE TYPE:nucleic acid
TOPOLOGY:linear
CLONE:HUMGS05158
SEQUENCE DESCRIPTION:
GATCGGGGCG GGGAAACAGC NGGAAGANGG NCGCACCCGG TCTGACTACA ACANCCAGAA 60
AGAGTCCACT CGGCACGTGG TCCNGCGCGT GAGGGGGGGT GTCTAAGTN 109

SEQ ID NO:4339
SEQUENCE LENGTH:195
SEQUENCE TYPE:nucleic acid
TOPOLOGY:linear

CLONE:HUMGS05159
SEQUENCE DESCRIPTION:
GATCAGTATA CAGTGTACGT AAACTACACC ATCCTCAAAC CCCGGAAAGC AAAGCAAATC 60
AGGAAGAAAA GTGGAGGTTA GCAACACACC TNTGGCCCCA AAGNACAACC ATCTTGTTAA 120
CTATTGATTC CAGTGACCTG ACTCCCTGCA AGTCATCGNC TGTAACATTT GTAATAAAGG 180
TCTTCTGNCA TGAAA 195

SEQ ID NO:4340
SEQUENCE LENGTH:54
SEQUENCE TYPE:nucleic acid
TOPOLOGY:linear
CLONE:HUMGS05160
SEQUENCE DESCRIPTION:
GATCTAATAT TCAGGAATTT TTCTCCTAAA GACACCCAGT AAAAATATCA CAAA 54

SEQ ID NO:4341
SEQUENCE LENGTH:89
SEQUENCE TYPE:nucleic acid
TOPOLOGY:linear
CLONE:HUMGS05161
SEQUENCE DESCRIPTION:
GATCACTGTG TAAAACTAAG TNTCTCTAAA TGTAATGCAT CGATTTAGTG TCTGGAACAT 60
AATAAATATT TGCTCTCATG ATTGCTAAA 89

SEQ ID NO:4342
SEQUENCE LENGTH:89
SEQUENCE TYPE:nucleic acid
TOPOLOGY:linear
CLONE:HUMGS05162
SEQUENCE DESCRIPTION:
GATCTACATC CCCATCCACC TATACGGACA TCTTTTCCGT TGTGGTTTGA GAATGTTCCT 60
ATAATAAACC CCTCTGCTTT GTTCTTAAA 89

SEQ ID NO:4343
SEQUENCE LENGTH:102
SEQUENCE TYPE:nucleic acid
TOPOLOGY:linear
CLONE:HUMGS05163
SEQUENCE DESCRIPTION:
GATCGTCTTG TATGGTGAAT TTTCTAGGAG CGATGATGTA CTGTAATTTT ATTTTAATGT 60
ATTNNGATTT ATGATTATNT ATTAGTTGNN TTTAAATGCT TN 102

SEQ ID NO:4344
SEQUENCE LENGTH:248
SEQUENCE TYPE:nucleic acid

TOPOLOGY:linear
CLONE:HUMGS05164
SEQUENCE DESCRIPTION:
GATCAATTNT GCANCANACA NCAATCTTAA CCATTTCCAG AAACTACTAG TTATATTAGG 60
CACGTTTTAA AAACTTAAAC AAAATAAAGG CCATCCCAAA CCCTGGGCTG TTGTCGGGTT 120
ATTTTGCAGC AGGGTTGTGT TCAAAANCTT CCTTTGACTC CCTGAGACTT TATCTNCTNC 180
TTGCAGAGTA AGATGGAAAG CCANTCAGTG TTTGCNGTAA TGTAATACAG NGTAGTGCCA 240
TTCNGGNN 248

SEQ ID NO:4345
SEQUENCE LENGTH:239
SEQUENCE TYPE:nucleic acid
TOPOLOGY:linear
CLONE:HUMGS05165
SEQUENCE DESCRIPTION:
GATCCAAACT TCTACAGCAA AAACTTACTT CTTTTAGGAA AGACATACTT GAAACTACAC 60
AACAAAAAGC TTGCTGCTTT CTGGCTAATG AAAGCCAAGG ACTATCCAGC ACACACAGAG 120
GAGGATAAAC AGATACAGAC AGAAGCTGCT CAGTTGCTTA CAAGTTTCAG TGAGAAGANT 180
TGAGAACTTT TCAGAGAAGA TTTATGAAAT AGCTAATAAA CATTGCCTTT TCTTTTAAA 239

SEQ ID NO:4346
SEQUENCE LENGTH:223
SEQUENCE TYPE:nucleic acid
TOPOLOGY:linear
CLONE:HUMGS05166
SEQUENCE DESCRIPTION:
GATCAAAAAA TGTCTCCGCT CAGGGATTTA TGGTGGATTA TTGCAGACAN TGCTAAAAAT 60
ATAGAGCACA AGACAAGTTT ACTAAATTAA AATTTTATTT TTTGAGAAAC TGTTATTTGT 120
ATAAATTATC AAGATTTGTA GGCTTTCCTT TNGTAGAAAT AATTGTTTTA TGTGCCAGAG 180
AATTTCAATT TNGTTTTCAA CAATAAAGCA TTGATAAGAG AAA 223

SEQ ID NO:4347
SEQUENCE LENGTH:52
SEQUENCE TYPE:nucleic acid
TOPOLOGY:linear
CLONE:HUMGS05168
SEQUENCE DESCRIPTION:
GATCATTTGT TTATTAAAAT GTGTCCTATT ACACAGTGAG TTAACTCTCA AA 52

SEQ ID NO:4348
SEQUENCE LENGTH:334
SEQUENCE TYPE:nucleic acid
TOPOLOGY:linear
CLONE:HUMGS05169
SEQUENCE DESCRIPTION:

GATCCGAGCG ACCATGGTGG CCCGGGTGTG GTCGCTGATG AGGTTCCTCA TNAAGGGAAG 60
TGTGGCTGGG GGCGCCGTCT ACCTGGTGTA CGACCAGGAG CTGCTGGGGC CCAGCGACAA 120
GAGCCAGGCA GCCCTACAGA AGGCTGGGGA GGTGGTCCCC CCNGCCATGT ACCAGTTCAG 180
CCAGTACGTG TGTCAGCAGA CAGGCCTGCA GATACCCCAG CTCCCAGCCC CTNCAAAGAT 240
TTTACTTTCC NATCCGTGAC TCCTGGAATG CAGGNATCAT GGACGGTTGA TGTCAGNTCT 300
NNTNGGTGGC CCCTTTCAAA GGGNCNGGTG AGTN 334

SEQ ID NO:4349
SEQUENCE LENGTH:146
SEQUENCE TYPE:nucleic acid
TOPOLOGY:linear
CLONE:HUMGS05170
SEQUENCE DESCRIPTION:
GATCCATCAA ATTGAACAAT CTGTTGTAAT TTAAAATTTT GGCCACTTTT TTCAGATTTT 60
ACATCATTCT TGCTGAACTT CAACTTGAAA TTGTTTTTTT TTNCTTTTTG GATGTGAAGG 120
TGANCATTCC TGATNNNGGC CTCNNN 146

SEQ ID NO:4350
SEQUENCE LENGTH:257
SEQUENCE TYPE:nucleic acid
TOPOLOGY:linear
CLONE:HUMGS05172
SEQUENCE DESCRIPTION:
GATCTCAAGC CTCTTCTGAA CCCCAGGCTT TCTTGAGTCT CCATATGAGC ATTTGCCTCT 60
GTGGGCACAA CTTCAAGTGT CCTTTGTGAG GAAATGTGCA TCTCTGGNTG GTGAACTTCC 120
ATCCCTCAGA GAGAAATAGA AGCACAAATT TGAAACAAGC TGTCTGTCTG NGNNNNNNCT 180
TTATGTCTTG GAAACTTTAT TTATCCATCC AAGCTTTNTT TTTTTTTAG CCTTTNNGGG 240
CAGGNAACCA TCCAGGN 257

SEQ ID NO:4351
SEQUENCE LENGTH:73
SEQUENCE TYPE:nucleic acid
TOPOLOGY:linear
CLONE:HUMGS05173
SEQUENCE DESCRIPTION:
GATCTGTCTA CTGAGAAGAC CCAAATCTTG GTTTCTAAAT GCCATTCTTC AATAAAAGGA 60
ATTGGGTTTC AAA 73

SEQ ID NO:4352
SEQUENCE LENGTH:73
SEQUENCE TYPE:nucleic acid
TOPOLOGY:linear
CLONE:HUMGS05174
SEQUENCE DESCRIPTION:
GATCTCCTTC TCCNTCCTGT CCTGTCCTTG CCCCTCAGGA CTGCTGGAAA ATAAATCCTT 60

TAAAATAGTG AAA 73

SEQ ID NO:4353
SEQUENCE LENGTH:306
SEQUENCE TYPE:nucleic acid
TOPOLOGY:linear
CLONE:HUMGS05176
SEQUENCE DESCRIPTION:
GATCCAAGCA GGGGGATGAA AAACTCAGCA GAGAAATTCG AGACCATTTT CCAAGACTNT 60
CCCCTTCTCC TCAGGACCCC CTGGCTCAGT NCTTGAAAAA CGGTGTCATA TTTAGTCAGA 120
GGCCCCACCC CCAGGAAGCA TGGATGGGGA TGAAGGCACA GGCGTCTCCA ACCTCAGAGG 180
CCCTTTGTGG GGTCAGGACA CAGAGTGGGN AGANGAGACT GATGCAGGCC TACCAGTNCC 240
TGGCTTTTTG TCTGGGGCTG GAATAAAGAG GTGNCTTCAG CTGTTTGGNN CGAGAGGCAG 300
GGTAAA 306

SEQ ID NO:4354
SEQUENCE LENGTH:76
SEQUENCE TYPE:nucleic acid
TOPOLOGY:linear
CLONE:HUMGS05177
SEQUENCE DESCRIPTION:
GATCAACTGG AACCTCTGTA TCATGCGGCT GAATTCCCTT TTTCCTTTAC TCAATAAAAG 60
CTACATCAGA CTGAAA 76

SEQ ID NO:4355
SEQUENCE LENGTH:52
SEQUENCE TYPE:nucleic acid
TOPOLOGY:linear
CLONE:HUMGS05178
SEQUENCE DESCRIPTION:
GATCAGGCTT TTAGGACCTA GGAAACAAAT AAAGAGGAAT TTGTTGCTGA AA 52

SEQ ID NO:4356
SEQUENCE LENGTH:268
SEQUENCE TYPE:nucleic acid
TOPOLOGY:linear
CLONE:HUMGS05180
SEQUENCE DESCRIPTION:
GATCCTTATT CTAGCTACAA GTCAAAGATA ACTCCTGGTC CAGACAAAAC ACCTGGCCTA 60
TCACAAGCTG ACTAAAAATC TGCACTTTGG GCCAGCGCAG GCAACAGTAA CTNTGACAGG 120
TTCAAATTAG ACCTCACACT TTCTACTCAT ATTCTAGTCA CTGGACCCAT CTGANTCAGT 180
AATCCCTNCT GCCCGGTCCT GGAGTAACTT CTTAGAGATA TTATAGCAAG TGGCAAAANT 240
AAAAGAGGGA TTTGCTANGA NTATCAAA 268

SEQ ID NO:4357

SEQUENCE LENGTH:114
SEQUENCE TYPE:nucleic acid
TOPOLOGY:linear
CLONE:HUMGS05181
SEQUENCE DESCRIPTION:
GATCTCAAAT ATAAATTTGA GATTATACAT GTAGTTATGT ATAGNGNCAT GTAGTTACAC 60
ATAGTTTTGT ATTTNCCTGT TTTTTTCTCA TATGAGCATT TNCTTATTAA NGTN 114

SEQ ID NO:4358
SEQUENCE LENGTH:179
SEQUENCE TYPE:nucleic acid
TOPOLOGY:linear
CLONE:HUMGS05182
SEQUENCE DESCRIPTION:
GATCTACACG TGTTGTTTTG TTTTTNCTTT ATTGATGCAC GNATGCTTTT GAACAGTAGA 60
GNGAAATGCT AGACATGGAG AATCTGCTCT GTTTGTCCTN TATACATTTC TGTAGTTAAC 120
AGAACACTGT AATGTGCCTT GGAGCTTAGT AACTNGTAAT AAATTCAATN GATATTAAA 179

SEQ ID NO:4359
SEQUENCE LENGTH:85
SEQUENCE TYPE:nucleic acid
TOPOLOGY:linear
CLONE:HUMGS05183
SEQUENCE DESCRIPTION:
GATCCTAAAT CCTATGTAGG CTNNGGTTAT TAGTGTATAT TTGTGTCTTA AAACAATTTT 60
GAAAGTNAAA AAAATATTTT TTAAA 85

SEQ ID NO:4360
SEQUENCE LENGTH:194
SEQUENCE TYPE:nucleic acid
TOPOLOGY:linear
CLONE:HUMGS05184
SEQUENCE DESCRIPTION:
GATCTNATTT TGAGATTAAA AAAAATACTT CTAGCTTTAG CCAGGTGTGC ATGTNGCTGC 60
CATTTTGCAT TTTTGAATCA TCTTGTGTAA TTGTCACCAT GAAAGTGTTA CTCAGAATNG 120
TCATAGATTT NNTNTTCTTT GTGCAAAAAC ATGGAAAATT GTCACTGACT TTGTTTTGAG 180
GTTTCCCCGG NNNN 194

SEQ ID NO:4361
SEQUENCE LENGTH:216
SEQUENCE TYPE:nucleic acid
TOPOLOGY:linear
CLONE:HUMGS05185
SEQUENCE DESCRIPTION:
GATCTGCTGT ATGTTACAGT TATGTAATTT GACTTGTTTC AAAATTATTA AAGTTTTAAG 60

TGTATGCATT CTACTGGTCA ATAAGTAAAT ATATTCAGGC TCTGAATGTA GACAGATAAA 120
TATATAAATA TAGAAAGTAC ATTTGTTAGA ATACAAACAA GAATAAAAAT TGAGTACAGA 180
NTGAAAACCT TATTAAATTT TATCACTCCA TATAAA 216

SEQ ID NO:4362
SEQUENCE LENGTH:333
SEQUENCE TYPE:nucleic acid
TOPOLOGY:linear
CLONE:HUMGS05186
SEQUENCE DESCRIPTION:
GATCAAACTC AAGCTCACAC CTGTACCTGA TGGGAATGAA CATAATGTGA AGAAACTTAT 60
TTCCCAGTGT AAACGCAGTT GTACAGAAAA AAAATGTTTT GGGTATAAAT CTGGGTTTTC 120
AAAACCAGCT TTNTTCTATA TATAAATNAT GCTGCTATTA CAGATTTAAC ATTTNCTGTT 180
AAAGGAAAGT CTACATTTTC TGCCTGTNCA GAACTGTTTA ATAGCAGTNN CTCTTGAGTG 240
TATTTNCCTT GGTATGTTTT TNAACCTATT TTCCTTNAAG CCGAAAATAT TGCCAATTGG 300
ATANGGTTAG CCTTANNAAG TNNAATAAAN NAN 333

SEQ ID NO:4363
SEQUENCE LENGTH:185
SEQUENCE TYPE:nucleic acid
TOPOLOGY:linear
CLONE:HUMGS05187
SEQUENCE DESCRIPTION:
GATCAGCCAG TCTCTTTCAT CATCAAGCAG ACATACTCAA GGTACCAACA CAGCAAAGGC 60
ACAAAATGTC TCTATATCAG TATTTTTCAT GGTATATGTG AGACCCATTA GTGAGGCATG 120
AAATCAATTC TGTGAGTCAC AATGAACATT TGTCTAAAAA ACATAATAAA NTGTAAAATA 180
CCAAA 185

SEQ ID NO:4364
SEQUENCE LENGTH:108
SEQUENCE TYPE:nucleic acid
TOPOLOGY:linear
CLONE:HUMGS05188
SEQUENCE DESCRIPTION:
GATCCTTTCC CATTTATTCT GGGAAGATGT TTTTCAAACT CAGAGACAAG GACTTTGGTT 60
TTTNTAAGAC AAACGATGAT ATGAAGGCCT TTTGTAAGAA AAAATAAA 108

SEQ ID NO:4365
SEQUENCE LENGTH:201
SEQUENCE TYPE:nucleic acid
TOPOLOGY:linear
CLONE:HUMGS05190
SEQUENCE DESCRIPTION:
GATCATTTGG CTCTTTTTTT CCTTCCAGAA GAGTNGCATC AACAAAGTTA ATTGTATTTA 60
TGTATGTAAA TAGATTTNAA NCTTCATTAT AAAATATTGT GAATGCCTAT AACTTGTTTN 120

NCAATGTGNT TGTGTGTGTT TCTAAGGACT TTTTCTTNGG NTTGCTAAAT ACTGTAGGGT 180
TGAAAATGCT TCTTTCTACT N 201

SEQ ID NO:4366
SEQUENCE LENGTH:118
SEQUENCE TYPE:nucleic acid
TOPOLOGY:linear
CLONE:HUMGS05191
SEQUENCE DESCRIPTION:
GATCCTTCAT GGACANTATT CTAACTGATA CGTAGACACT TACTTGGAAA TTTTTGGACA 60
TTATATTAAA TGAGTGCTAT CTGTGAAATT GGTTATATTA GGTGGCTNNN NCTAATGN 118

SEQ ID NO:4367
SEQUENCE LENGTH:277
SEQUENCE TYPE:nucleic acid
TOPOLOGY:linear
CLONE:HUMGS05192
SEQUENCE DESCRIPTION:
GATCTGAGGT CTCAAGCTAG GAGAGACTGA GAATTTTAAT CAGTTTGGGC ATATAGTTTG 60
GACTGAATCA CATCTGTAGT ACTTAGCCAA AGACAATTTG GAGGAGAATA TCAGCCTTCT 120
GGAAGTAGCT ACTTCCTGNT CAATGTAAAG TGTCGCAGAT ATTCAATAAA ATGGCAACCT 180
GTTATAATTT TNTTGAATAT CAGCCTTCTG GNAGTAGCNA CTTCCTGTAN CAAANGTAAA 240
GNGTCGCAGN TATTCAATAA AATGGCAACC TGTTTTN 277

SEQ ID NO:4368
SEQUENCE LENGTH:209
SEQUENCE TYPE:nucleic acid
TOPOLOGY:linear
CLONE:HUMGS05194
SEQUENCE DESCRIPTION:
GATCTCTTGA CAANTGTGTA TGTTAACTTT TTAGCACATG TTTTGTACTT AGTACACGAG 60
AAAACCCAGC TTTCATCTTT TGTCTGTATG AGGTCAATAT TGATGTCACT GAATTAATTA 120
CAGTGTCCTA TAGAAAATGC CATTAATAAA TTATATGAAC TACTATACAT TATGTATATT 180
AATTAAAACA TCTTAATCCA GAAATCAAA 209

SEQ ID NO:4369
SEQUENCE LENGTH:58
SEQUENCE TYPE:nucleic acid
TOPOLOGY:linear
CLONE:HUMGS05195
SEQUENCE DESCRIPTION:
GATCGTGTCG GAGACTNTNA CACAGTTTAA ACACATCAAT AAATACTTTA ACTTCAAA 58

SEQ ID NO:4370
SEQUENCE LENGTH:108

SEQUENCE TYPE:nucleic acid
TOPOLOGY:linear
CLONE:HUMGS05196
SEQUENCE DESCRIPTION:
GATCGAGGTA GTAAAGGCCA TCCACATTTT AAAGGGTTAT TTGTCTNTNN TATAATNCGT 60
TTGCTTTCAG AAAATGTTTT AGGGTAAATN CATAAGACTA TGCAATAN 108

SEQ ID NO:4371
SEQUENCE LENGTH:190
SEQUENCE TYPE:nucleic acid
TOPOLOGY:linear
CLONE:HUMGS05197
SEQUENCE DESCRIPTION:
GATCAAACAG GGACACAAAA GCAAGTATCA TGGGAGGAAC TTGAAATCTC TGNGGACAGC 60
AGAAGCTGAC TTCAACTCTN ATAATTGTGG CATCCATAAA TTGCAAATAT AGCCCTGAAT 120
AGATACACAC AAATGTCTAT AATGAAGGCC CAGCAGAATG GAATGTGTGA CCATCTTCAG 180
GAAGAAAATN 190

SEQ ID NO:4372
SEQUENCE LENGTH:241
SEQUENCE TYPE:nucleic acid
TOPOLOGY:linear
CLONE:HUMGS05198
SEQUENCE DESCRIPTION:
GATCAATCCC TAGTAAAAGC CCTGGACACC TAGGCATGGG TGAGCTACTN TGGTTGGTAA 60
TACTCTGTGC ACACATCATT GTAGCCACAC ATCATTGCTG GGAGAATTAA GCATTATCCT 120
GAAGACTCTG CCAGGAGAGG ATAATTGGAA GTTCTCTTGG ACCTTACCTT ATGTGCCTTT 180
NTTCATTGCT GATTTTAATC TGTATCCTTT CACTGTAATA AACTGTAACT ATGNGTGCAA 240
A 241

SEQ ID NO:4373
SEQUENCE LENGTH:51
SEQUENCE TYPE:nucleic acid
TOPOLOGY:linear
CLONE:HUMGS05199
SEQUENCE DESCRIPTION:
GATCGTTCAT TGCCTTTCCA GGGTTATTTA GTAAAGTTTG TTGAAAACAA A 51

SEQ ID NO:4374
SEQUENCE LENGTH:153
SEQUENCE TYPE:nucleic acid
TOPOLOGY:linear
CLONE:HUMGS05200
SEQUENCE DESCRIPTION:
GATCTCAGGT GATTCACCCG CTTCTGCCTC CCAAAGTGCT AGGATTACAG ACATGAGCCA 60

CCGCACCTGG CCATACAATT GCCTTCTATA TGTTTTTNAT TTAATATGAC ATTTGTATTT 120
CCCCATGTTA TTAAAAAATT CTNTATAAGC AAA 153

SEQ ID NO:4375
SEQUENCE LENGTH:103
SEQUENCE TYPE:nucleic acid
TOPOLOGY:linear
CLONE:HUMGS05201
SEQUENCE DESCRIPTION:
GATCACCCCA CCCTGTGAAG AGGCAGTTGG TGCTGGCAGT AAGCTGGTTT CCTCCTCTCC 60
AGGGTTTTCC TAGTAATAAA GGTGTTNCTG TTGANGCCGT AAA 103

SEQ ID NO:4376
SEQUENCE LENGTH:390
SEQUENCE TYPE:nucleic acid
TOPOLOGY:linear
CLONE:HUMGS05203
SEQUENCE DESCRIPTION:
GATCACATTC ATTCCATAGG TAGCTTTACG TGTGGCTACA ACAAATTTTA CTAGCTTTTT 60
CATTGTCTTT CCATGAAACG ANNTGANGAA AATGATTTTC CCTTTGCAGG TTGCACACAG 120
TTTTGTTTAT GCATTTCCTT AAANTTAATT GTAGTCTCNA GGATACAAAC CATAGTAGGC 180
AATACAATTT TAGAATGTAA TATATAGAGG TATATTTAGC CTCTTTTAGA AGTCAGTGGA 240
TTGAATGTCT TNTTATTTTA NNTTTTACAT TCATTAAGGT GCCTCGTTTT TGACTTTGTC 300
CATTAACATT TATCCATATG CCTTTGCAAT ANCTAGNTTG GTGNAAAGCT AACCAAGTGT 360
TGTAACCANT AATCCNTTGT TTNGNGGTGN 390

SEQ ID NO:4377
SEQUENCE LENGTH:144
SEQUENCE TYPE:nucleic acid
TOPOLOGY:linear
CLONE:HUMGS05204
SEQUENCE DESCRIPTION:
GATCACATGC AGGACTCTTT CTTAATGCAG CCTGAGACAA ATCTTGCAAT ATGAAGAGAT 60
GATTTCCAGG TTTTATTGAA TAAATGAATT GTAATTTTTA CTTGAAATTA TCTTATTTAG 120
TATAATAAAT ATTACAATAT TAAA 144

SEQ ID NO:4378
SEQUENCE LENGTH:386
SEQUENCE TYPE:nucleic acid
TOPOLOGY:linear
CLONE:HUMGS05205
SEQUENCE DESCRIPTION:
GATCCCACAG CGAAACACTG GCCACTGGCT ACCAGTATTN CTTCCCAGAG CTAGGGGCTG 60
CCTTAAAGGA AATTGTAGCC TAAGTAGGTC GTGGCAAGGG CCTGAGGCCT GTTCCTCACA 120
GGCTTCCAGG TTAGGCACTG TGAATAGGCT CAGCTCCTCT AGAGAGCTGA AGCCATCTGG 180

TTCTTAGATT CCTCTCCCAG TCCTCTTTCC CATTGTTCTG TTGCTCCACC TTATTGTCTC 240
AAGGCCGTAA TCTCATCAGG TTGGGACATT AATCTTTTCA ACTCCTTTGT AAAGATTTCC 300
CAGTTTGGTT TCTCTACATG TNCTGCAGCT GGCCNCAATT CTCCTTTNAC GTTGTGTAGA 360
GAATGNTCTG CAGTTTTAGG GCAAGN 386

SEQ ID NO:4379
SEQUENCE LENGTH:174
SEQUENCE TYPE:nucleic acid
TOPOLOGY:linear
CLONE:HUMGS05206
SEQUENCE DESCRIPTION:
GATCTAAACG AAAAATAGTT TCTTGTTTAA ATTCACATAA GGCAATNAGA TATGGAAAGA 60
TGACAAGATA CGTATAAACA TTGGTTTGCA TTTTATTAAA TNATTCTAAT GCAAATCTTG 120
TATAAAGAAC CCATGATGTT TTGTAACTTT CTAATTAAAA TGTTCAAAAT GAAA 174

SEQ ID NO:4380
SEQUENCE LENGTH:207
SEQUENCE TYPE:nucleic acid
TOPOLOGY:linear
CLONE:HUMGS05207
SEQUENCE DESCRIPTION:
GATCTTGGTC AATAACTGGG AGATGGACAA ACTAGAGATG GAAGATGCAG TCACATTTTT 60
GAAGACTAAA ATCTATTCAG TAGAAGCTCT TCCTGTTGCT GCTGTAAATG TGCAAAGCAC 120
ACAATAAAGT GAAAATCAAC CTTTTCATAT TAGGAGACAT GCATTTGTAA AAATTAATAA 180
AGATGACAAG TCAGTTGTCA ATGGAAA 207

SEQ ID NO:4381
SEQUENCE LENGTH:189
SEQUENCE TYPE:nucleic acid
TOPOLOGY:linear
CLONE:HUMGS05208
SEQUENCE DESCRIPTION:
GATCAGCACC TTGGCCGTGA TTCCCAAGGT CCCAGCCAAA GCAAAGGGCC AGTTGTTTCA 60
GTTTAAACAG ACATGTCTTT AGTCTAATAA AATTAGTTAA CTGCCAGTAA AGTTATTTGT 120
TAGCTTTGNT GAAAGCTATG NNGGTATCTT TCCCTAATCA TCAAAGTAAA TAAAAANTCA 180
TTTCTNAAA 189

SEQ ID NO:4382
SEQUENCE LENGTH:502
SEQUENCE TYPE:nucleic acid
TOPOLOGY:linear
CLONE:HUMGS05209
SEQUENCE DESCRIPTION:
GATCTGTTCT TTTTAAGTTG ATTCGGGAGT GGCATTCTTT TATACCCAAA GACTGTAGTG 60
CATCTTGAAG AGCTCAAAGC ACATGACCGC ACAAATGCTT ACAGGGTTTC CTCCCGAGTA 120

ATCCAATCTC ACTCCCCTTG TAAGGGAATT CTGGGGCAGC TATGGTTTGA GTATGCAGTT 180
TGCATCGTGT TTCTACCTTT AGTACCTTGC CACTCTTTTA AAACGCTGCT GTCATTTCCC 240
ATTTCTTAGT ACTAATGATT CTTTGATTCT CCCTCTATTA TGTCTTAATT CACTTTCCTT 300
NCCTAAATTT GGTGATTTGG CATATCAAAT TCTGGTAAAA TNGTTTTNGG TAAANCATAT 360
TTACCCNCAC TTGGGGTAAA TCCCAATTCC TGGNTAGGGN CTTTTAAAAA NGGGTTTTTN 420
NTTAATNGGG NNAATCCAAT TATTAAAAAN GGGGGGNCCN TTTTTTNAAG GGNGGAANAA 480
ANGGNGAGGG GGNAGNTNGN GN 502

SEQ ID NO:4383
SEQUENCE LENGTH:383
SEQUENCE TYPE:nucleic acid
TOPOLOGY:linear
CLONE:HUMGS05210
SEQUENCE DESCRIPTION:
GATCTCTGGT CAGAGTGAAC TCTTGCTTCC TGTATTCAGG CAGCTCANAG CAGAAAGTAA 60
GGGGCAGAGT CATACGTGTG GCCAGGAAGT AGCCAGGGTG AAGAGAGACT CGGTGCGGGC 120
AGGGAGAATG CCTGGGGGTC CCTCACCTGG CTAGGGAGAT ACCGAAGCCT ACTGTGGTAC 180
TNAAGACTTC TGGGTTCTTN CCTTCTGCTA ACCCAGGGAG GGTCCTAAGA GGAAGGTGAC 240
TTCTCTCTGT TTGTCTTAAG TTGCACTGGG GGATTTCTGA CTTGAGGCCC ATCTNTCCAG 300
CCAGCCACTG CCTTCTTTGT AATATTAAGT GCCTTGAGCT GGAATGGGGA AGGGGGNCAA 360
GGGTCAGTCT NTCGGGGTNG GNN 383

SEQ ID NO:4384
SEQUENCE LENGTH:258
SEQUENCE TYPE:nucleic acid
TOPOLOGY:linear
CLONE:HUMGS05211
SEQUENCE DESCRIPTION:
GATCAAACTG TTGGAGGAGA AGCTGAAGGA GGCTGAGACC CGAGCAGAGT TTGCCGAGAG 60
GTCTGTGGCA AAGTTGGAGA AANCCATCGA TGACCTAGAA GAGACCTTGG CCAGTGCCAA 120
GGAGGAGAAC GTCGAGATTC ACCAGACCTT GGACCAGACC CTGCTGGAAC TCAACAACCT 180
GTGAGGGCCA GCCCCACCCC CAGCCAGGCT ATGGTTGCCA CCCCAACCCA ATAAAACTGN 240
TGTTACTAGN CTNTNAAA 258

SEQ ID NO:4385
SEQUENCE LENGTH:174
SEQUENCE TYPE:nucleic acid
TOPOLOGY:linear
CLONE:HUMGS05212
SEQUENCE DESCRIPTION:
GATCAATGAA AGGAGACATC TGGAGTGTGC GTGCTTCTTC AGAGGGACGG GTGATGGGCA 60
GATTGGAAAA AGCACCGCAG ATGGGAACCT TAATCTTTCT TTTCTAAAAT TGATGCTATG 120
AAAATTTGCG TTTTCTGTAA CTTGTAAAAA CTAAAAGTTG CTTGTCTACT GAAA 174

SEQ ID NO:4386

SEQUENCE LENGTH:62
SEQUENCE TYPE:nucleic acid
TOPOLOGY:linear
CLONE:HUMGS05213
SEQUENCE DESCRIPTION:
GATCAGGCAA AAATAGTTTT CCAAAGTTAT TTTTAATAAA GTATATACAA AATTCTTATA 60
AA 62

SEQ ID NO:4387
SEQUENCE LENGTH:217
SEQUENCE TYPE:nucleic acid
TOPOLOGY:linear
CLONE:HUMGS05214
SEQUENCE DESCRIPTION:
GATCTAACGC CACACCTCCG TGAAAACACA ANTCNCACAG CAGGAACCAC NTGAACCTGG 60
ACATCAGCAG TTTATTCAGC AGATGCTTCA GGCTCTTNCT GGAGTAAATC CTCAGCTACA 120
GANTCCAGAN GTCAGATTTC AGCAACAACT GGAACAACTC AGTGCAATGG GATTTTNGAN 180
CCGNGAAGCA AACTTGCAAG CTCTAATAGC ANCAGGN 217

SEQ ID NO:4388
SEQUENCE LENGTH:95
SEQUENCE TYPE:nucleic acid
TOPOLOGY:linear
CLONE:HUMGS05215
SEQUENCE DESCRIPTION:
GATCTATGCA GACAACTGTG TATTCTGTTT TATAACAGTT TGTTTGAATT TACTTACAGT 60
TAAAAAATTT AAATATATTT ATGTTTGTAC GGAAA 95

SEQ ID NO:4389
SEQUENCE LENGTH:192
SEQUENCE TYPE:nucleic acid
TOPOLOGY:linear
CLONE:HUMGS05216
SEQUENCE DESCRIPTION:
GATCTGGGTG AAGGGGAGAA CCTGCCATCT TATCCCTACC CCCCCGGGGC CCTCAAGCTT 60
ATTTTCTTGT TGAAGAAACA CAAAACCCTC GAGATTCATG TACTGTATGT TGGAGAAAAA 120
AAATTACCTA ACTGTTCCCC CAAAAAAGTC AGTATNTTTT GTACTTTGTA AAAGTGTNGG 180
TTAAAAATGA AA 192

SEQ ID NO:4390
SEQUENCE LENGTH:135
SEQUENCE TYPE:nucleic acid
TOPOLOGY:linear
CLONE:HUMGS05217
SEQUENCE DESCRIPTION:

GATCTGCAGC CTTTTGGGTA TCAAATGGGT CAAAACCATG GGACCTGCCA CCTCCCATCA 60
GCAATTCTGG AAATGCACTA TTTCTACTGG TGNTCTTGCT TTTTTTTTT TTNNATTTTC 120
TNGNTGAANT GCCAN 135

SEQ ID NO:4391
SEQUENCE LENGTH:270
SEQUENCE TYPE:nucleic acid
TOPOLOGY:linear
CLONE:HUMGS05218
SEQUENCE DESCRIPTION:
GATCGTANTT AGTCCTTTTC ATCTCAGCTG CTCCTTAAAT CAGTGCAACC CCTCCAAATT 60
AATGTGAATT GAAACTTGCA TTGCTTTTCA ACTTAATTGA ATACTTACTA TATGTGAACC 120
ACTGCACAAA ATAGGGGAAG AAGATGTGGG AGACAAAGAT GTGGAGCCTG TCTTCAATAA 180
ATTTACAGTT AGTTTCAGGG TACATTGTTT TNCTNCTGCT GTTAATTAGG TGTATCCCCA 240
AGACAATAAA TACATGGNTG GGAAAGTAAA 270

SEQ ID NO:4392
SEQUENCE LENGTH:123
SEQUENCE TYPE:nucleic acid
TOPOLOGY:linear
CLONE:HUMGS05219
SEQUENCE DESCRIPTION:
GATCAAAATT GGNTAAATTC TATTGTAACA ATTTCGTTGG TCATTTTGGG CCATAAAATT 60
TTTTTGTAAT AGGAATTTGT ATAAAGCATT ACTCTTTTTC AATAAATTGT TTTTTAATTT 120
AAA 123

SEQ ID NO:4393
SEQUENCE LENGTH:288
SEQUENCE TYPE:nucleic acid
TOPOLOGY:linear
CLONE:HUMGS05220
SEQUENCE DESCRIPTION:
GATCNCACCA GTNTNCTNCA NCCTGGGCCA CAGAGCAAGA CCTTGACTCA AAAACAACAA 60
CAACAACAAC AAAAATTCTT GAAGATTTTG CATTCTGTCC CACTATCCAT TGGTTTTCAT 120
GTCAAGATAA TGTGAGAAAT TCTTTACAAT TGCTTCCAGA AGGANTAGCC TTTTGATTTA 180
GTGCACAGGT GTCCAGTCTT TTGGCTTCTC AGGCCCACAT TGGAAGAAGA ATGCTCCTGA 240
GCCACACATA AAATACACTA NTGNTAACAA CAGCTGATGG AGCTTAAA 288

SEQ ID NO:4394
SEQUENCE LENGTH:156
SEQUENCE TYPE:nucleic acid
TOPOLOGY:linear
CLONE:HUMGS05221
SEQUENCE DESCRIPTION:
GATCCTNCGT TCTNTTGTTA TAGATTAGTT TGCGTTTCCT ACAATTTTAT ACACATGGAA 60

TAATACTATA GGTACTCTTG TTTGGCTCCT NCCATCCAGC ATAATAAGTT TGAGATTCAT 120
NCATGTTGTT ATATGCGTCT ATGTTCATTC CTNNNN 156

SEQ ID NO:4395
SEQUENCE LENGTH:149
SEQUENCE TYPE:nucleic acid
TOPOLOGY:linear
CLONE:HUMGS05223
SEQUENCE DESCRIPTION:
GATCCTNCTT TTCCTTCATG NCAGCACAAG TGCTCACCGG GGCCAGAGCC AGGGCATGGA 60
TATGACAAGC AGGGCAGCCT GGACACTGCC CTCACAGGAC AGCGCCAATA ACAATACAGT 120
GTCTGAGTAT CTCCAGGGGA AAAAATAAA 149

SEQ ID NO:4396
SEQUENCE LENGTH:204
SEQUENCE TYPE:nucleic acid
TOPOLOGY:linear
CLONE:HUMGS05224
SEQUENCE DESCRIPTION:
GATCCTGCTT GGNCTATCAC AGAGCATTGA CCATTGGCTT CCCTCATCTG AGNCGTGGGA 60
GAGCANACTG GATAGATGAG AATTGTTTTA AAACAATTGT GAACAGAAAC TGNAGATGGT 120
ACAGTNCTAC ATCTGCACCT GCCCTTTTTT CATACCACAA AAGTATTTTT NNAGTACTGT 180
ACTGGACTTT TTGCNAGTNN CTAN 204

SEQ ID NO:4397
SEQUENCE LENGTH:213
SEQUENCE TYPE:nucleic acid
TOPOLOGY:linear
CLONE:HUMGS05227
SEQUENCE DESCRIPTION:
GATCCTGATG TATGAGGAAA AAGCGAGAGA AAAGGAAANG CCAACAGGCC CCCCAGCCAA 60
GAAAGCTATC TNTGAGTTGC CCTGATTTGA AGGGAAAAGG GATGATGGGA TTGAAGGGGC 120
TTCTAATGNC CCAGATATGG AAACAGAAGA CAAANTTNTA AGCCAGAGTC AACAAATTAA 180
NTAAATTACC CCCTCCTCCA ANAANTANTT TNN 213

SEQ ID NO:4398
SEQUENCE LENGTH:67
SEQUENCE TYPE:nucleic acid
TOPOLOGY:linear
CLONE:HUMGS05228
SEQUENCE DESCRIPTION:
GATCCATCTG AGGAATCACT CTGTGTTGCA CTTATGCCCT CATAAAANAT ATTTATTGAA 60
GAATAAA 67

SEQ ID NO:4399

SEQUENCE LENGTH:70
SEQUENCE TYPE:nucleic acid
TOPOLOGY:linear
CLONE:HUMGS05229
SEQUENCE DESCRIPTION:
GATCAATGCA TTTNATACGG ATATAGACCT AGGGCTCTGG AGGGTGGGGN ATTGTTAAAA 60
CACATGCAAA 70

SEQ ID NO:4400
SEQUENCE LENGTH:148
SEQUENCE TYPE:nucleic acid
TOPOLOGY:linear
CLONE:HUMGS05230
SEQUENCE DESCRIPTION:
GATCTCTATA GAGGCTGGAA TGGCCACAGA GAATCTAAAT NTGAAAACAA ACATGTCCTG 60
AGGGTGTGCT CCAATACCAC CCNNTAATAC TCTCCTGAGA TGTGAAGGCT NGAAATGAGA 120
ATGCCATNTT ATTGTNACTT NCAAACGN 148

SEQ ID NO:4401
SEQUENCE LENGTH:210
SEQUENCE TYPE:nucleic acid
TOPOLOGY:linear
CLONE:HUMGS05231
SEQUENCE DESCRIPTION:
GATCCTTGTT TTGAGCTCTC AGAATCACTC AGACAACATT TTGTAACTGC TGCTGTTGCT 60
TTCTACATAC ACCTTATAAA GTGACATTTC AAAAGAAATA AGGTGCCACA GTTTTAAACC 120
AGAAGGTGGC ACTCTGTGGC TCCNTGTAGT ATTATAGCTA TACTGGGAAA GCATAGATAC 180
AGCAATAAAG TACAGTAATT TTACTTTAAA 210

SEQ ID NO:4402
SEQUENCE LENGTH:71
SEQUENCE TYPE:nucleic acid
TOPOLOGY:linear
CLONE:HUMGS05232
SEQUENCE DESCRIPTION:
GATCCTGTTG CAAACTGTGA ATATTCTGAT GATGCCAGTA CAGTTTGATT TATTAAATGT 60
GGGTCCTCAA A 71

SEQ ID NO:4403
SEQUENCE LENGTH:240
SEQUENCE TYPE:nucleic acid
TOPOLOGY:linear
CLONE:HUMGS05233
SEQUENCE DESCRIPTION:
GATCTGAACC CGTCTCCCGG GTGCTGTAAA TAGTCTGATA AACGTTCACA CAGTCTAAAA 60

TTACCCTTTA TATTTGCTGA ATACAACTCA TCTTTTGTAG TTTAAAATTT CTATTGTTTT 120
GGAGCTAGCT GTGAGTTTCT AGAAGTGTAC AGAGTTGCTC CNGTGTTCCC GGGTCANGTT 180
GAGTAGGGAA TAAATAAATC TGATGCNGCC TCCTGAGGCT GCCGGGGGGT TTCTGCTAAA 240

SEQ ID NO:4404
SEQUENCE LENGTH:67
SEQUENCE TYPE:nucleic acid
TOPOLOGY:linear
CLONE:HUMGS05234
SEQUENCE DESCRIPTION:
GATCAAGNAT ATTTTTTATT TTTAAGAAAG CATAACCAGC AATAAAAATA CTATTTTTGA 60
GTCTAAA 67

SEQ ID NO:4405
SEQUENCE LENGTH:173
SEQUENCE TYPE:nucleic acid
TOPOLOGY:linear
CLONE:HUMGS05235
SEQUENCE DESCRIPTION:
GATCTAATTC CAAGGACTTC TCCACAGCTA AGTNAGATGC CTCANACCAT TAGGTGATGC 60
TTTGGACAGA ACAGAGTATT TNAATCTTGT GTTTAAAGCA ATTCCTTGGC TTCGGCTCCT 120
CACCACTTTC TATGCCAGTN TCCCATTTAT GTCCCTAGTA ATGCCTATGC AAA 173

SEQ ID NO:4406
SEQUENCE LENGTH:263
SEQUENCE TYPE:nucleic acid
TOPOLOGY:linear
CLONE:HUMGS05236
SEQUENCE DESCRIPTION:
GATCCCAGCA GTGATGTTGG TTGAGGCAGC AAACAGATGG CAGGATGGGC ACTGCCGAGA 60
ACAGCATTGG TCCCAGAGCC CTGGGCATCA GACCTTAACC ACCAGGCCCA CAGCCCAGCG 120
AGGGAGAGGT CGTGAGGCCA GCTCCCGGGG CCCCTGTAAC CCTACTNTCC TCTNTCCCTG 180
GACCTCAGAG GTGACACCCA TTGGGCCCTT CCGGCATGCC CCCAGTTACT GTAAATNTGG 240
CCCCCAGTGG GCATGGAGCC AAA 263

SEQ ID NO:4407
SEQUENCE LENGTH:65
SEQUENCE TYPE:nucleic acid
TOPOLOGY:linear
CLONE:HUMGS05237
SEQUENCE DESCRIPTION:
GATCAGAATG TTCTAAATNT TGACTAGTTA CTTTTTATTA TGAGTTAATA TAGTTTAGCA 60
GTAAA 65

SEQ ID NO:4408

SEQUENCE LENGTH:124
SEQUENCE TYPE:nucleic acid
TOPOLOGY:linear
CLONE:HUMGS05238
SEQUENCE DESCRIPTION:
GATCTCTATA TTAAATNCTA AAATGGGATT AAAAGAAGAG TTGGAGAATT CACACTTATT 60
GAGTAACTGA TGTCATACAA CCTGGAATTT CTGAATTCCA AATAAATAAA TTTCACTCTT 120
TAAA 124

SEQ ID NO:4409
SEQUENCE LENGTH:234
SEQUENCE TYPE:nucleic acid
TOPOLOGY:linear
CLONE:HUMGS05239
SEQUENCE DESCRIPTION:
GATCTATGCA GTATACATTT TNCAGGCTGA AATTCAAGGG GAATCATTCT GATTATNCTT 60
ACTACAAATG GAGATGGCTA TTATGAAACA GCATGAGCAT GAGCCTTTNA TCTNTNATAC 120
TTAGTGATAT ACTTTGCTTG AAAATCACTC AGCAAAGTAG TTCACATGAT GTGTTATCAT 180
ATTTGANGTG TGGNTTNTCT CAAAATCATT GNCTTTAAGG NGCTCATTNC TGAN 234

SEQ ID NO:4410
SEQUENCE LENGTH:191
SEQUENCE TYPE:nucleic acid
TOPOLOGY:linear
CLONE:HUMGS05240
SEQUENCE DESCRIPTION:
GATCTNTTTG TATGTNACGT GTTAAGGGCT TGTTTGGTAT CCCACTGAAA TGTTCTGTGT 60
TGCAGACCAG AGTCTGTTTA TGTCAGGGGG ATGGGGCCAT TGCATCCTTA GCCATTGTCA 120
CAAAATATGT GGAGTAGTAA CTNAATATGT AAAGTNGTAA CATACATACA NTTAAAATGG 180
ANATGCAGAA A 191

SEQ ID NO:4411
SEQUENCE LENGTH:308
SEQUENCE TYPE:nucleic acid
TOPOLOGY:linear
CLONE:HUMGS05241
SEQUENCE DESCRIPTION:
GATCACCTGA GCTGCCTCAG ATTCCATTTG GTCCTCTCCT TCCTGGAAGG CTTCCTTTTA 60
TGTTTTGTTT TAATCCCAAA TGTCTGAATG TTTTGCAGTG TGTAGGGGTT TGAGCCCCTT 120
GTTCATTCTC CTTCCTTTTT CCTCCCGCTT CCCTCTCCAT GAAGTGATTC TGTTGACAAT 180
AATGTATACT GCGCGTNCTC TTCACTGGTT TATCTGCAGA AATNNCTCTG GGCTTTTNTC 240
GGTGTTAGAT TCAACACTGC GCTAAAGCGG GGATGTTCCA TTGAATAAAA GAGCAGTGTG 300
GTTTTAAA 308

SEQ ID NO:4412

SEQUENCE LENGTH:210
SEQUENCE TYPE:nucleic acid
TOPOLOGY:linear
CLONE:HUMGS05242
SEQUENCE DESCRIPTION:
GATCCACCCA CCTTGGCCTC CCAAAGTGCT GGGATTACAG GCGTGACACN CAAACCCAGC 60
CACCAATTTT ACTTTAGGTA AACTTTTATT TTCAAGCTTT TGTTGGTGTT GCAAGTGTAA 120
ATCTGTTTTA TAAAATGTTC TATAAATATA ACCACTATTC CTTGTAAGCT ATTTAAAATA 180
AATTTTAAAG TCTTTCAAGT AAACACCAAA 210

SEQ ID NO:4413
SEQUENCE LENGTH:337
SEQUENCE TYPE:nucleic acid
TOPOLOGY:linear
CLONE:HUMGS05243
SEQUENCE DESCRIPTION:
GATCTGAATT TAGGNACATT TCCTTTTTCT GTGGGAATCA CAAGAGTTTA TCCACTAAAA 60
AAAGATATGT AAAAAAGATA CTTTCCAGCA CATAAATAAA GGCTGGAATT TTACAACCTG 120
ATGTATATAT TAGACAGTTC TAGGATGTTA GTTCCCTNCA TNCCAGAGCT ATGCTTGTGA 180
TACAGCCCCT TTTCTTATAA AGTCAGTTAG ANGGACTTCC TTAACAATGA CTATTATAAT 240
GNCTTNCTTA AAATACAGTT TTGTATTCTG TCAATGCAAA TATAAGACAG GTTGTGCCNT 300
AATCATGTAN CANGNNTATT TTGNAGNTTT ACATATN 337

SEQ ID NO:4414
SEQUENCE LENGTH:120
SEQUENCE TYPE:nucleic acid
TOPOLOGY:linear
CLONE:HUMGS05244
SEQUENCE DESCRIPTION:
GATCAGCTAG CTACCTTCTA CTTTCCATTT TTCAAGTGGA TTGTTCTCTT AATTTGTATA 60
TAACCTGTTG TCTAAATTTT ATGTACAGTC TTTTATAATA AACCATTCTC CTATATGAAA 120

SEQ ID NO:4415
SEQUENCE LENGTH:185
SEQUENCE TYPE:nucleic acid
TOPOLOGY:linear
CLONE:HUMGS05245
SEQUENCE DESCRIPTION:
GATCTGGCTC ATCCATCTGC ACACTGACCA CGCTAAGAGC TGTCTGCAGT GATGGAAACC 60
TTCTCTAGCT GTGCTGTCCT CTATAGTGGC ACCAGCTGCC TGTGGCTACT GAGCACTTCA 120
ACTGTGGCTG CAGAGACTAT GTTAAATTGT ATTTAATCAT AATTAATTTA ATTGTAAACA 180
ACAAA 185

SEQ ID NO:4416
SEQUENCE LENGTH:58

SEQUENCE TYPE:nucleic acid
TOPOLOGY:linear
CLONE:HUMGS05246
SEQUENCE DESCRIPTION:
GATCTTAAAA TTCGTTTGCC TTTTTAAAGC TATATTAAAA ANGTATTGTT GAATCAAA 58

SEQ ID NO:4417
SEQUENCE LENGTH:109
SEQUENCE TYPE:nucleic acid
TOPOLOGY:linear
CLONE:HUMGS05247
SEQUENCE DESCRIPTION:
GATCCACAAG ATTNAGAAAA TATTATATAG TTAGATAATA ACATTCTTGT CTACTGATAT 60
CCNANCTNGT TACAAAATTN TTTAAAACTT AAATAAAAAC ATGCATCTN 109

SEQ ID NO:4418
SEQUENCE LENGTH:363
SEQUENCE TYPE:nucleic acid
TOPOLOGY:linear
CLONE:HUMGS05248
SEQUENCE DESCRIPTION:
GATCCCTCTA CAGAGCTTCC CTGACTCATT CTGAAGGAGC CCCATTCCTG GGAAATATTC 60
CCTAGAAACT TCCAAATCCC CTAAGCAGAC CACTGATAAA ACCATGTAGA AAATTTGTNA 120
TTTTGCAACC TCGCTGGACT CTCAGTCTCT GAGCAGTGAA TGATTCAGTG TTAAATGTGA 180
TGAATACTGT ATTTNGTATT GTTTCAATTG CATCTCCCAG ATAATGTGAA AATGGTCCAG 240
GAGAAGGCCA ATTCCTATAC GCAGCGTGCT TTAAAAAATA AATAAGANAC AACTCTTTGA 300
GAAACANCAA TTTCTACTTT GAAGTCATAC CAATGAAAAN ATGTATATGC ACTTATAATT 360
TTN 363

SEQ ID NO:4419
SEQUENCE LENGTH:266
SEQUENCE TYPE:nucleic acid
TOPOLOGY:linear
CLONE:HUMGS05249
SEQUENCE DESCRIPTION:
GATCACATTC AGGATAAGTG TACAGAAGAA AATACGGGTG TTTACTCTTT AGGGAACTGG 60
AAACACTCCC TGCATTGATG TACATTTTAA GAATGGCACT TTTGATACAT GTTATCATAA 120
AGGTGCTTAA TAGAGCTGAA TTAAAGTTTT TCAAATCTGT AAACANNGCA AAAANGTAAA 180
TTGTAGTCAT TTGATTATTT TTTAAATNGG NGCTTTATAT TTNGTNCTCA CTCAGAGTAA 240
AAGCTGCAAT TTATTGTNCA CCCAAA 266

SEQ ID NO:4420
SEQUENCE LENGTH:104
SEQUENCE TYPE:nucleic acid
TOPOLOGY:linear

CLONE:HUMGS05251
SEQUENCE DESCRIPTION:
GATCAAAGCA ATTTACAGAT TCAATGCTAT TCCTGTCAAA CCACCTATGA CATTCTTCAC 60
AGAACTAGAA GAATAAACTA TTAAAAGTTC GTATAAAGTT TAAA 104

SEQ ID NO:4421
SEQUENCE LENGTH:256
SEQUENCE TYPE:nucleic acid
TOPOLOGY:linear
CLONE:HUMGS05252
SEQUENCE DESCRIPTION:
GATCTCAACA GTGTGGGGTG AACAAGAAGA CTGTATCTTC AGCCTTTTTC CTATAATCAT 60
GGATGATTTG GTCTTATTCA AAAGGACCGC ACATATTAGT ACTCTTAAGA GCATCTTCCA 120
AGACTCCAGC AGNGAGCATT TAGAGAGTGT GTTGTCTTCC AGAGTCATGA ATGATTTTGT 180
TTAGCTATCA GGTCTACTAC CTCTAAGGAC ATTTCATAGC AGCATCTCTT GAGTTGCCTG 240
CATCAGTGTG GTGGTN 256

SEQ ID NO:4422
SEQUENCE LENGTH:255
SEQUENCE TYPE:nucleic acid
TOPOLOGY:linear
CLONE:HUMGS05253
SEQUENCE DESCRIPTION:
GATCTGAATA TAATTCTGGT AAACAGCTGT CTTCATTTTT NTCCTCTAAA GAACTTAATT 60
CATTTGTTAC ATAAAATATA AGGAAATCTT TGTACTATTT TACAGTAACC ACAATCTAAA 120
TATTTACATA TACCCAAAAT TANCTTATGC TCATATATTA GGATGTGAGA NTATCATCTG 180
TTTATGGACA CATGAAACCT CCTAATGACC TGGAATTGTT AGANTATNTG ACTTTTTATA 240
TGCAAAGTTT TTCGN 255

SEQ ID NO:4423
SEQUENCE LENGTH:58
SEQUENCE TYPE:nucleic acid
TOPOLOGY:linear
CLONE:HUMGS05254
SEQUENCE DESCRIPTION:
GATCCCCGGT GGTTTTGTGC TCAAAATAAA AACTGATTAA ACCTTTGTGG CTGTGAAA 58

SEQ ID NO:4424
SEQUENCE LENGTH:234
SEQUENCE TYPE:nucleic acid
TOPOLOGY:linear
CLONE:HUMGS05255
SEQUENCE DESCRIPTION:
GATCCTTTTC TTCTCTTATA AATCATCCTC TTAATGAAAA TTAGCCTAAC AAAAGTTTGG 60
AGACTGGAAT CCTACTTTGA GCCACTGACT TGAAATAACT CTTTTGGCAA GTTGCCTGAC 120

ATCCTGTCTT ACCAAGGTGG CAGGTTTGCA TTTTTACTGC TTAAAACATT TTTNTTTTTT 180
TAACCATCTT NANCCAAATT NATCATATNN GNGGTNGGCC TANCAGGNTT TTNN 234

SEQ ID NO:4425
SEQUENCE LENGTH:200
SEQUENCE TYPE:nucleic acid
TOPOLOGY:linear
CLONE:HUMGS05259
SEQUENCE DESCRIPTION:
GATCCACCTG CCTCAGCCTC CCAAANTGCA NGGTATTACA GNCANGAGNT TCTGCGTCTG 60
GCAATAATGT AACTTTGAAG CTTAAAAATT AATCCCAGTT TGTAGCAATA ACAGAAGACT 120
ATCTACAACG GAAGAAAGAA GCAACTGCCT TACAGTTCTG TAAAGANTTG GCAAGAAAAT 180
AAAGCCTATA GTTGCCGAAA 200

SEQ ID NO:4426
SEQUENCE LENGTH:84
SEQUENCE TYPE:nucleic acid
TOPOLOGY:linear
CLONE:HUMGS05260
SEQUENCE DESCRIPTION:
GATCTAGTCA TCTTCTGTAG CTACATAGTA CTCCTAATGT TAAGATTACC AAATTCAGCA 60
ATAAAGCCAA AATCAAAACT GAAA 84

SEQ ID NO:4427
SEQUENCE LENGTH:75
SEQUENCE TYPE:nucleic acid
TOPOLOGY:linear
CLONE:HUMGS05262
SEQUENCE DESCRIPTION:
GATCATAAAC CAGTGNNATC AGACCTATGT GTAATAAGAC TCCTGTTAAT ACAAAAATAA 60
AAAGCTAAAA GCAAA 75

SEQ ID NO:4428
SEQUENCE LENGTH:424
SEQUENCE TYPE:nucleic acid
TOPOLOGY:linear
CLONE:HUMGS05263
SEQUENCE DESCRIPTION:
GATCTCAAAC TTGTCTTAAA TCAACAACCT TCTACTCATG TTAATGTCTT GATTAAATAT 60
CACAATGCAA AATACACATT AAGTAAAAGA ATTCCAGCTG GTAAACATGA CCTGGACATT 120
TGTAAGAATA TATTTAATAT ATGTNCACCC ATTATGTTTT TAGGTAACAG GNGGNNAAAT 180
GCANCNCAAT TTTTTTTCTC TTGAAAGGCA CTGTCATTTA ANCATAAACC TGGAGTACTC 240
GAAATAGAAT TCAGGTTTAC ANGATGAAAG CGTGTGGAGA AGTGTCAGAT GGCAGTGGNA 300
GCATGTGTGT TTCTAAAANG TAAAANTCTC AAGGAAACCA NGANATGGCA TGCTTTTACC 360
CATCTTNCCT TAGTGAAAAG NGTGCTTCAG GTTTGANATT GTTTTAAGAA AGNTAGCCGG 420

GNTN 424

SEQ ID NO:4429
SEQUENCE LENGTH:122
SEQUENCE TYPE:nucleic acid
TOPOLOGY:linear
CLONE:HUMGS05264
SEQUENCE DESCRIPTION:
GATCTGCTCC CTCTCGCTCT CTCACTCCAC TACTTTTTGG AACAAAGTGA TGGCAGAATG 60
CGGTGGTGGT GGGGGTCTTT TGTACTGTTG GATTAATAAA ATGATTTNAA ATTNCNNNTA 120
AA 122

SEQ ID NO:4430
SEQUENCE LENGTH:85
SEQUENCE TYPE:nucleic acid
TOPOLOGY:linear
CLONE:HUMGS05266
SEQUENCE DESCRIPTION:
GATCAGTATA ATTTTTNAAA GTTACTTTGT NAGAGGCACA AAAGGGTTTA AACTGNTTCA 60
TAATAAATAT CTGTACTTCT TCAAA 85

SEQ ID NO:4431
SEQUENCE LENGTH:187
SEQUENCE TYPE:nucleic acid
TOPOLOGY:linear
CLONE:HUMGS05267
SEQUENCE DESCRIPTION:
GATCACGTAG ACATGTAACC CAGCAGCAGT TTGCTTCTGT TGTCCACTGA TTAATCAGCC 60
TATGTGCCTG ACACTGGTCT TGCAGTACAA CTGNAAGCCA AAACAAGGTG GAAGATGTCC 120
TGAATTAAGA TGTTTTCACC ACATTGTATT ACAGAGACAG CCAATAAATC TACTATTTGA 180
TTTCAAA 187

SEQ ID NO:4432
SEQUENCE LENGTH:211
SEQUENCE TYPE:nucleic acid
TOPOLOGY:linear
CLONE:HUMGS05269
SEQUENCE DESCRIPTION:
GATCTTTTAT TTNTGAAACA CTCAAACACC TTACAAAGTG CTGAGTAGGT AATAGTGACC 60
CAACTTGTTT GCTAAATGAT TATTTGTTTA AATCTGTACA GTTTTAAGTG TTCACTTATA 120
CAAAGAGTGT ATATACTTTC AAATAATTNA AAATGCTTTA NATTATNNGG CTAGAAATTG 180
CNGTTTTNAA TAAATGTGAA TNTNTNAAAA N 211

SEQ ID NO:4433
SEQUENCE LENGTH:62

SEQUENCE TYPE:nucleic acid
TOPOLOGY:linear
CLONE:HUMGS05270
SEQUENCE DESCRIPTION:
GATCTGTTGT TAGGATTCTA GTTAACACTT TTNTTTTTAA AAACTAAGGA CAAATGAACA 60
AA 62

SEQ ID NO:4434
SEQUENCE LENGTH:98
SEQUENCE TYPE:nucleic acid
TOPOLOGY:linear
CLONE:HUMGS05271
SEQUENCE DESCRIPTION:
GATCCCAGGG GAGGGGTCTC TCTCCCCATC CCAAGTCATC CAGCCCTTCT CCCTGCACTC 60
ATGAAACCCC AATAAATATC CTCATTGACA ACCAGAAA 98

SEQ ID NO:4435
SEQUENCE LENGTH:239
SEQUENCE TYPE:nucleic acid
TOPOLOGY:linear
CLONE:HUMGS05272
SEQUENCE DESCRIPTION:
GATCGTATCC CGCTGACACA GAAGGNTGCC GGGTGGGGGG AGGGCACCCA TGAAATTCAA 60
AGAAAACCCT AAGTGTGGAT GTTCAGAATT TTGGAAAAAT GTCACTGAAG TGATTGAAAG 120
AGTGCCTGGC ATGCAAGGNT AAGAGGTTCT TTACATAACA TTTCCTAAAT GCTTAATAAA 180
CGTTTGTTGC ACTTGGTTTT GACATTGCCA ATGGGGTTTG AACCAGTGCT CTCTCTAAA 239

SEQ ID NO:4436
SEQUENCE LENGTH:102
SEQUENCE TYPE:nucleic acid
TOPOLOGY:linear
CLONE:HUMGS05273
SEQUENCE DESCRIPTION:
GATCTGCAAT GACTGGAACT TGCCGGTGCC TGGGGTGCCT TTCCCCCAGC CAGGGTCCAA 60
AGAAGCTTGG CTGGGGCAGA AATAAACCAT ATTGGTCGGA AA 102

SEQ ID NO:4437
SEQUENCE LENGTH:333
SEQUENCE TYPE:nucleic acid
TOPOLOGY:linear
CLONE:HUMGS05274
SEQUENCE DESCRIPTION:
GATCTTCTTT TGGGAGGATT TGATGTAAGT NACTGACAAG CCTCAGCAAA CCCAAAGATG 60
TTAACAGTAT TTTAAGAAGT TGCTGCAGAT TCCTTTGGCC ACTGTATTTG TTAATTNCTT 120
GCAATTTGAA GGTACGAGTA GAGGTTTAAA GAAAANTCAG TTTTTGTNCT TAAAANTGCA 180

TTTAAGTTGT AAACGTCTTT TTAAGCCTTT GANGTGCCTC TGATTCTATG TAACTNGTTG 240
CAGACTGGTG TTAATGAGTA TATGTNACAG TTTAAAANAA AAGTTGGTAT TTTATANGCA 300
CAGTCAATNC TNATGGTAAC TTTTGGTNGT CTN 333

SEQ ID NO:4438
SEQUENCE LENGTH:72
SEQUENCE TYPE:nucleic acid
TOPOLOGY:linear
CLONE:HUMGS05275
SEQUENCE DESCRIPTION:
GATCAATGTT TCAGCCCTAG ACTGCCCAAG GAGTATTATT AATTATTAAT AAATGAATTC 60
TGTGCTTTTA AA 72

SEQ ID NO:4439
SEQUENCE LENGTH:100
SEQUENCE TYPE:nucleic acid
TOPOLOGY:linear
CLONE:HUMGS05276
SEQUENCE DESCRIPTION:
GATCAGATTA TTTTACTACC AACAGTTATA GTTTGAAAGT CCAACTGTAT TAATTGACTG 60
ATAATATGAT AATATAGAGA TTAAATTGTT TGTNTTCAAA 100

SEQ ID NO:4440
SEQUENCE LENGTH:96
SEQUENCE TYPE:nucleic acid
TOPOLOGY:linear
CLONE:HUMGS05278
SEQUENCE DESCRIPTION:
GATCACAAAG AGCAAACAAA TGTCAATATC TCTTATGAAT GGTGAAAAAA AGGAATTAAT 60
CGATGCAAAT AAACTTTTCA CAGTATTACT TTTAAA 96

SEQ ID NO:4441
SEQUENCE LENGTH:116
SEQUENCE TYPE:nucleic acid
TOPOLOGY:linear
CLONE:HUMGS05279
SEQUENCE DESCRIPTION:
GATCTAGCAA GCCATGGCGA CAGGTAGCAT TTNTAAGATG NTGNACAGGA GCAGNATTAT 60
CCCCAAAGAT ATTACAGGGT AGACACGTTT TAACTAAAAT CAATCAAGNT AACTTN 116

SEQ ID NO:4442
SEQUENCE LENGTH:411
SEQUENCE TYPE:nucleic acid
TOPOLOGY:linear
CLONE:HUMGS05280

SEQUENCE DESCRIPTION:
GATCTGACAT ATATCACCTT CTGGGNTATT TACTCATTGT GCCAGGACCT GGCATTTTCA 60
TGTGCCTTTG ACCAAGTGTT CAGAATTTGC TTGNCTCTAA CCTGGAGAGC TTCTTAAGTG 120
ATGCCCCTTC ATGGAGCTTC TATGACAGTG AATAAACTAT TAATTGAAGG AAAATGTTAT 180
AATTAANGTA TCTATTTGCT GCATTGTATA TGGATTAANT GNTAAAAAAC AAGTAATCTA 240
CCCTCAGAGC CATGTATTTG AGAATGCTTT CAATCATATT TNCCTATGNA CTTTTTTTTT 300
ATAAACTTAG TTTTANGCCT ATGTTGTAAA ANTGGGAAGG TTNGTAAACN TATGTTTGGN 360
AAAANTAGGG GAAATGTNGG CTTAAANTTN TTTTNCANTT ATNTTNGTTT N 411

SEQ ID NO:4443
SEQUENCE LENGTH:90
SEQUENCE TYPE:nucleic acid
TOPOLOGY:linear
CLONE:HUMGS05281
SEQUENCE DESCRIPTION:
GATCTATCTG GGGGTCCTAG TGTACCACAA GCTGAACCTG AAGATTTGGA AAAATGTTTC 60
TAATTAAATA AAACAGAGCA AAAAGACAAA 90

SEQ ID NO:4444
SEQUENCE LENGTH:342
SEQUENCE TYPE:nucleic acid
TOPOLOGY:linear
CLONE:HUMGS05282
SEQUENCE DESCRIPTION:
GATCTACATT TAAGTGGAAA AATTAGCAGT ATTTGAAAGC TCAGTTTNAA TCATTGTCTT 60
AACTTCAGAT ACAAATAACT GAACAGAAAG TTTTAACCTT TAATATCTCA TGTNCTGTTT 120
NTTATTCAGT ATTTTCCTNT ATGTTAATTC AATTATATAC TTCTGAATGG CACCTTACTT 180
TTTGGAAACA AATCTTCTGT TATTTACAAA ATAATAATTT TTANAAAACA TTTAAAAAAA 240
ATCCAAAGCT GCTCTCGATA ATAGTCAACA TTTGCATATA TATGGAATTT CTTACTTTNT 300
TTCTCCCAAA CTCTATTTAA TAAACTNATT TTAATGGTTA AA 342

SEQ ID NO:4445
SEQUENCE LENGTH:61
SEQUENCE TYPE:nucleic acid
TOPOLOGY:linear
CLONE:HUMGS05283
SEQUENCE DESCRIPTION:
GATCACAAAA ATGTATTTTA TAAAATGTTC TGTACAATAA AGTTACACCT CAAAGTGTAA 60
A 61

SEQ ID NO:4446
SEQUENCE LENGTH:301
SEQUENCE TYPE:nucleic acid
TOPOLOGY:linear
CLONE:HUMGS05284

SEQUENCE DESCRIPTION:

GATCATTATC AGTAATTTCA TAGCAACTGT TCTAGNGTTT TGNGTTTTTA AAACAGAATT 60
AGGAATTTGA GATATCTGAT TATATTTTTC ATATGAATCA CAGCTGTTGA CAATGTCCCA 120
TATATTTAAG AAATTATATC ATACTGATAC TATTTGTAAC ATTTTGATTT GATTTAATCT 180
CCAGGGACAG AAATAATTCA TTGGTAAAGN GTAATAATGC GTTTTTTAAA AATGCTTTGA 240
GAGGTAATTA CTTGCATATG AGAGAAATAA AACATTTGGC ACATTGTTTA CAGGTGTGAA 300
A 301

SEQ ID NO:4447
SEQUENCE LENGTH:153
SEQUENCE TYPE:nucleic acid
TOPOLOGY:linear
CLONE:HUMGS05285
SEQUENCE DESCRIPTION:

GATCAGACCG AGAAGCAGGG TGAGAGATTC TAACGACTGG ATGCTGCTAG TAACACATTG 60
TTTGTATTGC TTTACCATTT TTAACTGTTA GATTTGAGAT GAACATACAT TTTGCTTTTT 120
TAATAAATGT TTAAAAGAAG TCCACATAAG AAA 153

SEQ ID NO:4448
SEQUENCE LENGTH:216
SEQUENCE TYPE:nucleic acid
TOPOLOGY:linear
CLONE:HUMGS05286
SEQUENCE DESCRIPTION:

GATCACTACA GTGAAGTATT ACAGTTGTAC AGTTCCCAGT CTGGCCTTGG CTTGCTCGGA 60
TAAAACTTTG TATGTATTTT GTATGGCATA GATTCTATAT TGTAATGATG TCCTATGCAA 120
AAAGAAAAAT TAACGAAATT GTAAATTTTA TTGTTTTAAC GTGTATGCAT GTTTAGTGAC 180
GTTTACATTT TGAAATAAAA TTTATGATTC ATTAAA 216

SEQ ID NO:4449
SEQUENCE LENGTH:172
SEQUENCE TYPE:nucleic acid
TOPOLOGY:linear
CLONE:HUMGS05287
SEQUENCE DESCRIPTION:

GATCTGGAAA CGTTTTGTAC CGATTATCCA CAGCAAAACA AAAATAAGCT TTTATTTTAT 60
TAATAATTTC GTTCCTCTTG TGCCCAATCA AATCTTTTAG GAACAAACTG CAAGAAAAGC 120
TAAGAATGTT TTAGAGTGAA CTAAATACAG ACATTGCTTA CTTGTTTTGA AA 172

SEQ ID NO:4450
SEQUENCE LENGTH:289
SEQUENCE TYPE:nucleic acid
TOPOLOGY:linear
CLONE:HUMGS05288
SEQUENCE DESCRIPTION:

GATCTCCCTG TGGCAGCAGG CATGGAGAGT CAGGGCTGCC TTCATGGCAG TAGGCTCTAA 60
GTGGGTGACT GGCCACAGGC CGAGAAAAGG GTACAGCCTC TAGGTGGGGT TCCCAAAGAC 120
GCCTTCAGGC TGGACTGAGC TGCTCTCCCA CAGGGTTTCT GTGCAGCTGG ATTTCTCTG 180
TTGCATACAT GCCTGGCATC TGTCTCCCCT TGTTCCTGAG TGGCCCCACA TGGGGCTCTG 240
AGCAGGCTGT ATCTGGATTC TGGCAATAAA AGTACTCTGG ATGCTGAAA 289

SEQ ID NO:4451
SEQUENCE LENGTH:121
SEQUENCE TYPE:nucleic acid
TOPOLOGY:linear
CLONE:HUMGS05289
SEQUENCE DESCRIPTION:
GATCTCTTAA AAATACCACA GTTTGTATTT TTCCTTTAAG GAGTAAAGAT TTGCCTTTAA 60
ATAACTTGGT ATTTTCCTGG CTTTCGTTTA ATACAATAGA AAATAAAGTA TTACACCGAA 120
A 121

SEQ ID NO:4452
SEQUENCE LENGTH:293
SEQUENCE TYPE:nucleic acid
TOPOLOGY:linear
CLONE:HUMGS05290
SEQUENCE DESCRIPTION:
GATCAATGTG ACGGCAGGGA AATGTATGTG TGTCTATTTT GTAACTGTAA AGATGAATGT 60
CAGTTGTTAT TTATTGAAAT GATTTCACAG TGTGTGGTCA ACATTTCTCA TGTTGAAGCT 120
TTAAGAACTA AAATGTTCTA AATATCCCTT GGACATTTTA TGTCTTTCTT GTAAGGCATA 180
CTGCCTTGTT TAATGTTAAT TATGCAGTGT TTCCCTCTGT GTTAGAGCAG AGAGGTTTCG 240
ATATTTATTG ATGTTTTCAC AAAGAACAGG AAAATAAAAT ATTTAAAAAT AAA 293

SEQ ID NO:4453
SEQUENCE LENGTH:336
SEQUENCE TYPE:nucleic acid
TOPOLOGY:linear
CLONE:HUMGS05291
SEQUENCE DESCRIPTION:
GATCTAGGTC CTTCTATCTC GTCCCTCTAC AGATGTATTT TCCACTTGCA TAATTCATGC 60
CAACACTGGT TTTCTTAGGT TTCTCCATTT TCACCTCTAG TGATGGCCCT ACTCATATCT 120
TCTCTAATTT GGTCCTGATA CTTGTTTCTT TTCACGTTTT CCCATTTCCC TGTGGCTCAC 180
TGTCTTACAA TCACTGCTGT GGAATCATGA TACCACTTTT AGCTCTTTGC ATCTTCCTTC 240
AGTGTATTTT TGTTTTTCAA GAGGAAGTAG ATTTTAACTG GACAACTTTG AGTACTGACA 300
TCATTGATAA ATAAACTGGC TTGTGGTTTC AATAAA 336

SEQ ID NO:4454
SEQUENCE LENGTH:252
SEQUENCE TYPE:nucleic acid
TOPOLOGY:linear

CLONE:HUMGS05292
SEQUENCE DESCRIPTION:
GATCTGCTGC AACCATTATC TTNGTTCTTG AGTTCATCAT GCATTTAAAA TGAGATGCAA 60
GAGCATTTAG CATACCATGT AAATATGGAC CTTTTAAAAT TAAAATACTA TTGGAAGTGC 120
TTTCAACTCA GCCACATCTT AGCTATTAGT TCTTTGTGTT GTCTTATCAA AGTTTAATGG 180
ATACAGTTCC ACAGTTGTTG ATGTGTTTGT GTGTATATTC GATTNNNNNA AAGATGGTAG 240
TAAGTCAATA AA 252

SEQ ID NO:4455
SEQUENCE LENGTH:57
SEQUENCE TYPE:nucleic acid
TOPOLOGY:linear
CLONE:HUMGS05293
SEQUENCE DESCRIPTION:
GATCATACCA CTCCACTCCA GCCTGGGCAA CACAGCGAGA CTCCATGCTC CCCCAAA 57

SEQ ID NO:4456
SEQUENCE LENGTH:564
SEQUENCE TYPE:nucleic acid
TOPOLOGY:linear
CLONE:HUMGS05294
SEQUENCE DESCRIPTION:
GATCAGGCTC TCCTCATGCT GTTTAGTGAT GGCACTGTCC AGGTGAACTN CTACGGGGAC 60
CACACCAAGC TGATTCTCAG TGGCTGGGAG CCCCTCCTTG TGACTTTTGT GGCCCGAAAT 120
CGTAGTGCTT GTACTTACCT CGCTTCCCAC CTTCGGCAGC TGGGCTGCTC TCCAGACCTG 180
CGGCAGCGAC TCCGCTATGC TCTGCGCCTG CTCCGGGACC GCAGCCCAGC CTAGGACCCA 240
AGCCCTGAGG CCTGAGGCCT GTGCCTGTNA GGCTCTGGCC CTTGCCTTTG TGGCCTTCCC 300
CNTTCCTTTG GTGCCTNACT GGGGGCTTTG GGCCGAATCC CCCAGGGAAT CAAGGGACCA 360
GCTTTACTNG AGTTTGGGGN CGGGNTTGTT TTCGNTGGNT TCCTACCCCA TNTCCAAGAT 420
AAGCCTTNAG CNTTAANTCC CANGNTAGGG GGCGTTATTT NATGGACCAA TTTTTAATTT 480
ATTNTAAGAA ANTTTNTTTA NTTGGGGATT TTAAGCCCAA AGGGGGGCCC TCNTCCTNAG 540
GTNANAAAAN AATTTTTTAA GAAA 564

SEQ ID NO:4457
SEQUENCE LENGTH:112
SEQUENCE TYPE:nucleic acid
TOPOLOGY:linear
CLONE:HUMGS05295
SEQUENCE DESCRIPTION:
GATCTTTATT TTTGATTAAT TTGTTCTGAT TTTTTGGTTT GTTTTTTAAG GAACTGTAAT 60
GAACAAATGT CAGGATATCC AATGCCAAAT AAAGATGTTG TATTTATTTA AA 112

SEQ ID NO:4458
SEQUENCE LENGTH:152
SEQUENCE TYPE:nucleic acid

TOPOLOGY:linear
CLONE:HUMGS05296
SEQUENCE DESCRIPTION:
GATCACACCC NGGTGAACCC ANGGTGCTAA GANTGAAAAT AACCTTGGTG AAATGTACAA 60
NTTAAAGACA AAGAACTACA TGTGAAGATA GACTTGCTTT CTATTTTNAA ATCAGTAGTA 120
GTACTGTTGC TGAATAATAC TAGGTTTTNN CN 152

SEQ ID NO:4459
SEQUENCE LENGTH:323
SEQUENCE TYPE:nucleic acid
TOPOLOGY:linear
CLONE:HUMGS05297
SEQUENCE DESCRIPTION:
GATCTGGTGC TGCTTTCAGA TGTTTATCTT TTATTTTTTT CCCTTAAGCT TTAATCTTCG 60
TCATTGTCTT AAAGTCAACT GGTGTTTCTT GTTCATTGAC TTTGGTACGA TGGTGCTTTG 120
CAAGGATGTA TTTATGTTAT AATGGCCAAC ATTTGGTCAG CCCTTGTCCA CTTATTCACT 180
TCCCTCCNTT TGTAAAATAA GTGCTTTAAT TATAAACTGT ATAAAAATAC CTTGTATAAA 240
CCCCTTTTTT GATTATTACA ATAAATAAGC TGAATTGTAA CAAATGAAAT TTGATTTTTG 300
TAATAAAACA GTGGAAAAGT AAA 323

SEQ ID NO:4460
SEQUENCE LENGTH:229
SEQUENCE TYPE:nucleic acid
TOPOLOGY:linear
CLONE:HUMGS05298
SEQUENCE DESCRIPTION:
GATCCTTTTT AGTTAATACT ATGACAGTAC TAAAAATATA TGAATAACAT TTCAGATACC 60
ATTATATTAA AATATTTGTG TATGTGTACA AAAGCGTTGA TAAATACTAA TCTTTAAAGT 120
TTGTGGAGTT CCTTTATTTG TAATATATGT GCTCTTAAAA GCAATGGGAT GTGAAATTAT 180
GAAAGTATTT TATTGTTCAT AGAAATAAAA AACACAGTTA CTTTGCAAA 229

SEQ ID NO:4461
SEQUENCE LENGTH:135
SEQUENCE TYPE:nucleic acid
TOPOLOGY:linear
CLONE:HUMGS05300
SEQUENCE DESCRIPTION:
GATCTGGACC TAATGAATTC CTCAGAAAAT TCCCATGAGT CAGCTGATTT CAGTGCTGCT 60
GAACTAATTN CTGTGTCTAA ATTTCTTCCT ATTTCTGGAA TGGAAAAGGA GGNCATTCTG 120
AGCCACACTG NNNNN 135

SEQ ID NO:4462
SEQUENCE LENGTH:102
SEQUENCE TYPE:nucleic acid
TOPOLOGY:linear

CLONE:HUMGS05301
SEQUENCE DESCRIPTION:
GATCTGGGTA AAGGACCATC TGGGGATTCT TTGTACTATT TTTCTTTCCG CAACTTTTCA 60
AGTTTCAAAC TATTTGCAAA TAAAGAGTTT TTGTTTGTTA AA 102

SEQ ID NO:4463
SEQUENCE LENGTH:189
SEQUENCE TYPE:nucleic acid
TOPOLOGY:linear
CLONE:HUMGS05302
SEQUENCE DESCRIPTION:
GATCACATGG CTAAGGTGGT GTGTGCCAGG TTTCTCCACT GTAAAATTAG GAATTTTCCC 60
TTTGAAATTA ACAAGAATCT TCCACTTTGA AGCTGTGTGA ATGTCTTATT CTTAAAATTT 120
TGCCTACTGA TTTTNANNNN CATCATCAAT GATTCTTGCT TATAACAGGT ATTAATGTTA 180
TGCTTGAAA 189

SEQ ID NO:4464
SEQUENCE LENGTH:364
SEQUENCE TYPE:nucleic acid
TOPOLOGY:linear
CLONE:HUMGS05303
SEQUENCE DESCRIPTION:
GATCATGTTA ATTTAAAGCT TTATACACAT TGTTGTTTTT GCTGGTCTCA TCTTTGGTAA 60
TATGCTATAC CCCACTGCTG CCCGACACTG CCCTNTAGCT GCAGAGCTGG ATTAGCTGTT 120
GACCATTTGA TGCTGTTGTC TGTCTGGCAG GGACTGAATG ACCTGATGTC AGATTTAGAT 180
TCTTCCTGGG GATTACACAG CTATGAATGT ATTTGCTTCT AAAACCTCCC AAAGTGAATC 240
TAATCTTAAA ACTACAAGTT GTAAGTATTC TGAAATTGGG AAACATTTAT NTNAAATGCA 300
ATCAGGNNAG TGTTGCTTTT TACAGCATAA TNAATATANG TNTCAANNAN NGTGGANGGT 360
NGTN 364

SEQ ID NO:4465
SEQUENCE LENGTH:229
SEQUENCE TYPE:nucleic acid
TOPOLOGY:linear
CLONE:HUMGS05304
SEQUENCE DESCRIPTION:
GATCACTTGA TGTCAGGGAG TTTGAGACCA GCCTGGCCAA CATGGTGAAC CCCATCTCTA 60
CTAAAAATAC GAAAATTAGC TGGGTGTTGT GGTAGGCACC TGTAATCTCA GTTACCTAGG 120
AAACTGAGGC AGGAGAATCA CTTGAACCTG GGAGTTGGAG GTTGCAGTGA GCCAAGATTG 180
TGCCACTGCA GTCCAGCCTG GGTGATGAAA CAAGACTCTT TGTCTCAAA 229

SEQ ID NO:4466
SEQUENCE LENGTH:496
SEQUENCE TYPE:nucleic acid
TOPOLOGY:linear

CLONE:HUMGS05305
SEQUENCE DESCRIPTION:
GATCTCTAAG GAACTCCTGT TGCTAAATAT GAAGAGTATG GAACATTCAT ATAGTCTCTG 60
TGAAGCATGG GGGGAGGGAA GACATTTCTN TTNCTTATAG GCTTTATGCT CAAATGTCAT 120
AGTCTCCTTT CAAAGAATTG TGTTGCATTT TAAATGCACC CAGCTTAAGT AGAAGACATT 180
GAAGGATGCA TTAATTTTCA GGAACTATTT TGGATTATGA AAAGATTCCC AATTGAAAAA 240
ATTATTCAAC AAGTAAAAGC TAAGAAATTN CATTGAAATC ATAATGGCAG TTTAAGCATA 300
AATTTGATAA ANAATAGCTG TGTACTACTA AATTAATTAG GAAAATTCNA TTCCAANCCC 360
AAGNNGAAGG AGTCCANGGT GGAAATATCC GGTTTGGTTT TATTTTGNNC TTAGGNTGAN 420
GTTTTCCTTT GGTNAACCGN TTGNNTTTTT TATTTAANAT NGNTTAATTA TTTTTNGGTT 480
AANGNTATGC CCCNTN 496

SEQ ID NO:4467
SEQUENCE LENGTH:415
SEQUENCE TYPE:nucleic acid
TOPOLOGY:linear
CLONE:HUMGS05306
SEQUENCE DESCRIPTION:
GATCTTGGGA GATATTAATA TCTGCTGGCC AGACCTAAAA AAAACTGAAG GCCCAAGGAA 60
AGTGACTGGA CTCACTGGAA AGTNTCTAAC AAATACTTTG GATTCCAAAG TATTTCCAAT 120
ACCTTGAAGA TTCCTTTGAA AACATCAAAG CACAAACTCA AATCTGCACA AGATTACAAA 180
AGCACAATTT TGGTGTTATT TTTAGANTNC AATACACAAA GCTATTGTTC ATTTCTNTCA 240
GCAAGCATTT TCCTGGGTAG TAGTAATAGC TTAGGAATCA AATGCCACTT TTNTTTTTCC 300
TTTTTTCCAC TAGTTTGAAA TACCACAANT NACAGGTNAG TCAGTCCTCC CAGAGGNNAA 360
NGGNCAGTCC TAGGGGNGGNA TCAACAGGNC TNGCAAGNCA AGNCCCACAA NTTNN 415

SEQ ID NO:4468
SEQUENCE LENGTH:209
SEQUENCE TYPE:nucleic acid
TOPOLOGY:linear
CLONE:HUMGS05307
SEQUENCE DESCRIPTION:
GATCGCAAAT TGAAATATGG ATATTTGTAC CAGGCTGCGT GTTTTTCATT TTAAACACAC 60
AAGATTTAAT TGAAAGGACA TCAATAATCA TAATTGTGTA TTTAACAGGT GGTTTTNTAT 120
TAGTTTTCTN GTGTTTCAGA CTTTATGCAG CCATATAAAC TGTTCTCTAG GCATGCTGTG 180
ACATTTTAAT AAAAAGCAAA AGGAGCAAA 209

SEQ ID NO:4469
SEQUENCE LENGTH:353
SEQUENCE TYPE:nucleic acid
TOPOLOGY:linear
CLONE:HUMGS05308
SEQUENCE DESCRIPTION:
GATCGCACCT TTTATACCAG AGACCTGAGG CAGATGAAAT TNATTTCCAT CTAGGACTAG 60
AAAAACTTGG GTCTCTTACC GCGAGACTGA GAGGCAGAAG TCAGCCCGAA TGCCTGTNAG 120

TTTCATGGAG GGGAAACGCA AAACCTGCAG TTCCTGAGTA CTTTCTACAG GCCCGGCCCA 180
GCCTAGGCCC GGGGTGGCCA CACCACAGCA AGCCGGCCCC CNCTCTTTTG GCCTTGTGGA 240
TAAGGGAGAG TTGACCGTTT TCATCCTGGC CTCCTTTTGC TGTTTGGATG TTTCCACGGG 300
TCTNACTTAT ACCAAAGGGA AAACTCTTCA TTAAAGNCCG TATTTCTTCT AAA 353

SEQ ID NO:4470
SEQUENCE LENGTH:64
SEQUENCE TYPE:nucleic acid
TOPOLOGY:linear
CLONE:HUMGS05309
SEQUENCE DESCRIPTION:
GATCCTCTAA CACTGTACTA AAACATTTCA ATAAAATCAT TCTGACTGCG TTCATAAAAA 60
CAAA 64

SEQ ID NO:4471
SEQUENCE LENGTH:169
SEQUENCE TYPE:nucleic acid
TOPOLOGY:linear
CLONE:HUMGS05310
SEQUENCE DESCRIPTION:
GATCTATTAC TGACCGTATG ATGAGGCCAA CTTTTTTNCC TTGTGGTTAG CAAGACTGCA 60
AGAGATGGAA AAAAAGTAGT TTGAATGTTT TGTGTGTAAG GAGTATACCA TGAGATGAGA 120
TGACCACCAA TCATTTCCTT GGGGGGAGGG GGTGTCTGCA CCNTAGAAA 169

SEQ ID NO:4472
SEQUENCE LENGTH:132
SEQUENCE TYPE:nucleic acid
TOPOLOGY:linear
CLONE:HUMGS05311
SEQUENCE DESCRIPTION:
GATCACTTGA ACCCAGGAGT TTAAGTCCAG TCTGAGCAAG GCAGTGAGAC CCTGTCTCTA 60
AACAATAAAA ATTAAAATAA GTATTATCGA GAGTTCCTGG AGGGGTGAAA TTAAAGACTT 120
TCAAAACTTA AA 132

SEQ ID NO:4473
SEQUENCE LENGTH:328
SEQUENCE TYPE:nucleic acid
TOPOLOGY:linear
CLONE:HUMGS05312
SEQUENCE DESCRIPTION:
GATCACCAGG CTGCCCAAGG GTGCTGTGCT CTACAAGACT TTTGTCCACG NGGNTCCTGC 60
CAAGCCTGAG GGCACCCGTC AAACTGGTAG CTATGCTTNG ATGTCCTGTT GAGGCCATCG 120
GACAGAGACT GGAGCCCANG TGACAGGAGA TGGTGATACC AGAAGTCAAG GGTTGGGGTG 180
GCGACACGGC CTCCCGAGGA AGAGGTCTGC TTGATGGTGA CTCTGCAGGA GACTCTGAAG 240
TGACTGCTGG GAAACCCTTT GGGAGACCTN ACCTGNGGCC AAAANTAAAG TNNGCCAGCN 300

TCATGAACCG CATNCTATTT AAGGGAAA 328

SEQ ID NO:4474
SEQUENCE LENGTH:307
SEQUENCE TYPE:nucleic acid
TOPOLOGY:linear
CLONE:HUMGS05313
SEQUENCE DESCRIPTION:
GATCTGGACA TGCTGTNACA AGAAATAGTG ACCTTGGGGA ATATGTACAG AAAAGACCTT 60
TTTCTAAGAG TTGGGAGGGG GTGGGGGACT GTATAATGAA AAGAATTAGC TTACAAAAAA 120
GAAATTGATA TTCAAAGTAT TTTAGGCAAA GCCAGTCAAA ATGCTTTCAT GATATTTTGA 180
GATGTAAATT TTGTCTGATT TGTATTGTNC TTTTCATTTG CCTTCTTAAC TTTATATGTG 240
ACCTGAATTT TCCATAACTT GATGACTATA GATTGCACCT AGGCATTCCA ATAAAAGCCA 300
ACCCAAA 307

SEQ ID NO:4475
SEQUENCE LENGTH:133
SEQUENCE TYPE:nucleic acid
TOPOLOGY:linear
CLONE:HUMGS05314
SEQUENCE DESCRIPTION:
GATCGTAAAA ACATGTCTTA TGCGGAACTT TCATGTCAGA TGCCTGGAGT GTGAAGTGTT 60
CATTGGACAT TCCATTTTAC TGTTCGAAGT ATGGACTTAC ATATCAAATT AAAAGGCTCT 120
TTTGTTCTGC AAA 133

SEQ ID NO:4476
SEQUENCE LENGTH:130
SEQUENCE TYPE:nucleic acid
TOPOLOGY:linear
CLONE:HUMGS05315
SEQUENCE DESCRIPTION:
GATCAACTGT CCTTAACCAC AGCAGCATGA TACGCTGGNT TACCCAATCG CTTGTAAGGN 60
AAGNCAGAGA TGACGTTATC CTGACTGCTG AGATGAGCAG TTTACCCTAG GAGGNCCNAT 120
TNGGACTATN 130

SEQ ID NO:4477
SEQUENCE LENGTH:105
SEQUENCE TYPE:nucleic acid
TOPOLOGY:linear
CLONE:HUMGS05316
SEQUENCE DESCRIPTION:
GATCAGCCAC ACAACTGTTT TGTACATACT TATTTTCTCA TGCACTTTTC TGTATGCAAA 60
TAAAGCTATA AATTTACTCA TTTCAATAAA CTGGAGTGGC AGAAA 105

SEQ ID NO:4478

SEQUENCE LENGTH:159
SEQUENCE TYPE:nucleic acid
TOPOLOGY:linear
CLONE:HUMGS05318
SEQUENCE DESCRIPTION:
GATCTGGGAC AGANTTCACT CTCACCATCA GCAGTCTGCA ACCTGAAGAN TTTNCAACTN 60
ANTACTGTCA ACAGAGTTAC AGTGCCCCGT GGACGCTCGG CCCAGGGACG AAGGTTGGAC 120
GTCAAACGAA CTGTGGCTGC ACCNTCTNTT TTCATCTTN 159

SEQ ID NO:4479
SEQUENCE LENGTH:170
SEQUENCE TYPE:nucleic acid
TOPOLOGY:linear
CLONE:HUMGS05319
SEQUENCE DESCRIPTION:
GATCTGAAAA GAAGGTGAAT ATTTTGCACT TTTGATACTC TTAGGAACAA ATAACTTATT 60
TGGCAAATTG TTTNTTTTTT ATGTTTTGGG TGTTGTTTTT GTTTTTTGAC TTTTTTTGTA 120
TTGTGAACAG GCACTGAAGC TGATGTTATT TTAAGATTAC AGAATGGAAA 170

SEQ ID NO:4480
SEQUENCE LENGTH:73
SEQUENCE TYPE:nucleic acid
TOPOLOGY:linear
CLONE:HUMGS05320
SEQUENCE DESCRIPTION:
GATCATGTAA AATTACAGTA CTCAACCCTT CTAGCAAAGT ATTTGGNTGA AAAATAAACT 60
AAATGCCTTT AAA 73

SEQ ID NO:4481
SEQUENCE LENGTH:130
SEQUENCE TYPE:nucleic acid
TOPOLOGY:linear
CLONE:HUMGS05321
SEQUENCE DESCRIPTION:
GATCCGCAGA GGGTGGTTGG GATACACCGG ATACCTCTGC TCTCATTGCT TGTTTCCAAA 60
TGCTCTATGG ACATTTGTGT GCTAAATCCT ATTAAATAAA AAAGACGGGT TAAAACCCAG 120
ATGCTGTAAA 130

SEQ ID NO:4482
SEQUENCE LENGTH:56
SEQUENCE TYPE:nucleic acid
TOPOLOGY:linear
CLONE:HUMGS05322
SEQUENCE DESCRIPTION:
GATCAAATTG AGAGACAATA AGTGTACATT GTTGAATAAA AAATTTTAAA GTTAAA 56

SEQ ID NO:4483
SEQUENCE LENGTH:90
SEQUENCE TYPE:nucleic acid
TOPOLOGY:linear
CLONE:HUMGS05323
SEQUENCE DESCRIPTION:
GATCTTGAGA GTATGTTCTT ACTCATTTCT GTAATTGCAG TAGTGTAGTC ATCTGCATAT 60
AGTTTTAATA AATGCATGTT AAAATTTAAA 90

SEQ ID NO:4484
SEQUENCE LENGTH:58
SEQUENCE TYPE:nucleic acid
TOPOLOGY:linear
CLONE:HUMGS05325
SEQUENCE DESCRIPTION:
GATCACGCCA TTGCACTCCA GCCTGGGGGA CAAGAGTGAA TCTGTGTCTC ACCAGAAA 58

SEQ ID NO:4485
SEQUENCE LENGTH:115
SEQUENCE TYPE:nucleic acid
TOPOLOGY:linear
CLONE:HUMGS05326
SEQUENCE DESCRIPTION:
GATCTGGTGA CTGTNCTCTC CCTGTTTTCC TTTCTTTTTG GGTGTTTGAG GAGCANNGCT 60
TAGCTTGAGA CACACACAGA CACTGTGTAC TTCAGTNAAG GGCTTAATAN ACAGN 115

SEQ ID NO:4486
SEQUENCE LENGTH:201
SEQUENCE TYPE:nucleic acid
TOPOLOGY:linear
CLONE:HUMGS05327
SEQUENCE DESCRIPTION:
GATCATTTCT ATGTGTGTGG GTAATCTGGT CAGTAAGATT GAGACTTAGT TAAGATTCCC 60
CTTGGAAATT CCTTAATGTT TATTAGCTTC TAACTAGTGT TGTAAGTCCG ATGCCAGAAT 120
TTGGAGATTT GAGTTCTTCT TTTCATGGCT TTTATTCACT GTGACTAATA AGCTTCCTAA 180
TAAATCCTTG CCAGACTTAA A 201

SEQ ID NO:4487
SEQUENCE LENGTH:274
SEQUENCE TYPE:nucleic acid
TOPOLOGY:linear
CLONE:HUMGS05328
SEQUENCE DESCRIPTION:
GATCTAGTTC GTGTATAACT GTTAACTCAT TTTACATTTT TTNCTTCGTT GAATGTTTAT 60

CAGATTATAA ATATAGAGGC ATTTCTNCAT CCTGTTTAAT GTTTATTTTG TAATCATTGG 120
AGTATGTGAA AAATTTTNGT TTGTTTTGCT TTGGTCTCTA AGTAAACATG TTCAGCAAAG 180
TATTTNTNTT TTNAAGAAAT AAATNGNGNT TTGTATGTCC TNCCTTTTAG GTTTGAGGCA 240
AAGAGGAGAT GGGAANCCAA AGGCAACANG GGNN 274

SEQ ID NO:4488
SEQUENCE LENGTH:332
SEQUENCE TYPE:nucleic acid
TOPOLOGY:linear
CLONE:HUMGS05329
SEQUENCE DESCRIPTION:
GATCTNTGCC ATGCAGAGCA TCTTCCAGAA GACCAGGACT CTGGGAGGCG AGGAGAGCTG 60
AGCTGGGCCA CCTGGTCTCA GCCACCTGTT CTTGGCTCCC CAACAGACTC TGCACTGCAC 120
CATGGGAGGC TCCTGGGATG TTTGGAAGAA GAAACGGGCT TCTCCTTGAG GGGGTAGTGG 180
AGGGATTTTT TCCCCAGCAG TGGCCTCTGA GAGTNNNTCA GTGCCTGGTG GGGCAGGGCA 240
GGCCTCTTGG AGCACCTCCT CCCTGGNTCA GGGCCTGGAT GCAGGTGCCA AGCTCTCCAT 300
GTGGTGCATG TTNGACCCAG CCACGTGGTG AN 332

SEQ ID NO:4489
SEQUENCE LENGTH:155
SEQUENCE TYPE:nucleic acid
TOPOLOGY:linear
CLONE:HUMGS05331
SEQUENCE DESCRIPTION:
GATCTTAACT ATGAGTAATT CACTATCAAG AGCTAATTAT TTGAAAAAAT ATAAGATGTT 60
ATGGAGCAGA TAGCTCAAGC AGTTAAAAGA AATTNTGCAA CTNTAATTCT NTTCCTTATT 120
TTAATACTAC TACTTAATAA ATAGTATTTG CTAAA 155

SEQ ID NO:4490
SEQUENCE LENGTH:115
SEQUENCE TYPE:nucleic acid
TOPOLOGY:linear
CLONE:HUMGS05332
SEQUENCE DESCRIPTION:
GATCAAATCA GAGATTGACA GCCTGTGGAG GGTGCTGAAC TATACAGAAT TAGACACAAC 60
TATGTCATTA TTTTTTGTAC CTACTGNTCA GAATAAAAAC ACTTGAAATA TGAAA 115

SEQ ID NO:4491
SEQUENCE LENGTH:197
SEQUENCE TYPE:nucleic acid
TOPOLOGY:linear
CLONE:HUMGS05333
SEQUENCE DESCRIPTION:
GATCCCTTCT GTTGGTGTCA TGTTGTAAAC ATTTCTTTCA ATAAACTAAA GAAAAATTAT 60
TAAAGGAACA CATACCTTTG GTTAAATAGT CTAGACTAAA AGATTGAGAA GTTACTTTCC 120

ATTGCTATCT ATTGATAATT TAGACATTGA GTTCAAATTG CCTTCATTTT ATGATAAATA 180
ATGATTTAAC TGACAAA 197

SEQ ID NO:4492
SEQUENCE LENGTH:79
SEQUENCE TYPE:nucleic acid
TOPOLOGY:linear
CLONE:HUMGS05334
SEQUENCE DESCRIPTION:
GATCATTTTT ATTTTGTAAA TATTACCTAA CTTTACATAA ACTATATCAT AATAAACTAT 60
TTTTGCATCA CCCTTTAAA 79

SEQ ID NO:4493
SEQUENCE LENGTH:377
SEQUENCE TYPE:nucleic acid
TOPOLOGY:linear
CLONE:HUMGS05335
SEQUENCE DESCRIPTION:
GATCTCCAAG CTGAAACTTA CACGTTAAAA CTTTTGCCTG TAAGAATTTG CACATGAATG 60
TTAATGGAAA ACACAAAACT TAAGATGGCC CAAAACNAAA GCCACAAACA GTTCATCATT 120
TGGTGCTTAG TCTTTGTAAG GGCTCTCTGT GGTTTGACTT ACTCCAGCTA CCGTTAGATG 180
AGGGCAAATC ACCTTAGAAC ATGTTCATTT GATTCATAAC ANGGAAAATT GGGTCTATGA 240
TTTTTNGCCA ATCTTAGCCT AAAAGAAATT GCTTTAGCTT CTGGTCAGCA CTGATTAAAA 300
TGTGAATAGT GAAGTGGCTA TCCTAAACTG GTTTATCTCC ACCCACACTA TCATAGANTT 360
CTTAGGTAAT ACAATTN 377

SEQ ID NO:4494
SEQUENCE LENGTH:170
SEQUENCE TYPE:nucleic acid
TOPOLOGY:linear
CLONE:HUMGS05336
SEQUENCE DESCRIPTION:
GATCAAACAC ACACACCTAT ACACATACAT ATTAANATGC AGAGGATTTT AAATAATTGC 60
AAATGTCTTG TGGCTGTTTT ATTTCTACAA AATTCTGTAA GATGTATACC AGAAAACATA 120
ATAAAGATAA GCTCAGTTCT TCATTGGGNA AAANTATTTT TAAGNNNNNN 170

SEQ ID NO:4495
SEQUENCE LENGTH:57
SEQUENCE TYPE:nucleic acid
TOPOLOGY:linear
CLONE:HUMGS05337
SEQUENCE DESCRIPTION:
GATCTGTAAT GTGTCAAAGC ATTTTTCACT TTTCAAATAA AGATACCTAT AATGAAA 57

SEQ ID NO:4496

SEQUENCE LENGTH:373
SEQUENCE TYPE:nucleic acid
TOPOLOGY:linear
CLONE:HUMGS05339
SEQUENCE DESCRIPTION:
GATCAGAATC TNCCAACTTT AGAGGTGCAA TGGAAGACAC TACGCTACTT GGTTGAGCCT 60
GGTGAAGAAT GTATTAATGA GACTNCTTTG CATAAAACTG GGAAGAAAGA GAAGACAGTT 120
GGAGATGGAA GATGGTTTTN TATATATNTN GGAACTTTAG TTCCTCTGTA AGACGAAAGA 180
GGAGAGCTAT GTTTNNTNTC ACATTGTCTG ATATATATNG TGTACCTGTC AGGTGAGTTG 240
ATTTAGACAN CATAGCTGAC CTTTTATGAC ANGGCAGTTT GANTAGGGNC TATTGTAATA 300
CCCTCACACA TTATAGGGGC ATCAGAGNAT GGCCATGGNA GGGACAGTCT TACAGGGGGC 360
TTTANGGNGN CCN 373

SEQ ID NO:4497
SEQUENCE LENGTH:368
SEQUENCE TYPE:nucleic acid
TOPOLOGY:linear
CLONE:HUMGS05340
SEQUENCE DESCRIPTION:
GATCTGAAGA AGTTGCTGTT CTGAAACAGA AGTTGGAGCG ATGTCATTCT GCTGAGCTTG 60
CACTTAACGT AATCACGAAG TAGAGGAAAG GCTATGNAGT TGATGATTAC GTCTCAAAGA 120
AATCCAAACA TGAGGAGGAA GAATGGACTG ATGACGACCT GGTAGAATCT CTCTAACCAT 180
TTGAAGTTGA TTTCTCAATG CTAACTAATC AAGAGAAGTA GGAAGCATAT CAAACGTTTA 240
ACTTTATTTA ANANGTATAA TGTGAAAACA TAAAATATAT TANNCCTTTN CTATTGTTTT 300
CTTTCCCTTT CACAGTAACT TTATGTNAAA TAACCATCTT TCAAAAGGGC TANAANNNAN 360
ANANNTNN 368

SEQ ID NO:4498
SEQUENCE LENGTH:150
SEQUENCE TYPE:nucleic acid
TOPOLOGY:linear
CLONE:HUMGS05341
SEQUENCE DESCRIPTION:
GATCACATCA CCTCTCTGCC TCAGTTTCCC CATCTGTAAA ATGGGAGAAT AATACTTGCC 60
TACCTACCTC ACAGGGGTGT TGTGAGGATT CATTTGTAAT TTTTTTTTTT TTTTTTAANA 120
GNGTTTTAAN GNNTAAAAAN CAGTNAAATN 150

SEQ ID NO:4499
SEQUENCE LENGTH:109
SEQUENCE TYPE:nucleic acid
TOPOLOGY:linear
CLONE:HUMGS05342
SEQUENCE DESCRIPTION:
GATCTCCATC ATTTCTTACA GGTTTTCCTC CCTCAAGCAA TTCGACATCT CAAGTGAAGA 60
CATGGCCCCT GAAGGGCAAT AAAGCTGCTA GTTTATTAAT ACAGTCAAA 109

SEQ ID NO:4500
SEQUENCE LENGTH:315
SEQUENCE TYPE:nucleic acid
TOPOLOGY:linear
CLONE:HUMGS05343
SEQUENCE DESCRIPTION:
GATCTACAGG NCAAAGGGAG GGAGCAAGCC CTACTCGGAT GGGGCACGGA CTGTCCACCT 60
TTNCTAATGT GTGTTGTCAG CCTGTGCTGT GGCATAGACA TGGATGNGNG GACCACTTTG 120
GAGACTGGGG TGGCCTCAAG AGCACACAGA GAAGGGAAGA TGGGGCCATC ACAGGATGCC 180
AGCCCCTGCC TGGNTTGGGG GCACTCAGCC ACGACCAGCC CCTTCCTGGG TATTTATTCT 240
CTATTTATTG GGNNNNGNTG AAGAGGCATC CTGCCTGGNT GGGNCAGCCC CTTCAGCCCC 300
TTNTNCCCTN CCCGN 315

SEQ ID NO:4501
SEQUENCE LENGTH:157
SEQUENCE TYPE:nucleic acid
TOPOLOGY:linear
CLONE:HUMGS05344
SEQUENCE DESCRIPTION:
GATCAGACTG TTGCTTTCGC ATGNTGTATG TAGTGTCTCA TGNCTGGAGT TTGCTTTGTT 60
TTATAGTATC TGTACTCCTT GTATTTTTCA AGAGCTATTT TGTAAACAGA TGATGTATTT 120
CTCCATTGAA AACACAATAA AAAAAAANCA GCACAAA 157

SEQ ID NO:4502
SEQUENCE LENGTH:397
SEQUENCE TYPE:nucleic acid
TOPOLOGY:linear
CLONE:HUMGS05345
SEQUENCE DESCRIPTION:
GATCTAGTCT TTACTTTCAG TTGTNTGTTA GGTCCATTCT GTTTACTAGA CGGATGTTAA 60
TAAAAACTAT GCGAGCCTGA NTGANTTCTC AGCCAAATTT AGTCTTGTNT CTCATCTTGA 120
TTGGNTTAAT TCCAAATTCT AAAATGATTC ANTNCACAAT AGCTCTAGGG GATGANGAAT 180
TTGCCTTACT TTGCCCAGTT CCTAAGACTG TGAGTTGTCA AATCCCTAGA CTGTAAGCNC 240
TTCAAGGAGC AAGAGGCGCA TTTNCTCCGT GTCATGTAAT TNNNCTAAGG TGCTTGGCAG 300
CACTCTGTAC CCTGTGGAGT ACTCAGTACC TNTGGTTTGA NGTTGCTGAC AAGACCTGAA 360
AAAAATCCCT TAAAAANNAA ACCCTTTAAG GTGTGGN 397

SEQ ID NO:4503
SEQUENCE LENGTH:84
SEQUENCE TYPE:nucleic acid
TOPOLOGY:linear
CLONE:HUMGS05346
SEQUENCE DESCRIPTION:
GATCAGACTT GTTAAGTATG CCGCAAGTAA GAGCTAATTC ATTCATTCCA TGTNTGCCAC 60

TAAATAAAGA GATTGAGCAA GAAA 84

SEQ ID NO:4504
SEQUENCE LENGTH:316
SEQUENCE TYPE:nucleic acid
TOPOLOGY:linear
CLONE:HUMGS05347
SEQUENCE DESCRIPTION:
GATCTNAACC CCAGACATCA AGGATAACAT CGTTGCTCAG CTGAAGCAGC TGTACCGCAT 60
CCTTNAAACC CAGGAGAGCT GGCAGCCCAT GCAGCCCACC CCCAGCATGC AACTGCCCCC 120
TGCCCTGCCT CCCCAGTAAT TGTNAATGCN ATCTTCTTCC TTCTNTTTTT TATAATATTG 180
TACATATGGA TTTTTTNAGN GNTTAGATTT AACCAGCTTT TAAATCTCTC TTTNNTCTAA 240
CAGTGTTAGA AGTTTGTNAT TCTNCAAATA TGCCTAGATT TAAAGCTGNT TTGAATTTAT 300
GGNAAAAAAA AANAAN 316

SEQ ID NO:4505
SEQUENCE LENGTH:218
SEQUENCE TYPE:nucleic acid
TOPOLOGY:linear
CLONE:HUMGS05349
SEQUENCE DESCRIPTION:
GATCAAAGTG TTAGGGGAAA AAAATTCCAA AAGTAGTTAC AAGCTATATC AATGTCAAAG 60
TAAATNCACT TCATCAAAGC TAAGAAGTCA CAGGAATTGT NCTCAGGTTT TTAAAAAAAT 120
TTTNCCTGAA TTCAGGAAGT GTCTTCTGAA TAGCAGCTAG CCANNTAAAG CGGTGTGTGT 180
GTACTGCAGC TGTAGGTGAA CTTAAAAATA ATAATAAA 218

SEQ ID NO:4506
SEQUENCE LENGTH:297
SEQUENCE TYPE:nucleic acid
TOPOLOGY:linear
CLONE:HUMGS05350
SEQUENCE DESCRIPTION:
GATCAGCCAG TTACCTTCTC CATTGTNTTA GTTCTGTCAC CCATTTCGTC AAGTGACCTC 60
TCATCTTCTA TAAACTAATA CAGGAATTCT TTCCAAAGCA ATGTCTAAAA ACTCTTTTTT 120
TTAAAGTAAC AGTTTGGTAT GTTTATTGTA GATAAATNAT TTTTNAGGCC TTCATTTTAG 180
CTAAGTTTAG AATTNATATT AGGCANCTAT GATTTGAGTG GTTATNCATT GAGTAANTTT 240
CCACTATAAA GAATTTNATT GANCANTTGT TAAAGNATNN TGGTAATGCA TGGNCAN 297

SEQ ID NO:4507
SEQUENCE LENGTH:322
SEQUENCE TYPE:nucleic acid
TOPOLOGY:linear
CLONE:HUMGS05351
SEQUENCE DESCRIPTION:
GATCAGAGCA CTGAGAAACT CCAGAGGCTT CTGGTTCACC GCCCTGATTC CACAGATGTG 60

GGTCAGAGGC AGAGATGGAA GTTGGAATGG ACAAGCCTGC CCTCTCCTCA GCTCTCCATG 120
GCTCTGTCCT CAGGCTGTTT CTGCAATTTG ACATGAGGCC TGGCCCCAGG GAAACCAGTG 180
GGGCCTCATC TCCTCCCTGT GCTGCTCTGA GGTGGAGCCT TCTNAGGGTC AGTCCAGCTC 240
TGGAGNCCCT CTCCAGCTCG GTCACCAAAG CTGTCTGGTC CCAGGGCGGT GTGGAGTGAC 300
AGGCACGGCA GCCCAATNCN GN 322

SEQ ID NO:4508
SEQUENCE LENGTH:137
SEQUENCE TYPE:nucleic acid
TOPOLOGY:linear
CLONE:HUMGS05352
SEQUENCE DESCRIPTION:
GATCGCGCCA TTGCACTCCA NCTTGGGTGA CAGAGTGAGN CTCTGTCTCA AAAAATAATA 60
ATAATNAATT AATTAATTAA TTAAAAAATA AACAGTGGGT TAGTTATCTC AAAAGTGGGC 120
TTGTTATAAA ANCNNGN 137

SEQ ID NO:4509
SEQUENCE LENGTH:285
SEQUENCE TYPE:nucleic acid
TOPOLOGY:linear
CLONE:HUMGS05353
SEQUENCE DESCRIPTION:
GATCATAAGA CTGTCATGTA AGAGGTGCTC TCCTGGCACC CAGAGAAAAG GAGCATCCTT 60
ACCTCCAAAA GCACAGGGAC ACAAAGAGGA ATCTAAACAA ACAGGCCTCT NAGTTTCCCC 120
CAGTTTATTA CATTTAGCTT GTTCACACTT TGCCCTATGA CATTNCTACA TCACTGGNTG 180
CTCTTCATCA AACCTACTAT AANNNNCATT CAAGTTCAAC TGTTTCTTTG GGCCTTNNTT 240
NNCTTATGGA GGCCCTCGTG TCGTGTAAAA CTTNTATTNG GTNAN 285

SEQ ID NO:4510
SEQUENCE LENGTH:327
SEQUENCE TYPE:nucleic acid
TOPOLOGY:linear
CLONE:HUMGS05354
SEQUENCE DESCRIPTION:
GATCAGCAGA GTGGAGGCTG AGGATGTTGG GATTTATTAC TGCATGCAAG CTCTACAAGT 60
CCCGGTCACC TTCGGCCAAG GGACACGACT GGAGATTAAA CGAACTGTGG CTGCACCATC 120
TGTNTTCATC TTGCCGCCAT CTGATGAGCA GTTGAAATCT GGAACTGCCT CTNTTGTGTG 180
CCTGCTGAAT AACTTCTATC CCAGNGAGGC CAAAGTACAG TGGAAGGTGG ATAACGCCCT 240
CCAATCGGGT AACTCCCAGG AGAGTGTCAC AGAGCAGGAC AGCAAGGACA GCACCTACAG 300
CCTCAGCAGC ACCCTGACGC TGAGCNN 327

SEQ ID NO:4511
SEQUENCE LENGTH:194
SEQUENCE TYPE:nucleic acid
TOPOLOGY:linear

CLONE:HUMGS05355
SEQUENCE DESCRIPTION:
GATCCTCCAC TCCAGAGCAC CTGAGAAGGC CGGGACCAGA GGCCCTGTGT GATGTGTACT 60
CCGCAGCTGT TTGGGGTGGG ACATTTCTGT ACTTCTCGAT TTGCTTATGG CTCAGCCATT 120
ACCTGTGTCA GTCCATGATT CTGTTGTAAC AGTTTTAAGA GTAAATAAAT AAAGCTGCCT 180
GATGTCCCAT CAAA 194

SEQ ID NO:4512
SEQUENCE LENGTH:106
SEQUENCE TYPE:nucleic acid
TOPOLOGY:linear
CLONE:HUMGS05356
SEQUENCE DESCRIPTION:
GATCACTTTT CCCTGTCTAA ACTCCAGGNT ACAGTATCCA ATANATCCAA ACAGAACTCT 60
GGTGTCAAAG TGTAANTAAT NGTGTAAAAT AGCCTTCCCA AGNTTN 106

SEQ ID NO:4513
SEQUENCE LENGTH:89
SEQUENCE TYPE:nucleic acid
TOPOLOGY:linear
CLONE:HUMGS05357
SEQUENCE DESCRIPTION:
GATCTCACCA CTGTACTCCA GCCTGGNTGA CAGAGCGAGA CCCAGACTCA AAAAATAAAA 60
ATAAAAACCC TGAATATCTT CCTTCTAAA 89

SEQ ID NO:4514
SEQUENCE LENGTH:366
SEQUENCE TYPE:nucleic acid
TOPOLOGY:linear
CLONE:HUMGS05359
SEQUENCE DESCRIPTION:
GATCTCTATA CCTCAGCTTC TTCATCTGCA AAGTGAGATA ATTGTACTAT TCTACTTTCC 60
TCATAGTGCN NTTATAACAG ATTATGATAA TATTACAAAG TACTTAATAC TTGGCATATA 120
GTATATATAT NANCTATTAG TATTTTGCAA TTTGGGGATA TTGAAAATTA GAAATTGCNT 180
GTTAGTTCAT GCTGCACTAC CATATTGTCC TTTTGGGGCT CTTCAATGGG TGAAATTCCA 240
TTTNACAGTT TATCATGTGC ATCTGTATCA CAGGNGTATT TTGGCAAAAT GATTCTGTNC 300
CAAAAAGGAG TACTATANNN NGNGGGTGCC AAATATATTG AGTCTNTTAT GGGGAAAANA 360
AATTTN 366

SEQ ID NO:4515
SEQUENCE LENGTH:313
SEQUENCE TYPE:nucleic acid
TOPOLOGY:linear
CLONE:HUMGS05360
SEQUENCE DESCRIPTION:

GATCTCTGGT AAAAAGCAAC ATCTGAGAAA ATTNCTTGTT TTTAGGAACC TGTGTACATT 60
CATACTTAAT TCTNCCAATN ATGCTTTTCA GTTTTTTGGA TTAACACTCA AAAGTAACAA 120
GTCCTCAAAA TTAGGTTTGT CCTGTTGTTT TAAGTGGACC TTTGGCAAGA GATTTCTAGT 180
GAAGCTTGAT TCTNAGTACT TTCCTCATGT ATTAAAAAGA GGCTGGAGGA GGGAACCTGG 240
GACGTGGAGA GTGAGGAGTG ATAGCCTGAG GTTCTTGGTT TGCCAACCTG GGGAAACCCT 300
GTCTCTACTT AAA 313

SEQ ID NO:4516
SEQUENCE LENGTH:73
SEQUENCE TYPE:nucleic acid
TOPOLOGY:linear
CLONE:HUMGS05361
SEQUENCE DESCRIPTION:
GATCACTAAA CTATCTCCCC TCTTGCTGAA GTTCTTTGTA GTAATAGCTC ATAAAAATTT 60
GTTTATTAAT AAA 73

SEQ ID NO:4517
SEQUENCE LENGTH:247
SEQUENCE TYPE:nucleic acid
TOPOLOGY:linear
CLONE:HUMGS05362
SEQUENCE DESCRIPTION:
GATCTTTTAA AAAGAGATTA AACCGAAGTG ANTTAAAAGA CCTTGAAATC CATGACGCAG 60
GGAGAATTGC GTCATTTAAA GCCTAGTTAA CGCATTTNCT AAACGCAGAC GAAAATGGAA 120
AGATTAATNN GGAGTGGTAG GNTGAAACAN TTTGGAGAAG NTAGAAGTTT GANGTGGAAA 180
ACTGGANGAC AGAAGTACGG GAAGGCGAAG AAAAGANTAG AGAAGATAGG GAAATTNGAA 240
GNTAAAN 247

SEQ ID NO:4518
SEQUENCE LENGTH:95
SEQUENCE TYPE:nucleic acid
TOPOLOGY:linear
CLONE:HUMGS05363
SEQUENCE DESCRIPTION:
GATCGCAGAA CATACTGTAT TTTNACTATA TCTTTTTTTT TAAATCTGAC AAACTTTTTT 60
NCTTTNATTT NAATTGACAT AATTGTACAT ATAAA 95

SEQ ID NO:4519
SEQUENCE LENGTH:134
SEQUENCE TYPE:nucleic acid
TOPOLOGY:linear
CLONE:HUMGS05364
SEQUENCE DESCRIPTION:
GATCTGCTTT GGAAATTAAT TGCCTCTAAT ACATACCTAA GTAAACATAA CATTAATACC 60
TAAGTAAACA TAACATTACT TGGAGGGTTG CANTNTCTAA GTGAAACTGT ATTTGANACT 120

NTTNAGTNTA CTTN 134

SEQ ID NO:4520
SEQUENCE LENGTH:269
SEQUENCE TYPE:nucleic acid
TOPOLOGY:linear
CLONE:HUMGS05365
SEQUENCE DESCRIPTION:
GATCTGGCAC TGAGCTTTTT GTCACTGAAC CTTCCCGATG CCAGCTGGGC TCTTGGACTC 60
CCCTCTTCCT CAAGGGTAGA TGAGAGGAAC GATNNNTAGA GGTTGGCTGT GGGTCCTGGG 120
TACCACCTTC TGAGCCTCAG TTTCCTCATC TGTAAAGTGG GGAGAAAAGT CTGTTTGCCT 180
CAGGAGTGTG AGGACTACAC TAGTGAAAGC NCCTGGCGGG CAGCCGGCGA TGCCCAATAA 240
ATGTGTGNTT TGCTGTTTGT TAAGTGAAA 269

SEQ ID NO:4521
SEQUENCE LENGTH:189
SEQUENCE TYPE:nucleic acid
TOPOLOGY:linear
CLONE:HUMGS05366
SEQUENCE DESCRIPTION:
GATCAGTTTG TAATTTTTTT NCTCCTTGAA GGCACAAAAT AATTGGTTTN AGGTATGTAA 60
AACACTAGAG GTCAACCTTA CATAGTATAT AGAACTNATG GGTTTACCCA GCTACCCAGT 120
AGCATAACTT TNCACAGCTC GGGGATGAAT TAACATGGCT GAAATAAAAC TAAAAGTATG 180
GTTTTNAAA 189

SEQ ID NO:4522
SEQUENCE LENGTH:79
SEQUENCE TYPE:nucleic acid
TOPOLOGY:linear
CLONE:HUMGS05367
SEQUENCE DESCRIPTION:
GATCACAGTA CACCATATGC ACTCTGGTAC CTTAATTTTT TTTTATAAAT AATAAAAGTG 60
AATATTGAAG CTTCTTAAA 79

SEQ ID NO:4523
SEQUENCE LENGTH:255
SEQUENCE TYPE:nucleic acid
TOPOLOGY:linear
CLONE:HUMGS05368
SEQUENCE DESCRIPTION:
GATCTGTTGA ATTTGGGAAG CCTCCAGTTT TAATAAAATT TTTTTATTAT GGTGGAAACN 60
TGTCTGTAAA NCTTAGGACT ATAGAAATGC AAAGAATAAA GANCTGAGGG AGTTACACAC 120
ATTTGTAGTC AGAAATTAAG TTCAAAANAT AATTTAGGTC ATTTGCATTT TTTAGTATGC 180
AAAATAAATG GAAAANTTAA NGTGCAATAN ANNTATAATT NGGGAAATGA ATGCAGTAAA 240
NTTGANAACA TTGAN 255

SEQ ID NO:4524
SEQUENCE LENGTH:51
SEQUENCE TYPE:nucleic acid
TOPOLOGY:linear
CLONE:HUMGS05370
SEQUENCE DESCRIPTION:
GATCTGTCAG GGCCACTTAG TGATAATAAA TTCTTCCCAA CTGCAGACAA A 51

SEQ ID NO:4525
SEQUENCE LENGTH:353
SEQUENCE TYPE:nucleic acid
TOPOLOGY:linear
CLONE:HUMGS05371
SEQUENCE DESCRIPTION:
GATCCGCATC GACTTGGCCG TGGGCGACGT GGTCAAGACC TGGCGTTTCA GCAACATGCG 60
CCAGTGGAAT GTCAACTGGG ACATCCGGCA GGTGGCCATC GAGTTTGATG AACACATCAA 120
TGTGGCCTTC AGCTGCGTGT NTGCCAGCTG CCGAATTGTA CACGAGTATA TCGGGGGCTA 180
CATTTTCCTG TCGACGCGGG AGNGGTCCCG TGGGGAGGAG CTGGATGAAG ACCTCTTCCN 240
GCAGCTCACC GGGGGNCATG AGGNCTTCTG AGGGCTGTTT GATTGCCCCT GCCNTGCTNA 300
ACAACCTGTN ACAGGCACTT CCAAGGCCAG AACCACAGGG GCTNACTNTC GAN 353

SEQ ID NO:4526
SEQUENCE LENGTH:174
SEQUENCE TYPE:nucleic acid
TOPOLOGY:linear
CLONE:HUMGS05372
SEQUENCE DESCRIPTION:
GATCTGGAAT AATTATGGTA AAATAATAGT ATTTGCTAAG ACTAGATGAT TGGTGTACTG 60
GATTTCATCC GCTATTTTCT AAGTTTGTTA TGAACGCTTA AAATATATAT GTATGTAGTA 120
AAATTAATGT AAATTTGTAC AAATAAAAAT AAATGGCATG TGATATATGG CAAA 174

SEQ ID NO:4527
SEQUENCE LENGTH:201
SEQUENCE TYPE:nucleic acid
TOPOLOGY:linear
CLONE:HUMGS05373
SEQUENCE DESCRIPTION:
GATCATGTGT TTGGGGGCTT TTAGGGGACC CAGCTGCACT GGGGCACTGC CCGTGGCCTG 60
GGTAGGACAT TTCCCAGCAA GGGCTGGAGG AGTTGCCGTG CCTTCAGCCT GAATCGAATG 120
TCAGAACCAG CCAGCGGTGC TTCACCCTCT TGGGGATAAC TTGCTTAGTT TTTTAATAAA 180
TGTTCCTGGT TGGTTTTCAA A 201

SEQ ID NO:4528
SEQUENCE LENGTH:308

SEQUENCE TYPE:nucleic acid
TOPOLOGY:linear
CLONE:HUMGS05374
SEQUENCE DESCRIPTION:
GATCTATGAA GCCCACCAAC AAGGCCGAGG GGCTCTGGAG GCATTGCTAT GTGGGGGACC 60
CCAGGGGGCC TGCTCAGAGA AGGTGTCAGC CACAAGAGAA GAGCTCTAGT CCTGGACTCT 120
ACCCTCCTCT GAAAGAAGCT GGGGCTTGCT CTGACGGTCT CCACTCCCGT CTGCAGGCAG 180
CCAGGAGGGC AGGAAGCCCT TGCTCTGTGC TGCCATCCNG NNNGGNTCCT CCAGCCTCAG 240
GGCACTCGGG CCTGGGTGGG AGTCAACGNC TTCCCCTCTG GACTCAAATA AAACCCAGTG 300
ACCTCAAA 308

SEQ ID NO:4529
SEQUENCE LENGTH:194
SEQUENCE TYPE:nucleic acid
TOPOLOGY:linear
CLONE:HUMGS05375
SEQUENCE DESCRIPTION:
GATCTTCTGA GCTTGTTTCC CTGCTGGGTG GGACAGAGGN CAAAGGAGAA GGGAGGGTCT 60
AGAAGAGGCA GCCCTNCNTT GTCCTCTGGG GTAAATNAGC TTGACCTAGA GTAAATGGAG 120
AGACCAAAAG CCTCTNATTN TNAATTTCCA TAAAATGTTA GAAGTATATA TATACATATA 180
TATATNCCTN TAAN 194

SEQ ID NO:4530
SEQUENCE LENGTH:314
SEQUENCE TYPE:nucleic acid
TOPOLOGY:linear
CLONE:HUMGS05376
SEQUENCE DESCRIPTION:
GATCTTGATT GATAACTGCC ATGATATTTT GCTTTGATGT GTTTCTACAT GTGGTTGCAC 60
ACGGTTCAGT AAAAATAATG CTGCTATCGA GTATGCAAAT ATTGAAGTAT GATGGTTTGA 120
CTGTATGGNA GTGTTGTAGC AGCCTCTTGT TTTTTTCCCC ATTGCCTCTT TTTTTAAAAA 180
ACTTATAAAG TCACTNTTNA TTTTNCCTCA GTCTTCAATG ACGAGAGCAA TATTAAGANG 240
ACATTGCTAT CTAATNNTNA ATCTTNTTAA ATGAAAAATT CCTATGTTCA GTAGCGTGGT 300
TGATGCTATT GTGN 314

SEQ ID NO:4531
SEQUENCE LENGTH:182
SEQUENCE TYPE:nucleic acid
TOPOLOGY:linear
CLONE:HUMGS05377
SEQUENCE DESCRIPTION:
GATCGAGTCA GCATAAATTT CTAAGTCAGC CTCTAGTCGT GGTTCATCTC TTTCACCTGC 60
ATTTTATTTG GTGTTTGTNT GAAGAAAGGA AAGAGGAAAG CAAATACGAA TTGTACTATT 120
TGTACCAAAT CTTTGGGATT CATTGGCAAA TAATTTCAGT GTGGTGTATT ATTAAATAGA 180
AA 182

SEQ ID NO:4532
SEQUENCE LENGTH:314
SEQUENCE TYPE:nucleic acid
TOPOLOGY:linear
CLONE:HUMGS05378
SEQUENCE DESCRIPTION:
GATCATGAAA CTGGTTTCTG TTTTATGCAG TTGTCATTTG TAAAGNCTAA TAAAATATNC 60
TCTATAATTN CTTCTAGATT ACAAAAATAT GACAATCTTG TAAGTAGCAG ACTATGGAGA 120
AAAATGAGTT ACCTGGNGGG TCAGGTAACT TGCCAAACTN AAANGTNTGT TAGTTGAGGC 180
AAAGTCCTAA GCAAGGTTGT GCTATCANGG CTCAGCATAC CTTCGTGGGC CTTTGANTTA 240
CCANCACTGG AANTGCCTGC CANCTAATCT GGGGTAGATT CTTTAAGGCA TTCCACTTAG 300
CTTGCCAGTT GNGN 314

SEQ ID NO:4533
SEQUENCE LENGTH:311
SEQUENCE TYPE:nucleic acid
TOPOLOGY:linear
CLONE:HUMGS05379
SEQUENCE DESCRIPTION:
GATCAACTAA TAGCTCTGCT AGTAATTGAT TTATTTTNCT TCAATAAAGT TGCATAAACC 60
AATGACTTAG CTGCCTGGAT TAATCAGTAT GGGAAACAAT CTTTTGTAAA TGCAAAGCTG 120
TTTTTNGTAT ATACTGTTGG GATTTGCTTC ATTGTTTGAC ATCAAATGAT GATGTAAAGT 180
TCGAAAGAGT GAATATTTTG CCATGTTCAG TTAAAGTGCA CAGTCTGTTA CAGGTTGACA 240
CATTGCTTGN CCTGATTTAT GCAGAATTAA TANGCTATTT GGATAGTGTA GCTTTAATGT 300
GCTGCACATG N 311

SEQ ID NO:4534
SEQUENCE LENGTH:330
SEQUENCE TYPE:nucleic acid
TOPOLOGY:linear
CLONE:HUMGS05381
SEQUENCE DESCRIPTION:
GATCGGAGGA CGACCCGAGT CCCAAGAGTG GGGTTTTGCT TTTTAAAAGG AGAGAGGAGG 60
GGTGATGGCA GGGGAGTGGA GGGTGGCCGG GCAGGTCCTG CCGGCGCAGG GAGCCCTCTG 120
CCCTTCACAC TCTCCTCCAA AAGAGCCTCC ATCTGTAAGG AAGCAGGTCT CCGCGAGGGG 180
TTTCTTTCCA TGTNTTTTCC TCCTGTNGTT TTAGAACTTT TTNGAAAAAA ACNGNCCTCG 240
TTTTAGATTT ATAGCATTGA CTTTTACACA CATTCACACA AGANAAAAAT CCTTNCAAAA 300
TTCTTAAANT NNNCTGGTNC CNNCTTTNNN 330

SEQ ID NO:4535
SEQUENCE LENGTH:204
SEQUENCE TYPE:nucleic acid
TOPOLOGY:linear
CLONE:HUMGS05382

SEQUENCE DESCRIPTION:
GATCTCAGCT CCCGTTCCCC AAGCACACTC CTAGCTGCTC CAGTCTCAGC CTGGGCAGCT 60
TCCCCCTGCC TTTTGCACGT TTGCATCCCC AGCATTTCCT GAGTTATAAG GCCACAGGAG 120
TGGATAGCTG TTTTCACCTA AAGGAAAAGC CCACCCGAAT CTTGTAGAAA TATTCAAACT 180
ANTAAAATCA TGAATATTTT TAAA 204

SEQ ID NO:4536
SEQUENCE LENGTH:136
SEQUENCE TYPE:nucleic acid
TOPOLOGY:linear
CLONE:HUMGS05383
SEQUENCE DESCRIPTION:
GATCACTTGG AGACTCTTTT TTTGCTGGTT TCTAGATAAC TCAGGTAAAT CAGACCTTTA 60
CAGAGTACAG GGCTAGGTGA NAGANTTACT GANAAATCAC CTTGAAAATC CGANGGGCTG 120
ATATACCCTT NNTGTN 136

SEQ ID NO:4537
SEQUENCE LENGTH:87
SEQUENCE TYPE:nucleic acid
TOPOLOGY:linear
CLONE:HUMGS05384
SEQUENCE DESCRIPTION:
GATCTATGCT GTGTTTGCTT ATTCTTTAGT TGAACACACT ATGAAGAATT CCAGGTGTAC 60
TAGTGAATGT AATTTATAGT TGCCAAA 87

SEQ ID NO:4538
SEQUENCE LENGTH:305
SEQUENCE TYPE:nucleic acid
TOPOLOGY:linear
CLONE:HUMGS05387
SEQUENCE DESCRIPTION:
GATCGACAAC GAGGCCCTCT TCAGCTGCGA GGTCAAGCAC CCAGCTCTGT CGATGCCCAT 60
GCAGGCAGAG GTCACGNTGG TTGCCCCCAA AGGACCCAAA ATTGTNATGA CGNCCAGCAG 120
AGCCCGGGTA GGGGACACAG TGAGGATTCT GGTCCATGGG TTTCAGAACG AAGTCTTCCC 180
GGAGCCCATG TTCACGTGGA CGCGNGTTNT GAGCCCGGNN NNNGGACGGN AGCGCTGAGT 240
TCGACGGGAA GGANCTTGGT GCTGGAGCGG TTTCCCGNCG AGNTCAATTG GCTCCATGTA 300
TTNGN 305

SEQ ID NO:4539
SEQUENCE LENGTH:106
SEQUENCE TYPE:nucleic acid
TOPOLOGY:linear
CLONE:HUMGS05388
SEQUENCE DESCRIPTION:
GATCATAAAA TGAATTCTTT ATTGTATCTA CACACTCCAC ATTCTTTACT GTGTCCTACT 60

ACTGTATCTT GGCTCCANTG CTGTATTAAA CACCATCTTA AGCAAA 106

SEQ ID NO:4540
SEQUENCE LENGTH:167
SEQUENCE TYPE:nucleic acid
TOPOLOGY:linear
CLONE:HUMGS05389
SEQUENCE DESCRIPTION:
GATCCTCCTT TGCATGCAAC TGTAGCTGCA TTTAATGAAT AGTTTGAACC CTTGTNAATN 60
CATTTTTTNA AAAAGAAAGA AAAAAAAAAC TTCGTGTATG NAACTCAAAG CATGNAACCT 120
TAAGATGTTG CATTCTAAAC TNACANTAAA GACCTTTCCC AANTAAA 167

SEQ ID NO:4541
SEQUENCE LENGTH:111
SEQUENCE TYPE:nucleic acid
TOPOLOGY:linear
CLONE:HUMGS05390
SEQUENCE DESCRIPTION:
GATCCATACG TGGCCGCACC CACACAGCAC GTGCTGTGAC NGATGGCTGA ACGGAAAGTN 60
TACACTGTTC CTGAATATTG AAATAAAACA ATAAACTTTT AATGGTANAA A 111

SEQ ID NO:4542
SEQUENCE LENGTH:216
SEQUENCE TYPE:nucleic acid
TOPOLOGY:linear
CLONE:HUMGS05391
SEQUENCE DESCRIPTION:
GATCAGTTCT GCAAGATGCC CATCAACAAC AAGATGGCCC AGTGGGGCAA CAAGCCTATA 60
TGCTGAGCAG GAGGCAGACT TGCAGAGCTT GTTTTGTTTC ATCCTGTCCG TAAGGGGTCA 120
GCGCTCTTGC TTTGCTCTTT TCAATGAATA GCACTTATGT TACTGGTGTC CAGCTGAGTT 180
TCTCTTGGGT ATAAAGGCTA AAAGGGAAAA AGGAAA 216

SEQ ID NO:4543
SEQUENCE LENGTH:103
SEQUENCE TYPE:nucleic acid
TOPOLOGY:linear
CLONE:HUMGS05392
SEQUENCE DESCRIPTION:
GATCGAAAAT AGTTGGAAAA AAGAAGAAGA GTTGCATCTC TACTGAACAT GTACAGACTT 60
TCTTCTTCTT ATTATTCCCT AAACAATACA GTGTAAAACT AAA 103

SEQ ID NO:4544
SEQUENCE LENGTH:194
SEQUENCE TYPE:nucleic acid
TOPOLOGY:linear

CLONE:HUMGS05393
SEQUENCE DESCRIPTION:
GATCAGAACA NTNCAAAAAA TTCACACCCC CAAAAAAGNC AAGCTATTAT ATGGCAATTT 60
CGTGATAAAA TATTCATCCT ATTTGTCCTG CATTTCTAAT TTTCCCGTAA CTAGACACAT 120
CAGTTTTATA ATTAGGGNAA AAATACCTTN TAAAAGTAAA GCGAGCANAG GTTTCNNTTG 180
CANTGTTCAT TCCN 194

SEQ ID NO:4545
SEQUENCE LENGTH:108
SEQUENCE TYPE:nucleic acid
TOPOLOGY:linear
CLONE:HUMGS05394
SEQUENCE DESCRIPTION:
GATCTGGTAG GTTGCAGCAG AGAAAATAAA TGTGACTTGN GAGNCCACTC AGAGAGGGTC 60
CAAGGGTGAT GGAGAAGGAA GCATGGCCTG GNAGCTTGGA AGGNANGN 108

SEQ ID NO:4546
SEQUENCE LENGTH:266
SEQUENCE TYPE:nucleic acid
TOPOLOGY:linear
CLONE:HUMGS05395
SEQUENCE DESCRIPTION:
GATCTGCTCT TGCAATGGCC TCACCATCTG GNAGGAGCGA GGCCGGCCCA TCGTTGGCAA 60
GGGCCGAAAG GAGTCAGCGC ANCCCCTGCC CACCTTTNAG CAGGACCTCA CCTTGGCTCT 120
GTTGCTGTCC TCCAGGGCGA GCACTTTCCA CTTCCAGAGG GGGCCAGAGG GACTTTGCCT 180
GCCCAGTCTT TGGAGAGCTC AGTACAGGGC AGGAGCTGCT GTGGTGTTCC CTTGGCAAAT 240
NAAAGTTTTA TTTTCGTTTG GGNAAA 266

SEQ ID NO:4547
SEQUENCE LENGTH:72
SEQUENCE TYPE:nucleic acid
TOPOLOGY:linear
CLONE:HUMGS05396
SEQUENCE DESCRIPTION:
GATCTCCGTG TGTGCATGTN ACTGTGCTGG GTTGGAATGT GAACAATAAA GAGGAATGTC 60
CAAGTGTTCA AA 72

SEQ ID NO:4548
SEQUENCE LENGTH:104
SEQUENCE TYPE:nucleic acid
TOPOLOGY:linear
CLONE:HUMGS05398
SEQUENCE DESCRIPTION:
GATCATGGGG GTGGATTTCT TATGAATGGT TTAGTACCAT CCACTTTGTA TTGTCCCCGT 60
GGTAATGAGT GAGTTCTTAA GAAATCTGGT CATTGAAAAA TAAA 104

SEQ ID NO:4549
SEQUENCE LENGTH:74
SEQUENCE TYPE:nucleic acid
TOPOLOGY:linear
CLONE:HUMGS05399
SEQUENCE DESCRIPTION:
GATCTTCACA TCTTGGACCT TNGTCAGTTT TGCTATTCAT TATTAAACAC TAAAACTTTG 60
GCGGTTNTTG CAAA 74

SEQ ID NO:4550
SEQUENCE LENGTH:292
SEQUENCE TYPE:nucleic acid
TOPOLOGY:linear
CLONE:HUMGS05400
SEQUENCE DESCRIPTION:
GATCTGAAAT CACATCCAGC TCCTGATGTT TTCTTCTCCC TCTGACTGCA GAGGAAGTGT 60
TCCTACCTGC AGGAAGGCAC CTGTCACACA GGGCGTTCAC TCAGACCATC TGTGCTCTGC 120
CCTGAGTTCA GTTGAGAAAA TCCTATTATC AAATTTGGNT TTCCTGGCCC CAGAACTTCC 180
CAAAGACCTG TAAAATGGAG GGATTTACCA CCTCACATAT GTCCAGTTAA ACAGTTTGTG 240
GACTTGTAAC CGTCGCAGCC CANTGGTACA ACAGTAGTTT AATCACGGGA AA 292

SEQ ID NO:4551
SEQUENCE LENGTH:265
SEQUENCE TYPE:nucleic acid
TOPOLOGY:linear
CLONE:HUMGS05401
SEQUENCE DESCRIPTION:
GATCGGATGG GCAAGTCCCT GCCAGGGTCT CCAGATGGCA ATGGAAGCTC CTCCTTGCCC 60
CACTGGGAGT AGCGGCTAAA GCTGGGGGAT AGAGGGGCTG CAGGGCCACT GGAAGGAACA 120
TGGAGCTGTC ATCACTCAAC AAAAAACCGA GGCCCTCAAT CCACCTTCAG GGCCCGGCCC 180
ATGGGCCCCT CACCGCTGGT TGGAAAGAGT GTTGGGTNGT TGGCTGGGGG TGTCAATAAA 240
GCTGTGCTTG GGGGTCGCTN GGAAA 265

SEQ ID NO:4552
SEQUENCE LENGTH:173
SEQUENCE TYPE:nucleic acid
TOPOLOGY:linear
CLONE:HUMGS05402
SEQUENCE DESCRIPTION:
GATCTATGCT CTTTCAAATG GAGACTGGGA TATTGTTGTT TTAAGACCAC TGTTTTCCTG 60
TTTCCTCCCA AATNTTAGTC ACATATATNA TTACCCTTAA GTTGGTCACT ATCTATCTAT 120
CTGTCAATAA TTCAGGATTA AAGTTTGAAG TGTAATTACA TTTGANTTTT AAA 173

SEQ ID NO:4553

SEQUENCE LENGTH:270
SEQUENCE TYPE:nucleic acid
TOPOLOGY:linear
CLONE:HUMGS05404
SEQUENCE DESCRIPTION:
GATCGACCAG CAGGTGGTCT ATATCCATCC TTCCAGTGCC CTCTTCAACA GACAGCCAGA 60
ATGGGTNGTG TACCATGAGC TNGTGCTCAC CACCAAGGAA TACATGCGTG AAGTTACCAC 120
CATCGACCCT CGGTGGCTTG TGGAGTTTGC CCCAGCCTTN TTNAAGGTCT CAGACCCANC 180
TAAGCTAAGN AANCAGAAGA AGCANCAGCG TCTTGGNACC GTTGTACAAC CGNTATGAGG 240
ANCCCAATGN CTGGGAGAAT ATCTCGGNGN 270

SEQ ID NO:4554
SEQUENCE LENGTH:333
SEQUENCE TYPE:nucleic acid
TOPOLOGY:linear
CLONE:HUMGS05406
SEQUENCE DESCRIPTION:
GATCGGGTTG AGCCCAGGAG GTTGTGGCTG CAGTNAGCTG TGATGTGCCC TTATGCTATA 60
GCCTGGGCAA GAGCGTGAGA CCCTGTCTCA AAGAAGAAAA AAAGAGAAAA ATAACTCTTT 120
TGAACAAACA GNCAAATTAG CTAGTAGTAT GGAGATGTAT ACCCTCTATT ACACACATAA 180
AACCGTAACA AAATTCATTG NGGTGTATTA TAGTTAGTTT TGTGAATAGA GAAATAAAGC 240
ACTGATGNTT AANNTTGGTT ACAGTGACTT TTAGAAGGGN TTNNTTGNTG GTTCACATTN 300
GTCAGAANTT TTTTTCCNNC NNGGCTGGTT CAN 333

SEQ ID NO:4555
SEQUENCE LENGTH:250
SEQUENCE TYPE:nucleic acid
TOPOLOGY:linear
CLONE:HUMGS05407
SEQUENCE DESCRIPTION:
GATCTCCCCC TTCCCTTGNG GGGCACAGCT GGCCTGGGCC TCCAATCCTG CGGANTTTCC 60
TGGGTGTGGC TTTGACCTCA GAAGTGGCTC TGGTTTGGCC TCAGGAGTGT GGCCTGGCCC 120
AGCCTGCTGC AGCCTCCTGT GGNGGGCCCT TNATGCCACT AATCCCCNGA CCCCCCGGAT 180
NTTNCAAACT GNACAGACAC ACGNATTGTT AAGGCCGCTT ANGGCCTCCA GCGTGCACTC 240
TTNTTTANGN 250

SEQ ID NO:4556
SEQUENCE LENGTH:211
SEQUENCE TYPE:nucleic acid
TOPOLOGY:linear
CLONE:HUMGS05408
SEQUENCE DESCRIPTION:
GATCAATGCA NCGACGTNGG GCTCATGGCC TACCTCGGCA CCATCACCAA AACGNGCAAC 60
ACCATGAACC ATTTTGTGAA CAANTTCAAT GTCCTCTACG ACCGACAAGG CATCGGCAGG 120
AGAATGCGCG NGCTCTNTTT CTGATGAGGG TACTTGAAGN GGCTGATGGA CAGGGGTCAN 180

NGCAACTATC CCAAAGTGGA GNGCACTACA N 211

SEQ ID NO:4557
SEQUENCE LENGTH:308
SEQUENCE TYPE:nucleic acid
TOPOLOGY:linear
CLONE:HUMGS05409
SEQUENCE DESCRIPTION:
GATCTTTTTT CCATTGGTTA AACATTTAAC TCCATGNCAG ACCTTGTTTT AACCCCTCTC 60
ACATCATGTT CTTTCCTTTT TTGCGAGTTA TTTTGCATTA ACCAACTTTG TCAGTGACAG 120
ATGCGTATCT GAGGGTGTCA CACACGACCN TCAGCAGGGA AGACTTCTGG GCCATGGAGG 180
GCCGTCTAAT ACATGGACTT ATAAACTGAC TGCNTGGGCA ATGAAAAGGC CAAATTATTC 240
TGAATTTTTT TNGAATCACT GTAAAAAAAC TGATTTNTTN NTGTATAGAG ANCACTTAAA 300
CGGTGGAN 308

SEQ ID NO:4558
SEQUENCE LENGTH:175
SEQUENCE TYPE:nucleic acid
TOPOLOGY:linear
CLONE:HUMGS05410
SEQUENCE DESCRIPTION:
GATCCAGCTG GCTCATCCCA GCCCAGGTGG GCCAATTATT CAATTTTNAA GAATTTTGTT 60
GCAAGCCAGT TGTCAAACAC AGCCATTATA ATTATGTAAA TTTGCAANTT ATGTTAAAAA 120
CAAGGACAAT AAATATTCAA AATGCATCCC TAATTACTAC ATTTGACCAC GNAAA 175

SEQ ID NO:4559
SEQUENCE LENGTH:178
SEQUENCE TYPE:nucleic acid
TOPOLOGY:linear
CLONE:HUMGS05411
SEQUENCE DESCRIPTION:
GATCTTNACA ACTGTTTAAT CAGTTTTATT TTTGTACAGT ATTAGTGACC TAAGTNATTT 60
TGCTGTCCCG TTTTTGTAAA TCAANTGAAA NTATAAAAGA GGCTTGTGAC AGTAGGTATT 120
TTGTACATAT GTATATATGT TGTCCAAATA AAATTAATAA ANTGGTNAAA GGCTGAAA 178

SEQ ID NO:4560
SEQUENCE LENGTH:77
SEQUENCE TYPE:nucleic acid
TOPOLOGY:linear
CLONE:HUMGS05412
SEQUENCE DESCRIPTION:
GATCTTCAGC ATCTTTTACT TTCACCAGCG TTTCTGGGTG AGCAAAAACA GGNAGGCAAA 60
ATGCCGCAAA AAAGAAA 77

SEQ ID NO:4561

SEQUENCE LENGTH:203
SEQUENCE TYPE:nucleic acid
TOPOLOGY:linear
CLONE:HUMGS05413
SEQUENCE DESCRIPTION:
GATCCCAGGA ATATTGTAAA TNTTCTAACT TTACTCTGCA TTTTGTATAT CTGGCCTCTA 60
TTGCCCTTGA AGGTGAAGAT GAAACTGTCT TTAGAAGATA TCTCTTTGAT TCTGTGATAG 120
AAGTCCCTCA CATCTTGTAT TAATTTTATG TATATATCAA CGGTGGTTGG TCTTTAAAAA 180
AATAAATCAA AAGAATANGT AAA 203

SEQ ID NO:4562
SEQUENCE LENGTH:352
SEQUENCE TYPE:nucleic acid
TOPOLOGY:linear
CLONE:HUMGS05414
SEQUENCE DESCRIPTION:
GATCAACCCA CATTAGAGTG TCTAAGGACT CCTGAGAATT CCTGTTACAG TAAACAAAAC 60
TAACGTAATC TACCATTTCC TACACTATTT GAGCATGGAA ATCATAGTCC CCACTCTGTG 120
AAAACTTAAC GCTTTTTGGA AGACATTTCT GTAGCATGTC AGTTTGGAGA AATGATGAGC 180
TACGCCTTGA TGAAAGAACC GTGTTGGTGC TGCTAAGTTT AGCCATTATG GTTTGNGCCN 240
TTNTCTNTCT TAAGGCCTTA TTCTTTCAAC TAAAAAGNTG GGGGATTNAG GNGCCAAGGN 300
AGNTTGGGGG GGGGNTNNTG NAAATTAANT TTTNTTGAGG GGTNGGCNTA GN 352

SEQ ID NO:4563
SEQUENCE LENGTH:210
SEQUENCE TYPE:nucleic acid
TOPOLOGY:linear
CLONE:HUMGS05415
SEQUENCE DESCRIPTION:
GATCCGCAAC CTGGACCCCA GTGAGAGGCG GTGGGCTGAC TGGGAAGNAG AGGCCCCCAA 60
CCTACTCAGG ATTTNCACGT GTGAACTAGG CTGCCTGTGG GGTGCCCCTT AGGCTTGGAG 120
AGCCCCAGAT TGGAGGCAGA CAGACTGCAC CACCCCTTCC CCCCCTGCAT CTCAAGAATA 180
AAGCAAGCTG CCTTTGTACT TGGTTGNAAA 210

SEQ ID NO:4564
SEQUENCE LENGTH:74
SEQUENCE TYPE:nucleic acid
TOPOLOGY:linear
CLONE:HUMGS05416
SEQUENCE DESCRIPTION:
GATCANATAG TTGCTGTTTT GTAAAATGTA ATGTATATNT GGTTTTAAGT AAAATAGGGC 60
ACCTGTTTCA CAAA 74

SEQ ID NO:4565
SEQUENCE LENGTH:303

SEQUENCE TYPE:nucleic acid
TOPOLOGY:linear
CLONE:HUMGS05417
SEQUENCE DESCRIPTION:
GATCCAGAAA CTGAGAAGCT TATCCGAGAG AAAGATGAGG AGCTGCGGCG GATGCAGGAG 60
ATGCTACACA AAATACAAAA ACAGATGAAG GAGAACTATT AACTGGCTTT CAGCCCTGGA 120
TATTTAAATC TCCTCCTCTT CTTCCTGTCC ATGCCGGCCC CTNCCAGCAC CAGCTCTGCT 180
CAGGCCCCTT NAGCTACTNC CACTTCGCCT TACATCCATG CTGACTGNCN AGAGACTNAN 240
GNGGCANTAA AGTTTAATAA ATCTGTAGGT GGCNNANAGA AAAAGAAAAN AACAAAANAA 300
AAA 303

SEQ ID NO:4566
SEQUENCE LENGTH:109
SEQUENCE TYPE:nucleic acid
TOPOLOGY:linear
CLONE:HUMGS05418
SEQUENCE DESCRIPTION:
GATCGTGTTT TAACTTTTTC TTTTCCTGTT TTTATTTTGG TATTAAGTCG TTGCCTTTAT 60
TTGTAAAGCT GTTATAAATA TATATTATAT AAATATATNA AAAAGGAAA 109

SEQ ID NO:4567
SEQUENCE LENGTH:303
SEQUENCE TYPE:nucleic acid
TOPOLOGY:linear
CLONE:HUMGS05419
SEQUENCE DESCRIPTION:
GATCTGGAGG CAGCAGTGCT GGATGGAATT GTCTAGGCTG TGGCATGTTG GTTTTGTCTT 60
TCTTTTCTCC TTTGATTATG TAAGAGCTAT TTCATTATAA CTTATTATGG TGATTATACA 120
GGCAAGAAGA CAAAAAGGAG AGAAAATGTA CCTCTTCTAC TGGAATAATG TTTATGATTA 180
CAAGTGAGAT AAGGTATTTT NATCAATATG AAGGCAACCT TGGCTGATAA AACCTCTATA 240
GTGAATACTC ACATCTTTNA CTTCACTCAC TATCAATAAT AAATATATTT NCTGACAANG 300
AAA 303

SEQ ID NO:4568
SEQUENCE LENGTH:230
SEQUENCE TYPE:nucleic acid
TOPOLOGY:linear
CLONE:HUMGS05420
SEQUENCE DESCRIPTION:
GATCTCTTTT GTAGAGGATT TCAATGTATT TCTTTATCAT TTGAGTGTGT GTGTGATGGA 60
CGAATATGTG TGTGAGTTTG AGAAGCATAT CGTTCGTGTC CAGTTACTTT GCAAATTTGT 120
GGACATTTGT GATTGGACAG AGGGGTTTGT GCTGTGGCCT AACACTTGCC AAGTGAGGTG 180
TAGGTTATGC CTATATGCAA ATTAAACTTC ACCTTTCTTG AATATTCAAA 230

SEQ ID NO:4569

SEQUENCE LENGTH:355
SEQUENCE TYPE:nucleic acid
TOPOLOGY:linear
CLONE:HUMGS05421
SEQUENCE DESCRIPTION:
GATCCCCAAT TGACTCAGAG TTTGAGGAGT TAGTATCACA GAATTAGATT TTTTTAAAGC 60
ATTTGTACGT TTCCATTCCC AAATATGTAG CTGTGGTTCT TGAAAACACA TCCTACATTG 120
CATATGGGCA TAGCAGTTTT TGACCCAGGC AGAATAAGTT AATATTTAAT TAAATATTGC 180
TTTGAAGATG GCGCTCTGGG CATGAGCATG GGGCTCCATG ACTTCCCTTC TATCCCCATG 240
AGCCCCTCCT NCATCCAGCG ACAAGCCATG GGCATGCATA CAATGCAGCA AGACCAACAC 300
ANGNGCAATA TTGAATTGGN TCATTCTATC TAAAAATTAC ATGGGGNGGG ANGTN 355

SEQ ID NO:4570
SEQUENCE LENGTH:353
SEQUENCE TYPE:nucleic acid
TOPOLOGY:linear
CLONE:HUMGS05422
SEQUENCE DESCRIPTION:
GATCAGAAAT AAACGACAAA TAGTGTGAGA TGTGTTGTGA ACAGGCATGG TGACCGTGGT 60
CAGCGCCACT CTTGTTTCCT GAAATGTGCT TCTAAGACAG AAAATGTATT TNCTCATCAA 120
GGGTGTCTGG AGACACAGAC CGTGACCTTG GCGCANGGTG TGCATCAGAG GCGTGTGCTG 180
AGAAGGGTGG TGTTAAGCTG TGGAAAATGA CTCAAGAGCA ATAAATCAGT GCCAAAGCTT 240
CCCTGCGCAT CTGAATAGNC ACAAGTGAAG CCCGTGTGCG CACAGTGCAG AGGCCNAGNC 300
TGTATTTCCA GGTAGNGTGG CTTTATTTGA CTGTGAATCC ATTTACCTGT TAN 353

SEQ ID NO:4571
SEQUENCE LENGTH:266
SEQUENCE TYPE:nucleic acid
TOPOLOGY:linear
CLONE:HUMGS05423
SEQUENCE DESCRIPTION:
GATCGGGTCT ATTTCACCCA ATAGTCACTT GTGTGTCCCC CGAAATTGGG ATGGGTGAGC 60
ACCCTTGCTG TGGTGTAAAC TTCCTGTTAT TTAATCTCCC CCACGGTTTC ATTTTTNTNT 120
TCTGTTAATA CTTAGAACTT TCTAAGAAAC TCCATGAAAT GAGGAAATAC TGTTTCTAAT 180
AATATGAGGA AAGANGTAAA ANTGTTCATT CCANGTGGCA AGTCTTATTG GACACATTTT 240
ANATAAGNTC ATGCATACCT GATAAA 266

SEQ ID NO:4572
SEQUENCE LENGTH:279
SEQUENCE TYPE:nucleic acid
TOPOLOGY:linear
CLONE:HUMGS05424
SEQUENCE DESCRIPTION:
GATCGCCCGC ATGAGGTGCT GCTGTGCTCG GACCTGGTCA AGGCATACCA GCGCTGCGTG 60
AGCGCCGCCC ACAAGGGNTG AGGAGCAGAC ATCATTCCCT GCCCTGGCAG TGACTTGGAG 120

CCCTGAAGAA GGGCCAATCA TGGGACCACA GCCACTGTGC CCTGCCGTTT CCTGCTGGGC 180
CCCTGCATAT GCCCCTGAGC CTGGGGCTGC CACGTGTTTA GGAAACAAAG TATGCGCTAC 240
TGTCTGAAAA CAAATAAAGC AGATGCCTTT GTTTTCAAA 279

SEQ ID NO:4573
SEQUENCE LENGTH:131
SEQUENCE TYPE:nucleic acid
TOPOLOGY:linear
CLONE:HUMGS05425
SEQUENCE DESCRIPTION:
GATCTAGGCC AGTGGCTGCT TGTCCTTGTG GAGCTGTGTG TNTNCTNCTN TAAGCAGCTC 60
CTCCCCGGAG TCCCCCAGCA CAGTCCCAGG AGATGACAGG AAGGAAGCAC CAGGGCAAGN 120
CGGACGNTCA N 131

SEQ ID NO:4574
SEQUENCE LENGTH:413
SEQUENCE TYPE:nucleic acid
TOPOLOGY:linear
CLONE:HUMGS05427
SEQUENCE DESCRIPTION:
GATCTCACCT TCCACCTCCT CCGGGAAGTC TTGGAAATGG CCAGGGCCGA GCAGTTAGCA 60
CAGCAAGCTC ACAGCAACAG GAAACTCATG GAGATTATTG GGAAATAAAA CGGTGCGTTT 120
GGCCAAAAAG ANTCTGCATT TAGCACAAAA AAAATTTAAA AAAATACAGT ATTCTGTACC 180
ATAGCGCTGC TCTTATGCCA TTNGTTTATT TNTATATAGC TTGAAACATN GAGGGAGAGA 240
GGGAGAGAGC CTATACCCCT TACTTAGCAT NCACAAAGTG TATTCACGNT GCAGCAGCAN 300
CACANTGTTA TTCGTTTTGG NCTTCGGTTT NGTTNCCGGG GNCAGGTGTT TTTATTAGTG 360
GNGTTTTTAA AAGGNGGNTN GTNGGGACCC TGTTGGAGNA NAANCGGTTT TGN 413

SEQ ID NO:4575
SEQUENCE LENGTH:55
SEQUENCE TYPE:nucleic acid
TOPOLOGY:linear
CLONE:HUMGS05428
SEQUENCE DESCRIPTION:
GATCACGCCA CTGCACTCCA GCACCCTGGG CGACAGAGTG AGACCCTGTC TCAAA 55

SEQ ID NO:4576
SEQUENCE LENGTH:102
SEQUENCE TYPE:nucleic acid
TOPOLOGY:linear
CLONE:HUMGS05429
SEQUENCE DESCRIPTION:
GATCTGGCGT TGCCAGGCAT TCTGTAAGGG AGGGCTTTTC CAGTTCCTGG AAATGGAACT 60
GGAAAATNTG GCTGGAAACA TTAATATGGC TGGAAATGGA AA 102

SEQ ID NO:4577
SEQUENCE LENGTH:420
SEQUENCE TYPE:nucleic acid
TOPOLOGY:linear
CLONE:HUMGS05430
SEQUENCE DESCRIPTION:
GATCCAAGCT GCTTGAGGGG GTCAACCTTG GACCAAAGTT GCCTTAAGCC TGTGGTAAAA 60
GGGCTTCAGG GAAGGTAAGT GGGCCANCTG CTGGAAGCTG CCAGCTGCCC GGNTGGCAAT 120
GGTGTGAGTG TCTTGGCCCT GTCCCTGCCC TGGGGTCCAG CAGGTNATCC CTCCCTTCTT 180
CTCTCTCCTT TGGCGTTTGT TCCTGTAGTC ACTGGGCTAA TCTCCCCCTA GCTTGAAGCT 240
GTACATAGGG CCTCCNAGTG CAAATGNTNC TGNCCATACC GTGCACCCTT AGAAGCCTGC 300
GTGTGCATAG AGCGCCCNNT ACTTNCCAGT TAANTTCCNA GTTCTTGTTN CNTGAGCTTG 360
GTATTTTGTC ATGTGNCAAA TTCTGACTTT TGAAGGTTGG GNAAGTGAAG GGGAATTNAN 420

SEQ ID NO:4578
SEQUENCE LENGTH:307
SEQUENCE TYPE:nucleic acid
TOPOLOGY:linear
CLONE:HUMGS05431
SEQUENCE DESCRIPTION:
GATCTGGGAC GTCCACGAGT GAGGGGCTGG CCACCCAGCC CGACAGCCTT GCCTGACCAC 60
CCTCCAGCAG ATAGACGCCG GCACCCCTTC CTCTTCCTAG GGTGGAAGNG GCCCTGGGCC 120
GAGCCTGTAG ACCTATCGGN TCTCATCCCT TGGGNTAAGC CCCAGTCCAG GTCCAGGAGG 180
NTCCCTCCCT GCCCAGCGAG TCTTNCAGAA GGNGTTTAAA AGGGTTGCAG GTNCCNACCA 240
CTGACCCTTC CCGGNTGGCC TNCTTGCNCA GCTTANAANC TTAAAGTCCA GNACGCAGTA 300
ACCTNTN 307

SEQ ID NO:4579
SEQUENCE LENGTH:239
SEQUENCE TYPE:nucleic acid
TOPOLOGY:linear
CLONE:HUMGS05432
SEQUENCE DESCRIPTION:
GATCCCTCTC CAAAGTGGTT GTCTTTCTNT ACCTTGTGTC TTCTCTTGTA AAAATGAAAC 60
TCAAAAATAA AATAAATGTG TCAAATTTTG AAAAAAAANG AAAACTGAAA AAGCTAACAT 120
GANTTGTGTG AAATTGCATA ATGCTGTANT GCTAATCTAC AATATGTAAT GCTATCTTGT 180
ATGTNGNATT TGTTAATGCA CCACACAAGT GCAANGTAAA GACTGATTCA CNTTGNNNN 239

SEQ ID NO:4580
SEQUENCE LENGTH:51
SEQUENCE TYPE:nucleic acid
TOPOLOGY:linear
CLONE:HUMGS05433
SEQUENCE DESCRIPTION:
GATCTGACTG TACTAAATAT GAAAAATAAA GCAGCACATA CTTTCTCTAA A 51

SEQ ID NO:4581
SEQUENCE LENGTH:201
SEQUENCE TYPE:nucleic acid
TOPOLOGY:linear
CLONE:HUMGS05434
SEQUENCE DESCRIPTION:
GATCCATATA ACTGTTGGGT GCATCAGTGC TTTTNAAAGG GGCTGCTTAC TATAGGGTTA 60
ACTATGTATA TNCATTGTTA AGAGTTAACT TGTGGTTTGG CTGTTTCCTG GATTTNATAA 120
CATACATGTG CAGAAATGTA TTCAAATGAA AGGNAGCATA CCTTTATCAN GATGCTATTA 180
AANTTGANCA TCGNGTNTAA A 201

SEQ ID NO:4582
SEQUENCE LENGTH:170
SEQUENCE TYPE:nucleic acid
TOPOLOGY:linear
CLONE:HUMGS05435
SEQUENCE DESCRIPTION:
GATCATACAG TGAGGCTACA GTGACTGAGG GGAGAATCCC CTCCTGTTCA CTCTCCCAAC 60
CCTGCTCCAG CCCCTCAGCT TCCCAGACCC TCATGCANTT GGTTGTAAAT NCTCCCAGGA 120
GCTGTTTTAC TGTCTACTTT TCAGGATTAA AAAAAANNNN AAAACTTAAA 170

SEQ ID NO:4583
SEQUENCE LENGTH:393
SEQUENCE TYPE:nucleic acid
TOPOLOGY:linear
CLONE:HUMGS05436
SEQUENCE DESCRIPTION:
GATCGGACTC TGCCCCTGGA CCTGGGAACG ACTGGACTGT CACGGGGTTA CCTCCTAGCT 60
CTCCCAGTGA ACTCCTGCCA GGCACACACA GCCCCTATAG CACTGAGCTC ACATGGGACT 120
GGGATATGGG GGCATCTCTT CCCCAGAGAG GCACTCAGTG AGCCTCCTGT GCCTGGNCCC 180
AGCCTGGGCC ATCTCTTAGG TGAGACAGTT GCCCGAAACT AAGCCAGGCC TGGCTGGAGG 240
AGCAGCAGCT TGGGGAGAGG GATTTCCCTG CAGACCTCAA GCCATCATGC GGTGGGTGCT 300
GCCATGACAG AGGCTGGACC NCTGGGCCAG CGGGGTTGCT CAACCACCTT TTGTGCAAGG 360
TGGCCTTTGT GCTTGCGCCT GCAGGCAGAN GTN 393

SEQ ID NO:4584
SEQUENCE LENGTH:162
SEQUENCE TYPE:nucleic acid
TOPOLOGY:linear
CLONE:HUMGS05437
SEQUENCE DESCRIPTION:
GATCCTGCCC GAGGAGGAGG ACGAGGGTGC CGACTCCTAA CCCCGCCAGG CAGCCTCGTT 60
CTGCACAGGC ACTTTAGCCC GAGCCAGGNA CACCTGCGAG GGGGCAGGTG TGCTCCGCCG 120
CCCTGCTGAT AAGCTGGCTT CATTAAACTG ACACTTCTCA AA 162

SEQ ID NO:4585
SEQUENCE LENGTH:122
SEQUENCE TYPE:nucleic acid
TOPOLOGY:linear
CLONE:HUMGS05438
SEQUENCE DESCRIPTION:
GATCTGTAGG GTCAGGAACA CAGCACCTAA CACAGTGCCT TATGGATAGT AGTTTTATAA 60
TACAGATGTG TTGGAAGAAT AAATGAATGG GTCCTTTTCA AACAAATTCA CCAGAAGTTA 120
AA 122

SEQ ID NO:4586
SEQUENCE LENGTH:288
SEQUENCE TYPE:nucleic acid
TOPOLOGY:linear
CLONE:HUMGS05439
SEQUENCE DESCRIPTION:
GATCTGTCAC CTTCAGCGAG ACCTATTTCC TCCCCACCCC CAGAAACCTC TTGTGTTCTT 60
GCCTAGGCCC AGGTGTTCCT GGCAGCCAAA TCGAGTCTCT CATTTTCTCT TGTGGACCAG 120
TTAGTTTTGC CCATAACGCA GTATTCTGAG TTTGCAACTG TCTCTCTGAT GTGTGCCTTT 180
TGTTCAACAC AGTAACCCCT GCATTCTGCT CTGCTCTAAT ACACTACCTG GAGAAAGTCT 240
TTTCCTTATT TTCAATAAAT GTCAGACATT ATTGNAAAGA AAAAANTN 288

SEQ ID NO:4587
SEQUENCE LENGTH:188
SEQUENCE TYPE:nucleic acid
TOPOLOGY:linear
CLONE:HUMGS05440
SEQUENCE DESCRIPTION:
GATCCCTATG TGGCTAACAG AATTAATGNT TCCAAAAATG TTGNAAATTA TATAGTNCTC 60
TTAATTCCCC ACCTCTAACT ATATTTTTGG GTTATTTCTT TAGGAACAGA TGCCCAGGAG 120
TCATATTACT GAGAATCTAG AAATCTTTTG CAAAGTTCTT GTTATNTTGN CAAATTGCTT 180
CCCNNNNN 188

SEQ ID NO:4588
SEQUENCE LENGTH:287
SEQUENCE TYPE:nucleic acid
TOPOLOGY:linear
CLONE:HUMGS05441
SEQUENCE DESCRIPTION:
GATCTGCCCG CCTCGGCCTC CCAAAGTGCT GGGATTACAG GCGTGAGCAC CAGTGCCCAG 60
TCCTGCCACA TCTTTTTTGT TAGTTGCAGT AGCAATACAA CCTTATCTAG AAAAGCTGGC 120
CAAAAAAGAA AATACCATGA TAATAGGGTA CCTAATTTAT TGCTTTATTT AAGAAAAATG 180
TAATATTTCA TTCGAATAAG TACTTTTGGG CTGGGATGNT ATTTCATTTA GCTACTTGGC 240
ATGCCACCCA TCTTAGGGAA TTATATGAGG GTTTATACTT TAAAGTN 287

SEQ ID NO:4589
SEQUENCE LENGTH:289
SEQUENCE TYPE:nucleic acid
TOPOLOGY:linear
CLONE:HUMGS05442
SEQUENCE DESCRIPTION:
GATCAAAATT TCAAGTTGAC AAATTTGGAT TTGAAAACTC ATTCACTTTT TCAATTTTTT 60
TAATGAGGCA TAACATGCAT ATGGTTAAGT GCACAAATCT TAAGTGTACA GTCAGTGAAT 120
TTTTGCATGT ATGTATGTAC CTGANGCAAG ATATAGAACT TATTTACTTG TAAATAACAG 180
CTATATTGAT ATGTCACATA CCAAANANTT TACCCTTTAA NTTATACAAT TCAGTGGTTT 240
TTNGTATATT TGCAAAATTG TGCAACCNTC CCNACCATCT AATTCTAGN 289

SEQ ID NO:4590
SEQUENCE LENGTH:293
SEQUENCE TYPE:nucleic acid
TOPOLOGY:linear
CLONE:HUMGS05443
SEQUENCE DESCRIPTION:
GATCCTCAAA GAAAGATTTG TTAGAGATAG ACAGGTTCAC AATTTGTGGA AACCGAATTG 60
ACTGAATCTG TGGCTTCATG CGCTGAAGAA GCTGGGTCCT GGGGCAACAA GTGCTGTGTT 120
GCCAGGACAA ATAGATGCTA AACATGGCAC TTAAATATTT ATTTAAAAAC TTAAATTATT 180
ATTGGCAAGC AAATCTTAGT ATCTTTCTTC CAGTAATATG GCCTGGCTGA GGGTCAGACC 240
ACAGGACAGG NGCGACCTCC GGCCTTGACT GTCTGGGAAG CTTGATGGAT TTN 293

SEQ ID NO:4591
SEQUENCE LENGTH:62
SEQUENCE TYPE:nucleic acid
TOPOLOGY:linear
CLONE:HUMGS05444
SEQUENCE DESCRIPTION:
GATCCACTTA GAGCAATAAC CACTTTTTAA ATGTAAAATA AAAAGACAAA TGNAAAGGCA 60
AA 62

SEQ ID NO:4592
SEQUENCE LENGTH:64
SEQUENCE TYPE:nucleic acid
TOPOLOGY:linear
CLONE:HUMGS05445
SEQUENCE DESCRIPTION:
GATCTTCCCA CTGCACTCCA GCCTGGCCAA TAAGACCTGT CTCCAGCAAG ACCCGNGTCT 60
CAAA 64

SEQ ID NO:4593
SEQUENCE LENGTH:323

SEQUENCE TYPE:nucleic acid
TOPOLOGY:linear
CLONE:HUMGS05446
SEQUENCE DESCRIPTION:
GATCTAGATT CATTAGGAAT GTNTTCTTGT CAGCCAGGCC AGGACCCGGG CTTGCCAAGA 60
GCAGAGGCCC TCCCAGCAAC CAGGATACCA CCACTTTGGG GGCTTTGTGT ACAGAGGTCC 120
GGGTCTGAGA CCTCATAGGC TGCAGAAATC TGGGGCAGCC ACCATCAAGA AGCCCCTCTC 180
AGGGGCCAGA ACTCCTTTGC CAGCGTGGAT TTCTCAAGTC GGGACTGCAT AATTAAAGCA 240
GTTGCAGTTT TATTTTTTTT ACAGCTTTTT TCCCAAAAAT GATTTGTAGT TGTGTGTGCA 300
GCACTTTCGG CCTGATATGT GTN 323

SEQ ID NO:4594
SEQUENCE LENGTH:70
SEQUENCE TYPE:nucleic acid
TOPOLOGY:linear
CLONE:HUMGS05447
SEQUENCE DESCRIPTION:
GATCAAAAAG ATAAGTTTAC AAAGTTGAGA CCTCTCATAA AACAAATGAA TAAAAATTTC 60
CTCTTGTAAA 70

SEQ ID NO:4595
SEQUENCE LENGTH:183
SEQUENCE TYPE:nucleic acid
TOPOLOGY:linear
CLONE:HUMGS05448
SEQUENCE DESCRIPTION:
GATCACCTGT GCCTCCCCTC CCCCTTTGTT TGCCCCTGTG TGGTTGGTCA AGGAGGGATG 60
TGAGGGNAAT AGGGACCCCC CGACTTGCCC TCCTGCCTCA GTCTTTCCCC CACCCTGTCT 120
CTTCCTTGTC CTTCTCTGGA AAATGCCAAA ATACACGATG TGAATAAAAG TACAACGGCT 180
AAA 183

SEQ ID NO:4596
SEQUENCE LENGTH:70
SEQUENCE TYPE:nucleic acid
TOPOLOGY:linear
CLONE:HUMGS05451
SEQUENCE DESCRIPTION:
GATCTATAAT TATGCCAATA CCACACTGTC TTGATTACTG TAACTTTAGA ATAAATCTTG 60
AAAGTAGAAA 70

SEQ ID NO:4597
SEQUENCE LENGTH:202
SEQUENCE TYPE:nucleic acid
TOPOLOGY:linear
CLONE:HUMGS05452

SEQUENCE DESCRIPTION:
GATCTGCCTG TCAGAGTCCA CGTGTCCTGG TCACTGGTCT TTATAATTGT TGTGCAATAA 60
CTAAAGGCTA AGGACTAGAT GCACTATCGT GTAAAGAGAT TACACATGAC TGTACCATGT 120
TGCACTTAAT CAAATAGTAT GTGGGGNTTT AAAATCGCTT GCATTGTTTC ACAAAATAAA 180
TATCTCAATG TCAAATACTA AA 202

SEQ ID NO:4598
SEQUENCE LENGTH:149
SEQUENCE TYPE:nucleic acid
TOPOLOGY:linear
CLONE:HUMGS05453
SEQUENCE DESCRIPTION:
GATCCTGGNG CCTGGACCGG GCTGGANGAC AGCTGAGNAT GGGCCTAGCA GATGAAGCTT 60
GCCAGCAAGG CCAAAGCAAA CGGTTTCTNC TGTGGATAGT GGACAGAGAC CTTTGTAACC 120
AATGGAATTA TTCATTTTNC TCTATCNNN 149

SEQ ID NO:4599
SEQUENCE LENGTH:311
SEQUENCE TYPE:nucleic acid
TOPOLOGY:linear
CLONE:HUMGS05454
SEQUENCE DESCRIPTION:
GATCAAGCAA TGGAAAAATT TGCACCACCG TGTCCCATGA AATTGTTTCA ATGTTTAGTT 60
TAGATATTGC TAACTTGTGC TATTAACCTT TTGACAAGCG TGTAGTATTG TTTGTTTTGG 120
GTTTCTTTTT GATTTATAAC TATTTAGTAG TCCCCAGCTA GCTACCAGTA CAGATTTTAC 180
CCCATGGGTA GGATTCTATT GTTAAGCCAC ATAACAAAGC ACACTAATTC TGACANCACT 240
GCAAATGGAA AATACGAACC AAAAAAAAGT TACACCGATA AGTTCANCAA ACTGTCCATT 300
TTTGGATATT N 311

SEQ ID NO:4600
SEQUENCE LENGTH:221
SEQUENCE TYPE:nucleic acid
TOPOLOGY:linear
CLONE:HUMGS05455
SEQUENCE DESCRIPTION:
GATCCTGCCT TTTCNTTTTC CCCACAACTT CACGCCAGNA GTTCGTTCAC ATCAGGTAAA 60
TCTTGAGAAA GCCTTGGTGC CCTCCTCCCC ACCCCCCACT CAGTAACCAC GTGCGTGCAC 120
CCCCGNATCC TTTCTCAGTA GTTAAGACGT TCTAGGCAAT ACCATAGACA CATCTGACTG 180
TGTCTCTNTC TTTGAGTGCA AGAAACTCAA GTNATCTTAA A 221

SEQ ID NO:4601
SEQUENCE LENGTH:176
SEQUENCE TYPE:nucleic acid
TOPOLOGY:linear
CLONE:HUMGS05456

SEQUENCE DESCRIPTION:
GATCAGAGCA GCTACACTGA AGCAAAAACC CCAGCGCCCA ACGCTAAATA TATTGCAATG 60
GAGAAACCGT CCACAATGGG GTGTCTCTGT CCTTAGCAGA GCCTTGGGGC TGCCGTTTAA 120
GACCCTCCTG CTACTCCCAC CCTTTTAGCA TCTAGTAAAC CACGCGCTGT AACAAA 176

SEQ ID NO:4602
SEQUENCE LENGTH:293
SEQUENCE TYPE:nucleic acid
TOPOLOGY:linear
CLONE:HUMGS05458
SEQUENCE DESCRIPTION:
GATCTCTATC ACTTTGCCAC CTGTTTGATG TGCAGTGGAA ACTGGTTAAG CCAGTTGTTC 60
ATACTTCGTT TACAAATATA AAGATAGCTG TTTAGGATAT TTTGTTACAT ATTNGTAAAT 120
TTTNGAAATG CTAGTAATGT GTTTTCACCA GCAAGTATTT GTTGCAAACT TAATGTCATT 180
TTCCTTAAGA TGGTTACAGC TATGTAACCT GGTATTATTN CTGGGNCGGG CTTNTTTAAA 240
ATACNAGNCC NGGCCAANAN AAANTAGGAN CAAAAACCTN GGGGGTTCTG AAA 293

SEQ ID NO:4603
SEQUENCE LENGTH:184
SEQUENCE TYPE:nucleic acid
TOPOLOGY:linear
CLONE:HUMGS05459
SEQUENCE DESCRIPTION:
GATCAGGGAT GGGGATGAGG TGAGGGGATT TGCTAGAAAG GGGTGCAAAG GAATTTAGCG 60
GGTGATGGAG CTGTTCTGTG TCTTGATTGT GGAGTTATTA CAAGACGGTT ATTACAGTTT 120
TGTNGTATTG AATTTTACTG TATGAAAATC ATACCGCAAT AAACCTCACT CACATACGCA 180
CAAA 184

SEQ ID NO:4604
SEQUENCE LENGTH:372
SEQUENCE TYPE:nucleic acid
TOPOLOGY:linear
CLONE:HUMGS05460
SEQUENCE DESCRIPTION:
GATCAAGGAA TCTTTCATGA AGGAAATTTA AATACAGAAT GAAACATTAA NGGTAAAAGT 60
GGAGTAATTA TTTAAATTAT GTGTATAAAA GGAATCAAAT TTTGAGTAAA CATGATGTAT 120
TACATCATCT TCGGAAATAG ATATGATGGA TTCTAGTGAA GACCAAANTT ACTTCTGTTT 180
ACTTTCTATC AGGAAGCATC TCCATTGTAA ATATGTATTT NCATGTTTAT TACAAAGACC 240
CAAATGANAA NTTTTNNGTC CATTTTTNGC ATAGCCTAAT GGTAGACTNG GAATAANAGT 300
TCTGTATTTN TGGGATTTCC NGNGTATAAN NCTGGTTTCT AGNTGGGGCG TTAAGTCCCN 360
TTTGGGTAAA NN 372

SEQ ID NO:4605
SEQUENCE LENGTH:279
SEQUENCE TYPE:nucleic acid

TOPOLOGY:linear
CLONE:HUMGS05461
SEQUENCE DESCRIPTION:
GATCATGTAG GACGCGGCAT CCTGCCCCTG GTAGAGAGGA CGAATGTNCC ACACCATGGT 60
GTCTACGAAA AANAAATAGT TAGTCACCTT CTNACCTTCT CCTCTTTCTC AAAGCCTTCT 120
GTCCCTGGTT TTTGCAAGTN CTGCATTTCC GCCGAGAATC CGCGTTGCCT ACTGCTGCCA 180
CCTCCTGTNC ATTTAGAACT ATGCAAAGAC TCCGNTTCCG TTTTCCTGAG CTCCTNGGGC 240
CCCAGAGTCT CTGTTTGATT ATTTATTTAT TTATTNAGN 279

SEQ ID NO:4606
SEQUENCE LENGTH:324
SEQUENCE TYPE:nucleic acid
TOPOLOGY:linear
CLONE:HUMGS05462
SEQUENCE DESCRIPTION:
GATCGAATTT CACATTGTAG AGATGTATAG CTGTGATTAA GCTGCATATG TATGCTGGGA 60
AACGGCTTGA GTGGATGGCT AATATAAAAG TTGTTGGCCA TAATGCCACA CCTCTCATCT 120
GCTTGAAATA TGGACCAGTA TTCTAAAGTA TCAGCTACTG GGGTCTCATT CACTACAAGA 180
TAANTTCAAA CTTCACAGNA CTTTGCAGCA GTTCACCACC AGGCGGGACT GTGTCTGCAA 240
ACTCNCTTGT CTTCACAGAC GNCTGATTAA NGCNANNNCT CTGGNNTCTC AAAGATNTAT 300
TGGGGAGTGN NGGTTTTNTG GGNN 324

SEQ ID NO:4607
SEQUENCE LENGTH:225
SEQUENCE TYPE:nucleic acid
TOPOLOGY:linear
CLONE:HUMGS05464
SEQUENCE DESCRIPTION:
GATCACAGAC CTAAGGCCCA GGCCACCTNT CCANACCTGG AATCTATGCA NAANTAATTT 60
CCCCTTAACC TGGGAGGCAG CGACACGTGT NACTTCTGTA AGAAACGTGT GTACGTAATG 120
GAACGGCTGA GCGCCGAGGG CCACTTNTTC CACCGGNAGT NTTTCCGATG CAGCATCTGT 180
GCCACCACCT TGCGCCTGGC CGTCTACACC TTTNANTGCN ATGAN 225

SEQ ID NO:4608
SEQUENCE LENGTH:251
SEQUENCE TYPE:nucleic acid
TOPOLOGY:linear
CLONE:HUMGS05465
SEQUENCE DESCRIPTION:
GATCTAAATN CAGACTNNGG AGTTGGGAGG AGGNACCAAA GTGAACCATC CTTCATTTAT 60
TCAGTCATTC GTTCATCTGT CAAACACGTA TTTGGACATC AAGGTTGCAG AGATGAACAA 120
TGCATGGNTT TCATCTTTNA GGAGTTCAAA ACCTAGTGGA GAGANCACAT GGTACAATCG 180
TAACACATGA AGGTCAAGTA AGTNCTGCAG TAAAGGTACT AATAACATGT TCCTTGGNNC 240
AGNAGGATGN N 251

SEQ ID NO:4609
SEQUENCE LENGTH:239
SEQUENCE TYPE:nucleic acid
TOPOLOGY:linear
CLONE:HUMGS05466
SEQUENCE DESCRIPTION:
GATCCAAGTG TTGCCTGTCA GCAGAGATAG GAGTGAAGTT GATGATGATG GTTAGATGGC 60
CTCACAAGGG ACTCTAGTGA TAGTAAAGGT TCCAGTGGCA AACACACAAG TGAAAAGTTT 120
GAATATAGTT TCATAATGTT TGAGAGTGTA AAAAATGCAT TTTTAAATTA ATACATTACT 180
TTCTGTACTA TTTTAAATAA GTGTGTATCT AAATAAACAC ATAANATGTT ATGAGTAAA 239

SEQ ID NO:4610
SEQUENCE LENGTH:344
SEQUENCE TYPE:nucleic acid
TOPOLOGY:linear
CLONE:HUMGS05467
SEQUENCE DESCRIPTION:
GATCATCCTG GCTGGGAGAA GTGGGACCGA GCCACCCAGC TNCACTATCC CCAAGCNGCG 60
CTACAGCCGG GATGGGAGGC ACGTGGCCTC TCTTTTATCC GTCTATTTAT NTTGTAAGTG 120
TATTCGTGTG GAGGAGGTTG TTGCTTTATT TTTTTAAGGC TCTGGAGTNT TGTGTATGGT 180
TTCTTTTCAC ATCNGGGCNT CCCATGGGCA CTTCTAAGAA GAGAGGGGAT TTCTTTGAAA 240
AGGAGAGAGG AATCCCCTAG AGCAGGGGAA GCANTGCCTG CCAGCTNTTG TGCACCTTNC 300
GGGNGAANTT AAATATNCNN NAAATTTTCA AACCAGNGAA AANN 344

SEQ ID NO:4611
SEQUENCE LENGTH:128
SEQUENCE TYPE:nucleic acid
TOPOLOGY:linear
CLONE:HUMGS05468
SEQUENCE DESCRIPTION:
GATCTCTCTC TCTCTCTCCC TTTCCTCTCT CTCTGTTGGA TGCTTCTGTA TCTTTTTCCA 60
CAGCATAATG TCTGGTGTGA TGGGGAGCTA TTTATTGAAA TNAAAGATTA TNTATTTGTG 120
CCATGAAA 128

SEQ ID NO:4612
SEQUENCE LENGTH:92
SEQUENCE TYPE:nucleic acid
TOPOLOGY:linear
CLONE:HUMGS05469
SEQUENCE DESCRIPTION:
GATCCATTTC TGGGATAGAA TTGTATTTTT TAAGTCATTT TTTTTTCTTG AAATGGATAT 60
GTACAAATAA AATAAATGGA AGACAGGATA AA 92

SEQ ID NO:4613
SEQUENCE LENGTH:130

SEQUENCE TYPE:nucleic acid
TOPOLOGY:linear
CLONE:HUMGS05470
SEQUENCE DESCRIPTION:
GATCCAACAC ATTCANGTCA GTAGAANCAA ACCAAAACAT GCAAGCTTCA GCCTGNTCGA 60
GTTCTATAGT TGTCACCTAA ATCGTATGTT GTATGATACA TANGGTTATG TATTAGCNCG 120
TAGCCGNGGN 130

SEQ ID NO:4614
SEQUENCE LENGTH:279
SEQUENCE TYPE:nucleic acid
TOPOLOGY:linear
CLONE:HUMGS05471
SEQUENCE DESCRIPTION:
GATCACAGCT NCCCTTGAAT CTCATTGCCT CAGAGAAGAC TAGAGGGCTC TTGGACTATC 60
CCTAGGGCTA CACAAGAATA GTTCAGCCTT CTGCCATGCC ACACAGCCTC AGCTTGAATC 120
TGGTTCATTG CGTCCTCGTG TTCTTCTCTC ATCCTTGCCT TANACCAGGG ATTCTGATAC 180
CGAAGAAGAG GGGCCAATGA AAACCATGGA GTCTGTTCGT GACTCCCAGG GCTGGGACAT 240
TATGTAGGAG CCACTTCATA AACATTCTCT TTACTCAAA 279

SEQ ID NO:4615
SEQUENCE LENGTH:333
SEQUENCE TYPE:nucleic acid
TOPOLOGY:linear
CLONE:HUMGS05472
SEQUENCE DESCRIPTION:
GATCAGTCAT GTGTCCGTAA CCTCGTTCCA TGACATCAGG ATAGCTGTGT TTGCACACCA 60
TGCTCCATGT NCATTCGGAG CCAGGAGGGG TTCTNAGTGG AGCCTGGCTT AGGGAACAGG 120
GAGCGATGGA AGAATGCCAA CATTAGCGTT GGTCTTCTCT TGTNAGGAAT NAAGGATGCT 180
TGCACACATG CACCCCCTCA CTCTCACACT TGCACACATA CACACACACA CACACGAANA 240
TGGTTGGTTT GTCAAANCTC ACTGTAGTAC ATAAAGNTTG CACTCTGNGT NNNNATATCT 300
AGCNGNATGG GGGGTACGNT TTGGGCNGGT GTN 333

SEQ ID NO:4616
SEQUENCE LENGTH:335
SEQUENCE TYPE:nucleic acid
TOPOLOGY:linear
CLONE:HUMGS05474
SEQUENCE DESCRIPTION:
GATCTCTTCC CACCTACCCT ACCTACTCCT ACCCAANACC CCCCTTCCCC ATTAATGTGA 60
GTAATGAATT AGCCTGACCA CAGGTGGTCA CTGTAGGCTA ATGGAAAATA CCCAAGGGNG 120
GGCAAAGCCC CCCATCAGAT GCATGAAGGT TTGCNAATGT TGACTGCCAC TGCCCCACAC 180
ACTGTGTCTT TATAGANTTN CCCTTTGCCN CACCCTCTTC CTGTNTCCAA CCTGGGACAC 240
ANCTTNGCTC AAAGNGCTGG TGGNTTGTGG GGCATTAATC TACAACCANA GTCCTGAATG 300
GAGGCAAAGA GGGCCCCACN GGNTTTGNGG GGNTN 335

SEQ ID NO:4617
SEQUENCE LENGTH:131
SEQUENCE TYPE:nucleic acid
TOPOLOGY:linear
CLONE:HUMGS05475
SEQUENCE DESCRIPTION:
GATCCATTCA CATTGCAAAC TTATCCTACT GTATAGCCAT CCATTGTAAG AATACATTTT 60
TCNATTTATA TTGTGTACAT TACTCCAGTG AACATTCTNN ANCTCCTCTT TTNGTATCCT 120
GTGCAAGANT N 131

SEQ ID NO:4618
SEQUENCE LENGTH:323
SEQUENCE TYPE:nucleic acid
TOPOLOGY:linear
CLONE:HUMGS05476
SEQUENCE DESCRIPTION:
GATCTCAGCT ACGTGGGAGG CTGAGGCAGG AGAATCTCTT GAACCTAGGA GGCAGAGGTT 60
GCAGTGAGCC AAGATTGTNC CAGCCTGGGC GACAGGGTGA GGCTCTTGTC TCAAAAAAAA 120
AAGTCCACAT CTTCATGANC CCTCAGACTC TGGAGTTGGG TGTCGGCTTT TTTAGCCAGN 180
TTTTGTGGGA ATTGCCTTTN NCCTATTAAA GANGGAANGT GGGTAATGGA GTCCCAGCCA 240
CTCANGAGNC TGGNTATCCC CCGAGANTGG CTTGGGTTAC CAGCTATGGA CCCTTGGAAG 300
ATGANTCTAN ATCCTTCTCA CNN 323

SEQ ID NO:4619
SEQUENCE LENGTH:346
SEQUENCE TYPE:nucleic acid
TOPOLOGY:linear
CLONE:HUMGS05477
SEQUENCE DESCRIPTION:
GATCACCTCT CTGTATCCCC CACCCACTAT CCCATTTNCC CTCCTCCTCA GCTAGGGCCA 60
CGCGGCCCCA CATTGCACTT CTGGGGGGTG ACCGACTTCG TACACGGGTT TAAAGTTTAT 120
TTTNATGGTT TAGTCATTGC AGAGTTCTTA TTTTGGGGGG AGGGAAAGGG GGCTAGTCCC 180
CTTCTTTTGG CCCTCCGCCC CCGCAGGCTT CTGTGTGCTG CTAACTGTAT TTATTGTNAT 240
GCCTTGGTCA GGGCCCCTCT ACCCACTTCT CCCAGTAAGT TGTGGCCCCA AGCCCNTATC 300
CNTGTGCTGT GTGGAGTGGN CAACCTGACC CCCGNAAGNG GGNNAN 346

SEQ ID NO:4620
SEQUENCE LENGTH:343
SEQUENCE TYPE:nucleic acid
TOPOLOGY:linear
CLONE:HUMGS05478
SEQUENCE DESCRIPTION:
GATCTTTTTA AGGATAGACG TGGTCTCATG CTTCCTTTGT NCGACTCCGT TTTAAAGGAG 60
CATTGTAACC ACTATCCTCA TCCCTACAAA AATCCAGTGG ATGCACTTCC AAATTACTTC 120

TGTCATTTTG TATGTGTTGT GTTTAGGGNT GCGTAATTTT CACTGGATGN TGAGATACAT 180
GGTGATACTA TGTCAGTTTA TTCTCTGTGA ATATGTCTGT ACATAGNCAG GAAGCTCTAC 240
TTATTTGTNC ATAGGCTGTC TGANGTATGT ACTTCTGTCA CATGGNCTTC ATTCTGATAA 300
AGGGTGTTTC TCCCTTGNTT CACTGTCCAT ACCTTTGTGN NGN 343

SEQ ID NO:4621
SEQUENCE LENGTH:50
SEQUENCE TYPE:nucleic acid
TOPOLOGY:linear
CLONE:HUMGS05479
SEQUENCE DESCRIPTION:
GATCCTTCTG TGCTTGCTTG CATCTTTAAT AAAGACATGT TCCCGGCAAA 50

SEQ ID NO:4622
SEQUENCE LENGTH:170
SEQUENCE TYPE:nucleic acid
TOPOLOGY:linear
CLONE:HUMGS05480
SEQUENCE DESCRIPTION:
GATCCAGCTA GGCTGGGACA CTCCCTAAAT CAGCTGCGTG TTGTTAGCAT CAGGCAGAAT 60
GAATGGCAGA GAGTGATTCT GTCTTCATAG AGGGTGGGGT ACTTCTCCAT AAGGCATCTC 120
AGTCAAATCC CCATCACTGT CATAAATNCA GNGGNAATNG NCTGNACAAA 170

SEQ ID NO:4623
SEQUENCE LENGTH:290
SEQUENCE TYPE:nucleic acid
TOPOLOGY:linear
CLONE:HUMGS05481
SEQUENCE DESCRIPTION:
GATCCTCTCT GTGTAAATCC TGCCCGGTGC TGTNAAGGCT GGACGGTGGA GGACCTGCTG 60
GGGTCTCCTG GNACCCGCCT GTTGCTTCTN CCCTCCCCTG TGGAAAGGTC TATATNACGG 120
GCTGCCTGAG GCCCCAGAAC TCGTCTGTAA ACCACCTTTT CCAGCCAGAG TTCCCAAAGC 180
TGGAACGCTA GCTGCCTGCT CTTCCTTAAG NTGGCCTCCC CCNGACCCGT CANGGTCCTC 240
AGTTGCCAGG GCTGGGGCCA NCCACTGTNA CACTGNGGNN TGCANGANAN 290

SEQ ID NO:4624
SEQUENCE LENGTH:125
SEQUENCE TYPE:nucleic acid
TOPOLOGY:linear
CLONE:HUMGS05482
SEQUENCE DESCRIPTION:
GATCCAGCTT CAGCTGCACA CCTCTGTCCC CTTGGATGGG GAACTAAGGG AAAACGTCTG 60
TTGTATCACT GAAGTTTTTT GTTTTGTTTT TATACCGTGT CTGANTAAAA ATNCCCACNG 120
TTAAA 125

SEQ ID NO:4625
SEQUENCE LENGTH:252
SEQUENCE TYPE:nucleic acid
TOPOLOGY:linear
CLONE:HUMGS05483
SEQUENCE DESCRIPTION:
GATCCTGACC CCAGCCAACA GCTGAGCTGC CGGGCCTCCT CGAGGCCCTA AGCCAGCCCC 60
AGAGGTCCCA TCTNAAGCTG GAGTACCCTG GGGTCAGCAG CAAGAGAAAG AAGNGGAGAT 120
TTTNTGTTTN TTTTTCCCCT CAGCCCTGCC ACCGTGGGGA GTCTGGTTTT TNTCTTCATC 180
CTGTCTCTCT CCTCCTTACT CTTGGATAAA TAAACAGCCT GTGAGCACAC AGGCAAGCCC 240
GGCCCAGTGA AA 252

SEQ ID NO:4626
SEQUENCE LENGTH:334
SEQUENCE TYPE:nucleic acid
TOPOLOGY:linear
CLONE:HUMGS05484
SEQUENCE DESCRIPTION:
GATCGTNACA AATCTNCCCT CCTCACCAGC TAGTCTGGGG TTTCCTCTCC CTGCCCCAGG 60
CCAGAACTGC CTTCTTCATT TCCACCCACG CTCCCAGCCT CTTAGCTGAA AGCACAAATG 120
GTGAAATCAG TAGTCTCGNT CCATCTNNAA TAGACTAAAC CTAAATGCCT CTAGGACGGA 180
CTGTTGCTAT CCAAGNGTTT GGTGTTACCT TCTCCTGGGA GGTCCTGCTG CAAACTCAAG 240
TTCCNCAGGA TGGTCAAGNT GTCANGANAT ACAAGTTTNC ATCAATTGTA ATTATTACCT 300
GGTCTTTNCA ANTTTGCAAN GGNGTTTTGG GNTN 334

SEQ ID NO:4627
SEQUENCE LENGTH:192
SEQUENCE TYPE:nucleic acid
TOPOLOGY:linear
CLONE:HUMGS05485
SEQUENCE DESCRIPTION:
GATCTTCCAT CAGGGCCGGA GTAAATCCCT AAGTGNCTCA ACTGCCCTCT CCCTGGCAGC 60
CATCTTGTCC CCTCTATTCC TCTAGGGAGC ACTGTGCCCA CTCTTTCTGG GTTTTCCANG 120
GGANTGGGCT TNCAAGGTNG GNGTNTCTGT AAAATCAACA GGTAATAAAA ACTGTGTATG 180
AGNCCNGGCA AA 192

SEQ ID NO:4628
SEQUENCE LENGTH:315
SEQUENCE TYPE:nucleic acid
TOPOLOGY:linear
CLONE:HUMGS05486
SEQUENCE DESCRIPTION:
GATCTACAAT NAGATATCAC TTAACACTCA CTGGAATAGA TATATAATCA AAGTGATAAT 60
ATCAAGTGTT GGNGAGGATG CAGAGAAATT GGNACCCTCA TGCACTGTTG GTGGATACCC 120
ACGTGATATN AAAACAAATG TCTACACAAA AACTTGTGCA CAGATGTTCT TTGCAGCATT 180

ATTTATAATA GCCCAAAATT GANACAGCCC TANTTTCTAT CAACTNACAG GTGAATAATC 240
ANAACGTGGC ATATCCATNC ATGGNGTATT ATTCAGCATT ANANNATGGG ATGAAGTACT 300
GGTATGCACC ACTCN 315

SEQ ID NO:4629
SEQUENCE LENGTH:51
SEQUENCE TYPE:nucleic acid
TOPOLOGY:linear
CLONE:HUMGS05488
SEQUENCE DESCRIPTION:
GATCTGAAAT NATGGTTCAA AAAATACATT CATAATAAAA GTCTTAACAA A 51

SEQ ID NO:4630
SEQUENCE LENGTH:337
SEQUENCE TYPE:nucleic acid
TOPOLOGY:linear
CLONE:HUMGS05490
SEQUENCE DESCRIPTION:
GATCAATGCC CNTNTNTCAG TCTCATCTGT ACTCACGGCA GCCCTGTGGA GTACGGTGTA 60
CTGGCCCAGC TTACAGATGC AGAAAGCGAG ACGTTCTGCC ATNAGATAAA GTCACGTGGC 120
TCTTTAGTAN CACGGACAAG GCTCCTCGCC AAGGAACTCG TGGCAGAAGA GGGCAGCAGT 180
TGGCAGTAGC TGCCGATGTC TGTCCCCAGC TNCACCATTC CTCCCTGTGG CTGTGCCATG 240
CTCGTGGTTT CAGTNTCCGT GTGTNCATGT GNCTNCCCTT NAAGAGCTCG CANCTGGTGT 300
GCTTNGGGGG TCCCAGGGCT GTGTAGNGTN TTCTNCN 337

SEQ ID NO:4631
SEQUENCE LENGTH:90
SEQUENCE TYPE:nucleic acid
TOPOLOGY:linear
CLONE:HUMGS05491
SEQUENCE DESCRIPTION:
GATCTGTCTA TTTCGTTCAT CCATAGTTGC CTNACTCCCC GTCGTGTAGA TAACTACGAT 60
ACGGGAGGGC TTACCATTTC TTCCCTGAAA 90

SEQ ID NO:4632
SEQUENCE LENGTH:97
SEQUENCE TYPE:nucleic acid
TOPOLOGY:linear
CLONE:HUMGS05492
SEQUENCE DESCRIPTION:
GATCAAAGGC TCTTTCCTCT TTTGTTTGAG AGTTGGTTGT TTTAAAGCTT AATGTATGTT 60
TCTATTTNAA AATAAATTTT TCTGGCTGTG GCAGAAA 97

SEQ ID NO:4633
SEQUENCE LENGTH:320

SEQUENCE TYPE:nucleic acid
TOPOLOGY:linear
CLONE:HUMGS05493
SEQUENCE DESCRIPTION:
GATCATCGTA TGCAACAGCT GGGTAATAAG ACTGGCATAG CTCAAACTAT CCTGCCAAAC 60
GCTCTCATCT GATTTTTCCT CCCTTCTCCC CCAACCTCCA ATCACCCTGA GTCACCTGTA 120
AATTCATTTG TCATTCAAAG CGGAATAACA AGTTGTCCCT AGCAAAACCG CTGAGCGCTT 180
TATAATTTTG TGGTGTATTT TTGTCAGTAG GTAGCAGAGG CGGAAGTATT TTTTGGTGTA 240
ATTCTTGAAA TTTTCTGACA GGAAACCGGN TAAAGATAGA TGTGTCTGAG GNTAAAAAAN 300
GTTNGGNNNG NNNTGNTTTN 320

SEQ ID NO:4634
SEQUENCE LENGTH:146
SEQUENCE TYPE:nucleic acid
TOPOLOGY:linear
CLONE:HUMGS05494
SEQUENCE DESCRIPTION:
GATCTATGAA ATAGGTAACA TTTGAGTAGG TGTCATTTAA ANATAGTTGG TGAATGTCAC 60
NTATGCCTTC TATGTTGTTT GCTCTGTAGA CATGAAAATA AACAATATCT CTCGATGATA 120
ATTTGTATTA AGTAATCTAT AAGAAA 146

SEQ ID NO:4635
SEQUENCE LENGTH:177
SEQUENCE TYPE:nucleic acid
TOPOLOGY:linear
CLONE:HUMGS05495
SEQUENCE DESCRIPTION:
GATCTATCCA GCTGCTTCCT TGTAGCCAAG AATGTGTTCA ATGAATTGTG ATTCACTGAT 60
TTTATTGATT TTGTTTTAAA ACAGGGAGAC TGGTATTTTT GAAGCTGCTA TCATTTTCTA 120
TTTCTNTATT AATNNCTTTG TAATCATCTT ATTAAAGTTT TCTTATTTAG TGGGAAA 177

SEQ ID NO:4636
SEQUENCE LENGTH:103
SEQUENCE TYPE:nucleic acid
TOPOLOGY:linear
CLONE:HUMGS05496
SEQUENCE DESCRIPTION:
GATCCTCTCC TTGTATGTAA TACCTTTTTT TCCCCTTTCT AGCAAGTACT TTCAAAAGAA 60
CTCTGTACAT TTTAACATAA AAAATAAATT ATGTTGAGCC AAA 103

SEQ ID NO:4637
SEQUENCE LENGTH:145
SEQUENCE TYPE:nucleic acid
TOPOLOGY:linear
CLONE:HUMGS05497

SEQUENCE DESCRIPTION:
GATCCATTTG TAACTACTCT TAATATATGG TACATAGTTT AAAAAATTTN ATTGAGCTTA 60
AAGTGTATCT AGTTTTGTGT CCTAATTATC CTGCTTAACC TTCTATTGTA AGGNTTTNCC 120
ACGTCAATAA ATATTATAAT GTAAA 145

SEQ ID NO:4638
SEQUENCE LENGTH:279
SEQUENCE TYPE:nucleic acid
TOPOLOGY:linear
CLONE:HUMGS05498
SEQUENCE DESCRIPTION:
GATCANCAAA CTCAACACCA TCTTGCAGGA GCAACGGGNT GGCCTTTAAC ATTGCTGAGG 60
CTTTCAAAGA TGTGTCAGAA CCCATTGTAT TTCTGCAACA GATGCAGGAG TTTAGAGAGA 120
AAATCAAAGT AATCAAGGAA ACTCCTTTAC CTCCCTCTAA TTTGCCTGCA AGCCCTTTAA 180
TGAAGAACTT TGATACCAGT CAGTGGGAAG ACATAAAACT AGTCGGTGTG GATAAACTTT 240
CTTTGCCTCA AGACACTGGC ACATTCATTN GCAAGGNTN 279

SEQ ID NO:4639
SEQUENCE LENGTH:242
SEQUENCE TYPE:nucleic acid
TOPOLOGY:linear
CLONE:HUMGS05502
SEQUENCE DESCRIPTION:
GATCAGAGCA AAGAGAAAAG AAAGCATAAG GACACTTACT CAACAGAAGC ACCTTTAGGC 60
GAAGGAACAG AACAATATGT CAATAGTATC TCAGATTGAC CATTTCTGTT ACTTGTCATT 120
TCTACTTTCA GAAACTAAAT GACTTTCAAA TTTGGGTATA GACAATAAAG AACTGAAGTG 180
CTCACTACTC AGTGATTTGG AAATTTTGAT GCTTGTATAA ATGTCAGATA ATTAATTTCN 240
AN 242

SEQ ID NO:4640
SEQUENCE LENGTH:64
SEQUENCE TYPE:nucleic acid
TOPOLOGY:linear
CLONE:HUMGS05503
SEQUENCE DESCRIPTION:
GATCTTGTTA GACACATGAA TGTGTATAAT CATGTTAAAT GCAAATAAAA CTAGTTCATA 60
GAAA 64

SEQ ID NO:4641
SEQUENCE LENGTH:62
SEQUENCE TYPE:nucleic acid
TOPOLOGY:linear
CLONE:HUMGS05504
SEQUENCE DESCRIPTION:
GATCATNAAG GTAAGGACTG GAGTNATGCA GCCACAAACC AAGGAATATC AGTAGCCATA 60

AA 62

SEQ ID NO:4642
SEQUENCE LENGTH:339
SEQUENCE TYPE:nucleic acid
TOPOLOGY:linear
CLONE:HUMGS05505
SEQUENCE DESCRIPTION:
GATCAGTNCT AACCCTGAGC TGGCGGCTAT CTTTGAAAGT ATCCNGAAAG ATTCATCATC 60
TACTAACTTG GAATCAATGG ACACTAGTTA GATGTTTGTT CACCATGGGG ACCATTACAT 120
ATGACCATAC AATGCACTGA ATTGACAGGT TAATCATAAG ACATGGAAAG AGAAGTGTCT 180
AAAAGCTTCA AAATGTTCCA CTTTTTTTTT CCTTCATGGN GACTGTTTGT TTGGCTTTCT 240
TCCATTGTNG TTTTTGTAGC ATTTATTTCA GAAATGTGTA TTTCCATAAT CCAGAGGTTG 300
TAAAACCACT AGTGTTTTAG TGGGTTACAG CANCATTTN 339

SEQ ID NO:4643
SEQUENCE LENGTH:70
SEQUENCE TYPE:nucleic acid
TOPOLOGY:linear
CLONE:HUMGS05506
SEQUENCE DESCRIPTION:
GATCTTCCTC CTTATTTTAA TGCTTCTTTC TCTTTACAAT TAAAAGTTCA TAAAATCTTT 60
CAACATTAAA 70

SEQ ID NO:4644
SEQUENCE LENGTH:57
SEQUENCE TYPE:nucleic acid
TOPOLOGY:linear
CLONE:HUMGS05507
SEQUENCE DESCRIPTION:
GATCAGCCCT AATNATGCTG TGTCCATGAT GCTTTTAATA AAAACAACCC CCACAAA 57

SEQ ID NO:4645
SEQUENCE LENGTH:158
SEQUENCE TYPE:nucleic acid
TOPOLOGY:linear
CLONE:HUMGS05508
SEQUENCE DESCRIPTION:
GATCTCTATG CCCAATATGC TGCCTCAACT CTGAGCTGTC TGCAAGGCTT AGTAAGTATT 60
GAGTGGTGTT TTTTTTTTTC NTTTTATACA ATACCATNTT AACCACATGA GTTAAATAAA 120
TTTGAGAAGT TNTTTTAAAA CAGTGCTTCA AACTGAAA 158

SEQ ID NO:4646
SEQUENCE LENGTH:153
SEQUENCE TYPE:nucleic acid

TOPOLOGY:linear
CLONE:HUMGS05509
SEQUENCE DESCRIPTION:
GATCCCCGGG TACNANTNAN CCATGCAGGC ATGTTTTTTT TNATTTTTGC ATAGGCAATC 60
CATTCTGTAC AGGTAGCTAA CTTTGTAAAC GCTGTGTATT CCCTCTGCCC CCATGGCTGC 120
TGGTGTAAAT AAACTGCATC TCCCGTTGGT AAA 153

SEQ ID NO:4647
SEQUENCE LENGTH:342
SEQUENCE TYPE:nucleic acid
TOPOLOGY:linear
CLONE:HUMGS05510
SEQUENCE DESCRIPTION:
GATCCAGAAG TCATTAAAAG AAACATAAGG CTGGGGAGGC AGGGGGAGTA AGTTCTATTA 60
GAAAATCCTA ANAGCTTAAC AAAACTAGGG TACTAAATTC AGTTATAACA TGTTACAGAC 120
TTAAATCATA GAGCTGCCCC AACATCTAGA CAGTCTCTCC TACTGATTAT AAATGAGTGA 180
AAACTATCAG TTAGAAAAAT CTAATTTAAG TTGTTAATAC ATGTTTCTTT GGTGAGCACC 240
TGGATATANT TATCACAAAT NCTTTTATAC ATATGTCGAA AATGCTTTCA ACAAACCTAA 300
GTGTCCTAAT TACATGCCAC TTTTTTNAGG CTCACTTTAT GN 342

SEQ ID NO:4648
SEQUENCE LENGTH:333
SEQUENCE TYPE:nucleic acid
TOPOLOGY:linear
CLONE:HUMGS05511
SEQUENCE DESCRIPTION:
GATCGCTACG GTTCTAAATA CTGAAGTCAT GATGTGTATT TCCNGGAGAA ATTCCTCTTT 60
AAATGAACAA GTAACCACAT CTCAGGCGGC AGTGAAGTCC AGATAGTTTT GCAGATTGTT 120
TTGCTACTTN TNCATATGGT ATATGTTTCT GNTTTTAAAT ATTNCTNTTG AGAAATTCTG 180
AGTNCTGATG TAGGAGCTTT CCTGTNATNN CTGTTTCACG TTCCTTCCTG TCACACCCTC 240
CTTGTGGCGT CTCTGTGTAT ATCCTNGGCT TTATTTTCTN GGANCCTTGN GATTTCAACA 300
CTGNGGGCCT GGAGACCTCG GCTCCTNCTG GTN 333

SEQ ID NO:4649
SEQUENCE LENGTH:187
SEQUENCE TYPE:nucleic acid
TOPOLOGY:linear
CLONE:HUMGS05513
SEQUENCE DESCRIPTION:
GATCATGAGA AATTACCAAA ATGTCCACCA ATAGAATGAG TGAATCACCT GTGGTATATT 60
TATAAATAAA AATATTAANT AGAAGGTAAA ACAAATCAGA CATACACACA TTAGATAGAT 120
AAATTTAGAG GAAAAAAAAA AAACCATGCA AGNTTCAGCT NCNCGNGTNC TNTGGTGNCA 180
CCTAANN 187

SEQ ID NO:4650

SEQUENCE LENGTH:228
SEQUENCE TYPE:nucleic acid
TOPOLOGY:linear
CLONE:HUMGS05514
SEQUENCE DESCRIPTION:
GATCGAGGGG GTATGCGTGG GGGAGTTGTG AGTGAGCTCA GCTCCTTTCA GGCCTGGCCT 60
CTCCCAGAAC AGAGTGAAGC CCCTGCCCTG GACCCCTGCT CCCTTCTACC CTTGAGGGCC 120
ACCAATGGAA GAGCCCGCCC TTCATTCTGC AGCTCCTGCG GCCAGGCTGA GCTCTGCCCC 180
CAACTTTGTT TTCTCTCATG TTCTCAATAC ACTCTGCCTC AAATCAAA 228

SEQ ID NO:4651
SEQUENCE LENGTH:300
SEQUENCE TYPE:nucleic acid
TOPOLOGY:linear
CLONE:HUMGS05515
SEQUENCE DESCRIPTION:
GATCTGTAGT TAAGGGAACC AAGGGGTCAT TGGGGCAAAA GCATTGTTTC TCAAAGCTCC 60
TTGATTAAGA GAAAGAACAG AAATTTGCAC AGAAGATAGT GTCAAGGAGT GAGAAAGTTT 120
GTTTGAGGGC AGTAGCTCAG TGTGGAAGAA AATCCTGAAG TTTCTGTTGA AGCCATACAA 180
TGTTCTATGG GGTTACTCTC TAAGACATTC TCTGAGGTGT GTGAGGAAGT CACTACTCCT 240
AGCCTTTGTT AAGATGTAAT TTTAAATATT CAGTTATGGT ACTATGTTTG CAACTCTCTN 300

SEQ ID NO:4652
SEQUENCE LENGTH:154
SEQUENCE TYPE:nucleic acid
TOPOLOGY:linear
CLONE:HUMGS05516
SEQUENCE DESCRIPTION:
GATCAGTTTA TATTGCTCTT AAATGATAAT CCTCAGCTTC CAAGTTTTTG TGAACTCACT 60
TTGGTTTATG TGTTTGCTAC ACTCACTGAG AAATTAAACA CCTTGCTTGA TTTATTGTAA 120
AAAGGAAAAA ACAACAGAGC CATACTTAAG CAAA 154

SEQ ID NO:4653
SEQUENCE LENGTH:250
SEQUENCE TYPE:nucleic acid
TOPOLOGY:linear
CLONE:HUMGS05518
SEQUENCE DESCRIPTION:
GATCTTTATG CTGTTATNAC AGGAGAAGTG ACATACTTTA TATATGTTTA TATTAGCAAG 60
GTCTGTTTTT ANTACNATAT ACTTTATATT TCTATACATN TATATTNCTA ATAATACAGN 120
TATCACTGAT ATATGTAGAC ACTTTTAGAA TTTATTAAAT CCTTGACCTT GNGCATTATA 180
GCATTCCATT AGCAAGAGTT GTACCCCCNC CCCAGTCTTC GNCTNCCTCG GGNTTAAGCT 240
GTTTTATGNN 250

SEQ ID NO:4654

SEQUENCE LENGTH:240
SEQUENCE TYPE:nucleic acid
TOPOLOGY:linear
CLONE:HUMGS05519
SEQUENCE DESCRIPTION:
GATCCCCACC CCAATTCAAT CCCGGAAGGG ACTTACTTAG GAAACCCTTC TTTACTAGAT 60
ATCCTGGCCC CCTGGGCTTG TNAACACCTC CTAGCCACAT CACTACAGTA CAGTGAGTGA 120
CCCCAGCCTC CTGCCTACCC CAAGATGCCC CTCCCCACCC TGACCGTGCT AACTGTGTGT 180
ACATATATAT TCTACATATA TGTATATTAA AACTGCACTG CCATGTCTGC CCTTTTTAAA 240

SEQ ID NO:4655
SEQUENCE LENGTH:290
SEQUENCE TYPE:nucleic acid
TOPOLOGY:linear
CLONE:HUMGS05520
SEQUENCE DESCRIPTION:
GATCCAAATA GTCAGAAAGA AAGGAAGGAA GTGCTGGTAA GAAGGAAGGG AGGAGCGTAT 60
CCTAAAGAAC TACTCCCAAC TATACAACTG TTGTTTTCCT CCCAATTTTC CTAGGGATTC 120
CTCATAATTA TCTCCTTTCT CTTCATAAAT TCTACTTACT GACCTGAAAT AAAATTTCTC 180
ATTTGGTTGT CCTCAAGGTG CAGAATATGG ACTTAAGGCT CCTCCAGACA ATTCAAATGT 240
ATTGTAGACA GTTAAGNTAG AGTAAAACTC ACCGTTTTGT CCCACTGAAA 290

SEQ ID NO:4656
SEQUENCE LENGTH:355
SEQUENCE TYPE:nucleic acid
TOPOLOGY:linear
CLONE:HUMGS05521
SEQUENCE DESCRIPTION:
GATCGACAGG CATCCACAGT TGAGTATCTC CCAGGCATGC TACATTCAAA TTGCCCTAAA 60
GGTCTCCTAC CCAAATTCTC CACGCTGGTC TTNGGTTAAA CTAGGCCCTG CTAAGTCTTA 120
TAACTTTCAT ACAGGTTTGG ACCAACAGGG CTTTATTCCA GGANCAAATT ATCTTATGCC 180
TTGGGACATT GNCATCAGGA CTAGAGCTGN AGATGAAGGA GACTTAGACA CAAACTCTTG 240
GCCTGCTCCA AATAAAGCTA TTCCTNGNAA GAGNAGTGCG GTTGTAATGG NTAAGAGNGN 300
AGANCGGCGA GATGACATTG NTCGAGCTTN TNNTGGNCTT TGAATATNGA AGTCN 355

SEQ ID NO:4657
SEQUENCE LENGTH:382
SEQUENCE TYPE:nucleic acid
TOPOLOGY:linear
CLONE:HUMGS05522
SEQUENCE DESCRIPTION:
GATCCGCAAA CGATTAATTC TGTCAGACAA AGGGCAGCTG GATTGGAAGA AGATGTATTT 60
CAAACTTGTC CGATGTTACC CAAGGAAAGA GCAGTATGGA GATACCCTTC AGCTCTGCAA 120
ACACTGTCAC ATCCTTTCCT GGAAGGGCAC TGACCATCCG TGCACTGCCA ATAACCCAGA 180
GAGCTGCTCC GTTTCACTTT CACCCNNGGN CTTTATCAAC TTGTTCAAGT TCTGGNATCC 240

CAGCACATGA CAACACTTCA GAAGGGTCCC CCTGCTGACT NGAGAGCTNG GGAATATGGC 300
ATTTGGACAC TTCATTTGTT AAATTAGTGT ACATTTTTAA ACATTGGGTT CGGNNAACTT 360
TCAGNGGTNA GGTCGGNGNN TN 382

SEQ ID NO:4658
SEQUENCE LENGTH:326
SEQUENCE TYPE:nucleic acid
TOPOLOGY:linear
CLONE:HUMGS05523
SEQUENCE DESCRIPTION:
GATCATTAAA AACTTAAAAT ATATTTTATT AGAAAACATT TATCTATGAA TGAATATTTC 60
CTTGATGCTG GTCTCTGCAC ACATATGCTT GGTTACTTGC ATGCATTCAT TGGTTGTTCA 120
ATAAGTGAGA TGATTACAGA TAACTTAATA CTGTATTTNC CTTATATGGA AAACCGTTAT 180
AGACCCAATA ACAACTAANC CTTTCAAAAG AAANTATTTN CTATTATGAN TGTTGATTTT 240
CATACCNAAG AAGATGGAGA GTCTAAAATT TGGGNTATGG ATNCTNGATG GNNTTTTTAN 300
TNGGNAAGCC TTCTTNCAAG TTTNAN 326

SEQ ID NO:4659
SEQUENCE LENGTH:133
SEQUENCE TYPE:nucleic acid
TOPOLOGY:linear
CLONE:HUMGS05524
SEQUENCE DESCRIPTION:
GATCAGCTTT GCAGTAGATT ATGCTGCATC CTCGTGGCAA AATTCTGTAT TCTTAGTGAT 60
TGTTACAAAC CCCTTTATTG CTGTCTGAGA AAGTGAAAGA TTGTGTATTT CTATTAAAAC 120
ATTTACAATC AAA 133

SEQ ID NO:4660
SEQUENCE LENGTH:264
SEQUENCE TYPE:nucleic acid
TOPOLOGY:linear
CLONE:HUMGS05525
SEQUENCE DESCRIPTION:
GATCAACATC CGGAGATGGA TTTTNCCAAG GCTAAATTCA ACTAGCCCCT GTTTTTTCCT 60
CCCTGAACTC TTGGGGCTGA GCTGCAACCA CCCAACTTTC TTTCCCACTC TTCTCTGGGA 120
CTTGTGGGCC TCAGGGCTTG GGGCAGGCAT GGGACTGGCC CAGGCACACA GGTCCCGGGG 180
CATCAGGAGA AAGGCTGGGT CTTGGGACCT TGTCCTCCCC AGTTGGCCTA CTGTTACACA 240
TTAAAACGAT TTGCCCAGNT CAAA 264

SEQ ID NO:4661
SEQUENCE LENGTH:319
SEQUENCE TYPE:nucleic acid
TOPOLOGY:linear
CLONE:HUMGS05526
SEQUENCE DESCRIPTION:

GATCACCTNT TCCCTTCAAT CCTTGTGGCT TCTGGGAATC TTCAGAGCCT GGGTCTGAAA 60
GGTGTTTCCT ACATGTCTCA GGGCTGGATG CAAACCTGGC TGGGGACCTG AGCATCAACT 120
CCCATTTAGA ATCAGACATC TCCCTTCCCT GCAAATTGTC TACAACTACC AAATTGCTCC 180
AGGTTTGAGA TGGTATTGCT AAATTTAAAA TTAAACAAGN GACCCANCAN CAGCTTTTTA 240
AAGTGTCTNC TATTTCATNN GTATTTTNNT NAACCTTGGC CCCANTTGNT AGAAAAGTCT 300
TTTGCNGAAA TGNATTTNN 319

SEQ ID NO:4662
SEQUENCE LENGTH:239
SEQUENCE TYPE:nucleic acid
TOPOLOGY:linear
CLONE:HUMGS05528
SEQUENCE DESCRIPTION:
GATCAAAACA GTGTGGTGCT GGCAAAACAA GAGACATAAN TCAGNAGAAG AAAGAGTCCA 60
GAATTAGACC CACACACATA TGGACAATTG ATTTTTCAAT AAGGGCACCA AAGCCATTCA 120
ATGTATAATA GAGAAGACAG TTTTTTTCAA CAAACAGTAT TGGANCAGTT GNNCNNNTAC 180
AAAAAGTAAA CTTCANCCCA TACCTCACAC TATATCTAAA ANTTAGCATC AAATGGNTN 239

SEQ ID NO:4663
SEQUENCE LENGTH:68
SEQUENCE TYPE:nucleic acid
TOPOLOGY:linear
CLONE:HUMGS05529
SEQUENCE DESCRIPTION:
GATCTATTTA GTCCTACAAA TCAGGAGTGG TGTAGAGACA TCCAAATTTA AAGAAAAAAA 60
AACACAAA 68

SEQ ID NO:4664
SEQUENCE LENGTH:122
SEQUENCE TYPE:nucleic acid
TOPOLOGY:linear
CLONE:HUMGS05531
SEQUENCE DESCRIPTION:
GATCGAGTTT TAAATTGATA GGAGGGAACA TGTCCTAATT CTNCTGTCCT GAGAAGCATG 60
TAATGTTAAT GTTATATCAT ATGTATATAT ATATNTNCNC TATGTATATA CATATATNTN 120
AN 122

SEQ ID NO:4665
SEQUENCE LENGTH:196
SEQUENCE TYPE:nucleic acid
TOPOLOGY:linear
CLONE:HUMGS05532
SEQUENCE DESCRIPTION:
GATCAGGGAC GGGGCTGAGC TGGAGAGTGT GCAGGATTGA TTAATGATGT CTGCCACGGT 60
CAGGCCAACT GCTGTGTTTC TGGGGCCCAC AAGCTAAAAA TGGTTGGAAA AAAATCAAAA 120

CATTAATATT TGTGACATAT GAGAATGCTA TGAAAGTCAA CTTTGCTATC CATAAATAAA 180
GTTTTATTGA CATAAA 196

SEQ ID NO:4666
SEQUENCE LENGTH:316
SEQUENCE TYPE:nucleic acid
TOPOLOGY:linear
CLONE:HUMGS05533
SEQUENCE DESCRIPTION:
GATCAGATTG CTCAACTCCA TTATAATTTG TTTCCAAACT AATCTGTTCA GGCAAGATAA 60
AGATATAAAT GTGGATTGTT GCTGAGGTTA AGTCTGGTCA GATTTTNCCA NTTATGCTTG 120
AAAAGTAACC ATATTTCAAT CAAATATATA ATATGTATAT NTCATAATAA TATANGCAAT 180
ATTAGTCTCA TTATTAATTC AAAANTCTCA TCTATATTTC ANCACTGTAG AACATTATTA 240
NNTGANTAAN TGACCTATAA NTATTTGGTG CCTGANGAAG TCACTTTGCT ATTANTNATA 300
CGGTGTAGNG TGTGGN 316

SEQ ID NO:4667
SEQUENCE LENGTH:168
SEQUENCE TYPE:nucleic acid
TOPOLOGY:linear
CLONE:HUMGS05534
SEQUENCE DESCRIPTION:
GATCTCAGGG AAGCCTGGGA GTCCCTTCTC ACCCCTCAAC CTCCGGAGTC CAGGAGAACC 60
CGTACCCCCA CAGAGCCTTA AGCAACTACT TCTGTGAAGT ATTTTTTGAC TGTTTCATGG 120
AAAACAAGCC TTGGAAATAA ATCTCTATTA AACCGCTTTG TAACCAAA 168

SEQ ID NO:4668
SEQUENCE LENGTH:311
SEQUENCE TYPE:nucleic acid
TOPOLOGY:linear
CLONE:HUMGS05535
SEQUENCE DESCRIPTION:
GATCATCCCA TCCTTGACCA CAGGGACAAG AGGGGCCCCC TCGCCCCAGC CCCACCCCAC 60
ATGGAGCTCA GGGGGAAGCA GGGAGGGGTT CCCAAAAGAG CCCTGCGGAG GCTAGGAGTG 120
GTTCTTGATG CTCACCTGAA GCCCCTAGAC GCTGCTAGGA GGGGGGGCCC TCCTGGGCCC 180
NAAATACCCN TCCGCTTGCC GCTGCTAGCG NACCCCTGCA GTCCAGCGTG CATCCACCCC 240
ACGNACGTCC CGTGCTGTGT TCCTGGGCCN TGTCCATGGC CTNAATAAAC TCTCTGCTTG 300
GGTGTGACAA A 311

SEQ ID NO:4669
SEQUENCE LENGTH:167
SEQUENCE TYPE:nucleic acid
TOPOLOGY:linear
CLONE:HUMGS05536
SEQUENCE DESCRIPTION:

GATCGCAAGC TGTTGATGCA CAGGCGTCTT GTTGGCAAGC CCAGCTTCAG TTTATGTTAG 60
ACAATCAGTA CACATGCTGG TGTGTTTTCC AATGGCCAGT NATAAGAAAT TAAATAAGTA 120
TTTGTGAGAT TTGCTATTTT TTAATAAAGT AATTNTTTTA AATTAAA 167

SEQ ID NO:4670
SEQUENCE LENGTH:118
SEQUENCE TYPE:nucleic acid
TOPOLOGY:linear
CLONE:HUMGS05538
SEQUENCE DESCRIPTION:
GATCTCATAC TTGTATAGAG TAATAACAAC AGTTGTACTT CCACCAAAGG CATGTGGCAT 60
ATTTACCAAT GTCATGTATT CTGAACAAAG GCAAAAAATA CAAATTCCTA CCATTAAA 118

SEQ ID NO:4671
SEQUENCE LENGTH:311
SEQUENCE TYPE:nucleic acid
TOPOLOGY:linear
CLONE:HUMGS05539
SEQUENCE DESCRIPTION:
GATCCTTCAA ATAGGCCCTG AGATGTGAGG TCTGCTGCTT CACTGGGGCC CGATGACTTT 60
GGCTGGGGGA GGGGGCCTAG GGCTCTNCTC ATTGAAAGCT CTGCTTTATA CAGACCCAAG 120
CATACACACC AGGCCGTCAC TTTGGGTTCT GGCATAAGTT CAGAACAATT CAAGTCCATG 180
TGTCCCATGG CTGGTCAGAG CCCTGGGTCA AAACCACTCA GCCCAGGGGA GGGGATGAGG 240
CATTGTCACC CTAGACCCCT CTTCCTCTCT CCCNCACCAT AGTGTGCAAT AAAGTGTCTG 300
TTCTTACCAA A 311

SEQ ID NO:4672
SEQUENCE LENGTH:338
SEQUENCE TYPE:nucleic acid
TOPOLOGY:linear
CLONE:HUMGS05540
SEQUENCE DESCRIPTION:
GATCTTGTCA GAGCTGCACC TCTCTGTGAA CTGGCCATTC CTTTCGGTGC TGCTGTCCTT 60
TTTGGGGGGG TTCCTGATTT CTGTATACAT GTAGCTTTGC CAGATATGTA CTTAGTAATA 120
TAAACTGTAT TAATAAAATC CATTTACTGT GTAATACACG AGTTTAAAAA TTAAGAGCGA 180
TTAGCTGTTC ACTTTGGTAA CAGGTAGTTA AGTGTCACAC AAGGTGTTGT TGCAATGGCA 240
TGACGGTGAC CTGTTGGNGG NACTAAGCCT TACAGCAAGG CATGTGCCGN ACGTGCTCCG 300
TGGTACTTAG AGCCCTGGGG GCCCCGNTCG GACTCCGN 338

SEQ ID NO:4673
SEQUENCE LENGTH:117
SEQUENCE TYPE:nucleic acid
TOPOLOGY:linear
CLONE:HUMGS05541
SEQUENCE DESCRIPTION:

GATCTGTTTA CAAGGTTTGT AGATACTTTT TTTGTTCCTG TTCATAGATG GGAAGCTTCC 60
TTATAACTGA TGCAGAGAAA AATTAGTCCT TCAAATACTG CTGTATTTTC AGGAAAN 117

SEQ ID NO:4674
SEQUENCE LENGTH:279
SEQUENCE TYPE:nucleic acid
TOPOLOGY:linear
CLONE:HUMGS05544
SEQUENCE DESCRIPTION:
GATCAGTGTT GCTAGTAGCT TGGCAGCTCT TCATAAAAGC ATATTGGGTT GGNAAGGTGT 60
NTGCNTATTT TTCAAATNAT TTAATAGATG TNTGGTACCA TTTAAAAGTG GTTGTGTCTG 120
AATTTACTGT GGGGATAACA TACACTGTAA TGGGGAAAAA TTACCTAAAA CCAATTTCAA 180
AANGGCTTTC TNTGTATTTC AGTTTAAAAA CCCAGTGCAT GATNCGCCCT CTGGAGATGC 240
AATAAACNCC TTNGTGCCAA AGGNGATTGC CNGNGGAGN 279

SEQ ID NO:4675
SEQUENCE LENGTH:137
SEQUENCE TYPE:nucleic acid
TOPOLOGY:linear
CLONE:HUMGS05545
SEQUENCE DESCRIPTION:
GATCTGTAAT AAAATAGTAT ACTGGACTGT GCATCAAAGG GATGTAAAAT TACAGTATTC 60
CAAAGGTTGA AGTTCTGCTG TTTTGTTATA ATGCCTGATA CACATCTTGA ATAAAGTCTT 120
AACATTTTNC TTATAAA 137

SEQ ID NO:4676
SEQUENCE LENGTH:259
SEQUENCE TYPE:nucleic acid
TOPOLOGY:linear
CLONE:HUMGS05547
SEQUENCE DESCRIPTION:
GATCATAAAA ATTAAAATGT GAATGTCAAC AATAAAAAGC AAGACTATGA AAGGCTCAGA 60
TTTCTTGCAG TTTAAAATGG TGTCTGAGGT TGTNCTATTT TGGCCAAGTC TGTAGAAAGC 120
TGTCATTTGA TTTTNATTAT GTAGTTCATC CAGCCCTTGG GCATTGTTAT ACACCAGTAA 180
AGAAGGCTGT ACTCAAGAGG AGGAGCTGAC ACATTTCACT TGGCTGCGTC TTAATAAACA 240
TGANTGCAAG CATTGGAAA 259

SEQ ID NO:4677
SEQUENCE LENGTH:261
SEQUENCE TYPE:nucleic acid
TOPOLOGY:linear
CLONE:HUMGS05548
SEQUENCE DESCRIPTION:
GATCATCATG ATGAGTTTTG CCTTATGCCT TGAATCTTNA GGTTAATAGT CATAAAATCC 60
CTGCTTTCTA AATTCGCATT TTTCCTGGTG TACCNTTAAT GTGAACNTTT TGGCATTCTT 120

CTGCAATNTT CTGATTGGAG ATTGCATTTT GACCTAGTCT GTAAGTTTTT CTGTCAGAAG 180
AGGACTTTCA TCAACTTTCA NGGNAAGATT GTTTATTGGC ATACTGTAAA GTTAAGTNAN 240
GCANTTTNAA AGTNGTCTAA A 261

SEQ ID NO:4678
SEQUENCE LENGTH:183
SEQUENCE TYPE:nucleic acid
TOPOLOGY:linear
CLONE:HUMGS05551
SEQUENCE DESCRIPTION:
GATCCGATTT TACCTTGGAC CCCTGCGTCT TGCCCCTGGG AACCCANATG GGGACTGCTC 60
TGAACCTTTG GATGACATNT GTGGCCCCGG AGATGTTCTC AACCCAGGGG TGCCCTTCGT 120
AAATGTTNCT CCATCCTCAC TGTACCAGGA GTGTGTGAAT AAACACAGAC CCCCTCTGTG 180
AAA 183

SEQ ID NO:4679
SEQUENCE LENGTH:234
SEQUENCE TYPE:nucleic acid
TOPOLOGY:linear
CLONE:HUMGS05552
SEQUENCE DESCRIPTION:
GATCCCAGTG ACGTGGAAGT CATCAGAACC CCACGGTACT TTGAGTACCT CTCTGCACCA 60
AGATAGCTGA CTGNTTTTCT GCTCAGTCAC AATTTTACTT GNAAGCAAGN ATTGTCCTAG 120
NTCCTNTTCC ATTATTCCAA AACGTTTAAC GTTCAAAGCA GGGTCTCATT AAAANCCCAA 180
CTACTGGTTG ATATAATTGA GATATTACAA TTTCAGAATA AACATTTGAT TAAA 234

SEQ ID NO:4680
SEQUENCE LENGTH:316
SEQUENCE TYPE:nucleic acid
TOPOLOGY:linear
CLONE:HUMGS05553
SEQUENCE DESCRIPTION:
GATCAGAATC TTCAAGAATC AGTTAGGTTC CTCACTGCAA GAAATAAAAT GTCAGGCAGT 60
GAATGAATTA TATTTTCAGA AGTAAAGCAA AGAAGCTATA ACATGTTATG TACAGTACAC 120
TCTGAAAAGA AATCTGAAAC AAGTTATTGT AATGATAAAA ATAATGCACA GGCATGGNTA 180
CTTAATATTT NCTANCAGGA NAAGTCATCC CTATTTCCTT GTNTTACTGC ACTTANTATT 240
ATTNGGTTGA ATTTGTNCAG TATAAGCTCG TNCTTGTGCA AAATTAANTA NATATTNCTC 300
TTACCTNATA NCANAN 316

SEQ ID NO:4681
SEQUENCE LENGTH:304
SEQUENCE TYPE:nucleic acid
TOPOLOGY:linear
CLONE:HUMGS05555
SEQUENCE DESCRIPTION:

GATCATCCTG GAATCCGACT CTGTCTCAGC TCAAGGGGGT TCNGAGATTT CCTAAGCTAG 60
CCCTCTCATT TTACAGCTGC AAAAACGGAG GCCTGTTAAT TCAGTCATTT TATGAAATCC 120
TTTGAAATTT TTATTGAGCT CTAATTGTGT GTGAAGTATT ATGGAATGCT TCTGTACTGG 180
AATGCATAGG TTGAACAAGA CAGAAAGGCC CAGATGGCAG AAGTTCAAGA NCTTTCACCA 240
ACCCATATAG NGAACCTAGT AATATTAATG ACTAATGTTA ATGGANTCCA TGCTTTGTTA 300
CGAN 304

SEQ ID NO:4682
SEQUENCE LENGTH:287
SEQUENCE TYPE:nucleic acid
TOPOLOGY:linear
CLONE:HUMGS05556
SEQUENCE DESCRIPTION:
GATCAAATCG GAGTACTTAG CATATCTATC ATCTCGTGCA TTTATCATTT CTTTGTGGCG 60
AGAACATTTA AAAATCCTAT TTTCTAGCTA TTTTGTAATA TACACTACCT TACTGTTGCG 120
AACCATAGTC ACCCTACTGT GCAATAGAAC ACCTGAACTT ATTCCNCCTA TCTAGTTGTA 180
ACTTNGTACC CATTGNCCCC CCNTCCCTNC CTTCCACTCT CCCCTCCCCA GNCTCTGGTA 240
ACCACTGTTC TGTTCCCTAT GAGATAAAAC TTTTTTTGNC TTCCAAA 287

SEQ ID NO:4683
SEQUENCE LENGTH:298
SEQUENCE TYPE:nucleic acid
TOPOLOGY:linear
CLONE:HUMGS05558
SEQUENCE DESCRIPTION:
GATCTTTTGA AGATTGCTGG AATACTGGTG TCTGTTAGAA TGCTTCAGAC TACAGATGTA 60
ATTAAAGGCT TTTCTTAATA TGTTTTAACC AAAGATGTGG AGCAATCCAA GCCACATATC 120
TTCTACATCA AATTTTTCCA TTTTGGTTAT TTTCATAATC NGGTATTGCA TTTTGCCTTC 180
CCTGTTCATA CCTCAAATTG ATTCATACCT CAGTTTAATT CAGAGAGGTC AGTTAAGTGA 240
CGGATTCTGT TGTGGTTTGA ATGCAGTACC AGGNTTCTCT TCGAGCAAAG TAGACCTN 298

SEQ ID NO:4684
SEQUENCE LENGTH:297
SEQUENCE TYPE:nucleic acid
TOPOLOGY:linear
CLONE:HUMGS05559
SEQUENCE DESCRIPTION:
GATCCATCTT CCTTACACTC CTTCCAAAAT AATGTTAATA CTCTGTCTAT CAAATGCTTA 60
CAGTGTTCTC TAGCACATAT AGGGTAAAGT CAAAGCTCAT TAGCAGGGCA TAGGAGGCCC 120
TTCATGACCA GCCTNACCNG CACCTCTAGC TACATCTCCT ACTGNGNNCA CCTCCACATT 180
TACCCTTCGG CATGCCAACC TGCTTATGGT TACGGGGCAC AGCTTGCTGT TTTGGNTNNT 240
GTGCCTCCCN TTGTTTTGCT TCCTGNTGNT GAANTNATGN TGGGACCATT CTTGTGN 297

SEQ ID NO:4685
SEQUENCE LENGTH:120

SEQUENCE TYPE:nucleic acid
TOPOLOGY:linear
CLONE:HUMGS05560
SEQUENCE DESCRIPTION:
GATCAGATTC ACTGTTTCAT CATATTTATT GTAATATATT TTTTGTTTTG TAAATATGTT 60
ACAACAAAAT GTGATTGGTC TAAAATATTT GTAATGTATA TTAAAAGGTT CAAAAATAAA 120

SEQ ID NO:4686
SEQUENCE LENGTH:53
SEQUENCE TYPE:nucleic acid
TOPOLOGY:linear
CLONE:HUMGS05561
SEQUENCE DESCRIPTION:
GATCAGAGGA GTTCAGGGAG ACATACATAA AAATGTTAAT CACGGTTTTC AAA 53

SEQ ID NO:4687
SEQUENCE LENGTH:363
SEQUENCE TYPE:nucleic acid
TOPOLOGY:linear
CLONE:HUMGS05562
SEQUENCE DESCRIPTION:
GATCGCCAAG TGCAGGCACG GCATCGCATC CCCAGGTGTC ATCCTGTGAC CCAGGAGGGG 60
AAGATGACAC ACCCAATTTA GGTAATGCTG CACTTCTNGT TATAATTGGT TATTGGAAAA 120
TGTGCCTTTA TTATCAGAAC AGTGGTGTGC GCATTTCTGA TTATGGAGCT AACTTTTGGA 180
TATCTGACTT AGATACTAAA AACATTTGGG TGTTGCAGTT TTGCACTTGT AGATACAACT 240
TAAAAGCATG AAACTGGTGA TTTAATTAGA TGTTTTCTAT AAAATGCACC TTGTTACATT 300
TCTGTGAAAG GGGTCTTACC AGCGTGGAAT AACNTGGCTC AAGGGTTTGA GGGGANCANC 360
NGN 363

SEQ ID NO:4688
SEQUENCE LENGTH:109
SEQUENCE TYPE:nucleic acid
TOPOLOGY:linear
CLONE:HUMGS05563
SEQUENCE DESCRIPTION:
GATCCACGTG TACTGGCACC TCAGAAGACC AAATCATGGN CTGTACAAGT CTCTATACAA 60
TGTCTTTATC CCTGTGGGCA GNANGCAATG ATGATAATGN CAANCAGGN 109

SEQ ID NO:4689
SEQUENCE LENGTH:369
SEQUENCE TYPE:nucleic acid
TOPOLOGY:linear
CLONE:HUMGS05564
SEQUENCE DESCRIPTION:
GATCAGAAAG CATTCAGTCC AGTCAGAATT CAAGGTGGGA AAAGTAGGTG TATTTCTGAN 60

CCATTTGGGA AGGAAAATCA TTTTTCTGAA CANCACTACC TGCATTTCTG TTTCTGCAGA 120
CAATCAAAAA ACAAACCANT AAAANTAAGT AAGTGAANGA AAGGCCANTT AGCATTATTC 180
ATTTGCCTTA GGTTTACAGG GTCACAGATT CATTAGCTTA ATTACATCCT CATTTTGGNT 240
GCCAAAGAAT GGAGCAAGAG CANTCAGTAT TGCTANCTGG CTCTTGCTTC TTCTTCCTGG 300
GTTTGTTTTG GTTTTNCAGA GAAACAGGGA AGNGAGGGCA TTNGCNNAGT ATAATTGGTN 360
TATAGGAGN 369

SEQ ID NO:4690
SEQUENCE LENGTH:70
SEQUENCE TYPE:nucleic acid
TOPOLOGY:linear
CLONE:HUMGS05565
SEQUENCE DESCRIPTION:
GATCATAAAT GTACTTGCTG AATTCAATCA TTTTTAACAA GCCAATAAAG TTTGATAATT 60
CATCTCCAAA 70

SEQ ID NO:4691
SEQUENCE LENGTH:293
SEQUENCE TYPE:nucleic acid
TOPOLOGY:linear
CLONE:HUMGS05566
SEQUENCE DESCRIPTION:
GATCCTCAAG GCCTTGCTCT NCCCCGTGGA GACGCTTGCT CGGATGAGCT CAGGAAACAG 60
TACCGGCTGC GTGGCAGGTC TGGGTGTTGT GTGCGAGGAC GTGGCCTTTG CACACCGCTG 120
TGTTCTCAGA GGTCCTTAGG AGATATTTTT TTTTGTCTTA GGGGGACTGT NTTAAGTTCA 180
GACAAATCAN GCTGGGTGTG GAGAGAGTCT GAAATACGTC AGTGAAGTAA GTAGCAGTGA 240
GCGATTGTGA ATTTGNANTN TAAATGGAAA ACCGGGTTTT TACCGGNGTT TTN 293

SEQ ID NO:4692
SEQUENCE LENGTH:251
SEQUENCE TYPE:nucleic acid
TOPOLOGY:linear
CLONE:HUMGS05567
SEQUENCE DESCRIPTION:
GATCAAACTC TTCATTGAAG TACTCATNAG GCGTGATGGC TGTGGGGTTG TTTGCAGTAG 60
CACATTNCGT GGTGAGGTCC TGCTGAGCCA GNACTCAGGA GAGTCAGAAA TGCCAAANNN 120
TGTTCACAAG TGAAGGCCTG TGGCACTCAG TGTCTGAGGT GGACGTGTTA ACCAGCTAAA 180
GACCGGGAAG TTCTATTCAC TAACTTTTTT GCATGGAACA AGAAAATCTA TGAGGATATC 240
ACCAAACTAA A 251

SEQ ID NO:4693
SEQUENCE LENGTH:97
SEQUENCE TYPE:nucleic acid
TOPOLOGY:linear
CLONE:HUMGS05568

SEQUENCE DESCRIPTION:
GATCCATCTT GCTTTTGCCT TATATAAAGA CTACAGTTAT GGAAGTNTGG AAAACTGTGG 60
CTTCTCAATA AATATTCAGA TGTCCTAAGA NTATAAA 97

SEQ ID NO:4694
SEQUENCE LENGTH:106
SEQUENCE TYPE:nucleic acid
TOPOLOGY:linear
CLONE:HUMGS05570
SEQUENCE DESCRIPTION:
GATCTCACAA AGTACAAAAC GAATGCCTTT CTTTTCTTGT TTATAATGGT CACTCACTGT 60
GTTTGGTTAC TGTCAAGAAA TCAATAAATG TGTTTAACAA GTTAAA 106

SEQ ID NO:4695
SEQUENCE LENGTH:106
SEQUENCE TYPE:nucleic acid
TOPOLOGY:linear
CLONE:HUMGS05571
SEQUENCE DESCRIPTION:
GATCAGTNGA TATTAAGATG AGATTACAAA TTTTNTTAAG TTCAGCCATT ATTACTTTTG 60
GTATCCCAGA ACATGACAAA TTATGAATAA AACAAGTATA CATAAA 106

SEQ ID NO:4696
SEQUENCE LENGTH:149
SEQUENCE TYPE:nucleic acid
TOPOLOGY:linear
CLONE:HUMGS05572
SEQUENCE DESCRIPTION:
GATCCATAAA ATTTTATTAA TCAAGATATC ATATTAAAGT ATTATTATAT TAATTTATGA 60
CACCAAATTT TTAAACCAAT TTTATGTTTT TCCTTATGTA TCATTATAAA CCCTATTGCA 120
TTTTATAAGT AATANANTAT TTTNAGAAA 149

SEQ ID NO:4697
SEQUENCE LENGTH:356
SEQUENCE TYPE:nucleic acid
TOPOLOGY:linear
CLONE:HUMGS05573
SEQUENCE DESCRIPTION:
GATCGCGACG GCTGGTTTGC ATCGCCAGCT TCTATATATT ACGGCCTTTT TTTTTGCTGG 60
ATATTATCTT GTAAAACGTG AAGACTACCT GTATGCTGTG AGGGACCGTG AAATGTTTGG 120
ATATATGAAA TTACATCCAG AGGATTTTCC TGANGAAGAT AAGAAAACAT ATGGTGAAAT 180
TTTTGAAAAA TNCCATCCAA TACGTTGAAG TCTTCAAAAT GCTTGCTCCA GTTTCACTGA 240
TACCTGCTGT TTCTGAATTT GNTGGGGCAT GTTTCTTATG GCANTTGGAG GCTNATTGNT 300
AATNTGTATG TTGGCACCTT GTNATTAAAA TACGGGGCCA TGNNANAAAA AAAAAA 356

SEQ ID NO:4698
SEQUENCE LENGTH:190
SEQUENCE TYPE:nucleic acid
TOPOLOGY:linear
CLONE:HUMGS05574
SEQUENCE DESCRIPTION:
GATCCACCTG CTTTGGCCTC CCAAATGTTG TGTCTTTTTA AAGTATCTAC AGTAACCTTG 60
TATCAACTTA GTTTGTCAGT CTATTAATAC TAAATTTAGC TCCTTCAAAG CAGTTGGAAC 120
TATGTGCTAC ATAAATTTCA GTTTCACCCA AGGGAAGAAG TGAAATTAGT GAATAGACAG 180
TTACAGCAAA 190

SEQ ID NO:4699
SEQUENCE LENGTH:81
SEQUENCE TYPE:nucleic acid
TOPOLOGY:linear
CLONE:HUMGS05575
SEQUENCE DESCRIPTION:
GATCCGNTCC CAGACGGCCT TGGACTGCTT GCATTTCCCC GGAGAAAAAG GGGTTAATAA 60
ATGGGCCATC CTTTCCTGAA A 81

SEQ ID NO:4700
SEQUENCE LENGTH:89
SEQUENCE TYPE:nucleic acid
TOPOLOGY:linear
CLONE:HUMGS05576
SEQUENCE DESCRIPTION:
GATCACTGTG GGGTCAAGAA TGTCTTACAT GTTTTATTCA TCATTCTTGA NGGAAGAAAT 60
AATTCAAACC TTGGAATTAA AAAGTCAAA 89

SEQ ID NO:4701
SEQUENCE LENGTH:311
SEQUENCE TYPE:nucleic acid
TOPOLOGY:linear
CLONE:HUMGS05577
SEQUENCE DESCRIPTION:
GATCTTGGCA AGAAACCCTA ATCTCCCTCC AGAAACAGTG GACTCTCTAA AAAATATCCT 60
GACTTCTAAT AACATTGATG TNANGAAAAT GACGGTCACA GACCAGGTGA ACTGCCCCAA 120
GCTCTCGTAA CCAGGTTCTA CAGGGAGGCT GCNNNNACTC CATGTTACTT CTGCTTCGCT 180
TTCCCCTACC CCACCCNCCG GCATAANGNC ANACCAATCA ACCACGACAA AGGNAGTTGA 240
CCTAAACATG TAACCATGCC CTACCCTGTT ACCTTGNTAG CTGCAAAGTA AACTTGTNGC 300
TGNCCTGCAA A 311

SEQ ID NO:4702
SEQUENCE LENGTH:94
SEQUENCE TYPE:nucleic acid

TOPOLOGY:linear
CLONE:HUMGS05578
SEQUENCE DESCRIPTION:
GATCTCTCTT GCACGGGCCT TGCCAAGGCC CAGGAGGGAC TTGGGCAGTA TGTTCATGTG 60
GTCATATGTT TTTGTAAAAA ATTGTGAAAG TAAA 94

SEQ ID NO:4703
SEQUENCE LENGTH:248
SEQUENCE TYPE:nucleic acid
TOPOLOGY:linear
CLONE:HUMGS05579
SEQUENCE DESCRIPTION:
GATCCGAATC CGACTGTGGG GGGCGGGCTG GGAGGTGGGA GCCGCGTCTC AGGCCCGGCC 60
GTTATCAAGG CCCCTCCGCC CCCGAACCCT GGGGAGCTGG ACCAGGAGGT GGAGGCTCAG 120
GGGACCCCAT GGGGACAGGC AGAGCTGGTC TCCTCCCAGC AGACGGAGCC AGGACGGGCA 180
CAAGAGTCTT GGAGGTTTGC GTGTTTCTGC TAGAATTAAA AAGTTAAATT TAAAAATGAA 240
AATGNAAA 248

SEQ ID NO:4704
SEQUENCE LENGTH:66
SEQUENCE TYPE:nucleic acid
TOPOLOGY:linear
CLONE:HUMGS05580
SEQUENCE DESCRIPTION:
GATCAGCAAA GTGAGACAAA AGAAGATACT TCCCCAAAGA AGAAAAAGAA AAAATTGAGG 60
CAGAAA 66

SEQ ID NO:4705
SEQUENCE LENGTH:65
SEQUENCE TYPE:nucleic acid
TOPOLOGY:linear
CLONE:HUMGS05581
SEQUENCE DESCRIPTION:
GATCGGGTCT CGGAAGGGAA GTAGCCATCA CACCATTAAA AAGCCTGTGG ACCTTTAAAG 60
GNAAA 65

SEQ ID NO:4706
SEQUENCE LENGTH:308
SEQUENCE TYPE:nucleic acid
TOPOLOGY:linear
CLONE:HUMGS05582
SEQUENCE DESCRIPTION:
GATCTTCTTA GGACATCTTT TTTAAAAAGC TGTTTAGTAT CATTTTGTGT ATATTGTTGA 60
AATGCTTTTT CATCAATAGC AGTCANCATT TTATCCTTTC TTTTTATATT CATAATGTTA 120
TTTAAGTGTC ATTGATGTAC TGTATTGACT TGGGGTTTGC TTATTTGTTA CTTAACATGT 180

GTACATGCAT GAAAGCATTT TTNGTTGTTC CCTGATAGTT ACATTTCAAC CTTGGGATTT 240
TNCCAAATNA CTTAAGANGT TTAATGTCAG TNAAAGATTT TNTTACCCTC NTTTTGGGAA 300
CATCAATN 308

SEQ ID NO:4707
SEQUENCE LENGTH:323
SEQUENCE TYPE:nucleic acid
TOPOLOGY:linear
CLONE:HUMGS05583
SEQUENCE DESCRIPTION:
GATCCGGAAG ACCACGGCCA GCCAGGTGTA CGAGACATTG CTCACCTACA GTGACGTCGT 60
GGGCGCGGAT GTGCTGGACG AGGTGGTGAC TGTGCTCAGT GACACTGCGT GGGACGCAGA 120
GCTTGCAGTG GTGAGAGAGC AGCGCAACCG TCTGTGTGAC CTTCTGGGCG TACCCAGGCC 180
CCAGCTGGTG CCCCAGCCTG GTGCNTGCTG AAGCCAGTCC TTGGAGCCCA TANCTCACCC 240
CTGCCTGGTG AGGATGTCTT GTTTCCTGAG GGTAGGCCGG TTGTGGAAAA GCCTCANNAC 300
NAGTGNTGCC TNNAGGCTGN GAN 323

SEQ ID NO:4708
SEQUENCE LENGTH:302
SEQUENCE TYPE:nucleic acid
TOPOLOGY:linear
CLONE:HUMGS05585
SEQUENCE DESCRIPTION:
GATCTTCCTT TGGAACCAGG CACATTTGGC CCCTTCTCAG TGACTGCACT GTGGAACTCT 60
TCTTAAGAAA ATATTGAAAA CAGCTTAATG CTTTCATATA GTGACCGACA TTTAGTTGAA 120
AACTACTGCT GCATAGCAAA TATTGTGACT CTTCATGTGT CCACAGGAGC TCTTGTGTGG 180
GTTTAAAGCT ATGAAGTGTA TTCACATTGT GAAGTTTTAA TTATCTTTAT TGAAATTAAT 240
TGTGTAAAAA TGGTATGTGC TCTATTAGGT ATTCAGTTTG TATGTGAATT CTATAAAGAA 300
TN 302

SEQ ID NO:4709
SEQUENCE LENGTH:302
SEQUENCE TYPE:nucleic acid
TOPOLOGY:linear
CLONE:HUMGS05586
SEQUENCE DESCRIPTION:
GATCCCTCTC AGCTACTGGG AGCTGCTTTG TGCCTTGGAA GCCTCAAGCT GAAATGGATG 60
TCAGCCTCAA GTTTAACTCC AAGAGGGTTC ACCTTGTTTC TTTGAACAAA ATGTGCTGCC 120
TGTTGCTTCC CTTTGCCAGG AGTGGTGTTC CCTTTTCCAC AGGTCTGGAC CACAGTGTCT 180
CTAGCTCCCG CTCCTGGATA CTTGGTTACT GTGTGTTTCG GGAAAAAAGG ATNCACAACA 240
TGAATATNCA CTAAGCTGTA ACAGGGGCCT GCTGCANCTA TAGTATTTCC TGAAAAAGTA 300
TN 302

SEQ ID NO:4710
SEQUENCE LENGTH:161

SEQUENCE TYPE:nucleic acid
TOPOLOGY:linear
CLONE:HUMGS05587
SEQUENCE DESCRIPTION:
GATCTTGCCT CTTTCCTCCT TCATGAAAGC AGCACACATT GTGTTAACTT ATGTCTCTTG 60
TTAAATNAGC TTAATGTCTT TGTGTTTTGT CCAAAACTGT ATTGNNNNAA TATTGTTTAA 120
TGCAAATNAA GGAATGCAAT AAAGAGTAAA TATACTTGAA A 161

SEQ ID NO:4711
SEQUENCE LENGTH:120
SEQUENCE TYPE:nucleic acid
TOPOLOGY:linear
CLONE:HUMGS05588
SEQUENCE DESCRIPTION:
GATCAGAGCG TGGGAGGAGG TGGGGGTGGA CGTCCATCCG GTGAACAGTG AAGGCGTTTG 60
TGAGGTCTTT CTGGTCCCAG CATGAAATAA AGCCTTGGCC TGGGGGCCGC TTCATTCAAA 120

SEQ ID NO:4712
SEQUENCE LENGTH:297
SEQUENCE TYPE:nucleic acid
TOPOLOGY:linear
CLONE:HUMGS05589
SEQUENCE DESCRIPTION:
GATCTGGCAG TACCCAGCCC TTGTGTGTGT GCGTTANTCA GCACCTGCCC ACACTGCGAG 60
CCCCCGTAGG ATGTGCCTTG TCCTTCCCTG TTTCAGCACT TAACACACTA CCTGGTACAG 120
AGTATGTAGT GGGCATCTGT TGAATGAATG CTTTTCCCAG TAGCAGTGTA TTCATACAAT 180
ATTAATATAA TTGTCCCCTG GCTTACAGAT ANAAATGAAA GCATCAAGTG CCCAGTTGAG 240
TGAGACCCAG GTGTTCTTCC TNCACCCCTT AGTGGTTCCC CTGGGGGCAG NTCTTTN 297

SEQ ID NO:4713
SEQUENCE LENGTH:67
SEQUENCE TYPE:nucleic acid
TOPOLOGY:linear
CLONE:HUMGS05590
SEQUENCE DESCRIPTION:
GATCTCTGCG CTAGGTCGTT TACCGCTATT TATTTTTATG GGGAAAATAA AACTCTCAAA 60
AATGAAA 67

SEQ ID NO:4714
SEQUENCE LENGTH:50
SEQUENCE TYPE:nucleic acid
TOPOLOGY:linear
CLONE:HUMGS05591
SEQUENCE DESCRIPTION:
GATCACTAGA TGATTCTAAC ATCGAAATAA ACCTCTTTTT ATATGGCAAA 50

SEQ ID NO:4715
SEQUENCE LENGTH:233
SEQUENCE TYPE:nucleic acid
TOPOLOGY:linear
CLONE:HUMGS05593
SEQUENCE DESCRIPTION:
GATCTGGTGC GTGTGGCCCT GTGGGAGTCC ACTTTCCTCT CTCTCTCTCT CTCTGTNCCA 60
AGTGTGTGTG CAATGTTCCG TTCATCTGAG GAGTCCAAAA TATCGAGTGA ATTCAAAATC 120
ATTTTTNTTT TCCTCCTTNT CAATGTGATG GAATGAACAA AAAGGAAAAA ATTCAAAAAA 180
CCCAGTTTGT TTTAAAAATA AATAAATANA GCAAATGTGC CANTTAGCGT AAA 233

SEQ ID NO:4716
SEQUENCE LENGTH:282
SEQUENCE TYPE:nucleic acid
TOPOLOGY:linear
CLONE:HUMGS05595
SEQUENCE DESCRIPTION:
GATCTTGCCT TGAGGAAGAC CCAGGCCATG TTCCCAAAGG NCAGCGGGGG CCCTGGATTG 60
TAATGCAGCC TCGGGACAGG GCTGAGGCCT GCGGGGGAAG ACCTATACCC CACGACTGGG 120
CCTGGNTTCA CCTCACCCTA ATCCCCCGGG AGGAGCTGAC TGATGCAAAA AGCTGAGGNN 180
GCCTGCTGGG NNGTGGCTGT TTTNATGNCC CAGCCCCGNN AAGTTGGGGA GTGTTTGTGG 240
GGGTNCAGAG CCCTNCCCCA GNCAGGAGAG AACCTTCCTN TN 282

SEQ ID NO:4717
SEQUENCE LENGTH:80
SEQUENCE TYPE:nucleic acid
TOPOLOGY:linear
CLONE:HUMGS05598
SEQUENCE DESCRIPTION:
GATCTAGGAC TAATTCAAGG GTCTAGATTT ACCTCCAAAC TTTGTTTATC TACAAAAAAT 60
AAAACTCTAT CTTCTTTAAA 80

SEQ ID NO:4718
SEQUENCE LENGTH:204
SEQUENCE TYPE:nucleic acid
TOPOLOGY:linear
CLONE:HUMGS05599
SEQUENCE DESCRIPTION:
GATCCTTCCC CTCTCAGACA CCCCTCTTTC AGCTTAGAAT TTGGGGAACC TATAGAGAAG 60
TCACAGAATC CCATGAAAGG GAATGGGGCA GGAGCCAGGG GTGTTTTTNC CCAGTGCAGG 120
GTTGTGTCTT TGTGTCTCTG TCTCTGTGAC TGTAAATCCT GCCCTGCCCC ACTCCCAATA 180
AAAGCTTTGG TGTATAGGCT CAAA 204

SEQ ID NO:4719

SEQUENCE LENGTH:83
SEQUENCE TYPE:nucleic acid
TOPOLOGY:linear
CLONE:HUMGS05600
SEQUENCE DESCRIPTION:
GATCAGTTTG TTTCTTTCTG TGCTGGTAAC AATAAGCGTC GCACAGACAT GGTTTCAGGT 60
AANTAAATCT ATTCTATGAT AAA 83

SEQ ID NO:4720
SEQUENCE LENGTH:289
SEQUENCE TYPE:nucleic acid
TOPOLOGY:linear
CLONE:HUMGS05601
SEQUENCE DESCRIPTION:
GATCTCTGGA TTGTGTTATG TCAGTGTTGG TAGGTTAGGA ACTAGATTTC CCAGAATCCA 60
TTCCATTTGT NATTCCATGA TACAATTGAC CAGTAACCTA TCTTACATGA GATTCGGAAG 120
TAAGTTAAGA AGGCATTAGT CATGGTTTGG AAGCACCATA CAGGGAGACA GCTGTGTGAA 180
TACAGGCTGT ATGGACACTT GCTTCCATCC CATTNNCCTG CTTCTTTGGG TTGCCAATCA 240
AGTGTATCCT CAAAACGACT TGACTTTAAT TTTCTCGGAG GTGATAGTN 289

SEQ ID NO:4721
SEQUENCE LENGTH:235
SEQUENCE TYPE:nucleic acid
TOPOLOGY:linear
CLONE:HUMGS05602
SEQUENCE DESCRIPTION:
GATCAAGTCT TACATGCCCA TTCAGCTTCT AGGCCCCCCT CACCTCCCTG CCCTCATTCA 60
CAAGTGGCCC TGAGACACGT GAACACCTCC CTNCTATGCA TCACAAACCT TCTCCACCGA 120
GCTTTGGTGC TTTNGCCTCT GGNACACCTA ACTAGCATTG GCAGAGGAGA GTCTACACTC 180
TNTTNCTNAT TCAGGGTNGA TGCTTTAAGA AATNCTGNCT CTTGTGNANG CAGAN 235

SEQ ID NO:4722
SEQUENCE LENGTH:276
SEQUENCE TYPE:nucleic acid
TOPOLOGY:linear
CLONE:HUMGS05603
SEQUENCE DESCRIPTION:
GATCGTCAGC ATCATCGGAG GCATCAGTTC CTTCTTCATC TTCATCTTCC CAGGTTTGTG 60
CCTCATCTGT GCAATGGGTG TCGAGCCTAT AGGACCAAGA GTCAAGTGCT GCCTGGAGGT 120
CTGGGGAGTG GTCTCTGTGC TGGTCGGCAC CTTNATCTTT GGGCAGAGCA CGGCGGCAGG 180
GTCTGGGAGA TGTTCTGATG GGCAGCCAGT GCCGGGCAGG AAGGGGCCCT CCGGGGGCTG 240
ACCCTACGTG GCTGCTGTAT GCAGNNGGGA GANCAN 276

SEQ ID NO:4723
SEQUENCE LENGTH:278

SEQUENCE TYPE:nucleic acid
TOPOLOGY:linear
CLONE:HUMGS05604
SEQUENCE DESCRIPTION:
GATCAAGGTC ATAGAAAGTG AAGATTATGG CCAACAGTTA GAAATCGTAT GTCTGATTGA 60
CCCGGGCTGC TTCCGAGAAA TTGATGAGCT AATAAAAAAG GAAACTAAAG GCAAAGGTTC 120
TTTGGAAGTA CTCAATCTGA AAGATGTAGA AGAAGGAGAT GAGAAATTTG AATGACACCC 180
ATCAATCTCT TCACCTCTAA AACACTAAAG TGTTTCCGTT TCCGACGGCA CTGTTTCATG 240
TCTGTGGTCT GCCAAATACT TGCTTAANCT ATTTGGAN 278

SEQ ID NO:4724
SEQUENCE LENGTH:280
SEQUENCE TYPE:nucleic acid
TOPOLOGY:linear
CLONE:HUMGS05606
SEQUENCE DESCRIPTION:
GATCTGGAAT GCNACCGGAG CACTTGCTCT GAGGAATCCC AGGGTGACTC TGTCGGGGAA 60
GAATCCGGTC ACAGCCTCCC CTCAGAGACA GGCCTCANTT CGAGGGCAGC CCATTATCTG 120
TCGCAGCATC TGCCATGTCC CTNAGACTGC GGGAGGCAGG GNATGCATGG GTGTCCCCAT 180
CTGTCCCTGG TGAGAAGCAA GGCTAGCTCT GCCTTCCACA TGCTTNTNAG GATGCCAAGA 240
GGCCTAGGAA CCCAGNAACC CCCTTGNGAG GAGCTGTGNN 280

SEQ ID NO:4725
SEQUENCE LENGTH:253
SEQUENCE TYPE:nucleic acid
TOPOLOGY:linear
CLONE:HUMGS05607
SEQUENCE DESCRIPTION:
GATCTGCATT CACCTTAGGG CTTAGCCTCT NATTTTATGA ATTTTCAGGC TGTGATTNGT 60
GTTGGATGGC TAAGNTCTAA GATAATGCAA ATAGCCCCAA TCTTTAAATA TAGCGGTGCT 120
AAGTTGAACA NGTAACACAN TACAGAAAAT GCTGATATGA ATACTTAGTA ATAATACAAA 180
AGTTCCTGCT AAATTATATA TGTGTATCAC TGCCTGNCNN GANACCCCAC TTNNCTTTTC 240
TAATCCAGCN CAN 253

SEQ ID NO:4726
SEQUENCE LENGTH:301
SEQUENCE TYPE:nucleic acid
TOPOLOGY:linear
CLONE:HUMGS05608
SEQUENCE DESCRIPTION:
GATCCAGATA GCCTAGGGTG AGGGTGACAG AGGGACAGTG GGCTATGCCA CTAGGCCCTG 60
GTCTGGCTTT GGAAAGACCT NTGAGGGGAA ACCTTCACCC AGCACCCATG CCCCACTCTG 120
CTGAGGCCAG AGGAAGGGAG GCCTGAGGGG CAGATTNGTT CATGCCTGGG GTGGAGGCTA 180
AGCCTGGACA CAGTCAGGGC GGGGCTGGCC AGCTGTGCGA GAACACAAGC CACGCCTGCN 240
ATGGTGCTGC ACCCCTGGGT GTCCCTGCCT TGGCCCTGCT CTGGTCACTT CAAACATNGT 300

N 301

SEQ ID NO:4727
SEQUENCE LENGTH:295
SEQUENCE TYPE:nucleic acid
TOPOLOGY:linear
CLONE:HUMGS05610
SEQUENCE DESCRIPTION:
GATCATAAAA CAATAAAAGT AGTTTTCTAA TGTTGCTCAT AAACTGGGAC TGAAAATCAT 60
TTCAAATCCA GGAGACAGTC CAAAAATGGT TATTGAGCAN TGGCCACATA TTGGAATAAA 120
AATTTTNATT TTCAGTCTGT GTTTTGGGAA AACAGCATTT CATTGCATAG GTGGATAAGT 180
NCTAGAATAT TGGCTAANCA TATNGTCATT TGCTATAATG CATATGCATT NCAGAAGTTT 240
TCTNGCTCCT GANGTATNAT GGGGGGTTTT CTAGCTCCCN TATNGCNAAA ANTTN 295

SEQ ID NO:4728
SEQUENCE LENGTH:99
SEQUENCE TYPE:nucleic acid
TOPOLOGY:linear
CLONE:HUMGS05612
SEQUENCE DESCRIPTION:
GATCACTTGA GCCAGAGGTT AAGTCTGCAG TGAGCTGTGN TTGTACCACT GCTCTCCAGC 60
ATGNGCAACA GAGCCGGACC CTGTCTCACC AAAGNCAAA 99

SEQ ID NO:4729
SEQUENCE LENGTH:329
SEQUENCE TYPE:nucleic acid
TOPOLOGY:linear
CLONE:HUMGS05613
SEQUENCE DESCRIPTION:
GATCCATTGT CGGGAGCTTG TGGCACATCA CCTTTCTACT CTGCAGTCTT CCCTGCCTCT 60
NAATTCCGTT TATGTCTACC GTCCCCTCAA GCACACCCTG GTGACCTGTG ACAAAGGAGT 120
GTTCAGATTA CATCCCTCCT CTGTCCCAGG CCCAGACTTC TCCAAGGACA ACAGCAAGCC 180
AGAAGTGCCA GTCAGAGGTT NAGTAGCCTT TTACCATCAT CTCCCAGNTG CCAGTGGGTG 240
CAAGCAGACC TCTACTAAAC GNAAAGTAGA GGGAAATGGN AGTGGATGAC TTNTGTGATG 300
GANTCAAACG GCTCTATAAT GGANGGTTN 329

SEQ ID NO:4730
SEQUENCE LENGTH:140
SEQUENCE TYPE:nucleic acid
TOPOLOGY:linear
CLONE:HUMGS05614
SEQUENCE DESCRIPTION:
GATCCTGCCC TTTCCTGGGT NGGTGGGNGG TATAAGCACT GCCACCAGAA CTGTTGGGTG 60
CTAGCTTCAA AGCCCAACAC AAGAAATACT ACCAATTATG AAATTACAAC AACAANTNTA 120
AATGACATAA TTTTTTGAAA 140

SEQ ID NO:4731
SEQUENCE LENGTH:190
SEQUENCE TYPE:nucleic acid
TOPOLOGY:linear
CLONE:HUMGS05615
SEQUENCE DESCRIPTION:
GATCCATAGG ACTACCGCAG CCCGGACTCA CCAACTTGCC ACATGTTCTA GGTTTCAGCA 60
ACAAGACTGC CAGGTGGTTG GGTTCTGCCT TTAGCCTGGA CCAAAGGGAA GTGAGGCCCA 120
AGGAGCTTAC CCAAGCTGTG GCAGCCGTCC CAGGCCACCC CCATGGAAGC AATAAAGCTC 180
TTCCCTGAAA 190

SEQ ID NO:4732
SEQUENCE LENGTH:159
SEQUENCE TYPE:nucleic acid
TOPOLOGY:linear
CLONE:HUMGS05616
SEQUENCE DESCRIPTION:
GATCCCAGCC AGGAAGGCTG GGGGCCCTAC TGTTTGTCCC CTCTGGGCTG GGGTGGGGGN 60
AGGGAGGAGG TTCCGTCAGC AGCTGGCAGT AGCCCTCCTC TCTGGCTGCC CCACTGGCCA 120
CATCTCTGGC CTGCTAGATT AAAGCTGTAA AGACATAAA 159

SEQ ID NO:4733
SEQUENCE LENGTH:295
SEQUENCE TYPE:nucleic acid
TOPOLOGY:linear
CLONE:HUMGS05618
SEQUENCE DESCRIPTION:
GATCACAGAC AAGCTGGGCC TGCATTCCCT TCGTCACCGT AACTGGTACA TTCAGGCCAN 60
CTGTGCCACC AGCGGGGACG GGCTGTACGA AGGCCTGGAC TGGCTGGCCA ATCAGCTCAA 120
AAACAAGAAG TGAAAGCCAG ACAGCCCTAA CAAAGCACCC CACCCACCCC TGACATACCT 180
ACTGTNACCC TGCCCCAGTC CTACCCCTTC CTCTCCATGC AAGTNTGGCC AGGGCCCTGG 240
GTATCATGTC CACATGCCCA GCAAGAGCCT TGCCTNCCNT TGCCTGCNCT GCTTN 295

SEQ ID NO:4734
SEQUENCE LENGTH:295
SEQUENCE TYPE:nucleic acid
TOPOLOGY:linear
CLONE:HUMGS05619
SEQUENCE DESCRIPTION:
GATCCACCCA CCTNGGCCTC CCAAAGTGCT AGTATTATGG GCGTGAACCA CCATGCCCAG 60
CCGAAAAGCT TTTGAGGGGC TGACTTCAAT CCATGTAGGA AAGTAAAATG GAAGGAAATT 120
GGGTGCATTT CTAGGACTTT NCTAACATAT GTCTATAATA TAGTGTTTAG GTTCTTTTTT 180
TTTTCAGGAN TNCATTTGGA AATTCAAANC ANTTGGCAAN CTTNGTATTA ATGTGTTAGG 240
TGCAGGNGGN CATTGGTATT CTGGGCACCT TCCTAATATG GCTTTACAAT CTNNN 295

SEQ ID NO:4735
SEQUENCE LENGTH:259
SEQUENCE TYPE:nucleic acid
TOPOLOGY:linear
CLONE:HUMGS05621
SEQUENCE DESCRIPTION:
GATCTCAGCA ACAGCTGGGT AATAAGACTA GCATAGCTCA AACTATCCTG CCAAACGCTC 60
TCATCTGATT TTNCCTCCCT TCTCCCCCAA CCTCCAATCA CCCTGAGTCA CCTGTAAATN 120
CATTTGTNAT TCAAAGCGGA ATAACANGTT GTCCCTAGCA NNNCCGCTGA GCGCTTTATA 180
ATTTTNTGGT GTATTTTTGT NAGTAGGTAG CAGAGGCGGA NGTATTTTTN GGTGTAATTC 240
TTGANATTTT CTGACAGGN 259

SEQ ID NO:4736
SEQUENCE LENGTH:232
SEQUENCE TYPE:nucleic acid
TOPOLOGY:linear
CLONE:HUMGS05622
SEQUENCE DESCRIPTION:
GATCACTGCA TGTTATGATG GCTTGTAAGT GCGTTGTTAA GACTTTTNTT TCAGTGTTTG 60
TNTCCCAGTA TTTGAACCTA ATTTAAAGAA AAAGACGTTT CCAAGTTGTA TTTATTAAAT 120
GTNTTTTNCC TTACCTTTTG TNCTGCTACT TTNCTAATCT CATTAGCTTA GCTGTGTTTG 180
TNCATAGGTT ATATNTGGTA ATAAATNTAT AGAGTGTTNG TTGTCATCGA AA 232

SEQ ID NO:4737
SEQUENCE LENGTH:138
SEQUENCE TYPE:nucleic acid
TOPOLOGY:linear
CLONE:HUMGS05623
SEQUENCE DESCRIPTION:
GATCTGTAAG TTTATTTGCT CAATGTACGA CAGCTACATA ATGACTCACA TTCATGATAT 60
TCCATCACTG AGGAAACTGC TAAAGATGGT CCGTGTGTGA AATAATTCCT TAGAGAAACA 120
CGGAGCTGGA AAAATAAA 138

SEQ ID NO:4738
SEQUENCE LENGTH:135
SEQUENCE TYPE:nucleic acid
TOPOLOGY:linear
CLONE:HUMGS05624
SEQUENCE DESCRIPTION:
GATCCAACTG GACAACGTGT GGGATGGACC TGGAAACAAG CACCTCCCCA AACACATCAC 60
CACTCCCTAG GGCGGGGCCT GTGCATGCTC TCGCATGACA TCTCCATGCT GGTTTCTGCA 120
TAGCATAAAT GGAAA 135

SEQ ID NO:4739

SEQUENCE LENGTH:64
SEQUENCE TYPE:nucleic acid
TOPOLOGY:linear
CLONE:HUMGS05625
SEQUENCE DESCRIPTION:
GATCGATGTT AAACGTCACA GCAGTACTTT GCTCAATAAA GGTCATATTG GAAACATAGT 60
CAAA 64

SEQ ID NO:4740
SEQUENCE LENGTH:117
SEQUENCE TYPE:nucleic acid
TOPOLOGY:linear
CLONE:HUMGS05627
SEQUENCE DESCRIPTION:
GATCTGTGGT AGGGTATTGA GGGATGAGGG AGAAAAACGA CCTAATTCAN GTAGGACTNG 60
CAACGGCTCC CACCCTCCCT GGGGACANGG NTTAATAAAA ATTGACATCA CAAGAAA 117

SEQ ID NO:4741
SEQUENCE LENGTH:244
SEQUENCE TYPE:nucleic acid
TOPOLOGY:linear
CLONE:HUMGS05628
SEQUENCE DESCRIPTION:
GATCAGAAGG GCTGCGTGTA GCACCCTTAT GGTCTTGCAG AACACACCAA GAAACCCGAC 60
GGCTTCCATA TTTNCTGACC CTACAAACAG GTGCCTAATA CTGATGTGGA GAAAGCTCAA 120
GAGGGAATCA TCTGGCCTGA GCCTACCATG AGGCTGTTTG TGGTNCTGGC AGAAAAGCAA 180
CAACTCGCTC CCCTTCCCAT GTATCAGTGA AAACCATTAT GCAAATAAAG AGCTGGGCCC 240
CAAA 244

SEQ ID NO:4742
SEQUENCE LENGTH:209
SEQUENCE TYPE:nucleic acid
TOPOLOGY:linear
CLONE:HUMGS05629
SEQUENCE DESCRIPTION:
GATCTTTTTT CCTCCTTCTG TTTATNTTTT TGTTTGTTTT ATTTATAGTC TGATTTAAAA 60
CAATCAGATT CAAGTTGGTT AATTTTAGTT ATGTAACAAC CTGACATGAT GGNGGAAAAC 120
AACCTTTAAA GGGATTGTNT CTATGGTTTG ATTCACTTAG AAATTTTATT TNCTTATAAC 180
TTAAGTGCAA TNGAANGNGT TTTTNCAAA 209

SEQ ID NO:4743
SEQUENCE LENGTH:265
SEQUENCE TYPE:nucleic acid
TOPOLOGY:linear
CLONE:HUMGS05630

SEQUENCE DESCRIPTION:
GATCATATAC TGTATATATA AAANNNTTGA GATGGTAGAA ACATGTATGA NTGTACTAAG 60
TAGTATTCCA CTGTACTCAT TCATAAGGTA GGTTTTCTTA CAAAACTCAC ACCAGGTACT 120
TAAAGATGTG CTCTGCTTTT TTCCAACTAC GGAGTGTCAC TGCTTTCTAG GTCAGTCCCT 180
GCAGACTCTT CTCAACTCTT TCCCTATAGG AAACTTACTC CGCGTCCTGC CCCCACCTCC 240
TAAATAAATA ANGGAATCGG CGAAA 265

SEQ ID NO:4744
SEQUENCE LENGTH:177
SEQUENCE TYPE:nucleic acid
TOPOLOGY:linear
CLONE:HUMGS05631
SEQUENCE DESCRIPTION:
GATCCCTAAC ATACTGTACT ACTTGCTTNT ACAATGTGTT AGCAGAAACC AGTGGGTTAT 60
AATGTAGAAT GATGTGCTTT CTGCCCAAGT GGTAATTCAT CTNGGTTTGC TATGTTAAAA 120
CTGTAAATAC AACAGAACAT TAATAAATAT CTCTGGTGTA GCACCTTTTA CTGTAAA 177

SEQ ID NO:4745
SEQUENCE LENGTH:377
SEQUENCE TYPE:nucleic acid
TOPOLOGY:linear
CLONE:HUMGS05632
SEQUENCE DESCRIPTION:
GATCAGCCGA AGAAAGCAGA AGCTCTGGAG GCTGCCATCG AGAACCTCAA TNAAGCCAAG 60
AACTATTTTG CAAAGNTTGA CTGCAAAGAG CGCATCAGGG NCGTCGTTTA CTTCCAGGCC 120
AGACTCTACC ATACCCTGGG GAAGACCCAG GAGAGGAACC GGTGTGCGAT GCTCTTCCGG 180
CAGCTGCATC AGGAGCTGCC CTCTCATGGG GTACCCTTGA TAAACCATCT CTAGAGAGGA 240
CATCCCTGCT GGGCTGCTGT GCAGAGTATA AGATTTTGGA CTTGTTCATG TCCCCTCTCT 300
CCCTATAAGG GGTGTATTTG TGACACCCTA TCTNGTCANT AAACAGCATT TCTGATTAAA 360
AANAANAAAA AAANAAN 377

SEQ ID NO:4746
SEQUENCE LENGTH:89
SEQUENCE TYPE:nucleic acid
TOPOLOGY:linear
CLONE:HUMGS05634
SEQUENCE DESCRIPTION:
GATCCTGAAG TTTTTGGTCA ATAGGCTCTG TCTTCGTGAG AGACGGGCTG AGAGTCAGAA 60
ATAAATCAAC CATTTNTGGT TTATTCAAA 89

SEQ ID NO:4747
SEQUENCE LENGTH:55
SEQUENCE TYPE:nucleic acid
TOPOLOGY:linear
CLONE:HUMGS05635

SEQUENCE DESCRIPTION:
GATCTTTCTT CTCCCAGCTA AGAGTTCTTC AATAAATTTA AGAAATACCT GGAAA 55

SEQ ID NO:4748
SEQUENCE LENGTH:244
SEQUENCE TYPE:nucleic acid
TOPOLOGY:linear
CLONE:HUMGS05636
SEQUENCE DESCRIPTION:
GATCAGCAGG GACCAAACCA TTGCCACATC CCAAGGNTGA ACAAGCATGG CTGAGCACCA 60
GAGTNTGCAC CAAGTNTGAN TTTAGGCCTG CCAAGTGGAT TTACACCCAG CACGTCCCAA 120
ATTTGGGTGA GTGCATGCCA ACCCTGTAAA CATGTAGGGG TGGACAGGTC AACAGACAAG 180
GTGACCCCAG CNTCCCGGCA AACTTGATTA ACACATACTG GNCACAGCAA TTANNNNGAN 240
NTGN 244

SEQ ID NO:4749
SEQUENCE LENGTH:184
SEQUENCE TYPE:nucleic acid
TOPOLOGY:linear
CLONE:HUMGS05637
SEQUENCE DESCRIPTION:
GATCGCAGGG CCNTAGGTGC CTGGACCCAG GGTGTGCCAG CCCGTCTCTN TGCAGTCCCT 60
GGAAGGGCGC TGAGAAAGGC ACCAGCTCCT TGGACCCCAC CTCCCATGCT CTCACTCTCA 120
TCCCCGTTCT CTTGTCCACA CAGCTCTTCC AATAAAGGTG TTTCTNTTCC TCCTTCTCCT 180
CAAA 184

SEQ ID NO:4750
SEQUENCE LENGTH:282
SEQUENCE TYPE:nucleic acid
TOPOLOGY:linear
CLONE:HUMGS05639
SEQUENCE DESCRIPTION:
GATCCTAGTT GCCTTTGTGT ATATTTACTG CCTGCTTGAG TGTTTCTATG TGTGGGTTTT 60
CCCTGTATCT TGTAGAAATG TTGGGGTGTT TTCCTCTGCC ATATGGCTCG TGGCCTGCGA 120
GCCAACTATT TCAGNTGTGT TTNACCTTCA TTTTTGATGA GGTGATTTAA ANNTNGTTTC 180
ACTTTGTGTA GTGAATTCCA CAGTAGTTTT CTGANTGTNG TTAAAAATGA CTTAACATAT 240
TACACAGATA TTCAATAAAA ANGTTTTATT TCCTGTTGNA AA 282

SEQ ID NO:4751
SEQUENCE LENGTH:65
SEQUENCE TYPE:nucleic acid
TOPOLOGY:linear
CLONE:HUMGS05640
SEQUENCE DESCRIPTION:
GATCCCATTG TGTATAAGTT AATATGTGAT AACTATTGAA TCTTGTACAA AAACAAAAAT 60

TGAAA 65

SEQ ID NO:4752
SEQUENCE LENGTH:159
SEQUENCE TYPE:nucleic acid
TOPOLOGY:linear
CLONE:HUMGS05642
SEQUENCE DESCRIPTION:
GATCCATCCG CGGTGTTGCC TGAATCAACC GCTCATCCCT CGGATTGCTG AGCAGGATTC 60
ACAGGGTGGA GGCACCACAA TGTATCTGTG TCCCCAGAAA GAATACTTGC AGTTTCCAGT 120
TGGACACTTT ATCAATAAAA TCACTATAAA CATCTGAAA 159

SEQ ID NO:4753
SEQUENCE LENGTH:352
SEQUENCE TYPE:nucleic acid
TOPOLOGY:linear
CLONE:HUMGS05643
SEQUENCE DESCRIPTION:
GATCTTGAGT TGCAGTCCAC AGCATTCACA TTCTCAGGAT AAACATGCTT GTCTCCATAT 60
ATACTATCTG TGCCTGTAAT GAATGAAAAA TCCATCCACT CTGCTGCCAT TCACAGCCTA 120
ATCTTCTGGA GTAGTCCAAA CAATGTTTGG AAAAACATGG GACTGTATGT CATTACTATG 180
ACTGATAGCA GAATAATAAT GCATCTNACT TCTATATAGN GATAGNCTTA CATAGGTTAC 240
CCTTGANATT CATTAGTNTG TCATANGTTT TAGGAAAGGT AGGACCCGGN AAGNNGTTCT 300
ANTTAGTTGT CTAAATNTTT TNCAGTGNGC CAAGNNATTC ACCATGANAN AN 352

SEQ ID NO:4754
SEQUENCE LENGTH:297
SEQUENCE TYPE:nucleic acid
TOPOLOGY:linear
CLONE:HUMGS05644
SEQUENCE DESCRIPTION:
GATCGGCTCA AAGCCTGTCA GCAGAGGGAA GGACAGAACT ACCAGCAGAA CTGTATCAAG 60
GAAGTGGAGC AGTTCACCCA GGTGGCCAAG GCCTACCAGG ACCGCTATCA GGACCTGGGG 120
GCCTACAGTT CTGCCAGGAA GTGCCTGGCC AAACAGAGGC AGAGGATGCT GCAAGAGAGA 180
AAAGCTGCAA AAGAGGCCGN CGCTGCCACC TNCTGAGGCA GCTGTGGGTG CCCCTGCTGT 240
GTGGCTCTGT ATNACTTGTT TGCTTGAAAT NTAAAAGGCC TNGCAAACCC TGGGAAA 297

SEQ ID NO:4755
SEQUENCE LENGTH:126
SEQUENCE TYPE:nucleic acid
TOPOLOGY:linear
CLONE:HUMGS05645
SEQUENCE DESCRIPTION:
GATCCCAGGC CTGTGGCTAG CAGCACTGGG GACAGGAATG GCTGGTCCCT TGAGGAGGTC 60
GTGACAGGCT CAGCCTNGTN GTCTNGAGGG GACTCGGAAA TAAATTGTAG CAGCTTTCCT 120

GCCAAA 126

SEQ ID NO:4756
SEQUENCE LENGTH:317
SEQUENCE TYPE:nucleic acid
TOPOLOGY:linear
CLONE:HUMGS05646
SEQUENCE DESCRIPTION:
GATCTAAAAA GTCAAAAATC TGAAATTTAA TAATATGAGA CTTACACTGA NTATAATGTT 60
CATTTAGAAG TTGCTGTGGT CCACTTCATT TATANGGAAC AAATATTTTT ACAGTACACT 120
ATAGCAACAG CAAAAGCCCT CTCTCACCCT GATAGGAATG GGTTTGCTGG GTGTCTAGAA 180
GTTAGATTCC TGCTGAATAG ANTTAGCCAT CCTTAAAAGA TNTTAATCCA ATACTGAACT 240
GGTTTATAAA ANGCTTTCTC TATTGTANTG NACTGTAAGT AGTGAAATTC TGTATATACT 300
GCTATTNGCT GNCTGTN 317

SEQ ID NO:4757
SEQUENCE LENGTH:228
SEQUENCE TYPE:nucleic acid
TOPOLOGY:linear
CLONE:HUMGS05647
SEQUENCE DESCRIPTION:
GATCAGTAAT CCTATTAAAG AAGCGGAAGA AGATACCTCA GAAAGGTCGG AAGAAAAAAG 60
GTTTTCGGAG GCGGCGGTGA TTATGGGTGT ACATATTTGT ATATTTTTTG TCATCCTGAG 120
ATACTTCTAA TTTCATTGTA TATAGGTGGT TTTCCCTGGA ATTCATTAAT TGTTTGCTTT 180
GGACATGTGG AAAGAGCCTT ACTAATAAAA TTGATTTTAC TTATGAAA 228

SEQ ID NO:4758
SEQUENCE LENGTH:312
SEQUENCE TYPE:nucleic acid
TOPOLOGY:linear
CLONE:HUMGS05648
SEQUENCE DESCRIPTION:
GATCAGTTCT GAAACAGTTT CTTTCTGAAA CAGAGAAAAT GTCCCCTGAA GACAGAGCAA 60
AATGCTTTGA AAAGAATGAG GCCATACAGG CAGCCCATGA TGCCGTGGCA CAGGAAGGCC 120
AATGTCGGGT AGATGACAAG GTGAATTTCC ATTTTATTCT GTTTAACAAC GTGGATGGCC 180
ACCTCTATGA ACTTGATGGA CGAATGCCTT TTCCGGTGAA CCATGGCGCC AGTTCAGAGG 240
ACACCCTGCT GAAGGACGCT GCCAAGGTCT GCAGAGAATT CACCGAGCGT GAGCAAGGAG 300
AAGTCCGCTT TN 312

SEQ ID NO:4759
SEQUENCE LENGTH:95
SEQUENCE TYPE:nucleic acid
TOPOLOGY:linear
CLONE:HUMGS05649
SEQUENCE DESCRIPTION:

GATCAAGGTA AGAAACATTG TAAAAAAAAA TTACAAAAGT GCTATTTGTT TCCTAAAAAC 60
AGTGATTTCT ATTAAAAAGG TGTCAGANCT GGAAA 95

SEQ ID NO:4760
SEQUENCE LENGTH:336
SEQUENCE TYPE:nucleic acid
TOPOLOGY:linear
CLONE:HUMGS05650
SEQUENCE DESCRIPTION:
GATCCTNTTT CCANTTNAAA GGAACTGTAA GCTTTTATCT TTTAACCAAC TGAACAATAC 60
ACCAAAAGCA GCCTAGGGAT GAGCATTTCT TTGAAAGCAA TTAGGTTATT CACCTGGTAT 120
TAAAACTATT TACTGTTAAA AAATCTGTGA CTTCATGAAG TTGATTTNTA AAGGCAGCAT 180
CANAAACTGA NANGGAAGGG AAANANTAGG CAGCTTCTCT GCACTTGTTT GGAGCTCCCC 240
AAANCAGGGT GCCATGGGGN AGTGGGCATC AAGNCCGGGC TGCCCTTTCG NGGTCANCCT 300
GTGGCAGTTC AGAGTCANGG TTTTTCCTNC GCTGGN 336

SEQ ID NO:4761
SEQUENCE LENGTH:310
SEQUENCE TYPE:nucleic acid
TOPOLOGY:linear
CLONE:HUMGS05651
SEQUENCE DESCRIPTION:
GATCCAAATT CCCAGGTCGG CGCCTGCATC GTGAATTCAG AAAACAAGAT TGTCGGGATT 60
GGGTACAATG GGATGCCAAA TGGGTGCAGT GATGACGTGT TGCCTTGGAG AAGGACAGCA 120
GAGAATAAGC TGGACACCAA ATACCCGTAC GTGTGCCATG CGGAGCTGAA TGCCATCATG 180
AACAAAAATT CGACCGATGT GAAAGGCTGT AGTATGTATG TTGCCTTGTT CCCTTGTAAT 240
GAATGCGCTA AGCTCATCAT CCAGGCAGGT ATAAAAGAAG TGATTTTCAT GTCTNGATAA 300
ATACCATNTN 310

SEQ ID NO:4762
SEQUENCE LENGTH:364
SEQUENCE TYPE:nucleic acid
TOPOLOGY:linear
CLONE:HUMGS05652
SEQUENCE DESCRIPTION:
GATCCCCTCC AAGGAGCACC CATATNACGC CGCCAAGGAC TCCATCCTGC GCAGGGCCAG 60
GGGCATGTTC ACTGCCGAAG ACCTGCGCTA GGGGACTCCT CATAGCCCTC AGCCCTTCCC 120
TCGTTTCCAG GCCTCTCCCC AGGCTTGCCA TCAGCCTTCT TTACTTTTTG AGCCTCTGAT 180
TTCCAATTCC CTGCTNCNNN CCACTCCATT AAGAGGCTAG GTGAGGCGCT TCTAGGTTGC 240
TGGGGCTCTG CTGGTTAAGG AACAGGAAGC CTGACCATCT CCCTCCACTA CCTCTTCCCT 300
GTGCTGTTAC ACAGTGTCAT TGTTGATGTT AAATTAAAGT CATATTCTTG CTTCTTTCCG 360
NAAA 364

SEQ ID NO:4763
SEQUENCE LENGTH:333

SEQUENCE TYPE:nucleic acid
TOPOLOGY:linear
CLONE:HUMGS05653
SEQUENCE DESCRIPTION:
GATCGTCTGG AAGCAGCAGA GATGTACAGA AAAGCTGCTT GGGAAGCGTA TTTGAGTAGA 60
CTTGGTGTTT GAGTGCTTCA GATACATTTT TCAGATACAA TNTGAAGACA TTGAAGATAT 120
GTGGTCCTCC TGAAAGTCAC TGGCTGGAAA TAATCCAATN ATTCCTGCTT GGATTCTTCC 180
ACAGGGCCTG TGTAAGAATG GGTTCTGGAG TTCTCATGGG NCTTTTAGGA AATATTGAGT 240
AATTTGGTAA NTCACCGNAT TGGTTACTAT ANATAAGGTC CCANTNCNTT AAGGCTTGAT 300
AAGGTGAATN GNGGTGNGNN NTNTGGGGGG AGN 333

SEQ ID NO:4764
SEQUENCE LENGTH:131
SEQUENCE TYPE:nucleic acid
TOPOLOGY:linear
CLONE:HUMGS05654
SEQUENCE DESCRIPTION:
GATCCTCCAA AATTCAAACA AGGAAAAATC TCTCCCAATC TCTCCTTTCC TCCATCCCTT 60
TTCTTTACTT CTCATCCCAT ATATAATATT ATATAANGCA TACGTAATAA AAGACATTCA 120
CTATTATTAA A 131

SEQ ID NO:4765
SEQUENCE LENGTH:271
SEQUENCE TYPE:nucleic acid
TOPOLOGY:linear
CLONE:HUMGS05655
SEQUENCE DESCRIPTION:
GATCTCTTTT GCTATGGCTC ACATTATTTT CATAAAGTTG CTGGCCAGCT TTTACCTCTT 60
GCATATAATC TGTTGAAGAG GAATCTGTTT GCAGAAATTA TTGAGGAGCA TCTGGCAAAC 120
AGAAGTCAAG AGAACATAGA CCAACTTGCT GCATGAGTAA GGTGGCTTTG NTTGGTGTAC 180
AGTATTTCAA AGGACTAGTA TTAANCTTGT GATTTNGGTT TTGTTTTTAA GGGGTACANN 240
NNGTAAGCCA TTTNCTTAAN AACCGTTGAA A 271

SEQ ID NO:4766
SEQUENCE LENGTH:87
SEQUENCE TYPE:nucleic acid
TOPOLOGY:linear
CLONE:HUMGS05656
SEQUENCE DESCRIPTION:
GATCTTTCTC TTGAGTCATT TTATTTTTAT CATGGACTAG TGCGTGCTCC GTGTCCACCC 60
CAATAAAAGG GTCTTTCCTA CTTGAAA 87

SEQ ID NO:4767
SEQUENCE LENGTH:293
SEQUENCE TYPE:nucleic acid

TOPOLOGY:linear
CLONE:HUMGS05657
SEQUENCE DESCRIPTION:
GATCTGGGCA ATTTTGGCCT TGACTCTTTC CTGGCTCCAG AGCTCAAGCT TAGANGCCAG 60
CCCTGCTATT TCCAGCCTCC TGAAGGCTCA GCACGGTGAG GCCTGACATC CTGGGGAAGG 120
GCAACAGGGA GACCTACAGG ATGNTGGCTG CTTGCAGACT GGTCAATGGG GGATGACGGT 180
GGGGAGGTTG NCAGATGTGA GACTTNNGTA GCATTTNGTA CACATGGCCC TGTAGTTGTC 240
CTTTNGAAGA ACATCANTNA AAATATANGG GTNNTTAAAT TNTGGGNAAA AAN 293

SEQ ID NO:4768
SEQUENCE LENGTH:313
SEQUENCE TYPE:nucleic acid
TOPOLOGY:linear
CLONE:HUMGS05658
SEQUENCE DESCRIPTION:
GATCTTGCTA GGGAAGGCCA CCTTGTATGC CGTGCTGGTC AGTGCCCTCG TGCTGATGGC 60
CATGGTCAAG AGAAAGGATT CCAGAGGCTA GCTCCAAAAC CATCCCAGGT CATTCTTCAT 120
CCTCACCCAG GATTCTCCTG TACCTGCTCC CAATCTGTGT TCCTAAAAGT GATTCTNACT 180
CTGCTTCTCA TCTCCTACTT ACATGAATAC TTNTNTCNGT TTTTNTGTTT CCCTGAAGAT 240
TGAGCTCCCA ACCCCCAAGT ACGAAATAGG CTAAACCAAT AAAAAANTGT GTGGTTGGGG 300
CCTGGGGNGC AAA 313

SEQ ID NO:4769
SEQUENCE LENGTH:279
SEQUENCE TYPE:nucleic acid
TOPOLOGY:linear
CLONE:HUMGS05659
SEQUENCE DESCRIPTION:
GATCCCCTCA TCACCCCTGT GCCCACTTCG GAGAACCCNT TCCGGGAGAA GAAGTTCTTC 60
TGTGCTCTCC TCTGAGCTCC CCTGTCCCTT CTCACAACTN CTCCCTTTTC CCTCTCCTGG 120
GCCCTTCCTT AGGTCAGTAA TTGTTGTGAG CCCCTTAGGC TCCTTGCATC CCATCCCTAA 180
CCCTTGCCTG ACCATGTGAG GTTATCTGAA GCACAAGNNC CACCCTNANG NNGNNTNTCG 240
ACCCGATTTC CTACCACCTT TTTTGGGCCG ANCCNAAGN 279

SEQ ID NO:4770
SEQUENCE LENGTH:203
SEQUENCE TYPE:nucleic acid
TOPOLOGY:linear
CLONE:HUMGS05660
SEQUENCE DESCRIPTION:
GATCCATGTA ATCTGACGTC ATCTTGTCTC GAAGTCTCTT TTTTTGGCCC AGGCCTTGAA 60
GAATACACTG TGACTTAAGA AGCCTTACCA CGCAGTAACT AAAGCTTTAG GATGACTGTN 120
TTCGAGGAGT GCCGTGTGTT GCATGCAGCT ACCCGTAGGA AGACTTCGCG CATATCACTA 180
ATAAACCTGA AGTCGTGATG AAA 203

SEQ ID NO:4771
SEQUENCE LENGTH:112
SEQUENCE TYPE:nucleic acid
TOPOLOGY:linear
CLONE:HUMGS05661
SEQUENCE DESCRIPTION:
GATCGTTGTG CTTTNTTTNC TGTGTTGGTT GCCAGTTTAT AGTGCCAACA CGTGGCGCGC 60
CTTTAATGGC CCGNGTGCAC ACCNAGCACT CTCGGGTGCT CCNATCTCCT TN 112

SEQ ID NO:4772
SEQUENCE LENGTH:291
SEQUENCE TYPE:nucleic acid
TOPOLOGY:linear
CLONE:HUMGS05662
SEQUENCE DESCRIPTION:
GATCTGGGAG GTGGAAGCCA GGCCAGAGGA CTTGGGGAAA ATNAGATGGA GGAAGGAAAA 60
AGGGAGAAGC TGAGCCACAG CTTAACTCCT ACAGAGTGAA ATNAAAACGG GCTGAAAATA 120
CCACCCCAGG AGAGGACCTC GNCCCANGCA AGCCAGTGAG CAGCCCTGCC AGACTACTGN 180
CAGNCTGNGA AACCCAGANG CTGGGTAGTC ATGTGGGGCT TGCCTTCTTT TGCCAAAACG 240
NCTGGGGGAA NCCAAAAATN GGGCCCCACC CTTNNTGTTT CTTTCCTTNG N 291

SEQ ID NO:4773
SEQUENCE LENGTH:133
SEQUENCE TYPE:nucleic acid
TOPOLOGY:linear
CLONE:HUMGS05665
SEQUENCE DESCRIPTION:
GATCCCAGGC CTNTGGCTAG CAGCACTGAG GACAAGGAAA TGGNTGGTCC CCTTGAGTAG 60
GTCGTTACAA GGCTCAATGC CTGGTNGTCT TGGAGGGTAC TCGGAAATAA ATTGTAGCAG 120
CTTTCCTGCC AAA 133

SEQ ID NO:4774
SEQUENCE LENGTH:316
SEQUENCE TYPE:nucleic acid
TOPOLOGY:linear
CLONE:HUMGS05667
SEQUENCE DESCRIPTION:
GATCACCCAG CAGGAGTCGT GGCAGAACGG AGCATCAGCC AGACCCTGTT GTGGGCGTTG 60
TCATCAAGGG AGCTTGAATG GAGGGTCTGG TGTCAGATAC AGCCGACTCC AGCCCCAGCT 120
CATCCCCCAT GATGCTGTGT GACCCACTGG GCACTCTGGT GAGGGAGCTT TCCAGACATC 180
AACAGCCCAC TCTGCTTCCC TTTCTGAGTC CCCTGTCCAG CACTGCCTAG TGTTGGAGGG 240
TAGACCAAGG CTGTGCATGA TTCACCCCNT CCTTCCATCC TGGAGCTGGC AGTGAATAAA 300
AGCCCGTATT TACAAA 316

SEQ ID NO:4775

SEQUENCE LENGTH:113
SEQUENCE TYPE:nucleic acid
TOPOLOGY:linear
CLONE:HUMGS05668
SEQUENCE DESCRIPTION:
GATCTCCAGA GACCCAAGTT TAGGTTCTCA TAGTGTATTT GAAGTAGTTA TACTCCTGGC 60
TTAAGTAGTT TAGTGCCTGG GAGAATCCAT TACTGAAAAG CATTTAACTT AAA 113

SEQ ID NO:4776
SEQUENCE LENGTH:157
SEQUENCE TYPE:nucleic acid
TOPOLOGY:linear
CLONE:HUMGS05669
SEQUENCE DESCRIPTION:
GATCTAACAA AAATGAAACA TGAAATGCAG TAGCAACCAC TAAAAAAAAA AATTCAAGGN 60
CATCTAACTN TTTCTCTCAC TTTNGCCCTT TGTTTATCCT TCCCTGTGAT TAGATAANCA 120
NAATAAAAAN CAAAATGCTG TATTTCTCTN CTTACGN 157

SEQ ID NO:4777
SEQUENCE LENGTH:214
SEQUENCE TYPE:nucleic acid
TOPOLOGY:linear
CLONE:HUMGS05671
SEQUENCE DESCRIPTION:
GATCACTTAG AGAAACAGGC CGTTAGGAAT CCATTCTCAC TGTGTTCGCT CTAAACAAAA 60
CAGTGGTAGG TTANTGTGTT CAGAAGTCGC TGTCCTTACT ACTNTTGCGG AAGTATGGAA 120
GTCACANCTA CACAGNGANT TCTCAGCCTA CAAATTGTGN CTATACATNT CTAAGCCTTG 180
TTTGCAGNAT AAACAGGGCA TTTNGCAAAC TAAA 214

SEQ ID NO:4778
SEQUENCE LENGTH:141
SEQUENCE TYPE:nucleic acid
TOPOLOGY:linear
CLONE:HUMGS05673
SEQUENCE DESCRIPTION:
GATCAGTTTN CAGCAGCAAG TACAAAAGGA GAAGAGGAAC ATCCGTTGAA TNAGTGTGTT 60
TTGTACATAA CTTCAGATAC TTGTNAACAT GCCTTATATT TGTCCAACAA CTGTCAGANT 120
AAAGAACATT CTAAAATNAA A 141

SEQ ID NO:4779
SEQUENCE LENGTH:154
SEQUENCE TYPE:nucleic acid
TOPOLOGY:linear
CLONE:HUMGS05674
SEQUENCE DESCRIPTION:

GATCTTGTGA AAACACNGTN TTNTCGACTA AAAATTCCAC TNTCTTCATC TNGTTGTGAA 60
GCTAAAAAGA GGGACTGTTA AATACAATGT ATGATACCAT GGCAAAAATC TTTCCTGAAT 120
TGTCTTTNGT AAAAGTATTA TTGAATTTTN CAAN 154

SEQ ID NO:4780
SEQUENCE LENGTH:236
SEQUENCE TYPE:nucleic acid
TOPOLOGY:linear
CLONE:HUMGS05676
SEQUENCE DESCRIPTION:
GATCAAAATA GCTATTGCTA CAACATTTTC GAAAACAAAG TTGGGGCTGT ATTTCTTTAA 60
AAAGATAAGC CTCTAAAAAT GCTTGGCAAA AAAAATATAG TGTTAAAATA GGCCAGTGAT 120
ATTAATGAGA AAATGAAAGT ATGTATCAGG ANTAANGTGA TATTGCATAG GNGTATTGTA 180
TTTTNATGAA TTTNATGCCA GTTGTTTACA TGTACTATAT ATGTNAANTT ANNNNN 236

SEQ ID NO:4781
SEQUENCE LENGTH:168
SEQUENCE TYPE:nucleic acid
TOPOLOGY:linear
CLONE:HUMGS05679
SEQUENCE DESCRIPTION:
GATCTCAGTT GCACGTGCAA ACAAATCCAA ATTCGAACAT TCTTAGGAGG TTTTTNTTAA 60
GGCATGACAG ATGATNTAAA CAAATAAATA GCATGCAACA AAANGTCTTT ATTGNAAGAA 120
GTGGAAGTTA TCTTAATGCC ACGGNTCACC ANCAATTNAA AAGTANAN 168

SEQ ID NO:4782
SEQUENCE LENGTH:79
SEQUENCE TYPE:nucleic acid
TOPOLOGY:linear
CLONE:HUMGS05680
SEQUENCE DESCRIPTION:
GATCAGTGAA CACTAACATT TTGGGGACAA CTTAGTCAAT TGGTTTTCCT TACAACAAAA 60
TAAAGTAAAA TGTAGCAAA 79

SEQ ID NO:4783
SEQUENCE LENGTH:107
SEQUENCE TYPE:nucleic acid
TOPOLOGY:linear
CLONE:HUMGS05681
SEQUENCE DESCRIPTION:
GATCACCATT CAATTTGAGT TTCCAGGGGG AAGTGCATGT ATAATGNAAT GATAATGGAC 60
TTCAATGANG GATGTCATCA ATNATGNACA TATAGATNGC CTTATAN 107

SEQ ID NO:4784
SEQUENCE LENGTH:319

SEQUENCE TYPE:nucleic acid
TOPOLOGY:linear
CLONE:HUMGS05682
SEQUENCE DESCRIPTION:
GATCTAATAA GACACGTAAT AGATTAGAAG TAGGGAAATT AATGAAAAAG CAGAATTAAG 60
GATGACTTCT CACTGGCTTG AAATNCTAAA TGGATGACGG TGCTGTTTAG TGAGTTGGGG 120
AAGACTGGGC GTGGTGATGG GTGTAGAAAT TGAGAAAGGA AAATCAAGAN TTTCCAGAAC 180
TTTCAGAGCA ATGTTAAATA CTTTCCTTGA TAGTGGGTCA GTCATCATTT ATAAGTGCAT 240
TTCTAAAAAT NTTGCTTAAT TTTNTTTTTG AGCTTTGTAT AAGTGGAATT TTGTGTGTAC 300
TGATTATNTT GCTCAATAN 319

SEQ ID NO:4785
SEQUENCE LENGTH:78
SEQUENCE TYPE:nucleic acid
TOPOLOGY:linear
CLONE:HUMGS05683
SEQUENCE DESCRIPTION:
GATCTTTCAT CTGTGATTCT TATAAGAGCT TTGTCTTCAG AAAAACTAAA AATAAAAGGC 60
ATTGACTTAA ACAGCAAA 78

SEQ ID NO:4786
SEQUENCE LENGTH:51
SEQUENCE TYPE:nucleic acid
TOPOLOGY:linear
CLONE:HUMGS05684
SEQUENCE DESCRIPTION:
GATCAGTTGT AAGTTCATTT CTTTACAAAT AAAAGCCTCT TCCATTTGAA A 51

SEQ ID NO:4787
SEQUENCE LENGTH:288
SEQUENCE TYPE:nucleic acid
TOPOLOGY:linear
CLONE:HUMGS05685
SEQUENCE DESCRIPTION:
GATCTTTTCC ATCTGAGGGT ACAGGAAGTA CCAGGACCTG TTTCAGTTTT TNAATCCTGC 60
AAGCACATTC CAAGACTGGC CTGAAACTGC ATGAGCAACA TCACTCGAAA TAATTTTTTT 120
TTTCAAANGC ACCTTAACAA CCANTTGCGA TGCTGTCCTG TTCCTTTTTA CTCACACCCT 180
TCTNTCCTTT CTCGTCCCNA TGCTCCACCA NCTCAGTGCT NTGTGCTGTA TGCGTGTGCT 240
CTCTGTTNTN GTATACTCAA TATANGTGNA ATAAACTGGT GTTTTGTN 288

SEQ ID NO:4788
SEQUENCE LENGTH:278
SEQUENCE TYPE:nucleic acid
TOPOLOGY:linear
CLONE:HUMGS05686

SEQUENCE DESCRIPTION:
GATCATGCAT GCAGTCATGT CTCCAGTATT CTGTGAAAAG CCAGGATACG TTAGGATGTG 60
AGTTTAGAAA CTGAGTCCAT TCGGTGCATG GAAATNCACC GCGGAGTTAC ACAANGCTCC 120
TCACTGTAGT CCAGGCGATT GGTNGGCCGC CANCACCCTG CGGGCTTCAC AGGGACGCGT 180
GCTTGTNCTC CAAGNCCCGN AACCCTTACT GTGCACACAN CATCNNACAC ACCTTTGTTT 240
TTGAAATTAA AATGTTNATT AGTTNAAAAA AGGGNGGN 278

SEQ ID NO:4789
SEQUENCE LENGTH:337
SEQUENCE TYPE:nucleic acid
TOPOLOGY:linear
CLONE:HUMGS05687
SEQUENCE DESCRIPTION:
GATCCCGGTG TGTGGCCCAG CTTGTNCAGG CCCTGGNATG CTGCATCTCC AGGCAACTAT 60
GCACTTTCCC GGGGAGAGAA CCAGTATGAG AAGTGGGGGC AGGGCACACA TTCATCTTTG 120
TAGGAAGGTC TGGCCTGGGG TCGGGTGAAG GAGGGCCCAG GTCAGTNCTG GGGTCCCAGT 180
GACCTGCTTT GCCATTNTCC TGGTGCCGNT GCTGCTCCCT GTTTCTGGAG CTGGATGTTN 240
CCNAGCTGGC AGTTGAGCTG CCTGAGCCAA TNTAGTCTAG NCTTTGGTAA CTTGNGTGAA 300
CCATAATAAN GGGGAACCAT TNTNGGCCTT GTGNAAA 337

SEQ ID NO:4790
SEQUENCE LENGTH:251
SEQUENCE TYPE:nucleic acid
TOPOLOGY:linear
CLONE:HUMGS05688
SEQUENCE DESCRIPTION:
GATCACAAGG CTGCCTGCCT CAGTCTTGGA GTCCTGTTGG GTGAATNAGG CAGATGGGAA 60
ANAGCCTCAC CAGCAGCTGC TTTTGGAGCA GGGGTCCAAG GAAGAGAGGG TGGCCTCGAC 120
ATCAAACTGC CTGGATTTTT CTACCACCCT GTTACATCAT AACAACTTCT GAAACACACA 180
CCAGCCCTGA GTTCTGGGCT CATTTGAAGC CTGGAATAGC AATAAATCTT TTTAACTTGC 240
GGACAGTTAA A 251

SEQ ID NO:4791
SEQUENCE LENGTH:316
SEQUENCE TYPE:nucleic acid
TOPOLOGY:linear
CLONE:HUMGS05689
SEQUENCE DESCRIPTION:
GATCGAAGAT GTACAATGTT AAATGTNCAT ACCAGTTAAA CTGCACTTTC CCAAGGGGNC 60
AGCTGGNATC TACTTGAAAT GGGNATCAAG AGACCACCAA ATGTTTACAG TTTCTGTGGC 120
CCGTTTAAGT GTAAAATGCA GAAGTGGCTC TGTCCCCCCA GAGTTAAGGN TTTGCTACAC 180
TGGTCCTCTG GAAGCATTTA NGTAGAAGAA AAGGCTTATA GTCAGNNTCA CTTTTAGTAA 240
TCGTCTNCTG GTCCTTGGNC TNTTCTGGTT CTGTNANTGC TTGTAACCAA TGANTGATGG 300
TGTTGCCAAT CTNAGN 316

SEQ ID NO:4792
SEQUENCE LENGTH:346
SEQUENCE TYPE:nucleic acid
TOPOLOGY:linear
CLONE:HUMGS05690
SEQUENCE DESCRIPTION:
GATCAGTCCT GTCATCCAGC TGCAGCCCAC CTTCACACCC ATGCGGGTCG CACTNGCTGG 60
CACATTCCAC TACTACAGCT GCGAGAGAGC CAAAAAGGCC ATGGGCTACC AGCCACTAGT 120
GACCATGGAT GATGCTATGG AGAGGACCGT GCAGAGCTTT NGCCACCTGC GGAGGGTCAA 180
GTGAGGGNCA CTNGNGGCTG GGCTCTCTNG ACACGTTGCT CAGCCAGTCA CTNCTTCCCC 240
TGNTGGATTG ATGAGNTAAC ATCCTTTNGA ATGGAGTTTN GCTCTTTGCC TGTGACTCCT 300
TCTGCTAGGC AGNGGAGCGC ACCNTACTNT TTNCGTNGCG GTGNGN 346

SEQ ID NO:4793
SEQUENCE LENGTH:187
SEQUENCE TYPE:nucleic acid
TOPOLOGY:linear
CLONE:HUMGS05692
SEQUENCE DESCRIPTION:
GATCCCAGGG ACTCATCCTG TGACTTTGTA ACCACACTCA TTCCTTTCAA ACACAGAGGA 60
GGAATTATTG CTTCATCTGA CTTTATTCAA ATCTAGTTAT GTATTGGCCT AAATTATTTG 120
TGCAAAATTT GAANNNNNNT TTNATTTAAA TATTAGAAAT AGCACAAAGT CATGGNGTTT 180
TTAANAN 187

SEQ ID NO:4794
SEQUENCE LENGTH:228
SEQUENCE TYPE:nucleic acid
TOPOLOGY:linear
CLONE:HUMGS05693
SEQUENCE DESCRIPTION:
GATCTCAAGA GCCCTGTCAG TAGAGTAGAA GTCTCTTCCA GTTTGCTTTG CCCTTCTTTC 60
TACCCTGCTG GGGAAAGTAC AACCTGAATA CCCTTTTCTN ACCAAAGAGA AGCAAAATCT 120
ACCAGGTCAG AATAGTGCCA CTAACGGTTG AGTTTTGACT GCTTGGAACT GGAATCCTTT 180
CAGCAAGACT TCTCTTTGCC TCAAATAAAA AGTGCTTTTG TGAGCAAA 228

SEQ ID NO:4795
SEQUENCE LENGTH:230
SEQUENCE TYPE:nucleic acid
TOPOLOGY:linear
CLONE:HUMGS05694
SEQUENCE DESCRIPTION:
GATCTGCTTA AAGTTTTAAC TTTTATACCT ATCTGAGTGA ATTACAGACA ACCTATCATT 60
NATNCTGCTN CGAGGGTCCC CAGGGCCCTT GTACAACCGA CAGNTCTTAC TTTTAAATGC 120
AATCTCTTGT CTACATACAT NATTTTCTTA ATTNGTTAGC TATTTATAGA AAGCTGCAAT 180
AGAACTGTNT CAACTGTATA ACTATTTACN ATTCANAATA NAANTATTTN 230

SEQ ID NO:4796
SEQUENCE LENGTH:367
SEQUENCE TYPE:nucleic acid
TOPOLOGY:linear
CLONE:HUMGS05695
SEQUENCE DESCRIPTION:
GATCTCATTT GCTGAATGAT TGATTTAAGA GTAAAATTNG CCACTGCGTG CTTTTTGTAT 60
AGTAACCGGT TTTGTTGCAC TNGTCTGTGG CTGAAACACT TACCTGCAGT GGAANGCCGA 120
GCAAGGAGGA GGTAAACATT GGAGATGTTT GTGAAAATAT TACTCTTGCT GTGAGGTTTT 180
AGTTTGTTTT GAATGAGAAA GGGGCTAAGT AACTTAATCC ATCTCAATGT AGTGTTTTAA 240
TAAAAANGGN GCCTAATATT TGAAATGGGG TCTCAGGNCA TGNTCTACAA ATNTGGGGNA 300
NCTATTTTTN ATGCCCCGTG TAATTTTNCA CCATTTTGNN NATGAGTTNA TTGTAGGGGT 360
CANNATN 367

SEQ ID NO:4797
SEQUENCE LENGTH:88
SEQUENCE TYPE:nucleic acid
TOPOLOGY:linear
CLONE:HUMGS05696
SEQUENCE DESCRIPTION:
GATCTTTTCT CAGAGCGTCT CCATGCTATG GTTGCATTTC CGTTTTCTAT GAATNAATTT 60
GCATTCAATA AACAACCAGA CTCAGAAA 88

SEQ ID NO:4798
SEQUENCE LENGTH:239
SEQUENCE TYPE:nucleic acid
TOPOLOGY:linear
CLONE:HUMGS05697
SEQUENCE DESCRIPTION:
GATCACAAGT GCAGCATTCC TTAATTCCTT CTGCTATATG TCACACAGTT GTTATTTGGA 60
GAACCAAGTA TGTATTGCAT GAAAACATTA TGACTTTTTT CTCTTAGTTT AAATAAACTC 120
CAAGGTAACT GGACTTCTAA AGCACCTTTC TGTTTGCCTG ATATCTACTT TAGCAATAAT 180
TTTTTTTACA ACCCTCTGAC TCAACAAAGT AAATAAAAGT ATATTTTATC ACTATTAAA 239

SEQ ID NO:4799
SEQUENCE LENGTH:148
SEQUENCE TYPE:nucleic acid
TOPOLOGY:linear
CLONE:HUMGS05701
SEQUENCE DESCRIPTION:
GATCTGGCCT TATGCCCAGG CCTGGGCCCT CAGAAAGCCC GGAGGCTGTT TGATGTCCTG 60
CACGAGCCCT TCTTGAAAGT ACCCTGATGA CCCCAGCTGN CAAGGAAACC CCCAGTGTAA 120
TANTAAATCG TCCTCCCAGG CCAGGAAA 148

SEQ ID NO:4800
SEQUENCE LENGTH:109
SEQUENCE TYPE:nucleic acid
TOPOLOGY:linear
CLONE:HUMGS05704
SEQUENCE DESCRIPTION:
GATCCTCAAC AGACACCTTC CTTGACTGAA AGTTTTAATC GTGATGCAGA GNCAGTCAGT 60
NATNTNCCGC CATCCACAGG AAATTCAGCA TCTTTATCTC TNCCACTTN 109

SEQ ID NO:4801
SEQUENCE LENGTH:147
SEQUENCE TYPE:nucleic acid
TOPOLOGY:linear
CLONE:HUMGS05707
SEQUENCE DESCRIPTION:
GATCAATTCA ATTAGATTAA AATNAAGAAC CTTGGGGCAG AGTTTGGGTT TTGTAAAATN 60
AAAAGAGTTC TGGAGTTAAG CATCGTGAAC ATGCTTAACA CTACTGAACT GTACACTCAT 120
TTTATAATAA AACTATTAAA TTGTAAA 147

SEQ ID NO:4802
SEQUENCE LENGTH:295
SEQUENCE TYPE:nucleic acid
TOPOLOGY:linear
CLONE:HUMGS05708
SEQUENCE DESCRIPTION:
GATCTAATTT AAAATTCATT GGATTGAAAT NTATTGAATA TAATTCANCT TTTGTAACTA 60
GGTAGACTTT NCTNATAGTT TAGTTTTAAT ATAGTTTAGG GTATTACTGG CTTTTCCCCA 120
GGTGACACAC TAAGGNCTGT TTACATGGCA ATTCCAGCAG CAACAGGNGG CTGGAATTNA 180
GACAAGGAAA GCTCATTTGT TTCTTTTATG AAGGTTAAAG AAGTCTACAG CTTGGGCTGG 240
CCGGTATTTA TTNATTCTGT GGCCAGAGGA TTTTAAATTT NNCNCNAATN TTNTN 295

SEQ ID NO:4803
SEQUENCE LENGTH:52
SEQUENCE TYPE:nucleic acid
TOPOLOGY:linear
CLONE:HUMGS05709
SEQUENCE DESCRIPTION:
GATCATGCCA CTGTCCTCCA GCGTGGCAAC AGAGTGAGAT TCTGTCTCCA AA 52

SEQ ID NO:4804
SEQUENCE LENGTH:374
SEQUENCE TYPE:nucleic acid
TOPOLOGY:linear
CLONE:HUMGS05710
SEQUENCE DESCRIPTION:

GATCAGATGG GGACACACAA CCCCTGGATA TGTTTCATTG TCAGATTTTG TGCTGNNATT 60
TTAAGAATGG AATGGTGGGT ATCTTTCCTT TTTTTAATG TATCTTAACT GTTGCCTGTC 120
AGTGNNNACA AACTAGTGCG TTGACGGCAC CGTGTCCAAG TTTTAGAAC CCTTGTTAGC 180
CAGACCGAGG TGTCCTGGTC ACCGTTTCAC CATCATGCTT TGATGTTCCC CTGTCTTTCC 240
CTCTTCTGCT CTCAAGAGCA AAGGTTAATT TAAGGACAAA GATGAAGTCA CTGTAAACTA 300
ATCTGTCATN GTTTTTACCT NCCTTTTCTT NTTTNAGNGC AGAGGTTAAA AGTAAGTATN 360
AAGCACCCGT GAAA 374

SEQ ID NO:4805
SEQUENCE LENGTH:123
SEQUENCE TYPE:nucleic acid
TOPOLOGY:linear
CLONE:HUMGS05711
SEQUENCE DESCRIPTION:
GATCTGACTG TGGAAACATC CTCTCACTTG CATTCTTTTA ACTTAAAACT ATTTAAGAAC 60
TGATGTTCCG ATTATTGTAT ATATTNNNCT AAAANCCAAA TAAAGCTACC TATGAAAATG 120
AAA 123

SEQ ID NO:4806
SEQUENCE LENGTH:299
SEQUENCE TYPE:nucleic acid
TOPOLOGY:linear
CLONE:HUMGS05714
SEQUENCE DESCRIPTION:
GATCAAACAG GAAATTGAAA GCCACTGNAA CTATGCAGCA TTTCAGTTTT CATTTAAACA 60
CTTAGTTCAG AAACCTTAAA GGATTTGANT ATTTCAAATT GCACACGTCA CTCCAGCATC 120
TCTGTAANAT AATTGGAATG AANATACTTC TTGCACTTAA ACACTGCACA TGCCGTNCTT 180
TGCGGTTNGT CTGCATCTTG AACTTTAATA CCAANTTNNT TTTGCATGCT TTTGTTTCCT 240
ACGNGATAAT GGCATGGTTT CATATGTNAC TACTTTGGAC CAGTGTTTTC ANNATGGNN 299

SEQ ID NO:4807
SEQUENCE LENGTH:204
SEQUENCE TYPE:nucleic acid
TOPOLOGY:linear
CLONE:HUMGS05715
SEQUENCE DESCRIPTION:
GATCTGGACA AATGACAGTA ACTGTTTCTA ACCTTGATTT TCTCATCTNT AAGATGCCAA 60
TTGTAACTCC TAAGGNTACT GAGGATTTTT TAAAATGCGT GTACAGTTCC TGACCAGTGG 120
TTTGTGCCTA ATAACTNATT ACAAATNATT ACCNAGTAAA ANCCTTGCGC CANGTGTGAN 180
AACGTAAAGC TANTTAATCC ATNN 204

SEQ ID NO:4808
SEQUENCE LENGTH:348
SEQUENCE TYPE:nucleic acid
TOPOLOGY:linear

CLONE:HUMGS05716
SEQUENCE DESCRIPTION:
GATCAAGCGA CCTTGAGAGC AGAGNACAAA TCCCAGTGTT GCTGATATAT GGCTTCTNCT 60
TCTCTTGTGT GTGCCTCAGC TCTGAAGNAG TTCCTGAGAA TGGGTTACAG ATGTCATGTT 120
AGCTGGGAGT CTTCCCACAT GTGGCACTTC AAAAGGCAGC ACCACTGGGC GCCTGCACTT 180
ATTTGAAAAT GGNACTTTGG GNGAAGTATC CCTGCTAGTG GCTCTGTAAC TTAACAGATG 240
TCANTTAGGC TTTTGTCATT GTTGCCATCA TATNNANGCT AATGTNTACA TCCTTTTAAA 300
CANNTTGNGT ATGACAATTN CCTCAGGATT TNGGGTAAGN CTTCCCAN 348

SEQ ID NO:4809
SEQUENCE LENGTH:287
SEQUENCE TYPE:nucleic acid
TOPOLOGY:linear
CLONE:HUMGS05718
SEQUENCE DESCRIPTION:
GATCCTCCTG CAGACCACGC CCGTCCTGCC TGTGGCGCCG TCTCCAGGGG CTGCTTCCTC 60
CTGGAAATTG ACGAGGGGTG TCTTGGGCAG AGCTGGCTCT GAGCGCCTCC ATCCAAGGCC 120
AGGTTCTCCG TTAGCTCCTA TGGCCCCACC CTGGGCCCTG GGCTGGAATC AGGAATATTT 180
TCCAAAGAGT GATAGTCTTT TGCTTTTGGC AAAACTCTAC TTAATCCAAT GGGTTTTTCC 240
CTGTACAGTA GATTTTCCAA ATGTAATAAA CTTTANTATA NAGTAAA 287

SEQ ID NO:4810
SEQUENCE LENGTH:342
SEQUENCE TYPE:nucleic acid
TOPOLOGY:linear
CLONE:HUMGS05719
SEQUENCE DESCRIPTION:
GATCACCACC CCGAGCCCTC TNATCCTCTA TGGNTTCACT CCCATCATGA TTTGGAATTA 60
TTTATTTGTG AACTTGATTA GCCGAGATTG TAAATNACAT GAGGTCAGGG ACTGGACTGG 120
GTATGTTTTC TTCTCTGTTG CTTTCCTGGC ATGAAGTACA GTAAATACAG CACCTGATAC 180
ATTATACATG GCCACAAATA TTTGTTGAAT GAATGANTAA AAGCACATTG GGTTCTGACC 240
ATTCAGTGTC CCTGNAANGA ATGCAGTTTT TCAAACTTGT GCNTCACCAT CCATTAGCGG 300
GNGTGTGAAT CCAATTTAGG TNGGTGATGT CTAGCATTNN AN 342

SEQ ID NO:4811
SEQUENCE LENGTH:299
SEQUENCE TYPE:nucleic acid
TOPOLOGY:linear
CLONE:HUMGS05720
SEQUENCE DESCRIPTION:
GATCATTAAC CAGAGTACCT CTACTCTTAG CAAACTCTAG TTTATGACAA GTATTTAAAA 60
TATTTAAAAC AAGCTTATGC AGTTCTTAAG GNCGNAGGTA AATGAGATGT ANCTTAAAAA 120
TAGTATTGGG AAAATGTTGA TAGTTAACAT TAGTGGATTT AGACTAGCCA AATGACATAG 180
TAGGCTCTGA AACATCTTGT CAAGTNTATG TNTTTTGTGC ATGANTTTTT GCTGGAAAGC 240
TGTCTTTCTC TGAAAAACAC ANCGTTCTTA GACTGNNANG CCANTTATAA CNTAANNCN 299

SEQ ID NO:4812
SEQUENCE LENGTH:310
SEQUENCE TYPE:nucleic acid
TOPOLOGY:linear
CLONE:HUMGS05721
SEQUENCE DESCRIPTION:
GATCTTNATG CCCAACATTG GTTATGGAAG CAACAAAAAA ACAAAGCACA TGCTGCCCAG 60
TGGCTTCCGG AAGTNCCTGG TCCACAACGT CAAGGAGCTG GAAGTGCTGC TGATGTGCAA 120
CAAATCTTAC TGTGCCGAGA TTGCTCACAA TNTTNNGNTC CAAGANCCGC AAAGCCATCG 180
TGGAAAGAGC TGCCCAACTG GCCATCAGAG TCACCAACCC CAATGCCAGG CTGCGCAGTG 240
AAGAAAATGA GTAGGCAGCT CATGTGCACG TTTTCTGTTT AANTAANTGT AAAANCTGCC 300
ANANAAANTN 310

SEQ ID NO:4813
SEQUENCE LENGTH:63
SEQUENCE TYPE:nucleic acid
TOPOLOGY:linear
CLONE:HUMGS05722
SEQUENCE DESCRIPTION:
GATCCCTTTT CTTTTGATGG ACCAGAAATN ATGGGTTGTA CAGGGTGCCA GATAGATTGG 60
AAA 63

SEQ ID NO:4814
SEQUENCE LENGTH:127
SEQUENCE TYPE:nucleic acid
TOPOLOGY:linear
CLONE:HUMGS05723
SEQUENCE DESCRIPTION:
GATCCCAGTG CTCTACTGGG GGATGAGAGA AAGGCATTTT ATAGCCTGGG CATAAGTGAA 60
ATCAGCAGAG CTCTGGGTGG ATGTGTAGAA GGCACTTCAA AATGCATAAN CCTGTTACAA 120
TGTTAAA 127

SEQ ID NO:4815
SEQUENCE LENGTH:350
SEQUENCE TYPE:nucleic acid
TOPOLOGY:linear
CLONE:HUMGS05724
SEQUENCE DESCRIPTION:
GATCGAATAC AGATGACCCT GCTGTCGTGT CTTCATTTGT AAAATNCTCC TACAAATGGA 60
GACATTGAAG GATTATGTGT ATGGAAATNT TTACAAAATT CCTTTCTGTA CCTATTCTGT 120
AGATGCCTGT ATAATCGGNC TGGATGTACA GCTATAAATG TGTTGTGAGA GCAAGAGAGA 180
GAGAATGGGT AGAGCCTGCT GTACCTTTTT CCTTTTAAAT TCTTCGAGTT GGTAAATGAG 240
TAAGATATTG GAAACCTTAA TGNAAAGCTG CTGTTCATGN TTTGTGTGTA TAGTGAGCTG 300
CCTGTAGTAT TTNNCTATTG TCTGNTAAAA AAAGNAAANC CTGTAAAACN 350

SEQ ID NO:4816
SEQUENCE LENGTH:178
SEQUENCE TYPE:nucleic acid
TOPOLOGY:linear
CLONE:HUMGS05725
SEQUENCE DESCRIPTION:
GATCTTGAAC AAAGNCACTT AGTTNCCCTG GGCCTTGGTG TCTTTCGTCT ATAAAATATT 60
GTACCAGATT ATCTCTGAAA TCACTNCAAG GTTTAATATA TATANTTATC AATATTTTAA 120
AATTCAAGCA ATATTCACTT TAGANATTAT TATAACATTC TATANTNGTN GGGGGGGN 178

SEQ ID NO:4817
SEQUENCE LENGTH:123
SEQUENCE TYPE:nucleic acid
TOPOLOGY:linear
CLONE:HUMGS05726
SEQUENCE DESCRIPTION:
GATCGAATCG GGACATNATG GGAGCTGACA AATTTATATT CTNTTTCAAT NATTTTAATC 60
CCACTTCACT GTGAGGGGAA GGCCTTTTCA CGGGAACTCT CCAAATATTA TTCAAGTGCC 120
AAA 123

SEQ ID NO:4818
SEQUENCE LENGTH:61
SEQUENCE TYPE:nucleic acid
TOPOLOGY:linear
CLONE:HUMGS05727
SEQUENCE DESCRIPTION:
GATCCCAAAT TTGTCCATAG CTGAAGTCCA CCATAAAGTG GATTTACTTT TTTTCTTTAA 60
A 61

SEQ ID NO:4819
SEQUENCE LENGTH:292
SEQUENCE TYPE:nucleic acid
TOPOLOGY:linear
CLONE:HUMGS05729
SEQUENCE DESCRIPTION:
GATCTATAGA ATTAGATTTN NTTATATCTA AAGAAATATA TATAAATACA TAAACGTAGG 60
TATGTATAGC ATGANCAGAA TACAGTGCTA TTTGACCATT GTCACAAGAC TTATTTATAA 120
AACCTCCTCA AGCTCTTGCC AATATCTTAG TTAAGNTTGC ATCTAAGGTG GGCGGNGGAT 180
GNNGATTGAG GCACTGTCAG GTCATGNTTG ATTACCTGGG TGACAANATT ATGTTTACAC 240
CAAACCCCCA TGNCACACAG TTTACCCATG TGACANNACN TNCACATGTC CN 292

SEQ ID NO:4820
SEQUENCE LENGTH:61
SEQUENCE TYPE:nucleic acid

TOPOLOGY:linear
CLONE:HUMGS05730
SEQUENCE DESCRIPTION:
GATCCCCCAT ATACTCTGCT ACAGCTCCTA TCATGAAAAA TAAAATGTTT GTCTTTGCAA 60
A 61

SEQ ID NO:4821
SEQUENCE LENGTH:140
SEQUENCE TYPE:nucleic acid
TOPOLOGY:linear
CLONE:HUMGS05731
SEQUENCE DESCRIPTION:
GATCCGNTGG GGTGAAAGCA GCCAGCTCAT CCCAGTAACT CACAGGACAC AGCCATCCAG 60
CGGCATCTTT CCTTGTCGAA TGATACTGTA ATNACCTTCC AAAGTNAAGA GTAGCACATT 120
AAAGTNATTT TATTGTTAAA 140

SEQ ID NO:4822
SEQUENCE LENGTH:309
SEQUENCE TYPE:nucleic acid
TOPOLOGY:linear
CLONE:HUMGS05732
SEQUENCE DESCRIPTION:
GATCTCAATG CTGTCTGGGG ACCCTAAGAG TTTNCTNACC TGTNCAGTCT CATCTAACCT 60
TCCAATGTCT GATGTTCCTG CCAAATTCCT GCCTGATTCT GGGTCCGTCC TGACCTCCAA 120
AGGTCAGCTT GGTGCTTGAG GTCTCCCTGC TCTTGGTGGC AGTGGTAGCA GCAACAGCAG 180
CAGCAGCAGC AGCAGCAGCA GCAGCGNCCT CTCCACTTTN CCTTNGCCCN TCTNCTGGGT 240
AGAGAGGCAC TTTCAGGGAC TTTCCTCCAG GNTGGNTCNT CAATATGGGA ATGNGCTNTG 300
NAAGGNTTN 309

SEQ ID NO:4823
SEQUENCE LENGTH:109
SEQUENCE TYPE:nucleic acid
TOPOLOGY:linear
CLONE:HUMGS05733
SEQUENCE DESCRIPTION:
GATCCTCCTG GCGGCAGTAG CCTTGACAAG GGCCACCTCT TCACAGGATG CAGTCTGTCT 60
GTGCACCAAA CTCTTCACCA AATAGAACAC TTGTGTCTCT CTGTGGAAA 109

SEQ ID NO:4824
SEQUENCE LENGTH:233
SEQUENCE TYPE:nucleic acid
TOPOLOGY:linear
CLONE:HUMGS05734
SEQUENCE DESCRIPTION:
GATCCATTTG GNAGGAAATG AGAGGGCTGT NATCAGCTCT NATTAAGAAA GGAGATTTCT 60

TCATGCTTTC GATTCTGCAT GGGGTACAGC CAGTCACCTC ACCAGAGANT GACGGCTGGA 120
GAAGTNAACT CTGTAATACC ATAAATAAGA GTGCTTGTAA TAAAAGNCTG TGCACANGGA 180
TTAATATTTC CCNTCTTAAG TATCAAACGA CCTCTGGANC AAATTNTACC NTN 233

SEQ ID NO:4825
SEQUENCE LENGTH:102
SEQUENCE TYPE:nucleic acid
TOPOLOGY:linear
CLONE:HUMGS05735
SEQUENCE DESCRIPTION:
GATCTGACGT TTTCTACGTA GCTTTTGTAT TTNTTTTTTT TTTAAANTTG AAGGACCACT 60
GATGAAGCCC TNCCANACCC CTCCCGGGTC TAANAAAACG TN 102

SEQ ID NO:4826
SEQUENCE LENGTH:277
SEQUENCE TYPE:nucleic acid
TOPOLOGY:linear
CLONE:HUMGS05736
SEQUENCE DESCRIPTION:
GATCAAGAAA TNTCTCTNCT CCTACATCCA GCTCCTCTAG GGGCAGCCTC CGTCATCCAT 60
GCCCTCCCAG GACCCTCCAC TCACTGCTGT GAGTGCGCCT CACCAGAACC AGTTAAGAGA 120
CAACTATCAA TTNNGGGGAC CCAAATTATA AGGGCCCTGN CCTGTACTGA AGNAAAGGGG 180
NGCACAAGGC CTTAATGGAC ATTGNCTTGT GAAAACGCAN ACATGAATAT GGTTGGAGAG 240
CNTGGCTTAG GAGGGTGTCA TGGGGNAGNA GNGGCTN 277

SEQ ID NO:4827
SEQUENCE LENGTH:98
SEQUENCE TYPE:nucleic acid
TOPOLOGY:linear
CLONE:HUMGS05738
SEQUENCE DESCRIPTION:
GATCCCCTCT CCTGCCCAAG CACTTCACAG CTGGACCCTG CTTCACCCTC ACCCCCCTCC 60
TGGCAATCAA TACAGCTTCA TTATCTGAGT TGCATAAA 98

SEQ ID NO:4828
SEQUENCE LENGTH:81
SEQUENCE TYPE:nucleic acid
TOPOLOGY:linear
CLONE:HUMGS05741
SEQUENCE DESCRIPTION:
GATCTGGGAT TTTNTTTTTA TTTTGAAATG GGAGCTTTTT TGTTTACAAG TTCATTAAAA 60
ACTAAAAACT GTTTCTGTAA A 81

SEQ ID NO:4829
SEQUENCE LENGTH:234

SEQUENCE TYPE:nucleic acid
TOPOLOGY:linear
CLONE:HUMGS05742
SEQUENCE DESCRIPTION:
GATCGATTTC TTTCCTCCAG GTAGAGTTTT CTTTGCTTAT GTTGAATTCC ATTGCCTCTT 60
TTCTCATCAC AGAAGTGATG TTGGAATCGT TTCTTTTGTT TGTCTGATTT ATGGTTTTTT 120
TTAAGTATAA ACAAAAGTTT TTAATTAGCA TTCTGAAAGA NGGAAAGTAA AATGTACANG 180
TTTANTAAAA NGGGGCCTTC CCCTTTAGAC TAANTTTCAG CATGTGCTTT CAAA 234

SEQ ID NO:4830
SEQUENCE LENGTH:130
SEQUENCE TYPE:nucleic acid
TOPOLOGY:linear
CLONE:HUMGS05743
SEQUENCE DESCRIPTION:
GATCCACCCA CCTCAGCCTC CCAAAGTNCT GGGATTACAG NCATGAGCCA CCATGCTCAG 60
NCGTGTGTGT NTGTGTGTAT TATTTATATG TATATGCATA CTATATACAT NTATATATAC 120
ACTATATAAA 130

SEQ ID NO:4831
SEQUENCE LENGTH:103
SEQUENCE TYPE:nucleic acid
TOPOLOGY:linear
CLONE:HUMGS05747
SEQUENCE DESCRIPTION:
GATCTTTAAT CAAGCACTAT GTTAATACTG TAATATCAGA ATACTATGTT GCATTATTTA 60
AAATGTTCAA ATTGAATAGN TTAAAANGTT TTTAAATNCT AAA 103

SEQ ID NO:4832
SEQUENCE LENGTH:325
SEQUENCE TYPE:nucleic acid
TOPOLOGY:linear
CLONE:HUMGS05748
SEQUENCE DESCRIPTION:
GATCCTTGGT GCTGTGTGCC CCACTCCAGT CTGGGGACCC CACTTCANAA GGTAGGGGCC 60
GTGTCCCGCG GTGCTGACTG AGGCCTGCTT CCCCCTCCCC CTNCTGCTGT GCTGGAATTC 120
CACAGGGACC AGGGCCACCG CAGNGGACTG TNTCAGAAGA CTTGATTTTT CCGTCCCTTT 180
TTCTCCACAC TCCACTGACA AACGTCCCCA GCGGTTTCCA CTTGTGGGCT TNAGGTGTTT 240
TCAAGCACAA CCNACCACAA CAAGCAAGTG NATTTTAAGG GGGTTGTGCT TTTTTGTTTN 300
GTGCTAACGT CTNACTAATT TTAAN 325

SEQ ID NO:4833
SEQUENCE LENGTH:212
SEQUENCE TYPE:nucleic acid
TOPOLOGY:linear

CLONE:HUMGS05749
SEQUENCE DESCRIPTION:
GATCTGAAGA GAAGCCAGAG TATAATTTNC TACTATTTTC AATACAAAGN TGTGTTTTCA 60
TTACAATTAG NGGAATATAG GCTTCTGTGA GCTAGCCTGG AAGCAAACAT AATCATTATT 120
GTNCATTGTT TCTGTGNGAN ACTGTAATGC TGTTTCTAAA TATTGACCTA ACANTAAACT 180
CTGNGGAATT CATGCTTGTN ACTGGNTGGA AN 212

SEQ ID NO:4834
SEQUENCE LENGTH:284
SEQUENCE TYPE:nucleic acid
TOPOLOGY:linear
CLONE:HUMGS05751
SEQUENCE DESCRIPTION:
GATCTGGCCT TATGCCCAGG CCTGGGCCCT CAGAAAGTAA GAGCTCTGGG AAAGAACCCA 60
AGGAGTTGGG GGAAGGAGAG AGCCCCAAAT AAACACAACC TGAGACCCCA AAGTTTTAAG 120
GTGNAAAANG NACCAAAGAC CAGNCACAGT GGCTTCCGCC TGTAATCCCA NCATTTTGGG 180
NGGCCAAGGC GGGAGGACTG CTTGAGGCCA GAAGTTGGNG ACCATGCCTG GGCANCGTGG 240
NCACCTCATT TTTACTACAA ATNAAANAAN CTTCGCTGNG TAAA 284

SEQ ID NO:4835
SEQUENCE LENGTH:60
SEQUENCE TYPE:nucleic acid
TOPOLOGY:linear
CLONE:HUMGS05752
SEQUENCE DESCRIPTION:
GATCCTACTG ATGAAATTCC CACTAAAAAG TCAAAGAAGC ATAAAAAGCA CAAAAACAAA 60

SEQ ID NO:4836
SEQUENCE LENGTH:144
SEQUENCE TYPE:nucleic acid
TOPOLOGY:linear
CLONE:HUMGS05754
SEQUENCE DESCRIPTION:
GATCAAAAAA TTGAATGATA AATATGAATG GCTTTTCAAT TCTGTGGACT TTGTACCATT 60
TGGCTTCACC TTGTACTGCA AGATGAATTT GTAAACAAAA CAAAATTGGN CTGTCTGGAA 120
AGCTAAAGTN CTGAAATATG GAAA 144

SEQ ID NO:4837
SEQUENCE LENGTH:138
SEQUENCE TYPE:nucleic acid
TOPOLOGY:linear
CLONE:HUMGS05755
SEQUENCE DESCRIPTION:
GATCACAATG ANGGCCAAGC TTGCNCGCNT AGAGGCCCAG GAGCAGGCCT TCCTGGCTCG 60
TTTCAAAGGC CAGGACCCTG GGGCCCCTCA ACTGCAGTCA GAGAGCAAAG CCCTTAATAA 120

ATATTTNACA TCCTTAAA 138

SEQ ID NO:4838
SEQUENCE LENGTH:84
SEQUENCE TYPE:nucleic acid
TOPOLOGY:linear
CLONE:HUMGS05756
SEQUENCE DESCRIPTION:
GATCTTGTTC ACATCTTGCT CACTGCTATA TTCTCATTGC TGAGCACACA GTTGCAGCTC 60
AATAAATTTT GAATGAACTC CAAA 84

SEQ ID NO:4839
SEQUENCE LENGTH:114
SEQUENCE TYPE:nucleic acid
TOPOLOGY:linear
CLONE:HUMGS05757
SEQUENCE DESCRIPTION:
GATCACCTNA TGGATGTCGC AATAATACAA ACCAGTGTAC CNCTGACCTN NTCATCAAGA 60
GAGCTGGGGT GCTTTGAAGA TAATCCCTAC CCCNCTCCCC CAAATGCAGC TGAN 114

SEQ ID NO:4840
SEQUENCE LENGTH:323
SEQUENCE TYPE:nucleic acid
TOPOLOGY:linear
CLONE:HUMGS05758
SEQUENCE DESCRIPTION:
GATCTGCACT ATNAACTTTG TGAAGAGTTT CTCCTAAAAA CTTTAAGTAA AATGTTAATG 60
GTAGCTTTGA TAACATCAAA TTCTAAGGGA GAAAAAAACA ATATTAAACC GCCCAAGCAG 120
TGTGCCCTAG CAGAGGAAAA TGCAACATCT CGCAAGCGCT GCTGTAACGC CTTCAGGAGT 180
CACTGATTCA GCACTAGTTT CCGGCTGTGA AAACTCATCT TTCATTTTTN CCGTGGNTNG 240
GCGCTTTTNT TANTNGTNGT CCTAATGANA TTTCTGNCAT TGTCATATAC AANCGNTGAA 300
TATCATTAAA ANTTTTTNAN TNN 323

SEQ ID NO:4841
SEQUENCE LENGTH:121
SEQUENCE TYPE:nucleic acid
TOPOLOGY:linear
CLONE:HUMGS05759
SEQUENCE DESCRIPTION:
GATCAAAAAA ACGTTCTTTG AAGAGCTGGC AGTAGAAGAT AAACAGGCTG GGGAAGAAGA 60
GAAAGTNCTC AAGGNGANGG AGCAGCAGCA GCAGCAACAG CAACAGCAGC AGCAANANAN 120
N 121

SEQ ID NO:4842
SEQUENCE LENGTH:351

SEQUENCE TYPE:nucleic acid
TOPOLOGY:linear
CLONE:HUMGS05763
SEQUENCE DESCRIPTION:
GATCTTCACC AGCTGCTCGT GGTGGCTCGG TGTCTGTCTC TCAGTGCTGG TCAGACAACG 60
CTGTCAAGAG ANCGATGGCT GAGAGCAAAG CAGCTAGAGT CTTTAAGAAG AACGAGGCTT 120
CAGCAGCAAA AATGTGTGAA TGGAAATNAA CTTTAAAGAT GTAATACCTA TGAAGAGTAA 180
TGGGCAAACT GTAGCCACAT AATTGTAAAA TTCAGATATT CATTTATACC ACATTGTTTT 240
ATAGGTAATT TCTATCACAA ACCAGTGACA TTTCCTGAAA TCANGCCTGG TAACACCTGA 300
TGTTTATATG ATATTCAGTA AGGACTTTTA CCTTACTGAT TTCATGGNGN N 351

SEQ ID NO:4843
SEQUENCE LENGTH:71
SEQUENCE TYPE:nucleic acid
TOPOLOGY:linear
CLONE:HUMGS05765
SEQUENCE DESCRIPTION:
GATCTGAATT TCCCCATGCT TCCTGGTTTT NATATGTTTC TTGACAAATA AAGCTGATTT 60
AAATCTCTAA A 71

SEQ ID NO:4844
SEQUENCE LENGTH:111
SEQUENCE TYPE:nucleic acid
TOPOLOGY:linear
CLONE:HUMGS05767
SEQUENCE DESCRIPTION:
GATCTTTTTC TTTATCTCCC TTCCCCTTCC TAAGTCCCAT TTCTTGGTCA TAAATATTGC 60
ATTATTCACA CTTTCAAACT GTGTATTTTC TTACAATAAA AAATGATGAA A 111

SEQ ID NO:4845
SEQUENCE LENGTH:202
SEQUENCE TYPE:nucleic acid
TOPOLOGY:linear
CLONE:HUMGS05768
SEQUENCE DESCRIPTION:
GATCTCACCA CCCACCTGGC CTCGCAGGGC GTTCATCTCC TCCTCGTGGT TCTTCTTCAG 60
GTAGGCCAGC TCCTCCTTGA GGTTCTCAAT CTGCATCTCC AGGTCGGCTC TGGCCAGGGT 120
CAGCTCATCC AGCACCCTGC TTCAGTCCCT TCCCCATGCT TCCTTGCCTG ATGACAATAA 180
AGCTTGTTGA CTCAGCTATA AA 202

SEQ ID NO:4846
SEQUENCE LENGTH:262
SEQUENCE TYPE:nucleic acid
TOPOLOGY:linear
CLONE:HUMGS05769

SEQUENCE DESCRIPTION:
GATCANAACC CTAGCGCCTT TGGTCCTAAG AATGGNAGGC TGCCTTCCNT CCCAATCTCC 60
CTGCCAGGGC CCACAGCGTG GCCCTAGCCC TCCCCTCCCC GGAATGTAGA ACGGGGACCC 120
TCGCAGGGTT GGGGCGGGGG CTGATACTCC TNGGNCCCTC CCTACCCTGC CCTGTGTGTT 180
GGCTTTNTGG CCGTCCAAGT GCCAATTGGG TTTTNGCCCA AATAAGGGCT GGTATTTNTN 240
CTNTGTNCTT GGAGGTGNGN TN 262

SEQ ID NO:4847
SEQUENCE LENGTH:250
SEQUENCE TYPE:nucleic acid
TOPOLOGY:linear
CLONE:HUMGS05770
SEQUENCE DESCRIPTION:
GATCTGTATG TNAGGCACCC ATAACAGTAG TTTTCCCTGT NAGTCGTCTT CACACATGCT 60
GTTTTCTCTG CCTGGCTCTC TCTTCCCCTC CTTACCTGGC CAGTCCTGTT TATCATCAGG 120
CCTTGTNTTG GATATNACGT CCTCTGGGAA GTCTTCTGGG NCCCTCTAAC CTAGGACCCT 180
CATTACCGGN TCTCATAGNA CAGTCTACTG NTTTGTACGG AATTCTAAGT ATTTCNNGTG 240
GNACTNAATN 250

SEQ ID NO:4848
SEQUENCE LENGTH:227
SEQUENCE TYPE:nucleic acid
TOPOLOGY:linear
CLONE:HUMGS05772
SEQUENCE DESCRIPTION:
GATCATTCTT CAATTAGGAC ACAAACCAGA CAGGTTTAAT AGCGAATCTA ATTTTNAATT 60
CTGACCATGG ATACCCATCA CTTTGGCATT CAGTGCTACA TGTGTATTTN ATATAAAANT 120
CCCATTTCTT GANGATAAAA AAATTGTNAT TCAAATTGTT ATGCACAGAA TGTTTTTGGT 180
AATATTAATT TCCACTAAAA ANTTAANTGT NTTTTAAGGG ANNNGAN 227

SEQ ID NO:4849
SEQUENCE LENGTH:231
SEQUENCE TYPE:nucleic acid
TOPOLOGY:linear
CLONE:HUMGS05773
SEQUENCE DESCRIPTION:
GATCTAAATA TAAGAGCAAA AACCATAAAG CATCTAAAAG TAATTGAAGG AGAAAATCTT 60
AGTGCCTTTT GTNCTGGCCA AGATTTTTTA AAGAGGACAT AGAAGTCAGG AACTATAAAA 120
GAAACAATTT TAAATTTTNC TTTATCAAAA TTAAAAGCCA TAGACCGGGA TAANNCATTT 180
GCAANATATA TNTCTGACAA AGTTCTTGAN TCAAGANTAT GTAANANCNG N 231

SEQ ID NO:4850
SEQUENCE LENGTH:128
SEQUENCE TYPE:nucleic acid
TOPOLOGY:linear

CLONE:HUMGS05774
SEQUENCE DESCRIPTION:
GATCAGCCTG AACAACATAA TAAGACCCTG TCTCAACAAA AAATACAAAA ATTAGCTGGG 60
TGTGGTGGCA TATGCCTGTA GTCCCAGCTA TTCAGGAAGC TCCATTGCAA TCTAGCCCGG 120
GTGACAAA 128

SEQ ID NO:4851
SEQUENCE LENGTH:350
SEQUENCE TYPE:nucleic acid
TOPOLOGY:linear
CLONE:HUMGS05775
SEQUENCE DESCRIPTION:
GATCCTTCCT TCAACCCCAA GGCCAGCTCC CATCTCATTT CCAGAAAGGC TCATACCTGG 60
CTTGCAGGGA AGCATCTGTC TTGTCATTCC AGGTGCCAGA ATCCTCTCAG AGTCATTGAA 120
GGGTGTTCAC CCATCCCACC CAAGGCTTGG CACACTGCCA GTNTCTTAGC AGGGTCTTGT 180
GAGGGCTGGG GGCATCCAGG CACTCAGAAG GCAAAGGAAC CACCCTACCC ATTTGGCCTC 240
TGGAGGGGGC AGAAGAAAGA AAGAAACCTC ATCCTATATT TTNCAAAGCA TGGGGGTTCT 300
NGCATTTAGC TCTGGNGGGT GANCCANTGT GCTTNCTTGN TTAAGTGTCN 350

SEQ ID NO:4852
SEQUENCE LENGTH:355
SEQUENCE TYPE:nucleic acid
TOPOLOGY:linear
CLONE:HUMGS05776
SEQUENCE DESCRIPTION:
GATCTCAGTT TCCCTCACTC AGGAACTCTG TTTCTAAAGT CTTCAGTTAA GTTTNAGTTT 60
ATNACTGAGT GGCCTGTACT GTCAGACGTG AATGGGCCTG ACGGGCAAAT CCATCCCTCT 120
CTCCCTCACA GTTCCAGGAG CGGCTTCCCT CGTCTCCCCT TACTCCACAG GGAGCCTCCC 180
TTGCCAGGAC CAGGGCTGCG ACGGCCATGC TGGGGCAGGT GAGTGCTCTG TTAGCTGCTC 240
CCAGTGCTGT CCNCAGGCTG CAGTTCTGGT CCNNGGGGTG TNAGGGTAGG GAAGGGTGCA 300
CTTGAAGGAN GGTGGTTCAA TNTTGGGNTN CCTTAACGTT TATAGTCTNN ACAAN 355

SEQ ID NO:4853
SEQUENCE LENGTH:81
SEQUENCE TYPE:nucleic acid
TOPOLOGY:linear
CLONE:HUMGS05777
SEQUENCE DESCRIPTION:
GATCCAGGAG TTTAAGGTTA CAGTGAGCTA TGACCGCTAC TNCACTCCAG CCCAGNCAAC 60
AGAACAAGAC CCTGTTTCAA A 81

SEQ ID NO:4854
SEQUENCE LENGTH:136
SEQUENCE TYPE:nucleic acid
TOPOLOGY:linear

CLONE:HUMGS05778
SEQUENCE DESCRIPTION:
GATCCTCCGT AACATTNCNA GACCAGANAG AACATCCAGA AATCCCTGGN TGGAAGCTCA 60
GGCCCCGGGG CCTCCAGCGG CACCAGCGGA GACCACGGTN AGCTCGTCGT CCGGATTGCN 120
ACGATTAAAG ACTGAN 136

SEQ ID NO:4855
SEQUENCE LENGTH:108
SEQUENCE TYPE:nucleic acid
TOPOLOGY:linear
CLONE:HUMGS05779
SEQUENCE DESCRIPTION:
GATCAAATAT TTTTNATTAA CCTATATAAA GAATTTGACT GCTTATGATT CAATACATAT 60
GATTTAATAA AATAGATTGC ATTTTTNAAA ATAAAGAATG ATTNGAAA 108

SEQ ID NO:4856
SEQUENCE LENGTH:362
SEQUENCE TYPE:nucleic acid
TOPOLOGY:linear
CLONE:HUMGS05780
SEQUENCE DESCRIPTION:
GATCAACCTC TGATTTTACA TCATGATGTA ATCACCACTG GAGCTTCACT GTTACTAAAT 60
NATTAATTTC TTGCCTCCAG TGTTCTATCT CTGAGGCTGA GCATTATAAG AAAATNACCT 120
CTGCTCCTTT NCATTGCAGA AAATTGCCAG GGGCTTATTT CAGAACAACT TCCACTTACT 180
TNCCACTGGC TCTNAAACTC TCTAACTTAT ANGTGTTGGT GAACCCNCAC CCAGGCAGTT 240
ATCCATGAAA GNACAAGTGG ACTAGTNCTA TGNNGTACAA AGGCCTGTAT CNTCTTGTNG 300
ATGGATTTCC TGGTGCTCTT NGCTTGTTTG CAAGTTGCTT AANTTAAAGG CAGNTNTTTN 360
TN 362

SEQ ID NO:4857
SEQUENCE LENGTH:102
SEQUENCE TYPE:nucleic acid
TOPOLOGY:linear
CLONE:HUMGS05781
SEQUENCE DESCRIPTION:
GATCCTACCG GAAACCAACC TGTTTACTGT ACTGTTGTAA ATATCATGCC CTTTATATCC 60
TGTATATTGG CTTCTTTTAA ATAAAGTGAA ATGTCTTAGA AA 102

SEQ ID NO:4858
SEQUENCE LENGTH:195
SEQUENCE TYPE:nucleic acid
TOPOLOGY:linear
CLONE:HUMGS05782
SEQUENCE DESCRIPTION:
GATCTAAACT TTATGCTGCA TAAATCACTT ATCGGAAATN CACATTTCAT AGTGTGAAGC 60

ACTCAANNTN TAAACCTTAT TATCTAAGGT AATATATGCA CCTTTCAGAA ATTTNTTTTC 120
GAGTAAGTAA AGCATATTAG AATAATTGTG GGTTGACAGA TTTTTAAAAT AGANTTTAGA 180
GTATTTGGGG TTTTN 195

SEQ ID NO:4859
SEQUENCE LENGTH:210
SEQUENCE TYPE:nucleic acid
TOPOLOGY:linear
CLONE:HUMGS05783
SEQUENCE DESCRIPTION:
GATCCCTTTG AAATNTTTTT TTTGTTTGTT TGTTTAAATC AAGCCTGAGG CTGGTGAACA 60
GTAGCTACAC ACCCATATTG TGTGTTCTGT GAATGCTAGC TCTCTNGAAT TTGGATATTG 120
GTTATTTTTT ATAGAGTGTA AACCAAGTTT TATATTCTGC AATGCGANCA GGTACCTATC 180
TGTTTCTAAA TAAANCTGTT TACATTCAAA 210

SEQ ID NO:4860
SEQUENCE LENGTH:139
SEQUENCE TYPE:nucleic acid
TOPOLOGY:linear
CLONE:HUMGS05784
SEQUENCE DESCRIPTION:
GATCTTTGTT TGTCTGAACA GGTATTTTTA TACATGCTTT TTGTAAACCA AAAACTTTTA 60
AATTTCTTCA GGTTTTCTAA CATGCTTACC ACTGGGCTAC TGTAAATGNG AAAAGNATAA 120
ANTTATTTAA TGTTTTAAA 139

SEQ ID NO:4861
SEQUENCE LENGTH:150
SEQUENCE TYPE:nucleic acid
TOPOLOGY:linear
CLONE:HUMGS05785
SEQUENCE DESCRIPTION:
GATCCTGAAA GAATAATTGG TGCCACAGAC AGCAGTGGAG AATTGATGTT TCTCATGAAA 60
TGGAAAGNTT CAGATGNGGC AGACTTGGTG CTGGCGAAAG AGGCAAATAT GANGTGTCCT 120
CAAATTGTAA TTGCTTTTTA TGNNGNGNGN 150

SEQ ID NO:4862
SEQUENCE LENGTH:155
SEQUENCE TYPE:nucleic acid
TOPOLOGY:linear
CLONE:HUMGS05787
SEQUENCE DESCRIPTION:
GATCGCAGAT TTNTNTAGAA ACACGGATGT NCATGTCCAG ATTCCCTTTT NCAGGTATTA 60
AAAATAATTA AAAATAGTCC TGCCTGAGGT TGCAGTGAGC CGAGCTTGCA CTACTGCACT 120
CCAGCCTGGG TGACAGAGTA AGACTCCATG TCAAA 155

SEQ ID NO:4863
SEQUENCE LENGTH:262
SEQUENCE TYPE:nucleic acid
TOPOLOGY:linear
CLONE:HUMGS05789
SEQUENCE DESCRIPTION:
GATCTGTACA ACTGATTTTA AAAAAATGCT ACAAAAAGCC CCAAAGCATA TAATCTCTAC 60
TCCTTACAGT CTCTAGAATT AAATGTACTC ATTTAGACAA CATATTAAAT GCATATTTNA 120
GCCACTTTAG AGAAACCTCA TAGGCACAGA GTTTCCAAGN TTAATTTTAA GNATATCTTC 180
ACGAACTTGA CCCTCCTACT CCACATTGCA NCATTTCCNT CAGNCAGCAT TTNAATTCCN 240
GTATTATGTN TNTNGCAANT NN 262

SEQ ID NO:4864
SEQUENCE LENGTH:232
SEQUENCE TYPE:nucleic acid
TOPOLOGY:linear
CLONE:HUMGS05790
SEQUENCE DESCRIPTION:
GATCAAGAAG GCCACTGGTG TGCAGGTGTN AGGCTCCTAC AGGCCCACTC TCTTCAGCAG 60
CCACCCGGCC CTCCCTCCAG CACCCGTTTT AATCCCACAG AACAACGGGA ACGTTACTGA 120
CTCTGGTGCC TTATCTCGAA GGGACCAGAA GTGCTGCGTG TTCAGGCCAT CNNTGGCTGT 180
NTTCCTGTCT CTCCTGTCTG TCCACCTACT ACTNNNTNCT NTACTTTANC NN 232

SEQ ID NO:4865
SEQUENCE LENGTH:76
SEQUENCE TYPE:nucleic acid
TOPOLOGY:linear
CLONE:HUMGS05792
SEQUENCE DESCRIPTION:
GATCAGAGCC GACACCAGAC GTGATTAGCA GGCGCACAAN ATTCAATTTG TTAAATGAAA 60
TTGTATTTTG CCCAAA 76

SEQ ID NO:4866
SEQUENCE LENGTH:80
SEQUENCE TYPE:nucleic acid
TOPOLOGY:linear
CLONE:HUMGS05793
SEQUENCE DESCRIPTION:
GATCTCTTGG GCCCAGGACT GTTGATGCCT TTNAGTTTTG TATTCAATAA ACTTTTTTTG 60
TCTGTTGATA ATATTTTAAA 80

SEQ ID NO:4867
SEQUENCE LENGTH:149
SEQUENCE TYPE:nucleic acid
TOPOLOGY:linear

CLONE:HUMGS05794
SEQUENCE DESCRIPTION:
GATCCATGCA TTATTTTCCT AGCTTCCTGC CTTGCTCCCT ATTCACTTTA CACTGTGAAA 60
GGTGGGGGGT GAGTCCCACT TGAGCGCTTC CTGTTGAATA AAGCAGGCAC TTGACCTGGC 120
TGTAGCCTAG GTCTTGAGTG AACCCCAAA 149

SEQ ID NO:4868
SEQUENCE LENGTH:209
SEQUENCE TYPE:nucleic acid
TOPOLOGY:linear
CLONE:HUMGS05795
SEQUENCE DESCRIPTION:
GATCATGTCT GTACATTGTG TAATGAATGA AAAGCACATA AATNTAATCT ACTTTGAACT 60
TTGTAAAAAT AATGTGTGGA GGCTATTCTT GTTTCTCCAT CTCAAGTCCT GTGTGTGCAC 120
GTGTGTGCAA GTGCACATGT GTGTGTGTAA TAACACATTG TAAAGANCAG AAATNACTTT 180
AAAAAATAAA CAGAAATGGA GACCTGAAA 209

SEQ ID NO:4869
SEQUENCE LENGTH:335
SEQUENCE TYPE:nucleic acid
TOPOLOGY:linear
CLONE:HUMGS05796
SEQUENCE DESCRIPTION:
GATCCGNCCA CCTTGACCTC TCAAAGTGCT GGGATTACAG GCATGAGCCA TCACGCCCGG 60
CCACGCTGTT GGTTCTTAAT GACACAGCTT ANCTTTATTG TGAAAAGATT GCAGCAACAA 120
ATGAGATTTT ACCTGTATTT GTTAAAAATG CTTATCCTTG TCTAAGACTG GCAACATAAG 180
CAGTTCTTNG GCTTCTATGC CAATGGNCAC TAGGCAGTAA TACATGTGCA GTGCTANATA 240
GAAAATATTG GAGTNAGGGT GTACTNAGGG AAGTTCTCAA TCTTTNCCCT NTCANTNTCT 300
TCTGTAATAG TAACCTTNAA TAACATGNGG ATTCN 335

SEQ ID NO:4870
SEQUENCE LENGTH:207
SEQUENCE TYPE:nucleic acid
TOPOLOGY:linear
CLONE:HUMGS05797
SEQUENCE DESCRIPTION:
GATCTTTCTG CAGGAAATAG TCACTCATCC CACTCCACAT AAGGGGTTTA GTAAGAGAAG 60
TCTGTCTGTC TGATGATGGA TAGGGGGCAA ATCTTTTTCC CCTTTCTGTT AATAGTCATC 120
ACATTTCTAT GCCAAACAGG AACAATCCAT AACTTTAGTC TTAATGTACA CATTGCATTT 180
TGATAAAATT AATTTTGTTG TTTCAAA 207

SEQ ID NO:4871
SEQUENCE LENGTH:208
SEQUENCE TYPE:nucleic acid
TOPOLOGY:linear

CLONE:HUMGS05798
SEQUENCE DESCRIPTION:
GATCTGTCAC GGAAGGCGTC CTTTTTCCTT GTAGCTAACG TTAGGCCTGA GTAGCTCCCC 60
TCCATCCTTG TAGACGCTNC AGTCCCTACT ACTGTGACGG CATTTCCATC CCTCCCCTGC 120
CCGGGAAGGG ACCTTGCAGG GACCTCTCCC TCCAAAAAAA GAAAAAAAGA AAAAGANAGA 180
AAAANTAAAT GAGGAAACGT GTTNCAAA 208

SEQ ID NO:4872
SEQUENCE LENGTH:84
SEQUENCE TYPE:nucleic acid
TOPOLOGY:linear
CLONE:HUMGS05799
SEQUENCE DESCRIPTION:
GATCAGACCA TAATGTTACA TTATTATCAA CAATAGTGAT TGATAGAGTG TTATCAGTCA 60
TAACTAAATA AAGCTTGCAA CAAA 84

SEQ ID NO:4873
SEQUENCE LENGTH:337
SEQUENCE TYPE:nucleic acid
TOPOLOGY:linear
CLONE:HUMGS05801
SEQUENCE DESCRIPTION:
GATCCTGGCC GAGGTGAGGA ACAGACAGGG GGGGTCTAGA TTCTAAGGGG GTTGGTGGAT 60
ATTGGGCAAG GCAGGAAACC TCTGGAGACC TCATTTTCTC CATGGGGAAG ACAGCCATGC 120
TCTTCAGGAG GAGACTCCAA GGGCAAAGNA GGGTGTCTTG GCTGTNCTTG AAGGCGAAAC 180
CCTGCCATAT CCCCAGTGCC AGTCCCCTCA GNCTGTGGTG GCCTTGCATC CTGACTGGAT 240
GTTCTCAGCC CNTTGTTCTG GGCAAGAACC CAGAGCTCCC CAGTNTGGAT ACTTATTAAA 300
CCTNTTTGGA GNCNCAAATA NAAAAAAANA AANNAGN 337

SEQ ID NO:4874
SEQUENCE LENGTH:329
SEQUENCE TYPE:nucleic acid
TOPOLOGY:linear
CLONE:HUMGS05804
SEQUENCE DESCRIPTION:
GATCCAGGGG CTGATTGGCA GCGTGGAGGA GCAGCTGGCC CAGCTTCGCT GCGAGATGGA 60
GCAGCAGAAC CAGGAATACA AAATCCTGCT GGATGTGAAG ACGCGGCTGG AGCAGGAGAT 120
TGCCACCTAC CGCCGGCTGC TGGAGGGAGA GGATGCCCAC CTGANTCAGT ACAAGAAAGA 180
ACCGGTGACC ACCCGTNAAG TGCGTACCAT TGTGGGAAGA GGTCCAGGAT GGNAAAGGTN 240
AATCTTCTTC CGTGNAGCAN GGTNCAACCA GACCAACCGT TTGAGGGACT TANGTTACCN 300
NGNGCCGGGC CAACCNAAGG AGGGNNTTN 329

SEQ ID NO:4875
SEQUENCE LENGTH:142
SEQUENCE TYPE:nucleic acid

TOPOLOGY:linear
CLONE:HUMGS05807
SEQUENCE DESCRIPTION:
GATCACTTGC TTTTATGTCC ACTTTTTNAG TGGTACTTAA ATGTAAAGTA ACAACCTGAA 60
TTGAGTCATT GCTTTCTGAA GGAATCATTG TCCTTTCTCC AGTTTTTGTN CCAGAATAAA 120
AGGAAATATT TTAAAAGCCA AA 142

SEQ ID NO:4876
SEQUENCE LENGTH:331
SEQUENCE TYPE:nucleic acid
TOPOLOGY:linear
CLONE:HUMGS05808
SEQUENCE DESCRIPTION:
GATCCTNCAG CACAGGAATT CTCAAGACCT GAGTATTTTT AATAATAGGA ATGTCCACCA 60
TGAACTTNAT ACGTCCGTGT GTCCCAGATG CTGTCATTAG TCTATATGGT TCTCCAAGAA 120
ACTGAATGAA TCCATTGGAG NAGCGGTGGG GNACTAGCCA GACAAAATTT NAGANTACAT 180
AANCAACGCA TTGCCACGGN AACATACAGN GGATGCCTTT TCTGTGATTG GGTGGGATTT 240
TTTCCCTTTT TATGTGGGAT ATAGTNGTTA CTTGTGACAN GTATAATTTT GGAATAATTN 300
CTATTAAATA TCANCTCTGA NGNTACTNGT N 331

SEQ ID NO:4877
SEQUENCE LENGTH:61
SEQUENCE TYPE:nucleic acid
TOPOLOGY:linear
CLONE:HUMGS05810
SEQUENCE DESCRIPTION:
GATCATGCCA TTGCACCCCA GCCTGGGCAA CAACTTAACT TTTTTGAAAC TCTGTCTCAA 60
A 61

SEQ ID NO:4878
SEQUENCE LENGTH:81
SEQUENCE TYPE:nucleic acid
TOPOLOGY:linear
CLONE:HUMGS05813
SEQUENCE DESCRIPTION:
GATCCTCTGG ACACGTAACC TATGTCAGNC ACTACATGAT GACTCAAGGC CAATAATAAN 60
GCCATTTCCT ACCTGCACAA A 81

SEQ ID NO:4879
SEQUENCE LENGTH:102
SEQUENCE TYPE:nucleic acid
TOPOLOGY:linear
CLONE:HUMGS05814
SEQUENCE DESCRIPTION:
GATCCANAGA GGACCCCCGN CCCCAGAGAC TTGGTTTTGG CTCCAGCCTT CCCCTGGCCC 60

CGTGACACTC AGGAGTTAAT AAATGCCTTG GAGGAAAACA AA 102

SEQ ID NO:4880
SEQUENCE LENGTH:66
SEQUENCE TYPE:nucleic acid
TOPOLOGY:linear
CLONE:HUMGS05815
SEQUENCE DESCRIPTION:
GATCAAAAGG TCTCAGATGA TGATAAAGAA AAGGGAGAGG GAGCTCTTCC AACTGGGAAA 60
TCCAAA 66

SEQ ID NO:4881
SEQUENCE LENGTH:287
SEQUENCE TYPE:nucleic acid
TOPOLOGY:linear
CLONE:HUMGS05816
SEQUENCE DESCRIPTION:
GATCTCTAGA GTCCATTAAA AGCAAGCTAG ATTGAGAGCT CTTGGAGGGC AGGGACTGGG 60
CCTAGCTCAT GGTTTACAGC TCTTGAGGGT GACTGCACAG TGGACTCAGT CAGTCATGGC 120
TGAGTTGTGT TGGCTTNATT ATCTCTAGAA TGTAGTTCTC CATTCAAATG CAAATCAGCC 180
TGGNNGGNTC ACCTGTAGTA AAGGGGGTTT CTACCTNGNG GATGGAAANA GNNNTCTTAG 240
GNACCTTCCT TGGGCCTCCA GNACTTATTT TNGATATCCC NTTATGN 287

SEQ ID NO:4882
SEQUENCE LENGTH:74
SEQUENCE TYPE:nucleic acid
TOPOLOGY:linear
CLONE:HUMGS05817
SEQUENCE DESCRIPTION:
GATCATACCA TTGCACTCCA GCCTGAGTGA CCTGACTGAG ACTGTCTCAA AAAGNGTAAA 60
ANATAAAAAT TAAA 74

SEQ ID NO:4883
SEQUENCE LENGTH:106
SEQUENCE TYPE:nucleic acid
TOPOLOGY:linear
CLONE:HUMGS05818
SEQUENCE DESCRIPTION:
GATCTNACTC CAGCAGAGTC TGTNCCAATN TGGGGTGAGC AGGGCCCCTG CCATGGAGCA 60
GCAGGAATTT NAGGGATGGA GCAGGTTAAA GAATGGGAAA CATAAA 106

SEQ ID NO:4884
SEQUENCE LENGTH:96
SEQUENCE TYPE:nucleic acid
TOPOLOGY:linear

CLONE:HUMGS05819
SEQUENCE DESCRIPTION:
GATCTTAGTG GTGAAAGTAT GTGTAACCAT GTGATGGTTA AAACANGACT TACAATNCCT 60
AAATGTGTAA CTGAGAATAA AACGTACTCT NTTAAA 96

SEQ ID NO:4885
SEQUENCE LENGTH:63
SEQUENCE TYPE:nucleic acid
TOPOLOGY:linear
CLONE:HUMGS05820
SEQUENCE DESCRIPTION:
GATCCNAAGN CTCCGGGCAG CCGTTATCCC GTGGTTTAAT AAAGCTNCCG CGCGCTCACC 60
AAA 63

SEQ ID NO:4886
SEQUENCE LENGTH:61
SEQUENCE TYPE:nucleic acid
TOPOLOGY:linear
CLONE:HUMGS05821
SEQUENCE DESCRIPTION:
GATCCTGCTG TGTGTCCTGT TCCATGTTCC GGTTCCATCC AAATACACTT TCTGGAACAA 60
A 61

SEQ ID NO:4887
SEQUENCE LENGTH:274
SEQUENCE TYPE:nucleic acid
TOPOLOGY:linear
CLONE:HUMGS05823
SEQUENCE DESCRIPTION:
GATCTCCTTG TCAGGAGCAC CTAAGAACTG GCATGACTCT ACACCCTNTC TCCTTCTNCA 60
ATGACCTCGT GTCAAGATGG CAGACCTGCA GAGATGAATT TGCCTNGATA CCTGAGTCAC 120
CAGNGNGGAG GAAACTCCCA TTNGACATGC ATCACACTCC ACATATGAAA CCCTNCTTTT 180
GCTGCACTGT CACTGGGGGN TAGGGGTTAT TACTCACTGC AACTTAGCCT AGCATTAAGT 240
TGNTTTGACT NATNCANGTT TCAGTAAANG TCGN 274

SEQ ID NO:4888
SEQUENCE LENGTH:268
SEQUENCE TYPE:nucleic acid
TOPOLOGY:linear
CLONE:HUMGS05824
SEQUENCE DESCRIPTION:
GATCTGTGTT GGTTTCTTAG ATTGCTAGCT TTTCCTCCAG GGGACCACAG CAGGTGAAGC 60
TCAAGAGCGC ATGGCTCTGC TAATAGTAAA TTGTTTTCAG GGCCTTGTCC AGCTGAGAGC 120
TTCATGTCCA CCAGATTCTG AGAGGTGTCA GCAGCACTTT TTTTTTNATT TGTNGTTNGG 180
TTTCCNCCCG GTTATCGGAC CATGGGCTNA GCTCAGGCAC TTGCTGTAGG AGACTNGNNA 240

TTNCTGTAAA GGANNGGTNA TTTANCCN 268

SEQ ID NO:4889
SEQUENCE LENGTH:323
SEQUENCE TYPE:nucleic acid
TOPOLOGY:linear
CLONE:HUMGS05825
SEQUENCE DESCRIPTION:
GATCAAACTT GGTCTGGGTA TTGATGAAGA TGACCCTACT GCTGATGATA CCAGTGCTGC 60
TGTAACTGAA GAAATGCCAC CCCTTGAAGG AGATGACGAC ACATCACGCA TGGAAGAAGT 120
AGACTAATCT CTGGCTGAGG GATGACTTAC CTGTTCAGTA CTCTACAATT CCTCTGATAA 180
TATATTTTCA AGGNTGTTTT TCTTTATTTT TGTNAATATT AAAAAGTCTG TATGGCATGA 240
CAACTACTTT AAGGGGAAGA TAAGATTTTC TGTCTACTAA AGTGATGCTG TGGTACCTTA 300
GGCACTNAAG CAGNGCTAGT ATN 323

SEQ ID NO:4890
SEQUENCE LENGTH:66
SEQUENCE TYPE:nucleic acid
TOPOLOGY:linear
CLONE:HUMGS05826
SEQUENCE DESCRIPTION:
GATCTAAGAG TATGGCTAAA CATCTATATA TGCAATCTAT TAAAAGAACT TAATTCGGCT 60
ATTAAA 66

SEQ ID NO:4891
SEQUENCE LENGTH:89
SEQUENCE TYPE:nucleic acid
TOPOLOGY:linear
CLONE:HUMGS05827
SEQUENCE DESCRIPTION:
GATCTCTGGA GCTCAAGAGG TTGAGGCTGC AGTGAGCCGA TGTGCCAGTA CACTGCAGCN 60
TNGGTGACAG AGCAAGACCC TGTCTCAAA 89

SEQ ID NO:4892
SEQUENCE LENGTH:344
SEQUENCE TYPE:nucleic acid
TOPOLOGY:linear
CLONE:HUMGS05829
SEQUENCE DESCRIPTION:
GATCGCCACT TACCGCAAGC TGCTGGAGGG CGAGGAATGC AGACTCAGTG GAGAAGGAGT 60
TGGACCAGTC AACATCTCTN TTGTCACAAG CAGTGTTTCC TCTGGATATG GCAGTGGCAG 120
TGGCTATGGC GGTGGCCTCG GTGGAGGTCT TGGCGNCGGC CTCGGTGGAG GNCTTGCCGG 180
AGGTAGCAGT GGAAGCTACT ACTCCAGCAG CAGTGGGGGT NTCGGCCTAG TNNNNNGGGC 240
TCAGTGTGGG GTGCTCTTGG CTTCAGTGCA AGCAGTAGCC NGAGGGCTGG GGGTGGNCTT 300
NTGGCANTGG CGGNTNTAGC AGNTCCAGCG TCAAATTTTN CTCN 344

SEQ ID NO:4893
SEQUENCE LENGTH:342
SEQUENCE TYPE:nucleic acid
TOPOLOGY:linear
CLONE:HUMGS05830
SEQUENCE DESCRIPTION:
GATCAGGNAG CCTCCTGATG CCAGAACACC TNAGGCAGAG CCCTACTCAG CTGTACCTGT 60
CTGCCTGGAC TGTCCCCTGT CCCCGCATCT CCCCTGGGAC CANCTGGAGG GCCACATGCA 120
CACACAGCCT AGCTGCCCCC AGGGAGCTCT GCTGCCCTTG NTGGCCCTGC CCTTCCCACA 180
GGTGAGCAGG GCTCCTGTCC ACCAGNACAC TNAGTTCTNT TCCCTGCAGT GTTTTCATTT 240
TATNGGGGNC AAACATTTTG CCTGTTTTCT GGTTTCAAAC ATGATAGTTG ATATGAGACT 300
GAAANCCCTG GGTTGTGGAG GGGAAATTGG GNTNAGAGGG GN 342

SEQ ID NO:4894
SEQUENCE LENGTH:138
SEQUENCE TYPE:nucleic acid
TOPOLOGY:linear
CLONE:HUMGS05831
SEQUENCE DESCRIPTION:
GATCCTGGAC TTTNCCCTCA GCAAAGATGC CTCCNTGGTG GTGACCACGT CAGGAGACCA 60
CAAAGCGAAA NTNTTTNTTT TCCAAAGGCC TGACCGTTAA TGGCTGCAGC CCCTGCCTGT 120
TTGTNTGGTG TTGAGGGN 138

SEQ ID NO:4895
SEQUENCE LENGTH:109
SEQUENCE TYPE:nucleic acid
TOPOLOGY:linear
CLONE:HUMGS05832
SEQUENCE DESCRIPTION:
GATCCACCCG CCTCAGCCTC CCAAAGTGCT GGGATTACAG GTGGGAGCCA CAACACCCGG 60
CCATGAATTA ACTCCGTTAA AAAATAAACG TATACATTCT GTGAGCAAA 109

SEQ ID NO:4896
SEQUENCE LENGTH:274
SEQUENCE TYPE:nucleic acid
TOPOLOGY:linear
CLONE:HUMGS05833
SEQUENCE DESCRIPTION:
GATCGGTTTC GCCATCATGA CGCAGTGTCT CCCAGTGGCC CTGTTCTCCC TGGTGGGCTT 60
CACCCAGATG ACCATNTGGG CCAAGGGCAA GCACCGCAGC TACCTGAAGG AGTTCCGGGA 120
CTACCCGCCC CTGCGCATGC CCATNATNCC CTTCCTGCTC TGAGCGCTCA CCCCTGCTGA 180
GGCTCAGCCC CTNAACCCGG TGGCATTCTG GGGGAGGAGT GGGGCCCACA GNTCTCCAGC 240
ACCCGGAATA AAGCCCGNCT GCCCCAGTCG GAAA 274

SEQ ID NO:4897
SEQUENCE LENGTH:343
SEQUENCE TYPE:nucleic acid
TOPOLOGY:linear
CLONE:HUMGS05834
SEQUENCE DESCRIPTION:
GATCTTGCTG GATGTGAAGA CGCGGCTGGA GCAGGAGATT GCCACCTACC GCCGCCTGCT 60
GGAGGGCGAG GATGCCCACC TTTCCTCCCA GCAAGCATCT GGCCAATCCT ATTCTTCCCG 120
CGAGGTCTTC ACCTCCTCCT CGTCCTCTTC GAGCCGTCAG ACCCGGCCCA TCCTCAAGGA 180
GCAGAGCTCA TCCAGCTTNA GCCAGGGCCA GAGCTCCTAG AACTGAGCTG CCTCTACCAN 240
AGCCTNCTGC CCACCAGCTG GCCTCACCTC CTGAAGGNCC GGGTCAGGAC CCTGCTCTNC 300
TGGNGNAGTT TCCCAGGTAT NTTCNNTGGT TCCTGTTGGT GAN 343

SEQ ID NO:4898
SEQUENCE LENGTH:338
SEQUENCE TYPE:nucleic acid
TOPOLOGY:linear
CLONE:HUMGS05835
SEQUENCE DESCRIPTION:
GATCAGNAGC CCCCAACACA GGCAAGTCCA CCCCATAATA ACCCTGCCAG TGCCAGGGTG 60
GGCTGGGGAC TCTGGCACAG TGATGCCGGG CGCCAGGACA GCAGCACTCC CGCTGCACAC 120
AGACGGCCTA GGGGTGGCGC TCAGACCCCA CCNTACGCTC ATCTCTGGAN GGGGCAGCCC 180
TGAGTGGTCA CTGGTCAGGG CAGTGGCCAA GCCTGCTGTG TCCTTCCTNC ACAAGGTCCC 240
CCCACCGTTC AGTGTCAGCG GGTGACGTGT GTTCTTTTGA GTCCTTGTAT GANTAAAAGN 300
CTGGAACCTN ANAAAAAGTG NGNNGNGGTN TNTGNTAN 338

SEQ ID NO:4899
SEQUENCE LENGTH:356
SEQUENCE TYPE:nucleic acid
TOPOLOGY:linear
CLONE:HUMGS05836
SEQUENCE DESCRIPTION:
GATCAGCCCC TGACTTTNTT AAAAGATATC AGAAGTGTCT TGGAGCCAAC TAGAGGCAGG 60
GTCATCCTTG CCCTTNTCCT CCCCTTTCAT CCCTATGTGG AAAACGTAGG TGGCAAGTGG 120
GAGAAACCAT CAGAAATTTT GGAAATCAAA GGACAGAACT GGGAAGANCA AGTGAATAGT 180
CTGCCTGANG TTTTCAGAAA ANCTGGTTTT NTTATCGAAG CTTTCACCAG ACTACCATAC 240
CTGTNTGAAG GCGACATGTN TAANGNCTAC TNCGTTCTGG NTGACGCTNT CTTTGTCCTC 300
AANCCAGTAT AAACACGTGG NGGTCGAAGT CTTCAGAGTC CGTACCNTNC TGGATN 356

SEQ ID NO:4900
SEQUENCE LENGTH:111
SEQUENCE TYPE:nucleic acid
TOPOLOGY:linear
CLONE:HUMGS05837
SEQUENCE DESCRIPTION:

GATCCCCCAG AAAAGAAAGC TACAGAAGAA ACTGGGGCTC CTCCAGGGTG GCAGCAACAA 60
TAAATAGACA CGCACGGCAG CCACAGCTTG GGTGTGTGTT CATCCTTGAA A 111

SEQ ID NO:4901
SEQUENCE LENGTH:293
SEQUENCE TYPE:nucleic acid
TOPOLOGY:linear
CLONE:HUMGS05838
SEQUENCE DESCRIPTION:
GATCTTAATG GGTTCGATGG TAGGGAATAC GGAAAGGATA CACTGCTAAT ATTGTGTCTA 60
ACAATAAAGC AAACAACTCG ATGCTGTAAG AAGACAAGCT GTTTGAGCTT TCAAACACAC 120
AGGGGCACAG CACAATTCAG AAATACCCAG GAGGATTCCT GCAGGCTTAA CAGTTGGCAT 180
CGTACTGAAA TGAAACGTGT ATTAACANGA AANTGTCAAA CTTTTGGGGA AACAAATTTT 240
CTGTTACTTC CCACCCNACT AAAAGGGAAG ATAAAACCAT CATCACCAGN NNN 293

SEQ ID NO:4902
SEQUENCE LENGTH:99
SEQUENCE TYPE:nucleic acid
TOPOLOGY:linear
CLONE:HUMGS05839
SEQUENCE DESCRIPTION:
GATCTACATA AGGAAAGGAA GAATATTAAA GAAGAAATAA ATGAAGACGA GATAAAATCT 60
TTTTTCTTAA TTATCAAATA AAAGTTTGTT AAAAGTAAA 99

SEQ ID NO:4903
SEQUENCE LENGTH:290
SEQUENCE TYPE:nucleic acid
TOPOLOGY:linear
CLONE:HUMGS05840
SEQUENCE DESCRIPTION:
GATCAGGCAG TTCCTGGACT GTTCCACCAC TCAGAGTGAC CTGTCCCTGT GTGAGGGCTT 60
CAGCGAGGCC CTGAAGCAGT GCAAGTACTA CCATGGTCTG AGCTCCCTGC CCTGAAGAGG 120
TCGGTGCAGA CTCGGGGGCC AGTCCTGCAC NCACNTCTAC CCCTCGCCGA CAGCCAGACC 180
ACAACACCAG ATTGTACCCA GATAGCTGGG ATTGGAAGTN AGGAGGTTTC TCACCCCACA 240
GATAACCCAA GACACAAATG TGCAATTAAA AGTTTATTTT AGACCACAAA 290

SEQ ID NO:4904
SEQUENCE LENGTH:68
SEQUENCE TYPE:nucleic acid
TOPOLOGY:linear
CLONE:HUMGS05841
SEQUENCE DESCRIPTION:
GATCTGCCCG CCTTGGCCTC CCAAAGTGCT GNGATTNCAG GCATGAGGCG CTGCATCAGT 60
TCTGCAAA 68

SEQ ID NO:4905
SEQUENCE LENGTH:223
SEQUENCE TYPE:nucleic acid
TOPOLOGY:linear
CLONE:HUMGS05842
SEQUENCE DESCRIPTION:
GATCTGCAAG CNGGAGCCCT CCTACCCGCA GCTTNAGCAC AGNATCATCC AGCCCTGGGG 60
AGGCGCACCC TTAAGCAATA CAGACTGGGC TGAGGCTGAC AGGCAGAAGA CTAACAGAGC 120
GCANTCTGCA CACGCAGGTT CTGGGGCGAC CTCTGGCCCT GGNCATCTCT GNACTAACTN 180
ATCTGAATTA TGGAAGGTGG CAGTNTTGGT CANTAGTTTT NNN 223

SEQ ID NO:4906
SEQUENCE LENGTH:293
SEQUENCE TYPE:nucleic acid
TOPOLOGY:linear
CLONE:HUMGS05843
SEQUENCE DESCRIPTION:
GATCCGTCCG CTTCGGCCTC CCAAAGTGCT GGGATTACAG TCGTGAGCAC CGCGCCCGGN 60
CTGNGGTCAA TTTTCTATGT CAAGTGAGTG ACATTATTTT TACAAGTATT TTCTTCTTTT 120
TAAAATTATA CCAGCAAATA CAGTTAATGC AGAAACCTAA GTAATTTGAG TGTAGCTATT 180
CACAATCTAT GTGAAGNNNN CAAAATAGAA TGACCAGTTT TATTATAAAT TAAGGGGCTT 240
AATTTTCTAA AAAAGNANAN AANTGAGCCN TATCTCTGTG TTTTATAATT ATN 293

SEQ ID NO:4907
SEQUENCE LENGTH:65
SEQUENCE TYPE:nucleic acid
TOPOLOGY:linear
CLONE:HUMGS05844
SEQUENCE DESCRIPTION:
GATCACTTGA GGCCAAGTAT TTAAAACCAG CCTGGGAAAT ACAGTGAGAC CTTNTNTCTA 60
CCAAA 65

SEQ ID NO:4908
SEQUENCE LENGTH:225
SEQUENCE TYPE:nucleic acid
TOPOLOGY:linear
CLONE:HUMGS05845
SEQUENCE DESCRIPTION:
GATCCTGAAG GCCTTCGAGT ATTTTACTAT TTGGTACAAG ACTTGAAATG TTTNGTNTTC 60
AGTCTTATTG GATTACACTT CAAGATTAAN CCAATTTAAA TTGTATGTNT TCAGGCTGTT 120
TGTATATTTN ATTAAGGGAT GGGAGGGGTT ATTTGTCATT TACAGTATTG GGGTTTTNAT 180
GAATGTGAAG CAAACAANAN ANATTTGTNT GTAAACTGTT TTNTN 225

SEQ ID NO:4909
SEQUENCE LENGTH:135

SEQUENCE TYPE:nucleic acid
TOPOLOGY:linear
CLONE:HUMGS05846
SEQUENCE DESCRIPTION:
GATCAGGTTG GGGGGCAGCC CCCAGTCCCT TTCTGTCCCA GCTCAGTTTT CCAAAAGACA 60
CTGACATGTA ATTCTTCTCT ATTGTAAGGT TTCCATTTAG TTTGCTTCCG ATGATTAAAT 120
CTAAGTCATT TGAAA 135

SEQ ID NO:4910
SEQUENCE LENGTH:225
SEQUENCE TYPE:nucleic acid
TOPOLOGY:linear
CLONE:HUMGS05847
SEQUENCE DESCRIPTION:
GATCCAGTAT TTGATGAAAC TCATGAAAGT GGGTGGAGCC CATCTNGCCC CTCCTCTTTT 60
CTAGGACGCA CTATATGTGA CTGTGACTTT CAAGGACATT TGTTTGCCAT TTGCTGATTT 120
TTTTGGGAAG TNAATTNCTA ACTNCTTTCA CTGATAAATG NAGAAAAGTA TTGCACCNGT 180
GAAATGCACC AAATGAATNG NGTTTTGTAA TTAAAAAAAN NTNTN 225

SEQ ID NO:4911
SEQUENCE LENGTH:281
SEQUENCE TYPE:nucleic acid
TOPOLOGY:linear
CLONE:HUMGS05848
SEQUENCE DESCRIPTION:
GATCCCCTAA GGTCCTGGCC CAGGNCTGCC CAAAGAGAAG CCCAGGATGG TCGGCTGCCT 60
GGGGGCATTG TCACCACGTN GCCATGACGG CTGGTCCCNA CAGGNCCAGC TGGGAGGACT 120
GGTTGTGCTG CTGGAGAAGG GCTGGAGAAG GCAATGGCAT GCTGCCGCTT TGCCAGTCCC 180
TAGAAGTCGC GGTGCAGGTG ATGTGTNGNG AGCCGTNCCT NCANTGGGCA GGCCNGGGAG 240
TNTACTTGTG TGCAGCTGAC CCAAGGGCAG CCACATNTNN N 281

SEQ ID NO:4912
SEQUENCE LENGTH:179
SEQUENCE TYPE:nucleic acid
TOPOLOGY:linear
CLONE:HUMGS05849
SEQUENCE DESCRIPTION:
GATCAAAACC CAGCAGAGTG CAAGCAGCAG TGAAGCAGGA TGTATGTGGC CTTGAGGATA 60
ACCTGCACTG GTCTACCTTC TGCTTCCCTG GAAAGGATGG ANTTTACATC ATTTGGGCAA 120
GGCCTATTTT CAAGGTTTNT TTGGTNTGNT TGGNTNGCTT GTTTTTGGNT TNTGGCAGN 179

SEQ ID NO:4913
SEQUENCE LENGTH:313
SEQUENCE TYPE:nucleic acid
TOPOLOGY:linear

CLONE:HUMGS05850
SEQUENCE DESCRIPTION:
GATCGAGGGC TGGGNACCCC GGTGTNAGCA GGATGGGGCC CTGCCCTCCC GTGGNAGTTG 60
TGGACTCGAG CCCAGGGGCT GCCCGTAACA GCGGTGTCCC AGGTCCCTGC CATCCNATTT 120
TACCTGGAAT GTCTTCTCTG GAGTTTGGAA TTGCTTGAGG AACCCTGCGT GTGCTTGGAG 180
AGGCCAGAGG GCTTGCTGAG AACCCCATGG ACAGTGGAGA GCGGGATTCG AACCAAGGGC 240
TGGACTCCCA CACCTNTGGC CTGCGTCGCC CAGTTCTTTG TGGCTCTGAA GAATTGGNCG 300
CTGTGGAAAA GTN 313

SEQ ID NO:4914
SEQUENCE LENGTH:124
SEQUENCE TYPE:nucleic acid
TOPOLOGY:linear
CLONE:HUMGS05851
SEQUENCE DESCRIPTION:
GATCTTATGT GTTACAAAAT TGGAGAAAGT ATTTAATAAA ACCTGTTAAT TTTTATACTG 60
ACAATAAAAA TGTTTCTACA GATATTAATG TTAACAAGAC AAAATAAATG TCACGCAACT 120
TAAA 124

SEQ ID NO:4915
SEQUENCE LENGTH:313
SEQUENCE TYPE:nucleic acid
TOPOLOGY:linear
CLONE:HUMGS05852
SEQUENCE DESCRIPTION:
GATCCCCTGA ATGCCTTTGG AGGCCAGTAG AGCACACAGG GTATCCACAT GTTACCCTGC 60
AGCTACATTG TTGAGTTAGT GATGATATTG TATATGCTCA TGGTCTNAAC TGGATTACAA 120
AAAGCAAATA CTAGAACAGC TAGCTCATCT TTNACCCAAT GTACTTAGTA TTTTCNTGCA 180
CTGGTTTAAT CATGNTTAAT ACTACAAANC AAAANTAANT ATTTCACAGT GGTTGGNTTG 240
TNTTGTTTTT AANCCACAGT TNGANTTAGT TAGCCTTGCT GGGGCCATAA TATGCTTCAG 300
GGTGTGTAAN TGN 313

SEQ ID NO:4916
SEQUENCE LENGTH:312
SEQUENCE TYPE:nucleic acid
TOPOLOGY:linear
CLONE:HUMGS05854
SEQUENCE DESCRIPTION:
GATCTGGGGA CTACCAGAGA CTCTGGGAAA TGGACAAGGT GGGGGGCCCC ACTCTTTCTC 60
TCCTGCAGTC CCGTAGCTGG GGCCTCCTTC CTTCTCAGGG TCTCCCCAGC CCAGTCCCCT 120
TGCTCCCATC CCACTCGGTG CTGTTGGGTA GGGGCCCTGC CAGGAACTGA CCAGCTNAGC 180
GAGGAGCCAT AATGTGCATA TGTGCACAAG CAGGGTTTGG GGAGGGGGNT GTGAGGGGNT 240
GTGCCCAGGT GTTGCCCCCT ATCTCCTGGG GAGGGTGAGG NAGGGCAGGG ACAGTNTCCA 300
GGGGTNAATT TN 312

SEQ ID NO:4917
SEQUENCE LENGTH:55
SEQUENCE TYPE:nucleic acid
TOPOLOGY:linear
CLONE:HUMGS05856
SEQUENCE DESCRIPTION:
GATCTAAATA TTCTGGTTTG GATTAGGCAA TTAAAATTTT TTTGGCAACA GGAAA 55

SEQ ID NO:4918
SEQUENCE LENGTH:370
SEQUENCE TYPE:nucleic acid
TOPOLOGY:linear
CLONE:HUMGS05857
SEQUENCE DESCRIPTION:
GATCCAAGGA NCGTTTGGNT TTTCCTAGCT ACATCTGGTA CCTTGGCTGG CATTATGGGA 60
ATNAGGTTCT ACCACTCTGG AAAATTCATG CCTGCAGGTT TAATTGCAGG TGCCAGTTTG 120
CTGATGGTCG CCAAAGTTGG AGTTAGTATG TTCAACAGAC CCCATTAGCA GAAGTCATGT 180
TCCAGCTTAG ACTGATGAAG AATTAAAAAT CTGCATCTTC CACTATTTTC AATATATTAN 240
GAGAAATAAG TGCAGCATTT TTNCATCTGN CATTTTACCT AAAAANAAAG ACACCAAANC 300
TTGGCAGAGA GGTGGGAAAA TCAGTCATGT TTTACCAANC CTANCNGAGG TTGGCGTGTT 360
TTGTTACCTN 370

SEQ ID NO:4919
SEQUENCE LENGTH:168
SEQUENCE TYPE:nucleic acid
TOPOLOGY:linear
CLONE:HUMGS05858
SEQUENCE DESCRIPTION:
GATCTTGAAA GGAAGTATGC TGTTCTCTAT CAGCCTCTAT TTGATAAGCG ATTTGAAATT 60
ATTAATGCAA TTTATGAACC TACGGAAGAA GAATGTGAAT GGAAACCAGA TGAAGAAGAT 120
GAGATTTCGG AGGAATTGAA AGAAAAGGCC AAGATTGAAG ATGAGAAA 168

SEQ ID NO:4920
SEQUENCE LENGTH:128
SEQUENCE TYPE:nucleic acid
TOPOLOGY:linear
CLONE:HUMGS05859
SEQUENCE DESCRIPTION:
GATCATGGGA GGGAGCTGAG GGGTTAATAT ATATACATAC ATACACATAT ATATATTTNN 60
GTAAATAAAC AGGAACTGAT TTNCTGCCTC CATCCCACCC ATGAGGGCTG CAGGNACTAC 120
AAGAGAAA 128

SEQ ID NO:4921
SEQUENCE LENGTH:166
SEQUENCE TYPE:nucleic acid

TOPOLOGY:linear
CLONE:HUMGS05860
SEQUENCE DESCRIPTION:
GATCGGGTCC CCAGAGCCAC CATCTCCTGA GCCTCCCACC CCGCTGCCTG GGCCCTGTGG 60
TTGCTGGGCC TCCCACCTCA AGGAGGGGAA GGTTGTACAG CCCGAACCCG TGGAGCAATG 120
CCCTGTNTGG NCTCCAAAAC CAAAATAAAA CTGGTGTCAC TTTAAA 166

SEQ ID NO:4922
SEQUENCE LENGTH:97
SEQUENCE TYPE:nucleic acid
TOPOLOGY:linear
CLONE:HUMGS05862
SEQUENCE DESCRIPTION:
GATCTTTAAA CTGCTTTATA CACTGTCACG TGGCTTCATC AGNTGTGTGC ATTTCAGGAT 60
GGTTTTTAAA GNAACCTCAG AAAGNTATTT CCTTAAA 97

SEQ ID NO:4923
SEQUENCE LENGTH:211
SEQUENCE TYPE:nucleic acid
TOPOLOGY:linear
CLONE:HUMGS05863
SEQUENCE DESCRIPTION:
GATCACAGAA AAATTAAAAA TCCAAGTGCT CTCTAGATTT GTTGATAAAC ATTTTATGCT 60
TGCATTTAAA CTTGAAATGT ATGAGCAGAA TGAGACAATC AGTTAAATCA GAAATGAGAA 120
GTATTATAAT GTAAAGGCCT TGTTTTGCTG TAGCAATANN ATGACCAAGT GCAATGACTT 180
GATTTAATAA AATCATATNT TAAAAGTTAA A 211

SEQ ID NO:4924
SEQUENCE LENGTH:338
SEQUENCE TYPE:nucleic acid
TOPOLOGY:linear
CLONE:HUMGS05864
SEQUENCE DESCRIPTION:
GATCAACGCC TCACTGAAAC ATGGCTGTGT TTGCAGCCTG CTCTAGTGGG ACAGCCCAGA 60
GCCTGGCTGC CCATCATNTG GCCCCACCCA ATCAAGGGAA GAAGGAGGAA TGCTGGACTG 120
GAGGCCCCTG GAGCCAGATG GCAAGAGGGT GACAGCTTGC TTTCCTGTGT GTACTCTGTC 180
CAGTTCCTTT AGAAAAAATG GATGCCCAGA GGACTCCCAA CCCTTGGCTT GGGGTCAANG 240
AAACAGCCAG CAAGGNGTTA GGGGNCTTAG GGCACTTGGG CTTGTTTGTT CCCATTTGAA 300
NGGCGGNCTT NTGGGCCCTG GGCCCTTNAG GTTTGGTN 338

SEQ ID NO:4925
SEQUENCE LENGTH:259
SEQUENCE TYPE:nucleic acid
TOPOLOGY:linear
CLONE:HUMGS05865

SEQUENCE DESCRIPTION:
GATCCTGTCT TCTTTCATGT TTACTACGAG GCATGACATT CATGGCAAAA CAGGGATGCC 60
GTTTCCAAGA GCAGCTATAA AACAATCTGT TTTCCTAAAG ACAGCCACTC TGAGAAGGNG 120
ATGANGGAAC ACGGCAGGGT TCATCANTTC ATAGACTGTT TCCAAAAATA ATTTTTAAAC 180
TTTTCCACTA GGCATCTGTG TCTTTAACCA CTGCACANTG GCNTTGCCTG TTTAATANAA 240
GGTNNTCANN ACGTGGAAA 259

SEQ ID NO:4926
SEQUENCE LENGTH:195
SEQUENCE TYPE:nucleic acid
TOPOLOGY:linear
CLONE:HUMGS05866
SEQUENCE DESCRIPTION:
GATCCACCCA CCTCGGCCTC CCAAAGTTTT GGGATTACAG GCACGAGCCA CAGTGCCAGC 60
TGAAAACGTT TCCTAAACAG AAGTGCGCAG AGCATGATGG AGCCCAGCCT GGGCTTGACA 120
TTTGGAGAGA ACTACAGAGA AGTATCCAGA CTGTTGTTAC TAATAAATAA ATACATAAGC 180
TCCGCTGNAC GGAAA 195

SEQ ID NO:4927
SEQUENCE LENGTH:56
SEQUENCE TYPE:nucleic acid
TOPOLOGY:linear
CLONE:HUMGS05867
SEQUENCE DESCRIPTION:
GATCCAACAA TAAAATATTA ATTTATAAAA AAGAAATTTT AAAAAGTAAC AAGAAA 56

SEQ ID NO:4928
SEQUENCE LENGTH:82
SEQUENCE TYPE:nucleic acid
TOPOLOGY:linear
CLONE:HUMGS05868
SEQUENCE DESCRIPTION:
GATCATCCTC CAAGCAGTCC TGTTCATTCA TGTCTGCCTC AAATTGCTGG TGAATACATA 60
AAATAGTTAC TTGCTAATCA AA 82

SEQ ID NO:4929
SEQUENCE LENGTH:286
SEQUENCE TYPE:nucleic acid
TOPOLOGY:linear
CLONE:HUMGS05869
SEQUENCE DESCRIPTION:
GATCCACCCG CCTCAGCCTC CCAAAGTGCT GGGATTACAG GCAAAAGCCA ACACACCCGG 60
CCTGGATATG AATTTATAAT ACCCTACAGT GCAGCACAAG AAGATGCACT CAAAGCACTG 120
ATGTGAGGAA GTACTTGCCC CGTAGCAGCT ATTCACTCTA CCAGTGTAAA CAAACTCTAA 180
GGCTAGGGCG GGGCAGCACA GGCACANGNN TATACTAGCC AGGGGTTTAA TATAANTACA 240

ACCAGCATAG NAAGNCCCAA ANCTNTACAG NNNCCAANAC CNGGNN 286

SEQ ID NO:4930
SEQUENCE LENGTH:311
SEQUENCE TYPE:nucleic acid
TOPOLOGY:linear
CLONE:HUMGS05870
SEQUENCE DESCRIPTION:
GATCTGTGCC CCATGCCTGT CCAGCCTGGG CAGCCAGGCT GCCAAGGCCA GAGTGGCCTG 60
GCCAGGGGCT CTTCAGGCCT CCCTCTCTCT TCTGCTCCAC CCTTGGCCTG TTTCATCCCC 120
AGGGGTCCCA GCCACCCCGG GCTCTCTGCT GTACATATTT GAGACTAGTT TTTATTCCTT 180
GTGAAGATGA TATACTATTN TGNTNAAGCG TGTCTGTANT TANTGTGTGA GGANCTGCTG 240
GGNTTCANTT CGNGTGNACG GTGGGAGAGN TTGGGTGCCC GNGAGAATNG ACGGGCTNAT 300
GGNTTCCTTT N 311

SEQ ID NO:4931
SEQUENCE LENGTH:305
SEQUENCE TYPE:nucleic acid
TOPOLOGY:linear
CLONE:HUMGS05871
SEQUENCE DESCRIPTION:
GATCAAAAAA CTACCTATCT GGTACTATGC TTTTNATCTG GATGATGAAA TAATCTGTAC 60
AACAAACCCT GGTGACATGC AATTTACCTA TATAGCAAGC CTACACATGT GCCCCTGAAC 120
CTAAAAAAAA AGTTAAAAGA AAAACGTTTG GNTTATTTTC CCTCTTTNGA ACAANGNCAT 180
TGGTTTGCCC AAGGNCTACA AATAANCCAA CGGGAAAAAN GANAGGTTCC AGTTTTGTNT 240
GANAATTCTG ATTAAGCCTC TGGGCCCTAC AGCCTGGGGA ACCTGGNGAN TCCTACACCC 300
GGGAN 305

SEQ ID NO:4932
SEQUENCE LENGTH:233
SEQUENCE TYPE:nucleic acid
TOPOLOGY:linear
CLONE:HUMGS05872
SEQUENCE DESCRIPTION:
GATCCAGAGA CACACAAGAG CACCAAAGCA GCTCATCCCA CTGATGACAC CACGACGCTC 60
TCTGAGAGAC CATCCCCAAG CACAGACGTC CAGACAGACC CCCAGACCCT CAAGCCATCT 120
GGTTTTCATG AGGATGACCC CTTCTTNTAT GATGANCACA CCCTNCGNAA ACGGGGTCTG 180
TTGGTCGNAG NTGTGCTNTT CATCACAGGG ATNATNATTN CTNACCAGTT GGN 233

SEQ ID NO:4933
SEQUENCE LENGTH:276
SEQUENCE TYPE:nucleic acid
TOPOLOGY:linear
CLONE:HUMGS05874
SEQUENCE DESCRIPTION:

GATCTACTAA AACACTAAGT TTCATACTAA ACCGTTTCAT GTATCTTTAT CATATTCTGT 60
AAAGTTTACA CTTTGATGTA CCTGTATTTT ATTGAAATAC TGTCCAGCTT CAAATGGAAC 120
AATATTGTAT GTAGAAATTC CAATTACTGG TTTGTCAGTN GGATTCTTAG CTCTGTAAAA 180
CGCCAGTGCA GTCTCTCCTG GCACCACCTG TNTTAAAGAG TATATATATN ATCAGTNCTT 240
TGNNTCTCAC AAAGNGCCTC NNTTTCCCTG CTAAGN 276

SEQ ID NO:4934
SEQUENCE LENGTH:89
SEQUENCE TYPE:nucleic acid
TOPOLOGY:linear
CLONE:HUMGS05875
SEQUENCE DESCRIPTION:
GATCTGGTGG GCTGGGTCCC CCTCTCGACC NCAGACGCCC CTAACTCGCC CCTTCTCATT 60
TTATACAATA AACATTCTCC ACCTACAAA 89

SEQ ID NO:4935
SEQUENCE LENGTH:313
SEQUENCE TYPE:nucleic acid
TOPOLOGY:linear
CLONE:HUMGS05876
SEQUENCE DESCRIPTION:
GATCAGGAGT TCTAGGCTGT AGTGTGCTAT GAGCACATCT ATGAATAGGC ACTGCGTTCC 60
AGCCTGGGCA ACATAGTGAG GCCCCATCTC TTAAAAGGGC ACACGTGTAT AGGACAGCTC 120
CATTATAATC TTATGGGACC ACTGTAATAT GTGTAATCCA TTGTTGACTG GAACATCCTT 180
ATGCAGCACA TGACTGTGTA CATAACTTAA AATTTATCAT TTAAACCATT TTTAAGTGTA 240
CAATTCGGTG GTATAAAGTA CATTTACATT ATTGTGTAAC TATCACCTCT ATCCATCTCC 300
CTAACTTTTT CNN 313

SEQ ID NO:4936
SEQUENCE LENGTH:224
SEQUENCE TYPE:nucleic acid
TOPOLOGY:linear
CLONE:HUMGS05877
SEQUENCE DESCRIPTION:
GATCCGGGTT CAGAGTGACG TTTTTCCTCA CAAGCAGAAG GTAACAAATT TCATGCTCTC 60
CCTAAATTCT GTCTGATTTT GCCTNGTGGT GATAGATTGT CANGAACACA ATGTCCTCTG 120
GAGAAATCTA TTGACAGAAA TTGGTGCAGT GTTANCAACG CTAATGTAAA ACACAGANTT 180
TACAGAAAAN TAGAGAAAAT AAACACATTT GTNNGCCTCA GAAA 224

SEQ ID NO:4937
SEQUENCE LENGTH:314
SEQUENCE TYPE:nucleic acid
TOPOLOGY:linear
CLONE:HUMGS05879
SEQUENCE DESCRIPTION:

GATCCTCTCT CCCAGGGAAT CCGACACAGG AGGAACCCCT TCTNTGGTTG ANCTGGGCCA 60
GGCCTAAGAG TAGCAGGAAC TCTAAGACCA CAGAGTTTTT TATAAATGTA TAAATGTATC 120
AAGCCAAATN TGCAGATGCT AACTGGACAT TCTGGGGAAC TGGGCACCAG GNGTGCCTTC 180
ATACACTGTA CCCCAGCTCT NTTCTAAAAG AGAAGTNGGT GGGCACACTT GAACTGTTTG 240
GTGGNCCNAA CCACAGGANG CTGCAATTNT NTGGNTTAGG GTGATACTTT TGGCCCTNCN 300
TGTGGCCCCT NTTN 314

SEQ ID NO:4938
SEQUENCE LENGTH:143
SEQUENCE TYPE:nucleic acid
TOPOLOGY:linear
CLONE:HUMGS05880
SEQUENCE DESCRIPTION:
GATCTGAAAG TATAAAACTG CTAGAAGAAA ACATGGATAT TTGATTGGAC AATGATATTT 60
GGATATGACA CCAAAAGCCT AGGCAACTAA TGCAAAAATA GACAGGGAGA GTACATCAAT 120
CTTAAAAACC TTATGATGGC AAA 143

SEQ ID NO:4939
SEQUENCE LENGTH:389
SEQUENCE TYPE:nucleic acid
TOPOLOGY:linear
CLONE:HUMGS05882
SEQUENCE DESCRIPTION:
GATCTGAAAT ACCATCTCTA CCAGAGACTC TGGGAAAAAT AATGGATTCC AAGTGTTAAA 60
CGGAATTAAG TTTGGCCTAA AACTGCCTCT GTACATAGTG ACTTATAACC TCACTTAATG 120
TATAAGCAAA CTGGGACCTG ACTTGAGAGT ACACTTCTGT AACAAGCAGC AGAGTCTCAC 180
CAATCACATG CTGAAGGCTC CCAAAACATG NCTATGTGAG GTGGAGGCTG ACCTGCAGTC 240
AGTCTGGCCA TGTACATGNG TCTTTCCCTT NCTGTGGCTA TAAATAAATA CAGCCTGNAC 300
ACANCGGACA GGNGGACCGT TCTGNACCAN GNTTGGGTCC TGNGNGNTAC CNAGGTNGAN 360
GNAACTTTTT TTNNGCTCAN TTAAACTGN 389

SEQ ID NO:4940
SEQUENCE LENGTH:131
SEQUENCE TYPE:nucleic acid
TOPOLOGY:linear
CLONE:HUMGS05885
SEQUENCE DESCRIPTION:
GATCTCTCCG CCTTAGTTAA GAGCATGTGT CGGGAAATTC CTCAGAGTGC TCAGAGTCCC 60
TGTATNTTTA TACCTTTTTA CAATGTTAAC TGTTCAGAAC TGNTTTTTGT AACAAAACCT 120
NNTGTTTTAA A 131

SEQ ID NO:4941
SEQUENCE LENGTH:115
SEQUENCE TYPE:nucleic acid
TOPOLOGY:linear

CLONE:HUMGS05886
SEQUENCE DESCRIPTION:
GATCATGGCG CAGTTGGNCA GGAGATGTTC TGCCCCATCT GCTGTAGCGA ATATGTGAAG 60
TGGGAGGTGG CAACNGAGCT GCCNTTCCAC CACTATTNCC ACAAGCCGTG TGTNN 115

SEQ ID NO:4942
SEQUENCE LENGTH:105
SEQUENCE TYPE:nucleic acid
TOPOLOGY:linear
CLONE:HUMGS05887
SEQUENCE DESCRIPTION:
GATCCTCCCG CCTTNGTCTG CCAAAGCACT NGGGTTACAG ATGTGAGCCA CTNCACCCAG 60
CCTAATCTTN ATAAATCTCC ATCCCCATTA CTCTGGTTCA CCCNN 105

SEQ ID NO:4943
SEQUENCE LENGTH:350
SEQUENCE TYPE:nucleic acid
TOPOLOGY:linear
CLONE:HUMGS05888
SEQUENCE DESCRIPTION:
GATCACGGAC GTGCAGCTCG CCATCTNCGN CAACATGCTG GGCGTGTCGC TCTTCTNGCT 60
TGTNGTTCTC TATCACTACG TGGCCGTCAA CAATCCCAAG AAGCAGGANT GAAAGTGGCG 120
CTTTCTCCGC CCCAGGGTTC CAGGACATAG TCTGAGGCAA GATGGAGGGT ATGAGGGGCC 180
TTCACACTNC ACTTCATCCC TTCTACCCAT CACAACATAC AAAGCAACTA CACCTGGGAT 240
TTTNCCAAGC AGCTTTNATT TCCTCAGNGT CTTCCTTAAT CCTATGGGNA CANGGAAGCT 300
GCCACTNGNN TAGGGNCCAG TATAGGGGGC TTNGCTTTTT TAACTGCNTN 350

SEQ ID NO:4944
SEQUENCE LENGTH:304
SEQUENCE TYPE:nucleic acid
TOPOLOGY:linear
CLONE:HUMGS05889
SEQUENCE DESCRIPTION:
GATCCAGCGG CAGTGACAGA ATCCAAAGAG GGAACAGAGG CATCAGCATC GAAGGGGCTG 60
GAGAAGAAAG AGAAATGATG CAGCTGGTGC CCGAGCCTCT CAGGGCCAGA CCAGACAGAT 120
GGGGGCTGGG CCCACACAGG CGTGCACCGG GTAGAGNGCA CAGGTAGGCC AAGGGGNAGC 180
TCCCAGGACA GGGCAAGGGG GCAGCANGGA TACCTGCNAG CCAGGGNCTC TNTGGCCTNT 240
NTTTTCCTAN TCCNTTTTTT TGGCCCTTCT TTTTTNTNTG CCGTACANCN TGCAGGCAAA 300
AGNN 304

SEQ ID NO:4945
SEQUENCE LENGTH:64
SEQUENCE TYPE:nucleic acid
TOPOLOGY:linear
CLONE:HUMGS05890

SEQUENCE DESCRIPTION:
GATCAAAACA AAACAAAAAA GTTGTCTCTG AAAAAAAAAC AAAACAAAAA AGTTGTCTCT 60
GAAA 64

SEQ ID NO:4946
SEQUENCE LENGTH:136
SEQUENCE TYPE:nucleic acid
TOPOLOGY:linear
CLONE:HUMGS05892
SEQUENCE DESCRIPTION:
GATCAACCTT TGTNTGCGAG GGTCTAAGTA GGGTCGAACA CAGAAGTGGG AAGNAGAGGG 60
GTGGGCCAGG GGCTAATGGT GTCACTGTGT AAAGTTTTTG ACATACTAGC TCTATAAATA 120
TATGAATATG GACAAA 136

SEQ ID NO:4947
SEQUENCE LENGTH:162
SEQUENCE TYPE:nucleic acid
TOPOLOGY:linear
CLONE:HUMGS05893
SEQUENCE DESCRIPTION:
GATCCTCTGC TACCAAACAA TGATGTGGAT TCTTTTGCAC AGAAATATTT AAGGTGGGAT 60
GGTAAAAAAT GTCACAAAAG ACTCCTCACC AATACTTNAT GTTGATATCA CTTAATATTA 120
ACCAGACTTT GCTGTATTGC AATAAAACAG AGAACTGTTA AA 162

SEQ ID NO:4948
SEQUENCE LENGTH:405
SEQUENCE TYPE:nucleic acid
TOPOLOGY:linear
CLONE:HUMGS05894
SEQUENCE DESCRIPTION:
GATCATCGCC TCCCTGGTTT ATAGTAGACA GACCTTTTCT GTTTTTCATC CGACATAATC 60
CTACAGGTGC TGTGTTATTC ATGGGGCAGA TAAACAAACC CTGAAGAGTA TACAAAAGAA 120
ACCATGCAAA GCAACGACTA CTTTGCTACG AAGAAAGACT CCTTTCCTGC ATCTTTCATA 180
GTTCTGTTAA ATATTTTTGT ACATCGCTTC TTTTTCAAAA CTAGTTCTTA GGAACAGACT 240
CGATGCAAGT GTTTCTGTTC TGGGAGGTAT TNGAGGGAAA AANCAAGCAG GATGGCTGGA 300
ACACTGTACT GAGGAATGAA TAGAAAGGCT TCCAGATGTC TAAAAAGATT CTTTTAAACT 360
ACTGNAACTG TTACCTAGGT TAACCANCCC TGTTTGAGGT ATTTN 405

SEQ ID NO:4949
SEQUENCE LENGTH:330
SEQUENCE TYPE:nucleic acid
TOPOLOGY:linear
CLONE:HUMGS05895
SEQUENCE DESCRIPTION:
GATCTGCTCG GAATCCTCCT GCTAAAGAAG GNTCTGGGCG TGAGCTCAGC CGCCCCGCAC 60

CCCAAATCCA GTACAGGAGG CTGCTGGTAC AAATACTGCA GTAATGACAC CAATAATGGG 120
GCAGACACAA CAGCGTGGCT TAGATTGTGC CCACCNAGGG AAGGTGCTGA ATGGGACCCT 180
GTTGATGGCC CCATCTGGAT GTAAATCCTG AGCTCAAATC TCTGTTACTC CATTACTGTG 240
ATTTCTGGCT GGGTCACCAG AAATATCGNT GATNCAGACA CAGATTATTG TTCCTGCTGT 300
ATTTGCTGGT TTNCCTGGTT GAATTGGTTN 330

SEQ ID NO:4950
SEQUENCE LENGTH:308
SEQUENCE TYPE:nucleic acid
TOPOLOGY:linear
CLONE:HUMGS05896
SEQUENCE DESCRIPTION:
GATCCAGAAT TTATTAGTTT AAAATGCAGG TGAACTTTTT TTTGCGTTTG GTTTACTTGT 60
CTGTCAAATG TTTCCTTAAA CATGAAACTG AATAAGGAGA AGAGTATTTT TAACACTTAA 120
ATTTCTTGGC AAATTTTAAA ACATTTTTNA GTCTGTAATA CACTCCACTT GANGCACTTA 180
AGTNTTCCTT AAATGACTTT CCTTAGGTAA TGATACTGGT GTGTTTTCCN AAGGCCCTTT 240
TAAAAAAGTN TNNNTAANTG ACTATCTGTT GAAANGGTGT CCTGTNCCTT NCTNCTAGNA 300
ATNTGGGN 308

SEQ ID NO:4951
SEQUENCE LENGTH:97
SEQUENCE TYPE:nucleic acid
TOPOLOGY:linear
CLONE:HUMGS05897
SEQUENCE DESCRIPTION:
GATCGGAAGC AGAAGATGGC CTTCTTAAAG AGTTTAGAAG AATTTATGGC AAATGTTGGT 60
GGTCTCCTTT GTGTAAAATA AACAACAGAA CTTTAAA 97

SEQ ID NO:4952
SEQUENCE LENGTH:229
SEQUENCE TYPE:nucleic acid
TOPOLOGY:linear
CLONE:HUMGS05898
SEQUENCE DESCRIPTION:
GATCTGATAT TAAAACATCC AGTAGTACGA GTCTGTTTAT TGAATTTTAT GGTCCCTTTC 60
TTTGATGGTG CTTGCAGGTT TTCTAGGTAG AAATTATTTC ATTATTATAA TAAAACAATG 120
TTTGATTCAA AATTTGAACA AAATTGTTTT AAATAAATTG TCTGTATACC AGTACAAGTT 180
TATTGTTTCA GTATACTCGT ACTAATAAAA TAACAGTGCC AATTGCAAA 229

SEQ ID NO:4953
SEQUENCE LENGTH:144
SEQUENCE TYPE:nucleic acid
TOPOLOGY:linear
CLONE:HUMGS05899
SEQUENCE DESCRIPTION:

GATCCAGAGC CCCTCCCCAT CTTCCTCTCT CTAAAAACAA CCCTACCCCC CATTGCCACC 60
TTCACTCCTG TGTCTCCAGC TGATTAGCCT CAGACTCTTC TTNTATTGTT TTTCTTTTGT 120
AAATAAAAAG CACCAGGTTC CAAA 144

SEQ ID NO:4954
SEQUENCE LENGTH:105
SEQUENCE TYPE:nucleic acid
TOPOLOGY:linear
CLONE:HUMGS05900
SEQUENCE DESCRIPTION:
GATCCGTGCC CTGTAAAATG GATATNATGT TTTACTGATG TCTNTAATAC ATTTGTAAAC 60
TTCCAATAAA ATTTGAATAA AAGAAATNTT GCCATTCTTC TCAAA 105

SEQ ID NO:4955
SEQUENCE LENGTH:325
SEQUENCE TYPE:nucleic acid
TOPOLOGY:linear
CLONE:HUMGS05902
SEQUENCE DESCRIPTION:
GATCTGGCAG CCCTGGAGAG GGACTATGAG GAAGTGGCGC AAAGTTTCTG AGGCATGTAT 60
GATGGGTGCA AATGAATGTC AAGTTAAAAT GGCATGTTTT CTTTTCAAGC CGNTATATGC 120
CTAGTGGGTA GTTCCCCTGA TGAGCAGAAA AATTGAACTC AAATCAGGCA AGACTCTAGG 180
NTCCACACTA AATTAAGTGG GTCAGTANCG NCANTGANCA CATTATTTCC TGGTCTACTG 240
GGTACANGTC AGCTCTGCTT ACCTGNGGAG CNTTTTGGAA GCTTAAGAGT NCTTTTGAAC 300
CTGGCTTGAG GTCAACTTGT TAGGN 325

SEQ ID NO:4956
SEQUENCE LENGTH:321
SEQUENCE TYPE:nucleic acid
TOPOLOGY:linear
CLONE:HUMGS05903
SEQUENCE DESCRIPTION:
GATCTGTACA GACAGGACAG AATGAAACTC CTGCGGCTCT TTGGCCTGAA AGTTGGGAAT 60
GGTTGGGGGA GAGAAGGGCA GCAGCTTATT GGTGGTCTTT TCACCATTGG CAGAAACAGT 120
GAGAGCTGTG TGGTGCAGAA ATCCAGAAAT GAGGTGTAGG GAATTTTGCC TNCCTTCCTG 180
CAGACCTGAG CTGGCTTTGG AATGAGGTTA AAGTGTCAGG GACGTTGCCT GAGCCCAAAT 240
GTTGTAGTGT GGTCTGGGCA GGCAGACCTT TTAGGNTTTT NCTGCTTTAG TTCCTGAGGN 300
ANNTNGGNCA CTCTTTNTNT N 321

SEQ ID NO:4957
SEQUENCE LENGTH:313
SEQUENCE TYPE:nucleic acid
TOPOLOGY:linear
CLONE:HUMGS05907
SEQUENCE DESCRIPTION:

GATCCAGGGG CTGATTGGCA GCGTGGAGGA GCAGCTGGCC CAGCTTCGCT GCTGAGATGG 60
AGCAGCAGAA CCAGGAATAC AAAATCCTNC TGGATGTGAA GACGCGGCTG GAGCAGGAGA 120
TTGCCACCTA CCGCCGCCTN CTGGAGGGAG AGGATNCCCA CCTGACTCAG TACAAGAAAG 180
AACCGGTGAC CACCCGTCAG GTGCGTACCA TTGTGGAAGA GGTCCAGGAT GGNAAGGTCA 240
TCTNCTNCCG NGNGCAGGTT CACCAGACCA CCNGTTTGAG GNCTCANCTA CCCCGGNTCG 300
GNCANCCATN TNN 313

SEQ ID NO:4958
SEQUENCE LENGTH:54
SEQUENCE TYPE:nucleic acid
TOPOLOGY:linear
CLONE:HUMGS05908
SEQUENCE DESCRIPTION:
GATCATTAAT GTCATTGCAA TNGTTATTTT TTAAAATAAA TTTATAAAAA TAAA 54

SEQ ID NO:4959
SEQUENCE LENGTH:208
SEQUENCE TYPE:nucleic acid
TOPOLOGY:linear
CLONE:HUMGS05912
SEQUENCE DESCRIPTION:
GATCTACAAG AGCAAGCCAA CAGAGTAAAG TGGAAGGAAG TTTATTCAGA AAATAAAGGA 60
GTATCACAGC TCTTTTAGAA TTTGTCTAGC AGGCTTTCCA GTTTTTACCA GAAAACCCCT 120
ATAAATTAAA AATTTTTTAC TTAAATTTAA GAATTAAAAA AATACAAAAA AGAAAAAATG 180
AAAATAAAGG AATAAGAAGT TACCTAAA 208

SEQ ID NO:4960
SEQUENCE LENGTH:92
SEQUENCE TYPE:nucleic acid
TOPOLOGY:linear
CLONE:HUMGS05913
SEQUENCE DESCRIPTION:
GATCCAAGTT ATAGATGGAA TTGAAGCCCT TGTNGCTGTA GATGTGCGTG CAGTCTGNCA 60
GCCTTAAGCC CACCTNGGCA CTTTTAGATA AA 92

SEQ ID NO:4961
SEQUENCE LENGTH:270
SEQUENCE TYPE:nucleic acid
TOPOLOGY:linear
CLONE:HUMGS05914
SEQUENCE DESCRIPTION:
GATCAACAGT AACTGGATGT TTTTNAGGNG CTCAATTGGA ATAAAAATAT TNNAATCTAT 60
TTGGAGACCA AAGGCAAAAT CGGTTTTCTT ACCTTTGGAA TTATTCGTAC CTTTTATGGT 120
AAATTTCAGC TTTGACANGT ATTATGAGGA ACGTACCAAA AACCGGTTTG TAACAAATCT 180
NTAGAGNAGG TCTGAATCTN TCGNNCTTGC CTTTTCAGGT GCCATTTCTA CTGGCCTAAT 240

ACAGNGCCAT TTGGCTTGTG AAGNNCCATN 270

SEQ ID NO:4962
SEQUENCE LENGTH:236
SEQUENCE TYPE:nucleic acid
TOPOLOGY:linear
CLONE:HUMGS05916
SEQUENCE DESCRIPTION:
GATCTATGCC CNTNTTAATC TCCATCCATA TAGGAGTCAC GTTCTTTAAG ACAGATGGTG 60
GTAGTTATTT TTGTGGCATG GTTAGATTTG ACTGGTTTTG CAGAAGATTA CAGTTATGTA 120
CTGCATAATG NCATATACAA TAGTGGTCCC ATAAANNNNN TAATGGAGCA GAAAATCTAT 180
TGCCTCATGA TGTTTTAGCC GGTTTANTGT CATAGNCTAG TGCATNACTC ACGTGN 236

SEQ ID NO:4963
SEQUENCE LENGTH:265
SEQUENCE TYPE:nucleic acid
TOPOLOGY:linear
CLONE:HUMGS05917
SEQUENCE DESCRIPTION:
GATCGAGTAA GGAATGACCT NTAGATTGTG CGACTTTTGT TTTTGTTTTN TTAAATTTTT 60
ATAAACCAAG AATGATTTCT CCNGCTTCCN TCTCCTCACC ATCTTCCCAG ACGGAGTTCA 120
AAGGCCACTT CTCAAGCAGC TTTTGGCACC TTCAGCCTCA GAGTGGAATC TTTTAAAGAC 180
AGGACCCCTA TGTCCAGGNA AGGGGAAAAG GAACNTTGCC ANTGATAGTG ACCACAGCAN 240
NNGCAANTAA TAGTAATNTT NATAN 265

SEQ ID NO:4964
SEQUENCE LENGTH:54
SEQUENCE TYPE:nucleic acid
TOPOLOGY:linear
CLONE:HUMGS05918
SEQUENCE DESCRIPTION:
GATCACATTT ATACAGTTAT AAAAATAAAG GTTTGATTTT GGTCGTTCTT CAAA 54

SEQ ID NO:4965
SEQUENCE LENGTH:63
SEQUENCE TYPE:nucleic acid
TOPOLOGY:linear
CLONE:HUMGS05919
SEQUENCE DESCRIPTION:
GATCTCTATG GCTGTAAAAT ATTTCTTTAT AGCGTTATTA AAGTGTGTCT TAATAAAATT 60
AAA 63

SEQ ID NO:4966
SEQUENCE LENGTH:233
SEQUENCE TYPE:nucleic acid

TOPOLOGY:linear
CLONE:HUMGS05920
SEQUENCE DESCRIPTION:
GATCCACAAA AGAAGTGAAA GTAGCCTTAA CTGATGACAT TCCACCATTG TNATTTGTTC 60
CTGCCCCACG CTAACTGATA CCATATATTC TTCCCCCGCC CTTGAGAATG TACTTTGTAC 120
ACCTATCCCA AACCTATAAG AACTAATGAT AATCCNACCA CCCTTTGCTG ACTCTCTTTT 180
TGGACTCAGC CCGNCTGCAC CCAGGTGAAA TAAACAGCCC TGTTGCTCAC AAA 233

SEQ ID NO:4967
SEQUENCE LENGTH:137
SEQUENCE TYPE:nucleic acid
TOPOLOGY:linear
CLONE:HUMGS05921
SEQUENCE DESCRIPTION:
GATCTCTGCG TTTTTATACT GAGTGTGCCT AGGTTGCCCC TTATTTTTNA TTTTCCCTGT 60
TGCGTTGCTA TAGATGAAGG GTGAGGACAA TCGTGTATAT GTACTAGAAC TTTTTTATTA 120
AAGAAACTTT TCCCAAA 137

SEQ ID NO:4968
SEQUENCE LENGTH:90
SEQUENCE TYPE:nucleic acid
TOPOLOGY:linear
CLONE:HUMGS05923
SEQUENCE DESCRIPTION:
GATCTGTAAA CCCTTACCCC TTGCTGTTCA GAGAGCTACT CTTTGTAGTG TTCTTGCATG 60
CATATATAAT AAATATTTTT TCTATTGAAA 90

SEQ ID NO:4969
SEQUENCE LENGTH:259
SEQUENCE TYPE:nucleic acid
TOPOLOGY:linear
CLONE:HUMGS05925
SEQUENCE DESCRIPTION:
GATCTAAATC AATGCAATAA AGTAAGAAAA ATAAATAAAA GTATTGAAAA GATTGAAAAG 60
AAAGAATTGA AGCTGTCTTT ATTCACAGAT AATGACTGTG TATGTTAATA ATCCTAGANA 120
TCTACAAAAN TCTGCCAAAN CTAATTAGTG AGTTTGGTAA TGTTGCAGGA TATAAGCTCA 180
ATAGAAGTAG TCTTTNGTAT TTCTGTGTAT TAGCAGTGAG CATTTGGAAA ATGANATAAN 240
ANTACAGTTT CATTTCAAA 259

SEQ ID NO:4970
SEQUENCE LENGTH:350
SEQUENCE TYPE:nucleic acid
TOPOLOGY:linear
CLONE:HUMGS05926
SEQUENCE DESCRIPTION:

GATCTGTGCC CTTGGTCCCA GGTCAGGCCC ACCCCCTGCA CCTCCACCTG CCCCAGCCCC 60
TGCCTCTNCC CCAAGTGGGG CCAGCTGCCC TCACTTCTGG GGTGGATGAT GTGACCTTCC 120
TTGGGGGACT GCGGAAGGGA CGAGGGTTCC CTGGAGTCTT ACGGTCCAAC ATCAGGACCA 180
AGTCCCATGG ACATGCTGAC AGGGTCCCCA NGGAGNCCCC GTCAGTAGGG ATGTTTGCCT 240
GGNTGTTTAC GTNGGGGTGT NCAGTGNACG TGNAGGAGCA CGTGGGCGGC TTCTGNGGGG 300
GNCAATGGTT TGGGGNANGG GAAGGTGTGC CAGCAAACCT NGGNNGGATN 350

SEQ ID NO:4971
SEQUENCE LENGTH:202
SEQUENCE TYPE:nucleic acid
TOPOLOGY:linear
CLONE:HUMGS05927
SEQUENCE DESCRIPTION:
GATCCACGGG CTGCAGAATG GATATTGTAT TAGCAGGCAT GAAAACAACA TTCATCTCCT 60
TGTGCATCTC TGTCTGAGTT CTTGGGTGAC CAAATGCCTT GTCAATGAGT GTAGTATTTT 120
GAAAGGAATC TTTTTTTTTT TTTTCNCAGC AGTAGGNCNC AANGGTGGGC TTCAATTATT 180
CAGTAAACTA TGTAANCAGA AA 202

SEQ ID NO:4972
SEQUENCE LENGTH:127
SEQUENCE TYPE:nucleic acid
TOPOLOGY:linear
CLONE:HUMGS05928
SEQUENCE DESCRIPTION:
GATCCCAGCT ACTCAGGAGG CTGAGGCAGA AAAATCACTT GAACCCAGGA GGTGGAGGTT 60
GCAGTGAGCA GAGACCACAC AACTGCACTC CAGTCTGGGC AACAAAGTGA GACTCTGTNT 120
TACTAAA 127

SEQ ID NO:4973
SEQUENCE LENGTH:249
SEQUENCE TYPE:nucleic acid
TOPOLOGY:linear
CLONE:HUMGS05932
SEQUENCE DESCRIPTION:
GATCCTATCA ATTAGACAGT AAATAAGTGC ATCTTTTTTG AGACAAATGC AGTAGGCAAA 60
TGTTGACATT CACCAGGGCA CAGTCAATCC CTGACAATTT CAGAAGTTTT GTTGCCAATA 120
CTGATTTATG TGGGCAGATT CTGGNAGTCT GCTGCTCCTT CNCTCATGGA TNACCTNCNN 180
NNNCCTACAG TTGCACCTAT TCTACACAAG AGTGGCATTC ANTNGTATCC AGGAAAGCTA 240
GANCAGGTN 249

SEQ ID NO:4974
SEQUENCE LENGTH:189
SEQUENCE TYPE:nucleic acid
TOPOLOGY:linear
CLONE:HUMGS05933

SEQUENCE DESCRIPTION:
GATCCGAGTC GTCCGGAAAT CCATTGCCCG TNTTCTCACA GTTATTAACC AGACTCAGAA 60
AGAAAACCTC AGGAAATTCT ACAAGGGCAA GAAGTACAAG CCCCTNGACC TGCGGCCTAA 120
GAGACACGTG CCATGCGGCN NCGCTNAACA AAGACGGGNG AACTTAAGGC CAGGAGAGCA 180
NNGNAGGAN 189

SEQ ID NO:4975
SEQUENCE LENGTH:230
SEQUENCE TYPE:nucleic acid
TOPOLOGY:linear
CLONE:HUMGS05934
SEQUENCE DESCRIPTION:
GATCATCATC TTTCAGGTGG TCTTCCTGGG CCTCCTGGCT GGCCTGGTGG TCCTATTCTA 60
CGNATATCCT GTCCNCTGTG AGTGGTATGA GTTCCTCACC TGCATCCCCT TCACTGACAA 120
GTTCTGTGAG AAGTACGAAC TAGATGCTCA GCTCCACTGA GCTNGCTGCG GGGTTNCAGC 180
GNNGGGTGTG CTCCAGCAGG GCCAGAGNCA GNCAAGANCT ACNCTGNGGN 230

SEQ ID NO:4976
SEQUENCE LENGTH:66
SEQUENCE TYPE:nucleic acid
TOPOLOGY:linear
CLONE:HUMGS05937
SEQUENCE DESCRIPTION:
GATCATTAAT TATATGTAAA ATAATATACA TTCAGTTATT AAGAAATAAA CTGCTTTCTT 60
AATAAA 66

SEQ ID NO:4977
SEQUENCE LENGTH:105
SEQUENCE TYPE:nucleic acid
TOPOLOGY:linear
CLONE:HUMGS05938
SEQUENCE DESCRIPTION:
GATCTATGTC TTTCAGCTAT GTGCTTTTAA AACTTTGTAA TTTAGACATT GATATTGTCA 60
TCAGATTGCT TATCAGCATT GCCATAAATA TAATTATTAA TGAAA 105

SEQ ID NO:4978
SEQUENCE LENGTH:162
SEQUENCE TYPE:nucleic acid
TOPOLOGY:linear
CLONE:HUMGS05939
SEQUENCE DESCRIPTION:
GATCTGCCCA CCTCGGCCAC CCAGAGTGCT GGGATTACAG NCGTGAGCAC CGTGCCTGAC 60
CAAAATAAAT AAATTTTAAA ATAGAAGTAA CTCAACTTCT GAGCTATGGA ATAGTTCTCT 120
GTACCTCCAG TACCTAATAA AGAACCCAAA TACTGCAATA AA 162

SEQ ID NO:4979
SEQUENCE LENGTH:189
SEQUENCE TYPE:nucleic acid
TOPOLOGY:linear
CLONE:HUMGS05940
SEQUENCE DESCRIPTION:
GATCGAGGAC TGGTACAGGA ACCACCAGGA GGAAGACCTG ACTGAATTCC TCTGCGCCAA 60
CCACGTGCTG AAGNGAAAAN ACACCAGTTN CCTGGCAGAG CAGTGGTCCG GCAAGAAGGG 120
AGACACAGNT GCCCTGGGGNG GGANGAAGTC CAAGAAGAAG AGCATCANNG CCAAGGCAGC 180
AGGNGTCAN 189

SEQ ID NO:4980
SEQUENCE LENGTH:66
SEQUENCE TYPE:nucleic acid
TOPOLOGY:linear
CLONE:HUMGS05941
SEQUENCE DESCRIPTION:
GATCTNTTTA AAAAATGTAT AAATTNCTTG AGTAATTCCT GGATTAAAGA ATATGCACTT 60
TTTAAA 66

SEQ ID NO:4981
SEQUENCE LENGTH:113
SEQUENCE TYPE:nucleic acid
TOPOLOGY:linear
CLONE:HUMGS05942
SEQUENCE DESCRIPTION:
GATCGCTGGC ATCAAGAGTG GAGAGGACCG NACAGCCGNG AAGGCAGCCT TTCANGAACC 60
TATGCTGGTG AGTGTAGGAG ATNGCTTANG AAGAGCTCAG ACCCTCTATC AAN 113

SEQ ID NO:4982
SEQUENCE LENGTH:316
SEQUENCE TYPE:nucleic acid
TOPOLOGY:linear
CLONE:HUMGS05943
SEQUENCE DESCRIPTION:
GATCATAGTT ATTATACAAT GTAGTGAGTC CTGCATGGGT NCTCGATGTG TAATGAAACC 60
TGAAATAATA AGATAATAAG AAAAGCAATA ATTTTCTAAA GCTGTGCTGT CGGTGATACA 120
GAGATGATAC TCAAATTATA ATAAAACTCT TCATTTTGTG AATTATAGAA GCTACTNNNN 180
NTAAAGCCAT ATTTTTTTAG GGAAACTAAG GAGTGACATA GAACTGATGA ATGAGCAAAA 240
GTAAGTTTTG CTGGATTTTT GTAGAACTCT GGACGTTGAG GATTCATTAT GCTGTGGTTA 300
ACTTTAAATA TTTTTN 316

SEQ ID NO:4983
SEQUENCE LENGTH:338
SEQUENCE TYPE:nucleic acid

TOPOLOGY:linear
CLONE:HUMGS05946
SEQUENCE DESCRIPTION:
GATCAGCCCA CTTCAGTCTC CCAAAGTGCT NGGATTACAG ACATGAGCCA CCTACCTGNC 60
CGGAGTTATT TCTAAACACA ACTAATATCA GAATCGCCTA TTTCATAAAA ATTGGATTCA 120
TCAAATNAAT CTTTGGCCAA CAACCATTTG AGAATGATNN NNANCATCAT ACATAGGAAT 180
GCTACGTTTT CTAGGATTTG ACATTTACAG CANTCGAGAN TTACCCTATT TTGTAAATNG 240
AAATAACCAT TACTAAAATC ATGCAAGCTT CAGCTNCTCG GGTTCCTATA GNGTCACCTA 300
AATCGTATGT GNATGGATAC ATAAGGNTTA TGNATCAN 338

SEQ ID NO:4984
SEQUENCE LENGTH:154
SEQUENCE TYPE:nucleic acid
TOPOLOGY:linear
CLONE:HUMGS05947
SEQUENCE DESCRIPTION:
GATCCGCCCG CCTTGGCCTC CCAAGGTGCT GGGATTGTAG GCGTGAGCAC CCCTGGCCTC 60
NAATNTTCAC ATTTATATAC TGAATTATAC ATTACATTAT GTACATATGA TAACTAGTTG 120
CTTAAATATT TTAATAAATT ATATCTAATG TAAA 154

SEQ ID NO:4985
SEQUENCE LENGTH:122
SEQUENCE TYPE:nucleic acid
TOPOLOGY:linear
CLONE:HUMGS05948
SEQUENCE DESCRIPTION:
GATCATGCAG AGAATATATA TTGTACTGTA GTAATTTTNT ATTTACATTT GTATGATGTG 60
ACATAATAGA TGTGAATGTT AATCACTGNT TGACTATGTT AATAAAGTTG TTTAACTATA 120
AA 122

SEQ ID NO:4986
SEQUENCE LENGTH:133
SEQUENCE TYPE:nucleic acid
TOPOLOGY:linear
CLONE:HUMGS05949
SEQUENCE DESCRIPTION:
GATCTCTATT TTAATNNTAA TTCTAGTTAG TTGTGCCTNA ACTCCAAAGA AATGNGAGTA 60
TAGTGAGGTA TGTCTGACTC CCACTTCTTA TCATGCTCTG AACTAGTTNT TTATGTTTCT 120
TTGTGTTNCC TTN 133

SEQ ID NO:4987
SEQUENCE LENGTH:351
SEQUENCE TYPE:nucleic acid
TOPOLOGY:linear
CLONE:HUMGS05951

SEQUENCE DESCRIPTION:
GATCGCACAC ATGCTTTNTT NNNATATGGA GTGAACACAA TTATGTACCA AATTTAACTT 60
GGCAAACTTT CTATTGCCTG TCCCATGTGC ATCTTATTTA AAATTTCCCC CATGGAAATC 120
ACTCTCCTGT TGACTATTTC CAGAGCTCTA GGTGTTTAGG CAGCGTGTGG TGTCTGAGAG 180
GCCATAGCGC CATCATGGGC TGATTTTTNA TTACCAGGTC CCCCAGAAGC AGGTGGGGAG 240
GCTCTGCTTN CTGCTGCCGG TCTNCAAGNC TTGGACCTNT GGACCCTGNT TTGTAAAGNG 300
TAAATTNGTA TCCTTNAGGG AAACCCAAGT GTCAACCTTG TTTNTTCAAT N 351

SEQ ID NO:4988
SEQUENCE LENGTH:310
SEQUENCE TYPE:nucleic acid
TOPOLOGY:linear
CLONE:HUMGS05952
SEQUENCE DESCRIPTION:
GATCGACACA TTCGNATCTN NAATGNGTGC TCTGGGGCCT GTCTGAGTGC CGTGGATGCC 60
CATTCCCAGG TGTGCTCCAT CCTCTGGTCT CCCCATTACA AGGAGCTCAT CTCAGGCCAT 120
GGCTTTGCAC AGAACCAGCT AGTTATTTGG AAGTACCCAA CCATGGCCAA GGTGGCTGAA 180
CTCAAAGGTC ACACATCCCG GGTCCTGAGT CTGACCATNA GCCCAGATNG GGCCACAGTG 240
GCATCCCGNA GCANCAGATT GNGACCNTGA GGCTATGGNC GCTGTTTTGA NGTTGGTACC 300
NTGCGNNGGN 310

SEQ ID NO:4989
SEQUENCE LENGTH:150
SEQUENCE TYPE:nucleic acid
TOPOLOGY:linear
CLONE:HUMGS05953
SEQUENCE DESCRIPTION:
GATCTGAAAC AAAATCTTTC TAAAACATTG TTTTAGTTGT CAAAGCACCA ACAGGACATT 60
TTGGGATGTG AAATGTAATT TCTTGGAATC TGTAATTTGT ACTTAATATT TNAGGCTTGT 120
ATTTAATATA ATAAATAGGT GTTTGTTAAA 150

SEQ ID NO:4990
SEQUENCE LENGTH:240
SEQUENCE TYPE:nucleic acid
TOPOLOGY:linear
CLONE:HUMGS05954
SEQUENCE DESCRIPTION:
GATCATCCCT TCACAATCCC AGAGTGGCAG GCGGGACCAG CCCCATGGTC TGGCTCCTGT 60
CACCTGGGTC CGTGCCAGCA CAATCTGCCA AAGTTCTAGA GACCCTGTTC CCTTCCCCAT 120
NAGGTCACAT GCTTCTTCTG TGTGTATTTC TTTTTGTTTT TATGGTTTTT GGAGCAATTT 180
AAACTCCCAG TTGNNTATTT TCACAAAAGA AANTAAAATT GCAGTTGCAA GCCCTTTAAA 240

SEQ ID NO:4991
SEQUENCE LENGTH:323
SEQUENCE TYPE:nucleic acid

TOPOLOGY:linear
CLONE:HUMGS05955
SEQUENCE DESCRIPTION:
GATCCTTTTT TGTACATTTA AGAATATTTT GATTATATTA AACAAGACTG CTGATTTTGC 60
TACTTTTTTT AAGGGGTCTT CANGTANGTA AAACATACAT CGTAGCTAGA AGAAAAATGT 120
ACCTTAAATT TGCATCTTCC CTCTCATACC CAAGCTGTAA ACAATTGAAA TATTTTGTCT 180
TAAATCACTT GGTTCAATAC ATGCTTATTT GTTTTAAAAC CTGTATCATC ANACTCTCTC 240
TCNAAATTTA AAATGCTGTT GAATATGATA CTTTTGAGGA GAGAGTGTGC TCAGAACTTA 300
GACGGGGTTT GGTAGGCCAA NGN 323

SEQ ID NO:4992
SEQUENCE LENGTH:110
SEQUENCE TYPE:nucleic acid
TOPOLOGY:linear
CLONE:HUMGS05956
SEQUENCE DESCRIPTION:
GATCTTAACC ATCACAAGTT TTTACCCTCT TCCTTCATGC CTGACCTCAA CCCCGCTCTC 60
CTCATCCTAT TCCTAAATTA GGCTAATAAA GTGAAATTGG TATACTTAAA 110

SEQ ID NO:4993
SEQUENCE LENGTH:228
SEQUENCE TYPE:nucleic acid
TOPOLOGY:linear
CLONE:HUMGS05957
SEQUENCE DESCRIPTION:
GATCGCTTGA GCCCAAGAGT TTGGGACCAA TCTGAGCAAT ATAGCAAGAC CCCATCTCTA 60
CAAAGAATAA AGAAAAATTA GACTAGCTTG GTGGCATGTG CCTGTGGTCC CAGCTACTCA 120
AGAGGCTGAG GTAGGAGGAT TGCTTGAGCC TGGGAGGTGG AGGCATCAGT GAGCCATGAC 180
CGTACCACTT CATTCCAGCC TAGGTAACAG AATAAGACCC TGTCTAAA 228

SEQ ID NO:4994
SEQUENCE LENGTH:182
SEQUENCE TYPE:nucleic acid
TOPOLOGY:linear
CLONE:HUMGS05959
SEQUENCE DESCRIPTION:
GATCAAGCTA CACATCAGTT TTACAATATA AATACTTGTA CTACCTTAAT GATAAGNACT 60
CCTTAAAGTT CCATTTGCTA ATNATTAATA CACTGTTTGG GCTGGCCAGT TTTTCATGCA 120
TGCAGCTTGA CGATTGAGCA CAGTCAGGCC TTTGTATTAA AAATGAAANA TGAAANAACA 180
AA 182

SEQ ID NO:4995
SEQUENCE LENGTH:300
SEQUENCE TYPE:nucleic acid
TOPOLOGY:linear

CLONE:HUMGS05960
SEQUENCE DESCRIPTION:
GATCAACTGG TTTGTGTTTT GCTGCTGCAT TTATCCCAAG TNAAACAGGT TTAATCTCCA 60
GANGAAAACC AAAATACCAT GGGATTTATG CTGNATTGAC ATCTTGCCCT AAACGNACAA 120
CATCATAGTA ATTTGTCATG GGCAACATGA CCAGNGAGAA GATTTTTGTC ATGATTTTAA 180
ATACACTGNC ACGCTACTGT TGGTTAAATT TAAACANGTT TTACCTGCAG NAATTCTCTC 240
ACAAATAACC NGCAATAACT TGAAATGCAT NCCCTTTTGA ACACTTCCTT TNCTCATGGN 300

SEQ ID NO:4996
SEQUENCE LENGTH:162
SEQUENCE TYPE:nucleic acid
TOPOLOGY:linear
CLONE:HUMGS05961
SEQUENCE DESCRIPTION:
GATCCTGCTC CCTTCTGGGC TCTGACTCTC CTGGAAATCT CTCCAAGGCC AGAGCTATGC 60
TTTAGGTCTC AATTTTGGAA TTTCAAACAC CAGCAAAAAA TTGGAAATCG AGATAGGTTG 120
CTGACTTTNA TTTTGTCAAA TAAAGATATT AAAAAAGGCA AA 162

SEQ ID NO:4997
SEQUENCE LENGTH:136
SEQUENCE TYPE:nucleic acid
TOPOLOGY:linear
CLONE:HUMGS05962
SEQUENCE DESCRIPTION:
GATCTCAACA ATATTCTTCC AAAATGGCAT ACATCTTTTG TACAAAGAAC TTGAAATGTA 60
AATACTGTGT TTGTGCTGTA AGAGTTGTGT ATTTCAAAAA CTNAAATCTC ATAAAAAGTT 120
AAATTTTTGT CTGAAA 136

SEQ ID NO:4998
SEQUENCE LENGTH:53
SEQUENCE TYPE:nucleic acid
TOPOLOGY:linear
CLONE:HUMGS05963
SEQUENCE DESCRIPTION:
GATCTGACCT CAGAATATGC TTTCAGTGTT ATTTCTGACT AGTCCTTTTT AAA 53

SEQ ID NO:4999
SEQUENCE LENGTH:334
SEQUENCE TYPE:nucleic acid
TOPOLOGY:linear
CLONE:HUMGS05964
SEQUENCE DESCRIPTION:
GATCTGCATA AAGTNTTACA AACAAAATCC TCTTNATGAG CAACACATTT TTCAGCTGCC 60
AGTCAGACCA ACTGCTGTAA AGAACTTATA TCAAAGTGAG AAGCCACAGA AATGGAGAGT 120
GGAAATATAT AGTGGTCAAA AGANGATTAA GACAGTTTGG CAACTGAGTG ACAGCTCACC 180

CATAGACCAT CTGANTTTNC ACAAACCTGA TTTTTCGGAA TTATCACTAN ACGGTAGCCT 240
NGAAGAAAGG NTATNCTTTN CTANCATGGT TACCTGCAGC CAGGTGCATT TGCAAGTGAN 300
GTGTGCTGGT TGANGTCCTC TTNTAAGGGN ATTN 334

SEQ ID NO:5000
SEQUENCE LENGTH:117
SEQUENCE TYPE:nucleic acid
TOPOLOGY:linear
CLONE:HUMGS05966
SEQUENCE DESCRIPTION:
GATCTTCCAC TAGATGTCAC CAAAAGGACT GAGAACTAGA GAAAAGGGCT GCGATTTCTG 60
CTCTCCTTGT AAGATTGCAC AAAAGANTAA ATTGCATTTA TCGCTGTTTG CATGAAA 117

SEQ ID NO:5001
SEQUENCE LENGTH:290
SEQUENCE TYPE:nucleic acid
TOPOLOGY:linear
CLONE:HUMGS05967
SEQUENCE DESCRIPTION:
GATCCCCCTT CNAGGTAGTG AAAATNAAAT NCGCCGGAGT GTGGCTGCCT TCACCGCGGA 60
CCCTCTGTCT CTCCTGCGCA ACGTCTGAGC ACAGGAGCCC ATCCTTGGCT CTAGGNTTCC 120
NCCGCTGGAA GCCTTCTGTT CANACACCCC TTATGNTCCA AGGCCTGATG TGAGCCAGCG 180
GGGGGTGCAT GGGAAACTGN ACCCNACAAC CCACATCCTN CATCCTGACT GCAGCANTGG 240
GGTTCCCCGG CAGGGNTGGG TGAGGNAGGA AGGGGGTCAA GCCTGGNGTN 290

SEQ ID NO:5002
SEQUENCE LENGTH:284
SEQUENCE TYPE:nucleic acid
TOPOLOGY:linear
CLONE:HUMGS05968
SEQUENCE DESCRIPTION:
GATCACCGTG AATCCGGCTT CCTCTGAGCA TTCGATGGCC TTAGCACCTC ATCAAGCCAG 60
CACATCCTGC CTGCTGTTGC AGCCTGGCTG GGTTTATTCT TCAGTTACCC TAATCCCATG 120
ATGCCTGGAA CCTTGATTAC CGTTTTACAT CAGCTCTTGT ACTTTTNAGT ATATTTTCAT 180
AATGAGTTAT ATTGTCATTT AGACTTTGAA CAGCTCTGGG AAATAGAAGA CTAGGGTTGT 240
TTCTTAAAGT TTAGCTCATG TTATAATAAA AAGTTGNAAT GAAA 284

SEQ ID NO:5003
SEQUENCE LENGTH:346
SEQUENCE TYPE:nucleic acid
TOPOLOGY:linear
CLONE:HUMGS05969
SEQUENCE DESCRIPTION:
GATCGTCCCT GGTCACCCGA CTTTGGAATT TGCACCATCA TGTTTCAGTG AAGATGCTGT 60
AAATAGGTTC AGATTTTACT GTCTATGGAT TTGGGGTGTT ACAGTAGCCT TATTCACCTT 120

TTTAATAAAA ATACACATGA NGACAAGAAA GAAATGGCTT TTCTTACCCA GATTGTGTAC 180
ATAGNGCAAT GTTGGTTTTT TATAAAGTCT AAGCAAGATG GTTTTGTATA AANTCTGAAT 240
TTTGCAATGT ATTTAGCTAC AGCTTGTTTA NCGGCAGTGT CATTNCCCTT TGCACTGNAA 300
NGAGGAAAAA NNNGGNATTA AAAGGGTTGC CAANTTGGCA AAAGGN 346

SEQ ID NO:5004
SEQUENCE LENGTH:337
SEQUENCE TYPE:nucleic acid
TOPOLOGY:linear
CLONE:HUMGS05970
SEQUENCE DESCRIPTION:
GATCTGCTCG CCTCGGCCTC CCAAAGGATG GGATTACAGG CACCAGCCAC TGNGCCTGGC 60
TGGCCTCTGN TTTTTAATAA AACNTGACTA GAGTGACTCC ATCTTAAAGT GAGTAGCTAG 120
GCACTTACAA GGTTCATGCT TATGGCCTGA AAATAACCAC ATCCCAGGCT GACCACCAAT 180
TATAATTACA GANTATTTAT GGCCATACAG NNCATGNTCC ACCAAGCCTG CAGAATGTCC 240
AAATNTCCTA AGAATGCAGC CNCCATTACT TANATATANC ATAAATGAGC ANGCTTAGGT 300
TGCAGGNTTA ATGGTCGTGG ATANCAGCAT TNGNCCN 337

SEQ ID NO:5005
SEQUENCE LENGTH:211
SEQUENCE TYPE:nucleic acid
TOPOLOGY:linear
CLONE:HUMGS05974
SEQUENCE DESCRIPTION:
GATCAGGGTT ACATAGGTTT GGTAAAATNA GTGCTGGAAA TNAACTTTCT CCCAGTAGTC 60
TTAGGTCATG CTCAGTGAAC TTAAACTTTA TCCAGATATG GTTTTCCTTC AGCCTTTCTA 120
TTCCCTTTCT AGCCAGTGAA AGACCCGCTG CCCTTTGACC TCAGCCCCTC CAAGCCCCCA 180
AGTTTAAAAC GCCACCCNCT GCCACCAGAA A 211

SEQ ID NO:5006
SEQUENCE LENGTH:103
SEQUENCE TYPE:nucleic acid
TOPOLOGY:linear
CLONE:HUMGS05975
SEQUENCE DESCRIPTION:
GATCCTCCTG CCTTGGCCTC CCAGGGTGCT GGNATTGTAG GTGTAAGCCA CCAGGCCCAG 60
GCTAACCCTT TATAAGAAAT AAAGTCTCCT CTCAAAATTT AAA 103

SEQ ID NO:5007
SEQUENCE LENGTH:265
SEQUENCE TYPE:nucleic acid
TOPOLOGY:linear
CLONE:HUMGS05976
SEQUENCE DESCRIPTION:
GATCCGTCTG GGCACGCTGG AGCAGGAGGC CACAGCAGAC GTGGAGTGGC GCTGGCACCC 60

TTACACCAAT ACCGCACGCA AGAGAGTCTT CCTGAGCACC GAGTGAGCAC ACTCACCACT 120
CAGTCAGGAC ATGGACTTGG AACTCAGGAT TGGGCTGTCA TAGACAGACC CACCAGTAGG 180
AACTGTCACA GAATGGCCTG CTGAACTGGG ATGTGGAACT GTGGCGGGTG GAGAGGTCTG 240
AATAAACCGT CTGTGTCATG GCAAA 265

SEQ ID NO:5008
SEQUENCE LENGTH:308
SEQUENCE TYPE:nucleic acid
TOPOLOGY:linear
CLONE:HUMGS05978
SEQUENCE DESCRIPTION:
GATCATCAGC AATNCCTTCT GCACCANCAA CTGCTTAGCA CCCCTGGCCA AGGTCATCCA 60
TGACAACTTT GGTATCGTGG AAGGACTCAT GACCACAGTC CATGCCATCA CTNCCACCCA 120
GAAGACTGTG GATNGCCCCT CCGGGAAACT GTGGCGTGAT GGCCGCGGGG CTCTCCAGAA 180
CATCATNCCT GCCTCTACTG GCGCTGCCAA GGCTGTGGGC AAGGTCATCC CTGAGCTGAA 240
CGGGAAGCTC ACTGGCATGG NCTTTCGTGT NCCCACTGGC AANGTGNTCA GTGGTGGTNC 300
NGANCGTN 308

SEQ ID NO:5009
SEQUENCE LENGTH:336
SEQUENCE TYPE:nucleic acid
TOPOLOGY:linear
CLONE:HUMGS05980
SEQUENCE DESCRIPTION:
GATCCGATTT GTGGAGGATG AGAAAGATGA AGAAGACAAT NAGACGAGAA CACTTCTAAG 60
GCGAGCCACT CCACACCCAG GGGAGTTAAA GCACATGAAA AAGACAGTGG AGAATTTACG 120
GAATGACATG AATGCTGCTA AAGGACTGGA CTCAAACAAA AACGAGGTCA ATCATGCCAT 180
TGACCGAGTG ACCACTTCTG TGTAGAGTTT CACCTCCAAG TTTTACCTCC TGTGTCTTCC 240
TCTGCTGTCG AGACGTTCCT GTCTGCTTNC CGGGAGCCTC ACGTGCTCCT TGTCCTAACC 300
AGCCCCCGNG NGCCATCTTT CCCGTGGAGT GTTGTN 336

SEQ ID NO:5010
SEQUENCE LENGTH:327
SEQUENCE TYPE:nucleic acid
TOPOLOGY:linear
CLONE:HUMGS05981
SEQUENCE DESCRIPTION:
GATCGCTTTT NTGACTGTTC ATCTGTCCTT GACAGTGGCT GTCATATTGA CTACTTTNTT 60
GATTTGTTGG TATTGGGGAC ATTTTAAAGG CTGAGTTATT TTTGAATGTC ATGTTTATGT 120
CATAGACGTA GTTTTCGCAT CCTTGAATTA AACTGCCTTA ACTCCTTTNG TGGTATAAGC 180
AAAACTCCAT GGNCNNNGTC CTGGTATCCT TTTCCTGTGT GGTTGCCCTG TGTCCTCTGG 240
CCTAGGGTTA AGTNTGCAAG ATAACTACTC GTGAGTATTC AGAATGTNGT NCCTAATAAA 300
TGCACTTGTN GTCTGTCTTC TTTTTNN 327

SEQ ID NO:5011

SEQUENCE LENGTH:326
SEQUENCE TYPE:nucleic acid
TOPOLOGY:linear
CLONE:HUMGS05982
SEQUENCE DESCRIPTION:
GATCAAGTTT TAAATCCTCC TCCTTCCCTT TTTNCTGGAG TNTTGAGGGC CAGAGTTTTN 60
NTTTTTNTTT TTGTTTTTGT TTTCCTGCTT GCTACTGTTT TGTGGTGTTG AAAAGTGGTT 120
TAAACCTGAG ACTAACTTAA ACACTTCCTT GACCTTCTNG TTGCCTGTNC ATTTTTGTGC 180
CAAGGAAGTA GCTGCCCCAG TGTATGTCTT GCCTTCTCCG CGTCATTGTT GGAAGAGGAG 240
AGATGCATCG AGCAGTCCCA GCTGCTTTTC ATTTATAACG GCTTCTTTCC AGGACCTGAC 300
AGAAGTCAGG GAAGAGTCCC TGGGNN 326

SEQ ID NO:5012
SEQUENCE LENGTH:326
SEQUENCE TYPE:nucleic acid
TOPOLOGY:linear
CLONE:HUMGS05983
SEQUENCE DESCRIPTION:
GATCATTTGA CTGGGCTGGA GAACATCCTT TTACATGGCC NTCCCATGGA TGTGCTGTAC 60
ATCTGCTTAA AAGAAAATAA TTACTTTNAT GAGCGTCTTC AAAAGGACTC TTGGTGCAAC 120
AGACTNAATT GGAACTCAGC TTTTCTAACT GTCACTGCAC CAAGCTCTGC TGGAGGAGTG 180
ACCAGACTCA CGATTTGGTA TAGTGGGGCT CTCAAGCATC TTCAATTTGA ATGTACATGC 240
TGCTGAGGAG CCGGTGAAGT CATCAGTTCC GCACATCCCT TCTACCCTNC AACTGCATGG 300
GAAGCCAAAG TNCTGGTTTT GAAGAN 326

SEQ ID NO:5013
SEQUENCE LENGTH:327
SEQUENCE TYPE:nucleic acid
TOPOLOGY:linear
CLONE:HUMGS05984
SEQUENCE DESCRIPTION:
GATCTGGGGG CTAGCAGTGA GTACCCGCAT GGTATCAGCC TGCCTCTCCC GCCCACGCCC 60
TGCTGTCTCC AGGCCTATAG ACGTTTCTNT CCAAGGCCCT ATCCCCCAAT GTTGTCAGCA 120
GATGCCTGGA CAGCACAGCC ACCCATCTCC CATTCACATG GCCCACCTCC TGCTTCCCAG 180
AGGACTGGCC CTACGTGCTC TCTCTCGTCC TACCTATCAA TGCCCAGCAT GGCAGAACCT 240
GCAGCCCTTG GCCACTGNAG ATGGAAACCT NTCAGTGTCT TGGNATGACC CTACCNAGGT 300
GGTGGGTCTC CAACANAGCC ACTTGTN 327

SEQ ID NO:5014
SEQUENCE LENGTH:293
SEQUENCE TYPE:nucleic acid
TOPOLOGY:linear
CLONE:HUMGS05987
SEQUENCE DESCRIPTION:
GATCTCAAAA AAGAAGGAAT TTCTCCTTTG TTTCTTGCAG TTAATGTAAG AATACTTTAA 60

ATCTCTAAGC TTCTGAAGTG TTAGAGGTAG AGATGGTCTA GTAAAGATGT AGTAGTAATG 120
TTTTATCCAT TTAGCATGTG TTTATNTNTC CATATGTNCT CAAAGGTGAC TTATTGGTTC 180
ACCTCAGTGA TATTACAGCT AAANNNNTCA TTCATTAGCA AAAGGNAAAG TGGTCTCAAC 240
CTAACATCAG ANGTGTTTCT NATTATGNTN TNATATTGCG TTGGANTATT GGN 293

SEQ ID NO:5015
SEQUENCE LENGTH:71
SEQUENCE TYPE:nucleic acid
TOPOLOGY:linear
CLONE:HUMGS05988
SEQUENCE DESCRIPTION:
GATCTATACA GGTATGTCTG ACGGGACGCA GCACCGTGGG CACGCACCAA ATAGAGTTTT 60
TAAAAGAGAA A 71

SEQ ID NO:5016
SEQUENCE LENGTH:354
SEQUENCE TYPE:nucleic acid
TOPOLOGY:linear
CLONE:HUMGS05989
SEQUENCE DESCRIPTION:
GATCCTNTTC TTTCAGCAGG TGAAAAATAA AACGCAGTTC AAATTTCATG GTTTTAATTT 60
TCANCTCAGA AGCACTCAAA AATGCAAAAT GTGATAATGG GCACTTGTTT AAAAGANTTA 120
GTGTATCCAG CCTTCACTCC AGCTGGTTAA AAATGTTGCA CTTNTCAGCA ACCCTACCAC 180
TTTCATCTGC TGAAAGGNCA NATGTGCTTG GTTTTACNAT TATGTAATCA CAACTTACTT 240
TCTGCTTGTN GTNGCTTAAA ATTATGTATT TGGTCTTTGG GCTGCAANTT GGTTTTATGC 300
TGGATTTGAT GNTNACTGGC NGNNAGTTTG ACCTTTGCTG NTATGGGGAA ANTN 354

SEQ ID NO:5017
SEQUENCE LENGTH:149
SEQUENCE TYPE:nucleic acid
TOPOLOGY:linear
CLONE:HUMGS05991
SEQUENCE DESCRIPTION:
GATCGGAAGA GGGAGGAGAG GCAGTCTCTT NACAAAATAA AGTATTTTAA TTCATTTNTA 60
TTTATTAAAT NAAAAAACAA TCCCATGGTG TCCCTNTTGT TTGGTGGAAC CTAATGACTG 120
TNGAAATAAA GTCCTGTNTT TNCCCTAAA 149

SEQ ID NO:5018
SEQUENCE LENGTH:259
SEQUENCE TYPE:nucleic acid
TOPOLOGY:linear
CLONE:HUMGS05992
SEQUENCE DESCRIPTION:
GATCCAAAAA CCTCCACCAT AGAGACTGAT AAATTGTAGC TATTGAAGCT GTACTNCTTT 60
GTACATAGGT GCTTTGGGAG ACTCACTCTG ATTATTGTAA ATTTNCTCAC TAGCTACCCA 120

ACATTAAATT CAAATAACAG AGAATTTCTA TTGTACAGCC TTGCAACTTT NCTTTAAGTT 180
TAANCTTTNC TNNAAAATAA AATTTTAAGT GGCAAAAATN ATTATTAGAN GAGAAAANGT 240
GTGCTCATTT TTNANTATN 259

SEQ ID NO:5019
SEQUENCE LENGTH:173
SEQUENCE TYPE:nucleic acid
TOPOLOGY:linear
CLONE:HUMGS05993
SEQUENCE DESCRIPTION:
GATCACCTGA GGGGAAAGGN TTGTNTCTCT CCTTTCTGTT GGGNGAGGGG GATGGGGGAC 60
TTTTNTTGGT GGCTCCCACC CATATATCCC TCCTTNACCA TAGTACTCCC ACCCACTTCC 120
ATCACCCATC CAATAAAATG CAGCCAGGTT TAGCCTTTGG CTTTGGTCAC AAA 173

SEQ ID NO:5020
SEQUENCE LENGTH:266
SEQUENCE TYPE:nucleic acid
TOPOLOGY:linear
CLONE:HUMGS05994
SEQUENCE DESCRIPTION:
GATCTACCCT CTAGTTTACT TGCTCGGGAG AAGAAACTGA CTCGTTTTAT TTAGTGCCTA 60
TTTAGCGAGC CCAGAGTAAC GTACATTTGT NCTGTTTTCA ATTTTGTGCT ATCGCAAATC 120
ACAAAAAAAC TGTTATCAAT TCACATTCAT CATCAGCACG AGACCCATTT CCTTGTGCTC 180
CTGCCAGCTC AGGNTATNAT GTTTCTTTTT TCATGTTTGC CAGTCTGTTA GGTAAAANGT 240
CATATGCGGT TGTTTTAATT TGCAAA 266

SEQ ID NO:5021
SEQUENCE LENGTH:121
SEQUENCE TYPE:nucleic acid
TOPOLOGY:linear
CLONE:HUMGS05996
SEQUENCE DESCRIPTION:
GATCAAGGTG ATTCTGAAAG TTTTAATTTT TAATGTTGTA ATGTTATGTT ATTGTTAATT 60
GTACTTTATT ATGTATTCAA TAGAAAATCA TGATTTATTA ATAAAAGCTT AATTTCTCAA 120
A 121

SEQ ID NO:5022
SEQUENCE LENGTH:58
SEQUENCE TYPE:nucleic acid
TOPOLOGY:linear
CLONE:HUMGS05997
SEQUENCE DESCRIPTION:
GATCTAGTTG TATTTCTCAT ACTAGCAATG AACAATTGGC AGTTGAAGTA AAAGCAAA 58

SEQ ID NO:5023

SEQUENCE LENGTH:238
SEQUENCE TYPE:nucleic acid
TOPOLOGY:linear
CLONE:HUMGS05998
SEQUENCE DESCRIPTION:
GATCGTAATA TCTCGAACAT TACATAGACA CTTAAAACCT TTAGTTGTAT TTCATCAAAA 60
ATCTGTTCAT GCCCCACGTT GGTTTCAAAA CATACTATGC TTTTNCTTCG TGTTATTTCC 120
TATATNCATT TTNGTGTGTA TGTGTATGTC ACAAATATTG ATATGCCTGG TTGTTTATTT 180
TGGTNTGCTA TTATGCCTTT TTCAAAATAT AAAANTAAAC TNGTAANTNC TAACNAAA 238

SEQ ID NO:5024
SEQUENCE LENGTH:198
SEQUENCE TYPE:nucleic acid
TOPOLOGY:linear
CLONE:HUMGS05999
SEQUENCE DESCRIPTION:
GATCGAGACC AGCCTGACCA ACATGGTGAA CCCTGTCTCT ACTAAAATAC AAAAATTAGC 60
TGGGCGTGTT GGCACACACC TGTAATCCCA GCTACTCAGG AGGCTGAGGC AGGAGAATTA 120
CTTTAACCTG CGGGGGGAGC CTAGATTGCG CTACTGCACT CCAGCCTAGG CAACAGAGGG 180
AGACTCTGTC TCATTAAA 198

SEQ ID NO:5025
SEQUENCE LENGTH:301
SEQUENCE TYPE:nucleic acid
TOPOLOGY:linear
CLONE:HUMGS06000
SEQUENCE DESCRIPTION:
GATCTTGTGT CAAGTTAACA GGAAGACTGC CCACAGATGA CACTGCATTT GTATCCCTGT 60
GGATGTAGAG TATTGGGAAG GCATTTNTTT AGTTTCATGC CTCCAAACCA TGCATTGTCT 120
GTNATGTATC TGCCCAGCCT TCTCAGAACT CAATGTTGTA CATTTTTCCC TCTAGGACCT 180
GAATTGGTCT TGGAGGCAGC TTTTAGAAAT CCTCTATGTA GTTCCTGTCC ACATGCACTA 240
TATATNGGTT TCTCCNNGNN CCTCTCATGA ATCTGCTCGT TTCTCTACNT CTTGGCTACG 300
N 301

SEQ ID NO:5026
SEQUENCE LENGTH:301
SEQUENCE TYPE:nucleic acid
TOPOLOGY:linear
CLONE:HUMGS06001
SEQUENCE DESCRIPTION:
GATCCTGGTA ACTGTTCCAG GATTGCTCCA GGTTTGAGAT GGTATTGCTA AATTTAAAAT 60
TAAACAAGAG ACCCAACAAC AGCTTTTAAA GTGTCTTCTA TNGCATTGTA TTTNNNTNAA 120
CTTGCCCCAA TGATAGAAAA GTCTTTTGCT GAAATNATTT TNATGATTTT GGTTTATCGT 180
TTATAAAANG GAAAAGANAT ATACAAACTT TGACTTTGGT GACTTTGTGA AGGTTTCTTT 240
AAGATGTGCA TTCCTTTCTG CTTGCNCTCT AGTAAAGGNT TTACTACCGG GAAAAAAAAG 300

N 301

SEQ ID NO:5027
SEQUENCE LENGTH:117
SEQUENCE TYPE:nucleic acid
TOPOLOGY:linear
CLONE:HUMGS06004
SEQUENCE DESCRIPTION:
GATCANGAAA AAGACAAAAT CTNTCAAAGC CTTCAAAACC AGGAAAAGAT AAGGAGCCCA 60
GGAAAAGNAA AAGGTAAAGT TCGAAGAANT TTAACCATTT TTNACTAGCG ACAGCAN 117

SEQ ID NO:5028
SEQUENCE LENGTH:231
SEQUENCE TYPE:nucleic acid
TOPOLOGY:linear
CLONE:HUMGS06005
SEQUENCE DESCRIPTION:
GATCAGGAAT GGTGTGGATT GAGAACTTGT TACTTGAAGA AAAAGAATTT TGATATTGGA 60
ATAGCCTGCT AAGCGGTACA TGTGGGTATT TTGGNGTTAC TTAAAAANNA TTTTNGGGAT 120
AAGAGAATTT CAGCAAAGAT GTTNTAAATA TATATAGTAA GTATAATGNA TANTANGTAC 180
AATGGAAAAN ACAATTATAT TGTAAAATTA TANCTGGGCA AGCATGGNTG N 231

SEQ ID NO:5029
SEQUENCE LENGTH:194
SEQUENCE TYPE:nucleic acid
TOPOLOGY:linear
CLONE:HUMGS06008
SEQUENCE DESCRIPTION:
GATCATGGAA CAGAATCACC CTCTCCTGCA TGTTTTTNTT TCTGTCTCCT GCTTTNCTGT 60
TCTTTTTCCA CTTTCTCTAT GTGTGAGTTG ACTTGGCTGC CTGTAGCTTC ATCGTCAAAG 120
CTGGTCCACG TGGGNTCAAC TTGGTGCTCT CACTCTCCTC CAGCATTGTT TTTGGCATCA 180
AAGCTAGAAT TAAA 194

SEQ ID NO:5030
SEQUENCE LENGTH:89
SEQUENCE TYPE:nucleic acid
TOPOLOGY:linear
CLONE:HUMGS06010
SEQUENCE DESCRIPTION:
GATCNTTTTT AAGAGCGTTT CCATACTATG NTTACATTTC CATATTTNTA TGAATAATTT 60
TGCATTCAAT AAACAACCAG ACTCAGAAA 89

SEQ ID NO:5031
SEQUENCE LENGTH:174
SEQUENCE TYPE:nucleic acid

TOPOLOGY:linear
CLONE:HUMGS06011
SEQUENCE DESCRIPTION:
GATCTTGGAG AGTTTCTCCT TGTGATTTTA GTTCATAAGT ATGTCACCTT TCATTTTATA 60
GNGTTCATCA TTGAGTAATG GATTAAGTGA AAATCCAGGA GTATCCATCT NCAGTTATGT 120
NCTGAGGTGA TAATNCATCC AACATATTTN TNAGCATAAA TATNANGCTT CAGN 174

SEQ ID NO:5032
SEQUENCE LENGTH:260
SEQUENCE TYPE:nucleic acid
TOPOLOGY:linear
CLONE:HUMGS06013
SEQUENCE DESCRIPTION:
GATCTAAAGC AGCCCTTTTT ACAGTCTAGT TAGGAGAGAG AAAATAATTG CAAATATCCA 60
CTTAGAGGCA AAGAACAATT TTTNATTATC AAAAAGGTTT CTGCACATTG TTGTGGCAAT 120
ATTGTATCTG TTTAGAAAAT GGGCTTTTCC AAAAGCAAAC AAAGATAGGT TCCTCAGGTG 180
ACCAAAACTG AAAATCAATA TTTCCATGTT TCATTAATCA NGGCATAAAA TACAATTAAN 240
GCAAAATATT TTACATTAAA 260

SEQ ID NO:5033
SEQUENCE LENGTH:85
SEQUENCE TYPE:nucleic acid
TOPOLOGY:linear
CLONE:HUMGS06014
SEQUENCE DESCRIPTION:
GATCTCTGGA GGTCGAGGCT GCAAGTGAGT CATGTTCGTG TCACTACACT NCAGCCTGGG 60
CGATAGAGTG AGAACCTGTC TCAAA 85

SEQ ID NO:5034
SEQUENCE LENGTH:352
SEQUENCE TYPE:nucleic acid
TOPOLOGY:linear
CLONE:HUMGS06015
SEQUENCE DESCRIPTION:
GATCCAGAAC ATGACAACAG AGAGCTGCGT CCACAGGGAA CAAAGCCCTG ACCTNTCTCT 60
CCACATTACC CTTACAAAAA CAGGCCCTCC CCATGAGAGA GCTACACGGC AGGGGCAGAC 120
ACTGTGAGTA TAAGCTACTT TCCTCCCTGG AGTGCTCTAT GTGGGCAGAA CATGCTCTCC 180
TTGCCTCTCC TGGAAGGTGT CTTCTCTATG GCCTGGCTAG AGCTGNAAAA AAGGGACACA 240
CCCNACTTCG GTAAAAGAAA NTAGGGAAAG GCCATATACA NNGNCAGACT TTGTAGTTTA 300
TTTTGTATTT TTTTNTAAAT NNTTTNCACT TTTACATTTA ANGGNAGATT GN 352

SEQ ID NO:5035
SEQUENCE LENGTH:206
SEQUENCE TYPE:nucleic acid
TOPOLOGY:linear

CLONE:HUMGS06016
SEQUENCE DESCRIPTION:
GATCTTAATG GCAAGAAGGC CACAGAGGTA CTTTNCCTTN TTNAGCTCAG GAAAATATGT 60
CAGGCTCAAA CCACTTCTCA GGCAGTTTAA TGGACACTAA GTCCATTGTT ACATGAAAGT 120
NATAGATAGC AACAAGTTTT GGAGAAGAGA GAGGGNGATA AAAGGGGGAT ACAANNGATG 180
TACAGAAATN NTTTCCTGGC TGGNCN 206

SEQ ID NO:5036
SEQUENCE LENGTH:351
SEQUENCE TYPE:nucleic acid
TOPOLOGY:linear
CLONE:HUMGS06017
SEQUENCE DESCRIPTION:
GATCTGGCCA TGTATATGGT AGCTGTGTTT TAATTTGAGA ATCTTGAGGG TAGAGCCACA 60
AATTTCAATT CTNACATTTC CATTTGCAAA GTGACTAGAG AAAAAGAAAT CAGCTTAAAT 120
GAGGTATTAA GTAATGTTTA GAGTCGTAGG TATTAACTAG AATATAAATC CTTAGAAATT 180
GTCTTTATAC CTTCAAAAAT TATNCTATGC ATTTNTCATA GANCTGTGAT TACAANGNNG 240
TCTGACTACC ATGTCTTTAA ACATATGGCA TCTCTCATCT TTCCTTCCCT TATGGGGGCT 300
ACATTTNGTN CCATTTTCCA GNAGTAGNCT TTAACTTNCC GGTGCCNNNC N 351

SEQ ID NO:5037
SEQUENCE LENGTH:131
SEQUENCE TYPE:nucleic acid
TOPOLOGY:linear
CLONE:HUMGS06018
SEQUENCE DESCRIPTION:
GATCCTGAGA CTATNTGCAT GCTNCTNTCT AAATGATAAT TAANAGGAAA TTTCATGGAT 60
TAACCCATGG GTTTAATGCA GCAAGGAAAC TTACAATGTC CCTNTATATA TAACATGCAN 120
CTTGTTTTGG N 131

SEQ ID NO:5038
SEQUENCE LENGTH:208
SEQUENCE TYPE:nucleic acid
TOPOLOGY:linear
CLONE:HUMGS06020
SEQUENCE DESCRIPTION:
GATCCTNCAG GAGATTCAGC GTCTCCGCCT GGAACACGAG CAGGCCTCCC AGCCCACCCN 60
TGAGAAGGCA CAGCAGAACC CCACGCTGCT GGCAGAGCTG CGGCTGCTGA GGCAAAGGAA 120
GNATGAACTG GAGCAGAGGA TGTCGGCCCT GCAGNAGAGC AGGCGGGAGC TGATGNGTCC 180
ANNTGGAAGA GCTGATGAAG TTGNNCNN 208

SEQ ID NO:5039
SEQUENCE LENGTH:150
SEQUENCE TYPE:nucleic acid
TOPOLOGY:linear

CLONE:HUMGS06021
SEQUENCE DESCRIPTION:
GATCTGAGTG ACAGGGTCAA GTTCTCTTTG AAAACAGGAG CTTTTCAGGT GGTAACTCCC 60
CAACCTGACA TTGGTACTGT GCAATAAAGA CACCCCCTAC CCTCACCCAC GGCTGGCTGC 120
TTCAGCCTTG GGCATCTTCA TAAATGGAAA 150

SEQ ID NO:5040
SEQUENCE LENGTH:365
SEQUENCE TYPE:nucleic acid
TOPOLOGY:linear
CLONE:HUMGS06022
SEQUENCE DESCRIPTION:
GATCTGCTGG GTTTTAAGGA GTGATTTNCT TTCTNCCCCT TGAAGGGGAA AAAGCTATTT 60
CAATTGGTAC ATTTAAAGTC CCCCAACTAT GGGGAGGTAC CAATTCTGGA CANNNTGCCA 120
CTACAACAAC ACTAAACCTG AACTTTTCAA CTCCGTTGGT GGTGGGAGGC AGCGGGCAGA 180
AATTTACTGT TGGCCACTGN CAGGTCTATT TNATATTTCA AAGGAATATT GGGTGCTGCA 240
TATAGGNACT GAAGGGGTCA ATGNATTAAA CCTGTGATTA GGTTGNTTTC CTGTCATTTT 300
TGAGAGACTA AAATNGTGGG GGGGCAGATG TNCANAATAC CTGGTACANG TTTTTAAAAA 360
NTGGN 365

SEQ ID NO:5041
SEQUENCE LENGTH:147
SEQUENCE TYPE:nucleic acid
TOPOLOGY:linear
CLONE:HUMGS06023
SEQUENCE DESCRIPTION:
GATCTTNACC TTATTCAGTC AACTCCCTTT AAACCCCTGG CACTGAAAAC ACCACCTCGT 60
GTACTTACGC TGAGTGAAAG ACCACTAGAT TTTNTGGATT TAGAAAGACC TCNNNCAACC 120
CCTCAAAATG AAGAAATCCG AGCANNN 147

SEQ ID NO:5042
SEQUENCE LENGTH:98
SEQUENCE TYPE:nucleic acid
TOPOLOGY:linear
CLONE:HUMGS06024
SEQUENCE DESCRIPTION:
GATCCGTCCT CATTTTATTG GTGATGATGA ATGGGAATAA AATNAGGGGG CTGTCTACTA 60
GAGCCTGGAA TAAATATGCT GCTTTGTGGA TTTTTAAA 98

SEQ ID NO:5043
SEQUENCE LENGTH:208
SEQUENCE TYPE:nucleic acid
TOPOLOGY:linear
CLONE:HUMGS06025
SEQUENCE DESCRIPTION:

GATCTGTATG TNAATATCGC CAAGCAGGAT ATCATGTTGT CCCTGGCAAA CCTGGTGAGA 60
CTCAACCAAG CCCTTTTTCC CCCAAGCAAG AGGAAAATGG AATCTNTGTA TCAGTGTGGT 120
TTTGATGACA CTGTTAAGTT TNTACTTAAA GAAAATTGGT TTGAATAAAA TGCATAAAAG 180
TTTATANTGC ATTNNATTGT NNANNTGN 208

SEQ ID NO:5044
SEQUENCE LENGTH:211
SEQUENCE TYPE:nucleic acid
TOPOLOGY:linear
CLONE:HUMGS06026
SEQUENCE DESCRIPTION:
GATCTTTGCC CACCTCTTCC TCAGAGCCCC CACTGAGGGG CCGTAGCCCT ATCTAGGGCT 60
GTGGAAGGAG CAGACTGGTT CCTAACTCTC TCCCTCCTCC TGCCCACACA CATCAAAAGA 120
ATCTTCCCTA CACCCTTCTC TGCCTTTATT TTTTGATTTG TGCAACTTGT AACTAGGTGT 180
TTATGGAGTA AAGGAGAATG GAAAAAAGAA A 211

SEQ ID NO:5045
SEQUENCE LENGTH:345
SEQUENCE TYPE:nucleic acid
TOPOLOGY:linear
CLONE:HUMGS06027
SEQUENCE DESCRIPTION:
GATCTTGTTT ACAGCAGTGC TTTTGTGAAC AATTATTTAT TTGCTGAAAG NGCTCTTCTG 60
AACTGTGTCC TTTTAATTTT TGCTTAGAAT AGAATGGAAC AAGTTTAAAT TTCAAGGAAA 120
TATGAAGGCA CTTCCTNTTT TTCTAAGAAG GAAGTTGCTA GATGATTCCT TCATCACACT 180
TACTTAAAGT ACTGNGAAGA GTATCTGTAA ATAAAAGGGT TCCAACCTTT TAAANAAGNN 240
GGAANAAACT TTTTGGTGCT CCAGTGTAGG GCTATCTTGT NNANAAANTG TCAACAAGGG 300
GGAAANGTAA GNCTGTCNAG CTNGGGGATT GGTCACTTGN NTTAN 345

SEQ ID NO:5046
SEQUENCE LENGTH:298
SEQUENCE TYPE:nucleic acid
TOPOLOGY:linear
CLONE:HUMGS06028
SEQUENCE DESCRIPTION:
GATCTTATAA ATTTCCTGGG CAAGAGTGTA TGCATACAAA GTTTTCACTN TTGTGAAATG 60
TAATTTTNCT GTTTTTGCAA NGGGATGAGG TGATTGGAAT TGCTTTNACC ATGCTGCCTT 120
TATTCTCAAA CTGGCAAACT TAGCATGTTA GGTGTATTAA CCTCATCAGT CTTGAAGAAC 180
ATGTGGCTCA TGAGTATAAC ACTTCTGTAG NGNNCNCCCN NACANAAGTG AAGAATTAAC 240
TTCTCCTNCA GAACAAGTGC ANTTCAGAAG GCAAGCTCTG CATTCTACCT TGNTTGAN 298

SEQ ID NO:5047
SEQUENCE LENGTH:153
SEQUENCE TYPE:nucleic acid
TOPOLOGY:linear

CLONE:HUMGS06030
SEQUENCE DESCRIPTION:
GATCGGGGAG CCACAGGGTG TCCAGACCCA GCGGGAGCCG AGGATTCAGG AGAATCTCCC 60
CAGTCAGCTT CAGAGGTGCC CACAGTNTGA GCTCTAGTGT CTNCCAGGTA GGGGGNCAAA 120
GCGGNAANCT GNCCGGGCCT NGGGGGTGCA AGN 153

SEQ ID NO:5048
SEQUENCE LENGTH:351
SEQUENCE TYPE:nucleic acid
TOPOLOGY:linear
CLONE:HUMGS06031
SEQUENCE DESCRIPTION:
GATCAACATT CGCAATGCCC GGAAACACTT TAAGAAGCTG GAGAGAGTGG ATGGCCCCAA 60
GCACAGTCTC CTTATGCGTT GAATATTGCC AAATNCTCTT NCTGAAAATC CTGAATTGCC 120
TTTTTNGTTT GCATCCTTTA TTTTNAATAT TCATAATGTC GTGTGCTTAA AAGTGGGCTT 180
TGAAGTGTGT GCTGCTTACT CCTNTCATCT TTCTCCCCGC TTCCCCAGTC TTTAAACATT 240
GGACGNTATT TACTCAGCTA CCCAGTAGAG CTTGAAGCTG ACCTTTCTGA GAAAGTTGGG 300
TATGGTGGTA ACACTTAAAG TAGGTGGTTC CGTGTGGTGT TCTTCANTNN N 351

SEQ ID NO:5049
SEQUENCE LENGTH:124
SEQUENCE TYPE:nucleic acid
TOPOLOGY:linear
CLONE:HUMGS06032
SEQUENCE DESCRIPTION:
GATCAACAAA TTGCTCAACC TGCAAAATCT TTGATTAGAC TTAGCCAGCC CATCTGGCCT 60
GCTTTGGGTT TAAAAAAAAA AAAAANNTTT TTTTAACCNC ANAANTGGAA AAAGGGGAAG 120
GGNN 124

SEQ ID NO:5050
SEQUENCE LENGTH:218
SEQUENCE TYPE:nucleic acid
TOPOLOGY:linear
CLONE:HUMGS06034
SEQUENCE DESCRIPTION:
GATCATGACT ACTGGGTTGT CATTTCAATG GACAGTGCTA TGAAATAAGT ATTTTTGAAA 60
NCAACGGAAA TAGTTTATAT CGATATTTCT GATTCAAATT TANCCATCCA ATTGTTTAAT 120
TAACCTTACT GATTTTTTAC CTATATCACT TTNCTCTNAC ACTGNAANTA TTAGTTCTAN 180
TAGCATTATG NCANTTACCT ATGTTGCTTT ATGAANGN 218

SEQ ID NO:5051
SEQUENCE LENGTH:128
SEQUENCE TYPE:nucleic acid
TOPOLOGY:linear
CLONE:HUMGS06035

SEQUENCE DESCRIPTION:
GATCAATGCT GGCTTCCACG CCTGANTTCT ACCAAGTATG CCACACCAAA AAGGATTATN 60
AAGAAATTGG ACCTAGCATT TNTCGTCACA ATCCAGTGTT TGGAGTCATG TCGTAAAATT 120
GNCNCCNN 128

SEQ ID NO:5052
SEQUENCE LENGTH:234
SEQUENCE TYPE:nucleic acid
TOPOLOGY:linear
CLONE:HUMGS06036
SEQUENCE DESCRIPTION:
GATCTCATTC AGATTCCAAA GAGAATCATT TACAAGTTAA TTTCTGTCTC CTTGGTCCAT 60
TCCTTCTCTC TAATAATCAT TTACTGTTCC TCAAAGAATT GTCTACATTA CCCATCTCCT 120
CTTTTGCCTC TGAGAAAGAG TATATAAGCT TCTGTACCCC ACTGGGGGGT TGGGGTAATA 180
TTCTGTGGTC CTCAGCCCTG TACCTTAATA AATTTGTATG CCTTTNCTCT TAAA 234

SEQ ID NO:5053
SEQUENCE LENGTH:145
SEQUENCE TYPE:nucleic acid
TOPOLOGY:linear
CLONE:HUMGS06038
SEQUENCE DESCRIPTION:
GATCTGCAAG GCATAGGCAA GCCTCTTAGA GAATCAGAAA AGTGATTTGG AAGGAACTTC 60
AGCTGTTACT ACTTTGTTTA AGCAAATCAA ATGCTGTTCT TTAATGTTAC TGGACGTTTC 120
AAATTAAACA CAAGAAAACC TGAAA 145

SEQ ID NO:5054
SEQUENCE LENGTH:124
SEQUENCE TYPE:nucleic acid
TOPOLOGY:linear
CLONE:HUMGS06039
SEQUENCE DESCRIPTION:
GATCAACTGA AGACCCCCAA AGGCCCCTAC TTGATGGTTT TGAGGGGCAA CATTGACTCA 60
TTTGCCCCNT CCCTCTCGGA ATGTTGGACA AAGGGAATAA AATTGGGGAT ATGTCTACTT 120
CAAA 124

SEQ ID NO:5055
SEQUENCE LENGTH:118
SEQUENCE TYPE:nucleic acid
TOPOLOGY:linear
CLONE:HUMGS06040
SEQUENCE DESCRIPTION:
GATCTAAAAC ANCTTCAGAC TACCTANGAA ATANTTNNAC CAAATAAGAA ACAGCACTGT 60
GGAATAAAAT ATACCATTGT GAACATATCT GATGCTGCAA TGAAATGTAA AGTTCCTN 118

SEQ ID NO:5056
SEQUENCE LENGTH:316
SEQUENCE TYPE:nucleic acid
TOPOLOGY:linear
CLONE:HUMGS06041
SEQUENCE DESCRIPTION:
GATCTCCTAC TCCATCCAGT CCTGAGGAGC CTTAGGATGC AGCATNCCTT CAGGAGACAC 60
TGCTGGACCT CAGCATTCCC TTGATATCAG TCCCCTTCAC TGCAGAGCCT TGCCTTTCCC 120
CTCTGCCTGT TTCCTTTTCC TCTCCCAACC CTCTGGTTGG TGATTCAACT TGGGCTCCAA 180
GACTTGGGTA AGCTCTGGGC CTTCACAGAA TGATGGCACC TNNNNNAACC CTCATGGGTG 240
GTGTCTGAGA GGCGTGAAGG GCCTGGAGCC ACTCTGCTAG AAGAGACCAA TAAAGGGCAG 300
GTGTGGAAAC GGCAAA 316

SEQ ID NO:5057
SEQUENCE LENGTH:106
SEQUENCE TYPE:nucleic acid
TOPOLOGY:linear
CLONE:HUMGS06042
SEQUENCE DESCRIPTION:
GATCTATTTC TTTNCACTTG TATAACTTTG NAATAAATNC CTCATCCCTT AGACTAAGAG 60
AGTGGGTGTC TGTGAAAATN GTNTTCTCCT TCATAATACC TAAATN 106

SEQ ID NO:5058
SEQUENCE LENGTH:74
SEQUENCE TYPE:nucleic acid
TOPOLOGY:linear
CLONE:HUMGS06043
SEQUENCE DESCRIPTION:
GATCGAATTT AAAAGAACAA TGGAACCCTG ACTACGTTTC AACAAAAATA AAACTNGTTT 60
TTTTCCCTCC TAAA 74

SEQ ID NO:5059
SEQUENCE LENGTH:325
SEQUENCE TYPE:nucleic acid
TOPOLOGY:linear
CLONE:HUMGS06044
SEQUENCE DESCRIPTION:
GATCTCTGGC GCTGGCAGAA GCAGAATCCT TCAGGCTTTG GCANGCAAGC NTGAGGACCC 60
TCCCCTACCA AGGACCAGGA AAAGCAGCAG CTGCCTGCTC TCCAGCCTNT GGCAGGAACT 120
CAGGGCCCTG GAGCTGCTGG GGCCAAGCCA AGGCCTNCCC TACCTCAAAC CCCAGCTGGG 180
CCCGNTTAGC CCACCAGGCA TGAGGCCAAG GGCTCCACTG ACCAGGAGGC CGAGGTCTCT 240
AACTCTTATN TTCCACAGGG TCCAAGAGTT CATCAGGACC NNNAAGAGTG AGTTNAGGGG 300
GGGCAAGGNT TCTNGNAACA AAAGN 325

SEQ ID NO:5060

SEQUENCE LENGTH:107
SEQUENCE TYPE:nucleic acid
TOPOLOGY:linear
CLONE:HUMGS06045
SEQUENCE DESCRIPTION:
GATCTGCCTN TAAAGCCAGT TTAAGAAGCA CTNATGANAA CACCTGGACT GTGTATAGNG 60
ATTGCACATT CCTTATCCTT CAGCTNCAGG ACAGGTGGAG CAAATGN 107

SEQ ID NO:5061
SEQUENCE LENGTH:112
SEQUENCE TYPE:nucleic acid
TOPOLOGY:linear
CLONE:HUMGS06046
SEQUENCE DESCRIPTION:
GATCTCAGAG CATTTCAATT AGGGATGCTC AAACGCAACT TTTTCTACTT CCCCATTTCA 60
GGTGTGAGAT GTAACCCACC TTNACCATAA ATTGGCTTTN NATAGTGCTA AA 112

SEQ ID NO:5062
SEQUENCE LENGTH:298
SEQUENCE TYPE:nucleic acid
TOPOLOGY:linear
CLONE:HUMGS06047
SEQUENCE DESCRIPTION:
GATCACAACA TACAAATTCA ATTCAGTGCA TGCTTTAGGT GTTAAGCATG AGATTGTACA 60
TGTTTACTGT TAGGTCCTTG CATCTGTGGT GCTAGGTGAG TATGAGAAGA TGTCAAGGAC 120
TGGACGTATT TTGTTGCCTA AAAAAAAAAG GNTGTTTGTN GGCGTTTTAA ATATGCTTAT 180
TTNGTGTGTC NCNNNCTACC TNTTACACAC TGTTGCTTTG TGGGTTTGTT TNGNATGTGC 240
GTGTGTTATA CAGTNGTTAA ATTTCCNTGC AGAAAAATAA ATGTCCNGAA TTCTCAAA 298

SEQ ID NO:5063
SEQUENCE LENGTH:62
SEQUENCE TYPE:nucleic acid
TOPOLOGY:linear
CLONE:HUMGS06048
SEQUENCE DESCRIPTION:
GATCTCCCAG TGAGCCATCA CGATGCCCGN TTTCAAATAA ATNTTAATAT TGTCACCAGA 60
AA 62

SEQ ID NO:5064
SEQUENCE LENGTH:55
SEQUENCE TYPE:nucleic acid
TOPOLOGY:linear
CLONE:HUMGS06049
SEQUENCE DESCRIPTION:
GATCTNCTGC CATGATGCCT ATTTGGTGTN TTTCTAATTA AAATGAAATC ACAAA 55

SEQ ID NO:5065
SEQUENCE LENGTH:51
SEQUENCE TYPE:nucleic acid
TOPOLOGY:linear
CLONE:HUMGS06050
SEQUENCE DESCRIPTION:
GATCGCATCA TTGCACTCTA ACCTGGGCAA CAAGAGCAAA ACTCCATCAA A 51

SEQ ID NO:5066
SEQUENCE LENGTH:210
SEQUENCE TYPE:nucleic acid
TOPOLOGY:linear
CLONE:HUMGS06051
SEQUENCE DESCRIPTION:
GATCTGCTGA AAGGNAAGCA AGAACTATAA GGAGGNTGTG TAGTTGCAAA AATGGCTCCA 60
TCGGTAACTC AAGCTTCAGA ATGTTATGAC ATACAGAAAA TCAAAGATGC TGTGTCTTAG 120
CATTGAGTAA TATGGGTGGT GCTGTCTGGT CAGATTCAGT NAACTGTTCA GAACAAAATT 180
NCCTCTGAAA TNCNGTTGAG AAAGNCTGTN 210

SEQ ID NO:5067
SEQUENCE LENGTH:211
SEQUENCE TYPE:nucleic acid
TOPOLOGY:linear
CLONE:HUMGS06052
SEQUENCE DESCRIPTION:
GATCAAGTCA GCATTTGGGG TCGGGATTTG GGCAGGGAAC TTTGGGGTGC AAATATCTGG 60
TGAAACCACC AGAGAATTTN TTTGTCAAAG TGACATGTAT AATTTTAGTT GAGACGTGTT 120
TGTGTATATA TGTGTATTTG CCTTTNTTTA GTAGCACGGT TATTTGCTAA AATAATTGTC 180
AAAAAATTGC TCTGTCGGTC GGTCTTTGAT N 211

SEQ ID NO:5068
SEQUENCE LENGTH:230
SEQUENCE TYPE:nucleic acid
TOPOLOGY:linear
CLONE:HUMGS06053
SEQUENCE DESCRIPTION:
GATCTAGAAT CTTGTCCTCA GGCTTCTAGG GGNTTATCTG AGTCTTTTAT CTAAGACAAA 60
TGCAACAGTT TTCTTTTCAA ATGCTCAGAA AAATAGTGAT GATGATGATG ATGATGATGT 120
TCATTTGTAT ACATTTACAT GCAACCACAG CATTGTTTTT ATTATAATAC CTTAGCAATA 180
ACTAATGAGA ACATGTTAGG TGATGTTAAA TAAAATAATT TTTTCCCAAA 230

SEQ ID NO:5069
SEQUENCE LENGTH:108
SEQUENCE TYPE:nucleic acid

TOPOLOGY:linear
CLONE:HUMGS06054
SEQUENCE DESCRIPTION:
GATCGCACGG ATGCAGCAGG AAGACCAGGA GGGGCCGCCT ACGTAGCCCC AGCTCCAGCC 60
ATCCACCCCN CAGCCCTTTN CTNCAACGTC ACCANTAAAT TTNTTTTN 108

SEQ ID NO:5070
SEQUENCE LENGTH:339
SEQUENCE TYPE:nucleic acid
TOPOLOGY:linear
CLONE:HUMGS06055
SEQUENCE DESCRIPTION:
GATCACTGTT AATGATTTGC CTGTGGGACG CTCCGTGGAT GAGGCTCTGC GGCTGGTCCA 60
NNNNTTCCAG TACACAGACG AGCATGGGGA AGTTTGTCCC GCTGGCTGGA AGCTGGCAGT 120
GACACGATTA AGCCCAACGT GGATGACAGC AAGGAATATT TNTCCAAACA CAATTAGGCT 180
GGCTAACGGA TAGTGAGCTT GTGCCCCTGC CTAGGTGCCT GTNCTGGGTG TCCACCTGTG 240
CCCCCACCTG GNTGCCCTAT GCTGACCCAG GAAAGGCCAG ACCTGCCCCT CCAAACTCCA 300
CAGTATGGGA CCCTGGAGGG CTAGGCCAAG GNCTTTTN 339

SEQ ID NO:5071
SEQUENCE LENGTH:102
SEQUENCE TYPE:nucleic acid
TOPOLOGY:linear
CLONE:HUMGS06056
SEQUENCE DESCRIPTION:
GATCACAACC TCANCNAAGA CCACGANGAA GCCCNCAACA GCCACGCCCA CGACTGCCCG 60
GACGAGGCCG ACCACAGACG TAAGTGCAGG TAAAAATNGA GN 102

SEQ ID NO:5072
SEQUENCE LENGTH:231
SEQUENCE TYPE:nucleic acid
TOPOLOGY:linear
CLONE:HUMGS06057
SEQUENCE DESCRIPTION:
GATCGCCTTA ATACCAGAAA TGATTAGAAG TGCTGATTTA GATTCAACAA ATACCATATG 60
TCCTTATCAT TTTTTGTAAG AAGAAATTGG TTAAGTCCTA ACTTTCAATG TGTACCCAAA 120
TACTTGTATT TTATGCTTTT NATAAAATGT AATTTTCAGC ATTANTACAC ATCCGATTAT 180
GCCTTATTTA TATATGGNGG ANTAAAGGTT ACCATGTTTA TACCTGTTAA A 231

SEQ ID NO:5073
SEQUENCE LENGTH:250
SEQUENCE TYPE:nucleic acid
TOPOLOGY:linear
CLONE:HUMGS06059
SEQUENCE DESCRIPTION:

GATCTTGGTT TCATGTGTTT TTGAAAGTGT TATTGTTTAA AAAATGAAAA AAGCATATNT 60
GCTAAAGAGC TGTCAGGTTT TCATTACTGA CTCTGTANAA TACACTGNTC TTTGTGTACT 120
GTGTGTTATT TTGCCCAGCT NGCTGCATTA GCCTTCAAAN GTATTTGGGA AACCTNAAGG 180
ATGAACCTAC AGTTTCTNGC AAAGGTACCA TTNCCTTTCC TGGGGGTANT TTNGGTCCCN 240
GTNAACTGGN 250

SEQ ID NO:5074
SEQUENCE LENGTH:298
SEQUENCE TYPE:nucleic acid
TOPOLOGY:linear
CLONE:HUMGS06060
SEQUENCE DESCRIPTION:
GATCCTCGCG GGCCTCGGAG AATTCCCCCA CTGCCATGCC CTCCCCCACG TACCAGTGCA 60
CTAAGGCGCA CTTGGCCGAA CTTATGGTCC AGGCAGGCCC AGGCCTCCGT GATGGCCGTG 120
GTGTTGCTCA GCATGCACAC CGNCCGCTGC ACTTTGGCCA GGTCTCCCAC AGGGACCACC 180
GTGGGGGGCT GGTAGTTAAT NCCCACCTGT CAGAGATGGG CAGCATAGAA TGAAGTTTAT 240
ATTGGGCTCA GAAATGGGGA GCTCATTAAA AATCAGAGCT TGTGGNACCA GTTNCAAA 298

SEQ ID NO:5075
SEQUENCE LENGTH:80
SEQUENCE TYPE:nucleic acid
TOPOLOGY:linear
CLONE:HUMGS06061
SEQUENCE DESCRIPTION:
GATCACTGTG TCAAATGGAT GGGAAATNTT ATNTTAATAT AAACAGTAAA ATAGCATTGT 60
TTTCACTTGC AGCTTTNAAA 80

SEQ ID NO:5076
SEQUENCE LENGTH:210
SEQUENCE TYPE:nucleic acid
TOPOLOGY:linear
CLONE:HUMGS06062
SEQUENCE DESCRIPTION:
GATCTGTATG TNCAATATGG TAGCTACTAG CTACATACAT CTATGAGCCA GACACTTAAG 60
CACTTGANGT TTGGCTACTG CACATTGACA CATGGTGTTA GTAAAANCAT ACACTGGATA 120
CACTGGATTT TTGGNCTTAG TATGGAAAAA TATATTANGT ACCTCANTTT AAAAATACTT 180
ATTACATGTT AATATTTTGG TTGTANNNNN 210

SEQ ID NO:5077
SEQUENCE LENGTH:91
SEQUENCE TYPE:nucleic acid
TOPOLOGY:linear
CLONE:HUMGS06064
SEQUENCE DESCRIPTION:
GATCATGTTG TATTTNATTT TACTTATTTT TTTTACATTT CCCAAGGCTC TGTCATCCAC 60

CTTTTCATTA AATCATTCTA CCAATCTCAA A 91

SEQ ID NO:5078
SEQUENCE LENGTH:317
SEQUENCE TYPE:nucleic acid
TOPOLOGY:linear
CLONE:HUMGS06065
SEQUENCE DESCRIPTION:
GATCAATGTA AATCCTCACC TGCCCTTTGA AGAGCCAATA AGGGCATCTT TGTCTTTAAA 60
TACAGCAAAT GTTATTCAGA TTTTGTACTT GTCTATCTGT TTACCTCAGT GTAGATGATA 120
TGAGGATGGN ATAACTATGT AAATTGTTTT TGAAAATAAT TTGTTTCCCC ACAACTTTTG 180
AAGGTTTTAT TTGTNGTTGT TATTACTAGT TCAGTCCCAA ACTNACCCTG TCCTTTTCAG 240
AGAGCTAATG GCCATGCTTT NCTTCAAAGG AAAGAATAAT TNATTTGGAA TGANAGTGGG 300
GATTACTTTG CATAANN 317

SEQ ID NO:5079
SEQUENCE LENGTH:75
SEQUENCE TYPE:nucleic acid
TOPOLOGY:linear
CLONE:HUMGS06066
SEQUENCE DESCRIPTION:
GATCTTTATT CTAACCTTTT TTTGTAAAAN ATATCTATTG ATTTCCATAT GCAATAAACC 60
TTTTTTTCAG AGAAA 75

SEQ ID NO:5080
SEQUENCE LENGTH:139
SEQUENCE TYPE:nucleic acid
TOPOLOGY:linear
CLONE:HUMGS06067
SEQUENCE DESCRIPTION:
GATCGCACCA CTGCACTCCA GTCTGGCGAC AGAGTGAGAC TACATCTCAA AAAAAAANGC 60
CGCAATTTTN CATTCGNTTC ANCAGANCTC TGCCATTCTA AACATTTCTA AACTTTTACT 120
CTCAATTNCT GTACTTAAN 139

SEQ ID NO:5081
SEQUENCE LENGTH:125
SEQUENCE TYPE:nucleic acid
TOPOLOGY:linear
CLONE:HUMGS06068
SEQUENCE DESCRIPTION:
GATCTCATTT TCAGTTTAAG TAACTNCTGT TACTTAAGTG ATTGCACTTT TCTCAAATTG 60
AAGTTTAATG GAATAATAGT TCTCAGGATA GTATTTTGTA AATAAAGATG GNCTTGAATA 120
TGAAA 125

SEQ ID NO:5082

SEQUENCE LENGTH:284
SEQUENCE TYPE:nucleic acid
TOPOLOGY:linear
CLONE:HUMGS06069
SEQUENCE DESCRIPTION:
GATCCTACCA TGGCAAGAAG AGCTTCGAGA CTTTCTCTCA CCGCCGCTCT TGCCTGGTGA 60
GGCCTCTAAT GAATGATGAA GGCCTGAAGG TCAGATACCC CCCGAGCCCG GCCAAGATGA 120
CCCAGCACTG AGGAGGGGTT GCTCCGCCTG GCCTGGCCAT ACTGTGTCCC ATCGGAGTGC 180
GGACCACCNT NACTGGCTCT CCTGGCCCTG GGAGAATCGC TCCTGCAGCC CCAGCCCAGN 240
CNCANTNCTA TGNTGACCTG CTGACCTGTG CACACCCNAC TTTN 284

SEQ ID NO:5083
SEQUENCE LENGTH:280
SEQUENCE TYPE:nucleic acid
TOPOLOGY:linear
CLONE:HUMGS06070
SEQUENCE DESCRIPTION:
GATCTAACCA TGACCTGTTT CACGTCATTC ACATTCAAAA TGAATGAAGG GATGANCATT 60
TTCTCTGGTC ATTTTTTATT ATTTATACCC TTTTGCAAAT NCTAGTAGAG AATGAAGTGG 120
AGGAATAAGT TATAAATGAA CAGTGGCTTA TGACTCCAAG AGTTAATCGT GTTATTTTGG 180
TAGCTGTCTG TGTAGGTGTG CATGTNGTTG CGAGTTTGTA TTGNGNTTTA CAAGCAAAAN 240
TTATATNATG NTTCAACTTT CTGGAAATGC TTNTAATTTN 280

SEQ ID NO:5084
SEQUENCE LENGTH:62
SEQUENCE TYPE:nucleic acid
TOPOLOGY:linear
CLONE:HUMGS06071
SEQUENCE DESCRIPTION:
GATCATAATA TGAATNTCCC ACTTATTAAT AGCCTTTTAA TAAATATATA ATTAATTTCA 60
AA 62

SEQ ID NO:5085
SEQUENCE LENGTH:57
SEQUENCE TYPE:nucleic acid
TOPOLOGY:linear
CLONE:HUMGS06072
SEQUENCE DESCRIPTION:
GATCTATAAT CACCAGTGCA CTCCACCCTG GGTGACAGAA TAAGACCCTG GCTGAAA 57

SEQ ID NO:5086
SEQUENCE LENGTH:68
SEQUENCE TYPE:nucleic acid
TOPOLOGY:linear
CLONE:HUMGS06074

SEQUENCE DESCRIPTION:
GATCTCAGGA TTNAAGCACA GTGCCTGGCA TACAGCAGGT GCTCAATAAA TACTTATCAA 60
ATTGGAAA 68

SEQ ID NO:5087
SEQUENCE LENGTH:269
SEQUENCE TYPE:nucleic acid
TOPOLOGY:linear
CLONE:HUMGS06075
SEQUENCE DESCRIPTION:
GATCAAGGTT TAGTTTAACT TCCACTGTTA AAATAAAGCT TACATAGTTT TCTTCCTTTG 60
AAAGACTGTG CTGTCCTTTA ACATAGGTTT TTAANGACTA GGATATTGAA TGTGAAACAT 120
CCGTTTTCAT TGTTCACTTC TAAACCAAAA ATTATGTGTT GCCAAAACCA AACCCAGGTT 180
CATGAGTATG GTGTCTATTA TAGTGAACAT GTACTTNGAG CTTATTGGTT TTATTCTGTT 240
ATTAGATNTT TCAGGGTGTA AACANGNNN 269

SEQ ID NO:5088
SEQUENCE LENGTH:151
SEQUENCE TYPE:nucleic acid
TOPOLOGY:linear
CLONE:HUMGS06076
SEQUENCE DESCRIPTION:
GATCGGCAGG GCAGGAATCT GAGCGCTGGC CCCGTGGTGA GGCCATGTTC TCATAATCAG 60
GCTCCAGCCA GAAAAATAAG AAACACCGCA ACAGGCTTCA GTATCAGGGC TGAAACTGCT 120
GGATGANTAA ACTATTTATT AAAAACGGAA A 151

SEQ ID NO:5089
SEQUENCE LENGTH:267
SEQUENCE TYPE:nucleic acid
TOPOLOGY:linear
CLONE:HUMGS06077
SEQUENCE DESCRIPTION:
GATCTTGGCT TAATTTAATG TATTAATCTG TTTGTGCAAA CATAGTACCA CCATTTAAAA 60
ATGTTAGGGA GATGAGTTGC AGTTTTTATA ATAGATTTTT TTTAAAGTTT GGTATTGTAA 120
ANCATTCACA CCTCTGTCCC TCAAANTTGN TAATTACGGT TAAAGTGCAG TCATTTGTGG 180
NTAGAATCTT GTTTNGTTGC TNCCATTATT GGGGTCCNCC NAAGGAATTG AGGAGAGGNC 240
CGAATAGAGC CCAATTCNTA TAANGAN 267

SEQ ID NO:5090
SEQUENCE LENGTH:180
SEQUENCE TYPE:nucleic acid
TOPOLOGY:linear
CLONE:HUMGS06078
SEQUENCE DESCRIPTION:
GATCTCAGGG AAAAATTTTA ATCACTGTGT ATAATGATAC TGAACCTTGA TTAATAACAG 60

AAATTCAGGA TGTAAAGCCA CAGAATGGGN TTTATTAATG TGGGATACCT CAGACTGTTT 120
GTTTTCTTTC TGGGNAGCAA AGTGTGTTCT ATAATGAATA AATATAGAGT GGTTTTTAAA 180

SEQ ID NO:5091
SEQUENCE LENGTH:121
SEQUENCE TYPE:nucleic acid
TOPOLOGY:linear
CLONE:HUMGS06079
SEQUENCE DESCRIPTION:
GATCTTCCTG GNACCCTGAG GGTCGTCCTC CAGCAGGGGC TTCTTNGACC CCTGTCTGAG 60
GGGTCGCGTC TTAGTGGGCG GGATTCTTTT CCCTTTGAAT AAAAATGGAT TCAAACCCAA 120
A 121

SEQ ID NO:5092
SEQUENCE LENGTH:378
SEQUENCE TYPE:nucleic acid
TOPOLOGY:linear
CLONE:HUMGS06080
SEQUENCE DESCRIPTION:
GATCTGTNTT GTTCCTGCAG CAAAAATCCT CTATGGACAT AGGAGGTGCT GTGTCCCATG 60
CCCTCTTGCC CTGNCAGTGT CCCATGGGCC CCCTTCTGCT CCCTGCCCCC TCCCTGCTAC 120
TGCTGATGCA CTGTCCTCTC CCTGCAGCCC CTGGCTTCCN AGCCTTCCTC CTGACCCCTT 180
CCAACAGCCT TGGAACTCCA GCTGCCACCA CCCTNTGGGT CGGACACTGG GACCCANTGG 240
NCCAGTCTTG GNTGCTGNTT ACCCCTAGCC TTGATGNCTG CCCAGGGGAC CCCNAGCCCN 300
CTTCNGTTGN CNTGCAGCTT TAACAGAGTG AACCATGTGT ATTGTACAGG CGGGGGTTGT 360
TAATTGNAGA AAACNNAN 378

SEQ ID NO:5093
SEQUENCE LENGTH:81
SEQUENCE TYPE:nucleic acid
TOPOLOGY:linear
CLONE:HUMGS06081
SEQUENCE DESCRIPTION:
GATCACACCA CTGCACTCCA ACCTGGACAA CAGAGCAATA TTGTGTCTCA AAAAATAATA 60
AATAAATAAA TAAATAGTAA A 81

SEQ ID NO:5094
SEQUENCE LENGTH:302
SEQUENCE TYPE:nucleic acid
TOPOLOGY:linear
CLONE:HUMGS06083
SEQUENCE DESCRIPTION:
GATCACCCGG AAGTTCAGGC TGAATTCTGA AGGCAAACTT GAGCAGACGG TCTCCATGGC 60
AACCACGACA CAGCCAATNA CTCAGCATCT TCACGTCACC TACAAGAAGG TGACCCCGTA 120
AACCTAGAGC TTCTGGAGCC CTCGGGAGGG CCTGGCTACT GTGCCTCAAC GGTTCGGCTC 180

CTCAACAGAC AGTCCCTGCG GCAGAAGTGG GTGTGGCCGT GAGCCTCTGC AGGCTCAAGA 240
GTGTTGTCCA GATGTTTCTG TACTGGCATA GAAAAACCAA ATAAAAGGCC TTTNTTTTTA 300
AA 302

SEQ ID NO:5095
SEQUENCE LENGTH:55
SEQUENCE TYPE:nucleic acid
TOPOLOGY:linear
CLONE:HUMGS06084
SEQUENCE DESCRIPTION:
GATCTGACTT CAGTTGTGCA GATNACAGCA TGATAAATGA GTCGGGACCA TCAAA 55

SEQ ID NO:5096
SEQUENCE LENGTH:145
SEQUENCE TYPE:nucleic acid
TOPOLOGY:linear
CLONE:HUMGS06085
SEQUENCE DESCRIPTION:
GATCAACAGA TGCTGGCAGA AATGGCTGTN TCCCTGGCCT TGAGAAGCAG ATNCAGCTCC 60
CTGTCCCCAG AGGCCTACAG TCATATGGTA CCNCCACAGN CTCGCTTGGN TGCGCTACAG 120
GAGGCGGCGT CANGGAAGCT TNCTN 145

SEQ ID NO:5097
SEQUENCE LENGTH:199
SEQUENCE TYPE:nucleic acid
TOPOLOGY:linear
CLONE:HUMGS06086
SEQUENCE DESCRIPTION:
GATCTCATGA GAATTCACTC ACTATTATGA GAACAGCATG GGGAAAACCN CTCCCATAAT 60
TCAATCACTN TCCTCTCTCC ACACGTGGGG ATTACAATTT GAGACAAAAC CTGGGTGGGG 120
ACACAGAGCC AGACTATATC AGGGGGCTTA TGTTTATCCA NTTGTNATGG CCTCACTTNT 180
TGNAAAGACT GTNCTNNCN 199

SEQ ID NO:5098
SEQUENCE LENGTH:249
SEQUENCE TYPE:nucleic acid
TOPOLOGY:linear
CLONE:HUMGS06087
SEQUENCE DESCRIPTION:
GATCACAGAC TGTAGAGTTT TGAAAAGTCA CTTTTATTTT NAATTATTTN ACATATGCAA 60
CATGAAGAAA TCGTGTAGGT GGGTTTTTTT TTNAAATAAC AAAATCACTG TTTAAAGAAA 120
CAGTGGCATA GACTCCTTCA CACATCACTG TGGCACCAGC AACTACTTCT TTATATTGTN 180
CTTCATATCC CAAATTAGAG TTTACAGGGN CAGTCTTCAT TTACTTGTAA ATAAANTATG 240
ANTCTCAAA 249

SEQ ID NO:5099
SEQUENCE LENGTH:109
SEQUENCE TYPE:nucleic acid
TOPOLOGY:linear
CLONE:HUMGS06089
SEQUENCE DESCRIPTION:
GATCTGCAGT CCTGCCTCTG CCACAGTCTC TCTGTTGTCC CCACATCTAC CCAACTTCCT 60
GTACTGTTGC CCTTCTGATG TTAATAAAAG CAGCTGTTAC TCCCACAAA 109

SEQ ID NO:5100
SEQUENCE LENGTH:341
SEQUENCE TYPE:nucleic acid
TOPOLOGY:linear
CLONE:HUMGS06090
SEQUENCE DESCRIPTION:
GATCACGGAC GTGCAGCTCG CCATCTTCGN CAACATGCTG GGCGTGTCGC TCTTCTTGCT 60
TGTCGTTCTC TATCACTACG TGGCCGTCAA CAATCCCAAG AAGCAGGAAT GAAAGTGGCG 120
CTTTCTCCGN CCCAGGGTTC CAGGACATAG TCTGAGGCAA GATGGAGGGT ATGTGGGGCC 180
TTCACACTTC ACTTCATCCC TTCTACCCAT CACANCATAC AAAGCANCTA CACCTGGATT 240
TTTCCAAACA ACTTTTATTT CCTCAGAGTC NGNNGNAATN CTATGGNNCA AGANGCTGNC 300
ACTGAATAGG GCCNAGTATA GGGGCTTGCT TTTCTACTCT N 341

SEQ ID NO:5101
SEQUENCE LENGTH:117
SEQUENCE TYPE:nucleic acid
TOPOLOGY:linear
CLONE:HUMGS06091
SEQUENCE DESCRIPTION:
GATCTGGTAC CGCAGTAGCG CCGCCGCCGC CGCCGNCGGA GCCTGTCGTC GTCCTGTCCC 60
CAGCCTNCTT GTGTCCCGTG AGGTTGTCAA TAAACCTGCC CTCGGGCTGC CGGCAAA 117

SEQ ID NO:5102
SEQUENCE LENGTH:151
SEQUENCE TYPE:nucleic acid
TOPOLOGY:linear
CLONE:HUMGS06092
SEQUENCE DESCRIPTION:
GATCCAAAAG GACTCATCTA AAACACAAGT TGGGTAGAGG AGATAGGTGA GTACTATTAT 60
ATTATGTTCA TGGAAAATNA GTTCATGGGC TTTCCTCTAA TTTTTATTTT AAAAATAAAT 120
GTAAATATTA AATAAAATTT TTTAAATGAA A 151

SEQ ID NO:5103
SEQUENCE LENGTH:86
SEQUENCE TYPE:nucleic acid
TOPOLOGY:linear

CLONE:HUMGS06093
SEQUENCE DESCRIPTION:
GATCAGCTTT TAATATCTGT CAAATAAAAA TTGAAATTGA AGATGGTATA TAAGATTTCA 60
GNAAATAAAC TACTCTGAAT ACTAAA 86

SEQ ID NO:5104
SEQUENCE LENGTH:210
SEQUENCE TYPE:nucleic acid
TOPOLOGY:linear
CLONE:HUMGS06094
SEQUENCE DESCRIPTION:
GATCTAATCC GTNTCCCACA TGTCCCAGGG AATCCNAGGT CTGCCCTGAG CTTCCTGTGT 60
CCTTGGNAAA GTCGGTTAAT CTCTGGGCCT CACTGTCCTC ATCGCTGAGC TTGGGGACTT 120
GTAACANATG ATTTCTAAGT ATCTNCCAGC TTTGANTAGA GCATTTGTNT TGGGTGGCCA 180
GGCTTAANGT TTAAAGGCAT AANGAAAANN 210

SEQ ID NO:5105
SEQUENCE LENGTH:242
SEQUENCE TYPE:nucleic acid
TOPOLOGY:linear
CLONE:HUMGS06095
SEQUENCE DESCRIPTION:
GATCATATTG GTCATGTCTA TTGGTGTATT ATTTCAGTAT CACCAATGTT TTCAGAAATA 60
CAGTACTAAT TCATCATTAA ACTCTTTGAA GTTAATATTT TCCTGCCTTC TAACTTATAG 120
ACTCAACTAT GTATCTGTAG TTTTTGGGAA TGGGGTGGTG TTTTTNNCTT TGTGTTGGGA 180
AGTTATTGAG AAAACCTATA TAATANAATT NAANATTATA GTTTTAAAAA AANNNCANNN 240
NN 242

SEQ ID NO:5106
SEQUENCE LENGTH:219
SEQUENCE TYPE:nucleic acid
TOPOLOGY:linear
CLONE:HUMGS06097
SEQUENCE DESCRIPTION:
GATCTGCCCT TGGCCTCCCA AAGTGTCGGA ANNACAGGCA TGNGCCACTG AGCCTGANCC 60
ACTTTAGCTA TTTTNAAAAA TAGGACCTAT TGTTTTGGAT TACTATCTGA GAATTTCATG 120
GATTCACTCC TGGGTATAGG AGNTAAGAAT ACGTTTAATT CATAACTTTT ATATTANCCA 180
ATATAGTAAA AAAATAAAGC TCACACATAC ANAANCAAA 219

SEQ ID NO:5107
SEQUENCE LENGTH:227
SEQUENCE TYPE:nucleic acid
TOPOLOGY:linear
CLONE:HUMGS06098
SEQUENCE DESCRIPTION:

GATCTATTTT TTGGGCTTTT AATATTCTGT TCCATTGACC TATNTCNCTT NTCTTTTACC 60
AATACCGCAA CATCTTGGTT ACTGTAGCTT CACGGTAAGT CTTAAAACTT GGATATTGTC 120
AGTCCTCCAA CTATGTTCTA CTCCTTCAAT ATTGTGTTGG TTATTCTGAG TCTTGNNGGG 180
TCTCCATATA AATNGTAGAA TTAGAAAAAA AANNGNGGGG GGGANNN 227

SEQ ID NO:5108
SEQUENCE LENGTH:135
SEQUENCE TYPE:nucleic acid
TOPOLOGY:linear
CLONE:HUMGS06100
SEQUENCE DESCRIPTION:
GATCAGTTTG GACTCTGTAA CTCATAGGTG CATTCTTTTT TTCTTTGGTG TCTCACAAAT 60
NACCCAATTG TTGAATAAAA CTATAAATAT GTTAATACCA GCTTTTTCCA ATAAAATAAC 120
AAATGTTACA TCAAA 135

SEQ ID NO:5109
SEQUENCE LENGTH:167
SEQUENCE TYPE:nucleic acid
TOPOLOGY:linear
CLONE:HUMGS06101
SEQUENCE DESCRIPTION:
GATCTNATGG GTTTAAAAGN GCGGCATTTC CCTCCNCTTT CTCCCTCTCC TGCACCATGT 60
TAAGATGTGC CTTGCTTCCC CTTTGCCTTC GACCATGATT GTAAGTTTCC TGAGGCCTCC 120
CCTGCCATTC AGAACTGTGA GTAAATTAAA TCTCTTTNCT TTATAAA 167

SEQ ID NO:5110
SEQUENCE LENGTH:101
SEQUENCE TYPE:nucleic acid
TOPOLOGY:linear
CLONE:HUMGS06102
SEQUENCE DESCRIPTION:
GATCTTCTGC CACCATCAGG GNCAAACGGG TGCAGGAGGA AAGTCACTGA TGCCCAGATG 60
TTTGCNTCCT GCACAGCTAC AGGTCCTTAA NNAAAAGTNT N 101

SEQ ID NO:5111
SEQUENCE LENGTH:172
SEQUENCE TYPE:nucleic acid
TOPOLOGY:linear
CLONE:HUMGS06103
SEQUENCE DESCRIPTION:
GATCTGTGGC TACTTTCACA CCACAACAGA GTACCATGGT TCTGACAGAG ACTAGGAGAC 60
ACAGTCTAAA TGACTTCTGA CCTGGACCTT TACTGAAAAT CCTGCCAATC ATTCTGTTGG 120
CAAGANTGAT GTATTACTTT TAGCAATAAG AAACAAGTAA CCTTTGCAGA AA 172

SEQ ID NO:5112

SEQUENCE LENGTH:74
SEQUENCE TYPE:nucleic acid
TOPOLOGY:linear
CLONE:HUMGS06104
SEQUENCE DESCRIPTION:
GATCTCCAAA GCTGTCCTCT GGCGTCCAGG AGTGCCTCTT ATGTGAGTCC TAATAAACTC 60
ATCTATTCAC CAAA 74

SEQ ID NO:5113
SEQUENCE LENGTH:134
SEQUENCE TYPE:nucleic acid
TOPOLOGY:linear
CLONE:HUMGS06105
SEQUENCE DESCRIPTION:
GATCTNTTCC CACCCATAAA AAGCCCAGAG AACAAAAAGT TGGCCCAGGA AATGCAATTN 60
GGAAGCTGAA GAAGATTATA GTATTTNTNG CAGTCAAGAT TTGGAAAGTT ACCNACTAAG 120
AAGCTACAAT ATNN 134

SEQ ID NO:5114
SEQUENCE LENGTH:186
SEQUENCE TYPE:nucleic acid
TOPOLOGY:linear
CLONE:HUMGS06107
SEQUENCE DESCRIPTION:
GATCTTGAAA TNAAACTAAG ATTTTCCTGG GGAAATGTTC AGATACAGTT TTGTGAACTG 60
TAAATCAAAA TACCTTTTNC TACAGTTTAT CTTTNATNTC CTGCAAATTT AGGAACATAT 120
TTCCTCGTTT NCACATTGAA TCTTAAGTTT AAGCNCTTCA TTTGGNATTT NGGCAATATA 180
TGNGAN 186

SEQ ID NO:5115
SEQUENCE LENGTH:275
SEQUENCE TYPE:nucleic acid
TOPOLOGY:linear
CLONE:HUMGS06108
SEQUENCE DESCRIPTION:
GATCTTTNTG ACTAGAGTTA GTGTCCTAGG AAAACCAGAA CTCAGAACTT GCCTCCATGG 60
TTGAGTAACA AGCTGTACAA GAACCCCTTT TATCCCTGGA AGAGGCTGTG TATGAAACCA 120
ATGCCCAGGG TTTGAAGGGT GTTAGCATCC ATTNCAGGGG AGTGTGGATT GGCTGGCTCT 180
CTGGTAGCAT TTTGTCCTNA CACACCCATC TACTATGTCC AACCGGTCTG TCTGCTTCCC 240
TCACCCCTTG CCCAATAAAG GNCAAGNNCT TCAAA 275

SEQ ID NO:5116
SEQUENCE LENGTH:122
SEQUENCE TYPE:nucleic acid
TOPOLOGY:linear

CLONE:HUMGS06109
SEQUENCE DESCRIPTION:
GATCGAGTGG TCTGTGTTCC TATTGCTGGT GGGGTGATAG GGTGGGCTAA AAACCATGCA 60
CTCTGGAATN TTTTGTATTT TCTCCCAGTA AAGCTTTTCT TCTCCCGAAA AAAAAAAAGA 120
AA 122

SEQ ID NO:5117
SEQUENCE LENGTH:94
SEQUENCE TYPE:nucleic acid
TOPOLOGY:linear
CLONE:HUMGS06110
SEQUENCE DESCRIPTION:
GATCACAAAC TACAGCCTGC AGGCCAAATC CAGCCCACAG CCTGTNTTTG TAAATAAAGC 60
TTTGTTGGAN CAAAGCCACA CCCCTTAATC TAAA 94

SEQ ID NO:5118
SEQUENCE LENGTH:80
SEQUENCE TYPE:nucleic acid
TOPOLOGY:linear
CLONE:HUMGS06111
SEQUENCE DESCRIPTION:
GATCTGAATC CAGNTCATTT GAGGGAACCC CCAAGGTTCT CTGGTTGTGG CCTCTTGAAT 60
AAACCTCTCA CATTTGGAAA 80

SEQ ID NO:5119
SEQUENCE LENGTH:274
SEQUENCE TYPE:nucleic acid
TOPOLOGY:linear
CLONE:HUMGS06112
SEQUENCE DESCRIPTION:
GATCTATTAT CTAGCTGCAA AGCCTGGCTT TGNTTTGNAA TTTTGTAAAA ATTTCATGGC 60
ACCCAAGGTT TCTGNTTCTG ACCCAGCAGT GGTCCTGAAG AGAGCTGATG GCAAGTNTTG 120
TAGTCATTTT GATTTTAATT GAAGGGTGAG CATAACCTTG TGAACCAGCA CTAGCTTGTT 180
CCAAGCTGGA ATTTATCTAA TCTATTTTTG TGTTTAAAAA AGCTGTACCT ACCAAATAAA 240
TAAATAGTTT ATAAAATGTA TTACTTAAGG TAAA 274

SEQ ID NO:5120
SEQUENCE LENGTH:350
SEQUENCE TYPE:nucleic acid
TOPOLOGY:linear
CLONE:HUMGS06113
SEQUENCE DESCRIPTION:
GATCTTTGAA GATATCCTCA ACGTGAGGCT CTNCTGCCAT GAAGGTGAAG ATTAAGTGCT 60
GGAACGGCGT GGCCACTTGG CTCTGGGTGG CCAACGATGA GAACTGTGGC ATCTGCAGGA 120
TGGCATTTAA CGGATGCTGC CCTGACTGCA AGGTGCCCGG CGACGACTGC CCGCTGGTGT 180

GGGGCCAGNG NNCCCACTGC TTCCACATGC ATTGCATCCT CAAGTGGCTG CACGNACANC 240
AGGNGCAGCA GCACTGNCCC ATGGTGCCGN CAGGAATGGA AGTTCAAGGA GTGAGGCCCG 300
ACCTNGNNTC TAGNTGGGAG GGGNATNCTG AAGACTTCTT TNCTTNATTN 350

SEQ ID NO:5121
SEQUENCE LENGTH:360
SEQUENCE TYPE:nucleic acid
TOPOLOGY:linear
CLONE:HUMGS06114
SEQUENCE DESCRIPTION:
GATCTTATTC AGTCTGCTGN TTTGTATAGT GTGATGACCC TAGTAAGCAC ATATTTGGTA 60
GCCTTTGCAT ACAAGANTGT GAAATTTGTT CTCAAGCACA AAGTAGCACA GNAGNGGGAA 120
GATGCTGTTT CCAAAGAAGT GACTCGAAAA CTTTCTGAAG CTGATAATAG AAAGATGTCT 180
CGGAAGGAGA AAGATGAAAG ATTCTTGTGG AAGAAGAATG AAGTNNCNNN ATTATGAAGC 240
TACANCANNT TCCATCTTCT ATAACAACAC TCNGTTCCTG GTCGTNGTCA TTGTTGCTTC 300
CTTCTTCATA TTGGAAGAAT TTCAACCCCA CAGTGAACTA CANATTTGTC CATAAGTTGN 360

SEQ ID NO:5122
SEQUENCE LENGTH:88
SEQUENCE TYPE:nucleic acid
TOPOLOGY:linear
CLONE:HUMGS06115
SEQUENCE DESCRIPTION:
GATCTGGTCA CACCTAAAGT CATTAAAATC ACTGAAATGT TTAGATACAC AGCCCAATAA 60
ATTCTCTTTA TAAAACACAA GCAGTAAA 88

SEQ ID NO:5123
SEQUENCE LENGTH:177
SEQUENCE TYPE:nucleic acid
TOPOLOGY:linear
CLONE:HUMGS06116
SEQUENCE DESCRIPTION:
GATCAGGCCA CATAAGGAAC AGGGGAATTC CAGGGGTGGG ACACAGCTNG GGGAGTCCAG 60
ACCAGGGCAG GGAAAGGAGA CTCACAAGCC AAACAGAGCT GCTTTGGGGA AAGTTCTTAT 120
CAGCTGGTGC TGCTTCCTGA GCCATATGCC CATTCCTCAA GCTGTACCCN NNNNNNN 177

SEQ ID NO:5124
SEQUENCE LENGTH:60
SEQUENCE TYPE:nucleic acid
TOPOLOGY:linear
CLONE:HUMGS06117
SEQUENCE DESCRIPTION:
GATCTTGGAC CTCCCAGCCT CCAGAACTGT GAGAAATAAA TNTGTTTTGT TTCAGCTAAA 60

SEQ ID NO:5125

SEQUENCE LENGTH:54
SEQUENCE TYPE:nucleic acid
TOPOLOGY:linear
CLONE:HUMGS06118
SEQUENCE DESCRIPTION:
GATCTGGCTG ACTCACACGC GACTGGAGAA TAAACAAACA GCAACATAAG GAAA 54

SEQ ID NO:5126
SEQUENCE LENGTH:316
SEQUENCE TYPE:nucleic acid
TOPOLOGY:linear
CLONE:HUMGS06119
SEQUENCE DESCRIPTION:
GATCTCCCAT GGGTCACCCA GGTCCTATGC CTCCGCATGG TATGCGTGGA CCTCCTCCAC 60
TGATGCCCCC CCATGGATAC ACTGGCCCTC CACGACCCCC ACCCTATGGC TACCAGCGGG 120
GGCCTCTCCC TNCACCCAGA CCCACTCCCC GGCCACCAGT TCCCCNTNGA GGCCCACTTN 180
GAGGCCCTCT NCCTCAGTAA ATTCACATTT TNCTTCCTNC TGTTACATTT TNCNAATATN 240
TTTTCTATTC CTTGGACCAA TCAGAGATGC TGTAGCTTNC TTGGGGCAAA GGGTACTAAA 300
TCNCNTTTNA GCACNN 316

SEQ ID NO:5127
SEQUENCE LENGTH:222
SEQUENCE TYPE:nucleic acid
TOPOLOGY:linear
CLONE:HUMGS06122
SEQUENCE DESCRIPTION:
GATCCTGTTC CTGTCACCAT TGCTCTCGAT TCACTCAGCT GGCTGCTACT TCGCCTTCCC 60
TGCACCACAC TCTGCCAGGT CCTGCATGCT GTGAGCCATC AGGACTCTTG TCCTGGTGAG 120
ACCCCTCCTT CATTGTTTCC CCTCATACAT CTCCCTCTGC CAAGGAGTGT GCCCCTTTTC 180
CTTTCTACCC TAGAATAAAC ATCTGGGTTC TCCAGTCAGA AA 222

SEQ ID NO:5128
SEQUENCE LENGTH:130
SEQUENCE TYPE:nucleic acid
TOPOLOGY:linear
CLONE:HUMGS06123
SEQUENCE DESCRIPTION:
GATCGACTGG AACAAAGTGA CCGCTGNGTA AAACTACTGC CTTGCCACTC ACTGTTGTAT 60
ACATTTCTTA TTTACGATTT TCATTTGTTA TATATATATA TAAANATACN GTATANANAT 120
GCAACATANN 130

SEQ ID NO:5129
SEQUENCE LENGTH:160
SEQUENCE TYPE:nucleic acid
TOPOLOGY:linear

CLONE:HUMGS06125
SEQUENCE DESCRIPTION:
GATCTTAGAG CTAAAAAGGA CTGTAAAAAT TACCCAGAAC AGCGTCCTCA GACTTAACCT 60
TCTGCAAGTT ATGTCTGTAT ATAAGANGAT TCTAATTGCT AACTGTTTAT ACTTTNCTGN 120
ATAAAATAGT TGTTTCTAAT TAAAANGTAG CCAAGCTAAA 160

SEQ ID NO:5130
SEQUENCE LENGTH:349
SEQUENCE TYPE:nucleic acid
TOPOLOGY:linear
CLONE:HUMGS06126
SEQUENCE DESCRIPTION:
GATCTTCGTT CAGGGCATCA TCTGGGACAT CAACAGCTTT NACCAGTGGG GAGTGGAGCT 60
GGGAAAGCAG CTGGCTAAGA AAATAGAGCC TGAGCTTGAT GGCAGTGCTC AAGTNACCTC 120
TCACGACGCT TCTACCAATG GGCTCATCAA CTTCATCAAG CAGCAGCGCG AGGCCAGAGT 180
CCAATAAACT CGTGCTCATC TGCAGCCTCC TCTGTNACTC CCCTTTCTNT TCTCGTCCCT 240
NCTNCCCGGA GCCGGCACTG CATGTTCCTG GACAACACCC AGAGCACCCT NTNGTTGTGG 300
GNTTTTGNNC NNCGAGCCCT TTAGCNGGGA ANGNCTTGGT CTTCNCCAN 349

SEQ ID NO:5131
SEQUENCE LENGTH:148
SEQUENCE TYPE:nucleic acid
TOPOLOGY:linear
CLONE:HUMGS06127
SEQUENCE DESCRIPTION:
GATCCTCAAG GACCTGTAAT TNTTTGGTAT TTAAAATTAA AATAACATTA TTTTTATTTT 60
GANTTTTTTT CAAACAGTAA GAATAATATA TCAGCAGCAT GTATCTGCCA ATCATATGTA 120
ATAAATTNTT TATTTTGCCA AAACCAAA 148

SEQ ID NO:5132
SEQUENCE LENGTH:101
SEQUENCE TYPE:nucleic acid
TOPOLOGY:linear
CLONE:HUMGS06128
SEQUENCE DESCRIPTION:
GATCTAATAC AAGANCTAGC AAAGCAAAAG TNTTTGCAAG TGACAGCATA AAGACGGANC 60
ACAACAAATC CTTCCTGNAG TGAATTAGGA AACTCCANGG N 101

SEQ ID NO:5133
SEQUENCE LENGTH:347
SEQUENCE TYPE:nucleic acid
TOPOLOGY:linear
CLONE:HUMGS06129
SEQUENCE DESCRIPTION:
GATCCTCATT CTTTCCCCCG TACAGTGGAA AACATCTTTN ATGTTTCCTT CATTATACGG 60

GATGGTTTTN CAAGAATAAG ACTTGACCAA GACCGACTGC CAGTAATAGA GCCTGTNAGT 120
ATTAATGAAG AAAATNAGGG ATTTGAACAT AACACACAAG TTAGAAATCA AGGAATTATA 180
GCTTTGAGTT ACCGTGACTG GGNGGNGATT GTGAAGACCT TTTGAGATTT CAGAGCCTGT 240
GATTACTCCA AGTCAGAGGC AGCAGNNGNC AAGTNCTTTG NTTGCTAGCT GAAAGGACTT 300
AAATGGNTAG TGAAGGTCCA AAACCGGGNA AGCGGCATNG TGTTGGN 347

SEQ ID NO:5134
SEQUENCE LENGTH:72
SEQUENCE TYPE:nucleic acid
TOPOLOGY:linear
CLONE:HUMGS06130
SEQUENCE DESCRIPTION:
GATCACACTG CAGTTTCCAT GTTAGCACTG TGGATGGGTT TTNAATCAAT AAAAACTGGG 60
GGTTTCTTCA AA 72

SEQ ID NO:5135
SEQUENCE LENGTH:203
SEQUENCE TYPE:nucleic acid
TOPOLOGY:linear
CLONE:HUMGS06131
SEQUENCE DESCRIPTION:
GATCTGTAAA ATAAAAACTC AGCTGGGTCC AGATATCAGG TGTTCTAAAT CTAAGAATGT 60
AAGAACAATC TTAATAGAAA TATGTNTTTA ATAAGTGGCT AATATCTACA AATAGTACCT 120
TTGCTAAATG ANTTCTTTTT NATGACTATN TTGTTTTTNA GNAATTGGGT GAAAANNCAA 180
CTGANATGAG ATTATTTATN ATN 203

SEQ ID NO:5136
SEQUENCE LENGTH:129
SEQUENCE TYPE:nucleic acid
TOPOLOGY:linear
CLONE:HUMGS06133
SEQUENCE DESCRIPTION:
GATCTTAGAT TTNATGAAGC ACAGTATGCA GGTAGGCCTA ATGGGGGAAG ATGGTAATAT 60
AAAAGCAAGA AGTATTTTTT TTTTTGAAAT GCCTGAANGC TGTNCTGNGG NTGCCCTCCC 120
CTTNCCTTN 129

SEQ ID NO:5137
SEQUENCE LENGTH:270
SEQUENCE TYPE:nucleic acid
TOPOLOGY:linear
CLONE:HUMGS06136
SEQUENCE DESCRIPTION:
GATCACCTGA GGTCGGGAGT TCGAGACCAG CCTGACCAAC ATGGAGAAAC CCCATTTCTA 60
CTAAAAATAC AAAATTAGTC AGGCATGGTG GCGCATGCNT GTAATCCCAG CTACTTGGGA 120
GGCTGAGGCA GAAGAATCGC TTGAAACTGG GAGGCGGAGG TTGCGGTGAG CCGAGANCAT 180

GCCATTGCAC TCCAGCCTGG GCAGCAAGAG TGAAGCTCCA TCTAAAAATA AATAANTAAA 240
TAAATAANTA ANCCCTCANA NNNAAAAATN 270

SEQ ID NO:5138
SEQUENCE LENGTH:51
SEQUENCE TYPE:nucleic acid
TOPOLOGY:linear
CLONE:HUMGS06137
SEQUENCE DESCRIPTION:
GATCTGTAGG GGAGGGAGTT CAAATAAAGC TTTATTTTTT TCATTTTCAA A 51

SEQ ID NO:5139
SEQUENCE LENGTH:190
SEQUENCE TYPE:nucleic acid
TOPOLOGY:linear
CLONE:HUMGS06138
SEQUENCE DESCRIPTION:
GATCTGGGCT CCCTGACTTT CTGAAGCTAG AAAAAGGTTG TGTCTCCCAA CCACCTTTCC 60
ATCCCCAGCC CCTCTCATCC CTGGAGCACT CTGCCGCTCA AGAGCTGGTT TGTTAATTAT 120
TGTTAGACTT TGCCATTGTN TTCTTTTGTA CCTGAAGCAT TTTGAAAATA AAGTTTACTT 180
AAGTTATAAA 190

SEQ ID NO:5140
SEQUENCE LENGTH:199
SEQUENCE TYPE:nucleic acid
TOPOLOGY:linear
CLONE:HUMGS06139
SEQUENCE DESCRIPTION:
GATCGGGAGG ACCTGTATGC CTGACCGTTT CCCTGCCTCC TGCTTCAGCC TCCCGAGGCC 60
GAAGTCTCAG CCCCTCCAGA CAGGCCGCTG ACATTCAGCA GTTTGGCCTC TTTCCCTCTG 120
TCTGTGCTTG TGTTGTTGAC CTCCTGATGG CTTGTCATCN TGAATAAAAT ATANTAATAA 180
ATTTNGTATA AATAGGAAA 199

SEQ ID NO:5141
SEQUENCE LENGTH:312
SEQUENCE TYPE:nucleic acid
TOPOLOGY:linear
CLONE:HUMGS06141
SEQUENCE DESCRIPTION:
GATCCAACCA CCTCAGCCTC CCAAAATGCT GGGATTATAG GCGTGAGCAC TTGCACCTGG 60
CAAAAAAATT TTTTTTAAAA AAGGAGAAAG CAGTTTAACC ACAAACAGTG AAACAGAGCT 120
GGCAGACGCC TGAAACTCCA TGGAACCATG CAGCTGTACC CTAGGGCTCC TCCCAGGAAA 180
AGAGAGATGT CAACGTGGCC CAAGGTGTCC TGAAGCACTG GGATTGGNTT ANCTCGAGCG 240
NTGGCCTTCC CAGCCCTNCG TGCTGCAGAG CCTCTGCTCG CTAGGCTNCC TTCATNCTNA 300
CTCTGTGTGC TN 312

SEQ ID NO:5142
SEQUENCE LENGTH:229
SEQUENCE TYPE:nucleic acid
TOPOLOGY:linear
CLONE:HUMGS06142
SEQUENCE DESCRIPTION:
GATCTAAGAC AGCAAAGAAG TGTGCAAGGA GGGCCCTGTT AGCTCCCACT GTCCTGGTTT 60
CTCCTCCTGG AGTCTAATTT CCTTGGCCCT CTGAGCCTTT TGAGTCTGGG CCCTGGTCCA 120
ATGCTGCTGT TGTCTGAGGA ATGGTTTGGT GAGAACAGAT GTTAGAACTT GTTTGTTGAT 180
TCTTGTCTGG CTAATAAATC ANCACCAACT GNCTTCTCCN ACAGGGAAA 229

SEQ ID NO:5143
SEQUENCE LENGTH:332
SEQUENCE TYPE:nucleic acid
TOPOLOGY:linear
CLONE:HUMGS06144
SEQUENCE DESCRIPTION:
GATCTAAGAT TATTCCCATG AGAAATGTTG AATTTATGAA GAATAGATTT TAAGGCTTTG 60
AAAATGGTTA ATTTCTCAAA AACATCAATG TCCAAACATC TACCTTTTTT CATAGGNGTA 120
GACACTAGCA AGCTGGNCAA ACTATCACAA AAGTATTTGT CACACATAAC CTNTGGTCTG 180
TTGCTGATTA NTACAGTACT TTTTCTTTGT GTGATTCTNA ACATTATAGC ACAAGTATTA 240
TCTCAGTGGG TTATCCGGAA TAACATCTGA AAGNGGGGTT CATCTNTGGT NGNGTTTGCT 300
CNNTAAACTA GNGTNTCTCC TGGTCCCANT GN 332

SEQ ID NO:5144
SEQUENCE LENGTH:275
SEQUENCE TYPE:nucleic acid
TOPOLOGY:linear
CLONE:HUMGS06145
SEQUENCE DESCRIPTION:
GATCACATCA CGGAATATTC TTTGCCTTTC CACTTTCCAG GAAATCTCTC GGACTGGGCT 60
AGCCTCCTTG TGTGTGATGA AAGATGGGCT ATATTTCAGA ACANAGTGCT GTGTTGTCAT 120
GATTTGCCTG GACTCCCAGG GCGTCTCTTA CCCAACTTGA TAACGATGCT GTTCATTAGC 180
AGCCTGTGTT AACTGATAAC CAAGAGCGGT AATGTGATAC TCATAAGCAA TTTTCTGTGT 240
GTAGGATAAA ATANNCCATC TTGTATNATT NCAAA 275

SEQ ID NO:5145
SEQUENCE LENGTH:92
SEQUENCE TYPE:nucleic acid
TOPOLOGY:linear
CLONE:HUMGS06146
SEQUENCE DESCRIPTION:
GATCTCTTTT CAAGTTCACT CATTCTTTCT TCTGTAATGT TCAATCTCTC TTAAGAAAGT 60
CCAAATAAAT TTTTCATTTC AGAGAGTGGA AA 92

SEQ ID NO:5146
SEQUENCE LENGTH:323
SEQUENCE TYPE:nucleic acid
TOPOLOGY:linear
CLONE:HUMGS06148
SEQUENCE DESCRIPTION:
GATCTGGGCA TTAAGTCAGT GGCTTTGCAT AGCTTTCACA AGTCTCCTAG ACACTCCCCA 60
CGGGAAACTC AAGGAGGTGG TGAATTTTTA ATCAGCAATA TTGCCTGTGC TTCTCTTCTT 120
TATTGCACTA GGAATNCTTT GCATTCCTTA CTTGCACTGT TACTCTTAAT NTTAAAGACC 180
CAACTTGCCA AAATNTTTGG CTGCGTACTC CACTGGTCTG TCTTTGGATA NTAGGNATTC 240
AATTTGGCAA AACAAAGTGT AATGTCAGAC TTTGCTGCAT NTTACACATG TGCTGATGNT 300
NACAATGGTG NCGAACATNA GTN 323

SEQ ID NO:5147
SEQUENCE LENGTH:347
SEQUENCE TYPE:nucleic acid
TOPOLOGY:linear
CLONE:HUMGS06150
SEQUENCE DESCRIPTION:
GATCTTTTGA GTGGAGGTGG GTAGAGAGAG CAAGNAGGGC AGGACACTTA GCAGGCACTG 60
AGCAAGCAGG CCCCCACCTG CCCTTAGTGA TGTTTGGAGT CGTTTTACCC TCTTCTATTG 120
AATTGCCTTG GGATTTCCTT CTCCCTTTCC CTGCCCACCC TGTCCCCTAC AATTTGTGCT 180
TCTNAGTTGA GGAGCCTTCA CCTCTGTTGC TGAGGAAATG GTAGAATGCT GCCTATCACC 240
TNCAGCACAA TCCCAGCGAA AAAGGGTGTG AAGNACCCAC CATGTTCTTG GAGCANTCAA 300
GGGTTNCTAA ATNNNNCGGC TGGGACCANT NANATAGTGA GTNNNNN 347

SEQ ID NO:5148
SEQUENCE LENGTH:388
SEQUENCE TYPE:nucleic acid
TOPOLOGY:linear
CLONE:HUMGS06151
SEQUENCE DESCRIPTION:
GATCTGCCAT ACCACTGTGA CTGCATTGAA AGATGTCCCT TTCTCTCTCT GCCAGTCGGT 60
CGGTGTGGGA CAAAACACAG ATATACAGCA GATAGCGGCC AAAAACTTCA CAGACACCTC 120
TCTCATGTAC CCAGAGGACA CTTGTAAATC TGGACCAAGT ACGAATACAC AGAGTGGTCT 180
TTTCAACACT CCTCCCCCTA CTCCACCGGA CCTCAACCAG GACTTCAGTG GATTTCAGCT 240
TCTTAGTGGA TGTTGCACTC AAACGGGGCT GCAGAGATGG AGGCTTNAGG GNANANCTTT 300
ACAGNTTAAC CCNATTTTCA AGCANAAACA GTTCTCANGA AGTGTCATGA TTGNCCGGGG 360
TAGAAGGGAA AGAGATTGAA TTTGCTTN 388

SEQ ID NO:5149
SEQUENCE LENGTH:308
SEQUENCE TYPE:nucleic acid
TOPOLOGY:linear

CLONE:HUMGS06152
SEQUENCE DESCRIPTION:
GATCTATAAT GGGATACATT GTCATCCTCT AGCAACTCCT ATATAGAAAG TTTTAACTGA 60
ATATGTTACA TATANGAATT AAATTCTTCT CAAATAATTC TTAACCTCAG TNATGAGCCT 120
AAATTTACTC TGCTTGGCTC TCTACACATG GCATTTCAGG GTATAAGATG TAGCATTTNA 180
ATGTGTAAGA TATATGTACN NNNCATATGT GTTGCTATCN TCATCATTAA CATCCTTCTT 240
TNCTATTGCT TGGCTGTAAT TTTTGTAAAG ATAAATTATA TTGTNTNNNT GTATGTGTGT 300
TTGTAGTN 308

SEQ ID NO:5150
SEQUENCE LENGTH:316
SEQUENCE TYPE:nucleic acid
TOPOLOGY:linear
CLONE:HUMGS06153
SEQUENCE DESCRIPTION:
GATCTGTCCT GTGAGGCATT TAGGGGCTTT CAGGAATTTA GTAAAAGGTG GAGTATGCCT 60
TTCCAGTATC TTCCATCTTC CTTTGTATAC TTGTCCTTCC TCCCATTTCC TCCCTTTGGC 120
CCGAGGTAGG AGGATGGAGG GAGGCTGCTA CTCTACCACT TCCTGTGTGC CTCTACTGTG 180
GCCTCAACCC TGGCAATTAT AGCTACTCCC ATCCCTTACC TGGGCATGTG TGAGCCCTTC 240
TCACTGGATT TTATACCCTT GTGTCTGTGT ACATAAATAT ATATACATAT ATATATACAT 300
AAAANCTTTG TACAAA 316

SEQ ID NO:5151
SEQUENCE LENGTH:66
SEQUENCE TYPE:nucleic acid
TOPOLOGY:linear
CLONE:HUMGS06154
SEQUENCE DESCRIPTION:
GATCAGGCAG GAGAAAAACT TTAGATGGGA GTCTTGTCAT CAAAAATAAA TACAGTTGCA 60
TGGAAA 66

SEQ ID NO:5152
SEQUENCE LENGTH:311
SEQUENCE TYPE:nucleic acid
TOPOLOGY:linear
CLONE:HUMGS06155
SEQUENCE DESCRIPTION:
GATCAGGACA AGAGCGANGC ACCAGGGGAG CTCTCTCCCA AAGGCCTTGT CGCCCCAGAC 60
CTAACTCGCT CCCCCTCAAC CTGCCTGAGG AAGAAACTCT CAGGATTGCA CGGATATTTT 120
CTTCTCAGTA CTCCCAGAAA GACTGACGCA GCCTGAGGGG CTGGGGTTGA AGGAGACACC 180
TGCCCANCNN NCGTCAGNTT TGTCCAGCTT GCAGGAGGCA CCAGGTCTGG CTCCTTCAGG 240
GCTGTCACAG TCCTGAAACC ACCACTTGCC TAGGTCACGG ATGCCTNAAG AGACCCGGTN 300
AGGCTAGATN N 311

SEQ ID NO:5153

SEQUENCE LENGTH:111
SEQUENCE TYPE:nucleic acid
TOPOLOGY:linear
CLONE:HUMGS06156
SEQUENCE DESCRIPTION:
GATCTAGGGC TGGCCCCTAG TGAGTGGGCT CGAGGGAGGG TTNCCTGGGA ACCCCAGGAA 60
TTGACCCTGA GTTTTAAATT CGAAAATAAA GTGGGGCTGG GACACACGAA A 111

SEQ ID NO:5154
SEQUENCE LENGTH:274
SEQUENCE TYPE:nucleic acid
TOPOLOGY:linear
CLONE:HUMGS06157
SEQUENCE DESCRIPTION:
GATCTCCTNN TCCAGTGCTG CACACTCCTG TNTTTGGAAC TTTAATAGCG TTGCAACGAA 60
ATCCTATATC CAGTTTCCTG TAATTTANTT GAAGAAAAAT ACATCCAAAT AAAGACTTTA 120
TTATTAACAG ACCAGATAGC ATCAGAAATC ATGTGACTGT TNTGCTTATC AGANTATGTC 180
TNAACTNTTT AGGGCAAAGT TAACACTGAA AGTTCTAGCT TAAGTGTTGA NCCTNTNGTG 240
GGAAAAANAA TTCCACTTGT GAAACNTCAG GCTN 274

SEQ ID NO:5155
SEQUENCE LENGTH:155
SEQUENCE TYPE:nucleic acid
TOPOLOGY:linear
CLONE:HUMGS06158
SEQUENCE DESCRIPTION:
GATCACTCTG GTGCGGGTGT TTGGTTTTGT TTTAAAATAG CTTTGCAGTG AAAGCTTTCA 60
TGACCATACA AATNATCTTT TTTCTTCCTA TTTCCTTGTA GAGGTTTTTT TCCTCCTTGT 120
CTTAAGGTCA TAAAAATATT GTTATGTGGG ACAAA 155

SEQ ID NO:5156
SEQUENCE LENGTH:369
SEQUENCE TYPE:nucleic acid
TOPOLOGY:linear
CLONE:HUMGS06159
SEQUENCE DESCRIPTION:
GATCTGTGGC TTTGCTCCAC CATCCCTAAC CAACCTCTCA TCACAGCTTT NTGTGTGGGC 60
TGAGTGCTGG CCTTAACCCT AGGCGTGGAA GAGAAAATGT GAGGTTGTTT AGATACTCAT 120
CAGGACCTCA CAGGAGCTGA GACTTATCAG CCAGAATNTG TTCTTCGGAC AGTCGTACAC 180
ATCTTACAGA AAACCCTCCT TNGTAGAGTT GGNTTGTGGT ATGTGTTTGA TGCTATAAAG 240
CTCATTTTTA ATGGTGTACA CCTGNTTCTA GGGNCGATTT CGTTTTGGAA GNGGGTAANG 300
NTGCNTTAAC CAGGTAACTN ATCCTTGGGN TTTAAGGTGN AAAAAATNTT CTNGNCAAAG 360
GGAANCCNN 369

SEQ ID NO:5157

SEQUENCE LENGTH:279
SEQUENCE TYPE:nucleic acid
TOPOLOGY:linear
CLONE:HUMGS06160
SEQUENCE DESCRIPTION:
GATCAAAATC AAAAGAACCG TGTAGATATA CTTNATTGTA TAAGTAGAAA ATTACTTAAT 60
TNCATACTAG AAATGGATGG ATGCTGCAAG TTGAAATGGA CTGTCCATTG ACGGTCCTAA 120
TGTGGTAGCA GAAAAANATG GTGTCTTAAG TNCTTAGNGT TTGATGTCAT TAACAGTTTC 180
GTAAAACTCT ACAGTGTAGN AAGTTTTTTC AATACTAAAC TGTGCGTTGT NCATAGTTCT 240
AATGCATTGT NTTNGCCNCC CGGTACTTTC TNTNAANGN 279

SEQ ID NO:5158
SEQUENCE LENGTH:337
SEQUENCE TYPE:nucleic acid
TOPOLOGY:linear
CLONE:HUMGS06161
SEQUENCE DESCRIPTION:
GATCTCAAGT GAGATAGCAG AGCAAGATGC CAAAAGACCA TAAATAGAGT AAGGTTTCTA 60
TAGATGTNAG ACAGATTTGA GAGAGCATTT ACTCTGTCTC CCTGTGGATG AAACTGCTGC 120
TGAAATGGTT CCAATTTTNA GGAATCTGCT TACCCNCTTC ATTATTTGAC AGCTTTCCTT 180
GGTGACCCAA ACCTTGTAGC CTAAGCCATT TGTCTTTTTC TCAGTGGAGG GAGTGTATGG 240
ACCTGGCCCC ATGGCTTTGC ATGTTAGAGA CCTGGCNGAC TAAAGTCNCN GGGTGTTTGT 300
TTGCTCACAT TTGCTGAGTG ACAGNTATGG TGNCCNN 337

SEQ ID NO:5159
SEQUENCE LENGTH:378
SEQUENCE TYPE:nucleic acid
TOPOLOGY:linear
CLONE:HUMGS06163
SEQUENCE DESCRIPTION:
GATCTACAAC CCCTTGGAGC AGTGCTGTNA CAATNACGCC ATCGTGTCCC TNAGCGAGAC 60
CCGCCAATGT GGTCCCCCCT GCACCTTCTG GCCCTGCTTT NAGCTCTGCT GTCTTNATTC 120
CTTTNGCCTC ACAAACGATT TTATTGTGAA GCTGAAGGTT CAGGGTGTGA ATTCCCAGTG 180
CCACTCATCT CCCATCTCCA GTAAATGTGA AAGCAGAAGA CGTTTTCCCT GAGAAGACAT 240
AGAAAGAAAA TCAACTTTTC ACTAAGGCAT CTNAGAAACA TAGGCTAAGG TAATATGTGT 300
ACCAGTAGAG AAGCCTGAGG AATTTACAAA ATGNTGCAGC TCCAAGCCAT TTGTATGGCC 360
CATGTGGGAG ACTGNTGN 378

SEQ ID NO:5160
SEQUENCE LENGTH:135
SEQUENCE TYPE:nucleic acid
TOPOLOGY:linear
CLONE:HUMGS06164
SEQUENCE DESCRIPTION:
GATCAGCCCC ACCCTGGCCT AGACCAGCAG ACAGAGCCAG GAGAGGCTCA GCTGCATTCC 60

GCAGCCCCCA CCCCCAAGGT TCTCCAACAT CACAGCCCAG CCCACCCACT GGGTAATAAA 120
AGTGGTTTGT GGAAA 135

SEQ ID NO:5161
SEQUENCE LENGTH:350
SEQUENCE TYPE:nucleic acid
TOPOLOGY:linear
CLONE:HUMGS06165
SEQUENCE DESCRIPTION:
GATCTCGTAA GATTAAATGT AGCATTCTAT ACTCCCTACA TGCACAGTAN GATTTGTTTA 60
ATGAATGGAA GGAAAGACTA AATACAATGT CAAATGTNAC TATTAATTGG ACAAGAAAAT 120
CCAAGTAGCA AATGACCATT ATGTGTAAGA CTTACTCTAA AAAGACCNTT TTAAATAGCT 180
CATATATTAA TNTNGTTTAA AACCTGCTAT TTTCCAAAAC TGCAAAACTA GGGCTATAAA 240
AATCTAAGTT AAAAAATCAG CTNATCCTCA TTGGTATAGT GGTNTGGNNA AAAAAATCCA 300
GTTTTAAACC NGNNTNNATT GANAGGNTGT CTCCATGCCT NCCATTGGGN 350

SEQ ID NO:5162
SEQUENCE LENGTH:57
SEQUENCE TYPE:nucleic acid
TOPOLOGY:linear
CLONE:HUMGS06166
SEQUENCE DESCRIPTION:
GATCACTTTT ATGTAAAAAT TATAATTTAA TTAATAAAAA ATTTCTTAAG AAGTAAA 57

SEQ ID NO:5163
SEQUENCE LENGTH:203
SEQUENCE TYPE:nucleic acid
TOPOLOGY:linear
CLONE:HUMGS06167
SEQUENCE DESCRIPTION:
GATCAGGCCT TGGCCTCTNA GCCCTGCCTG CTCTGGGCCA TGCAGAGGAA GGACAGAGNG 60
TGGGCGCAGG GCACCAACTC AGGGACATCC CCTCTCCTGG GCGACGTCAG TGGACCTTCC 120
TGCACCCCCA GCCTGGAATG TAAATNAGCT GTGTGGTGCC CGCGTNGCTG GAAGGAAATA 180
GACCCTTTTN TAGCTCCCTG AAA 203

SEQ ID NO:5164
SEQUENCE LENGTH:274
SEQUENCE TYPE:nucleic acid
TOPOLOGY:linear
CLONE:HUMGS06168
SEQUENCE DESCRIPTION:
GATCCTCTGT GATACCAAAA TCCATGGATG CTAAAGTCCC TTTTTAAAAA TGGCATAGTA 60
TTTGCATATA ACCCAAACAC ACCCTCCTGT GTATTTTAAA TCATCTGTAG ATTACTTATA 120
ATACATAATG TAATGTAAAT GCTATAAAAN TAGTTATTAG ACTGTATTTA GGGAATAATG 180
ACAAGANGAA AAAGTCTGTA CATGTTCAGT ACAGAGGCAA CCTTTGTAGG TCTAAATANN 240

TTTTTAATAC AGNANTNNGG ATGTNAGNNN NGGN 274

SEQ ID NO:5165
SEQUENCE LENGTH:343
SEQUENCE TYPE:nucleic acid
TOPOLOGY:linear
CLONE:HUMGS06169
SEQUENCE DESCRIPTION:
GATCTCTTTT TATTTAAATG TGAATTTCAA CTTTTGACAA TCAAAGAAAA GACTTTTGTT 60
GAAATAGCTT TACTGTTTCT CAAGTGTTTT GGAGAAAAAA ATCAACCCTG CAATCACTTT 120
TTGGAATTGT CTTGATTTTT CGGCAGTTCA AGCTATATCG AATATAGTTC TGTGTAGAGA 180
ATGTCACTGT AGTTTTGAGT GTATACATGT GTGGGTGCTG ATAATNGTGT ATGTCCTTTG 240
GGGGTGGAAA NGGATAACAA TTCAAGCTGA GAAANGTATT CTCAAAGATG CATTTTTATA 300
ANTNTNATTA ANCAATNTTG TNANAGNNNG ANGNGTGGGN NTN 343

SEQ ID NO:5166
SEQUENCE LENGTH:124
SEQUENCE TYPE:nucleic acid
TOPOLOGY:linear
CLONE:HUMGS06170
SEQUENCE DESCRIPTION:
GATCATACGA GGCATGTAAT ACCAAGAATT GTTACTTTAC AATGTTCCCT TAAGCAAAAT 60
TGAATTTCCT TTGAACTTTT AGTNATGCAC AGACTNATAA TAAACCCTCT AAANCCTGCC 120
CAAA 124

SEQ ID NO:5167
SEQUENCE LENGTH:367
SEQUENCE TYPE:nucleic acid
TOPOLOGY:linear
CLONE:HUMGS06171
SEQUENCE DESCRIPTION:
GATCTTGGAC CCAGTCTGGC CCGTGCCCAG CATGGGGCTG CTTCTCCTCC ACAGCTCCTT 60
CCCAGAGGGC TGGGGCCTCA GCCTCCCTGC CACGTCCACT CTGGGCTGGC CTGGAACATA 120
CTACTATGTT GACAGTAACA ATAAAGCTCC TCAGGGACCA CCGTACAGGC CCGCAGCCCC 180
ACTGNCTGCT GTTGGAAGAA AGCTGTGGAA AGAAAGCAGG GGGNCCCTGC CCCAGGCCAA 240
GGCCCTGCCC TTGCCACACA CGGTACACAN ATGCAGCCTC TCTNTCTCTN TGTGTCCACT 300
TGTGAAAATA ANCTGCAGGN CTGGNTNGGA CAACAAAGGN AGNNGNACAG TGTCTNGGNN 360
GGCANNN 367

SEQ ID NO:5168
SEQUENCE LENGTH:207
SEQUENCE TYPE:nucleic acid
TOPOLOGY:linear
CLONE:HUMGS06172
SEQUENCE DESCRIPTION:

GATCAGTTCA GTATATTGCA CTAATTATTT TAGGTATTTT CATTATATGA AAGCTACCAT 60
GTGTCAGAGA TGATTTAATC TATTTAAGTG TTGGACTGCT AGGAGAACTT GTACATTTAT 120
GATAATGCAG AATTAGGAAA ACGGTTCACC AGTGTTTAGT TTTATATTGA GGTGCTCAGG 180
TTGGTATAAA GTGGTATAAA AAGCAAA 207

SEQ ID NO:5169
SEQUENCE LENGTH:116
SEQUENCE TYPE:nucleic acid
TOPOLOGY:linear
CLONE:HUMGS06173
SEQUENCE DESCRIPTION:
GATCATGCCC TTTGTAGTAN CACAAATGGN GCTGGAGACC ATTATATTAA GCAAACTAAC 60
ACAGTNCCAG ANAACCAAAT ACAGTATGTT CTCACTTATA AATGGGAACT AAAGNN 116

SEQ ID NO:5170
SEQUENCE LENGTH:92
SEQUENCE TYPE:nucleic acid
TOPOLOGY:linear
CLONE:HUMGS06174
SEQUENCE DESCRIPTION:
GATCTGCTAG GGATTGTCAA AATAATCTCC TTGAGGCATC TTTATTTTTA AAATGAGATT 60
AAAGTATGTG ATTTGCTTGT TATGTGGCTA AA 92

SEQ ID NO:5171
SEQUENCE LENGTH:120
SEQUENCE TYPE:nucleic acid
TOPOLOGY:linear
CLONE:HUMGS06175
SEQUENCE DESCRIPTION:
GATCANCAGA AAAGGCAAAT TTCACTCGNC TAAACACTTT CAATTTTCAG TTNTAATTTT 60
ATNTTCTATA TACCCAGTCA TAAAGTATAA GCATCAGTTG TCATTAAAAG TTTTCAGAAA 120

SEQ ID NO:5172
SEQUENCE LENGTH:102
SEQUENCE TYPE:nucleic acid
TOPOLOGY:linear
CLONE:HUMGS06176
SEQUENCE DESCRIPTION:
GATCTGTAAT GAAAAGAATC TGNTACTGCA AGTAAAACCT ACTCCCCAAA AATGTGTGGC 60
TTTGGGTCTG CATTAAACGC TGTAGTCCNN GTTCATGCCA AA 102

SEQ ID NO:5173
SEQUENCE LENGTH:171
SEQUENCE TYPE:nucleic acid
TOPOLOGY:linear

CLONE:HUMGS06177
SEQUENCE DESCRIPTION:
GATCACTTNT NGTCTGATTC CAGCCTGCTT GCAACCCTGG GNTCCTCTTG TTCCCTGCTG 60
NCCTGCCCCT TGGGAAGGGG CAGTGATGGC TTTGAGGGGA AGGAGGAGAC CCTCTTTCTC 120
CCATGCTGCA CTTACTCCTT TTGCTAATAA AAGNGTNTNT AGATTGTCAA A 171

SEQ ID NO:5174
SEQUENCE LENGTH:188
SEQUENCE TYPE:nucleic acid
TOPOLOGY:linear
CLONE:HUMGS06179
SEQUENCE DESCRIPTION:
GATCTAAACT GTATTGNCCA ATTTAAATAA AAAATNNAAT ATAGATTCAG AAAGGTTCAT 60
ATTTTTCTAA TGACTTCATT CTATATTATN TTGTTAGGTT GCATAAAGAA GCAAGGAATT 120
GTACTTGTAT TAAAAGATGA AGAAAGCTAT TAGGTATATT TGTACATGNC TGCANNTGTG 180
TCTATNNN 188

SEQ ID NO:5175
SEQUENCE LENGTH:69
SEQUENCE TYPE:nucleic acid
TOPOLOGY:linear
CLONE:HUMGS06180
SEQUENCE DESCRIPTION:
GATCTTGAGA GGTGGAGGTT GCAGTGAGCC AAGATTGTGC CACTGNCTTC CAGCCTGGGA 60
GACAGCAAA 69

SEQ ID NO:5176
SEQUENCE LENGTH:134
SEQUENCE TYPE:nucleic acid
TOPOLOGY:linear
CLONE:HUMGS06181
SEQUENCE DESCRIPTION:
GATCTGTTAA TATGTAACAT ATTAATGGGT AACTTGCTGT GTAAAATTAT AAGCCATATT 60
TTAAAAGGTT TTAAAAATAC TTATTGTGCT CCATTTGTGA TATAATTNCT AACATTTCTG 120
CTCTGTGANN NNNN 134

SEQ ID NO:5177
SEQUENCE LENGTH:59
SEQUENCE TYPE:nucleic acid
TOPOLOGY:linear
CLONE:HUMGS06182
SEQUENCE DESCRIPTION:
GATCTTNGAG ATTGTTCCAC ATCAGTACAT AAAGTACATA AAGATTGTCA CCCCACAAA 59

SEQ ID NO:5178

SEQUENCE LENGTH:193
SEQUENCE TYPE:nucleic acid
TOPOLOGY:linear
CLONE:HUMGS06183
SEQUENCE DESCRIPTION:
GATCTGTCTA CTTNNCTTAT CCTGATTCAA ATGGAATGCC AGTATGGAAC TCCTAGGATT 60
TGTCACGAAT GGGAAGCCAA GTGCCATCTT CAAAATTTCA GGTCTTAAAC NTGGAGAAGG 120
AAGCCAACAT CCTTTTGGAG CCATGAATNT TGTCCGAACT CCANNNTGTT GCTCAGANTG 180
GANTTTCAGT GGN 193

SEQ ID NO:5179
SEQUENCE LENGTH:109
SEQUENCE TYPE:nucleic acid
TOPOLOGY:linear
CLONE:HUMGS06184
SEQUENCE DESCRIPTION:
GATCATTATT TGGNAATATG TCCTATGGAA AGAATAAAAG CATGTNCTTC ACAGCTAGCA 60
TGTTCACAGA TTTGAAAGAA GTTTCATTAA AAGCACCATT GCTTTCAAA 109

SEQ ID NO:5180
SEQUENCE LENGTH:297
SEQUENCE TYPE:nucleic acid
TOPOLOGY:linear
CLONE:HUMGS06185
SEQUENCE DESCRIPTION:
GATCTTAATT TAAATAGGTG CTTTAATTTT ACAGCTTTCA AAAGTAGTTA CTGTAAATTT 60
TACTCCTGTT AATGCTGGAT TGGTTTAAAG GATTTATAAG GGCTGTGTTT NCTCTNTACA 120
AGGCAAGATG CCATACAGNG TTCANTAATA GAAGGTCTNG ATACAACCGA CATTTTAAAT 180
ATTCTNTATA CATGNGGACT GCGTCTTTTT CACTGACCCT ATGANTTCAC TGTGGAGTAG 240
GGTGAACTGG NCTGCANATT AGTTTATTTG TTGCCATAAT CACATTNNNG AAAAGAN 297

SEQ ID NO:5181
SEQUENCE LENGTH:301
SEQUENCE TYPE:nucleic acid
TOPOLOGY:linear
CLONE:HUMGS06186
SEQUENCE DESCRIPTION:
GATCTNATTG CTTATAAACT TTGGTGACGG TAGTGTGTAA GGCCGTATTT NNAGCATCTN 60
ACAGGTGTTT ACAAAAAAGT GGTTGTCGCA CTGGGAAGTG GAGTGATGGC CTCGTCTCCA 120
GTGCTCCTCT GGGCTCTTGA GTTGCTGCTT GANTTGCCGT GTAGACATTT NCTTGGAGAG 180
TCCACTTGTN ATTTGACGGA GGTAGGTTTC AACCCAGAGT TAATGTCAAG CATGCTAATT 240
TANCTAGTCA CTCACAGATG ACTTTCCTTT AATAAAAGTC CCTTTTCCCT AAAANAAAAA 300
A 301

SEQ ID NO:5182

SEQUENCE LENGTH:293
SEQUENCE TYPE:nucleic acid
TOPOLOGY:linear
CLONE:HUMGS06187
SEQUENCE DESCRIPTION:
GATCTTTGGG CAAGTCACTT AACCTCTCTT TNCCTCAATT TCCTCATCTT GAAATGAGGA 60
TAATAATACC TGCTGTACCT ACCTCACAGG GCTGTTGTGA GGATTAAATG AGNTGGCATG 120
TGAAAGCACT TTGAAAATTG TAAAGCGCTA TGTAAATGTA AGGTATTATA GAANCATCTT 180
TAACATATAG TTTCATACCA TTCATTTTTT ACCAAAGAAA GGGAAAGTNT GCTNGTANGC 240
TGGTTGAAAA AGTTANNCTT GGTATAANNT TGNGTTTGGT TGANGNANAA GGN 293

SEQ ID NO:5183
SEQUENCE LENGTH:149
SEQUENCE TYPE:nucleic acid
TOPOLOGY:linear
CLONE:HUMGS06188
SEQUENCE DESCRIPTION:
GATCACAAAA GTATATAAAA AGTCTACTAG AGCAACATTT TCCCATATTT TTNGGTTCTC 60
AAAGCAACTT ATGTATGNTT TAGAGANGNC AAAAGAGGAA GCCTAGGGGG AAAAGAAAGA 120
NAATTAGAAT TTGCAAATNC TAGAAAANN 149

SEQ ID NO:5184
SEQUENCE LENGTH:157
SEQUENCE TYPE:nucleic acid
TOPOLOGY:linear
CLONE:HUMGS06189
SEQUENCE DESCRIPTION:
GATCGAAAAG AAATTTNTTT ATACTTGATG CCTTAAGATN CCCAAAGCTG CCCAAAGCTC 60
TGAAAGACTT TAAGATAGGC AGTAATGCTT ACTACAATAC TACTGANTTT TTNTAGAGTT 120
AACATTTGAT AATAAAACTT GCCTGTTTAA TCTCAAA 157

SEQ ID NO:5185
SEQUENCE LENGTH:62
SEQUENCE TYPE:nucleic acid
TOPOLOGY:linear
CLONE:HUMGS06190
SEQUENCE DESCRIPTION:
GATCTTGCCT GGAAGGTGTT TTAAGTTTTN TAATAAACAA GATGATGTCT GAAAATNTGA 60
AA 62

SEQ ID NO:5186
SEQUENCE LENGTH:145
SEQUENCE TYPE:nucleic acid
TOPOLOGY:linear
CLONE:HUMGS06191

SEQUENCE DESCRIPTION:
GATCTAGAGC AGCATGGAGC TGTTGGTAGA ATATTAGTTT TTAACCATAC ATTGTCCCAA 60
AAGTGTCTNT GCATTGTGCA AAANGTAAAC TTAGGAACAT TTNGGTATTA AAAGGATTAT 120
TTTAATTGNN AAAAGCCNGA NGCGN 145

SEQ ID NO:5187
SEQUENCE LENGTH:103
SEQUENCE TYPE:nucleic acid
TOPOLOGY:linear
CLONE:HUMGS06192
SEQUENCE DESCRIPTION:
GATCAACAGC TCCGCCTACC GCAAAGCATT TGANAGCAGA CTAGCCANCA GTNGTGCTCT 60
GAGAGTGAAC GNGCACCTCC AGGTNGAGGG CCACAGCAAC GTN 103

SEQ ID NO:5188
SEQUENCE LENGTH:248
SEQUENCE TYPE:nucleic acid
TOPOLOGY:linear
CLONE:HUMGS06195
SEQUENCE DESCRIPTION:
GATCTGATAG AGGGGGAGGC AGAAGCAGAT ACTAGAAACA AACAAAACTT TGGACCAAAA 60
TCCCAGTTCA AAGAAACAAA AAAAAGAGTG GAAACTATTC TATCATAACT ACCCAAGGAC 120
TACTAAAAGG ANAAATTGTG TTACCTTTTT TAAATTCCCT GTTAAGTCCC TTCCGTAATT 180
TTNATGTTCT TGTGAGGANA AAAGTAAANC ATGTTTANTT TTAANNAAGG NGANAGNGGG 240
NGTNTAGN 248

SEQ ID NO:5189
SEQUENCE LENGTH:354
SEQUENCE TYPE:nucleic acid
TOPOLOGY:linear
CLONE:HUMGS06196
SEQUENCE DESCRIPTION:
GATCTCTTAT ACAGACGCAC CAAAGCCCTC ATTGACTATG AGAACTCAAA CAAAGCTCTG 60
GATAAGGCCC GGTTAAAGAG CAAAGACGTC AAGTTGGCTG AGGCACACCA GCAGGAGTGC 120
TGCCAGAAAT TTGAACAACT TTCCGAATCT GCAAAAGAAG AACTGATAAA TTTCAAACGG 180
AAGAGAGTGG CAGCATTTAG AAAGAATCTA ATTGAAATGT CTGAACTGGA AATAAAACAT 240
GCCAGGNACA ATGTCTCCCT TTTGCAGNGC TGGTNTTGAC TTGTTCAAGA GTAACTGGTT 300
ATGCCTTCAC TCNGTAGGAA NNGGAGTTGA ATTGTTGTAA GGAAGCCNAN GTGN 354

SEQ ID NO:5190
SEQUENCE LENGTH:77
SEQUENCE TYPE:nucleic acid
TOPOLOGY:linear
CLONE:HUMGS06197
SEQUENCE DESCRIPTION:

GATCTCTATT TTAAAACTAG CTTTTTAAGC AGCTGTATGA AATAAATNCT GAGTGAGCCC 60
CAGCCCGCCC CTGCAAA 77

SEQ ID NO:5191
SEQUENCE LENGTH:257
SEQUENCE TYPE:nucleic acid
TOPOLOGY:linear
CLONE:HUMGS06198
SEQUENCE DESCRIPTION:
GATCTAGCTT CANAGGAAGT CTACACCCCA TTCCCTTCTG CTTACAATGT ACCCATGATA 60
TGTGTTTAGC ACAGTAATAC TGTAACAGGA CATCACATGG AAAAATCAAA GCAGCTGGCT 120
CACTGTATTT AACTGAAAAG AATGCCTACA GATTGGATAT TTNGTAGGGG NAATTAAGGC 180
ACTTTNNTAA CAAACTTCAT TNTGTGAAAC TNGGTTGAAT ATTAACATAC AATTNNGNCG 240
GGNNTATTAA TGCCATN 257

SEQ ID NO:5192
SEQUENCE LENGTH:185
SEQUENCE TYPE:nucleic acid
TOPOLOGY:linear
CLONE:HUMGS06199
SEQUENCE DESCRIPTION:
GATCCCAGGC TGCCCTGGAC TTAGACCAGT GTCTGAGGTG GTAACAGCGG CCGCACAGGG 60
TTGGCCTAGA CCTNGGATTT GTGGGGAAAG CTGCTGGTGT GACCANCTGA GCACCCAGCC 120
AGGAGACCTG CAGCCCTNCG NCTTCCAGAA GCAGGTCCCA AATAAAGNCA GTGCCNACCT 180
NCAAA 185

SEQ ID NO:5193
SEQUENCE LENGTH:60
SEQUENCE TYPE:nucleic acid
TOPOLOGY:linear
CLONE:HUMGS06200
SEQUENCE DESCRIPTION:
GATCTGTATA TAACTTTAAT TTTAAGGACC ATAATCAACT TTGTAATATT CTNATATAAA 60

SEQ ID NO:5194
SEQUENCE LENGTH:286
SEQUENCE TYPE:nucleic acid
TOPOLOGY:linear
CLONE:HUMGS06201
SEQUENCE DESCRIPTION:
GATCACCCTC TACGGGAGAA CCAAGGAGCT GACTTCGGAA CTAAAGGAGA ACTTCATCCG 60
CTTCTCCAAA TCTNTGGGCC TCCCTGAAAA CCACATCGTC TTCCCTGTCC CAATCGACCA 120
GTGTATCGAC GGCTGAGTGC ACAGGTGCCG CCAGCTGCCG NACCAGCCCG AACACCATTG 180
AGGGAGCTGG GAGACCCTCC CCACAGTGCC ACCCATGCAG CTGCTCCCCA GGNCACCCNG 240
NTGATGGAGC CCCACCTTGT NTGCTAAATA AACATNTGCC CTCAAA 286

SEQ ID NO:5195
SEQUENCE LENGTH:138
SEQUENCE TYPE:nucleic acid
TOPOLOGY:linear
CLONE:HUMGS06202
SEQUENCE DESCRIPTION:
GATCTCTTGT TCTCTCCTGA GTGTCTTNCT ACTNTGCTGA GNGTCTTTCT GCTGTCCTTA 60
TCCTGTTCTC TTATCCTNAT CCCCTCCAGT CTCTGCCTAA TTTTNAGTGT TTAATAACAA 120
CCGNATGTCT AGTAAATN 138

SEQ ID NO:5196
SEQUENCE LENGTH:96
SEQUENCE TYPE:nucleic acid
TOPOLOGY:linear
CLONE:HUMGS06203
SEQUENCE DESCRIPTION:
GATCAAGAAC TTGGAACCGC AGAAAACGAA ATCCCATAGT AGCACAAAGC TTGGCTGTTC 60
AGTGAATAAC ATTTAAATAA TCGTAAANTA CAGAAA 96

SEQ ID NO:5197
SEQUENCE LENGTH:222
SEQUENCE TYPE:nucleic acid
TOPOLOGY:linear
CLONE:HUMGS06204
SEQUENCE DESCRIPTION:
GATCTTGCAG CTTTATTTGA GTATTTGTTC TTTTGTGTAG TTTCCATCTT TTAAAATATT 60
TAAAATATTT TCAAGATAAA GTATTATCTT CTCTGCAAAA ATTCCTGGAG TAATTTNCTC 120
TCATAATATT TGAGGTCAGT GGTTCTCAGT TGTATTAGTG GGGTAACTAC ATCAAAGTAA 180
ATAANGTCTT ATTTTTNANA TGCAAATTTT AGACCATAGA AA 222

SEQ ID NO:5198
SEQUENCE LENGTH:53
SEQUENCE TYPE:nucleic acid
TOPOLOGY:linear
CLONE:HUMGS06205
SEQUENCE DESCRIPTION:
GATCAAGTGA ACCATCCCTA GTCTTCCCTC AATAAATAAC TTTTAACTCC AAA 53

SEQ ID NO:5199
SEQUENCE LENGTH:239
SEQUENCE TYPE:nucleic acid
TOPOLOGY:linear
CLONE:HUMGS06206
SEQUENCE DESCRIPTION:

GATCANAGTG AACCCTCAGT TTGTACAAAA GAGTATGGGC TCACAAGANG ATGATTCAGG 60
AAACAAACCA TCCAGTTATT CTTGAAACTA ACATCCATCC TGAGCTAAAC NAGAGAAACT 120
ACCATCTTGG CCAGTGNCAN GTGTTCGGAG GGCAGCAGAG NGGACCAAGC CTGTGTCACC 180
TGGNGACTAA GAANTTAAGT TTTGTNTTGA CATATTCAGT CCTGTGTGCT TNCAGNAAN 239

SEQ ID NO:5200
SEQUENCE LENGTH:233
SEQUENCE TYPE:nucleic acid
TOPOLOGY:linear
CLONE:HUMGS06207
SEQUENCE DESCRIPTION:
GATCGAATAC GTGGATGNGA CGGGCCGGAA ACTCACACCC AAGGAGGCTT TCCGGCAGCT 60
GTCGCACCGN TTCCATGGCA AGGGCTCAGG CAAGATGAAG ACAGAGCGGC NGATGAAGAN 120
GCTGGACGAG GAGGCGCTCC TNAAGAAGAT GAGCTCCAGC GACACGCCCC TNGGCACCGT 180
GGCCCTGCTC CAGGNGAAGC AGAANGCTCA GANNGACCCC NTACATCGTG CTN 233

SEQ ID NO:5201
SEQUENCE LENGTH:163
SEQUENCE TYPE:nucleic acid
TOPOLOGY:linear
CLONE:HUMGS06208
SEQUENCE DESCRIPTION:
GATCAAAAAG GGGTTGATTA CAAGGGCAGG AAGGAATTGT TAGTGTGATT CTACAGTTCT 60
GCATCTTGNT TATGGTATGA CTGTGCGTTT GTCAAACCTT AACAGGATGA ATTTTGCTGA 120
ATGTAAAATC ATACTTCAAT AAACATGACT TTAAAANCTT AAA 163

SEQ ID NO:5202
SEQUENCE LENGTH:319
SEQUENCE TYPE:nucleic acid
TOPOLOGY:linear
CLONE:HUMGS06209
SEQUENCE DESCRIPTION:
GATCAGTTTA TGTGTAATAT TCTAGTGCTT TAATGACTCT TTTTTTCTTT GGAGGGGAGGG 60
TAACATTATT TGGACAGATG CAGAAGGAAC TGTTAGTGAG TCAAGACAAA CACATCTGAA 120
ATAAAGGAAC TGTGTATTAA CATGTTAACA ATTCATAACT GCACTTTTTA TGACATTTTG 180
AAAATCTATT TATAGGTACA GANCAATGGG TTTTGTTAAN CTGTATCACA TTTATNCTTG 240
CAGAAATTTA TTNCATTGTT ATTAGTAGGA ATTTTNTTGG TTCAATAAAA TTGGCAAAAN 300
CTGGGAAAAN NAAANNTTN 319

SEQ ID NO:5203
SEQUENCE LENGTH:82
SEQUENCE TYPE:nucleic acid
TOPOLOGY:linear
CLONE:HUMGS06210
SEQUENCE DESCRIPTION:

GATCTGTCTA GTTCCCATTC TCTGTTCAAC CTCAGTGNTT CAAAAGTTCC TAATAAATAA 60
ACTCATTTGA GTTGAACCTA AA 82

SEQ ID NO:5204
SEQUENCE LENGTH:145
SEQUENCE TYPE:nucleic acid
TOPOLOGY:linear
CLONE:HUMGS06211
SEQUENCE DESCRIPTION:
GATCAAGAAA GAAGCTATCT GGGANCANTG TAGCANTTAC GTCAGACCAG GACACTTCCT 60
GTTTACAGGA GACTATAAAA CCTTTGTCCT ATCATCACTT GATGTGGACG NCATTTTAGG 120
CCTCAGCCCG CCTGCACCCA GNNNN 145

SEQ ID NO:5205
SEQUENCE LENGTH:295
SEQUENCE TYPE:nucleic acid
TOPOLOGY:linear
CLONE:HUMGS06213
SEQUENCE DESCRIPTION:
GATCAGAAGC ACCTCTTCTT TTATAGAAGT TGATGGATGG TTCATTCCAT TCTGCTACCA 60
AGAAGTGCAT GCTGCTGCAG CGACACCTCA TTGCAACCTA TTATGAGAGA GTAAAACACA 120
CACTCAGTGA CATTTCAGCC TGCTCTCAAG NCCCAATTTT TATTCCATCA TTAAAATAAT 180
CCAGTAAACT TNCAAATTTG GAAACCANAA ACTAAGGCAT ACCTGGCTTA GATTCTTTCA 240
GAATTTTCTG NTCTTTTGTA CGTAAATTNA AAAATAATTT GTGGCAGGGN TTAAA 295

SEQ ID NO:5206
SEQUENCE LENGTH:87
SEQUENCE TYPE:nucleic acid
TOPOLOGY:linear
CLONE:HUMGS06215
SEQUENCE DESCRIPTION:
GATCTTTAAA ATNAAGTGTA CGCTTATAAT ACAAGTAATG CTTGTGTATT TCTCACTTTA 60
GGAAATAAAA TATTTGCCAA TTTGAAA 87

SEQ ID NO:5207
SEQUENCE LENGTH:165
SEQUENCE TYPE:nucleic acid
TOPOLOGY:linear
CLONE:HUMGS06216
SEQUENCE DESCRIPTION:
GATCCTCCCA TCTCAGCCTC TCGAGTAGCT NGGACCACAG TTGTGTGCCA CCACACTTGG 60
GCTAACTTTT TAATTTNTTT GCGGAGACGG TATTGCTATG TTGCCAAGGN TGNTTACATG 120
CCAGTACAAT TTATAATANA CACTCATTTT NCCTNCCGTC TGAAA 165

SEQ ID NO:5208

SEQUENCE LENGTH:116
SEQUENCE TYPE:nucleic acid
TOPOLOGY:linear
CLONE:HUMGS06217
SEQUENCE DESCRIPTION:
GATCTTCTTC AGAAGCTTCA AGTCTCANCT GCATTAAAGC TCTCCATTGA ACCCGNTCAC 60
AAAAGTACAA GCTTAAAGGG GCTAAAATCC ATTAATACAT NCCATNTTAT ANCCCN 116

SEQ ID NO:5209
SEQUENCE LENGTH:117
SEQUENCE TYPE:nucleic acid
TOPOLOGY:linear
CLONE:HUMGS06218
SEQUENCE DESCRIPTION:
GATCCCGTGN ACCGAGTACA CGACCCAGGT ATGTACCAAG TAGGCACCCT TGGGCGCACC 60
CACTGGGGCC AAGGGTCGGG GGAGTGTTGG GAGCCTCCTN CCCACCNNAC CTNCCTN 117

SEQ ID NO:5210
SEQUENCE LENGTH:181
SEQUENCE TYPE:nucleic acid
TOPOLOGY:linear
CLONE:HUMGS06219
SEQUENCE DESCRIPTION:
GATCAGCCAC TTGCTGACCC TGGTTCTTAA GGACACATGA CATTAGTCCA ATCTTTCAAA 60
ATCTTGTCTT AGGGCTTGTG AGGAATCAGA ACTAACCCAG GACTCAGTCC TGCTTCTTTT 120
GCCTCGAGTG ATTTTCCTCT GTNTTTCACT AAATAAGCAA ATGAAAACTC TCTCCATTAA 180
A 181

SEQ ID NO:5211
SEQUENCE LENGTH:344
SEQUENCE TYPE:nucleic acid
TOPOLOGY:linear
CLONE:HUMGS06220
SEQUENCE DESCRIPTION:
GATCANAACC AGTTTGTAGA TTTCTTTGTT CCTTCTCCAT TCCCACTGCT TCACTTGCCT 60
AGTCTTGAAG NAAAAAAACA AAAAACAAAA AAAACCTTGT TCCTTTATAG GTTCCTGGTA 120
GAATCAGTAG AGATGATTTC AGCTCATTGA CATTTTTTTA AGCTATATCC CCTTGTCATT 180
CCATTGAGAA AGCTGACAAC TGGGATAGGG AGGGGATTCG ATAATAGATG GGGTCAAATT 240
CTGTGTGAAT GTGAACTTGC CTAGTAAGCA CTTTGGTCTG TGGTTCACTN CTGCGGTTAG 300
NGGAAATCTA TCTCCCCTAT CTTNGGGTCC TTGAACTACA GCNN 344

SEQ ID NO:5212
SEQUENCE LENGTH:139
SEQUENCE TYPE:nucleic acid
TOPOLOGY:linear

CLONE:HUMGS06221
SEQUENCE DESCRIPTION:
GATCACATAA CTAGGAAGTG GCAGAACTGA TTCTCCAGCC CTGGTAGCAT TTGCTCAGAG 60
CCTACGCTTG GTCCAGAACA TCAAACTCCA AACCCTGGGG ACAAACGACA TGAAATAAAT 120
GTATTTTAAA ACATCTAAA 139

SEQ ID NO:5213
SEQUENCE LENGTH:179
SEQUENCE TYPE:nucleic acid
TOPOLOGY:linear
CLONE:HUMGS06222
SEQUENCE DESCRIPTION:
GATCGTAAAC CATTATCCTT TAAAGGTNTA TTTGAAGATG CTGTTAAAGT ACAGAATTTT 60
GTGTACAGGT AGATTTTNCC GTCCCTCATT AATAGTGCCT TCTTAATTAA TACAGACTGG 120
TGTTAGCTAT AACAAAACTC CAGTANNGGG GGGTGNATCC CAAGTNCTTT GTGGCNANN 179

SEQ ID NO:5214
SEQUENCE LENGTH:129
SEQUENCE TYPE:nucleic acid
TOPOLOGY:linear
CLONE:HUMGS06224
SEQUENCE DESCRIPTION:
GATCAAAATG TTCTGGAATT AGATGTGATG TTTGCACAAC TCTGTGAATG TACTAAGAAC 60
CACTGAATTG TATATTTTTA AAGCATGGTA TGTGAATAAT ACCTCAATAA AGCTGTTATA 120
AAAAATAAA 129

SEQ ID NO:5215
SEQUENCE LENGTH:131
SEQUENCE TYPE:nucleic acid
TOPOLOGY:linear
CLONE:HUMGS06225
SEQUENCE DESCRIPTION:
GATCTTCAAG CATAGNNTTG CATATTTTAA CATCTGAAAT CAGAAAGTGT CTTNNCTGGG 60
AGTNTGGTTT TAGAAACATG AAATAGTACT GTATGTTAAA ATCGATGGCA GTGTTGCCTC 120
GATGATATNT N 131

SEQ ID NO:5216
SEQUENCE LENGTH:190
SEQUENCE TYPE:nucleic acid
TOPOLOGY:linear
CLONE:HUMGS06226
SEQUENCE DESCRIPTION:
GATCTTTGAG CGGGACCTGA AGAAGAAGGG GCCCGAGCCG GTGCCACTGG AGTTTATCCC 60
AGCCCAGGGC CTGCTGGGAC GGCCGGGTGA ACTCTGTGCC CAGCACTTCA CGCTCAGCTG 120
ACCCTGGCCC ACCTGTNAAT AAATCTCAGC TGACCCCAGC CCACCTGTNA ATAAATNTTT 180

TTGCAGGAAA 190

SEQ ID NO:5217
SEQUENCE LENGTH:323
SEQUENCE TYPE:nucleic acid
TOPOLOGY:linear
CLONE:HUMGS06227
SEQUENCE DESCRIPTION:
GATCCCTGGT CTTCACACTG TAGCAGCCAA AAGGAACAAG CTGAAANTGG ATACCTAATG 60
TAAGTGGAGT CTTCTAAGTG TTTCCTGGAA GAAGTCGGGT CCCAGGCTCN AGTTGCTGGT 120
TCTNACATGC CACCATCCAC AGTCCTTCAG CAGATTNATT CAGTGGCTAA TGCTGATATC 180
ATAAATGCGG CAAAGAAGTT TNTTTCTGGC CAGANGTCAN TGGCAGCAAG TGGAAATTTG 240
GGNCATNCAC CTTTTTGTTG ATGAGTTGTA ATACTGNNGC GCNCATTACC AGGTGAGAGC 300
TGGANCGTNT TTTCAGNCCN TGN 323

SEQ ID NO:5218
SEQUENCE LENGTH:225
SEQUENCE TYPE:nucleic acid
TOPOLOGY:linear
CLONE:HUMGS06228
SEQUENCE DESCRIPTION:
GATCAAAGTT CAAATTAATT TTAACTTAGC TAATNAACTC ATCACCAGGA CAGTTGGAGG 60
GGGTAGGCCG AGGTTAAATG GTCCACGTTT CAAAAATGTT AATGGCTAAT CCATAATTAA 120
AGAAGGTTTA ACTGTTACTG AAGTTTACAA GTTTTATTGT CATGNACATG AANTACAAAC 180
ACGATGGCTT CGAAATGTCT TTCAATAANT GTTTCTGCAT TTAAA 225

SEQ ID NO:5219
SEQUENCE LENGTH:317
SEQUENCE TYPE:nucleic acid
TOPOLOGY:linear
CLONE:HUMGS06229
SEQUENCE DESCRIPTION:
GATCTCATTC TCACCTCAGA AACCATTTAC AACCCAGATT ATNATAGTAA TTTGCACCAG 60
ACTTTCCTTA GACTGTTAAG TAAAAATGGA CGTGTACTTT TGGCCAGCAA AGCACATTAT 120
TTTGGTGTAG GTGGAGGTGT TCATCTCTTT CAGAAGTTTG TAGAAGAAAG AGATGTTTTT 180
AAGACCAGAA TACTCAAAAT AATTGATGAA GGATTGNNGA GGTTCATAAT TGNAATAACT 240
TTTAAGTTTC CTGGNTTANT TAACATTCAC TTGNGTGTCC ANAATTGGNG ATAGACNGNN 300
AGGCACANGN TGTCNTN 317

SEQ ID NO:5220
SEQUENCE LENGTH:51
SEQUENCE TYPE:nucleic acid
TOPOLOGY:linear
CLONE:HUMGS06230
SEQUENCE DESCRIPTION:

GATCATATAC AGTTCCCTTT TTAAAAAGCA ATAAATNCTT GGAATTAGAA A 51

SEQ ID NO:5221
SEQUENCE LENGTH:103
SEQUENCE TYPE:nucleic acid
TOPOLOGY:linear
CLONE:HUMGS06232
SEQUENCE DESCRIPTION:
GATCAAGAAT GTTTCCCAAA GTTNTGAATA GAGTGTCACA ATGTATCCAA GANTTGTGAA 60
GCAATGATTC TTTACAATAC AATAAAGTTT AATTATTTAC AAA 103

SEQ ID NO:5222
SEQUENCE LENGTH:218
SEQUENCE TYPE:nucleic acid
TOPOLOGY:linear
CLONE:HUMGS06233
SEQUENCE DESCRIPTION:
GATCTCACAG ATGTNTGGAA TTTCAAAAAG CCAAACTCAC AGAAGCAGAG TAGGATGGTG 60
GTTGCCAGGG CCTGGGACGT GGGGGAAATA GGGAGATGTT TGGTGTCTGT TAGGAAGAAT 120
GAATAAGCTC TGGAGAGCTA ATGTACAGCA TAGTGACTNN NGNTAATAGA ACCGTATTAT 180
ATACTTGANA TTTGTGAAGA GAGTAGATGT TAANNNNN 218

SEQ ID NO:5223
SEQUENCE LENGTH:87
SEQUENCE TYPE:nucleic acid
TOPOLOGY:linear
CLONE:HUMGS06234
SEQUENCE DESCRIPTION:
GATCTGTCAC TTACTTCTGT GTGACCTTTG AAAGGCTACT TATTTCCTCT CTTAGCTTTC 60
TCATTAAAAT CAATGAACAA TGCCAAA 87

SEQ ID NO:5224
SEQUENCE LENGTH:202
SEQUENCE TYPE:nucleic acid
TOPOLOGY:linear
CLONE:HUMGS06235
SEQUENCE DESCRIPTION:
GATCTCAGGC TGAGCAAAAT NAAACCTTGA TGTTACGGGC TAAATCAAGA GCAGCTTAAT 60
CCTGTTTACA ATGTGAGCTT TTTGTGCGTC TGTGAAATGT TTTACAGTGT TTCTCATCAT 120
CTGTTTCCCA GCAAGGTCTT TNNTTTTNCN ACATTGAAGT NCTGNCTATG TATCTNAATC 180
ACAAATGGNT TCATTCACTN TN 202

SEQ ID NO:5225
SEQUENCE LENGTH:148
SEQUENCE TYPE:nucleic acid

TOPOLOGY:linear
CLONE:HUMGS06236
SEQUENCE DESCRIPTION:
GATCACAAGC GTATCCATNG TGAGGTTCCG TGGATGGAAG CTNATGGATN TNCCTTCCTG 60
ATGTTCATCT GCCACCTTGC TATTNAATGC ATCCTGTTTA TAACTGTCTT CTAAATATTG 120
AATAGAAATA AAGCATTTGT ACAGTAAA 148

SEQ ID NO:5226
SEQUENCE LENGTH:370
SEQUENCE TYPE:nucleic acid
TOPOLOGY:linear
CLONE:HUMGS06237
SEQUENCE DESCRIPTION:
GATCGAAATT GCGGGCTATG GCGCCGAAGT TTTTCGTNAG TACTGGGATA TCCCCGATGG 60
CACCGATTGC NACCGCAAAG CCTACAGCAC CACCAGTATT GCCAGCGTCG CTGACTGACC 120
GCCGCTGCCT ACAGAGTCAC ACTCAATCCT CCGGGCACCT TCCTTGAAGG AGTGGCTAAG 180
GTTGGACAAT ACACGTTCAC TGCAGCTGCT GTCGGGGCCG TGTTTGGNCT NACCACCTGN 240
ATCAGCGTCC ATNTCCGTNA GAAGCCCGGA CGANCNCNTG AACTACTTNN CTCGGTGGCT 300
TTCGCCGGAG GCTTNACTTT TGGGGGNANG GACGTACAAT NTACGGGGTT TGGGGCCGCC 360
GNNTTTGGTN 370

SEQ ID NO:5227
SEQUENCE LENGTH:149
SEQUENCE TYPE:nucleic acid
TOPOLOGY:linear
CLONE:HUMGS06238
SEQUENCE DESCRIPTION:
GATCTGTGTG AAGAATGGTA ATNACGTAGT TGNAAAGGAA AATGTACTGT TGTGTGTTTC 60
ATTTGTGTGA TTTCGTACCA AAAAAATGTG TTTGAACTAT ATTGTATGTA ATTTGGAAGT 120
CGTGTTAATA AAACCCTGCA GTTTCCAAA 149

SEQ ID NO:5228
SEQUENCE LENGTH:129
SEQUENCE TYPE:nucleic acid
TOPOLOGY:linear
CLONE:HUMGS06240
SEQUENCE DESCRIPTION:
GATCAGGCTT TGAAGGCTGG ATTCTATCTA CATAAGTCCN TTCAATTCCA CCATGGGNCA 60
GAGCAGCTCC ACCACGTGTG CACTTAGCCA TNGNTGGCAA CAGAAACCAA GAGACACANN 120
TACGCAGGN 129

SEQ ID NO:5229
SEQUENCE LENGTH:108
SEQUENCE TYPE:nucleic acid
TOPOLOGY:linear

CLONE:HUMGS06241
SEQUENCE DESCRIPTION:
GATCATTTAG GAAAAATAAT TCTATTTTTA GCTTTTCATT TCTCAGCTGT CCTTNTTTCT 60
NNTTTGATTT TTGACAGCAA TGGAGAATGG GNTATATAAA GACTNCCN 108

SEQ ID NO:5230
SEQUENCE LENGTH:102
SEQUENCE TYPE:nucleic acid
TOPOLOGY:linear
CLONE:HUMGS06243
SEQUENCE DESCRIPTION:
GATCAAGAGT TGTCATGCTA GGGAGACTAT TCTGCCTAAA ATTCTGGGGN ACTATTTTGT 60
GTTTAAGNAT ACTATGTATA TTAAAAGGGT ACATATTTGA AA 102

SEQ ID NO:5231
SEQUENCE LENGTH:60
SEQUENCE TYPE:nucleic acid
TOPOLOGY:linear
CLONE:HUMGS06244
SEQUENCE DESCRIPTION:
GATCTTAAAA GTGTTATTTT TGCTTTTATT TTTTAAAAAA GGNNGAGGNT TATTGGCAAA 60

SEQ ID NO:5232
SEQUENCE LENGTH:76
SEQUENCE TYPE:nucleic acid
TOPOLOGY:linear
CLONE:HUMGS06246
SEQUENCE DESCRIPTION:
GATCTGCAGA TAGTTCTTTT TCAAAATATT GTTTAGACAA AATAAATTTC TTTAAATATA 60
ATATCTCTAC TTCAAA 76

SEQ ID NO:5233
SEQUENCE LENGTH:314
SEQUENCE TYPE:nucleic acid
TOPOLOGY:linear
CLONE:HUMGS06247
SEQUENCE DESCRIPTION:
GATCGNTCTG GTCGCAGCTT AGGAACAGCA GACGTGCACT TTGAGCGGAA GGCAGATGCC 60
CTGAAGGCCA TGAAGCAGTA CAACGGCGTC CCTNTGGATG GCCGCCCCAT GAACATTCAG 120
CTTGTCACGT CNNNGATTGA CGCACAGCGG AGGCCTGCAC AGAGCGTAAA CAGAGGTGGC 180
ATGACTAGAA ACCGTGGCGC TGGAGGTTTT GGTGGTGGTN NNGGCACCCG GAGAGGCACC 240
CGCGGAGGCG CCCGTGGAAG AGGCAGAGGT GCCGGCAGGA TTTCAAAGCA GCAGCTTTCG 300
GCAGAGGAGC TTTN 314

SEQ ID NO:5234

SEQUENCE LENGTH:238
SEQUENCE TYPE:nucleic acid
TOPOLOGY:linear
CLONE:HUMGS06248
SEQUENCE DESCRIPTION:
GATCAATACC AAAAGACACT TGGAATCTTC TTTTAGACTT CAGTACGATG ATTGCAGATG 60
ACATGTCTAA TTATGATGAA GAAGGAGCAT GGCCTGTNCT TATTGATGAC TTTTTGGAAT 120
TTGCACGCCC TCAAATTGCT GGGNCAAAAA GTACAACAGT GTAGCACTAA AGGNNCCTTC 180
TAGAATGTAC ATAGTCTGTA CANTAAATAC ANCAGTAAAT TGCACAGTCA GNANNNNN 238

SEQ ID NO:5235
SEQUENCE LENGTH:83
SEQUENCE TYPE:nucleic acid
TOPOLOGY:linear
CLONE:HUMGS06249
SEQUENCE DESCRIPTION:
GATCTGCAAA CAATGAAGAA TTATGTAATA TATTGTACAA ATGTAAGCAA AGGCTCTGAA 60
ATAAAATGCC ATAGTTTGTG AAA 83

SEQ ID NO:5236
SEQUENCE LENGTH:178
SEQUENCE TYPE:nucleic acid
TOPOLOGY:linear
CLONE:HUMGS06250
SEQUENCE DESCRIPTION:
GATCTTAAAT ATCTTGCAGC CTTAAATCAC TACAAACATT TGCATTNTTA TTATGCCAGT 60
TATTTCCAAA ANGTATTGTN ATACATGTCA CTTATGTTTT TAAGCTANTA TTGACTGTAG 120
GATAATCTCC TTGTTCCCCT CAAAATAGTC ATGNAAGTGT ACATGAANNT NTTTTTAN 178

SEQ ID NO:5237
SEQUENCE LENGTH:139
SEQUENCE TYPE:nucleic acid
TOPOLOGY:linear
CLONE:HUMGS06251
SEQUENCE DESCRIPTION:
GATCTGAAGA GCTTTGCAGC TCTNTTAAAT AGGTTGCTGN ACATGTTCTA AGGTTTTGTA 60
GAACACGTGT GCCTGTTTAA GTGTATTGAT GTTAATANTA TTAAATATCC TAATGATNGA 120
ATGCATTGTA TGGNTNCTN 139

SEQ ID NO:5238
SEQUENCE LENGTH:363
SEQUENCE TYPE:nucleic acid
TOPOLOGY:linear
CLONE:HUMGS06253
SEQUENCE DESCRIPTION:

GATCTAAGCC ACAGNGGGGA CTGGAGCCTC GTTTTCCCCC TAGGCAGACC TAAATCAAGC 60
AGCTTACCAC AATAGTGCAT GCTGAGTAGA TTAGTTCCAA GGAAGGGAGA CTGGAATGCT 120
GGTGTCAAGG AAAAGCCTCC CTCATCATCT AGTCTAAGAC CATAACGGGC AGAAGCATAA 180
GAGGTTCCAG GGCCATAGGN GANTGAGAAG TAGGGCACAT ATAGGGAGGA AAGAAAAGTG 240
NAGNNGGGGG ANATCTCCGA NTTTTTTTAC TGCTTGGGAA AAGTGTACTA CATGANGGNT 300
GCTTNGGGCT TTGCTAACCT GCTCACCGCT AACCCTNCCA AGTNTTAACC ATCCCCAGNG 360
GTN 363

SEQ ID NO:5239
SEQUENCE LENGTH:171
SEQUENCE TYPE:nucleic acid
TOPOLOGY:linear
CLONE:HUMGS06254
SEQUENCE DESCRIPTION:
GATCTGAGGA ACAAAATGAT GTCATTTAAT CGGAATCTAA ATGTGACACA AACAACGTGC 60
CAGCAATACC TGCTTGTGAA ATAATGTTCT GAGCCCACAG TGTTCCTGGG TATGTGAGTT 120
TATATCAAGT GAAAAGGCTG CTTAATTGAC ATTAAAGTTT TGGAATGTAA A 171

SEQ ID NO:5240
SEQUENCE LENGTH:348
SEQUENCE TYPE:nucleic acid
TOPOLOGY:linear
CLONE:HUMGS06255
SEQUENCE DESCRIPTION:
GATCATAAGT ATAAATGTCC TCGTTTAATC TTTCCATATT GAGGACAGCC TCTGCATATA 60
AAAATGTTCA TTGGGAGAAA AATTATTTTT AAAACTAAAT TTTTATAGTA GTCCTCCATT 120
ACTGGCAATT AAGGAGCAAC CCACAAAATG TCATTATGTG GTTCTTTGAT TAGGCATAAA 180
ANGTATTGCA AACAGCTTTN CTATTCCTTT GGAATATGAN TCTTTTTAAT GTCTAANGTT 240
AACCTGCAAC ATATTCTAGT AGGACANGTC AATGTAAATN AGGTAATTNT NGCCTGTNTT 300
ATACCTNCTG TNGCCATTTG GTNCAAATGN NAACCATTTN TNAGNCCN 348

SEQ ID NO:5241
SEQUENCE LENGTH:357
SEQUENCE TYPE:nucleic acid
TOPOLOGY:linear
CLONE:HUMGS06256
SEQUENCE DESCRIPTION:
GATCAACAAG AGAAATAAAA AAGCCCAAGA GGAAGTGGTA GGGGAAGGAA TTTAAGAACA 60
GCAATAAGTA AAACTCTTAA GTAACTCCAA AAAGAAAATG GTACATTTTG CCAAAGACCA 120
CTTATACTTG AGAACATGGA AGAATTTGCC TGATACTNNN NGNNGGGGAA AAGAGTCTCT 180
CCTCTTTTCC TCAAACCCCA GTACACTCAG CCTCTNTGCC CCACCTTCTC CTGACTTTGT 240
CCTCACTTGC TTCTGCAGTA CATTGGAACC TGNNTTGAAA GAAAGTCTTC CTTGANTAAT 300
TGGAGTTTGT CTTTGNGNGG CAAATTTAGN CCCNNGGNTC ACAAGNTTTC GGGGGTN 357

SEQ ID NO:5242

SEQUENCE LENGTH:160
SEQUENCE TYPE:nucleic acid
TOPOLOGY:linear
CLONE:HUMGS06257
SEQUENCE DESCRIPTION:
GATCGGGGAC ATCCTTAGGC TGGTGGCCGG CCGCATCAAG AACACCTTCT GATTGAGCGG 60
TTGCCATGGC CGGTCTCCGT GGGGCAGGGT TGGGCCGCAT GTGGAAGGGC TCTGAGCTGT 120
GTCCTCCTTC ATTAAAAGTT TTTATGTCTC GTGTCAGAAA 160

SEQ ID NO:5243
SEQUENCE LENGTH:122
SEQUENCE TYPE:nucleic acid
TOPOLOGY:linear
CLONE:HUMGS06258
SEQUENCE DESCRIPTION:
GATCAGACTA AAAACNATAA CACCATAGGT CAAAANAAGT TGTTCTGAGG TTTTTTTGAA 60
ACATTAAAGN TCCAAAACAT GACATTTTTA AGAATAAATT TGNAATAGAG TATAATTGNN 120
TN 122

SEQ ID NO:5244
SEQUENCE LENGTH:71
SEQUENCE TYPE:nucleic acid
TOPOLOGY:linear
CLONE:HUMGS06259
SEQUENCE DESCRIPTION:
GATCTCCCCT CTCACCCCTC CCACCCATTA ATCTCCTCCC AAAAAACAAG TAAAGTTATT 60
CTCAATCCAA A 71

SEQ ID NO:5245
SEQUENCE LENGTH:114
SEQUENCE TYPE:nucleic acid
TOPOLOGY:linear
CLONE:HUMGS06260
SEQUENCE DESCRIPTION:
GATCTGTTGT AATATATGGT GGATATTTTC CTTTAATTTC AAACATAACC TCTGAAATNT 60
GTATCTNTCC TNTTTNATCT TACCATTANT TTTAAATCTA GTGGATTGGT TTTN 114

SEQ ID NO:5246
SEQUENCE LENGTH:337
SEQUENCE TYPE:nucleic acid
TOPOLOGY:linear
CLONE:HUMGS06261
SEQUENCE DESCRIPTION:
GATCAGACCG CTCAAAGGTC CCCGTNTTCA CTGTTACCCA GAGGCTCTTG TNACTACCCA 60
CTTCATTCCC CACCGCTGCC AGTGCCACTG CCAACCCTGT TCACAGGCGN TTCCAGCCCA 120

CTCCAGCCAG GGGAGCAGGG AAGAAGAAGG GGCTCCCTCC TCTTCACATT CCCCCCGACC 180
CCAAAGCCAG AGAAAGCCAG ATGGCACCAG CTGCTCCGGA TGTGCCTGCC CACATTGGGG 240
GACAGGGCCG GGCCTGGGCT CGGTTCCNAG GTTTGAGCTC TGCAGNCTGT GTCCTGGNGT 300
GAGGGGGCTG AAGTNAGNCC AAAGGAAGAA CTNAGTN 337

SEQ ID NO:5247
SEQUENCE LENGTH:168
SEQUENCE TYPE:nucleic acid
TOPOLOGY:linear
CLONE:HUMGS06262
SEQUENCE DESCRIPTION:
GATCACCTNA TTTTTTNAAC AAAATATTTN TAACAGGAAT GGGTGGGAGT NCTGGTGAAA 60
AGAGGTGAAA TGTGGTTGTA TGAGCCAATC ATATTTGTAA TTNTTTAAAA AAAGTTTAAA 120
AGGAAATATC TGTCCTGAAA CCCCACTTAA GCATTGTNTT NNTATAAN 168

SEQ ID NO:5248
SEQUENCE LENGTH:378
SEQUENCE TYPE:nucleic acid
TOPOLOGY:linear
CLONE:HUMGS06264
SEQUENCE DESCRIPTION:
GATCAACAAA GAGGACTTTG TGGCACTGGA GAGGCTTACC CAGGGGATGG ACATTCAGTG 60
GATGCATGTT NCTGGTCATT CGGGATTTAT AGGCAATGAA GAAGCTGACA GATTAGCCAG 120
AGAAGGAGCT AAACAATCGG AAGACTGAGC CATGTGACTT TAGTCCTTNG GAGAACTTNA 180
GCCAGCGGCT GTCTTGCTGC CTGTACTTAC TTGTGTGGAA AATAGCCTGC AGGTAGGACC 240
ATTGCAGTGA TGGGCAGATT GGGTCTTTCA CACGGNGTCA GGCACAGTGG CCTTCTGTNG 300
NCATGGTNGT TTTATANANA AATNGGTTAA GGTATATAAA TAAAGTNTGG NCCANCTTTT 360
GNNAAGGNAN GANATGGN 378

SEQ ID NO:5249
SEQUENCE LENGTH:131
SEQUENCE TYPE:nucleic acid
TOPOLOGY:linear
CLONE:HUMGS06267
SEQUENCE DESCRIPTION:
GATCANCTAC AGGAAAAGTA AATGTGAACG CAAGCAGAAT GAAACACAGT AAAAGCCGGT 60
CTAGCCTGCC TGGCCACACT CTCAGCGCCT TGTGAGGATG GGNCANTANA TAAANTCATC 120
TTTAGTAGAA A 131

SEQ ID NO:5250
SEQUENCE LENGTH:322
SEQUENCE TYPE:nucleic acid
TOPOLOGY:linear
CLONE:HUMGS06268
SEQUENCE DESCRIPTION:

GATCATCACT AGCAGATGTC AGTTGCACAT TGAGTCCTTT ATGAAATTCA TAAATAAAGA 60
ATTGTTCTTT CTTTGTGGTT TTAATAAGAG TTCAAGAATT GTTCAGAGTC TTGTAAATGT 120
TATTTTAATA ATCCCTTTAA ATNTNATCTG TTGCTGTTAC CTCTTGAAAT ATGATTTATT 180
TAGATTGCTA ATCCCACTCA TTCAGGAAAT GCCAGGNAGG TATTCCTTGG GGAAATGGTG 240
CCTCTTACAG TGTAAATNTT NCCTCCTGNA CCTTTGCTAA TATCATGGCA GANTTNNCCT 300
NATCCCTTTG TGAGGCAGTT TN 322

SEQ ID NO:5251
SEQUENCE LENGTH:215
SEQUENCE TYPE:nucleic acid
TOPOLOGY:linear
CLONE:HUMGS06269
SEQUENCE DESCRIPTION:
GATCAAAAAT AAGATTACAG TTAAAATATT NCTATATTCA GATGGTTTAG AGACCAGGCT 60
GTAGAATCAG ACAGCCCTGA AATTGTATCA CACANGGCTG TGTTACCCTG TACAAATAAC 120
TTAGCCTTAC TAAGCCTGTA TTTCCTCATC TGCAAANTAG GGNTGNTAAT ATACCTGTNG 180
NTAAANATGT TTTCATTAAA AANCGTTGGC GCAAA 215

SEQ ID NO:5252
SEQUENCE LENGTH:229
SEQUENCE TYPE:nucleic acid
TOPOLOGY:linear
CLONE:HUMGS06270
SEQUENCE DESCRIPTION:
GATCTAAAGT GCAGCAGAGT GGCTGNTGCT GCAAGTNATG TCTAAGGCTA GGAACTATCA 60
GGTGTCTATA ATTGTAGCAC ATGGAGAAAG CAANTGTAAA ACTGGATAAG AAAATTATTT 120
TGGCAGTTCA GCCCNTTCCC TTTTTCCCAC TAANTTTTTN CTTAAATTAC CCATGTAACC 180
ATTTNANCTC TCCAGTGCAC TTTGCCATTA ANGTCTCTGC ACATTGAAA 229

SEQ ID NO:5253
SEQUENCE LENGTH:219
SEQUENCE TYPE:nucleic acid
TOPOLOGY:linear
CLONE:HUMGS06271
SEQUENCE DESCRIPTION:
GATCGTGAAA GAGGCTTACC CAGACCACAC ACANGTTTGA GAAAAACAAT CCCCATTATN 60
ACCCATCTAG CAAAGAGGAC AACCCTAAGT GGTCCATGNG TGGATGTACA GTTTGTNCGG 120
ATGATGAAGC GTTTCATTCC CCTGGCTGAG CTCAAATCCT GTCATCAAGG CNCACAANGC 180
TACTGNTGGC CCCTNAAAAA ATATTGTTNT NTGTCACTN 219

SEQ ID NO:5254
SEQUENCE LENGTH:144
SEQUENCE TYPE:nucleic acid
TOPOLOGY:linear
CLONE:HUMGS06272

SEQUENCE DESCRIPTION:
GATCACGCTG AAAGAATACC TTGCAGACAC TTAATCTCTG CGGCAAATGC ATGAAAGTAG 60
AAGGATGAAG CCAACTTTCC AAAAACTTTT ATGAAATTAC TTCAGTGNGA AAACCTATTA 120
AATNAANTNC AGCCNTTCTN TAAA 144

SEQ ID NO:5255
SEQUENCE LENGTH:184
SEQUENCE TYPE:nucleic acid
TOPOLOGY:linear
CLONE:HUMGS06273
SEQUENCE DESCRIPTION:
GATCCCGGGG ACATGATGCG GGAAATCCGC AAAGTNTTGG ACGCCAATAA CTGCGACTAT 60
GAGCAGAGNG AGCGCTTCTN GCTCTNCTGC GTCCACGGAG ATGGGCACGC GGAGAACCTC 120
GTGCAGTGGG AAATGGGAAG TNTGCAAGCT GCCAAGACTN TCTCTNNACG GGGGTCCGGG 180
TNTN 184

SEQ ID NO:5256
SEQUENCE LENGTH:97
SEQUENCE TYPE:nucleic acid
TOPOLOGY:linear
CLONE:HUMGS06274
SEQUENCE DESCRIPTION:
GATCAATGTG GGCACCTAAA AGGGTATGTT AAAATCACCA TTTCTCAGGT CAAAATACTG 60
TGAATAAGTC TTCAATAGAA TCACTANTGG TTNNAAA 97

SEQ ID NO:5257
SEQUENCE LENGTH:173
SEQUENCE TYPE:nucleic acid
TOPOLOGY:linear
CLONE:HUMGS06275
SEQUENCE DESCRIPTION:
GATCATATCT ACATGTATGA ACTTAACATG GAAAATGTTA AGGAAGCAAA TGGTTGTAAC 60
TTTGTAAGTA CTTATAACAT GGTGTATCTN TNTNCTTATG AATATNCTGT ATTATAACCA 120
TTGTTTCTGT AGTTTAATTA AAACATTTNC TTGGTGTTAG CTTTTCTCAG AAA 173

SEQ ID NO:5258
SEQUENCE LENGTH:176
SEQUENCE TYPE:nucleic acid
TOPOLOGY:linear
CLONE:HUMGS06277
SEQUENCE DESCRIPTION:
GATCTTTCTG GGAGCTGCGC CGGACAGAGT GGGGAGCTCC TAGTTTNTGG GGGGAAGCTT 60
TGATATCCAT GCCACGTCCA TCCACCCCAC CCCTNTTCGT CACGAGCACA ATGGTCTTAC 120
ATTNGATNTN TGTAAAAAAA TANAAATAAA TGGAGACTTT AACTCAAGCA ANNAAN 176

SEQ ID NO:5259
SEQUENCE LENGTH:93
SEQUENCE TYPE:nucleic acid
TOPOLOGY:linear
CLONE:HUMGS06278
SEQUENCE DESCRIPTION:
GATCATTTGA ACCTAGGAGT TAGAGGCTGC AGTGAGCTAG AATTGCACCA CTGNATTGCA 60
CCTTGGACAA CAGAGTAAGA CTCCGTCACC AAA 93

SEQ ID NO:5260
SEQUENCE LENGTH:296
SEQUENCE TYPE:nucleic acid
TOPOLOGY:linear
CLONE:HUMGS06279
SEQUENCE DESCRIPTION:
GATCAGGAGG CCACCCCCTT CTGTGGCACT TCTGAGATGA CAACTTGTTT CACAGTTGGA 60
TGGGGGTGGG GAAGGAAAGA GCGGCCCCAT TATTCCAGAA CATTTTATCC ACACAGGCTC 120
AGACACCCTG TGATGAAGAG TCCAAAGAACA GCCTGAGATA AGGATGTCAT TNTTNTTTCA 180
CAGGNCCCAC GATGGGTTTA TNTTTTGGGA CCAAGACATT GGGTCCCTAC ATGTTTTCCC 240
CTACCCTTCT NNGGGAATAA AATATCAAAC ANCCCNCANA GNTGTNNTAT TNNTGN 296

SEQ ID NO:5261
SEQUENCE LENGTH:61
SEQUENCE TYPE:nucleic acid
TOPOLOGY:linear
CLONE:HUMGS06280
SEQUENCE DESCRIPTION:
GATCTATTGA CTTGTAGAGG TTTGGGACAA ACAGGAATAT AAAGATTATG GAATTGGGAA 60
A 61

SEQ ID NO:5262
SEQUENCE LENGTH:339
SEQUENCE TYPE:nucleic acid
TOPOLOGY:linear
CLONE:HUMGS06281
SEQUENCE DESCRIPTION:
GATCACTGCA AAACTCAAGG AGGCCCGGGG CAGAGGAAAA AAATTTACCA CCGATGATTC 60
CATTTGTGTG CTGGGAATAA GCAAAAGAAA CGTTATTTTT NAACCTGTGG CAGAGCTGAA 120
GAAGCAAACG GATTTTAAGC ACAGGATTCC CAAAGANCAG TGGTGGCTCA AGCTACGGCC 180
CCTCATGAAA ATCCTGGCCA AGTACAAGGC CAGCTATGAC GTGTCGGACT CAGGCCAGCT 240
GGAANCATGT GCAGNNCTGG NGTGTCTGAC CCAGTCCCGN CTGCATGTGC CTGCAGCCAC 300
CGTGGNCTGT CTCTTTTTTG TNACACTTAA GTTNTTTTN 339

SEQ ID NO:5263
SEQUENCE LENGTH:258

SEQUENCE TYPE:nucleic acid
TOPOLOGY:linear
CLONE:HUMGS06282
SEQUENCE DESCRIPTION:
GATCTTATCT ACTTTCTCAT CTCCATATTT TGTTAAAGAT AATATTGCCA GATGTAAAGA 60
AAATGGTTTA TTCTACCTAA AGAGTGGATA AGGAGAGAAN ATTATGATGA AATAAAAATT 120
ATAAGCAAAG TTTATTTAAN CTAAATTTAA ATGTGAAACA TCACCTTTTT CCTANTCAGA 180
TTTGATATTG CTACGGATAT ATNTTGNTGA TATNGTCACT ATATANNCAT ANGGGGANTT 240
TNACTNTCTA TTACCCTN 258

SEQ ID NO:5264
SEQUENCE LENGTH:102
SEQUENCE TYPE:nucleic acid
TOPOLOGY:linear
CLONE:HUMGS06283
SEQUENCE DESCRIPTION:
GATCTCTCTC CTCTCTGACC TGGATACGCT TTGGTTTCTC AACTTCTCTA CCCCAAAGAA 60
AAGATTATTC AATAAAGTTT CCTGCCTTTC TGCAAACATA AA 102

SEQ ID NO:5265
SEQUENCE LENGTH:196
SEQUENCE TYPE:nucleic acid
TOPOLOGY:linear
CLONE:HUMGS06284
SEQUENCE DESCRIPTION:
GATCTAGATT TGAAAGTAAA TNTTATGAAG ACATTGCCCA TTTTTACTTC CTCATTCATT 60
ATTGTACCAG CATCATAGCT TTATTACTCT AATCCCAGGT AAGTCAAGCC TACACTGCCC 120
TAGNGGNNGC GTAAACCCAG AANTTCATGC TGGCTTAANT AATCTATTTT TGTTTCTNAT 180
CNTTTGAATN TTTAAN 196

SEQ ID NO:5266
SEQUENCE LENGTH:203
SEQUENCE TYPE:nucleic acid
TOPOLOGY:linear
CLONE:HUMGS06286
SEQUENCE DESCRIPTION:
GATCAGCCTG GGCAACATGG TGAGACCCTG TCTCTACAGA AAAGACCTGA AAATTTTAAA 60
AAAAAGTATA AAGCCAAGCT GTTACCCAGC CACCTTGCTC ACATATTCTC AGGACCTGCT 120
GAGGTGGTTT CACAGGTCAT AGTCCTCACA TTTGCTCAGA ATAAATATCT TCAAATATTT 180
AAANNNNGGN NANAATNTNT GNN 203

SEQ ID NO:5267
SEQUENCE LENGTH:136
SEQUENCE TYPE:nucleic acid
TOPOLOGY:linear

CLONE:HUMGS06288
SEQUENCE DESCRIPTION:
GATCTGGACT ATCCACGTGC TTTGAAACTT TTCCCCTCAC CCTCCAGCCC TGGAGGCTTT 60
TGCTGGGATG AATGTTTTTA TAGGGTTTTT GTTGTAACAT AAGCTATTTT CTAATATGCT 120
GCCAGGTACC CTTAAA 136

SEQ ID NO:5268
SEQUENCE LENGTH:71
SEQUENCE TYPE:nucleic acid
TOPOLOGY:linear
CLONE:HUMGS06289
SEQUENCE DESCRIPTION:
GATCTNGGAC TACNACTGGA CTACTAGCCT CCAGAACTAT NAAATAATAC ATTTCTAATG 60
TCTAAGCCAA A 71

SEQ ID NO:5269
SEQUENCE LENGTH:345
SEQUENCE TYPE:nucleic acid
TOPOLOGY:linear
CLONE:HUMGS06290
SEQUENCE DESCRIPTION:
GATCGGTTTT TGTGGGGAAT CTCCCTTATA AAGTTGAAGA ATCTGCCATT GAGAAGCACT 60
TTCTGGACTG TGGAAGTATC ATGGCCGTGA GGATTGTNAG AGACAAAATG ACAGGCATCG 120
GCAAAGGGTT TGGCTATGTG CTCTTTGAGA ATACAGATTC TGTTCATCTT GCTCTGAAAT 180
TAAATAATTC TGAACTCATG GGGAGAAAAC TCAGAGTCAT GCGTTCTGTT AATAAAGAAA 240
AATTTAANCA ACAAAATTCA AATCCACGAT TGANGAATGT CAGTAAACCT AAGCAGGGAC 300
TTAATTTTAC TTCCAAAACT GCAGAAGGNC ATCCTAAAAG CTTGN 345

SEQ ID NO:5270
SEQUENCE LENGTH:132
SEQUENCE TYPE:nucleic acid
TOPOLOGY:linear
CLONE:HUMGS06291
SEQUENCE DESCRIPTION:
GATCTCTGCT GTGCAGGTGC TAAGATTAAA CACTAAAAAG AAAGAGAGAT ATATGTAATG 60
TACAACTGAC ACTGCCATTT TNCCTTTTTG GAGGAAATGG ACATAGATAA AGAAGATATT 120
TCTNCGGTTA AA 132

SEQ ID NO:5271
SEQUENCE LENGTH:111
SEQUENCE TYPE:nucleic acid
TOPOLOGY:linear
CLONE:HUMGS06292
SEQUENCE DESCRIPTION:
GATCTTCTGC CAAGTAATTT TNTTGCACAA TTTTCCTGTN CCAAANTTTA CTCAGTTAAA 60

AGTAAAANAA TNCTTTACAC GTTTTAGGTT TTAGGACATT AAAAGTATGT N 111

SEQ ID NO:5272
SEQUENCE LENGTH:117
SEQUENCE TYPE:nucleic acid
TOPOLOGY:linear
CLONE:HUMGS06293
SEQUENCE DESCRIPTION:
GATCATCTTA CGATATCAAG CTGAAAATTT CACCACTACC TGGACAGTTN CACATGTTTT 60
ACTGGGAATA TTTTTTCTTT NTTTNNTGTA TGCTCTGTGC TAGTAGGNTT GATTCAN 117

SEQ ID NO:5273
SEQUENCE LENGTH:232
SEQUENCE TYPE:nucleic acid
TOPOLOGY:linear
CLONE:HUMGS06294
SEQUENCE DESCRIPTION:
GATCATATTC ATTATGAAAG GTCAAGCAAA GACAGCAGAN TACCAATCAC TTCATCATTT 60
AGGAAGTATG GGAACTAAGT TANGGAAATC CAGAAAGANG CCAAGATATA TCCTTATTTC 120
CATTTCCAAA CAACTACTAT GATAAATGTG ANGAGGATTC TNTTTTNTTG TGACCTATAA 180
TANTTNTACA NGCTTTCATG CATTTACTTG GTCCTAAGCA AATTTANNGC AN 232

SEQ ID NO:5274
SEQUENCE LENGTH:56
SEQUENCE TYPE:nucleic acid
TOPOLOGY:linear
CLONE:HUMGS06295
SEQUENCE DESCRIPTION:
GATCAAGTAT AATGTTAGCT GCAGGTTTTT AATAAATACC TTAATTCAGT TTGAAA 56

SEQ ID NO:5275
SEQUENCE LENGTH:125
SEQUENCE TYPE:nucleic acid
TOPOLOGY:linear
CLONE:HUMGS06296
SEQUENCE DESCRIPTION:
GATCCTGAAC ATGTGCTGAT TTNAATTGTG CTATATAGTA TATAAANTAA AATANTTTTG 60
GTATTTCTGC AACGTGACCT GATAATNAAT CTATTCATCC TGAGNAAGCT ATACTTCTGT 120
CCNTN 125

SEQ ID NO:5276
SEQUENCE LENGTH:198
SEQUENCE TYPE:nucleic acid
TOPOLOGY:linear
CLONE:HUMGS06297

SEQUENCE DESCRIPTION:
GATCATTGGT TCCANNCACA GGTNTAGTAA TNNTGGGTAC TTTAAGGTTT GGAGCACTTA 60
CAAGGCTGTG GTAGAATCAT ACCCCATGGA TACCACATAT TAAACCATGT ATATCTGTGG 120
AATACTCAAT GTGTACACCT TTNACTACAG CTGCAGAAGT GTTCCGGNTA GACAAAGTTG 180
TGACCCGGGG NNCTCTGN 198

SEQ ID NO:5277
SEQUENCE LENGTH:231
SEQUENCE TYPE:nucleic acid
TOPOLOGY:linear
CLONE:HUMGS06299
SEQUENCE DESCRIPTION:
GATCTCAAGT TGTATAAAAC CAATAAATTT GTGTTACTGC AGTAGTAATC TTATGCACAC 60
GGTGATTTCA TGTTATATAT GCAAAGNAGG CAACTGTTTT CTTAGTTACA GAAGTTTCAN 120
GCTTCACTTT TGTGCAGTAG AAACAAAAGT AGGCTACAGT CTGTGCCATG TTGATGTACA 180
GTTTCTGAAA TTGTTTTACA AGACTTTGAT AATAAAACCC TTAANCTTAA A 231

SEQ ID NO:5278
SEQUENCE LENGTH:180
SEQUENCE TYPE:nucleic acid
TOPOLOGY:linear
CLONE:HUMGS06300
SEQUENCE DESCRIPTION:
GATCAAGNGC CATAGCCTTT ATTCAGGGGG CCTATAAACC CTTCCAGTTC TTGCCCCAGG 60
GCTGGCCTGC TAGCGCTTCA AATTCCCAGG TGTCCCTAAT TTNAGAAGTA ACCTTTTGGA 120
ATAGCATTAG ACCCTGGCTG TCCCCTCCCC ACCAAATAAA CATGATATTT CATTCTNAAA 180

SEQ ID NO:5279
SEQUENCE LENGTH:158
SEQUENCE TYPE:nucleic acid
TOPOLOGY:linear
CLONE:HUMGS06301
SEQUENCE DESCRIPTION:
GATCAAGNNT ANTGCAGCCN TTCTTTTCCT ATTTCTTTTT TTNAAGGGTT AGTATTAACA 60
AATGGCAATA AGTAGAAAAG TTAACATGAA GATTTTAGAA GGAGAGAACT TACAGGACAC 120
AGATTTGTNA TTCTTTGACT GTNACACTAT TGGATGTN 158

SEQ ID NO:5280
SEQUENCE LENGTH:375
SEQUENCE TYPE:nucleic acid
TOPOLOGY:linear
CLONE:HUMGS06302
SEQUENCE DESCRIPTION:
GATCGAACAT NACGTGGTGA TGAAAGCCAN CAGCAGCCTA CCCAAGAAGC TGGCACTGCT 60
GAAGGCCCCA GCCAAGAAGA AAGGGGCAGC TGCCGCCACC TCCTCCAAGA CACCTTCCTG 120

AGGACGCTGG CCCCAGTGCA GGCCAACATC NCACCCCCTA CCTCCATATG GGACCTTGCA 180
GGTAATCCCA CAGGCTGCAC TGTCAGGGGG GGGACCCTGT CCCCCAGCAC TGGGCTTCAC 240
CTAGAACTTC AGTGGGGGCC AAGGGTGCTG AGAACCCAGC AATGNCCAGG ANGATACAGT 300
CACTAAACTT CATCTGTCCC CGTGCCCCTT CCCAGGTCCT GNTTCACAGG TTTAACCCAG 360
NACANTAAAN CCTNN 375

SEQ ID NO:5281
SEQUENCE LENGTH:272
SEQUENCE TYPE:nucleic acid
TOPOLOGY:linear
CLONE:HUMGS06303
SEQUENCE DESCRIPTION:
GATCAGGATG AGATTTAATG CCCATATCTT ACCAGTTCAG TAATCTCCAG AGCCATTTCA 60
CCCTTTAGAG TGAGTCACAT GCAGGGAGTG TGAATGTCAG AGGTGGTTTA TTATCCAGTC 120
TGCCTTACCC TTAATCTGTT CACAGATATT TATTTACTAA TGCTTTTTTT TTCTTAAGAG 180
TTATGGGATA GGAAAATGAA GTGTTTGCTC TTCATTTACT AAATGATTGT AAACTTGAGT 240
TTTTCATCAA AATAAAATTC CATTGTTTTA AA 272

SEQ ID NO:5282
SEQUENCE LENGTH:379
SEQUENCE TYPE:nucleic acid
TOPOLOGY:linear
CLONE:HUMGS06304
SEQUENCE DESCRIPTION:
GATCCAAGGC CACAAGTAAG GCTATGGCTC TAATTCTAGA AGACAACCTT CCAAGATGCC 60
TGGCAAAACC ACCTCCCTGT GCCACACAGA CACACTAGGC CTGTGTATTT ATTTCCCCTT 120
CAAAGCAGAC TGAGGAGGGA GGAGACGAGG TTCTCTTGGC ATCACTTTCT CCCTGGCTGC 180
AGAACTAGAC ACCCTTGAAG ATTTGGCCTG GGCCAGTNAG ACTGAAATCA AGAAAAACAG 240
AAGGGATGTG CAGGGTGGGG GGGTNCACTT CCTGCTCCCA TGTCAACCCC CAGGGCCTCC 300
AGCGTGCAGA CGCGTGTCCT ANTTNATCTG CTCCCACGGA TGANCCTGGT CTTCAATGGG 360
TTAGCAGNNG NGAGATNTN 379

SEQ ID NO:5283
SEQUENCE LENGTH:377
SEQUENCE TYPE:nucleic acid
TOPOLOGY:linear
CLONE:HUMGS06305
SEQUENCE DESCRIPTION:
GATCCTCTGA AAAGGCCCAG AAAAATAGCT CAGTTCAAAT ACAATGTTCT AGGACAATTG 60
GAATATAAAT ATTGTCCAAA AATATAATTA AAAGAAAAAA GTTTAGCACT GTGTAAAGTA 120
AGTGTTAACT GAGGAAGTCC CAAAAAGGTG CTGTCACTTT AAGTTCTGGA CTTGGGGTTC 180
TTTGTATTTG TAAACAGCAA NGCATTTGTG TTTGTTTGTC TATTTGTAAA GCANCCACCT 240
TCCTTATTGG NAGGNGANAA NNNGGGGTNC ATNCATGTNA NTACTTGCTG CAGCATTTNA 300
TATGTTTNAT TTTGTGTTAN GCTTTTTGTT GCATCGTGNN CACATTTATT GTTACCAATG 360
GACATGGGTT CTTTGTN 377

SEQ ID NO:5284
SEQUENCE LENGTH:151
SEQUENCE TYPE:nucleic acid
TOPOLOGY:linear
CLONE:HUMGS06306
SEQUENCE DESCRIPTION:
GATCGTAGTG TNTTTAAATA AACACAGTCC AGACTCAAAC GGAGGAAGCC CACATATTCT 60
ATTATAGATA TATAANTANT CACACACACA CTTGCTGTCA ATNTTTTGAG TCAGTGCATT 120
TCAAGGAACA GCCACANAAT ACACACCCNN N 151

SEQ ID NO:5285
SEQUENCE LENGTH:325
SEQUENCE TYPE:nucleic acid
TOPOLOGY:linear
CLONE:HUMGS06307
SEQUENCE DESCRIPTION:
GATCAACTGA GGAAACTCCA GGCCATGGTG ATTGANATAT CAAACAAAAC CAGCAGCAGC 60
AGCACCTGCA TCTTGGTCCT ACTAGTCTCC TTCTGCCTCC TCCTTGTACC TGCTATGTAC 120
TCCTCTGACA CAAGGGGGAG CCTGCCAGCT GAGCATGGNG TGTTGTCCCG CCAGCTTCGT 180
GCCCTCCCCA GTGAGGACCC TTACCAGCTG GAGCTGCCTG CCCTGCAGTC AGAAGTGCCG 240
AAAGACAGCA CACACCAGTG GTTGGACGGC TCAGACTGTG TACTCCAGGN CCCTGGCAAC 300
ANTTCCTGCC TGCTGCATNA CNGAN 325

SEQ ID NO:5286
SEQUENCE LENGTH:204
SEQUENCE TYPE:nucleic acid
TOPOLOGY:linear
CLONE:HUMGS06308
SEQUENCE DESCRIPTION:
GATCTGCCTC CTGGGAAGGG AAGGGGCCAA TGTCAGCCTC CAGGTGATTT CTTTAATCTG 60
ACATTACAAA GAGACCAACA GACTGCCAAT TCTACCACAT TCCCCTGGTG GGTACTCGAC 120
TATGTAGTTT TNTCTATGCT TCTGGTCAAC TCTGACACAT CTTGGAATTC TTGTAATAAG 180
AAAAGAAATT TTTATACTTT TAAA 204

SEQ ID NO:5287
SEQUENCE LENGTH:219
SEQUENCE TYPE:nucleic acid
TOPOLOGY:linear
CLONE:HUMGS06309
SEQUENCE DESCRIPTION:
GATCCTCAAA CAGTCGGACC AGATGACAGC CAAACTCTGC TAACCCGGGA CTCCTTGCCA 60
TGGACTTTTC CTGTTGTTGT ACACACACAA CAGAACAAAA TGANGCAAAA CAAAACAAAA 120
AAACCCACAA AANTTTGTAA AATGTAATTA ATATTCAANG AAAAGGNGGA AANTCTTCAT 180
TTGTTGGAAA TGAAANCGTT CTAGCAACTG TAANNNANN 219

SEQ ID NO:5288
SEQUENCE LENGTH:259
SEQUENCE TYPE:nucleic acid
TOPOLOGY:linear
CLONE:HUMGS06310
SEQUENCE DESCRIPTION:
GATCTTAAAA TTCATCTAGA TTTCAAGCAT TCAGCTGACT TGCCTCATAA ATGTAGTGAC 60
TGCTTGATGA GGTTTGGAAA TGAAAGGGAA TTAATAAGTC ACCTTCCAGT CCATGAGACA 120
ACTNGATTAT TCTCTNTAAC TTACAGAATG TTAGTNTAAA ATAATAAATN CATCCTTNTT 180
TTGGAGATGA TTAAATGGAT GATTGTAAAC ACAACTTATG AAATCTGCCT NTAACAAGTA 240
NCTTTNTTAA ATNNTANNN 259

SEQ ID NO:5289
SEQUENCE LENGTH:99
SEQUENCE TYPE:nucleic acid
TOPOLOGY:linear
CLONE:HUMGS06311
SEQUENCE DESCRIPTION:
GATCTTATTG GCTGATGGTG TTGTTGAATT CTATATCCTG TTGATTTGCT GTCTAGTTGT 60
TCTATCAATT ATTAAAAGAG GAATGTTAAT GTTTCCAAA 99

SEQ ID NO:5290
SEQUENCE LENGTH:81
SEQUENCE TYPE:nucleic acid
TOPOLOGY:linear
CLONE:HUMGS06313
SEQUENCE DESCRIPTION:
GATCAAATAC TTTAAAGGAA AAAATGTTGG ATTTATAAAT NCTATTTTTT ATTTTACTTT 60
TATAATAAAA GGAAAAGCAA A 81

SEQ ID NO:5291
SEQUENCE LENGTH:132
SEQUENCE TYPE:nucleic acid
TOPOLOGY:linear
CLONE:HUMGS06314
SEQUENCE DESCRIPTION:
GATCTGAAGG CCGTTCCAAG CGAGGGTGCT TCTGAGGACA CTGAGAAGAG TTTTGCCATT 60
CTGAAGAGGC GAGCCTGTGG AACTNTTTAA GGTCCTGTTC CCAGAAGAAT AAAAGTTTCT 120
CATTATGGCA AA 132

SEQ ID NO:5292
SEQUENCE LENGTH:102
SEQUENCE TYPE:nucleic acid
TOPOLOGY:linear

CLONE:HUMGS06315
SEQUENCE DESCRIPTION:
GATCACTCTC CCTGACATCC ACCTGTATGA CTTTGTCACC AAATGCTGTC TTCTCTTTCT 60
CCAATCAAGA AATAATAATC CCTCGAGTTT ACAAAACACA AA 102

SEQ ID NO:5293
SEQUENCE LENGTH:196
SEQUENCE TYPE:nucleic acid
TOPOLOGY:linear
CLONE:HUMGS06316
SEQUENCE DESCRIPTION:
GATCTCANCT TCATGCCAGG CCTTGGGTTT GACAAACATG GCAACCGACT GGGGAGGGGC 60
AAGGNCTACT ATNATGCCTA TCTGAAGCNC TGTTTGCAGC ATCAGGAAGT GAAGCCCTAC 120
ACCCTGGCGT TGGCTTTCAA AGANCAGNTT TNCCTCCAGG TCCCAGTGAA TGAAAACGAC 180
ATTGAAGNTA GATGNN 196

SEQ ID NO:5294
SEQUENCE LENGTH:237
SEQUENCE TYPE:nucleic acid
TOPOLOGY:linear
CLONE:HUMGS06317
SEQUENCE DESCRIPTION:
GATCAAGAAG TTTGTGTACA CATAATCTCA TTTTNAGATA TATAACTATT TTTNTCTTTC 60
AGAAGTGAAT CAAAATATTT CAAAATGCTG TCTTATGAAA CTACAATATT CTCACAGATT 120
AGAAAAGTTT TNCTGTAAAA GTCAGATAGT AAATATTTNA GGTTTTGCAG TGTCTNTTGC 180
ANCTNCTCAA CTTTCCTACT GTNGCACANG NGTAGCTGTG GTACTGTGCA AATNAAN 237

SEQ ID NO:5295
SEQUENCE LENGTH:125
SEQUENCE TYPE:nucleic acid
TOPOLOGY:linear
CLONE:HUMGS06318
SEQUENCE DESCRIPTION:
GATCACTACC TTTTTTTCTC TNATCTGCTG CCATNTGAGA TGTGGCTTTC ACCTTCCGCC 60
ATGATTGTAA GGCCTTCCCA GCCCCGTGGN CCTGTAAGTC CANTAAACCT CTTTTGTAAA 120
TTAAA 125

SEQ ID NO:5296
SEQUENCE LENGTH:198
SEQUENCE TYPE:nucleic acid
TOPOLOGY:linear
CLONE:HUMGS06319
SEQUENCE DESCRIPTION:
GATCTTACAG ACTGTATCAG TCTAATTTTT AAATTTTAAG TTATTTTGTA CAGCTTTGTA 60
AATTCTTAAC ATTTTTATAT TGCCATGTTT ATTTTGTTTG GGTAATTTGG TTCATTAAGT 120

ACATAGCTAA GGTAATGAAC ATCTTTTTCA GTAATTGTAA AGTGATTTAT TCAGANTAAA 180
TTTTTTGTGC TTATGAAA 198

SEQ ID NO:5297
SEQUENCE LENGTH:129
SEQUENCE TYPE:nucleic acid
TOPOLOGY:linear
CLONE:HUMGS06320
SEQUENCE DESCRIPTION:
GATCTAATTT CCCTNTTCAC ACAAACTTTA CACTTTAATC TGATGATTGG ATATTTAATT 60
NNAGTGAACA TCATCTTGTT AGCTAACTTT AAAAANTGGA TGTAGAATGA TTAANGGTTG 120
GTATGATNN 129

SEQ ID NO:5298
SEQUENCE LENGTH:143
SEQUENCE TYPE:nucleic acid
TOPOLOGY:linear
CLONE:HUMGS06321
SEQUENCE DESCRIPTION:
GATCTGTGCC ACTTGTGTCA GAGAAGAAGA AACACGTAGN GCAAGTTGTA AAATAATTGG 60
TATTCCTAAC AATCCTGATG TATAATTNTT TGTTACTTTT GNTTTGAGAA CTCTACANAT 120
AAAAGTGCTG GGACTAGATT AAA 143

SEQ ID NO:5299
SEQUENCE LENGTH:334
SEQUENCE TYPE:nucleic acid
TOPOLOGY:linear
CLONE:HUMGS06322
SEQUENCE DESCRIPTION:
GATCTCTTCG ACTCCTACGA CCTTGGGGAC CTGTTGATTA ATTGAGTGGC CCTGCCTGCC 60
CCCAGCAGCC TGCCCCCGAC TCTACCTCCT CACAGACAGG CTGACAGCCC CTCTGCCTGC 120
ACAGGGACAT TGGACACTAG GTGCTGCCCT CAGGGCATGG GGTCTCCTCG CCTTTCCTGC 180
CCCAGCCGGC AGAAGCTGTG TGGGGNGNNA TGAATGGTAC GGGTGAGGAG TGGATAAGGG 240
GTGGTCCTCA CCTTNCTAAT GGAAGCTGGG CCTAGGGAGG CCCATNCAGT CTTCTGACTT 300
CTNGACCTCT NACAAGAAGG NTGCAGGTGA GGTN 334

SEQ ID NO:5300
SEQUENCE LENGTH:337
SEQUENCE TYPE:nucleic acid
TOPOLOGY:linear
CLONE:HUMGS06323
SEQUENCE DESCRIPTION:
GATCTGGAGC CAGCTGGCTT TGCTCCGTTC CCTGGCTAGT CTGTGCCTGG TCACCAGGGA 60
GGCTGAGTGA GGGGCTGTGA ACAGTTGCCG GAAGCACCCC CTCCCTCCCC GGCCCGTGCA 120
GCAGTGTCTG AGCTGTAGTG AAAGTTTCAG GGCCTGCAAA AGAAGAGGCT TGGGCACAGG 180

ACTGACCATN GCTCCAGGGG TTTAGGACCC CCAGGNCNGT GAAGGTGGGA GCAGCTCACC 240
ACCTTTNACG CAGGCTTTGG TATGTTCTCT NAGGCTTAGT TGATTTTGGG CCCNAAANCA 300
AATNCAAAAG GTTTCTGGGC CAACCTTTTG TTAANTN 337

SEQ ID NO:5301
SEQUENCE LENGTH:326
SEQUENCE TYPE:nucleic acid
TOPOLOGY:linear
CLONE:HUMGS06324
SEQUENCE DESCRIPTION:
GATCACAGCT CCAGGACCAG TGCAGGCTGG CTGCCCTCTT TTGGCCGCGT CTGGAATAAT 60
GGACGCCGCT GGCAGTCCAG ACATCAATTC AAAACTGAAG CTGCAGCAAT GAAGAAGCAG 120
TCACATACAG AAAAAAGCTA ATCATGCTCT CTACCAACTA CCATGAGGCT AGNAAGCAAA 180
GTCAACAAAC CCCTATTATA CCTTCCACCA AATTCTTTAT CATTGTCTTT CTTAGGAAAC 240
AGNCATACTC ATTCATTTGA TTTANTAAGG TTTTATTTTT CCAANGTNTG NTGTGGNTGN 300
TTNGGNNNNN TTGTGGTGGT TGNGNN 326

SEQ ID NO:5302
SEQUENCE LENGTH:174
SEQUENCE TYPE:nucleic acid
TOPOLOGY:linear
CLONE:HUMGS06325
SEQUENCE DESCRIPTION:
GATCTTTNAT TTACCTGTAC AGGAGTGTAA ACTTTTTNGT GNTTTTATTT TTCAATTGTG 60
AGAACCACTG ATTGGTATGT TCAACAAATT TGTGTATACA AAGAAATGGA TAAATCACTG 120
CTATATANGG GAAACTACCT TAGGAAAGAN TGTTTACTGA ATGTTTATNN NATN 174

SEQ ID NO:5303
SEQUENCE LENGTH:104
SEQUENCE TYPE:nucleic acid
TOPOLOGY:linear
CLONE:HUMGS06326
SEQUENCE DESCRIPTION:
GATCAGTGAT ATTTGATGTT AGTGTTGTAC TATAGGGTGC CAATAACTCT GCCTATACAA 60
GATGGTGTAC TTAATCAATA AACGTGTATT CTGACTGCCT TAAA 104

SEQ ID NO:5304
SEQUENCE LENGTH:238
SEQUENCE TYPE:nucleic acid
TOPOLOGY:linear
CLONE:HUMGS06327
SEQUENCE DESCRIPTION:
GATCCTTCCG TCTCAGCTCC CCAACCCCAG TAGCTGGGAC TACAGTTGCA CATCATCATG 60
CCTGGCTAAT TTTTTNATTT TTGTAGAGGT AGGTTTCACT ATGTTGCCCT GGCTGGTCTT 120
GAACTCCTGG GCTCAAGTGA TTCACCTGCC TCGGNCTCTC AAAGNGCTGG TGTTTACNGG 180

CATGAGCCAC TGTGTCCGAC CCANACCTAC TANTGTTAAT NGANNTTTTN TTAACTNN 238

SEQ ID NO:5305
SEQUENCE LENGTH:132
SEQUENCE TYPE:nucleic acid
TOPOLOGY:linear
CLONE:HUMGS06329
SEQUENCE DESCRIPTION:
GATCAACAGA GTGAGACCCC TGTCTATATA TTNNTTAAAT TTAAAAAATA AAAGANTAAA 60
ATTGTGTAGC TCAGTATAGT ATCAAGATTA ATCTGCCTAC TCACATTTCT ACACTTTATA 120
NNANTGTAAT AN 132

SEQ ID NO:5306
SEQUENCE LENGTH:293
SEQUENCE TYPE:nucleic acid
TOPOLOGY:linear
CLONE:HUMGS06330
SEQUENCE DESCRIPTION:
GATCCAGCCA TNACTAACCT ATNCCNNTTT TGGGGAAATC TGAGCCTAGC TCAGAAAAAC 60
ATAAAGCACC TTGAAAAAGA CTTGGCAGCT TCCNGATAAA GCGTGCTGTG CTGTGCAGTA 120
GGANCACATC CTATTTATTG TGATGTTGTG GTTTTATTAT CTTAAACTCT GNTCCATACA 180
CTTGTATAAA TACATGGATA TTTTTATGTA CAGAGGTATG TCTCTTAACC AGTTCACTTA 240
TTGTNCTCTG GCAATTTAAA NGANNGTCAG TAAATTNTTT TNCTTGTNAA AGN 293

SEQ ID NO:5307
SEQUENCE LENGTH:228
SEQUENCE TYPE:nucleic acid
TOPOLOGY:linear
CLONE:HUMGS06331
SEQUENCE DESCRIPTION:
GATCAAAACC CAGCAGAGTG CAAGCAGCAG TGAAGCAGGA TGTATGTGGC CTTGAGGATA 60
ACCTNCACTG TAATAGCCTA AACACANCTC TAATTTACTT ACAGCCTTAT GTTTTTGTAT 120
TGNCTTGGTA GACTAGGTAA TTTTTTTTTA AAGGNCAGGN GACGGNTATT TTAAAGNCCA 180
ATTTGTTCTA CGTAGCATNT TAACTAGTTT TNNTGCCAGC NATGTTGN 228

SEQ ID NO:5308
SEQUENCE LENGTH:113
SEQUENCE TYPE:nucleic acid
TOPOLOGY:linear
CLONE:HUMGS06332
SEQUENCE DESCRIPTION:
GATCCCTGAA GTTGCCCTGG TCTCTGCACC TTCTAAACCT AGTTCTTAAG AGCTTTCCAT 60
TACATGAGCT GTCTCAAAGC CCTCCAATAA ATTCTCAGTG TAAGCTTCTG AAA 113

SEQ ID NO:5309

SEQUENCE LENGTH:154
SEQUENCE TYPE:nucleic acid
TOPOLOGY:linear
CLONE:HUMGS06333
SEQUENCE DESCRIPTION:
GATCTGTCAC TGTCTCCCGT CACCCNCAGA TGGGGCTGTC TAGTTGCAGG AAAACAAGCT 60
CAGGGCTCCC ACTGAGTCTC TGTGATNGTG AGTTGTAGAA TTATTTAANN ATATGTTACA 120
ATGTAATANT AGTAGAANTA AAGTGCACAN TAAN 154

SEQ ID NO:5310
SEQUENCE LENGTH:214
SEQUENCE TYPE:nucleic acid
TOPOLOGY:linear
CLONE:HUMGS06334
SEQUENCE DESCRIPTION:
GATCTCTTTC CCTGTCTTGG AGGTGAAAAG TNATATCTAA GGNTNTGTTT GCAGTGACCC 60
CTAGTTTGGG GTACACAGAC CAGTGTTCCT NATTGACAGT GTTCAATAAG GCCCCGTCAT 120
TCTCGCCAGT NTNTTGTTGT NCTTAATGGG CTCCTCCTTG AANTGTGTGT GTGTTTGTGT 180
CAAGAGGAGT TNGTGTTCTT GGTAAATAAA GNTN 214

SEQ ID NO:5311
SEQUENCE LENGTH:127
SEQUENCE TYPE:nucleic acid
TOPOLOGY:linear
CLONE:HUMGS06335
SEQUENCE DESCRIPTION:
GATCTCTNCG ANTNAAATTC AATCTAGGGA AAAATGGCAG AGAAGTAAAT GGATGTNCTG 60
GTGTCAATAG ATATGAAAGT GTTGGTTGGC GACTTGCAAA TCAACAAAGT TTAAAAAATC 120
GAATNNN 127

SEQ ID NO:5312
SEQUENCE LENGTH:67
SEQUENCE TYPE:nucleic acid
TOPOLOGY:linear
CLONE:HUMGS06337
SEQUENCE DESCRIPTION:
GATCGAGAAT NACNATNTTT TGTAAATGGA AATACCACTA CTAAAGACAG AATGCTATAA 60
ATAGAAA 67

SEQ ID NO:5313
SEQUENCE LENGTH:130
SEQUENCE TYPE:nucleic acid
TOPOLOGY:linear
CLONE:HUMGS06338
SEQUENCE DESCRIPTION:

GATCTTCTGT ACTTCTGTCT TCCATAGGAC AAATNATAAG TACTACATAC CTCATCTCTT 60
GGGTTATNAT TGTAGTCTTG CATTCATGAT TATNAATTTA AAAATAAATA CCAATTATGG 120
AAATTGTAAA 130

SEQ ID NO:5314
SEQUENCE LENGTH:106
SEQUENCE TYPE:nucleic acid
TOPOLOGY:linear
CLONE:HUMGS06339
SEQUENCE DESCRIPTION:
GATCTACATN AGGAAAAGTA TAAGCCCCCT CCCTAATGGC CGCTGGGNGG TGAGGGCGGT 60
GTGTTGTATG TCTTTGGGTG TTTGTTNNTT NATAAAGCAT ATAATN 106

SEQ ID NO:5315
SEQUENCE LENGTH:195
SEQUENCE TYPE:nucleic acid
TOPOLOGY:linear
CLONE:HUMGS06341
SEQUENCE DESCRIPTION:
GATCTNAAAA AGAGAGAAGA AAATAAAAAG TATTATTTAT ATGTCAGTGC CAACTTTGAA 60
GGTAAAACAG TAGTAGTAAC TTTTGGGAGA AATTCTAAAA ACTACTTTAA AAAGAAGCGT 120
GCACATAAAA TAANTATCTT TCTATCCAAG GCTTGTCATT TNATATGCTT TNNTTGTAAA 180
TTTAANATNN NGCTN 195

SEQ ID NO:5316
SEQUENCE LENGTH:136
SEQUENCE TYPE:nucleic acid
TOPOLOGY:linear
CLONE:HUMGS06343
SEQUENCE DESCRIPTION:
GATCTAATTA CTTATAGATT CTGTAGTCTN GNGAAGGNGT GGGTGACGTG ATGAGAGGTT 60
TGAGAAATGG GTGAAATGAA ATGGGGGGAT ATGTAGGTCA AATCANATNA AAGATGATTT 120
TTTTNATGTG AATAAN 136

SEQ ID NO:5317
SEQUENCE LENGTH:180
SEQUENCE TYPE:nucleic acid
TOPOLOGY:linear
CLONE:HUMGS06344
SEQUENCE DESCRIPTION:
GATCAGCTTA AGGNTTTAGC GCTGGTGGAT AGACACTCGA GACCAGAAAT CATATTTTTN 60
CTGAAAGTAG ATGATTTACC TGAGGATAAT CAGGAACGTT TTAATAGCCT TTNCTCTCTA 120
AGGGAGAAGT GGACAGAAGA AGATATTGCT CCATATATTC AAGATTTGTG TGGAGAGAGN 180

SEQ ID NO:5318

SEQUENCE LENGTH:83
SEQUENCE TYPE:nucleic acid
TOPOLOGY:linear
CLONE:HUMGS06345
SEQUENCE DESCRIPTION:
GATCGCGGTA TATAGAGATA TGCATTTTAT TTTACTTGTG TAAAAATATC GGACGACGTG 60
GAATAAAGAG CTCTTTTCTT AAA 83

SEQ ID NO:5319
SEQUENCE LENGTH:180
SEQUENCE TYPE:nucleic acid
TOPOLOGY:linear
CLONE:HUMGS06346
SEQUENCE DESCRIPTION:
GATCTCCTTA GGTGATGCAC AGTCCAACTA CCTGCTAACC ACCGCCGAGA ACGAGCTGGG 60
AGTGGTGGTA GCCCACAGTG AGTCAGGTAT CCAGATGGTT CCCATCAGCT GGTGTGAGAT 120
GCAGTGCCCT AAGACCCACA CTAAAGAATT CCGGAAAGTA GCCCGNGTAC AACCCGAATN 180

SEQ ID NO:5320
SEQUENCE LENGTH:126
SEQUENCE TYPE:nucleic acid
TOPOLOGY:linear
CLONE:HUMGS06347
SEQUENCE DESCRIPTION:
GATCAGAGAA TCACAAGAGC AGCAAATGTG GTTTCATCAA GTGGGAAGAA AGCAGCAATT 60
TAAAATANCT TTTTGGGNGA CTGANTTGAG TAATAATAAA NCTTCAGTCT TTCGCTAATN 120
ATANTN 126

SEQ ID NO:5321
SEQUENCE LENGTH:225
SEQUENCE TYPE:nucleic acid
TOPOLOGY:linear
CLONE:HUMGS06348
SEQUENCE DESCRIPTION:
GATCTAGACG CCCCCAAATC CTTGAGATGT GGGTATAGCT CAGGGTAAGC TGCTCTGAGG 60
TAAAGGTCCA TGAACCCTGC CCCACTCCTG TCAGCCCCTC ATCAGCCTTT TCAGCAGGTT 120
CCAGTGCCTG ACTTGGGATA GGACTGAGTG GTAGGAGGAG GGGGAGTGGA GGGGCATAGC 180
CTTTCCCTAA TTCTGCCTTA AATAAAACTG CATTGCTGAT TCAAA 225

SEQ ID NO:5322
SEQUENCE LENGTH:341
SEQUENCE TYPE:nucleic acid
TOPOLOGY:linear
CLONE:HUMGS06350
SEQUENCE DESCRIPTION:

GATCAGCAAA TTGTTATNTT AAAAAGTTTT CTTGTTTAGG CAATTTGGTA AGTGGTTTCT 60
AGTTATAAAC TAGCCACTGT TGATTAACTT GAGAAGGTGA AGCAGGGGAG TTCTGAAGAG 120
ATTACCTAGA GCTACTTAAC CCTAAGTTAA GTAAAATTTT CACTTTCATT CTTTTCATTC 180
TCTCATAAAC TGGTTGAAAA AACACTGTAC TACCAACAAA GGTGTCAGTT GCTTGTGCTG 240
CCCTGTCTAT GTTATGCTCT GAATAGGTCT CGGTAAAGAT TATATGGAAA TACTATAAAG 300
NNTACATAAG GTAAAATACT AGTCAGANAG GGTTCAGGTT N 341

SEQ ID NO:5323
SEQUENCE LENGTH:157
SEQUENCE TYPE:nucleic acid
TOPOLOGY:linear
CLONE:HUMGS06351
SEQUENCE DESCRIPTION:
GATCTGAAGT NCTTCCATTA TATCTATATC TGTATATGNA CATATATGTG GGTGTGTATA 60
TATATGNGTG TATGTATATA TATACAATTN CCTGAGAACT CACTNTTTTT TGTGAATTTN 120
CAATAGTGTT CGCCATGTTT TCCACCAGNA TGTATAN 157

SEQ ID NO:5324
SEQUENCE LENGTH:94
SEQUENCE TYPE:nucleic acid
TOPOLOGY:linear
CLONE:HUMGS06353
SEQUENCE DESCRIPTION:
GATCAAGTTT TGATGAAGAT GTGAAAAACN GTTGGGCAGT ACTACTCAAG CTGAACCCCA 60
TGCATCAGAG AAATTACACT TTTATATNTC CAAA 94

SEQ ID NO:5325
SEQUENCE LENGTH:128
SEQUENCE TYPE:nucleic acid
TOPOLOGY:linear
CLONE:HUMGS06354
SEQUENCE DESCRIPTION:
GATCTGCCCT NCTCAGCCTC CCAAAGTGCT GGGATTAGAG GCGTGAGCAC CATGCCTGGC 60
CAGGTCTTNC TAACTAAAAA ATAATAATAA TAATAATAAA AAANAAAAGA NGATACTTGC 120
AAAATAAA 128

SEQ ID NO:5326
SEQUENCE LENGTH:117
SEQUENCE TYPE:nucleic acid
TOPOLOGY:linear
CLONE:HUMGS06355
SEQUENCE DESCRIPTION:
GATCAAGAGT ATTTNCTNTA GCTAGCTTAA AAAGAAAACA TATATTTAAT GTAAAANCAC 60
ATACATGGCC AAATGCAATA CTGANGAGAC AATCATCCTG TATCTTTNAT GGTTTTN 117

SEQ ID NO:5327
SEQUENCE LENGTH:334
SEQUENCE TYPE:nucleic acid
TOPOLOGY:linear
CLONE:HUMGS06356
SEQUENCE DESCRIPTION:
GATCAATGCG CTAGTGCAGG AGCTCGGCTT CTACCGCAAG GCGGCGCCCG ACGCGCAGGT 60
GCCCCCCGAG TACGTGTGGG CGCCCGNAAA GCCCCAGAGG AAACTTCGGA CCACGCTGAC 120
CTNTAGGTCC GGGGGCGCGG CGGANCTGGG ACCTACCTGC CTGAGTCCTG GAGACAGAAT 180
GAAGCGNTCA GCATCCCGGG AATACTTCTC TTGCTGAGAG CCGATGCCCG TCCCCGTGGC 240
CAGCAGGGAT GGGGTTGGGG AGGTTCTTCC AACCNGACTT TNTTTCCTTC CNCAAGNTCC 300
ACTAAAATTT CCCTTCTGCN TTAAAAAAAA AANN 334

SEQ ID NO:5328
SEQUENCE LENGTH:330
SEQUENCE TYPE:nucleic acid
TOPOLOGY:linear
CLONE:HUMGS06357
SEQUENCE DESCRIPTION:
GATCAGCCAA ATTGGGCGGC CACCCCCGNC TCCACCACTT TCCACCATCA GCTGCCAAAC 60
TGGTCCCTCT GTCTCCCTGG GGCCTTGGGT TCTGTTTGGG GGTCATGACC TTCCTAGTTT 120
CCTGACGCAG GGGNATACAG GGGAGAGGGT TGTCCTTCCC CCCAGCAAAT GCAATAATGC 180
CCTCACCCCT CCTGAGAGGA GCCCCCTCCC TGTGGAGCCT GTNACCTTCG NATTTGACAC 240
GAGTCTGCTG TGAACCNCGC ANTCTGCTTC NCACCTNCNA TCTNTCCTNC CAGGGCCATN 300
CCTGGGCCAG AGCAGGAGGG GAGGGAGGTN 330

SEQ ID NO:5329
SEQUENCE LENGTH:175
SEQUENCE TYPE:nucleic acid
TOPOLOGY:linear
CLONE:HUMGS06358
SEQUENCE DESCRIPTION:
GATCAAAAAT NTGTGGGTTT ATTTCTGGAG TCTCTGTTCT ATTGGTCTAT ATGTCCGTCT 60
TCATGCTGGT ACTATGCTGT TTTATTTACT GTAGCTTTGT AATATATTTN GAAATCAGAA 120
GGTGGGATGC CTTCAGCTTT GTNCTTTTTN CTCAAAATAA AAAATNCTTT TGAAA 175

SEQ ID NO:5330
SEQUENCE LENGTH:123
SEQUENCE TYPE:nucleic acid
TOPOLOGY:linear
CLONE:HUMGS06359
SEQUENCE DESCRIPTION:
GATCAAATCA GAGTAATTTG GATATTCATC ACCTCGAATA TTTATCTTTC CTTTGTGTTG 60
GGAACATTAT AATTTTCCTC TGCTAGCTAT TTTAGAATAT ATAATAAATN ATNGTCCCCT 120
AAA 123

SEQ ID NO:5331
SEQUENCE LENGTH:161
SEQUENCE TYPE:nucleic acid
TOPOLOGY:linear
CLONE:HUMGS06360
SEQUENCE DESCRIPTION:
GATCAAAGCC AGCCACCCCC ACCCCAACAC ACTCGGTGTC CCTTTCATCC TGGGCCTNTG 60
TGAATCCCAG CCTNGGCCAT ANCCTCAACC TCAGTGGGCT GGAAATAACA GTGGGGNCCC 120
TGTAGCAGTG GCAGAATAAA CTTAGATGTT NTNACAGGAA A 161

SEQ ID NO:5332
SEQUENCE LENGTH:139
SEQUENCE TYPE:nucleic acid
TOPOLOGY:linear
CLONE:HUMGS06361
SEQUENCE DESCRIPTION:
GATCNTCTCA GNGANGNCGC TTNGGTCGCA GTTTTAGGGG GTTCTCGGTT TGCAGGAACC 60
CTGGGTGCAG GTTCGCAGCC CTGTCAACCA GCTCCGAGCC GGCAGCGAAA CCTGAAGTGG 120
ACCCTGTGGA AAATNAAGN 139

SEQ ID NO:5333
SEQUENCE LENGTH:145
SEQUENCE TYPE:nucleic acid
TOPOLOGY:linear
CLONE:HUMGS06362
SEQUENCE DESCRIPTION:
GATCTNCTCA CCCTCGGTGA NAACAGTGTC AGCCATGCAA GCAGGACAGA ATGGTGACTG 60
GGTGCCCTTG GTGAGCTGTG TATTTCCTAG NAGGTAGAAA ACTGTGGGAA ACTGTGGCTA 120
ATAAAAACTA AGTGTGAGCG TCAAA 145

SEQ ID NO:5334
SEQUENCE LENGTH:351
SEQUENCE TYPE:nucleic acid
TOPOLOGY:linear
CLONE:HUMGS06363
SEQUENCE DESCRIPTION:
GATCTTAAAT NACCTATTTC TAATGTGATT CATACACGTT TTTNACTTAG TGGTTTAAAA 60
TATGAAAGTA TATAAACACA TTACCAAACA CTTTGGGGTA GGAGAAGTAC GCTTAGCATG 120
TCTGTGTCAT ACCTAATTTT TTTAAAGCTA TAATGAACTG ATACACATCA ATTTANNNCG 180
NGGTTTTATT CTTTCAATGA ACACTGTATC AAAATTTTTT NATGANGTAA TGTTAGTCTT 240
TGTGGTCAAT AGTTATTGGT AATACTACTA GTAATATGGG TNCATGGCTA TAAGATGCTT 300
TAGCTTAGTG GTCAGTTGTT ATNCTAAGGG GTTTNAATCN AATTNGGTNG N 351

SEQ ID NO:5335

SEQUENCE LENGTH:348
SEQUENCE TYPE:nucleic acid
TOPOLOGY:linear
CLONE:HUMGS06364
SEQUENCE DESCRIPTION:
GATCACAAGT AGAGAACTTG TTTCTTTTCA CCCCTCTTGC TCCTCCCCGG AGTTATGAAA 60
AATTTCAAAC CTGCCAAAAG TTTGAAGTTT ACAACAAATA CCCTCCAACG TTTGGNTTGC 120
CTCCCTCCCC CTTCTCTCCC TTNCTCCCTC TGATTTCTTA CAGAACCACG CTTTCATGAC 180
CATGCCCGGG AACTTCACGC TGGCGCGGTG AGGTCCTGCG CCTCCGNTTN CNGCACACAC 240
GTGCTTCTNA ATAGCTGCAG GCTATTTCAT GAGAGGAATT TGCTCACGTT AACATNGCTG 300
TTCCCAAATG CCAGGCTGNT TTTAAGTGGT TTCCTAATTT ATAAAGGN 348

SEQ ID NO:5336
SEQUENCE LENGTH:220
SEQUENCE TYPE:nucleic acid
TOPOLOGY:linear
CLONE:HUMGS06365
SEQUENCE DESCRIPTION:
GATCTCAATA GTCAGTTACT GATGCTCCTG AACCCTATGT GTCCATTTNT GCACACACGT 60
ATACCTCGGC ATGGCCGCGT CACTGCTCTG ATTATGTGCC CTGGCCAGGG ACCAGCGCCC 120
TTGCACANGG GCATGGTTGA NTCTGAAACC CTCCTTCTGT GGCAACTNGT ACTGAAAATC 180
TGGTGCTCAN TAAAGANGCC CATGGCTGGT GGCATGCAAA 220

SEQ ID NO:5337
SEQUENCE LENGTH:227
SEQUENCE TYPE:nucleic acid
TOPOLOGY:linear
CLONE:HUMGS06366
SEQUENCE DESCRIPTION:
GATCCCAGCC ATAAAGTACC TGGGATGAAA GAAGTTTTTT CCAGTTTGTC AGTGTCTGTN 60
AGAATTACTT ATTTCTTTNC TATATTCTCA TAGCACGTGT NTGATTGGTT CATGCATGTA 120
GGTCTCTTAA CAATGATGGT GGGCCTCTGG AGTCCAGGGG CTGGCCGGTT GTTCTATGCA 180
GAGAAAGCAG TCAATAAATN TTTGCCAGAC TGGGTGCAGA ATTTAAA 227

SEQ ID NO:5338
SEQUENCE LENGTH:91
SEQUENCE TYPE:nucleic acid
TOPOLOGY:linear
CLONE:HUMGS06367
SEQUENCE DESCRIPTION:
GATCTTNACT CTTGTTTCCT TTGTCCTTTT NTTCAGACAG AGTTGTACCT GCAGCAGACA 60
ACTCTGAATT AAAGCATGAA AACACAGCAA A 91

SEQ ID NO:5339
SEQUENCE LENGTH:269

SEQUENCE TYPE:nucleic acid
TOPOLOGY:linear
CLONE:HUMGS06369
SEQUENCE DESCRIPTION:
GATCTCTCCC CACACAGAGG CCAGCAGACC TCTTCCTGAG GACTTCTGTT TAAAGGAGGA 60
CGAGGAGGAG GTTGGTGACA GTCAGGCCTG GGAGGAGCCC ACAAACTGGA GCACAGAGAC 120
ATGGAACCTA GCTACTTCCT GGNAGGTGGG GCGGGNACTA CGGAGAAGGT GCAGCCAGGC 180
TGTGGCAAAG GGCCCCANTC ACAGCCTTGG CTGGGAAGGN GGGNNCGACA GCTGANGGTC 240
GACTAAAACA AAGTCTGTTT TCATGNAAA 269

SEQ ID NO:5340
SEQUENCE LENGTH:67
SEQUENCE TYPE:nucleic acid
TOPOLOGY:linear
CLONE:HUMGS06370
SEQUENCE DESCRIPTION:
GATCTCTTGT NATGGGGCCA ATAAGTCAAT TGAATTCATG GGCCAAACAG TTCCCATTCT 60
CTTCAAA 67

SEQ ID NO:5341
SEQUENCE LENGTH:313
SEQUENCE TYPE:nucleic acid
TOPOLOGY:linear
CLONE:HUMGS06371
SEQUENCE DESCRIPTION:
GATCCTNATN ACGNAGCTGA TGCCTGCCTT GGTNCCTACC ACTCTGGTGT AGACAGAGGA 60
CTTTNTCCAA GTGTCACACT AAACATGCCC GGGCTTCGAT GTGCCTGGAG GATTTCCCCA 120
CAGGGACCCA CGCACCTCCC TGTATCTGCG CAGTTGCTCG TGTGGCTGGA AGCGCTGTCG 180
ATAAGCTTCN NGGCGCTGGN TTGCCAGTTC TTCAGCTCTA GGACTTGTAG CTTTAAGCAA 240
ACGGTGTGGC ATGGGNTTGA NGTNTGGCCA CCTGGCAGNG NTGTGCAGGG TCTTGCCTTA 300
NNAAAGCTGG CTN 313

SEQ ID NO:5342
SEQUENCE LENGTH:87
SEQUENCE TYPE:nucleic acid
TOPOLOGY:linear
CLONE:HUMGS06372
SEQUENCE DESCRIPTION:
GATCAAAAAA TATATGAAAG TTCCTTGTAG ATACATATCT ATAGATATAT ATGTNTATGT 60
ATATAAAGAT AGATATATAC ATTGAAA 87

SEQ ID NO:5343
SEQUENCE LENGTH:125
SEQUENCE TYPE:nucleic acid
TOPOLOGY:linear

CLONE:HUMGS06373
SEQUENCE DESCRIPTION:
GATCTTTTTA TATTCTTCTA CCACACCTGG AAACAGACCA ATAGACATNT TGGGGTTTTG 60
TAATGGGAAT NTNTATAAAG CATTACTCTT TTTNAATAAA TTGTTTTTNA ATTTAAAAAA 120
AGAAA 125

SEQ ID NO:5344
SEQUENCE LENGTH:51
SEQUENCE TYPE:nucleic acid
TOPOLOGY:linear
CLONE:HUMGS06374
SEQUENCE DESCRIPTION:
GATCAGGTGG AAATGTTTAA GCTAAATAAA TCTAGGGGTT NTTTNAACAA A 51

SEQ ID NO:5345
SEQUENCE LENGTH:273
SEQUENCE TYPE:nucleic acid
TOPOLOGY:linear
CLONE:HUMGS06376
SEQUENCE DESCRIPTION:
GATCTGAAGA AACAACGCAG TGTNCACGCA AGCCACTCAG ACAAGCCCAG GGGTTCCTTG 60
GCCTTCTCAG TCAGCTAACT TTCCTGAGTT CTCCTTTGAC TTCACTAGGG AACAGTTGAA 120
CAAGAGATTT TTCAATCAAG NCTTTGGAAC TACGAAGATG GAAGAGATTT GAAGAGGGAT 180
ATNGAATGAG TAAATTAAGC AACTTTTTAN TATCCTATTT CTNTAAATNT CCAGATTTTA 240
AAACATTCCT GTATTATANT ATCTAAAAAC AAA 273

SEQ ID NO:5346
SEQUENCE LENGTH:76
SEQUENCE TYPE:nucleic acid
TOPOLOGY:linear
CLONE:HUMGS06377
SEQUENCE DESCRIPTION:
GATCTAGATA TTTTCCTCTT TTTCTGAAGN TACTTTCTTA AAAAATAAAT AAAATGTTTA 60
TAGCATTCCT NGTAAA 76

SEQ ID NO:5347
SEQUENCE LENGTH:295
SEQUENCE TYPE:nucleic acid
TOPOLOGY:linear
CLONE:HUMGS06378
SEQUENCE DESCRIPTION:
GATCAATTCT CCTTTGCGAG GNGACCACCA ANGGACTGCA GAGCTGNCCG GGCATTCGTG 60
CGTGTTCATG ACCGAGAACT TTNCACACAG AGAGGACTTC TGTCCTGACG AAGGAGCGCA 120
GAGCTGGCCG NGCATTCGTG CGTGTTCATG ACCGNGAACT TTTCACAGCT AGGACTNCTG 180
TCCTGACGTC TTTATNGGTA TAATAGTTGC GTTTAAGNCT GCAGACTTCA AGTTTATATT 240

TCTCTTTATT GTAACACTCT GTGCANNNTT TGATTNCTAG TTTATTTNCA TTGTN 295

SEQ ID NO:5348
SEQUENCE LENGTH:416
SEQUENCE TYPE:nucleic acid
TOPOLOGY:linear
CLONE:HUMGS06379
SEQUENCE DESCRIPTION:
GATCGACCTG AGCACAGTGG ACCTGAAGAA GCTCCGAGTT AAAGAGCTGA AGAAGATTCT 60
GGATGACTNN NNGGAGACAT GCAAAGGCTG TGCAGAAAAG TCTGACTACA TCCGGAAGAT 120
AAATNAACTG ATNCCTAAAT ATGNCCCCAA GGCAGCCAGT GCACGGACCG ATTTGTAGTC 180
TGCTCAATCT CTGTTGCACC TGAGGGGGTA AAAACAGTTC AACTGCTTAC TCCCAAANCA 240
GCCTNTTTGT AATTTATTTT TTAAGNGGGC TCCTGACAAT ACTGTATCAG ATGTGAAGCC 300
TGGAGCTTTC CTGGATGATG CTGGNCCTGC AGTACCCCCA TGAGGGGATT NCCNTTCCTT 360
CTGTTTGCTG GTGTACTNCT GGGGGNGNGA AGTGTGTCTG GGGNNTTTTT TNNTGN 416

SEQ ID NO:5349
SEQUENCE LENGTH:88
SEQUENCE TYPE:nucleic acid
TOPOLOGY:linear
CLONE:HUMGS06380
SEQUENCE DESCRIPTION:
GATCTGAAGA AGTGGTTAAG ACCAAGAGTT TATGTACCTT TTTAAACAAA GATTAATAAA 60
TTTTTGAAGA AGTGATGAGA CAAAGAAA 88

SEQ ID NO:5350
SEQUENCE LENGTH:259
SEQUENCE TYPE:nucleic acid
TOPOLOGY:linear
CLONE:HUMGS06381
SEQUENCE DESCRIPTION:
GATCAGAACA TGAACTTTTT TTTTAAAAGA AGATTTGAGC ATTTTCCTGT AATCACATCA 60
AAATNATGTT TTCTGTGTAA AGCGAGATAC ATATTNCTNA TAATGCAGCA TTGTGAGAAG 120
TCAGTTCGGC CCACTGCACC ANCACTGTCG TATCCTTGTT AAAATGGTGT GTACCTTACA 180
ANTTATAATT TATGTGCCAG GTTCGTTTTG TNCTTAATTT GCTATTATTG TGCTGTGTNT 240
ANCCCCTGTN NNCTNGGTN 259

SEQ ID NO:5351
SEQUENCE LENGTH:65
SEQUENCE TYPE:nucleic acid
TOPOLOGY:linear
CLONE:HUMGS06382
SEQUENCE DESCRIPTION:
GATCTTATAA AGTACTTGTG TGGAGCTTTG ATTCCACAGA ATAAAAGATA TTTTGTGTGT 60
AGAAA 65

SEQ ID NO:5352
SEQUENCE LENGTH:226
SEQUENCE TYPE:nucleic acid
TOPOLOGY:linear
CLONE:HUMGS06383
SEQUENCE DESCRIPTION:
GATCTCAGCC CAGGACAAGG TGGGAATNAG TCAAGCCTGG ACTCTGGCCC CCCTGCCTGG 60
CCAGTAAGAA GGGCAAAGTC CAAGGGGAGG GATGAGGNAG GGGCCAGATG GGGTCCTGGA 120
GGAAGAATTN CCTGGCAAAA GCCATTGGAG CTTGTNTGTG TGTCTTTGGT GATGACATNT 180
GTTGTGAGGG TAGATGGGAA CCATNTTAAA AGGATGAAAT GTGAAA 226

SEQ ID NO:5353
SEQUENCE LENGTH:305
SEQUENCE TYPE:nucleic acid
TOPOLOGY:linear
CLONE:HUMGS06384
SEQUENCE DESCRIPTION:
GATCCTTTTG TAAGGNCCTT CCTGAAGAAG AAAGGTGGCA AATACAGTNT TCAGTTCAAG 60
TTGCCCGACG TGTATGGTGT ATTCCAGTTT AAAGTGGATT ACAACCGGCT AGGCTACACA 120
CACCTGTACT CTTCCACTCA GGTATCCGTG CGGCCACTCC AGCACACGCA GTATGAGCGC 180
TTCATCCCCT CGGNCTACCC CTACTACGGC AGCGGCTTTC TNCATGATGC TGGGGGCTCT 240
TTCATCTNTC AGCATCGGGC NGGCTTTGCA CATGNAGGGN GAATGNTGGA NGTCCGANTT 300
TTNGN 305

SEQ ID NO:5354
SEQUENCE LENGTH:356
SEQUENCE TYPE:nucleic acid
TOPOLOGY:linear
CLONE:HUMGS06385
SEQUENCE DESCRIPTION:
GATCTACATT CTTCAAAAAA AAAAATTGGC TTAACTGTTT CATCTTNAAG TAGCATTTTG 60
CTGCCATTTG TATTGGGCTG AAGAANTCAC TATTGTGTAT ATACTCAAGT CTTTTNATTT 120
TCCCNCTTTT CATAAATNCT CTTGGNCATN ATTGGGCTTG CAGAGTTCCC TTATNCTGGG 180
GATTACAATG CTTTTATCGT TTCAGGCTTC ATTTNNGCTT CAAANCAAGC TGGGCACACT 240
GTTAAANCAT GATTTTGCAG ANCCTTTGGT TTTGGACAGT TTCATTTTTT NGGATTTGGG 300
ATAGATTNCA TAGGAGTATG GAGTATGCTG TAAATAAAAA TNCANGCTAG TGCTTN 356

SEQ ID NO:5355
SEQUENCE LENGTH:74
SEQUENCE TYPE:nucleic acid
TOPOLOGY:linear
CLONE:HUMGS06386
SEQUENCE DESCRIPTION:
GATCTTTAAC AGCTCCTTTA CTTGCNCTTT GTAAAAAAAA ATAAAATTAG NTTGAATTAT 60

TCTACCTTTG TAAA 74

SEQ ID NO:5356
SEQUENCE LENGTH:201
SEQUENCE TYPE:nucleic acid
TOPOLOGY:linear
CLONE:HUMGS06387
SEQUENCE DESCRIPTION:
GATCCTGCTG GCCGAGCTCG AGCAGCTCAA GGGCCAAGGC AAGTCGCGCC TGGAGCAGCA 60
GAATAAGNTC CTTAACTTAA AATAAATTAA TTTTTTCAAA AAACATAAAT CTGGAACTGT 120
TGTTTCTATA TTTNATAACA AATACAGTAT ATNTNATNTA TAAGCCATGG TCTACTGATA 180
CTGTATGAGG ACTTNCCTGA N 201

SEQ ID NO:5357
SEQUENCE LENGTH:409
SEQUENCE TYPE:nucleic acid
TOPOLOGY:linear
CLONE:HUMGS06388
SEQUENCE DESCRIPTION:
GATCAGAACG TTAACATATG AACCTGCAGA AATCTGGTAA GACTTAAATT CAGTGTGAGG 60
AATAACTCTA GTTCTCTCCT ATGAGCATTT CCTAAAAGCC ATCTGATTTG GCATTCTTAC 120
TGGAGCTGCA GACAGAAATC TACAAAGNCN NAAGTAAACA AAATTAAGTT ATTATTCCAC 180
TGTTAGGAAT GGAAATAAAC TTGTGAAGTC TGTTTATTTT GAAGTATTGG TGAACTAGGC 240
TTGCTAATNG GGAGCTGCAG CAGTTTGTGT TTACTCCAGT TCATCAGCTT AGGTCATTTG 300
AAAGNTATAA GAGCTTAAGG CAAGAAAGGA ATACCATGGG ATTCTATTTN AGGGCCACCA 360
GNCCATTCTT GGNAAGCAGC TCCNGTTTGG TTTTCCAGCT GTCAAACTN 409

SEQ ID NO:5358
SEQUENCE LENGTH:175
SEQUENCE TYPE:nucleic acid
TOPOLOGY:linear
CLONE:HUMGS06389
SEQUENCE DESCRIPTION:
GATCTGCCCG TNTCGGCCTC CCAAAGTGCT GGGATTACCG GCATGAGTCA CTGCGCCTGG 60
CCGACAAAGG CTTTGATATC AGAATGAACT GTCAAGGGAG GTGCTGGAGA GGGATTAACC 120
TGTGCAGAGN CCAANNGGTA TGGAANNAAA ANTGGGATTG GGACTAGGTG NTCTN 175

SEQ ID NO:5359
SEQUENCE LENGTH:104
SEQUENCE TYPE:nucleic acid
TOPOLOGY:linear
CLONE:HUMGS06390
SEQUENCE DESCRIPTION:
GATCAGGCTC AGGCGGCCCC ACTCACCCAC AGCATCCGCC GCCACCCCTT CGGGTGTGAG 60
CGCTCAATAA AAACAACACA CTATAAAGTG TTTTTAAATC CAAA 104

SEQ ID NO:5360
SEQUENCE LENGTH:106
SEQUENCE TYPE:nucleic acid
TOPOLOGY:linear
CLONE:HUMGS06392
SEQUENCE DESCRIPTION:
GATCTTTCCC AAGAGAAGGG GAAGCACTCG TGTGCAACAG ACAAGTGACT GTATCTGTGT 60
AGACTATTTG CTTATTTAAT AAAGACGATT TGTCAGTTAT TTTAAA 106

SEQ ID NO:5361
SEQUENCE LENGTH:382
SEQUENCE TYPE:nucleic acid
TOPOLOGY:linear
CLONE:HUMGS06393
SEQUENCE DESCRIPTION:
GATCTACGCT GCCTTCAAGG AAGTGCTGGG TTCGGGCATG CACCACCACC TCCAGAACAA 60
TGAGCTACTC CGTGACATCT TTGGCCTGGG CCCTGTGCTG TTGCTGGATG CCACTGCCCT 120
GAAGGCCTGC AAGGTTCCAC GCTTTGAGAA GCACCTGTAC AATGCTGCTG CCTTCAAAGC 180
CCGGACCAAG GCTCGAAGCC GTGTGCGGGA CAAGCGGGCA GACATCCTGT GAAGCAGGAC 240
CTGCTGAAGA GGAGACTTTC TATGCCCTTG GTCCGTATTT TTAACAGAAG ACAGTGCAAC 300
AACTGGTCTT CACCAGTATT TGTCACTTTA TTTTTTTTAA TGTCAAAACC AAAAACAGAC 360
ATGGGGTTGG GTAGCTGGGG GN 382

SEQ ID NO:5362
SEQUENCE LENGTH:354
SEQUENCE TYPE:nucleic acid
TOPOLOGY:linear
CLONE:HUMGS06394
SEQUENCE DESCRIPTION:
GATCCNATCA TTCCTTAAAC ATANTACCCT TNGTCTTGGA GTAGAATACT AAGTTAGAGT 60
TAGTGGATTT CTAGTTTAGG AGAGGAGCTC AAAACTATAA TCTNTAACAA ATTGAAAAAT 120
GAAATAGGGT GTTTTCCCTT TTTGTGCACA CCTACATNAC CTTAAGAAAT NNCCTTCCAT 180
AGACAGCTGC CTCAAAGGGA AATCCTCTTT AAACCGTAGT TGGCGCAGAG GTCAGTCCTA 240
GTCGGAGCTT AGGAGGTGCG GNGACGCTCA CATCGTCTGG ACTTGAGTCG NCACTGGATT 300
GTGGCANCAN NTTTNGCTCA TGNGTCAAAA ATTTNGGCAA TTTCTTTTGG ATTN 354

SEQ ID NO:5363
SEQUENCE LENGTH:79
SEQUENCE TYPE:nucleic acid
TOPOLOGY:linear
CLONE:HUMGS06395
SEQUENCE DESCRIPTION:
GATCCAGGGC CTCCCCGCCT TCCTTCTTCC TTTTTATATA CAATTTGTTA TTGTCAAATA 60
AAAGTAGGAA ATATTCAAA 79

SEQ ID NO:5364
SEQUENCE LENGTH:238
SEQUENCE TYPE:nucleic acid
TOPOLOGY:linear
CLONE:HUMGS06396
SEQUENCE DESCRIPTION:
GATCTTTGTG GCCAAGGAGC TGCTATAGCC TGGGGTGGGG TCATGCCCTC CTCTCCCATT 60
GTCCCTCTNC CCCATCCTCC AGCAGGGAAA ATGCAGCAGG GATGCCCTGG AGGTGGCTGA 120
GCCCCTGTCT AGAGAGGGAG GCAAGCCCTG TTGACACAGG TCTTTCCTAA GGCTGCAAGG 180
TTTAGGCTGG TGGCCCAGGA CCATCATCCT ACTGTAATAA AGATGATTGT NAAATAAA 238

SEQ ID NO:5365
SEQUENCE LENGTH:321
SEQUENCE TYPE:nucleic acid
TOPOLOGY:linear
CLONE:HUMGS06397
SEQUENCE DESCRIPTION:
GATCCTTGTC CGTGGCAACC TTAAACGTGT GCTGCAGTGC TGATGTATTT ATAGGCTTAG 60
CATGTCTGCT TTATTAGTTA TCCTGGAAAG AATGCTATTC AGTTTTTTTT TTAATGTCTA 120
CATCANGGAA TGAATGCTAA ATNAGCCCAT TTAGCAGCCT GAAATCAAAT NCTGCATTTN 180
TTTTTTNAAA CTCTTGGTAG CAAAAGAAAA AGCTGATTAA ANTATGATTG TGTTACAATG 240
ACTAAAGGGT TANCTNCTCA TTAATTCTAA ATNCTTCAAA ATGNACAAGC ATCATAATAA 300
AGGCTTTTGA TGNGAANTAA A 321

SEQ ID NO:5366
SEQUENCE LENGTH:409
SEQUENCE TYPE:nucleic acid
TOPOLOGY:linear
CLONE:HUMGS06398
SEQUENCE DESCRIPTION:
GATCTACAAG TCAGTGCCCT GTATGCTCTC CAGGTGCACT GCTATAACAG CAACTTCCCA 60
AAAGGCATGT TACTTCGCTT TTTTGTGCAC TTCTATGACA TGGAAATTAT TGAAGAAGAA 120
GCTTTCTTGG CTTGGAAAGA AGATATAACC CAAGAGTTTC CGGGAAAAGG CAAGGCTTTG 180
TTCCAGGTGA ATCAGTGGGC TAACCTNGTT AGAAACTGCN GAAGAAGAAG AATCAGAGGA 240
NGAAGCTGAC TAAAGAACCA GCCAAAGCCT TAAATTGTGC AAAACATACT GTTTGCTATG 300
ATGTAACTGC ATTTGACCTA ACCACTNGCG NAAANTNATT CCGNTGTAAT GTNTTCACAA 360
TATTTNAAAG CAGANGCACG TCAGTTTNGG NTTTCCTTCT GCANAAAGN 409

SEQ ID NO:5367
SEQUENCE LENGTH:178
SEQUENCE TYPE:nucleic acid
TOPOLOGY:linear
CLONE:HUMGS06399
SEQUENCE DESCRIPTION:

GATCCTCAGT ATAGCCGGTG AACCCTGATA CCAGGAGCCT GGGCCTCCCT GAACCCCTGG 60
CTTCCAGCCA TCTNATCGCC AGCNTCCTCC TGGACCTNTT GGCCCCCAGC CCCTTCCCNA 120
CACAGCCCNA GAAGNGTCCC AGAGCTGACC NCACTCCAGG ACCTAGGNCC AAGCCTTN 178

SEQ ID NO:5368
SEQUENCE LENGTH:75
SEQUENCE TYPE:nucleic acid
TOPOLOGY:linear
CLONE:HUMGS06400
SEQUENCE DESCRIPTION:
GATCATTGTA ATATATTTAN TTGTATATNA AGCATGACTA CTTTAAGTNT TAAATAAACT 60
GCAATAATCC TTAAA 75

SEQ ID NO:5369
SEQUENCE LENGTH:354
SEQUENCE TYPE:nucleic acid
TOPOLOGY:linear
CLONE:HUMGS06401
SEQUENCE DESCRIPTION:
GATCAGTGNC TGGCACCAGA CACTTCTGGG GATGGTACAG GGTGTGACAA CATGACCTGC 60
ATCATCATTT GCTTCAAGCC CCGAAACACA GCAGAGCTCC AGCCAGAGAG TGGCAAGCGA 120
AAACTAGAGG AGGTGCTCTC TACTGAGGGG GCTGAAGAAA ATGGCAACAG CGACAAGAAG 180
AAGAAGGCCA AGCGAGACTA GCAGTCATCC AGACCCCTGC CCACCTAGAC TGTTTTCTGA 240
GCCCTCCGGA CCTGAGACTG AGTTTTGGTC TTTTTCCTTT AGCCTTAGCA GTGGGTATGA 300
GGTGTGCAGG GGGAGCTGGG TGGCTTCACT CCGGCCATTC CAAAGAGGGG TCTN 354

SEQ ID NO:5370
SEQUENCE LENGTH:93
SEQUENCE TYPE:nucleic acid
TOPOLOGY:linear
CLONE:HUMGS06402
SEQUENCE DESCRIPTION:
GATCACTGGA ATGTGGGGAT TCTGAAACAG AAATGAAACT GTCCTTTTGA CAACTCTCTT 60
ATATAATAAA GTATCACCGG CTTGTGTATG AAA 93

SEQ ID NO:5371
SEQUENCE LENGTH:80
SEQUENCE TYPE:nucleic acid
TOPOLOGY:linear
CLONE:HUMGS06403
SEQUENCE DESCRIPTION:
GATCATGCAT TATCTTTATA ATTCAAAAGA ATTTATATAT ATGTAAAGGA GGAGAAAATA 60
AAAAAAATTA TAATCTTAAA 80

SEQ ID NO:5372

SEQUENCE LENGTH:292
SEQUENCE TYPE:nucleic acid
TOPOLOGY:linear
CLONE:HUMGS06404
SEQUENCE DESCRIPTION:
GATCATNTTG ATAATGCTCG GAATATTTTN AAATGTCCAT TCCGCTGTGT TGATTGAGGA 60
CGTTCCCTTC ACGGAGAAAG ATTTTAAGAA TGGCCCCCAG AACATATACA ACCTTTACGA 120
GCAAGTCAGC TACAACTGTT TCATCGCTGC AGGCCTTTAC CTCCTCCTCG GAGGCTTCTN 180
TTTCTGNCAA GTTCGGCTCA NTAAGNGCAA GGANTACATG GTGCGCTAGG GCCCCGGNGN 240
GTTTCCCCGN TCCAGCCNCT TCTCTATTTA ANGACTGCNN GCACCGTGTC AN 292

SEQ ID NO:5373
SEQUENCE LENGTH:194
SEQUENCE TYPE:nucleic acid
TOPOLOGY:linear
CLONE:HUMGS06405
SEQUENCE DESCRIPTION:
GATCCTNCTG GCCGAGCTCG AGCAGCTCAA GGGCCAAGGC AAGTCGNGCC TGGGGGACCT 60
CTACGAGGAG NAGATGCGGG ACTGCGCCGG NAGGTGGACC AGCTAACCAA CGACAAAGCC 120
CGCNTCGAGG TGGAGCGCGA CAACCTGGCC GAGGACATCA TGCGNCTCCG GGAGAAATTN 180
CAGGNNGGAG ATTN 194

SEQ ID NO:5374
SEQUENCE LENGTH:72
SEQUENCE TYPE:nucleic acid
TOPOLOGY:linear
CLONE:HUMGS06407
SEQUENCE DESCRIPTION:
GATCCTGGAT TCACTCACTC ACTCATTCCT TCACTCATCC AGCCACCTAA AAACATTTAC 60
TGACCATGTA AA 72

SEQ ID NO:5375
SEQUENCE LENGTH:219
SEQUENCE TYPE:nucleic acid
TOPOLOGY:linear
CLONE:HUMGS06408
SEQUENCE DESCRIPTION:
GATCACGCAC GAAGTGTACG AACTGACCCA GATTATCGCC GACGTGTCCC AGGACCCCAC 60
GTTGCCGCGG ACCGAGGACC ACCCGTGCCA AAAGTGCGGC CACAAGGAGG CTGTGTTCTT 120
CCAGTNACAC AGTGCGCGGG NCGANGACGN CATGCGCCTT TACTACGTGT GNACAGCCCC 180
ACANTGNGGC CACCGCTGGA CCGAGTGNCC TNCTNTGTN 219

SEQ ID NO:5376
SEQUENCE LENGTH:65
SEQUENCE TYPE:nucleic acid

TOPOLOGY:linear
CLONE:HUMGS06409
SEQUENCE DESCRIPTION:
GATCCAACAA GAACATTTCC AAAAGTATCT AGGTGTTCTC AAATAAAAAG CTTTCTTTGC 60
ACAAA 65

SEQ ID NO:5377
SEQUENCE LENGTH:97
SEQUENCE TYPE:nucleic acid
TOPOLOGY:linear
CLONE:HUMGS06410
SEQUENCE DESCRIPTION:
GATCTGCTAA TGCTTTTTAG GAGTCTGCCT GGAAACTTTG ACATGGTTCN TGTTTTTACT 60
CAAAATCCAA TAAAACAAGG TAGTTTGGCT GTGCAAA 97

SEQ ID NO:5378
SEQUENCE LENGTH:206
SEQUENCE TYPE:nucleic acid
TOPOLOGY:linear
CLONE:HUMGS06411
SEQUENCE DESCRIPTION:
GATCCCAGAG AGCANCTTCA GTGACAAACA TATCCTTTCA AGACAGAAAG AGACAGGAGA 60
CATGAGTCTT TGCCGGAGGA AAAGCAGCTC AAGAACACAT GTGCANTCAC TGGTGTCACC 120
CTGGAATAGG CAAGGNATAA CTCTTCTAAC ACAAAATAAG TGTTTTATGT TTGGAATAAA 180
GTCANCCTTG TTTCTACTGT TTTAAA 206

SEQ ID NO:5379
SEQUENCE LENGTH:84
SEQUENCE TYPE:nucleic acid
TOPOLOGY:linear
CLONE:HUMGS06412
SEQUENCE DESCRIPTION:
GATCACTTAA ATGATTTTNT GGTGATTCGT GCCATTGCTT TTTTATTTAA AAGAAAATTT 60
TGTAATTAAA TGCCTTTTTC TAAA 84

SEQ ID NO:5380
SEQUENCE LENGTH:127
SEQUENCE TYPE:nucleic acid
TOPOLOGY:linear
CLONE:HUMGS06413
SEQUENCE DESCRIPTION:
GATCACACCA GAGTACATGT CTCTGCCTCT NTTTTCAGTG TGGCTTTGGA CAGGAATATA 60
TGAATAANTC ACTGCCATAC AGGTTTTCCA ATACACAAGT GCTAGAAAAT ACACACAATT 120
CCCCAAA 127

SEQ ID NO:5381
SEQUENCE LENGTH:229
SEQUENCE TYPE:nucleic acid
TOPOLOGY:linear
CLONE:HUMGS06414
SEQUENCE DESCRIPTION:
GATCAAAAAT GCAACTGAGA AAATNATGGC TCTTGTTGCT GAGCTGTCCA TGAAACAAGC 60
CCTAACCATT GAACTCCAAA AGGAAGTCAG GGAGAAAGAA GACTTCATCT TCACTTGCAA 120
TTCCAGGATA GAAAAAGGTC TGCCACTCAA TAAGGAAATT GAGAAAGANT GGTTGAAAGT 180
CCTTCGAGAT GAAGAAATGC ACGCCTTGGC CATCGCTGAA AAGTCTCAN 229

SEQ ID NO:5382
SEQUENCE LENGTH:71
SEQUENCE TYPE:nucleic acid
TOPOLOGY:linear
CLONE:HUMGS06415
SEQUENCE DESCRIPTION:
GATCCCTAAC TGTTCCCTTA TCTGTTTTCT ACCTCCTCCT TTGTTTAATA AAGGCTGAAG 60
CTTTTTGTAA A 71

SEQ ID NO:5383
SEQUENCE LENGTH:235
SEQUENCE TYPE:nucleic acid
TOPOLOGY:linear
CLONE:HUMGS06416
SEQUENCE DESCRIPTION:
GATCACTTAC AAATTTTTTG AATAACACAA AATCTCATTC TACCTGCAGT TTAATTGGAA 60
AGATGTGTGT GTGAGAGTAT GTATGTGTGT GTGTGTGTGT GTGTGTGTGC GCGTGCACGC 120
ACGTCTTGAG CAGTCAGCAT TGCACCTNCT NTGGAGAAGG GTATTCCTTT ATNAAAATCT 180
TCCTCATNNN NGNTTTGCTT TCAGTTGGTT TTCAATTTNC TCACTGGCCA GAGAN 235

SEQ ID NO:5384
SEQUENCE LENGTH:236
SEQUENCE TYPE:nucleic acid
TOPOLOGY:linear
CLONE:HUMGS06417
SEQUENCE DESCRIPTION:
GATCTTCCCT CTTCTGAACA GCAACTCCCC CCAACTCATA CACTGCCGTT TTCAGGGTAA 60
GCGGCCTTCA CCCCTAAGTG AAAATGTTAG TGAACTAAAG GAAGGAGCCA TTCTTGGAAC 120
TGGACGACTT CTGAAAACTG GAGGACGAGC ATGGGAGCAA GGCCAGGACC ATGACAAGGA 180
AAATCAGCAC TTTCCCTTGG TGGAGAGCTA GGCCCTGCAT GGCCCCAGCA ATGCAN 236

SEQ ID NO:5385
SEQUENCE LENGTH:135
SEQUENCE TYPE:nucleic acid

TOPOLOGY:linear
CLONE:HUMGS06418
SEQUENCE DESCRIPTION:
GATCCAGTAC TTGGCAGCCA TAGTTTAGAC AATATTGTTC AGTGCTGTGT TGCTNNCATG 60
TTAACAACAA AACCTTTTAG AGGNCCCACA AATCATNGGT ATNGGACACA GGTCCGAGGN 120
ATTCCAGAGC ATCAN 135

SEQ ID NO:5386
SEQUENCE LENGTH:261
SEQUENCE TYPE:nucleic acid
TOPOLOGY:linear
CLONE:HUMGS06419
SEQUENCE DESCRIPTION:
GATCAGCTCG CACAGAGGTT GTCTGCAAGC AGTGTGAAGC TCATCTAGGT CACGTGTTTC 60
CTGATGGACC TGGGCCCAAT GGTCAGAGGT TTTGCATGAA CAGTGTGGCT TTGAAGTTCA 120
ANTCAAGGGA ACACTGACCA TCTTCAAGAG TCCCGTTCCC TTGCCACCCC TTCACGTGCA 180
CCCTCAATTT CCACAATTCA CTTGAATGAC TTGTNTTATT NGCAATAAAA CTGGGCTGAA 240
TTTGCTGCTG TCTCCAAANA N 261

SEQ ID NO:5387
SEQUENCE LENGTH:300
SEQUENCE TYPE:nucleic acid
TOPOLOGY:linear
CLONE:HUMGS06420
SEQUENCE DESCRIPTION:
GATCATAGAC ATGATTATAT GTGAGTATCA TTAATCATTA GTTTATAGCA NTTNCTCTTT 60
ATTCCAATAT TATAATAATC CNCACTCTAC AATCATAACC TAGGAAAAAC CAGGCCATAC 120
AGAGATAGGA GCCGAGGGGN CATAGTGCGA AGTGGCCAGA AGACAAGAGT GTGAGCCTTC 180
TCTTATGCCT GGACAGGGCC ACCAGAGGGC TTGGTCTAGC AGTAACACCA GTGTCTGGGA 240
AGATGCCTGT TGCAAAGTGG ACCATGGTCT AGCGGGGGGC ATCAGTGTCA NGGTANNGAN 300

SEQ ID NO:5388
SEQUENCE LENGTH:299
SEQUENCE TYPE:nucleic acid
TOPOLOGY:linear
CLONE:HUMGS06421
SEQUENCE DESCRIPTION:
GATCAACAGT GGCAATGGAG CTGTGGAGGA CAGAAAGCCA AGTGGACTCA ACGGAGAGGC 60
CAGCAAGTCT CAGGAAATGG TGCATTTGGT GAACAAGGAG TCGTCAGAAA CTCCAGACCA 120
GTTTATGNCA GCTGATGAGA CAAGGAACCT GCAGAATGTG GACATGAAGA TTGGGGTGTA 180
ACACCTACAC CATTATCTTG GAAAGAAACA ACCGTTGGAA ACATAACCAT TACAGGGAGC 240
TGGGACACTT AACAGATGCA ATGTGCTACT GATTGTTTCA TTGCGGAATC TTTTTTGAN 299

SEQ ID NO:5389
SEQUENCE LENGTH:134

SEQUENCE TYPE:nucleic acid
TOPOLOGY:linear
CLONE:HUMGS06422
SEQUENCE DESCRIPTION:
GATCCACCTG CCTCGGCCTC CCAAGGTGCT GGGATTGCAG GTGTGAGCCA CTGTGCTCGG 60
CCTTATTTAT ATTTTATATA TTGTTGATAT GGTTGAATTT ATGTATGCCA TTAAATTATT 120
TGTTTCTATT TAAA 134

SEQ ID NO:5390
SEQUENCE LENGTH:303
SEQUENCE TYPE:nucleic acid
TOPOLOGY:linear
CLONE:HUMGS06423
SEQUENCE DESCRIPTION:
GATCAACGTC CTCCATGAGC ATGGNATAGT CCACCGTGAC ATTAAAGGTG CCAATATCTT 60
CCTTACCTCA TCTGGTTTAA TCAAACTGGG AGATTTTGGA TGTTCAGTAA AGCTCAAAAA 120
CAATGCCCAG ACCATGCCTG GTGAAGTGAA CAGCACCCTG GGGACAGCAG CATACATGGC 180
ACCTGAAGTC ATCACTCGTG CCAANGGAGA GGGCCATGGG CGTGCGGCCG ACATCTGGAG 240
TCTNTGGGTG TGTTGTCATA GAGATNGTGA CTTGGCANGA GGCCTTGGCA TGAGTATGAG 300
NAN 303

SEQ ID NO:5391
SEQUENCE LENGTH:147
SEQUENCE TYPE:nucleic acid
TOPOLOGY:linear
CLONE:HUMGS06424
SEQUENCE DESCRIPTION:
GATCCAGAAG AAGTGACCTG AAGTTTCTGT GCAACACTNC ACACTAGGCA ATGCCATTTC 60
AATGCATTAC TAAATNACAT TTGTAGTTCC TAGCTCCTCT TAGGAAAACA GTTCTTGTGG 120
CCTTCTATTA AATAGTTTGC ACTTAAA 147

SEQ ID NO:5392
SEQUENCE LENGTH:128
SEQUENCE TYPE:nucleic acid
TOPOLOGY:linear
CLONE:HUMGS06425
SEQUENCE DESCRIPTION:
GATCAATTCG ACAGACAGAT GGNGTGTATG CCCCTCCCAN GTTTGACTTC ACACACACTC 60
ATAACTTTCC AAATGAAGCC CCACAGTATA GCGCATATTT TNGATATTTT TGTGAATNCC 120
ANNAGGAN 128

SEQ ID NO:5393
SEQUENCE LENGTH:122
SEQUENCE TYPE:nucleic acid
TOPOLOGY:linear

CLONE:HUMGS06426
SEQUENCE DESCRIPTION:
GATCCCTAGG AGCTGNCCTN CTTCCAGCCA NTGCCAGGAA CCAGCACTCC ATGAAGATAA 60
ACTCTGGGCC CGACCACCAG CAGGNCCTGA CGACCTGAGG TGACCCTNGG CCTTTCCCCC 120
NN 122

SEQ ID NO:5394
SEQUENCE LENGTH:57
SEQUENCE TYPE:nucleic acid
TOPOLOGY:linear
CLONE:HUMGS06427
SEQUENCE DESCRIPTION:
GATCACAAAT TTTGATATGT GCTATACAGG AAATAAAGTG TTATGATTGA GAATAAA 57

SEQ ID NO:5395
SEQUENCE LENGTH:359
SEQUENCE TYPE:nucleic acid
TOPOLOGY:linear
CLONE:HUMGS06428
SEQUENCE DESCRIPTION:
GATCTGAAAT CTTCAGTAGG TTGATGTGGT TTCCTCTGCA GTGATTTTCC TAGGAAGTTC 60
AAATTTGACA GCGAGTTCAG CTCAGCTGTG GCCCTCTGCC CTTCCAGCTG TGCCTAGCAA 120
GCAAAACCCA GGNAAGAAGC AGTNGCCTCC TGGCCTTACA TACAGAATGC CTGGACAAGA 180
GAGAACTTGC TGCGGNCTGC TTTGTATTTT AAAACACAGC TTGNGAGTTC AGAGTTGGTG 240
GTTTGCTCAC TTAGCGGTTG TTANGNTGGC TTGAAAAGTT TTCATTATGA TACACTGGGT 300
ACCCTGGGCT TNGAATGTTN NCAACTTNGG NNNATCTNAT GGTTACCTNT GATTANNAN 359

SEQ ID NO:5396
SEQUENCE LENGTH:246
SEQUENCE TYPE:nucleic acid
TOPOLOGY:linear
CLONE:HUMGS06429
SEQUENCE DESCRIPTION:
GATCTACCCA CCTCAGCCTC CCAAAGTGCT GGGATTACAG GCATGAGTCA CCACGCCCGG 60
CCCTGNTTAT CAATCTTCAA TATTTTGTAT TTTGAGCACT TTGTTGGATT TGTTATTTAG 120
CACATGTATA CATTGTTTTA TTATAATATT GTGTGTAATT TATTTCTGAT TAATTTTATA 180
TCCTTCCTTC ACATTTCAAA TACTTCTAAT TAAAAGCAGA CTGTTCAAAA AANNNNNGNN 240
NGNNNN 246

SEQ ID NO:5397
SEQUENCE LENGTH:133
SEQUENCE TYPE:nucleic acid
TOPOLOGY:linear
CLONE:HUMGS06430
SEQUENCE DESCRIPTION:

GATCTGGCTG AGGGCTCAGG ACACAGGCCC AGCCACCCCC AGGGGCCTCC ACAGNCCGCT 60
GCATGACAGC NATACAGTAC TTAAGTGTCT GTNTAGACAA CCAAAGANTA AATGATTCAT 120
GGTTTTTTTT AAA 133

SEQ ID NO:5398
SEQUENCE LENGTH:119
SEQUENCE TYPE:nucleic acid
TOPOLOGY:linear
CLONE:HUMGS06431
SEQUENCE DESCRIPTION:
GATCCTGAAC CAAATTTATA GCAATATAAT TAGTGTTTNT CTAAGGCATT ACTCATCAAA 60
AAAATTGCTG CAGTCTTTAC AGTTGAATAA ATAAAAACAA CTGCATAAAT ATGCCCAAA 119

SEQ ID NO:5399
SEQUENCE LENGTH:80
SEQUENCE TYPE:nucleic acid
TOPOLOGY:linear
CLONE:HUMGS06432
SEQUENCE DESCRIPTION:
GATCACCACC TGTAGAAACT GTTCTTCCTC TGTGAAACTA ACGGCCTGCT GGTAATAAAA 60
GTAGCTACCA TTTCTTGAAA 80

SEQ ID NO:5400
SEQUENCE LENGTH:130
SEQUENCE TYPE:nucleic acid
TOPOLOGY:linear
CLONE:HUMGS06433
SEQUENCE DESCRIPTION:
GATCACTCAT TGCTAATTTT GTCCTGTACA GCTTATGTAA TATTTNNATG GTGGAGACGG 60
ACTCTGTGTG CTCAGGGCCT TGTCTCTAGG AAGATTTTGT CAATTCCAAA TACAGTTTTG 120
AAGATTCAAA 130

SEQ ID NO:5401
SEQUENCE LENGTH:379
SEQUENCE TYPE:nucleic acid
TOPOLOGY:linear
CLONE:HUMGS06434
SEQUENCE DESCRIPTION:
GATCAACTGA TAAGTTGCCT TATGTCACCA TGGGTTCTGG CTCCTTGGCA GCAATGGCTG 60
TATTTAAAGA TAAGTTTAGG CCAGACATGG AGGAGGAGGA AGCCAAGAAT CTGGTGAGCG 120
AANCATCGCA GCTGGCATCT TCAACGACCT GGGCTCCGGA AGCAACATTG ACCTCTGCGT 180
CATCAGCAAG AACAAGCTGG ATTTTCTCCG NCCATACACA GTGCCCAACA AGAAGGGGAC 240
CAGGCTTGGC CGGTACAGGT TGTGAGAAAG GGGACTACTG CAGTCCTCAC TGTGAAANTN 300
ACTCCTCTNG AGATTTGAGG TTNTTGGAAG AAACANGTCC AAACAATGGA CCACTTCCTG 360
AATTGGCATC ANTGGTNNN 379

SEQ ID NO:5402
SEQUENCE LENGTH:297
SEQUENCE TYPE:nucleic acid
TOPOLOGY:linear
CLONE:HUMGS06435
SEQUENCE DESCRIPTION:
GATCTGTAGT AAACAATGGT GATTTTATTT ATTTTNACTC TCTGGAAAAG GAGATAATAC 60
AATTCCAGAA AGTGAACTCA TATTTCTAAG GTTAAGATTC CCTTTTATTG CACCTAGAAT 120
AGTGCTATGC ACAGAGCGGG TGCTTGAGTT GTTGTCGTTT TNTGTTTGTT TTTTAAATGT 180
AAACTGGTAA ATNTTGTGCT TATCTTCAAG GCTAGCTTAN GTATAAAATN GTTTTTTAAA 240
CACTTGNAAA ATTAAAGGAT TTGTTTTAAN NANCGANNAG GGGNGGTGGT TNGGTNN 297

SEQ ID NO:5403
SEQUENCE LENGTH:294
SEQUENCE TYPE:nucleic acid
TOPOLOGY:linear
CLONE:HUMGS06436
SEQUENCE DESCRIPTION:
GATCAAATAG TTGCATAGCA GTATATGTNA ATTTGTGTGT TTTTAGCTGT GACACAGCTG 60
TGTGATTAAA AGGTATACTT TAGTAGACAT TTATAACTCA AGGATACCTT CTNATTTAAT 120
CTNTTCTTAT TTTTGTACTT TATCATGAAT GCTTTTAGTG TGTGCATAAT AGCTACAGTG 180
CATAGTTGTA GACATGGTAC ATTCTGGGGA AACAACATTT NTNTGTNGCC TTTACTGTTT 240
GATATACCAN ATNANAANAA AATTGGGTCT NATTNCTNAT GCTGGGGCAC CNTN 294

SEQ ID NO:5404
SEQUENCE LENGTH:103
SEQUENCE TYPE:nucleic acid
TOPOLOGY:linear
CLONE:HUMGS06437
SEQUENCE DESCRIPTION:
GATCCTCACC NCTTGGAATT AAAATNCTTG TTCACTCAGG CAAGCCTACA GTTTGCAACC 60
AAGTCTAGGG TTTCTGTTAA TCAANGNCGT TTTTTCANTT AAA 103

SEQ ID NO:5405
SEQUENCE LENGTH:60
SEQUENCE TYPE:nucleic acid
TOPOLOGY:linear
CLONE:HUMGS06438
SEQUENCE DESCRIPTION:
GATCTGCTGG ACATCATGCC CCATGTCATA GAGAATAAAG CTGATGATTG TACCAGTAAA 60

SEQ ID NO:5406
SEQUENCE LENGTH:91
SEQUENCE TYPE:nucleic acid

TOPOLOGY:linear
CLONE:HUMGS06439
SEQUENCE DESCRIPTION:
GATCATACCN ATAAAAATAA TNCCAAACAC CAAATATGAA TTTTATGAAT GTACACTTTG 60
TGCTTNACAT TAAAAGAAAN AAACACACAA A 91

SEQ ID NO:5407
SEQUENCE LENGTH:108
SEQUENCE TYPE:nucleic acid
TOPOLOGY:linear
CLONE:HUMGS06440
SEQUENCE DESCRIPTION:
GATCGTCGCC TGCTGGCTAA GACCAGGGAG CTAAAAACTT NAGGAAGGGA ACCTGCCTGG 60
TTGGNTGCTA CTTCTGATTC ATTGTCCTTN TCCCTNTCAT AAGTNCCN 108

SEQ ID NO:5408
SEQUENCE LENGTH:321
SEQUENCE TYPE:nucleic acid
TOPOLOGY:linear
CLONE:HUMGS06441
SEQUENCE DESCRIPTION:
GATCTTATGT TTAAAGAGAT TCTGTTATTA GCACCATGAA CTCGTACTAT GAAATNTTTA 60
AGCCTTTTAT TTTTCTAACT ATATTACTGT AGGACTGGAT ATTAGGTGTC ATATAGGAAA 120
CACAAAAGTT ATTGCTGTTT GCTAAAGCAA AGTAGCAGAA AATTTTGTAT ATGCAAAACT 180
GTTGAAGGNC CATAGAGAAA TGTGTACTAC TGACGGGGCT TTTACTAGGC TTCCTGCGTG 240
TGTANAAGTC GAGGTTTTGG CTTGGCATTC AGGGTGACAT GGTGGTGCTA AATTGTTTTC 300
CNTTTAAAAG GCNTTCTATT N 321

SEQ ID NO:5409
SEQUENCE LENGTH:390
SEQUENCE TYPE:nucleic acid
TOPOLOGY:linear
CLONE:HUMGS06442
SEQUENCE DESCRIPTION:
GATCCGCAGC AGAAGCTCTT CCCTTCTGTA TCCTATGTAT GCAGTGTGCT TTTNCTTGCC 60
AGCTTGGGGC ATTCTTGCTT AGACAGTCAG CATTTGTCTC CNCCTTTAAC TGAGTCATCA 120
TCTTAGTCCA ACTAATGCAG TCGATACAAT GCGTAGATAG AAGAAGCCCC ACGGGAGCCA 180
GGATGGGACT GGTCGTGGTT TGTGCTTTTC TCCAAGTCAG CACCCAAAGG TCANTGCACA 240
GAGACNCCGN GTGGGTGAGC GCTNGCTTCT CAAACGGNCG AAGTTGCCTC TTTTAGGNAT 300
CTCTTTTGGA ATTTGGGAGC ACGATGNCTC TNGAGTTTGA GCTATTAAAG NACTNCTTGG 360
CACATTAANA AAANANAAAT GNGGTNNGNN 390

SEQ ID NO:5410
SEQUENCE LENGTH:197
SEQUENCE TYPE:nucleic acid

TOPOLOGY:linear
CLONE:HUMGS06443
SEQUENCE DESCRIPTION:
GATCCCAGGG AAATATCAGC CTTGGGCAAC TGCAGTGACC AGGGGCACCG GCTGCCCACA 60
GGGAACACAT TCCTTTGCTG GGGTTCAGCG CCTCTNCTGG GGCTGGAAGT GCCAAAGCCT 120
GGGGCAAAGC TGTTTTTCAG CCACACTGAA CCCAATTACA CACAGCGGGA GAACGCAGTA 180
AACAGCTTTC CCACAAA 197

SEQ ID NO:5411
SEQUENCE LENGTH:104
SEQUENCE TYPE:nucleic acid
TOPOLOGY:linear
CLONE:HUMGS06444
SEQUENCE DESCRIPTION:
GATCTTCAAG TGAACATCTC TTGCCCTGAT TGAAGGCTTT GCCACATGCT GGAAGGCCCC 60
CTCCCAGGAA GATGGGGGTC ATTAAAGGAA ACTNAACATT GAAA 104

SEQ ID NO:5412
SEQUENCE LENGTH:244
SEQUENCE TYPE:nucleic acid
TOPOLOGY:linear
CLONE:HUMGS06445
SEQUENCE DESCRIPTION:
GATCACCTGA ACAATCTAGA TGTGGACAAA ACCATTGGGA CCTAGTTTAT TATTTGGTTA 60
TTGATAAAGC AAAGCTAACT GTGTGTTTAG AAGGCACTGT AACTGGTAGC TAGTTCTTGA 120
TTCAATAGAA AAATGCAGCA AACTTTTAAT AACAGTCTCT CTACATGACT TAAGGAACTT 180
ATCTATGGAT ATTAGTAACA TTTTTCTACC NNGNNGTCCG TAATAAACCA TACTTGCTCG 240
TAAA 244

SEQ ID NO:5413
SEQUENCE LENGTH:139
SEQUENCE TYPE:nucleic acid
TOPOLOGY:linear
CLONE:HUMGS06446
SEQUENCE DESCRIPTION:
GATCTNAGAA AANGCACCAC GTNAGCTTAC TAANTNATAA TATCTGTTTC TACTACGGAT 60
TTAGGCAACA GGACCTGTAC ATTGTCACAT TGCATTATTT TTCTTCAAGC GTTAATAAAA 120
GTTTTAAATA AATGGCAAA 139

SEQ ID NO:5414
SEQUENCE LENGTH:196
SEQUENCE TYPE:nucleic acid
TOPOLOGY:linear
CLONE:HUMGS06447
SEQUENCE DESCRIPTION:

GATCGGGGTC CATGGGCCCA CCACCCCCTG CAAGGAGGTC TCCATGAAGC ATGTAGGCAA 60
CCAGCAATAC AACGTCACAT ACGTCGTCAA GGAGAGGGGC GATTATNTNC TGGCTGTGAA 120
GTGGGGGGAG GAACACATAC CTGGCAGCCC TTTTCATGTC ACAGTGCCTT AAAACAGTTT 180
TCTCAAATCC TGGAAA 196

SEQ ID NO:5415
SEQUENCE LENGTH:69
SEQUENCE TYPE:nucleic acid
TOPOLOGY:linear
CLONE:HUMGS06448
SEQUENCE DESCRIPTION:
GATCCATTAA TCTGCTAATT GGTTGCCTAT GAAATCACAT TGTCTGTTAT TAAAGCTTTT 60
TACCATAAA 69

SEQ ID NO:5416
SEQUENCE LENGTH:119
SEQUENCE TYPE:nucleic acid
TOPOLOGY:linear
CLONE:HUMGS06449
SEQUENCE DESCRIPTION:
GATCTAGTAA AGTGTTTTAG AACCCCTTTT TATCCCATGC ACCATTCAGT AAACATAAAA 60
ATCACAATTC TGCTAATGTC ATTTGGAACT TCAAAATAAA TATCTTGTCT AAAAACAAA 119

SEQ ID NO:5417
SEQUENCE LENGTH:308
SEQUENCE TYPE:nucleic acid
TOPOLOGY:linear
CLONE:HUMGS06450
SEQUENCE DESCRIPTION:
GATCATTGCA TGAATAGAAT CAAGGAGCTC ACTCAGAGTG AACTTNAATT ATNACTTTNN 60
AGGCTCATTT GTACTCTCTT CCCCTCTCAT CGTCATGGTC AGGCTCTGAT ACCTGCTTTN 120
AAAATGGAGC TAGAATGCTT GCTGGATTGA AAGGGAGTGC CTATCTATAT TTAGCAAGAG 180
ACACTATTAC CAAAGATTGT NGGTTAGGCC AGATTGACAC CTATTTNTNG NCNNTATGCG 240
TATATTTNCC TGTGCTATAT ATGAAAANTA ATTGCATGAT TTCTCATTCC TGNGTCATTT 300
CTCAGNGN 308

SEQ ID NO:5418
SEQUENCE LENGTH:293
SEQUENCE TYPE:nucleic acid
TOPOLOGY:linear
CLONE:HUMGS06451
SEQUENCE DESCRIPTION:
GATCTGAAGG GAGAAGGTGA TATTCATGAA AATGTGGACA CAGACTTGCC AGGCAGCCTG 60
GGGCAGAGTG AAGAGAAGCC CGTGCCTGCT GCGNCTGTGC CCAGCCCGGT GGCCCCGGCC 120
CCAGTGCCAT CCAGAAGAAA TCCCCCTGGC GGCAAGTCCA GCCTCGTNTT GGGTTAGCTC 180

TGACTGTCCT GAACGCTGTC GTTCTGTCTG TTTCCTCCAT GCTTGTGAAC TGCACAACTT 240
GAGCCTGACT GTACATNTTT TGGATTTGTT TCATTANAAA GAAGCACTTT AAA 293

SEQ ID NO:5419
SEQUENCE LENGTH:180
SEQUENCE TYPE:nucleic acid
TOPOLOGY:linear
CLONE:HUMGS06452
SEQUENCE DESCRIPTION:
GATCCTGGNA CATTTCATAA GCTTGGGTAA AAAAATTTTT TGTTATNNTC CATGCACAAT 60
AAGGGAAATG TTTTAATTGG CTTTGNGCCT CCTGCTTTAC AACAGTTCTG GCCTCAAGTT 120
ACCAGGTAGC CTGAACTTTG NTTTTATTTT ACCATTAANT TGCTTTAATA CCATNGTAAA 180

SEQ ID NO:5420
SEQUENCE LENGTH:99
SEQUENCE TYPE:nucleic acid
TOPOLOGY:linear
CLONE:HUMGS06453
SEQUENCE DESCRIPTION:
GATCACTTGA GCCCANGTTG AGGTTGAGGC TGCAGTGAAC TATNATTGTA CCACTGCACT 60
TCAGCCTGGG CAACAAAGGA AGACCCCAGT TCTGGGAAA 99

SEQ ID NO:5421
SEQUENCE LENGTH:209
SEQUENCE TYPE:nucleic acid
TOPOLOGY:linear
CLONE:HUMGS06454
SEQUENCE DESCRIPTION:
GATCCCTGCA TTNCCTGAAG ATNTTTAAAC GTGAGAGTNT GGTAGGCAAA GCAGTCTGAG 60
AAAGAAATAG GAAATGCAGA AATAGGTTTT TTTTGGTTGC ATATAATCTT TGCNCTTTTT 120
AAGCTCTGTG AGCTCTGAAA TATATTTTNG GNTTACTTCA GTGTGTTTGA CAAGACAGCT 180
TGATATTNCT NTCAAACAAA TGACTNNGN 209

SEQ ID NO:5422
SEQUENCE LENGTH:246
SEQUENCE TYPE:nucleic acid
TOPOLOGY:linear
CLONE:HUMGS06455
SEQUENCE DESCRIPTION:
GATCCAGTGG ACAAAAGAGG GGAAACTATG GGAGTTCCCA ATTAACAATG AAGCAGGTTT 60
TGATGATGAT GGTTCAGANT TTCATGAACA TATATTTNTG GAGAAACACC TGGAGAGCTT 120
TCCAAAACAA GGACCAATTC GCCACTTCAT GGAGCTGGTG ACTTGTGGCC TTTCCAAAAN 180
CCCATATCTT AGTGTTAAAC AGNNGGTTGA NCACATNGAG TGGTTTAGAN ATTNTTTTNA 240
TGNNAN 246

SEQ ID NO:5423
SEQUENCE LENGTH:105
SEQUENCE TYPE:nucleic acid
TOPOLOGY:linear
CLONE:HUMGS06456
SEQUENCE DESCRIPTION:
GATCTGCTCA GAAAGGAAGA GGCAGGCGCC AGGGNGAACC CCCTTCCGTG NTTTGTAACC 60
CTCCCTNTAA GGTGAAGCCC TTTTNCTTGC TAAAACCGGN AATTN 105

SEQ ID NO:5424
SEQUENCE LENGTH:159
SEQUENCE TYPE:nucleic acid
TOPOLOGY:linear
CLONE:HUMGS06457
SEQUENCE DESCRIPTION:
GATCCTGGGG GTCGTGGCTA CCCCGTCCTC GTGGCCGNNC TCCTTGCTGC TCCTGTCAAT 60
AGTGAGCTTG TGCCGTCCCN CTAGGATGGG GGCATGGCCN TGGGCTGCCA GATGCCCACA 120
GCACCCTGGC ATGACCTNCC ACCTNGGGTT CCANACCGN 159

SEQ ID NO:5425
SEQUENCE LENGTH:211
SEQUENCE TYPE:nucleic acid
TOPOLOGY:linear
CLONE:HUMGS06458
SEQUENCE DESCRIPTION:
GATCACCTAT CCCCTGTTTC AACAATGAAT AAGGAACAGC AAATNTTTGA GACGTGCCCT 60
GAAACTGAAC AGAGATGCAC TCTCCAGTGA CTCCCCAGCA AACACAAGGT GCAGCAGGGT 120
CCCAAAGGTA GCTGGNTGGC TGANCTGCTG GNTATGGGAG ATACATGACG CGAAGACGGN 180
TTTNACATCC ACAGGGCGGT NTTNANGAAA N 211

SEQ ID NO:5426
SEQUENCE LENGTH:66
SEQUENCE TYPE:nucleic acid
TOPOLOGY:linear
CLONE:HUMGS06459
SEQUENCE DESCRIPTION:
GATCCACCAC AGGGTTAGGG GACAGGAAGC CTGTTCTATT CTCAATAAAT CTTACAAAAT 60
TCCAAA 66

SEQ ID NO:5427
SEQUENCE LENGTH:189
SEQUENCE TYPE:nucleic acid
TOPOLOGY:linear
CLONE:HUMGS06460
SEQUENCE DESCRIPTION:

GATCATTATT TCCATTCTTA ATGTGAAAAA AAGTAATTAT TTATACTTAT TATAAAAAGT 60
ATTTGAAATT TGCACATTTA ATTGTCCCTA ATAGAAAGCC ACCTATTCTT TGTTGGATTT 120
CTTCAAGTTT TTCTAAATAA ATGTAACTTT TCACAAGAGT CAACATTAAA AAATAAATTA 180
TTTAAGAAA 189

SEQ ID NO:5428
SEQUENCE LENGTH:193
SEQUENCE TYPE:nucleic acid
TOPOLOGY:linear
CLONE:HUMGS06461
SEQUENCE DESCRIPTION:
GATCTAGACA TGCCTTAGGT AACTGCAGAA ATAGCAAATT AAATNATTAA TGAGCTTATT 60
AGTCATAGTA AACATACAGA TTCCTANTTC TATGACAGTG TACATNTATG ANAATAAATG 120
ATGAAAAAAG AACTATAGTA TNTTTAAAAA TATGCTTTTA TTTNTATTNC ACATAGTGNA 180
AAGTTCTACA NAN 193

SEQ ID NO:5429
SEQUENCE LENGTH:270
SEQUENCE TYPE:nucleic acid
TOPOLOGY:linear
CLONE:HUMGS06462
SEQUENCE DESCRIPTION:
GATCACATTT CTGGGAAGAT GAGANCTNNT TTCCAGACCA GCATCCAGTG GCCATCAGGT 60
CTTGTGGCCC AAAGGCTATG CTTGCCTCCG GCTGAGTGCC TGGNATAGGC CTTTNCTATG 120
TCTCCCCAAG GCTGGGGTGC TGAGCCTGCC TTCCTCACCA CCTAGCCATA GTCTCAAACC 180
TGTGGGGAAG GNGGNNTTCT CCCTGCCCGG GAAGAGGACA GATAACTGAT TTCCGTTCTT 240
TTGACTGTGT TTTAAAATTC TCTTTCTAAA 270

SEQ ID NO:5430
SEQUENCE LENGTH:162
SEQUENCE TYPE:nucleic acid
TOPOLOGY:linear
CLONE:HUMGS06463
SEQUENCE DESCRIPTION:
GATCTCCTGG GACTCCGAGC AAGGCCTGCA CGAGAGAGGG CTGAAAGGCT GCTGGGGCTG 60
CCACCTCGCT ATTCCCGCAT AAGCATCTGC CCCCAACCCC TTTGACCTTC ATCTGATGGA 120
CATTTTTATA CAGAAAACAA TAAAGATTTC CCTCTCAGCA AA 162

SEQ ID NO:5431
SEQUENCE LENGTH:83
SEQUENCE TYPE:nucleic acid
TOPOLOGY:linear
CLONE:HUMGS06464
SEQUENCE DESCRIPTION:
GATCAGTTGT ACTAGAGGAA CATCATAGAA GAGAAAAATC CAACTCTTTA TATTAAAAAT 60

NAATACAATA AGAAAATTGC AAA 83

SEQ ID NO:5432
SEQUENCE LENGTH:168
SEQUENCE TYPE:nucleic acid
TOPOLOGY:linear
CLONE:HUMGS06465
SEQUENCE DESCRIPTION:
GATCTGCAAG CCCTGCTGTA CAAGAAAATC ATTAAGGAAC ATTTGGAGAG TTAGCTACTA 60
GCTGCCTAAG TGTGCACTTT CAATCTAACT GTGAAAGAAT CTTCTGATGT TTGTATTATC 120
CTNCTTATAT TATATTANCA AAATAAATCA AGTTGTGGTA TAGTCAAA 168

SEQ ID NO:5433
SEQUENCE LENGTH:357
SEQUENCE TYPE:nucleic acid
TOPOLOGY:linear
CLONE:HUMGS06466
SEQUENCE DESCRIPTION:
GATCCACCCA CCTTGGCCTC CCAAGGTGCT GGGATTGCAT GCATGAGCCA CTGCACCCAG 60
CCAAGATACA TTGTTGACGT GAAAAAAAAA TTTCAAGATA TATAGAACAG TGTAAACATG 120
CATCCAATTG TGTTTTTCAN GGAGAAAATA ATGTATACTT GTATTGGTTT ACGTATGAAA 180
CAAAANTATC TAGAAGGATG AATAAAAATC TAGTAACTGA TTNTACACTA GGGCAAATTG 240
GAAACAANTG GGNGACTTCA TGGATGACTT TTTTATATTT GTAAAGTGGA NGTGANTGTN 300
TTACTTATTC CACCAGTTAN GTTGAAACCT TTNNNNGGNA GGTTNGTGGT GNTTNTN 357

SEQ ID NO:5434
SEQUENCE LENGTH:175
SEQUENCE TYPE:nucleic acid
TOPOLOGY:linear
CLONE:HUMGS06468
SEQUENCE DESCRIPTION:
GATCAGATGG CTTAGAGAAA TTNCTGGAAT ATTCACATTC GAAGATTCCT TATTAATNAA 60
TGTCTTTNAC TTAAATCTAA CCAAAAACTG CAACATTATT CTTTGTACAT TTNCATTATA 120
TAGTGTTAAC AAGCTTAGTT GCANACAANT AAAATACTTA AGCTATTTGT TTAAA 175

SEQ ID NO:5435
SEQUENCE LENGTH:218
SEQUENCE TYPE:nucleic acid
TOPOLOGY:linear
CLONE:HUMGS06469
SEQUENCE DESCRIPTION:
GATCTGGTGA AACTNGCTAA ACGNCTCAAG AAGGAGAAAG TTAATNTTGA CATTATCAAT 60
TTTNGGGAAG AGGAGGTGAA CNCAGAAAAG CTGACAGCCT TTGTAAACAC GTTGAATGGC 120
AAANATGGAA CCGGTTCTCA TCTGGTGACA GTGCCTCCTG GGCCCAGTTT GGCTGATGCT 180
NTCATCAGTN CTCCGATTTT GGCTGGTGAA GGTGGNNN 218

SEQ ID NO:5436
SEQUENCE LENGTH:153
SEQUENCE TYPE:nucleic acid
TOPOLOGY:linear
CLONE:HUMGS06470
SEQUENCE DESCRIPTION:
GATCACAGTG GGCAGCCGGC ACCCNGCACC ACTTTGGCGA GCGTCCTGCT TCCGNCCTCG 60
CCCTCATCTA CGCTGCTCCG CTTTCCTCAG ACCCCTTTTT GCCGTGCAAA GGGAATTCTT 120
GACATTAAAT AAAAGGTATC CAGNTTGCAG AAA 153

SEQ ID NO:5437
SEQUENCE LENGTH:310
SEQUENCE TYPE:nucleic acid
TOPOLOGY:linear
CLONE:HUMGS06471
SEQUENCE DESCRIPTION:
GATCAGTTGG GTGCACGAGT GGGTTACATC GAACTGGATT ATAGGAAGCT TATAACAAGA 60
ATGGAAGATT CTTAAATAAC TCACTTTCTT TGGNATCCAG TAACAGTAGA TGTTCAAAAT 120
ATGTAGCTGA TTAATACCAG CATTGTGAAC GCTGTACAGC CTTGTGGTTA TTACTAAGCA 180
AGTTACTACT AGCTTCTGAA AAGTAGCTTC ANAATTAATG GTTATTTNTA NACTGCCCTT 240
CCNTGGCTGN TACTTTNCCC TAAGNTNAAT CTNCGAGAAT CTGAAATGNT TNCTNCANTT 300
NTCAGGGAGN 310

SEQ ID NO:5438
SEQUENCE LENGTH:67
SEQUENCE TYPE:nucleic acid
TOPOLOGY:linear
CLONE:HUMGS06472
SEQUENCE DESCRIPTION:
GATCACATTN GATNAAAGAG CAAAACTTGT TAAGTCCAAA ATAAATTCTT ACTGTTTATA 60
TCCTAAA 67

SEQ ID NO:5439
SEQUENCE LENGTH:268
SEQUENCE TYPE:nucleic acid
TOPOLOGY:linear
CLONE:HUMGS06473
SEQUENCE DESCRIPTION:
GATCCACAGA AGGTCCTGGT GGTATTTGTA ACTTTTTGCA AGGCATTTTT TTATATATAT 60
TTTTGTGCAC ATTTTTTTTT ACGNTTCTTT AGAAAACAAA TGTATTTCAA ANGTATATTG 120
ATAGTCGANC AATTCATATA TTTGAGGTGG AGCCATATGA ATGTCAGTAG TTTATACTTC 180
TCTATTATCT CAAACTACTG GCAATTTGTA AAGCNATATA TATGNTATAT AAAGGTGATT 240
GCNGCNTTNC ANTGTTAGCC CCNGTGNN 268

SEQ ID NO:5440
SEQUENCE LENGTH:306
SEQUENCE TYPE:nucleic acid
TOPOLOGY:linear
CLONE:HUMGS06474
SEQUENCE DESCRIPTION:
GATCCAGGCT CAGAACTTCT TCTCCAGCTT CACCCTGATG AAGCTGAGGC ACTCCTCCGC 60
TCTGGGTGGG GCCAGCCTAG GGGCCAGGCA CATCGGGCAC CTNCTCCCCA TGGACTATAG 120
CGCCAATGCC ATTGCCTTCT ATTCCTACAC CTNTTCCTAG GGGGCTGGTC CCGGGCTCCA 180
CCNGGTCCAA GCTCAGTGGA CACTGGGTCT GAAAGGAAGG AGTCTTTTGN TTCCTTTCTC 240
CTNTTTACAN GAACAAACAT AGATGGTAAA TAAATNNACT TTNTCCNNCT CCCCAGAANN 300
NAATNN 306

SEQ ID NO:5441
SEQUENCE LENGTH:111
SEQUENCE TYPE:nucleic acid
TOPOLOGY:linear
CLONE:HUMGS06475
SEQUENCE DESCRIPTION:
GATCAGCTAC ATAAAACGGC CTGAGTGCTG TTTTAAACAG CNCTTGGGTG ATGGTACAAC 60
ATNACTTTTT AAGATAATNA NGTAGTAAAA GTTTCTAGTG GAAACATGAA A 111

SEQ ID NO:5442
SEQUENCE LENGTH:255
SEQUENCE TYPE:nucleic acid
TOPOLOGY:linear
CLONE:HUMGS06476
SEQUENCE DESCRIPTION:
GATCCCAACA TTTTTGGCAG TGAGCATTAC GATGTTANCC GTGGGGTGCA AAANATCTGA 60
ATAAAATTTA CAAAAGGAGA ATTCAGACAG ACCAACCTGG GTNATGTGTT TCAAGCTCTG 120
CCCCAGGAGC TTGAATGTCT NTNCCTNGTG GCAGGAGCAT TCCTAGAGAC TGGTGCATCA 180
GGTTTTATGT TGTTGTCTCT CTGCTCCAGC TCACCCNTNA AANCTCTNCC CTNGGATGTT 240
GGAACTCANA TTCTN 255

SEQ ID NO:5443
SEQUENCE LENGTH:313
SEQUENCE TYPE:nucleic acid
TOPOLOGY:linear
CLONE:HUMGS06477
SEQUENCE DESCRIPTION:
GATCCCATAA AGCTACGAGG GGGACGGGAG AGGGCAGTGC AATGGGAAGT AAAGAGATAT 60
TTNCCAGTAG GAAAAGCAAT GCTTTCTTGT CTTTAGACTC AAATGCTTAG GGAACGTTTC 120
ATTTCTCATT CATGGGGAAA GGCAGCCTCC TTAAATGTTT NCTGAAGAGC GGTAAAATCT 180
AGAAGCTTAA GANTTTNCAG TTCCTTCAAT AACCATGATG ACCTGAAGTN CACCTATCCC 240
ATTTTAGCAT CTNCTTGTTT TNCCCATCTC TNCCTTTCCA ATTTTNCTTA TNCTGCTGTA 300

ATATTTTTGT AAA 313

SEQ ID NO:5444
SEQUENCE LENGTH:218
SEQUENCE TYPE:nucleic acid
TOPOLOGY:linear
CLONE:HUMGS06478
SEQUENCE DESCRIPTION:
GATCAGCTCA CACCCAGTCT CTCACAAGTN TTCATTCCAT AAAAGTGGTT GACTAAAGTG 60
AATATGTCCA TAGTGGGGTC TTTTAATCCC TCTGGTCTTA CCANGGGTTA GANGTCTNTN 120
GGTTNTAACT TTTCCATATT GTTAGAACAT CATGGNGACA TCATGTNCAT CTGTTCAAAA 180
TTACAAGAGG CTTTTNCAAG CTCTACCATG CTTTGNAN 218

SEQ ID NO:5445
SEQUENCE LENGTH:256
SEQUENCE TYPE:nucleic acid
TOPOLOGY:linear
CLONE:HUMGS06481
SEQUENCE DESCRIPTION:
GATCATTGTG AAGGCAGGGG AATGTATGTG CACATCTGTT TTGTAACTGT TTAGATGAAT 60
GTCAGTTGTT ATTNATTGAA ATNATTTCAC AGTGTGTGGT CAACATTTCT CATGTNGAAA 120
CNTTAAGANC TAAAATGNTC TAAATATCCC TTGGACATTT NATGTCTTTC TTGTAAGGCA 180
TACTGCCTTG TTTAATGGTA GTTTTACAGT GTTTCTGGCT TNGANCAAAG GGGCTTAATT 240
ATTGATGNNT NGGATN 256

SEQ ID NO:5446
SEQUENCE LENGTH:344
SEQUENCE TYPE:nucleic acid
TOPOLOGY:linear
CLONE:HUMGS06482
SEQUENCE DESCRIPTION:
GATCCGGAAG CGAATTCATC TCCGAGCTGA GGATNCCTTG TTTTTNTTTG TCAACAATGT 60
CATTCCACCC ACCAGTGCCA CAATGGGTCA GCTNNACCAG GAACACCATG AAGAAGACTT 120
CTTTCTCTAC ATTGCCTACA GTGACGAAAG TGTCTACGGT CTGTGAAGCT GCTGCCCCTG 180
AGCTGGAGGG GGGTCTCATT CTACAAAGAG AGAGGTGGCC CCCCTTTCTT GACCTGCTCC 240
TCCTTCAAGC TTAAACANCA CCTCCTTATT CAGGACCGGC ACTTCTTAAT GTTTTGTGGG 300
CTTTCTNTCC AGCTNTCTTT NGGNGGGGTA ATGGGTNGGA GTTN 344

SEQ ID NO:5447
SEQUENCE LENGTH:334
SEQUENCE TYPE:nucleic acid
TOPOLOGY:linear
CLONE:HUMGS06483
SEQUENCE DESCRIPTION:
GATCTTTCTA GGAGGGAGAC ACTGGCCCCT CAAATCGTCC AGCGACCTTC CTCATCCACC 60

CNATCCCTCC CCAGTTCATT GCACTTTGAT TAGCAGCGGA ACAAGGAGTC AGACATTTTA 120
AGATGGTGGC AGTAGAGGCT ATGGACAGGG CATGNCACGT GGGCTCATAT GGGNCTNAGA 180
GTAGTTGTCT TTCCTGGCAC TAACGTTGAG CNCCTGGAGG CACTGAAGTG CTTAGTGTNC 240
TNGGAGTATT GGGGTCTGAC CCCAAACAAC TTTCAGNTTC NNTAACATAC TGGGNCTTGA 300
CTGTTTTNTC TNGGTNCCNN ATGGGTCCTG GTTN 334

SEQ ID NO:5448
SEQUENCE LENGTH:80
SEQUENCE TYPE:nucleic acid
TOPOLOGY:linear
CLONE:HUMGS06484
SEQUENCE DESCRIPTION:
GATCTAGAAA GCCCTAAGCT CTCACCACTG GCTACCTTGA GCCTCTACAG AATNAGGAAG 60
TAAAGTCTAA GGCAGTTAAA 80

SEQ ID NO:5449
SEQUENCE LENGTH:147
SEQUENCE TYPE:nucleic acid
TOPOLOGY:linear
CLONE:HUMGS06485
SEQUENCE DESCRIPTION:
GATCGCANCT CCGAAGACGG AGAGGAGGGA AATGGGGCCC TTTCCCCTCT ATTGCATCCC 60
CCTGCCCGNC TCCTTCCCCG CACCCACGTG CCCTAGATTC ATGGCAGAAA ATGNCCAAAT 120
CCNGNGTATT TGTTGTANAT ANTTAAN 147

SEQ ID NO:5450
SEQUENCE LENGTH:109
SEQUENCE TYPE:nucleic acid
TOPOLOGY:linear
CLONE:HUMGS06486
SEQUENCE DESCRIPTION:
GATCATTGTA TATNACACAC ATGTATCAAA GTATCACATG TACTCCAAAT GTTTGTACAA 60
CTATNATATA TCATTAAAAA AATACAAANT AAAAAATGTA GCTATGAAA 109

SEQ ID NO:5451
SEQUENCE LENGTH:274
SEQUENCE TYPE:nucleic acid
TOPOLOGY:linear
CLONE:HUMGS06487
SEQUENCE DESCRIPTION:
GATCCAGTGT GCAGAAGAGC CAGCAGGGAA CCGGAAGCTC TNATGTCAAG GCCAGAGCAG 60
TTGAGANTGG GNCCCAGAGT AGATGCTGAC CTGGGCACTC CACCATTCCG GGGCCACCAC 120
AGAGATGCCA GCAGGATGCC ACTTTNCCAG CCCGACACAC GGACCTTTNT AAAGANCAGC 180
AACAGGCAGG AGAGGCAGCG TNTGNCCAGA TTGTTTCCCG TCATTGGGTG GCATNTGTTA 240
NCTAGCTGNC AAACANCTTC ANCCCGTGTA ATTN 274

SEQ ID NO:5452
SEQUENCE LENGTH:183
SEQUENCE TYPE:nucleic acid
TOPOLOGY:linear
CLONE:HUMGS06488
SEQUENCE DESCRIPTION:
GATCACGGCT CTTTTTAAGC CCTGGCAGCA TTTTGGTCCC TGCTCCTTGC CCATAGTAAA 60
ACAGCTTGAA ATATCCCATG CAAGAGAGTA GTTTCAAGTG GGCAACTCTG CTCTCTATTT 120
AAAAGCGTGC ACAATCAAAA GTACTATGCA ATTTTAGGAC AATAAAGANC ATACAGTTTT 180
AAA 183

SEQ ID NO:5453
SEQUENCE LENGTH:215
SEQUENCE TYPE:nucleic acid
TOPOLOGY:linear
CLONE:HUMGS06489
SEQUENCE DESCRIPTION:
GATCACCAGT GGGCACTAGA TGATGTNGAA GAAGAGCTCA TGGCCAAANA CCTCCACTTC 60
GAAGGCATGT TCAAAAAGGA ATTACAGACC TCTATTTTTT GAAGACCGAG CAGGGATTAG 120
CTGTGTCAGG AACTTGGAGT TGCACTTAAC CTTGTAACTT TGTTTGGAGC TGGCACCTCT 180
TGAAATAAAA AGGGAGGNTG CACGAGCTGG GCAAA 215

SEQ ID NO:5454
SEQUENCE LENGTH:70
SEQUENCE TYPE:nucleic acid
TOPOLOGY:linear
CLONE:HUMGS06490
SEQUENCE DESCRIPTION:
GATCAAAAAA TTCAATTTNC TACAAGTTCT NCTATCTGAA ATAAATAACA GACTCATTTT 60
TGACAATAAA 70

SEQ ID NO:5455
SEQUENCE LENGTH:104
SEQUENCE TYPE:nucleic acid
TOPOLOGY:linear
CLONE:HUMGS06491
SEQUENCE DESCRIPTION:
GATCANGNTA GTGGTGTCTG TCAGCTGTCT AAGAGGTTGG AGAATNAACT ACTCAAGATA 60
GTCACGAAAA NACTGAAAGT TTNATTTTCC TTTCCATATT TAAN 104

SEQ ID NO:5456
SEQUENCE LENGTH:214
SEQUENCE TYPE:nucleic acid
TOPOLOGY:linear

CLONE:HUMGS06492
SEQUENCE DESCRIPTION:
GATCCCAGTT ATCCTTGNAT CCATGTAATT TCAGATGAAT TATTAAGCAA ACATTTTAAA 60
GTGAATTCAT TATTAAAAAC TATTCATTTT TTTCCTTTGG CCATAAATCC NCTAATTNTC 120
ATTAAAATTC TAAGGTCATT TCAACTGTTT TAAGCTGTAT TNCTTTAATT CTGCTTACTA 180
TTTCATGGAA AAAANTAAAT TTNTCAATTT TAAA 214

SEQ ID NO:5457
SEQUENCE LENGTH:105
SEQUENCE TYPE:nucleic acid
TOPOLOGY:linear
CLONE:HUMGS06493
SEQUENCE DESCRIPTION:
GATCCAGAAT AACTTCTCCC ACTCATATCT NCAGTTCACC NAATGAAATN AATGGATAGC 60
AAGAGCCCNT TGTTCCCAGG ACTTTAAGGC AAAATATTAA AANTN 105

SEQ ID NO:5458
SEQUENCE LENGTH:56
SEQUENCE TYPE:nucleic acid
TOPOLOGY:linear
CLONE:HUMGS06496
SEQUENCE DESCRIPTION:
GATCCTTTTA TAAATTTAAA AAATTTAATG TTAATAAAGC GAGGTGTAAT TGTAAA 56

SEQ ID NO:5459
SEQUENCE LENGTH:103
SEQUENCE TYPE:nucleic acid
TOPOLOGY:linear
CLONE:HUMGS06498
SEQUENCE DESCRIPTION:
GATCCGNGAA AGGCTGAAAG GCCAGGAAGA CAGCCTAGCC TCTNCAGTGG ATGCTGCCAC 60
CGNACAAAAG ACCTGTNACT CCGACTGAGG CATGNCCTGC CCN 103

SEQ ID NO:5460
SEQUENCE LENGTH:190
SEQUENCE TYPE:nucleic acid
TOPOLOGY:linear
CLONE:HUMGS06499
SEQUENCE DESCRIPTION:
GATCNTGGAT NTAGAAGAGG TNACATTGCA CAATGTTTGA ATAAGGAAAC TATTGGAAAG 60
TTTTTCCAAG CTGACATTGC AGAAAATGCT TTGNAAAATT CCTCAGAAAC AGAAATACCT 120
ACTGTCAGCG TNTTAGCTGA TGAAGANTTC CTTCCCTNCA AAGAAAATAC ATTTGACCTG 180
GTGGTNNGCN 190

SEQ ID NO:5461

SEQUENCE LENGTH:105
SEQUENCE TYPE:nucleic acid
TOPOLOGY:linear
CLONE:HUMGS06500
SEQUENCE DESCRIPTION:
GATCCCGCCC ACTCAGAAAA GGCGAGAAGG CTTTGTAATT TTCTACATGA ATCAAACACA 60
GAAACAACTT TTGGAGAAAT TAAATTCTGA GTGTNACTTC TGAAA 105

SEQ ID NO:5462
SEQUENCE LENGTH:83
SEQUENCE TYPE:nucleic acid
TOPOLOGY:linear
CLONE:HUMGS06501
SEQUENCE DESCRIPTION:
GATCTTCTTC ACACCAAGCT CTGTTTACAT TCCGAGAGTT GTCATGAAGA AAGTNCTNNT 60
CAATAAGNTT TTGGAATGTT AAA 83

SEQ ID NO:5463
SEQUENCE LENGTH:180
SEQUENCE TYPE:nucleic acid
TOPOLOGY:linear
CLONE:HUMGS06502
SEQUENCE DESCRIPTION:
GATCAGGACA CGAGGAAGAG TAAATGTGAG ACAGTCAATG TGACTTCTGC GATAAACAGA 60
TTTTTAAACC CCGAAATTTT GCAAAATTTT GGTGAACCTG ANCTTNCTTC GTTGCATATG 120
CTGGCACTAT CTGTACCATC ATACANCTGT CTCACATTAA AGCTATTTTC CTTGGGCAAA 180

SEQ ID NO:5464
SEQUENCE LENGTH:214
SEQUENCE TYPE:nucleic acid
TOPOLOGY:linear
CLONE:HUMGS06503
SEQUENCE DESCRIPTION:
GATCATCTCC CCTGGCAACG TGACTCTGTT TTTTGTGTGT GTTTCCATGC TGACTAGTCC 60
CCTACTGTTA ATATCACTAC TAATTAGGCT ATAACCAGGT CTTTCCTGGC CTGAGAAATA 120
TTCTCTTAAA ATNACCTTTG TTTTAATCTC ATTCATGATG TTGATTTTTT TTCAATGTGG 180
TGCAATATAT ACANTAAAAN TNGTCATANC TAAA 214

SEQ ID NO:5465
SEQUENCE LENGTH:186
SEQUENCE TYPE:nucleic acid
TOPOLOGY:linear
CLONE:HUMGS06504
SEQUENCE DESCRIPTION:
GATCTGTCCA GGGAGGTGGC TGAAGAGTGG GCATCTCCCT TAGGGACTCT ACTCAGCACT 60

CCATTCTGTG CCACCTGTGG GGTCTTCTGT CCTAGATTCT GTCACATCGG CATTGGTCCC 120
TGCCCTATGC CCCTGACTCT GGATTTGTNA TCTGTAAAAC TGGAGTAAAA ACCTCAGTCG 180
TGTAAA 186

SEQ ID NO:5466
SEQUENCE LENGTH:117
SEQUENCE TYPE:nucleic acid
TOPOLOGY:linear
CLONE:HUMGS06505
SEQUENCE DESCRIPTION:
GATCTTTGCT TCCTTNCTAT TATGTTTGTA CACAACACCT AAAACCAGTT TTGCTGCTAT 60
ANTTCTATAC TGTTGATTCG TCTGCNANNT ATCTGTAACC AAATAAACAT AATAGGN 117

SEQ ID NO:5467
SEQUENCE LENGTH:309
SEQUENCE TYPE:nucleic acid
TOPOLOGY:linear
CLONE:HUMGS06506
SEQUENCE DESCRIPTION:
GATCCAAACC AAAGACTGTA GAACCCTGGG GTGTGGCTAA CTGGCCCNTN CNGCACCCGT 60
AGCCAGGTCT TCCTGGCCCT TNAGGCTGGG CTGGCGGACA GGCACCTNCC TGTTCCTTAA 120
GCTGAAGCTC CCACACTGTC TTCCAGGGCT GAGGAGATGC TCTCCTTTTC TACTGACCAT 180
CTTGATACTT ATTTATACGA GAGNCAGTTG CTGGACGGGG TAGTACTGGG AAGCAGGAGG 240
CAGAATGGCT CTGCTGAGCC TNCNACCCAT GGNCAACANC CTAATAAAAC AGANCATTTC 300
AGAGCCAAA 309

SEQ ID NO:5468
SEQUENCE LENGTH:97
SEQUENCE TYPE:nucleic acid
TOPOLOGY:linear
CLONE:HUMGS06507
SEQUENCE DESCRIPTION:
GATCCCATAC CAGAAGATGA GAAAAAAGAA TAAGTGTTGC CTTGTTTTGT GTGTTCTAAA 60
TACTTTTTNT AATGAAAAAA TGNTTTTTNG TTTTAAA 97

SEQ ID NO:5469
SEQUENCE LENGTH:67
SEQUENCE TYPE:nucleic acid
TOPOLOGY:linear
CLONE:HUMGS06508
SEQUENCE DESCRIPTION:
GATCATCTAA ACTAAGTCCA GCTGCCTAAT TCTAGATATA TATATATATA TAGAGNTCCA 60
CCATAAA 67

SEQ ID NO:5470

SEQUENCE LENGTH:72
SEQUENCE TYPE:nucleic acid
TOPOLOGY:linear
CLONE:HUMGS06509
SEQUENCE DESCRIPTION:
GATCTTGGGA AGTATTCCCA AGGAAAACAG TCCTGGGCAC AGTCTCCAAA ATAAATTCTN 60
AATTTTGGGA AA 72

SEQ ID NO:5471
SEQUENCE LENGTH:467
SEQUENCE TYPE:nucleic acid
TOPOLOGY:linear
CLONE:HUMGS06510
SEQUENCE DESCRIPTION:
GATCCCATGG CTTTCTTTAC TGGGCTCTGG GGCCCCTTCA CCTGTGTAAG CAGAGTGCTG 60
AGCCATCACT GTTTCAGCAC CACTGGGAGT CTGAGTGCGA TTCAGAAGAT NACGCGGGTA 120
CGAGTGGTGG ACAACAGTGC CCTGGGGAAC AGCCCATACC ATCGGGCTCC TCGCTNCATC 180
CATGTCTATA AGAAGAATGG AGTGGGCAAG GTGGGCGACC AGATACTACT GGCCATCAAG 240
GGACAGAAGA AAAAGGCGCT CATTGTGGGG CACTGCATGC CTGGCCCCCG AATGACCCCC 300
AGATTTGACT NCAACANCGT GGTCCTCATT GAGGNCAACG GGGAACCCTN TNGGNGACAN 360
GTATTNAAGA CACNGTNCCC ACCTAGGCTG TGGNAGGGTG AAGGGCGAGT TTTCCCAAGN 420
TGGTGGGCCT TNGTTNAGAN CTTTGTGTTG NGTTTGGNNC NNGNTAN 467

SEQ ID NO:5472
SEQUENCE LENGTH:327
SEQUENCE TYPE:nucleic acid
TOPOLOGY:linear
CLONE:HUMGS06511
SEQUENCE DESCRIPTION:
GATCTTAACT NTCTTGACTG ATTTAAAAAG CTAATCAATT TATTAAAGCC AGANATGTNC 60
TCCTCTGGGA AAGCAATTTC TATCTATTTA TCTACCAATC CCCACTCACA TATATATGTT 120
TGTNTTTACA TAGAGATAGA TGGTTTTAAG GGTTTTCCTA TTGTTAAATT ATTTCCTAAG 180
ACATTGTGGG NTGGAGACCA CTAAACTTAT AAAAAACCAA GGAAAGTAAA GTTTTNCAAA 240
TGTTTATACT GTTTAAGGTG TTTATTTATN TAANCCATGC ATGGGGTNNC ATGCATAANT 300
NNNGNGGTTT AAATTTCACC AGTGANN 327

SEQ ID NO:5473
SEQUENCE LENGTH:70
SEQUENCE TYPE:nucleic acid
TOPOLOGY:linear
CLONE:HUMGS06512
SEQUENCE DESCRIPTION:
GATCAAGGAA CTTGTGCCTG TCTATAGATT TTATTTCCCC TGTACAAATA AACTTGGCTG 60
CAATCCCAAA 70

SEQ ID NO:5474
SEQUENCE LENGTH:414
SEQUENCE TYPE:nucleic acid
TOPOLOGY:linear
CLONE:HUMGS06513
SEQUENCE DESCRIPTION:
GATCTTGCAA GACTAAAATT GGCCATTAAG TACCGTCAAA AAGAGTTTGT TGCCCAGCCC 60
AATTGTCAAC AGCTGCTGGC ATCTCGCTGG TACGATGAGT TTCCAGGCTG GAGGAGAAGA 120
CACTGGGCAG TGAAGATGGT GACATGTTTC ATAATAGGAC TTCTTTTTCC TGTCTTCTCT 180
GTGTGCTACC TGATAGCTCC CAAAAGCCCA CTTGGACTGT TCATCAGGAA GCCATTTATC 240
AAGTTTATCT GNCACACAGC CTNCTATTTG ACTTTTTTGT TCCTGNTGNT GCTTGCCTCT 300
CAGGACATAG GCAGGTCAGG NNTGANCNGG GAAGGTCCAC CANCACCATC GTCGAGTGGA 360
TGATATTGNC NTGGGTNCTN GGNNNATGTG TTANNTAGGA AATGTNATAA ANCN 414

SEQ ID NO:5475
SEQUENCE LENGTH:112
SEQUENCE TYPE:nucleic acid
TOPOLOGY:linear
CLONE:HUMGS06514
SEQUENCE DESCRIPTION:
GATCAACAAT NAGCATCAAA TCTATGGCTC CTGGCTCCAG CANCAAGACG CAAGAAGACA 60
GGANGAGAAG CANGAAGTGA GACTGGAGGG AAAGGGAGCT GAGTCTNCTA GN 112

SEQ ID NO:5476
SEQUENCE LENGTH:52
SEQUENCE TYPE:nucleic acid
TOPOLOGY:linear
CLONE:HUMGS06515
SEQUENCE DESCRIPTION:
GATCATTCTT TTATAGAGCA TATTTGCCAA TAAAGCTTTT GGAAGCCGGA AA 52

SEQ ID NO:5477
SEQUENCE LENGTH:223
SEQUENCE TYPE:nucleic acid
TOPOLOGY:linear
CLONE:HUMGS06516
SEQUENCE DESCRIPTION:
GATCTAACTT TGCACTCTTT GATAATAATG TTTTAGATAA TGTGCGTAAT CCAAATTGGT 60
ATTGTAGCCT CTGTTAACAC AGACAGTATA TGTTTTAAAC TTTGATGTAA ACCTTTTNAG 120
ACCCAAACTT GTGGAAGTAT CATGTGTTAA GTNCTCTGTC TCTGTTTCTT TGTTCATTTA 180
TTACTAAAAT GAACTTGTTA TTAAAGTATA TGCAAATNTG AAA 223

SEQ ID NO:5478
SEQUENCE LENGTH:144
SEQUENCE TYPE:nucleic acid

TOPOLOGY:linear
CLONE:HUMGS06517
SEQUENCE DESCRIPTION:
GATCCGGGGN AGCTGTATCC CCAGTAGAAA AAACATTTTA ATCACTCTAA TATAACTCTG 60
GATGAAACAC ACCTTTTTTT TTAATAAGAA AAGAGAATTA ACTNCTTCAG AAATAACTAA 120
TAAATGAAAA ACCTTTAAAG GAAA 144

SEQ ID NO:5479
SEQUENCE LENGTH:117
SEQUENCE TYPE:nucleic acid
TOPOLOGY:linear
CLONE:HUMGS06518
SEQUENCE DESCRIPTION:
GATCTTTTGA CGNNTATANA AATGTACTAA TGTNATAAGT TAATGCTGGN CATTTCATTT 60
ATATATATTT TTAAGAGGCT AGAGGCTTTT AGCTTTTTAA AACTCCATTA TATACAN 117

SEQ ID NO:5480
SEQUENCE LENGTH:168
SEQUENCE TYPE:nucleic acid
TOPOLOGY:linear
CLONE:HUMGS06519
SEQUENCE DESCRIPTION:
GATCTTTCAT TCTCATGCAG ATATTTCTAT TGTGGGCTTA TACATTTGNT CACCTATTGT 60
TGATGGNACT TGNGACTTTC CATTTTGGCG CTATTACAAN TAGGCACTAT GAATGACTGA 120
TGNACCACTT ACTTGAGCTT TATGNCTTAT TNTCAAAGCN GCAAAATN 168

SEQ ID NO:5481
SEQUENCE LENGTH:395
SEQUENCE TYPE:nucleic acid
TOPOLOGY:linear
CLONE:HUMGS06520
SEQUENCE DESCRIPTION:
GATCAGTTAT GAAGAAGGGA AAGCTTTGGC AGAATCTTGG AATGCAGCTT TTTTGGAATC 60
TNCTGCTAAA GAAAATCAGA CTGCTGTGGA TGTTTTTCGA AGGATAATTT NGGAGGCAGA 120
AAAAATGGAC GGGGCAGCTT CACAAGGCAA GTCTTCATGC TCGGTGATGT GATTCTNCTG 180
CAAANCCTGA GGACACTGGG AATATATTCT ACCTGAAGAA GCAAGCTGCC CGTCCTCCTT 240
GAGGATAACC TATGCTNCTT TTTNCTCCTG TTAACCTGNA AGATATCATT GGGNTCAGAG 300
CTCCCTNCCT TCAGATTATG TTACCTCTGA GTCTGTCCAA TGAGTCACTT CCATTTCAAA 360
TTNNNNGCAT CNNTTTNCAN TGTGTATGTN GNATN 395

SEQ ID NO:5482
SEQUENCE LENGTH:89
SEQUENCE TYPE:nucleic acid
TOPOLOGY:linear
CLONE:HUMGS06521

SEQUENCE DESCRIPTION:
GATCTTCCAG AGAAGGGAAC ATCTGTCACA GACAATGACT GTATCTGTNT AGATATTTCT 60
TATTTAATAA AGACGATTTG TCAGTTAAA 89

SEQ ID NO:5483
SEQUENCE LENGTH:137
SEQUENCE TYPE:nucleic acid
TOPOLOGY:linear
CLONE:HUMGS06522
SEQUENCE DESCRIPTION:
GATCAGAAAT GCCTCCTTTA CCTACCAGAG CCTACAACAC TACCTGCCAC TAATAGTTAT 60
GTCACCCTCT TATTAATCAT CATCTAGCCC TAAGTCTGGN CTATNAGTGA CTACAAAAGA 120
TTAGCTGAGT GAATAAA 137

SEQ ID NO:5484
SEQUENCE LENGTH:250
SEQUENCE TYPE:nucleic acid
TOPOLOGY:linear
CLONE:HUMGS06523
SEQUENCE DESCRIPTION:
GATCAGGGGA AGAATACCAA CCTGCATAAG TGTACTAANT CTACGTCTGG TTAATGTATG 60
TACTCTCCTG GACTGAATGC AGTGTATAAT TCCTGTCTAC ANTAGAAGTG TGCCCCAGTT 120
CCACATTTGA TTACACATGT GAGATTTGCT GCTGTTGCAT ATAACACTAG GNTAATAGNT 180
TTGNATNCAT GCAGTCATAA AAATTGGAAT GAGAATTAAN CTGCAGGAAC ATTTGNACGN 240
TNTCTNCNCN 250

SEQ ID NO:5485
SEQUENCE LENGTH:117
SEQUENCE TYPE:nucleic acid
TOPOLOGY:linear
CLONE:HUMGS06524
SEQUENCE DESCRIPTION:
GATCAGAACC AGAAAGAACT NTGCTTNACC GAGAAAATAT CTAAACATCG AAAAACTTAA 60
ATATTATGGA AAAAAAACAT TGCAAAATAT AAAGTAANTA AAAAAAGGAA GGANCNN 117

SEQ ID NO:5486
SEQUENCE LENGTH:118
SEQUENCE TYPE:nucleic acid
TOPOLOGY:linear
CLONE:HUMGS06525
SEQUENCE DESCRIPTION:
GATCTAATGC AGGATGTCAG ACTTNTNTCT ATCCAGGAAT AAAACCAGTT ATGCCAGAAG 60
TAACTATTAT NCACCTNTCT CCTACTGAAT TAAAATACTA TCAGTTGTAT ATNAAATN 118

SEQ ID NO:5487

SEQUENCE LENGTH:118
SEQUENCE TYPE:nucleic acid
TOPOLOGY:linear
CLONE:HUMGS06526
SEQUENCE DESCRIPTION:
GATCACAGTT TTCAGCANNT CAGACATGGT CTTGNGTAGT TGTTCAACAG TGTTTGCCAG 60
CTACAGAGGA AANGNCAGAC TCACAGAAGG GTGTCATTTA GAAGAAAGAA CACTAATN 118

SEQ ID NO:5488
SEQUENCE LENGTH:120
SEQUENCE TYPE:nucleic acid
TOPOLOGY:linear
CLONE:HUMGS06527
SEQUENCE DESCRIPTION:
GATCCTCAAC GAAATCAATC TCACAATAGG NAAGGAAAAG NCAATACAAG GAAGAAACCA 60
TTGAGAAGAT GCAGAATAAA GTAATCTTAT ATACANGCTT TGATTAAAAC TTGAAACAAA 120

SEQ ID NO:5489
SEQUENCE LENGTH:111
SEQUENCE TYPE:nucleic acid
TOPOLOGY:linear
CLONE:HUMGS06528
SEQUENCE DESCRIPTION:
GATCGCCATC ANTTGGGTAG TGACAGNGTA TTAATTTGCT TATACANTTN CTTTACTNTC 60
CTTTTTTCCT TTCTGGAGCA TCACATGCTG GTGCTGTGTC TTTATAATGT N 111

SEQ ID NO:5490
SEQUENCE LENGTH:422
SEQUENCE TYPE:nucleic acid
TOPOLOGY:linear
CLONE:HUMGS06529
SEQUENCE DESCRIPTION:
GATCCTACAG ATACAAGTTG CTAAANGGGC ATATCAACAG TCCAACAAAT AAAGAAGATG 60
CCTGGATTGA GCATGATGTT TGGAGATGGA GATTTATGTG TCTCTGGGAT TGTGGGTTGG 120
ANTACTGGTC TGTGGCTGTN ACANCTATTC CTCTGTGAGT GACTCTTGCA TGGAGAGATT 180
CATATATTCA GGCAGCTAGG ATTGTTCCTT CTACTGGGAC ATACACGATT GANTTTNCTG 240
AATANGGGTA GTTAANCATT GTATGTTACN CNCACTTTTG TGCTTTTCCT CCATTGTGCC 300
NTATTAACGC TCTTTCGCNT CTGNGAGAGT CTGAGTTAGC TGGTGGAGCG CCAAATACAA 360
CTGGTTGTCC GTGTGTNCTT TCCCTTTGTC ANTGGTTTGC CACATCAGTT TGTGNTGTAT 420
GN 422

SEQ ID NO:5491
SEQUENCE LENGTH:192
SEQUENCE TYPE:nucleic acid
TOPOLOGY:linear

CLONE:HUMGS06530
SEQUENCE DESCRIPTION:
GATCCCCCAG ATACCAGCTC TTCTGGNTAT ACACAGACCA GCTATAATTN GCTAAGCATA 60
AANTATTNCA GTGTATTAGC CAGCNGTGGC TCCAGAGTCT GTGCCCTTGA CCACCGTGCC 120
CAACTGTTTC ACATGCTGTG TTTGGTAGTT TNNCCTAGTG CTCTCCTTAA TAATGGTGGT 180
GTNTAGCAAA NN 192

SEQ ID NO:5492
SEQUENCE LENGTH:108
SEQUENCE TYPE:nucleic acid
TOPOLOGY:linear
CLONE:HUMGS06531
SEQUENCE DESCRIPTION:
GATCATTAGG TCAGAGTTTA CACGNCCTCT CGTCACTAGA ATCCACCCAG CCCACGAATC 60
ATCACCCNCT TGAAGGACAT TATTACTACT GNAANTTNGA ACAAACTN 108

SEQ ID NO:5493
SEQUENCE LENGTH:329
SEQUENCE TYPE:nucleic acid
TOPOLOGY:linear
CLONE:HUMGS06532
SEQUENCE DESCRIPTION:
GATCCATCCA GAACAGCGGT ATCTGAAGCC CAGGCCATAC TCCCTGCCTC CTTNCTTCTG 60
CCTACCAGAG GCTCCANAGT TGAGCTTGTC CNTTATCTAG AAACATGTGA AGATGCCCAA 120
GAGCCTGGAG GCACTGCTGT CCTTCCTNCA GAAACAGTTT CTCCTCCTNC CCTCAGCCTT 180
GTGGCCANTT CCNCTTCACA TGAAGCCCCT TGGCATTTNC TNGGGAAGGG ACTNGCCTGG 240
TACTTGCTGT TAGGGCAGGA AGGGGCAAAN GGNNGACTTT GGGTAAGTAA TCTGGGGGGT 300
TTAGATGGGN AGCANTAAGC CAGCTNGGN 329

SEQ ID NO:5494
SEQUENCE LENGTH:125
SEQUENCE TYPE:nucleic acid
TOPOLOGY:linear
CLONE:HUMGS06533
SEQUENCE DESCRIPTION:
GATCATGCTT GTNTNAGGCA ATCATGGTGG CATCACCCAT AAAGGGAACA CATTTAACTT 60
TTTTTNCTCA TATTTTAAAT TACTACANGA TTATTAAAGA TAAAATNATT TGAAAAACTC 120
TTAAA 125

SEQ ID NO:5495
SEQUENCE LENGTH:337
SEQUENCE TYPE:nucleic acid
TOPOLOGY:linear
CLONE:HUMGS06536
SEQUENCE DESCRIPTION:

GATCATGAGA CTGAAATTGG TCAGAATTGT GTCCAGAATG TGCTCAGCTA ATTCAGTATT 60
CTTCCCCATT CTGGGTTGGA GTTTACTGCA GAGTAATTCT TACAGTGCTG ATGTCAAGAC 120
TGTTACTGTT CTTCGACTTT GATTCCTTGC TCATGACATG AGTAGGGTGT GCTCTTCTGT 180
CACTTCACAC AGACCTTTTG CCTTTTTTAG CTGCAAGTCA AGGACTAGGT TGATGATGCN 240
CATGACCTGT AATTGTAAAG ANGCTTGGNC ATCTGCAAAT GATATTNAGA CCATCTNGGC 300
TTGTGCTTNN ATTCAAACTA ATGTGAACCA ATANGTN 337

SEQ ID NO:5496
SEQUENCE LENGTH:194
SEQUENCE TYPE:nucleic acid
TOPOLOGY:linear
CLONE:HUMGS06537
SEQUENCE DESCRIPTION:
GATCCATGAT GATAATTAAG AGAAGAGAAA AAAAGATATT ATTTTTGTNA GGACAAACCA 60
TTGTAGGTTT TTAGCAATGT GTATCTGTGT GTCCCTCACA CCTTTTCCTA TTCTACTTTT 120
TTTTCCTTTT TTTAAAAATA TTATGTACTA TATTTTNAGT CTCAAACACA CTATATATTA 180
TATATNTNNN NNNN 194

SEQ ID NO:5497
SEQUENCE LENGTH:207
SEQUENCE TYPE:nucleic acid
TOPOLOGY:linear
CLONE:HUMGS06538
SEQUENCE DESCRIPTION:
GATCATTTCT AGGCAAAATT GTGAAGCTAA TGACCAACCT GTTTCTACCT ATATGCAGTC 60
TCTTTATTTN ACTAGAAATG GGAATCATGG CCTCTTGAAG AGAAAAAAGT NACCATTCTG 120
CATTTAGCTG TNTTCATATA TTGCATNTCT GTATTTTTTG TTTGGATTGT AAAAAGTTCA 180
CATAATAAAC GATGGTTGTG ATGTAAA 207

SEQ ID NO:5498
SEQUENCE LENGTH:320
SEQUENCE TYPE:nucleic acid
TOPOLOGY:linear
CLONE:HUMGS06539
SEQUENCE DESCRIPTION:
GATCCAAGTG ACCAAAGGAG TTCAAGGCCC AAAATTTAGT TATGCTGGAT TAATTCTGAG 60
AGTAACAAGC ACATAGATTA TAATCTAAGA AAACCCTTTG TAGCTATGCA TNGTCGGGAG 120
AGCATCTAAC ACTAATGGTG ATGTTTCCCA TGCAGAGACT CAGATTACAG TGACTCTTCC 180
AGTGAAGACA GATGAAAGCC ATTGGGCATT GTNCCTTTNT TAATCCAAGC TAAACTANCC 240
AAGGATATAG GGGTGTGTAT GTGTCTGNGT GTGTGTGTTT GTNTGTGTGT GCACATACAT 300
NTNTAGGTAT GGTTGTTGGN 320

SEQ ID NO:5499
SEQUENCE LENGTH:115
SEQUENCE TYPE:nucleic acid

TOPOLOGY:linear
CLONE:HUMGS06540
SEQUENCE DESCRIPTION:
GATCTGTCTT GGTCGTTTGT GTTATAAAAC CAAAGTTCTC TACAGACTTT ATTTTTGTAC 60
AATATCATTT TGTAACTTTT TACAAATAAA AACTCATTTC TATTGCTCTC TGAAA 115

SEQ ID NO:5500
SEQUENCE LENGTH:314
SEQUENCE TYPE:nucleic acid
TOPOLOGY:linear
CLONE:HUMGS06541
SEQUENCE DESCRIPTION:
GATCACTTAC GGAGAAACAG GAGGAAATAG CCCTGTCCAG GAGTTCACTG TGCCTGGGTG 60
CAAGTCTACA GCTACCATCA GCGGCCTTAA ACCTGGAGTT GATTATACCA NNACTGTGTA 120
TGCTGTCACT GGCCGTGGAG ACAGCCCCGN AAGCAGCAAG CCAATTTCCA TTAATTACCG 180
AACAGAAATT GACAAACCAT CCCAGATGCA AGTGACCGNT GTTCAGGGNA GCAGCATTAG 240
TGTCAAGTGG CTGCCTTCAA GTTCCCCTGT TACTGGTTAC AGNGTANCCN NCGCTCCCNA 300
AANTGGGCCA GGAN 314

SEQ ID NO:5501
SEQUENCE LENGTH:312
SEQUENCE TYPE:nucleic acid
TOPOLOGY:linear
CLONE:HUMGS06542
SEQUENCE DESCRIPTION:
GATCTGGGTT TAGAGTGTGT ATGATTAATA AATATACCTT TCCTCTTTTC AGGGAAAATA 60
ACAACCACCC NTACTGATAG TTGGGAAAAG AAGATTGGGT TATTTTNCCA TATCATTTAG 120
CTGGAAGTGA CATTTAAAAG CACCCTGCAT CACTAGTAAT AGTGTATTTN NCTATTCTGC 180
CCTTGTAATC GGTGTCCCTG TAAAACANTC CCCACAGATT ACTTTCAGAA ATAGATGTAT 240
TTCTCTNCGT AAGGGCCAGG TTTATTTCCT CCTTTTTTGN GATNNNNGGN GGAAANTGCT 300
GCTTGCACAT TN 312

SEQ ID NO:5502
SEQUENCE LENGTH:217
SEQUENCE TYPE:nucleic acid
TOPOLOGY:linear
CLONE:HUMGS06544
SEQUENCE DESCRIPTION:
GATCATCCTG TAAACATTTT ATCATGTATT AATCATCCCT GCCTGTGTCT ATTATTATAT 60
TCANATCTCT ACGCTGGAAA CTTTCTGCCT CAATGTTTAC TGTGCCTTTN NTTTNGCTAG 120
TATGTGTTGT TGAAAAAAAA AACATTCTCT GCCTGNGTTT TAATTTTGGT CCAANGTTAT 180
TTNAATCTNG TACAATTAAA AGCTTTTGCC TATCAAA 217

SEQ ID NO:5503
SEQUENCE LENGTH:334

SEQUENCE TYPE:nucleic acid
TOPOLOGY:linear
CLONE:HUMGS06545
SEQUENCE DESCRIPTION:
GATCAGGTTG AATGAATGGA ACTCTTCCTG TCTGGCCTCC AAAGCAGCCT AGAAGCTGAG 60
GGGCTGTNTT TGAGGGGACC TCCACCCTGG GGAAGTCCGA GGGGCTGGGC NAAGGGTTTC 120
TGACGCCCAG CCTGGAGCAG GGGGGCCCTG GCCACCCCCT GTTGCTCACA CATTGTCTGG 180
CAGCCTGTGT CCACANTATT CGTCAGTCCT CGACAGGGAG CCTGGGCTCC GTCCTGCTTT 240
AGGGAGGCTC TGGNAGGAGG TCCTCTCCCN NATCCCTCCA TCTTGGGGCT CCNCCAACCT 300
NTGCACAGCT CTGCGGGNGC TGAGATATNA ATTN 334

SEQ ID NO:5504
SEQUENCE LENGTH:87
SEQUENCE TYPE:nucleic acid
TOPOLOGY:linear
CLONE:HUMGS06546
SEQUENCE DESCRIPTION:
GATCTTTTAA AGGTTTTNAC CATTTTGTNA TGAGGAATTA TACATGTATC ACATTCACTA 60
TATTAAAATT GCACTTTTAT TTTNAAA 87

SEQ ID NO:5505
SEQUENCE LENGTH:323
SEQUENCE TYPE:nucleic acid
TOPOLOGY:linear
CLONE:HUMGS06547
SEQUENCE DESCRIPTION:
GATCCATCCC ATGATTTGTN CCTGTCTGAG GCATAGAGGC AGGCAAGCCG TGGATTTTNC 60
ACATGGTGAC TTTCCCACTG TGCCATGATA CAGTGTGCAT CTTATAGCAG TGCCTTTNTG 120
TCAGGGTCTC TGCTGGCAGT CTAGACCTTT TGGGCAGAAA GGAGCTTCAA ATGGCTGTGA 180
TAAGGAATAT TAAAAATNGT GTTTCTACTT TAATTGTATT GGCTGTTCAT GTATGTAGGA 240
GTTAAAATAG GCCAAACTGG AGAAATAAGC GCATTCTGTC CACCANAAAN GTGGNTTGGN 300
TGGTNNNTGT NNGNTTNGGN GGN 323

SEQ ID NO:5506
SEQUENCE LENGTH:90
SEQUENCE TYPE:nucleic acid
TOPOLOGY:linear
CLONE:HUMGS06548
SEQUENCE DESCRIPTION:
GATCAAGTTG CCATGAAAAA CCATCTACTA TATGTNAGAC ATGACATTCT NTTTCTCTCC 60
TTCCTGAAAA ATAAAGTGTG GGAAGAGAAA 90

SEQ ID NO:5507
SEQUENCE LENGTH:102
SEQUENCE TYPE:nucleic acid

TOPOLOGY:linear
CLONE:HUMGS06549
SEQUENCE DESCRIPTION:
GATCTTACTT TCTTGCTGCT TTTCCAACTA TAATCATTTT GCTCTTGCTT TAACAAAAGN 60
NGNGGCACAG NAATTTAGTA AATTTGTGTA ATAACATTTA AA 102

SEQ ID NO:5508
SEQUENCE LENGTH:64
SEQUENCE TYPE:nucleic acid
TOPOLOGY:linear
CLONE:HUMGS06550
SEQUENCE DESCRIPTION:
GATCTCACCA CTGCACTATA GCCTGGGCGA CAGAGCAAGA CTCCCGCTCC AAAAAANGGA 60
GAAA 64

SEQ ID NO:5509
SEQUENCE LENGTH:360
SEQUENCE TYPE:nucleic acid
TOPOLOGY:linear
CLONE:HUMGS06551
SEQUENCE DESCRIPTION:
GATCCACAAA TCGCTAGGAA TGTGTATACT CTGGGTAGTA AGTTTGTAGT ATTAGAAGGT 60
TTAATGACAA GTATTTGAAG AGCTGAAGTT AAGTGTTGGG AGGCCATCCC TTATGAAGGA 120
CAATGAATGG CAAGTAAAAG ACCAAGTTCT GTTCCTGGGT CCATTAATTT AGTTAAAANG 180
GCAAAGTTCT TTCAGCACAC ATATCTGCAT GCAGTCACGT GCTGCCTAAG GNTGCTTCAT 240
TCAACANTGA GCCTCACAGC CGTGCTACAC ANTAGGATAN TACTGTATTT TTNCTGTACC 300
TTTTAACATT GTGTTACAAT GGCCTACAAG AGGCTTGTGT GGNTGGCTTT NNNTGTGGGN 360

SEQ ID NO:5510
SEQUENCE LENGTH:357
SEQUENCE TYPE:nucleic acid
TOPOLOGY:linear
CLONE:HUMGS06552
SEQUENCE DESCRIPTION:
GATCATCCTC ATTAGCACAC AGATTTTNAA AAATCAATTC TCTNGCCATG CCTCCTATGT 60
GTTCACATCT CTGCATACAC TACAGATATA AGTGCATAAT CATTCATATA NACATCTGGT 120
AGGTATTCTG TAAAACTGTG TTTACTTTAG TGCATGTNAT TGTCATGTNA TGATGTGACT 180
GGGGTGTTTC TTTGTGCATG AAACTTTGCT TCTNCACAGA NTTAGATTAC TGCTCTCTCT 240
ATATNGANCT NCATATACAG CGTTTTCTTG TTATCAGCCC CCCNAAGTCT GGGATGCCCG 300
GTGGTTGGTG NTGACATGTG AGTGGTGNCC TGGGNGGTCT GNTNCACATA GGGGCNN 357

SEQ ID NO:5511
SEQUENCE LENGTH:138
SEQUENCE TYPE:nucleic acid
TOPOLOGY:linear

CLONE:HUMGS06553
SEQUENCE DESCRIPTION:
GATCTTTTCA GCTGCTATTT TGGAATAATG ACTATCATAT ATCATAACAG TGACTGATGT 60
TGGTTGTAAT GGTTGGGTTT AGGATGAACC ATTTTAAGGA TGCCAAATNA AATATTAGTA 120
TTTGTACACA GTAAGAAA 138

SEQ ID NO:5512
SEQUENCE LENGTH:167
SEQUENCE TYPE:nucleic acid
TOPOLOGY:linear
CLONE:HUMGS06555
SEQUENCE DESCRIPTION:
GATCCCAGCA GGGNTTCTGC TCTGGGGCTG GCAGGTTGCC TGGTATTATG CCCAAGGNCG 60
NTCTGCCTGG GGGAAAGGGC AGCCAGGCAG AGGCCCAGTG TCTAGTAGGC NGCTGAATTT 120
CCTGGAAGGG GTGATTGGTT GGAAAGNGGN CAGNAACCCC AGCCTNN 167

SEQ ID NO:5513
SEQUENCE LENGTH:159
SEQUENCE TYPE:nucleic acid
TOPOLOGY:linear
CLONE:HUMGS06556
SEQUENCE DESCRIPTION:
GATCCCTTGG AGCACCTTNA TGCCTGGGGT TTCTCTCCCT AAAGCTTCTT GCAGTCTAAG 60
CCTTATCCCT TATGTNCCCC ATTAAAGGAA TTTCAAAAGA CATGGAGAAA NTTGGGAAGG 120
TTTGTNCTGA CTGCTGGNNG CAGAATAGCC GTGGNAGGN 159

SEQ ID NO:5514
SEQUENCE LENGTH:167
SEQUENCE TYPE:nucleic acid
TOPOLOGY:linear
CLONE:HUMGS06557
SEQUENCE DESCRIPTION:
GATCTGGACA AATGGCAAAT AATTTGGGGT TGAATTATGG CCCAGAGAAG TAAGCAAATG 60
NAAAACTAAA CANTTATAAA TGTATCTGGG AAGCAAAGGT ATTCATANTA NGTCCCATGG 120
CCAGCTGCGA GCAGTATTTA ACTAGACATG CTGTTGTCAA TANNNNN 167

SEQ ID NO:5515
SEQUENCE LENGTH:97
SEQUENCE TYPE:nucleic acid
TOPOLOGY:linear
CLONE:HUMGS06558
SEQUENCE DESCRIPTION:
GATCGTCCCC CTTTNTGGGC TGGAAAAGCA GGTGAGGNTG GGGTGGGCTG AGGCCATTNC 60
CGCCACTATC TGTTTAATAA AATCCGTGAG CACGAAA 97

SEQ ID NO:5516
SEQUENCE LENGTH:225
SEQUENCE TYPE:nucleic acid
TOPOLOGY:linear
CLONE:HUMGS06559
SEQUENCE DESCRIPTION:
GATCCTCCGG AAGTAGTCTC TNCCCTCGGC AGCGGAAATA CTGATTCCCA CTGCTCCTNC 60
CTCTAGGGTG CAGTGTCCGT ACCTNCTGGA GCTGGNCCCT CCTTCCCCAN CCCAGACATT 120
GAGAAACTTG GGAAGAAGAG AGAAACCTNA AGCTCCCAAA CAGCACGTTG CGGGAAAGAG 180
GAAGNGAGAG TNTGAGTNTG TGTGTGTNTT TTTCCTATTG ANCAN 225

SEQ ID NO:5517
SEQUENCE LENGTH:340
SEQUENCE TYPE:nucleic acid
TOPOLOGY:linear
CLONE:HUMGS06560
SEQUENCE DESCRIPTION:
GATCATTAAT AAAGCTTTTC TTGGTTCCTT CAGTGGTGTT GGTAGTAAAA TGGAAGGNGT 60
CTTGCTGCAG GTAACTAATG AAGANGTGGT CAACCACAGA GTCTTCAAGA AATAAGAAAT 120
TCTGTACCAN CTGAAAGTAG TGCTTGTTGG TGCCTTCNTT TAAAAAGCAC TCTTTTAAAT 180
AAAAGGGAAA TGTTTTCTGA TAAACCAAAC ATTTAGTTGA GGTTCTTGAT ATAAAACATT 240
TACAANNTGA GTGTTGTNTG TAAAGCAGTA GCCNTCATTA TTGGCTAGNG AGNTAAATGN 300
GTCANGTTTT ANCTAGGTTT TNGTGAGTNG NCAGGTTTNN 340

SEQ ID NO:5518
SEQUENCE LENGTH:330
SEQUENCE TYPE:nucleic acid
TOPOLOGY:linear
CLONE:HUMGS06561
SEQUENCE DESCRIPTION:
GATCAAAGCC AGTNTTAAAT GGGAGACCGA GCGAGCGCGG CAAGTGCTGG AACACCTGCT 60
GAAGGAAGGG TTGGCGTGGC TGGACTTACA GGCCCCAGGG GAGGCCCACT ACTGGCTGCC 120
AGCTCTCTTC ACTGACCTCT ACTCCCAGGA GATTACAGCT GAGGAGGCCA GAGAAGCCCT 180
CCCCTGACTG CATGTGGAAG GGCACACAGC AGCAGGCAGG GAGGAGGCGG AGGTGGCAAA 240
TAAACCTGGG CAATTTTGTT TATACAAAAA ATAGAAGAAA AGTTCCAAGT TTTTCTTTCT 300
TCCCTCATTC TTTTNTTTTT GAAAAAAGAN 330

SEQ ID NO:5519
SEQUENCE LENGTH:111
SEQUENCE TYPE:nucleic acid
TOPOLOGY:linear
CLONE:HUMGS06563
SEQUENCE DESCRIPTION:
GATCTCATGA TGATTCANAC CACAGNCTGG GNTAAAGATG TCTTGACCTC TGAGGCTAGC 60
ATAACATATA TNAGAATACA NCTTGCCTAT TTCCAGAAAA TCTGTATATT N 111

SEQ ID NO:5520
SEQUENCE LENGTH:130
SEQUENCE TYPE:nucleic acid
TOPOLOGY:linear
CLONE:HUMGS06564
SEQUENCE DESCRIPTION:
GATCAGCTTA CTGAAAAGAA GCCTCCNNAG TATTCCAAAT ACAGATTACA TCCAGCTGCT 60
TAGTGAAATT GCCAAGGNAC AAGGNTTTAA TATAACATAT TTGGATATAG NTGAACTGAG 120
CGNCAATGGN 130

SEQ ID NO:5521
SEQUENCE LENGTH:113
SEQUENCE TYPE:nucleic acid
TOPOLOGY:linear
CLONE:HUMGS06565
SEQUENCE DESCRIPTION:
GATCNACAAT NTGAATATGT GTTACTTAAT AAGGCTAGGC TGGCCATCAG TNGCTTANTT 60
CAGATGTGTC ACTAAATTTC CCNTCTAGAT GGTCCTTGAG CAAAACTTAA TAN 113

SEQ ID NO:5522
SEQUENCE LENGTH:271
SEQUENCE TYPE:nucleic acid
TOPOLOGY:linear
CLONE:HUMGS06566
SEQUENCE DESCRIPTION:
GATCATCACA GTTTGAGTTT GACCCCAATG CCAGGATGGT GACACACATA TTAAAGAGTA 60
ACAGCTGGTT ACATTGCTAG GCGAGATAGG GGGAAGACAG ATATGGGTGT TTTTAATAAA 120
TCTAATAATN ATNCATCTAA TGTATTATGA GCCAAAATGG NTAATTTTTC CTGCATGTTC 180
TGTGNNTGTN GAAGATGAGC CTTGCAGATA TCTGCATGTG TCATGNNGAA TGNTTCTGGA 240
ATNCTTNACT TGCTNTTGGA NTTGCACTGG N 271

SEQ ID NO:5523
SEQUENCE LENGTH:126
SEQUENCE TYPE:nucleic acid
TOPOLOGY:linear
CLONE:HUMGS06567
SEQUENCE DESCRIPTION:
GATCTCACTT TCCCCTTGTG GGGTAGGAAC CGATGCCAGT GGGAGGGATG TGCCCCTGAC 60
CATTAACGAC TGTTTTTTTT TTTTTTTTTA AAAAAATGGN GTTNTTGGGN GGGGNAATNC 120
ACANAN 126

SEQ ID NO:5524
SEQUENCE LENGTH:156
SEQUENCE TYPE:nucleic acid

TOPOLOGY:linear
CLONE:HUMGS06569
SEQUENCE DESCRIPTION:
GATCACGGGA ATGCTGCTGG AGATAGACAA CTCTNAGTTG CTGCACATNT TAGAGTCCCC 60
CGAGTCTCTC CGCTCCAAGG TGGATGAAGC TGTAGCAGTT CTACAGGCTC NTCATGCCAA 120
GAAAGAAGCT GCCCAGAAGG TGGGCGCTGT TNNNNN 156

SEQ ID NO:5525
SEQUENCE LENGTH:133
SEQUENCE TYPE:nucleic acid
TOPOLOGY:linear
CLONE:HUMGS06571
SEQUENCE DESCRIPTION:
GATCCTTCTA GACCAAGGAC CCATTGTTAA AAGCATGGAT TCTGCCTGAN TTACTTCCCT 60
TTTGAGAAAT CATATCTCAA ATACATAACC TGGTAATATA ACTGAAAAAA TAAAAGTGAT 120
TGCTCCTTCC AAA 133

SEQ ID NO:5526
SEQUENCE LENGTH:156
SEQUENCE TYPE:nucleic acid
TOPOLOGY:linear
CLONE:HUMGS06572
SEQUENCE DESCRIPTION:
GATCCCTGCT TCATAGATTT GCATCATTCA AGCATATCTT GTAAAACAAA CACATATTAT 60
GGGACTAGGA AATATTTATC TTTCCAAATT TGCCATAACA GATTTAGGTT GCTTNCCTTN 120
CTTTGAAGGA AAGTTTAATT ACATTGCTCT NNNNNN 156

SEQ ID NO:5527
SEQUENCE LENGTH:63
SEQUENCE TYPE:nucleic acid
TOPOLOGY:linear
CLONE:HUMGS06573
SEQUENCE DESCRIPTION:
GATCCTAGCT GCAGATTGCA TCCCACAATG CGAGAATAAT AAAATAAAAT TNGATATTNG 60
AAA 63

SEQ ID NO:5528
SEQUENCE LENGTH:207
SEQUENCE TYPE:nucleic acid
TOPOLOGY:linear
CLONE:HUMGS06574
SEQUENCE DESCRIPTION:
GATCNATGGT GTGACTCAAG CTCCNCCTTA CCCAGGGGGG GTAAGGGCAG GCCTCTAGCT 60
ACTTGGAGTT GTCTGTAATA ATCTNGAAAG GCCCAAGGGC CTGTGCCCAT CCTGACTNAA 120
AGGNATCTCC TTCCCTGTTT CATATCACAT GACAGAGAAA CCTGTTCTCA TGGCATGNAA 180

CATCCCTGTN AAGAGAGCGT TGNCNNN 207

SEQ ID NO:5529
SEQUENCE LENGTH:190
SEQUENCE TYPE:nucleic acid
TOPOLOGY:linear
CLONE:HUMGS06576
SEQUENCE DESCRIPTION:
GATCTTGCAC ATGAAGCTAC AAAACTGGTA AGAAACAAAT GGCACATGTG NTGGGAATGG 60
AAGTCAACCT TTTTGAATTT TTCAAATGTA AGTCAGTCTG GAAAATCCCA GTAAATTGGT 120
ATATTTGTTT TGGGTTATTT TTGAAGACCT CTTTTAAAAT TTGCCTAAAA TAAATNCTCG 180
GTATGCTAAA 190

SEQ ID NO:5530
SEQUENCE LENGTH:341
SEQUENCE TYPE:nucleic acid
TOPOLOGY:linear
CLONE:HUMGS06577
SEQUENCE DESCRIPTION:
GATCCGCCGC CAGAAAGGTT ATGCATCATG TGCCACAGCC TCCAAGGTGC CCATCATGGA 60
ATNTCGTGGG GGCTGTGGGC CNANTGCTGC CAGCCCACCC GCAGCAAGCG NCGGAAATAC 120
GTCTTCCAGT GCACGGNTGG CTCCTCGTTT GTAGAAGAGG TGGAGAGACA CTTAGAGTGC 180
GGCTGCCTCG CGTGTTCCTA AGCCCCTGCC CGCCTGCNTG CCACCTCTNG GACTCCAGCT 240
TGATGGAGTT GGGACAGCCA TGTGGGACCC CCTGGTGATT CAGCATGAAG GANATGAAGC 300
TGGAGANGAA GGTAAAGGAG GAAGAGANTA TTANGTATTT N 341

SEQ ID NO:5531
SEQUENCE LENGTH:144
SEQUENCE TYPE:nucleic acid
TOPOLOGY:linear
CLONE:HUMGS06578
SEQUENCE DESCRIPTION:
GATCTNTTCC AACAGTTCTN TGTAAACCAA ATGCTATTTT TCANTGCATT TGGACTGCTG 60
ACCATTTAAA AGCAGCCTAT GATTGCTTCT TTTTGTAAAG CAAGCAACCN GCCTCCACTT 120
TAGCTGTTTT NACTATTNGA TNTN 144

SEQ ID NO:5532
SEQUENCE LENGTH:224
SEQUENCE TYPE:nucleic acid
TOPOLOGY:linear
CLONE:HUMGS06579
SEQUENCE DESCRIPTION:
GATCCTAAAA CGAAGAGAAT TTTGACTCTC CAGAAAGCTA ATAGCTGGTT CAACTGCAGG 60
AAAAATTGAA CATTACTAAT TTGAATGGAA AACACATGGT GTGAGTCCAA NGGAGGTGTT 120
TTCCTGAAGA NCTGTCTATT TCCTCAGTCA TTTTTAACCT CTAGAGTCAC TGATACACAG 180

AATATAATCT TATTTATACC TCAAANNGAN TNANANGGGG NNNN 224

SEQ ID NO:5533
SEQUENCE LENGTH:345
SEQUENCE TYPE:nucleic acid
TOPOLOGY:linear
CLONE:HUMGS06580
SEQUENCE DESCRIPTION:
GATCTNCCTC NACACCTNGG GCAAAGTCTA TGCGAAGATG GTTTCTTAGC TCTCCATTTG 60
CCATGATTTT CCTCCCATTC ATCATGAGGG AGTTTCTCAA ACCAGGAGTT NATATTTATT 120
TNTNAGAAAA TACACACTTT TCAGGAGAAA CCTGAGCATG ATTTTGGATT CTCCACCTCC 180
CCCCAGTCTC GGNNNCTGGG ATTCAGCTCA AGGNTTCAGT GTCTTCATTT TTACAAAAGT 240
TCCCCCAAGA AATCAGCAAC CAGCCTCTGT TTCATCTGGG AGCCCCTCCC TNGGNCCCCT 300
GGGTTTTGGG GGGTGCTGNC CTACTGGGGG AACAGCGGGG GGTGN 345

SEQ ID NO:5534
SEQUENCE LENGTH:179
SEQUENCE TYPE:nucleic acid
TOPOLOGY:linear
CLONE:HUMGS06581
SEQUENCE DESCRIPTION:
GATCATNTTG GAACCAGACA AGAATCCAAA GAAAATAAGA ACACAGACCA CCAGTGCAAA 60
ACAAGAAAAA GCACCAAGTA AAAAGCCAGT GAAAAGAAGA AAAAAGAAGA GAGCTGCCAN 120
TTAAAGCTAA CAGTTGTATA TCTGTATATA TAACTATTAA AAGGGATATT TATTCCAAA 179

SEQ ID NO:5535
SEQUENCE LENGTH:287
SEQUENCE TYPE:nucleic acid
TOPOLOGY:linear
CLONE:HUMGS06582
SEQUENCE DESCRIPTION:
GATCAAAGTT AAGNTTATAT CCTGTTTGTA GTATCAGATA TNTTCCTGTG TACAATTATA 60
GGATTGTAAT CTACACTGGA ATTTTNAGGC AGTAAGTCAC CACAAAATGT TTTAGATAAG 120
ACACAATAAA ATTATTATAA ATAAAAGCTT AATGTTTGTA AAAANTCTCT TTTTTAGTAT 180
TTCTTTTTTC ACATGNAAGN NGTGGTGGCT GCTAAANAAA NAGCTACAGT GTTTATTANG 240
GGTCGTTTTG ATTTNTGTNA NTCTTTGTAA ATTGGTCAGT GCCTGTN 287

SEQ ID NO:5536
SEQUENCE LENGTH:258
SEQUENCE TYPE:nucleic acid
TOPOLOGY:linear
CLONE:HUMGS06583
SEQUENCE DESCRIPTION:
GATCCCAGAG GCTCTCCTCA CCTCANCTGA GCTCCTTTAA AAGTAATTCA AGGGACTATG 60
TCACTCAGCC TCATTTNCTG GACCAAATCT GGAGGGAGAA CCCCTAAAAC CCCTAAGTAA 120

GGTTGCCCAG GGGGTTGTCC CCAGGTGGGG GGAAGCAGGG GAGAGAAAAT GGTAGCCATT 180
TTAACATTGT TTTGTATAGT ATTTATTGAT TCAGGAAACA AACACAAAAT TCTGAATAAA 240
ATGACTTGGA AACTGAAA 258

SEQ ID NO:5537
SEQUENCE LENGTH:248
SEQUENCE TYPE:nucleic acid
TOPOLOGY:linear
CLONE:HUMGS06584
SEQUENCE DESCRIPTION:
GATCGCTTGA GGCCAGTTCA AGACCAGCCT GGCCAACATG GCGAAACCCG TCTCCACTAA 60
AAATACAAAA CTTAGCCAGG CGTGGTGGCG CGCGCCTGTA ATCCCAGCTA NTCGGGAGGC 120
TGAGGCAGGN GAATCGCTTG AACCCGGGAG GTGGAGGTTA CAGTGAGCCA AGATTGCACC 180
ACTGCACTCC GGCCTGGGCA NCATNGCGAG ACTCTGTCTC AGNNAGGAGG GGGGGGTTNN 240
NNTANTNN 248

SEQ ID NO:5538
SEQUENCE LENGTH:198
SEQUENCE TYPE:nucleic acid
TOPOLOGY:linear
CLONE:HUMGS06585
SEQUENCE DESCRIPTION:
GATCCTGAAA GAGGACAAAC TACATTGCCT TTTAGACCTN TGACTCAAGA AGAAGATGAT 60
GACCAAAGGT GATTTGTAAC TTAACATGCC TTNTCCTGAT NTTGAAGGAT TTGTGAAGGG 120
AAAAAAAATTC TNGGCTCTTT GTATATAATA AAATGAGACT ANANGCATTC TNNAAAAAAA 180
AAAAGATTAA NTTTAGAN 198

SEQ ID NO:5539
SEQUENCE LENGTH:188
SEQUENCE TYPE:nucleic acid
TOPOLOGY:linear
CLONE:HUMGS06586
SEQUENCE DESCRIPTION:
GATCCCCACA CTTACTCTAC CCTCTNGGCA AATTGGCATT CCGGTGGTGG GTTTTGTNTC 60
CTTTAACACA TTAAATAAAT NAGTATATAG GATGTGAGGG GAGGGGTGAG ANCAACTAGC 120
TGTAGCATGT NTAGGCTATA TACTTTACCA NTTGACTTCT TTCCTGTTTT TNTTTNAAAA 180
NAAAAAAN 188

SEQ ID NO:5540
SEQUENCE LENGTH:138
SEQUENCE TYPE:nucleic acid
TOPOLOGY:linear
CLONE:HUMGS06587
SEQUENCE DESCRIPTION:
GATCTGGGTG TGCTCTATGG GGAGTNCTGA CGTCAAANAG CAAATGTCTA TAAGGGGCCC 60

TTTTAAAATN AACATTTTCC TCATTGAGCA AGCNTGGATT CTCTAATGTA GAAATCAAGC 120
CATCTTNATA ATTNCACN 138

SEQ ID NO:5541
SEQUENCE LENGTH:332
SEQUENCE TYPE:nucleic acid
TOPOLOGY:linear
CLONE:HUMGS06588
SEQUENCE DESCRIPTION:
GATCCTCCCA CCTCAGCCTG CTGAGTAGCT GGGTCTACAG GTGCACACCA CCATGCCTGG 60
CTAAAGGCAT GTCTGCATTT ATGCGAGGTG TGTTGACCGG GTGGGGAGGG AGCTAGGACT 120
GAGATTCGCC TGTNTAAAAT GCACAAAAAG GGTTTGCTAA GCAAATTGAT ATTTGAACTA 180
AAAGATGGAT TAGGGTGAGA GACTTAGTTT ANTCAGGAAT AGTNGCAGAG NCANCCACAN 240
CAANAANTGN ATTTCCCTGN GACAGCCGTT TGTACGTACA GCAGGGNNCT GGTTTACAGN 300
TTTCACGGAG GGNGGNTAGG GTTCTTNTAC TN 332

SEQ ID NO:5542
SEQUENCE LENGTH:106
SEQUENCE TYPE:nucleic acid
TOPOLOGY:linear
CLONE:HUMGS06590
SEQUENCE DESCRIPTION:
GATCAAATNA TTTGACTATT GCTAGACATT TCTATACTCT GTTGTAACAC TGAGGTATCT 60
CATTTGCCCA TGTTAATTTT TTTCTAAATA AATTGACAAA AACAAA 106

SEQ ID NO:5543
SEQUENCE LENGTH:268
SEQUENCE TYPE:nucleic acid
TOPOLOGY:linear
CLONE:HUMGS06592
SEQUENCE DESCRIPTION:
GATCACAGCC TCCAGGGCCC CCCAAATCCC AGGGAAGGAC TTGGGAGAGA ATCATGCTGT 60
TGCATNTAGA NCTTTCTGCT TTGCACAGGA AAGAGTCACA CAATTAATCA ACATGTATAT 120
TTNCTCTATA CATAGAGCTC TATTTCTCTA CGGTTTTATA NAGGCCTTGG GGNTCCAACC 180
AGGCAGTAAG ANGTGCTTCT GAACCGCAAG GGGCGNACGA CTGGAAGTAA AATAGTTTGT 240
TTTTCNCANT TCGNGGGNAG GGGNNGGN 268

SEQ ID NO:5544
SEQUENCE LENGTH:177
SEQUENCE TYPE:nucleic acid
TOPOLOGY:linear
CLONE:HUMGS06593
SEQUENCE DESCRIPTION:
GATCCAATTA ACAAATTCTG CACTGTGGCA ACTNAACGAN ATTATGCTTT GGACAGCTTT 60
ATGTGTGAAT GTAAGAACAT ATTCTNAAGA AATGCTGTAA CTTTATCCCA TATANCTTCA 120

TANGAATTTC TCTATAGGCT TGGNATTTGC AATAAAAATA GAGGATATGT TGGCAAA 177

SEQ ID NO:5545
SEQUENCE LENGTH:105
SEQUENCE TYPE:nucleic acid
TOPOLOGY:linear
CLONE:HUMGS06594
SEQUENCE DESCRIPTION:
GATCANACAG CCTACANCCA GCCCCGAGTG TGCATCANGG TAAAAGAGCT GAGGGCTCTC 60
TNCAGGGAGC AGCCCATTTA GGTCTCTGTN GTTGTTGTTA GGGAN 105

SEQ ID NO:5546
SEQUENCE LENGTH:149
SEQUENCE TYPE:nucleic acid
TOPOLOGY:linear
CLONE:HUMGS06595
SEQUENCE DESCRIPTION:
GATCATATNA GACGAGNAAG AGGAAGACCT AAAAGTGCAT CTGCCAAAAA ACATGAGGAA 60
GAAAGTGAGA AACAGGAAAA GGAAATTNAC ATCTATGCTA ACCTGTCTGA TGAAAAGNCT 120
TTCGTGTTTT CAGTCGCCTT NGCAGCNNN 149

SEQ ID NO:5547
SEQUENCE LENGTH:411
SEQUENCE TYPE:nucleic acid
TOPOLOGY:linear
CLONE:HUMGS06596
SEQUENCE DESCRIPTION:
GATCAGTGTG AGTCCTGAAG CACTTTNAGN GCTGTGNGAA CGACATCCAC TTTGGGTTTC 60
ATTCGTTTGT AAGCAGAGGA GCTGTCAGTC ACTCGTGCTT GTCGGTGGCC TCTGAGCCAT 120
GGTGTCGAGT GAAGAGTAGT TCTTGTTTGT TACAACCTTT GTGAGTCAGC CATGCCCGCA 180
AAGCGTGCTG TGTTTTAGTC CTGGTAGGAA TATTTATNAG AGTTCACACT ATGTAAAACC 240
CAACNGCTTC ANCTATTGCC CTTTCAACAG TTTTGCCACT GACCGGATAG AACCGGTTTC 300
AGTCTCTGGA TGGGTGTGTT TGGTGGGTTT GTAACCATTG ACGGGTTTAA ANCCATGGGT 360
TTNAAGATTT GGCCCAAATA ACCAGNAATT TTGTTCCGGG GAGGGGGTTN N 411

SEQ ID NO:5548
SEQUENCE LENGTH:79
SEQUENCE TYPE:nucleic acid
TOPOLOGY:linear
CLONE:HUMGS06597
SEQUENCE DESCRIPTION:
GATCATAAAA GGCTATTATC TATNCTGGGG AACGATTATA ACTTAAATAA TTATTTTAAT 60
AAAAATACTA AGCTNNAAA 79

SEQ ID NO:5549

SEQUENCE LENGTH:102
SEQUENCE TYPE:nucleic acid
TOPOLOGY:linear
CLONE:HUMGS06599
SEQUENCE DESCRIPTION:
GATCATCCTG CAAGTGCTCT NGTGCCTAGG ATTCATCTTT CTTTTCACCG TAGGCCCTGA 60
CTGGCATTNT ATTAGCAAAC TCATCACTAG TCGTNGTNCT NN 102

SEQ ID NO:5550
SEQUENCE LENGTH:166
SEQUENCE TYPE:nucleic acid
TOPOLOGY:linear
CLONE:HUMGS06600
SEQUENCE DESCRIPTION:
GATCAAAACG ACGAAGAAAN GNCNCANAGA CTCAAATAAG GACAGAGTGA TTTCCAATAA 60
TGTTCAATAG ATTTAGGAGC AGAAATGCAA GGGGCTGCAT GACCTACCAG GACAGANCTT 120
TCCCCAATTA CAGGGTGACT CACAGCCGCA TTGGTGACTC ACNNNN 166

SEQ ID NO:5551
SEQUENCE LENGTH:219
SEQUENCE TYPE:nucleic acid
TOPOLOGY:linear
CLONE:HUMGS06601
SEQUENCE DESCRIPTION:
GATCTCAGTT TCAAATCTCC CCAAGCTGCC TAAATTGAAA AAGCTTGAAC TCAGTGAAAA 60
TAGATTCTTT GGAGGTCTGG ACATGTTAGC TGAAAAACTT CCAAATCTCN NACATCTAAN 120
CTTAAGTGGA AATAANCTGA AAGATATCAG CACCTTGGAA CCTTTGAAAA AGTTAGANTG 180
TCTGANAAGC CTGGNCCTCT TTANNTGTGA GGTTNCCAN 219

SEQ ID NO:5552
SEQUENCE LENGTH:104
SEQUENCE TYPE:nucleic acid
TOPOLOGY:linear
CLONE:HUMGS06602
SEQUENCE DESCRIPTION:
GATCTCTTNG CGACAGTACC AAGCACGGTT CTCTACACAG GTGACTNAAG TTGCCTCTGT 60
GTTGGCTGGC ATCCCTGAGT CCCCTNCGGG CTCCTATGGA NCNN 104

SEQ ID NO:5553
SEQUENCE LENGTH:94
SEQUENCE TYPE:nucleic acid
TOPOLOGY:linear
CLONE:HUMGS06603
SEQUENCE DESCRIPTION:
GATCATCTGA GCCTCAGGAG GTTGAGGCTG NAGTGAGCTG TGACTNCGCC ACTGNACTCC 60

AGTCTGGGAC AACAGAGCAA GACCCNGTCT TAAA 94

SEQ ID NO:5554
SEQUENCE LENGTH:78
SEQUENCE TYPE:nucleic acid
TOPOLOGY:linear
CLONE:HUMGS06604
SEQUENCE DESCRIPTION:
GATCTCCAGC TGCACAGGCC ACCGNCCCAG GGCGTGGCCG CTGTTACAGA AACAATAAAC 60
CCTGATGGGC ATGGCAAA 78

SEQ ID NO:5555
SEQUENCE LENGTH:191
SEQUENCE TYPE:nucleic acid
TOPOLOGY:linear
CLONE:HUMGS06605
SEQUENCE DESCRIPTION:
GATCGTAACA ATGCCAAGAA GACACAAGAA TATGAGAAAG GAATTCCTAG AAAAAAGGCA 60
AAGGTTTCAA TGACTAAGCT AATTTTTNCT AAGTCTACTG ATAACATACT CCCTACCCCA 120
TCCTCCATTA ATAAAAGANT GGTTGTTGTA TTGTATTATG AGGGAATTAA AAGTGTGATA 180
NGANANNNNN N 191

SEQ ID NO:5556
SEQUENCE LENGTH:102
SEQUENCE TYPE:nucleic acid
TOPOLOGY:linear
CLONE:HUMGS06606
SEQUENCE DESCRIPTION:
GATCGGAGAA TTGTGTAGGC GAATAGGAAA TATCATTCGG GCTTGATGTG GGGAGGGGTG 60
TTTAAGGGGT TGGCTAGGGT ATAATTGTCT GGGTCGCCTA AA 102

SEQ ID NO:5557
SEQUENCE LENGTH:135
SEQUENCE TYPE:nucleic acid
TOPOLOGY:linear
CLONE:HUMGS06607
SEQUENCE DESCRIPTION:
GATCTCACTT CTGTCAGATG CTCCCATCCA CTATNAATGC ACTAGGTGGG AGGAGAGGGC 60
GGCAATGACA CTGCACCTCT CCTTTCCCAC CGCATTCCCT GGAGCTCCCT AAATAAAACT 120
TTTTTTAACG TGAAA 135

SEQ ID NO:5558
SEQUENCE LENGTH:182
SEQUENCE TYPE:nucleic acid
TOPOLOGY:linear

CLONE:HUMGS06608
SEQUENCE DESCRIPTION:
GATCAAGTAA AAATGCAAAT CAATGATGCA AAGAAAACAG AAAAGAAAAA GAAGAAAAGA 60
CAGGATATTT CTGTTCATAA ATTAAAGCTG TAATATATTT NNANTATAAT GTAAATATTA 120
ATGTGTAAGC TTATATTGTG TCATTGTNCT GTTTTATAAT AAAATNCTTG AGAACCTTCA 180
AA 182

SEQ ID NO:5559
SEQUENCE LENGTH:125
SEQUENCE TYPE:nucleic acid
TOPOLOGY:linear
CLONE:HUMGS06609
SEQUENCE DESCRIPTION:
GATCTTGACC TCACGGTGTA AGAAGGGAGA GGATGGTTTC TCTTCTGCCC TCTCCCATGG 60
TGCCCGTGCC TCTNTGCTGT GTATGTGAAC CACCCACGTG AGGGAATAAA CCTGGCACTA 120
GGAAA 125

SEQ ID NO:5560
SEQUENCE LENGTH:195
SEQUENCE TYPE:nucleic acid
TOPOLOGY:linear
CLONE:HUMGS06610
SEQUENCE DESCRIPTION:
GATCTTTTTA GATACAATCC TTATTTAAAA AGGAGAAGAT AACTGAGGAT TTTAAAAAGA 60
AGCCATGGAA AAACTTCCTA GTAAGCATCT ACTTCAGGCC AACAAGGTTA TATGAATATA 120
TAGTGTATAG AAGCGATTTA AGTTACAATG TTTTATGGCC TAAATTTATT AAATAAAATG 180
CACAAAACTT TGAAA 195

SEQ ID NO:5561
SEQUENCE LENGTH:131
SEQUENCE TYPE:nucleic acid
TOPOLOGY:linear
CLONE:HUMGS06611
SEQUENCE DESCRIPTION:
GATCTTGTGC TCAACATGAT NCNTTAATAG AAGTTTTATN TTTCNGTGCA CTCTGCTAAT 60
CATGTGGGTG AGCCAGTGGA ACAGCGGGAG ACCTGTGCTA GTTTTACAGA TTTCCTCCTA 120
AATNNCGCGG N 131

SEQ ID NO:5562
SEQUENCE LENGTH:196
SEQUENCE TYPE:nucleic acid
TOPOLOGY:linear
CLONE:HUMGS06612
SEQUENCE DESCRIPTION:
GATCTAATAG CTGAACTGAT TCAAACTGTA ATAAGCTCAT CAATTTCANT TCTATGAAAA 60

TGTGCTCTGT TGTCACAGGA TGTTTCTGTT GGTTTTNTTC ATTTCCTGGG AATTGGTAAA 120
CATCATGTTC CNGATGATAA CCCAGTAGCA AAANCATTTG TNCTGTGTGG TACAAGCCTT 180
NGGGACTGNN AAANAN 196

SEQ ID NO:5563
SEQUENCE LENGTH:307
SEQUENCE TYPE:nucleic acid
TOPOLOGY:linear
CLONE:HUMGS06613
SEQUENCE DESCRIPTION:
GATCCCATAA TATAAATCAT ATCCTTTATA TTTNAGAATA TCTCAAATGT ATTCCTTTTT 60
TGTATGGTGG GTTTGCCTAG GGACGTGTAA CTACAGGCTT TTACTAAGCC AAGGAAAAAG 120
AGAATTTTNC TTTTCATCTT ACAAATTCCA GATATCTACA AANGNTGTGA AAGCACTAAA 180
AGNGNNGNTT TTAAGCAGTA CTTTACCTGT TTTTCCTTTA GCAAACCAGG TTATGTGGTG 240
TAAAGGTTTG TNATACGTGC CACAATATAG CATATAAATG TTATGCCATC ATTCCTTCTC 300
TTGTTAN 307

SEQ ID NO:5564
SEQUENCE LENGTH:188
SEQUENCE TYPE:nucleic acid
TOPOLOGY:linear
CLONE:HUMGS06614
SEQUENCE DESCRIPTION:
GATCTATACA TGAACAGCAG AATGTATAAG CTAGCATTTA CTAGCATGTC TCTNATACTT 60
TTGTNCATTT CTGTTAATCG TCATTTCTGT CCCCTCTGCA TGGAATTTNA TACTTGTCTA 120
GGGTAAGAGT TTGTAGACTC TTTCAGCAGA ACAGATTTTA ATAACCAATT TATNTGTTTT 180
ATTTTAAA 188

SEQ ID NO:5565
SEQUENCE LENGTH:178
SEQUENCE TYPE:nucleic acid
TOPOLOGY:linear
CLONE:HUMGS06615
SEQUENCE DESCRIPTION:
GATCAGCACA TCCAAAAACA CCAAGACAAA CATCCATGTG GAGCAGAACC TNTCCTGCCT 60
CGGGAGTTCT GACCAGGTTC CCCAGCAGGG TGTTAAGCCT CTGTNTCTGG CCCTACCAGC 120
ATNCGGGTTC CACTTTTCCT AGGAGAGNGT GGAGATGGGN NGACAGGGAA AGGNNGGN 178

SEQ ID NO:5566
SEQUENCE LENGTH:104
SEQUENCE TYPE:nucleic acid
TOPOLOGY:linear
CLONE:HUMGS06616
SEQUENCE DESCRIPTION:
GATCTATCAT TTTAATTGAT TCTGAAGCTT GTATTAAAAA CTAGGCAATA NCATCATGGG 60

ATACATAGGA GANGGCACAT TTTACAATCA TNCATTNTGG CCNN 104

SEQ ID NO:5567
SEQUENCE LENGTH:130
SEQUENCE TYPE:nucleic acid
TOPOLOGY:linear
CLONE:HUMGS06617
SEQUENCE DESCRIPTION:
GATCTGCTGA CAAAACCTGG GAATTTGGGT TGTGTATGCG AATNTTTCAG TGCCTCAAAC 60
AAATNTGTAT TTAACTTATG TAAAAGATAA GTCTGGAAAT AAATGTCTGT TTATTTTTGT 120
ACTATTTAAA 130

SEQ ID NO:5568
SEQUENCE LENGTH:80
SEQUENCE TYPE:nucleic acid
TOPOLOGY:linear
CLONE:HUMGS06618
SEQUENCE DESCRIPTION:
GATCATTTAA CATTCTGTGT ATGTAACAAA ATATCACATG CATAAATATT ATGTATCAAT 60
AAAATTTTTT AATGGGCAAA 80

SEQ ID NO:5569
SEQUENCE LENGTH:58
SEQUENCE TYPE:nucleic acid
TOPOLOGY:linear
CLONE:HUMGS06619
SEQUENCE DESCRIPTION:
GATCCGGTCA CTGCACTCCA GCCTNGGAGA CTACAGCAAG ACTCTNTCTC CAGTTAAA 58

SEQ ID NO:5570
SEQUENCE LENGTH:188
SEQUENCE TYPE:nucleic acid
TOPOLOGY:linear
CLONE:HUMGS06620
SEQUENCE DESCRIPTION:
GATCTGAGGG GTGGTGCCTA CTTCCACTGT AAGAAAGAAT CTNGGTGGAT TTGTGTNTCA 60
AATCANGATA AGAGAAGCCT GTTTAAAGGA GCAGATGCCA TCTNCTGNCT TCCTCAAGGA 120
GCCAGTTAAA AAACCAGAGC ATTCCTTTTN ATTGAAAAAT AAAATTAGTT TGTTATCAAA 180
ANNNGGTN 188

SEQ ID NO:5571
SEQUENCE LENGTH:240
SEQUENCE TYPE:nucleic acid
TOPOLOGY:linear
CLONE:HUMGS06621

SEQUENCE DESCRIPTION:
GATCATAAAC CGTTGCGGGA CAGAAGTTAG ATATGTGATT GATTATNATG ATGGTGGTGA 60
AGTCAACAAG GACTACCAGT TCACCATCCT GGACGTCCGT CCTGCCTTAG ATTCACTTTC 120
GGCAGTATGG GACAGAATGA AAGTCGCTTG GTGGCGTTGG ACCTCGTAAA GCACTGTTTC 180
AGATGGAAAA NTATAANCTA TTTTTTNCTG AGCGATACAT TAAACTATTT TCCCCAGAAA 240

SEQ ID NO:5572
SEQUENCE LENGTH:197
SEQUENCE TYPE:nucleic acid
TOPOLOGY:linear
CLONE:HUMGS06623
SEQUENCE DESCRIPTION:
GATCCTCATC TTCCCCAAAT ACTCGTTCCC AAAATTGACG AGCCTGACAA TGTGCATGCC 60
AGGCAAGGCT CTTGGGGTTC CCCTAAAACA CTTCCTCTTT TAAGCCTACC ACTCACTCAT 120
CATGAATATA GTCCATTGTC CCAGGGTGTA AAACCCTCTA TAGTGTTAAA TAAAAGANTG 180
ATTGGGGAAC ATTGAAA 197

SEQ ID NO:5573
SEQUENCE LENGTH:109
SEQUENCE TYPE:nucleic acid
TOPOLOGY:linear
CLONE:HUMGS06624
SEQUENCE DESCRIPTION:
GATCTGGCTC AGTCTACTAT GGGCAGGGCC CCCCACCAAG CTGAGCCGAA TGGAGACAGC 60
TGAGCTGAGG CCTGACTTTT TCAATAAAAC ATTGTGTAGT TCTGGGAAA 109

SEQ ID NO:5574
SEQUENCE LENGTH:333
SEQUENCE TYPE:nucleic acid
TOPOLOGY:linear
CLONE:HUMGS06625
SEQUENCE DESCRIPTION:
GATCTGAGCT GATGACTTGT GAGAGAAAAA GGGAACAGAG TAAAGCCATG GAAGCCATGA 60
ACAGTAAGAG ACTGCCGCCT GGCATGGTTT CTTCTTCTGC AGAAGATGAA ACTGAGGAGA 120
AACAAGACAA CATCCTTCAT ACCAGGAATG GTCAAGATAA TGCAAGAAGA GNGNAGCTTT 180
CAAACAANTC AGNAGGCAGT CAACAAACAG NAAGGGGGNC ATTCCTTCCC TGGCAGTTAC 240
TCATANCTGA AATTGCTTAT TGTGTACACC GGGGCTTGTA CTTGGGGNAT TTAATANNAT 300
TGCTCATTNC CAAGCTCCNA ANNNAANTTA TTN 333

SEQ ID NO:5575
SEQUENCE LENGTH:132
SEQUENCE TYPE:nucleic acid
TOPOLOGY:linear
CLONE:HUMGS06626
SEQUENCE DESCRIPTION:

GATCGATTGC AGTCAGAGCC TGAAAGTATC CGTAAATGGA GAGAAGAACA AATGGAACGC 60
TTGGAAGCCC TTGATGCCAA TNNTNGGAAG CAAGANGCAG AGTGGAAAGA AAAGGCAATA 120
NNGGAGCTAG AN 132

SEQ ID NO:5576
SEQUENCE LENGTH:272
SEQUENCE TYPE:nucleic acid
TOPOLOGY:linear
CLONE:HUMGS06627
SEQUENCE DESCRIPTION:
GATCTTGGCT TCTNACAGTT CTNCCTGTCC AAACTCCATC CTGTCCCTCA GGAACGGGGG 60
GAAAATTCTC CGAATGTTTT TGGTTTTTTG GCTGCTTGGA ATTTACTTCT GCCACCTGCT 120
GGTCATCACT GTCCTCACTA AGTGGATTCT GGCTCCCCCG TACCTNATGG CTCAAACTAC 180
CACTCCTAAG TCGCTATATT AAGGCTTATA TTTTGNTGGA TTACTGCTAA ATACAAAAAT 240
NTTTGANAGG GANNGGNGNG TGNGGNGTGT NN 272

SEQ ID NO:5577
SEQUENCE LENGTH:133
SEQUENCE TYPE:nucleic acid
TOPOLOGY:linear
CLONE:HUMGS06628
SEQUENCE DESCRIPTION:
GATCATCCTG TCTGGCTGCA GTGAGAGACC AACCCCTAAC AAGGGCTGGG CCACAGCAGG 60
GAGTGCAGCC CTACCTTCTT CCCTTGGCAG CTGGAGAAAT CTGGTTTCAA TATAACTCAT 120
TTAAAAATTT AAA 133

SEQ ID NO:5578
SEQUENCE LENGTH:186
SEQUENCE TYPE:nucleic acid
TOPOLOGY:linear
CLONE:HUMGS06630
SEQUENCE DESCRIPTION:
GATCCACAAA CTNCCTGACA CTACTNNCAT ATTTCCACTA AAGGAGATTC AGCTACAAAA 60
GGAGGCCAGC CNTCGGTCTT CGTCGATGCC TGACTCAGGC ANGCCAGCTT TCCGCGTGGT 120
GGACACCGAG TTCTAAGCCA CAGACCGAGG CCCCAGCAGN CAGCGGCTGG AGGGNTGCCA 180
CANCNN 186

SEQ ID NO:5579
SEQUENCE LENGTH:136
SEQUENCE TYPE:nucleic acid
TOPOLOGY:linear
CLONE:HUMGS06631
SEQUENCE DESCRIPTION:
GATCCACTGC CTCGGCCTCC GAAAATCTGG AATNATAGAT GTGAGCCACT GCGCCAGGCC 60
TGTAATTTTA TGTATCTTTA TGTGATTATT TGAATGTCCC TCTCTGACTA GATTATAAAT 120

TCTAAGGATA GTGAAA 136

SEQ ID NO:5580
SEQUENCE LENGTH:66
SEQUENCE TYPE:nucleic acid
TOPOLOGY:linear
CLONE:HUMGS06632
SEQUENCE DESCRIPTION:
GATCCCCGTT CTCAAGTGTC NCNGGGAACA TAAACNGAGA TAACATGAAA AAACTTGACA 60
CAGAAA 66

SEQ ID NO:5581
SEQUENCE LENGTH:122
SEQUENCE TYPE:nucleic acid
TOPOLOGY:linear
CLONE:HUMGS06633
SEQUENCE DESCRIPTION:
GATCCTTNAA GGCAGCTGCC TTATCTTATT CCTTNTTGTA TTTCCATACC TAGCCTAATA 60
CCTAGCTTAT ATAAGGGAAC AGAATAAATA AACAATAAAG AAAAGTCTTT ACAGCCTCTA 120
AA 122

SEQ ID NO:5582
SEQUENCE LENGTH:262
SEQUENCE TYPE:nucleic acid
TOPOLOGY:linear
CLONE:HUMGS06634
SEQUENCE DESCRIPTION:
GATCCAGGGC CGTTCATGAA CCACTGGGCT GGATTNGACT GTTGAGTGTG GCAGTTAATG 60
CCCCTCAANA AATCAAAGGA TGTCTTATAA GTGTCTTCCA AAAAAAAGCA AATGCTGAAA 120
TCCTATTGGC AAAGTAAACT GAAATTGGCT GCTATATTTN ATATAATCAT TTCTGCAANT 180
CCCANTTNTT GNATACTNAT GTNGGACATG GTTANTTCTT ATTAATTTGT TGGAATTGTT 240
TATTGTTAGN AGTGCANATN GN 262

SEQ ID NO:5583
SEQUENCE LENGTH:275
SEQUENCE TYPE:nucleic acid
TOPOLOGY:linear
CLONE:HUMGS06635
SEQUENCE DESCRIPTION:
GATCCTGGCC AGTACAAAGT CGTTGCTCTT GTTTTATCTT CTCTTACAGA GTCTCCCTCC 60
CTTTATAGAA TGTCAACCAA AGAGTGCCCT CCTCCCCTCT CAGCCTCCTN TNNAGCTAGC 120
CTCCCCATCT CATCACAACG CATGTCTGTG ACCTTTGGTA ATCATTTACA GTGCCACACG 180
GAACCCTGTA TTTTGCACAC AGCAAAACAA ACAATGTTTA GCTTTATTTA TGGTATTTGA 240
TGCTGTAAAT GGAAATAAAT ATTGTTCTTT ATAAA 275

SEQ ID NO:5584
SEQUENCE LENGTH:170
SEQUENCE TYPE:nucleic acid
TOPOLOGY:linear
CLONE:HUMGS06636
SEQUENCE DESCRIPTION:
GATCTCTCTC GCCACGGCAC CGTCAGCTCC TTTGTGGCCA AGCTTTTCAT CTTTGTGTCT 60
GCAGAGCTCT GAGCACTCGC TTCGCGTCGC GGGGTCTCCC TCCTGTTGCT TTCCTAACCC 120
TGCCCTCCGG GGCGTTAATA AAGTCTTAGC AAGCGTCCCA CACAGTGAAA 170

SEQ ID NO:5585
SEQUENCE LENGTH:115
SEQUENCE TYPE:nucleic acid
TOPOLOGY:linear
CLONE:HUMGS06637
SEQUENCE DESCRIPTION:
GATCAGCTTT NAGAGTGGTC CTAAGCAGTA AACTGGATGA TGTTGCATTA GAAAAACATC 60
AATACTTCAT ATTTAAGTNT NAGTAGTTAC TACTGATTNG ATAATCACTT AANTN 115

SEQ ID NO:5586
SEQUENCE LENGTH:254
SEQUENCE TYPE:nucleic acid
TOPOLOGY:linear
CLONE:HUMGS06638
SEQUENCE DESCRIPTION:
GATCCTTGTG GACTGCCACT GCTGGCTGCC TCCTCTCCAA CTCATCAGAA GAGCATTTTG 60
ACAAAGACAG AGGACTTGGA CCAGGGCATG CCCTGTTGGG GATGTTTCTG TCAGCAAGGA 120
CATGGCATGG AATCAAGTGG GCTGATGTGT TGTTATTTAA ACAGTACCAA AGTGCATTCT 180
TCAATGTATG TTTGCCTGCT TGAAGCAAGG CCCAAGTTGA GAATACAGTA AAGGNAACTC 240
ATGAAAGTGA GAAA 254

SEQ ID NO:5587
SEQUENCE LENGTH:301
SEQUENCE TYPE:nucleic acid
TOPOLOGY:linear
CLONE:HUMGS06639
SEQUENCE DESCRIPTION:
GATCCCGCCC TGACCCAGAG GAAGCCTCTA TTTATTTATT AGCTTTTGTT TACACCGTGG 60
AATTGACCCC TTCCTCCAGG GGTCTTGGGT GGGGGAGCCC AGGGCCCCTG TGACCCCTCC 120
TTTCTTCCTC CAATCCCCAG TTTGTATTTA GCTGCCAAAT AAGATTCCCA TTGGNTCCCT 180
GTGTTCTCTT GGGGGGTCAG GGTGCTGTCC CCTNCNNTCT GTTTACATCT CCCCTNTACC 240
CCGCTGTATC GCATATTGNN GAGTTTNCTA TTTTTGCAAA ATAAAGTGAT GGAAACTCAA 300
A 301

SEQ ID NO:5588

SEQUENCE LENGTH:206
SEQUENCE TYPE:nucleic acid
TOPOLOGY:linear
CLONE:HUMGS06640
SEQUENCE DESCRIPTION:
GATCAGAGAA AATACGGTAC ATGTCCCCAT GGAGGATATG GCTTGGNCTT GGAACGATTC 60
TTAACGTGGA TTCTGAATAG GTATCACATC CGANACGTGT GCTTATACCC TCGATTTGTC 120
CAGCGTTGCA CGCCATAACC ATTTTCTCCA GAAGCGTGGA GGAAAGATTA TNNAAGGANC 180
AGGCTCTTCA AAAAAANNAN GNNAAN 206

SEQ ID NO:5589
SEQUENCE LENGTH:84
SEQUENCE TYPE:nucleic acid
TOPOLOGY:linear
CLONE:HUMGS06641
SEQUENCE DESCRIPTION:
GATCTTCTTC ACACCAAGCT CATGGTTACA TNCCGTGAGG TGTCATGAAG AAAGTNCTGT 60
TCAATAAGGT TTTGTAATGT TAAA 84

SEQ ID NO:5590
SEQUENCE LENGTH:150
SEQUENCE TYPE:nucleic acid
TOPOLOGY:linear
CLONE:HUMGS06642
SEQUENCE DESCRIPTION:
GATCTGTGGC TGGAATNGAG GGAGCGCCTG CTTCTTCTCC AGCTTGGAAA AACAATAGGA 60
GAAGGGTCAG CTCCGCGCGG GCATCATCCC TACCAGATTG AGTGTTTTGC ACGCACTTCA 120
ATCACAAAAT AAAACAGTGA GAAAGTCAAA 150

SEQ ID NO:5591
SEQUENCE LENGTH:123
SEQUENCE TYPE:nucleic acid
TOPOLOGY:linear
CLONE:HUMGS06643
SEQUENCE DESCRIPTION:
GATCATCATA TCAAGATGAT ACCAAAAGTA TGTAAAAAGA AACCTGCATT ATTTTGTAAT 60
NATTTCTTAT AGATATTTCA TGGTAAGATT AGCAGTCAAT AAAGTTACTT TTTTNCCTTT 120
AAA 123

SEQ ID NO:5592
SEQUENCE LENGTH:170
SEQUENCE TYPE:nucleic acid
TOPOLOGY:linear
CLONE:HUMGS06645
SEQUENCE DESCRIPTION:

GATCCTGCAA ATATCTTCTT TCCAGTTTGN TATTGTCATT TGNCTTTTGA CTTTGTTCTG 60
GTATTTTTTG NTATGTAGAA ATNTTTATTT TCATGTAAGC AAATTTATGA ATCTTTGNAC 120
ACCATAAGTA TATACAATTA TGATTTGTCA ATTAAAAATA TTAGTACAAA 170

SEQ ID NO:5593
SEQUENCE LENGTH:101
SEQUENCE TYPE:nucleic acid
TOPOLOGY:linear
CLONE:HUMGS06646
SEQUENCE DESCRIPTION:
GATCATCCGG TGAAAGAACC AGTCCCTGNG CCCCAAGGAT GCCGGGGAAA CAGGACCNTC 60
TCCTTTCCTG GAGCTGGTTT AGCTGGATAT NGGNGGNTTT N 101

SEQ ID NO:5594
SEQUENCE LENGTH:104
SEQUENCE TYPE:nucleic acid
TOPOLOGY:linear
CLONE:HUMGS06647
SEQUENCE DESCRIPTION:
GATCTCCGTG GGAACATTTT CGTTGCTGTC GGTGCTGGCA GGAGCCTGTT TCTTCCTGGT 60
CCTGAAATAT AGAGGCCTGA TTAAATACTG GTTTCACACT CAAA 104

SEQ ID NO:5595
SEQUENCE LENGTH:91
SEQUENCE TYPE:nucleic acid
TOPOLOGY:linear
CLONE:HUMGS06649
SEQUENCE DESCRIPTION:
GATCGTGCTG CACAACCGGA GAAGACAGAA TTACCTCTGC TCTTTTAATA TATAATGATG 60
GCTTTAAATA AANTTAGGAG AAAATGTCAA A 91

SEQ ID NO:5596
SEQUENCE LENGTH:126
SEQUENCE TYPE:nucleic acid
TOPOLOGY:linear
CLONE:HUMGS06650
SEQUENCE DESCRIPTION:
GATCCAGAGA GTGGGGGTGA GGGGTGGGCC CTGAAGACTT TTTGTCTGTA GCTCTTGCAT 60
ATTGAGTGTA TAAACCCGGC TTCTGGACCA ACCCAAGATG AATAAACTGG GGCAGAAAAT 120
TACAAA 126

SEQ ID NO:5597
SEQUENCE LENGTH:393
SEQUENCE TYPE:nucleic acid
TOPOLOGY:linear

CLONE:HUMGS06651
SEQUENCE DESCRIPTION:
GATCCTGGTG GCCACCAATN TGTTTGGCCG GGGGATGGAC ATTGAGCGAG TCAACATCGT 60
CTTTAACTAC GACATGCCTG AGGACTCGGA CACCTACCTG CACCNNGTGG CCCGTGCGGG 120
TCGCTTTGGC ACCAAAGGCC TAGCCATCAC TTTTGTGTCT GACGNGAATA ATGCCAAAAT 180
CCTCAATAAC GNCCAGGACC GGTTTGAAGT TAATGTGGCA GAACTTCCAG AGGAAATNGA 240
CATNTCCACA TACATCGAGC AGAGCCGGTA ANCACCACGT GCCAGAGCCG GNCCANCCTG 300
AGNCGACCGT ATTGTAGCTT TTAACTNCCC TTTNCCAGGC GACCATTTTT TGNGAAAGTT 360
NGAGGTTTGT TTTNNTGGNT TTAAACTTTN TTN 393

SEQ ID NO:5598
SEQUENCE LENGTH:69
SEQUENCE TYPE:nucleic acid
TOPOLOGY:linear
CLONE:HUMGS06652
SEQUENCE DESCRIPTION:
GATCAGAAGG CTCTGGNCAA GAGAATGAAA ATNAGGATGA GGAATAATAA ACTCTTTTTG 60
GCAAGCAAA 69

SEQ ID NO:5599
SEQUENCE LENGTH:85
SEQUENCE TYPE:nucleic acid
TOPOLOGY:linear
CLONE:HUMGS06653
SEQUENCE DESCRIPTION:
GATCTCTTTG TCCCTGAGGT GTCAGCTGCC AACAAGAAGC ATTAGAACAA ACCATGCTGG 60
GTTAATAAAT TGCCTCATTC GTAAA 85

SEQ ID NO:5600
SEQUENCE LENGTH:149
SEQUENCE TYPE:nucleic acid
TOPOLOGY:linear
CLONE:HUMGS06654
SEQUENCE DESCRIPTION:
GATCTTGGAG TCGTGGAACT GCTGCCCCAC CGGGTCCTGC GCCGTCAGCA CAAGCCACGC 60
TTCACCACCA AGAGGCCCAA CACCTTCTTC TAGGTGCAGG GCCCTCGTCC GGGTGTGCCC 120
CAAATAAACT CAGGAACGCC CCGGTGAAA 149

SEQ ID NO:5601
SEQUENCE LENGTH:120
SEQUENCE TYPE:nucleic acid
TOPOLOGY:linear
CLONE:HUMGS06656
SEQUENCE DESCRIPTION:
GATCGATGTG CTCAGCCAAA TNTCCTGTTT GAAATATCAT GTTAAATTAG AATGAATTTA 60

TCTTTACCAA AAACCATGTT GCGTTCAAAG AGGTGAACAT TAAAATATAG AGACAGGAAA 120

SEQ ID NO:5602
SEQUENCE LENGTH:162
SEQUENCE TYPE:nucleic acid
TOPOLOGY:linear
CLONE:HUMGS06659
SEQUENCE DESCRIPTION:
GATCAAAACC AAAAAAAATG NACCAAAGGC TTGGGTGGTG AGGGTGCTTA TCCTTTCTGT 60
TATTTTGTAG ATGAAAAAAC TTTCTGGGGA CCTCTTGAAT TACATGCTGT AACATATGAA 120
GTGATGTGGT TTCTATTAAA AAAATAACAC ATCCATCGGA AA 162

SEQ ID NO:5603
SEQUENCE LENGTH:56
SEQUENCE TYPE:nucleic acid
TOPOLOGY:linear
CLONE:HUMGS06660
SEQUENCE DESCRIPTION:
GATCCTGTTC TGTAATAAAC ATATTTTGCC TTGAGTAAAT TTGTTGTAAG CTTAAA 56

SEQ ID NO:5604
SEQUENCE LENGTH:90
SEQUENCE TYPE:nucleic acid
TOPOLOGY:linear
CLONE:HUMGS06661
SEQUENCE DESCRIPTION:
GATCCTTCTT GTATCTTGAA GTTTTGTACT TGGGATTTCT GGACTGATAA ATGAATCATC 60
ACATTCTTCT GGTAAATATT TTCTTGGAAA 90

SEQ ID NO:5605
SEQUENCE LENGTH:219
SEQUENCE TYPE:nucleic acid
TOPOLOGY:linear
CLONE:HUMGS06662
SEQUENCE DESCRIPTION:
GATCTGAAGA AGATGATGAA GTTGTGGCAA TNATTAAGGA ATTGTTAGAT ACTAGAATAC 60
GGCCAACTGT GCAGGAAGAT GGAGGGGATG TAATCTACAA AGGCTTTGAA GATGGCATTT 120
TACAGCTGNA ACTCCAGGGT TCTTGTACCA GCTGCCCTAG TTCANTCATT ACTCTGAAAA 180
ATGGGAATNC NGNGCATGCT GCAGTNTTAT ATNCCGNNN 219

SEQ ID NO:5606
SEQUENCE LENGTH:289
SEQUENCE TYPE:nucleic acid
TOPOLOGY:linear
CLONE:HUMGS06664

SEQUENCE DESCRIPTION:
GATCAACTGA ACCAGTATGC CAAAACCAGG CATCCAATTT GTAAACCAAT TATGATAAAG 60
GACAAAATAA GCTGTTTGCC ACCTCAAAAC TTTATGAACT TCACCACCAC TAGTGTCTGT 120
CCATGGAGTT AGAGGGGACA TCACTTAGAA GTTCTTATAG AAAGGACACA AGTTTGTTTC 180
CTGGCTTTAC CTTGGGAAAA TGCTAGCAAC ATTATAGAAA TTTTGCCTTG TTGCCTTATC 240
TTCTTCCAAA TGTACTGTTA AATAAAAATA AAGGGTTACC CCATGCAAA 289

SEQ ID NO:5607
SEQUENCE LENGTH:216
SEQUENCE TYPE:nucleic acid
TOPOLOGY:linear
CLONE:HUMGS06666
SEQUENCE DESCRIPTION:
GATCTGGAGC ATTTGAANCA AGTTTATAGA AGANCATGCC ACAAAACTAA GCAGGACCAA 60
GGAAGAGCTT TNAAGGCCTG AGGTCTGCGG AAGGTGGGAG GAGGCAGACG CCCTGCNTGG 120
CCCATGGTCG GGGCGTCCAC GGCGAGGCCG GCAACAAACG GCAGTATCTN GNATTCCTTT 180
TTTNTTTTTT NAAATTTTTA ATNCTTTGGT NTTTCN 216

SEQ ID NO:5608
SEQUENCE LENGTH:350
SEQUENCE TYPE:nucleic acid
TOPOLOGY:linear
CLONE:HUMGS06667
SEQUENCE DESCRIPTION:
GATCAGAATT ATAACCATGG AGAATTTTTT CTTCTGAGCA TTTTAATATA CTTGAAAACA 60
ACATTGACTT GAAAAATTTC AGAACATTTT TCAGTACCTA GTTTTATTAA ATATTACACT 120
TGAGAGACAC TTTTTAAAAA TGTGTTAATG TCAATATGAT GAGATTTTAG CCTTTCTCCA 180
GAACTAAGGC ATTAAAGAAA ATAGCAAATA TTAAAAAATA AAACTGTTAC TTTTTTCCTT 240
CTTTCTTTTC ACCTTTAGGT TAATATCCAG TATTATGTGT TATCCCNTTG GATAAGTATG 300
CTTTATTTTA CCTCNGTTAA AAATTAAAAT AAATGATTTC TATTCATAAA 350

SEQ ID NO:5609
SEQUENCE LENGTH:105
SEQUENCE TYPE:nucleic acid
TOPOLOGY:linear
CLONE:HUMGS06668
SEQUENCE DESCRIPTION:
GATCCTNAAG AGAGCTGTCC CAGCACTCTG GCCACCAGGA GGGCCANATT CCCCANAAAC 60
TACCTNTTGC CCAAAGAACA TNCTCAGTAT TTGGGGCATT TCCTN 105

SEQ ID NO:5610
SEQUENCE LENGTH:265
SEQUENCE TYPE:nucleic acid
TOPOLOGY:linear
CLONE:HUMGS06669

SEQUENCE DESCRIPTION:
GATCCCAGTG TCTGAGTGAA CTAACAGTCC TGCTTTCAGC CACCATTTGT ACAAGACACC 60
CAGCACTGAA AGTCCCGCTG CCAGGAGCAA GGGATTCTTT GGAAGCACCC GCCCTTTGTG 120
CCTTGTTGGG GGAAACCGGT GACGCAGTAG TGAGTGTGGA TACACCAGAG TTTGCATTGG 180
AAGGAATGAG TGTCACGTGG GGAGGGAAGG GGCCAGTGGA CCTTTTGTAA GCTTTCCACT 240
CAATAAAATG AACCTGTATG GCAAA 265

SEQ ID NO:5611
SEQUENCE LENGTH:138
SEQUENCE TYPE:nucleic acid
TOPOLOGY:linear
CLONE:HUMGS06670
SEQUENCE DESCRIPTION:
GATCCATGTT GAACAATACA TGTAGGTTCT TTTTCCACGC AATGTAAGAN CATGATATAC 60
TGTACGTTGG AAAGCATTTA CCTTATTTAT ATACCTGAAT GTTCCTACTA CACAAATAAA 120
CATATATTAA ATTCTAAA 138

SEQ ID NO:5612
SEQUENCE LENGTH:160
SEQUENCE TYPE:nucleic acid
TOPOLOGY:linear
CLONE:HUMGS06672
SEQUENCE DESCRIPTION:
GATCTGTCAT GCCCCACCGC CACCCCTACT CCCTTCAACC CTCCCTCTTT CTGCCCATTT 60
CCTCCCACCC CCTCACTCCC ATTTCCTAGC AAAATCAGAA GATTGTGAAG AGGCCGGCTT 120
CAACAAAATN GGATAAANAN ATAATTTTTT AAAACTTAAA 160

SEQ ID NO:5613
SEQUENCE LENGTH:217
SEQUENCE TYPE:nucleic acid
TOPOLOGY:linear
CLONE:HUMGS06673
SEQUENCE DESCRIPTION:
GATCCTATGT NCTGCTTGCA CAGAGGCCTT TGGATTTTAA CCCAGGAGNT TCGNGAGNTG 60
AGAGCTGCCC TGTCTTTNAT TGAGCCTTAG CATTTCCCTC TCAGGCTTCC TCTCAAGCTC 120
AAGGAACTCA CCCCCAGATA AGAAGCTGGT GCCTCTTGTC TCTCTNATTT CAGAAACGGA 180
CTTTCTNATC ATGCTTTCCT ATGGTGGGTA TGNGNNN 217

SEQ ID NO:5614
SEQUENCE LENGTH:109
SEQUENCE TYPE:nucleic acid
TOPOLOGY:linear
CLONE:HUMGS06674
SEQUENCE DESCRIPTION:
GATCCCAGAA TTCAACCTGT ATTTATAGAT GGATAATGTA NTTAGCTAAT TTTTGGTTTA 60

AATGANCTTN TTGGGTTAGC TTNCGTAAAT GNTATAATTT TTACTGTTN 109

SEQ ID NO:5615
SEQUENCE LENGTH:267
SEQUENCE TYPE:nucleic acid
TOPOLOGY:linear
CLONE:HUMGS06675
SEQUENCE DESCRIPTION:
GATCCCCAGC TGGACTCCCC ACCCCCTGGC TTCCCCACCT CTCCAGGNGT CAAAGGTGAA 60
CCGAGTCCAG TATTAGCTGA ATGTCATTTC GTACACCACA GCTCAGTCAG CCCATGGGCT 120
TCGTGAACTT TGCTCCAACA CACTGATGTG TGGGCTTCCC ACAGCAGGAC TGACTTCCCC 180
CTTTCTGTTG TTTGCACATN CCCCTCTTAC TGTACTGTCA ATAGATATTT TTGAGATTTA 240
TTTTTAAATA AATATTCAAC TTGCAAA 267

SEQ ID NO:5616
SEQUENCE LENGTH:61
SEQUENCE TYPE:nucleic acid
TOPOLOGY:linear
CLONE:HUMGS06676
SEQUENCE DESCRIPTION:
GATCAATAAT ATATAAAATN TGAGTGTGGG TTTGTATCTA ATAAAGTATG CCAACACCAA 60
A 61

SEQ ID NO:5617
SEQUENCE LENGTH:330
SEQUENCE TYPE:nucleic acid
TOPOLOGY:linear
CLONE:HUMGS06677
SEQUENCE DESCRIPTION:
GATCTTGAGG CCCAACATGC TTGACTCTGC CCAAAAGTCT CCTTCCTCAG CAGGATGCAG 60
CCTATTACAC TCAGCACAGN ATCTGGCACT CTTAGGTTCC CGGTCCATTT CAAACCTCCT 120
GCAACCAACA AAAGGATAGT TCCTTAATAA TTTGTCAAGC CTACCTAGGG CACTGCTAGT 180
AGTCTTACCA TTATTCACAA ATAGGGCTGA AACATCTAAA ATTACCCCAC TTAGTATTTC 240
ATATCCCTTC AACGTTCTAT CCTAAAGCTA ATGTTTCTTA GTGCTAATTA NAATCGTCAG 300
TCTATTGACA NTNTGACAAA GGGTAAGAAA 330

SEQ ID NO:5618
SEQUENCE LENGTH:84
SEQUENCE TYPE:nucleic acid
TOPOLOGY:linear
CLONE:HUMGS06678
SEQUENCE DESCRIPTION:
GATCCCAGGN TTCTGCGGAC CGACGATACG TTTAAATGTT GTTCTAGTAA ATATTCTTGA 60
ATGTATTAAA ATGGCTGAAA CAAA 84

SEQ ID NO:5619
SEQUENCE LENGTH:244
SEQUENCE TYPE:nucleic acid
TOPOLOGY:linear
CLONE:HUMGS06679
SEQUENCE DESCRIPTION:
GATCGTGTTC TNTAGATGAA ACCATCAAGA ACCCCCGCTC GACTGTGGAT GCTCCCACAG 60
CAGCAGGCCG GGGCCGTGGT CGTGGCCGCC CCCACTGAGA GGCACCCCAC CCATCACATG 120
GCTGGCTGGC TGCTGGGTGC ACTTACCCTC CTTGGCTTGG TTACTTCATT TTACAAGGAA 180
GGGGTAGTAA TTGGCCCACT CTNTTCTTAC TGGAGGCTAT TTAAATAAAA TGTAAGACTT 240
CAAA 244

SEQ ID NO:5620
SEQUENCE LENGTH:102
SEQUENCE TYPE:nucleic acid
TOPOLOGY:linear
CLONE:HUMGS06680
SEQUENCE DESCRIPTION:
GATCTTCATT AGTCAAGNTT TAGTTATTTN GTTGGCTTTT CCATGTAGAT TTCCAAGTGA 60
CATTAACATA CTTTNATTAA AGAAAATTTG GAGACTGTTN NN 102

SEQ ID NO:5621
SEQUENCE LENGTH:71
SEQUENCE TYPE:nucleic acid
TOPOLOGY:linear
CLONE:HUMGS06681
SEQUENCE DESCRIPTION:
GATCGGGCCC CATGTCTGTG CTGTCTAGTT TGTGTTCAAA ATGTCAGAAT AAACACAGAA 60
TAAATGTTAA A 71

SEQ ID NO:5622
SEQUENCE LENGTH:269
SEQUENCE TYPE:nucleic acid
TOPOLOGY:linear
CLONE:HUMGS06682
SEQUENCE DESCRIPTION:
GATCTTTTAA AAAAGTTAAA GGACATCCTA GAGCCTTAAT AGTTAAGAAG NGTTAAATTA 60
TCAAGCCTAT TTGTGCATTT CCTTTTNTTG AAAAAGGTAA GTTGCTGATT AAGTCTAATT 120
GGAATTGATA ATTCCATAGT CTTAGATTAN NNTGAGGATA TTTCCTCCTA GATTTCCTCA 180
TGTNATGCCA TGCATTTATA TATCTNACCA TTAATTTCAC ACTAAGGATG CTTCACCATA 240
TAATAANNGG TGCANGNTGG AAGCAANNN 269

SEQ ID NO:5623
SEQUENCE LENGTH:375
SEQUENCE TYPE:nucleic acid

TOPOLOGY:linear
CLONE:HUMGS06683
SEQUENCE DESCRIPTION:
GATCTCCTTT TCCAGTGCTG CACACTCCTG GTTTGGAACT TTAATGGCGT TGCAACGGAA 60
TCCTATATNC AGTTTCCTGT AATTTAATTG AAGAAAAATG CATCCAAATN AAGACTTTAT 120
TATTAACNGA CCAGATNGCA TCAGAAATCA TGTGTCTGTT ATNATTATCA GAATATGTCT 180
TAACTTTTTN GGGCAAAGTT AACACTGAAA GTTCTAGCTT AAGTGTTGAN ACTTTTGTGG 240
GGNAAAAAAN TCACTTTTGA AACTCAGACT TCAGNGTATA CCCANTAATT TAANGTTCNT 300
GTGAAATGGT TTAAATGTGT NGGACTCGTA ATGACTGTTT TANTGGTTCC AGTTTCTNCA 360
GNGTGGGTAN TTGGN 375

SEQ ID NO:5624
SEQUENCE LENGTH:54
SEQUENCE TYPE:nucleic acid
TOPOLOGY:linear
CLONE:HUMGS06684
SEQUENCE DESCRIPTION:
GATCTGATAA CTTGAATTCA GAATATTAAG AAAATGAAGT AACTNATTTT CAAA 54

SEQ ID NO:5625
SEQUENCE LENGTH:182
SEQUENCE TYPE:nucleic acid
TOPOLOGY:linear
CLONE:HUMGS06685
SEQUENCE DESCRIPTION:
GATCTGTTTT TATAAAGTGC TCCATGATTC TCTGAAAACA GTATACTATG TATCTAATTT 60
TTGGATACAG GATGGACATA CGTACTCAAA CTGTGTTTTT CAATTTGTAC TGTATTTGTT 120
TCTCTCGACC TGACTTAGTG ATAGAGGTAT ATTAAAGTNT TCCACTAGCA TGGTAGATTA 180
AA 182

SEQ ID NO:5626
SEQUENCE LENGTH:367
SEQUENCE TYPE:nucleic acid
TOPOLOGY:linear
CLONE:HUMGS06686
SEQUENCE DESCRIPTION:
GATCCTATGG NTTTTCCCGG CTGGTTGCCA CTACTGTACA ACATTCAGTG CCCACATCCA 60
TCTNTGCCAT TAAGCTTTTT TGAGACATGA GAGATGCCTC TNCCCTGCTG TATGACATGC 120
ATTTGGGAAG TTGGAAAGAA ATGACAAANT CAGGGAGAAA ACATCCAAGC TTCTTACCTG 180
TAGATAGANT CAGCCCTCAN TTGGTGCTTA TNACCAGTTA TTCANGAACA ATAACANCAA 240
CAAANTTAGT AGACATCCAA GAGGCACATA TTAGGGCCAA AGATAGCATC ANCTGTNTTT 300
GAGGGANCTG TAGTTTTGCG CATTTTNATG NCATTTTTAT AAAGGTACTG TNATTNTTTC 360
ATTGANN 367

SEQ ID NO:5627

SEQUENCE LENGTH:336
SEQUENCE TYPE:nucleic acid
TOPOLOGY:linear
CLONE:HUMGS06687
SEQUENCE DESCRIPTION:
GATCTAGTTT GACTTTCTAG TTAGTGGTGT TTTGAAGAGG GTATTTTATT GTTTTTTAAA 60
AAAAGGTTCT TAAACATTAT TTGAAATAGT TAATATAAAT NCATAATTNC NTTTCCCCTG 120
TTTATTGTAA TGTATTCTAA ATTAATGCAG AACCATATGG AAAATTTCAT TAAAATCTAT 180
CCCCAAATGT NCTTTCTGTA TCCTNCCTTC TACCTATNAT CCTGATTTTC AAAAATGCAG 240
TTAATGTACC ATTTNANNTG CTTGATGAAG GGAGCTCTAT TGCCNTTANC AGNAATTGTN 300
GCTAAGGTAA TTCCNAATAG AAAGCTGCTT ATTNNN 336

SEQ ID NO:5628
SEQUENCE LENGTH:364
SEQUENCE TYPE:nucleic acid
TOPOLOGY:linear
CLONE:HUMGS06688
SEQUENCE DESCRIPTION:
GATCTTCTTG ACCTTCTCCA TCATCGCCAT CCTTTCCCAA AGGCCAAGGA AGTGACCTGC 60
CTGCTGGCAT TGGCCAGACC ACAGCAGCAC CTCCTCCATG CAGGCCTTAA CTTTCCCATG 120
GTCAATGCAG TTTTTATCAG CCTTGCTTTG GATAGGACCT CCAAGGACTA AGGCCTCCAG 180
CCCCATGTNT GACCCTTGTN ATCTCTCTGC CCCACATAAT TCTGTTACTT TGCTATGTGC 240
TCCTAATGTA TCTAGTGTGT CCTGTGACAA CACTCATCAN ACTTCAGTGN AAATCACTTG 300
GTTTTAATTG ACTGTNTTNT CCTATAGACT GTAAGGNTCC ATGAGGGCAG GGCACATGTT 360
GGTN 364

SEQ ID NO:5629
SEQUENCE LENGTH:347
SEQUENCE TYPE:nucleic acid
TOPOLOGY:linear
CLONE:HUMGS06689
SEQUENCE DESCRIPTION:
GATCCCGTGT GCCACCAGGA GGGAGTGGCA GCTATAGTCT GGCACCAAAG TCCAGGACAC 60
CCAGTGGGGT GGAGTCGGAG CCACTGGTCC TGCTGCTGGC TGCCTCTCTG CTCCACCTTG 120
TGACCCAGGG TGGGGACAGG GGCTGGCCCA GGGCTGCAAT GCAGCATGTT GCCCTGGCAC 180
CTGTGGCCAG TACTCGGGAC AGACTAAGGG CGCTTGTCCC ATCCTGGACT TTTCCTCTCA 240
TGTCTTTGCT GCAGAACTGA AGAGACTAGG CGCTGGGGCT CAGTTTCCCT CTTAAGCTAA 300
GACTGATGTC AGAGGCCCCA TGGCGAGGCC CCTTTGGGCC ACTTACN 347

SEQ ID NO:5630
SEQUENCE LENGTH:318
SEQUENCE TYPE:nucleic acid
TOPOLOGY:linear
CLONE:HUMGS06690
SEQUENCE DESCRIPTION:

GATCAAGGTG TGAATAACTG AAGAAAATAA CTTGCTGGCT ATATAGGAAA ATGCTGTGGA 60
AATGAACTGT GTATATACTT CTGGGAGGAA CAAATTTAAT CATTTCTCCT GTTAAGCACT 120
AATCAGTATA GTGCAACTCC TGGTTCTGTN CTGTATTTNA TATGCAACAT ATATGCTTTA 180
ATATTTNNNT GTTTGTNCAT TAATATTTNC AATTTGTTTA ACCACTTTGC TGCTAAGATT 240
TTNCCGTCCC ATTCCCATTT NTCCCTTTAC AATNNTAAAC ANGTTTCTTC ATTAAANNCT 300
ATGGTGATGA AAAANAAN 318

SEQ ID NO:5631
SEQUENCE LENGTH:185
SEQUENCE TYPE:nucleic acid
TOPOLOGY:linear
CLONE:HUMGS06691
SEQUENCE DESCRIPTION:
GATCTGTGGG TGTTTTTAAA AAAACTCAAC CTATCTGGTG TTTTATTTTA ATGGATAAAA 60
ATGTAATTTT CCTAAGGTAG CAACTTATTT CCAAATTAAT ATAGATGANA AATAGATNCC 120
ANTTNGACTA AATTGAAAGC TTTTNNTCCT ATATTTNCAT AGCCTTTGAG AATATTGNTN 180
AGTGN 185

SEQ ID NO:5632
SEQUENCE LENGTH:130
SEQUENCE TYPE:nucleic acid
TOPOLOGY:linear
CLONE:HUMGS06692
SEQUENCE DESCRIPTION:
GATCAAATAA ACTCANTTTT TTATGGTTAC TGTAAAAAGA CTGTNTAAGN CAGCTCAGCA 60
CCATGCTTCT NGTAAAAGCA GCTTCANTTA TCCACTGGGG TTATCTTTTG ACAACTTGCC 120
ATTATCTGNN 130

SEQ ID NO:5633
SEQUENCE LENGTH:123
SEQUENCE TYPE:nucleic acid
TOPOLOGY:linear
CLONE:HUMGS06693
SEQUENCE DESCRIPTION:
GATCTTAACA AGCTTAAAAA AGAATTTTAT GACCAGAATC CAACAAGAGC TCTATTTTGG 60
AATTGTGCCC AAGTTGGTGA TGTTTACTCT AAAATTAATA ATAAAACTAC TTGTAAGCAC 120
AAA 123

SEQ ID NO:5634
SEQUENCE LENGTH:235
SEQUENCE TYPE:nucleic acid
TOPOLOGY:linear
CLONE:HUMGS06694
SEQUENCE DESCRIPTION:
GATCTGAACT GCCTCCTCCT TTCTCTGGCC TCTNCCCCCT TCCCTCTTCT CTTCAGCTAG 60

GCTAGCTGGT TTGGAGTAGA ATGGCAACTA ATTCTAATTT TAATTTATTA AATATTTGGG 120
GTTTTGGTTT TAAAGCCAGA ATTACGGCTA GCACCTAGCA TTTCAGCAGA GGGACCATTT 180
TAGACCAAAA TGTACTGTTA ATGGGTTTTT TTTTAAAATT AAAAGATTAA ATAAA 235

SEQ ID NO:5635
SEQUENCE LENGTH:250
SEQUENCE TYPE:nucleic acid
TOPOLOGY:linear
CLONE:HUMGS06696
SEQUENCE DESCRIPTION:
GATCACGCTA CTGCACTCCA GTCTGGGCAA CAGAGCAAAA TCCTATCTCA AAAAATATAT 60
ATATATTATC CAGCCCCAAA TGTTTATGGG GTTGTTATTG ATGAACCCTG CATTAAACAA 120
TCCCACAGTC GGTGCTGCGG TGAGCCGTGA TGGGTACCAC TNCATTCCAG CCTGGGTGAC 180
AGANTGAGAC CCTGTCTNNA AAAAAAANGT NNAANTTGTT TTCCCNATAT TTTGGNNGGA 240
TTNGTGGGGN 250

SEQ ID NO:5636
SEQUENCE LENGTH:131
SEQUENCE TYPE:nucleic acid
TOPOLOGY:linear
CLONE:HUMGS06697
SEQUENCE DESCRIPTION:
GATCTTGTGT ATTTTTGTCC ATATCCAATG TTATATGAAC TAATTGTATT GTTTTATACT 60
GTGACCACAA ATATTATGCA ATGCACCATT TGTTTTTTAT TTCATTAAAG GAAGTTTAAT 120
TTAAATTGAA A 131

SEQ ID NO:5637
SEQUENCE LENGTH:58
SEQUENCE TYPE:nucleic acid
TOPOLOGY:linear
CLONE:HUMGS06698
SEQUENCE DESCRIPTION:
GATCTTAATG GGCAGGGTTA AGAAAGTTAT TTAAAATAAA GTTACCTATT CTACTAAA 58

SEQ ID NO:5638
SEQUENCE LENGTH:250
SEQUENCE TYPE:nucleic acid
TOPOLOGY:linear
CLONE:HUMGS06699
SEQUENCE DESCRIPTION:
GATCAGAGTG ATGTGTTATG CAGAGAATTA AAGGGTGATG TGATAAAGTG ATTGGGTCGT 60
GCTTTATGTT GGCAAAAAAA ATCTGGAGTA TAAGAAGTTG CTGACTTGGG TGATTTGGAA 120
GAGATTGGGA GACTGGGGGG ATTCAGANCT GAAGGANCAG GCTATAGGCT GTAAGGCAAA 180
ANTATACTTG CNATGTTCAA ATTACTNAGA GGNTTNTACC NNCNTGCTTT TCTTTAAAAN 240
ATAAACNTTN 250

SEQ ID NO:5639
SEQUENCE LENGTH:354
SEQUENCE TYPE:nucleic acid
TOPOLOGY:linear
CLONE:HUMGS06700
SEQUENCE DESCRIPTION:
GATCTTGTTA TTGATTAACC ATTTTCTTAT GCCTTGCTAT TGACATATTC ATGCTCTTTC 60
TACGTCTAGT GGCTGAAAAT NTTTGCATTT GTTCATTTGA CTAATGGTGT GATTTTNGTT 120
TCTATATTAT NAGACCTGTA ATGTTTTAAA ATGTATTTNA TTAAATTTGG ACTGGATGTA 180
TGTCTCTAGC AATACGAGGT ACTTTCTAAA CTATTAAGGG AGGGGTTGTA TCCTCATGTT 240
GAGATAAGAT GATGGTCGTT TAANTTTNGC AATTTTTTTT GGCCTGCAGG GATATTTTGT 300
GTTTATGTGT CCAAAANNGG ANTAANTTGG CATTCTTGTG CCAAANGTTG TTNN 354

SEQ ID NO:5640
SEQUENCE LENGTH:342
SEQUENCE TYPE:nucleic acid
TOPOLOGY:linear
CLONE:HUMGS06701
SEQUENCE DESCRIPTION:
GATCTGCATT CTGTAAAAGA GGTACTTTCC CATGATGTAG GCATGAAGTG GTGCCAGTAA 60
GCGTAGAGCG GAAATNTTGA CTTTAGTTAA CATTGGGTNT AGCATTTCCA GTGCAGCATT 120
ATCAGTGGGC CTTTAAAAAT ACTTCGTAAG TACATTAGCT TTCACTTTGT NGTNAAATTA 180
TAGCAGACTC ATTATAGAGA ACAAGTTTGC CTTGATTTNN TTTAAAATGA CTTCTGCTAA 240
GCACCCAGAA GATAAANTTG ACATATTTTT NTAATNTAAG CATACTTTTT TNGTACATNG 300
TGTNCATTCT TGANTAANAT GNGGTTCTGT GTTGGCTTGT NN 342

SEQ ID NO:5641
SEQUENCE LENGTH:272
SEQUENCE TYPE:nucleic acid
TOPOLOGY:linear
CLONE:HUMGS06702
SEQUENCE DESCRIPTION:
GATCCAGTAT AATTNCAGAT TCTNGTGTCC CATCGAAGTT TCTAGCAAGA GGACTGTTGT 60
CACATTTCCT TCTTGGACTC ATTACTGGTC ACTGAAAACC AGAAGAAGTT GGTGAACCAT 120
CTAAAGAAGA GAAGGCTGTG GNCAAGTATC TNCGATTCAA CTNTCCAACA AAGTCCACCA 180
ATATGATGGG GTCACCGGNT TGATTATTTT TTTGCTTCAA AAGCAGTGGN CTGTNTTTNG 240
GGATTCAAAG NGGGNAAAGT CCANGTANTG GN 272

SEQ ID NO:5642
SEQUENCE LENGTH:264
SEQUENCE TYPE:nucleic acid
TOPOLOGY:linear
CLONE:HUMGS06703
SEQUENCE DESCRIPTION:

GATCGCAATG AATTTNTAAA ATTACTATTT AANATATTGT TGAGATTCCA TTACCTCCAA 60
TCAGTCTTGG GACATTAGAA CAGTGGTATA ATTAAGACTG TATGTTTAAA TTGAGAAATT 120
TTGCACAACT TTAACAAGTA GCAGANCTAA TTGTNCACTG ATGGCTTTAA AGATGAAATA 180
NTTCTGTGAA TNCATGANAC TGTGAGGGNC TCTNCGTGGT TGTCTAGGTT TATTTATATG 240
CATNCTCATT AATNAAAGTN NTTN 264

SEQ ID NO:5643
SEQUENCE LENGTH:247
SEQUENCE TYPE:nucleic acid
TOPOLOGY:linear
CLONE:HUMGS06704
SEQUENCE DESCRIPTION:
GATCTTTTTT ATTTTTATAC ACATGACAAG ATTTTACATC AAGAATAGTC AGTTAAATAG 60
TACAAATTTA CATTCATGAG GAATGTTAAA AAAAATTCAA CTAAAAAACC CACTTTTTCC 120
TGTGACCCAT AATCCCACAT TTTACAGTGC AGGGGAGAAG GGGATTAGGG GGCATNCAAA 180
ACAAGTCTCT CCCAAAAAGA AAAATGCAGG CATGCAGGGN CGGCGTAATC ANTNCCCNGN 240
NNNNNNN 247

SEQ ID NO:5644
SEQUENCE LENGTH:318
SEQUENCE TYPE:nucleic acid
TOPOLOGY:linear
CLONE:HUMGS06705
SEQUENCE DESCRIPTION:
GATCCATACA GGTTGTGTGT CATCTGTCCT CTTGAGGTTC AGGAGATTCT AAATTGAATA 60
TTCCACGGTT TGGGACAGCA TNCGGAAGTT TTCCCTATNA CTTTATATTT TGTATTATGT 120
CAAATGTTAT GGCAGGGCCC AAATAGCATA GCCACAAGTT TGGTTTATGT GGGCATAAAA 180
TTCTAACCAA ACCCCAGACA TAGGGAGTCA TTTGGAGAAA GCCTGTATGT GGTGTTTTAA 240
CCTAATAAAG TTGATGAGAG AGAAGGGGAG AGGAAGCGAA CATAAAGCGG GTCAAGTGTA 300
GTGCATCTTT TGTATTGN 318

SEQ ID NO:5645
SEQUENCE LENGTH:197
SEQUENCE TYPE:nucleic acid
TOPOLOGY:linear
CLONE:HUMGS06706
SEQUENCE DESCRIPTION:
GATCTTTTGA CATACTCACT TTGAGTGGCA TATGCCCAGG AAAATATTTA AAAGAAAGAA 60
AAGCTATTTT TACAAAGTTT TCTAGCAGTT CCACTCAGAT AACTTTAAGG GGGAAAAAAG 120
CCCAACGNTT GGNNATGGTT AAGTAAATTT NGGTGTATTG CTAGTGCTAT CNCNGANTGT 180
GTATGATGGC CNTNCAN 197

SEQ ID NO:5646
SEQUENCE LENGTH:210
SEQUENCE TYPE:nucleic acid

TOPOLOGY:linear
CLONE:HUMGS06707
SEQUENCE DESCRIPTION:
GATCCCCCGA ATAACTTTTT TCAATCAAGT TGAAAAACCA AACAGCTATG TAAAGTCCCC 60
AGGAGAGCTC TTTTTCCCTT ATNTNCTCAT TTNTTTTGTT ATAAACTGTT TAAATAGTTC 120
AAATGGTTGT NCATGCCTAG GGGATGAAAC CTATGTAATA GAAATGTACT GTTTTATGAA 180
TAAGGAGTAA AATTNTTTTA AAAAATTAAA 210

SEQ ID NO:5647
SEQUENCE LENGTH:372
SEQUENCE TYPE:nucleic acid
TOPOLOGY:linear
CLONE:HUMGS06709
SEQUENCE DESCRIPTION:
GATCTGAAAC AGAAACCACC CTCCGGAACC GCCCAAGAAA GGNCTGCTCC ANANACTCTT 60
CAAGCGGCAG CATCAGAACA ATTCCAAGAG TTCGCCCAGC TCCAAGACCA GTTTTAACCA 120
CCACATAAAC TCAAACCATG TCAGCTCGAA CTCCACCGGA AGCAGCTAGT TTCGGCTCTG 180
GCCTCCAAGT CCACAGTGGA ACCAGCCCAG ACCCTTCTCC TTAGAAGTGG AAGTAGTGGA 240
GCCCCTGCTC TGGTGGGGCT GCCAGGGGAG ACCCCGGGAG CCGGGGAAGG AAGGCCGTCC 300
ATCCCGTCGA CGTAGAACCT CGNGGTTTTT TCAAAGNAAT TTCCACTNNG GTCTGTTTTT 360
NCGAGGGGGG NN 372

SEQ ID NO:5648
SEQUENCE LENGTH:228
SEQUENCE TYPE:nucleic acid
TOPOLOGY:linear
CLONE:HUMGS06710
SEQUENCE DESCRIPTION:
GATCCTTGGT ACCCCTAAAA AGATTGCCAA TTTTTTTCAT CTTTGCCATA TGGAGGACTG 60
TGACAGACTT TGGACAGTGG CCTCTTGAGT TCCTCTGCAG TTTTGACATT TAGGATTTTG 120
TGTCTTTTAA ACTGGAAAAT CTTCTAGCAT GTTGGGTTGT TACAGAGTAT ATTTTTGTCT 180
GCAGCTGTTT GTTGCCCCAT TCCTAAGAGG AGTTTATCCA TCCNGAAA 228

SEQ ID NO:5649
SEQUENCE LENGTH:193
SEQUENCE TYPE:nucleic acid
TOPOLOGY:linear
CLONE:HUMGS06711
SEQUENCE DESCRIPTION:
GATCAAAACA AGATTTTCCC AGTAAGAATA TACTGGGACA TAACAAATGA AAATATTTAT 60
ACCAAAATAC CAAAAGTNAT TCACCTCTTT AAAATATCCT ACTATAGTCT AATTAGTAGC 120
TTGTNCAAGA CCAAGTAACC ATTTTTNNCT GCNCTTTTGA NTGATGATTA GAAATTCAAA 180
CAANGTATTT NNN 193

SEQ ID NO:5650

SEQUENCE LENGTH:176
SEQUENCE TYPE:nucleic acid
TOPOLOGY:linear
CLONE:HUMGS06712
SEQUENCE DESCRIPTION:
GATCCAAGTG CGGAANTCAC AGAAGGAACG GATTACAATT CTCCTNCAGN TAGGGAACCC 60
CTAAGNCCAC AGAAGATAAG GAGGAAACAA TACAGAAACT AGAGACCCTG GAGAAGAAGG 120
AAGAAGAAGT AACTTCAGAG GAGGATGAGG AGAAAGAAGA AGAAGNAGAG ANGNNN 176

SEQ ID NO:5651
SEQUENCE LENGTH:273
SEQUENCE TYPE:nucleic acid
TOPOLOGY:linear
CLONE:HUMGS06713
SEQUENCE DESCRIPTION:
GATCAAGTTT TTAAAATTGT NCTNTTATTG TTTTAAATTA AGAACTTTTT GAACTAAAAC 60
CAGAAACTCT GCCATAGTTC ATTGATTTTA ACATGAAACA TTTATTTACA AGATGATATC 120
AAGATTACTT TTCACAAAAT AGGCACATTA TATCANCACT TTGCNCTGCT TTGTAAATTT 180
NCCATCCAGG ATTTTGGGGT GGTATTCATG TGAAATTAAA GCAGATTCTC TAGCAGTTTC 240
CTATAGCTAC AGTTTTNNNN NTNNCCCTGG NTN 273

SEQ ID NO:5652
SEQUENCE LENGTH:231
SEQUENCE TYPE:nucleic acid
TOPOLOGY:linear
CLONE:HUMGS06714
SEQUENCE DESCRIPTION:
GATCCTGTGT GGAGAGGGAA TGTGAAATTA ACATCATTTC TTTTTGGGAG AGACTTGTTT 60
TGGATGCCCC CTAATCCCCT TCTCCCCTGC ACTGTAAAAT GTGGGATTAT GGGTCACAGG 120
AAAAAGTGGG TTTTTAAGTT GAATTTTTTT TAACATTCCT CATGAATGTA AATTTGTNCT 180
ATTTAACTGA CTATNCTTGA TGTAAAANCT TGTCATGTGT ATAAAAATAA A 231

SEQ ID NO:5653
SEQUENCE LENGTH:228
SEQUENCE TYPE:nucleic acid
TOPOLOGY:linear
CLONE:HUMGS06715
SEQUENCE DESCRIPTION:
GATCAACATG GTGGAACAAA ATGATAAAGA NCAGAAAACA TTTCAATATA TTACTAATAA 60
CTTTTTCCAA TATAAATCCT AAAATTCCTA TAACATAGTA TTTTACAGTT TTATGAAGCT 120
TTCTATTGTG ACTTTTATGG AATTAAGAGA TGAAGAAGAT GAGATATTTT AGCATTTATA 180
TTTTTCAAAA TTATATGTAT ACTTAANANT AANGTANCTT TATGCAAA 228

SEQ ID NO:5654
SEQUENCE LENGTH:182

SEQUENCE TYPE:nucleic acid
TOPOLOGY:linear
CLONE:HUMGS06716
SEQUENCE DESCRIPTION:
GATCACAACT GTGAAAAAAA ACTGCACACG CAAAAGACTA CAGGGAAATA TGTAATTNAT 60
TATNTTGTTT TCTCTCAGTG ATAAGATGGG GTGGTTTCTT CTTTGTACTT TTGTATTAAT 120
TTCTTACGGT GTTTGTATTA CTTTTATAAT CAAGAACTTT GTTTTAAAAA ATAAATCAGA 180
AA 182

SEQ ID NO:5655
SEQUENCE LENGTH:93
SEQUENCE TYPE:nucleic acid
TOPOLOGY:linear
CLONE:HUMGS06717
SEQUENCE DESCRIPTION:
GATCGTAGCT AGTTTGTATT GTCTTGTCAA TTGTACAGAC TTTTTAAAAA AAACAACCAC 60
CAGTGAAATG TGTGTGTATA CAATAAACTG AAA 93

SEQ ID NO:5656
SEQUENCE LENGTH:331
SEQUENCE TYPE:nucleic acid
TOPOLOGY:linear
CLONE:HUMGS06718
SEQUENCE DESCRIPTION:
GATCAACATT TATGACCTAA GTCAGGTAAT ATACCTGGTT TACTTCTTTA GCATTTTNAT 60
GCAGACAGTC TGTTATGCAC TGTGGTTTCA GATGTGCAAT AATTTGTACA ATGGTTTATT 120
CCCAAGTATG CCTTAAGCAG AACAAATGTG TTTTNCTATA TAGTTCCTTG CCTTAATAAA 180
TATGTAATAT AAATTTAAGC AAACGTCTAT TTTGTATATT TGTAAACTAC AAAGTAAAAT 240
GANCATTTTG TGGAGTTTGT ATTTNGCATA CTCAAGGTGA GANTTAAGTN TTANNTAAAC 300
CTATAATATT TNNNCTGANA CNCNNGNACC N 331

SEQ ID NO:5657
SEQUENCE LENGTH:250
SEQUENCE TYPE:nucleic acid
TOPOLOGY:linear
CLONE:HUMGS06719
SEQUENCE DESCRIPTION:
GATCAAGCCA AAAAATCCAC CAGGAANTCC TACTGACTCT CTTTNTAAAA TATAGTCATT 60
TAACNGTCAA AAGAGTCCCC AATAATGTCA GTNCTAAGAT TNCCCCTATT TTTTAAAAAA 120
ACAAAGTAAA TGAAGACTCA AAGAGCAAGT AACTAGTCTA AATTAGATAG TCACAGGTGG 180
TAAAGCCAGA AATCTGTCTG GNCAGTCTAA CTCTATACAN CCCAGGCTTT TAANTATTAG 240
AAAGCNNNNN 250

SEQ ID NO:5658
SEQUENCE LENGTH:409

SEQUENCE TYPE:nucleic acid
TOPOLOGY:linear
CLONE:HUMGS06720
SEQUENCE DESCRIPTION:
GATCTCAGTA TCATACTGAG ACACCTCCCC TGAGCCTTAC CTACTTAAAT TGGTAACCGT 60
CCTACCAAAA TTTGTCAATT TNAATCAAGT GAGGCAAGTT GCAAGGGAGC CAGAGATACG 120
TGAAAAAAGA AAAGCAGAAA TACTGATACT TTCTAAGAAA GAAGTTGTAA TAATTNCTTT 180
GGCACATTGA CTTACTGATA TCTTTTGAAA TGCATAGATG ACTCTTGTGA ACCAANGAGA 240
GCAGTNGTGG TTNTTCCCCG GCGCAATAAA AATGCCTTTG TGTATCACTT GCGGTTGCCA 300
GTCATTTCTG GTGCCAGTTG CTTTTNNTNN CCAGAGGTTT GGCCTTNATT CTTCCGAAAN 360
NAGGGTGATG ATTNTTNGAA GNTGGGGTTT TCCTNACCAN ATTATTNTN 409

SEQ ID NO:5659
SEQUENCE LENGTH:56
SEQUENCE TYPE:nucleic acid
TOPOLOGY:linear
CLONE:HUMGS06721
SEQUENCE DESCRIPTION:
GATCTGGAAA AGTCAAGAGA AGAGAGCAGA AAGCAGGCTC TTGCTGCTAA AAGAAA 56

SEQ ID NO:5660
SEQUENCE LENGTH:69
SEQUENCE TYPE:nucleic acid
TOPOLOGY:linear
CLONE:HUMGS06724
SEQUENCE DESCRIPTION:
GATCTAAAGA TGATGGAAAA CATCAGCTAC ATTAAATACT CATAAGCAAA ATAAAGGCTG 60
GCCTGTAAA 69

SEQ ID NO:5661
SEQUENCE LENGTH:208
SEQUENCE TYPE:nucleic acid
TOPOLOGY:linear
CLONE:HUMGS06725
SEQUENCE DESCRIPTION:
GATCCAAAGG GAAGGGACTA TGCAAGGGCA TTTATAAACG NTGGACAAGG GTTCATTATA 60
GAACACCANT GTNATGGACT ATCCCAAGCA TGTACCACTG TGTCTCATGC ATGGTAAAGG 120
TCCCATAAAT GTTATTGCTC TCTTGCAGTA TAGTAGTAAT AGTAGTGGTA GCTGNTTTAG 180
GAGTAAAAAC AGCNAAAAAN AGANNNNN 208

SEQ ID NO:5662
SEQUENCE LENGTH:326
SEQUENCE TYPE:nucleic acid
TOPOLOGY:linear
CLONE:HUMGS06726

SEQUENCE DESCRIPTION:
GATCTGAAAA AAAAGTTTCA CCGTTTGTTT TCTTACCTCA TTTTAAGAAG CATGTGAAAA 60
TGGGATACTA TAGACTACTG AGAATTTCAG AAATTGAGAA CAATTTCATA ATAAAACGGC 120
TATATTTGAA GAGAGANTAC ATTTTATATA ANCAGGAAAA TACATTTGAC ACTTTATGGA 180
ATTTNATGAG ACTTTTTGTG GGAACAGAAG GTCTTCAAAT TGTAAAATGT AAAGNTTGCN 240
CTNTTTNTTA NGTCTTTAAC AGGGGTGTNT TTCATTGTNT GTTTTGGGTA TGGCTTTGGN 300
ATAANTCATT NTATATNTNA NTTGAN 326

SEQ ID NO:5663
SEQUENCE LENGTH:227
SEQUENCE TYPE:nucleic acid
TOPOLOGY:linear
CLONE:HUMGS06727
SEQUENCE DESCRIPTION:
GATCTGCACA GTTTGGTTTT TGCACAAAAG TCATTTAAAA AAATCTGAGT AATTGTCAAA 60
TATTAAAAGA AAGNTATTCT TCCTGGTAAG GAATACAGTT TTNAGTCAAA GTGGCCATTA 120
CATCCNCTTT TNAATTTACA TAATACAGNT ACTTGTGAAA GTTGTTGTGG TGTTGTATGC 180
CAAGNNAATN CTTTTTTATT GGTGCCTATA TTGTCACANT TNTNTTN 227

SEQ ID NO:5664
SEQUENCE LENGTH:324
SEQUENCE TYPE:nucleic acid
TOPOLOGY:linear
CLONE:HUMGS06728
SEQUENCE DESCRIPTION:
GATCCACCCT CCTCGGCCTC CCAAAGTGCT GAGGATTACA GGCATGAGCC ACCACAGCTG 60
GCCCCCTTCT NTTTTATGNT TGGTTTTTNA GAAGGAATGA AGTNGGAACC AAATNAGGTA 120
ATTTTGGGTA ATCTGTCTCT AAAATNTTAG CTAAAAACAA AGCTCTATGT AAAGTAATAA 180
AGTATANTTN CCATATAAAT TTCAAAATTC AACTGGCTTT TATGCAAAGA AACAGGTTAG 240
GGACATCTAG GTTCCAATTC ATTCACATTC TTTGGTTCCN GGTAAAATCN GGNGGNNTAT 300
ANCAATTTCT ANTTGGGGTT TNCN 324

SEQ ID NO:5665
SEQUENCE LENGTH:149
SEQUENCE TYPE:nucleic acid
TOPOLOGY:linear
CLONE:HUMGS06730
SEQUENCE DESCRIPTION:
GATCCTTCCA CCTTGGCCTC CCAAAGCATT GGGATTACAG GCGAGAGCAC TTGGTTGGCT 60
AAAATATTTA TATTTACCTG NCATNATTTG TTGATTTGGG GGCTAGGGAG TTCTTAAAAC 120
AACAATAAAA AATCTTCTGT GGCTGCAAA 149

SEQ ID NO:5666
SEQUENCE LENGTH:236
SEQUENCE TYPE:nucleic acid

TOPOLOGY:linear
CLONE:HUMGS06731
SEQUENCE DESCRIPTION:
GATCACAGTC TTGAGGTCCT CTAGCAGGGG TGAGGGAGAG CAGCGACTTC AGCTGAGTCC 60
CTGCCAGTGG TTAAGCAAAC AATGGTTTCA AAATTCAAGG TCCCCAAATG GCAGCATTTT 120
ATGTTCTGAC CTGTTTGTGT TATATAGTGG TGTTTTNNNN CCNCTTTGGA NCTCTTGNGT 180
TGTTAATAAA ATGAAATGAT TACTTTTNAA TTAAANTGAA AAANGTAAAA AANAAN 236

SEQ ID NO:5667
SEQUENCE LENGTH:101
SEQUENCE TYPE:nucleic acid
TOPOLOGY:linear
CLONE:HUMGS06732
SEQUENCE DESCRIPTION:
GATCGCGCCA CTGCACTCCA ACCTGGGTGA CAGACTCTGT CTCCAGAACA AAACAAACAA 60
ACAAAAAGAT TTTATTAAAG ATATTTTGTT AACTCAGTAA A 101

SEQ ID NO:5668
SEQUENCE LENGTH:259
SEQUENCE TYPE:nucleic acid
TOPOLOGY:linear
CLONE:HUMGS06733
SEQUENCE DESCRIPTION:
GATCCATGGA CCATCTGGGT CATGGCGCCT GGTTTCAGAC AACCTGAATC AAATCTTAGG 60
GGTGGGGCTT TGGGATGTCA TTGTTCAATA GGCACCTCAG GAGATTCTGA GCACACCNNT 120
GTTTGAGAAC CACTAAAATN AGGAGTGGGA AAAAAAAAAT AGGTGTTTTG TTAATTTAGA 180
GCTGAGCTGN GANGNTAATA TATTTTNNTT GTCANTGCCA TTACCAGNTA TGCACTGNTT 240
CTTTTATACC TACANTTTN 259

SEQ ID NO:5669
SEQUENCE LENGTH:218
SEQUENCE TYPE:nucleic acid
TOPOLOGY:linear
CLONE:HUMGS06734
SEQUENCE DESCRIPTION:
GATCCTGAAG CTTACTATGC AGCCTACAAA CAGCCTTAGT AATTAAAACA TTTTATACCA 60
ATAAAATTTT CAAATATTGC TAACTAATGT AGCATTAACT AACGATTGGA AACTACATNN 120
ACAACTTCAA AGCTGTTTTA TACATAGAAA TCAATTACAG CTTTAATTGA AAACTATAAC 180
CATTTTGATA ATGCAACANT AAAGCATCTT CAGCCAAA 218

SEQ ID NO:5670
SEQUENCE LENGTH:222
SEQUENCE TYPE:nucleic acid
TOPOLOGY:linear
CLONE:HUMGS06735

SEQUENCE DESCRIPTION:
GATCAGATTG AGGTTATGCA TTTTTGGCAA GAAAACCACG GCAATGATAT GCCCGTCTCG 60
GTGCATTTNA TCAGGAGATA CTTGATGTCG ATGTGTCATA TTACTGGTGA TGTTAACCTT 120
GGTTAAGGTT AGTAGCTTTC AGGTTTCTCC ATTGTAAAGT TGCTATTACT TCTTAATATA 180
ATAGGCTAGG TTGTACTGCT ATAACAAATA TACCTTAGTA AA 222

SEQ ID NO:5671
SEQUENCE LENGTH:86
SEQUENCE TYPE:nucleic acid
TOPOLOGY:linear
CLONE:HUMGS06736
SEQUENCE DESCRIPTION:
GATCCTTGAA TGAAAAGGAA CCCTCACCAT GGGGCTTACT GTTATAATGT TTCTTCATTT 60
ACTGTTATTA AAAGATTTAT GAGAAA 86

SEQ ID NO:5672
SEQUENCE LENGTH:391
SEQUENCE TYPE:nucleic acid
TOPOLOGY:linear
CLONE:HUMGS06737
SEQUENCE DESCRIPTION:
GATCAGAAGC TGCCAGGAAC AAGGAGAAGA CGCTCCCCTT CCTCACCTTC CCACCCCCAC 60
ATGGCTTCTC ANTCGGGGAA AAGATACAAC TTAACTTTAA ACCAAGTNTT AAGTTTTGAT 120
TATNACATGG GATTAGATGC ACCAAAAACA ATTTCAAGTG ACTGTGGGGC ATTCTATTGC 180
CTGAGAATGT TCAAGAGTCC TGATATGACC TGTTGTTNTT ATCCTAAACA GTGAAAAATT 240
CATCCACCAA ATATATAGGA ATAGAGGGTG ACAGNAAGAT GCTTTCTGAA AGGTATCCNA 300
TATTTGGGGG TNTTGGGTTA CATATNNCTN ATGTNGGNTT TTAANCTATA GCTCTTCANT 360
TANNNTTACT CNATCCATTN CTTGAAAGGA N 391

SEQ ID NO:5673
SEQUENCE LENGTH:210
SEQUENCE TYPE:nucleic acid
TOPOLOGY:linear
CLONE:HUMGS06739
SEQUENCE DESCRIPTION:
GATCCTNTGT ACATGAAATG AAAGGTGCAT CANCTTGGGG GCTGGGAAAC CTGCAGTATG 60
GNTTTACTNC GTCCCTATCA CTGGTGTGGC TGTGGGCAAA CCACTTATTG CCTGACCTAC 120
CTCACAGGGA TGTTGTGAGG GTTTGATGAG AGAATGAATG TTAATAGGAA TTGGAAAATT 180
CAAAGCATTA AACACATGTA AACAGGTAAA 210

SEQ ID NO:5674
SEQUENCE LENGTH:223
SEQUENCE TYPE:nucleic acid
TOPOLOGY:linear
CLONE:HUMGS06740

SEQUENCE DESCRIPTION:
GATCTCAAGA AAAAGCAGGA ATTAAATAAG ACAAGGNNCA GATTAANATA CCTTTCCCAG 60
CAGGGGAAAC AGCAAACTTA AAGGTTCTAA GGCACANGAG AGCATGGCAT ATATGACGAC 120
CCAAAAGAGT GGCCAGATAT CCAGACATCT AGAAGGTCTA GCTAAGTNCT TAGGAAGTAG 180
TATAANCAGA NAAGANAAAN TTGTGGCCTC TTTTTTNAAA ANN 223

SEQ ID NO:5675
SEQUENCE LENGTH:391
SEQUENCE TYPE:nucleic acid
TOPOLOGY:linear
CLONE:HUMGS06742
SEQUENCE DESCRIPTION:
GATCATGCAC TGTTGACCAC TGAGCAATCT GTGTNACACT AGAGTTCACA GGGCATTTTA 60
AGTGTAGACG TGAGTCCTTA AACATATTTN GNTTTCTCTC TCAGGTTTTA AATGTTTCAA 120
ATGTAATTNT TGCTCATCAG TGCAGTTATC AATGCAATTT NATATTCCTT GAGGGGAGAA 180
AGNGGGGTCT TATTGTACAT GTCCAAGGGG GGTGATAAGA GTATTANCTG TTTAATTTAA 240
TTGGANCAAN CCATTGTCTT AACGCAGCCA TGGTTTGANT TTGTCATCTT NGGGCTGACC 300
GGTGCATGTA AATACAGTAT GCNCTTTGGA TGTAATCCTT NGAAATGNCA GTGTTGNATG 360
GTAGGTTATC CTTNNGTNAA CCCTTNTCTC N 391

SEQ ID NO:5676
SEQUENCE LENGTH:271
SEQUENCE TYPE:nucleic acid
TOPOLOGY:linear
CLONE:HUMGS06743
SEQUENCE DESCRIPTION:
GATCCCTATA ACTCAAAATA ACTTGTTTGT AAAAGAAAAT TTGTTTACTT ACCCATTAGT 60
AAGTTCCTGC ATATTCATTA TAAGATGGCA AATCAAACTT TTCTAGGATG AAGACAGCTT 120
ATNTTNAAGT TGTATAGTCT TAGTTGGTTT AGGGTCTCAA TTTTAATTAA TAAAATNCTT 180
GGTTTTAATT TGCTTGTCCT TTTGAATTCC NGTTTTAATA ATTNTNNNAG NGNGCAAAGN 240
GATGTTGAAG TTCAGATTAA NCTCTNCTGA N 271

SEQ ID NO:5677
SEQUENCE LENGTH:166
SEQUENCE TYPE:nucleic acid
TOPOLOGY:linear
CLONE:HUMGS06744
SEQUENCE DESCRIPTION:
GATCCTGGAA GTAATCCGGG CACACTTACA TTTGTTGTCA ACAAGAACAA TCCTTTCATC 60
TTCTTGGGCT TTCACATGAA CAGCCTTAAT AAAAACCGCC AGGACTCCCC AGAAAAGCAA 120
ATGGTTCTTC ATCTTGACTT CACTTCTTCT GAAAAACTGG AGCAAA 166

SEQ ID NO:5678
SEQUENCE LENGTH:386
SEQUENCE TYPE:nucleic acid

TOPOLOGY:linear
CLONE:HUMGS06745
SEQUENCE DESCRIPTION:
GATCACAAAT TAATGTTGGG GAAAATAAAG ATTTAATATT TCTTTAAATA GAAAAAGAAT 60
TTGGTTTTCC TCGTTTAAGA GCAATGAGAA AATNATGGAA TGTTGACTGT NTTTGGCACA 120
CAGGACACGN ACCTTCATGG AAGTCCTTGC TCTGCGTGGC ATCTGTCAGC TTTNCACCTT 180
TCATTCTTAT TCTTCACTTT TGCTGCTGAG CCTAGCTGTA CAAACTTNGC ACTTTCATTT 240
GCTAATATAA ATTCAGTTTT ATTNTACCAT TTTAGAGACT ACTAATGGTT AANTGTAGAA 300
GGNGNGGGGT GNACATGTTT TTATGTNGNG TGTTTNAAAG GATATGTTTT NTNACCCCNT 360
GTNATGGTGG CAGCTTTNTN TTTTTN 386

SEQ ID NO:5679
SEQUENCE LENGTH:260
SEQUENCE TYPE:nucleic acid
TOPOLOGY:linear
CLONE:HUMGS06746
SEQUENCE DESCRIPTION:
GATCCAGGAA GCGGCTGGAG ACATTCCTGA GCCTCCTGGT CCAAAACCTG GCACCTGCAG 60
AGACGCATAC CTAGAGGAAC TCTAACCCCG GTGTACCTGT ATAAACTGAA CTGTGAAACT 120
GTTTCGGTTA TCTCTGTCTT TTGAGGATGG CTTTGTCCTG TTGCTGGTTA ACATTCACCT 180
TCCTCTTTTG AGGAGTATTT TTCCATTATG TATTCATAAT AATGTTAATT TCAATAAATG 240
ACATTCATGC AGCAATTAAA 260

SEQ ID NO:5680
SEQUENCE LENGTH:183
SEQUENCE TYPE:nucleic acid
TOPOLOGY:linear
CLONE:HUMGS06747
SEQUENCE DESCRIPTION:
GATCAACATT CCAGGGTTGG TAATGTTGGA TGATGAAACA TTCATTTTTA CCTTGTGGAT 60
GCTAGTGCTG TAGAGTTCAC TGTTGTACAC AGTCTGTTTT CTATTTGTTA AGAAAAGNTA 120
CAGCATCATT GCATAATTCT TGATGGTAAT AAATTTGAAT AATCAGATTT CTTACAAACC 180
AAA 183

SEQ ID NO:5681
SEQUENCE LENGTH:124
SEQUENCE TYPE:nucleic acid
TOPOLOGY:linear
CLONE:HUMGS06749
SEQUENCE DESCRIPTION:
GATCTCACTA TAGAGGCATC ACTAATTGGG GAAAGACAGA CTAGGTAATA GTCAGATGTN 60
ATATAGTATG TTTAANCTAT AGGATGGCAG GTNCATGANT NTTGGTTGTA TTATCCTTTA 120
TNTN 124

SEQ ID NO:5682

SEQUENCE LENGTH:268
SEQUENCE TYPE:nucleic acid
TOPOLOGY:linear
CLONE:HUMGS06750
SEQUENCE DESCRIPTION:
GATCTGAAGT TGCAGGTTAA GCTTGTNTGG TCAACATTCC AGTGTGGAAA AATAATTTAA 60
ACAATCTTAT TCTCTTAATT CTTTTGGCAA CAAAAACTAT TAGTAATAGC TATTTGGGAC 120
CAGACAAAAT CAGCTTTCAT CTATAATTCA TTGGGGATAA TGGGAGATTT AGATAATGTA 180
TCCNGATTTA AACCTACCAG TTTGTCCTAC CCCTTANGCG TTTAANATAA ANTATGCANC 240
AAATTTGGNG GNANGNNGTG TGNNGTTN 268

SEQ ID NO:5683
SEQUENCE LENGTH:127
SEQUENCE TYPE:nucleic acid
TOPOLOGY:linear
CLONE:HUMGS06751
SEQUENCE DESCRIPTION:
GATCAATGCA AAAATTGTTA TATATGAACA TATAACTGGA GTCGTCAAAA AGCAAATTAA 60
GAAAGATTTG GACGTTAGAA GTTGTCATTT AAAGCAGCCT TCTAATAAAG TTNTTTCAAA 120
GCTGAAA 127

SEQ ID NO:5684
SEQUENCE LENGTH:221
SEQUENCE TYPE:nucleic acid
TOPOLOGY:linear
CLONE:HUMGS06752
SEQUENCE DESCRIPTION:
GATCTTTACA ATTACAGTGT AGTAGACAAT TCGGAATTAA TTTGAAACAC ATCACAACAT 60
AACTTGAGAA AATTTCCTAN TGTATATGTA GTGGTAATAA ATGTTTAAAG GCTGCAAGGA 120
TTGCATTATA AANNCTGTTT GTNGGCTTTC TAAGTCTTTN ATNCCTGTAT TGGGANTAAN 180
CAGATTTNAG AAAATGGATA TAAGCTGANA TGTATATTTN N 221

SEQ ID NO:5685
SEQUENCE LENGTH:146
SEQUENCE TYPE:nucleic acid
TOPOLOGY:linear
CLONE:HUMGS06753
SEQUENCE DESCRIPTION:
GATCATAAAT TTTTCTCAGT GTCCTTTTTG TGTTGAACAC ATATATCTGA AACCTGTATT 60
TAACCAGATA TTTCTACTCA ACTGAATATA CAAGTGTAAA TTTGATTGGA AAATTGTATA 120
TTATAAATGA AAATACAATA ATCAAA 146

SEQ ID NO:5686
SEQUENCE LENGTH:344
SEQUENCE TYPE:nucleic acid

TOPOLOGY:linear
CLONE:HUMGS06754
SEQUENCE DESCRIPTION:
GATCCACCCA CCTCGACCTC CCAAAGTGCT GGGATTACAG ATGTAAGCCA CCGTGCCTGG 60
CCAAAGTTTT CTAATACCTG TTTATAATCC CCTTCTTTGT CCTCAGGTAA CACTATCTAC 120
TACGTGTCTC TNCAGATTAG CTTGCATTTC CTAGAATCTA ACTTATATAA TTAAAATAAT 180
ATAGTATATA CCCTTCTTTT GGCTTTCTTT ATTCAGCTAT GTTGTGAGAT TCATTCATGT 240
TGTTTAGAGT ATCAGTTACT AAGTAGTATT CCATTTTNGG CTATATCAAA NNTNGTTTGN 300
CCATTTACCT ATTAANGGGC ATTTNGCACT GGNTCCAGNN TGAN 344

SEQ ID NO:5687
SEQUENCE LENGTH:132
SEQUENCE TYPE:nucleic acid
TOPOLOGY:linear
CLONE:HUMGS06755
SEQUENCE DESCRIPTION:
GATCTCTGTT TTGACTAAAC TAGAGGAAAA ATNATTGGAT GTGTTTATTC TTTTCTAAGC 60
AGAATGGTTT AACTTTGTAC TCTTTGAAAA ATAATGCTGA TTTATAAATC TCTGCCTATA 120
NCAGAATGGA AA 132

SEQ ID NO:5688
SEQUENCE LENGTH:244
SEQUENCE TYPE:nucleic acid
TOPOLOGY:linear
CLONE:HUMGS06756
SEQUENCE DESCRIPTION:
GATCCTGCTG TGGCACGGGG CCTATGTGTC TCTGTCGCGT CTGCTGTGAA GACATGATGC 60
TCTATTTATT GTAGAGAGTG ACTTTATTTG CTTTCTAGAA TTGTTTATAA CAGATGGTAT 120
AAGAGAGGTA ATAAACAGAG AAAAATCTAT GCTTGTAAAG AATACAAAAG TTAATTTTAC 180
CTACTATAAT ATGGCTGGCT GAANCTTATT TCCTCTCTNN GNAATAAATG TTCTANTGGG 240
CAAA 244

SEQ ID NO:5689
SEQUENCE LENGTH:143
SEQUENCE TYPE:nucleic acid
TOPOLOGY:linear
CLONE:HUMGS06757
SEQUENCE DESCRIPTION:
GATCTTNTCT ATACCATTTA ATGTAGAAAA ACTAATTTTC TATAATTNTN TCTATTAAAG 60
GTGACATTAA TGCTGATAGA AAAATATTCC CACATATATC TNACTTGGAA AACTTATAAA 120
AATTGTGCTG TCTTTAAAAT AAA 143

SEQ ID NO:5690
SEQUENCE LENGTH:219
SEQUENCE TYPE:nucleic acid

TOPOLOGY:linear
CLONE:HUMGS06758
SEQUENCE DESCRIPTION:
GATCGCAAAG GGATTGTTCT TATAAAAGTG GCATAAATAA ATGCATCATT TTTAGGAGTA 60
TAGACAGATA TATCTTATTG TGGGGAGGGG AAAGAAATCC ATCTGCTCAT GAAGCACTTC 120
TGAAAATATA GGTGATTGCC TGAATGTCGA AGNTCTACTT TTGTCTATAA AACACTATAT 180
AAATGANTTT TAATAAATTT TTGCTTCAGC ACTTGGAAA 219

SEQ ID NO:5691
SEQUENCE LENGTH:292
SEQUENCE TYPE:nucleic acid
TOPOLOGY:linear
CLONE:HUMGS06759
SEQUENCE DESCRIPTION:
GATCTAGATG AGATTACCCA TAGCAGGGAA AATAATTCAT CACCTACCAA AGACTGTACG 60
GAAAGGTGTG TTATTTTCAA AATTGCATTT TAGTTTGCAC ACATGTAAAT NATTATCCTA 120
TGTTAATCTA ATTGACAATT CANTTAGCTC CGCATCCTAA GACTTAAAAC TAGCAATTCC 180
TATGCAAATC ATCTGCTTAA CTGANCTACA AACGTTTTTC ATTAATTCAT TCTTTGATGA 240
CATATTCCAT GTTATATANT ANTNCANAAT AAAGTTAATA TACTACCCCA AA 292

SEQ ID NO:5692
SEQUENCE LENGTH:330
SEQUENCE TYPE:nucleic acid
TOPOLOGY:linear
CLONE:HUMGS06760
SEQUENCE DESCRIPTION:
GATCCTCTCG CTTGTTCACT GGGGCATGTA CAGTGGGCAC GGGAAGCTGG AATTCGTATG 60
ACGGAGCTTA TCTGAACTAC ACTTACTGAA CAGCTTGAAG GCCTTTTGAG TTCTGCTGGA 120
CAGGAGCACT TTATCTGAAG ACAAACTCAT TTAATCATCT TTGAGAGACA AAATGACCTC 180
TGCAAACAGA ATCTTGGATA TTTCTTCTGA AGGATTATTT GCACAGACTT AAATACAGTT 240
AAATGTGTTA TTTGCTTTTA AAATTATAAA AAGCANAGAG AAGNCTTTGT ACACACTGTC 300
ACCAGGGTTA TTTGCATCCA AGGGAGCTTN 330

SEQ ID NO:5693
SEQUENCE LENGTH:143
SEQUENCE TYPE:nucleic acid
TOPOLOGY:linear
CLONE:HUMGS06762
SEQUENCE DESCRIPTION:
GATCANAGGT CTTGAGGTCT TTACATCTTG ACATATACAC TGAAAAAAAN GGGGGTTGTA 60
TGTATNAATG TGCTACCCAA ACCTNTGGCC GCCACTTTTA AATTCTCAGA TTGCCCTGAA 120
TTTTGNCACT TTNAAATAAT GNN 143

SEQ ID NO:5694
SEQUENCE LENGTH:273

SEQUENCE TYPE:nucleic acid
TOPOLOGY:linear
CLONE:HUMGS06763
SEQUENCE DESCRIPTION:
GATCTTAATG CTTCTCCTGC TCTGTAAGCC TCTNAAGAGC AATATCTAAT TAATTATTAC 60
TGTAATTTTT TAAAAGGCTT TAAAGTGCCT CAGGGGTCCC CTGAAACTAA TTTCCTATTT 120
CTGGGATTCC CTGGATTCAT TATATGNNAT GGTGACATGA TTAGAGGAAT TCNTTTTAAG 180
TATGAAANTT GTCCCTTTCC TNCTTCAGTA CTTGCCTCCT NGCTGGCATT GANTTAACAC 240
AGGGACAAAA TTTGGTTAAT TNNTNATTNC NAN 273

SEQ ID NO:5695
SEQUENCE LENGTH:337
SEQUENCE TYPE:nucleic acid
TOPOLOGY:linear
CLONE:HUMGS06764
SEQUENCE DESCRIPTION:
GATCTGTACT ACAGGAACCA AATGTCATGC GTCATACATG TGGGTATAAA GTACATAAAA 60
TATATCTAAC TATTCATAAT GTGGGGTGGG TAATACTGTC TGTGAAATAA TGTAAGANGC 120
TTTTCACTTA AAAAAAATGC ATTACTTTCA CTTANCACTA GACACCAGGT CGAAAATTTT 180
CAAGGTTATA GTACTTATTT CAGCANTTCT TAGAGATGCT AGCTAGTGTT GAAGCTAAAA 240
NTAGCTTTAT TTATGCTGGG NTGGTGATTT NTTTATGNCA AAATTTTTNT AGNGCGAANC 300
ATGTGGTGGN TAGCTTGGNN ATGAANGAAT TNTGNCN 337

SEQ ID NO:5696
SEQUENCE LENGTH:290
SEQUENCE TYPE:nucleic acid
TOPOLOGY:linear
CLONE:HUMGS06765
SEQUENCE DESCRIPTION:
GATCTAGAAG TTGCAACATT CGTTTGATAG GAATTCCAGA AAAGNAGAGT TATGAGAATA 60
GGGCAGAGGA CATAATTAAA GAAATAATTG ATGAAANCTT TGCAGAACTA AAGAAAGGTT 120
CAAGTCNCGA GATTGTCAGT GCTTGTCGAG TACCTAGTAA AATNGNATGA AAAGAGGCTG 180
GCTCCTAGGC ACATCTTGGT GAAATTTTGG AATTCCTAGT GATAAAGGGG AAATNATAAG 240
GGCTNCTAGN GGGNGANGGG AATTNCNTGC CAGGGANCAG GNTCAGTTGN 290

SEQ ID NO:5697
SEQUENCE LENGTH:393
SEQUENCE TYPE:nucleic acid
TOPOLOGY:linear
CLONE:HUMGS06766
SEQUENCE DESCRIPTION:
GATCATTCCT GTATTAAACT TTCATATGCC AAAAGGGTTT GTCCCGTTTN ATCTGCCATC 60
AGTGTTTGAC CTGTTTAGGG CAGAGGCAAT AAGTCAGAAG TCTTGAAGTT GAAATAGTTA 120
TATGTGTGTC ATTGGACTGG ATTATAAACA GCTGTCTTGG ACTTTCCCTC TCTTAACACT 180
GACTGGTNAT CAGTATCATT AGTGAAAAAG NAAGCAAATT GTTTGTNATC ATCTCTTTAG 240

NCAGGTAAGC TGGAATGGGT GGGGCTTTAA ATAATANAAN CATGCGCATN GTTGGCTTGT 300
GTATGGGCCT NCTCTTGGGA TTCCTNGTNG TNATGGGCTT GGTATTTTGG GTNCNTGATT 360
TTGTCCANNG GCANTNCCAG GTGGGTGCCA NCN 393

SEQ ID NO:5698
SEQUENCE LENGTH:176
SEQUENCE TYPE:nucleic acid
TOPOLOGY:linear
CLONE:HUMGS06767
SEQUENCE DESCRIPTION:
GATCCANGNT AAGAGGCNCC CCCCCGACCA GCAGAGGCTC ATCTTTGCAG GCAAGCAGCT 60
GGAAGATGGC CGCACTCTTT CTGACTACAA CATCCAGAAA GAGTCGACCC TCAACCTGGT 120
CCTGCGCCTA NGGGTTGCTG TAAATCTTCA ATCAATGCAA TNNCNATGCC CATNAN 176

SEQ ID NO:5699
SEQUENCE LENGTH:178
SEQUENCE TYPE:nucleic acid
TOPOLOGY:linear
CLONE:HUMGS06768
SEQUENCE DESCRIPTION:
GATCTACTCA GACAGGACCT TCGCACCTTT GCCAGAGTTT GAGCAGGAAG AGGATGATGG 60
TGAGGTAACT GAGGACTCTG ATGAAGACTT TATACAGCCC CGNAGAAAAC GCCTAAAGTC 120
AGATGAGAGA CCAGTACATA TAAAGTAAAA TNACATGGAT TTAAATCAAT TGTTTAAA 178

SEQ ID NO:5700
SEQUENCE LENGTH:233
SEQUENCE TYPE:nucleic acid
TOPOLOGY:linear
CLONE:HUMGS06769
SEQUENCE DESCRIPTION:
GATCGTTTCT TTAGAAAAGT GGCATTGGCT TTTGAATATG AAATCTTCTC ATAGCTGCCT 60
AAAAAAGACT AGAATGTATA TGGATATCAG AAACCTTTAA TAATACATAA TTTATGCAAA 120
GATAAGATAA AGCAACATAA CACTAATTAT AAAACTATTT TTAAATCCCC ATTTTTAATG 180
ATAANTATAC ATTTNTATTT AATTTCCTTT GANTAAAACA NTTGTAAAGT AAA 233

SEQ ID NO:5701
SEQUENCE LENGTH:374
SEQUENCE TYPE:nucleic acid
TOPOLOGY:linear
CLONE:HUMGS06770
SEQUENCE DESCRIPTION:
GATCTTCATC TGACTATGAC ATAAAAACAA CTTTATACCC AGAAAGTTAT TGATAAGTTC 60
ATACATTGTA CGAAGAGTAT TTTTGACAGA NTATGTTTCA AACTTTGGAA CAAGATGGTT 120
CTAGCATGGC ATATTTTTCA CATATCTAGT ATGAAATTAT ATAAGTNTTC TAAATTTTAT 180
ATCTNGTAGC TTTATCAAAG GGTGNNANTT ATTTNNTTCA TACATATTTT NGTAGCACTG 240

ACAGATTTCC ATCCTNGTCA CTACCTTCAT GCATAGGTTT NGCAGTATAG TGGCGCCACT 300
GTTTTGAATC TNATAATTNA TACAGGTCAT ATTANTNTAT NGCCCTTAAA AAANATCAGT 360
TGTTCCAGNG GAAN 374

SEQ ID NO:5702
SEQUENCE LENGTH:452
SEQUENCE TYPE:nucleic acid
TOPOLOGY:linear
CLONE:HUMGS06771
SEQUENCE DESCRIPTION:
GATCCTCCTG CCTCAGCCTC CTGAGTAGAC GGATTATAGG CACCTTGCCC AGCTTCTATA 60
GCAGCATTTT TTTCTAACAC CTAAAAAATG GAAACAAGCC AAATGCTCAT CCACTGGTAA 120
ATGGATAAAC AAACTGTAGT CTTTCTATAC ACTGGAATAT TTGGCTATAA AANAGANTGA 180
NATATTGATA CATGCTACAA CATTGATGAN CTTCAAAAAC ATGCTAAGAG AAAGCCAGTC 240
ACAGAANAAT ACATNGTATT CTATAAGAAT GTCTAGGGAN CGTNAATCTA TATNGGCAGA 300
AGGTAGGTTN GTGGTTGCCT AGGGCCCGGG GGGAAGGGGT TAGGGGGCCT NGGGGGTGNC 360
AGCTTATGGG GCTGCTAATN GGGCCTNCCG NTTCTCTTTN GGGGGTTGGT ACCCTGCTAA 420
ATTGNCCTNN TTGGCNTGGT TTTNNCCCGG GN 452

SEQ ID NO:5703
SEQUENCE LENGTH:257
SEQUENCE TYPE:nucleic acid
TOPOLOGY:linear
CLONE:HUMGS06772
SEQUENCE DESCRIPTION:
GATCTGTCTG TGCACATGCC TCTGTAGAGA GCAGCATTCC CAGGGACCTT GGAAACAGTT 60
GACACTGTAA GGTGCTTGCT CCCCAAGACA CATCCTAAAA GGTGTTGTAA TGGTGAAAAC 120
GTCTTCCTTC TTTATTGCCC CTTCTTATTT ATGTGAACAA CTGTTTGTCT TTTTTTGTAT 180
CTTTTTTAAA CTGTAAAGTT CAATTGTGAA AATGAATATC ATGCAAATAA ATTATGCAAT 240
TTTTTTTTCA AAGTAAA 257

SEQ ID NO:5704
SEQUENCE LENGTH:77
SEQUENCE TYPE:nucleic acid
TOPOLOGY:linear
CLONE:HUMGS06773
SEQUENCE DESCRIPTION:
GATCANGAAG CTATTTTCNA TTCTGGGGAA CGATTATAAC TTAAATNATT GTTTTAATAA 60
AAATTCTAAG CTGGAAA 77

SEQ ID NO:5705
SEQUENCE LENGTH:278
SEQUENCE TYPE:nucleic acid
TOPOLOGY:linear
CLONE:HUMGS06774

SEQUENCE DESCRIPTION:
GATCCAATTT CTTTGTGGTA CCTGCAGTTT ACAAAATAAT TTGACTTCAG TGAGCATATT 60
GGTATCTGGA TGTTCCAATT TAGAACTAAA CCATATTTNT TACAAAAAGA TATTAATCCC 120
TCTACTCCCA GGTTCCCTTT ATATGTTAAG ATATAATGGC TTTGAGGGGG GAAAAAATAA 180
ACCTAGGGGA GAGGGGAGTT TCCTGTAGTG CTGTTTCATT AGAGGATTTC AGTAAATTAA 240
ATTCCACAGC TAATTGAATA AATAATGGNA CCTTTAAA 278

SEQ ID NO:5706
SEQUENCE LENGTH:308
SEQUENCE TYPE:nucleic acid
TOPOLOGY:linear
CLONE:HUMGS06775
SEQUENCE DESCRIPTION:
GATCTGCATA TNATGTATTA GGTTAGGTCA CAAAGGTTTT ATCTGAGGTG ATTTAAATAA 60
CTTCCTGATT GGAGTGTGTA AGCTGACCNA TTTCTAATAA AATTTTAGTT GTACACTTTT 120
AGTNGTCATA GTGAAGCAGG TCTAGAAAAT AAGCCTTTGG CAGGGAAAAA GGGCAATGTT 180
GATTAANCTC AGTATTAANC CACATTANTC TGTATCCCAT TGTCTGGCTT TTGTAAATNC 240
ATCCAGGNCA AGNCTAAGTA TGTTGGTTAA TAGGNATCCT TNTTTTTTTT TTNAANGCCT 300
AANNGGGN 308

SEQ ID NO:5707
SEQUENCE LENGTH:104
SEQUENCE TYPE:nucleic acid
TOPOLOGY:linear
CLONE:HUMGS06776
SEQUENCE DESCRIPTION:
GATCGTAACC CCTTGTTGAC TAATCTNGCC GTGGTTTTGT NTAGGTCTGC AGGAAGGAAG 60
NCTNAAAAAG CGGACGAAGA TTTTNACTTA AGTGGGACTT TGTN 104

SEQ ID NO:5708
SEQUENCE LENGTH:137
SEQUENCE TYPE:nucleic acid
TOPOLOGY:linear
CLONE:HUMGS06777
SEQUENCE DESCRIPTION:
GATCCTAGTA AGTCANTNGT AGTTTACTGG TGANGTGAGC ATTNTAGATT CCCCCNTTAT 60
CACTAATCAC ACAACAATTT TAAGAAGTAG GCTATAAAAC AAAAAAAGGT TGCTGTTTTC 120
TATTTTTAAA TAAACNN 137

SEQ ID NO:5709
SEQUENCE LENGTH:333
SEQUENCE TYPE:nucleic acid
TOPOLOGY:linear
CLONE:HUMGS06778
SEQUENCE DESCRIPTION:

GATCTCATTT GCTGGAATGA TTGATTTAAG AGTAAAATTA GCCACTGCGT NTGTNTNGTG 60
TATAGTAACC GGTTTTNTTG CACTTGTCTG TGGCTGAAAC ACTTACCTGC AGTGGAAAGC 120
CGTGCAAGGA GGAGGTAAAC ATTGGAGATG TTTGTNAAAA TATTACTCTT GCTGTGAGGT 180
TTTAGTTTGT TTTGAATGAG AAAAGACTAA GTAACTTAAT CCATCTCAAT GTAGTGTTTT 240
AATANNNNGG GNNCCTAATA TTTGANATGG GTCTCAGACA TGTCTACAAA TATGGGTACT 300
ATTTTNNTGC CCGTGTATTT TCACATTTNA NAN 333

SEQ ID NO:5710
SEQUENCE LENGTH:126
SEQUENCE TYPE:nucleic acid
TOPOLOGY:linear
CLONE:HUMGS06780
SEQUENCE DESCRIPTION:
GATCCTTATA AATNAGAAAC ATGCAAGCTT CAGCTGCTCG AGTTCTATAG TGTCACCTAA 60
ATCGTATGTG TATGATACAT AAGGTTATGT ATTAATTGTA GCCCGGTGNN CNTCGNGGGN 120
ACCTTN 126

SEQ ID NO:5711
SEQUENCE LENGTH:133
SEQUENCE TYPE:nucleic acid
TOPOLOGY:linear
CLONE:HUMGS06781
SEQUENCE DESCRIPTION:
GATCCGGCAA ACAAACCACC GCTGGTAGCG CGTTTGTNTT TGTTTGCAAG CTTCAGCTGC 60
TCGANTTCTA TANNGCCACC TAAATCGTAT GTGTATGATA CATAAGGTTA TGTATTAATT 120
GTAGNCGTTN GCN 133

SEQ ID NO:5712
SEQUENCE LENGTH:116
SEQUENCE TYPE:nucleic acid
TOPOLOGY:linear
CLONE:HUMGS06783
SEQUENCE DESCRIPTION:
GATCCTTTTA AATNAAAAAT AAAGTTTTAA AAAAAAACAT GCAAGCTTCA GCTGCTCGAG 60
TTCTATAGTG TCACCTAAAT CGTATGTGTA TGATACATAA GGTTATGTAT NCNNNN 116

SEQ ID NO:5713
SEQUENCE LENGTH:268
SEQUENCE TYPE:nucleic acid
TOPOLOGY:linear
CLONE:HUMGS06784
SEQUENCE DESCRIPTION:
GATCTTCTCA ATGGGTGGNC TCCTGGTATT CGTGGGACCA CCTGTGGGCA CAGGAGCCAA 60
TGTGGTTCAG ACAGCTGGGC AGAGGGAGCT GCTAGTCAGC TGTTTGCCCA CACTCTGCCT 120
GTCATGGTGA TTTGNACCCT ACCCCCCTGG TCCCAGNGNC CTGGGGCTCA CTTGATACCG 180

TTGAAAGTAA GCACAGNCTG CATGTNCTCA GGNAGGNCCT GGGGCTCAGG GCACTCGAAG 240
NCATCAATGN TGGGNACANT GTTNTTGN 268

SEQ ID NO:5714
SEQUENCE LENGTH:129
SEQUENCE TYPE:nucleic acid
TOPOLOGY:linear
CLONE:HUMGS06785
SEQUENCE DESCRIPTION:
GATCTCAGAA TACAAATTTA AATAAATTAA TTTAATTCTC AAAACACCCT TGATTGGAAC 60
TTACAGCTGG CCCTCTGTAT TCGTGGGTTC AACCAATCAC TAATTAAAAA TATTGGAAAC 120
AANNNNNNN 129

SEQ ID NO:5715
SEQUENCE LENGTH:214
SEQUENCE TYPE:nucleic acid
TOPOLOGY:linear
CLONE:HUMGS06786
SEQUENCE DESCRIPTION:
GATCCTGCAT TTATGGTGTA GTTCTGAGTC CTGAGACTTT TCTGCGTGAT GGCTATGCCT 60
TGCACACAGG TGTTGGTGAT GGGGCTGTTG AGATGCCTGT TGAAGGTACA TCGTTTGCAA 120
ATGTCAGTTT CCTCTCCTGT CCGTGTTTGT TTAGTACTTT TATAATGAAA AGAAACAAGA 180
TTGTTTGGGA TTGGAAGTAA AGATTAAAAC CAAA 214

SEQ ID NO:5716
SEQUENCE LENGTH:253
SEQUENCE TYPE:nucleic acid
TOPOLOGY:linear
CLONE:HUMGS06787
SEQUENCE DESCRIPTION:
GATCTAATCC CTTCAAGGAA GTGATAACAC TGGAGTGGTA ACAAGAGGAG CAGGAAGCAA 60
GGCCAGCCCT GGTTCTCCAT CCCCATGTGT TTCAGGTGGA ACAGGAGGAA CTGGNCCAGG 120
CCAGGCCTCA TCCTCCTGGA CCCAGCAGGG GCAGAAGGAG GAAGGGACTG GTCCAGGCAT 180
GGGTCCCTTC CCNNTGCTCC ATGGGCANCT CTGCTGTATT GATATCACTA GTAAAGTCTG 240
TCTGCACTGC AAA 253

SEQ ID NO:5717
SEQUENCE LENGTH:332
SEQUENCE TYPE:nucleic acid
TOPOLOGY:linear
CLONE:HUMGS06788
SEQUENCE DESCRIPTION:
GATCATGTCT TTCAGAATCC ATGTACATAT AGGCTGTNCT AGCCACAGTG GTAGATTTAT 60
GATTCACATC CGAAAGTGTG CCTTAGTTAT CAATTTAACA TTTCTCTAGT ATTCATATCC 120
CTTTCCTTAC CATATACCTC AGTGTATCCT TCCTGTGTCA AGCCTTATAA ATCATGTAAT 180

TTAGTGCCAC TCATGTAAAT CAGTAAAATC ACAAAGGTCT ATTTAATGAA AACGATTTAC 240
CAATGTTGTT TTTTTTTAAC AGGANAAGNA NTAGNAGCAC NTTGTCATTT TTNAGANGGN 300
GGNNTTGTTG GGNGNTGGGG GGGNNGNTTG GN 332

SEQ ID NO:5718
SEQUENCE LENGTH:284
SEQUENCE TYPE:nucleic acid
TOPOLOGY:linear
CLONE:HUMGS06789
SEQUENCE DESCRIPTION:
GATCATGCCA CTGCACTCCA GCCTGGGCAA CCAGGTGAGA CCCTGCCTCA AAAATAATAC 60
AATAAAAAGT AAAAGCAGCC CTGGGGAAAT GGAGACAGTG GACAAAGCAG GCTGGGGCAC 120
CAGGTGTGCA TGTGGGTGTG AGTCTGTCAT GAGAGCTGTC CTGANTGCCC TTAACGCCAC 180
TGAACTGTAT GCTGGAAAAC AGTGACAACA GTGGATTTTG TTATATAGAC AATTANANNN 240
GTATANCACC CAGAANANNT GTGTGNNTGT TGGGNNGTGG TGNN 284

SEQ ID NO:5719
SEQUENCE LENGTH:253
SEQUENCE TYPE:nucleic acid
TOPOLOGY:linear
CLONE:HUMGS06790
SEQUENCE DESCRIPTION:
GATCTTTAAC TCCTCCTGGC TGCACCTGGG TAGGGATGGT GGCATCGATG CCCCTCTGTC 60
TGCTGAAGGA CCTGTTGCTG CTTCTGTCTT TTCACCCCTC CTTGGCTGAT GACCCAGAGC 120
CCTCTGATGA TGGCATTCTC CTGGCAAGAG AAAAAGACTT AACTAGACTT CTGAACTTGA 180
ACAGTTTCAG GTTATATTTT AATTTTNNNT TTTTTTGTAC NGGTNCNGAT NCTAATACAT 240
TNCACCNNGC AAA 253

SEQ ID NO:5720
SEQUENCE LENGTH:389
SEQUENCE TYPE:nucleic acid
TOPOLOGY:linear
CLONE:HUMGS06791
SEQUENCE DESCRIPTION:
GATCCACCCA CCTCGGCCCC ACAAAGTGCT AGGATTACAG GTGTGAGCCA CCATGCCCGG 60
CCCAGAATAC TTTTTAAAAG AAGAGCAGGT TAGAGGAAAG AAAAAAATTG ATGCTGAATG 120
TGGTGATGAA AGCATGTTTC TAAAATGGGA AGCAGATGCT TAANGAGGAA AGACTAATCT 180
GGGATTTTGC CCCATTTCTC TGGNTTTTCA CTCCTATATT TNATTCTCAC AATCGTGTCG 240
TCACATAGTG CANAAANACAN ATTTCTTGTA AAGTCCCCAG GGGTTTATGC TTGGGTGAAA 300
GTTTTAGCCT GAGTATTTTT CTTNCTCTAA AAANAGGTGG GGANTGNGNC ATTTGAGGNT 360
NTTNACCTTT TAATTGTNCT GCTATNGGN 389

SEQ ID NO:5721
SEQUENCE LENGTH:174
SEQUENCE TYPE:nucleic acid

TOPOLOGY:linear
CLONE:HUMGS06792
SEQUENCE DESCRIPTION:
GATCTGGTTG GGGTTTTGGG TTTTTTGTTG TTGTTGTTTA TTTGTTATTT TAAAGGTAAA 60
TTGCACTTTT AAAAAAATAA TTGGTTGACT TAATATATTN NCTTTTTTNC TCACCTGCAC 120
TTAGAGGAAA TTTGAACAAG TTGGAAAAAA ACANNNTTNG TTTCAATTCT ANGN 174

SEQ ID NO:5722
SEQUENCE LENGTH:367
SEQUENCE TYPE:nucleic acid
TOPOLOGY:linear
CLONE:HUMGS06794
SEQUENCE DESCRIPTION:
GATCCTATGT TGCTGGAACT TGCCAAACAG GATGTTGTGC TCAATNACCC CATGTGAGTG 60
CGTGGACTCC TGGGAGCTCC TGCTCCNTAC AGTGGGCTGC AACTCCTGTA CTTGAAGCTG 120
AGACCTCATA TGACGTGGCC TTCGTGTTGT CAGAGAGTGT CTGGAAGCTG CTGTTGCCAT 180
CTTGAACAAC TCACCAACCT CCAACCCAGA GCCCCAGTGA GAGAGGAGCA TTTGGCCTCC 240
TGCTTCCTTC TGTGGCCTCT GCCGGGCTCC ATTNCCAAGG AAAAGAGAGG AGCTTGGGCT 300
CACAGAAAGA GAAGGGGATG AAACCCCAAG NGGCCCTATC TTTTGGGATT TACATGGGAT 360
TTTTATN 367

SEQ ID NO:5723
SEQUENCE LENGTH:311
SEQUENCE TYPE:nucleic acid
TOPOLOGY:linear
CLONE:HUMGS06795
SEQUENCE DESCRIPTION:
GATCCTGAGT AATCCTTTTA GAGCCCGAAG AGTCAAAACC CTCAATGTTC CCTCCTGCTC 60
TCCTGCCCCA TGTCAACAAA TTTCAGGCTA AGGATGCCCC AGACCCAGGG CTCTAACCTT 120
GTATGCGGGC AGGCCCAGGG AGCAGGCAGC AGTGTTCTTC CCCTCAGAGT GACTTGGGGA 180
GGGAGAAATA GGAGGAGACG TCCAGCTCTG TCCTCTCTNN NNNCTCCTNC CTTCAGTGTC 240
CTGAGGAACA GGACTTTCTC CACATTGTTT TGTNTTGCAA CATTTTGCAT TAAAAGGAAA 300
ATCCACTGAA A 311

SEQ ID NO:5724
SEQUENCE LENGTH:342
SEQUENCE TYPE:nucleic acid
TOPOLOGY:linear
CLONE:HUMGS06796
SEQUENCE DESCRIPTION:
GATCCTCCGG CCTCAGCTGG TCTACAGGCG GGTAATCAAG GGACTGGGAC TACAGGCGCG 60
CCCTGCCGCG CCGGGACAAG TTTTGTATTT TTAGTAGAGA CAGACTCTCC CTGTGTTGCC 120
CAGGCTGGTC TCCAACTCCT GGGCCTCCCA AAGTGTTGGG ATTACAGGCG TGAGTCACCC 180
CTGCAGGCCA CAGCTTTGCT TTTTAAAAGC ATTTCTGCAT AACTAAAAAG ACAGGAAATT 240
ACGAGCATAC GGNAACTGAT TTCTGCTTTC CTGAATTTTC TAAATGTATT TAATGGGCAT 300

GGTTCTTTTG CAATGAAAAA GTAATANATT NGGGTNTCAT CN 342

SEQ ID NO:5725
SEQUENCE LENGTH:312
SEQUENCE TYPE:nucleic acid
TOPOLOGY:linear
CLONE:HUMGS06797
SEQUENCE DESCRIPTION:
GATCCGCCGA CCTCGGCCTC CCAAAGTGCT GGAATTACAG GCGTGAGCAC GTGCGCCTGG 60
CCAAGAATGG ACATTTTTTA AAAAAACATC AGTACTTCCT ACCACTGCTG CATGAGTATA 120
ATGCTCCGGA ATTATCAGAA AGCATAATGC AGAAATACGA ATTAGTGGAA CTTAATCATG 180
TGCCATATAA GCTTACCTAA CAAACAGTTA TATCCCTATT CCTCAACTGA ATGTCTTTTA 240
ATAAATNAGA NTTTATCATT TNNNGTANGG GTGNGTGTGN GGNTGGNNGG GGGGGGGGNG 300
GGGNNGGGGN GN 312

SEQ ID NO:5726
SEQUENCE LENGTH:132
SEQUENCE TYPE:nucleic acid
TOPOLOGY:linear
CLONE:HUMGS06798
SEQUENCE DESCRIPTION:
GATCCTAGGC TCCCTCCATC CCTGGTATGT GGTCCTCATT TTCATATAGA CCAAGAGCTA 60
GAGCTCCAGT TACCATTTCT TAAAGTATAT TTTAATGCCA AATAGTGAAT AAAGGTGGCC 120
AGAAAATTCA AA 132

SEQ ID NO:5727
SEQUENCE LENGTH:135
SEQUENCE TYPE:nucleic acid
TOPOLOGY:linear
CLONE:HUMGS06800
SEQUENCE DESCRIPTION:
GATCANCTAA GGTCAGNAGT TNGAGACCAG CCTGACCAAC ATNGTGAAAC CCTGTCTTTA 60
CTAAAAATAC AAAAATTTAC CAGGCACCTA TAATNCCAGC TACTTCAAAG GCTGAAGCAG 120
GAGAATCACT TGANN 135

SEQ ID NO:5728
SEQUENCE LENGTH:334
SEQUENCE TYPE:nucleic acid
TOPOLOGY:linear
CLONE:HUMGS06801
SEQUENCE DESCRIPTION:
GATCAGTGGG CCTCCAAGGA GGGNCTGTAA AATGGAGGCC ATTGTGTGAG CCTATCAGAG 60
TTGCTGCAAA CCTGACCCCT GCTCAGTAAA GCACTTGCAA CCGTCTGTNA TGCTGTGACA 120
CATGGCCCCT CCCCCTGCCA GGAGCTTTGG ACCTAATCCA AGCNNCTCTT TGCCCAGAAA 180
GAAGATGGGG GAGGAGGCAG TAATAAAAAG ATTGANGTAT TTTGCTGGAA TANGTTCNGA 240

TTCNTCTGAA CTCAAACTGA GGAATTTCAC CTGTNANCCT GNGTCGTACA GANAGCTGCC 300
TGGTNTATCC AAAANGCTTT TTNTTCCTCC TGTN 334

SEQ ID NO:5729
SEQUENCE LENGTH:158
SEQUENCE TYPE:nucleic acid
TOPOLOGY:linear
CLONE:HUMGS06802
SEQUENCE DESCRIPTION:
GATCCTGTAA GCAGACTTGG GCAGTCTCCT TTAGAAATAG GTTGTCTGTA CATGTTCTAN 60
GGTNTTGTAG ACACGTGTGA CTGTNTANGT GTATTGATGT GAATANTATT AAATATCCTA 120
ATTATNTAAT TCATTGTATT GTTTCTGNGA AGTTGGGN 158

SEQ ID NO:5730
SEQUENCE LENGTH:106
SEQUENCE TYPE:nucleic acid
TOPOLOGY:linear
CLONE:HUMGS06803
SEQUENCE DESCRIPTION:
GATCCATANA AGAGCTCCTA AAGAAGAACC CACATGAAAA ACTATCAGAA TCATGTTTAA 60
GCTAAACCCA TATNCAAAGA CCAANCNACG NACCACCATT CTTCGN 106

SEQ ID NO:5731
SEQUENCE LENGTH:105
SEQUENCE TYPE:nucleic acid
TOPOLOGY:linear
CLONE:HUMGS06804
SEQUENCE DESCRIPTION:
GATCCTGGTG TGTGTATTAT ATAAAGAGAC CCCTCCCCTT ATTTTGTGTT TTCCATCCCT 60
CTCCCTTAAC AGGATTGAAA TAAAACATGC TTCTGTTTTT GTAAA 105

SEQ ID NO:5732
SEQUENCE LENGTH:243
SEQUENCE TYPE:nucleic acid
TOPOLOGY:linear
CLONE:HUMGS06805
SEQUENCE DESCRIPTION:
GATCANCAAA ATCCAATTTC ACATGTGGCA CTAGGAAAAT GGAATGCTAT AAAATTAATC 60
CCCATCACCC AAGTAGGGAT GCTGGTGCTT TTATACTGAG TCTTTACTGC CAATAAGTGA 120
TGCTGGCTGC AAAGGANCAG CTCTACAGAA AGTTTTTGGG TTAGTGTTTC CCTAGCTTAT 180
TNCTGCANTG GGGAAAGCCA GTTTCATACC AAAAANGANA ATGNAGGTGN TTATANTGGN 240
NNN 243

SEQ ID NO:5733
SEQUENCE LENGTH:194

SEQUENCE TYPE:nucleic acid
TOPOLOGY:linear
CLONE:HUMGS06806
SEQUENCE DESCRIPTION:
GATCTCGGTC ACGTGTCCCG GGCGCTGAGG GGAAAGGAAG CGGGCATGAT TGTAGACAAT 60
GAGGGGGTTC TCTTGATGTA ATGAAATGCA ATTTTATGGT TTGGTGCAAA AACTCCTATT 120
TTCCAGTAAA TTAACTTTAT TTCTAAAGCA TATTTTGATT TGCCATCAAG AGCAATAAAG 180
CATTAAATCT TAAA 194

SEQ ID NO:5734
SEQUENCE LENGTH:216
SEQUENCE TYPE:nucleic acid
TOPOLOGY:linear
CLONE:HUMGS06807
SEQUENCE DESCRIPTION:
GATCTGGGCC CTGAAGTATA TAAGTAGGAA TTTGCAAGAT GGATGAGGTA GAAAANNGTA 60
TTTCATATTG TGGAATAAAC AGAGGGAAGA GCCATTTGTC TTATCCCCAA CTCCAGAACT 120
CTACTACTCA TGGAGTCTGA CACAAACAAT GTTAATAATC TGTTTNAGTT CTCATNATAG 180
GGCACTGGAN TTGGCTGTAA ANTNCAAANC ACNTNN 216

SEQ ID NO:5735
SEQUENCE LENGTH:133
SEQUENCE TYPE:nucleic acid
TOPOLOGY:linear
CLONE:HUMGS06808
SEQUENCE DESCRIPTION:
GATCTTCTAG TTNCTGTGTT GTTTAGACTC AAAGAATCAC AAATNTGTCA GTAACATGTA 60
GTTGTTTAGT TATAATTCAG AGTGTACAGA ATGGTAAAAN TNCCAATCAG TCAAAAGAGG 120
TCAATGANTT ANN 133

SEQ ID NO:5736
SEQUENCE LENGTH:275
SEQUENCE TYPE:nucleic acid
TOPOLOGY:linear
CLONE:HUMGS06809
SEQUENCE DESCRIPTION:
GATCTTAGCA CCCAACTCTG CTGCCCTTNC TGCTGGGCAG TGCCTNTCCT AAGAGGGGAC 60
TCCCTAAAGG CCAGAGAGAG GGTGCTCATC TCCAGGACCT AGGCCACCTG TCAGCACAAA 120
CAGAACTTAT TCCAGACATA AACATGAGTC TTCTTATTTC AAACACTGGA GCAACTGNTC 180
AAATATCACT CTCCTACTTA CAGGNCTCAN CATACTNGTC ACAGCCCTTT ACTCCCNCTA 240
CATATTTNCC ACANCACANT GGGGCTCANT NNCCN 275

SEQ ID NO:5737
SEQUENCE LENGTH:211
SEQUENCE TYPE:nucleic acid

TOPOLOGY:linear
CLONE:HUMGS06810
SEQUENCE DESCRIPTION:
GATCATGCTA GTNACTTCCT GCCCCCAGGC ACCGTGCCAA ANACTGGATG CCCCCTACTC 60
CTCAGGGGAC TGTCCAGGGC GCCCAGTGGT AGTGAGGGAG AGTGTCTCTG TTCTTTTGCT 120
CAGCCTGCTG GGCCCTTTGT GTTTGAGGAT GCTTAATGAT TCCAGCCTCT CACTGTGCCT 180
TATGCATTAA AATTNCTTTG TTACGAGCAA A 211

SEQ ID NO:5738
SEQUENCE LENGTH:133
SEQUENCE TYPE:nucleic acid
TOPOLOGY:linear
CLONE:HUMGS06811
SEQUENCE DESCRIPTION:
GATCTNCTTT ATAGCCANCT CTCCTCACTT AACAGGTACT TTCTCCAAAA ACCTAAGAAC 60
CAAAGTCTTC CGCTTGCAAC TTGGGATGGT GATATGGCTG TGTCCCCACC CAAATCTCAT 120
CTNCACCTGN NNN 133

SEQ ID NO:5739
SEQUENCE LENGTH:169
SEQUENCE TYPE:nucleic acid
TOPOLOGY:linear
CLONE:HUMGS06812
SEQUENCE DESCRIPTION:
GATCATCGCA AGGAAGNAGG CAGACAAGAG TAAGGCACAC CTGACTCTTA GGACTAGCAG 60
TCAGAACCAG GAGGAAAGNT TTTATTGCTA TGCGGGTAGG TAAGAACAGA TTTTACTTAC 120
ATCCATATAG TTACTTAAAG TCCAGTTTTC TGTTAAACNN NNTTCTNNN 169

SEQ ID NO:5740
SEQUENCE LENGTH:127
SEQUENCE TYPE:nucleic acid
TOPOLOGY:linear
CLONE:HUMGS06813
SEQUENCE DESCRIPTION:
GATCTCTTGT CCACTAAGTA TCTTGTTAAA TGCCAGTATC TCAGTCTTTC TGAAGCCCTG 60
AAATGGTAAT TGTAGCATTT CAGAAAATNT CTTTCATTTC AATCAATAAA AAGCTTTTGT 120
AAATAAA 127

SEQ ID NO:5741
SEQUENCE LENGTH:179
SEQUENCE TYPE:nucleic acid
TOPOLOGY:linear
CLONE:HUMGS06815
SEQUENCE DESCRIPTION:
GATCTTCCAG ATTAGGCTAA ATGTAATGAA AACCTCTTAG GATTATCTGT GGAGCATCAG 60

TTTGGGAAGA ATTATTGAAT TATCTTGCAA GAAAAAAAGT ATGTCTCACT TTTTGTTAAT 120
GTTGCTGCCT CATTGACCTG GGNAAAATGN AACAANACAN TAAGGCAANT GGTAGGAAA 179

SEQ ID NO:5742
SEQUENCE LENGTH:196
SEQUENCE TYPE:nucleic acid
TOPOLOGY:linear
CLONE:HUMGS06816
SEQUENCE DESCRIPTION:
GATCAGCCCT NCCCTTCAGC ATCCTCAGNT CCTGNAGCAG AGCCTGGAAG ACACCCTAAT 60
GTGGCAGCTG TCTCAAACCT CCAAAAGCCC TGAGTTTCAA GTATCCTTGN TAACACGGNC 120
ATGACCACTT NCCCCGTGGG CCATGGCAAT TTTNACACAA GCCTGAAAAG ATGTTGTNTC 180
TTNTGTTTTT TTCTTN 196

SEQ ID NO:5743
SEQUENCE LENGTH:326
SEQUENCE TYPE:nucleic acid
TOPOLOGY:linear
CLONE:HUMGS06817
SEQUENCE DESCRIPTION:
GATCCGCAGT CCTAACAGGG TGAGAAAGCA CAAGAGGGGC CAGCTCGGCT GAGGCCTGAG 60
GAGAAATAAT GAGAAGTTTT GTTTCGTTCT TGGGCTGGGG CATCTCAGGA ACAGTGAGGG 120
CAACAAGCTG TCCCTCAGGG ACCCTCACCT GCCCTCCCAT GTGACCAGGC CCTTCCCAGG 180
TGGGGACTGG GGCTTGCTTT ATTAAGTGAG CTCTTGGGCT TTTGAAGTAG GACAGAGGGC 240
CCTGGTTTGG CAGAGAGGAG GCAAGGGAGG GCTTTAGAAC ATCTGAGAAA TGTTAATAAA 300
ATANTTCAGA AANTAGGAAA AAAATN 326

SEQ ID NO:5744
SEQUENCE LENGTH:105
SEQUENCE TYPE:nucleic acid
TOPOLOGY:linear
CLONE:HUMGS06818
SEQUENCE DESCRIPTION:
GATCTCGAAC CCTGTCTAGA AGGAATNTAT TTGTTGCTAA ATGTTCACTT TTTATAGTNA 60
TGTCCTGTAT TCTAAACAGT AAAGGGGNTT TATTNCTATC ACAAA 105

SEQ ID NO:5745
SEQUENCE LENGTH:167
SEQUENCE TYPE:nucleic acid
TOPOLOGY:linear
CLONE:HUMGS06819
SEQUENCE DESCRIPTION:
GATCTCAGAA GTTTTNAAAA ATAGCAAAGA AGACTGNATT TGGAAANCAT GGTCTACAAT 60
TGGNTGTTAA ATNCTGAAGC TATGAAGAAT AAATATTTCA ACTTTGGATT ATGAAACCCC 120
ATTTATGATT TTTTAAATAC ACTTGAAATA AAAATGATTA AACTAAA 167

SEQ ID NO:5746
SEQUENCE LENGTH:258
SEQUENCE TYPE:nucleic acid
TOPOLOGY:linear
CLONE:HUMGS06820
SEQUENCE DESCRIPTION:
GATCTNTAGA ATTNTTATTG TAATTTTCCT ACTTCTTTGA TAAAAGAAAA ATAAGTCAGA 60
TTGTTAACTC CAAGATTGAA AAAAAAAACT CTTGAAAGAN GATTATNAGT TGTAACNAAT 120
TTGGGGGGTC TGGGCACAGA CATCTAACCT GGTATTGTAA GGCAGAGGCT CCCATTGGAN 180
TGGTAGTGGT CCGGGTCAGT TGTTCATGGT GTAAGCTTTG CACNGTGTAT TGAACATTNG 240
NAGGNTCTGN CTNGAAAN 258

SEQ ID NO:5747
SEQUENCE LENGTH:350
SEQUENCE TYPE:nucleic acid
TOPOLOGY:linear
CLONE:HUMGS06821
SEQUENCE DESCRIPTION:
GATCCCTGCC CTTNTCAAGA CTTTAGCTNT TCCTTCCATC CGGTGGCCTA TTCCAGGAAT 60
TCCTCTTTTG CTTAAATCAG TTGGAGTTTG TNTCTGTTGC TTGTAATCAA GCCTTTATGG 120
CTGCTGGGCT GAGTGACACA AGCACTTTAA TGGCCTGGAG GGACTTTTAA TCAGTGAAGA 180
TGCAATCAGA CAAGTGTTTT NGAAAGAGCA CCCTCGNGAA GGGTGGATGA CAGGGCAGGG 240
CAGGAAGGNC AGGNAGATGG CAGAACGGTG GTGGCTGCAG CCGGGGGTNC AACCAGGANA 300
CTGGTTGGCA AGCTNGGGGC TAGGGGGTAA GGNNTTTTNA GGGGTNGAAN 350

SEQ ID NO:5748
SEQUENCE LENGTH:331
SEQUENCE TYPE:nucleic acid
TOPOLOGY:linear
CLONE:HUMGS06823
SEQUENCE DESCRIPTION:
GATCTTCCAT GTTTTGACGT TTGCAGTCAC ACACAACACC TTAGTTCCTC TAGGGGCTGT 60
ACAGTATTGT GGCATCAGAT AATGCCACCA AAGGAGACAT ATCACTGCTG CTGGGACTTG 120
AACAAAGACA TTTATATGGG TTTATTTTCA TTCTGCCAAA GTAAANCANT ACATCAACAN 180
GAGGAAACTC AGNTTTAACC TGTTATTTCT ATGANNNTGG GATGATTGCT TTGTTTATGC 240
ACTTTTTCCT GNCTGTGCAT CCGCCTGTNA GTGTTTTGCC CTNTAGTCCC TCACTAGCCA 300
TGCTTNTGTG NTGGGTTATC ANGGGNGTNN N 331

SEQ ID NO:5749
SEQUENCE LENGTH:236
SEQUENCE TYPE:nucleic acid
TOPOLOGY:linear
CLONE:HUMGS06824
SEQUENCE DESCRIPTION:

GATCGAGGAT ATTTTTTAAG AGGTTCAAGC TTGTGACTGT TAGTAGTGCA AGGATTTGTA 60
TGGGCTCAGT GATTACAAAC AAAGACATGC AAANTACAAC TGTTTAAACA TTTTCAAGGT 120
ATTTTTAAAG GTTTTGTATT GAAACANTGT TTATATTTNC TGAGCTCCTA AAAATGGTAG 180
ANTACACATG ANANCTTTTG TTTAGGAAAN GTTANCTTGT TAANTNCTNT TGATTN 236

SEQ ID NO:5750
SEQUENCE LENGTH:60
SEQUENCE TYPE:nucleic acid
TOPOLOGY:linear
CLONE:HUMGS06825
SEQUENCE DESCRIPTION:
GATCCCACCT TTGCTCCTGA CAACACTCAT TACACTAAAC ACCTCTTGGA TTTGGTGAAA 60

SEQ ID NO:5751
SEQUENCE LENGTH:129
SEQUENCE TYPE:nucleic acid
TOPOLOGY:linear
CLONE:HUMGS06826
SEQUENCE DESCRIPTION:
GATCACTGGA TGGATAGCTC AGCCTGGGGC ATTTAGTGTT TTCCCTGGTG ATAAATCCCC 60
AAGGCAGCTG GATTTGGAGC TGGTGGCAAG TTGAAATNAT TAAAAATTGA TTTGTGTGGG 120
ACTGTCAAA 129

SEQ ID NO:5752
SEQUENCE LENGTH:180
SEQUENCE TYPE:nucleic acid
TOPOLOGY:linear
CLONE:HUMGS06827
SEQUENCE DESCRIPTION:
GATCTTTGGA CTGAGAATGT TTGGTAACAT AGTCAAGCAT TTGTTAAGCC AAGTGTGGAA 60
ATGTTCACTT TTTAACTTTA CAGTTTTTTT AATGAGCAAT TCTACATTTC TAAGAAAAAA 120
GATACTTCAT TTTTATATAA GGTTACAACT GCTTTATAAA NCTGTATTCA CAATGTCAAA 180

SEQ ID NO:5753
SEQUENCE LENGTH:119
SEQUENCE TYPE:nucleic acid
TOPOLOGY:linear
CLONE:HUMGS06828
SEQUENCE DESCRIPTION:
GATCTGGCCT TTTCTTAACA AAATCNGTGC AAAANATGCA GGTGGATGTC CCTAGGTCTG 60
TTTTCAAAGA ACTTTTTCCA AGTNCTTGTT TTATTTATTA AGTGTCTACC NNGTAAATN 119

SEQ ID NO:5754
SEQUENCE LENGTH:67
SEQUENCE TYPE:nucleic acid

TOPOLOGY:linear
CLONE:HUMGS06829
SEQUENCE DESCRIPTION:
GATCTCCATA AATNACTTAG TCTTCTATGT ATAGCTATCA AGGAAATAAA ACCAATTTTG 60
CCACAAA 67

SEQ ID NO:5755
SEQUENCE LENGTH:323
SEQUENCE TYPE:nucleic acid
TOPOLOGY:linear
CLONE:HUMGS06830
SEQUENCE DESCRIPTION:
GATCAGAACA AAAACACCAC TCTTTAATGG GTCTTTTTCT TGGCTAGAAG TCTTTCCTCA 60
GTTTTTGTTG ACTATCATAC GCTGATTTGT GGGTNATGGA AGGGGGCTAG AGATAACGTT 120
TCTCTGTACT TTGGTAAAGN ATGAAGGAGT CAGAGAGGGA ACAGÁAAAAG TGAGAGAGTG 180
GCCTGCTGAA ATAATAGCCT GCCTCTAAAA GTTGTGACCA GAAAGTATGA TTCATTTGGG 240
CCCAGNGGGN GTAANNTATG TTTTTNCATN TACCTCCCAT AGNACANGGT GTTNGGAGGG 300
NNGGAGTGAG TGTNNTAGAN GGN 323

SEQ ID NO:5756
SEQUENCE LENGTH:274
SEQUENCE TYPE:nucleic acid
TOPOLOGY:linear
CLONE:HUMGS06831
SEQUENCE DESCRIPTION:
GATCAGGTGT CTATAACATT TTTAATTCTC TCTGGAAACA GACTCAGGTT TCTTTGGACC 60
AAATCCAAAA GAACACATAG CTGTAACACA GCTGTAGTTG ACTAGAATGC TCTGTATACT 120
TTATATTAAA AANTGCTTTG CATTTCTNCC AGTGCAATGA ANTTCATATG GTGTCCCACC 180
TTATTTAATG ATGGTACANT TTAAAATCTT AGTCAACTGC TGTNGAAAGT TTTCTCTATG 240
ANAGTNANGC TGTTTGAAGA ATTNANGNGN TTNN 274

SEQ ID NO:5757
SEQUENCE LENGTH:344
SEQUENCE TYPE:nucleic acid
TOPOLOGY:linear
CLONE:HUMGS06832
SEQUENCE DESCRIPTION:
GATCCGCCCA CCTCGGCCTC CCAAAGTGCT GGGATTACAG GTGTGAGCCA CTGAGCCCAG 60
NCTGTATCCA TGTNTTCTGC ATGTTGATTC AACCAGCTAC ACATGGACAT TGTAGTTAGG 120
CCTATGATGA TTGTGTCTGT TCTGAAGATG TACATACCTT TTCTTCTATC ATTATTCCCT 180
AAACANTAGA GTATAACAAC TATTTACACA GCATTTACAT TGTATTGGGT NTTNTANGTA 240
ATCTAAAGAT GGTTTAAAGN ATNCAAGAGG GTATGCATAG GNTATATGCG AATACCACCT 300
CATTTTANAT NGNTGACTTG NGCNTCGANN ATGAGGGGAT NTGN 344

SEQ ID NO:5758

SEQUENCE LENGTH:329
SEQUENCE TYPE:nucleic acid
TOPOLOGY:linear
CLONE:HUMGS06833
SEQUENCE DESCRIPTION:
GATCATGATA CAATTATTTT ATTAAGTCAT GGTTAATAAC AAATGAATCC AGACTTGTCT 60
AACAGATTTT CCATCAACAA ATATTGTTAT GTGCAAAAGT ATTGCCTATG TTGTTTTACA 120
CACCACTGCA TTAACTAGAA CTGCTGAGAG GACTGTATAT ATGATTTTAA ACCTAAGTTG 180
ATTTTTTTTC TCACTCTTGA AAGGAGTACT TCTTTGTGAA AGCAGTTCTT ACAGCTTTGT 240
TTTCAACCAG CTAAAAATGT TTTGTATATN ACTCTAACCT GTTGTCCTCC ACATTCTGTT 300
GGTNCTGATT GNTGCTGTNT NCTGGTTTN 329

SEQ ID NO:5759
SEQUENCE LENGTH:239
SEQUENCE TYPE:nucleic acid
TOPOLOGY:linear
CLONE:HUMGS06834
SEQUENCE DESCRIPTION:
GATCTATCCC TGCGGCCTCC ACACCTGAAC TTGCCTAACT AACTGGCAGG GGAGACAGGA 60
GCCTAGCGGA NCCAGCCTGG GAGCCCAGAG GGTGGCAAGA ACAGTGGGCG TTGGGAGCCT 120
AGCTNCTGCC ACATGGAGCC CCCTCTGNCG GTCGGGCAGC CAGCAGAGGG TNNGTAGCCA 180
AGNTGNTTGT NCTGGGGGCT GNCCNTGTGT ATTCACCACC AGGTAAATTA GACCATTNN 239

SEQ ID NO:5760
SEQUENCE LENGTH:143
SEQUENCE TYPE:nucleic acid
TOPOLOGY:linear
CLONE:HUMGS06835
SEQUENCE DESCRIPTION:
GATCAACTTT CCTTCTGTGT GTGGCTCAGN AGTTCTTCTG CAGAGATGGC GCTATCTTTC 60
CTCCTCCTGT GATGTCCTGC TCCCAACCAT TTGTACTCTT CATTACAAAA GAAATAAAAA 120
TATTAACGTT CACTATGCTG AAA 143

SEQ ID NO:5761
SEQUENCE LENGTH:52
SEQUENCE TYPE:nucleic acid
TOPOLOGY:linear
CLONE:HUMGS06836
SEQUENCE DESCRIPTION:
GATCGCATCC CACAATGCGA GAATAATAAA ATAAAATTGG ATATTTGAGA AA 52

SEQ ID NO:5762
SEQUENCE LENGTH:359
SEQUENCE TYPE:nucleic acid
TOPOLOGY:linear

CLONE:HUMGS06837
SEQUENCE DESCRIPTION:
GATCCTCCCG TNTCAGCCTC CTGAGTAGCT GGGATTACAG GCGCACACCA CCAATGCCCA 60
GCTAGTTTTT GTNTTTTNNA TAGAGACGGG GTCTCGCCAT GTCATTCAGG TTGGTCTTGA 120
ACTCCTGGGC TCAAGCAGTC TGCCTGCCTT GGNTTCCCAG TGCTGGGATT ACAGGCGTGA 180
GCACCGTGCC CGGCTAAAAA GTATTTTNAA GTTCTGCATA TNGCTTATTT CACTTAACAC 240
TATATTAGAG NTTGTTTTAT ANCANTACAT ATAGATATGC TTATCCTTGN TTGACAGTNG 300
CATAATTNTT CCANTAAAAT TTGGTTGTAT CNAGGGGCAN TTAACCCAGT TACTTAGNN 359

SEQ ID NO:5763
SEQUENCE LENGTH:308
SEQUENCE TYPE:nucleic acid
TOPOLOGY:linear
CLONE:HUMGS06838
SEQUENCE DESCRIPTION:
GATCATNAAG GACATTCGCA ATCTGGAGGA CATCAGGAAG ACCTTACCAT CTGGCTGCTT 60
CAACACCCCG TCCATTGAAA AGCCCTAGTC TCTTTAGGGC TGGAAGGCAG CATCCCTCTG 120
ACAGGGGGGC AGTTGTGAGG CCACAGAGTG CCTTGACACA AAGATTACAT TTTTCAGACC 180
CCCACTCCTC TGCTGCTGTC CATCACTGTC CTTTTTNAAC CAGGAAAAGT CACAGAGTTT 240
AAAGAGANGC AAATTAANCA TCCTGANTCG GGNACANAGG GTTTTATCTA ANANNNGGTN 300
GNNNGTGN 308

SEQ ID NO:5764
SEQUENCE LENGTH:91
SEQUENCE TYPE:nucleic acid
TOPOLOGY:linear
CLONE:HUMGS06839
SEQUENCE DESCRIPTION:
GATCATAAAT ATTAATAATN AAAACTGAGA AACTATTCAC AATGCATTCC TTATAAATAA 60
ATGCTACATT TAGTAACTCA TTTCACCCAA A 91

SEQ ID NO:5765
SEQUENCE LENGTH:320
SEQUENCE TYPE:nucleic acid
TOPOLOGY:linear
CLONE:HUMGS06840
SEQUENCE DESCRIPTION:
GATCAGCACA AGTAACAGAA AATNAGCCTG ACGGTGGCTT AAGCAATGGG AATGTTTATC 60
TCACATAGCA AAAAGTCTGT AAATAGATGG TTCTAGAGTT GGGTGGAAGC CAAAATGTCA 120
TCAGGATTTC CTTGGAACCG TCTGCTTTGC CATCCTCAGT ATTGCAGAAT GGCTGCTCCT 180
GCGCCAAGCA TCATTCTGCA AATNATGGTG TCCACAGCCA GAGTGGACAG TAGTACAGGN 240
GCCTTCTCAT TCTGCCCTTA TCCGGGANTA ACCGTCAGCN GCTCCAGCAA CCTNCCCCCT 300
GTCTNTTTAT AGNGTGGGNN 320

SEQ ID NO:5766

SEQUENCE LENGTH:320
SEQUENCE TYPE:nucleic acid
TOPOLOGY:linear
CLONE:HUMGS06841
SEQUENCE DESCRIPTION:
GATCCCACTC TGATTGCATC CATTTCTCTG AAAGACTTGT TTGTNCTGCT TCTCTTCATA 60
TAACTGAGCT GGCCTTATCC TTGGCATCCC CCTAAACAAA CAAGAGGTGA CCACCTTATT 120
GTGAGGTTCC ATCCAGCCAA GTTTATGTGG CCTATTGTCT CAGGACTCTC ATCACTCAGA 180
AGNCTGCCTC TGATTTACCC TACAGCTTCA GGCCCAGCTG CCCCCCAGTC TTTGGGTGGT 240
GCTGTTCTTT TCTGGTGGAT TTAATGCTGA CTCACTGGTA CAAACAGCTG TTGANGCTCA 300
GAGCTGGAGG TGAGCTTCNN 320

SEQ ID NO:5767
SEQUENCE LENGTH:111
SEQUENCE TYPE:nucleic acid
TOPOLOGY:linear
CLONE:HUMGS06842
SEQUENCE DESCRIPTION:
GATCCAGTGA CAATNTGCCC CCTGCCAGCC TGNGTGTCGT CACTCATCAT TCATTCATCC 60
ATNCTAGAGC CAGGTCTCTG CNTCCCAGAC GCGGCGGGNG CAATGCTCCT N 111

SEQ ID NO:5768
SEQUENCE LENGTH:256
SEQUENCE TYPE:nucleic acid
TOPOLOGY:linear
CLONE:HUMGS06843
SEQUENCE DESCRIPTION:
GATCATTCTC TCTGAGGGCA GNTGNGGCGC AGGNAAATAG TCTTGGNAAT GTTAAATATG 60
ATGGGTAAAT TAAAAGTTTT ACAACATTCT ACCTAATATT TTNCTTTTAA CATACTTTTT 120
CTGTTCTATT GTATTATGGT GTCCGAAAGC TAAATAACGN CTAGGNAAAA TTTTTTTAAA 180
AAAAGNAAAA TCAGTTTAAT GTGGGNAGTA CTTAAGTGGT ATTATATTTN NCATTTTCAC 240
GTATAGTGCA TANAGN 256

SEQ ID NO:5769
SEQUENCE LENGTH:163
SEQUENCE TYPE:nucleic acid
TOPOLOGY:linear
CLONE:HUMGS06844
SEQUENCE DESCRIPTION:
GATCGTGTTT ACAGAGGACT TCTTTAAAGT GTCACATTTT TAAAATGTAA ACATATTTTT 60
AATGCATACT TAAGTAATAT TTAAGAAACT AAACAAAATA ATGATACANT TGCAGTGTTG 120
TGCTTGTTGT CAATGACAAA AATAAAACCA TTTTGGTGGT AAA 163

SEQ ID NO:5770
SEQUENCE LENGTH:253

SEQUENCE TYPE:nucleic acid
TOPOLOGY:linear
CLONE:HUMGS06845
SEQUENCE DESCRIPTION:
GATCTCCAAG AAGTGACTCT AGCAGAAGAT TTTATGGTGA TAAGCATNCC AATCANGAAA 60
TGAAACCAGT GTTCCTGAGT GCACACTGAC ATGTCTGTGA ATATGTTACT GAACCTATAG 120
TCCAGTTTTT TNATNTCTTG TTTTAGTCTG AAATNATTTG GGCCCTAATA ATCCTAAAAN 180
GAATGGAGCT GCATTGATGA ATTGGCTCAG TATTTAANGG GAGCAAACTT NTNGATAATA 240
NNTCTTTTNA GGN 253

SEQ ID NO:5771
SEQUENCE LENGTH:137
SEQUENCE TYPE:nucleic acid
TOPOLOGY:linear
CLONE:HUMGS06846
SEQUENCE DESCRIPTION:
GATCTGAAAT ACTGACAGNG TTCCCCAAAG TTAGATATTA ACACAGCTTT TNACNTCTCT 60
TATGAGAACT TTACTGTAAA TATACCTTTA TCATAGAAAT GAGATATCCA ATACCTNATG 120
GNGACTACAA AAAGGNN 137

SEQ ID NO:5772
SEQUENCE LENGTH:371
SEQUENCE TYPE:nucleic acid
TOPOLOGY:linear
CLONE:HUMGS06848
SEQUENCE DESCRIPTION:
GATCCCAGAG TTCAGCCAGC CTGGGGTCCA GAACTCAAGA GTCCGCCTGC TTGGAGCTGG 60
ACCCAGCGGC CCANAGTCTA GCCAGCTTGG CTCCAATAGG AGCTCAGTGG CCCTAAGNAG 120
ATGGGCCTGG GGTGGGGGCT TATNAGTTGG TGCTAGAGCC AGGGCCATCT GGACTATGCT 180
CCATCCCAAG GGCCAAGGGT CAGGGGCCGG GTCCACTCTT TCCCTAGGCT GAGCACCTCT 240
AGGCCCTCTA GGTTGGGGAA GNAAACTGGA ACCCATGGCA ATAATAGGAG GGTGTCCAGG 300
CTTGGGNCCC TNCCCTGGTN CTCCNAGTGT TTTGNTNGGG TAATAANTTG GANCTATGGG 360
CTCTNAATTG N 371

SEQ ID NO:5773
SEQUENCE LENGTH:131
SEQUENCE TYPE:nucleic acid
TOPOLOGY:linear
CLONE:HUMGS06850
SEQUENCE DESCRIPTION:
GATCGCACAC TACGCTCCAG CCTAGGTGAC AGAGTGAGAC TCCATCTCAA CAACAACAAC 60
AACAAAAAAC CACAAAAACT ATATTTTTAA TCATGTTTGG TAGCTTGAAA ANTAAATNCT 120
TTCTGCCTAA A 131

SEQ ID NO:5774

SEQUENCE LENGTH:254
SEQUENCE TYPE:nucleic acid
TOPOLOGY:linear
CLONE:HUMGS06851
SEQUENCE DESCRIPTION:
GATCTGGAAA ANCTGCTGAC AGAGTGGCCA GACACAATAC CACTGTCTTT GGCTCTGGGG 60
NCAGTTGGCA TGCCAGGCCT GACTGCCTAC TTTGGCCTAC TTNAAATCTG TGGTGTNAAG 120
GGTGGAGAAA CAGTGATGGT TAATGCAGCA GCTGGAGCTG TGGGCTCAGT CGTGGGGCAG 180
ATTGCAAAGC TCAAGGNCTG CAAAGTTNTT GGNGCAGTAG GGTCTNNTGN AAAGGNTGCC 240
TACCTTNAAA AGGN 254

SEQ ID NO:5775
SEQUENCE LENGTH:346
SEQUENCE TYPE:nucleic acid
TOPOLOGY:linear
CLONE:HUMGS06852
SEQUENCE DESCRIPTION:
GATCCTGGTG CTATGGTTTG AATGTCCCCA CCAAAACTCA TGTTGAAATT GAATTGCCAT 60
TGTTTAACAG TATAAGGGGT GACTGGGCCA TGTGAGCTCT GCTCTCATGA ATGGATTAAT 120
GCCATTGTCA TGGAGTAGGT TAGTTTTCTC TGGCGTGGGC TCCTGGCAAA AGGATGAGTT 180
CTGCTGCCAT GCTGTCTGTN TCATGCACTT GCTCACCATG TGAGCCTTCT GCNTCACACA 240
GGATGACCCT CACCAGATAC TTGCACCATG CTCTNGGGAC TTTCCCAAGT NTCCAGAGCN 300
GTGAGCCAAA TNAACTNGCT NTNCTTTATA AAANANAAAN ANAGTN 346

SEQ ID NO:5776
SEQUENCE LENGTH:336
SEQUENCE TYPE:nucleic acid
TOPOLOGY:linear
CLONE:HUMGS06854
SEQUENCE DESCRIPTION:
GATCCAGGGT GNCTAAAGAA TAGAAGTAGA GTTCCACCCA GGGCCAGAGG GAACAATCTG 60
GGATTCAGAC ACAGCAGTGG TGCCAACGTG AATCTACCTC GTNCTAGAGT CTGAGTAGCC 120
TAGTCTATCA CCCAACACAG ATAAGACTGG ACAGGGGCTC CCAGGAACCA ACATCTAAGN 180
GGGGAGTTTG GAGAGGGGCT AAAAGAAAGC AGGTTAAGCC CCAANTGTAA CTGATTGATG 240
ACCAGGGATA ACTATTTACT AGTGCTTTTN TGGNTTTATT GTAACTCTGT NACTTTCCCA 300
GTATTTGTNA ATTNTTNNNC TTCTGNNTCT CCTGTN 336

SEQ ID NO:5777
SEQUENCE LENGTH:65
SEQUENCE TYPE:nucleic acid
TOPOLOGY:linear
CLONE:HUMGS06855
SEQUENCE DESCRIPTION:
GATCTATTTA GGTGGAAATA GTTGTGGATG TACTAAGAGT AATGAAATAA AATTATAGCT 60
TCAAA 65

SEQ ID NO:5778
SEQUENCE LENGTH:127
SEQUENCE TYPE:nucleic acid
TOPOLOGY:linear
CLONE:HUMGS06856
SEQUENCE DESCRIPTION:
GATCTTGNGA CGAACTGAGC CACGAGCGTG GCTTTAAGGG CCGTCCGAAC GCTGCAGGCC 60
GGCCAGGTCC CTGGGCGTCC AGGCCTGGCC TACGCACCAC TTTNTCCCTT AGCGTTTAAA 120
GGNNNNN 127

SEQ ID NO:5779
SEQUENCE LENGTH:118
SEQUENCE TYPE:nucleic acid
TOPOLOGY:linear
CLONE:HUMGS06857
SEQUENCE DESCRIPTION:
GATCCTATGT GAAAAGAAAA GTGAAGCAAC TGAATCTTCA GCATGTNCTC ATCGGCGGAC 60
CTTCTTGTGT AATGTAAACT GTGCCATGTT ATTAAAAAAT GTGAACTAAG CTTCCAAA 118

SEQ ID NO:5780
SEQUENCE LENGTH:259
SEQUENCE TYPE:nucleic acid
TOPOLOGY:linear
CLONE:HUMGS06858
SEQUENCE DESCRIPTION:
GATCTAGTAT AAACCTCACT TTACTCTTTT GGAATAAAGT GATTGGNTAG GACCCCATTG 60
CCAGGAATAA GANTCAATAT GCTGCTGAGA AGGAACCCAT GGTGTTGANG ATGTTGAAAA 120
GACANCACAG TCAGTAACGT GGAGAAATGG CCTGGNTTCA ANTCTTGGCT CTGACACTTG 180
GTAGGTATGA GACCTTGGAG ANGTTTCTGN NCCTCTCCAT GCTTCATTTT TNTGANGGTT 240
TATTGTGTGG TTTAANTTN 259

SEQ ID NO:5781
SEQUENCE LENGTH:171
SEQUENCE TYPE:nucleic acid
TOPOLOGY:linear
CLONE:HUMGS06859
SEQUENCE DESCRIPTION:
GATCCTNAGC GGAGGTTAAG ATATTAAGTN ATTTCCCATA TGAATCAGAA TTATATTGAT 60
TCTGTGCAAT CAAAACAAAA GGCAGANTAG AATGCTGAGA TTGGTTAAGT TTGCAATNAC 120
CATCTTGAAC CACAGATTTC TNCTATGTGT CATCAAAACA TCTAGTNNNG N 171

SEQ ID NO:5782
SEQUENCE LENGTH:114
SEQUENCE TYPE:nucleic acid

TOPOLOGY:linear
CLONE:HUMGS06863
SEQUENCE DESCRIPTION:
GATCCTCAGA CCCTGCACAG CCCTGANTGG CTTCACATCT NTTGGTCAGT GTCTTCATTC 60
TCCCCAGTCA GGGACCTNGG ATGTTTGTTA CTNCCAGAAG CCCTTNCTCA CCCN 114

SEQ ID NO:5783
SEQUENCE LENGTH:55
SEQUENCE TYPE:nucleic acid
TOPOLOGY:linear
CLONE:HUMGS06864
SEQUENCE DESCRIPTION:
GATCACACTG TGAAGATACA CAGAGTCAAT AAATGTGCAC TGTAATGGAC TTAAA 55

SEQ ID NO:5784
SEQUENCE LENGTH:298
SEQUENCE TYPE:nucleic acid
TOPOLOGY:linear
CLONE:HUMGS06865
SEQUENCE DESCRIPTION:
GATCCTGGAG AGCTCCCAGA CTTTGCTCAG CGTTCTCANG AGGNAAGCTG GGAACCTGAC 60
CAAGGCTACA GCCCCAGACC AGAAAAGTAG CGGCGGCAGG GACAGCTGAC CAGACCACGG 120
GCAGGGCCTG CNTCCNTNTG CCCCTCAGCT CAGCCCCAGC AAGTGTGTGC TCAGAGCATC 180
TTTGTTCTTC ACGGTAGCAG CTACCTTCCC TCACTGTCTC AGGTGCCGAG AGGGGCAGGT 240
GCCAGCCTNC ACTGGCATCA NTGACAAGNC CANGGCANGN NCCACCNGNG GGGTCCTN 298

SEQ ID NO:5785
SEQUENCE LENGTH:117
SEQUENCE TYPE:nucleic acid
TOPOLOGY:linear
CLONE:HUMGS06866
SEQUENCE DESCRIPTION:
GATCAATTGA TTGTTTAACA ATGGAATTTT GAGAAATATT TGGGTAGAAA AAAAACTAGT 60
TTTAAAAGAN GAGAATCCAA CCATTAAAAC ATCAAAANNT GANATTNTTT GTAAATN 117

SEQ ID NO:5786
SEQUENCE LENGTH:358
SEQUENCE TYPE:nucleic acid
TOPOLOGY:linear
CLONE:HUMGS06869
SEQUENCE DESCRIPTION:
GATCAGCATG TGGACTGCAC TGTTTACTTT TGGTTGGACT GGGACAATTT GGGGAATATC 60
TACTATGTTT ATTCTTCAAG AACCCATCAT CCCATTAGAT GGAGAAACCT GGAGTTATCT 120
CATTGCTATA TGTGTCTGTC CTACTGCAGC ATTCTTAGGA GTTTATTATG CCTTGGNCAA 180
ATTCCATCCA GCTTTGGTTA GCACAGTACA ACATTTGGAG ATTGTGGTAG CTATGGTCTT 240

GCAGCTTCTN GTGCTGCACA TATTTCCTAG CATCTATGAT GTTTTTGGAG GGGTAATCAT 300
TATGATTAGT GTTTTTNTCC TTGCTGGCTA TAAACTTTAC TGGAGGAATT TAAGAAGN 358

SEQ ID NO:5787
SEQUENCE LENGTH:128
SEQUENCE TYPE:nucleic acid
TOPOLOGY:linear
CLONE:HUMGS06870
SEQUENCE DESCRIPTION:
GATCTATGGG TAATTTTAAA TNTTGCCTGT CAAGCTTAAN AGGGACTTGA CAACAGCTAG 60
GATNGAAATN ATTTCAACAC TTAAATATGC TTACTTTGTG CCGGACGCTG TTCTAAGNCT 120
TTNACAAN 128

SEQ ID NO:5788
SEQUENCE LENGTH:339
SEQUENCE TYPE:nucleic acid
TOPOLOGY:linear
CLONE:HUMGS06871
SEQUENCE DESCRIPTION:
GATCCACCTA CCTTGGCCTC CCAAAGTGCT GGGATTACAG GCGTGAGCAC AGTGCCCGGC 60
CTGTATTTGG TGATATTTTA AAAAATTCTA CTTTGACCTT AAGTGCTTCA AGAATTGTGT 120
TCAGTTAGTA GTCCTNTTGG TAAGACTAAC TTTCATATGC TATCTTTGCT CCATGAGCTA 180
TCATAGTGCT GTTTTCTTTC ATTACCCGTA AGAGTGGCTC TATCACAGCA TTTACTGTTA 240
AGGGCTACAG TTAGACCTCT TGTTAACTCT ACTTTNATTT GTGATGGCTG TGTTTCACAC 300
TACCTTGATT TATAAATGTA NGTAATGTNG TTAAATNGN 339

SEQ ID NO:5789
SEQUENCE LENGTH:343
SEQUENCE TYPE:nucleic acid
TOPOLOGY:linear
CLONE:HUMGS06872
SEQUENCE DESCRIPTION:
GATCCTNTGT ATAACATCGG GGCCTTGCTC AGGGGCTGTT GCGTGGTTGC CCTGCATTCG 60
CTCCGNCGCA CCGCCTTCCG TATCAAAACC TAAATAGAAG TTGTTGTTAC CGTGTGCCAA 120
TGTGTCCCAT GTGGGTTGTG CCAGGTAGAG AAACAGGAAG TCAATCATCT GTNACAGTCT 180
CTATTCTGTC GTTTTGCTCC TTGGTATTTN ATTTGCACTA TATTNAGGTG AAGCCTGTTC 240
ACTGTTTAAA NCCGGAGTNT TCTTCAAANG GCATGGNGAC CTGGTTCCAG TAAATGTCCC 300
ANCAGTGGGG GTTTTAGAAN GCATNNTTCA NTGACCNTNG NAN 343

SEQ ID NO:5790
SEQUENCE LENGTH:330
SEQUENCE TYPE:nucleic acid
TOPOLOGY:linear
CLONE:HUMGS06873
SEQUENCE DESCRIPTION:

GATCATTATG TAGTTTCTGG ATTAAAAAAA TTTGTGTGTG AAGTTGCTTT GTAAAGTGCA 60
TGTGGAATTA ATGGGACAGT GTGCCCTTTG TGTTAGATGT TAGAGCAAAA GAAAGGGCTT 120
ATAGTGTTAG TATTGGAGCA CTTTGAAGAT AGATATTTNC AGAAAAGATG TAGGATTTAA 180
NAGTTAAATT TTAANTTTTA GAANANGATA TGATGGCAAT TGGNAATAGT CACAATGNNG 240
TTCTTCATCC AGTNGGTGTT TAACCAGTGT TNATTTTGCC NCTGGTAATG TGGTNACCTG 300
TGNGTGNTTT CCANTAGATG NGTTATGGGN 330

SEQ ID NO:5791
SEQUENCE LENGTH:265
SEQUENCE TYPE:nucleic acid
TOPOLOGY:linear
CLONE:HUMGS06874
SEQUENCE DESCRIPTION:
GATCACCACA AAGTCTGGCT CAGTGACTAT GGCTTTGCAA ACACTAAACA TTCCTGTCTC 60
AAGTCCTGTA GATGGCTTGA AATAGTATAC ATAAGCATGC CTTATAAACA TCTTCAGTCT 120
TTATCAACTC TTTGGAGATT CAGCCATTCT TTTTTGTACT TTAGGGAATT AAATCANNNN 180
ATAATGTATA AATCTGAGCA TGGCCGATGG CAGAAGANAT TTTGTCATAC GTTTTTAAAG 240
ANNTAAGANT GTATTATGTT TTAAA 265

SEQ ID NO:5792
SEQUENCE LENGTH:191
SEQUENCE TYPE:nucleic acid
TOPOLOGY:linear
CLONE:HUMGS06875
SEQUENCE DESCRIPTION:
GATCAGAAAC CCTCATTTAA TATAGATTAT TTTGGAAATN AAAAAATTTG TAACAAGGTG 60
GCTTTCTTAC CCACCCTAAA ATGTAAATAA AACATGCAAG CTTCAGCTGC TCGAGTTCTA 120
TAGTGTCACC TAAATCGTAT GTGTATGATA CATAAGGTTA TGTATGCCCT GTAGCCGTTN 180
NNNNNTNTNT N 191

SEQ ID NO:5793
SEQUENCE LENGTH:204
SEQUENCE TYPE:nucleic acid
TOPOLOGY:linear
CLONE:HUMGS06876
SEQUENCE DESCRIPTION:
GATCTTGTCT TTATCTACTT TCTTCAGTAA ACTCTGGAAG AAAAAAACTT CCTGGAAGTT 60
TGGGCAATCA TTTGCCATTA TTTNCCTTTC CTTGAATTTC TTGATATCAT GAATACTGCT 120
GTAGTATGGG ACATTATGTA TTTNGGTAAG CAGACTTCTC TNCTGTATTT GTNTTTNGTT 180
AAACAGAATG ACAAGTTCCT TAAA 204

SEQ ID NO:5794
SEQUENCE LENGTH:237
SEQUENCE TYPE:nucleic acid
TOPOLOGY:linear

CLONE:HUMGS06878
SEQUENCE DESCRIPTION:
GATCCCACCC CAGGCCTTGC CCCTGCCCTC CCACGAATGG TTAATATATA TGTAGATATA 60
TATTTNAGCA GTGACATTCC CAGAGAGCCC CAGAGCTCTC AAGCTCCTTN NTGTCAGGNT 120
GGGGGGTTCA GCCTGTCCTG TCACCTCTNA GGTGCCTGCT GGCATNCTCT CCCNCATGCT 180
TACTAATACA TNCCCTTCCC CATAGCCATC AAAACTGGAC CAACTGGNCN NTTCCTN 237

SEQ ID NO:5795
SEQUENCE LENGTH:214
SEQUENCE TYPE:nucleic acid
TOPOLOGY:linear
CLONE:HUMGS06879
SEQUENCE DESCRIPTION:
GATCAAAAGC CAAGCTCTGT CCCCGTCAGC ACGTCACCCT CCCTCACTGT AATCCCTGAC 60
TGTTTTCTCT AAATGAAGAA TTATTTAGAG AATATAANTT AGAAGGGCCG CCACGTCAGA 120
TTTGCCTGAN TGGGAGCAGT CTTTCCAGCC TCGAAAAATG TTTAANCAAT ATGCAGATGA 180
CCTAGCACAA ATAANGANTG CTAGCAACCG CAAA 214

SEQ ID NO:5796
SEQUENCE LENGTH:187
SEQUENCE TYPE:nucleic acid
TOPOLOGY:linear
CLONE:HUMGS06880
SEQUENCE DESCRIPTION:
GATCAAGTGA TACTAGGAAT ATAGGTACAA GTAAGACTGT CATTTNCATT GTTTTCTCTT 60
TGGACTGATT CTTTNCATTA TAACAGCGTT GTGAAGTAGA GGTGGGATTA GGAACCNTCT 120
GCTGTAATCA ACTCCCTNGC TGCCCAAACA CCAGCCTNAT ACACAACACC ANCAGGTCCN 180
GCNTNGN 187

SEQ ID NO:5797
SEQUENCE LENGTH:247
SEQUENCE TYPE:nucleic acid
TOPOLOGY:linear
CLONE:HUMGS06882
SEQUENCE DESCRIPTION:
GATCTTTNAG TGCTTTGGGG ATGGGGTGGG CTGGGGTGGA TGGATGGGCA ACAGTGACTT 60
TAATTACCCT TGCTGCTCTG CATTTNCCAG TTTATNCTTT TGTTTCTTTT ATCTGACTGA 120
CTCTGTCAAA CAAGTGTCAA AGTTGTGTGT TAAAAAATGT TTAACAAAAA ANNTGTTGTA 180
ATGACACAAA GCCTTATGAN ANTNTTTATG GAGTTCAATA AAAGANGTAA GAGGTCAANC 240
CNNAGGN 247

SEQ ID NO:5798
SEQUENCE LENGTH:106
SEQUENCE TYPE:nucleic acid
TOPOLOGY:linear

CLONE:HUMGS06883
SEQUENCE DESCRIPTION:
GATCTTCCCA ATATGAACAA ATATACTATG TATATNGTGT GTATTTCTAG AAATCAATGG 60
CAGCTGCTGA TGGNGTTGTA ATTAGAANTC TATATAGNNT ATAGAN 106

SEQ ID NO:5799
SEQUENCE LENGTH:214
SEQUENCE TYPE:nucleic acid
TOPOLOGY:linear
CLONE:HUMGS06884
SEQUENCE DESCRIPTION:
GATCAGGACC TACGTTAAGG TGGATTTTNA ATTATTTAAA ATTCTGATTT TGTTTTATTT 60
TTAATTAATC TTTGGGGGAA GGATGGGAGG GGGGTGAGGG TTGAAGAAGT AAACATTGTA 120
CCTATTGGAT ACAATGTTTA ATATTTGTGT GATGGGCACA CTGGAAGCCC AAACCTCACT 180
ATTACACAGT ACATTCATGT AACAAGCCTG CAAA 214

SEQ ID NO:5800
SEQUENCE LENGTH:119
SEQUENCE TYPE:nucleic acid
TOPOLOGY:linear
CLONE:HUMGS06885
SEQUENCE DESCRIPTION:
GATCCTTAAT GGTGGCCAGA GCTACCATGC AGTGCAAAAA TAAATTAAGT GAAAATGTAT 60
TATTGAGATT AACAGTGCAA GGCCAATACA TTTTATAAAT CTGTCAGTAT CTTTTTAAA 119

SEQ ID NO:5801
SEQUENCE LENGTH:339
SEQUENCE TYPE:nucleic acid
TOPOLOGY:linear
CLONE:HUMGS06886
SEQUENCE DESCRIPTION:
GATCCTCAGT GAAGTGCATT CGGAATGAGG ATGGAACTTG GTTAACACCA AATGAATTTG 60
AAGTCGAAGG AAAAGGAAGG AACGCAAAGA ACTGGAAACG GAATATACGT TGTGAAGGAA 120
CGACCCTAGG AGAGCTGCTN AAGAGTGGAC TTTTGCTCTG TCCTCCAAGA NTAANTCTCA 180
AGAGAGAGTT AAATAGCAAG TGAATTTCTA CTACCCTCTC AGTCACCATG TTGCAGACTT 240
TCCCTGTCTG GAGGCTCACC TTAGAGCTTC TGAAGTTTCC AAGCTCTGAG GTCAACCTNC 300
ACATTTTGGG CAATGGCATC TTCAAAAACA ATTAATTTN 339

SEQ ID NO:5802
SEQUENCE LENGTH:381
SEQUENCE TYPE:nucleic acid
TOPOLOGY:linear
CLONE:HUMGS06888
SEQUENCE DESCRIPTION:
GATCTCCAAG CGAACTCAGC CTCCTCCCAA GCTCCCTGTG GGTCCTAGCC ACAAGCTCTC 60

CAACAATTAC TATTGCACTC GCGATGGCCG NCGGGAATCT GTGCCCCCTT GCATCATCAT 120
GTCGTCGCAG AAGGCGCTGG TGTCAGGCAA GCCAGCAGAG AGCTCTGCTG TAGCTGCCAC 180
TGAGAAGAAG GCGGTGACTC CAGCTCCTNC CATAAAGAGG TGGGAGCTGT CCTCGGACCA 240
GCCTTACCTG TGACATTNCA CCNTNACGNC CACCCGNCTA CTTTTGCCTN CTTTGGATTT 300
NCTTCAGGGN GAATNTTGAC CTAATTGTAT GACAATTTAC GTAGGGGCTC AGGTATTCAC 360
TTCTGAGGTN TTACTTTTTG N 381

SEQ ID NO:5803
SEQUENCE LENGTH:150
SEQUENCE TYPE:nucleic acid
TOPOLOGY:linear
CLONE:HUMGS06889
SEQUENCE DESCRIPTION:
GATCTACACA TGTTGCCTTG CATTTGTGGT AATCTACACC AATGGAAACA TGTACTACAG 60
CTATATTTGA TTATGTATGG ATATATTTGA AATAGTATAC ATTGTCTTGA TGTTTTTCCT 120
GTAATGTAAA TAAACTATTT ATATCACAAA 150

SEQ ID NO:5804
SEQUENCE LENGTH:276
SEQUENCE TYPE:nucleic acid
TOPOLOGY:linear
CLONE:HUMGS06890
SEQUENCE DESCRIPTION:
GATCCTAAGC AAAATCGGAG ACTTGAACAA AGTTTGTTTC TGGCTGTNTG CCCTTCCATT 60
TTCTGTTCTC TTCTTTCTCC CTGGAGATGA NAGAACACAG AATCANATTN CCAACCTCTC 120
TCTCCNATGN ACTCTCTCAT CCCCCAGCCC AGCCACATTT ACACATAGGG CAGACACATC 180
TCACACAAAG GAAGTACCCC TAAAGACCTC ACATAAATAA ACACTAGCAA ANTTCAAGTT 240
GNNTCTTGGC AAANGTAGTN NTGGGTATGT NAGTCN 276

SEQ ID NO:5805
SEQUENCE LENGTH:219
SEQUENCE TYPE:nucleic acid
TOPOLOGY:linear
CLONE:HUMGS06891
SEQUENCE DESCRIPTION:
GATCANGTTC NGGGCTCTNT CAGCCGCCAC CTCTGGGAAA GAGAAAAGNT TTGGGTCCAC 60
TGAACATCAT GTTTGTAGAC GCTGACAGGT GGGGTCCTAA TNAGAGCCAA CACATGCTCA 120
CTGCCAGCNC CTGCCCTGAG TACTGGGAAG TTTCTCCTGG AAGCCCTTGT GAGATGGCTC 180
NGNNGGCTGG TATCCCGACT TGGAAGATGA GGAAACTNN 219

SEQ ID NO:5806
SEQUENCE LENGTH:191
SEQUENCE TYPE:nucleic acid
TOPOLOGY:linear
CLONE:HUMGS06893

SEQUENCE DESCRIPTION:
GATCAGTTTT NTGCTGAAGG CACCTACTCA GTATCTTTTC CTCTTTATCA CTCTGCATTG 60
GTGAATTTAN TCCTCTCCTT TGTGTTCAAC TTTTGTNTGC TTTTAAAATC AGCTTTATTC 120
TAAGCAAATC TGTGTCTACT TTAAAAAACT GGAAATGGAA AAAAAATTAA ATCTNNGCCA 180
GGTCCTTCAA A 191

SEQ ID NO:5807
SEQUENCE LENGTH:206
SEQUENCE TYPE:nucleic acid
TOPOLOGY:linear
CLONE:HUMGS06894
SEQUENCE DESCRIPTION:
GATCTGCAGC TTCGCGGGGA CAGAGATGTA ACCCAACTCG TTCACGGATG TNCCGCGCGC 60
CGTGTCACCG GCTGCGGGCC AGGGGTACTC GGAAGGCGCG GGCAGGAGCC TNGCGAGGAT 120
GCACCTTCCC CTGCNTTGGA AAGGTAGAAC TGGGGAGTGC GGGAGGNTGA AGGTGGCCCG 180
GAAAAGAACA CCTNTTCGCA ACCAAA 206

SEQ ID NO:5808
SEQUENCE LENGTH:55
SEQUENCE TYPE:nucleic acid
TOPOLOGY:linear
CLONE:HUMGS06895
SEQUENCE DESCRIPTION:
GATCTAAATA TGTATTTCAT AATNTTCCCA TAATAAATTA TATAAGGTGG CTAAA 55

SEQ ID NO:5809
SEQUENCE LENGTH:197
SEQUENCE TYPE:nucleic acid
TOPOLOGY:linear
CLONE:HUMGS06896
SEQUENCE DESCRIPTION:
GATCTGGTTT NATTCCTGTA ATTCAGCCAC CTGATTTGCT TGAGGGGGNG GNATANTATG 60
TGGGTTTTTG TACAAACATG TTTCTCAGTG TGTTGTTATT TTGGAAAAAA TGAGGGGAGG 120
GAGTTTTGCA AGAATGNGAG AAAATGANTG AAGAAGGCCT AATCNTCTCT CTTTTTCCAG 180
TGGATTNAAT GGGNCCN 197

SEQ ID NO:5810
SEQUENCE LENGTH:344
SEQUENCE TYPE:nucleic acid
TOPOLOGY:linear
CLONE:HUMGS06897
SEQUENCE DESCRIPTION:
GATCCAATGA TTTAATTTTT GGGAAGAAAA ATCCAGTGAT TAAAGTCATG CAAGGAGATG 60
GCCTGGGGCT TGCTAAAAGT CAGGTTGCAG TTTCCATTGC ATTCAAGAAA ATCAGAAAAA 120
TAAATACAAC TTTTAGAAGA AACTGAGTTT TCTCCCATTC ATGTTTAGCT ATTGGGGGGT 180

CTCATACTTC TGGACTTGCT TAGACCTATG CTATCTAATA TGGTNGCCTC TGCCCATCCA 240
TATGTGGCCA CTTGGGAACT TGGAAATGTN ACTAGTTTTG AATTAAGGTG TGCTNGTAAC 300
TAAAATNCAC CCTGNGGTNT CAAAGGCTNN AATAAANGNA NGGN 344

SEQ ID NO:5811
SEQUENCE LENGTH:111
SEQUENCE TYPE:nucleic acid
TOPOLOGY:linear
CLONE:HUMGS06898
SEQUENCE DESCRIPTION:
GATCTATCTC CCTGTTAGGT GTNCTTTTTC TCCTCTTTAG AAAGNAGCCA AGGNAACCAG 60
GGTTCTCTCA AAGTGGAAAA TACTGGAACT NATGTACTGN TATCATAATG N 111

SEQ ID NO:5812
SEQUENCE LENGTH:169
SEQUENCE TYPE:nucleic acid
TOPOLOGY:linear
CLONE:HUMGS06899
SEQUENCE DESCRIPTION:
GATCCATTGC TTTTAGACAG GACTGGGTTT TGCTGTCCAA TGATATACAN TAATAGTNCT 60
TCTTACAGCT AAGCTGGCCC CAGCCTTGTC TTGATATTAA TACATGAANT NTTTATAATT 120
GTCTCATTGT CTCATTTAGA AACATCCANA TNTTNCTGCT GTGNCTATN 169

SEQ ID NO:5813
SEQUENCE LENGTH:112
SEQUENCE TYPE:nucleic acid
TOPOLOGY:linear
CLONE:HUMGS06900
SEQUENCE DESCRIPTION:
GATCAGTAAC TTTATCTCTA TCCTTAATGA ACATTTGTTT TATTGGTGGC TGGAAATATT 60
TCTATTGTAT TTCTGTGTAT ATNNTNAATA AAATAATTTT TGGCCTCTNA AA 112

SEQ ID NO:5814
SEQUENCE LENGTH:271
SEQUENCE TYPE:nucleic acid
TOPOLOGY:linear
CLONE:HUMGS06901
SEQUENCE DESCRIPTION:
GATCAGCAGC TCGGAAGACT CAGATGCCGA AAACTCGTCC TCCCGAGAGC TGGATGACAG 60
CAGCAGTGAG TCCAGTGACC TCCANCTGGA AGGCCCCANC ACCCTCAGGG TCCTTTGACG 120
AGAACCTTGC TGACCCCCAA GCAGNAGACA GACCTCTGGT TTNNTTTGAC CTCAAGATNG 180
ACAATGAAAG TGGGTTCTCC TGGGGCTACC NNNACCCNTT TCTAATTTAG TCTCTNGAGT 240
CCCAAAAAGA AGTNCAGGCA GAGCCATCTN N 271

SEQ ID NO:5815

SEQUENCE LENGTH:317
SEQUENCE TYPE:nucleic acid
TOPOLOGY:linear
CLONE:HUMGS06902
SEQUENCE DESCRIPTION:
GATCATGAAA AGAAAAATNA ATGGCAGGAA AAAAGTTTGG TCCTTAATAT ACTTTGGCCT 60
AGTNAAAATA TGTGCCTTTT TGGTGTGTTT TGTTCATCAC TACAAGATAA AAAGGAAACA 120
TTACANCTCA AGGCTTTAAA AAGTTCATTT ATTGAAAATC ATATGTATAG CCTAGCATAC 180
GAATGTGCAG TTTTAAACAC ATAGCTTCAA GCCATTTCTG NAAGCATNCA CCGGGNGCTC 240
TGCTCAGNTA GAGTCAGNCT CCNNGCTCCA GNCCGACTGC GTGCGGGGGA CCAGCGGCCG 300
CGTTNNATGG NGGGNCN 317

SEQ ID NO:5816
SEQUENCE LENGTH:294
SEQUENCE TYPE:nucleic acid
TOPOLOGY:linear
CLONE:HUMGS06903
SEQUENCE DESCRIPTION:
GATCTAGGAG AGGCGGCTCT GAACGAGTAC CTGCGGGTCA AGACAGTGAC CTTCGATTAC 60
TGAAGAAAGG TCTTTNTGAG AAGAAAGTCC CTGCCCCTCC CTCGTGGCTG GGGTCCCCTC 120
CCTCTTGAGC CTNGGTGCAC AGCANCTCCC ACCTGGGGGG CTAGTGGAAG CCCTCCTGCC 180
TGCACACCAT GTCTGCATCT TGGACGNCCT CTGTCCAGTC AGAAGCAGCC CTTGGNTNNG 240
GGTGAGNTGT GNCCCTTCCA GGGGGAATAA AGCTTCTGAA GAGAGACCGT CAAA 294

SEQ ID NO:5817
SEQUENCE LENGTH:263
SEQUENCE TYPE:nucleic acid
TOPOLOGY:linear
CLONE:HUMGS06904
SEQUENCE DESCRIPTION:
GATCAAAATC AATGGGAAGG TGCTGCCAAG GTGTCCTAAG CTTCTAGGAT AATATTNATN 60
CTCTCAGGGT TTTGGAACTG CTGCCCAAAG TCATAGTTCT CAGTCCTGGG TGTGAGTCAG 120
ANTCACCTGT GGAGCCTGGC AAACCTTACA GACACCCTGG CTCCAGCCCT GGAGATTTTN 180
ATTTANTGGA AATATCACTT AGAATTCTAT TGTAAGCAAT AAAAGTNTAT TCTCACTAAA 240
CTTTCAAAAA NTNGNGNTGN NNN 263

SEQ ID NO:5818
SEQUENCE LENGTH:199
SEQUENCE TYPE:nucleic acid
TOPOLOGY:linear
CLONE:HUMGS06906
SEQUENCE DESCRIPTION:
GATCTTTCCC NTTTGGATGT GCGTGTGTGT CTGCNTGTGC CATGTGCGTG GCACGCATAT 60
GAAGTGTGTG TGCGTGTGAA CGGCTTTGGG TCCTGCTGGT TTTNCTGTGA GCTNCAGTGT 120
TCTGTGGGTC TGTGGTATCT NACACTGTGG NCATTAATGT ACTTCTTGGA CATTTTAATA 180

AATTTTTNAA CAGTTCAAA 199

SEQ ID NO:5819
SEQUENCE LENGTH:104
SEQUENCE TYPE:nucleic acid
TOPOLOGY:linear
CLONE:HUMGS06909
SEQUENCE DESCRIPTION:
GATCANAAAA CCTAAANTCC ATAGGNACCC NAAATCCTCA CAGTTACAGA GACACCAAGA 60
AGANTCTGGA CAAATAGGAC TTGCTAAGGC CTCCACAGTT TATN 104

SEQ ID NO:5820
SEQUENCE LENGTH:343
SEQUENCE TYPE:nucleic acid
TOPOLOGY:linear
CLONE:HUMGS06910
SEQUENCE DESCRIPTION:
GATCGAACGG ACTNTAAATC CGCTCTTTAT CGAAAGCTAA GCAAGCTGTG GCTTTTTTCC 60
AACTCCGTGT AACGATTCTA AGTGTAGTGT NGTAGGACCC CGACGGGTNT GGCAGCAACT 120
GCCCTGGAGC CCCAGCCCCT GCTTCCATCT NTNCTGTGCG CCCCACAGTA GACGTGCAGA 180
CGTCCCTAAG AAGGTTCTTN AAGATGTTTA TTTTATATAG TCCTNTTTTA CTGGAAGACG 240
TACGNATACT CCATCGATGT TGTATTTGGN GGGGGGTGNG GAATTCTTGT ACGCAGTTTT 300
CNTTNNGCTT TACGAAGCCG GTNAANNGAC CGTTTTGAAN TAN 343

SEQ ID NO:5821
SEQUENCE LENGTH:106
SEQUENCE TYPE:nucleic acid
TOPOLOGY:linear
CLONE:HUMGS06911
SEQUENCE DESCRIPTION:
GATCACTTGA GCCCAGGAGT TCAAGACCAG CCTGGGCAAC ATGGCTAATN AACACTAAGC 60
ATAGTTTTAA TGTCTTTNAT ATTAAAGACT TTCTCAAATT CTTAAA 106

SEQ ID NO:5822
SEQUENCE LENGTH:350
SEQUENCE TYPE:nucleic acid
TOPOLOGY:linear
CLONE:HUMGS06912
SEQUENCE DESCRIPTION:
GATCCATTCC AGCCACTCGG GGCTGACAGC GGCGACCCGT TCCAAAGTAA AAAGGGGTTT 60
GGGGACCCGT TTAGTGGAAA AGACCCATTT GTCCCCTCCT CTGCAGCTAA ACCTTCTAAG 120
GCCTCTGCCT CGGGCTTTGC AGACTTCACC TCTGTAAGTT GAGTCCTCCG NCTCCGGGCC 180
ACCCCACTCC CTTCCGCTTG CAGCTTCCCT GGGATTTTNG TNTCCTTTTA AAGGCAAACC 240
TCCCAGCTTC TTTAGCCTCT TGGTACCTNA CACTCTCTGT CCNTCGCGTT ATTNATTCTA 300
CACTGCCACT TCTGTAAGAA AAACAGTTTN TCAATAAAAA AAANAGNTGN 350

SEQ ID NO:5823
SEQUENCE LENGTH:163
SEQUENCE TYPE:nucleic acid
TOPOLOGY:linear
CLONE:HUMGS06913
SEQUENCE DESCRIPTION:
GATCTCGTCT CTAAATAAAA TATACAAAAG AGTATTGGGA GGGGGTATGA AGAATGTATA 60
GGGCAATAAT TGAAATCTGT TGAAGCTGGG TACACAAAAG TNCATTATGT TGTTTCTCTA 120
CATTTATATC TATTNTNAGT NNTTTNATAT CTATTTTNAN TTN 163

SEQ ID NO:5824
SEQUENCE LENGTH:180
SEQUENCE TYPE:nucleic acid
TOPOLOGY:linear
CLONE:HUMGS06915
SEQUENCE DESCRIPTION:
GATCAGTGGT TTCCATTGTC AGGACTGGGC ATGGGGAAGT NTGGCTGTAC AAGAGGAGCC 60
CAAGGGAACT CCTTGGTGGT GATGGAACTG CTCTGTATCT CAAATGTCTG TATCTTTAGA 120
AAGAANTCTA CATATGTNAT AACACTGCAT AGAAATATAT ACATGTACAC ACAAGTGAAA 180

SEQ ID NO:5825
SEQUENCE LENGTH:247
SEQUENCE TYPE:nucleic acid
TOPOLOGY:linear
CLONE:HUMGS06916
SEQUENCE DESCRIPTION:
GATCAGTGAA ATCCTAGGGT GCTCTATGAG ATTGTACTAG GCCTATNAAG AGTGGTAAGC 60
CAAATAGGTC TCCATGGGAG ATACATTATG TAAATAAATA AACAATGGTT TGCTGGTTCC 120
TGTTGGTGTC TCCACAAGTA GGTAANCATG TTTAAAGGAA CCCGGGTTCT TAGATTTTNT 180
NAGACTTTTT AANCTCAAGG CTGNGCATAA GTNCTTGAAN ATAAANTGCT AATNCTTAAG 240
TGTCAAA 247

SEQ ID NO:5826
SEQUENCE LENGTH:169
SEQUENCE TYPE:nucleic acid
TOPOLOGY:linear
CLONE:HUMGS06917
SEQUENCE DESCRIPTION:
GATCAAGAAT TGCAAAAATG GAAAAAATTA ATGAAAAGGC ATCTGATAAA TGTGGACGGC 60
TCCAAATCAT GTCCTTAGAA AATCTTTCTA TTGAAAAGGA GACTAAATTG TAATGTGATT 120
CACAATGTAA CAATATAANN CTNNGTTTTT NTATAATTNT NTAANAGTN 169

SEQ ID NO:5827
SEQUENCE LENGTH:268

SEQUENCE TYPE:nucleic acid
TOPOLOGY:linear
CLONE:HUMGS06919
SEQUENCE DESCRIPTION:

```
GATCACCTCC GGGCACAGGA TGCGAGGAAC TGTTGACATT CAGGGATGTG ACCATAGAAT  60
TCTNTCTGGA GGAGTGGGAG TTTCTNAACC CTGCTCAGCA GAGTTTGTAT AGGAAAGTNA 120
TGCTAGAGAA CTACAGAAAC CTGGTCTCTN TGGGTCTTAC TGTTTCTAAG CCAGAACTGA 180
TTAGCCGTCT GGAGCAAAGA CAGGAGCCCT GGAATGTGAA GAGACATGAG ACCATAGCCT 240
GGGCGACAGA GCGAGACTCC GTNTCAAA                                   268
```

SEQ ID NO:5828
SEQUENCE LENGTH:219
SEQUENCE TYPE:nucleic acid
TOPOLOGY:linear
CLONE:HUMGS06920
SEQUENCE DESCRIPTION:

```
GATCTGTGCC CAGAGACGGG ACTGGGAGGG CCCACTTCAG GGTTCTCCTC TCCCCTCTAA  60
GGCCGAAGAA GGGTCCTTCC CTCTCCCCAA GACTTNGTGT CCTTTCCCTC CACTCCTTCC 120
TGCCACCTGC TGCTGCTGCT GCTGCTAATC TTCANGGGCA CTNCTTGCTG NCTTTAAGTC 180
GCTGAGGGAA AAATAAATGA CAAATTGCTT GNGNCCAAA                        219
```

SEQ ID NO:5829
SEQUENCE LENGTH:153
SEQUENCE TYPE:nucleic acid
TOPOLOGY:linear
CLONE:HUMGS06922
SEQUENCE DESCRIPTION:

```
GATCATAAGT NCTTCTATCT GCTGAGCTTT ATTTATTNAT TTTGGACAGA AAGTTTGGTG  60
GGAAGGTTGC AATAAAATCA GAATCTCTCT TGTNTGAATT ATGCAGTTTA ACCCTGTCCA 120
TGGNNNGGCT GTACTCTATT CTNACTGTAT TTN                              153
```

SEQ ID NO:5830
SEQUENCE LENGTH:172
SEQUENCE TYPE:nucleic acid
TOPOLOGY:linear
CLONE:HUMGS06923
SEQUENCE DESCRIPTION:

```
GATCTTGTTT TNATTAAATA TGCCAGTTTG TATTTNCCTG CCTTGGGATT TTTTTGTGTC  60
CGCTGTACAG TATTCTAAGG GAAAAAGAAA AAGAAAGATG TGTAAAGTAA CAGAGAGAGG 120
TGGCTATGGT GTAGAGACCT CTTTCTAATA AAGAAATGAA AATATGTCTA AA         172
```

SEQ ID NO:5831
SEQUENCE LENGTH:96
SEQUENCE TYPE:nucleic acid
TOPOLOGY:linear

CLONE:HUMGS06927
SEQUENCE DESCRIPTION:
GATCCCAGGG AAGACGTCTC TCTCNGCATC CCAAGCCATN CAGCCCTTAT CGCTGTACCC 60
AGTAAACCCT CAATAAATAT CTTTNTCAGC CAGAAA 96

SEQ ID NO:5832
SEQUENCE LENGTH:228
SEQUENCE TYPE:nucleic acid
TOPOLOGY:linear
CLONE:HUMGS06928
SEQUENCE DESCRIPTION:
GATCACTGTA TTAATTTTGG TTTATCTTGG CATATATNGT TCAGTTTGTN TTTATTTTTA 60
ATTTTNCCTT TTTTTCCGAT TAGGCTTTGG TCAGCATTTT NCATTTAAAG AAAAGTAACA 120
CTCCCATCCA CTCATAAGCT TGGTACAAAA ACTTCTCTGG CAGTTACTTT TGAAGCTTCA 180
CTCTGCTTTC TGTATAAAGG GCAGTCTGTG GTCACGCAAG ACTTTAAA 228

SEQ ID NO:5833
SEQUENCE LENGTH:109
SEQUENCE TYPE:nucleic acid
TOPOLOGY:linear
CLONE:HUMGS06929
SEQUENCE DESCRIPTION:
GATCTTTCTG CTTTCGAAGG TATANTGTAT CTATTTCTGT CAGGAATNAT ATTTCCAAAT 60
GAAAATGTAA AGAACATTGG GAAATAATAA ACTTTCCTTT CAAAGTAAA 109

SEQ ID NO:5834
SEQUENCE LENGTH:160
SEQUENCE TYPE:nucleic acid
TOPOLOGY:linear
CLONE:HUMGS06930
SEQUENCE DESCRIPTION:
GATCTGTGTT TGGAAAGAAA AGAAGGAAAC TAAGACGTGC GAGGGNAAGA AAAAGGAAAA 60
CCTAATTAAA AAATATGTAT CCTCTATAAT NAGTTATNAC AGCCATTTGT AATGAATTTG 120
TCGCAAAGAC GTAATAAAAT TAACTGGTAG CACGGNCAAA 160

SEQ ID NO:5835
SEQUENCE LENGTH:77
SEQUENCE TYPE:nucleic acid
TOPOLOGY:linear
CLONE:HUMGS06931
SEQUENCE DESCRIPTION:
GATCGACCGG CACCGCTGTT GCCTCGTAAG CCATAGCGCA TGCGCGCTCT CAGAATAAAC 60
AGGCCCTGCC TGGGAAA 77

SEQ ID NO:5836

SEQUENCE LENGTH:137
SEQUENCE TYPE:nucleic acid
TOPOLOGY:linear
CLONE:HUMGS06932
SEQUENCE DESCRIPTION:
GATCANATAA CAGAATGCAC CAGTCATCAG CTATTCAGTT GGTAAGCTTC CAGGAAAAAG 60
GACAGGCAGA AAGAGTTTNA NACCTGAATA GCTCCCANAT TTCAGTCTTT TCCTGTTTTT 120
GTTAACTTTG GGTTAAA 137

SEQ ID NO:5837
SEQUENCE LENGTH:402
SEQUENCE TYPE:nucleic acid
TOPOLOGY:linear
CLONE:HUMGS06933
SEQUENCE DESCRIPTION:
GATCCAGCTT CATTCAACAT TACTACCAGT TATTTGATAA TNATAGAACC CAACTAGGCG 60
CAATTTACAT TGACGCGTCA TGCCTTACGT GGGAAGGACA ACAGTTCCAG GGGAAAGCTG 120
CCATTGTGGA GAAGTTGTCT AGCCTTCCGT TCCAGAAAAT TCAGCACAGC ATCACCGCGC 180
AGGACCATCA GCCCACTCCA GATAGCTGCA TCATCAGCAT GGTTGTGGGC CAGCTTANGG 240
CGGNTGAAGA CCCCATCATG GGNNTNCGCC AGATGTTCCT ATTAAAGAAC ATCAACGATG 300
NTTGGGTTTG CACCAATGNC ATGGTTCAGG CTCGTCCTGC NCANNTTTGG CTGNCCTNCT 360
TTGCAGNTTG GGNACTNANG NTGTTTGCTT GCTGCCTTTT GN 402

SEQ ID NO:5838
SEQUENCE LENGTH:261
SEQUENCE TYPE:nucleic acid
TOPOLOGY:linear
CLONE:HUMGS06934
SEQUENCE DESCRIPTION:
GATCTTCGTA ACGGCCTCGG CACGCTCCAC ACCCCTGGAG TCTCCCGAGG CCCCCATGTG 60
CTGCTGAGTG GCCAAGATGA TGCCAGGCTG CCCTATACAC TGGGGGATTC TGCCCTGGCC 120
CATGCACACC CGTCTTTCCA TGATGGCAGA GACATCCAGT NAGGACCTGA CCCGTCTCTG 180
TCTGAGGCCG GCTCAGCAGT GCAGCCTGGT CCCTGGGGGC TGGACCCAGG CTCCTAAATA 240
AACCAGCAAC TNCCTCGNAA A 261

SEQ ID NO:5839
SEQUENCE LENGTH:158
SEQUENCE TYPE:nucleic acid
TOPOLOGY:linear
CLONE:HUMGS06935
SEQUENCE DESCRIPTION:
GATCTCGGCT CACTGCAACC TCTGCCTCCC GAGTCCTGGT TCAAACTATT CTCCTGCCTC 60
ANTCTCCTGA GTAGCTGGGA CTACAGNNAC GAGCCACCAC ACCCTNCCCC CGNATCCCCT 120
GCCTCCATCC TAGTAAAGAC TCCTTGCTAT GCTGCAAA 158

SEQ ID NO:5840
SEQUENCE LENGTH:127
SEQUENCE TYPE:nucleic acid
TOPOLOGY:linear
CLONE:HUMGS06936
SEQUENCE DESCRIPTION:
GATCAGTTTT GCCAAGGGGC CAGAATTATT CCTTGTTAGA ATTGCTCCAG TTCAAGTCTG 60
CTGCTTTCCT ACAGTTTTTC AAATTTTATA ATGTATTAAA TACAATAAAC TCTGTTTAAA 120
AAATAAA 127

SEQ ID NO:5841
SEQUENCE LENGTH:232
SEQUENCE TYPE:nucleic acid
TOPOLOGY:linear
CLONE:HUMGS06937
SEQUENCE DESCRIPTION:
GATCAAAGAA AATTTATCTC TTTTTATAAA CTTAAGGACA GTTGCAAAAG GCTTCAAGGA 60
ATTTATCTCA ACATTATTCT TTCTATGTCC TAACTAAATT CCTCAACTGT NATGANTTTT 120
CCANCTACTT CTTGAACAGT GGTCTATNCT GCTACATGAN GATGAATACA AACAANNTTN 180
TTGTATAACC TACTATNCCA GTNCTCCTGT GTCAGTCTTG CCTATGGATN TN 232

SEQ ID NO:5842
SEQUENCE LENGTH:290
SEQUENCE TYPE:nucleic acid
TOPOLOGY:linear
CLONE:HUMGS06938
SEQUENCE DESCRIPTION:
GATCTCCAGN GCGCAGTGGG TTGGAGGAAG TACTACGTGA CAGCTGGGGG AGCCTAGCCC 60
TTGTNTGGGN ACTCACACAT CCACTCTAAC CCTTCTNACC ACAGGTCCGG CCGCCCCATT 120
AGCTTCATNG TGGTTTTCAT CACCCCCAAC CCCCTGAGCA AGATTTCCTG GGTCAACAGG 180
TTACATTTGG CCANAATCGG ACTCCGTGAG TATAGCCCCA GGGAGAGTGC CTNAGGGGGC 240
AGNGGTGTGG AACGNGNAGG TTTNGGGTGA GTNNGGGTAC AGAATGTNGN 290

SEQ ID NO:5843
SEQUENCE LENGTH:198
SEQUENCE TYPE:nucleic acid
TOPOLOGY:linear
CLONE:HUMGS06939
SEQUENCE DESCRIPTION:
GATCTTGTGA CATTCCTTCT CCTGGACAAT NAGTCTCAGG AGCTCCTCAC CAAGCACCTT 60
GTTACCCCCA CCCCTGCCCA CAAGAGAAAA CGCACGTTAA CTGTAATTTN TCCACTACCT 120
ACCCAAATCC TATAAAACTG CCCCACCCCA TCTCCTTTTG CTGACTCCAG GTGATTAAAN 180
AACTATTGCT CAGGGAAA 198

SEQ ID NO:5844

SEQUENCE LENGTH:255
SEQUENCE TYPE:nucleic acid
TOPOLOGY:linear
CLONE:HUMGS06941
SEQUENCE DESCRIPTION:
GATCTTTTTT CTAACACATA TTTGAACTGA ATAACAGACT TAAAGAAAGC CTTTGTTCAC 60
ATTGCTATTT ACTNTTGTGT TTGGGGGAAA ATACGAGGGA TTGATTTTAA ATAAAAAACA 120
TTCCNTCTTT CATTNAATAT CAGTATCAAA AGAGGNNGAC AAACATCTAT CTTTCTCATC 180
TNTATTNAAG TNCCTTTTTG TANTGTAGTA TCAANGTTTT TTAGGTGNTG CAAGGTTTTA 240
CAANTCATTT GTGGN 255

SEQ ID NO:5845
SEQUENCE LENGTH:318
SEQUENCE TYPE:nucleic acid
TOPOLOGY:linear
CLONE:HUMGS06942
SEQUENCE DESCRIPTION:
GATCCCTCAG AAGACTTAAC TTGGCTGGAG TGGGAGGAAC TGAAAATACC ACTCCATGGT 60
ATACCCATAT ATCCAAATCG TAGAGAACGA GAAGCTATGA TTTTATCATC TTATGCTGGA 120
ATCTTAATGA ACAGTATCCC GATTGAGGAA GTCTTTAAAA TTTATGGGGC TGATTCTNCT 180
GNCGATTCTG GTACCATCAA GTTTCCCCGA GTTTCATCTN TCCGNCTCTC CTTNCACCNC 240
TNTTGCCATG TTAGCAGNAN CNANAGCAGC AGCATACGGC CGCAAACNGG GTGTCNAGTC 300
AAGNANGGTT GCAGCAGN 318

SEQ ID NO:5846
SEQUENCE LENGTH:54
SEQUENCE TYPE:nucleic acid
TOPOLOGY:linear
CLONE:HUMGS06943
SEQUENCE DESCRIPTION:
GATCGGAGGT TGGGTTCCTG ATTAAACTTC ACTGTGTGTT TTCTATNTCG GAAA 54

SEQ ID NO:5847
SEQUENCE LENGTH:127
SEQUENCE TYPE:nucleic acid
TOPOLOGY:linear
CLONE:HUMGS06945
SEQUENCE DESCRIPTION:
GATCAGAGAN TTACCACCCC TGCTTNGAAG CAGTGTTGGT GTATTCCCAC TAAAATAAAC 60
CAACTGGTAT AACATTCCCT GGAGCTTAAT AAGCACTCAA TAAATATTTG TNGANTAAAN 120
ANAGAAN 127

SEQ ID NO:5848
SEQUENCE LENGTH:212
SEQUENCE TYPE:nucleic acid

TOPOLOGY:linear
CLONE:HUMGS06946
SEQUENCE DESCRIPTION:
GATCTGCAGA TTCCAAATGG GAATAAGCTC TATCATATTC TGAAACAAGA ATTAGAATGN 60
CTTGAGAACG GGCAAATAAC AAAGCAAACC AATATAATTA TATGGTCATT CTGACCCCAG 120
CTCTTATACA NNTTATACAT GTATTTTTGT GTATGTTTGT GAGAGTTGTA TGTATGTGAA 180
TGNGTGTGAG TGTGTATTCA CATACACATA TN 212

SEQ ID NO:5849
SEQUENCE LENGTH:213
SEQUENCE TYPE:nucleic acid
TOPOLOGY:linear
CLONE:HUMGS06947
SEQUENCE DESCRIPTION:
GATCATAAGG TGAAGTCCCA CAATAGGCCG TCTGCAAGCT GAGAAGCAGN GNAAGCCAGT 60
CTGAGTCCCA AAACCCTAAC AGTAATCCTT TNCTAACGTC AGGNTGCGTA TCGACCTGTG 120
TGTGCACATT TGCTGTGCAG AGTGTGCACT GCTGAGGAGA ATGGTGCCCA AGAAGGGCAC 180
TGTTTGNCCC AAANTNTTNC AANTAANCAT TGN 213

SEQ ID NO:5850
SEQUENCE LENGTH:65
SEQUENCE TYPE:nucleic acid
TOPOLOGY:linear
CLONE:HUMGS06948
SEQUENCE DESCRIPTION:
GATCATCTTG AATTACTGTG GGATGTANGT TTCAAAATTT TCAAATAAAG CCTTTGCAAG 60
ATAAA 65

SEQ ID NO:5851
SEQUENCE LENGTH:55
SEQUENCE TYPE:nucleic acid
TOPOLOGY:linear
CLONE:HUMGS06949
SEQUENCE DESCRIPTION:
GATCACATAC TGCAGTCCAG CCTAGGCAAC GGNGCAAGAC GTCATCTCAA GGAAA 55

SEQ ID NO:5852
SEQUENCE LENGTH:186
SEQUENCE TYPE:nucleic acid
TOPOLOGY:linear
CLONE:HUMGS06951
SEQUENCE DESCRIPTION:
GATCTCCCCA AATCCATTGN TNTCACCAGG CCCTCCCAGA ACCTCCTCAG TTCCTTCACA 60
GTGCAACCCT NTGTACTTGG CCCGCAACCC ANTAGTATTG TGCCTCACTT CACCTTCCAT 120
GGGCAACTGC CCTCCCTTCT GGACATAAAA CCTNATATNT TAAATAAAGT TGAANTATGN 180

NAAGAN 186

SEQ ID NO:5853
SEQUENCE LENGTH:102
SEQUENCE TYPE:nucleic acid
TOPOLOGY:linear
CLONE:HUMGS06952
SEQUENCE DESCRIPTION:
GATCAACTGA AAAAAGCATG ACTTTTGAAA TCTCTGAATG CCTTGGTTCT NAGTATTATC 60
ATNCTTNATT GAATTTATTT CTNATTAAAN TATGTAGTTT TN 102

SEQ ID NO:5854
SEQUENCE LENGTH:97
SEQUENCE TYPE:nucleic acid
TOPOLOGY:linear
CLONE:HUMGS06953
SEQUENCE DESCRIPTION:
GATCATGTGG AGTGAAAGGC AAATCTTACT GCTTAATGTA TAAACTCTCA CCACAGGAAG 60
CATCGCTGTT TCCAATAAAT ATTGCTGAAG ACAGAAA 97

SEQ ID NO:5855
SEQUENCE LENGTH:308
SEQUENCE TYPE:nucleic acid
TOPOLOGY:linear
CLONE:HUMGS06954
SEQUENCE DESCRIPTION:
GATCTGTCCA GTTTGTGTAT GAAATGGATT TGATAAAGTT TTTGCTAGTT ATTTACTACA 60
TTTTGGGATT AATAAGTGAT TTANATGCAT ATNTTCCTGT AAATCTACAG TTTTTTGTAC 120
AAGATATTCT ACAAGTNATG ANGCTAAGGG AAGAAAATGC CAAAGATACC TCTAGTTATG 180
TTGANCACAG CCAGCACAGT TTCGACAGGT CAAGGAAGAG CTGTTTCAGT AAAGANTGAN 240
GTGAAAACNC TTATTTNGGA AANTGTTTCT CANCANTAAN CTGTATAGTT GTTTCTCTNA 300
NACCCCCN 308

SEQ ID NO:5856
SEQUENCE LENGTH:128
SEQUENCE TYPE:nucleic acid
TOPOLOGY:linear
CLONE:HUMGS06955
SEQUENCE DESCRIPTION:
GATCCCAAGT GTTTAGAAAA CTAAGTAAAC TTTGTTGTCA AGCAATTTGT CCATGTGGAA 60
AGAATTTAAA GAGAAGCTGA ACAATGGAAT AAACAAATAA AGCATGTTTT TNNATCTTTC 120
CCATTAAA 128

SEQ ID NO:5857
SEQUENCE LENGTH:143

SEQUENCE TYPE:nucleic acid
TOPOLOGY:linear
CLONE:HUMGS06956
SEQUENCE DESCRIPTION:
GATCTTAAAA ACTGCAGTAT TCCCCCATTT TNTAATGAGA GTGTGGGGCT GGCAGGGNTT 60
GGTTGGAGGG AGGAGAGAAG ACAGAGGAGC ACTTAAGGTG CAAAGCAGCC TATTTTTCCT 120
TCAATAAAAA TTGTTAAGGG AAA 143

SEQ ID NO:5858
SEQUENCE LENGTH:235
SEQUENCE TYPE:nucleic acid
TOPOLOGY:linear
CLONE:HUMGS06957
SEQUENCE DESCRIPTION:
GATCCGTGGA AATAGCCTGG CTCCCTCTTA CCCAGTAATG AGGGGCAGGG AAGGGAACTG 60
GGAGGCAGCC GTTTAGTCCT CCCTGCCCTG CCCACTGCCT GGATGGGGCG ATGCCACNNN 120
GTCATCCTTC ACCCAGCTCT GGCCTCTGGG TCCCACCACC NAGCCCCCCG TGTNAGAACA 180
ATCTTTGCTC TGTACAATCG GGCTCTTTAC AATAAAACCT TCCTGCTTNC ACAAA 235

SEQ ID NO:5859
SEQUENCE LENGTH:258
SEQUENCE TYPE:nucleic acid
TOPOLOGY:linear
CLONE:HUMGS06959
SEQUENCE DESCRIPTION:
GATCCAGTAC TATCAGAATG ACATCCCCTA CTGAGGAGGG GACCTTCGAG GGCCTCTGCC 60
CCATGTGCCC TCAAAGCTGT CCCACAATCA TGGAGCCCTG CNACCTNCCT GCCCTGNCGN 120
CACATGCAGT NGAGGAGAGG CCTGTGGCCA AAGAACCTGG TAGCGGCTCC TNGGGCAGCA 180
CGTGGNTGGC GCACTTTGGT AACGCCATGG GANTGCAGCG NCCANTCGAA TGGGATNGGT 240
GCTGTTNTAT GCACAGGN 258

SEQ ID NO:5860
SEQUENCE LENGTH:309
SEQUENCE TYPE:nucleic acid
TOPOLOGY:linear
CLONE:HUMGS06960
SEQUENCE DESCRIPTION:
GATCAGATTA CCAGGAACAT AGGAGTGGAT TCCTGCCCCA ACCAAACCGC ATTCGTGTGG 60
ATTTTTTTAT TCAACTTAAT TGGCTATTCC AAAGATTTTT TTTTCCCTAT TTTTAACGAT 120
TGGAGCCCTT AAGATGCACG ATGGAATTGT GTTTTGCGTT TTTNGGNAAA NGGGAGCAAA 180
GCGGGGNCCT GGNGATAAAC GNTTGGAGCA ATCTCCTTNG GNAGGGATTT CAGCANGGGG 240
TTAGNTNGGT GAAACATTTT AAAGGGGGGA AAGGGGGGGG GTTTTTTTTT NAAANTGGGN 300
TAAATNCGN 309

SEQ ID NO:5861

SEQUENCE LENGTH:179
SEQUENCE TYPE:nucleic acid
TOPOLOGY:linear
CLONE:HUMGS06961
SEQUENCE DESCRIPTION:
GATCATGTGG ACCAGAGCAA ANNAAAGTTC AGTTTGTGTC ACAATTCATT GCCAGACTTC 60
ATTGGAATGC TTTGTTTGAT GATGTATGTN CATTCTCAGC TTTATTTTCA GATGCTTAAC 120
TGGGCAATGA AGTCTAACTT CAGGTTGAAC TNTCTCCNGN NTAANCTCAG GCTAAATGN 179

SEQ ID NO:5862
SEQUENCE LENGTH:341
SEQUENCE TYPE:nucleic acid
TOPOLOGY:linear
CLONE:HUMGS06963
SEQUENCE DESCRIPTION:
GATCGTGGTG GTGGGGACTG GAGACCGGAC CGAGAGGCTG CAGTCCCAGG TGCTTCAAGC 60
CATGAGGCAG CGGGGCATTG CTGTGGAAGT GCAGGACACG CCCAATGCCT GTGCCACCTT 120
CAACTTCCTG TGTCATGAAG GCCGAGTAAC TGGAGCTGCT CTCATCCCTC CACCAGGAGG 180
GACTTCACTT ACATCTTTGG GCCAAGCTGC TCAATGAACC GNCAGGAACT GACCTGCTGA 240
CTGCACTCTG CCAGGCTTCC NAATTGCTTT CANTCTTATC TACCCTTTTG GGACTTATNT 300
NGGCTTATCA ACATAATAAT TTGATACAAC TNNTTCCCAA A 341

SEQ ID NO:5863
SEQUENCE LENGTH:60
SEQUENCE TYPE:nucleic acid
TOPOLOGY:linear
CLONE:HUMGS06964
SEQUENCE DESCRIPTION:
GATCTGGTGA AAATAGGATT ACATTGGAGC CAATTGAATA AATTTATTCT TTCAATCAAA 60

SEQ ID NO:5864
SEQUENCE LENGTH:359
SEQUENCE TYPE:nucleic acid
TOPOLOGY:linear
CLONE:HUMGS06965
SEQUENCE DESCRIPTION:
GATCTGGGAG GTGGAAGCCA GGCCAGAGGA CTTGGGGAAA ATAAGATGGA GGAAGGAAAA 60
AGGGAGAAGC TGAGCCACAG CTTAACTCCT ACAGAGTGAA ATNAAAACGG GCTGAAAATA 120
CCACCCCAGG AGAGGACCTC GCCCCAAGCA AGCCAGTGAG CAGCCCTGCC AGACTACTGC 180
CAGACTGAGA AACCCAGAAG CTGGTAGTCA TGTGGGCTTG CNTTCTNTGC CAAACGACTG 240
GGAAACCAAA ATNAGCCCAC CTTGTGTTCT TCCTAGCTCC ACCCTCCCCG TGCTGCTGTG 300
TTCTGCTCCT TCCNCACGGT TTCCCTGCTA TAGTTCCCAG CTGCTTGTAA CGGGGCCNN 359

SEQ ID NO:5865
SEQUENCE LENGTH:170

SEQUENCE TYPE:nucleic acid
TOPOLOGY:linear
CLONE:HUMGS06966
SEQUENCE DESCRIPTION:
GATCATGGCT AACGGGCACA GGCTTTCTTT TTTGGGTGAT GAAAATATTC TAAAATAACT 60
GTAAAAATAT TGCACAACTC TGTNAACATG TGAAAACCCA CTGAACTGTA TGCTCTAACT 120
GTATGAATTG TATGGTATTT AAATTTNACC TCAATAAAGC TGTTATCAAA 170

SEQ ID NO:5866
SEQUENCE LENGTH:187
SEQUENCE TYPE:nucleic acid
TOPOLOGY:linear
CLONE:HUMGS06967
SEQUENCE DESCRIPTION:
GATCAGAGCA AGAGAACTTC CAGAGATAAT GGATAGAATG AAAGAATATA TATCTATATA 60
TATTTAACAT GTTGTTTAGT AATCCCTATT TTAATTCAAT TCTCCATCTG TAAAATATTA 120
GCTAACTGAA TTATTTCTGT GTTCTACTCT GGGCTCTCAC ATTAAAACTC TGTTCAAAAA 180
GTTTAAA 187

SEQ ID NO:5867
SEQUENCE LENGTH:57
SEQUENCE TYPE:nucleic acid
TOPOLOGY:linear
CLONE:HUMGS06969
SEQUENCE DESCRIPTION:
GATCTTGGAC TTCCCAGCCT CCAAAACTGT GAGAAATAAA CTCCTGTTGT TTGTAAA 57

SEQ ID NO:5868
SEQUENCE LENGTH:289
SEQUENCE TYPE:nucleic acid
TOPOLOGY:linear
CLONE:HUMGS06971
SEQUENCE DESCRIPTION:
GATCATGGTC AGCTCACACC CTAATAAGTC CACATCTTCT CAGTGTTTTA GCTGTTTTTT 60
TCATTAGGNT TCCTTNTATT CTGTACCTTG CAGCCATGAC CAGTTCTGGC CAGGAGCTGG 120
AGGAGCAGGC AGTGNGTGGG AGCTCCTNTT AGGTAGANTT TAACATGACT TCTGCCCCAG 180
CTTNATCTGT CACACANGTN TGTGGCACCN CTAGTGCTAC TGCTAGGATA TCACTTACTC 240
AGTTAGATTT TTCCNAAAAA NTAAGCTTTT AATTNATTTC TTTGGTGTN 289

SEQ ID NO:5869
SEQUENCE LENGTH:346
SEQUENCE TYPE:nucleic acid
TOPOLOGY:linear
CLONE:HUMGS06972
SEQUENCE DESCRIPTION:

GATCTCCTCT TCCACCTCTT CCTGGTTCCT TTGCGGGGAA AATTGCACTA AAACAGAACC 60
TTTTCTTAAT CCATGTTGGA AGGAAGCAAC AGTGAACTCT ACCTGTTCTG GAGTTCTCCT 120
GGGTCTGCAG AAGGTTGGGA ATTTAGAAAA TAAGGCTGTT CTTTCATATT TTAATTTAAT 180
CTCTGTCAAT GGCCATCCCT CCCACAAAAA AACGTGGGTT AAGAGAACTT GCAGACTGGA 240
TATGCAAGCA AACGGGCANC TCTGGAGAAA ANTANGGNAN GGAATGCTGA CTTTCTCTTT 300
CTNTCTCTTT GTCCCCACAC CNNTTCCCAN CCCAATTACT GGGGTN 346

SEQ ID NO:5870
SEQUENCE LENGTH:187
SEQUENCE TYPE:nucleic acid
TOPOLOGY:linear
CLONE:HUMGS06973
SEQUENCE DESCRIPTION:
GATCCCGACC CTCACCAGTC CCATTTGCCT CCCTCCAGCT CTGCTTAGGC ATTTTGCCCC 60
TCACCCCAAT GTTCCACACC ATCGACAACC AAGGGGTGAG GTGGGGACAG GCCTCAGCAG 120
GGAATGGGGC GTATATGTTA GTGTTGCTGC AACAATAAAG CCTGTTGCAT CTCTCATGCC 180
AGTGAAA 187

SEQ ID NO:5871
SEQUENCE LENGTH:135
SEQUENCE TYPE:nucleic acid
TOPOLOGY:linear
CLONE:HUMGS06975
SEQUENCE DESCRIPTION:
GATCTNGGAC TTCCAGAAAA CAGAACTGTG AAAAGNCAAG TTTCTGTNGT TTAAACCACC 60
CAATCTGAAG TATTTNGTTA TGGCAGTCCT NGGCAGACTA ATACAATATG CAACACATTT 120
ACAATANATA TACAN 135

SEQ ID NO:5872
SEQUENCE LENGTH:138
SEQUENCE TYPE:nucleic acid
TOPOLOGY:linear
CLONE:HUMGS06977
SEQUENCE DESCRIPTION:
GATCAAATAA TGATGTTAAA TTCTTAAATC ATATTTGCTA TGCAGCTGAA GATGATATTT 60
TGATTTGTAT TTTGGGGGTA CCTGTGTTGA GTTGATAAAC ATTTCCATCT TCATTAAAAC 120
TGCTTCCAAA CTAGTAAA 138

SEQ ID NO:5873
SEQUENCE LENGTH:189
SEQUENCE TYPE:nucleic acid
TOPOLOGY:linear
CLONE:HUMGS06978
SEQUENCE DESCRIPTION:
GATCGGCGGG GCACAGTGGC TCGTGCCTGT AATCCCAGCA CTTTGGGAGG CCTAAGCAGG 60

CTGGTCGCTT GANCCCAAGA GTTCGAGACC AGCCTGGGCA ATACAGCGAG ACCCCATCTC 120
AATTTATATT ATTTAAAAAN TAATTAATGT AAGAGNAATT AAAACAGATT TTTAAANNGG 180
NNAAANNAN 189

SEQ ID NO:5874
SEQUENCE LENGTH:98
SEQUENCE TYPE:nucleic acid
TOPOLOGY:linear
CLONE:HUMGS06979
SEQUENCE DESCRIPTION:
GATCTGACTC TTCATTTNTA AGTTCCTTGT TACATCATGG TCATTTNCTA GTTTTTNACC 60
AGACTCCCAT CTCACAATAA AATGCATCAA CAAGCAAA 98

SEQ ID NO:5875
SEQUENCE LENGTH:182
SEQUENCE TYPE:nucleic acid
TOPOLOGY:linear
CLONE:HUMGS06981
SEQUENCE DESCRIPTION:
GATCTGCCGG CTCCCGCCCA GCCTGTGTNG AAAGAAGGCC CACGGGCACT AGGGGAGCCG 60
AATTCTACAA TCCCGCTGGG GCGNCCGGGG CGGGAGAGAA AGCTGGTGCT GCAGTGGTGG 120
CCCTNGGGGG CCATTCGATT CGCCTCAGTT GCTGCTGTAA TAAAAGTCTA CTTTTTGCTA 180
AA 182

SEQ ID NO:5876
SEQUENCE LENGTH:87
SEQUENCE TYPE:nucleic acid
TOPOLOGY:linear
CLONE:HUMGS06982
SEQUENCE DESCRIPTION:
GATCTTGTTT TNCCTCATCT GTAAAAGNAG GTTAACAAAG CTTTTCTGCC CACTTCTTGG 60
GGAGAAGGGA ATAACATAAT TGGTAAA 87

SEQ ID NO:5877
SEQUENCE LENGTH:315
SEQUENCE TYPE:nucleic acid
TOPOLOGY:linear
CLONE:HUMGS06983
SEQUENCE DESCRIPTION:
GATCTTTGGC AGCGCATATG GCCTCTTTGG GGTCATCGTC GCAATTNTTC AGACCTCCAG 60
AGTGAAGATG GGTGACTAGA TGATATGTGT GGGTNGGGCC GNGCCTCACT TTTATNTATT 120
GCTGGTTTTC CTGGGACAGC TGGAGCTGTG TCCTTTAGCC TTTCAGAGGC TTNGTGTTCA 180
GGGCCCTCCC TGCACTCCCC TCTTGCTGCG TGTTGATTTG GAGGCACTGC AGTCCAGGNN 240
NNGTCCTCAG TGCGGGGAGC AGGCTGCTGC TGCTGACTCT GTGCAGCTGN GCACCTGTTN 300
NCNNCANCTG CACCN 315

SEQ ID NO:5878
SEQUENCE LENGTH:190
SEQUENCE TYPE:nucleic acid
TOPOLOGY:linear
CLONE:HUMGS06984
SEQUENCE DESCRIPTION:
GATCTNTTTT TTTTTGGTAG TTCCTCAGCT ATTCAGGAAG GTGTATTTNT GCCAGTAATT 60
ATCCCTATGC TAATACTTTT TAAAACATTA CTAGTGAGTG CCCTAAAACA TTACTAGTGA 120
GTGCCCTTTT NNTTTACAGA AGAAATTTGA TTGTTGTGAG CGATAATAAA ATTAACGTGG 180
ANCTCTTAAA 190

SEQ ID NO:5879
SEQUENCE LENGTH:322
SEQUENCE TYPE:nucleic acid
TOPOLOGY:linear
CLONE:HUMGS06985
SEQUENCE DESCRIPTION:
GATCTCTATT ATNAGGCTAG ATGTATAGCC TCTACTCCCC CAGCTTCTTG CTCTTGACCC 60
TGCACTGTAA GTTGCCCTTC TATTAGCAGC CAAGGAAAAG GGAAACATGA GCTTATCCAG 120
AACGGTGGCA GAGTCTCCTT GGCAATCAAC CAACGTTGCT ATGAAATATG CCTCACACTG 180
TATAGCTCAT TATAGGACGT CAGGTTTNTT GAAAAAAGTG GGCAAGNCAT GATTAATGAA 240
TCAGAATCCT GTTTCATTGG TGACTTGGGT GGGGGCTNTT TGAATTTTAA AAANTNNNTNG 300
TNAGNTGTAG NNNGNGTGNT NN 322

SEQ ID NO:5880
SEQUENCE LENGTH:247
SEQUENCE TYPE:nucleic acid
TOPOLOGY:linear
CLONE:HUMGS06986
SEQUENCE DESCRIPTION:
GATCATGGAA TACAAGATAA TTTCTCAGGA ATCAGATGGT CTTATTCCAC ACCTGCAGGA 60
ATGTCAGCAT TGCAACCACC ATGGCAAATA TTGATTTAAT AANCAATTAC TATTCCCAGA 120
TAAGTGTCAA ANTCTTAGAN GAAATTGTCA TATCTTAGAC AATTTAGANG TGATGGAGGG 180
TGGATGATGA GAAATTATCG CACCACTGCA CTCCAGCCTG GGCGACAGAG CAAGACTCTG 240
TCTCAAA 247

SEQ ID NO:5881
SEQUENCE LENGTH:51
SEQUENCE TYPE:nucleic acid
TOPOLOGY:linear
CLONE:HUMGS06987
SEQUENCE DESCRIPTION:
GATCCACAGT GTTGGGCACA ACTTTGACGT TTCTTAAAAA AAAAATTTAA A 51

SEQ ID NO:5882
SEQUENCE LENGTH:276
SEQUENCE TYPE:nucleic acid
TOPOLOGY:linear
CLONE:HUMGS06988
SEQUENCE DESCRIPTION:
GATCCTTGAT TGGTGAAAGG ATACACAAAA TCAGGCTGCT TCAGAGGAAG TCATCTGCTC 60
ACAGGAATCA GGGTGAAGGC CAAGGACGGC ACTCAAGAAT TGNCCTGTCT CTAAAAAAAA 120
AGCATCTGCA ACGAACTTCC TTTAATTTTT GAGTTCAGTG TCTGTGATAA TTAAGAAATG 180
GCANTACCAT GTATTTNCCC CAGAAGTTAA GANATATTGC TGNACGGACC ANTAAAAGTA 240
ATTNCAGCTC CCTCAAGNAN NATGNGGTGN GGGNGN 276

SEQ ID NO:5883
SEQUENCE LENGTH:54
SEQUENCE TYPE:nucleic acid
TOPOLOGY:linear
CLONE:HUMGS06989
SEQUENCE DESCRIPTION:
GATCGCGCCA CTTCTCTCTA GCCCAGGCAA CAGAAAGAGG CCCCATCTCA GAAA 54

SEQ ID NO:5884
SEQUENCE LENGTH:72
SEQUENCE TYPE:nucleic acid
TOPOLOGY:linear
CLONE:HUMGS06990
SEQUENCE DESCRIPTION:
GATCCTTAAT TGCTACCACA TTTCAAACAA GAACTATGAC CAATTAAACT TTACTGTTGT 60
TGAACTGCCA AA 72

SEQ ID NO:5885
SEQUENCE LENGTH:267
SEQUENCE TYPE:nucleic acid
TOPOLOGY:linear
CLONE:HUMGS06991
SEQUENCE DESCRIPTION:
GATCCNTTGG GAATCCAGTT GAAGTTCCCA AATACTTTAT AAGAGTTTAT CAGACATCTC 60
TAATTTGGCC ATGTCCAGTT TATACAGTTT ACAAAATATA GCAGATGCAA GATTATGGGG 120
GAAATCCTAT ATTCAGAGTA CTCTATAAAT NTTNGTGTAT GTGTGTATGT GCGTGTGATT 180
TACCAGAGAA CTACTNAAAA NAACCAACTG ATTNTTTNAA TCCTANTGTG TAGGTTAAAG 240
TGTCATGCCN TNNACCANTN CTAATGN 267

SEQ ID NO:5886
SEQUENCE LENGTH:164
SEQUENCE TYPE:nucleic acid
TOPOLOGY:linear

CLONE:HUMGS06992
SEQUENCE DESCRIPTION:
GATCATAAAA ATAGCAGNGC ATGTTNCCTG ATGATTTTAC AGTGCATATT GTCATGGCCA 60
GGGCTTAGAG GTAGGGGAAA AACATGTGGA CAGCTTTGTG ACAAGAGTCT CTCTTATAGT 120
GTTTCTTTCT AAAGTAACTT TTTCTGAGTA TGAATGTNNN NNNN 164

SEQ ID NO:5887
SEQUENCE LENGTH:341
SEQUENCE TYPE:nucleic acid
TOPOLOGY:linear
CLONE:HUMGS06993
SEQUENCE DESCRIPTION:
GATCCATGCA AATTGTCGCA TCCGACGGGT TAACTTCTCA GACAGACTCT ACTCAGANGA 60
TGAGCTGCCG GCAGAGTTCA AACTGTATCT CCCAGTTCAG AACAAGGCAA AGCAATAACT 120
GGAATTGTGA CTCGAGGGAT AGACCCCTGG ATGTGACTCT NCTTTTTAAA AGGAAACTAT 180
GTGGAGGACG ATGCAAAANC ATATTTATCT TAGTTTGCTC TGCTGTAGTT CTGTTATTTA 240
TACTTGGTGT TGCTTGTCAT GGACACNGGT GAACATGCCG TAACTCTGTG ACTGCATTGT 300
AAGTGCAGTG GGGGTAAGCA GTCCTGTGGC GTGGCGCATT N 341

SEQ ID NO:5888
SEQUENCE LENGTH:256
SEQUENCE TYPE:nucleic acid
TOPOLOGY:linear
CLONE:HUMGS06994
SEQUENCE DESCRIPTION:
GATCCTGGAC TGGGTACCTT ACATCAATGG CAAGTTTAAG AAGGNTAATT AATTACACAA 60
ACCCTTCACA GACTGCTCTG GTGCCTGGTG GTGCTAGCTC CTCCCACCTC AGCACCTGCT 120
GCATCTGGAG CAGCCCAAGC CTCAGGATGG NCAAGNGGAA ACCCACAGCT CAGCTTCAGG 180
CTTCTTATGT TTCTGAAAAC AGCTTGGNTA TTTTAATGCA CGTTGCATTA AACCTCACTG 240
AANCATAANG GGGAAA 256

SEQ ID NO:5889
SEQUENCE LENGTH:204
SEQUENCE TYPE:nucleic acid
TOPOLOGY:linear
CLONE:HUMGS06995
SEQUENCE DESCRIPTION:
GATCTTTGTA GGACTATATG TGTCAGTAAT NATGAGCTTC AATCCTGACA TTAATAACTT 60
ACTGTNGAGC AGAGAAGAGT TGCTTGTAAA CCTTGTGCTT TTATATACTA NTTTAAAANC 120
TTAAAGTGAT AGTGAGCTAT GAAGTACCAA TCATGTATGA TATGTGGATA CAAAGCACAG 180
TATTATTAAA GTGTNATGTG GAAA 204

SEQ ID NO:5890
SEQUENCE LENGTH:173
SEQUENCE TYPE:nucleic acid

TOPOLOGY:linear
CLONE:HUMGS06996
SEQUENCE DESCRIPTION:
GATCAGCAAT AAAAANAAAC CTACAAAAAA NCCTATATTC NTTAATTNTT GCTTTTACGG 60
TGATATAGTG CATGCAAACC AGGAGCATTT TGTGTCTTAA GAAAAATAAT CTTAGAACAG 120
ATGGCTGTGA AAATTACACC CATGCACAGN ACANGCCACA GGAATAATNG GNN 173

SEQ ID NO:5891
SEQUENCE LENGTH:156
SEQUENCE TYPE:nucleic acid
TOPOLOGY:linear
CLONE:HUMGS06997
SEQUENCE DESCRIPTION:
GATCAAATAT TGGTTGAATG CCTATGTATG TCAGGCCCTG TGCTGAGCCA TGAGGATTAA 60
AAAGATGAAT AANCATATCT TGTTTAGGAA ATGGATGTAT AAAAAAATCA AGTGCAATAA 120
AGTGTGTGTC CAAAAGCTGA CACAATGGAA AGGAAA 156

SEQ ID NO:5892
SEQUENCE LENGTH:100
SEQUENCE TYPE:nucleic acid
TOPOLOGY:linear
CLONE:HUMGS06998
SEQUENCE DESCRIPTION:
GATCGCTTGA ACCTGGGAGG CAGAATTTGC AATNAGCTGA GATTGCACCN TTGCACTCCN 60
GCCTGGGTGA CAGAGTGACA CTCTGTTTGA AACAAACAAA 100

SEQ ID NO:5893
SEQUENCE LENGTH:65
SEQUENCE TYPE:nucleic acid
TOPOLOGY:linear
CLONE:HUMGS06999
SEQUENCE DESCRIPTION:
GATCACTTGA GCTCAGGAGT CCTAAACCAG CCTGGGCAAC ATAGTGATAC CTTGTCTCTA 60
CCAAA 65

SEQ ID NO:5894
SEQUENCE LENGTH:94
SEQUENCE TYPE:nucleic acid
TOPOLOGY:linear
CLONE:HUMGS07000
SEQUENCE DESCRIPTION:
GATCTTGAAT AATTCATAAT TCTTCCACAG TAGAAATAAA ATAATTNCTG GCTAGTTATT 60
TTCTTAAAAA TAAAAAAAAT TTGAAAACCT CAAA 94

SEQ ID NO:5895

SEQUENCE LENGTH:89
SEQUENCE TYPE:nucleic acid
TOPOLOGY:linear
CLONE:HUMGS07001
SEQUENCE DESCRIPTION:
GATCCCTCTT TTCCAGAAAA TTCTGTGGAA TNTTTCTGTA GGACTTTGTT CTCCACAAGC 60
TTGAATTAAA GCAGGATTCA GTTTGCAAA 89

SEQ ID NO:5896
SEQUENCE LENGTH:196
SEQUENCE TYPE:nucleic acid
TOPOLOGY:linear
CLONE:HUMGS07002
SEQUENCE DESCRIPTION:
GATCAGCTGA AGTGTGTTTT AGACCCAAAC CATCTGGCCC CTTCGTTTTG CTCAGAGGAA 60
GTAAATNTNC ACTTAAATGA AATTGAAAAC GCCATGTGGC ACCACAAAAG AGCTCTCTGT 120
ACTTNCCCCA TGCTGCCTCA AAAGTNCTGT AAGTTTCGGG GTCAGTGTCC CACCCTTCAC 180
TTCCCGANNN NNGGGN 196

SEQ ID NO:5897
SEQUENCE LENGTH:56
SEQUENCE TYPE:nucleic acid
TOPOLOGY:linear
CLONE:HUMGS07003
SEQUENCE DESCRIPTION:
GATCCCAGTT TGCCTTGCCA AATAAACTCT GTTTATGTGA ATTTATTAAA CGCAAA 56

SEQ ID NO:5898
SEQUENCE LENGTH:294
SEQUENCE TYPE:nucleic acid
TOPOLOGY:linear
CLONE:HUMGS07004
SEQUENCE DESCRIPTION:
GATCCCAGCT TTTTTCCCTC TCCCACAAAA CCAGGTAGTG AAGTTATATT ACCAGTTACA 60
GCAAAATACT TTGTGTTTCA CAAGCAACAA TAAATGTAGA TTCTTTATAC TGAAGCTATT 120
GACTTGTAGT GTGTTGGTGA AATGCATGCA GGAAAATNCT GTTACCATAA AGANCGGTAA 180
ACCACATTAC AATCAAGCCA AAAGAATAAA GGTTTCGCTT TTNTNTTTGT ATTTNATTGT 240
TGTCTTTGTT TCTATCTNNG ANATGCCATT TAAAGGTAGA TTTCTATCAT GTAN 294

SEQ ID NO:5899
SEQUENCE LENGTH:113
SEQUENCE TYPE:nucleic acid
TOPOLOGY:linear
CLONE:HUMGS07005
SEQUENCE DESCRIPTION:

GATCTCCCTA CTCCATTNNN CCCTTTAATT NAAGTGGCCA CATGTATATG TCTTCCCTGC 60
TGTGTTAGGA AAATGGGGGC TGGATATCCC AAGAATCAGA GGTTATATAN AAN 113

SEQ ID NO:5900
SEQUENCE LENGTH:171
SEQUENCE TYPE:nucleic acid
TOPOLOGY:linear
CLONE:HUMGS07006
SEQUENCE DESCRIPTION:
GATCGCTTTT GTGACTGTCC ATCTGTCCTT GACAGTGGCT GTCATATTGA CTCCTTTGNT 60
GNTTTGTTGG TATTGGGGAC ATTTTCCACG GCTGAGTTNT TTTTGACTGA CATGTTTATG 120
ACATAGACGT NGTTTTCGCA TCCNTGACTT AAACTGACTT GNACNCCNTT N 171

SEQ ID NO:5901
SEQUENCE LENGTH:76
SEQUENCE TYPE:nucleic acid
TOPOLOGY:linear
CLONE:HUMGS07007
SEQUENCE DESCRIPTION:
GATCTACTGT AAAAAGAGGA TTAAGNAAAA TAAAATNAGA GCAATTATAT ATATAATTNT 60
ATATCATACA CAGAAA 76

SEQ ID NO:5902
SEQUENCE LENGTH:118
SEQUENCE TYPE:nucleic acid
TOPOLOGY:linear
CLONE:HUMGS07008
SEQUENCE DESCRIPTION:
GATCACGGGA GCAATGCTGT ACGGTTTTGT ACACTGGTGG TTTGTTTCCT AGAAAACCCA 60
TTGTNTCTCT GGATTTCTAG CACATTACTA AAAGAGCCTC TNCTTTGTAA ACATGAAA 118

SEQ ID NO:5903
SEQUENCE LENGTH:185
SEQUENCE TYPE:nucleic acid
TOPOLOGY:linear
CLONE:HUMGS07009
SEQUENCE DESCRIPTION:
GATCANNCTG ANCCAATTTA CCAAGCATTA TTAAGGAGGA ATAATNCCAT ACCATGTAAA 60
ATACCATGAT ATGCTGATTA TAATACATTA ACAAATTTTT AAGTTGCGTT CACTAAATNC 120
TGTCCTGTTT CTTCAAAATA ATATAGCTTA AATTGCATGN TAATNGTATA TCTTACCTNT 180
TTNGN 185

SEQ ID NO:5904
SEQUENCE LENGTH:134
SEQUENCE TYPE:nucleic acid

TOPOLOGY:linear
CLONE:HUMGS07011
SEQUENCE DESCRIPTION:
GATCTACCCA ACTCTGTCAT CTGTAACAAC CAGTGATAAC TCTCTTGTCT TGTAAAAAGG 60
GTTTATACAT AACTTGTACA TGGTTTCATT TTGTATTTNC CGCAGATTAA AAATTTATGT 120
ATTTCTNTTC TAAA 134

SEQ ID NO:5905
SEQUENCE LENGTH:297
SEQUENCE TYPE:nucleic acid
TOPOLOGY:linear
CLONE:HUMGS07012
SEQUENCE DESCRIPTION:
GATCGAATTN CNACCCCTAA NCTTGTCCGG ACGTATGGTC CAAATTCANA TTAAGGTGGT 60
CACCCAACCC GAGATGTCAG GAAAGGCCTT CTGCAGAGAA AATNTCCCCC CACCCGCCAT 120
CTGCAGCCAG GTGTGTGCCA CACGGCAGCC TTCCCGAAAC ATAGTATGGA TTTTAAAAAT 180
GTGTTTATTT TTNTNTCTCA ACCACTTTAT AACGTATTTC TCAATTTANN TTGTAATGTC 240
TTGTNTTGGG GNGTTGCTGG GTATCCTTNG TTATGCTTCC CACTGTTNTT NTCACTN 297

SEQ ID NO:5906
SEQUENCE LENGTH:254
SEQUENCE TYPE:nucleic acid
TOPOLOGY:linear
CLONE:HUMGS07013
SEQUENCE DESCRIPTION:
GATCTAGAAT GGGGCTGCAA GNCGGTCCCC ATTTTGGATG GGTCACATTC AGACACCACA 60
CTATGGTTGC ATCACCTGTC ACACCGACCT AGACAGCTGG ATGCCATTTG GNGTAGAATA 120
ACCAGACTGG TGAGTAANCT AGAAATCATA TTACGTGAAG AGTGGCAAAA GGCCTGAGAN 180
AGTTTAGTTT GGNAAACATG ACTTANAAGG GGCATCACTG CCCTCNCTAT TTNTNTNANG 240
GCCTCATTTC CNGN 254

SEQ ID NO:5907
SEQUENCE LENGTH:120
SEQUENCE TYPE:nucleic acid
TOPOLOGY:linear
CLONE:HUMGS07014
SEQUENCE DESCRIPTION:
GATCCATGAA GGAATCGGGA GTGGATGGAA TGCGGTAAAA CTGTCCTGGG GCCGGACCCG 60
TNGCCCAAAG TCCGGACCNN GACCNNAAAA ACGCTTCCAT CTTAGTCACG TTAGCAGAAA 120

SEQ ID NO:5908
SEQUENCE LENGTH:93
SEQUENCE TYPE:nucleic acid
TOPOLOGY:linear
CLONE:HUMGS07015
SEQUENCE DESCRIPTION:
GATCCCAATT TTGTAAAAAT ATCTGTAATA TGTAAGCATA GGTATTTGTA TATCTTAAGA 60
AAAAAAGCCT AGAAATAAAG CCACAAAAAT AAA 93

SEQ ID NO:5909
SEQUENCE LENGTH:306
SEQUENCE TYPE:nucleic acid
TOPOLOGY:linear
CLONE:HUMGS07016
SEQUENCE DESCRIPTION:
GATCTCAATN TGCAAACTCC TTGGNCTCCA GACCCCTGTC TGGNTGGNAC TCCTAGCAGT 60
GTGTGGTTTC ATCAGGAAAT CCTTGCAGCA GGGGNTTAGA TGGTTCCTTC AGGAGGTGGN 120
GAGCCTCCAT AGCATTGAAG GCCAGCCTGC ATCTCGCGGT GTGTGTCCAC TTCTCCCCTC 180
ACTTTCCCCT NACCCATCCN TTTAAAGTGA TTGGCTGTGA GGAGCACAGA GGNGTGGCAA 240
TTCCATCTGT TNATGGGGTC AGAAGACATA GCCGNCATGG NGATTTCACA ATTNACCNGN 300
GTAAAN 306

SEQ ID NO:5910

SEQUENCE LENGTH:80
SEQUENCE TYPE:nucleic acid
TOPOLOGY:linear
CLONE:HUMGS07017
SEQUENCE DESCRIPTION:
GATCATATAG AGTAGTTAAG ACAACTGTAA CAGCATTTTT TGAAATACAT TGTAAAATAA 60
TAAAAGGTAA CATAATTAAA 80

SEQ ID NO:5911
SEQUENCE LENGTH:110
SEQUENCE TYPE:nucleic acid
TOPOLOGY:linear
CLONE:HUMGS07018
SEQUENCE DESCRIPTION:
GATCTTAAGC TCTGCTCTCA GCAGATTTCA GGTGTACCAT TTGTAAACTG TACTGAAGGT 60
GTGTCCTCAA GAAGAAAGTG TTCAAATTAA AAAAGCTGCT GCCAAGTAAA 110

SEQ ID NO:5912
SEQUENCE LENGTH:159
SEQUENCE TYPE:nucleic acid
TOPOLOGY:linear
CLONE:HUMGS07020
SEQUENCE DESCRIPTION:
GATCGGGAGG ACTGTGGCCA GCAATTAACA CCATGTAGAC TTCCTTAGTT CTTAAGTGGT 60
TGAATTCGCT GNTTGTTCTG TAACGTTATA AATAGTNNAT ATCTGAAGAC GGAGAGCCTG 120
TANTATTCTT CAGATTAAAT GAAGCGTGAG ACACTTAAA 159

SEQ ID NO:5913
SEQUENCE LENGTH:330
SEQUENCE TYPE:nucleic acid
TOPOLOGY:linear
CLONE:HUMGS07021
SEQUENCE DESCRIPTION:
GATCTCTCCC ATGCCTTTTA AGCAATTAGA ACTAGATAAT GCAGCCATTT GTCACCCAAT 60
TAAATTTGAA AGGCCAGAAA TGCATGGATT AGCACTTCAG CTTTTGCTGC AGTCAAAGCA 120
ATTGTTACTT AGATAGAAGG ACAGTTAAAN TGAAAAATGA ATTTTGNTAA AANTGCGGNN 180
GTATAGATAA TATTCACTTC TNCTTTTTAA TGCTTAAACA CCCTGTATTC TGTATTTNAC 240
TTGTATCATA NTTTTCATTG CTCATTCCTA TTTCCCTGNG ATACTTATTN CTTNCACAAT 300
ATTTCTTCAN GTGGATATGT NCTTTAANTN 330

SEQ ID NO:5914
SEQUENCE LENGTH:299
SEQUENCE TYPE:nucleic acid
TOPOLOGY:linear
CLONE:HUMGS07022

SEQUENCE DESCRIPTION:
GATCATAGCC CTNNGCCTGG CGGCACAGCA GCACTTGCGT TCTCGGGGCT GTCGATTNCC 60
TGCCACCTGG CCAGATAACC TCAAGATTTC CACCTTTTCT TTTCAGCTTG ATTGCATTTG 120
ACTATATTTN ACAGCCAGTG ATTGTAGTTT CATGTTAATA TGTGGCAAAA TATTTTTGTA 180
ATNATTTNCT AATCCCTTTC TGAGTACTCT GGGGCCCTGC ATTTATNAGG CACCTACCTT 240
CATTTTGCTA ACGCTTANNC TGAATAAAAG NTTTTTGATT CCTTAANAAA AGGGNNNGN 299

SEQ ID NO:5915
SEQUENCE LENGTH:85
SEQUENCE TYPE:nucleic acid
TOPOLOGY:linear
CLONE:HUMGS07023
SEQUENCE DESCRIPTION:
GATCCTTTGT CCAGAAGAAG CCCATGGAAT ATTGAATGTA ATACATTTAG TCAATTAANT 60
TTTAAGNAGA TTCTTATNTA ATAAA 85

SEQ ID NO:5916
SEQUENCE LENGTH:189
SEQUENCE TYPE:nucleic acid
TOPOLOGY:linear
CLONE:HUMGS07025
SEQUENCE DESCRIPTION:
GATCCAGCTC AGGGGAGCCC TCCTCCTGAC TTTGGAGACC TCCTTCCAGA AAGACTGCTC 60
GGACTATGGA CTCTGAGCTC TTAAGTTGCA CCCGCTGTAA TACAAATNAG TTGTTTGCCT 120
GAATTCCAAG AACTGTCTCT GTGGTCTAGG CTGTCTGAAA TTTCTGTTGA GGGTAAGAGA 180
ATAAGGAAA 189

SEQ ID NO:5917
SEQUENCE LENGTH:215
SEQUENCE TYPE:nucleic acid
TOPOLOGY:linear
CLONE:HUMGS07026
SEQUENCE DESCRIPTION:
GATCCCTAGG GATTTNTCAT CTTAAGACTC AAAAGGCTTA ATACCAGGAA CCACCTTGGC 60
AAGATATTTA CCCACCGGCC ATCTCTGTTT ACTCATGAAT GTTAAATGTT AAAACGCAGC 120
GCTCTAACCC TGCNTNNNAT TTACTTGCAA ATGTCTGTAA TCTGTAATTG TNATGCCTCT 180
GATGGAATAA NTTATCTTTT TCAGTCTCCT CTAAA 215

SEQ ID NO:5918
SEQUENCE LENGTH:100
SEQUENCE TYPE:nucleic acid
TOPOLOGY:linear
CLONE:HUMGS07027
SEQUENCE DESCRIPTION:
GATCGGATTG ATTAAAAAAC AAATNCTCTG GAATTTTTNC GTTCATGCTT TTCCGTATTC 60

TTTATGGCTT TTAAATAAAT ATACAATGGT TAATAGTAAA 100

SEQ ID NO:5919
SEQUENCE LENGTH:163
SEQUENCE TYPE:nucleic acid
TOPOLOGY:linear
CLONE:HUMGS07028
SEQUENCE DESCRIPTION:
GATCAGGGGT CCGGCTGTCC TTCTGTCCTG CTGCAGACTA AAGGTCTGGC CAATGTCTTG 60
CCCCACCCCG CCAGCCGCGA TACGGCGCAG TTCCTATATT CATGTTATTT ATTGTGTACT 120
GACTCCATCT GCCCCGTCAA ATAAAAAACC ACAAGGTTCG AAA 163

SEQ ID NO:5920
SEQUENCE LENGTH:132
SEQUENCE TYPE:nucleic acid
TOPOLOGY:linear
CLONE:HUMGS07029
SEQUENCE DESCRIPTION:
GATCACTGCT GCTAGCTGAC TGGACCTCCC CATTGGAAGT TTGTAATTTT NCTTTGGCAA 60
AGTTTCATTG ACTAGTAGAA CTCATTCTGT TTTAGTGTAT ATTTCAATAT AAATGTAAAC 120
ATTTTGCTCA AA 132

SEQ ID NO:5921
SEQUENCE LENGTH:249
SEQUENCE TYPE:nucleic acid
TOPOLOGY:linear
CLONE:HUMGS07030
SEQUENCE DESCRIPTION:
GATCCTAAGG AAGGAAAAAG AAAGTGTTAA AAATTAATAA AGGTTTTTTT GAATCCCGTG 60
TCCTTCAAGT GCTTTCTAAC TTTGAGAGGA AGAAATTGAC CACCTGGACT ATGGAACTGT 120
GCGTAACAGC TTTGAAAGTG TATTTAAAAN TTAANTCTAT ATGCCTTTAA NTCAGTGAAT 180
TGGAAACATA TTACATGNGN TTNNATGATT TNCCTCAAAT ATANTAANTN GTTTCCTTTC 240
CAGTAGAAA 249

SEQ ID NO:5922
SEQUENCE LENGTH:191
SEQUENCE TYPE:nucleic acid
TOPOLOGY:linear
CLONE:HUMGS07031
SEQUENCE DESCRIPTION:
GATCTGCAAG TAGCCTAAAA ATGCGATTGC TGGTAAACCT GGCCTCAAAT TTCATACTAC 60
CATAACTNNT TTTATATATT GCCACTAATT TTGACTGGAT TTAATAGCAC TTTATTGTAC 120
AACTACAAAA AAAAANTNTA TTCCTGGANT TGTTGCCNGT GTAATTNCTC TAATGTNCTG 180
GTGCTTTNCA N 191

SEQ ID NO:5923
SEQUENCE LENGTH:236
SEQUENCE TYPE:nucleic acid
TOPOLOGY:linear
CLONE:HUMGS07032
SEQUENCE DESCRIPTION:
GATCTTATTT CATGAAGGTT GTATAAATGA AATCATTCAG TAATGTATCT TTTTAAGACA 60
GGTTTTTTTT TTTCNCTCAG CACAATTTCC TTGAGGTTCA TCCAGGTTGT TACCCATAAT 120
ACCAGTTTGT NAGTCTTTAT TGCNGTAGTC TATAGCATGC TTATATCACA GTCTGNCCAA 180
CCATTCACCC NTNGATGGGC ATATGGGCAT TNCCNGTTTG GNGCTATTNC AANTAN 236

SEQ ID NO:5924
SEQUENCE LENGTH:101
SEQUENCE TYPE:nucleic acid
TOPOLOGY:linear
CLONE:HUMGS07033
SEQUENCE DESCRIPTION:
GATCTNTTTC ATAGTGTTCA AANCTGTATT ATTATATGTA GAGGGAAATA ATTTTCTCAA 60
CACTAATCGT TAATAACTAT TGTATTAAAT NGTCATNANG N 101

SEQ ID NO:5925
SEQUENCE LENGTH:193
SEQUENCE TYPE:nucleic acid
TOPOLOGY:linear
CLONE:HUMGS07034
SEQUENCE DESCRIPTION:
GATCTGCAGT TTCCCCTTTT NTCCCCTCTG AAGAGTGGTT CTTATGTGCA ATCTGCAGTA 60
ACCTTGAACT CCAGAGCTGC ACTATAGAGG AGAATGCATG CCACTATGAC AGCAGTATGC 120
CAAGCTTTGT NTTCATCTCC TAATAAACGT TAAGACCTTT GGTGTAATAA ANGCAGCAGC 180
ATTCACCAAC AAA 193

SEQ ID NO:5926
SEQUENCE LENGTH:251
SEQUENCE TYPE:nucleic acid
TOPOLOGY:linear
CLONE:HUMGS07035
SEQUENCE DESCRIPTION:
GATCTGGGGT AGGGGGCACC CTACAGTGGG ACCCNTCCCN CATTATTCTT TCTNTCCAGC 60
CCCTCCCTCC CACTGGAGCA GCTCCAGAGN CATTCCTCAC CCCCGTNACC TCTCCCAGCC 120
AGGGCTGAGA GGATTCGAGG TGGCTGGGAG GGTTTCCAGG CCCCCTGCCC CTTNCCCACA 180
AACTGGGTGN TAGGGCGGAC TTACCTGGCC CAGCCCTGCC TCCNTNAGCC AACCCAGNCC 240
NTNGGGCTGT N 251

SEQ ID NO:5927
SEQUENCE LENGTH:132

SEQUENCE TYPE:nucleic acid
TOPOLOGY:linear
CLONE:HUMGS07036
SEQUENCE DESCRIPTION:
GATCTCGATA TTTCAAATGG GCTTTNNATG CACTGTTGCC AAGAAAGGCT TTTCCTGATT 60
TTTTNACAAN TNAATTTTTG CACACTTTCA TTGGTGTCTT TCGGCAACTT ACATTGAAAN 120
TGAGCTATTG TN 132

SEQ ID NO:5928
SEQUENCE LENGTH:90
SEQUENCE TYPE:nucleic acid
TOPOLOGY:linear
CLONE:HUMGS07037
SEQUENCE DESCRIPTION:
GATCCCTGGG TTGCCCTGTC CCAACCTGCT TGTTAGGTGC TTTCCCATAG NAGGCCCTTC 60
TTGAGAAACA ATAAACTAGG TAGAACTAAA 90

SEQ ID NO:5929
SEQUENCE LENGTH:119
SEQUENCE TYPE:nucleic acid
TOPOLOGY:linear
CLONE:HUMGS07038
SEQUENCE DESCRIPTION:
GATCTCAAAA CTCATTTGTT GTACCATATA TNATGGTTTT NTGTCTATAT CATATATTTC 60
CCTACTTATG TGTTTAAATN CTATTTNGTN ATGTCAATCT CTCTGATGCC ACCTATTTN 119

SEQ ID NO:5930
SEQUENCE LENGTH:252
SEQUENCE TYPE:nucleic acid
TOPOLOGY:linear
CLONE:HUMGS07039
SEQUENCE DESCRIPTION:
GATCGGGGCC AGAGGCAGAA CCCTGGTGAG GAAGCTCCAG TCCTGCTCTC TACCCAGCCC 60
ATCTTGCCTC CATGGTGCCT CTGGAGGCCT CTGGGCCTCC TCTAACAGGG GCTGGTGGGC 120
ACCAAGAGNC AATGGAGTAG ACCCCTGGNT GGTAAGGGNC AAGTNCCACC GGTTGCTTCT 180
GGGAAGGGGT TTCTAACACT AGTTTGTGTG CTGTGGTTCC TGGGGTGCCC TCCACTGCCC 240
TCTNTTCAGG AN 252

SEQ ID NO:5931
SEQUENCE LENGTH:76
SEQUENCE TYPE:nucleic acid
TOPOLOGY:linear
CLONE:HUMGS07040
SEQUENCE DESCRIPTION:
GATCCCACTG CAGTGGTGCC TACATTTATC ATCATGGCTC CTCTTGAATA AAATCTACCT 60

TACCATCTTC AACAAA 76

SEQ ID NO:5932
SEQUENCE LENGTH:103
SEQUENCE TYPE:nucleic acid
TOPOLOGY:linear
CLONE:HUMGS07041
SEQUENCE DESCRIPTION:
GATCAGCACA GTAATGCCCA ACAATATTGT TTTTAAACAA AAAATGTAAA CATTCAAAAG 60
AATCTCTCTA AATCACTGAT TAANTCATTT GACTGAAAAC AAA 103

SEQ ID NO:5933
SEQUENCE LENGTH:231
SEQUENCE TYPE:nucleic acid
TOPOLOGY:linear
CLONE:HUMGS07043
SEQUENCE DESCRIPTION:
GATCTACCCG NCTNGGCCTC CCAAAGTGCT GGCATTACAG GTGTGAGNCA CCGTGCCCGG 60
CACCAGNATC CTTTGGTATA GCCAAGCCTT TTGGTTACCG NCTCATGAAG AATATGCTTC 120
CCGCATTGTC CTAGTCCCAG TTGTATTCTC ACAGGTGTTA TGNGGGGGGC ACAATCCAAA 180
TNATAAACCT GGTTCATGNC CAACANATTT NTGCTAAATA GGGAGGNGGG N 231

SEQ ID NO:5934
SEQUENCE LENGTH:279
SEQUENCE TYPE:nucleic acid
TOPOLOGY:linear
CLONE:HUMGS07044
SEQUENCE DESCRIPTION:
GATCTGAGCC CAGCGTACAG AGAAGGCTCT GTGCAGACAC TGCTCACACC TCCAGCTCCC 60
AGCTCACCTG CCCCACAGGT GCCAGGNTAC CTNTTTNANT GGCCAGCAGG GCCCTNACCA 120
TCCTCAGCCT GCNTTCCAGT CGCCCATTCT TTCCATTCTG CGGCTCGTGC TTTAAAAGCA 180
ACCAGCCAAC CNTTTNTGAT ATAGTAGGGG ACTTGAAAGG GGCTAAGAAG ACTGANGATG 240
GTCTGCCACC TTTAACCACA AGGNTTNGGG CTTTGGTTN 279

SEQ ID NO:5935
SEQUENCE LENGTH:235
SEQUENCE TYPE:nucleic acid
TOPOLOGY:linear
CLONE:HUMGS07046
SEQUENCE DESCRIPTION:
GATCTAGGAG AAGGGNCTCA TGCGGACCCT CACATGGGCA GAAAAATGGT GGTCATTGGC 60
CGACATCACA GTTTTCCNGT TTCCCACCCA GCTAAAAACC GTTGTTTGCT TTAANTTTNN 120
ATAAACTGGA ATCCTTTCAC CCGCTCCTAC AGCTAACCCT CACANGCATG AGGTGCTGTG 180
GCTGTNCCTT ATCCTAATGA TGCGCTNNGG NCCCGTAAAT GTNAACACTC ATGAN 235

SEQ ID NO:5936
SEQUENCE LENGTH:119
SEQUENCE TYPE:nucleic acid
TOPOLOGY:linear
CLONE:HUMGS07047
SEQUENCE DESCRIPTION:
GATCAGTTAC ATTCATCAGT TGGTGAGTTT TGAAGAATAA CTACATTTNA TTTCAAACTA 60
ACAAATGTAT ACGGTTTTAG TTCAGTGTTG AAGAATTTNA ATACAATATT AANTNNCTN 119

SEQ ID NO:5937
SEQUENCE LENGTH:213
SEQUENCE TYPE:nucleic acid
TOPOLOGY:linear
CLONE:HUMGS07048
SEQUENCE DESCRIPTION:
GATCCCTTTT NAAAAATATA TGTATGCATG TNTGCATTAA AGAGTCTGAA AGGATACTTG 60
CCAAAGTGTA AAAGCATTTA TCTCTGGGAG GTGAGATTTG GAGCAATTAT ATTTNCTTTT 120
TGCATATCTG TAACTNCTNA TTTCCCNCAA TGNGCATGTA GTGTCTGTAA AATTTTCCAG 180
GGAATAANCA NTGATGTTCA CAATATATAT TNN 213

SEQ ID NO:5938
SEQUENCE LENGTH:147
SEQUENCE TYPE:nucleic acid
TOPOLOGY:linear
CLONE:HUMGS07049
SEQUENCE DESCRIPTION:
GATCCAGACC ATCCTGGCTA ACATGATGAA ACCCTGTTGC NTGTNGTACC AACTACTTGG 60
GAGGCTGAGG CAGGAGAATG GCATGAACTC GGGAGGCGGA NTTGCAGTGA GCCTCCAGCC 120
TGGGCAACAG AGTGAGACTC TCTCAAA 147

SEQ ID NO:5939
SEQUENCE LENGTH:299
SEQUENCE TYPE:nucleic acid
TOPOLOGY:linear
CLONE:HUMGS07050
SEQUENCE DESCRIPTION:
GATCCTGGGA GCCTCTGTTC TTTGCGCATT TCAGACACAG TCTGTGTGGC GAGGAGTGTG 60
ANGGNAGGAG CCTNGGGTGC AAGCCCGTGT NTTTGGCCTC TTTCCTCGTG AAGACGATGT 120
GTCCCCGTCA GAAAAAGTGG GCTCCNTCTG CAGCCCCGTG AGCTGAGCCC AGGCTGCGTA 180
GTGACCACAA GCTTATGTGN AGCACTGCTC AGGGAGGNTN TCAGGAATTC CCCTCACCTC 240
GGAAAGGANC TTCTCAGTTT TTATTGGGGG TGTNTAAATT TNCTTNCATA TNGTTCATN 299

SEQ ID NO:5940
SEQUENCE LENGTH:186
SEQUENCE TYPE:nucleic acid

TOPOLOGY:linear
CLONE:HUMGS07053
SEQUENCE DESCRIPTION:
GATCCTTCAG GCAGTAAGGG AGAGTTTTGC CTCCTTACAC AGTGGCCTTT GCTTGCACCT 60
CCAGCTGGAG ATGGGTGTGC CCCAGAAGTA AGCTTTGCAT CTCTTACAAG AGGGGAGCTA 120
CAGGGGCAGC CGTGGCCTAG GCCCAAACTC TGCTCTGAGA AAATAAATAT CTGTACCACC 180
TGTAAA 186

SEQ ID NO:5941
SEQUENCE LENGTH:195
SEQUENCE TYPE:nucleic acid
TOPOLOGY:linear
CLONE:HUMGS07054
SEQUENCE DESCRIPTION:
GATCCGGTNA TGGTCTGGGC AGAGGCTGGG TCAGGAGTCC CAAAGGTCAG TGACAGTTTC 60
TCAGAAGAGG CCCAGCGTCC ACCTCTCTCC CAGGGCCAGA CACCTCTTCC TGGCTCCCCC 120
ATCCCCCTAT GGGCTCCCAG CNCCTTGCAC CCTCATTGCT GNNNAGATTA AAGCCTCTGT 180
TTTGCACCTG TCAAA 195

SEQ ID NO:5942
SEQUENCE LENGTH:116
SEQUENCE TYPE:nucleic acid
TOPOLOGY:linear
CLONE:HUMGS07055
SEQUENCE DESCRIPTION:
GATCATTCAC TGTTTTNGTT TTAAAACTGG TCAGTACTAT GAAGCTTCTG TGGTTTGTAT 60
GATGTTTTAA CTTTCACTTT AAACTGCATA GAATAAATTA AATTGAAAAC ANCAAA 116

SEQ ID NO:5943
SEQUENCE LENGTH:116
SEQUENCE TYPE:nucleic acid
TOPOLOGY:linear
CLONE:HUMGS07056
SEQUENCE DESCRIPTION:
GATCCGNAAG CNAGANACCC TGCAGGCTGG CGGGGACCCA NGGGAAAGGC CTTCCCAGGC 60
CCTTCTAACC CCTGTGGCCC TGGACTTTCC TTGTNTGGAC TCACCCCAGG CCTGGN 116

SEQ ID NO:5944
SEQUENCE LENGTH:179
SEQUENCE TYPE:nucleic acid
TOPOLOGY:linear
CLONE:HUMGS07057
SEQUENCE DESCRIPTION:
GATCATAATC ACTGCCTTAC CNNCCTCACG GTTGTNGTAA GGACTGAGTG TGTGGAAGTT 60
TTTAATAAAC TTTGGATGCT AGTNTACTTA GGGGGTGTNC CAGGTGTCTT TAATGGGGCC 120

TTCCAGACCC ACTCCCCACC CTTNTCCCCT TCCTTTGCCC GGGNACGNCG AACTCTCTN 179

SEQ ID NO:5945
SEQUENCE LENGTH:166
SEQUENCE TYPE:nucleic acid
TOPOLOGY:linear
CLONE:HUMGS07058
SEQUENCE DESCRIPTION:
GATCTAGATT AGCAATATAA AGAAGCATAG TGGTACTCTG TTTCACACTT TCAGTAGATT 60
TATTAGAAGT CAAATNCTAT TCAACAGACA CTTATTAGGA TATACAACTA ATTTANGAAT 120
AAAATTCCAG GCACAATATA TTTNNNTNAA NTGGTATTTG TNAGTN 166

SEQ ID NO:5946
SEQUENCE LENGTH:230
SEQUENCE TYPE:nucleic acid
TOPOLOGY:linear
CLONE:HUMGS07059
SEQUENCE DESCRIPTION:
GATCTTTTGT GACTACAACT TCTNTTGTAA ATAATGATAT ATGGTATTNC CATCGTCAGT 60
TACCGAGTAT AGCCACTGGG TATCACTACT TTGTGTTAAA GTGCCTTCGC ACTTTAAGTA 120
CATTACTTAA ATGTTGCTTT TAGCTTTGAT AAATTGAAAA TATTTNAATG GGNNGGGGNN 180
TTGAAATTGA AAACACTGTA AAATAGGTTG GNGTGTCAGC NNTATTANGN 230

SEQ ID NO:5947
SEQUENCE LENGTH:177
SEQUENCE TYPE:nucleic acid
TOPOLOGY:linear
CLONE:HUMGS07060
SEQUENCE DESCRIPTION:
GATCCGCCTA CCTTGGCCTC CCAAAGTGCT GGGATTACAG GCATGAGCCA CCATGCCCAG 60
CCAGNGATTT AATTNTTAAT AACTAATTTN AGGGCACAAT TTGACCTCCA TTGNCAGCCT 120
GTTTGTATTA CTTAATGTGA ATATNTGTGT GTTTTGCCAN GGGNTATTGA NGTATTN 177

SEQ ID NO:5948
SEQUENCE LENGTH:96
SEQUENCE TYPE:nucleic acid
TOPOLOGY:linear
CLONE:HUMGS07061
SEQUENCE DESCRIPTION:
GATCTCCAGG CCCAGCCTGG CCCCAAATAT TCTTTCCTTT CATCCTCAGC AAGTGCTGAG 60
TCTGTGAATA AAGCCACATA ACCAGCGGGC ACTAAA 96

SEQ ID NO:5949
SEQUENCE LENGTH:191
SEQUENCE TYPE:nucleic acid

TOPOLOGY:linear
CLONE:HUMGS07062
SEQUENCE DESCRIPTION:
GATCATAAAG CCACCGGCCA CTGCCACGCA TGTTGCACCT GTGCCATCGT GCTCCCTCCT 60
GATGGGCACC GTGGTGGAGG AAGTCACCCA GCTGTTTCTC AGTCCCAGAG GCCGGTGGCT 120
GGTTTTAAAC TTGTGTTGAC TGTTGATACT TATTTACTGT ATAAATATAA TTNATCATTT 180
GTACCATGAA A 191

SEQ ID NO:5950
SEQUENCE LENGTH:109
SEQUENCE TYPE:nucleic acid
TOPOLOGY:linear
CLONE:HUMGS07063
SEQUENCE DESCRIPTION:
GATCCAACTG GGAGAACCTC AGCCAATGCT GGAAGNATGA TTGAAGTACC TCTCTTTTNT 60
NACTCTTGTA CAGCTTAATG TGCAATAAAG GAAAAGTTAT ATCTGTAAA 109

SEQ ID NO:5951
SEQUENCE LENGTH:216
SEQUENCE TYPE:nucleic acid
TOPOLOGY:linear
CLONE:HUMGS07065
SEQUENCE DESCRIPTION:
GATCGACCTG AAACTCCTCA CCAAAGTNCT CGNNCCGGAG CACGAAGTCC GGGAGGATGA 60
TGTCGACTGG GACTGGGACC ATCTGTTCAC TGAGGTGTCC TTAGAGGTCC TCACTGAGTG 120
GGACCCACTG CAGACGGAGA AGGAGNACCC TGCGNGGCAG GCCATNCACA CCTNAGCCCG 180
TCACCCATGC TCTAGACATN AAGAANTGCA ANGGGN 216

SEQ ID NO:5952
SEQUENCE LENGTH:192
SEQUENCE TYPE:nucleic acid
TOPOLOGY:linear
CLONE:HUMGS07066
SEQUENCE DESCRIPTION:
GATCCACCAA AATAAATGAT TTTCTACATT TTCATTTGGA CTAAATCCCA CGAATGACAA 60
CTACCACCTT TTTTTCCTTT TTAATTAATA CTAAATATTG TGATTTCTTA TTTGAGGTTC 120
AAAATGACCT GCTTGAAACT TTGATACATA TTGGAATACA TTATGTTAAT AAACTTGTAG 180
CTTTTTGTGA AA 192

SEQ ID NO:5953
SEQUENCE LENGTH:298
SEQUENCE TYPE:nucleic acid
TOPOLOGY:linear
CLONE:HUMGS07067
SEQUENCE DESCRIPTION:

GATCACACCC TGGAACAATT GGNGGATTGC TTGCTGCCCT GCATCTTAAG GCGTATTCTA 60
GCAGCAAAGC TTGTNATGCA CTGACTTGGG GCAAGTAAAA CTGATGCTGA GGACAAAGGG 120
AGANGATGTT CCTCGCCCAG GGAAATNAAA ACTTTNCTNA TTCACCTTGC TTGTGCTTAA 180
NCACTTATGT NCATCTAGTG TAGAATACAT GGCTACTTGT TTGTAATTNT NTNATTGGTG 240
GAATTAAAGT AATTTTCCCT TATCTTTGAN CTTTTATTTA GAGAACTNAA ANAAAGCN 298

SEQ ID NO:5954
SEQUENCE LENGTH:250
SEQUENCE TYPE:nucleic acid
TOPOLOGY:linear
CLONE:HUMGS07068
SEQUENCE DESCRIPTION:
GATCTNCAGG GGNATGTTTT ACCATCTGNA TCCAGCCTCC TGCTAACTCC TAGCTGACTC 60
AGCATAGATT GTATAAANTA CCTTTGTAAC GGCTCTTAGC ACACTCACAG ATGTTTGAGG 120
CTTTCAGAAG CTCTTCTAAA AAATGATACA CACCTTTCAC AAGGGCAAAC TTTTTCCTTT 180
TTCCCTGTGT ATTCTAGTGA ATGAANTCTC AAGANTCAGT AGANCNAATG ACNTTGGNAT 240
GTAATGNTNN 250

SEQ ID NO:5955
SEQUENCE LENGTH:135
SEQUENCE TYPE:nucleic acid
TOPOLOGY:linear
CLONE:HUMGS07069
SEQUENCE DESCRIPTION:
GATCCGTNAA TNCNCGGAAT GGCAGGGGAA GCCTTGCACT AGGTTGCAGA GAAGCATCCT 60
CCACATCCTG TGTCAGAAAC CCTGGTCTCC GTGGCACTTG TAACTCACCG TNCTGTCTNC 120
TGGTCTGTNT GTGTN 135

SEQ ID NO:5956
SEQUENCE LENGTH:193
SEQUENCE TYPE:nucleic acid
TOPOLOGY:linear
CLONE:HUMGS07071
SEQUENCE DESCRIPTION:
GATCTAGAAT TGACATTTTG CCTTCTTGTT TCCAGGTGTT TCTATTTTTT CTATTCTTTC 60
AGAGAAATCT CATATTTCGG TGTATTTATT GCTGTTACTA CTATATTTAC TGCTGAAAAC 120
TGTAACAACC TGAAGATTTG TAAAATGTTA AACATAGTTC ATTAAAAATA ATAAAATAAA 180
TCTAAAATGT AAA 193

SEQ ID NO:5957
SEQUENCE LENGTH:273
SEQUENCE TYPE:nucleic acid
TOPOLOGY:linear
CLONE:HUMGS07072
SEQUENCE DESCRIPTION:

GATCCTCCAA AGGCCTGCCC CATGNATTTA ACAGTAATAC AGGAAGCATG GCAGGCACCA 60
TGCAAACCAA GGATGGATGG TGCAGTCCCT GTGTCAGTGG GCGGTGGTTT CCTGCTGGCC 120
TGGAATCACT CATCACCTGA TTGATTGGCT CTGTGGTCCT GGGCAGGTGC CTCATAGGTG 180
TGTGGNTATG ATGACGTTTC NNTAAAATGT ATGTNTTTAA CANATACTNA ATNGTATTAN 240
GGTCATGTAC CAATGGNTTT NATAATGTTT NTN 273

SEQ ID NO:5958
SEQUENCE LENGTH:108
SEQUENCE TYPE:nucleic acid
TOPOLOGY:linear
CLONE:HUMGS07074
SEQUENCE DESCRIPTION:
GATCAGAAAG NATTAAATAT CACTACCACA TGAGTTAAAA CAAAAAATGG AATAAAAANC 60
AAAAGTGGTG TCTATGTTAT CAGTGGNTTA CTTGATAGGN CATTTNAN 108

SEQ ID NO:5959
SEQUENCE LENGTH:256
SEQUENCE TYPE:nucleic acid
TOPOLOGY:linear
CLONE:HUMGS07075
SEQUENCE DESCRIPTION:
GATCTTCACA AGGNTTGTAT TGGCCACATC CACGTCCTCC GAGCCTACAT CAAGACCCAA 60
GTGAACAAAG AGCTGGAGCA GCTCCAGGGG CTGGTGGAGG AGCGGCTCAA GGCCAGCGAG 120
GAAAGGCTCA GCAGCAAGTT GACTGCACTA GAGCGGCCCT TCCAGCTACC TCCGGGTAAA 180
GGCAAGAGCA AGACCAAGTG ACCCCCAACA TTTTCCCCAA TAAAGGTCTG GGCCAGANTG 240
GCACCATCCC TGCAAA 256

SEQ ID NO:5960
SEQUENCE LENGTH:262
SEQUENCE TYPE:nucleic acid
TOPOLOGY:linear
CLONE:HUMGS07076
SEQUENCE DESCRIPTION:
GATCTAGTCA TCTGGTTAAG GATTTTAAGC AGATGCAACT ATAAACCCAA GAAACTGTAT 60
TACTATTACT GTTGGTCATA CTAAACCTGT CTATTTCCTG AAGTATATGA CCCACAAGGA 120
TGTGGAATAA CTAGGAGAAA CTGTTTTTGT ACACTGTACA TCCTTAGTAT TTTTACACGT 180
ATATGATAGG GATGAACATG ATTTTNCTTC GTACAGACAG CTTANNTAAN GCACTATGTC 240
ANTCTGCAAA ANAGNNNNNN NN 262

SEQ ID NO:5961
SEQUENCE LENGTH:295
SEQUENCE TYPE:nucleic acid
TOPOLOGY:linear
CLONE:HUMGS07079
SEQUENCE DESCRIPTION:

GATCTCAGCT CACTGCAACT NNTGCCACCT GGGCTTAAGC CATCCTCCCA CTTCAGCATC 60
CCAAATAGCT GGGACTGCAG GCACACACCA CCACACCCAG CTAATTTTNG TATTTTTTGT 120
AGAGACGGGA TTTCACCATG TTGCCCAGGC TGGTGTTGAA CTCATGAGCT CAAGTAATCT 180
GNTGGCCTTG GCCTCCCAAA GTGCTGAGAT TATAGGCGTG AGCACTGCAT CCAGCTCACT 240
CCTNATTTCT TTCTAGCCCC AAAGGGGTTG AGTCAGCAAA TCCTNCAGCC TTTTN 295

SEQ ID NO:5962
SEQUENCE LENGTH:272
SEQUENCE TYPE:nucleic acid
TOPOLOGY:linear
CLONE:HUMGS07080
SEQUENCE DESCRIPTION:
GATCCTGGAA TGTTTGCTGG AGAAATTTAA AATACNGGGG TTTTTTNTTT AATGGTGCCT 60
ATTTAGAGTT GGAAGTTGAA CAGCTGTTGC ATTACATACT TTTCCTTTTT NATTGAAATT 120
TTGAAATCAA ACGTCTTGAT TTTCCTNTCC TGTTGAATTG CTATGTNCAG GATGTCCTAG 180
GGGGTGGGGG CAGGGACTCT TTTCGTAATA AGCACTTGTN TTATNTGGTG TGTGTGGNGT 240
ATAAANGGCT ACACCCTTAT TNTAAAAANN NN 272

SEQ ID NO:5963
SEQUENCE LENGTH:91
SEQUENCE TYPE:nucleic acid
TOPOLOGY:linear
CLONE:HUMGS07081
SEQUENCE DESCRIPTION:
GATCTGTCGT CTTCAAAAAA ATGGCAAAGT GGGAAATGTG GGAGGGGAAA ACACTTCAGA 60
ATAAAAGAGA TTTAAGAGAT GTATCAACAA A 91

SEQ ID NO:5964
SEQUENCE LENGTH:194
SEQUENCE TYPE:nucleic acid
TOPOLOGY:linear
CLONE:HUMGS07082
SEQUENCE DESCRIPTION:
GATCAGGTTG TCTGCATTTG TTGGTGTAAG TGAACATCAT CACAGTTATC CTGAGTTGAG 60
TTTAAGCCAA ATANATGCAT AGAAAAGGGT CTTCCTATTA ATGGAAGANG GTAATTTTTA 120
GGATGTGTAT TATTTCAGTT TTGTATGTTT AACTNTNATT NAATAAAGTG TTTTTAAAAT 180
CNCAAAAATN ANNN 194

SEQ ID NO:5965
SEQUENCE LENGTH:266
SEQUENCE TYPE:nucleic acid
TOPOLOGY:linear
CLONE:HUMGS07083
SEQUENCE DESCRIPTION:
GATCTGCCTG AAGAAAATAT CTGTTTTCTA TATAAAAAAA TTTTTTAAAA TAATTGTAAA 60

GTTAGATTTA AAATTGTAAA ATATAAAATC ACAAAGGAAT GTACCTTATG AATGTTGTTG 120
ACATTTTATG AAATTATGTG GATTCATATT ACTGTTACAA GATAGANTTG AATGCAAAAA 180
GTCCAAAACC TCAATAAANT TTGAGGAAAA CGTGTTATTA TGTAATTGAN NTANAANCAT 240
TTTATAATTG TGCANGTNAN NNNNAN 266

SEQ ID NO:5966
SEQUENCE LENGTH:256
SEQUENCE TYPE:nucleic acid
TOPOLOGY:linear
CLONE:HUMGS07084
SEQUENCE DESCRIPTION:
GATCCTTTTN AGTTTTTCCA GGTGAGAAAT CCTATCAATT ATGTNATAAT NATATGTTTT 60
CCTNTTCAAA ATTTATAACA TAATTCCTGA CTGCATGGGT TAAACATTTN CATAACAGTG 120
CTAAATAATA AAGGTGGCAG TGGCATTTTT GTCTTGTTTC TCATTTTAGC CAGAATGTGT 180
TTGCTACAGC AATTCCAAAT TGTGTTTTGT GAAAAANAAA TATTCTNCTT NGGAGACANG 240
GNNTNTACAT TTGCAN 256

SEQ ID NO:5967
SEQUENCE LENGTH:119
SEQUENCE TYPE:nucleic acid
TOPOLOGY:linear
CLONE:HUMGS07085
SEQUENCE DESCRIPTION:
GATCAGAACA GGCTTAAATT GATGCTATAG AACCAATTAC TCTGTATTAT CTTTGCAACT 60
CTTTTGTAAG TCTAAAATTG TTTGGNAATA AAAGTTTATT TTAAANNTTA AAAAANANN 119

SEQ ID NO:5968
SEQUENCE LENGTH:146
SEQUENCE TYPE:nucleic acid
TOPOLOGY:linear
CLONE:HUMGS07086
SEQUENCE DESCRIPTION:
GATCTTCTAA TCGACAATCT ACATCATAGT GACCACCAGT AATCACCTGT AAGAGATTAA 60
AAAAGAAAAA TCACTGTGAA AAGTGTGAAA AATTAATCAG ATTTAATCAG ATTTCCTATT 120
AAAATGGCAA TAAGGTGATG GGCAAA 146

SEQ ID NO:5969
SEQUENCE LENGTH:203
SEQUENCE TYPE:nucleic acid
TOPOLOGY:linear
CLONE:HUMGS07087
SEQUENCE DESCRIPTION:
GATCTGGTTA AGTGTGCGGC ATCTCATCCC CCACTCTNTC CCTTCTTGCT CCTGCTTCCC 60
CCTTATNACT TGCCAGCTCC TGCTTCACCT TCCACCATGA GTAAAAGCTC CCTGAGGCCT 120
CCCCAGAAGC CAAGCAGATG CCAACACCAC GCTTCCTGCA GCCTGCAGAN CTGTGAACCA 180

ATTAAACCTC TTTTCTTTAT AAA 203

SEQ ID NO:5970
SEQUENCE LENGTH:72
SEQUENCE TYPE:nucleic acid
TOPOLOGY:linear
CLONE:HUMGS07088
SEQUENCE DESCRIPTION:
GATCTTCCAA AAATTTATTA AGTGTTTACT TTTAAAGTGG AAACAATGTT TTTAAGAGGT 60
GATATAAAGA AA 72

SEQ ID NO:5971
SEQUENCE LENGTH:106
SEQUENCE TYPE:nucleic acid
TOPOLOGY:linear
CLONE:HUMGS07089
SEQUENCE DESCRIPTION:
GATCCCTAGC TTGTTTTCTG TCAGTCATNC ATTGTAAGTA GCACATNGCA ACAACAATCA 60
TGCTTATGAN CAATACAGTC ACTAGGTNTT AGTTTTNTTT AAATAN 106

SEQ ID NO:5972
SEQUENCE LENGTH:212
SEQUENCE TYPE:nucleic acid
TOPOLOGY:linear
CLONE:HUMGS07091
SEQUENCE DESCRIPTION:
GATCTGTGTG ACTGATGGGC AGGGCTCAAT GATGCCCATT GAACTGAGCT TACTGCTCAC 60
ACCACTGACC TGGACCCNAA CAAAAAGCTG ATTGTCTTTT TAAAAGTTAT TATTTTGCCC 120
TGAGCAAATT GCATTTTANT TGGGGCAGTT GGAGTGTTGA NTTCCTGGCA GCATTGTGAA 180
GNNGACCNNG GTGGANGTTT NCTGTCCCNG GN 212

SEQ ID NO:5973
SEQUENCE LENGTH:215
SEQUENCE TYPE:nucleic acid
TOPOLOGY:linear
CLONE:HUMGS07093
SEQUENCE DESCRIPTION:
GATCTACCCA CCTTGGCATC CTAAAGTGCT GGGATTACAG GCGTGANTAC CAAGCCCGGC 60
TTTTTTTGTT TTGACATGGA GTTTCGNTCT TGTCACCCAG GCAGGAGTGC AGTGGCGTTA 120
TCTNGGTTCA CTGCAACATN CGCCTCCTGG NTTCAAGTGA TTCTCCTGCC TGAGCCTCCC 180
ANGNACCTGG GNTTACAGGT GCTCCNACCA AGNTN 215

SEQ ID NO:5974
SEQUENCE LENGTH:140
SEQUENCE TYPE:nucleic acid

TOPOLOGY:linear
CLONE:HUMGS07094
SEQUENCE DESCRIPTION:
GATCTGGAGG TTTACAGAAG CCTTTACCCA TCCTGAGACT CAGTTCTGCT GTTGGTCTNC 60
AGAGAAGTGT CTGCACCTGC TGAGANGNAA TCACACCACT GCACTCTAGA CTGGGCAACA 120
GNGCGAGACT CTGTCTCAAA 140

SEQ ID NO:5975
SEQUENCE LENGTH:133
SEQUENCE TYPE:nucleic acid
TOPOLOGY:linear
CLONE:HUMGS07095
SEQUENCE DESCRIPTION:
GATCTGTTGA AGAACTCAGC TGCTGGAAAC CATGCAAAAT GTTTTGAATT GCCCTTAAAA 60
TATGGAAAAT GTTTTCTGAA TGCTTTATAT TCTTNCTGCT GTAAATTAAA AATGANGAAA 120
ATTTCCCCAT AAA 133

SEQ ID NO:5976
SEQUENCE LENGTH:144
SEQUENCE TYPE:nucleic acid
TOPOLOGY:linear
CLONE:HUMGS07096
SEQUENCE DESCRIPTION:
GATCCCTGCC CTTTGGCACA TCCACTGAAA GGCCAAACAG CAAGTCCGAG TGAGTTTTAA 60
ATATTAATTA ATCACCCTTT ATTTTTTACA CTTGAGAGTG ATTGTAATAA AGGCTGTCAT 120
TAATAAACTT GGTTCTACCT TAAA 144

SEQ ID NO:5977
SEQUENCE LENGTH:114
SEQUENCE TYPE:nucleic acid
TOPOLOGY:linear
CLONE:HUMGS07097
SEQUENCE DESCRIPTION:
GATCTAGAAA TTGCCCTCCT TTTACCCCTA CCATGAGCCC TACCAAAACC AAACTTANCC 60
CTGGGCAACT AANTAAGGTT ATTGGACATN CCCTTCTNAA TTAATNATCC AATN 114

SEQ ID NO:5978
SEQUENCE LENGTH:112
SEQUENCE TYPE:nucleic acid
TOPOLOGY:linear
CLONE:HUMGS07098
SEQUENCE DESCRIPTION:
GATCTGACTT CAGACAGAAA CCAATACCAG CTTCCTTTTC CTTTAAACNT TTGAAGAGTG 60
TTGATTTGTT ACTATATTAC GTATGCAAAA CTGGAGAGNG TATNGNTNGA TN 112

SEQ ID NO:5979
SEQUENCE LENGTH:131
SEQUENCE TYPE:nucleic acid
TOPOLOGY:linear
CLONE:HUMGS07099
SEQUENCE DESCRIPTION:
GATCCGTCCC TAACAAACTA GGAGGCGTCC TTGCCCTATT ACTATCCATC CGTCAATCCG 60
TAGCAATAAT CCCCATCCTN CGATTATATC CAAACAACAG ANGCATTGAT ATNNTNGCCC 120
NACGTAAGGG N 131

SEQ ID NO:5980
SEQUENCE LENGTH:126
SEQUENCE TYPE:nucleic acid
TOPOLOGY:linear
CLONE:HUMGS07100
SEQUENCE DESCRIPTION:
GATCATGTAG GACGNGGCAT CCTGCCCCTG GTAGAGAGGA CGAATNTTCC AAAACANTGG 60
GCTCTACGGN AAAAAGAAGT AGGTTAGTCA CCTTCTGGAC CCTGTCTAAC TCGTTTGCTN 120
AANAGN 126

SEQ ID NO:5981
SEQUENCE LENGTH:79
SEQUENCE TYPE:nucleic acid
TOPOLOGY:linear
CLONE:HUMGS07101
SEQUENCE DESCRIPTION:
GATCTTATTT AACCAAAGAC TATTTCATTC CTGATATGAT TTGTGTGCTT GATTAAAATG 60
GNANTTAATG ATATTGAAA 79

SEQ ID NO:5982
SEQUENCE LENGTH:271
SEQUENCE TYPE:nucleic acid
TOPOLOGY:linear
CLONE:HUMGS07102
SEQUENCE DESCRIPTION:
GATCAACAGG CTTATTAGAA GAATGAACTA AGGTGAGAGA CCTCATCCTG AGAAAGGTTG 60
AGTTAATGTT AGAGTATAAG TGGTGGTAAT CTTGAATACT TTTCCCATTA AATATTGATG 120
GGGGTTTGAN TTTTAATCAG TGCTTTTNTG GGNAGTGGGG GTCAACTATC TTAATGATTA 180
CAGTTGGTTT GTTTACTGCT TTTGAGGACC TTTCTGGAGG AAAGGAAAAG CCTGTTTTGG 240
GGAGTCTTTA AAGATGGAAA TTGGGTGGTC N 271

SEQ ID NO:5983
SEQUENCE LENGTH:125
SEQUENCE TYPE:nucleic acid
TOPOLOGY:linear

CLONE:HUMGS07103
SEQUENCE DESCRIPTION:
GATCAATATG TGACTGGAAC CATTGGCGAG GATGAAGATT TGATAAAGTG GAAGGCACTG 60
TTTGAGGAAG TCCCTGAGTT ACTCACTGAG GCAGAGGAGG ANGNATTGGN TTNNGAACCT 120
GCNTN 125

SEQ ID NO:5984
SEQUENCE LENGTH:73
SEQUENCE TYPE:nucleic acid
TOPOLOGY:linear
CLONE:HUMGS07104
SEQUENCE DESCRIPTION:
GATCTNATTG TATTGTCGAA GGACTATTTT TNAACCTGTC CAAATAAACT CTGCAAAGNA 60
TTTCCACAAG AAA 73

SEQ ID NO:5985
SEQUENCE LENGTH:331
SEQUENCE TYPE:nucleic acid
TOPOLOGY:linear
CLONE:HUMGS07105
SEQUENCE DESCRIPTION:
GATCCTGCCC TGCTCTCAGC CATAGTGAAG GACCAGCCCT AGGAGTCTGC GAGAGCCTCC 60
TTGGTTCCAT CGTGAAGCCA TAAACAGGAA TGCCTTTGGC AATAGCCTTG AGCCTAGAGG 120
GCCCTCTNAT GCCCCACTGA GGTGCTGTTG GTTTATTGCT GGCANCGTGA ATTCTCTCAG 180
GGGTCTAGGA GGGGCATTTT GGAGACTGCC TGACANCACC NCTATCCCCT GCCTCCCNNN 240
CTNAGAAGAG GGTGGAAGNA TGAAATGAAA GCTATGGGNC TTTTGGGAGG NTAACCCAGN 300
GTCTATTCTA GGGTTNGNGG AAGTGNNTAA N 331

SEQ ID NO:5986
SEQUENCE LENGTH:107
SEQUENCE TYPE:nucleic acid
TOPOLOGY:linear
CLONE:HUMGS07106
SEQUENCE DESCRIPTION:
GATCCCCTGG CCTGGTGCCC ACACTGCCTC GCAAGCGCTC GCCACCCTCA CGTGGCTCAC 60
CTGCTGTTGA GCCTTGTGCT GTCAATAAAC GGTTTGAGGA TTGCAAA 107

SEQ ID NO:5987
SEQUENCE LENGTH:83
SEQUENCE TYPE:nucleic acid
TOPOLOGY:linear
CLONE:HUMGS07108
SEQUENCE DESCRIPTION:
GATCAGATAT ATCCACAACG NGTTTAAGAT TTTCAATATT CTTAGAGAAA ATCAATAAAG 60
TCAATAACTA CTCCACCATG AAA 83

SEQ ID NO:5988
SEQUENCE LENGTH:157
SEQUENCE TYPE:nucleic acid
TOPOLOGY:linear
CLONE:HUMGS07110
SEQUENCE DESCRIPTION:
GATCAAAAAT TGTGGACATA TCTGCAAATN ACTACCTATA GCCTCATGAA AGTACATTTC 60
ACAAAATGTA AANNNCAATG AACACTAAAT TTAAGAGCAG TTACAGTGTG ACTCACTCAT 120
GTTTAAAAAA ANTCGGNGAG CTAAAAANTA CGTCTAN 157

SEQ ID NO:5989
SEQUENCE LENGTH:280
SEQUENCE TYPE:nucleic acid
TOPOLOGY:linear
CLONE:HUMGS07111
SEQUENCE DESCRIPTION:
GATCGAGTCT GCTCTGACCT CTCAATCTCC TTTCCTCCCC AGGCTGCTGC TCCTGCTGCC 60
CTGTGGGCTG TGCCAAGTGT GCCCAGGGCT GCATCTGCAA AGGGGCATCG GAGAAGTGCA 120
GCTGCTGCGC CTGATGTCGG GACAGCCCTG CTCCCAAGTA CAAATAGAGT GACCCGTAAA 180
ATCCAGGATT TTTTGTTTTT TGCTACAATC TTGACCCCTT TGCTACATTC CTTTTTTNCT 240
GTGAAATATG TGAATAATAA TTAAACACTT AGACTTGAAA 280

SEQ ID NO:5990
SEQUENCE LENGTH:205
SEQUENCE TYPE:nucleic acid
TOPOLOGY:linear
CLONE:HUMGS07112
SEQUENCE DESCRIPTION:
GATCTCCAAG CAGGAATATG AGGAGGGCGG GAAGCAGTGC GTGGAGCGAA AGTNCCCCTG 60
ATGGCACTCC TCCCCACACA CCTGCTCCCA AGCTCAGATG GAAGTCCCTT AACCCCCATG 120
CCACATTGCC CCCCTCCTCC TTTCCCTCTT GTCCTCATTA ATGGTGATGT TTCTGGGTTG 180
AAAGAAGTAA AAATGTTTTA AGAAA 205

SEQ ID NO:5991
SEQUENCE LENGTH:127
SEQUENCE TYPE:nucleic acid
TOPOLOGY:linear
CLONE:HUMGS07113
SEQUENCE DESCRIPTION:
GATCTTTAGC AGTCTCTTCT AGTTTCCCTG GATTTCACTG ATTTATTANT TAGGGAAAAT 60
GAAAAGAGGA CCTTNCTGTC CGCCTGCACT GCTTACTGGT AAACTATAAC AAACTTATGC 120
TGCCAAA 127

SEQ ID NO:5992

SEQUENCE LENGTH:240
SEQUENCE TYPE:nucleic acid
TOPOLOGY:linear
CLONE:HUMGS07114
SEQUENCE DESCRIPTION:
GATCGCTTGA GCCTGGGTAG AGGCTGCTGT GAGCTGAAAT GGCGCCNCTG CACTCCAGCC 60
TGGGTAACAG AGCGAGNCCC CGACTCAATA AATAAATAAG GATGGATAAA TTGTAGCGTA 120
TGCACCATTC ATTATGCTAA GACATTTTAA AGTAATTATG AGAAACGCTC TACTAATGAT 180
TTGTTTTTAT TTGTATTTTN CTGCTTATTA GACAAATATA TNCTCATTTT AAAANGGAAA 240

SEQ ID NO:5993
SEQUENCE LENGTH:328
SEQUENCE TYPE:nucleic acid
TOPOLOGY:linear
CLONE:HUMGS07115
SEQUENCE DESCRIPTION:
GATCAGAACG AGCTGTTAGA ATTCAGAGTG CTAGAACTCG AAGTAAGAGA CTCTATCTGT 60
TGTAAACTCT CAAACGGAGC AGACATTCTC TTTGAACCCA AACTGAAATT CATGTAAAGC 120
TCTCAGATGT TTTCAAGCAT GTGTAAAGGG GACATGTTAT AGTTTCTTTC TTTCTTTCTT 180
TCTNTNTTTT TTTAAANCTG TATGTNCAGA ATANTTNCNC TGCCTTAATG TGTNCTGGNG 240
AGCGTGCTCA CCCAGGNCTN TGGCCATGTN CCAGAGCTAA TATATTTNTT GCCTATGGCT 300
TNGTNTGGCA CTTAATGAAT ANTTGTGN 328

SEQ ID NO:5994
SEQUENCE LENGTH:250
SEQUENCE TYPE:nucleic acid
TOPOLOGY:linear
CLONE:HUMGS07116
SEQUENCE DESCRIPTION:
GATCTTCAAA ATCCAGGAGG GAACCGAGAT AACACAAGTA CAGGTGTGCA AAATTACTTA 60
GTAAAACTAT TTNTTCTATT ACTCCTTTTT TGCAAGTACA TACATTAAAT GATAANTTNA 120
TTGTGAAGTT AATAAANTGC CCATAGCTGT TCTATTACCT TCAGAAACTA TTTTCATAAT 180
TGANAAANAC TTGGNGTAAG TACATGTGTT ATATANCACC TACCCTAGNT GCTCAAGTGC 240
GTNNNGGAGN 250

SEQ ID NO:5995
SEQUENCE LENGTH:132
SEQUENCE TYPE:nucleic acid
TOPOLOGY:linear
CLONE:HUMGS07117
SEQUENCE DESCRIPTION:
GATCCTCCTT CCTCAGCTAC TCAGGAGGCT GAGGCAGGNG AATGGCATGA ACCCAGGAGG 60
CAGAGCTTGC AGGGAGCCAA GATTGTGCCA TGGCACTCCA GCCTCGGCGA CAGAGCAAGA 120
CTCCGTCTCA AA 132

SEQ ID NO:5996
SEQUENCE LENGTH:78
SEQUENCE TYPE:nucleic acid
TOPOLOGY:linear
CLONE:HUMGS07118
SEQUENCE DESCRIPTION:
GATCCTATAG GGAGGNCATT TCCTGTCCTG GAATTAGTAT TTCTAAAATG TGNATAAACT 60
TGTTTTATAA AAAGCAAA 78

SEQ ID NO:5997
SEQUENCE LENGTH:118
SEQUENCE TYPE:nucleic acid
TOPOLOGY:linear
CLONE:HUMGS07119
SEQUENCE DESCRIPTION:
GATCAGAAAG TGTTATTTAA CAGAAACTAC ATACATTTTT NATGTCTCTC TGGACCCTTT 60
GATTCTAAGT AAATTTNCAG TAAGTNAGCA GTAATAGACT ATCCTNTTCA NCTATGAN 118

SEQ ID NO:5998
SEQUENCE LENGTH:60
SEQUENCE TYPE:nucleic acid
TOPOLOGY:linear
CLONE:HUMGS07120
SEQUENCE DESCRIPTION:
GATCAAAAAG AGGAAACTTG AAATGTGATG GTGTTTATAA TAAAAGATGG TAAAACTAAA 60

SEQ ID NO:5999
SEQUENCE LENGTH:127
SEQUENCE TYPE:nucleic acid
TOPOLOGY:linear
CLONE:HUMGS07121
SEQUENCE DESCRIPTION:
GATCTCCTTC AGAATTTTTG TCTTGACTTT GAATCTTTGC CTGTTTGTCT AAACATTTGA 60
CTAACATTCT GTTTGAATTT GGAAGTATTC TAATACAAGA TTTGAATAAA GTTTATCCTT 120
AAATAAA 127

SEQ ID NO:6000
SEQUENCE LENGTH:195
SEQUENCE TYPE:nucleic acid
TOPOLOGY:linear
CLONE:HUMGS07122
SEQUENCE DESCRIPTION:
GATCCTGGAG CTGGAGCATG AGTGTCTGAC AATCAGAAGC ATGATGTCCA ATGTCNNGAT 60
GGCCAGAATG AATGTAATAG TTCAGACCAA TGCCTGNCCA CTGCTCCTTT ATGACTGCAC 120
TTCTAGCCAG TAGCTCTGCA CAAGTNAGCT CTGTAGAAGT AGGANCTTGG GCTTAAGTCN 180

TGNGCTNTNT CTCCN 195

SEQ ID NO:6001
SEQUENCE LENGTH:156
SEQUENCE TYPE:nucleic acid
TOPOLOGY:linear
CLONE:HUMGS07123
SEQUENCE DESCRIPTION:
GATCCCAGCA TGGGGTGAGC ATGAAAGAGT GGCAAACAGA GTGGCATAAG ACAGATAAAA 60
TACAAAAGGC AATTTACAAA GGACCAGGAC CGCAGAGGCA GAGATAAACC AGTGGGCTCA 120
GACTTCTGAG CGTCTCAGAN AAANNNAAAN AAANNN 156

SEQ ID NO:6002
SEQUENCE LENGTH:177
SEQUENCE TYPE:nucleic acid
TOPOLOGY:linear
CLONE:HUMGS07124
SEQUENCE DESCRIPTION:
GATCTGTCTA GCTTATTAAA GATGAAACTG AATTGGAAAA ATAGCTCCAT TTTTTGGTGC 60
TTGGGAAGCA CAGTGACCAA AAAAGTTGTA TGGCTGCTTA TTCATTAGTC TTTCCTACTG 120
ATGTCAAATC CATGGTACCT AGAGTTAAAT AAAATTCCAA TGCTCTTACT CTTTAAA 177

SEQ ID NO:6003
SEQUENCE LENGTH:305
SEQUENCE TYPE:nucleic acid
TOPOLOGY:linear
CLONE:HUMGS07125
SEQUENCE DESCRIPTION:
GATCCAGTTG GGACTAAGTG ACATCGTATC GGTTCCTGTT CAGATGACAT GGGGAAGATG 60
ATGGTTCAGC CACTGGTACT ACGAGAATGT TTGTATTACC CACATTTGAA TTNATTTGCT 120
ATGATTTTNA TGAAGATTAA AAATATATAC ACAGTTCCTG GTATGTTGAG GTTGTCTTTA 180
TTATTCTTAC TCAGAAAGGA AAATTTGTAA ATATGTTTGT GTGGCTCAAA CTGATACTAT 240
TCTTACANCT GGANCATTGA AAATNCAGAG GGCTGTATTT GGNTATTAGN GAGGNCTCCG 300
AATGN 305

SEQ ID NO:6004
SEQUENCE LENGTH:307
SEQUENCE TYPE:nucleic acid
TOPOLOGY:linear
CLONE:HUMGS07126
SEQUENCE DESCRIPTION:
GATCCTCAGG CTGGTATACG GCGCAATGTT GCATCAGCTN TGGGCAACTT GGGNCCTGAA 60
GGTTTGGGAG AGGAGCTGTT ACAGTGCGAA GTACCCCAGC GGCTCCTAGA AATGGCATNT 120
GGAGACCCTC AGCCAAATGT GAAGNAGGCT GCCCTCATTG CCCTCCGGAG CCTGCAACAG 180
GAGCCTGNCA TCCATCAGGT ACTGGTGTNC CTGGGTGCCA GTGAGAAACT ATCCTTGCTC 240

TCTNTGGGGG GNTCAGTCAC TGCCACACAG CAGTNCTAGG CCTTGNCTGT GCCAAAACAA 300
TTGNANN 307

SEQ ID NO:6005
SEQUENCE LENGTH:215
SEQUENCE TYPE:nucleic acid
TOPOLOGY:linear
CLONE:HUMGS07127
SEQUENCE DESCRIPTION:
GATCTCTCTC CGGCCCCCTA GAAGCCCTCT CCTCACTGCA GCCCTGGTGC TCTGGGGAAG 60
GAGGGGCATT GACAGGTGGT TTGACTGTTC ACCCAGCAGC CCCGCTTCCC CACTGGTTTC 120
TCTCCTGGCT GCCGGCGGNG GTAGGCTGGG AAAGCTTCCC CNTCACCTAG GNCGGGCCAG 180
CACCGGCCNG GGNCNNAAGA GGTCCNCTNN ATCCN 215

SEQ ID NO:6006
SEQUENCE LENGTH:273
SEQUENCE TYPE:nucleic acid
TOPOLOGY:linear
CLONE:HUMGS07128
SEQUENCE DESCRIPTION:
GATCCGAGGG GAAATNTTTA GAGCCCTCAG GAGGAGGAAG AGACCGAGTT TTAGGAAAAA 60
CATCAAAGCT GGATAGGTTG GGCAGAAGAG CTGGGGATAG CATTTAGAGA GGCTGAGAGT 120
CCTGGGTTCT GGNTTATCAC GTGTGAGNGA AACNGGAGGT TGCTANTTGT AGGTGGGCGT 180
CAGGNCTNGA GTGNTGCTTC CAGANGTTTC TGCAAGTGGG CCGGGTCTAA GNTAGGGCAT 240
GTTACGCCAG ANGTTTNAGG GNCACTAGGT AGN 273

SEQ ID NO:6007
SEQUENCE LENGTH:250
SEQUENCE TYPE:nucleic acid
TOPOLOGY:linear
CLONE:HUMGS07129
SEQUENCE DESCRIPTION:
GATCACAGGG AAAAACCGAC CGAGCTCAGG AAGCCTAATC CAGGTGGTAA CCACAGAAGG 60
AAGGACGGAA CTCACCCCAG CATATTTTTA GGCCTCATCT CAGTGCCTAT NAGGGGCCTG 120
CCAGNAAGGT CACTAACCTG GTCTCAGTGT GGCCTTGTCC AGCCTTGTGT TTNCTGTTAA 180
CCCCGNTTTG TGGTACGAGN TATTGNNTCC TATATTACGG ANACAGANCC NTGNTTTCGG 240
NTTTTGCATN 250

SEQ ID NO:6008
SEQUENCE LENGTH:106
SEQUENCE TYPE:nucleic acid
TOPOLOGY:linear
CLONE:HUMGS07130
SEQUENCE DESCRIPTION:
GATCCCAGTN AGTCATGAGC CTCAACCCCC TCCAGCCCAC TGGGGCTCTN ACCTCCACAT 60

GTGGGTAGAA GCTTNCCTGC CCCCTTTNCC TCCAGGAAGC CCTNAN 106

SEQ ID NO:6009
SEQUENCE LENGTH:108
SEQUENCE TYPE:nucleic acid
TOPOLOGY:linear
CLONE:HUMGS07131
SEQUENCE DESCRIPTION:
GATCGCCTGA GCCCATGAGG CCAAGGCTGC AGTGAGCCAT GGTCACGCCA CTGNATTCCA 60
GCCTGAGTGA CAGAGCAAGA CCCTGTTGAA AACAACAACA ACANCAAA 108

SEQ ID NO:6010
SEQUENCE LENGTH:335
SEQUENCE TYPE:nucleic acid
TOPOLOGY:linear
CLONE:HUMGS07132
SEQUENCE DESCRIPTION:
GATCTAAAAC TATTTTTCTG TATAAATATT ATTTGCCGAA AGTTTGTTTA TATTNAGAAG 60
TCTGACTATG ATGAATAAAT CTTAAATGCT TTGTTTAATT GAAAAACAAA AATCACCAAT 120
ATCCAAGACA TGAAGATATC AGTTCAACAA ATACTGTAGT TANGNGACTA NCTCTCCACT 180
TNTATGGGAA CTACATTTCA CTCTTGGTTT TCAGGATATA GCAGCACTTC ACCGAGATAT 240
TCTTTCAGCC ATANCACTGG TAACATTTCT NCTNAATCTN TCTGTAGCAC TTANAGGANT 300
TCCCTCATTC GNTNCCTTTC AGTGTTNNCG GGNGN 335

SEQ ID NO:6011
SEQUENCE LENGTH:112
SEQUENCE TYPE:nucleic acid
TOPOLOGY:linear
CLONE:HUMGS07133
SEQUENCE DESCRIPTION:
GATCCAAAGT AGCTGGGATT ACAGNCATGA TACCACANCA CAGTCATTTG AATNTATCAT 60
TACTTAGTAC TCNTATTTTT TTCCCTGCTG CTCTTTATGC ANTGAAGTCC TN 112

SEQ ID NO:6012
SEQUENCE LENGTH:279
SEQUENCE TYPE:nucleic acid
TOPOLOGY:linear
CLONE:HUMGS07134
SEQUENCE DESCRIPTION:
GATCTCCTTT TGATTAATTT ATAGAGTTTT TGATTATATG TCTTTGTATG ANCTTTTTAG 60
TTATTGCTCA AATATCACAT TTNATATTTA TAACACAGGC TACTGATGTT GACATTTTAA 120
CAGTTCAAGT GAAGTGTAGA AATCTTACCC ATTATCCCTC CCTATTAATN NNNTANTGGT 180
CTTAAATGTC TTTCCCTACA TATATTTNTA ACANGTATAT GAGATAGCCT TACAGTTTTG 240
NCTTTAACTA TCAATCATGC ATTAGGNNAC TCAAGNGGN 279

SEQ ID NO:6013
SEQUENCE LENGTH:119
SEQUENCE TYPE:nucleic acid
TOPOLOGY:linear
CLONE:HUMGS07135
SEQUENCE DESCRIPTION:
GATCTGGGAC TGGCAGGTTA TTAATCGAGA TACACTTGTT AGGAGGGACA GGGTTCCCCT 60
AAGGCACTTT TAAAGATACT CTGTAAGAAC CATTAACAAT AAACTTACTG TCAATCAAA 119

SEQ ID NO:6014
SEQUENCE LENGTH:315
SEQUENCE TYPE:nucleic acid
TOPOLOGY:linear
CLONE:HUMGS07136
SEQUENCE DESCRIPTION:
GATCGAGGTT GTTTGCAACG ACCGTCTGGG GAAGAAGGTC CGCGTTAAAT GCAACACGGA 60
TGATACCATC GGGGACCTTA AGAAGCTGAT TGCAGCCCAA ACTGGTACCC GTTGGAACAA 120
GATTGTCCTG AAGAAGTGGT ACACGATTTT TGGGGACCAC GTGTCTCTGG GGGACTATGA 180
AATCCACGAT GGGATGAACC TGGAGCTTTA TTATCAATAG ATGAGAATGC TCATCTTCCT 240
GNTGNGGGNT TTNCTNTTCG NTNCTGGATN CNCCACACTG GGGNTAGATG CTTGNTTGTA 300
AANNNCTTNC CTTTN 315

SEQ ID NO:6015
SEQUENCE LENGTH:218
SEQUENCE TYPE:nucleic acid
TOPOLOGY:linear
CLONE:HUMGS07137
SEQUENCE DESCRIPTION:
GATCCTAAGT AGAACCAGGT AATTGTCTCT TTTTCTAATA AGGAATTTGG GTAATTTTNA 60
ATTTTTNGTT TTTTAAAAAA TAACCTAGAC TATGCAAAAC ATCAAAGTGA ATTTCCCATG 120
ANTGTTTTTA ATATTCTCAT CTCAACATTG TGATATATGC TACTAAAANC CTTTNCATAT 180
ACATCTNACC TCATTTCAGG TGANTNATTN TAATCTNN 218

SEQ ID NO:6016
SEQUENCE LENGTH:53
SEQUENCE TYPE:nucleic acid
TOPOLOGY:linear
CLONE:HUMGS07138
SEQUENCE DESCRIPTION:
GATCAGAGCA GAACTAAATN AAATTAGAAT AAAGTTAAAC AATTAAAAAG AAA 53

SEQ ID NO:6017
SEQUENCE LENGTH:231
SEQUENCE TYPE:nucleic acid
TOPOLOGY:linear

CLONE:HUMGS07139
SEQUENCE DESCRIPTION:
GATCGACTTA TACCTATGCT AAAGTTAACG GTCTGAGAAG GTAGATTAGT GGTTGCCTAG 60
GGCTAGGGAG TGGGTTTGGG GGTGGGGAAT AGAAAGTGAC TGGTCATGGT TATAGGCTTT 120
TTTGTAGCAC CATAAAGATA GTTTAAAATT AGATTGTGGT GGTGATTGCA CCACCCTATG 180
ATTTGCATGG TATGTGANTT ATATCTTAAT AAAGCTGTTT AANANGTCAA A 231

SEQ ID NO:6018
SEQUENCE LENGTH:380
SEQUENCE TYPE:nucleic acid
TOPOLOGY:linear
CLONE:HUMGS07140
SEQUENCE DESCRIPTION:
GATCCGCTCC TAACCTGCGA CCGCAGCTGA CCTTCTCCTG CCACTAGCCC GNCCCTGNCC 60
TTAACANACG GGATGAAGTT TCCTTTTCTG TGCGCGGCGC TGTTTCCATA GGCAGAGCGG 120
GTGTNAGACT GAGGATTTCG CTTCCCCTCC AAGACGCTGG GGGTCTTGGC TGCTGCCTTA 180
CTTNCNNGGG GCTCCTGCTG ACTTCGGAGG GGCGGATGCA GAGCCCAGGG NCCCCACCGG 240
AAGATGTGTA CAGCTGGTCT TTACTCCATC GGCAGGGCCC GAGCCCAGGG ACCAGTGACT 300
TNGGCCTGGA CCTNCCGGTC TAACTGCAAG CATCTTCCCC AGGGCAAAGG NTTTNTGGGG 360
GAACGNGAGG TTTNATNTTN 380

SEQ ID NO:6019
SEQUENCE LENGTH:107
SEQUENCE TYPE:nucleic acid
TOPOLOGY:linear
CLONE:HUMGS07141
SEQUENCE DESCRIPTION:
GATCTATTTT GTGTGTTTTG AAACTTAGGT GCAATNTCCC CTGGAAAAAG CTAAAGAAAT 60
GTATATGTTC AATNACATTT TAAAATAAAA TATTATATAT ATGTAAA 107

SEQ ID NO:6020
SEQUENCE LENGTH:52
SEQUENCE TYPE:nucleic acid
TOPOLOGY:linear
CLONE:HUMGS07142
SEQUENCE DESCRIPTION:
GATCGATTTG ATAAAATTAA ACAAAGTGCT TTTAATGGAA AAAAAAAAGA AA 52

SEQ ID NO:6021
SEQUENCE LENGTH:355
SEQUENCE TYPE:nucleic acid
TOPOLOGY:linear
CLONE:HUMGS07143
SEQUENCE DESCRIPTION:
GATCCACTGT AATAGTCCAG TTTGGGGTGC AGACAAGTGT GAAGAACTTC TGGAAAAGAC 60

AGTGAAAAAC TGCTTGGCCC TGGCTGTTGA TAAGAAGCTG AAATCCATTG CATTTCCATC 120
CATCGGCAGC GGCAGGAACG GTTTTCCAAA GCAGACAGCA GCTCAGCTGA TTCTGAAGGC 180
CATCTCCAGT TACTTCGTGT CTACAATGTC CTCTTCCNTC AAAACGGTGT ACTTCGTGCT 240
TTTNGACAGC GAGAGTATAG GCATCTNTGT GCAGGANATG GCCAAGCTGN GCGCCGGNNN 300
NGGCTNGNGC AATGNCAGGA CCNGNTTGCA CCATGGTACC GNACCTTNAG TTTNN 355

SEQ ID NO:6022
SEQUENCE LENGTH:122
SEQUENCE TYPE:nucleic acid
TOPOLOGY:linear
CLONE:HUMGS07144
SEQUENCE DESCRIPTION:
GATCTTGGAG CTNCTGGAGA AGCAGGNCTG CNAGCTGCTC AACCGNTTCC AGGACTAGCC 60
CCCGCAGGCT CGTGCGCCAC CCCGTCCTGG TGAATAAACG CACTCCCTGT GCCTCAGACA 120
AA 122

SEQ ID NO:6023
SEQUENCE LENGTH:162
SEQUENCE TYPE:nucleic acid
TOPOLOGY:linear
CLONE:HUMGS07145
SEQUENCE DESCRIPTION:
GATCCGATAA TGTACCATGT GCCTGCTGGT TTGGCCTGTG CTGTTTCTGC TNTTTGCCCC 60
TNACTTCCTG TGCTAGAGAA AGCCTTATTG GTGTTGGTGG TGGTGATGGG AGGGAAAAGG 120
GTGGTCCTTT GGGACTCTTG ATAAAAATNT TATCATTTGA AA 162

SEQ ID NO:6024
SEQUENCE LENGTH:179
SEQUENCE TYPE:nucleic acid
TOPOLOGY:linear
CLONE:HUMGS07146
SEQUENCE DESCRIPTION:
GATCCAGGCC AATGCATGGC TATTGCTGTA AATCTTGATG TTTATTTCTG CCTTGTAAAG 60
TTCTATCACG GCCTACCTGG AATTTAAAAT TCAGTAGACA AATTAATTGG TCCTCTGCAC 120
AACTTTTTTA ATAAGTAGAT TATTTTNCAA NGGGNTTNGA NCAANTTTNA TTGANTCTN 179

SEQ ID NO:6025
SEQUENCE LENGTH:403
SEQUENCE TYPE:nucleic acid
TOPOLOGY:linear
CLONE:HUMGS07147
SEQUENCE DESCRIPTION:
GATCAGAGTG GCTGGAGCAG AGTGAATNAG GAGGGAAGCT GAGTAGCAGG TATCTGAGAA 60
GCAATGCAGC CCGCAGCTTA ACTTCAACAT GCTTTTAAAC TTTTTNCCCA TTCTNTTGAG 120
TTTCAAGATA TAACCTTGAG GAAACTCAAA ATCCTTTCTC CTTAGCCTTA AAATAGACTC 180

CACGTCCCTC CCCTTTNTCA CCAGATATGC TCCCTTTNCA TTTATCTAAC TGTATGCTAC 240
TATTTAATTG TGTGATGTCT TAGANGTTTC AGTGGCTAAT CTTGAGACAG ACCAAGNCTG 300
GAGACCCAGC TGGCAANCCT TCCANNATTT ANCTTCAAAA TTGGCTAATT NANCAACCNG 360
GCCCTTNGTT NNAAATTATG GTNATTCCGG GGGNCCCGGN TGN 403

SEQ ID NO:6026
SEQUENCE LENGTH:198
SEQUENCE TYPE:nucleic acid
TOPOLOGY:linear
CLONE:HUMGS07150
SEQUENCE DESCRIPTION:
GATCTTGCTG CAGCCACAGT GCAGTCCACA TTAACTCTAC AGACCAAACC ATTTGTATCT 60
GGCATCACTT ACTAACACAC GACATGCAGA AAAGCCGCAT GTNGTGTGTT AGTATACANG 120
AAGGAATAGA AAACTGATAC TGTTTTAAAT ANTCTGTAAT TTCAATTTTT TTNTTTTTNN 180
GCTGAANTGG GTCNTNTN 198

SEQ ID NO:6027
SEQUENCE LENGTH:190
SEQUENCE TYPE:nucleic acid
TOPOLOGY:linear
CLONE:HUMGS07151
SEQUENCE DESCRIPTION:
GATCATAACT CTTTNTTGAA CTAGAGTATT TTTGCAGCAT TCCTTGTCAT CAGAAACATG 60
GTTAAAGTTT AAAACTAGAA GCAGCAGAAA ACTAGCTTGT AAAATTTATC CAAGTAGAGT 120
GCAGGCTAGG CTGTCTTGGG GAAATAAACA TTAAANCTTA AAGCAATGTT TTACATGGAA 180
ANNNNANNNN 190

SEQ ID NO:6028
SEQUENCE LENGTH:217
SEQUENCE TYPE:nucleic acid
TOPOLOGY:linear
CLONE:HUMGS07152
SEQUENCE DESCRIPTION:
GATCTTGTGN CATTNTTGAC CTTGGACCTG CTTGGTTAAG GAGGGAGTGG GCCAAACCAG 60
AGTGCCAGGA GCTAATGGAG CCAGGCCTGA CTCCTAGGAG TGGTCCAAAG GCCTTCAGCC 120
TAGATGGTGC AAAGNTGGGG CCAGCCTGTT TTCACCGGNA CCNGNGCCTG TGACACCAAG 180
ACCCACCCCA ATCCCAGNNT TNACACAGTA TTNTCCN 217

SEQ ID NO:6029
SEQUENCE LENGTH:136
SEQUENCE TYPE:nucleic acid
TOPOLOGY:linear
CLONE:HUMGS07153
SEQUENCE DESCRIPTION:
GATCGGAGCC TACTTCCCTC CGCTCAGCCT NGAGGAGCTG AGNCCCTACT TCAGGGACCG 60

TGGGTGGCCT CTGCCCGGGC CCCTCTAGAC GTGCACCAGA AATAAAGGCG AAGACCCAGC 120
CCCTCGGCGG CTCAAA 136

SEQ ID NO:6030
SEQUENCE LENGTH:278
SEQUENCE TYPE:nucleic acid
TOPOLOGY:linear
CLONE:HUMGS07154
SEQUENCE DESCRIPTION:
GATCCAAACC CAAGTCTTGA GCAATGTTTT TCTCAAAAAG CTGCTATCCA ATNATATAGG 60
AAAATACATT GTGTTTTCCT AAACACACTT TTCTTTTTAA ATGTGCTTCA TTGTTTGATT 120
TGGTCCTGCC TAAATTTCAC AGGCTAGGNC AATGAAGGCT GAATCANNGN CANTTCATCC 180
ACCAATATCA TGTGTAGATA TTATGTATAG AAAGTANAGT AANTTATGGC TCTAACTTCT 240
GTGTTGCTGT NTATCCTTGN TNTTTNTCGG GGGTGNTN 278

SEQ ID NO:6031
SEQUENCE LENGTH:65
SEQUENCE TYPE:nucleic acid
TOPOLOGY:linear
CLONE:HUMGS07156
SEQUENCE DESCRIPTION:
GATCAGTTGA TGTACACTTG TATTATTAAA GCACTCAATA AATCACTGTG GCTGATAACT 60
GCAAA 65

SEQ ID NO:6032
SEQUENCE LENGTH:161
SEQUENCE TYPE:nucleic acid
TOPOLOGY:linear
CLONE:HUMGS07157
SEQUENCE DESCRIPTION:
GATCCACATG GCACGGTGAC TGTAGTTAAT GCATTTTATA TGTCAAAATT CCTTANATAG 60
TAGATTTTAA ATAATCTCAC CACAAAAAAG TAGATGTGTT GGTGATGGAT ATCTTANTTN 120
GCTTAANGTA NTTGTNTCAC CAATGTATAC ATNTATCAGN N 161

SEQ ID NO:6033
SEQUENCE LENGTH:130
SEQUENCE TYPE:nucleic acid
TOPOLOGY:linear
CLONE:HUMGS07158
SEQUENCE DESCRIPTION:
GATCATCTTT ACAGTTCCTC GGGAAAATNT GAATGTCCTG CGTTTTGTTT TCTTTACTGT 60
ATGAAAACAG GAAAATAAAA GAGAAATTTA GAAAATACAG CTCATTACAA TAAANNTNTT 120
GGNTTTCAAA 130

SEQ ID NO:6034

SEQUENCE LENGTH:165
SEQUENCE TYPE:nucleic acid
TOPOLOGY:linear
CLONE:HUMGS07160
SEQUENCE DESCRIPTION:
GATCTGGCCC AAGCTGGGCT CCCTNNAAGC CAAGAGAGCC TCTNATNGAC CCCGCCTGCC 60
CGAATGAAAT CCGAACAGTT GGGGCTGTTC ATGGCAAGTG GGGCTGGTTT TTCATNTCCA 120
TTGGTTATTT AAAGTTTCCN TTAAAATAAA CGATTTTAAG TTAAA 165

SEQ ID NO:6035
SEQUENCE LENGTH:77
SEQUENCE TYPE:nucleic acid
TOPOLOGY:linear
CLONE:HUMGS07161
SEQUENCE DESCRIPTION:
GATCTGGGGT CTGTTTTGTT CATTACTTTG TTCACTCCTT CTTCCATTTA TTCATTCAAT 60
AAACATTTAT TGGGAAA 77

SEQ ID NO:6036
SEQUENCE LENGTH:310
SEQUENCE TYPE:nucleic acid
TOPOLOGY:linear
CLONE:HUMGS07162
SEQUENCE DESCRIPTION:
GATCCGGCTT GCCCAGAAAG AGAAGACAGC CATGGAAGTG GAAGCCCCTT CAAAGCCAGC 60
CAGGACTAGT GAACCACAGC TCAAAAGGCA AAAGAAGACA AAAGCCCCCC AGGATGTAGA 120
AATGAAGGAC CTTGAAGATG AGAGCTAAAC CTCTTCCACT AGAAGATTCT CAACTGGAGC 180
CAGCCTTCNG GCTCAGTGGT TGTTTCAGAG GACTTTGACA AAAGCAAGGC CCCTTTTCAC 240
TCTCCAGATT TCCTCCTACC TAATGGCCTA CTGACCTCCC CTTAGAGGGA TGTNTTTGGG 300
NGGGAAGNTN 310

SEQ ID NO:6037
SEQUENCE LENGTH:288
SEQUENCE TYPE:nucleic acid
TOPOLOGY:linear
CLONE:HUMGS07163
SEQUENCE DESCRIPTION:
GATCCAGAAC ACTTCAAGAA CTCGTCAAAC AGCTCGATAA GCCTTTTTGA CTGTNTACAT 60
CTGTACCGGG AATAACATTC CTAGGCTGAA ATTTCCACAA AGANTAGAAC CTGTACCCAG 120
TTCTTCAGGC TGATTTCCCT GACCTCTTGG GCATTTGTAT TTGTAGTAAA GTATTGCAGA 180
GATTCCTAAG TATTTTTNNA GCAGCCATNA AAAATTGGNC TTTGTNTTNG TTGATTCATN 240
NGGAGGGCAA GTTGGGGANT AGACTTCAGT NGANCTCGGT ATGGNTGN 288

SEQ ID NO:6038
SEQUENCE LENGTH:51

SEQUENCE TYPE:nucleic acid
TOPOLOGY:linear
CLONE:HUMGS07164
SEQUENCE DESCRIPTION:
GATCACGCCA CTGCACTCCA GCCTGGGCAC CAGAGTGAGA CACTGTTTAA A 51

SEQ ID NO:6039
SEQUENCE LENGTH:195
SEQUENCE TYPE:nucleic acid
TOPOLOGY:linear
CLONE:HUMGS07165
SEQUENCE DESCRIPTION:
GATCTGGGCA GGGAACATCA CATACCAGGG CTGTCTGTCG GGGGGGTGGA GGGCTGGGGG 60
AGGGATAGCA TTAGGGGAAA TACCTAATGT AAATNACAAG TTGATGGGTG CAGCAAACCA 120
ACATGGCACA TGTATACCTA TGTAACAAAT CTGCATGTTG TGCACGTGTA CCCTAGAACT 180
TAAAGTATAA TTAAA 195

SEQ ID NO:6040
SEQUENCE LENGTH:75
SEQUENCE TYPE:nucleic acid
TOPOLOGY:linear
CLONE:HUMGS07166
SEQUENCE DESCRIPTION:
GATCAGGCCC CACCTTGTGT CTACCCCCAT CCCNGCTGTG AACGTGACAN TGAATAAAGT 60
CGGGGAAACG CGAAA 75

SEQ ID NO:6041
SEQUENCE LENGTH:315
SEQUENCE TYPE:nucleic acid
TOPOLOGY:linear
CLONE:HUMGS07167
SEQUENCE DESCRIPTION:
GATCTGAATT GAGGAACTTG TAGTTTTTGC TCTCCCACTT CTTGGAAACC CCTATCTACT 60
TTCCTATGTA TTTGACTACT TGAGGTAGCT TATACAAGTA GAATCATACA AATGTATTGT 120
TTTGTGACTA GCTTACTTCA NTTACAATAA GTCCTCAAGT TCTNCCATGT TGCTGTGTGT 180
GTCAAAATTN CCTTCCTNGC TAAGGCTGAA TAATATTNCA TNGTATATAT ATGNCNTTNA 240
TCCAGTCATT CATCAATGAN TACNTGTTTT TNGTGTGTGG TGTTGGAANC CTNCTGGGGA 300
CATGGTNATA TTTTN 315

SEQ ID NO:6042
SEQUENCE LENGTH:79
SEQUENCE TYPE:nucleic acid
TOPOLOGY:linear
CLONE:HUMGS07169
SEQUENCE DESCRIPTION:

GATCTAAGCT GATTTAAATC TAAAAACTGG ATAAGAAGCT GTGGCTTGTA ATTAAAAATA 60
GACCTTTATT CACTAGAAA 79

SEQ ID NO:6043
SEQUENCE LENGTH:189
SEQUENCE TYPE:nucleic acid
TOPOLOGY:linear
CLONE:HUMGS07170
SEQUENCE DESCRIPTION:
GATCAGGTAT TTAAAGGACA TCTGACAGTG CCTGGCACGG GATAGGTGCT CCATAAATGC 60
CTGTAGCTAT AATTATTACT GTNCCACTAG CTGTTTACTT AAGTAACATT GAATTTCCAC 120
AGANTGCATT TCGAATCAGG TGGACTATGA TTATACTGAT TNTNAAAATA GNGAGCTGCA 180
TCTGTTAAA 189

SEQ ID NO:6044
SEQUENCE LENGTH:151
SEQUENCE TYPE:nucleic acid
TOPOLOGY:linear
CLONE:HUMGS07171
SEQUENCE DESCRIPTION:
GATCTGGAGT GGAAGGGGAA TTCATTTGTT CATTGTCTAT CCTTTTGTAT TGATTGAATT 60
TTTNATATAT ATATGTAAAT TTNCACAATA AAATTTTTTN CCAAAATAAA ATAAACAAAA 120
GGGGCTTTTT GCAACCCAAT TCCTATCTAA A 151

SEQ ID NO:6045
SEQUENCE LENGTH:178
SEQUENCE TYPE:nucleic acid
TOPOLOGY:linear
CLONE:HUMGS07173
SEQUENCE DESCRIPTION:
GATCATAGCA CTNTATCATC TTAAAAGTTA ATAATAGATT ACTTGTAACA CAGGCTCACT 60
ATGNGAGAAT TCATCAAGCT GTATGTACAT TTATTGTGCT CTTCATTGTA TGTATAANTC 120
AATAAATTGA AACAGTAAAC GAAAAGGTAA TTNTAGANAN CCTACAGTGA TNCTTGNN 178

SEQ ID NO:6046
SEQUENCE LENGTH:380
SEQUENCE TYPE:nucleic acid
TOPOLOGY:linear
CLONE:HUMGS07174
SEQUENCE DESCRIPTION:
GATCCGTCAC AGACTAAGGA GATGGCAGGC ATTGACAGCT TCACTCCATG AAGGCCATCT 60
CTGTTTCTNT CCTCCGCTTA ACCAAGCTGT TGTGGTTTTT NAGCATAGTG TTGTATGTTC 120
CATTGCTAGC TGTCCTGCTG TTTAACACAG TGTTGTATTT TTTTNCTAAA TGTACATAAT 180
TAGAAAAGAA GATAACAATA GGANGCTATG TGTATCTCCT GTGTAAAGNA GTGGCTTCAC 240
TGGAAAAATG GTGTGGCTAG CATTTCCCTT TGAGTCATGA TGACAGATGG TGTGAAAACC 300

ATCTAAGTTT GCTTTTGACC ATCACCTCCC AGTAGCAATT NTGCTTTCAT AATCCNTTTT 360
AGCANTCCAG GCCTCTTTGN 380

SEQ ID NO:6047
SEQUENCE LENGTH:72
SEQUENCE TYPE:nucleic acid
TOPOLOGY:linear
CLONE:HUMGS07175
SEQUENCE DESCRIPTION:
GATCGTAATG TAAAATTCTT TTACCATGTC AAGAAATTAT TAAAAATACA GGTACTTTGA 60
CCTCTTTCTA AA 72

SEQ ID NO:6048
SEQUENCE LENGTH:226
SEQUENCE TYPE:nucleic acid
TOPOLOGY:linear
CLONE:HUMGS07178
SEQUENCE DESCRIPTION:
GATCCTGAAA AGATAGGGCT ATTGTCCCCT GCCTCCTTGG TCACTGCCTC TNGCTGCACG 60
GNCTCCTNAG CCCACCCCCT TGGGGCACAA CCTGCCACTG CCACAGTAGC TCAACCAAGC 120
AGTTGTGCTG AGAATGGCAC CTGGNGAGAG CCTTCTGTGT GCCAGNTTTT GTTCTGAGTG 180
CTGTACATGT ATTANNTCCT NTACTGCTNG CCACATTGTA CCNNTN 226

SEQ ID NO:6049
SEQUENCE LENGTH:225
SEQUENCE TYPE:nucleic acid
TOPOLOGY:linear
CLONE:HUMGS07179
SEQUENCE DESCRIPTION:
GATCCCCGTA CCACAGTGCC AGCCATGCCC TTCCCCTGGG CTACCATTGT CCCTTTCCTC 60
ACCCAGTTGG TAGAGGAGTC AGGAGGTGGG AGGCCGTGGG CTTTGGTTTT ATAATGTAAC 120
CACTGTGGGG GTGGGGGAGG ATGGTGAACC ATGTATTTNA GTGAAATATT TAATATATTT 180
AANTATCANT AAANTCAAAC TCTTTGTAAA ANANANNNAG NANGN 225

SEQ ID NO:6050
SEQUENCE LENGTH:332
SEQUENCE TYPE:nucleic acid
TOPOLOGY:linear
CLONE:HUMGS07180
SEQUENCE DESCRIPTION:
GATCATCCTG GGCAGCAAAG TCATACACTC TTGAGGGAAG AGAGAGACCT TCTCATATTG 60
TTTTATATTG TTTTATACTC AGTACCTGTT TTAAGAAAAA AACAAGGAAG TGAAATCAAA 120
GACAGGCAGC CCGGCACCAG GCCTGANACC AGCCCTGGGC CTGCNTGGCC TAAACCTAGT 180
AGTTAAAATT CAACTTACGA CTTAGANCCT GATGTTATCC GTAGATTCCA AGCNTTGTAT 240
AAAANATTTG TGANACTCCC TGTTGTGTTC TGTGCCAGTG CATGNNACCC CTGTCACATA 300

TCCCCTAGAT TGCTCANTCA NTCNCGGTTT GN 332

SEQ ID NO:6051
SEQUENCE LENGTH:259
SEQUENCE TYPE:nucleic acid
TOPOLOGY:linear
CLONE:HUMGS07181
SEQUENCE DESCRIPTION:
GATCACAGGC CTTTGGAGCA CTTTTACTCT CTGAGAAGAA CTGGAGCTAG AGATGTAAAA 60
TGGACAGTCT TGATGGGGTT GAGAACCTTC TGGGGAGCCA GATGACCCTC TCTTTGCACA 120
ATAGATAAAA GTCTTTATAT GANTATATAT AANTTTNTTT NTTTTTCCCT TCCTGTGGGA 180
TTTCTGGAGA ATGAGAATTA TCCAAATNCT CAGTCTACCT GNGATAGTAA ATNCATGGCT 240
TATGCTTCTG GTCCTTAAA 259

SEQ ID NO:6052
SEQUENCE LENGTH:92
SEQUENCE TYPE:nucleic acid
TOPOLOGY:linear
CLONE:HUMGS07182
SEQUENCE DESCRIPTION:
GATCGNACCA CTGCACTCCA GCCTGGAAGA CCAAACGAGA CTCTGTATCA AAAAATAATA 60
ATAATTAATA AAGTTTTACT GGAAAAAAGA AA 92

SEQ ID NO:6053
SEQUENCE LENGTH:92
SEQUENCE TYPE:nucleic acid
TOPOLOGY:linear
CLONE:HUMGS07183
SEQUENCE DESCRIPTION:
GATCAGCTAC AATGCCCTGT GTTAAATTNT TTAAAAGTTT CCCTTTTCTT TTTTGCCAAT 60
AAAGTTGTAA ATAAAGACCA TCATACATTA AA 92

SEQ ID NO:6054
SEQUENCE LENGTH:95
SEQUENCE TYPE:nucleic acid
TOPOLOGY:linear
CLONE:HUMGS07185
SEQUENCE DESCRIPTION:
GATCCCTTCC CGCCACACGA TGCTCCGTTT TCTTCCGTTG TGAATNCCGC GTCCTGTCCT 60
GGTGACAGGA GAACAATNTT GGTGAACGTC GCAAA 95

SEQ ID NO:6055
SEQUENCE LENGTH:155
SEQUENCE TYPE:nucleic acid
TOPOLOGY:linear

CLONE:HUMGS07186
SEQUENCE DESCRIPTION:
GATCTCCTTC TCACTGCATT CACTCGGTTC CCATAGCCCA AGTCATATTG TNTCTCTCCT 60
GGACAGTAAT AACCTTTTNT TGGGCTCCCT GCTTCCAACC TTGCCTTCTG TAGTGTACTT 120
TCCAAATAGA AGCCAGAGTG ATACCTTATA TTAAA 155

SEQ ID NO:6056
SEQUENCE LENGTH:138
SEQUENCE TYPE:nucleic acid
TOPOLOGY:linear
CLONE:HUMGS07187
SEQUENCE DESCRIPTION:
GATCAGGAGG CCCACTTGGA TTTATAGTAT AGCCCTTCCT CCACTCCCAC CAGACTTGCT 60
CATTTTCCGA GTTTTTAACT AGACTACACT CTATTGAGTT TAATTTTNTC CTCTAGGATT 120
TATTTCTGTT GTCCTAAA 138

SEQ ID NO:6057
SEQUENCE LENGTH:158
SEQUENCE TYPE:nucleic acid
TOPOLOGY:linear
CLONE:HUMGS07188
SEQUENCE DESCRIPTION:
GATCTGAAAA CCAAGTGGTG ACCTAGGGAG GGAACAAGCN CTGTGCAGCA TTGATGAAAC 60
TTAAAAGATG AAGTCCTGGT CCGGGCACGG TGGCTCACTT CTNTAATTCC AACACTTTGG 120
GAGGCCGAGG CAGGAAGATG GCTTCANCCC ACGACAAA 158

SEQ ID NO:6058
SEQUENCE LENGTH:151
SEQUENCE TYPE:nucleic acid
TOPOLOGY:linear
CLONE:HUMGS07192
SEQUENCE DESCRIPTION:
GATCTNTGGC CCACTCCGCA TAAATCAGGT GCATAATGTA GCTGCCCGGG CTTCTATACT 60
GCAACATTCG CAAGGCAATT AGCTTGAAAA CTGAATTGAT GTACATTGAT TTTTTAATA 120
AAGTTATTGT AATATATTCA CTCGTTATAA A 151

SEQ ID NO:6059
SEQUENCE LENGTH:362
SEQUENCE TYPE:nucleic acid
TOPOLOGY:linear
CLONE:HUMGS07193
SEQUENCE DESCRIPTION:
GATCTATACC CTAAGCAGAT AGCAAAGAAG ATAATGGAGG AGCAATTGGT CATGGCCTTG 60
GTTTCCCTCA AAACAACGCT GCAGATTTNT CTGCACAAAC ATCTCCACTT TTGGGGGAAA 120
GGTGGGTNGA TTCCAGTTCC CTGGACTACC TTCAGGAGGC ACGAGAGCTG GGAGAAGAGG 180

CAAAGCTACA GGTTTACTTG GGAGCCAGCT GAGAAGAGAG CAGACTCACA GGTGCTGGTN 240
CTTGGTTTTN GCCAGGCTCC TCCGAGCACC TCATGCATGT CCCAGCCCCT GGGCCCTAGC 300
CCTTTCCNGN CCTNCNGTCT NCAGTGCCAG NACGGAANTN CCTTGNACCA NNGGNTNTTN 360
GN 362

SEQ ID NO:6060
SEQUENCE LENGTH:149
SEQUENCE TYPE:nucleic acid
TOPOLOGY:linear
CLONE:HUMGS07194
SEQUENCE DESCRIPTION:
GATCCATCAT NATGTAAAAN TTCACAATAT GGTTCAAATG TAACANTGCA GAATTGAATA 60
TGGAGGCATG CATAACCTTC CTCTTAGAAA ATGGCAGGTN TTGTAATTTC AAATTTTTNT 120
GCAATTAGAT TAANTCATAA TGCAACAAA 149

SEQ ID NO:6061
SEQUENCE LENGTH:184
SEQUENCE TYPE:nucleic acid
TOPOLOGY:linear
CLONE:HUMGS07195
SEQUENCE DESCRIPTION:
GATCTTGCTT TAACTTTCAA CACTTAGAAA ATCTACAAAC ATTCAGACCT GTCTGGGTTG 60
GTATTGCCAC CCATGACATT TAACATGTTG TGATGCTTGA AAACACAGGA GTAGAGAAAN 120
TCGATGANGA TTGTATTTTN NCACCTTAAC TCCACATTGC TTTATTGGNT AATTNATANN 180
CTNN 184

SEQ ID NO:6062
SEQUENCE LENGTH:196
SEQUENCE TYPE:nucleic acid
TOPOLOGY:linear
CLONE:HUMGS07196
SEQUENCE DESCRIPTION:
GATCAGTAGT CTCTATTCAA ACTTTNAAAA TGTCGTGGTA TTGTAACAAT ATATTTAATG 60
AAAGANGGTT ACAGACTCCC CTGAAGAACC AGCTTTCCTA CGCTTTTNAT TTTNCTAACT 120
TGTCTAACCT GATTTNAAAA TGACTGCANT TCCAGACTAA AAACATGCTT CANCCCTGTT 180
TCANGACATT NTGCTN 196

SEQ ID NO:6063
SEQUENCE LENGTH:295
SEQUENCE TYPE:nucleic acid
TOPOLOGY:linear
CLONE:HUMGS07199
SEQUENCE DESCRIPTION:
GATCCAAACT CTACATCCAG AGATTATGAA AAATTCTTAT AGAATTTTGT AACAAGTATT 60
TACATGTTGG GTGAAAGANA TTTCATAGTC GTTGGAGTGC CATGAAATTA ATACTTGCAA 120

TTCAGATTGC GGTAGTTTAC ACTTTTCCTG TATGTTTCAA ATCAGGTGTG TACCATTTGT 180
NCTGAGANCA CCACAGANTG ATTTATCCAA AGTCCATTGA TTTTNATACG TGTTTTGGTT 240
TGTNACCAAN TTATNGTNAT TCNTNGCACA TTTTTGGNAA TTNATTGTAT AGGNN 295

SEQ ID NO:6064
SEQUENCE LENGTH:312
SEQUENCE TYPE:nucleic acid
TOPOLOGY:linear
CLONE:HUMGS07200
SEQUENCE DESCRIPTION:
GATCTTCGCT GATTNNNTCC ATCAATNACC CGGAGCATCC ACTNACGCTA GAGGAGTTGA 60
ACGTAGTAGA GCAGGTGCGG GTTCAGGTTA GCGACCCCGA GAGTACAGTG GCTGTGGCTT 120
TCACACCAAC CATTCCGCAC TGCAGCATGG CCACCCTTAT TGGTCTGTCC ATCAAGGTCA 180
AGCTTCTGCG CTCCCTTCCT CAGCGTTNNN NNGATGGACG TGCACATTAC TCCGGGGACC 240
CATGCCTCAG AGCATGCAGT GAACAAGCAN CTTTGCAGNT AAGGNGCGGG TGGCAGCTGC 300
CCTNGNGAGC AN 312

SEQ ID NO:6065
SEQUENCE LENGTH:108
SEQUENCE TYPE:nucleic acid
TOPOLOGY:linear
CLONE:HUMGS07201
SEQUENCE DESCRIPTION:
GATCAGTGCT CTTCAAGGTG TCAAGATTAT CAAAGACATA AAAGANTGGN TGANCTGCCA 60
TAGATTGGNG GAGACAATGC AATGTGAAAT CCTGANTTTG ACCCTGAN 108

SEQ ID NO:6066
SEQUENCE LENGTH:103
SEQUENCE TYPE:nucleic acid
TOPOLOGY:linear
CLONE:HUMGS07202
SEQUENCE DESCRIPTION:
GATCTTTAGT CCGAGTATGN GAAATTCAAG CAACTTTGCG AGANAAAGGA ATACTATTTT 60
TNAGNCAACA AATTAAGGAA CTTGAAAAGC TAAAAANTCA GAN 103

SEQ ID NO:6067
SEQUENCE LENGTH:127
SEQUENCE TYPE:nucleic acid
TOPOLOGY:linear
CLONE:HUMGS07204
SEQUENCE DESCRIPTION:
GATCANNGAA ACTAAACATT ACACCTTCTG GNCTTTNATT TATGAAGTGG CACAGAATCT 60
AACTTTNCAC TGAATATCTT TCCATTCACA TCTCCTCTGC CTTAGTCACA ATGGCAACAG 120
TACAGNN 127

SEQ ID NO:6068
SEQUENCE LENGTH:136
SEQUENCE TYPE:nucleic acid
TOPOLOGY:linear
CLONE:HUMGS07206
SEQUENCE DESCRIPTION:
GATCTCCCAA GNAAGCACAA ATAGTTTTTT TCGCTAACTT AGTTATGAGT GAAGCCTCTG 60
TNCACTTATA ACTNGCCAGT TTCATTGGTG GAATAAGTCC CCTTACTCAT GATTCANCAA 120
TATNCCTATA TTANAN 136

SEQ ID NO:6069
SEQUENCE LENGTH:108
SEQUENCE TYPE:nucleic acid
TOPOLOGY:linear
CLONE:HUMGS07209
SEQUENCE DESCRIPTION:
GATCAGAGAA GGTTTTGTTT GTCCTTTTAA TCTTTACTTC TTTGAATTGT GAAAGTAATA 60
CTACATATTC ATAATAAATT CAAATAATAC AGAAGTATAT TATGTAAA 108

SEQ ID NO:6070
SEQUENCE LENGTH:120
SEQUENCE TYPE:nucleic acid
TOPOLOGY:linear
CLONE:HUMGS07210
SEQUENCE DESCRIPTION:
GATCCGTGCT GTCCAATCCG GTAGCCACAC ACGGCTCTCG GGCACCGCAC AGTGGCTAGT 60
CTGAAACCTG ACATGCTCTA AGTGTAAAAT ACATGGTGGA TTTTNAAGAC TTGAACCAAA 120

SEQ ID NO:6071
SEQUENCE LENGTH:174
SEQUENCE TYPE:nucleic acid
TOPOLOGY:linear
CLONE:HUMGS07211
SEQUENCE DESCRIPTION:
GATCCTGGCC CTCCACCTNC CTCCAGGCCA CGAAATGGGA ATNCCAGCAC TAAGCCAGGC 60
ACCGGGCAGA AGCTGGGCCT TCCGCCTCCN TTGGATGGGG TCAAGAGGCC ANGCCTGGNA 120
CATTTTGGNG TGTCCTGGCT ACCAGCTCTC ACCTACANCC ACGCACCTCT NTAN 174

SEQ ID NO:6072
SEQUENCE LENGTH:186
SEQUENCE TYPE:nucleic acid
TOPOLOGY:linear
CLONE:HUMGS07212
SEQUENCE DESCRIPTION:
GATCTAGATA TNAGGTTTTT CTCCTTCATT CTCAGCTGTC GAAGAAATCA AAGTAGCATA 60

TGCACAAGGT TAAAAACCAC ATATACAAAT ACTATAGAAC AGCTTATANT GAAAACCTTG 120
CCTGCCTTTA TANAANNTGT GATTATCTTC TTCTGTTAAT GTCAGTAANN GATGGTTTGT 180
CCTAAA 186

SEQ ID NO:6073
SEQUENCE LENGTH:229
SEQUENCE TYPE:nucleic acid
TOPOLOGY:linear
CLONE:HUMGS07213
SEQUENCE DESCRIPTION:
GATCTGAGAG ATGATACATG ATACCAGATG AAAAGAAGGA GAAGTGTGTA CCATATGTTT 60
TNAGCAGAGG NCCCTCCAAC TTATGGCATC AGGGGCAAAA AGTCACAGCT TATCCCAGGC 120
ACCCTGGCAG GTTCTCAGAG CCTGCCTCCT CCCTGTTTAT ATGCGTACAG CCTGGTAACC 180
CCCAGGCATG CAANTATACA GTCTGTAACA ACAGGNNNTT NNTNNNTTN 229

SEQ ID NO:6074
SEQUENCE LENGTH:50
SEQUENCE TYPE:nucleic acid
TOPOLOGY:linear
CLONE:HUMGS07214
SEQUENCE DESCRIPTION:
GATCTTAAAA CTGAATAAAA ATATTTAATA AAACTTTGAA ATATTTTAAA 50

SEQ ID NO:6075
SEQUENCE LENGTH:297
SEQUENCE TYPE:nucleic acid
TOPOLOGY:linear
CLONE:HUMGS07215
SEQUENCE DESCRIPTION:
GATCCATTCT ATCCATCCGT TATGTGGCTT TGCCATCCCA GCTTGGAGTG TCTTTACAAA 60
GATAATAACA GTTGTTTTCT TTGCTCTCGT TTTGGATGCA TAGACTGAAA AATTAAAACA 120
AATAACTTGT AAAATGGCTT GTNAAAAAAT ACAATTACCT CTAATTAGTA GTACGCGTAA 180
NTNTTTTACA GAATGANAGG CGTGCTTTNN ATTTCCTTAC TGCGTTACAT TGGTGGCGAA 240
AGANGTCTGT ATGANANTCA GTNCTTNTGC TGACACAAGN TCCATTTGGT NACANAN 297

SEQ ID NO:6076
SEQUENCE LENGTH:191
SEQUENCE TYPE:nucleic acid
TOPOLOGY:linear
CLONE:HUMGS07216
SEQUENCE DESCRIPTION:
GATCCTCAAT CAGTGGGTGG AGGATAACAG GGTAGATTCA CTTGTTTGCA CTTNTACAGT 60
TGTAGCTGCG AGTCCAGAAG TCCTCTAGAG CATGTTTACT GGCACTGAGT TGGTGAGACA 120
GTTGTGGAGT ATCCCATTCT GATGAGTACA GAAAAAAAAA GAAAAAGAAA NGANGAGAAA 180
AANTTANGAA A 191

SEQ ID NO:6077
SEQUENCE LENGTH:235
SEQUENCE TYPE:nucleic acid
TOPOLOGY:linear
CLONE:HUMGS07217
SEQUENCE DESCRIPTION:
GATCCGAGCG TCGTGAACTC ACCTGAACTT TGGAAGGTTG GCACTGGGGG GCCGCATCCC 60
CCTGGGAAAT NACTGAACGC GGGGTTTCAN TGGGTCCCGC CTGGGTCTAA GGACCCAGTT 120
TCTTCTGTNA GCACGGGCAG TGGGAGGCCG GTGGAAAGGC GCAGGGGCCC ACCTACGCGT 180
CTCACATCTT CAGGGGCGCA TTTCTACAAT AAAAACTCAC CCAATTAGAC CTAAA 235

SEQ ID NO:6078
SEQUENCE LENGTH:181
SEQUENCE TYPE:nucleic acid
TOPOLOGY:linear
CLONE:HUMGS07218
SEQUENCE DESCRIPTION:
GATCTATTGG ACCAAACCTT CTGCACACTC GGCCAGTTCC CTCTCCAATG TCCGGTGCCA 60
TCTTTCCTGA CCTTTGTTTC TTTCTGTTCA GGAACCATCA GTCCCCTTGT AATAAAGGTG 120
GTAGATTTCA TTGAGGTTTT AGATTGAAAC TTTGAATAAA TCAAAAATAC TCATTCTTAA 180
A 181

SEQ ID NO:6079
SEQUENCE LENGTH:54
SEQUENCE TYPE:nucleic acid
TOPOLOGY:linear
CLONE:HUMGS07219
SEQUENCE DESCRIPTION:
GATCTATCAT TACAAAATNA ATTGAATAAT AAAATAGTTT GGTAGAAACT TAAA 54

SEQ ID NO:6080
SEQUENCE LENGTH:225
SEQUENCE TYPE:nucleic acid
TOPOLOGY:linear
CLONE:HUMGS07220
SEQUENCE DESCRIPTION:
GATCCAGAGT TGAAGACCCT TCAGCTGGCT CTGCCTGCCA GTGCCACANA GTGCCATGGC 60
CCAGGAAGAC AGGTTTTNTT CCATCTAGGC CAGGCCATCC AGTGGCCATC CTCCGTGTCC 120
TCCCGCCTCC TCCTGGTGTG ACTTCTGAAA ACCAAGAATT TGTTCCTGTT GACTTTTTCT 180
GTGCTATGGA CCATTGTCCT CTCACCCACT CAATAAATCT TGAAA 225

SEQ ID NO:6081
SEQUENCE LENGTH:291
SEQUENCE TYPE:nucleic acid

TOPOLOGY:linear
CLONE:HUMGS07221
SEQUENCE DESCRIPTION:
GATCTCTNCT AACNNTAGTA AATAAACACT AAAGTGGTGG GCTTTGATAA TGCATTTNGT 60
GGATACAAAA TCATCGNCTC ATCACTTTCT AGAATGAAAG GCTGGGAGGC ATTGCTCACC 120
TTTTGCGTGA AGAAAAGACC TGTCCCTTAG AGCCAGTTTT TNCTTAAGGG GAGGACAGAC 180
CGTCGTCACC CAGTGGACCN CGTTGGATGG NTGGATGCAG TNCATNCTTT AGAAGCACCG 240
CATACCTGCT TGTTACAGGT NCAGNGTAAG TGGTCTTNGC TNCTGTGGGA N 291

SEQ ID NO:6082
SEQUENCE LENGTH:107
SEQUENCE TYPE:nucleic acid
TOPOLOGY:linear
CLONE:HUMGS07222
SEQUENCE DESCRIPTION:
GATCTNCCCT AGCCCTCTNT TCCAGAAGAT GCCCTCCAAT CCTTTCCACC CNATTCCCTA 60
ACTNTNGGGA CCTCGTTTGG AAGTTTTCTG TGGGCTTGGC CTTGGTN 107

SEQ ID NO:6083
SEQUENCE LENGTH:173
SEQUENCE TYPE:nucleic acid
TOPOLOGY:linear
CLONE:HUMGS07223
SEQUENCE DESCRIPTION:
GATCTAGTTT TCCAAGTAAC ATTTTGTGGT GACAGAAGCC TAAAAAAAAG CTAAAATCAG 60
GAAAGAAAAG GAAAAATACG ANTTGAAAAT TAAGGAAATG TTAGTAAANT AGATGAGTGT 120
TAANCTAGAT TGTNTTCATT NCTAGATAAN NTGTATAACG CTCTCTGTNC TAN 173

SEQ ID NO:6084
SEQUENCE LENGTH:328
SEQUENCE TYPE:nucleic acid
TOPOLOGY:linear
CLONE:HUMGS07224
SEQUENCE DESCRIPTION:
GATCACCTGC GTGTCCCATC TACAGACCTG CGGCTTCATA AAACTNCTNA TTTCTCTTCA 60
GCTTTGAAAA GGGTTACCCT GGGCACTGGC CTAGAGCCTC ACCTCCTAAT AGACTTAGCC 120
CCATGAGTTT GCCATGTTGA GCAGGACTAT TTCTGGCACT TGCAAGTCCC ATGATTTCTT 180
CGGTAATTCT GAGGGTGGGG GGAGGGACAT GAAATCATCT TAGCTTAGCT TTCTGTCTGT 240
GAAATGTCTA TATAGTGTAT TGTGTGTTTT AACAAATGNT TTACACTGAC TGTTGCTGTA 300
AAAGNGAATN TGGGAATAAA GTTATTTN 328

SEQ ID NO:6085
SEQUENCE LENGTH:156
SEQUENCE TYPE:nucleic acid
TOPOLOGY:linear

CLONE:HUMGS07225
SEQUENCE DESCRIPTION:
GATCTCAGCT ATTTAAATAC AAACTTGAAA AAAATATTTT TCCAGGGTAC CATAAATATG 60
ATTAANTNNN TAAAAAATTT AGTGTCACTT GATGAAAATA TAACTAGACC AATAGTAAAA 120
TNCAGGTGTA TCTTANTTCC CTGANGTCCT CATTTN 156

SEQ ID NO:6086
SEQUENCE LENGTH:276
SEQUENCE TYPE:nucleic acid
TOPOLOGY:linear
CLONE:HUMGS07226
SEQUENCE DESCRIPTION:
GATCCTGGGC TGCTGGTGCT ACCTGCGGCT GCAGCGCATC ANCCAGTCGG AGGACGAGGA 60
GAGCATCGTG GGGGATGGGG AGACCAAGGA ACCCTTCCTG CTGGTGCAGT ATTCGGCCAA 120
GGGACCGTGC GTGGAGAGAA AGGCCAAGCT GATGACTCCC AACGGCCCGN AAGTCCACGG 180
CTGAGCCAGG ATTCAAGGNT CCTNGTCCTA TTTNCAGCCG GCCAANNGGC GCTNGNAGGG 240
GNAAANCCAT ACGGTTGCGC TNNTGTCTGA GAGGAN 276

SEQ ID NO:6087
SEQUENCE LENGTH:151
SEQUENCE TYPE:nucleic acid
TOPOLOGY:linear
CLONE:HUMGS07227
SEQUENCE DESCRIPTION:
GATCTTGCCA CATCTGGCAC GGAGACGACA CAGTAATNCT GAAAAAGCCT CTATGTAGTC 60
CTGTTAGTNT CTTAAAGAAC CTAAAAGCTN GGACCAGTAA AATCCACAGA AATTCACTCT 120
TGCCTTTAAG AACTTTTGAA AACTGTTTAA A 151

SEQ ID NO:6088
SEQUENCE LENGTH:373
SEQUENCE TYPE:nucleic acid
TOPOLOGY:linear
CLONE:HUMGS07228
SEQUENCE DESCRIPTION:
GATCCAATGG ATTTAAACAG AAGCNCTTTG CCAGGCTTGC CAGCAAGAAG GCAGTGGAGG 60
AACTTGCCTA CAAATGGAGT GTTGAGGATA TGTAACTTTC CTNAGGCTGT GGGGGTGGCT 120
GGNCTGTGGT AGTGGGCATA GGCAGCGAGA TATCCAGTGG TAACAGTTGT CTGTGCTAAT 180
AATTGGAGCC CACACAGNCC AGCAGCTTGT TGAATGCCAG TTTTGACCAC AGAAGGTTTA 240
TTCGAGGCCT NATGTTTGGA CTGAGGTACC TGTACTTCTT NGGGTGTTGA CAGCACCGGC 300
TGTTGCTGGN TTTCAGAGGG AGCGTTGATT TCTCNTTGAC CAGGGNTTNT TCTTTGGTTA 360
GGGTGTTTCT TCN 373

SEQ ID NO:6089
SEQUENCE LENGTH:174
SEQUENCE TYPE:nucleic acid

TOPOLOGY:linear
CLONE:HUMGS07229
SEQUENCE DESCRIPTION:
GATCAGNAGA GGCCANGCTC CNCCCCACCC CAAACAGCAT GAACTTCCCA AGGCTCCATC 60
CCTTTCTCCC AGTGCACAGG CTGGTCACTT TCTCCGGGTA CCCCCTTGGG TACCTTGGCT 120
GTCTCAGTTC TATTTAACCA TTTCCCCACT TTTGAGCATT TACTCACAGT TAAA 174

SEQ ID NO:6090
SEQUENCE LENGTH:142
SEQUENCE TYPE:nucleic acid
TOPOLOGY:linear
CLONE:HUMGS07230
SEQUENCE DESCRIPTION:
GATCTCAGAT TTTTTTTGAA GTTAACATGC CTGACACAGA CATCCTTTCC TCTCACAAGC 60
TGTGTGACTT AGTAGATAAA ATACTGCCTT CTGCCTTTGG GACCATGATT AAAAACAAAG 120
ACAAAAACCA AAAGTCATTA AA 142

SEQ ID NO:6091
SEQUENCE LENGTH:139
SEQUENCE TYPE:nucleic acid
TOPOLOGY:linear
CLONE:HUMGS07231
SEQUENCE DESCRIPTION:
GATCACTGGA CATCAAAGAT TCATTGCACT TATGAACAAG GAACCTTCTT TTCAATTTCT 60
GTGTAATTTG CAAGGCTGTA CAATGTGTGC TGATGCAAGC CTTTTTCAGT TCAAGAGAAT 120
AAATGTTTAC AANTATAAA 139

SEQ ID NO:6092
SEQUENCE LENGTH:230
SEQUENCE TYPE:nucleic acid
TOPOLOGY:linear
CLONE:HUMGS07232
SEQUENCE DESCRIPTION:
GATCTATAAA TCAAGAAAAT CCATTGTCAT AACCATTTTT AAAAGTCAAA AATTAAGACA 60
TCCTTAATTA AAANGTTTCA AATCTAGACA CTAAATGTGT GTGANTGTAC AAAGAAAACA 120
ANCCATTGCT TATNCTGTTA TATACTAGTG NNCCTTTTAN TTTTGCTTGC TGTTTTAAAC 180
TTGGACAGTT TGAGGACTTT NGTTGGANCT TCGTATTGTA NGANCTGTTN 230

SEQ ID NO:6093
SEQUENCE LENGTH:103
SEQUENCE TYPE:nucleic acid
TOPOLOGY:linear
CLONE:HUMGS07235
SEQUENCE DESCRIPTION:
GATCGAGGAC TATTCCNGTG CTATTCATTC CTCCATTGCT AAACAGATNA AAAGCGACGG 60

TTNCTNCACT AACAGCAGCT GGGAAAGANG TTATGCTTGT TGN 103

SEQ ID NO:6094
SEQUENCE LENGTH:246
SEQUENCE TYPE:nucleic acid
TOPOLOGY:linear
CLONE:HUMGS07238
SEQUENCE DESCRIPTION:
GATCCCAAGG ACCTGGAGCC ACGGGCTGCC AACTGCACTC GGGTACTGGT GTGGCATACT 60
CGGACAGAGA AGCCCAAGAT GAAGCAGGAG GAGCAGCTGC AGCGGCAGGG CCGGGGCTCA 120
GACCCAGCAA TTGAGGTGTG ATGGCGGCCC CACCCCAACT ACCACCTCTT TTCAGGCACA 180
GACCTTGTGG GACTGGGNCC AGGCCTNCCC AGGATTGTGG GTTTTTCCAA AGTCCTTGAC 240
CNTTTN 246

SEQ ID NO:6095
SEQUENCE LENGTH:158
SEQUENCE TYPE:nucleic acid
TOPOLOGY:linear
CLONE:HUMGS07241
SEQUENCE DESCRIPTION:
GATCCACCAG CCTCAGCCTC CCAGAGTGCT GGGATTACAG GCGTGAATGT AATNNTTTTT 60
AAAAAGAAAA ACATGGNTGT AAAGAAAGTA AAGCTTCGTT TTGAACAGTT ATGATGGTAA 120
CACANCTCAA NGGTGCAACT AANGCAGTGC TGCANCCN 158

SEQ ID NO:6096
SEQUENCE LENGTH:112
SEQUENCE TYPE:nucleic acid
TOPOLOGY:linear
CLONE:HUMGS07243
SEQUENCE DESCRIPTION:
GATCTGTGAC ACTNACATGG CTGTGGTGTG CATACTGTGT AGTTACATAG CCCTTCCAAT 60
TCTGGGTCCA TTTGCACTAG CAAATTAAAA TATGCTTTGA TTCATACTTA AA 112

SEQ ID NO:6097
SEQUENCE LENGTH:81
SEQUENCE TYPE:nucleic acid
TOPOLOGY:linear
CLONE:HUMGS07244
SEQUENCE DESCRIPTION:
GATCCCAAAC NACTCACTCC TTAAATTCAC ACTTTGCCAC TTAACTCCAG TGTGGATGAC 60
AGAGCGAGAC CCTGCCTCAA A 81

SEQ ID NO:6098
SEQUENCE LENGTH:104
SEQUENCE TYPE:nucleic acid

TOPOLOGY:linear
CLONE:HUMGS07245
SEQUENCE DESCRIPTION:
GATCAACCTC AACACCTGGC NTCTCANGCC TATATACCGA CATATNCAGC AAACCCTNAT 60
GNAGGCTACA AAGTACGCGC AAGTACCCAC GTAACTGACG TTAN 104

SEQ ID NO:6099
SEQUENCE LENGTH:54
SEQUENCE TYPE:nucleic acid
TOPOLOGY:linear
CLONE:HUMGS07246
SEQUENCE DESCRIPTION:
GATCTTTTCT AAATATTATT ACTTGTAAAT AAAGTCTATT TTGNTCCCGT GAAA 54

SEQ ID NO:6100
SEQUENCE LENGTH:133
SEQUENCE TYPE:nucleic acid
TOPOLOGY:linear
CLONE:HUMGS07249
SEQUENCE DESCRIPTION:
GATCTAAATA TGAAAAGTAT GTCCTGCTGA ATTTTCCTGT GNATTGGTGA GCGGACAGAC 60
TACCTGTAAA CCATGACCTC CTTGCCAAAC GTTAGTTTTA TNAGNTCACT NGTAACCTTT 120
GAGAATNNTC TGN 133

SEQ ID NO:6101
SEQUENCE LENGTH:104
SEQUENCE TYPE:nucleic acid
TOPOLOGY:linear
CLONE:HUMGS07250
SEQUENCE DESCRIPTION:
GATCGTNCCA CTNACCTCCA GCCTGGGGGN NAGAGCAAGA CCGTGTCTCA AAAACAATTN 60
AGTCTGAAAC ACAATTGTCC TGAATCTGTC TGACTATAAC TCTN 104

SEQ ID NO:6102
SEQUENCE LENGTH:126
SEQUENCE TYPE:nucleic acid
TOPOLOGY:linear
CLONE:HUMGS07251
SEQUENCE DESCRIPTION:
GATCTCAAAA AAGAAGGAAT TTCTCCTNTN TNTCTTGCAG TTAATGTAAG ANTACTTTAA 60
ATCTCTAANC TTCTGAAGTG TTAGAGGTAG AGATGGTCTA GTAAAGATGT GGTAGTAATG 120
TNTTAN 126

SEQ ID NO:6103
SEQUENCE LENGTH:179

SEQUENCE TYPE:nucleic acid
TOPOLOGY:linear
CLONE:HUMGS07253
SEQUENCE DESCRIPTION:
GATCAAGCCA TCGGAGCTGC TAGAGTTCTG TCTGGACTTT CCAGAGACCA AGTNTTCCCT 60
TTTGCTGCCT CTAAAAGGCC TGTGCCTGCA GACATGTGAG ACAGCAGGTC TGATGGGGGT 120
GACAAGCTTT NTTTTTTTTN CCNAAAGAAN TTNCAAATTC AATTCCAGAT TGNTTTTNN 179

SEQ ID NO:6104
SEQUENCE LENGTH:293
SEQUENCE TYPE:nucleic acid
TOPOLOGY:linear
CLONE:HUMGS07257
SEQUENCE DESCRIPTION:
GATCAAATTT CANTGGCTTT NGACAGAAAA GAAGGCTCTG GATTTAAGCG GGTGGTCACC 60
TGTNAGACCA GGTCTACCTT GGGACTGTNA TTTAACTGAA TCAGTTATTT CCTTGAAATT 120
TCACAGTAGT GGGTGGGCCT GTTTTAAGGN TCTGACAGAT ACCACGAAAC ATGAAGCACG 180
TGGAACTACA AGACCCCCGG GGTCTTTCTG AGTGCAAGGC TGAAATGGGN CAAGGGCTCC 240
TCACGGGGNG TGGAGGGNNG CCGGGAGNCT TNCNTGGTTG TTNCNTTTTT TGN 293

SEQ ID NO:6105
SEQUENCE LENGTH:110
SEQUENCE TYPE:nucleic acid
TOPOLOGY:linear
CLONE:HUMGS07259
SEQUENCE DESCRIPTION:
GATCCCGTNC GGCGCGCTGC CCGAGAGGAA CAATGACAGC TTTGCAGTTC TCCNGGAGTN 60
CGCTGAGGAG CAGCTGCAAG CCGACCANGT NTTCATTTAC TTCCACAAGN 110

SEQ ID NO:6106
SEQUENCE LENGTH:183
SEQUENCE TYPE:nucleic acid
TOPOLOGY:linear
CLONE:HUMGS07261
SEQUENCE DESCRIPTION:
GATCTAGTAC TCCTTGATGA AGAATCGGGG CCTGTAAACT TACCTCCAGC ACTAAAACAT 60
CCTCANGANT TACTATAATG TGTCCAAAAT ATCACTGCAT ACAATATCTG NNATTTGAAG 120
TGAAAAACTG ACTTTNGTAT AGTATAAAAC ACAGGCTTTC ACAANTTTTG GTNTNGCTGG 180
NTN 183

SEQ ID NO:6107
SEQUENCE LENGTH:135
SEQUENCE TYPE:nucleic acid
TOPOLOGY:linear
CLONE:HUMGS07263

SEQUENCE DESCRIPTION:
GATCCTGANG ATGATGATGA TGATGATGAT GAAGAATAAA TGGAATCAAA ATGCTAGCTT 60
NATTTGACAT NAATAAGTAA AATAAGTCAT CATGTNTTCA ACNCTGTAGT TCAGCTGTTT 120
ACATTGAATA ATNTN 135

SEQ ID NO:6108
SEQUENCE LENGTH:173
SEQUENCE TYPE:nucleic acid
TOPOLOGY:linear
CLONE:HUMGS07264
SEQUENCE DESCRIPTION:
GATCCTTTCC AGTTTAAGCA GCCAATCAGA CCNCTTCACA TTTCTAAAAA AATGACCTAA 60
CTAAAGCACC AGAGCTCGGA GANTTCCAGG GAAGCCAGAT ACCGAGGCGC AAATTTTTTA 120
AAAAATAAGA GTCAGAAATA AAAATAAANG GTNTCTGTTG GTCAAGATTT AAA 173

SEQ ID NO:6109
SEQUENCE LENGTH:152
SEQUENCE TYPE:nucleic acid
TOPOLOGY:linear
CLONE:HUMGS07265
SEQUENCE DESCRIPTION:
GATCAATTGA AATCAACTGT GTTTTGTNTT CTCTATGTCA AAGTTTAGTT TTATATTGAG 60
AATGTTAACT TATTGCTTTG TATCTNGGGA AAAAAACTTT GTAAATAAGN TATAAAGTTT 120
CTTTGAGACA GTAAAATTAT GATTNCTTGA AA 152

SEQ ID NO:6110
SEQUENCE LENGTH:337
SEQUENCE TYPE:nucleic acid
TOPOLOGY:linear
CLONE:HUMGS07266
SEQUENCE DESCRIPTION:
GATCTGCTCA CCTCGGCCTC CCAAAGCCTC AGCCTCTTAC AGTGTTGGGA TTACAGGCGT 60
GAGACACTGT GACCCGGGAC GATTTTCAAT CACAGTTTTT TGNTACGAGT GGAAAATGCG 120
TATTTATAAG AATGAAGTAG TACAGACATG ANTNTGTAGA AATCTCTATA ATCCTGCCAT 180
CCAAGGTTGG TACCTGTTAA TGTGTATATC AGGGATGTGC AATCTTTTGG CCTNCCTGTG 240
CCACACTTNG AAGAAGAAGA ATCGNCTTGG GCCACGGATA NANTNNCACT AACACTAGCA 300
ATACCTGGTG NGCTGAAANA GNNAGTTAAT TCNCNTN 337

SEQ ID NO:6111
SEQUENCE LENGTH:76
SEQUENCE TYPE:nucleic acid
TOPOLOGY:linear
CLONE:HUMGS07267
SEQUENCE DESCRIPTION:
GATCCTGTTA ATNAAACCAC AGACACCATA TATCCTTCTG CATCCTTTGG CCAATAAAAG 60

TTGCTGGAGA ACCAAA 76

SEQ ID NO:6112
SEQUENCE LENGTH:176
SEQUENCE TYPE:nucleic acid
TOPOLOGY:linear
CLONE:HUMGS07268
SEQUENCE DESCRIPTION:
GATCAAGGAG AGTCACAGCC TTATTAAAAG TTTATTAAAC AATANTATAA AANTTTTAAA 60
CCTACTTGAT NTTCCATAAC AAAGCTGATT TAAGCAAACT GCATTTTTTC NCAGGAGAAA 120
TAATCATATT CGTAATTTCA AANGTTGTAT AAAANTATTT NCTATTGTNG TTCAAN 176

SEQ ID NO:6113
SEQUENCE LENGTH:63
SEQUENCE TYPE:nucleic acid
TOPOLOGY:linear
CLONE:HUMGS07269
SEQUENCE DESCRIPTION:
GATCGACACG TGGGGGCGCC TGAGCGAAGA TAACCGTAAT AAATAGTAAC CTAACGGTCC 60
AAA 63

SEQ ID NO:6114
SEQUENCE LENGTH:73
SEQUENCE TYPE:nucleic acid
TOPOLOGY:linear
CLONE:HUMGS07270
SEQUENCE DESCRIPTION:
GATCAGNCCT CGCGCAGTGG CTCAAGGCTG TAATCTCAGC ATTTTGGGAG NCCGAGTGAG 60
ACCTTGTCTT AAA 73

SEQ ID NO:6115
SEQUENCE LENGTH:243
SEQUENCE TYPE:nucleic acid
TOPOLOGY:linear
CLONE:HUMGS07272
SEQUENCE DESCRIPTION:
GATCTTAAAT ATTCTAAAGT TTGTATTGTG ACTTCCAGTA ATGTAAACTA TATGAGGCTA 60
ATCCTCTTTT CCTCCTTTTT GTTTGTTAAC CTCATTTGGG ATAACTGTAA CTAGTATTAT 120
TTTATTTATG TAATAAAAAT ATATGTGAAA TAAAGTGTTA CTGGTAAAGG ACTTGTAAAC 180
ATGATAANTA TTAGCAAGTT GCATACAGAN TTAANAGNGN GGNGNGNANG GNGTGNGGTN 240
TGN 243

SEQ ID NO:6116
SEQUENCE LENGTH:199
SEQUENCE TYPE:nucleic acid

TOPOLOGY:linear
CLONE:HUMGS07274
SEQUENCE DESCRIPTION:
GATCAGAAGT TCAAGGGACC GTGAAAGCCC TCAGAGTCAG CACCTAGTTT CAGACCAAGC 60
ACCCTTTCGA ATCCCTGGAT GGCTGAGGGG GCTGAGGCCG GCTCTGACTG GGCAGCTCAG 120
CNCCTCCCCC AGAGCCCAGG GTCTTGCACA CCCCTNCCTG TAACCAAGGA ACACTCTGAA 180
ATAAAGGTGA ATGGCTAAA 199

SEQ ID NO:6117
SEQUENCE LENGTH:283
SEQUENCE TYPE:nucleic acid
TOPOLOGY:linear
CLONE:HUMGS07275
SEQUENCE DESCRIPTION:
GATCAAAATA CTAATAAATT ACTCACAGAC TCAGTGTATT TTTNCTTGGA GTAAAAGTCC 60
AGGATGGGTA ATAGAATACC TGCTGTTGGC TTTTGGAAAA ATTGGTACTG TATGTAGCAA 120
AATAATGTGA AACCCATATG CATGGATATT CTTAACAATT TGAAGAAATC GTCACAGCTT 180
TCCTGGGTTG TTGAGCCTCT AAGATGGTCT TTTCCTCTGA TGTGATAATA AAGTGTTTAT 240
TCTGANCTCT AAAAAAAANN NANNGNGGNG NNTGNNGTGG NGN 283

SEQ ID NO:6118
SEQUENCE LENGTH:317
SEQUENCE TYPE:nucleic acid
TOPOLOGY:linear
CLONE:HUMGS07276
SEQUENCE DESCRIPTION:
GATCACAGTT TTACAAATCT TAGTTTTAAA TAAATTATTT CAGTGTGCTG TTAGTCCTCT 60
ACAGTCATTT TGGTTTAAAA AGTGACTATT TATTTATGGT AGNATATCAA TAATTTATTA 120
ATGTTAAAAA ATACTGTGTA TGACATTACA AACCAGANCA GTTCCTGGGG GAGAGGNTTC 180
TNATTGATTG GCAGTTCTGA GAGGGCAAGA NGANTGGNAC TTTATNCTTC AAAAGGNGGT 240
TTTGGTTTTN CCAGGTACTG CTTATGTNAA TCGTTTATTT TTNTTTCATC AAAGCCTGGC 300
ANGTNTATGC NTTCCAN 317

SEQ ID NO:6119
SEQUENCE LENGTH:360
SEQUENCE TYPE:nucleic acid
TOPOLOGY:linear
CLONE:HUMGS07277
SEQUENCE DESCRIPTION:
GATCGACGAG CTGCTGCAGG AGTTCGAGGA GAAGAGTGGC CGCACCTTTC TGCACACCGT 60
CTGCTTCTAC TGAGCCCAGC GCCCGCATGG AGCCGCCTCT GGAGCTTCCT GTTGTTCATA 120
CTTTTTCCTT CCTGACATTT GTTTTTACTT ACAGGTGTTC TGCTGGTGAC GGTAGCATTA 180
CCCAAATAAA CTGTGCATAT GAAATGGGAG AGGAGATGCC AAAACGCCAG ATGAAAGCAA 240
TCAAGTTTCT TCTTTTCCAC TTTTACTTAT GAGCGGGATA TTGATTACAN GGNNTTTCTT 300
CTTTAACCAA AAAGGAAAGA CANCGGTTTG TGTGCACTTC CCGNCATACC TGTGTCTTTN 360

SEQ ID NO:6120
SEQUENCE LENGTH:292
SEQUENCE TYPE:nucleic acid
TOPOLOGY:linear
CLONE:HUMGS07278
SEQUENCE DESCRIPTION:
GATCGGGAAG ACTGAGTAGG GAAGGCAGGG CTGCCNAGAA GTCTCANAGG CACCTCACGC 60
CAGCCATCGC GGAGAGCTCA GAGGGCCGTC CCCACCCTGC CTCCTCCCTG CTGCTTTGCA 120
TTCACTTCCT TGGCCAGAGT CAGGGGACAG GGAGAGAGCT CCACACTGTA ACCACTGGGT 180
CTGGGNTCCA TCCTGCGCCC AAAGACATCC ACCCAGACCT CATTATTTCT TGCTCTATCA 240
TTCTGTTTCA NTAAAGACAT TTGGAATAAA CGNGCATATC ATAGCCTGGA AA 292

SEQ ID NO:6121
SEQUENCE LENGTH:30
SEQUENCE TYPE:nucleic acid
TOPOLOGY:linear
CLONE:HUMGS07279
SEQUENCE DESCRIPTION:
GATCCAATAA ATNTCCTTTT TTTGCTTAAA 30

SEQ ID NO:6122
SEQUENCE LENGTH:149
SEQUENCE TYPE:nucleic acid
TOPOLOGY:linear
CLONE:HUMGS07280
SEQUENCE DESCRIPTION:
GATCGGGAGA GGGAGGAGAG GCAGTCTCTT GACAAAATAA AGTATTTTTA TTCATTTGTG 60
TTTATTAAAT GAAAAANCAA TCCCATGGTG TCCCTGTTGT GTGGTGGACC TAATGNCTGT 120
TGAAATAAAG TTCTGTGTTT TCCCTGAAA 149

SEQ ID NO:6123
SEQUENCE LENGTH:216
SEQUENCE TYPE:nucleic acid
TOPOLOGY:linear
CLONE:HUMGS07281
SEQUENCE DESCRIPTION:
GATCCACCAA TGGAATTAGA TGGGTAGAGT TGGGTTCTTG AGTTTTACCA CCACTTTGTT 60
CCCACTGAAT TTTGTAACTT GCTGTGTTTG CATCCTCTGT TCCTATTCTG CCCTTGCTCT 120
GTGTCATCTC AGTCATTTGA CTTAGAAAGT GCCCTTCAAA AGGGGCCTGT TCACTGCTGC 180
ACTTTTCAAT GAATTAAAAT TTATTTCTGT NCTAAA 216

SEQ ID NO:6124
SEQUENCE LENGTH:205
SEQUENCE TYPE:nucleic acid

TOPOLOGY:linear
CLONE:HUMGS07282
SEQUENCE DESCRIPTION:
GATCTCATCA ACCCTTTGAA ATGTTCTGGG CAGCACAGCT CCTTGGTCTG CATGGTTGGT 60
GTAAGTATAA TCANGTAGTG AAATTGTATG GTATATACCT ATATGCCANC AGTGTACACC 120
CGTATGTGCA GAATTTGCTA NTATAACCAT TTCAATAAAA ACCTATTGAA AAAGCTGAAA 180
AAANNANAAA NGNAGGAGGN GNGGN 205

SEQ ID NO:6125
SEQUENCE LENGTH:279
SEQUENCE TYPE:nucleic acid
TOPOLOGY:linear
CLONE:HUMGS07283
SEQUENCE DESCRIPTION:
GATCTTTGCC AGGGGTCAAA GCGTCGGGAA GCTTGGGGTC CTTCATCCTG ACGTTATCAC 60
CAAATTTNAG CTGACCATGC CCTGCTCCTC CCTAGAAATC AATATTGGAC CCTTTTTGTG 120
AAGATTGGTC TCTGTGGTGT GATTCTCTNC CCAGGTGTCC CTTTCTCCTC CCCTAGTGTC 180
CTTAAGTCCT CCTCCACAGG GAACATCTAT TTGGGCTTTG ATGTTTAATA AAGTAGAAAG 240
CACTGGNAAA AAAAGAAANN NGNNNTGTGT NTGTTNNNN 279

SEQ ID NO:6126
SEQUENCE LENGTH:226
SEQUENCE TYPE:nucleic acid
TOPOLOGY:linear
CLONE:HUMGS07284
SEQUENCE DESCRIPTION:
GATCCTAAGG AACATAAAGT TAATTAAAAA CTTACACCTA ATTATGTAAA TNGCCTTGTT 60
AAAGACATGT GATTTGTATT TNAGATGCTT GTTTCCTATT AAAATACAGA CATTTCTACC 120
CTCAGTTTCT AAATGTAGAC TATTTGTTGG CTAGTACTTG ATAGATTCCT TGTAAGANAA 180
ANTGCTGGGT AATGTNCCTG GTAACANGCC TGTNANTATN TNANGN 226

SEQ ID NO:6127
SEQUENCE LENGTH:339
SEQUENCE TYPE:nucleic acid
TOPOLOGY:linear
CLONE:HUMGS07285
SEQUENCE DESCRIPTION:
GATCCCATTT AGCCCCTGAA CATAATACCA TCTGCAGTAT TATAAAAGTC ATTTAGAAGG 60
TCAGGGGGAT AATCTAGAGG CAGGATTTTG GACATTGTGA AGGAAATGTG CTCCTTCTCA 120
GCTCACTTCA ATAACTATTT NCTGAGACTG AAGTTTTTTA GACAAGANTA AGANAACTTT 180
GCTTTNTTCA GTTATCACAT GTGANAGCTT TTGCTTTTGG TTAGGAAANG GTGTGGNTGA 240
CTCTACTGCG TGGNTGGTTA CATTTTGCTT TCCGTGNAAT TGCTCNGGAA ATGNTCNGGA 300
CATNCCNTTT NCCATTTGTG TGTCAGTGCG TATGGTNAN 339

SEQ ID NO:6128

SEQUENCE LENGTH:269
SEQUENCE TYPE:nucleic acid
TOPOLOGY:linear
CLONE:HUMGS07286
SEQUENCE DESCRIPTION:
GATCTCCTAG GACCTAGTGG ACATCTGGNA TTAATTTAAT CTCAGGAAAA ACAAGANATN 60
AACCCAGAGA GAGTCTGGGT TCTGGAATTC AGCGTANTAC CTCCAGACCG TGGTGTCTGG 120
CCTCCATTTT TGTCTGTNAT TCAGCTCTGA CTTACAGCTG CAGTCACCTT TNCTATAAGG 180
CACCTGGGTA GAAGGGTGGA TGGGCTTCAC ATCAAATTTT TTNCTTCCTT TAGGGTGGGG 240
NATTGGNTTG GCTNTNTTTT GTNGTGGNN 269

SEQ ID NO:6129
SEQUENCE LENGTH:47
SEQUENCE TYPE:nucleic acid
TOPOLOGY:linear
CLONE:HUMGS07287
SEQUENCE DESCRIPTION:
GATCTGTCCT CCAAATCCTC TATTAAACCT GATAGCTTGA GCACAAA 47

SEQ ID NO:6130
SEQUENCE LENGTH:330
SEQUENCE TYPE:nucleic acid
TOPOLOGY:linear
CLONE:HUMGS07288
SEQUENCE DESCRIPTION:
GATCAGCCCT TCTCAGCCTC CCAAAGTGCT GGGATTACAG NTGTGAGCCA CTGTGCCCGG 60
TTCTGTGGCC TTTTCTAACA TTATCCAAGN NGCCCTAAGG TTCCTTATGA CAGGTTTGGG 120
GATTGGGAAA GTTCAATTTT ATTGTACCGC CAATGAAAAG GTGCTATATT TTTTTCTTGA 180
ACCTNAAGGG CAGAGGCTGA ATTAATCATA TTCCTCAGCT CATTCCACAT TATCATGGCA 240
AAGCTCTCTT TTGCTTTATT TATTTATTCT CTNNTATCTT CATACTTAAT CTCATTCCAT 300
TCTCTGNCAT GGGCAACCAT NNNAATNGNN 330

SEQ ID NO:6131
SEQUENCE LENGTH:333
SEQUENCE TYPE:nucleic acid
TOPOLOGY:linear
CLONE:HUMGS07289
SEQUENCE DESCRIPTION:
GATCTTACAT GAGCATAATC ATCCTTATAC TTCATGAGGG GATTATNAGT ACAATCCCCA 60
TTTTACTGTG TTTGAGTTAA AAACCAAACA TCCCTGTAAT TTAATTTGAA GATTCTTTAA 120
CAGATTGCAG CAAAGTTCAT TATAAAACTG TTATGGTGTC TTCAAAGNCT TGATAAAANT 180
AACACTGNGA GAGANTTGGT CCATTTGTAT GCTGTATTTC TATTACTTGC CAAANGGAAT 240
GGGGNTAGGA TTAACCTTGT TTCCATTCTC TTCNCATGGG TATCCATCCC CATGTTTANC 300
TGNCCACNCT GGGGGGCTCN GTTGTGTGCT GTN 333

SEQ ID NO:6132
SEQUENCE LENGTH:21
SEQUENCE TYPE:nucleic acid
TOPOLOGY:linear
CLONE:HUMGS07290
SEQUENCE DESCRIPTION:
GATCATAAAT AAATGTTTAA A 21

SEQ ID NO:6133
SEQUENCE LENGTH:331
SEQUENCE TYPE:nucleic acid
TOPOLOGY:linear
CLONE:HUMGS07291
SEQUENCE DESCRIPTION:
GATCATTTTC AAAAATTGCA TTGGGGCCTT CGTTATTTAC CATAGTATTT NCACTTTCAT 60
AGTTTTGTCA CCTTTTTGTA CTGTGAACAG TTCAACCAGT GACCGACTTC TCTCTCATGC 120
TGTTTACCCC ACACACAATT TCCCACTCAA TTCTGAAAAT AAGAACCTGT TAATAGGTTG 180
GAAAGCTGTG TACTCTATTC ATATATTGTC CTTTCATGCT AGTGGAGAGT GGTGTCATTA 240
GCATCTNAAT TTTAGAGTTG TGNAATGATT TTNCCAATTA GGAATTGANT GTGTATTTNT 300
NTCCTGTTTA ATAAGNNGNG CAAATNTGNT N 331

SEQ ID NO:6134
SEQUENCE LENGTH:40
SEQUENCE TYPE:nucleic acid
TOPOLOGY:linear
CLONE:HUMGS07292
SEQUENCE DESCRIPTION:
GATCTTAAAA CAAAATGAAA TAAACGTGAA AAGGAGCAAA 40

SEQ ID NO:6135
SEQUENCE LENGTH:53
SEQUENCE TYPE:nucleic acid
TOPOLOGY:linear
CLONE:HUMGS07293
SEQUENCE DESCRIPTION:
GATCGTGAAT AGTAAGTATC ACTTCCATAA ATAGAAAGTG TCAGAAAAAT AAA 53

SEQ ID NO:6136
SEQUENCE LENGTH:336
SEQUENCE TYPE:nucleic acid
TOPOLOGY:linear
CLONE:HUMGS07294
SEQUENCE DESCRIPTION:
GATCTTGAAA GAGGTAAAAT AAACCTTCAC TGCTCCACTC CAGGTGAATC CGCCCACTCC 60
CACTGACCTA GTAGAATTTG TAATTTAATA CTTACCTTCT ATTTCTGAAA TCAGTTGTGA 120

ACTGTTGCCT TATGTTCAGA GGTTTAAGAA CCTCAGTGAA TTCATTTTTT AAAATCTGCT 180
ATTCTGAGAA GCATTGAATG AATTCTTAAC AAGAAGNCTC ATCTGTAGCT GTTTGCTGAC 240
TCCTATGAGC CCATAAGGGT TCTGTGCTTA GCATTAACAA ANTAAGGTTT ATAGGGTAAA 300
AGCCAATGTA TTANTTTTTT TTTGCATGGG GGGCNN 336

SEQ ID NO:6137
SEQUENCE LENGTH:235
SEQUENCE TYPE:nucleic acid
TOPOLOGY:linear
CLONE:HUMGS07295
SEQUENCE DESCRIPTION:
GATCTAGGAC ACTCATATCC AGGGTGATTC TCTTCATAAG GAGGCATGGG CTGAGTCTCC 60
ATTTATAGGG GCTGTCCCCA TAGAGACTTC AAGCCACACC TTTCTAGGGA TTCAGGTTGC 120
ACATTACCTG ATTCTCACAG TTGATTTGAG CCTGCAAGAC TGTAAACCCA TCCCATTATC 180
ATGTTTTCTT CCTCATTTAC CTACATTTAT CATTAAACCT TTGTGATTCA GCAAA 235

SEQ ID NO:6138
SEQUENCE LENGTH:142
SEQUENCE TYPE:nucleic acid
TOPOLOGY:linear
CLONE:HUMGS07296
SEQUENCE DESCRIPTION:
GATCCAGGAT GACTGTACAT CTCAGATAAA GGAGGCTCAG CGCTGGAAGG ACAGCTGGAA 60
GCAGTCCCTG CACACTATCC AGGGCCTGTA TGTGTGACCC TCCGCCCCAC CATGAATAAA 120
CACTTTCTTA TACAGACTCA AA 142

SEQ ID NO:6139
SEQUENCE LENGTH:277
SEQUENCE TYPE:nucleic acid
TOPOLOGY:linear
CLONE:HUMGS07297
SEQUENCE DESCRIPTION:
GATCCGCCAA GCAGGGCAGT ATCTGGGTTA CAGCCACAGC AGGAAGAACC ACTGGAGAGA 60
CACTCAGAAG GACTTCCCTG ACATCTGCAC CAAGGAGAGT CTGGACAAAG CTACTGAAAC 120
TCCTCCACCT GCTTCTAAAA TGCCCAGAAG CCAACTGGAC CNGTTTGGTA TTTNGAAGTA 180
GGNGGNTGGN GGCTTTAACC NCCAAAGGGT TCCTGAAGCC CNGGAACTTG GANTNTGGAG 240
GGGGTTNNGG CCTTANTTGA GGGTTGGTGG GTTTCNN 277

SEQ ID NO:6140
SEQUENCE LENGTH:247
SEQUENCE TYPE:nucleic acid
TOPOLOGY:linear
CLONE:HUMGS07298
SEQUENCE DESCRIPTION:
GATCAGAAAA CATTGATTTA TATTTAAAAA TAGGATATTG GAGCCATTGT CTTAGATAAT 60

TNNCAAGGCA AGANCAAGCC TGCAATAAAT AATGAGACTT TGACATTCCT TGGCGAAGAA 120
GATGTCTCCT GCCCTAATAT GACTGTCACT CAGCAATTTC ANTTTTNTGT TTACTTGATT 180
TANAANGGGA GGATAANTTG TCCTGANAAG TTTTTTTGTT TCCTTAACAT TTCANNGTTT 240
TCNCTGN 247

SEQ ID NO:6141
SEQUENCE LENGTH:228
SEQUENCE TYPE:nucleic acid
TOPOLOGY:linear
CLONE:HUMGS07299
SEQUENCE DESCRIPTION:
GATCCCTGAT GGGATGCCTG AAACCCAAAA TACTCTTAAG TCTTGTATTC TAGGGCCTTC 60
TAAGGCAAAA NATTGAGAGA CCCAAAAAGA GAAAATATGT TTAGTAAAGA AAATATTTNC 120
TTTGACTAGT TATGATGGTC ATATAGGGGA GANAAGGCAA ATTTTGGNAA TGTAAGATGG 180
GGACANTTAA TGTNTATNCN CTGTCCAANT TCAANAGGTA CAACNGAN 228

SEQ ID NO:6142
SEQUENCE LENGTH:228
SEQUENCE TYPE:nucleic acid
TOPOLOGY:linear
CLONE:HUMGS07300
SEQUENCE DESCRIPTION:
GATCCACCTG TCTCTTGAAA ATATAAAAAG ACAAAACAGG TTTGCCTTGG CATCAGAGAG 60
CACAAAGATT AAAAGTTACT TTAAATTTGC CAATATTTTG GGAGANCAAT AAANCTACAT 120
TTTTCCCTCT TCCATACTGG TAGATGCGAA ATTNATCTGT GCATGANAGG GTCACTTCTG 180
TNATCGTGCA ACAGNTTTGG TATTANNCCT TNNCTGTGGT TTTNCCAN 228

SEQ ID NO:6143
SEQUENCE LENGTH:299
SEQUENCE TYPE:nucleic acid
TOPOLOGY:linear
CLONE:HUMGS07301
SEQUENCE DESCRIPTION:
GATCCTCTGA TATACAATTA GAGATATTTT AATATAGNCC CCAAGCATTC TGTGCATAAA 60
AGTTAACATT AGGCTGTGGT GCAGTAACCA TTTAATGTCG AGGCTCTATT TCGGAAATAC 120
ACTACAAATG TTAAAGTACG TGGCTGTCCT CTTAAGNCAC TAGTAGAGCA AAGCCTTAAT 180
CATATCANCT TAATTCTGTT ACACAATATG TGTTTTTNAA TATACTANCC ATTTCTNATG 240
GAANGGTCCT GTGGGGNGCC CNTCCTCTCG CCANGCCATC ACAGGCTCTG CATACACGN 299

SEQ ID NO:6144
SEQUENCE LENGTH:152
SEQUENCE TYPE:nucleic acid
TOPOLOGY:linear
CLONE:HUMGS07302
SEQUENCE DESCRIPTION:

GATCGGGTGC TAGGACAACT GCAGTCCAAT CCACCAGCTC TCCNTGCCCC TGTGTCTTAT 60
TTCAGACATG AGAATAACTG TACAGTGTAA ACTTATAAAG CGTTTTTAAT GGTTGTAGAT 120
TGGAAATAAA GTATGTCATA TGAACAGCTA AA 152

SEQ ID NO:6145
SEQUENCE LENGTH:131
SEQUENCE TYPE:nucleic acid
TOPOLOGY:linear
CLONE:HUMGS07303
SEQUENCE DESCRIPTION:
GATCCCACCT GCTCAGGCCG TACACCTTAT GGGGAGCCAT TAAGGGGNCT CATCTAGGCA 60
TTCTGGNTAC AGCCCAGTGC TCATCCCAGC GTATGCTCCN CTCTNTAGNG CAGCCTAAAG 120
NGTCAGCCAC N 131

SEQ ID NO:6146
SEQUENCE LENGTH:154
SEQUENCE TYPE:nucleic acid
TOPOLOGY:linear
CLONE:HUMGS07304
SEQUENCE DESCRIPTION:
GATCTTCAGA GAAATAAAAC TTGAGTGCAG TAGTTTATTT GGCGTTTGGC TCCAGGATGC 60
AGGNGTGGGA AAGATGGTAG AGAGCCAGAA GAAAANTCAA TAGAACGGTA TTACGGCGNN 120
TGGNTATATA ATNCTTCCCN GAATTGTCTG ACTN 154

SEQ ID NO:6147
SEQUENCE LENGTH:251
SEQUENCE TYPE:nucleic acid
TOPOLOGY:linear
CLONE:HUMGS07305
SEQUENCE DESCRIPTION:
GATCCAGTGC TATTCTGTCA CCAACCTAAG AATCCCAATT GCACCTTCTG TTTCTGACAG 60
TCACAGGTGA CAGCTGNGGN TCTATAATAC AGACTGGTGT CTTAGAGGTA GGAATAATAC 120
ATGATTATNA AGCATCACCC TGCTAATACA TAATAATGTC TTTTTATATT ATAAGNGATT 180
GAGTTTAGTT CATTNCAATA CATTTGTACN TGTAAAAANT GCAANGTNGA CCTNNGTAAT 240
CNTTTAATCG N 251

SEQ ID NO:6148
SEQUENCE LENGTH:213
SEQUENCE TYPE:nucleic acid
TOPOLOGY:linear
CLONE:HUMGS07306
SEQUENCE DESCRIPTION:
GATCTCGGAG ACTCNGTCCA TTCTAAGATA CCATGTTTCC ATGACCCTAC ACACAATAGC 60
TACACAGGTG GAGCAACATG AGTCTGAGNA GCTTTGCACA AAAGANGCTT GTTTTCCACA 120
AATGNANCTG GGGTGGTTAA CTTCTAATTG TACCCTGTCT TAGCGTTCAG ATGCATTGGT 180

TCCTTCCCCA ATGTGACCAT CTNNNNATTC NAN 213

SEQ ID NO:6149
SEQUENCE LENGTH:113
SEQUENCE TYPE:nucleic acid
TOPOLOGY:linear
CLONE:HUMGS07307
SEQUENCE DESCRIPTION:
GATCAAGATG AATNTAATGT ATAATCAGAA TCCTAGGTAA GTAAACAAAA AAAGGGTTTT 60
TTTNAGAATC AAACCTTTGG GGATTGGGTG GTAAAGGGAA ATATTTNTNT NCN 113

SEQ ID NO:6150
SEQUENCE LENGTH:228
SEQUENCE TYPE:nucleic acid
TOPOLOGY:linear
CLONE:HUMGS07308
SEQUENCE DESCRIPTION:
GATCTGTGCA TCTGGCCAGC ACTGTGGTGA CATCCTTAGG AATCCATGGG GAGAGAGAAA 60
GCATTCAGGA GTTAGTGGGT CACGTTTGAC AAGGGCCAAT AAAGAAATAT GCAAAGACAA 120
AAANCAAGAA GNACATTGTC ATATTTNCTA CCTTTTGTTT ATATAAATTT ATNTCANTGA 180
TTTTCGCTTN TGTTAATNTG CAATGTATAT NNTATGCTAA CNTTTNCN 228

SEQ ID NO:6151
SEQUENCE LENGTH:42
SEQUENCE TYPE:nucleic acid
TOPOLOGY:linear
CLONE:HUMGS07309
SEQUENCE DESCRIPTION:
GATCACAATT GTGGACATTA AAACAGAAAC TGTTCACACA AA 42

SEQ ID NO:6152
SEQUENCE LENGTH:311
SEQUENCE TYPE:nucleic acid
TOPOLOGY:linear
CLONE:HUMGS07311
SEQUENCE DESCRIPTION:
GATCCAGCTT ATNCTTTAAT TTTCAAGTCC ATTCTTGGGG CTGGTGGGGA GGCAGGAGAA 60
TACCCCTCCC TAAGCCCTTA GTGTGTGCCG AGCTTGCTTT GTNATGTTGG CAGGGGAGGG 120
GAGACCTGGG TGGTGACTGA GTTCCCTTTA TCAAACCCTT CAATGGGCAC AAAATTGAGT 180
GCTTGATTTT AGGTTTTATT TTTTNATGAA TGTCCAAATC TGTGTTTCCC CCTGCCATCC 240
CAGACTNTGT GGCCAGTTNA AAAGTGTCTG GNNTGTGTTC ANCTCTNCCC TAANATNCTG 300
GAGNAGGGGT N 311

SEQ ID NO:6153
SEQUENCE LENGTH:270

SEQUENCE TYPE:nucleic acid
TOPOLOGY:linear
CLONE:HUMGS07312
SEQUENCE DESCRIPTION:
GATCTNNGTG GGTAGCCTCA AGCTGGAGAC ATTGTCTGNC CTCACTGCCC ACTGGGTAGA 60
GACACCTGGA TGACATTTCA GTACCCGCTG GCAGCTCCTC CCACTCATCC AGAGACGGCC 120
GGTGGGCAGG CGGGGAGAGC AGCTTTCCTT CCCTGGNGAG AACCGTCTTT ACCTCAGCTC 180
CTAGGGNCTC CAGCCAACAA AGCTNCAGCA CGGTGACTNG GTGATGGGNG ACCAATNNCA 240
GAAGTAAANN GACTGTAGAC CNATATTCCN 270

SEQ ID NO:6154
SEQUENCE LENGTH:40
SEQUENCE TYPE:nucleic acid
TOPOLOGY:linear
CLONE:HUMGS07313
SEQUENCE DESCRIPTION:
GATCAAATTC TAAAATAAAG TTGCCTGTTG TGACTTTAAA 40

SEQ ID NO:6155
SEQUENCE LENGTH:130
SEQUENCE TYPE:nucleic acid
TOPOLOGY:linear
CLONE:HUMGS07315
SEQUENCE DESCRIPTION:
GATCTATACT TAACATATTT AAAATAGTGA TGATTGAAAT AACTGCAAGG TATAGATGNA 60
ACTGAAGGNT TTCACATTCC TTTTTTGTTT ATCATTTGGA ATTTNCTGTA TTTANCTGNC 120
TAGAGANCAN 130

SEQ ID NO:6156
SEQUENCE LENGTH:173
SEQUENCE TYPE:nucleic acid
TOPOLOGY:linear
CLONE:HUMGS07316
SEQUENCE DESCRIPTION:
GATCATATCA CTCAATGTGG CCATTAATAT TTCTGGAGAA GACACAAACC TATTTATAAT 60
ACTTTTNAAA AATCCGAAGT GATAATAAGA NTGTTTTTAC ATTGAAAGTA ATACTTAAGT 120
GTTTACATAA CATTCTGTAT GCACTCAAGA AAATAAAGTT TTGTTAATAC AAA 173

SEQ ID NO:6157
SEQUENCE LENGTH:248
SEQUENCE TYPE:nucleic acid
TOPOLOGY:linear
CLONE:HUMGS07317
SEQUENCE DESCRIPTION:
GATCTCAGTT ACAGTTTTTT TTTCCTTTTT AATTTCATTA TTTTGGGTTT TTGGTTTTTG 60

CAGTCCTATT TATCTGCAGT CGTATTAAGT CCTATTGCTA GAATAGGTTA CTNNANGAAA 120
GATTATATTC TGAAAGAAAA ATAACTGACA TTATATATAA CCAATTAATT TAANGTATTG 180
CCATTTAAAT TACACACTGN GAGCATGTCC TATGCAGACA ATAGATTTTN CCTGTNCAAN 240
NNNTTNTN 248

SEQ ID NO:6158
SEQUENCE LENGTH:187
SEQUENCE TYPE:nucleic acid
TOPOLOGY:linear
CLONE:HUMGS07318
SEQUENCE DESCRIPTION:
GATCCAGCAG CGCAGGCAGG AGCTGGAGCA GTCGCTGGTT GTNCGCAACA CCTAGGAGCC 60
CAAAAATAAG CAGCACGACG GAACTTTCAG CCGTGTCCCG GGCCCCAGCA TTTTNCCCCG 120
GGCTCCAGCA TCACTCCTCT GCCACCTTGG GGTGTGGGGC TGGATTAAAA GTCATTCATC 180
TGACAAA 187

SEQ ID NO:6159
SEQUENCE LENGTH:21
SEQUENCE TYPE:nucleic acid
TOPOLOGY:linear
CLONE:HUMGS07319
SEQUENCE DESCRIPTION:
GATCATAAAT CCCTGTCTAA A 21

SEQ ID NO:6160
SEQUENCE LENGTH:122
SEQUENCE TYPE:nucleic acid
TOPOLOGY:linear
CLONE:HUMGS07320
SEQUENCE DESCRIPTION:
GATCAAACGT GCTTAAGAGC TAACTCGTGA CACTATGCAG TATTGTTTGA AGACCTGTTG 60
TTCAACCTCT GTCTCTTTAT GTTAACTGGA TTTCTGCATT AAATGACTGC CCCCTTGTTA 120
AA 122

SEQ ID NO:6161
SEQUENCE LENGTH:125
SEQUENCE TYPE:nucleic acid
TOPOLOGY:linear
CLONE:HUMGS07321
SEQUENCE DESCRIPTION:
GATCTCTGAC TCATTGTGGC ATATGAGGTG TAAAATNTTG TCAAAAAAGA ATCTGGTGTT 60
TAAACCAAAA TCACACTATG GTGGCATTAA ACCGTTCACT TTTATTCTAA ACNNNANCCA 120
NCGCN 125

SEQ ID NO:6162

SEQUENCE LENGTH:41
SEQUENCE TYPE:nucleic acid
TOPOLOGY:linear
CLONE:HUMGS07322
SEQUENCE DESCRIPTION:
GATCTGTCCA CGACATGGAA AATAAACTGG ATTTTCAGAA A 41

SEQ ID NO:6163
SEQUENCE LENGTH:137
SEQUENCE TYPE:nucleic acid
TOPOLOGY:linear
CLONE:HUMGS07323
SEQUENCE DESCRIPTION:
GATCCTATAC GTGAAGGAAC ATATTTAATT TCCTCNCTTT ATATTTCCTG GTTAAAATAT 60
CGTCATTATA GTTAGCAATT TGGAATCTGG CTTACATTGG TTGATACAAA TAANTAATAG 120
ANTANNGCAA NATCAGN 137

SEQ ID NO:6164
SEQUENCE LENGTH:88
SEQUENCE TYPE:nucleic acid
TOPOLOGY:linear
CLONE:HUMGS07324
SEQUENCE DESCRIPTION:
GATCTTTTTT CTATGCGTAA TCAGACATAC ATATATACTG CAGTGTATCT CACGTATTAA 60
TTTTAAAAAA TCTTTTGTTT TACTTAAA 88

SEQ ID NO:6165
SEQUENCE LENGTH:274
SEQUENCE TYPE:nucleic acid
TOPOLOGY:linear
CLONE:HUMGS07325
SEQUENCE DESCRIPTION:
GATCTCTGTA TTTGTNTTTC AACTAACAAC TAATTTNAAA TGTATTGTAT CTTTAATNAC 60
CTGTNCTCTT AGATTGTNTT TTGCTAGTAA CCCTTACTCA GCTTCTGCCT TTGGGGCTTG 120
GACTAATATT GCTTGTATGG AACTACAGTA CTGTGACTAA ACATGGAGCT CAATGCAGCT 180
ACTGCTTACA ACTGCTTAAA CTTTGTAGTG TGGACTTCAT TTTTTTCTGT AATAACANCT 240
TATTAAATCC TNGGTGGTAT GGCGGTCCTG GAAA 274

SEQ ID NO:6166
SEQUENCE LENGTH:310
SEQUENCE TYPE:nucleic acid
TOPOLOGY:linear
CLONE:HUMGS07326
SEQUENCE DESCRIPTION:
GATCATGTTC AGAAAGGGAC TCATTTGGTC CCACTTTGTA ACATGGGAAG AGTAGAAGAG 60

AAAATTAAAT GTGGAAATCA TAAATGCTTT TCTAACAGGG TTATGCTATT CTGTAACACT 120
AAATGTCTGT CTTTTCTCTT AAAAGATTAA GTTTTATTAA TTTTGTCATA CATTTCTCTA 180
TTTGANTATA TCTTAACCAT TTTATGTTCC AAATTCGTTT TTTGTCCAAG ACATTCTATC 240
AGANTTTTNC TTGCCTCTAT AAATCTGAAA ANTGGGTTCT AGAATGTCCC ACTTGCTGTC 300
TCTNAGAGTN 310

SEQ ID NO:6167
SEQUENCE LENGTH:307
SEQUENCE TYPE:nucleic acid
TOPOLOGY:linear
CLONE:HUMGS07327
SEQUENCE DESCRIPTION:
GATCCGCCCA CCTCAGCCTC CCAAAGNGCT GGGATNACAG GCATGAGCCA CTGCACCTGG 60
CCTTAACCCC TCTTTAGATT GGAAAAAATA NTTACAACTT TAAAAATAGC TTAGTGTTGA 120
ACCCTTTGGT AAACTAAAGA CCCTTTTATA ATGCACATAT TCCCAACANG GTTAATATAT 180
TTNGTGAGAT TAAACAAGGC TTGTATATGC TTGACCTTTC TTAANNTATG TCCATGTCAT 240
ACTATTATGA NTGTACATTT TTATGAGTCA TNANTATTNT TTTCAAAANG CGCTNCAGGC 300
CCNTGAN 307

SEQ ID NO:6168
SEQUENCE LENGTH:74
SEQUENCE TYPE:nucleic acid
TOPOLOGY:linear
CLONE:HUMGS07328
SEQUENCE DESCRIPTION:
GATCAGAAAA TGTGTGACCT TTTTAAAGTT AAATTGTTAA AAAATTTTAA CTATTAANCA 60
TTTTAAATAT TAAA 74

SEQ ID NO:6169
SEQUENCE LENGTH:71
SEQUENCE TYPE:nucleic acid
TOPOLOGY:linear
CLONE:HUMGS07329
SEQUENCE DESCRIPTION:
GATCTCAGAT GCTAGAAATA GTAGACAAAG ATGTTATTTC CCAGTGCAAA TAAAGAAATA 60
AAGATAGCAA A 71

SEQ ID NO:6170
SEQUENCE LENGTH:306
SEQUENCE TYPE:nucleic acid
TOPOLOGY:linear
CLONE:HUMGS07330
SEQUENCE DESCRIPTION:
GATCTCAATG TGCAAACTCC TTGGACTCCA GACCCCTGTC TGGTTGGCAC TCCCAGCAGT 60
GTGTGGTTTC ATCAGGAAAT CCTTGCAGCA GGGGTTTAGA TGGTTCCTTC AGGAGGTGGG 120

NAGCCTCCAT AGCATTGAAG GCCAGCCTGC ATCTCGCGGT GTGTGTCCAC TTCTCCNCTC 180
ACTTTCCCCT CACCCATCCC TTTAAAGTGA TTGGCTGTNA GGNGCACAGA GGGTGGCAAT 240
TCCATCTGTN ATGGGNTCAG AAGGCATTNG CCGCCATTNT TGATTTNACA TTAAAACTGA 300
TGNAAN 306

SEQ ID NO:6171
SEQUENCE LENGTH:65
SEQUENCE TYPE:nucleic acid
TOPOLOGY:linear
CLONE:HUMGS07331
SEQUENCE DESCRIPTION:
GATCTGTCTG TTGTTGGAAT TGAGATATTA AAATCCCCTA CTATTATAGC GTTCCTCCAG 60
TCAAA 65

SEQ ID NO:6172
SEQUENCE LENGTH:33
SEQUENCE TYPE:nucleic acid
TOPOLOGY:linear
CLONE:HUMGS07332
SEQUENCE DESCRIPTION:
GATCTTCCAG CACCAAATTT AGATGGCCTC AAA 33

SEQ ID NO:6173
SEQUENCE LENGTH:218
SEQUENCE TYPE:nucleic acid
TOPOLOGY:linear
CLONE:HUMGS07333
SEQUENCE DESCRIPTION:
GATCAAATGT GTGAGAAAAT CAATGGGTTT TAAAGACTAT TCTATGTCAA CTATAACATT 60
TAATTTGGGG ATTTCTGTCC CTTATAATGT CTACCTCATT TTGGATGGAA TCCTTGAGGC 120
CTGGTTTATT TTCCTTTNCC TTNCTACACA TCGCTGCTCA GAGTGATGAA TGGAGTTGTG 180
TTTTGAATAA AATATCTATG CATCCNTTTG TGAGCAAA 218

SEQ ID NO:6174
SEQUENCE LENGTH:186
SEQUENCE TYPE:nucleic acid
TOPOLOGY:linear
CLONE:HUMGS07334
SEQUENCE DESCRIPTION:
GATCATGTTT TAACTAACTT CATTTTGTTG CTGAATCATA ATNNATGCTC TAATGACCTT 60
TTCTTTCCTA TCCCAACCCT GTGTAATCTG CTCTATTTAG GGCTAATTGT TCCTTGTCTT 120
GTATGTATGT AATGTGTCCT NGTNTTCTTC CTCCTATTTN NTNCTCATAA AACTCCATCC 180
NNTTTN 186

SEQ ID NO:6175

SEQUENCE LENGTH:152
SEQUENCE TYPE:nucleic acid
TOPOLOGY:linear
CLONE:HUMGS07335
SEQUENCE DESCRIPTION:
GATCCATTCA ACTTGTGTGT ATCAGTAGTT AATTNCTTTG TÀTTGGTCAG CATTCCATTA 60
TTTGGAATTT ATACAGTATG TTTATCCATT CACCTGTTGA AGGACATTGT TTCCAATGCT 120
TATTACTAAT AAAGCTGTTA TGAACATTCA AA 152

SEQ ID NO:6176
SEQUENCE LENGTH:236
SEQUENCE TYPE:nucleic acid
TOPOLOGY:linear
CLONE:HUMGS07336
SEQUENCE DESCRIPTION:
GATCTGTATT TAATGAGGAG GAGCCGAGGC CCCAGCTTCA TCCAGCTTCA ACCAATGCCT 60
GGACCTGTCC ACCTGANAGG CCCCTGGGGC TCCCCAGCTG CTGGCCAGAC CCTGGCGCTG 120
CCACAGTCCT GGCACTGCCC AAGGCCATAC CTGNCTAGCC CTTTGGNTCC ATCCTGTGGA 180
TGCCCACTCA CCCCTNAGAC TCCTNCTGNC CATGCTGTGG NCGGACTTGT AAGCAN 236

SEQ ID NO:6177
SEQUENCE LENGTH:148
SEQUENCE TYPE:nucleic acid
TOPOLOGY:linear
CLONE:HUMGS07337
SEQUENCE DESCRIPTION:
GATCATGCCT GTGTTTACCC TCTAAGCTGA AGCTGCTCAT CAACGGTGAG ATGGCAAAAA 60
GGTGGGTCCA GAAGAGGGGA AAAGAAGGGA GTCTGTGAAA ACAAAATGCT GAAGANTCTG 120
CATCAAATAA ACCCTTCCTT CCTTCAAA 148

SEQ ID NO:6178
SEQUENCE LENGTH:155
SEQUENCE TYPE:nucleic acid
TOPOLOGY:linear
CLONE:HUMGS07338
SEQUENCE DESCRIPTION:
GATCTCCTTA TTGACCACAG ATTATTCCTC TGTTCAGCAC TCCTTATTCT TATCCCTGTT 60
ACAATTCTTA TTATGGTGCA CAAGCTCCAT AAATATTTAT AAAACTGATA TTAATTGANT 120
AAAATATTGT CTAGAGGTCT CAGTCATTCT TCAAA 155

SEQ ID NO:6179
SEQUENCE LENGTH:198
SEQUENCE TYPE:nucleic acid
TOPOLOGY:linear
CLONE:HUMGS07339

SEQUENCE DESCRIPTION:
GATCATCTAG ACATAATTNC TGAAGATGAA CTTTTGGACC AACTTCTAAG TCACACAGCA 60
TCATTATCAG ATTTATTACA CAACGNCTTT TTNGTTTTNA CTCTATTTCT GAGGAAAAAG 120
CCTTCCCGAA ATCCGTAATG ANTTTNTCCA TGGTAACCCC TCTCCTGTTT TNACACAGNA 180
AAGTTTCTCT NGACTGTN 198

SEQ ID NO:6180
SEQUENCE LENGTH:188
SEQUENCE TYPE:nucleic acid
TOPOLOGY:linear
CLONE:HUMGS07340
SEQUENCE DESCRIPTION:
GATCGCTGGT TTCCTCTGCA GCGCGAGGGC TCCGGCGACC AGAGGATTCT NCCCGGAAGG 60
CATTCCTGCC GCGCTCCCNG GGGCACCCCT CAATTGTGTA CTACGTCCTT GTTTAGTGTG 120
TATCCGTGCC CACGTAGATG ATGTCTGTAA CGNAGTTTTG TTTGAAATAT GAGNATATGC 180
GGCTTAAA 188

SEQ ID NO:6181
SEQUENCE LENGTH:135
SEQUENCE TYPE:nucleic acid
TOPOLOGY:linear
CLONE:HUMGS07341
SEQUENCE DESCRIPTION:
GATCAGTTTA TTATTGATAC ATCACTCCAT TAATGTAAAG TCATAGGTCA TTATTGCATA 60
TCAGTAATCT CTTGGACTTT GTTAAATATT TTNCTGTGGT AATATAGNGA AGAATTAAAG 120
CAAGAANNTC TGAAA 135

SEQ ID NO:6182
SEQUENCE LENGTH:226
SEQUENCE TYPE:nucleic acid
TOPOLOGY:linear
CLONE:HUMGS07342
SEQUENCE DESCRIPTION:
GATCAGTTTT CCNAGTCTTT GGGAAACGGG TTGTGTTTTT TNCCCTTTGC CGTGTTGGAT 60
TTCCGGTCCC GGCTGACACC TCCCTTCGTG TGTGCGNCCC TCCCCACCCC TGCCTTTGTN 120
TCGGTGCCCT GTGTCCGTTC TCTNATGTGG GGATGTTTGT GCTGCAGACA AAAAGAACAA 180
NAAAAANTTT TTAAATACCA CAAAGATGGN ANAAAANGAC AAAANN 226

SEQ ID NO:6183
SEQUENCE LENGTH:97
SEQUENCE TYPE:nucleic acid
TOPOLOGY:linear
CLONE:HUMGS07343
SEQUENCE DESCRIPTION:
GATCGTCACA TGCCGGGGAG CAATGTGGAT GGCCTGGGGA CTCCTGGGTT TTCTCCCTCC 60

CGACTCCCTA ATAAACCCCG TGAACCTTGG AGCCAAA 97

SEQ ID NO:6184
SEQUENCE LENGTH:321
SEQUENCE TYPE:nucleic acid
TOPOLOGY:linear
CLONE:HUMGS07344
SEQUENCE DESCRIPTION:
GATCACCTTN AGTNCTNAGC AGGGACTAGG CTTGCAGGTA ANATAATGGG CCAGGGCACC 60
CAGTCCAGAA GGAGCAATGG CACCTGGGCA GTGCCAGGCC TTAAAGCCCG CTGCTCCTTT 120
NCGGTAGAGG AGAGGCCCAT CACTGGTGTG GTGGGGTGGG CTCTCCCTTA GGCTTGGGCA 180
AGGCAGCCAC CTGCCCTTNC TCTCCCTTAG TGTTCCCTGG CCTCCCTGCC ATCAGGTTGC 240
TGGGAGTGGA GATGGAGGGA TTATTGAGCA GAAAATNAGT TGGATGGAGN TAAACAGNTC 300
CCATCCCTGG GTAATGGATT N 321

SEQ ID NO:6185
SEQUENCE LENGTH:292
SEQUENCE TYPE:nucleic acid
TOPOLOGY:linear
CLONE:HUMGS07345
SEQUENCE DESCRIPTION:
GATCAGCTTG GAAGCTTAAA GTCCAATCCT TACTTCGAGT TTGTTTTGAA AGCAAATTAT 60
GAATATNATG TTCAGGGACA AATATAACTT TTNCTAAAAA TGGCCATTGT TTATGAAATC 120
TGTATAAGTG TGTCCTTATA CAAATTTNAG GCCATAAACA AGTGTAAGTT TGTACAATTT 180
CATAACATGT ATAGCTGAGT TTTTATACTT TATATGTAGG AAGCTAATAT AAAATAGTTA 240
TGTAACTGTG ATTTTGGTTT TCAGTTATGT GACTTGTTTT TNCCACCNGA AA 292

SEQ ID NO:6186
SEQUENCE LENGTH:155
SEQUENCE TYPE:nucleic acid
TOPOLOGY:linear
CLONE:HUMGS07346
SEQUENCE DESCRIPTION:
GATCCATAAC AGAGATNCAN AGAGGCACCG TGGAGTTCCA GGGTCATCGG TCAGCGAGGA 60
ACAAGGAGGG AAAGGTGTCT TCCTGCCCCT TGATGCTCAA CTAAGCATCT GTTCCCTAGA 120
AATACATGTG TCCAGGNNGN NTCCATGGGC TTTNN 155

SEQ ID NO:6187
SEQUENCE LENGTH:301
SEQUENCE TYPE:nucleic acid
TOPOLOGY:linear
CLONE:HUMGS07348
SEQUENCE DESCRIPTION:
GATCTTNAGT TGTTTTAATT TTTTTTTCT GCATCTGAAT CTGCATGATT TCCAAACCCT 60
GTACCATCTG AATTTTGCAT TTNAGCACTT GCACTATTAC TCAGCAGCAG TAACATGGTA 120

ACACTTAAAA TGGTACTCGG GGACCTCCAA AGACTAAACT GNCANGCCTT CAAGGNGCCC 180
AGGGGTACGT TANCTTGTCA ACGGCATGGT TTAATCCCTT CTTTACACTT GTGTAAATTT 240
CAGTTACTGG TCATAGNNGG CTTTCAATGT TGCGTGGCCT NTTATTANCN TGTTTATGGG 300
N 301

SEQ ID NO:6188
SEQUENCE LENGTH:203
SEQUENCE TYPE:nucleic acid
TOPOLOGY:linear
CLONE:HUMGS07349
SEQUENCE DESCRIPTION:
GATCTTTATC AATATTCTGG ATTAGACAAC AAATNACCTT TCTGGGTGTT TCTTGTAAAC 60
TATACTCCTG TTTGAATGTT AAACTTTGTT GCTAAAGTTT AATTTTAAGA TGTTTGAATG 120
TNCAGTTTAT GTATTTGANC TACAATAAAC CAACCCTTTT NATATATCTG TNTNGTATAT 180
GATTATNGTN ACTTAATTNT NAN 203

SEQ ID NO:6189
SEQUENCE LENGTH:316
SEQUENCE TYPE:nucleic acid
TOPOLOGY:linear
CLONE:HUMGS07350
SEQUENCE DESCRIPTION:
GATCAAAGCA GGTGGGGCTT TGGGATGTGA AGTGGGTTCT NACGCGGGGC CCCACAGACC 60
TGTCTCCGCG TGANTGCCTG CAGGCCCCTG CGTCATTCCT CAGCTGCGTG CGAGGCCTCT 120
GGCTTGGAGA GCCTCCCCGC TGTGCACAGT TCTCTCTTTG CCAGGGCAGG GCTTGGGGCA 180
GCACCAGGCT GGGGCAGAGG AGGAAAGCAA GAGGACAGAC CTCCAGAAGA GCAGCGGAGG 240
GCGGGTGAGG ATTTCCCCAT ATACCAGTGT GTGTTCATCT ACCCATGAGG AATAAAACCA 300
TGCAGCAGCA TGCAAA 316

SEQ ID NO:6190
SEQUENCE LENGTH:129
SEQUENCE TYPE:nucleic acid
TOPOLOGY:linear
CLONE:HUMGS07351
SEQUENCE DESCRIPTION:
GATCCCCAGN ACTGCAGCCG AGCCCCGGGC TTCCTTTCTT ACCATTCTGT ATGCTTCCAA 60
GGTGTGACCA TTCAAACTAA CNNTATTATT AAGATTATTA ATAAAGATTT CTTTCTTCAA 120
ACCAGGAAA 129

SEQ ID NO:6191
SEQUENCE LENGTH:293
SEQUENCE TYPE:nucleic acid
TOPOLOGY:linear
CLONE:HUMGS07352
SEQUENCE DESCRIPTION:

GATCAGAATC AGAAAACAGG TAAACCTCAC TCACACATTT GGACTCATTT GAACAAAAAT 60
CTAGGCCAAA ATACTGAAAA GCCTATGTGT TTTTTTAATT GGAAGTATAT GTAAGGTTAA 120
TGCATTTAGT GAACGTGACT AACAAAGACT AATGTGCACA TTAACAGNTG TNCTTTTTAA 180
GGTTTTATGG GAGGCTGTGC ATTGCTCAAA AGCTGTTGGG AACGCCTTCT GACCAGTTGC 240
CTTCAGAACT AGTTTGNGCT GCTCANTNAA AANCCAGTGN NTTTACTCNN GGN 293

SEQ ID NO:6192
SEQUENCE LENGTH:52
SEQUENCE TYPE:nucleic acid
TOPOLOGY:linear
CLONE:HUMGS07353
SEQUENCE DESCRIPTION:
GATCTGCATG TGTGACACTG ATTCTTTGGA AATAAAGAGT GGAAGCTGCA AA 52

SEQ ID NO:6193
SEQUENCE LENGTH:236
SEQUENCE TYPE:nucleic acid
TOPOLOGY:linear
CLONE:HUMGS07354
SEQUENCE DESCRIPTION:
GATCTAAATT TTAAAAATAG GAATGAATCT NTTAATTGCA GCTTGTGGAG AGTATAAAAC 60
ATTTTGAGCA CAATATGCAG CACATAACTT TTCAGATTGT GTTTGCATAG AAAAGGTTAA 120
AATCAATTCT TTCAGTTACT GTATTGTTCA TTTCTTGTAA AGGTCTTCCT ACATTTGAGA 180
CGAGACCCTT TAACATAGGC TGCCTTAGTA ACAAAATAAA ACAGATGTGT CATAAA 236

SEQ ID NO:6194
SEQUENCE LENGTH:282
SEQUENCE TYPE:nucleic acid
TOPOLOGY:linear
CLONE:HUMGS07355
SEQUENCE DESCRIPTION:
GATCTAGAGG GNGGATTTCC AGCCAGGGCT GCTAGACGGA GGCCTACTCT TCCATCTTTC 60
CTGATGGCAG GATGGCCTGG CCAGGGCCTG GAAGACAGAG ACCTCCTGCC TCCGCCTCAG 120
TAAGACGACA AGGAAAGGCA AATGCCCAAG GGAAAGAAAA GGAAGGCTCT TCTCCCCAGA 180
GTTCCCCATG CAGACATGAG TGCGTGCTCA GTTCAGANTC ACTTCCGAGA ACTCATCCCT 240
AATGCTGCAG ATTTGGGCTG GAACAGATTC ACACTGTCTG TN 282

SEQ ID NO:6195
SEQUENCE LENGTH:158
SEQUENCE TYPE:nucleic acid
TOPOLOGY:linear
CLONE:HUMGS07356
SEQUENCE DESCRIPTION:
GATCTTCATC TCTTGTTTGC TCCACTTGTA TTCTCTTTTC AGTAACTGGA TTAATGCTTT 60
GAGCCCAAAA TGAGATATAT TTNTAATTAC TGTAGATTAC ATGTTGTTTG GCTGGGAATA 120

CAATACAATA AACTTTATTT TGCATAATGC ATTTCAAA 158

SEQ ID NO:6196
SEQUENCE LENGTH:113
SEQUENCE TYPE:nucleic acid
TOPOLOGY:linear
CLONE:HUMGS07357
SEQUENCE DESCRIPTION:
GATCTGGATT ACTATGTAAA TTCACAGNAG TACCGCTAAT ATAATTTTTG TTGCNTGTAT 60
TANCATCATC TGGTCTGNAA ATGTGGGTTT TTATTTGGCA CATTTANATA AAN 113

SEQ ID NO:6197
SEQUENCE LENGTH:323
SEQUENCE TYPE:nucleic acid
TOPOLOGY:linear
CLONE:HUMGS07358
SEQUENCE DESCRIPTION:
GATCTGGAGC CACAGTCTGT NTATCTTCCA GTTCATCTCA GTCCTCGAGA AAGGCCCTNT 60
AAATATGTAA CTTTCCCATT TNCCTTTAAC CATGGGTTGT GTGAGCCAGA AAGAGCTTTG 120
AGAAAGATGG CTGCTTCCAC CAGGGNGGAG GCTTCTNGGT CTGCATGATG ATGGGGCCCG 180
TTTCTGGCCA NAGGGTGGCT CTGGGAGCAG TTGTNCTGCG GGCTTGCTGG GGGCGAACTC 240
TAACTGTTGC AGAAACAGNG CTTNATGGCT TGCTNNGGGT ACTTAGCTGG AATATTTTAA 300
AGTGTCAGNT AAATGTGATN TNN 323

SEQ ID NO:6198
SEQUENCE LENGTH:50
SEQUENCE TYPE:nucleic acid
TOPOLOGY:linear
CLONE:HUMGS07359
SEQUENCE DESCRIPTION:
GATCTGTATG TACTTTTAGT TGTATTAATA AAGTGACAGT GAGTGTCAAA 50

SEQ ID NO:6199
SEQUENCE LENGTH:149
SEQUENCE TYPE:nucleic acid
TOPOLOGY:linear
CLONE:HUMGS07360
SEQUENCE DESCRIPTION:
GATCACCCAG TCTTAAGGTT TCAAAAACTC TTTGACATTA GATTTCACAA CTGCACAATT 60
GANCTTATTG GCCTGTAACT TATTTNCTAA ATGCTCAGTG CTATTTATAT ACTACAGTAN 120
TTNCCTGTTA AGNNGGCAGT TGTAAAGNN 149

SEQ ID NO:6200
SEQUENCE LENGTH:329
SEQUENCE TYPE:nucleic acid

TOPOLOGY:linear
CLONE:HUMGS07361
SEQUENCE DESCRIPTION:
GATCGGACTG GAACTGTTCG GCTGCNACCA GAAATTNATT TTCCTGAGTA AATTGCCGAG 60
AATTAAGAAT GAAGAGGGCC ATTTGCATCT CCTTAAATTA TTCAGTTACC TGCTTTATTG 120
CTCCATGTGG AAAACTTAAA ATTGTTAAGT TGTGCATTAC TGTATTTNAN CTTGTTGCTT 180
AGTTTCTNCA TGTTTATTTN CAGTAATGGC TGANAGTGTT AACTGTNCCA TACTTTTNGC 240
ACAATGTNCT GCATAAGGTT ACCTGTGTAC AGAGTTTTAC TTTAGNTTAN CTAAAATATT 300
GCCTGGGTTC AGTNTTNATT TCCATTNTN 329

SEQ ID NO:6201
SEQUENCE LENGTH:150
SEQUENCE TYPE:nucleic acid
TOPOLOGY:linear
CLONE:HUMGS07362
SEQUENCE DESCRIPTION:
GATCTAGGAT GTGGNTGTGG AGTACTTAGC ATCGGAACTG CAATNTNAGG AGCAGGGTTG 60
TGTGTTGGAT TTAACATAGA TGAAGACGCA TTGGAAATAT TTAATAGGAN TNCAGAAGAG 120
TTTGANTTAA CAAATATTGN CATGGTTCAN 150

SEQ ID NO:6202
SEQUENCE LENGTH:142
SEQUENCE TYPE:nucleic acid
TOPOLOGY:linear
CLONE:HUMGS07363
SEQUENCE DESCRIPTION:
GATCCAGGCG CCTGGGCAGA GCACCTGCTG TCTGCCCTGC ACTTTGAAGG GTTGTCGTGG 60
GGATTGAAAT NAACCAACTG CCGGCTGAGC GGAGGCTCCC TGGGAGTNAC AAGTTCCGTA 120
TTAAAATGCT TTCAGTGGNA AA 142

SEQ ID NO:6203
SEQUENCE LENGTH:294
SEQUENCE TYPE:nucleic acid
TOPOLOGY:linear
CLONE:HUMGS07364
SEQUENCE DESCRIPTION:
GATCTATAAA ATTTCAGTTT TAAAAAATGT GTTAAGGCTT TGTTTTTGGC CTAACAGGTG 60
GTCTATAAAG AAGNAAGTTG TATGAGGTAA TGAGAAGGCT GTGTATCCTG ATGTTGTCGA 120
GGAGTGTTCT CTATACCTTC ATTAGAAATA ATTGTNTTTT TACTGCCTTC TAGTCTTCTG 180
TTTCCTTACT AATATNCTCT CTTGTNTTAT TACAGAAAGT GGNGTATTGC AATATCCTAC 240
TATAATTGTN TTGCNCTCTT TGTGTTNATC CCAGTNTNNG TNAGTNTANG CTTN 294

SEQ ID NO:6204
SEQUENCE LENGTH:306
SEQUENCE TYPE:nucleic acid

TOPOLOGY:linear
CLONE:HUMGS07365
SEQUENCE DESCRIPTION:
GATCCCAGCC CGGCCTTTGT GGGACTAAGG TGGCGCTGAG TGGGAGAGCA GCAGGCGATG 60
CGCGAAAGGC GGGACTTGGA GGTTCTGGAT GTTCCTGTGG ATAACCTCAA GGAGCACGGN 120
ACCTGGGACA TGACTGCCCC CAGCATTCCT TCTGGCGGCA GACAGNGTTT GTNAGTTCGC 180
CAACATCCTG CTTTCATGNG AACAGTTTGC TGTTTGCTNA CAGAGCCTCC AGTNGTATAC 240
TGAGTTGGTC ACGACCNTNA TTNCTTCGNN CCTACAAACA TNTCCCACTT TTCGNTTNTN 300
TGGTTN 306

SEQ ID NO:6205
SEQUENCE LENGTH:146
SEQUENCE TYPE:nucleic acid
TOPOLOGY:linear
CLONE:HUMGS07366
SEQUENCE DESCRIPTION:
GATCTTCAGT AAATATTTTG AAATGAATTC ATTTATGGAA GAATCAAANC TAGGTCAGAT 60
TGTCCAAACC AGACTAAGAA ACTGCATAGG GTNTTTTATG AGGTNTTTAA TGACTCATCA 120
GCNCCATTAT ANCTGANATT NCTACN 146

SEQ ID NO:6206
SEQUENCE LENGTH:298
SEQUENCE TYPE:nucleic acid
TOPOLOGY:linear
CLONE:HUMGS07367
SEQUENCE DESCRIPTION:
GATCTCACTG CCCAGGCTGG TCTTGAACTC CTGGCCTCAA GCACTCCTCC TGCCTCACCC 60
TCCCCAAGCG CTGAGATATA GACCTGAGCC ACAGCGCCTT GCCAGTTGTT TTATGTTCTT 120
TATCCTTTTA CCTGCTTGCC TTCTGTAACC TGGGTTGCAT TAAATTTACT CCTACTNGTG 180
AGTAAATGTG AACCATAGCA ATAAATTTAG TCTATCCCCA GAACCTTCAG TAGCAAGGAC 240
CNATATTNCT ACGGTATTTG TNTGTGTCCA NANANAAATA AAATTACCTT NAAAAANN 298

SEQ ID NO:6207
SEQUENCE LENGTH:28
SEQUENCE TYPE:nucleic acid
TOPOLOGY:linear
CLONE:HUMGS07368
SEQUENCE DESCRIPTION:
GATCATTAAA GATAAACATA TTTTTAAA 28

SEQ ID NO:6208
SEQUENCE LENGTH:205
SEQUENCE TYPE:nucleic acid
TOPOLOGY:linear
CLONE:HUMGS07369

SEQUENCE DESCRIPTION:
GATCCTGTTG GCAANGGGAA TGTAGAGCTT CCAGGGTGCA TTGCCCACTG TTCTCATTGC 60
ATCCCAGTCC TCCAAGTCAG TCTAGCCTTA CTGTACACTT GACAAGTGCT TACTCAGCAA 120
GTCCCAGACC CACGNCCTNT TATCTCCNAA GACTGGNTTT GGCCATGNCC TTCCTCATCA 180
GGCCTATGCG CANNANTATN TAGGN 205

SEQ ID NO:6209
SEQUENCE LENGTH:302
SEQUENCE TYPE:nucleic acid
TOPOLOGY:linear
CLONE:HUMGS07370
SEQUENCE DESCRIPTION:
GATCCATTTA ATATGTTATC CCCACTAATT AATTTTCGTA TATNATTTCC AATATTTGGA 60
AAGCTCTTTA TAGCCATTTG GTATTTCCTA TTACCCACCT CCTATTTTAA ATATTTATCA 120
GTCTAAACTT GTGCAGTGTA GTAAACATGC AAGTTGTTAT GCTTGAGCTG TATTACCATA 180
AGTNGCNTTT TANGTAAACT GGTGNNTTTG GGCCATAGAT GTTTTTGCTT TTTGTTTGAT 240
TTTNTNTTNC AGGCTAACTG TTNGNGGTAT ACATNTATTT NTCTGTTGTA CAGATTTGAA 300
NN 302

SEQ ID NO:6210
SEQUENCE LENGTH:301
SEQUENCE TYPE:nucleic acid
TOPOLOGY:linear
CLONE:HUMGS07371
SEQUENCE DESCRIPTION:
GATCCCCCAC ACCAGCGCCC AAGAAAGGAC CAGCGTTGCA CAGCCCGGTA GAAAGGGTTT 60
ATTTATGCGC AACGGTTCAC ACAAGCCTTC CTGAAATTCC ACTTTACAGT AAATAAAGCT 120
GTGCGTTTCC CCTTCCCATG CACAACTGCG TATCAATCTA CAACTGTCAT TTAACTGTGA 180
AAAAATAGNG CGTCTCCCCT TTTGTCATCG TTCTGGTAAC ATTTGGCGTA GCATCTGACA 240
GCACGGAGNT GCTCACTCCT GGACCGGTTA TTTGGTTAAA ACCCAAAATG TTAGGTCGGA 300
N 301

SEQ ID NO:6211
SEQUENCE LENGTH:111
SEQUENCE TYPE:nucleic acid
TOPOLOGY:linear
CLONE:HUMGS07372
SEQUENCE DESCRIPTION:
GATCCATTTA ACTATTCATC AGAGGTGCTG TAATTTTAAA TTGTCTCTTG TCTCCTTCAG 60
TTAATTTTCA GAATTAAAAA CATACCATGG GNAAAAAAAA CNCNAANCAN N 111

SEQ ID NO:6212
SEQUENCE LENGTH:206
SEQUENCE TYPE:nucleic acid
TOPOLOGY:linear

CLONE:HUMGS07373
SEQUENCE DESCRIPTION:
GATCCTGGAA ATNAGATGGG CCTTACTCTG TCGACTAAAT GAATAGCTAT TTTCTTGTCA 60
TTTTTTAAAG TGCAACTCTT GCTTCATGCT GCTTAAGTTA CCAGATGAAT GCTGNGAAAT 120
AAGTAATCAC AGNCATTTTA ATACCATTTC ATTGCTGTTT TACGCGTGTT CATTACTTAA 180
CAAAAAATTA TCTTTTAGCT TTTAAA 206

SEQ ID NO:6213
SEQUENCE LENGTH:101
SEQUENCE TYPE:nucleic acid
TOPOLOGY:linear
CLONE:HUMGS07374
SEQUENCE DESCRIPTION:
GATCTAGCCT GCCCTGCCTG CCTGCCCTGG GCGATGAGGT ACGGTGGGGA AGGTGCCTAT 60
TTTAGAGAAC TTTGTCACAG TATTAAAGTT CCCAGAACAA A 101

SEQ ID NO:6214
SEQUENCE LENGTH:252
SEQUENCE TYPE:nucleic acid
TOPOLOGY:linear
CLONE:HUMGS07375
SEQUENCE DESCRIPTION:
GATCTAGAAT GTAACTAAAT TGCTTATAAA TGTCAGAGCA TTTTTNAAAG GTACAGTATA 60
TGGGGATTGT NTCGTTTTTC CTAGCAGGGG AACCTTAGTT AATANTAAAA TACTACTTAT 120
TTGAGTTACT GNTACAGNTT CATTTAAGGC TTGTGTGCAA ATGTGGTCTC ANTCTTTTTT 180
CCCTCCATGA TTTTCCTATG TGCTTCCTCT GGCATTCACT GNGGTTTTGG TAAANTAANT 240
TGCCNNTTAA NN 252

SEQ ID NO:6215
SEQUENCE LENGTH:257
SEQUENCE TYPE:nucleic acid
TOPOLOGY:linear
CLONE:HUMGS07376
SEQUENCE DESCRIPTION:
GATCAGGAGT ACCTCCCTCA AGAGTCGAGG GNGGTAGATT TCTACACGCA GAGGAGGTGC 60
TGACTCCTCA GGGCACGCCA GCAGTNGNNT GTTGCTTTTN TAAGGGAGGG CCACTAAGAA 120
AGTTAGTGGA TTGCAAAGTT GGTTGCCTGG TGGNAGTTAG ANCAGACCAA CATTTTATGC 180
CATAACTTTA NGAGTTCCAT AGGTGGCGGC GCAGTTCTTN CGNATCAATG TNTAACTTTG 240
NGGGGGGTGT TCATTTN 257

SEQ ID NO:6216
SEQUENCE LENGTH:189
SEQUENCE TYPE:nucleic acid
TOPOLOGY:linear
CLONE:HUMGS07377

SEQUENCE DESCRIPTION:
GATCAGACAT AAACTTAGCA TCTTAATGGA AGAAAAATGA GGGGAACTTC AATTATGATT 60
TATTAAAGAC AATTTCTATT ACACCCTCCT TTATGACAAG TGACATTTTA GATGTAAAAG 120
TAAAANCTTT ACCATGCCTT TTTTTTTTTT NGTGGGCCTA CCTTNGGGGC NTNAANCCCN 180
GGGGNNCCN 189

SEQ ID NO:6217
SEQUENCE LENGTH:272
SEQUENCE TYPE:nucleic acid
TOPOLOGY:linear
CLONE:HUMGS07378
SEQUENCE DESCRIPTION:
GATCTCGCAA TGTCTGCCTT AATATTTCAC AATTAAACCT TTCATATTTA TTATAATTTG 60
GTAATATTCA TTAATTTTCC TGTCTTTTTA AAGTCTTGAT AGATTTGTCA AAGTATATGT 120
NCTTTTCCCT ATGCTGGTTT AGAAGTNCGA NATATCACTT CTAATAGTAA TTATGCTCAA 180
NTAGGTACAC ATATACATAC ATNTTTCTCT CNCNCTTTAT ATACTTNTGT GCAATTGGCA 240
CTTTTGACGT ATGTCATANN NNTGTCATNC CN 272

SEQ ID NO:6218
SEQUENCE LENGTH:74
SEQUENCE TYPE:nucleic acid
TOPOLOGY:linear
CLONE:HUMGS07379
SEQUENCE DESCRIPTION:
GATCCTCGGC CTCCCCAAGT GCTGGGATTA TAGGCATGAG CCACCGTGCC CAGNCTAATT 60
TCTAATTAAT TAAA 74

SEQ ID NO:6219
SEQUENCE LENGTH:303
SEQUENCE TYPE:nucleic acid
TOPOLOGY:linear
CLONE:HUMGS07380
SEQUENCE DESCRIPTION:
GATCTCGTCT GCTCACCTTA GCTTTCTGCC TTGGCAGCAC GGGCTGCGGA AGAAAGCACG 60
CTGGGCCAGG AGGCAGGGGT GCCCAAGCCA CAGGGAGCCC CTGGGGAAGC CTGCTCCATT 120
CTTCTGGTGA CCTTGGCGCT CCTTCACTCA TCTCCCCTGC CCCCTCAGGA ACTGGTGGCC 180
CAGCTTCCAC ACCNNCACCT CCCAGTCTCT AGNCTATCCA TCTGTCTGTG TATGGCCTGG 240
AGTCACTNCT TCCTCANGCC CCCAGGGNAA NGTGTGCTCA AAATAAAAAC CAGNGGNCTG 300
AAA 303

SEQ ID NO:6220
SEQUENCE LENGTH:205
SEQUENCE TYPE:nucleic acid
TOPOLOGY:linear
CLONE:HUMGS07381

SEQUENCE DESCRIPTION:
GATCCCCTTT CCCACATCCG TGCTGTGTCA TTGTTGCTCT GCTTCCTTTC AATGTGTCAG 60
TGCCTGGGGG GAGGGGAGGA GCACCCCCTC AGCCCCCCTG AACCTGACCA AAAGCCATGG 120
CTGTTGCTCC NNNCTTTGTA TGATGCAAAT GCTGAAATGT ACAAAATCAA CCATGACAAC 180
AANGAAAAAG GCCTTGTACA GCAAA 205

SEQ ID NO:6221
SEQUENCE LENGTH:110
SEQUENCE TYPE:nucleic acid
TOPOLOGY:linear
CLONE:HUMGS07382
SEQUENCE DESCRIPTION:
GATCATAAAA ATGAAGGAAA ATGCCCAAGC ATATCCCCAA CCCCTCACCC CACAACCACC 60
ACCACCACAC ATAANCAAGT TGCTGACATA NNAGGGGGAC CTTTTTNANN 110

SEQ ID NO:6222
SEQUENCE LENGTH:248
SEQUENCE TYPE:nucleic acid
TOPOLOGY:linear
CLONE:HUMGS07383
SEQUENCE DESCRIPTION:
GATCGCATCT CATTGTTAAC TCTTTACTGA TATGTTTGTA AATACAGAAG TGAAATGTGG 60
ACATAAAATA GTTACGCTAT TTGGTTAATG GTACTAGACA ACATGTAATT AATGACATTC 120
AAAAATTTAT GGCTAGTGAT ATATATAANG TAAAATTTTC TTTGCAGTAA AATATGCCCT 180
TNATTATNGN CGGGNGGCTA TANGGNNCCA ACAGTTTGTA TGAAANTAGC TCCNAATCAT 240
ATCTNTNN 248

SEQ ID NO:6223
SEQUENCE LENGTH:145
SEQUENCE TYPE:nucleic acid
TOPOLOGY:linear
CLONE:HUMGS07384
SEQUENCE DESCRIPTION:
GATCCCTTTA CACTCTTCAA AAGTGNGAAC CCCAAAGAGT TTTCATGTAT GTGGGTTAAA 60
TCTATTGATA TTTTCTGTTA GAATTTAAAA ATTATGTTAA AANAATAAGA NGAATGACTA 120
TTTTNCAAAA CAANANATGA GNTGN 145

SEQ ID NO:6224
SEQUENCE LENGTH:129
SEQUENCE TYPE:nucleic acid
TOPOLOGY:linear
CLONE:HUMGS07385
SEQUENCE DESCRIPTION:
GATCTGCTTG GTGAAGAGTT CAGGAAAGTA TATCAACAAA GGNGTGAANT TTGTGAAGAG 60
AAATNNTATC ATCAAAATTG GGTAAAGCAT AGNTTTTTTG TATGTTACCA CTAGATGTCA 120

GCATANCTN 129

SEQ ID NO:6225
SEQUENCE LENGTH:212
SEQUENCE TYPE:nucleic acid
TOPOLOGY:linear
CLONE:HUMGS07386
SEQUENCE DESCRIPTION:
GATCAGAAAA CATTGATTTA TATTTTAAAA TAGGATATTG GAGCCATTGT CTTAGATAAT 60
TTTCAAGGCA AGANCAAGCC TGCAATAAAT ANTGAGNCTT TGACATTCCT TGGCGCGGAC 120
GATGTCTCCT GCCCTANTAT GACTGTCACT CAGCAATTTC AATTTTATGN NTACTTGATT 180
TAAANCGGGA GGCTANACTT GNTCTGAAAN GN 212

SEQ ID NO:6226
SEQUENCE LENGTH:235
SEQUENCE TYPE:nucleic acid
TOPOLOGY:linear
CLONE:HUMGS07387
SEQUENCE DESCRIPTION:
GATCTTACTG AAGATGATGT TCCAGCAGCA GCGACTTAGC CCCAGGAGCC CAGTTTCAAT 60
GGCCTTGCTG TGTGGTGTTT CAAGTGCATT TAAAATGTGT GACACAGNAA CGGCACACTC 120
TTNCACATGC TTTTGNAGTA TTATAANNCA CTTTATTACA AATTTGTCTT NGCTATTAGC 180
AAATANAACT GATTATCATT CTTTATTAAC CCACCTNGGC CTTTTNNAAN CCTCN 235

SEQ ID NO:6227
SEQUENCE LENGTH:290
SEQUENCE TYPE:nucleic acid
TOPOLOGY:linear
CLONE:HUMGS07388
SEQUENCE DESCRIPTION:
GATCTCACTC TTNCCCAAGC TGGAGTGCCG TGGCAAACTC ACAGCTCACT GCAGCCTTGA 60
CCTCCTAGGC TCAAGCAATC CTCCTACCTC AGCCTCCCAA GTTGCTGGGC CTGTAGGTGG 120
ACACCANCAT GCCCTGTTGA TTTTNAAATT TTTTTTATAG AGTTGAGGTC TCGGTATGTT 180
GCCAACCTAG TCTCAAACTC CTNGGCTCAC GGCTGTAATC CCCAGCTCTC AGGGAGGNTA 240
AGCGGCGGNN GGNTAGCTTA AGCCNAGGAN TNCGAAGNCC TGNCTTNGGN 290

SEQ ID NO:6228
SEQUENCE LENGTH:249
SEQUENCE TYPE:nucleic acid
TOPOLOGY:linear
CLONE:HUMGS07389
SEQUENCE DESCRIPTION:
GATCCATGGG GCCCAGGNCT AGAGGGCATC CGAGGGGCTG GGCCTTGGCC ACAGCCTCTT 60
GGATGATGTC CTTGCTCCAC CACGCAGGCC CTTGCTTCTC CCATCAACGA GTCTTGGCCC 120
AGGGGACACT GACCCTCCCN CAGGGAGGAG AGAGGAGTTC CTTNCCTTTA AACGAGGGCT 180

TCCACGACCT CTAAGGGGTG GCGNGGAAGG GAGGAGGATG AGAAGATGNA GATTAAATAA 240
AGGTATGTN 249

SEQ ID NO:6229
SEQUENCE LENGTH:125
SEQUENCE TYPE:nucleic acid
TOPOLOGY:linear
CLONE:HUMGS07390
SEQUENCE DESCRIPTION:
GATCTATCTC AGGGCTTGTT GTAAGAATGA AATGAAGTAT AAAGCCATGT AGAGCTTGTA 60
GAATATGCCT GGCACTTTGT AAGACCTTAA TANNTGTTAA CTATCTAANC ACANAACAAN 120
CCNCN 125

SEQ ID NO:6230
SEQUENCE LENGTH:71
SEQUENCE TYPE:nucleic acid
TOPOLOGY:linear
CLONE:HUMGS07391
SEQUENCE DESCRIPTION:
GATCCTAGTG CTCCTGTCTG GTCAGGGCCT ATCTTTATGT GTTCGTTAAA CTTTTAACAA 60
TGAGNATTAA A 71

SEQ ID NO:6231
SEQUENCE LENGTH:182
SEQUENCE TYPE:nucleic acid
TOPOLOGY:linear
CLONE:HUMGS07392
SEQUENCE DESCRIPTION:
GATCTGAAAA GTTATTAAGT TCAACTCCAC AGAGGTTCCT AGGAGAATGT TAACAGTCCA 60
GACCTTTTTC ACTACATCAA CCACGGATGA TTGGCATATA AAATCCTACT TAACCTTTAC 120
ATAACTGCAA TACCAGGAAA CAAATATGTC AATAAAAGGA AACATCATTC AATTTTTGGA 180
AA 182

SEQ ID NO:6232
SEQUENCE LENGTH:233
SEQUENCE TYPE:nucleic acid
TOPOLOGY:linear
CLONE:HUMGS07393
SEQUENCE DESCRIPTION:
GATCCAAAAA ACCATTCAGG CCAAGTNACC CTGACATGTG ACATCTACCT CCCGACCTAC 60
CACCCCACTG GCTGGTTCCA GAACGTCTCT CACCTAGACC TTGCCTCCCC TCCTCTCCTG 120
CCCAGCTCTG ACCCTGATGN TTAATAAACG CAGCGACGTG AGGGTCCTGC TTATCCCTGG 180
TNNGGNNNCA GNTCNATCCT TGCATCANTG GGGAGGACGT GATGNGTGAG GAN 233

SEQ ID NO:6233

SEQUENCE LENGTH:158
SEQUENCE TYPE:nucleic acid
TOPOLOGY:linear
CLONE:HUMGS07394
SEQUENCE DESCRIPTION:
GATCAGCCTG TGCCACACTT CGCTCTTGAG GAGCTCAACG GTCTGGCAGG GCAGCAGGAG 60
GCTTTCTGGG CTCTGGNCTC CACGGATGCG TGGGCAGNGG AATGTGGGCT ATGTAGTCAT 120
AATAANTTAG GNCACAGAAA AAAAAAANAA AAAANNCN 158

SEQ ID NO:6234
SEQUENCE LENGTH:51
SEQUENCE TYPE:nucleic acid
TOPOLOGY:linear
CLONE:HUMGS07395
SEQUENCE DESCRIPTION:
GATCTCTTTT CATTTGTCAA CCTTTTCAGT AAAGCCCTCT GTTACATCAA A 51

SEQ ID NO:6235
SEQUENCE LENGTH:280
SEQUENCE TYPE:nucleic acid
TOPOLOGY:linear
CLONE:HUMGS07396
SEQUENCE DESCRIPTION:
GATCAGCGAG AAGGACAAGA AGAANNTTCT GGACAAGTGT CAAGAGGTCA TCTCGTGGCT 60
GGACGCCAAC ACCTTGGCCG NGAAGGACGA GTTTACGCAC AAGAGGAAGG AGCTGGAGCA 120
GGTGTGTAAC CCCATCATCA GCGGACTGTA CCAGGGTGCC GNTGGTCCCG GGCCTGGCGG 180
CTTCGGGGCT CAGGGTCCCA AGGGNGGGTC TGGGTCAGNC CCTNCCATTG AAGGAGGTGG 240
NTTAGGGGNC TTTGTTCTTT TNGTATGTTT GTCTTTGNGN 280

SEQ ID NO:6236
SEQUENCE LENGTH:199
SEQUENCE TYPE:nucleic acid
TOPOLOGY:linear
CLONE:HUMGS07397
SEQUENCE DESCRIPTION:
GATCTAAATA CTGTGTTAAA ATATAGAAAA ACGGAGACTC TNATAAGGTT TTTNACCTCT 60
ACACTTGAAA GTGAGCTCTT ACAAGAAACT AATCAGTGTT TCGTTATTCC ATATCAGTGG 120
CTTTTACTGT CAACGNTTGT GTAAGCTNAC TTATCTGGCA AACTTTTNCT TTCCAGAANT 180
ACTATCTATN TTACTNTNN 199

SEQ ID NO:6237
SEQUENCE LENGTH:279
SEQUENCE TYPE:nucleic acid
TOPOLOGY:linear
CLONE:HUMGS07398

SEQUENCE DESCRIPTION:
GATCCACCCA CCTCGGCCTC CCAGAGTGCT GGAATTACAG NCGTGAGCAC CGCGCCTGGC 60
CAAGNAAAAA TTATTAGAAC AAGTGAGGCA TCACACTCCT CTGCCATTCA GTCAAAAGTA 120
NGCACCAATT TATTAACACA AACATTACAT AATACATGAT ACAGGCGCAA TCTCGCTACT 180
GCTAGCACTA CGNNAATGTT GTTCTTGCAC TATAACATTA AACTGTTAAA TACTGACAGT 240
GAAATGCACC NATANNGGNN ACCTGTCATC ACNGGGNNN 279

SEQ ID NO:6238
SEQUENCE LENGTH:21
SEQUENCE TYPE:nucleic acid
TOPOLOGY:linear
CLONE:HUMGS07399
SEQUENCE DESCRIPTION:
GATCCAAAAA CTAACTTCAA A 21

SEQ ID NO:6239
SEQUENCE LENGTH:174
SEQUENCE TYPE:nucleic acid
TOPOLOGY:linear
CLONE:HUMGS07400
SEQUENCE DESCRIPTION:
GATCCAAGTA CTCTCTCTCC TAAAGACACC ACCTTCCTGC CAGCTGTTTG CCCTTAGGCC 60
AGTACACAGA ATTAAAGTGG GGGAGATGGC AGACGCTTTC TGGGACCTGC CCAAGATATG 120
TATTCTCTGA CACTCTTATT TGGTCATAAA ACAATAAATG GTGTCAATTT CAAA 174

SEQ ID NO:6240
SEQUENCE LENGTH:294
SEQUENCE TYPE:nucleic acid
TOPOLOGY:linear
CLONE:HUMGS07401
SEQUENCE DESCRIPTION:
GATCTTGAAG AGACCGCTGG CAGCACCAGT ATTCCCAAGA GGAAGAAGTC TACACCCAAG 60
GAGGAAACAG TTAATGACCC TGAGGAAACA GGCCACAGAN GTGGCTCCAA GAAAAAGAGG 120
AAATTCTCCA AAGAGGAGCC GGTCAGCAGT GGGCCTGAAG CGGCGGTTGG CAAGAGCANC 180
TCCAAGAAGA TGCAAANAGTT CCATAAAGCA TCCCNAGGAN GNTTAAGAAT GCAAATGGAC 240
CATTCTCTTG GNNGGTTNGG NCATACCCAT NAGCCCNAGG TGACATTTCC CNTN 294

SEQ ID NO:6241
SEQUENCE LENGTH:295
SEQUENCE TYPE:nucleic acid
TOPOLOGY:linear
CLONE:HUMGS07402
SEQUENCE DESCRIPTION:
GATCTCGCCC TTACCCCGGG GTCTGGTGTA TGCTGTGCTT TCCTCAGCAG TATGGCTCTG 60
ACATCTCTTA GATGTCCCAA CTTCAGCTGT TGGGAGATGG TGATATTTTC AACCCTACTT 120

CCTAAACATC TGTCTGGGGT TCCTTTAGTC TTGAATGTCT TATGCTCAAT TATTTGGTGT 180
TGAGCCTCTC TTCCACAAGC GCTCCTCCAT GTTTGGNTAG CAGTTGAAGN GGTGTGTGGG 240
TGGGCTNTTG GGNGTGAGGA TGGAGTNTTA AGTGCCNACT NNCNNCTNNN ACCNN 295

SEQ ID NO:6242
SEQUENCE LENGTH:201
SEQUENCE TYPE:nucleic acid
TOPOLOGY:linear
CLONE:HUMGS07403
SEQUENCE DESCRIPTION:
GATCTAATCC CTTCAAGGAA GTGATAACAC TGGGAGTGGT AACAAGAGGA GCAGGGAAGC 60
AAGGGCCAGC CCCTGGTTCT CCATCCCNAT GTGTTNGAGG GTGGGACCAG NTGGACCTGG 120
TCCAGGCCAG GCCTCATTCC TCCTGGNCCN GGCAGGGTCA GAGGGNGGAG GGGCNTGGTC 180
CAGGNATGGG TCCNTTCCAN N 201

SEQ ID NO:6243
SEQUENCE LENGTH:26
SEQUENCE TYPE:nucleic acid
TOPOLOGY:linear
CLONE:HUMGS07404
SEQUENCE DESCRIPTION:
GATCTTGGCA GTTGATAAAA CGTAAA 26

SEQ ID NO:6244
SEQUENCE LENGTH:324
SEQUENCE TYPE:nucleic acid
TOPOLOGY:linear
CLONE:HUMGS07405
SEQUENCE DESCRIPTION:
GATCGNTGCC TGGTTTGTCT TCTCTGTGTC CTGGAGCAAA GCCAGTTCCT AAAACTAAAA 60
CTCCATTCTA GTCTTGGGAA GAAAAGTTTC TACTCAGAAC TGGGGAAGGA GTGGAACTTA 120
TGACTTGGGC CTCTAGGCTG TCTCTGTCCC CTCAGCTCCC CGACATGCAT TTACTCTCTG 180
CCGTGGGTCT GCAGTCGCTG CAACCTACCC TCTCTCTGCC TCAGCCTTAC ACCCAAGCAG 240
TAGGTCTGTG CTCTCCCTGT CTCTAGGTCG NTGAGAGAGG TGCTTTTNTT CATAAAACCT 300
TTGGGGTTTG GGATTTCCCC AGAN 324

SEQ ID NO:6245
SEQUENCE LENGTH:256
SEQUENCE TYPE:nucleic acid
TOPOLOGY:linear
CLONE:HUMGS07406
SEQUENCE DESCRIPTION:
GATCCATTCC GGCGCCTTCC TCTCCCAGTC ACCCAGAGGG CCCCAACCCC GGCGGNCCTT 60
TCTTCCTCAA ATGTCCTCGG CTCTATACCG TGNCTGGGTC TTTTNTCTNT CTCTCTGCCT 120
GGAAGATTCC TTCTTTNCCC TTTTGTCTTG CCCACTACTG TTTACCCTTC AAGTTTCAAG 180

GTCATGTCAC TGTCTNAGAG AGGTTTTCCT GTGNTNGACC TGTTTTTNAT CAGGAAGGCT 240
TGCTNNTTTC CATNAN 256

SEQ ID NO:6246
SEQUENCE LENGTH:37
SEQUENCE TYPE:nucleic acid
TOPOLOGY:linear
CLONE:HUMGS07407
SEQUENCE DESCRIPTION:
GATCATTAAA CCTGGCTTGA GTCTCTGTTC TGGCAAA 37

SEQ ID NO:6247
SEQUENCE LENGTH:152
SEQUENCE TYPE:nucleic acid
TOPOLOGY:linear
CLONE:HUMGS07408
SEQUENCE DESCRIPTION:
GATCAAGNTT GNTCTTCTGT GATATATGGG ATATNATGTT TTAAAGACCA TCTTGGAATA 60
CAATTAGAGA ACTTAGTATT TTGATGTACT AAGACCTATT TTAAGTTTAA TATTCTACTT 120
TGCAAAANCT TTAATTAANG ATGTTATTTA AA 152

SEQ ID NO:6248
SEQUENCE LENGTH:213
SEQUENCE TYPE:nucleic acid
TOPOLOGY:linear
CLONE:HUMGS07409
SEQUENCE DESCRIPTION:
GATCCTGCAT TAAGAAATTC AAAAATCAGT TAATCATCGT TACATGTTGT AAAAGTTGGA 60
TACTGACAGT CTCTAATAGT TTATCATCCA ATNGCANGTA ACCTACAATT TTAGGCATAA 120
ACGTTGATGT GTATAANCAT GNATACAACG CTANTGGCTG GTCACAGTGT TTTCNCAAAC 180
TACAGNTCAC ACCANGTNTT TCTCCTNTTC CTN 213

SEQ ID NO:6249
SEQUENCE LENGTH:274
SEQUENCE TYPE:nucleic acid
TOPOLOGY:linear
CLONE:HUMGS07410
SEQUENCE DESCRIPTION:
GATCTCCTCT TAAGCGGAAA AGAGATTGAC CTTCTAGCAG AGGCAAAGAA TGGAGTCTTC 60
TGGGGGCAGC TGCAAAGCGT TCTCCCTAGG ACAGATGGAG CCTCCCTTTC CTCATCTACT 120
CTGTGGGTGG TTTCAGGGCC CACGAGTCAA CATNAGGAGT TGTGCTGGTG GTATGTGTGT 180
TGGAGGCTGG GCTGGCTGAT TCACAGTGAC GAGGATGTCA CTAATANCAN GTATTGCGAN 240
TGNTGATACC TAATAANAGN CTTTTTTTCC CAAA 274

SEQ ID NO:6250

SEQUENCE LENGTH:216
SEQUENCE TYPE:nucleic acid
TOPOLOGY:linear
CLONE:HUMGS07411
SEQUENCE DESCRIPTION:
GATCACCATG CTTGAATGTC CTCTACTGCT GATTCTAACA TCCGTGTCAG TTTTGGACCG 60
GTTTCAGTAG ATGGATTTTN CTCCNNGNNA TGGGCCATAC TTTCCTGCAT TCTTTGCATG 120
CCTCATAATC TTTGATTGGA TGCCAGACAT TGTAAATTTT ACCTTGTTGG GTATTGGATA 180
TTTTTGTATT CCTATAAATA TNCTTGNGCT TTGAAA 216

SEQ ID NO:6251
SEQUENCE LENGTH:319
SEQUENCE TYPE:nucleic acid
TOPOLOGY:linear
CLONE:HUMGS07412
SEQUENCE DESCRIPTION:
GATCTAATTT TGTATGTGGG AATCTTGCCT TGCCCTGATT TCCAGACCCA TAGGCAGGCT 60
TGCAGGCTGT AGCTGGGGAA GACCCCACGC TTATNCTGGT GCCCTGAGCA AGCCCTCCTG 120
CTCCACTATT AGGCAGAAGC ACCTGTTCTG CCACAGGAGG CGCTGTCTTT TGCTGGCAGA 180
GCTCAGTTTC CCTTAAGGGG AAGTTCCTTA GGATTTAAGG CCTCATGACT ATCTCAAAAC 240
CCATGTTTGT TTGCCAATAA AGNAAATAAA TAAACTGTAA AAAANNAANN AACNACCANN 300
NAAAAAACAC NAACANCAN 319

SEQ ID NO:6252
SEQUENCE LENGTH:111
SEQUENCE TYPE:nucleic acid
TOPOLOGY:linear
CLONE:HUMGS07413
SEQUENCE DESCRIPTION:
GATCTACTGT TTGCATGTAA GAGACCAGGN TATGTAACTC TTATATTTTA AGTGTATACA 60
TATTGTGTAT ATAACATATG GATATTAAAA ATGGGGANTT GCACATTTAA A 111

SEQ ID NO:6253
SEQUENCE LENGTH:108
SEQUENCE TYPE:nucleic acid
TOPOLOGY:linear
CLONE:HUMGS07414
SEQUENCE DESCRIPTION:
GATCCCAGGA ACTGTGGGCA CCCATTTTNT GTGTCTCCCA GCCNATTTCC ACTCCTAGTT 60
TGTCATGGNT ANTTTTTGTT CTTCCCTGTG TGAGGGGNGC CATCAACN 108

SEQ ID NO:6254
SEQUENCE LENGTH:317
SEQUENCE TYPE:nucleic acid
TOPOLOGY:linear

CLONE:HUMGS07415
SEQUENCE DESCRIPTION:
GATCACAACC ATCAGCATCA CGGCCACAGC AGCCAGTGTT GGGGCTGCTG GCATCCCCCA 60
GGCGGGTCTG GTCACCATGG TCATTGTGCT TACGTCGGTC GGCTTGCCCA CGGAAGACAT 120
CACGCTCATC ATTGCCGTGG ACTGGTTCCT TGACCGGCTT CGCACAATGA CCAACGTACT 180
GGGGGACTCA ATTGGNGCGG CCGTCATNGA GCACTTGTCT CAGCGGGNGT GGAAGCTTCA 240
GGAAGCTGAG CTTACCCTAC ACAGNCTGGG GAAACCCNTA CAAGTCCCTT AATGGNACAG 300
GACGCAGGGG AGCATAN 317

SEQ ID NO:6255
SEQUENCE LENGTH:218
SEQUENCE TYPE:nucleic acid
TOPOLOGY:linear
CLONE:HUMGS07416
SEQUENCE DESCRIPTION:
GATCGAGACC ATCCTGGCTA ACACGGTGCT GGCGGAGATA ACACTCCCTT GAAGCAGTGG 60
AGTACAATCA AACATCTTGG CTGCTCCTGA AACCNGGTCC CACGNATTTC AGTCCGGCTA 120
AGANCGAAGA GCTGGTAAAC CANTAANTTG GGTGGAGGCC GAGAGCTCGG GGCCGCGAGC 180
AAGCCTCCGA CGANNTGGTC CCCTGGACCN NCNGGTTN 218

SEQ ID NO:6256
SEQUENCE LENGTH:194
SEQUENCE TYPE:nucleic acid
TOPOLOGY:linear
CLONE:HUMGS07417
SEQUENCE DESCRIPTION:
GATCTNACTC ACAGAGGGAA GAACTTATAA TTCTTCACAG GTCACATAGA AGCATGANAA 60
TTTGGGTTCA AGCAAGTAAA TTCTAAATCA GAATCCATAC ATAAAGTGTT TGCAATGTCC 120
AGTTATATCT CCATGATATT TNCTTTGTGG AAGTTGATTG TCCTTCCTTA CAATAAATTG 180
CTTGANTTGT CAAA 194

SEQ ID NO:6257
SEQUENCE LENGTH:345
SEQUENCE TYPE:nucleic acid
TOPOLOGY:linear
CLONE:HUMGS07418
SEQUENCE DESCRIPTION:
GATCCTGTGT ATTTCCCTGA GTCTTCTAAC ATATGAAAAT TCATATCTAA ATCAACAAGT 60
GACTGTAATC TGGTACTATA AATACTAAAT AAACACTTCT TCATAACACT GTACCAATTC 120
AGCTTTTAAA TTTNATTACT TTGCTTTCCT GTCCTTTGCC AACTCTTAAC CTAGTTAATC 180
CTAGTNCTGT TGACATTGGA CCAGGCTCAG TAAATAANCG AATGGATTTC CAGCCTTTTT 240
TTCCCATCTG TNCCTGCTTT TANGTCCTCT GAATCTGCTT CTTTGCTTNC TGCTGCTTTA 300
ATTTTANCAG TGNTTTTNGT CAAAACATAG GNTNCAGGGN CTNNN 345

SEQ ID NO:6258

SEQUENCE LENGTH:167
SEQUENCE TYPE:nucleic acid
TOPOLOGY:linear
CLONE:HUMGS07419
SEQUENCE DESCRIPTION:

```
GATCTGGGTT CTAGAGGGCA TGTGATGACT GTAAATGTTC ACTGGGTGGG TAGGGAGTGG  60
TATCCAGTGT TCAAGTGCAG AAATCTTTGG CTTNGCTACC AGTTCCATAT GATGAGAAAT 120
AAACGTTCGC TGAGGTTTTG TTTCATAAAA AGANNNNNAA AANNTCN             167
```

SEQ ID NO:6259
SEQUENCE LENGTH:130
SEQUENCE TYPE:nucleic acid
TOPOLOGY:linear
CLONE:HUMGS07420
SEQUENCE DESCRIPTION:

```
GATCCACACT TTTAAAGAAA TTCCATAAAT GTATATTTTG TATTATGTAT TATTTCCTGG  60
TCCNAAGAAA ATATGTGAAT TCAGTTCTAA CTTNNGGANT GTACTGTTTG TTTTCANGTT 120
CATTGNAAAN                                                        130
```

SEQ ID NO:6260
SEQUENCE LENGTH:110
SEQUENCE TYPE:nucleic acid
TOPOLOGY:linear
CLONE:HUMGS07421
SEQUENCE DESCRIPTION:

```
GATCCGAGAT GGCACCATTG TACTCCAGCC TGGGCAACAA GAGCGAAACT CCGTCTCCAG  60
ACAAACAAAC AAANCAANAA CAAGTGTCCC TATNNGNATG GGTGTGTTTN            110
```

SEQ ID NO:6261
SEQUENCE LENGTH:310
SEQUENCE TYPE:nucleic acid
TOPOLOGY:linear
CLONE:HUMGS07422
SEQUENCE DESCRIPTION:

```
GATCTGGCTG TCTAGAGGGA TTCTACGACT GCGTGCTGCC GCCTCCCCAA GAGGCATTCA  60
GGTTATTGGA GAACTAATCT CATCTCAAGG GGCCAGNCAC CAAGTCCCAA AGCCTACAGA 120
CCTCTTTCCG CCAGGCCCTG AAACCTGGCC CCGTGCCAGC AGGATGACAA GCCCCAGGGC 180
GNTCCTGATG AATATGGCTT GGAGATGNTG TACAANTTTT TATTNCCCTN TGGNTTTTGA 240
GGNATGAAAT GATTTGCACT TTTGAAAACC TGTTAACCGT AGGCCNCTTN GGNCANTGAN 300
GNNTGGAAGN                                                        310
```

SEQ ID NO:6262
SEQUENCE LENGTH:129
SEQUENCE TYPE:nucleic acid
TOPOLOGY:linear

CLONE:HUMGS07423
SEQUENCE DESCRIPTION:
GATCCACTCG GNCAACCCAA CAGTTTTACC AACCACCCCG GGCTCGGAAC TAATAGGNAA 60
AGTAGACTCT TTGTGAAGAA ACGAGCCANT NNCTGGAACA CCCTGGTGGA AACCTGTTGA 120
CANGCCCTN 129

SEQ ID NO:6263
SEQUENCE LENGTH:157
SEQUENCE TYPE:nucleic acid
TOPOLOGY:linear
CLONE:HUMGS07424
SEQUENCE DESCRIPTION:
GATCACTTGA ATGTTAGGNT TCATAGNACT ATACTAATCT TCTCACAAAA GGTCTATAAA 60
ATACAGTCGT TGAAAAAAAT TTTGTATCAA ANTGGGGNGG CAAATTNGAA GCTTCTCCTT 120
AANCTGTATT GATACTGACT TGAATTCTTT TCTANAN 157

SEQ ID NO:6264
SEQUENCE LENGTH:171
SEQUENCE TYPE:nucleic acid
TOPOLOGY:linear
CLONE:HUMGS07425
SEQUENCE DESCRIPTION:
GATCTCCTTC TTTAGCTCAA GGTTGCTGCA ANTTGGAGCT GAGCTGTCAG TGGAACGNGT 60
CCTGGAAATN ATTAAGCAAG GCGTCGTTGC GCTGCCCAAN GACAGACTGA AGAAATTTCC 120
AGANTTGAAA TTCAAATATG TGGAAGNGGA GCAGCCCGNG GNGTTTTTAT N 171

SEQ ID NO:6265
SEQUENCE LENGTH:174
SEQUENCE TYPE:nucleic acid
TOPOLOGY:linear
CLONE:HUMGS07426
SEQUENCE DESCRIPTION:
GATCCTACAT TTACAGACAG TTTTGTCATA ACCCTTTGCA TTGCAGCACC TAGTACAATT 60
CCTTGGAAAC AGCGTGGCTC AATAAATTTT TATTGAATGA ATAAATGTGG GCCCAGAAGC 120
GTGCTAGANG AGTGCCTTTC TGGGCTACTN AAAAAANNAA AAANACNAAN AANN 174

SEQ ID NO:6266
SEQUENCE LENGTH:123
SEQUENCE TYPE:nucleic acid
TOPOLOGY:linear
CLONE:HUMGS07427
SEQUENCE DESCRIPTION:
GATCAAAGAC TCCCTGGAGC GAGAAAACAG TTACTGCCAG AAGCAGAATG GAAGAGCCAA 60
AAAGTACACA AAATGGACGC CATAANTTCT GAAATAAAAG TGTATGATGT GTTCTGAGTC 120
AAA 123

SEQ ID NO:6267
SEQUENCE LENGTH:174
SEQUENCE TYPE:nucleic acid
TOPOLOGY:linear
CLONE:HUMGS07429
SEQUENCE DESCRIPTION:
GATCTGGGCC TCAAAATACC ATAGTAGCTG CTTGATAAAA TTCTAAAAAT ATCTGGTTCT 60
CTATTATGTA AACACTATTA CAGTCACCAG TGTGTGAAGA CTCTTGAGTC TGGTTCTCAT 120
ATCAGAGTCA TCATTTTTCT TCCNGTGGAA TAAANTGCCT TGTGGNCTTC CAAA 174

SEQ ID NO:6268
SEQUENCE LENGTH:163
SEQUENCE TYPE:nucleic acid
TOPOLOGY:linear
CLONE:HUMGS07430
SEQUENCE DESCRIPTION:
GATCTTTTCA CTGGCTGACT CTCCCATATC TGCAAGAGAT TCTGCAGGAA CTGGGTGTGC 60
ACACGGTGTT GTAGCCAGTT CAGGTACTGN ATATTTAGGA TTTGGTGNCG TTCACTGTAT 120
TGCTATATTT TTGTAGCTAA ATAAANCTCA ATAANTTGTT AAA 163

SEQ ID NO:6269
SEQUENCE LENGTH:192
SEQUENCE TYPE:nucleic acid
TOPOLOGY:linear
CLONE:HUMGS07431
SEQUENCE DESCRIPTION:
GATCTCCAGT ACTTTGTACA ATGTATAGTA TATTAAATAC ATTTATGTGT AATTTTGTGT 60
GTATTTNTAT ATGGTATAAT TAAAATNATT ACATTTATGT GATTGTAATT CACAAAANGT 120
ACTACTTACT ATATNCTGTA GNGTGTGTTG TGAATGTNTT TACCNGTGTT TGAGCATTAT 180
GGNGTAGGGN TN 192

SEQ ID NO:6270
SEQUENCE LENGTH:85
SEQUENCE TYPE:nucleic acid
TOPOLOGY:linear
CLONE:HUMGS07432
SEQUENCE DESCRIPTION:
GATCTTCTTT TGTAATGTTT TTCATGTTAC TGCCTAGGGC GGTGCTGAGC ACACAGCAAG 60
TTTAATAAAC TTGACTGAAT TCAAA 85

SEQ ID NO:6271
SEQUENCE LENGTH:284
SEQUENCE TYPE:nucleic acid
TOPOLOGY:linear

CLONE:HUMGS07433
SEQUENCE DESCRIPTION:
GATCCTAGTG AGGGATTGAT GAATGTTCTA AAGAAAATTT ATGAAGATGG AGACGATGAT 60
ATGAAGCGAA CCATTAATAA AGCCTGGGTG GAATCAAGAG AGAAGCAAGC CAAAGGAGAC 120
ACGGAATTTT GAGACTTTAA AGTCGTTTTG GGAACTGTGA TGTGATGTGG AAATACTGAT 180
GTTTCCAGTA AGGGAATATT GGTGAGCTGC ATATATAAAT TTGACAGATA GCTATTTACA 240
TAGCCTTCTA AGTAAAGGCA ATGAATTCTC CATTTCCTAC TGGN 284

SEQ ID NO:6272
SEQUENCE LENGTH:211
SEQUENCE TYPE:nucleic acid
TOPOLOGY:linear
CLONE:HUMGS07434
SEQUENCE DESCRIPTION:
GATCAGTATT AACATCCAGG TTTTGATTGT TGTANTATGG TTACCTGGTA GATTACCTTT 60
GTATATAGGN AATAGACACT GGAATAGTAA GGNGTAGTGG GTCAAGATGT CTGCAACTTA 120
TTGTTCAGAA TCACTCACAA NTGGGNAATC TGATATAAGG NCAAANTGGG AGCACTGTGG 180
CCTNTTTTTN CAACTTTTGT GTACATCTGN N 211

SEQ ID NO:6273
SEQUENCE LENGTH:170
SEQUENCE TYPE:nucleic acid
TOPOLOGY:linear
CLONE:HUMGS07435
SEQUENCE DESCRIPTION:
GATCTCATGA AGTCTTCCTT GGCCCAACCA GGCAGATGCC CCGGGCTCCA GTGGGGGGAG 60
GGGTCTGGGG TCTGGTCCGG GTTCCCNAAT TTCTCTTTNN CCCANATCNC TATTTCTACG 120
CAGTGCGNAT AAAATTTGGA NTCGACTAAA ACCCTTGGTG CCCNGAAAAN 170

SEQ ID NO:6274
SEQUENCE LENGTH:301
SEQUENCE TYPE:nucleic acid
TOPOLOGY:linear
CLONE:HUMGS07436
SEQUENCE DESCRIPTION:
GATCTGCCCG CCTCGGCCTC CCAAAGTGCT GGGATTACAG ACGTGTGCCA CTGNGCTCGG 60
CCTCAGATTC CATATTTNAA CACCAGCTGA TTGAGAGAAG GGGAATNAGA AGAGCTGGAT 120
GAGTTTAAAT AACTTTNATT GTTCAGATTC CTGAACAGGN GTTGGGATAA TGGCCATCTT 180
TNCTTTCCTA TCCTTCCCCC CCTCACTGTG AAAAATAACA GTCCACCCCA AGTCATACAC 240
TGGACCCAGT GCCTGCGGGG ACAGGNCTGT GGGTTTCTTG GTCACACCTG TGTTGGTGNT 300
N 301

SEQ ID NO:6275
SEQUENCE LENGTH:198
SEQUENCE TYPE:nucleic acid

TOPOLOGY:linear
CLONE:HUMGS07437
SEQUENCE DESCRIPTION:
GATCCAAAAA ACCATTCNGG CCAAGNAACC CTGAAANGNA ACATCTACCT CCCGACCTAC 60
CACCCCACTG GCTGGTTCCA GAACGTCTCT NACCTAGACC TTGCCTCCCC TCCTCTCCTG 120
CCCAGNTCTG ACCCTGATGC TTAATAAACG NAGCGACGTG AGGGTCCTGA TTCTCCCTGG 180
TTTTNGGGGG GNTCCATN 198

SEQ ID NO:6276
SEQUENCE LENGTH:102
SEQUENCE TYPE:nucleic acid
TOPOLOGY:linear
CLONE:HUMGS07438
SEQUENCE DESCRIPTION:
GATCTCTACT CAAAGATGTC TTGCAGTTTA TGAACTGAGA TGTAACTTCA TGACTAACAT 60
GATGACATCG TTATTAACTA CATAAACTTT ATAAAGCTTA AA 102

SEQ ID NO:6277
SEQUENCE LENGTH:318
SEQUENCE TYPE:nucleic acid
TOPOLOGY:linear
CLONE:HUMGS07439
SEQUENCE DESCRIPTION:
GATCTTAATA CCTAATTTCA TCATTTCTGA AAATGTACTG TTTTAGAATG TATTACAATA 60
TCAATGTGAA TATCTTGAAT CCTGTTACAA ATCCTGCACT GTATTAAACA TGTAAATTAA 120
TTGTTTGTCT GATTAGCCAA TCTCACCACC CAAATGGGGA GGTATACATG TTTGANGAAC 180
TGTGTAACTC AGTAATTGAT TTGTNCTGAT GTTGTAACTC ANTAGANGTG TTTTGGAAGG 240
NAGCATGGTG TGTGAGGCAG TGTCTGTCCT TTTGTGCCAG CTCTGTATGN TGTTTGTNGG 300
CCNTGTTTGT AGGCCTGN 318

SEQ ID NO:6278
SEQUENCE LENGTH:321
SEQUENCE TYPE:nucleic acid
TOPOLOGY:linear
CLONE:HUMGS07440
SEQUENCE DESCRIPTION:
GATCTCGCCA ACAGCTAGCT GCTTAGGAGT ACCCCCACGA TACGCACAGC ACACCACTGT 60
CCCTTCACTG CACTTTCTTC CTGCCTTAGG TAGTTGGGCT TGCCCACCCT AGTTTGCTTT 120
TGTAGTGGTT TGGCAAGGTT AGAAGGCCTC GGCCTCTCTG TNATGCTGGG AAGTGCCTAC 180
TCTCTGGGCC ACTGCTGCAG AGGCCGTGGC ACTTGTNATG GGTTTGGAAG ACCCAGCCAT 240
CTGCAGCAGA GGCAGCCTAT TCCCATTGCA AGGNGNGGAN TGAACGGAGT AATTATTCTA 300
CTNTTCTTTT TACATAAATA N 321

SEQ ID NO:6279
SEQUENCE LENGTH:290

SEQUENCE TYPE:nucleic acid
TOPOLOGY:linear
CLONE:HUMGS07441
SEQUENCE DESCRIPTION:

GATCCTGCAC GTCCGCAAGC CGGAGAAATA CCAGCTGCAC CTCCACGAGA GCTGCGTGCT 60
GTCCCTGAAG TTTGCCTCCT GCGGACGGTG GTTTGTAAGC ACCGGGAAGG ACAACCTGCT 120
CAACGCCTGG AGGACGCCGT ACGGGGCCAG CATTTTCCAG TCCAAGGAGT CGTCCTCAGT 180
CCTGAGTTGT GACATCTNCA GAAATAACAA ATACATTGTN ACAGGCTNGG GGGCCAAGNA 240
GGGCACCGTG TATGNGNTNG TNTNCTTNGG CCATTNNCCC NTTCCTGTNN 290

SEQ ID NO:6280
SEQUENCE LENGTH:130
SEQUENCE TYPE:nucleic acid
TOPOLOGY:linear
CLONE:HUMGS07442
SEQUENCE DESCRIPTION:

GATCCAGTCT NTGGTATCAT GCTAGTNCTG ACAGGGCCTT GATAGTCTAG AGTTGGAAAA 60
GATGGTAAGC TTTTGTNAGN GTTTTAACAT TTNCTTGATG AAACAATAAA AAGAGGTAAG 120
CTTTTNCCTN 130

SEQ ID NO:6281
SEQUENCE LENGTH:192
SEQUENCE TYPE:nucleic acid
TOPOLOGY:linear
CLONE:HUMGS07443
SEQUENCE DESCRIPTION:

GATCCAGTTT TGTTTGGGAC CAAAAACCAG TATTGTACAA AGTATTAAGC ATATATTTTN 60
ATATTTACTA AAATNGACTG TGGTGACTTT GGATAATAAG NAAAANTTTA ATATTAAANC 120
CATGTTTATT ACAGTATAAT TAACATGTTA AACCATGGGG NTAAATGCCA TCANTAAAAA 180
ATTATGNCAT NN 192

SEQ ID NO:6282
SEQUENCE LENGTH:125
SEQUENCE TYPE:nucleic acid
TOPOLOGY:linear
CLONE:HUMGS07444
SEQUENCE DESCRIPTION:

GATCCACCCC CTGCCCGCAA AACATTGCTC CTAACTCCAC CGNCTGTCCC AAAACCTATG 60
NGANCTAATG ATAATCCCAC CACACTTTAC TGACTNTNTT TCCAGACTCA GCCCGGCTGN 120
ACCAN 125

SEQ ID NO:6283
SEQUENCE LENGTH:301
SEQUENCE TYPE:nucleic acid
TOPOLOGY:linear

CLONE:HUMGS07445
SEQUENCE DESCRIPTION:
GATCTGAGTT GCAGCTCTGC ACCCTAAATC ACACCCTGGG CATTGTCTGG GCTGCAGGGC 60
TGCCAGGTTC TGTACTTGTG TCCAGCTATG GCCCTGGNTG CTGGAGCTGG AGGGTTTTCT 120
GTGCTCAGAC TGTAGCCTGT AGCTCTTGGC CTGTGTAGAG CCCCCTCCTG TGCCCTCAGT 180
GGCTGTCGTT TGTTAACATN ATCAGGAAGA TGGGAAAGGT CAGGCAGNAT TTTCTGNCC 240
TACAAAGGGN GGNNGAGAAA GGACACAGTA TTTTNAATGA NTTTACCATA TATCTTTGGN 300
N 301

SEQ ID NO:6284
SEQUENCE LENGTH:108
SEQUENCE TYPE:nucleic acid
TOPOLOGY:linear
CLONE:HUMGS07446
SEQUENCE DESCRIPTION:
GATCCTTGNT TTCTAAAAGC CAGTCCTNCC CTNCAGCTTT CCACAGTTTC TGTAAATGTT 60
TAATACTTNT ACAGTCAATG GCAATTTTAA ATATATATAT ATAANATN 108

SEQ ID NO:6285
SEQUENCE LENGTH:159
SEQUENCE TYPE:nucleic acid
TOPOLOGY:linear
CLONE:HUMGS07447
SEQUENCE DESCRIPTION:
GATCTGGGGG AAAGGCTGCC AGGGCCAAGA GGCTCGTCTC ACTCCCTGCC CTGCCATTTC 60
CCGGAGTGGG AAGACCCTGA GCAAGCAGCA CTGCCTTCCT GAGCCCCNGT TTTCTCATCT 120
GTAAAGTGGG GGTAATAAAC AGTGATATAG GNGTGCAAA 159

SEQ ID NO:6286
SEQUENCE LENGTH:125
SEQUENCE TYPE:nucleic acid
TOPOLOGY:linear
CLONE:HUMGS07448
SEQUENCE DESCRIPTION:
GATCACTTGA CTCTTGTAAA CGGACCTATA CTCAAAAGGC TTTGGCACAC TGTCACGNTA 60
TTAGTATACA AATGTGCATT TGTGTTTAAG TTAAANTGAG GTNTGCNGGG TAAACTGCNA 120
ANTAN 125

SEQ ID NO:6287
SEQUENCE LENGTH:63
SEQUENCE TYPE:nucleic acid
TOPOLOGY:linear
CLONE:HUMGS07449
SEQUENCE DESCRIPTION:
GATCTACAGT AATTTATTCT NTCTAAAAGT AAAGCATTTT CAAAACTCAG TATTTAAACC 60

AAA 63

SEQ ID NO:6288
SEQUENCE LENGTH:104
SEQUENCE TYPE:nucleic acid
TOPOLOGY:linear
CLONE:HUMGS07450
SEQUENCE DESCRIPTION:
GATCTTTTCC ACTGGNCTAC TACCTGACCA TAAACATGTC TGCATTTGAA TTCTCTAAAC 60
CCTAAATCTG TGTCTATGAA AAATACAAAT GACTATTAAA TAAA 104

SEQ ID NO:6289
SEQUENCE LENGTH:123
SEQUENCE TYPE:nucleic acid
TOPOLOGY:linear
CLONE:HUMGS07451
SEQUENCE DESCRIPTION:
GATCTACCCA CCTCGGCCTC CCAATGTGCT AGGNTTACAG GCATGAGCCA TGGCGNCCGG 60
NCAAGTTTCT ATTCTTAAAA TAAAGAGGTT ATACATCTAA NTTACAANTC GTGGCCAAAA 120
NGN 123

SEQ ID NO:6290
SEQUENCE LENGTH:296
SEQUENCE TYPE:nucleic acid
TOPOLOGY:linear
CLONE:HUMGS07452
SEQUENCE DESCRIPTION:
GATCTGCCCG NCTCAACCTC CCAAAGTGCT GGGATTATAG GCGTGAGCAC CGCCCCCAGN 60
TGATTCCAGG CGAATTCTTC TCTGATGGGC GGGCGAGGGT GTGTTCGTGT GATGGGTTGG 120
NGTGTGTGTG TCTNNGTACA CAGATGATGT GTTTTCCCTT CAGCTTCTTA CGTTTTCTGA 180
GCATCCGTTG TGCCTTAACA TTTTCTGNTT GTCCTTTGGG NCAAAGCAGT ATTTTACTNA 240
TNCTTTGAAT GTTCTCATTC TATTNTATCA TGTGACTTAT TAAAANNAGN TTNTAN 296

SEQ ID NO:6291
SEQUENCE LENGTH:228
SEQUENCE TYPE:nucleic acid
TOPOLOGY:linear
CLONE:HUMGS07453
SEQUENCE DESCRIPTION:
GATCTGATGG ATTAATAAGG GGTNTCCCCN ATTGCTTGGC TCTCATTCTC TCTTGTCTGC 60
TGCCATGTAA GATGTGCCTT TTGCCTTCCA CCATGATTGT NAGGCCTCCC AGCCACATGG 120
AACTGTGAGN TCATTAAACC TCTTTTTCTT CATAAATTAC CCAGTCTCGG GTATGTCTTT 180
ATCAGCAGTG TGCAAGNNGG GGNGNTACAC CACTNCTCTA ACTANNTN 228

SEQ ID NO:6292

SEQUENCE LENGTH:176
SEQUENCE TYPE:nucleic acid
TOPOLOGY:linear
CLONE:HUMGS07454
SEQUENCE DESCRIPTION:
GATCCTGTGG TCAGAGGCCT CTTTGGCAGT GTNTTCTTAC CCTTAAGNAA GGTCATGAAA 60
TCCAGAAGGG GCAACCTTTN CAGGAGAGCT TTGGAGTCAT TTCTGTGTGA GNCACTATTG 120
CATAATCCTG NCAGNTTGCT TTTATATTTA AGGANTGATG TTACTTANCA ACTGAN 176

SEQ ID NO:6293
SEQUENCE LENGTH:310
SEQUENCE TYPE:nucleic acid
TOPOLOGY:linear
CLONE:HUMGS07455
SEQUENCE DESCRIPTION:
GATCTGACTG CTGGCTATAG GATTTTGGAC TTAATGACTG AAATTGCAAA TTGTCCTTTT 60
TCTTGGCATT ACAGATTTTG CCAAAATAAC TTTTTGTATC AAATATTGAT GTGTGAAAGT 120
GAAGGAGCTA GTCTGCTGAA CCAGGAATAG TTTGAGATAT TGAACTGTCA TTTTTGNACA 180
TTTGAATACT TTGCAGGCTG GCTTTGTATA AACTTATCCT CNGGNNNCCT ATATGTTGTA 240
AATATNTAGN CCATAATTCC ATTATAANTA AANCCTATAA ACTATTCAAA CNAANANCAA 300
ANAACANANN 310

SEQ ID NO:6294
SEQUENCE LENGTH:258
SEQUENCE TYPE:nucleic acid
TOPOLOGY:linear
CLONE:HUMGS07456
SEQUENCE DESCRIPTION:
GATCCCAAAA AGTTTCCTTC TGCCCTTTTT CAATNCCTCC TTCCCGCCCN TCTGCTTTTG 60
TCATTATATA TTACTTTGCA TTTCCTAGAA TTTCATATAA ATGGAATCAT ANAGTATCTA 120
CTCTTTGTTG TTGTCTGGCT TCTNTCACTC AGCATAATTA TTTTGAAACT TATCTATGCT 180
GNTGTGGGTA TCAGTAGCTC ATTANCCNAT TGTTGCTGAG TAGTNTTTNA TTGTATGANN 240
GTGCTACACG TTATNCGN 258

SEQ ID NO:6295
SEQUENCE LENGTH:31
SEQUENCE TYPE:nucleic acid
TOPOLOGY:linear
CLONE:HUMGS07457
SEQUENCE DESCRIPTION:
GATCTTAATA AATAATACCT TACTGCTTAA A 31

SEQ ID NO:6296
SEQUENCE LENGTH:38
SEQUENCE TYPE:nucleic acid

TOPOLOGY:linear
CLONE:HUMGS07458
SEQUENCE DESCRIPTION:
GATCTATGTA CAATTTTAAT AAAATCCTGT CCATGAAA 38

SEQ ID NO:6297
SEQUENCE LENGTH:73
SEQUENCE TYPE:nucleic acid
TOPOLOGY:linear
CLONE:HUMGS07459
SEQUENCE DESCRIPTION:
GATCACCTAC TAAGANGTAC TTCCTACNTT TCATTCTTTC TTTATAAGAA TTAATTAANT 60
TTTAATTGAC AAA 73

SEQ ID NO:6298
SEQUENCE LENGTH:311
SEQUENCE TYPE:nucleic acid
TOPOLOGY:linear
CLONE:HUMGS07460
SEQUENCE DESCRIPTION:
GATCCACCCA CCTTGGCTTC CCAAAGTGCT GGGATNACAG GCATGAGCCA CCGCACCCGG 60
CCCTATCTCA AGCTTCTTAA CTGCACCTGC TAAATCCCTT TTGCCATGTA AGGTAACGTA 120
CCAACGGTTT CAGAGNTTAG GNTGTGGNCA TTTTGGGGGG CTGTTTTTCT GACTGCAATA 180
CCTGGTNTCC AGTGAATCCA TGTTTAGTGG AGGACAACAT TTGAGGCACT TACATGGATN 240
CTAGGGGACA TNTATGTGAN TGTTAGGCAN AAAGTGCTGC CTNCANGNCA NGCAAATACC 300
NATTNAATAT N 311

SEQ ID NO:6299
SEQUENCE LENGTH:115
SEQUENCE TYPE:nucleic acid
TOPOLOGY:linear
CLONE:HUMGS07461
SEQUENCE DESCRIPTION:
GATCAAAAAA TAACTATAAG TATCCTCAGC CAAGTAGCCC ATGCCGCTGC TCTGCCTACG 60
GNGTACTCAT CTTTATTTCT TTACTTTCTT AATAAACTTG CTTTCAATGT ACAAA 115

SEQ ID NO:6300
SEQUENCE LENGTH:310
SEQUENCE TYPE:nucleic acid
TOPOLOGY:linear
CLONE:HUMGS07462
SEQUENCE DESCRIPTION:
GATCCTACTG CCTCGTCCTC TCAGAGTGCT GGGATTACAG NNGTGAGCCA CCACAGNAAG 60
NCACAAATAG CGATTTTATC TACTACCACC AGAAATCTTC TGACATACTG TTTTACCGCA 120
TGAACTATTG TTTTCTTTAT AAATTCCTTA ACAAATCTCT GTTGTATAGA ATACTGTTGG 180

TTAAAATATA TCAGCAGCTA ATAGCTCATT TTAAATAGAT TCTGAAAGTT CAAGNATTTC 240
AGGTTGCCAG NCACCNNTAT ACTCTACANA ATCAGANANG GCTCATTCCT GNTTNCTNAC 300
ACAACAAANN 310

SEQ ID NO:6301
SEQUENCE LENGTH:209
SEQUENCE TYPE:nucleic acid
TOPOLOGY:linear
CLONE:HUMGS07463
SEQUENCE DESCRIPTION:
GATCAGGGAT GTCCAATCTT TTGGCTTCCC TGAGCCACAT TGGAAGAAGA ATTGTCTTGG 60
GCCGCACATA AAATATGCTA ACACTGACGA TAGCTGATGA GCTTAAAAAA AAAAAANTTG 120
CANGAAAAAT CTCATGTTTT ANGAAGGTTT NCAGNTTTGT GTTGGGCTGC ATTTAANGCT 180
GTNCTGGGCT GCNTTGGNCC CGNGGGCTN 209

SEQ ID NO:6302
SEQUENCE LENGTH:312
SEQUENCE TYPE:nucleic acid
TOPOLOGY:linear
CLONE:HUMGS07464
SEQUENCE DESCRIPTION:
GATCATAGCT CCTGGTAACT GTGGACTTGA TAGATTTNAG GTACCTAGTT CAGAACTCCC 60
TAGTCACCAT CTCCAAGCCT GTCAACATCA CTGCATATTG GAGGAGATGA CAGTGGTAGG 120
ACCCAAGGAA GAGATGTGTG NCTGANTAGT CGTCACCATA TCTCCAAGCT TCCNGGCAAC 180
CAGTGGGAAA AGAAACATGC GAGNCTGTAG GAAGNGGGAA GCTCTTCCTT GGCANCTAGG 240
AGGNNTTAGC CATTCTCTTC CTTATGCAAN GATTGAGGAN TGCAACAATA TAAAGNACGT 300
GNAGTCCCNA NN 312

SEQ ID NO:6303
SEQUENCE LENGTH:147
SEQUENCE TYPE:nucleic acid
TOPOLOGY:linear
CLONE:HUMGS07465
SEQUENCE DESCRIPTION:
GATCCGCCCG CCTCAGCCTC CCAAAGTGTT GGGATTACAG GCGTGAGCAC CGCGCCTGAA 60
NCTGAAGGTT ACTTTTCTTT TCAGTGGTTT GGGGCAGGGT GTTACACAGC TTAATAGATT 120
AAGAAGAATA AAATGCTGAA TGCTAAA 147

SEQ ID NO:6304
SEQUENCE LENGTH:294
SEQUENCE TYPE:nucleic acid
TOPOLOGY:linear
CLONE:HUMGS07466
SEQUENCE DESCRIPTION:
GATCATATTT CCTCAAAAAT AAGAGAAATA AGGTTCATAA GGAAACTTGC TGGGATTGTG 60

GTTGTTTTGT TTTCTCAGCA GCACAAACAA AACCAGAATT TAGCCTTTAG GACTGCTGAG 120
TAAGCCAAAT TTAAATGACT ACTGCTTTGT NCATGGGTAA GCCATGTNCT TTTCAAAATA 180
AGTGCCACTA AAANCCACAT AATGCTTTGG TTTCTATGTG GCTAATANNT ATTTNGTCCT 240
ATAGTTTATC TTATTTGTNA CTGATTTNCN TCTCTTGNAT GCCTCATATT ANTN 294

SEQ ID NO:6305
SEQUENCE LENGTH:294
SEQUENCE TYPE:nucleic acid
TOPOLOGY:linear
CLONE:HUMGS07467
SEQUENCE DESCRIPTION:
GATCCATTCG CCCCAAGGGG ACAACGGGGT GTCAGGGGAG TNAGGGGTGG AGGTAGGGGC 60
CAAAGAGGCT TACACGATAT CCCATACCTT TAATGCCTTT GGCCTTCCAT TCTNACTTCT 120
CTNATGAGAT TATTGTCAAC CCTGCTTTCC CTGGTAGATA TTTGCCAGGC CCAATGCTTT 180
AACCTTAAGC TGATATTTTN CCTTTAGATG TCAATCTCGT TACCAGCAGC CTTTTGACCC 240
AACTNCAGNG NTCTATATTT NCGTAGNGGN TTTTCNCCCA TGTGCATGGA TNTN 294

SEQ ID NO:6306
SEQUENCE LENGTH:129
SEQUENCE TYPE:nucleic acid
TOPOLOGY:linear
CLONE:HUMGS07468
SEQUENCE DESCRIPTION:
GATCCCAGAG GCTGAGGGTT GGGGAGAGGA AGCCATCATC TCATTACCTC TGTCAGTGCA 60
GGGGTGGCTN CTNCTTCCCT TGTGCACTTG GGTCGCTGTG ATTGTAAATA AACGTGATTT 120
CGTACCAAA 129

SEQ ID NO:6307
SEQUENCE LENGTH:193
SEQUENCE TYPE:nucleic acid
TOPOLOGY:linear
CLONE:HUMGS07469
SEQUENCE DESCRIPTION:
GATCTCTTTG TNATTTAAGA AGTGGCTGGA TTGGAACTTT TAATATGCTA ATNTGGAAAA 60
TTAATTACCT TTATGAAGGT GGTTTATTAC AAATAAGCAC ACTAACCCCT CGGAAGTTGT 120
TTTACCTACT TTAAAAGTTT TANTGGATTG CNCCTCTGTA ANCTATTCCT AANNTGTGTA 180
TGANATATTT NAN 193

SEQ ID NO:6308
SEQUENCE LENGTH:291
SEQUENCE TYPE:nucleic acid
TOPOLOGY:linear
CLONE:HUMGS07470
SEQUENCE DESCRIPTION:
GATCTTTCTT CCTCTCCAGC CCACAAGTGG CTCTAGTGAG ACGAGCATTT AAGCTGCAGG 60

TGGTGGCACT CGGGTCTCAG AGCCACCAGA CGTGCAGGTG GGACAGTGGT TCCAGCTCCG 120
GCTGCCTGTN AGCGTCCCTC CCAGGTGCTA GCCCTGGCAG GCACAGCCCT NCCCTCATGC 180
CAGCAAAGGA AATGCTCTTG ATGAGAGGCT CGCTTTAAAG AAGCCCAAAG CGTGTGCTTA 240
TCCAAGGGGT TCAGCTATTC CTGACTTCAC GGCTTGCATG TTTGATTTTG N 291

SEQ ID NO:6309
SEQUENCE LENGTH:97
SEQUENCE TYPE:nucleic acid
TOPOLOGY:linear
CLONE:HUMGS07471
SEQUENCE DESCRIPTION:
GATCATATGT ATTTNTATGT TCTGTAAAGN ATTTGACTTA CTAGTTCTCA ATAAAATTTT 60
ATTAGGACTA TAAAAAAAAA AAAAAANTTT TAATAAA 97

SEQ ID NO:6310
SEQUENCE LENGTH:147
SEQUENCE TYPE:nucleic acid
TOPOLOGY:linear
CLONE:HUMGS07472
SEQUENCE DESCRIPTION:
GATCTTATGT ACATACCCAT TTTAGCTTTC CCATGCATAC TTAACTGCAC TTGCTTTATC 60
TCCTTGGGCA TTCTTACTTA GGATTCAATA GAAACATGTA CAGGGTAAAC AATTTTTTAA 120
AAATAAAACT TCATGGAGTA TCTGAAA 147

SEQ ID NO:6311
SEQUENCE LENGTH:190
SEQUENCE TYPE:nucleic acid
TOPOLOGY:linear
CLONE:HUMGS07473
SEQUENCE DESCRIPTION:
GATCACATTT TGAAATGCCT AAAAGACTTT ATTGTTCTAA TTATCCAGAT GTACCTTTGT 60
AAAATAGCTC TTTTATGAAT TAGCTGATAA GGCTGTATGT TTCTGGANCA AATATTGGGN 120
CACCTAAAAC CTTCCGGTTT CCGGGGGCCN GGGAAATNGG AAATNAGGTT TCNAATNTTA 180
AATNAGGNTN 190

SEQ ID NO:6312
SEQUENCE LENGTH:65
SEQUENCE TYPE:nucleic acid
TOPOLOGY:linear
CLONE:HUMGS07474
SEQUENCE DESCRIPTION:
GATCTGGATT TTTGCCAATT CCTATGAAAA ATAATCCCCA GAAATAAACT ACTAAGGAAA 60
ATAAA 65

SEQ ID NO:6313

SEQUENCE LENGTH:79
SEQUENCE TYPE:nucleic acid
TOPOLOGY:linear
CLONE:HUMGS07475
SEQUENCE DESCRIPTION:
GATCCATTGG TGTTTGGTTT GGGGTGTTTT TTAAGTTTTT TCTTTTATAT CATCCAGAAA 60
TAAAGACACG TACACTAAA 79

SEQ ID NO:6314
SEQUENCE LENGTH:204
SEQUENCE TYPE:nucleic acid
TOPOLOGY:linear
CLONE:HUMGS07476
SEQUENCE DESCRIPTION:
GATCCAATAA CCCAGTTAGG TATTATAGAT TAGTATTTAA GTTTGCTGTA GATTGTGTGT 60
GTGTGTGCTA AGTAAAAGTN CCATGATTCA CAATTTTAGT ATTAACCCAT GTTGCAAAAG 120
CTGTTACTGG CCAACAGATT GTNATGATGT GTACCATATA ACACAAGCNT TANCTNGGCA 180
TTANCGTTTG TNAAGCCANN TNTN 204

SEQ ID NO:6315
SEQUENCE LENGTH:204
SEQUENCE TYPE:nucleic acid
TOPOLOGY:linear
CLONE:HUMGS07477
SEQUENCE DESCRIPTION:
GATCTCATCT CTGAAAATAA AATACCCCGT TAAAATTCTG GCTTTTGTTT GTTAAGGTTA 60
TTGAGAAGGT AATAGAAAAA GATATGATAC TACTTTNCTC TCTTGGATTT GTAAAANTAG 120
TGGAATTTTC ATAAGGATTT TGTATTTTCT ATTGCTGTAT AACATTATTA ATACAAACTT 180
ATTGGCTTAA NANNCAAANN NNNN 204

SEQ ID NO:6316
SEQUENCE LENGTH:218
SEQUENCE TYPE:nucleic acid
TOPOLOGY:linear
CLONE:HUMGS07478
SEQUENCE DESCRIPTION:
GATCACGAAG ATACATGTGT TTGACTGTTT AATTTGAAAG TTTACATTTT TNATGCTTTG 60
TGTTGGTGTG TAATTTTTGT ACTCTTGGTG GCTAGTTTTT GTCAAATCTT TTTTGGAATA 120
TTGCTTAAAT GTNTTGATTT TATGATAGTG AAGCTTGTAT TCAGTGTTTT GCCAATNAAT 180
ATTATATGCT TGTAATAANN GCAAAAGANA AGCTTAAA 218

SEQ ID NO:6317
SEQUENCE LENGTH:335
SEQUENCE TYPE:nucleic acid
TOPOLOGY:linear

CLONE:HUMGS07479
SEQUENCE DESCRIPTION:
GATCCCAGTG GGAACCTTCA TGCCTTATTN ATTTCTAATG GGTAAAGGGG TTTTNTTACC 60
AAGCATCCCT GACCTCCTGG AGACACCACC TGCTTTCCGG GCGGCACTGT GATGGGAGCT 120
GGTGGCGACT GAGTCCTTCT GTACGTGCAA CTGGGAAACT TTTGTCCTTT GAGGCTAGGC 180
AGCTCCCTGC CCTCCGTGTG TGTCTGTTAT CTGGGGGAGA GGAGTGTGGG AAGGNNTTGG 240
GGGAAGAGCT CCAGCCTGTN TTGNTCCCAA AGTTCTNNAG TGGGAAGACC NGGGTNACCT 300
TTTNAGGGTN TANGGTGNGG GGAANTTNTT TTTTN 335

SEQ ID NO:6318
SEQUENCE LENGTH:266
SEQUENCE TYPE:nucleic acid
TOPOLOGY:linear
CLONE:HUMGS07480
SEQUENCE DESCRIPTION:
GATCTGCGTG TCAAACTGTT CCATTTGTTT ATGTAAAGTG ATATTAAAAA AGATATAAAC 60
TATAACTGTC CGTTACTTTT GGCAAAAGAT ACAACCACAT AATGTATATA ATTCCTAGTT 120
TCCATATTTA TCCGCATGTA AAGGGCCGGT TTATCCATGT TACAGCTCTT CAATATTTAT 180
GGCTAGAAGA NCTCGTATGT ACACTTTAGT TTCCAGAACT GTTTGGTAAC CTTTCGTACC 240
TNATTAGAGG ATTCTNAAAT CTCAAA 266

SEQ ID NO:6319
SEQUENCE LENGTH:313
SEQUENCE TYPE:nucleic acid
TOPOLOGY:linear
CLONE:HUMGS07481
SEQUENCE DESCRIPTION:
GATCCACCCA NCTCGGCTTC CCAAAGTNCT GGGATTATAG GCGTGAGGCA CCGTGCCTTG 60
CCGATAAATG GATTTNAAGC TGAGTCTTTG AGGGAAGAGT AGGACCTGCA CTGAAACAAA 120
AAGACAATTT GTGGGGTTGG GGNGAGGAAC CAGATGTAAA CAATGGAAAG NTTTTGGTGT 180
CACTTTGAGN NGGATTTTTT GGAAGGGTTT TGCAATGTCC ATTCTGTATC TTGGGTTANC 240
ACTAGTAAGT CAAATGGNGA ATAATTTGAG TGTCACTCNT TCCCTNCAGT TNGGAAAAAN 300
TTTTTANCNG TTN 313

SEQ ID NO:6320
SEQUENCE LENGTH:144
SEQUENCE TYPE:nucleic acid
TOPOLOGY:linear
CLONE:HUMGS07483
SEQUENCE DESCRIPTION:
GATCTGTGCT TTTNCCCACT TTGCCACACT GTCTAAGGTG GCTTTNAACT GGAACCCAAG 60
TGCAAATAAA GGTTGGTATT CGCTCCTGAA AAAAAAAAAA AAAAAAAAAA AAAAAANAAA 120
AAAAAAAANA AAATNAACNA AANN 144

SEQ ID NO:6321

SEQUENCE LENGTH:332
SEQUENCE TYPE:nucleic acid
TOPOLOGY:linear
CLONE:HUMGS07484
SEQUENCE DESCRIPTION:
GATCCCTCCT CACTTGCCCT GCCACTTTGC ATGGTTTNAT TCTTAACCTG GTCCCCCACG 60
TGTNAAGTGT AGTGGCATCC ATTTCTAATG TATGCATTCA TCCAACAGAG TTATTTATTG 120
GCTGGAGATG GAAAATCACA CCACCTGACA GGCCTTCTGG GCCTCCAAAG CCCATCCTTG 180
GGGTTCCCCC TCCCTGTGTG AAATGTATTA TCACCAGCAG NCACTGCCGG GCCTCCCTCC 240
CGGGGGCACT GCCTGAGGGC GAGTGTGGGN NTAGCATTAG CTGCTTNCTN CNNNCCTGGN 300
ACCNACTGTG GCCTGGAATN GNATAGTGGT NN 332

SEQ ID NO:6322
SEQUENCE LENGTH:376
SEQUENCE TYPE:nucleic acid
TOPOLOGY:linear
CLONE:HUMGS07485
SEQUENCE DESCRIPTION:
GATCTAATTA ATGTGAAGGA CTNGCCAGTC TGGGAAACCG CCTGCCACGT GGAAGAGCCA 60
AACCCGACTC TNTGCTGCCA CATGCCGTTC CCATGCCCGG CTGCTGGGCA CCTGGNAGAG 120
CTTCCAGAAT CCTCGCAGAC AGCCCAGAGC CTGCCGCTAC CCTCGGCCTG CCCACCACCA 180
AGCAAGCAGC AAGCAAGATG GGGTTCTCAT CAGTTCTTCC TTCCCACAAT GTAGGACCTT 240
TCCTTTTACC TTTNCAATGG GATAAAATAG TTCAGAGTTC ATTAGTCATA TTTCATTGGN 300
CACAAGGAAT TCAANGGTTT TTTNANCAAT TTANCANTTC ACCCGTCTTA TGTNGTTTAA 360
NANTNANANC NANTCN 376

SEQ ID NO:6323
SEQUENCE LENGTH:109
SEQUENCE TYPE:nucleic acid
TOPOLOGY:linear
CLONE:HUMGS07486
SEQUENCE DESCRIPTION:
GATCACAGAC ACCCAGTNCG TGGNCCCTGT CTTCACCCCA GCNAGCATCA TGTNAGTGGG 60
CCTCGTTCCC CCCGGAGAAT CCCAGCGGGG CCTCANAGAT GCATCTGGN 109

SEQ ID NO:6324
SEQUENCE LENGTH:156
SEQUENCE TYPE:nucleic acid
TOPOLOGY:linear
CLONE:HUMGS07487
SEQUENCE DESCRIPTION:
GATCTTGGCT GCTTTTAACT AGAAGGTTGC TTTNATNAGC ATATTTATAC TGCTGAAGGA 60
TGAGTGTNAA TTTAAATTAA CTTTGCCGTT TTGTAGAGAA AACTATTCAC AAGATAAATN 120
CCAAGTCTTT NCACCTGTCA GGCATGCATA TNTNAN 156

SEQ ID NO:6325
SEQUENCE LENGTH:229
SEQUENCE TYPE:nucleic acid
TOPOLOGY:linear
CLONE:HUMGS07488
SEQUENCE DESCRIPTION:
GATCCCAGTG AAGGGTTTTG TGTGTTTAGG CCTCATTTCT TTGTCTTTTT CCTACTCCGT 60
TCCTGGCATT TCCTGATTTC NAGTGTATAC TCTGTAGTCT CAGTTCGTGT TTGATTCCAT 120
TCCATGGAAA TAAAAAGTAT GTTGTACATA CTGCCGAAGA ATTGTCTTGC AAGTAAAGGC 180
TTCCCCCTTT ACTATAAGAC TATAANTAAA ACCTTATTTN ATCCTTAAA 229

SEQ ID NO:6326
SEQUENCE LENGTH:190
SEQUENCE TYPE:nucleic acid
TOPOLOGY:linear
CLONE:HUMGS07489
SEQUENCE DESCRIPTION:
GATCTTACAC ATTCTGTGTA TAAAGACCTT AACTCCACAG GACGGACATT TTAGAGTTTA 60
AATTATTAAG GCTATCATTC TTTTAGTAAT GTCATATTTG CAAACTTTTT NAGTTTTGGC 120
CTTTAATTTA AAANGCCTAA TTTTAANGTG CTGCCTGTNA GTAACTCTTG ANTAANANCA 180
ANTTATNACN 190

SEQ ID NO:6327
SEQUENCE LENGTH:355
SEQUENCE TYPE:nucleic acid
TOPOLOGY:linear
CLONE:HUMGS07490
SEQUENCE DESCRIPTION:
GATCTCAGCT CTTTNGGTGA CTGCATATGC AGTGGTGACC CAGCACAGTT TCCATCAATC 60
TCGTCAAAAG ACTTGAGGTT GTTGGGCACA GTATTTCAGA TGACCACAGT TAAAAAGCTG 120
GGTATACACA ATTAACANCC AANGTGATAT GCATTTATAC CTTTCCCTTT ATGNCCAATT 180
TCTTTATAAA TATGGCTCAT CTGCTCATAA CTGTTAGNCC CACCCAACTG TCATTAGTAA 240
CCTGAGTGTT TATGCTTGCA AAACTGTATG TTGTTATTGT CTGATTTGAT NATATTCNGT 300
GNTGTTTAGN GAATGCNGTN GTGTTTNANT GNATCACAAA NAATTNCCCC ATTNN 355

SEQ ID NO:6328
SEQUENCE LENGTH:298
SEQUENCE TYPE:nucleic acid
TOPOLOGY:linear
CLONE:HUMGS07491
SEQUENCE DESCRIPTION:
GATCATTTGA ATCTCAGTCT TCAGCCTGCA CTGATTTGTA GCCTGCACTG TCTTACTGAT 60
TTACAAACTG AAATCACTGA GAAATGTCTT TAGTTCAGTG AGAAGAAACC AGAACACTTG 120
TTCCTAGTGT TGTGTTGTTT TTTTTAAGCA AATTACTTAC TGTATTTTNA TGGCAGGNGG 180
GNGAAAAAGT GTTACAACGG TTTCTAATGA AGTCCGGTAT TTANNTGGTA AATGACTAAT 240

GTGTTTAGTA GTGCCAAANT AAACCATTAN NTGNTTGTCC TTTGCCAAAN ANANNANN 298

SEQ ID NO:6329
SEQUENCE LENGTH:225
SEQUENCE TYPE:nucleic acid
TOPOLOGY:linear
CLONE:HUMGS07492
SEQUENCE DESCRIPTION:
GATCACAAAG AAAAAGCATT TTTAAAAAGT TGGCAAACGC TGAAACGCAC TGTGGTATGA 60
GGCGCATTGC ATTTCCATAG CACTGAAGTA CCAGTTTCCA TTCCTGGGCT GNGATTGTTT 120
TTCCCGTGGT TGTATTGTTC TGATTTAACG ACCACCAGNG TAACTGNTTT TTNTTTGTTT 180
GTTTNCTGGT GGGGTNNCCA CCAATTAAAA CTNNTANAAN CCCGN 225

SEQ ID NO:6330
SEQUENCE LENGTH:229
SEQUENCE TYPE:nucleic acid
TOPOLOGY:linear
CLONE:HUMGS07493
SEQUENCE DESCRIPTION:
GATCGACTTC GATAAAATAC TTTTGCCTAA TCAAAATTAG AGTGTGTTTG TTGTCTGTGT 60
AAAATAGAAT TAATGTATCT NGCTAGTAAG GGCACGTAGA GCATTTAGAG TTGTCTTTCA 120
GCATTCAATC AGGCTGAGCT GAATGTAGTG ATGTTTACAT TGTTTACATT CTTTGTACTG 180
TCTTCCTGCT CAGACTCTAC TGCTTTTAAT AAAANTTNAT TTTTGTAAA 229

SEQ ID NO:6331
SEQUENCE LENGTH:345
SEQUENCE TYPE:nucleic acid
TOPOLOGY:linear
CLONE:HUMGS07494
SEQUENCE DESCRIPTION:
GATCTAAAGA CCCGATAGGT GCAGAACCCA TCTGGACACG GAGACCAGGA ATGGAGTTCC 60
ATGGAGGCCT GGCTGGCACT GCACCCGGGC ATGAGGACAC ATCCAGTAAG AAGACCTGCC 120
TCAAGAGGTG CACTGCGGTG ACCAGTGGAG GTNACTGGTT GGAGCCTGGA ATTGGAAGCA 180
GATTCCAAGC TCTGGTGGAC AAACTCTCCA GGCCTGGTGG GATTCACAGC TGGGGCAGNC 240
CTCATCCTGG CTGCNTGGCC ACAGNCCCNN GCTNTTTNCC ACTTGGTGGT AGGGCCGATG 300
CCTTNTTTGG ANGAGNTGGG NTTNTTTTGG TCNCGACTGN GTANN 345

SEQ ID NO:6332
SEQUENCE LENGTH:219
SEQUENCE TYPE:nucleic acid
TOPOLOGY:linear
CLONE:HUMGS07495
SEQUENCE DESCRIPTION:
GATCACAAAG AAAANGCATT TTTAAAAAGT TGGCAAACGC TGAAACGCAC TGTGGTATGA 60
AGCGCATTGC ATTTCCATAG CACTCNAGTA CCAGTTTCCA TTCCTGGGCT GAGNTTGTTT 120

TTCCCGTGGT TGNATTGTNC TGATTTCACG TACNCCAGAG TAACTGNTTT TTTTTTGTTT 180
GTTTTCTTGT GGAGTTAACA CCANNTAAAA NTTGTAAAN 219

SEQ ID NO:6333
SEQUENCE LENGTH:60
SEQUENCE TYPE:nucleic acid
TOPOLOGY:linear
CLONE:HUMGS07496
SEQUENCE DESCRIPTION:
GATCTATTTC TCAGCTTTCA CTTATGTGAG CCAATAAATT CCTTTTTTGT TGAAGGCAAA 60

SEQ ID NO:6334
SEQUENCE LENGTH:154
SEQUENCE TYPE:nucleic acid
TOPOLOGY:linear
CLONE:HUMGS07497
SEQUENCE DESCRIPTION:
GATCTACCCC CCCATCAACG TGCCCCCTCG ACTCCCGGTG CTGCGGAAGA ACTGAAGGTT 60
GCNATGCCTT ACTCTGACGG GAGCATCTGT ATTTTAATGT TAAAAGCCCA CAAAATAAAA 120
ATAAAAAGTA ACTGAGATGA ATTTAATAAA CAAA 154

SEQ ID NO:6335
SEQUENCE LENGTH:91
SEQUENCE TYPE:nucleic acid
TOPOLOGY:linear
CLONE:HUMGS07498
SEQUENCE DESCRIPTION:
GATCTATGCT GTATGCACAG ATTGCATAAT GGCTTTTTTN CTTAACATTA TGTGATAAGC 60
ATTTNCCTGA ATAANCTTAT TTTAAAATAA A 91

SEQ ID NO:6336
SEQUENCE LENGTH:181
SEQUENCE TYPE:nucleic acid
TOPOLOGY:linear
CLONE:HUMGS07499
SEQUENCE DESCRIPTION:
GATCAGGTGT GGCCAATNAA ATTNAAGAGG AAGTCTACCT GGGNTTCTGG GAAAGCTTTT 60
CCAATAAAAG ACACAGGCAT GGCTAACACC TCCCTGGGCC TCTTCTTCCT ACCTTGATTG 120
AGGGTGTGAT GCCTGGAGCC ACAGCAGCCA CTTTGNNNNC ATGACAAAAA GGCCAAGNGN 180
N 181

SEQ ID NO:6337
SEQUENCE LENGTH:77
SEQUENCE TYPE:nucleic acid
TOPOLOGY:linear

CLONE:HUMGS07500
SEQUENCE DESCRIPTION:
GATCACCAAA ACCTGATGTT TTAAATNTGC TTTCTTTTTG AAATTTATGT TTTCAAATAA 60
AATCTCCCTA AAGCAAA 77

SEQ ID NO:6338
SEQUENCE LENGTH:265
SEQUENCE TYPE:nucleic acid
TOPOLOGY:linear
CLONE:HUMGS07501
SEQUENCE DESCRIPTION:
GATCCTATGA GTGTAGTTAA TGACTGTTNG TAAGTCAGTA GAGTAAAATN CTGTGTCCAC 60
GGGGTGTCAC AGCCTCACCA TACCCTGTTG AGGTGTGAAA TGCCCCGTCA GAAATTAAAT 120
ACAAACTTAA ATGTGCCTAT TGGTGTCTAA NCTTCATACA ATGTAAGGNC AGATTCCTTT 180
TNGGNNTACT GGGTGCTGTC GCCAGGTTTG NTAGTTAGAC TTAAAANCTT GANATTCACT 240
TTTTGGGGGG NGGGNTATAC TGANN 265

SEQ ID NO:6339
SEQUENCE LENGTH:161
SEQUENCE TYPE:nucleic acid
TOPOLOGY:linear
CLONE:HUMGS07502
SEQUENCE DESCRIPTION:
GATCTATTGG AAAACAACAT GGAATGGAAT TCTGGAAATN ATTATTCATT GAAGAATGCA 60
GTGGCCAAGA AAATATCAAA TGTAGATTGT NAACGCTTGA GAATCATGGC TATGGTTTCT 120
NATGTCCTGG TAACANGCTG TTATCTTTNN NGACATTTTN N 161

SEQ ID NO:6340
SEQUENCE LENGTH:272
SEQUENCE TYPE:nucleic acid
TOPOLOGY:linear
CLONE:HUMGS07503
SEQUENCE DESCRIPTION:
GATCTAGTTA TAAAATACAT GAAAAGGCTG ATGCAGCAAT CGGTGGAATC GGTTTGGAAT 60
ATGGCATTTG ANTTTATTCT TGACAATGTC CAGGTGGTTT TANAACAAAC TTATGGAAGC 120
ACATTAAAAG TTACATAANT ATTACCAGAG AGCCTGATGC TCTCTGATAG CTGTGCCATA 180
AGTNCTTGTG AGGTATTTNC AAAGTGCATG ATAGTAATGC TCGGNGTTTT TATAATTNNN 240
NNTTCCTTTT AAAGCAAGTN TTTTGTACAT NN 272

SEQ ID NO:6341
SEQUENCE LENGTH:345
SEQUENCE TYPE:nucleic acid
TOPOLOGY:linear
CLONE:HUMGS07504
SEQUENCE DESCRIPTION:

GATCAGTGTG GAAAAGCCTT CANTCAGAAA GGAAGCTTAA TTNTNCACAT CAGAGTCCAC 60
ACAGGCCTGA AGCCCTATGC CTGTNCCCAG TGCAGGAAGA GTTTCCACAC CAGGGGGAAT 120
TGTATTCTGC ATGGCAAAAT CCACACAGGA GAGACACCCT ATCTNTNCGG CCAGTNTGGA 180
AAAAGCTTCA CCCAGAGAGG GAGTCTGGCT GTGCACCAGC GAAGTCTTCA CAGAGGCTCA 240
CCCTTTGACC ACTTTCCTGA AGAGAAGTTC TCTTTATGAA TTAAGGAGTA CAAAATCCTC 300
TGAGNTTGAN GCAACCCTAT CCAAGTTTCT ANTGGGANTG GTTGN 345

SEQ ID NO:6342
SEQUENCE LENGTH:212
SEQUENCE TYPE:nucleic acid
TOPOLOGY:linear
CLONE:HUMGS07505
SEQUENCE DESCRIPTION:
GATCTGTCTG CCCATCTGGC CCAGGGGGTC CGAGAAGGGA AGCCTTGGGC AAGAGGNGAC 60
CAGTTGCAAT ACTGTACTTC CTGGTCAGTG GCCAGAGGAT GCGTGCAATA GCAGAGGCCA 120
GGTNACCCCT TCAGCCTTGG CCTCTGCCNC TCCATTGGCC CTCCCTNCCT GNTNCTNCNT 180
GGTGTNGGTC AGTCCTTTTC TAAAGNTGTC CN 212

SEQ ID NO:6343
SEQUENCE LENGTH:316
SEQUENCE TYPE:nucleic acid
TOPOLOGY:linear
CLONE:HUMGS07506
SEQUENCE DESCRIPTION:
GATCTTCGTA TAATTGGCCA CTAATGTGAG AGTTCACTAC TAGGCAGAAA CTATTATGGA 60
CAGTNAAATA ATGACTTTAA TCTCACCACG TGAGTTTNAT GCAGTCTTTC CTGTCTAGCC 120
CTTGCCTCTN CCTGCCCATG TNATTGCGGT GCAGTAGTTT CTGTNGTATA ATAGTGTGGA 180
CAGCAGCTCA GAAAAGGAGG GAATGCTACT GATAATTTGT AGATAATNNN NTTTANGACT 240
TAGGGGAACC ATTGACCTTT GANATTTTNA TTAGAAAATN ATTTGTNCAG AATCAGACTC 300
CATTATTTNC CATATN 316

SEQ ID NO:6344
SEQUENCE LENGTH:184
SEQUENCE TYPE:nucleic acid
TOPOLOGY:linear
CLONE:HUMGS07507
SEQUENCE DESCRIPTION:
GATCAAAAGG AATGATGTAT TTTTAACGTG TTGGCCAAAG TCACTGGATA AAATGAGAAT 60
TGTATATTTN TAATTCATTT TGCAAATNCA GAAAGTTGGT CCAGATATAT GTCACAGANC 120
TTTNCACTTG TATACTACTC TTACAATGGA AAANNNTCCC GAAANCTGTA TACTTCTGAT 180
TANN 184

SEQ ID NO:6345
SEQUENCE LENGTH:361
SEQUENCE TYPE:nucleic acid

TOPOLOGY:linear
CLONE:HUMGS07508
SEQUENCE DESCRIPTION:
GATCAGACCA TGCAGGTAAG GTGAACCAGC TGCACGGACC AGGTTCCCGC AAAACATTGC 60
CAGCTAGTGA GGCATAATTT GCTCAAAGTA TAGAAACAGC CCACCTGTGC CCACTTTGAC 120
CATTGGTGAG GATAGATATA AAATCACTTC TTCCAACGAA GCCTAGGTGA AAATCTATTT 180
ATAAATGGAC CACAACTCTG GGGTGTCGTT TTTGTGCTGT GACTTCCTAA TTATTGCTAA 240
AGAACTACTG TTTAGTTGGT AATGGTGTAA AATTACATTC AGCTCCTTCT TGTCATATAA 300
ANAGGAATTT GGNGGGTGTC GGTTTAAANA TTTTATTNCA CCTGTACCAT TTNGTCACTC 360
N 361

SEQ ID NO:6346
SEQUENCE LENGTH:266
SEQUENCE TYPE:nucleic acid
TOPOLOGY:linear
CLONE:HUMGS07509
SEQUENCE DESCRIPTION:
GATCTGTTGT TATACCAATT GAATTAATTG AGTTNNTTGT TGGACATTTG GGTTGGTTNC 60
AAGTCTTTGC TATTGTGAAT AGTCAATGGC AGTATTTTNA ATGTTGGTCT ATTATTGTAC 120
TAAATAGTTG TTTATATTGA GCTTGGGAAA GCTTTCGTGT CCAATAGAAA TAGCATATTA 180
AAATAAATGC CACTGTATAT NAATGANAGT GTTGATTATA TACCCTAGAN ACTTAANCCT 240
TAAGGNAGTT ATGTNTNAAT TNNCTN 266

SEQ ID NO:6347
SEQUENCE LENGTH:355
SEQUENCE TYPE:nucleic acid
TOPOLOGY:linear
CLONE:HUMGS07510
SEQUENCE DESCRIPTION:
GATCAACTCC AGTTTCCAAT GTCTATGTGT CTATGTGTGT ATGTNCCATA CATATGTATT 60
CACATGAAGA CCGGCATGGC CAAGTNCTGC TGGAGGAGCA CTCAAGTGTG ACGAGCAGGG 120
CCACTGGACC CTGCAGGGCT GTGGTGTATA TAGTGCAGCT TTGGAGGTGG AACTCTATTT 180
NAACACTTTT CTATGGAGCC TTCCGAGTCC CAGGTTTTNA CTTGAGGCTG TCTGTCTGGA 240
TGGCGGTTTT NAGACCTCCA TTAACATCCC TACCCAGCAT TCTGTACTTC GGGGGGNCTT 300
CTATCTTGTN ATAAAACTTN TTACCAAGTG GAACATCGGT TACCCACCTT TTGTN 355

SEQ ID NO:6348
SEQUENCE LENGTH:358
SEQUENCE TYPE:nucleic acid
TOPOLOGY:linear
CLONE:HUMGS07511
SEQUENCE DESCRIPTION:
GATCGTCCTG CCTTTGTAAG GCAGATTTGG AGGGGAGGCC CAACAGCAAA GATGGAGAGG 60
AAAAAGGGAG TGGAGCTTGT GGAACAGGAA GCCCCCCAGG GTCTGTCCAG GGCTGCTCAG 120
CTCTTNAGGA TGTTGTGGAT GGTGAAGTGG TGATGGTGTC CTGGAGCAGC CCAGCACCTC 180

CACTGGGCCC AGAAGGTCTT GACTTCGGAC ACTTACCCCT GTTTCACAAG TGTTTATAAA 240
GCTGTTTTTN CTTTNGTTTT TCTCCTAAAT AATCCTTACT TGGTAGTTAT NAATCCTTCT 300
TTGGGGGAGG GACAGGGGCT TTAAANCCTG GAGGAAAATT AATTTTNNAT TAANGGAN 358

SEQ ID NO:6349
SEQUENCE LENGTH:106
SEQUENCE TYPE:nucleic acid
TOPOLOGY:linear
CLONE:HUMGS07512
SEQUENCE DESCRIPTION:
GATCATGGAA GCTCCGTGCC CCTTCTCCCA TTCCTTGCCC TAAGTATCGC CTCCATCTGG 60
TTGTTTGTCT GTATCCTTTA TTAATAAACA AGTAAATNTA AGTAAA 106

SEQ ID NO:6350
SEQUENCE LENGTH:135
SEQUENCE TYPE:nucleic acid
TOPOLOGY:linear
CLONE:HUMGS07513
SEQUENCE DESCRIPTION:
GATCAGTGCC AGAAGTCATT GTATTCAAAG AAGAATAAGC AAGAAAGAAA AGAAGGAAGG 60
AAGAGAGGTA GACAGATACA AGATGAAATC CTGTCAAAAA ATGGAAGGAA ANCCAGAAAN 120
TGAGAGTNNN CCANN 135

SEQ ID NO:6351
SEQUENCE LENGTH:156
SEQUENCE TYPE:nucleic acid
TOPOLOGY:linear
CLONE:HUMGS07514
SEQUENCE DESCRIPTION:
GATCAGGAAC ATGGCAAGGA ACACCACACC ACTGCTTTTN AGCATTGTAC TGGAAGTNCT 60
ATATAATGCA GTGTTAAAAA AAAAAGAAAT AAAATGCACA TAGATTTGAA AGGNTGAAAT 120
ACAACTTTNT NCACAAGTAA CATGATTGTN TTTNTN 156

SEQ ID NO:6352
SEQUENCE LENGTH:134
SEQUENCE TYPE:nucleic acid
TOPOLOGY:linear
CLONE:HUMGS07515
SEQUENCE DESCRIPTION:
GATCAAAAAG AAACTATGAG TAACAAGCTA TAACATAGTT CACCACAATG GGACCCCCCC 60
CCCTTTTCCT NACCCTACAG TTAGTAATAT TNCAATTAAA NTANCTATAT TCTNCTATAT 120
TTTNCCTGTT AAAN 134

SEQ ID NO:6353
SEQUENCE LENGTH:107

SEQUENCE TYPE:nucleic acid
TOPOLOGY:linear
CLONE:HUMGS07516
SEQUENCE DESCRIPTION:
GATCCACATA TCTCTAACAC CATTAATATG AATCATTGGA AATATTTTAC TTATTAACAA 60
AGTCTCTTGT GTTAAGCTAC AGATAAAGCT TTTGTGGTAG TGTCAAA 107

SEQ ID NO:6354
SEQUENCE LENGTH:350
SEQUENCE TYPE:nucleic acid
TOPOLOGY:linear
CLONE:HUMGS07517
SEQUENCE DESCRIPTION:
GATCCAGAGA CATGTGGCAG CAGGCATGGC TTCCCCTTGG CCTCTCTGTA CACTGCCCCA 60
GGACTGTCAT TTTGGCATCT GCAAAGGAAT CACTTTAGAA AGCCAGCACC TGGTTGATGT 120
GTATTCATAC TGACATTAGA TTGATGTGCA CTGCAGGGGN AANNGCGGTA GCTGNCACAG 180
NAAAAGGATG TTTTGATAGG AATAATTTTC TAGTATGTCT TGAAACATGT TCATCTGGAA 240
GTATTTTCCT CCAAAGTAAT GTAGCATGAT TTTNCAAGGA TTGTTAACAT GCCTGGGATT 300
GGGAAAGNTA GGCCTAANGT TGTGCCAAAC TATATCAATA AATTCCCTTN 350

SEQ ID NO:6355
SEQUENCE LENGTH:137
SEQUENCE TYPE:nucleic acid
TOPOLOGY:linear
CLONE:HUMGS07518
SEQUENCE DESCRIPTION:
GATCCCACTT GCATGCACCC AGAATTTTAT ATTCCCCAGG AATATAGCAG AGGAATATTC 60
TGGTATCATA TTTATCTTTC ACCATCTCAA AAATTACAGT TTTAAAAACT CAAATAAAAT 120
TCATCACTAT AACCAAA 137

SEQ ID NO:6356
SEQUENCE LENGTH:135
SEQUENCE TYPE:nucleic acid
TOPOLOGY:linear
CLONE:HUMGS07520
SEQUENCE DESCRIPTION:
GATCCAACCC TTGCTAGGTA TCATAGTTTA GGCCCATTTA CCTTCCCCTG TACTGGCAGT 60
TCAGCCGCTT ACATGCACTC ACCCTGTTTG TGGCTATTTT AAATTCATAT TATTAAAAAA 120
CAAAAAAAAC CCAAA 135

SEQ ID NO:6357
SEQUENCE LENGTH:69
SEQUENCE TYPE:nucleic acid
TOPOLOGY:linear
CLONE:HUMGS07521

SEQUENCE DESCRIPTION:
GATCCTGTCT CAAAAAAAAC ATAACCGACA TCCAGAAGTG AAATAAATTA GCCAGTGTCA 60
CAAAGTAAA 69

SEQ ID NO:6358
SEQUENCE LENGTH:200
SEQUENCE TYPE:nucleic acid
TOPOLOGY:linear
CLONE:HUMGS07522
SEQUENCE DESCRIPTION:
GATCTTAAAA NTACAAAATG TAGACAATTA ATATTCAGGT GCTTACTTTT GAGCCAATAT 60
GTAGCAGAGA AAAACTAATT NNTCTGTGGC AGTATTTTNA TTTTNGGCAT TTCCCAGGAA 120
TTAACTGAAG ACTAAAACTC ATATGTGANG TGTAATGATA CTANGGNAAG AGTCAGTAAA 180
ATTNCCATTT GGCTACCAAA 200

SEQ ID NO:6359
SEQUENCE LENGTH:115
SEQUENCE TYPE:nucleic acid
TOPOLOGY:Iinear
CLONE:HUMGS07523
SEQUENCE DESCRIPTION:
GATCTTGTAA ATATATAAAT NTAAATAANA TTAAANCTGT AATTCCTTTN CCCTCCAAAG 60
GCTTTTNTGT ACATGGCGCT GCATTTGGCT ATTTCCTTTG GAAATAAATA ATGTN 115

SEQ ID NO:6360
SEQUENCE LENGTH:319
SEQUENCE TYPE:nucleic acid
TOPOLOGY:linear
CLONE:HUMGS07524
SEQUENCE DESCRIPTION:
GATCTTCCCA CCATCACAAA TGAATTTAAA GATGAAAAGA AACTCAGTTG CTCATACAAC 60
TGCATTTTTC CTGTCTATTA TGGGAAACAT CAGACGTTCT GAGTAAGATA TATCTCATGG 120
CATTAGTTAA TATAACTNAT ATTGTTTAAN TCATGGTATT ACATGCAATT NATATCAGAT 180
AAAAGCAGAA CACATTTTTG TNCTGCCTCT CTTAAATGCT GAATGTAGCT GTNATGTATA 240
ANTCCATTTA GTTTTATGTN CTAAAGGNCT ATTTGTGCAC TCCAGNTTTN CAGTAAANTA 300
GNATTCCTAG TAAANCNNN 319

SEQ ID NO:6361
SEQUENCE LENGTH:141
SEQUENCE TYPE:nucleic acid
TOPOLOGY:linear
CLONE:HUMGS07525
SEQUENCE DESCRIPTION:
GATCCTAATT TNNTCACGAA TCCCGATGGA ACCCTGTGCT GTTNANACAT CAGTGTGTAA 60
AACTCTCTGT GTCCCTGTTG GGCTGTCCAG ACAGTCTGGA CTCTTGTAAA TTTAANATTT 120

AATTAAAGGA AACAAACCAA A 141

SEQ ID NO:6362
SEQUENCE LENGTH:105
SEQUENCE TYPE:nucleic acid
TOPOLOGY:linear
CLONE:HUMGS07526
SEQUENCE DESCRIPTION:
GATCTTTTGC TTAGACGTTA ACTTGATGCA TCATTGGAAA GNTGTTTCTC TCATCTCTGT 60
CCTAAGGCTT GATAAAGTCA TTAAAATTGT NTTCTTTTGA CTAAA 105

SEQ ID NO:6363
SEQUENCE LENGTH:392
SEQUENCE TYPE:nucleic acid
TOPOLOGY:linear
CLONE:HUMGS07527
SEQUENCE DESCRIPTION:
GATCTCAGGC CAGCCGTTTG TNCTCCTAGC AGGGTTGCTG TGCTGGCCAC ACGGAGAGGC 60
CCTAGAGAGC CTCATGGATT GTAACTAAAG AAGAAACGGT TCCTTTTTGT TTTTTTAAAA 120
ATNATTTTAA AATACCGTTT TTAACACCGT TCTCTCGGTA CTTTTTTTAA GCTAAGTCAG 180
CATTGTCTTC CAGTGTTAAA GGCATCCCTC ACCTCTGCAT TGACCTTACG NATCCATGCC 240
AAGGAATGGG ATTTCCATCC CTGNGGCCAG TCAGTTAGGG TGTCAATTGT NCCTATTTGA 300
NTTTTTAATG NAATCTGATG NGGTGAGNTC AGGTTTANAA NTCTTCAAAN GNNCGGTTNA 360
TTGTACCAGG NNTGNTATGG ATTAATCCNT GN 392

SEQ ID NO:6364
SEQUENCE LENGTH:205
SEQUENCE TYPE:nucleic acid
TOPOLOGY:linear
CLONE:HUMGS07528
SEQUENCE DESCRIPTION:
GATCTCCATT CCTTGGGCTC CGCGTCCGAN TTCATGGTGC GCCGCTGTGC TGGGAGCTGC 60
AGTGGGAATN TNTGGGACAC CTTGACCAAA GGGGAGCTTT GTNTCGTGTG TTTTGAAAAA 120
GGCTTAATGA AGAGAATGTT GTTCATTCTT AGTAGTATAG TTTGCAATTC TTAATGGCAA 180
ATAATAAGTT TCAGTAGAAA ACAAA 205

SEQ ID NO:6365
SEQUENCE LENGTH:343
SEQUENCE TYPE:nucleic acid
TOPOLOGY:linear
CLONE:HUMGS07529
SEQUENCE DESCRIPTION:
GATCCTTTTC TACCCACTTT CCTATGGAGG ATTCCAAGTN ACCACTTCTC TCACCGGCTT 60
CTACCAGGGT CCAGGACTAA GGCGTTTTTN TCCATAGCCT CAACATTTTG GNAATCTTCC 120
CTTAATCACC CTTGCTCCTC CTGGGTGCCT GGAAGATGGA CTGGCAGAGA CCTCTTTGTT 180

GCGTTTTGTG CTTTGATGCC AGGAATGCCG CCTAGTTTAT GTCCCCGGTG GGGCACACAG 240
CGGGGGGCGC CAGGTTTTCC TTGTNCCNCA GCTGCTCTGC CCNTTTNCNN TTATTNCCTG 300
ACTTCAGGNC TTGAAACNNT TCCNGTGGTG TAAATAAAAA ANN 343

SEQ ID NO:6366
SEQUENCE LENGTH:332
SEQUENCE TYPE:nucleic acid
TOPOLOGY:linear
CLONE:HUMGS07530
SEQUENCE DESCRIPTION:
GATCTGTACT TTGAAAACAC ATTGAGTCCC TTTAACCAGG AAGAACTCTT CTGGGCGACC 60
GTCCACATGT TCCTTTCCCT TCCTAATGAC GTGAAGTCTG AACTCTACAG AGCCTCTGTA 120
CCTTCTCTTT AAAGGCAGCT CTGCTCACCA GGAATTGGCT ATAATNCTTT CCAACTCTCT 180
CATCATTTTA ATAGCTATCA GCAAATGTAG AGGTGTAGGG GATTTTGTAA CATCTGATGT 240
ATGTAAAAAG ANATATTCAA GTTGGAAGGC AGNCTAGAAC TGNCCTGNGA GATGTTTGAT 300
GTACACATTA AANNTAAGGA ATTTTACATA AA 332

SEQ ID NO:6367
SEQUENCE LENGTH:258
SEQUENCE TYPE:nucleic acid
TOPOLOGY:linear
CLONE:HUMGS07531
SEQUENCE DESCRIPTION:
GATCAAGAAT GGCAAGAAAA TCACAGCCTT TGTACCCAAT GACGGTTGCT TGANCTTTAT 60
TGAGGAAAAT NATGAAGTNC TGGTTGCTGG ATTTGGTCGC AAAGGTCATG CTGTTGGTGA 120
TATTCCTGGA GTCCGCTTTA AGGTTGTCAA AGTAGCCAAT GTTTCTCTTT TGGGCCTATA 180
CAAAGGCAAG ANGGNAAGGC CNAGNTCACC ATCCCCTAAA TTTTTAAAAG NAGTTTCACA 240
AAGNTNTGCA TATTTNNN 258

SEQ ID NO:6368
SEQUENCE LENGTH:148
SEQUENCE TYPE:nucleic acid
TOPOLOGY:linear
CLONE:HUMGS07532
SEQUENCE DESCRIPTION:
GATCTGTACA TTATTGCATG TGGCAGTAGT GTATTTNCAT TTTGCATACT ATTCCATTTT 60
AAGAATATAT TGCTGTTTAT CCTATTGATG GATATTTGTA TTTNTNATAC TTTTGTGTAA 120
TAATAAAAAT ACTGCTGTAA ATATTAAA 148

SEQ ID NO:6369
SEQUENCE LENGTH:344
SEQUENCE TYPE:nucleic acid
TOPOLOGY:linear
CLONE:HUMGS07533
SEQUENCE DESCRIPTION:

GATCTGATTC AAAGATAACC ATTACCCATC TTTCCAAGGC CCATCTAAGT AGTACAGGTG 60
TTTTGTCTTT TAAAATTGAC AGTCCTCTTT GCATTAAGTC TGTAGGGGGG GAATACAATA 120
TAAATATTCT NCTGTGTAAA CGTAAGAATG TGCTGTAATC TCCAGTTCCT TTGGATACCT 180
CAGAATTCTA TTTATTGTAT ACACTTATAC TGTTTATNCA ATGTTTTGGG TCTTACTCTT 240
TTTGACGTAT TCTCATCAGT CTTCACTCTA GTTTGCAAGC NCTGAAGTTA TTGATTACGT 300
CTAATGTGCA ATTNGGCTTC TATTAANTGC TATTTNATGG TTCN 344

SEQ ID NO:6370
SEQUENCE LENGTH:292
SEQUENCE TYPE:nucleic acid
TOPOLOGY:linear
CLONE:HUMGS07534
SEQUENCE DESCRIPTION:
GATCTNTGTC ACACCCAAGC TGGTGGAGGA GCACCTCAAA AGTGCCCANT ATANGAAACC 60
ACCCATCACA GTGGACTCCG TCTGCCTCAA GTGGGCACCC CCCAAGCACA AGCANGTCAA 120
GCTCTCCAAA GAAGTGAGCA GCCTGGCCCC TGCTGTCGGA CCTGAGCCTC CTGGCTNNGG 180
GNCTGTAAAT ATGTATAGAC CTGTTTTGTC ATTTTTTTAA TAAAGTCAGT TCTGGTGGCC 240
CTGNGACTTT GGNAGGGNAA GGGGGAGGCC ANAAANANTA AANGGGGGGA AN 292

SEQ ID NO:6371
SEQUENCE LENGTH:139
SEQUENCE TYPE:nucleic acid
TOPOLOGY:linear
CLONE:HUMGS07535
SEQUENCE DESCRIPTION:
GATCCACCTG CCTTGGCCTC CCAGAGTGCT GGGATTACAG TCGTGANACC GCACCTGGCC 60
AATTTTAATA TTTTAACTGA AGATGTTTCA TATAATATTC AGCAATATAA AAATTACTAA 120
TGACTTTTTT GGTAAGAAA 139

SEQ ID NO:6372
SEQUENCE LENGTH:105
SEQUENCE TYPE:nucleic acid
TOPOLOGY:linear
CLONE:HUMGS07536
SEQUENCE DESCRIPTION:
GATCCTAACC GNAGGCCCAT CAATTGCAAC ATAGAGATAC ACATGGAAAG AGGGTTTAAC 60
AGACCCTCTC TNGGACACAN CTGTTTCTCC TTTGAGCANT ANTTN 105

SEQ ID NO:6373
SEQUENCE LENGTH:151
SEQUENCE TYPE:nucleic acid
TOPOLOGY:linear
CLONE:HUMGS07537
SEQUENCE DESCRIPTION:
GATCCAGACC AGCGTCTGGC CCAAGCCATT CANAGGTGGG GTCTGGGGNC TGGGGGTTTT 60

GTNTCGTGAC ACTTTGTCTC AAGGAGATTC CACATAGGGA TTTNAATTAG GCTTTCTNCT 120
TGCNAAGGCC TGTGGCATCT AANCCTGGCT N 151

SEQ ID NO:6374
SEQUENCE LENGTH:288
SEQUENCE TYPE:nucleic acid
TOPOLOGY:linear
CLONE:HUMGS07538
SEQUENCE DESCRIPTION:
GATCTNAGGC AGATAAAGAA ACTTCAACTT ATTTNAGAGA GGAGGTAGGG GATGGGGAGG 60
TCACAGAGAA CTCTGGGTTT CTNCAGTTTA CTATGCCACA GCACCATATT TCCGGGTATG 120
AGTTCTGAGC CCCACAATGG CCATAAGCAC TTAACCACAG ACCTAGTGAC GTATGTATAA 180
NATATACACT AGATTCCAAA GNCTTAGTAT ACAAAAGACC ATAAAATATC TTATTTGTAA 240
TTGTTTAANA CTGATTACAT GTTAANNTGN TAATNTTTTN NNTATANN 288

SEQ ID NO:6375
SEQUENCE LENGTH:319
SEQUENCE TYPE:nucleic acid
TOPOLOGY:linear
CLONE:HUMGS07539
SEQUENCE DESCRIPTION:
GATCCTCTAC CCCCGGAATG AGGACACACT TCAGGACCCA GCCCCACTGG AGTNTGGCCA 60
AGNATTCTCC CAGCAGGAAA ATGGCCATTN CATGGACACC AATGANTGCN TCCAGTTCCC 120
ATTCGTNTGC CCTCGAGACA AGCCCGTATG TNTCAACACC TATGGAAGCT ACAGGTGCCG 180
GACCAACAAG AAGTGCAGTC GGGGCTACGA GCCCAACGAG GNTGGCACAG CCTGCNTGGC 240
TCAAGTGGCC TTTTTAGGTG GGTATTCTTC AGCCGTCTCT AGAATCTTTN AGCCTCTTTN 300
TNGGGCCTCA NATCTTTGN 319

SEQ ID NO:6376
SEQUENCE LENGTH:166
SEQUENCE TYPE:nucleic acid
TOPOLOGY:linear
CLONE:HUMGS07540
SEQUENCE DESCRIPTION:
GATCTCTGTA TGNTCTGTGT GTTTCTGTGT CCTGGAATTG GATGCGTGGG ACTCGTTCTG 60
TCCGCGGAGT GCACTCTTTT TTNCAGTGTG GCCCACATAT CTTGTAAATN TTTGCTGAAG 120
AGTTGTGTCT ATATATAGAG AAAATATATA TAANCAGAGA AATAAA 166

SEQ ID NO:6377
SEQUENCE LENGTH:158
SEQUENCE TYPE:nucleic acid
TOPOLOGY:linear
CLONE:HUMGS07541
SEQUENCE DESCRIPTION:
GATCCAATGC CCTAAACTTT ACATAAGAGT TCCGGCAAGG CTGTGACTTC AACTTGTGTT 60

GTAACTGCAA CCTGCAGAAT CAAATTTAGG GTTATGTTTT CAGTTGTATC AGTCTTACGG 120
CTACAAGATA TTAAAGAGAT TCTTTCCTGG TTATCAAA 158

SEQ ID NO:6378
SEQUENCE LENGTH:323
SEQUENCE TYPE:nucleic acid
TOPOLOGY:linear
CLONE:HUMGS07543
SEQUENCE DESCRIPTION:
GATCTCACCT TGTTGTGCAT CATAGAATTC ACACTGGACT AAAACCTTTT AAGTGTAAGG 60
ATTGTGGAAA ATGTTTTAGT CGAAGCTCTC ACCTTTATTC ACATCAAAGA ACCCACACTG 120
GAGAGAAACC ATATGAGTGT CATGATTGTG GAAAATCTTT CAGCCAGAGT TCTGCCCTTA 180
TTGTGCATCA GAGGATACAC ACTGGNGAGA AACCATATGA NTGCTGTCAG TGTGGGAAAG 240
CCTTCATCCG GNAGGATTGN CCTCATTANG CACCNGNGAT TTCATGTTNG NGNAGAGGCC 300
TATANTTGTA ATCAATGGTN ANN 323

SEQ ID NO:6379
SEQUENCE LENGTH:318
SEQUENCE TYPE:nucleic acid
TOPOLOGY:linear
CLONE:HUMGS07544
SEQUENCE DESCRIPTION:
GATCCTTCTA ATCTCCTGGA CCCTCCAGGG CACTCTGGTC CCTATTCCCC AGCTCCTAGG 60
CAGCTGAGCC GGGTCCCTTA GGGGAGGTGA CCAGGAGCTT TGGTGCAGGG AGCTCTTNGT 120
GGGGCAAAGG GCTGGACCCC TGCCAGGTCT GTGGACATGG TTATATGCCC GGNNGAGGGG 180
GGTACAGGGC CCCAGGGATG GCCCCCAATC CCACCTCTGT TTATTCTGTA AACTGCAACC 240
TATAAATAAC CTTTAGCATT CCTATTTGTA ACAAAATTNG NNTTTNTGNA AATAAATTAT 300
ATTTCCTAGT CTAAAAAA 318

SEQ ID NO:6380
SEQUENCE LENGTH:77
SEQUENCE TYPE:nucleic acid
TOPOLOGY:linear
CLONE:HUMGS07545
SEQUENCE DESCRIPTION:
GATCTTCTCT GCCTTTATTG TTTTGCCATT TCACTAAAAT ATGTTTAGAG ATATATAAAT 60
NCTGCTTGGA CTCTAAA 77

SEQ ID NO:6381
SEQUENCE LENGTH:32
SEQUENCE TYPE:nucleic acid
TOPOLOGY:linear
CLONE:HUMGS07546
SEQUENCE DESCRIPTION:
GATCTCATTT TACAAGAGAA TCTCTCTGCA AA 32

SEQ ID NO:6382
SEQUENCE LENGTH:83
SEQUENCE TYPE:nucleic acid
TOPOLOGY:linear
CLONE:HUMGS07547
SEQUENCE DESCRIPTION:
GATCTCTAAG TGTAGCTGTA AATTTTGGGG TTAATTTGGC TTATATTGGN CCTTTTAAAA 60
GNAATAAAGT TTTTTAATGC AAA 83

SEQ ID NO:6383
SEQUENCE LENGTH:74
SEQUENCE TYPE:nucleic acid
TOPOLOGY:linear
CLONE:HUMGS07548
SEQUENCE DESCRIPTION:
GATCTATAAA GAAACATAAG CTTAAAGTTG TTTATCACTG TGGTGTTAAT AAAACAGTAT 60
TTTCAAAAAA TAAA 74

SEQ ID NO:6384
SEQUENCE LENGTH:342
SEQUENCE TYPE:nucleic acid
TOPOLOGY:linear
CLONE:HUMGS07549
SEQUENCE DESCRIPTION:
GATCCAGACC TCTCTAATAT GAAGAATAAT GAGCCTTATN ACTATAAGTT TGTNAAATGG 60
ATGACTAAAC ATAAGAAACT ATCAGCCATC CCCGTTTCAG CATTCTGTAA CTCAGAGACT 120
AAATCACAGT TTGAGAAGTT TGTNCGTTTT TCCTTCATTA AAAAAGACAG CACACTGGAT 180
GCTGCTGAAG AAATCATCAA GGCATGGNGT GTACAGAAGT CTTGATTTGT GCAGAATGGN 240
TTAATGTTTC TGTTAGATGA CCTAGTATGG NATTGTNACT TAGTGCTGCC ACCTGCTGGG 300
ATGTTAANNG ANTGTNGGAT GCGTGGCCTG GCGGTGNNNG CN 342

SEQ ID NO:6385
SEQUENCE LENGTH:118
SEQUENCE TYPE:nucleic acid
TOPOLOGY:linear
CLONE:HUMGS07550
SEQUENCE DESCRIPTION:
GATCCTGTGT TTTGAGTCCC AAAGGCCATG CCAAGGATTG GCTTTGGNTG GCTTTAATCA 60
CCAACCCATC AACATCAAGC CTCCNCCAGG CCGGTTCAAA TAAATGTANT TAAATAAA 118

SEQ ID NO:6386
SEQUENCE LENGTH:277
SEQUENCE TYPE:nucleic acid
TOPOLOGY:linear

CLONE:HUMGS07551
SEQUENCE DESCRIPTION:
GATCCAACAA ATTAACCATA TAAGCACAGA AAATAGAGAA ACACAGTTAT TGAATCTACT 60
CTTGTCATTA ACATTTTCAA AAAACAAAAT GCATATTGTA ATATTTGGTA CATGACACTT 120
GCATGTTGNT ATGCCTATAT NCTTGCAAAG NATTCAATGT GTACTTAGCG GCGNTTAAAN 180
CTATGTCATG TACAACTNTT ATAAGCNTTT TTNCNGGGGT CCCATTTGCC CTTCATCTTT 240
CAGTAAAGTC TTTGTCAGGN AANNGTTGTN TGGTTAN 277

SEQ ID NO:6387
SEQUENCE LENGTH:102
SEQUENCE TYPE:nucleic acid
TOPOLOGY:linear
CLONE:HUMGS07552
SEQUENCE DESCRIPTION:
GATCCAGGGA TGGGAATGGG AGTGGGTAGG AGGGGTAATG CGGTCATTGG AGTCGGGGGC 60
TGGAACATTA TGAGTGCTCA ATAAATATAA ACTAATGAGA AA 102

SEQ ID NO:6388
SEQUENCE LENGTH:338
SEQUENCE TYPE:nucleic acid
TOPOLOGY:linear
CLONE:HUMGS07553
SEQUENCE DESCRIPTION:
GATCCAGAGG TGGGTGCAGC TGAAAGTAAA CAGAATGGAT TGCCAGTNAC ATGTATGCCT 60
GCGCAGTTCC CTTTTNATTT GCAGAAGCTG TGAGTTTTGT NCACAATTAG GTTCCTAGGA 120
GCAAAAAACC TCAAGGATTG ATTTATTGTT TTCAACTCCA AGGCACACTG TTAATAAACG 180
AGCAGGGTGT TTTCTCTCTT CCTNTCTAAT ATATGGAGTT TCGAAGAATA AAATATGTGN 240
GCAATATTTA AATTCTCAGG AATTGNCTNA TACTCTTGNG GAATGGANTT CAGTTTTCAN 300
TCANGNTTTA CATTTNTGGT GGCTTAANAA NCNTTGGN 338

SEQ ID NO:6389
SEQUENCE LENGTH:245
SEQUENCE TYPE:nucleic acid
TOPOLOGY:linear
CLONE:HUMGS07554
SEQUENCE DESCRIPTION:
GATCAGTCTT TTGAAATTGG GATTCTAAAT TAGAATGAGA GGAGGAGCTG CTAAAGCATA 60
AAAATGGTAG GGGTGATTTT GGTNTTTTAT CCTAGTGACC AATGTTGACC ATTAATTGTA 120
CATTATAAAT AATGTTTTCN TCTGGGACTT TTTGCACTTA ANGCTGGTAA TTNTNATCAT 180
AGGGCTTCAA ATACAAAAGA NTTGTATGCG GTNTTCAGAA AATAAACATG CTCTTTGCAT 240
ATAAA 245

SEQ ID NO:6390
SEQUENCE LENGTH:345
SEQUENCE TYPE:nucleic acid

TOPOLOGY:linear
CLONE:HUMGS07555
SEQUENCE DESCRIPTION:
GATCTTNACG CGNCTGGGGG CGAAGATTTC GCCATGGATG AGGATGGGGA CGAGAGCATT 60
CACAAACTGA AAGAAAAAGC GAAGAAACGG AAGGTTCGCG GCTTTGGNTC CGAAGAGGGG 120
TCCCGAGCGC GGATGNTGAG GNTTATNACA GCNTGGAGCA GGATGGCGAT GAACCCGGAC 180
CACAACGCTC TTTTGAAGGC TGGATTCTNT TTGTAACTGG AGTCCATGAG GAAGCCACCG 240
AAGAAGNCAT ACACGTCAAA TTCGCAGANT ATGGGGAAAT TAAAAACATT CATCTCAACC 300
TNGNCAGGCG TTCCAGGNTA TCTGGAAGGG GGTNTNCTCT NGTCN 345

SEQ ID NO:6391
SEQUENCE LENGTH:343
SEQUENCE TYPE:nucleic acid
TOPOLOGY:linear
CLONE:HUMGS07556
SEQUENCE DESCRIPTION:
GATCCCCTGC TGGTCTCTGG CAGTCTCCTT GATTTTGGGT ACCATGTATA TTTCCCGCTT 60
TNACTTTAAC GCTTTCTAGG ATAGGGTAAG CACCCTTAAT TCAGGCACCG TCCATNAGCT 120
TCCTTTGCAA AGGCTACTTA TGGCCGGTCA CAATCCAGCA CTCAGACAGA GCCAAGGCAA 180
TATCCTCTTG CCCATGGCTA TNATGTCAGA CAGTGGATGG GCTCCAGCAA CAAGAGTCAA 240
AATAACTAAA GGCCTTTGCT CTCCTCTGAC ATTGAGGCCT GGGGCTTACA GTTTGGAATA 300
CAACATGTGA NGGTTTTTGN TNGTTGTTTG TATTATTGNA GAN 343

SEQ ID NO:6392
SEQUENCE LENGTH:185
SEQUENCE TYPE:nucleic acid
TOPOLOGY:linear
CLONE:HUMGS07557
SEQUENCE DESCRIPTION:
GATCAAAAAT TGTGGCCATC TTTGCAAATN ACTACCTNTA GCCTGTNAAA ATACATTTCA 60
AAAAATGTTA TGTGCAATGA ACACTAAATT NAAGAGCAGT TACAGTGTGA CTCACTCATG 120
TTTAAAAAAN ATCGAAGAGC TAAAAAATAC GTCTAATTTA TGTAACCCNT TGGNATGTAT 180
TNNTN 185

SEQ ID NO:6393
SEQUENCE LENGTH:68
SEQUENCE TYPE:nucleic acid
TOPOLOGY:linear
CLONE:HUMGS07558
SEQUENCE DESCRIPTION:
GATCATTTCT TGTAATGTTT TGAGAGTAAT GCATACAGAA ATATAATAAA NTGTGTTGAA 60
ACTGCAAA 68

SEQ ID NO:6394
SEQUENCE LENGTH:163

SEQUENCE TYPE:nucleic acid
TOPOLOGY:linear
CLONE:HUMGS07559
SEQUENCE DESCRIPTION:
GATCCCCATT GTGTATGANC TGAACAAGGA GCTGAAGCCC ACCAAGCCCA TGNAGTTCCT 60
GGGTGATGAG GAAACGGTGC GGAAGGCCAT GGAGGCTGTG GCTGCCCAGG GCAAGGCCAA 120
GTNAGGGGTG GGCTTGGGCA ATAAAGGCAC CTCCCCCAAC AAA 163

SEQ ID NO:6395
SEQUENCE LENGTH:275
SEQUENCE TYPE:nucleic acid
TOPOLOGY:linear
CLONE:HUMGS07560
SEQUENCE DESCRIPTION:
GATCAACTCC CACTCATCCT GCAGATTTAA GCTTGGATGT CACCTCCCGA GGGACGCTGT 60
GCCTGCCTCC CTCGTCAGAG CAGGTGTTGT CATCTACTCA CAGAATCTGG CTTCCTTCCA 120
CAAAGCACCC CCGTGAGCAT CTGTTTAACA TTGTCTCTCC ACCTAGACTG TAAGGTCCGT 180
GAAAGCAGGG ACCACACCCA TTTGTGCTCA CTTGGGACCT ACAGCCTCTA ATCCAGAGCC 240
TGACACAAAC CTCAATAAAT ATTTAAGGAC TGAAA 275

SEQ ID NO:6396
SEQUENCE LENGTH:131
SEQUENCE TYPE:nucleic acid
TOPOLOGY:linear
CLONE:HUMGS07561
SEQUENCE DESCRIPTION:
GATCCGGGAC CTTCANACCC AGGGAAAGGG TGAGGGAGAC TGGGGCCTGG TCTGCTTTCC 60
CGGGCCTGAA AGCTTCCCCG AGGTTTGCAG GGTCAGGNAG GAGGAACGGT GGGGGNNGGC 120
AGTCACTGNN N 131

SEQ ID NO:6397
SEQUENCE LENGTH:359
SEQUENCE TYPE:nucleic acid
TOPOLOGY:linear
CLONE:HUMGS07562
SEQUENCE DESCRIPTION:
GATCCATTTA AAACAAGCTG ATAGTGTTTC GTTAAGCAGT ACATCTTGTG CATGCAAAAA 60
TGAATTCACC CCTCCCACCT CTTTCTTCAA TTAATGGAAA ACTGTTAAGG GAAGCTGATA 120
CAGAGAGACA ACTTGCTCCT TTCCATCAGC TTTATAATAA NCTGTTTAAC GTGAGGTTTC 180
AGTAGCTCCT TGGTTTTGCC TCTTTAAATT ATGACGTGCA CAAACCTTCT TTTCANTGCA 240
ATGCATCTGG NAGTTTTGAT ACTTGTAACT TTTTTTTTTT TTNGGGGNNN ANTNTTTNNG 300
GGNCCAGGGG TTTTTTTTNG GAACCCCTTG GGCNNTGGCC CNGGGGTACC CGGCNAGNN 359

SEQ ID NO:6398
SEQUENCE LENGTH:61

SEQUENCE TYPE:nucleic acid
TOPOLOGY:linear
CLONE:HUMGS07563
SEQUENCE DESCRIPTION:
GATCTCTGAC GATACCTGTA TGTTCTTATT GTGTAAATAA AATTNCTGGT ATGAAATNAA 60
A 61

SEQ ID NO:6399
SEQUENCE LENGTH:367
SEQUENCE TYPE:nucleic acid
TOPOLOGY:linear
CLONE:HUMGS07564
SEQUENCE DESCRIPTION:
GATCCTGTGG GAAAGAGAAA GGCATCCCAG AAGTTTGACT TGTTTTNCTT TTAGAATCTG 60
TCTCTGCAGG AAAGAAAAGT CATTTTAATA AAAACCAAAA TAATTTATAA TAATTGTNGC 120
CTTTCCCTCC CCATCCTCTC CACTAAGCCA GCAACTAAAT GTATTCAGAA AACAAGTTAA 180
TCAAGGAGGG CAGAAGAATT TTTTTTAAAC TGTAAGAAAN TGTTCCTAAG TTTAGATTTA 240
TTACCTGTCA TTTAGGGCAA AANTCCAGTG TCCCCAGCAC ATGGTGACCC AACTNTGTCT 300
CACAGTTGCA AAGTGTGTTG GAAAATAAAG TTGCTTTGTG NACTTNGNGG GNAANNCNNT 360
NANNNNN 367

SEQ ID NO:6400
SEQUENCE LENGTH:379
SEQUENCE TYPE:nucleic acid
TOPOLOGY:linear
CLONE:HUMGS07565
SEQUENCE DESCRIPTION:
GATCAGGAAC CAAAAAGAAG AATGTACTTC ATCTGGTTGG GCTGGATTCC CTCTNATAAG 60
CCTTCCCAGT TGACTGAAAG ATGAGGCTAG GCTCTAGCAA GTTGAAGTCA AACCAGCTCC 120
TTCAAGAAGC TTTGAGCAGA ATGAAGTGGG GAGGACCCAG CTTCCAGCCC AGGAAGCCCA 180
CTGTACCTGG AGCCATCTGG GATAAGACTT TGACCCATGA CTCCCATATC CACAGCCTGT 240
CCATCCTAGC CCATCCCAGT TTATCCTGTA TCATTTGAGC TGGGNTTCCA CATCCTCTGA 300
GTTGGTAGTC CCATNTCAAG TCNTTNAATA AANGNCTNTT TGGANTATTT GANTTCACAT 360
TAAAAAAANA ANAAAGGCN 379

SEQ ID NO:6401
SEQUENCE LENGTH:225
SEQUENCE TYPE:nucleic acid
TOPOLOGY:linear
CLONE:HUMGS07567
SEQUENCE DESCRIPTION:
GATCGGGGTA TGAAGTGTGC ACACGCAGCC CAACAACGGG CAGTGGTCTC TGTGCTCCTA 60
GGCATCCAGC ACAGGTTCTG GCAGGGCACC CCTGCTGGGG TTGGGGGCTG GTCTGTGCAT 120
AATCCTGGAC TGTGATGGGA ACAGCCCAGT GCAGTCTAAA CTTCAATTGT GTTGAAACTA 180
CTTTAATAGA CAAAGTAATA AATCATGTTT ATCTATTGAT TTAAA 225

SEQ ID NO:6402
SEQUENCE LENGTH:60
SEQUENCE TYPE:nucleic acid
TOPOLOGY:linear
CLONE:HUMGS07568
SEQUENCE DESCRIPTION:
GATCTTCAGT TGTATTTTNG TGAATATTTT AATACATCTT TTTCAATTTC TGAAAACAAA 60

SEQ ID NO:6403
SEQUENCE LENGTH:176
SEQUENCE TYPE:nucleic acid
TOPOLOGY:linear
CLONE:HUMGS07569
SEQUENCE DESCRIPTION:
GATCCGGTGC TACGGGAAAC ATTTTCCTAA GATGCCCATG AGAACAGACC AAGATGTGTA 60
CAGCACTATG AGCATTAAAA AACCTTCCAG AATCAATAAT CCGTGGCAAC ATATCTCTGT 120
AAAANCAAAC ACTGTAACTN CTAAATAAAT GTTTAGTCTT CCCTGTAACC TTCAAA 176

SEQ ID NO:6404
SEQUENCE LENGTH:360
SEQUENCE TYPE:nucleic acid
TOPOLOGY:linear
CLONE:HUMGS07570
SEQUENCE DESCRIPTION:
GATCTTCCCT GCGGCCTCTG CCCTGGCCTG CTTCCCAGCA CACACTTCTT TGGCCTAAGG 60
GCTTCTCTCT CAGGACCTCT AATTTGACCA CAACCAACCT GGGCTTCAGC CACATCAGTG 120
GGCACTGGAG CTGGGGTGCA CATGGGGCCT GCTCACCTTG CCCACACATC TCCAGCCAGC 180
CAGGGCCCTG CCCAGCTTCA ATTTACAGAC CTGACTCTCC TCACCTTCCC CCCTGCTGTC 240
CAGAGCTGAA CATAGACTTG CACTTGGATG TCACCTGGAG TGTNACATGG GAGTGTTATG 300
GCAGCATCAT ACCAAGGCCT ACTGTTGCAC ATGGGGCCAA AACCAGTAAA CAGCCACCTN 360

SEQ ID NO:6405
SEQUENCE LENGTH:32
SEQUENCE TYPE:nucleic acid
TOPOLOGY:linear
CLONE:HUMGS07571
SEQUENCE DESCRIPTION:
GATCTNACTA ATAAATGAAG GGAAAAAGGA AA 32

SEQ ID NO:6406
SEQUENCE LENGTH:219
SEQUENCE TYPE:nucleic acid
TOPOLOGY:linear
CLONE:HUMGS07572

SEQUENCE DESCRIPTION:
GATCTGGTCA TTTAAAAGTG TGTGGCACCT CCCCACCACC TCTNTCTCTC GCTCGCTTGC 60
TCGCTCCTGC TTTNACCATG TNATGTGCCT GCTCCCCCTT TGCCTTCCGC CATGATTGTA 120
NGCTTCCTGA GGCTTCCCTA GAAGCAGATG TCAGCATTAT GCTTCCTNTA CAGTCTGCAG 180
AACTGTGAGC CAATNAAACT TCTTTCCTTA TAANTTAAA 219

SEQ ID NO:6407
SEQUENCE LENGTH:347
SEQUENCE TYPE:nucleic acid
TOPOLOGY:linear
CLONE:HUMGS07573
SEQUENCE DESCRIPTION:
GATCCAGTTA CATGTATTAT NNTTTTCCTA CCTTATGGAC TATTTTGGAG GGATAAGCTA 60
TTAAGACTAA GACTATGNGT GANAGTNGGG GAAGGAGCAG GAAGGGAGGA ACCTGCACAC 120
CACATTGGAA CCNGCACACC ACATTAACAC AAAGGCAATC TTCTGGCTCG GACTGTTCTT 180
TACTACTGTN CTTAAAGAAA ATGTTCATTC TGCTGCAGCT ANCTAGCCTC CATCTNCTAC 240
ACCAAATACT ATTCCATGCC ATGGAAGTGC TATGCAATAA CTNTCCCAGG TAGGCACCTT 300
ATACCCGCTT AAANAGNCTT TAAANATNTG CANTTTTGGT NGGNGTN 347

SEQ ID NO:6408
SEQUENCE LENGTH:208
SEQUENCE TYPE:nucleic acid
TOPOLOGY:linear
CLONE:HUMGS07574
SEQUENCE DESCRIPTION:
GATCACTCCT TATTTGCTGT TATTTTNGTN CTCTTAACCA TACATTTCCC AATGTTTAAA 60
GGGTGGTATT TNGAGAAAGT NAATGGGTTT TGTAAGTGCC AGCNCTTTAT ATAAGCAGGT 120
AGACTAGCTT TTNCCCAGCA GACGCTTGTC TGTNTAGACT TCCTTAAAAN AATTATCTTC 180
AAATAAAGCA AAATCTCCAT AAAANGNN 208

SEQ ID NO:6409
SEQUENCE LENGTH:318
SEQUENCE TYPE:nucleic acid
TOPOLOGY:linear
CLONE:HUMGS07575
SEQUENCE DESCRIPTION:
GATCCATAGA CTTAGCAAGT CTTGCCTTAT CTATGGAGCT CGATGGTGAG AATTGTNACC 60
ATTGTCTGAT GTCCATAGTT CCTTCCCCCT AGATTGTTTC TTTCCACGGA TTGTGTTCAT 120
CTGAACCATT NNATTTTTAA TTTACCAAAG TACTGTACTT GGCTATTTGC AGTGTTTTCA 180
AAACCAAATG TTTCTNTTTT GGTGTTTTTA ATCTCCGATA CTTGGTGCAA TAGAAGCTGC 240
AAAGATGTGC CACTTNATCT ATGAAATGGA GTNTTGTATA CCAATAAATN CTAGTTTAAA 300
AACANTTNAN NANNNANN 318

SEQ ID NO:6410
SEQUENCE LENGTH:233

SEQUENCE TYPE:nucleic acid
TOPOLOGY:linear
CLONE:HUMGS07576
SEQUENCE DESCRIPTION:
GATCTGTTAA GGGGTGGATA ACATAATATG CAGCTTAGGA TGCTATTTTA AGATGTATGA 60
TATTCAGTTC ATTCACCTGA TTACTTTGGT TGCAGCACAA CTGTATATAT NGTATAACCG 120
AAATTGATTA TTTNCATTGT CCTTAATGCA GTGATTTATA ATTNGAGCAT GTTTAATAAG 180
TTTNCTCTTC TNGTTAACTA GTCATTTGAC TGGNAANNNA TAANATNCTT TTN 233

SEQ ID NO:6411
SEQUENCE LENGTH:105
SEQUENCE TYPE:nucleic acid
TOPOLOGY:linear
CLONE:HUMGS07577
SEQUENCE DESCRIPTION:
GATCCCACGA GGACCACTNT GTAATTGNCA GGGGGCTTCC AGNNAAGACA CTGAACCCTG 60
AAGCCGTGGT CTGGGGCAGG GTGGTGGGAG AGGTGGGCGG GGGNN 105

SEQ ID NO:6412
SEQUENCE LENGTH:101
SEQUENCE TYPE:nucleic acid
TOPOLOGY:linear
CLONE:HUMGS07578
SEQUENCE DESCRIPTION:
GATCACCTTC CCACTTCATC CATCCCCACC CTTCCAGCTC CCCATCCATC CTGCTGAAAG 60
CATTTCCACC ACTCAATAAA ACCTCGCATT CATCCTTCAA A 101

SEQ ID NO:6413
SEQUENCE LENGTH:332
SEQUENCE TYPE:nucleic acid
TOPOLOGY:linear
CLONE:HUMGS07579
SEQUENCE DESCRIPTION:
GATCAGAAGG AATCCGNCGA GGCAGGGATG GGTGTGCCCA TGTNTGCCTT GACGGGACTT 60
CATCTTATAG ACTGTTAAAC TGTCACACAC AAACAGGCTT TCCACCCCTG CTCTGNGNGC 120
ACCACGCACA GNTTTCCAGT TCTTAGTGTG GCTGTTTAAA GTAGAAAATC TGGGGGCTGG 180
GTGAGGCCAC TCATGCCTGT AACCCAGGGC TTTAGAAGGC TGAGGCTGGG GGGNTTGTTT 240
GAAGGTCAGG AGGTTCAAGG CCCAACCTTG GGAACCATAG GCAACNACCC CCCATNNTNT 300
NCCAAANTNG AAAAANCCAA AAAGGNNAAC CN 332

SEQ ID NO:6414
SEQUENCE LENGTH:129
SEQUENCE TYPE:nucleic acid
TOPOLOGY:linear
CLONE:HUMGS07580

SEQUENCE DESCRIPTION:
GATCTACCAG CATAAATATC TNCTNATTTG TCCCTATGCA TATCANTTGA GCTTCATATA 60
CCAGCAATAT ATCTNAAGAG CTATTATATA AAACCCCCAA ACTNTTGATT ATTAGCCAGG 120
TAATGTGNN 129

SEQ ID NO:6415
SEQUENCE LENGTH:257
SEQUENCE TYPE:nucleic acid
TOPOLOGY:linear
CLONE:HUMGS07581
SEQUENCE DESCRIPTION:
GATCTAGAGG AATTAGTCAT GACATCAAGA AAGGAGTCTA AAGAAGAGAA AGAAAATCAA 60
GATGAAAGAG AAGTAAAAGA AGANGAACAA GAAGAAGAAG AAGAAGTCAG GTCAGCAGAA 120
ANTTCCTCAA ANTCTCCAAA GAAAGGTCAC TGATTCCAAG GCANCCTGTG GNAAGANGAN 180
TTCAGATGTC AGTGGGAGCC TCCAGGTGGG NGGNGAGCAT GCCTAANCCC TGGTGGCCAT 240
TGTNCTGTAC TANGGAN 257

SEQ ID NO:6416
SEQUENCE LENGTH:138
SEQUENCE TYPE:nucleic acid
TOPOLOGY:linear
CLONE:HUMGS07582
SEQUENCE DESCRIPTION:
GATCCACAAG GCTTCTGTAT ACAGTTCACG GTCCTCGGCA TTGCTTCTNG TAATTTTNCC 60
ATCCCAAAGG AGAGCTACTG TACTGACTGT ACTTGTGGAA TNCAGCGCTT CATTAAATTA 120
AGTTTATTAA AATGCAAA 138

SEQ ID NO:6417
SEQUENCE LENGTH:331
SEQUENCE TYPE:nucleic acid
TOPOLOGY:linear
CLONE:HUMGS07583
SEQUENCE DESCRIPTION:
GATCTAATGG GACACGGCCA GTGCCTAGGG GTGCCAAGTC CAAGGCCTCC CACTGGGNGT 60
AATCGCTGAG AAGATGCCAA TNTTTCATCC ACCGGCTGCA CAGGCACAAA CTCCCCCACC 120
CAGGACGGCT GTNATGAGGT GGCCCTCCCT NTNAACCCTG GTCCCTGGAG TCCCCAGCAC 180
CTGGGGCCCT GGTGGGGCTG ATGTCACAGG TGTTTACTGT GCTGCTGCAC TGGTCCTATG 240
NCAGCTTCAC CCCATGTGGG GACCACGGAA GGNACAATNC CTTACCCCCG NTTGCCGGGN 300
CCGTGCGGTN NNCAAGANGG GNCAAGGANG N 331

SEQ ID NO:6418
SEQUENCE LENGTH:337
SEQUENCE TYPE:nucleic acid
TOPOLOGY:linear
CLONE:HUMGS07584

SEQUENCE DESCRIPTION:
GATCCAGCTT ATCCTTTAAT TTCAAAGTCC ATTCTTGGGG CTGGTGGGGA GGCAGGAGAA 60
TACCCCTCCC TAAGCCCTTA GTGTGTGCCG AGCTTGCTTT GTNATGTTGG CAGGGGAGGG 120
GAGACCTGGG TGGTGACTGA GTTCCCTTTA TCAAACCCTT CAATGGGCAC AAAATTNAGT 180
GCTTGATTTT ANGGTTTNAT TTTTTNATGA ATGTCCAAAA TCTGTGTTTC CCCNNTGCCC 240
TCCCAAGANN TGTGTGGGCC AGTTGAAAGT GTCTTGGTTT TGTTGGTCAT CTTTTCCCTN 300
AATTTCTGGA GCANGGNCCC TGAGNNCCNT GCCANAN 337

SEQ ID NO:6419
SEQUENCE LENGTH:350
SEQUENCE TYPE:nucleic acid
TOPOLOGY:linear
CLONE:HUMGS07585
SEQUENCE DESCRIPTION:
GATCATGGAT GTAGCATACA CCAAAATCCA CCGAGACCTT TGCAAAATNA GGCTACTTGT 60
TTNATTTTGC CAGATTATCA TGGGAGAGTC TTTAACTAGT NCTGAATATT TGTAAGTCTG 120
AGTACTTCAT AGAATTATAA AGAAGGTACA GCTGACCCCT TCTCACCATG TNGTAAATAT 180
TTCCTGAGAC TGGGTGCAGT ACTGAGGGAT AATAATCTCC TGNTTTATCA AATCCTGACT 240
GTCTAGAGCA ANTTGTACAC TCTCTTTGTG AATGGTCCTG TAACGTGTAT GNNCACATTT 300
NTGGGTGCCT TAGANGTNGT NTTTTNCATG TGTGCTAATN TGCAGTTTCN 350

SEQ ID NO:6420
SEQUENCE LENGTH:362
SEQUENCE TYPE:nucleic acid
TOPOLOGY:linear
CLONE:HUMGS07586
SEQUENCE DESCRIPTION:
GATCCAGGGT CTNAACCGCC GTGGGACGAG GAGGGCATCC CGGCCACAGC AGGGGCCGAG 60
GAAGAGGAGG AGACAGAGGG GAAAGGGGAG CCGGCCTGAC CCACACCCCC GGCCATCGCT 120
CCCTGGGCCA GGTTTAGAGC AGGGAGTTTG GCTGGTGCTG GGCCTGAGCC AGGGGGCCGG 180
GACACCCTTG TTTCCGGTGG TCTTCCCGTT GTGGGAGCAG GTNGAGGGTG GAGACCTAAA 240
CTTTGGGGTC CAGCTGCCTT CAGCCCNNCT CCNCCAGACT AACAGACCCT TGGAGGCAGG 300
GGCTGTGGAA ATAAATCTTT GCCTGNTGGG NTAAAAAAAA ANTATNANNT GTGNNNNGTT 360
GN 362

SEQ ID NO:6421
SEQUENCE LENGTH:114
SEQUENCE TYPE:nucleic acid
TOPOLOGY:linear
CLONE:HUMGS07587
SEQUENCE DESCRIPTION:
GATCTNCTCT CCCACCCACA CTTATCTCCC CCAGGGCCAC TCCAAAGTCT ATACACAGGG 60
GTGGTCTCTT CAATAAAGAA GTGTTGATTA GACCTGAATT TCTCCACCTA TAAA 114

SEQ ID NO:6422

SEQUENCE LENGTH:62
SEQUENCE TYPE:nucleic acid
TOPOLOGY:linear
CLONE:HUMGS07588
SEQUENCE DESCRIPTION:
GATCTTTGCT ACTGGAGTCC TTTAACAACA CCTATAACGA TAAAAAATTC CTAATTNTTA 60
AA 62

SEQ ID NO:6423
SEQUENCE LENGTH:96
SEQUENCE TYPE:nucleic acid
TOPOLOGY:linear
CLONE:HUMGS07589
SEQUENCE DESCRIPTION:
GATCCTTCAT ATTTAATTAA AAGTGTTCAT TCAGGTAAGG TGTATGTTGA AATTTTNCCA 60
ATNATCTTAA TAAAACCTGG CAATTTAAAA NCCAAA 96

SEQ ID NO:6424
SEQUENCE LENGTH:88
SEQUENCE TYPE:nucleic acid
TOPOLOGY:linear
CLONE:HUMGS07590
SEQUENCE DESCRIPTION:
GATCTATTTA TNTAAGTATG TAGTAAACAA TATCTNAAAG TGTCCGATTC ACTACTTGTA 60
AATTAAAAAA GTNATGATTA ATGTGAAA 88

SEQ ID NO:6425
SEQUENCE LENGTH:314
SEQUENCE TYPE:nucleic acid
TOPOLOGY:linear
CLONE:HUMGS07591
SEQUENCE DESCRIPTION:
GATCAGAAGA AGCTACAGTT TTNTTTTAAA CTTGAAAAAA AATTAAGAAG TATTAAGGAA 60
AAAGCTTAAT ACTCTGGAAA CCAGACATCT GGTCTTTCTC ATGCGTAGGG AGCAGTGTAA 120
ATGAAAGTGA AGTCAAACGG ACAGTTTTTT NCTGCATTAT GCACAAGGTG TTTTAAATTG 180
TCAGCTTAAG GTTTAGATGA NGATAATCTT TAACTTTGTT TACAATTTTG NCTATTTNGC 240
ATTATCCCCC TTTTNCCATA AAANNTAANG TGAATATGTG TGCATATNNN TNTNGAGTCA 300
NAATTAAACT TATN 314

SEQ ID NO:6426
SEQUENCE LENGTH:91
SEQUENCE TYPE:nucleic acid
TOPOLOGY:linear
CLONE:HUMGS07592
SEQUENCE DESCRIPTION:

GATCCTGTGG TGTTGTATAA AAATGAACAG CTAAAAATTT ACCCATGACA AGATTAAAGC 60
AAAAATAAAC ACAAAGTTGT TAGACTTTAA A 91

SEQ ID NO:6427
SEQUENCE LENGTH:122
SEQUENCE TYPE:nucleic acid
TOPOLOGY:linear
CLONE:HUMGS07593
SEQUENCE DESCRIPTION:
GATCTTGTGC TATAACTTTT AAAGCCATAC AAATAAGAGT GCTAAACTNT GGACTTAAAA 60
GTAGGTGTNT AAATATTTTT AATCAGTATT ACTTGGAAAA TAAAATATAA CAAACCCATA 120
AA 122

SEQ ID NO:6428
SEQUENCE LENGTH:293
SEQUENCE TYPE:nucleic acid
TOPOLOGY:linear
CLONE:HUMGS07594
SEQUENCE DESCRIPTION:
GATCCTAACC CCTGCAGGGC TCAGGGGTCA GCAGGGACCC ACTGCCCCAT CTCCCTCTCC 60
CCACCAAGAC AGCCCCAGAA GGAGCAGCCA GCTGGNATGG GAACCCAAGG CTGTCCACAT 120
CTGGCTTTTG TGGGACTCAG AAAGGGAAGC AGAACTGAGG GCTGGGATAT TCCTCATGGT 180
GGCAGCGCTC ATAGCGAAAG CCTACTGTAA TATGCACCCA TCTNATCCAC GTAGTAAAGT 240
GAACTTAAAA ATTCAATCAA ATGAACAATT AAATAAACAC CTGTGTGTTT AAA 293

SEQ ID NO:6429
SEQUENCE LENGTH:209
SEQUENCE TYPE:nucleic acid
TOPOLOGY:linear
CLONE:HUMGS07595
SEQUENCE DESCRIPTION:
GATCTGAATT TGTAAGTTAT TAATGTGAAA GGGGGAGCAG GAAGCTCCCC GTTTGCACAA 60
AGGGGGATTT GTTTGCACAA AGTTCACTGT AAATACTGTG AGANTNGANT TCTGAAAAGG 120
TCATATTTAG TGATTTCAAA AGCACACATT TAANTGANGA CTGACTAAAT AANAGGTACC 180
ACACACTTCT NGGTTGGTTT CATTCCAAA 209

SEQ ID NO:6430
SEQUENCE LENGTH:200
SEQUENCE TYPE:nucleic acid
TOPOLOGY:linear
CLONE:HUMGS07596
SEQUENCE DESCRIPTION:
GATCTCCCTC AGTNTGCCCC CAGCCCCCAA ACTCCTCCTG GCTAGACTGT AGGAAGGGAC 60
TTTTGTTTGT TTGTTTGTTT CAGGAAAAAA GAAAGGGAGA GAGAGGAAAA TAGAGGGTTG 120
TCCACTCCTC ACATTCCACG ACCCAGGCCT GCACCCCACC CNCAACTCCC AGCCCCGGAA 180

TAAAACCATT TTCCTGCAAA 200

SEQ ID NO:6431
SEQUENCE LENGTH:131
SEQUENCE TYPE:nucleic acid
TOPOLOGY:linear
CLONE:HUMGS07597
SEQUENCE DESCRIPTION:
GATCTNATTC CAGGATTGCT CAGAAATTGG AAAAAAACTC ACCTGTGCTT CCCAAATTTA 60
TTTCCCAAGA CAAAGATGTC TATAGCACAG AAAAAAATGC ATGANTTAAA AAATCAAGAT 120
GTATTATTAA A 131

SEQ ID NO:6432
SEQUENCE LENGTH:42
SEQUENCE TYPE:nucleic acid
TOPOLOGY:linear
CLONE:HUMGS07598
SEQUENCE DESCRIPTION:
GATCCTGAGC TGTCCCATTA AACATGGCCC TGTCTCGGTA AA 42

SEQ ID NO:6433
SEQUENCE LENGTH:332
SEQUENCE TYPE:nucleic acid
TOPOLOGY:linear
CLONE:HUMGS07599
SEQUENCE DESCRIPTION:
GATCCACTAG ACAGTTTNCA GTTTGCTTGG AGGTANCTGG GTAATCAAAA ATGTTTAGTC 60
ATTGATTCAA TGTGAACGAT TACGGTCTTT ATGACCAAGA GTCTGAAAAT CTTTTTGTNA 120
TGCTGTTTAG TATTCGTTTG ATATTGTNAC TTTTCACCTG TTGAGCCCAA ATNCAGGATT 180
GGTTCAGTGG CAGCAATGAA GTTGCCATTT AAATTTGTTC ATAGCCTACA TCACCAAGGT 240
CTCTGTGTCA AACCTGTGGC CACTCTATAT GCACTTNGGT TTACTCCTTT ATTNCAAATA 300
ANTATACTAA AAGNCTTTAA ANNANGNNNA NN 332

SEQ ID NO:6434
SEQUENCE LENGTH:224
SEQUENCE TYPE:nucleic acid
TOPOLOGY:linear
CLONE:HUMGS07600
SEQUENCE DESCRIPTION:
GATCAATACG GGTCTATTTT NATGTCAACT GAACACTGTA GGGTACCTTC CAGTCTTTTT 60
CAAGATTGTN AAATTGAGAC AAGTAATTGA ATAATTTGTC CTATTTTAAT TTTAAAAAAA 120
GTGAATGGNC TGAAATGTTA AATGTGAATG TNCATTTCTN AATTGCAACT TTNCTACTGA 180
GTGTTTGCAC TATACTTNCT GGAATCTNAT TTAACAAANN TANN 224

SEQ ID NO:6435

SEQUENCE LENGTH:188
SEQUENCE TYPE:nucleic acid
TOPOLOGY:linear
CLONE:HUMGS07601
SEQUENCE DESCRIPTION:
GATCAAAAAG CACAAAGAGC TGGGGCAGAG GCAGGAAGCA GGGGCCCTCC TGGCAGCTCC 60
TCTNAGTGGG GAGAGGTTGG GCAGTGAGTG AGGGACCCCT AATGCAGGGN CTAGAAGCCT 120
CANTTTCCCC NTTTTACCCT TCCACACAAT AGCCTCTGTA GGNNAGGCTG CCCCATNCCA 180
CCNGGNTN 188

SEQ ID NO:6436
SEQUENCE LENGTH:256
SEQUENCE TYPE:nucleic acid
TOPOLOGY:linear
CLONE:HUMGS07602
SEQUENCE DESCRIPTION:
GATCTAGGAC TAANACTTAA AATGTGGTTG GAATCTCACT CAAGGCCTAC CCACCTAAAA 60
CTGCGTTCTA GCAATTGCAT GCTGACTGCA CCTATGAGTC GTAAGNCTGT AGTTGCAAAG 120
TGTGTCTAGT TTNANATTCT TTCTAAATAC TGGTACGGGN AACAGGCATT CCAGNACTTG 180
TGGCTTGCTG GNGGTGGTNT GAGTNATGNG GAATCTTGGG AAGGGGTTCC TGCCCCTGGG 240
ANTTGGGGGG TTTTNN 256

SEQ ID NO:6437
SEQUENCE LENGTH:240
SEQUENCE TYPE:nucleic acid
TOPOLOGY:linear
CLONE:HUMGS07603
SEQUENCE DESCRIPTION:
GATCCAGGGC CTTTNAAACA TCCCCAAAGT CATGGCCATA CTCACCATTA GCCAGTTTCT 60
AACATCTGTT TCAGGGTATC CAGCTGTAGA TGTTCTTATC CCCCATACTT GTGAGTTCTT 120
GGGGTTGCTC ACAAATACTA GGGGTTTTTG TTGTATTTTT AACAANTATA TCCTAATGTC 180
ATATTNATNC TCTTTTGTAA CTGCTGGTCT TTACANTAAA GGNGTCATCT GCCTTTCAAA 240

SEQ ID NO:6438
SEQUENCE LENGTH:125
SEQUENCE TYPE:nucleic acid
TOPOLOGY:linear
CLONE:HUMGS07604
SEQUENCE DESCRIPTION:
GATCAGCACC CTTCTCCACA TCCACATGAC TGGTTTTTAA TGTAGCACTG TGGTATACAT 60
GCAAACATCC GTTCAAAATC TGAGTCGGAG CTAAAAATAA AAAATGAAAA ANCAGAAATA 120
AGAAA 125

SEQ ID NO:6439
SEQUENCE LENGTH:321

SEQUENCE TYPE:nucleic acid
TOPOLOGY:linear
CLONE:HUMGS07605
SEQUENCE DESCRIPTION:
GATCCGCCTG CNTCGGCCTN CCAGAGTGCT GGGATAAAAG GTGTGAGCCA CTGTGCCTGG 60
CCACTCCCTT GAGACCCAGT TTCCACGTCT GTNAAATGGG GGAATACCCA CAGTGAGAAT 120
TAGATGAGAT AGATGCATTT NAAACCACAA ATCGGGGTGC CCACCTGTGG TGGGTAGTTA 180
GGGTAGCTGT TACAAAGTGC CCCAGCAGAG ACCCCCTTGA CTAACTTTNA TTCTTCCATC 240
TTTTCCCACA GGTGGCAGCT GCCAACAAGA AGCATTAGAA CAAACCATGC TGGGTTAATA 300
AATTGCCTCA TTCGGTANTG N 321

SEQ ID NO:6440
SEQUENCE LENGTH:123
SEQUENCE TYPE:nucleic acid
TOPOLOGY:linear
CLONE:HUMGS07606
SEQUENCE DESCRIPTION:
GATCTTTGTC CAGAAAATAA AGCTTTCTGT TGATTTTTTC TTTAAGCAAG AGGTAAGGAA 60
ATATGCACTT AAGANTATGT GTACAAGAAA ATAGTTTCTA AAATAAATTG AAATGTCTGC 120
AAA 123

SEQ ID NO:6441
SEQUENCE LENGTH:284
SEQUENCE TYPE:nucleic acid
TOPOLOGY:linear
CLONE:HUMGS07607
SEQUENCE DESCRIPTION:
GATCCACTCT GGACACTCAA CAAGACCTGA ACAGGATTTN CCTACCTGGT CCTTACACTA 60
CATCATCATC ATCTCATGCC CACCTGCCCA CACCCAGCAG AGCTTCTCAG TGGGCACAGT 120
CTCTTACTCC CATTTNTGCT GCCTTTGGNC CTGCCTGGCC CAGCCTGCAC CCCTGTGGGG 180
TGGAAATGTA CTGCAGGCTC TGGGTCAGGT TCTGCTCCTT TATGGGACCC GACATTTTTN 240
AGCTCTTTGC TATTGAAATA ATAAACCACC CTGTTCTGTG GAAA 284

SEQ ID NO:6442
SEQUENCE LENGTH:215
SEQUENCE TYPE:nucleic acid
TOPOLOGY:linear
CLONE:HUMGS07608
SEQUENCE DESCRIPTION:
GATCTGGTAC GNGGTGGACG TCATGGCCCG ANATGCCCAC GGNAACACAG CGCTGACCTA 60
CGACCGGCAG GCCTCCAGCC ATGGAGTGCA TCAACGTGCT TCTNCAGTAC GGCTGCCCCG 120
ACGNGTGTGT ATAGTATCTG TTTTATTTGA CTGCAGTCTC CTTGGTGCAA AANCAAANTG 180
GGGAANNTAA GGATAACTCA GNATTTCAAA AGGAN 215

SEQ ID NO:6443

SEQUENCE LENGTH:61
SEQUENCE TYPE:nucleic acid
TOPOLOGY:linear
CLONE:HUMGS07609
SEQUENCE DESCRIPTION:
GATCTNAGAC TTCTCACGTC CAGAACTGTA AGCAATAAAT GTCTGTTGTT TATAAATTAA 60
A 61

SEQ ID NO:6444
SEQUENCE LENGTH:75
SEQUENCE TYPE:nucleic acid
TOPOLOGY:linear
CLONE:HUMGS07610
SEQUENCE DESCRIPTION:
GATCCTTGCT CTACCTGGCT AGAGTTTCTN CTCATCAGAG CACTGGGACA TTAAACCAAC 60
CTTTTACAAC ACAAA 75

SEQ ID NO:6445
SEQUENCE LENGTH:207
SEQUENCE TYPE:nucleic acid
TOPOLOGY:linear
CLONE:HUMGS07611
SEQUENCE DESCRIPTION:
GATCTCAGCT CAGAGAGAGA GCATGAGGTC TTTTTTAACT GTCAGGAAAC AGAGCTGTGC 60
CCAATTCCAC TCAACTTTTG GCACAACTGT TAATCTGGGC CTTCACCTAC CTTAAACTGA 120
GTTTCTGCAA GCATAGCATT TTAGACACCC TGGAATAACC TTTTGGGAAT GATGCCACAG 180
AATAAAGTTC ACTCTTAACT TTTCAAA 207

SEQ ID NO:6446
SEQUENCE LENGTH:307
SEQUENCE TYPE:nucleic acid
TOPOLOGY:linear
CLONE:HUMGS07612
SEQUENCE DESCRIPTION:
GATCTGATAG TGGTAGGTCT ATCAGGAATA TCAGGNAAAA TNCTTTTAAT GTTGTTGGCA 60
AAGTCAATTC CTATCATTTG TACTTGACAC CAGCTACCTA GCAGGCTCAT TGTGAACTTC 120
AGGTTTCGCC CACGTTATTT ATATAAAGTA TCTCAGTGTA ATATAGGGAG TTGCTGGTTA 180
TTTGAAAATA GTAACAAAGG TAACCCAAAC GAGAAACTCC ATTTCCATAA TTNGCTTTCC 240
TGAAAGTCAG GCATGTCTTT GAGGACAGGT TTTGGCAGTA TTNNNNTNGT GCACAGGNGG 300
GAAATTN 307

SEQ ID NO:6447
SEQUENCE LENGTH:219
SEQUENCE TYPE:nucleic acid
TOPOLOGY:linear

CLONE:HUMGS07613
SEQUENCE DESCRIPTION:
GATCTGCCTG TTTATTTTGG TGGGTGGTCT TTCCTCCCTC CCCTACCACC CATGCCCCCC 60
TTCTNAGTCT GCCCCTGGCC TCCAGCCCCT AGGGGACTAG CTGGGTTGGG GTTCCTCGGG 120
CCTTTTCTCT CCTNCCTCTT TTCTTTCTGT TGATTGTCGC TCCAGCTGGC TGTATTGCTT 180
TTNAATATTG CACCGAAGTT TTTNAAATAA AATTTTAAA 219

SEQ ID NO:6448
SEQUENCE LENGTH:272
SEQUENCE TYPE:nucleic acid
TOPOLOGY:linear
CLONE:HUMGS07614
SEQUENCE DESCRIPTION:
GATCTGGTGT AGTATATTTT ATCGCATTTT CTTATATTAA AAAATGTCTG CATGATTACA 60
TTTTATTTCC TTTGTAATTT ACATTTCAGA ATAGTGTATT GCTATATGGG TGCCAAGATT 120
GANTATGAAG AACCCGAGTN TTTGTAGTAT TATAGTTTTA AGCAAATCTG TGTGGTGATA 180
CAGCCATAAG ANTGGGGCTT ATATAANCTC TGTACATGTA AGATTTTGTA CAGAGAATTT 240
TTAACTTTAT AAATTGTATA TGANCATGTA NN 272

SEQ ID NO:6449
SEQUENCE LENGTH:300
SEQUENCE TYPE:nucleic acid
TOPOLOGY:linear
CLONE:HUMGS07615
SEQUENCE DESCRIPTION:
GATCCACCCG CCTCAGCCTC TGAAATTGTT GGGATTACGG GCGTGACACN CGCGCCTGGC 60
ATGTATATCA TTTTNNATTG TGTATTTTGT AATTTTTGTT TGTNAATTTA TTTTTAATAT 120
AGTTTTCAAT TTAACTTGTT AGTTTCTGTA GTGGCAAATC CTTGTGTTTG CAATATTGGA 180
TTATTTCTCC ATAAGAGTTT CACATTTCTC CTGCTAGGAA ATCCATGGGG AATATTGGAA 240
TTGGGCTAGT TTTAATNNNA TNNCTAATGT TTAANGGTTC TGAAAAANAT TGGCGNCAAA 300

SEQ ID NO:6450
SEQUENCE LENGTH:286
SEQUENCE TYPE:nucleic acid
TOPOLOGY:linear
CLONE:HUMGS07616
SEQUENCE DESCRIPTION:
GATCTCCACT TTNCCAAAGG GCTGCNAGAG GTGATTGANA AGAACCGGCC TGCTGATGTN 60
AGGGCGTCCA CTGCCCCCAC ACCGTCCACA GCAGCTGTCT GAGCCCTCAA TCCCCAAGCT 120
GGCAGCTGTC ATTCAGGACC CCAACCCCTC TNAGCCCCTC TTTTCCCACA TTCATAGCCT 180
GTAGTGCCCC CTCTAACCCC CAGTGCCACA GAGAAGACGG GATTTGAAGC TGTACCCAAT 240
TTAATTCCAT AATCAATCTA TCAATTACAG TCCGTCCACC ACCAAA 286

SEQ ID NO:6451
SEQUENCE LENGTH:97

SEQUENCE TYPE:nucleic acid
TOPOLOGY:linear
CLONE:HUMGS07617
SEQUENCE DESCRIPTION:
GATCTGTGCA GAGAGAGATG GCAGCCACTC CCTTCAGTCC TCCCAGATTT CTGTAGCTAT 60
TTATGTAGCA GGCTCAATAA AATGTCTTCT CTCTAAA 97

SEQ ID NO:6452
SEQUENCE LENGTH:113
SEQUENCE TYPE:nucleic acid
TOPOLOGY:linear
CLONE:HUMGS07619
SEQUENCE DESCRIPTION:
GATCNNAGGC ATNNTTTCTT CAGTCAGAGC ATTCAATCCT CAAGCCATTC ACACATTTAG 60
GCCTCATGCT TTNTCCTGTC TTTAATCACA TCCATTTCCT ACACTCATTG AGN 113

SEQ ID NO:6453
SEQUENCE LENGTH:118
SEQUENCE TYPE:nucleic acid
TOPOLOGY:linear
CLONE:HUMGS07620
SEQUENCE DESCRIPTION:
GATCATAAGT CATTCCCCTC CCCTTCCAGG CCTCCTGCTA TATTTGGGGG ACCTGACTNG 60
NTTNGCTGGA GTCCCATGAG GATGTGGGCC CNTTAATAAN GGATAGCAAA CAGGCAAA 118

SEQ ID NO:6454
SEQUENCE LENGTH:233
SEQUENCE TYPE:nucleic acid
TOPOLOGY:linear
CLONE:HUMGS07621
SEQUENCE DESCRIPTION:
GATCCCAAAA TCCAAGGATG CTTCGAATCC CTTATATAAA ATGGCATGGT ATTTGCATAT 60
AACCTACATG CATCTTCAAA TATGTTAAAC CATCTCTAGA TTACTTATAA TACCAAATAC 120
AATGTAATGC TATGTAAACA GTTGTTACAA TGGATTGTCA AAGGCAAGCT CTNCTTGCCC 180
ANTNCGGGCC AANTGNGNGG TNCATGAGCA AATTAANTNN NTGCTGGTAT TTN 233

SEQ ID NO:6455
SEQUENCE LENGTH:253
SEQUENCE TYPE:nucleic acid
TOPOLOGY:linear
CLONE:HUMGS07622
SEQUENCE DESCRIPTION:
GATCCGAATT TCCTGCTCTA AACCCTCCAC TGCTTTCTGC TGTTTCCACT GAGGAAAGAG 60
GATGTGTGTC ATTAAAGCTT TGTTGGGACC TGTCACAGTC AAGGTGGGTA TAATTATGTT 120
TAATTTATTG AAGTGTTTCT GGGCATCGTC TTTTTCAAAT TCATATATNC TATCCAGAAG 180

ANGTGNATTC ATTNNNCATT ATNAAAAAAN ATAAAATATT ACCAGATAAA GCTCANCACT 240
NCNCTCTNCC CCN 253

SEQ ID NO:6456
SEQUENCE LENGTH:44
SEQUENCE TYPE:nucleic acid
TOPOLOGY:linear
CLONE:HUMGS07623
SEQUENCE DESCRIPTION:
GATCTTTCCT ATCAGTGTAA TAAAAACTCA TATTATGGCA GAAA 44

SEQ ID NO:6457
SEQUENCE LENGTH:320
SEQUENCE TYPE:nucleic acid
TOPOLOGY:linear
CLONE:HUMGS07624
SEQUENCE DESCRIPTION:
GATCAGGCAG GGTGCCATTN ATTGTCTTTC TCTCCTAGCC CCCTCAGGAA AGAAGGACTA 60
TATTTGTACT GTACCCTAGG GGTTCTGGAA GGGAAAACAT GGAATCAGGA TTCTATAGAC 120
TGATAGGCCC TATCCACAAG GGCCATGACT GGGAAAAGGT ATGGNAGCAG AAGGAGAATT 180
GGGATTTNAG GGTGCAGCTA CGCTCACCCT AAACTTTTGG TGGCCTGGGG TATGTCTTGA 240
GGCCCAGACT GTTAAGCAGG CTCTGCTGGC CTGTTTACTC GTCACCACCT CTGCACCTGC 300
TGTCTTGAGA CTCCATCCGN 320

SEQ ID NO:6458
SEQUENCE LENGTH:179
SEQUENCE TYPE:nucleic acid
TOPOLOGY:linear
CLONE:HUMGS07625
SEQUENCE DESCRIPTION:
GATCCAAATN AGAACAGATT GGTTATTGCA GGTATCACAG CCTAAAGAAA TTATCTTTTT 60
GCAAAAGAAA TATTAAATGA TTTAGCAGTC TCCACGTGTG TTAATGTTTC AAACGTGTAT 120
CATAATGTGT ATAATTGTGT AACAAAATTG TCTACAATAA ATCTNTTGGT ATTTGTAAA 179

SEQ ID NO:6459
SEQUENCE LENGTH:337
SEQUENCE TYPE:nucleic acid
TOPOLOGY:linear
CLONE:HUMGS07626
SEQUENCE DESCRIPTION:
GATCCATTCG CCCCAAGGGG ACAACGGGGT GTNAGNGGAG TAAGGGGTGG AGGTAGGGGC 60
CAGAGGGGCT TACACGATAT CCCATACCTT TAATGCCTTT GGCCTTCCAT TCTNATTTCN 120
CTNATGAGAA TATTGCTGGC CCTGCTTTCC CTGGTAGGTA TTTGCCAGGC CCAATGCTTT 180
AACCTTAAGC TGATACTTTG CTTTAGATGT CAGTCTCGTT ACCAGCAGCC TTTTGACCCA 240
ACTACGGCGC TCTATATTTA AGTNGAGGGA TTTTNANCCA TTGTGCATGG NAAAAAGNTG 300

TTCATGGGTA CNATNGTAAG NAAANNTAAA ANTTTTN 337

SEQ ID NO:6460
SEQUENCE LENGTH:101
SEQUENCE TYPE:nucleic acid
TOPOLOGY:linear
CLONE:HUMGS07627
SEQUENCE DESCRIPTION:
GATCGGTTGN GNCATGGAGT GGGGGCTTCC AGGTGGACCT NCCCGGCACA CATTCCATTT 60
NTTGAGCCCC AGTCCTGCCC CCCACCCNAC CCTCCCTACC N 101

SEQ ID NO:6461
SEQUENCE LENGTH:138
SEQUENCE TYPE:nucleic acid
TOPOLOGY:linear
CLONE:HUMGS07628
SEQUENCE DESCRIPTION:
GATCCTTCTT GGTTAGTATT ACTTGTNCCG GAGCAATTCT NATTCCTCGT TTAGACATTG 60
NNATTTCCTT CGTTGGAGCT GTGAGCAGCA GCACATTGGC CCTAATCCTG CCACCTTTGG 120
TNGAAATNCT TACATTTN 138

SEQ ID NO:6462
SEQUENCE LENGTH:172
SEQUENCE TYPE:nucleic acid
TOPOLOGY:linear
CLONE:HUMGS07629
SEQUENCE DESCRIPTION:
GATCTNATTA GNNTAGTAAT GTTTTCCCTT TAAACAGATT CATCATTATC CCTAAAGTAT 60
TGCTGTATTA ATACTGTCTT CTAGAAAGTA TACACCAGTN CCTACTTTCC TCCGATATCA 120
TTAGCTGTTT TCNGAAACTN AATTTCCTCT TCAGAGATTN CTCATATGTN TN 172

SEQ ID NO:6463
SEQUENCE LENGTH:239
SEQUENCE TYPE:nucleic acid
TOPOLOGY:linear
CLONE:HUMGS07630
SEQUENCE DESCRIPTION:
GATCCACAGA GTTCATAGGC ATGATTAAAT GGTAGTAGTT TTCCACCACG AAGTTTTGGA 60
ATAATTTGTC ACAAAGCAAT ANGGTAATTA GAAAAATAAC CTTTCAGAAG GAGACAGTTT 120
CTCCAAAGCT TGGGTACTTT CGCTTAATAT TAAANAGTGG ANGGAATTTT TTNCCTGCCA 180
GNCAAAAGGC AGNAGGTGTA TTTCTACACC ATGGNTAGGC AANTNNTTTT TTTNCCTAN 239

SEQ ID NO:6464
SEQUENCE LENGTH:213
SEQUENCE TYPE:nucleic acid

TOPOLOGY:linear
CLONE:HUMGS07631
SEQUENCE DESCRIPTION:
GATCTGCATT TAGCAATGTG ATGTCAGTAA ATGGACATAA CAGGATTGTT GTAAAGGTTG 60
GGCATGATGT ATGCAAAGTA CTGGCCAGGG TAGACTAATA ACTGATGGCA TTTATATGCT 120
GTGCTGGAAT ATTGTTACCA AGCTGATGTG CCGTTCTCAC CCNGGGNGAA TACTGGTTTT 180
GTCATTTCAT AAATGATATT TTTATAANTA AAA 213

SEQ ID NO:6465
SEQUENCE LENGTH:329
SEQUENCE TYPE:nucleic acid
TOPOLOGY:linear
CLONE:HUMGS07632
SEQUENCE DESCRIPTION:
GATCTGGCCA AGAATGAGGC CCTCCCAACA CTTTCACTCC CTCTCCAAGC CTTGATGGGA 60
CCTCCACTTA TTTAGGCCTN ATGTGCTTTG AAGAAGCTTT GAGAGCCAAT NTGTCTTCCA 120
CGGGTCTCTT TTTTGCTACA AGTAATCAGC CCCATGTNTT CTCTTAAACT GAGAATTGCA 180
CCGGGGGCAA TTCCTGTTTT CTAAGGTGGT CTCTGCTGCT ATTTAACAAC CCAGAGTAGG 240
CCTCTGTNAG GCTTCAGTGG CCTCAGAAAC CAGAGGGTCC AGATAGGGGG CCTGCTTGGG 300
CCCTCTGCTG NCAAACTGCT TAAAANTTN 329

SEQ ID NO:6466
SEQUENCE LENGTH:236
SEQUENCE TYPE:nucleic acid
TOPOLOGY:linear
CLONE:HUMGS07633
SEQUENCE DESCRIPTION:
GATCCACGTG TGTGGCCATA TTGTAACACA TTTTTCTGCA AATNACCTCT TTCATTTAAC 60
AGCCCTTATT CAATGGCCTT TTNCTTTTTC AGTAGTACAT ACACATCTGT GTCATTNGTT 120
GAATGACGNC ATGAATGTTT TGTAGATAAA ATATAATTAA ANGNTTTTAA GCATTTCTNA 180
CTCATAGATA TTNATGTTAG CACCAAATTT GNTGTGCAAT GANCCTCCCT ANATAN 236

SEQ ID NO:6467
SEQUENCE LENGTH:239
SEQUENCE TYPE:nucleic acid
TOPOLOGY:linear
CLONE:HUMGS07634
SEQUENCE DESCRIPTION:
GATCTATGCC TTAGTGTTGT TTTTNTTGTC TGCTTTGATG TAAAAGCAAG AGTATTTGGA 60
GCAAGAAGCT GCAATGCGCC AAATACACAN TAGGCCTTTC NTTTATCATT CATACGACAT 120
TTCTGGTTTA GGTGTATGTA GTTGGGCCAT GTTACTTGTC CAGAGGAAGA GNCTAAAGAT 180
ATACGGNTAA TTTTTTAGTC CNTAATGCTG TTTTGNTTAC NTNCTNTCAN GGTTATTTN 239

SEQ ID NO:6468
SEQUENCE LENGTH:82

SEQUENCE TYPE:nucleic acid
TOPOLOGY:linear
CLONE:HUMGS07635
SEQUENCE DESCRIPTION:
GATCGAGCAA TTGCAATCCA GCCTGGGCAA CAGAGCGAGA CTCCATTTCA AAAAAAGAAT 60
CAATAAAAAT GTTAAAAGCA AA 82

SEQ ID NO:6469
SEQUENCE LENGTH:348
SEQUENCE TYPE:nucleic acid
TOPOLOGY:linear
CLONE:HUMGS07636
SEQUENCE DESCRIPTION:
GATCCGTAAG GCCATGACCA AGGAGGCCAT CCGAGAGCAC CAGATGGCCC GCACTGGCGG 60
CACGAGACAG ACCTGTTCAC CTGCGGCAAG TGCAGGAAAA AGAACTGCAC CTACACACAG 120
GTGCAGACCC GCAGCTCTGA TGAGCCCATG ACCACCTTTN TTGTCTGCAA CGAGTGTGGA 180
AACCGCTGGA AGTTCTGCTG ACCCCTTGTG TAGATGTGCT GCAGCCTTNG GCCTTCNCGG 240
GCCACGTTCT TCGTTGACAN AGGTTNTNTT GGGGNCCCCT AGGAAGGCGG TATGTNCCTN 300
GCCNTTNAAN CTTGNCTGGN TTGGATTGNA NCTTTTTTGN CCTTTTTN 348

SEQ ID NO:6470
SEQUENCE LENGTH:178
SEQUENCE TYPE:nucleic acid
TOPOLOGY:linear
CLONE:HUMGS07637
SEQUENCE DESCRIPTION:
GATCGTGAAA TCTAAGGCTG CTTGATGTTC TGATGCTTGT NAATGCCGTC GTGCTCTGTG 60
ACTCCACGAA ACGCCAAGGA GCTTCTTCCG GACGNCTCCT CTTCCTCTGT AACATACGTG 120
TGAATAGTCA AGGTTTAATT TTNCTTTAAT CTAATAAAGT CGAAGTGGGA CTNTTAAA 178

SEQ ID NO:6471
SEQUENCE LENGTH:50
SEQUENCE TYPE:nucleic acid
TOPOLOGY:linear
CLONE:HUMGS07638
SEQUENCE DESCRIPTION:
GATCCAGCAC ACCTCCTCGG TTTTTTTTTC AATAAAAGTT TAGAAAGAAA 50

SEQ ID NO:6472
SEQUENCE LENGTH:108
SEQUENCE TYPE:nucleic acid
TOPOLOGY:linear
CLONE:HUMGS07639
SEQUENCE DESCRIPTION:
GATCTCTGTG AAACAAAATN AAGCAAGAAC GGAGACGCTG AATAGTTTTA TTTCTNTATT 60

AGAAACTGTG ATTGGAACAA TTGAAGAAAT AAAATCATAA CAGAGAAA 108

SEQ ID NO:6473
SEQUENCE LENGTH:210
SEQUENCE TYPE:nucleic acid
TOPOLOGY:linear
CLONE:HUMGS07640
SEQUENCE DESCRIPTION:
GATCTGAAGA AAGCAGCAAT ATCTGTNACT AGAGAACATT CCCATGTTTT TAAACTCTTC 60
ANTTCTTAGA TGCATTTAAN TTCTTAATGC AAATNACGTA GCAATTTGAA AACTTCTCCG 120
TATTACTNGT GTTTAAAATG TCTTGCTTTA AATACAANNC AAATGGTAAA GGGGATTATC 180
TTTGGTTTAG ATGGTTAANT ATNATTTNGN 210

SEQ ID NO:6474
SEQUENCE LENGTH:307
SEQUENCE TYPE:nucleic acid
TOPOLOGY:linear
CLONE:HUMGS07641
SEQUENCE DESCRIPTION:
GATCAGCAGG ACTGTCTCCA GCACGGAGCT GACACTGTTC AGCTGCCTCA ACTGGTAGAC 60
GCTCCCAAGA AGTCAGAGGC AGCNGTCGGG GCGGAGGTGT CCATNACCTC CCCAGGACAG 120
AGCAAAAACT TCTCCCTCAA GAACAGAAAC GTTTTACCNN CTATTGTATG ACCTTTGCTG 180
AGGGTATGTC CTGCTCCTTT CCACCAGTGA TTTGTATTAA GNCAGCACTT ATATTGTACA 240
ATACTTCAGN CTGTTTTTTT TAAATACATA AAACTTTATG TTAAAAAACT CTATTANCAT 300
GGGCAAA 307

SEQ ID NO:6475
SEQUENCE LENGTH:140
SEQUENCE TYPE:nucleic acid
TOPOLOGY:linear
CLONE:HUMGS07642
SEQUENCE DESCRIPTION:
GATCACAGGT TAGGGGTTTC TCTAGATGGG GGTTCTGAAA TTTGCAGTGT CTGCTCCTGG 60
GAGGCAGCAC CAGAAAGGGC ACTGAAATGT ACTAGCTGGA TGTNACCCAG TCTTAATAAN 120
CAGGTTTTCT AATCCAGAAA 140

SEQ ID NO:6476
SEQUENCE LENGTH:187
SEQUENCE TYPE:nucleic acid
TOPOLOGY:linear
CLONE:HUMGS07643
SEQUENCE DESCRIPTION:
GATCAACTTT GTATATTCAC GTGTATTAAA ATATTGTGCA CTAAATGTTT TGCCCTTGTT 60
TGCTATTATA TGGTCAAGGC ATTTATCAGC ACTATTGTAA TGAACTCATG TAAGTGGCAT 120
GGGTCAGGGA AAATTATTTC CTACTTTNCT GCCTAATTAA ATTNCTGTTT TCCAGTATTA 180

CATTAAA 187

SEQ ID NO:6477
SEQUENCE LENGTH:113
SEQUENCE TYPE:nucleic acid
TOPOLOGY:linear
CLONE:HUMGS07644
SEQUENCE DESCRIPTION:
GATCTTGTAT ATTTNATTTT TAAAAAACTA GAATAAACAG AGAGGCATAA NCATATCTTA 60
GAGTCCAAGT GGTAGTGTTT AGCATTGGAT ATAATAAATG GATGTTTTAC AAA 113

SEQ ID NO:6478
SEQUENCE LENGTH:276
SEQUENCE TYPE:nucleic acid
TOPOLOGY:linear
CLONE:HUMGS07645
SEQUENCE DESCRIPTION:
GATCTCATTA TGGGCTAAAA TAAGACAATA TTCAAAGGTC AGAGATATCT AGCCAGAATC 60
TNATGGAGGC TGGATTTCAG ATTTTNTNAC AGAATTAGAC AGAGGAACAC AGAGGGGACA 120
GGCTCAGTTA GGGTGGAGGT GTGGGGTAGG GAAGCAGGNC TTGATATAAN TNATTGGAAT 180
CATTGTCTTT TAANCCAGTG GTTTATGTCA GGGTATAGCG TTTCANGGGA TTTGAGGGTC 240
AGATGGGGNA ATGTAGCCTC TTATTTTGCC AGTGTN 276

SEQ ID NO:6479
SEQUENCE LENGTH:160
SEQUENCE TYPE:nucleic acid
TOPOLOGY:linear
CLONE:HUMGS07646
SEQUENCE DESCRIPTION:
GATCTGTCAC TGTNTCCCGT CACGCCTTGA TGGGACCGTC TAGTTGCTGG AAAACAAACT 60
CAGGGCTCCC ACTGATTCTA CATTATGGTG TGTTGTACAA TTATTTCATT ATATATNACA 120
AGGTAATANT AATAGANATA AAATCCACAG TAANTGCAAA 160

SEQ ID NO:6480
SEQUENCE LENGTH:158
SEQUENCE TYPE:nucleic acid
TOPOLOGY:linear
CLONE:HUMGS07647
SEQUENCE DESCRIPTION:
GATCATAAGT NTTAGGACCA AAGGTGTTTT TAATGAGATA ATCTTTTTNA TTTNNTAACA 60
AGAAAGGAAA TATTCACTTT ACATTTTAAC ATCAACTTTT AAGTGATATT CAGANTTAAT 120
TGACCATGGT GATACGTTGT AAAANGTTTN CATATTAN 158

SEQ ID NO:6481
SEQUENCE LENGTH:112

SEQUENCE TYPE:nucleic acid
TOPOLOGY:linear
CLONE:HUMGS07648
SEQUENCE DESCRIPTION:
GATCCAAAAA AAAAAAAAAC GAANANAATT NATCCTCACA AATTGGTGTT CTAAATATCT 60
TAAGANCCTA ATTAAATAGC TGACTACAAA AAAAAAAAAA AAAATNCAAA AN 112

SEQ ID NO:6482
SEQUENCE LENGTH:105
SEQUENCE TYPE:nucleic acid
TOPOLOGY:linear
CLONE:HUMGS07649
SEQUENCE DESCRIPTION:
GATCCTGTGT GGCCGGTGTT TGCATCTTCA GTGGCCACAA GCATAATAAA GCCCCTTTGC 60
CTTTCTCTGT ATTATATTCA ATACAATACA TCAATAGTCT TGAAA 105

SEQ ID NO:6483
SEQUENCE LENGTH:252
SEQUENCE TYPE:nucleic acid
TOPOLOGY:linear
CLONE:HUMGS07650
SEQUENCE DESCRIPTION:
GATCAGATTT AAAGNCTGGA TTCTATCTAC ATAAGTCCTT TNAATTCCAC CAGGGCCAGA 60
GCAGCTCCAC CACTGTGCAC TTAGCCATGA TGGCAACAGA AACCAAGAGA CACAATTACG 120
CAGGTATTTA GAAGCAGNGG GNCAACCAGA AGGCCCTTAA CTNTCACCAG TGCATCACAT 180
CTGCACACTC TCTTCTCCAT TCCCTAGCAG GGGNGGCTAG CTCATTTNAC NGGGTNNAGA 240
ANCTGAGGNC CN 252

SEQ ID NO:6484
SEQUENCE LENGTH:36
SEQUENCE TYPE:nucleic acid
TOPOLOGY:linear
CLONE:HUMGS07651
SEQUENCE DESCRIPTION:
GATCACGNGT AAAGAGAACT GTTTCCTGCC CTTAAA 36

SEQ ID NO:6485
SEQUENCE LENGTH:125
SEQUENCE TYPE:nucleic acid
TOPOLOGY:linear
CLONE:HUMGS07652
SEQUENCE DESCRIPTION:
GATCAGCCTC ATCAATATTG CCGTCATCTG TTTAACACTC CCAGTATATT TCCTCAATGT 60
CTGTTTACTT AAAATTTTGT GGAGTGACAT AATTAATAAG NAATAAAGTC TGAATTATAC 120
ACAAA 125

SEQ ID NO:6486
SEQUENCE LENGTH:128
SEQUENCE TYPE:nucleic acid
TOPOLOGY:linear
CLONE:HUMGS07653
SEQUENCE DESCRIPTION:
GATCCACACT GGGGNGAAGC CTTATNAGTG TACGANATGT GGGAAGAGCT TCAGTCAGCA 60
TGCGGGCCTC ANCTCCCACC AGAGACTCCA CACCGGNGAG AAGCCATATA AGTGTAAGGN 120
GTGTGGGN 128

SEQ ID NO:6487
SEQUENCE LENGTH:329
SEQUENCE TYPE:nucleic acid
TOPOLOGY:linear
CLONE:HUMGS07655
SEQUENCE DESCRIPTION:
GATCCAGAGA GCTTCCTGAA GGACCTGTTG AACTCAGTCC CCTGACCACC ACACAGCAGC 60
TGCGGCGGCG AAGAGAAGCT GGCTTGCCTT CCACCCTCTG TTCTCCCTCC TTGTGCATTA 120
AGTTCCCTCC GNGGGATGCT GCATTGGTAC CCAGCCCTCC NCTATCTCAT TTTTCTTGGN 180
GTGGCTTGGG GTTTTTAGGC TTCCNGTTTT ATCTCGTGTG TGTGGTGCAC CAGCTATGAG 240
GTTGTCTGTA ANCNAAGCCA TCAAAGGGNC TNTAAATANC TAGGNGCCAT GAGTNGTCCC 300
GGNCANCTTT CANAACTTGA GTNTGANAN 329

SEQ ID NO:6488
SEQUENCE LENGTH:101
SEQUENCE TYPE:nucleic acid
TOPOLOGY:linear
CLONE:HUMGS07656
SEQUENCE DESCRIPTION:
GATCTGTACT GTAATTTTGT TTGTAATCCT GTATATNATG GTGTAATGCA CAATTTAGAA 60
AACATTCATC CAGTTGCAAT AAAATAGTAT TGAAAGTGAA A 101

SEQ ID NO:6489
SEQUENCE LENGTH:215
SEQUENCE TYPE:nucleic acid
TOPOLOGY:linear
CLONE:HUMGS07657
SEQUENCE DESCRIPTION:
GATCATGTAT ATAATTGTAA CATCACATTG ATTTTACGGA AGATGTTATA TGGNCTTTAA 60
TGACACAATG TTTAGNGATA AAATGTACAT TATTTTGGTT CAGTTTTTTA AAAAANNTAT 120
GCTTTAACAA AATTCTTAGG AATNCTTTTA AGCAATGCAG GTATTGCGAT ANCTGTAGNT 180
TTTACAATAA TGTTACTCTA CAGNTGGGAN AATNN 215

SEQ ID NO:6490

SEQUENCE LENGTH:364
SEQUENCE TYPE:nucleic acid
TOPOLOGY:linear
CLONE:HUMGS07658
SEQUENCE DESCRIPTION:
GATCTCTTTG TATTCTCTTC AAGCCCAAAC CAATTCTGTN CCTTCAATCT AAATAGTGGT 60
AATATGAATG TTTAAGAAAT GAAATAAGAA ACATGTGCAG GCACTTTGGA AGGTGCTAAG 120
TGACTGCCCT AAGGAATGAA AAGCAAGGGC CAGGTGGGAG TAGCCCAGCG AAGGCACTTG 180
GGCTGCCAGG AACAGGNGGC GTGGGAAACT CTGGCTTAGG NAAACATGAA CACNGGGGCA 240
ACAGCGGCAN ACTGTTGTTC GAGTTAAATC TAANTCTCAG GCTCTTTNAC GGTAAANGGG 300
TTANGGATAC TCCATTNNGA TGAAGANAAG NGTGAGGCTG ANNGTAANGC ACATGGCAGN 360
TNNN 364

SEQ ID NO:6491
SEQUENCE LENGTH:85
SEQUENCE TYPE:nucleic acid
TOPOLOGY:linear
CLONE:HUMGS07659
SEQUENCE DESCRIPTION:
GATCTTTCTT CTCCTTTAAG CTCTTTGTTA ACATTTCTCT AGACGCAATT TATTGAAAAT 60
AAAGATTTNC TGTTTACTCA TGAAA 85

SEQ ID NO:6492
SEQUENCE LENGTH:122
SEQUENCE TYPE:nucleic acid
TOPOLOGY:linear
CLONE:HUMGS07660
SEQUENCE DESCRIPTION:
GATCCTCATT TATGTTTGTA GTTGGAAAGC AAAGCTAGGT AGCCATTTCT TCTGTTCTAC 60
CAAGTTATAA TAGCATTCAT TTCCCTTTAT ATTTCCCTGA AATAAAGCAC ATTCCACTTA 120
AA 122

SEQ ID NO:6493
SEQUENCE LENGTH:362
SEQUENCE TYPE:nucleic acid
TOPOLOGY:linear
CLONE:HUMGS07661
SEQUENCE DESCRIPTION:
GATCCTACAT TAAGGTCATT TGTNAGAATG ATGTTTGTGT TTGTNTAGAG TTGGCTGACC 60
TCCAACTCNT GGGGTCAAGG AATCCTACTG CCTNAGCTTC CCAAATAGCT AGGACTATAG 120
GCATGCACCC GGCCATGTGT TTTATTTATA GCTCTTAAAG CCCAGATGAA GAAATCACAT 180
TTTTNCCCAT AGTGAAGAAA CATTTGGCCA TTGCTTAGTC CTTNNGGGGG GTGNCTGTCC 240
TGTTTTCATT AGATTAGNGA GACCCTGTGT GGGCCACAGT TAATATANNC CATTATCACT 300
TTTAAGGTAA CCTGCACATC TTAGGTTTCA TAATTNCCTT ATTGTTCTNG NCTCAAANTT 360
GN 362

SEQ ID NO:6494
SEQUENCE LENGTH:328
SEQUENCE TYPE:nucleic acid
TOPOLOGY:linear
CLONE:HUMGS07663
SEQUENCE DESCRIPTION:
GATCGGGGCA CTGCAGGGGC CGAGCCATTT TGGGGGGCCC CCCTCCTTGC TCTGCAGGCA 60
CCTTAGTGGC TTTTTTCCTC CTGTGTACAG GGAAGAGAGG GGTACATTTC CCTGTNCTGA 120
CGGAAGCCAA CTTGGCTTTC CCGGACTGCA AGCAGGGCTC TGCCCANAGG CCTCTCTCTC 180
CGTCGTGGGA GAGAGACGTG TACATAGTGT AGGTCAGCGT GCTTAGCCTC CTNACCTGAG 240
GCTCCTGTGC TACTTTGCCT TTTGCANACT TTATTTTCAT AGATTGAGAA GTTTTNTACA 300
GAGANTTAAA AATGNAATTA TTTNTAAA 328

SEQ ID NO:6495
SEQUENCE LENGTH:351
SEQUENCE TYPE:nucleic acid
TOPOLOGY:linear
CLONE:HUMGS07664
SEQUENCE DESCRIPTION:
GATCCTGTTT CTACCAACAC TGCACCTTAT CCCAGGAACC TGCCCTAGAC CTCCAGAGAC 60
CATATTTNNT CTCCCTCCAT TTCTACCCAG ACCTCCAGGC CTCCTTCTGG AATNATAGAA 120
CCGTAGAATT GGAAGGAATT TNAGAGGGGN TCTAGTTGGA GTTGTGTCCA ACAGAATTCA 180
TTAACACCAG CCTGGGCTTG TTTTTCCTCC TCCCTCTGGA CTTTTTTAAT CTTTTCCTCC 240
ACCTCAAAAA ATACTTACAC ACAGATTCTT CTTGTACAGG CATCAAAACC AACTNCTNTG 300
CCCCTAAGGC TGTGTNNGNG TGGTNTCCAG NNANCCNTAC CNCAGTCANT N 351

SEQ ID NO:6496
SEQUENCE LENGTH:122
SEQUENCE TYPE:nucleic acid
TOPOLOGY:linear
CLONE:HUMGS07665
SEQUENCE DESCRIPTION:
GATCTGGTAG GGGAGAAATG GCGAATGTAA TACACATGAN ATGGTATATC CTTGCAATGT 60
ACAGTATCAG AAGGTGGTTT GACAGCATCA TAAACAGGCT GACTGGCAGG AATGAAAACA 120
AA 122

SEQ ID NO:6497
SEQUENCE LENGTH:227
SEQUENCE TYPE:nucleic acid
TOPOLOGY:linear
CLONE:HUMGS07666
SEQUENCE DESCRIPTION:
GATCANTTGC AAACCTCCCC CAGCCCCTAC CATCCAATNT GGACGGAAAA CANGAACTGC 60
CTGAAGAAGA GTCCAAGCTA CAGATACACA GCGTGTGCAT TGCGGCTGTC ACCTTCCTNC 120

TNCCACTTCT GTATCCTCAG AGATGCTGCG TGGATGTTTC CTTANCCTCA GCTGACTTCC 180
CTGTGAATNT CTAATGCTAG TTCANGGGCC TNCNGGAAAT TGANTTN 227

SEQ ID NO:6498
SEQUENCE LENGTH:238
SEQUENCE TYPE:nucleic acid
TOPOLOGY:linear
CLONE:HUMGS07667
SEQUENCE DESCRIPTION:
GATCTTGCCC TTCTCACTCC AGAGGCTAGT GGTCACAGAC AGCTGGGAAT GGCAGCCACA 60
GAGGGTCCCC TCTGGGAGAA ACAGCTTCAC CCCAGCCTCA GGGCCCTGGG CCATCACTGC 120
AGTGGCCCTG GGAGGTGAGG AAGAAGCTGG CTAGAGGAGG GGGCTCCCAC CTACCTTTTA 180
TTTAAGCCAG TATTCTTTGT TCCTGCTTGT AATAAAACTT CAGTTTATAA GAGTTAAA 238

SEQ ID NO:6499
SEQUENCE LENGTH:142
SEQUENCE TYPE:nucleic acid
TOPOLOGY:linear
CLONE:HUMGS07668
SEQUENCE DESCRIPTION:
GATCTGTCAT AACTCATTTT AAACTGTGAA ATATGCCATA TGAAGAGGGN TTAAGAGGCT 60
GNGAAACTCA TATTTCAACC AAATCCAGCA TTAGTTTTTC TTAGGTCTAA TANTTCCTNG 120
TTAATAAAGA TTTAAATGCA AA 142

SEQ ID NO:6500
SEQUENCE LENGTH:160
SEQUENCE TYPE:nucleic acid
TOPOLOGY:linear
CLONE:HUMGS07669
SEQUENCE DESCRIPTION:
GATCTGTCAT TGTCTCCCAT CACCCCCAAA TGGGCCCCTC TCGTTGCAGG AAAACAAGCT 60
CAGGGCTCCC ACAGTTTCTA CATTATGGTG AGTTGTATAA TTNTTTCATT ATATATTACA 120
ATGTAATAAT AATAGAAATA ANGTACACAA TNNNTGTAAA 160

SEQ ID NO:6501
SEQUENCE LENGTH:266
SEQUENCE TYPE:nucleic acid
TOPOLOGY:linear
CLONE:HUMGS07670
SEQUENCE DESCRIPTION:
GATCTGCCCG AGACTCCCAG AGGGTAATGC CACTCCCACA ATCTCAGGCC TGGTACCCAT 60
CCTNTTTCCA CTGTGAGCAG AGCCAGAAGG TAGGTCTNTT CAGAGTCTGT NNCCCTGGAC 120
CTGGGGNGTG GATATGANAT GGGATATCTC CTTCCATTCC CCGNTCCAGG GGAGAGTCAC 180
TAGTTGTACC CTACTCCATT AGGTCCCAAA TAGGGGCCCA ATTTCACCTG TATNAGGNTC 240
TGTGCATNCC AGCTGCCNNA AATNTN 266

SEQ ID NO:6502
SEQUENCE LENGTH:119
SEQUENCE TYPE:nucleic acid
TOPOLOGY:linear
CLONE:HUMGS07671
SEQUENCE DESCRIPTION:
GATCCAGACT TAGGGACGAG GCTGTCACTG GTGGGCACCC TCTGTTCCTG TTTGTGTGTT 60
TGAATAGTCT GAAATNCTGT GACTTTTTTT GTGTGAATAA AGNTATGAAA CTTCTGAAA 119

SEQ ID NO:6503
SEQUENCE LENGTH:90
SEQUENCE TYPE:nucleic acid
TOPOLOGY:linear
CLONE:HUMGS07672
SEQUENCE DESCRIPTION:
GATCTTCGTG TGAGCCGCGN CGTCCGNCGN GACCGAGCTG CCTTCGCGTG CCCCCGNCCT 60
GTGTTTTATA AAAAGAAAGA TTCTCGGAAA 90

SEQ ID NO:6504
SEQUENCE LENGTH:62
SEQUENCE TYPE:nucleic acid
TOPOLOGY:linear
CLONE:HUMGS07673
SEQUENCE DESCRIPTION:
GATCTGTCAG TGTATCAAAA CAACATAAAT AAACTTGAAC AACTGATACG CAAAAAAACA 60
AA 62

SEQ ID NO:6505
SEQUENCE LENGTH:72
SEQUENCE TYPE:nucleic acid
TOPOLOGY:linear
CLONE:HUMGS07674
SEQUENCE DESCRIPTION:
GATCAAGTTT GGTCAGGTTT TGTCACCAAG CTTTGTAAAT AAACTGGTTT TCATAGCTTT 60
TTGGAGATGA AA 72

SEQ ID NO:6506
SEQUENCE LENGTH:221
SEQUENCE TYPE:nucleic acid
TOPOLOGY:linear
CLONE:HUMGS07675
SEQUENCE DESCRIPTION:
GATCTTTTTT CAGAATGTTG GAAAATCCTG TAAATGCAAA TAGTCAATAC TGTATTAAAT 60
ACGTGCACTT GGNGTNTGCT TCGCTTGTAC AGTTGTAAAT AATCAGAACA TATGNAAAAG 120

GTACCCTACA GAGGAAATTC TGATACAGAT TATTGCTATA TTATAACCTN TTGCTGTTGA 180
GCGGGCTGTA GGTAANCTAA TGTTGTGGTT TGACTATTAA A 221

SEQ ID NO:6507
SEQUENCE LENGTH:42
SEQUENCE TYPE:nucleic acid
TOPOLOGY:linear
CLONE:HUMGS07676
SEQUENCE DESCRIPTION:
GATCTTCATT TTTGTAATAA AACGGNAGAC TCATCCAGTA AA 42

SEQ ID NO:6508
SEQUENCE LENGTH:62
SEQUENCE TYPE:nucleic acid
TOPOLOGY:linear
CLONE:HUMGS07677
SEQUENCE DESCRIPTION:
GATCTTAGAC TTCCCATCCT CTAGAACTGT GAGAAATAAA TTCCTGTTGT TTATGAGCCA 60
AA 62

SEQ ID NO:6509
SEQUENCE LENGTH:117
SEQUENCE TYPE:nucleic acid
TOPOLOGY:linear
CLONE:HUMGS07678
SEQUENCE DESCRIPTION:
GATCTGCCAT CTAGAACTCA TATTCTAAAG GGCAGTGNTT TCTCAGAACA GTGATGTTCT 60
GGAATATGTA TTATTTATTT TAACACTTTT TTAATAAANT CTTTATTATA AACCAAA 117

SEQ ID NO:6510
SEQUENCE LENGTH:113
SEQUENCE TYPE:nucleic acid
TOPOLOGY:linear
CLONE:HUMGS07679
SEQUENCE DESCRIPTION:
GATCTGTTTA AAAGATTAAA TCTCAAGTTA ATTAAAAATA AGCTTTTCAA AAATGTATTA 60
TATTTATAAC AAATATACTG TAAATAGANT AAAGACATGC TATTCACTGT AAA 113

SEQ ID NO:6511
SEQUENCE LENGTH:159
SEQUENCE TYPE:nucleic acid
TOPOLOGY:linear
CLONE:HUMGS07680
SEQUENCE DESCRIPTION:
GATCTGTCAC TGTCTCCCAT CACCCTAAGA TGGGNCTGTC TAGTTGCAGG GAAAGAAGCT 60

CAGGGCTCCC ACTGATTCTA CATTATGGTG ANTNGTAGNA TTATTTTATT ATATATTACA 120
ATGTCAATAA TAGAAATAAN GTGTGCANTA AATGTCAAA 159

SEQ ID NO:6512
SEQUENCE LENGTH:219
SEQUENCE TYPE:nucleic acid
TOPOLOGY:linear
CLONE:HUMGS07681
SEQUENCE DESCRIPTION:
GATCAGCCGG AAGAGAAAAT NAAGACCCCA GAGACATTTA TTGGGGAGTA GGATGTGGCT 60
GAGTGCTTTT TTTTTGGCCA GACTAGCGGA TTCAGTCCTG GAAGAGAGTA TCATATAATG 120
AGNCCCACAG GCACTGGCAC CCTTGGGTTG GNAATAGANG NNGNCCATGG ANTGGCGAAA 180
ACCAGGGATT CCAGCTGGGG NATAGTANCT TGAAGGTGN 219

SEQ ID NO:6513
SEQUENCE LENGTH:217
SEQUENCE TYPE:nucleic acid
TOPOLOGY:linear
CLONE:HUMGS07682
SEQUENCE DESCRIPTION:
GATCTTGACC TACAGTTTGA AAAACAGAGT ACTGAAGAGT CTTGTTTCCT TTATTTAGAA 60
CAGTGCAAGG ATGAGTATCT GGGTAGAGTT GTGCGTTGTA CATAACCTTT GATAGTCATA 120
TAGTTGAACC AAAAGCAANN TCTGTAGTGN CATTGAAAAA ACATTTTGAC TTGATTTATC 180
CTGNCAGNNT NCNNTAATTT TTGGCTGGCT AATNCAN 217

SEQ ID NO:6514
SEQUENCE LENGTH:119
SEQUENCE TYPE:nucleic acid
TOPOLOGY:linear
CLONE:HUMGS07683
SEQUENCE DESCRIPTION:
GATCTCTGCT CACGTTGCCT CCCTTCATCT TCCTGCATAA GTGGAAGCAG CCTGAAGCCC 60
TCACTGTTTC TTGTACAGCC TGCAGAGCCG TGAGCCAAAT AAAGCTCTTT TCTTTTAAA 119

SEQ ID NO:6515
SEQUENCE LENGTH:173
SEQUENCE TYPE:nucleic acid
TOPOLOGY:linear
CLONE:HUMGS07684
SEQUENCE DESCRIPTION:
GATCTGATGG TTTTATAAGC ATCTGGCATT TCCCCTGCTT GCACTCACTC TGTCCTGGTG 60
CGCTGTGAAG AAGGTGCCTG CTTCTCCTTT GCCTTCCACC ATGATTGTAA GTTTCCTGAG 120
GCCTCCCTGG CAATGCAGAA CTGTGAGTCA ATTAAACTTC TNTTCTTTAT AAA 173

SEQ ID NO:6516

SEQUENCE LENGTH:238
SEQUENCE TYPE:nucleic acid
TOPOLOGY:linear
CLONE:HUMGS07686
SEQUENCE DESCRIPTION:
GATCACCATG TNAGCCTTAG ACCAAGAAGC TGGCTAGTCC TTNCTGTGAA GCTAATACAA 60
TNGNCATTTC CAGACAAATT TAAAGGNAAC ACTAAGGCTG CTTCAAAGAT TATCTGATTC 120
CTTTAAAATA TATGTCTATA TACACAGGCA TGCTCTTTTT TTAAGTGCTT ACATTTTAAT 180
AGAGATGANT CAGTTTTNGA ATCTANGCTG TTTGCCCAAG NTGANGCTNC CAGGGTTN 238

SEQ ID NO:6517
SEQUENCE LENGTH:289
SEQUENCE TYPE:nucleic acid
TOPOLOGY:linear
CLONE:HUMGS07687
SEQUENCE DESCRIPTION:
GATCTGTTTT TNACCACTTC ACTGAAAGAC ACCATTTATC TACCCAAGGG CAGAAAGTAG 60
AACTTACTAT TCATTAAATG TTTGACACAA TTGGAATTGT CTTTAATTTC TGTCAGAATG 120
CTATTGAAAA TGTGAATTGC ATGACTTGGT AGCATATTCT TTTCNTGCAA AAATAGGCCA 180
TATTAACCAT GCTTATGACC ANTGNCCTGT GCTACTGGCT TTTGGNAAAA ATGGTTTGCN 240
TCAGNTTGGG GAAGTAATNA AAGGANTNCA CCTGGNGNCC CNNGAATAN 289

SEQ ID NO:6518
SEQUENCE LENGTH:236
SEQUENCE TYPE:nucleic acid
TOPOLOGY:linear
CLONE:HUMGS07688
SEQUENCE DESCRIPTION:
GATCCCCCAG GATTCTATTN TGTTTAATGG GCTTTTCTAC TAAAAGCATA AAATACTGAG 60
GCTGATTTAG TCAGGGCAAA ACCATNNACT TTACATATTC GTTTTCAATA CTTGCTGTTC 120
ATGTTACACA AGCTTCTTAC GGTTTTCTTG TAACAATAAA TNTTTTGNGT AAATANTGGG 180
TACATTNTAA CAAACTCNGT AGTNCAACCT AAACTNGTAT NAACGGTGTG TAAAAN 236

SEQ ID NO:6519
SEQUENCE LENGTH:157
SEQUENCE TYPE:nucleic acid
TOPOLOGY:linear
CLONE:HUMGS07689
SEQUENCE DESCRIPTION:
GATCAGGTCT GCTTTNAGTT TCATTTTTGT TTCTTTCCCG TCCCACTCTT TAAAAACTGG 60
TTCCGTGAGA AANGGCAGAA GCCGTTCCGT GTCTCTTGCA GGCTGGGCCG NCTTCATGNA 120
GTNCGAGGGC GTNCCGTGAC NACGTACATA CGTATGN 157

SEQ ID NO:6520
SEQUENCE LENGTH:195

SEQUENCE TYPE:nucleic acid
TOPOLOGY:linear
CLONE:HUMGS07690
SEQUENCE DESCRIPTION:
GATCTTTCTG GATATAATGA AGCTTCCTGG AGAAACAGAC TGGCAATCAG AAAGATTTGG 60
CTGCTTCCGA CACCTCCATC TTGGGCCTGC AGAGGCTGCA GCAATCTGTG AGGCAGCCGA 120
ATCTGCGNGG CAGNCCACTC TTCTAGGACA TATGGGTTTC ANNAGGCACC NTTTTGCTAN 180
GNAGGCATAN TTANN 195

SEQ ID NO:6521
SEQUENCE LENGTH:337
SEQUENCE TYPE:nucleic acid
TOPOLOGY:linear
CLONE:HUMGS07691
SEQUENCE DESCRIPTION:
GATCGAAACC AAGTTATTAT TATCCTTACT GTATTCCATT GCTTGTATCA AAATCTACTT 60
TTTTNCTAGT AGGAAACTAT GCATTCCCTA TCAGTCCCTT TGATTATCAG GAATGTATAA 120
GAATTGTGAT AAAATAGGCC AATATGCTAT TTGGTACCAT TTGTATGTAT TTTANGTGCA 180
TAACCTAGGT AAAATAAGNN TAGTTTCTAT GTGTAAAGAA GCAAGTTTTA CAGTATGCAG 240
ACTCCAGTGG ATGTATTTTA GGCTGTATAC ATNTTTAAGC TCTNTNTGGT ANATAGGGTA 300
TACAAACTAN NTGCCTCNNA ATNCNAATAN NTTCGGN 337

SEQ ID NO:6522
SEQUENCE LENGTH:331
SEQUENCE TYPE:nucleic acid
TOPOLOGY:linear
CLONE:HUMGS07692
SEQUENCE DESCRIPTION:
GATCATTTCA TGGAATCTTT GAAGTATCTT TGACTCTAAC TTTGACTTGG TGGTGGACCT 60
TCCTTGGTTT TANTAACACC TAAGAGATAT CCTTTAGAAT TACATGTATT TTAGCATAAG 120
GAAATTGNAA AAGTAAAACA TACTGGTTTT TTTCAACAAG ACCATATGTA AATTAAATAG 180
TGAAATGTGT ATGNGTTTCA GTAGAACTGT ACCATCAACA ATGTTTCCAT AAATATGCAG 240
NGTTCTTNCT NNTGTATTGT TATTTACAAT ATTGTTAAAT NGGATGCATT TGCAATNNCT 300
CGGNTTCTAA AGNATTGNGG TNCNGANCGT N 331

SEQ ID NO:6523
SEQUENCE LENGTH:107
SEQUENCE TYPE:nucleic acid
TOPOLOGY:linear
CLONE:HUMGS07693
SEQUENCE DESCRIPTION:
GATCTGGAAT GGTGTTTTGG TTTGGGGGGA ATTTTTTTTT TTCCCNGGCA AATNACATGT 60
GTTGTTGATG TGAGTATCTG ATGAAAAANC AATGNCAGAA TANCCGN 107

SEQ ID NO:6524

SEQUENCE LENGTH:196
SEQUENCE TYPE:nucleic acid
TOPOLOGY:linear
CLONE:HUMGS07694
SEQUENCE DESCRIPTION:
GATCTATATA TGTATAAATA TGTATTTTGT CAAATTTGTN ACTTAAATAT ATAGAGACCA 60
GTTTNCTCTG GAAGTTTGTT TAAATGACAG ANGCGTATAT GANTTCAAGA AAATTTAAGC 120
TGCAAAANTG TATTTCCTAT AAANTGAGNA GTCTCACTGN TAGCGGTTCT TTATTGCTCA 180
TTTTTTAAAA ANTGGN 196

SEQ ID NO:6525
SEQUENCE LENGTH:257
SEQUENCE TYPE:nucleic acid
TOPOLOGY:linear
CLONE:HUMGS07695
SEQUENCE DESCRIPTION:
GATCGGCTCT AGAAACACTG CTTTCCCTTC TTTNCTTTTG TTTTCTTTCT NTTATTATTT 60
TTTTAAAGAG TAAACGAAAG TNCTGTATGA ATTCGGACCC AGGCAGCAAC CACAGGNCTN 120
GGGGTGAGAC CCTCCTCCTC CCAAGNGAGC AAAAAGGACC CATGGCCCCC AAAAAAACCC 180
CTCAACGAGA ATCCTCAGCN TTGTAAAATG CAAAANTGTC TAATGAATAT TTNTATNTGT 240
NCCATTTTTG ATAANTN 257

SEQ ID NO:6526
SEQUENCE LENGTH:297
SEQUENCE TYPE:nucleic acid
TOPOLOGY:linear
CLONE:HUMGS07696
SEQUENCE DESCRIPTION:
GATCCTATTT AACATGACAA GTAAAATGCT TGCCATAGGA TATTTATATC TAGAACTTTG 60
ACAGACATAC CTAATCATTG TCACTCAGGC ATCCTGCATA TCATTCATTT ATTCATTTGC 120
CTATCATTTG TTGANCATTT AAAATGTCCT AGATACTTAG NATATGANTG TTGAATGTCA 180
CAAAACTGAA TGTCACACTC CCTCCCCTTT AGANATTTNC AGCATGGGAT GCGAGCTAAT 240
GATATGTAAA TAANTACATA TTTNTAANTA ACACAACCNN AAAANAANAN AAACNAN 297

SEQ ID NO:6527
SEQUENCE LENGTH:137
SEQUENCE TYPE:nucleic acid
TOPOLOGY:linear
CLONE:HUMGS07697
SEQUENCE DESCRIPTION:
GATCGTGGCC AACGTGGACC TGCCTACGNA GGGTGGGCTC TGACCCAAGT GGGGCCTCCT 60
TGTCCAGGTC TCACTGCTTT NCACCGTGGT CAGNGGGACT NTCAGCTGAG CTTNAGCTCC 120
CATGGAGCCA GCAGGNN 137

SEQ ID NO:6528

SEQUENCE LENGTH:293
SEQUENCE TYPE:nucleic acid
TOPOLOGY:linear
CLONE:HUMGS07698
SEQUENCE DESCRIPTION:
GATCAGCCTG AGAAGCCACT CAACTTGGCT CACATCGTGC CTCCCAGACC TGTAACCAGC 60
ATGAACGACA CCCNTGTTCT CCCACTCTGT TCCCTCCTCA GGAAGTCCTT TTATTACCAG 120
GAGCTCGGAC GGTGCTTTTG GTGGNTGCGT CTAGATGGAT AACATGACTT CTTCTACCCT 180
AAAATATTCC TATAATACTT TNAGCTGTTC TGGTTCCTCC AGGGTGNATG GTACCNATTA 240
ACCCAAAATA CTGNTTATNT ACCCNTTNTT ACNATNTANN AGTCANTTTT GGN 293

SEQ ID NO:6529
SEQUENCE LENGTH:345
SEQUENCE TYPE:nucleic acid
TOPOLOGY:linear
CLONE:HUMGS07699
SEQUENCE DESCRIPTION:
GATCCACGGG GCCAGGCCAC CCTGCGGGAG CGCCACACGC ATCCACTTCG GATTCAGTGG 60
GTGAAGACAG AACGCTNAGA GTCTGCAGGC GGCTCCTGTG CTTTTAATTT CTGGCTCTTC 120
GGATGTCTTC TAGACATTTA CTATCACTGC ACCTGAAGAA AAAATCACTT TTACCTTCCT 180
AATTTAAAAA GACAAAACAG AAATGTACGT TCCTTCGCTA GCTTTAGTCT TTCTGTTCCC 240
ATTTTNATAA ANTCTGAGGC ATTGATAATG NTCNNATCTA AAATTTGTAC AGTGTGATTT 300
CNNTNNTAAG NGTTAAANCT TTTANTAAAA GGGGCANGAN NTNAN 345

SEQ ID NO:6530
SEQUENCE LENGTH:266
SEQUENCE TYPE:nucleic acid
TOPOLOGY:linear
CLONE:HUMGS07700
SEQUENCE DESCRIPTION:
GATCCAGAAA ATAGGTACGG TTTTAAAATA TTTNATATAG AAAAGCTACA AAGTAAATTG 60
AGCAATGCTT TTAAAGTTAT CTTTGTTTTA TAGACTTTTT TGTTGTATGT ATTACAGTCT 120
TTATAATCTT ATTTAATGNA TATNTGTACT TTCAAGTACT GATGGAGATA GNCTCAAAAC 180
AGTTATTTTN TTACAATTAA TCTACAANGG GCATTANTAT TGTNGNCTTT TAAAACATCT 240
GCTGGNTATC TNATNTGCAA TTCNTN 266

SEQ ID NO:6531
SEQUENCE LENGTH:43
SEQUENCE TYPE:nucleic acid
TOPOLOGY:linear
CLONE:HUMGS07701
SEQUENCE DESCRIPTION:
GATCCCACAC ACTAAATATA TGGTTGTTTT GCCACCTTTT AAA 43

SEQ ID NO:6532

SEQUENCE LENGTH:285
SEQUENCE TYPE:nucleic acid
TOPOLOGY:linear
CLONE:HUMGS07702
SEQUENCE DESCRIPTION:
GATCTGTTTT TCACCACTTC ACTGAAAGAC ACCATTTATC TACCCAAGGG CAGAAAGTAG 60
AACTTACTAT TCATTAAATG TTTGACACAA TTGGAATTGT CTTTAATTTC TGTCAGAATG 120
CTATTGAAAA TGTGAATTGC ATGACTTGTA GCATATTCTT TTCTGCAAAA TAGCCATATT 180
ANCATGCTTA TGACAATGAC TGTGCTACTG TCTNTGGAAA ANTGTTTGTC TCAGTTGGNA 240
ATNCTAANNG NTTCACCTGC GGANNAAACG CCCCNNNCCN CCCAN 285

SEQ ID NO:6533
SEQUENCE LENGTH:43
SEQUENCE TYPE:nucleic acid
TOPOLOGY:linear
CLONE:HUMGS07703
SEQUENCE DESCRIPTION:
GATCCTCTTG TGTATATGCA AATAAACCTA TTATTCACTT AAA 43

SEQ ID NO:6534
SEQUENCE LENGTH:194
SEQUENCE TYPE:nucleic acid
TOPOLOGY:linear
CLONE:HUMGS07704
SEQUENCE DESCRIPTION:
GATCAAGACA CCTTTTATTA ATAAAGAAGN GACACAGGTG TAGATATGTA TATACAAAAA 60
GATGTACGGT CTGGCCAAAC CACCTTCCCA GCCTTTATGC AAAAAAAGGG GAGANTCAAA 120
GCTTTCATTT CAGAAGTGTT GCGTGGANAA GTATCTGTAA TTAAAGTTTC GANGTAATTT 180
AACCTATTTT TAAA 194

SEQ ID NO:6535
SEQUENCE LENGTH:45
SEQUENCE TYPE:nucleic acid
TOPOLOGY:linear
CLONE:HUMGS07705
SEQUENCE DESCRIPTION:
GATCAAGCCC TGCTTAAAGT TAAATAAAAT AGAATGNATG ATAAA 45

SEQ ID NO:6536
SEQUENCE LENGTH:38
SEQUENCE TYPE:nucleic acid
TOPOLOGY:linear
CLONE:HUMGS07708
SEQUENCE DESCRIPTION:
GATCTTCTGG ATGACCTGAA ATNAGAACTA ATTGGAAA 38

SEQ ID NO:6537
SEQUENCE LENGTH:52
SEQUENCE TYPE:nucleic acid
TOPOLOGY:linear
CLONE:HUMGS07709
SEQUENCE DESCRIPTION:
GATCGCCCAA CTGCACTCCA GCCTGGNAGC AAGACTCTGT CTCGAAAAGA AA 52

SEQ ID NO:6538
SEQUENCE LENGTH:55
SEQUENCE TYPE:nucleic acid
TOPOLOGY:linear
CLONE:HUMGS07710
SEQUENCE DESCRIPTION:
GATCAGNACC ACTGCACTCC AGCCTGGGCA ATCANAGTAC GACTCCTGGC TCAAA 55

SEQ ID NO:6539
SEQUENCE LENGTH:252
SEQUENCE TYPE:nucleic acid
TOPOLOGY:linear
CLONE:HUMGS07711
SEQUENCE DESCRIPTION:
GATCAGTCTG AAGTCGATTA CTAACAGCTG AAGCTGATAA AACCATTAAA GTATACAGAG 60
AGGATGACAC AGCCACAGAA GAAACTCATC CAGTCAGCTG GAAACCAGAA ATTATCAAGA 120
GAAAGAGATT TTAATGAATG TGGAATTTTT TCTCTCTCTT TTTTTTTCTT TTTAATTAAA 180
AAAAAAAAGG CTTGGGGTTC ATGGGGTTTT CCNGTCATTT NGTGCNCTGG CTGGGATTTT 240
AAGGGGGAAT TN 252

SEQ ID NO:6540
SEQUENCE LENGTH:48
SEQUENCE TYPE:nucleic acid
TOPOLOGY:linear
CLONE:HUMGS07712
SEQUENCE DESCRIPTION:
GATCGAGNCA CTACACTCCA GNCCGCCGAA GCAAGACTCT GTCTCAAA 48

SEQ ID NO:6541
SEQUENCE LENGTH:235
SEQUENCE TYPE:nucleic acid
TOPOLOGY:linear
CLONE:HUMGS07713
SEQUENCE DESCRIPTION:
GATCANACAT TTNCTACCTA TGAATNTTGC TGCATACAGA AAGTGCCCTT TCCTCAGGAA 60
GTTGCTGTGT NTCATTTCTT TAGATGGNCT CTTATCTAGA ATACATAGCA GCTCTGCAAA 120

GAANCAGTTT TTAAAAATGG GAACTTCTAC ATTGAAAAGT CCCCNTTTTT GTGCCAACTA 180
TGATTAGTGN GCGGAAGNAN TCTTATTCTA TGGCATATGT ATGGANGGGG GGGGN 235

SEQ ID NO:6542
SEQUENCE LENGTH:36
SEQUENCE TYPE:nucleic acid
TOPOLOGY:linear
CLONE:HUMGS07714
SEQUENCE DESCRIPTION:
GATCCTTTTA AATTTAAAGC AAGTCCTAAA ATGAAA 36

SEQ ID NO:6543
SEQUENCE LENGTH:155
SEQUENCE TYPE:nucleic acid
TOPOLOGY:linear
CLONE:HUMGS07715
SEQUENCE DESCRIPTION:
GATCCAGCGN GCGCACGCAG AGTACCTGCA CAAGGCCGTC TTCAGGGGCC TNCTCACGGA 60
GAAGNCGGCG CCCGTNATGA ACGTCATCCA CAGCATCTTC AGCCTCGTGC TCAAGTTCCG 120
CAGCCAGCTN ATCTCCCAGG CCTGGGGGCC CCNNN 155

SEQ ID NO:6544
SEQUENCE LENGTH:110
SEQUENCE TYPE:nucleic acid
TOPOLOGY:linear
CLONE:HUMGS07716
SEQUENCE DESCRIPTION:
GATCAAATAT CACTCTCCTA CTTACAGGAC TCAACATACT AGTCACAGCC CCTATAACTC 60
CCNCTTACAA TANNTANCCA CAACCAAAAT GGGGCTCANT CACCCAACCN 110

SEQ ID NO:6545
SEQUENCE LENGTH:192
SEQUENCE TYPE:nucleic acid
TOPOLOGY:linear
CLONE:HUMGS07717
SEQUENCE DESCRIPTION:
GATCTGGTTG TNTAGAAGCA TGCAGCACCT CCTGCTTCAC TCTCTCTGTC TCTCCTGCTC 60
CACCATGGCC AGAAACGTGC CTGCTTCCCC TTCGCCTTCT GCCGTGATTG TAAGTTTCTT 120
GAGGGCTCCC CAGCCATGCT TCCTGTACAG CCTGCAAAAC TGTGAGTCAA TTAAACCTCT 180
TTTNTTCATA AA 192

SEQ ID NO:6546
SEQUENCE LENGTH:73
SEQUENCE TYPE:nucleic acid
TOPOLOGY:linear

CLONE:HUMGS07718
SEQUENCE DESCRIPTION:
GATCATACTG GTTTGTTTCT TATTTTGATT GGAGAAAAAT NAAATTGCTG CATTTCGCAG 60
TGACCCATTT AAA 73

SEQ ID NO:6547
SEQUENCE LENGTH:80
SEQUENCE TYPE:nucleic acid
TOPOLOGY:linear
CLONE:HUMGS07719
SEQUENCE DESCRIPTION:
GATCAAATGT GTGGCCTTTG ACTGAAATCA GCCAGCCCAT GGCCCCCAAT AAAGGCAGCT 60
GCCTACTNCT CCNTCTGAAA 80

SEQ ID NO:6548
SEQUENCE LENGTH:115
SEQUENCE TYPE:nucleic acid
TOPOLOGY:linear
CLONE:HUMGS07720
SEQUENCE DESCRIPTION:
GATCCANATG CGTGTGGCAG CCNAAGGATT TGTTGTAGGA GCAATGNCTG TTGGTATGGG 60
CTATTCCATG TATCGGGAAT TCTGGGNAAA ANCTAAGCCT TAGAAGAAGA GATGN 115

SEQ ID NO:6549
SEQUENCE LENGTH:98
SEQUENCE TYPE:nucleic acid
TOPOLOGY:linear
CLONE:HUMGS07721
SEQUENCE DESCRIPTION:
GATCATAGCA TATCTACTAT TTTATTATTT GATTTTTAAA ATTTAACATT ATATTATGGG 60
TAACCTTACA TGTCAATAAA CAATTCCACA TTGTCAAA 98

SEQ ID NO:6550
SEQUENCE LENGTH:33
SEQUENCE TYPE:nucleic acid
TOPOLOGY:linear
CLONE:HUMGS07722
SEQUENCE DESCRIPTION:
GATCAACTTT GGAATAAAGT GATGCCCTTC AAA 33

SEQ ID NO:6551
SEQUENCE LENGTH:42
SEQUENCE TYPE:nucleic acid
TOPOLOGY:linear
CLONE:HUMGS07723

SEQUENCE DESCRIPTION:
GATCCACTGT AATATTTAAT AAAAAATGTT ACTATCTGTA AA 42

SEQ ID NO:6552
SEQUENCE LENGTH:24
SEQUENCE TYPE:nucleic acid
TOPOLOGY:linear
CLONE:HUMGS07724
SEQUENCE DESCRIPTION:
GATCAAATAT CACTCTCCTA CAAA 24

SEQ ID NO:6553
SEQUENCE LENGTH:141
SEQUENCE TYPE:nucleic acid
TOPOLOGY:linear
CLONE:HUMGS07725
SEQUENCE DESCRIPTION:
GATCACGACC CTNTCACGTG CACCCANTTA GNGTTGTGAG CCCTTAAAAG GACCAGGGAT 60
TGCTCACTCG GGGAGCTCGG CTCTTGAGAC AGGAATCTTG CCCATTCCCC GAACGAATAA 120
ACCCCTTCCT TAACTCAGAA A 141

SEQ ID NO:6554
SEQUENCE LENGTH:226
SEQUENCE TYPE:nucleic acid
TOPOLOGY:linear
CLONE:HUMGS07726
SEQUENCE DESCRIPTION:
GATCAGGCAG CAGGAGTGAG GGGCACAGTC ACCCCAGGCC TTGCTGAGCC CAGGTCTGGG 60
TACTGAGTGT CCAGAGCTGC CTCCCCAGGA GGTTAAGGTG GGGGCAAAGG GGAAGCTTCA 120
AGCACTTTGC CTACTTTTGT TCACTNCCCA GTGCACTGTG ACTCAGGCCT TCCCATCAGG 180
CCTATTTGTC TACCCAATAA AGCGTGTTTT TTCCAGAAAC AAGAAA 226

SEQ ID NO:6555
SEQUENCE LENGTH:46
SEQUENCE TYPE:nucleic acid
TOPOLOGY:linear
CLONE:HUMGS07727
SEQUENCE DESCRIPTION:
GATCAGCCTG GCCATGGTNA AACCCCATAT CTAGTAAAAC TACAAA 46

SEQ ID NO:6556
SEQUENCE LENGTH:306
SEQUENCE TYPE:nucleic acid
TOPOLOGY:linear
CLONE:HUMGS07729

SEQUENCE DESCRIPTION:
GATCCACTAT NAGGGTGGCA TCAGCCTGAA TTGAACATCC TATATCTNAA CATCTGCATA 60
AATTCCAGGT GACCACCAGT GTGCACCACT CACCCATCCC AGTGTATTCA GGCCNNNTGA 120
CCTGACCCAN TTTGGCCCAG AGGACTCTGC TGGTGACCTG AAAAGAGGGA CTACATATTT 180
NAGGATATTG TTGGTCTCAC ATGACTCTTG ATAGAGTTTG CCAGCCTTCA AATCACCAAC 240
TGTGGGAACC AAGNNGGNNN TTATTGCCTT GCTGTGTTCA ATTACANAAA ACGNTGGAAA 300
NGGCTN 306

SEQ ID NO:6557
SEQUENCE LENGTH:255
SEQUENCE TYPE:nucleic acid
TOPOLOGY:linear
CLONE:HUMGS07731
SEQUENCE DESCRIPTION:
GATCTGAATA CCCCTTCTCT GTAGCTGCTA GTGAGCCTTC CCATTTAGAT TAAAGATTGC 60
TTTATCCAGC AGTCAATTAA CTCTCCAGTT ATCAGTACTC CCACAATTGG CCAGGGCAAC 120
AATAATTGGA GTTCATACTG ATGCCCTGAG GCACTGAAAA AAAAAAAAAT TCCCAAGGNG 180
CCTTNTGGGN TGNCTAAAGG TTNCATTGTG NTTGGNGGCT TTNGTGTTAG GTGTGCNGTT 240
TANTTTCCTA NTTNN 255

SEQ ID NO:6558
SEQUENCE LENGTH:59
SEQUENCE TYPE:nucleic acid
TOPOLOGY:linear
CLONE:HUMGS07734
SEQUENCE DESCRIPTION:
GATCACGNCA CTGCACTCTA GCCTGGGCGA CAGAGAGAGA GACTGTCTAA AAAAGGAAA 59

SEQ ID NO:6559
SEQUENCE LENGTH:69
SEQUENCE TYPE:nucleic acid
TOPOLOGY:linear
CLONE:HUMGS07735
SEQUENCE DESCRIPTION:
GATCCTNAGG TCTCCTAGCC ATGTGGAGCT GTAAGTCCAA TNAAACTTCT TTTTCTTCCC 60
AGTCTCAAA 69

SEQ ID NO:6560
SEQUENCE LENGTH:53
SEQUENCE TYPE:nucleic acid
TOPOLOGY:linear
CLONE:HUMGS07736
SEQUENCE DESCRIPTION:
GATCTGTCAA GTCCAGTAGA GCTTCAAGGT AAAATNAAAA TAACAATGTG AAA 53

SEQ ID NO:6561
SEQUENCE LENGTH:33
SEQUENCE TYPE:nucleic acid
TOPOLOGY:linear
CLONE:HUMGS07738
SEQUENCE DESCRIPTION:
GATCCAGAGG CCATGGAAGA GTCTCCAAGG AAA 33

SEQ ID NO:6562
SEQUENCE LENGTH:158
SEQUENCE TYPE:nucleic acid
TOPOLOGY:linear
CLONE:HUMGS07739
SEQUENCE DESCRIPTION:
GATCAGCTCA AAAAGNTTTG GCTTAGNGTT TTCATTGCAA ATNATAATTG CTGTAGAGCC 60
ACACACAACT TTTGAACTTT TAATTATAAG TGTTATGGCT AAAGTTATTN NCTGAAAATT 120
TCAGTAAAGT GTGTGAATGT TTCTTTATGT ATTANCNN 158

SEQ ID NO:6563
SEQUENCE LENGTH:121
SEQUENCE TYPE:nucleic acid
TOPOLOGY:linear
CLONE:HUMGS07740
SEQUENCE DESCRIPTION:
GATCAGGGTT CTTTTTTTCC CCCATACAAG TATCCAGTCA TTGTAACACT GTTTATTGAA 60
AGAATTATCC TTTCCTCATT AAATTACCTT GCCAATTAGT AAAAAATCAA TTAACCATAA 120
A 121

SEQ ID NO:6564
SEQUENCE LENGTH:87
SEQUENCE TYPE:nucleic acid
TOPOLOGY:linear
CLONE:HUMGS07741
SEQUENCE DESCRIPTION:
GATCAGCATA CACAAATNAC AAAAGTCTGA ATTTTTTTTT ATCAAGAGGG ATAAAACACC 60
ATGAAAATAA ACTTGAATAA ACTGAAA 87

SEQ ID NO:6565
SEQUENCE LENGTH:54
SEQUENCE TYPE:nucleic acid
TOPOLOGY:linear
CLONE:HUMGS07742
SEQUENCE DESCRIPTION:
GATCCNTTTT NCTGTGGCAT TTGGTGCCCT AAAAATTTTG CATTTATCTT GAAA 54

SEQ ID NO:6566
SEQUENCE LENGTH:20
SEQUENCE TYPE:nucleic acid
TOPOLOGY:linear
CLONE:HUMGS07743
SEQUENCE DESCRIPTION:
GATCATTTGA CAACTGCAAA 20

SEQ ID NO:6567
SEQUENCE LENGTH:143
SEQUENCE TYPE:nucleic acid
TOPOLOGY:linear
CLONE:HUMGS07744
SEQUENCE DESCRIPTION:
GATCGACTAT GAGACAGCAA AGGGGCCTAC CTNCTNATGG AGTTAGCCAA GAGCCAGGAC 60
AAGAGTCTCA AGATTTATGA CGGTGCCTAC CATGTTCTCC ACAAGNAGCT TCCTGACGTC 120
ACCANCTCCG TNTTCCNTGA ANN 143

SEQ ID NO:6568
SEQUENCE LENGTH:199
SEQUENCE TYPE:nucleic acid
TOPOLOGY:linear
CLONE:HUMGS07745
SEQUENCE DESCRIPTION:
GATCTGCTGC AATGCTCNAA GGCCAAGGAT TCATCTNTCT TNTCACCGTA GGACCTGNCT 60
GGCATTGTAT TAGCAAACTC TTTCACTAGA CATCGTACTA CACGACACGT ACTACGTNGT 120
AGCTCACTGC CNCTGATGTC CTATCAATAG GAGCTGTATT TNCCATCATA GGAGGCTTCA 180
TTCACTGGTN TCCCCNATN 199

SEQ ID NO:6569
SEQUENCE LENGTH:37
SEQUENCE TYPE:nucleic acid
TOPOLOGY:linear
CLONE:HUMGS07746
SEQUENCE DESCRIPTION:
GATCGTTTCC AGGACAATAA AAGTTTCGGA GTTGAAA 37

SEQ ID NO:6570
SEQUENCE LENGTH:57
SEQUENCE TYPE:nucleic acid
TOPOLOGY:linear
CLONE:HUMGS07748
SEQUENCE DESCRIPTION:
GATCAGATGT TTAAAATNAT TCCAAATACT TCATTAAAAT AATTATTTTA TTATAAA 57

SEQ ID NO:6571
SEQUENCE LENGTH:152
SEQUENCE TYPE:nucleic acid
TOPOLOGY:linear
CLONE:HUMGS07749
SEQUENCE DESCRIPTION:
GATCGAGGAA AGGGGCGGAG AATTTTTTTT TCTTCTTCCC ATTTCTTTAA AAAACCAACA 60
CAAGAAAACA CACACACACA CAACCAACGT TTGTTCCCGA GTAGAAACAT AAATAGGGCA 120
GAATCGAACA CTCTGTTCTT CTCTCTTCCA AA 152

SEQ ID NO:6572
SEQUENCE LENGTH:22
SEQUENCE TYPE:nucleic acid
TOPOLOGY:linear
CLONE:HUMGS07750
SEQUENCE DESCRIPTION:
GATCAGAGCT TGAAAAAACA AA 22

SEQ ID NO:6573
SEQUENCE LENGTH:111
SEQUENCE TYPE:nucleic acid
TOPOLOGY:linear
CLONE:HUMGS07751
SEQUENCE DESCRIPTION:
GATCAACCTG TGGCTGTTTT CCCGTCTAGG TTCTCACAGG NATCTCCTGA CAGAGGTACT 60
TAACAATGGC TCTNCTGGAA ATTTCTATAA ATAAAATGTC CAAAATGGAA A 111

SEQ ID NO:6574
SEQUENCE LENGTH:44
SEQUENCE TYPE:nucleic acid
TOPOLOGY:linear
CLONE:HUMGS07752
SEQUENCE DESCRIPTION:
GATCTCATCA CTGCTCCTTA TAATAAACCT AATAAAGCAA GAAA 44

SEQ ID NO:6575
SEQUENCE LENGTH:42
SEQUENCE TYPE:nucleic acid
TOPOLOGY:linear
CLONE:HUMGS07753
SEQUENCE DESCRIPTION:
GATCCCGCCA CTGCACTCTA GCCTATGTGA CTCTGTCTCA AA 42

SEQ ID NO:6576
SEQUENCE LENGTH:48

SEQUENCE TYPE:nucleic acid
TOPOLOGY:linear
CLONE:HUMGS07754
SEQUENCE DESCRIPTION:
GATCATTTGT AAGACCAAAA TATAAATAAA AAGTTTCAAA AATCTAAA 48

SEQ ID NO:6577
SEQUENCE LENGTH:46
SEQUENCE TYPE:nucleic acid
TOPOLOGY:linear
CLONE:HUMGS07755
SEQUENCE DESCRIPTION:
GATCGATTAC AATAAATGTT TTTCTAAAAA GCCATTTTCC CACAAA 46

SEQ ID NO:6578
SEQUENCE LENGTH:125
SEQUENCE TYPE:nucleic acid
TOPOLOGY:linear
CLONE:HUMGS07756
SEQUENCE DESCRIPTION:
GATCAAAAGC TTTGCATTGA TAAGTGTCTC ATAATATTTG CTGTGATTGG AGAAAAAATG 60
TAGTCGTAGC CAATAAATTT TATCAGCTTT TAAGTTTCAG TATTATTAAA CCATTTTCAT 120
ATAAA 125

SEQ ID NO:6579
SEQUENCE LENGTH:36
SEQUENCE TYPE:nucleic acid
TOPOLOGY:linear
CLONE:HUMGS07757
SEQUENCE DESCRIPTION:
GATCAAATGT GAAATAAATA TGAATGGAGT GGTAAA 36

SEQ ID NO:6580
SEQUENCE LENGTH:199
SEQUENCE TYPE:nucleic acid
TOPOLOGY:linear
CLONE:HUMGS07758
SEQUENCE DESCRIPTION:
GATCTCTGGC CCTTTGCGTT TCAGTATTTG TCCTTTTNAT GCCTGAATGT TTAACCTGCC 60
ACATNTTTGT ACTGCAACAT GAATCTTGGA AATTTTAATG TTATGCATTT NAAANGTTTT 120
TGGTTATCTG TAAACTAAAT GTGCCCTTAT TGGCTNGGNG GTCAAGTNAA GATGTTTGTA 180
TAATGTATTN GTCAGNAAA 199

SEQ ID NO:6581
SEQUENCE LENGTH:66

SEQUENCE TYPE:nucleic acid
TOPOLOGY:linear
CLONE:HUMGS07759
SEQUENCE DESCRIPTION:
GATCTACTGT GATGTTGTCT TCAAAGGCAG GNGAAAATAA TGTTCACAAT AAAATGTGCT 60
AACAAA 66

SEQ ID NO:6582
SEQUENCE LENGTH:135
SEQUENCE TYPE:nucleic acid
TOPOLOGY:linear
CLONE:HUMGS07760
SEQUENCE DESCRIPTION:
GATCTGTAAA GAATGACTTT CAAGGATAGT TCTTAGGGGA CTGTGAAAGT GTTGGGTCTT 60
ACTCNAGGAT GCCTGCATGG GACCCNACCC GGAGCTGGTG TGGCCATTCC CCAAGTGCCA 120
CTGGNCCATG GNNNN 135

SEQ ID NO:6583
SEQUENCE LENGTH:130
SEQUENCE TYPE:nucleic acid
TOPOLOGY:linear
CLONE:HUMGS07761
SEQUENCE DESCRIPTION:
GATCAGCCTG GCCAACATGA TGAGACCCCA TTTGTACTGG AAATGCAAAA ATTGGCCAGG 60
CATGGTCGCG GGTGNCTGTG GTCCCAGCTG CTCGGGACGC TGAGGCAGGN NNNATCGTTT 120
GAACCTNGGN 130

SEQ ID NO:6584
SEQUENCE LENGTH:80
SEQUENCE TYPE:nucleic acid
TOPOLOGY:linear
CLONE:HUMGS07762
SEQUENCE DESCRIPTION:
GATCAAATGA AAGGCCTATA ACTAAAATCA GCCAGCCCAT GGCCCNCACT AAAGGAACCT 60
NCCTCTGCTC CCTNTGCAAA 80

SEQ ID NO:6585
SEQUENCE LENGTH:48
SEQUENCE TYPE:nucleic acid
TOPOLOGY:linear
CLONE:HUMGS07763
SEQUENCE DESCRIPTION:
GATCAACAAT TTTTGTTTTG ATTAAAATNA GTTTTTTGAA AGTTGAAA 48

SEQ ID NO:6586

SEQUENCE LENGTH:178
SEQUENCE TYPE:nucleic acid
TOPOLOGY:linear
CLONE:HUMGS07764
SEQUENCE DESCRIPTION:
GATCCCCTGC TGGCTGGGGG CAGCTCCCAG GATATACTGC CTTACAACTG TTTCTAAAGC 60
CCATCCTNCT AACATGGCGA TTCCGGAGGT CAAGGCCTTG GGCTCTNCCC AGGGTCTAAC 120
GGNTAAGGGG GCCACATACC NGTTCCAAGG GGNNTGTCAA GTNGTGAAGT CGTTNTGN 178

SEQ ID NO:6587
SEQUENCE LENGTH:99
SEQUENCE TYPE:nucleic acid
TOPOLOGY:linear
CLONE:HUMGS07765
SEQUENCE DESCRIPTION:
GATCAGATGG TTTATAAAAG TAATAACCAT AAAGCAAAAA ATAATTTGAA AGCCCGTCTA 60
TTCCTATGCT CANTAAAGTT AAGGTTTTTC TTCATTAAA 99

SEQ ID NO:6588
SEQUENCE LENGTH:65
SEQUENCE TYPE:nucleic acid
TOPOLOGY:linear
CLONE:HUMGS07766
SEQUENCE DESCRIPTION:
GATCCTTTGA GCCCAGGAGT TTGAGACCAG CTTGGGCAAC ATAGTGAGAC CCTGTCTCTA 60
CCAAA 65

SEQ ID NO:6589
SEQUENCE LENGTH:205
SEQUENCE TYPE:nucleic acid
TOPOLOGY:linear
CLONE:HUMGS07768
SEQUENCE DESCRIPTION:
GATCACAGGG TCAGGAGTTC AAGACCACCC TGGACAATAT TGTGAATTCC TGCCTCTACT 60
AAAAATACAA AAATTAGTCG GGCATGGTGG CGGGTGCCTG TGGTCCCAGC TACTCGGGAG 120
GCTGAGGCCA GAAAATCGGT TGAACCTGGG AGGTGGAAGG TGCACTGAGC CAATATCACA 180
CCAGTGCACT CCAACCTGGT GACGN 205

SEQ ID NO:6590
SEQUENCE LENGTH:57
SEQUENCE TYPE:nucleic acid
TOPOLOGY:linear
CLONE:HUMGS07769
SEQUENCE DESCRIPTION:
GATCTCATAC TCAACAGAAG GAGACTCAGA GACTCCAGAA GGCACAGGAG CTGCCCN 57

SEQ ID NO:6591
SEQUENCE LENGTH:184
SEQUENCE TYPE:nucleic acid
TOPOLOGY:linear
CLONE:HUMGS07770
SEQUENCE DESCRIPTION:
GATCATGTTT ATTTACTGAT AAAACTCAAT ACCTAGAACT GCACCTAGCT CATGATGGTA 60
CCTAATAAAT ACTTCAATGA AATGATGTGG TGATGCATGC CTATAGTTTT AGCTACTTGG 120
GAGGGTGGCC TGAGCCCAGG GGTTCTAGAC CAGCTTAGGC AACATAGCAA GACCCCATCT 180
CAAA 184

SEQ ID NO:6592
SEQUENCE LENGTH:165
SEQUENCE TYPE:nucleic acid
TOPOLOGY:linear
CLONE:HUMGS07771
SEQUENCE DESCRIPTION:
GATCTGAATG GTTTTAATAA GTGCCTGGNA TTTCCCCTAC TGGCTCTCAT NCTNACTCTT 60
GCCGCCCTGT GAAGAGGTGC CTTCCACCGT GATTGTNAAG TTTCCTGAGG CCTTCCCAGC 120
CATGTGGAAC TGTGAGTCGA AAATNAAACC TCTTTTATAA TTAAA 165

SEQ ID NO:6593
SEQUENCE LENGTH:134
SEQUENCE TYPE:nucleic acid
TOPOLOGY:linear
CLONE:HUMGS07773
SEQUENCE DESCRIPTION:
GATCCAAGCT TAATTTTCAA ACAGCTTCCC AAAAAGCTGT ATCCTGAATA TCATAAAGTC 60
TATCATATGA TGTGTACTCA GTCATATTCT GTAGACTCAA ATCTTTTATC TGATTATACC 120
AGCTTTCCCG NAAA 134

SEQ ID NO:6594
SEQUENCE LENGTH:148
SEQUENCE TYPE:nucleic acid
TOPOLOGY:linear
CLONE:HUMGS07774
SEQUENCE DESCRIPTION:
GATCTGCCCA CGTTGGCCTC CAAAGTGCTA GGATTATAAG TGTGAGCCAC CACACCAGGC 60
CCGTTTCATC CCTGNCTCAG CCTGCATGCC AAGCTCCCTN GGTGCCCGNC CACAGGTGTC 120
ACCCTTTNAG GAGAANGTGG CCTGGGGN 148

SEQ ID NO:6595
SEQUENCE LENGTH:238
SEQUENCE TYPE:nucleic acid

TOPOLOGY:linear
CLONE:HUMGS07775
SEQUENCE DESCRIPTION:
GATCGCTTGT AGCTGGGAAC AGTGCTTACT TCAGGGAAAA CTGATTTTGA TAGAACCGAC 60
TAGTTGAGTA TTAAACTGTT TTGGAAATAA TTTATATATC TAAGTGCTGA AATATATTTC 120
TCTTCTCCCC ACTTATTCAA ATAAAAANGT TTTNATACTA TAAACTTTTC NNNGTTAGCG 180
CCACGCAGTC CTTGTCAAGN TTGTNCTGGG GGNCAANAAC TTGATGTATA AAGCATGN 238

SEQ ID NO:6596
SEQUENCE LENGTH:102
SEQUENCE TYPE:nucleic acid
TOPOLOGY:linear
CLONE:HUMGS07776
SEQUENCE DESCRIPTION:
GATCTCTACT ACCANAAGGA AAATAGTTTA GGNGAAACCA GCTTTTANTG TTTTTGTAAA 60
ATTAGCTTCA CCCTGTNATG TTANCAAGGT ATGTCTTTGC CN 102

SEQ ID NO:6597
SEQUENCE LENGTH:65
SEQUENCE TYPE:nucleic acid
TOPOLOGY:linear
CLONE:HUMGS07777
SEQUENCE DESCRIPTION:
GATCGCGCCA CTGCACTCTA GCCTGGGTGA CCAGCCTGGG TGACAGAGTG AGNCTCCGTC 60
TCAAA 65

SEQ ID NO:6598
SEQUENCE LENGTH:282
SEQUENCE TYPE:nucleic acid
TOPOLOGY:linear
CLONE:HUMGS07778
SEQUENCE DESCRIPTION:
GATCTAGAAG AAAGTATTGT ACGTTTGAAT GCAGATTTTN ATCCACAGAT AGTTGTAGTG 60
TTTAGACATG ACAGGACCTA TCGTTGAGGT TTCTAAGACT TACTATGGGC TGTAAACCTG 120
TTTTTNAAAA CTATTTTAGA AACCTGAGAC TTGCCGTCTG GCATTTNAGT TTAATACAAA 180
CTAATGATTG CATTTGNAAG NGNTNCTTGA CCTTATTTCT AAACGTCTAG NGCTCTGAAA 240
TGTCTTGCTG GNNGGTATTA AACTATTTGC CTGTNGTACA TN 282

SEQ ID NO:6599
SEQUENCE LENGTH:173
SEQUENCE TYPE:nucleic acid
TOPOLOGY:linear
CLONE:HUMGS07779
SEQUENCE DESCRIPTION:
GATCAAATGG CTTAGAGNTA GTCCTGGAAT ATTCATATTC AAAGATTCCT TATTAATGAA 60

TGTCTTTAAC TTAAATCTAC CCAATAATTG CAACATGGTT CTTTGTACAT TTTCATTATA 120
TGGTGTTAAC AAGCTTCACT GCAAACAAAT AAATTACTTA AGTTATTTGN AAA 173

SEQ ID NO:6600
SEQUENCE LENGTH:263
SEQUENCE TYPE:nucleic acid
TOPOLOGY:linear
CLONE:HUMGS07780
SEQUENCE DESCRIPTION:
GATCAGTGTA GCAAATNAGG GATGAAGNGT TCAAACCTTT TGGCCCTTTC TTTCTTTNCT 60
AGGCTTCTCC CTCGCAGGGT GTTCCGTAGT TTCTTCTCGA GCCAATGCAT GTATTATAGC 120
AGCAGGTGTC TTTGTGCTTT CTCATCATAG TAACGTACTA CTTGTAAATA CATTTTNCTA 180
TTTNCTATTT TTTTGTATTT TTTTTTGACA TTTTGTTTCA TTGGTGTGCT GNGNGNNNCC 240
CATGCCCTCA CTCCTTTAAG AAA 263

SEQ ID NO:6601
SEQUENCE LENGTH:51
SEQUENCE TYPE:nucleic acid
TOPOLOGY:linear
CLONE:HUMGS07781
SEQUENCE DESCRIPTION:
GATCCAAAAT TCTCAACAAA ATATTAGCAA ATTGAACCCA CCAAGGTTAA A 51

SEQ ID NO:6602
SEQUENCE LENGTH:283
SEQUENCE TYPE:nucleic acid
TOPOLOGY:linear
CLONE:HUMGS07782
SEQUENCE DESCRIPTION:
GATCAAAGGC CTCTNAGGGG TGTCCACCAA AAACTTCTCC TTCAAAAGAG AAGACTCCGT 60
GCTTCAGGGC TATGACATCA ACAGCCAAGG GGAAGAGTCC ATGGGAAATG CAGAGCCCCT 120
TAGGAAGCCN ATCAAAAACC GGNGCNTANN GTTAANGAAA GTGAACTCCC AGGAAATACA 180
CATGCTCCCA ATCAAAAAAC AACGGCTGGC CACCTTTTTT CCAAGAAAGT AAATAACGGC 240
TTTTTAAANT NNGTATGNTT ATAATATGGG GAAAGGTGCA TTN 283

SEQ ID NO:6603
SEQUENCE LENGTH:223
SEQUENCE TYPE:nucleic acid
TOPOLOGY:linear
CLONE:HUMGS07783
SEQUENCE DESCRIPTION:
GATCGTTCAT CCGATTGCNA CGTTCCAAAG TGTCCAGGTT TTTAAGACCT TACAAATGAC 60
TCAGCCCACG TGCCACTCAA TACAAATNTT CTGCTATAGG GTTGGTGGGG CAGAGCTGTC 120
TTCCTTCTGC ATGTCAGCAC AGTCGGGTAT TGCTGCCTCC CGTATCAGTG ACTCATTAGA 180
GTTCAATNNN NATAGATAAT ACAGATATTT TGGTAAATTG AAA 223

SEQ ID NO:6604
SEQUENCE LENGTH:100
SEQUENCE TYPE:nucleic acid
TOPOLOGY:linear
CLONE:HUMGS07785
SEQUENCE DESCRIPTION:
GATCACCTGA GCCTGAGAGT TCCAGACCAN CCTGGGCAAC ATAGCGAAAC TNCATCTCTT 60
TAAAAATAAA ATAAAATTAA AATTAATACA AATAATTAAA 100

SEQ ID NO:6605
SEQUENCE LENGTH:69
SEQUENCE TYPE:nucleic acid
TOPOLOGY:linear
CLONE:HUMGS07786
SEQUENCE DESCRIPTION:
GATCCCTTCC ACTCAGGATT CTCGCCGCCT TTTCTAAGAA AATAATAAAA AAAAAATNCT 60
TGTTTCAAA 69

SEQ ID NO:6606
SEQUENCE LENGTH:164
SEQUENCE TYPE:nucleic acid
TOPOLOGY:linear
CLONE:HUMGS07787
SEQUENCE DESCRIPTION:
GATCTGCCTG CCTCGGCCTC CCAAAGCGCT GGGATTACAG GCGTGAACCA CTGCGCCCAA 60
CCTTTGGATT ATTTTTTGTA GAGATGGGGC TTCCCTATGT TGGCNAGGCT GGTCTCAAAC 120
TCCTGGANTC CAGCCTGGGC GGCAGAGCAA AACCTTGTCT CAAA 164

SEQ ID NO:6607
SEQUENCE LENGTH:73
SEQUENCE TYPE:nucleic acid
TOPOLOGY:linear
CLONE:HUMGS07788
SEQUENCE DESCRIPTION:
GATCACTCCT CTGCTTCAGC CTGGGCAATT GAGCCAGACT CTGTCTCAAA AATAAACAAA 60
AAAACTTGAC AAA 73

SEQ ID NO:6608
SEQUENCE LENGTH:74
SEQUENCE TYPE:nucleic acid
TOPOLOGY:linear
CLONE:HUMGS07789
SEQUENCE DESCRIPTION:
GATCTTTTTA CCTGGATATG TCTGTAAGGC TCCTGAAAGA GACAAATAAA GTCAATATAT 60

TTNCACAGTG CAAA 74

SEQ ID NO:6609
SEQUENCE LENGTH:58
SEQUENCE TYPE:nucleic acid
TOPOLOGY:linear
CLONE:HUMGS07790
SEQUENCE DESCRIPTION:
GATCTTAGGT CAAATAACAG ACTTGAGAAT ACTTTATAAA CTGCCATGAT ACAGCAAA 58

SEQ ID NO:6610
SEQUENCE LENGTH:139
SEQUENCE TYPE:nucleic acid
TOPOLOGY:linear
CLONE:HUMGS07792
SEQUENCE DESCRIPTION:
GATCGNGNGC GTTTAACGCT GGTGGGCAGC AATAAGGGGC AGATGTGACC AGATGCCTGC 60
NTCCCCAGGG TGCCGAGGGC AGCAGGAAAA AGTGGGGACC TCGGTGCATT TGCCCCACCC 120
NTCCCCTNNC TGGGCTAAN 139

SEQ ID NO:6611
SEQUENCE LENGTH:75
SEQUENCE TYPE:nucleic acid
TOPOLOGY:linear
CLONE:HUMGS07793
SEQUENCE DESCRIPTION:
GATCATCGAC ACTTCGAACG CACTTGCGGC CCCGGGTTCC TCCCGGGGCT ACGCCTGTNT 60
NAGCGTCGCT TGAAA 75

SEQ ID NO:6612
SEQUENCE LENGTH:171
SEQUENCE TYPE:nucleic acid
TOPOLOGY:linear
CLONE:HUMGS07794
SEQUENCE DESCRIPTION:
GATCTGGGGG AGGAAGTTCA AGGAAGGCAC CACACCAGAA ATNGGACCTA AATTAAGGCT 60
TAAAGAATGA GCATGGCCAC ACTGTCAGTN AGTGTTCTTT AGGTGGTAGG ACTATGGTTG 120
ANATTTTCNT TCTTCCTATC TTCCTGCCTT TTTCAGGNNN TNAATATTAA N 171

SEQ ID NO:6613
SEQUENCE LENGTH:69
SEQUENCE TYPE:nucleic acid
TOPOLOGY:linear
CLONE:HUMGS07795
SEQUENCE DESCRIPTION:

GATCACGATG TAAATAATTA AGCATTTTGG TTGGATTTCT TTTGTAATAA ACTATTTTTG 60
TACCATAAA 69

SEQ ID NO:6614
SEQUENCE LENGTH:271
SEQUENCE TYPE:nucleic acid
TOPOLOGY:linear
CLONE:HUMGS07796
SEQUENCE DESCRIPTION:
GATCTAGGAT AGCTAAGTNA CCTACATGGT GACTGTTCGG TCTAATTCAG AAACAGAAGT 60
ACTAAAAGAA TACTAAAAAG TTTTTACCCA TTATCTCTAT CCCTGTTTTN TTTTTGCAAT 120
TCTCAGCNGN AGGGAGCAAG AAAATACACA GCAGNGGGGG GAATGTTTGT TTGCATCCTG 180
GCTTTCTCAA CCGCACTTTA CGTCCTTGAA TATNCTAGAT ATTTACTCCT TCAAGAAGTC 240
AGGAGTATAT CCAGGCCTAC CTATCANCCT N 271

SEQ ID NO:6615
SEQUENCE LENGTH:237
SEQUENCE TYPE:nucleic acid
TOPOLOGY:linear
CLONE:HUMGS07797
SEQUENCE DESCRIPTION:
GATCACACCA TGGAGACTTT CTACTAGAGG ACTTGAAAGA GAACTGAGGG GCCACAAAAT 60
AAACTTCACC TTCCATTAAG TGTTCAAGCA TGTCTGCAAA TTAGGAGGGN GTTAGAAACA 120
GTCTTTTTCA TCCTTTGTGA TGAAGACTGA AATTGTGCCG TGTTGCCTTA TATGANTATG 180
CAGTATGGGA CTTTGAAATA ATGATTCATA ATAAAATACT AAACGTGTGT CTTCAAA 237

SEQ ID NO:6616
SEQUENCE LENGTH:196
SEQUENCE TYPE:nucleic acid
TOPOLOGY:linear
CLONE:HUMGS07798
SEQUENCE DESCRIPTION:
GATCTNAAAT AAAATTCAAA CTCTACTTCT GTTGTCTAGT ATGTAATTGA GCTAATGATT 60
CATTGGGAAT ATACCTTTTT ATACTCCTTT ATCATGGTAC TGTAACTGTA TCCATATTAT 120
AAATATAATT ATCTNAAGGN TTTTNAATTT TTNTTNATGT CCAAGNGCCC ACGTGAATTT 180
NCTGGTGAAA GTNAGN 196

SEQ ID NO:6617
SEQUENCE LENGTH:75
SEQUENCE TYPE:nucleic acid
TOPOLOGY:linear
CLONE:HUMGS07800
SEQUENCE DESCRIPTION:
GATCATCGAC ACTTAGAACG TACTCCNGNC CCCGTGCTCC TCCAGAACCT ACGNCTGCAC 60
TAAGCGTCGA TTAAA 75

SEQ ID NO:6618
SEQUENCE LENGTH:50
SEQUENCE TYPE:nucleic acid
TOPOLOGY:linear
CLONE:HUMGS07801
SEQUENCE DESCRIPTION:
GATCACGCCA CTGTACTCCT GGGCAACAAA GCGAGACTCC CTGTCTCAAA 50

SEQ ID NO:6619
SEQUENCE LENGTH:208
SEQUENCE TYPE:nucleic acid
TOPOLOGY:linear
CLONE:HUMGS07802
SEQUENCE DESCRIPTION:
GATCGAGGCT TGTNATGCCT TGTGAGAAAT AAGGGCCTTA ATTTGTACTG TCTGCGGCAT 60
TTTTNATAAT ATTGTATATA GTGACTGACA AATATTGTAT TACTGTACAT AGAGAGACAG 120
GTGGGCATTT TTGGGCTACC TGGTTCGTTT TTATAAGATT TTGCTGGGTT GGTTTTTTTT 180
TTAATTAAAA AGTTTTGCAT CTTTTAAA 208

SEQ ID NO:6620
SEQUENCE LENGTH:151
SEQUENCE TYPE:nucleic acid
TOPOLOGY:linear
CLONE:HUMGS07804
SEQUENCE DESCRIPTION:
GATCAGGAAG CCAAACTTAA GAAAGAGCTT GAAAATACTG CTCTAGAGCT TAGTCAGAAA 60
GAAAAACAGT TTAATGCCAA AATGCTGGAA ATGGCACAGG CTAACTCAGC TGGAATCAGT 120
GATGCAGTGT CAAGACTGGA AACAAACCAA A 151

SEQ ID NO:6621
SEQUENCE LENGTH:33
SEQUENCE TYPE:nucleic acid
TOPOLOGY:linear
CLONE:HUMGS07805
SEQUENCE DESCRIPTION:
GATCTNGGCA ACACAGTCAG ACTCCGTCTG AAA 33

SEQ ID NO:6622
SEQUENCE LENGTH:321
SEQUENCE TYPE:nucleic acid
TOPOLOGY:linear
CLONE:HUMGS07807
SEQUENCE DESCRIPTION:
GATCCGGACG ACGTAATCAC TGACATTNAG ATGGTGGACG AGGGCTGGTG GCGGGGACGT 60

TGCCATGGCC ACTTTGGACT CTTCCCTGCA AATAATGTCA AGCTTCTGGA GTNACTAGAG 120
CTCACTGTCT ACTGCAACTN TAATTTCCCA TGTCCAAAGN GGNTCTGCCT CCACCCCCTC 180
CCTATTCCTG CTGCAAATNT CTAACCAGAT GAGGTTCTGG ACAGACTTCC CTCTCCTGCT 240
TCATTAAGGG CTTGGGGCAG AGNCAGCATG GGGAAGGAGG TCCCCTTCCC CAAGAGTCCT 300
CTTCTATCCC TGGATGAANN N 321

SEQ ID NO:6623
SEQUENCE LENGTH:96
SEQUENCE TYPE:nucleic acid
TOPOLOGY:linear
CLONE:HUMGS07808
SEQUENCE DESCRIPTION:
GATCCCAGCA CCCATTCCTT AGCTATTTAT CCCTTTCCTG GTTTCCGAAA GGCAATTATA 60
TCTATTATGT ATAAGTAAAT ATATTATATA TGGAAA 96

SEQ ID NO:6624
SEQUENCE LENGTH:242
SEQUENCE TYPE:nucleic acid
TOPOLOGY:linear
CLONE:HUMGS07810
SEQUENCE DESCRIPTION:
GATCTAGGCT TCTNGCCTCA CTCCCATTAG GAATAACACC CAGCAAAATG GAACTATCCA 60
AGCACTTCCT ATACATCTGT CTCCCCATTC TCTCTTAAGA AGTTTCTTCA GCTGGTGCGC 120
CATCATCATC ACCATGACTG CCCCCAGCTC TTGAGGAAAG GNATAATTAC CTCCATGGTG 180
TTGCCTCCTA TTTGCCTAAA GCACCAGAAA TAATATTAAA TACACCAGAT TAACTACANA 240
AA 242

SEQ ID NO:6625
SEQUENCE LENGTH:97
SEQUENCE TYPE:nucleic acid
TOPOLOGY:linear
CLONE:HUMGS07811
SEQUENCE DESCRIPTION:
GATCCCGTCC TTGTCAGAGA AGTGTTCCTT GGATGAGCGC CCAGAGGGAG GAGCCCATGG 60
CCCTTTCTTC TGTNACTGAT TAAAAATCTC GATGAAA 97

SEQ ID NO:6626
SEQUENCE LENGTH:98
SEQUENCE TYPE:nucleic acid
TOPOLOGY:linear
CLONE:HUMGS07812
SEQUENCE DESCRIPTION:
GATCCCTTCA AGACTTTGTC ACAGCCTCTA TCACACATCT GTTTTTCTCG AAGNAAAAAA 60
TATAATTAAT AAAAATGTTT TACTCTTTTA CACTGAAA 98

SEQ ID NO:6627
SEQUENCE LENGTH:36
SEQUENCE TYPE:nucleic acid
TOPOLOGY:linear
CLONE:HUMGS07813
SEQUENCE DESCRIPTION:
GATCCCGNTG TTCTTAATAA ATCCTGACTT CCCAAA 36

SEQ ID NO:6628
SEQUENCE LENGTH:35
SEQUENCE TYPE:nucleic acid
TOPOLOGY:linear
CLONE:HUMGS07814
SEQUENCE DESCRIPTION:
GATCAAAAAT AAATACTGCA CATAAAANAA AAAAN 35

SEQ ID NO:6629
SEQUENCE LENGTH:77
SEQUENCE TYPE:nucleic acid
TOPOLOGY:linear
CLONE:HUMGS07815
SEQUENCE DESCRIPTION:
GATCTTGTTT TTATTGTTAT TGCTTTTATT ATNATTGCTT TTATTATCAT TAAAACTCTA 60
GTTCTTGTTT TGTCAAA 77

SEQ ID NO:6630
SEQUENCE LENGTH:98
SEQUENCE TYPE:nucleic acid
TOPOLOGY:linear
CLONE:HUMGS07816
SEQUENCE DESCRIPTION:
GATCCAGTAC AGAAAGGAAA GGNTATTTAT TGATTAACAG AAGTTGTCTT TTTAAAAGTG 60
TTTATTTTTG GCAATAAAGA GCACAACATA ATCTCAAA 98

SEQ ID NO:6631
SEQUENCE LENGTH:132
SEQUENCE TYPE:nucleic acid
TOPOLOGY:linear
CLONE:HUMGS07817
SEQUENCE DESCRIPTION:
GATCAGGGAA CAAAATCCTC TCCTTGTGGA AATATCCCAT GCAGTTTGTT GATACAACTT 60
AGTATCTTAT TGCCTAAAAA AAAATTNNCT TATCNTTGTT TCAAAAAAGC AANNTCATGG 120
ANAATTTTTG TN 132

SEQ ID NO:6632

SEQUENCE LENGTH:304
SEQUENCE TYPE:nucleic acid
TOPOLOGY:linear
CLONE:HUMGS07818
SEQUENCE DESCRIPTION:
GATCAACGGC TGACCTTCTG GCGTCTGGGG CATGGTGAAC CCACCTNCAT GAATAGCACT 60
GTGTTCCATG TGCCTGANGT GGCTGACATG GACTGCTGGC CTGTAAGCCC TGAGTTTGGC 120
CACCGTTGTG CCCTTGGGGG TCAGGGGNTT NAGGTTTACA ACTGGTATGA CTGAGGTATC 180
CTGCGGTGGC TGGCGTGCTG GGCATGGGGC NTGCTCACAG ACAGNATGGA GCAGGGAAGG 240
GCTGTCCTGT GCCCATGCTC AGCTTGNCCT TNNNGNNGAN GGAGGTNGTG GCCGTGGGTN 300
CCTN 304

SEQ ID NO:6633
SEQUENCE LENGTH:35
SEQUENCE TYPE:nucleic acid
TOPOLOGY:linear
CLONE:HUMGS07819
SEQUENCE DESCRIPTION:
GATCTCAAAT AAAATTNCTG TAAAGAATTT CCAAA 35

SEQ ID NO:6634
SEQUENCE LENGTH:185
SEQUENCE TYPE:nucleic acid
TOPOLOGY:linear
CLONE:HUMGS07820
SEQUENCE DESCRIPTION:
GATCCAAATC GGGCCATCTT CATTGCCATG AGTGTTCGGA CAGGCTCTGC GTGCCAGGCA 60
ACAGTGGGCA GACAAAGGGC TCCCTGGCTA CTGGGAAGAT ACAGAACCAT CCTGTGAAAA 120
TCATTTCAGC ACTGGAGACC TTCTTTGCTA TTACTTCTTT CATTAACAAT GACCTCTAAT 180
TTAAA 185

SEQ ID NO:6635
SEQUENCE LENGTH:151
SEQUENCE TYPE:nucleic acid
TOPOLOGY:linear
CLONE:HUMGS07821
SEQUENCE DESCRIPTION:
GATCCGTGGC CTGAAGGTTC CTAGAGNCGT CAAGAAATNA ATATCTTACA CTGTGATTCT 60
GTGAGGAAAG ACTGGTAACC CAAAACTCTC TTCTCTAATG TATTTTTNAA CGNAAATGAC 120
AATATTTCTT TAATAAAGTA TTTATACCAA A 151

SEQ ID NO:6636
SEQUENCE LENGTH:165
SEQUENCE TYPE:nucleic acid
TOPOLOGY:linear

CLONE:HUMGS07822
SEQUENCE DESCRIPTION:
GATCCAGGTC TAGGCCCTTN ATCTGCTGCT CTGTGGCCCA GGGCAGGTTT GCTTGACCTC 60
TGCCTCAGTT CTCGACTCTA AAGGACATAC TGACCTACCT CACAGGGGTG TTGTNAGGAT 120
TAATAAATNT TGGTACTCTG CTTTGGAAAT NTGAAAAAAA AGAAA 165

SEQ ID NO:6637
SEQUENCE LENGTH:71
SEQUENCE TYPE:nucleic acid
TOPOLOGY:linear
CLONE:HUMGS07823
SEQUENCE DESCRIPTION:
GATCAAATTC AGTGTTGTAT GTATGTTTAT GGCAACATTT TGAAAATAAA AATTAAATGT 60
TTTNCTTCAA A 71

SEQ ID NO:6638
SEQUENCE LENGTH:348
SEQUENCE TYPE:nucleic acid
TOPOLOGY:linear
CLONE:HUMGS07824
SEQUENCE DESCRIPTION:
GATCTCAATA CTTCCCTGAC ATCCCTTAAG ACTCAAGTCT TGATACTTTG TCTGCTCTCT 60
GGCTCTTTCT TTGTCATTAC TATNATNNNA ATCTTCTTGA TGTTGACTCT TGCTAAAATT 120
TTTNGTCTTC CTTATTGTTA ATGCTTATAG AAAATGATTC CTTGTTATCT TTNAGCCCAT 180
GTCATCCGAA TCATTTGCTG TTTCTAGCCT AGGTGCTTTG ACTATGCCAG CTTGTATTTC 240
TNCAGAATTT NCATTANCTA TATGTGTGGT ATTGTAGGCA TTACCATATT TNAAAATAAA 300
NTTTAGGCCA TTGCTTCAAA TCACAACAAA NNACAACNCA ACNNACNN 348

SEQ ID NO:6639
SEQUENCE LENGTH:53
SEQUENCE TYPE:nucleic acid
TOPOLOGY:linear
CLONE:HUMGS07826
SEQUENCE DESCRIPTION:
GATCTGAAGA GTGAAAATAA TGCTTTAAAT NAAAAATTGA AATCAGAAGG AAA 53

SEQ ID NO:6640
SEQUENCE LENGTH:127
SEQUENCE TYPE:nucleic acid
TOPOLOGY:linear
CLONE:HUMGS07827
SEQUENCE DESCRIPTION:
GATCTTTTTN AATTCAGTAC AACCCATAAT CATGTAAATN CTCATTTNCT TTAGGNCATA 60
AAGAGAGCCC TAGGGTGCTC TGAATGTGTA CATGTNCTTG TCATAAAATG CATACTGTTG 120
ATACAAA 127

SEQ ID NO:6641
SEQUENCE LENGTH:23
SEQUENCE TYPE:nucleic acid
TOPOLOGY:linear
CLONE:HUMGS07828
SEQUENCE DESCRIPTION:
GATCTGTTGT TACTTAACAG AAA 23

SEQ ID NO:6642
SEQUENCE LENGTH:85
SEQUENCE TYPE:nucleic acid
TOPOLOGY:linear
CLONE:HUMGS07829
SEQUENCE DESCRIPTION:
GATCTTNATG TTAATNTTCA GAGGACTCTA GACAACAACT TAGCAACTGA AGCTTATNAA 60
AGAAGAATTA AGCGCCTTGA GCAAA 85

SEQ ID NO:6643
SEQUENCE LENGTH:113
SEQUENCE TYPE:nucleic acid
TOPOLOGY:linear
CLONE:HUMGS07831
SEQUENCE DESCRIPTION:
GATCTTTTGT GGATATTTGT CACTTGGTTT CAGAAAGTNC ACAAATGTAG CAACAGCTCA 60
CATGACTGAG TAGGTAGAAA ATGTGAAATA AATCTCATAT ATATNGTTTT AAA 113

SEQ ID NO:6644
SEQUENCE LENGTH:54
SEQUENCE TYPE:nucleic acid
TOPOLOGY:linear
CLONE:HUMGS07834
SEQUENCE DESCRIPTION:
GATCGTCCCG CTGCACTCCA GCCTGGCCAA CANAGCGAGA CTGTCCCCCC CAAA 54

SEQ ID NO:6645
SEQUENCE LENGTH:59
SEQUENCE TYPE:nucleic acid
TOPOLOGY:linear
CLONE:HUMGS07835
SEQUENCE DESCRIPTION:
GATCTTGCTC CTCTGTAAGG AACAAGGGTG CCTAATAAAT ACATTNCTCC TTTATTAAA 59

SEQ ID NO:6646
SEQUENCE LENGTH:184

SEQUENCE TYPE:nucleic acid
TOPOLOGY:linear
CLONE:HUMGS07836
SEQUENCE DESCRIPTION:
GATCCAGGGT CCCCCAGGGT GGGCTCAGCT CCAGGGAGAG GCCACCCACG TGGCAGCCCC 60
ACCTCTTGAG AGCCCCCAGT GCCGGAGCAG AAAGGACCCT GGACCCNGAG GCAGNNACTG 120
CGGGGTGGTA GAAAAGGTAG AGTAGGCTGT GGCAATGGAA TAAANCACGA TTAAAAACGT 180
TAAA 184

SEQ ID NO:6647
SEQUENCE LENGTH:166
SEQUENCE TYPE:nucleic acid
TOPOLOGY:linear
CLONE:HUMGS07837
SEQUENCE DESCRIPTION:
GATCCTTCCC CTGCAACCAG ACAGTCTACA ACTGCCCCCT CCAGCCCATT TNCTGCCGTG 60
AAACCCCAGC CAGCCANACC AGGCTCTGGA ACCCTTTTTC GACTAGGNCA ACTCTTGGNC 120
ACCAGGNGGG GTAGAACACC NAACACCAAA CTGTACAGAN TNTCCN 166

SEQ ID NO:6648
SEQUENCE LENGTH:227
SEQUENCE TYPE:nucleic acid
TOPOLOGY:linear
CLONE:HUMGS07838
SEQUENCE DESCRIPTION:
GATCTNTNAT GGTCAGCACC ACCCGCNTAC CCAGTTCCTC CAGTGGAGAC CAGTTGGGTA 60
GTGGTGACTG GGTACGCTAC AAGCTCTGCA TTGTNTGCTG ATGGGACGCT CTTCAAGGTG 120
CAAGTAACCA GCCAGAACAT GGGCTGCCAA ATCTNAGNCA ACCCCGGTGG AAACACTNAT 180
TAAAAGAACC CCAGAGGACA CCAACAGGTG TNTATCTGGN CNTGNCN 227

SEQ ID NO:6649
SEQUENCE LENGTH:314
SEQUENCE TYPE:nucleic acid
TOPOLOGY:linear
CLONE:HUMGS07839
SEQUENCE DESCRIPTION:
GATCTTTGGG GTTGGAGTGA AGGTCTTATC TGCATTAATT TTGTTAATGC ACATTGTAGA 60
TGTGGAGAAC ATCTTGACCT GCATAAGGAT TGCGTCTCAT TTTATTGTGT AGTGCTTTTT 120
CGAAATATTT ACATTTCTTT GGTTCCCATG TTCATGTATT CTGGAGAAGT AAAATTTCTT 180
GATTACTGGG CAGACCCTAA AACTACTAAG AGAAACATAA CATGTAGTTA GCTGTCTGCT 240
CATGGTTTGT TCAAATCTAA GCTAACCTTA ATGGTGCTGT GTCCGCTGTN CACAGAAAAC 300
GGCCTTAAAG TAGN 314

SEQ ID NO:6650
SEQUENCE LENGTH:104

SEQUENCE TYPE:nucleic acid
TOPOLOGY:linear
CLONE:HUMGS07840
SEQUENCE DESCRIPTION:
GATCCCCTCC CCTGCCCCTG GGTTCCTGCC TTCCNTCCTC AAGCAGGCAC NNAGGCTTTA 60
GAGAAGTATA GGGGGCTTCT CCCCTGCNGG GCTTACCACA NTTN 104

SEQ ID NO:6651
SEQUENCE LENGTH:358
SEQUENCE TYPE:nucleic acid
TOPOLOGY:linear
CLONE:HUMGS07841
SEQUENCE DESCRIPTION:
GATCAAGATG CCGAACCCTG TGTTCAGAAC CTCCAAATAC TGCCATGAGA AACTAGAGGG 60
CAGGTCTTCA TAAAAGCCCT TTGAACCCCC TTCCTGCCCT GTGTTAGGAG ATAGGGATAT 120
TGGCCCCTCA CTGCAGCTGC CAGCACTTGG TCAGTCACTC TCAGCCATAG CACTTTGTTC 180
ACTGTCCTGT GTCAGAGCAC TGAGCTCCAC CCTTTTCTGA GAGTTATTAC AGCCAGAAAG 240
TGTGGGCTGA AGATGGTTGG TTTCATGTTT TTGTATTATG TATCTTTTTG TATGGTAAAG 300
ACTATATTTT GTACTTAACC AGATATATTT TTACCCCAGA TGGGGATATT CTTTGTAN 358

SEQ ID NO:6652
SEQUENCE LENGTH:46
SEQUENCE TYPE:nucleic acid
TOPOLOGY:linear
CLONE:HUMGS07844
SEQUENCE DESCRIPTION:
GATCCAGTTT GGGATTATGA AATAAACCAC AAATNAAAAT TTTAAA 46

SEQ ID NO:6653
SEQUENCE LENGTH:63
SEQUENCE TYPE:nucleic acid
TOPOLOGY:linear
CLONE:HUMGS07845
SEQUENCE DESCRIPTION:
GATCTGGGGG GATGGAAAGC AAAATAAAGG AGGTGCCTGG NTGCATTATT TGCAGTGGGC 60
AAA 63

SEQ ID NO:6654
SEQUENCE LENGTH:290
SEQUENCE TYPE:nucleic acid
TOPOLOGY:linear
CLONE:HUMGS07848
SEQUENCE DESCRIPTION:
GATCCACCCA CCTCGGTCTC CCAAAGTGCT GGGATTACAG TCTTGAGCCA CCGGGTGACC 60
GTCTTACAGG GATATTTTNA ATCCCGTNAT GGACTCTGTC TCCAGGAGAG GGGTCTATCC 120

ACCCCTGCTC ATTGGTGGAT GTNAAACCAA TATNCCTTTC AACNGGTGNC TGNTAGGGAA 180
AANCTACTCC TCATTATCAT CATTATTATT GCTCTCCACT GTATCCCCTC TACCTGGCAT 240
GTGCTTGTNA AGTNCTAGTT GTTCAATAAA TTTGTNAATA ATGCTGTAAA 290

SEQ ID NO:6655
SEQUENCE LENGTH:145
SEQUENCE TYPE:nucleic acid
TOPOLOGY:linear
CLONE:HUMGS07849
SEQUENCE DESCRIPTION:
GATCTTCCTG GAAATNAGCA GTGACTAACG CTCACATAAC TGGTTTTTTT TTTNAACTGG 60
GCTGATGAAT ACATTTACCT ANGAAACTCA TTTCGTTTTA CTTAAGAGGG GANGTGCAGT 120
TTTNTTTTGG NAGTTCAGAN TCCAN 145

SEQ ID NO:6656
SEQUENCE LENGTH:351
SEQUENCE TYPE:nucleic acid
TOPOLOGY:linear
CLONE:HUMGS07850
SEQUENCE DESCRIPTION:
GATCCTCTGG TTTNTTCCAT TGGGGCGGAG TCGGGGGTGG AGGGAGCTGG CCACAACCCA 60
CTGCTCTGAT GGGTGGTTTG TCCAAGNATG CTGAATGTAA TGCCTGGTCA ATGTGGAAGC 120
CCATNAGGTT GCCCAGGGAA GCCTCCAAAA GCTGGGATGC TTGAGGGTAT CCAAGTTGAA 180
AAAGACAAAA TCTGACCATC AGCCAGTGAC AGTCCTGGCA AATNAAGGTG GGGCGGGGCA 240
GTNAGGGGTG GGNGANGGTG AATNNTTCAT TATTCCACCC NGCGGTTTNC TGGGGTGAGG 300
GGGAAGANTC GNTGCTTGCT TTGGGCACTG ANGGTTTTTC TGTNNGGGGA N 351

SEQ ID NO:6657
SEQUENCE LENGTH:346
SEQUENCE TYPE:nucleic acid
TOPOLOGY:linear
CLONE:HUMGS07852
SEQUENCE DESCRIPTION:
GATCATGTGA AGAAAACAGT GACCATTGAA AATNACCCTC ATCTGCCACC ACCTCCCATG 60
TGTTCAGTTC ACCCATGCAG GCATGCTGAG GTGATGAAGA AAATCATTGA GACTGTTGCA 120
GAAGGAGGGG GAGAACTTGG AGTTCATATG TATCTNCTTA TTTCCTTGAA ATTTGTACAA 180
GCTGTCATTC CAACAATAGA ATATGACTAC ACAAGACACT TCACAATGTA ATGAAGNGAG 240
CATAAAANCT ATCCTAATTA TTGGTTCTGA TTTTNAAAGA ATTAACCCAT AGATGTGNCC 300
ATTGNCCATA TTCATCANTA TATACAGTTT CTCTANTAGG GGAANN 346

SEQ ID NO:6658
SEQUENCE LENGTH:345
SEQUENCE TYPE:nucleic acid
TOPOLOGY:linear
CLONE:HUMGS07853

SEQUENCE DESCRIPTION:
GATCCACACG CTGGCCCAGG AGTTCGACAT TNACCGCAAG GTGGGCGNAG AGCCTGTNCC 60
TGTAACGAGG GATNATTCCA GCAATGGCTT TCCCCGCACT CAGCATGGTT CCCCAGTAAC 120
CTCCTGCTGA CCAGCTCCTC GGGGGAGCTG TGGAGGATGG TGCGCATCGG TGGACAGCCC 180
CTGGGCTTTA ATGAATGTGG CATCGTGGCA CAGATTGCAG GTCCCCTGGC TGCCGCTGAN 240
ATCTCTGCCT ACTACATCAG CACCTTCAAC TTNNGNNNNG GCCTGGTTGC CCGAGGACGG 300
TATCGGCAGC GTCATCGNGG GTCCTCCAGN GGNGGGNAGG AATGN 345

SEQ ID NO:6659
SEQUENCE LENGTH:47
SEQUENCE TYPE:nucleic acid
TOPOLOGY:linear
CLONE:HUMGS07854
SEQUENCE DESCRIPTION:
GATCTTTGTT AAAAAAATAA AAAGTACTAA CATTACAGAC ATGTAAA 47

SEQ ID NO:6660
SEQUENCE LENGTH:35
SEQUENCE TYPE:nucleic acid
TOPOLOGY:linear
CLONE:HUMGS07855
SEQUENCE DESCRIPTION:
GATCCTGCCA CTGCAGAGTG AAACCCTGTC TCAAA 35

SEQ ID NO:6661
SEQUENCE LENGTH:279
SEQUENCE TYPE:nucleic acid
TOPOLOGY:linear
CLONE:HUMGS07856
SEQUENCE DESCRIPTION:
GATCCCCCCG AAGTCTTCAC AGGCACTGCA TCGGGTTGTC TGGCGCCCTT TTCCTCCAGC 60
CTAAACTGAC ATCATCCTAT GGACTGAGCC GGCCACTCTC TGGCCGAAGT GGCCGCAGTG 120
TTGCCCCCGA GCTGCCCCCA CCCNCTCACA GGGTCCCTCA GNTTATAGGT GCCCAGGCTG 180
AGGTGAAGNG GCCTGGGGGN CCTGCNTTCC GGGCGCTCCT GGACCNTGGG GCAAACCTGT 240
GACCNTTTTN TACTGGANTA GAAATNNGTT TTATNATTN 279

SEQ ID NO:6662
SEQUENCE LENGTH:173
SEQUENCE TYPE:nucleic acid
TOPOLOGY:linear
CLONE:HUMGS07857
SEQUENCE DESCRIPTION:
GATCATAATA TAATTGGTTA CAAAGTGGAA GTGCCAGCTG GCTTAAGTAC CCAAAGAANA 60
GAATGCAGCA GCCTAACTTA GTGTTACCAT ATGTTACTGA ATTTGAAACT GACCTTTTTT 120
CCCACCCTAC TTCACACACC TAAAACTCTT TTTTTGTCAG ACCAAAGNGC AAA 173

SEQ ID NO:6663
SEQUENCE LENGTH:295
SEQUENCE TYPE:nucleic acid
TOPOLOGY:linear
CLONE:HUMGS07858
SEQUENCE DESCRIPTION:
GATCCAGTTG TCATCCACCA CAGACATCTC ACATCAGATA CAGACAGTTC CAAGATTGAC 60
AACAGAGAAC AACCTGCTGG AAAGACCTGG GCAGAAATGG AGAGCCCTGC GGGAACCATG 120
CTACATTTTC ATCTAAAGAG AGAATGCACA TCTGATGAGA CTGAAAGTNC TTTNTTGTTT 180
TAGATTGTAG AATGGTATTG ANTTGGTCTG TGGAAAATTG CATTGCTTTT ATTTCTTTGT 240
GTAATCAAGT TTAAGTAATA GGGGATATAT AATCNTAAGG CNTTTTAGGG TGGCN 295

SEQ ID NO:6664
SEQUENCE LENGTH:285
SEQUENCE TYPE:nucleic acid
TOPOLOGY:linear
CLONE:HUMGS07859
SEQUENCE DESCRIPTION:
GATCATGGTC AGANTGGAGT ACCTGGGGGC TGTCCATGCC CCCCTTTGGA CCTAATCCTA 60
CCCGTCCCCG CCAGCCCCTC TCCACACCNT TNCTCCCCAA GTACCCGCCC ACCGTTTCCA 120
TGGTCGAAGG TCAGGGCGAG AAGAACGTGA CCTTCTGGGG GAGACCGCTG CCACGGTGTG 180
AGGAGCTACA AGGGCAGAAG CTGGTGGTGG AGGNGNNACG ACCATNTNTA CACGTGCCTG 240
CTTGCAAAGA CCCTGAGGAA GAGGAACTCT AACANTTNTT TCCTN 285

SEQ ID NO:6665
SEQUENCE LENGTH:47
SEQUENCE TYPE:nucleic acid
TOPOLOGY:linear
CLONE:HUMGS07860
SEQUENCE DESCRIPTION:
GATCCTGTTA GTCACTGTGG TTCATCAAAT AAAACTGTTT GTGCAAA 47

SEQ ID NO:6666
SEQUENCE LENGTH:75
SEQUENCE TYPE:nucleic acid
TOPOLOGY:linear
CLONE:HUMGS07861
SEQUENCE DESCRIPTION:
GATCTCTAGA GCTGTCTTGT CGCCGCCCAG GATTGACCTG TGTGTAAGTC CCAATAAACT 60
CACCTACTCA TCAAA 75

SEQ ID NO:6667
SEQUENCE LENGTH:286
SEQUENCE TYPE:nucleic acid

TOPOLOGY:linear
CLONE:HUMGS07862
SEQUENCE DESCRIPTION:
GATCCTGCCT GGNCAACCCT TTCCTGTCCC AGGCCCTACA TTCAGCAGAA ACGCACTTTG 60
GACTTTNTCC TTTANATAAA AGAAAGACAT CCCAGGAGAA GGACAAACCA GAGGAGTGAA 120
CCAACAAAGA GTACCTAGGA ATGGAAGTTG AGCCCTGGAA TGGGGCTCCA TGGAGAGGTG 180
CATAGGACTC GGCAGAAATG GCCTCTCCCC AAAGCCTCTT TTTNAGAGGA GAGGGAAGCC 240
TATTTTNTTA ACTGGTTTGG GATAGGGAAT GGGGTTNCTT TTTNTN 286

SEQ ID NO:6668
SEQUENCE LENGTH:95
SEQUENCE TYPE:nucleic acid
TOPOLOGY:linear
CLONE:HUMGS07863
SEQUENCE DESCRIPTION:
GATCCCCGCC TGGCCAGGNG CAGGCACGGG TGGTCCCNGT TCCACCCCAA GAGAACTCGC 60
GCTCAGTAAA CGGGAACATG CCCCCTGCAG CCAAA 95

SEQ ID NO:6669
SEQUENCE LENGTH:213
SEQUENCE TYPE:nucleic acid
TOPOLOGY:linear
CLONE:HUMGS07864
SEQUENCE DESCRIPTION:
GATCTCCTAA TGATGTATTG TGCCGTGGNA GTACTGTGTG TGAATAGCAG TAGTGGGGGC 60
AAAAGCAATC TTCTCATTTG GAAATGTTGT AAATAATTTT ATTATATAGT GTTTTGGATG 120
TATTTGTTGT AGAAATGGNC CAGTGAATAA AGAGAATCTA AGGNTTTGTA CAATGTGAAA 180
TAACGTGTTA AATAANTGTC ATTGTCATAG AAA 213

SEQ ID NO:6670
SEQUENCE LENGTH:137
SEQUENCE TYPE:nucleic acid
TOPOLOGY:linear
CLONE:HUMGS07865
SEQUENCE DESCRIPTION:
GATCTAAAGA CCCGGGAGCG AGAAGCCAAG GCCAGGTTCT GGGNGNAGGG CCCAGAGAAG 60
CAGAACAGCC CAGAGCCCCA GGTGCCTGGC CTGGGCTAGA CCTCTGGAAT TGAGATTAAA 120
CAATTTGGAG TTGGAAA 137

SEQ ID NO:6671
SEQUENCE LENGTH:276
SEQUENCE TYPE:nucleic acid
TOPOLOGY:linear
CLONE:HUMGS07866
SEQUENCE DESCRIPTION:

GATCCGCTAC AGCAACGTGA AGAAGCTGGA AATAAAGCCA AAGTACCCGC ACTGCGAGGA 60
GAAGATGGTT ATCATCACCA CCAAGAGCGT GTCCAGGTAC CGAGGTCAGG AGCACTGCCT 120
GCACCCCAAG CTGCAGAGCA CCAAGCGCTT CATCAAGTGG TACAACGCCT GGAACGAGAA 180
GCGCAGGNTC TACGAAGAAT AGGGTGAAAA ACCTCAGAAG GGAAAACTCC AAACCAGTTG 240
GGAGGCNTTG TGCAAAAGGN CTTTTGCAGN TTNAAA 276

SEQ ID NO:6672
SEQUENCE LENGTH:348
SEQUENCE TYPE:nucleic acid
TOPOLOGY:linear
CLONE:HUMGS07868
SEQUENCE DESCRIPTION:
GATCCTGGAC CAGTCAGAAG CCGAGAAAGC TCGCAAGGAA CTTTTGGAGC TGGAGGCTCT 60
GAGCATGGCC GTGGAGAGCA CCGGGACTGC CAAGGCGGAG GCCGAGTCCC GTGCGGAGGC 120
AGCCCGGATT NAGGGAGAAG GGTCCGTGCT GCAGGCCAAG CTAAAAGCAC AGGCCTTGGC 180
CATTGAAACG GAGGCTGAGC TCCAGAGGGT CCAGAAGGTC CGAGAGCTGG AACTGGTCTA 240
TGCCCGGGCC CAGCTGGAGC TGGAGGTGAG CAAGGCTCAG CAGCTGGCTG AGGTGGAGGT 300
GAAGNAGTTC AAGCAGGTTG CCAGAGGCCA TAGGCCCAGC ACCNTNAN 348

SEQ ID NO:6673
SEQUENCE LENGTH:325
SEQUENCE TYPE:nucleic acid
TOPOLOGY:linear
CLONE:HUMGS07869
SEQUENCE DESCRIPTION:
GATCTGAGGC CAGCTCCCGT TCCCTGAGCT GCCTCCTGCT TCTCCCTCAA AGTCTCCTAC 60
CCTTCTCTTC CTCTTTNAAG CCCTCTCTTC CTCGCTTTCC TTCCTACCTA GCTCCTTGTT 120
GGTGAGCTTN TTGTGCCTTA ATNCTGTGAC CCAGGCCCTT ACACCANTTT NCACCTTCCT 180
GTNCGGAGNA CAAGGNCACT AGCTGCCCCA GGAAGTTGTG TGATTTTAAA TCACTTCTGT 240
CTTTGCTGGA AAGTGTATTT GTGCATAAAT AAAAGTCTGN GGATTTGTTA AAAAANAAGA 300
AAAANACNNA AAAANAACAN TNAAN 325

SEQ ID NO:6674
SEQUENCE LENGTH:348
SEQUENCE TYPE:nucleic acid
TOPOLOGY:linear
CLONE:HUMGS07870
SEQUENCE DESCRIPTION:
GATCAATGGA ATAGAATTTC ATTGGAAATG TAAAACACTT TCCCAACAAT GGTCATGACT 60
TTCTNCTGTT TTTGAGAAGA GTTTCATATG CTGGACCACA TTTNAGCTTT TATTGTTTTT 120
TTTTNCCCAT TGTCCAAAAA GTTAAGCAAC AAGTGGCCAC ACTTTTACGT GNCTACAACC 180
TGGAGTTCTG CAAAGAAGGT AATATTTNCT TGGNCTTTGC CTAANGTTAT CTCCCCATTC 240
TATGGTTACA TTTTATTTTG GCCTATGGGG CCTTCTAATA CGTTTTGGTA AAGAGGCGAG 300
TNTAANGNAA TTNCTTGTCA AATTTCACCT CAAAGGTAAT TTCNTGCN 348

SEQ ID NO:6675
SEQUENCE LENGTH:196
SEQUENCE TYPE:nucleic acid
TOPOLOGY:linear
CLONE:HUMGS07871
SEQUENCE DESCRIPTION:
GATCAACTCT CTAATGGTGC TAATGGCAAT CTAGCTAATG TGCAAATTTA GGAAGTCTTC 60
AGTACTAAGT ACATAATTTT CAAATAATAT TTTTNAATTG TCTACTTTGG ATGTTAGCTA 120
TGTCTTGTGN GTATAGGATT CCAATTTTAA GCACTATTTT GATGCAAAAN AATGCATAAN 180
NAATTTANTT TATAAA 196

SEQ ID NO:6676
SEQUENCE LENGTH:214
SEQUENCE TYPE:nucleic acid
TOPOLOGY:linear
CLONE:HUMGS07872
SEQUENCE DESCRIPTION:
GATCATAAAA ATGTGCGCCA ACAACGGCAG GCGCCATCTA AAGCAGCTTC TAAACAGAGA 60
GAGATGCTCA TNGAAGATGT NGGCAGTGAG GNAGAACAAG AAGAGGAGGA TGAGGCACCA 120
TTCCAGGAGA AAGATTCCGG CAGCGATGAA GATTTCCTAA TGGACGATGA TGACGNTNGT 180
NACTATGGCA GTTCGAAAAN GAAAAACANA TNNN 214

SEQ ID NO:6677
SEQUENCE LENGTH:138
SEQUENCE TYPE:nucleic acid
TOPOLOGY:linear
CLONE:HUMGS07875
SEQUENCE DESCRIPTION:
GATCCCCCAA GCCTGACGNG CCGNTNACCA TCCCCTCTGC CCTGCAGAGN CAGCCGCCAA 60
GGNTGACCTC AGCTTCGGAG CCACCTCTGG ATGAACTGCC CCCAGCACCC GCCCCATTAA 120
AGACCCGGAA GCCTGAAA 138

SEQ ID NO:6678
SEQUENCE LENGTH:208
SEQUENCE TYPE:nucleic acid
TOPOLOGY:linear
CLONE:HUMGS07876
SEQUENCE DESCRIPTION:
GATCCATCGC AGAGTCCTAA AGAAGAACCC ACTGAAAAAN TTGAGANTCA CAAGCTCCGG 60
GTGGATAAGG CAGCTGCTGC AGCANGGCAC TACAAGCCAA ATCAGATGAG AAGGCGGCGG 120
TTGCAGGCAA GANGCCTGTG GTAGGTAAGA NAGGAAAGAN GGCTGCTGTT GGTGTTAAGA 180
NGCAGAAGAN GCCTCTGGTG GGAAANNN 208

SEQ ID NO:6679
SEQUENCE LENGTH:188

SEQUENCE TYPE:nucleic acid
TOPOLOGY:linear
CLONE:HUMGS07878
SEQUENCE DESCRIPTION:
GATCTCCTGG AGGCTACAGG GAGCAGAGGC GATGCCCGCA GCGGCGAGCG TGTTCTCAGG 60
AGCAGGTGGG CAGGGGCCTT GCAGACTCTT CATTTCTTCC TTCCCTTATC ACCGTTAGCC 120
ATTTTNATGG GATAGAGATA CAGAGGAGCT CCCATTACAA AGCGCCATTA AACTGTCATC 180
TGTGCAAA 188

SEQ ID NO:6680
SEQUENCE LENGTH:35
SEQUENCE TYPE:nucleic acid
TOPOLOGY:linear
CLONE:HUMGS07879
SEQUENCE DESCRIPTION:
GATCTTGGTG GTAGTAGCAA ATATTCAAAC GCAAA 35

SEQ ID NO:6681
SEQUENCE LENGTH:297
SEQUENCE TYPE:nucleic acid
TOPOLOGY:linear
CLONE:HUMGS07880
SEQUENCE DESCRIPTION:
GATCTTCCGT GGTCAGAGTG CCACTGCGGG AGCTCTGTAT GGTCAGGATG TAGGGGTTAA 60
CTTGGTCAGA GCCACTCTAT GAGTTGGACT TCAGTCTTGC CTAGGCGATT TTTTCTACCA 120
TTTGTNTTTT GAAAGCCCAA GGTGCTGATG TCAAAGTGTA ACAGATATCA GTGTCTCCCC 180
GTGTCCTATC CCTGCCAAGT CTCAGAAGAG GTTGGGCTTC CATGCCTGTA GCTTTCCTGG 240
TCCCTCACCC CCATGGCCCC AGGCCCACAG CGTGGGAACT CACTTTCCCT TGTGNTN 297

SEQ ID NO:6682
SEQUENCE LENGTH:22
SEQUENCE TYPE:nucleic acid
TOPOLOGY:linear
CLONE:HUMGS07881
SEQUENCE DESCRIPTION:
GATCCAAGAT ATCACTTAGA AA 22

SEQ ID NO:6683
SEQUENCE LENGTH:292
SEQUENCE TYPE:nucleic acid
TOPOLOGY:linear
CLONE:HUMGS07882
SEQUENCE DESCRIPTION:
GATCCAGCTG TGGTTCCAGA GCAGTGAGCT GGTGAGCTGG GCAGATGGGG GCCAGCACAG 60
CAGNATCCAC CCAGNCTGAG GCTCAAGCTG CCCTTACCAC CCCATCCCCC ACGCAGGACC 120

AACTACCTCC GTCAGCAAGA ACCCAAGCCC ACATCCAAAC CTGCNTGTCC CAAACCACTT 180
ACTTCCCTGT TCACCTNTGN CCNACCNCAG CCCAGAGGAG TTTGNGCCAC CAACTTCAGT 240
GNCTTTTTGT ACCCNAAGNN AGCACAAGAT TGGACCAATA NTTTTTGNAG TN 292

SEQ ID NO:6684
SEQUENCE LENGTH:247
SEQUENCE TYPE:nucleic acid
TOPOLOGY:linear
CLONE:HUMGS07883
SEQUENCE DESCRIPTION:
GATCTGCCTT AGCCTCACTG CTTGCCCCTA AGTGGTGGGA TTGCAGGTGT GAGCCACTGT 60
GCTGGCCTCA AATAGTTTTN ATCAAAGCTA CTTCAATTAG TGGTTAGCAA GGCTTAAAGA 120
CTAATTCAGT GCTTTATTTT NACACTNGNT CGCAACATGT TGCTTTTTTN CCTGTGTCCT 180
ATGGGACCTA GTCATCTGTA TGTNAGATTC ACAGAGGAAT TAAAGAGATA AACATGCATG 240
TNCTAAA 247

SEQ ID NO:6685
SEQUENCE LENGTH:37
SEQUENCE TYPE:nucleic acid
TOPOLOGY:linear
CLONE:HUMGS07884
SEQUENCE DESCRIPTION:
GATCCACAAC TACACACATC ATAACCACAC CATCAAA 37

SEQ ID NO:6686
SEQUENCE LENGTH:81
SEQUENCE TYPE:nucleic acid
TOPOLOGY:linear
CLONE:HUMGS07885
SEQUENCE DESCRIPTION:
GATCTCTGAG GAGTTCCCTG GGGACAACTG AGCAGCCTCT GGAGAGGGGC CATTAATAAA 60
GCTCAACATC ATTGNACNAA A 81

SEQ ID NO:6687
SEQUENCE LENGTH:237
SEQUENCE TYPE:nucleic acid
TOPOLOGY:linear
CLONE:HUMGS07886
SEQUENCE DESCRIPTION:
GATCAGCCTT TCCAGGTGCT TTAGTCGTTC GCCTAGACAC GTTCCTGTCG CCTCTGACAG 60
CTGCAGTTGC TCTGCGCTGC TAGACATGGT GGACATTTNA GCAGCCTGAC CTGTGGGGAG 120
GGGGTCTCTC CCGAAGAGTT TCTGTTTTTA CTCAAAATAA TGTTATTCTC AGATGCTTGA 180
TGCACTGTTG GAAATGTGCT CNATTTAATC ATGCAGATAA ACCATTTAAA TGTCAAA 237

SEQ ID NO:6688

SEQUENCE LENGTH:93
SEQUENCE TYPE:nucleic acid
TOPOLOGY:linear
CLONE:HUMGS07887
SEQUENCE DESCRIPTION:
GATCTCTTGA GCCCAGGAAG CCGAAGCCAC AGTGAGCTGT GATTGCACCA CTGCACTCCA 60
GTCTGGGTGA CAAGAGTGAG ACCCCATCTC AAA 93

SEQ ID NO:6689
SEQUENCE LENGTH:62
SEQUENCE TYPE:nucleic acid
TOPOLOGY:linear
CLONE:HUMGS07889
SEQUENCE DESCRIPTION:
GATCGCAGCA TTGCACTCCA NCCTGGGCAA CAAGAGTGAA ACTCTGNCTC AGAAAAGAGA 60
AA 62

SEQ ID NO:6690
SEQUENCE LENGTH:221
SEQUENCE TYPE:nucleic acid
TOPOLOGY:linear
CLONE:HUMGS07890
SEQUENCE DESCRIPTION:
GATCCTCCTG CTTCAGCCTC CCAAAGTGCT AGGATTATAG CCACCGCACC TCGTCTTGAA 60
ATAGCCTTTT AAATGTTCAC CTCAGCTTGC CTCACAGTGG GTCTGTTCCT GGTTTCCCAG 120
ATGCATAAAG GAAGACATAT CCCTCCCCTG GGNAGCAAGG CTACAATGGG AGGGAGGGAG 180
AACATGGGAG CATGTGAATA AAATGGCATT AANTACTGAA A 221

SEQ ID NO:6691
SEQUENCE LENGTH:210
SEQUENCE TYPE:nucleic acid
TOPOLOGY:linear
CLONE:HUMGS07891
SEQUENCE DESCRIPTION:
GATCATCAAG AAAAAAGAAA CTTAAATGGA CACTTTTAAT GCTGCAAATN AGCTTGTCGT 60
GAAGTTACCT GATTGTNTAA TTAGAATGAC TACCACCTCT GTCTGATTCA CCTTCGCTGG 120
ATTCTAAATG TGGTATATTG CAAACTGCAG CTTTCACATT TATGGCATTT GTCTTGTTGA 180
AACATCGTGG TGCACATTTG TTTAANCAAA 210

SEQ ID NO:6692
SEQUENCE LENGTH:169
SEQUENCE TYPE:nucleic acid
TOPOLOGY:linear
CLONE:HUMGS07892
SEQUENCE DESCRIPTION:

GATCTGTCAT CTTCATAGCA CAACAAAACG AAATGATGGA AATNCTCTTN AGCTCACAAC 60
ATTTGTTTTN CTTTTAAAGT AAATGCAAGT ACCAAAGCTC ACTACTGCGG TTTGCCTGTN 120
CCTGGNCANT GAGGCGGAGC NCTGTTGTTT TGTTGGGGCA CCCCCTTNN 169

SEQ ID NO:6693
SEQUENCE LENGTH:226
SEQUENCE TYPE:nucleic acid
TOPOLOGY:linear
CLONE:HUMGS07893
SEQUENCE DESCRIPTION:
GATCTCTGCT TACCGTTCAA GAGGCGTGTG CAGGCCGACA GTCGGTGACC CCATCACTCG 60
CAGGACCAAG GGGGCGGGGA CTGCTGGCTC ACGCCCCGCT GTGTCCTCCC TCCNTCCCTT 120
CCTTGGGCAG AATGAATTCG ATGCGTATTC TGTGGCCGCC ATCTGCGCAG GGTGGTGGTA 180
TTCTGTCATT TACACACGTC GTTCTAATTA AAAAGCGCAT TATAAA 226

SEQ ID NO:6694
SEQUENCE LENGTH:31
SEQUENCE TYPE:nucleic acid
TOPOLOGY:linear
CLONE:HUMGS07894
SEQUENCE DESCRIPTION:
GATCTCTTAA ATAAACTTGC TTCTGGTTAA A 31

SEQ ID NO:6695
SEQUENCE LENGTH:182
SEQUENCE TYPE:nucleic acid
TOPOLOGY:linear
CLONE:HUMGS07895
SEQUENCE DESCRIPTION:
GATCTAAATT TGTTTATTTA AGCTGCAAGN AAGTTACATT AATTTGGAAT GTGTCATCAC 60
TTGGNCCCAG TATATCAGAT TTTNATTGAG TAAAATGTTA AAAATAATAA GTGTAACTCA 120
GATTCTGGAT GTNCGNGTTT ACAATACATT GCCTGTAATA AAAGCTGATT CTGANGTGCA 180
AA 182

SEQ ID NO:6696
SEQUENCE LENGTH:203
SEQUENCE TYPE:nucleic acid
TOPOLOGY:linear
CLONE:HUMGS07896
SEQUENCE DESCRIPTION:
GATCCCAACC CTGTGTCCTG CCCTGACCCC TGTTCTCCCA CTCGTGGCAC TGTCAGCCCA 60
GNCCTCCCTA CCGGCGAGAG TCCAGAGTGG GTACAGGAGC AAGGGGCACT GCTGGGGCCT 120
GATGGCTGAA GGAGACGCCG GCATCCTCGG GGGCCTGGGG AAGTTGTGTG TTGTGCAGTC 180
AGTAAAATCC TCCCACTGCN AAA 203

SEQ ID NO:6697
SEQUENCE LENGTH:51
SEQUENCE TYPE:nucleic acid
TOPOLOGY:linear
CLONE:HUMGS07897
SEQUENCE DESCRIPTION:
GATCAAATGA AATAATGTAA CGTGAGNATA CAGTATAAAT GATAAAGGAA A 51

SEQ ID NO:6698
SEQUENCE LENGTH:65
SEQUENCE TYPE:nucleic acid
TOPOLOGY:linear
CLONE:HUMGS07898
SEQUENCE DESCRIPTION:
GATCTCCGGG CAGCCACCAC CTCCTCGGTC TGCCCCNTCA TTAAAAAGTC CTCCTTCCAT 60
AGAAA 65

SEQ ID NO:6699
SEQUENCE LENGTH:59
SEQUENCE TYPE:nucleic acid
TOPOLOGY:linear
CLONE:HUMGS07900
SEQUENCE DESCRIPTION:
GATCTAGTGC CTAGTGTTGC TTTTCTGATG TAATAAAAGG TGGTCTGGCA GAACCTAAA 59

SEQ ID NO:6700
SEQUENCE LENGTH:129
SEQUENCE TYPE:nucleic acid
TOPOLOGY:linear
CLONE:HUMGS07901
SEQUENCE DESCRIPTION:
GATCCAGAGC CACAGAAAGA AATGTAGGTG TGAAGTATTA GGCTGCTGTC AGGGNGAGGA 60
TGGCAGATGG AGGCATCAAG CACAAGGAAA ATGCACAACC TATGCCCTNT TATACANNNN 120
GTTCATGTN 129

SEQ ID NO:6701
SEQUENCE LENGTH:39
SEQUENCE TYPE:nucleic acid
TOPOLOGY:linear
CLONE:HUMGS07902
SEQUENCE DESCRIPTION:
GATCTTAAGT TTACAATTAA AAACTCAGTA CTCAATAAA 39

SEQ ID NO:6702
SEQUENCE LENGTH:56

SEQUENCE TYPE:nucleic acid
TOPOLOGY:linear
CLONE:HUMGS07903
SEQUENCE DESCRIPTION:
GATCCCCCCG GGCATGGCCT GGGCTGGTTT TGAATGAAAC GACCTGAACT GTCAAA 56

SEQ ID NO:6703
SEQUENCE LENGTH:31
SEQUENCE TYPE:nucleic acid
TOPOLOGY:linear
CLONE:HUMGS07904
SEQUENCE DESCRIPTION:
GATCCTTTTA AAATAAAAAT NTTGATGCAA A 31

SEQ ID NO:6704
SEQUENCE LENGTH:108
SEQUENCE TYPE:nucleic acid
TOPOLOGY:linear
CLONE:HUMGS07905
SEQUENCE DESCRIPTION:
GATCAGGAGC CTTCATTAAA ACAGAAATTT TAGGGAAAAC CACAAAAACC TTCGTAAAGT 60
GAATTGCATT AAACATCAAT TATAATAAAA TTTCATATTT TGANTAAA 108

SEQ ID NO:6705
SEQUENCE LENGTH:29
SEQUENCE TYPE:nucleic acid
TOPOLOGY:linear
CLONE:HUMGS07909
SEQUENCE DESCRIPTION:
GATCAAAAGG GACAAGCATC AAGCACAAA 29

SEQ ID NO:6706
SEQUENCE LENGTH:152
SEQUENCE TYPE:nucleic acid
TOPOLOGY:linear
CLONE:HUMGS07911
SEQUENCE DESCRIPTION:
GATCATGGGC AAAATCTAGC CACAGTCCTG AGAGTCCAGG CTTCTGGGAT GCCCAGCTGG 60
GTATAAAAGT CCCTACTAAC CCTGNTTCAA ATTCAGAGGT TTCTTTGGTT NAGAATGCCT 120
CAATGAGATT TTGATACATC CAAGAGCGCA AA 152

SEQ ID NO:6707
SEQUENCE LENGTH:129
SEQUENCE TYPE:nucleic acid
TOPOLOGY:linear

CLONE:HUMGS07912
SEQUENCE DESCRIPTION:
GATCTAAAAA ATACAAAATG TAGACAATTA CTATTCAGGT GCTTACTTTT AAGCCAATAT 60
GTAGCAGNGN AAAACTAATT TCTCTGTGGC AGTATTTTAA TTTTTGGNAT GTCCCNGGNN 120
TTAACTGAN 129

SEQ ID NO:6708
SEQUENCE LENGTH:118
SEQUENCE TYPE:nucleic acid
TOPOLOGY:linear
CLONE:HUMGS07913
SEQUENCE DESCRIPTION:
GATCCGCACT GGGGGTTCTG CAGGGTGGGG NCAACCACAG CCTGTCACAA GCTCACCGTC 60
AGCCTCTNTG GNGCCATTCG GCAGCATGAA GTCCCTATCA CCCACATAAT AATGNGNN 118

SEQ ID NO:6709
SEQUENCE LENGTH:149
SEQUENCE TYPE:nucleic acid
TOPOLOGY:linear
CLONE:HUMGS07915
SEQUENCE DESCRIPTION:
GATCTTTCCC AGTATCTNNA GTGGGTGACC ACACTTGTCA GTGGGAGNCT NTGAAACTAC 60
CCTATCGNCT CCTTAAGGGC GGGATGAACT GCTTTGTGAC TTGGAAAGGT ACGCTGCTGG 120
CCAGCATTGG AGANGAAGCT NCTGAGCAN 149

SEQ ID NO:6710
SEQUENCE LENGTH:236
SEQUENCE TYPE:nucleic acid
TOPOLOGY:linear
CLONE:HUMGS07916
SEQUENCE DESCRIPTION:
GATCAGGTGG TCAAGGTGCT GCACGATGCC CAGCAGCAGT GCCGAGACGG CCACCCGGTT 60
GTGGTCAACA TCCTCATTGG GAGGACGGAC TTCCGCGATG GCTCCATTGC TGTATAGGGC 120
CTTGTGGGTC AGNACGCTTN GCTGCCTTCC TACCCCTGGA CTGTNTCCAA NTGGTTTTNG 180
AGTCTCATNA TTGCTTACCC CTGGNGCTNA TCCCATGAGG NCCCANTGNA CTTCAN 236

SEQ ID NO:6711
SEQUENCE LENGTH:173
SEQUENCE TYPE:nucleic acid
TOPOLOGY:linear
CLONE:HUMGS07917
SEQUENCE DESCRIPTION:
GATCCGCAAA GAGGAAGACC GCCTCTTCAT TCTCACGACC NTCACCTATT GATGGGAATN 60
TGTGCGGGCT CAGGGTTCCT NNACACACTA GNTGTGGGAA GCCATAGGAG CCTCCAGATG 120
GGGGCTGGCC TCTCTTGCCC AGCNAGNGGG CAGGGCCTGT GGGNTGGTGA ATN 173

SEQ ID NO:6712
SEQUENCE LENGTH:48
SEQUENCE TYPE:nucleic acid
TOPOLOGY:linear
CLONE:HUMGS07918
SEQUENCE DESCRIPTION:
GATCACGNCA TGGCACTTCA GCCTGAGCGN CAGAATGAGA CTATTAAA 48

SEQ ID NO:6713
SEQUENCE LENGTH:50
SEQUENCE TYPE:nucleic acid
TOPOLOGY:linear
CLONE:HUMGS07919
SEQUENCE DESCRIPTION:
GATCGTGTCA CTGAACTCCA GNCTGGGTAA CAGTGCGACT CTGTCTCAAA 50

SEQ ID NO:6714
SEQUENCE LENGTH:156
SEQUENCE TYPE:nucleic acid
TOPOLOGY:linear
CLONE:HUMGS07920
SEQUENCE DESCRIPTION:
GATCTTTGTT TAAATGCCAA AATGTACTTA AATGAGTTAC TTAGAATGCC ATAAAATTGC 60
AGTTTNATGT ATGTATATAA TCATGCTCAT GTATATTTAG TTACGTATAA TGCTTTCTGA 120
GTGAGTTTTA CTCTTAAATC ATTTGGTTAA ATCAAA 156

SEQ ID NO:6715
SEQUENCE LENGTH:208
SEQUENCE TYPE:nucleic acid
TOPOLOGY:linear
CLONE:HUMGS07921
SEQUENCE DESCRIPTION:
GATCGATACC CCCACCCCTC CACTNGCCAG CCTGGGGGTG CCCTGCCTGC CCTCCTGGTA 60
CTGTTGTCTT CCCCTCGGCC CNNTCACATG TGTATTCAGC AGCCCTATGG CCTTGGCTNT 120
GGGCCTGATG NGNAGGGGTA GAGGGAAGNT GAGCATAGCA CATTTTCCTA GAGCGNGAAT 180
TGGGGGNAAG CTGTTATTTT NATATTAN 208

SEQ ID NO:6716
SEQUENCE LENGTH:57
SEQUENCE TYPE:nucleic acid
TOPOLOGY:linear
CLONE:HUMGS07922
SEQUENCE DESCRIPTION:
GATCACGACA CTACACTCCA GCCTGGGGGA CCGGGCAAGA CTCTGTCTGT CTTTAAA 57

SEQ ID NO:6717
SEQUENCE LENGTH:333
SEQUENCE TYPE:nucleic acid
TOPOLOGY:linear
CLONE:HUMGS07923
SEQUENCE DESCRIPTION:
GATCTTGAAA AAAAAAGCAA AAGCTTAAAT ATTTGATACA AGTTTACTTA GCTACAACAT 60
ACTTTACATT GTTGCCTTTA GTTATCTCAC AGGCACTGAC ATTTTATATT TAGAAAATAC 120
TTTTAATCTT NCTAATCTTT TTTTGTAAAT ATTAGTGTCC ATTCTGTATG ACTCGCTAAC 180
CTACTTTGCA AGGCTTTGGG CAACATTTTA GCTCATTAAC TTCAAGATGA TGTGTCATCT 240
GTATAGGTCA AAGAATGGGN CTTCTGAACT GAGGAATTTT GCTGTTGACA GCCAAAGTNT 300
AGTGTACAAG GTTTGATGNA ACTNGNTTAT GTN 333

SEQ ID NO:6718
SEQUENCE LENGTH:57
SEQUENCE TYPE:nucleic acid
TOPOLOGY:linear
CLONE:HUMGS07925
SEQUENCE DESCRIPTION:
GATCTTGGAC TTCCCAGCCT CCAGAATTGT GAGAAATAAA TTTCTGTTGT TGATAAA 57

SEQ ID NO:6719
SEQUENCE LENGTH:63
SEQUENCE TYPE:nucleic acid
TOPOLOGY:linear
CLONE:HUMGS07927
SEQUENCE DESCRIPTION:
GATCTTTGCT TAATAAGANG AAAAAAGAGG CAAGGAGAAA ACGAAGAGAA AGAAATTTTG 60
AAA 63

SEQ ID NO:6720
SEQUENCE LENGTH:22
SEQUENCE TYPE:nucleic acid
TOPOLOGY:linear
CLONE:HUMGS07928
SEQUENCE DESCRIPTION:
GATCCNGTGT TCTTCTGTGA AA 22

SEQ ID NO:6721
SEQUENCE LENGTH:186
SEQUENCE TYPE:nucleic acid
TOPOLOGY:linear
CLONE:HUMGS07932
SEQUENCE DESCRIPTION:

GATCCGGCTT GGACAGGCAC CTGAGATGGT GCCAAAGTGC AGCTGACTCT TCCCACGACA 60
GCCCTGCCCT TCCCATGAGG CAGGCTCTTC AGTGAGTGTT TGAACGTAAT TATGTAGTTT 120
TCTGTTTAAT TGAAAAAGNG AGCTATGCCT TTTTTCTTT TTGGANGTAA AGCAGCTAAA 180
ANCAAA 186

SEQ ID NO:6722
SEQUENCE LENGTH:45
SEQUENCE TYPE:nucleic acid
TOPOLOGY:linear
CLONE:HUMGS07933
SEQUENCE DESCRIPTION:
GATCCAGGTG TTTGTTTCTA ACTTCTGTAA TACATACAAT GCAAA 45

SEQ ID NO:6723
SEQUENCE LENGTH:173
SEQUENCE TYPE:nucleic acid
TOPOLOGY:linear
CLONE:HUMGS07934
SEQUENCE DESCRIPTION:
GATCATGGTG ATTTNACTGC TGCTTGTGGN TATCGTGGTT GTTGCAGTCT GGCCGACCAA 60
CTGATGGCAG TAAAGAGACC ACCAGCAGTG ACACCTGCCA ATNACAGATG CAAGCCCAAC 120
ACCCTNTTGG TACGCAAAAC CTNATCTCAA TAAATTCCCC CAAAGCTCTG AAA 173

SEQ ID NO:6724
SEQUENCE LENGTH:61
SEQUENCE TYPE:nucleic acid
TOPOLOGY:linear
CLONE:HUMGS07935
SEQUENCE DESCRIPTION:
GATCCCAAGC CCTGCATTGT AAGCCCACAG TAGGCACTCA ATAAATGCTT GTTAAAGGAA 60
A 61

SEQ ID NO:6725
SEQUENCE LENGTH:269
SEQUENCE TYPE:nucleic acid
TOPOLOGY:linear
CLONE:HUMGS07936
SEQUENCE DESCRIPTION:
GATCCTGCAG AAGAATAACC TGAATCTGCT CAGAGACCTA AGCTGTGCAC ATTGCCCATA 60
GCCTCAGGAA CAGTCCAGAC TGGGGCGGTG TGGTCATATT ACACAGNNAG GAGGGTGATT 120
CAGAGTTCAT GAATATCATT GCCAATNAGA TTGGGTCAGA GGAGACCCTC CTGTTCTTAA 180
CTGTGCGCGA TGAGNAAGNT GGTGGNCTNT TNTTACTGGC AGGGCCACNA NCGCTCATGT 240
GGAGACCCNG GGGCCCAGGG TGGCTGATN 269

SEQ ID NO:6726

SEQUENCE LENGTH:229
SEQUENCE TYPE:nucleic acid
TOPOLOGY:linear
CLONE:HUMGS07937
SEQUENCE DESCRIPTION:
GATCCCTCAT CCCTGAAATT GAGTTTATGT AGTCATTTTA CTTATTTNAT TCATTAGCTA 60
ACTTTGTCTA TGTATATTTN TAGATATTGA TTAGTGTAAT CGATTATAAA GGATATTTAT 120
CAAATCCAGG GATTGCATTT NGAAATTATA ATTATTTCCT TTGCTGAAGT ATTCATTGTA 180
AAACATACAA AATAAACATA TTTNAANACA TTTGCATTTN ACCACCAAA 229

SEQ ID NO:6727
SEQUENCE LENGTH:50
SEQUENCE TYPE:nucleic acid
TOPOLOGY:linear
CLONE:HUMGS07938
SEQUENCE DESCRIPTION:
GATCCATGAA CTCTGACCCC TCCCCAGTAA AGGCTTCTGT AGAGAGCAAA 50

SEQ ID NO:6728
SEQUENCE LENGTH:122
SEQUENCE TYPE:nucleic acid
TOPOLOGY:linear
CLONE:HUMGS07939
SEQUENCE DESCRIPTION:
GATCCCCGCA CCAGCCTCTC CTCTCTTTCC CTGCAACCTG TTACCTCACA CAGCTGCTCA 60
GGAACAGCAG AAATAGTAAA ACTCTTCCAT ATATGTGGCA CCTCAGCCGC CCCTCCNTTA 120
AA 122

SEQ ID NO:6729
SEQUENCE LENGTH:213
SEQUENCE TYPE:nucleic acid
TOPOLOGY:linear
CLONE:HUMGS07941
SEQUENCE DESCRIPTION:
GATCAGGAAG TTCTTAGATT CTTGGATGAC AGATGCATGT TGATGCCCTA TGGAGATGTC 60
CTTNTGTTTT GAGGTCACTG AGGTAGGAAG ACCTGTCTAC TCTTGGTTTC ACCACTAGAA 120
CAGTCTTGGG CTGGATGGGT TATAGAGCTG AGCGGCTGTA ATGGTTCTNT TTTTACATTA 180
ACAAAAACAA TTAAAAACAC CAAAAACAAC AAA 213

SEQ ID NO:6730
SEQUENCE LENGTH:85
SEQUENCE TYPE:nucleic acid
TOPOLOGY:linear
CLONE:HUMGS07942
SEQUENCE DESCRIPTION:

GATCCCAGGN ATTCAAGGTT AGAGTGAGCT ATGATTGCAC CACTGTACTC CAGCCTGGGC 60
ANCAGAGANN GACCTGTCTC ACAAA 85

SEQ ID NO:6731
SEQUENCE LENGTH:313
SEQUENCE TYPE:nucleic acid
TOPOLOGY:linear
CLONE:HUMGS07944
SEQUENCE DESCRIPTION:
GATCCAGCTG GAATTTGAGG ACACTGGAAC AATGAGGTCA GCTTCCAAAA CAAAACAAAA 60
CAAAACTTTA AACATCTGTG TAAGTACAGG GTGGAATATA ATGAATTCCA GGTACCCATC 120
ACTTAGGTTC CACAATTAGC CATGGCCAAT CTTGTGTGAT AGGTATGTCC CTACCTATTT 180
CTCCCCTGCC CTNATTGTGA AGTAAATATT TTCCTAAAAG GTCTTTTTAA AATTAAGTTA 240
TAACCATGCC ATCATTGTAC AAANNTAATG CTATTANNTT CCTTANTNTN ATCAAATNTT 300
CNNGNCTNAN NTN 313

SEQ ID NO:6732
SEQUENCE LENGTH:73
SEQUENCE TYPE:nucleic acid
TOPOLOGY:linear
CLONE:HUMGS07945
SEQUENCE DESCRIPTION:
GATCACTTGA ACCTAAGAGT TTGAGGCTGC GCACTGCACT CTGGCCTGAC AGAGCGAGTC 60
ACTGTCTCTT AAA 73

SEQ ID NO:6733
SEQUENCE LENGTH:257
SEQUENCE TYPE:nucleic acid
TOPOLOGY:linear
CLONE:HUMGS07946
SEQUENCE DESCRIPTION:
GATCTGAATT CTAATGCTGG CTTGTGCCTC TAACTATGTG GTTTGGTCAT TATAGATAAT 60
GCCCAGTTTA ANTTTCCCCA TCTTTGAAAA GGAGATAATA TGTNATCTCT TGGGAGTAGT 120
TCTGAATATG AAGTGTGTTG ATACAAGAAG CACTGACAAT GTTTCTGTNN NNNAAATTTA 180
AAACTGGCCT GGTTTGCCTT TTTAATCAAG NGNGCTTAAC AGGTTAAAAN TTGGAATTAG 240
NCCTAAAAAN AAAAACN 257

SEQ ID NO:6734
SEQUENCE LENGTH:148
SEQUENCE TYPE:nucleic acid
TOPOLOGY:linear
CLONE:HUMGS07948
SEQUENCE DESCRIPTION:
GATCCTTTAG CCTCCTGGNA GTGCCCCAGT TGTACCCCCT ACACACCCCT CTTGGCATTG 60
AGTGCCAGTC CTCTGCCAGG CTNTGTGTTA CAAGTTGGGG NGGGCGGCAA AGTACCGAAT 120

TAAAGATGTC AGTTCTAAAG GAGGNAAA 148

SEQ ID NO:6735
SEQUENCE LENGTH:289
SEQUENCE TYPE:nucleic acid
TOPOLOGY:linear
CLONE:HUMGS07949
SEQUENCE DESCRIPTION:
GATCATATCC AATTCATATG GAAGTCCCGG GTNTGTCTTC CTTATCATCG GGGTGGCAGC 60
TGGTTCTCAA TGTGCCAGCA GGGCCTCAGT ACCTGAGCCT NAATCAAGCC TTATCCACCA 120
AATACACAGG GAAGGGTGAT GCAGGGAAGG GTGACATCAG GAGTCAGGGC ATGGNNTNGN 180
NAGATGANTA CTTTGCTGGA TGAAGCAGGC TGCAGGGCAT TCCAGCCAAG GGCACAGCAG 240
GGGACAGTGC CGGGAGGTNT NGGGTAAGGG AGGGCAGTNA CNTNAGAAA 289

SEQ ID NO:6736
SEQUENCE LENGTH:114
SEQUENCE TYPE:nucleic acid
TOPOLOGY:linear
CLONE:HUMGS07951
SEQUENCE DESCRIPTION:
GATCAGGCTT TTAAATACTC ATTTACAAGT TTTCTATCCT CCTTCAGTGT TAAAGTAGAA 60
AGTAAAAAGA GTATCTNATA CATGCATGAA ATTAAAGCAT ATACCAAATG CAAA 114

SEQ ID NO:6737
SEQUENCE LENGTH:229
SEQUENCE TYPE:nucleic acid
TOPOLOGY:linear
CLONE:HUMGS07952
SEQUENCE DESCRIPTION:
GATCACCCAG CATCCTTCCC CACCCCAGCT GTGTATTTAT ATAGATGGAA ATATACTTTA 60
TATTTTGTAT CATCGTGCCT ATAGCCGCTG CCACCGTGTA TAAATCCTGG TGTATGCTCC 120
TTATCCTGGA CATGAATGTA TTGTACACTG ACGCGTNCCC ACTCCTGTAC AGCTGCTTTG 180
TTTCTTTGCA ATGCATTGTA TGGCTTTATA AATGATAAAG TTAAAGAAA 229

SEQ ID NO:6738
SEQUENCE LENGTH:295
SEQUENCE TYPE:nucleic acid
TOPOLOGY:linear
CLONE:HUMGS07953
SEQUENCE DESCRIPTION:
GATCTCTTGT NCTTTAATTT TAGGGTCTTG TTCCAGGACT CAAATCAGTA ACTTGGTGAT 60
TACAAGGTGC TGAATGTGTT GGTAACCATA TCGCAATACA CCTCAAGGAA AAGGTTCAGA 120
TTTTNATTTT TAAAATATTT NCATTTTTTC CTTGAATTTN ATATCCGTTT GTCCACTCGT 180
ACATGCCTAG CCTACAGAAG GGGATATATA TNATGANATG GTCATTTTCC TGAAGNGNAT 240
ATTTTGCTTG AAATGCAAAG GNCTGAAAGN GNTTTGTAGG TTGTTGGTTT TTTCN 295

SEQ ID NO:6739
SEQUENCE LENGTH:85
SEQUENCE TYPE:nucleic acid
TOPOLOGY:linear
CLONE:HUMGS07954
SEQUENCE DESCRIPTION:
GATCATAACA CTACTCCAGT CTGGGTGATG AAGTGAGACC TGTCTACAAA AAAAAGAAGC 60
CCAAACTAAA CCTAAAGCAA GCAAA 85

SEQ ID NO:6740
SEQUENCE LENGTH:145
SEQUENCE TYPE:nucleic acid
TOPOLOGY:linear
CLONE:HUMGS07955
SEQUENCE DESCRIPTION:
GATCAGGCTG GTCTCAAACT CCTCGGTTCA AGCAATTCGC CCACCTTGGC CTCCCAAAGT 60
GCTGGGATTA CAGGGGAGCC ACTGCACTGG CCTTCATTGT CTTTTTGCTG CACAACCTAA 120
AAAACCAGTG ACCCTGTATT GGAAA 145

SEQ ID NO:6741
SEQUENCE LENGTH:81
SEQUENCE TYPE:nucleic acid
TOPOLOGY:linear
CLONE:HUMGS07956
SEQUENCE DESCRIPTION:
GATCCTAACA TGTATTGATT GTAAAAGTGA AATATGTAAT AGCCACACTT CACTGTTTTA 60
AAATAAAAGT GCAATTTCAA A 81

SEQ ID NO:6742
SEQUENCE LENGTH:50
SEQUENCE TYPE:nucleic acid
TOPOLOGY:linear
CLONE:HUMGS07957
SEQUENCE DESCRIPTION:
GATCTTGTCT CCAATTAAAC CGAGGCTTTC ACCGATAAAA AAAAAACAAA 50

SEQ ID NO:6743
SEQUENCE LENGTH:115
SEQUENCE TYPE:nucleic acid
TOPOLOGY:linear
CLONE:HUMGS07958
SEQUENCE DESCRIPTION:
GATCTGATGA AGTATATNTT TAATTGCCAT TTTGTCCTTT GATTATATTG GGAAGTTGAC 60
TAAACTTGAA AAATGTTTTT AAAACTGTGA ATAAATGGAA GCTACTTTNA CTAAA 115

SEQ ID NO:6744
SEQUENCE LENGTH:175
SEQUENCE TYPE:nucleic acid
TOPOLOGY:linear
CLONE:HUMGS07959
SEQUENCE DESCRIPTION:
GATCGGGCTA CTACAAAGTT CTGGGAAAGG GAAAGCTCCC AAAGCAGCCT GTCATCGTGA 60
AGGCCAAATT CTTCAGCAGA NGAGCTGNGG AGAGATTAAG AGTGTTGGGG GGGCCTGTGT 120
CTTGGTGGGT TTAAGGCNCA TTGGNGGGNA NTTCATTNAA TTNTTACTTC TTTTN 175

SEQ ID NO:6745
SEQUENCE LENGTH:61
SEQUENCE TYPE:nucleic acid
TOPOLOGY:linear
CLONE:HUMGS07960
SEQUENCE DESCRIPTION:
GATCAAAAGT AACACGTNNA GACTCTGAGC TTAATCCATT AAATGGATTT NAGCATCTAA 60
A 61

SEQ ID NO:6746
SEQUENCE LENGTH:118
SEQUENCE TYPE:nucleic acid
TOPOLOGY:linear
CLONE:HUMGS07961
SEQUENCE DESCRIPTION:
GATCATNAAC TACACTGGTT ATNTGGAAGT CTAGGTTCTG GTCCTGCTAC CCCTTTAACT 60
TGAGTNATTT ACAAGTCTGT TTAAAAGTTT CTAGACATAC ACTGGTTTGN TTTTTAAA 118

SEQ ID NO:6747
SEQUENCE LENGTH:140
SEQUENCE TYPE:nucleic acid
TOPOLOGY:linear
CLONE:HUMGS07963
SEQUENCE DESCRIPTION:
GATCGCCCAA CGTTTTTGGN TTATAGAATT ATTATTTCCT GCTTTCTTTC TTTGGGTCTT 60
TTGAATTTCT TTGGTTTCGT NTTTAAGAAG TAACCCANCA TTTCCTACAA CACTAAATAA 120
AATGGTACTT ACCTTTCAAA 140

SEQ ID NO:6748
SEQUENCE LENGTH:68
SEQUENCE TYPE:nucleic acid
TOPOLOGY:linear
CLONE:HUMGS07964
SEQUENCE DESCRIPTION:

GATCCCCGTT CCACCCCAAG AGAACTNGCG CTCAGTAAAC GGGAACATGC CCACTGCAGA 60
CACGTAAA 68

SEQ ID NO:6749
SEQUENCE LENGTH:79
SEQUENCE TYPE:nucleic acid
TOPOLOGY:linear
CLONE:HUMGS07965
SEQUENCE DESCRIPTION:
GATCCCCCCT GCCTATCAGC TTCTCCTGTG GNGCCTGTTC CTCACTGGAA ATTGGCCTCT 60
GTGTGTGTGT GTGTGTGTN 79

SEQ ID NO:6750
SEQUENCE LENGTH:28
SEQUENCE TYPE:nucleic acid
TOPOLOGY:linear
CLONE:HUMGS07966
SEQUENCE DESCRIPTION:
GATCGAAAGG GAGGCAGGTT TGCCTAAA 28

SEQ ID NO:6751
SEQUENCE LENGTH:294
SEQUENCE TYPE:nucleic acid
TOPOLOGY:linear
CLONE:HUMGS07967
SEQUENCE DESCRIPTION:
GATCTCAAGC CAGCCCCNTC CAGCTCATGA CACTGTTTGG CCTTTCTTGG GGAGAAGGCG 60
GGGTATTCCC ACTCACCAGC CCTGGCTGTC CCATGGGGAA ACCCTGGAGC CATCCCTTNG 120
GAGCCAACAA GACCGCCCCA GGGCTATAGC AGTAAGAACT TTAAAGCTCA GGAGGGTGAC 180
GNCCAGATNC GNCTGCTGGG AAGAGCTCCC CTNCACAGCT GCAGCTGAAT GCATTAGGGC 240
TACCGNAGGN CCCGGACTTA NCAAACTTTG NCNACAANNG TTCCTAGGGN TTTN 294

SEQ ID NO:6752
SEQUENCE LENGTH:160
SEQUENCE TYPE:nucleic acid
TOPOLOGY:linear
CLONE:HUMGS07969
SEQUENCE DESCRIPTION:
GATCCCCAAG TGTGAACAGT CTGAAAAGGG CTTTTTGCTT GCTGCAGGGA CCTGAGAATT 60
GAGAAACAGC TGTGAAACTT TGAACCCTGG CTGGAAGGTC CTGGTATTGA CTGTGGCANG 120
AAAACGGGGG AGCTAATCTG TAATCTGCTT AGACGCCAAA 160

SEQ ID NO:6753
SEQUENCE LENGTH:98
SEQUENCE TYPE:nucleic acid

TOPOLOGY:linear
CLONE:HUMGS07971
SEQUENCE DESCRIPTION:
GATCAGATTC ACGGACCCAG AGCTTTTCCA TGTGTTTATA TTGTAAATAT TTTTNATTTC 60
ATCAAATTAT TTATTCATTA AAAGAAATTT TTGTGAAA 98

SEQ ID NO:6754
SEQUENCE LENGTH:35
SEQUENCE TYPE:nucleic acid
TOPOLOGY:linear
CLONE:HUMGS07973
SEQUENCE DESCRIPTION:
GATCCTGTCT CTTAGGAAAA AAGAAAACCT CTAAA 35

SEQ ID NO:6755
SEQUENCE LENGTH:64
SEQUENCE TYPE:nucleic acid
TOPOLOGY:linear
CLONE:HUMGS07974
SEQUENCE DESCRIPTION:
GATCAAAGAC CCTGCCTCTA TTTNACCCCG CACTGACTGA ATAAAGCTCC TCTGGCCGTT 60
NAAA 64

SEQ ID NO:6756
SEQUENCE LENGTH:38
SEQUENCE TYPE:nucleic acid
TOPOLOGY:linear
CLONE:HUMGS07975
SEQUENCE DESCRIPTION:
GATCATCTGA GCTGAAGAAA TAAAAAAACA CACACAAA 38

SEQ ID NO:6757
SEQUENCE LENGTH:56
SEQUENCE TYPE:nucleic acid
TOPOLOGY:linear
CLONE:HUMGS07976
SEQUENCE DESCRIPTION:
GATCATTCAT ACACTATATT TATTTTTNCA TTAAANTATT TTAAAACCTT TGGAAA 56

SEQ ID NO:6758
SEQUENCE LENGTH:105
SEQUENCE TYPE:nucleic acid
TOPOLOGY:linear
CLONE:HUMGS07977
SEQUENCE DESCRIPTION:

GATCTTTCAT CAAGATTTTA CCTGCATTTA TGTCTAAGGG TCCCTGATAA GGCTTGAGGC 60
ATGTAACCTA TGTCTAATTA AAAATGATTT GTTGAATGAA NCAAA 105

SEQ ID NO:6759
SEQUENCE LENGTH:65
SEQUENCE TYPE:nucleic acid
TOPOLOGY:linear
CLONE:HUMGS07981
SEQUENCE DESCRIPTION:
GATCTACAGA AGCTTTNATT CTCTTACCTT AACAGTAGTA ATAAAGTTAA CATTNTCACA 60
TCAAA 65

SEQ ID NO:6760
SEQUENCE LENGTH:106
SEQUENCE TYPE:nucleic acid
TOPOLOGY:linear
CLONE:HUMGS07986
SEQUENCE DESCRIPTION:
GATCTGTGTG TTGTGGGAAG AGAATTTTCA ATATGTAACT ACGGAGCTGT AGTGCCATTA 60
GAAACTGTGA ATTTCCACAT AAATCTGAAC ACTTGTCTTT ATTAAA 106

SEQ ID NO:6761
SEQUENCE LENGTH:106
SEQUENCE TYPE:nucleic acid
TOPOLOGY:linear
CLONE:HUMGS07987
SEQUENCE DESCRIPTION:
GATCTCATGG CTTTGATTGN TTTGGGCTGT TCAAAATGTT TATTTGNAAA ACGTATACAT 60
TAATAAACTT AACAAAGCGN TATAAAATAC AGCGNAATCA CCCAAA 106

SEQ ID NO:6762
SEQUENCE LENGTH:335
SEQUENCE TYPE:nucleic acid
TOPOLOGY:linear
CLONE:HUMGS07988
SEQUENCE DESCRIPTION:
GATCGAAAGG GCCACCATGA ACTTGATACG TCCGTGTGTC CCAGATGCTN NCATTAGTCT 60
ATATGGTTCT CCAAGAAACT GAATGAATCC ATTGGAGAAG CGGTGGATAA CTAGCCAGAC 120
AAAATTTGAG ANTACATAAA CAACGCATTG CCACGGAAAC ATACAGAGGN TGCCTTTTCT 180
GTGATTGGGT GGGATTTTTT CCCTNTTTAT GTGGGATATA GTAGTTACTT GTGCCAAGAN 240
TAATTTTGGC ATNATTTCTN TTANTATCAA CTCTGANGCT AACTTGTACT AATCTGAGAT 300
TGTGTNTGNT CCNANTAANC GTGANGTGAA CCTGN 335

SEQ ID NO:6763
SEQUENCE LENGTH:169

SEQUENCE TYPE:nucleic acid
TOPOLOGY:linear
CLONE:HUMGS07989
SEQUENCE DESCRIPTION:
GATCTTGGAG ACAGGCCATC CATCCTGGAG CCTCATGGAA CAGGATGATG GCACTGAGAA 60
AGCCAATGAC CGAATCTCTT TTCTCTGTAA AAATGTAGAC TGAAAAGCCA TGTGTATTTT 120
CCTATGTGCT GTAGCTCTCC TTTGGAAATA AATCACAGGC ATCTGGAAA 169

SEQ ID NO:6764
SEQUENCE LENGTH:212
SEQUENCE TYPE:nucleic acid
TOPOLOGY:linear
CLONE:HUMGS07990
SEQUENCE DESCRIPTION:
GATCTGGGCT GCAGGAGCTG GGGCCACCCA CAGCCCCCCT ACCGACCTGG TGTGGAAGGC 60
ACAGAACACC TGGGGCTGCG GGAACAGCCT GCGTACGGCT CTCATCAAAC TCACTGGGGA 120
AGAAGTGGCC ATGCGGAANT GGTGCGCTCA GTGACTTNTG GTTNAGGACG NCGNGGNTTG 180
AGGATTGAGA NTACCNTATT CATTNACCAC NN 212

SEQ ID NO:6765
SEQUENCE LENGTH:141
SEQUENCE TYPE:nucleic acid
TOPOLOGY:linear
CLONE:HUMGS07991
SEQUENCE DESCRIPTION:
GATCATACCA CTGCACTCCA GCCTGGGCGA CAGAGAACCT CTCTAAAAAA AAAGGAAAAT 60
NAAAAATAAA TGGGGGCAGA AGCTGAGCCA AGCAGAGGCG GGCAGCTCCT CCTCCAGGGC 120
CCACCTGGCC TGCTGGGGAA A 141

SEQ ID NO:6766
SEQUENCE LENGTH:360
SEQUENCE TYPE:nucleic acid
TOPOLOGY:linear
CLONE:HUMGS07992
SEQUENCE DESCRIPTION:
GATCTGTTAT NAAACGAAAC ACCCCCCGTG TTAATAACTT GGTATGAAAT CTGTTTTTAT 60
GAGCCGGGCC CCCTGTGCCT CTAGTATACT TGTATTGACT CTCATAGTTA CCCTTTTAGT 120
TTTACTGTGT TCTGTGAAAA TTTGTAATTG GTTGNGAATC ACTGTGGGCG TCCATTCTTA 180
TTCAACTAAA TCTCCACAGG TTTTTTGAGC TGGTGTGGAT TAGTTTAACT CTTGTATTCA 240
ACCATTAGTG CTACCACCTT CTCACATTAC AATACANTTN CTGGAAGCAA GTACTGCATT 300
TCCTATGCAA CAAAAAAAGG GCAAAANTAC ANANATTGCT AAATGATAAA CAAANACTTN 360

SEQ ID NO:6767
SEQUENCE LENGTH:88
SEQUENCE TYPE:nucleic acid

TOPOLOGY:linear
CLONE:HUMGS07994
SEQUENCE DESCRIPTION:
GATCAAAAGG GCTATGGGAA GGGCAGACCC CGCCAATGAT TTCTCTTCAC CTGTCTTAAG 60
ATTAAATAAA AAAGAGTGTC CTGGCAAA 88

SEQ ID NO:6768
SEQUENCE LENGTH:65
SEQUENCE TYPE:nucleic acid
TOPOLOGY:linear
CLONE:HUMGS07995
SEQUENCE DESCRIPTION:
GATCTTGCCA AGAACCATCA CGTGTTAATT TAGTTGCTTA AACAAAAGGT GTTCTGTTTC 60
TTAAA 65

SEQ ID NO:6769
SEQUENCE LENGTH:246
SEQUENCE TYPE:nucleic acid
TOPOLOGY:linear
CLONE:HUMGS07997
SEQUENCE DESCRIPTION:
GATCAGTGGT GAAATATAGT GATTTTNACC TGTGCTTCCA TTCTGAAGTT CTGGAAAGAA 60
GTACTGGATG GACTGAAGTC CAGGACAACG TTCCAAAGAA AGGCAGAGTC CAGGTAGGCT 120
TGGAGGACCA AGCCCTGGAT GAGCACTGGA GGGCAGAGGC CTCAGTGTCC AGCACTGTGC 180
CCTGCACATG GAAAGCCCCT ACGTTTGTGG AATNAATGAN TANTAAAAAT ATTTTNATNA 240
GTGAAA 246

SEQ ID NO:6770
SEQUENCE LENGTH:23
SEQUENCE TYPE:nucleic acid
TOPOLOGY:linear
CLONE:HUMGS07998
SEQUENCE DESCRIPTION:
GATCCTGTCC CTGCCCTGCC AAA 23

SEQ ID NO:6771
SEQUENCE LENGTH:46
SEQUENCE TYPE:nucleic acid
TOPOLOGY:linear
CLONE:HUMGS07999
SEQUENCE DESCRIPTION:
GATCTGTATA TCCTCCAGTC CAAGATTAAA GAGGCGGACT GTGAAA 46

SEQ ID NO:6772
SEQUENCE LENGTH:108

SEQUENCE TYPE:nucleic acid
TOPOLOGY:linear
CLONE:HUMGS08000
SEQUENCE DESCRIPTION:
GATCCAAACT CATTCTTNCT GTAACCTCAT GATGACGTAT AACCNTTTCC ACCTCACTNG 60
GAGGTTGGNT CTNCATTCTG AAGGCTTCTA TGTATACATA TTAAATAN 108

SEQ ID NO:6773
SEQUENCE LENGTH:218
SEQUENCE TYPE:nucleic acid
TOPOLOGY:linear
CLONE:HUMGS08001
SEQUENCE DESCRIPTION:
GATCAACGAC GACCTTTTTC CTTTTNATAC TAAAGTAAGA AATAAGAATG TNAGCCCAAA 60
CTGCACTATT TTGCAGACCC CTACCATTTT ACAAACTGGT CAGAGTGGAA AATTCCACCA 120
GGGCCTGAGC TGTGAGAAAC ATCCTGTCAG GCAGGNCCCA GGNCTNAACC CCTGGCTGCA 180
CTAANTTNCT TCATNATCAN CAGCCTAACA NACCGTCN 218

SEQ ID NO:6774
SEQUENCE LENGTH:38
SEQUENCE TYPE:nucleic acid
TOPOLOGY:linear
CLONE:HUMGS08002
SEQUENCE DESCRIPTION:
GATCAACAGT GCAGAGAATC TAGCAAGTAT AAAAGAAA 38

SEQ ID NO:6775
SEQUENCE LENGTH:252
SEQUENCE TYPE:nucleic acid
TOPOLOGY:linear
CLONE:HUMGS08003
SEQUENCE DESCRIPTION:
GATCTGCAAC ATGAANCCCG AGGCATGGGT TGGTGTGATG CCAAGGCAAA GTGGTGAGGA 60
GAAAACAGGA AACGGNCTTT CTCTGAATTG GTAAATGGGA AAGAAGTGAG CAACTTAAGA 120
TTGTCACANT TAATCACAAG TGTACAGGAT TAGACTGGGT TTATATTTAA CTCTTGCTTC 180
ATAGGTGTAC CATTTAAAGN GTGTTATNTA NTGCTAAGAA NTAACTGCTT TNATAAAGCT 240
TATTNNNNAA TN 252

SEQ ID NO:6776
SEQUENCE LENGTH:34
SEQUENCE TYPE:nucleic acid
TOPOLOGY:linear
CLONE:HUMGS08004
SEQUENCE DESCRIPTION:
GATCAAAGGG AATAAAGAGA ACTCTTGGCT GAAA 34

SEQ ID NO:6777
SEQUENCE LENGTH:107
SEQUENCE TYPE:nucleic acid
TOPOLOGY:linear
CLONE:HUMGS08005
SEQUENCE DESCRIPTION:
GATCATATNC TNTTTCTNTT TTGTTCCTTA CAATATCAAT CTAATTTAAT ATTCTCTTGT 60
GAGAACACAA ACATNNGTAA ATTGCTCAGT AGTGGCAGCA GTAAGGN 107

SEQ ID NO:6778
SEQUENCE LENGTH:112
SEQUENCE TYPE:nucleic acid
TOPOLOGY:linear
CLONE:HUMGS08006
SEQUENCE DESCRIPTION:
GATCAAGGTT TGTNTGCCCA TTACCTTTCC TCTGCCTGAA AGACGTGTCT CAAGAAAAAT 60
AAATTCTATT TNAGATGCAG GTACTNATAN TAATTCTAAG ANTTGATATC AN 112

SEQ ID NO:6779
SEQUENCE LENGTH:140
SEQUENCE TYPE:nucleic acid
TOPOLOGY:linear
CLONE:HUMGS08007
SEQUENCE DESCRIPTION:
GATCAGAGNA AACGNCTTTA ACNCCACCAT TAGCACCCAA AGCTAAGATT CTAATTTAAA 60
CTATTCTCTG TTCTTTCATG GGGAAGCAGA TTTGGGTACC ACCCAAGTAT TGACTCACCC 120
ATCAACAACC GCTATGTAAA 140

SEQ ID NO:6780
SEQUENCE LENGTH:173
SEQUENCE TYPE:nucleic acid
TOPOLOGY:linear
CLONE:HUMGS08008
SEQUENCE DESCRIPTION:
GATCAATCTC ATATTCATTC CCTGGGATGT TTAGTTACCA GTTTTCCCAA AGTGTTCTGG 60
TAGCATCTAC CATATTTNAT CAAATCTGTG ATTCCTTTGA TTATNATATG AACCATTATT 120
TTATGTATCA TTAAGAAAAA ATACTGCCAA TTAAACTCTG TCATGNNAAC AAA 173

SEQ ID NO:6781
SEQUENCE LENGTH:186
SEQUENCE TYPE:nucleic acid
TOPOLOGY:linear
CLONE:HUMGS08009
SEQUENCE DESCRIPTION:

GATCCAAGTT GAGATTTTAA TTCTGTGACA TTTTTACAAG GGTAAAATGT TACCACTACT 60
TTAATTATTG TTATACACAG CTTTATGATA TCAAAGACTC ATTGCTTAAT TCTAAGACTT 120
TTGAATTTTC ATTTTTTAAA AAGATGTACA AAACAGTNTT GNAATAAATT TNAATTCGNA 180
TATAAA 186

SEQ ID NO:6782
SEQUENCE LENGTH:102
SEQUENCE TYPE:nucleic acid
TOPOLOGY:linear
CLONE:HUMGS08010
SEQUENCE DESCRIPTION:
GATCCATTTC TNATATGTCT TGAACTACTG TGTCTAGTGG GCAAATGTCA TTGTTACCTC 60
TGTGTGTTAA GAAAATAAAA ATATTTNCTA AAGGTCTGTA AA 102

SEQ ID NO:6783
SEQUENCE LENGTH:198
SEQUENCE TYPE:nucleic acid
TOPOLOGY:linear
CLONE:HUMGS08011
SEQUENCE DESCRIPTION:
GATCAAGTAG ATTTTTTAAA AATCAGAGTC AATTAATTTT AATTGAAAAT TTCTCTTATG 60
TTCCAAGTGT ACAAGAGTAA GATTATGCTC AATATTCCCA GAATAGTTTT CAATGTATTA 120
ATGAAGTGAT TAATTGGCTC CATATTTAGA CTAATAAAAC ATTAAGAATC TTCCATAATT 180
GTTTCCACAA ACACTAAA 198

SEQ ID NO:6784
SEQUENCE LENGTH:278
SEQUENCE TYPE:nucleic acid
TOPOLOGY:linear
CLONE:HUMGS08012
SEQUENCE DESCRIPTION:
GATCAGAGTA AAACNTGGAG CTAGATTGAT TACAGAGTGC AAGGACTAGG CACCCCGGGA 60
TTGGAGAGCA TCCTTGAAAG CAAGGGTTCT TCATTTATTT ATAAAAATTT NATTGCCTAC 120
TAGAAGCCCA GCTAAGAGTA TGAAGCAATT AAGACAACAN TCTTTGCTCA AGAATCTCCT 180
AGACAATCCT GATTCAAGCA CCAGAAACAA AGGGNNAAGA AACCTCCCAG TCTNGTGNNG 240
NAAGTNGTAC ACATCNTGTC AACAGNTAGG TTNTTTTN 278

SEQ ID NO:6785
SEQUENCE LENGTH:339
SEQUENCE TYPE:nucleic acid
TOPOLOGY:linear
CLONE:HUMGS08013
SEQUENCE DESCRIPTION:
GATCCCATGT NAGCTACACA GTGCAGAGGC TCTTGATGGT GGACTAAGCA ATTTCCTCCC 60
TCGTGCGCAT CTCAGAACCC ATCGGTAGGC AAAGGAAAAT ACGCTCAGGT GGTTGTGGNN 120

NAGNCTGTNT CAGGCCTACG GAGTCAGCCA GTGGCTAGCG CAAGACCAGT CACTCCCTCT 180
GCNTTCAGGC TTCTTTCAAT TTCATTATCA TCAAGCAGGA ATTATGTCGT AAGTCACTGA 240
CCCTAACTGC AGACCATGAA GTAAATTATG TAACTAGGTT TTTNNCTTCT CCAGTGGTGA 300
CCACCNNCNC NCATNCCCCG GTNANAACTT GGGTTTTTN 339

SEQ ID NO:6786
SEQUENCE LENGTH:80
SEQUENCE TYPE:nucleic acid
TOPOLOGY:linear
CLONE:HUMGS08014
SEQUENCE DESCRIPTION:
GATCGCCTAT TACTGACAGT CTCATTGTAG CTCTAAAAAG CCTAATGTAT CCACTGTGGA 60
ATAAACTCCA TAGACTCAAA 80

SEQ ID NO:6787
SEQUENCE LENGTH:279
SEQUENCE TYPE:nucleic acid
TOPOLOGY:linear
CLONE:HUMGS08015
SEQUENCE DESCRIPTION:
GATCTTGAAG AGACCGCTGG CAGCACCAGT ATTCCCAAGA GGAAGAAGTC TACACCCAAG 60
GAGGAAACAG TTAATGACCC TGAGGAGGCA GGCCACAGAA GTGGCTCCAA GAAAAAGAGG 120
AAATTNTCCA AAGAGGAGCC GGTCAGCAGT GGGCCTNNAG AGGCGGCTNG CAAGAGCAGC 180
TCCAAGAAGG NAGGAAAAAG TTCCNTTAAG CATTNCCNGG GAGNTTTGAA TTCAAAATTG 240
GGCATTNTTN TTTGGGGGTT GGGNNATTCC CTTGGCCCN 279

SEQ ID NO:6788
SEQUENCE LENGTH:192
SEQUENCE TYPE:nucleic acid
TOPOLOGY:linear
CLONE:HUMGS08016
SEQUENCE DESCRIPTION:
GATCCACTCA CCTCGGCCTC CCAAAGTGCT GGGATTACAG TGCCCGTCCG TGATGATGGG 60
AACATGNCCG ACGTGCCCAG NCACCCCCAG GACCCTCAGG GCCCCAGNCT GGAGTGGCTG 120
AAGAAACTGT GAGCACCTCC ACTGACAGAG GCGGCCCCTC CCACGGCTCC CAATAAAAAT 180
GTGAAAACCA AA 192

SEQ ID NO:6789
SEQUENCE LENGTH:303
SEQUENCE TYPE:nucleic acid
TOPOLOGY:linear
CLONE:HUMGS08017
SEQUENCE DESCRIPTION:
GATCCGTATC GNTTTAAGAA GCGGACGGAG CTGTTCATTG CCGNCGAGGG CATTCACACG 60
GGCCAGTTTG TGTATTGCGG CAAGAAGGCC CAGCTCAACA TTGGCAATGT GCTCCCTGTG 120

GGCACCATGC CTGAGGGTAC AATCGTGTGC TGCCTNGAGG AGAAGCCTGG AGACCGTGGC 180
AAGCTGGCCC GGGCATCANG GGACTATGCC ACCGTTATCT CCCACAACCC TTNGACCAAG 240
AAGNCCCGTT TTGAANGTTG CCCTNCGGTT TCCAAAGAAN GTTTATCTCC TTAANGCCAA 300
TCN 303

SEQ ID NO:6790
SEQUENCE LENGTH:267
SEQUENCE TYPE:nucleic acid
TOPOLOGY:linear
CLONE:HUMGS08018
SEQUENCE DESCRIPTION:
GATCTAAAAA TCAATGAGAA CATAATTTTA ATTGGCAGTT CTTCAGAAAA ATGTGAATGG 60
CTTATAAACA TAAATGTAAT ACTCAGCTGC TGTCATCATC AGAAGAATGA AGTTGTTTTT 120
AAGCTCTTCA NCTGTCAAGN GTAAGAAGCA ATGTTACTAA TACCGGCAAG NTATGAAAAG 180
ACGCTCATAC ATTGTTGGAA ATNAANGTTG GTATNGCTTT TCAGCGGCCN ATNAACATAA 240
TCCCNNTNTT TCTTCCCTAC NNACTCN 267

SEQ ID NO:6791
SEQUENCE LENGTH:189
SEQUENCE TYPE:nucleic acid
TOPOLOGY:linear
CLONE:HUMGS08019
SEQUENCE DESCRIPTION:
GATCATCTGG CAAGACAGCC CAAGAATAAA GCCGTCACCA TGGAGACTAA GAAGNGNCAG 60
AAAGNCACTG GGTCTTCATT GTNTCGTCCA GCTGTTCATG AGGCCTTATC TGAAGCCCAC 120
GTGACCTCCG GNCTACTCAC TTACATGCGN GAATAAAAGC TCTATTTTGT TTTAGGNCCN 180
AAATAANTN 189

SEQ ID NO:6792
SEQUENCE LENGTH:129
SEQUENCE TYPE:nucleic acid
TOPOLOGY:linear
CLONE:HUMGS08020
SEQUENCE DESCRIPTION:
GATCTGCTGG CAGAGGNCAA TCAAAAACGA CAACAAGCTT CTTCCCAGGG TGAGGGGAAA 60
CACTTAAGGA ATAAATATGG NGCTGGGGTT TAACACTAAA AACTAGAAAT AAACATCTCA 120
AACAGTAAA 129

SEQ ID NO:6793
SEQUENCE LENGTH:267
SEQUENCE TYPE:nucleic acid
TOPOLOGY:linear
CLONE:HUMGS08021
SEQUENCE DESCRIPTION:
GATCAGGACC CACCTCCAGT TCTNCTGAAA GTNTGACAGT GTCCAGCCGG TTCTGCAGCA 60

CTAGGGGAGG GGGCAGATGG TGGTTGCATG GGCTTCCTGG GTCTCCACTC TCCGTCTGGC 120
CTAAAGGTGA TGTATTTGGT GTTTGGNCCT GCAGTCCCCA CTNTTAAGGC TTAAGGCGGA 180
TGTGGAACAN CACTTTCTTT CAGGAGTAGG TNGNTNTACT TGTACCTCAT TTAGGGCTTA 240
GAAAGTTTTN CCNCANACTN NAAAAAN 267

SEQ ID NO:6794
SEQUENCE LENGTH:122
SEQUENCE TYPE:nucleic acid
TOPOLOGY:linear
CLONE:HUMGS08022
SEQUENCE DESCRIPTION:
GATCTACAAA ACAGATAGNG NAAAGTACAA CAGAATAGCT CGGGAATGGN CTCAGAAGTA 60
TGCGATGTAA TTAAAGAAAT TATTGGATAA CCTCTACAAA TAAAGATAGG GGNACTCTGA 120
AA 122

SEQ ID NO:6795
SEQUENCE LENGTH:56
SEQUENCE TYPE:nucleic acid
TOPOLOGY:linear
CLONE:HUMGS08023
SEQUENCE DESCRIPTION:
GATCCCACTT GTAGATGCTT GCACTCACAC AATAAAAACG TCTGNTATTT GCCAAA 56

SEQ ID NO:6796
SEQUENCE LENGTH:130
SEQUENCE TYPE:nucleic acid
TOPOLOGY:linear
CLONE:HUMGS08024
SEQUENCE DESCRIPTION:
GATCCGTGGT TCCCTGCAGA CTGGCAGGAG CAGATTCCAA AGGCACAGGA AGAAGCTTGC 60
AGGTAGAATG TGTTCATTAC CTTCTGCGCA TTATACCACA AAAAAGCTGG GNATAAAAAT 120
GCTAACCAAA 130

SEQ ID NO:6797
SEQUENCE LENGTH:234
SEQUENCE TYPE:nucleic acid
TOPOLOGY:linear
CLONE:HUMGS08025
SEQUENCE DESCRIPTION:
GATCTGCCCC ATGATTGGTT CCTTAGGANC AAGNAATTTA CAAGTAGNAA TTATTCCTTT 60
CAGAGTAACA TGCTGTATTA CTTCAATCCC TATTTTTGTC TGTTCCATTT TCTTTGGATT 120
CCCTATTCAC ATTGAATCCT NTTTGCCCTT CTGAAACAAT ATTCAGTCAC AAGNGNTTTT 180
GGTCATGTTG GTCTTTGTAA CAAATNAAAN TTACCTTATA TCCTTCTGGA CAAA 234

SEQ ID NO:6798

SEQUENCE LENGTH:246
SEQUENCE TYPE:nucleic acid
TOPOLOGY:linear
CLONE:HUMGS08026
SEQUENCE DESCRIPTION:
GATCACAAGG TCAGGAGTTC GAGACCAGCC TGGCCAATAT GGTGAACCTC ATCTCTATTG 60
AAAATACAAA AATCAGCCAG GCACGGTGGC AGGCGCCTGT AGTCCACCTA CTCGGGAGGC 120
TGAGGCAGGA GAACTGCTTG AACCCAGCGG CAGAGGTTGC AGTGAGCCAA GATTGAGCCA 180
CTGCACTCCA GCCTGGTGNT AGNGCGNGCT CTGTCTCTAA ATAATNANNT AAANTNCNTT 240
AANNNN 246

SEQ ID NO:6799
SEQUENCE LENGTH:114
SEQUENCE TYPE:nucleic acid
TOPOLOGY:linear
CLONE:HUMGS08027
SEQUENCE DESCRIPTION:
GATCAGTGTT AAACTAGATT GTATTCATTA CTAGATAAAA TGTATAAAGC TCTCTGTACT 60
AAGGNGAAAT GACTTTTATA ACATTTTGAG NAAATAATAA AGCATTTATC TAAA 114

SEQ ID NO:6800
SEQUENCE LENGTH:274
SEQUENCE TYPE:nucleic acid
TOPOLOGY:linear
CLONE:HUMGS08028
SEQUENCE DESCRIPTION:
GATCTTTTGT GCCAAGATGC AGGGAAATNA AGAAAGCTCT TTCCAGCTGT TTTNATGGAT 60
TCATGGAGTG GGCTCATGTT GGGACCAGCT GACTCTGGGN GGAAGACTGC CTCTCCATCG 120
CTGTCAGAGA GCCTGAAAGG ACCAACAATG AGNTGTTCTT GGGACCCGCC ATGGGGATGA 180
TTGCTTCTCT AGGCTNCTGG CTGTTGCTGC ATTCTAAGGN TTAACCTCCT GGTCTNATGG 240
TAGTGANCTT GAGCTTTTNN ATTCNATAGN AGNN 274

SEQ ID NO:6801
SEQUENCE LENGTH:42
SEQUENCE TYPE:nucleic acid
TOPOLOGY:linear
CLONE:HUMGS08029
SEQUENCE DESCRIPTION:
GATCTGTGNA GNAATGAAAT AAAATGGAGA AGAAGACAGA AA 42

SEQ ID NO:6802
SEQUENCE LENGTH:228
SEQUENCE TYPE:nucleic acid
TOPOLOGY:linear
CLONE:HUMGS08030

SEQUENCE DESCRIPTION:
GATCCAGTTT CAAGAATGGG CAGGTAAACG CAGNANGAGG AAAGGAATGT GGAATGAGAA 60
CTTGGTGGTT CACCGCTGTA CTATTTGTGT AAATGTTTAC GTATGTNATA AGCTACATGT 120
ATGTAAATNT TGCAATACCC CTAACAGTCG AGTAGTAGTC TCCCTTACAG GAATTTTTGA 180
CGGGGTTCCT CATCATCAAT ACCAAATAAN TATATGTAGG NNTGGAAA 228

SEQ ID NO:6803
SEQUENCE LENGTH:180
SEQUENCE TYPE:nucleic acid
TOPOLOGY:linear
CLONE:HUMGS08031
SEQUENCE DESCRIPTION:
GATCATATTC TTGAAATTTT TATAAATATG TATGGAAATT CTTAGGCTTT TTTTACCAGC 60
TTTGTTTACA GACCCAAATG TAAATATTAA AAATAAATAT TTGCAATTTT CTACAGAATT 120
GAATACCTGT TAAAGGAAAA TTACAGAATA ANCTTGTGAC TGGTCTTGTT TTACATTAAA 180

SEQ ID NO:6804
SEQUENCE LENGTH:277
SEQUENCE TYPE:nucleic acid
TOPOLOGY:linear
CLONE:HUMGS08032
SEQUENCE DESCRIPTION:
GATCATTCCG AGGTCATTTG AGGACGCACA CTGGAGAGAA GCCTTATNAG TGTAAACAAT 60
NTGGAAAAAC CTTCACTTGG TNCTCAACGT TTAGAGAACA TGTNAGAATT CACACGCAAG 120
AGCAGCTCCA TAAATGTGAA CACTGTGGGA AGGCCTTTAC CTCTTCCAGA GCATTCCAAG 180
GTCATTTGAG GATGCACACT GGAGAGAAGC CTTATGAGTG TACACAATGT GGANAAACCT 240
TCACTNGGGG CCTCAANCCT TACNTAATAA TGTGGTN 277

SEQ ID NO:6805
SEQUENCE LENGTH:273
SEQUENCE TYPE:nucleic acid
TOPOLOGY:linear
CLONE:HUMGS08033
SEQUENCE DESCRIPTION:
GATCTACCTC AGTATTCAAG TTCAGTGGGG ACACCAGTGG CTTCAAACTT CCTGGTTTCA 60
TGATATCTTG AGACGCCTTA CAAATGATGG AGGATTCCAA AGAGTTTTTG TTTATTTGGG 120
TTAATATTTG TTGGTATTTA TGGCATTTGA GATTGAAACT AAGNAATGTT TTAATTTATT 180
ACCTTTACAA CATTTATTTA CATTACATAC ATACATTTAC AACATTTATT AATTTATNTT 240
AAAATAGCAT GNATAAGCCA ATTATNGGTT AGN 273

SEQ ID NO:6806
SEQUENCE LENGTH:207
SEQUENCE TYPE:nucleic acid
TOPOLOGY:linear
CLONE:HUMGS08034

SEQUENCE DESCRIPTION:
GATCCACTCT TCTCGNCCTC CCAAAGTGTT GGGATTATAG GCGTGAGCAC ANGCGCCCGG 60
CCTGNTNTGG ATATTTCTTA TAAATGGAAT CATACAGTGC TTNTTTCAGT TAGCATAATG 120
TTTCCCAGGT TCATCCAGGT CATAACACAT AATCAGGCTT CATTTNTTTT TATTACCANC 180
TAATATTCTA TGAATATAAA NNNACCN 207

SEQ ID NO:6807
SEQUENCE LENGTH:273
SEQUENCE TYPE:nucleic acid
TOPOLOGY:linear
CLONE:HUMGS08036
SEQUENCE DESCRIPTION:
GATCCTGAAG AGATTGAAAA AGAAGAGCAG GCTGCTACTC AGCCTGAGGT TGCAGACTGG 60
TCTGAAGGTG TACAGGTGCC CTCTGTGCCT ATTCAGCAAT TCCCTACTGA AGACTGGAGC 120
GCTCAGCCTG CCACGGAAGA CTGGTCTGCA GCTCCCACTG CTCAGGCCAC TGAATGGGTA 180
GGAGCAACCA CTGACTGGTC TTAAGCTGTT CTTGCATAGG CTCTTAAGCA GCATGGNAAA 240
ATGGTTGCTG GCAAATAAAC ATCAGTTTCT AAA 273

SEQ ID NO:6808
SEQUENCE LENGTH:73
SEQUENCE TYPE:nucleic acid
TOPOLOGY:linear
CLONE:HUMGS08037
SEQUENCE DESCRIPTION:
GATCTCCAAC ATCTTTGTGT TGAAGGGCCT ACAGAGTAAA CTGTGGGACT CAATAAACAT 60
TCGTTACTTG AAA 73

SEQ ID NO:6809
SEQUENCE LENGTH:285
SEQUENCE TYPE:nucleic acid
TOPOLOGY:linear
CLONE:HUMGS08038
SEQUENCE DESCRIPTION:
GATCACAGCA GACAGGGTCG CTGCCCTGAT GGCGCTTACA TTCCAGTGGG TCTAATGACC 60
ATATCTTAGG ACACAGATGT GCCCAGGGAG GTGGTGTCAC TGCACAGGAA GTATGAGGAC 120
TTTAGTGTCC TGAGTTCAAA TCCTGATTCA GGAACTCACA AAGCTATGTG ACCTTACACC 180
AGTCACTTAA CTTGTTAGCC ATCCATTATC GCATCTGCAA AATGGGGATT AAGAATAGAA 240
TCTTGGGGTT AGTGTGGNGA TTAGATTAAA TGTATGTAAG GCACN 285

SEQ ID NO:6810
SEQUENCE LENGTH:62
SEQUENCE TYPE:nucleic acid
TOPOLOGY:linear
CLONE:HUMGS08039
SEQUENCE DESCRIPTION:

GATCAGTTTC TGGCACATAA ATGCCTCAAT AAAGATTTAA TTACTTTGTA TAGTGCTTTA 60
AA 62

SEQ ID NO:6811
SEQUENCE LENGTH:237
SEQUENCE TYPE:nucleic acid
TOPOLOGY:linear
CLONE:HUMGS08040
SEQUENCE DESCRIPTION:
GATCATTGGC TATGACAGAG AACCCGTCTC TCAAGCAGCA GCTCTTCTCC TATGCCATTC 60
TTGGCTTTGC CTGTCTGAGG CCATGGGGCT TTTNTGTTTG ATGGTCGCCT TCCTAATCCT 120
NTTCGCCATG TGAGGNTCCA TGGGGGGTCA CCGGANCTGT TGATACTGCA ACTACACACC 180
ATTNTTGGTG CTGGGGTGTG TTAAGATTTA ACCATTNANC ACAACGTTTC TCTNAAA 237

SEQ ID NO:6812
SEQUENCE LENGTH:215
SEQUENCE TYPE:nucleic acid
TOPOLOGY:linear
CLONE:HUMGS08041
SEQUENCE DESCRIPTION:
GATCAGCAAA TTTAAGAGAA GACCTAGCTA TGTTGGCTGG TTGCTTTCTA TTATCATGGT 60
ATTTGACCAT TTTAGTTTTA ATTCCATGTC AGATAAGTGT AAATAGAAGA GTTTAAAAGC 120
ATGANACATT TCAGANGGTA TCAGTTATAT GATATTCTTT AANCANNTAT GACACATGTA 180
AATCCTCNTG ANTGACAATN CATCTNTNTG TGAAA 215

SEQ ID NO:6813
SEQUENCE LENGTH:181
SEQUENCE TYPE:nucleic acid
TOPOLOGY:linear
CLONE:HUMGS08042
SEQUENCE DESCRIPTION:
GATCTAAAAG CCATATCCTG TCTTTGGTGG TGGCCTGGTA GGAATGGCTT GTNCTAGTTC 60
TTCAAAGGTA AGATTGACCA AAGCAAGTTC TCATTGGTGG TCTGTGTTTA CATCTTCCGT 120
GTCAAGNTAT TTTAAAGCAT GNAAATAAAG CCATTCTTGC CCTTTTTCGT GTGTTTGCAA 180
A 181

SEQ ID NO:6814
SEQUENCE LENGTH:162
SEQUENCE TYPE:nucleic acid
TOPOLOGY:linear
CLONE:HUMGS08043
SEQUENCE DESCRIPTION:
GATCTCAGGC TTCTGCAGTT CTCATGACTC CTACTTTTCA TCCTAGTCTA GCATTCTGCA 60
ACATTTATAT AGACTGTTGA AAGGAGAATT TGAAAAATGC ATAATAACTA CTTCCATCCC 120
TGCTTATTTT TAATTTGGGA AAATAAATAC ATTCGAAGGA AA 162

SEQ ID NO:6815
SEQUENCE LENGTH:431
SEQUENCE TYPE:nucleic acid
TOPOLOGY:linear
CLONE:HUMGS08044
SEQUENCE DESCRIPTION:

```
GATCTCAGCG GGGAGCCACG TCTCTTGCAC TGTGGTCTCT GCATGGACCC CAGGGCTGTG  60
GGGACTTGGG GGACAGTAAT CAAGTAATCC CCTTTTCCAG AATGCATTAA CCCACTCCCC 120
TGACCTCACG CTGGGGCAGG TCCCCAAGTG TGCAAGCTCA GTATTCATGA TGGTGGGGGA 180
TGGAGTGTCT TCCGAGGTTC TTGGGGGAAA AAAAAATTTN TAGCATATTT AAGGGAGGCA 240
ATGAACCCTN TCCCCCANCT NTTNCCNGCC CAAATNTGTC TCCTAGNATC TTATGTGCTG 300
GGAATAATAG GCCTTNACTG CCNTCNNGNT TTATAGGCCN ANGGGTCCCG GTTTCCCGGG 360
AACTGGAACC CTTCCTGAGG GGGAATCCCG GGGCTCAAAT TACCCCCNAA AGGAAAGTGG 420
CCAAGGCGGT N                                                      431
```

SEQ ID NO:6816
SEQUENCE LENGTH:231
SEQUENCE TYPE:nucleic acid
TOPOLOGY:linear
CLONE:HUMGS08045
SEQUENCE DESCRIPTION:

```
GATCTGTTTC TAAAGCGTTA CGCATCTTTN CTGAAGTATG CTGGAGAGCC TGTCCCTTTC  60
ATTGAACCCC CTGAAAGCTT TGAATTTNAT GCACAGCAGC TAAGAAAATT GAGGGAAAAC 120
TCTTCTTGAA ATAACCAGGC GATACTTTGT TTTGTATATA TTTGTGATTC TGTGTCTACA 180
TGTTATTTTG AAGTATATCT GAGGGAANNG GGAATGGAAA TTTTCTTTAA A          231
```

SEQ ID NO:6817
SEQUENCE LENGTH:147
SEQUENCE TYPE:nucleic acid
TOPOLOGY:linear
CLONE:HUMGS08046
SEQUENCE DESCRIPTION:

```
GATCCTTCCA GCTGCTCTTC CATAACTTCA TCTCTCCCCT CAACACCTGC AACCACTAAG  60
CCATTTTCCC CCTACCCGTC TGTAATTTTG TAATTTTGAG AATGCCAGGT AAATGGAATC 120
AGAGCGGATA ATCTTTGGAG CATGAAA                                     147
```

SEQ ID NO:6818
SEQUENCE LENGTH:101
SEQUENCE TYPE:nucleic acid
TOPOLOGY:linear
CLONE:HUMGS08047
SEQUENCE DESCRIPTION:

```
GATCATTTAT ATTTAAGTTT AAAAGGAGGN TGAGACTGAG TTTTTGTAGA GTTATAATNA  60
CATTCAAAAT ATACTTTTTT GCATTGNNAA AATGTTAANT N                     101
```

SEQ ID NO:6819
SEQUENCE LENGTH:236
SEQUENCE TYPE:nucleic acid
TOPOLOGY:linear
CLONE:HUMGS08048
SEQUENCE DESCRIPTION:
GATCAAGCCA AACAGTAAAA ACTACCAAGA GAACACGAGG AAGGCAGAAA CGNTGTTTAG 60
CAACAGTATT CTGCATGGTT CACTGCTTAA GAAAATGCCT TCTGGAATAT TTGTAAACTG 120
AAATTCTGTA TGTGTAAGNG NTGTTTAAAT ATGTTTGGGT CCTAAACAGC TTTTTAAAAT 180
TATACTTGGG CNTAANTTCA GCATCTTTTC AAATCANCTC TNTGCNCCGC CGCTGN 236

SEQ ID NO:6820
SEQUENCE LENGTH:195
SEQUENCE TYPE:nucleic acid
TOPOLOGY:linear
CLONE:HUMGS08049
SEQUENCE DESCRIPTION:
GATCATTTTG TTTGTTGCTC TCTCTAGTGT CTTCTTCTCT CGTCAGTCTT AGCCTGTGCC 60
CTCCCCTTAC CCAGGCTTAG GNTTAATTAC CTGAAAGATT CCAGGAAACT GTAGCTTCCT 120
AGCTAGTGTC ATTTAACCTT AAATGCAATC AGGAAAGTAG CAAACAGANG TCAATAAATA 180
TTTTTAAATG TNAAA 195

SEQ ID NO:6821
SEQUENCE LENGTH:160
SEQUENCE TYPE:nucleic acid
TOPOLOGY:linear
CLONE:HUMGS08050
SEQUENCE DESCRIPTION:
GATCATAAAT ATTTTGTAAT GAAATACTTC CNCTTTTCCA GGGCTTTGTA TGCACTTGTA 60
TAATTACATT GATGGCAATG TAGNGTTTGA ATTTCAGTCT GTAAATACTT TTNTGGNAAA 120
TAGNAATTTT NATTGCTTTA AAGTTTTGGA TATGGGTGGN 160

SEQ ID NO:6822
SEQUENCE LENGTH:157
SEQUENCE TYPE:nucleic acid
TOPOLOGY:linear
CLONE:HUMGS08051
SEQUENCE DESCRIPTION:
GATCTATAGG AAGATTGAAC CTGAATATTG CCATTATGCT TGACATGGTT TGCAAAAAAT 60
GGTACTCCAC ATACTTCAGT GAGGGTAAGT ATTTTCCTGT TGTCAAGAAT AGCATTGTAA 120
AAGCATTTTG TAATAATAAA GNATAGCTTT AATGAAA 157

SEQ ID NO:6823
SEQUENCE LENGTH:150

SEQUENCE TYPE:nucleic acid
TOPOLOGY:linear
CLONE:HUMGS08052
SEQUENCE DESCRIPTION:
GATCCAGNTT GTTGCATCAA TAGTTCTGTT TTTAGTACTG AGTGGGATGT CAGTATAGAT 60
GTACCATAGT AAACACCTGT GGAAGGACGT CAGGAATGTT TCCTGTTATT AGGTATCTAA 120
ATAAAGTTGC TGTAAACATT TGTGTACAAA 150

SEQ ID NO:6824
SEQUENCE LENGTH:268
SEQUENCE TYPE:nucleic acid
TOPOLOGY:linear
CLONE:HUMGS08053
SEQUENCE DESCRIPTION:
GATCTGTCCA CTTTGCTCTC ACGGACGTGG CATTTGCCAG GAAGGAGAAC CCAGCAGGAA 60
CGGAGGTCTC TGCCTTCACC TACAGTGAAG GGGCTGGTAT GTGAAATACT TTCACTGAGC 120
TTAGTGAAAA ATTCATTTTA CACCCTAGTT AGCTCCCAGA AAGGTTNAAC TTTCAAGANG 180
GTCAAAATAT GNAGCCTGTT ATATGTGTAA TTAAAAAAGN NNTAGNATAT CCCAAATGGN 240
ACATTTCAGG NATTAANTAA TTGNAATN 268

SEQ ID NO:6825
SEQUENCE LENGTH:197
SEQUENCE TYPE:nucleic acid
TOPOLOGY:linear
CLONE:HUMGS08054
SEQUENCE DESCRIPTION:
GATCTGAGAA GTGAGTGTAA GGAGTTTAAA TCAACCTCTG TTCTGTGCTA GTTAAGGTAA 60
TAAAGTTGCT GCAGTCCAGG GGGTAGGTAC CTGTGGACGG CTCTGCGAAT AGGNCAGTTG 120
CATTTCTTGG GAATCAAGTG CATCCCTAGG CTGGCAGTGC AGCAGAAATA CTGAATAAAA 180
TGTGACAAAT CTCCAAA 197

SEQ ID NO:6826
SEQUENCE LENGTH:196
SEQUENCE TYPE:nucleic acid
TOPOLOGY:linear
CLONE:HUMGS08055
SEQUENCE DESCRIPTION:
GATCAAGTAA AAATGAAGGA CAACGTGAAG TTTAAAGTTC GATGCAGCAG ATACCTTTAC 60
ACCCTGGTCA TCACTGACAA AGAGAAGGCA GAGAAACTGA AGCAGTCCCT GCCCCCCGGT 120
TTGGCAGTGA AGGAACTGAA ATNAACCAGA CACACTGATT GGAACTGTAT TATATTAAAN 180
TNCTAAAAAT CCTAAA 196

SEQ ID NO:6827
SEQUENCE LENGTH:213
SEQUENCE TYPE:nucleic acid

TOPOLOGY:linear
CLONE:HUMGS08056
SEQUENCE DESCRIPTION:
GATCATCAGT ACTGTTGTCT CATGTAATGC TAAAACTGAA ATGGTCCGTG TTTGCATTGT 60
TAAAAATNAT GTGTGAAATA GAATGAGTGC TATGGTGTTG AAAACTGCAG TGTCCGTTAT 120
GAGTGCCAAA AATCTGTCTT GAAGGCAGCT ACACTTTGAA GTGGTCTTTG AATACTTTTA 180
ATAAATTTAT TTTGATAAAT AATATTGAAC AAA 213

SEQ ID NO:6828
SEQUENCE LENGTH:133
SEQUENCE TYPE:nucleic acid
TOPOLOGY:linear
CLONE:HUMGS08057
SEQUENCE DESCRIPTION:
GATCGTCCTG CAGGTATTTT GCAAGCCCCT ACTGTCCGCC GACATCAGCG TGAATCTCAC 60
ATGGTGCACT TGGCTCCCTG GGGGAGTNTT NGCNTCTCCT GTTGTCTTTT CCCAAAACCA 120
NTAAAAAACCA TGN 133

SEQ ID NO:6829
SEQUENCE LENGTH:269
SEQUENCE TYPE:nucleic acid
TOPOLOGY:linear
CLONE:HUMGS08058
SEQUENCE DESCRIPTION:
GATCTGCTAC ATTTTAAAAG TGGGCATGAA AATCTTCTGT TATGTATGTA CTGTCATTAA 60
TTCTGTTATT TTTNCCCACT AGTACTATCA ACTACAGTAA AGACCTTCCT CTGGCCCAAG 120
GAATCAAGTT TCAGTAAAGG TGTTTTAGAT GANCATCCCT TAATTTGAGG TGTTCCAGCA 180
GCTGTTTTTG GNGANGACAA AGNAAATTAA NGTTTTCCCT GAATAANTGC ATTATTNTGA 240
CTGTGACAGT GNCTAATCCC CCTATGGTN 269

SEQ ID NO:6830
SEQUENCE LENGTH:297
SEQUENCE TYPE:nucleic acid
TOPOLOGY:linear
CLONE:HUMGS08059
SEQUENCE DESCRIPTION:
GATCTATCCA ATAAAGGAGA GAAACAACCG CACACGCCTG GCTCTCATCA TATGCAATAC 60
AGAGTTTGAC CATCTGCCTC CGAGGAATGG AGCTGACTTT NACATCACAG GGATGAAGGA 120
GCTACTTGAG GGTCTGGNCT ATAGTGTAGA TGTAGAAGAG NATCTGACAG CCAGGGATAT 180
GGNGTCAGCG CTGAGGNCAT TTGNTACCAG ACCNGNGCAC AAGTCCTCTG NCAGCACATT 240
CTTTGGTACT CATGTNTCNT GGCATCCTGG NGGGGTNTCT TGCGGAACTT GTTCTGN 297

SEQ ID NO:6831
SEQUENCE LENGTH:174
SEQUENCE TYPE:nucleic acid

TOPOLOGY:linear
CLONE:HUMGS08060
SEQUENCE DESCRIPTION:
GATCATGGTG GTTTGGCAGC AGGGAATTTG TCTTGTTGGA GCCTGCTCTG TGCTCCCCAC 60
TCCATTTCTC TGTCCCTCTG CCTGGGCTAT GGGAAGTGGG GATGCAGATG GCCAAGCTCC 120
CACCCTGGGT ATTCAAAAAC GGCAGACACA ACATGTNGCT CCACGCGGCT CAAA 174

SEQ ID NO:6832
SEQUENCE LENGTH:253
SEQUENCE TYPE:nucleic acid
TOPOLOGY:linear
CLONE:HUMGS08061
SEQUENCE DESCRIPTION:
GATCAATTTT GGAGACCTTG GCCGCCCAGG ACGTGGCGGC AGGGGAGGAC GAGGTGGACG 60
TGGGCGTGGT GGGCGCCCAA ACCGTGGCAG CAGGACCGAC AAGTCAAGTG CTTCTGCTCC 120
TNATGTGGAT GACCCAGAGG CATTCCCAGC TCTGGCTTAA CTGGATGCCA TAAGACAACC 180
CTGGTTCCTT TGTGAACCCT TCTGTTCAAA GCTTTTGCAT GCTTAAGGAT TCCAAACGNC 240
TACTGTAATT AAA 253

SEQ ID NO:6833
SEQUENCE LENGTH:86
SEQUENCE TYPE:nucleic acid
TOPOLOGY:linear
CLONE:HUMGS08062
SEQUENCE DESCRIPTION:
GATCATGAAC CAAAGGAATT TATGTTTTGT AACTTGGGTA CTTTATTTTG CATTTTGTTA 60
TACTATTAAA TANTTTTNTC CTGAAA 86

SEQ ID NO:6834
SEQUENCE LENGTH:124
SEQUENCE TYPE:nucleic acid
TOPOLOGY:linear
CLONE:HUMGS08063
SEQUENCE DESCRIPTION:
GATCCAAGTT TCACTTTTTG TGATATAATG AGCCAGTTTT CTCAGTAATG TCTACTAAAA 60
AAAAAGCCAT TAACCNGGNT CTAATATTTG CATGATTCTA ATAAANGTAT GTCAACTTCT 120
TAAA 124

SEQ ID NO:6835
SEQUENCE LENGTH:107
SEQUENCE TYPE:nucleic acid
TOPOLOGY:linear
CLONE:HUMGS08064
SEQUENCE DESCRIPTION:
GATCCCCTCA GAAGGCAGGA GTGCTGCCCT CTCCCATGGT GCCCGTGCCT CTGTGCTGTG 60

TATGTGAACC ACCCACGNGA GGGNATAAAC CTGGCACNNG GACCAAA 107

SEQ ID NO:6836
SEQUENCE LENGTH:125
SEQUENCE TYPE:nucleic acid
TOPOLOGY:linear
CLONE:HUMGS08065
SEQUENCE DESCRIPTION:
GATCCTCCTG TCTTGGTTTC CCAAAGTGCT GGGATTGCAG GTGTGAGCTA CTGCACCCGG 60
CCCAATAAAA AAATCTCGTT CTGTGAAAGA AATGTTTAAT AAAAGTTGTA TTGTCAAATT 120
TTAAA 125

SEQ ID NO:6837
SEQUENCE LENGTH:178
SEQUENCE TYPE:nucleic acid
TOPOLOGY:linear
CLONE:HUMGS08066
SEQUENCE DESCRIPTION:
GATCACTAAG ATTAGCTATA TCTATACAGT CATTAGTTTG ACAAGAAATA GNATCCTGTC 60
AGATGCCAAA GAGTGGGATT TTNATGTTTA ATGATTAAAC ACCATTATTT ATTGACAATT 120
TACCCTGTGG AACTGTATTA TTTCTAACTA TGAAATAAAG GGGTGATGTA AACACAAA 178

SEQ ID NO:6838
SEQUENCE LENGTH:222
SEQUENCE TYPE:nucleic acid
TOPOLOGY:linear
CLONE:HUMGS08067
SEQUENCE DESCRIPTION:
GATCCCCGGC CAAGCCCAGT CACGNGAATT TAAGGCTCTT CTCTCCGGCT CCAGCCTGTG 60
CCTGTAATTG TCCAGGANTG GCCTAGGCCG ACACCTTCTG CTGCCCCGGA GTCCCCTCCC 120
TGGGTGGGAT GCTCACTACA GCTCTGATGT ATATAGCACA CAGCCCAANT CCGACCCTCC 180
GGTCTGTCGC CAATAAAGNG GGGTGTGTTT CANTCCGGGA AA 222

SEQ ID NO:6839
SEQUENCE LENGTH:198
SEQUENCE TYPE:nucleic acid
TOPOLOGY:linear
CLONE:HUMGS08068
SEQUENCE DESCRIPTION:
GATCCCAACA GGCCCTGGCA GCGTCTGGAC ATGTGTAAAC AGCAGCAGTC ACGTTCCTCG 60
GCTGNCACAA CCAACACNNN AGCAGGTTTT CCATTTTCTG TACTTTNATA TTTCTGTTCA 120
ACCTGTTGGT TTCTACAATG ATTTTAAACA TTGGNAAGNC AGCCTTGNGT ATATTTTTAA 180
AAATTATATT NAAAATGN 198

SEQ ID NO:6840

SEQUENCE LENGTH:284
SEQUENCE TYPE:nucleic acid
TOPOLOGY:linear
CLONE:HUMGS08069
SEQUENCE DESCRIPTION:
GATCATGAAA CTTAGTAGAG GGGATTGTGT GTATTTNATA CAAATTTAAT ACAATGTCTT 60
ACATTGATAA AATTCTTAAA GAGCAAAACT GCATTTAATT TCTGCATCCA CATTCCAATC 120
ATATTAGANC TAAGNTATTT ATCTATGAAG ATATAANTGG TGCAGAGAGA CTTTCATCTG 180
TGGATTGCGT TGTTTCTTAG GGTTCCTAGC ACTGNTGCCT GCACAAGCAT GTGNATATGT 240
GANATNNNAT GGATTCTTCT ATAGCTAAAT GNGTTCCCTC TNGN 284

SEQ ID NO:6841
SEQUENCE LENGTH:114
SEQUENCE TYPE:nucleic acid
TOPOLOGY:linear
CLONE:HUMGS08070
SEQUENCE DESCRIPTION:
GATCCACCTG CTTCGGCCTC CCAAAGTGCT GGGATTACAG CTGTAAGTCA CTGATNCCGG 60
NTGCATGTCA AGCACATTGG NAAGTTCTTA CAACAATTCT GNTGGAGGNT TTTN 114

SEQ ID NO:6842
SEQUENCE LENGTH:253
SEQUENCE TYPE:nucleic acid
TOPOLOGY:linear
CLONE:HUMGS08071
SEQUENCE DESCRIPTION:
GATCTGTCTC TCAAAGGAAG AAACAAAAAC CCAAAATTTT GACTGCAAAA GATTGACTAA 60
TTCTAGGTGC AAATAACTGG AAATTTAGGA TTGTTATTGT ACTACACAGG TCACAANGTT 120
CCAAACCTAT TTCCCTTGTA ATTTTTAGTG CATGATTTTT CAGAATGCAG TTTCTATCTT 180
TTTAGGTGCA ATATTACGAA CTGCTTAGGT CAGANCANTT TNGTNTCTCC AGTTTTTTCT 240
CCCTANGTTA CAN 253

SEQ ID NO:6843
SEQUENCE LENGTH:103
SEQUENCE TYPE:nucleic acid
TOPOLOGY:linear
CLONE:HUMGS08073
SEQUENCE DESCRIPTION:
GATCTTATAA CCTCATTGTT TCCTAATCAT TTTATTTCAT CTCCTGCTAG TACTGTGCCG 60
CTTCCCCCTC CCCCCACACA AAATAAAAAC AGTATCTGCC AAA 103

SEQ ID NO:6844
SEQUENCE LENGTH:110
SEQUENCE TYPE:nucleic acid
TOPOLOGY:linear

CLONE:HUMGS08074
SEQUENCE DESCRIPTION:
GATCCGTGCC CCACCTCTCC CCAGTTAAGT GCCTTCACAC AGCACTGGTT TAATGTTTAT 60
AAACAAAATA GAGAAACTTT CCTTATAAAT AAAAGTAGTT TGCACAGAAA 110

SEQ ID NO:6845
SEQUENCE LENGTH:246
SEQUENCE TYPE:nucleic acid
TOPOLOGY:linear
CLONE:HUMGS08075
SEQUENCE DESCRIPTION:
GATCAGGGCT GAGGGTAAGG AAAAGAAGAG ACTAGGAAAG CTGGGCCCAA AACTGGAGAC 60
TGTTTGTCTT TCCTGGAGAT GCAGAACTNG GCCCGTGGAG CAGCAGTGTC AGCATCAGGG 120
CGGAAGCTTT AAAAGCAGCA GCGGCNTGTG CCCCAGGGCA CCCAGAATGA TTTCCCTATG 180
GGCACCAGGC CAGNGAAAAA ATTGGGCAGC NTCTTTAAAG GGNGGAAAAT TGTTTTGAGC 240
CCCAAA 246

SEQ ID NO:6846
SEQUENCE LENGTH:105
SEQUENCE TYPE:nucleic acid
TOPOLOGY:linear
CLONE:HUMGS08076
SEQUENCE DESCRIPTION:
GATCTACCTG CATATAACAG TCTCTAAGTT TTTTTTTCAA TCTTGCANTT TGATAATTTT 60
GATTTTGGCC ANATTAGNNT AATTTGGGAC TATCCTGAAA CAGNN 105

SEQ ID NO:6847
SEQUENCE LENGTH:26
SEQUENCE TYPE:nucleic acid
TOPOLOGY:linear
CLONE:HUMGS08077
SEQUENCE DESCRIPTION:
GATCAAAAGA GCTGTCTGAC TGCAAA 26

SEQ ID NO:6848
SEQUENCE LENGTH:84
SEQUENCE TYPE:nucleic acid
TOPOLOGY:linear
CLONE:HUMGS08078
SEQUENCE DESCRIPTION:
GATCCCTTGA GCCCAGGAGT TCCAGACCAG CCTGGGAAAT ATAGGGAAAC CCTGTCTTTA 60
CAAAAAAAAA TTTTAAAAAT TAAA 84

SEQ ID NO:6849
SEQUENCE LENGTH:217

SEQUENCE TYPE:nucleic acid
TOPOLOGY:linear
CLONE:HUMGS08079
SEQUENCE DESCRIPTION:
GATCAGATTC CTGACTAACC CCTCTCTTAG AGCTACAGCG AGCTGCATTA CCAGCTTAAA 60
ACACTTCTTA GGGATTAAAT ATAGNTGTNA TTTTTCAAAA TCGTTTTTAA TTTAAACTGT 120
GTTTTAGTGT AAAATTGTTA ACCTTGTAAG ATGGATANTG TGTATANGAN TGTAGGCCTT 180
AACTATTTCA CATGAGTCAA ANCANGGCNG CTTTAAN 217

SEQ ID NO:6850
SEQUENCE LENGTH:93
SEQUENCE TYPE:nucleic acid
TOPOLOGY:linear
CLONE:HUMGS08080
SEQUENCE DESCRIPTION:
GATCTCACCC AGTGTGCAAT TTGTGAAAAT GAACCAAATG TCATTACTTT TGTCTTAAAG 60
CAAAGTCTAT TTCAAGNATA TGNACAGCAN AAA 93

SEQ ID NO:6851
SEQUENCE LENGTH:73
SEQUENCE TYPE:nucleic acid
TOPOLOGY:linear
CLONE:HUMGS08081
SEQUENCE DESCRIPTION:
GATCAATGAA CCATCTCTTC TGGGCAGTTT TGTTGAAAAT AAAGGTTTCT CTTTGATTTC 60
AAGAATGACC AAA 73

SEQ ID NO:6852
SEQUENCE LENGTH:104
SEQUENCE TYPE:nucleic acid
TOPOLOGY:linear
CLONE:HUMGS08082
SEQUENCE DESCRIPTION:
GATCACCGGT TTCCGACTGA GTCTGGGGAT TTNGGCCTTG TTGAACCCTC CTACGGTGCC 60
CNGGGAATTT GAAANACGCT TTCTGCATNG ATATNTGGGC CCTN 104

SEQ ID NO:6853
SEQUENCE LENGTH:123
SEQUENCE TYPE:nucleic acid
TOPOLOGY:linear
CLONE:HUMGS08083
SEQUENCE DESCRIPTION:
GATCAACATC ATTAAAGATT ATGTAAAAAG TTAAAAGGCT TATGAGCCTA AGTTTGTTCC 60
TATATTACCA TATTTACTGA ATNTTCTGGN AAAGNAACTT TANTAAAGTT TAATCTNAGG 120
AAA 123

SEQ ID NO:6854
SEQUENCE LENGTH:91
SEQUENCE TYPE:nucleic acid
TOPOLOGY:linear
CLONE:HUMGS08084
SEQUENCE DESCRIPTION:
GATCACTTGA GCCTAGGAGG CAGNGGTTCA AGTGAGCTGA GATGGCACTC CTGCGCTCCA 60
GCCTNGGTGA CAGCGTGAGA NNCTGTCTCA N 91

SEQ ID NO:6855
SEQUENCE LENGTH:219
SEQUENCE TYPE:nucleic acid
TOPOLOGY:linear
CLONE:HUMGS08085
SEQUENCE DESCRIPTION:
GATCTGTCAG NACCATTTGA AAACTTGCTC AAGAGCAGTT GCTTATATAT AGTAGGATTT 60
TACTTTTTCC TGCTTATGTA NTACTATATG CTTAAAAAAC CTGGAGGAAT ACTTACCAAA 120
GAGGAGTAAC CATCTCTGAG GGTGGGATTC TGGGGGANTT TTTGTTTTTT TCTGTTTTCT 180
ATANTGTGCA NCTNTTNTAG TATGTNTNTT NCTAATTGN 219

SEQ ID NO:6856
SEQUENCE LENGTH:66
SEQUENCE TYPE:nucleic acid
TOPOLOGY:linear
CLONE:HUMGS08086
SEQUENCE DESCRIPTION:
GATCTTNCTC CATTTACAGT ACATTCTTAG GATGTATGTA GTAAATAAAG CTTTCTTTAA 60
AGCAAA 66

SEQ ID NO:6857
SEQUENCE LENGTH:43
SEQUENCE TYPE:nucleic acid
TOPOLOGY:linear
CLONE:HUMGS08087
SEQUENCE DESCRIPTION:
GATCACCCCG TAGGAAATAA AGCAGGCATC TCTGGACCTC AAA 43

SEQ ID NO:6858
SEQUENCE LENGTH:338
SEQUENCE TYPE:nucleic acid
TOPOLOGY:linear
CLONE:HUMGS08088
SEQUENCE DESCRIPTION:
GATCAAAAGA CAGNTAAATN TTTCTTCTAG AACACAGTTA CCCCCTTGCT TCATCTATTG 60

CTAGAACTAT CTCATTGCTA TCTGTNATAG ACTAGTGATA CAAACTTTAA GAAAACAGGN 120
TAAAAAGATA CCCATTGCCT GTGTCTACTG ATAAAATTAT CCCAAAGGTA GGTTGGTGTG 180
NTAGTTTCCG NGTAAGACCT TAAGGCCACA GCCAANCTCT TAAGTACTGT GTGNCCACTC 240
TTGTTGTTAT CACATAGTCA TNCTTGGTTG TACTATGTGA TGGTTAACCT GTAGCTNATN 300
ANTNTACTTA TTATTCTNTT CCTCCTTTAC CTCANCNN 338

SEQ ID NO:6859
SEQUENCE LENGTH:251
SEQUENCE TYPE:nucleic acid
TOPOLOGY:linear
CLONE:HUMGS08089
SEQUENCE DESCRIPTION:
GATCGTTTGC AAGTAACTGA ATCCATTGCG NCATTGTGAA GGCTTAAATG AGTTTAGATG 60
GGAAATAGCG TTGTTATCGG NTTGGGTTTA AATTATTTGA TGAGTTCCAC TTGTATCATG 120
GCCTACCCGA GGAGANGNGG NGTTTGTTAA CTGGGCCTAT GTAGTAGCCT CATTTNCCAT 180
CGTTTGTATT ACTGACCACA TATGCTTGTC ACTGGGCANG AAGCCTNTTT CNGCTGCCTN 240
CACGCANTTT N 251

SEQ ID NO:6860
SEQUENCE LENGTH:359
SEQUENCE TYPE:nucleic acid
TOPOLOGY:linear
CLONE:HUMGS08090
SEQUENCE DESCRIPTION:
GATCTTAATA TAAATNCACT TTCATTTTTN ATAGCTGTCC CATCTGGTCA TTTGGTTGGC 60
ACTAGACTGG TGGCAGGGGC TTCTAGCTGA CTCGCACAGG GNTTCTCACA ATAGCCGATA 120
TCAGAATTTG TGTTGAAGGA ACTTGTCTCT TCATCTAATA TGNTAGCGGA NAAAGGAGAG 180
GAAACTACTG CCTTTAGAAA NTATAAGTAA AGTGATTAAA GTGCTCACGT TACCTTGNCA 240
CATAGTTTTT CAGTCTATGG GTTTAGTTAC TTTAGNNGGG NAGCNTGTNA CTTATATTAN 300
TAGTANTTTN GTAAAGTTGG NTGGATAAGC TATCCATGTT GCCAGGTTCA TGGATTNGN 359

SEQ ID NO:6861
SEQUENCE LENGTH:75
SEQUENCE TYPE:nucleic acid
TOPOLOGY:linear
CLONE:HUMGS08091
SEQUENCE DESCRIPTION:
GATCCCCAAA CCACAAATAC ATATGTGTGC CTAAGTTTGT GCATGCATAC ATTAAACAGG 60
ACATAAAGTG TTAAA 75

SEQ ID NO:6862
SEQUENCE LENGTH:228
SEQUENCE TYPE:nucleic acid
TOPOLOGY:linear
CLONE:HUMGS08092

SEQUENCE DESCRIPTION:
GATCATCATG GCAAAACCAG CTGGTGGGCC CAAGCCTCCA AGTGGGAAGA AAGACTGGGA 60
TGATGACCAA AATGATTGAA ATTGGCTTAA TTTTTACTGT AGGTGAAGGC TGTATTTGTA 120
GTAGTACTCA AGAATCACCT GATGTNTTCT TATTCTCCTT AAATTAAGAG TTATTTTGTN 180
TTTGTNTTCT TGGCTGGATG TTATAATAAN CATATTGTNA CTGTCAAA 228

SEQ ID NO:6863
SEQUENCE LENGTH:299
SEQUENCE TYPE:nucleic acid
TOPOLOGY:linear
CLONE:HUMGS08093
SEQUENCE DESCRIPTION:
GATCATGCCT ACTTTCTTCT NTGTATCTCC CTCCTTCCCA GCCCACCCGG GCAGCAGACT 60
CTNATGGAAG NAAGGTGCCG TAGTGGGNTT TTAGAAACTA ACGGGACTGG TTTTCAAAGC 120
AGTTATCTTG GGAAACTGTT TATTCCAGCG ATGTGACTTT TTTNAGAATA TTTCTTGGAA 180
TCATATTCAG AGTCTGGGGC TGTGTGTTGA GCAGCCTTAA GGNTGCTAGA CACTCATTTA 240
GTGCCCAGGG AGTCCAGCGA ATGACGTCTG TGGCCAAGCG AGGTCTCAGG TGCAAAGCN 299

SEQ ID NO:6864
SEQUENCE LENGTH:298
SEQUENCE TYPE:nucleic acid
TOPOLOGY:linear
CLONE:HUMGS08094
SEQUENCE DESCRIPTION:
GATCCTCTGA ACCTGCAGAG GCCCNCTCCC CGAGCCTGGC CTGGCTCTGG CCCGGTCCTA 60
AGCTGGACTC CTCCTACACA ATTNATTTNA CGTTTTATTT TGGTTTTCCC CACCCCCTCA 120
NGNNTGTCGG GGAGCCCCTG CCCTTNACCT AGCTCCCTTG GCCAGGAGCG AGCGAAGTGT 180
NGGCCTTGGT GAAGCTGCCC TCCTCTTCTC CCCTCACACT ACAGCCCTGG TGGGGGAGAA 240
GGGGGTGGGT GCTGCTTGTG GTTTAGTCTT TTTTTTTTT TTTTTNTTTN TNANAAAA 298

SEQ ID NO:6865
SEQUENCE LENGTH:296
SEQUENCE TYPE:nucleic acid
TOPOLOGY:linear
CLONE:HUMGS08095
SEQUENCE DESCRIPTION:
GATCTGCGGG GCAANTTCAA GCNNNCCCCA CTGCGGAGGG TGCGCATGTC GNCCGATGCC 60
ATGCTCAAGG CCCTGCTGGG CTCGAAGCAC AAGGTGTGCA TGGACCTGAG GGCCAACCTG 120
AAGCAGGTCA AGAAGGAGGA CACAGAGAAG GAGCGGGACC TGCGAGACGT GGGTGACTGG 180
AGGAAGAACA TCGAGGAGAA GTCTGGCATG GAGGGCCGGA AGAAGATGTT TGANTCCGAG 240
TCCTAGGCCA CTCGCTGCCC CTACGNCTGC CCCGGTGCCC GGTTCCCAGC AGAANN 296

SEQ ID NO:6866
SEQUENCE LENGTH:310
SEQUENCE TYPE:nucleic acid

TOPOLOGY:linear
CLONE:HUMGS08096
SEQUENCE DESCRIPTION:
GATCCTGTTC TGTCAGCTGC ACCCTGAGCA AAAACTTGCC ACACCAGNGC TCCTAGAAAC 60
AGCCCAGGCT TTGGAGCGGA CCCTGAGGAA GCCTGGGAGG GGTTGGGATG ACACGTATGT 120
CTTGTCAGAT ATGCTCAAGA CGGTGAAAAT TGTGTGACTG CTTAGGCCAA GCAGCCCTCC 180
TGCCTAGAAT GACCTTGGAC TCCCAGCCTG CCAGNGAATG NAGAAATACA ACGCACAGTA 240
CTTTTGAAGC TTCGTATTTT TCTTGGTTTC ACACTCAGCT ACATGTGACC TCCAGCTTGG 300
TGCGGTTAGN 310

SEQ ID NO:6867
SEQUENCE LENGTH:285
SEQUENCE TYPE:nucleic acid
TOPOLOGY:linear
CLONE:HUMGS08097
SEQUENCE DESCRIPTION:
GATCAGTTCA AGAAGGTAAT CCAAGGGCCT GATGACTCTT TTGGTAACCA GACACCAATC 60
AAATAAGGGG AGGAGATGAA AATGGAATNA TTTCTTCCAT GCCACCTGTG CCTTTAGGAA 120
CTGCCCAGAA GAAAATCCAA GGCTTTAGCC AGGAGCGGAA ACTGACTACC ATGTAATTAT 180
CAAAGTAAAA TTGGGCATTC CATGCTATTT TTAATACCTG GNTTGCTGAT TTTNCAAGAC 240
AAAATACTTG GGGTTTTCCA ATAAAGATTG TTGTAATATT GNAAA 285

SEQ ID NO:6868
SEQUENCE LENGTH:201
SEQUENCE TYPE:nucleic acid
TOPOLOGY:linear
CLONE:HUMGS08098
SEQUENCE DESCRIPTION:
GATCAGACTC TGTATCAAAA GTACCTTTGC CCTTAGGAAG AGTGAGTATT GGAGTCATCT 60
TATCTATTAC TCCAAACCTC CCTTTTNATT TCTTGAGCCT GGCTTGGACC TTGGCATTCC 120
GTTTGANTTC CTTCTAACTG GAACATTTGT GTTGTATCTG TAACACTGGC ACTGAAATAA 180
AGACCACACG GTTAAAGNAA A 201

SEQ ID NO:6869
SEQUENCE LENGTH:50
SEQUENCE TYPE:nucleic acid
TOPOLOGY:linear
CLONE:HUMGS08099
SEQUENCE DESCRIPTION:
GATCATGTGC TGCCTTTTAC TGCTGAATTA AAACAGATAT TTCACGNAAA 50

SEQ ID NO:6870
SEQUENCE LENGTH:43
SEQUENCE TYPE:nucleic acid
TOPOLOGY:linear

CLONE:HUMGS08100
SEQUENCE DESCRIPTION:
GATCTTCAGT GTAAATNACC ATGTATAAAC TGTAAAGTGC AAA 43

SEQ ID NO:6871
SEQUENCE LENGTH:247
SEQUENCE TYPE:nucleic acid
TOPOLOGY:linear
CLONE:HUMGS08101
SEQUENCE DESCRIPTION:
GATCATTTAA TATATTCATA TTACCAAGAC TATTATACTG GAAGTGGTTT TTGTGTTATA 60
AAGGTTTAAT TTTACATANG GCAGTTACTT AATGTGATTT TNAACCCTTA AAAAAGTGGA 120
GATGTATACA TTTGTTAACA ATGCCATGAA AGCATTTNCT TTCTCCTAAG GAAAAGTGAA 180
TTTCTTATCA GAATATCTGG CTGGCCCTGT AATTTAAATT AAAATAAANT TTTGGCGNAN 240
CAGCAAA 247

SEQ ID NO:6872
SEQUENCE LENGTH:288
SEQUENCE TYPE:nucleic acid
TOPOLOGY:linear
CLONE:HUMGS08102
SEQUENCE DESCRIPTION:
GATCTATCTG AGAACAAAGA AGTCGACAGA ATAACATTGA TGACTTTTTA AATAATTAAT 60
TTATTTGAAG AATGCTTGCA TACAAAAAGC TTCCTGCAGG AATTTTAAAC AAGCTGTCCT 120
TGGTTATCCC AGGCAATGTC AGTTGATTTA NTTAAGTTTA CATGCAAAAG NGAATACAAT 180
TGTAACATTA ATTGGAACAT TAAGTTGGCT TATAAGCAGT ATTTCCACTG CTCCTACAAT 240
TGNTTTNNGT TATTTGATAN TTNATTTNAN ATTNGTNTTT GGTGTGGN 288

SEQ ID NO:6873
SEQUENCE LENGTH:294
SEQUENCE TYPE:nucleic acid
TOPOLOGY:linear
CLONE:HUMGS08103
SEQUENCE DESCRIPTION:
GATCGTACCT ATTGCACTCC ATCCTGGATG AAAGAGCCAG ACTCTGTCTC AAAACAAACA 60
AAAAAGCGTG GGGACTTCTG GGGACAGACA AGGTGCCTGT AATATATTTN CTCAGTCTTT 120
GCCCTGAATG GTCTCAGCTT GANACCATNT CAAACTGGAG AGAAGCAAGC CAGCCAATAG 180
AATGGGGTGA TTTACAGGGA TTTCTGTTTA CTGTCAAAAT ATTTCTCATC TGCACTATGT 240
TTCCATTTGT GGTCCTGAAG GAAATTCTTA TAACTCAACA TTTGTCTGGT CTTN 294

SEQ ID NO:6874
SEQUENCE LENGTH:295
SEQUENCE TYPE:nucleic acid
TOPOLOGY:linear
CLONE:HUMGS08104

SEQUENCE DESCRIPTION:
GATCCGACAC TCCAGGCAGA GCAGAGGGCA GGAGAGGCCC AAAGAGCTAG GTCAAGCAGC 60
TGGCTCCCCT GGAAGGGGAG CCGGGAGACA GGGAGGTCCT CACACTGCCC CCGTGGCCAC 120
GCATGGCTGT CTGCACACTC CATCAGGAGG AAGGGCAGGG GAGAAGTCAC ATGCNGTACA 180
GTGAAACTNA TCAGTGTCAC ACACGCCCAG CAGGAGGGGA GAGCTCTGCT CCACACGCTG 240
GCGGCCTTGG CTGCTCCANA GCAATCCGNT TGCTCCANCT TCAAGCCATT GGGGN 295

SEQ ID NO:6875
SEQUENCE LENGTH:168
SEQUENCE TYPE:nucleic acid
TOPOLOGY:linear
CLONE:HUMGS08105
SEQUENCE DESCRIPTION:
GATCCTGTAT TATCCATCTA CATCAGAACC AAACTACTTC TCCAACACCC GGCAGCACTT 60
GGCCCTGCAA GCTTAGGATG AGAACCACTT AGTGTCCCAT TCTACTCCTC TCATTCCCTC 120
TTATCCATCT GCAGGTGAAT CTTCAATAAA ATGCTTTTGT CATTCAAA 168

SEQ ID NO:6876
SEQUENCE LENGTH:141
SEQUENCE TYPE:nucleic acid
TOPOLOGY:linear
CLONE:HUMGS08107
SEQUENCE DESCRIPTION:
GATCCTTGAG ATGACTGTAG CCCTGGCTGA CAACTTNATT GCTGCCTGTN AAGGACCCTG 60
AACCAGAGGA CTCAGCGAAA TTGTGCCCAG ATTCCTGACC TACAGCGGCA GTGAGATAAT 120
AAATGCTGTT GAGGTCACAA A 141

SEQ ID NO:6877
SEQUENCE LENGTH:124
SEQUENCE TYPE:nucleic acid
TOPOLOGY:linear
CLONE:HUMGS08108
SEQUENCE DESCRIPTION:
GATCTGCTCA CCCTCGGTNC ACANCAGTGT CAGCCATGCA AGCAGGACAG AATGGTGACT 60
GGGTGCCCAT NGTGAGCTGT GTATTTCCTA GGAGGTAGAA AACAATAAAA AATTGCATGC 120
TAAA 124

SEQ ID NO:6878
SEQUENCE LENGTH:280
SEQUENCE TYPE:nucleic acid
TOPOLOGY:linear
CLONE:HUMGS08109
SEQUENCE DESCRIPTION:
GATCCTCCTG CCTCAGCCTC CTGGGTGGCT GGGACTGCAG ATACATGCCA CCATGCCGGA 60
CAAATTTNTT GTATTTTNAG TAGAGATGGG GTTTTACCAT ATTGCTCGGA TTGGTCATGA 120

ACTCCTGGAT TCAAGCAATC TGCCCACCTA GCCTCCCTAA GTGCCTGGCC AAATTTTTGG 180
TTTTAATAGT CGATTTGTGT ATATTTTTTG GTAGTATCTA CATATATTTA ATACATTTAT 240
NTGTTGNTGN AAAAAGTANA ATTTGTAAAA NNTTTGNAAA 280

SEQ ID NO:6879
SEQUENCE LENGTH:198
SEQUENCE TYPE:nucleic acid
TOPOLOGY:linear
CLONE:HUMGS08110
SEQUENCE DESCRIPTION:
GATCATTGTT GAACTTCAGC TCCAGTGTCT CTCCAGAATA AGACATTGGC ATTCAAATGT 60
CTATATCTTG TTACTTACAA AATAAAAAAC AGATTAATTA GTGGCTTTTA AATTGTAGTT 120
ATATCNGTGT ATATACACGC GGGGNACTGT ATAAAGACAT ACTAAAGGGC ACAGATTAAA 180
ATAAGTATTA TTANTAAA 198

SEQ ID NO:6880
SEQUENCE LENGTH:66
SEQUENCE TYPE:nucleic acid
TOPOLOGY:linear
CLONE:HUMGS08111
SEQUENCE DESCRIPTION:
GATCAAAGTT GTTAAAATTA TAAATNTATG ATGCAGNAAT AAAATTGATA TATTTTGATA 60
TTCAAA 66

SEQ ID NO:6881
SEQUENCE LENGTH:100
SEQUENCE TYPE:nucleic acid
TOPOLOGY:linear
CLONE:HUMGS08112
SEQUENCE DESCRIPTION:
GATCTCTTAC CTTTGGAAAA TAGGGGTTAG GCATGAAGGT GGTTGTGATT AAGAAGATGG 60
TTTTGTTATT AAATAGCATT AANCTGGAAT TGACAAGAAA 100

SEQ ID NO:6882
SEQUENCE LENGTH:30
SEQUENCE TYPE:nucleic acid
TOPOLOGY:linear
CLONE:HUMGS08113
SEQUENCE DESCRIPTION:
GATCAAAGAA ATAATTCTTT TGCCACTAAA 30

SEQ ID NO:6883
SEQUENCE LENGTH:172
SEQUENCE TYPE:nucleic acid
TOPOLOGY:linear

CLONE:HUMGS08114
SEQUENCE DESCRIPTION:
GATCACTACA ACAATTTGNC TGCCTCCAAG GTCCTCTGAG GCAGAAGGCT CTGGGGNTTC 60
TGCTGTCCTT TGGNGGGTGT CTTCTGGGTA GAGGGATGGG AAGGAAGGGA CCCTCACCCC 120
NGGCNCTTCT TCTGACCCTG CCACTAAAAA ATTTATGGNC CAAGGGCNCA AA 172

SEQ ID NO:6884
SEQUENCE LENGTH:291
SEQUENCE TYPE:nucleic acid
TOPOLOGY:linear
CLONE:HUMGS08115
SEQUENCE DESCRIPTION:
GATCAGCAAT NAGGGTGAGG ACAAAATCTT CTTAATCAAC AAGCTTCACT CCATCTACGA 60
GAGGAAGGAG AGGGAGGAGA GGAGCAGGGT TGGGACAACC GAGGAGGCTG CGGCACCCCC 120
TGCCCTGCTC ACAGATGAAC AGGATGCCTA GGGGGACGGC GATGGGCCTC ACGGGCCCGC 180
CCAGCACCCT GAGACCACAC TGTTGCCTCC CAGTGACCCT GCTGGGACAC CAGGACAAGG 240
AAGACAGTTT CGCCTCTNGA AAGCCGCAGC TGCGCCTAGG CTGGAGCTGG N 291

SEQ ID NO:6885
SEQUENCE LENGTH:30
SEQUENCE TYPE:nucleic acid
TOPOLOGY:linear
CLONE:HUMGS08116
SEQUENCE DESCRIPTION:
GATCTGTAAA TAAAACTTAC ATTTTTCAAA 30

SEQ ID NO:6886
SEQUENCE LENGTH:291
SEQUENCE TYPE:nucleic acid
TOPOLOGY:linear
CLONE:HUMGS08117
SEQUENCE DESCRIPTION:
GATCTGCCTG CCTCGGNCTC CCAAGTGCTG GGATTACAGG CGNGAGCACG TGCACCCAGC 60
CTAGAATCTT GTATAATATG TAATTGTAGG GAAACTGCTC TCATAGGAAA GTTTTCTGCT 120
TTTTAAATAC AAAAATACAN AAAAATACAT AAANTCTGAT GATGAATATA AAAAAGTANC 180
CAACCTCATT GGNACAAGTA TTAACATTTT GGAATATGTT TTATTAGTTT TGTGATGTAC 240
TGTTTTACAA TTTTNACCAT TTTTTTCAGT AATTACTGTA AANNGNNNNN N 291

SEQ ID NO:6887
SEQUENCE LENGTH:288
SEQUENCE TYPE:nucleic acid
TOPOLOGY:linear
CLONE:HUMGS08118
SEQUENCE DESCRIPTION:
GATCCCAAGC TCAGTCCCCA CAAAGTTCAG GGCCGGTCGG AGGCAGGGGC AGGTCCGGGT 60

CCAAAGCAAG GACACCACAG CTCTTCCGAC TCCAGCAGCA GCTCCAGCGA TTCGGACACG 120
GATGTGAAGT CCCACGCTGC TGGCTCCAAG CAGCACGAGA GCATCCCGGG CAAGGCCAAG 180
AAGCCCAAAG TGAAGAAGGA GAAGGGCAAG ANGGNGAAGG GCAAGANGAN GGAGGCTCCC 240
CACTGANGGG CCCTGGACAG GGCTCATTAA ACCTTCCTCT CTGCCAAA 288

SEQ ID NO:6888
SEQUENCE LENGTH:308
SEQUENCE TYPE:nucleic acid
TOPOLOGY:linear
CLONE:HUMGS08119
SEQUENCE DESCRIPTION:
GATCCGCATT GAAGACCCAC CCCGCAGAAA GCACATGGAA TTCCTGGGTG GTGCAGTTCT 60
AGCGNTTATC ATGAAAGNCA AAGACAACTT TTGGATGACC CGACAAGAGT ACCAAGAAAA 120
GGGTGTCCGT GTGCTAGAGA AACTTGGTGT GACTGTTCGA TAAACTCCAA AGCTTGTTCC 180
CATCATACCC GTAATGCTTT CTTTTTTCCT TTATTGCCAA TCTTTGAACT CATTCAACTC 240
CAGGACATGG AAGAGGCCTC TNTCTGCCCT TTGACTGGAA NGGTCAANGT TTTATTCTGG 300
TTGTCTGN 308

SEQ ID NO:6889
SEQUENCE LENGTH:308
SEQUENCE TYPE:nucleic acid
TOPOLOGY:linear
CLONE:HUMGS08120
SEQUENCE DESCRIPTION:
GATCATGTCT TTTGCAGGAA CATGAATGGA GCTGGAGGCT ATTATCCTTA GCAAACTAAT 60
GCAGGAACAG AAAACCAAAT ACTGCACGTN CTCACTTATA AGAGGGAGCT AAATNATGAG 120
AACTGATGAA CACAAAGAAG GAAACAACAC ACAATGGGGT CTACTTGACA GTGGAAGGTG 180
GGAGGAGGGN GAGGAGCAGA AAACATAACT ATTGGATACT GGGCTTAGTA TCTGGGTGAT 240
GAAATAATCT GTACAACAAA CCCCCATGNC ACAAGTTCAC CAATGTAACA NACCTTCACA 300
TGTACCCN 308

SEQ ID NO:6890
SEQUENCE LENGTH:224
SEQUENCE TYPE:nucleic acid
TOPOLOGY:linear
CLONE:HUMGS08121
SEQUENCE DESCRIPTION:
GATCAATTCC CTTGAATAGG GAAGTAACAT TTGCCTTAAA TTTTTTCGAC CTCGTCTTTC 60
TCCATATTGT CCTGCTCCCC TGTTTGACGA CAGTGCATTT GCCTTGTNAC CTGTGAGCTG 120
GAGAGAACCC AGATGTTGTT TATTGAATCT ACAACTCTGA AAGAGAAATC AATGAAGCAA 180
GTACAATGTT AACCCTAAAT TAATAAAAGA GTTAACATCC CAAA 224

SEQ ID NO:6891
SEQUENCE LENGTH:28
SEQUENCE TYPE:nucleic acid

TOPOLOGY:linear
CLONE:HUMGS08122
SEQUENCE DESCRIPTION:
GATCAAATAA AGTTATAAAA TTGCCAAA 28

SEQ ID NO:6892
SEQUENCE LENGTH:140
SEQUENCE TYPE:nucleic acid
TOPOLOGY:linear
CLONE:HUMGS08123
SEQUENCE DESCRIPTION:
GATCAGGCCT GGCCACTCCT CTTTGGAGGG CGGTTGAGGC CCACCTGCCT TGGGCTTCCC 60
AATNCTGATT CCNNTACTAC ACCCTTCCCA GTTCTATTCC CCTCACCACA CTTGCTCCAA 120
TAAAACTATT TATATACAAA 140

SEQ ID NO:6893
SEQUENCE LENGTH:163
SEQUENCE TYPE:nucleic acid
TOPOLOGY:linear
CLONE:HUMGS08124
SEQUENCE DESCRIPTION:
GATCGTCATC CCTTCCTGGA GTTCCTATCT TCCAAGATGT GACTGTCTGG AGTTCCTTGA 60
CTAGGAAGAT GGATGAAAAC AGCAAGCCTG TGGATNGAGA CTACAGGGGA TATGGGAGGC 120
AGGGAAGAGG GGTTGTTTCT TTTAATAAAT CATCATTGTT AAA 163

SEQ ID NO:6894
SEQUENCE LENGTH:253
SEQUENCE TYPE:nucleic acid
TOPOLOGY:linear
CLONE:HUMGS08125
SEQUENCE DESCRIPTION:
GATCACCTGA GGTCAGGAGT TTGAGACCAG CCTGGCCAAC ATGGTGAAAC GCCATCTCTA 60
CTAAAATTGA AAAATTAGCT GGGCATGGTG GCAGGTGCCT GTAATCCCAG CTATTGGGGA 120
GGCTGAAGCA GGAGAATCGC TTGAAACAGG AGGCAGAGAT TGCAGTGAGC CGAGNGGGGN 180
GCCACTGCAC TCCAGCCTGG GCAACAGGAA CAAAACTCTA TCTCAAAAAA TAAAACAAGA 240
TTTTTCTGCG AAA 253

SEQ ID NO:6895
SEQUENCE LENGTH:99
SEQUENCE TYPE:nucleic acid
TOPOLOGY:linear
CLONE:HUMGS08126
SEQUENCE DESCRIPTION:
GATCACTTGC AGCCCAGNAA GTTTGAGACC ACCCTGACCA ACACAGTGAA ACCCNCTCTC 60
TAAAAAAGTT TGTTTTTAAA GTCTCCTTGA AACATCAAA 99

SEQ ID NO:6896
SEQUENCE LENGTH:286
SEQUENCE TYPE:nucleic acid
TOPOLOGY:linear
CLONE:HUMGS08127
SEQUENCE DESCRIPTION:
GATCAAGCTA TGCCTGATNC TGCCTTAAGC CTTATNAGGT CGCCACGGAA CCGCAGAAAC 60
TCCTGGTGGG CAGAGCTGTG TGTGCCATCT TCATCATTGC ATTACCACCA CCAAGCACAG 120
TGCCTGGCAC AAAAGAGTTG CTCAATAAAT AAATCAGGAT GANTGGNTAA ATACATGGNT 180
AGGCACTTTG AGCTACAGAT GCGTTTAAAT ACTTTGTGTT TTTCTTAGTC AAACATGTGC 240
ANTTANGCAT GTGATAAATG CTATGATGAC CACAANANTG CACAAA 286

SEQ ID NO:6897
SEQUENCE LENGTH:137
SEQUENCE TYPE:nucleic acid
TOPOLOGY:linear
CLONE:HUMGS08128
SEQUENCE DESCRIPTION:
GATCATGGTG AAATATTTTN GATATATAAA TTCCTTAAGC TATTGTAACC ATGTTTTATT 60
GCAAAGATGT AAAATATGCC AGATGTGTGT NAGTTGGAAA TCAAAAAAAG NAAAATAAAN 120
TATGCAAAGA NTTCAAA 137

SEQ ID NO:6898
SEQUENCE LENGTH:290
SEQUENCE TYPE:nucleic acid
TOPOLOGY:linear
CLONE:HUMGS08129
SEQUENCE DESCRIPTION:
GATCCGCAAC AACTCAGCCA TCCACATCCG AGTCTTCAGG GTCACACCCA AGTAATTGAA 60
AAGACACTCC TCCACTTATC CGNTCCGTGA TATGGCTCTT CGCATGCTGA GTACTGGACC 120
TCGGACCAGA GCCATGTAAG AAAAGGCCTG TTCCCTGGAA GCCCAAAGNA CTCTGAATTG 180
AGGGTGGGGG TAATTGTNTG TTGGTNGGCC CAGTTAGTGG GCTTTNNTAA GTGTGTGTAT 240
GCGGTCTGTA NCTATTGCCA TATAAATAAA NAATCCTGTT GNACTNGTAN 290

SEQ ID NO:6899
SEQUENCE LENGTH:71
SEQUENCE TYPE:nucleic acid
TOPOLOGY:linear
CLONE:HUMGS08130
SEQUENCE DESCRIPTION:
GATCATAAAA CGTTGTAAGT TTTCTAAAAC ACATAAGCTC TCAATAAACG TTAGCTTATC 60
ATTGTCACAA A 71

SEQ ID NO:6900

SEQUENCE LENGTH:211
SEQUENCE TYPE:nucleic acid
TOPOLOGY:linear
CLONE:HUMGS08131
SEQUENCE DESCRIPTION:
GATCAAACAC TGTCAGAATT GCTGAAATCA ATACACAAAG AGATAAAGTT TAGCTTCTTT 60
TTACTATTCA ATATTGAACA TAATATTGTT AAATATNGNG ATGAAATGCT GTTGGATNTG 120
CTNCATTAAA TCTTAATGTA ATATTGTAAG NCTTTTGAGA ATATACTTGA TTAAAATGTG 180
AAAGNNGGGN TTGTTANCTT ATTGCTATNC N 211

SEQ ID NO:6901
SEQUENCE LENGTH:81
SEQUENCE TYPE:nucleic acid
TOPOLOGY:linear
CLONE:HUMGS08132
SEQUENCE DESCRIPTION:
GATCATCATC TNCTTGCGGA CTTCTCTGCC TGGTCTTGTG TGTTCTGTTA TTCAAACAAT 60
AAAANGCTGG TNGAACTTAA A 81

SEQ ID NO:6902
SEQUENCE LENGTH:175
SEQUENCE TYPE:nucleic acid
TOPOLOGY:linear
CLONE:HUMGS08133
SEQUENCE DESCRIPTION:
GATCAGACAC TGCCTTTTGT GTGTTTGCTG CCTNTGGATN CTTTTTTAAA AAGACTGTTA 60
CATATTAAAA NAGTGTACAT ATATAAATAT NACCTCTTTT GCTGTACAGT TGTGATAGAG 120
ACTGAAGNTT TTATTTTTNG TGTGCTTTTT ATAAGNAAAA ANTTAATACA CTAAN 175

SEQ ID NO:6903
SEQUENCE LENGTH:92
SEQUENCE TYPE:nucleic acid
TOPOLOGY:linear
CLONE:HUMGS08134
SEQUENCE DESCRIPTION:
GATCATCTAA TTCTCTGTGG AATAAATACA CACATATATA TTACAAGGGA TAATTTAGAC 60
CCCATACAAG TTTATAAAGA GTCATTGTTA AA 92

SEQ ID NO:6904
SEQUENCE LENGTH:312
SEQUENCE TYPE:nucleic acid
TOPOLOGY:linear
CLONE:HUMGS08135
SEQUENCE DESCRIPTION:
GATCTGGAAA ATACTGGAAA ATGTGATACT TAGAATACTT TGGCTGCTAA GGAAACTTCC 60

TCTCCATTGC AGAATAGCTG AGCCAAGTNA GTGAGTTTNC AGAAAGCAGG TGGTGAGCTC 120
NTGCCTGCTG GAGGTTGCCA TGGAGGGCCA TTCCTGCCCG GCAACAGCAC CGTCCTGCAG 180
GGAGCCACTT GGCAGAAGGG TGCAGGGCTG CTGGTGTCAG AGCAAGAGGG CTACAGGGAA 240
AGGGCCCTTT CTCAGGGGAT GTNGNTTTTT TAAAAGGTTT GGGNACACTT GGCGGGTTTG 300
CTAAAATTGA GN 312

SEQ ID NO:6905
SEQUENCE LENGTH:123
SEQUENCE TYPE:nucleic acid
TOPOLOGY:linear
CLONE:HUMGS08136
SEQUENCE DESCRIPTION:
GATCTCTCTT GCAGCTTGAG AATGTCCCAG TTAAGCCACT TTAAAANTCC TCTNTCCTGC 60
AGCCCAACAC TTTNCTAAGA GGTACAGAAA NGANAGGAAA TAAAACACAC ATCATTATGG 120
AAA 123

SEQ ID NO:6906
SEQUENCE LENGTH:212
SEQUENCE TYPE:nucleic acid
TOPOLOGY:linear
CLONE:HUMGS08137
SEQUENCE DESCRIPTION:
GATCTCAACA CAGACCCTNA CCAGCTGATN AATNCAGTNA ACACACTGGA CAGGGATGTC 60
CTCAACCAGC TACACGTACA GCTCATGGAG CTGAGGAGCT GCAAGGNTTA CAAGCAGTGT 120
AACCCCCGGA CTCGAAACAT GGACCTGGGG ACTTAAAGAT GGAGGAAGCT ATGAGCAATA 180
CAGGCAGTTT CAGCGTCGAA AGTGGCCAGA AN 212

SEQ ID NO:6907
SEQUENCE LENGTH:235
SEQUENCE TYPE:nucleic acid
TOPOLOGY:linear
CLONE:HUMGS08138
SEQUENCE DESCRIPTION:
GATCAAGCCA AAAGAAATAT TTTTNAAATA AGCCCTTTTC AAAAGTTTTT GAATTTATAG 60
AAAGCACCAA TGAATAACAT ATTTCTGTTT CGTTAATGTC AGCTGCCTGA ACATTCAGCA 120
GTTTATAAAT TGCTTAATTT GTGTNATCTA TTATCCAGTA AACCCATAGT TCCATGATAT 180
GTCACAGGNA TTGTNAGGTC CTATTTTAAA GGTACAGTCT TGTGGNATGT CATCN 235

SEQ ID NO:6908
SEQUENCE LENGTH:224
SEQUENCE TYPE:nucleic acid
TOPOLOGY:linear
CLONE:HUMGS08139
SEQUENCE DESCRIPTION:
GATCCTTTGA GCCCAGAAGT TGGAGACCAG CCTGGACAAC ATGACAAAAC CCCATCTCTA 60

CAGAAAATTT AAAAATTGGC CAGGCGTGGT CGCGCACGCT GTAGTCCCAG CTATTCAAAA 120
GGCTGAGGTG GGAGGATTAT GTCCCTGGAG CCCAGGAAGT GGNGGTTGCA GNGATTGCAT 180
CACTGCATTT NAGCCTGGGT GACAGNGTAC GCCNCTGTAT CAAA 224

SEQ ID NO:6909
SEQUENCE LENGTH:215
SEQUENCE TYPE:nucleic acid
TOPOLOGY:linear
CLONE:HUMGS08140
SEQUENCE DESCRIPTION:
GATCCTGCTG GCCAGAAGCA AGCGGGNNCG GCAATAAATC CAAGAAATTG TCCCAACAAC 60
CACCAATTCT TACGGAGGAN TATTATTTAG CCAGCAGGAG TCGNGTTTGG TTTACTGCTT 120
TTACTGTTTT GNGTTCATGN CTNTTTATTT TAATGGCGTT AAAAGCACAG GCAAATGTCT 180
TTGGTAATGC AGCTTAGTAT GANTTCTNTT ANAGN 215

SEQ ID NO:6910
SEQUENCE LENGTH:253
SEQUENCE TYPE:nucleic acid
TOPOLOGY:linear
CLONE:HUMGS08141
SEQUENCE DESCRIPTION:
GATCACAGAC TCTGAAGNCA GACTGGCAAG ATTCAAATCC CGACTGCCAT TTTTAACTGA 60
AAGACCTTAG GTAAGTTACC TAACCTCTCT GTACCTGTGT AAAATGGNGA TAACAGTAGT 120
ATCCACTTCA CAGAGTTATG AGGNTGAAAT ACATTCATAT TTATAAAACG TTTGGACCCA 180
GTACTTGGCA CATAGTAAGG CCATGTAAGT GTTTGTAAAA TAAAACTGNA AATAAAGCCA 240
TNTCCNAATN AAA 253

SEQ ID NO:6911
SEQUENCE LENGTH:359
SEQUENCE TYPE:nucleic acid
TOPOLOGY:linear
CLONE:HUMGS08142
SEQUENCE DESCRIPTION:
GATCACACTG CTGCAAGACC TGACCAGTTG AGCTTCCGGC GTGGGGAAGT GCTGCGTGTC 60
ATNACCACAN TGGATGAGGA CTGGCTCCGC TGTGGGCGGG ATGGCATGGA GGGTCTNGTG 120
CCTGTGGGGT ATACCNACCT TGTTCTGTAG CCCTGGNGNC CTTTCCTGCG TATGTGTCTC 180
CTTCCTGTCA CCTGGGCATG GAATGGCCAG TGAACACCAT CCCAGAAGCA TTTTCCCTCT 240
GCAAAATGAC GTTTCTTCCC ACGTCTGTTT CTGCTAATAT TTAAAATAAA CTTTCCTTCT 300
TCCCTCCTAT ACCANCTGTA AGGGAAAATN TGCTCTTNTT CCAAATATAT AAAAAGGTN 359

SEQ ID NO:6912
SEQUENCE LENGTH:49
SEQUENCE TYPE:nucleic acid
TOPOLOGY:linear
CLONE:HUMGS08143

SEQUENCE DESCRIPTION:
GATCTTTTAC ATTGCAAACA AAATGCTGTA TTGCTTAAAA GGGTTGAAA 49

SEQ ID NO:6913
SEQUENCE LENGTH:30
SEQUENCE TYPE:nucleic acid
TOPOLOGY:linear
CLONE:HUMGS08144
SEQUENCE DESCRIPTION:
GATCAGTTAA TAAACATGAG TAGCTTGAAA 30

SEQ ID NO:6914
SEQUENCE LENGTH:128
SEQUENCE TYPE:nucleic acid
TOPOLOGY:linear
CLONE:HUMGS08145
SEQUENCE DESCRIPTION:
GATCTGAACT GTACAAAGTT CTCATAGCAC CNCTGGGAAG AGAAGGACGT AAACAGAACC 60
TGACACCAGC TCCTTTTCCT TNTATACATT ATTTAATACC TATTAAATAC AATTATTTTT 120
GGAATAAA 128

SEQ ID NO:6915
SEQUENCE LENGTH:425
SEQUENCE TYPE:nucleic acid
TOPOLOGY:linear
CLONE:HUMGS08146
SEQUENCE DESCRIPTION:
GATCTACTTG TAACTTGTTG GCCTTCTTCC CACATCTGCC TCANACTGGG GGGGGCTCAG 60
CTCCTCGGGT GATATCTAGC CTGCTTGTNA GCTCTAGCAG GGATAAGGAG AGCTGAGATT 120
GGAGGGAATT GTGTTGCTCC TGGAGGGAGC CCAGGCATCA TTAAACAAGC CAGTAGGTCA 180
CCTGGCTTCC GNGGGCCAAT TCATCTTTCA GACAATCTTT AGCAGAAATG GACTCAGGGA 240
AGAGACTCAC ATGCTTTGGT TAGTATCTGT GTTTCCGGTG GGTGTAATAG GGGNTTAGCC 300
CCNGAAGGGN CTGAGCTAAA CAGTGTTATT ATGGGAAAGG AATGGCATTN CTNGTTTCAA 360
CNAGCGCTAN ATGCAACCAT TNCNCTNTTG TTATAGTATC TAAGGGTNNG NNAGTTAAAC 420
GGTTN 425

SEQ ID NO:6916
SEQUENCE LENGTH:81
SEQUENCE TYPE:nucleic acid
TOPOLOGY:linear
CLONE:HUMGS08147
SEQUENCE DESCRIPTION:
GATCCACCAC CCAGCTTCAC CCCTCACCCC CAGCGGCTCA CCATGGGGAT GGCAGCAATA 60
AAATACTTCT AATGGAACAA A 81

SEQ ID NO:6917
SEQUENCE LENGTH:254
SEQUENCE TYPE:nucleic acid
TOPOLOGY:linear
CLONE:HUMGS08148
SEQUENCE DESCRIPTION:
GATCAACAAG CTTTNCCACG AATTTAAGAG TTTTATCAAG ATATATCGAA TACTTCTACC 60
CATCTGTTCA TAGTTTATGG ACTGATGTTC CAAGNTTGTA TCATTCCTTT GCATATAATT 120
AAACCTGGAA CAACATGCAC TAGNTTTATG TCAGNAATAT CTGTTGGNTT TCCAAAGGNT 180
GTTAACAGAT GANGTTTATG TGCAAAAAAG GGTAAGATAT AAATNCAAGG AAGNAAAANN 240
GTTGNTAGCT AAAN 254

SEQ ID NO:6918
SEQUENCE LENGTH:134
SEQUENCE TYPE:nucleic acid
TOPOLOGY:linear
CLONE:HUMGS08149
SEQUENCE DESCRIPTION:
GATCAGAGGA ACCTTAGAGG CCTGAAATTG TTGCTTCCAG TTTAGCTGCC CCTCAAATTC 60
AAGTGAATAT TTTCCCTTCT CCCTTTACCC TTCTCCAGAA ATAAAGCAGG TGACAGGGTT 120
TTNAGAATCT TAAA 134

SEQ ID NO:6919
SEQUENCE LENGTH:277
SEQUENCE TYPE:nucleic acid
TOPOLOGY:linear
CLONE:HUMGS08150
SEQUENCE DESCRIPTION:
GATCAAGAGT AATTACCAAC TTAATGTTTT TGCATTGGAC TTTGAGTTAA GNTTATTTTT 60
NAAATCCTGA GGACTAGCAT.TAATTGACAG CTGACCCAGG TGCTACACAG AAGTGGCTTC 120
AGTGAATCTA GGAAGACAGC AGCAGACAGG ATTCCAGGAA CCAGTGTTTG NTGNGGCTAG 180
GNCTGAGGAG CAAGCGAGCA AGCAGAGNAA GAAAAGTTAA ATACCAGNTA AGCTTTTGAT 240
TTTTGTATTG TTTGCATCCC CTTGCCCTCA ATAAACN 277

SEQ ID NO:6920
SEQUENCE LENGTH:255
SEQUENCE TYPE:nucleic acid
TOPOLOGY:linear
CLONE:HUMGS08151
SEQUENCE DESCRIPTION:
GATCTACCAC CAGAGAGTTC ACACTGGNGA AAGNCCTCAT GAGTGCAATN AATGTGGAAA 60
ATCCTTTAGC CGAAGCTCCA GCCTCATTCA CCACCGGAGA CTTCACACTG GAGAAAGACC 120
CTATGCGTGC AGTAAATGTG GGCAGTCATT TAAGCAAAGC TCCAGCTTCA GTTCACATCG 180
GAAAGTCCAC ACNGGGGANN GGCCTTATGT GTGTGGGGNC TNTGGGNANT CCTTTAGCCA 240
TAGCTCCANC CTTAN 255

SEQ ID NO:6921
SEQUENCE LENGTH:273
SEQUENCE TYPE:nucleic acid
TOPOLOGY:linear
CLONE:HUMGS08153
SEQUENCE DESCRIPTION:
GATCTGGCTT NTCCNGGAGG CTNCCATGGT TGGAAGATGG TATCAGAGGG CCTGCCTGGG 60
CAGTCTGTCT CCGGGCCAGG GTCAGGGACC CTCTGCCTCT GGCAGCCTTA ACCTGTCCTC 120
TGCTAGGACC AGGGTGATTT CAAGCCAGGG AAGCAACTGG GACCCTGAAA ACTGTCCCTC 180
CCCAGCCCGG TCCNCGTGTA TGTGCCNTGG TCCCCTTGCT GCCATGTGGA TGCTGTTGTN 240
ATTGCTGTTT GTATATTATC AAAATGTTTT TAN 273

SEQ ID NO:6922
SEQUENCE LENGTH:83
SEQUENCE TYPE:nucleic acid
TOPOLOGY:linear
CLONE:HUMGS08154
SEQUENCE DESCRIPTION:
GATCTTCTTT ATAACTATTT ATTGTTCGAA TCACTTTTAG GATGTAACTT TATAAATAAA 60
CATGAGCGCT GATGATTTGC AAA 83

SEQ ID NO:6923
SEQUENCE LENGTH:207
SEQUENCE TYPE:nucleic acid
TOPOLOGY:linear
CLONE:HUMGS08155
SEQUENCE DESCRIPTION:
GATCCTCATC TCCATCCTCA TCCTCTTCCT TCACAGCATT TACTTGGAGC TCTTTGTGAC 60
ACACCATGTC AGTCATGATG AATCGGCCAA CAGCCAGCCC TTGCCAGCTG ACGTCACAGT 120
CTAAGATGGG AAACTGTGGT ACAGATAGAC ATGAAGAGAG CTTAGCAGTG ATTGAGGTGG 180
TGACTAAATA TACAGTCATT GAATAAA 207

SEQ ID NO:6924
SEQUENCE LENGTH:199
SEQUENCE TYPE:nucleic acid
TOPOLOGY:linear
CLONE:HUMGS08156
SEQUENCE DESCRIPTION:
GATCACTGTG GGCTGGAGAG GAGAAGGAAA GGGTCTGCGC CANNCTGTCC GTCTTCACCC 60
ATANCAAAGC ATACTAGAGC AAAAAACCAG TTGTAATATA AAATGCACTG CCCTACTGTT 120
GGTATGACTA CCGTTACCTA CTNTTGTCAT TGTTATTACA GCTATGGCCA CTATTATTAA 180
AGAGCTGTGT AACATCAAA 199

SEQ ID NO:6925

SEQUENCE LENGTH:358
SEQUENCE TYPE:nucleic acid
TOPOLOGY:linear
CLONE:HUMGS08157
SEQUENCE DESCRIPTION:
GATCATGCAG CAGAAGCAGA AAAAGGCAAA CNAGAAGAAG GAGGAACCCA AGTAGCTTTG 60
TGGCTTCGTG TCCAACCCTC TTNCCCTTCG CCTGTNTGCC TGGAGCCAGT CCCACCACGC 120
TCGCNTTTCC TCCTGTAGTG CTCACAGGTC CCAGCACCGA TGGCATTCCC TTTNCCCTGA 180
GTCTGCAGCG GGTCCCTTTT GTNCTTCCTT CCCCTCAGGT AGCCTCTCTC CCCCTGGGCC 240
ACTCCCGGGG GTGAGGGGGT TACCCNTTCC CAGTGTTTTT AATTCCTGTG GGGCTCACCC 300
CAAAGTATTA AAAGTAGCTT TGTAATTCCA AAAAAAANGG NNNNNAGGGN GGGGGGGN 358

SEQ ID NO:6926
SEQUENCE LENGTH:62
SEQUENCE TYPE:nucleic acid
TOPOLOGY:linear
CLONE:HUMGS08158
SEQUENCE DESCRIPTION:
GATCTGTACA TTTTACTATC TGTAAAATAT TCTTCAAAGA ATAAAATAAA AAAAAAAGCA 60
AA 62

SEQ ID NO:6927
SEQUENCE LENGTH:51
SEQUENCE TYPE:nucleic acid
TOPOLOGY:linear
CLONE:HUMGS08159
SEQUENCE DESCRIPTION:
GATCAGATAC AGCATTAAAC GTTTCTCAGT CTCCCTNTCC TCATCTGTAA A 51

SEQ ID NO:6928
SEQUENCE LENGTH:259
SEQUENCE TYPE:nucleic acid
TOPOLOGY:linear
CLONE:HUMGS08160
SEQUENCE DESCRIPTION:
GATCTTGCTA AAGTTAAAAT AAGGAACATT TCACCTTTTA AATATTTAAT TCTNATGTGG 60
ACTTATTTCC AGAAAACTTT GGTGATAATT CTTGAGACAA AAGGTGGTTA AGTAGCATTA 120
TTATGTAATG CTTATATACC ATAGAGTTTT TAATAGANGA GAAATCCATT TCCTCCGAGG 180
GTCACTATTA ACAATGTACT TCCTTAAATT TNGTTTAATG ATTGTGATGG GTGCTGCATT 240
TGCACATTGC ATTANGGAN 259

SEQ ID NO:6929
SEQUENCE LENGTH:226
SEQUENCE TYPE:nucleic acid
TOPOLOGY:linear

CLONE:HUMGS08161
SEQUENCE DESCRIPTION:
GATCGGATGC TGAATAAAAC TCACCGTGAA GCAAGTCCCA CTGAACGAAA AGTTGGTCGT 60
AGCATTTNTC AGCAGCTGCT TCCGGCTCCC TGAGACCCAG CCATGGAAGG CATAGAAAGG 120
CATGTGACAA GAAACTCATG GCTTTTAATC CTGGCAGGNC AAGGAAAACC CCGTCAAAGN 180
TTTGCATAAN TTTTGCTGCT CTCCTGCTGG GGGGGGNNGG NNNNNN 226

SEQ ID NO:6930
SEQUENCE LENGTH:219
SEQUENCE TYPE:nucleic acid
TOPOLOGY:linear
CLONE:HUMGS08162
SEQUENCE DESCRIPTION:
GATCGTTCTT GATTTTGTTT TCATTAGTGT CATTTCTTTG TCATTGAGGA CTTTTCCCCT 60
TACAACAGTA ACACCATTTT TTGAAGAGCA AAACTTATAA TACCTCCTGG GATTGTNAGC 120
TAGTCATTCA GCCTGTGTAA CCATGTGGAA ATAAAAATTG ACGACCAATG TATTATATGG 180
ACAACTTTTG CTTTGAGTAA TAAACTTGAT TGTAGGAAA 219

SEQ ID NO:6931
SEQUENCE LENGTH:111
SEQUENCE TYPE:nucleic acid
TOPOLOGY:linear
CLONE:HUMGS08163
SEQUENCE DESCRIPTION:
GATCCCACCC ATTCTTTCAC TTTAAGAAAA AACAAATAAT TGTTGCAGAG GTGTCTGTAT 60
TTTGCAGCTG CCCTTTTGTA AGANGCACTT TTCCCAAATA AAACAATTAA A 111

SEQ ID NO:6932
SEQUENCE LENGTH:109
SEQUENCE TYPE:nucleic acid
TOPOLOGY:linear
CLONE:HUMGS08164
SEQUENCE DESCRIPTION:
GATCATTTGA AATNTCTGGG AGTCTGAGGN GTACTGACAT AATNACCTGC TGGAGTCTGT 60
AAATACACAT TTAAGACAGT GAGGATGTGA ATAAATATAT TAATGCAAA 109

SEQ ID NO:6933
SEQUENCE LENGTH:111
SEQUENCE TYPE:nucleic acid
TOPOLOGY:linear
CLONE:HUMGS08165
SEQUENCE DESCRIPTION:
GATCAAATAG AGCCCTGTCC AGGTAGAAAA GAAATGGTAT GTAGAGCTTA GATGTCCCTA 60
TTGTGACAGA GCCATGGTGT GTTTGTNNTA ATAAANCCAA AGAAACATAA A 111

SEQ ID NO:6934
SEQUENCE LENGTH:243
SEQUENCE TYPE:nucleic acid
TOPOLOGY:linear
CLONE:HUMGS08166
SEQUENCE DESCRIPTION:
GATCCCAGCT GTTCTAGGCA GGGCCAGGCA GAGTGGGGCG CAGGCCCTNG AAGGGCGAGA 60
CCCAGTGGCT GGGCTGCCCA GGGCTGAGGG GCCGCCTCTT GAGGGTACAC GCCTCTGGTC 120
ACATGGCCAT GGAGCCTTGG GTACCCCTGA GTTAAGGGAG GACATTTGGC CAGCTGGTGG 180
CTGGGAGGGG AGCCTGGCTG CCCTGCTGCT TCTCCTGCCT AATAAACAGG CTTCTCCTGC 240
AAA 243

SEQ ID NO:6935
SEQUENCE LENGTH:124
SEQUENCE TYPE:nucleic acid
TOPOLOGY:linear
CLONE:HUMGS08167
SEQUENCE DESCRIPTION:
GATCTATTTT NATAGGTTCT TTTTCAGAAG TAAAATTTTG TACATATATA CATGTACATA 60
TCTNTTTAGT TTGGGTTCAT TTCTATAACA TTTTGTAAGA AAATAAAAGT TTGAGCACCT 120
GAAA 124

SEQ ID NO:6936
SEQUENCE LENGTH:169
SEQUENCE TYPE:nucleic acid
TOPOLOGY:linear
CLONE:HUMGS08168
SEQUENCE DESCRIPTION:
GATCAGTTGT ATGTAAATGT ACATTTTTGT NACTTTGGCT GTGCCCGTTA GAATTTATCT 60
TCCATAAAGT ATTTCTCCCA TTGAGTCTAA TGATGTATAC TTTGCCTAGG TCTTTCCAAA 120
ATTAAATTTA TGTAAATGTC TATTTTATAT AAAATATGNT TAAAATAAA 169

SEQ ID NO:6937
SEQUENCE LENGTH:314
SEQUENCE TYPE:nucleic acid
TOPOLOGY:linear
CLONE:HUMGS08169
SEQUENCE DESCRIPTION:
GATCACATTT AGGAGCAAGC AGGTGAGGGC AGAGGTGCTG GAGTGAACGT CGGCTCTTTG 60
GCTGGTAGCC TTACCCACTG AGGGAGAGGA GCTGAAACAT GAAATGGGGA TGCGTGANCA 120
ATGTCTCCCT TTTGCAGAGC TGTATTGACT TGTTCAAGAA TAACTGATAT GCCTTCACTC 180
AGAAGAAAAG AAATGAATGT GAAAGAAAGC CAAGCATCNN TTTGCACTTA AATCANTTAC 240
CACGGAAGNT ATATTTAGCT TCAACTTTAG TTTAAAATTA TGTGAATTAA ATATTTTGAT 300
TTCCCTGGGN NAAA 314

SEQ ID NO:6938
SEQUENCE LENGTH:46
SEQUENCE TYPE:nucleic acid
TOPOLOGY:linear
CLONE:HUMGS08170
SEQUENCE DESCRIPTION:
GATCAAGTGA CTGTTTAATA AATNAGTTGC TTGGGTAGAA TGGAAA 46

SEQ ID NO:6939
SEQUENCE LENGTH:21
SEQUENCE TYPE:nucleic acid
TOPOLOGY:linear
CLONE:HUMGS08172
SEQUENCE DESCRIPTION:
GATCTTGCTG AAAACCAGAA A 21

SEQ ID NO:6940
SEQUENCE LENGTH:85
SEQUENCE TYPE:nucleic acid
TOPOLOGY:linear
CLONE:HUMGS08173
SEQUENCE DESCRIPTION:
GATCAACAAT CATCACCTGC CTTTTGTAGA AAAGAAAAAC AAAAAAAGTA AATAAAAATT 60
TTAAACAGTA AAATAAAAGT TTAAA 85

SEQ ID NO:6941
SEQUENCE LENGTH:274
SEQUENCE TYPE:nucleic acid
TOPOLOGY:linear
CLONE:HUMGS08174
SEQUENCE DESCRIPTION:
GATCACTACT GCAGCAGAAT GGTCTATGTN ATTTTCCTTC TTTGGTTTTT NCCTGACTTA 60
CATTCGTAAT TTTAAGAAAA TTTCTTTACG GGTGGAAGCC AATTTACATG GATTAACCCT 120
CTATGACACT GCACCTTGCC CTATTAACAA TGAACGAACA CGGCTACTTT CCAGAGATAT 180
TTNATGAAAG GATAAAATAT TTCTGTAATG ATTATGATTC TCAGGGATTG GGGNAAGGTT 240
CACAGNAGTT GCTTATTCTN CTCTGTAATT TTAN 274

SEQ ID NO:6942
SEQUENCE LENGTH:246
SEQUENCE TYPE:nucleic acid
TOPOLOGY:linear
CLONE:HUMGS08175
SEQUENCE DESCRIPTION:
GATCTAAGTT GGGCAAAAGA GAGTCATCCT GTTACCTTAA AAAGGAGTCT AGGGTGAGCC 60
CCAGTCTGCT GGTGGCCATC TTCACCACCT TTGGGGAGAG ATGGCCTCNG CAGACACACA 120

AAGCAAAGAG GTGGAGAGAA ACTGTGTCTT TGTGTCATTG TTTGAGTCCC TGGACCCAGC 180
CCAGTCCCCC CGACTACGCT GANCTTTTCA ATTGTGGGGG GCAATAAATT CCATTTAGTT 240
ANGAAA 246

SEQ ID NO:6943
SEQUENCE LENGTH:215
SEQUENCE TYPE:nucleic acid
TOPOLOGY:linear
CLONE:HUMGS08176
SEQUENCE DESCRIPTION:
GATCCACATN ATACCGGTGC TGAAGACCTG CAAGTNATTT TGGAAAAGGG CTGGTGCGGA 60
TGGAAGGGCC CAGATTTTTG GAACAAGGAT GCATACTATA ACTTATGTCT TCCTCAGCGA 120
CCAAATATGA TTTAAAATAT CTTGGAGTCA AAGACTGCAG TAGAGTGGTA TTATAAATTT 180
NTGAATAAAG AATCAGTTTA NTTTTTCACA TTAAA 215

SEQ ID NO:6944
SEQUENCE LENGTH:171
SEQUENCE TYPE:nucleic acid
TOPOLOGY:linear
CLONE:HUMGS08177
SEQUENCE DESCRIPTION:
GATCTGGACA CGAGCAGTCC CTGAGGGGCG GGGTCCCTGG CGTGAGGCCC CCGTNACAGC 60
CCACCCTGGG GTGGGTATTG TGGGCACTGC TGCTCTGCTA GGGAGAAGCC TGTGTGGGGC 120
ACACCTCTTC AAGGGAGCGT GAACTTTATA AATAAATCAG TTCTGTTTAA A 171

SEQ ID NO:6945
SEQUENCE LENGTH:161
SEQUENCE TYPE:nucleic acid
TOPOLOGY:linear
CLONE:HUMGS08178
SEQUENCE DESCRIPTION:
GATCGGCCCC GGAAAAAGTC TGCCCCAGAA ACTCTCACAC TTCCAGACCC TGAGAAAAAA 60
GCCACCCTGA ATTTACCCGG CATGCACTCT TCAGATAAGC CATGTCGGCC CAAATCTGAG 120
TAACTTTATA TAANTATCTC ATGGGGTTTT ATATTTTCAA A 161

SEQ ID NO:6946
SEQUENCE LENGTH:251
SEQUENCE TYPE:nucleic acid
TOPOLOGY:linear
CLONE:HUMGS08179
SEQUENCE DESCRIPTION:
GATCCTTGGG GAAGGTTTCA GTTGCACTGT ATGCTGTTGG ATTTGCCAAG TCTTTGTATA 60
ACATAATCAT GTTTCCAAAG CACTTCTGGT GACACTTGTC ATCCAGTGTT AGTTTGCAGG 120
TAATTTGCTT TCTGAGATAG AATATCTGGC AGAAGTGTGA AACTGTATTG CATGCTGCGG 180
CCTGTGCAAG GAACACTTCC ACATGTGAGT TTTACACAAC AACAAATGAA AATAAATTTT 240

AATTTTATAA A 251

SEQ ID NO:6947
SEQUENCE LENGTH:277
SEQUENCE TYPE:nucleic acid
TOPOLOGY:linear
CLONE:HUMGS08180
SEQUENCE DESCRIPTION:
GATCCCTCTT GGTGTGAGTT TTCTGACCCA ACCAGCCTCT GGTTAGCATC ATTTGTACAT 60
TTAAACCTGT AAATAGTTGT TACAAAGCAA AGAGATTATT TATTTCCATC CAAAGCTCTT 120
TTGAACACCC CCCNCACTTT ANTCCNTNGT TCAGGCCGAT GNGCTTGCTT TCCTTCANCC 180
TGTTTGTTTT CTTATTTAGG NCTATTTATT ANTGGTTGGN CCAATGTACT CACAGCTGTT 240
GCGTCGAGCA GTCCTTNGTG AAANTNCTGT ATAANTN 277

SEQ ID NO:6948
SEQUENCE LENGTH:130
SEQUENCE TYPE:nucleic acid
TOPOLOGY:linear
CLONE:HUMGS08181
SEQUENCE DESCRIPTION:
GATCCAGCTT CAGCTGCACA CCTNTGTCCC CTTGGATGGG GNACTAAGGG NAAACGTCTG 60
TTGTATCACT GAAGTTTTTT GTTTTGTTTC NATACGTGTC TGCATAAAAA TGCCAAAGTT 120
TTTTTTCAAA 130

SEQ ID NO:6949
SEQUENCE LENGTH:101
SEQUENCE TYPE:nucleic acid
TOPOLOGY:linear
CLONE:HUMGS08182
SEQUENCE DESCRIPTION:
GATCACCAAT NNTNNCTTTG AGCCAGCCAA CCAGATGGNG AAATATGACC CTCGCCATGG 60
TAAATACATG GCTTGCTGCC TGTAATACCG TGGTGACGTG N 101

SEQ ID NO:6950
SEQUENCE LENGTH:304
SEQUENCE TYPE:nucleic acid
TOPOLOGY:linear
CLONE:HUMGS08184
SEQUENCE DESCRIPTION:
GATCATGAAG ACCTGCTTTA GCCCCAACAG AGTGATTGGA CTCTCAAGTG ACTTGCAGCA 60
AGTAGGAGGG GCATCAGCTC GCATCCAGGA TGCCCTGAGT ACAGTGTTGC AATATGCAGA 120
GGATGTACTG TCTGGAAAGG TGTCAGCTGA CAATACTGTG GGCCGCTTCC TGATGAGCCT 180
GGTTAACCAA GTACCGAAAA TAGTTCCCGA TGACTTTNAG ACCATGCTCA ACAGCAACAT 240
CAATGACCTT TTGATGGTGA CCTACCTGGC CAACCTCACA CAGTCACAGA TTGCACTCAA 300
TGAN 304

SEQ ID NO:6951
SEQUENCE LENGTH:268
SEQUENCE TYPE:nucleic acid
TOPOLOGY:linear
CLONE:HUMGS08185
SEQUENCE DESCRIPTION:
GATCCTATCT GTNCTGGCAG GACTGGCAGN CATGGTGGGC TACGCCTTGC TCAGNGGCAT 60
TGTCTCCATN CAGNGGGCAA CGCTGCTCGG GCCCCAGGCA CCCGGACCCT GGGCATGGCT 120
GAGGAGGATG AAGAGGAATN ATTTGTCCTC ACGCTCCCAA GACTGGTTTT TNTACTCTNA 180
TGCATTCCAG NGGCCCCCGT GCCTCCTCGT TGTTGGTACA GCCGGACACG GGGTGCTGCC 240
ACCCAGAATA AAGCCACTCA CACTGAAA 268

SEQ ID NO:6952
SEQUENCE LENGTH:152
SEQUENCE TYPE:nucleic acid
TOPOLOGY:linear
CLONE:HUMGS08186
SEQUENCE DESCRIPTION:
GATCCCACCG AAGCAGCGTG TCCTCCGTGG GAGCCCGAGG TGGGTCCCAC CCGCATCTCC 60
TCTTCCACAC CCTCCTGGGA CCCCAGCCCT GGAGAAGTAG CTCTGGCTTG TNTTCTTTTT 120
NATTAAAGAT AATTTATGTA TCGACTAAGA AA 152

SEQ ID NO:6953
SEQUENCE LENGTH:72
SEQUENCE TYPE:nucleic acid
TOPOLOGY:linear
CLONE:HUMGS08188
SEQUENCE DESCRIPTION:
GATCAGTGGG TCTAAGTNTC CGAGACTTAA CGAAAATAGT ATTTCAGCTG CAATAAAGAT 60
TGAGTTTGCA AA 72

SEQ ID NO:6954
SEQUENCE LENGTH:293
SEQUENCE TYPE:nucleic acid
TOPOLOGY:linear
CLONE:HUMGS08189
SEQUENCE DESCRIPTION:
GATCAGCATC CTNCGNTGCC CCTTAGCAAC TNAGGTGGTT GATTTGAAAC TGTGAAGGTG 60
TGATTTTTTC AGGAGCTGGA AGTCTTAGAA AAGCCTNGTA AATGCCTATA TTGTGGGCTT 120
TTAACGTATT TAAGGGACCA CTTAAGACGA GATTAGATGG GCTCTTCTGG ATTTGTNCCT 180
CATTTGTCAC AGGTGTCTTG TGATTGANAA TCATGAGCGA AGTGAAATTG CNTTGANTTT 240
CAAGGGAATT TAGTATGTNA ATCGTGCCTT NGAAACANGG GGGNAAAAAN ATN 293

SEQ ID NO:6955

SEQUENCE LENGTH:184
SEQUENCE TYPE:nucleic acid
TOPOLOGY:linear
CLONE:HUMGS08190
SEQUENCE DESCRIPTION:
GATCCTTCAA TAAAAAATCA ATGTGTGAAG GTTCCTAGGC ATAAAAGGAA GTTCAGAAGG 60
NTATGTAGGA AATACTGTTG TCTCTCTGGG GATTGGGATT ACAGGGATTT TTTCCTCTTT 120
NCTTTTTGTT AAGTTTGTGT TTCCTAAAGT TTCTGCAATA AACGTACGAT GCTTGTATAA 180
TAAA 184

SEQ ID NO:6956
SEQUENCE LENGTH:327
SEQUENCE TYPE:nucleic acid
TOPOLOGY:linear
CLONE:HUMGS08191
SEQUENCE DESCRIPTION:
GATCTTGAAT TTCTACATAA CTTTCTCAGT TTATATGCCC TGTGGCAAGT GCAGCAAGCA 60
CTGTTTCCTG TTTCTAAACT TGTAGAAAAT CATCCATACA TCTTACAGTT GTCAGTTTTA 120
ACCAGATAAC AGTGGCACTT TGTTGCTGCT TTTTTATCTT TAGCTTAGGT TAACAGGACC 180
CTGGAAGTAA AGTTGTTGAT TTATTCAATA GAGTATTCTC AATTAATTTG GCTAGATTTC 240
TACATGATTC AAANTCTAAA AANGTAGACA TGCATGCTTA CATGTCTAAG GCCTGAAAAA 300
TTGGNGGGGN CATCCCAAAA TAAATGN 327

SEQ ID NO:6957
SEQUENCE LENGTH:22
SEQUENCE TYPE:nucleic acid
TOPOLOGY:linear
CLONE:HUMGS08192
SEQUENCE DESCRIPTION:
GATCTAAGTG GTGGTATGGA AA 22

SEQ ID NO:6958
SEQUENCE LENGTH:223
SEQUENCE TYPE:nucleic acid
TOPOLOGY:linear
CLONE:HUMGS08193
SEQUENCE DESCRIPTION:
GATCTGTTGA CAATGGAGCA AGTGAAAGAC TTTGCTGCTA ATGTGTATGA AGCTTTTAGT 60
ACCCCTCAGC AACTGGAGAA ATGATTTTCC CTTCAAGAAA AACTACAGTG GGATTCATTT 120
ACTTTTTAAA ATACACTGGG TAAATCACCT ATACTTAGAG TAACAGTTTG TTATCAAAAT 180
GCCTGNTAAA ATATATNCTT AATAAANGTC TTCATTTCAT AAA 223

SEQ ID NO:6959
SEQUENCE LENGTH:331
SEQUENCE TYPE:nucleic acid

TOPOLOGY:linear
CLONE:HUMGS08194
SEQUENCE DESCRIPTION:
GATCTTACTT GCGAAATGCG ATGGTTGCTG GGAATACCTG AAACTGTGGA TTATATTGCT 60
TGACTTCTAC CTCAGAATCT NCTTTGTTTC ATGACTTAAT AGTGCTTTAA GTNNGGTATA 120
TNATTTNACC NCTAGGAATT CTNTGTTTTA CACAGAAATA AAAATTTTAA AATAGAAAAT 180
GCTTTTACTT TGTAAGGTAA GNGAGTATCC ATATGCTTAG ATGTGCTCGT TTCTAAAATN 240
CTAGNGGTTG CTATANTCAG CTCATGGAAT GCACAGCTAT TGCTTTTTGT GNTAGATTGT 300
ACATAAACAT CAGCAGNTGN AAGGGNAAAT N 331

SEQ ID NO:6960
SEQUENCE LENGTH:211
SEQUENCE TYPE:nucleic acid
TOPOLOGY:linear
CLONE:HUMGS08195
SEQUENCE DESCRIPTION:
GATCACCCAG ATGCAGTGAA GCTTTTNACC CGCCAGCTTT TGGAACTCCA TCAGGGACAA 60
GGCCTAGATA TTTACTGGAG GGCTAATTAC ACTTGTCCCA CTGAAGAAGA ATATAAAGCT 120
ATGGTGCTGC AGAAAACAGG TGGACTGTTT GGATTAGCAG TAGGTCTCAT GCAGTTGTTC 180
TCTGATTACA AAGAAGNTTT AAAACCGCAA A 211

SEQ ID NO:6961
SEQUENCE LENGTH:208
SEQUENCE TYPE:nucleic acid
TOPOLOGY:linear
CLONE:HUMGS08196
SEQUENCE DESCRIPTION:
GATCATTGTA GATGAACTGA AGCAAGAAGT TATCAGTACC AGCAGCAAGG CAGANCCNCC 60
CCAGTGCACC TCCCTGGCCT GGTCTGNTGA TGGCCAGACT CTGTTTGCTG GCTACACGGA 120
CAACCTGGTG CGAGTGTGGC AGGTGACCAT TGGCACACGC TCGAAGTTTN TGGCAGAGCT 180
TTNCAACTAA ANAANAANCT GGCATAAA 208

SEQ ID NO:6962
SEQUENCE LENGTH:165
SEQUENCE TYPE:nucleic acid
TOPOLOGY:linear
CLONE:HUMGS08197
SEQUENCE DESCRIPTION:
GATCCCAGCC CCTAGGTGGG CAGAGGCAGA CCCTCCCCAG AGCTCCTTAG GAAGAAGACA 60
GACTGGTTCA TTGAATGCCG CCTTATTTAT TTCTGGTGAG GATGCATGCG TGGGGCTGCT 120
GGTGTTTAGA GTGGGGGCTA CCCTTNTAAA TCACTGCTAC TCAAA 165

SEQ ID NO:6963
SEQUENCE LENGTH:280
SEQUENCE TYPE:nucleic acid

TOPOLOGY:linear
CLONE:HUMGS08198
SEQUENCE DESCRIPTION:
GATCACTCTN GGTCAGCTNC TCTTTCTTTA TCTNTCTTTC TCCTTTTTTA AGAAAACGAG 60
TTAAGTTTAA CAGTTTTGCA TTACAGGCTT GTGATTCATG CTTACTGTAA AGTGGAAGTT 120
GANCTTATTT TAAAACTTCA AGCTCAGTAA TTTTGAACAC TGNAACATTC ATCTAGGNCA 180
TAATAACAAA GTTCAGTATT GACCATAACT GTTAAAACAA TTTTAAGCTT TCCTCAAGTT 240
AGTTNTGTTG TAGGNGTGTA CCTAAGCAGG TAAGCGTANA 280

SEQ ID NO:6964
SEQUENCE LENGTH:51
SEQUENCE TYPE:nucleic acid
TOPOLOGY:linear
CLONE:HUMGS08199
SEQUENCE DESCRIPTION:
GATCCATATA TGTGTGCATG TCATGAAATA AAAGNATCAC ACANCACAAA A 51

SEQ ID NO:6965
SEQUENCE LENGTH:155
SEQUENCE TYPE:nucleic acid
TOPOLOGY:linear
CLONE:HUMGS08200
SEQUENCE DESCRIPTION:
GATCTTGGAG CTTCCCTGTA GCCCACCTTC CCCGTGCTTC ATGTTTGTAG AGGAACCTTG 60
TGCCGGCCAG GCCCAGTTTC CTTGTGTGAT ACACTAATGT ATTTGCTTTT TTTGGAAATA 120
GAGAAAATCA ATAAATTGCT AGTGTTTCTT TGAAA 155

SEQ ID NO:6966
SEQUENCE LENGTH:58
SEQUENCE TYPE:nucleic acid
TOPOLOGY:linear
CLONE:HUMGS08201
SEQUENCE DESCRIPTION:
GATCCAGCAT TGGGGCCACT ACACTCCAGC CTGGACCACG GAGCGAGACT GTCTCAAA 58

SEQ ID NO:6967
SEQUENCE LENGTH:111
SEQUENCE TYPE:nucleic acid
TOPOLOGY:linear
CLONE:HUMGS08202
SEQUENCE DESCRIPTION:
GATCAGGCTT CAGGATAGCC CAGCACTGAG GACATCTAAA ACAGTCTTCA TTTATTTATT 60
TATTGTTCAT AGTGTCCAAA AGTTGGAATT AAAGTGAATG GCTGAATCAA A 111

SEQ ID NO:6968

SEQUENCE LENGTH:39
SEQUENCE TYPE:nucleic acid
TOPOLOGY:linear
CLONE:HUMGS08203
SEQUENCE DESCRIPTION:
GATCATGACA AAGAAGATTA AAATTTCATT AGCATGAAA 39

SEQ ID NO:6969
SEQUENCE LENGTH:90
SEQUENCE TYPE:nucleic acid
TOPOLOGY:linear
CLONE:HUMGS08204
SEQUENCE DESCRIPTION:
GATCAATTGT ACCCCATACC TCAGCATCAT GCAATATAAC CATTAAGAGA CCTGCACATG 60
TATTCCCTGA ATTTAAAATA AAAGTTGAAA 90

SEQ ID NO:6970
SEQUENCE LENGTH:315
SEQUENCE TYPE:nucleic acid
TOPOLOGY:linear
CLONE:HUMGS08205
SEQUENCE DESCRIPTION:
GATCATTGCA GTCTCTGGCT TTTTNACTCC AGGCGGGCTC CGTGCCCTCC GGGGCCTCTT 60
CTTCCTTGTN AGGCCAGTGG GAGCCACAGA GGCCTCATTG GCTTCCTATG AATATNAAAT 120
GGGATTCTCT GTGGGGGAAG CTTCCATAAG GCAGGAAGGG GTCCCCGGTT GTTGGCTATN 180
AGTGTTATTT NCTTTTAAAT CATCTGTGCT CCTTCAAAGA CGACGTAAGA AGCTTGTGCC 240
CTGGCGTTGC AGAAATCTCT GGCTGTAGGG ATTAAACCTA CATGTCTTCC TGATGGGGTG 300
GTGAGGACGT AAACN 315

SEQ ID NO:6971
SEQUENCE LENGTH:262
SEQUENCE TYPE:nucleic acid
TOPOLOGY:linear
CLONE:HUMGS08206
SEQUENCE DESCRIPTION:
GATCACCTGA GGTCAGGAGT TCACGACCAG CCTGGCCAAC ATGGTGAAAC CCTTCTCTAC 60
TGAAAATACA AAAATTAGCC AGGTGTGGTG GCGCATACCT GTAGTCCCAG CTACTTGGGA 120
GGCTGNGGCA GGNGAATCAC TTGAACTCGG GAGGCGAAGG TTGCAGTGAG CCGAGATTTC 180
ACCAGTGCAC TCCAGCCTGG GTGACAGAGC AAGACTCCAT CTCAAAAAAA ANNTAATTAN 240
AAAAAATTGC AGCTGCNGGA AA 262

SEQ ID NO:6972
SEQUENCE LENGTH:118
SEQUENCE TYPE:nucleic acid
TOPOLOGY:linear

CLONE:HUMGS08207
SEQUENCE DESCRIPTION:
GATCTTGCAG GTCTAGTATT TNAATAATGC ACTATTACCC AGGGCAGNTA TTATGAGAAA 60
CTGTTTCTNC TCTAAGGGTT TATGGCAGAC TTTGCTTTTT NAACATGTGA GANATGAN 118

SEQ ID NO:6973
SEQUENCE LENGTH:152
SEQUENCE TYPE:nucleic acid
TOPOLOGY:linear
CLONE:HUMGS08208
SEQUENCE DESCRIPTION:
GATCTGAGTA TTCCCCAAGG GCACTCAGCC ACAGGGNTGA ACTAGTGTTT GTTTGTATCC 60
TTGCAGTTAT GTGACTTAAA GTGACTCAGA GATATCTATG TATGCNNGTG TACTCCGCTG 120
GCAATGAAGT AAATATATCA ATTTGTGGNA AA 152

SEQ ID NO:6974
SEQUENCE LENGTH:297
SEQUENCE TYPE:nucleic acid
TOPOLOGY:linear
CLONE:HUMGS08209
SEQUENCE DESCRIPTION:
GATCCTCCTG CTTTGGCCTC CCAAAATGCT GGGNTTATAG GCATGAGCCG CTGCACTTGG 60
CCTGNTACTG ATTTTAATNN CTTGCGTTAT CACATAGTGT TGTATTTGAA ACATAGTTCA 120
TGGTTTTATC AAAGAACTGA AGNTGAGAAT ACTGGTCATC TAACTTTGTA ATTTGATTTG 180
ATTATACTGT AAAGTTTGAC AGTCTCATTT TATCACTGCG TTTGTATCTA TNACTAAANT 240
GTATTTNTTG ACCTCTTACT GATTCATTGT TGGTNTGTNC AAACTGTTGA CTNGNTN 297

SEQ ID NO:6975
SEQUENCE LENGTH:301
SEQUENCE TYPE:nucleic acid
TOPOLOGY:linear
CLONE:HUMGS08210
SEQUENCE DESCRIPTION:
GATCCTGTCA CCCAGGTAGT GAGTATAGCA CCCAGTGAAA CTGTAGTCTC ATNCCAGGCA 60
CTGTGCTAGC CCACTCTGGG TCATTTAATC CTCTCCTAAG AAGAGAGGAG ACACAGCGTC 120
CCCATTTGAC AGATGCAGAA AGAGGTTCCA CAGGTGTGCN TTGATTCTNT CCTAAAACCG 180
TTTCCCGGAA GCTTTTCCTG GTGTGGGCGC TTCTAACCTA ATCCTCAATC GNTTCCAGNA 240
CTATTACTCT GTTTCCACAG TGNTACTGTG TCTAGGTTTT ANGGGCGGCA AGTTCNATTT 300
N 301

SEQ ID NO:6976
SEQUENCE LENGTH:264
SEQUENCE TYPE:nucleic acid
TOPOLOGY:linear
CLONE:HUMGS08211

SEQUENCE DESCRIPTION:
GATCCTTCCG ACTTGGCCTC CCAAAGTGTT GGGATTACAG GCATTAGCTA CCACACCTGN 60
CCAAGGCCCA GGTTTCGACA GAAAGGGAGA GAAAACCTGC CAGAGATGCC ATTTCGGAGC 120
CACTCTGCTT GGCAGGGACC TGTGTTCCCC TCATGCAGGT TCATCCTTAG AGGGCTGCGG 180
TCTTATCTGG TTGTGCAAAA GTCCCACAAC CTTTCTGGAT TGATAGTTTG TGGTGAANNT 240
AAACAATTTT AGTTTGTTTG GAAA 264

SEQ ID NO:6977
SEQUENCE LENGTH:318
SEQUENCE TYPE:nucleic acid
TOPOLOGY:linear
CLONE:HUMGS08212
SEQUENCE DESCRIPTION:
GATCAGAACT ACAATTACAA CCAGCATGCG TATCCCACTG CCTATGGTGG GAAGTACTCA 60
GTCAAGACCC CTGCAAAGGG GGGAGTCTCA CCTTCTTCCT CGGCTTCCCG GGTGCAACCT 120
GGCCTGATGN AGTGGGTGAA GTTTTGGTAG GCAATTTCTT GCAACCACCA CCGNGGCCCC 180
GNAAAGCACT GGTCGTNAGG GAGCTCCTNN CCTTGGCCCN CACGCCTGTG CCAGCCCTGG 240
NCCCGNCTGC CACACCTCTG TTTCCTANGG CTGGGGACNC AGATTGTNTC TCCTTNTTTC 300
TTNCNACTGC AATGTGCN 318

SEQ ID NO:6978
SEQUENCE LENGTH:166
SEQUENCE TYPE:nucleic acid
TOPOLOGY:linear
CLONE:HUMGS08213
SEQUENCE DESCRIPTION:
GATCATNCTG CCCCATNTCC ACGCACCCAA AACCAGCCCT CTCGCTTCTA ACACAGGGCA 60
CCTGCTGGGG CTCAGGGATG TTAGGGACGA GTTCCAGNCC TGCCACTNCC CTNGGGCGAC 120
CCCTCNCTGT CCCTGCCTCC CTGCTCTGCC GCCCCTNTTN CTGGAN 166

SEQ ID NO:6979
SEQUENCE LENGTH:105
SEQUENCE TYPE:nucleic acid
TOPOLOGY:linear
CLONE:HUMGS08214
SEQUENCE DESCRIPTION:
GATCCGATAC AGATTACATT TCTTGCCAGA GAGTGTTGCT GTTGTTTCTT TAGGTAAGTG 60
TGTATTGGTG ATTCCATAAT AAAAGCAGTA ACAATAACTA CCAAA 105

SEQ ID NO:6980
SEQUENCE LENGTH:233
SEQUENCE TYPE:nucleic acid
TOPOLOGY:linear
CLONE:HUMGS08215
SEQUENCE DESCRIPTION:

GATCAGATAC ATGAACATGT CTTACATGGG TTGCTGTATT TAGAATTATA AACATTTTTC 60
ATTATTGGAA AGTGTAACGN GGACCTTCTG CATACCTGTT TAGAACCAAA ACCACCATGA 120
CACAGTTTTT ATAGTGTCTG TATATTTGTN ATGCAATGGT CTTGTAAAGG TTTTTAATGN 180
AAACTACCAT TAGCCAGTCT TTCTTACTGA CANTAAATTA TTAATAAANT AAA 233

SEQ ID NO:6981
SEQUENCE LENGTH:72
SEQUENCE TYPE:nucleic acid
TOPOLOGY:linear
CLONE:HUMGS08216
SEQUENCE DESCRIPTION:
GATCTTTCCA GAGGTCCATG GTGGAAGACG ATAACCCTGT GAAATACTTT ATAAAATNTC 60
TTAATGTTCA AA 72

SEQ ID NO:6982
SEQUENCE LENGTH:58
SEQUENCE TYPE:nucleic acid
TOPOLOGY:linear
CLONE:HUMGS08217
SEQUENCE DESCRIPTION:
GATCACCCAT CTGTGTGCTT CCATCCTGCA TTAAAATTCA CTCAGTGTGG CCCAGAAA 58

SEQ ID NO:6983
SEQUENCE LENGTH:280
SEQUENCE TYPE:nucleic acid
TOPOLOGY:linear
CLONE:HUMGS08218
SEQUENCE DESCRIPTION:
GATCTCCAAC AGCCAGTGTG TGTTTCCCAT CTCTTGTAGG TTCCATCAAT GGTGAGCACC 60
AGCCTGAATG CAGAAGCGCT CCAGTATCTC CAAGGGTACC TTCAGGCAGC CAGTGTGACA 120
CTGCTTTAAA CTGCATTTTT CTAATGGGCT AAACCCAGAT GGTTTCCTAG GAAATCACAG 180
GCTTCTGAGC ACAGCTGCAT TAAANCAAAG GAAGTTCTCC TTTTGAACTT GTCACGGATT 240
CCATCTTGTA AAGGATATTA AATGTTGCTT TAACCTGAAA 280

SEQ ID NO:6984
SEQUENCE LENGTH:311
SEQUENCE TYPE:nucleic acid
TOPOLOGY:linear
CLONE:HUMGS08219
SEQUENCE DESCRIPTION:
GATCCAGAGA CTCGTGCTGT CTCTTTGTCC CTTTCCTGAT GGTGTTACTT CGATTAACTA 60
CTTCAGCTTG GTGTAAAAAT TGCCAAAGCA GTTGCCTGCC ACAACTTTGT AAAAGCCAAA 120
AAGGAGGTTG AAAATTCACA GGCTGCCCGA AAAAAGANGA AACTTGCATG GGGGTTTGAA 180
GCAAAGNNGN GATGGGAAAC CNAAAGCAAC ATGGGNTACA TGTAACTTGC CAGAGTGCTT 240
CAAGACATTT GTAGCCTCAA ATGCTCAAAT TTANTGNGAA TGTTTTCCTG CCTATGTTCA 300

TATGTCAGGT N 311

SEQ ID NO:6985
SEQUENCE LENGTH:160
SEQUENCE TYPE:nucleic acid
TOPOLOGY:linear
CLONE:HUMGS08220
SEQUENCE DESCRIPTION:
GATCTGATGG TTTTATAAAG GGGNGTTCTG CTGCATACAC TCTGTCTTGC CTGCTGNCAT 60
GCAAGATGTG ACTTAGCTCC TCCTTTACCT TCCACCATGN TTGTNAGGCC TCCCCAGCCA 120
TGTGGAACTG TGAGTTGANT AAACCTCTTT CCTTTATAAA 160

SEQ ID NO:6986
SEQUENCE LENGTH:115
SEQUENCE TYPE:nucleic acid
TOPOLOGY:linear
CLONE:HUMGS08221
SEQUENCE DESCRIPTION:
GATCAAAAGA TAACTGCTTA TNATAGTAAG ATAACCTTGG AACAGAACTG TGATTAAAAA 60
CCTGAATAAT AAAAAACACA CAAGACCCAT TAAAACCTAT TCCTCACTTT TTAAA 115

SEQ ID NO:6987
SEQUENCE LENGTH:331
SEQUENCE TYPE:nucleic acid
TOPOLOGY:linear
CLONE:HUMGS08222
SEQUENCE DESCRIPTION:
GATCCAGTTG GATATAGTAA CCACTGCCGA CTTNCTGGAT GTGCTAACTC ACACCAAGCC 60
CTCCGNAAAG AATCTGGCTC AGAGATACTC AGACTGGCAA AGAGAATTCG AGTCTGTCTG 120
AAACCACATT TACCCTGACC TGGCCACAAA GGCAACCACA AAGACCTCCT AGTTTATTAN 180
NGNNCGTGGG AGAACAAAAT GATTGGAATG GAAAAGAGAA AATTATTTTT GANGACTGGA 240
TTAACTTGAG CCACTGTATT GTTTTGGNTA GCTGAGATAT ATTTNTTAAC TTTACCATTA 300
TCGATGTCAG CAAAATATTG AGAGTTTCTT N 331

SEQ ID NO:6988
SEQUENCE LENGTH:121
SEQUENCE TYPE:nucleic acid
TOPOLOGY:linear
CLONE:HUMGS08223
SEQUENCE DESCRIPTION:
GATCTACTCC CAGTTTTTAA CTTTTNACAG AATATTGTTG CTTTCCTACA ACAATGTACA 60
TATATTTNCA GTTGTATATG CTTTTTTTTT TNCCAAATAA AGTTTGAGTT CTAAACTCAA 120
A 121

SEQ ID NO:6989

SEQUENCE LENGTH:151
SEQUENCE TYPE:nucleic acid
TOPOLOGY:linear
CLONE:HUMGS08224
SEQUENCE DESCRIPTION:
GATCCTTCCC TGGCTCTCAC CTGCTCCTTT AGGCTGTAAT TGCTTCCTAC ACACGAAGCC 60
TCTGATTGGA GCCTCTGGTN CATCTCAGAA AAACCTTCCA AGAGCGCTGG GGTTTATGCT 120
TTCTGAATAA ACACTACTGT TTACATGGAA A 151

SEQ ID NO:6990
SEQUENCE LENGTH:119
SEQUENCE TYPE:nucleic acid
TOPOLOGY:linear
CLONE:HUMGS08225
SEQUENCE DESCRIPTION:
GATCTCCATA ATTTTAAGAT ACTCTATGTT AAACTATTTT TNAAGTNCTC TTTTTACATC 60
ACGTCTGAAA TGCACGAGNG TGGCGGTTTC TGTTTCACTG GTTTNNTTGT TCATTTTTN 119

SEQ ID NO:6991
SEQUENCE LENGTH:116
SEQUENCE TYPE:nucleic acid
TOPOLOGY:linear
CLONE:HUMGS08226
SEQUENCE DESCRIPTION:
GATCCCGGGC CGTTATCCAT CTGGAGGCTG CAGGGTCCTT GGGGTAACAG GGACCACAGA 60
CCCCTCACCA CTCACAGATT CCTCACACTG GGGAAATAAA GCCATTTCAG AGGAAA 116

SEQ ID NO:6992
SEQUENCE LENGTH:207
SEQUENCE TYPE:nucleic acid
TOPOLOGY:linear
CLONE:HUMGS08227
SEQUENCE DESCRIPTION:
GATCCGCTGG GACAGAGTGC CCACCGTCGT GTCTTTNATA TCTCTGTAAT AACCCACAGT 60
TAAAAAGGGC AGGAAAAGGC ATCAGGGTAC CATGTTATCA GGCAAGTTCT TACATTCTAA 120
GAAAATTCCT AGCTTTGTTG GGTGAGGCAA GTTTTTAAAN GAANAATGTC ACTTCCCCAT 180
TTGATAANGA TTTCGTTATC ANTTAAA 207

SEQ ID NO:6993
SEQUENCE LENGTH:325
SEQUENCE TYPE:nucleic acid
TOPOLOGY:linear
CLONE:HUMGS08228
SEQUENCE DESCRIPTION:
GATCCTGATT GTAAGGTTTT CCACCTATTT CCCTGGATAT TTTNNTGGTC AGTACTGGCT 60

CTGGTGGGTG TTCCTTGTTT TAGGCTTTCT CCTGTTTCTN AGAGGATTTA TCAATTATGC 120
AAAAGTTCGG AAGATGCCAG AAACTTTCTC AAATCTCCCC AGGACCAGAG TTCTCTTTAT 180
TTATTAAAGA TGTTTTCTGG CAAAGGCCTT CCTGCATTTA TGAATTNTGG TCTCAAGCAG 240
CAAGNGAACA CCTGCAGGAN GTGAATCAAG CTGCAGAACA CAGAGGNNTA CTCACCTGCT 300
TTAAAAAAAT ANAGTACTGT TGATN 325

SEQ ID NO:6994
SEQUENCE LENGTH:130
SEQUENCE TYPE:nucleic acid
TOPOLOGY:linear
CLONE:HUMGS08229
SEQUENCE DESCRIPTION:
GATCCCTCGG GNNTTAANTA CANGAAACCT AGAACTGACC AAAAGGCATT ATAACTCTGA 60
CTCAAATACA AGGTACAGAA GATAAGCATC TTTGAGGAAA CTCCTACTTC AGTTCTTTTG 120
TTATNATGNN 130

SEQ ID NO:6995
SEQUENCE LENGTH:116
SEQUENCE TYPE:nucleic acid
TOPOLOGY:linear
CLONE:HUMGS08230
SEQUENCE DESCRIPTION:
GATCATATAA CATTCCAGGT ACTCAGGATG AATGGTTTGA GGACTGGTCT GAATTCTTCA 60
AAGGTTTCAG CTGTATTAAC ATTCTCCATC TAATAAACTT TATCTTGTCA TTGAAA 116

SEQ ID NO:6996
SEQUENCE LENGTH:33
SEQUENCE TYPE:nucleic acid
TOPOLOGY:linear
CLONE:HUMGS08232
SEQUENCE DESCRIPTION:
GATCAGCAAA TAAATGAAAT TTATTAAAAG AAA 33

SEQ ID NO:6997
SEQUENCE LENGTH:246
SEQUENCE TYPE:nucleic acid
TOPOLOGY:linear
CLONE:HUMGS08233
SEQUENCE DESCRIPTION:
GATCTTTAGG CAGTTTTTAC TTGGCCAGAA AGCAGTGCTG AATACTTGNA ACTGTGTGCT 60
CTGTTCTACT TAATGTTCTG TCAGAATGTT CTTTTGTAGG CAGTATGTCA TGNTGTAATC 120
ATCTATCTCC TNGTCTGTTT CCAAGTTNNN CTGTGAAGTC TGCGACCCTT TTGNGGTGGT 180
CATCANAGTC ACAGATTCCT TGTTTAACCA NGTGTCCCNA AGCATGTACC TGNAGTTATA 240
TCATNN 246

SEQ ID NO:6998
SEQUENCE LENGTH:186
SEQUENCE TYPE:nucleic acid
TOPOLOGY:linear
CLONE:HUMGS08234
SEQUENCE DESCRIPTION:
GATCATTATG CAATAAAGCT GCTAAAGTCA CATTTGTGGT TGATACTCAA GCCTTGTACA 60
AGCACATATC ATGTAGACAT TTTTAAATGT GAAGCTTTAA CAGGNATAGG ATAGCTGTTT 120
CAAACCTTGT GCAATTGTAA ATAANTTGTT TAGCTGTGTT TCTTTCATTT TTAATANAAA 180
NTTAAA 186

SEQ ID NO:6999
SEQUENCE LENGTH:154
SEQUENCE TYPE:nucleic acid
TOPOLOGY:linear
CLONE:HUMGS08235
SEQUENCE DESCRIPTION:
GATCTTCATA TTAATCTTAA AATTTTGTGA CGTGTCTTTT TCCTTTTTTT CCACAGTTTT 60
AATATATNAT TCTTCAACGA CATTTTTTGT AACTTTACAC TTTTTTGGTT ATNTTATTTT 120
AAAAAAATGA AAAATTAATT TAAAAAAATG CAAA 154

SEQ ID NO:7000
SEQUENCE LENGTH:195
SEQUENCE TYPE:nucleic acid
TOPOLOGY:linear
CLONE:HUMGS08236
SEQUENCE DESCRIPTION:
GATCTGGTAC GNGGNGGACG NCATGGCCCG AGATGCCCAC GGGAACACAG CGCTGACCTA 60
CGCCCGNCAG GCCTCCAGCC AGGAGTGCAT CAACGTGCTT CTGCAGTACG GCTGCCCCGA 120
CGAGTGCGTG TAGTATCTGT TTTATTTNNC TGNAGTCTCC TTGGTGTAAA AACAAAATNG 180
GNAAAATAAG GATAN 195

SEQ ID NO:7001
SEQUENCE LENGTH:163
SEQUENCE TYPE:nucleic acid
TOPOLOGY:linear
CLONE:HUMGS08237
SEQUENCE DESCRIPTION:
GATCAGGCCC TAAAACGTTG TTGTGATGAG GTTTCTTTAG CAAGTTCTTG TTTAAATTAT 60
CATTTATTTG ATGAGTGAAG TTTTTAACAT GCTTTGCTGT GTGAAATTTA AAAAAGGGAT 120
GTTTTTCCAG GCTGGAACAA TAAATGTGGC TGTGCAGTTT AAA 163

SEQ ID NO:7002
SEQUENCE LENGTH:127
SEQUENCE TYPE:nucleic acid

TOPOLOGY:linear
CLONE:HUMGS08238
SEQUENCE DESCRIPTION:
GATCTTTGTG AAAGTAGTAC AGTATATNAC CTTTAATTNC TTTTTTATTT TAAATATACT 60
GTNACCNTGA AGCACTGGTT GGGCATTTTA ATTCATGTTA ATAAATCACA ATTATGTCAG 120
TTTTAAA 127

SEQ ID NO:7003
SEQUENCE LENGTH:123
SEQUENCE TYPE:nucleic acid
TOPOLOGY:linear
CLONE:HUMGS08239
SEQUENCE DESCRIPTION:
GATCAGCCTC CTGTCTTATG TGGGCACGTT CCAAAGTTTA AATGCATTTT TTTGACTCTT 60
GGCCAAAATT TAGAAGATGC TGTGAATATC ATTTTGAACT TGTGTAAATA CATGAAAGAG 120
AAA 123

SEQ ID NO:7004
SEQUENCE LENGTH:260
SEQUENCE TYPE:nucleic acid
TOPOLOGY:linear
CLONE:HUMGS08240
SEQUENCE DESCRIPTION:
GATCTCAGCA TTACCTCTTT GGNAAAGGAA GGTAGTTCAA GAAATGAAGA GCTGTTGATG 60
GGATGATTGA AGAAACAGCT ATGAGAGGAT TGGCTCCCAT CTTTTGTNAC TCTTGGGNCA 120
TCCTGTCATC TGAGAATGAA CAAAGACCAA TTTTTTGTGT GTGAAGCTTA AGGGTCATAT 180
GTTTGCTTGT NTTTNTNAAT GCTAATCCTT GTGAAANTAA ATTGNCCAGG CGGAAGGAAA 240
NCTCTATTTT AGGATNCAAA 260

SEQ ID NO:7005
SEQUENCE LENGTH:79
SEQUENCE TYPE:nucleic acid
TOPOLOGY:linear
CLONE:HUMGS08241
SEQUENCE DESCRIPTION:
GATCTGTATT TNTATATACA CGTGTCCGNA CAAGTATAAC TAAATAAAAA TTAAAGNTTT 60
TAATCATTTT AATTGGAAA 79

SEQ ID NO:7006
SEQUENCE LENGTH:59
SEQUENCE TYPE:nucleic acid
TOPOLOGY:linear
CLONE:HUMGS08242
SEQUENCE DESCRIPTION:
GATCATATTT CTGCCAAGNA TGTGCCTTCA ACTTTATAAT TATAGTGTTG TAAAATAAA 59

SEQ ID NO:7007
SEQUENCE LENGTH:67
SEQUENCE TYPE:nucleic acid
TOPOLOGY:linear
CLONE:HUMGS08243
SEQUENCE DESCRIPTION:
GATCTCACTT TTNCTTATTA GAATTTGTAC CTTTGTTTGA TTAAGGAAAT AAAAGATGAA 60
TGACAAA 67

SEQ ID NO:7008.
SEQUENCE LENGTH:76
SEQUENCE TYPE:nucleic acid
TOPOLOGY:linear
CLONE:HUMGS08244
SEQUENCE DESCRIPTION:
GATCAGTCTC TCAGTGCCTN CCTACTGGGC AGCTCATCTG TCCACTTATT CGTATTAAAT 60
TTNCTTTTTA TTTAAA 76

SEQ ID NO:7009
SEQUENCE LENGTH:162
SEQUENCE TYPE:nucleic acid
TOPOLOGY:linear
CLONE:HUMGS08245
SEQUENCE DESCRIPTION:
GATCCACATC CCANATGCTG AACCAGAGAG CCCCTCATCC CTCCTCCCAG TACTTAGGCA 60
GCGCCTTCTG TGTGCACTGC CTCTTGCCGG GCACTGGGTA GGTGCACACG NGGACCGGGA 120
CAGGGCCAGA NACTTTGGTN CCCGNGGTTT GTNTGNGGGG NN 162

SEQ ID NO:7010
SEQUENCE LENGTH:58
SEQUENCE TYPE:nucleic acid
TOPOLOGY:linear
CLONE:HUMGS08246
SEQUENCE DESCRIPTION:
GATCGATTAA GTCTAGGAGT TTNAGACTTG CCTGGGCTTA GCAAGACCCT GTGGCAAA 58

SEQ ID NO:7011
SEQUENCE LENGTH:275
SEQUENCE TYPE:nucleic acid
TOPOLOGY:linear
CLONE:HUMGS08247
SEQUENCE DESCRIPTION:
GATCCTTGAG TCTCCNCTCT GGATGGAATC CGCGAGCTGG CCACCTGGCC ACCCTCTACA 60
CGACTCCACC CNNCCATGGC CGTGGGGCCC TTACTCTCTG ACTTCTCAGG ACACAGGTCA 120

TGGAGGTTCT TCCCAAGCTG GCAGAGGCCA TTTGTGGAAA GTGGAGAGCT ACGTGGTGGN 180
CATCTGCCAA CTCCAGCATC TCTGGNGGGG NTCCACGNTG AATGTNATTT TTGAAAACAG 240
CTTATGTANT TAAAGGTTGA ATGGCACATC ATAAA 275

SEQ ID NO:7012
SEQUENCE LENGTH:124
SEQUENCE TYPE:nucleic acid
TOPOLOGY:linear
CLONE:HUMGS08248
SEQUENCE DESCRIPTION:
GATCAAGACC ACCCTGGCTA ACACGGTGAA ACCCTGTCTC TACTAAAAAT ACAAAAANTT 60
AGCCGGGCAT GGTGGCACAC GCCTGTACAT CCCAGCTACT CANGGNGGCT NGTGGCATGG 120
NGNN 124

SEQ ID NO:7013
SEQUENCE LENGTH:215
SEQUENCE TYPE:nucleic acid
TOPOLOGY:linear
CLONE:HUMGS08249
SEQUENCE DESCRIPTION:
GATCANGAGC CCTTTTNCTC CAGTAGTACA GGCTTTGAAA ACTACTTCTA TTAAGTTATT 60
GATGCAATTT GATATTTTTT CATAATCTAT ATTTAAACAA AATTACATCA TTGCATCATC 120
TTTTCTAAAT TCATCTCCAT TAAAACTTGC CTTAAGCTAC CAGNTTGCTT TTGCCANNGN 180
NGGCCATACT GTGTGTTTGT TTGTTTAATN NNCTN 215

SEQ ID NO:7014
SEQUENCE LENGTH:373
SEQUENCE TYPE:nucleic acid
TOPOLOGY:linear
CLONE:HUMGS08250
SEQUENCE DESCRIPTION:
GATCTCTCAG GGATAGAAGA TATTTCTCAT GAAGGCAGCC TAACTCTGAG GAAAACAATG 60
CCAATTCAAG TACAGATTTC AACACATCTT CAACACTATG TGAAGGGTTC ACATCTTAAC 120
CTGTGCAATT CAGATTGATA CTCAGAATAT GGGTTGATTT GAATATCTGA AATATCAATG 180
GAAAATCCCA CTNNGTTTTT GATGAACAGT TTGAACAGTT TTCTGTAATC AAGCAGCTTG 240
CATAGAAATT GTATGATGAA ATTTTNCGNN GGTTCTTGGT GCTGTTTTGT TCTTTTTTTG 300
TTTTTTGTTG TTTTGTTATT TACTTATATA CATATAANCT TTTNTTGAAA ATATGTTTTG 360
GTTACTAANA GTN 373

SEQ ID NO:7015
SEQUENCE LENGTH:219
SEQUENCE TYPE:nucleic acid
TOPOLOGY:linear
CLONE:HUMGS08251
SEQUENCE DESCRIPTION:

GATCCCAACC TGGACCCACT CATGAACCCT CACATCCGCG TGGGCCCCTC CTNAGCCCCN 60
TTGCTTGTGG CTAGGCCAGC CTAGGATGTG GNTTCTGTGG AGGAGAGGCG GGGTAATGGG 120
GAGGCTGAGG GCACCTNTNC ACTGCCCCTC TNCNTCAAGC CTAAGNCACT AAGACCCCAG 180
ACCNAAAGCC AAGTCCACCA GAGTGGCTGC AGGNCAGGN 219

SEQ ID NO:7016
SEQUENCE LENGTH:91
SEQUENCE TYPE:nucleic acid
TOPOLOGY:linear
CLONE:HUMGS08252
SEQUENCE DESCRIPTION:
GATCGCTTGA ACCCATAAGC TGGAGACCAG CCTGTGCACA TGGTGAGACC CTGTCCCTAC 60
CAAAAAAAAT AAAGTCATTA GAGTTTTTAA A 91

SEQ ID NO:7017
SEQUENCE LENGTH:176
SEQUENCE TYPE:nucleic acid
TOPOLOGY:linear
CLONE:HUMGS08253
SEQUENCE DESCRIPTION:
GATCTGATGG TNTTATAAGG GGCTTCCCCC TTCACTCAGC TCTCATTNTT CTCTCTCCTG 60
CCACCATGTA AAGAAGGATG TGTTTGGTTT CCCTTCCATC ACGATTGTAA GTTTCCTGAG 120
GCCTCCTAAG CCATGTGGAA CTGTGAGTCA NTTAAATCTC TTTACTNTAT NNNGNN 176

SEQ ID NO:7018
SEQUENCE LENGTH:174
SEQUENCE TYPE:nucleic acid
TOPOLOGY:linear
CLONE:HUMGS08254
SEQUENCE DESCRIPTION:
GATCATCACT TACCCACCCC CCAAGTTCAA GACCAAATCT TCCAGCTGCC CNNTTCGTGT 60
TTCCCTGTGT TTGCTGTAGC TGGGCATGTC TCCAGGAACC AAGAAGCCCT CAGCCTGGTG 120
TAGTCTCCCT GACCCTTNTN AATTCCTTAA GTCTAAAGAT GATGAACTTC TAAA 174

SEQ ID NO:7019
SEQUENCE LENGTH:45
SEQUENCE TYPE:nucleic acid
TOPOLOGY:linear
CLONE:HUMGS08255
SEQUENCE DESCRIPTION:
GATCCTCCCT TTCCTATAGN GNTAAAAGTG ATTTATCTTG GCAAA 45

SEQ ID NO:7020
SEQUENCE LENGTH:109
SEQUENCE TYPE:nucleic acid

TOPOLOGY:linear
CLONE:HUMGS08256
SEQUENCE DESCRIPTION:
GATCATGTGA TGTTGGAGTA GGAGGGTTTA TNTTGGTGAC TTTGGGATGC TGGTTTCATT 60
GTAGGTATAA TNATGNGGNG CAAAGAATCC AGGNAAGAAT TGCCAGAGN 109

SEQ ID NO:7021
SEQUENCE LENGTH:225
SEQUENCE TYPE:nucleic acid
TOPOLOGY:linear
CLONE:HUMGS08257
SEQUENCE DESCRIPTION:
GATCCCACTG AGGCCAGAGA ATAGGGTCTG GAGACAAAGG AGCATTCACT TCAGCCTCTG 60
ACTGGTGGCA GGCCAAGTNT TTATTTACAT AGGGTGTAAC CAAATAGGAA ACCTCTAAAG 120
GGTACTTAAA CCCCAGATTT TNTACACAGG GCACTTGCTT GAGCCTCATC CCGCTTTCTG 180
GAATGTACTT TTGCTTCAAT AAATCTGTGC TTTTGTTCCT TCAAA 225

SEQ ID NO:7022
SEQUENCE LENGTH:196
SEQUENCE TYPE:nucleic acid
TOPOLOGY:linear
CLONE:HUMGS08258
SEQUENCE DESCRIPTION:
GATCTGGAGG GCGTGGCTAC AGGACCCGGG ATGCCATTCA GTTACTCATC TTTNATGCTT 60
TCGTCCTGAC CTGTCTCAAC TAGACTTGCT CCTGCAACCA CCATGGGGGT TTTGCATTTA 120
CATTTGTGGA CCATGTTACA GTTAAGAAAA ATCCTGTTTC AGTCCTTATA TGTAATAAAA 180
TGTTTTATGA TGTAAA 196

SEQ ID NO:7023
SEQUENCE LENGTH:170
SEQUENCE TYPE:nucleic acid
TOPOLOGY:linear
CLONE:HUMGS08259
SEQUENCE DESCRIPTION:
GATCTNTCCT CCGCTTGAGA AGACCATGGA ACTGGATAGC TCACTTCCGG AATACAACAT 60
GTTTCCCCGC AAAGGCCGGG GNCTCCCTAG NTCAAATTTC CAATCTCATN AGCAGGTGNT 120
AGAAAGGTNT TGTGGTAGCA ACTNCATGAT TTCTCTGTGA CCAAAATNAN 170

SEQ ID NO:7024
SEQUENCE LENGTH:84
SEQUENCE TYPE:nucleic acid
TOPOLOGY:linear
CLONE:HUMGS08260
SEQUENCE DESCRIPTION:
GATCAAATGT AAATNTTTTG AGGATATTTT NCTTGTTTTT NAAAATCAAT TTTTAACCAA 60

ATATTAAACC CTTTATTTGC CAAA 84

SEQ ID NO:7025
SEQUENCE LENGTH:246
SEQUENCE TYPE:nucleic acid
TOPOLOGY:linear
CLONE:HUMGS08261
SEQUENCE DESCRIPTION:
GATCCACCAC TGGNCTCTCC CTCCCCCAGC CTGGAGCACG GGAGGGGAGG TGACGGCTGG 60
TGACTGATGG ATGGGTAGTG GGCTGAGAAG AGGGGACTAG GAAGGGCTAT TCCAGGCTCA 120
GCCCTGCTAC TGCAGCTTTG CCGCTGAGTG TAGGAAAAAC AGGCATGACA GACCAGGGTG 180
AGGGTTGTGC CCAGCTGGGC CACGGCCATG CGTGGGGTGG CCCAATAAAC ACCGTGGACT 240
CCCAAA 246

SEQ ID NO:7026
SEQUENCE LENGTH:45
SEQUENCE TYPE:nucleic acid
TOPOLOGY:linear
CLONE:HUMGS08262
SEQUENCE DESCRIPTION:
GATCAGTCTT TATNAATAAA ACCTGTTCTC TTTAATCAGC TTAAA 45

SEQ ID NO:7027
SEQUENCE LENGTH:186
SEQUENCE TYPE:nucleic acid
TOPOLOGY:linear
CLONE:HUMGS08263
SEQUENCE DESCRIPTION:
GATCAGACTG NTAACCTTAA CACCATAGGT GCAAAANGAG TTGTTCTNAG GTTTTTTTGA 60
AACATTAAAG TTCCAAAACA TGACATTTTT AAGAATAAAT TTGAAATAGN GTATGATTGA 120
ATGCAGAGNA TTATGTACCT CTAATTGCTT AATTTTGTAG NGGTCTTTNA TTGTAGAATT 180
GGNNCN 186

SEQ ID NO:7028
SEQUENCE LENGTH:271
SEQUENCE TYPE:nucleic acid
TOPOLOGY:linear
CLONE:HUMGS08264
SEQUENCE DESCRIPTION:
GATCTCTTGG TAACAATTGA CATTGCACTA GNATTTGAAT GNTACTGTTG TTGGCATTTC 60
TGTTANTCTT TCAAGTAGTT GTAAATAATC TCTTTTTCTC ATATAAACGG AATGCATAAT 120
GTCTTACTCT TGAGTCATTT AGTAGCATTG ATATGGTACC AAGTTGTATA GCAGTGTAAC 180
AAAATCATGC TTTGACAATG NAATTTACAT ATT.GCCTCAC CTAGAGATGT TTTGTCTGTA 240
TGACTNNTNN CNTTNTAAAG TTNGNCNAAC N 271

SEQ ID NO:7029
SEQUENCE LENGTH:301
SEQUENCE TYPE:nucleic acid
TOPOLOGY:linear
CLONE:HUMGS08265
SEQUENCE DESCRIPTION:
GATCAAATCA GGGTAATTGG GTATCCATCA CCTCAAACAT TTACCATTTC TTTGTGTTGG 60
GAACATTCCA TATCTTTTTT TCTAGCTGTT CTGAAATTTG CTATACGTTC TTTTAACTAA 120
AGGTCCCTTG CTGTGTTATG GAACACTGGA ATTTGTTCCT TCTGTTTAAC TGTATTTTTG 180
TACCTATTAA CCAACTTCTC TTTATCCTCC CCCGTCCTCT ACCCTTCCCC AGCCTGTGGG 240
AATCACCCAT NCTACTCTCT GCCTTTATGA GATTTACTTN TTTNATCTCC CNATATAANA 300
N 301

SEQ ID NO:7030
SEQUENCE LENGTH:117
SEQUENCE TYPE:nucleic acid
TOPOLOGY:linear
CLONE:HUMGS08266
SEQUENCE DESCRIPTION:
GATCGTAGAA ACACAGAATG GGACTGGGGA AGCCCTTTGG AAATCCAGCT GCAGAAACAG 60
ACACCCCAAT GCTATTTACA TACAGCTCTA TATATATAAA AAAAGNAAAT ATGNAAA 117

SEQ ID NO:7031
SEQUENCE LENGTH:297
SEQUENCE TYPE:nucleic acid
TOPOLOGY:linear
CLONE:HUMGS08267
SEQUENCE DESCRIPTION:
GATCTCACCA GGGCCCAGTC ATATATGCAC AGTTAGACCA CTCCGGCGGA CATCACAGTG 60
ACAAGATTAA CAAGTCAGAG TCTGTGGTGT ATGCGGATAT CCGAAAGANT TAAGNGAATA 120
CCTAGAACAT ATCCTCAGCA AGAAACAAAA CCAAACTGGA CTCTCGTGCA GAAAATGTAG 180
CCCNTTACCA CATGTAGCCT TGGNGACCCA GGCAAGGACA AGTACACGTG TACTCACAGA 240
GGGAGNGNAA GNTGTGTACA AAGGNTATGT ATAANTNTTC TNTTTAGTCA TCCTATN 297

SEQ ID NO:7032
SEQUENCE LENGTH:58
SEQUENCE TYPE:nucleic acid
TOPOLOGY:linear
CLONE:HUMGS08268
SEQUENCE DESCRIPTION:
GATCCTGGGG ACTCATATTC TTTCAGAATC ATGTAAATAA ATGGCATCAT GTTGTAAA 58

SEQ ID NO:7033
SEQUENCE LENGTH:103
SEQUENCE TYPE:nucleic acid

TOPOLOGY:linear
CLONE:HUMGS08269
SEQUENCE DESCRIPTION:
GATCTATTTG GCCTCTCAAA TNAACTNAGA TTCCTGTTAA AAAAGATTGA TGTTATTGTC 60
TCTTGTAGAG GAAACTAATA AAGTGTGTGT ACCTNTGTGN AAA 103

SEQ ID NO:7034
SEQUENCE LENGTH:265
SEQUENCE TYPE:nucleic acid
TOPOLOGY:linear
CLONE:HUMGS08270
SEQUENCE DESCRIPTION:
GATCAAGAGT NATGGGCCTT GGAGCCCTTG CCAGCCCAGC CCACCTGTNA GGAGGCTGCC 60
CATCCCCTCT TTNAGGCCAC CCTGTGTCCT CTCCCCTGCC TCTGCCCAGA GCTCCAGCCG 120
GAGTGTCTTG CTGCTCAGAC CCCTCCGAGG TCCAAGTCCT GTCCTGCCAC TCAGCTCCCN 180
CTGCACCCCA GAAGAAACGC AGGGTGCGGT TGCATTTGAT TTCAGATAAA CAACAACTTC 240
TTAGTAAAAT NACCTNCCCA CTAAA 265

SEQ ID NO:7035
SEQUENCE LENGTH:46
SEQUENCE TYPE:nucleic acid
TOPOLOGY:linear
CLONE:HUMGS08271
SEQUENCE DESCRIPTION:
GATCAATCTT GANGTGAAAT ATTTCCAAAA TAAAATTCTA CAGAAA 46

SEQ ID NO:7036
SEQUENCE LENGTH:254
SEQUENCE TYPE:nucleic acid
TOPOLOGY:linear
CLONE:HUMGS08272
SEQUENCE DESCRIPTION:
GATCTTTGCA TAGAATGTCA AGCTAACCAG GCGTCCGNTA CTTCAGAAGA GTGTACTGTC 60
GCATGGGGAG TCTGTAACCA TGCTTTTNAC TTCCACTGCA TCTCTCGCTG GCTCAAAACA 120
CGACAGGTGT GTCCATTGGA CANCAGAGAG TGGGAATTCC AAAAGTATGG GCACTAGGAA 180
AAGNCTTCTT CCATCAGGNG TTAATTGTTT TGTTATTCAT TTAATGGCTT TCCCTGCTGT 240
TACCTAATTA CATN 254

SEQ ID NO:7037
SEQUENCE LENGTH:222
SEQUENCE TYPE:nucleic acid
TOPOLOGY:linear
CLONE:HUMGS08273
SEQUENCE DESCRIPTION:
GATCATTGAG AAAGTGTTTG AAACTTTCTC ATGAAGTGTA TATATAATGG CGTGAAAAAT 60

TCCTTTGGAA AAATNTATGT NCCTTTCATT TTGCCCAAAT TGCAAATTTT CAGCATGGNT 120
GTGAAAAGCA TTAAAATTAT AACTTTGTGT ACANGATGAA AATAATTCAC TAAATTTGCC 180
CTTTTTTACA CAAAATAAAA TGTTAANGTT ANNGCTGGNA AA 222

SEQ ID NO:7038
SEQUENCE LENGTH:32
SEQUENCE TYPE:nucleic acid
TOPOLOGY:linear
CLONE:HUMGS08274
SEQUENCE DESCRIPTION:
GATCAAATGA CANTAAATCA TTCAAAATCA AA 32

SEQ ID NO:7039
SEQUENCE LENGTH:109
SEQUENCE TYPE:nucleic acid
TOPOLOGY:linear
CLONE:HUMGS08275
SEQUENCE DESCRIPTION:
GATCACCCAG GTACANCATG CTCTATATCC ATGCGGCCCT AGAAACCCAA GGAACATATA 60
CAACCACTCT TGTGTCCCTC CTTATAACCN CAGNTTTATN CNAGAAGTN 109

SEQ ID NO:7040
SEQUENCE LENGTH:301
SEQUENCE TYPE:nucleic acid
TOPOLOGY:linear
CLONE:HUMGS08276
SEQUENCE DESCRIPTION:
GATCACAAAT TGGTGCATTC TTAACTGTGG ATAATTATGT ATGAAGCCAA ATTGTAATAA 60
AACTGTTATT TGCCAAACAT AGAANCCTTG CTTCGTGACT AGGCTGAGCC CAGAGTGTTA 120
TTCTCTTCTT CTGCCTGTNA TGTATCTGGG CAATTTGTAG GGCCTTTGCA GGTGAATCCA 180
GTAGAGAACC ATGCTAAGAN TGNGCAGCAG TCATGCATGC CTGTATCATT GAAAGCCTTC 240
CTNGCCATTT TNNCAGCTTC ACCTTNCGTG GTTTTTATTT TTTCCAGAGG GGGCCTGGGG 300
N 301

SEQ ID NO:7041
SEQUENCE LENGTH:151
SEQUENCE TYPE:nucleic acid
TOPOLOGY:linear
CLONE:HUMGS08277
SEQUENCE DESCRIPTION:
GATCCTCCTC CCTAAGCGCT TATCTTGGTG CTTCCAGCTC AGTCTGGCCA ATTCCACCTC 60
TATGAGATAA GGAGGGTTCA GAGAACCCAG TCACTTTGCT GTTTTTCTGC NCACAGAGTT 120
TCTCTAATAA ATCTTATTCT ATGTCTACAA A 151

SEQ ID NO:7042

SEQUENCE LENGTH:138
SEQUENCE TYPE:nucleic acid
TOPOLOGY:linear
CLONE:HUMGS08278
SEQUENCE DESCRIPTION:
GATCACATTG TTTGACCCAG TATGTCTTGT AGACACGTTA GTTATAATCA CCTTGTATCT 60
CTAAATATGG TGTGATATGA ACCAGTCCAT TCACATTGGA AAAACTGATG GTTTTAAATA 120
ANCTAATTCA CTAATAAA 138

SEQ ID NO:7043
SEQUENCE LENGTH:300
SEQUENCE TYPE:nucleic acid
TOPOLOGY:linear
CLONE:HUMGS08279
SEQUENCE DESCRIPTION:
GATCTGTCAA CAGGTTAAGT CAATCTGGGG CTTCCACTGC CTGCATTCCA GTCCCCAGAG 60
CTTGGTGGTC CCGAAACGGG AAGTNCATAT TGGGGCATGG TGGCCTCCGT GAGCAAATNG 120
TGTCTTGGGC AATCTNAGGC CAGGNCAGAT GTTGCCCCAC CCACTGGAGA TGGTGCTGAG 180
GGAGGTGGGT GGGGCCTTCT GGGAAGGTGA GTGGAGAGGG GCACCTGCCC CCCGCCCTCC 240
CCATCCCCTA CTCCCACTGN TCAGCGCGGG NCATTANAAG GGTGCCANAN ATTTTTTTNN 300

SEQ ID NO:7044
SEQUENCE LENGTH:90
SEQUENCE TYPE:nucleic acid
TOPOLOGY:linear
CLONE:HUMGS08280
SEQUENCE DESCRIPTION:
GATCTCATTC CATGGGAAAA AAAAAAATCC TGTCTTGTTC ATAAATTGAC AATGTCACTA 60
AATAGAAATA AAGTGCACAN TAAATGNAAA 90

SEQ ID NO:7045
SEQUENCE LENGTH:175
SEQUENCE TYPE:nucleic acid
TOPOLOGY:linear
CLONE:HUMGS08281
SEQUENCE DESCRIPTION:
GATCTNAGTT AGACATCACC ANTGGAAATC GATTGGAATA GAAACTTAAA GGAAATGGAA 60
CCCTGACTAT TCTCCCATCA AATCATATAT GTTGACCTGT CTGAATTATA AACCAGCCTG 120
ACCTTTCCTT TAGCATTAGA TGTAATAAAA TAACTTTGGA AATTTGTNAT TTAAA 175

SEQ ID NO:7046
SEQUENCE LENGTH:190
SEQUENCE TYPE:nucleic acid
TOPOLOGY:linear
CLONE:HUMGS08282

SEQUENCE DESCRIPTION:
GATCCTCAGG ACTGACCAGT TAGCTGGCAG GTTGTNCAGC TTTTAATTCC AGTCATAATA 60
GGTGACAGTG TTAACCGTGA AANTTTGNGA GGCACTNTTG NCCTCTTCCC TATAAAAATC 120
ACACAAGCGT GNTTTTACAA AGGGTCCCCG TGGGAACCTT NGCTTAAGGA CCCTTTGNCC 180
CNTCAGTTAN 190

SEQ ID NO:7047
SEQUENCE LENGTH:147
SEQUENCE TYPE:nucleic acid
TOPOLOGY:linear
CLONE:HUMGS08283
SEQUENCE DESCRIPTION:
GATCCTCAGG ACTGACCANT TAAGCTGGCA GGTTGTCCCA GCTTTTTAAT CCCAGTCATA 60
ATAAGGTGAC CAGTTTTAAC CCGTGNAAAC TTNGAGGGGA CTTTTGCCTT TTCCCTATNA 120
AATNACACNG GGGGGTTTTA NAAGGTN 147

SEQ ID NO:7048
SEQUENCE LENGTH:223
SEQUENCE TYPE:nucleic acid
TOPOLOGY:linear
CLONE:HUMGS08284
SEQUENCE DESCRIPTION:
GATCCATCTG CCCTCATAAA AGTGTTCAGG TACAGCAGCT GAGGCTGCCC TGAGNAATCA 60
AGGGGCCATT ACCAAGGGGC AGGAAAAGNA TATGTAAGAG GTGGCCTTCA TGGTAGAGCT 120
TGACCCAAGA ACTACTCCAN ATTCGGATGG CCCAGACTGG GNGCATCCCC TGACTTTCCC 180
TTTGACTTCA CCCTGTTTGT AAATAAANCA AGGNAGATGC AAA 223

SEQ ID NO:7049
SEQUENCE LENGTH:56
SEQUENCE TYPE:nucleic acid
TOPOLOGY:linear
CLONE:HUMGS08285
SEQUENCE DESCRIPTION:
GATCTCCTCC CTNGTTTAAA TGTCAATAAA TGCCCCAACT GCTTTGTAAG TGCAAA 56

SEQ ID NO:7050
SEQUENCE LENGTH:107
SEQUENCE TYPE:nucleic acid
TOPOLOGY:linear
CLONE:HUMGS08286
SEQUENCE DESCRIPTION:
GATCACCTTA AAATTATTTN ACGNACTGNA GNCAATNAAG TCCGTTATGT TTAGAGTAGA 60
AAATGTTTAG GTTAAAGAGC ATCTGTCAAC AGAATCTACA AAAAAGN 107

SEQ ID NO:7051

SEQUENCE LENGTH:125
SEQUENCE TYPE:nucleic acid
TOPOLOGY:linear
CLONE:HUMGS08287
SEQUENCE DESCRIPTION:
GATCCAATTN CATNNGCGGC TGCATCTNCT GCTCATGCCG CCCAAACACC ACCCAGATGT 60
CACTTACGTC AAGAAGGTCC GGACCCTCCG TATGCACCTG TTCACGGCCC TGCAGCTGCT 120
NTGNN 125

SEQ ID NO:7052
SEQUENCE LENGTH:276
SEQUENCE TYPE:nucleic acid
TOPOLOGY:linear
CLONE:HUMGS08288
SEQUENCE DESCRIPTION:
GATCCAAGAA ACGNANGACA CAACAACAAC GATGCCGTGT GTCTGCTCTA CCTGGGCAAG 60
CTCAAGNACT CCCTGCGGCA GCTNGAGGCC ATGGTCCAGC AGGACCCCAG GCACTACCTG 120
CACGAGAGCG TGCTCTTCAA CCTGACCACC ATGTACGAGC TGGAGTCCTC ACGGNGCATG 180
CAGAAGAAAC AGGCCCTGCT GGNGGCTGTC GNCGGCAAGG NGGGGGCCAG CTTTAACACA 240
CAGTGCCTCA AGCTGGCCTA GCTGNNTNNN ACACAN 276

SEQ ID NO:7053
SEQUENCE LENGTH:209
SEQUENCE TYPE:nucleic acid
TOPOLOGY:linear
CLONE:HUMGS08289
SEQUENCE DESCRIPTION:
GATCGAGTTT ACAGTTGTGN NNTCTTGAAG GTATTACTTA ACTTCACTAC AGCTTGTCTA 60
GAAGACCTTT CTAGGNGTTA TCTGGTTCTA GAAGGGTCTA TACTTGTCCT TGTCTTTAAG 120
CTATTTGACA ACTCTACGNG TTGTAGNAAA CTGCTAATAA TACAAATNCT TGTTGTCCAT 180
GGNAAGGCAA ATAAATTTNC TACAGTGNN 209

SEQ ID NO:7054
SEQUENCE LENGTH:126
SEQUENCE TYPE:nucleic acid
TOPOLOGY:linear
CLONE:HUMGS08290
SEQUENCE DESCRIPTION:
GATCTCATTT TCAGTTTAAG TAACTGCTGT TACTTAAGTG ATTACACTTT TGCTCAAATT 60
GAAGCTTAAT GGAATTATNG CCCTCAGGAT AGTATTTTGT AAATAAAGNT GATTTAAATA 120
TGNAAA 126

SEQ ID NO:7055
SEQUENCE LENGTH:276
SEQUENCE TYPE:nucleic acid

TOPOLOGY:linear
CLONE:HUMGS08291
SEQUENCE DESCRIPTION:
GATCGCACCA TTGCACTCCC ACCTGGGCAA CAAAGAGTGA AACTTGGTCT CAGAAACGAA 60
ACAAAACACA AAAACCTTTC TNAGTCCCAG CATATGTGGA GCAGCCTCAT TCTTCATAGC 120
TGTGTGTCAT TCCGTTGCGT GATGGGGTCA CAGAGCACAG ACCTGGTGCC CTTTTCCTTT 180
TTAATATGTG GAAACCCCTC CATGCTTTCC AAAGCCTACA AGTACAGCAG CCCCNAGTTT 240
AGGGTGNGCA GCAGTGGTCA GAGCTCTTTN CTANTN 276

SEQ ID NO:7056
SEQUENCE LENGTH:276
SEQUENCE TYPE:nucleic acid
TOPOLOGY:linear
CLONE:HUMGS08292
SEQUENCE DESCRIPTION:
GATCTCCAGG AGTCCCCTGC ACATACCTTC TCCATCGTGT CAGCTGTGTT TCTCTTGATT 60
CCGTGACACC CGGTTTATTA GTTCAAAAGT GTGACACCTT TTCTGGGCAA GGAACAGCCC 120
CTTTAAGGAG CAAATCACTT CTGNNNNAGT TATTATGGTA ATATGAGGCA ATCTGATTAG 180
CTTCACAGAC TGAGTCTCCA CAACACCAAA ATATCCAGAT GTAAACCCCA AACTTGTACA 240
CAAANGNAAG CACAGATTGT TTACCTGTTG TGGAGN 276

SEQ ID NO:7057
SEQUENCE LENGTH:167
SEQUENCE TYPE:nucleic acid
TOPOLOGY:linear
CLONE:HUMGS08293
SEQUENCE DESCRIPTION:
GATCCCTGTT ACCCAGAGAA TANNTACATT CTTTATCTTG ACATTCAAGG CATTTCTATC 60
ACATATTTGA TAGTTGGTGT TCAAAAAAAC ACTAGTTTTG TGCCAGCCGT GATGCTCAGG 120
CTTGAAATGC ATTATTNNGA ATGTGAAGTA AATACTGTAC CTTTAAA 167

SEQ ID NO:7058
SEQUENCE LENGTH:69
SEQUENCE TYPE:nucleic acid
TOPOLOGY:linear
CLONE:HUMGS08294
SEQUENCE DESCRIPTION:
GATCACATAC CCTTCTTTTA AGTGAATTTT TTTCATTTGA TTTGTCAATA AACGAATCAA 60
ACTGGNAAA 69

SEQ ID NO:7059
SEQUENCE LENGTH:222
SEQUENCE TYPE:nucleic acid
TOPOLOGY:linear
CLONE:HUMGS08295

SEQUENCE DESCRIPTION:
GATCTCCCTG CCTGGGCTGG CTGTGAGGCC ACCTTTGTCC CAGGCCCAGC CTCAAGGCAA 60
GGAGGGCGCT TCACTGAGGT GTGAATTGTA CGTACAGGCT TTTTATATAC CAAAAGTATT 120
TTTTGACTAG ACCATTCAAA GCTACCCGAA CTATGTTGGA AATTTTTTTT TTCTCATTAA 180
AATACAGGCC CTTAGGCTCT ATTTTTAATG TATNAGTCGC AN 222

SEQ ID NO:7060
SEQUENCE LENGTH:145
SEQUENCE TYPE:nucleic acid
TOPOLOGY:linear
CLONE:HUMGS08296
SEQUENCE DESCRIPTION:
GATCAAAATA CCTGTTTATA TATCTGTTAT TTAAAGAAAA ATTTTCCACA GTGTTTTGTT 60
ATTTTCCAGC GTTTCTTAGN ACATTTGGGN ATTNCACTAA AATATAGCTG GGTGGATTGA 120
AGTAAAGGGC AANTGCGTTT GCAAA 145

SEQ ID NO:7061
SEQUENCE LENGTH:20
SEQUENCE TYPE:nucleic acid
TOPOLOGY:linear
CLONE:HUMGS08297
SEQUENCE DESCRIPTION:
GATCTTCCAA AACACACAAA 20

SEQ ID NO:7062
SEQUENCE LENGTH:227
SEQUENCE TYPE:nucleic acid
TOPOLOGY:linear
CLONE:HUMGS08298
SEQUENCE DESCRIPTION:
GATCAATCAA GAATAATTTT AATTTTCTTT TGTATTTGAA ATGTAAATAG TTTTCTTTTC 60
GATTAAAAAA ATTTCCTATA ACTGCTAAAC AGTTAAAANC TTTAAAGTAG TAAATGAGTT 120
TATAGAAAGC ATGTATTCTT GATTTTNGTG CCTTGGTAAA GTTGATAACT ATTTATGAAT 180
ATTTGACCAA ATTATTCCAG CATCAGAATA ATAAGCAAAA TAACCNN 227

SEQ ID NO:7063
SEQUENCE LENGTH:209
SEQUENCE TYPE:nucleic acid
TOPOLOGY:linear
CLONE:HUMGS08299
SEQUENCE DESCRIPTION:
GATCACACCA CTGCATGCCA GCCTGGGCGA CAGAGTGAGA CCCTGTCTCA GAAAAGAAAA 60
AAAAAGTAAA AATTGCATGT AAGTTGACCC GCACTATTCA AATTTGTGGT GTTCAAGGTT 120
CAACTGTAAT TTCCTAGCAG CATTTTGTGT GTTTGAGAAT CTCTTGACAC TCTTCAAGTA 180
AATCCCTAAA TTACAACTTT GACATCAAA 209

SEQ ID NO:7064
SEQUENCE LENGTH:130
SEQUENCE TYPE:nucleic acid
TOPOLOGY:linear
CLONE:HUMGS08300
SEQUENCE DESCRIPTION:
GATCGACTGA GCTCAAATTT CAGTTGGGGC CCTATTAGTC TGCCTTTCCA AAAAGGGCGT 60
TGGGACTTTA ATTTAAAACT TAACCAGTTG AAAAATAAAG GTGGCGTGGA AATGCTTTTT 120
GATTTGCAAA 130

SEQ ID NO:7065
SEQUENCE LENGTH:223
SEQUENCE TYPE:nucleic acid
TOPOLOGY:linear
CLONE:HUMGS08301
SEQUENCE DESCRIPTION:
GATCTGGGCT TTAAAGCAGA GGCCATGTCC TTGTCTGGTC CTGCTTNTGG CTACAGCCAC 60
CCTGGAACGG AGAAGGCAGC TGACGGGGAT TGCCTTCCTC AGCCGCAGCA GCACCTGGGG 120
CTCCAGCTGC TGGAATCCTA CCATCCCAGG AGGCAGGNAC AGCCAGGNNG AGGGGAGGAG 180
TGGGCAGTGA AGATGAAGCC CCATGCTCAG TCCCCTCCNA TNN 223

SEQ ID NO:7066
SEQUENCE LENGTH:260
SEQUENCE TYPE:nucleic acid
TOPOLOGY:linear
CLONE:HUMGS08302
SEQUENCE DESCRIPTION:
GATCGGAGAC ATCCTGCTGT TCGGNACGTT GCTGATGAAT GCCGNGGCGG TGCTGAACTT 60
TAAGCTGAAA AAGAAGGACA CGCAGGGCTT TGGGGAGGAG TCCAGGGAGC CCAGCACAGG 120
TGACAACATC CGGGAATTCT TGCTGAGCCT CAGATACTTT CGAATCTTCA TCGCCCTGTG 180
GNACATCTTC ATGATGTTCT NCATGATTGT NCTGTTCGGC TCTTGAATCC CAGCGATGAA 240
ACCAGGAACT CACTTTCCAN 260

SEQ ID NO:7067
SEQUENCE LENGTH:154
SEQUENCE TYPE:nucleic acid
TOPOLOGY:linear
CLONE:HUMGS08303
SEQUENCE DESCRIPTION:
GATCGAAAGA AGNACATCAT GAAAAATAAA TGAACTTTGC TTAGTGGATT GACTCCTTTG 60
CTGAAGTCAG TTATTCATCA AGAATGCAAT TAGNCTAATT GTGAATAAAT GATTGNNTGA 120
AGNTATAATA AATAAAACCG TAAACTCTGG CAAA 154

SEQ ID NO:7068

SEQUENCE LENGTH:29
SEQUENCE TYPE:nucleic acid
TOPOLOGY:linear
CLONE:HUMGS08304
SEQUENCE DESCRIPTION:
GATCAATAAA ATGTGATTTT NCTGATAAA 29

SEQ ID NO:7069
SEQUENCE LENGTH:74
SEQUENCE TYPE:nucleic acid
TOPOLOGY:linear
CLONE:HUMGS08305
SEQUENCE DESCRIPTION:
GATCGTCCCA TCACAGTGTT GTTAATGTTT GCTGTATTTA TTAATTTNCT TAAAGTGAAA 60
TCTGAAAAAT GAAA 74

SEQ ID NO:7070
SEQUENCE LENGTH:264
SEQUENCE TYPE:nucleic acid
TOPOLOGY:linear
CLONE:HUMGS08306
SEQUENCE DESCRIPTION:
GATCTGGCTT CACAGACAGT TGACACATCT AAAAGAATCT TTGTTTCCAA CTGGACTGAC 60
TCATTGAGAG GAAACCTTCA CTGCACCTCA CCAGTGCAGA CAGAAGACCT GGAGGCAGGA 120
CGGGAGGCAT TTTATACTTC AGCACGATAT GGGCCCTGAC TTTTCATTTT CTACATGGAG 180
ACATATTGCT GGAGGCTCAT CATCCTGAGG GGAGAAACAT GGATTTCATG GTTTTGCCAT 240
AAAAGTATGC AACAAAGAGT TCCN 264

SEQ ID NO:7071
SEQUENCE LENGTH:27
SEQUENCE TYPE:nucleic acid
TOPOLOGY:linear
CLONE:HUMGS08307
SEQUENCE DESCRIPTION:
GATCANTAAA AAAAATAAAT AATTAAA 27

SEQ ID NO:7072
SEQUENCE LENGTH:175
SEQUENCE TYPE:nucleic acid
TOPOLOGY:linear
CLONE:HUMGS08308
SEQUENCE DESCRIPTION:
GATCTNTTTT NTCTGGAGTC CTGGAGTTNC TTTCCTAAGG TTTAATATAA GCAAGCNCCA 60
GAAAGAATGC TGACAGAAAA GGGACCCTAG CTGTGGTAGG AAGTGGCCCT CAGAGTCAAG 120
GAGGCAGGAT GANTTTAAAT NCTNCATGTA GGGCATATTT TGGGGAGTNA TGGGN 175

SEQ ID NO:7073
SEQUENCE LENGTH:281
SEQUENCE TYPE:nucleic acid
TOPOLOGY:linear
CLONE:HUMGS08309
SEQUENCE DESCRIPTION:
GATCGACTTT AAGTTGCTTC GGGTCTAGTA GAAATCCGGT TATTTCCAAG CCTGTNAAAT 60
GTGAAGTTCA AGAAAATTGA ATCCGTGTAT AGCAAAACCA TTTTTNCTCA CAATTTTTTA 120
ACCAATGAAC ATTGTGTGGG AAATTNATTC CATATGTTTT CAAGTCATTT ATGCAACTTG 180
AATAATTAAA ATATTCATTG GTAAGAGCTG CCTGATGTCT TTAAAGTCAG GAAATTGTCT 240
TATGCCAAAA GTAGACAANC TCAAACACCA AAAGGTCANN N 281

SEQ ID NO:7074
SEQUENCE LENGTH:106
SEQUENCE TYPE:nucleic acid
TOPOLOGY:linear
CLONE:HUMGS08310
SEQUENCE DESCRIPTION:
GATCATTTTT CTTGGAGGNT GGAGATTGGC TAGTACCTCT GGCCTAACTG TGTAGGTCAA 60
TACTCTTTTA CATTGCCTTC TAATAAAAGC AGNATGATNC AGCAAA 106

SEQ ID NO:7075
SEQUENCE LENGTH:69
SEQUENCE TYPE:nucleic acid
TOPOLOGY:linear
CLONE:HUMGS08311
SEQUENCE DESCRIPTION:
GATCTTGTGT CTGTTGTGCC TGAATTTGGC GTGTCTAATA AAGGCCCATG CACACTATAG 60
GCACTCAAA 69

SEQ ID NO:7076
SEQUENCE LENGTH:292
SEQUENCE TYPE:nucleic acid
TOPOLOGY:linear
CLONE:HUMGS08312
SEQUENCE DESCRIPTION:
GATCAGTGAA ATNTTAAACT CCCACGGTAA CATCATGCCT ATGGACATTA AAGGAATAGC 60
AAGGACTTGC TCCCACGACT TCACATTTGC TCCCACTGGC CAGGAATGTG GGCTCTTAGT 120
GATTCCAGAT AAGGGCAAAG CTGGGGCCCA ATGTATGTAG CCAAATTGNA GNAATCATGG 180
NGCACAGGCA AATGGTGACA TGAATTTGTA ANCTGTGTTT ATGTGTTNTT ATATTTATAT 240
TTCTGANCTC AGTACATGTT AATATTTAAN TAATTATGCA GTAACTTTCA AA 292

SEQ ID NO:7077
SEQUENCE LENGTH:29

SEQUENCE TYPE:nucleic acid
TOPOLOGY:linear
CLONE:HUMGS08313
SEQUENCE DESCRIPTION:
GATCCTGCAC TCTAGCACAT GACTCCAAA 29

SEQ ID NO:7078
SEQUENCE LENGTH:334
SEQUENCE TYPE:nucleic acid
TOPOLOGY:linear
CLONE:HUMGS08314
SEQUENCE DESCRIPTION:
GATCCCCTGT CCCCTGGGGC TGGTGGATAG GGCAGAATCC TGGGCCCCCA GAGACCTTTG 60
CCCACACACA CTCCTTCCCC TTGTCCCTGG GGNACTCCCC CAGGATTGTG CAATAGTCAG 120
AGTGTCCCTT TTTGCAGGGG ACTGGGCCAT GGGTCCTAGG CCCATCTGTC CATCCTCCTC 180
TCCATGCAAG TGCTGTTTGG GCAGNAGTCA CCATGCAAGG GTGNCATCGN CANCCACGTA 240
CCAAGNCACC GCAGCTGCTG CCACTCTGCT GCCTGTACAG AAGGAANCTG NATCTTTTTN 300
ATATTCTAAT AAAATNACTG TGGGNTTTNA NAAA 334

SEQ ID NO:7079
SEQUENCE LENGTH:245
SEQUENCE TYPE:nucleic acid
TOPOLOGY:linear
CLONE:HUMGS08315
SEQUENCE DESCRIPTION:
GATCCCGTAA ATTGAAGGCA ACAAGGTCTT GGTCCTNCAG TAAGGGGGGC ATGGAGCCCT 60
GGTGGGAAGG AAAGAAGNGA AATTTGTGTT CCTGATTCCT TAATCCCCAN GAAAGGTCCC 120
TCTGCTAACA TTCACATATC TNTTCCTAGC CAAAGTCTGT CCATTTNAAC CCTNTAGCAT 180
CTGAGCTATC TGCATCCAGN TTTCAAANAT ATTTCGNTGT TTACTAACAT GTNTGTNAGC 240
CTNTN 245

SEQ ID NO:7080
SEQUENCE LENGTH:88
SEQUENCE TYPE:nucleic acid
TOPOLOGY:linear
CLONE:HUMGS08316
SEQUENCE DESCRIPTION:
GATCACAAAT NACTTGTTGA AATGTAAGGC AGTCCCCCTC CTCCTCTTTA TCTACATTAC 60
TTCCCGAAAA TAAATGCANA TTAATAAA 88

SEQ ID NO:7081
SEQUENCE LENGTH:149
SEQUENCE TYPE:nucleic acid
TOPOLOGY:linear
CLONE:HUMGS08317

SEQUENCE DESCRIPTION:
GATCAGGTCA CAGTGTGTAC GTAAGAGTCA GCTNCTTATG GNTATTGTCA TCTAGGTAAC 60
TTTGTAAAAA AACAAACAAA ACTGGCATTG TTAATTCCAA AAAGAAAAAA AAAAGCAGTA 120
AAAANAAANG GNCATTTGNA CCNCCGGCN 149

SEQ ID NO:7082
SEQUENCE LENGTH:312
SEQUENCE TYPE:nucleic acid
TOPOLOGY:linear
CLONE:HUMGS08318
SEQUENCE DESCRIPTION:
GATCTCAGCC TCGGGTGGTC AGGGTGGGAA ATGATAGGAA GAAGCTGTCA TCTGCATCCT 60
AGTTTGCCTG AAATGAACCC AAATAATACC CATTATTATT AGTCCTGAAT TATGAGTAGT 120
GAATGATACC CATCATTCTG GCATCATGAT GAGTAGTGTC CACTTCCATT CTGAAAAGTG 180
CCCTGCTGTG AAAAATAAAT TATATAGTCC TCCTAGGTAA ATGAAGGAGG AGGGAGAAGT 240
GTGAAAGAGT ATGGCTTAAA TCAGACAAGA TATACAAGAA GATACTTTAT ATAGGGCAGG 300
AGCGGTGGCT TN 312

SEQ ID NO:7083
SEQUENCE LENGTH:87
SEQUENCE TYPE:nucleic acid
TOPOLOGY:linear
CLONE:HUMGS08319
SEQUENCE DESCRIPTION:
GATCTGGGAA ACTGATTGAA TAATACACTT TTCTTGCTTT GGTGCTCAAA GTGGTTTTTT 60
TCCCCCAATA AAATTATTTA ATTGAAA 87

SEQ ID NO:7084
SEQUENCE LENGTH:312
SEQUENCE TYPE:nucleic acid
TOPOLOGY:linear
CLONE:HUMGS08320
SEQUENCE DESCRIPTION:
GATCAGGAAA TGCATCTNCG CAGATGGTAT TTCCTTCAGA AAAGACTTTN CTACTTTAAA 60
TATAAATNAN GCCATAACAG TTTCATGCTG TGGAAAGAGG GTGAAAAGGT TCATTTTAAG 120
AGATTATATA ATANGACCTT NCACATTTCC TGTGAAATGT CTAACTTTNC CAGTNCTTCA 180
GCAAGTTTTT TTGGGGGGTG ATGGGGAGNN GGTAGTATTG GTTTTAGAGG TTTCAAATCT 240
GTGANCTTTG GAGAGGGGAC AGTTGTTGGC TCTGGTATTT CCTAGTTTTG TAGTAACGTT 300
TTGCTAGCCN NN 312

SEQ ID NO:7085
SEQUENCE LENGTH:168
SEQUENCE TYPE:nucleic acid
TOPOLOGY:linear
CLONE:HUMGS08321

SEQUENCE DESCRIPTION:
GATCTGATGG TTTTAAAAAG GGGTGTCTCC CTGCACAAAC TCATTCTCTG TTTGCCTACC 60
GCCATCCGCT TAAGATGTGA CCTGCTCCTC CTTGCCTTCC GCCGCGATTG TNAGGCCTCC 120
TCAGCTATGT GGAACTGTAA ATCGAATTAA ACCTCTTTCT TTTGTAAA 168

SEQ ID NO:7086
SEQUENCE LENGTH:146
SEQUENCE TYPE:nucleic acid
TOPOLOGY:linear
CLONE:HUMGS08322
SEQUENCE DESCRIPTION:
GATCCATGTG TTTTACTCTA AAGTGCTAAA TATGGGAGTT TCCTTTTTTT ACTCTTTGTC 60
ACTGATGACA CAACAGAAAA GAAACTGTAG ACCTTGGGAC AATCAACATT TAAATAAACT 120
TTATAATTAT TTTTTCAAAC TTTAAA 146

SEQ ID NO:7087
SEQUENCE LENGTH:314
SEQUENCE TYPE:nucleic acid
TOPOLOGY:linear
CLONE:HUMGS08323
SEQUENCE DESCRIPTION:
GATCAATAAA GCTGTCCTAC ACTTGTGCTA GGNAGGCACC GCTGTGACTG GGCTCCGGGC 60
CTTTCCACTA CGTTGAAGAA GAAAACCTAT TTTTAAATGT AAATAAAATA TCTGGTAGCC 120
TGTGTGGAAA GCTGACCGTT TTAAGAAGTG GCATGTGCCT TGAAAGGGGG CAGAATGTTC 180
AGTCGGTCGT GTTTTTAACA CAGAGTCTCT AGAAGAGGTG CAGACATCCC GTCTGACTGT 240
CCCTGTGGAC TCTCTCAGTT GTATGTTGCT ATAATCCTCC AAATCAAAGC TCTTTCTGCT 300
TGTGCAAGAT TNNN 314

SEQ ID NO:7088
SEQUENCE LENGTH:71
SEQUENCE TYPE:nucleic acid
TOPOLOGY:linear
CLONE:HUMGS08324
SEQUENCE DESCRIPTION:
GATCTTACTG AATTTATTTT GTAAATGCCT AATGAGGCAG ATTTTTGAAT TAAAGAAATG 60
CTACATGTAA A 71

SEQ ID NO:7089
SEQUENCE LENGTH:412
SEQUENCE TYPE:nucleic acid
TOPOLOGY:linear
CLONE:HUMGS08325
SEQUENCE DESCRIPTION:
GATCCTAACA TATCAGGACA TGGACTCAGG ACCTGCCCGG TGATGCTGTT GATTTCTCAA 60
AGGTCTTCCA AAACTCAACA GAGCCAGAAG TAGCCGCCCG CTCAGCGGCT CAGGTGCCAG 120

CTCTGTTCTG ATTCACCAGG GGTCCGTCAG TAGTCATTGC CACCCGCGGG GCACCTCCCT 180
GGCCACACGC CTGTTCCCAG CAAGTGCTGA AACTCACTAG ACCGTCTGCC TGTTTCGAAA 240
TGGGGAAAGC GTGCGTGCGC GTTATTTATT TAAGTGCGCC TGTGTGCGCG GGTGTGGGAG 300
CACACTTTGC AAAGCCACAG CGTTTCTGGT TTTGGGTGTA CAGTCTTTGT GTGCCTGGCG 360
AGAAGAATAT TTTCTATTTN TTTTAAGTCA TTTCATGGTT TCCTGGTCCT GN 412

SEQ ID NO:7090
SEQUENCE LENGTH:91
SEQUENCE TYPE:nucleic acid
TOPOLOGY:linear
CLONE:HUMGS08326
SEQUENCE DESCRIPTION:
GATCTATTTT TGGCTTATGT CTTTTAATAC TACTCTCTTT CTCTGAATTT TGAAATATGA 60
AGTAAAATCT AGCCCATTTG TTTTATGCAA A 91

SEQ ID NO:7091
SEQUENCE LENGTH:294
SEQUENCE TYPE:nucleic acid
TOPOLOGY:linear
CLONE:HUMGS08327
SEQUENCE DESCRIPTION:
GATCTCAAGA GTTCAAGACC AGCCTGGGCA ATATGGTGAA ACCCGTTTGT ATTAAAAATA 60
CAAAAATTAG CTGTGCATGG TGGCCCACGG CTGTAATCCC AGCTACTCAG GAAGCTGAGG 120
TCCGAGAATC ACTTGAATGC TTGAACTCGG GAGGCAGAAG TTGCTGTGAG CCGAGATGGC 180
ATCACTGCGC TCCAGCCTGG GCAACAGAGA GAGACTGTCT CCACTGTCTC CAAAAAAAAA 240
NGAAAAAAAA AAGGTTGTTN GATTCNTGGC CCTCAAGGAA ATTNTGGAAA ANAN 294

SEQ ID NO:7092
SEQUENCE LENGTH:79
SEQUENCE TYPE:nucleic acid
TOPOLOGY:linear
CLONE:HUMGS08328
SEQUENCE DESCRIPTION:
GATCATGTTT TGAAGCAGCA GGTCCAGGTC ACTTTGTATA TAGAATTTTG CTGTATTCAA 60
TAAATCTGTT TGGAGGAAA 79

SEQ ID NO:7093
SEQUENCE LENGTH:122
SEQUENCE TYPE:nucleic acid
TOPOLOGY:linear
CLONE:HUMGS08329
SEQUENCE DESCRIPTION:
GATCTTTCCC TAGAGAATGT AACATACGTT TTTATTCATT TAATCACTTC ATTATGCCGG 60
GGTTAATNAT GTTTATTTTA TAATTGGTAA TAAAGGCCAC ATTTATTTTT GTAACTGTTA 120
AA 122

SEQ ID NO:7094
SEQUENCE LENGTH:50
SEQUENCE TYPE:nucleic acid
TOPOLOGY:linear
CLONE:HUMGS08330
SEQUENCE DESCRIPTION:
GATCTTAAAC TCTTTGATTA AAAATAATAA ATGGAAATGT GTTAAGCAAA 50

SEQ ID NO:7095
SEQUENCE LENGTH:77
SEQUENCE TYPE:nucleic acid
TOPOLOGY:linear
CLONE:HUMGS08331
SEQUENCE DESCRIPTION:
GATCCAGGGA TTTGGGGAAA AGGAAGGAAT TTGATGTTTT TTGGGGTGGG AGGGGAGGGT 60
GTGTTTTTTA CACCAAA 77

SEQ ID NO:7096
SEQUENCE LENGTH:186
SEQUENCE TYPE:nucleic acid
TOPOLOGY:linear
CLONE:HUMGS08332
SEQUENCE DESCRIPTION:
GATCGCTGGC CTTCCTGACT TAACATTNAT GCATGCCAAG GTGTTGATGT TCCCAGCCAC 60
GTTAACACCT TCCACAAGNT CTCAAGAAAA AGCAGACGGA TAACTGATGT GAATTGGACA 120
GTTTCTATTG CTTTTCCTTT TTTCCATCCC TTCCCTACCA TCAAAAGCAT ACCTGCTCTA 180
ATTAAA 186

SEQ ID NO:7097
SEQUENCE LENGTH:291
SEQUENCE TYPE:nucleic acid
TOPOLOGY:linear
CLONE:HUMGS08333
SEQUENCE DESCRIPTION:
GATCATATAC TCTGCTGTGG TCATGCCCAC TCTTTGGGAG TATATTCCCT TTATATATAT 60
TGAGTATTGT ACCACTTGAG AAATTCCTTT GTTCTGTTAT ACAAAATTAA TCTTTCTGCT 120
CATAATGATT GATGATACCA CCAGTAAAAA TAGGATGTTT ACCCCAAAAC AAGTGTCAAT 180
TAAGAATTTG AACACAACCA CATTTTTTAA AATGAAACTT CTATCGGAAG TAAATTAATT 240
TGNGGTAATA AAGTCCAGTA TTNGGNNAAA TGTACAATGT TAAATCTCAA A 291

SEQ ID NO:7098
SEQUENCE LENGTH:307
SEQUENCE TYPE:nucleic acid
TOPOLOGY:linear

CLONE:HUMGS08334
SEQUENCE DESCRIPTION:
GATCAGACTT TTGTNTCTTG GGTCCCAGAT GGCACAGGAG CACTGCATGC TTGTTTTCTA 60
GAGCCCAGCC AGTCATGGGT GCTAGCCTAG TCTCCACACA CCAGCAAGTA GAACCCAAGT 120
GTANNNNNAT AAATATTTCC TGAGTACCAG TAAGAGAATG CATTCTTTTC TCATCTAGGC 180
CAGGAATGTT GAAAATGCTC AGCCTTACAT AGAAACTCCT AGATTTTCAC TAACGCATTT 240
CACAAAAGTA AATAAGTATT TCATATAATT CAGAGGATGT TTAAATTGTC AGCATTTTAA 300
TAAATAN 307

SEQ ID NO:7099
SEQUENCE LENGTH:199
SEQUENCE TYPE:nucleic acid
TOPOLOGY:linear
CLONE:HUMGS08335
SEQUENCE DESCRIPTION:
GATCTGTACT TTGAACAAAG AAAGTGTGTA GAGACCCCTG GGAGTGAGGG GTAGGGGATA 60
CAATACTCTG AAGCAGAAGA GGACCCTTGC AGTGATGGTT AGGGACTCTT CTGAGTAGGA 120
GACGTAATCG GCTTTTGGTC CATGGAACTT TTACCACATG TATTCAGCAT ATATACACGA 180
TTAAAGTGCT GCAAATAAA 199

SEQ ID NO:7100
SEQUENCE LENGTH:297
SEQUENCE TYPE:nucleic acid
TOPOLOGY:linear
CLONE:HUMGS08336
SEQUENCE DESCRIPTION:
GATCTGGGAG TGGCTTCCTA ACGGAAATGG AACAGGATTA CCAACTAGTN GACGAAAGTA 60
ATGCTTTCCA TGACAACCTT AGGTCTCTTG ACAGNAATCT GCCCTCAGAC AGCCAGGACT 120
TGGGTCAACA TGGATTAGAA GAGGATTTNA TGTTATAAAA GAGGATTTTC CCACCTTGAC 180
ACCAGGCAAT GTAGTTAGCA TATTTANNGT ACCATGGTTA TATGATTAAT CTTGGGACAA 240
AGAATTTNAT AGAAATTTTT AAACATCTGA AAAAGAAGCT TAAGTTTTAT CATCCTN 297

SEQ ID NO:7101
SEQUENCE LENGTH:296
SEQUENCE TYPE:nucleic acid
TOPOLOGY:linear
CLONE:HUMGS08337
SEQUENCE DESCRIPTION:
GATCTTTGTC TGTGAAGAAG AAAATTATCT CCCTAGTTCA ATCTGTAGTG AAATAAGGCT 60
ACAGAAGGCA TTGTTTTTTC CTTTTTNATT TTTTGTATTA TATATTTNCC TTAAATATGT 120
TTTATTGTCT TCTCTANGCA AAAAGTTCTT AATAANCATA GTATTTCTCT CTGCGTCCTA 180
TTTCATTAGT GAAGACATAG TTCACCTAAA ATGGCATCCT GCTCTGAATC TAGNCTTTTT 240
AGAAATGGCA TATGGTTTTG ATGATATGNC CACCATCCAA AATGTCCGAN TNAATN 296

SEQ ID NO:7102

SEQUENCE LENGTH:305
SEQUENCE TYPE:nucleic acid
TOPOLOGY:linear
CLONE:HUMGS08338
SEQUENCE DESCRIPTION:
GATCAACTTT AAGAAGATTT ACAAAACGCT CAAGAAAGAN GAGGATATCC NACTNTTCCC 60
CGTGCAGACA AAATACATGG ATGTGGTCAA NGAGTGAATC CGTTTAGCTC GACAGATTGA 120
GAAATCTGAG TATCGGAACT TCCAGGCTTG CCTNCACAAC TCTTGGATTG AGCAGGCAGC 180
AGCTGCCCTG GAGATTGAGC TGGAAGANGA CATGTATAAG GNAGGAAAAG CTNNCCAGCA 240
AGAAGAACGT CGGNGACAAA AGCAGATGGN GGTTCTNGAA GAAGGAGCTG CGNCNCCTGC 300
TTNTN 305

SEQ ID NO:7103
SEQUENCE LENGTH:414
SEQUENCE TYPE:nucleic acid
TOPOLOGY:linear
CLONE:HUMGS08339
SEQUENCE DESCRIPTION:
GATCCACAAG TCCTTGTTCC ACTGTGCCTT GGTTTCTCCT TTATTTCTAA GTGGAAAAAG 60
TATTAGCCAC CATCTNACCT CACAGTGATG TTGTGAGGAC ATGTGGAAGC ACTTTAAGTT 120
TTTTCATCAT AACATAAATN ATTTTCAAGT GTAACTTATT AACCTATTTA TTATTTATGT 180
ATTTATTTAA GCATCAAATA TTTGTGCAAG AATTTGGAAA AATAGANGAT GAATCATTGA 240
TTGANTAGTT ATAAAGATGT TATAGTAAAT TNATTTTATT TTAGATATTA AATGATGTTT 300
TATTAGGATA AATTTCAATC AGGGTTTTTN GGATTAANCA ACCANCCAAT TGGGTCCCCA 360
GTTAANTTTC CATTTCAGAT ANCCACCANN TAGTTTCNNN GTNTAGGTCC ATTN 414

SEQ ID NO:7104
SEQUENCE LENGTH:252
SEQUENCE TYPE:nucleic acid
TOPOLOGY:linear
CLONE:HUMGS08340
SEQUENCE DESCRIPTION:
GATCCCCTGA CTGGCTGGGG GCAGCTCCCA GGATATCCTG CCTTCCAACT GTTTCTGAAG 60
CGCCTCCTCC TAACATGGCG ATTCCGGAGG TCAAGNNCNT GGGCTCTCCC CAGGGTCTAA 120
CGGTTAAGGT GACCACATAC CAGTGCCAAG GGGNTTNTAA AGTGGTGATG TAGTTNTGCT 180
CCCCTCCCCA AGAGCGGGTG GNGGGGGGTT NAANATGTTT GGCCTGNNTA AGTGGGCCTT 240
CCCATTNAAG TN 252

SEQ ID NO:7105
SEQUENCE LENGTH:318
SEQUENCE TYPE:nucleic acid
TOPOLOGY:linear
CLONE:HUMGS08341
SEQUENCE DESCRIPTION:
GATCTTATTT TTTAATTTTA AGTGCCACTA TTAATGTAAA AAGGGGGGGG CTCTACAGCA 60

GTCGTGATGA AACTTAAATA TATATCCTTT GTCCTCGAGA TTTTAGGAAG GGTGTAGGGT 120
GAGTAGGCCA TTTTNAATTN CTGAAGTGCT AAGTGTTTTT ATACAGCAAA CAAAAAGTCA 180
ATTTTCCTTT CCACCAGTGC GAGAGAGGAT GTATACTTTN CAAGAGAGAT GATTGCCTAT 240
TTNCCGTTTG ACAGAGTCCC GTAGATGAGC AATGGGGAAC TGGTTGCCAG GGTCTAANTT 300
TGGATTGATT TATGCACN 318

SEQ ID NO:7106
SEQUENCE LENGTH:299
SEQUENCE TYPE:nucleic acid
TOPOLOGY:linear
CLONE:HUMGS08342
SEQUENCE DESCRIPTION:
GATCTGTGAC CCCAGCCATG AGGACCCTCG CCATCCTTGC TGCCATTCTC CTGGTGGCCC 60
TGCAGGCCCA GGCTGAGCCA CTCCAGGCAA GAGCTGATGA GGTTGCTGCA GCCCCGGAGC 120
AGATTGCAGC GGACATCCCA GAAGTGGTTG TTTCCCTTGC ATGGGACGAA AGCTTGGCTC 180
CAAAGCATCC AGGCTCAAGG AAAAACATGG CCTGCTATTG CAGAATACCA GCGTGCATTG 240
CAGGAGAACG TCGCTATGGA ACCTGCATCT ACCAGGGAAG ACTCTGGGCA TTCTGCTTN 299

SEQ ID NO:7107
SEQUENCE LENGTH:302
SEQUENCE TYPE:nucleic acid
TOPOLOGY:linear
CLONE:HUMGS08343
SEQUENCE DESCRIPTION:
GATCTTAGAG GTTGAAGCCC ACCTTCTCTT TTCACATAGG AGGGAACAGA CCATGGAAAT 60
TTAAACGACT TCCTCACGGT CACAGAACTA GTTTTTTAAT CCTCAGGCAG TGNATCCCCC 120
CACCCTACAA CTGTGCACAA CCTCTTTCCC CACAGTGCAA TTCAGAATAT GCTCAGGGAA 180
TGCCAGGCAC CTTGTAAAAC TGCTGGGAGA AAAGCATGNT TCCCACAAGG ACTAAGTATC 240
AGTGATTTGT AATTTTCCTG TTTTGTATTA TCTGCTTTGC TGATGTAGAC AAGAGTTAAC 300
TN 302

SEQ ID NO:7108
SEQUENCE LENGTH:110
SEQUENCE TYPE:nucleic acid
TOPOLOGY:linear
CLONE:HUMGS08344
SEQUENCE DESCRIPTION:
GATCNAACTG TCTCACTGCT TTTCCAACTA TAATCATNTC GCTCTNACTT TAACAAAAGG 60
CGATGGCACA GTAATTTAGT AAATTTNTGT AATAACATNT AAAAAAAAGN 110

SEQ ID NO:7109
SEQUENCE LENGTH:121
SEQUENCE TYPE:nucleic acid
TOPOLOGY:linear
CLONE:HUMGS08345

SEQUENCE DESCRIPTION:
GATCCTGTCT AAAAAAGAAA AGAAAAGAAA AGAAAAGAAA AGAAAATCAG CATTGTATCC 60
CCAGAACCTG CTACAGTGTA CTGTACACTT AATAAATATT GGTTGAATGA ATGAACTGAA 120
A 121

SEQ ID NO:7110
SEQUENCE LENGTH:99
SEQUENCE TYPE:nucleic acid
TOPOLOGY:linear
CLONE:HUMGS08346
SEQUENCE DESCRIPTION:
GATCATCAGT AAATTTGTCA TGTTATATAT TTATTTTTTT ATAAATCAAG ACTTCTGTGT 60
GCTCTTAAAT ATATTAAAAA CAATTTACAT TTCAGGAAA 99

SEQ ID NO:7111
SEQUENCE LENGTH:92
SEQUENCE TYPE:nucleic acid
TOPOLOGY:linear
CLONE:HUMGS08347
SEQUENCE DESCRIPTION:
GATCCCAGNA GGAGGGAAGA AAATTTGCAG TGACTGAAAA CAGTAAAAAA AAAAAAAATA 60
ATGTATAAAA AAGTTGCATT ACACAGTNCA AA 92

SEQ ID NO:7112
SEQUENCE LENGTH:304
SEQUENCE TYPE:nucleic acid
TOPOLOGY:linear
CLONE:HUMGS08348
SEQUENCE DESCRIPTION:
GATCATTCTN GTTGATACCC TTCTTTGAAT ATTCTAGTGT ATTAATATAC CATTGGTTAT 60
TNAATCATGT CTNATTAATG GACTGGCTGT TTTCACATAT TNGATATATC AAGTGTCTTC 120
ACAACTTGTG CTTGCATATT CTTTCCCAAA ATATTGAAAG TCCATATATT TCCTTGTACA 180
TNTTTAAAGT TGATATCTNA ATNTNTCATT GTAGTNGCAA AGCATGTAAT TNCTTGGGGG 240
AGGGGGGCTG TAAATATTGA CATTTTAAAA TAAAACTTTT AAATCAGCCT TAAATGTATC 300
AGGN 304

SEQ ID NO:7113
SEQUENCE LENGTH:309
SEQUENCE TYPE:nucleic acid
TOPOLOGY:linear
CLONE:HUMGS08349
SEQUENCE DESCRIPTION:
GATCAAATTT CACGTCGTCT TCTGTATACT GTGGAGGTAC ACTCTTNTAG AAAGTNCAAA 60
AAGTCTACGC TCTNCTTTCT TTCTAACTCC AGTGAAGTAA TGGGGTCCTG CTCAAGTTGA 120
AAGAGTCCTA TTTGCACTGT AGCCTCGCCG TCTGTGAATT GGACCATCCT ATTTAACTGG 180

CTTCAGCCTC CCNACCTTCT TCAGCCACCT CTCTTTTTCA GTTGGCTGAC TTCCACACCT 240
AGCATCTCAT GAGTGCCAAG CAAAAGGAGA GAAGAGAGAA ATAGCCTGCG CTGTTTTTTT 300
AGTTTGNNN 309

SEQ ID NO:7114
SEQUENCE LENGTH:282
SEQUENCE TYPE:nucleic acid
TOPOLOGY:linear
CLONE:HUMGS08350
SEQUENCE DESCRIPTION:
GATCTTCCCC AGCTATGAAG CCTATCTTGG AATGGGAAAC CATGAAATTT GTCTTTTAAA 60
AGAAAGGGAA GAAATAGCCT TACAGCCCCT GTTCCTCCAA GTGCACATTT AGCCTTAAAA 120
TCCTGGGAGT CGGGAGACAC CGTGGAGTCC TTACATCCAC AGTGTCTGCT GGTCGTGGTC 180
AGAAGGCAGT AATGCAAGAG TCCTTTTGTG AAGAGTGTTT CTATGTAGAG ATGTTTATAT 240
TTAAGTAGTT CTTTTATAAA TAAAAGCATT TCTAATGGCA AA 282

SEQ ID NO:7115
SEQUENCE LENGTH:27
SEQUENCE TYPE:nucleic acid
TOPOLOGY:linear
CLONE:HUMGS08351
SEQUENCE DESCRIPTION:
GATCTGTTTT CTGTAACTTT TGCTAAA 27

SEQ ID NO:7116
SEQUENCE LENGTH:251
SEQUENCE TYPE:nucleic acid
TOPOLOGY:linear
CLONE:HUMGS08352
SEQUENCE DESCRIPTION:
GATCTTAGGC TTCAGCTCTT TATCTTACAC CTTGAGACCC CAAGAGAGTC CCAAATTCCT 60
GGAAAGGCAG CATCACAGAG TGGGGAAATA CAGGACTTGG CTTTAGGTTC TAGTCCTTAA 120
CACAGCTTCT TCCTTGCTGT GTGTTCTTTG GTAGGTCACT AAATCTCTCT GAGCCTCAAA 180
TTTCTTATCT CTAGGTTGAA AAATAAAATA AAGTCCCCAT GCCAATCAGT GCAAGGAATC 240
TGTGAGATAA A 251

SEQ ID NO:7117
SEQUENCE LENGTH:123
SEQUENCE TYPE:nucleic acid
TOPOLOGY:linear
CLONE:HUMGS08353
SEQUENCE DESCRIPTION:
GATCACGCCA CGGCACTCCA CCCTGGGCGA CAGAGCGAGA CCTTGTCTCA GAAAAAATCT 60
GTGAGGTTAA AAGGCTGTGT GTGTGAAAAT ATCTTGGAAA TTGTAAAGAA CTACACAANT 120
AAA 123

SEQ ID NO:7118
SEQUENCE LENGTH:295
SEQUENCE TYPE:nucleic acid
TOPOLOGY:linear
CLONE:HUMGS08354
SEQUENCE DESCRIPTION:
GATCATTCAT CAACATGTGT CTCTNAATTT AATTCAGTAG TTGTAATTAT TCTTTGGTTT 60
AGACCAAGAA AAAGGAAATC CCCCCTTTNA ATGTATTCCT TGGTTTNAGG ACATGACTCC 120
TGTAAGGNAG AGGAAAGGGA GATGCTTCCT GTTTGANCTG CAGTGAATTC ACGGTTCCTG 180
TTTCACCACT CCAAACCTTA TGGCGACTCA CACACACATT CCTCTTTNCT GTTACTGCCA 240
AAGGTTCGGG TTTAGTACAC TTCAGTTCCA CTCAAGCATT GAAAAGGTTC TCGTN 295

SEQ ID NO:7119
SEQUENCE LENGTH:203
SEQUENCE TYPE:nucleic acid
TOPOLOGY:linear
CLONE:HUMGS08355
SEQUENCE DESCRIPTION:
GATCCCCAGG ATGCCCACTT CCCTCCACCC CCACACCCCA GCCGTGTCCC TAACCCCTGG 60
CAACCAGGAA TCCACTCTCC ATTTCTATAA TGTTGNNATT TCAAGAATGT TATTCAATGG 120
AATCATATAG TATGTAACCT GTTTTGAGCT TAAAAAAAAA GTATACATGA CTTTAATGAG 180
GAAANTAAAA NTGGATATTG AAA 203

SEQ ID NO:7120
SEQUENCE LENGTH:82
SEQUENCE TYPE:nucleic acid
TOPOLOGY:linear
CLONE:HUMGS08356
SEQUENCE DESCRIPTION:
GATCCGTTAG TGTCTGTTTA GAAAAGATGT TATAAACTTA CAGAAACAAA TATAATAAAC 60
TGAAGCAGAT TTGAAAAGCA AA 82

SEQ ID NO:7121
SEQUENCE LENGTH:79
SEQUENCE TYPE:nucleic acid
TOPOLOGY:linear
CLONE:HUMGS08357
SEQUENCE DESCRIPTION:
GATCTTTTGC ATTGCAGAGA TTTTATATAT ATTTTGTTTG TAATGAGCCA TTCTCAATAA 60
ACATTATTCT CACTGCAAA 79

SEQ ID NO:7122
SEQUENCE LENGTH:299
SEQUENCE TYPE:nucleic acid

TOPOLOGY:linear
CLONE:HUMGS08358
SEQUENCE DESCRIPTION:
GATCCCTAGT AAACTCCTTC TTCACTTTTA CTGTCAGATT TACAAAGGTC CTCCCATTGC 60
AAAGCAGTGN NNNTCCTAAT TTATATATTG TTTTNCTAGT TCATTTTGTG TTTCCAACTT 120
TTCATGTAAA ATTTTAATTA TTTTTGAATG TGTGGATGTG AGACTGAGGT GCCTTTTGGT 180
ACTGAAATNC TTTTTCCATG TACCTGAAGT GTTACTTTTG TGATATAGGA AATCCTTGTA 240
TATATACTTN ATTGGTCCCT AGGCTTCCTA TTTNGTNACC TTGCTTNNNN NGTGGCANN 299

SEQ ID NO:7123
SEQUENCE LENGTH:108
SEQUENCE TYPE:nucleic acid
TOPOLOGY:linear
CLONE:HUMGS08359
SEQUENCE DESCRIPTION:
GATCATGTGC CAAGGCTGCG GTGCGTTCTG TCACGATGAC TGTATTGGAC CCTCAAAGCT 60
CTGTGTATTG TNCCTTGTGG TGAGATAATA AATTATGGCC ATGGGAAA 108

SEQ ID NO:7124
SEQUENCE LENGTH:113
SEQUENCE TYPE:nucleic acid
TOPOLOGY:linear
CLONE:HUMGS08360
SEQUENCE DESCRIPTION:
GATCAGACTG TGCTTTCTAA TTCTAAAAAA TTGAGCAGTG TTGGTATTCT TNCAGTATTT 60
TTNCTCTCAC CTGGAAAACT ACCTGGAAGT TGTCAGGCAT TTNCTTACTG NNN 113

SEQ ID NO:7125
SEQUENCE LENGTH:125
SEQUENCE TYPE:nucleic acid
TOPOLOGY:linear
CLONE:HUMGS08361
SEQUENCE DESCRIPTION:
GATCTCTAAG TAATTNTNCA AAATCCGGTA TTACTATGTC AAGTTATTGC TTTTGGTAAA 60
TTCATCTGAC CCAGTTATAA ATGAAAGANT ATGGANTTAA AAATTTTAAA NCTAAATAAT 120
TTGNN 125

SEQ ID NO:7126
SEQUENCE LENGTH:122
SEQUENCE TYPE:nucleic acid
TOPOLOGY:linear
CLONE:HUMGS08362
SEQUENCE DESCRIPTION:
GATCATGCTC CATCCAGCCC CACCCAATGG CCTTTTGTGC TTGTTTCCTA TAACTTCAGT 60
ATTGTAAACT AGTTTTTGGT TTGCAGTTTT TGTTGTTGTT TATAGACACT CTTGGGTGTA 120

AA 122

SEQ ID NO:7127
SEQUENCE LENGTH:256
SEQUENCE TYPE:nucleic acid
TOPOLOGY:linear
CLONE:HUMGS08363
SEQUENCE DESCRIPTION:
GATCGGCACT CACGATTGTN ATGTTGGCTA CATCCTTGAC AAAGCCGATG GCCTTTCCAG 60
GTTGGAACTT GCAATAACCA TCCTGTTGAG GATGCAAAGG GCATGAAATA CAGGTTATGG 120
ACCCGAAGTC TCAGCCTCCC CACGCAGTTC AATAACATTT ACTAAACAAA ACAAGATGTG 180
CCAGGGCCTG GGGGATGGGA TAATTTCAGA GAGAATTAAA GCATCCTTGT CCTCAAGGAG 240
CTTAGGGTCT GGTAAA 256

SEQ ID NO:7128
SEQUENCE LENGTH:346
SEQUENCE TYPE:nucleic acid
TOPOLOGY:linear
CLONE:HUMGS08364
SEQUENCE DESCRIPTION:
GATCAACAGG GGTGAAAAGA ACCACCCTGA GGTTTCCATG CCTCTCCCAN NTTTAGTGGT 60
AGNATTTTGT GTCTTTACTC CACCCTTCAC CCTAGTTCCA CCAAGGTTCA CACACCAGAT 120
GTAACTGTTT TNNAGCTGAG TTGTATGGAT TAACTTCAGT CCACTGTAAA TACACCTGGG 180
ATGGGGTGGG GTTGGGGTTG TTTAGGGAGA AGCAGCCAGA CTTGCTTTGT GAACTGAATG 240
TATTTTTATG CAATTTTGAG TGGCCTTTCA ACCCTAAGAT GAATGGTTTT GTTTTACTGG 300
TTGTTGTTAT ATAGTTTTGA GTATTCTGTG TTTGAAAGTT TGGGTN 346

SEQ ID NO:7129
SEQUENCE LENGTH:42
SEQUENCE TYPE:nucleic acid
TOPOLOGY:linear
CLONE:HUMGS08365
SEQUENCE DESCRIPTION:
GATCGTGTCT CTGATAAAAG AATAAAAGCT GTTTATTGCA AA 42

SEQ ID NO:7130
SEQUENCE LENGTH:338
SEQUENCE TYPE:nucleic acid
TOPOLOGY:linear
CLONE:HUMGS08366
SEQUENCE DESCRIPTION:
GATCTTAGGT CCCACTCANG GTCCTAACTG GCTATGGCCA CAGCTGGAAC TACCCGAGAA 60
GNCTTTTGTA CATGTTTGGT AGCCGTAGCA CAAGTGATTG GAGTAGAACA TGTCACTGCT 120
GTACATTTTT AACTCCCCTA ATGGTGTGTC TATAATTNTN AAATCTAAGG GGGGGGGCTC 180
AGTAAAGCCT CCTGGCACCA GGCCTTCCTG CTCGACTGAA AAAAATTTCN TCTTTGAAAA 240

TCCCCTTTTA CTCATGGCCC ACAGTAGAAT ATCCAAAACG CCTTGGCTTT CAGGCCTGGC 300
CTTTCCTACA GGGAGCTCAG TAACCTGGAC GGCTCTAN 338

SEQ ID NO:7131
SEQUENCE LENGTH:294
SEQUENCE TYPE:nucleic acid
TOPOLOGY:linear
CLONE:HUMGS08367
SEQUENCE DESCRIPTION:
GATCTTTTCA AGATGCATTC CAGATGAACT GCTAGGTGAG GGGGAAGCTT CATTTTTGTT 60
ACCTGATAGA ATAGCTTTNC TNATGAGATA TATATAATGT GATACTATGT TTGGATATTT 120
TTGGTCTTAA AGCAAGACTC AGTGGTGTAT CTNCATTAAA AGCTTCCTTT AAAAAAGNTA 180
CAGGGNTACT AAAAAAAACAA GNACCCAAAC AATCAAGNTG GGCCCACCTT GGNACCNTGT 240
TTTGNATNAC CTTCCATGGT TTGGTTGGCG GTATGGNAAA AGGATTGNNT GGTN 294

SEQ ID NO:7132
SEQUENCE LENGTH:107
SEQUENCE TYPE:nucleic acid
TOPOLOGY:linear
CLONE:HUMGS08368
SEQUENCE DESCRIPTION:
GATCATGCTG TGTGGAGGAC AAATCTTTAA AATTCCAGCA TTGTATTGTC TATTGACACA 60
CAAAGTTTGA AAATAAAGGG GCAAAAAATT TTAATGCAAA AATGAAA 107

SEQ ID NO:7133
SEQUENCE LENGTH:328
SEQUENCE TYPE:nucleic acid
TOPOLOGY:linear
CLONE:HUMGS08369
SEQUENCE DESCRIPTION:
GATCTGCGAT GGCTGACATN AATAGGTCAC CATGTTGGCA GCTGTATAAT CAGCAATGAA 60
AGCTGAATAT TATCACCAGA TAACCAAATT ATGAAATATC TATATNANCT TCANGATTCA 120
GGNCCANTGA ACCAGTATCT GCCCTTACGA GATTTAGCTG AANGTGAANT GCAAGGTGGT 180
GAACGGCATT CTAACAAGAG TGTGAAGAAA GAGATNGANG GCTGTCCGCT GCTGGAGGGG 240
AAGAANGGTG GGGCGGCTTT TNCAAGGCTC TGTGGCTCAG TGGACCTTCA ATANATTGGA 300
CCTTCAATAA ATTAATTGNA AANCANGN 328

SEQ ID NO:7134
SEQUENCE LENGTH:244
SEQUENCE TYPE:nucleic acid
TOPOLOGY:linear
CLONE:HUMGS08371
SEQUENCE DESCRIPTION:
GATCTGAGGG ATTTGCTCGT CATTTGCTGT GGCAGAGATG ATGTTGACAC CCTGGGATGC 60
TGTGGGAGGG ACAAAAGACT CACGTATATG TTCTGTCAAA TCGGAGAAGT AAATAATCCG 120

TTGGGATGCC AGGATACTTT ATTCTACAGC GAAGTTAATT TTGTATCCGT CGAGGCTACA 180
GTAGAGGTAA AAATGACATT GAGTTGTACT TTGCTTTGAA TTAAAGTTGA TGGGTAGCAG 240
CAAA 244

SEQ ID NO:7135
SEQUENCE LENGTH:48
SEQUENCE TYPE:nucleic acid
TOPOLOGY:linear
CLONE:HUMGS08372
SEQUENCE DESCRIPTION:
GATCTTGACT CCAAAAATTA ATAAATAAAT AAAAATAAAT AAAATAAA 48

SEQ ID NO:7136
SEQUENCE LENGTH:48
SEQUENCE TYPE:nucleic acid
TOPOLOGY:linear
CLONE:HUMGS08373
SEQUENCE DESCRIPTION:
GATCATGGTA TAATTAATTG AAATTTATTA AAATCTGTTT TTATTAAA 48

SEQ ID NO:7137
SEQUENCE LENGTH:267
SEQUENCE TYPE:nucleic acid
TOPOLOGY:linear
CLONE:HUMGS08374
SEQUENCE DESCRIPTION:
GATCTGTGAC TTCAAGTTTG GACCGTGGAA GAACAGCACT TTCAAACCCA CAACTGAGAA 60
TNATGACACA GGGACAAGTA GCAGTGATAC ATCAGATGAC GACGATGTNT GAAGGATTTC 120
CTAACAGCTT TNGAAATCTT AGTGTGATAC ATCTCTCATA CAGTTTGGGG NGAATTGTNA 180
AAATGAAAGG ACTATANTTT ATGTAGTGGA ATACCCCCNN AGAAGAGGNN TTTTTTGGGG 240
GCTTCAACTT GNTGGAAACC NAGANTN 267

SEQ ID NO:7138
SEQUENCE LENGTH:314
SEQUENCE TYPE:nucleic acid
TOPOLOGY:linear
CLONE:HUMGS08375
SEQUENCE DESCRIPTION:
GATCATTGAA ATTCTGAGAA AACTTCTTTT AAACCTCACC TTTGTGGGGT TTTTGGAGAA 60
GGTTATCAAA AATTTCATGG AAGGACCACA TTTTATATTT ATTGTGCTTC GAGTGACTGA 120
CCCCAGTGGT ATCCTGTGAC ATGTAACAGC CAGGAGTGTT AAGCGTTCAG TGATGTGGGG 180
TGAAAAGTTA CTACCTGTCA AGGTTTGTGT TACCCTCCTA TAAATGGTGT ACATAATGTA 240
TTGTTGGTAA TTATTTTGGT ACTTTTATGA TGTATATTTA TTAAACAGAT TTTTACAAAT 300
GGAANAAAGA NAAA 314

SEQ ID NO:7139
SEQUENCE LENGTH:349
SEQUENCE TYPE:nucleic acid
TOPOLOGY:linear
CLONE:HUMGS08376
SEQUENCE DESCRIPTION:
GATCCAGAGT AAATGAGAAG TCTCTATCTG AGCTGGTCAG TTACTGGAGT ACATGTNACT 60
AATCTGGGTT TAAAGTTTAC TTCATTATCT GCTAGTGTCA TCCACAGCAG TTCATCCTCA 120
TCCACACTAA GCCATCCTGT NAGCTTTTAA AGGAAGTTAA TTTAATTAAC ATTAATATAC 180
TCTATGGGCT CCCTCTCCCA CCTGTCTGCA TAGAAAGGCA GAATTAGACA TAGCATGCTT 240
TGGAAAAGCA AATAGGAATT GTTGGGAATG ATTTANTCTT GTTGTTGTTG TTGTTGTTGT 300
TCACTTGTGG TTCTACATTC CTGGTGNATG NTGAATGTNG CTGTCAAGN 349

SEQ ID NO:7140
SEQUENCE LENGTH:347
SEQUENCE TYPE:nucleic acid
TOPOLOGY:linear
CLONE:HUMGS08377
SEQUENCE DESCRIPTION:
GATCTTGTGC TTCCATTTAA GAAATTCTTC CATTTAAAGA AGAAAAAAAA TCTCTCTAAT 60
TGACTATCTG AAGATATATG AAAAAGCCTA TGCTTTTAAA TTAANCTGTT AAGACAGTCC 120
ATTGAAAGAT TGTGGAAGTN CACATCTATT TTNCACCTTA ATTTTTTCCA TTGTCCCTAC 180
TCATGACTCT AAAAAGTGCA TGGCTTGGGG CTATACTTTG TTTTGCAGTT TGTTGGTATC 240
GTGCCTTTCC TTATCTACAT TAGCTTAGAC TATACCTTNT TTTTNAGAAG GGGAAGTGGN 300
AATTAACTGT GGCAAAACCT ATTTNGGCAC ACCNCCTTTG TTCAATN 347

SEQ ID NO:7141
SEQUENCE LENGTH:121
SEQUENCE TYPE:nucleic acid
TOPOLOGY:linear
CLONE:HUMGS08378
SEQUENCE DESCRIPTION:
GATCTTAATT CTTTGCAATT ATANATGAAA AATGACTGAT TTTTCTNAAA ATATGTAACT 60
TATNTAAATA TATCTGTTTG TACAGATTTT AACCATAAAA ACATTTTTGG AAAACCATAA 120
A 121

SEQ ID NO:7142
SEQUENCE LENGTH:409
SEQUENCE TYPE:nucleic acid
TOPOLOGY:linear
CLONE:HUMGS08379
SEQUENCE DESCRIPTION:
GATCTGGAAA CTATTTGGGT TTTGTTTTCA ACTTTTCATT NGGATGTTTG NCNTTGCACA 60
CACACATCCA CCGGTGGAAG AGACGCCCGG TGAAAACACC TGTCTGCTTT CTAAGCCAGT 120
NAGGTTGAGG TGAGAGGTTT GCCAGAGTTT GTCTACCTCT GGGTATCCCT NNTGTCTGGG 180

ATAAAAGATN TCAAGCCAGT TGGCGGGATG GAATGGNTGC ACCGCAAATA ATGCATTTNC 240
TGAGTTTTCT TGTTAAAAAA AAATTTTTTT AAGTAAGNAA AAAANAGGTA ATAACATGGC 300
CAATTTTNTT ACATAAAATG GCCTTTCTGG NGTNTAAATT ATTCCTAAAA AAATCCNGTT 360
TTATATANAC NATCCAGTTG NTGGAAAAAA NTTTTCCAAA NGTTTTTGN 409

SEQ ID NO:7143
SEQUENCE LENGTH:89
SEQUENCE TYPE:nucleic acid
TOPOLOGY:linear
CLONE:HUMGS08380
SEQUENCE DESCRIPTION:
GATCTAGAAA ACTTTGGATT TTTGAAGTAA ATTTTAATGT TTCATATTAA TTTCTTGAAA 60
ATGTATTAAA TGTCATTGAA AGCCTTAAA 89

SEQ ID NO:7144
SEQUENCE LENGTH:375
SEQUENCE TYPE:nucleic acid
TOPOLOGY:linear
CLONE:HUMGS08381
SEQUENCE DESCRIPTION:
GATCACTGTG GACCATCTTA GCAGTTGACC TAACACATCT TCTTTTCANN ATCTAAGAAC 60
TTTTGCCACT GTGACTAATG GTCCTAATAT TAAGCATGTT GTTTATATTT ATCATATATC 120
TATGGCTACA TGGTTATATT ATGCTGTGGT TGCGTTCGGT TTTATTTACA GTTGCNANTA 180
CAAATATTTG CTGTAACATT TGACTTCTAA GGTTTAGATG CCATTTAAGA ACTGAGATGG 240
ATAGCTTTTA AAGCATCTTT TACTTCTTAC CATTTTTTAA AAGTATGCAG CTAAATTCGA 300
AGCTTTTGGT CTATATTGTT AATTGCCATT GCTGTAAATC TTAAAATGAA TGAATAAAAA 360
TGNTTTCATN NTAAA 375

SEQ ID NO:7145
SEQUENCE LENGTH:513
SEQUENCE TYPE:nucleic acid
TOPOLOGY:linear
CLONE:HUMGS08382
SEQUENCE DESCRIPTION:
GATCTCGCTC CTTTGAGAGG TCCCATAAAA GCAAGCACCA TGGTGGCAGT CGCTCAGGAC 60
ATGGCAGGCA CAGGCGCTGA CTTTCTCTTC CTTTGAGNCT GCATCAGTTC TTGGTNTTGC 120
CTATCTACAG TGTGATGTAT GGACTCAATC AAAAACATTA AACGCAAACT GATTNGGATT 180
TGATTTCTTG AAACCCTCTA GGTCTCTAGA ACACTGAGGA CAGTTTCTTT TGAAAAGAAC 240
TATGTTAATT TTTTTGCACA TTAAAATGCC CTAGCAGTAT CTAATTAAAA ACCATGGTCA 300
GGTTCAATTG TACTTTATTA TAGTTGTGTA TTGTTTATTG CTATAAAGAC CTGGAGNGTG 360
AATTCTGTAA AAATGTATCT TATTTTTATA CAGTTAAAAT TTGCAGACAC TGTTCTATTT 420
AAGNGGTTAT TTTGTTTAAN TGGATGGGNG ATNCCTTNTT AANCACTGGN TTGGNCTGGA 480
TNGTGTAAGG TTTTTNCAGG GNNTAGTTCC TGN 513

SEQ ID NO:7146

SEQUENCE LENGTH:180
SEQUENCE TYPE:nucleic acid
TOPOLOGY:linear
CLONE:HUMGS08383
SEQUENCE DESCRIPTION:
GATCTGAGAA TCTTTATTGA GAAAGAGCAC TTAAGAGAAT ATTTTAAGTA TTGCATCTGT 60
ATAAGTAAGA AAATATTTTG TCTAAAATGC CTCAGTGTAT TTGTATTTTT TTGCAAGTGA 120
AGGTTTACAA TTTACAAAGT GTGTATTAAA AAAAACAAAA AGAACAAAAA AATCTGCAAA 180

SEQ ID NO:7147
SEQUENCE LENGTH:187
SEQUENCE TYPE:nucleic acid
TOPOLOGY:linear
CLONE:HUMGS08384
SEQUENCE DESCRIPTION:
GATCAAGAAA CTGGAAAATG TGAAGCAATC TCTATTGAGA TAGCATCTTA GTGTTTTAGA 60
GAAATCAAGA ATTTTTAAAA ACAAGAATAT CAACATTTGG TTTTGTGTAT AAGTGGTGTT 120
TGTATTAAAA TACTTTTTCA ATGAACTGTA TAAACTATGT TTTATTAAAC TACAATATAT 180
CAGTAAA 187

SEQ ID NO:7148
SEQUENCE LENGTH:133
SEQUENCE TYPE:nucleic acid
TOPOLOGY:linear
CLONE:HUMGS08385
SEQUENCE DESCRIPTION:
GATCCAGAGG CGAGAAGCCA CCCTGTTAGA NCTCCTGTCC ATCATCCAGG TAAGTCAACT 60
CTCTCCTTTC AGGGCCAATC CCCAGACCTT TTGTTGAGCC AGGNCNNTAT CACCTNTCCT 120
ACTNACTTAA AGN 133

SEQ ID NO:7149
SEQUENCE LENGTH:172
SEQUENCE TYPE:nucleic acid
TOPOLOGY:linear
CLONE:HUMGS08386
SEQUENCE DESCRIPTION:
GATCCTTGTC AAAATGAGAT TCCAGGTCCT AGAGAGCTGC TTTNAGGGAG TTCCAGGAGT 60
ACTTACTATT GGTCATGCAA TAGGAGAACA GAGACCCGNG GGCTGCTTTG GGGGAGGGGG 120
GAACTCGAGA ATGTATGGAT TTACCTGAAA ACAAATTATT CATTTAATCA AA 172

SEQ ID NO:7150
SEQUENCE LENGTH:250
SEQUENCE TYPE:nucleic acid
TOPOLOGY:linear
CLONE:HUMGS08387

SEQUENCE DESCRIPTION:
GATCACCATT CAATTTGAGT TTCCAGGGGG AAGTGCATGT ATAATGAAAT GATAATGGAC 60
TTCAATGAAG AATGTCATCA ATTATGTACA TATGTATTTN CTTTTAATAC AAAGTGTAAT 120
TTTGTGCCAG TGAAATGGAG TCTGAATAGT TATGTGTTTC TTTTATCCCT GGAAATATTT 180
ATTAAACTTT ATAGTTTATC CGAGTATGTT GTATGCTTTG ACAATAAATG ACTATTTTCT 240
TCAAAGCAAA 250

SEQ ID NO:7151
SEQUENCE LENGTH:282
SEQUENCE TYPE:nucleic acid
TOPOLOGY:linear
CLONE:HUMGS08388
SEQUENCE DESCRIPTION:
GATCATCACC ACCACGGTTC CTGAGGACAT CATGTCCAAC CGAGGCAATN ACCTCCTGCT 60
GAAGCTCCTG CAGNATCGGT ATCCCCGGGT GATGGCAGAG GAGGGTCTTC GAGTGGTGAG 120
GCAGTGGTTG GAGGCCTCCT CACAGCTGGA GGAAGCCTCA NTTTATAGCC GATGGGAGGT 180
GGAAGAGGAC TGGTGTCTGT NTGTCCTCCG CTCCTACCAG GCAGAACACG GGCCCGACTT 240
CCCCTGGAGC GTGGGGGAGG ACATGAGTGC AGATGGACGG TN 282

SEQ ID NO:7152
SEQUENCE LENGTH:391
SEQUENCE TYPE:nucleic acid
TOPOLOGY:linear
CLONE:HUMGS08389
SEQUENCE DESCRIPTION:
GATCAAAGTA GAAGCNTTTG AATAATGAAT TTAAGGCCAC GCAAAANCTT TCCTCACCTA 60
TTGACATGAC ATCTAAAAGA CATTTCGAAC TCGAAAAGCA TAGTGCCCCA AGTATGGTAC 120
ATTCTNCTCT NACTCCTTTC TCAGTGCAAG TANCTAACAT TCAAGATTGG NCTCTCAAAT 180
CGGAGCACTG GCATCAAAAA GANCTGAGTG GCAAANCTCA GAATAGTTTC AAAACTGGAG 240
TTGTTGAAAT GAAAGACAGT GGCTACAAAG TTTCTGACCC AGAGAACTTG TATTTGACGC 300
AGGGGNTAGC AAACTTATCT GCAGNGGTTG TCTCACTCAA GAGNCTTNTA GNCACACAAC 360
CAGTCTCTGT TTCAGACTCT GGGTAANTTN N 391

SEQ ID NO:7153
SEQUENCE LENGTH:119
SEQUENCE TYPE:nucleic acid
TOPOLOGY:linear
CLONE:HUMGS08390
SEQUENCE DESCRIPTION:
GATCTAAAAA TAATTCTTAA TCATGAATTT AATTCACGAC TTCCTTGTTT TCAGTCTTAT 60
TCAGCTTACT GTAGGAGATT TTAATANACT AAAGCTTTTG TTGATTTATG AGTTAGAAA 119

SEQ ID NO:7154
SEQUENCE LENGTH:284
SEQUENCE TYPE:nucleic acid

TOPOLOGY:linear
CLONE:HUMGS08391
SEQUENCE DESCRIPTION:
GATCTTGTAC CAAGCCAACG GGTNTCCTGG CTCTCCTGCC CACAGGATGA ACATTTTCGG 60
CTTCCTTAGG AGTTTTGCCC TACCGTATTC CAAAGCGTGT GCTGGTTTCT CATATTGTCT 120
GTAGGCTCAC TCAGCCCGCA GTTTATGTGT GTGCTTTTTT CTATGAAAAA TGATGTATTT 180
TGCTACTTCC TGTGTACAAA GTTTTATTGT AAATGTTTTT TGTGCTTTGC ATGAACAGGG 240
GCCACGTTGT TGCAATTGTT TCAGTAGAAC TGGTTTGATT TCTN 284

SEQ ID NO:7155
SEQUENCE LENGTH:289
SEQUENCE TYPE:nucleic acid
TOPOLOGY:linear
CLONE:HUMGS08392
SEQUENCE DESCRIPTION:
GATCGAGACC ATCCTGGCTA ACACAGTGAA CCCCGTCTCT ACTAAAAATA TAAAAAATTA 60
GTCGGGCATG GTGGCGGGCG CCTGTAGTCC CAACTACTCG GGAGACTGAG GCAGAAGAAT 120
GGCGTGAACT CAGGAGGCGG ANTTGCAGTG AGCCAAGGCG ACAGAGCAGG ACTCTGTCTC 180
AAAAAATAAA AAATAGTGCA CTGTCCTTCG AGAAAGTTTT CTAACATCTA GTAATTTGTA 240
ACTTAGAAGT GGAGTTGCCT TGTGGATGTC TTTTTTGCAT TCTGTAGGN 289

SEQ ID NO:7156
SEQUENCE LENGTH:293
SEQUENCE TYPE:nucleic acid
TOPOLOGY:linear
CLONE:HUMGS08393
SEQUENCE DESCRIPTION:
GATCCAACTG CAGAGATGGC AGCTGAGTCA TTGCCAGTNT CCTTCGGGAC ACTGTCCAGC 60
TGGGAGCTGG AAGCCTGGTA TGAGGACCTG CAAGAGGTCC TGTNTTCAGA TGAAAATGGG 120
GGTACCTATG TTTCACCTCC TGGAAATNAA GAGGANGNNT CAAAAATCTT CACCACTCTT 180
GACCCTGCTT NTTTGGCTTG GCTGACTGAG GAGGAGCCAG NACCAGCAGA GGTCACAAGC 240
ACCTCCCAGA GCCCTCACTC TCCAGATTCC AGTCAGGGCT CCCTGGCTCA TGN 293

SEQ ID NO:7157
SEQUENCE LENGTH:69
SEQUENCE TYPE:nucleic acid
TOPOLOGY:linear
CLONE:HUMGS08394
SEQUENCE DESCRIPTION:
GATCACGATG TAAATAATNA AGCATTTTGG TAGGATGTCT ANNGTAATAA ACAATTTTTG 60
TACCATAAA 69

SEQ ID NO:7158
SEQUENCE LENGTH:312
SEQUENCE TYPE:nucleic acid

TOPOLOGY:linear
CLONE:HUMGS08395
SEQUENCE DESCRIPTION:
GATCTTGGCC TCCCAAACCC CTTATTTTTA AAATACTTTG CGCCTTGCTT TGATAATTTG 60
TATTATGTAT CCAAACTGAA ATNATCTGCT TTCTGCATTA GAATGTAAGC CCCCTGAGGG 120
TTGAGTCAGT CTGTCTTGTT TGCTGTGCCA CGCCTGATGC CCAGCCCAGC AGCATGCTTT 180
GTACACTGAT ATATTGGGTA AATTTTGTTG AATAAATTAA GCTCAACTAT TTGTATTTCA 240
ATAGNTTGAG TTGTATTGCT TCCTGTTCTT CAAGCTTAAA TTTGACCTGT CTAATAAAAA 300
GGAGNAATTA AA 312

SEQ ID NO:7159
SEQUENCE LENGTH:92
SEQUENCE TYPE:nucleic acid
TOPOLOGY:linear
CLONE:HUMGS08396
SEQUENCE DESCRIPTION:
GATCTTTTGT GGTCCTGTAA AAGATACTGA GGAATGTNTT TCAGCCAAGC CAAGAGGATG 60
GTTTCAATAA ACCTAATAAT CTGAAGTTCA AA 92

SEQ ID NO:7160
SEQUENCE LENGTH:61
SEQUENCE TYPE:nucleic acid
TOPOLOGY:linear
CLONE:HUMGS08397
SEQUENCE DESCRIPTION:
GATCTTGCAA TATATACTCA GGAAATTACC ATGGGCAGAA TAAAATGCTT TTAAAAATAA 60
A 61

SEQ ID NO:7161
SEQUENCE LENGTH:319
SEQUENCE TYPE:nucleic acid
TOPOLOGY:linear
CLONE:HUMGS08398
SEQUENCE DESCRIPTION:
GATCCAAACT GNAGAACTTC TCATTCATCC CCAAGGCCTC CAGGCAGTAT CCAATGGGGA 60
ATCAGCTCTA AAAGGAACCA GACCAACGTT TTCCAGCCCC TTNATTCTGC TTCCNTCTGT 120
NTNAGGAAAG GNTAGAAATN TTCAGGGCAT CATCATACAG GCTCCTCATC TACAAAGTTC 180
CAGTAGCAGT GACGCCTACA CGGAAGANTT GGAACTGCAA ACAGGCTGGG GTCACCTCAN 240
TNGCATCTGA CGCTGTCCAA CCAGAAGTTC GATTTTNNTN CTGGGGGTGA AGGTGGAAAC 300
AGACTGTACT TAAAGGNTN 319

SEQ ID NO:7162
SEQUENCE LENGTH:134
SEQUENCE TYPE:nucleic acid
TOPOLOGY:linear

CLONE:HUMGS08399
SEQUENCE DESCRIPTION:
GATCCANAAA TAAGTTTAAC TTACGGAGGC TTCTAGTTCT AAAGATGCAA NGTAAGATGC 60
CAAAGCAGTG GAAAGATTGA AGTCAAACAG TCGGGCCCAT GTGTGTGTCN TACNTCAACC 120
NTTNGTGTGT TATN 134

SEQ ID NO:7163
SEQUENCE LENGTH:147
SEQUENCE TYPE:nucleic acid
TOPOLOGY:linear
CLONE:HUMGS08400
SEQUENCE DESCRIPTION:
GATCTATGTG TCTGTNTGTG TGTCCATCCC GCCGACCCCC CAGACTAACC TCCAGGCATG 60
GACTGAATCT GGTTCTCCTC TTGTACACCC CTCAACCCTA TGCAGCCTGG AGTGGGCATC 120
AATAAAATGA ACTGTCGACT GAACAAA 147

SEQ ID NO:7164
SEQUENCE LENGTH:320
SEQUENCE TYPE:nucleic acid
TOPOLOGY:linear
CLONE:HUMGS08402
SEQUENCE DESCRIPTION:
GATCTAGNAA CTAGNAAATA CTATTTGTCC CAGCAATCCC ATTACTGGGT ATATACCCAA 60
AGGAATATAA ACCATTTAAT TATAAAGATA CATGCAGATT TTTATTCATT GCAGCACTCT 120
NNACAATAGC AAAGACACAA TAGCAAATGC CCATCAAAGA TAGACTGGAT AAAGAAAATG 180
TGGTACATAT ACACCATGGA ATACTGTGCA GTGCAGCCAT TACAGCTTTT GGTGATACAG 240
TGAATCAGAT TTTNNATTAA TTCTTTTAAT TGGTTATTAC TGNNCGTGGA NNAGTAATGG 300
TTTGTATTGG NATCCTTGNN 320

SEQ ID NO:7165
SEQUENCE LENGTH:74
SEQUENCE TYPE:nucleic acid
TOPOLOGY:linear
CLONE:HUMGS08403
SEQUENCE DESCRIPTION:
GATCCATATT TTAAATTGCA ACCCTGTTCC CTATGATACC TATCTGTCTA TGAATGAAAC 60
AAAGGTTTTA CAAA 74

SEQ ID NO:7166
SEQUENCE LENGTH:178
SEQUENCE TYPE:nucleic acid
TOPOLOGY:linear
CLONE:HUMGS08404
SEQUENCE DESCRIPTION:
GATCCAAAGA ACTTCTGTTA TATGGGTTAT AGCTGTTAAT ATTTGTCATA TTCGAAATTG 60

AAATAAGATT TTAAGGTATT TTAAAATAAC AGTNCAAAAC CATTGTATNT TGACATGAAT 120
AACACTTTTA TGAAAATAAC TATATTCCAA AACAATAAAA TTACCAAAAA GTTGCAAA 178

SEQ ID NO:7167
SEQUENCE LENGTH:98
SEQUENCE TYPE:nucleic acid
TOPOLOGY:linear
CLONE:HUMGS08405
SEQUENCE DESCRIPTION:
GATCACAGAT TTCATCAGGA TTGAGCTGCG TGGTAAGCTC GCCACTCTGG ACAAGTCACA 60
GGGTACCCAT TATTTAGCAA TAAAAGCTTT AACTCAAA 98

SEQ ID NO:7168
SEQUENCE LENGTH:110
SEQUENCE TYPE:nucleic acid
TOPOLOGY:linear
CLONE:HUMGS08406
SEQUENCE DESCRIPTION:
GATCTCTGTG GCCTAGGTTT TGTACATACA GAAACTGCAT GGTATTTAAA TTATTGTTTG 60
TCTCTGATGA TGTATGCAGT TTCTTTAAAA ACAAACCAAA AAAAAGTAAA 110

SEQ ID NO:7169
SEQUENCE LENGTH:298
SEQUENCE TYPE:nucleic acid
TOPOLOGY:linear
CLONE:HUMGS08407
SEQUENCE DESCRIPTION:
GATCCTGAAG AGCGCTGAGG GACGGGTGCT TCGNCGANCA GCACCAGCGA NACGTCAAAC 60
TCATGAGACA GATGCTAATG GATGCCGNCC TCCCTANTTG TCCACTGCCC CAGCCACATC 120
ATCCCTGTGC GGGTTGCAGA TGCTGCTAAA AACACAGAAG TCTGTGGATG AGCTAATGAG 180
CAGCCATAAC ATCTACGTGC AAGCAATCAN TTACCCTACG GTGCCCCGGG NAGAAGAGCT 240
CCTTCGGGNT TGCCNCNACC CTTNNGGGGG GACCCCAGGA TTGNTGGAAC TAANTTCN 298

SEQ ID NO:7170
SEQUENCE LENGTH:329
SEQUENCE TYPE:nucleic acid
TOPOLOGY:linear
CLONE:HUMGS08408
SEQUENCE DESCRIPTION:
GATCCACCCG CCTCGGCCTC CCAAAGTGCT GGGATTATAG GCGTGACCNC TGCACCCGGC 60
CAAAAGTTGA TTTTTAATTA CATAAAAATC GTAAAAACTT CTAGTAAAAA CTTGATTNNG 120
NGANTACAGT TATATTTTAA AACCTTAAGG TGACAAGCAT TTTCTATGCC TAAATCTTCA 180
TTGGTTTGCC TGGAAAGAGT CTCTGTTAAA AGATTTTCCA TATTCAAAGT AAAAGGAAAG 240
ATTTCTTGCT TTCTAATTGT CTTTTGGACA CATGCCTATT TTCTTTGAGG TATAANCCTT 300
TAGATGTGAA ANATGTAATT TCCATTCNN 329

SEQ ID NO:7171
SEQUENCE LENGTH:213
SEQUENCE TYPE:nucleic acid
TOPOLOGY:linear
CLONE:HUMGS08409
SEQUENCE DESCRIPTION:
GATCCAGATT TNTTCTGTTG TGTCTGAAGA GAACCATGAC ATTTAGAAGT ATATTGGTAA 60
TCTATTAGGT CCATTGGCAA AGTATATTGG TCCATATTCA AACCTATTCC AAAAGGTTAG 120
AAGTGTTTAA GATTCCTTTA TTGTGGTACC ATGTCTGTTT ACCTATGCTT ATGAGCAACA 180
ATAANTTACT AGTTTACCTT CTGGAATTTT AAA 213

SEQ ID NO:7172
SEQUENCE LENGTH:332
SEQUENCE TYPE:nucleic acid
TOPOLOGY:linear
CLONE:HUMGS08410
SEQUENCE DESCRIPTION:
GATCTGGTGG GCAGTCGAAC CATGGTGAAC TCCACCTCCG TGGAATAAAC GGAGATTCAG 60
CGTGGGCGAC TGAATCCAGC ACGTCTGTGT GAGTANCGGG ACAGTAAACA CTCCACATNN 120
NNTCAGTTTT TCACTTCTAC CTACATATTT GTATGTTTNN NTGTATAACA GCCTTTTCCT 180
TCTGGTTCTA ACTGCTGTTA AAATTAATAT ATCATTATCT TTGCTGTTAT TGACAGCGAT 240
ATAATTTTAT TACATATGAT TAGAGGGATG AGACAGACAT TCNNCGGGNT ATTNCTTTTA 300
ATGGGCACAA AATGGGCCCT TGCCTCTAAN GN 332

SEQ ID NO:7173
SEQUENCE LENGTH:332
SEQUENCE TYPE:nucleic acid
TOPOLOGY:linear
CLONE:HUMGS08411
SEQUENCE DESCRIPTION:
GATCCAGAGG CTCAGTGAAC CCCATCAACT TGGTGGCCCT GGTGTCTCAC ACTTGTATCC 60
TTCTGCCCTC GAGACCTGGC ACAGCAGTAT CCCTTGAAGA AATCCTGAGG CTTTGTAGAG 120
TGCTCCTTGA CCATGTTTAA TAATTCTTCC CTCCCCTGCT TGTNTATTTT TTTCTCTTCA 180
CGGCTCTTCC TATACCTTAG GCCAGTCTCA AGCACTCACT GGAGACCCTT GGGCCTTGGG 240
CGACCATTGA GTCCTAGTCT CCCTTGTTTG TGCCCCTGTA GGAGGTAGGT CCTTTTNTCC 300
TCCGGCCTAG TAGGGGACCT TGGGTAACAT TN 332

SEQ ID NO:7174
SEQUENCE LENGTH:127
SEQUENCE TYPE:nucleic acid
TOPOLOGY:linear
CLONE:HUMGS08412
SEQUENCE DESCRIPTION:
GATCTGCTTT GCAGAGGAAA TAACTTGTTT TTTCTCATGT TTCATCTTCT TTTTATGTAA 60

ATATGTAATA CTTTCCTATA TTGCCCTTTG AAATNTTTGG ATAAAAGATG ATGTTTTAAG 120
TTCCAAA 127

SEQ ID NO:7175
SEQUENCE LENGTH:144
SEQUENCE TYPE:nucleic acid
TOPOLOGY:linear
CLONE:HUMGS08413
SEQUENCE DESCRIPTION:
GATCTGCCCG CCTCAGCCTC CCATTGTTGA CCATTTTTAT ATGTTTATTT ACCTATGTGG 60
TTTTCTTTTC TCATAAAATG CTTGTTCATA TATTTNCCCC ATTTCTACTT ATCTNGTTCT 120
TATTAAATNG CCAAAGTGCA TAAA 144

SEQ ID NO:7176
SEQUENCE LENGTH:347
SEQUENCE TYPE:nucleic acid
TOPOLOGY:linear
CLONE:HUMGS08414
SEQUENCE DESCRIPTION:
GATCATCTCT AAAATTAAAA AAATTAATAT TAAATGTTGG ACCAAGGATT TCAACCATGC 60
AGTATGTTGC TTCTGGGGTC TGCTGTCCTA TTCCAGCCCT AGTGAATAGT TAATGCCTTG 120
AACTATTCCT ATCACCACAG GTTGCCAGGC CACCAAATAA CGAAGCCAAT CCAGGATTTG 180
TCCATACAGA AAATGTGTGC GCTCCCAGCT AAATCTCAGT TAAGGAGGTT GTTCTATATA 240
CAGCCAAGTA GCAGAAATAT TCATAAATGT CATTTGCAAA TATAATAAAC ANTGTAATGA 300
ATGGGTATTT CCTTTCTTGT TAATTCTATG TGCTCAATTA AAAGGNN 347

SEQ ID NO:7177
SEQUENCE LENGTH:213
SEQUENCE TYPE:nucleic acid
TOPOLOGY:linear
CLONE:HUMGS08415
SEQUENCE DESCRIPTION:
GATCATACTA ATAATTTAAT ATTTGAATTG TATGGAAGTA CAATTCAGTA TCATTTTACA 60
TATGGTATAT TGTGATGCTG TATCATATTT TATGTTACGG TTTATAAGAA AAGCTCCTAG 120
GTATAAAATG CTACATAGCA GGAACTTGGT TTTTCAATGT TATTATTTCC TACTGTTTTT 180
GACGTAACGG CAATAAAATT TGTTTGAACC AAA 213

SEQ ID NO:7178
SEQUENCE LENGTH:161
SEQUENCE TYPE:nucleic acid
TOPOLOGY:linear
CLONE:HUMGS08416
SEQUENCE DESCRIPTION:
GATCTTAAGA AGAGAATAGA ATCTTTAATT GACCGGGACT ACATGGAAAG AGATAAAGAA 60
AATCCAAACC AGTACAACTA TATTGCATAG AATGTTGGCC TTGCAGCATT TGGTGTCATA 120

TGCAGTAGCC AGTGGATAAA CTAACCTGTT GATTCAATAA A 161

SEQ ID NO:7179
SEQUENCE LENGTH:69
SEQUENCE TYPE:nucleic acid
TOPOLOGY:linear
CLONE:HUMGS08417
SEQUENCE DESCRIPTION:
GATCATGTGC AATATTTCTT ACTGTGCCGA GAAGCCACAA TGAGCGAGAT TAAAGCTGTT 60
TAACACAAA 69

SEQ ID NO:7180
SEQUENCE LENGTH:348
SEQUENCE TYPE:nucleic acid
TOPOLOGY:linear
CLONE:HUMGS08418
SEQUENCE DESCRIPTION:
GATCTCTGTT CCTTTTGGCT CTAATATGCT ACAACTGTAG GCCAATTATC ACTTTACCAA 60
TTAAGAGTTA GGCCAGATAA GTGAAATTTA ACTTAAGGGC ACACAGCTAA TAAGTAATAG 120
GCCTAAACTG GATTTCCTTA TTCCAAATCC TGTCTTTTCC CCACTATTCC ATTAGACCCC 180
ACAAATGTTA GTTGTGTGTG TGTGTGTGTG TGTTTTTAAT CACTGTAACC GGGTGCATTT 240
TTTTAAGGCA AAATTTCTCC CTTATCTACT GTGATGACTT CAGAAGATAC ANTGGTCCCA 300
GGGGCCAAGT AGAAAGCATT TTTAAAGNTT AATCTGAATT AAGCTGAN 348

SEQ ID NO:7181
SEQUENCE LENGTH:132
SEQUENCE TYPE:nucleic acid
TOPOLOGY:linear
CLONE:HUMGS08419
SEQUENCE DESCRIPTION:
GATCCACTTC TGTAATTAAG TACNTGGTTG TCTCGTGATG CGTCTACAGT TATTTATAGT 60
TACAACCATT ACCNGNCACT GTAGATACAC ATATAAAAAT AGNTGTTNGG TATCCATAGT 120
TNCGCTTGTT CN 132

SEQ ID NO:7182
SEQUENCE LENGTH:85
SEQUENCE TYPE:nucleic acid
TOPOLOGY:linear
CLONE:HUMGS08420
SEQUENCE DESCRIPTION:
GATCTCATCA CTGCACTCTA GCCTAGGTGA CGGAGCGAGA CCCTGCCGCT AAATAAATAA 60
ATAAATAAAT AACATAGTAG TGAAA 85

SEQ ID NO:7183
SEQUENCE LENGTH:224

SEQUENCE TYPE:nucleic acid
TOPOLOGY:linear
CLONE:HUMGS08421
SEQUENCE DESCRIPTION:
GATCATGGAC TGGTGCCTTT GCATTCAGAA GGAGAGCTGT CAGCGTANAC CGAATTCAAG 60
ACCAAGGCGT GCTACCTGAG CTGACAGCTT TTTGAAAGCC GAGCTGTTTC TGAACCATGT 120
ACATACATGT TCTGAAACTT TCTCATCATT TTATGAGTAC TGTTCATTGA GAGATGACAA 180
TGAAGATTAG ATGAAATTGG AAATAAACCA ACATTGTTTA CAAA 224

SEQ ID NO:7184
SEQUENCE LENGTH:38
SEQUENCE TYPE:nucleic acid
TOPOLOGY:linear
CLONE:HUMGS08422
SEQUENCE DESCRIPTION:
GATCTCTGCA GTATTAAAAA GATGTACTCC CAATTAAA 38

SEQ ID NO:7185
SEQUENCE LENGTH:67
SEQUENCE TYPE:nucleic acid
TOPOLOGY:linear
CLONE:HUMGS08423
SEQUENCE DESCRIPTION:
GATCTTTGGC AGTGCCAGCA GGTTAAATTT TGACCTGCTC AATTAAAAAT AAACACTGGC 60
GTTTAAA 67

SEQ ID NO:7186
SEQUENCE LENGTH:117
SEQUENCE TYPE:nucleic acid
TOPOLOGY:linear
CLONE:HUMGS08424
SEQUENCE DESCRIPTION:
GATCTTCAAA GAAGAGAATG TAATTTATAC CATGAATATG TTATTTTTTC ATGTTGGAGC 60
ACTGAAATAC TTTCAGTTTG TTTTCTTATT GTTTAAATAA AATTTTTAAT TCTTAAA 117

SEQ ID NO:7187
SEQUENCE LENGTH:231
SEQUENCE TYPE:nucleic acid
TOPOLOGY:linear
CLONE:HUMGS08425
SEQUENCE DESCRIPTION:
GATCTTTGTT ATTTTAGGGG GGAAATCACA TGGTATCTAG CATGGGACAA AGCAGCAAAA 60
TAAACCAAAA GGACAATCTC TATTTTATAT AGAAAATAAA CATACTTTNC TGCATCCTGT 120
GTACTTTAAG TATATCTATA TTTNTNANGG GTTCATACTG TGTTGANTTT NCCTNATGAN 180
ATAGTCACTT CCCCAGTGTA TTTTAATGCA AATGCATATN CTATAANNTA N 231

SEQ ID NO:7188
SEQUENCE LENGTH:197
SEQUENCE TYPE:nucleic acid
TOPOLOGY:linear
CLONE:HUMGS08426
SEQUENCE DESCRIPTION:
GATCTCTGTT CTAAGAAAAG GAGTGTGCCT TGCATTTAAA AGGAAATATT GGTTTCTAGG 60
GAAGGGAGGA GGCTAAATAA TTGATACGGA ATTTTCCTCT TTTGTCTTCT TTTTTCTCAC 120
TTAAGAATCC GATACTGGAA GACTGATTTA GAAAAGTTTT TAACATGACA TTAAATGTGA 180
AATTTTAAAA ATTGAAA 197

SEQ ID NO:7189
SEQUENCE LENGTH:115
SEQUENCE TYPE:nucleic acid
TOPOLOGY:linear
CLONE:HUMGS08427
SEQUENCE DESCRIPTION:
GATCAGGAGA CAGGGAGGCC CCGCACATCA CACAGATAAA GTCAGACAAT TGTAATTAAT 60
ACTTTTGCTG CCTCAAGTTG TTTTTTAAAT AAAGTACTTT GAAATGCATG AGAAA 115

SEQ ID NO:7190
SEQUENCE LENGTH:329
SEQUENCE TYPE:nucleic acid
TOPOLOGY:linear
CLONE:HUMGS08428
SEQUENCE DESCRIPTION:
GATCCTTGTG GGCATTATTC TAACTGATAC GTAGACACTT ACTTGGAAAN NNNNGGACAT 60
TATATTAAAT GAGTGCTATC TGTGAAATTG GTTATATTAG GTGGCTTGAC TAATGTTTTT 120
CCTATAATTG TATATGGACT GCATTTTTAA AAAAAACCGC ATTTGCCTTT ATGCTAGATT 180
GTAAAAAATT ATATTAGAAT GCATAAGACA TGTTTTNCCN TCATATGCTA GACTTTNCCT 240
AGCATTTCGT ATTTCTGTGT TGTCAGTGTG TGATTTTNAA ACCGGAATTT GGTTTAAAAA 300
AAATCTGGTG GTAATATATG TGAGAANTN 329

SEQ ID NO:7191
SEQUENCE LENGTH:353
SEQUENCE TYPE:nucleic acid
TOPOLOGY:linear
CLONE:HUMGS08429
SEQUENCE DESCRIPTION:
GATCTTCATT CCAAATTTTA CTGCATGGAA ATGACTAATA ATGTGCATAA AAAGTTAACT 60
ATAAGGATGC TCACTGCAGG GTGGCTTATA TTAACACAAA TTCACAAACA ACTCCACTGT 120
TCCAGCACAG CAGTGTTTGG TAAATCATGA CATGTTTCTA TGCTCAATTA CTATCCAACT 180
TTTGAGAATT AGGTTGGAGA TATGTAATTN CCAATATGAA AATAAATTTC CTATATNCTA 240
TTTAGTAAAA NTAATTCATA AATNTCCATT TTTNCAAACA CTTCATGAAT TTNTTCCAAA 300

ATACANATGT TATCTATCCA TTTNGGAATT AGGANTGGTT NNNATCATTT TGN 353

SEQ ID NO:7192
SEQUENCE LENGTH:352
SEQUENCE TYPE:nucleic acid
TOPOLOGY:linear
CLONE:HUMGS08430
SEQUENCE DESCRIPTION:
GATCTCAGTT TTTCCATCAA AATTATAATA TGCTCATGAA AATAATATTA ATTTGCCTTC 60
CCTTTGCAAA CACCGGCAGT TGAAAGGAAA AGGACGGGGA ATGTGANNNN AAAGAGACCG 120
CCTGGAATAA ATGTCCCCCT ATGATTCTTT AAGGCAGTGG TTCTCGAGCT TGAATTTTCA 180
TTAGGAAATT CTGTGAGGNG NTTGTAACCA GATTTCTGGG TCTGCCACAT GCACCTATCT 240
NTNGCTGAAT TGCTTTAATA GAATAATGAG AGCAAGTTTG TCTAACTAAT ACCAACCTGN 300
CANCTTGAAT ANCAATAAAA TGCAATTTGG TACATAAATT ATAATGCTGC NN 352

SEQ ID NO:7193
SEQUENCE LENGTH:348
SEQUENCE TYPE:nucleic acid
TOPOLOGY:linear
CLONE:HUMGS08431
SEQUENCE DESCRIPTION:
GATCCTGGAG AGGAGAAATC CTGGCCTCTG CTCATGCGCA GGTCACAACC GGGCTGGAAG 60
TGCGGTTNAG CTCTCTGCCA CTTTCTTCAA TGTAAAAAGT AGGCATCAAA GATACGACTT 120
CTACTATATA CATTTNTNTG TCCCTGAGAA CAGAGAAACG TAACCCTGTA GGGGACTGGT 180
TACAGAGGAC CTCCTGGAGC ATCTGCCCCA TTCCTCCAAG TCCAAGCACA GCCATGCTGA 240
GTGGAAGCNA GGCCACCATC TGAGTGGATT GGTCCCAAGT NTGTAATGAG GAAAGTGGCC 300
AGGTGCCGGC ACAGTGCCTC TCCCAGAGCC TCAAGAAGGG CCATTNGN 348

SEQ ID NO:7194
SEQUENCE LENGTH:349
SEQUENCE TYPE:nucleic acid
TOPOLOGY:linear
CLONE:HUMGS08432
SEQUENCE DESCRIPTION:
GATCACAACT CTCAGTCTAC CTGGAACACC CCACCCAACA TGCCTGCTGC CTGGGGACAT 60
GCCAGTTTCA TCAGCTCTCC GCCCTACCTC ACAAGCACCC GAAGCTTGTN TCCAATGTCT 120
GGACTTTTTG GTTCCATCTG GGCCCCGCAA AGCGATGTGT ATGAAAATTG CTGCCCCATC 180
AACCCCACCA CGGAACATTC GACCCACATG GAAAACCAAG CGGGTCGTGT GCAAGGAATA 240
CTACCCGGGG TTCAACCCGT TTCGCGCCTA TATGAACCTG GACATATGGA CTACCACAGC 300
GAATAGGAAT GCAAATTTCC CACTGTCTAG AGACTCGAGT TACTTGTNN 349

SEQ ID NO:7195
SEQUENCE LENGTH:342
SEQUENCE TYPE:nucleic acid
TOPOLOGY:linear

CLONE:HUMGS08434
SEQUENCE DESCRIPTION:
GATCTGCCTG CCTCGGCCTC CCAAAGTGCT GGGATTACAG GCCTGAGCCA CTGCGCCCAG 60
ACCAAAAGTT TTTTTAAATG GATGTTTTAC AGATAGAACT AAATTCTTCA TTTACCTCAC 120
TTAATTTCTG AAAACACTGT GTCCTCCAAC TGCTCTGTAT TCCCAAAGAT TAAAATTAGC 180
CTTGGGGAGC AGAGCTGGTT CCTCGTAAAC CCCTGTGAGT GCCCAGACCC TTTGGAGGAG 240
GTTCTCAGTG GGCTTTTGCC TGCTCCCACA GATGTAACCC ACCCTTCCTG CTGCTCCTAA 300
GACCACCATG AGGAAGGCTG GGAATTGGCT CTTGAATTAA NN 342

SEQ ID NO:7196
SEQUENCE LENGTH:364
SEQUENCE TYPE:nucleic acid
TOPOLOGY:linear
CLONE:HUMGS08435
SEQUENCE DESCRIPTION:
GATCTGCATT TNNAGGTTCC GAGCCTGACC CCTGGAACTG ACCAGCGCTC GATTGTNAGC 60
CTTGGCCTGG GGTTTTAACC TTGCCAGTGA AGTTTCGGTT TTAAAGTGAT TAAATGTCAC 120
TTCCTCATCA GTTTCACTTC TGGAGGTTTT CTTATCCTAC TCCCTGGTGC CAGGGACGTA 180
CCTGGGAGTT TGAATCAGGC CCATTTGAGC GTGGCAGCCG TGTTGGGTGA AGGTCCGGGG 240
CTCGGTGAGG CACTGGGGGG GTTTTCGGGA GGAAAATGAA AATGCTTCTA GAATGAGTGA 300
ACCACATCAT AGCTCTCACT GTTTTTTCAA TAGCTACTTT TTTTAGCAGA CANCAGAGCC 360
ACAN 364

SEQ ID NO:7197
SEQUENCE LENGTH:30
SEQUENCE TYPE:nucleic acid
TOPOLOGY:linear
CLONE:HUMGS08436
SEQUENCE DESCRIPTION:
GATCTCAAAA TAAATTCAGA AAGAAATAAA 30

SEQ ID NO:7198
SEQUENCE LENGTH:196
SEQUENCE TYPE:nucleic acid
TOPOLOGY:linear
CLONE:HUMGS08437
SEQUENCE DESCRIPTION:
GATCTGCCTA GCCAACTTTC TTAACCCAAG CCCAAACTGA AAGTCTAAAA GATGTATTTC 60
TGGGGAATTA CCCCACCTCA ACATTCTCAG GTTTNCCAGT GTNCTCTGTC TGTNTCAGTA 120
TTTTAAAAAC ACTAACTCAA TAATNCTCAA AAATCAAAGT CTGCTTTNAT NATCTGGTAA 180
TAAAATAATA GGCAAA 196

SEQ ID NO:7199
SEQUENCE LENGTH:102
SEQUENCE TYPE:nucleic acid

TOPOLOGY:linear
CLONE:HUMGS08438
SEQUENCE DESCRIPTION:
GATCCGCCCA TCTCCGTCTC CCAAAGTGCT GGGAATNACA GGCGTGACAN CCAAACCCGG 60
CCTGATTAAA GTTAAATAAA TACTAGTTCC CTTCTCGTCA AA 102

SEQ ID NO:7200
SEQUENCE LENGTH:341
SEQUENCE TYPE:nucleic acid
TOPOLOGY:linear
CLONE:HUMGS08439
SEQUENCE DESCRIPTION:
GATCAGTGCA TTTAGACATC CAGAAGTTAC TTCCACTATT GAGGAACTAT GTAGACATGA 60
ATTTAAGCTT GTACATGTGC CAATCTTGTT AATGTCACTT GACCACTGAT TCACATAGAC 120
AGACATTAAC GTCTGATGCC TCAGGTTGAT GGCGAACTTN CTTCCTAGTG TCTTAACATT 180
TATGANCTTA TGTTTGACTT GAGGGGCATT TCAGCAAAGC TGTGNAGGAA AAGTAGGTTC 240
TGGNCTGATG TCTGCTTTGT NCCATAGACA TTTTGGAGCA TAAGGNAGTA TCTNCTTCTG 300
CTATATGTNC AGGGCTTGTC CCCAAATNAA TTCCTGATTT N 341

SEQ ID NO:7201
SEQUENCE LENGTH:110
SEQUENCE TYPE:nucleic acid
TOPOLOGY:linear
CLONE:HUMGS08440
SEQUENCE DESCRIPTION:
GATCTNCTAC AGTTCCTNCA AGCCTAGACA ACCTAAGAAA CTTTACTATC AGCAGCTTAA 60
GATGAAAATC ACAGACTTTA AGAACAGGCG AAGTTTTAAN TGTATATNNN 110

SEQ ID NO:7202
SEQUENCE LENGTH:45
SEQUENCE TYPE:nucleic acid
TOPOLOGY:linear
CLONE:HUMGS08441
SEQUENCE DESCRIPTION:
GATCCAAAGA AATAATCCAA AATAAAAAGT TATATCCAAG ATAAA 45

SEQ ID NO:7203
SEQUENCE LENGTH:202
SEQUENCE TYPE:nucleic acid
TOPOLOGY:linear
CLONE:HUMGS08442
SEQUENCE DESCRIPTION:
GATCCACAGT GGAGTTTTAG TAATTATATT TTNTTGATTT CTTCATTTTG TTTTCTGGTA 60
TAAAAGTAGA GATAATGTGT AGTCACTTCT AATTTAGTGA AACCAATTGT AATAATTNTG 120
GAAATATTTT GTCTTTAAGT GTAAATATTT AAAAATTTGG CATACCCTAA TGTNAATAAT 180

AAAAAGAACT ATTTGCAGAA AA 202

SEQ ID NO:7204
SEQUENCE LENGTH:337
SEQUENCE TYPE:nucleic acid
TOPOLOGY:linear
CLONE:HUMGS08443
SEQUENCE DESCRIPTION:
GATCGGAAAA CTCTGATTGA ACCATTCACT TTATTAGCCA GTAAACTTTC TCAACCCCAG 60
AGCAAATCAA GCTTTCCTTT GCCTGGGAGG AAAANCATAG GGAACTCTAA TTCTGAGAAC 120
TAAAAATCAG TAATTTCCAC AATTGTATGT TGAATAGTGA TTGCCTTTAN GNGNCTGTGN 180
NCATGGAGTA ATATTACTAT TTAAAATAGG CCATTTGTAT CTACCTTTGG TCCTTAGGAA 240
AATTCCTAAG GAAGTCAATT AATGCACTTT NAGATGTTAA AAGTATTTGG GCTAAGGTTA 300
TTATTGCCTG ATATGGAAAT AATTATNNTT CTGNTTN 337

SEQ ID NO:7205
SEQUENCE LENGTH:351
SEQUENCE TYPE:nucleic acid
TOPOLOGY:linear
CLONE:HUMGS08444
SEQUENCE DESCRIPTION:
GATCAGTGAC AATTTACAGG AATGTAGCAG CGATGGAATT CCCTGGAACA GTTTTTTGTT 60
TTTNTTTTTG TTTTTGTTTT TGTGGGGGGG GGCAACTAAA CAAACACAAA GTATTCTGTG 120
TCAGGTATTG GGCTGGACAG GGAAGTTGTG TGTTGGGGTG GTTTTTTTCT CTATTTTTTT 180
GTTTGTTTCT TGTTTTTNAA TAATGTTTAC AATCTGCCTC AATCACTTTG TCTTTTANAA 240
NGNTTCCACC TCCAGTCCTN TCTCCTCCCC CCTACTCAGG CCCTTGAGGC TATAGGGGGT 300
TNCTTNGAGG ACCTCANCAA AATCCCAATC CAAGTNAACC TTTTGCACAN N 351

SEQ ID NO:7206
SEQUENCE LENGTH:273
SEQUENCE TYPE:nucleic acid
TOPOLOGY:linear
CLONE:HUMGS08445
SEQUENCE DESCRIPTION:
GATCTATTTC TTTAAAAGAT ATTTGTGCAG AAACTGCATG TAACTCTAAG TTTTACTCCT 60
AACATACATA TGTTTGGGGA AGTATTCTAT TCTATACTTG CCAATGTGGA GAACAAAATA 120
GTTTTTTTAA GAATGAAGAA GTATATATAT CCATTCTGTA TTTTACGTGC AGCAGAATTA 180
TCTTCCGTAG GATTTTTTTG TGTATTCACA AGGTGATATT TGTATTGTAA AACAATAATG 240
GTGAAGGAAA TAAAAAGGCT TTTAAAATTT AAA 273

SEQ ID NO:7207
SEQUENCE LENGTH:328
SEQUENCE TYPE:nucleic acid
TOPOLOGY:linear
CLONE:HUMGS08446

SEQUENCE DESCRIPTION:
GATCAGATTT TTAAAAAATG GATTTCTCAT ATAAATAATA TTATCAAAAA AGGATTTCTC 60
ATATAAATAA TATTATCAAA AAAGCTGATT TTAAAAGTTT CTCCCAAAGT CTTATTCTAG 120
TAATTATAGA GACCTAGGTA ATGAGTGGCA GATATATCTG CCTTTCAGAT ATGCCGTAAT 180
GTGAAAAATA ACACAGTCAT GTGATATTCT TNATTAACTA AAACTGTGTT GTTTTTATTT 240
TGGAGTAGTT CTCATAATTC ATTGGTAGGG AACTATCCAG TATTTATATT CCTATGTATG 300
TNTATCAGNT TAATTTTGAG GCTTGGAN 328

SEQ ID NO:7208
SEQUENCE LENGTH:145
SEQUENCE TYPE:nucleic acid
TOPOLOGY:linear
CLONE:HUMGS08447
SEQUENCE DESCRIPTION:
GATCTGTACA TACAGTGTTA AGAATGTAGA TATTAAAATT GTTATATTTA GCTGTTACAT 60
AATATTAAGA CTCAGAGTTA AGTAATTTCA CTGAAATTGA TTGCTTTTTG TGTCTTGGAG 120
TCAAAATAAA TAACTGAAAT CTAAA 145

SEQ ID NO:7209
SEQUENCE LENGTH:227
SEQUENCE TYPE:nucleic acid
TOPOLOGY:linear
CLONE:HUMGS08448
SEQUENCE DESCRIPTION:
GATCTGTTNA CATAAGCACT AGTCACAGGA CTACTCGTGA ATTTCATCAA TTTATTCTAT 60
TGTGTTTATT CATGATATCA CCTTAGTAAT TTTTGAAAAA AAAAANGTAC CACACTTGGC 120
CAAANGCTCA TCAGGCTGAT TGTTTANCTG TNGCTGATTG CTTATCTTTA TATTGTATGA 180
CCCNGGGGNT NGNTTTGAAN GTTATATTAG GTTTGAAATG ANTTATN 227

SEQ ID NO:7210
SEQUENCE LENGTH:333
SEQUENCE TYPE:nucleic acid
TOPOLOGY:linear
CLONE:HUMGS08449
SEQUENCE DESCRIPTION:
GATCTTTGAA CTCTGCAATA TGAAGCATAT ATGTGAGTGC TTAGCTTTCA CTCTCTTTCT 60
GTGTGTGTGT GTGTGTATAC AGATACTAAG ACACAGATAC ATTCACATAT ATACATCTAA 120
ATATATATAC AGTACAGTCC ATATTCAGAT TTCTTCAAGG TTATGGGTCT ATTGTTTAAA 180
TTCGGAATCC AGTCAAGGAC AACACATTAA AATTGATTGT TTTGCCTCTA CAGTGTGTTT 240
TTTTGGTTTT CATGACATTG ACTTTGATTT GAATAGGTCA GACCATTTCA TGTACTTTAT 300
ANAGGCACTT TGTAGATTTG TCTGATGATT TGN 333

SEQ ID NO:7211
SEQUENCE LENGTH:151
SEQUENCE TYPE:nucleic acid

TOPOLOGY:linear
CLONE:HUMGS08450
SEQUENCE DESCRIPTION:
GATCAAGAGA GTTCGATTGG CCAAAGAACA GGAATCCCGG GCAGATTGTA TCAGTGAGTT 60
TATAGAATGG CAGTATAATG ACAATAACAC TTCTCATTGT TTTAACAAAA TGACCAATCT 120
GAAATTAGAG GATGCAAGGA GAGAAAAGAA A 151

SEQ ID NO:7212
SEQUENCE LENGTH:275
SEQUENCE TYPE:nucleic acid
TOPOLOGY:linear
CLONE:HUMGS08451
SEQUENCE DESCRIPTION:
GATCTGACAG ACGTCAATAC CTGCAGGCGT CTACAGAGAG GAAATTTGAT AAAAATATTT 60
TAGTCACTGA CTACACTACC ATTATCTGAA GCATTCAGAT AAGACTTAGC AGAGAATGTC 120
AGATTTTGTT TTCAATTATA ATTTTAGAAA TGCAAAATCT TATTTTTTCT AAGTTGCTTA 180
TTGTCTATAT AAATGTTTTA TATCATTTAA AAACATGCAG AATAATACAC CTGCAGAATA 240
AAAGAGCTGA AATTAAAATA TCTTTTTCAA TNAAA 275

SEQ ID NO:7213
SEQUENCE LENGTH:103
SEQUENCE TYPE:nucleic acid
TOPOLOGY:linear
CLONE:HUMGS08452
SEQUENCE DESCRIPTION:
GATCACTTGA GTCCAGGAGT TTGGTGTTAC AGTGAGCTAT GATGGCACCA CTGCACTCCA 60
GCCTGGGCCA CAGAGTAAGA ACATGTCTTT AAGAAAAAAA AAN 103

SEQ ID NO:7214
SEQUENCE LENGTH:144
SEQUENCE TYPE:nucleic acid
TOPOLOGY:linear
CLONE:HUMGS08453
SEQUENCE DESCRIPTION:
GATCTGTAGA CATGAAGGCA AAGCTCTTGT ATTTTTTTTT CATCCAAACA CCTCAATTTA 60
TTTTATAAAT TCGTTCATTT TTCCTGTTAT GTTTTATATA ATATATGGAC TAAACAAAAT 120
AAAATAACAG TGCAAAAGAG GAAA 144

SEQ ID NO:7215
SEQUENCE LENGTH:164
SEQUENCE TYPE:nucleic acid
TOPOLOGY:linear
CLONE:HUMGS08454
SEQUENCE DESCRIPTION:
GATCAGAAGA GGATGACAAT GGCAACAAGT GTTTGGAAGT TCCAAGGTGT GTTCAAAGAG 60

GCTTGCCTTG AGGGAGGGCT GGAATCTGTC TTCCCTGACT CGGCTCCTCA GGTCTTTAGC 120
CTCCACCTTG TCTAAGCTTT GGTCTATAAA GTGCGCTACA GAAA 164

SEQ ID NO:7216
SEQUENCE LENGTH:332
SEQUENCE TYPE:nucleic acid
TOPOLOGY:linear
CLONE:HUMGS08455
SEQUENCE DESCRIPTION:
GATCTGAAGT CCCCCCAACC CTCTCCAAGT AAAGTAGTTT ATTAGAAGCT CTTAAGTCTT 60
TAATAGACCT GCATATTTCC TTCCCTTATG ATTTCCTATA AAATATAGTT TATGGTGTTA 120
TATTTTTAAC TGAAATACTG TACATATGTA AATAACTCTT GACAGGAAGA AAATATATTA 180
ATGTAGTATT TGCCCCCTAT CAGTGAGCTG AACAAATACA TCATTTAAAT CTATGCTGCA 240
CTTTGAGTTG CTACAAATAT GGTTCGNTTT GTTTATTTTT GAAAAATGTG ATAAAGAAAT 300
CTAAAGANTG NNNAANNTAA NANNNNNNNG NN 332

SEQ ID NO:7217
SEQUENCE LENGTH:277
SEQUENCE TYPE:nucleic acid
TOPOLOGY:linear
CLONE:HUMGS08456
SEQUENCE DESCRIPTION:
GATCTGTACC ATCCTGTTGC TGCTGTATGC GTTCCATTGA TGGGACATCT TCAGGGACTC 60
TTGACAGCCA CCGCTTTCAC ACCCTGGTCT GGAATAAGGA AACATCGGAG GGAGAAGTTG 120
ACTGTCTTGA TAATTAGCCT GACCAGCAGG ATGAATGCAA GACTGACAGT GATGGACTCT 180
GTGACATGGT CAGGTTGAGC TGAAGCCACA GTTTCTCTGT GCTGTGTTTT CTAACACATT 240
TTTCTGTTTT TAATTAAAAA AANNNNNNNA GGGTAAA 277

SEQ ID NO:7218
SEQUENCE LENGTH:198
SEQUENCE TYPE:nucleic acid
TOPOLOGY:linear
CLONE:HUMGS08457
SEQUENCE DESCRIPTION:
GATCTTAAAC TCCTGACCTC AAGCAGTCTA CCCCCGTTGG CCTCCCAAAG TGCTGGGATT 60
ACAGGTGTGA GCCACTGCAC CCAGCCAAGT TTATGTATTT TNATTCTTTT TCATGAATGT 120
GATTTGTTAA ATNAGTGATT AAATATAATT TTNATATATT TGTATGACTT TNCTGTAAAT 180
AAAATTTAAA ATCAGAAA 198

SEQ ID NO:7219
SEQUENCE LENGTH:409
SEQUENCE TYPE:nucleic acid
TOPOLOGY:linear
CLONE:HUMGS08458
SEQUENCE DESCRIPTION:

GATCTGCATC TNACGCCCAC TGCACACCGT TCCTCTCCAT CTGCCCTTCC CTCCCAGGCC 60
CCCGGTCGGG GGAGCAGGTG TGGCAAGTGT TAGGAACTCC CTTCTGGTGA CAGCTGGTCT 120
TCCTAGAGTG TTGCTGGTAA CTATCGCCTC TTGGTCTTCT GACATCATTG CCAGGAACGG 180
GGCACTTTTT CGTCTTGTAG TTTGGCCCTC GGGTTTCCTC ACTAGGTATT GTGTAACTCC 240
CTCAAAAAAA GGTTTATGAA ATGCTGAACC TCAGGTTTCA TAGACGTCTT TGTACACTAA 300
AAATTCTGCA GCAGGAATAT TTTTAAACAT TCGCAGTTTT TTGTAAGCTA TATTTTTTGG 360
ATATTTAAAT TGCTATNTNA AAANTTTTAA ANTNATTTTT GCTAATNNN 409

SEQ ID NO:7220
SEQUENCE LENGTH:87
SEQUENCE TYPE:nucleic acid
TOPOLOGY:linear
CLONE:HUMGS08459
SEQUENCE DESCRIPTION:
GATCTCCTGT TCCGTTAGAA ATACGGTTAT GTTGACATTA TGGTTACTGT GCTTGTGGTT 60
TTGCAATAAA CATGTATTGA AAGCAAA 87

SEQ ID NO:7221
SEQUENCE LENGTH:130
SEQUENCE TYPE:nucleic acid
TOPOLOGY:linear
CLONE:HUMGS08460
SEQUENCE DESCRIPTION:
GATCAGATAA AATAACTCCT GACAGAAACT GTNNGGAAAC CAGCTGAATG TTTGACCTGA 60
TGACTGATGT TGTATGGTTT ATGTTAAATG TATATTCTTT TAATCAATGA ATAAAGCATT 120
AAAAATGAAA 130

SEQ ID NO:7222
SEQUENCE LENGTH:251
SEQUENCE TYPE:nucleic acid
TOPOLOGY:linear
CLONE:HUMGS08461
SEQUENCE DESCRIPTION:
GATCAGAAAC ATTTACAATC TATTCTCTTT GAAGCCTGGC ACCTGGAGGC GTCATCTGTA 60
TGAGANNNNN GGACTCCACA ACCTTTTATC ATAACCCAGA CATTCCTTTC TATTGATAAT 120
AACCAATTGC CAATCAGAAA AATTAAAAAT CTACTTATAA CCCAGAAGCA CTACCCCGCA 180
ACCCTTGCTT GCTTCAAATT GTTCCAACTT TCTGGACCGA ACCAATGTAT AGCTTAAATG 240
TATTTGATTG N 251

SEQ ID NO:7223
SEQUENCE LENGTH:189
SEQUENCE TYPE:nucleic acid
TOPOLOGY:linear
CLONE:HUMGS08462
SEQUENCE DESCRIPTION:

GATCGGTGCC ATGGCAAGCA CACCCTCAGT CATCATCCTC ATCAGGACAG NCAGCCNNGA 60
ACTCAAGTCT TACGCTTTGG GAGTTCTTTT TCTCNTCCTT CGTTTGTTGG GCTTCATCCC 120
NCCACCCCTC ATCTTCGGGG CTGGCATCGA CTCCACCTGC CTGTTCTGGA GCACGNNNTG 180
TGGGNGAGN 189

SEQ ID NO:7224
SEQUENCE LENGTH:97
SEQUENCE TYPE:nucleic acid
TOPOLOGY:linear
CLONE:HUMGS08463
SEQUENCE DESCRIPTION:
GATCTGATTT GGCCTGGGAC CAGTGTTCAA GTTGGTTTGG TCTTTATTAA AAATCACAAT 60
ATTCCGAAAA CAAAAAAACC TAGGAGATAA ATGTAAA 97

SEQ ID NO:7225
SEQUENCE LENGTH:255
SEQUENCE TYPE:nucleic acid
TOPOLOGY:linear
CLONE:HUMGS08464
SEQUENCE DESCRIPTION:
GATCTGAAAT NAGAGGTTTT GGTTTACCCT ATCTATGGTA TGTCTTAAAA ATCAACGAAG 60
ATGTCCTTGT CTTTTTTNAA TTTNCCGAGT GTGTTGCAGT TCCACAGCTC ACTGTTAGGT 120
GGCACATACC CCAAACTGAA AACCTGACCT CGTAGGGCAT GAGTCAAAAG ACAGGTAGGC 180
ACAGGACAGG GCAGTGGTGA CACTACAGCT TTCAGGGTTC CCAGCCTGTC AAGAATGAGC 240
ATGTCTTAGC AGGGN 255

SEQ ID NO:7226
SEQUENCE LENGTH:63
SEQUENCE TYPE:nucleic acid
TOPOLOGY:linear
CLONE:HUMGS08465
SEQUENCE DESCRIPTION:
GATCAAATTT GGAAAACCTA ACATTGAGAC AATACTATAT TAAAGTATCT CTGGTACCCG 60
AAA 63

SEQ ID NO:7227
SEQUENCE LENGTH:55
SEQUENCE TYPE:nucleic acid
TOPOLOGY:linear
CLONE:HUMGS08466
SEQUENCE DESCRIPTION:
GATCCAGCAA TCTCACTGAG TATCTAACCA AAGGAAAATA AATCACTATA CCAAA 55

SEQ ID NO:7228
SEQUENCE LENGTH:220

SEQUENCE TYPE:nucleic acid
TOPOLOGY:linear
CLONE:HUMGS08467
SEQUENCE DESCRIPTION:
GATCCAAGAA CCCTCTCTTG GGGTCTGAAT CATACCCCTT TCGGGTTACA GCCTATCACA 60
GCATTTTTAA TCCTATGCAC TTAACACTTC TGTATAGAAA AAGAACATCA CACTAGAATT 120
TTGATGACAA TAGATGACAA TATGATGAAT NTTATTATAT GAAGTGTTAA ACTTATTAGA 180
GCAGATATGG TACTAAAAAT AAAATCCCAC CTNCNCTAAA 220

SEQ ID NO:7229
SEQUENCE LENGTH:114
SEQUENCE TYPE:nucleic acid
TOPOLOGY:linear
CLONE:HUMGS08468
SEQUENCE DESCRIPTION:
GATCGCGCCA TTCCACTCCA GCCTACACGA CAGAGTGAGA CTCCATCTCG AAACAAAAAA 60
AAGACTTGAT TTTTATTTTC ACTATCTTTA GCATAAATTG TTCTAAGAGT GAAA 114

SEQ ID NO:7230
SEQUENCE LENGTH:274
SEQUENCE TYPE:nucleic acid
TOPOLOGY:linear
CLONE:HUMGS08469
SEQUENCE DESCRIPTION:
GATCTTATAC AGAGAAGTAT TTTATTAAAA ATTCAAAAGG GAAGACTTTT ATGTGCTCAT 60
TTTGTAATTT TTAATTTTAA ATATCTTTAC ATTGTCTGCC AATTAAAGTG TTTTAAACTT 120
GCATTGGAAT GGNCTCTGAA TGTATTTNTN TGGTGTNACG TTATCCGTAG ATTTCTAGCA 180
TGANGTTAGC CTCACGATGC TGTGCAAAGG ATTTTNAAAT ATGAGAGTCA CTGANAGAGT 240
TTAANCATCT GTCCATGTTA AATGCTCTAT GGGN 274

SEQ ID NO:7231
SEQUENCE LENGTH:135
SEQUENCE TYPE:nucleic acid
TOPOLOGY:linear
CLONE:HUMGS08470
SEQUENCE DESCRIPTION:
GATCCTTTGT TCACGGCTCT CCTTAATGAC TGAGTGAACA GTTCCTATCT GTATATTTGA 60
CTAAACCTTT TCCTAAGCTA TCTCTCATGG TTCCTATGTT TTTTTATCAT AATTAAAAGC 120
AAAACCATCT GGAAA 135

SEQ ID NO:7232
SEQUENCE LENGTH:238
SEQUENCE TYPE:nucleic acid
TOPOLOGY:linear
CLONE:HUMGS08471

SEQUENCE DESCRIPTION:
GATCGAGCGG AAAATTCCAG AACTGTACAA GCCAATATTC AGAGTTGAGA GTCAAAAGAG 60
AACATTGTGC TGTCTGTCAG CATATGTATA TCAGCTACAA AATATATTCA ACTTTGACTT 120
CTTTTGACAA AGGACTTTAG GAAAAAGAGG AACAAAGACA TTATTTGAGA ATTAAATTAT 180
ATATTTTAAN TATGACTGTG ACCTTGACTG ATAATAAAGA TGTAATAAGA NTTGCAAA 238

SEQ ID NO:7233
SEQUENCE LENGTH:71
SEQUENCE TYPE:nucleic acid
TOPOLOGY:linear
CLONE:HUMGS08472
SEQUENCE DESCRIPTION:
GATCAGTTCA GCCAAGCAAC TGACAAATCA AAAACCCACT TGTCAGTTCN GTAAAATAAT 60
TTGGTCAGAA A 71

SEQ ID NO:7234
SEQUENCE LENGTH:347
SEQUENCE TYPE:nucleic acid
TOPOLOGY:linear
CLONE:HUMGS08473
SEQUENCE DESCRIPTION:
GATCTAGAGG GCTTCGAGTG AAGCCAATCA TGTTGAACGA ACACTGGTTC AGTGTAGATG 60
AANTAGAGTG AGGCGTATGC AGTCATAAAA TGAAGGGTGG ACAGGAGATT ACATTTGAAG 120
AAAGGATAAG CACTCAGACT TGGTCTCTTT TAAATTAAAA TATTTCTTTT CTCATGTGCA 180
AATGTATATC AAAGACTTGA ACAGGTATTC TCTTGAAATT TGAGGATTTT TAGTTTTTAA 240
TGCTGNCTTT GCTTANNATT AANNATGNGT NGCCTATTGG TTCCCAAATT GTNTGGGGNC 300
CCTAANNGTT NCCCCTNGAG GGGTNCATTT TTGAAGGNGG GTTANGN 347

SEQ ID NO:7235
SEQUENCE LENGTH:93
SEQUENCE TYPE:nucleic acid
TOPOLOGY:linear
CLONE:HUMGS08474
SEQUENCE DESCRIPTION:
GATCTTGTGT TTTGCTATCC TTTTTACTGT AAAATGTAAA TATTTTAAGG GATATTTTGA 60
TTCTAAATAT GATAAAATAA TTTCTCACCT AAA 93

SEQ ID NO:7236
SEQUENCE LENGTH:417
SEQUENCE TYPE:nucleic acid
TOPOLOGY:linear
CLONE:HUMGS08475
SEQUENCE DESCRIPTION:
GATCCCAATT CAACTTATCC AGAATAATTC TCTCCATTCT CAAAATTTCA NATGAGGAAC 60
ATNNNNAGAA ACTGTCTCTT GCCAATACCT TTAGTACTGG AATTCTCTCA TCTTCCTCAA 120

CAGTCTTGGC AGCTAGGAAA AAACAGCTGA TTGCAATACA ACTCAAGTAT TTTGGATGAG 180
CCTTTACGGT AGCTAAAAAC CCATCCAAAA GACTGCTAGC CAGAGCAAAT GTTTCTGGGT 240
AAAGGTTGAA TTGGTACTTG AGTTTGGCCA GCCATTGANT TACTTCATCT CTCTGGGATG 300
GNGAACATTC TGATTTGAAG GCATTTTCCG CACATTCACT TTCCACATCT GTGNCTCCCT 360
AGTGATTGCC TTTTCCACCA GGAANGCCAA TCTCTGGTTT TCCAAAGGCC CTNGGAN 417

SEQ ID NO:7237
SEQUENCE LENGTH:80
SEQUENCE TYPE:nucleic acid
TOPOLOGY:linear
CLONE:HUMGS08476
SEQUENCE DESCRIPTION:
GATCAGACCC TCCTGTGGGC AGGGTTCTTA GTGGATGAGT TACTGGGAAG AATCAGAGAT 60
AAAAACCAAC CCAAATCAAA 80

SEQ ID NO:7238
SEQUENCE LENGTH:409
SEQUENCE TYPE:nucleic acid
TOPOLOGY:linear
CLONE:HUMGS08477
SEQUENCE DESCRIPTION:
GATCTGCCTG CCTCGGCCTC CCAAAGTGCT GGGATTACAG GCGTGAGCAT TTGCGCCTGG 60
CCAGAAAAAT ACCTTTAAAA AGCTGGGCCT GTTGGTGTAC CTAAAGGATA ATACAGATGT 120
TCCTCTCAAC TTAGCATGGG GGTTGTGTCT CAATAAACCC ATTTTTAAGT TGAAANCGTT 180
ATGTGGAAAA TGCATTTTAT TATACCTAAC CTACTAGACC TCGTAGTTTA GCCTCCCTTA 240
CCCTACAGGT GCTCAGAACA CTTTNGNAGG CCCTACAGTT NGGGCAGTCA TCTAACACAA 300
AACCTATTTT ATAGGTAAAA GTNGTTTGAA TATCTCAAAG NTAATTTATT TGAATGGTTN 360
CCACTTGAAT NGCGGANTCN ANGTGTTTNN NAACCNGTNT GGTNNANNN 409

SEQ ID NO:7239
SEQUENCE LENGTH:123
SEQUENCE TYPE:nucleic acid
TOPOLOGY:linear
CLONE:HUMGS08478
SEQUENCE DESCRIPTION:
GATCTCATAG ACTTAGTACA AAAAAAGGTG AAATGTCTTG ATATTTTTGT ATATTGATTA 60
TTGAAATAAC CTTTTATGTT GGGCTAAATA CAAATATGTT ATTAAAATNA ATTTAACAAC 120
AAA 123

SEQ ID NO:7240
SEQUENCE LENGTH:265
SEQUENCE TYPE:nucleic acid
TOPOLOGY:linear
CLONE:HUMGS08479
SEQUENCE DESCRIPTION:

GATCTGAGGG GGAGAGGGAC GGGTCGGGCC AGCTGGTCAG GAAGGGCCTT GAGGGCCTGC 60
AGCCAAGAGT AGCAGTTTTC TCCCCCGTCT TGTTGTTCTT GGTCCCTGGA CCACAGGGCA 120
CACAGGCCTG GACACCATAA GGCTGGTGGG CTTTCAGAAT TGTGTTAGGG NNNNNNGAGT 180
GGCAGGTTCC TGAATCTCGG TCAATATAGT AACCAGCAGG ACAAGAGGTT CAGGAGGAGC 240
CCACATCAGA GGCTTCTAGG GCAAN 265

SEQ ID NO:7241
SEQUENCE LENGTH:265
SEQUENCE TYPE:nucleic acid
TOPOLOGY:linear
CLONE:HUMGS08480
SEQUENCE DESCRIPTION:
GATCTCTTGA ATTGCCATCT GTCATCTAAG TCTAAGGCTT TATGCTTAAT GANTTGTGGT 60
GTACTAGCTG GACTAGCTTC AGGAATGTCA GTGTGTTCTA CCATTAAGGT CAGATTGTCT 120
ACTACATCGA GATGGTGGTT GTAGTTACAG CTACTTTTAG AAACATGTCT ATTTTTTAAA 180
GACATAACAC ATGAATGAAG AAATTCAGAA TTTGGAAAAA AGGTCCGTAA TGCCTGACAA 240
AGAAAAATGT TCTCCTGAGG GTCTN 265

SEQ ID NO:7242
SEQUENCE LENGTH:138
SEQUENCE TYPE:nucleic acid
TOPOLOGY:linear
CLONE:HUMGS08481
SEQUENCE DESCRIPTION:
GATCCCAGCA CCTTCACATC TTCAGCAACC CAGNNGNTCA AATTTGACCT GGTGTTATTT 60
TAGCCCCAAA TTTATGACAT TACACAATAT TAAAATGTAA ATGTTTCTTC ACCCAAACTA 120
CTTCTAGATA TNCTNGNN 138

SEQ ID NO:7243
SEQUENCE LENGTH:273
SEQUENCE TYPE:nucleic acid
TOPOLOGY:linear
CLONE:HUMGS08482
SEQUENCE DESCRIPTION:
GATCCTTTGA GAGATGGTTT GACTTTCCCC GAGCAAAGAG CCTGCGTTGA AAAGCGGGGG 60
TGGAATTCAG TCCTCACAGA TAATGAGGGG ACAANACCTA ATTGANCCGN GTATTGCCGG 120
GAAGGAAAAG GCAACGGGCC AAGCCTTTGA CAGGGTGCGA AACTGACTTT NATCATCGTT 180
ATAGTCTTTA AATCCTGGGA AACGAGTTGG CAACCCCAAA ATAAAGAAGT GTAATGACGT 240
CTGATGACTT CACCCAAATA CAGACCATTC CAN 273

SEQ ID NO:7244
SEQUENCE LENGTH:132
SEQUENCE TYPE:nucleic acid
TOPOLOGY:linear
CLONE:HUMGS08483

SEQUENCE DESCRIPTION:
GATCTATAAA ACAGACAGAG ATAAGTACAA CAGAATATCT CGGGAATGGA CTCAGAAGTA 60
TGCCATGTGA TGCTACCTTA AAGTCAGAAT AACCTGCATT ATAGCTGGAA TAAACTTTAA 120
ATTACTGTTA AA 132

SEQ ID NO:7245
SEQUENCE LENGTH:321
SEQUENCE TYPE:nucleic acid
TOPOLOGY:linear
CLONE:HUMGS08484
SEQUENCE DESCRIPTION:
GATCCGCCTG CTTCAGCCTC CCAAAGTGCT GGGATTAGAG GCGTGAGCAT TCATGTCCAG 60
CCAGCACTGT GCTGTTTTTG ACATGCATGT TCTCATTCAN TGCCATGCGG CAGATTCTAG 120
GATTATNATT ATNCTCACTT TACAGATGAG GGAGTGAGAC TTAAAGGGGT TAGGTAATTT 180
GCCCACATCC CCCAACTAGC AAATGGTAGA GCCAGGATTG GAATTTGTTC AGGCTCTTAA 240
CCATTGCACT CTTTCAAAAT GTTAAAAATA ATGTTGTGTC TCATTTTAAA AAATTAAATA 300
AAANCTCTTT GGACCAAGAA A 321

SEQ ID NO:7246
SEQUENCE LENGTH:108
SEQUENCE TYPE:nucleic acid
TOPOLOGY:linear
CLONE:HUMGS08485
SEQUENCE DESCRIPTION:
GATCTCCCCA TTTGGAGACA GAGCCATTTG GATATTTNCC CCTTNAACTT CTCCANNNCC 60
TGAAGCGTTC CTTCCCTGGA GGAACTCTTT GGTTGCAGGG CTAAACTN 108

SEQ ID NO:7247
SEQUENCE LENGTH:139
SEQUENCE TYPE:nucleic acid
TOPOLOGY:linear
CLONE:HUMGS08486
SEQUENCE DESCRIPTION:
GATCTCAGCT CAAATATTCC TTATGCAGAG ATGCCTTCCC CTGATTCCCT AAACTAGACT 60
CGGACCCCTC TGTTCTGCAT CTCTGTGGCA TATCATCTGC CCCTTTTGTA AAACTTATAA 120
ACTTGTGGTT ACTTGTAAA 139

SEQ ID NO:7248
SEQUENCE LENGTH:244
SEQUENCE TYPE:nucleic acid
TOPOLOGY:linear
CLONE:HUMGS08487
SEQUENCE DESCRIPTION:
GATCATGTGA GCTCAGGAGT TCGAGACCAG CCTGGGCAAC ACAGCAAGAC CCTGTCTCTA 60
TTTTATTCTA TGTTTTAAAA TTTAAAAATT AAAAAGAAAA AAGAATAACA TTTTATAAGA 120

AAATATTTTA TGAGAGACTT ATAAAAGAGA CATTAAGAAT ATGAAGTAAT ATATTNCGTA 180
TTCCTTATCT CAATAATGCT GTAGGGAAAA ANCAATTGAA ATAAAAGCAG TAAATGAGTG 240
GAAA 244

SEQ ID NO:7249
SEQUENCE LENGTH:155
SEQUENCE TYPE:nucleic acid
TOPOLOGY:linear
CLONE:HUMGS08488
SEQUENCE DESCRIPTION:
GATCTAACTT GCAGTGTATT TTCTAGGCTG GAAAGTGGAA AATNAAATAT ATTATAATCT 60
TAGGTTACAT AAAGTTTCTA AAGTTTCAAA GAGTCTTGAT ACAAAATCAG TTTATATTCT 120
GAAAATATTT ATAATAAAGT ATTCTAATTT CTAAA 155

SEQ ID NO:7250
SEQUENCE LENGTH:96
SEQUENCE TYPE:nucleic acid
TOPOLOGY:linear
CLONE:HUMGS08489
SEQUENCE DESCRIPTION:
GATCGCACTG GAAGATTTTA TAAAAGAATT TTTGTGGGTC GGGGGGACAG TAAACTTCCT 60
GGGCCACGTG GGTCCTTCAG GAGTTTTTCA GGCAAA 96

SEQ ID NO:7251
SEQUENCE LENGTH:118
SEQUENCE TYPE:nucleic acid
TOPOLOGY:linear
CLONE:HUMGS08490
SEQUENCE DESCRIPTION:
GATCTNATAT TGAACCCTAT GCCAAATAGC GTGGCATGAG GCCAGAGAAC AGTGCTTNCC 60
TTCCCCGNCT CCACCCAAAT TGTCCCTTGC TCTNGATATT ATGCCAGTNC CATATTTN 118

SEQ ID NO:7252
SEQUENCE LENGTH:391
SEQUENCE TYPE:nucleic acid
TOPOLOGY:linear
CLONE:HUMGS08491
SEQUENCE DESCRIPTION:
GATCTGGAAA CTATTTGGNT TTTNTTTTCA ACTTTTCATT TGGATGTTTG GCGTTGCACA 60
CACACATCCA CCGGTGGAAG AGACGCCCGG TGAAAACACC TGTTTGCTTT CTAAGCCAGT 120
GAGGTTGAGG TGAGAGGTTT GCCAGAGTTT GTNTACCTCT GGGTATCCCT TTGTNTGGGA 180
TAAAAAAAAT CAAACCAGAA GGCGGGATGG AATGGATGCA CCGCAAATAA TGCATTTCCT 240
GAGTTTNCTT GTTAAAAAAA ANTTTTTTTA AGTAAGAAAA AAAAAGGTAA TANCATGGCC 300
AATTTGTTAC ATAAAATGNC TTTCTGTGTA TANNTTATTC CTAAAANATC CTGTTTATAT 360
AANNNTTCAG TAGNTGGAGA AACTTTCCAT N 391

SEQ ID NO:7253
SEQUENCE LENGTH:385
SEQUENCE TYPE:nucleic acid
TOPOLOGY:linear
CLONE:HUMGS08492
SEQUENCE DESCRIPTION:
GATCATGAAT CTCTAATAAA TTTAAATCTC TTAAACCAGT AGGTGCTTAA TATTTTTTAA 60
TTTGATTAAT GCCCATTTAA ATCTCATGGG TTCTATTAAA AATATATATA TATAGGGCCC 120
CAATCCATTG CCATCAAATT GCCCTTGGAC TTTTCCAAGG TATATTATGG GGTTTTATGC 180
AAAATTCCAA GCTACCATGT AACTTTTTTN AACCATTTAA CAAGGAGGGG GAACTGTTTC 240
CTACCTTCTT TACATGTTGT GCATTGTTGT GGTCCAGAAA TGCCAAACCT TTTTAAAGAT 300
GGTGCAACTT TGAGTCCTTG GCTTGACTAT ACAGGNCTTG AACTTCATGG CATATCAACT 360
TTGNCATATC TGCAGGTGNG CTGTN 385

SEQ ID NO:7254
SEQUENCE LENGTH:414
SEQUENCE TYPE:nucleic acid
TOPOLOGY:linear
CLONE:HUMGS08493
SEQUENCE DESCRIPTION:
GATCACCATT TNTTTCCAGG CTTTCTGCCT CCNAGATGTG GCACCATAGT GCGGTGCCCT 60
GTGGCTTCAC CGCCCTACTT CCACCTCCGC CCAGCCTGTA ATGTTTATAT AAGCAGCCTC 120
AAGGACCAAG AACCATCTGC GAAAGGACAC ACACAGGAAA TTCATAAAAG AAATCTGANT 180
GGNTAAAACC ATGAAAAAAA GTATGCTTCA TTAGTAATTA AAGAAAGGCA AATAGAGCTG 240
GAAGCNTTTT NCCNTTNGCA AACCATAACA GAAAANNNTA NGACCCAATA TTGGCAAAGA 300
GACTACTGAA AANCCATTCC CATACATTGC GTGTGGGNGT ATACATCGGT GCAGGCTTCC 360
TGGATGACAG TTGGGTGATA TGTGTCATGT GGCCTAANAG CCTCCATGTC ANTN 414

SEQ ID NO:7255
SEQUENCE LENGTH:207
SEQUENCE TYPE:nucleic acid
TOPOLOGY:linear
CLONE:HUMGS08494
SEQUENCE DESCRIPTION:
GATCCTGGTT TTGTGATTGA CAATATTTCA TGCTCTGTAG TGAGAGGAGA TTTCCGAAAC 60
TCTGTTGCTA GTTCATTCTG CAGCAAATAA TTATNATGTC TGATGTTGAC TCATTGCAGT 120
TTAAACATTT CTNCTTGTTT GCATCTTAGT AGAAATGGAA AATAACCACT CCTGGTCGTC 180
TTTTCATAAA TTTNCATATT TTTGAAA 207

SEQ ID NO:7256
SEQUENCE LENGTH:412
SEQUENCE TYPE:nucleic acid
TOPOLOGY:linear
CLONE:HUMGS08495

SEQUENCE DESCRIPTION:
GATCAAGAGT ACACTTCCTG GCAGTGATTA AGGAGTGTGT ATCTAACAGA AAAAATATAT 60
ATACCCTGTG AACCCGAATA TGGAATTCAG ATTGTTTCTG CCCTCAGTAT CATACTTAAA 120
AAACAAGCAT ACAAACAAAC ATAAGGGAAC AAACAGCAAC CATAACAAAA ACAAACCTTT 180
AAAGGTGGGT TTTTGCTGTG ATAAATGAAT ACGGTACTCT GAAGGAGAAA AAAGTTTCTC 240
AAATGAGCTT AAACTGCAAG TGATTTAAAA NTTAGAGAAT ATAATTCTTA ANGCTATTGA 300
AAGTTTCAAC CAGAAANCCT CANGTGAATT TTGTATGTAA ATGGCATCTT GNNTGTAAGT 360
TCTGTGNTTC TTTAGGCAAC CAATTAGCTG GAACCTTGGT TTTGTGTGGT TN 412

SEQ ID NO:7257
SEQUENCE LENGTH:406
SEQUENCE TYPE:nucleic acid
TOPOLOGY:linear
CLONE:HUMGS08496
SEQUENCE DESCRIPTION:
GATCTGCAAA TCAAGCTACA TGTATTTGTG TATAAGACCC TGTGTTCAGG ACTGGGTGAC 60
TTTTCTAAGA AATATGGGGT TTAGAATGGG GTTGACTGTA TTTNTAAACC TAATTCTGGA 120
GAGAAGATTG TATTTNTAAC AGTTTTTTGG GTTTGGCTTC CTTCTCACAT TTCTTTAGCT 180
TTGAATTTTT ACTAAATAAA TTTCCTCCTG ATTAATTTTT TTTTTCCCCA TCTGGGAATT 240
TGAAATCTCG GTGCTTACTG TNACACCAAT TTGTCCAANG GGTTGAAATC ACTTTAATGC 300
CAGACCATGG TAATTTGCAG CCATTTCAGG CAGGTGGTGG NCCTTTTTNA TACCATCGTT 360
ACGGGGTCCC NTTAAATATT CTGGGGGGTG ATGTAAATNT AACGGN 406

SEQ ID NO:7258
SEQUENCE LENGTH:116
SEQUENCE TYPE:nucleic acid
TOPOLOGY:linear
CLONE:HUMGS08497
SEQUENCE DESCRIPTION:
GATCTCCATT TCCGCCAGCT GCCTGTAGCC ACGTGGCATC CTGCCTGTGG TCTGGGTGAG 60
ATTTACTGTG ACCAGATGTA GAATAAATGT GTCTCATCCT GCATTTTTTT TCTAAA 116

SEQ ID NO:7259
SEQUENCE LENGTH:435
SEQUENCE TYPE:nucleic acid
TOPOLOGY:linear
CLONE:HUMGS08498
SEQUENCE DESCRIPTION:
GATCACTTTT TAGTTAGTTT GAAACCTGCT TTTAGTTTAT ATGTAGTATT TCTACATATT 60
GTTACTTAAA TTGAACTAAC AATTTACTTT TNAATTTTNC TTCTTCCTTT TAAAAAAAGA 120
GTATATAATC TGACAATGTT AGTACATCTT TTAGAAGGTC ACTATAAAGT TGCTTTCCCT 180
ATTTATTTGG AAAGGATTAT TTCCTTATNA TTTTAAAAAA TCTTTTAATT NNTGTCTTNT 240
CAAGTAATTT TATCATTGTA CCAAGCCTAA GGATGAGTGG CAATTTAAAA GNCACAAGGT 300
GTGCATCTTC TATCTGCAAA TACTCCAAAC AGAANTTATT CCAGTTTGTT GATACTTTGA 360
GTGGCCCAGG GAAAATGTGT ATGGTTTTAG TGTNCTGAAT TGNCATTCCA AAGGGTGCGG 420

AAGGTTNCTG TTAAA 435

SEQ ID NO:7260
SEQUENCE LENGTH:62
SEQUENCE TYPE:nucleic acid
TOPOLOGY:linear
CLONE:HUMGS08499
SEQUENCE DESCRIPTION:
GATCAAAAAA GGAATGGATA TACAAAGTGT TTTGTGAAAT AAAAGCTCCC TAAAATGGTA 60
AA 62

SEQ ID NO:7261
SEQUENCE LENGTH:389
SEQUENCE TYPE:nucleic acid
TOPOLOGY:linear
CLONE:HUMGS08500
SEQUENCE DESCRIPTION:
GATCGCACCA CTGCACTCCA GCCTGGGCGA CAGAACGAGA CCCTGTCTCC AAAGGAAAAA 60
CAAAAAAGAA GAATAAAATA ATTTGGATGA AAATCATGTT TATTTAAATA GTAATGTCAT 120
GAGACTATTA AAGATGTGCC AGAGTTTCAA TGAAAATCAT TAAAGTAGGA CAGCTAAGAA 180
ATTAATATTA ATATCAAANT TATTGATANT CTTAAATTAT TGATTATTCC TTAACGCACT 240
CCATTCTCCT TTTACATTTT ATCATGTTTC TTTTGAATAT ATGANTTGGC AAAGGCCTTG 300
ATGAAACTGA GTACTAAGAT TTGGTCCAGN GTATGTCAGG ANGNCACCTC AGATTGCCAT 360
TTTAAATAAN GTTGTNCATG ACCAATAAA 389

SEQ ID NO:7262
SEQUENCE LENGTH:302
SEQUENCE TYPE:nucleic acid
TOPOLOGY:linear
CLONE:HUMGS08501
SEQUENCE DESCRIPTION:
GATCCCGTGC TACATCTCCT CCCCGGACGA GTGCCTCTGG ATGGACTGGG TCACAGAGAA 60
GAACATCAAC GGGCACCAGG CCAAGTTCTT CGCCTGNATC AAGAGAAGTG ACGGCTCCTG 120
TGCGTGGTAC CGCGGCGCGN CGCCNCCCAA GCAGGAGTTT CTCGACATCG AGGACCCATA 180
AGCAGGCCTC CAACGCCCCT GTGGCCAACT GCAAAAAAAG CCTCCAAGGG TTTCGACTGG 240
TCCAGCTCTG ACATCCCTTC CTGGAAACAG CATGAATAAA ACACTCATCC CATGGGTCCA 300
AA 302

SEQ ID NO:7263
SEQUENCE LENGTH:297
SEQUENCE TYPE:nucleic acid
TOPOLOGY:linear
CLONE:HUMGS08502
SEQUENCE DESCRIPTION:
GATCGGAAGG TGGTGAATCT TTCCAGAGCA GCTGAAAATN TCAGCATGGA GACAGTAAGA 60

AAAGAGACGG GGTGTGGATA AGACTCTGCC ACCGTGTCAC ACTAGCATAG GAGGCTGCAC 120
GTTCATTTGT TGTTGTTTTT TTTTCCTTTG CCAACCTCCG TTCTATTTAT GTGCAAGCAG 180
TTTGGATTCA AGTTCTTGTA TCTGTCTGTT CTGGGACCTG GGGATTGTNA GGGTTCCCTC 240
ACAGCCAGCA CGACCCCCAG AAAGAGGCGT CCCACAATAA ACACGTCACC TGCTAAA 297

SEQ ID NO:7264
SEQUENCE LENGTH:116
SEQUENCE TYPE:nucleic acid
TOPOLOGY:linear
CLONE:HUMGS08503
SEQUENCE DESCRIPTION:
GATCAACAGT TCTATTACAG ATTAGAATGT TTCACTTCAC AGTTCTTTGT AAATCATGTC 60
AATTTTTATN ATAGNTGTCA ATTTGGGAAA ATCTGTATTA AAATTTTTAA ATGAAA 116

SEQ ID NO:7265
SEQUENCE LENGTH:442
SEQUENCE TYPE:nucleic acid
TOPOLOGY:linear
CLONE:HUMGS08504
SEQUENCE DESCRIPTION:
GATCCCTAAT TGGCCAGGCC TGACCCTCTT GGACCTTTCT TCTTTGCCGA CAACCACTGC 60
CCAGCAGCCT CTGGGACCTC GGGGTCCCAG GGAACCCAGT CCAGCCTCCT GGCTGTTGAC 120
TTCCCATTGC TCTTGGAGCC ACCAATCAAA GAGATTCAAA GAGATTCCTG CAGGCCAGAG 180
GCGGAACACA CCTTTATGGC TGGGGCTCTC CGTGGTGTTC TGGACCCAGC CCCTGGAGAC 240
ANCATTCACT TTTACTGCTT TGTAGTGACT CGTGCTCTCC AACCTGTCTT CCTGAAAAAC 300
CAAGGCCCNC TTCCCCCACC TCTTCCATGG GGTGAGACTT GAGCAGAACA GGGGCTTCNC 360
AAGTTGCCAG AAAGACTGTC TGGGTGAGAA GCATGGCAGA GTTTTTCCCA GGACAGGTGT 420
TGACCAGGGC TTNTTGTTTA TN 442

SEQ ID NO:7266
SEQUENCE LENGTH:148
SEQUENCE TYPE:nucleic acid
TOPOLOGY:linear
CLONE:HUMGS08505
SEQUENCE DESCRIPTION:
GATCTTCTGA CATTACTGCT GTCTGAGATT TGTATATGTG TAAATACAAG TTCCTTGATA 60
CCCTAAAACC TTGGATTAAA CAGAATGTGC ATTGTACATC TTTAAACAAA ATGTATATTA 120
ATTTATTAAA TCTAGTTGTC ACTTTAAA 148

SEQ ID NO:7267
SEQUENCE LENGTH:169
SEQUENCE TYPE:nucleic acid
TOPOLOGY:linear
CLONE:HUMGS08506
SEQUENCE DESCRIPTION:

GATCTTAATT CTGTGTCTTA ACAGCAGGAG ATGAAATTCC CACATGGGAG ATGGCCTCCC 60
TGTCACAGGA AGGAATANCA TTCTCCCTAT CAATNNNGAG AAATTGGAAC TGNGAGAATT 120
CTGTATAGAC CCTTGTTCAA GTAATTNTTC TAGTTTAAAN CNTCCCAAN 169

SEQ ID NO:7268
SEQUENCE LENGTH:89
SEQUENCE TYPE:nucleic acid
TOPOLOGY:linear
CLONE:HUMGS08507
SEQUENCE DESCRIPTION:
GATCAAAGAG TCTCATCAAA GCAAGTTCTT CCCTCCCTGG TCCCCTTTGG CTTTCTGAAA 60
TNATTAATTA AAAGAAAGGC CCTAGGAAA 89

SEQ ID NO:7269
SEQUENCE LENGTH:225
SEQUENCE TYPE:nucleic acid
TOPOLOGY:linear
CLONE:HUMGS08508
SEQUENCE DESCRIPTION:
GATCATTGNT TTATTTTGTA GTCCACCTTT CAATCACTTC ATGTATATAA AAAGGGACTT 60
TGAATGTAAT AGTCTTCATC TTATTTATTG AAAAGATGTA CTAAAGGAAT GACTATTTTT 120
AAAAAAANCT CTAGTATTTA TCAATATGAC TCTAAGGGAA AAATCAAGAA ANTTAGAGGA 180
AGGTTCAANT TAGTAGTAAG GAATAGGANC NNNCATATNA NCTNN 225

SEQ ID NO:7270
SEQUENCE LENGTH:259
SEQUENCE TYPE:nucleic acid
TOPOLOGY:linear
CLONE:HUMGS08509
SEQUENCE DESCRIPTION:
GATCTGTAGT GATGGAGTTC CTTCTGGTGT CAGCCCCACA GGAGGCTCCC AGGCCTCCCT 60
CACTTCCCAT ACCCAGTCTA GGAGCTCCTT CTGGCTCCCA AGCACCCAGA GCTTTCCTCC 120
GCCTTTNAGT TTTGGTTCCT CCACTGGAAT GTAGGCTCCT CACGGGCGAT GGCTGTCTTT 180
TCTTGACTTT GTATCTTCAC TGCCAAGCAA AAAGTCTGCC AAGTGGGAAT NTTTAATAAA 240
TATTCATTGA ATAATGAAA 259

SEQ ID NO:7271
SEQUENCE LENGTH:357
SEQUENCE TYPE:nucleic acid
TOPOLOGY:linear
CLONE:HUMGS08510
SEQUENCE DESCRIPTION:
GATCCCAGAA GGAATGCTGA CCCCTCGTCG TATGACCTGT GCATAGTCTC CAGAGCTTCA 60
AAGGCAACAC AAGCTCGCAA CTCTAAGATT TTNTNAAACC ACAAAAACCC TGGTTAGCCA 120
TCTCATGCTC AGCCTTATCA CTTCCCTCCC TTTAGAAACT CTCTCCCTGC TGTATATNAA 180

AGGGAGCAGG TGGAGAGTCA TTTNCCTTCG TCCTGCATGT CTCTAACATT AATAGAAGGC 240
ATGGCTCCTG CTGCAACCGC TGTGAATGCT GCTGAGAACC TCCCTCTATG GGGATGGCTA 300
TTTNATTTTT GAGANGGAAA AAAAAAGTCA TGTATATATN CACATAANGG CATANAN 357

SEQ ID NO:7272
SEQUENCE LENGTH:265
SEQUENCE TYPE:nucleic acid
TOPOLOGY:linear
CLONE:HUMGS08511
SEQUENCE DESCRIPTION:
GATCTAAATN AACAGCATTT TTTTCCTTAG CCTCTGTTTG CCACTCTGGG TATCTCTCCT 60
ATGGGCAAAG CCATTAGAAA TGCATAAAAC CTCGAGACAT GGTTTTTGGC AAAAACTCCA 120
TGACTTTAAA CTAGCTCTTT NACTACTGAC CTTTCACAGA GAAAAAATAT TTCCCTTGAA 180
AAAAACTGGG CTTGTNATTT TTNCCCTTGT AGCTTTAAGC AGAGACATAA GTGCCTTGCA 240
TTACACATAG TAAACTTTCT TTAAA 265

SEQ ID NO:7273
SEQUENCE LENGTH:164
SEQUENCE TYPE:nucleic acid
TOPOLOGY:linear
CLONE:HUMGS08512
SEQUENCE DESCRIPTION:
GATCTTCTCT TTTTTTGGGT TCATTTTGTT CTGGGTTTTG GTTTTCTTCA CAATCTTGAA 60
CATTTTACAG TAGAACTCAT CTAAAAATGG ATTTGGGGAT GGGGAAACAT GCACAAAATC 120
TTTTCATAAT TAAAAAGAGC CTTACTTTCT TTACATACCA CAAA 164

SEQ ID NO:7274
SEQUENCE LENGTH:243
SEQUENCE TYPE:nucleic acid
TOPOLOGY:linear
CLONE:HUMGS08513
SEQUENCE DESCRIPTION:
GATCCGTTCC GTGGTCGCCC TGCACAACCT CATCAACAAC AAGATTGCCA ACCGGGATGC 60
AGAGANGAAA GAAGGGCAGG AGAAAGAAGA GAGCAAAAAG GATAGGAAAG AGGACAAGGA 120
GAAAGATAAA GATAAGGAAA AGAGTGATGT AAAGAAAGAG GAGAAAAAGG AGAAANAGTA 180
AAACATGTAT TAANTAGCTT TTTTAATTTG TAANTTAANA TCTTACANAC TNGAANCNAN 240
NNN 243

SEQ ID NO:7275
SEQUENCE LENGTH:265
SEQUENCE TYPE:nucleic acid
TOPOLOGY:linear
CLONE:HUMGS08514
SEQUENCE DESCRIPTION:
GATCACAGGC CTTGAGAGAG NAGAATAAGA CACTCTTGGG GACAGAAAGT TAAAACACCG 60

TGTGTCCTCA CTTATATGTG GGCGCTAAAA AAGTTGATGT CATAGAAGTA TAAAGTAGAA 120
CAGAGGATAT GGGAAGGTGG GAAGGGTAGG GGGAAGGGAA GGATAGGGAG AGGTTTGTTA 180
AAGGATACAA AATTACCACT CGATAGGAGG AGTAAGTNCT AAGTNCTAGT ATTCTATACC 240
ACTGTAGGAT GACTATAGTT AACAN 265

SEQ ID NO:7276
SEQUENCE LENGTH:261
SEQUENCE TYPE:nucleic acid
TOPOLOGY:linear
CLONE:HUMGS08515
SEQUENCE DESCRIPTION:
GATCGCCCGC ATTCCGGAGC CTTCAAGTAA ACGGCGCGCC CCACGAGACC CCGGCTTCCT 60
TTCCCAAGCC TTCGGGCGTC TGTGTGCGCT CTGTGGATGC CAGGGCCGAC CAGAGGAGCC 120
TTTTTAAAGN ACATGTTTTT ATACAAAATA AGAACAAGGA TTTTAATTTT NTTAAGTATT 180
TATTTATGTA CTTTNATTTT ACACAGAAAC ACTGCCTTTT NATTTATATG TACTGTTTTA 240
TCTGGCCCCA GGTAGAAACT N 261

SEQ ID NO:7277
SEQUENCE LENGTH:260
SEQUENCE TYPE:nucleic acid
TOPOLOGY:linear
CLONE:HUMGS08516
SEQUENCE DESCRIPTION:
GATCCACATC TAGTGAAATG TCAGTGTCAA AATATTATAG ATTATAGCTA AAATCCAGAT 60
TAATACTCAT TTGGGGTTTT TAATAGTGGA ACTTCATAGT AATACAAAAA GCAGATTGTC 120
TTCCTGTCTC CGCTGCTCCC ACAGTAGGTA TTGAAACTGG TAAAANCAGT TTTTTGATAG 180
TGTGTGTATA TAAGANANNN TAGATACACA CATTCTTTTT CCTCAGTCAN CACATTGATT 240
GACCACTCTG GCAAAGATGN 260

SEQ ID NO:7278
SEQUENCE LENGTH:60
SEQUENCE TYPE:nucleic acid
TOPOLOGY:linear
CLONE:HUMGS08517
SEQUENCE DESCRIPTION:
GATCCCACTC CACCCCACCT CAACTTATTT AACTTCCTAA TTAAATCAGA CTGTTTGAAA 60

SEQ ID NO:7279
SEQUENCE LENGTH:156
SEQUENCE TYPE:nucleic acid
TOPOLOGY:linear
CLONE:HUMGS08518
SEQUENCE DESCRIPTION:
GATCTGGAGA CTTAAAAAGA AAACAAAAAC AAATGGCAAG TTTCACTTAA GGGTGGTTTG 60
CCCTTAAGAA GAAAGCTGTT GGGACAAAGA CACCGAGCCA TTATACCCAG AATAAAATAA 120

TACATTTATG CTGGATTTTA TTCAGACCAA ACTAAA 156

SEQ ID NO:7280
SEQUENCE LENGTH:329
SEQUENCE TYPE:nucleic acid
TOPOLOGY:linear
CLONE:HUMGS08519
SEQUENCE DESCRIPTION:
GATCATTGTT TAGAATATTT CCGCCACCTT AGAAAGTTTT GTCATGCCTT TTTTTTCAGT 60
CAGTCCCTCC CCTTCACTCA CTGCAAGACC ACCACTGTTG TGACTTTTAG CATCTAAATG 120
TAGCTTTGGA ATCATGTAGT ATGTACTCTC ATGTCTCTGA AATTCATCCA TGTTGTTGCA 180
TAATTCAGTA GTTCACTACT TTTTAATTTC TGATTAGTAT TTCATTGTAT GGGTGTACTG 240
TAATTTGTTT AACCATTCAC CTGCTGTTTA ATCTTTTGAG CTGTATGCAT TTGGCTATTA 300
TGAATAAAGC TGCTTTGAAC ATTCCTAAA 329

SEQ ID NO:7281
SEQUENCE LENGTH:270
SEQUENCE TYPE:nucleic acid
TOPOLOGY:linear
CLONE:HUMGS08520
SEQUENCE DESCRIPTION:
GATCAGAGAA GACCCCTGAG ATGAATCAGG GCCCTGCCCT GNCCACCAAC TCTTTTCTAG 60
CAATCATGTC CTGCCCAGGC TGCAGGCTTC ATTAGGCTCC TGCTGTCCTC CATGATGTGA 120
CTAGCACAAG GTGTATATAT GTTTTGTACC TCTGCCGATG GCTGTACATA GTGTATGAAA 180
GTTATTTAAG CCCCATGTTG TACATTTCTG TTCCTAGATT GGATGTGTGT GTTCTAAGAA 240
GTTGTCATAA ATAAAACCTG AATGACCAAA 270

SEQ ID NO:7282
SEQUENCE LENGTH:195
SEQUENCE TYPE:nucleic acid
TOPOLOGY:linear
CLONE:HUMGS08521
SEQUENCE DESCRIPTION:
GATCCAATTA TTAGAAAAGC ACTCTTAAGA GTGCAATGAA TGTGATTTGA AAACTTGAAA 60
GCAGCCTAGA TTTTACTATA TTTGTTATTG TTATCAATGG GGGTTTGTCA ACCTTAAAAA 120
TTATTTTTNA TGTAAAGATA CCTAGGAATT TCTAATAACT GTATTTGTTT TAATAAACTT 180
TGACTTTGCT GTAAA 195

SEQ ID NO:7283
SEQUENCE LENGTH:386
SEQUENCE TYPE:nucleic acid
TOPOLOGY:linear
CLONE:HUMGS08522
SEQUENCE DESCRIPTION:
GATCTACCTC AGTTAAACAG TTGGGTGCTA TTACTAAGTC TGTCAAATTA AATTGGAAAA 60

AGTAACCAAA CGGTGAGATA CAACTCCACA TGAAACTTGA AATTGTAATT TCCGTTTATT 120
TAATGATATT TTNATTTATT TGTGCCTTTT ATGTTGAACC CCAATGCATT GAAAAANTTC 180
AGTATGAAAC AGTACATATT TNATTTATAT TACAGGTGGG AGAAAAGTCC AATTGGTCAT 240
GGAATTTGAT AGACTTTTCC CCAGCCAACT GCTACAGTGT ATTATAATCC CGACTGCCCC 300
CCTGTGAAAA GANAAAAAAA ATTGTCCANG GGCAATTTTN CCATTTNCNA ACCANTANNG 360
NCCANNCTCC TTTAAAANGN GNGNNN 386

SEQ ID NO:7284
SEQUENCE LENGTH:187
SEQUENCE TYPE:nucleic acid
TOPOLOGY:linear
CLONE:HUMGS08523
SEQUENCE DESCRIPTION:
GATCAGTTAG TAGGCTTTCG NTGTCTTCTC TTTCAATACA TGTACATCTT TACTGTTTGA 60
AAAGTGTTAC AGCTGTCAAA GAATCTTCAT GGACCTGAAG ATAATTTNTT GTGAAGTTGA 120
ATGCAAGTNT ACTGTCATTC ATAGTGTTAA TATCAAANTA CCAGGAATNT TNACTTTTNC 180
NACCTNN 187

SEQ ID NO:7285
SEQUENCE LENGTH:113
SEQUENCE TYPE:nucleic acid
TOPOLOGY:linear
CLONE:HUMGS08524
SEQUENCE DESCRIPTION:
GATCTCAGAA ATTGTATCTN AGTTGGTATC AACCAAATGG AGTGACTTAG TGTACATGAA 60
AGCGTAAAGA GGATGTNTGG CATTTNNACT TTTGGCTTGT AAAGTACNGG CTN 113

SEQ ID NO:7286
SEQUENCE LENGTH:120
SEQUENCE TYPE:nucleic acid
TOPOLOGY:linear
CLONE:HUMGS08525
SEQUENCE DESCRIPTION:
GATCAAAGAG TAAGCCACAC ACGGATAATC AGTTACTAGG GATGGAGGTG TGAGGGTTCA 60
TTATATTATT CATTTTACTG TTGTATATGT TTGAAAATNT CTATAATAAA AAGCTTTAAA 120

SEQ ID NO:7287
SEQUENCE LENGTH:65
SEQUENCE TYPE:nucleic acid
TOPOLOGY:linear
CLONE:HUMGS08526
SEQUENCE DESCRIPTION:
GATCATGTGT ATGAATTGTT GGTGCTCTAA AGAACAGCAC AAATAAAATN ATTTTCAAAT 60
TTAAA 65

SEQ ID NO:7288
SEQUENCE LENGTH:110
SEQUENCE TYPE:nucleic acid
TOPOLOGY:linear
CLONE:HUMGS08527
SEQUENCE DESCRIPTION:
GATCCGNCCG CCTCAGCCTC CCAAAGTGCT GGGATTACAG GCATGANCCA CTGCACCCGG 60
CCCCATTCCT CACTTTAGCC TCAGGCAGAG AAGACNGCGC GATGGGGNNN 110

SEQ ID NO:7289
SEQUENCE LENGTH:296
SEQUENCE TYPE:nucleic acid
TOPOLOGY:linear
CLONE:HUMGS08528
SEQUENCE DESCRIPTION:
GATCTGCGAC ACTGTGGACT GAACACACTG AAGCTCTGAT GGGAAAACCT GGTGACTGAT 60
ATAGTTGTTC AGCAATAATT CATAGTCTGT GATGAAGAGT AGTGAATACA ACACACAACC 120
AGGCAGCCTT CTTGACTATA CTTTGCACAT GNTTTCTTTA GGAATTCACT CACACATTTA 180
AACCAGTTAG TGCCTTCTAG AAGAATGGCT TTCCTTTNTN CTACACAAAN TTTGAATTAT 240
ACAAGNNTCT AAATATAATA CCTTTNAATA AAAAGGNTAA TTNNGNCCCT CTGAAA 296

SEQ ID NO:7290
SEQUENCE LENGTH:381
SEQUENCE TYPE:nucleic acid
TOPOLOGY:linear
CLONE:HUMGS08529
SEQUENCE DESCRIPTION:
GATCAGGTCC AAGCAAAACG TCCAGAGTGC TACAAAATGT TGCGTTCTCA GTCCAAAAAG 60
AAGTGGAAAA GAATCTGAAG TCATGCTTGG ACAATGTTAA TGTTGTGTCC GTAGACACTG 120
CCAGAACACT ATTCAACCAA GTGATGGAAA AGGAGTTTGA AGACGGCATC ATTAACTGGG 180
GAAGAATTGT AACCATATTT GCATTTGANG GTATTCTCAT CAAGAAACTT CTACGACAGC 240
AAATTGCCCC GGATGTGGAT ACCTATANGG NGATTTCATA TTTTNTTGCG GAGTTCATAA 300
TGAATAACAC AGGAGGATGG GNTAAGGCAA AACGGAGGCT GGGTATGTGT GATGGGAAAA 360
CTTTCTTCAT TGGTNCTTTC N 381

SEQ ID NO:7291
SEQUENCE LENGTH:51
SEQUENCE TYPE:nucleic acid
TOPOLOGY:linear
CLONE:HUMGS08530
SEQUENCE DESCRIPTION:
GATCACCCCC GCCGACGGCC CCGGGCCCCG ACGGCCCGGA AGTTCCGCAA A 51

SEQ ID NO:7292
SEQUENCE LENGTH:347

SEQUENCE TYPE:nucleic acid
TOPOLOGY:linear
CLONE:HUMGS08531
SEQUENCE DESCRIPTION:
GATCATTCTG TTCTTTCAAG GAGAAATAAG CCTAAAAGAA GAAAAACAAA AAAAATTCTG 60
TATAAAACTG TAATCCTTTG TATTCATGTT TACAGTGCTA TTACTATAAT NCAAAATTAT 120
GTATGTGACT TAGAGTTATA TAATCATAAT TTATGTTTAT TTCAAATATC TAAGTTTATT 180
GCTTGGATTT CTAGTGAGAG CTGTTGAATT TGGTGATGTC AAATGTTTCT AGGGTTTTTT 240
TAGTTTGTTT TTATTGAAAA NTTTAATNAT TTATGCTATA GGTGATATTC TCTTTGAATA 300
AACCNATAAT AGAAAATAGC AGNCACCATA ANCATCTTTG TAAATNN 347

SEQ ID NO:7293
SEQUENCE LENGTH:122
SEQUENCE TYPE:nucleic acid
TOPOLOGY:linear
CLONE:HUMGS08532
SEQUENCE DESCRIPTION:
GATCCAGAAA CTTTTCCCAA GAAGCTTGGA CATGGATTGC TGGTAAACAC AAAAACTGCA 60
TGGAGGGCAG AGCAGCCGGT TGACTCCCCC TTGTCAATAT CTTAAAGTGG AAAAGCTTGA 120
AA 122

SEQ ID NO:7294
SEQUENCE LENGTH:138
SEQUENCE TYPE:nucleic acid
TOPOLOGY:linear
CLONE:HUMGS08533
SEQUENCE DESCRIPTION:
GATCATCTTT CCTGTTCCAG AGAAGTGGGC TGGATGTCTC CATCTCTGTC TCAACTTTAC 60
GTGTACTGAG CTGCAACTTC TTAACTTCCC TAACTNAAAG ANCGACTCTG ACAGAAACTN 120
TNTTTNCNCA ACAGTTTN 138

SEQ ID NO:7295
SEQUENCE LENGTH:211
SEQUENCE TYPE:nucleic acid
TOPOLOGY:linear
CLONE:HUMGS08534
SEQUENCE DESCRIPTION:
GATCCTTCCG GNGGAGTGTC GGAGAAGAGA GCTTGCCGAC GATGCCTTCC TGTGCAGAGC 60
TTGGGCATNT CCTTTACGCC AGGGTGAGGA AGACACCAGG ACAATGACAG CATCGGGTGT 120
TGTTCTCATC ACAGCGCCTC AGTTAGAGGA TGTTCCTCTT GGTGACCTCA TGTAATTAGC 180
TCATTCAATA AAGCACTTTC TTTATTTTAA A 211

SEQ ID NO:7296
SEQUENCE LENGTH:152
SEQUENCE TYPE:nucleic acid

TOPOLOGY:linear
CLONE:HUMGS08535
SEQUENCE DESCRIPTION:
GATCAACGGT ACTTGTTTTA AAGAAGCTTA TGTGGACTTT GTCTTTTCTC GTTGCCACAA 60
CCTCTTTTAA TGTGCCTATA GTCCACTATA GCATGCATAT CTCAAATTGC AATTCCTTGC 120
AATTCTGGAA TAAACGCTTT ATTTTGGAGA AA 152

SEQ ID NO:7297
SEQUENCE LENGTH:381
SEQUENCE TYPE:nucleic acid
TOPOLOGY:linear
CLONE:HUMGS08536
SEQUENCE DESCRIPTION:
GATCTGCTGA CTTATATAAA GCTTTTTGAT TCCTACTAAG CTTTAAGATT TAAAAAATGT 60
TCAATGTTGA AATTTCTGTG GGGCTCTATT TTTNCTTTGG CTTTCTGGTG AGAGAGTGAG 120
GAAGCATTCT TTCCTTCACT AAGTTTGTNT TTCTTGTCTT CTGGATAGAT TGATTTTAAG 180
AGACTAAGGG AATTTACAAA CTAAAGATTT TAGTCATCTG GTGGAAAAGG AGACTTTAAG 240
ATTGTTTAGG GCTGGGCGGG GTGACTCACA TCTGTAATCC CAGCACTTTG GGAGGCCAAG 300
GCAGGCAGAC CACTTGANGG AGTTCGGGNC CAGCGTGGCC ACCGTGGTGA ANCCCTGTNT 360
CTNCTAAAAA TNCAAAATTT N 381

SEQ ID NO:7298
SEQUENCE LENGTH:379
SEQUENCE TYPE:nucleic acid
TOPOLOGY:linear
CLONE:HUMGS08537
SEQUENCE DESCRIPTION:
GATCAACAGC CTCCTCTCTT GGGGACTCTC AAGAGCCTGT TTTCATCTAG AAGTAGTAGT 60
TTGATTCTGG TTTCCCTCCT ACAGTGTGTC CTCCGTCTCT TTGCAGCTCC GTCATTACCA 120
TAGGGGACTT GGTTTTAGAC TCTGATGAGG AAGAAAATGG CCAGGGGGAA GGAAAGGTGA 180
GTGGGAAGGA GCAGAAAGCT GGGAAAGGGG ATGGGTAGAA CAAGACTGAG AAATCCACAT 240
GCTTCAGANT TCAGAGGGTT CAGGGAATNG TTTCGGATAG TAGGCTCTCC CTGCTCCCTT 300
CTCTACAGGA ATCTNTGGAA AACTATCAGA AGACAAAGTT TGACACCTTG ATACCCACTC 360
TNTGTGAATA CCCTACCCN 379

SEQ ID NO:7299
SEQUENCE LENGTH:82
SEQUENCE TYPE:nucleic acid
TOPOLOGY:linear
CLONE:HUMGS08538
SEQUENCE DESCRIPTION:
GATCATTACA AACAAAAACT GTAATTTTNT TATATTTGAT TCAATGGAAT TTACCTAAAA 60
AATAAAGACT AAAAATGTGA AA 82

SEQ ID NO:7300

SEQUENCE LENGTH:225
SEQUENCE TYPE:nucleic acid
TOPOLOGY:linear
CLONE:HUMGS08539
SEQUENCE DESCRIPTION:
GATCCCTTTA TTGACAGCCA GTTTGAGGAC ACTGTCCCAG CATCTCTAAT GGAGCCTGAG 60
CCGGTGTGAG GACCAGGATG TCTTTTCCCA GCCCCAAGAG ACCTGTTGCT GCTTTCTTGT 120
AATNATGGGG CTCCCCAGAG TCTGCGTAAC AGTCTCCCAC TGGCTGGCTC ACCCACAGGT 180
GCCATGTGCA CACTCCTGGT TTTCAAACAA TTCTCTGGAT TTAAA 225

SEQ ID NO:7301
SEQUENCE LENGTH:216
SEQUENCE TYPE:nucleic acid
TOPOLOGY:linear
CLONE:HUMGS08540
SEQUENCE DESCRIPTION:
GATCATGCAT GCCAATCCCT GTTGCCGCAT GGAGCTCCTC AGCCCACTGA CCTCTCCGTG 60
CCTGGTGCAG GCCAGGCCCC CGTCTTCCGC CTGCCTCTGC TTCCCCGTCA TGCATGGTGG 120
TGGTGTTTCT ACGGTGTCTG GTTCTGTGCC CGTCTCTGAG ACAGTCTCTG TGTGGAATNT 180
GCCTTAAACT GAAGTAAATN TGGTTCTTTT AGTAAA 216

SEQ ID NO:7302
SEQUENCE LENGTH:374
SEQUENCE TYPE:nucleic acid
TOPOLOGY:linear
CLONE:HUMGS08541
SEQUENCE DESCRIPTION:
GATCAATTCA TGCAAGATAT AATCATATTT TTTAATTAAG AAAATTTTTT TTGTAGAGAT 60
GGGGTTATGC TGTGTTGCCT GGCTGGTCTC AAACTCTGGG TCTCAAAGCG GTCCTCCTGC 120
TTTGGCCTCC TAAGGTGCTG GGATTATAGG CATGAGCCAC TGTGCCAGGC CTGAGGATAT 180
TATCATTATA AATATATATG TACCCCACAC TGGATGACCC AGATATAAAG CAAATATTAT 240
TAGATTTAAA GGGAGAAATA GACTCTAATA CAATAGTAGT TGAGGACTTC ACTCTACTCT 300
CAGCATTGGA CAGATTTTCT AGACATAAAA CCAATAAATA TTGGATTTAA ATTGCACTTT 360
ACACCAAATG GTCN 374

SEQ ID NO:7303
SEQUENCE LENGTH:113
SEQUENCE TYPE:nucleic acid
TOPOLOGY:linear
CLONE:HUMGS08542
SEQUENCE DESCRIPTION:
GATCCCATTT CTATGAAATG TCCAGGGCAG GCCCAGCCAC AGAGACAGGG AGTAACGTGG 60
TGGTTTATTA TAAATTTTTC ATGAAAATAA AATATACAGT ACTCTCGTGN AAA 113

SEQ ID NO:7304

SEQUENCE LENGTH:286
SEQUENCE TYPE:nucleic acid
TOPOLOGY:linear
CLONE:HUMGS08543
SEQUENCE DESCRIPTION:
GATCTGCACA GCCTCTAGAG GCCTCCCAGC AAATGCGGGG AGCCATGCCC CCAGGGTCTA 60
CACACTCTCG TTCATCAACA TCACAACTGG AATTCGGGAT TTGTGAAGTT TAGAGCTGNA 120
CAGACTGTTA CAGATTATGA GTCAACACGT ATATTTTCTC TTTCAAAATA ATAATATTTC 180
GTTTTTGACT TTTTACTAAG TGAATATTAT TTTTTAAATC TGCCTATATA TTGGAACCTC 240
TATTTTATAA TAATAATGAT AATAAATCAG TACCCAGAAG TATAAA 286

SEQ ID NO:7305
SEQUENCE LENGTH:116
SEQUENCE TYPE:nucleic acid
TOPOLOGY:linear
CLONE:HUMGS08544
SEQUENCE DESCRIPTION:
GATCTAAATN ATGTACTTGT TAGTGTATTC ATTCATATTG ATTGTAAAGG ATTATTTTTC 60
ACTCAGTACT GATGTCCTTG GAAATCTTAC CTGGAAACAT GTTTGCAAAA AACAAA 116

SEQ ID NO:7306
SEQUENCE LENGTH:137
SEQUENCE TYPE:nucleic acid
TOPOLOGY:linear
CLONE:HUMGS08545
SEQUENCE DESCRIPTION:
GATCCCAAGT CTACCCTGGA AATNCCCAGG CACAGCATGC CTGGAGGTGT GAGCCGCCTC 60
TTAGAAGCTT TATAATCCCG GATAATNCTG CTGTAATTGG CATTGTTTTT CCATTACAAT 120
CCTTTCATTA CCTGAAA 137

SEQ ID NO:7307
SEQUENCE LENGTH:317
SEQUENCE TYPE:nucleic acid
TOPOLOGY:linear
CLONE:HUMGS08546
SEQUENCE DESCRIPTION:
GATCCCAGCT TGGTGGGAAA GTGCAGAAGA ATTGCAAAAC TGACATCCCA TNTNACAGCA 60
ATAGTGACCT TTATTTAAAT TGTTGTGTTA TAGTTTATGC TTCTTAAATC ATTTTTCAAC 120
NTAAACAGCC AATTTCTAAG CAGACAGGAA AACTAAATAA TAAGTTNATT NATATAACAA 180
AGATGCAGGT TCCTGCTCAT TCCAGTAATG TCTTTGAAAG CANNNCTNAT ATTTNTTTTC 240
TAGATTATCC CTGTGAATAA TTGAGANCTT TTTGGAGTCA AGNATGAATA AAGGTGTGGC 300
AAGGNTATAA ACANNAN 317

SEQ ID NO:7308
SEQUENCE LENGTH:337

SEQUENCE TYPE:nucleic acid
TOPOLOGY:linear
CLONE:HUMGS08547
SEQUENCE DESCRIPTION:
GATCTGTCAG AAAAGACATA TTTTTGAGAT AAAGGAGTTT CTAAATAATG AATTATTGTT 60
TAGTACCAGC TTTTGGAATC CTTACATTTT AAGGAACTTG AATAATTTAA ATATTAGATA 120
ATGTAACTCC TCATAAAAAC AGTGTCTAGA CAAGGGCCAA TATTGCATTG AGATTGGATG 180
AACATTTTAT TTTGCATATA ATTGTTGTCT ATAAAACAAA ATACTAATAA TAATGGCTAC 240
CNGTTAGNCA NTTTAGNNCN TGTCTTACTA TTTACCNNTG TGGNAAAACA TTGCATGNTT 300
TAATNTTTTT CTAATAAAAT ATTNTNCCAT TANCNNN 337

SEQ ID NO:7309
SEQUENCE LENGTH:389
SEQUENCE TYPE:nucleic acid
TOPOLOGY:linear
CLONE:HUMGS08548
SEQUENCE DESCRIPTION:
GATCCCACCA ATCCTCGTTC CCCCTAAACA TGCTAACAAA AATCCCTTAT TGTGGGTATT 60
AAAATAATAA CAGTTACACT GTATGCATAT TTATGTGCTC CTTTTGTCTG GTTTTNCTTT 120
TCATCATGTA TAAGCTGAAT TCAGCATTAG TTTCTCACAT CTTCCCCCAG GTATCCCCAA 180
CAGAATTTTT ATGTCCCAGC TTGTATTAAA TAGAAGTGAA ATATTAAGGA AAATAAGGAA 240
CTTGTGCAAC TTTTTTNATG CATTGTTCTC AACCATTTAA TTTATTGAAA GGAGATGCTG 300
CAACAGTTCT TGATTTAGCA GCAGTTATTC TCTTGTTTAC ATAGTTATGG TTTTNTGTTG 360
TTGGTTTTTG CTCTGGTACT GGGAACCGN 389

SEQ ID NO:7310
SEQUENCE LENGTH:367
SEQUENCE TYPE:nucleic acid
TOPOLOGY:linear
CLONE:HUMGS08549
SEQUENCE DESCRIPTION:
GATCCAGGTC TTCTGATTCT TATTCCAGTG TCCTTTCTAG CATACCATGT TGCCTCTAAA 60
GATTGCAGCT CCTTATTTAC TAGAAAATTG TTCCTGCCCA ATCTACATCT CCACCTCACC 120
CCATCTTTTC TTAAGCACTA TGTTTGTGTT TTTATCAGTA TTATATTCAT TGTCTTTGGA 180
ATACATGTTC TTGTTTGTGT TTGGAAAAAA AATCTCTTTT ACCAGCTTGC ACTCGGACCA 240
ACTTGGAAAA AAAAANGCTT AAATGTTTTN GCTATGTNCA GTTTAAAAAT GTGAAGTTTG 300
TNGCTTTANC TTTTTGTAGG AAAATCTANT ANCACTGGCT TAAGTGCTGA CTTGAAATGC 360
TATCCAN 367

SEQ ID NO:7311
SEQUENCE LENGTH:56
SEQUENCE TYPE:nucleic acid
TOPOLOGY:linear
CLONE:HUMGS08550
SEQUENCE DESCRIPTION:

GATCCAGCGA ACCACTGCAC TCTAGCTGGG GAGACAGAGC AAGACTCCGT CTCAAA 56

SEQ ID NO:7312
SEQUENCE LENGTH:92
SEQUENCE TYPE:nucleic acid
TOPOLOGY:linear
CLONE:HUMGS08551
SEQUENCE DESCRIPTION:
GATCTCTTCC CTTAGGAAAT ATCAACATCT TTATGTACAT GTCAGAGTCA TGGTGTGATG 60
ACGTACTTTG AAGCATGGAG TCAGTTTTCA AA 92

SEQ ID NO:7313
SEQUENCE LENGTH:368
SEQUENCE TYPE:nucleic acid
TOPOLOGY:linear
CLONE:HUMGS08552
SEQUENCE DESCRIPTION:
GATCCACTTA TGAACTGAAC ATTCTCCATG AACAGGTGGT TGGATTGGTA TCTGTCATTG 60
TAGGGATAGA TAATAAGCTC TTCTTATTCA TGTGTAAGGG AGGTCCATAG AATTTAGGTG 120
GTCTGTCAAC TATTCTACTT ATGAGAGAAT TGGTCTGTAC ATTGACTGAT TCACTTTTTC 180
ATAAAGTGAG CATTTATTGA GCATTTTTNC ATGTGCCAGA GCCTGTACTG GAGGCCCCCA 240
TTGTGCACAC ATGGAGAGAA CATGAGTCTC TCTTAATTTT TATCTGGTTG CTAAAGAATT 300
ATTTACCAAT AAAATTATAT GATGTGGAAA AAAAAAAAAA AATTGCGGGC TTGNANGNTN 360
GGGGTCAN 368

SEQ ID NO:7314
SEQUENCE LENGTH:297
SEQUENCE TYPE:nucleic acid
TOPOLOGY:linear
CLONE:HUMGS08553
SEQUENCE DESCRIPTION:
GATCACACCA CTGTACTCCA GCCTGGATGA CAGAGTGGAG ACTCTGTTTC AAAAAAACAG 60
AAAAGAAAAT ATAGTTTGAT TCTTCATTTT TTTAAATTTG CAAATCTCAG GATAAAGTTT 120
GCTAAGTAAA TTAGTAATGT ACTATAGATA TAACTGTACA AAANTTGTTC AACCTAAAAC 180
AATCTGTAAT TGCTTATTGT TTTATTGTAT ACTCTTTGTC TTTTTAAGAC CCCTAATAGC 240
CTTTTGTAAC TTGATGGCTT AAAATTCCTN ATTAATNCTG CCATTTCAAN TTCCAAA 297

SEQ ID NO:7315
SEQUENCE LENGTH:86
SEQUENCE TYPE:nucleic acid
TOPOLOGY:linear
CLONE:HUMGS08554
SEQUENCE DESCRIPTION:
GATCTTTGAA TGAGCTTTTT AAGGAAGAAA TATTATATAT TGTTTGTTAA AGTTTATTGA 60
AATAAAGAAT CATTTAAATC TTCAAA 86

SEQ ID NO:7316
SEQUENCE LENGTH:356
SEQUENCE TYPE:nucleic acid
TOPOLOGY:linear
CLONE:HUMGS08555
SEQUENCE DESCRIPTION:
GATCCAAATT GTGAAGCCGT TGGTCAGGTG CCTGGCTACA CACAAAATGG TGGAGGCTTA 60
GCCTAAACAG TTTCTTCATT TCACATTAAA ATCCTAATTN NTGGCTCCTC TCAAAATGAG 120
TTCTAGAAGC ACNGGNTGTA GATTCCCATG TGGACACAGC CTTCAGGAGG TGAGCAGCAG 180
CTCCCTGCAG CCAGCTGGAG CCTGCCAGTT TTCCCTAGTG CCCAGCACTC CTGAGCTTCT 240
GCCCTCCGAG GGATTCCCGA AGTTTTTTCT AATGGGNTTA GTGGNGNCNC CTTTGGGGTT 300
GCTTNGGTTT CNTTTNGTTA NGNGCCCTTG NTTTTAAATT AAANCNGGGC CATTNN 356

SEQ ID NO:7317
SEQUENCE LENGTH:349
SEQUENCE TYPE:nucleic acid
TOPOLOGY:linear
CLONE:HUMGS08556
SEQUENCE DESCRIPTION:
GATCTGTGGG ACTGTCTGGG CCTGTTACTC ATCCTGCTAT CAATTTCTTA TTAATTAATC 60
TTGATGATTC TNATTAATTA ATCACATTTG CAGGAAATTC AGATGAGGCA AGAAAATTTN 120
ATTGGCCTGG GNNNGACTGA AAGCATTCCA AATTAGGCTT AGACTGTGCA AAGGGCTTAG 180
CTAAGTTATC GAGCTTAAAA CCCGTCAATT AANCAAACAT TATTTGANCA GTTACTGCAT 240
GCCACGCACT GTGTTGGGCT TAGTAATAAA ANNNAGNAAA GGTAAGNGCT TGTNCTNGCC 300
TAAATTAAAG GGTCCCCGGG GGTTTTTTCT NGAGGGGGCN NCTGCCCCN 349

SEQ ID NO:7318
SEQUENCE LENGTH:351
SEQUENCE TYPE:nucleic acid
TOPOLOGY:linear
CLONE:HUMGS08557
SEQUENCE DESCRIPTION:
GATCACCTGA GGTCAGGAGT TCAAGACCAG CCTGGCCAAC ATGGTGAAAC GCTGTCTCTG 60
CTAAAANTAC AAAANTTAGC TGGGCATGGT GGTGGGCACC TGTAATCCCA GCTACTCGGG 120
AGGCTGAGGC AGGAGANTCG CTTGATTCCA GGAGGCAGAG GTTGCAGTGA GCCAAGACTG 180
CACTGTTGCA CTCCAACCTG GGCAACAAGA GCAAAACTCC GTCTCAAAAA AAAANGTGTT 240
TAATTATTCT AAAAAAAATT CAGCCAGGTT ATTCCAAATT NTAATTGAAA NCNGGGGCCC 300
CAGGTTAANC GGGGCCTTAC CCTTCCCTTN GCNCTTTGGA NTTTNNCCAT N 351

SEQ ID NO:7319
SEQUENCE LENGTH:333
SEQUENCE TYPE:nucleic acid
TOPOLOGY:linear
CLONE:HUMGS08558

SEQUENCE DESCRIPTION:
GATCAGCAGT GTTGACCATT TATGCTGCAT AGCTGGTATT ATAGCCTTAT TAGTTGTGTG 60
GTTGACCCTT GGGGTATACA AATGTCAGTC TGAGTGGTGN CTTACTCCTT TGTTTATAAG 120
TGAATGATTG TGCATGTTTT GTATGTCATA GTATGTCGTC ACATAAAAGG GAGGGAGCGA 180
AAAACCATTA CATTAAGATA ATATTGGACC AAACTACTTA CTTGCTCTAA ACAGTTACTT 240
GTACCCCTTA ACCTGTCTTC AAAAGTTGCA TATAGTTACA GTAGTGTATA AATTAAATAT 300
TGTGGAAAAC CANNAAAANN NNNNANNNNN NNN 333

SEQ ID NO:7320
SEQUENCE LENGTH:192
SEQUENCE TYPE:nucleic acid
TOPOLOGY:linear
CLONE:HUMGS08559
SEQUENCE DESCRIPTION:
GATCAGCTGA AGGTTCATGG GTTTTAAGTG CTTGTGGCTC ACTGAAGCTT AAGTGAGGAT 60
TTCCTTGCAA TGAGTAGAAT TTCCCTTCTC TCCCTTGTCA CAGGTNTAAA ACCTCACAGT 120
TGATAATGTA CCATTGGGGN CCGTTTTAAC TGGGCCTAGG NACCCCCTCA NGAATAACCT 180
GNAAGGGCCA AA 192

SEQ ID NO:7321
SEQUENCE LENGTH:55
SEQUENCE TYPE:nucleic acid
TOPOLOGY:linear
CLONE:HUMGS08560
SEQUENCE DESCRIPTION:
GATCGTGCCA TTCCACTCCA GCTTGGACAA CAAGAGCAAA ACTCCGTTTA TTAAA 55

SEQ ID NO:7322
SEQUENCE LENGTH:78
SEQUENCE TYPE:nucleic acid
TOPOLOGY:linear
CLONE:HUMGS08561
SEQUENCE DESCRIPTION:
GATCAGACCC CCACAATATG TCTCAACCTC TGTTGAAGGG CTAGTGATGT TTAAAAAAAC 60
CTGTTATTTC CAATCAAA 78

SEQ ID NO:7323
SEQUENCE LENGTH:375
SEQUENCE TYPE:nucleic acid
TOPOLOGY:linear
CLONE:HUMGS08563
SEQUENCE DESCRIPTION:
GATCTGGTAT TTCCTGGACT AAATTCCCCT TGGGGAAGAC GAAGGGATAN TGCAGTTCCA 60
AAAGAGAAGG ACTCTTCCAG AGTCATCTAC CTGAGTCCCA AAGCTCCCTG TCCTGAAAGC 120
CACAGACAAT ATGGTCCCAA ATAACTGACT GCACCTTCTG TGCCTCAGCC GTTCTTGACA 180

TCAAGAATCT TCTGTTCCAC ATCCACACAG CCAATACAAT TAGTCAAACC ACTGTTATTA 240
ACAGATGTAG CAACATGAGA AACGCTTATG TTACAGGTTA CATGAGAGCA ATCATGTAAG 300
TCTATATGAC TTCAGAAATG TTAAAATAGA CTAACCTCTA ACAACAAATT AAAAGTGATT 360
GTTTCAAGGT GGAAA 375

SEQ ID NO:7324
SEQUENCE LENGTH:271
SEQUENCE TYPE:nucleic acid
TOPOLOGY:linear
CLONE:HUMGS08564
SEQUENCE DESCRIPTION:
GATCCACCTG CCTCGGCCTC CCAAAGTGCT GCGATTACAG GCATGAGCCA CCGCGCCTGG 60
CCGCTTTCGA TATTTTCTAA ACTTTAATTC AAAAGCACTT TGTGCTGTGT TCTATATAAA 120
AAACATAATA AAAATTGAAA TGAAAGAATA ATTGTTATTA TAAAAGTACT AGCTTACTTT 180
TGTATGGATT CAGAATATAC TAAATTAACT TTTTAAAACA CAACTTTTAA AAAATGTATC 240
AAAATAATAA ACGTGTTCTG ATATTTTTAA A 271

SEQ ID NO:7325
SEQUENCE LENGTH:102
SEQUENCE TYPE:nucleic acid
TOPOLOGY:linear
CLONE:HUMGS08565
SEQUENCE DESCRIPTION:
GATCCCCTGA GTTCAAGAGT TTGAGACCAG CCTAGGCAAC CTTGTCTCTA CAGAAAAATA 60
AATAAATAAA TAAATAAACA AACAAACAAA CCAAAAATTA AA 102

SEQ ID NO:7326
SEQUENCE LENGTH:216
SEQUENCE TYPE:nucleic acid
TOPOLOGY:linear
CLONE:HUMGS08566
SEQUENCE DESCRIPTION:
GATCATGAGG TCAGGAGACC GAGCACCATC CTGGCTGACA CGGTGAGACC NCGTCTNTAC 60
TAAAAATACA AAAGCAAAAA TTAACTGGGT GTGGTGGCGG GTGCCTGCAG TCCCAGCTAC 120
TNGGGAGGCT GAGGCAGGAG AATGGCATGA ACCTGGGAGG CGGANTTGCA TTGAGCTGAG 180
ATGGCACCAC TGCACTCCAG CCTGAGCGAN NNNNNN 216

SEQ ID NO:7327
SEQUENCE LENGTH:176
SEQUENCE TYPE:nucleic acid
TOPOLOGY:linear
CLONE:HUMGS08567
SEQUENCE DESCRIPTION:
GATCTGCTCT CTGTTGAGAG TTGGTAATCA TTGGTTTGAA ATGTGATGAA ACCACTCAAG 60
CCAATGAAGG TGGGTGTGTA GGTGGGGAGT ACTTTGCCAT AATATTTTAA AACATTACCT 120

GGTTAGAGTT CTAAGTGGTA CTTATTTTTG TTTGGTTAGG GGAAAGCCTG AATAAA 176

SEQ ID NO:7328
SEQUENCE LENGTH:106
SEQUENCE TYPE:nucleic acid
TOPOLOGY:linear
CLONE:HUMGS08568
SEQUENCE DESCRIPTION:
GATCTCTGGC TGTTTGATTT TTTTNATATT GTATTTTTAA AATNTGTTAA ACAGTGCCCT 60
GTGAGCACCA AGTACCACTA GATGAATAAA ACGTATTATA TCTAAA 106

SEQ ID NO:7329
SEQUENCE LENGTH:379
SEQUENCE TYPE:nucleic acid
TOPOLOGY:linear
CLONE:HUMGS08569
SEQUENCE DESCRIPTION:
GATCCGGCAG GCAGAGAGGA AAAAGGGAAC TTGTACCAAG CACAAACAGG NTCTAGGCAT 60
AGTGCTGTGT ACTTAGGTTC TTCCTGTGCT TCAGTCTTTC CAATAAATCC AGGGGTTAGG 120
TTTTAATCTN NCTATTTNAT AGTTACAGAC CATTTTAAAT GGACAAGCAG CCCATTTAAA 180
GATAAAAGTA CTTAGATGGA CTAGCAGCTT CTCTCACCAA TTATTTCACC ACACAAAGNT 240
AACATCATTG TGAAATTTGG TTTAGTTAAC ATGCATTCCC TGGCCTGCCT CACACCGGAC 300
CTNTACCCGG TACCATGCAA ATGCACATCT GCTGCTACAC TTGTGGGATG CTGGTTTAGG 360
TAATTNTTTT TTATAATNN 379

SEQ ID NO:7330
SEQUENCE LENGTH:271
SEQUENCE TYPE:nucleic acid
TOPOLOGY:linear
CLONE:HUMGS08570
SEQUENCE DESCRIPTION:
GATCCCAGCA GAATACCAAA ATCCTATTTT TTTTGACTGA GTATTTGTAG ATGCTTAATG 60
ACTGAAATNA ATTTGGAGGC ACTGATGAAA GTAATTTTTT NAAAGTTCTC AGGTACTGTT 120
CAATTATTTA ATGTTAAGTT TAGTATCAAG ATACAGTTGT TTTNAAAATG CCAAAATGCT 180
GTTTATNATA CAGAATATTT NATTACATTT GCAATATCTT TGTATATAGT GATTTTNTNC 240
TTGATAATAA ATGGAAAANT TCTAAACCAA A 271

SEQ ID NO:7331
SEQUENCE LENGTH:382
SEQUENCE TYPE:nucleic acid
TOPOLOGY:linear
CLONE:HUMGS08571
SEQUENCE DESCRIPTION:
GATCGACCTG CCTCTGCTTC CCAAAGTGCT GGGATTACAG GCGTGAACTA CCGCGCCTGG 60
CCCTCAGATA TTTTTAAATT TGAAAATTAA GAGTATATAT TAATATCAAG TNGCAGAAAC 120

AGAATGAAGT GTTATAAAGG CGTTAAGTAG TAGGGNGGCA TAAAACTTAA ATCTTTAAGC 180
TAGAAATAGC TTGAGGAATT ATCTGATGCA GTACTCTGTT GTAATATAAC AAAAATGGAA 240
TCACAGTTTT ACAAATCAGG CAGCTGCAGC TCAGNGAGGT TAAATTTCCA CAGACCTGTT 300
TATGCAGCGC GGTAANTGAG GAAGTAGACC TAAAATTTGG GGCCTCTGCT ACTCTGGGCC 360
ATTACCTCAG CCTCGCTGGC AN 382

SEQ ID NO:7332
SEQUENCE LENGTH:315
SEQUENCE TYPE:nucleic acid
TOPOLOGY:linear
CLONE:HUMGS08572
SEQUENCE DESCRIPTION:
GATCTCACAG GAATCCTTCA ACTGCTTTCT ACCAAGCGTT CCATTTGAAC ACGTTAAAGG 60
AATCAAAGAG CCTCTGGGAT AGGGGGGAAA NCACATGGTA TCTAGCATGG GACAAAGCAG 120
CAAAATAAAC CAAAAGGACA ATCTCTATTT TATATAGAAA ATAAACATAC TTTTCTGCAT 180
CCTGTGTACT TTAAGTATAT CTATATTTTT GAAGGGTTCA TACTGTGTTG AATTTTTCTT 240
ATGAAATAGT CACTTCCCCA GTGTATTTTA ATGCAAATGC ATATTCTATA AAATAACCTA 300
TATTTTAATT TTAAA 315

SEQ ID NO:7333
SEQUENCE LENGTH:104
SEQUENCE TYPE:nucleic acid
TOPOLOGY:linear
CLONE:HUMGS08573
SEQUENCE DESCRIPTION:
GATCTTTAAT ATCTGATATA TTCCTGGTAC TCGTACTGAT AAGGGATTAT TGGAAGTCAG 60
TCACAGAATT TGGAAATAAA TTCTAGTCTN TCCTTAGCTN NNNN 104

SEQ ID NO:7334
SEQUENCE LENGTH:140
SEQUENCE TYPE:nucleic acid
TOPOLOGY:linear
CLONE:HUMGS08574
SEQUENCE DESCRIPTION:
GATCTACCTG AGGCTCAGTC CTCATCTGTA AAATGCTGAT AAAAGGACCT ACCACCTAGC 60
GTTGCTGTGA GGTGTCAGTG AAATAAAATG TAAAATGCTT AGCACAGTGA ACTTTAATAA 120
ATGGTAGTTG TGGTTGCAAA 140

SEQ ID NO:7335
SEQUENCE LENGTH:93
SEQUENCE TYPE:nucleic acid
TOPOLOGY:linear
CLONE:HUMGS08575
SEQUENCE DESCRIPTION:
GATCTAAAGT CTAAAAAACA AATCCTTAGG TTAAGAAGAA AATAATGCAA TAAATAAAAT 60

GTTATAAATA AATGACATAT TGCNACTTTT AAA 93

SEQ ID NO:7336
SEQUENCE LENGTH:96
SEQUENCE TYPE:nucleic acid
TOPOLOGY:linear
CLONE:HUMGS08576
SEQUENCE DESCRIPTION:
GATCATACCC ATGAAACCCA CTGTTTAGAT TTTAAAAAGT TCAATGTTTT TCTGTATTTN 60
ATCTCTTCAT TTTAAAATAA AGTTTTGCAC ATGAAA 96

SEQ ID NO:7337
SEQUENCE LENGTH:224
SEQUENCE TYPE:nucleic acid
TOPOLOGY:linear
CLONE:HUMGS08577
SEQUENCE DESCRIPTION:
GATCTAAAAG CAGATGCTGT AGCTGCCTAC TTTAACCCAT TTTTCAACCT GTTTGTTTTT 60
TAAAGGGCTT CACTAAGGGT NNNNNATGTA CCATTGTAGG GGGCAATTTT AAGTCAGCTA 120
AGGCAATAAC CTTATGCATG AACATTTCCC AGACTTTCAT GAAGCTGTTG AGGTCCTAGG 180
CAATTAATGC GGCAGTTGTG ATAAATAAAA ACATCTCACC TAAA 224

SEQ ID NO:7338
SEQUENCE LENGTH:175
SEQUENCE TYPE:nucleic acid
TOPOLOGY:linear
CLONE:HUMGS08578
SEQUENCE DESCRIPTION:
GATCNATATT TAGTGACTGC AACATGTTTA TACCACTGAT TCAAATTCCA NNNNTGATGA 60
AGTTATACAA ATAATGCATA TATTGATAAC TTTTATTGCA AAAATGTAAA TTTAAAACTT 120
GTATAATGTT CTTGTGCTTT TTAAAATAAA ATATATGTGT ATATTTAAAA AGAAA 175

SEQ ID NO:7339
SEQUENCE LENGTH:143
SEQUENCE TYPE:nucleic acid
TOPOLOGY:linear
CLONE:HUMGS08579
SEQUENCE DESCRIPTION:
GATCTGAGGA TGGTTATAAA TACTGTAAGT ATTGTAATGT TATGAATGCA ANNNTATTTG 60
AAAGCTGTTT ATNATNATAT CATTCCTGAT AATGCTATGT GAGTGTTTTT AATAAAATTN 120
ATATTTATTT AATGCACTCT AAA 143

SEQ ID NO:7340
SEQUENCE LENGTH:106
SEQUENCE TYPE:nucleic acid

TOPOLOGY:linear
CLONE:HUMGS08580
SEQUENCE DESCRIPTION:
GATCACTTGA TTTTTAAGAA ATNAAAAATT GCACTAGGTA GAAATAGTGT ACCAGGCAAT 60
AGTCCAGCTT TAGTTTTNTG TCATTTTTNG TCTCTATTTA AAGNNN 106

SEQ ID NO:7341
SEQUENCE LENGTH:93
SEQUENCE TYPE:nucleic acid
TOPOLOGY:linear
CLONE:HUMGS08581
SEQUENCE DESCRIPTION:
GATCTCAAAG CAAATCTGTA TTTTAATCTC TCAGGTGTTC ACTAGTATTA CCAGTGACTA 60
GTGGAAATAA TAAAAGGAAA TCTTGTTCAT AAA 93

SEQ ID NO:7342
SEQUENCE LENGTH:193
SEQUENCE TYPE:nucleic acid
TOPOLOGY:linear
CLONE:HUMGS08582
SEQUENCE DESCRIPTION:
GATCTTCGCT CCAGCTTTCT GCATCTGAGC TGGAAAAAGA GCAAAAACGG GTAAAAATTG 60
AATCCAAATA TTGTCGNATG TATGCCTAAT TTAGAATGAA ATCTTGGGGT GGCAGACTCT 120
GGAGTTCTCT GTGAGGTGTG GCATTCTTTT NTCTAAATCA CTTATTTGCC AATGTATGTG 180
TCACTTTGTT AAA 193

SEQ ID NO:7343
SEQUENCE LENGTH:137
SEQUENCE TYPE:nucleic acid
TOPOLOGY:linear
CLONE:HUMGS08583
SEQUENCE DESCRIPTION:
GATCTATAAT AAGAGCTCAA TAACTNNGTC AAGGAAAGCT CTAATATATG CAGTGATGGT 60
TTATGAAAGG GTGTGGCAAT NTNAAATNTA TATTGTGTGT GATGTTCAAA TAAAGTGGTA 120
TCTACATNCA TGTGAAA 137

SEQ ID NO:7344
SEQUENCE LENGTH:389
SEQUENCE TYPE:nucleic acid
TOPOLOGY:linear
CLONE:HUMGS08584
SEQUENCE DESCRIPTION:
GATCCCTGGT ATNGTCCGAA TTGTAAAGAA CATCAGCAAG CCACAAAGAA ATTGGATTTA 60
TGGTCCCTGC CTCCAGTACT TGTAGTACAT CTNAAGCNAT TTTNTTACAG TCGATACATG 120
AGAGACAAGT TGGANACCTT AGTTGATTTT CCTATCAATG ACTTGGATAT GTCGGAATTC 180

TTAATTAATC CAAATGCAGG TCCTTGCCGC TATAATCTGA TTGCTGTTTC CAACCACTAT 240
GGAGGGATGG GAGGAGGACA CTATACTGCT TTTGCAAAAN ATAACGATGA TGGAAANTGG 300
TACTATTTTG ATGACAGTNG TGTCTCCACT GCATCTGANG ACCAAATTGT GTCCAAAGCA 360
GCATNTGTAC TCTTCTACCA GAGACAAGN 389

SEQ ID NO:7345
SEQUENCE LENGTH:367
SEQUENCE TYPE:nucleic acid
TOPOLOGY:linear
CLONE:HUMGS08585
SEQUENCE DESCRIPTION:
GATCTGTTAC CATCAGGTCA ATTCCTAGTA TGCATAAATT TTTTAACCCT TTTAAAAGAG 60
ACCTATGTTG AAAACCCCTG AAAATTCACT GAAGAAAAAT CATTACTCTT NTTCTCNGTA 120
AATCATATCA TCTGAAATAT TACAAATTTC AAATTTCTAG GTGCTATATT AATTCAATAT 180
TACAATAACT CTTACCTAAT TATTCTTACA AGTTTTAAGT TGTGGTAGTT TAGTGATTTT 240
TTTAAAAGAT GTGTGAAATG TTCTCTGCAA AATAATTCAG GCCACTGTCT CCTTTTATAT 300
ATNATNATNN TNGTNTGTGA TGANGACCAG TGAATTACGA TATTTAAAGT GAGAGACCTT 360
AATTNTN 367

SEQ ID NO:7346
SEQUENCE LENGTH:23
SEQUENCE TYPE:nucleic acid
TOPOLOGY:linear
CLONE:HUMGS08586
SEQUENCE DESCRIPTION:
GATCCCGTCT ATAATCNAAC AAA 23

SEQ ID NO:7347
SEQUENCE LENGTH:103
SEQUENCE TYPE:nucleic acid
TOPOLOGY:linear
CLONE:HUMGS08587
SEQUENCE DESCRIPTION:
GATCGGCTCC CTGTCGCGCC CGAGGAGGGC TGGACCTTTC GTGTCGNACC CTTGGGNGCG 60
NNGAGACTGG GTGGGGAGGG TGTTGAATAA AAGGGAAAAT AAA 103

SEQ ID NO:7348
SEQUENCE LENGTH:367
SEQUENCE TYPE:nucleic acid
TOPOLOGY:linear
CLONE:HUMGS08588
SEQUENCE DESCRIPTION:
GATCTGGTGG CTGGAAAAAC AAGAGTGACA TTAACAAATC ACAAAAAATC AGTTAGGGCT 60
GTGGTTTTAC ATCCAAGACA TTACACATTT GCATCTGGTT CTCCAGATAA CATAAAGCAG 120
TGGANATTCC CTGATGGANG TTTCATACAA AATCTTTCCG GTCATAATGC TATTATTAAC 180

ACATTGACGG TAAATNCTGA TGGAGTGCTT GTATCTGGAG ACAGAAGAAA CTCATCCAGT 240
CAGCTGGAAA CCAGAAATTA TCAAGNGAAA GNGATTTTAA TGAATGTGGA ATTTTTCCTC 300
TCTCTTTTTT TTTCTTTTTA ATTAAAANAA AAAAGGCTTG GNGTTCATGG GGGNTTTCCC 360
GNCCTTN 367

SEQ ID NO:7349
SEQUENCE LENGTH:22
SEQUENCE TYPE:nucleic acid
TOPOLOGY:linear
CLONE:HUMGS08589
SEQUENCE DESCRIPTION:
GATCCCGCCT NTTTCTTACA AA 22

SEQ ID NO:7350
SEQUENCE LENGTH:378
SEQUENCE TYPE:nucleic acid
TOPOLOGY:linear
CLONE:HUMGS08591
SEQUENCE DESCRIPTION:
GATCTTTTAA AAATTCATCC CATATCCAGA AAGTACCAGT TATAAAGATT GCTGACCAAG 60
CAAAGTTTTG CATCAAAGTG TCACCTCATT GCTCTGACCA AAGACTGACT GTTGTGGTTT 120
TAACTCCTCT CTGTAAAGCA TTTTGCATTT TCCCCAAGCT CCTTTCTGAA AGAAGAACCA 180
GTGCAGAGCG GCCTTTACTT TCAATTTCTA CTGCTGAATA GACTACTTAG AGAAAATGTG 240
AGTTTCAGTG TGAACAGAAT GGATTAGGAT GACGAGTTTG ATGGGCATTT TCAGTACTGT 300
ATCTAAGAAA AAAAAATTNG CACAGCTAGG AGCCTCTGAC CATTGTCTGG GTGTTTTACG 360
TGGGTCCTGT TCATCAAA 378

SEQ ID NO:7351
SEQUENCE LENGTH:379
SEQUENCE TYPE:nucleic acid
TOPOLOGY:linear
CLONE:HUMGS08592
SEQUENCE DESCRIPTION:
GATCAAAGTC AAAAATAGCA ATGNCTCCCT ATCCCTCACA CATCCAGACA TCATGAATTT 60
TACATGGTAC TCTTGTTGAG TTCTATAGAG CCTTCTGATG TCTCTAAAGC ACTACCGNTT 120
CTTTGGAGTT GTCACATCAG ATAAGACATA TCTNTAATTC CANCCATAAN TCCAGTTCTA 180
CTATGGCTGA GTTCTGGTCA AAGAAAGAAN GTTTAGAAGC NNNNNCACAA AGGGTTGGGA 240
GCTGATGAAA CTCACAAATG ATGGTAGGAA GAAGCTCTCG ACAATACCCG TTGGCAAGGA 300
GTCTGCCTCC ATGCTGCAGT GTTCGAGTGG ATTGTAGGTG CAAGATGGGA AAGGNTTGTA 360
GGGTGCAAGC TGTCCCAAN 379

SEQ ID NO:7352
SEQUENCE LENGTH:119
SEQUENCE TYPE:nucleic acid
TOPOLOGY:linear

CLONE:HUMGS08593
SEQUENCE DESCRIPTION:
GATCTGCTTT AGTGAGAGGA CAATTTCTGA TTGATTGTTT TCTCTTCAGG CCATCTCACC 60
TCTTCATTCT CTTGTNACAT TTGAAGCAGT TGATATAATG GGTTTATANN NNTAAAAGN 119

SEQ ID NO:7353
SEQUENCE LENGTH:93
SEQUENCE TYPE:nucleic acid
TOPOLOGY:linear
CLONE:HUMGS08594
SEQUENCE DESCRIPTION:
GATCAGCTAT TTTCAACATA ACTGAAGGCA TATGCTGGCC CATAAACACC CTGTAGGTTC 60
TTGATATTTA TAATAAAATT GGTGTTTTGT AAA 93

SEQ ID NO:7354
SEQUENCE LENGTH:297
SEQUENCE TYPE:nucleic acid
TOPOLOGY:linear
CLONE:HUMGS08595
SEQUENCE DESCRIPTION:
GATCTCTGTA GACTTGCCTC TTCTGGACAT GACATAGAGA AAGGAGTCAT AAATTCTCCA 60
AGGTGTCTGT TTCTTCTTTA ATGTCATTCC CTGTTTCTCC TCACATTCCC TCCCCATTTC 120
CTGGGCCCAG TCTCACACTG GTCCTTGCTT ACCCTAAATG CTATTAATTC CATCACTCTG 180
AGTATGGTGT TTGCTGTCCG CTGAATGCCA AGAGCTTCAA GAGTGTGTGT AAATAAAGCC 240
ACACCTTTAT TTTTGTATTA TTCTGAACCA TGGCTAATAA ATTGTTTCAC CAAGAAA 297

SEQ ID NO:7355
SEQUENCE LENGTH:45
SEQUENCE TYPE:nucleic acid
TOPOLOGY:linear
CLONE:HUMGS08596
SEQUENCE DESCRIPTION:
GATCTCCGAT GTGATGAATA CGAATAAAAG GCCCTTAATG GCAAA 45

SEQ ID NO:7356
SEQUENCE LENGTH:212
SEQUENCE TYPE:nucleic acid
TOPOLOGY:linear
CLONE:HUMGS08597
SEQUENCE DESCRIPTION:
GATCTGTTCA TGCTTTTGTC TTCGTCACTG CGGCGGGGCC CTTTGATGTC TTCATCTGTA 60
TGGGGTGGAA AAATCACCGG GAATCCCCCT NNNAGTTCTT TGAAAAAGTT CCATGACTCG 120
AATATCTGAA ATNAAGAAAA CAAACCGACT CACAAACCTC CAAGTAGCTC CAAATGCAAT 180
TTTTAAAATG GAAAACAAAA ATCTGAAAGA AA 212

SEQ ID NO:7357
SEQUENCE LENGTH:250
SEQUENCE TYPE:nucleic acid
TOPOLOGY:linear
CLONE:HUMGS08598
SEQUENCE DESCRIPTION:
GATCTAAACT GTATTTTCCA ATTTAAATTA AAAATGTAAT ATAGATTCAG AAAGGTTCAT 60
ATTTTTCTAA TGACTTCATT CTATATTATT TTGTTAGGTT GCATAAAGAA GCAAGGAATT 120
GTACTTGTAT TAAAAGATGA GGAAAGCTAT TAGGNTTATT GGNCCATGCC TGAAATNGGG 180
CCTTTGCCCG TTAAAAGGAA AGGTTGGCCC NTTTTTNAAG GGGGCNTTNA TNTGNNTTTN 240
TNTTAACCAN 250

SEQ ID NO:7358
SEQUENCE LENGTH:112
SEQUENCE TYPE:nucleic acid
TOPOLOGY:linear
CLONE:HUMGS08599
SEQUENCE DESCRIPTION:
GATCTGGCTT NGCCCTAAGC NNCTTAGGNA CAGAAGAGTC CATGGGGGCA TGGCACAGAG 60
CTGGTCCTGT ATTCTCCAGG GTCCGGAGCT GGCCAGGGGC GGGGAGGAGG NN 112

SEQ ID NO:7359
SEQUENCE LENGTH:386
SEQUENCE TYPE:nucleic acid
TOPOLOGY:linear
CLONE:HUMGS08600
SEQUENCE DESCRIPTION:
GATCTTATTT NACTTCATCC TTCTTGTAAC CATATTGTNA ATCAGGAAAG CTCAATCAGA 60
TACAAAAACA GGAAATTCGT TAAAAGTAAC GTTTGTAGAA GATACTAGTT GAGGAGGAGA 120
GACTTTTNAG AAAGCCTGTG TGTGTTTGTA ACACTCTTGT GACCGTGCCA AGAAAACGTA 180
AGCAAATATC AAGCCTCCAA ATCCTAATGC AACANGTCCT GANTCCCTTG CAGGTATGTC 240
CTGGATGATG ANTACACAAG CTCAGTAGGC TCCAAATTTC CAGTCCGGTG GTCCCCACCG 300
GANGTCCTGA TGTATAGCAA GTTCAGCAGC AAATNTGACA TTTGGGCTTT TGGGGTTTTG 360
ATGTGGGAAA TTTACTNCCT GGGGAN 386

SEQ ID NO:7360
SEQUENCE LENGTH:341
SEQUENCE TYPE:nucleic acid
TOPOLOGY:linear
CLONE:HUMGS08601
SEQUENCE DESCRIPTION:
GATCCGGGNA TTATACAGGT ACTGTTGGAA GTATCTTGGG GATTTTCCTG ATAAGAACAG 60
TAGTGATTGN ATAAAAAGGA CAGGATGTAA AGTGAAATCA GTAAAATATC TTAGTAGACA 120
GAGGGTGCTG AAATTTTAAC AAATGTGTAA AAAGTTCTTC CTATGCATTA ATTTTCCAGA 180
TACCCTTAAA ATGTTTAAGG AATGTAATTC AAAATACTGT TTAANAGAGA CATGTGACCA 240

TCATTCTCCC AGCGAATGTG AATCATTTAG TGNGCTACTC AAAATTAGGN GTAAATGTAT 300
ATGTACACTA TAAGANTAAN ANTCGNTACC ATTTCTTTAA A 341

SEQ ID NO:7361
SEQUENCE LENGTH:92
SEQUENCE TYPE:nucleic acid
TOPOLOGY:linear
CLONE:HUMGS08602
SEQUENCE DESCRIPTION:
GATCCGTCAG NCGACTCTAA AGCTAATTGT TTGTATTGGC ATGGNTTTTG CCAGGCATCC 60
AAAATTCAGA TTAAACAACC AATGCAATCA AA 92

SEQ ID NO:7362
SEQUENCE LENGTH:359
SEQUENCE TYPE:nucleic acid
TOPOLOGY:linear
CLONE:HUMGS08603
SEQUENCE DESCRIPTION:
GATCTCGTCT GAAACAGTGT TTGGAAGTGG GAACAGTTTT GTCCTGTATG CTGATGTGTC 60
CAGAATTTCA TTTAATGATA GACGGAAAAT GTGTGGTTAC TGAAAACTGT ATATGATACA 120
GAATTTCATA AGAGCCATGC TGTTGGGCAA AGCAACTCTT TTTCAACCAC TGCTCATCAG 180
TTTCTGTAGA GACAAAAACT CTGTACATAT TTTGGAATCT GAAGAATCCT ATGTAAATCA 240
TTTGTTACTT AAGTCTGTGA AAAACATATT TCTTTGGAGG AAAATGTATG CATTTATAAG 300
TGTTCCATGG AATCAGTTTT TATTGTATCG ATATAATTGT CTCTAAGTGT TGACTGTCN 359

SEQ ID NO:7363
SEQUENCE LENGTH:273
SEQUENCE TYPE:nucleic acid
TOPOLOGY:linear
CLONE:HUMGS08604
SEQUENCE DESCRIPTION:
GATCTCCTTA CACTGCTATT CCAGAGGAGA AGNCACCAGG TCACTCAACT CTTAGTATCA 60
TCTACTGGAA ACTGGCTAAG ACAGTATTTA TGTGCTTCTC TCACAATAAC AGGAAGATAN 120
CTATATCACA CAATGGATAT GGCAGTTGGC TTGACTGCAG TAATCATTTA ACTATGTATA 180
TGTATATCAA AACATCATGT TGCATACCTT AAATATGTAC AATAAAAATA AAATCTAAAG 240
GANATAANTT AANATTACCT TCCACCAGGC AAA 273

SEQ ID NO:7364
SEQUENCE LENGTH:358
SEQUENCE TYPE:nucleic acid
TOPOLOGY:linear
CLONE:HUMGS08605
SEQUENCE DESCRIPTION:
GATCAGGGAT GCAGTACCAC TTGCAGTCAA ATGAATTCCT TCGAAATGTA TTTNNACTTG 60
GACCCCCAGT GATGCTTGAT GCTGCAACGC TTAAAACGAT GAAGATTTCT CGTTTCGAAA 120

GGCATTTATA TAACTCTGCA GCCTTCAAAG CTCGAACCAA AGCTAGAAGC AAATGTCGAG 180
ATAAGAGAGC AGATGTTGGA GAATTCTTCT AGATTTTCAG AACTTGAAGA CTATTTTCTA 240
ATTTCTATTT TTTTTTCTAN TTCAATGTAT TTAAACTCTA GACACAGTTT TTATCCTGGA 300
TTAACTTAGA TAACTTTTGT AGCAGTGGTT ATATTGCTTA TAATTTAATG TACAATTN 358

SEQ ID NO:7365
SEQUENCE LENGTH:263
SEQUENCE TYPE:nucleic acid
TOPOLOGY:linear
CLONE:HUMGS08606
SEQUENCE DESCRIPTION:
GATCTACNGT GAATATAAAA TGTTATTAGC CTTATCAAAT AAATAAATGA GCANAGCTCT 60
TTGTTCGCTC TATTGAACTG GGTGATAATN ATTTAGATGT GAATGTTAGG TCAGTTCTAC 120
TGTAATAACT AATCGTAAAA GTTGTAAATN TGTTTAATGG ATGAAATGTA CANTTTTTGT 180
ATTCTNTACA GCTTTGTCTT TTTTAAATAT GAAGTACTTA AATGTACTTA CTATGATAGG 240
AAATATAATG TGTGCCTTCT AAA 263

SEQ ID NO:7366
SEQUENCE LENGTH:366
SEQUENCE TYPE:nucleic acid
TOPOLOGY:linear
CLONE:HUMGS08607
SEQUENCE DESCRIPTION:
GATCTAAGTA AAATGTTTTG AAGTTTATCA GAAACTAAAT GTACTTTTAA AACGTATAGG 60
GTCAGGGTTG GGGGAAAAAT ACAGGTATAG TAAGNAAGAA AAGTGACCCA TGAAGAAAGC 120
ATCGTGAGGT TGTATGTTGG TTGACTGTGA TTAAAATGCG GGGCTGGTGT AAGTTGTAAG 180
TGGTGGCTGA TTGCCGTGTA ACTATGTACA TGATTGTTGG GATGGCTGTC CCATATTTTG 240
TATATTGGAA TAAAAATTTC TATAANTTAT NGTAACTAAA AGTAAATATT CTAAATTAAG 300
TCCCACTNCT TAAGTCACAT GGCTTCTGTC TTGGGAATTT TACCTTTTAA NANGATTATT 360
TTNAGN 366

SEQ ID NO:7367
SEQUENCE LENGTH:378
SEQUENCE TYPE:nucleic acid
TOPOLOGY:linear
CLONE:HUMGS08608
SEQUENCE DESCRIPTION:
GATCTTGGCT GTTCTTTCTA TTCTGCCAGC CATCTTCCAA AAAAAACTAA AGCAGAAATT 60
TGAGTAAAAA TAATCATCTG ATTTTAAGTT TTGCTGTCAT CACCATCTCA GGATTAACAG 120
CTGCGACTTT AGGTGGGGTA TATTTTCTTC TCCTAAGAGA ATAGACAGTT TTTCCAGATT 180
CATCATCATT GACTGTCAAG AAAGGACCCT TCAGCAAGGC TGTACCNTCA ATGCAGTTGA 240
TGGCCTGTNT TCACGGNTTT ACAGACTTGG CCTGATGCCC ATGTAANTTC AAGCTTTGGC 300
TTGTGGNAAC AACCACANGG ANGNCAAGGC ATCTGTNGGT GCGGGNGGGC AAAGCAGGCT 360
TANCTNGGGN GGTTGACN 378

SEQ ID NO:7368
SEQUENCE LENGTH:421
SEQUENCE TYPE:nucleic acid
TOPOLOGY:linear
CLONE:HUMGS08609
SEQUENCE DESCRIPTION:
GATCTAGAAA CCCTTTTACC TGGCCCCAAG GCTCTAGCCA GTGGTCTCTG GTGACCCCTG 60
CAAGCTCAGA GATGGGGNTG TTGTGTGCCA GGCATGAGCT TCCCCAAGAG GCAGAGGGCA 120
TACATCCCAT TTATACATTC CCTAAGCCCA CCCCTGTNTC CTCAGGCACC ACCTGCCCTG 180
GTCAGCTCCA GCCAGTCCCT GCTGGAGTGG TGCCAGGAAG TNACCACTGG CTACCGTGGG 240
GTCCGAATCA CCAACTTNAC CACATNNTGG CGCAACGGCT TGGCCTTCTG TGCCATCCTG 300
CACNGATTNT ACCCAGACAA GATGTGAGCT GCCAGAGGGG TGGGACGAAT GGGGGAGCCA 360
TCAGGGAGGG CAGTNTGGAC CCAGCTTTGA CCAGANAATT TTTNGAGTGC TTTGCTNGTT 420
N 421

SEQ ID NO:7369
SEQUENCE LENGTH:189
SEQUENCE TYPE:nucleic acid
TOPOLOGY:linear
CLONE:HUMGS08610
SEQUENCE DESCRIPTION:
GATCAGCAAT GAGTCGGCAA TTGACTTCTA CAGGAAGTTT GGCTTTGAGA TTATTGAGAC 60
AAAGAAGAAC TACTATAAGA GGATAGAGCC CGCAGATGCT CATGTGCTGC AGAAAAACCT 120
CAAAGTTCCT NCTGGTAAGA CNNCAGNTGT NAAAANGCCA GCAACCTGNC CAATTTCCAN 180
TGANCCTTN 189

SEQ ID NO:7370
SEQUENCE LENGTH:147
SEQUENCE TYPE:nucleic acid
TOPOLOGY:linear
CLONE:HUMGS08611
SEQUENCE DESCRIPTION:
GATCTGGTGG AGAAAAGAAG CAAAACTTTG TTTTAATTTA CTGTTTTTTT AAAATCTTTT 60
TGGATATTGT TTTGCATCCC ATACATTTGC AACTGGCAAT GAAATGTAAG CTTTGGTTCT 120
GAAAAAAAAT AAATGTAGCT ATTCAAA 147

SEQ ID NO:7371
SEQUENCE LENGTH:108
SEQUENCE TYPE:nucleic acid
TOPOLOGY:linear
CLONE:HUMGS08612
SEQUENCE DESCRIPTION:
GATCCACATG CNTGTGGCAG CCCAAGGCTT TGTTGTAGGA GCAATGACTN TTGGTATGGN 60
CTATNCCATG TATCGGGAAT NCTGGGCAAA ACCTAAGCCT TAGAAGAN 108

SEQ ID NO:7372
SEQUENCE LENGTH:273
SEQUENCE TYPE:nucleic acid
TOPOLOGY:linear
CLONE:HUMGS08613
SEQUENCE DESCRIPTION:
GATCTTTGAG CTGTGGTTTC TCCACCTGTG GAATATAAAT GTNAACAACA TAACCTTCTT 60
TCTAAGGATA ACTTGAGAAT TAACAGTGCA AGTAAATGGA AAGTATAGTA CCTGTNACCA 120
GATATTTAAT AGTAGTTATT ACGAAATTGT AACTCACTAC CTGTNTTAGT TGAGGTTTAT 180
CTTCAGCCAT GCAAATCAAA ATCAGATTAT TTNCACATCA GCAGATAAGG TTTGTNTGAG 240
ANTCTNATTT TNNATTTGNT ACTCTNATTT NNN 273

SEQ ID NO:7373
SEQUENCE LENGTH:419
SEQUENCE TYPE:nucleic acid
TOPOLOGY:linear
CLONE:HUMGS08614
SEQUENCE DESCRIPTION:
GATCACAGAT GTAGTTCCTG AGGTTGAGTC TCCTTCTCAG ATGGATGTTG AATTGGTGAG 60
TGGGTCTCCT GTGGCACTCT CACCCCAGCC TCGATGTGTG AGGTCTGGTT GTNAGAACCC 120
TCCCATTGTG AGTAAGGACT GGGACAATGA ATACTGCAGC AATGAGTGTG TGGTGAAGCA 180
CTGCAGGGAT GTATTCTTGG CCTGGGTAGC CTCTAGAAAT TCAAACACAG TGGTGTTTGT 240
NAAATAGTCC TTCCTGTTCT CCAAGCCAGT GAAGAGTTAT CTGCTGGGAA AGTGTCCAAG 300
AGCCTGTTTT TGAAACACAA GCTGGGCTTC TGGTAGTGCC TCATCACAAC CCATGATGGC 360
TGTTCATGGT TTCACCCCTT TTNTTTCCNT CAGCAGAGGC CAGGCTATTG GAGCAGTTN 419

SEQ ID NO:7374
SEQUENCE LENGTH:209
SEQUENCE TYPE:nucleic acid
TOPOLOGY:linear
CLONE:HUMGS08615
SEQUENCE DESCRIPTION:
GATCCTAATT TGCACACTAT CACCTAAAGT CCTAGCATAC CTGCATTTCC AGGAAATCTA 60
ATGAGAAAGA AGTCCTGAAA GCCTCTAATT CCTGGAAACA TATGAAACGG CTAGAAAAAA 120
GTAGACTATT TTGGAGGATT AAAGACAATG GAAACTTTTT ACATTGTTTG TCTCACATAA 180
TGAATAAAAT TCCACTTTAA AAAATGAAA 209

SEQ ID NO:7375
SEQUENCE LENGTH:422
SEQUENCE TYPE:nucleic acid
TOPOLOGY:linear
CLONE:HUMGS08616
SEQUENCE DESCRIPTION:
GATCCGCAGC TCGAAAAAGA ACAAGCCACA GAANCGGGCT CGCTCGTGCC AGGACACAGC 60
AGTGTCTTTA AAAAAATCAA AACCAGAAGT TTTATCAGCA GCAGGAAGNN TGTGGGACTC 120

TGTCCAAGTT CACCGTCACC ATCAAGCCAC TGGCTGTGGA AGGAGTTTNT CCAACAGGGT 180
CAGTGTCACA GCCACAACTT CAGAAAGCAG CCATCCCGCG TGTCGTCCAA ACAGCAGTGC 240
CTGCNTCCCG CTCCACGGAG TGTTCCNGAA CACCTCCCTC TGAACTAATC CCGGAGCACC 300
CCACCCCCAG CGAGTCCCAC CTGGACCTNT TCAAAGTCAA AACTCTTCTT TNGGGCCAAC 360
CAATTNTTCG GGCTTTNGAG GCCTTGAACT TGGGGCACCA AGTTTNTTTC CATGGGAANT 420
TN 422

SEQ ID NO:7376
SEQUENCE LENGTH:413
SEQUENCE TYPE:nucleic acid
TOPOLOGY:linear
CLONE:HUMGS08617
SEQUENCE DESCRIPTION:
GATCCCACCA GTTTCTGGAC GTGGAGAGTC TGGGGCATCT CCTTCTTATG CCAAGGGGCG 60
CTTNNNGTTT TCATGGCTGC CCCTCCAGAC TGCGAGAAAC AAGTAAAAAC CCATTGGGGC 120
CTCTTGATGT CTGGNATGGC ACGTGGCCCG ACCTCCACAA GCTCCCTCAT GCTTCCTGTC 180
CCCCGCTTAC ACGACAACGG GCCAGACCAC GGGAAGGACG GTGTTTGTNT CTGAGGGAGC 240
TGCTGGCCAC AGTGAACACC CACGTTTATT CCTGCCTGCT CCGGCCAGGA CTGAACCCCT 300
TCTCCACACC TGAACAGTTG GCTCAAGGGC CACCAGAAGC ATTTNTTTAT TATTATTATT 360
TTTTAACCTG GACATGCATT AAAGGGTCTA TTAGCTTTCA GAAAAAAAAA ATN 413

SEQ ID NO:7377
SEQUENCE LENGTH:157
SEQUENCE TYPE:nucleic acid
TOPOLOGY:linear
CLONE:HUMGS08618
SEQUENCE DESCRIPTION:
GATCCACAAA TGAAAGGGAT ATAAAAATAA TGTCATAGGT AAGAAACACA GCAACAATGA 60
CTTAACCATA TAAATGTGGA GGCTATCAAC AAAGAATGGG CTTGAAACAT TATAAAANTT 120
GACAATGATT TATTAAATAT GTTTNCTCAA TTGTAAA 157

SEQ ID NO:7378
SEQUENCE LENGTH:399
SEQUENCE TYPE:nucleic acid
TOPOLOGY:linear
CLONE:HUMGS08619
SEQUENCE DESCRIPTION:
GATCCTAACA GCTTGCATTA TTTGGTGCAT CTGCTCAATC AAGTCTAATA GACACAAGGA 60
TGGCTTTCAT CGGCTCAGGC AGCATCATGA TGAGTATGAA GATGNAATTC GCATGATGTC 120
TACCGGCTCC AAGAAGTCCC TCCTAAGCCA TGAGTTCCAG GATGAAACAG ACACTGAAGA 180
GGAAACATTA TATTCTAGCA AACATTGGNG GNCACATTTT GCATATCTCC CAGCATAAGT 240
ACCAAGCAAA ATTACAGTTC CTCTTGGGAG AACACTGCAT TAAGGATGCG AGACTCTCTT 300
GCTTCTTCAA AGNGCTTTTG GGAATTTAAA TTGCTAANTA TGTATTCTCA AAANAANNNN 360
NGGGANNTTT GGGGGGNNTG GGTTTTNCGG AGNGANGNN 399

SEQ ID NO:7379
SEQUENCE LENGTH:34
SEQUENCE TYPE:nucleic acid
TOPOLOGY:linear
CLONE:HUMGS08620
SEQUENCE DESCRIPTION:
GATCGGTCCC TAAACCTCAT TAAAATATTT GAAA 34

SEQ ID NO:7380
SEQUENCE LENGTH:405
SEQUENCE TYPE:nucleic acid
TOPOLOGY:linear
CLONE:HUMGS08621
SEQUENCE DESCRIPTION:
GATCTNATGG ATATGGCAGT GGACGTGGAT TTGGGGATGG CTATAATGGG NATGGAGGAG 60
GACCTGGAGG TGGCAATTTT GGAGGTAGCC CCGNTTATGG AGGAGGAAGA GGAGGATATG 120
GTGGTGGAGG ACCTGGATAT GGCAACCAGG GTGGGGNCTA CGGAGGTNGT TATGACAACT 180
ATGGAGGAGG AAATNATGGA AGTGGAAATT ACAATNATTT TNGAAATTAT ANCCAGCAAC 240
CTTCTAACTA CGGTCCAATG TNGAGTGGAA ACTTTGGTGG TAGCAGGAAC ATGGGGGNAC 300
CATATGGTGG AGGAAACTAT GGTCCAGNAG GCAGTNGAGG AAGTNGGGGT TATTGTNGGA 360
NGGNGCCGAT ACTGAGCTTC CTTCCTATTT NNCCATGGCA TTTNN 405

SEQ ID NO:7381
SEQUENCE LENGTH:261
SEQUENCE TYPE:nucleic acid
TOPOLOGY:linear
CLONE:HUMGS08622
SEQUENCE DESCRIPTION:
GATCCTAGCC AGATTACCAG AAATTNCGTT AGTCATCTAA ATCAGAGACA TCAATTTNAT 60
TATGGAGAAT TTNTGAATCT CCAGCTAGAT GANGAAACCC AATACCAAAC TCCTGTTGAA 120
GAATCTTTNC AAGTAAACAT CTGAAGGCTG TAGACATCTC TCCATCTTTG TACCTGCAAG 180
TCCCATCTTT AAGGGGGAAA CTACATGANG TCACCGTTAC AGTAACTTGA TGTGTATATT 240
AATAAAAGTA ATTCAGTCAA A 261

SEQ ID NO:7382
SEQUENCE LENGTH:44
SEQUENCE TYPE:nucleic acid
TOPOLOGY:linear
CLONE:HUMGS08623
SEQUENCE DESCRIPTION:
GATCTATGTA TAGATAAAGA TTAAACATAT CTAGTACAAG GAAA 44

SEQ ID NO:7383
SEQUENCE LENGTH:109
SEQUENCE TYPE:nucleic acid

TOPOLOGY:linear
CLONE:HUMGS08624
SEQUENCE DESCRIPTION:
GATCTGTTTC TTAAAGCTAC AGGGTTTAAA AAATAAAAAT GAGTGAAAAT ACTTGATGTT 60
TCTTGAAAGA TAAATTTAAT AATAATAAAT AAATACATAA ATACATAAA 109

SEQ ID NO:7384
SEQUENCE LENGTH:93
SEQUENCE TYPE:nucleic acid
TOPOLOGY:linear
CLONE:HUMGS08625
SEQUENCE DESCRIPTION:
GATCATTGTG GCGTCCACAA TGTATATATA AAACAAGTTG TACACATTAC CATGTATAAA 60
TTTGTGTTTG TCGATTATAT GTCAAGAAAG AAA 93

SEQ ID NO:7385
SEQUENCE LENGTH:54
SEQUENCE TYPE:nucleic acid
TOPOLOGY:linear
CLONE:HUMGS08626
SEQUENCE DESCRIPTION:
GATCTTAATC TTATTCTCTG CCATCTTCAA ATTAAAAAAA AATTTCTTTG TAAA 54

SEQ ID NO:7386
SEQUENCE LENGTH:140
SEQUENCE TYPE:nucleic acid
TOPOLOGY:linear
CLONE:HUMGS08627
SEQUENCE DESCRIPTION:
GATCCCTTCA GAAGAAGACA TGATAGGATT GTAAGTTTTT NTCTAACTTT GGATGCAGTG 60
AGATGACCAG TGTGTTCCAG TTAAAGAAGA AGAGTGTTTT AAAATCATAA ACCAAATAAA 120
GAATCCTACC TTACATTAAA 140

SEQ ID NO:7387
SEQUENCE LENGTH:206
SEQUENCE TYPE:nucleic acid
TOPOLOGY:linear
CLONE:HUMGS08628
SEQUENCE DESCRIPTION:
GATCCACCCA CCTCAGCCTC CCAAAGTGCT GGGATTACAA GCGTGANCAC TGTGCCTGGC 60
CCTTTTTTTT TTNAAAGAGA TGGCATCTTG CTATGTCGTC CAGGCTGGTN TTGAACTCCT 120
GAGTTCAAGC AGTCCTCCTG NTTCAACATA CAGNTACAGG TACCCCCCAC TATACATTTT 180
NAATANGGNT TCATGGNTCA GNGGGN 206

SEQ ID NO:7388

SEQUENCE LENGTH:130
SEQUENCE TYPE:nucleic acid
TOPOLOGY:linear
CLONE:HUMGS08629
SEQUENCE DESCRIPTION:
GATCCCATTG CTTTCCTGTT TTAAAAATAT TTTATGCTCT TTATTTCCAC TTCTGTGAAT 60
GTGATATTTC TATTTTNTGA TTATGTTACT GAATAAACAA ACTTGCTACA TAAAATNCTT 120
AGCAATTAAA 130

SEQ ID NO:7389
SEQUENCE LENGTH:355
SEQUENCE TYPE:nucleic acid
TOPOLOGY:linear
CLONE:HUMGS08630
SEQUENCE DESCRIPTION:
GATCCAATTG TGGATTTATT TACAAACATC AAATGCCTTC AAGCCAATCC TNTTTGCTGT 60
ATGTTTTGCA GCCTACTGTA GTAGATACGC AACAGATAAT GTGGGAAAAA AAGAGATAAG 120
AGGAGGAAGC TAATAAGAGA CTGTCAAGAT TGTATACCTT CTTGGTTTCT TTTAAGAATT 180
TGTTGCCTTT NTACTATNNC AGCAAAGCAG CATTTTGTTA CTGACTGCCT AAAATCACTT 240
AATCTCAGGT GAACGCATCA CTTGCCAAAC TGTTGGAATG CTATTTGTGT TTTGTTGCAC 300
TGTTTTTTTC GTTTGTTTGT TTGTTTATTT GGTTGGCTTT TTGGAGAGGG AAATN 355

SEQ ID NO:7390
SEQUENCE LENGTH:96
SEQUENCE TYPE:nucleic acid
TOPOLOGY:linear
CLONE:HUMGS08631
SEQUENCE DESCRIPTION:
GATCTAAAAT NAGAGAAGCA TTTAAAAAAT NATTTCTGTC TTGGTTCATC TTATTGTTAG 60
TGGTTTATGC TAATAAAAAT TGATTTAACA ATCAAA 96

SEQ ID NO:7391
SEQUENCE LENGTH:411
SEQUENCE TYPE:nucleic acid
TOPOLOGY:linear
CLONE:HUMGS08632
SEQUENCE DESCRIPTION:
GATCCAGATA TGTCTGTTAT GAAAGATATC AGTATAGGTA AAGCCACAGG CAGAGGTCAG 60
TACTGATAAT TAATGTAGTA TAAATACATC ATTTACCATT TTATTTTAAA TAGGAAGCCA 120
TCAAGCATGC TAGAATTGTG AACTTTCATT ATATNTTTTT GTTGTTGACA TGAATTAACC 180
TGGCCAAAAA CAAAAAAGAA NNAAAAAACC ATGCCATTTG TCATGTAAAC CTTTTTTTTA 240
TCCCTATGGG CCTTNGGGGN CNGAATCAGT ACTTCAGTTA TTGTAATNGT GNGCTAACCT 300
CAAATTTNTN TCACCCNGTT GCCCTTTTCA TGNCCTAACC AATTNTCTGG GGGNTTGGTA 360
TGGTTCNCCC GGTCCTGNTC ATGTTTACCG NNCTCTTTTT TNGNGGGTCC N 411

SEQ ID NO:7392
SEQUENCE LENGTH:248
SEQUENCE TYPE:nucleic acid
TOPOLOGY:linear
CLONE:HUMGS08633
SEQUENCE DESCRIPTION:
GATCTAAGTT CTAAGCCAAA TAACTAACAA ATTCCAGGTA CTATGCCATC ATATAATATT 60
TATAAATGTG TTATATATAT TATGAGATAT GTATAAGAAT ATAGAATGAA TATGTAATAA 120
ATTATACTTT TAAATTAATA TTTTCAAATA TATAAATATG TAGTTTCAGA CTGAAACTGT 180
TCTAAACTAT ATAAATGTAT TATGTATGTT TTATAAAATA CAGTACTCAT AATATGTATT 240
ATATGAAA 248

SEQ ID NO:7393
SEQUENCE LENGTH:247
SEQUENCE TYPE:nucleic acid
TOPOLOGY:linear
CLONE:HUMGS08634
SEQUENCE DESCRIPTION:
GATCCTTCCA CCTCAGCCTC CCCAGTAGCT GGGACTACGG GGTCGCCACT GCACCCAGCC 60
TGTGTCTTCC AGCTCTAATC AGAAAGCTGC TTGTCGAGGG CATGGGGAAT CAAACTGAAT 120
GAACTTTTCT CTGCACTGTG GCAAAACTGT TATTTTTATG GATTTTACTA AATGGCGTTA 180
CCTTTTCAAG ATTTATATGT TTGTATAATC ATAAGAAAAT TGAGCCATTA AAGCCTTGTT 240
ATTCAAA 247

SEQ ID NO:7394
SEQUENCE LENGTH:108
SEQUENCE TYPE:nucleic acid
TOPOLOGY:linear
CLONE:HUMGS08635
SEQUENCE DESCRIPTION:
GATCCAGGCT CGTAGNTCAC TCCCTGCCCG TNTCCCAGAG ATGCTTCACC ANCACCTGCC 60
TCTGAGACCT CGCTCTCTGT TCCAGCAACC CTGGNTTGGG GGGTCANN 108

SEQ ID NO:7395
SEQUENCE LENGTH:61
SEQUENCE TYPE:nucleic acid
TOPOLOGY:linear
CLONE:HUMGS08636
SEQUENCE DESCRIPTION:
GATCTTGAGC TCCTGGCCTC CAGAATTGCA GGAGAATAAA TNTGTGTTGT TTTTAATGAA 60
A 61

SEQ ID NO:7396
SEQUENCE LENGTH:275
SEQUENCE TYPE:nucleic acid

TOPOLOGY:linear
CLONE:HUMGS08637
SEQUENCE DESCRIPTION:
GATCAGTGTT TGNTTGGGAC TGAAGGGATG ACTAATAAGG AGCAGAGGAA AGCTTTTGGG 60
GATGATGGCA CTCTTCTCTG TCTCCATTAC GGCATTGGAT ACAAGTCTCT AGAACTCCCA 120
GAACTCGTCA CTGAAAGGGG TGAATAGTAC TGTATGTAAA TTAGGTCCNN ATTTTNAAAT 180
GGGANAAATA AATATAAAGA CAAATTACAC TGCTGTATTA NNTCANNTGA TAGATGTAAA 240
AGTCTAATAT ATACGTNTTT TTCNTGATAN NCCCN 275

SEQ ID NO:7397
SEQUENCE LENGTH:288
SEQUENCE TYPE:nucleic acid
TOPOLOGY:linear
CLONE:HUMGS08638
SEQUENCE DESCRIPTION:
GATCCACGCC CTTCCCGCCA GTCTGGAGAG GGCCCTGACC GAGGACTCAA CCCAAACCAG 60
TGACACAGCT ACCAATTCTA CTTTACCTTC TGCAGAGGTG GCGTTACAGG CAAAGTGAGG 120
AGGGAGCTGG GGGACACTTT CGAGCTCCCA GCTCCAGCTT CGTCTCACCT TGAGTTAGGC 180
TGAGCCACAG GCATTTCCTG CTTATTTTAG GATTACCCAC TCATCAGAAA AAAAAAAAAG 240
GCCTTTGTGN CCCNTGTTTT GGGGGGATTA ACCNGTTTTG GGTTTAAA 288

SEQ ID NO:7398
SEQUENCE LENGTH:290
SEQUENCE TYPE:nucleic acid
TOPOLOGY:linear
CLONE:HUMGS08639
SEQUENCE DESCRIPTION:
GATCTAATTN CTATTTCATT TATTCAGAAA TAGCATTTAG ACATAAAANC CAATGTCTCA 60
CTTTGTAAAA TAACCTTTGG CTAATTTACA CACATCTAAT ACAGCGTGTT ATATAAGTTT 120
TAAGTAATAC AATGAGTCAC TACTATCATT CAGTTTTAAN TATTTTNNGT GTTAACAGGG 180
CTGAGAATAT CATGTGGTTC AGTCTTCTGN NGGNCGTTAT ATNATACCNG CNTAGTGCCT 240
TTGACCATGC NGACTATCCT CAAGGCCAGA CATCCTACAN CGGAACTGNN 290

SEQ ID NO:7399
SEQUENCE LENGTH:267
SEQUENCE TYPE:nucleic acid
TOPOLOGY:linear
CLONE:HUMGS08640
SEQUENCE DESCRIPTION:
GATCCNTAAG TACTCTTCTC TANAGAATAA AATGCATTAC CTAGGCTTTA ATAGTTAAAA 60
GCTGGNATTT ATNGACCCGC ATCTACTTGA TTTCANAATA TGCATTGATT TTAGTATGTT 120
GCTGNTCTGT TATCTGTACT CAACTATANC AGAGTAATAT ATTGTGGTNT TCTGGACCAA 180
AANTCGTGCT ACACATATGT CTGATGGCCT TNACCTTTTC ANGACAACAG TTGCAATTTT 240
GAGCACAGTC CNCCTAGTGG TANTCTN 267

SEQ ID NO:7400
SEQUENCE LENGTH:127
SEQUENCE TYPE:nucleic acid
TOPOLOGY:linear
CLONE:HUMGS08641
SEQUENCE DESCRIPTION:
GATCGGGAGC AGCCCCACTG GACCTAGGTG CCCCATCTGT TGGTCATCCA TCCTGAAGGG 60
ACAGGAAACC TCCCAGGCAG TTATTTTTTT TTCTCTATAT TTCTAGTAAA GTTTTCGATA 120
TGTTAAA 127

SEQ ID NO:7401
SEQUENCE LENGTH:168
SEQUENCE TYPE:nucleic acid
TOPOLOGY:linear
CLONE:HUMGS08642
SEQUENCE DESCRIPTION:
GATCACTAGT GCAGGAGAAC ATTACATTTT CTTCTGAAGG CAAAATGCTT GTAGGTTTTG 60
CCTCTACTTT GTATTTACTT TTAAAATTGC ACTTGTTCAC CTACCAGTGT TTACGAAATC 120
CTGTATTTGG GATGCTTTTT CTATAATAAA ATATTATAAT TTGTGAAA 168

SEQ ID NO:7402
SEQUENCE LENGTH:323
SEQUENCE TYPE:nucleic acid
TOPOLOGY:linear
CLONE:HUMGS08643
SEQUENCE DESCRIPTION:
GATCATAGTA AAGAGTAGTC AATAGGGTCT TCAGCTATTA ATTGTAGAGG TGATTAAAAC 60
CAACAAGGAG TTTCATGTGC AAAGGAGATA AGGAATGAAT ATAAAGATTG CTATTTGGGT 120
GGCTCTTATT AAACTGTGTA TTTTGTACTT ATCACTACAC GTATCCCCCA AATGCTTACA 180
TGGGAGTTTG AGGTTAGTAT TTTCACTTCC TTGGTGTTAG TACTCTATTC ACATTCTTAT 240
TGTAACCTTC CTCATTTCAC AGATAAGGAA TCTTTGGGGA TTAACCAACC TCCTTTCTGT 300
AATGGTAATC ATTAAANTAA GTN 323

SEQ ID NO:7403
SEQUENCE LENGTH:94
SEQUENCE TYPE:nucleic acid
TOPOLOGY:linear
CLONE:HUMGS08644
SEQUENCE DESCRIPTION:
GATCACTTAN GCCCAGGAGG CAGAGGTCAC AATGAGCCGA AATTGTGCCA ACTGCACTCC 60
AGCCTGGGCA ACAGAGGAAG ACTCTTCACA GAAA 94

SEQ ID NO:7404
SEQUENCE LENGTH:226
SEQUENCE TYPE:nucleic acid

TOPOLOGY:linear
CLONE:HUMGS08645
SEQUENCE DESCRIPTION:
GATCGGGGCT TTGTGTTTAA CCTCATCAGA CATTATTGCA GCCAGCTGTC AGCCAAGCTC 60
AGTAACCTTC CAACGCTCAT TTCCATGAGG CTAGAGTTCC TGAGAATCCT CTGTAGCCAT 120
GAGCATTACC TCAATCTGAA CCTTTTTTTT ATGAATGCTG ATACTGNTCC NACATCTCCT 180
TGTCCTTCCA TATCTTCCCA GGTAATAAAA GAATTATTTA ACTAAA 226

SEQ ID NO:7405
SEQUENCE LENGTH:124
SEQUENCE TYPE:nucleic acid
TOPOLOGY:linear
CLONE:HUMGS08646
SEQUENCE DESCRIPTION:
GATCCAGTCA GAGCCAGGGA GAGTGTTTGC CTTAAGGAGG GACTCCCTTG TCCAGGATGG 60
GGCTGGCACT AGGGATGGGG TGAATCTTAG GACAGGAGTG GGGGTCAGAA ATGGGAGGGG 120
TAAA 124

SEQ ID NO:7406
SEQUENCE LENGTH:206
SEQUENCE TYPE:nucleic acid
TOPOLOGY:linear
CLONE:HUMGS08647
SEQUENCE DESCRIPTION:
GATCGACCAG CAGGTGGTCT ATATCCATCC TTCCAGTGCC CTCTTCAACA GACAGCCAGA 60
ATGCCCAAAG AACTTCCTCC CTNTGGTTGC TGTAGGTGTC TCATTGGAGC AGTGCCTCTC 120
CAAGTTTGAG GATNTGAACA AAGAACTGGG ACTGGTGACT TGTNANNGNN NAGTTCAGAG 180
GGCAGAGGNC CATCATCTCA GCTTNN 206

SEQ ID NO:7407
SEQUENCE LENGTH:103
SEQUENCE TYPE:nucleic acid
TOPOLOGY:linear
CLONE:HUMGS08648
SEQUENCE DESCRIPTION:
GATCCATAAG GAGGGCTGTG TGGNGAAGAT TCAGGGACTG ANTAGAGGGA AAATCGTGCT 60
ATNCGGTAGC TACAGCAGCC CTGGGAATNC CNTTTTGTCG AGN 103

SEQ ID NO:7408
SEQUENCE LENGTH:116
SEQUENCE TYPE:nucleic acid
TOPOLOGY:linear
CLONE:HUMGS08649
SEQUENCE DESCRIPTION:
GATCCATAAG NAGGGCTGTG TGGAGAAGAT TCGGGNCTGG CTGAGGAAAA ANCGGCTGGT 60

GGTAGCTACA GCANCCCTTG GAATACCCTT TTGTCGAGGT TTTGGGNATT GTCTCN 116

SEQ ID NO:7409
SEQUENCE LENGTH:60
SEQUENCE TYPE:nucleic acid
TOPOLOGY:linear
CLONE:HUMGS08650
SEQUENCE DESCRIPTION:
GATCTATCAT GTATCTTGGT GATTACAGTT AATAATATAT TGTATATTTG ANAATNGAAA 60

SEQ ID NO:7410
SEQUENCE LENGTH:91
SEQUENCE TYPE:nucleic acid
TOPOLOGY:linear
CLONE:HUMGS08651
SEQUENCE DESCRIPTION:
GATCGCACCA CCGCACTCCA GCCTGGGCAA CAGAGCAAGA CTGTTTCAAA ATAAATAAAT 60
AAAATAATAA ATAANNCTTT CCAACTNCAA A 91

SEQ ID NO:7411
SEQUENCE LENGTH:240
SEQUENCE TYPE:nucleic acid
TOPOLOGY:linear
CLONE:HUMGS08652
SEQUENCE DESCRIPTION:
GATCTTGTGG CTAAACCAGC ATTTCTGTGT TTGAGAGATT TCCTGTTAGG TGCTTCGTCT 60
GAAAGTGAAC TCTCATAATT CAAATTGTAT AAANTAAAGC TACATTTCTA AGAGCTTGGT 120
GTAGGGCAAT TGGAATANTG TCCTGTTAGA TAAACAGACA TTTAGCANTC CTGACATTAA 180
AAGGAAATGT ATTTCTATAC AGGATTATTA GCTGTANTAC ANGATATTTA TTTANCCAAA 240

SEQ ID NO:7412
SEQUENCE LENGTH:247
SEQUENCE TYPE:nucleic acid
TOPOLOGY:linear
CLONE:HUMGS08653
SEQUENCE DESCRIPTION:
GATCGCACCA CTGAACTCCA GCCCGNATGA CAGAGTGAGA CCCTGTTTCT AAAAACAAAA 60
CAAACAAACA AACAAACAAC GTATCTTACT GACTAAACTG GAATGGAAAT CTTATAATNA 120
GTGTTGTATT TTTCTGCAAA ATAGTGAATT NGNACTTGGG AGACTTCNNN NCTTTGTTGA 180
AAAAAATTGC CTTTGTTTTG AAATTATATC ACCTGTGTTC TGANCTAAAA TGNTGGGAGT 240
TCATNNN 247

SEQ ID NO:7413
SEQUENCE LENGTH:128
SEQUENCE TYPE:nucleic acid

TOPOLOGY:linear
CLONE:HUMGS08654
SEQUENCE DESCRIPTION:
GATCCATAAG GAGGGCTGTG TGGAGAAGAT TCAGGNANCT GACTGAGGAA AAATNTGCTG 60
GTGGTAGNTA CAGCANCCCT TGGAATACCC TTTTGTCGAA AAATAAAGGA ATTGTCTTCA 120
NCCTGCTN 128

SEQ ID NO:7414
SEQUENCE LENGTH:118
SEQUENCE TYPE:nucleic acid
TOPOLOGY:linear
CLONE:HUMGS08655
SEQUENCE DESCRIPTION:
GATCCATAAG NAGGGCTGTG TGGAGAAGAT TCAGGGAGCT GACTANAGGA AAAATGTGCT 60
GGTGGNANAT ACAGCAACCC TTGGAATANC CTTTTGTCGA GAATAAAGGA ATTGTCTN 118

SEQ ID NO:7415
SEQUENCE LENGTH:151
SEQUENCE TYPE:nucleic acid
TOPOLOGY:linear
CLONE:HUMGS08656
SEQUENCE DESCRIPTION:
GATCTTTNTN ATATATGCAG TCCCATCCCT TCTGTGCCAC TCAATGCCAT CCAGACATGG 60
TTTTNCCCTC CANGGGCCTT TCTCTCCAGA GGGCACTTCG GCTGCCTCTG CTTCCTCTCA 120
TTCGAGGCCC GNCTCTTNCT NACAGAATAG N 151

SEQ ID NO:7416
SEQUENCE LENGTH:374
SEQUENCE TYPE:nucleic acid
TOPOLOGY:linear
CLONE:HUMGS08657
SEQUENCE DESCRIPTION:
GATCCCGTTG CCTCATGGAA CTCATGCATT TCACGCTGCT TGCCCTTTCT GTGCTACACA 60
GCTGGTTGGG GNGCAAAACT GCATCAAATT AATTTNCCAA GGTCCAATTG ACTGACGCCC 120
TTGACAGCCA TCTACGACTT TATTAACAGG TTACTGTGAA GATTTTNCCA CTAACTCTAG 180
ATTTTNCCTT TTTGTAATGC TGTTTATCAG AGGCGGGTGA CANGGGCTGG AAATAAAGNG 240
AGGGGACATG GTGATGAAAC ATGGCAGGNG TGTACAGATA CCAGTGGTGT GTTGCATGCT 300
CAANACAGCA GCGTCGTCAA TGGCGTCTGC TTGNTTGNNC CATNATGTCT TTGTCATAAT 360
TGGNAANNAA NNNN 374

SEQ ID NO:7417
SEQUENCE LENGTH:110
SEQUENCE TYPE:nucleic acid
TOPOLOGY:linear
CLONE:HUMGS08658

SEQUENCE DESCRIPTION:
GATCTTTATG TCACTAGTTA TAGTTAAGTT CATTCAGACA TAATTATATA AAAACTACGT 60
GGATGTACCG TCATTTGAGG ACTTGCTTAC TAAAACTACA AAACTTCAAA 110

SEQ ID NO:7418
SEQUENCE LENGTH:232
SEQUENCE TYPE:nucleic acid
TOPOLOGY:linear
CLONE:HUMGS08659
SEQUENCE DESCRIPTION:
GATCTTTTTA ATTCAGTACA ATCTCTGAAG GTGTTAACAC TAATNTTTTA CAAGAATGTA 60
GAGGCGACTC GGTGCTATGA AGCGTGTTAA ACAACTCACA CAATTGCTAC AAAACACCAG 120
GGAGGGGCTT TTTGTGTTTT TAATTTTTAA AACATTTTTC CTTTTCTTGT GAGCCATAGT 180
AGGCCTAGAG AGATTACTGG GTTTTTATAA TTAAACATTT ATTTCGGTGA AA 232

SEQ ID NO:7419
SEQUENCE LENGTH:370
SEQUENCE TYPE:nucleic acid
TOPOLOGY:linear
CLONE:HUMGS08660
SEQUENCE DESCRIPTION:
GATCTTAACC AGTCGAGTGG AGTGTACATT GTCTGAATAC AGGATGCACA ATGTTGTCAA 60
TCCTGGAAAT GGTCTTTCTT TTTTGTAAGA TATGTGAATG AAGTGTTGGT GTCCTCACCA 120
AGAGGTGGCA CCTAAGGGTT CTGAGGAAAT AAATGTATAG ACCCTTATGT ACAGACCTGT 180
GTATAANCAN CTNNNGTATA TACATATAAG GATAGCTTTT TTGACCTATA CAGCTGTNCA 240
TAAAAGTAGC TGATATTAGT TAGGCCTGTG TCACCAGTTT GGNTTTTTTN CACTGGTNCA 300
TTTGGGATTT NNTTTTNGGT GGTTTAAAAT GGCATATGCT AAGGTGTGTG ATTGGGAANA 360
AANNANNANN 370

SEQ ID NO:7420
SEQUENCE LENGTH:83
SEQUENCE TYPE:nucleic acid
TOPOLOGY:linear
CLONE:HUMGS08661
SEQUENCE DESCRIPTION:
GATCTGTGTT TTAATGAGTT TCACAGTGTG ATTTTGATTA TNAATTGTGC AAGCTTTTCC 60
TAATAAACGT GGAGAATCAC AAA 83

SEQ ID NO:7421
SEQUENCE LENGTH:394
SEQUENCE TYPE:nucleic acid
TOPOLOGY:linear
CLONE:HUMGS08662
SEQUENCE DESCRIPTION:
GATCCTCTCT TACCCCGTCC CCAGGTTTGA AACACATAGC CTCATTTCAA GGTGTAGCCA 60

GGTTCCCCCG ACTTTCCTCT GGGATATAAA AAAGGGGGTA AGGGGGCAAA GAGAGCCCTC 120
TGGGACTCTC CTCCCATACA CACTACACTG CCCCTTCTCC CCCCATCAAA ACGCTCAGNG 180
ACGTTGTGAT GATGCGACTG AGGATTATGC AACGTGGTCC AACCGGAGCG GNCAGCATGA 240
CCAGCTGTCC AGGGGCTGNC TNCTGCCTTT TCTTTTGTAA AAGCCANGAC CCTTGGGGAG 300
TTTTAATNCT GTTTTGTACT TGNCCTGTGG GGNCTNCACT TGCTTTTTCT ATGGGAGACA 360
CTTTTNAAAT TTAACAAGNT GNGGGTTTTT TGNN 394

SEQ ID NO:7422
SEQUENCE LENGTH:134
SEQUENCE TYPE:nucleic acid
TOPOLOGY:linear
CLONE:HUMGS08663
SEQUENCE DESCRIPTION:
GATCCCCATG CCTGAGGGCC CCTCGGCTGG CCTGGGCATG TGATGGCTCC TCACTGGGAG 60
CCTGTGGGGG AGGCTCAGGT GTCTGGAGGG GGTTTGTGCC TGATAACGTA ATAACACCAG 120
TGGAGACTTG CAAA 134

SEQ ID NO:7423
SEQUENCE LENGTH:69
SEQUENCE TYPE:nucleic acid
TOPOLOGY:linear
CLONE:HUMGS08664
SEQUENCE DESCRIPTION:
GATCATGNGT TTCCTCATGA GGACAAATAC CTGATTTTGA ATAAAGCAGC ATTCAGTTGA 60
AATAACAAA 69

SEQ ID NO:7424
SEQUENCE LENGTH:52
SEQUENCE TYPE:nucleic acid
TOPOLOGY:linear
CLONE:HUMGS08665
SEQUENCE DESCRIPTION:
GATCAACATT TTTTTAAAAA GAGCATTAAA GAAAGAATTG TGGTACAATA AA 52

SEQ ID NO:7425
SEQUENCE LENGTH:356
SEQUENCE TYPE:nucleic acid
TOPOLOGY:linear
CLONE:HUMGS08666
SEQUENCE DESCRIPTION:
GATCACACAT TCAGACTGTT GTGTCTGTGG AGTTTTAGGA GTGGGGGGTG ACCTTTCTGG 60
TCTTTGCACT TCCATCCTCT GCNACTTCCA TCTGGCATCC CACGCGTTGT CCCCTGCACT 120
TCTGGAAGGC ACAGGGTGCT GCTGCCTCCT GGTCTTTGCC TTTGCTGGGC CTTCTGTGCA 180
GGACGCTCAG CCTCAGGGCT CAGAAGGTGC CAGTCCGGTC CCAGGTCCCT TGTCCCTTCC 240
ACAGAGGCCT TCCTAGAAGA TGCATCTAGA GTGTCAGCCT TATCAGTGTT TAAGATTTTC 300

NTTNTATTTT TAATTTTTTT GAGACAGAAT CTCACTCTCT CGNCCAGGCT GGAGTN 356

SEQ ID NO:7426
SEQUENCE LENGTH:246
SEQUENCE TYPE:nucleic acid
TOPOLOGY:linear
CLONE:HUMGS08667
SEQUENCE DESCRIPTION:
GATCGTCGCC CCTCTGCCAA CTGCGACCCC TTTTCGGTGA CAGAAGCCCT CATCCGCACG 60
TGTCTTCTCA ATNAAACCGG CGATGAGCCC TTCNAGTACA AAAATTAAGT GGACTAGACC 120
TCCAGCTGTT GAGCCCCTCC TAGTTCTTCA TCCCACTCCA ACTCTTCCCC CTCTCCCAGT 180
TGTCCCGATT GTAACTCAAA GGGTGGAATA TCAAGGTCGT TTTTTTCATT CCATGTGCCC 240
AGTAAA 246

SEQ ID NO:7427
SEQUENCE LENGTH:286
SEQUENCE TYPE:nucleic acid
TOPOLOGY:linear
CLONE:HUMGS08668
SEQUENCE DESCRIPTION:
GATCCTTGCA GGAACCTAAG GATTACAGCA GCTGCAGATG GCAGGAGCTA CAAGCCAGCA 60
GCAGCCAATG CTCANTGGGG TACAAATGGC TCAGGCAGGT CAACCAGGGA AAATGCCAAG 120
TGGAATAAAA ACCAACATCA AGTCGGCTTC CATGCATCCC TACCAGCGGT GAGTGTGGCT 180
GGCAACCTCG ACTCCCTGGT GCTCTTTGCA GAGTTGGGCA GTGAAATNNN NNNNTGCTCA 240
AGGCTCACCT AGGATGGGTA CAATAAAAAG AACATGGGCT TTCAAA 286

SEQ ID NO:7428
SEQUENCE LENGTH:177
SEQUENCE TYPE:nucleic acid
TOPOLOGY:linear
CLONE:HUMGS08669
SEQUENCE DESCRIPTION:
GATCACTAAC AGGTGATGGG CTTTGTGCCC ACTCCAGAGA TATTGTGGGA GACAAATTCT 60
TTTAACAGCC TGTCCTCCCG GCATCAGGAG TCATTGAACA ATCATGGATT GTTGTGTTTG 120
GGATTTTTTT TTTTTTNGGN TTNNTTTTGG GTTTTNGNGN GTGTGTGTGT GNCCTGN 177

SEQ ID NO:7429
SEQUENCE LENGTH:388
SEQUENCE TYPE:nucleic acid
TOPOLOGY:linear
CLONE:HUMGS08670
SEQUENCE DESCRIPTION:
GATCCAGGTG GTCCAGGAGC CCAGGACAGG CCTTTNCTGT GGGCCCTGGC CAGACAGGGT 60
TACCTGGTGA GGTGCAGAGA GTCCCTCTAG TGGCCATTTT GTATGGTAGT TGCTAATGCA 120
GAACAAGTTC TGTCTTGGGC TTAAATTGAC TGAAGACTTT AGGGGGAAAG AATAGTAAAT 180

GCATGTAAAC AAATGGGGAC ACTCTGTTCA GGAGAATAAT CCGACTGGCA TTTGTGGCAG 240
TTTTTGAAAT GTAAATGTAT TCATGTGTGT TCTTGTAAAT ACGTGTCGCT CAGATGTCCT 300
TTGAAGTGGG AGGGAATCAA TCCGGGGATA ATTTCAAATG GAATAGAGTA TTTTGATATT 360
GTTCATTCAG AGGGTGATGT GTACACAN 388

SEQ ID NO:7430
SEQUENCE LENGTH:392
SEQUENCE TYPE:nucleic acid
TOPOLOGY:linear
CLONE:HUMGS08671
SEQUENCE DESCRIPTION:
GATCCCCACT GTCAATGGGG GATTGTCCCA GCCCCTCTTC CCTTCCCCTC ACCTGGAAGC 60
TTCTTCAACC AATCCCTTCA CACTCTCTCC CCCATCCCCC CAAGATACAC ACTGGACCCT 120
CTCTTGCTGA ATGTGGGCAT TAATTTTTTG ACTGCAGCTC TGCTTCTCCA GCCCCGCCGT 180
GGGTGGCAAG CTGTGTTCAT ACCTAAATTT TCTGGAAGGG GACAGTGAAA AGAGGAGTGA 240
CAGGAGGGAA AGGGGGAGAC AAAACTCCTA CTCTCAACCT CACACCAACA CCTCCCATTA 300
TCACTCTCTC TGCCCCNATT CCTTCAAGAG GAGACCCTTT GGGGACAAGG CCGTTTCTTT 360
GTTTCTGAGC ATAAAGAGGA AAATAAATCT TN 392

SEQ ID NO:7431
SEQUENCE LENGTH:127
SEQUENCE TYPE:nucleic acid
TOPOLOGY:linear
CLONE:HUMGS08673
SEQUENCE DESCRIPTION:
GATCTTTGGG AAGGGAGACT AGGGCAGGTG GAGACAGCGC AGAACCCCCG TGCTGGGTGG 60
GAAGCATGAC CACACGGTGG GTGAGCAGCC CCCATGCACT GATGGTAAAT TCCCCTGTGG 120
ACTCAAA 127

SEQ ID NO:7432
SEQUENCE LENGTH:353
SEQUENCE TYPE:nucleic acid
TOPOLOGY:linear
CLONE:HUMGS08674
SEQUENCE DESCRIPTION:
GATCTGTCTG GCATGTCAGG AGCCAGAGAT ATTTTNATAT CAAAAATTGT CCACAAGTCA 60
TTTGTGGAAG TGAATGAAGA GGGAACAGAG GCGGCAGCTG CCACAGCAGG CATCGCAACT 120
TTCTGCATGT TGATGCCCGA AGAAAATTTC ACTGCCGACC ATCCATTCCT TTTCNNNNTT 180
CGGCATAATT CCTCAGGTAG CATCCTATTC TTGGGGAGAT TTTCTTCCCC TTAGAAGAAA 240
GAGACTGTAG CAATACAAAA ATCAAGCTTA GTGCTTTATT ACCTGAGTTT TTAAATAGAG 300
CCAATATGTC TTTATAANCT TTNCCCANTA AAAACCCACT TGTCCCAAAG AAA 353

SEQ ID NO:7433
SEQUENCE LENGTH:399
SEQUENCE TYPE:nucleic acid

TOPOLOGY:linear
CLONE:HUMGS08675
SEQUENCE DESCRIPTION:

```
GATCTAATTG GCTTTTATTA GCAATTCATG AGCCCGGCAG CATCCAGTCT ACAAAATAGA  60
AAGGAGTGCC ACTAAGCTAG ACAGAATGGA TGGGTTTTAT GGCAGAAAAA AGTGGAAGCA 120
AGCAAGAACA ATGAACAAAT TAATGGATGG ATTGTTTTAA GATTACATTT CTTTTATGGG 180
AGTAAACAAA ATCTTGTTAG CCTAATGGAA TTTGGCATCA TCTCCTGATT TCTCAGAAGG 240
TCATATCTTG TAAGTGAACC ATTTAGGTTT GGTGACCTGG NACCTTTGGC ATAANGTGAC 300
TCCATTTTGG GGCCTGCTGC CTTTTTCTTT AAAAANGGAG TGACACCNTA ATTTCCATGG 360
GNNGAGCACA CANNATGCCC TTGTNTTGCA GGACCTNCN                        399
```

SEQ ID NO:7434
SEQUENCE LENGTH:230
SEQUENCE TYPE:nucleic acid
TOPOLOGY:linear
CLONE:HUMGS08676
SEQUENCE DESCRIPTION:

```
GATCCTGACC ATATTCCTCA AGGTCTCCTT TTCCTTCCTC CAGTGTAACA AAATTCCCTG  60
CTGGCGTCCT ATGTAAAGAT AATCGGCCCT TTTTGGACCT TTTGTTGGGN TCAAGAATTT 120
GGGTGGGAGA AAAGAAAGTG GGTTATCAAG GGTGATTTGA AATTTTCTGC AGCATTAAAG 180
CTGGCGCTTA ATAAGAATAA GTAATAATAA AGAAATTTCT AACATTCAAA            230
```

SEQ ID NO:7435
SEQUENCE LENGTH:153
SEQUENCE TYPE:nucleic acid
TOPOLOGY:linear
CLONE:HUMGS08677
SEQUENCE DESCRIPTION:

```
GATCCTGAAA ATAGCCCCGT GAAGGCAGAA ATGTATGTGA CTAGAACGAG GCCACATGAA  60
TAAGCCACTG CCCACTGGCA GGAGTGAAAA CTGAAGCGCT CCTTACCTGA AGGACCCCAA 120
AACCATATAG AATAGAATAA CCAGGAGTTC AAA                              153
```

SEQ ID NO:7436
SEQUENCE LENGTH:138
SEQUENCE TYPE:nucleic acid
TOPOLOGY:linear
CLONE:HUMGS08678
SEQUENCE DESCRIPTION:

```
GATCTCACAA GCNCTTTCCC TCTTGGTGCC TCAGTTTCCT GACCTATGAA ACAGAGAAAA  60
TAAAAGCACT TATTTATTGT TGTTGGAGGC TGCAAANTGT TAGTAGATAT GAGGCATTTG 120
CAGCTGTGCC ATATTAAA                                               138
```

SEQ ID NO:7437
SEQUENCE LENGTH:361
SEQUENCE TYPE:nucleic acid

TOPOLOGY:linear
CLONE:HUMGS08679
SEQUENCE DESCRIPTION:
GATCATGGCT AAAGATGACC TCTCTGGTGC TGACATCAAG GCAATCTGTA CAGAAGCTGG 60
TCTGATGGCC TTAAGAGAAC GTAGAATGAA AGTAACAAAT GAAGACTTCA AAAAATCTAA 120
AGAAAATNTT CTTTATAAGA AACAGGAAGG CACCCCTGAG GGGCTGTATC TCTAATGANC 180
CATGGCTGTC ATCAGGAAAA TGGTTGGGAG ATTTCTCAAT CCCTGAAAGG ATGAGGTTGG 240
GGGAGTTGCC CAGAGGAATC CCTGTTCCCA CTGATTTTTA TTAGCAAAAC ATCCTGTGTC 300
TTTTGGAGTA CGNTGTGTAA GTGCCCATTG GGTGGCCTGT TNGGTCACTG TGCAGCAGTT 360
N 361

SEQ ID NO:7438
SEQUENCE LENGTH:273
SEQUENCE TYPE:nucleic acid
TOPOLOGY:linear
CLONE:HUMGS08680
SEQUENCE DESCRIPTION:
GATCAAAGTG TAAACACCAT AGGGACCCNT TCTACACAGA GCAGGACTGC ACAGCGTCCT 60
GTCCACACCC AGCTCAGCAT TTCCACACCA AGCAGCAACA GCAAATNACG ACCACTGCTA 120
GATGTCTATT CTTGTTGGAG ACATGGGATG ATTATNNNCT GTTCTATTTG TGCTTAGTCC 180
AATTCCTTGC ACATAGTAGG NACCNNGTTC AATTACTATT GNATGAATTA AGAATTGGTT 240
GCCATAAAAA TAAATNNGGT TCATTTAANG TNN 273

SEQ ID NO:7439
SEQUENCE LENGTH:173
SEQUENCE TYPE:nucleic acid
TOPOLOGY:linear
CLONE:HUMGS08681
SEQUENCE DESCRIPTION:
GATCAGAGGA ATGTTATNTT GTTCAGCCTT CTGGTTGGTT CACAGTAGTG ATAGTATTGT 60
AAATACTCAG AGCAATCCTA CAAGAAGAAA ACTACATNAG CCCCCACATG TTCCCCCCAA 120
CATACCCCAT CACAGCAACT GAAAACAAGA GAAATGTTAG TTTTCTGTGG AAA 173

SEQ ID NO:7440
SEQUENCE LENGTH:194
SEQUENCE TYPE:nucleic acid
TOPOLOGY:linear
CLONE:HUMGS08682
SEQUENCE DESCRIPTION:
GATCTCTTGA ACTCTGGGTT CTCCCAGCCC CTTGTCCTTC CTTCCAGCTG AGCCCTGGCC 60
ACACTGGGGC TGCCTTTCTC TNACTCTGTC TTCCCCAAGT AAGGGGGCTC TCTNAGTGCA 120
GGGTCTGATG CTGAGTCCCA CTTAGCTTGG GGTCAGAACC AAGGGGTTTA ATAAATAACC 180
CTTGAAAACT GAAA 194

SEQ ID NO:7441

SEQUENCE LENGTH:132
SEQUENCE TYPE:nucleic acid
TOPOLOGY:linear
CLONE:HUMGS08683
SEQUENCE DESCRIPTION:
GATCANATGA AGAAAAGGAG AGGTAGATAC AGTCAGTGTC ACTTCAGGAA AGCTATTTAA 60
AAAANCTTGA AATATAATTG AAAGAAGAAA CAACACCAAA NAAGCCTAAN CCTAGCCTCT 120
GANCANCACT AN 132

SEQ ID NO:7442
SEQUENCE LENGTH:208
SEQUENCE TYPE:nucleic acid
TOPOLOGY:linear
CLONE:HUMGS08684
SEQUENCE DESCRIPTION:
GATCAATCTG AGGAGACTTA ATAATGATAT TNATTCTCCC AATTCATGAA TATAGTATAC 60
CCCTGTATTT ATTTGTNTTC TTGAATTTCT TTTATCATTG TTTTGTAGTT TTCACCATGA 120
CAGTCTTGCA CATATTTTGT TAAATGTACA GCTGAGAATT TAATTTTTCC TGGTGTACAA 180
TGCTAATAAA ATGGTGCTTT AAAAGAAA 208

SEQ ID NO:7443
SEQUENCE LENGTH:244
SEQUENCE TYPE:nucleic acid
TOPOLOGY:linear
CLONE:HUMGS08685
SEQUENCE DESCRIPTION:
GATCCTCTAA GGTTCTACAG TTTACTTCTT GTATTCTCCT TTGTAAGTCA TCTCCAAGAC 60
GATGTCCAAA TCCATCACCA TTAAAATTAA TAGTTTCCTC ACCCACAACA CTTAATATTT 120
TAAAAAANGA TACTTTTCAT TGTATTATAA TTACTTGATA CATACATATT TGCTCTGTGA 180
GTTCCTTATT CATCATATTA GTGCCTGACA ATAAATGTGT GCTGGATTGA GCTGAATCTT 240
TAAA 244

SEQ ID NO:7444
SEQUENCE LENGTH:323
SEQUENCE TYPE:nucleic acid
TOPOLOGY:linear
CLONE:HUMGS08686
SEQUENCE DESCRIPTION:
GATCCTGCAA TTCCACTTAT AGGAATTGAC CCACAAGAAA TGAAAGCAGG GACTTGAACC 60
CATATTTGTA CACCAATATT CATAGCAGCT TATTCACAAG ACCCAAAAGG CAGAAGCAAC 120
CCAAATGTTC ATCAATGANT GATTGATTGG CTAAGCAAAA TGTGATATGT NCCTAACGAA 180
GTATCCTTCA GCCTGAAAGA GGAATGANGT ACTCATACAT GTTACAACAC GGACGAACCT 240
TGANANCTTT ATGCTAAGTG AAATAAGCCA GACATCANCA GATAANTAGT TTATGNTTCC 300
ACCTACATGN GGTACTGNGA GTN 323

SEQ ID NO:7445
SEQUENCE LENGTH:318
SEQUENCE TYPE:nucleic acid
TOPOLOGY:linear
CLONE:HUMGS08687
SEQUENCE DESCRIPTION:
GATCCTCCTG CCTCATCCTC CCAAAATNCT GGGATTACAG GCATAAGCCA CCGTGCCTGG 60
CCTCTTTAAT AATNTTNAAA ATACCCTAAA GGCTTGTGAA TATACAAGTC TACTGATAAA 120
TTATGTATTG TCTGGGAATT TGATAGTNAT TGTTTTAGAT AACTGGNTTT TACGCTGTGG 180
TAGNCNGGCT GTGACACTAG TGTTGCACAG GTGTAATTGG TCATCCTATG CCTTCACCAG 240
AATAACTTGG GAGTGGTGCC AGAANCTAGA GTCTACAATT CTCACTGTTT AGAGAGTGTT 300
ANTGNCATAC TGTGTATN 318

SEQ ID NO:7446
SEQUENCE LENGTH:317
SEQUENCE TYPE:nucleic acid
TOPOLOGY:linear
CLONE:HUMGS08688
SEQUENCE DESCRIPTION:
GATCAAGATA GTAGTATTAT TACACAAGAA ACTTGGTCTG CAGTCTGGAA GCTTGTCTGC 60
TCTATAGAAA TGAAAATGCA GCATGAAGTT GACATTGTGG AAATGAAAGT AATTGGGTAT 120
TAGAAATCTG AAAGTACTGT CATCTAANAG CAATTGTGAT TTTNTTGTAA TTGGTTGTCA 180
CTGTTGTNCG GTGTCTAGNG TTANNGAATA CATGTNANCT TTCATGGTAT TTNGCCTTTC 240
TTAANTTTTT TTAAAATTTA ANCTTTCTAA CCTATGTATT CAACTTCTGT ATTTATATNT 300
NATCAGTGGT TCATGTN 317

SEQ ID NO:7447
SEQUENCE LENGTH:284
SEQUENCE TYPE:nucleic acid
TOPOLOGY:linear
CLONE:HUMGS08689
SEQUENCE DESCRIPTION:
GATCATCAAA GAAAGACAGG GTGACCTGAG GATTATATGT TCAGCCAAGC TCCCCTCCAA 60
GCATAAAGGC TACAGATAAC CATTTGTACA TATTCAGGAA CTGGAGTAAT GTGATTCCAA 120
TGAGCCTGTC TTGAAGGAAC TCCTAGGGGA TTAACTTCAG CTAACCTGGG AAGTTATCTG 180
GAGAAACCAC AACAGAAGGA CTGGTTGTAG TTCGAGGCTG TAGTCAGCTA TGATTGCACC 240
ACTGCACTCT AGCCTGGATA ATAGAGCAAG ACTTTGTCTC CAAA 284

SEQ ID NO:7448
SEQUENCE LENGTH:324
SEQUENCE TYPE:nucleic acid
TOPOLOGY:linear
CLONE:HUMGS08690
SEQUENCE DESCRIPTION:
GATCCCTAGT ATAACACATT CAGTGTTCCC CTTTCAGTCT TACTACTTTN ACCGCGATGA 60

TGTGGCTTTN AAGAACTTTG CCAAATACTT TCTTCACCAA TCTCATGAGG AGAGGGAACA 120
TGCTGAGAAA CTGATGAAGC TGCAGANCCA ACGAGGTGGC CGANTCTNCC TTCAGGATAT 180
CAGGAAACCA GACTGTGATG ACTGGGAGAG CGGGCTGANT GCAATGGANG TGTGCATTAC 240
ATTTGGAAAA AANTGTGAAT CAGTCACTAC TGGANCTGCA CAANCTNGCC ACTGNCAAAA 300
TTGACCCCCN TTTGTNTGAC TTCN 324

SEQ ID NO:7449
SEQUENCE LENGTH:324
SEQUENCE TYPE:nucleic acid
TOPOLOGY:linear
CLONE:HUMGS08691
SEQUENCE DESCRIPTION:
GATCACCTGA GGTCAGGGGT TCGAGACCAG CCTGGCCAAC ACGGTGAANC CCATCTCTAC 60
TAAAAATGCA AAAATTAGCC GGGCGTGGCG GCACATGCCT GTAATCCCAG CTACATGGGA 120
GGCTGAGGTG GGAGAATTGC TTGAACCCAG GAGGCGGAGG CAGAGGCTNC AGTGACCCAA 180
GATTGTGCCA CTGCACTCCA CCCTGGGCAA CAGAGCAAGA CCCCATCTCA AAAATAAATA 240
ANTATATATA NAAAATAAAA NGCTATTTCT NGNNNNTTTC ACTATAAAGT TTTGCTTTAT 300
TAAAAAGCTA NTANGCAGCT ATTN 324

SEQ ID NO:7450
SEQUENCE LENGTH:326
SEQUENCE TYPE:nucleic acid
TOPOLOGY:linear
CLONE:HUMGS08692
SEQUENCE DESCRIPTION:
GATCTTAAAC TCAGTAAGCC ACTATCTGCA ATTTTGTACA TTATATAGTA TTTTGAAGAT 60
ATGGAACCTT ATGAAAAAAA AATAGCAAAT NAGTTCTTTT TCCCCCAGAG GGGAAAGTTA 120
TGTNCTGCAA ATAGTGTGTG TCTTATTTTA CTGTTGANCA GCAATNGCNN NTTATTTTTT 180
NATTGCCTAG AACTTCAACA TGTTGTATAG GAATCCTGTA GTGCCACTAG TTAAATGCCG 240
AATTCTCATC TGGATGTTAC CATCAAACAT CAGTACACTT GTCATTTCAC ATGTGTTTAA 300
TGTGACAGTT TTTCAGTACT GTNTNN 326

SEQ ID NO:7451
SEQUENCE LENGTH:38
SEQUENCE TYPE:nucleic acid
TOPOLOGY:linear
CLONE:HUMGS08693
SEQUENCE DESCRIPTION:
GATCTNGTGG GCATAAATAA AGGTGTCATA AAGACAAA 38

SEQ ID NO:7452
SEQUENCE LENGTH:83
SEQUENCE TYPE:nucleic acid
TOPOLOGY:linear
CLONE:HUMGS08694

SEQUENCE DESCRIPTION:
GATCTTAAGA AGTGCTTTGA AGGGTTAAGA ATCAGGGGTC CAAGAGAGAC CCCAGTCCCT 60
CAATAAAGCC ACAAGAGCCC AAA 83

SEQ ID NO:7453
SEQUENCE LENGTH:351
SEQUENCE TYPE:nucleic acid
TOPOLOGY:linear
CLONE:HUMGS08695
SEQUENCE DESCRIPTION:
GATCTACACT CCAGGCCGGA AAGAGCAAGG GGAGCCCATG ACCCCTCGCC GCACGCCAGC 60
CCGCTTTGGT CTGTAAGCTG AGGCTGTCCC CAGAGAGGAT GGCAGCAGGT ATTGGGTCCT 120
CAGCCTTCTG GCGGGAGCCT GAGGCTGCGG ACAAAGCCCT TTAATCTAAG GACTTTAATC 180
TGTNCATATN ACGGCCCCCC AGGGCAGTTC CTGCTGGACC AGACTCTNTG GCAGAGGAGG 240
TGGAGTTCTT CCATGCAGGA GCACGNCATG GCGGGAGCGG GGCTGCAGAG TATCCGNGGT 300
GCTGCCGNGG CACGGNGAGG TNGCTNGACC CATCGCATCT AAAANTAGGA N 351

SEQ ID NO:7454
SEQUENCE LENGTH:55
SEQUENCE TYPE:nucleic acid
TOPOLOGY:linear
CLONE:HUMGS08696
SEQUENCE DESCRIPTION:
GATCTAAGGT TTTAAAAGAA TTTGGTTTGA AAATATACAG TTGTCTTGAA GGAAA 55

SEQ ID NO:7455
SEQUENCE LENGTH:61
SEQUENCE TYPE:nucleic acid
TOPOLOGY:linear
CLONE:HUMGS08697
SEQUENCE DESCRIPTION:
GATCTGTTCT GTTTCACCAT GTAACACACA ATACATGCAT GCACTGTATT AGTGTTAGAA 60
A 61

SEQ ID NO:7456
SEQUENCE LENGTH:273
SEQUENCE TYPE:nucleic acid
TOPOLOGY:linear
CLONE:HUMGS08698
SEQUENCE DESCRIPTION:
GATCCCATTT TCCTCTGACG TCCACCTCCT ACCCCATAGG AGTTAGAAGT TAGGGTTTAG 60
GCATCATTTT GAGAATGCTG ACACTTTTTC AGGGCTGTGA TTGAGTGAGG GCATGGGTAA 120
AAATATTTCT TTAAAAGAAG GATGAACAAT TATATTTATA TTTCAGGTTA TATCCAATAG 180
TAGAGTTGGC TTTTNTTTTT TTTTGGNCCA NGGGGGGGGG TTTTTTCCCC NTGCCCCCCT 240
GGGGTTTTTN NANTCCCNGT NNNAAAANAA ANN 273

SEQ ID NO:7457
SEQUENCE LENGTH:363
SEQUENCE TYPE:nucleic acid
TOPOLOGY:linear
CLONE:HUMGS08699
SEQUENCE DESCRIPTION:

```
GATCTTAACC AGTCGAGTGG AGTGTACATT GTCTGAATAC AGGNTGCACA ATGTTGTCAA  60
TCCTGGAAAT GGTCTTTCTT TTTTGTAAGA TATGTGAATG AAGTGTTGGT GTCCTCACCA 120
AGAGGTGGCA CCTAAGGGTT CTGAGGAAAT AAATGTATAG ACCCTTATGT ACAGACCTGT 180
GTATAAACAA CTNTTGTATA TACATATAGG ATAGCTTTTT TGTACTATAC AGCTGGTACC 240
ATAANAGTAG CTGGATANTT TGGTTAGGNC NGGTTGTCCA CCAGTTTTGG NNTTTTTTTT 300
CACTTTGNTN CATTTTGGGG GATTTTCCTT GTGGGTTGNN TTAAAANTTG CATATGCTTA 360
NGN                                                               363
```

SEQ ID NO:7458
SEQUENCE LENGTH:173
SEQUENCE TYPE:nucleic acid
TOPOLOGY:linear
CLONE:HUMGS08700
SEQUENCE DESCRIPTION:

```
GATCCGTATT TCCAATAAAA TCCCCTACCT GAATATTCAA GTTAAACATG TCCAGAATAC  60
TTACTGATTT TATTGTTAAT AGCCACTATT CTGTTGTCTG GAATTAAAAC CTGTATAACT 120
AATTTGCATC CCTTTATCTT CTTAGTCAAT AAAACCTACA ATCCTCTTTC AAA        173
```

SEQ ID NO:7459
SEQUENCE LENGTH:349
SEQUENCE TYPE:nucleic acid
TOPOLOGY:linear
CLONE:HUMGS08701
SEQUENCE DESCRIPTION:

```
GATCACCCCC CAACTTGCCC TTCAAGTTCT ACTTCAGTTT GATAAGGCTA TAAATGCAGC  60
ACTGGCTCAG AGGGTCAGGA ACAGAGTCAA TTTCAGGGGC TCTCTAAATA CGTACAGATT 120
CTGCGATAAT GTGTGGACTT TTGTACTGAA TGATGTTGAA TTCAGAGAGG TGACAGAACT 180
TATTAAAGTG GATAAAGTGA AAATTGTAGC CTGTGATGGT AAAAATACTG GCTCCAATAC 240
TACAGANTGG ATAGAAAANN TATGACTNNT TTACACCATC TTCCTGTTAT TCATTGCTTT 300
TTGAAGAGNA GCATAGANGA GACTTTNTTN TTTTATNCTN GGAATTTTN             349
```

SEQ ID NO:7460
SEQUENCE LENGTH:395
SEQUENCE TYPE:nucleic acid
TOPOLOGY:linear
CLONE:HUMGS08702
SEQUENCE DESCRIPTION:

```
GATCTGGAGT AAGGAATATT GTCATCACCT GGATTTTGAG AAAGAAAAAT AACTTCTCTG  60
```

CAAGATTTCA TAATTGAGAG AATTCCTGAG TTGATAGCTC TAAAGGCAGA TATGCTGTAT 120
TTGCCTACTT TAACCCATTT TTCAACCTGT TTGTTTTTTA ANNGGNTTCA CTAAGGGTTG 180
ATATGTACCA TTGTATGGGG CAATTTTAAG TCAGCTAAGG CAATAACCTT ATGCATGAGC 240
ATTTCCCAGA CTTTCATGAA GCTGTTGAGG TCCTAGGCAA TTAATGCAGC AGTTGCGATA 300
AATAAANGNC NTCTCACCTA AGNNTCCNTT TTCTTCNATA ACCATGGTTC CTGGNCATGN 360
TTGGGAGGCT NCTCGGCTTT AGGGNAANGG GGTTN 395

SEQ ID NO:7461
SEQUENCE LENGTH:347
SEQUENCE TYPE:nucleic acid
TOPOLOGY:linear
CLONE:HUMGS08703
SEQUENCE DESCRIPTION:
GATCGCCTCC CAACAGCAGA TTTCGACATT ACCTGAGAGT CTTGATTTTA GGCTTGTTTT 60
TTGTAAACCC ATGTGTTTGT AGAGATTTTA GGCGTCTTCG GATATCTTCT CACCTATGTT 120
CCCTGGCTAA GAAGTCAGAG GTAGCCAATG TTTCCTTAAA TTCATTTTTA AACTTACCAT 180
TGGTGCATAT GTTCCAGATG GCAGATGCTG TCAATAATCT CACCATTGAT GACCTTTGTG 240
TATGTAGTTC TTGCATCCTA TACTGGATAA GCCTGTTTTA ACCTGCTATG ATGGGTGCTT 300
TCCATTGCTT CNTAATCTTC ATGGAGGGTT GCCATGCTTT TTGCCNN 347

SEQ ID NO:7462
SEQUENCE LENGTH:274
SEQUENCE TYPE:nucleic acid
TOPOLOGY:linear
CLONE:HUMGS08704
SEQUENCE DESCRIPTION:
GATCGGNATG TAATAACAGC ATTAACTCAC AGACCTTGGA GCCTAAGCCA TACAGGAGAT 60
GGGAAACCAC GCTATGATAC TTNCTGGAAA CATTTTATAT TTNTAATGAT GGACATTTTN 120
CTCGATTGGA GCATGCATAA TANCTTGTGG TACCTGTGTG GAATTTCANC TTTCCTCATG 180
CAAAAGGATT TTNTATCCCC GGCCTACTTG ANGAAGTGGT CAGCTAANGG NNTCCAGGTT 240
GTTGGTTGGA CTGTAATACC NTTTGATGAN AAGN 274

SEQ ID NO:7463
SEQUENCE LENGTH:69
SEQUENCE TYPE:nucleic acid
TOPOLOGY:linear
CLONE:HUMGS08705
SEQUENCE DESCRIPTION:
GATCAGAAAC TTGCATATGC GTGAAAAAAG GAAGAGCAGC AGAAAATAAA TAAATGACAG 60
GAAAGCAAA 69

SEQ ID NO:7464
SEQUENCE LENGTH:199
SEQUENCE TYPE:nucleic acid
TOPOLOGY:linear

CLONE:HUMGS08706
SEQUENCE DESCRIPTION:
GATCTATAGC AATGTGATTT TATTTTTAAA AAGAAAAGCA GTGTGTTTTC TTTTTTTGTT 60
GTTTTCTTTT GCTTAAGCAC TTCATCAATT GCTTTATTCT GTATCTGCGA AGTNATCTGC 120
AATCTCTNTT GNNCTTTNTA AAATTTGGAT NTGTTATAAA AATTGNCCAN ATTGGAAGGT 180
GTTTCAGTTA AAAAAANAN 199

SEQ ID NO:7465
SEQUENCE LENGTH:320
SEQUENCE TYPE:nucleic acid
TOPOLOGY:linear
CLONE:HUMGS08707
SEQUENCE DESCRIPTION:
GATCACAAAC TTGGAGGGCT GTATGGTTTT TCATTTAAGA AAGTAATTGT CGTGCAATAA 60
AGTTATTAAG CCTTGTTTAA GAATATTTTT ATTTTGTACA TCAAGTGGAG TTGGAAATAG 120
TTGGTTTCAT AATGATATAT TTCAAAAGTA TCTTGAAGTC ATCTGGTTCA TTTCACTACC 180
CAGGGCACAC ATTCCTCCTG TAACATCCCA AGTGGTTATT AAGCAAAACA GATTTATTTT 240
ATTTAGAAAA NNTTAGTATG ACTGATTTGC TATTGATTTT GTACTTCTAT AAATNAATGT 300
GGATACATGC TATATTCAAA 320

SEQ ID NO:7466
SEQUENCE LENGTH:311
SEQUENCE TYPE:nucleic acid
TOPOLOGY:linear
CLONE:HUMGS08708
SEQUENCE DESCRIPTION:
GATCACTTAC AGGTGCCCTC TCCCCCAACA GCCAGAAAGG GAAAACAGAG CCGAGTTTGG 60
CCACTGGGGC CTCCACAGCC CTCTGGAAGA AGGGGCTGAC TGATAGGAAG GCACTCCTCT 120
GGTGTTCACT GGCCAGACCC CTGTGGGCTC AGAAGACTAC CTAGACCCCA TGAAGAAAGA 180
CTCTAGACAC ATTTTTATAA TCAGAAACTT ACAGTTTATT GTGAATAAAC CACTCAACTT 240
TGAGCAGATG ACATTGCCTC ANTTAACTTT TTCGTTGAAG AAAAACAAAG ATATATGGTA 300
AATTCATGAA A 311

SEQ ID NO:7467
SEQUENCE LENGTH:434
SEQUENCE TYPE:nucleic acid
TOPOLOGY:linear
CLONE:HUMGS08709
SEQUENCE DESCRIPTION:
GATCATCAAC AAAGACTTGT TAGAAAGGTC TAGTCTTAGC ACTTGGCAGT TAATCTAGGG 60
AAGATGAATT AAATGGGTAG ATAGTGATGC ATACCTGTAT TCACTGATGT ATGTTTAAGG 120
GATTTGGGGG GGATACCTCA GTTCATGTGG AAGGGACAGT CTCGGTGTGT CCCATGAATA 180
ACCTTGGAAC TGCAACAAAT GGTTTGTGCT CAGAAAAAGT CTTTCATGGT GACAGGAAGA 240
CAGTTTCCCT GGAGCTGGCC ATGAAGGCCT TAGAAGCCAT TTCTGGTGTC TGGTGGGTAG 300
CAGGCATAGA GATGATGTNC CGAGGTCCCA GTGAACACCA GTAGCCAAAN GAATTGTACT 360

ACCTTNTATC CATTANTTNG GGGAAGGTCC TTCCCTNGGG NAACCAATGN CCTTTTTTGN 420
TGGGCCAAAA TTTN 434

SEQ ID NO:7468
SEQUENCE LENGTH:414
SEQUENCE TYPE:nucleic acid
TOPOLOGY:linear
CLONE:HUMGS08710
SEQUENCE DESCRIPTION:
GATCGAGCTG TGGACTTATA CCTGTGTGCN TGGTTTACAG GTCCAGTGGT TCTTCAGCCC 60
ATGACAGATG AGAAGGGGCT ATATTGAAGG GCAAAGAGGA ACTGTTGTTT GAATTTTCCT 120
GAGAGCCTGG CTTAGTGCTG GGCCTTCTCT TAAACCTCAT TACAATNAGG TTAGTACTTT 180
TAGTCCCTGT TTTACAGGGG TTAGAATAGA CTGTTAAGGG GCAACTGAGA AAGAACAGAG 240
AAGTGACAGC TAGGGGTTGA GAGGGGCCAG AAAAACATGA NTGCAGGCAG ATTTCGTGAA 300
ATCTGCCACC ACTTTATANC CAGATGGTTC CTTTCACAAC CCTGGGTCAA AAAGAGAATA 360
NTTTGGCCTA TNATGTTANA AGGAANGCAG GNNGGTGGGT TNAANTAAAA NTTN 414

SEQ ID NO:7469
SEQUENCE LENGTH:214
SEQUENCE TYPE:nucleic acid
TOPOLOGY:linear
CLONE:HUMGS08711
SEQUENCE DESCRIPTION:
GATCAGTATT TTTAATCAAC ACCTGAGAAC TGTTACACCT TTNATTTTGT CTTTNAGGAA 60
ATCCCTGTCT TTCCATTTTT TCATGTAAAT NTTGCACAGT TACTTGTTCA TATGTAAATA 120
TTTTACTTTC AGAAATNAAG TTTTTAATTG CTATTGTTTT ATATAGGATT GAAAGAAAAT 180
NAACTCCTTT ATTAAAAACA AATTTATCTG TAAA 214

SEQ ID NO:7470
SEQUENCE LENGTH:418
SEQUENCE TYPE:nucleic acid
TOPOLOGY:linear
CLONE:HUMGS08712
SEQUENCE DESCRIPTION:
GATCCATTTC CTCAGGACTC AGTTAGGTGT GCCCAGACGC ACTGACAAAA TNACGTGACT 60
TCAGGGAAGC CTGGACACCC GAGGCACCTG GACCAGCTAT GGGTAGTTCT GTGGGTGGAA 120
CACATTCTGT GTAAGAGCCC CACTGAGGGC TCTGCAGCGG AGTGACAGCA ACCCCAGAGA 180
TGAGGCACCA GAGAGTGCCA CTGCATGAGA CACCTGTGAC CATTCGAAGT CTGAAATGCG 240
GGGGGGGAGT TTCATTTTTA AGTGAAGACC AAAAGCCCTT TAAAAATAAT AGTTTTTTAT 300
CATTTTATAG TAATCAGCGG NNTCTCTTTT ACTAATATAC TCATTCCTNT TGCGTATGAT 360
TGGNCTTTTG GAGCTACATG CTCCNACAAN NCTGTATCTT AATTGGTTAA CTTGTTAN 418

SEQ ID NO:7471
SEQUENCE LENGTH:422
SEQUENCE TYPE:nucleic acid

TOPOLOGY:linear
CLONE:HUMGS08713
SEQUENCE DESCRIPTION:
GATCTTGGCG TTGTGGTAGA TTGCCTTCCT GTGCTCACCA ATTGTTTACA GGAAGAAAAA 60
CAATATATCT CACTTGGCTG CTGTGTTGAC TTGTTGCCTC TAGTAAAGTC ACTACTTAAA 120
AGCAAATTTN AAGAATATGT TATAGTTGGT TTAAACTGGC TTCAAGCAGT CATTAAAAGG 180
TGGTGGTCAG AACTATCATC CAAAACAGGA ATTATAAATG ATGGAAATAT TCAAATTTTA 240
AAACAACAAT TAAGTGGATT ATGGGTACAG GANAACCATC TTACTTTGGT TCCAGGATAT 300
ACTGGTAATN TAGCTANGGG TGTAGNTGCT TNTTTTCTTT CCAGTTCCNT TGNGGGATTT 360
CCTCTNCCTG NGGGGCATTT TGGTTTTTCA ANACCNTCCC NTGGACCTGT TTTANTTTTC 420
CN 422

SEQ ID NO:7472
SEQUENCE LENGTH:359
SEQUENCE TYPE:nucleic acid
TOPOLOGY:linear
CLONE:HUMGS08714
SEQUENCE DESCRIPTION:
GATCGCCTTA ACTACTGTAA ACATAGCCTA TTTTTGTGCT TAANATACTG AATGGAAAAC 60
TCCATTGTGT GTTGCTGGAC TGTTTTGGAA ATATNNNGTT AAATGTGTGT TAATTTGGCT 120
GTAATGGCAT TTAAAGCAAA CAAACAAACA AAAAAAGCTG TGAAAATGGC CTTGGAGCAT 180
TATCTTTAGT TACTTGAAGA GTTTCTAGTT TTTTTAAAAT ACAGTTTATG TTAAAATAAT 240
TTTANTTAAT TTAGAGANGC CAATCATTGT CTGNGGGGAA ACCGGCCTTN CNTTTTGGGN 300
TTNCNTTTTN GTGGGCCATT GGTGAGGTGA GTGCCTNTTN CCCGTTTCNT TNGGTTTCN 359

SEQ ID NO:7473
SEQUENCE LENGTH:105
SEQUENCE TYPE:nucleic acid
TOPOLOGY:linear
CLONE:HUMGS08715
SEQUENCE DESCRIPTION:
GATCTTTAAA GTTATTTTTA GGCACATTTG TGACAAACAA CTACTTGATA TTGAAATCTC 60
CNTCAGCCAT TAGAAGCTAT CAAATAAAGT AGGTGTAAAA ACAAA 105

SEQ ID NO:7474
SEQUENCE LENGTH:104
SEQUENCE TYPE:nucleic acid
TOPOLOGY:linear
CLONE:HUMGS08716
SEQUENCE DESCRIPTION:
GATCAAGTAT GCTAATNTTA AGCAAATGTA AAAACTCATA AAACAGGTAA ACAGTGGGGT 60
GATTTCATTT GCCATAATTC ACATAAGACG AATTTTAATC TAAA 104

SEQ ID NO:7475
SEQUENCE LENGTH:340

SEQUENCE TYPE:nucleic acid
TOPOLOGY:linear
CLONE:HUMGS08717
SEQUENCE DESCRIPTION:
GATCCTTGTT TTTAAGTTAC TTTCGATGTG TATACGAACA AAGACCAGGG AGAAACAGAA 60
CTTTTAAAAC TATAGGCTGC TGTNTTGGGG CCAGAAATAA GGTGACAAGT AAAGACTTTT 120
ATAATACCTA CCTTCAAANT TAGAATCAGC AGCACTGTAC AANTAAAGTG CCTGTGCTTC 180
ACCTCACACC AAGANTCTTG CCTAACCCTG TCAGAGTGCC TAATATCCTG TGGTGATTAT 240
GTATAATGAA ACAGTCTTAT TTTGTGCTCT TTGGTTTCTT AAAAGGAAAG CTTGTTTTCT 300
TTCCTTTATC ATTACAGAGG TATGTCTTTG GTTCTTANTN 340

SEQ ID NO:7476
SEQUENCE LENGTH:189
SEQUENCE TYPE:nucleic acid
TOPOLOGY:linear
CLONE:HUMGS08718
SEQUENCE DESCRIPTION:
GATCTGTGAA AAAATACAGT ATTGTAAATC CAAGACTCTG ATACTGAATT TAAGTTATTT 60
CACAGTTGTA GCTACACAGT AAGTAGCTTG GTAGATAGTT ATTGANTGTA TTTATGTAGT 120
GTATTAAGAA GCTTATATTA CTACAAAAAA CTTATTTTAA TATATNTNNN TATNTNNGTN 180
TTATTTATN 189

SEQ ID NO:7477
SEQUENCE LENGTH:342
SEQUENCE TYPE:nucleic acid
TOPOLOGY:linear
CLONE:HUMGS08719
SEQUENCE DESCRIPTION:
GATCTGTGTG ACAAAATNAT TTCTTAGCTA TAATCAGCAC ATCACTTAGT TCAAACAAAA 60
TTCCCCAGCA AATGTTAGAT AGTAGGTATA TCAGTCACCT GGGGAGTTTT CTTCATAATA 120
TGCATATTCA TCTNGTAATG CATACATAGT TATCATCCTC CTTCTCAACC CATCTCCCTA 180
ACCCCACATG CTTGCCAGTT CTTGAAGGGA TAAAGTGATT GTAATAGTGT TTNACTNCTC 240
TCTGTTCAAT TTANTGTGAT ATAATTCTAG TNTAANNNTA TTTNGGACAG TNGCTTAACC 300
ATGGTCATAA GAGGATTTGT ACTATAGATT ATCTTCNGGG GN 342

SEQ ID NO:7478
SEQUENCE LENGTH:54
SEQUENCE TYPE:nucleic acid
TOPOLOGY:linear
CLONE:HUMGS08720
SEQUENCE DESCRIPTION:
GATCTTTGTC AACCAAAACA TGTATTTTCA ATAAATTGAG ATTCCCAGTT CAAA 54

SEQ ID NO:7479
SEQUENCE LENGTH:79

SEQUENCE TYPE:nucleic acid
TOPOLOGY:linear
CLONE:HUMGS08721
SEQUENCE DESCRIPTION:
GATCTGTTTC CCTTTCACAT TTTCCCCATT TTGTGAGCAT TTCCTTACTT CCTAACATGA 60
CAAAATATTT CAAACTAAA 79

SEQ ID NO:7480
SEQUENCE LENGTH:281
SEQUENCE TYPE:nucleic acid
TOPOLOGY:linear
CLONE:HUMGS08722
SEQUENCE DESCRIPTION:
GATCCCCCTG GCCTGGAGGC AGCAGCTGTA TAAACTCTAC GAGGCCGACT TTGTTCTCTT 60
CGGCTACCCC AAGCCCGAAA ACCTCCTCCG AGACTGAAAG CTTTCGCGTT GCTTTTTCTC 120
GCGTGCCTGG AACCTGACGC ACGCGCACTC CAGTTTTTTT ATGACCTACG ATTTTGCAAT 180
CTGGGCTTCT TGTTCACTCC ACTGCCTCTA TCCATTGAGT ACTGTATCGA TATTGTTTTT 240
TAAGATTAAT ATATTTCAGG TATTTAATAC GAAATGTGAA A 281

SEQ ID NO:7481
SEQUENCE LENGTH:326
SEQUENCE TYPE:nucleic acid
TOPOLOGY:linear
CLONE:HUMGS08723
SEQUENCE DESCRIPTION:
GATCTGAGTC CAGGCAGTGT TTTTAAGNGA TATGAGTTAG TAGTGAAAAT TGAAAAGGAT 60
ATTTTACAAC CAAATACAGG TTTNNTGGTG TNTTTTGAAA AATCCAAAGA TTTGGCAACT 120
CGACCTAGGC TCCGACAAAA CCACAGTTGT TGGGAGCTGA GCCGGAGCCA GAACTCACAA 180
TTCACCGTGG CCCGGCCACC CATCCCCACC ACTCCCGTGG GCTCCTGACC AAGCACCACG 240
TGCCATTCAT CATGGCTTGC TTTTAGTGTT TTTGGTGTTT ATCTTAAAGT ACAGCGAAGA 300
AGAAAGGGAA GTATTTNTTA GACCCN 326

SEQ ID NO:7482
SEQUENCE LENGTH:331
SEQUENCE TYPE:nucleic acid
TOPOLOGY:linear
CLONE:HUMGS08724
SEQUENCE DESCRIPTION:
GATCTGGATT TGGTAAAGGT TGACTTAATT GAACTCTCTG AAAAATGCTG TAGTGACTTT 60
AATTTGCACT CAGAATTAGA GCGCTCATTT TTGTCAGANC CATCATCTCC AGGAAGAACC 120
AAGACTACTA AAGGATTCAA ACTTGGGAAG CACAAGCATG AGACCTTTAT AACGTCAAGT 180
GGAAAATCTG ANTACATTGA NCCTGCCAAA AGAGCTCATG TTGTGCCACC ACCAAGAGGA 240
AGGGGCAGGG GTGGNTTTGG ACAGGGTATA CGACCTCATG ATATTTTNNG TCAGNGNAAC 300
CAGNNCACAN GTNGACCACC ATCTNTGCNT N 331

SEQ ID NO:7483
SEQUENCE LENGTH:338
SEQUENCE TYPE:nucleic acid
TOPOLOGY:linear
CLONE:HUMGS08725
SEQUENCE DESCRIPTION:
GATCGGAGTC GCCAGGCAGT ATATCCAGTT AGAACTTCCG GGCTTTTGCA TTAGCTTGTC 60
TGATGCTCAT GCCCCACTCA GAGAAAAGAC ATCAGCAAAT TAAGAATTTT CTGGGTTCCT 120
GTGACCCTCA GGTTATTTTA AAGCAATTGG AAGAGCATAT GAACACGGGC CAGCTAGCAG 180
GATTTTCACA TCAAATTAGA AGTCTGATTT TGANTAATAT CATCAATAAG AAGGAGTTTG 240
GGATTTTGGC AAAGACCAAA TACTTTCAAA TGTTGAAGAT GCATGCGATG AATACCAACA 300
ATATCACTGA GCTAGTGAAC TATTTGGCAA ATGACTTN 338

SEQ ID NO:7484
SEQUENCE LENGTH:338
SEQUENCE TYPE:nucleic acid
TOPOLOGY:linear
CLONE:HUMGS08726
SEQUENCE DESCRIPTION:
GATCCGCCCT CCTTAGCCTC CCAATCCTCT CTTAAAAAAG TGATAGCTCA GAAATATTTG 60
TAAAAGCAAG GTTTTTATTT CATTTTGGCT CTGTCATTTT CAGAGGCAAA GAAGTTGGCC 120
TGTAAAATAG AGTGCTAGAG CTCTTACGCC CCTCCCCTTC TTCCCAACTT CCTACTTCCT 180
AGCCCTTTTA TCAACTCCTA GAATAGTTAA AGAGAGACAC ATCTAGATGG GATGAAAGGT 240
GCCCTAAGCA GGAGAACCTG ANCAAAAGGC TAGAGGCATG GGCCAGGTAA AATTTGGGGC 300
CTAGAGTGAA GNCTGTGCTG TCGTTAAGNG CTTTCGAN 338

SEQ ID NO:7485
SEQUENCE LENGTH:338
SEQUENCE TYPE:nucleic acid
TOPOLOGY:linear
CLONE:HUMGS08727
SEQUENCE DESCRIPTION:
GATCACAGTG ACAAAGAAGA TGAACAGCCT CAAGTGGTGG TTTTAAAAAA GGGAGACCTG 60
TCAGTTGAAG AAGTCATGAA AATTAAAGCA GAAATAAAGG CTGCCAAAGC AGATGAAGAA 120
CCAACTCCAG CCGATGGAAG ANTCATATAT CGAAAACCAG TCAAGCATCC CTCAGATGAA 180
AANTATTCAG GTTTAACAGC AAGCTCAAAA AAGAAGAAGC CAAATGAAGA TGAAGTAAAT 240
CAGGACTCGG TCAAAAAGAN CTCACAAAAN CAANTTAANA TTAGTAGCCT CCTTTCTTTT 300
GACAACGAGG ATGAAAATGA GTAAGTGTAA TTNTTTTN 338

SEQ ID NO:7486
SEQUENCE LENGTH:302
SEQUENCE TYPE:nucleic acid
TOPOLOGY:linear
CLONE:HUMGS08728
SEQUENCE DESCRIPTION:

GATCTTAATT CATATCTTGA AGATAAAGTC TACCTTACAG GGTATAACTT TACATTAGCA 60
GATATACTAT TGTACTATGG ACTTCATCGC TTTATAGTGA GTATTTGTAT AGTTACTGGC 120
TTTTTTTCTG TAGTGTTGAG AATGATTTTT AAAACTTTGT CTTGAATTTA AATCAGATTT 180
GCATTTTTGG CTTATCAAAT AAAAATGAAA GGAATTCCTT TATACAAGGT GCCTTTTGTA 240
ACAAACCTGC ACGTTGTGAC ATGTACCCTA AAACTTAAAG TATAATANTA ATAANNTTNA 300
AA 302

SEQ ID NO:7487
SEQUENCE LENGTH:334
SEQUENCE TYPE:nucleic acid
TOPOLOGY:linear
CLONE:HUMGS08729
SEQUENCE DESCRIPTION:
GATCAAAAGT GAATAATGTA TGAATGAGAG CTGTAAGAAG GATTTTNATT TTGTTATAAT 60
TTAGTTACCA TTTTCAGTGT TATTTCAAAG GTTCTTTGAA GAATTTTGGG GCAGGGCATC 120
AGATTAGAGT TTTAAAATTT GAGTATTTTG GATATCAGTG TTCCTCATGA AGATATACAT 180
GGATATNCAA TTTTGATGGC TTCCAGATTT GTAAGATTTT ATGTTGTATA TACCATTCTA 240
TTAAGAAACA TGTCCACTGT GCTTTCAAAC ATAGATAAAG CATGATAAAG ATTATNATTT 300
AAGATATACT TGTATTTATA CCTCAGATAT TCTN 334

SEQ ID NO:7488
SEQUENCE LENGTH:337
SEQUENCE TYPE:nucleic acid
TOPOLOGY:linear
CLONE:HUMGS08730
SEQUENCE DESCRIPTION:
GATCAGCTGG ATACTCNTGT NACCAGAATT CCAAAGGTTC TGATAGAATC AAAGATGGAT 60
ACAAAGTGAA CTCACACATA GCTAAGCTGC AAGAGTTATG GAAAACTCCC CAAAATCAAA 120
CAATCCACCT CTCTAANTCA NTGATGGAGG CGTCCTTTTT CAAGCATCCA GACCTCACCA 180
CAGGCCAGAN GCGTTACCNG TGCAGCATTG CTAAANTCTA TAATGCAAAC TATCTGANGA 240
TGTTAATGNA GNGGCAGTAC ATGCACGTNC TTCAGCACAG CTCACAAAAG CCAGGTGTCC 300
TCACTCATCA CAGNNGCCGC CTTNGCTCCC GTTTATN 337

SEQ ID NO:7489
SEQUENCE LENGTH:292
SEQUENCE TYPE:nucleic acid
TOPOLOGY:linear
CLONE:HUMGS08731
SEQUENCE DESCRIPTION:
GATCATTGAG AACACTGNAC ACAGCAGCTT CAGGCAAAGG GAAAAGAACT AAATGAATTC 60
CGGGAAAAGC ACAACATTCG TCTCATGGGA GAAGATGAGA AGCCAGCAGC CAAGGAAAAC 120
TCAGAAGGGG CTGGGGCTAA GGCCAGCTCA GCTGGAGTGT TGGTCTCCTA GGGACCAAGG 180
CCTTTGCATT TTTTCTACC CTGACTCCCA CTTCTAATTT CTCTTTATTG TTATTATTAT 240
TATTTTCTCT GCTATTGTAA TATTTTTTTG TTAATTAAAT GTTTTGGTTA AA 292

SEQ ID NO:7490
SEQUENCE LENGTH:140
SEQUENCE TYPE:nucleic acid
TOPOLOGY:linear
CLONE:HUMGS08732
SEQUENCE DESCRIPTION:
GATCAACTGC AAACAGGCAC GAAGGAACTT TTTTGGGTGA TGAAAAATAA AACTTTGGAT 60
GTCGAACATA TTTTGAAACT AGATTGCAAT NATAGTTGCA TAACTNTATA CATTTATTAA 120
AATCGTGGAA CACTTACAAA 140

SEQ ID NO:7491
SEQUENCE LENGTH:337
SEQUENCE TYPE:nucleic acid
TOPOLOGY:linear
CLONE:HUMGS08733
SEQUENCE DESCRIPTION:
GATCCGGTCT CCCCCGCCCC CCGGCTGCAG GGTGGATAAA GAGGAAGATG TGAAGAGGAG 60
GCAGACAAAA GCAAAAGGAC ATCCTGTCAG TAGGAAACGG CCGAATTACA ATTCAGATGT 120
GCAATGTTTG CATAAGTAAA CTGACACGGG ACTATTATAT GCAGAANTCC ACGTGCAAAA 180
NTTTTCAGAC ACACCTTTGT ACCTCTNCCT AATCCTGCCC TGGTAAAATG GCGGTTATCA 240
GTGCAAGTTT GAGGATACAT ACAGCTGGAA ANTGGNCATC ACCAAACTGG ACTCCAGAAA 300
GTNCTGTCTA GGATTTTATT AGACTGCTTT TTTNCAN 337

SEQ ID NO:7492
SEQUENCE LENGTH:337
SEQUENCE TYPE:nucleic acid
TOPOLOGY:linear
CLONE:HUMGS08734
SEQUENCE DESCRIPTION:
GATCTATTTC TCAGTAATNA GTGTGTGCCT CATGCCTGTT TCAATATTGG GTTTTGGAGA 60
CANTATTGTA CCAGGCCTGT TGATTGCATA CTGTAGAAGA TTTGATGTTC AGACTGGTTC 120
TCCTTACATA TACTATGTTT CGTCTACAGT TGCCTATGCT ATTGGCATGA TACTTACATT 180
TNTNGTCCTG GTGCTGATGA AAAAGGGGCA ACCTGCTCTC CTCTATTTAG TACCTTGCAC 240
ACTTATTACT GCCTCANTTG TTGCCTGGAG ACGTAAGGAA ATGNAAAAGT TCTGGAAAGG 300
TAACAGCTAT CAGATGATGG ACCATTTGGN TTGTCAN 337

SEQ ID NO:7493
SEQUENCE LENGTH:75
SEQUENCE TYPE:nucleic acid
TOPOLOGY:linear
CLONE:HUMGS08735
SEQUENCE DESCRIPTION:
GATCGGCCTG CGAAAGGATA CTGTGAAATN ACTAATTAAC TAATAAACCT GTCTCAAGTT 60
GAGGATTTGA AGAAA 75

SEQ ID NO:7494
SEQUENCE LENGTH:343
SEQUENCE TYPE:nucleic acid
TOPOLOGY:linear
CLONE:HUMGS08736
SEQUENCE DESCRIPTION:
GATCTGGGGT ACTTGGCAGG GCTTCCCTCA GACAATACCT TAGAGAGTCA CAACCATTCC 60
AGGGTCTGGT CTACCTCACA TCCTCATTCA TTGTTGGATG TGCTTAGCAT AGCCAGAGGT 120
GTCTTCTCTT TGACAGACTT GCTATCTGAA AAATAAACCA GGACACCAAT TTTTTAGCAT 180
ATAATAGAGG ACATCCCTTC CCAGCTATAG GAATGCTTGG TATCCTTCCA AGAACTCCTC 240
CTTTAGAGGA GCCTCACAGA AGTTGCTGGG TAAACATGGG CTCAAACCAA GGAAAGTCAG 300
GTAACCTGGG TGGNGGTTAT CCCACCACCA ATGGCAGGGG GTN 343

SEQ ID NO:7495
SEQUENCE LENGTH:95
SEQUENCE TYPE:nucleic acid
TOPOLOGY:linear
CLONE:HUMGS08737
SEQUENCE DESCRIPTION:
GATCAATATT GTAAAGAATT TATTCTGATA AATNAGAAAC TGGATATAAT GTCAAAATAG 60
CTATTTTCTC AATAAAAATC TCAAATCTCC TGAAA 95

SEQ ID NO:7496
SEQUENCE LENGTH:143
SEQUENCE TYPE:nucleic acid
TOPOLOGY:linear
CLONE:HUMGS08738
SEQUENCE DESCRIPTION:
GATCCTCCCT CCTTGGCCCC CCAGTGTTGG GATTACAGGC ATGAGCCACC GTGCTTGGCC 60
TGGAATNAAT TTTTATTTGA AGTTATTCTG TATCTCCAGT GTTGGCATAT GCTAGACATT 120
CAATGAATAT TTGTTGATTT AAA 143

SEQ ID NO:7497
SEQUENCE LENGTH:342
SEQUENCE TYPE:nucleic acid
TOPOLOGY:linear
CLONE:HUMGS08739
SEQUENCE DESCRIPTION:
GATCAACAAA ATGTGGTATA TTCATACAGT GGAATATTAT TCAGCCTCTA AAAGGAAGAT 60
ACGCTGACAT GTGCTGCAAC ATGGATGANT CTTGAGGACA TGATGCTAAG TGAAGTAAGC 120
CAGTCACTGG AAGACAAATA CTCTATGCTT CCNTTTATGT GAAGTATCTA GAGCAGTCAA 180
ATGCATAGAA ACAAGTAGAA TGGTAGTTGC CAAGGACTGG GGGAGGAGGA AATGAGGAGT 240
TGTTTAATGG GTATAGTGTT TCATTTTTGC NAGATAAAAA GTCCTGTGGN TTGGTTGCAC 300
AGTTAAGTTA TGTGANTGCT GTATGCCCAA CTCANCTGTT NN 342

SEQ ID NO:7498
SEQUENCE LENGTH:236
SEQUENCE TYPE:nucleic acid
TOPOLOGY:linear
CLONE:HUMGS08740
SEQUENCE DESCRIPTION:
GATCCAGTCG GCCTGGAGAT TCTACGCCAC CAACCTCTCG CGCACAGACC TGCACTCCAC 60
GTGGCAGTAC TACGAGCGAA CGGTCACCGT GCCCATGTAC AGGTACCGCC GCCGGGCACC 120
TGCCACCAAG CAACTGTTTC ATTTTTTATT TTCCATTTGT TCTTAAACCC CACTTTTTGT 180
TGTTCATTAT TTTGATTGAT TTTNNNTTCT TTAAAATGTA TTTTTCACAA AGGAAA 236

SEQ ID NO:7499
SEQUENCE LENGTH:160
SEQUENCE TYPE:nucleic acid
TOPOLOGY:linear
CLONE:HUMGS08741
SEQUENCE DESCRIPTION:
GATCCTAACC CTTTAGTATG CTGGAATTCT ACTCTTCACT TACTGCATTG ACTGTTGTTG 60
ATTAGTTATN ATTGCAAAGC ACTGTCACCG GCCTCAGGGA GTTTATGTGT AATAGAATTA 120
AAAATAATAG CTGTGTATAA CACTTAGCTC AAGCCACAAA 160

SEQ ID NO:7500
SEQUENCE LENGTH:210
SEQUENCE TYPE:nucleic acid
TOPOLOGY:linear
CLONE:HUMGS08742
SEQUENCE DESCRIPTION:
GATCTTATTG TGGAGTTAAA CAAAGCACCC TTAAAGTAGC AAGCGAGCAA AATAAAAGCA 60
AGGAAAATCA GTTGGAACAT AACTTATGTA TTTTTAGGA TTAAGAGTAA TTTAACTGAG 120
TCAATTCAAT ATGTTTTTAA AGGTGTGATT CTTAAATNCT TTATGATTGT TTAGATTTCT 180
ATATCTTGGT AACATGAATA CACACTTAAA 210

SEQ ID NO:7501
SEQUENCE LENGTH:351
SEQUENCE TYPE:nucleic acid
TOPOLOGY:linear
CLONE:HUMGS08743
SEQUENCE DESCRIPTION:
GATCAGCATT TGTATTTNAG TCCATTAATG GGAAAAATAG CTGATATTGT NCATAGGAAC 60
TTGGAAACCA CACAGGACTT AAGTCCCTTG TCTGTNTTGA TGGTCAACAT ATCTCCTTTA 120
ATATCACGAC ATTTTCAACA ACANCTGGTG AACAAAACAG ANCTNCTTTT TGACACCATA 180
GATTCTCCTG AGGTCAACGT TGCAAAANGC ATAGCAAAGT TNCTCCGAAA TGTTAGATAT 240
CGTTATCAAC CACTATTAGA CAGATGTAAT ACCGTATTTT TTCAGTAATG TGGNCCACCT 300
TGATTTTGGN TTCCATCAGT ANAGNGACTT AGTGTATACA NNNACNAAAN N 351

SEQ ID NO:7502
SEQUENCE LENGTH:195
SEQUENCE TYPE:nucleic acid
TOPOLOGY:linear
CLONE:HUMGS08744
SEQUENCE DESCRIPTION:
GATCGCACCA CTNCACTCCA GCCTAGGTGA CAGAGGGGAT GACAGAGGGT GCTCTGCGNN 60
CAGGGAAGGC GGGGTGGTTG GGGGGTAGAG AATGTTGCNC TNATAACCAC AGCTGTGGTT 120
ATTTTNTGGC AGGTACAGGT NAGGTTCCCT CTCCTTTCCA CCAGATGGCA CTGGAGAAAA 180
GGGATTTNAN TTAAN 195

SEQ ID NO:7503
SEQUENCE LENGTH:257
SEQUENCE TYPE:nucleic acid
TOPOLOGY:linear
CLONE:HUMGS08745
SEQUENCE DESCRIPTION:
GATCAGAGCA GAAATAAGTG AAATAGAGAC TAAAAAATAG AAAACATCAA TGAAACAAAG 60
AATTATTTTT TAAAAAGATA AACTAAATTG ACAAACCTTT AGCTAGACAA AGAAAAAAGA 120
GATAAGACAC AGATAANTAA NTTCAGAGAT TAANAAGAAG CCATTATACC TGATACCACA 180
GAANTACAGA GANTCATAAG AGACANTTAT GTTGTGCACA TGTACCCTAG ANCTTAAAGT 240
ATAATANNCN NTANTTN 257

SEQ ID NO:7504
SEQUENCE LENGTH:328
SEQUENCE TYPE:nucleic acid
TOPOLOGY:linear
CLONE:HUMGS08746
SEQUENCE DESCRIPTION:
GATCTTACAG AGAAAGACTA TGAAATACTT TTCAAATCTA TTAATGGAAT CCTTTNCCCT 60
GGAGGAAGTG TTGACCTCAN ACGCTCAGAT TATGCTAAAG TGGCCAAAAT ATTTNATAAC 120
TTGTCCATAC AGAGTTTTGA TGATGGAGAC TATTTNCCTG TGTGGGGCAC ATGCCTTGGA 180
TTTGAAGAGC TTTCACTGCT GATTAGTGGA GAGTGCTTAT NAATTGCCAC AGATACTGTT 240
GACGTGGCAA TGCCGCTGAN CTTCACTGGA GGTCANTTGC ACAGCAGAAT GTNCCAGANT 300
TTCCCTACTG AGTTGTTGCT GTCATTAN 328

SEQ ID NO:7505
SEQUENCE LENGTH:102
SEQUENCE TYPE:nucleic acid
TOPOLOGY:linear
CLONE:HUMGS08747
SEQUENCE DESCRIPTION:
GATCAATTTG AAGTCCCTGT TTGGGAATAA GGCACTTATC AGCATGAAGA ATTTTTTCTC 60
ATTCTGTGCC ATTTTAAAAA TAGAATACAT TTGTATATTA AA 102

SEQ ID NO:7506
SEQUENCE LENGTH:343
SEQUENCE TYPE:nucleic acid
TOPOLOGY:linear
CLONE:HUMGS08748
SEQUENCE DESCRIPTION:
GATCACCTTC GNACGACTAC ATCGCCTGCT GCGTCAAACT GAGGGCTCTT ACAGNCAGTT 60
TTCGAAGNCG GGGTACTGCT CAGCAAGGTG TTGTGAATTT CCCATATGAT GATTTCATTC 120
AATGTGTCAT GAGTGTTTAA ATCAAGAGGA AGCTGCATGA ATGTAATCAA CATTCCAACT 180
GGAGCTCTCC TTTGCTTGTC CTCTTTGCCT TCGGTAATAT GTATAAACTT ACATCACGAC 240
TTTCTCTTAA CAGCTGTTGT AAAGTTTATT ACTTTATGTA CAACTGAAGT TTTGTTTTAG 300
TTTTGATAAT AAAATTCTTT GGAACTTTAA AAAAAAAAAA NGN 343

SEQ ID NO:7507
SEQUENCE LENGTH:342
SEQUENCE TYPE:nucleic acid
TOPOLOGY:linear
CLONE:HUMGS08749
SEQUENCE DESCRIPTION:
GATCCAAGTG CTTNTTTGGT ACCAAGAGAC CCGGNTCTNT GCCAGCATAA ACCACTCACA 60
CCCCAGGGGG ATGAGCTCTC TNAGCCCAGG ATTCTGGCAA GGGAGGTGAA GAAAGTGGAT 120
GCGCAGAGTT CGGCTGGGGA AGAGGACGTG CTCCTGAGCA AAAGCCCATC CTCACTCAGC 180
GCTAACATCA TCAGCAGCCC GAAAGGTTCT CCTTCTTCAT CAAGAAAAAG TGGANCCNGC 240
TGTCCCTCCA GCAAAAACAG CAGCCCTNAA TAGCAGNCCN CGGTCTTTGG GGAGGNGCAA 300
AGGNAGGCTC CGGTTGCCCC AGATTGGCAG CANNATTATN AN 342

SEQ ID NO:7508
SEQUENCE LENGTH:369
SEQUENCE TYPE:nucleic acid
TOPOLOGY:linear
CLONE:HUMGS08750
SEQUENCE DESCRIPTION:
GATCAAGCAA TTAATCTTCT GTAGAACCAA AATTNACTGT AATAACTTGG AGCAGTACTT 60
TATACAACAA GGAGGAGGAC CTGATAAAAA AGGACACCAG TNCTCATGTG TTTGTNTTCA 120
TGGTGACAGA AAGCCTCATG AGAGAAAGCA AAACTTGGAA AGATTTAAGA NAGGNGATGT 180
AAGATTCTNG ATTTGCACAG ATGTAGCTGC TAGAGGNATT GATATCCACG GTGTTCCTTA 240
TGTNATANNT GTCACTCTGN CCGATGAAAA GCAAAACTNC GTACATCGAA TTGGCAGAGT 300
AGGGTGGAGC TGANGGATGG GTCTGGCAAT TNCCCTNGTG GCACCGAAAN NGGACAGGGT 360
TTNGTACCN 369

SEQ ID NO:7509
SEQUENCE LENGTH:335
SEQUENCE TYPE:nucleic acid
TOPOLOGY:linear
CLONE:HUMGS08751

SEQUENCE DESCRIPTION:
GATCTGGCCA GAGGGGTGTT GGAGCCCAGC CCACCCACAT ACCAGTCAAG CTCTTAGGGG 60
AGCAGAAGAA AAGCAGGAAG AATTTAAATG TTTAATTTTT TTTTNAAATT GACTTTNCTA 120
GTTATTAAAA GTTGCTTGTT TCAGCAGTGN TATTGTATAA NGACCATCTT GTAAGNTACT 180
CCTGACATCT TGCTTTAGCA CATGTACAGT ACAGTTNCTN TGATAATGTG TTTGCCCTAA 240
CTNCCCTGGC TTCNCCTTCA GCCCATCCNC TCTCCTCTAG NGCAGTTGGG TTGGAGGCTC 300
NTTGAGGCAA GCNGCANCAT TGGNGGGGGT GCAGN 335

SEQ ID NO:7510
SEQUENCE LENGTH:270
SEQUENCE TYPE:nucleic acid
TOPOLOGY:linear
CLONE:HUMGS08752
SEQUENCE DESCRIPTION:
GATCCGACAC AGAGAACAGG AGGAACTNAG GCGACAGCAA GAGGGCTTTA AGCCAAACTA 60
CATGGAAAAT AGAGAACAGG AAATNAGAAT GGGTGATATG GGTCCCCGTG GAGCAATAAA 120
CATGGGAGAT GCGTTTAGCC CAGNCCCTGC TGGTAACCAA GGTCCTCCTC CAATGATGGG 180
TATGANTATG AACANCAGNG CAACTNTACC TGGTCCACCA ATGGGTACTG GTNCTGCCAT 240
GGGACCAGNA AGGNGCCGCA ANTNTNGGGN 270

SEQ ID NO:7511
SEQUENCE LENGTH:85
SEQUENCE TYPE:nucleic acid
TOPOLOGY:linear
CLONE:HUMGS08753
SEQUENCE DESCRIPTION:
GATCCCAGGA GAATCAGAAA CTCCAACATT TTGGAATCTT CAAGGGCACA TACTGAGAAA 60
AAAAATAAAA TTGTTTATGA GCAAA 85

SEQ ID NO:7512
SEQUENCE LENGTH:66
SEQUENCE TYPE:nucleic acid
TOPOLOGY:linear
CLONE:HUMGS08754
SEQUENCE DESCRIPTION:
GATCAAACTA TACGCTGTCT ACAAGAGACA CTTTAGATTT AAAGACACAA ATAGGTTGAA 60
AGTAAA 66

SEQ ID NO:7513
SEQUENCE LENGTH:390
SEQUENCE TYPE:nucleic acid
TOPOLOGY:linear
CLONE:HUMGS08755
SEQUENCE DESCRIPTION:
GATCTTTTGT ACTGAGGTTT TTAACACTTT ACTTGGGTTT ACCAAGCCTC AACTGGACAG 60

ACCATAAACA GTCCACAGGC ACCGTTCCTG CCAGGCCCCA ACCCACAGGG AGTCTNTCCG 120
CAGAGCCTTC TTGGTGTTGC CCTAACTTGC CAGTGGCCTT TGCTCAGAGC CTCCTCCTGT 180
GACATGTGAA CAATGAAGAG GCCTGCGCCT CCTGCCTTGC CGCCTGCAAA GCAAAGAAAC 240
TGCCTTTNAT TTTTTAACCT TAAAAAGTAG CCAGATTAGT ANCANGACTG GCTGGCTTGA 300
TGAGCAAAGC CTTTGCTCTC ACGGNAGGAG GGTAGGGTTT NGGNTGTACA ATGGTAACTT 360
GCNNTGGGNA CCTTAAAAAG GGAGGTGNAN 390

SEQ ID NO:7514
SEQUENCE LENGTH:212
SEQUENCE TYPE:nucleic acid
TOPOLOGY:linear
CLONE:HUMGS08756
SEQUENCE DESCRIPTION:
GATCACCTGA GGTCGAGAGT TTGAGACCAA CCTGACCAAC ATGGAGAAAC CCCGTCACTA 60
CTAAAAATAC AAAATTAGCT GGGCGTGGTG GCACATGCCT GTAATCCCAC CTACTTGTGA 120
GGCTGAGGCA GGAGAATCAC TCGAACTCAG GAGGCGGAGG TTGNAGTGAG CCACTGCACT 180
GCCTGGGCAA CAGAGCGAGA CTCCATTTCA AA 212

SEQ ID NO:7515
SEQUENCE LENGTH:136
SEQUENCE TYPE:nucleic acid
TOPOLOGY:linear
CLONE:HUMGS08757
SEQUENCE DESCRIPTION:
GATCCACCCG NCTTGNGCCC CCCAAAGTGC TGGGATTACA GGTGTGAGCC ACTGTGCCCG 60
GCCGAGTTAA ATATATTTCT AACATAGTAA AAAAAAAAAG GTANGTTNCA TTAAATAAAT 120
CCNGGAAGTN CAAATN 136

SEQ ID NO:7516
SEQUENCE LENGTH:160
SEQUENCE TYPE:nucleic acid
TOPOLOGY:linear
CLONE:HUMGS08758
SEQUENCE DESCRIPTION:
GATCATGAAG ATGGACAGAA CAGACACTGT GGCAATAAGA TACCAAATTG TAAACAGGAC 60
CTAAGGCCAT GCCAAGCAAG GGTTAAGTCA TGTAGAGCCC GCCTTCATGC CCCGCCTCCC 120
CTGCCCCCAA CTTATGAATA AACTATGTTC TAACTGCAAA 160

SEQ ID NO:7517
SEQUENCE LENGTH:355
SEQUENCE TYPE:nucleic acid
TOPOLOGY:linear
CLONE:HUMGS08759
SEQUENCE DESCRIPTION:
GATCTGGAAC TTGAAGATGC CATTCATACA GCCATCTTAA CCCTAAAGGA AAGCTTTGAA 60

GGGCAAATGA CAGAGGATAA CATAGAAGTT GGAATCTGCA ATGAAGCTGG ATTTAGGAGG 120
CTTACTCCAA CTGAAGTTAA GGATTACTTG GCTGCCATAG CATAACAATG AAGTGACTGA 180
AAAATCCAGA ATTTCAGATA ATCTATCTAC TTAANCATGT TTAAAGTATG TTTTGTTTTG 240
CAGACTTTTT GCATACTTAT TTCTACATGG TTTAAATCGA CTGTTTTTTA AAATGNCACT 300
TATANNTCCN AATAANCTGT TAANCCNGGN ANANGGNNTT GTNTTNGTGN GGNTN 355

SEQ ID NO:7518
SEQUENCE LENGTH:77
SEQUENCE TYPE:nucleic acid
TOPOLOGY:linear
CLONE:HUMGS08760
SEQUENCE DESCRIPTION:
GATCTTCATT TTCCTGTTCT GTATTCTGCT ATGAAGTTCT CTGTTTTTCA AATAAAATTT 60
ATGTGTTAAA TAACAAA 77

SEQ ID NO:7519
SEQUENCE LENGTH:20
SEQUENCE TYPE:nucleic acid
TOPOLOGY:linear
CLONE:HUMGS08761
SEQUENCE DESCRIPTION:
GATCATCTTG GAACTGCAAA 20

SEQ ID NO:7520
SEQUENCE LENGTH:247
SEQUENCE TYPE:nucleic acid
TOPOLOGY:linear
CLONE:HUMGS08762
SEQUENCE DESCRIPTION:
GATCGGAGGC TGCAGTGTGA CAGTGCCAGC CAATGTGCAG AGGTGGATGA GGTCTTGTNA 60
AAACCTGGCT CCTTTTAACA CGGCCCTCAA GCTCCTTAAG TNAATTGCCG TAACTAATTT 120
TAAAGGGTTT AGATTTTAAG AATGGTGCTC TTTCATGCCT ATNATCAGTA AGGGGACTTG 180
TATTAGAGTC AGAGTCTTTT TATTTAGGCC AGTTGTCAAG TGTCAATAAA AGCATCATGT 240
AATTAAA 247

SEQ ID NO:7521
SEQUENCE LENGTH:164
SEQUENCE TYPE:nucleic acid
TOPOLOGY:linear
CLONE:HUMGS08763
SEQUENCE DESCRIPTION:
GATCTTAATA TTGACTAGTT TCACATCCAG GTTTCTAAGA AATGATAAGA TACTTCACTT 60
TTCCAGAGTG AAATGTAGGA GGGAGCACAT TCTAAGTACA GCTAAAAATT TAGCTCACTG 120
TAACACAGTT TCACTCTCTG AATAAATAAA GCAAAAAACA CAAA 164

SEQ ID NO:7522
SEQUENCE LENGTH:110
SEQUENCE TYPE:nucleic acid
TOPOLOGY:linear
CLONE:HUMGS08764
SEQUENCE DESCRIPTION:
GATCTGCCTG CGAAGCTCCC CACAACACTG TCCCTGGAGA CCACCCATGT GGCAGACACT 60
CAAAATCTGT TGTATTTGCT AAATAAATTA ATATGGACAA GTTACCCAAA 110

SEQ ID NO:7523
SEQUENCE LENGTH:181
SEQUENCE TYPE:nucleic acid
TOPOLOGY:linear
CLONE:HUMGS08765
SEQUENCE DESCRIPTION:
GATCAAATTG TTCATGCCTT GGGATTTTTT CTTCTATAAT TTGGATATCT TTNTCCATTG 60
CTCATTTCTT TATTGAAGTT GCTTTCTTAA AAATTGATTT AACTTTGTCC TTGGTATCTT 120
TAGTTCAGTC CAGAAATNAT TAATTGTGAT GTGATTAAAT TCATTTTTTG GTCTGGTGAA 180
A 181

SEQ ID NO:7524
SEQUENCE LENGTH:175
SEQUENCE TYPE:nucleic acid
TOPOLOGY:linear
CLONE:HUMGS08766
SEQUENCE DESCRIPTION:
GATCTGAATG GTTTTAATAA GGGGCTTTCC CCCTNCTTCG CTCTGCACTT CTCCTTGCTG 60
CCATGCGANG AAGGATGTGT TTGCTTCCCC TTCTGCCATG ATTGTAAGTT TCCTGAGGCC 120
TCCCCAGCCA TGTTAAACTT TGAGTTAATT AAACCTCTTT CCTTTATAAA TTAAA 175

SEQ ID NO:7525
SEQUENCE LENGTH:362
SEQUENCE TYPE:nucleic acid
TOPOLOGY:linear
CLONE:HUMGS08768
SEQUENCE DESCRIPTION:
GATCTGAAAA ATNCTTTTTG TCCTCATTGT TGCAGTTTCA ATTGTAATCC ATTGCATCTN 60
ATTTTCTTGG GTGTTGGCAC TGTAATATAT TAAAAAAAAA TTCAGTGCTC AGTATTGTAA 120
CAACTGTCCC TACTGAATAT TCCTTTTCAT AAATACTTGC TACCACAGGG AAATTAAGTT 180
TTCATATAGA ATAACTAGTT TCAGTAGTAA CCATTGAAGA NTTAANCNTG TGGTTCANGG 240
CAGATATTTT TCTGGAAAGT TTTCTCTATT GTANAATTTG TNGTATATGG CTATGCTGAC 300
TATCNTGGAT NCTGTCATGG GACATGCATT ACCACANNTN CNNGCCCCTT TCTGTGGTNN 360
NN 362

SEQ ID NO:7526

SEQUENCE LENGTH:306
SEQUENCE TYPE:nucleic acid
TOPOLOGY:linear
CLONE:HUMGS08769
SEQUENCE DESCRIPTION:
GATCCCATGG ACATTTGGGG AAAGGGCTCC TTGGGCTGCT GGTGAACTTC TGTGGCCACC 60
ACCTCCTGCT CCTGACCTCC CTGGGAGGGT GCTATCAGTT CTGTCCTGGC CCTTTCAGTT 120
TTATAAGTTG GTTTCCAGCC CCCAGTGTCC TGACTTCTGT NTGCCACATG AGGAGGGAGG 180
CCCTGCCTGT GTGGGAGGGT GGTTACTGTG GGTGGAATAG TGGAGGCCTT CAACTGATTA 240
GACAAGGCCC GNCCACATCT TGGAGGGCAT CTGCCTTACT GATTAAAATG TCAATGTGAA 300
TCTAAA 306

SEQ ID NO:7527
SEQUENCE LENGTH:76
SEQUENCE TYPE:nucleic acid
TOPOLOGY:linear
CLONE:HUMGS08770
SEQUENCE DESCRIPTION:
GATCTTTTAG TTATCAAACT GTTAAATACA TTTTAACTTT CAAATTTTAT GAATAAAGGA 60
GATGAATCCT GTTAAA 76

SEQ ID NO:7528
SEQUENCE LENGTH:74
SEQUENCE TYPE:nucleic acid
TOPOLOGY:linear
CLONE:HUMGS08771
SEQUENCE DESCRIPTION:
GATCTGAAGT CTGATTGTGT GTACGTTCGC GCGTGCGTTA GTCCCTGCCA CGATTAAAGA 60
CTTAGACCGN CAAA 74

SEQ ID NO:7529
SEQUENCE LENGTH:102
SEQUENCE TYPE:nucleic acid
TOPOLOGY:linear
CLONE:HUMGS08772
SEQUENCE DESCRIPTION:
GATCGCGCCA CTGCACTCCA GTCTGGTGAT AGAGCAAGAC TCCATCTCAA AAATAAAAAA 60
TAAAATGTTG GNTGTGGGGA GCCAGGCCTC TCACAACTGA AA 102

SEQ ID NO:7530
SEQUENCE LENGTH:312
SEQUENCE TYPE:nucleic acid
TOPOLOGY:linear
CLONE:HUMGS08773
SEQUENCE DESCRIPTION:

GATCGAATCC AGGCCCTGGG GCTTCANTTG CCAGACCCAC CATTAGAGAG TGAAGATGAA 60
GATGAGGAGG GAGCTACAGC GTTGAACAAC CACAGCTCTA TTCCCATGGA CCCAGAACAT 120
GTAGAGCTGG TGAAAAGGAC AATGGCTGGN GTAAGCCTGC CTGCGCCAGG GGTTCCTGCC 180
TGGGCTCGGG AGATATCGGA TGCCCAGTGG GAAGATGTGG TACAGAAAGC CCTCCAAGCC 240
CGGCAGGCAT CCCCTGCCTG GGAGTGACCA CAGTGAGAGC TGCCTTATAT TCCTACATTC 300
CAGGCCAGAA CN 312

SEQ ID NO:7531
SEQUENCE LENGTH:186
SEQUENCE TYPE:nucleic acid
TOPOLOGY:linear
CLONE:HUMGS08774
SEQUENCE DESCRIPTION:
GATCTGGACT GAGGACCCTG CTACTCCCCA AGCCAGAGCC CATCAGCCAG GCCTGCTGTG 60
AGCCACCTGC CTGTGGAGTG CTGAGCTCAA CCAAAGGCTG GCAAGCTCTG GGCCTCATTT 120
AAGGGATTCT GATGAGCCGA TGGGCCCTGG AGGCAGCCCA TTAAAGCATC TGGCTCGTTT 180
TTGAAA 186

SEQ ID NO:7532
SEQUENCE LENGTH:390
SEQUENCE TYPE:nucleic acid
TOPOLOGY:linear
CLONE:HUMGS08775
SEQUENCE DESCRIPTION:
GATCACATTG CCATTCCTCC CCTCCAGAGG TAACAATTAT CCACAATTTG ATGTTTATCA 60
TTCCTGTGTT GTTGTACTTT CACTGTGTAT AACCTAANNC NTCTACTNTT TAGTACTGTT 120
TTATATATTT TNAAGCCTCA TACTTGCTCA TTCTACAGCT TTTTTCACTC ATTATTGTAT 180
AATTATATCT GANGCNCTCG TTCATTAATT TTAGTCCNGT GTAGCAGAAT TCAATTACGG 240
GAACTACCAT AATTTATCTG TNCTCCAGTT GAAGGCATGA NGTTGTTGCC AGTTTCTGTA 300
TTATAACCAC TGTAGTGGGA CCATTCTTCT GCNTTGGGCT CACTGCGGGT TACCNAAGNC 360
GGTNCCNCAG GATAANCCCC ATTTGGGNTN 390

SEQ ID NO:7533
SEQUENCE LENGTH:378
SEQUENCE TYPE:nucleic acid
TOPOLOGY:linear
CLONE:HUMGS08777
SEQUENCE DESCRIPTION:
GATCTAGCAT GTGGATTTTA AAAGATTTGC CCTCATTAAC AAGAATAACA TTTAAAGGAG 60
ATTGTTTCAA AATATTTTTN CAAATTGAGA TAAGGACAGA AAGATTGAGA AACATTGTAT 120
ATTTNNCAAA ANCAAGATGT TTGTAGCTGT TTCAGAGAGA GTACGGTATA TTTTTGGTAA 180
TTTTNTCCAC TAGCAAATCT NGATTTNGTT TGATAGTGTG TGGAATTTNC TTTTGAGGGA 240
TAAGACCATG GGANANTTGT GGTAAAGACT GTTTGTNCCC TTCATGANTN CTTCTGAAGT 300
TGCCNTCAGT TTTNCTAATC TCCTGTGAAA TGCCTAGGTT TTGCGCCTGT CCCACTTTTT 360
NTTTGTGGGN CTTATNTN 378

SEQ ID NO:7534
SEQUENCE LENGTH:175
SEQUENCE TYPE:nucleic acid
TOPOLOGY:linear
CLONE:HUMGS08778
SEQUENCE DESCRIPTION:
GATCATTTGA GTGAAAAAAA GGTACCTGTN CAAGCAAGCT CCTGGACACC ACAAGAAGGA 60
GGAATTATTT TAAAAGCTGT ACTCTTAAAT TGTNAGTATC TTTAAAATCA GTTGTGAACA 120
ATGAAGGATT TGAAAGAGCA TTGACTTTGC CACTTAAAAG TATTTCTAAA ATAAA 175

SEQ ID NO:7535
SEQUENCE LENGTH:159
SEQUENCE TYPE:nucleic acid
TOPOLOGY:linear
CLONE:HUMGS08779
SEQUENCE DESCRIPTION:
GATCAAGGTG AATTTTTTGT ATCACTGCCT GTGGAAATCT ATCCTCATCA GTNATTGCAT 60
TTTTCCCTGC CTATACCTGT GCTCCTTTTT NTNACTGTGT TTTCAGTCAC TTCCTTTCTG 120
TNAAAGGTTG CTTAGCTTTT NTTTTNAANA TTTGTTGTN 159

SEQ ID NO:7536
SEQUENCE LENGTH:304
SEQUENCE TYPE:nucleic acid
TOPOLOGY:linear
CLONE:HUMGS08780
SEQUENCE DESCRIPTION:
GATCCAAACC CACAGTNTTG TCCTGGAGGC AGCAGGGGTG AAGGTGGAGG GTCCAGGGCC 60
ATGAGGAGCC CCCTTGCCAT CAGAGCCTGG CCTAACCACC CTCTTCTCTA CTTACACACA 120
CATGCATTTT ATAATAGCTC TGACCCAACC TGGCCACTCT GCAGAGACTG GGACAGACAG 180
GTGCAGGCAA TGGGCCCTCC CACACCCAGT CACCTACAAG GAATTTTCAA ATCCACTTTT 240
AAAACAGAAA CCGGTAAATG CGCCGTATTG TATATTTTAT TTAAATAAAA AAAATTCCAG 300
CAAA 304

SEQ ID NO:7537
SEQUENCE LENGTH:110
SEQUENCE TYPE:nucleic acid
TOPOLOGY:linear
CLONE:HUMGS08781
SEQUENCE DESCRIPTION:
GATCCACCCT TCCTGGGCCT CCCAAAGTGC CGGGTATTAC AGTCATAAGC CACCACACCC 60
GGCCTAACAT GCATTTTTTT AATTCAATAA ATATTTATTG AGCACCTAAA 110

SEQ ID NO:7538
SEQUENCE LENGTH:160

SEQUENCE TYPE:nucleic acid
TOPOLOGY:linear
CLONE:HUMGS08782
SEQUENCE DESCRIPTION:
GATCCCCTCC CCTCCTGCTT TTCTGTATTT TTTAAAAATT ATTTTGTTTT TAATTCACAA 60
ATATTGTATA TGTTATATGG GGTAGAATGT GATGTTGATA TATGTTTAAA ATGTGGAATG 120
ATTAAATCAG GCTAATTTAC TTTTTTTNCC NAAAAGGAAA 160

SEQ ID NO:7539
SEQUENCE LENGTH:219
SEQUENCE TYPE:nucleic acid
TOPOLOGY:linear
CLONE:HUMGS08783
SEQUENCE DESCRIPTION:
GATCCACCCA GTTGCTCAAG TAAAAAACCT GAGAGACACC TTACGCCATC TGGGAACTTG 60
TTAGAAATTC TTAAACCTCA TTCTATACCT GCTAAATCAG AAAATGTAGG GATTAAGTCT 120
GATTATTTGT GTTTTCGCAA GTCATCCNGG TGATTCTGAT GCATGGAAAG TTTGAGAACC 180
ACTGGTTTAG GTTATTAAAA GTTCTTGTCT CCCTATAAA 219

SEQ ID NO:7540
SEQUENCE LENGTH:355
SEQUENCE TYPE:nucleic acid
TOPOLOGY:linear
CLONE:HUMGS08785
SEQUENCE DESCRIPTION:
GATCAGAGAA GACTTTAAAA CTCTTGACTT AATTGAGTAA ACTCTTCATG CCATATACAT 60
CATTTTCATT ATGTTAAAGG TAAAATATGC TTTGTNAACT CAGATGTCTG TAGCCAGGAA 120
GCCAGGGTGT GTAAATCCAA AATCTATGCA GGAAATGCGG AGAATAGAAA ATATGTCACT 180
TGAAATCCTA AGTAGTTTTG ANTTTCTTTG ACTTGAATCT TACTCATCAG TAAGAGAACT 240
CTTGGTGTCT GTCAGGTTTT ATGTGGTCTG TAAAGTNAGG GGTTCTGTTT TNGTTTCCTT 300
ATTTTGAGGT AAGAGTACTG CTNGGTGGTC GGGGGGGTTA TATNNTNCCC TTTTN 355

SEQ ID NO:7541
SEQUENCE LENGTH:314
SEQUENCE TYPE:nucleic acid
TOPOLOGY:linear
CLONE:HUMGS08786
SEQUENCE DESCRIPTION:
GATCAGGTTT CCCCTCAGAA CCTGTGTTGC CTGCCTGCTG TATCTTCCAT GTCTTTCTTA 60
TTCAACTTCT AGAAGTAGAG AAGGTTGGGA CTTGAGTGTA GAAGAAACAT CAGAATAATT 120
TCAAAAAACA GGAAGTTGGA ACTATAATTT TTAAATTATT GTATTGTTTT TACGGATTTT 180
ACATGTATAG TTAAATATTA ANNTTCATCT CTTGCAGTTC ANTAAAAGCT ATAGTATAGA 240
TTTTATTTTT AAGAAGTTTA TATAATNCTT TTGTGAGCCA ATATGTAAAA CTNGAATATT 300
TATTTANCCT ANTN 314

SEQ ID NO:7542
SEQUENCE LENGTH:298
SEQUENCE TYPE:nucleic acid
TOPOLOGY:linear
CLONE:HUMGS08787
SEQUENCE DESCRIPTION:
GATCACAGAG AAAGTGATTA CAGACATTGC TGGGGTAATA CCAGCTGAGA AAATTGATGG 60
AGTATTTNCT GCCTGTNAGA GTGGCTCTTT TGACAAACTA GANGCTGTGG TCAAGGATTT 120
AATAGATGAG GGTCATGCAG CAACTCAGCT CGTCAATCAN CTCCATGATG TGGTTGTAGA 180
AAATANCTTA TCTGATAANC AGAAGTCTNT TATCNCAGAA AACCTTGCCG AGGTTGCCAA 240
ANGCCTGGCA GATGGTNCTG TTGTCCATTT GCANCTCATC NGCCTTTTNG CCCTGNGN 298

SEQ ID NO:7543
SEQUENCE LENGTH:32
SEQUENCE TYPE:nucleic acid
TOPOLOGY:linear
CLONE:HUMGS08788
SEQUENCE DESCRIPTION:
GATCAAAATT AAAGACAGTT AAAAAGGTGA AA 32

SEQ ID NO:7544
SEQUENCE LENGTH:249
SEQUENCE TYPE:nucleic acid
TOPOLOGY:linear
CLONE:HUMGS08789
SEQUENCE DESCRIPTION:
GATCATCATT TATTGCATCT CATAACTAAA TTTNNTAAAG TTTGGATTGG GACTTTTNAG 60
GTCCTTTTTG GAGGGCAAAG GAAGTGCCAG CTTCTNTGGG GAACTTGTTT TTAAATCCAA 120
AGACTTGAAC CACATTCCCT GCACATGANC ATGTTTGCTT TTATCCCTTC TCTCATTGTN 180
TCCTACCCAT CTTAGTACCA TTGTGGTTAT AANCATCTGC ATTTNTNAGA AGCATTTNAC 240
CCATTTNNN 249

SEQ ID NO:7545
SEQUENCE LENGTH:257
SEQUENCE TYPE:nucleic acid
TOPOLOGY:linear
CLONE:HUMGS08790
SEQUENCE DESCRIPTION:
GATCATATGG AATGTACTAT TTTGTAATGT CTTTTTTCAT TTTACAATGT ATTATCAACC 60
TTTTCCCTCT CAAAAATACA TTGTGAATGA CTGCATAGTA TTCACTTTAT GANTATTTAA 120
TTCATTTCAC AGTCTTCTAT TGTNGGACCA CTNACATTGT ACCAAATGTT TTCCTTTGGT 180
TTATTCTTTA ATGTATTAGT ATTTTACTGC TGGTCACTCA TGGAATCCTG CAGCTTTANT 240
NAAAAGCAAG GATGAAA 257

SEQ ID NO:7546

SEQUENCE LENGTH:158
SEQUENCE TYPE:nucleic acid
TOPOLOGY:linear
CLONE:HUMGS08791
SEQUENCE DESCRIPTION:
GATCGTGCCA CTGCACTCCA GCCTGGGCGT TGCAGTGAGA CTCCGTCTCA ACAACAACAA 60
AAAGGTGGAT ACATGTCAAA AGGGCACAGG AGCCACTTGA AGGATTATAC TTGCTAAATC 120
TGTGTCAATT ATCAAAATAA ACTGGTAGCA ACGTTAAA 158

SEQ ID NO:7547
SEQUENCE LENGTH:128
SEQUENCE TYPE:nucleic acid
TOPOLOGY:linear
CLONE:HUMGS08792
SEQUENCE DESCRIPTION:
GATCAAAGGG ATGAGCAACA GGGACTTCTG CCACAGTGAC AATGGAATTG TGTTGTGCCT 60
TACTTCAGAG GTGGTCTCTN CTTTCTTGTA ATAAAAGCAA TATTTATGCG GAAAGCAAGC 120
AGCTCAAA 128

SEQ ID NO:7548
SEQUENCE LENGTH:205
SEQUENCE TYPE:nucleic acid
TOPOLOGY:linear
CLONE:HUMGS08793
SEQUENCE DESCRIPTION:
GATCACATCA AGCTAAAAAA CTTCTACACA GCGAAGGAAA CAAAGTGAAC AGAATAACAT 60
GGGAATGTTT TCTGTAATTT AGTAGTAACT GGCAATAGTT TACAAACACA TTTTGTGTAT 120
ACTGCTGTCA TTGCACTGNT TACCTTCTGT TGTAGTGACT TTGTTCTATT AGTCCACTCA 180
ATTAAAATAT TTGGTTTTGT TTAAA 205

SEQ ID NO:7549
SEQUENCE LENGTH:372
SEQUENCE TYPE:nucleic acid
TOPOLOGY:linear
CLONE:HUMGS08794
SEQUENCE DESCRIPTION:
GATCTTATTT TTAATGTAGT GAGGATATTA TTTAAACTTT TATTTTAACT GGAAATGTCC 60
TGAAACACAT ATTTAAAATA TTGGGATACA GTGAAAGAAA AATTCAAATT TTAATAACAT 120
AAAGATTTCC TAACTTTATG TTATTGANCA CTTACTCACT AGAAGTGAGT TCTTTAGAAA 180
ANTACAGTGA AGGACTCAGT TCAGTCTTGT TTTTATCAGA GTGATAATCA TCCTGTTTCA 240
CATCCCAATA CTATTTTGAN ATTCTAAACA ATGGAACCCA AANTNCCAAT AANTATAAGG 300
TTATGCCTTC AATATATTCC TATACANTTC TGTAACCATG GTTTANNATA CACAAGCTTA 360
NNATACCATG GN 372

SEQ ID NO:7550

SEQUENCE LENGTH:97
SEQUENCE TYPE:nucleic acid
TOPOLOGY:linear
CLONE:HUMGS08795
SEQUENCE DESCRIPTION:
GATCCGGTNG TCTAAATGCA TTCATATTTT TATGATTGTT TTGTAAATAT CTTTGTATAT 60
TTTTCTGCAA TAAATAAATA TAAAAAATTT AGAGAAA 97

SEQ ID NO:7551
SEQUENCE LENGTH:244
SEQUENCE TYPE:nucleic acid
TOPOLOGY:linear
CLONE:HUMGS08796
SEQUENCE DESCRIPTION:
GATCTGGGTT TGGCATTAGA AGTATTTCAT AATTTTGGTT TTTNATTTAG GTTTCCTCAA 60
CATCTGTAAA GTGATTGATT AAATTAGAGG AGGCGTGTAG AATAAATCCC AATCCCATTG 120
CAACTGGCAG AGCATTATAA NTNTTTATAA ATNCAGTTAC AACAAAGGAG AGGNTCTACA 180
TTCTTCAAAA AANAAANTTG GCTTACCTGT TTCANCTTTN GGTGGCATTT NNCTGCCATT 240
NGTN 244

SEQ ID NO:7552
SEQUENCE LENGTH:364
SEQUENCE TYPE:nucleic acid
TOPOLOGY:linear
CLONE:HUMGS08797
SEQUENCE DESCRIPTION:
GATCTGGGAA TTACCCATCA TCATTAAGCA ATGAAACAGA CCGGCTGGTG CACTGGTGCC 60
ACGGCGCCCC GGGGGTCATC CACATGCTCA TGCAGGCGTA CAAGGTCTTT AAGGAGGAGA 120
AGTACTTGAA AGAGGCCATG GAGTGTAGCG ATGTGATTTG GCAGCGAGGT TTGCTGCGGA 180
AGGGCTACGG GATATGCCAT GGGACTGCTG GCAACGGCTA TTCCTTCCTG TCCCTTTACC 240
GTCTCACGCA GGATAAGAAG TACCTCTACC GAGCTTGCAA GTTTGCAGAG TGGTGTCTAG 300
ATTACGGAGC ACACGGGTGC CGCATTCCTG ACAGACCCTA TTCGCTCTTT GAAGGCATGG 360
CNAN 364

SEQ ID NO:7553
SEQUENCE LENGTH:129
SEQUENCE TYPE:nucleic acid
TOPOLOGY:linear
CLONE:HUMGS08798
SEQUENCE DESCRIPTION:
GATCTGTGTG TCTCTGTTTT GAGTTTTTCC TGTTTATTTT GAAAAGTACT GTTGGTCAAG 60
ATAATTGGTC AATAATCCAT GTTGGTTTTA ACAAAAAGCA TTTTAACATT AAAAATATTA 120
CAGTATAAA 129

SEQ ID NO:7554

SEQUENCE LENGTH:316
SEQUENCE TYPE:nucleic acid
TOPOLOGY:linear
CLONE:HUMGS08799
SEQUENCE DESCRIPTION:
GATCACATTT TNATTTTTTN TTCTCCACAT CAGGATAGTT TACTGAAGCA CAATCTCTTA 60
TACTAGTGGG ACAAAAGGGA GAAAAAGGAA GCAAGATAAA TGGGTATGTA GGATGAAGGG 120
TTATTTAAAA TGGAACTAAA GATAGAAGGA GGACTGTAGG AAGANATGGA ATNNTTTANN 180
TNTGAGGAAA GATATCTNTG GTAGACATGT CCTTCCATGA CTAATTTCTA ATTGTNACTC 240
ANCACACATT GAGGTATGGN CCCTCCTCAG TGACTTTNAC TAGCTCAGAN ACGTACTCCC 300
CCACCANCCC CACCTN 316

SEQ ID NO:7555
SEQUENCE LENGTH:365
SEQUENCE TYPE:nucleic acid
TOPOLOGY:linear
CLONE:HUMGS08800
SEQUENCE DESCRIPTION:
GATCAGTGGA ATTCTTGGTT TGATGATAAC CTTATTAATT GAAATNTTTT ACTGATGTGG 60
CTTTAAAAGA GGTTTATNTT GTATATGTTT AGAACTCTCT GATTTTNATG AATTATATGG 120
GAATGAGAAA CAGAAGAAGT GGTATTTNCT GGCGAGTTAA ATAGGCAAGG TACCCAGTGA 180
TAACACCAAC CAAACCACTC CTATCTGCAT GATTCTGAAC ATCTGGATGC CTGTGGTTTT 240
ACTGTGTATA TNTNATTTTT AATATATTAC CTTTGTGGAT NCATTTAAGG TCTACTCAAA 300
NGTANCACTG TCAAAACCAC TAATATGTAT GTNAAANTTG TNCTGTNTAC TACATTACNG 360
TNCAN 365

SEQ ID NO:7556
SEQUENCE LENGTH:349
SEQUENCE TYPE:nucleic acid
TOPOLOGY:linear
CLONE:HUMGS08801
SEQUENCE DESCRIPTION:
GATCTTGAGT AACTGAACTT ATTTCCCTTA AGGTATAAAA ATTTAAATAA CCTGTATATA 60
TTTGNTAACA NCTTTATNAG GGAGGTAAGT NACTTACAAT ACATTGCACA TATTTAANGT 120
ATAGANTTCG ACGAGTTTTG ATGTATGTTT GCACCTGTGA AACCATCACA ACAAACAGTG 180
AGCATATCTN TCACCCCTAA NNGTTTCCTC CTGTCCCTTG GTAATCCCTC TNTTACCTGT 240
CCCACTTCTG TTCCTCAAGA NCCACTTACC TGCTTTNNTN ACTGTTAATG TACGTGAGTT 300
TACATTTTTG AGAGTTTTAT CCAAAGGGNA TCATGANGTA TGTNCTCTN 349

SEQ ID NO:7557
SEQUENCE LENGTH:369
SEQUENCE TYPE:nucleic acid
TOPOLOGY:linear
CLONE:HUMGS08802
SEQUENCE DESCRIPTION:

GATCTNATCA ATCTAGCGGG AGAGACAGGA TAACCTGTCC GAAAGTATAG CGCCACTATN 60
ACTCCGCCGG AAAAATTACT TTAAAAATCG CCAAAAATTA CTTGGAGCAA AGGGCAGTCG 120
GCGGANTTCG CCAAGGCTGG CGCAGTCGGT TTTNACCTGT AGCAGAGAAC CNGGNCTGGA 180
GAACAGCCTC ACTTCTTTNA TTGANTACTT ACATAATGCA TTGGAACATG ACATGAGATT 240
AAGGTTTAAT AATGATAGAN TGAAGACCAC AATAAAAGAG ACCTCTACTT AGCTCAGCAA 300
TTCTTACCTT TCTTACCTAT TTGATGGAGG NTGTCTTTTG ANAGGTGTNC TGCAGGGNCC 360
AAANTGTTN 369

SEQ ID NO:7558
SEQUENCE LENGTH:290
SEQUENCE TYPE:nucleic acid
TOPOLOGY:linear
CLONE:HUMGS08803
SEQUENCE DESCRIPTION:
GATCTTGTAT GCCAAACTTT AATCTGCTTT TATGTTTTCA GGCTGAAGGT GTGAAAATCC 60
TAAGAGGATT TCATATTGAA TATGTGTACA CAATCTTAAC TATCGTGGTG GAAAACATAC 120
TACTATAATT NATTATTATA TCTNCCAGAT AATGTTATTC ATTTAGAACA AATAAGGTAT 180
ATTTNTNAGA ATCANCTTTG TAAGCACTAT AANNNCTTTN ATAAGTTATA AGGTCTATGA 240
TGTGTTTACT TTAANNCTTG CTGTTAANAG CAACACGTAT TANNTATGTN 290

SEQ ID NO:7559
SEQUENCE LENGTH:55
SEQUENCE TYPE:nucleic acid
TOPOLOGY:linear
CLONE:HUMGS08804
SEQUENCE DESCRIPTION:
GATCTGGATA AATTTTCATT TCTACTTAAT TAAAACTTCC TATGTAAAAT CCAAA 55

SEQ ID NO:7560
SEQUENCE LENGTH:109
SEQUENCE TYPE:nucleic acid
TOPOLOGY:linear
CLONE:HUMGS08805
SEQUENCE DESCRIPTION:
GATCCCGAAT CCCAGAAATA ATTCATAGNG CACCTATCAT GACTATTTAT TTAATCTCAG 60
AAGCTGAATC TGTACGTATT TTTCTCCAGG TCATCAGCTT TGGGNNNNN 109

SEQ ID NO:7561
SEQUENCE LENGTH:40
SEQUENCE TYPE:nucleic acid
TOPOLOGY:linear
CLONE:HUMGS08807
SEQUENCE DESCRIPTION:
GATCCTACTC TCTTATTAAA ATCTTGAGTT TATGTTCAAA 40

SEQ ID NO:7562
SEQUENCE LENGTH:338
SEQUENCE TYPE:nucleic acid
TOPOLOGY:linear
CLONE:HUMGS08808
SEQUENCE DESCRIPTION:
GATCTGCCGA CCTCGGCCTC CCAAAGTGCT AGGATTACAG GTGTGAGCCA CCGCGCCCAG 60
CCATAACTTT NCTCTTTTCT CCATTAGCTA AAAGATAATA GTAAAACGTA TATTTGAACA 120
CTTAATTTTC TCTATATATC TTTTNAGGAT TATATTTAGT GTATTTCCCA ATTTTCCTAA 180
AATGATTATC ACTTCTGTAA AGAAGAAAAC AATACTACTG GTATTGCATT TGGAAATATT 240
TTNCTATTAA NNTGTCTCAT TTCCTTTNCA TGTATGCACA TACATGTNNT TNGTATAGCT 300
CCTTTNNTCA AAANNTATTC TGNGGTTATT ATTTANNN 338

SEQ ID NO:7563
SEQUENCE LENGTH:338
SEQUENCE TYPE:nucleic acid
TOPOLOGY:linear
CLONE:HUMGS08809
SEQUENCE DESCRIPTION:
GATCTTGTGG TCAAGAGCAA TCTGAATCCA AATGCAAAGN AGTTTGTCCC TGGGGTGAAG 60
TACGGAAATA TTTAAGTAGA CGGGGCCCTC TTTTGGTGGA TGTAGCACAA TTNCCACACT 120
GTGAAGGCAG TATTAGAAGA CTTAATTGTA AAANCNCTCT TGTCACTGTG TTACACTTAT 180
GCATTGCCAA AGTTTTTNTN AGTCTTGCAT GCTTAATAAA NGTNCTGAGA CTGTTACTAA 240
GTAAAANGCT GTCAANCATT TCCTGANAAT AGANTTGGCC CCATGGCTTG ATGTGAAGAC 300
AGCAAGGNAA GANGCACCAG TCAAGTTGTG ANCAAGNN 338

SEQ ID NO:7564
SEQUENCE LENGTH:324
SEQUENCE TYPE:nucleic acid
TOPOLOGY:linear
CLONE:HUMGS08810
SEQUENCE DESCRIPTION:
GATCCCTGCC ACAAGTTCCT TGGGCAGTGG CCATGTCACC ATTGAGATGA AGATATACAA 60
CAGAAAATAG TGGCTGTGTT TGGAAGCTTC AGCCCTGCAC ATTTGAACTA GTCACTCTCC 120
CAGACTTGCG TGGGTCAGTT CTTTCTGAGT AGAGGACTTG CTGGTAAAGG GGCAGATGCT 180
TTTTATTAGT ACTGATAAAA CAAACTGAGG GAAACATCCC TCTTAGCTGG GAAACTTTTA 240
CTCTTCAGGA GCTTGGCATC ATGGACTGTT AATGTATGTG ATTTTCCCCC TATTTTCTCT 300
CTCCAAAATG ATAAAAACAA TAAN 324

SEQ ID NO:7565
SEQUENCE LENGTH:318
SEQUENCE TYPE:nucleic acid
TOPOLOGY:linear
CLONE:HUMGS08811
SEQUENCE DESCRIPTION:

GATCCCACTA CGAAACGCTC CAGAGGCTTC GGTTTCGTCA CGTTCGCAGA CCCAGCAAGT 60
GTAGATAAAG TATTAGGTCA GCCCCACCAT GAGTTAGATT CCAAGACGAT TGACCCCAAA 120
GTTGCATTTC CTCGTCGAGC GCAACCCAAG GTGTTCTAGC TGAGCGTTTA AGAGCATGGA 180
CTCTGGAACC AGACTTTGAA TCCTTGCTCT GCCACTGCAG CTGTGTGACC TTGAGCAAGC 240
TATCTAAATA TTCTGTGCCT TTGTTTTGTC ATCTGTACAA TGGAGATGAT GATGGTATCA 300
CCTTCATGGG GTTGTCAN 318

SEQ ID NO:7566
SEQUENCE LENGTH:313
SEQUENCE TYPE:nucleic acid
TOPOLOGY:linear
CLONE:HUMGS08812
SEQUENCE DESCRIPTION:
GATCTGAGAC TCAGGAACCT TGGCCCCTTT GTCACTTACA ACTGTGTGTG AACAGGACTA 60
ACAGTTTGGA CTGAGAGCAC TGANGCTGCT GTGTGTTGTG CATATAACTT AATGCTGTGT 120
GTTGTGGCTT GCACTGCTGT GGGGGCGGGG AGGGCAGTGG TGGGGAATGG CTGGCATGAA 180
GCTTGCTTGA GCCAATGCTA TGCTTGCTGG GCTTTGGCTG ACTGGCTTTG GGCCACCAAC 240
TGTATTTGCA TAGTGATGGC TTGGAAGTGT CCATAGAAGG GATTGCAATA AAGGTGTGTC 300
TTCCTTCTAA AAA 313

SEQ ID NO:7567
SEQUENCE LENGTH:316
SEQUENCE TYPE:nucleic acid
TOPOLOGY:linear
CLONE:HUMGS08813
SEQUENCE DESCRIPTION:
GATCTTGAGT CAGAGAGGAT TGGAAGTGCT TTCTCCTCCA CCCAGGTGAG GTCAGGGGAG 60
CTTAGGTCTT AGGGAGATGG CAAGTTGAGG TATGAAGGGA AGCTGGGGCT TTTGGAGCTG 120
CCGAACAACT GAGGGACCCA GTGCGCCTTC CATCCCGCAC TAGTGAATAG CGCCCCCTCT 180
TCCCCCGAAA ACGAGGTGCG AGAGGAACAA TTCCCACGCT GGGGAAGGAC TTGTCTCCTT 240
TTCTGTGAAA ATGCTTTGTA AAAAGTTGTT ATTGTTTGCA TAGAGCAGAT TCTTGAGAAA 300
AACTGTTTTG GACCAN 316

SEQ ID NO:7568
SEQUENCE LENGTH:316
SEQUENCE TYPE:nucleic acid
TOPOLOGY:linear
CLONE:HUMGS08814
SEQUENCE DESCRIPTION:
GATCCCCGTC TGCTGACCAG CTAAACACCA ATTTCTTCCT GCTTTTCTCC ACTTGGTTTG 60
GAAAATCACA CAGTTTTCAG GCTCCATCTG TTTGGAGAAA ATACATTCTG AAGCATCCCC 120
AATTCACCTT CTAAAAACTC ATGTGCAGGT TTGATAAACA CCAGAACAGA AGACAGTGAT 180
GCTGTATTAT TTTAGATTTA TTACATAGAT TTGGAATTCA CTTTTTTCAT GACCTAGAAA 240
AAAACATTCC AGTGTTCANC TGTTTTNTAT TATTANGGGG CTTTTNATTT GTGAACTTCT 300
GANGGCATGA GTGTTN 316

SEQ ID NO:7569
SEQUENCE LENGTH:226
SEQUENCE TYPE:nucleic acid
TOPOLOGY:linear
CLONE:HUMGS08815
SEQUENCE DESCRIPTION:
GATCTGGTGT ACCAGGCGAA GTGGTCCGAG CAGGCTGAGC GATACGACGA AATGGTGGAG 60
TCAATNAAGA AAGTAGCAGG GATGGATGTG GAGCTGACAG TTGAAGAAAG AAACCTCCTA 120
TCTNTTGCAT ATAAGANTGT NATTGGNGCT AGNNGAGCCT CCTGGAGAAT AATCAGCAGC 180
ATTGANCAGA ANGAAGAAAN CAAGGGAGGA GAAGACAAGC TAAANN 226

SEQ ID NO:7570
SEQUENCE LENGTH:306
SEQUENCE TYPE:nucleic acid
TOPOLOGY:linear
CLONE:HUMGS08816
SEQUENCE DESCRIPTION:
GATCAAGAGT TAAATAAAAA GCCCCAGGAC GAAACAAGAN GAGAGCACTT CATGCCCTGT 60
GGTACAAAAA AGAGCAGCTC TACTTAATGC AATTCTTCAT TCAGCATATA GCGAGACATT 120
TCTCCTGCCT CATTTGAAAG ACATCCCAGC ACAGCATATC ACGCTGTTTC TTAAGTATTT 180
GTATNNNNNN GTACCTGAAG TGTAGCGAAA ATNCTACTAT GACTCTNCCT GGAATACACC 240
CACCTACCTT GANCCAGATT ATGGATTGGN TATGTCTACT TCTGGATGCA AATTTTNCTG 300
TTGTTN 306

SEQ ID NO:7571
SEQUENCE LENGTH:304
SEQUENCE TYPE:nucleic acid
TOPOLOGY:linear
CLONE:HUMGS08817
SEQUENCE DESCRIPTION:
GATCCTGCGA GGGTGTCATA ATACCTTGCT TTTCCATGCT TCCTGTATAT TTATCCATTT 60
CAGCACATCT GCAGTAGCAA TAACTNCTNA TTTNTAAATT TNCTTTTGTT GGGGGAGACA 120
GGCTTATGGC TGTCTGAGCA GCTGCTGTAG TGTAAGCAGT GAGGTTAGCA GTGGTTGTNA 180
GATTTNCTCA TTCCTGAGCA CCATGCTGTT GTGTCAGCAG TTGTAATGGA CTGTGTGGGC 240
ATACTTCCAG TAGGTGATGC TTGCAAGTAN GANCCAGCTG CAGTGGTAGC AGTAGGGTTT 300
GTAN 304

SEQ ID NO:7572
SEQUENCE LENGTH:30
SEQUENCE TYPE:nucleic acid
TOPOLOGY:linear
CLONE:HUMGS08818
SEQUENCE DESCRIPTION:
GATCTGTACA AATAAAATNC CAAGTCCAAA 30

SEQ ID NO:7573
SEQUENCE LENGTH:118
SEQUENCE TYPE:nucleic acid
TOPOLOGY:linear
CLONE:HUMGS08819
SEQUENCE DESCRIPTION:
GATCATCTCT CCTTGGGTTT TNNTTTAAAA AGGGGAATCT NCTATAAAGG TTCTNTTGCT 60
TCAAACCAAT GTCAAATAGA CTTNATTTTA AGAGTCATGG AATTACAGTG CAACCTTN 118

SEQ ID NO:7574
SEQUENCE LENGTH:53
SEQUENCE TYPE:nucleic acid
TOPOLOGY:linear
CLONE:HUMGS08820
SEQUENCE DESCRIPTION:
GATCTACCTA TACAGTCCTA CATTAGCTTC TAAAATATTT GTCAGGAGGG AAA 53

SEQ ID NO:7575
SEQUENCE LENGTH:102
SEQUENCE TYPE:nucleic acid
TOPOLOGY:linear
CLONE:HUMGS08821
SEQUENCE DESCRIPTION:
GATCCCGAGG CTTTGTCTTC CTCTCGTCAG TTCTTTTGGT TGTGTTTTTT GTTTTTNTTT 60
NAANAACTCA AAAAAAAAAN AAAAGACTTG GAGGAAGGGA AA 102

SEQ ID NO:7576
SEQUENCE LENGTH:362
SEQUENCE TYPE:nucleic acid
TOPOLOGY:linear
CLONE:HUMGS08822
SEQUENCE DESCRIPTION:
GATCCTACTG ACAGCCATCG TGCAGGTGCT CAGCTGCTTC TNTCTCTATG TCTGGTCCTT 60
CTGGCTTCTG GCTCCAGGCC GGGCCCTTNA CCTCCTGTGG GTGAATGTNC TGGGCCCCTG 120
GTTCACTGCA GACAGTGGCA CCCCAGCACC AGAGCACAAT GAGAAACGGC AGCGCCGACA 180
GGAGCGGCGG CAGATGAAGC GGTTATAGCC ATTGANATTG TGGCCACAGG CCACTGGGCC 240
CTGGGTGGCT CTGTNAGGGT GCACAGCCCC TCATGCCTGG AGCAATGAGG GTCTAGTCCA 300
GGGGCCAAAA GCAGTCTTGA GGGTATTGGG GTATACTTAT ACTCTATAGG GGTCGTTTGA 360
AN 362

SEQ ID NO:7577
SEQUENCE LENGTH:109
SEQUENCE TYPE:nucleic acid
TOPOLOGY:linear

CLONE:HUMGS08823
SEQUENCE DESCRIPTION:
GATCACTCAT ATAACATGTC ATTTTTTTGG TTCTGTTTTG GTTTGGTTTT TGCCAATNAT 60
TTTGTTATAT TTCCAAAAAA CTAAATAAAA ATNATTTTAA TTTTTNAAA 109

SEQ ID NO:7578
SEQUENCE LENGTH:361
SEQUENCE TYPE:nucleic acid
TOPOLOGY:linear
CLONE:HUMGS08824
SEQUENCE DESCRIPTION:
GATCTTCACG GGGCTATTAT TTCAGTGACA AAATCCAAAT GCCCCTCTTA TGTGGGTATT 60
ACAGGAATCC TTCTACAGGA AACAAAGCAC ATTTTCAAAA TTATCACCAA AGAAGACCGC 120
CTGAAAGTTA TCCCCAAGCT AAACTGCGTG TTCACTGTGG AAACCGATGG CTTTATTTCC 180
TACATTTACG GGAGCAAATT CCAGCTTCGG TCAAGTGAAC GGTCTGCGAA GAAGTTCAAA 240
GCGAAGGGNN CGATTGACCT GTGANTTCTT TGCCGTCTAA GGCAGTTGTT TATGACAGCT 300
GAAAACTGGA CACTCCCTAA NTGTCCACCT TTCAGTGAAG NGATAGTTAA GCCAATTCCN 360
N 361

SEQ ID NO:7579
SEQUENCE LENGTH:323
SEQUENCE TYPE:nucleic acid
TOPOLOGY:linear
CLONE:HUMGS08825
SEQUENCE DESCRIPTION:
GATCCAATTC GTGTCTGTGG ATGCTGCAAG GACTGGCTCA TTGGAGCCAA ACCATGTGGC 60
GGAAACTCCT GAAATTGGTG GAAAAAGTGG AATATGGCAG ATTGGGTAGG TAGGAGAACA 120
ATTTTGGAGA TTTTTTTTCT TATGTTAAAG TGTCTGCATT TTATTTGCTG TACCCTGCCT 180
ACTCTGGTTT TNAGGATTTT TTGACTTCTT CGAAAAGCAC TGAATAATCT CTTTNCCGGG 240
TCTAGATTTT TTGATGAGTA CTGTGTAAAA TTGTCAATAT TCAATGGATT ATTTAATAAT 300
ANNTTAAGAN TTCCACTCAT AAN 323

SEQ ID NO:7580
SEQUENCE LENGTH:183
SEQUENCE TYPE:nucleic acid
TOPOLOGY:linear
CLONE:HUMGS08826
SEQUENCE DESCRIPTION:
GATCCTAAGT TAAGACTCTC CAAAGCAAAC ANTTGGTTTA AATCATTTAT CTGATGTAGA 60
TAAAAGGTAG AGAAACTTGT AGTGTTCTCC ACATAATGGA AAATGTACAA ATGCCTAGTT 120
GTGTTGCCCA TCAATTTTGT ATATNTCCAT AATTTTNATT GTTGANAATT GCATTATNTT 180
TTN 183

SEQ ID NO:7581
SEQUENCE LENGTH:350

SEQUENCE TYPE:nucleic acid
TOPOLOGY:linear
CLONE:HUMGS08827
SEQUENCE DESCRIPTION:
GATCCACCTG CCTCGGCCTC CCAAAGTGCT GGGATTACAG GCAGGAGCCA CCGCGCCTGG 60
GCCTTTTTTT AAGTTTTAAG NNCCNATAAA GNCACTGAAA GGTGATGTGT GTGGATGAGC 120
TAGGAAGACC TGAAATAGGC TCTCTCTAAA TTAATCAAAT TAATCCTGAA GCCATTCTGC 180
AATACTGTCT TTAATGTATA CTCACTTGTT ATAGAAGCCA GGGTTTTTNC CCCTAATTTG 240
TATCATTGCT ATATGTGTTA TTGTACCAAA CTACACTGTT TTAATTGCTG TAAATTTTAA 300
TATGTCTTAG TATCTGGGTG TGGGAATCTT GANAGCATGG AGTTTGTGTN 350

SEQ ID NO:7582
SEQUENCE LENGTH:348
SEQUENCE TYPE:nucleic acid
TOPOLOGY:linear
CLONE:HUMGS08828
SEQUENCE DESCRIPTION:
GATCCTTGGG AGAGCTATCA CGGGAATCCA GGCGTTCACG GCCAGCAAGT GAGCCGCTCC 60
ATCAGGGGCC CGAAACTNTC AAGCCCCTTT NTGGAGAGNT GCCTGGCTGG ACCGTAGAGC 120
GCTGTGCTCT GAGCTTAGAA AGGGAGGTGG CGGATGGAGT GGGAAGTGAG AGACACTGAT 180
TTTNAAATAT CAAAATTTCC CTTCTGAAGT CGTTCAGATG TNTTCCTTAA AAAGAAGATG 240
GAATTCTCTG TAGAGCGTCT CANTCCACTT TTAACCATGG NTGAGAGCAG ACTCCATTTA 300
CCNTGAAATA GCAGCTTCTT TTGAGGGGAG AGTGACATGG AAGCAACN 348

SEQ ID NO:7583
SEQUENCE LENGTH:258
SEQUENCE TYPE:nucleic acid
TOPOLOGY:linear
CLONE:HUMGS08829
SEQUENCE DESCRIPTION:
GATCTGCAGG TTTGGGCATA TGCTTTCATC ATTAAATTAT CTGATAAAGT TACAAGTCAC 60
AAAGGAGAAT GAGAACTTAA TGATTCTATT GGATTTAATA TATTAGCAAG AAANCATACT 120
ATTTNCATAT GTNTAGCTTA GTAAGGCATT ANCATAAGTN CAANACCTAT GANACAGATG 180
CNTATTTCCT CAACATACTG TGTCAGGTAT ACTGTTTTAT ANTTNGGTTG TTTTAGCCTT 240
ATTGCACACC NNCTCCCN 258

SEQ ID NO:7584
SEQUENCE LENGTH:308
SEQUENCE TYPE:nucleic acid
TOPOLOGY:linear
CLONE:HUMGS08830
SEQUENCE DESCRIPTION:
GATCCATTTC ATTACAAGTA AAATATTACA AAATATTTAC AGGAAAATAT TAAAAATAAC 60
TCAAACACAA AAGGCAAGAT GAAATAAACT GTTTTTTTTT CTCCAGAAAT CTCTACTCCA 120
GTGCCCACAG CACACAAGAG TCAAAACAAA TAAGCAACTA AGNTCCTTAC GGAAAAGGNA 180

CAGNTTGTTC CTAAACCAGA AGAGGAGGTT GCCCAGAAGA AAAAGATATC CCGGAGGAAC 240
CTGAGGANCC NAAACTTTTT GCCCCGGGGG TAATTCNGNC TTTNNATTAA TTTTNTTTAN 300
NGGGNNNN 308

SEQ ID NO:7585
SEQUENCE LENGTH:36
SEQUENCE TYPE:nucleic acid
TOPOLOGY:linear
CLONE:HUMGS08831
SEQUENCE DESCRIPTION:
GATCTCTTAT CATAATAAAG AATCTTTGTC ATCAAA 36

SEQ ID NO:7586
SEQUENCE LENGTH:227
SEQUENCE TYPE:nucleic acid
TOPOLOGY:linear
CLONE:HUMGS08832
SEQUENCE DESCRIPTION:
GATCTAAGGA GGCCAGTGAA TTTCAAACAG GATTAAAAAG AAAAAAAACA GAGTTAGGTA 60
TCAAATTAAG AATACTGAAA GCAAAGATAT AANTAGAACT TTAAAAGCAG CCAGAAAAAC 120
AAACATATTA CATATAGGGC AGCAACANTA AGANTAACCG CTAACTTTTG ATTAANNGTA 180
ATATAATTCA CAATACANTG TAACANCANT TTTNNGTGCT AACNGGN 227

SEQ ID NO:7587
SEQUENCE LENGTH:215
SEQUENCE TYPE:nucleic acid
TOPOLOGY:linear
CLONE:HUMGS08833
SEQUENCE DESCRIPTION:
GATCTATTGA GAATGGAATT GTTTTCTTAA TTTCATTTTC AAATTGTTTG TTGCTAGCAT 60
ATAGAAATAC AAGTGATTTT NTATATTGCT CTTGTATCCT GTGACTTTGC TGAGCTTATT 120
TTAGTAGTTT TTTTTCACTG AATTCTTTAG GATTTTNTAC ATAAAAAATT CATATCATCT 180
GTGAATAAAG ACAGTTTTAT TTCTNAATTT TTAAA 215

SEQ ID NO:7588
SEQUENCE LENGTH:43
SEQUENCE TYPE:nucleic acid
TOPOLOGY:linear
CLONE:HUMGS08834
SEQUENCE DESCRIPTION:
GATCATATTT TTAAATAAAG CATTTTTTGT AGAAAGTGCT AAA 43

SEQ ID NO:7589
SEQUENCE LENGTH:334
SEQUENCE TYPE:nucleic acid

TOPOLOGY:linear
CLONE:HUMGS08835
SEQUENCE DESCRIPTION:
GATCAGGATA ACCAGGATGA TAGCAGTGAC GATGGATTCC TCGCTGATGA CAACTGCAGT 60
TCAGAAATAG GACAGTGGCA TNTAAAGCCT ACTATCTGTA AACAACCTTG TATCCTACTT 120
ATGGNCTCAC TCCGAGGCCC TTCTCGGTCA AATGTTGTCA AAATTTTAAG AGAGTATTTA 180
GANGTGGAAT GGGAAGTTAA AANAGGAAGC NNNAGANGTT TTTCCAAAGA GAAAAGAAAG 240
CATAAGGNCA CTTACTCAAC AGANGCACCT TTAGGCGGNG GANCAGNACA TTGTGTCAAT 300
AGTNTCTCNG GTTTGCCCTT TCTNTTACTT GTCN 334

SEQ ID NO:7590
SEQUENCE LENGTH:235
SEQUENCE TYPE:nucleic acid
TOPOLOGY:linear
CLONE:HUMGS08836
SEQUENCE DESCRIPTION:
GATCAAATTT AAAAATCAGT TTTAAAAGTG GTTCCCGACT TCTTTCCTTT GGAAGAATTT 60
GGNCTNATGC TCATAAGCAG TTCATGAGTG ACAAGTGTAG CAAAAGCCAG CCATATATTT 120
CNAGANTCTA TATCCTCAGG AAATGGTCCT TTTTTTTCCT ATAANTCACC AACCAACTAT 180
CTAANTGGNC ATTTGGGGGA NTTCCCTCCC AAATTTGATT TTNTNATTNG AAAAN 235

SEQ ID NO:7591
SEQUENCE LENGTH:172
SEQUENCE TYPE:nucleic acid
TOPOLOGY:linear
CLONE:HUMGS08837
SEQUENCE DESCRIPTION:
GATCCAGAGC ACAGATGGCA AGTTGTGTGG ATGATACTGA ATATNTTTAT TTTCCTAAAG 60
ATAAATNTCA CGACACGACA TTTCTCAGTT TTAATTTAGA TGTTTATNAC CATGTGTTAA 120
CAGAATCTTA AAACCCAAGG NATTTCTTCA GTAAACTAGA CTTTGATTCA AA 172

SEQ ID NO:7592
SEQUENCE LENGTH:335
SEQUENCE TYPE:nucleic acid
TOPOLOGY:linear
CLONE:HUMGS08838
SEQUENCE DESCRIPTION:
GATCAAGAGG AAGCATGACT TGCACAAGAT GGCAGAGGCC AACCGTGCCC TGGCCCACTA 60
CCGCTGGTGG TAGAGTCTCC AGGAGGAGCC CAGGGCCCTC TGCCGCAAGA AACAGTGTGA 120
GCTACTGCCA CGCTGAAAAC TACCTGTGGG TTAAGGATGT AGTTCCTTTG TAAGGGTGGG 180
CAGGCCTCGT AAGAAAGATG TAGCAGCATA TTCACTATCC GTTAATCCTT CTTTCTTTGA 240
GGCTGGAACT TGCTCTCTCT GCCCCTATTT CCTTGTAAAG AGGGAGCACA TTGACTTGGG 300
AATTTCCTCC AGGAAACTCA GGGCTGTTTT CTCTN 335

SEQ ID NO:7593

SEQUENCE LENGTH:333
SEQUENCE TYPE:nucleic acid
TOPOLOGY:linear
CLONE:HUMGS08839
SEQUENCE DESCRIPTION:
GATCTGGGAC TTCTTTAGTA TCGAGTCTCC TTTGCACTCT TAAAAATTGT TGAGGACCCC 60
AGAGAGCTTT TGTTTTTATG GGTTGTATCT ATCAATATTT ATCACATGAG AAATNATTTA 120
TTTATTTTAA AATAACTTTT TTATTGAGAA AGATAGCAGT GTTTTTTTTT AATCTTGCAA 180
ATTGTTTAAT GTTTGATTTA ATGGAAGACA GCCAGATTGT CCTATCAGCA TTCAAACTGC 240
TGCAGTAAGT TGTTTTGACT AATGTATATG AGGAAAACCT GACCTCACTG AGATTTTAAT 300
TGGAAAATGG AGGAGTATTT TNCCAGCCTT TNN 333

SEQ ID NO:7594
SEQUENCE LENGTH:220
SEQUENCE TYPE:nucleic acid
TOPOLOGY:linear
CLONE:HUMGS08840
SEQUENCE DESCRIPTION:
GATCTCCTCC ATACGGAACT TTCTCATCTA CGTGGCCCTC CTGCGAGTCA CTCCATTTAT 60
CTTAAAGAAA TTGGACAGCA TATGAAGACA GGACATCACA TATGAATGCA CGATATGAAG 120
AGCCTGGTTA CAGTTTCGAC TCCTCTCTGC AAGTGAATAG GCCCAGAAAG GTGTAAGAGA 180
CTCTTTGAAT GGACATAAAA TTCTGCTTGT TAAGAACAAA 220

SEQ ID NO:7595
SEQUENCE LENGTH:326
SEQUENCE TYPE:nucleic acid
TOPOLOGY:linear
CLONE:HUMGS08841
SEQUENCE DESCRIPTION:
GATCTGTGCT GGCCACGTGC CCGGNGTCTC AGCTCTGAAC CTGCTGTCCC TGCTGAGAAG 60
CTCTGAGGGC CCCTCCCTGG AGGACCTGTG AGGGTGGCTG GCCCCTGGGC TGCCCCTTCT 120
NATGGCTTCG TGCTGACTCC ATAAACATTC TCTGTTGAGG ATGTCCAGTC AGGGCTTGAC 180
AGGCCCAGGN TCAGCCCGCC GTGGCTGGGA AGGTTCCCTG CAGTGCCAGT GCTGCAGCAG 240
GGAGAGCTGG GCAGAAGCAG CGAGGGGGCC CAGCTGGCGA GACTGTAGCC CCGTCCCACT 300
CCCACACTCA CTCTTGTTAG AGCCTN 326

SEQ ID NO:7596
SEQUENCE LENGTH:327
SEQUENCE TYPE:nucleic acid
TOPOLOGY:linear
CLONE:HUMGS08842
SEQUENCE DESCRIPTION:
GATCAGATGT TGGAACGTGC TATGCTGTAG CGTGTCTGGA AACAAAGTAC ACAAACCTGG 60
CTACGGTGAT GAGTTAGCTT CTGCTTACTA CCTGTNACAA CCCAAGTGGG TGACACTAGT 120
GAACCTTCTC CAGTCTGCAG GCTGGCATAG AAGGCTCTTA GATTATATTG GGCAGCTTGC 180

AATCTGCCGA AGCAGTGACT TGCATTTCCA CACTTGGCTT GAGCACTCAA CCCAGAAGGC 240
GAAGATAGCT TTTGGTTGTA GGCGGCTTCC TGTATGGGAT ATCCCTCGGT AAGGGTAAAG 300
GAGCAGAGGC AAAGGAGAAA AGCAGTN 327

SEQ ID NO:7597
SEQUENCE LENGTH:278
SEQUENCE TYPE:nucleic acid
TOPOLOGY:linear
CLONE:HUMGS08843
SEQUENCE DESCRIPTION:
GATCTTTCCT CCCTCCCCTG CATGAGGCAG AGGCAAGCTG CCTGCCAACC CCCTCCCTCA 60
AGGAATGGCC TTGCCCAGGA ATGCCCACCA CACATACCCT CTNCTTTTTT NNTAGTCAAA 120
CTCTTGTTTA TTCCTTGGNT TGCNTCCCTC CTTCCTCCCC TCTCAACCTT TACTTCTGAT 180
TTNNATTTCA TGGAATTTGG GATTGANGTT AAACTACAAC AGTGCCGCCA ACACCAAGTN 240
TTGCAGGAAA AAAATACAAA GAAATTTANC ANANAANN 278

SEQ ID NO:7598
SEQUENCE LENGTH:202
SEQUENCE TYPE:nucleic acid
TOPOLOGY:linear
CLONE:HUMGS08844
SEQUENCE DESCRIPTION:
GATCTTCATT ACCGTTNCAT GATTGGAAAT ANTTAAAACA TTGTACAGTT TTAGTATAGA 60
GAAATNTAAT GGNTTTTTGT AACCAGTTTC TGTCTGCATG TAATTTGGAT TTCTCAAATA 120
CATTCATTAG TAATTNATCA GTAACATTAG TTTTATTTNN GTCCATCTCC TTATCTATAA 180
AANGGGGATA TNCTTAGGAT NN 202

SEQ ID NO:7599
SEQUENCE LENGTH:47
SEQUENCE TYPE:nucleic acid
TOPOLOGY:linear
CLONE:HUMGS08845
SEQUENCE DESCRIPTION:
GATCCATAAT ATAGCATCAC ATCTCAATAA ATTTATTTGG AAGCAAA 47

SEQ ID NO:7600
SEQUENCE LENGTH:327
SEQUENCE TYPE:nucleic acid
TOPOLOGY:linear
CLONE:HUMGS08846
SEQUENCE DESCRIPTION:
GATCTGCCCA CCTTGGCCTC CCAAAGTGCT GGGATTACAG GCATGAGCCA CTGTGCCTGG 60
CCCCTTCCTG TAAAATTTTN AAATGGAGAA TTGGGTGCNA GATGTGGTTT CCAGCCTGGT 120
GCCTGGGGTG CTGAGCTANN NGAGTGGTGC AGTCCAGGAC ACCTTTGCTT TATCTCACTT 180
ACACGGTCAC CTGGAGCCGG CTCAAGTGGC TAAAGCATCC TGGGGCCCAG AGCCAGGTGA 240

TAGGTCCCTN TGGCCAACTG GACAGTTGAG GCCTGTGGTT ACCCGAAGCC CAGCTTGGGG 300
CCCTGGTCCA GCCTNGCCTC CCAGGAN 327

SEQ ID NO:7601
SEQUENCE LENGTH:326
SEQUENCE TYPE:nucleic acid
TOPOLOGY:linear
CLONE:HUMGS08848
SEQUENCE DESCRIPTION:
GATCTAGATG CTGGTATTGA GGTGGGAGAC AAGTACTGCC ACCNGAAACA ACAGTCTTGG 60
TAAATTTAGC CGACGAGGGT AAACACATCC TAACAGGGAA GGTAAACTGT ACGTCCATCA 120
GTACCACTAG AGGGCATCNC NGGTTTATAG TTCAATACAG TGAATATATC AGANTAATGG 180
CCTTTAGTTT TCCTGAAAGA TTAANTTAGG CTTGCTAACT TGTTTAATGN GATANTCAAA 240
CATATGATGT AATTTTAAAG GGTTTACATT TTTANAANTT TAATGGTTGC TACATAAAAN 300
GGGNTTCAGT TAACTNATTT TGCGTN 326

SEQ ID NO:7602
SEQUENCE LENGTH:310
SEQUENCE TYPE:nucleic acid
TOPOLOGY:linear
CLONE:HUMGS08849
SEQUENCE DESCRIPTION:
GATCTCAGCA GGGAACCATG GAAGCACCCA CCCCCTTGCA TTTTGGCTTA CCTGTAGCGG 60
AATGCAACAG AGAATTTTAT ATGCTAAGAT TTGTGGTTCC GTGGGAGAAT ATTTCAAACA 120
GGTGGAAACC ATCAAACTAA AAGAGAACCA GAAATCATTT AAAGGTCACG TGAGGAGCTT 180
CTGTNTTGTG CAACTTAAGC CTTCACCAGT CTCCTATGCC CAGGTGCCCA GCACCCCCAT 240
CATCTGCATA TTCACCATCC TCAGGTCAGA TTCTATGATA CTGNNAATAA AGGCATTCTG 300
GAAAANTAAA 310

SEQ ID NO:7603
SEQUENCE LENGTH:287
SEQUENCE TYPE:nucleic acid
TOPOLOGY:linear
CLONE:HUMGS08850
SEQUENCE DESCRIPTION:
GATCCGGTCA GCAGCCCCTG TGGCTAGAGG AACACCAGCA CAAACGACAG CCTCAAGTCT 60
CCTTCGAGCT TTATATCCAT TTGGGGATGA AGTCTACTTT NACAGCTAGC AAGGCGACAT 120
GCAACTGTTG TTGAATGATG ACAGCAATTC AGGAAAGACT TAAATATGAA AGCAAATTGA 180
NCACATCGGG TGTTTGTNAT CAGANNAGNG NTGNGATGTG ATAAGACTTG TTTATTGACT 240
AGCCAATATG TCATTCACCT TCNGGTTTAT NTTGTGNANC CCCANNN 287

SEQ ID NO:7604
SEQUENCE LENGTH:66
SEQUENCE TYPE:nucleic acid
TOPOLOGY:linear

CLONE:HUMGS08851
SEQUENCE DESCRIPTION:
GATCGAGTTT TGGTTCGGGT TAACTGTGTG CCTACTGAAC CTGGCAAATA AACATCACCC 60
TGCAAA 66

SEQ ID NO:7605
SEQUENCE LENGTH:339
SEQUENCE TYPE:nucleic acid
TOPOLOGY:linear
CLONE:HUMGS08852
SEQUENCE DESCRIPTION:
GATCTGGAAT GTGAAGCGTT ATAGAAGATA ACTGGCCTCA TTTCTTCAAA ATATCAAGTG 60
TTGGGAAAGA AAAAAGGAAG TGGAATGGGT AACTCTTCTT GATTAAAAGT TATGTAATAA 120
CCAAATGCAA TGTGAAATAT TTTACTGGAC TCTATTTTGA AAAACCATCT GTAAAAGACT 180
GAGGTGGGGG TGGGAGGCCA GCACGGTGGT GAGGCAGTTG AGAAAATTTG ANTGTGGATT 240
AGATTTTGAN TGATATTGGA TAATTATTGG TAATTTTATG AGCTGTGAGA AGGGTGTTGT 300
AGTTTATAAA AGACTGTCTT AATTTGCATA CTTAAGNTN 339

SEQ ID NO:7606
SEQUENCE LENGTH:337
SEQUENCE TYPE:nucleic acid
TOPOLOGY:linear
CLONE:HUMGS08853
SEQUENCE DESCRIPTION:
GATCCTTTGT GAGCAAGTTC TATTTGTTCA TTGCTTGCCA GAGATGAACA CAGAATGTTC 60
TGTTTCATTT TACAAGAACT ATCCTGAGTT TCTGTGGATG GAAACATTAC ATGTAATGCA 120
GATATAGTGA ACACTGGAAA GATTTATTAA AGANTTATAT TTGTGTATAC TTTATAAATT 180
AGTCCCTCAT TAGATTTTTT TTTTCTTAAG CATAAGACTG ANCTTAAATG TGTTAATTTT 240
AGTAGAATCA GGCACTGCTC GCAGAAGGAN CACAGATTGT NGAGATTAAC ATAAATNGTT 300
CTTGTTCTAA TATATATNTN TNNCCATTTC TGTCGAN 337

SEQ ID NO:7607
SEQUENCE LENGTH:203
SEQUENCE TYPE:nucleic acid
TOPOLOGY:linear
CLONE:HUMGS08854
SEQUENCE DESCRIPTION:
GATCCGTGGA TTGAGGGTGC CTTTCCATTT ATTTTGGTCT TCTTTACTTT CTTTCAACCA 60
AGTTTTGTAG TTTTCAGAGT ATAAATTTTA TACTTGTTTT GTTCAGCCTA TTACTGTTTT 120
ATTTTTGGTG CTATTGTAAA TNAAATCTTC TTAATTTCAA TTTCAGATTA TTCACTTAAA 180
TATACAGAAA TACAGCTGAT AAA 203

SEQ ID NO:7608
SEQUENCE LENGTH:207
SEQUENCE TYPE:nucleic acid

TOPOLOGY:linear
CLONE:HUMGS08855
SEQUENCE DESCRIPTION:
GATCAAAACT TTTCATAATA CAAAGACATT ATTATTTGNT TTTTTCACTG TCCCCTCCTG 60
AATATACAAC GGAGCATTCC AGATGCTAGA GTGACAGCCT CACTCAGACT CAGGGGATGC 120
ATGCTTGCNT GTACAGTACT CGTATGTTTT AAATTTGTTT TAACTCCTAA TATGTAAGTA 180
TCAATAAATA TAATCACCTT AAGTAAA 207

SEQ ID NO:7609
SEQUENCE LENGTH:249
SEQUENCE TYPE:nucleic acid
TOPOLOGY:linear
CLONE:HUMGS08856
SEQUENCE DESCRIPTION:
GATCTTCGTG GTGGAAAGCT AAATTTTAAA ACCACCCCAA TGGATGCAGA CAGTGATGTT 60
GCATTGGACA TTCTAATTAC AAATGTAGTC TGTGTTTTNA GAACAAGATG TCATTTAANC 120
TTAAGGAAGA TTGCTTTGGA AGGNGCAAAT GTAATTNATA ANCGTGATGT TGGAAAAGTA 180
TTANTGAGGC TTAGAAAACC TAGANTTACA GCTACANTTN GGTCCTCAGG AAANNTTATT 240
TGCNCTGGN 249

SEQ ID NO:7610
SEQUENCE LENGTH:319
SEQUENCE TYPE:nucleic acid
TOPOLOGY:linear
CLONE:HUMGS08857
SEQUENCE DESCRIPTION:
GATCTGTAGT ATTTTAATGT GCATCTACTT TAAATGAGTC ATCTTGGGGT TTTTATAATT 60
CCCTTATGTT CTCGCCCCTC TACACTTGAA ATAACAAAAT GCCTTAATTT TATGGATTAG 120
NTCTCTTATA GGAGACAGGC AGCTATATGN AGCAANCCCA GTAAAGTTAT TTTTCAACTN 180
TCANAGTTGT AAAATATTTT ATAACAGTTT ACANCAACAG CTGNAGANCA ACATGCCACA 240
TTTTNTTTGC AGCATTTTCA AATANTTTGT TTTGTGGTGT NAGCACAGGN TAAANNTGGG 300
GNGCGTCGAT GTGAGGCGN 319

SEQ ID NO:7611
SEQUENCE LENGTH:208
SEQUENCE TYPE:nucleic acid
TOPOLOGY:linear
CLONE:HUMGS08858
SEQUENCE DESCRIPTION:
GATCTACAAA AAATTCCTGG AGCCATATAT ATACCCTCTG GTTTCCCCCT TCGTTAGTCG 60
TATATGGCCT ANGAAAGCAA TACAAGANTC CAATGATACA AACAANGGCA ANGTAAACTT 120
TAAGGGTGCA GACATGAATG GTTTACCAAC AAAAGGNCCA ACAGAAATCT GTGATAANNC 180
GNAAGACTAA NGAAATTTTC CNAAAGGN 208

SEQ ID NO:7612

SEQUENCE LENGTH:321
SEQUENCE TYPE:nucleic acid
TOPOLOGY:linear
CLONE:HUMGS08859
SEQUENCE DESCRIPTION:
GATCTTCCCA CCTTCAGCCT TCCAAGTAGC TGGGACTACA GATGCACACC TCCAAACCTG 60
GGTAGTTTTT NAAGTTTTTT TGTAGAGGTG GTCTAGCCAT GTTGCCTAGG CTCCCGAACT 120
CCTGAGCTCA AGCAATCCTG CTTCAGCCTC CCAAAGTACT GGGATTACAG GCATCTNCTG 180
TAGTATATAG GTCATGAGGG ATATGGGATG TGGTACTTAT GAGACAGAAA TGCTTACAGG 240
ATGTTTTTNT GTAACCATCC TGGTCANCTT AGCAGAAATG CTGCGCTGGG TATAATAAAG 300
CTTTTCTACT NCTAGTCTAA A 321

SEQ ID NO:7613
SEQUENCE LENGTH:136
SEQUENCE TYPE:nucleic acid
TOPOLOGY:linear
CLONE:HUMGS08860
SEQUENCE DESCRIPTION:
GATCCTTCCT AGGGGATGGG GGAAGCCCTG GCTGCAGGCA GCCTTCCAGG CAATATGNNG 60
ATAGGAGGCC CACGGGCCTG GCAGTGAGAG GTGTGGCCCC ACACCGATTT ATGATATTAA 120
AATCTCAACT CCCAAA 136

SEQ ID NO:7614
SEQUENCE LENGTH:47
SEQUENCE TYPE:nucleic acid
TOPOLOGY:linear
CLONE:HUMGS08861
SEQUENCE DESCRIPTION:
GATCCCGTCT CTACAAAAAA TAAAAAAAAA TTAGTCGTGG TGCCAAA 47

SEQ ID NO:7615
SEQUENCE LENGTH:36
SEQUENCE TYPE:nucleic acid
TOPOLOGY:linear
CLONE:HUMGS08862
SEQUENCE DESCRIPTION:
GATCACTTTT TCAATATTAA ATNTTATTTA CATAAA 36

SEQ ID NO:7616
SEQUENCE LENGTH:260
SEQUENCE TYPE:nucleic acid
TOPOLOGY:linear
CLONE:HUMGS08863
SEQUENCE DESCRIPTION:
GATCATCATG AAATNATAAG AGGGCTTAAG AATTTGTCCA TTTGCATTCG GAAAAGAATG 60

ACCAGCAAAA GGTTTACTAA TACCTCTCCC TTTGGGGATT TAATGTCTGG TGCTGCCGCC 120
TGAGTTTCAA GAATTAAAGC TGCAAGAGGA CTCCAGGAGC AAAAGAAACA CAATATAGAG 180
GGTTGGAGTT GTTAGCAATT TCATTCAAAA TGCCAACTGG AGAAGTCTGT TTTTAAATAC 240
ATTTTGTTGT TATTTTTAAA 260

SEQ ID NO:7617
SEQUENCE LENGTH:102
SEQUENCE TYPE:nucleic acid
TOPOLOGY:linear
CLONE:HUMGS08864
SEQUENCE DESCRIPTION:
GATCAACTTT AGTCACCAAC CCAAATGGAG GATTTNTGGC TTGTGGGNCC TTATATGCCT 60
ATAGNTGTGG ACATTTGCAT TACACAACTG GANTCTTTNC TN 102

SEQ ID NO:7618
SEQUENCE LENGTH:228
SEQUENCE TYPE:nucleic acid
TOPOLOGY:linear
CLONE:HUMGS08865
SEQUENCE DESCRIPTION:
GATCAAGGGG CCTCTCAGAA CCATGTTCCC CAGCCAGGTG AGGACCATTT TAACTGGGAC 60
CCAGGNCAAA ACCATGTGGG TGCACAAAGC CAGGCACTGC CAAGTGGAAC ATGAGGTTAT 120
TTCCAAATCA TGGGAGCCAC CAGCAGGGAG AGGGCAGTAT GGAAAATCCC CTGGAGCCGG 180
TCAACTNTTT GCTCATGGCT AGTGAAATAA AGTTGTTTGA GTACTAAA 228

SEQ ID NO:7619
SEQUENCE LENGTH:113
SEQUENCE TYPE:nucleic acid
TOPOLOGY:linear
CLONE:HUMGS08866
SEQUENCE DESCRIPTION:
GATCTCTTAC TCCCCCAGTT TGAATGGTAA ATTTGAATGG TAAATTCCCA TGAACATATA 60
TGGAAATATC TTTATCCTAC TTTNTCCAAT AAAGGCTGTT CTTAGCTTTT AAA 113

SEQ ID NO:7620
SEQUENCE LENGTH:279
SEQUENCE TYPE:nucleic acid
TOPOLOGY:linear
CLONE:HUMGS08867
SEQUENCE DESCRIPTION:
GATCACCTGN CAAATGTTTG AGGACGGAGC TGTGCAGTCA CATTATTGGG GATTCCACAG 60
CTGGTGCTGC AGGCCTTGCG CCTCCAACCA GGACTTTNTT CTTAATGCTC TCGACACTTA 120
GCTAAACACG ACTATATCCC GGCCCAGCAG GCCCCAGCGC CGTTAGTCTC CAGCTGACTC 180
TGTGGGTTGG TCTTAAAGCA AATTCTGTTT TGTGGACTGC CTGGCAATTT TTNAGCTAAC 240
TGTAATGATA AAAAGGGAGT NTTAATCTAT TCTGAATCN 279

SEQ ID NO:7621
SEQUENCE LENGTH:198
SEQUENCE TYPE:nucleic acid
TOPOLOGY:linear
CLONE:HUMGS08868
SEQUENCE DESCRIPTION:

```
GATCAAGTTG TGGTGGATAA CGTGTTTCCA AATTGCATCT TGTTGCTGAA ACTTCCTGGA  60
CTTGAGAAGT TACTTCATCA TGTGACAGAG GAAAAAGGTA ATCCAGAAAT AGACAACAAG 120
AAATATTACA AGTNCAGCAA AGNGAAGACA TTAAAGTGGC TGGNAANNNN GGTTAATCAA 180
NCTGTGGCAG CATTAANN                                               198
```

SEQ ID NO:7622
SEQUENCE LENGTH:184
SEQUENCE TYPE:nucleic acid
TOPOLOGY:linear
CLONE:HUMGS08869
SEQUENCE DESCRIPTION:

```
GATCCAGAAA GTAGCAATAT AGGACTTTAA GGGGTATGTA TTAGTGTAAA TAATATATGT  60
GCAGTCTAGG CTAATCATNT TAATTAAAAA CAAATAAGCA TACTTTTTTN CTTTAAAAAT 120
ATGCCCTCAA AATGGTGGGG GATAAANTGT AAGATATTCT TTAGCTGNNN TTATTTNNCC 180
GNCN                                                              184
```

SEQ ID NO:7623
SEQUENCE LENGTH:212
SEQUENCE TYPE:nucleic acid
TOPOLOGY:linear
CLONE:HUMGS08870
SEQUENCE DESCRIPTION:

```
GATCCTAAGT CTCTTACAAA AGCTTTGAAT ACTGTGAAAA TNTTTTACAT TCCATTTCAT  60
TTGTGTTGTT TTTTTAACTG CATTTTACCA GATGTTTTGN TGNTATCGCT TATGTTAATA 120
GTAATTCCCG TACGTGTTCA TTTTATTTTC ATGCTTTTTC AGCCATGTAT CAATATTCAC 180
TTGACTAAAA TCACTCAATT AATCAATGNA AA                               212
```

SEQ ID NO:7624
SEQUENCE LENGTH:373
SEQUENCE TYPE:nucleic acid
TOPOLOGY:linear
CLONE:HUMGS08871
SEQUENCE DESCRIPTION:

```
GATCGCAGAC CGCGCTGCCC ACGACGAGTC CCCGGGGAAC AACGTGGTTG GACATCAGCG  60
TGCCGCCCAC CACGGACGGA CAGAAGTCTC TTTAAGAAAA TAGTTTAAAC AATTNGNTAA 120
AAAATTTTCC GTCTTATTTC ATTTCTGTAA CAGTTGATAT CTGGCTGTCC TTTTTATAAT 180
GCAGAGTGAG AACTTTCCCT ACCGTGTTTG ATAAATGTNG TCCAGGTTCT ATTGCCAAGA 240
ATGTGTTGTC CAAAATGCCG TTTAGTTTTT AAAGATGGAA CTCCACCCTT TTGCTTGGTT 300
```

TTANGTATGT ATGGAATGTT TATGGATAGG GACATAGTAG TAGCGGTGGT CAGACCATGG 360
GAANTGGGTG GNN 373

SEQ ID NO:7625
SEQUENCE LENGTH:74
SEQUENCE TYPE:nucleic acid
TOPOLOGY:linear
CLONE:HUMGS08872
SEQUENCE DESCRIPTION:
GATCCTACAA GCATGTCTGC TGTAACTCTA TGTATGTTTT CTGTAAAATC ACATACCTAT 60
AAAATAAAAA GAAA 74

SEQ ID NO:7626
SEQUENCE LENGTH:53
SEQUENCE TYPE:nucleic acid
TOPOLOGY:linear
CLONE:HUMGS08873
SEQUENCE DESCRIPTION:
GATCGTGAAC ACTGCATTCC AGCCTGGGTG ACAGAGCCNG ACCCTGCCTC AAA 53

SEQ ID NO:7627
SEQUENCE LENGTH:379
SEQUENCE TYPE:nucleic acid
TOPOLOGY:linear
CLONE:HUMGS08874
SEQUENCE DESCRIPTION:
GATCTACCCA CCTTGGCCTC CCAAAGTGCT GGGATTACAG GCGTGAGCAC CGCGCCTGGC 60
CCTCTGCAGT TGTTTAATAA GGCACAGAAT ACCTGTAGCA TAGGTCAGCC TTACGATGTC 120
CATGAATTAC ATATTCAGAC GTTTTAGAGC CTGATACATT TTGGAANCGA AAAACAACTT 180
CTACACCTAT TCTACAGTCC GCATTTAAAA CAATAAATTC CTCTATTAAA AACGTAAAGC 240
CGGGTTTGCT TGCGTGCCAC AGGGGGTATA TCCAGGAAGG TTATTATGAA GCTGTCAAAT 300
CAAGATGATG GNAATAAGGC AGTTTGAACG GACAGTCTTC CCACAGTCAG GCCATTTTTG 360
CTGATTTGGT TTAGGATTN 379

SEQ ID NO:7628
SEQUENCE LENGTH:212
SEQUENCE TYPE:nucleic acid
TOPOLOGY:linear
CLONE:HUMGS08875
SEQUENCE DESCRIPTION:
GATCGGGCGC TGTGCAGTGT GGTCAGCATG GTGAAGAAAG TCATTTCCTC GGTGGGCAGT 60
ATTCCTCTTT ATCTCTCATT ACACTGGAAA TGTTATTTCT GCTGTATCAT CCGTGCTCAA 120
CGTTTTAGTC TGTCAGGCTC ACCTTCTCTC TGGAAAGAAT TTGCTTAACT TGACATTCCA 180
TGTGCCGCTA ATAAAATATA TTTTGAAAGA AA 212

SEQ ID NO:7629
SEQUENCE LENGTH:350
SEQUENCE TYPE:nucleic acid
TOPOLOGY:linear
CLONE:HUMGS08876
SEQUENCE DESCRIPTION:
GATCAGAANT AATTACCATT CCCNGTGTTT TGCTGTAGGA CCTTCAGGTC TATGTTCTAA 60
GTGGGGNTTA TGAAACTGGT AATAGAAAAT AATGGAGAGC AGTTAAAGAG TTAATATACA 120
TTATTAATAT GNGTGCTCTC TACAGGCACA CAAAGCCTGT AGACTCAACA AATTTACTCC 180
TGAAGTTTTC CAAATTTGCA AAATCTGCAA TTTNTGTGCC ACATAAAGCT NTCCAGAAAT 240
TGAAAAANCNG TAAANCACCG GGTCTCANTT TNTTCCTGCA TTTCACTGAT ACCTTAANTT 300
ACATAGNTTA NCTTCTTTAN AGCACTACCT TTTTNGCTGT TGNTATTAAN 350

SEQ ID NO:7630
SEQUENCE LENGTH:76
SEQUENCE TYPE:nucleic acid
TOPOLOGY:linear
CLONE:HUMGS08877
SEQUENCE DESCRIPTION:
GATCTCACAA CTGCACTCCA GCCTGGTGAC AGAGCAAGAC TCCATCAAAA ATAAACAAAT 60
AAAACAAACA AACAAA 76

SEQ ID NO:7631
SEQUENCE LENGTH:185
SEQUENCE TYPE:nucleic acid
TOPOLOGY:linear
CLONE:HUMGS08878
SEQUENCE DESCRIPTION:
GATCAAAGTC TGCTTCATTG TCTTGTTCCC CATTTTAATG GCAATGTTAG GTATAATNTT 60
TGCTTTTATT AAGTATAATN TTTGCTTTTG TTGGTAGAGT TTTACGATTT TACATCAGAA 120
GTACCTGTTC AACTTAATTG CCACGTTTAA GCATCAATTA AAATATTTTC ATTGAACTCA 180
TGAAA 185

SEQ ID NO:7632
SEQUENCE LENGTH:149
SEQUENCE TYPE:nucleic acid
TOPOLOGY:linear
CLONE:HUMGS08879
SEQUENCE DESCRIPTION:
GATCTTTTAG GAATTAAACT TGCAAATNAA ATGAAATATG TTGGGACTTT GGCATAATTT 60
GAATATAGCA TTGTGTGAGT TGTGATTGTN TCTTCCTAAT TGTGCTGTAA TAACCTGTGT 120
ATGAGAATAA AATGATTTCC ACCATCAAA 149

SEQ ID NO:7633
SEQUENCE LENGTH:193

SEQUENCE TYPE:nucleic acid
TOPOLOGY:linear
CLONE:HUMGS08880
SEQUENCE DESCRIPTION:
GATCTCACTC CATGATTACT GTGTAAAATA TTTTTGCACT GTTGTGAAGT ATTTTTAACT 60
TTTTTCTGTA CATAACTGTG TTCTCAGAGC TGAATGTTTA TATCTTTTGC TGTGCAAAAG 120
AAACANNNNA AATGTTGTTC AGTTGTATAT ACAGAAATGT GTATAAAACA TTTTGTTATT 180
TTTNAAAAGT AAA 193

SEQ ID NO:7634
SEQUENCE LENGTH:148
SEQUENCE TYPE:nucleic acid
TOPOLOGY:linear
CLONE:HUMGS08881
SEQUENCE DESCRIPTION:
GATCAAGAAT TTTGTAGAGT GGACAGTCAT TACATATGTT ATAACTTATC CTTTAAAAAC 60
TATTTTAAAC TTTATCCTTT CAGCTTTACT TAGTGCGATG TTTTAGAAGC AGTCTTCAAA 120
GAATAAAACA CTAACCATGC ATGTGAAA 148

SEQ ID NO:7635
SEQUENCE LENGTH:376
SEQUENCE TYPE:nucleic acid
TOPOLOGY:linear
CLONE:HUMGS08882
SEQUENCE DESCRIPTION:
GATCCATTTC TGTGTCCTNT AGCCTAGTAT GTCTGAACTT CCATTCTTGT TATATATTTA 60
AACTTTCCCT CTATATTATA GGNTTNGTGG CNNCCACGGT CAGGTGTAGA GGAAGCTGCC 120
CCTTGCAGAA CTGTACTGTA ATATTTTTCT TTTATAAATA TTTTCACAGG ACTGATTGTA 180
CACAGGGCTT GTAATAAAAT TTTAACACTG TGCTGTGAAA CAACTATGGG GAATCTCCAT 240
TGAAGGCTAC TTCATGGGCA CCTGAAAGTG GAGTGTTATA GCTATGACTT TCTATTTCTT 300
GTTTCCTAAG TAAATTAAAC CTAATTTTCA CCCTTTCATT CTGTTTCAGC CTCCTGTNTA 360
AGGAAGTACC GTTTGN 376

SEQ ID NO:7636
SEQUENCE LENGTH:279
SEQUENCE TYPE:nucleic acid
TOPOLOGY:linear
CLONE:HUMGS08883
SEQUENCE DESCRIPTION:
GATCCCATTC ATTGTCCCCT TTGGGGTATT TCCAATACTT GAATGGCAGA TTGGAGTTTT 60
TCAGAGTATG TGTTTCATCT GCTAGTCTTT CTCTCCTTCA TAGCTTTTCT TTTCCTGGAC 120
TTGCTCCTTT TGAGTTGCTT TTGCGTTTCT CATGCCTAGG CAAGTGTAAT AGAAATTATG 180
TAGCTCCTTA TGTTGGCAAA GGAGCTCTAT ATAGTTTCAC TTTGTATACA AGTTAGGNCC 240
AGCTGTTGTT ACATGTAATA TNNTGGTTCA GAACTTGAN 279

SEQ ID NO:7637
SEQUENCE LENGTH:373
SEQUENCE TYPE:nucleic acid
TOPOLOGY:linear
CLONE:HUMGS08884
SEQUENCE DESCRIPTION:
GATCTCTGAT TGCATCTATG TGGATTCTTT TTGGAGGTTA TGTTGCTAAA GAAAAAGACA 60
TAGTATACCC TGGAATTGCT GTATTTTNCC AGAATGCCTT CATCTTTTTT GGAGGGCTGG 120
TTTTTAAGTT TGGCCGCACT GAAGACTTAT GGCAGTGAAC ACATCTGATT TCCCACAGCA 180
CAACAGCCCT GCATGGGTTT GTTTGTTTTT TTACTGCTCA CTCCCAACCT TTTGTAATGC 240
CATTTNCTAA ACTTATTTCT GAGTGTAGTC TCAGCTTAAA GTTGTGTAAT ACTAAAATCA 300
CGAGAACACC TAAACANCAN CCAAAAATCT ATTGTGGTAT GCACTTGATT AACTTATAAN 360
ATGTTAGGTG GTN 373

SEQ ID NO:7638
SEQUENCE LENGTH:53
SEQUENCE TYPE:nucleic acid
TOPOLOGY:linear
CLONE:HUMGS08885
SEQUENCE DESCRIPTION:
GATCACACCA CTGGCACTCC AGCCTGGGCG ACAGAGTGAG ACTCCGTCTC AAA 53

SEQ ID NO:7639
SEQUENCE LENGTH:363
SEQUENCE TYPE:nucleic acid
TOPOLOGY:linear
CLONE:HUMGS08886
SEQUENCE DESCRIPTION:
GATCCGAACT GGACTCACAT CCTGTATGGT GGATGGACTG TATATTGAGG GTTCCATTCT 60
TCGCGCAGTT TAGACATCTC TGTTTTGATT CTTTGTTGTT GTTTTTATTT TAAAAGGCAC 120
AAACTCTAGA TATTAGTTGA ATGTTGAGGC TTTAACTTTT TCGGTGTCTT TCTACAACTG 180
TGTTCTGTGA CTCAATTGTG TCGTGTTAAT ATCAGTACAG ACTGTCTCCT CTACGTGACC 240
GTATAATGTT TTTCTCTTCT TGTAGTCTCT ATGGCGTGTC TTTATGGTGT AATAAGGTTC 300
TCACGGGTTC AATCTTTTGT GTTTAGGGNN NNCACGGTTC AGACCAAATG GTATATAATT 360
TTN 363

SEQ ID NO:7640
SEQUENCE LENGTH:270
SEQUENCE TYPE:nucleic acid
TOPOLOGY:linear
CLONE:HUMGS08887
SEQUENCE DESCRIPTION:
GATCTGGTTC TGAGGAGGAC ACACCTGGCA TCGGATGACC TTTATAAATT ATCTATGAAA 60
ATGCCAAAAG CTCTCAAGCC AAAGAAGAAG AAAAATATTT CCCATGATAC TTTTGGTACA 120
ACTTATGGAA GGATTCATAT GCAGAAGCAA GACCTAAGCA AACTACAAAC CAGGAAAATG 180

AAGGGGTTGA AGAAGCGACC TGCAGAAAGG ATAACAGANG ACCACGNGGA AAAAGTCAAA 240
AAGAATTAAA AAAAATTGAT GGAACTTAAA 270

SEQ ID NO:7641
SEQUENCE LENGTH:336
SEQUENCE TYPE:nucleic acid
TOPOLOGY:linear
CLONE:HUMGS08888
SEQUENCE DESCRIPTION:
GATCTCTGAA AAGACGTTAT CACCTTAAAG CTCAAATTCT TTGGGATGGT TTTAACTTAA 60
GTCCATTAAC AATTCAGGTT TCTAACGAGA CCCATCCTAA AATTCTGTTT CTAGATTTTA 120
AATGTCAAGT TCCCAAGTTC CCCCTGCTGG TTCTAATATT AACAGAACTG CAGTCTTCTG 180
CTAGCCAATA GCATTTACCT GATGGCAGCT AGTTATGCAA GCTTCAGGAG AATTTGAACA 240
ATAACAAGAA TAGGGTAAGC TGGGNTAGAA AGGCCACCTC TTCACTCTCT ATAGANTATA 300
GTAACCTTTA TGAAACGGGG CCNTNTAGTT TGGGTN 336

SEQ ID NO:7642
SEQUENCE LENGTH:331
SEQUENCE TYPE:nucleic acid
TOPOLOGY:linear
CLONE:HUMGS08889
SEQUENCE DESCRIPTION:
GATCCATTTC AGATACTTGT GGCAGCAAAC AAAGCAGTTC ACCTCTACAA ACTGGGAAAA 60
ATGAAGACAA GAACTCTATC TACTGAAATN ATTTTCANCC TTTCCCCAAA TAACAATATT 120
TCAGAGGCTT TGAAAAAATT TGGTATCTCA GCAAATGACA CTTCAATTCT AATTGTTTAC 180
ATTGAAGAGG GAGAAAAACA AATAANTCAA GANTACCTAA TATCTCAAGT AGAAGGTCAT 240
CAGGTTTCTC TGAAAANTCT TCCTGAAATA ATGATTATTA CAGANGTCAA ANAGGTATAT 300
AANCTCTCTT CACAGGNGGN ANGTATCGGA N 331

SEQ ID NO:7643
SEQUENCE LENGTH:327
SEQUENCE TYPE:nucleic acid
TOPOLOGY:linear
CLONE:HUMGS08890
SEQUENCE DESCRIPTION:
GATCCACTGT CCGGCAGAGG AAAAATGCCA GCTGTGGGAC AAGGAGTGCA GGCCGCACAA 60
CCTCGGCAGG CACCGGGGGG ATGTGGCGAT TCTACACAGA AGATTCACCT GGGCTCAAAG 120
TTGGCCCTGT TCCAGTATTG GTTATGAGTC TTCTGTTCAT CGCTTCTGTA TTTATGTTGC 180
ACATTTGGGG CAAGTACACT CGTTCGTAGA TTCAGTTACA TCCATCTGTC ATCTGAAGAA 240
GGAGGAAAAA ACCCAACATT TCTTGGACCA AAAGTATAGT GACTATCTGT TCATGAGAGA 300
AATTTTCTGT NAGCTTGCTG TTTTGAN 327

SEQ ID NO:7644
SEQUENCE LENGTH:287
SEQUENCE TYPE:nucleic acid

TOPOLOGY:linear
CLONE:HUMGS08891
SEQUENCE DESCRIPTION:
GATCAAGATG AAGTTCAGCT AGAAGTCATA CCACCCTCAG GAATCAGCTA AGTAATTATN 60
ACTTGATTCT TTTAGCAAAT CAATGCACGT NATCCTACTT AATCCTTAAA TAAGTTTAGA 120
TTTAACTAAC CCAAAGTCCA GGAGGATGTT CTTACAAAAN TAGCTATATC ANGGGCTGGC 180
ACCTAGACAT TAANCTGTAA TTNGANAATA AGCAACATGT TGCATAACTN GTTGGAATAA 240
TTCCTTGTNC TGTTTAACAC TTGTCATAAN TTAGCAGNGT AANNNTN 287

SEQ ID NO:7645
SEQUENCE LENGTH:327
SEQUENCE TYPE:nucleic acid
TOPOLOGY:linear
CLONE:HUMGS08892
SEQUENCE DESCRIPTION:
GATCTACCCA ATTAGAAATG CAACTTGAGA AACCCAAACC TGTAAAACCA GTGACGTTTT 60
CCACAGGCAT CAAAATGGGT CAACATATTT NACTGGCACC TATTCACAAG CTTGAAGAAG 120
CTCTGTATGA ATACCAGCCA CTGCAGATAG AGACATATGG ACCACATGTN CCTGAGCTTG 180
AGATGCTAGG AAGACTTGGG TATTTAANCC ATGTCAGAGC TGCCTNACCA CAGGACCTTG 240
CTGGAGGCTA TACTTCTNCT CTTGCTTGTC ACAGNGCACT ACAGGATGCA TTCNGTGGGC 300
TTTTCTGGCA GCCCAGTTAA CCATTTN 327

SEQ ID NO:7646
SEQUENCE LENGTH:329
SEQUENCE TYPE:nucleic acid
TOPOLOGY:linear
CLONE:HUMGS08893
SEQUENCE DESCRIPTION:
GATCTGGCTG CTCATGAAGA CAAAGTTCTG AGTGTAGACT GGACAGACAC AGGGCTACTT 60
CTGAGTGGAG GAGCAGACAA TAAATTGTAT TCCTACAGAT ATTCACCTAC CACTTCCCAT 120
GTTGGGGCAT GAAAGTNAAC AATAATTTGA CTATAGAGAT TATTTCTGTA AATGAAATTG 180
GTAGAGAACC ATGAAATTAC ATAGATGCAG ATGCAGAAAG CAGCCTTTTG AAGTTTATAT 240
AATGTTTTCA CCCTTCATAA CAGCTAACGT ATCACTGGGG CTTATTTTGT ATTTTATAAT 300
AAGNTAGGGT TGTGTTTTNT NAAATNCGN 329

SEQ ID NO:7647
SEQUENCE LENGTH:93
SEQUENCE TYPE:nucleic acid
TOPOLOGY:linear
CLONE:HUMGS08894
SEQUENCE DESCRIPTION:
GATCTTCAAG TGAACATCTC TTGCCATCAC CTAGCTGCCT GCACCTGCCC TTCAGGGAGA 60
TGGGGGTCAT TAAAGGAAAT CTGGGACAAC AAA 93

SEQ ID NO:7648

SEQUENCE LENGTH:283
SEQUENCE TYPE:nucleic acid
TOPOLOGY:linear
CLONE:HUMGS08895
SEQUENCE DESCRIPTION:
GATCTGCATG CATTGCTTGC ATTTTNNTGG TATCTGAATG TTGGTTCCTT GTTCCAGGAA 60
TTCAACATTA NTTTCCAAAA GTATCATGGG ACTTGTGACA ATACANGTCA TGAATCTATG 120
TATAAAATTT ATCGGCCTTT CTCATTTACC TGCTCTAGTA TTATTGTATT GTGTGTGCGT 180
GCGTGTGTGA TGTCAGGCTG CCACGTAAAA CTTCAGAGGA AAATCTTAAA NGCAGGCCAT 240
CCTTTTTNGC ATGCTCTATT CTAAGTAGGA ATGTTCAATG TAN 283

SEQ ID NO:7649
SEQUENCE LENGTH:276
SEQUENCE TYPE:nucleic acid
TOPOLOGY:linear
CLONE:HUMGS08896
SEQUENCE DESCRIPTION:
GATCACCTGT AGGTCAATAG TTCGAGACCA GCCTGGCCAA CAAGGTGAAN CCCATCTNTA 60
CTAAAAATAC AAAAAATTAG CCAGGTGTGG TGGTGTGCAC CTGTAGTCCC AGCTATTTGG 120
GAGGCTGAGG TGGGAGAATC ACTTGAACCT GGGAGGCGGA GGTTGCAGTG AGCTGAAATC 180
GCGTACCACA CTCCAGCCTG GGTGACAGAG CGAGACTGTG TTTCAAAGAA AAAAANAACC 240
AGGCAGCCTT TTGCTTGGTT GGAATCTGAT TTTCTN 276

SEQ ID NO:7650
SEQUENCE LENGTH:182
SEQUENCE TYPE:nucleic acid
TOPOLOGY:linear
CLONE:HUMGS08897
SEQUENCE DESCRIPTION:
GATCTAAATA TTCAGGTTTT AAGCCTGCTG CAAACTTTTA AAATATTATG ATAGNTTCTG 60
TACTACATGT GGGAAACAAG CAAGAACTAA ATAATCAAAT GTTGTCAACC AAAAGTAATA 120
GTTGGGTATT GGAGATTTTT TTAAAATGTT TTTATGTTAT TNGCTATTTG GAGTTAAATA 180
AA 182

SEQ ID NO:7651
SEQUENCE LENGTH:93
SEQUENCE TYPE:nucleic acid
TOPOLOGY:linear
CLONE:HUMGS08898
SEQUENCE DESCRIPTION:
GATCTNAGAA AACGTTTAAT ATCAGATTTN TTTATTTTTN CCAACACAAC TGAATCAGTT 60
TTCTTAAATA AAAGTTTATT GTCTACTGTT AAA 93

SEQ ID NO:7652
SEQUENCE LENGTH:275

SEQUENCE TYPE:nucleic acid
TOPOLOGY:linear
CLONE:HUMGS08899
SEQUENCE DESCRIPTION:
GATCTAAAGT TAGTTGCCTT TGCCTGTAAA ACATGTGATT TGCAAATTAT TATTTTNNTT 60
TTTTTTAACA AATGGAAGTA AATTTGTTTC ACGTAAATCT TAATTTTCAA CCTTTCTGGA 120
TACCTTAATT GTAACTGTCA GTTTGCACTG GTCGGTATAT GGAAACACAT TGCTCTACCC 180
TGCTACTTAG TTGNTTTTAA AGTGAATTTN CAGTGATGNG AAATTTGTGA AANNTATATT 240
GTATTTCTTT TGATGTTTCA AAAGGTTGCC TATGN 275

SEQ ID NO:7653
SEQUENCE LENGTH:279
SEQUENCE TYPE:nucleic acid
TOPOLOGY:linear
CLONE:HUMGS08900
SEQUENCE DESCRIPTION:
GATCTTTAGG TGTTGAACTG GGAATATGCT CTGCAACACC CAGGATTAGG ATAGAGATGG 60
ATTTCATTTT ATATGGGCTA GCATATTATT ATTAAAGACC ATTTCAGGAG TGGGACTGTG 120
AAAGGAGGTG CTCTGTTATC TGGTCCAAGA GCTCCTCTGG GAATTAATCC TGGCTGGTTC 180
TGATGAGCTG GCAAGGNGCA GNGGATTGCA GTCTTTAAGT AGAAAATTNG AACCCCATCC 240
ATATAACACG TGNACGTGCA TTAAATAGCT TGAATTGTN 279

SEQ ID NO:7654
SEQUENCE LENGTH:264
SEQUENCE TYPE:nucleic acid
TOPOLOGY:linear
CLONE:HUMGS08901
SEQUENCE DESCRIPTION:
GATCAAGACC AGAAAGAAAT TATTGATACC AATGGAGCTG GAGATGCATT TNTTGGAGGT 60
TTTCTGTCTC AACTGGTCTC TGACAAGCCT CTGNCTGAAT GTNTCCGTGC TGGCCACTAT 120
GCAGCAAGCA TCATAATTAG ACGGACTGGC TGCACCTTTC CTGAGAAGCC AGACTTCCAC 180
TGATGGAAGA GCTGAAAACA CAAGCCCAGG AGTGCAGACA CTGCCCTAAT TGCTTCCTGA 240
GAATTCCCAT ATTAATAAAG ANGN 264

SEQ ID NO:7655
SEQUENCE LENGTH:61
SEQUENCE TYPE:nucleic acid
TOPOLOGY:linear
CLONE:HUMGS08902
SEQUENCE DESCRIPTION:
GATCTTTTAG GAAAAAAAAT AAATATTTTT AACATTAAAG CAGTCACTTT GGTTTATCAA 60
A 61

SEQ ID NO:7656
SEQUENCE LENGTH:66

SEQUENCE TYPE:nucleic acid
TOPOLOGY:linear
CLONE:HUMGS08903
SEQUENCE DESCRIPTION:
GATCTGTTTG CTTAGCTGTC AACAAAAAGA AAACCTGAAG GAGCATTTGG AAGTCAATTT 60
GAGGTN 66

SEQ ID NO:7657
SEQUENCE LENGTH:252
SEQUENCE TYPE:nucleic acid
TOPOLOGY:linear
CLONE:HUMGS08904
SEQUENCE DESCRIPTION:
GATCTNACTA AGCTTTCTTA GGAAGGGAAA GCATCTGGCA AGGAGTAAAA TGTCTGTTTC 60
CAACAATTGT CCCTGTTCCC ACTACTTTTT AAAAATGTCC TTAGTAAAAA GTGTATATGA 120
GGCAAGATTA TATTGAAAAA NTGAATATAA GAAAAAAATA TTATGTTAGA GAGNAACGTA 180
TTCAGCATTG TTNTCAAAAT CACAAACTGT TAGGNGACTC TGACATCAGG TTTGTTCTCC 240
ATATGCAGCA AN 252

SEQ ID NO:7658
SEQUENCE LENGTH:153
SEQUENCE TYPE:nucleic acid
TOPOLOGY:linear
CLONE:HUMGS08905
SEQUENCE DESCRIPTION:
GATCTACATG GATGTTCTGT TCTCTGAACT GTCTGGATGA ACCGGTCAAC GGCACTCATC 60
ATACCTTAGT TTTTAAATCT GCATTGTGGT CATAATCTGT TATTTAATTA ATTTCTCGTA 120
TTTTTAATAA AAACTTTGCC TATATATTTT AAA 153

SEQ ID NO:7659
SEQUENCE LENGTH:23
SEQUENCE TYPE:nucleic acid
TOPOLOGY:linear
CLONE:HUMGS08906
SEQUENCE DESCRIPTION:
GATCCCAAAA GTAGCAAGAG AAA 23

SEQ ID NO:7660
SEQUENCE LENGTH:280
SEQUENCE TYPE:nucleic acid
TOPOLOGY:linear
CLONE:HUMGS08908
SEQUENCE DESCRIPTION:
GATCAATTGC AGAATGATAC AGATAGTACA ATGACTTTTT TGTAAATTTG AATTAAAAGT 60
TTCCTCATAA AACTCAGTAC TATCTATTGG TAATGGATGC ATATATGTAT GTACATGTTT 120

TTNAAACGGA TTGGAAGGAT ACAGACCAAA CTCTTGATAG TGGTCACCTG TGAAGAGTGG 180
AGAAGGGAAA ATNATGAGTG TGGGAGGTGG GATGGGTATT GGTTAANGGG GACTTCAGCT 240
TTTTATATAA ACATCCACTT CTCTTTCAAA AAGNCTTCAN 280

SEQ ID NO:7661
SEQUENCE LENGTH:137
SEQUENCE TYPE:nucleic acid
TOPOLOGY:linear
CLONE:HUMGS08909
SEQUENCE DESCRIPTION:
GATCATTTTA AAAAACTGAA TCTATATNAT NTTTAACTTC AGAAGGCATC ATTTATAAGA 60
CAGTATGGCA GTTAATTATA AAATNATTTT GATGAATTAT GATACAATCT ACATAATAAA 120
GAATNCTTTT GATTAAA 137

SEQ ID NO:7662
SEQUENCE LENGTH:305
SEQUENCE TYPE:nucleic acid
TOPOLOGY:linear
CLONE:HUMGS08910
SEQUENCE DESCRIPTION:
GATCCTAAAA CTGTCTCTCA CATTATATAG TAGATGTTTG TTTATAATGT TTACAAAACA 60
TTTTGGTGAA TTTCCTCAAT GTTTTATAAA TGTACATTTT TTAAGTCCTT AAGCTGACTC 120
TTAGCCATCA TGTAGCTTAA GGAGTCTGAA ATCTGCCATT AAAACTGCAC CTTTAAGCCA 180
GGTGTGGTAG CATGTGCCTA TAGTCCCAGC TACTTGGGAG GTGGAGGTGG GAGGATTATA 240
AATAGAGACT TTCCTTAAGA CTTTAAAAAT GTATTGNNAA ACTATTTTTT ATTAAATACT 300
TTGTN 305

SEQ ID NO:7663
SEQUENCE LENGTH:103
SEQUENCE TYPE:nucleic acid
TOPOLOGY:linear
CLONE:HUMGS08912
SEQUENCE DESCRIPTION:
GATCCTAACC TTGAAGTATG CCTTGAACTT ATTAACATGG CCATTATAAG AATAAAATAT 60
GTAGTTGTGT CTTAATGGAA TTAATAAATG TCATTTCACT AAA 103

SEQ ID NO:7664
SEQUENCE LENGTH:211
SEQUENCE TYPE:nucleic acid
TOPOLOGY:linear
CLONE:HUMGS08913
SEQUENCE DESCRIPTION:
GATCAAGGAA TAAAGAATCA TTAAAGAATG GGGTGTTGGG GGTTCTCTTA ATTATCAGTG 60
ACACATTAAT TNTTTTCCTT AGAGCTTCAT ACAATAAACA CATTGCAGAA AGTTTTAGAA 120
AGTTTTGCAG GACCTTAAAT TTTCTGCCCT GACACTTTTC GTCATGTTTC CTGTATTCCN 180

GGTTATTGTA GCGCATGCTT TTCAGGTAGT N 211

SEQ ID NO:7665
SEQUENCE LENGTH:71
SEQUENCE TYPE:nucleic acid
TOPOLOGY:linear
CLONE:HUMGS08914
SEQUENCE DESCRIPTION:
GATCTTAAAT TTTGTGATAT GGAGCCCTGT AATNTTTTTC TTATATAAAA ATGGGTATCT 60
ATATTCATAA A 71

SEQ ID NO:7666
SEQUENCE LENGTH:344
SEQUENCE TYPE:nucleic acid
TOPOLOGY:linear
CLONE:HUMGS08915
SEQUENCE DESCRIPTION:
GATCCCAGCT CATGGCACAG CCGTTTGTAT CCTCGGAAGA ACGGAAGGAA CGATGGGAAC 60
AGGGCCAGGC TGATTATATG GGAGCAGATT CCTTTGACAA CATCAAGAGG AAACTTGACA 120
CTTACCTCCA GTAGAAACAC TGCATTTTTC TGTGAACACA TCCACTTCAC AAGCCTTGTT 180
TCTGATACTT AGTATCTAGA GCTGGGTTGA GAAAAGTCTG TTACAGTTGC TAGAGGTTTT 240
CATTAAANCT TATCAGGNGG GCGGCTTTTT TAGGATAAGA GGTGAGAACT GGGCAAAAGT 300
TGTGAAGCAG CAATTCTGTT ATATGGNCAG TGTTCTGCTT TTTN 344

SEQ ID NO:7667
SEQUENCE LENGTH:200
SEQUENCE TYPE:nucleic acid
TOPOLOGY:linear
CLONE:HUMGS08916
SEQUENCE DESCRIPTION:
GATCACGACT GCTGCACTCC AGCCTGGGTG ACAGNGCGAG ACTCCATCTA AAAAAACAAA 60
AAGCAAAAAA ACCCACAAAT ACCTCATGGA GATGAACTGT AATAATTGCT TAAAGTTCCA 120
TTTAATTATG TTAACTCTAA TCTAGCAAAA ACATAGATGT ACTTAAAAAT AAATCATGGA 180
TAATGATTTT TTAACCTAAA 200

SEQ ID NO:7668
SEQUENCE LENGTH:277
SEQUENCE TYPE:nucleic acid
TOPOLOGY:linear
CLONE:HUMGS08917
SEQUENCE DESCRIPTION:
GATCTCATTT TCAAGTNTGA GTCTGACCAA CCATGGAAAA TATTCGACAT GAATTAATGT 60
AGAGAACTAT AAAGCATTTA TGACAGCTCC AAGAAAAGTC ATCTACTCTA TGCAGGAGAT 120
ATGTTTAGAG ACCTCTCAGA AAAACTTGCC TGGTTTGAGG GTACACAGTA CCATTTTAAT 180
CTTCTGAAAA TATCTGTATT CCTGCTCTGG GGNTGCTGTC ACTGTCAATC TGCTATATTT 240

TTCACTATCC TATTAAAATA TTACTGTCTC CTTTAAA 277

SEQ ID NO:7669
SEQUENCE LENGTH:163
SEQUENCE TYPE:nucleic acid
TOPOLOGY:linear
CLONE:HUMGS08919
SEQUENCE DESCRIPTION:
GATCTTCTAC ATTTATATTT TTAATCTTCT GTTAAATACA CTTTCCGATA TTGCCTTGCC 60
TTTTGAGCTC TTGCTACAGT CGCCTTTGCT ACTGCTTTAA GAGAATTTAC AGGTATTGAT 120
AAAGAACAAG ACTGTTTTAT TAAAAGNTTT ATTCAACTTG AAA 163

SEQ ID NO:7670
SEQUENCE LENGTH:111
SEQUENCE TYPE:nucleic acid
TOPOLOGY:linear
CLONE:HUMGS08920
SEQUENCE DESCRIPTION:
GATCCAAATC TGGTTCAAAC ATTCAAAACT TCAAAGATAA TTCATCTTTC GCTAATGCTT 60
GTGGTTCTGT NGTTCCCTTG AAAAAAAATA AAAACCCGAC CCAGCAGCAA A 111

SEQ ID NO:7671
SEQUENCE LENGTH:155
SEQUENCE TYPE:nucleic acid
TOPOLOGY:linear
CLONE:HUMGS08921
SEQUENCE DESCRIPTION:
GATCTGTCAC TGTTCCCCGT CACCCTCAGA TGGGACCGTC TAGTTGTAGG AAAACAAGCT 60
CAGGGCTCCC ACTGATTCTA CATTTTGGTG AGTTGTATAA TTGTTATGTA TTGCAATGTA 120
ATAATAATAG AAATAAAGTG TACGATAAAT GCAAA 155

SEQ ID NO:7672
SEQUENCE LENGTH:239
SEQUENCE TYPE:nucleic acid
TOPOLOGY:linear
CLONE:HUMGS08922
SEQUENCE DESCRIPTION:
GATCTGGAGA GGTTGTTTGT GAAAGTGGAG AAGATTCACG AAAAGGTCTA AATGAATTGC 60
GTGTGCAGGG CGCGGATTTA AAGTCCAATT TCTCATGACC AAAAAATGTG TGGTTTTTTC 120
CCATGTGTCC CCTACCCCCC AATTTCTTGT CCCCTCTTAA AGAGCAGTTG TCACCACCTG 180
AACACCAAGG CATTGTATTT TCATGCCCAG TTAACTTATT TACAATATTT AAGTTCAAA 239

SEQ ID NO:7673
SEQUENCE LENGTH:327
SEQUENCE TYPE:nucleic acid

TOPOLOGY:linear
CLONE:HUMGS08923
SEQUENCE DESCRIPTION:
GATCCTTTCA TTAATCAGAA CAGCAGCTTA TNAACTGCTT CATTTTCCAA AAGTGTTTCC 60
CAAAAATCAG AAACCTAAAC ACCCAACAAC AGGAATTATT GCCATCACAT TGGCGTTTTA 120
CATATGTCAC GAAGTTCACC TAGCTGGTTT TAAATACAAC TTTTCTGANC TCAAGAGTCC 180
TTTGCACTAC TATGGGAATG CCACCATGTC TTTGATGANT AAGAACGCGT ATCACAATGT 240
GACTGCAGAG CAGCTCTTTT TGAAGGACAT TATAGAAAAA ANACCTCGTA ATCAACTTGA 300
CTCANGATTG ACTCTACAGA CTCGGAN 327

SEQ ID NO:7674
SEQUENCE LENGTH:377
SEQUENCE TYPE:nucleic acid
TOPOLOGY:linear
CLONE:HUMGS08924
SEQUENCE DESCRIPTION:
GATCTCCCNT AATTGGGAAT GAANGATTTC ACAGACTAGA GTCTCCGATG CTGGTCATGA 60
TGTCAAAACT AAGTTCTGAC TCATTTAGGG AACTGGATAC TTGGGTCTCC AGAAGGGCCA 120
ATGGGAGGGC CATAATTCTG TTTATTTTCA AATTGTCTTG TTTTCACCTT GTTAGAATGA 180
NCTNTGGAAG CCCAGCCAGG GACAATGCAC CTTCACAGAG ATTCTGCACT AATCTGAGTG 240
AAGGTCTAAG GTTTGGAATC TCCCCCTCAT GGAGAGAAGC TTTGTATGGC TGTCATGCTT 300
AGACAGTGAT TCCTGCAACT TGACCTTCAG GCTGGGAGAG GTNGAGAGCC ATGCCTGTTT 360
CTCCTTNCTT TGCTATN 377

SEQ ID NO:7675
SEQUENCE LENGTH:350
SEQUENCE TYPE:nucleic acid
TOPOLOGY:linear
CLONE:HUMGS08925
SEQUENCE DESCRIPTION:
GATCTGCTTC CCGCAAGACT CACAGCTGTT GGCAGGAGAC CTCAGTTTGT TGCCACATGT 60
TCCCCTCCAG AGGGCCTCTC ACAACATGGC AGTTATTTGT CCCCAGAGCA AGCAACACCG 120
GAGGGCAAGG AAGAAGCCAT GATGTTTTTT GTAACCTAGC CTCTGAAAGT GTCATACCAA 180
TTCTGTATTT TGTTGGTCAC ACAGACCANG TCAACTACAA CGTGGGAGAC TCCTACACAA 240
GGCATGAATT CTAGGAGGTG GGCATTTTTA AGTGTCATCT GGAAGGAGGC TGTCACAACC 300
TGGAAGTTAA AAGCATTGAT ATTCTGAAAT ACAGCGTGTA TTACCATGGN 350

SEQ ID NO:7676
SEQUENCE LENGTH:335
SEQUENCE TYPE:nucleic acid
TOPOLOGY:linear
CLONE:HUMGS08926
SEQUENCE DESCRIPTION:
GATCTAGAGG AGGATTTGCA GGAAGAGCTC GTGGAAGAGG TGGTGGCCCC AGTCAAAACT 60
GGAACCAGGG ATATAGTAAC TATTGGAATC AAGGCTATGG CAACTATGGA TATAACAGCC 120

AAGGTTACGG TGGTTATGGA GGATATGACT ACACTGGTTA CAACAACTAC TATGGATATG 180
GTGATTATAG CAACCAGCAG AGTGGTTATG GGAAGGTATC CAGGCGAGGT GGTCATCAAA 240
ATAGCTACAA ACCATACTAA NTTATTCCAT TTGCAACTTA TCCCCAACAG GTGGTGAAGC 300
AGTATTTTCC AATTTGAAGA TTCATTTGAG GGNAN 335

SEQ ID NO:7677
SEQUENCE LENGTH:295
SEQUENCE TYPE:nucleic acid
TOPOLOGY:linear
CLONE:HUMGS08927
SEQUENCE DESCRIPTION:
GATCCAAATT TGAAGCTCCA AGGATAGGAA AAAGCTTACT TGTGCCTGTC CTAGACAAGT 60
ACTAGAGTTT ATATTGAAAT CAAAACTTCT AAAAATAAAA ATCAGTAAGA TTAATTTTAC 120
TCATTTTTCT AACACGAGTC AGTATGCTTG ANGTTTTCAT TGTATTTGAT GTCATGTAAA 180
TTTACTGTAA AAAATAAGAA AANNTTTTAA AATGCTTTAT TGATTAANTT TAGGGTTGCT 240
TTTCAGTATA CTCAGTAACT CCGAAGTCAG TGTTAACAGT ACTGAGCTTA CATAN 295

SEQ ID NO:7678
SEQUENCE LENGTH:136
SEQUENCE TYPE:nucleic acid
TOPOLOGY:linear
CLONE:HUMGS08928
SEQUENCE DESCRIPTION:
GATCTTTTGT TAACTTTTAA ATGGCCTGAG TCCATAGAAA CTGCATCCTG AGAGGTTGCT 60
CGGCTAGAAA GCGGAAAACC GCTTCACATG AGTGCTCTAC TTTGTAATGA AAATAAACAC 120
TATTAATACA GGCAAA 136

SEQ ID NO:7679
SEQUENCE LENGTH:310
SEQUENCE TYPE:nucleic acid
TOPOLOGY:linear
CLONE:HUMGS08929
SEQUENCE DESCRIPTION:
GATCTGGGAA CGGAAGATGA ATGAATGGAA GATGAATGGT TTCTCTCCCA GTTTTCTCTG 60
AGGCAAACAT AATTATACCT NCTCCTTTGT TCTGGGAGGT TTTTCTCTTC TCATCTCCCC 120
TCCATTCACC AAAACAGAGT TGGGTCTATA TTTNACCCGG CTGGCTCTTG GAACTGCAGG 180
GAATCCTGGC TGTTTTCATT TTTAAAAAAT TATGAGATTA NCTACCAAAC TTGAGATGTA 240
AGTTGTGGGT TAGCTCTCGG ATTCTGCTAG ATAACCTGGA ACAAAGCATT AACTTCTTTG 300
AGAGCTTCCN 310

SEQ ID NO:7680
SEQUENCE LENGTH:177
SEQUENCE TYPE:nucleic acid
TOPOLOGY:linear
CLONE:HUMGS08930

SEQUENCE DESCRIPTION:
GATCTGCCAT GTAGGAATGA GACAAATACA GTTTGCTTAT GAAAGGAAAG TGGCATACTT 60
TTAAATTGGT CTACACAGAA AAGTAAAAGT AAACTATTCA TTTAAATAAG ATTCATTATC 120
TAATAAATAT TGAGGGAATA TTTTTCCTAA ATAAAAATTT TTCTGACTGC TAACAAA 177

SEQ ID NO:7681
SEQUENCE LENGTH:84
SEQUENCE TYPE:nucleic acid
TOPOLOGY:linear
CLONE:HUMGS08931
SEQUENCE DESCRIPTION:
GATCTAAGCT TTCTTTAATA TAAAAAAAAT NATACTTCTC TGAACTGTTT AATAGAGCAA 60
TAATTAAATA CATCTTCTGA TAAA 84

SEQ ID NO:7682
SEQUENCE LENGTH:285
SEQUENCE TYPE:nucleic acid
TOPOLOGY:linear
CLONE:HUMGS08932
SEQUENCE DESCRIPTION:
GATCTGTTGG GAAAGATAAG AAAGCTATTC AGGCATCAAT TAGACGTAAT AAGGAAACCA 60
ACACCGTTTT GGCCAGATTG AATAGCGAAT TGCAGCAACA ATTAAAGGAT GTTCTTGAGG 120
AGAGAATTTC CCTGGAAGTT CAACTGGAAC AACTTCGACC ATTCTNTCAC CTATAAGCCA 180
ATTGCCGTTA ACTGTGAACA TACTTGTTTT TAAGTGGTTT TGGGTTCAAA GCCAATTTGG 240
AGACCTAGAC ATTCAGCTCA CTGCTTAACT CANCATTAAA TTTTN 285

SEQ ID NO:7683
SEQUENCE LENGTH:313
SEQUENCE TYPE:nucleic acid
TOPOLOGY:linear
CLONE:HUMGS08933
SEQUENCE DESCRIPTION:
GATCTCAATT CTGTTTCCTC TCACATGATT ACTTGATAGC TAAGCATCTG ATTGGTTTAC 60
TGCTTTACCA CTGAGCTGAA ATGCCGTGTT TTCCATTTAT TAAAATCACA CATGGCTCCT 120
GTTTTTNTCA CTCAGCACTT TTTCTCCATA TTCTTCAAGA CGATTGTGAG TATGGTACGT 180
AACAGGAATT ACATCTGGTA AGTTGTATAG TTTTGTGTAG GAACTCTATA TTCATAGCAT 240
ATTTGTGGAA ATGATACCTA TGGAGGTTTC TCACACTGGT GTGTCATTAT ACATTAATTG 300
TACAATATGC ATN 313

SEQ ID NO:7684
SEQUENCE LENGTH:110
SEQUENCE TYPE:nucleic acid
TOPOLOGY:linear
CLONE:HUMGS08934
SEQUENCE DESCRIPTION:

GATCAGTAAT GGCAAGAGCC TTTNATTCTC GAATGTTTAA AGCCTAGGAG TTCTACAAAA 60
TTGGNTTCTT TCTACAAGAN TCCCAAAATG GAATGCCTAA AGAGGTNTNN 110

SEQ ID NO:7685
SEQUENCE LENGTH:132
SEQUENCE TYPE:nucleic acid
TOPOLOGY:linear
CLONE:HUMGS08935
SEQUENCE DESCRIPTION:
GATCCCAAAA ACCGGGCAGC CCAGGAAGGA ACTGGGGAAG GTGGTCATTC AGGGGAAGAA 60
CCAGGATGCA GGGCTGGCTC AGGGTCTGCG CAAGATGTTT GGCTGATTAA AAGTTAAACC 120
TTAAAAGAGA AA 132

SEQ ID NO:7686
SEQUENCE LENGTH:85
SEQUENCE TYPE:nucleic acid
TOPOLOGY:linear
CLONE:HUMGS08936
SEQUENCE DESCRIPTION:
GATCCCATGC CTGAAATNTG GAAGCATATG TACAAAAATC ATTTTTACGT TTTATTTTTA 60
ATAAATCATT GTGTTTGACC GTAAA 85

SEQ ID NO:7687
SEQUENCE LENGTH:333
SEQUENCE TYPE:nucleic acid
TOPOLOGY:linear
CLONE:HUMGS08937
SEQUENCE DESCRIPTION:
GATCCTAGTC ATGACTATAC ATTGTGACAT GGTCATTACA TATGGATTAG ACCAACTGGA 60
GAATTGCCAG ACTTGTGGTA CCGATTATAT CATCTCAGTC TTGAATTTAC TCACGCTGAT 120
TGTTGAACAG ATAAATACGA AACTGCCATC ATCATTTGTA GAAAAACTGT TTATACCATC 180
ATCTAAACTA CTATTCTTGC GTTATCATAA AGAAAAAGAG GTTGTTGCTG TAGCCCATGC 240
TGTTTATCAA GCAATGCTCA GCTTGANGAA TATTCCTGTT TTGGAGACTG CCTATAAGTT 300
AATATTGGGN GANATGACTT GTGCCCTAAA CAN 333

SEQ ID NO:7688
SEQUENCE LENGTH:333
SEQUENCE TYPE:nucleic acid
TOPOLOGY:linear
CLONE:HUMGS08938
SEQUENCE DESCRIPTION:
GATCTGATTC GCTCCATCAA TGACCCGGAG CATCCACTGA CGCTAGAGGA GTTGAACGTA 60
GTAGAGCAGG TGCGGGTTCA GGTTAGCGAC CCCGAGAGTA CAGTGGCTGT GGCTTTCACA 120
CCAACCATTC CGCACTGCAG CATGGCCACC CTTATTGGTC TGTCCATCAA GGTCAAGCTT 180
CTGCGCTCCC TTCCTCAGCG TTTCAAGATG GACGTGCACA TTACTCCGGG GACCCATGCC 240

TCAGAGCATG CAGTGAACAA GCAACTTGCA GATAAGGAGC GGGTGGCAGC TGCCCTGGAG 300
AACACCCACC TCTTGGAGGT TGTGAATCAG NTN 333

SEQ ID NO:7689
SEQUENCE LENGTH:110
SEQUENCE TYPE:nucleic acid
TOPOLOGY:linear
CLONE:HUMGS08939
SEQUENCE DESCRIPTION:
GATCCATGAA CCCTTTTTAT AAGCTGTGTG TGTCCTCTGT ATTATTGTTA TTAACTATTT 60
TTTAGNATTT GCCTGTAAGT TATTAAAGAC TGATAACTGT AGCTCTTAAA 110

SEQ ID NO:7690
SEQUENCE LENGTH:186
SEQUENCE TYPE:nucleic acid
TOPOLOGY:linear
CLONE:HUMGS08940
SEQUENCE DESCRIPTION:
GATCCAGCCA AACGAATTTC TGGCAAAATG GCACTGAATC ATCCATATTT TAATGATTTG 60
GACAATCAGA TTAAGAAGAT GTAGCTTTCT GACAAAAAGT TTCCATATGT NATGTCAACA 120
GATAGTTGTN TTTTNNTTGT TAACTCTTGT CTATNTCTCT CTTATATATA TCCCNCTNTT 180
ATCAAA 186

SEQ ID NO:7691
SEQUENCE LENGTH:231
SEQUENCE TYPE:nucleic acid
TOPOLOGY:linear
CLONE:HUMGS08941
SEQUENCE DESCRIPTION:
GATCATGTGA CCAATTGTGC ATTCAGTTTT CAGAATTCTT TGCTATATGA TTTGGATTAA 60
TTCTATATAA TTTTGGACTT TTAAATATTA AGGTTAAAAA ATACCTGTAT CTAAAATTGA 120
TTCTGTTAAC TGTTGTCTTA AAACTAAAGG TATTAAAGTA TAANNTTAAA ATTNGCAATT 180
TTTTTAAAAN NNTTGCAATT TTGATTCTCA TGGGGGAAAT TGGAGATAAT N 231

SEQ ID NO:7692
SEQUENCE LENGTH:277
SEQUENCE TYPE:nucleic acid
TOPOLOGY:linear
CLONE:HUMGS08942
SEQUENCE DESCRIPTION:
GATCACAATT TAATAACAAG TAAAACTTTA CTTTTGACAA TTTTGCCACT TATTTTGTAA 60
TTTGTAAGCA CACGTTTGAA GATATATTTA TGCCATTAAT ATTTTAAGGC ATGTTTTAAA 120
ATTTACTGTT TTCTGAAAAA TTAAATGNNN CTGGTTTGAT TGCTGGAGAT TTATTCCAGT 180
TTTNCTCAAA TGTATTTTNA TNCTGTATGG TACATTTTNG NGGATTNCCT GATTAATGTC 240
ATCTTAGCAT TATATTCTGG TTATCTATAA TNNANNN 277

SEQ ID NO:7693
SEQUENCE LENGTH:312
SEQUENCE TYPE:nucleic acid
TOPOLOGY:linear
CLONE:HUMGS08943
SEQUENCE DESCRIPTION:
GATCTGGGCA AAGCAAATTG AAAACCTTCT GGAAAGGATT CACCATTTTA GATGCCATTA 60
AAAACATTCG TGATTCATGG GAGGAGGTCA AATTGTCAAC ATTAACAGGA GTTTGGAAGA 120
AGTTGATTCC CNNCCTCATT GATGACTATN AGGGGTTCAA GACTTCAGTG GAGGAAGTAA 180
GTGCAGATGT GGTGGAAATA GCAAAAGAAC TAGAATTAGA AGTAGAGCCT GAAGATGTAA 240
CTGANTTGCT GCAATCTCAT GNTAAANCTT TAACAGNTGA GGAGTTGTTT CTTATGGNTG 300
CGCAAAGAAA TN 312

SEQ ID NO:7694
SEQUENCE LENGTH:83
SEQUENCE TYPE:nucleic acid
TOPOLOGY:linear
CLONE:HUMGS08944
SEQUENCE DESCRIPTION:
GATCTTTACT GTTCAGAATT TAGGAAAGTT CTCTGGCTGT TGCATCCAAG TAAAATTAAA 60
ATAAAATTGG TTGCANNTTT AAA 83

SEQ ID NO:7695
SEQUENCE LENGTH:263
SEQUENCE TYPE:nucleic acid
TOPOLOGY:linear
CLONE:HUMGS08945
SEQUENCE DESCRIPTION:
GATCCCTTCC TCTGTCTCTC TAACTCCCCA GCCATGAATT TTGGCCCCAG GGAGAGGAAT 60
AGGCAGTATA GGAGGGATGT CAGGGAACTC ACATCTGTGG TTGAATCTCA GAAACAATGG 120
AAAGGCAGTT GTCTTTGGAT ATGGTGAAAT TGTGGTAGAG CACCCATGGT GGGGGTCTGG 180
CAGGAGCTCT GCGGCTTCGG TAGCAGCAGT ATGAGGAGAG CTGGGCCACA TGCTTATGGG 240
TTAGGAGAAG GTAATTTCCA GTN 263

SEQ ID NO:7696
SEQUENCE LENGTH:142
SEQUENCE TYPE:nucleic acid
TOPOLOGY:linear
CLONE:HUMGS08946
SEQUENCE DESCRIPTION:
GATCATGGCT GAAAAAGCAG CAAATGCAGC AGGAAAAAAG TTCCGAAAGA AGAAGAAATT 60
TCGCAATTAA GATTTACCAA GCAAACTGCA ACATTTTACA TTGCTCCTTT ATTTACTTAT 120
TAAAGACGTT TGGAAAACTA AA 142

SEQ ID NO:7697
SEQUENCE LENGTH:200
SEQUENCE TYPE:nucleic acid
TOPOLOGY:linear
CLONE:HUMGS08947
SEQUENCE DESCRIPTION:
GATCAAGATT GAGAAGACTA GATACATAAC AACCAACTGC AATGTTTGGT CCTTCATTGA 60
ATGGTTGAAC AAACCAGCTG TGAAAGACAT TTTGGGACAT ACAGACATGG ACTGAGAATT 120
AGACATAAGA AAATTATTTT GTTAACTGTT ATCATGGTTA ACAAAACGGT CATATAATNT 180
GATTATGTAG AGAAGTTAAA 200

SEQ ID NO:7698
SEQUENCE LENGTH:351
SEQUENCE TYPE:nucleic acid
TOPOLOGY:linear
CLONE:HUMGS08948
SEQUENCE DESCRIPTION:
GATCTGCCTG CCTCAGCCTC CCAAAGTGCT GGGNTTACAC GCGTGANCAC CACACCCAGC 60
CTCCATCTTT AAACTTTTAA ATGTGAAATT TCTATCATGT ACCGTTAGCC TAACAAGATT 120
TTNTTTCCTA TTTCTGACTG GTGCCTTTCC CCTTTTTAGG AGCAACGAAA GCTACTCTCT 180
TAGTTATGTT CTTGTGATGT GACAAAATGT CAAGAAGATA GGAGAAGAGA ATATTTTATT 240
TCGTTGATGC TTTTGTTCCC AAGTGTGACC CTAAACTTAA GCTTTGTAGG AGTTGACATT 300
CTTTCATGTC CCTTCCCTTT ACTCATGCCG AAACTATCAA CTGGGNCATG N 351

SEQ ID NO:7699
SEQUENCE LENGTH:350
SEQUENCE TYPE:nucleic acid
TOPOLOGY:linear
CLONE:HUMGS08949
SEQUENCE DESCRIPTION:
GATCTGTTTC TGGGGCCTCT GGCGGTGGCG GCCTGGGGCG GCGCGACGGC TGGTGCGCAG 60
GTACANTGAT GCTGAAGTAC TATGAGCCTT CGGAACTTGT GGAGAGACTA CAANGTTTTG 120
GNNNNTATGG TCCCTTTAGT TGGGCTCATA CATTTGGGGT GGTACAGAAT CAAAAGCAGC 180
CCTGTTTTCC AAATACCTAA AAACGACGAC ATTCCTGAGC AAGATAGTCT GGGACTTTCA 240
AATCTTCAGA AGAGCCAAAT CCAGGGGAAG TAGCAGGCTT GCAATCTTCA GGTAAAGAAG 300
CAGCTTTGAA TCTGAGCTTC ATATCGAAAG AAGAGATTGA AAAATACCNN 350

SEQ ID NO:7700
SEQUENCE LENGTH:351
SEQUENCE TYPE:nucleic acid
TOPOLOGY:linear
CLONE:HUMGS08950
SEQUENCE DESCRIPTION:
GATCCCGCCA GAAATGAATA GACACCGCAA GAAACAAAGC TCGAGTGAAA GCTTGTTACA 60
TAATGATTGG ACTCACAATT ATCGCCTGCT TTGCTGTGAT AGTGTCAGCC AAAAGGGCTG 120

TAGAACGACA TGAATCCTTA ACAAGTTGGN NCTTGGCAAA GAAAGCTAAG NGGCGTGAAG 180
AAGCTGCATT GGCTGCACAG GCTAAAGCTA AATGNTATTC TAAGTGACAA AGTGTTCACC 240
TGAATACCAT CCCTGTCATC AGCAACAGTN GAAGATGGGA AAAATNGAAT ATTTACCAAA 300
ATNTCTGCCA TGGTTTTATT TTGGTAACCN GATGCACAAT GTCTTTTTTT N 351

SEQ ID NO:7701
SEQUENCE LENGTH:85
SEQUENCE TYPE:nucleic acid
TOPOLOGY:linear
CLONE:HUMGS08951
SEQUENCE DESCRIPTION:
GATCATATGT GAAGATGTCA ATTAAGCTTG CATTAAGCCA CCTGCTTTGT AAGTGGATTG 60
ATTAATAAAT AACTTATATT TCAAA 85

SEQ ID NO:7702
SEQUENCE LENGTH:89
SEQUENCE TYPE:nucleic acid
TOPOLOGY:linear
CLONE:HUMGS08952
SEQUENCE DESCRIPTION:
GATCCCAGGG GCTTATCTCT TCAAGTGTGG AGAGGGCAGG GTCCACGCCT CTGCTGTAGC 60
TTATGAAATN AACTAATTGA AAATTCAAA 89

SEQ ID NO:7703
SEQUENCE LENGTH:375
SEQUENCE TYPE:nucleic acid
TOPOLOGY:linear
CLONE:HUMGS08953
SEQUENCE DESCRIPTION:
GATCCAGTGG CAATTGTAAA ACTAGCTATT TATGGCATGC TGCCAAAAAA CCTTCACAGA 60
AGAACATTTN ATGGAAAGGT TGCATCTTTT TCCAGATGAG TATATTCCNN CNGATATTCT 120
TAAGAATTTA GTAGAGGAGC TTCCTCAACC ACGAAAAATA CCTAAACGTC TAGATGAGTA 180
CACACANGAA GAANTAGACG CCTTCCCAAG ATTTGTGGAC TCCACCTGGA AGATTATCGG 240
CTATAAGAGA ATAAGATTTG CAGATAATTA ACAGTTGANG TGATTGAGAC TTTCTNCTTG 300
ATGAGTTTCT CTAACCTNCA GGGTGGTGTT AACCAACTGC TACAGTTCAG CACCTGTTTT 360
TNTTGTGCCG NTTTN 375

SEQ ID NO:7704
SEQUENCE LENGTH:276
SEQUENCE TYPE:nucleic acid
TOPOLOGY:linear
CLONE:HUMGS08954
SEQUENCE DESCRIPTION:
GATCACATCC TACTTCCTCA ATGAAGGGTC CCAAGCCCGT CCCCGTTCTT CCCACCGATA 60
TTTCCTNGAA CGCGGCCTGG AGTCAGCAAC CAGCCTCTAG CAGCTGCCTC TACGCGCTCT 120

ACCTGCTTCC CCAACCCAGA CATTAAAATT GTTTAAGGAG AACCACACGT AGGGGATGTA 180
CTTTTGGGAC AGAAGCAAGG TGGGAGTGTG CTCTGCAGCC GNGTCCAGCT ACTTCCTTTT 240
GGAACCTTTA AATAGAATGG GTGTTGGTTG ATTAAA 276

SEQ ID NO:7705
SEQUENCE LENGTH:220
SEQUENCE TYPE:nucleic acid
TOPOLOGY:linear
CLONE:HUMGS08955
SEQUENCE DESCRIPTION:
GATCCACTGC CATTCTAATT GCTTTAACAA GTCATTACCA CACTACTGTT ACATCTTAAT 60
TATGCATACA GACAGGTAGA CTTGTTTTAC ATATGTGAAC TAACTAGTTG TCAAAGCAAA 120
TGCAGATTGT ATTCTGCAAG TAAAGTCTTT TTCTCTCTGA AATTTCTAGG GATGTTCTTT 180
AAGTGAAATT CATATTAAAA CTGAAGATTT TNGTTACAAA 220

SEQ ID NO:7706
SEQUENCE LENGTH:252
SEQUENCE TYPE:nucleic acid
TOPOLOGY:linear
CLONE:HUMGS08956
SEQUENCE DESCRIPTION:
GATCCAAAGT AACTNAAACA GGAGCCCACA AGACGGTCTG CCAGATTGTN AGCGAAACCT 60
GCTCCACCAA AACCTGAACC CAAACCAAGA AAAACATCTG CTAAGAAAGA ACCTGGAGCA 120
AAGATTAGCA GAGGTGCTAA AGGGAAGAAG GAGGAAAAGC AGGAAGCTGG AAAGGAAGGT 180
ACTGCACCAT CTGNAAATGG TGAAACTAAA GCTGAAGAGG CACAGAAAAC TTGNTTCTNG 240
TNGATTAACG AN 252

SEQ ID NO:7707
SEQUENCE LENGTH:166
SEQUENCE TYPE:nucleic acid
TOPOLOGY:linear
CLONE:HUMGS08957
SEQUENCE DESCRIPTION:
GATCTCCTGA CCTCAGGTGA TTCACCCACC TCAGCTTCCC AAAGTGCCGG GNTTACAAGC 60
TTGAGCTACC GCGCCCAGCC AACACATCAC TTTTATCTTG CAGCTCCCGA GATTCTCTTT 120
TCTGTGACTN TTAAAATTGT GCTNACATAT GTGTTTGATA TAAATN 166

SEQ ID NO:7708
SEQUENCE LENGTH:52
SEQUENCE TYPE:nucleic acid
TOPOLOGY:linear
CLONE:HUMGS08958
SEQUENCE DESCRIPTION:
GATCAAAATA TTCTGGAAAA AAATAATAAA AAATAATTTG TATACATTGA AA 52

SEQ ID NO:7709
SEQUENCE LENGTH:318
SEQUENCE TYPE:nucleic acid
TOPOLOGY:linear
CLONE:HUMGS08959
SEQUENCE DESCRIPTION:
GATCATACTN GTGATTATAA AAGNTCCTAG GAGGCTAGAA GAGCCAACCA ACAGAGAAGG 60
GAAAGCAGTC TGTTCTGAAC ATAGGGACAT AAGTTCATTC ATGCCAAGTA TCTTTCCAGC 120
ATGTTTCTCC CATTTAGAAT ATCTAGCATN TAAGGCCTTT CAATATTAAT ATAAGCCCAA 180
TATCAGCTCT TTCTCTNTGT ATTTCATCTC TTTCTACTCT CCNATTTGTA TTTGGTGTTC 240
CTNTTGAAAG TGTCGTATCT GGGAGATGAC CTGCCTTATC CTGTNCTATA ACAGTTTNTG 300
NTTGCTGCTG TGTCTTTN 318

SEQ ID NO:7710
SEQUENCE LENGTH:243
SEQUENCE TYPE:nucleic acid
TOPOLOGY:linear
CLONE:HUMGS08960
SEQUENCE DESCRIPTION:
GATCCAAGAA ATTACTGAAA TGGACAAGAA ATGTCATTAG TTAAAAGCCA TGTTATCTTG 60
AGCACACATA CAGTTAATTT GATAAAGAAG AGTATGCCTC TCAGCATTGT GAGATATATT 120
TCAGACTAAT TTGATGTTCT TTAATGTTCC CTTATTTTTG AAACACGTTT TAAATGTGAA 180
ATTAATGTCC TTCACAAAAT GTCTTTTGAA AGCACTGTTA CAGTTGTATA AAGATGATGT 240
AAA 243

SEQ ID NO:7711
SEQUENCE LENGTH:122
SEQUENCE TYPE:nucleic acid
TOPOLOGY:linear
CLONE:HUMGS08961
SEQUENCE DESCRIPTION:
GATCTTGGCT TCTGAGGGCT CATTTGTAGA GTGGTGAGAA TAATATCTAA CTTGCTAATC 60
TTTCAGAGAC ATTCTGAGAG ACATATGCAA AAGAAACTTG TAAATAAAAA GCACTGTACA 120
AA 122

SEQ ID NO:7712
SEQUENCE LENGTH:240
SEQUENCE TYPE:nucleic acid
TOPOLOGY:linear
CLONE:HUMGS08962
SEQUENCE DESCRIPTION:
GATCCTTTAT TAAGCTTCTG ATTCATAAAA AAACCTATAA GAGACATTTG GGGGGATATT 60
TGGGGAAATA GGAAGATGGA ATGGGTAGTA GATAATGTGG TATATTTNAT TTTNCTAGAA 120
GTGATAATTG AGTTACGTAG GATAATGTTC TAATTCTTAG GAGATGCATG CAGAAGTTTT 180
TTTTTTAAGT TTTGAAAAGN CATGATGTTG NCAGCATATT TNCAAATAAN TATGTCCAAA 240

SEQ ID NO:7713
SEQUENCE LENGTH:110
SEQUENCE TYPE:nucleic acid
TOPOLOGY:linear
CLONE:HUMGS08963
SEQUENCE DESCRIPTION:
GATCTGTGTC CAAAAGTGAA CTTNAGTCAG GAATGAATCA ATTTCAGCAT AAACAAGCAC 60
AAAANTTTAG TCTGCTGGCT GACTGGAAGC AAAAAAGTCA AGATGGNNNN 110

SEQ ID NO:7714
SEQUENCE LENGTH:111
SEQUENCE TYPE:nucleic acid
TOPOLOGY:linear
CLONE:HUMGS08964
SEQUENCE DESCRIPTION:
GATCTCAAAA CAATTGTTGC AGCAGGCTCC TGGCAGTCTC AAGCAGTTCA TCTTCTTGGT 60
GTACTGGTTT CCTATTGTGA TTTTATCATG GAAAATCAAT TGGCTNNNNN N 111

SEQ ID NO:7715
SEQUENCE LENGTH:309
SEQUENCE TYPE:nucleic acid
TOPOLOGY:linear
CLONE:HUMGS08965
SEQUENCE DESCRIPTION:
GATCCTATAG GCAGAGAGTT TTCCTTTCTG ACTTTTTCCC TTTGCTTTCG TGTGACCACA 60
TGTTTTCTGT ACCAGTCACT GGGGAAAGAA GTGAGTTTAT CTCGTTTGTT TTAAAAGTTT 120
TGCTTGTCTA TTTAGCATTC CTTTTTGGGT CTCAAGATTT ATGGAACAAT AAATGTCATT 180
TAATGCTGTG TGCTTATTTT GAATTCCTCA TCAGGTTTTA GAAGCGGGGT AAAAATACTT 240
AGATGCTTAT CAGACTTGAA ATTATACTGA GTGGCATTGA ACGTGAGTTT GTCCCAGTGA 300
ACCAGGCTN 309

SEQ ID NO:7716
SEQUENCE LENGTH:204
SEQUENCE TYPE:nucleic acid
TOPOLOGY:linear
CLONE:HUMGS08966
SEQUENCE DESCRIPTION:
GATCCATGAA GCCAGAGCCT GGCTCTGGGT GACAGCAGGA AACGGCTGGG GAAAAGNACA 60
GCAGGGCGTC CAGCTGAGCC TGCCATTCCA GCCTGTTCGG GGGAAACAGC TCCCAATCCT 120
AGACTGTGCT GCTCCGGGAG GGCTGCTGGT GGATATTTGG GCTGTTTTCA CCATATAGCT 180
GTCATGAAAA ATGCTTCCGT GAAA 204

SEQ ID NO:7717
SEQUENCE LENGTH:121

SEQUENCE TYPE:nucleic acid
TOPOLOGY:linear
CLONE:HUMGS08967
SEQUENCE DESCRIPTION:
GATCTCAGGA ATACAGTCCC ATGCAAAGAT TCTCTGGTTT TATGGCTTTT TTCCCTTTCT 60
TTACACCATC CTCTCCCATA AGCACCCATG TNTTTGAATA TGAATGTATT TGTAAAATAA 120
A 121

SEQ ID NO:7718
SEQUENCE LENGTH:186
SEQUENCE TYPE:nucleic acid
TOPOLOGY:linear
CLONE:HUMGS08968
SEQUENCE DESCRIPTION:
GATCACCTGA GGTCGGGACT AAAAATACAA AAATNAGCTG GGCATGGTAG CCGGTNCCTG 60
TAATCCCAGC TATTTNGGAA ACTNAGAACA AGAGACTTGC TTGACCCTGG GAGGTGAAGG 120
TTGCAGAGAG CCGAGGTAGT GCCACTCCAC TCCAGCCTGG NCGACAGAGT GAAACTCCGT 180
CTCAAA 186

SEQ ID NO:7719
SEQUENCE LENGTH:351
SEQUENCE TYPE:nucleic acid
TOPOLOGY:linear
CLONE:HUMGS08969
SEQUENCE DESCRIPTION:
GATCAGCTCT CTGTTTTGGA AAATGGAGTA GATATAGTTC TAGGTACTCC GGGAAGACTA 60
GATGACTTGG TGTCAACTGG AAAGCTGAAC TTATCTCAAG TTAGATTCCT GGTCCTGGAT 120
GAAGCTGATG GGCTTCTTTC TCAAGGTTAT TCTGATTTTA TAAATAGGAT GCACAATCAG 180
ATTCCTCAGG TTACCTCTGA TGGAAAAAGA CTTCAGGTGA TTGTTTGCTC TGCCACTTTG 240
CATTCTNTCG ATGTAAAGAA ACTNTCCGAG AAGATAATGC ATTTCCCTAC ATGGGTTGAC 300
TTAAAAGGAG AAGGCTCTGT TTCCAGATTN CTGTACACCA TGTTGTTNAA N 351

SEQ ID NO:7720
SEQUENCE LENGTH:142
SEQUENCE TYPE:nucleic acid
TOPOLOGY:linear
CLONE:HUMGS08970
SEQUENCE DESCRIPTION:
GATCCCAAAG AAGTAACGGG AAAAAGGAAT GTAAGGCATC TCGATAATNT TTTCATATTG 60
ATTACATGTT GAAATANTAT TTTGTATNTA TTGAGTTAAG AAAAATATGT CATTAAAATT 120
AATNTCACCT GTTTCTTTTA AA 142

SEQ ID NO:7721
SEQUENCE LENGTH:130
SEQUENCE TYPE:nucleic acid

TOPOLOGY:linear
CLONE:HUMGS08971
SEQUENCE DESCRIPTION:
GATCTTGTGA ATCAAATTCA CAAACATCAG CTTGTTACAA ATCATTNTAT TTATACCCTT 60
TAAAGAGTGT GGATTTAAGA TTCTTGAAAG GATGTAGNCT ATATANTTTC TCATTCCNTA 120
TGGTGTAACN 130

SEQ ID NO:7722
SEQUENCE LENGTH:180
SEQUENCE TYPE:nucleic acid
TOPOLOGY:linear
CLONE:HUMGS08972
SEQUENCE DESCRIPTION:
GATCGTTAAT TAATATCTTT CCATNCGAAG AGNAAGCAGA TTTCTTCTGA GATTTCTGAA 60
CAGTTCCTGG GTGTTTCCGG TTCTGCTTCA GTGTACTATC ACTTTGCCTT GCCTCAGTCT 120
CTCCTTCCTC CGGGGCCACA GAAGGAGAAA GCCCAAAGCA TACCCATCCT GCCCTTTAAA 180

SEQ ID NO:7723
SEQUENCE LENGTH:266
SEQUENCE TYPE:nucleic acid
TOPOLOGY:linear
CLONE:HUMGS08973
SEQUENCE DESCRIPTION:
GATCACTGTC CCGNTTCAAA TTATTCTTCA GTCCATTTCC CCGGCCTATT TCAGCTGTTC 60
CTTTTCACCT AACTGTTNAG TCATTCNGGT TTTCAAGCAG TGCTTNATCT CATGTCCTTG 120
AATATAGTTG TGTACTTTAT TNTTNAGGTA ATAATTAGAA CAGTTCCCTT CAGAGGCTGC 180
ATTTGCCTTC TTCTGCCACC TAAATATTAC TTCCCTTCAA ATCNGCCTTT GAATCATCNN 240
NTTTAAAAAA AANTTANCAT GNTTTN 266

SEQ ID NO:7724
SEQUENCE LENGTH:33
SEQUENCE TYPE:nucleic acid
TOPOLOGY:linear
CLONE:HUMGS08974
SEQUENCE DESCRIPTION:
GATCAAAGGT ACCATGACCA AATAAGAAAC AAA 33

SEQ ID NO:7725
SEQUENCE LENGTH:219
SEQUENCE TYPE:nucleic acid
TOPOLOGY:linear
CLONE:HUMGS08975
SEQUENCE DESCRIPTION:
GATCTTCTTT TTTNTTCTGG AGCCAGACTT NCTGGGCTTN NATTGCTGCT ATGCTACTTA 60
CTTGGAAATC TTGGACAAGT TACCTAATTT CCTTGTTCTT AAGTTACTTC TGTTGTAAAA 120

TGATGTGGCC AATAATAGTG CCTATCTCAT AACATGGTTA TTAGAATTAA ACAAGTTAAT 180
ACTTTTAAAA TGCTTAGAAC AATTGTGGGT ACATACTAN 219

SEQ ID NO:7726
SEQUENCE LENGTH:275
SEQUENCE TYPE:nucleic acid
TOPOLOGY:linear
CLONE:HUMGS08976
SEQUENCE DESCRIPTION:
GATCTGTCTG GNAACAGTTT ATGGAAATAT AGATATTCAT GCATCANATA AAAGTGCTGT 60
GACCATAGAT AAACTGCAGG GAAGTTCTGT NACTGTATCT ACCGAAGATG GTTTGCTGAA 120
AGCCAAGTAT CTTTATACAG AATCATCATT TCTGTCTTCT GCTGCTGGGG ATATTACATT 180
AGGAAGTGTT CATGGNAATA TAACATTACA AAGCAAGANG GGTAGCATCA CAGTNGATTC 240
GTCTTCTGGA TGTCTAAAAG CCTCAACTAA TCAGN 275

SEQ ID NO:7727
SEQUENCE LENGTH:37
SEQUENCE TYPE:nucleic acid
TOPOLOGY:linear
CLONE:HUMGS08977
SEQUENCE DESCRIPTION:
GATCCCAGGT TCTCATGAAC CAAATAAGTA ACTTAAA 37

SEQ ID NO:7728
SEQUENCE LENGTH:97
SEQUENCE TYPE:nucleic acid
TOPOLOGY:linear
CLONE:HUMGS08978
SEQUENCE DESCRIPTION:
GATCTGCCCA CCTNGGCCTC CCAGAGTGCT GGGATTACAG GCATGAGCCA CTGCGCCCGG 60
NCTGTACTAA GTCTTTTTTT TTTAAATTTC CTCNAAA 97

SEQ ID NO:7729
SEQUENCE LENGTH:284
SEQUENCE TYPE:nucleic acid
TOPOLOGY:linear
CLONE:HUMGS08979
SEQUENCE DESCRIPTION:
GATCTATGAA CTTCTGCCCC ACATAACTTT TAAAGCAGTA TTTTATAAAC CCCATGAAGA 60
GCTTGCTTTT TTGTCTTTGT AATTCCTTAG GGTCTGTGAT ATATGCCAGG AAATCTGATT 120
GAAACTAATA TGCTTTTTAT TCTCCCATTA TTCCCTAAAT NGTTATATCA CAGGCACTTG 180
CCTACATTGG GAAAGTAAGG ACAAATAATA CCCATTTAAA GTAACTNGCC TATTATTNNG 240
ACTGTCTTTC GTAAGCACTT GCTGAACNGC TGACTTAACT CAGN 284

SEQ ID NO:7730

SEQUENCE LENGTH:374
SEQUENCE TYPE:nucleic acid
TOPOLOGY:linear
CLONE:HUMGS08980
SEQUENCE DESCRIPTION:
GATCTCGCAA TGGGGAAGGC CCTGGTCGAC CAGAGGGAGC TGTACCTGGG CCTGCTCTAC 60
CCCACGGAGG ACTACAAGGT ATACGGCTAC GTCACCAACT CCAAGGTGAA GTTTGTCATG 120
GTGGTAGATT CCTCCAACAC AGCCCTTCGA GACAACGAAA TTCGCAGCAT NTTCCGGAAG 180
CTACACAACT CCTACACAGA CGTGATGTGC AACCCCTTCT ACAACCCGGG GGACCGTATC 240
CAGTCCAGGG CCTTTGATAA CATGGTGACG TCGATGATTG ATACAGGTTG TGCTGAGTGA 300
GCTGTGCTGC CAGCCATCGN AGAGGAGCCC NTNGCACGAC TTGTGGTGGG GTCCGTCGGT 360
CTTGTTTCTN TTTN 374

SEQ ID NO:7731
SEQUENCE LENGTH:164
SEQUENCE TYPE:nucleic acid
TOPOLOGY:linear
CLONE:HUMGS08981
SEQUENCE DESCRIPTION:
GATCCGGTTG GACTCTGACA TCGGATGCCC TCAAACATAC AGAACTTCCA AACTCAAGTC 60
CAGCCATAAG CTATTTTGCC AACATGTCAG AGTAATCTGT ATTTTTGTAT GTGATTTCTA 120
CTTTTATAGA CTTGTTTTAA AACAATAAAA CACATTTTTA TAAA 164

SEQ ID NO:7732
SEQUENCE LENGTH:287
SEQUENCE TYPE:nucleic acid
TOPOLOGY:linear
CLONE:HUMGS08982
SEQUENCE DESCRIPTION:
GATCTTTCAT GAAAATTAAT TGTGCCCATG TCCAAGTTTG AATTAGAGAT ACACAGCACA 60
CAATCATTTC TGTTACCACT TTTGGAATAT CTAGCATTAG CCTTGATAGT TTTGTGTGGT 120
GTGTTTTGAG TATATCTGAN CTGTTAGTTA TATTTGGTTA ATTTATTAAA NGATGTGTGT 180
TAANCCTTAA TATTTATGCA GTGTTTAAGT ATTTGGAATA TATTNGAAAT AAATTATCCA 240
GTGTTTTAGA TAAATGGTTA TTTAATTGCN NAANGTGATT CTCTAAA 287

SEQ ID NO:7733
SEQUENCE LENGTH:173
SEQUENCE TYPE:nucleic acid
TOPOLOGY:linear
CLONE:HUMGS08983
SEQUENCE DESCRIPTION:
GATCTGTGCC GTTCAATAGG CATCGTCTCT CAGCCTGAGG GAGGCTGGAT TCTGGGTTCC 60
TGTAGTCACA GGGAGGAAAA GCTTTCTTAA AAATGGACAT GTATGTGCGT GTNAGTGTGT 120
GTGTAGATTT ATAGTTTTTG GTAGTGGCAG GAATAAAAAA AATCCATCCT AAA 173

SEQ ID NO:7734
SEQUENCE LENGTH:198
SEQUENCE TYPE:nucleic acid
TOPOLOGY:linear
CLONE:HUMGS08984
SEQUENCE DESCRIPTION:
GATCTCTATT TTGTGCCCAG ACTCTGAGCT GCTTTATGAA CACCTCAATT AAATNACTTC 60
ATTGGAGTGA TTTGAGTAAT CTATCTATAG TGTAATATTT TCTATGAATA ATTTTCTGTA 120
ACCTTATTTC ATAAGTTAAT ATCTAAAAAG TGTTTATTTC CAAATTGCCA AATAAAAGGT 180
TTTATATTAG TCATTAAA 198

SEQ ID NO:7735
SEQUENCE LENGTH:94
SEQUENCE TYPE:nucleic acid
TOPOLOGY:linear
CLONE:HUMGS08985
SEQUENCE DESCRIPTION:
GATCTAGAGA CTCATTCAAT AGCAATGTGA CCTTTTAAAT ACTTACATTA AGTAAAACTG 60
CCAGTAGATT AAATCATATA TATATATATA TATN 94

SEQ ID NO:7736
SEQUENCE LENGTH:363
SEQUENCE TYPE:nucleic acid
TOPOLOGY:linear
CLONE:HUMGS08986
SEQUENCE DESCRIPTION:
GATCACCTGN ACTCCAGCTC AGATTGCCTC TCCTGGACAT GGCAATGAAT GAGTTTTTAA 60
AAAACAGTGT GGATGATGAT ATGCTNTTNT GAGCAAGCAA AAGCAGAAAC GTGAAGCCGT 120
GATACAAATT GGTGAACAAA AAATGCCCAA GGCTTCTCAT GTCTTTATTC TGAAGAGCTT 180
TAATATATAC TCTATGTAGT TTNNTAAGCA CTGTACGTAG AAGGCCTTAG GTGTTGCATG 240
TCTATGCTTG TGGAACTTTT CCAAATGTGT GTGTCTGCAT GTNTGTTTGT ACATAGAAGT 300
CATAGATGCA GANGTGGTTC TNCTGGTACG ATTTGATTCC TGTTGGGATG TTTTAACTAA 360
NAN 363

SEQ ID NO:7737
SEQUENCE LENGTH:262
SEQUENCE TYPE:nucleic acid
TOPOLOGY:linear
CLONE:HUMGS08987
SEQUENCE DESCRIPTION:
GATCTTCTTT GATGATGAGA GGCGGAATAT TGTAGACGTC AGCAAACTGG GTGTTACCTG 60
CATTCACATC CAGAATGGAA TGAATCTTCA AACTCTAAGT CAAGGGTTAG AGACATTTGC 120
AAAGGCCCAA ACTGGGCCTT TAAGGTCCAG CCTTGAGGAG AGCCCATTTN AGGCCTAAAC 180
TGAAAGGAAA TCAAGAAGGC ATTTTCAGGT GCATTTGTAA TTTATTAAAG TTCATCTGTG 240
TGTGACAGAA AAAAAAAGTA AA 262

SEQ ID NO:7738
SEQUENCE LENGTH:361
SEQUENCE TYPE:nucleic acid
TOPOLOGY:linear
CLONE:HUMGS08988
SEQUENCE DESCRIPTION:
GATCTGTGCC ACATGGAGGT TTTGGGATGG GATTTNAACG CTACCTGCAG TGCATCTTGG 60
GTGTTGACAA TATCANAGAT GTTATCCCTT TCCCAAGGTT TCCTCATTCA TGCCTTTNAT 120
AGCTGGAAGA TTGGTTAAGG CAAAGCACCC CCCATGGCAG AGACACTGCA CATGATTGTN 180
CATACAGCAG AATGCATGTT TGGATTNTAG AAATGCAGAT TTCAATATGT AATTGTTGTG 240
CCATAAGATA TCATAGNAAA NNTATAAGTG GTTGTNCTTT TCTTAGAAAG TTGAGGGTAT 300
TTCACGTAAG GATGAGCTCC CGCAAGNNGA GGTACTTNTT AGCANGGGGA CTCTCAATCG 360
N 361

SEQ ID NO:7739
SEQUENCE LENGTH:143
SEQUENCE TYPE:nucleic acid
TOPOLOGY:linear
CLONE:HUMGS08989
SEQUENCE DESCRIPTION:
GATCTTGATG TGGGTGATAG TTTTACTTAT GTACATCGAT ATACATCTAT TTAAAAACAT 60
GTACATTTAA ATTGTTGCAT TTTATGTATT TAAAATTTAT TGAGTANTCC TTCTGTATGT 120
ATTATTAAAG AGAATATAGC AAA 143

SEQ ID NO:7740
SEQUENCE LENGTH:333
SEQUENCE TYPE:nucleic acid
TOPOLOGY:linear
CLONE:HUMGS08990
SEQUENCE DESCRIPTION:
GATCATAGAG TTTGGCTGGG GAGGGGGGCA GTTTTAGAGG CTTCCACTTG GTGTTCCTCA 60
GAATGATATC TCTTACTCCG GGGGCCAAGG TAGGGGTTAG CTTTTGTTCT CTTTGTAGTT 120
TAGATTGTAT CTCTTGCCTT GTTCAAGTTC ACAAATCTTT TTGTGTATAC ACATATGTAC 180
ATGAAAATNA TGTTCATGCT TTTAATTATT TTACCCTTCA TTATTTCATT TTTNATAGTT 240
CTCATAGCTA TGTCTTTCAG TTCACTAATC TTTTTTCCAC AGTGTTTAAT CTGTCATTAA 300
ATCCCATCCA AAGTATGTTT TCTCTCAGAA GAN 333

SEQ ID NO:7741
SEQUENCE LENGTH:135
SEQUENCE TYPE:nucleic acid
TOPOLOGY:linear
CLONE:HUMGS08991
SEQUENCE DESCRIPTION:
GATCCTTGTA CACTGTTTAT ATGTGCAATA AAATGCATGG CCAGCTGACA GTATCGTCAG 60

TGGACAGAAT GTATATAGTG CAAATATCCT TCCTTTTATT ATTTAACAGC CATTAAAATC 120
TTGGATTTGT ACAAA 135

SEQ ID NO:7742
SEQUENCE LENGTH:333
SEQUENCE TYPE:nucleic acid
TOPOLOGY:linear
CLONE:HUMGS08992
SEQUENCE DESCRIPTION:
GATCCCAGTT CACAATTATT CACCGGGTAT TTAGTTATTC ANAGGCTGCT CTGCTGAGAA 60
GNTGAACAAA TTTCTTGTCC AAAACAATGT ATTTCAAACG TGCCGCTCGG GCCTTTCCCG 120
TATTGCTCAC TGGTGCTGGG AAGACAACAC TTCTGAACTA TATTTTGACA GAGCAACATA 180
GTAAAAGAGT AGCGNNNNCT TTAAATGAAT TTGGGGAAGG AAGTGCGCTG GAGAAATCCT 240
TAGCTGTCAG CCAAGGTGGA GAGCTCTATG AAGAGTGGCT GGAACTTAGA AACGGTTGCC 300
TCTGCTGTTC AGTGAAGTGA GGAATGTGTT TTN 333

SEQ ID NO:7743
SEQUENCE LENGTH:219
SEQUENCE TYPE:nucleic acid
TOPOLOGY:linear
CLONE:HUMGS08993
SEQUENCE DESCRIPTION:
GATCCCAACT GTATAACATT CTGAAAAAGG CACAACTATG GAGACAATAA AAAGAACAGA 60
GTTTTGGATG AAGGGAGGGA TGAACAGGCA GAATACAAAC AAATTTTAGG GCAGTGAAAC 120
TATTATTATG ATACTTTATG GATATAGAAT ATTATGCATT TTTCAAAACA CATAGAATAT 180
ACAACATGAA GAATGAATGC TAATGTAAAC TGGATTTTN 219

SEQ ID NO:7744
SEQUENCE LENGTH:59
SEQUENCE TYPE:nucleic acid
TOPOLOGY:linear
CLONE:HUMGS08994
SEQUENCE DESCRIPTION:
GATCTTGGGC CACACATGAA AATACACTAA CACTAATGAC AGCTGATGAG ATGCTTAAA 59

SEQ ID NO:7745
SEQUENCE LENGTH:353
SEQUENCE TYPE:nucleic acid
TOPOLOGY:linear
CLONE:HUMGS08995
SEQUENCE DESCRIPTION:
GATCAGAGTG ATGCTGGAAC TCAGTGTGCA TAAAATTTCA GTCAGTGAAT ATCACTGAAC 60
GTCATATACT ACTTGGTATG TGACTTTGGT TTGTGTTAAG AAAGCTTGTA TATAATATTT 120
TTNGCCATAG TAAGTGAGAA ATTGTCCTTA ATCATGCCTG TTTGATGGTA CTAGGAAAGA 180
AAGGGGTAGA GATTAATTCT TGCACAGTAT AAGCAACAGT GCAACAAACT ATGCCATTTN 240

CCTTTTNCCT CTTACTTGAA GGCAGAATCG CAAAACGTTT GANATGGCTT TCCTAAACTA 300
CTCTACTCTG GTGNGAGCTC ATTTACCACA NGAAGCCTTA TAACACCGTT TNN 353

SEQ ID NO:7746
SEQUENCE LENGTH:327
SEQUENCE TYPE:nucleic acid
TOPOLOGY:linear
CLONE:HUMGS08996
SEQUENCE DESCRIPTION:
GATCAGGTGA TTGCCTTTCT CAGCTGTCAG TTCTCTAATT TCAGGCTTGG TAGCTTGTAG 60
GAACTGAAAT TGCAATTAAA ACCTTTATAA ACTCAAACTA AATCATGAAT TACAGAAAAA 120
GTCCATTCTT CCAAAACTTG ATGTTACCAC ACTTACAAGT TTAAAATATG AAGTCGACTG 180
TTTAAAGGAT TCTGCATATA TTCTAGTGTG CACATTCAGA AACATTTTTC TTGGAAAAAG 240
TACCCAACAT TTTTTATAAC TGCACATATT AATTTATTGC CAGAATAAAT TGCATTGCAT 300
GCTAAATTAA NGTCAGATAN TTCNAAA 327

SEQ ID NO:7747
SEQUENCE LENGTH:153
SEQUENCE TYPE:nucleic acid
TOPOLOGY:linear
CLONE:HUMGS08997
SEQUENCE DESCRIPTION:
GATCATGTAA ACTTGCTGTT TTNGTTTTTN CCTGCCGGGT GTTGTATGTG TGGTGACTTG 60
CGGATTTATG TTTCAGTGTA CTGGAAACTT TCCATTTTAT TCAAGAAATC TGTTCATGTT 120
AAAAGCCTTG ATTAAAGAGG AAGTTTTTAT AAA 153

SEQ ID NO:7748
SEQUENCE LENGTH:41
SEQUENCE TYPE:nucleic acid
TOPOLOGY:linear
CLONE:HUMGS08998
SEQUENCE DESCRIPTION:
GATCTTAATT TTGTTTTTGG TTTAAAATAG TGTTTCCTTT N 41

SEQ ID NO:7749
SEQUENCE LENGTH:299
SEQUENCE TYPE:nucleic acid
TOPOLOGY:linear
CLONE:HUMGS08999
SEQUENCE DESCRIPTION:
GATCTATCTA AATGCCGATT TGAGTTCGCG ACACTATGTA CTGCGTTTTT AATTCTTGTA 60
TTNANACTAT TTAATCCTTT CTACTTGTCG CTAAATATAA TTGTTTTAGT CTTATGGCAT 120
GATGATAGCA TATGTGTTCA GGTTTATAGC TGTTGTGTTT AAAANTTGAA AAAAGTGGAA 180
ANCATCTTTG TNCATTTAAG TCTGTATTAT AATAAGCAAA ANGNTTGTNT GTATGTNTGT 240
TTNATATAAC ATGACAGGCA CTAGGACGTC TGCCTTTTTN NGGCAGTNCC GTTAAGGGN 299

SEQ ID NO:7750
SEQUENCE LENGTH:169
SEQUENCE TYPE:nucleic acid
TOPOLOGY:linear
CLONE:HUMGS09000
SEQUENCE DESCRIPTION:
GATCAATTAG ACATTTTGAA AATAATTTAA AGTGTTTTCC TTAATGTTCT CTGAAAACAA 60
GTTTCTTTTG TAGTTTTAAC CAAAAAAGTG CCCTTTTTGT CACTGGATTC TCCTAGCATT 120
CATGATTTTT TTTTCATACA ATGAATTAAA ATTGCTAAAA TCATGGAAA 169

SEQ ID NO:7751
SEQUENCE LENGTH:53
SEQUENCE TYPE:nucleic acid
TOPOLOGY:linear
CLONE:HUMGS09001
SEQUENCE DESCRIPTION:
GATCATTTAA TGAATCCTCA AGGACTAATG AAATAAATGC TAGACTGCTG AAA 53

SEQ ID NO:7752
SEQUENCE LENGTH:252
SEQUENCE TYPE:nucleic acid
TOPOLOGY:linear
CLONE:HUMGS09002
SEQUENCE DESCRIPTION:
GATCCTGAAA ATAAAACTAA CACTCCAGTA TTTTGTCATT GTTTTTCGCA ATTGAGCTAT 60
CTGAAAACTG TTATTCCTAA GTAATGTTCA AAAATGATAA GTAATCTGGA TACCTTTTTC 120
TTATACTTTC TCCTAGGAAA ACTTTAAAAC TTTAAAAAGG CAAACCTACC AATAGGAATA 180
ACANATTAAA TGTCAAGAGA GTATATCCAA TATTAGGATA TAANTGTATG TGTCTCAAGT 240
TTAACTCTAC AN 252

SEQ ID NO:7753
SEQUENCE LENGTH:237
SEQUENCE TYPE:nucleic acid
TOPOLOGY:linear
CLONE:HUMGS09003
SEQUENCE DESCRIPTION:
GATCTACAAA AAATTCCTGG AGCCATATAT ATACCCTCTG GTNTCCCCAT TCGCTAGTCG 60
TATATNGCCT NAGAAAGCAA TACAAGAATC CAATAATACA AACAAAGGCA NAGTAAACTT 120
TAAGGGNGCA GACATGAATG GCTTACCAAC AAAAGGACCA NCAGAAATCT GTGATAAAAA 180
GAAAGACTAA AGAAATTTTC CTAAAGGACC CCATCATTTA AAAANTGGNC CNGGNNN 237

SEQ ID NO:7754
SEQUENCE LENGTH:264
SEQUENCE TYPE:nucleic acid

TOPOLOGY:linear
CLONE:HUMGS09004
SEQUENCE DESCRIPTION:
GATCTTCCCA AACCCAGAGA AAGACATCAA TTTCCAAGAA CAAGAAGGTT ATCAAACACC 60
AGGCAGATTT AACCCAGAGG ATACTACCTC AAGGCATTTA ATAATCAAAC NNCCAAAAGT 120
CAAGAATAAG GAAATAATCC TAAAAGCAGC AAGAGAAAAG AAACAANTAA TATACANTGG 180
AGTTCCACTA TATCTGGCAG CAGACTCTTC ATTGGAAACC TTATAGGCCA GGNGNGAGTG 240
GCATGACACA TTTAANGTAC TGGN 264

SEQ ID NO:7755
SEQUENCE LENGTH:286
SEQUENCE TYPE:nucleic acid
TOPOLOGY:linear
CLONE:HUMGS09005
SEQUENCE DESCRIPTION:
GATCCAGAGC TGTTGCCTGT GACAGCGGTT TCTNTGGATG TCAAAGGCAG CTGCCTGGTT 60
GCCCAGCTTG CTTCTCGACT GGTGGCCCCT ATGGGTGGGT GTGCGATGGA AATNTGTTCC 120
TGCCGGAGTC TGAGGCACCA GGGTGTGCTC AAAGGCTGGC CCTGGTGGTG GACTGGCACC 180
TGTGCAGAGT GCCGTGTGCT TGTGGTGCGC CATCTGAAGC AAGAGTCCAG CGTTCTGCCG 240
TGTCTGTCCC CCACCATGCC CCCTACAGGC GGTACTGATG GCGCTN 286

SEQ ID NO:7756
SEQUENCE LENGTH:382
SEQUENCE TYPE:nucleic acid
TOPOLOGY:linear
CLONE:HUMGS09006
SEQUENCE DESCRIPTION:
GATCCTTTTT CCTTCAGCAG CATTTCTTAC TGGCTGTGGC TGGAATCTGC CTTTAATCAC 60
AGCTGTCACC ATTCTCACGT GATTCTTGTG AGACTCTTTT TGGTTATAAT TACTATTTAA 120
TATTTAGACT ATTTTACTGA GCAGACTTTA TAAATGAGAT ATCTACAAGG CACTTAAAGT 180
GTTACAGATG TTTTACCTTA AGANTTATTT AAGTNGTGTT GGGTTAAGAC AGTTTTCAGT 240
GTACCGTAAA TGTTGTGTTT TCAGAAAAAG ACAAAACGAT GGTGCTGACT GGTTTTCTGT 300
ATATNGCACA ACAGTCCTCA AATACACTGG ATGTATGGAA CCTNTTCCTT NCATCCAGGC 360
AGCATTTTTN TTNCNCTCTN CN 382

SEQ ID NO:7757
SEQUENCE LENGTH:298
SEQUENCE TYPE:nucleic acid
TOPOLOGY:linear
CLONE:HUMGS09007
SEQUENCE DESCRIPTION:
GATCATCTTT GAGTAGCACT GTTTTGGGGC CCTCGGTCTC TCTGAAGACC CTAGCAGAAC 60
TGATACCTAC CTGTATCTCT TGTTCTCTCC TATTTGAGTT TCACTTCCAG AGAACTTGTT 120
CTTCAGCAAG AATGTGTCAC TAGTAAGGAC ATCTCTAGCA TTTCTCTAGC CTTCCTTTTC 180
TGCTGCTCAA AAATAATCGT TACAAAGCTT AGGTTTAAGC TGTATATGAA ATATTTATGC 240

GACTCTCAAA CTTTAAAGGA GTTGCTCCTT TGTTCCAAAA TTAAATGTGT TAGATAAA 298

SEQ ID NO:7758
SEQUENCE LENGTH:292
SEQUENCE TYPE:nucleic acid
TOPOLOGY:linear
CLONE:HUMGS09008
SEQUENCE DESCRIPTION:
GATCATGGTC TTCAAAGATG TAAATACAAG AGAACCAAAG TTATTCCGGG CAGTTCCAGA 60
TTCAGAACGT GTTATTCCTC CCAGAACGAC AACGCCATAT TTCCTTTGAG GCCTTCGCCC 120
ATCCTGCTGA CCCATTTTTN TGCCCTCTTC TTACCCCAAT TTTNTTGTAT TACCCTCTAC 180
AATATACTTT TNATTGAGCA CTTTGCTGCT GAAATGCTGC CTCTTGCCTT TTTTTTTTNA 240
AATTTTAAAT TATCTAAATT TATGGTTTGT GGTGGTGTCT ATNGCAAAGT TN 292

SEQ ID NO:7759
SEQUENCE LENGTH:307
SEQUENCE TYPE:nucleic acid
TOPOLOGY:linear
CLONE:HUMGS09009
SEQUENCE DESCRIPTION:
GATCTGTTAA TGAAAAGTGC TGGAATACCT CAACCAAGAA AAGGCAGAAA AGGGAAGGGA 60
GCATATTGCT AAGAGAGCAA TGGAAAATAT GTAAGCTGCT TTCATTAATT ACCCTACTTT 120
CATTCCTCCC ACCCCAAGCA AATCCCAACA TTTCTCTTCA GTGTGTTGAC TTCTATCCTG 180
TTAACACTGT AATATCTTTA AATGATGTAC AGGCAGATGA AACCAGGTCA CTGGGGAGTC 240
TGCTTCATTT CCTCTGAGCT GTTATCTTGT GTATGGATAT GTGTAAATGT NGGTGACTCC 300
TTGATAN 307

SEQ ID NO:7760
SEQUENCE LENGTH:165
SEQUENCE TYPE:nucleic acid
TOPOLOGY:linear
CLONE:HUMGS09010
SEQUENCE DESCRIPTION:
GATCTCTGAA CATGAAAACC AAGGTGGCTA TTTTCAGGTT GCTTTCAGCT CCAAGTAGAA 60
ATAACCAGAA TTGGCTTACA TTAAAGAAAC TGCATCTAGA AATAAGTCCT AAGATACTAT 120
TTCTATGGCT CAAAAATAAA AGGAACCCAG ATTTCTTTCC CTAAA 165

SEQ ID NO:7761
SEQUENCE LENGTH:334
SEQUENCE TYPE:nucleic acid
TOPOLOGY:linear
CLONE:HUMGS09011
SEQUENCE DESCRIPTION:
GATCGATGAC GCAGGTCCTT CCAGTGAAAT GAATGGTCTC AAAAAATGGA GAGGATGTGC 60
TGGGGATTTA GAAGGCAGGG TAGCCAGAAA GAAGCACAAA TTACCCCCAC CATAAGCTTC 120

ACAGAATTGC TGACGCTAAC GGCCATGGGT GAGATTTTTG CAATAAGTGT CAGCCTGATG 180
ACAGATACCA CAGACAGTTG CAAAAATGTC CAAGTTTCTC CTGAAGTTTT CTGGCAGGGC 240
AGTGGGCTCC ATCCTTAGGA GTGAATCAGC TTTGGTGAGT ATCGTGATTG AACAGAGCCT 300
TAACACCAGG GCCCCCTTAA CTCCGCACTC CTTN 334

SEQ ID NO:7762
SEQUENCE LENGTH:247
SEQUENCE TYPE:nucleic acid
TOPOLOGY:linear
CLONE:HUMGS09012
SEQUENCE DESCRIPTION:
GATCGCTTTG ATTTTAAAGT TCATTGGAAC TACCAACTTG TTTCTAAAGA GCTATCTTAA 60
GACCAATATC TCTTTGTTTT TAAACAAAAG ATATTATTTN GTGTATGAAT CTAAATCAAG 120
CCCATCTGTC ATTATGTTAC TGTCTTTTTT AATCATGTGG TTTTGTATAT TAATAATTGT 180
NGACTTTCTT AGATTCACTT CCATATGTGA ATGTAAGCNC TTAACTATGT CTCTTTGTAA 240
TGTGTAN 247

SEQ ID NO:7763
SEQUENCE LENGTH:312
SEQUENCE TYPE:nucleic acid
TOPOLOGY:linear
CLONE:HUMGS09013
SEQUENCE DESCRIPTION:
GATCCTTCCT GAGCGTTTCC TGGCAGTTTT AAAACGAAAA ATCAGTGTTA AGTTTGATGC 60
AGTTATTGGA TATAAAATGA AAGCGCAATA AGCACCTAGT TTTCTGAAAA CTGATTTACC 120
AGGTTTAGGT TGATGTCATC TAATAGTGCC AGAATTTTAA TGTTTGANCT TCTGTTTTTT 180
CTAATTATCC CCATTTCTTC AATATCATTT TTGAGGCTTT GGCAGTCTTC ATTTACTACC 240
ACTTGTTCTT TAGCCAAAAG CTGATTACAT ATGATATAAA CAGAGAAATA CCTTTAGAGG 300
TGACTTTAAG GN 312

SEQ ID NO:7764
SEQUENCE LENGTH:93
SEQUENCE TYPE:nucleic acid
TOPOLOGY:linear
CLONE:HUMGS09014
SEQUENCE DESCRIPTION:
GATCTGCCTG TCCGGGCTGG GACAGAGACT CCCCAAGNAC CCCATTCTGC CTCCTTCTGG 60
GGAAATAAAT AAGTGTCTGT TTCAGCAGCT AAA 93

SEQ ID NO:7765
SEQUENCE LENGTH:133
SEQUENCE TYPE:nucleic acid
TOPOLOGY:linear
CLONE:HUMGS09015
SEQUENCE DESCRIPTION:

GATCTGGCTA AGCATCTTCC TCATGCTTTC AGTAAATTGG AAAACCCACG GAGGTTAAAT 60
ATTACCTTTT TTTTTTTAAA CTTAAANCAA TNCTGTTTAT TTTTTNATTA CATTTNCCCA 120
TATGNGNGGA ATN 133

SEQ ID NO:7766
SEQUENCE LENGTH:300
SEQUENCE TYPE:nucleic acid
TOPOLOGY:linear
CLONE:HUMGS09016
SEQUENCE DESCRIPTION:
GATCGTNAAA ACCCCAAATG GACTTATTTT AAGTCACTTG CCAAATGGCC CAACTGCTCA 60
TTTTAAAATN AGCAGNGTTC GTCTTCGTAA AGAAATTAAG AGAAGAGGCA AGGACCCCAC 120
AGANCACATA CCTGAANTAA TTCTGAATAA TTTTACANCA CGGCTGGGTC ATTCANTTGG 180
NCGTATGTTT GCATCTCTCT TTCCTCATAA TCCTCAATTN NTCGGNAGGC AGGTTGCCAC 240
ATTCCACAAT CAACGGGATT ACATATTCTT CAGATTTCAC AGATACATAT TCAGGTGTGN 300

SEQ ID NO:7767
SEQUENCE LENGTH:105
SEQUENCE TYPE:nucleic acid
TOPOLOGY:linear
CLONE:HUMGS09017
SEQUENCE DESCRIPTION:
GATCAAAGTA GCCACAACAT TCTCTTAAGC CAAAGNCCAA TCCAGAACAA NGCCCTAACT 60
CTCTTCAATT CTATGAAGAC TAAAAAAGTA AGGAAGCTNC AGANN 105

SEQ ID NO:7768
SEQUENCE LENGTH:216
SEQUENCE TYPE:nucleic acid
TOPOLOGY:linear
CLONE:HUMGS09018
SEQUENCE DESCRIPTION:
GATCGTCAAA CCATTCATCC TTTTAAAGGT TTATTTGAAG ATGCTGTTAA AGTACAGAAT 60
TTTGTGTACA GGTAGATTTT TCCGTCCCTC ATTAATAGTG CCTTCTTAAT TAATACAGNC 120
TGGTGTTAGC TATAACAAGG CNCCAGTAAG GCCAAAGAAT CCCAAGTTCT TTGTGGAAAA 180
ANAAAAAANN NCTTTTGGGG NCAGNTTTCC CCTTCN 216

SEQ ID NO:7769
SEQUENCE LENGTH:75
SEQUENCE TYPE:nucleic acid
TOPOLOGY:linear
CLONE:HUMGS09019
SEQUENCE DESCRIPTION:
GATCCTCCCG TGTGTGGTTG GAAAACTTTT GTTTTTTGGG GTTTTTTTTT TCTGAATAAA 60
AAAGATNCTA CTAAA 75

SEQ ID NO:7770
SEQUENCE LENGTH:341
SEQUENCE TYPE:nucleic acid
TOPOLOGY:linear
CLONE:HUMGS09020
SEQUENCE DESCRIPTION:
GATCTGTGTC TTAATTGTTC AGTTAGAGTG AGAAGTTGAC CTATGATTCN TTTTTAAATT 60
TTATATTTGG AACAAAGCTG CAAGTTATGG TAANGTACTG TACTGNGNGA NGTATTATGA 120
TATTTAATGC ATCTNTGGCT TAACACTTGT GAGAGTTACC AGCTTGAAAA TGATGGTGTT 180
GACTACCTCT TGANTCACAT CTATCAACCA CTGGCACCTA CCACCAAGCT GGCTTCAATT 240
AGTATGTGTT GCTTTTTGGT ATTAACANCT AACCGTACTA GAGACCAAAG TGAACCCTGA 300
TNTTTAATAT GTNTTTAATA ATGGTGGTTT TATCTAGTGG N 341

SEQ ID NO:7771
SEQUENCE LENGTH:238
SEQUENCE TYPE:nucleic acid
TOPOLOGY:linear
CLONE:HUMGS09021
SEQUENCE DESCRIPTION:
GATCCAAGCC TGGAGTTTGC AGAAGATACT GTCCTAATAA GCAGGCATTT CTAAACCAAG 60
TATCTAAGCC TAAGCACAGC TTGTCCTGGG TGAAATGTCT GCCACAAAAG ATAGTTTCTC 120
CTAGCTCAGA CTTAACCATT TATAAAGGTT GGTAAAATAC TGGCAGTGAC AACAAATTGA 180
CTTTTTAATT TTTTTATTTG CATTATTCCA ATAAATGAAA ATCTGTCAGA GTTCTAAA 238

SEQ ID NO:7772
SEQUENCE LENGTH:123
SEQUENCE TYPE:nucleic acid
TOPOLOGY:linear
CLONE:HUMGS09022
SEQUENCE DESCRIPTION:
GATCTGCCCC CACTGTCCTG TGAGGACAGC TGAGGCCAAG NAGTGAAAAA CCTATTACTA 60
CTAAGAGAAG GGGTGCAGAG TGTTTACCTG GTGCTCTCAA CAGGACTTAA CATCAACAGG 120
AAA 123

SEQ ID NO:7773
SEQUENCE LENGTH:361
SEQUENCE TYPE:nucleic acid
TOPOLOGY:linear
CLONE:HUMGS09023
SEQUENCE DESCRIPTION:
GATCCAGGTA GAATTTTNAA ACCAATCAAA TGAAAAAAAC AAACAAACAA AAAAGGAAAT 60
GTCATGTGAG GTTAAACCAG TTTGCATTCC CCTAATGTGG AAAAAGTAAG AGGACTACTC 120
AGCACTGTTT GANGATTGCN TCTTCTACAG CTTCTGAGAA TTGTNTTATT TCACTTGCCA 180
AGTGAAGGAC CCCCTCCCCA ACATGCCCCA GCCCACCCCT AAGCATGGTC CCTTGTCACC 240
AGGCAACCAG GAAACTGCTA CTTGTGGNCC TCACCAGAGA CCAGGAGGGT TTGGTTAGCT 300

CACAGGACTT CCCCCACCCC AGANGNTTAG CATCCCATAC TAGACTCNTA CTCAACTTNN 360
N 361

SEQ ID NO:7774
SEQUENCE LENGTH:54
SEQUENCE TYPE:nucleic acid
TOPOLOGY:linear
CLONE:HUMGS09024
SEQUENCE DESCRIPTION:
GATCAAGCGA ACTAATTTTA GGTTGAAATC CGAATAAAAG AACTTTACTG GAAA 54

SEQ ID NO:7775
SEQUENCE LENGTH:188
SEQUENCE TYPE:nucleic acid
TOPOLOGY:linear
CLONE:HUMGS09025
SEQUENCE DESCRIPTION:
GATCTGTATA ATGTACATTC AATATAGAAA GCTTTATATA TTTAAAAGNG TATAGAACAT 60
TTNACAATTA CACTCATCTT TAACATAACA TCTTGACATC CATTTTNAAA TTTTTTTGCA 120
CAAGCTCCTT TNCATTCAAT TTGGTAAAGC CAGTTATACA TACTANTGTG TACTGTGAGC 180
TTTCAGAN 188

SEQ ID NO:7776
SEQUENCE LENGTH:221
SEQUENCE TYPE:nucleic acid
TOPOLOGY:linear
CLONE:HUMGS09026
SEQUENCE DESCRIPTION:
GATCACCTGN AGGTCAGGAA GTTTGAAAAC CAGCCTGGCC AACATGGCAA AACCCTGTCT 60
CTACTAAAAC AAAAATNAGC CAGCATGATG GCAGGCGCCT GTAGTCCNNN CTATTCAGGA 120
GGCTGAGGCA GGAGAATCGC TTGAACTGGG AGAGGGGCAG AGGTTGCAGT GAGCTGAGAC 180
TGCATTCCAG CCTGGGCGAC ACAGCAAGAG TCTGTCTCAA A 221

SEQ ID NO:7777
SEQUENCE LENGTH:200
SEQUENCE TYPE:nucleic acid
TOPOLOGY:linear
CLONE:HUMGS09027
SEQUENCE DESCRIPTION:
GATCGTAGGA ATTGAAGGAG TGTCCCGCCT TGTGGCTGAN AACTGGACAG TGGCAGGGGC 60
TGGAGATGGG TGTNTGTGTG TGTGTGTGTG TGTGTGTGTG TGTGTGCGCG CGCGNCAGTA 120
CAAGACCGAG ATTGAGGNAA ANCATGTCTT CTGGGTGTNA CCATGTTTCC TCTCAATAAA 180
GTNCCCCTGT GACACTCAAA 200

SEQ ID NO:7778

SEQUENCE LENGTH:286
SEQUENCE TYPE:nucleic acid
TOPOLOGY:linear
CLONE:HUMGS09028
SEQUENCE DESCRIPTION:
GATCCTGGTC CCCTCCCTGC TGGACCTGCC ACCCAGAGCT TCCTGNCTAG TCCCACTGGG 60
CTGGCCCACC AGGCCTCTGA CCCAGGCTGC TCTNCGGCCC CTTCCTCCTC CTNTTCCTGC 120
TCCAACTTCT GTCCACCTGG GGACAGTCTG TGCCTGTAGC CTNATGACCC CAACCCAGCC 180
CCAGGCATGG CTAACCCCTG ACTGNTTGCC TCATATTTAA GCTGCTGNTN TGGCCAAGTG 240
NCTAATTTTA ACCCAGACCT NAATAAAGAC ACNTNTTTGT ACCAAA 286

SEQ ID NO:7779
SEQUENCE LENGTH:127
SEQUENCE TYPE:nucleic acid
TOPOLOGY:linear
CLONE:HUMGS09029
SEQUENCE DESCRIPTION:
GATCTGATTT TTCATAAAAA ACATTTGTGA ACCTTCGGCA TAAATGGGTT AAGGTGCCAT 60
CCCTGAAACT GCAATGCAGA TATGTTCAGN TAACTTTNAT TTNTTAATTA AAAATAAATC 120
TTTCAAA 127

SEQ ID NO:7780
SEQUENCE LENGTH:105
SEQUENCE TYPE:nucleic acid
TOPOLOGY:linear
CLONE:HUMGS09030
SEQUENCE DESCRIPTION:
GATCTGANTT TTCATAAAAA ACATAAANTG ACCTTCGGCA TAAATAGGTT AAGAAGCCAT 60
CCCANAAACT NCAATGCAGA TATGTTCAGN TAACTTTTAT TTTTN 105

SEQ ID NO:7781
SEQUENCE LENGTH:321
SEQUENCE TYPE:nucleic acid
TOPOLOGY:linear
CLONE:HUMGS09031
SEQUENCE DESCRIPTION:
GATCGGCGCC GTTACAGACG TGGCTACTAT GGAAGGCGCC GTGGCCCTCC CCGGAATNCT 60
GGTGAGATTG GAGAGATGAA GGATGGAGTC CCAGAGGGAG CACAANTNNN GGGACCGGTT 120
CATCGAAATC CAACTTACCG CCCAAGGTAC CGTAGCAGGG GACCTCCTCG CCCACGACCT 180
GCCCCAGCAG TTGGAGAGGC TGAAGATAAA GAAAATCAGC AAGCCACCAG TGGTCCAAAC 240
CAGCCGTCTG TTCGCCGTGG ATACCGGCGT CCCTACAATT ACCGGCGTCG NCCGNTTCCT 300
CCTAACGGTN CTTCACANGT N 321

SEQ ID NO:7782
SEQUENCE LENGTH:66

SEQUENCE TYPE:nucleic acid
TOPOLOGY:linear
CLONE:HUMGS09032
SEQUENCE DESCRIPTION:
GATCCACTGA GTTAGTTTCA TTGGGGCGGG GGAAAGAACT GGAATTAAAC TTGTTTAATC 60
CTTAAA 66

SEQ ID NO:7783
SEQUENCE LENGTH:131
SEQUENCE TYPE:nucleic acid
TOPOLOGY:linear
CLONE:HUMGS09033
SEQUENCE DESCRIPTION:
GATCCTCCCA GCTGTCGGCC GCGGACCCGG GCCGCGTGTG AGCGGCTTTT GCACCTCCTA 60
TCCCCAGGGT CCGCCGAGAG CCACGATTTT TTTACAGAAA ATNAGCAATA AAGAGATTTT 120
GTACTGTCAA A 131

SEQ ID NO:7784
SEQUENCE LENGTH:143
SEQUENCE TYPE:nucleic acid
TOPOLOGY:linear
CLONE:HUMGS09034
SEQUENCE DESCRIPTION:
GATCACAAAT GCATTTTTCA ATCTTTACAA TATGAAATNT ATTTATAACT TTAGTTCCAA 60
AATGGACTCT GTAATGCATT TGCCCATATT TTTAGTCGTA ATGGATGTGA TTATATTTNA 120
TTGAATTAAA AATCTAACAG AAA 143

SEQ ID NO:7785
SEQUENCE LENGTH:345
SEQUENCE TYPE:nucleic acid
TOPOLOGY:linear
CLONE:HUMGS09035
SEQUENCE DESCRIPTION:
GATCTTAACT TTAATAAGGC AAAACAAAAG CTTCGAGCTG TTGCGTGTGT AAGTCTGTTG 60
TGTGGATGTN CGTGTNTGGT CCCCAGCCCC AGACTGGATT GGAAAAGTGC ATGGTGGGGG 120
CCTCGGGGCT GTCCCCACGC TGTCCCTTTG CCACAAGTCT GTGGGGCAAG AGGCTGCAAT 180
ATTCCGTCCT GGGTGTCTGG GCTGCTAACC TGGCCTGCTC AGGCTTCCCA CCCTGTGCGG 240
GGCACANCCC CAGGAAGGGA CCCTGGACAC GGCTCCCACG TCCAGGNTTA AGGTGGATGC 300
ANTTTCNGNA ACCTCCANTC TTCTGTGTAG CANCTTTAAC CNAGN 345

SEQ ID NO:7786
SEQUENCE LENGTH:167
SEQUENCE TYPE:nucleic acid
TOPOLOGY:linear
CLONE:HUMGS09036

SEQUENCE DESCRIPTION:
GATCAGCTCT CTGTGAAAAT GGACCAATCA TCAGGATGTG GGTGGGGCCA GATTAGGGAA 60
TAGAAGCAGG CTGTCTGAGC CAACAGTGTC ANCATGCTTG GGTCCCCTTN CATGCTGTGG 120
AAACTTTGTT CTTTCGCTCT TCACANTAAA TNTTGCTGCT GCTCAAA 167

SEQ ID NO:7787
SEQUENCE LENGTH:172
SEQUENCE TYPE:nucleic acid
TOPOLOGY:linear
CLONE:HUMGS09037
SEQUENCE DESCRIPTION:
GATCCTGGAC AAAGCTTGGC TGCCGGCTTC ATTTATTCCT GCTGATGGCT GAGAAGCATC 60
TGTTTTCCAT CCCACTTGCC TGTCCCAAGT TTTGTTCCAT TTTTNAAAAA TTTGTTGTAA 120
ACTGCATGTT TTATAAAATA AAAATAAAAT ATCGTTTGTA ATTTATCTCA AA 172

SEQ ID NO:7788
SEQUENCE LENGTH:255
SEQUENCE TYPE:nucleic acid
TOPOLOGY:linear
CLONE:HUMGS09038
SEQUENCE DESCRIPTION:
GATCCGCCCA CCTTNGGCCT CCCAACACAA TTTATATTTT TGANGGTCCT TTTATGTGTA 60
TTGTGCCATA TGCTTCTNAC ATCAGCCCCA ATAAATGCTT TAGGCGTTTT ATATTATCTA 120
CCACTATTAC ATGCTCTCCA TCTCCAGACT ACCCATTTCT GCCATTGCCC TCATAGGGGC 180
CCATTCTGAT AATTCTGGAT ATGTAGAAAA ATAAATCCTG TTTATGTGTT TAAATAAATA 240
GATGTATACT ATAAA 255

SEQ ID NO:7789
SEQUENCE LENGTH:256
SEQUENCE TYPE:nucleic acid
TOPOLOGY:linear
CLONE:HUMGS09039
SEQUENCE DESCRIPTION:
GATCCCTGAC AGCAAAAAGT TTCTTTTCTG AGGCTGCCAT ACTGCCACTG TCCAGGTGGA 60
GACTGAGCAA AGGAAGTCCT GGGCTGTGCC AGCTCCCAGA GCTTCGGAAG AAAGAGCAGC 120
AGCTCTCTCC CTGGGAACCA TCAGAGAATT CTGTTGATGT GTTCTGTGTC TGTCTGTCAC 180
CTGGTCACGA GCTTCTACCA CCTTTGCAAT TGTCACTTAT CTTTCACTCC CTGAATAAAG 240
TATCTATGCA TATAAA 256

SEQ ID NO:7790
SEQUENCE LENGTH:123
SEQUENCE TYPE:nucleic acid
TOPOLOGY:linear
CLONE:HUMGS09040
SEQUENCE DESCRIPTION:

GATCTCCCAC CTTTCGGGCC ATGTTGCCCC CGTGAGCCAA TCCCTCACCT TCTGAGTACA 60
GAGTGTGGAC TCTGGTGCCT CCAGAGGGGC TCAGGTCACA TAAAACTTTG TATATCAACG 120
AAA 123

SEQ ID NO:7791
SEQUENCE LENGTH:310
SEQUENCE TYPE:nucleic acid
TOPOLOGY:linear
CLONE:HUMGS09041
SEQUENCE DESCRIPTION:
GATCTAGCTT CAGAGGAAGT CTACACCCCA TTCCCTTCTG CTTACAATGT ACCCATGATA 60
TGTGTTTAGC NNNGNAATAC TGTAACAGGA CATCACATGG AAAAATCAAA GCAGCTGGCT 120
CACTGTATTT AACTGAAAAG AATGCCTACA GATTGGATAT TTAATAGGGA AAATTAAGGC 180
ACTTTAATAA CANACTTCAT TATGTGAACT TGTTGAATAT TNNCATACAN TATACCTTGT 240
ATATTANTGC CATAGTTTTT NGAAACACTA ATTTAAACAA CANATTTNAG GGGTGCTGGC 300
ATATCCCAGN 310

SEQ ID NO:7792
SEQUENCE LENGTH:61
SEQUENCE TYPE:nucleic acid
TOPOLOGY:linear
CLONE:HUMGS09042
SEQUENCE DESCRIPTION:
GATCCGAAGG CTGCGGGCAG CGTNTNATCC CGTGGTTTAA TAAAGCTGCC GCGCGCTCAA 60
A 61

SEQ ID NO:7793
SEQUENCE LENGTH:310
SEQUENCE TYPE:nucleic acid
TOPOLOGY:linear
CLONE:HUMGS09043
SEQUENCE DESCRIPTION:
GATCTTGGTC TTATTTGCAC AGTTTTTGCG TCTTGTTTGC TTCTTGCATC TGATTAACTA 60
GAATATTTCT NTTTCCCCCT TTTAATTTGT GATGTCACTT GACCCCATTT ATGTGTAGGA 120
GCACTACNCC ATTGGTTTCC AATACTGCAC ACATAAGATA CATACTTGTG TGCAGAAAGT 180
ATCTTCCTCC AGGCTTGTAA TACCCTTCAC ATGGAAGATT AATGAGGGAA ATCTTTATAT 240
TCTGTATAAA ANCAAANGCA AATTTATATA CTAAAATCAT TTGTCTAAAA NTTTAAGTNG 300
TTTTCAAATN 310

SEQ ID NO:7794
SEQUENCE LENGTH:165
SEQUENCE TYPE:nucleic acid
TOPOLOGY:linear
CLONE:HUMGS09044
SEQUENCE DESCRIPTION:

GATCAGTAGA AAATGGACAT CAAGTTTGAA CAGATAAATC ATGGACAGCC TTATTGTGAT 60
TGAAATGCTT GTAGGTTCTG TGCCAATTTT CCACCACTGT GTACTTTGTT GCTATTTAAA 120
ACTGTATCAA CTCTAACGGA AGAATAAATT ATTTGTGATT TTAAA 165

SEQ ID NO:7795
SEQUENCE LENGTH:149
SEQUENCE TYPE:nucleic acid
TOPOLOGY:linear
CLONE:HUMGS09045
SEQUENCE DESCRIPTION:
GATCCGAGAG TNAAGGAAGT TCCCTGTNTT CCCCGTCCTT TTCCACCAGT NTCGCCTCTG 60
GCCTTCTCTG GCCACTCCTG GGAGGGACTG CCTCACCACC CCTGTCCCGC TGCCAGAAAT 120
ACACCCACAA TAAAAACCTG AAAACCAAA 149

SEQ ID NO:7796
SEQUENCE LENGTH:98
SEQUENCE TYPE:nucleic acid
TOPOLOGY:linear
CLONE:HUMGS09046
SEQUENCE DESCRIPTION:
GATCCAAACT AACTGCCCAA GCACTACACA GCTGGACCCT GNTTCACCNT AACCCGCANC 60
TAGCAATCAA TACAGCTTCA TTATCTGAGT TGCATAAA 98

SEQ ID NO:7797
SEQUENCE LENGTH:265
SEQUENCE TYPE:nucleic acid
TOPOLOGY:linear
CLONE:HUMGS09047
SEQUENCE DESCRIPTION:
GATCTNTAGC CTCACCCTGN TCTCCTTCCT CCTACTGGCT GCTCAGGTGC TCCTGGTGGA 60
GGGGAAAAAA AAAGTGAAGA ATGGACTTCA CAGCAAAGTG GTCTCAGNNC AAAAGGNCAC 120
TCTGGGCAAC ACCCAGATTA AGCAGAAAAG CAGGCCCGGG ANCAANGGCA AGTTTGTCAC 180
CAAAGACCAA GCCAACTGCA GATGGGCTGC TACTGAGCAG GAGGAGGGCA TCTTTCTCAN 240
GGGNNGGGTG CACTCAATTG GNNNN 265

SEQ ID NO:7798
SEQUENCE LENGTH:146
SEQUENCE TYPE:nucleic acid
TOPOLOGY:linear
CLONE:HUMGS09048
SEQUENCE DESCRIPTION:
GATCTTACCC GTGCNTGGNC CCCTCCCCTC ANAGCCCATG GTAACGAACC CCTAGAAAGG 60
AGAGAACGGG CGTCAGGGGT GCACAGTCCA CAGCTGAAGA GCAAGGTTTN GTGGCAGCAC 120
GGNCCGGCCC CTCACCCTCT NTNCCN 146

SEQ ID NO:7799
SEQUENCE LENGTH:317
SEQUENCE TYPE:nucleic acid
TOPOLOGY:linear
CLONE:HUMGS09049
SEQUENCE DESCRIPTION:
GATCTCCGGA CCTCGGCTGC AGAGGCCATC GCAGCTTTTN AAAAGTNAAG TGGTTAATTC 60
CCATTGGTGT CTTTNCTTAT AGCATTTTCN TCTAACCTAT AACAAGGGGG CATTACATTT 120
ANCTTTAGAA CATGTGAATA GGAGGTTTNC TCATGACTTA CCATTCCAGC TGNATGGGAA 180
AGCAAAGCAG AAAACAGTGC CCCAAATGGA AAANAGNTAC TCACACAGAN CAAAACAGTT 240
CTNGGTCTTG TCCTTGGTCT TGTCAAACCT TGCCTGATGC TCTTTCTAAN GTCNAAATAT 300
GANTGATAAG CCNGGCN 317

SEQ ID NO:7800
SEQUENCE LENGTH:372
SEQUENCE TYPE:nucleic acid
TOPOLOGY:linear
CLONE:HUMGS09050
SEQUENCE DESCRIPTION:
GATCTGGTTA AGTTGTGTAG TAAAGCATTA GGAGGGTCAT TCTTGTCACA AAAGTGCCAC 60
TAAAACAGCC TCAGGAGAAT AAATGACTTG CTTTTCTAAA TCTCAGGTTT ATCTGGGCTC 120
TATCATATAG ACAGGCTTCT GATAGTTTGC AACTGTAAGC AGAAACCTAC ATATAGTTAA 180
NATCCTGGNC TTTCTTGGTA AACAGATTTT AANTTTCTGA TATAAANCAN GCCNCAGGAG 240
AATTCGGGGA TTTNAGGTTC NCNGAATAGC CTATATATGG TGCATCGGNT AGGTCNTTAT 300
TGATTTTTTG ACCCTTTTCG GCTTTACCTN ATGGGAAGAC CCNGTTCNTT TTTAAATNAT 360
CCNGGTTTTT GN 372

SEQ ID NO:7801
SEQUENCE LENGTH:356
SEQUENCE TYPE:nucleic acid
TOPOLOGY:linear
CLONE:HUMGS09051
SEQUENCE DESCRIPTION:
GATCAACCTC TGAATTTTAC ATCATGAATG TAATCACCAC TGGAGCTTCA CTGTTACTAA 60
ATTATTAATT TTTTGCCTCC AGTGTTCTAT CTCTNAGGCT GAGCATTATA AGAAAATNAC 120
CTCTGCTCCT TTTCATTGCA GAAAATTGCC AGGGGCTTAT TTCAGAACAA CTTCCACTTA 180
CTTTCCACTG GCTCTCAAAC TCTCTAACTT ATAAGTNTTG TGAACCCCCA CCCAGGCAGT 240
ATCCATGAAA GCACAAGTGA CTAGTCCTAT GATGTACAAA GCCTGTATCT CTGTGATGAT 300
TTCTGTGCTC TTCACTCTTT GCAATTGCTA AATAAAGCAG ATTTTATAAA TACAAA 356

SEQ ID NO:7802
SEQUENCE LENGTH:359
SEQUENCE TYPE:nucleic acid
TOPOLOGY:linear
CLONE:HUMGS09052

SEQUENCE DESCRIPTION:
GATCAAGCAG ATTCCACGAA TCCTCGGCCC AGGTTTAAAT AAGGCAGGAA AGTTCCCTTC 60
CCTGCTCACA CACAACGAAA ACATGGTGGC CAAAGTGGAT GAGGTGAAGT CCACAATCAA 120
GTTCCAAATG AAGAAGGTGT TATGTCTGGC TGTAGCTGTT GGTCACGTGA AGATGCCNGA 180
CGATGAGCTT GTGTATAACA TTCACCTGGC TGTCAACTTC TTGGTGTCAT TGCTCAAGAA 240
AANCTGGCAG ANTGTTCCGG GGCCTTATNT TNTCAAGAGC ACCNTGGGGN AAAGCCCCCA 300
GNGGCCTNTT NTTAAGGGCA CCGTTTNGAN NTAAAATTTC TNGTTANCCC AGTTCNAAA 359

SEQ ID NO:7803
SEQUENCE LENGTH:134
SEQUENCE TYPE:nucleic acid
TOPOLOGY:linear
CLONE:HUMGS09053
SEQUENCE DESCRIPTION:
GATCCGGCCA GTTCATTGAA CAAACCTTTC CTTTTTCTAA AGTTCCAGAA GCCTTCCTGA 60
AGGTGGAAAG AGGACACGCA CGAGGAAAGA CTGTAATTAA TGTTGTTTAA ATAAAAATGC 120
AGTTTAGTGA TAAA 134

SEQ ID NO:7804
SEQUENCE LENGTH:62
SEQUENCE TYPE:nucleic acid
TOPOLOGY:linear
CLONE:HUMGS09054
SEQUENCE DESCRIPTION:
GATCTACTGA CATTAAGCAC TTTGTACAGT ACAAAATAAA GTCTACATTT GTTTAAAACA 60
AA 62

SEQ ID NO:7805
SEQUENCE LENGTH:120
SEQUENCE TYPE:nucleic acid
TOPOLOGY:linear
CLONE:HUMGS09055
SEQUENCE DESCRIPTION:
GATCTGGATG ATGGTTATGT AGCTGCATAT ATTTGTCAAC ATTCCTCATG CTGTATGTTT 60
AGATTTGTGA TTTTACTATA TGTAAATTAA TTCCTTAATA AAATACTATT ATAAAATAAA 120

SEQ ID NO:7806
SEQUENCE LENGTH:209
SEQUENCE TYPE:nucleic acid
TOPOLOGY:linear
CLONE:HUMGS09056
SEQUENCE DESCRIPTION:
GATCTAATCT TCTGTGCTCT GTGTATTGGC GACGAGCAGG GTGAGAATAA GTGCGTGCCC 60
AACAGCAATN AGAGATACTA CGGCTACACT GGGGCTTTCC GGTGCCTGGC TGAGAATGCT 120
GGAGACGTTG CATTTGTGAA AGATGTCACT GTCTTGCAGA ACACTGATGC CCTCCTGGAA 180

GCCTGTGAAT TCCTCAGGAA GTAAAACCN 209

SEQ ID NO:7807
SEQUENCE LENGTH:59
SEQUENCE TYPE:nucleic acid
TOPOLOGY:linear
CLONE:HUMGS09057
SEQUENCE DESCRIPTION:
GATCTTAATT TTAAAAATTA AAACTTGGNG TTTTTAAAT AAAAACCTTT TGTCTTAAA 59

SEQ ID NO:7808
SEQUENCE LENGTH:100
SEQUENCE TYPE:nucleic acid
TOPOLOGY:linear
CLONE:HUMGS09058
SEQUENCE DESCRIPTION:
GATCAAAGTA ATTGCTTAAA TGTCTTGTGG AATNAGCTGT CACTTTATAG TTTATGTATT 60
GTGGTGGGTA CCTTAATAAT AAAAAGATTT TTAATGTAAA 100

SEQ ID NO:7809
SEQUENCE LENGTH:118
SEQUENCE TYPE:nucleic acid
TOPOLOGY:linear
CLONE:HUMGS09059
SEQUENCE DESCRIPTION:
GATCTGTTTG ATGGTTACAT CAGTGTATAT TTGTCAAAAT TCCTCAAACT GTATGCTTAA 60
GGCTTTGTGC ATTGTATTGC ATGTAAATNA TACTTCAATA AAGTACCAAT AGCATAAA 118

SEQ ID NO:7810
SEQUENCE LENGTH:183
SEQUENCE TYPE:nucleic acid
TOPOLOGY:linear
CLONE:HUMGS09060
SEQUENCE DESCRIPTION:
GATCCATAAC CTCTTTAGAG ACCGGCTCCT GGATGCAGAA TCCTATGAGG TTCTGGCCAC 60
ACCTGTTTCT TGTCCNTCAG GGGCTGACAC CCATGTNCCA TCCCTCTAAA CCCCTGTCCC 120
TAAACCTACC CCATCTNATG TAACTCTGGG GTTCCTTGGT CATTAGAGGG TTTTNAGGTC 180
AAA 183

SEQ ID NO:7811
SEQUENCE LENGTH:175
SEQUENCE TYPE:nucleic acid
TOPOLOGY:linear
CLONE:HUMGS09061
SEQUENCE DESCRIPTION:

GATCTGAATG GTTTTAATAA GTGTCTGGNA ATTCCCCTGC TTGCACTTGT NTNTCTCGCC 60
CACCACCACG TAAGACGTGC CTGCTTCCCC TTCTGCCATG ATTGTAAGTT TCCTGAGGCG 120
TCCCCAGCCA TGCAGAACTG TGAGTCAATG AAACCTCTTT TNTTTATAAA TTAAA 175

SEQ ID NO:7812
SEQUENCE LENGTH:329
SEQUENCE TYPE:nucleic acid
TOPOLOGY:linear
CLONE:HUMGS09062
SEQUENCE DESCRIPTION:
GATCCCCCAT TCCCCACCCT GGGAGATGAG GGGGTCCCCA TGTGCTTTTC CAGTTCTTCT 60
GGAATTGGGG GACCNCCGCC AAAGACTGAG CCCCCTGTNT CCTCCATCAT TTGGTTTCCT 120
CTTGGCTTTG GGGATACTTC TAAATTTTGG GAGCTCCTCC ATCTCCAATG GCTGGGATTT 180
GTGGCAGGGA TTCCACTCAG AACCTCTCTG GAATTTGTGC CTGATGTGCC TTCCACTGGA 240
TTTTGGGGTT CCCAGCACCC CATGTGGATT TTGGGGGGTC CCTTTTGTGT CTCCCCCGCC 300
ATTCAAGGAC TCCTNTCTTT TNTTCAANN 329

SEQ ID NO:7813
SEQUENCE LENGTH:326
SEQUENCE TYPE:nucleic acid
TOPOLOGY:linear
CLONE:HUMGS09063
SEQUENCE DESCRIPTION:
GATCTGCCAG CCCTACATCC GGCCGGGGCC CAGGGAGGGG AGCAAGGAGA AGGCAGGTGC 60
GCGCANAAGC GGGCCCTGGA GGAAGAGGAG GGTGGCACGG AGGTCCTGTC CAAGAACAAG 120
CAAAAGAAGC AGCTGAGGAA CCCCCACAAG ACCTTCGACC CCTCTTTGAA GCCAAAATAT 180
GCAAAGTNTG ACCAGTGTGG AAACCCAAAG GGCAACAGAT GTNTGTTCAG CCTGTGCCGN 240
GGCTGCTGCA NGANGCGNGC CTTCAAAGNG ACTGCAGACT GNCCAGGTNA CGGTTTGCTT 300
TTTAAAACCA ATTTNGGAGA AGTTTN 326

SEQ ID NO:7814
SEQUENCE LENGTH:20
SEQUENCE TYPE:nucleic acid
TOPOLOGY:linear
CLONE:HUMGS09064
SEQUENCE DESCRIPTION:
GATCGGAATG NAAATCTAAA 20

SEQ ID NO:7815
SEQUENCE LENGTH:248
SEQUENCE TYPE:nucleic acid
TOPOLOGY:linear
CLONE:HUMGS09065
SEQUENCE DESCRIPTION:
GATCAATGTT GAAAGTGTTG TTATATGGCA AGTNTTTAAC ACATTCACAG TGTTTGTTTG 60

ATTTCAACTG TGAATTGTCT TACAGTTTTT TCAAACCTAG TTGTTTCTAT GGACACCTGC 120
TCTGAATTGT ACATTGACTN CATTACTAAA GAACAAAAAT GTTCATTTTT GTCCCAGTAA 180
ATTGAGACTG CTTGTACACT TTCAGAAAAA TATGTGAATT TATAAAGNTT TTGTAGATAC 240
TTGTCAAA 248

SEQ ID NO:7816
SEQUENCE LENGTH:87
SEQUENCE TYPE:nucleic acid
TOPOLOGY:linear
CLONE:HUMGS09066
SEQUENCE DESCRIPTION:
GATCCCCGGA TTTNACACTC CTTCTGTTTT GTTGCCGTTT ATTTTTGTAC TCAAATCTCT 60
ACATGGAGAT AAATNATTTA AACCAAA 87

SEQ ID NO:7817
SEQUENCE LENGTH:267
SEQUENCE TYPE:nucleic acid
TOPOLOGY:linear
CLONE:HUMGS09067
SEQUENCE DESCRIPTION:
GATCCACCTG NCCCTGACCT CCGACAGTGC TGGGATTACA GGCATAGCCA CCGTGCCTGA 60
CCAGGGCTCT TTTAGCAAGG AAAACGTGAG GAATGAATGG CTGTTGGTGT GCAACAAATC 120
ATACTTGCTA CATGTTGTGA ACCTGAAGTT ATTTGTTAGT CTGTATGAAG AATGTACCCC 180
AGAGATGCAC CCTGTATCTG CCTTATGTCT CTTACAATGG AGGTTCTCTC CTGTTATATT 240
GCTGAATAAA CTATGTGAAC TGCTAAA 267

SEQ ID NO:7818
SEQUENCE LENGTH:22
SEQUENCE TYPE:nucleic acid
TOPOLOGY:linear
CLONE:HUMGS09068
SEQUENCE DESCRIPTION:
GATCTTCAAG CAAGAAAATA AA 22

SEQ ID NO:7819
SEQUENCE LENGTH:90
SEQUENCE TYPE:nucleic acid
TOPOLOGY:linear
CLONE:HUMGS09069
SEQUENCE DESCRIPTION:
GATCATAGCT GAAAATTAAT GATACTGTCA ATTTGAGATA GCAGAAGTTT CACACATCAA 60
AGTAAAAGAT TTGCATATCA TTATACTAAA 90

SEQ ID NO:7820
SEQUENCE LENGTH:25

SEQUENCE TYPE:nucleic acid
TOPOLOGY:linear
CLONE:HUMGS09070
SEQUENCE DESCRIPTION:
GATCATTAAA AAATTAATAA AGAAA 25

SEQ ID NO:7821
SEQUENCE LENGTH:33
SEQUENCE TYPE:nucleic acid
TOPOLOGY:linear
CLONE:HUMGS09071
SEQUENCE DESCRIPTION:
GATCCCAATG AAACGAATTC TGTCTCCCTG AAA 33

SEQ ID NO:7822
SEQUENCE LENGTH:23
SEQUENCE TYPE:nucleic acid
TOPOLOGY:linear
CLONE:HUMGS09072
SEQUENCE DESCRIPTION:
GATCTTCAAG CAAGTTAANT AAA 23

SEQ ID NO:7823
SEQUENCE LENGTH:75
SEQUENCE TYPE:nucleic acid
TOPOLOGY:linear
CLONE:HUMGS09073
SEQUENCE DESCRIPTION:
GATCTTCATT TAGCTCCTTT ACTGGGATTT ATTGGATGCT NGTAAAAAAA TAAAATTTAC 60
ACTGTNTATG CGAAA 75

SEQ ID NO:7824
SEQUENCE LENGTH:284
SEQUENCE TYPE:nucleic acid
TOPOLOGY:linear
CLONE:HUMGS09074
SEQUENCE DESCRIPTION:
GATCAAGAAA GGGCTGTGGC TTGTATCAAA GGGTGTTTAA AAGGATTTAC TATCAGATTT 60
GGCCTTGTTT TCTGTGATTT TGGNNAGCGT TCAAGGAAGC CAGGCTTTGG TCTGGATTGG 120
ATGTTCTCAG GTCATGAATA TAATTCTCTG GGAACTCCCA AAGTTCTCAT CTACAAAGCA 180
GAAGTTAATT GGAGCTGAAA ACTAATCAGT AAAGCCACAG CAGCCAGTCA TATGGGGGAG 240
AAGAGGGGAA TGTTTGATGG TTTTTGTGGT TTAGAGGTGA GTTN 284

SEQ ID NO:7825
SEQUENCE LENGTH:21

SEQUENCE TYPE:nucleic acid
TOPOLOGY:linear
CLONE:HUMGS09075
SEQUENCE DESCRIPTION:
GATCCTTTGT TAACAAATAA A 21

SEQ ID NO:7826
SEQUENCE LENGTH:159
SEQUENCE TYPE:nucleic acid
TOPOLOGY:linear
CLONE:HUMGS09076
SEQUENCE DESCRIPTION:
GATCATATTT ATTTTAACTA TTTTTTTAGA AAATTATTAA TTTTAATTGT ATTTTTGTAT 60
TTAAAAAGCT TCTTCACTTG TTTTCCCTAA NNNTTCANAT TGCTGCCCAA AAGTATGACT 120
GTGGAGGAAA AAAAANTACT TNAAAANTCC ACACTTTTN 159

SEQ ID NO:7827
SEQUENCE LENGTH:328
SEQUENCE TYPE:nucleic acid
TOPOLOGY:linear
CLONE:HUMGS09077
SEQUENCE DESCRIPTION:
GATCTGTGCA GGGTATTAAC GTGTCAGGGC TGAGTGTTCT GGGATTTCTC TAGAGGCTGG 60
CAAGAACCAG TTGTTTTGTC TTGCGGGTCT GTCAGGGTTG GAAAGTCCAA GCCGTAGACC 120
CAGTTTCCTT TCTNAGCTGA TGTCTTTGGC CAGAACACCG TGGGCTGTTA CTTNCNTTNA 180
GTTGGAAGCG GTTTGCATTT ACGCCTGTAA ATGTATTCAN TCTTAANTTA TGTAAGGTNT 240
TTNTTGTACG CAATTCTCGG TTCTTTGANG NGATGGCAAC AAATTTTGGG NTTCTACTGT 300
TATGTTGGGG NNCATTNGGG CCCANGNN 328

SEQ ID NO:7828
SEQUENCE LENGTH:108
SEQUENCE TYPE:nucleic acid
TOPOLOGY:linear
CLONE:HUMGS09078
SEQUENCE DESCRIPTION:
GATCTCCTGA CCTCGTCATC CGCCCGTNTC GGCCTCCCAT AGTGCTGGGN TTACAGGCAT 60
GAGCCACCAC GCCCGGCTGT TTTATTTCTT ATAACTGTAC AGGANNNN 108

SEQ ID NO:7829
SEQUENCE LENGTH:277
SEQUENCE TYPE:nucleic acid
TOPOLOGY:linear
CLONE:HUMGS09079
SEQUENCE DESCRIPTION:
GATCATGAAG CCATCAGCTC CTCTGGGGCC AGCTANAGGA CAACAGAACT NTCACCAAAG 60

GACCAGACAC AGTGAGCACC ATGGGACAGT NTCGGTCAGC CAACGCAGAG GATGCTCAGG 120
AATTCANTGA TGTGGNGAGG GCCATTGAGA CCCTCATCAA GAACTTTCAC CAGTACTCCG 180
TGGAGGGTGG GNAGGNGACG CTGACCCCTT CTAAGCTACG GGACCNGGGT CACCCAGCAG 240
CTGCCCCATN TTNATGCCGA GCAACTTNTN GNCTGGN 277

SEQ ID NO:7830
SEQUENCE LENGTH:123
SEQUENCE TYPE:nucleic acid
TOPOLOGY:linear
CLONE:HUMGS09080
SEQUENCE DESCRIPTION:
GATCAGACCA AACAGTGCTG TTTCCCGGGG AGGAAACACT TTTTTAATTA CCCTTTTGCA 60
GGCTCCCACC TTTAATCTGT TTTATACCTT GCTTATTAAA TGAGCGACTT AAAATNATTG 120
AAA 123

SEQ ID NO:7831
SEQUENCE LENGTH:338
SEQUENCE TYPE:nucleic acid
TOPOLOGY:linear
CLONE:HUMGS09081
SEQUENCE DESCRIPTION:
GATCCAGAAA TGGAGACTGG ACCTTCCAGA CCCTGGTGAA TGCTGGAAAC AGTTCCTCGG 60
AGTGGAGAGG TTTACACCTG CCAAGTGGAG CACCCAAGCC TGACGAGCCC TCTNACAGTG 120
GAATGGAGAG CACGGTCTGA ATCTNCACAG AGCAAGATGC TGAGTGGAGT CGGGGGCTTC 180
GTGCTGGGCC TGCTCTTCCT TGGGGCCGGG CTGTTCATCT ACTTCAGGAA TCAGAAAGGA 240
CACTCTGGAC TTCAGCCAAC AGGATTCCTG AGCTGAAGTG AAGATGNCCA CATTCAAGGA 300
AGAACCTTCT GCCCCAGCTT TGCAGGATGA ANCACTTN 338

SEQ ID NO:7832
SEQUENCE LENGTH:309
SEQUENCE TYPE:nucleic acid
TOPOLOGY:linear
CLONE:HUMGS09082
SEQUENCE DESCRIPTION:
GATCTAATCT CAAATACAGT GCTTAGTAAG GGAACTCTTG TGCCTTTGGG GATTTGGGGA 60
TTAAAACGAA TCTTTTAAAT ATATTTCCAA ATATTAAAAT ATAAAAGGAT ATTTCCTCTG 120
ATGTTAAAAG TGAGTATGGG GGTGGCACAT TATATGGAAA GTNCCATGTT TGCTTAANTG 180
CTATGTAAAA TAACTTGGCA TATTANTTGA CTTTGANTGT GAAANTCATA TGCCCTTNGA 240
NTATGGNTGC CTTAGCTCAT TTGACANCTG GNAGANTACT TGTNTATCTN GTTTNAGACA 300
CATTGANTN 309

SEQ ID NO:7833
SEQUENCE LENGTH:233
SEQUENCE TYPE:nucleic acid
TOPOLOGY:linear

CLONE:HUMGS09083
SEQUENCE DESCRIPTION:
GATCCAAAGT GGCAGGAGGT CTGTGTTGTC ATGGNGAACT GGAGTTTCTC TTGTAAGAGT 60
TCCCTCATCT GAAATCATGT ATCTNTCTCA CAAATACAAG CATAAGTAGA AGATTTGTTG 120
AAGACATAGA ACCCTTATAA AGAATTATTA ACCTTTATAA NCATTTAANG TCTTGTNAGC 180
ACCTGGGAAT TAGTATAATN CCANTGTTAA TNTTTTNNAT TTACATTTTG TNN 233

SEQ ID NO:7834
SEQUENCE LENGTH:99
SEQUENCE TYPE:nucleic acid
TOPOLOGY:linear
CLONE:HUMGS09084
SEQUENCE DESCRIPTION:
GATCGGAATT TNACAGAATT GGGACTGTGG AACCNNGGGT CACTTGAATT TCCTATATTT 60
TGTATGGCCA AATCAGGAAC CAGACAGTCT CCATAGTCN 99

SEQ ID NO:7835
SEQUENCE LENGTH:288
SEQUENCE TYPE:nucleic acid
TOPOLOGY:linear
CLONE:HUMGS09085
SEQUENCE DESCRIPTION:
GATCAGCCGA TGACAGAGAT TGTAAGCCGT GTGTCGAAGC GAAACTGGNG CCGCCACGTG 60
CGGGCNCTGG TGCTTGACCT GTGCTGTAAC GACGAGAGCG GCGAGGATGT CGNGGGGCNA 120
NAAAANCGGT ACACCATCCG CTGACCCCGT CTGCTCCTCT AGGCTGGCCC CTTGTCCACC 180
CCTCTCCACA CCCCTTCCAG CCCAGGGTTC CCATTTGGCT TCTGGCAGTG GCCCAACTAG 240
CCAAGTCTGG TGTTCCCTCA TNATCCCNCT TACCTGAACC CNTNTTGN 288

SEQ ID NO:7836
SEQUENCE LENGTH:32
SEQUENCE TYPE:nucleic acid
TOPOLOGY:linear
CLONE:HUMGS09086
SEQUENCE DESCRIPTION:
GATCTAAATA AAATGGACNG TTCTACCTTA AA 32

SEQ ID NO:7837
SEQUENCE LENGTH:152
SEQUENCE TYPE:nucleic acid
TOPOLOGY:linear
CLONE:HUMGS09087
SEQUENCE DESCRIPTION:
GATCTGAAAC AGATGAAAGT TATAATAGTT TACATACCTT ACCTTAAAGT ATGGTTTTTA 60
AATACTTAAG ATGTGATATG ACATATAGTG TGTATATATA TCTNATNCTA NNGGAAAAAG 120
GGACATTAAA AAAGCTTATG TTAAAAGATA AA 152

SEQ ID NO:7838
SEQUENCE LENGTH:108
SEQUENCE TYPE:nucleic acid
STRANDEDNESS:single
TOPOLOGY:linear
SEQUENCE DESCRIPTION:
GATCGTTCTT CATGGGGGTA AGAAAAGCTG GTCTGGAGTT GCTGAATGTT GCATTAATTG 60
TCCTGTTTGC TTGTAGTTGA ATAAAAATAG AAACCTGAAT GAAGGAAA 108

SEQ ID NO:7839
SEQUENCE LENGTH:30
SEQUENCE TYPE:nucleic acid
STRANDEDNESS:single
TOPOLOGY:linear
SEQUENCE DESCRIPTION:
TGAAAATTTA TTACTACAGT GTTTTCACCA 30

SEQ ID NO:7840
SEQUENCE LENGTH:20
SEQUENCE TYPE:nucleic acid
STRANDEDNESS:single
TOPOLOGY:linear
SEQUENCE DESCRIPTION:
TAATACGACT CACTATAGGG 20

SEQ ID NO:7841
SEQUENCE LENGTH:25
SEQUENCE TYPE:nucleic acid
STRANDEDNESS:single
TOPOLOGY:linear
SEQUENCE DESCRIPTION:
CTTCTTTCTG TAGCCAGGTA ACTCT 25

SEQ ID NO:7842
SEQUENCE LENGTH:25
SEQUENCE TYPE:nucleic acid
STRANDEDNESS:single
TOPOLOGY:linear
SEQUENCE DESCRIPTION:
TTTTCGGCGC TCCCATTTAT TCCTT 25

SEQ ID NO:7843
SEQUENCE LENGTH:35
SEQUENCE TYPE:nucleic acid
STRANDEDNESS:single
TOPOLOGY:linear
SEQUENCE DESCRIPTION:
CACGAATTCA CTATCGATTC TGGAACCTTC AGAGG- NH2 35

SEQ ID NO:7844
SEQUENCE LENGTH:37
SEQUENCE TYPE:nucleic acid
STRANDEDNESS:single
TOPOLOGY:linear
SEQUENCE DESCRIPTION:
CTCGCTCGCC CATCCTTATA CAGGCTCAGT TTTGTCT 37

SEQ ID NO:7845
SEQUENCE LENGTH:37
SEQUENCE TYPE:nucleic acid
STRANDEDNESS:single
TOPOLOGY:linear
SEQUENCE DESCRIPTION:
CTCGCTCGCC CATGTATAGG GACAGCATTT CTGAGAG 37

SEQ ID NO:7846
SEQUENCE LENGTH:38
SEQUENCE TYPE:nucleic acid
STRANDEDNESS:single
TOPOLOGY:linear
SEQUENCE DESCRIPTION:
CTGGTTCGGC CCACCTCTGA AGGTTCCAGA ATCGATAG 38

SEQ ID NO:7847
SEQUENCE LENGTH:22
SEQUENCE TYPE:nucleic acid
STRANDEDNESS:single
TOPOLOGY:linear
SEQUENCE DESCRIPTION:
CCAGGGTTTT CCCAGTCACG AC 22

SEQ ID NO:7848
SEQUENCE LENGTH:22
SEQUENCE TYPE:nucleic acid
STRANDEDNESS:single
TOPOLOGY:linear
SEQUENCE DESCRIPTION:
TCACACAGGA AACAGCTATG AC 22

## Claims

**1.** A purified single-stranded DNA, a purified single-stranded DNA complementary thereto, or a purified double-stranded DNA consisting of said single strands, containing all or a portion of a single-stranded DNA or a single-stranded DNA complementary thereto comprising any of the base sequences listed under SEQ ID NO 1-7837 and hybridizing specifically to a particular site of human genomic DNA, human cDNA or human mRNA.

2. A DNA probe consisting of a purified single-stranded DNA , a purified single-stranded DNA complementary thereto, or a purified double-stranded DNA consisting of said single strands, containing all or a portion of a single-stranded DNA or a single-stranded DNA complementary thereto comprising any of the base sequences listed under SEQ ID NO 1-7837 and hybridizing specifically to a particular site of human genomic DNA, human cDNA or human mRNA.

3. A DNA primer consisting of a purified single-stranded DNA, a purified single-stranded DNA complementary thereto, or a purified double-stranded DNA consisting of said single strands, containing all or a portion of a single-stranded DNA or a single-stranded DNA complementary thereto comprising any of the base sequences listed under SEQ ID NO 1-7837 and hybridizing specifically to a particular site of human genomic DNA, human cDNA or human mRNA.

4. A purified single-stranded DNA, a purified single-stranded DNA complementary thereto, or a purified double-stranded DNA consisting of said single strands, containing all or a portion of a single-stranded DNA or a single-stranded DNA complementary thereto, wherein said single-stranded DNA is complementary to a human mRNA containing any of the base sequences listed under SEQ ID NO 1-7837 (wherein T is read as U) or any portion thereof at its 3' region, and hybridizing specifically to a particular site of human genomic DNA, human cDNA or human mRNA.

5. A DNA probe consisting of a purified single-stranded DNA, a purified single-stranded DNA complementary thereto, or a purified double-stranded DNA consisting of said single strands, containing all or a portion of a single-stranded DNA or a single-stranded DNA complementary thereto, wherein said single-stranded DNA is complementary to a human mRNA containing any of the base sequences listed under SEQ ID NO 1-7837 (wherein T is read as U) or any portion thereof at its 3' region, and hybridizing specifically to a particular site of human genomic DNA, human cDNA or human mRNA.

6. A DNA primer consisting of a purified single-stranded DNA, a purified single-stranded DNA complementary thereto, or a purified double-stranded DNA consisting of said single strands, containing all or a portion of a single-stranded DNA or a single-stranded DNA complementary thereto, wherein said single-stranded DNA is complementary to a human mRNA containing any of the base sequences listed under SEQ ID NO 1-7837 (wherein T is read as U) or any portion thereof at its 3' region, and hybridizing specifically to a particular site of human genomic DNA, human cDNA or human mRNA.

Fig. 1

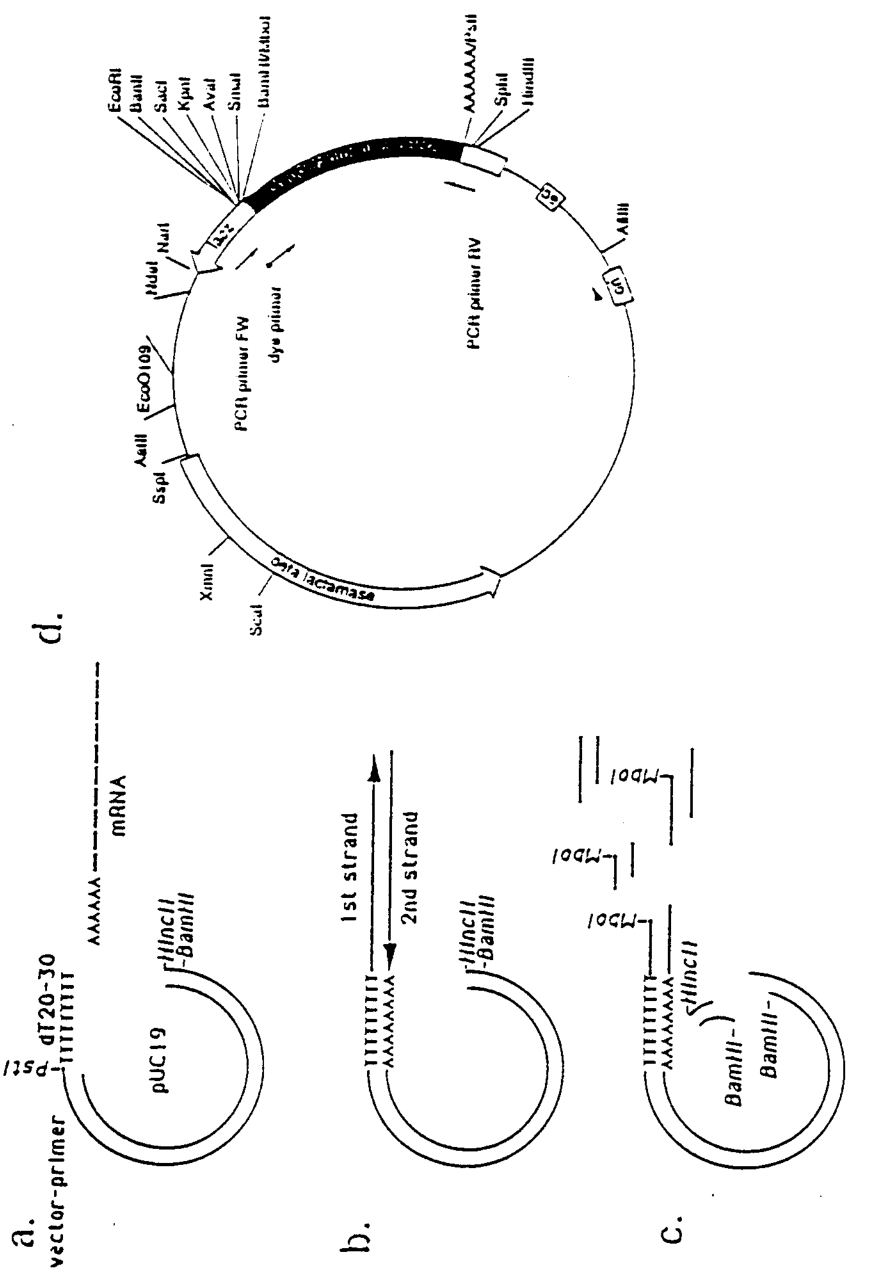
a.
vector-primer
PstI
dT20-30
TTTTTTT
AAAAAA
mRNA
pUC19
HincII
BamHI
b.
1st strand
2nd strand
c.
MboI
BamHI
d.
EcoRI
SacI
KpnI
AvaI
SmaI
NarI
NdeI
EcoO109
AatII
SspI
XmnI
ScaI
PCR primer FW
dye primer
PCR primer RV
SphI
HindIII
AflIII

Fig. 2

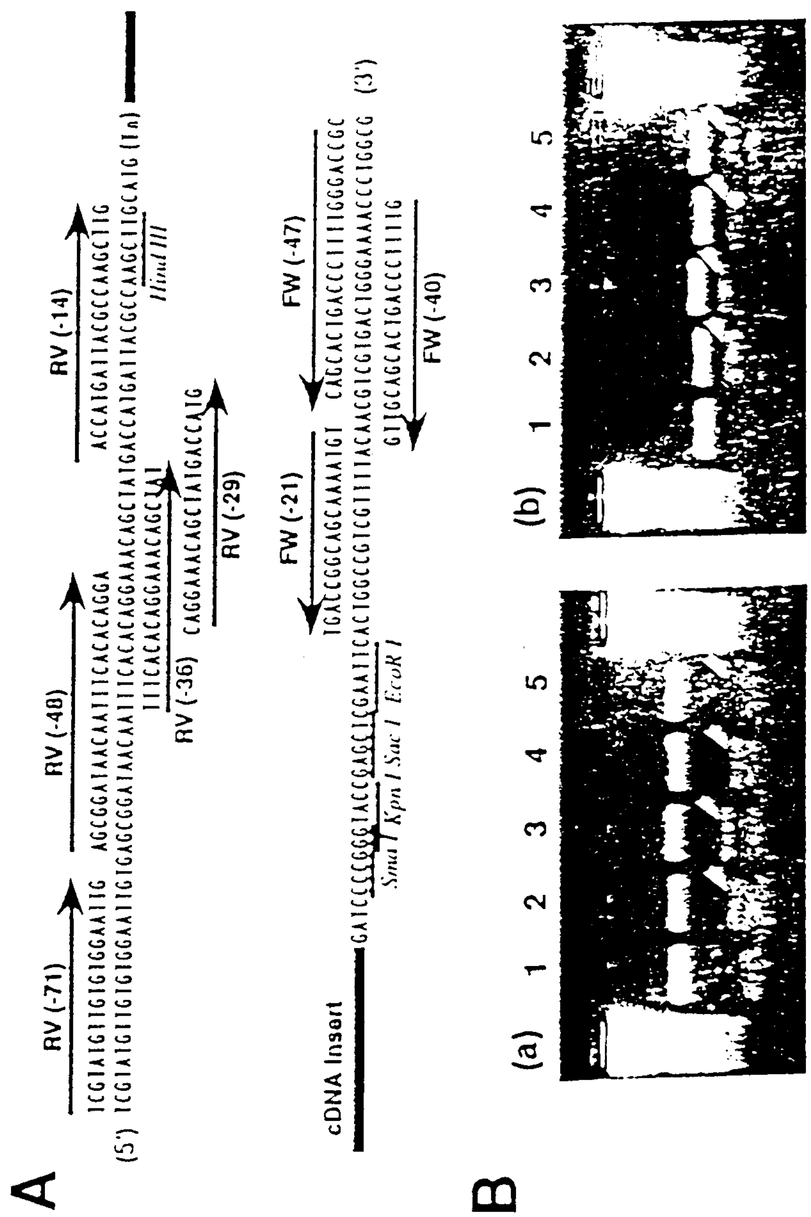
A
B
RV (-71)
RV (-48)
RV (-36)
RV (-29)
RV (-14)
FW (-21)
FW (-47)
FW (-40)
cDNA Insert
(5')
(3')
HindIII
SmaI
KpnI
SacI
EcoRI
(a)
(b)
1
2
3
4
5

Fig. 3

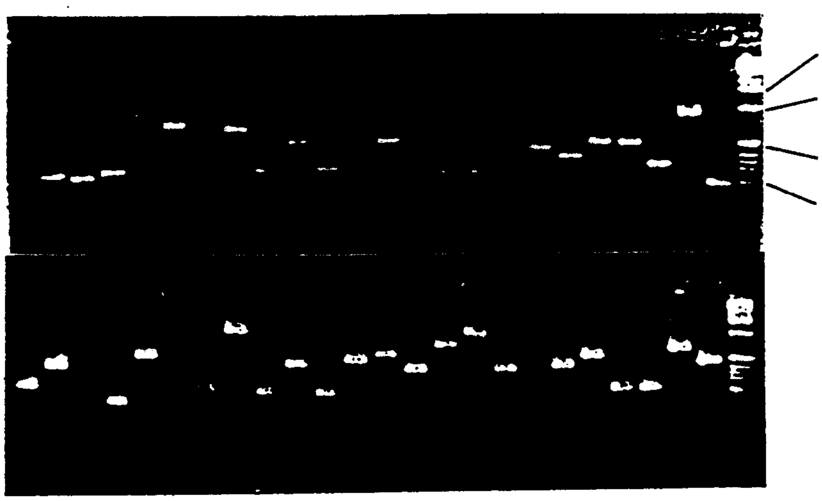

Fig. 4

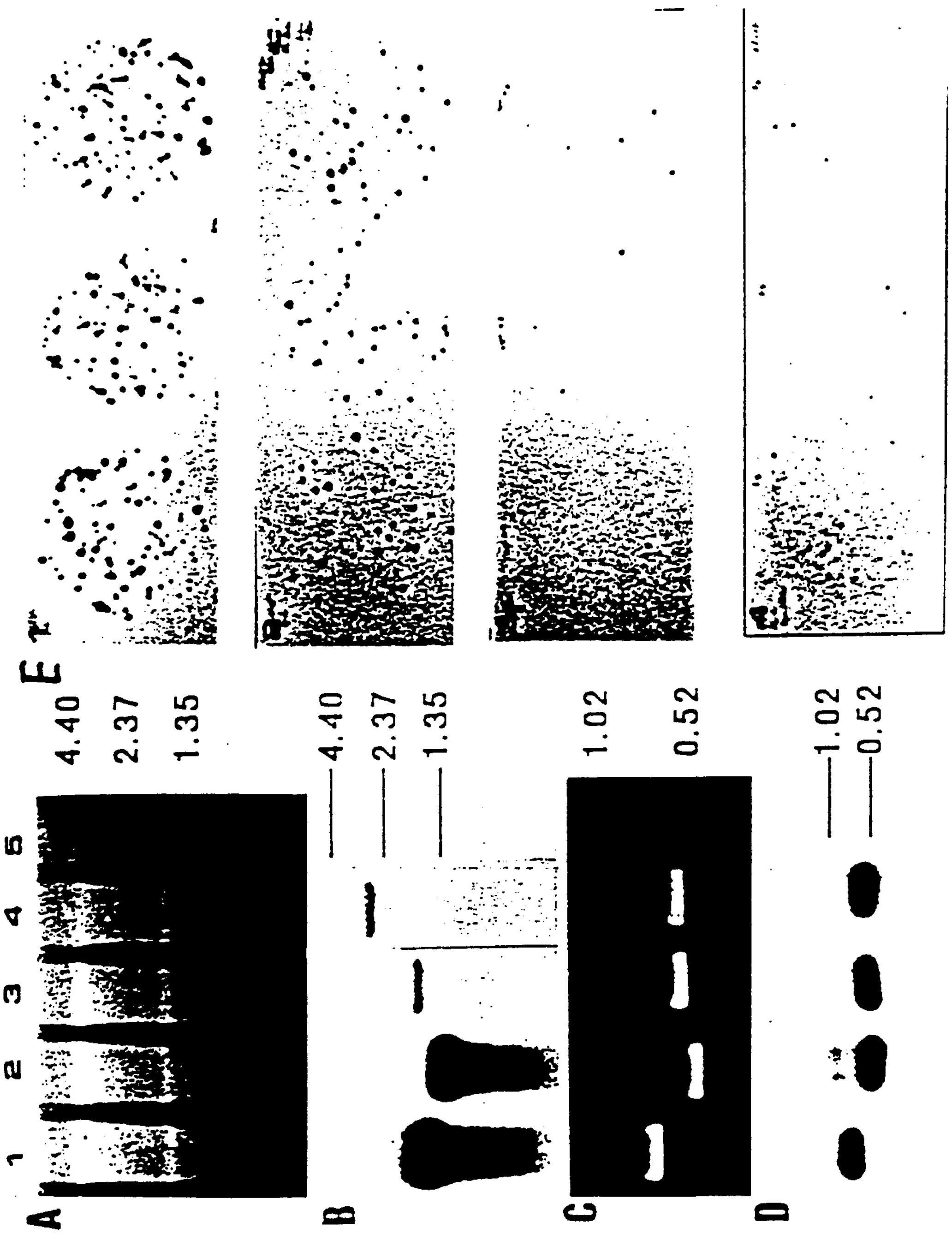

Fig. 4

F

| probe No. | 1 | 2 | 3 | 4 |
|---|---|---|---|---|
| gene | Elongation factor 1-α | α1-antitrypsin | HnRNP core protein A1 | Inter-α-trypsin inhibitor |
| (a) Band intensity of Northern blot(cpm) | 687 | 423 | 10 | 15 |
| (b)Band intensity of control blot(cpm) | 133 | 177 | 100 | 127 |
| (c)Normalized signal(a)/(b)x10 | 52 | 24 | 1 | 1.2 |
| (d)Positive signals on colony blot | 307 | 119 | 7 | 9 |
| (e)Relative representation | 44 | 17 | 1 | 1.3 |

Fig. 5

Appearance frequencies of various cDNAs in the 3'-directed HepG2 cDNA library

| Group | Clone | Gene | A in 982 (%) | B "in 8,800 (%)" | C "in 26,400 (%)" |
|---|---|---|---|---|---|
| I | a15 | Elongation factor - 1Aα | 22 (2.2) | 307 (3.5) | NT |
| | c321 | Translationally restricted tumor protein | 12 (1.2) | 89 (1.0) | NT |
| | lb038 | α-1-antitrypsin | 8 (0.8) | 119 (1.4) | NT |
| | hm01b02 | Light chain of ferritin | 6 (0.6) | 62 (0.7) | NT |
| | c13a04 | NADP(II) Menadione oxidoreductase | 4 (0.4) | 27 (0.3) | NT |
| | hm02d02 | Ribosomal protein S11 | 3 (0.3) | 29 (0.3) | NT |
| | lb042 | Human RNP core protein A1 | 2 (0.2) | 7 (0.1) | NT |
| II | s155 | unknown | 1 | 2 | 5 (0.02) |
| | s159 | unknown | 1 | 2 | 4 (0.02) |
| | s639 | unknown | 1 | 1 | 3 (0.01) |
| | s635 | unknown | 1 | 0 | 2 (0.01) |
| | s170 | unknown | 1 | 0 | 1 (0.004) |
| | s154 | unknown | 1 | 0 | 1 (0.004) |
| | s167 | unknown | 1 | 0 | 1 (0.004) |
| | s645 | unknown | 1 | 0 | 1 (0.004) |
| | s647 | unknown | 1 | 0 | 0 (<0.004) |
| | s632 | unknown | 1 | 0 | 0 (<0.004) |

Fig. 6

| GS | CH | Chromosomal position | Sequences of primers: Sense | Sequences of primers: Anti-sense | AT | HO | HE | MO | CO | G | T |
|---|---|---|---|---|---|---|---|---|---|---|---|
| gs000788 | pm2366 | 1 | CAGAGCCCCAGTACAACTAT | AAGTTTATTGTTGGGGTCAG | 48 | 114 | 115 | 104 | 110 | 1 | 2 |
| gs001026 | pm2444 | 1 | AATGGGACAGTTACACTTGA | CCAGCTTCCTTGACTTGAGA | 48 | 83 | 81 | >200 | >200 | 1 | 1 |
| gs001075 | pm0883 | 1 | TGGACTGTGGATACCTATCT | ACAAGTACCCCTGAATGGCT | 48 | 124 | 124 | 103 | 107 | 4 | 4 |
| gs001087 | pm1772 | 1 | GTCACTCTCAGCCATAGCAC | ACCATCTTCAGCCCACACTT | 50 | 104 | 104 | 180 | >200 | 6 | 6 |
| gs001094 | pm0347 | 1 | GCCCCTAACACGAGGAACTC | TAATTTCCCACTCCCGTAAC | 51 | 114 | 116 | >200 | 200 | 1 | 1 |
| gs001116 | pm1771 | 1 | GGGTTTTCAATAGGGTGTAGACC | GCCCCAATCTGTCAAAACTG | 49 | 95 | 95 | 78 | 107 | 1 | 1 |
| gs001191 | pm0609 | 1 | TTGCTGGATTGTAACTTTTG | GGCTGAACATTCACTCTTTG | 47 | 97 | 97 | - | 200 | 1 | 1 |
| gs001200 | pm1351 | 1 | TTAAGAAGACCCTTATGGAGACC | AATAATCTTGGTTAGTCACTTAC | 47 | 97 | 98 | - | - | 1 | 1 |
| gs001346 | pm0982 | 1 | TCAGGTCTGCTTGGAGGATG | AACTCACAGCACAGTATTTG | 53 | 120 | 122 | >200 | >200 | 1 | 1 |
| gs001446 | pm1518 | 1 | AAGGTGTACAGGATATTTGCAGA | TGCAATAGCCCAATCTCATT | 47 | 130 | 125 | >200 | >200 | 1 | 1 |
| gs001464 | pm1439 | 1 | CCAAAGACCTCCGTTGAACA | TTTGGGAGAGCCATAGACAG | 51 | 100 | 100 | >200 | - | 1 | 1 |
| gs001468 | pm0427 | 1 | TACTCAGTGGAAAGATAAAC | CAGTGGACCACATTTTCTTA | 40 | 98 | 98 | - | - | 2 | 2 |
| gs001521 | pm2785 | 1 | CCCAAATCAAATTGTTAAATG | TTTGAATCAGAGACATGAAGTT | 43 | 102,175 | 100 | >200 | >200 | 1 | 1 |
| gs001554 | pm2291 | 1 | CCAGAGAGTTCAAGGGATTG | GGTACAAAGTGCAAATGACT | 46 | 57 | 57 | 78 | 155 | 1 | 1 |
| gs001572 | pm2006 | 1 | CCAACATGGTCCTAGCACTG | AAACTTTATTGCAGCTTCTT | 44 | 58 | 59 | >200 | >200 | 4 | 4 |
| gs000120 | pm1350 | 2 | CATGATACTCTTCGGTGGTA | AAACAGTAGTTGCCAGCATT | 46 | 84 | 109 | - | - | 1 | 1 |
| gs001006 | pm1730 | 2 | AGGCTGAAATGTGGCATGCT | CCCGTTATTGCTACATGTCT | 48 | 119 | 119 | 93 | 115 | 1 | 1 |
| gs001081 | pm0931 | 2 | AAAGCAATACAAAATACCAA | TTCAATATGTTTAACCAGTA | 40 | 90 | 90 | - | - | 1 | 1 |
| gs001090 | pm0925 | 2 | TAATGTACCACGATGAATAG | TAATGTAATAATGCAGGTAA | 45 | 88 | 88 | - | - | 1 | 1 |
| gs001213 | pm2010 | 2 | CCAGATGGAAAGGGAAGTCT | CTGGAATATGGAGAATCAAACAG | 47 | 125 | 125 | 150 | >200 | 1 | 1 |
| gs001252 | pm0935 | 2 | TCGAGTTTTGTCTCTAATAA | GGAAATAATCGCTTCAGTTG | 43 | 103 | 103 | - | - | 1 | 1 |
| gs001268 | pm2093 | 2 | AGTCCTTCTTGGCTCCTCAT | TATCGTCAGTGCCTTTATTG | 52 | 137 | 137 | >200 | - | 1 | 1 |
| gs001438 | pm2435 | 2 | TTTTGTACCTACGTAAGAGTACTT | ATCCGTGCCACACATAGTGA | 45 | 105 | 108 | - | - | 1 | 2 |
| gs001442 | pm1671 | 2 | TTATTAGGGAGTCATTATTCTGTG | AGTTCCCATTCTTCCACATG | 45 | 67 | 65 | >200 | >200 | 1 | 2 |
| gs001453 | pm1245 | 2 | TTGCTTTCCCGTCTCTAAGT | ATGTACAATTTGCGTATGTAGG | 45 | 75 | 75 | 170 | 190 | 1 | 1 |
| gs001535 | pm1246 | 2 | ATCTACTGTTTGTTGAAGTG | ACTGATTTTGGTCCCATCTG | 44 | 68 | 67 | - | - | 1 | 1 |
| gs000875 | pm0449 | 3 | CGAACATTTCACCTCTCATA | ATGATTTATTTAGGCAGGAA | 43 | 68 | 69 | - | - | 3 | 6 |
| gs001001 | pm1758 | 3 | TCTGGCTCTTTGGTGTTGGA | GGCCCACTGAGTACAATGTC | 51 | 115 | 115 | - | - | 1 | 1 |
| gs001218 | pm2434 | 3 | AAAGAAAGCACACTGCCTAA | ATGTATAGACAAATCCAAAG | 42 | 90 | 90 | - | - | 1 | 1 |
| gs001219 | pm0668 | 3 | GTAGTCTCCTGCCCTTTAGC | AAGGATTTGATTTTCTACAT | 43 | 77 | 77 | - | - | 1 | 1 |
| gs001277 | pm1729 | 3 | GGTCCTGTTTATTTTGACAT | AAACAAGAGGATGGTTCAGA | 43 | 75 | 75 | 155 | >200 | 1 | 1 |
| gs001306 | pm1822 | 3 | GATCCTTGGTGTGTAGTTCAGTC | CTGCAAAATACAGGGAATCAT | 46 | 83 | 83 | 160 | 140 | 1 | 1 |
| gs001418 | pm2209 | 3 | ACCCCAGTCCCAAATCCAGT | ACACTCCCCCAGCCCTTACT | 55 | 105 | 105 | 113 | >200 | 1 | 1 |
| gs001466 | pm2455 | 3 | ATCTAGCTGGCTGTAGTATT | TTAAAGAGATGAATTTATTGGT | 42 | 130 | 130 | 190 | >200 | 1 | 1 |
| gs000271 | pm1252 | 4 | GTCCTTTGCTATCTGTGTTA | AAGCATTTATTTGAGGTTTA | 43 | 90 | 90 | 95 | >200 | 1 | 3 |

Fig. 7

| | | | | | | | | | | | |
|---|---|---|---|---|---|---|---|---|---|---|---|
| gs000448 | pm2256 | 4 | GGCCAAGTTTCTTCTAGTAT | GTTCAGTTTTATTCAGAGCA | 42 | 62 | 62 | >200 | 69 | 1 | 2 |
| gs001052 | pm1151 | 4 | GTGCCATGCACTGTTGTTAT | GTCATATATTCCATCATCAA | 43 | 80 | 80 | - | - | 1 | 1 |
| gs001215 | pm0988 | 4 | AGAAAATTAATAGCATAGGT | TAGAGTCAAAGTTGCCTGTG | 43 | 100 | 100 | 130 | - | 1 | 1 |
| gs001298 | pm2367 | 4 | ATCAAAGTTTAATTGCTTCA | CATCCCATCACATACAAGTC | 43 | 116 | 116 | >200 | 180 | 1 | 1 |
| gs000993 | pm0904 | 5 | TCTCGTGAAGAGCAGCACAA | TCTAAAGGAAGAACAGCATC | 49 | 101 | 102 | 113 | 200 | 1 | 1 |
| gs000598 | pm1889 | 5 | AAGCAATGCCTTATCCACAG | CTAAAGAGCTTGAACCCTTCAT | 45 | 87 | 87 | >200 | >200 | 1 | 1 |
| gs001065 | pm0319 | 5 | TCACCCAGATAATTTACAGT | GAGACATAAGCAGGTAAGAT | 44 | 120 | 120 | - | - | 1 | 1 |
| gs001101 | pm2364 | 5 | TTACCTTACCGTGTCTTTAC | AGACAAATATCCCAAAAAGC | 47 | 89 | 89 | 190 | >200 | 1 | 1 |
| gs001461 | pm1160 | 5 | ATTTTGTGAGTGGTTTACTA | AGAAATGGATGCTTTTATTC | 43 | 101 | 99 | >200 | >200 | 1 | 1 |
| gs000353 | pm2720 | 6 | AATGTCATAGTCTCCTTTCA | TGCATCCTTCAATGTCTTCT | 44 | 78 | 78 | 72 | >200 | 2 | 3 |
| gs001326 | pm1154 | 6 | CATTGAGACAGCAGCAACAG | CCTGGCCCTCTTCCTGAGTA | 53 | 102 | 104 | 145 | 200 | 1 | 1 |
| gs001434 | pm1216 | 6 | TAGGCAAAACACGAGAAGAG | AAGGAGCTGGTGTCAGGTTC | 48 | 65 | 65 | 110 | >200 | 1 | 1 |
| gs001457 | pm1785 | 6 | TATATGCAAATATTCCAAAGTCTG | TCTAATAATTCTGGTCCCTTATCT | 46 | 90,>200 | 90 | >200 | >200 | 1 | 1 |
| gs001523 | pm0285 | 6 | TTGTAACGTGTGTCGTCAGT | TTTAAAAATGTCATGGTAAT | 42 | 86 | 70 | >200 | 100 | 1 | 1 |
| gs001525 | pm0328 | 6 | GCACCTAAGCCTCCCAAAGT | TTTTATATCAGTCCAAGAGC | 49 | 138 | 138 | >200 | >200 | 1 | 1 |
| gs001562 | pm2619 | 6 | TCTGCATTGACAAGGACCAC | TTTGAGATTTTAATGAGTCATTC | 43 | 62 | 62 | >200 | 45 | 1 | 1 |
| gs000624 | pm0991 | 7 | GACCTGAAGTGTGAATGAGT | AACTTAGCTTTATGGGATTT | 45 | 119 | 119 | >200 | - | 1 | 2 |
| gs001145 | pm0281 | 7 | AGCCAAACTCGGGGTCATCT | CCACGGGACAGGTGAGTCAT | 56 | 159 | 159 | 115 | >200 | 4 | 4 |
| gs001469 | pm0219 | 7 | AATCATTTGGCGAGACTGTA | AAGACAACTTTATCCAGACA | 45 | 88 | 89 | 130 | - | 1 | 1 |
| gs001579 | pm1102 | 7 | TCAGGCAGTCTGCTCAGATA | TTTGCAGGTTAATCTGTTTA | 44 | 77 | 76 | 170 | - | 1 | 1 |
| gs001207 | pm0956 | 8 | AACAGTATTGCGTTGTCAGACTAG | TCCATTAATAAGGCCAGTCTTCAG | 47 | 81 | 81 | 105 | 70 | 1 | 1 |
| gs001176 | pm2527 | 9 | TTGCCTCTAATGGTGTCTAC | AAAAACCAGAACACACTAAG | 48 | 99 | 99 | 118 | 180 | 1 | 1 |
| gs001248 | pm2708 | 9 | TGTATTGGATTTGGATTCTC | CAAAAGCAAAACAGCAGATA | 44 | 95 | 95 | - | 85 | 1 | 1 |
| gs000260 | pm0995 | 97 | TTGCCATCAAAACACATACA | CTTGTGAGTTTTGGTTTCTG | 43 | 55 | 55 | - | - | 1 | 1 |
| gs001055 | pm0959 | 97 | TTAAAGAATCACCCTCATTG | CACATGCTTATTGGAACACT | 44 | 74 | 74 | 72 | 73 | 1 | 1 |
| gs001157 | pm0547 | 10 | AAGTATTGTGCAAAGATGTA | AAGAAAACACTGCCTTGTGG | 45 | 138 | 139 | >200 | >200 | 2 | 3 |
| gs001268 | pm2245 | 10 | TGTGAAATGCTATCTCTCCT | GCAATCGTTTCCATATCAGT | 47 | 100 | 100 | 200 | >200 | 1 | 1 |
| gs000228 | pm2664 | 11 | ATCAAAACAAACAATCCAGA | ACTATAATATACTGCCAACT | 42 | 117 | 121 | 134 | 95 | 2 | 2 |
| gs001159 | pm0880 | 11 | GAATAGCTTGGAGATTTCAC | GGAGAATCATACCTTCAGCA | 46 | 100 | 100 | 84 | 95 | 1 | 1 |
| gs001315 | pm0445 | 11 | AAAGTGACCTTGATGGACAGTGGA | TCGAGCCAAAAATACATGCTGACT | 50 | 153 | 153 | >200 | 160 | 1 | 2 |
| gs001352 | pm2943 | 11 | AGGGTGAAGGGTATTTTACG | CACATCATGGTTGAGAGCTA | 47 | 83 | 85 | - | - | 2 | 2 |
| gs001469 | pm0559 | 11 | AACCCTCTAGTAAGGCATTG | TTATTAAACCAAATCCAGTA | 37 | 47 | 47 | 125 | 53 | 1 | 1 |
| gs001570 | pm2810 | 11 | CTGTAAAGGTTTTTGGAATTATGT | TTTCATTTTTCTACCAGATTTATT | 42 | 75,82 | 75 | 145 | >200 | 3 | 3 |
| gs000279 | pm0266 | 12 | AGTGTATGGAAGACCTTGAG | GTTCATTGAAACGGTGTAGC | 48 | 130 | 130 | 103 | >200 | 1 | 2 |
| gs001163 | pm2756 | 12 | TCTCCCTATTCACAACCAGT | AATGATTTCGTAGGATAGCA | 49 | 88 | 89 | >200 | 120 | 1 | 2 |
| gs001193 | pm1193 | 12 | CACAGCATAAAAGAATCATA | ACCCTAATTTAGTTTCTCAC | 46 | 100 | 100 | - | - | 1 | 1 |
| gs001235 | pm2790 | 12 | CATCATGGTACAGTCAGAAG | CAGTTTGTCAAAATGTATTG | 44 | 83 | 82 | 93 | 87 | 1 | 1 |
| gs001274 | pm1355 | 12 | AGATGTCAGTATTCTCCTCATGG | GAGAACAGCAGTAAAGCAACCAC | 47 | 87 | 87 | >200 | >200 | 1 | 1 |
| gs001308 | pm0368 | 12 | CCAAAGTGCTAGGGTTACAG | TTCAATAGACCTTGGGTTAC | 47 | 95,165 | 95 | >200 | >200 | 1 | 1 |
| gs000159 | pm2645 | 13 | CTAAGATTTAATGCGATTCC | AGTTAGTGTATGGCAGAGGA | 46 | 104 | 104 | >200 | - | 1 | 2 |

Fig. 8

| | | | | | | | | | | | |
|---|---|---|---|---|---|---|---|---|---|---|---|
| gs001044 | pm1659 | 13 | TTGTAAGCCTATCAGAGTCA | AGACAGACTTATGCCATCTA | 44 | 109,200 | 109 | >200 | 100 | 1 | 1 |
| gs001290 | pm1731 | 13 | GCTTCTTCCTGTGCTGTGGT | GCAGTTAATCATGGCTATTCTCC | 50 | 122 | 122 | >200 | 190 | 1 | 1 |
| gs001362 | pm0118 | 13 | ACTGAAATGGAACATAGTCT | TACATTACATGACATTGTGA | 40 | 61 | 61 | 95 | 103 | 1 | 1 |
| gs001366 | pm0364 | 13 | TGCTTAGCTTTCCCTCCTTA | GAGCATTTCTGTTGTTCCTA | 45 | 67 | 67 | . | . | 1 | 1 |
| gs001389 | pm2301 | 13 | CATGAACCTGCTCACGACAA | GCCTTACTTTAATGCTGACC | 51 | 100 | 100 | 100,>200 | . | 1 | 1 |
| gs001492 | pm0541 | 13 | AAATGAATGTAAATAGCACT | ATTTAGTTTACAGGGAGAAT | 41 | 72 | 72 | . | 74 | 1 | 1 |
| gs001367 | pm0441 | 14 | GTTTTAAGTTTTGATTTGGG | CATTCCACTCTTACATTTCT | 41 | 77 | 77 | >200 | 180 | 3 | 4 |
| gs001564 | pm2307 | 14 | CGTTCCTAAACTCTGAAATC | AATGCTCATTTATTCTCAAG | 42 | 55 | 55 | >200 | >200 | 1 | 1 |
| gs001576 | pm2019 | 14 | ATCACAATTACCTTTAGTTG | ACGATAACTTTATTGGAGAT | 39 | 69 | 69 | . | . | 1 | 1 |
| gs001339 | pm2220 | 15 | TCCCCATCCTCAGTTGAAGT | TGAGAACAAAAGGAACCAGT | 47 | 70 | 70 | 80 | 150 | 1 | 1 |
| gs000980 | pm0985 | 16 | TTGGAATGGAACCCTTGCTA | ACTTATGCTGCCTGAAATGG | 48 | 79 | 79 | 66 | 70 | 2 | 2 |
| gs001242 | pm1127 | 16 | CCCTTGTTTTTACATGTTCA | TATTAAATTCTCCCATTCAT | 44 | 105 | 105 | 103 | 102 | 2 | 2 |
| gs001516 | pm2543 | 16 | ACAGTGCTAAAATCAAAGGTG | TCTGACAACTCAAGGTGCAAT | 45 | 70 | 70 | >200 | . | 1 | 1 |
| gs001566 | pm0913 | 16 | TTTGTGTCGGACTATGTAAT | TCACTTTTAATGGGAACCAG | 41 | 53 | 53 | >200 | >200 | 1 | 1 |
| gs000806 | pm1157 | 17 | CTCTCCATGTTCTCTACAAG | TAGAAGGAGAATCTGTGGTT | 47 | 77 | 77 | 140 | >200 | 2 | 3 |
| gs001015 | pm2369 | 17 | ATATTCACCTTCCCATCCAT | TCAAATACGTCCTCTCAAGC | 50 | 80 | 80 | >200 | >200 | 1 | 1 |
| gs001156 | pm0202 | 17 | CAGAAATTAAGTGCAGCAAT | TCGTATCTGCATCTTTAAGT | 45 | 103 | 100 | >200 | >200 | 2 | 2 |
| gs001173 | pm2117 | 17 | AAAATCTTGTGGTTATTTCC | GTGATTCTACTGTACATTGC | 41 | 118 | 118 | 145 | 200 | 1 | 1 |
| gs001301 | pm1878 | 17 | TAAATTTGTGGAAATCTCTTGGA | ACACATTTGGGTTTGCTTTAAC | 47 | 100 | 100 | 95 | 97 | 1 | 1 |
| gs001316 | pm0511 | 17 | TGTGACAGCAGCAGCTTCAT | TCGTACATTTTAATTCCACC | 45 | 128 | 129 | . | 1 | 1 | 1 |
| gs001356 | pm0538 | 17 | CATCTCACAGACAAGGAAAC | ACCTAAGAGTCCAGAGAAAC | 48 | 90 | 90 | 69 | >200 | 1 | 1 |
| gs001495 | pm2212 | 17 | TGACTGCAATAAGGAGTTGT | GAACATACCACGTTTATTTCT | 46 | 90 | 90 | 180 | >200 | 1 | 1 |
| gs001522 | pm0642 | 17 | GTCTTCAGCAGATTTCAGGT | ACTTTCTTCTTGAGGACACA | 45 | 68 | 68 | 160 | . | 1 | 1 |
| gs001078 | pm1815 | 19 | TGTGTTCTCCAGCTTTGTAG | GTTACATTGCCTTGGTACAG | 48 | 65 | 65 | >200 | >200 | 1 | 1 |
| gs001417 | pm0289 | 19 | GGATCAGACCAACAGTGCTG | GCAAGGTATAAAACAGATTA | 46 | 50 | 50 | . | . | 1 | 1 |
| gs001467 | pm1688 | 19 | GAAGCCCACCCTGCACCTCA | GGAGAGTATTGGGGAACGGT | 54 | 93 | 93 | >200 | >200 | 2 | 2 |
| gs001069 | pm1879 | 20 | GCCATGCTTGTAAAGTGATGT | TTAAGAAGCCATTAGCTAGGATA | 48 | 140 | 140 | . | . | 1 | 1 |
| gs001068 | pm1146 | 20 | GCCCTTAGGATTCACTGCTC | ACCACCCAAGGTCTTTCAGG | 52 | 66 | 66 | 180 | >200 | 1 | 1 |
| gs001069 | pm0112 | 20 | TGCTGGATGACTTCTACACG | TCCCTATCATGGCTGCTGTT | 49 | 59 | 59 | 59,115 | 59 | 1 | 1 |
| gs001128 | pm0332 | 20 | CTGCTCGGCTAGTCTGACTC | CAAATGGTCTAAGAGGACAT | 49 | 135 | 135 | 153 | 160 | 1 | 1 |
| gs001132 | pm0647 | 20 | TCTGAATGATGATGGAAACA | ATCCTAGTCCCAACCCAGTA | 48 | 109 | 109 | . | . | 1 | 1 |
| gs001158 | pm1774 | 20 | GGAGCCACATGGATTGATTG | AAATGTACCCCTGGCACCTC | 52 | 124 | 124 | >200 | >200 | 1 | 1 |
| gs001210 | pm1235 | 20 | AGCCATCTGGTTATGTCTTA | GGAGCAGAATGAAACTTCAC | 44 | 90 | 90 | >200 | >200 | 1 | 1 |
| gs001377 | pm1701 | 20 | TCCATGGTGTTAGAAGCCAG | CCACATCTCCAACAGGGAGT | 54 | 142 | 142 | >200 | 74 | 1 | 1 |
| gs001395 | pm2101 | 21 | GTCAGCTCAATGCTACACAG | TTTATAGTGCAACACAGAGT | 45 | 130 | 130 | 180 | >200 | 1 | 2 |
| gs001427 | pm0648 | 21 | CTTCTGCTATAAAAGTAGAG | ACAATTGGTTCACTAAATGA | 39 | 58 | 58 | 145 | >200 | 1 | 1 |
| gs000978 | pm0912 | 22 | GGTGTAGTGTAACCATTTAG | AGTTGCACCCATCTCCTGTC | 46 | 124 | 124 | >200 | >200 | 1 | 1 |
| gs001444 | pm0911 | 22 | GGTCTTGTTCTCCCATCTGT | AGAAAGCCCCAAAGTAGTCC | 48 | 65,80 | 65 | 100 | 125 | 1 | 2 |
| gs001473 | pm2231 | 22 | TGAGCTGCACTTACCTGTGAGAG | AAGCAGGTTGAGTTGGGTTTTCT | 50 | 94 | 94 | 67 | 135 | 2 | 4 |
| gs001479 | pm2328 | 22 | TACAGCCCTCCCAGCTAAAC | TTTATTCTGCATCCACTACAA | 46 | 65 | 65 | 190 | >200 | 1 | 1 |

Fig. 9

| | | | | | | | | | | | |
|---|---|---|---|---|---|---|---|---|---|---|---|
| gs000999 | pm1759 | X | CTGCCATAGTTACCTGGATT | TCACCCACCACTATTTAGCA | 47 | 103 | 103 | - | - | 1 | 1 |
| gs001149 | pm2180 | X | GGAGGGGAGATATAGATTGT | AAAAAAATCCAGAAGACTGA | 46 | 70 | 70 | 135 | 150 | 1 | 1 |
| gs001161 | pm0608 | X | TTCTATAAGTGTGACCAGTT | GGAGGATTTGAGATACACAT | 40 | 85 | 85 | >200 | 77 | 1 | 1 |
| gs001406 | pm1294 | X | TAATGCCAGTGAATGTTGCGTAA | GTAAAGGTTTATCCTTGCATCAGA | 47 | 82 | 82 | >200 | 80 | 2 | 3 |
| gs001160 | pm2289 | 1,18 | ATCCTGCTGAAATACATCTG | GGGGAGAGACATCACATGAC | 46 | 70 | 70 | 68 | 130 | 1 | 1 |
| gs001436 | pm0113 | 1,2,12,13,Y | GATCCGATGGGAGTGTAAAT | AATACAAAGCTAAACCACAA | 44 | 69 | 69 | 170 | - | 1 | 1 |
| gs001404 | pm2272 | 1,2,3,5,8,12,14,17,X | TTGGAATTGACATTCTCTAT | TTTATTGTAACAAAGCAACT | 43 | 130 | 130 | 150 | 132 | 2 | 2 |
| gs000803 | pm0314 | 1,2,6,X | TATCAAGCTGAAAATGTCAC | TTACTGAATCCAGCCAACCA | 45 | 93 | 93 | 110 | - | 1 | 3 |
| gs000140 | pm1461 | 1,3,4,5,8,16 | TCCAAATGAAGAAGGTGTTA | AGTTGACAGCCAGGTGAATG | 49 | 96 | 96 | 100 | 100 | 1 | 3 |
| gs001354 | pm1561 | 2,20,21,22 | GTCTGTCAAGCCAAGATTCA | TTTTTATTGTTGCTCCAAGT | 48 | 110 | 110 | 170 | 150 | 1 | 1 |
| gs000336 | pm2795 | 2,4,5,10,12,15,17,20,22,Y | GACCTGTGACATTCTGGACT | TTATATGGTTGTTACACTCG | 43 | 61 | 61 | - | - | 2 | 6 |
| gs001077 | pm0943 | 2,5,14,C | GCCTTGTTATTTCACCACTC | ATCTCCCTTTGCTCCAGTTA | 46 | 82 | 82 | >200 | 82 | 1 | 1 |
| gs001192 | pm1853 | 2,8,12 | TCTGAGGACATTCCAAGACAG | CAGTCAAAACCAACACGGTAT | 49 | 95 | 95 | 93 | 160 | 1 | 1 |
| gs000213 | pm1778 | 2,9,13,17,X | TGCAATAAAGGGAAAGACCA | CCGTTGTAGGTGATGAAATG | 49 | 78 | 80 | >200 | >200 | 1 | 2 |
| gs000919 | pm0885 | 20,X | GTCATTTGTATGCAATTTCC | ACATTTTTATTTTTTCAACG | 37 | 45 | 45 | - | - | 1 | 2 |
| gs001109 | pm0457 | 3,10,15 | CATGTACTCAGAGGCACTTC | GCAACTACAAATCCCAAACT | 50 | 133 | 133 | >200 | 150 | 2 | 2 |
| gs000071 | pm2651 | 3,4,M | CAGGGACTGGAGCAGGAAAG | GATTTAACCCATTAGGAAGC | 50 | 101 | 101 | 101 | 88 | 3 | 3 |
| gs001426 | pm2632 | 3,6 | TTAGGAAAATATGGTTAGACAG | ATAGTATTGGGTTGACACAGTA | 43 | 80 | 80 | >200 | 120 | 1 | 1 |
| gs001391 | pm1133 | 3,8 | TGGATTTGCTTTACCTTGTT | ACACCCTCAGGAGATGTTAC | 47 | 93 | 93 | 95 | >200 | 1 | 1 |
| gs000077 | pm2250 | 3,9,10,15 | GCACTACAAGCCAAATCAGA | CTTCTTAACACCAACAGCAG | 50 | 96 | 96 | >200 | 125 | 2 | 10 |
| gs000605 | pm0626 | 4,6 | GGATTTCTATTTGCTGTCAT | GTTTATTGTACGGCATTTAC | 44 | 105 | 100 | >200 | >200 | 2 | 4 |
| gs001212 | pm1234 | 6,20 | GCATTAAACAGGAAACAATA | CTGTCCATGTGGCATAAACC | 44 | 110 | 110 | 105 | 107 | 1 | 1 |
| gs001312 | pm0606 | 7,18 | AGATGCTAACATTAGGGATA | TTTTAGACATACAGAGGAGT | 43 | 81 | 81 | 102 | - | 1 | 1 |
| gs001441 | pm1253 | 9,11 | CCAGACTACAGGCTGATGGC | CCCTTACCCCAGCAACTCTT | 55 | 75,130 | 75 | >200 | >200 | 1 | 1 |
| gs001357 | pm0115 | 9,M | ACCAATGTCACTGCTTCTAAAATA | CCCATAATAAGTGAAGAGGTAGTTC | 48 | 125,155 | 127 | 125 | >200 | 1 | 1 |
| gs001261 | pm0428 | 10,15,22 | AAGAAATTGTTTACTGGATT | TTATCTGACTTGGAGGAAAT | 42 | 107 | 107 | - | - | 1 | 1 |
| gs001456 | pm2420 | 10,15,22 | ACTACCCCTGAGATATTAGTT | TTCATTTATTTGATTAGTTGA | 46 | 100 | 100 | 170 | - | 1 | 1 |
| gs000290 | pm2303 | 11,M | ATACCACTTCCGCTGTCACG | GAGGAGCGTCTACTGGTCTT | 50 | 72 | 74 | 72 | >200 | 3 | 20 |
| gs000314 | pm2643 | 12,19 | GCACCAAGAAGCAGTTCCAG | TTGGGAATGAGAAAATAACT | 46 | 83 | 83 | 81 | - | 1 | 7 |
| gs000403 | pm2773 | 12,M | GATCTCAGTTCTGCGTTTATT | TACATACAAAGATGCAAACAGT | 44 | 80 | 80 | 79 | 68 | 1 | 1 |
| gs001487 | pm2725 | 13,16 | ATTCTTGTGTGCTGCTTTCC | GTCTCTCTTCTGATGGCTGA | 46 | 62 | 60 | 135 | 180 | 1 | 1 |
| gs000976 | pm2780 | 14,16 | AACCTGTTTTACCGCATCTT | AGGTTATTTGTCCACCAGAA | 48 | 87 | 87 | >200 | >200 | 1 | 1 |
| gs001435 | pm1683 | 17,20,C | TGTTGGTTCACCATTGAGAC | AGAACAACACATCAAAGATGC | 46 | 90 | 90 | >200 | 90 | 1 | 1 |
| gs001393 | pm1748 | 17,22,Y | GAATGTCATCCAAGACGTAG | CTAGTTATATCCTGGCTCTG | 44 | 81 | 81 | >200 | 200 | 1 | 1 |
| gs000096 | pm0964 | 17,C | TTTATCCCAGCAAGCACAAC | TCTTCCTCTTCACTCTCCTC | 49 | 120 | 120 | >200 | 170 | 4 | 11 |
| gs001369 | pm2217 | 17,C | ACTTAAAGTAGCTTTGTACG | TGCCTCCTGGTCTGATAATA | 43 | 95 | 95 | >200 | 95 | 1 | 1 |
| gs001440 | pm1213 | 18,C | CCCCAGTTAAAGATTATTGT | AGTGACGATGGAAGGATGTA | 44 | 92 | 92 | - | 92 | 1 | 1 |
| gs001217 | pm1118 | 19,20 | TGCAGAGTGATTTTCCAGAG | CGTAGGTCATTCTTTTCAGC | 46 | 75 | 72 | 160 | 65 | 1 | 1 |
| gs001009 | pm2824 | 19,22 | ATCCCTCTGTCTATTCACAC | GCTCGTTTAACTCACTTCAC | 46 | 110 | 110 | 130 | 170 | 2 | 2 |
| gs001172 | pm0887 | 19,22 | GCCTGCATCTGTGTTGACTT | AACCTCTGGGAACAAATCAT | 48 | 91 | 89 | 160 | 86 | 1 | 1 |

Fig. 10

| | | | | | | | | | | | |
|---|---|---|---|---|---|---|---|---|---|---|---|
| gs001057 | pm2049 | C | AGGACACAAACACCAGCTAT | TTTTCTGATTATGACATGAC | 45 | 75 | 75 | 101 | 75 | 1 | 1 |
| gs000473 | pm1753 | M | ATCTCTTTGTAGCCATCCTG | GTTAAAGTGCTGATGCCATT | 42 | 64,100 | 64 | 64 | >200 | 1 | 1 |
| gs001096 | pm2236 | M | GTAGAGCTGCATTGACTACC | ACAGACAAGGAATAATCATA | 42 | 108,96 | 110 | 110 | 112 | 1 | 1 |
| gs001166 | pm0506 | M | GTCCCACAGTCCAGCCTAAC | GCCACATATTAGAATCCATC | 46 | 74 | 74 | 74 | >200 | 1 | 1 |
| gs001454 | pm2354 | M | TGTCTTTGTGGACTCTGCCT | TTTAACAGTCAATAAATACATGTT | 44 | 110 | 110 | 110 | 106 | 1 | 1 |
| gs000329 | pm2492 | M,C | GCTAGAAAGAAGGGCACTCA | CTTAACTCGATAGCCAGGTC | 46 | 75 | 75 | 75 | 75 | 1 | 1 |
| gs000253 | pm2786 | M,C | CACAAAACAGCAAACTTCAG | ATGGTTATTTTATCAGATTG | 41 | 83 | 83 | 82 | 83 | 2 | 3 |
| gs000285 | pm1704 | M,C | TCCACCCAGAGAAGCACACT | AATTCATAGGGAATAGGTTC | 48 | 75,130 | 75 | 75 | 75 | 1 | 23 |
| gs000302 | pm2318 | M,C | TCGAGAAGGACAAAATCACC | GAACAGGGTTAGTCCATTCG | 48 | 58 | 58 | 58 | 58 | 1 | 1 |
| gs000543 | pm1689 | M,C | CATGAGGCTACGGAAACAGG | AGGAGTCCGTGGGTCTTGAG | 51 | 84 | 84 | 84 | 84 | 4 | 18 |
| gs000675 | pm1442 | M,C | AAAGCATCTTGAGAGGAACA | GGAGGACTCGCTTGGTCTTA | 49 | 110,>200 | 110 | 110 | 110 | 1 | 9 |
| gs000732 | pm1452 | M,C | GCAGCAGATACCTTTACACC | TGGTTCATTTCAGTTCCTTC | 51 | 102 | 105 | 102 | 102 | 3 | 13 |
| gs000995 | pm0268 | M,C | GAAGCTCTTGTGAGGAAAGT | CAGACCCCATCTTTTATACC | 47 | 79 | 79 | 79 | 79 | 3 | 4 |
| gs001016 | pm2783 | M,C | ACGATATTTATAGTGATGTG | TCAAAACTTTAATATATGCT | 40 | 93 | 93 | 91 | 92 | 1 | 1 |
| gs001053 | pm1144 | M,C | AGATGAGTGTGGGTCAGAGA | CCATTCCTGTCATTCCAGTT | 52 | 135 | 140 | 135 | 135 | 1 | 1 |
| gs001127 | pm2290 | M,C | ACTGGTGATGGAAGGTTACA | CCACACAGTGAGCACCGTCT | 47 | 55 | 55 | 55 | 55 | 1 | 1 |
| gs001167 | pm1626 | M,C | GAGAGCCCTTGCATCCTTTA | CTTCCCTTTGGTCTTTCTGT | 49 | 100 | 100 | 100 | 100 | 1 | 1 |
| gs001216 | pm2109 | M,C | TAGTCAGAGATTCAGTAAGT | ACATGTATTTTGATAGTCTT | 42 | 110 | 110 | 110 | 110 | 1 | 2 |
| gs001253 | pm1240 | M,C | AACTGGTTCCATCAAGACTG | AGTGAATAAACTCTCCACTCC | 48 | 120 | 120 | 120 | 120 | 1 | 1 |
| gs001281 | pm1131 | M,C | ACTTAAAAACCCACCAGCAT | ACAACAGCAGTCAAATAGAA | 47 | 97 | 97 | 97 | 97 | 1 | 1 |
| gs001375 | pm0952 | M,C | AAGAGGAGTTTCCCTGCTCA | ATCATGGCAGATGGCAAGGA | 51 | 89 | 89 | 89 | 150 | 1 | 1 |
| gs001396 | pm2216 | M,C | ATCTGCATGACCTATAATCT | CGTTCTCTTTTATTTGACAT | 45 | 108 | 108 | 108 | 108 | 1 | 1 |
| gs001411 | pm0958 | M,C | ATGGGTTTATCAGGGGTTTC | GAGACCAAAGGCACTTCTTA | 47 | 80 | 77 | 80 | 80 | 1 | 1 |
| gs001460 | pm2626 | M,C | ACATTGAATGGGGATGAGGT | GGACATTTCTAGCCCACAGC | 51 | 75,55 | 75 | 75 | 75 | 1 | 2 |
| gs001482 | pm1210 | M,C | TTGTTGACATTCCTTTTAGAA | CAGTGCCTCTGTACTGAGACA | 46 | 85 | 85 | 85 | 85 | 1 | 2 |
| gs001490 | pm0109 | M,C | GCCCACAGAGACATCATCCT | TCTTAGTAGGTGCTCTGGTG | 51 | 98 | 98 | 98 | 98 | 1 | 1 |
| gs000168 | pm2042 | No product | CAACCAGTTAGCGTGAAAGT | GAAATAATCCTTGTCATCTA | 45 | 87 | 87 | . | . | 1 | 1 |
| gs000850 | pm0304 | No product | CTTTGGGATATTTTCTTCAT | CCCTCGGGTACTTTTCTATG | 43 | 60 | 60 | 62 | . | 1 | 2 |
| gs000983 | pm0808 | No product | AGCCAGCCTCTTTGTATGTG | CTGGATTTGATTTTCATTAG | 44 | 87 | 87 | . | 112 | 1 | 1 |
| gs001254 | pm1673 | No product | TGTGGTATGAAAATATCTGA | TTATGAATGAAGACAACACT | 43 | 98 | 98 | 163 | >200 | 1 | 1 |
| gs001365 | pm2908 | No product | CAGTAGTGTGCTTTGAAATG | TTTATGTGAAATGTGGTTGT | 41 | 63 | 63 | . | 150 | 3 | 3 |
| gs001373 | pm0361 | No product | TACAGCCGCTTCTAAAAGTC | TTTGAGCATCAAGGAAATCT | 46 | 82 | 82 | . | >200 | 1 | 1 |
| gs001556 | pm0849 | No product | TACATTCTTCAGACTCATCG | TTTTCAAAAACTTTATTCTT | 40 | 86 | 86 | >200 | 100 | 1 | 1 |
| gs001574 | pm1284 | No product | ATCAGAGCTCAGTTCCTGTAG | ATTTTGCCTCTTGCATGGTC | 44 | 57 | 57 | 67 | 67 | 2 | 2 |
| gs001622 | pm1606 | No product | GATCTTGAGCCTTAACTGGA | TTTGCAGTTCAGCTTTATTC | 45 | 54 | 54 | . | . | 1 | 1 |
| gs001640 | pm0852 | No product | GATCTCTGTTCCTTTTCACA | TTTATAACAAGACACCATAC | 36 | 45 | 45 | . | . | 1 | 1 |

Fig. 11

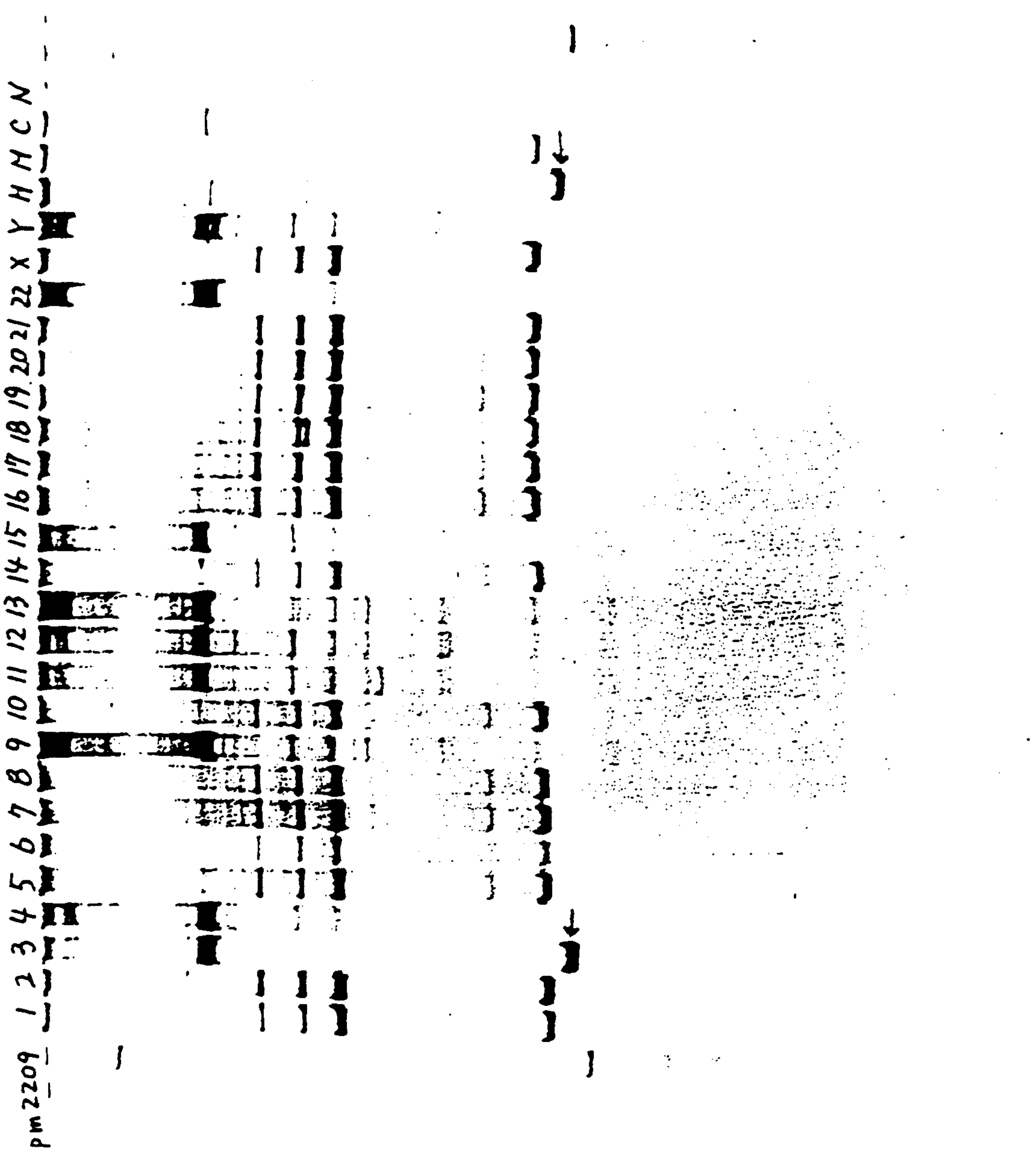

Fig. 12

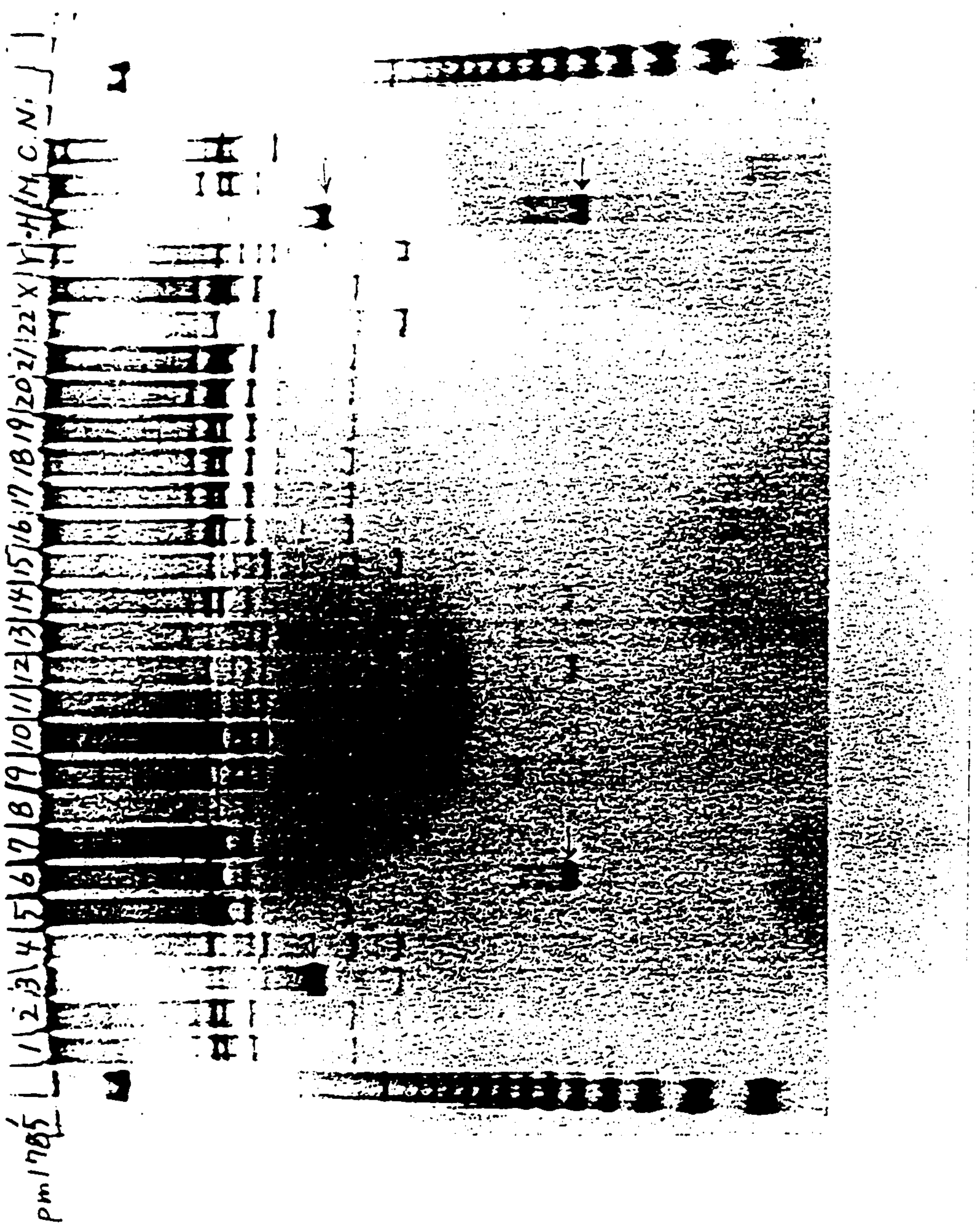

Fig. 13

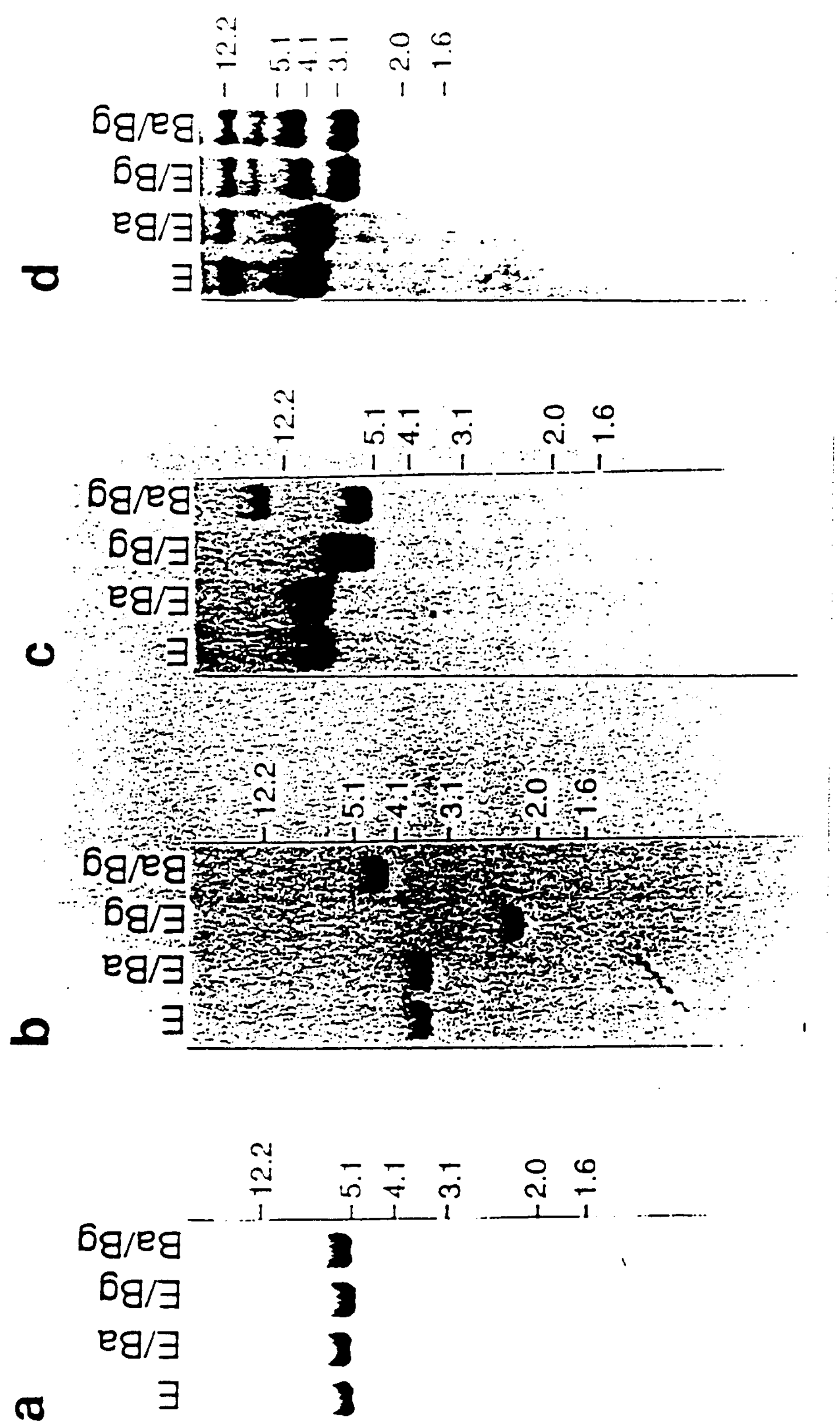

Fig. 14

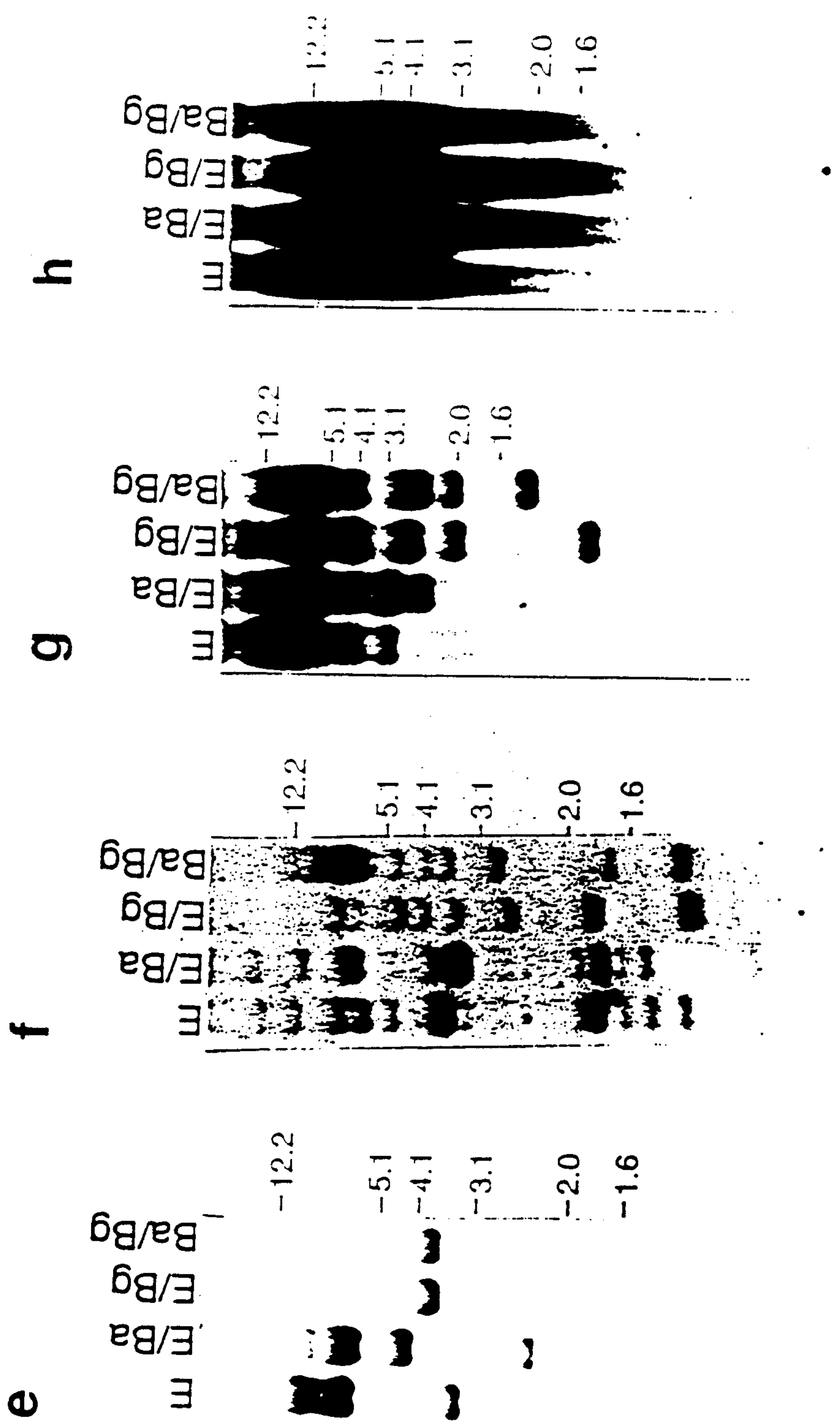

Fig. 15

Hybrid cells used for Southern hybridization

| Hybrid cell | Human chromosome No. | Parent cell | Intact chromosome (%) | Translocated chromosome (%) |
|---|---|---|---|---|
| A9(neo-1)-4 | 1 | A9 | 100 (0) | 0 |
| A9(neo-2)-1 | 2 | A9 | 93 (8) | 0 |
| GM10253 | 3 | CHO | 100 (0) | 0 |
| GM10115 | 4 | CHO | 100 (0) | 0 |
| A9(neo-5)-4 | 5 | A9 | 40 (0) | 90 |
| A9(neo-6)-3 | 6 | A9 | 100 (60) | 0 |
| A9(neo-7)-2 | 7 | A9 | 100 (89) | 0 |
| A9(neo-8)-1 | 8 | A9 | 91 (82) | 0 |
| GM10611 | 9 | CHO | 79 (5) | 11 |
| A9(neo-10)-3 | 10 | A9 | 94 (6) | 75 |
| A9(neo-11)-1 | 11 | A9 | 24 (0) | 76 |
| GM10927A * | 11 | CHO | 96 (21) | 4 |
| A9(neo-12)-4 | 12 | A9 | 0 (0) | 100 |
| GM10868 * | 12 | CHO | 82 (6) | 0 |
| GM10898 | 13 | CHO | 82 (0) | 10 |
| GM10479 | 14 | 3T6 | 76 (29) | 0 |
| A9(neo-15)-2 | 15 | A9 | 9 (0) | 78 |
| GM11418 * | 15 | CHO | 62 (0) | 100 |
| GM10567 | 16 | A9 | 69 (0) | 0 |
| GM10498 | 17 | LTMK | 80 (10) | 0 |
| A9(neo-18)-5 | 18 | A9 | 100 (66) | 0 |
| A9(neo-19)-1 | 19 | A9 | 92 (23) | 8 |
| A9(neo-20)-3 | 20 | A9 | 81 (5) | 17 |
| GM08854 | 21 | A9 | 81 (24) | 0 |
| GM10027 | 22 | CHO | 93 (0) | 100 |
| GM10324 | X | A9 | 81 (10) | 0 |
| GM06317 | Y | CHW1103 | 91 (0) | 9 |

Fig. 16

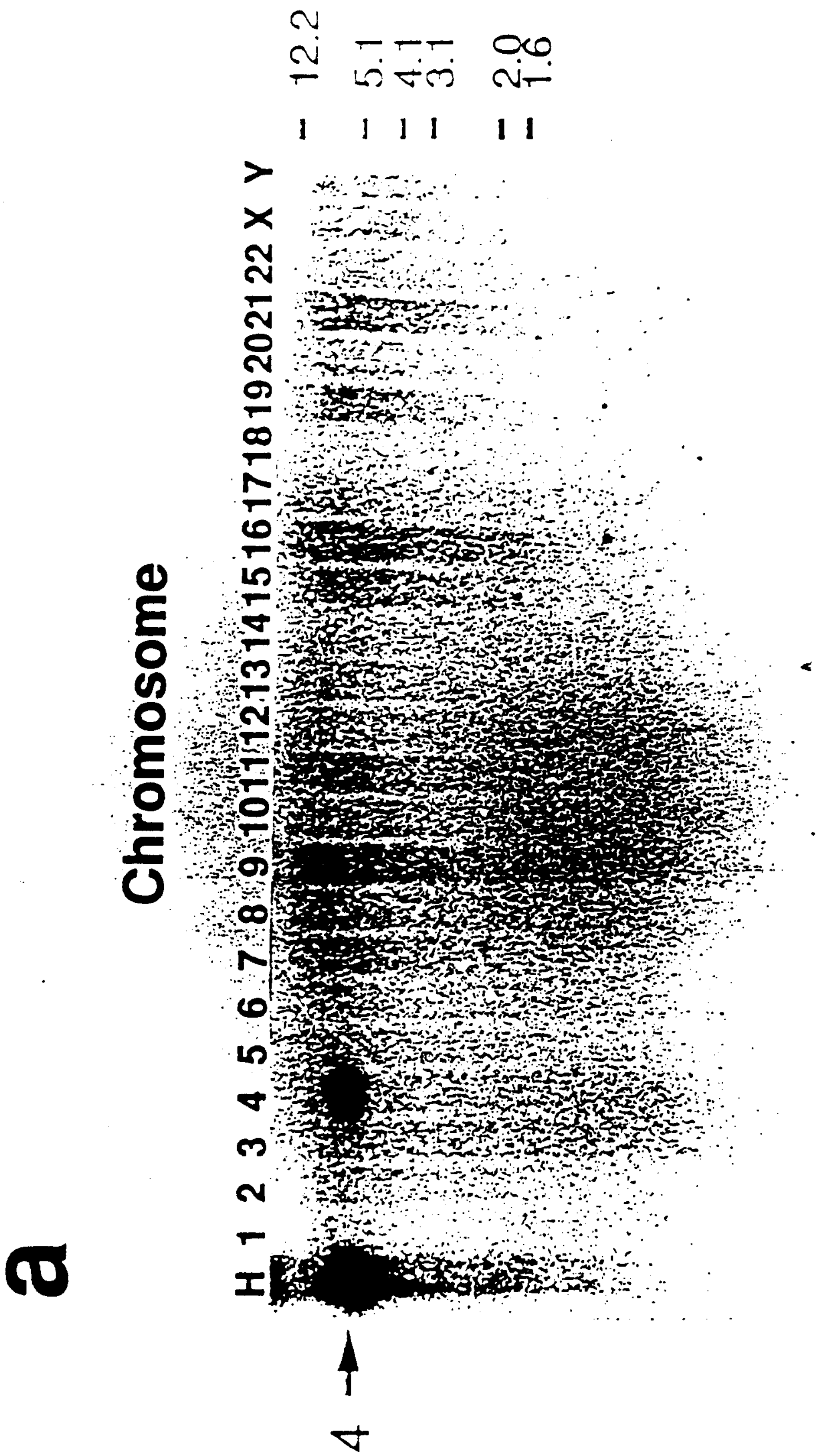
a
Chromosome
H 1 2 3 4 5 6 7 8 9 10 11 12 13 14 15 16 17 18 19 20 21 22 X Y
12.2
5.1
4.1
3.1
2.0
1.6
4

Fig. 17

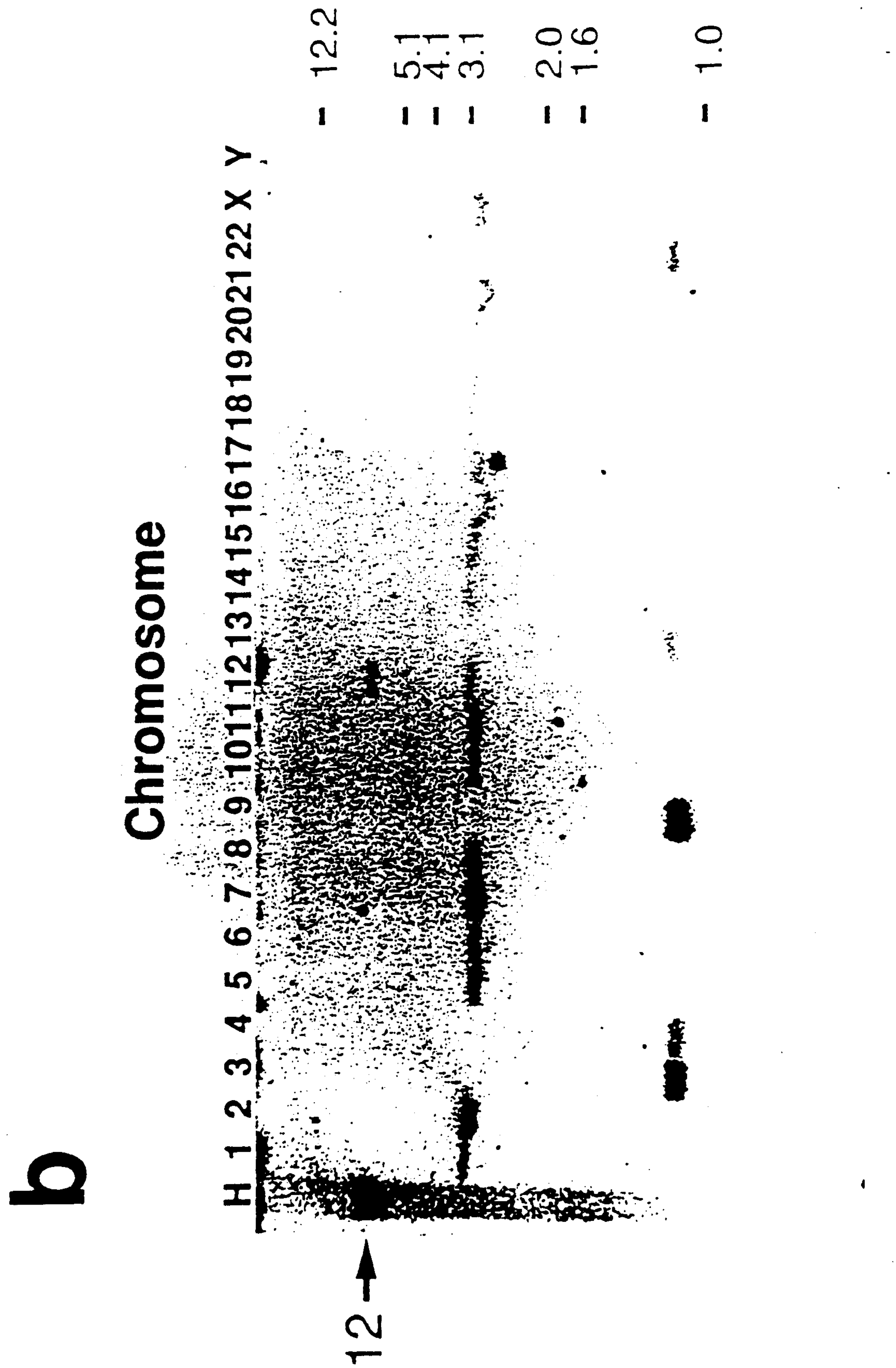
b
Chromosome
H 1 2 3 4 5 6 7 8 9 10 11 12 13 14 15 16 17 18 19 20 21 22 X Y
12.2
5.1
4.1
3.1
2.0
1.6
1.0
12

Fig. 18

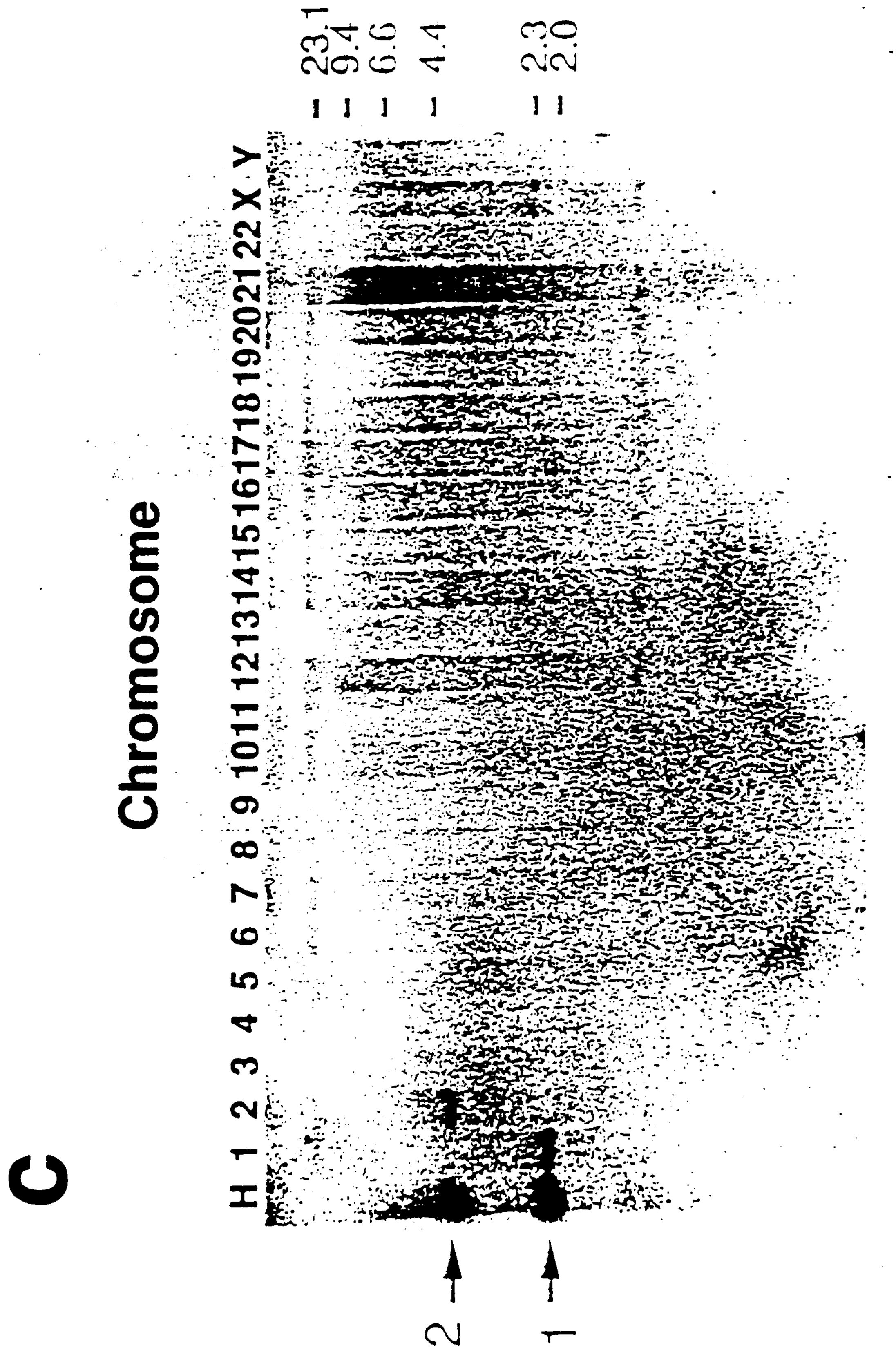
C
Chromosome
H 1 2 3 4 5 6 7 8 9 10 11 12 13 14 15 16 17 18 19 20 21 22 X Y
23.1
9.4
6.6
4.4
2.3
2.0
2
1

Fig. 19

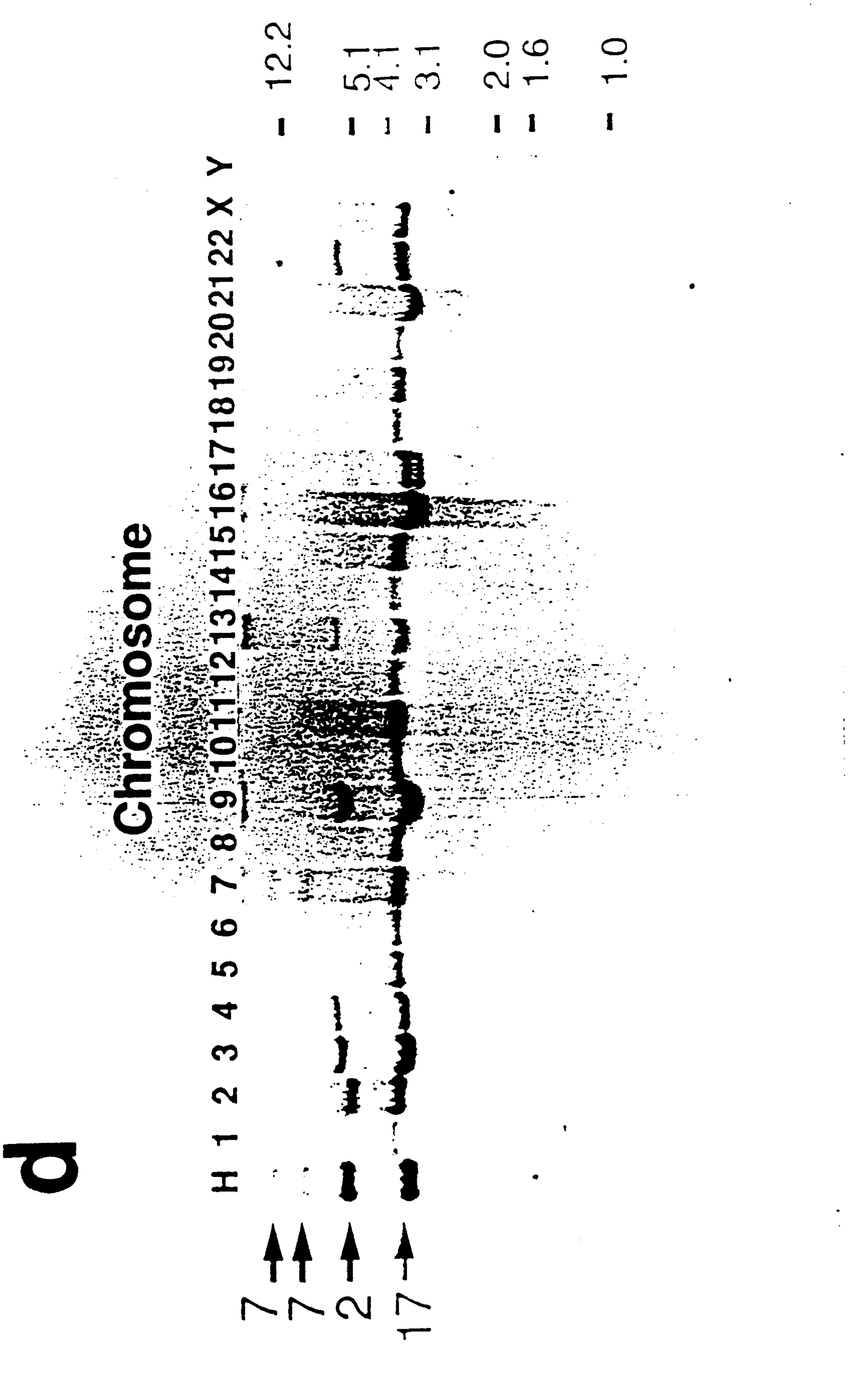
d
Chromosome
H 1 2 3 4 5 6 7 8 9 10 11 12 13 14 15 16 17 18 19 20 21 22 X Y
12.2
5.1
4.1
3.1
2.0
1.6
1.0
7
7
2
17

Fig. 20

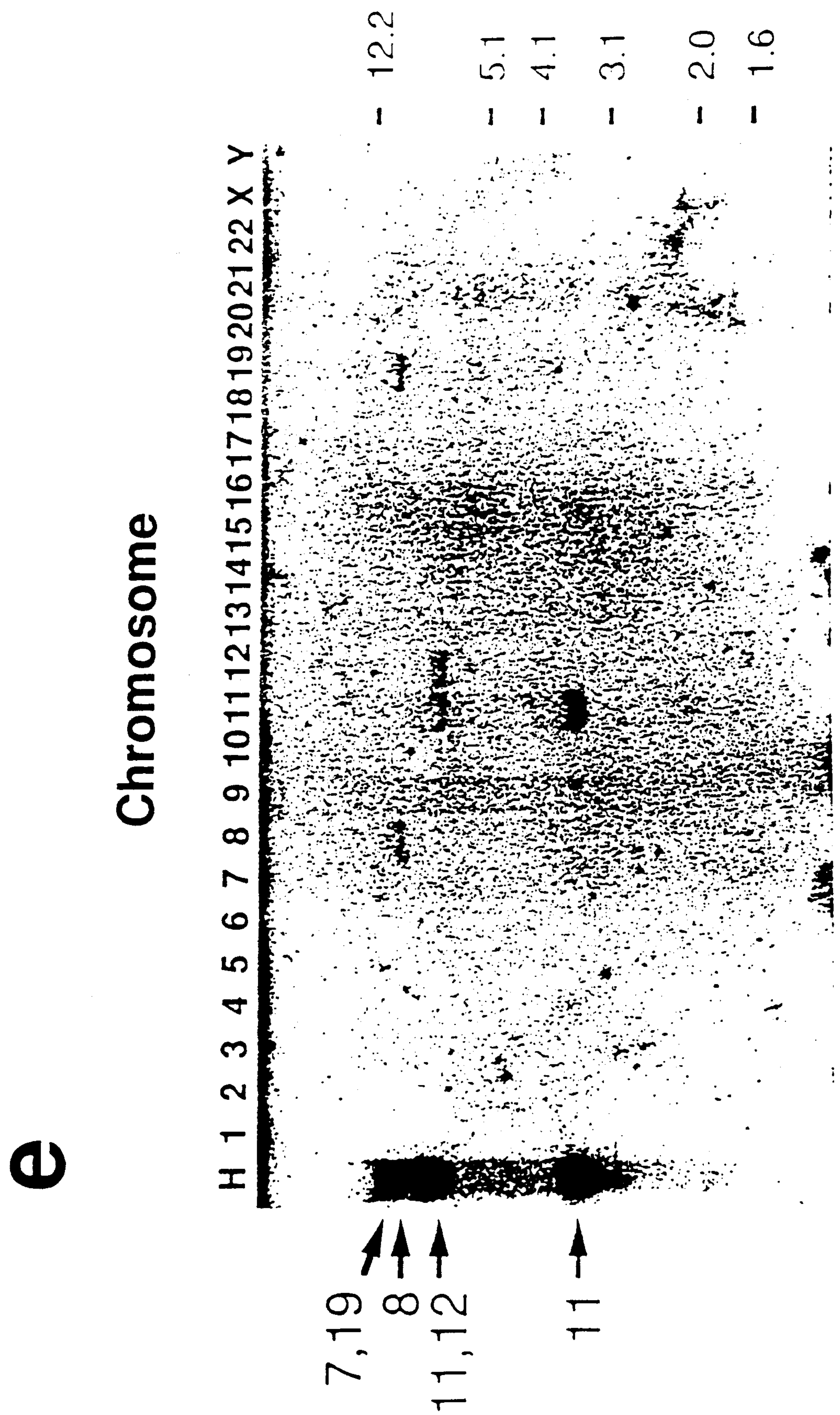
e
Chromosome
H 1 2 3 4 5 6 7 8 9 10 11 12 13 14 15 16 17 18 19 20 21 22 X Y
12.2
5.1
4.1
3.1
2.0
1.6
7,19
8
11,12
11

Fig. 21

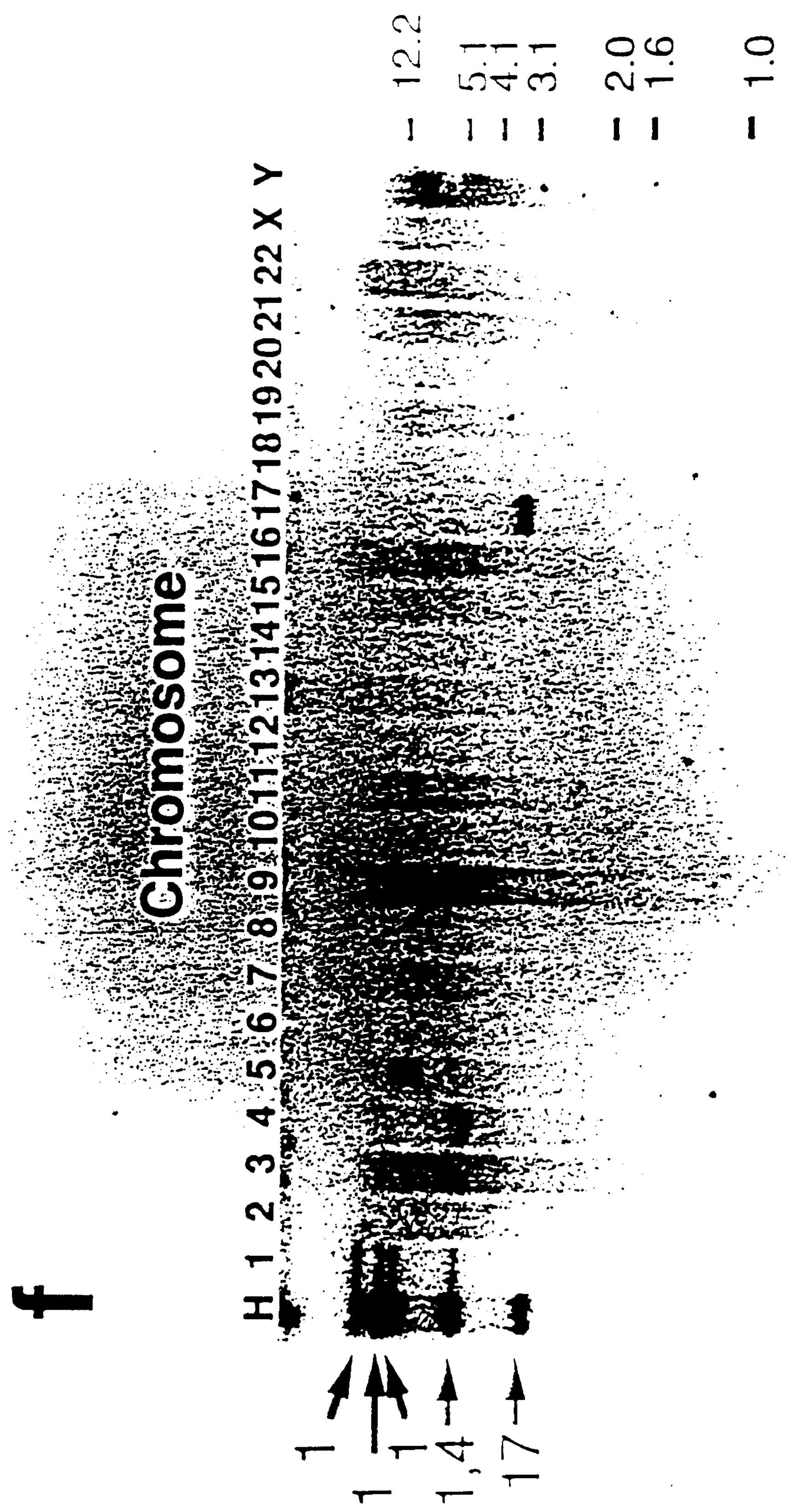
f
Chromosome
H 1 2 3 4 5 6 7 8 9 10 11 12 13 14 15 16 17 18 19 20 21 22 X Y
12.2
5.1
4.1
3.1
2.0
1.6
1.0
1
1
1
1,4
17

Fig. 22

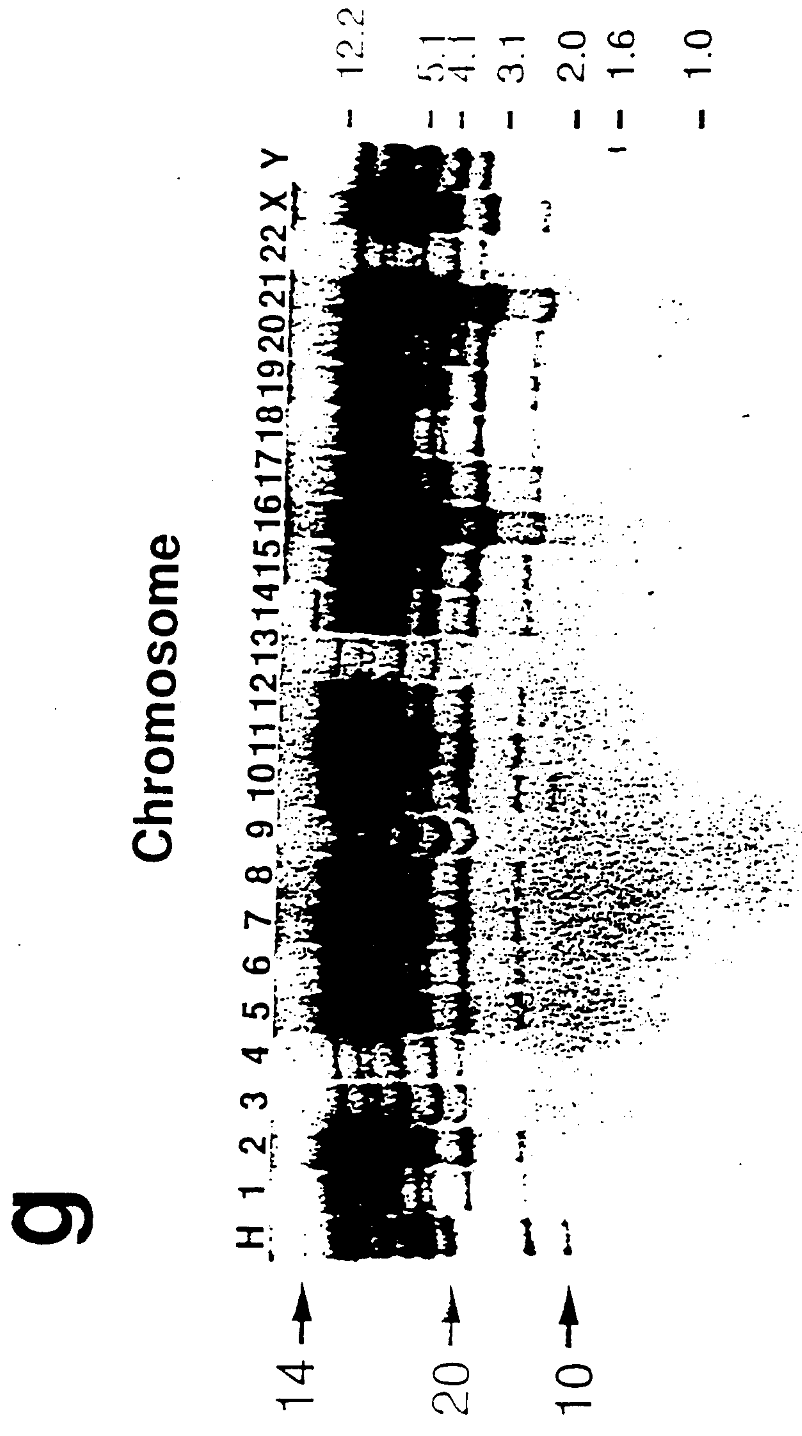
g
Chromosome
H 1 2 3 4 5 6 7 8 9 10 11 12 13 14 15 16 17 18 19 20 21 22 X Y
14
20
10
12.2
5.1
4.1
3.1
2.0
1.6
1.0

Fig. 23

Chromosomal mapping of each GS by Southern blot technique

| Numbers of bands detected with human whole chromosomes | | | | | | | Chromosomes assigned | Background | |
|---|---|---|---|---|---|---|---|---|---|
| Clone | | Sequence length | E | E/B1 | E/B3 | B1/B3 | | Mouse | Chinese hamster |
| Single band group | | | | | | | | | |
| c12c11 | GS000075 | 432 | 1 | 1 | 1 | 1 | 9 | 0 | 0 |
| c12c06 | GS000062 | 540 | 1 | 1 | 1 | 1 | 6,15 | 0 | 0 |
| c12g01 | GS000280 | 212 | 1 | 1 | 1 | 1 | 2 | 1 | 1 |
| c13c05 | GS000117 | 359 | 1 | 1 | 1 | 1 | 11- | 0 | 0 |
| c13c07 | GS000120 | 355 | 1 | 1 | 1 | 1 | 2 | 0 | 0 |
| c13f10 | GS000206 | 267 | 1 | 1 | 1 | 1 | 14 | 0 | 0 |
| c13h01 | GS000279 | 133 | 1 | 1 | 1 | 1 | 12- | 0 | 0 |
| c13h02 | GS000322 | 167 | 1 | 1 | 1 | 1 | 6 | 0 | 0 |
| d0g02 | GS000095 | 397 | 1 | 1 | 1 | 1 | 3 | 0 | 0 |
| d0h07 | GS000164 | 313 | 1 | 1 | 1 | 1 | 11 | 1 | 1 |
| d1b10 | GS000348 | 153 | 1 | 1 | 1 | 1 | 20 | 0 | 0 |
| hm01a12 | GS000223 | 246 | 1 | 1 | 1 | 1 | Y? | 0 | 0 |
| hm01c09 | GS000423 | 157 | 0 | 1 | 1 | 1 | 1 | 0 | 0 |
| hm01c12 | junk | 394 | 1 | 1 | 1 | 1 | 17 | 0 | 0 |
| hm01f05 | GS000066 | 454 | 1 | 1 | 1 | 1 | 19,22 | 0 | 0 |
| hm01f10 | GS000299 | 173 | 0 | 1 | 1 | 1 | 10 | 0 | 0 |
| hm01g09 | GS000053 | 477 | 1 | 1 | 1 | 1 | 6 | 0 | 0 |
| hm01h07 | GS000115 | 363 | 1 | 1 | 1 | 1 | 12 | 0 | 0 |
| hm02a02 | GS000130 | 344 | 1 | 1 | 1 | 1 | 4 | 0 | 0 |
| hm02a04 | GS000329 | 164 | 1 | 1 | 0 | 0 | 10 | 0 | 0 |
| hm02c01 | GS000203 | 271 | 1 | 1 | 1 | 1 | 15 | 0 | 0 |
| hm02c01 | GS000016 | 590 | 1 | 1 | 1 | 1 | 20 | 0 | 0 |
| hm02e02 | GS000342 | 156 | 0 | 1 | 1 | 1 | 14 | 0 | 0 |
| hm02e05 | GS000401 | 223 | 1 | 1 | 0 | 0 | n.d. | 0 | 0 |
| hm02g02 | GS000191 | 278 | 1 | 1 | 1 | 1 | 17 | 0 | 0 |
| hm05a05 | GS000251 | 219 | 1 | 1 | 1 | 1 | 6 | 2 | 0 |
| hm05a10 | junk | 392 | 1 | 1 | 1 | 1 | 1 | 1 | 1 |
| hm05c10 | GS000009 | 606 | 1 | 1 | 1 | 1 | 1 | 0 | 0 |
| kmd01 | junk | 169 | 1 | 1 | 1 | 0 | n.d. | 0 | 0 |
| s105 | GS000001 | 703 | 1 | 1 | 1 | 1 | 5 | 0 | 0 |
| s110 | GS000057 | 471 | 1 | 1 | 1 | 1 | 8 | 0 | 0 |
| s11d11 | GS000307 | #175 | 0 | 0 | 0 | 1 | 7 | 0 | 0 |
| s11h01 | GS000263 | 204 | 1 | 1 | 1 | 1 | 3 | 0 | 0 |
| s147 | GS000060 | 461 | 1 | 1 | 1 | 0 | 2 | 0 | 0 |
| s14e06 | junk | 639 | 1 | 1 | 1 | 1 | 1 | 0 | 0 |
| s14g02 | GS000152 | 322 | 1 | 1 | 1 | 1 | 4 | 0 | 0 |
| s14h12 | GS000271 | 193 | 1 | 1 | 1 | 1 | 4 | 1 | 1 |
| s150 | GS000143 | 330 | 1 | 1 | 1 | 1 | 17 | 0 | 0 |
| s156 | GS000002 | 306 | 1 | 1 | 1 | 1 | 2 | 1 | 1 |
| s15b11 | GS000250 | 221 | 1 | 1 | 1 | 1 | 14 | 0 | 0 |
| s179 | GS000275 | 196 | 1 | 1 | 1 | 1 | n.d. | 0 | 0 |
| s246 | GS000234 | 241 | 1 | 1 | 1 | 1 | 9 | 0 | 0 |
| s247 | GS000347 | 153 | 1 | 1 | 1 | 1 | 1 | 0 | 0 |
| s270 | junk | 135 | 1 | 1 | 1 | 1 | 19 | 0 | 0 |

Fig. 24

| Numbers of bands detected with human whole chromosomes | | | | | | | Chromosomes assigned | Background | |
|---|---|---|---|---|---|---|---|---|---|
| Clone | | Sequence length | E | E/Ba | E/Bg | Ba/Bg | | Mouse | Chinese hamster |
| s306 | CS000256 | 205 | 1 | 1 | 0 | 1 | X | 0 | 0 |
| s309 | CS000171 | 305 | 1 | 1 | 0 | 1 | 1 | 0 | 0 |
| s342 | CS000323 | 165 | 1 | 1 | 1 | 1 | 4 | 3 | 2 |
| s331 | CS000265 | 207 | 1 | 1 | 0 | 1 | 6,15 | 1 | 1 |
| s334 | CS000165 | 312 | 1 | 1 | 1 | 1 | 1 | 0 | 0 |
| s337 | CS000276 | 195 | 1 | 1 | 1 | 1 | 17 | 0 | 0 |
| s339 | CS000295 | 130 | 1 | 1 | 1 | 1 | n.d. | 0 | 1 |
| s443 | CS000330 | 251 | 1 | 1 | 1 | 1 | n.d. | 0 | 0 |
| s470 | junk | 261 | 1 | 1 | 1 | 1 | 17 | 0 | 0 |
| s474 | CS000192 | 273 | 1 | 1 | 1 | 1 | 5 | 0 | 0 |
| s503 | junk | 312 | 1 | 1 | 1 | 1 | 12 | 0 | 0 |
| s507 | junk | 600 | 1 | 1 | 1 | 1 | 1 | 2 | 1 |
| s517 | CS000334 | 161 | 1 | 1 | 1 | 1 | 14 | 1 | 1 |
| s632 | junk | 587 | 1 | 1 | 1 | 1 | 2 | 0 | 0 |
| s633 | CS000166 | 311 | 1 | 1 | 1 | 1 | 22 | 2 | 1 |
| s650 | CS000041 | 644 | 1 | 1 | 1 | 1 | 12 | 1 | 1 |
| tw1-04 | CS000025 | 537 | 1 | 1 | 1 | 1 | 3,7 | 0 | 0 |
| tw1-19 | CS000213 | 255 | 1 | 1 | 1 | 1 | 17 | 0 | 0 |
| tw1-32 | junk | 250 | 1 | 1 | 1 | 1 | 5 | 0 | 0 |
| tw1-37 | CS000237 | 235 | 1 | 1 | 1 | 1 | 22 | 0 | 0 |
| tw1-42 | junk | 391 | 1 | 1 | 1 | 1 | 8 | 1 | 1 |
| tw1-43 | CS000098 | 178 | 1 | 1 | 1 | 1 | 14 | 0 | 0 |
| tw1-96 | CS000133 | 339 | 1 | 1 | 1 | 1 | 11 | 0 | 0 |
| Two band group : | | | | | | | | | |
| c12f12 | CS000195 | 277 | 1 | 2 | 2 | 2 | 1, | 1 | 1 |
| c13d02 | CS000042 | 503 | 2 | 2 | 1 | 1 | 2, | 0 | 0 |
| hm01a06 | CS000129 | 344 | 2 | 2 | 2 | 2 | 11,18 | 3 | 5 |
| hm01a07 | CS000207 | 269 | 2 | 2 | 2 | 2 | 7, | 0 | 0 |
| hm01d05 | CS000232 | 243 | 2 | 2 | 2 | 1 | 2, | 0 | 0 |
| hm01e01 | CS000181 | 292 | 2 | 2 | 2 | 2 | 1,2 | 0 | 0 |
| hm02a08 | CS000435 | 302 | 2 | 2 | 2 | 2 | 3, | 1 | 1 |
| hm02c04 | CS000221 | 253 | 2 | 2 | 2 | 2 | 3, | 0 | 0 |
| hm02c05 | CS000146 | 332 | 2 | 2 | 2 | 2 | 17,19,22 | 0 | 0 |
| hm05f07 | CS000043 | 503 | 1 | 1 | 2 | 1 | 3, | 0 | 0 |
| s11d06 | CS000268 | 205 | 2 | 2 | 2 | 2 | 11,12 | 0 | 0 |
| s11g12 | CS000337 | 255 | 2 | 2 | 2 | 2 | 6, | 0 | 0 |
| s124 | CS000083 | 404 | 2 | 2 | 2 | 2 | 9, | 1 | 1 |
| s144 | CS000132 | 342 | 1 | 2 | 2 | 2 | 1,7 | 0 | 0 |
| s14f03 | CS000239 | 243 | 1 | 2 | 2 | 2 | 2, | 3 | 2 |
| s15c02 | junk | 439 | 2 | 2 | 1 | 2 | 6, | 0 | 0 |
| s16b09 | junk | 420 | 1 | 1 | 1 | 2 | 10,14 | 0 | 0 |
| s17c09 | CS000248 | 223 | 2 | 2 | 2 | 2 | 14, | 0 | 0 |
| s231 | junk | 284 | 2 | 2 | 2 | 2 | 11, | 0 | 0 |
| s254 | CS000124 | 353 | 2 | 2 | 2 | 2 | 1, | 3 | 1 |
| s255 | CS000235 | 239 | 2 | 2 | 2 | 2 | 11, | 0 | 0 |
| s272 | junk | 195 | 2 | 2 | 2 | 2 | 10,16 | 1 | 1 |

Fig. 25

| Numbers of bands detected with human whole chromosomes | | | | | | | Chromosomes assigned | Background | |
|---|---|---|---|---|---|---|---|---|---|
| Clone | | Sequence length | E | E/Ba | E/Bg | Ba/Bg | | Mouse | Chinese hamster |
| s311 | GS000092 | 333 | 1 | 1 | 2 | 2 | 16, | 1 | 1 |
| s313 | junk | 132 | 2 | 2 | 1 | 0 | 20, | 0 | 0 |
| s317 | GS000100 | 339 | 0 | 0 | 1 | 2 | 14,14 | 1 | 1 |
| s336 | GS000134 | 337 | 2 | 2 | 2 | 2 | 12,14 | 0 | 0 |
| s338 | GS000139 | 233 | 2 | 2 | 2 | 1 | 22,X | 0 | 0 |
| s339 | GS000233 | 137 | 2 | 1 | 1 | 2 | 17, | 0 | 0 |
| s394 | GS000063 | 449 | 2 | 1 | 2 | 2 | 13,14 | 0 | 0 |
| s396 | junk | 277 | 2 | 2 | 2 | 2 | 17, | 0 | 1 |
| s455 | junk | 452 | 1 | 2 | 2 | 1 | 4, | 0 | 0 |
| s456 | GS000236 | 132 | 2 | 2 | 2 | 2 | 8,10 | 1 | 2 |
| s465 | GS000201 | 274 | 1 | 1 | 2 | 2 | 6,15 | 0 | 0 |
| s635 | junk | 250 | 1 | 1 | 1 | 2 | 9,13 | 0 | 0 |
| s639 | GS000297 | 205 | 1 | 2 | 2 | 2 | 2,X | 0 | 0 |
| s656 | GS000025 | #590 | 2 | 2 | 0 | 2 | 6,11 | 0 | 0 |
| tw1-33 | junk | 352 | 2 | 2 | 2 | 2 | 1, | 0 | 0 |
| tw1-39 | GS000153 | #321 | 2 | 2 | 2 | 2 | 17, | 0 | 0 |
| tw1-70 | GS000061 | 441 | 1 | 1 | 2 | 1 | 11, | 0 | 0 |
| tw1-80 | junk | 453 | 2 | 2 | 1 | 2 | 9,17 | 2 | 2 |
| tw1-87 | GS000158 | 316 | 2 | 2 | 2 | 2 | 7, | 0 | 0 |
| Three band group | | | | | | | | | |
| d0h06 | GS000080 | 417 | 3 | 3 | 3 | 1 | 1, | 0 | 0 |
| hm05b07 | junk | 336 | 2 | 3 | 3 | 3 | 5, | 0 | 0. |
| hm05g02 | GS000209 | 267 | 2 | 2 | 2 | 1 | 3,17,19 | 1 | 1 |
| s129 | GS000107 | 378 | 3 | 3 | 3 | 3 | n.d. | 1 | 1 |
| s178 | GS000357 | 146 | 1 | 2 | 2 | 3 | 2, | 0 | 0 |
| s17a10 | GS000294 | 131 | 3 | 3 | 3 | 3 | 2,13,22 | 1 | 1 |
| s308 | GS000412 | 638 | 2 | 2 | 2 | 3 | XX | 1 | 1 |
| s401 | GS000224 | 249 | 2 | 3 | 3 | 3 | 6,6, | 0 | 0 |
| s654 | GS000045 | 491 | 3 | 3 | 3 | 3 | 1,22, | 0 | 0 |
| tw1-82 | GS000208 | 267 | 3 | 3 | 3 | 3 | 13, | 4 | 0 |
| Four band group | | | | | | | | | |
| c12g07 | GS000154 | 320 | 4 | 4 | 2 | 3 | 5, 14, | 0 | 0 |
| c13a08 | GS000055 | 508 | 3 | 3 | 4 | 4 | 2,7,7,17 | 1 | 2 |
| c13c04 | GS000106 | #376 | 4 | 3 | 3 | 3 | n.d. | 0 | 2 |
| c13e09 | GS000302 | 195 | 4 | 2 | 4 | 4 | 2,17, | 7 | 2 |
| s136 | GS000160 | 315 | 4 | 4 | 4 | 4 | 4,X, | 2 | 1 |
| s163 | GS000004 | #613 | 4 | 4 | 4 | 2 | 4,4,8,20 | 3 | 1 |
| s479 | GS000130 | 293 | 4 | 4 | 2 | 2 | 7,8,11,11,12,19 | 0 | 0 |
| Group with 5 or more bands | | | | | | | | | |
| c12f08 | GS000253 | 217 | 5 | 5 | 5 | 2 | 2,7,9,14, | 2 | 0 |
| hc01 | junk | 374 | 12 | 12 | 15 | 13 | 1,2,6, | 22 | 20 |
| hd10 | junk | 361 | 4 | 4 | 4 | 8 | n.d. | 12 | 6 |
| he10 | junk | 173 | 6 | 2 | 3 | 3 | 6,8,9,19,21, | 3 | 3 |
| hm01c05 | GS000305 | 176 | 9 | 7 | 5 | 5 | X | 9 | 8 |
| hm01f04 | GS000246 | 215 | 8 | 10 | 5 | 5 | n.d. | 12 | 12 |
| hm01g02 | junk | 411 | 9 | 6 | 6 | 4 | 10,14,20, | 14 | 6 |

Fig. 26

| Numbers of bands detected with human whole chromosomes | | | | | | | Chromosomes assigned | Background | |
|---|---|---|---|---|---|---|---|---|---|
| Clone | | Sequence length | E | E/Ba | E/Bg | Ba/Bg | | Mouse | Chinese hamster |
| hm02f09 | GS000273 | 442 | 8 | 7 | 7 | 5 | 3,3,6,11,13,14,15,16 | 0 | 0 |
| hm05a02 | GS000096 | 373 | 5 | 6 | 4 | 6 | 2,3,17, | 3 | 3 |
| hm05a04 | GS000236 | #239 | 6 | 6 | 6 | 7 | n.d. | 8 | 5 |
| kmb01 | junk | 350 | 3 | 5 | 5 | 5 | 13, | 14 | 7 |
| s11f06 | GS000316 | 170 | 6 | 6 | 6 | 4 | 1,2,2,3,4,6,13,15, | 0 | 3 |
| s14f01 | GS000407 | 262 | 12 | 11 | 10 | 9 | 1,6,9,13, | 6 | 3 |
| s173 | GS000094 | 397 | 5 | 4 | 6 | 8 | 1,1,1,1,4,17 | 0 | 0 |
| s265 | GS000323 | 167 | 10 | 12 | 11 | 14 | 18, | 9 | 5 |
| s341 | junk | 494 | 9 | 9 | 8 | 6 | n.d. | 15 | 3 |
| s406 | GS000113 | 364 | 6 | 7 | 5 | 4 | 2,7,8,13,20,20 | 4 | 1 |
| tw1-46 | junk | 593 | 9 | 10 | 10 | 10 | 1,1,2,2,6,11,X, | 3 | 5 |
| tw1-63 | junk | 203 | 8 | 10 | 10 | 12 | 3,4, | 17 | 11 |
| Bands no detected: | | | | | | | | | |
| c13g02 | GS000340 | 157 | 0 | 0 | 0 | 0 | - | - | - |
| hm01e10 | junk | 232 | 0 | 0 | 0 | 0 | - | - | - |
| hm02d11 | GS000274 | 196 | 0 | 0 | 0 | 0 | - | - | - |
| s323 | GS000273 | 194 | 0 | 0 | 0 | 0 | - | - | - |
| s359 | GS000199 | 279 | 0 | 0 | 0 | 0 | - | - | - |
| s511 | junk | 233 | 0 | 0 | 0 | 0 | - | - | - |
| s645 | GS000012 | #734 | 0 | 0 | 0 | 0 | - | - | - |
| s647 | GS000105 | 360 | 0 | 0 | 0 | 0 | - | - | - |
| s651 | junk | 540 | 0 | 0 | 0 | 0 | - | - | - |

## INTERNATIONAL SEARCH REPORT

International application No.
PCT/JP94/01916

**A. CLASSIFICATION OF SUBJECT MATTER**

Int. $Cl^6$ C12N15/11, C12Q1/68//G01N33/566

According to International Patent Classification (IPC) or to both national classification and IPC

**B. FIELDS SEARCHED**

Minimum documentation searched (classification system followed by classification symbols)

Int. $Cl^6$ C12N15/11, C12Q1/68//G01N33/566

Documentation searched other than minimum documentation to the extent that such documents are included in the fields searched

Electronic data base consulted during the international search (name of data base and, where practicable, search terms used)

BIOSIS PREVIEWS, CAS ONLINE

**C. DOCUMENTS CONSIDERED TO BE RELEVANT**

| Category* | Citation of document, with indication, where appropriate, of the relevant passages | Relevant to claim No. |
|---|---|---|
| X | Nucleic Acids. Res., Vol. 15, 1987, Ou, J. H. "Cloning and characterization of a human ribosomal protein gene with enhanced expression in fetal and neoplastic cells" p. 8919-8934 | 1-6 (164) |
| X | Differntiatios, Vol. 33, 1986, Oshima, R. G. et al. "Comparison of mouse and human keratin 18:A component of intermediate filaments expressed prior to implantation" p. 61-68 | 1-6 (226) |
| X | J. Biol. Chem., Vol. 265, 1990, Wilkin, D. J. et al. "Isolation and sequence of the human farnesyl pyrophosphate synthetase cDNA:coordinate regulation of the mRNAs for farnesyl pyrophosphate synthetase, 3-hydroxy-3-methylglutaryl coenzyme A reductase, and 3-hydroxy-3-methylglutaryl coenzyme A synthetase" p. 4607-4614 | 1-6 (255) |

[X] Further documents are listed in the continuation of Box C. [ ] See patent family annex.

* Special categories of cited documents:

"A" document defining the general state of the art which is not considered to be of particular relevance

"E" earlier document but published on or after the international filing date

"L" document which may throw doubts on priority claim(s) or which is cited to establish the publication date of another citation or other special reason (as specified)

"O" document referring to an oral disclosure, use, exhibition or other means

"P" document published prior to the international filing date but later than the priority date claimed

"T" later document published after the international filing date or priority date and not in conflict with the application but cited to understand the principle or theory underlying the invention

"X" document of particular relevance; the claimed invention cannot be considered novel or cannot be considered to involve an inventive step when the document is taken alone

"Y" document of particular relevance; the claimed invention cannot be considered to involve an inventive step when the document is combined with one or more other such documents, such combination being obvious to a person skilled in the art

"&" document member of the same patent family

| Date of the actual completion of the international search | Date of mailing of the international search report |
|---|---|
| February 6, 1995 (06. 02. 95) | March 7, 1995 (07. 03. 95) |
| Name and mailing address of the ISA/ Japanese Patent Office | Authorized officer |
| Facsimile No. | Telephone No. |

**INTERNATIONAL SEARCH REPORT**

International application No. PCT/JP94/01916

C (Continuation). DOCUMENTS CONSIDERED TO BE RELEVANT

| Category* | Citation of document, with indication, where appropriate, of the relevant passages | Relevant to claim No. |
|---|---|---|
| X | J. Biol. Chem., Vol. 266, 1991, Batra, S. K. et al. "Molecular cloning and sequence analysis of the human ribosomal protein S16" p. 6830-6833 | 1-6 (275) |
| X | Proc. Natl. Acad. Sci. U.S.A., Vol. 87, 1990, Ben-Ishai, R. et al. "A human cellular-sequence implicated in trk oncogene activation is DNA damage inducible" p. 6039-6043 | 1-6 (313) |
| X | J. Biol. Chem., Vol. 263, 1988, Fischer, R. et al. "Multiple divergent mRNAs code for a single human calmodulin" p. 17055-17062 | 1-6 (386) |
| X | J. Cell Biol., Vol. 108, 1989, Barnett, T. R. et al. "Carcinoembryonic antigens: Alternative splicing accounts for the multiple mRNAs that code for novel members of the carcinoembryonic antigen family" p. 267-276 | 1-6 (446) |
| X | J. Biol. Chem., Vol. 265, 1990, Natsumeda, Y. et al. "Two distinct cDNAs for human IMP dehydrogenase" p. 5292-5295 | 1-6 (454) |
| X | Genes Dev., Vol. 7, 1993, Patton, J. G. et al. "Cloning and characterization of PSF a novel pre-mRNA splicing factor" p. 393-406 | 1-6 (706) |
| X | Nucleic Acids Res., Vol. 16, 1988, Stanford, D. R. et al. "The complete primary structure of the human snRNP E protein" p. 10593-10605 | 1-6 (711) |
| X | Proc. Natl. Acad. Sci. U.S.A., Vol. 84, 1987, Inoue, C. et al. "Evolutionary conservation of the insulinoma gene rig and its possible function" p. 6659-6662 | 1-6 (723) |
| X | J. Immunol., Vol. 144, 1990, Jongstra-Bilen, J. et al. "Human and mouse LSP1 genes code for highly conserved phosphoproteins" p. 1104-1110 | 1-6 (741) |
| X | Biochem. J., Vol. 248, 1987, Sakai, I. et al. "The cDNA and protein sequences of human lactate dehydrogenase-B" p. 933-936 | 1-6 (772) |

**INTERNATIONAL SEARCH REPORT**

International application No.
PCT/JP94/01916

C (Continuation). DOCUMENTS CONSIDERED TO BE RELEVANT

| Category* | Citation of document, with indication, where appropriate, of the relevant passages | Relevant to claim No. |
|---|---|---|
| X | Biochim. Bioiphys. Acta., Vol. 1089, 1991, Tamura, T. et al. "Molecular cloning and sequence analysis of cDNAs for five major subunits of human proteasomes (multicatalytic proteinase complexes)" p. 95-102 | 1-6 (775) |
| X | Mol. Cell. Biol., Vol. 3, 1983, Cowan, N. J. et al. "Expression of human alpha-tubulin genes: interspecies conservation of 3' untranslated regions" p. 1738-1745 | 1-6 (820) |
| X | Nucleic Acids Res., Vol. 17, 1989, Taaman, J. W. et al. "Nucleotide sequence of cDNA encoding subunit VIb of human cytochrome c oxidase" p. 1766-1766 | 1-6 (844) |
| X | Gene, Vol. 93, 1990 Taanman, J. W., Schrage, C., Ponne, N., Das, A., Bolhuis, P. A., de Vries, H. and Agsteribbe, E. Isolation of cDNAs encoding Subunit VIb of human cytochrome c oxidase and steady-state levels of coxVIb mRNA in di different tissues p. 285-291 | 1-6 (844) |
| X | J. Biol. Chem., Vol. 264, 1989, Gray, P. W. et al. "Cloning of the cDNA of a human neutrophil bactericidal protein:Structural and functional correlations" p. 9505-9509 | 1-6 (861) |
| X | Immunogenetics, Vol. 32, 1990, Angelisova, P. et al. "The human leucocyte surface antigen CD53 is a protein structurally similar to the CD37 and MRC OX-44 antigens" p. 281-285 | 1-6 (1158) |
| X | Proc. Natl. Acad. Sci. U.S.A., Vol. 88, 1991, Koken, M. H. et al. "Structural and functional conservation of two human homologs of the yeast DNA repair gene RAD6" p. 8865-8869 | 1-6 (1181) |
| X | Oncogene, Vol. 5, 1990, Firmbach-Kraft, I. et al. "Tyk 2, prototype of a novel class of non-receptor tyrosine Kinase genes" p. 1329-1336 | 1-6 (1345) |
| X | Science, Vol. 248, 1990, Smith, C. A. et al. "A receptor for human tumor necrosis factor difines an unusual family of cellular and viral proteins" p. 1019-1023 | 1-6 (1431) |

**INTERNATIONAL SEARCH REPORT**

International application No.
PCT/JP94/01916

C (Continuation). DOCUMENTS CONSIDERED TO BE RELEVANT

| Category* | Citation of document, with indication, where appropriate, of the relevant passages | Relevant to claim No. |
|---|---|---|
| X | J. Biol. Chem., Vol. 263, 1988, Luster, A. D. et al. "Molecular and biochemical characterization of a novel gamma-interferon-inducible protein" p. 12036-12043 | 1-6 (1455) |
| X | J. Clin. Invest., Vol. 83, 1989, Look, A. T. et al. "The human myeloid plasma membrane glycoprotein CD13 (gp150) is identical to aminopeptidase N" p. 1299-1307 | 1-6 (1469) |
| X | J. Cell Biol., Vol. 105, 1987, Argraves, W. S. et al. "Amino Acid Sequence of the Human Fibronectin Receptor" p. 1183-1190 | 1-6 (1607) |
| X | Nucleic Acids Res., Vol. 18, 1990, Liebhaber, S. A. et al. "Characterization of a human cDNA encoding a widely expressed and highly conserved cysteine-rich protein with an unusual zinc-finger motif" p. 3871-3879 | 1-6 (1642) |
| X | J. Biol. Chem., Vol. 264, 1989, Didsbury, J. et al. "Rac, a novel ras-related family of proteins that are bolulinum toxin substrates" p. 16378-16382 | 1-6 (1709) |
| X | EMBO J., Vol. 6, 1987, Willison, K. et al. "The human homologue of the mouse t-complex gene, TCP1, is located on chromosome 6 but is not near the HLA region" p. 1967-1974 | 1-6 (1749) |
| X | J. Biol. Chem., Vol. 266, 1991, Wu, Y. et al. "Activation of globin gene expression by cDNAs from induced K562 cells: Evidence for involvement of ferritin in globin gene expression" p. 17566-17572 | 1-6 (1785) |
| X | Proc. Natl. Acad. Sci. U.S.A., Vol. 83, 1986, Ikuta, T. et al "Three human alcohol dehydrogenase subunits: cDNA structure and molecular and evolutionary divergence" p. 634-638 | 1-6 (1864) |
| X | Proc. Natl. Acad. Sci. U.S.A., Vol. 85, 1988, Fukumoto, H. et al "Sequence, tissue distribution, and chromosomal localization of mRNA encoding a human glucose transporter-like protein" p. 5434-5438 | 1-6 (1878) |

**INTERNATIONAL SEARCH REPORT**

International application No.
PCT/JP94/01916

C (Continuation). DOCUMENTS CONSIDERED TO BE RELEVANT

| Category* | Citation of document, with indication, where appropriate, of the relevant passages | Relevant to claim No. |
|---|---|---|
| X | J. Clin. Invest., Vol. 76, 1985, Cooke, N.E. et al. "Serum vitamin D-binding protein is a third member of the albumin and alpha fetoprotein gene family" p. 2420-2424 | 1-6 (1888) |
| X | J. Biol. Chem., Vol. 264, 1989, Huang, S.-H. et al. "Human deoxycytidine kinase: Sequence of cDNA clones and analysis of expression in cell lines with and without enzyme activity" p. 14762-14768 | 1-6 (1894) |
| X | J. Biol. Chem., Vol. 266, 1991, Huang, S.-H. et al. "Additions and corrections Human deoxytidine kinase. Sequence of cDNA clones and analysis of expression in cell lines with and without anzyme activity" p. 5353-5353 | 1-6 (1894) |
| X | Somat. Cell Mol. Genet., Vol. 11, 1985, Bell, G.I. et al. "Human alpha-2-macroglobulin gene is located on chromosome 12" p. 285-289 | 1-6 (1895) |
| X | Proc. Natl. Acad. Sci. U.S.A., Vol. 81, 1984, Yang, F. et al. "Human transferrin: cDNA characterization and chromosomal localization" p. 2752-2756 | 1-6 (1902) |
| X | Proc. Natl. Acad. Sci. U.S.A., Vol. 83, 1986, Ny, T. et al. "Cloning and sequence of a cDNA coding for the human beta-migrating endothelial-cell-type plasminogen activator inhibitor" p. 6776-6780 | 1-6 (1904) |
| X | J. Biol. Chem., Vol. 267, 1992, Bausch-Jurken, M. T. et al "Molecular cloning of AMP deaminase isoform L: Sequence and bacterial expression of human AMPD2 cDNA" p. 22407-22413 | 1-6 (1908) |
| X | Gene, Vol. 44, 1986, Board, P. G. et al. "Molecular cloning and nucleotide sequence of human alpha-1 acid glycoprotein cDNA" p. 127-131 | 1-6 (1921) |
| X | Eur. J. Biochem., Vol. 155, 1986, Wathelet, M. et al. "Molecular cloning, full-length sequence and preliminary characterization of a 56-kDa protein induced by human interferons" p. 11-17 | 1-6 (2101) |

C (Continuation). DOCUMENTS CONSIDERED TO BE RELEVANT

| Category* | Citation of document, with indication, where appropriate, of the relevant passages | Relevant to claim No. |
|---|---|---|
| X | Nucleic Acids Res., Vol. 11, 1983, Chebath, J. et al. "Interferon induced 56,000 mr protein and its mRNA in human cells: molecular cloning and partial sequence of the cDNA" p. 1213-1226 | 1-6 (2101) |
| X | Biochemistry, Vol. 25, 1986, Koide, T. et al. "Amino acid sequence of human histidine-rich glycoprotein derived from the nucleotide sequence of its cDNA" p. 2220-2225 | 1-6 (2174) |
| X | Biochemistry, Vol. 22, 1983, Friezner-Degen, S. J. et al. "Characterization of the complementary deoxyribonucleic acid and gene coding for human prothrombin" p. 2087-2097 | 1-6 (2214) |
| X | Biochem. J., Vol. 268, 1990, Steinkasserer, A. et al. "Heterogeneity in human serum amyloid A protein. Five different variants from one individual demonstrated by cDNA sequence analysis." p. 287-193 | 1-6 (2238) |
| X | Nucleic Acids Res., Vol. 17, 1989, Fabrizi, G. M. et al. "Sequence of a cDNA specifying subunit VIIa of human cytochrome c oxidase" p. 7107-7107 | 1-6 (2264) |
| X | Proc. Natl. Acad. Sci. U.S.A., Vol. 86, 1989, Sims, J. E. et al. "Cloning of the interleukin 1 receptor from human T cells" p. 8946-8950 | 1-6 (2265) |
| X | Eur. J. Biochem., Vol. 169, 1987, Mackinnon, C. M. et al. "Molecular cloning of cDNA for human complement component Cls. The complete amino acid sequence" p. 547-553 | 1-6 (2266) |
| X | J. Virol., Vol. 65, 1990, Tsujimoto, A. et al. "Isolation of cDNA for DNA binding proteins which specifically bind to TAX-responsive enhancer element in the LTR of HTLA-1" p. 1420-1426 | 1-6 (2475) |
| X | Immunogenetics, Vol. 37, 1993, Emi, N. et al. "Isolation of a novel cDNA clone showing marked similarity to ME491/CD63 superfamily" p. 193-198 | 1-6 (2556) |
| X | Nature, Vol. 353, 1991, Kelly, A. P. et al. "A new human HLA class II-related locus, DM" p. 571-573 | 1-6 (2583) |

C (Continuation). DOCUMENTS CONSIDERED TO BE RELEVANT

| Category* | Citation of document, with indication, where appropriate, of the relevant passages | Relevant to claim No. |
|---|---|---|
| X | J. Biol. Chem., Vol. 265, 1990, Hla, T. et al. "An abundant transcript induced in differentiating human endothelial cells encodes a polypeptide with structural similarities to G-protein-coupled receptors" p. 9308-9313 | 1-6 (2600) |
| X | J. Biol. Chem., Vol. 267, 1992, White, R. T. et al. "Human adipsin is identical to complement factor D and expressed at high levels in adipose tissue" p. 9210-9213 | 1-6 (2802) |
| X | Proc. Natl. Acad. Sci. U.S.A., Vol. 87, 1990, Rouault, T. A. et al. "Cloning of the cDNA encoding RNA regulatory protein-the human iron-responsive element-binding protein" p. 7958-7962 | 1-6 (2832) |
| X | Nucleic Acids Res., Vol. 17, 1989, Sawada, R. et al. "Complementary DNA sequence and deduced peptide sequence for CD59/MEM43 antigen, the human homologue of murine lymphocyte antigen Ly-6c" p. 6728-6728 | 1-6 (2954) |
| X | DNA Cell Biol., Vol. 9, 1990, Sawada, R. et al. "Isolation and expression of the full-length cDNA encoding CD59 antigen of human lymphocytes" p. 213-220 | 1-6 (2954) |
| X | Proc. Natl. Acad. Sci. U.S.A., Vol. 87, 1990, Weller, P. A. et al. "Complete sequence of human vinculin and assignment of the gene to chromosome 10" p. 5667-5671 | 1-6 (2983) |
| X | Cell, Vol. 58, 1989, Mellentin, J. D. et al. "LYL-1, a novel gene involved by chromosomal translocation in T-cell leukemia, codes for a protein with a helix-loop-helix DNA binding motif" p. 77-83 | 1-6 (3023) |
| X | Cell, Vol. 60, 1990, Uze, G. et al. "Genetic transfer of a functional human interferon alpha receptor into mouse cells: Cloning and expression of its cDNA" p. 225-234 | 1-6 (3041) |
| X | Biochem. Biophys. Res. Commun., Vol. 179, 1991, Xiao, L. et al. "Characterization of a full length cDNA which codes for the human spermidine/spermine N-1-acetyltransferase" p. 407-415 | 1-6 (3053) |

**INTERNATIONAL SEARCH REPORT**

International application No.
PCT/JP94/01916

C (Continuation). DOCUMENTS CONSIDERED TO BE RELEVANT

| Category* | Citation of document, with indication, where appropriate, of the relevant passages | Relevant to claim No. |
|---|---|---|
| X | J. Biol. Chem., Vol. 266, 1991, Casero, R. A. Jr. et al. "Isolation and characterization of a cDNA clone that codes for human spermidine/spermine N-1-acetyltransferase" p. 810-814 | 1-6 (3053) |
| X | Nucleic Acids Res., Vol. 20, 1992, Wintzerith, M. et al. "Sequence of the human RNA polymerase II largest subunit" p. 910-910 | 1-6 (3083) |
| X | J. Cell Biol., Vol. 103, 1986, Lawler, J. et al. "The Structure of Human Thrombospondin, an/ adhesive Glycoprotein with Multiple Calcium binding Sites and Homologies with Several Different Proteins" p. 1635-1648 | 1-6 (3266) |
| X | Nature, Vol. 352, 1991, Maslen, C. L. et al. "Partial sequence of a candidate gene for the marfan syndrome" p. 334-337 | 1-6 (3334) |
| X | J. Cell Biol., Vol. 111, 1990, Fishman, G. I. et al. "Molecular Characterization and Functional Expression of the Human Cardiac Gap Junction Channel" p. 589-598 | 1-6 (3403) |
| X | Cell, Vol. 40, 1985, Ebina, Y. et al. "The human insulin receptor cDNA: The structural basis for hormone-activated membrane signalling" p. 747-758 | 1-6 (3447) |
| X | Oncogne, Vol. 5, 1990, Westin, E. H. et al. "Alternative splicing of the human c-myb gene" p. 1117-1124 | 1-6 (3529) |
| X | Genomics, Vol. 4, 1989, Todd, S. et al. "cDNA sequence, interspecies comparison and gene mapping analysis of argininosuccinate lyase" p. 53-59 | 1-6 (3575) |
| X | FEBS Lett., Vol. 207, 1986, Codina, J. et al. "-Subunits of the human liver Gs/Gi signal-transducing proteins and those of bovine retinal rod cell transducin are identical" p. 187-192 | 1-6 (3796) |
| X | Nucleic Acids Res., Vol. 18, 1990, Roessler, B. J. et al. "Cloning of two distinct copies of human phosphoribosyl pyrophosphate synthetase cDNA" p. 193-193 | 1-6 (3828) |

**INTERNATIONAL SEARCH REPORT**

International application No.
PCT/JP94/01916

C (Continuation). DOCUMENTS CONSIDERED TO BE RELEVANT

| Category* | Citation of document, with indication, where appropriate, of the relevant passages | Relevant to claim No. |
|---|---|---|
| X | J. Biochem., Vol. 109, 1991, Sonoda, T. et. al. "Complete nucleotide sequence of human phosphoribosyl pyrophosphate synthetase subunit I (PRS I) cDNA and a comparison with human and rat PRPS gene families" p. 361-364 | 1-6 (3828) |
| X | J. Biol. Chem., Vol. 263, 1988, Wermuth, B. et al. "Human carbonyl reductase: Nucleotide sequence analysis of a cDNA and amino acid sequence of the encoded protein" p. 16185-16188 | 1-6 (4033) |
| X | Biochim. Biophys. Acta. Vol. 1048, 1990, Forrest, G. L. et al. "Induction of a human carbonyl reductase gene located on chromosome 21" p. 149-155 | 1-6 (4033) |
| X | Proc. Natl. Acad. Sci. U.S.A., Vol. 88, 1991, Schuetz, T. J. et al. "Isolation of a cDNA for HSF2: Evidence for two heat shock factor genes in humans' p. 6911-6915 | 1-6 (4093) |
| X | Nucleic Acids Res., Vol. 13, 1985, Hallewell, R. A. et al. "Human Cu/Zn superoxide dismutase cDNA: isolation of clones synthesising high levels of active or inactive enzyme from an expression library" p. 2017-2034 | 1-6 (4110) |
| X | Proc. Natl. Acad. Sci. U.S.A., Vol. 80, 1983, Sherman, L. et al. "Nucleotide sequence and expression of human chromosome 21 - encoded superoxide dismutase mRNA" p. 5465-5469 | 1-6 (4110) |
| X | J. Biol. Chem., Vol. 268, 1993, David, V. et al. "Interaction with newly synthesized and retained proteins in the endoplasmic reticulum suggests a chaperone function for human integral membrane protein IP90 (calnexin)" p. 9585-9592 | 1-6 (4373) |
| X | J. Exp. Med., Vol. 172, 1990, Tekamp-Olson, P. et al. "Cloning and Characterization of cDNAs for Murine Macrophage Inflammatory Protein 2 and its Human Homologues" p. 911-919 | 1-6 (4452) |

## INTERNATIONAL SEARCH REPORT

International application No.
PCT/JP94/01916

C (Continuation). DOCUMENTS CONSIDERED TO BE RELEVANT

| Category* | Citation of document, with indication, where appropriate, of the relevant passages | Relevant to claim No. |
|---|---|---|
| X | Biochemistry, Vol. 30, 1991, Tomkinson, B. et al. "Characterization of cDNA for human tripeptidyl peptidase II: The N-terminal part of the enzyme is similar to subtilisin" p. 168-174 | 1-6 (4522) |
| X | J. Biol. Chem., Vol. 263, 1988, Verma, A. K. et al. "Complete primary structure of a human plasma membrane Ca2+ pump" p. 14152-14159 | 1-6 (4673) |
| X | J. Biol. Chem., Vol. 267, 1992, Shechter, I. et al. "Solubilization, purification and characterization of a truncated form of rat hepatic squalene synthetase" p. 8628-8635 | 1-6 (4818) |
| X | J. Biol. Chem., Vol. 267, 1992, Mckenzie, T. L. et al. "Molecular cloning, expression, and characterization of the cDNA for the rat hepataic squalene synthase" p. 21368-21374 | 1-6 (4818) |
| X | Nucleic Acids Res., Vol. 13, 1985, Furutani, Y. et al. "Cloning and characterization of the cDNAs for human and rabbit interleukin-1 precursor" p. 5869-5882 | 1-6 (4872) |
| X | Proc. Natl. Acad. Sci U.S.A., Vol. 89, 1992, Katoh, M. et al . "K-sam gene encodes secreted as well as transmembrane receptor tyrosine kinase" p. 2960-2964 | 1-6 (4914) |
| X | Differentiation, Vol. 42, 1989, Kuruc, N. et al. "Synthesis of cytokeratin 13, a component characteristic of internal stratified epithelia, is not induced in human epidermal tumors" p. 111-123 | 1-6 (5264) |
| X | J. Biol. Chem., Vol. 266, 1991, Kiefer, M. C. et al. "Identification and molecular cloning of two new 30-kDa insulin-like growth factor binding proteins isolated from adult human serum" p. 9043-9049 | 1-6 (5374) |

C (Continuation). DOCUMENTS CONSIDERED TO BE RELEVANT

| Category* | Citation of document, with indication, where appropriate, of the relevant passages | Relevant to claim No. |
|---|---|---|
| X | J. Biol. Chem., Vol. 265, 1990, Opipari, A. W. et al. "The A20 cDNA induced by tumor necrosis factor alpha-encodes a novel type of zinc finger protein" p. 14705-14708 | 1-6 (5427) |
| X | J. Biol. Chem., Vol. 265, 1990, McLean, J. W. et al. "cDNA sequence of the human integrin beta-5 subunit" p. 17126-17131 | 1-6 (5715) |
| X | Cell, Vol. 66, 1991, Ge, H. et al. "primary structure of the human splicing factor ASF reveals similarities with drosophila regulators" p. 373-382 | 1-6 (5860) |
| X | Cancer Res., Vol. 52, 1992, Kondoh, N. et al. "Differential expression of S19 ribosomal protein, laminin binding protein and HLA class I mRNAs associated with colon carcinoma progression and differentiation" p. 791-796 | 1-6 (6439) |
| X | J. Biol. Chem., Vol. 263, 1988, Collart, F. R. et al. "Cloning and sequence analysis of the human and chinese hamster inosine-5' -monophosphate dehydrogenase cDNA" p. 15769-15772 | 1-6 (6471) |
| X | J. Biol. Chem., Vol. 261, 1986, Romeo, P. -H. et al. "Molecular cloning and nucleotide sequence of a complete human uroporphyrinogen decarboxylase cDNA" p. 9825-9831 | 1-6 (6569) |
| X | J. Cell Biol., Vol. 106, 1988, Leube, R. E. et al. "Molecular characterization and expression of the stratification-related cytokeratins 4 and 15" p. 1249-1261 | 1-6 (6875) |
| X | Proc. Natl. Acad. Sci. U.S.A., Vol. 85, 1988, Daher, K. A. et al. "Isolation and characterization of human defensin cDNA clones" p. 7327-7331 | 1-6 (7106) |
| X | J. Exp. Med, Vol. 172, 1990, Larsen, A. et al. "Expression Cloning of a Human Graulocyte Colony-stimulating Factor Receptor: a Structural Mosaic of Hematopoietin Receptor, Immunoglobulin, and Fibronectin Domains" p. 1559-1570 | 1-6 (7126) |

**INTERNATIONAL SEARCH REPORT**

International application No.
PCT/JP94/01916

C (Continuation). DOCUMENTS CONSIDERED TO BE RELEVANT

| Category* | Citation of document, with indication, where appropriate, of the relevant passages | Relevant to claim No. |
|---|---|---|
| X | Oncogene, Vol. 8, 1993, Schulz, A. S. et al. "The genomic structure of the human UFO receptor" p. 509-513 | 1-6 (7790) |
| A | Nature Genetics, Vol. 2, 1992, Okubo, K. et al. "Large scale cDNA sequencing for analysis of quantitative and qualitative aspects of gene expression" p. 173-179 | 1-6 |
| A | Nature Genetics, Vol. 2, 1992, Khan, A. S. et al. "Single pass sequencing and physical and genetic mapping of human brain cDNAs" p. 180-188 | 1-6 |